(19) **Europäisches Patentamt European Patent Office Office européen des brevets**

(11) **EP 4 435 105 A2**

(12) **EUROPEAN PATENT APPLICATION**

(43) Date of publication:
**25.09.2024 Bulletin 2024/39**

(21) Application number: **24173045.6**

(22) Date of filing: **28.09.2016**

(51) International Patent Classification (IPC):
**C12N 15/113** (2010.01)

(52) Cooperative Patent Classification (CPC):
**C07K 16/28; A61K 39/3955; A61P 9/04; A61P 9/10; C07H 21/02; C12N 15/1138;**
C07K 2317/30; C07K 2317/76; C07K 2317/77; C07K 2317/92; C07K 2317/94; C12N 2310/14

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2015 US 201562234546 P**
**24.11.2015 US 201562259553 P**
**07.04.2016 US 201662319740 P**

(62) Document number(s) of the earlier application(s) in accordance with Art. 76 EPC:
**16781931.7 / 3 356 415**

(71) Applicant: **Amgen Inc.**
**Thousand Oaks, California 91320 (US)**

(72) Inventors:
• **NIOI, Paul**
**Wellesley, 02482 (US)**
• **COWARD, Peter**
**San Francisco, 94118 (US)**
• **MURAWSKY, Christopher**
**Roberts Creek, V0N 2W0 (CA)**
• **PIPER, Derek, E.**
**Santa Clara, 95051 (US)**

• **GARCES, Fernando**
**San Mateo, 94401 (US)**
• **ZHANG, Jun**
**Foster City, 94404 (US)**
• **LI, Yang**
**Mountain View, 94040 (US)**
• **CHAN, Brian, Mingtung**
**Port Coquitlam, V3C 5N8 (CA)**

(74) Representative: **Eder, Michael et al**
**Df-mp Dörries Frank-Molnia & Pohlman**
**Patentanwälte Rechtsanwälte PartG mbB**
**Fünf Höfe**
**Theatinerstrasse 16**
**80333 München (DE)**

Remarks:
•The complete document including Reference Table(s) and the Sequence Listing(s) can be downloaded from the EPO website
•This application was filed on 29-04-2024 as a divisional application to the application mentioned under INID code 62.
•Claims filed after the date of filing of the application (Rule 68(4) EPC).

(54) **ASGR INHIBITORS FOR REDUZING CHOLESTEROL LEVELS**

(57) Antigen binding proteins that interact with AS-GR, ASGR-1 and/or ASGR-2 are described as well as methods of making and using such antigen binding proteins. Methods of treating and preventing cardiovascular disease by administering a pharmaceutically effective amount of ASGR, ASGR-1 and/or ASGR-2 antigen binding proteins. Methods of treating and preventing cardiovascular disease by administering a pharmaceutically effective amount of interfering RNA compositions that reduce expression of ASGR, ASGR-1 and/or ASGR-2 are described.

EP 4 435 105 A2

**Description**

CROSS REFERENCE TO RELATED APPLICATIONS

This application items priority to U.S. Provisional Patent Application No. 62/319,740, filed April 7, 2016, U.S. Provisional Patent Application No. 62/259,553, filed November 24, 2015, and U.S. Provisional Patent Application No. 62/234,546, filed September 29, 2015, which are incorporated herein by reference in their entirety.

REFERENCE TO THE SEQUENCE LISTING AND TABLES IN ELECTRONIC FORMAT

This application contains a Sequence Listing which has been submitted electronically in ASCII format and is hereby incorporated by reference in its entirety. Said ASCII copy, created on September 28, 2016, is named APMOL017WOSEQUENCE.txt and is 14,772,812 bytes in size.

FIELD

The field of this invention relates to compositions and methods related to ASGR inhibitors, including but not limited to anti-ASGR, anti-ASGR-1, and/or anti-ASGR-2 antigen binding proteins.

BACKGROUND OF VARIOUS EMBODIMENTS

Cardiovascular disease involving the heart or blood vessels remains a leading cause of global mortality. Cardiovascular disease includes coronary artery disease (CAD) which can lead to angina and myocardial infarction (MI), stroke, hypertensive heart disease, rheumatic heart disease, and other disorders of the cardiovascular system. Medicines for treating cardiovascular disease, and in particular coronary artery disease, have been introduced over the years (e.g., the small molecule class of drugs called statins and the recently approved Repatha®, an antibody targeting PCSK9).

SUMMARY OF VARIOUS EMBODIMENTS

In some aspects, the invention provides an isolated antigen binding protein that binds to human ASGR and inhibits ASGR function. In one embodiment, the invention comprises an isolated antigen binding protein that binds to human ASGR and inhibits ASGR binding to ligand. In another embodiment, the invention comprises an isolated antigen binding protein that binds to human ASGR-1 and inhibits ASGR-1 binding to ligand and/or ASGR-1 interaction with ASGR-2. In another embodiment, the invention comprises an isolated antigen binding protein that binds to human ASGR-2 and inhibits ASGR-2 binding to ligand and/or ASGR-2 interaction with ASGR-1. In yet another embodiment, the invention comprises an isolated antigen binding protein that binds to human ASGR-1 and human ASGR-2, and inhibits ASGR-1 and/or ASGR-2 binding to ligand. In some embodiments, the isolated binding protein binds specifically to human ASGR, ASGR-1 and/or ASGR-2.

In some aspects, the invention provides an isolated antigen binding protein, wherein the isolated antigen binding protein binds to human ASGR-1 and comprises one or more VH CDR1, VH CDR2 or VH CDR3 having an amino acid sequence identical to or comprising 1, 2, or 3 amino acid residue substitutions, deletions or insertions in each CDR relative to the VH of any of the sequences set forth in Tables 3-7. In some aspects, the invention comprises an isolated antigen binding protein, wherein the isolated antigen binding protein binds to human ASGR-1 and comprises one or more VL CDR1, VL CDR2 or VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises one or more VH CDR1, VH CDR2 or VH CDR3 having an amino acid sequence identical to or comprising 1, 2, or 3 amino acid residue substitutions, deletions or insertions in each CDR relative to the VH of any of the sequences set forth in Tables 3-7, and one or more VL CDR1, VL CDR2 or VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises one VH CDR1, VH CDR2 or VH CDR3 having an amino acid sequence identical to or comprising 1, 2, or 3 amino acid residue substitutions, deletions or insertions in each CDR relative to the VH of any of the sequences set forth in Tables 3-7, and one VL CDR1, VL CDR2 or VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises two VH CDR1, VH CDR2 or VH CDR3 having an amino acid sequence identical to or comprising 1, 2, or 3 amino acid residue substitutions, deletions or insertions in each CDR relative to the VH of any of the sequences set forth in Tables 3-7, and two VL CDR1, VL CDR2 or VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises the VH CDR1, VH CDR2 and VH CDR3 having an amino acid sequence identical to or comprising 1, 2, or 3 amino acid residue substitutions, deletions or insertions in each CDR relative to the VH of any of the sequences set forth in Tables 3-7, and the VL CDR1, VL CDR2 and VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises the VH CDR1, VH CDR2, VH CDR3, VL CDR1, VL CDR2, and VL CDR3 having an amino acid sequence identical to any of the sequences set forth in Tables 3-7. In some embodiments, the isolated antigen binding protein comprises the VH CDR1, VH CDR2 or VH CDR3 having an amino acid sequence identical to or comprising 1, 2, or 3 amino acid residue substitutions, deletions or insertions in each CDR relative to the VH of any of the sequences set forth in TABLE A. In some embodiments, the isolated antigen binding protein comprises the VL CDR1, VL CDR2 or VL CDR3 having an amino acid sequence identical to or comprising 1, 2, or 3 amino acid residue substitutions, deletions or insertions in each CDR relative to the VL of any of the sequences set forth in TABLE A. In some embodiments, the isolated antigen binding protein comprises the VH CDR1, VH CDR2, and VH CDR3 having an amino acid sequence identical to or comprising 1, 2, or 3 amino acid residue substitutions, deletions or insertions in each CDR relative to the VH of any of the sequences set forth in TABLE A, and the VL CDR1, VL CDR2 and VL CDR3, having an amino acid sequence identical to or comprising 1, 2, or 3 amino acid residue substitutions, deletions or insertions in each CDR relative to the VL of any of the sequences set forth in TABLE A. In some embodiments, the isolated antigen binding protein comprises the VH CDR1, VH CDR2, VH CDR3, VL CDR1, VL CDR2, and VL CDR3 having an amino acid sequence identical to any of the sequences set forth in TABLE A. In some embodiments, the isolated antigen binding protein comprises the VH CDR1, VH CDR2 or VH CDR3 having an amino acid sequence identical to or comprising 1, 2, or 3 amino acid residue substitutions, deletions or insertions in each CDR relative to the VH of any of the sequences set forth in TABLE B. In some embodiments, the isolated antigen binding protein comprises the VL CDR1, VL CDR2 or VL CDR3 having an amino acid sequence identical to or comprising 1, 2, or 3 amino acid residue substitutions, deletions or insertions in each CDR relative to the VL of any of the sequences set forth in TABLE B. In some embodiments, the isolated antigen binding protein comprises the VH CDR1, VH CDR2, and VH CDR3 having an amino acid sequence identical to or comprising 1, 2, or 3 amino acid residue substitutions, deletions or insertions in each CDR relative to the VH of any of the sequences set forth in TABLE B, and the VL CDR1, VL CDR2 and VL CDR3 having an amino acid sequence identical to or comprising 1, 2, or 3 amino acid residue substitutions, deletions or insertions in each CDR relative to the VL of any of the sequences set forth in TABLE B. In some embodiments, the isolated antigen binding protein comprises the VH CDR1, VH CDR2, VH CDR3, VL CDR1, VL CDR2, and VL CDR3 having an amino acid sequence identical to any of the sequences set forth in TABLE B. In still some embodiments, the isolated antigen binding protein comprises the VH CDR1, VH CDR2 or VH CDR3 having an amino acid sequence identical to or comprising 1, 2, or 3 amino acid residue substitutions, deletions or insertions in each CDR relative to the VH of any of the sequences set forth in TABLE C. In some embodiments, the isolated antigen binding protein comprises the VL CDR1, VL CDR2 or VL CDR3 having an amino acid sequence identical to or comprising 1, 2, or 3 amino acid residue substitutions, deletions or insertions in each CDR relative to the VL of any of the sequences set forth in TABLE C. In some embodiments, the isolated antigen binding protein comprises the VH CDR1, VH CDR2, and VH CDR3 having an amino acid sequence identical to or comprising 1, 2, or 3 amino acid residue substitutions, deletions or insertions in each CDR relative to the VH of any of the sequences set forth in TABLE C, and the VL CDR1, VL CDR2 and VL CDR3, having an amino acid sequence identical to or comprising 1, 2, or 3 amino acid residue substitutions, deletions or insertions in each CDR relative to the VL of any of the sequences set forth in TABLE C. In some embodiments, the isolated antigen binding protein comprises the VH CDR1, VH CDR2, VH CDR3, VL CDR1, VL CDR2, and VL CDR3 having an amino acid sequence identical to any of the sequences set forth in TABLE C. In further embodiments, the isolated antigen binding protein comprises the VH CDR1, VH CDR2 or VH CDR3 having an amino acid sequence identical to or comprising 1, 2, or 3 amino acid residue substitutions, deletions or insertions in each CDR relative to the VH of any of the sequences set forth in Table 6. In some embodiments, the isolated antigen binding protein comprises the VL CDR1, VL CDR2 or VL CDR3 having an amino acid sequence identical to or comprising 1, 2, or 3 amino acid residue substitutions, deletions or insertions in each CDR relative to the VL of any of the sequences set forth in Table 6. In some embodiments, the isolated antigen binding protein comprises the VH CDR1, VH CDR2, and VH CDR3 having an amino acid sequence identical to or comprising 1, 2, or 3 amino acid residue substitutions, deletions or insertions in each CDR relative to the VH of any of the sequences set forth in Table 6, and the VL CDR1, VL CDR2 and VL CDR3, having an amino acid sequence identical to or comprising 1, 2, or 3 amino acid residue substitutions, deletions or insertions in each CDR relative to the VL of any of the sequences set forth in Table 6. In some embodiments, the isolated antigen binding protein comprises the VH CDR1, VH CDR2, VH CDR3, VL CDR1, VL CDR2, and VL CDR3 having an amino acid sequence identical to any of the sequences set forth in Table 6.

In some aspects, the invention provides an isolated antigen binding protein, wherein the antigen binding protein specifically binds human ASGR-1 and comprises a heavy chain variable domain having at least 90% identity to any of the VH domain amino acid sequences set forth in Tables 3-7. In some aspects, the invention provides an isolated antigen binding protein, wherein the antigen binding protein specifically binds human ASGR-1 and comprises a light chain variable domain having at least 90% identity to any of the VL domain amino acid sequences set forth in Tables 3-7. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Tables 3-7, and a light chain variable domain having at least 90% identity to any of the VL domain amino acid sequences set forth in Tables 3-7. In some embodiments, the invention provides an isolated antigen binding protein, wherein the antigen binding protein specifically binds human ASGR-1 and comprises a heavy chain variable domain having any of the VH domain amino acid sequences set forth in Tables 3-7, and a light chain variable domain having any of the VL domain amino acid sequences set forth in Tables 3-7. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table A. In some embodiments, the invention provides an isolated antigen binding protein, wherein the antigen binding protein specifically binds human ASGR-1 and comprises a light chain variable domain having at least 90% identity to any of the VL domain amino acid sequences set forth in Table A. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table A, and a light chain variable domain having at least 90% identity to any of the VL domain amino acid sequences set forth in Table A. In some embodiments, the invention provides an isolated antigen binding protein, wherein the antigen binding protein specifically binds human ASGR-1 and comprises a heavy chain variable domain having any of the VH domain amino acid sequences set forth in Table A, and a light chain variable domain having any of the VL domain amino acid sequences set forth in Table A. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table B. In some embodiments, the invention provides an isolated antigen binding protein, wherein the antigen binding protein specifically binds human ASGR-1 and comprises a light chain variable domain having at least 90% identity to any of the VL domain amino acid sequences set forth in Table B. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table B, and a light chain variable domain having at least 90% identity to any of the VL domain amino acid sequences set forth in Table B. In some embodiments, the invention provides an isolated antigen binding protein, wherein the antigen binding protein specifically binds human ASGR-1 and comprises a heavy chain variable domain having any of the VH domain amino acid sequences set forth in Table B, and a light chain variable domain having any of the VL domain amino acid sequences set forth in Table B. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table C. In some embodiments, the invention provides an isolated antigen binding protein, wherein the antigen binding protein specifically binds human ASGR-1 and comprises a light chain variable domain having at least 90% identity to any of the VL domain amino acid sequences set forth in Table C. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table C, and a light chain variable domain having at least 90% identity to any of the VL domain amino acid sequences set forth in Table C. In some embodiments, the invention provides an isolated antigen binding protein, wherein the antigen binding protein specifically binds human ASGR-1 and comprises a heavy chain variable domain having any of the VH domain amino acid sequences set forth in Table C, and a light chain variable domain having any of the VL domain amino acid sequences set forth in Table C. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table 6. In some embodiments, the invention provides an isolated antigen binding protein, wherein the antigen binding protein specifically binds human ASGR-1 and comprises a light chain variable domain having at least 90% identity to any of the VL domain amino acid sequences set forth in Table 6. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table 6, and a light chain variable domain having at least 90% identity to any of the VL domain amino acid sequences set forth in Table 6. In some embodiments, the invention provides an isolated antigen binding protein, wherein the antigen binding protein specifically binds human ASGR-1 and comprises a heavy chain variable domain having any of the VH domain amino acid sequences set forth in Table 6, and a light chain variable domain having any of the VL domain amino acid sequences set forth in Table 6.

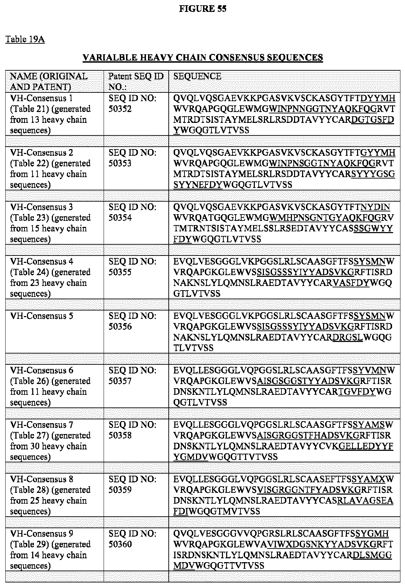

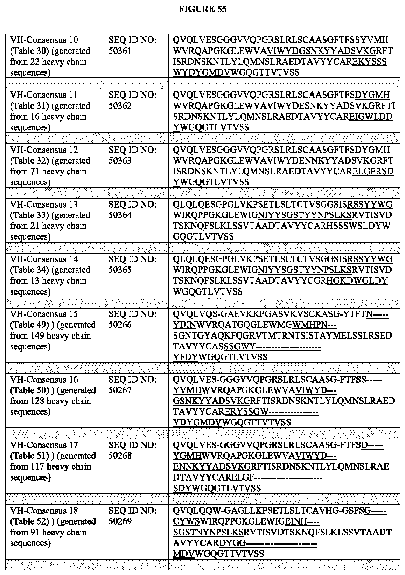

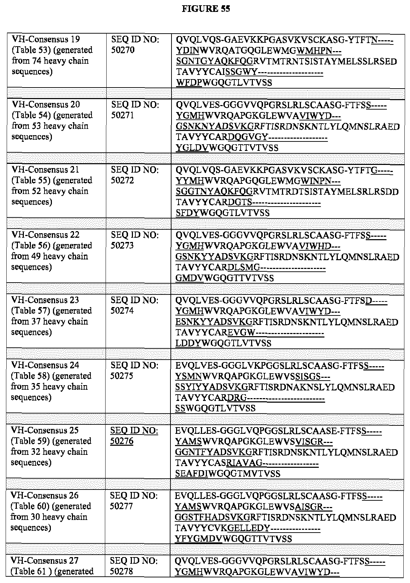

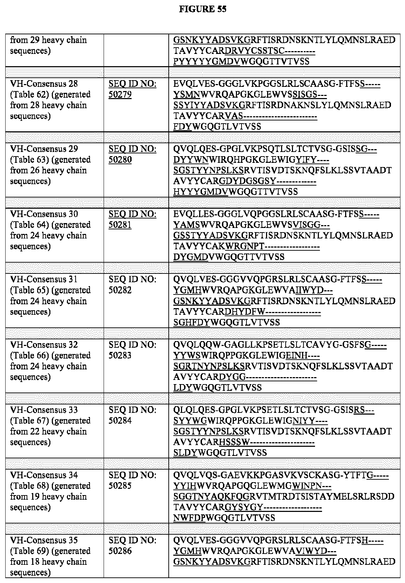

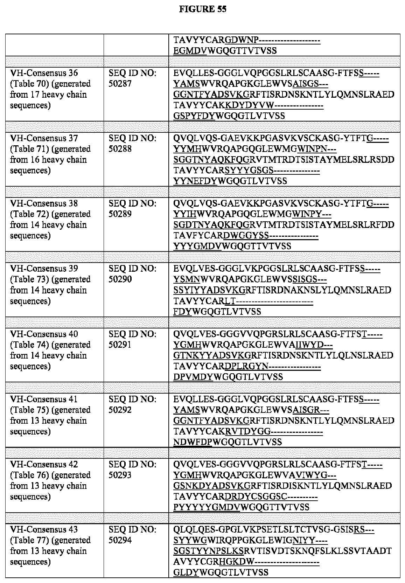

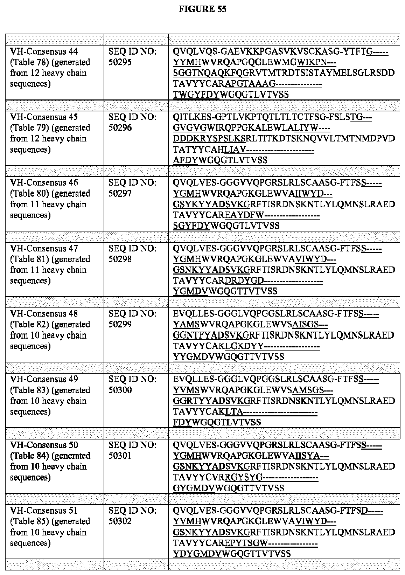

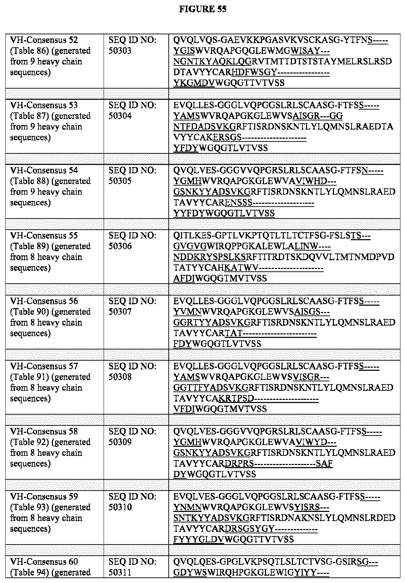

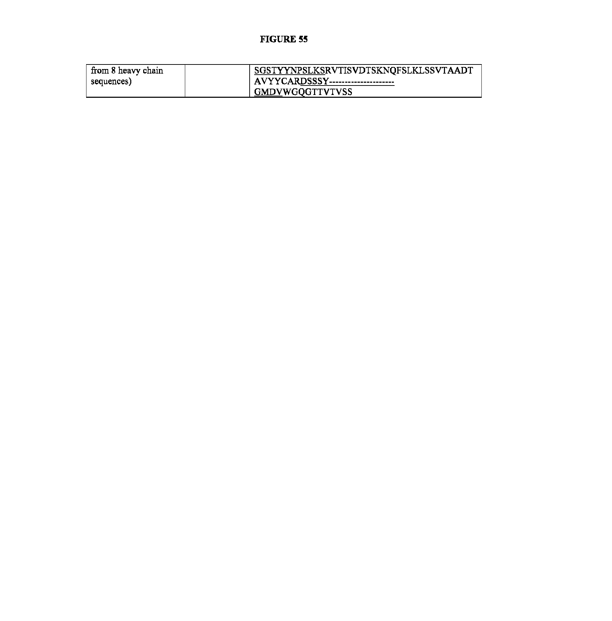

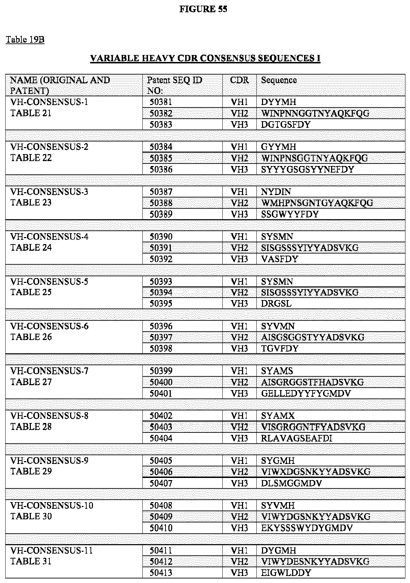

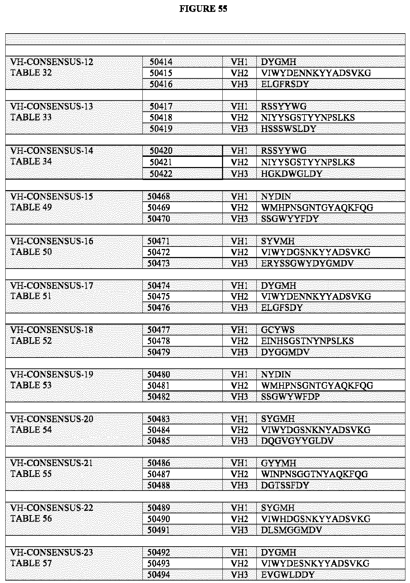

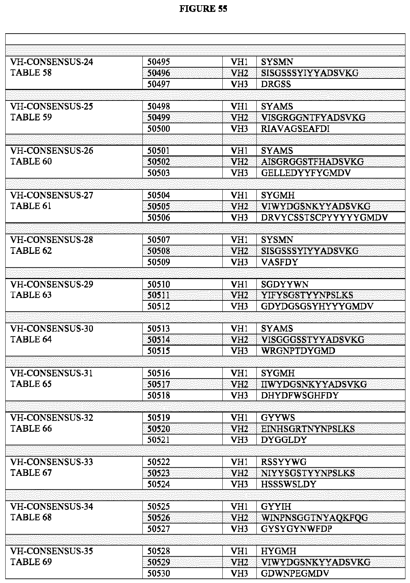

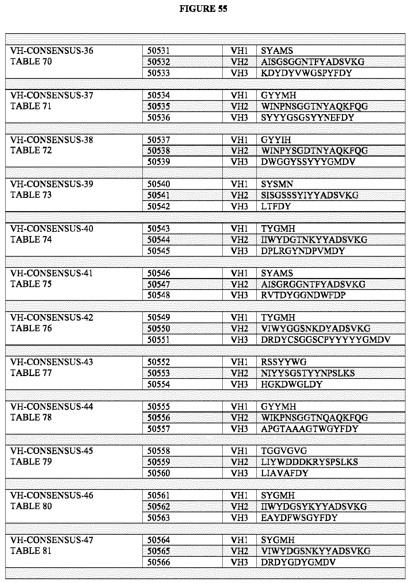

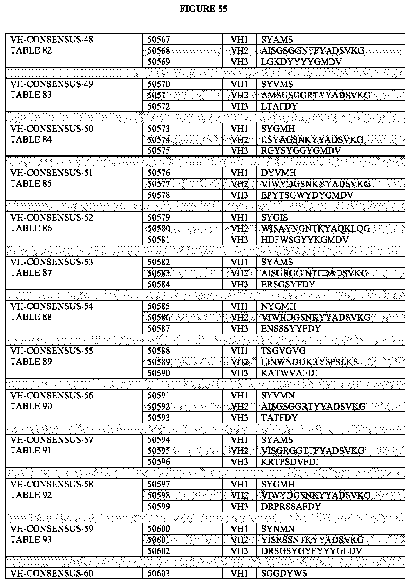

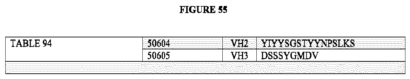

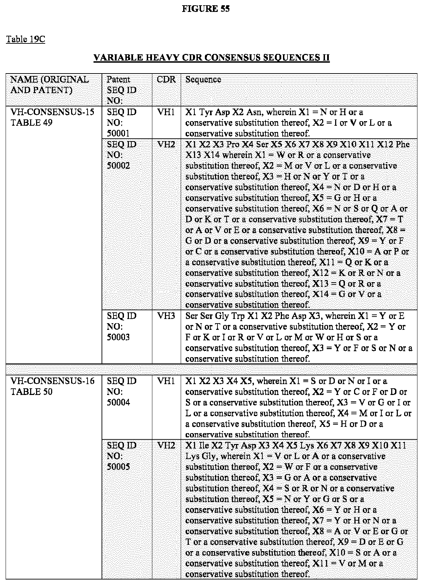

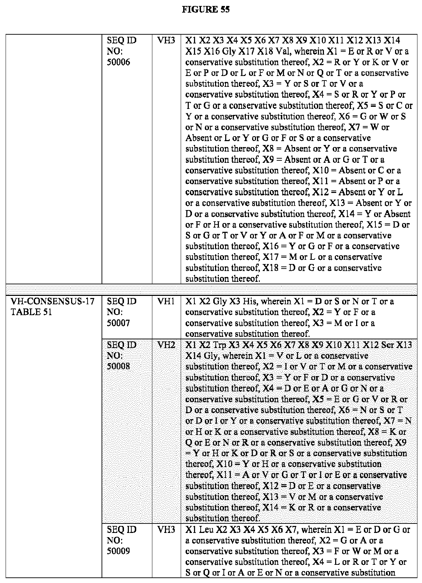

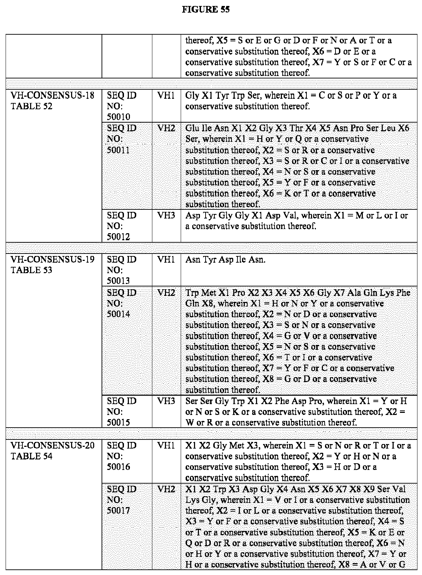

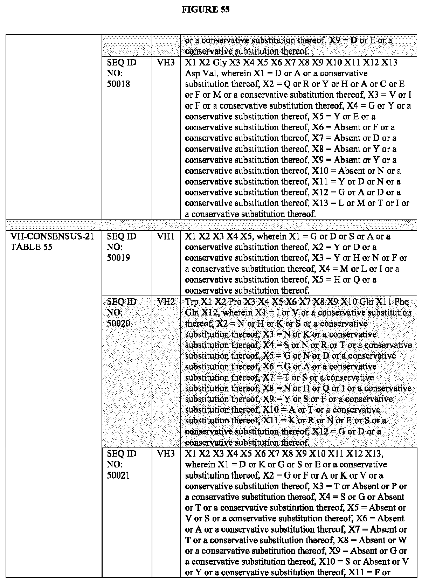

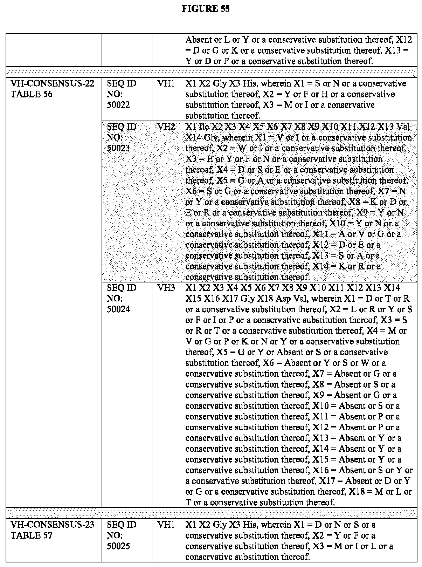

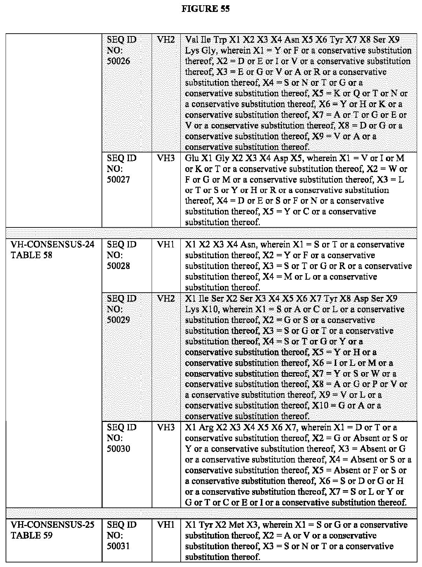

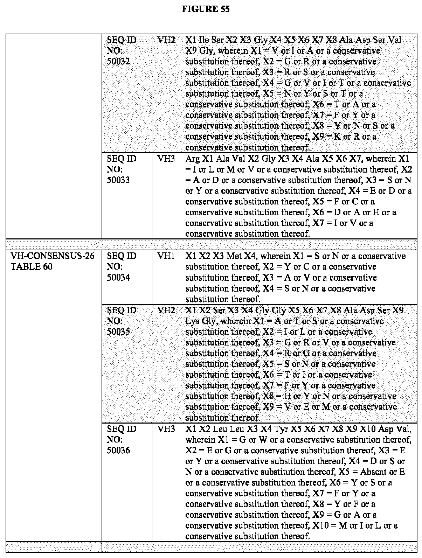

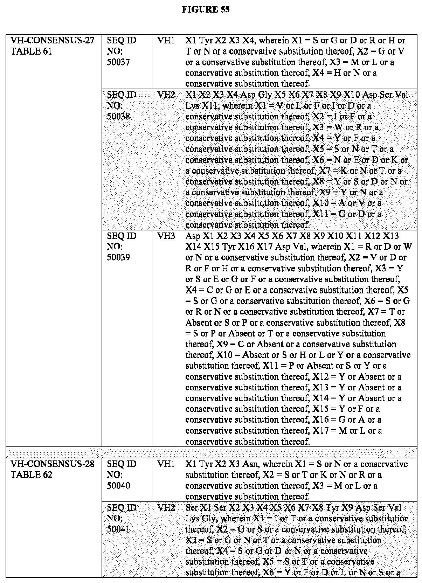

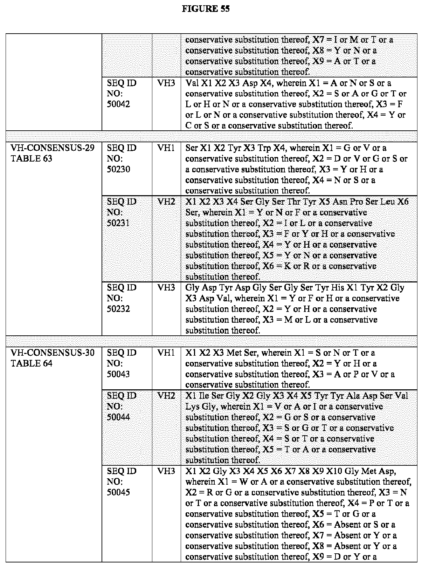

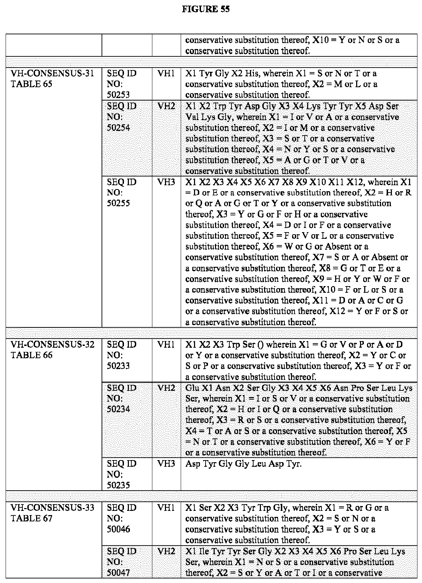

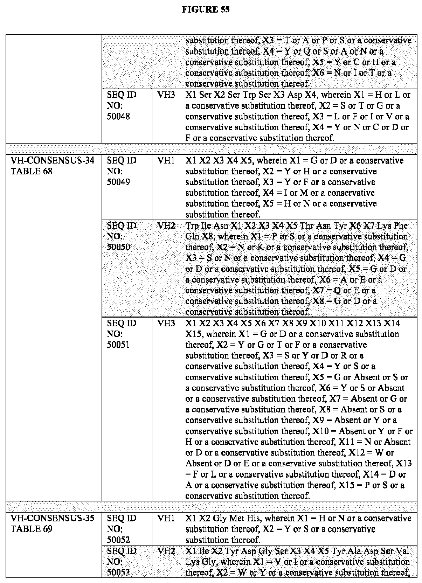

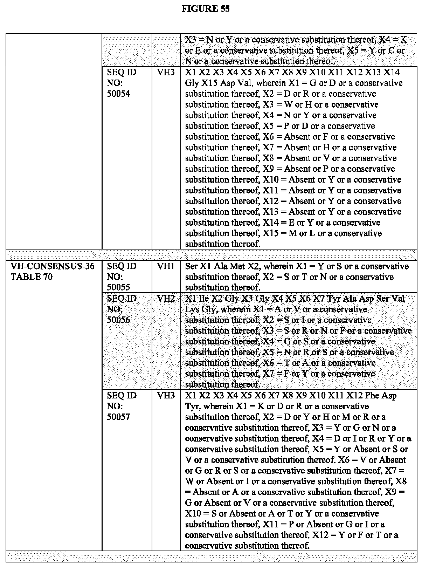

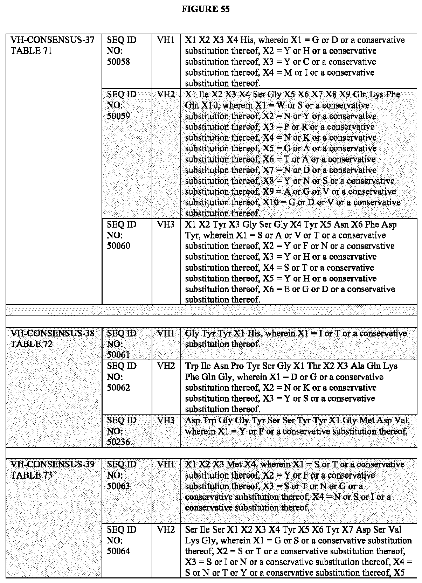

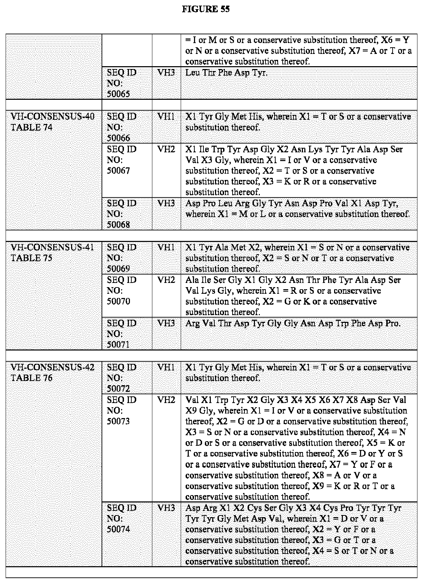

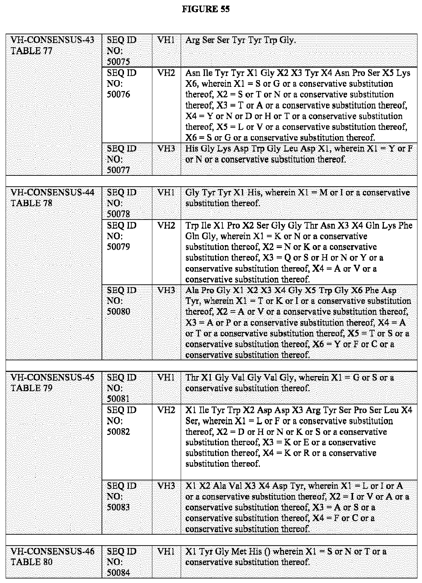

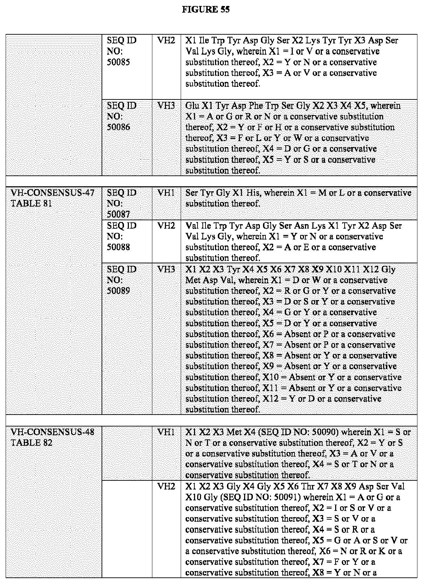

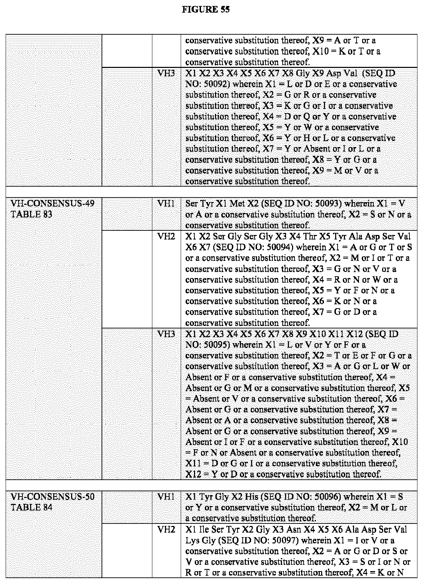

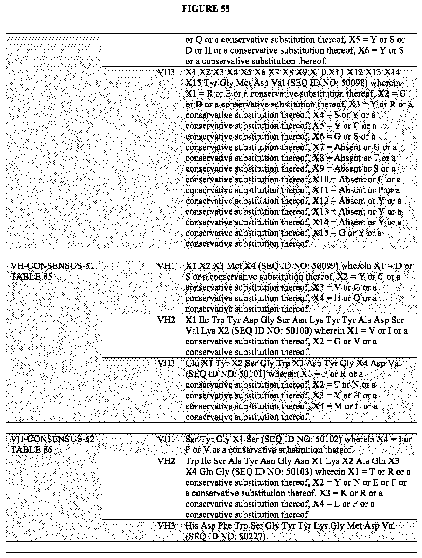

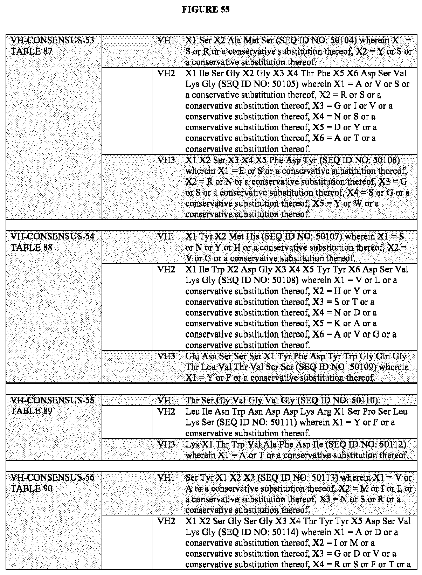

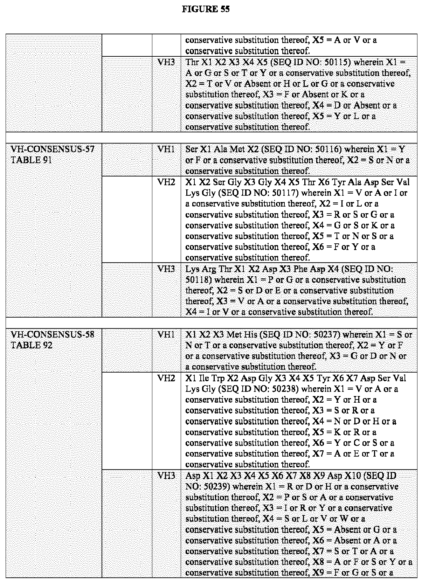

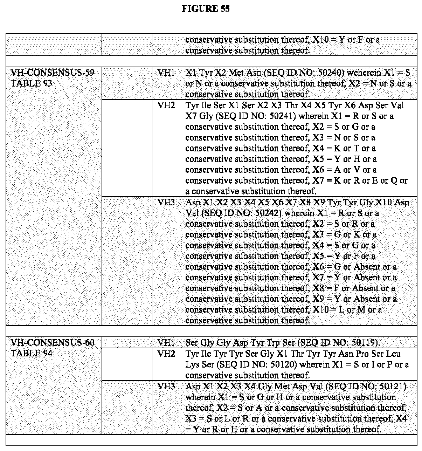

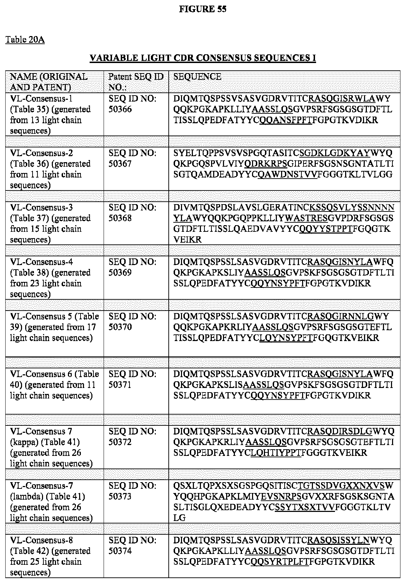

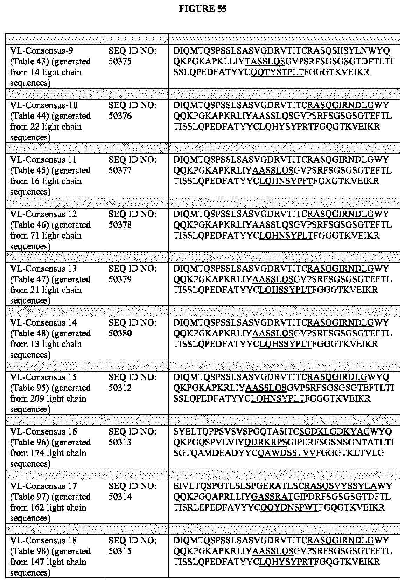

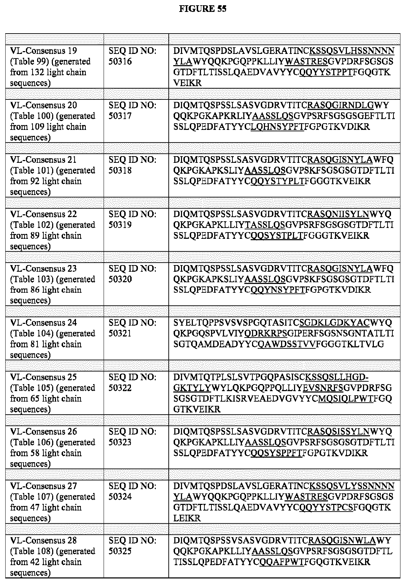

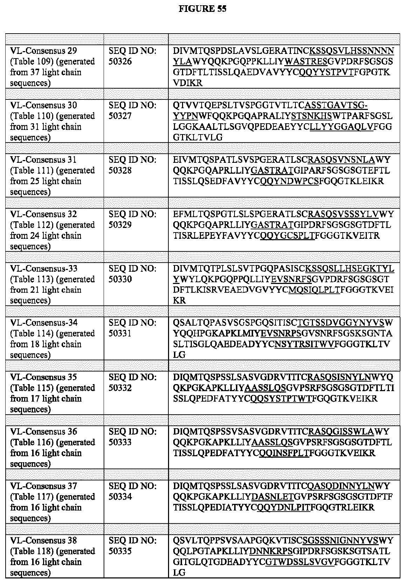

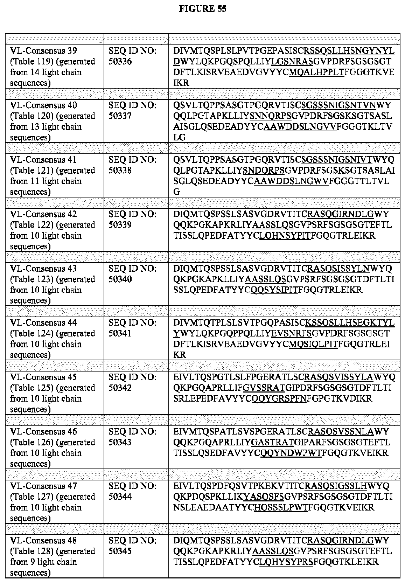

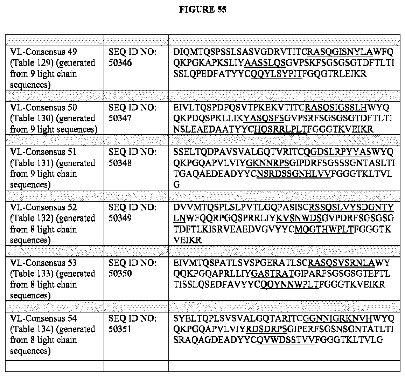

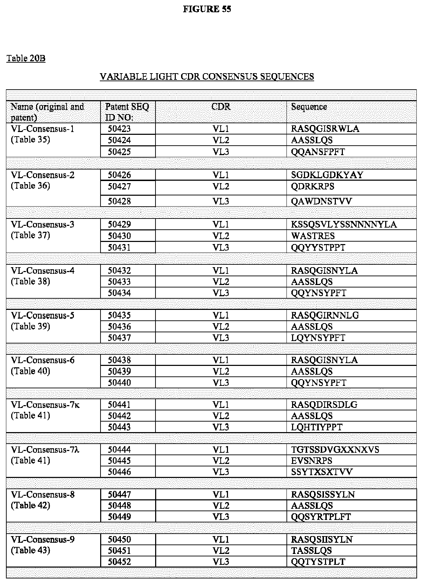

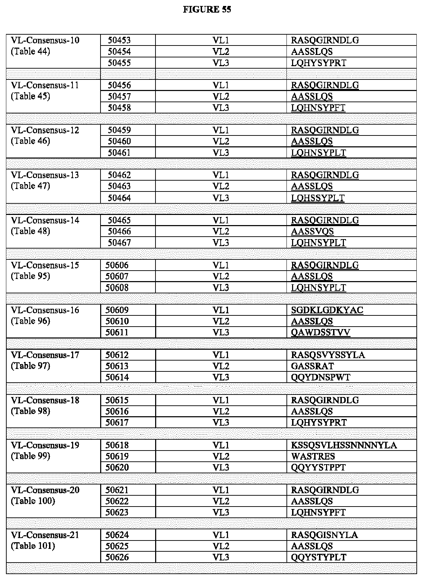

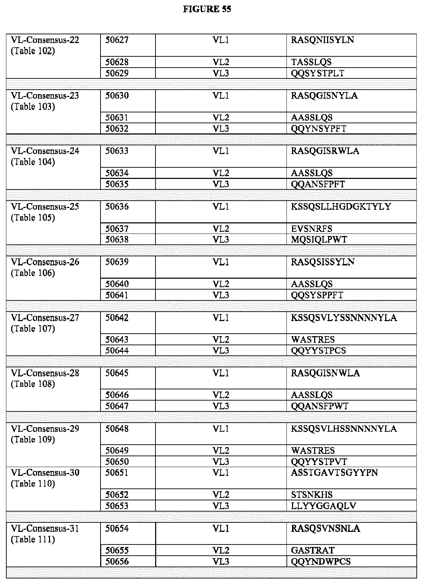

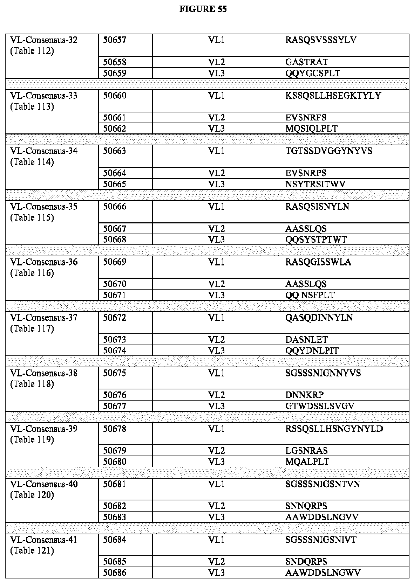

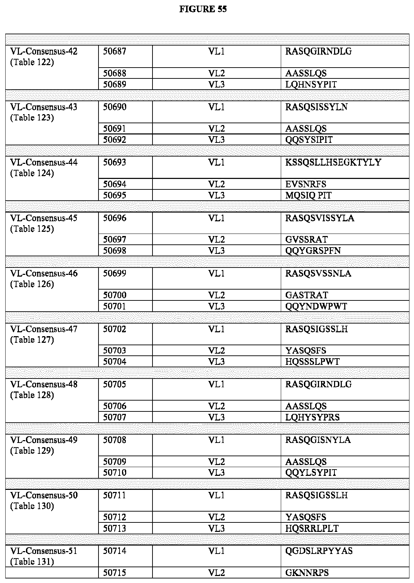

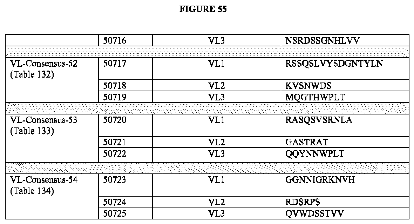

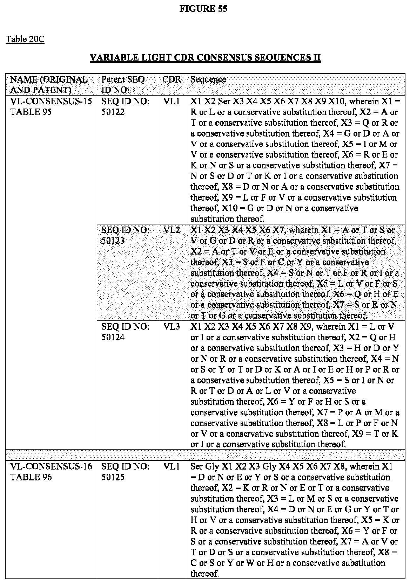

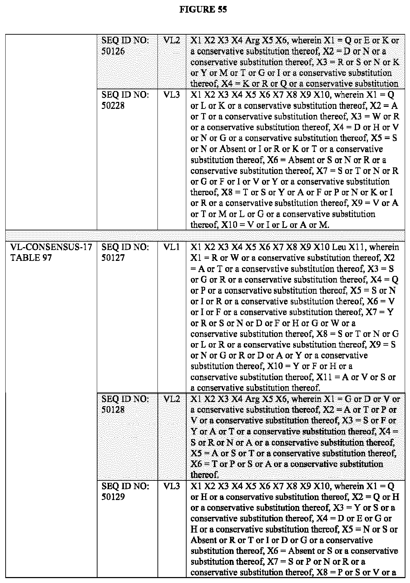

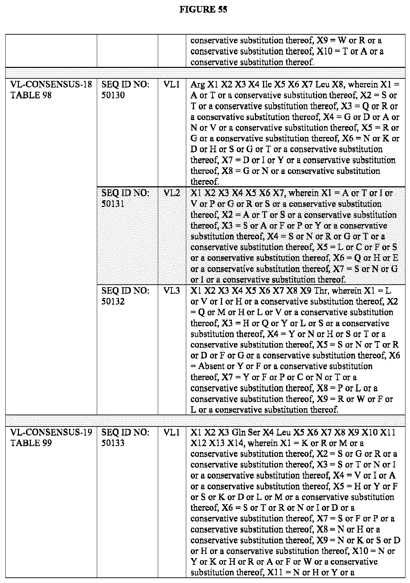

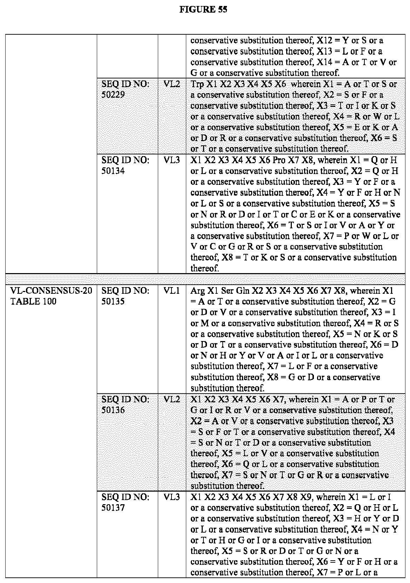

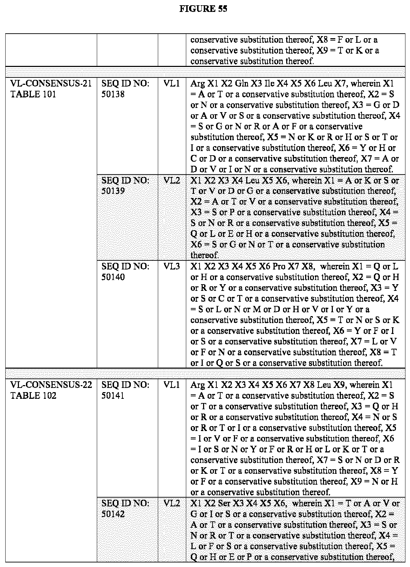

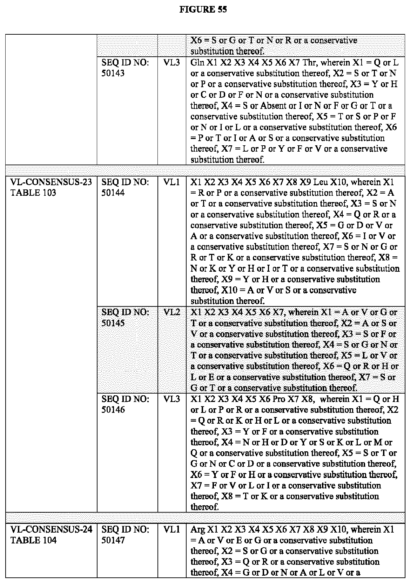

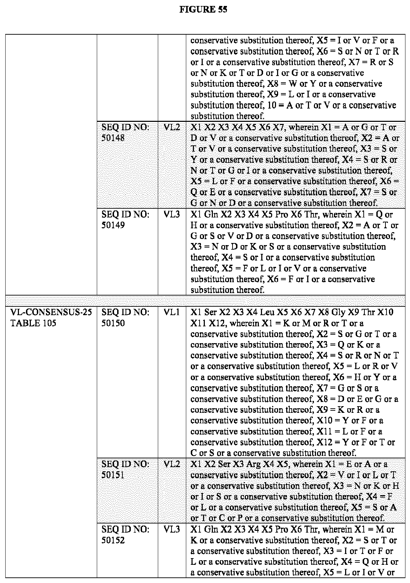

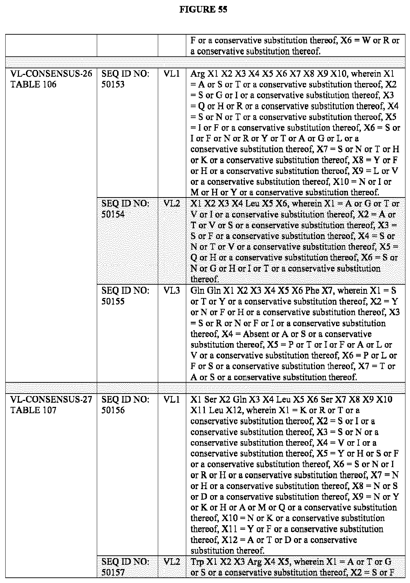

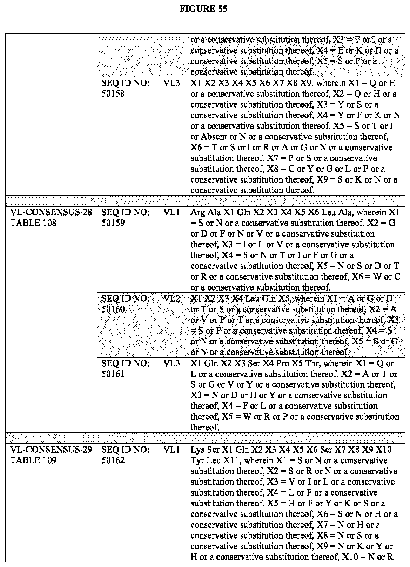

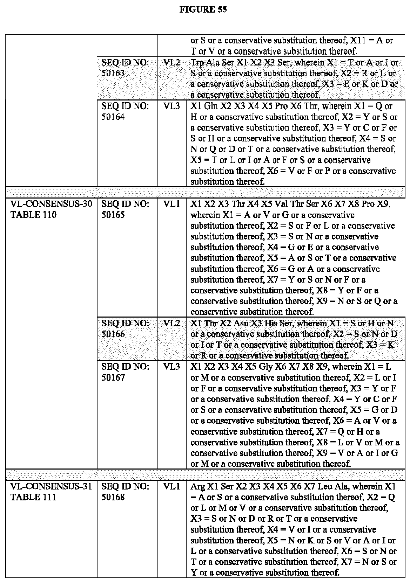

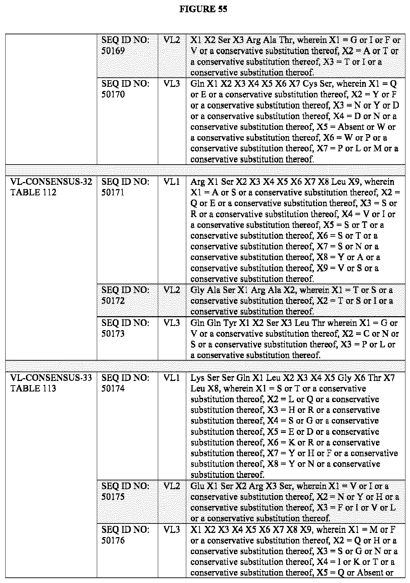

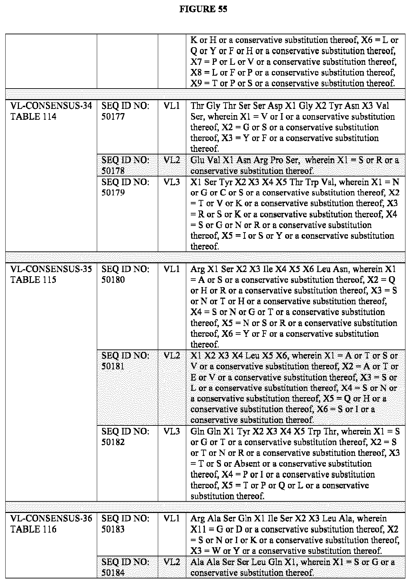

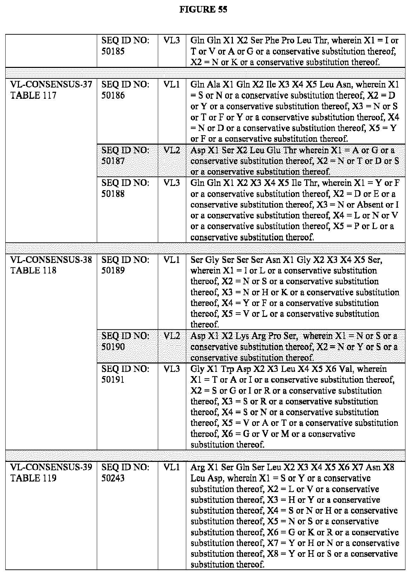

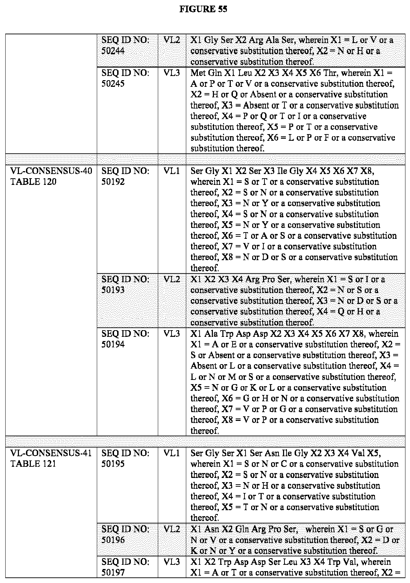

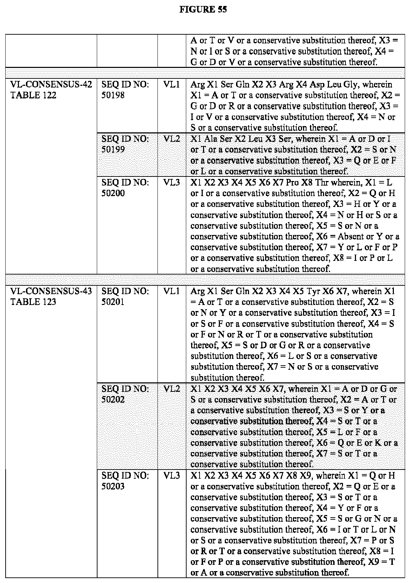

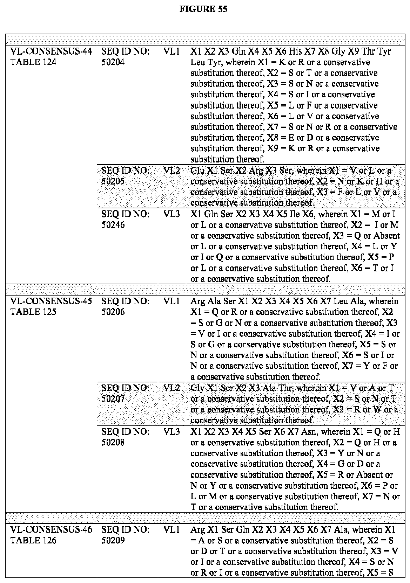

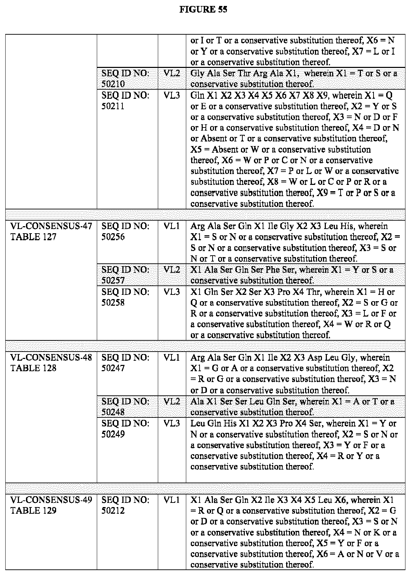

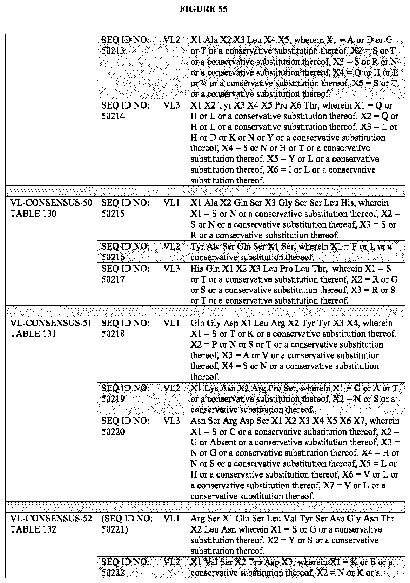

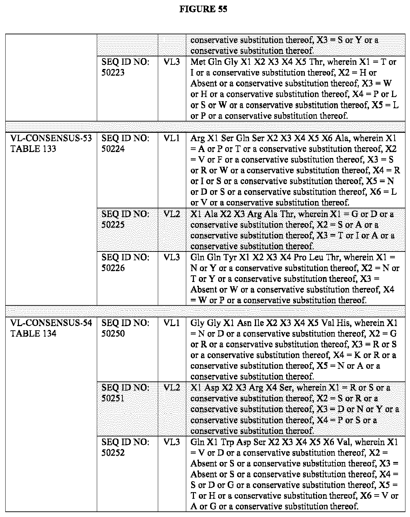

In some aspects, the invention provides an isolated antigen binding protein, wherein the antigen binding protein binds human ASGR-1 and comprises one or more VH CDR1, VH CDR2 or VH CDR3 having an amino acid sequence identical to or comprising no more than 18 amino acid residue substitutions, insertions or deletions in each CDR relative to the VH of any of the sequences set forth in Table 19A as depicted in Figure 55. In some embodiments, the invention provides an isolated antigen binding protein, wherein the antigen binding protein binds human ASGR-1 and comprises one or more VH CDR1, VH CDR2 or VH CDR3 having an amino acid sequence identical to or comprising a conservative subsitutuion of any of the amino acid sequences set forth in Tables 19B or 19C, as depicted in Figure 55. In some aspects, the invention comprises an isolated antigen binding protein, wherein the isolated antigen binding protein binds to human ASGR-1 and comprises one or more VL CDR1, VL CDR2 or VL CDR3 having an amino acid sequence identical to or comprising no more than 14 amino acid residue substitutions, insertions or deletions in each CDR relative to the VL of any of the sequences set forth in Table 20A as depicted in Figure 55. In some embodiments, the invention provides an isolated antigen binding protein, wherein the antigen binding protein binds human ASGR-1 and comprises one or more VL CDR1, VL CDR2 or VL CDR3 having an amino acid sequence identical to or comprising a conservative subsitutuion of any of the amino acid sequences set forth in Tables 20B or 20C, as depicted in Figure 55. In some embodiments, the isolated antigen binding protein comprises one or more VH CDR1, VH CDR2 or VH CDR3 having an amino acid sequence identical to or comprising no more than 18 amino acid residue substitutions, inserions or deletions in each CDR relative to the VH of any of the sequences set forth in Table 19A, as depicted in Figure 55, and one or more VL CDR1, VL CDR2 or VL CDR3 having an amino acid sequence identical to or comprising no more than 14 amino acid residue substitutions, insertions or deletions in each CDR relative to the VL of any of the sequences set forth in Table 20A as depicted in Figure 55. In some embodiments, the invention provides an isolated antigen binding protein, wherein the antigen binding protein binds human ASGR-1 and comprises one or more VH CDR1, VH CDR2 or VH CDR3 having an amino acid sequence identical to or comprising a conservative subsitutuion of any of the amino acid sequences set forth in Tables 19B or 19C, as depicted in Figure 55, and one or more VL CDR1, VL CDR2 or VL CDR3 having an amino acid sequence identical to or comprising a conservative subsitutuion of any of the amino acid sequences set forth in Tables 20B or 20C, as depicted in Figure 55. In some embodiments, the isolated antigen binding protein comprises one VH CDR1, VH CDR2 or VH CDR3 having an amino acid sequence identical no more than 18 amino acid residue substitutions, insertions or deletions in each CDR relative to the VH of any of the sequences set forth in Table19A, as depicted in Figure 55, and one VL CDR1, VL CDR2 or VL CDR3 having an amino acid sequence identical to or comprising no more than 14 amino acid residue substitutions, insertions or deletions in each CDR relative to the VL of any of the sequences set forth in Table 20A, as depicted in Figure 55. In some embodiments, the invention provides an isolated antigen binding protein, wherein the antigen binding protein binds human ASGR-1 and comprises one VH CDR1, VH CDR2 or VH CDR3 having an amino acid sequence identical to or comprising a conservative subsitutuion of any of the amino acid sequences set forth in Tables 19B or 19C, as depicted in Figure 55, and one VL CDR1, VL CDR2 or VL CDR3 having an amino acid sequence identical to or comprising a conservative subsitutuion of any of the amino acid sequences set forth in Tables 20B or 20C, as depicted in Figure 55. In some embodiments, the isolated antigen binding protein comprises two VH CDR1, VH CDR2 or VH CDR3 having an amino acid sequence identical to or comprising up to 18 amino acid residue substitutions, insertions or deletions in each CDR relative to the VH of any of the sequences set forth in Table 19A, as depicted in Figure 55, and two VL CDR1, VL CDR2 or VL CDR3 having an amino acid sequence identical to or comprising up to 14amino acid residue substitutions, insertions or deletions in each CDR relative to the VL of any of the sequences set forth in Table 20A, as depicted in Figure 55. In some embodiments, the invention provides an isolated antigen binding protein, wherein the antigen binding protein binds human ASGR-1 and comprises two VH CDR1, VH CDR2 or VH CDR3 having an amino acid sequence identical to or comprising a conservative subsitutuion of any of the amino acid sequences set forth in Tables 19B or 19C, as depicted in Figure 55, and two VL CDR1, VL CDR2 or VL CDR3 having an amino acid sequence identical to or comprising a conservative subsitutuion of any of the amino acid sequences set forth in Tables 20B or 20C, as depicted in Figure 55. In some embodiments, the isolated antigen binding protein comprises the VH CDR1, VH CDR2 and VH CDR3 having an amino acid sequence identical to or comprising up to 18 amino acid residue substitutions, insertions or deletions in each CDR relative to the VH of any of the sequences set forth in Table 19A, as depicted in Figure 55, and the VL CDR1, VL CDR2 and VL CDR3 having an amino acid sequence identical to or comprising up to 14amino acid residue substitutions, insertions or deletions in each CDR relative to the VL of any of the sequences set forth in Table 20A, as depicted in Figure 55. In some embodiments, the invention provides an isolated antigen binding protein, wherein the antigen binding protein binds human ASGR-1 and comprises the VH CDR1, VH CDR2 or VH CDR3 having an amino acid sequence identical to or comprising a conservative subsitutuion of any of the amino acid sequences set forth in Tables 19B or 19C, as depicted in Figure 55, and the VL CDR1, VL CDR2 or VL CDR3 having an amino acid sequence identical to or comprising a conservative subsitutuion of any of the amino acid sequences set forth in Tables 20B or 20C, as depicted in Figure 55.

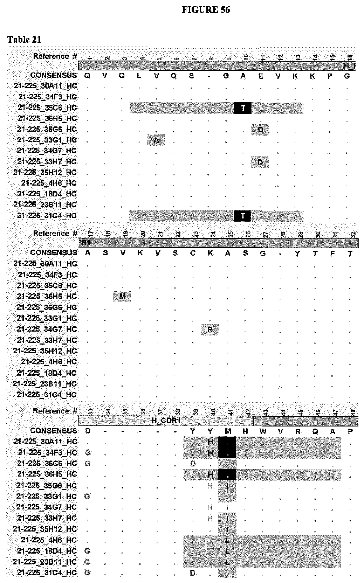

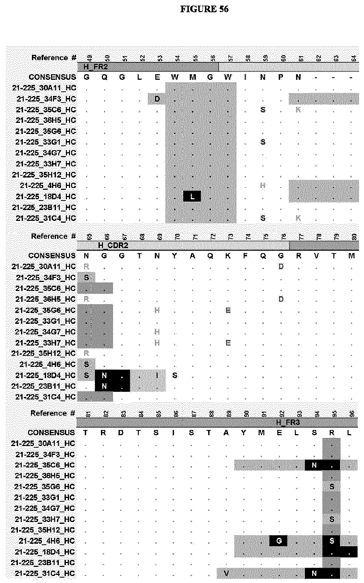

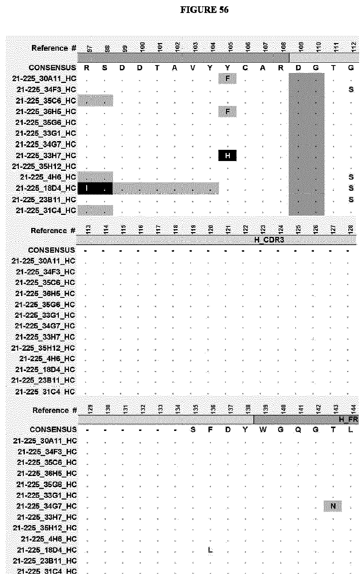

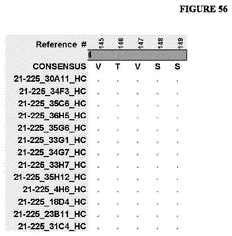

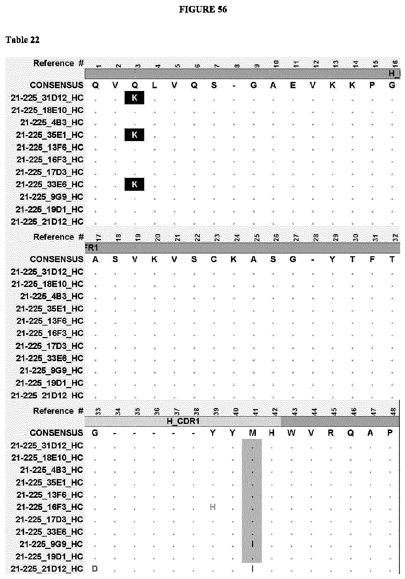

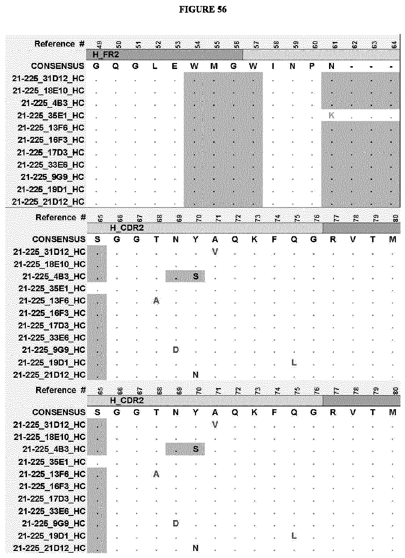

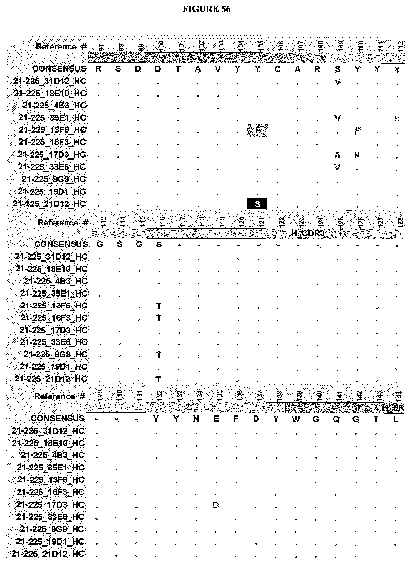

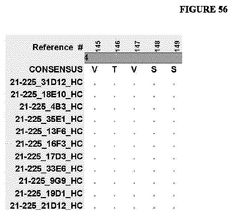

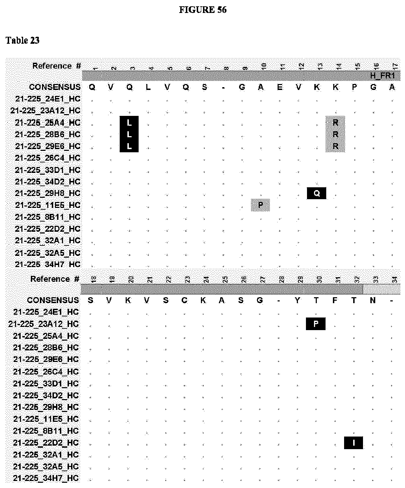

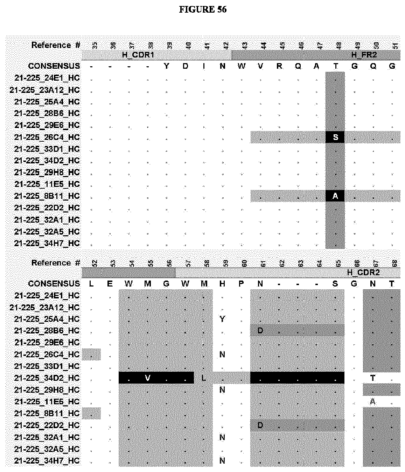

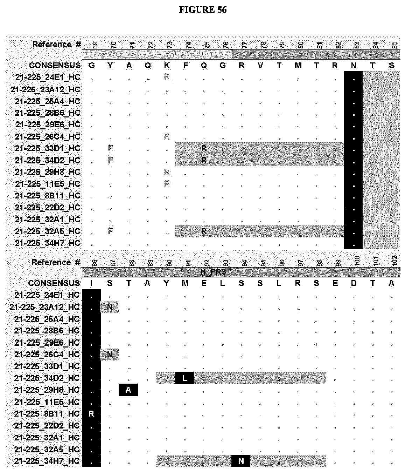

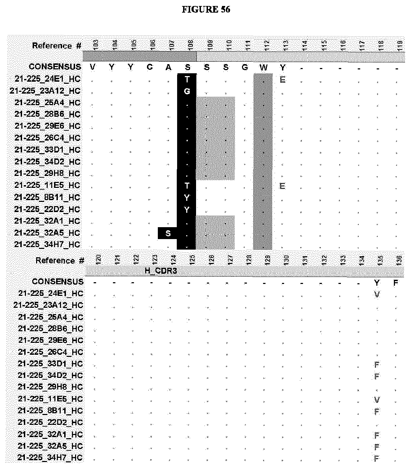

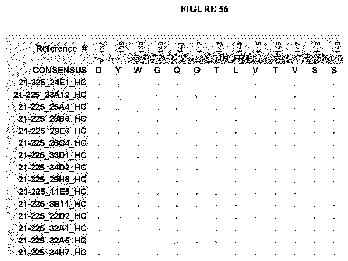

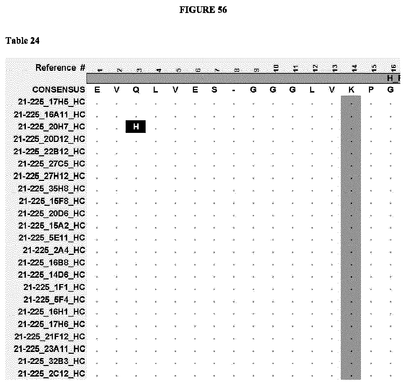

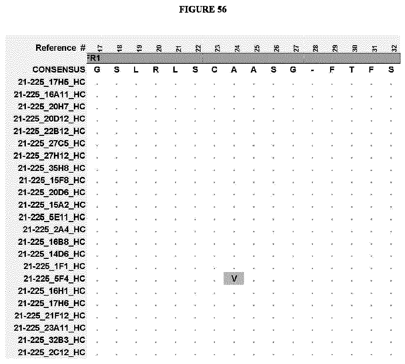

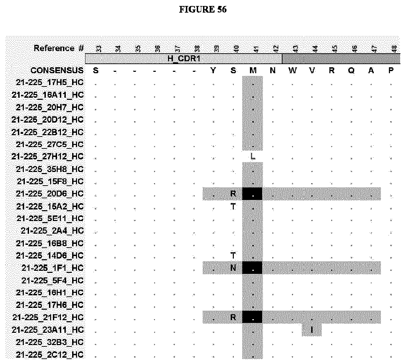

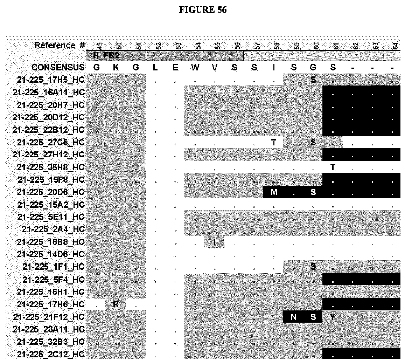

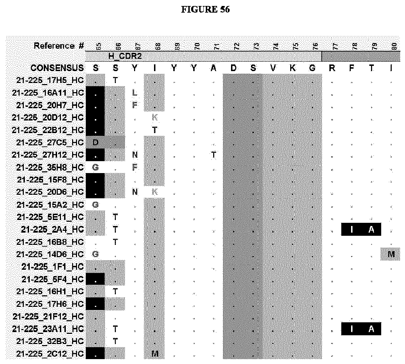

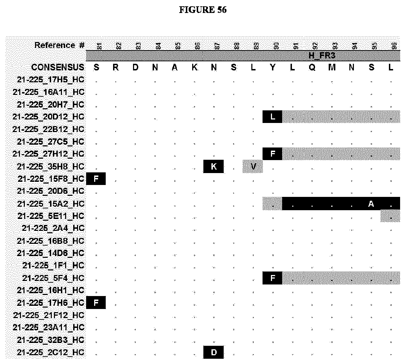

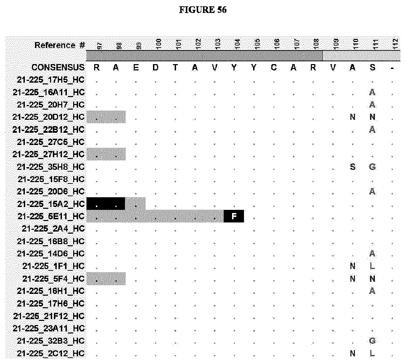

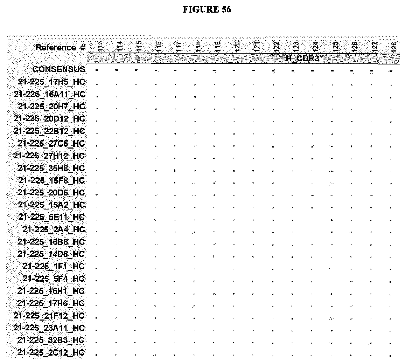

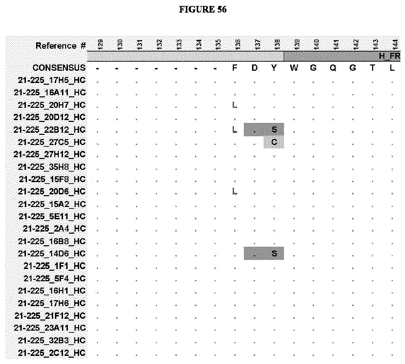

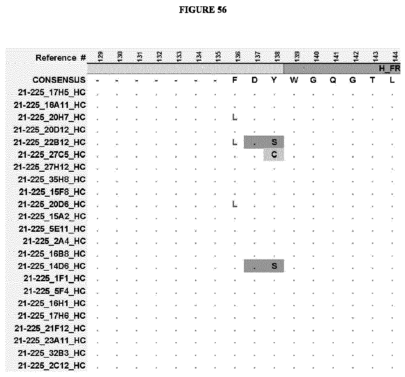

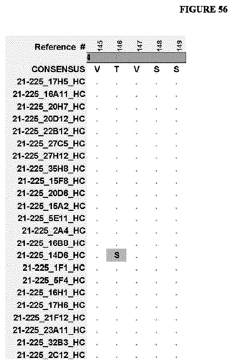

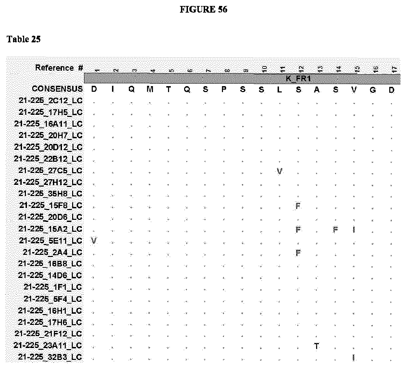

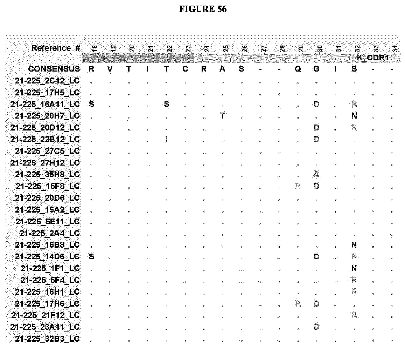

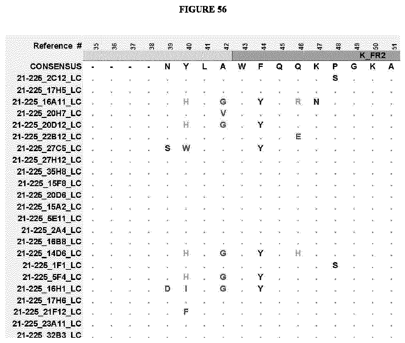

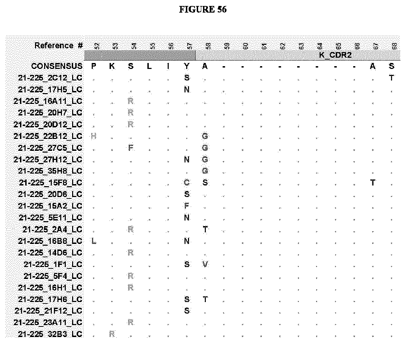

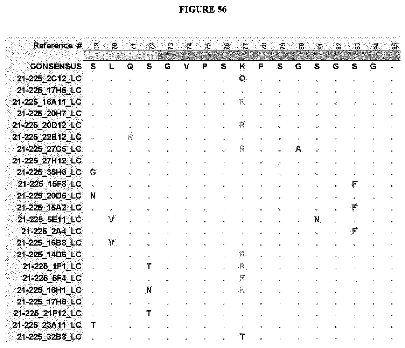

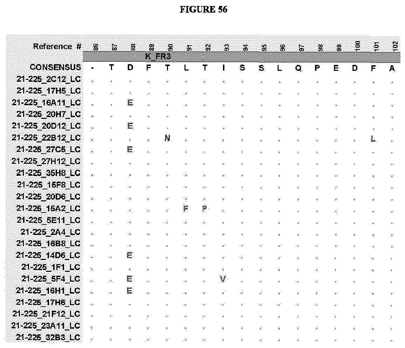

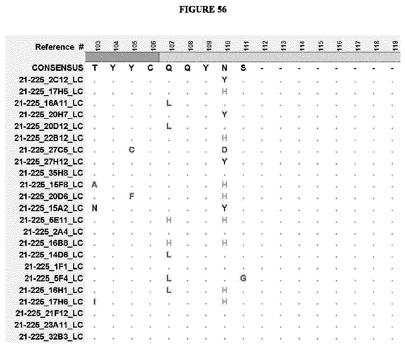

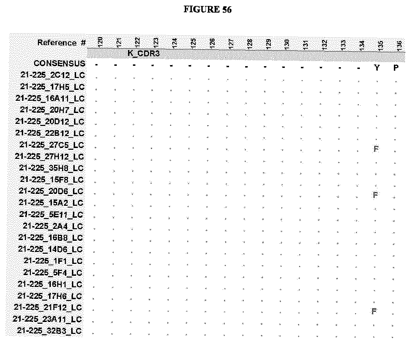

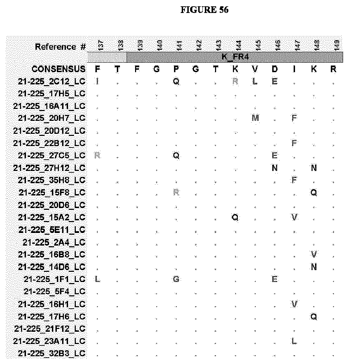

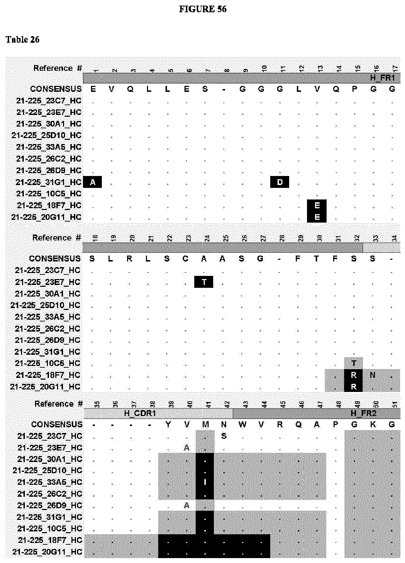

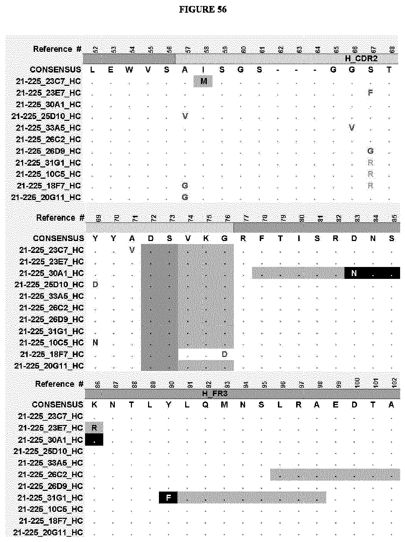

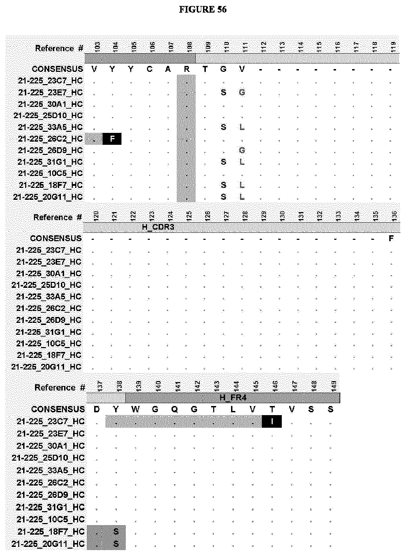

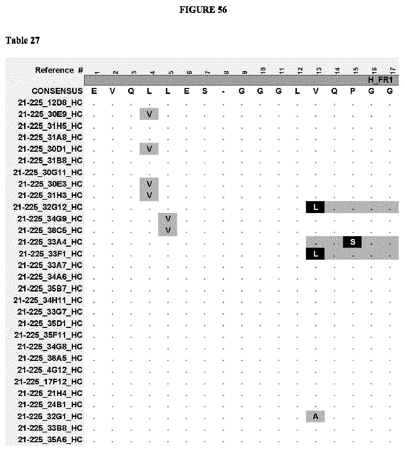

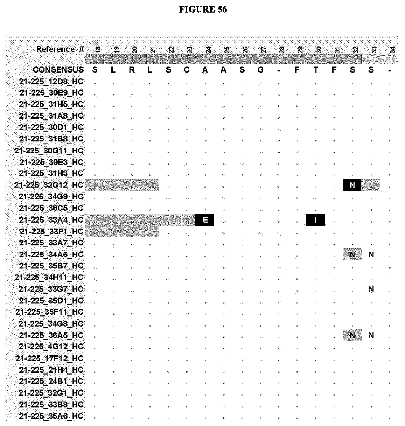

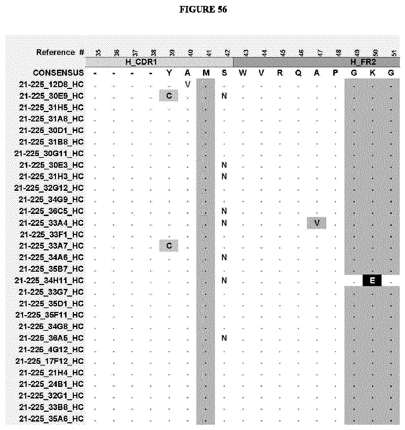

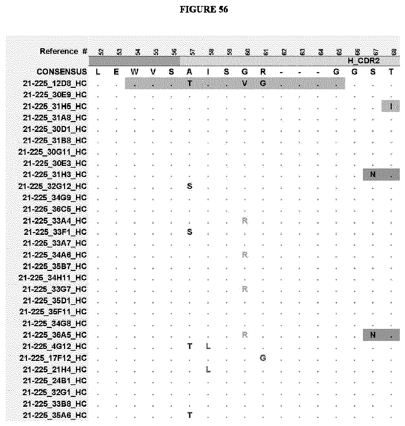

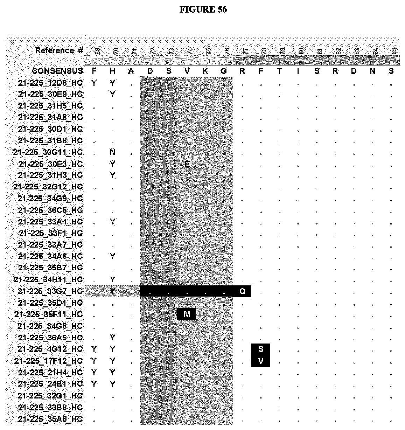

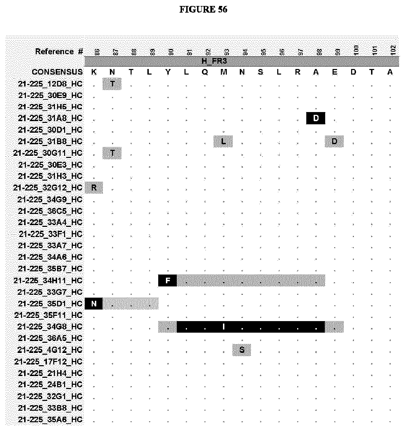

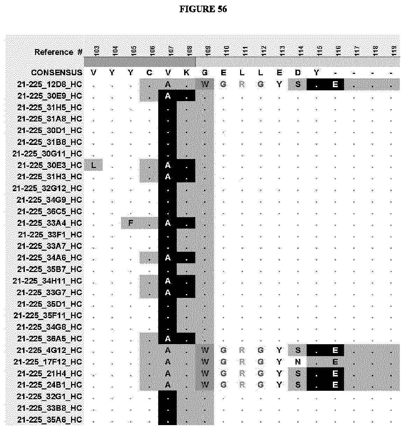

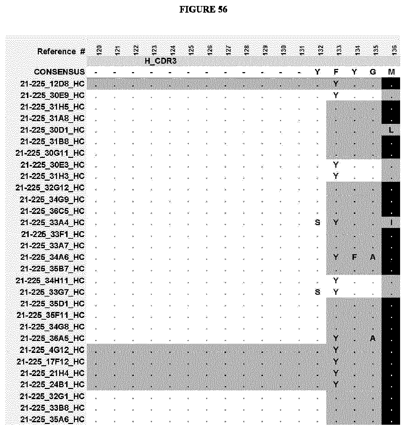

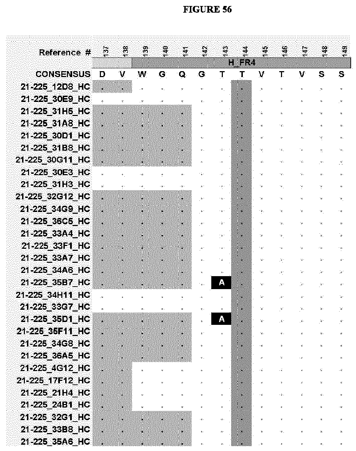

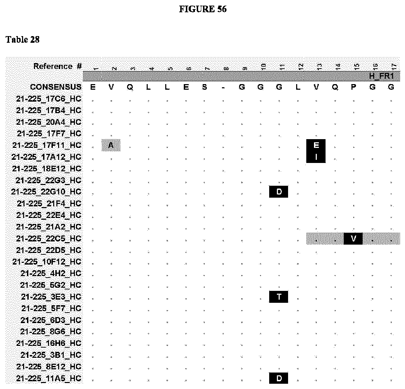

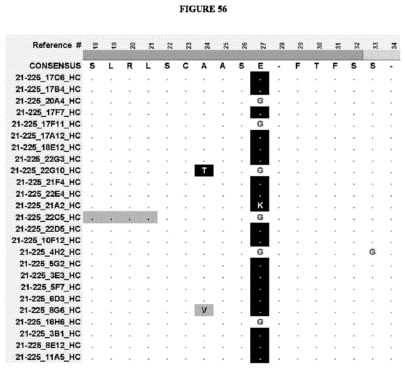

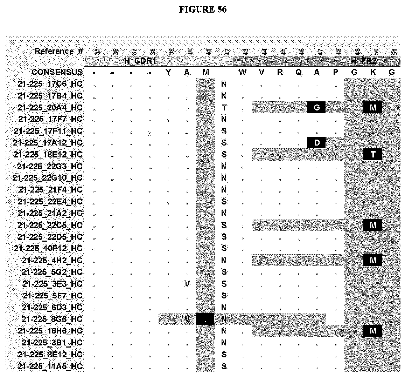

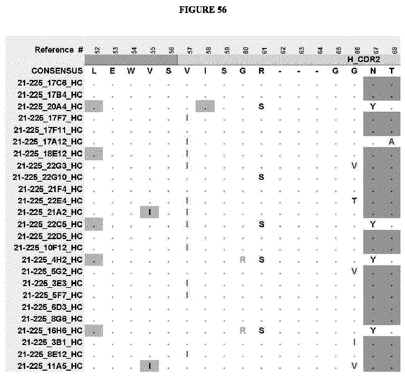

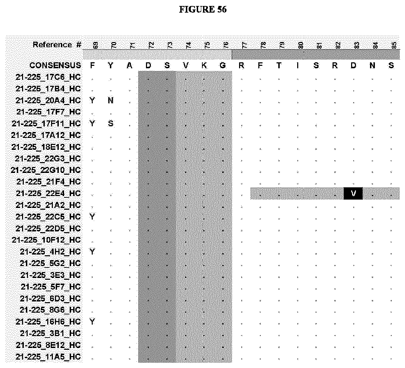

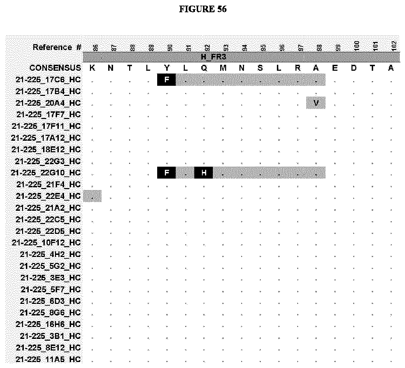

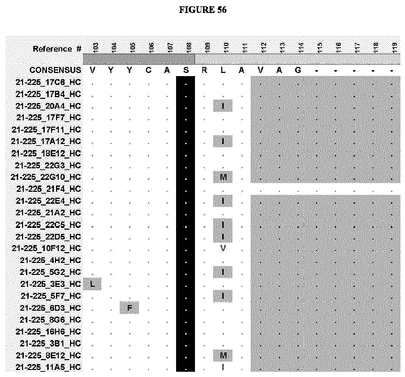

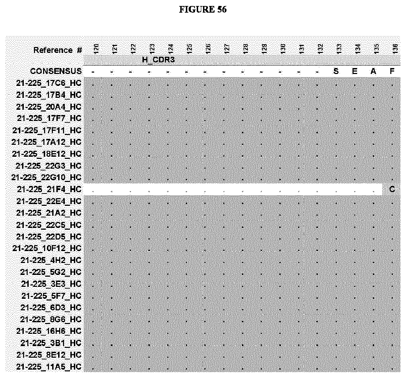

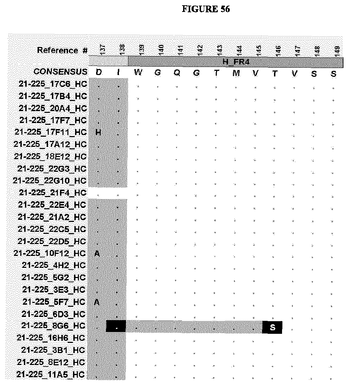

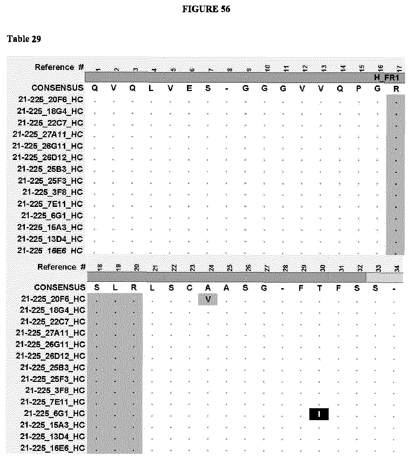

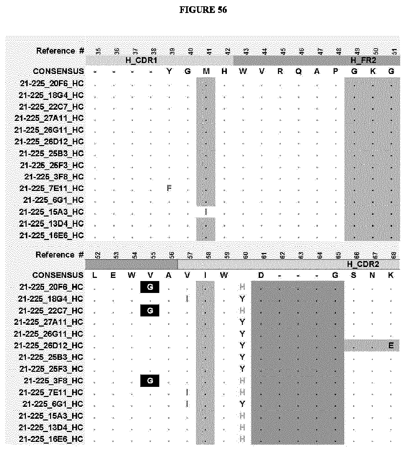

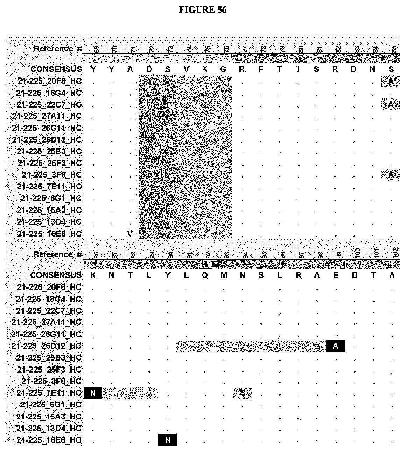

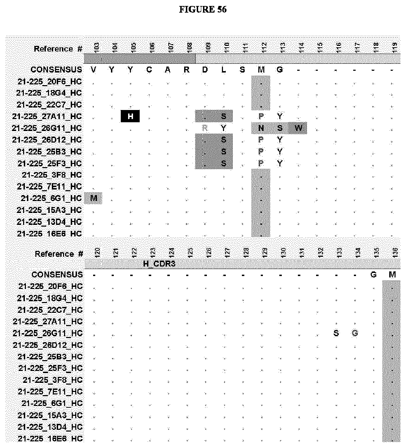

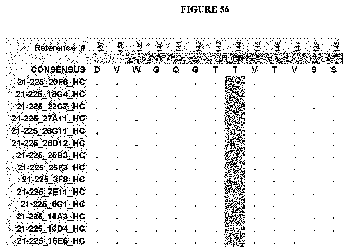

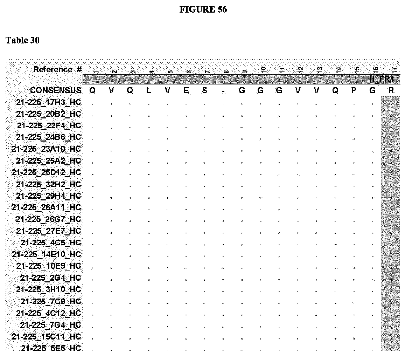

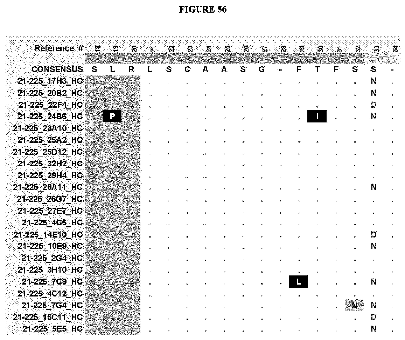

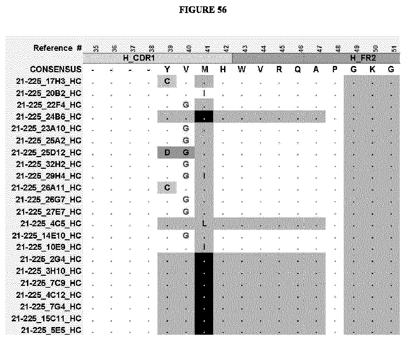

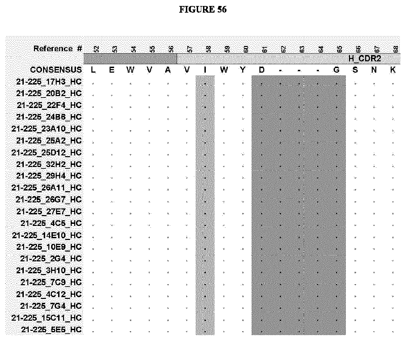

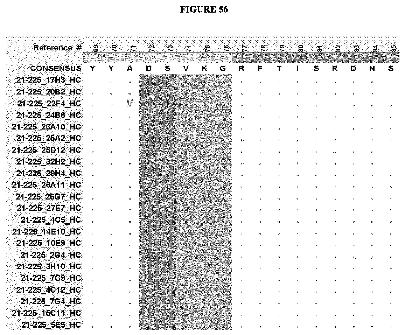

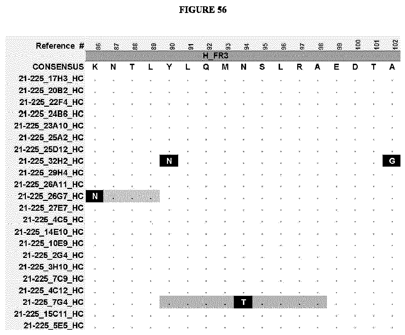

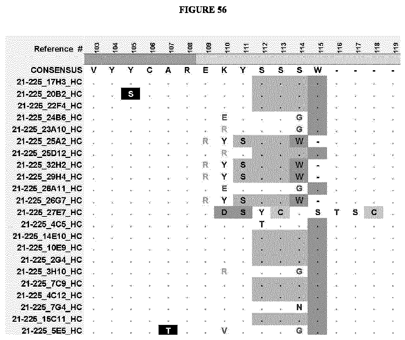

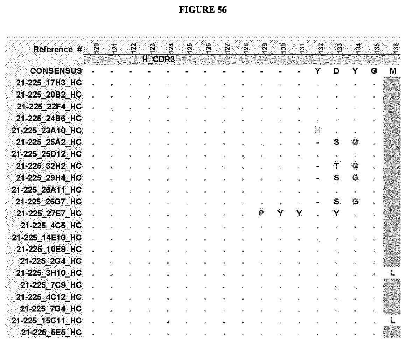

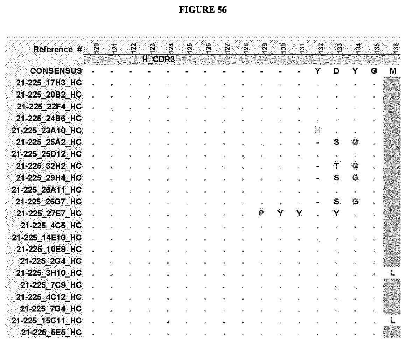

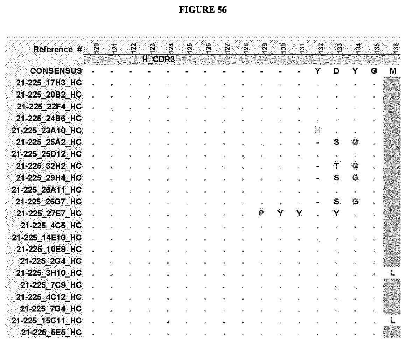

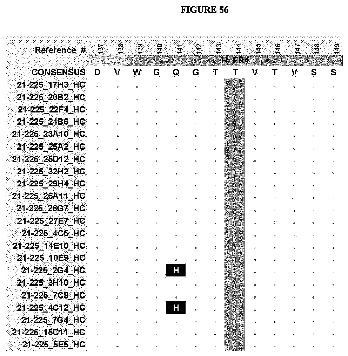

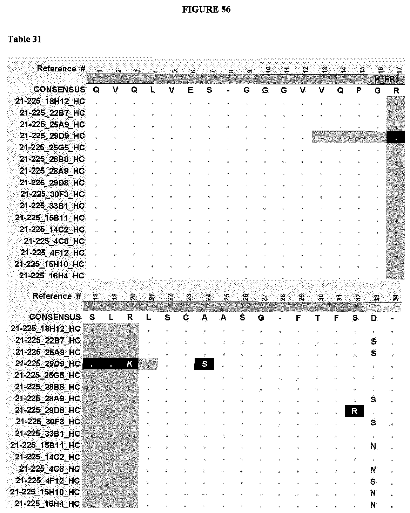

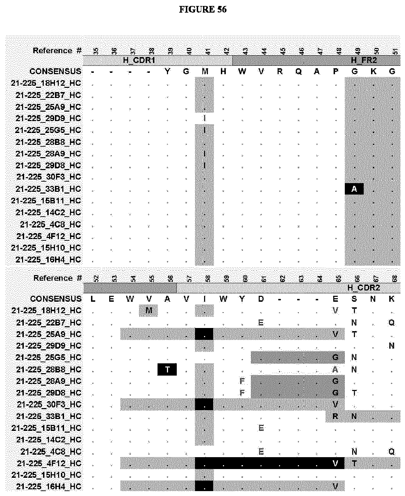

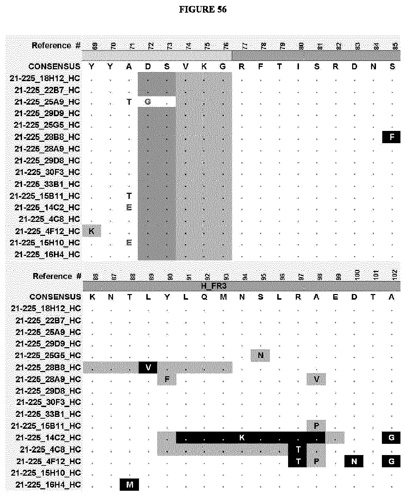

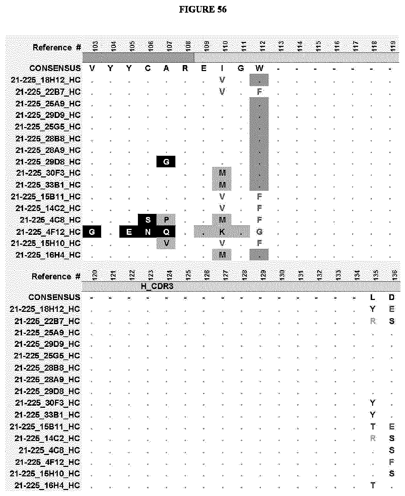

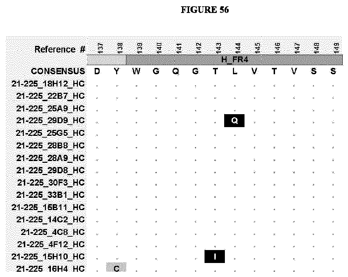

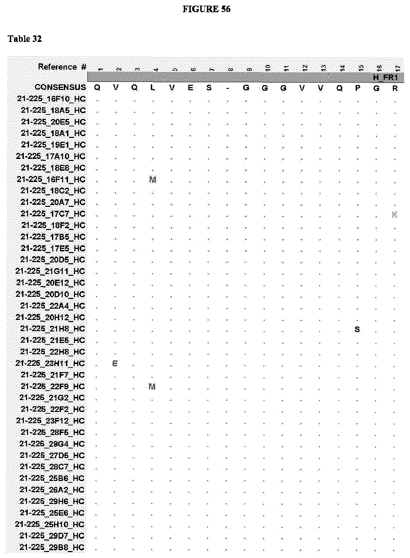

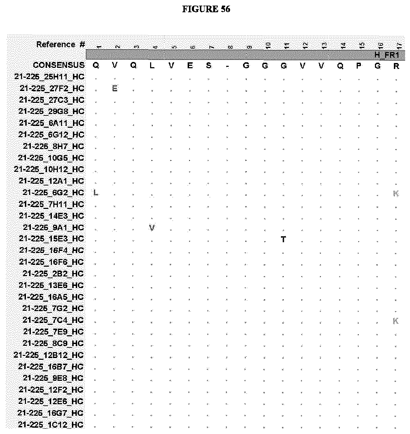

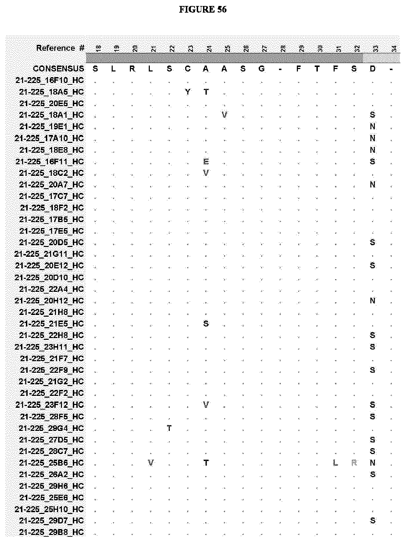

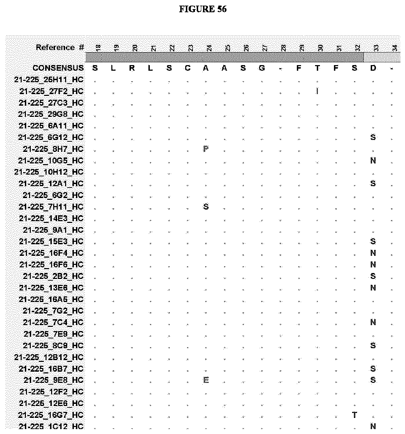

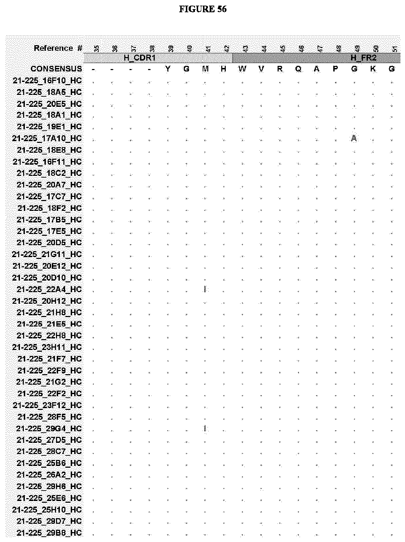

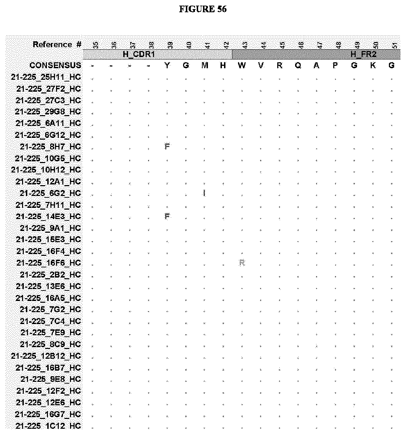

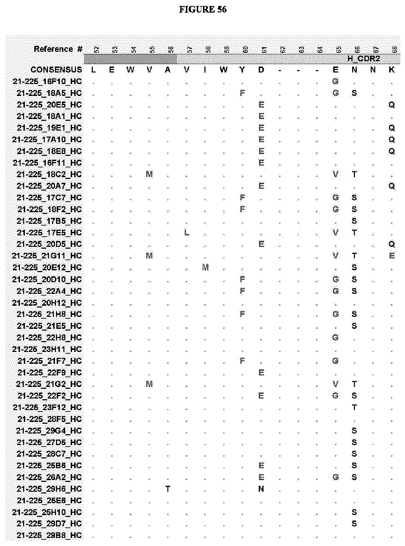

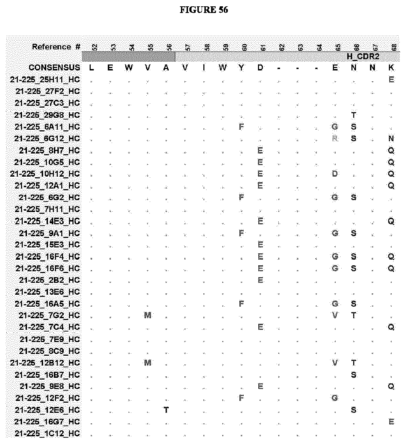

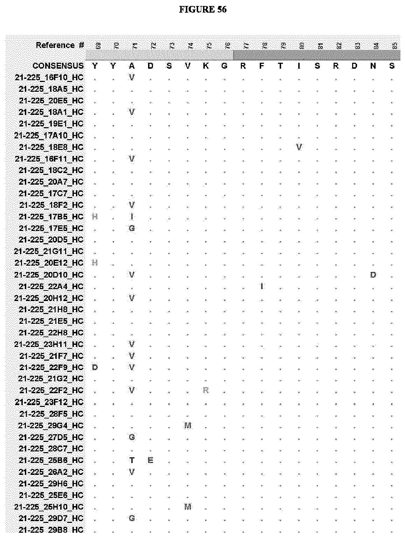

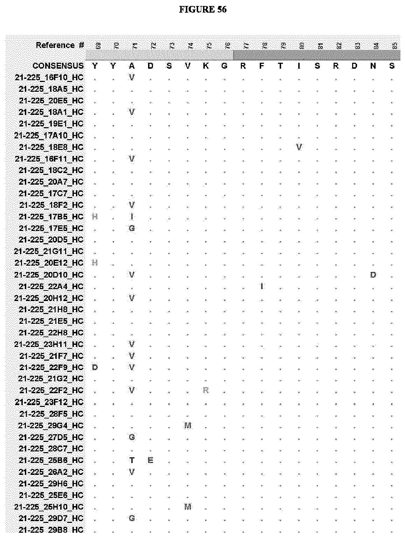

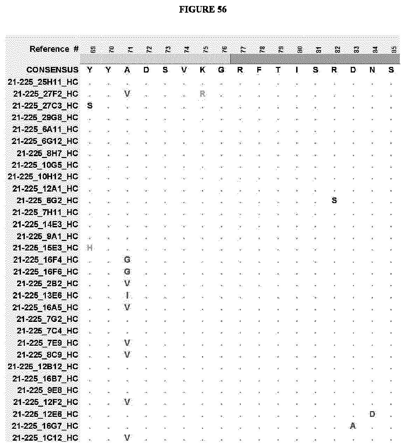

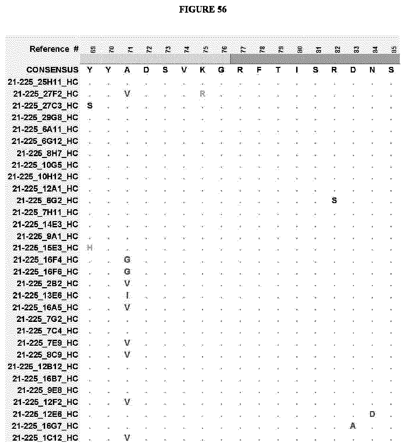

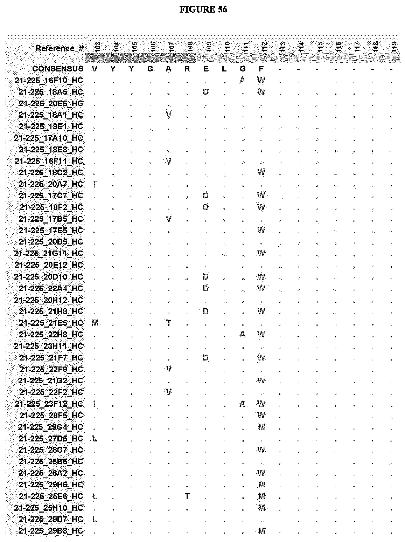

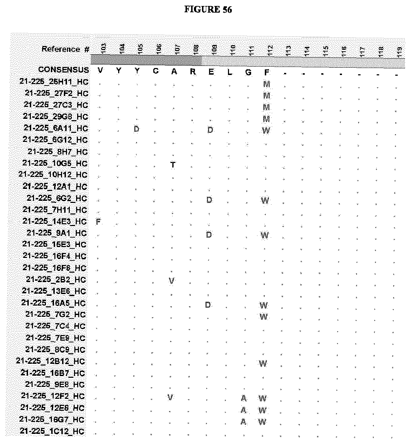

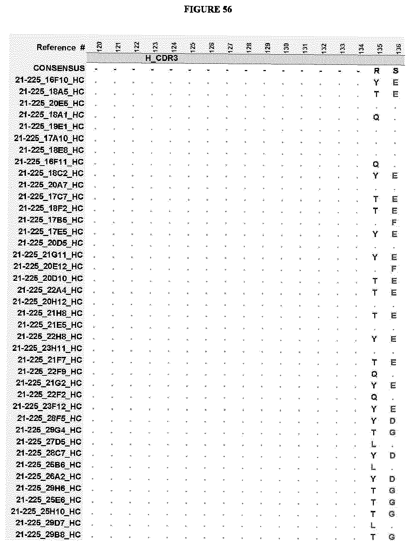

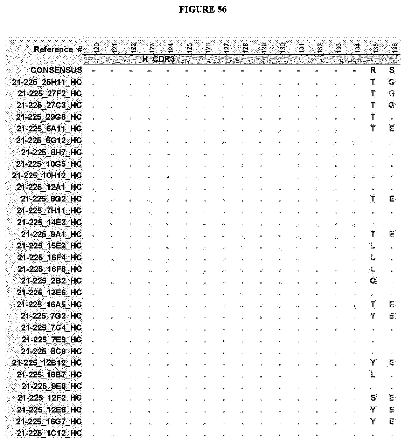

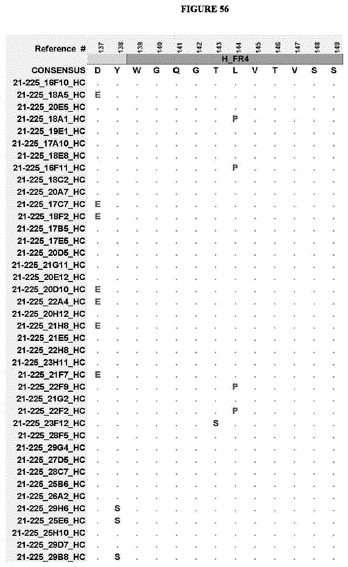

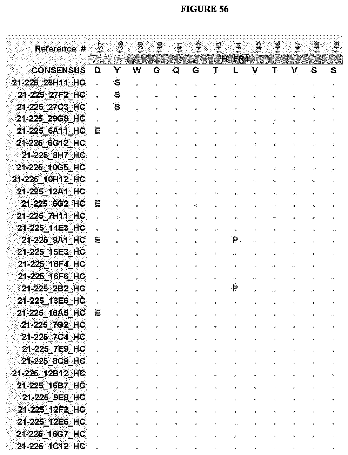

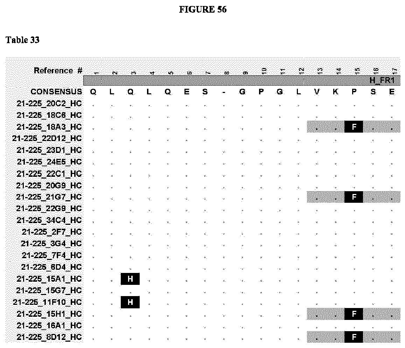

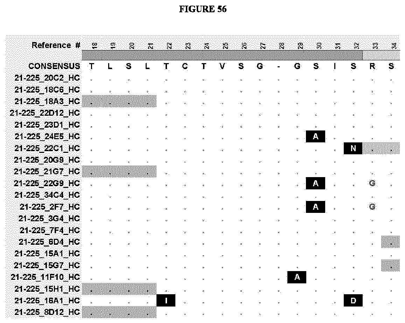

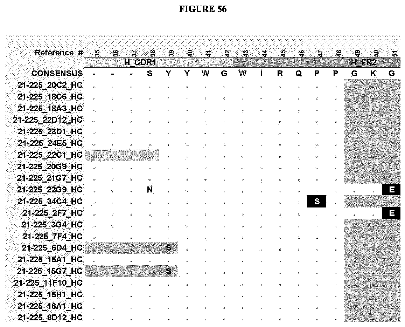

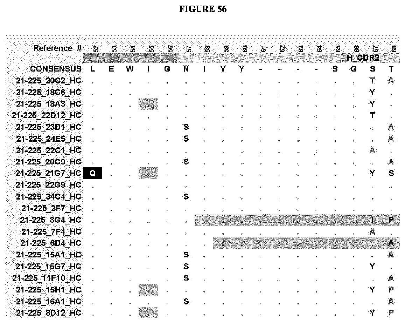

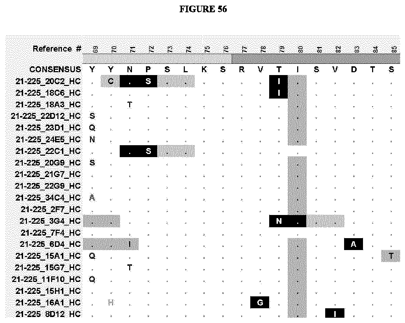

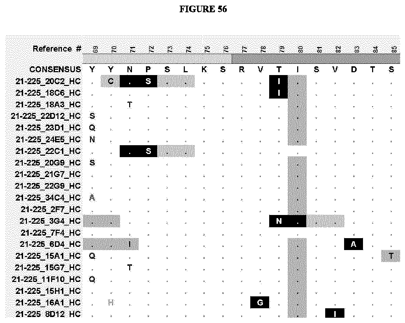

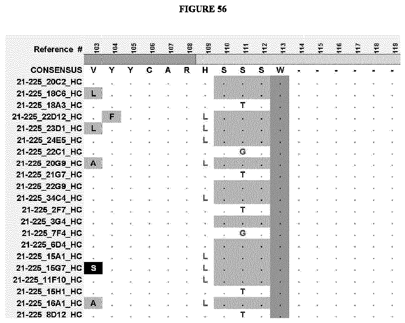

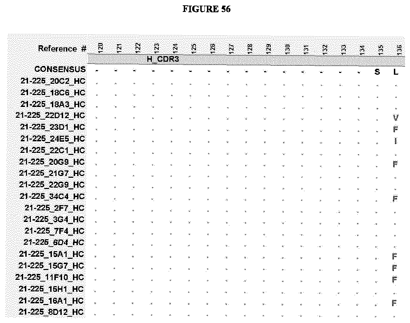

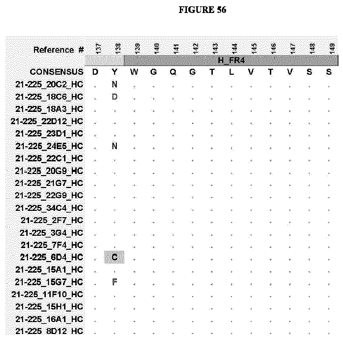

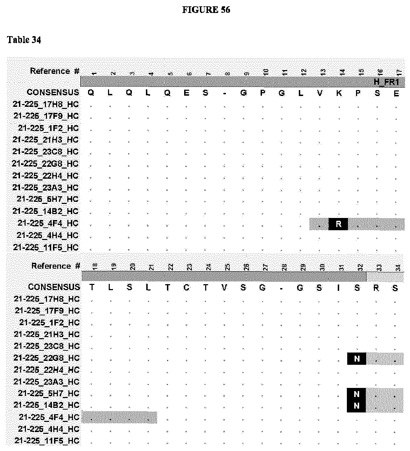

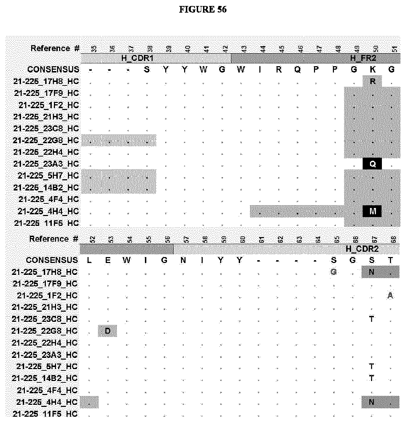

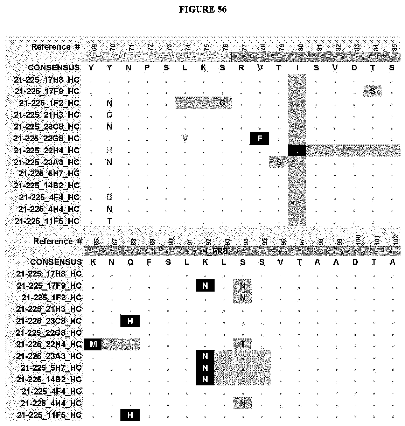

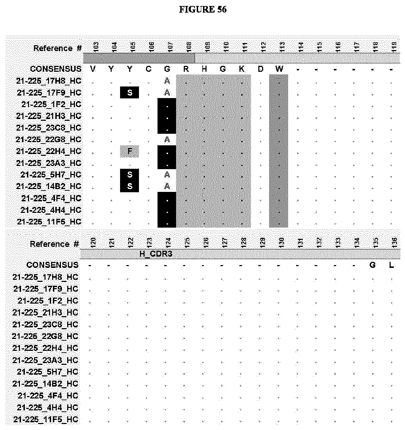

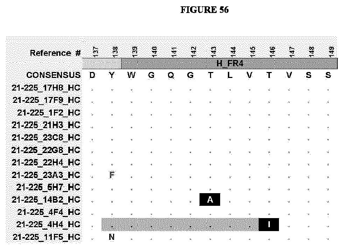

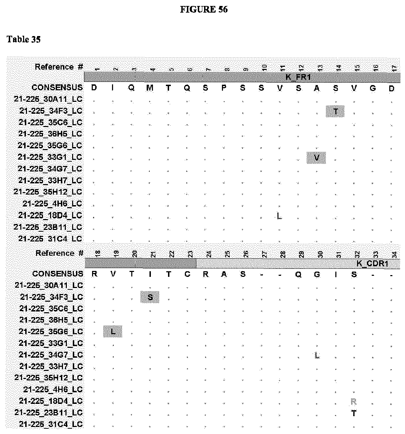

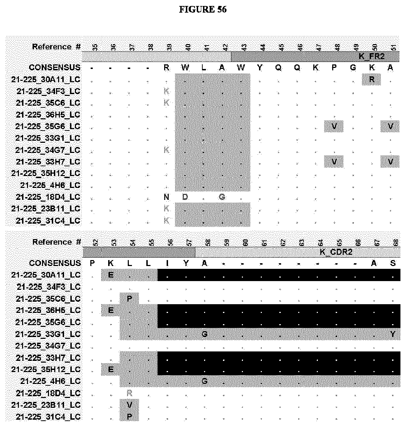

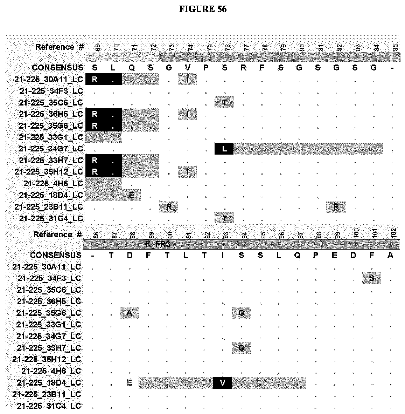

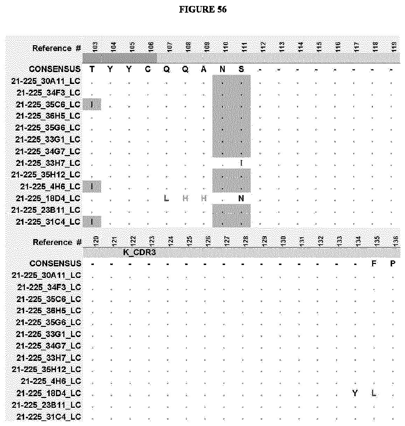

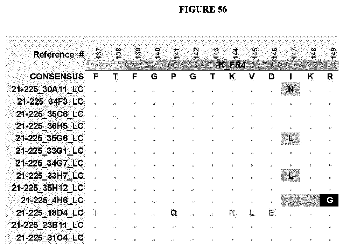

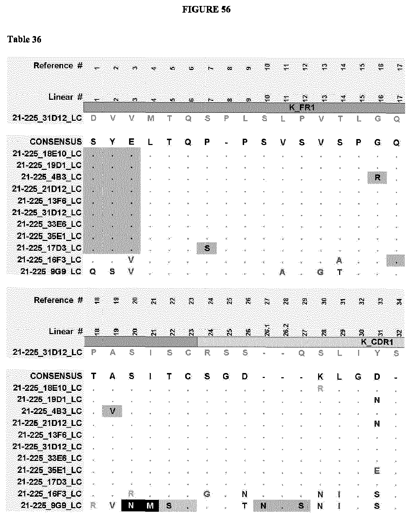

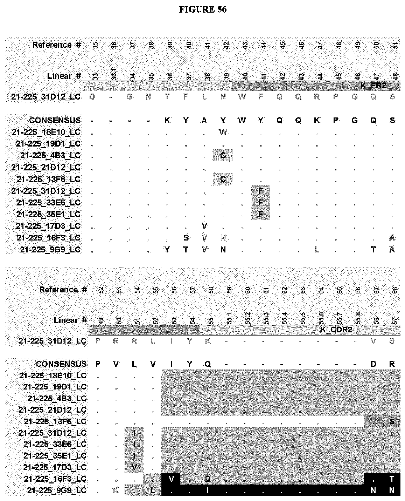

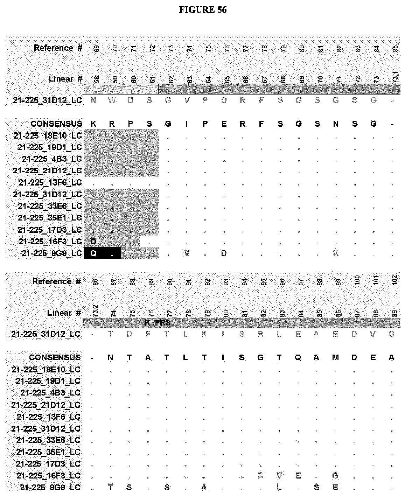

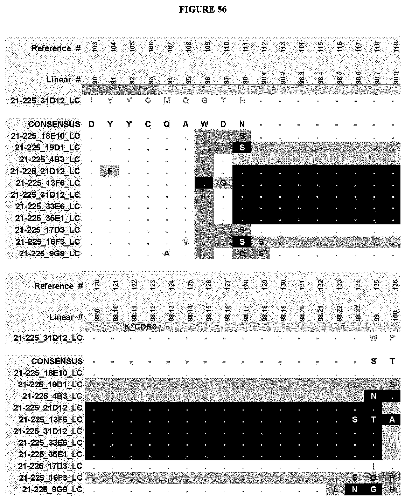

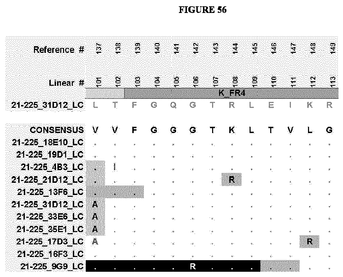

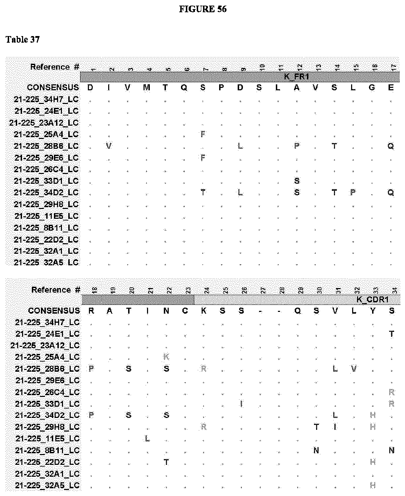

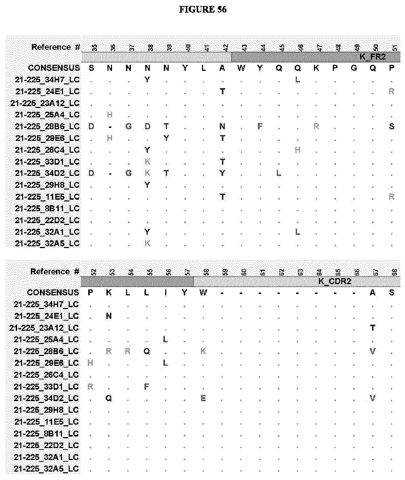

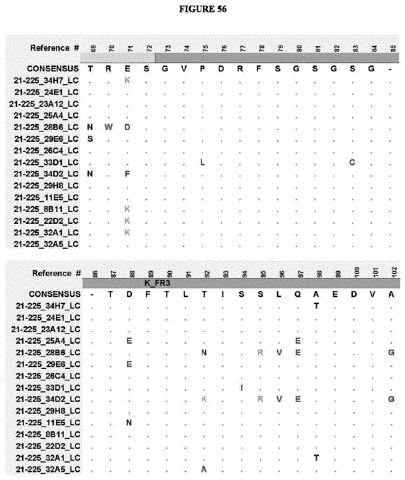

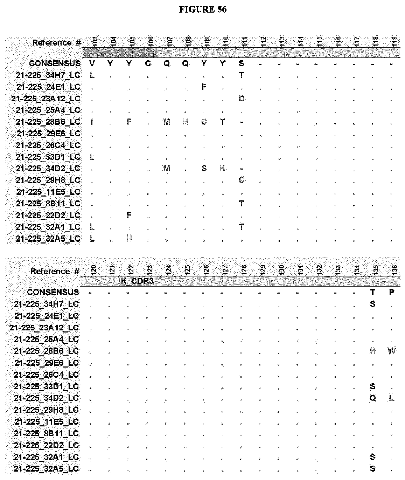

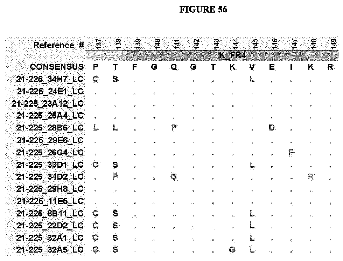

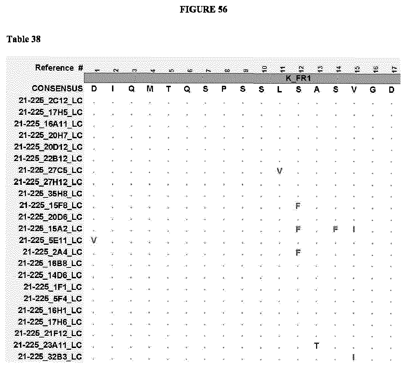

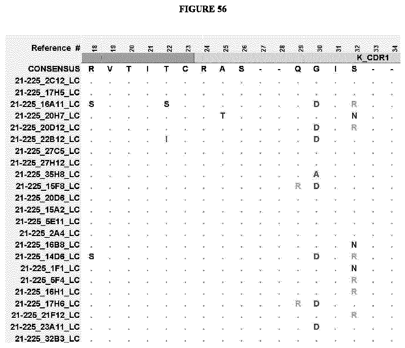

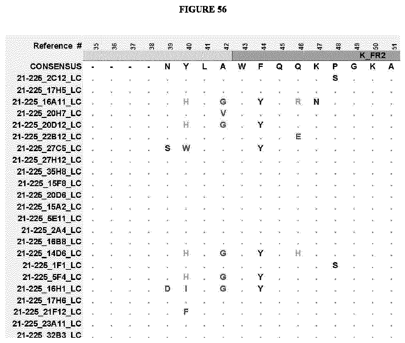

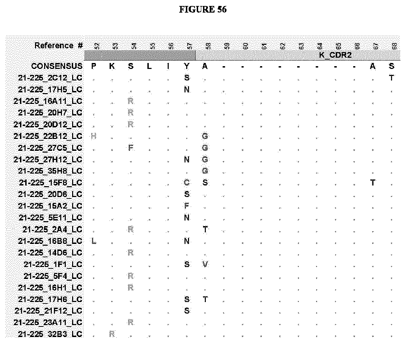

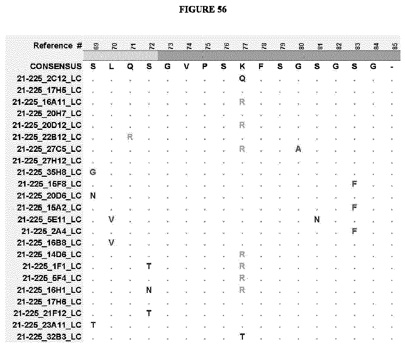

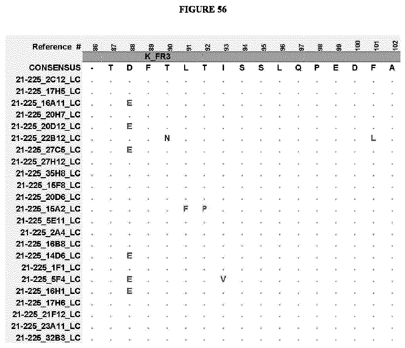

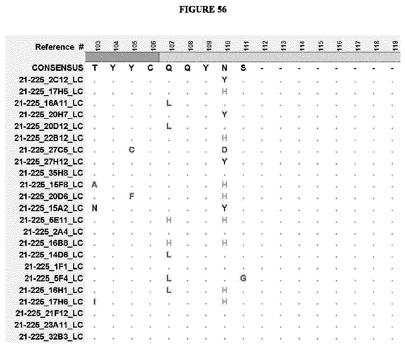

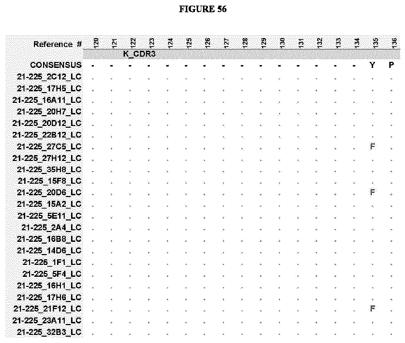

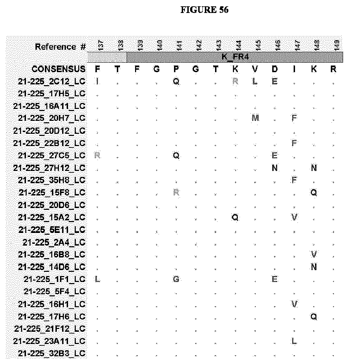

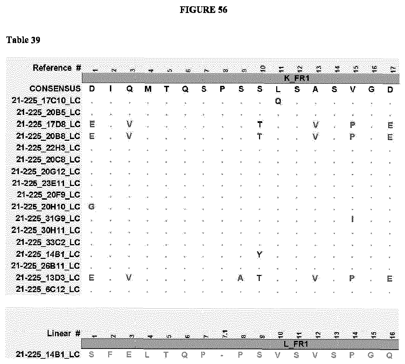

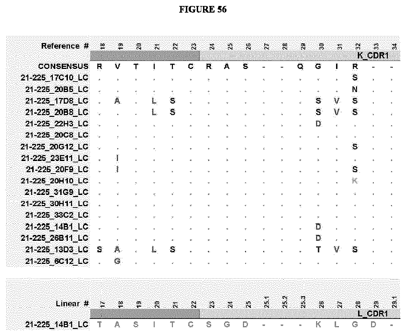

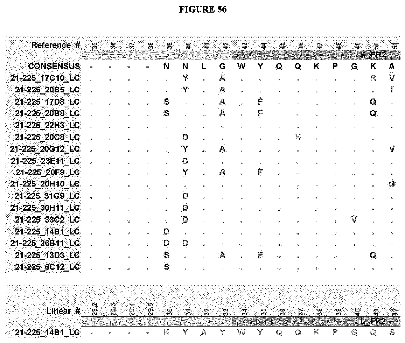

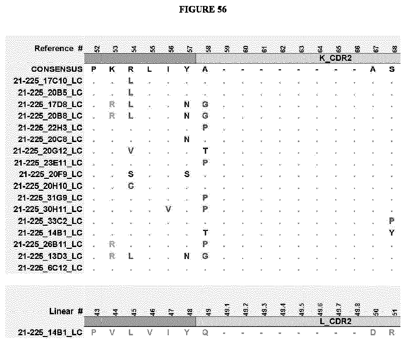

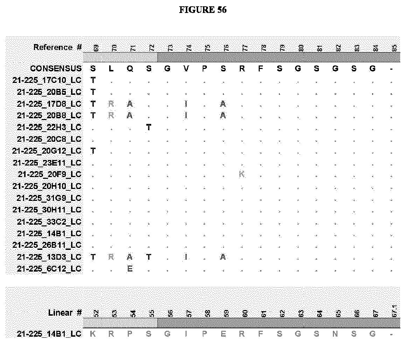

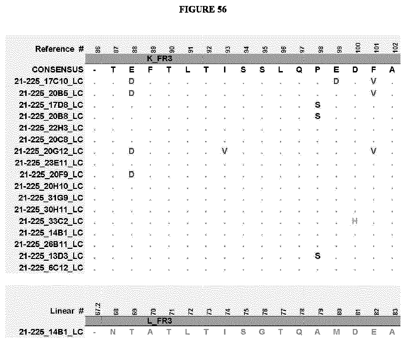

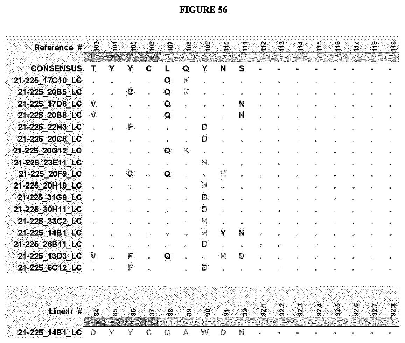

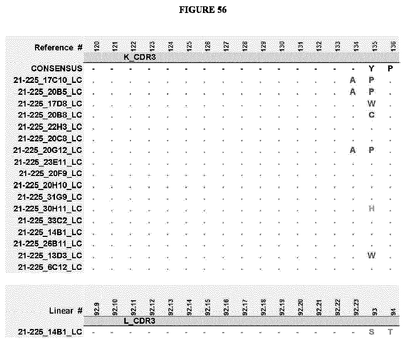

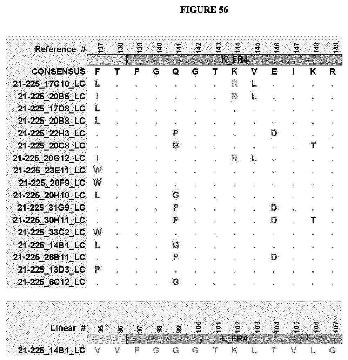

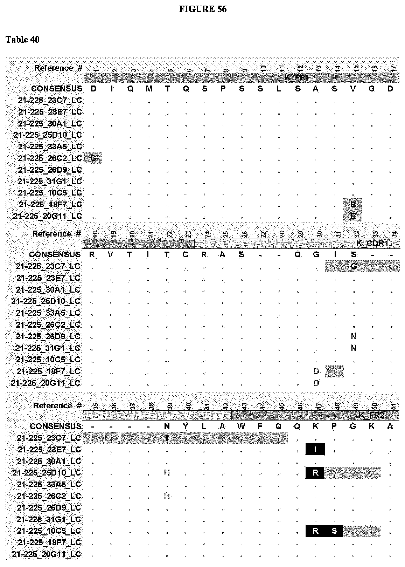

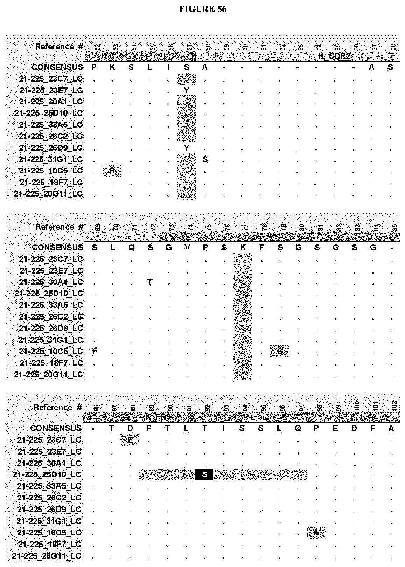

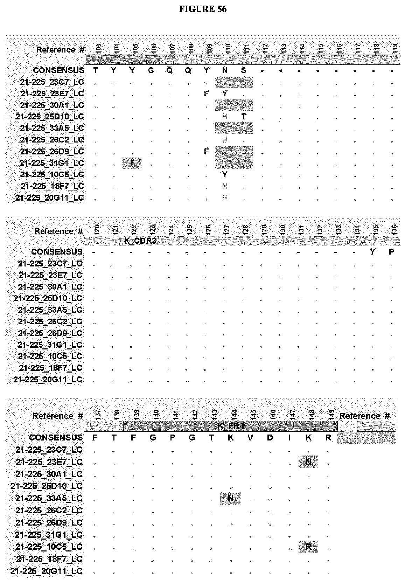

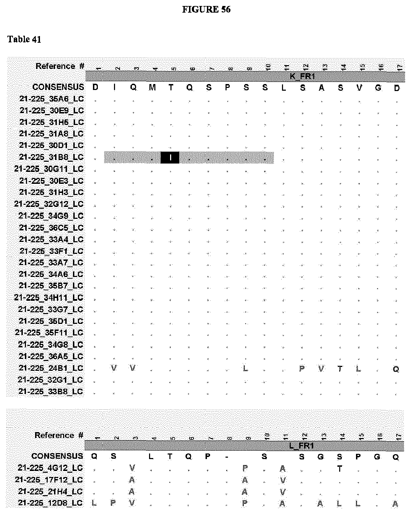

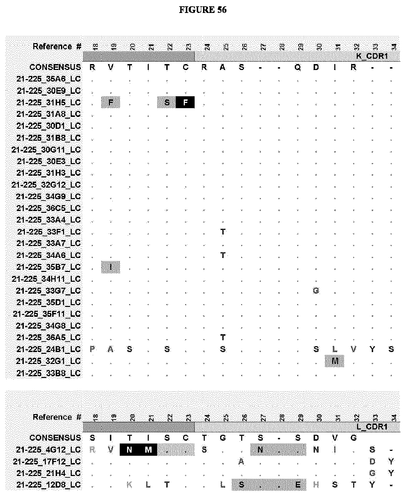

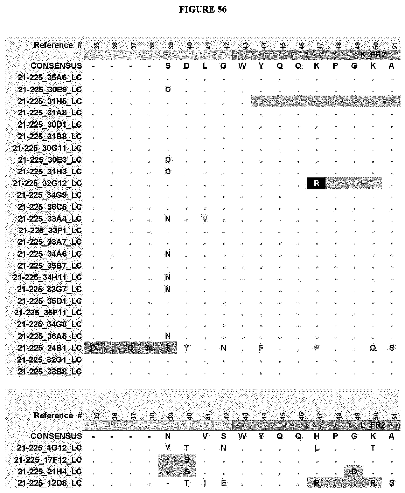

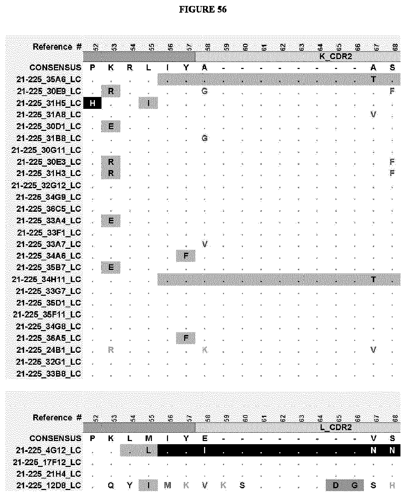

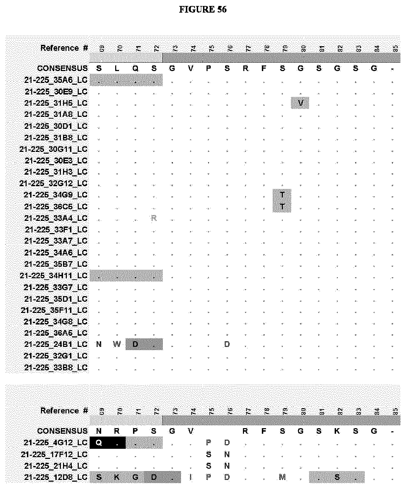

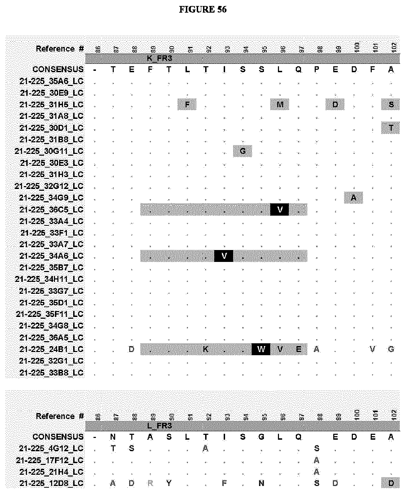

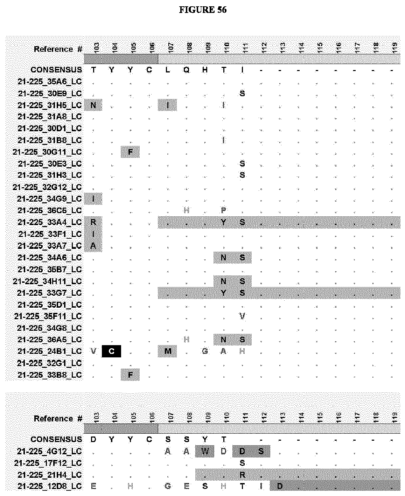

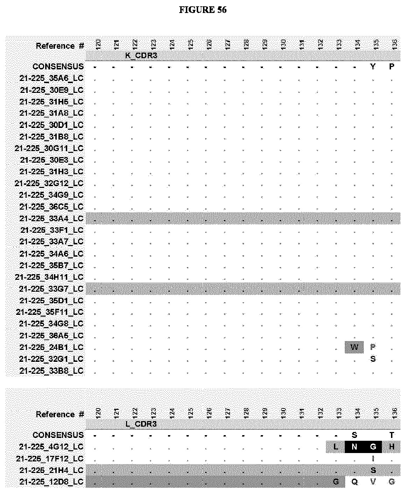

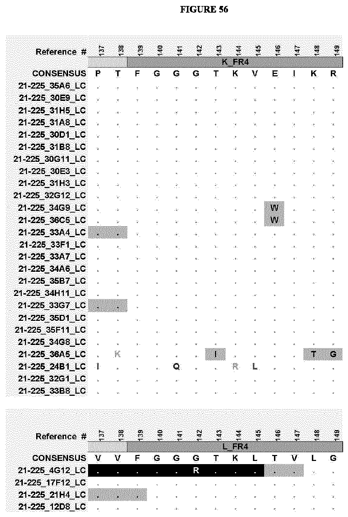

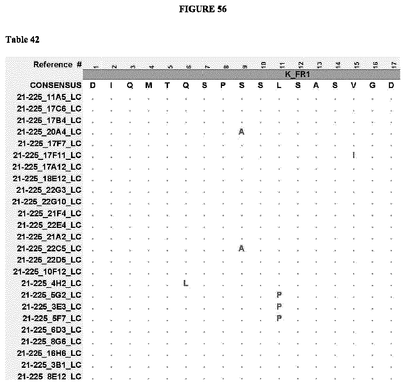

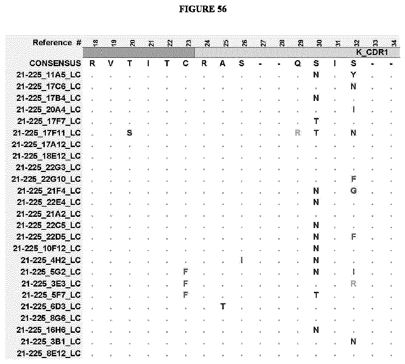

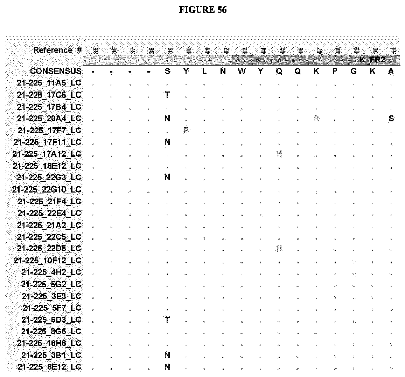

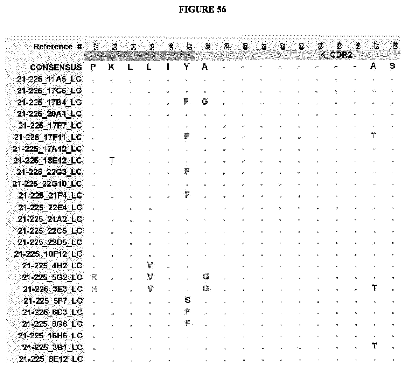

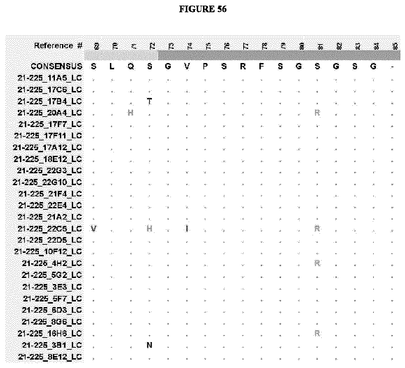

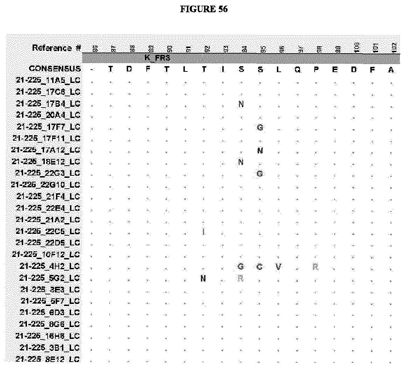

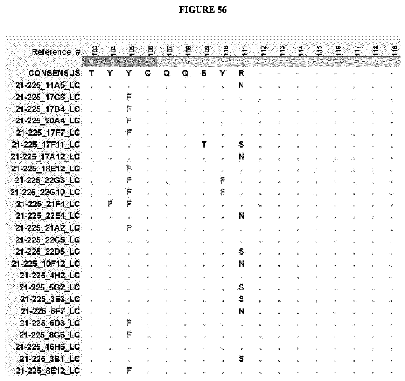

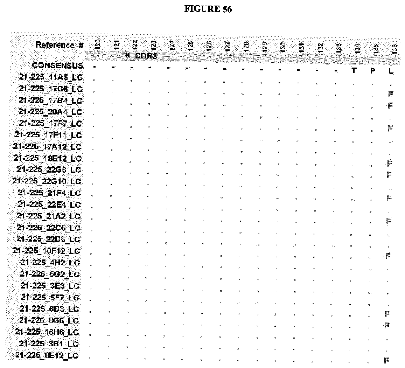

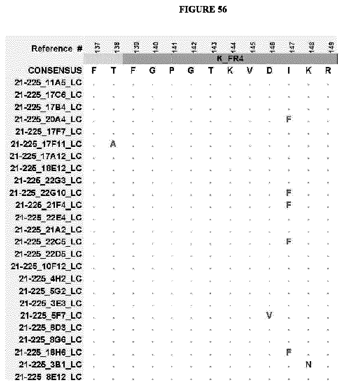

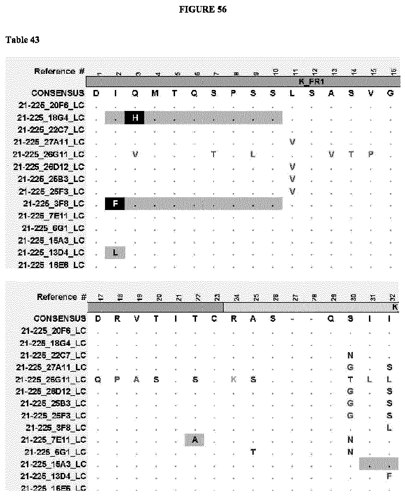

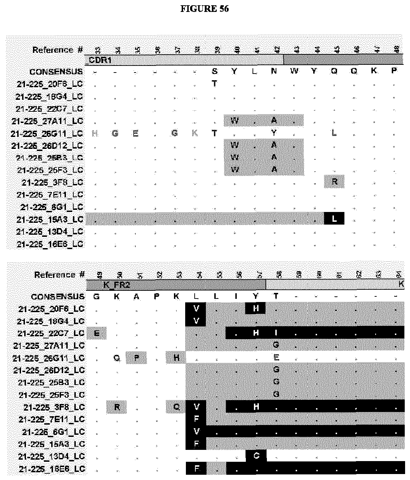

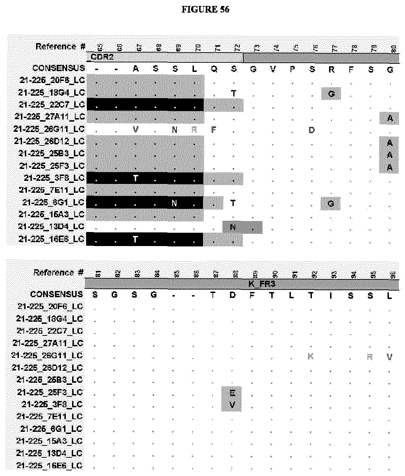

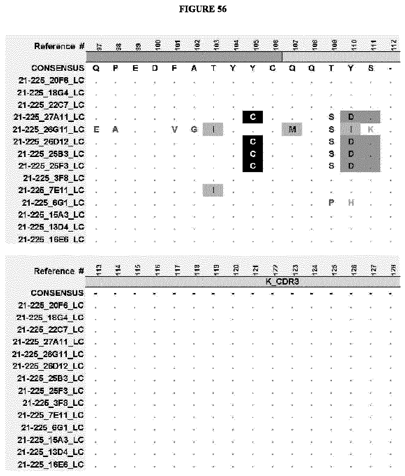

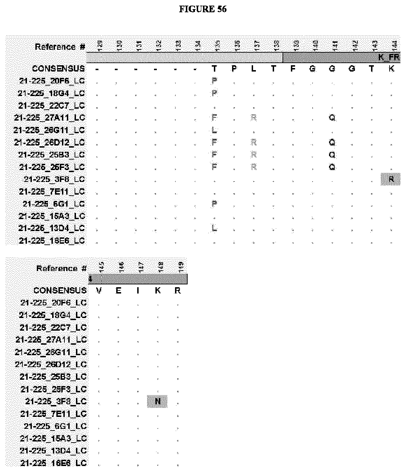

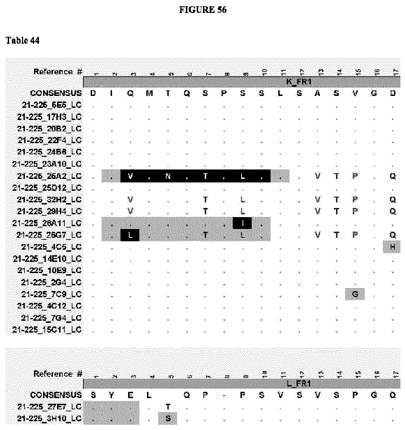

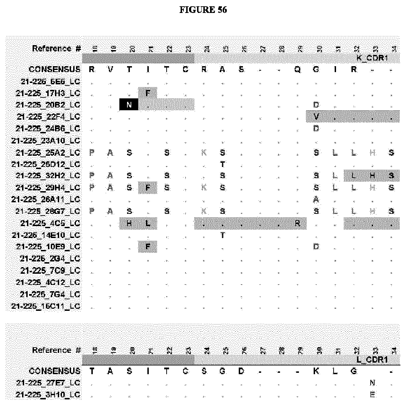

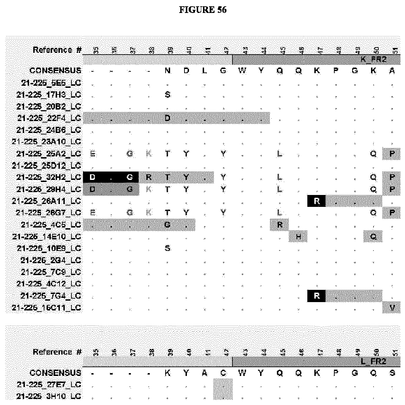

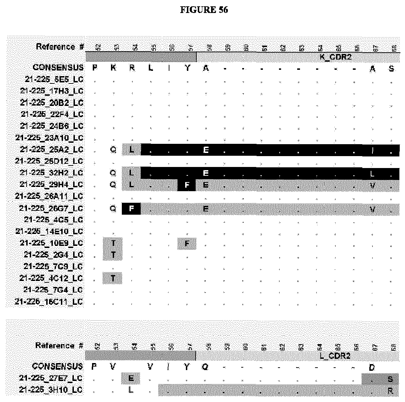

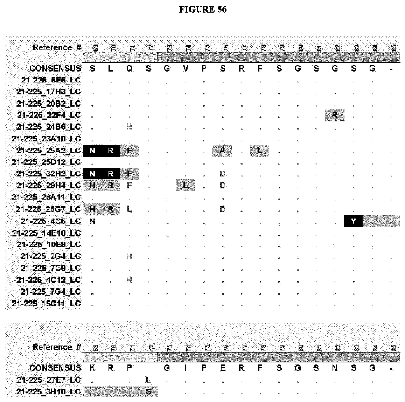

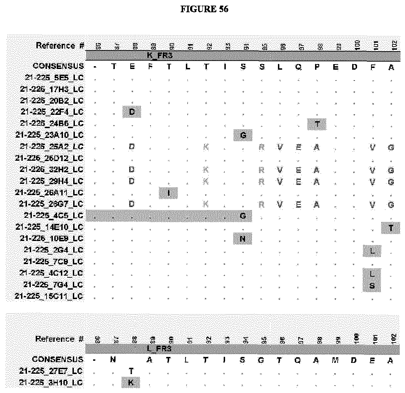

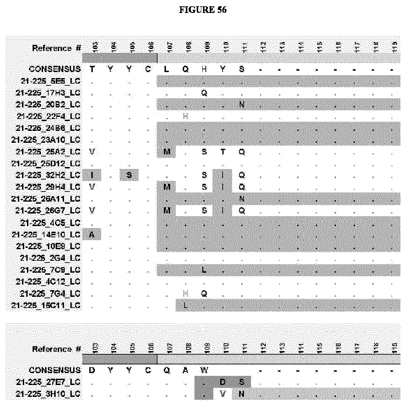

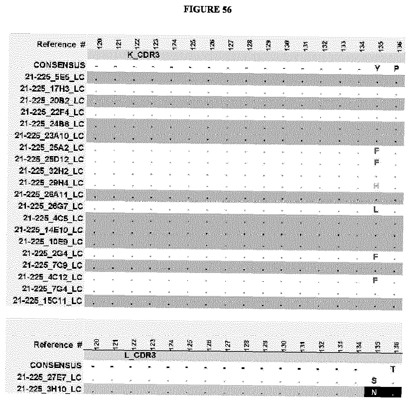

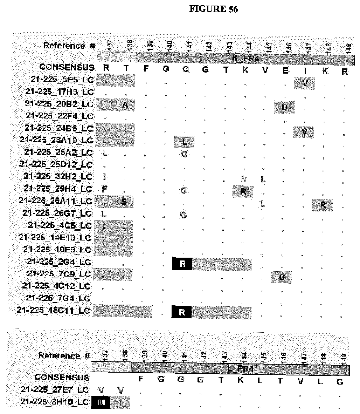

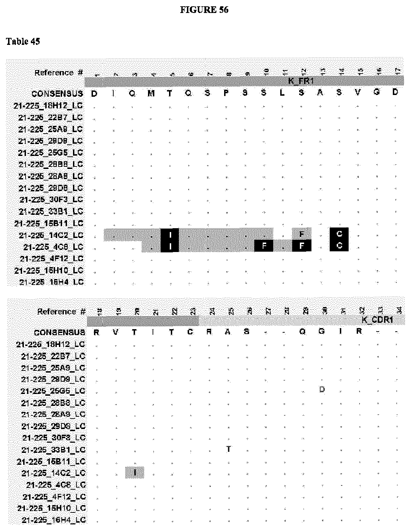

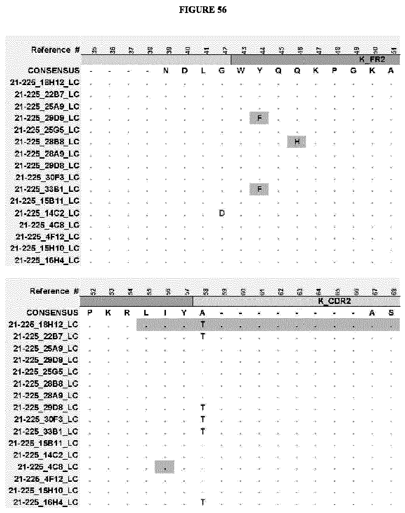

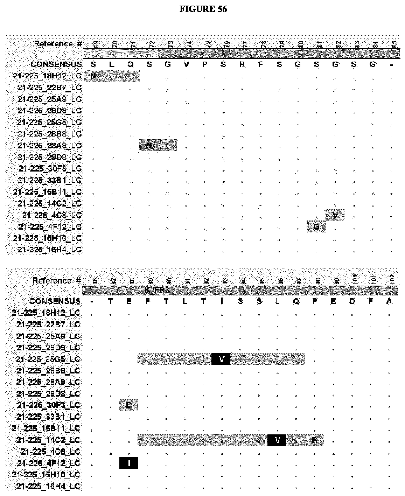

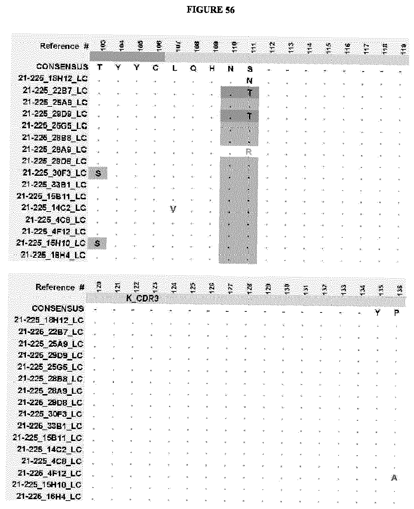

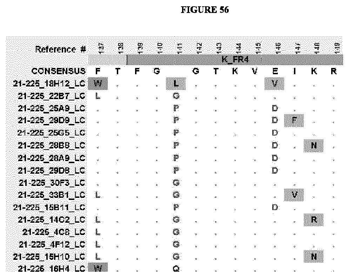

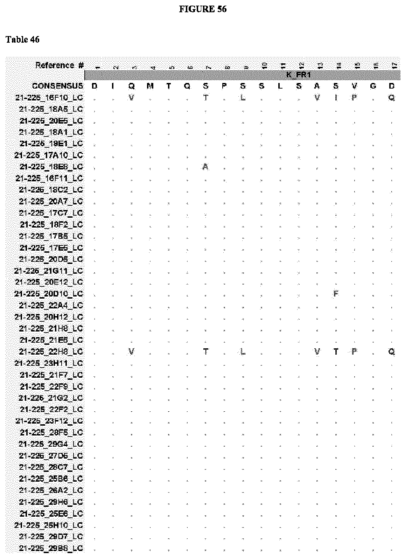

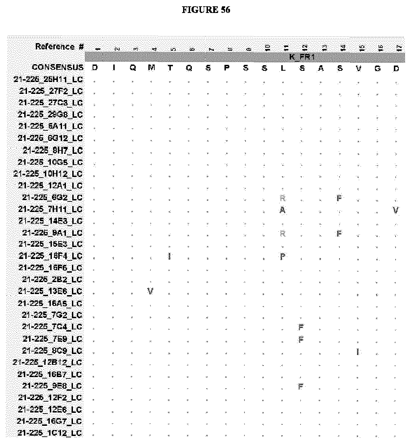

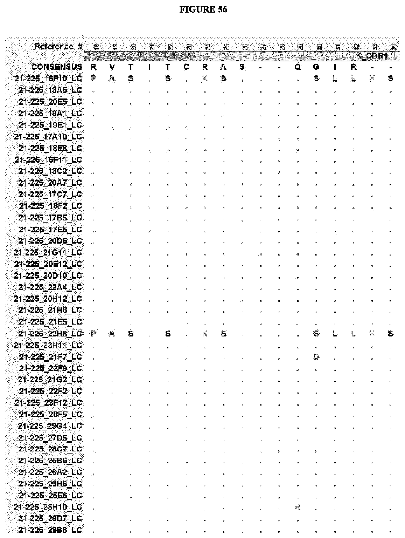

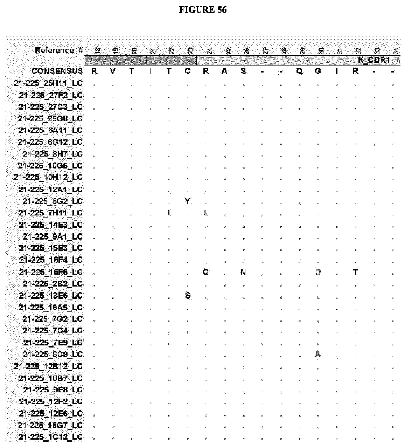

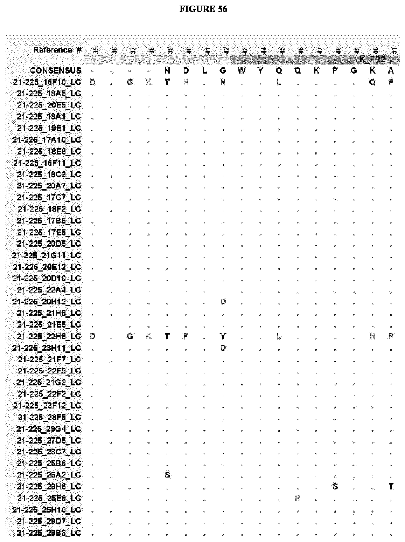

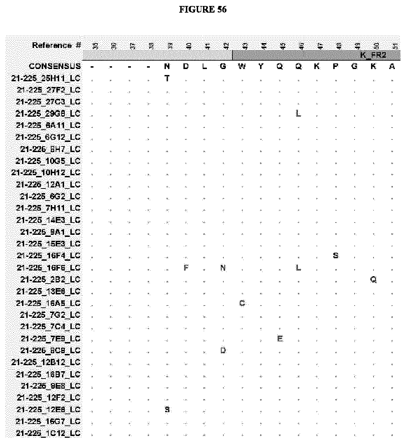

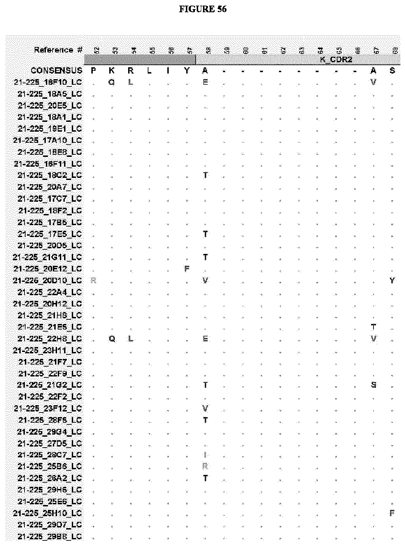

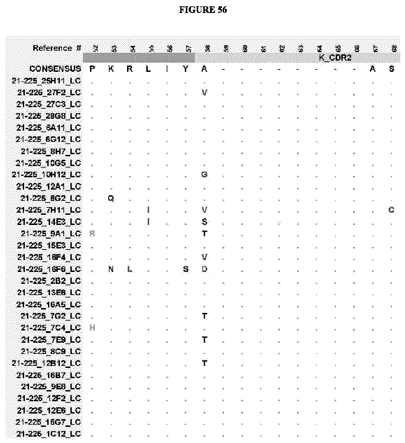

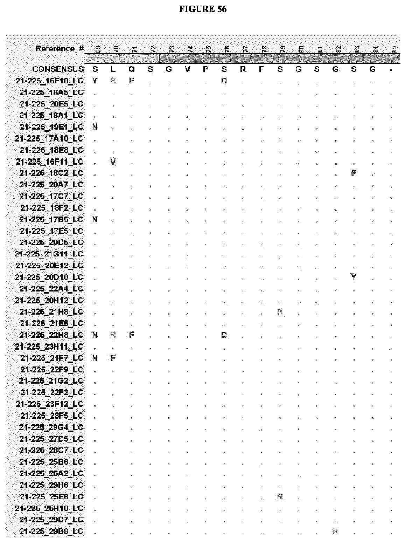

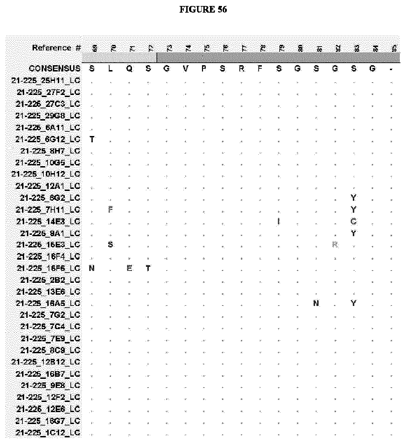

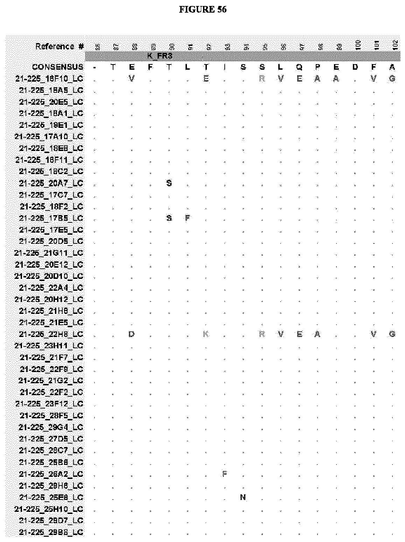

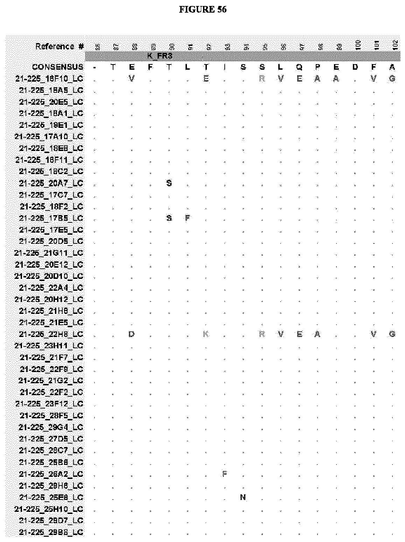

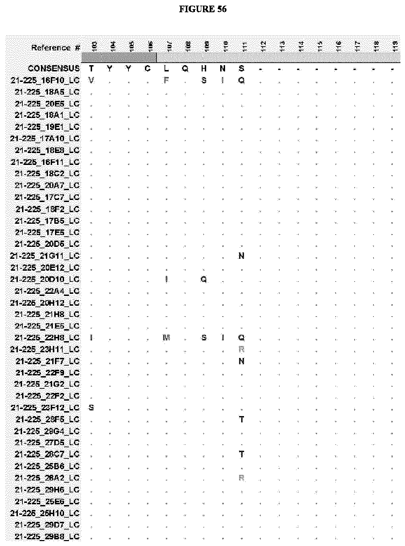

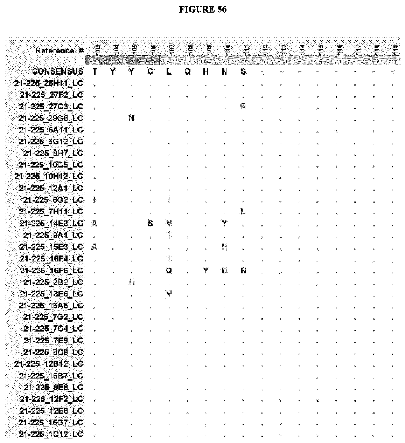

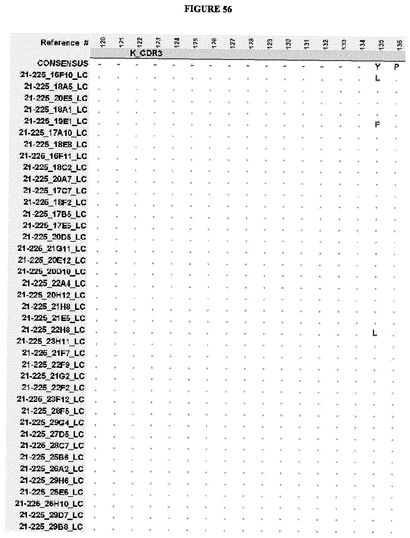

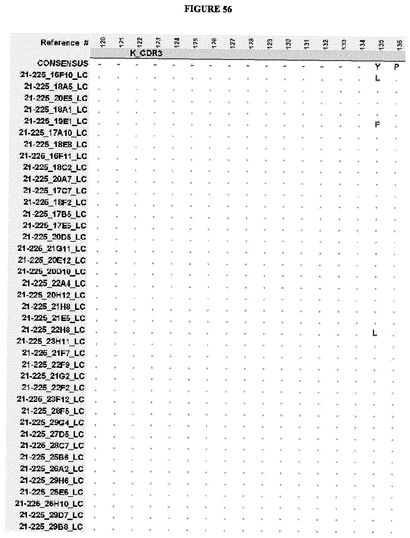

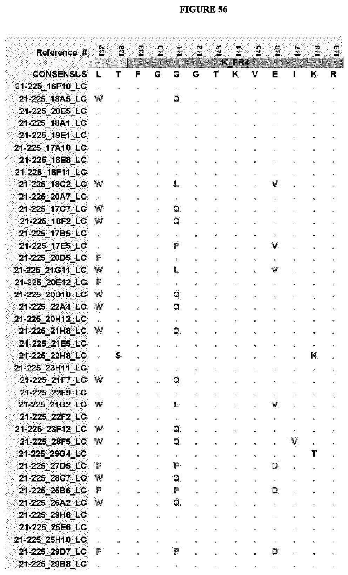

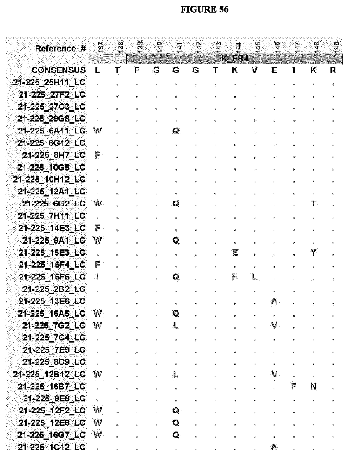

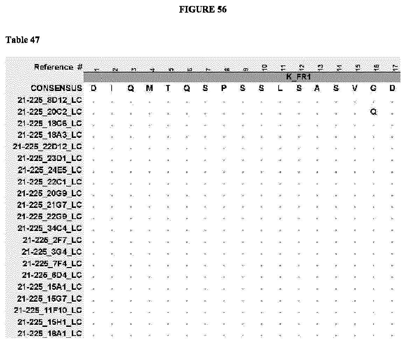

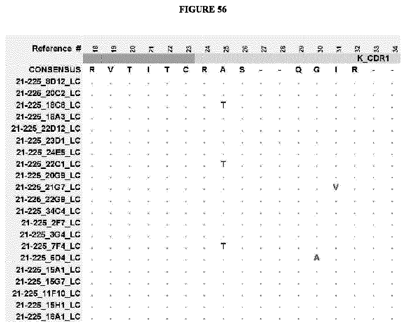

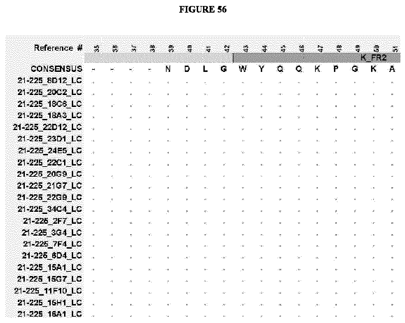

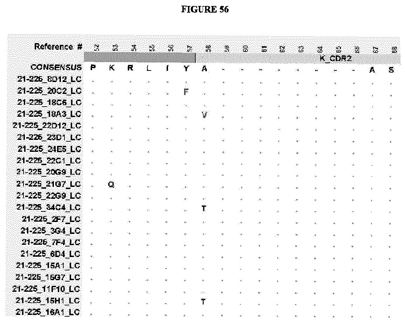

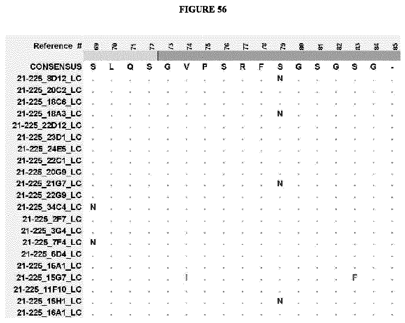

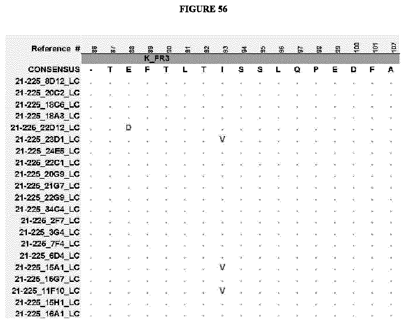

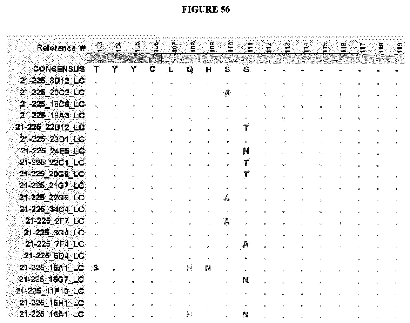

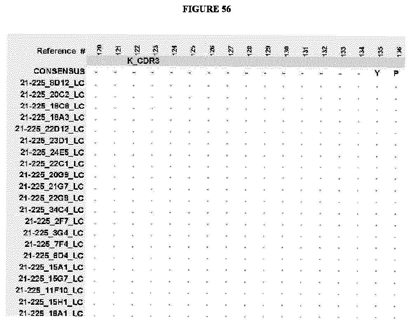

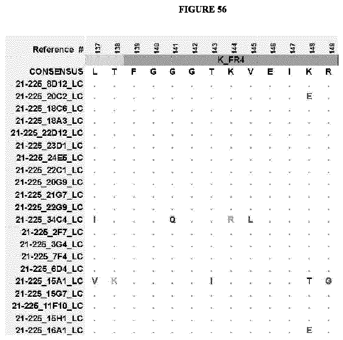

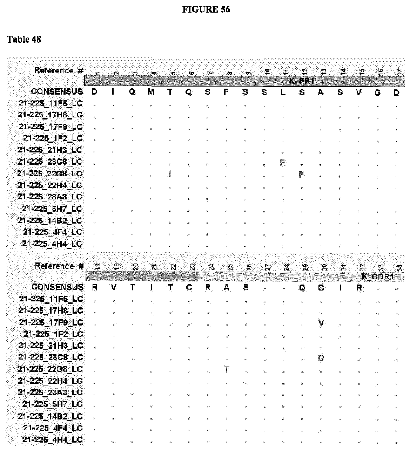

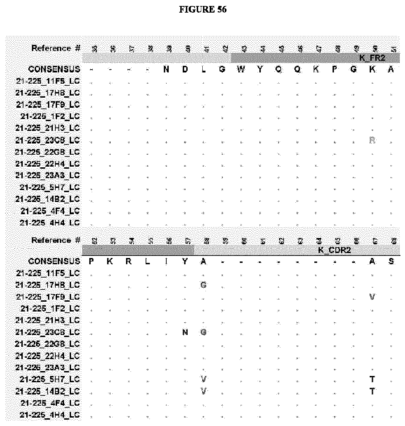

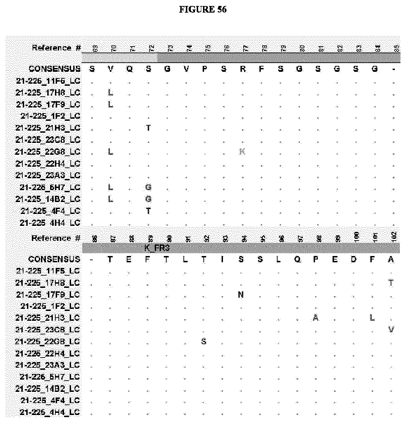

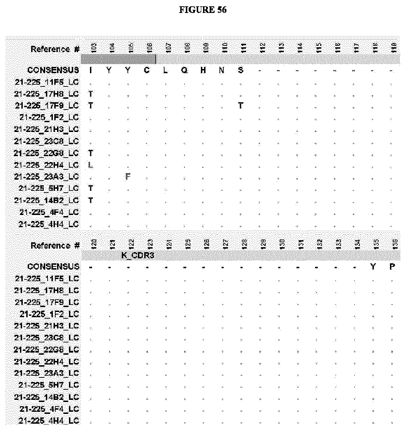

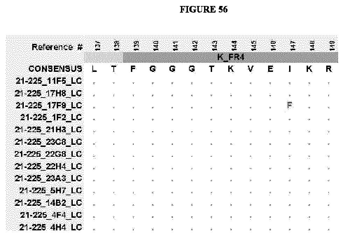

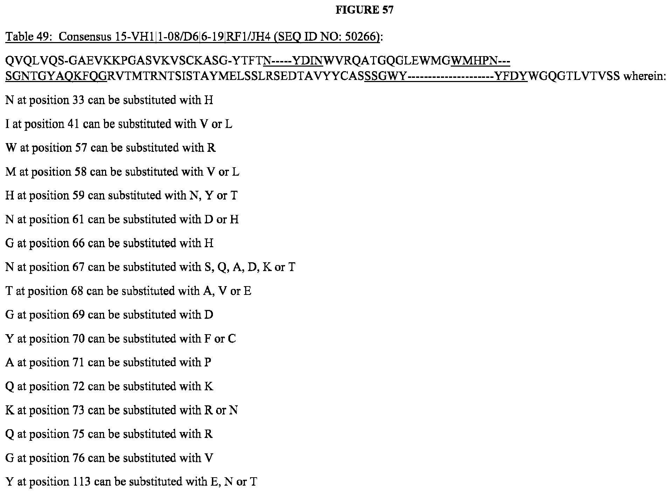

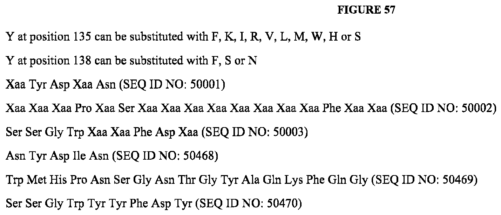

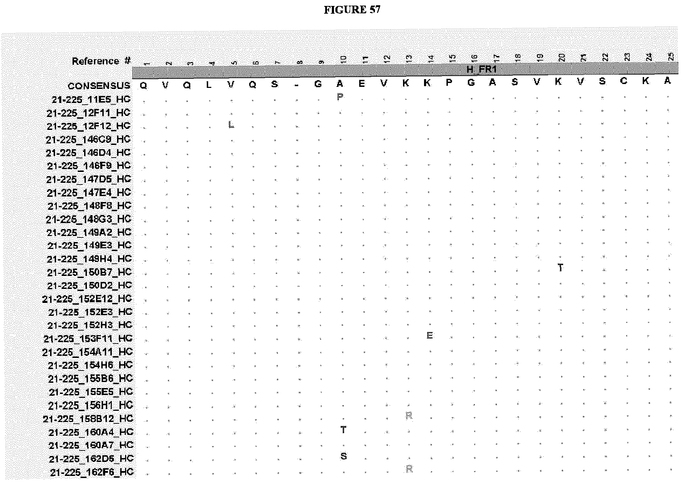

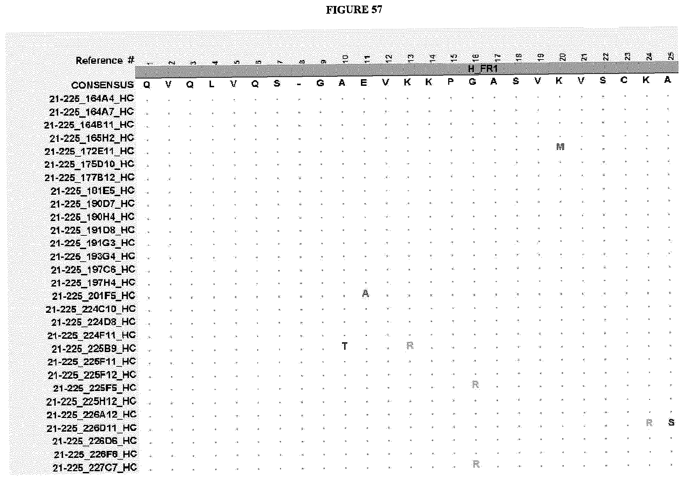

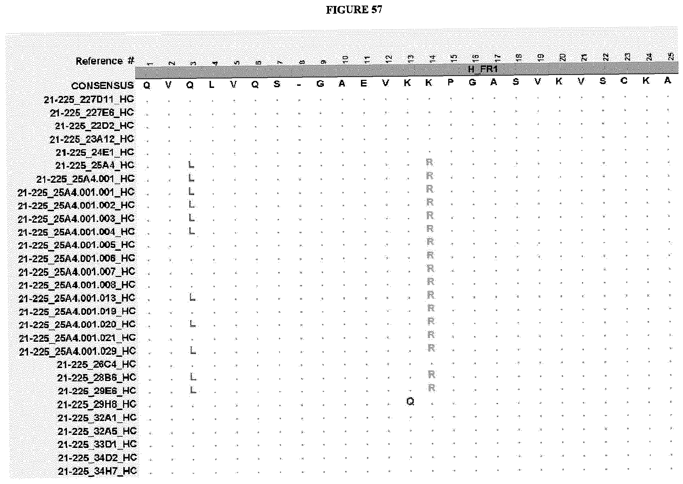

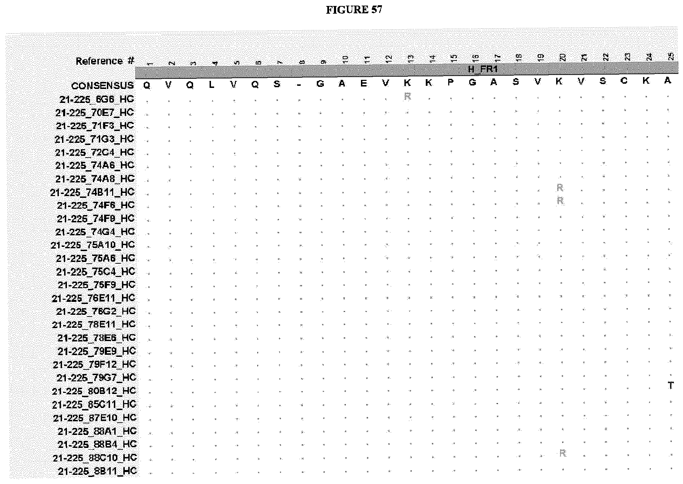

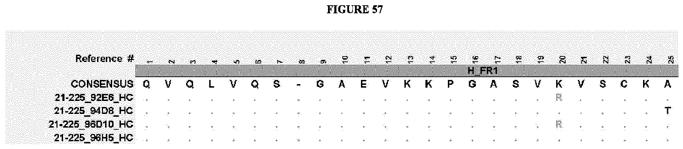

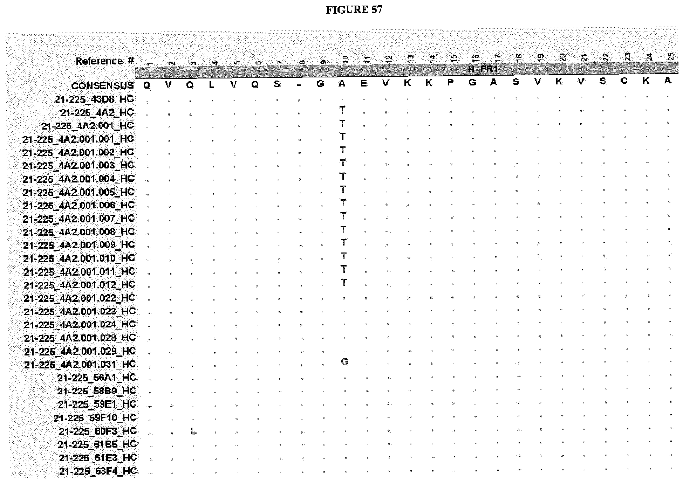

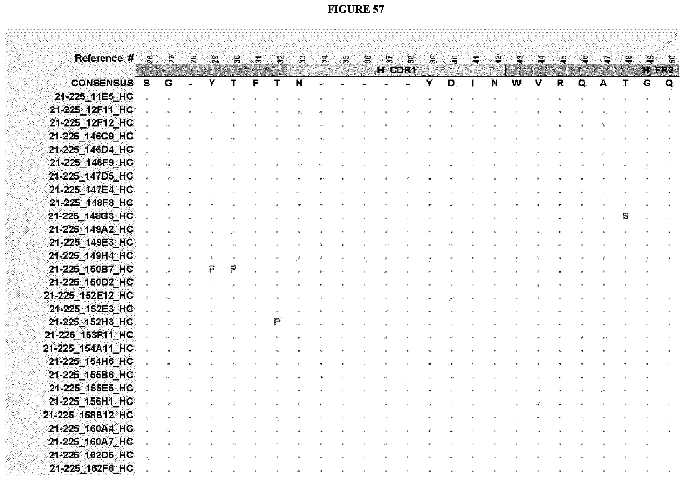

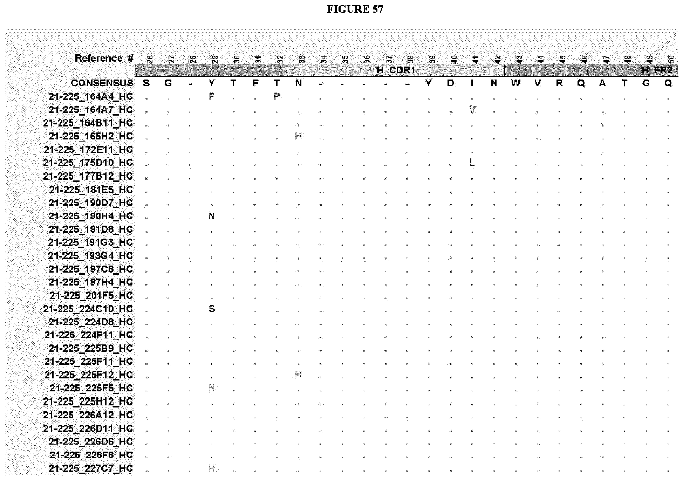

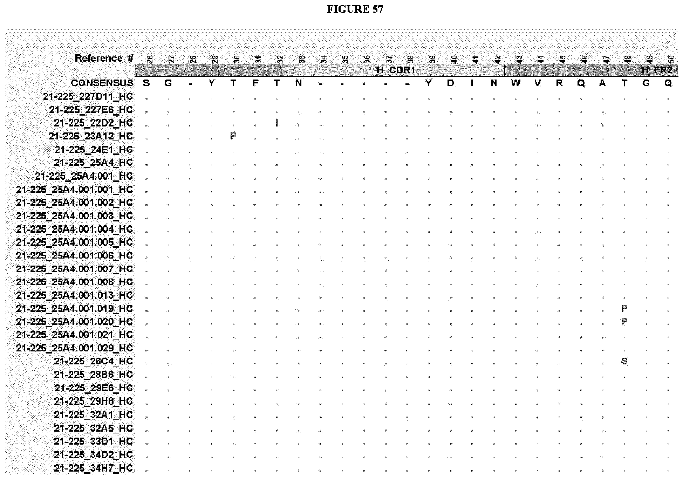

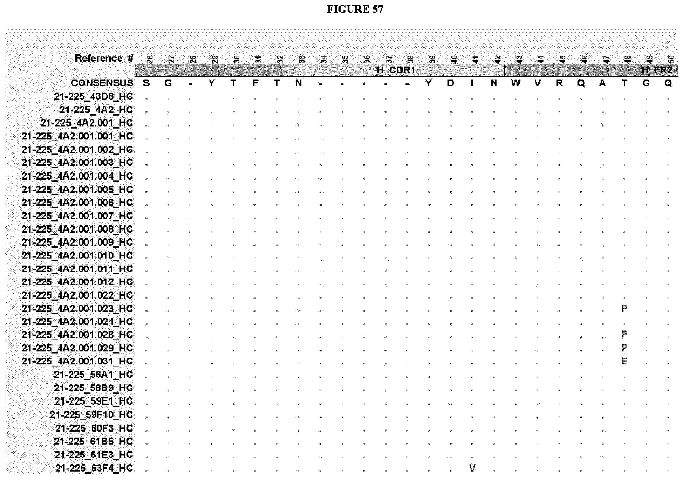

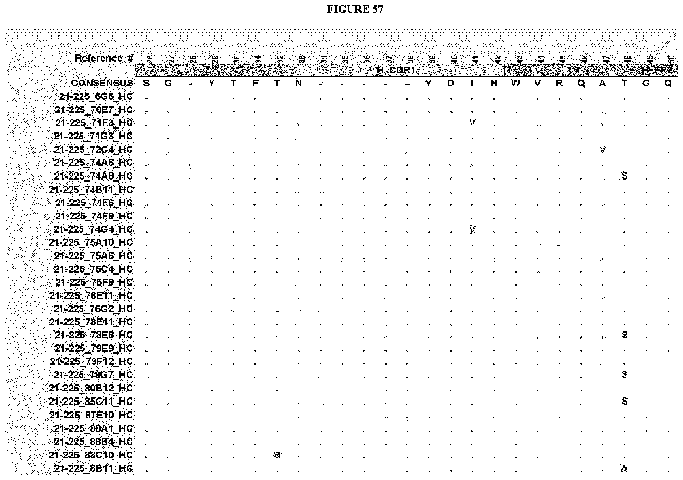

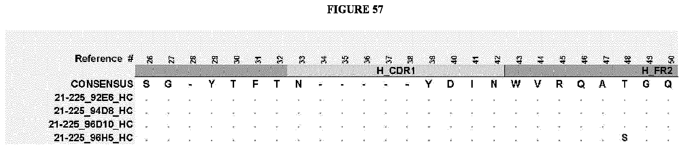

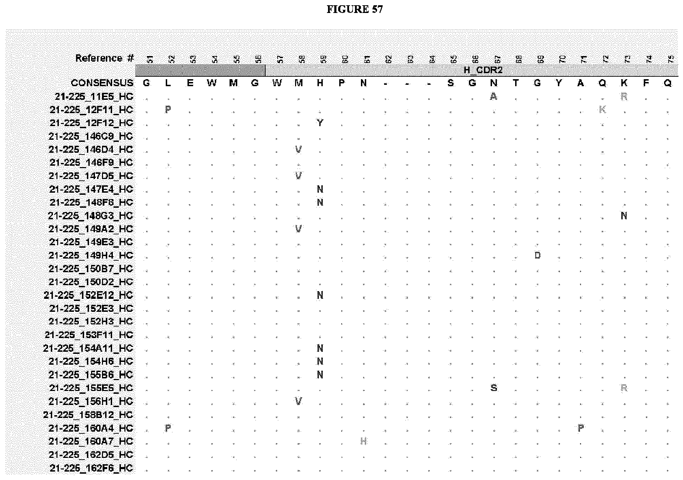

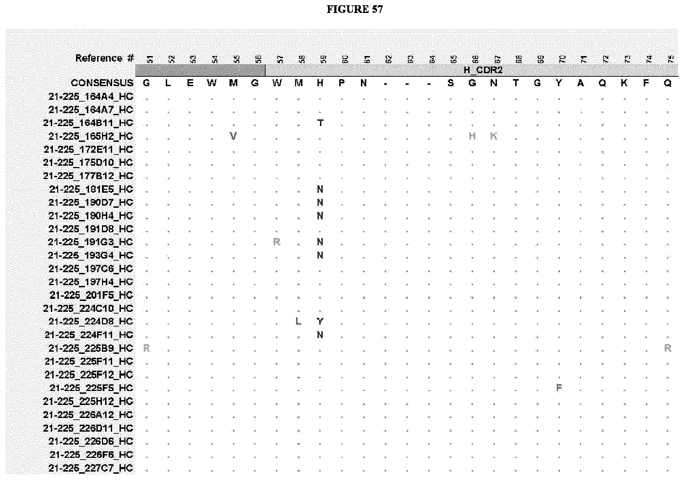

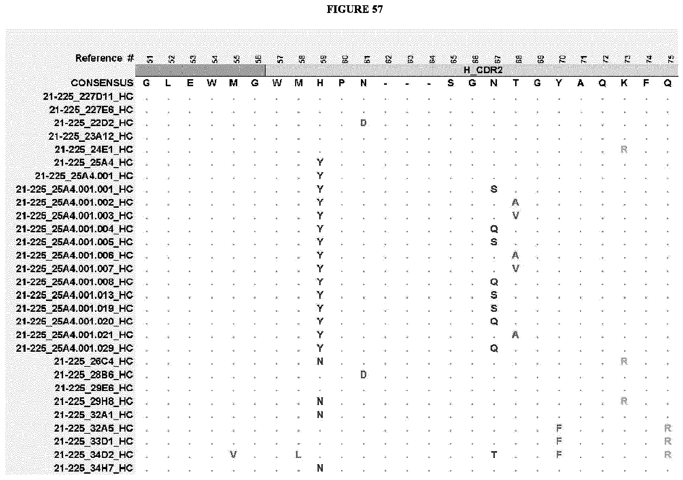

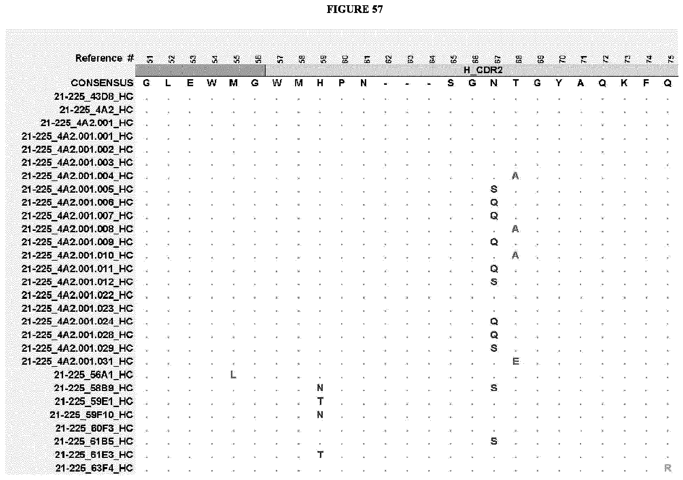

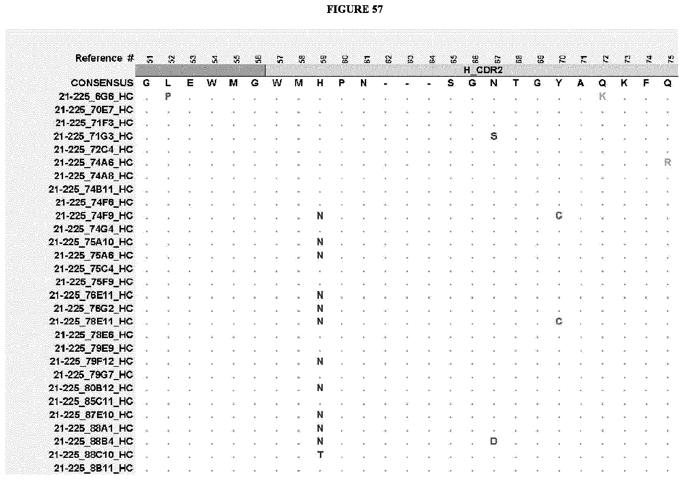

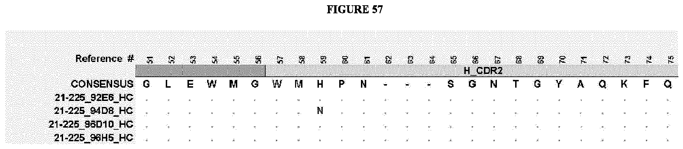

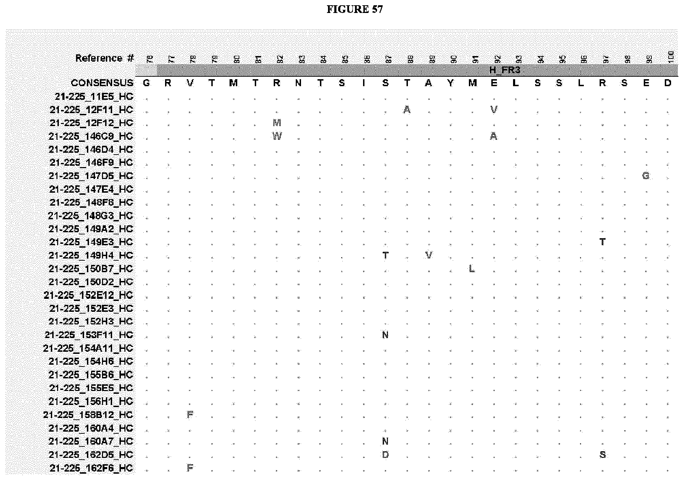

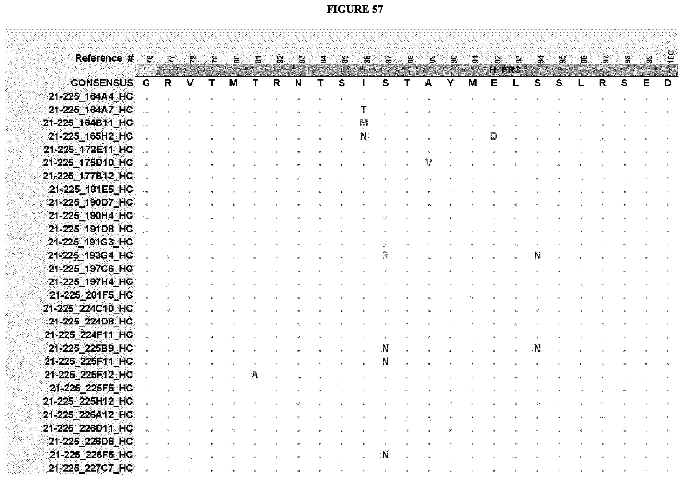

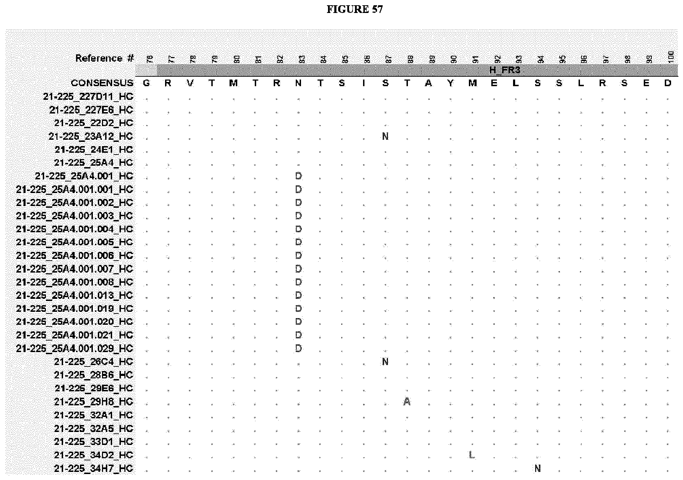

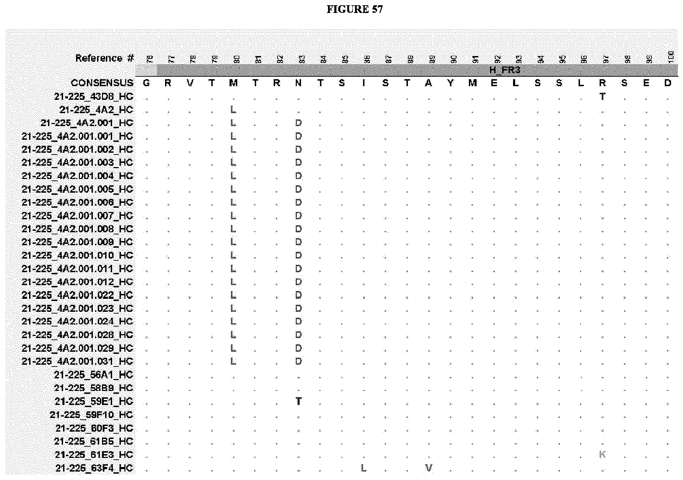

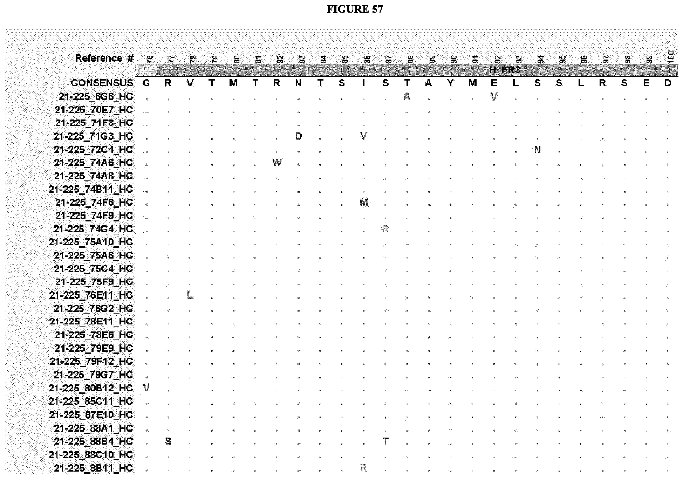

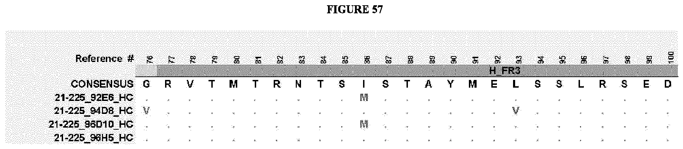

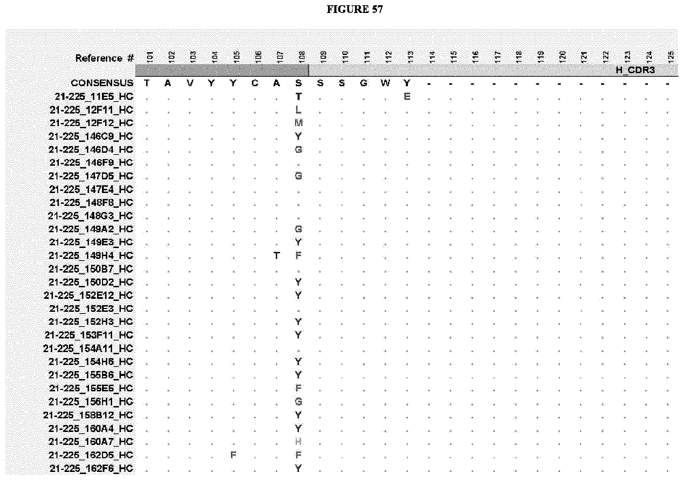

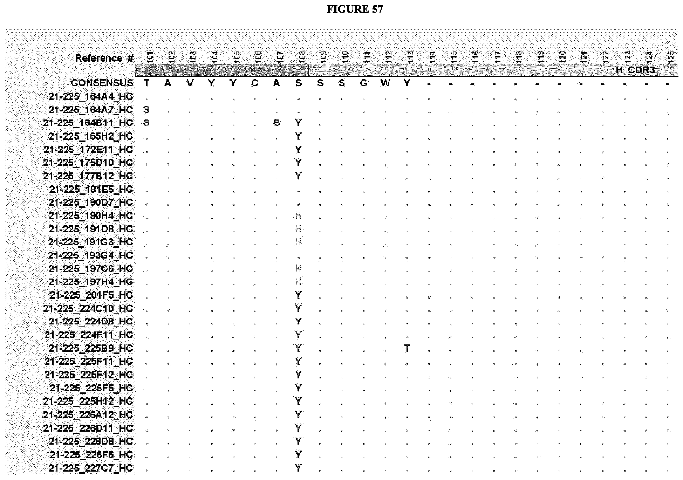

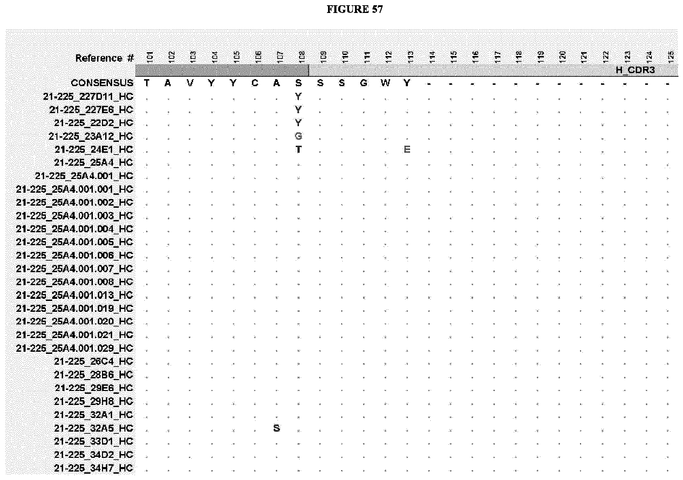

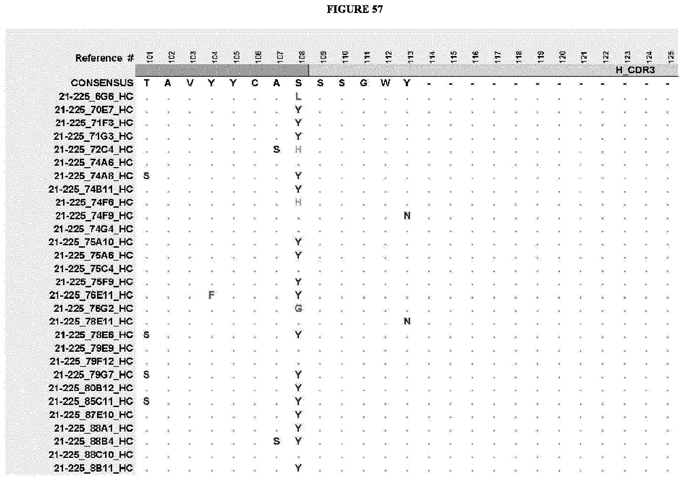

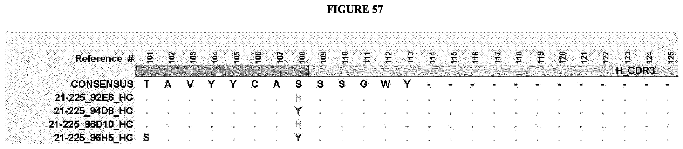

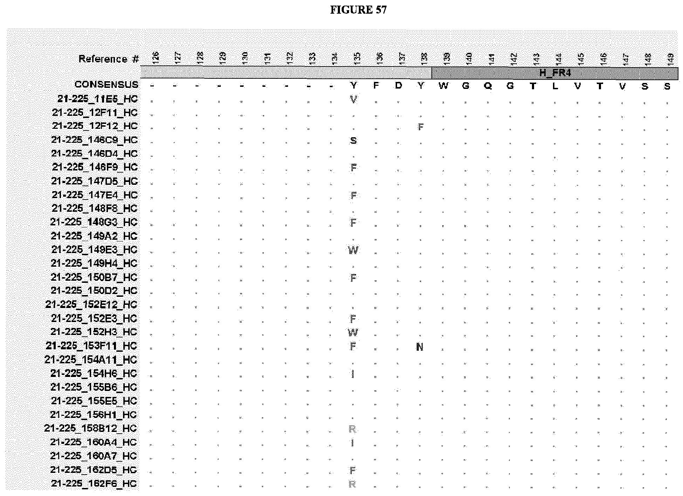

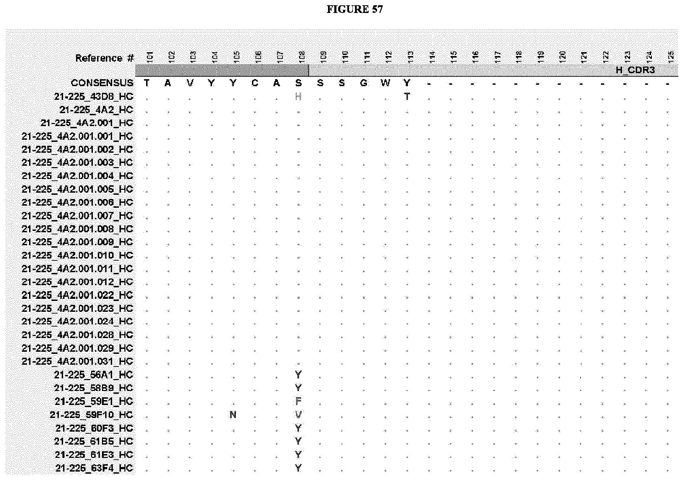

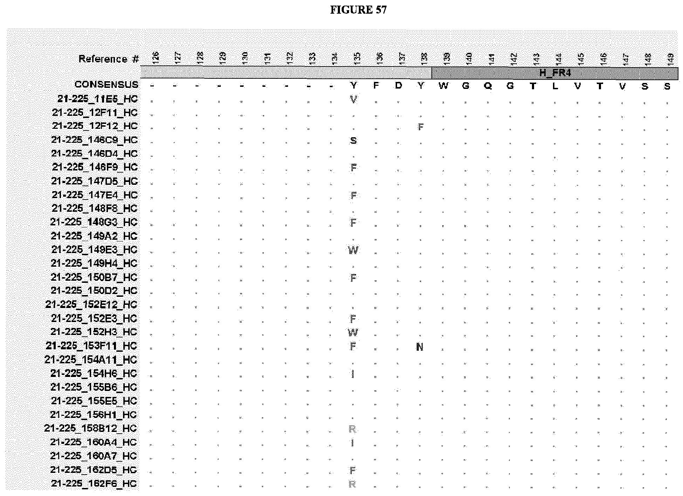

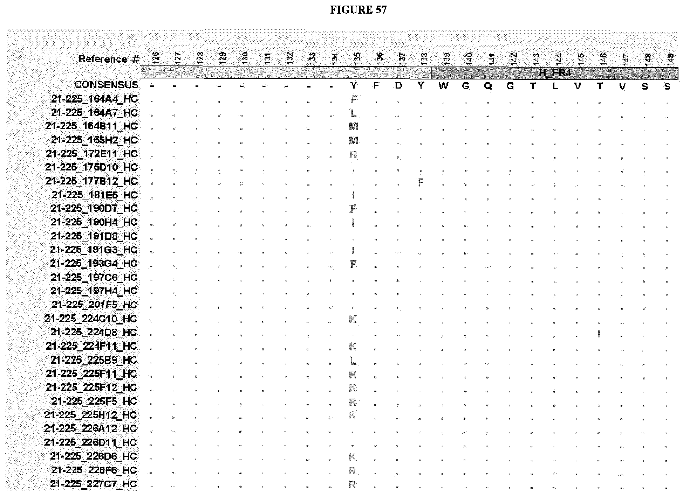

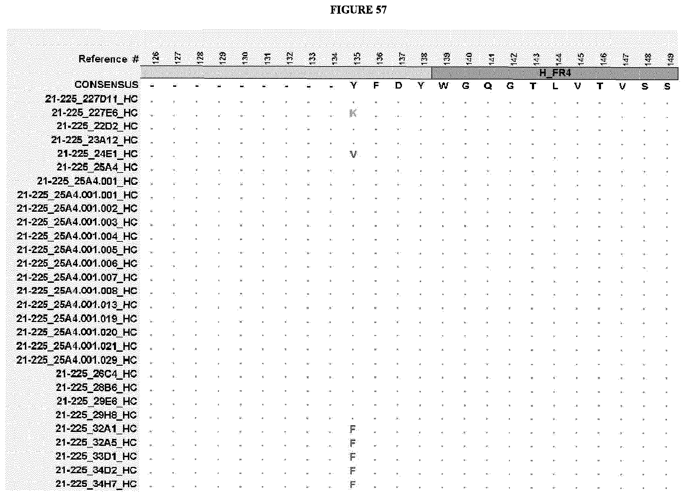

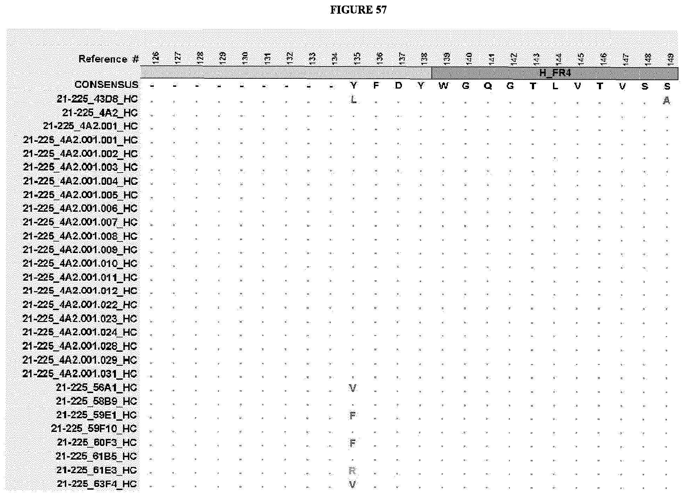

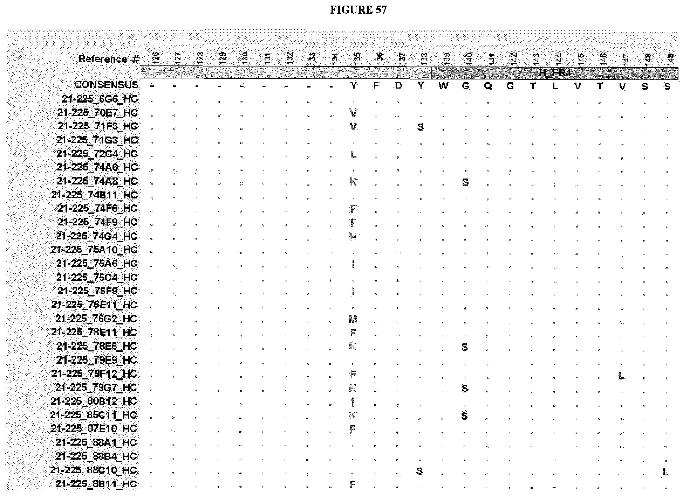

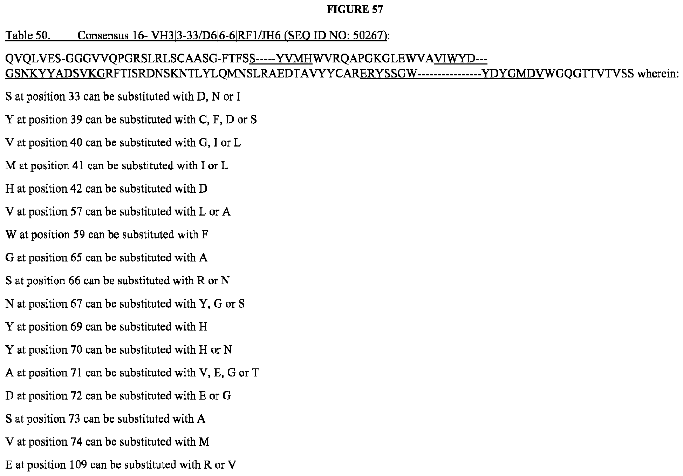

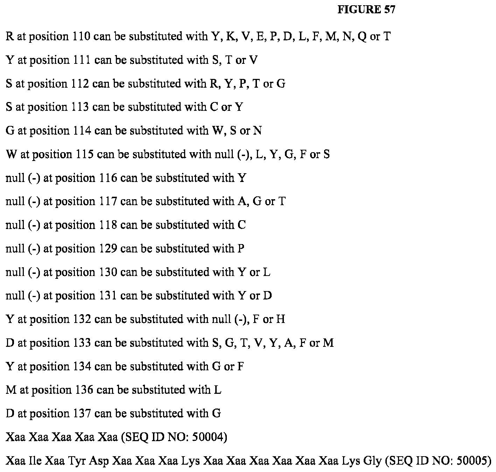

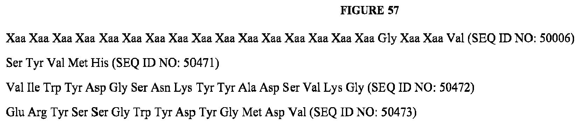

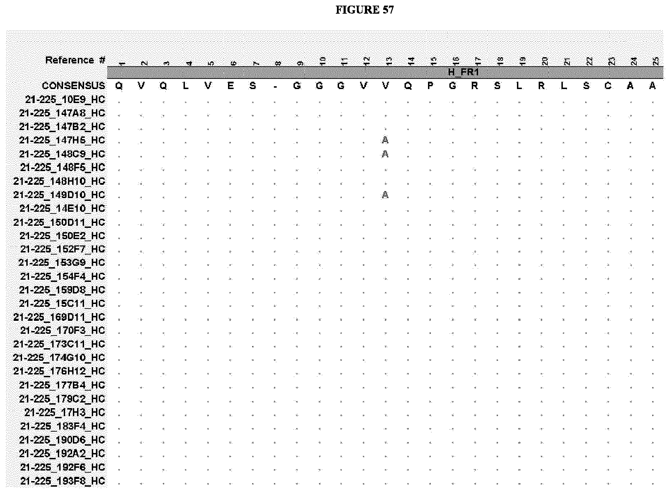

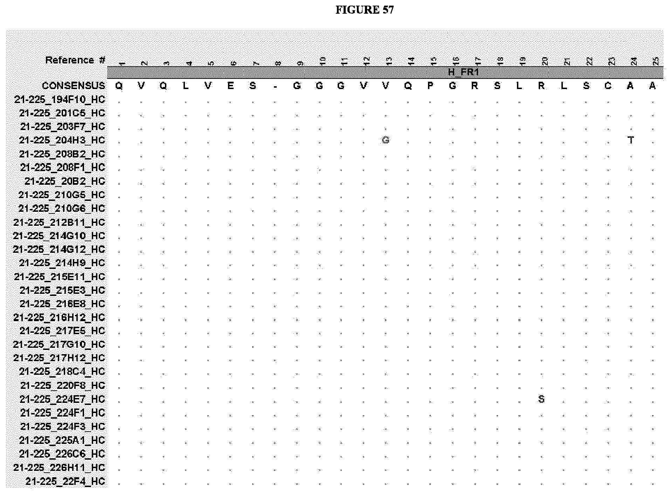

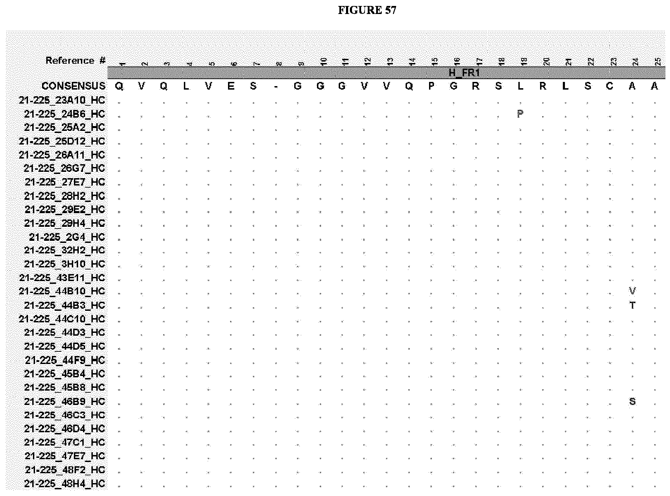

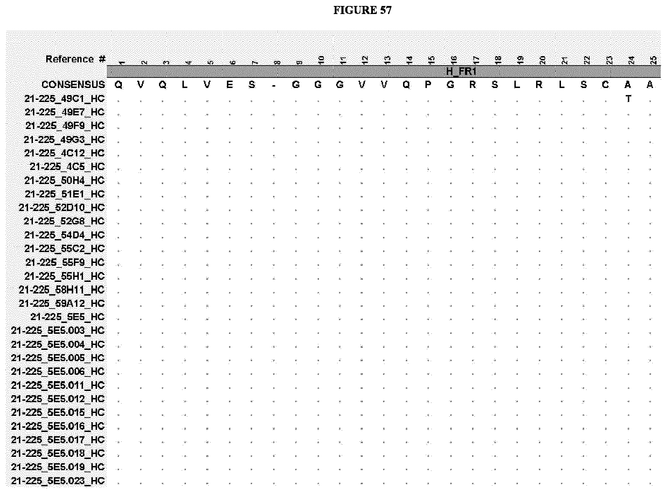

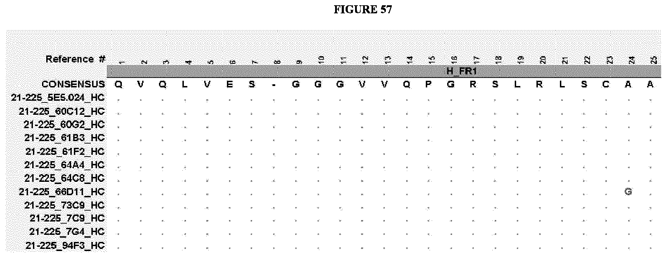

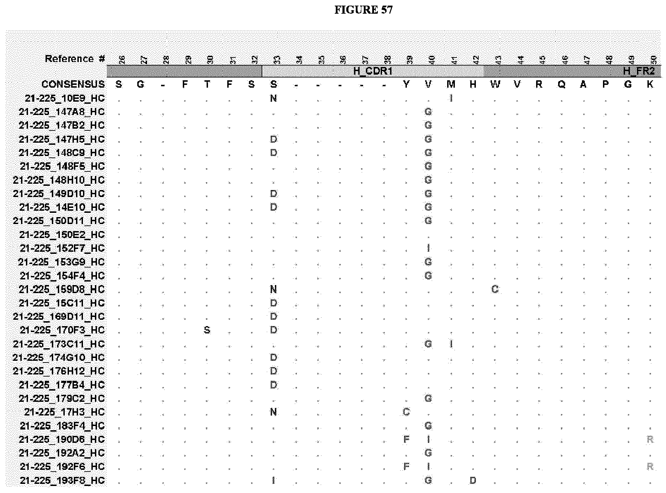

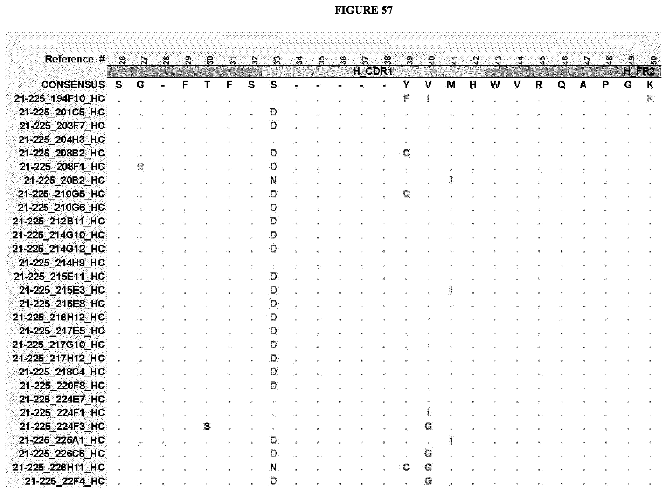

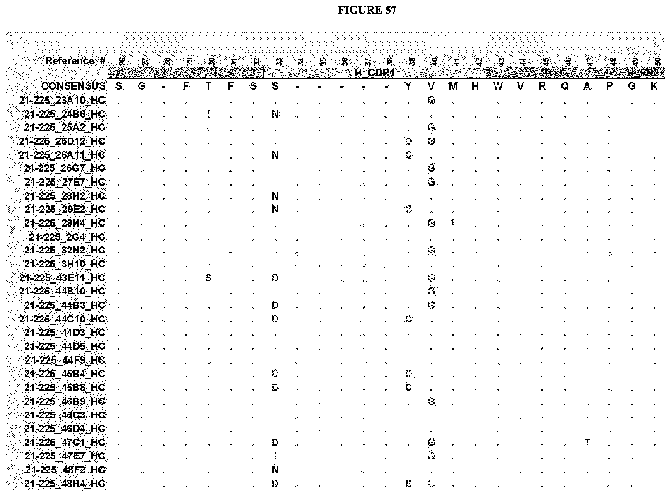

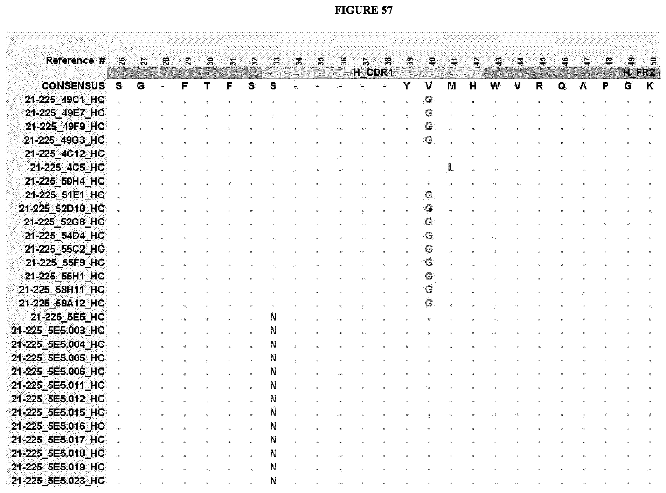

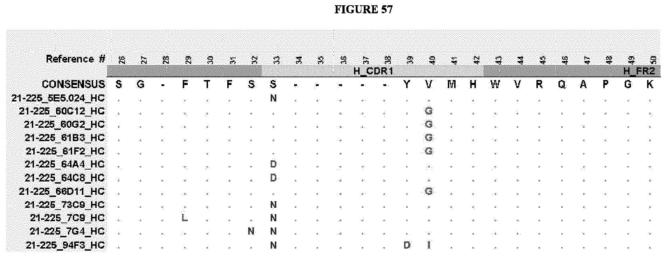

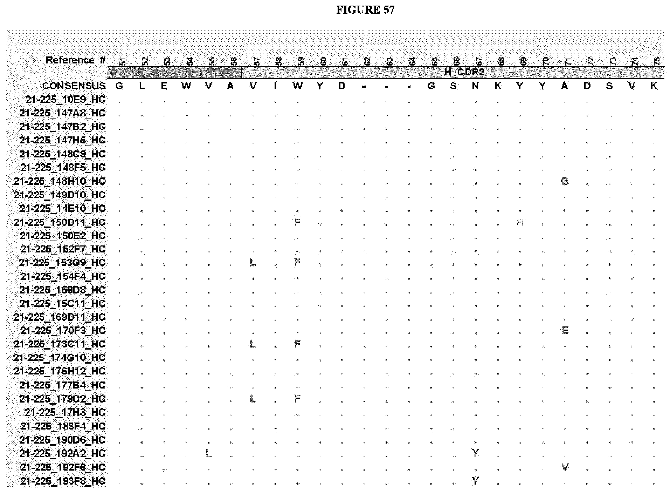

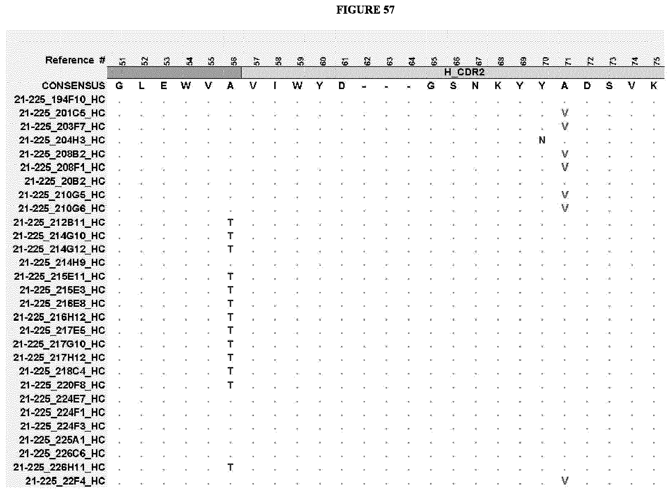

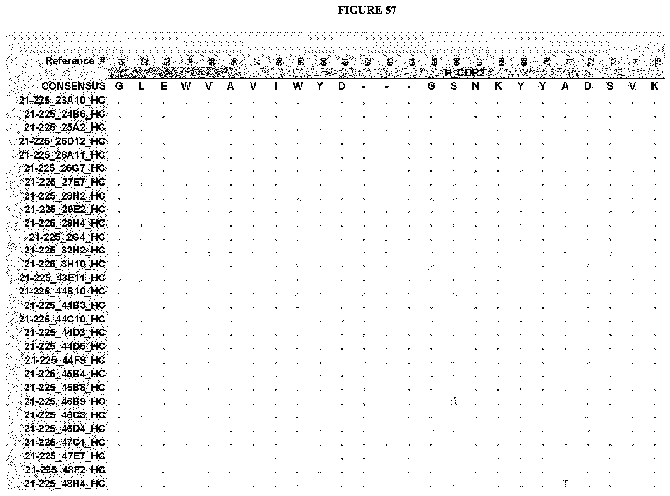

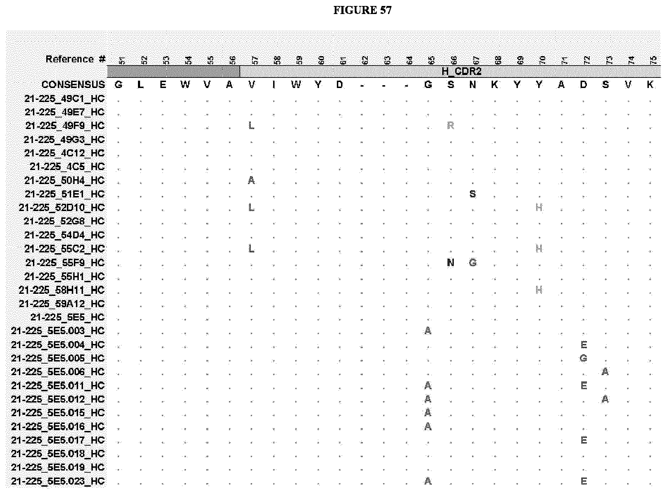

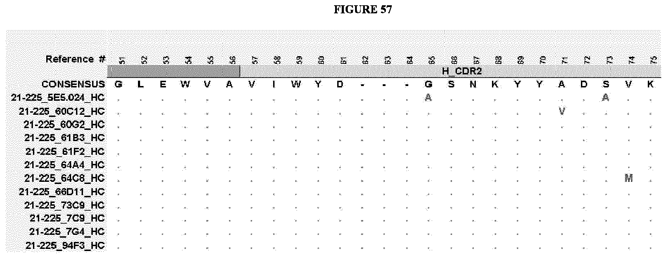

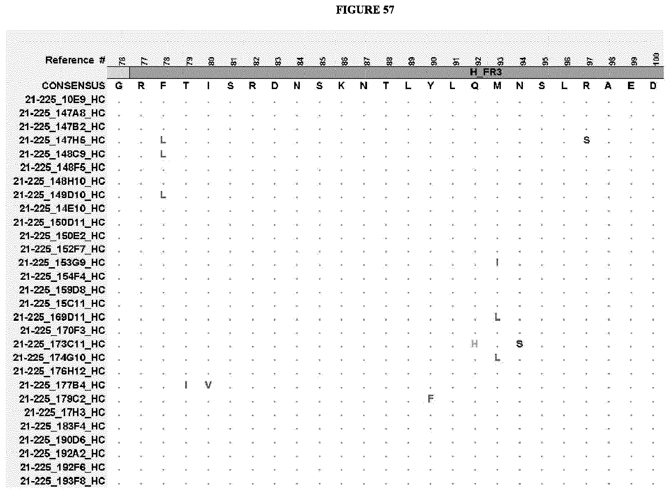

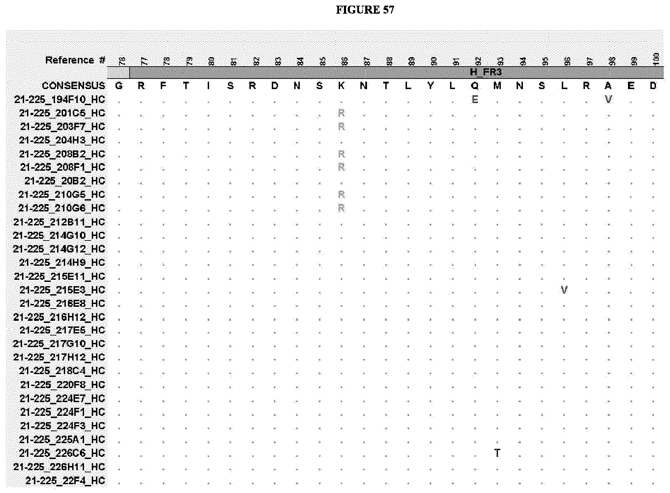

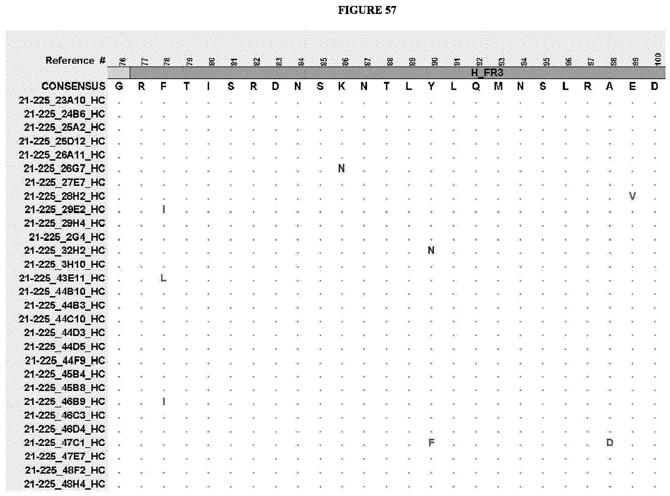

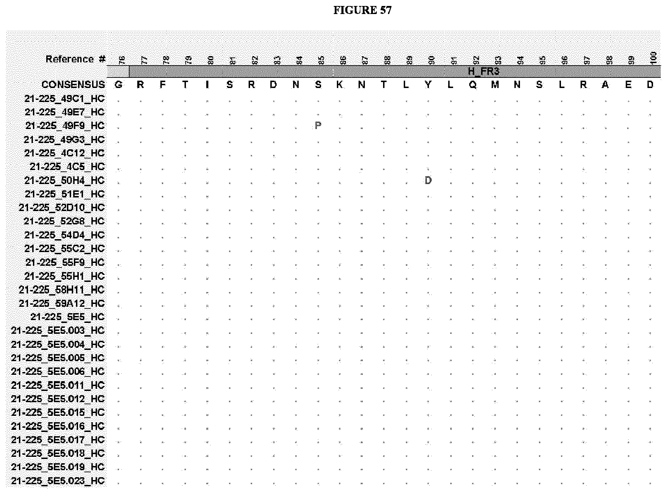

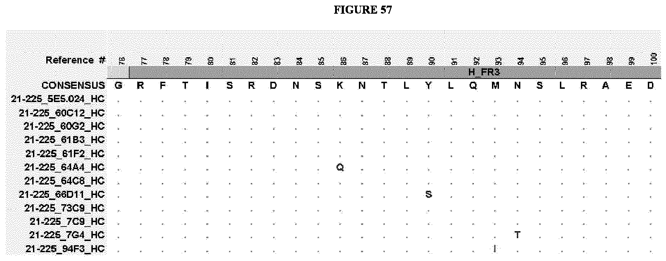

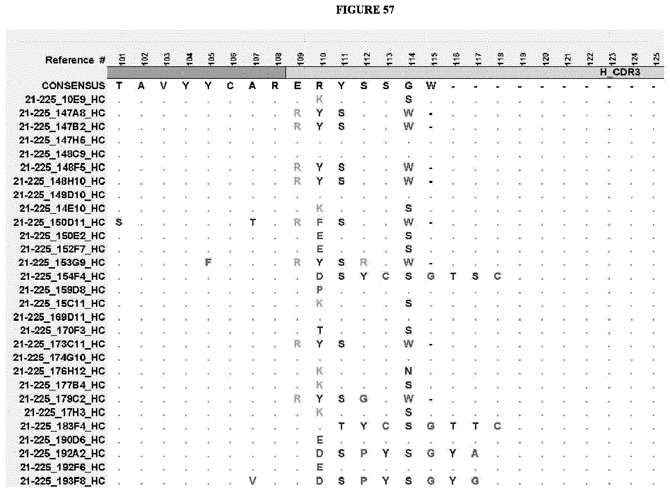

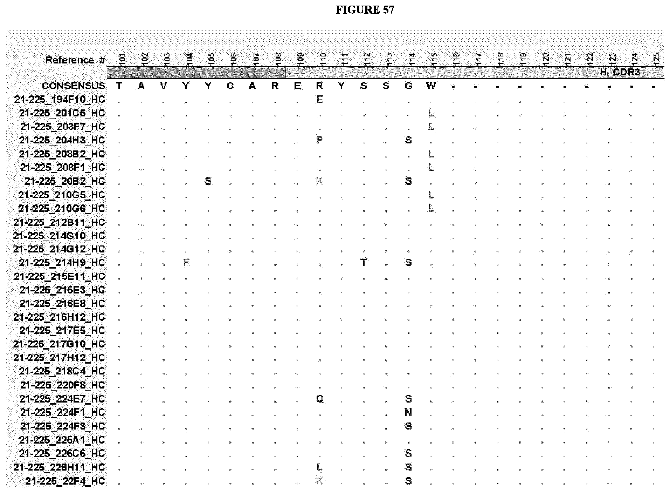

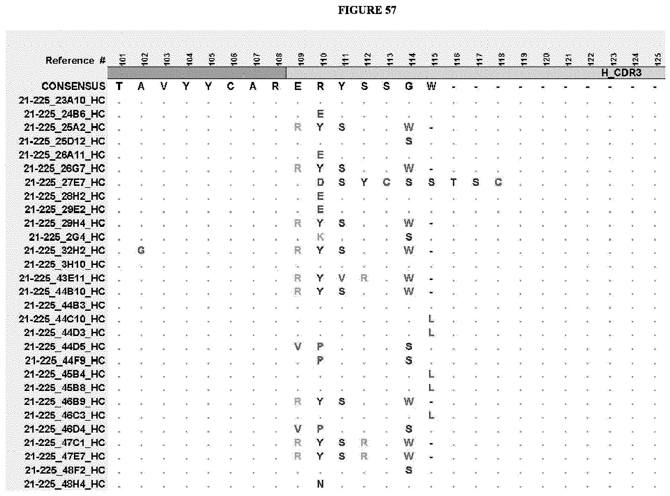

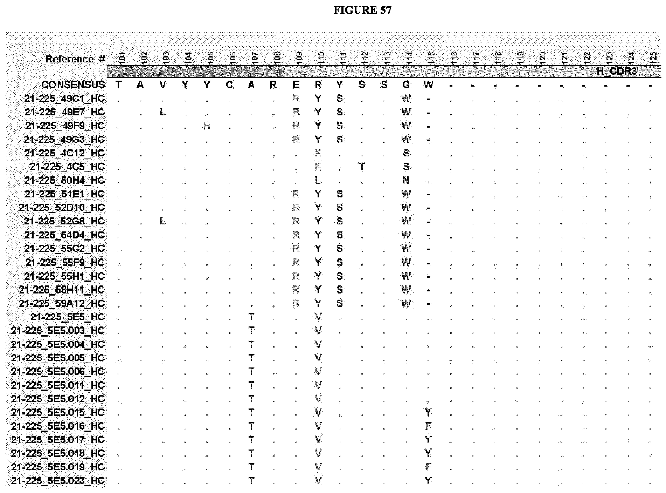

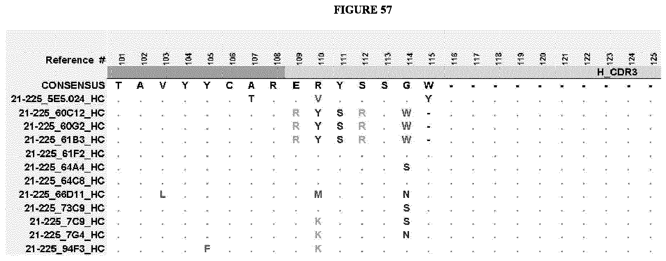

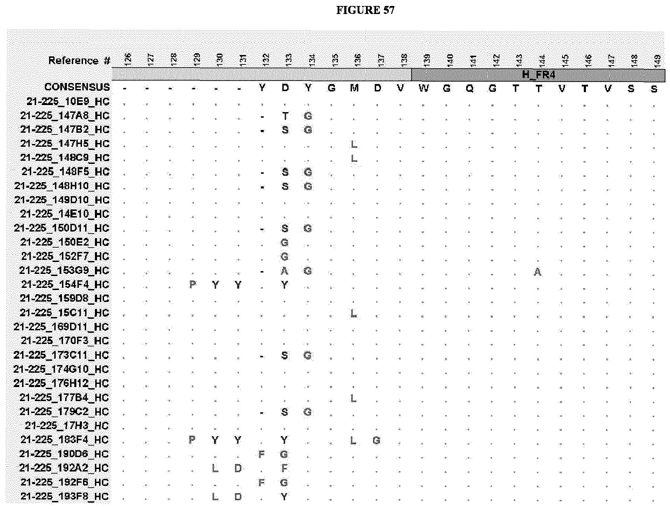

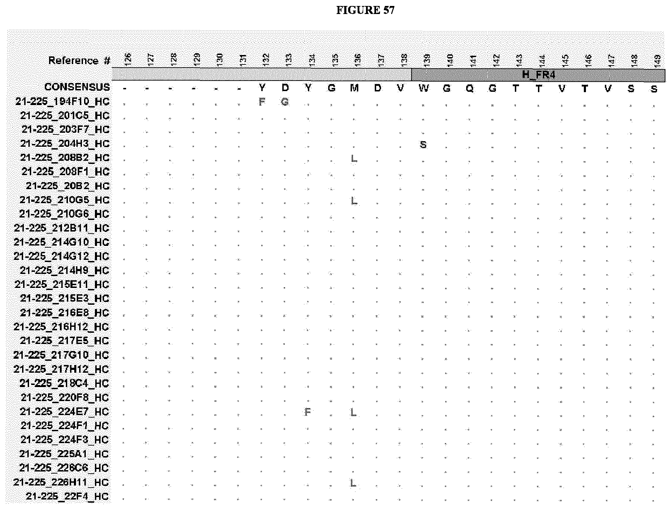

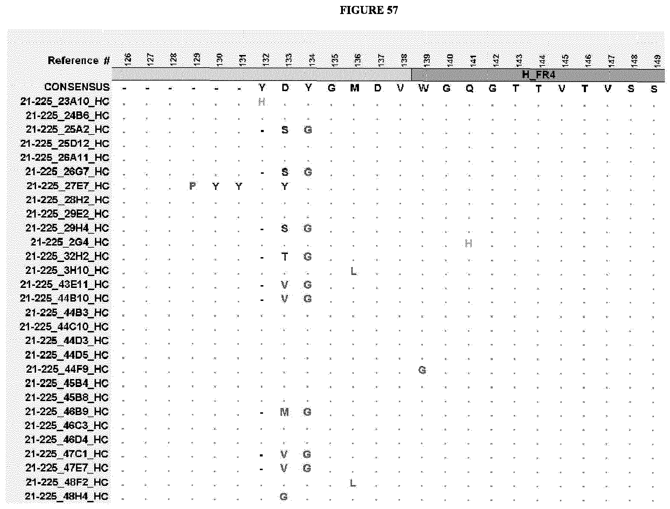

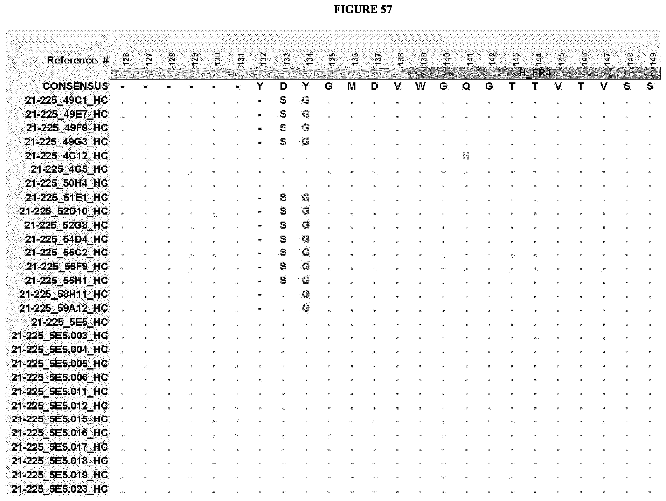

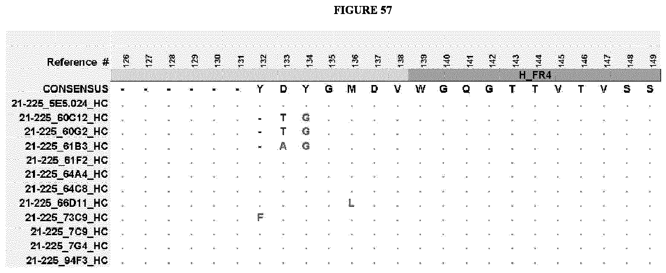

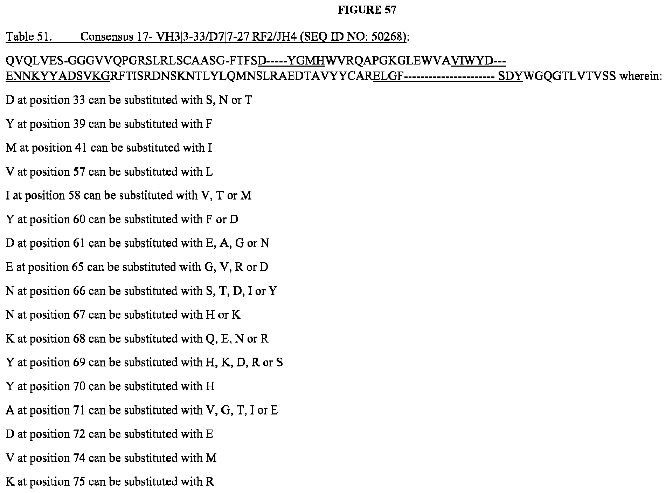

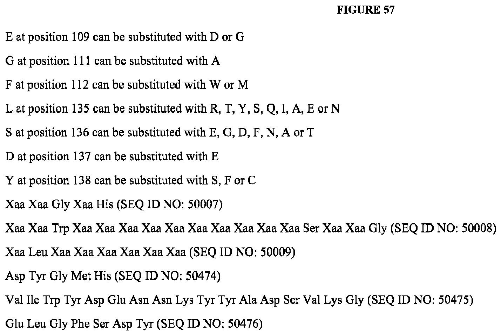

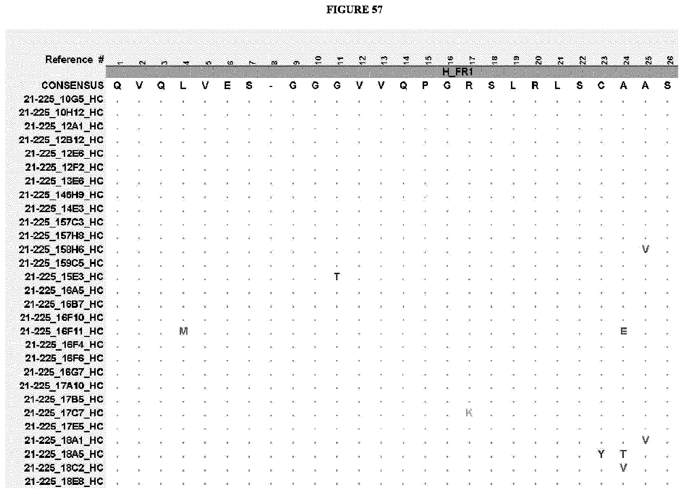

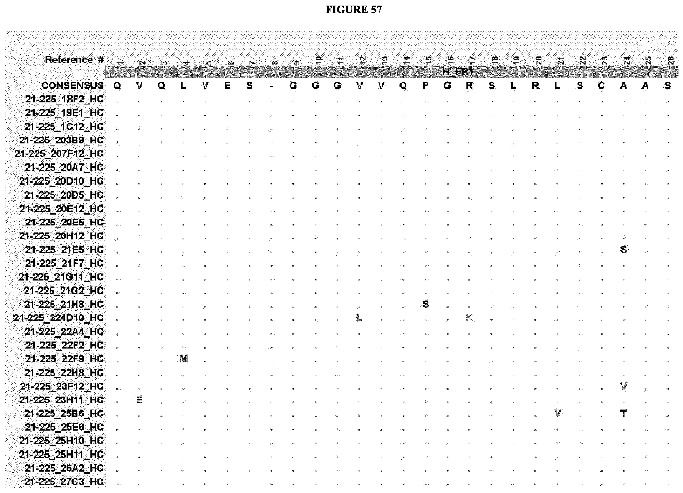

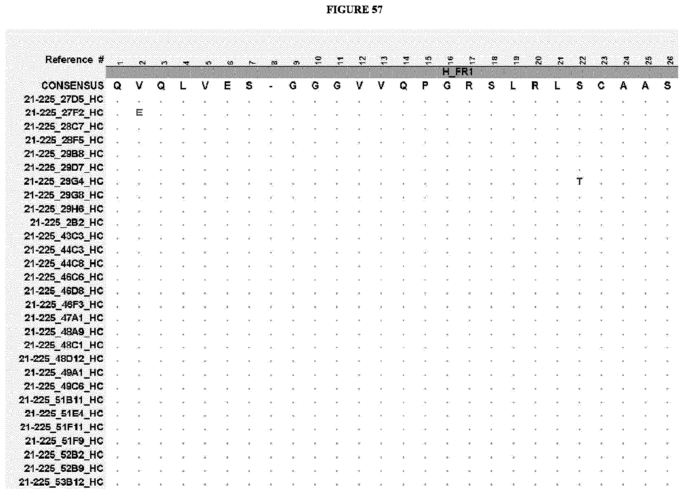

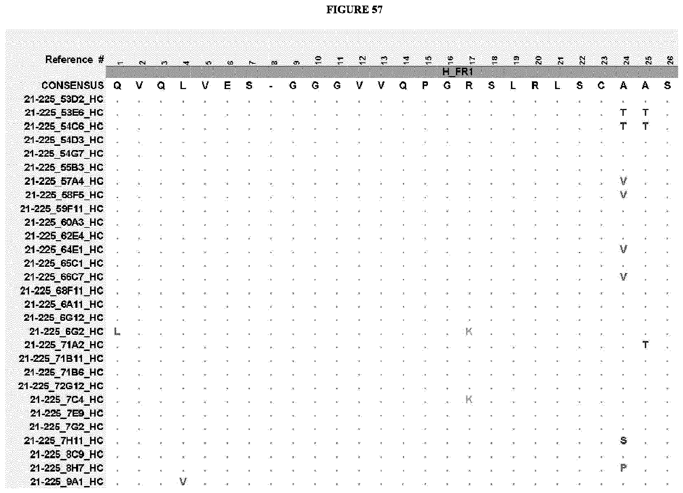

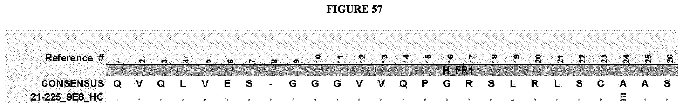

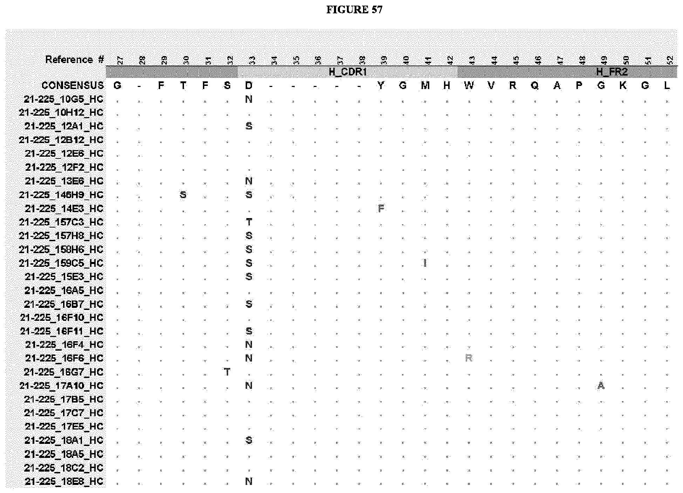

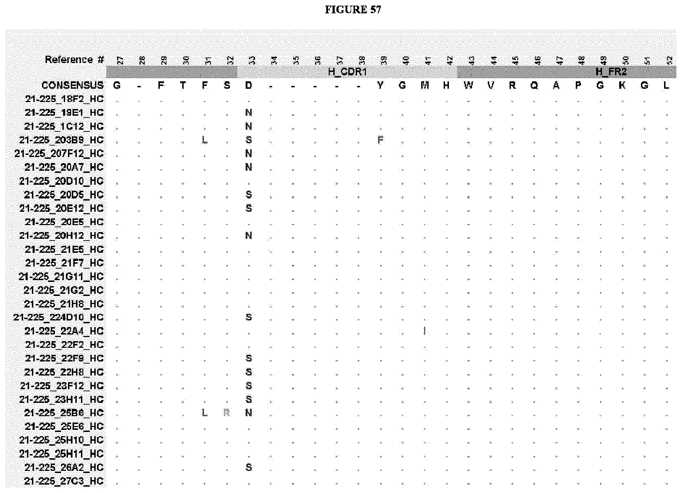

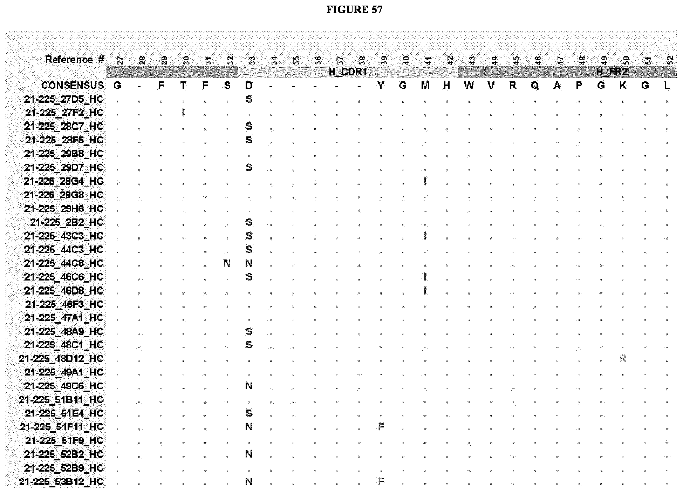

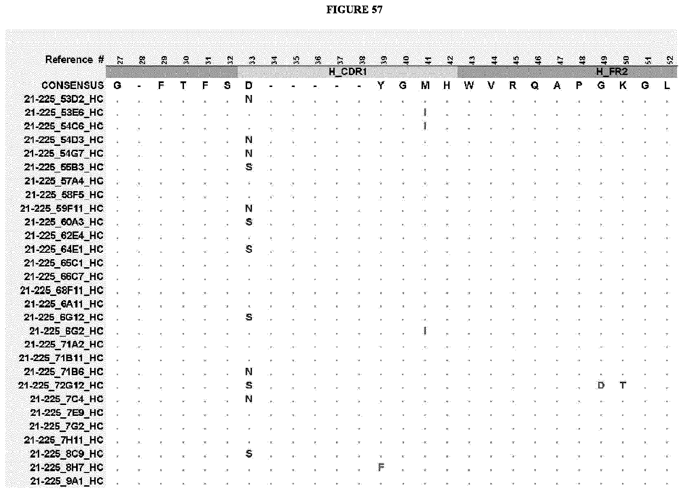

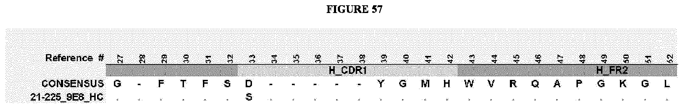

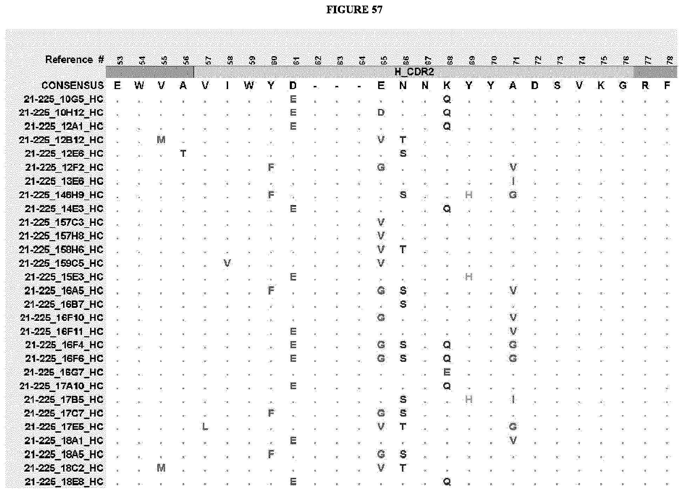

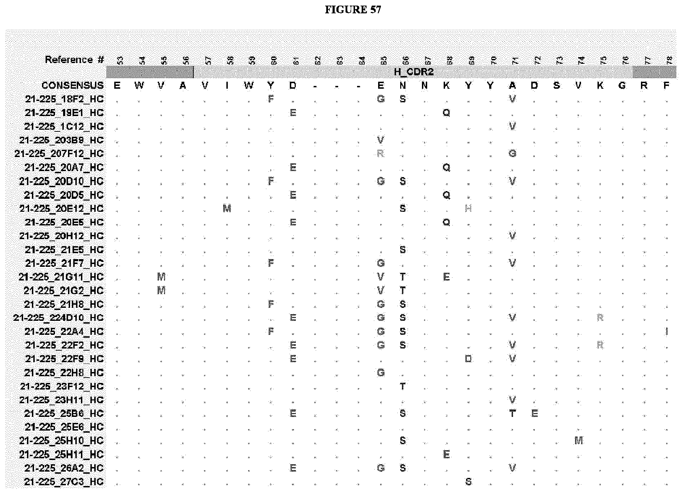

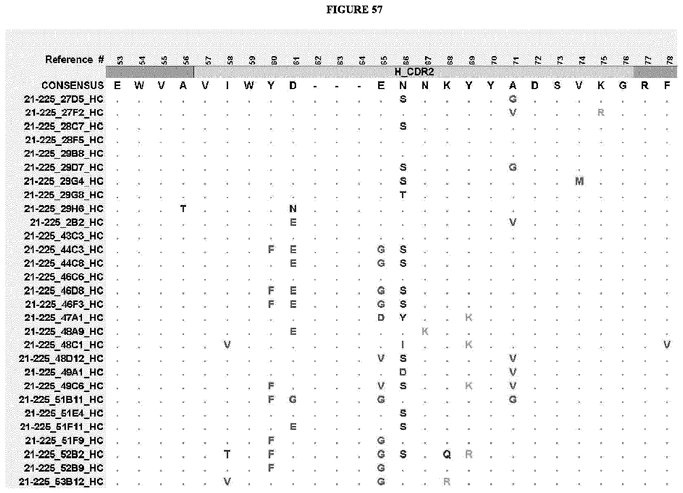

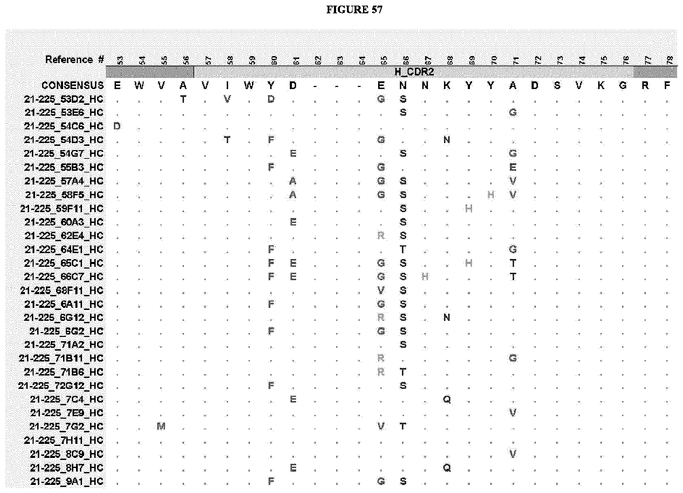

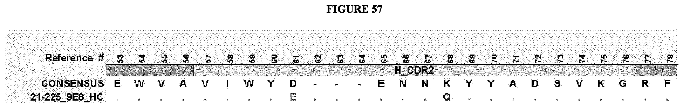

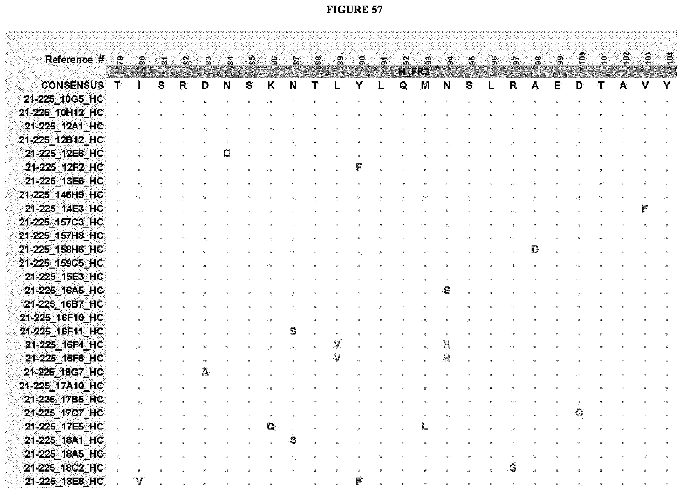

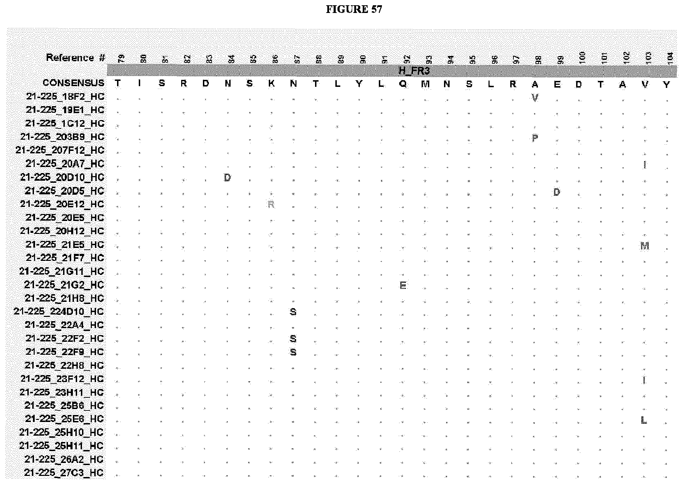

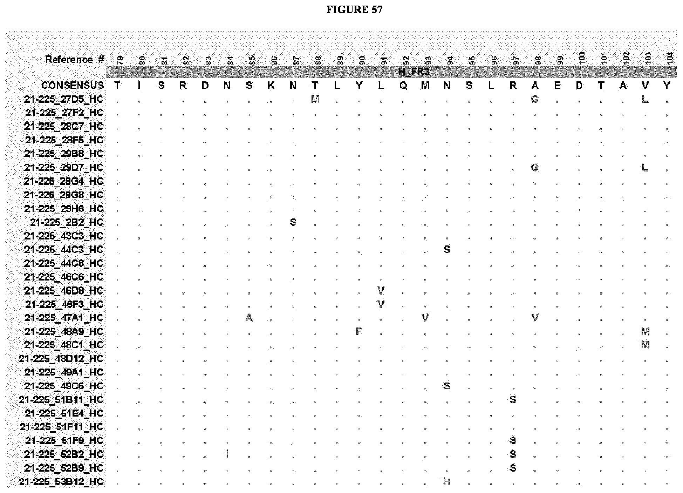

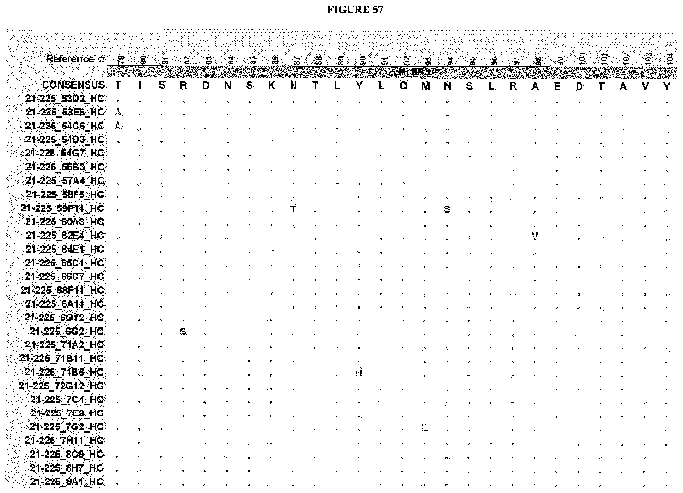

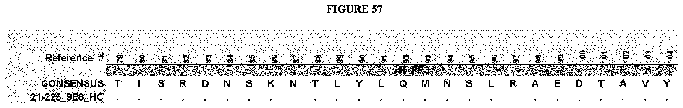

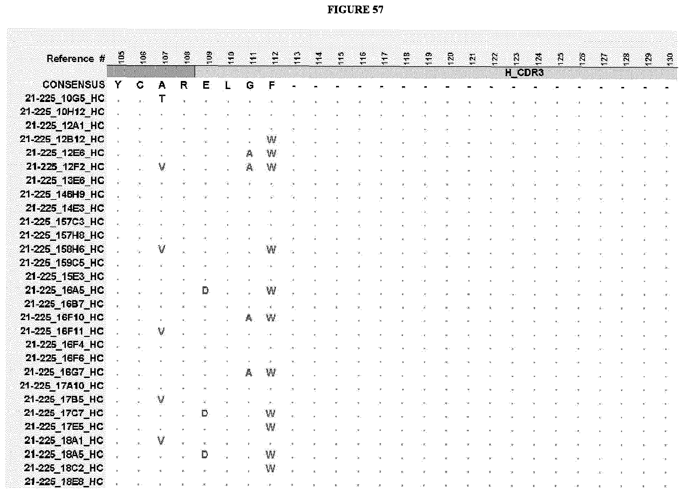

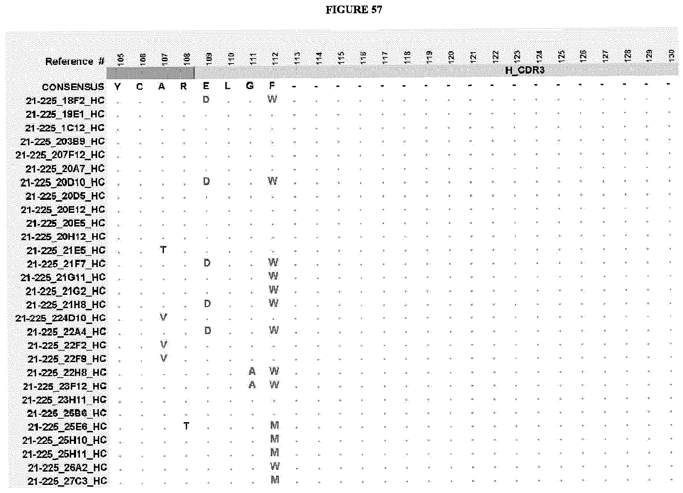

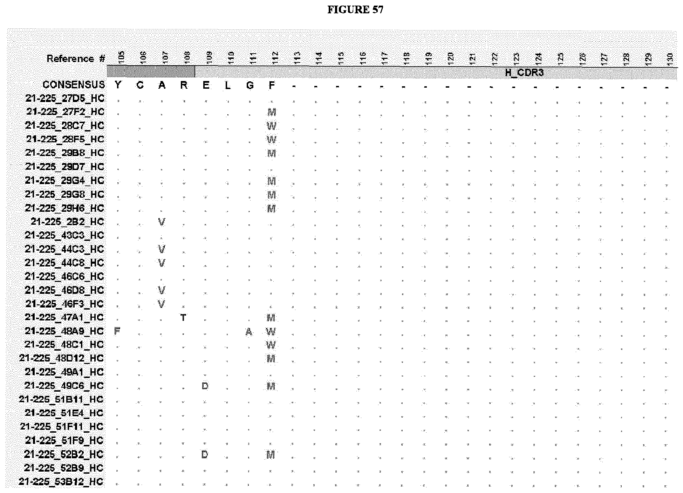

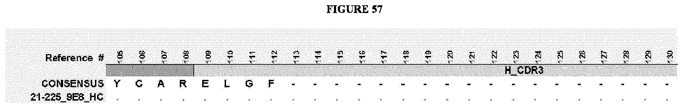

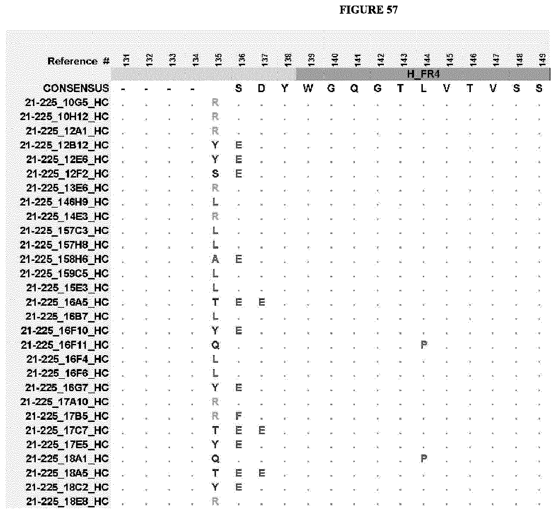

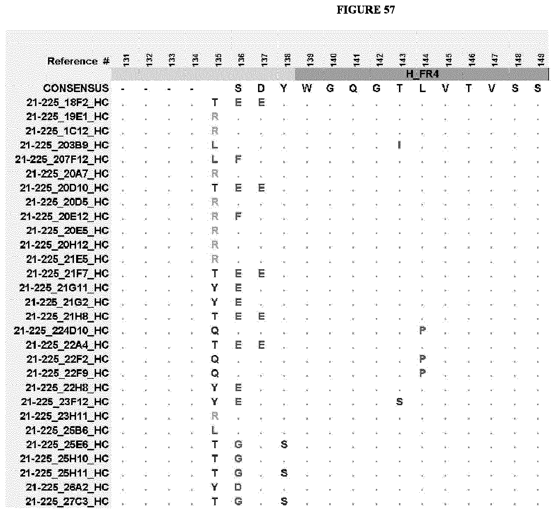

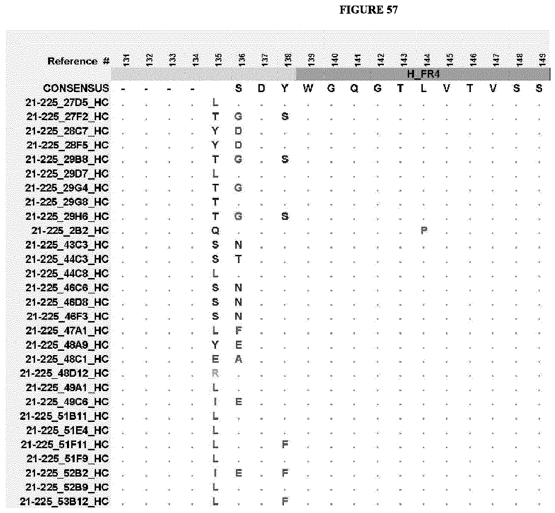

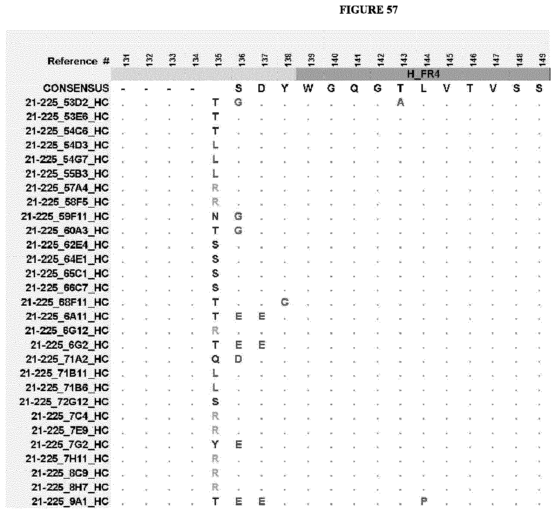

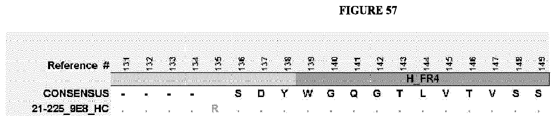

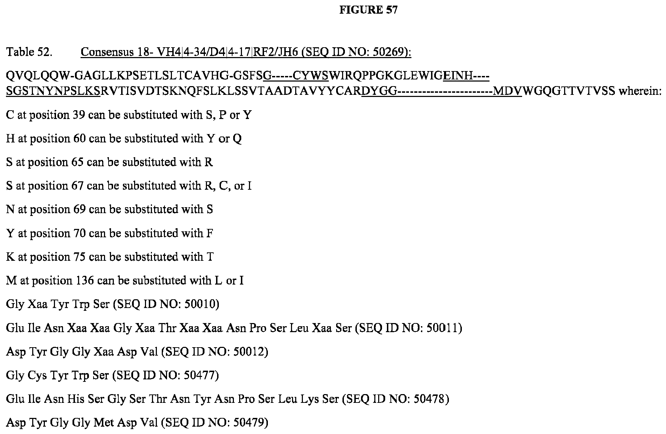

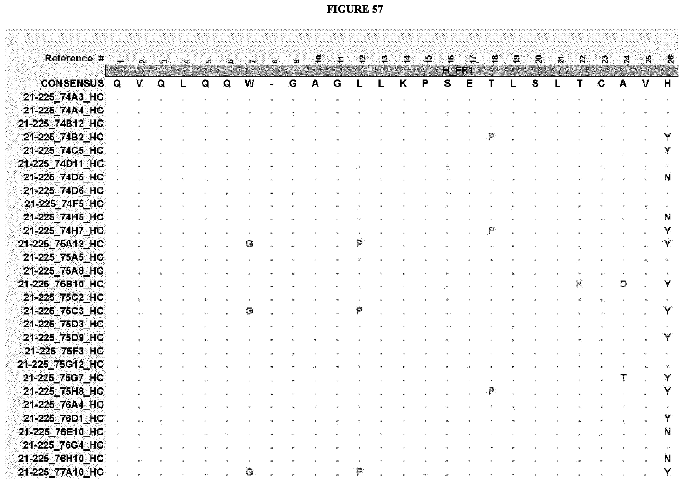

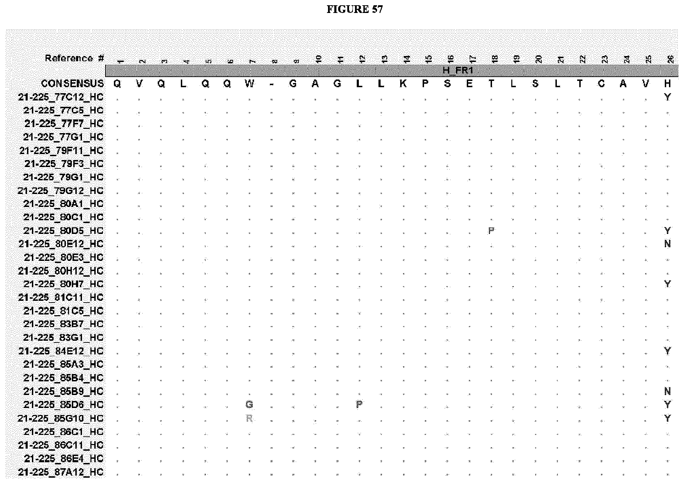

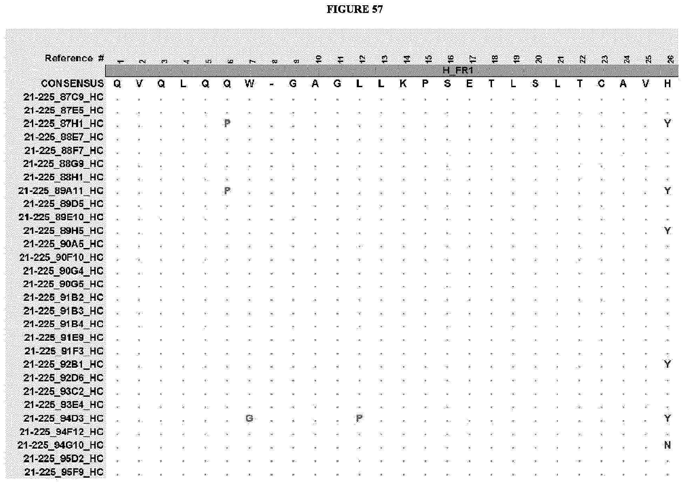

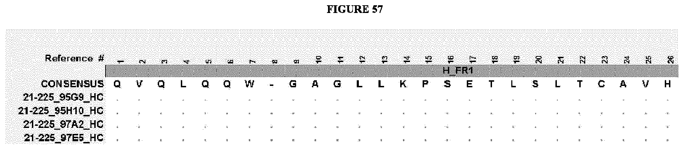

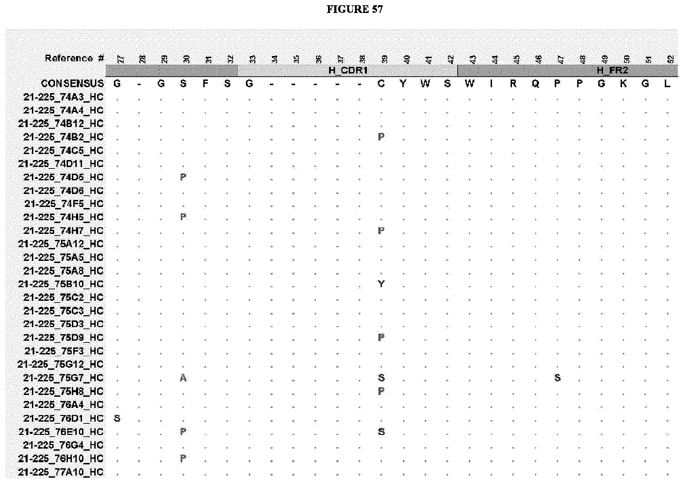

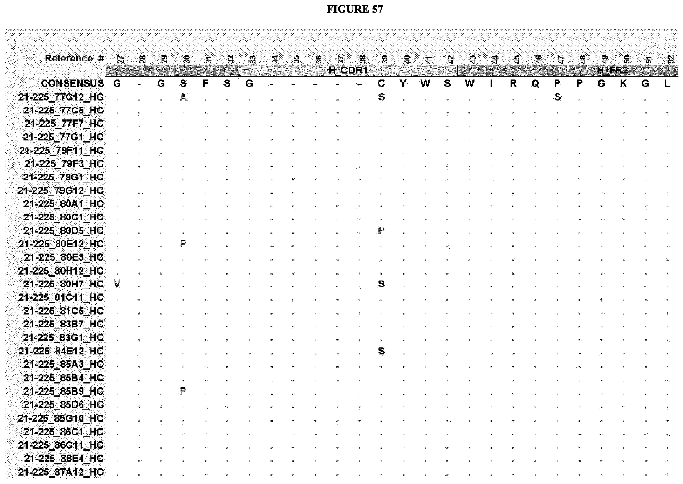

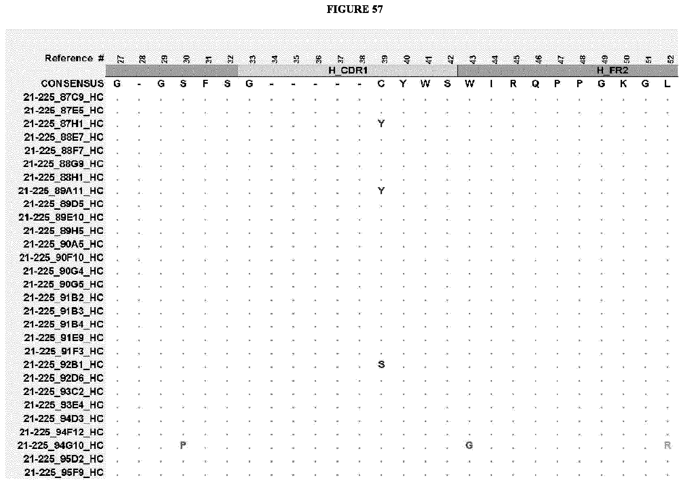

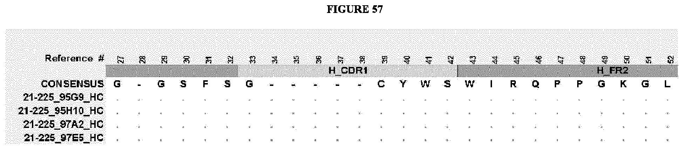

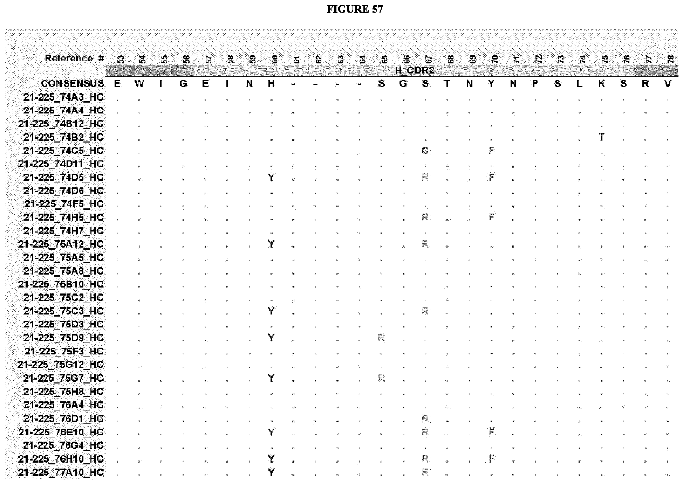

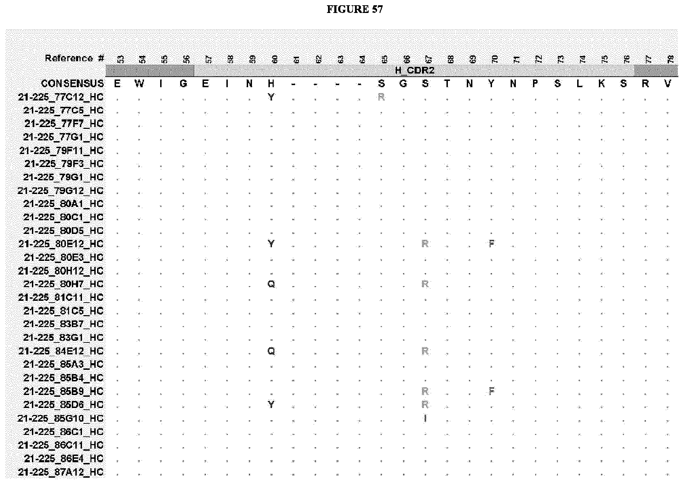

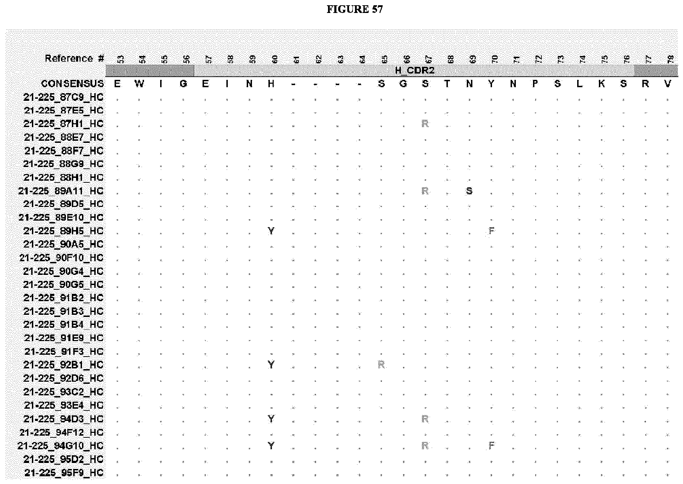

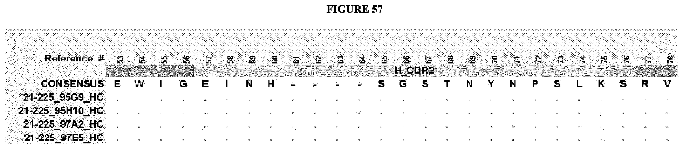

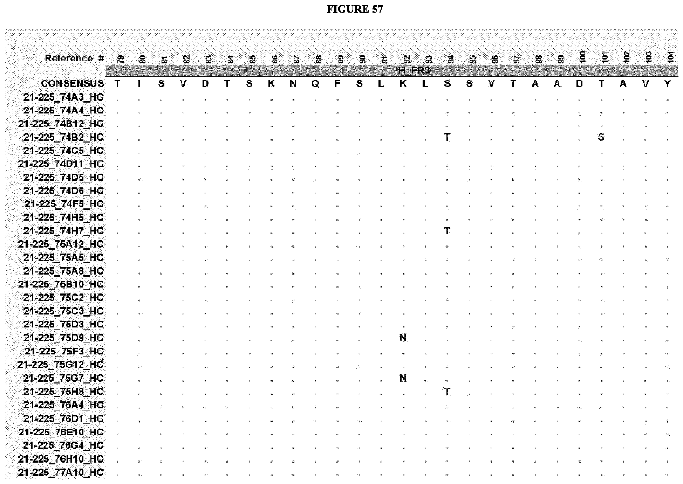

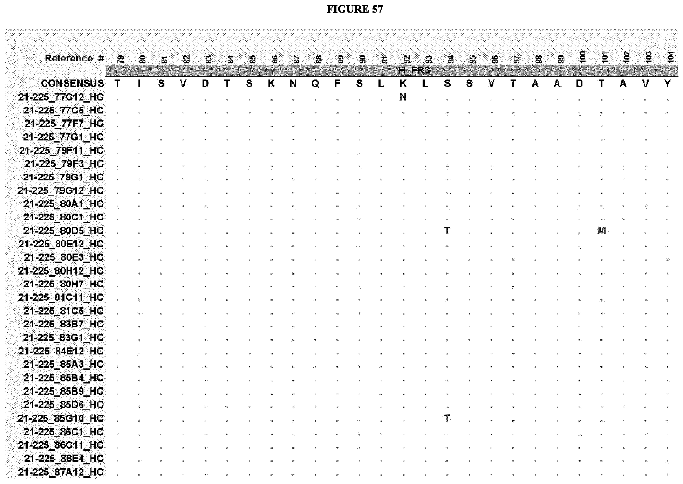

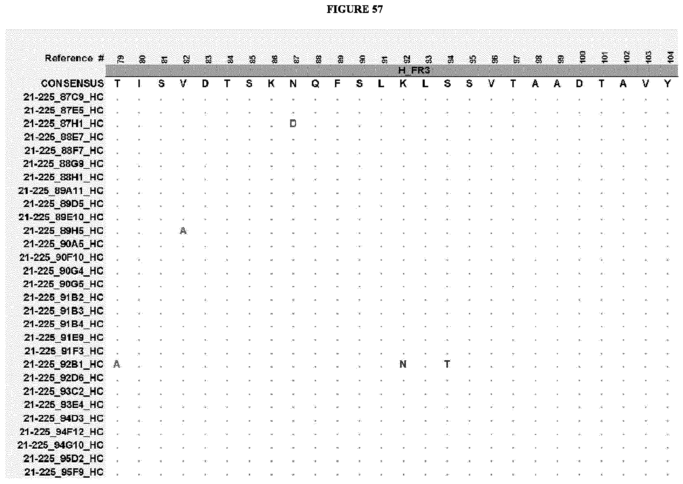

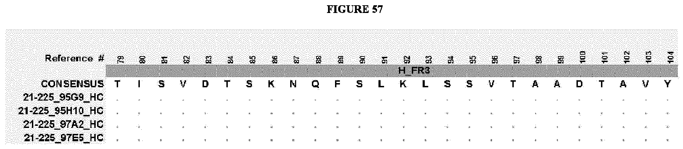

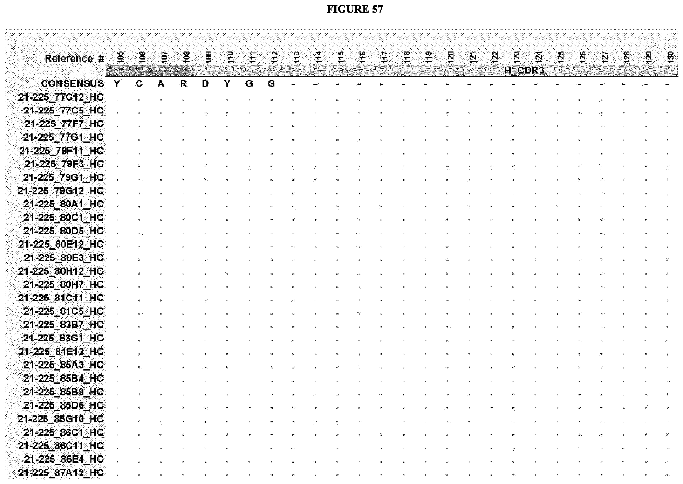

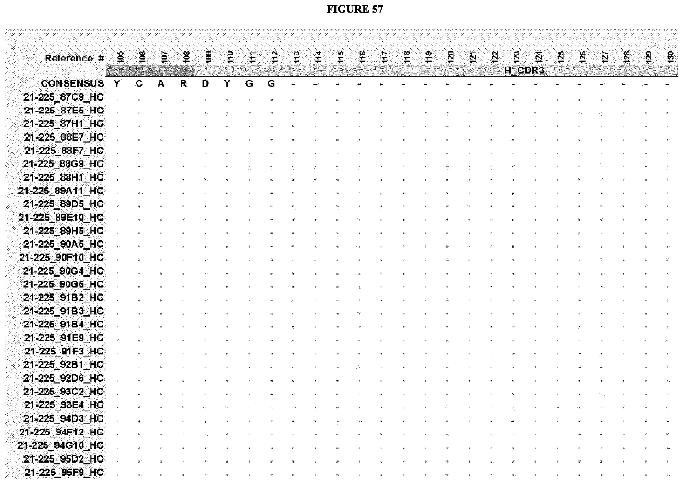

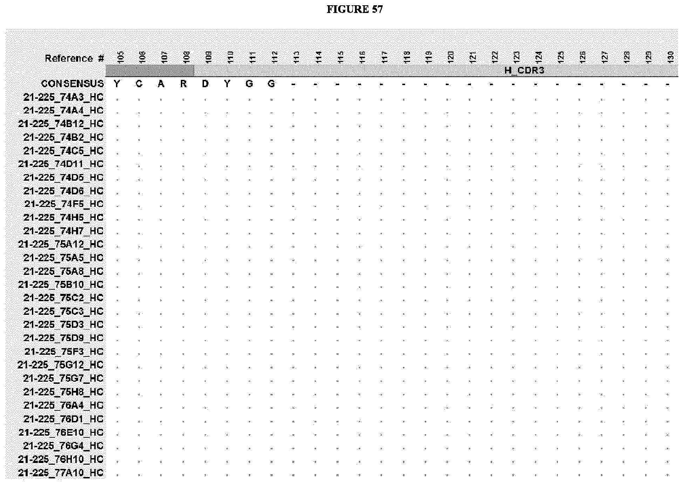

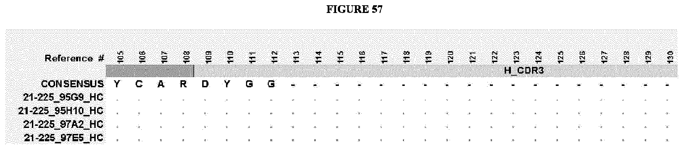

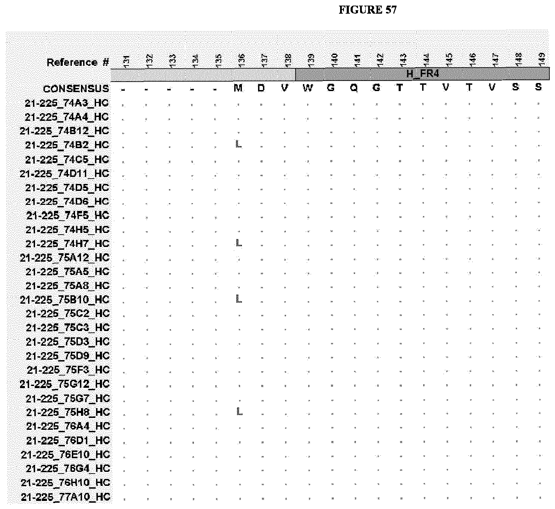

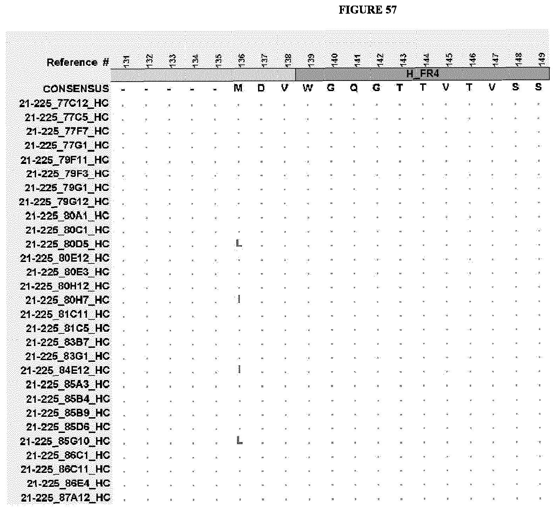

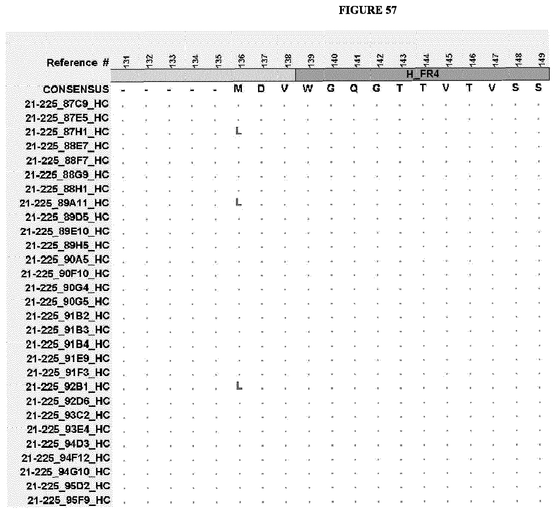

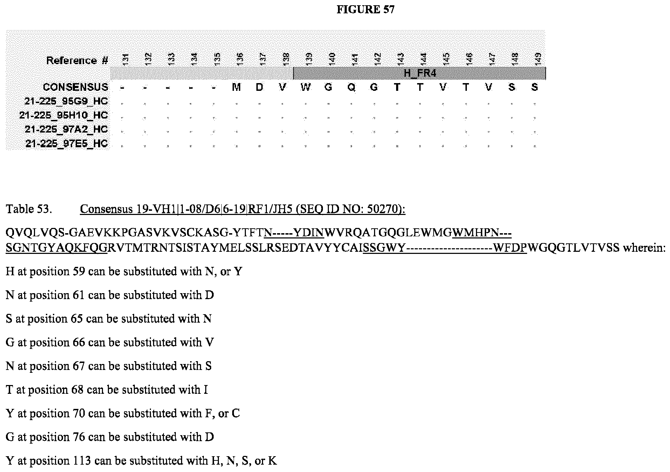

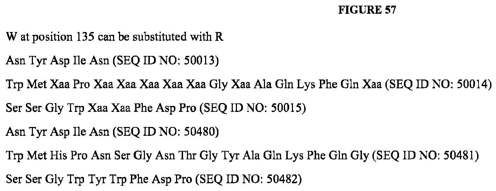

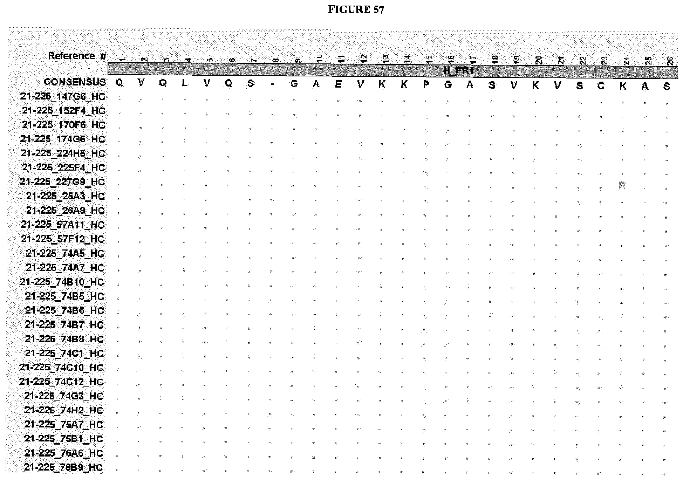

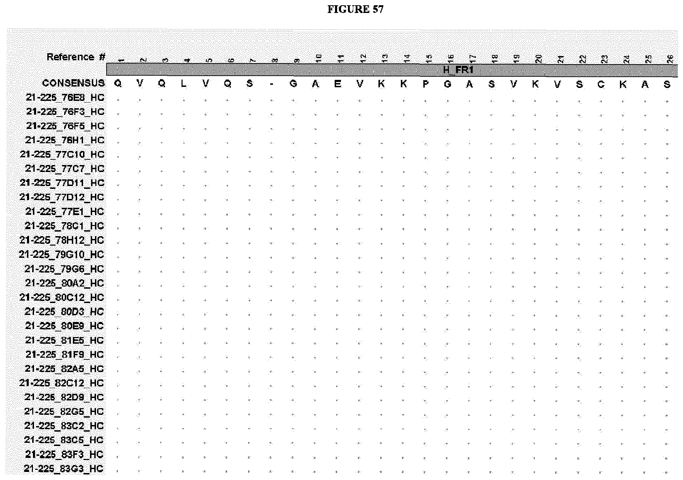

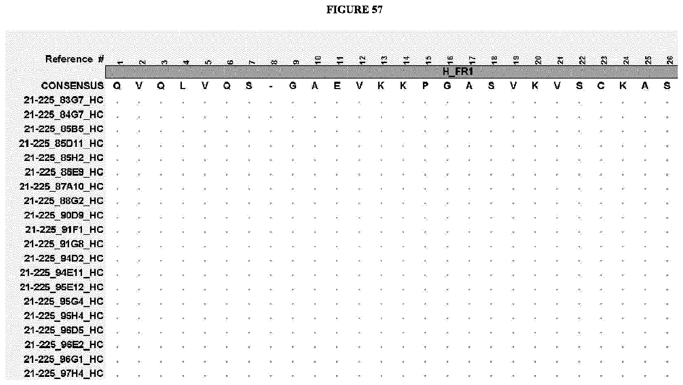

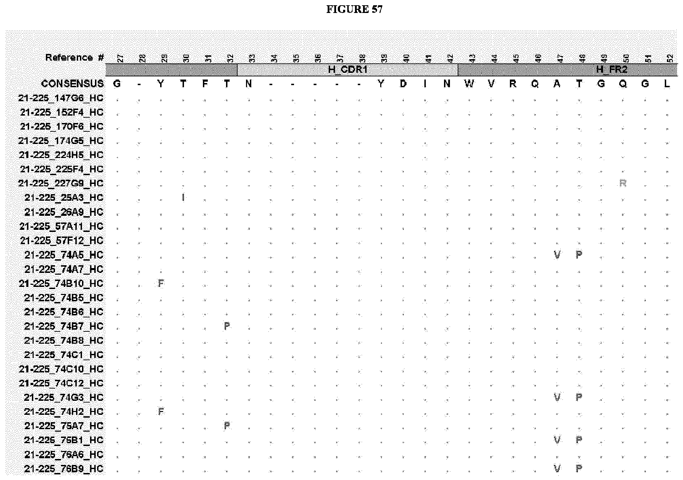

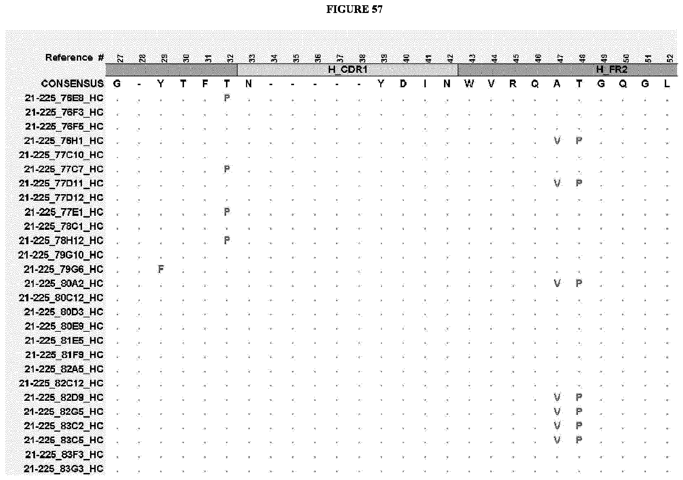

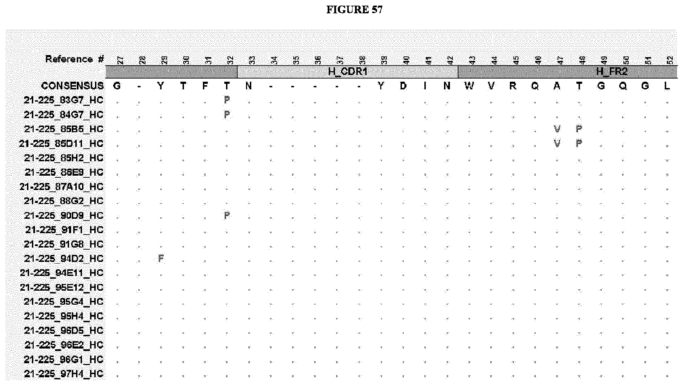

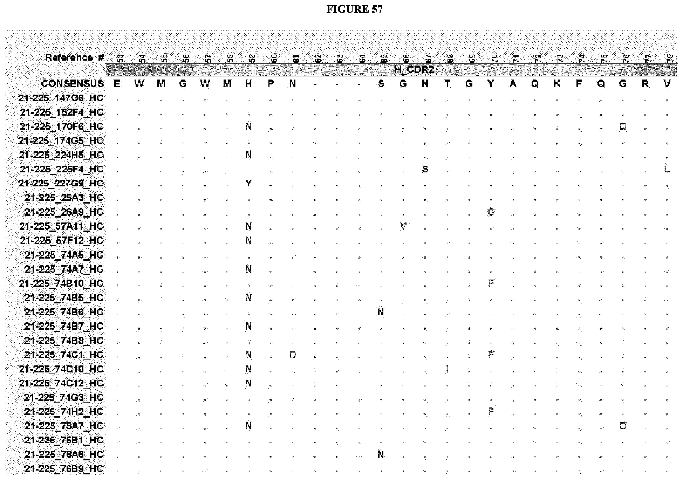

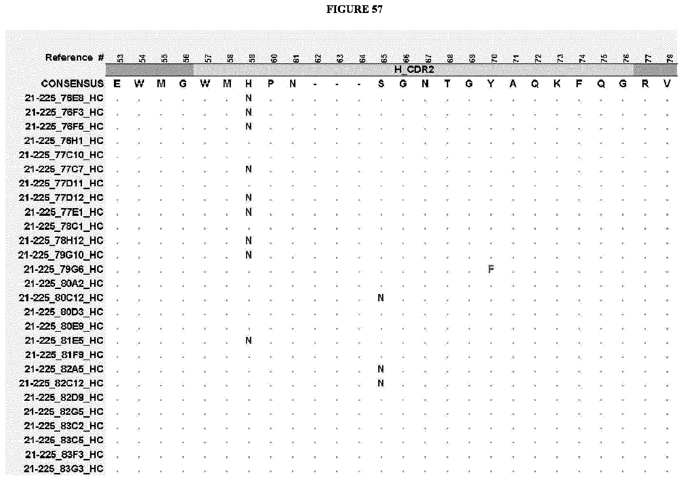

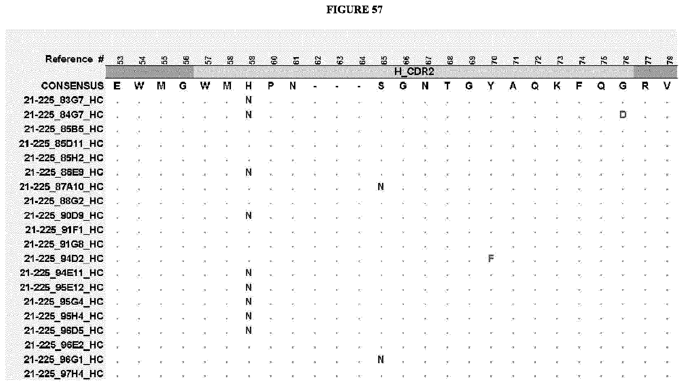

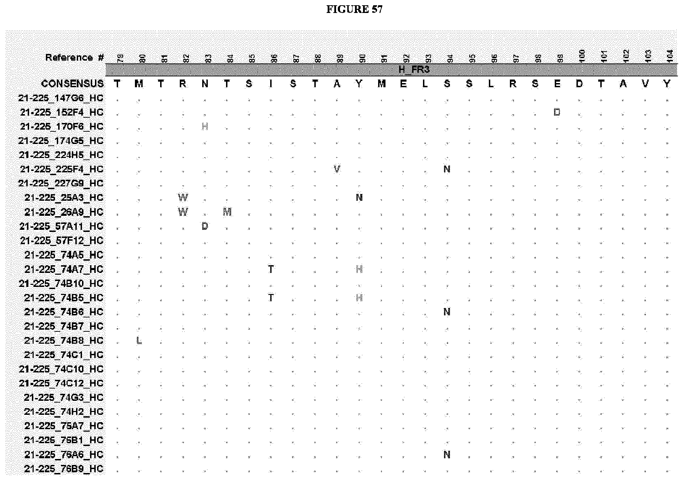

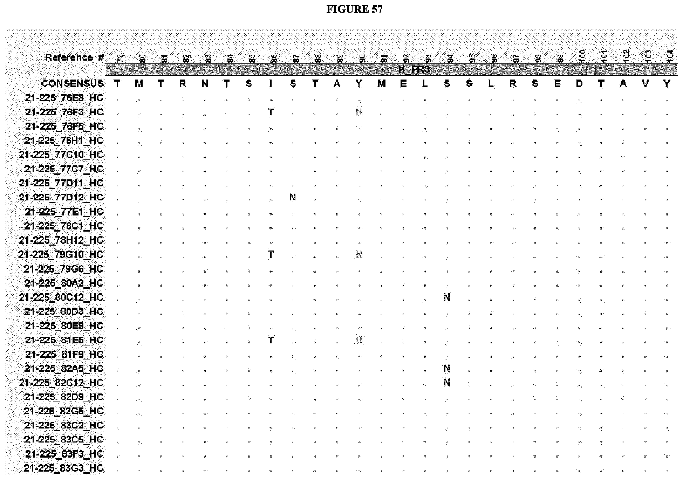

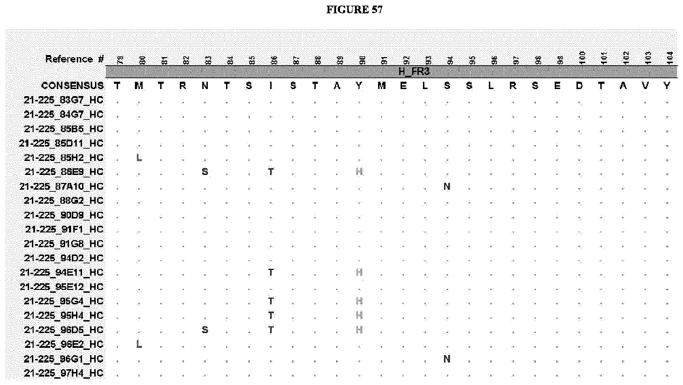

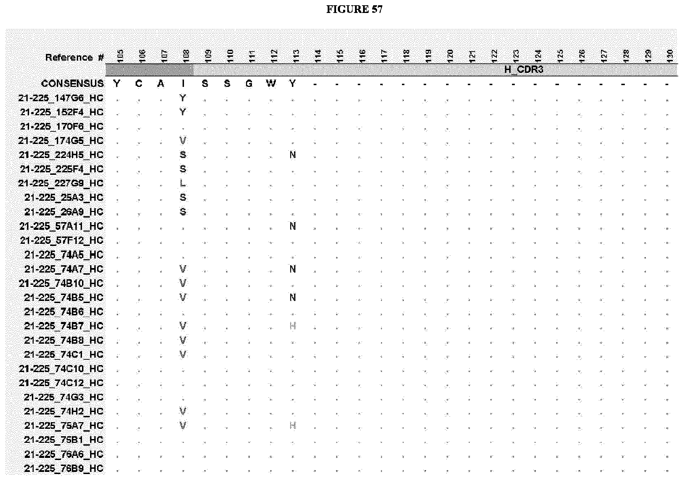

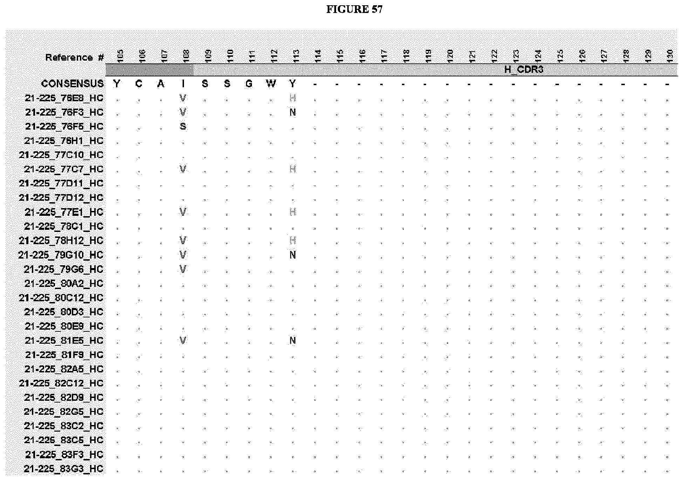

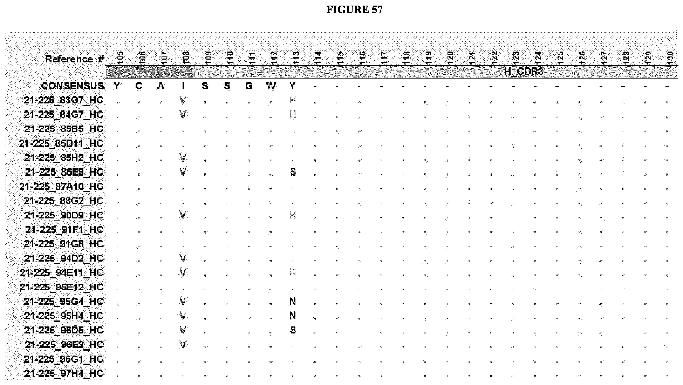

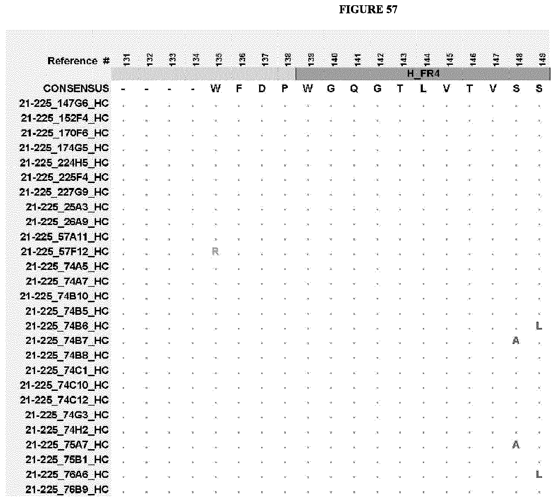

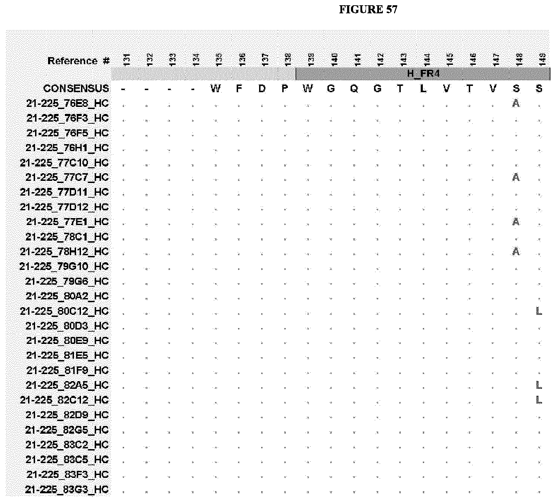

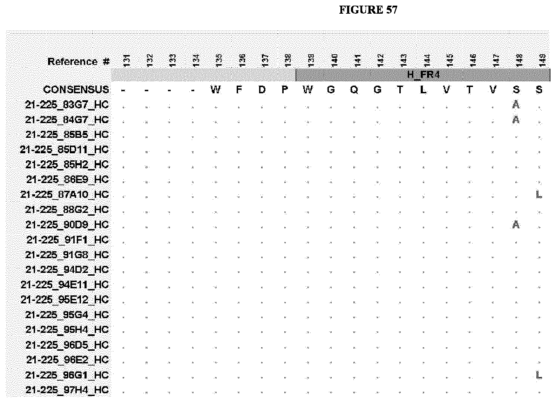

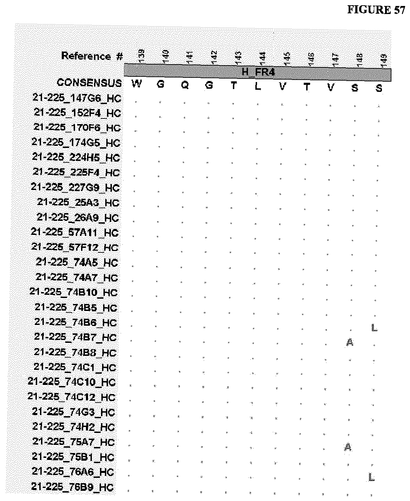

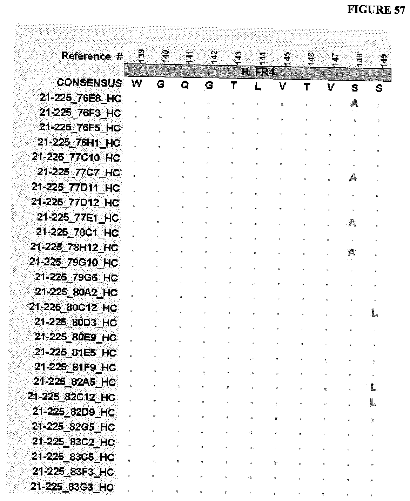

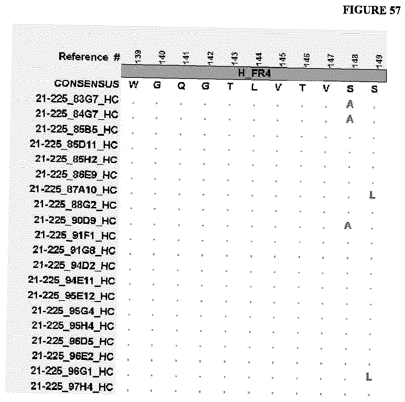

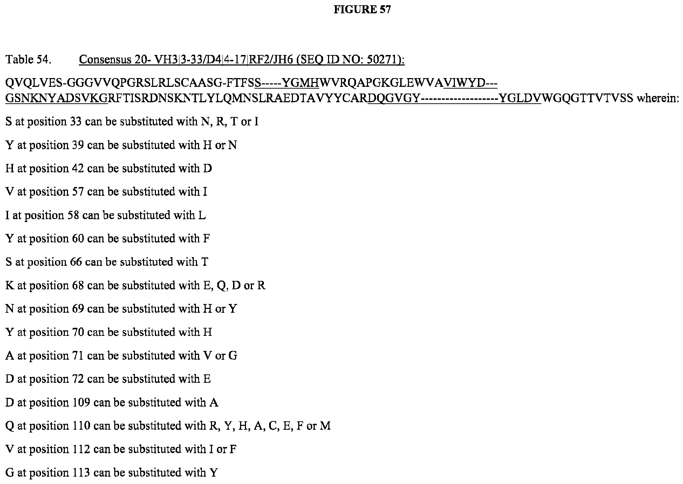

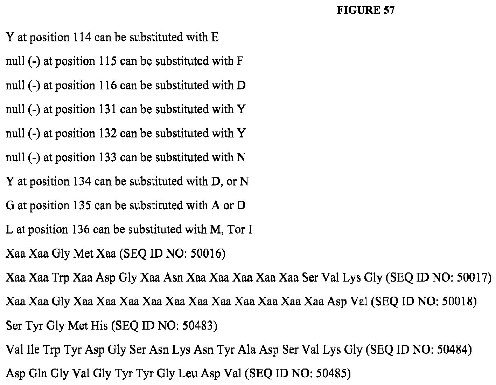

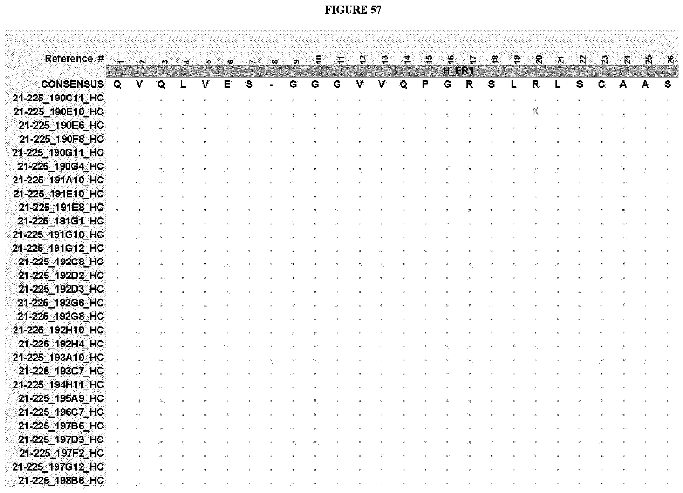

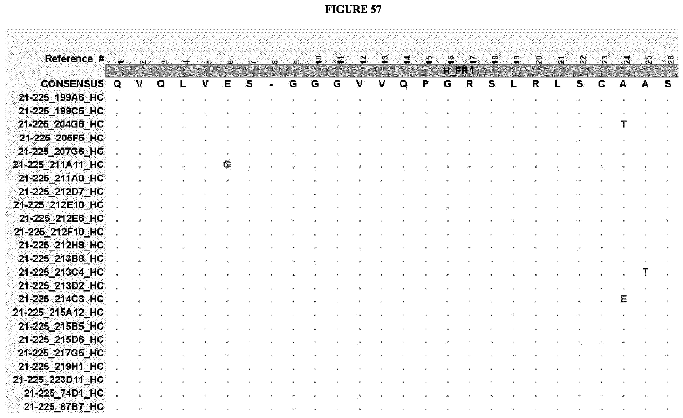

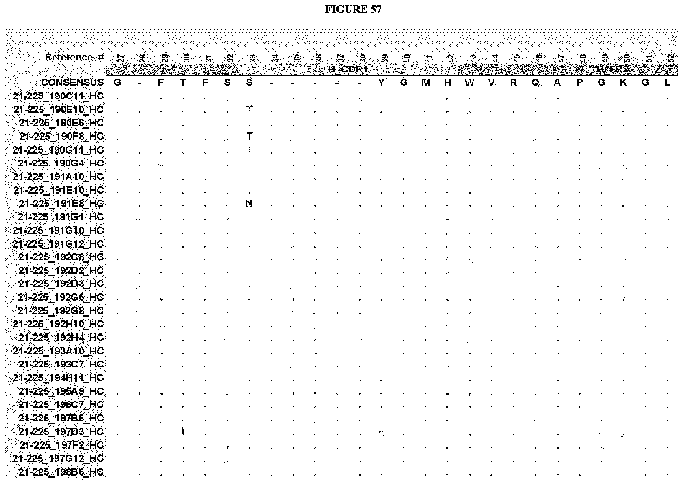

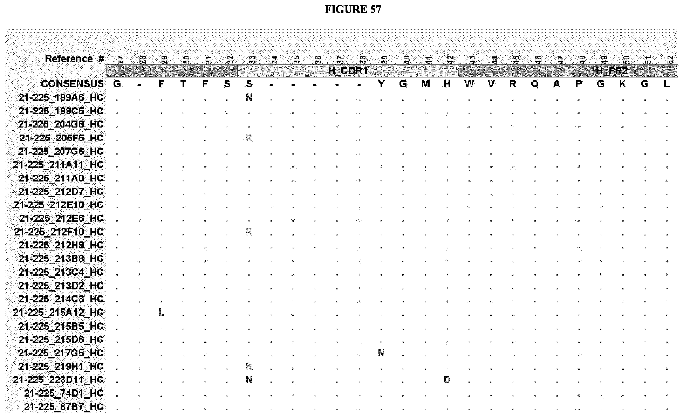

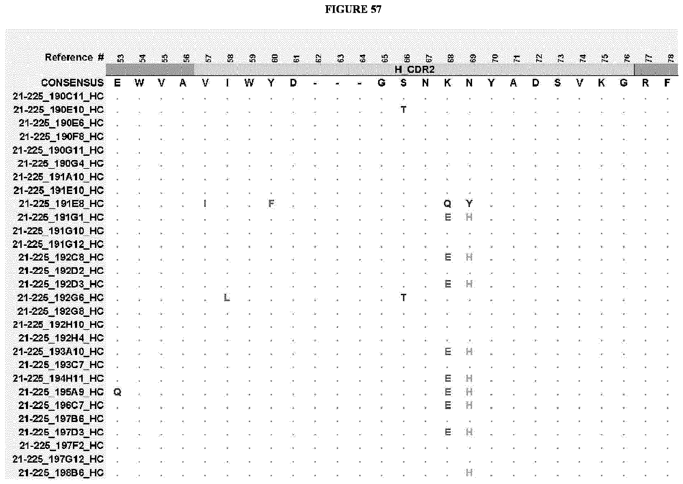

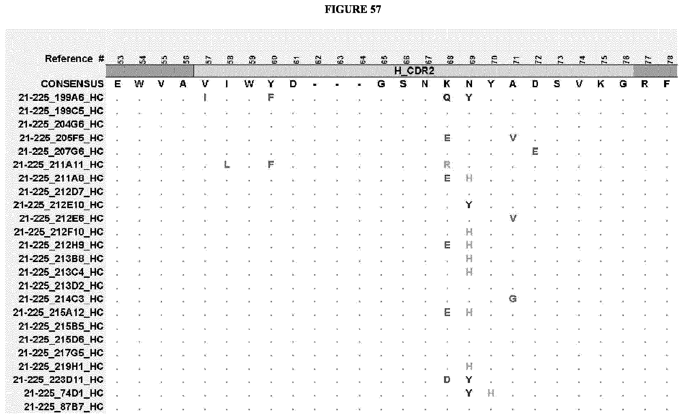

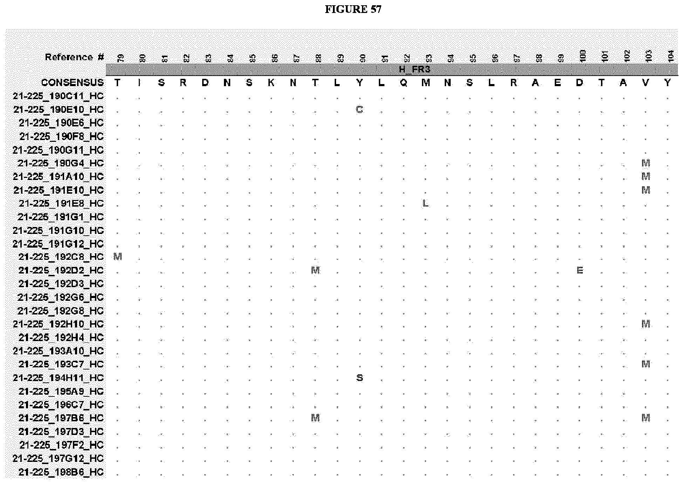

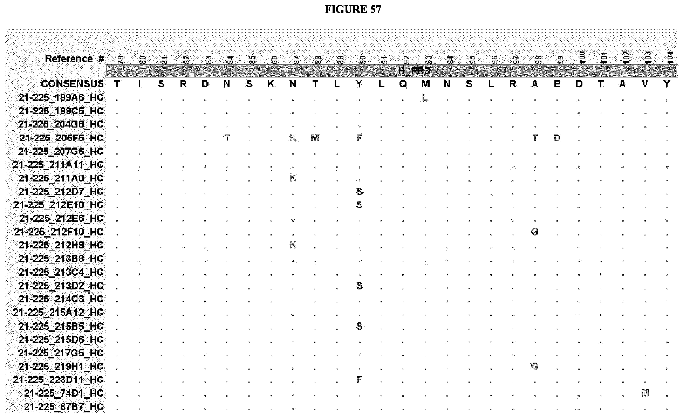

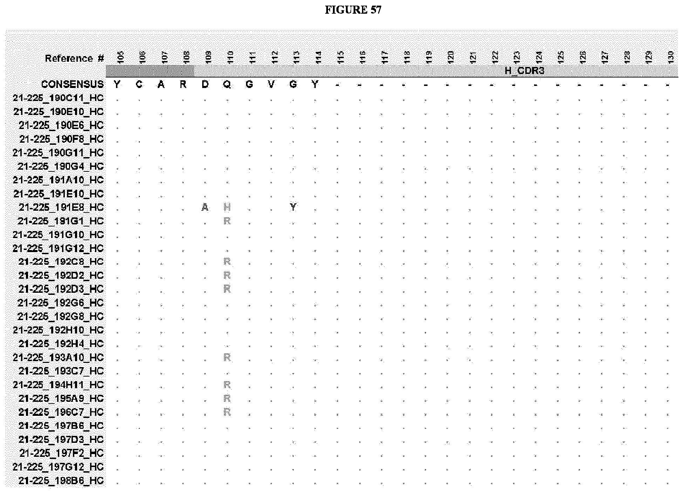

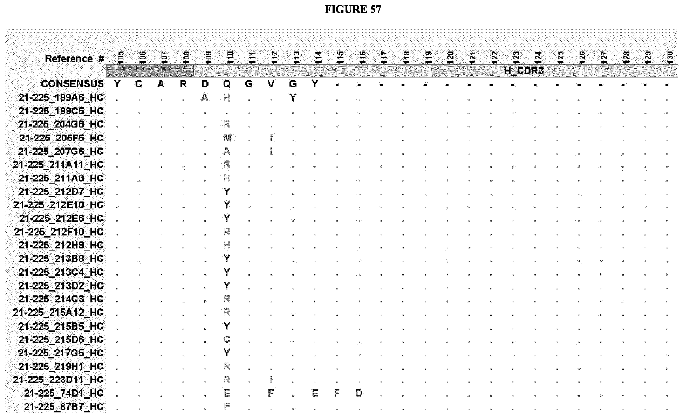

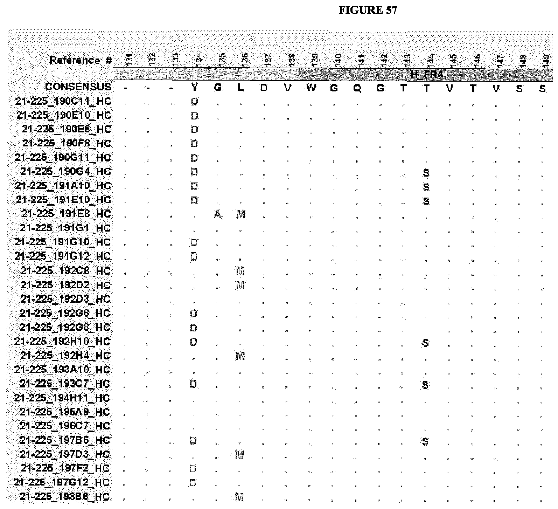

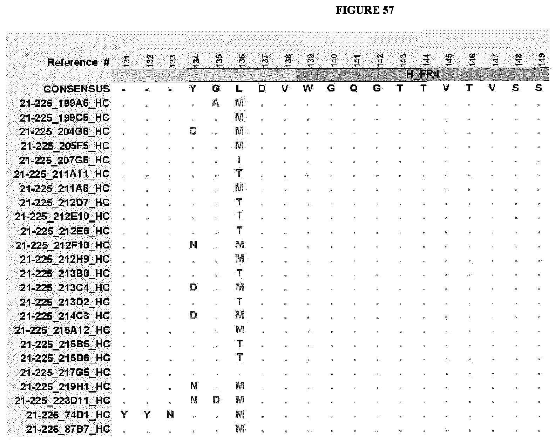

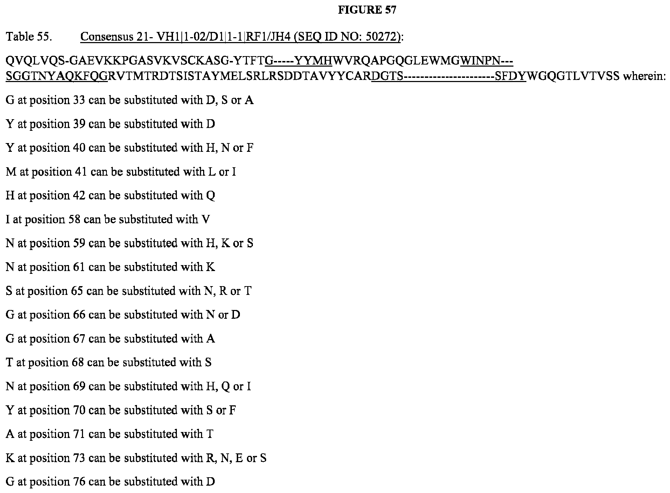

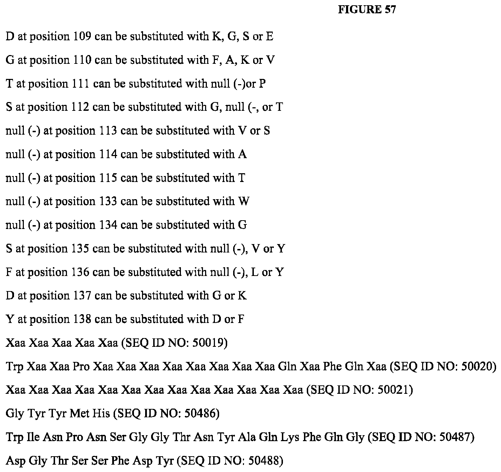

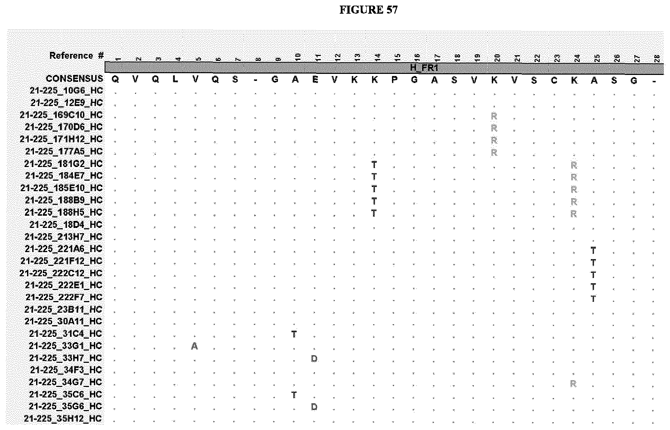

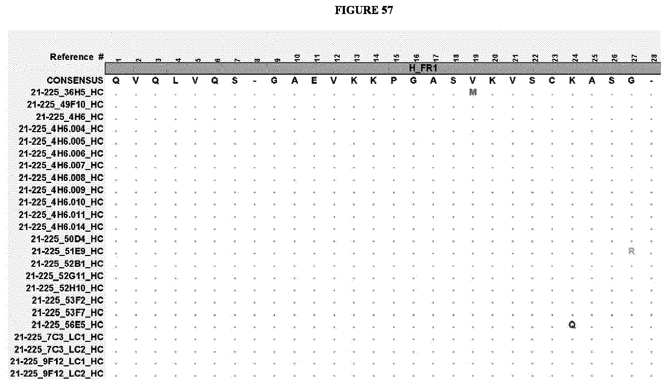

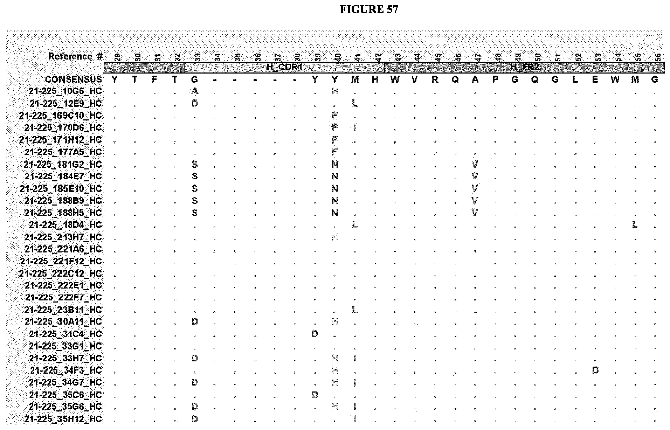

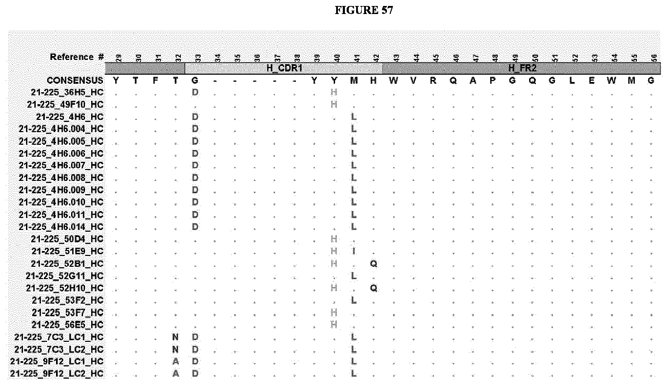

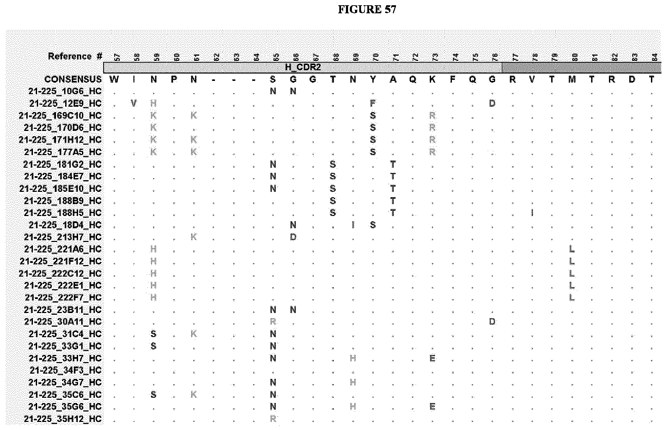

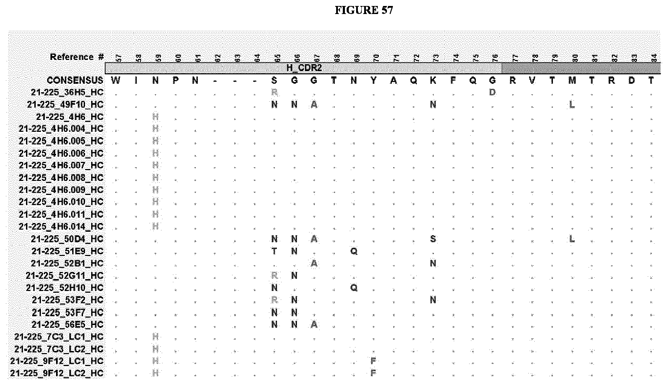

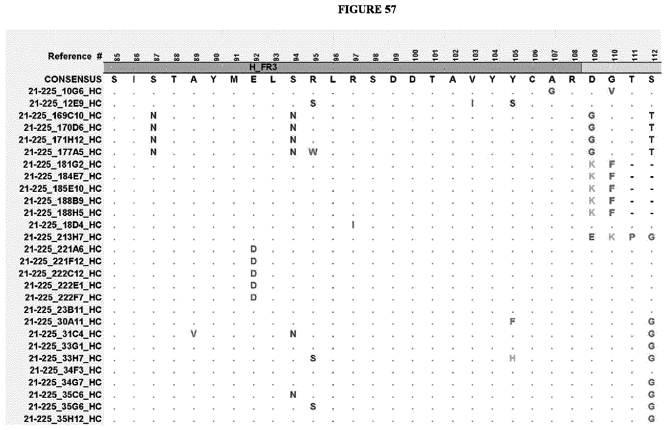

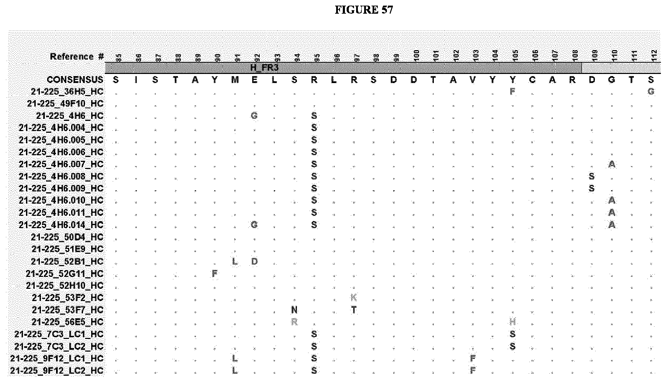

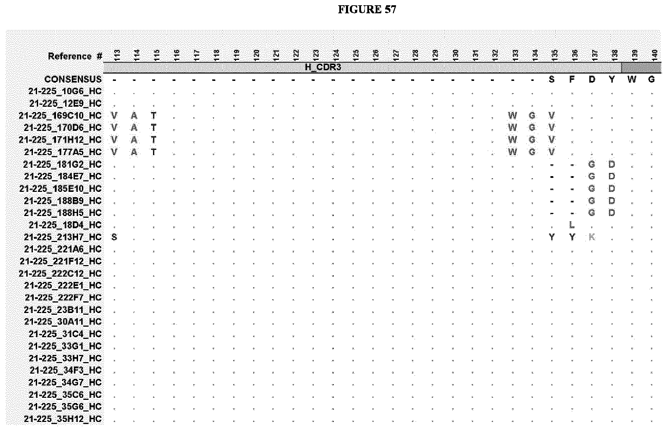

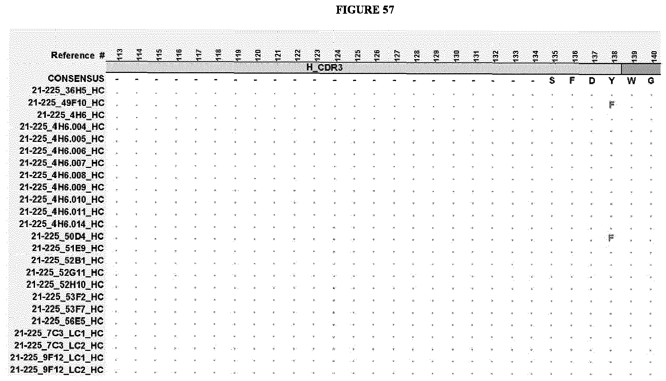

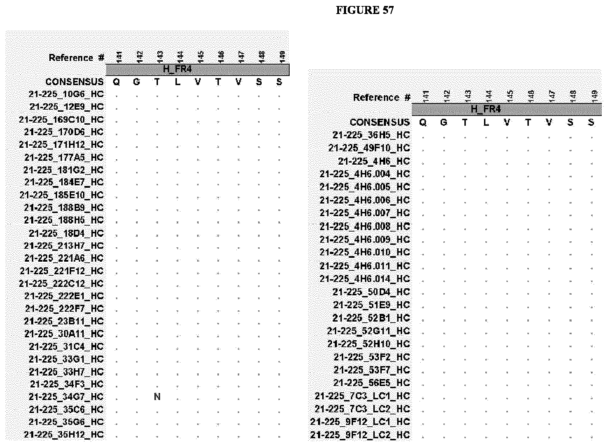

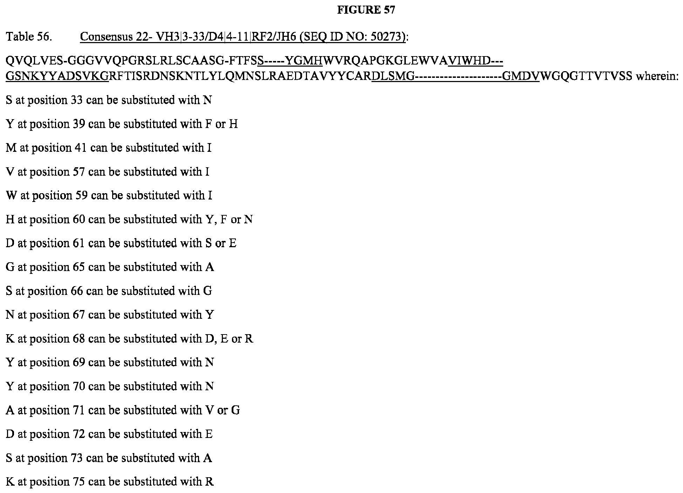

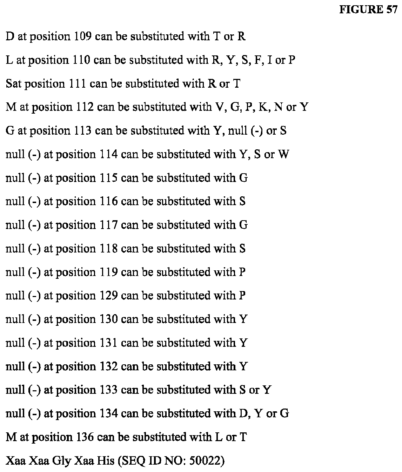

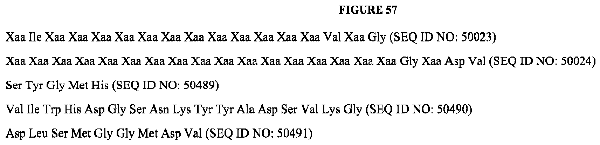

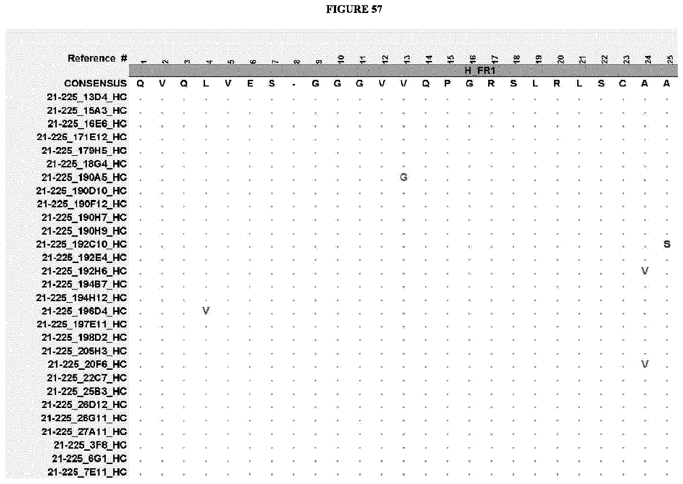

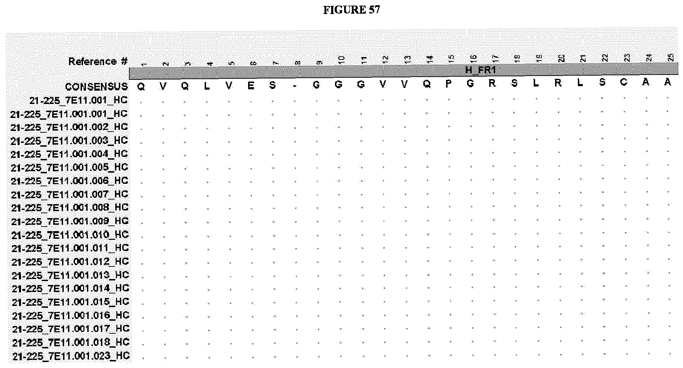

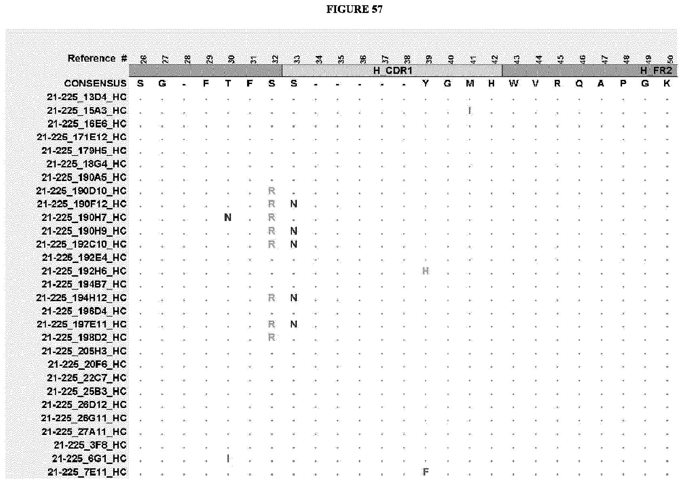

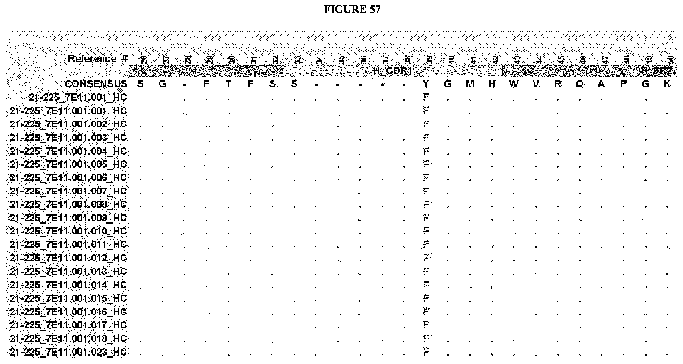

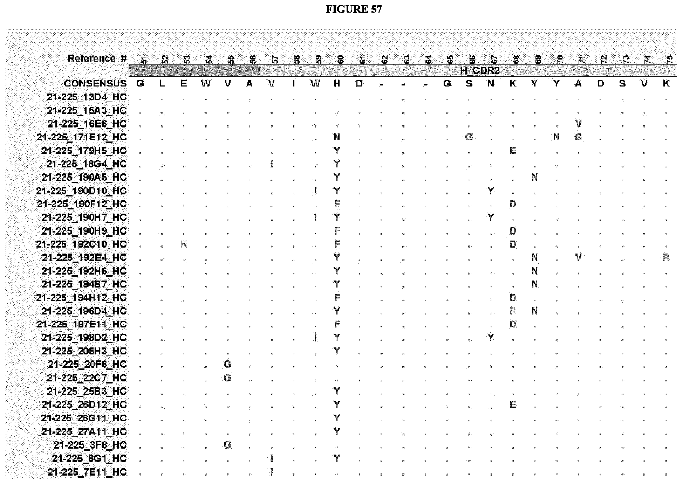

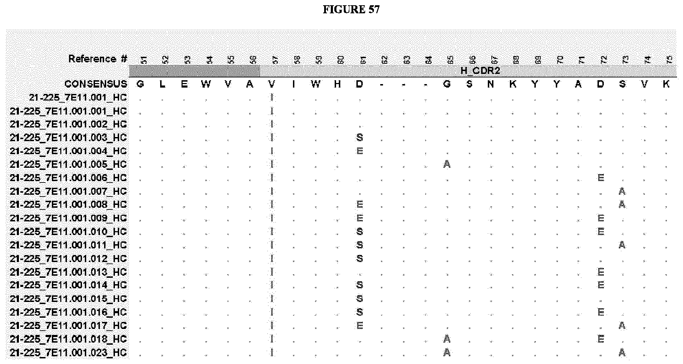

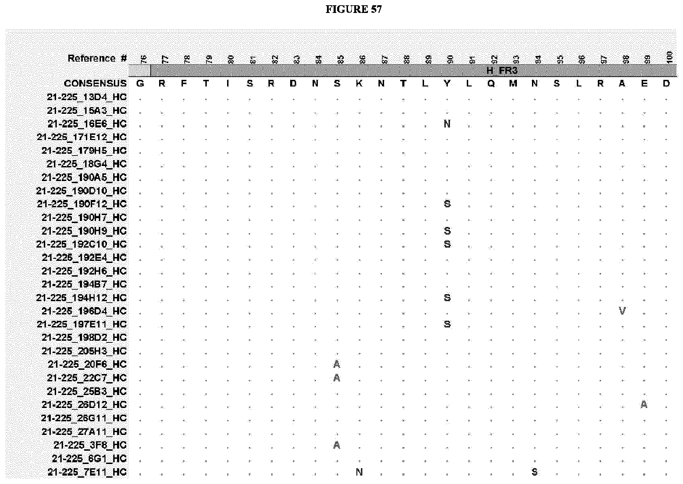

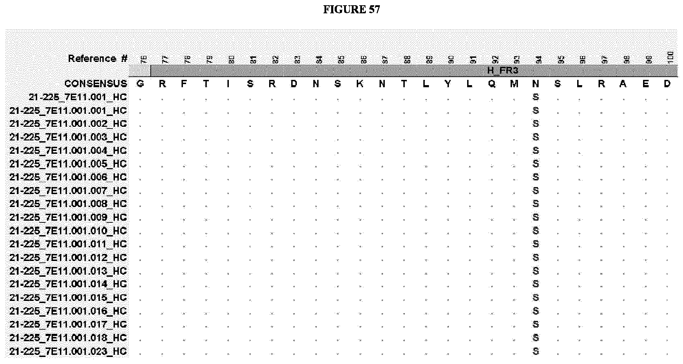

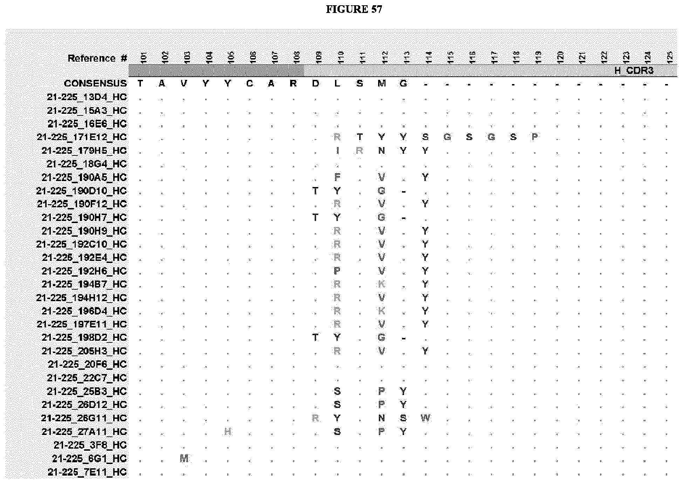

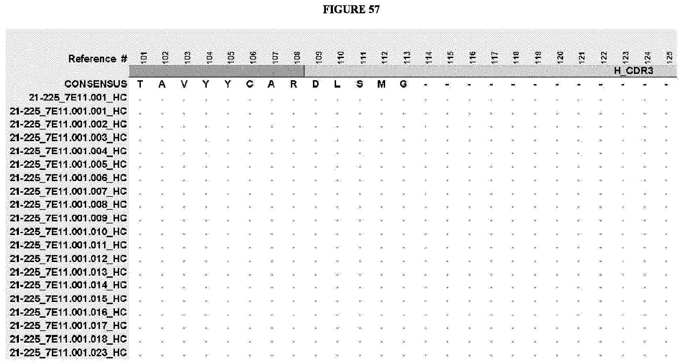

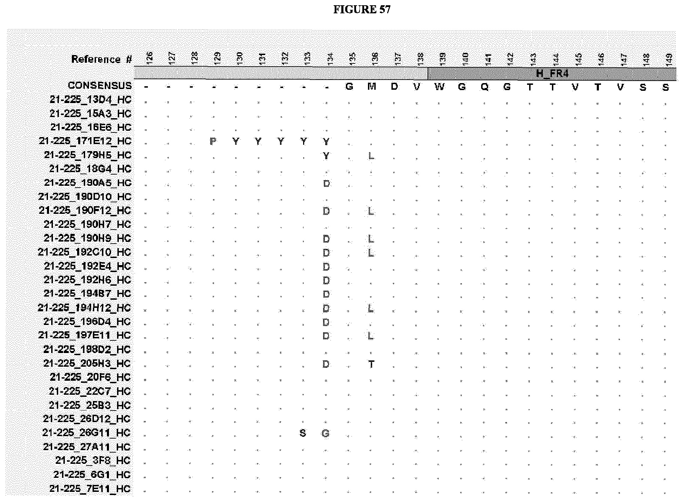

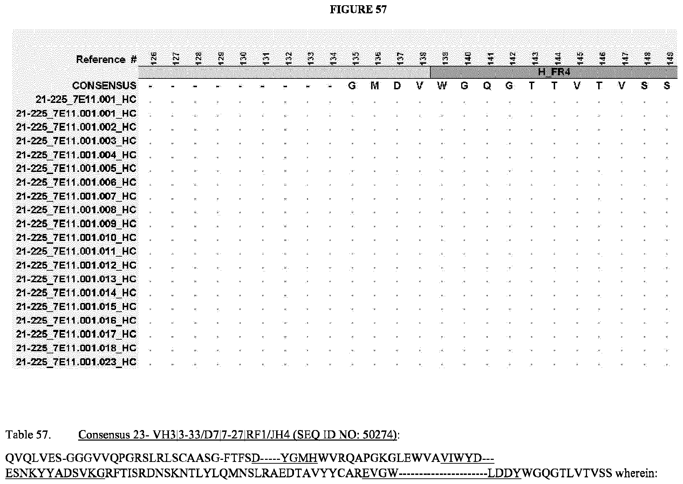

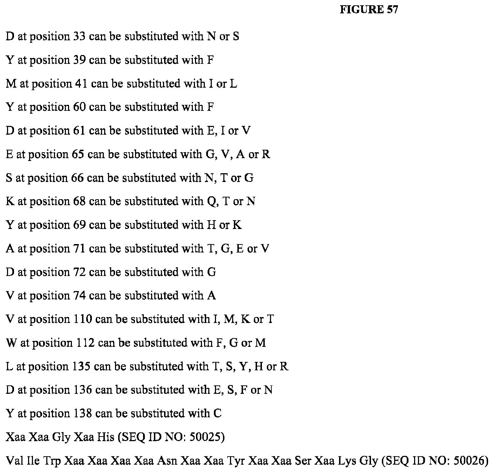

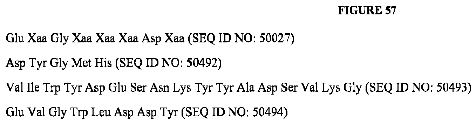

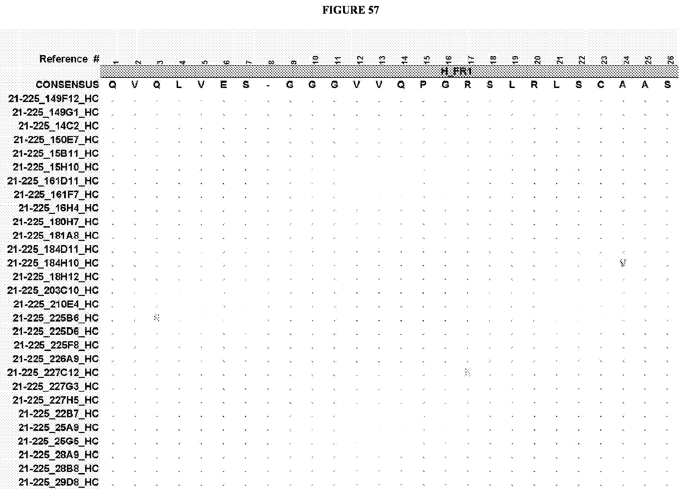

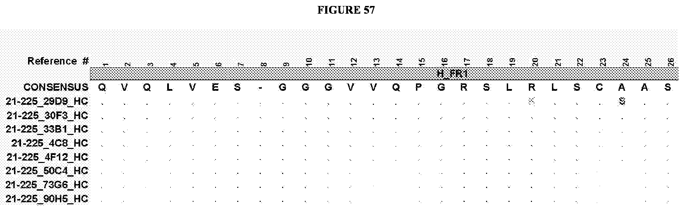

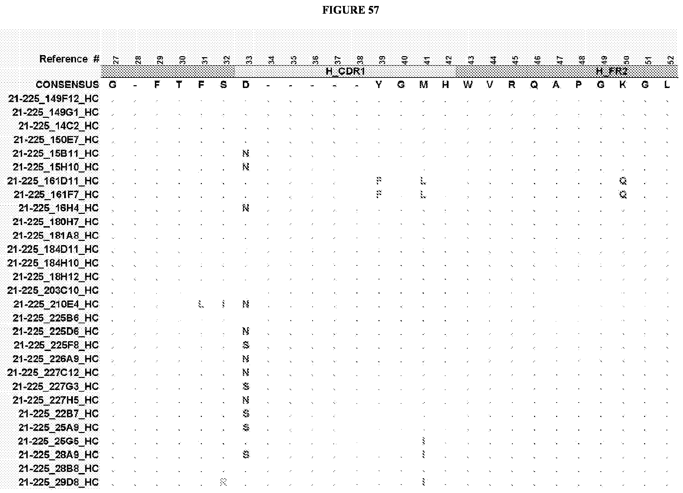

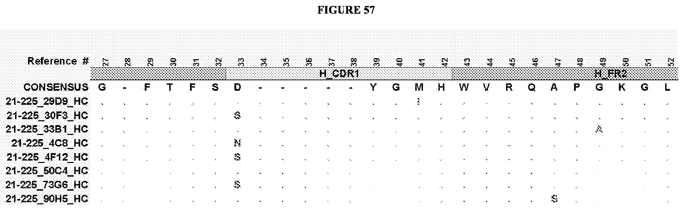

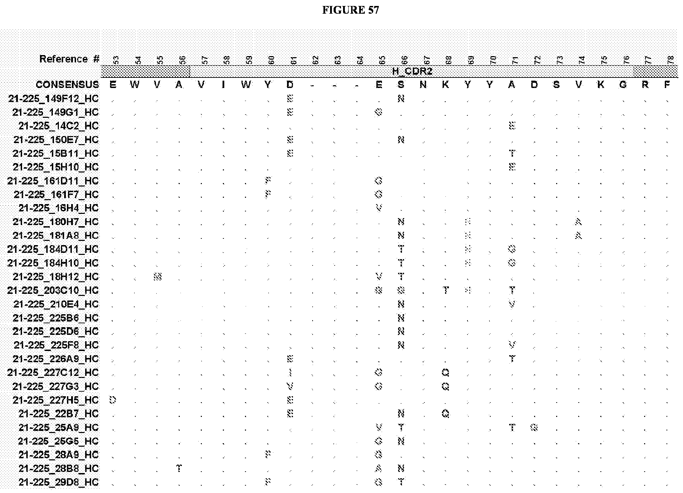

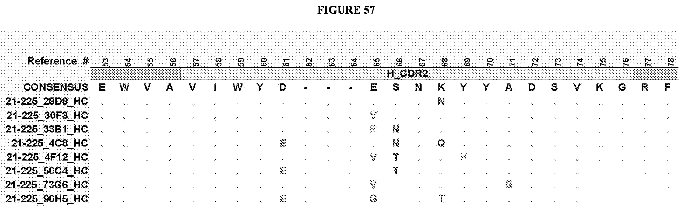

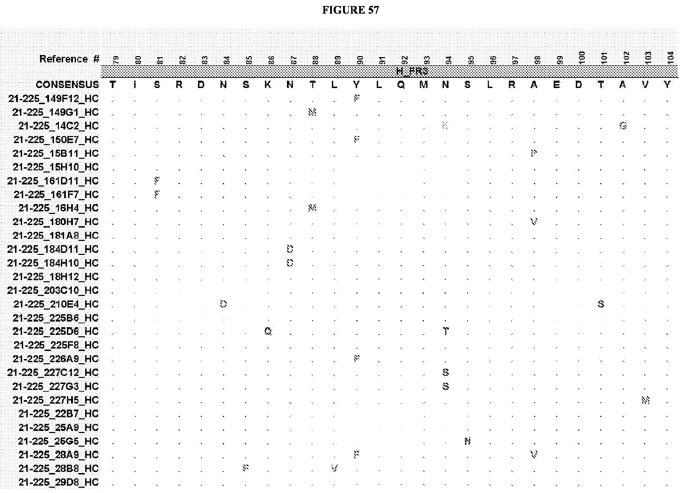

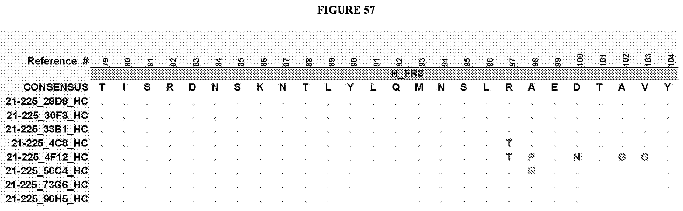

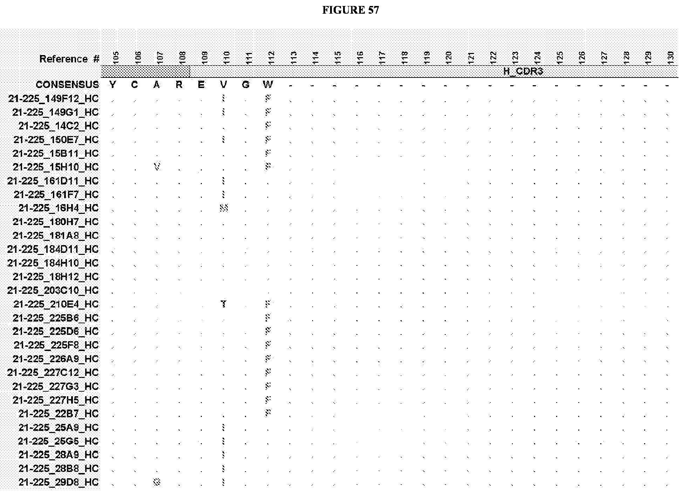

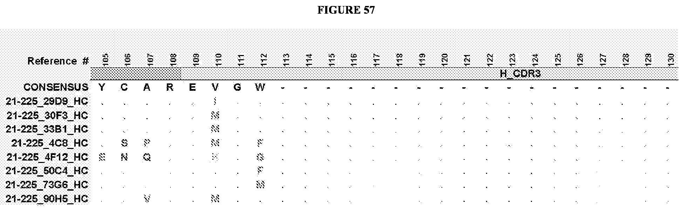

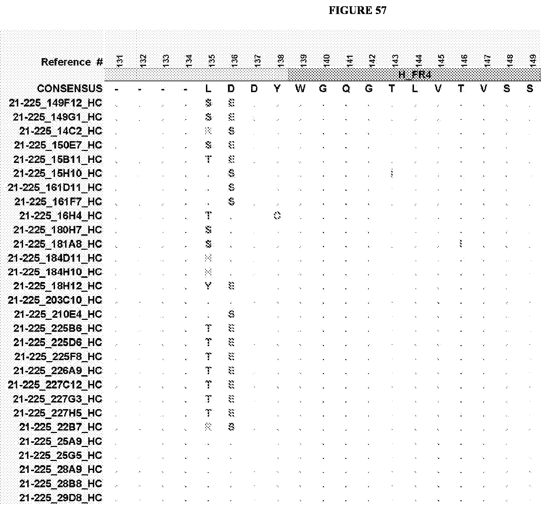

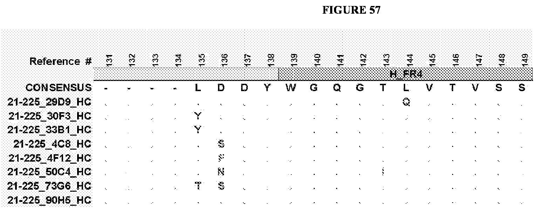

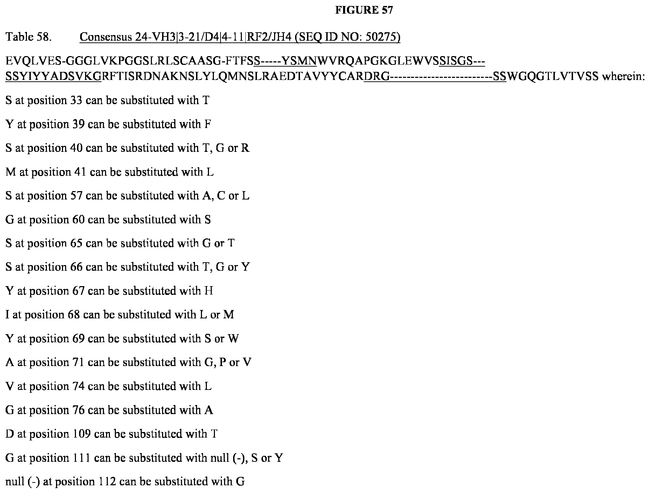

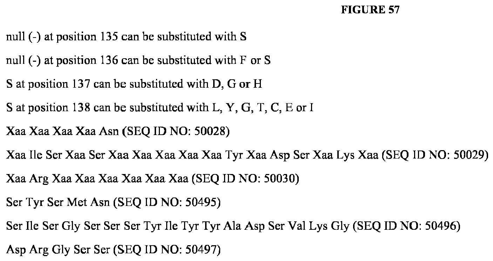

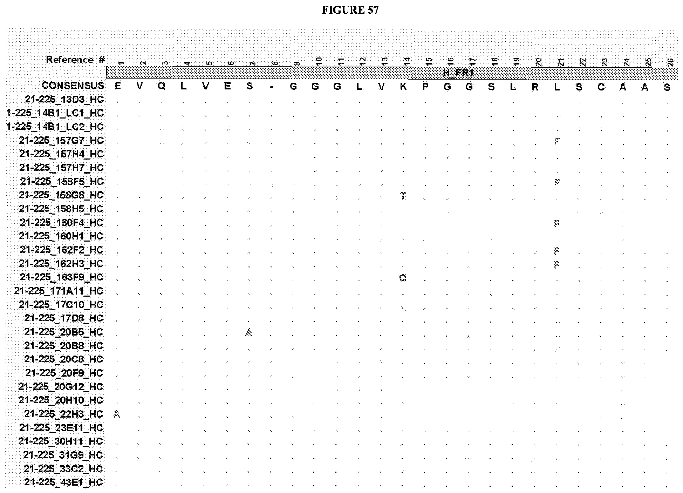

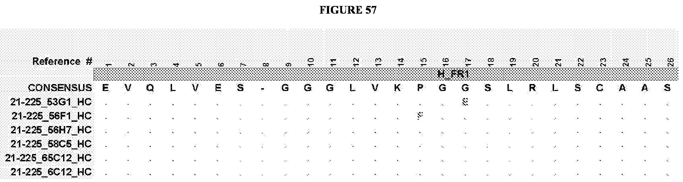

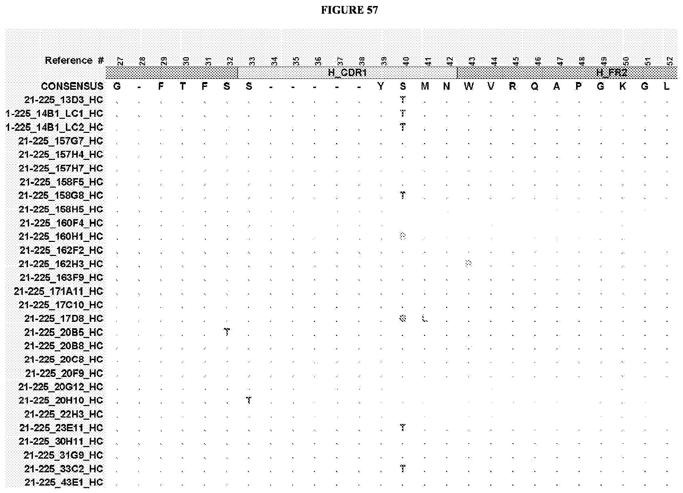

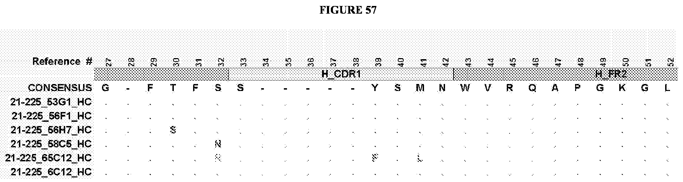

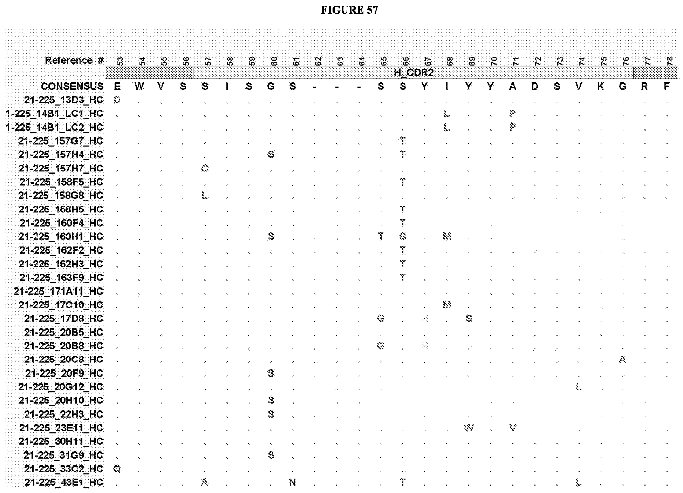

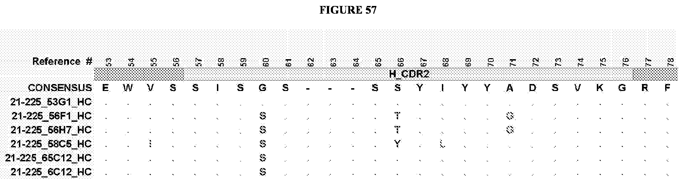

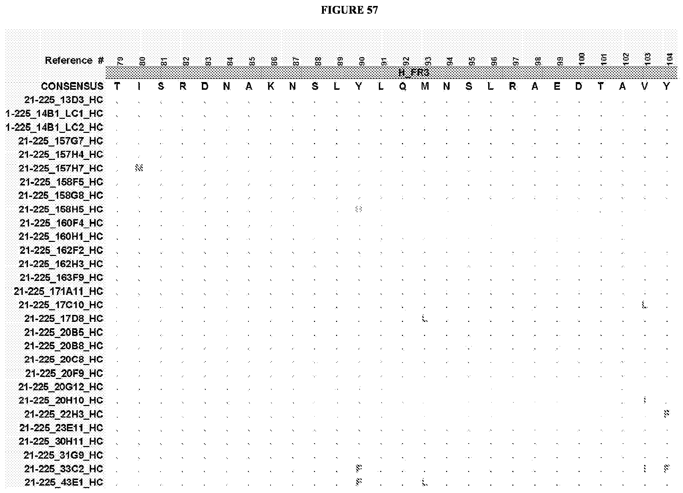

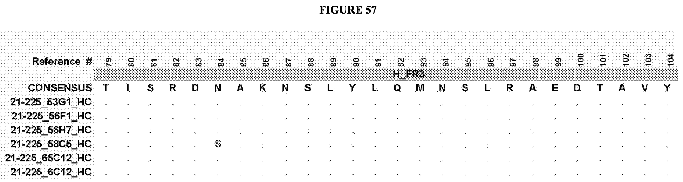

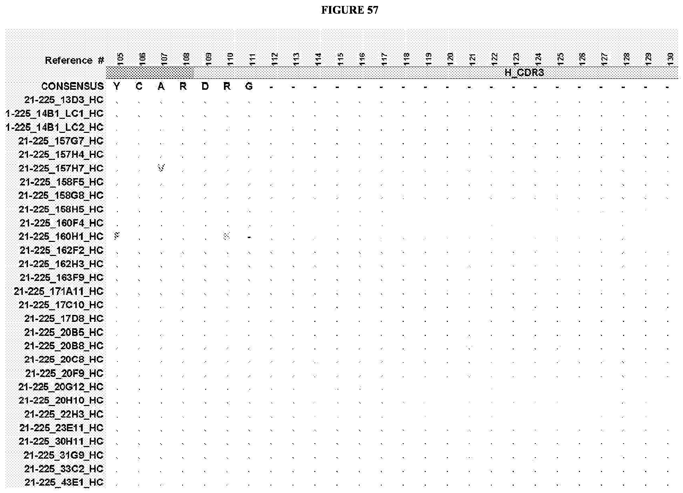

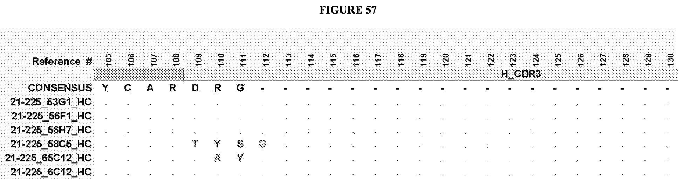

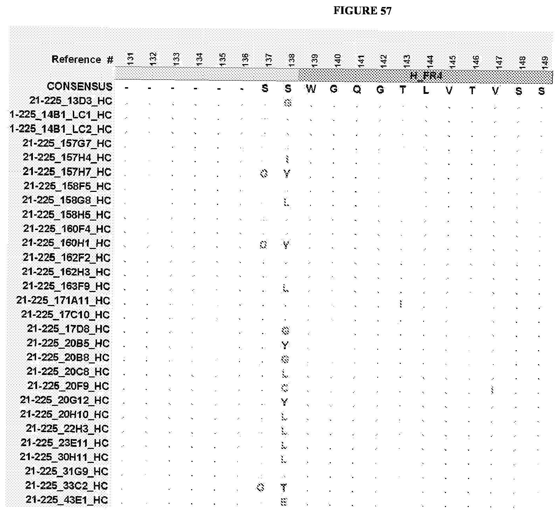

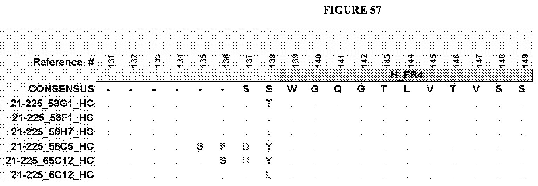

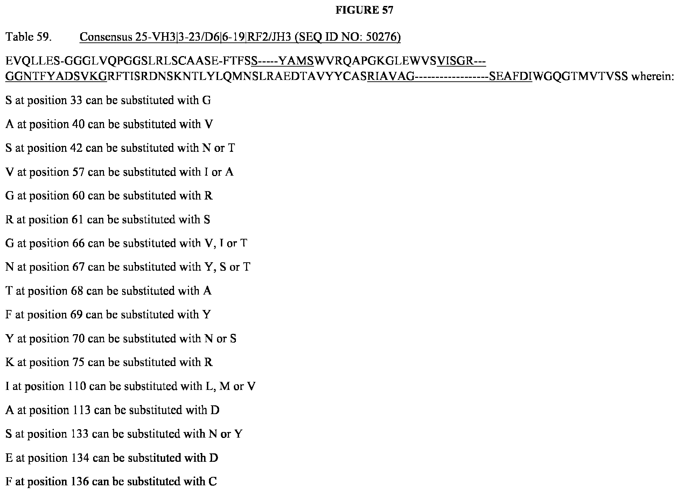

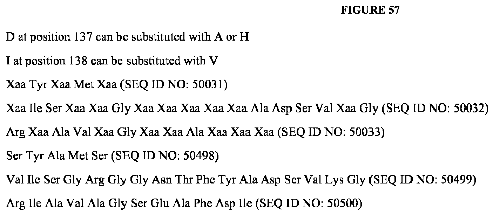

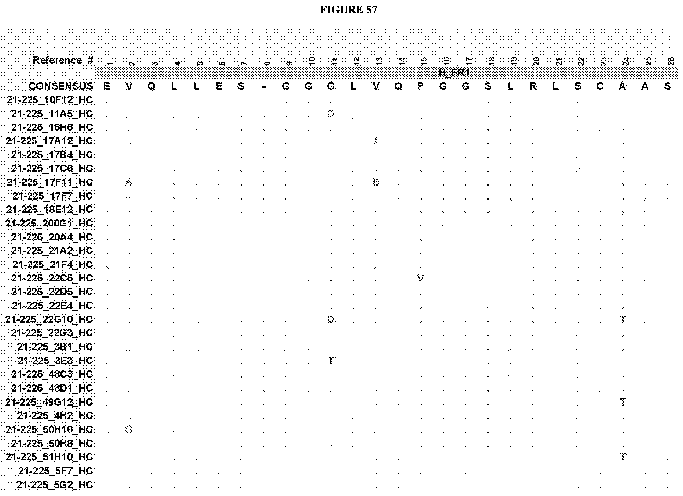

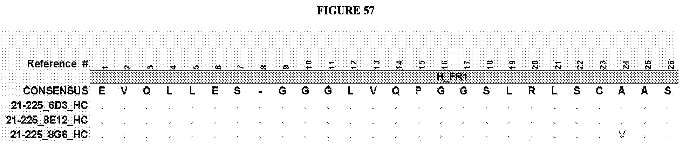

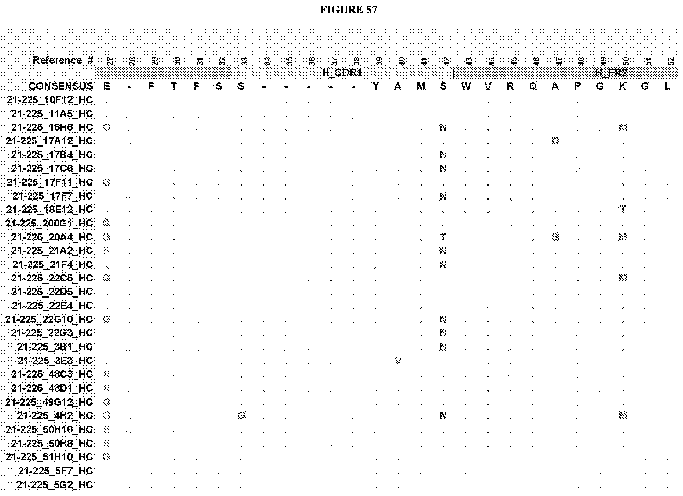

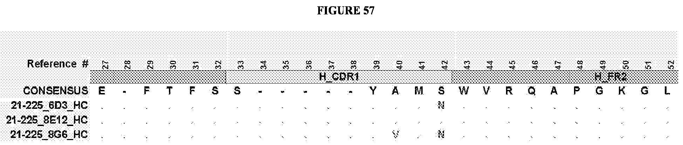

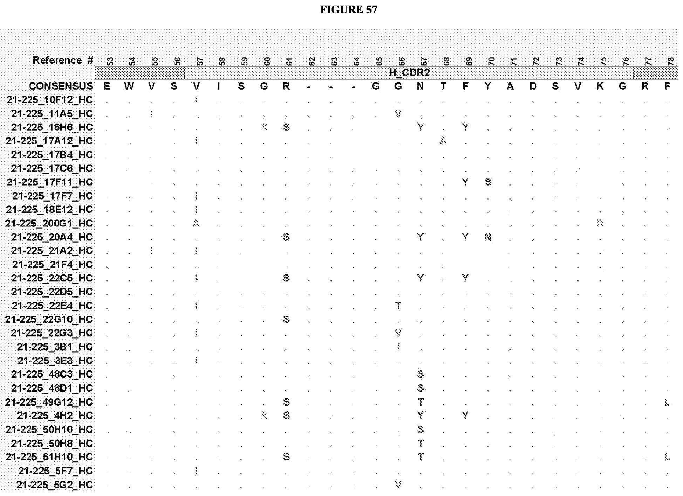

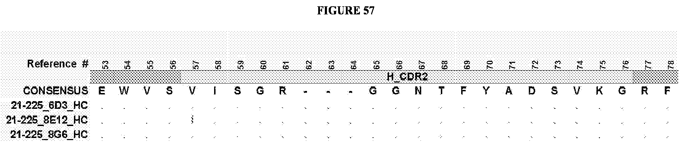

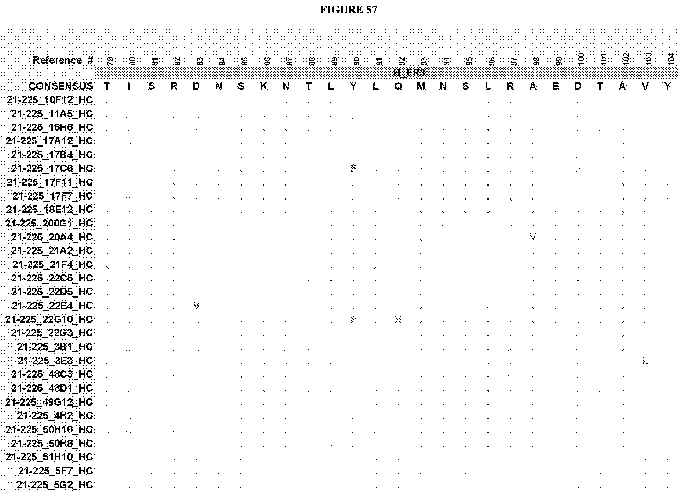

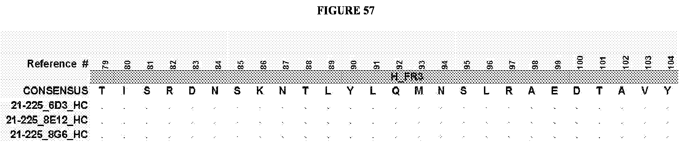

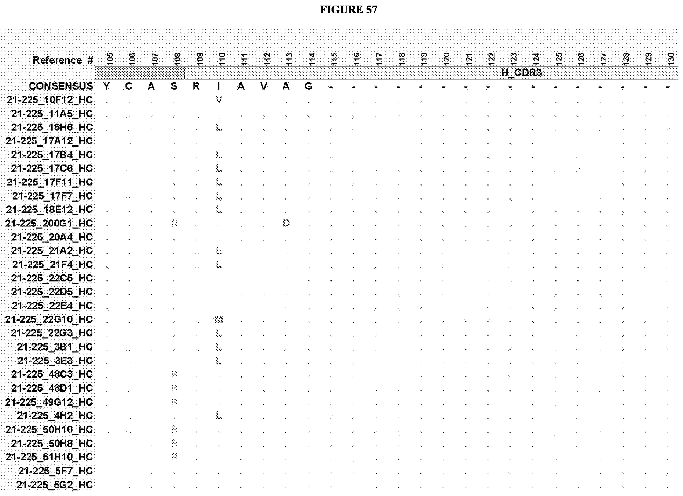

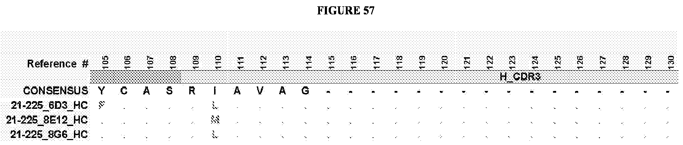

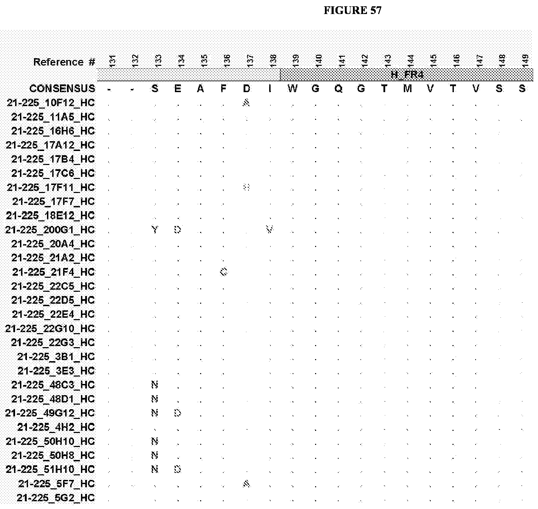

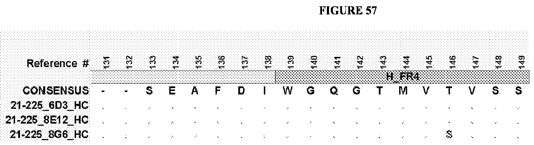

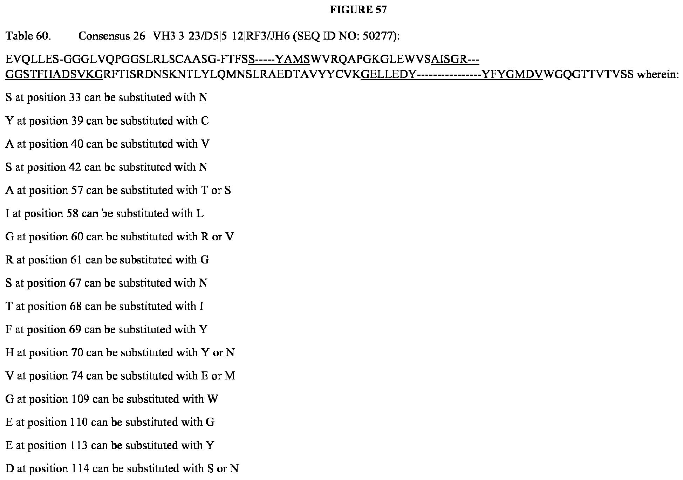

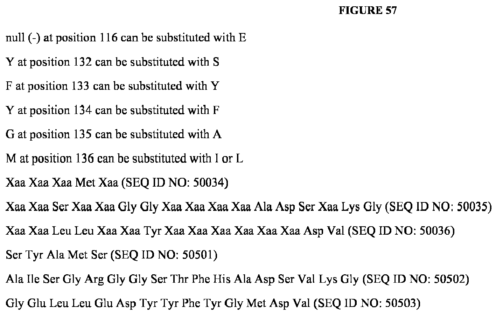

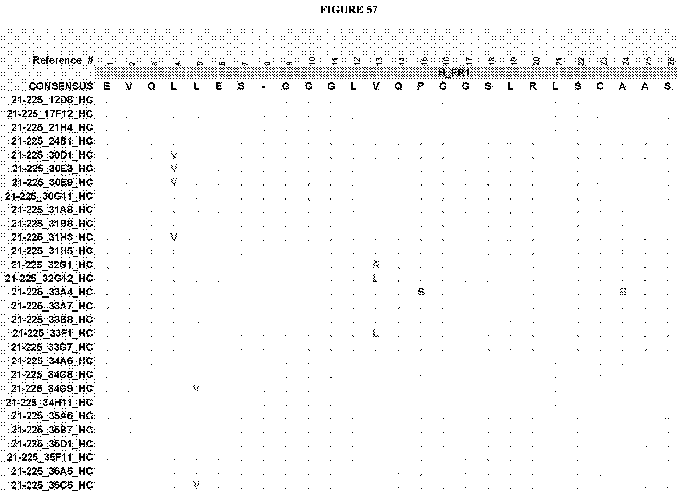

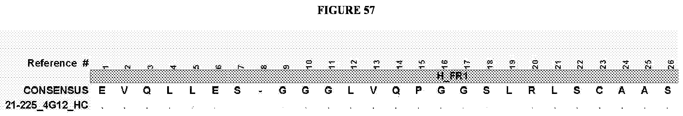

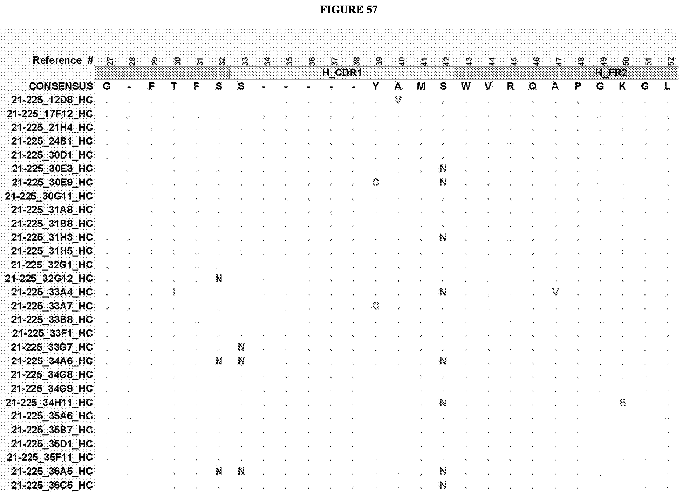

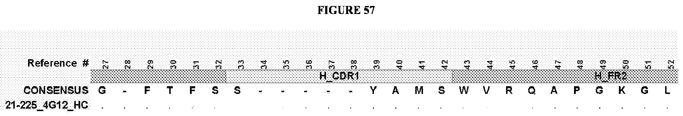

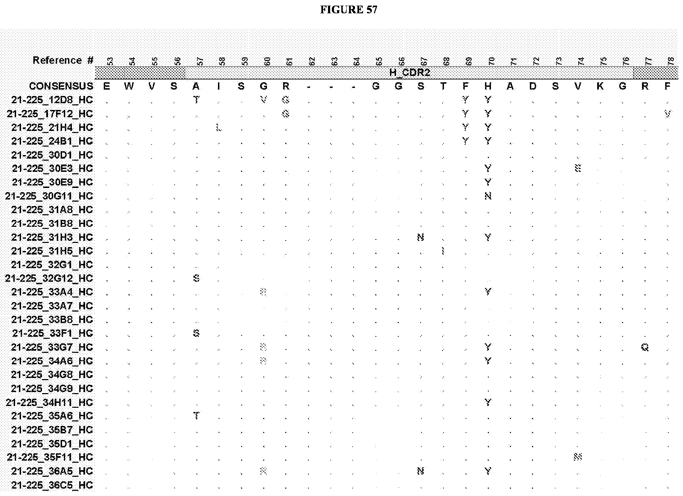

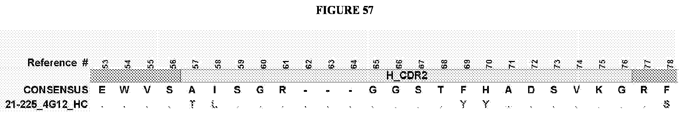

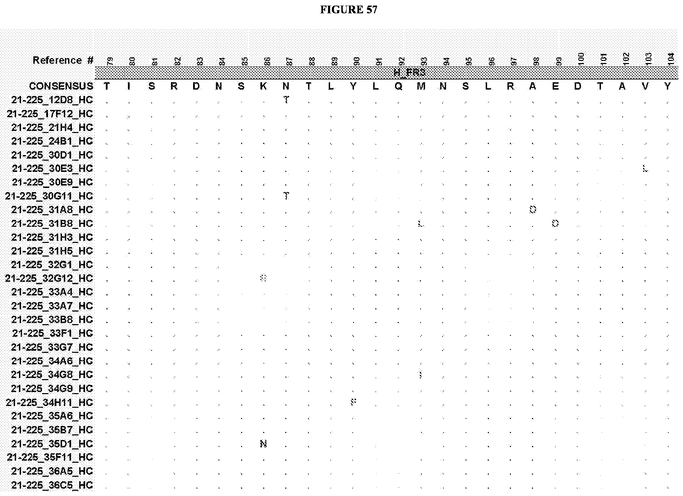

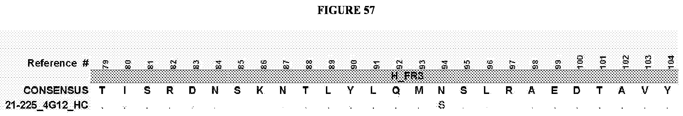

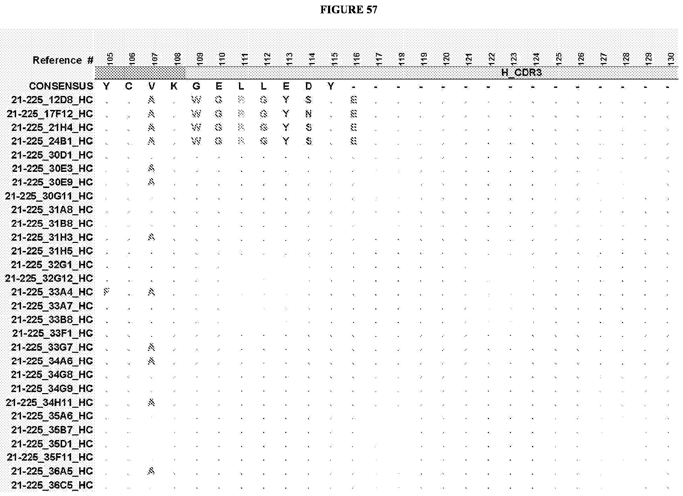

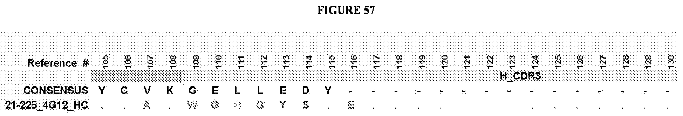

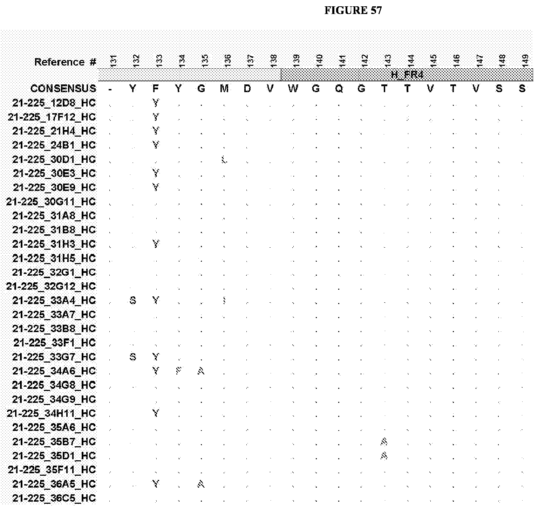

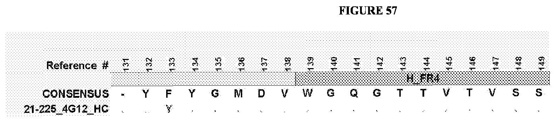

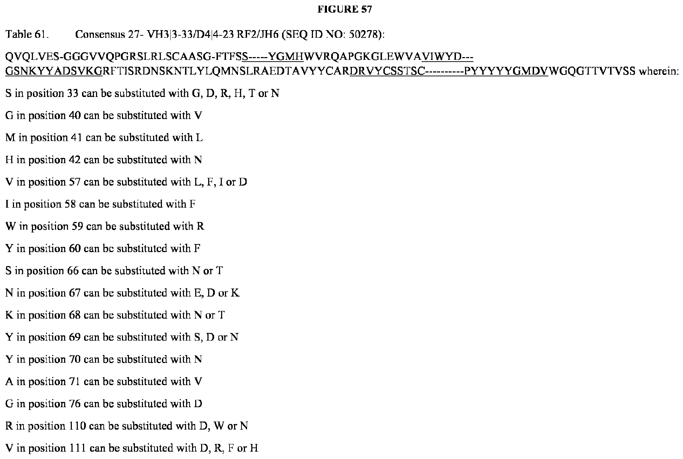

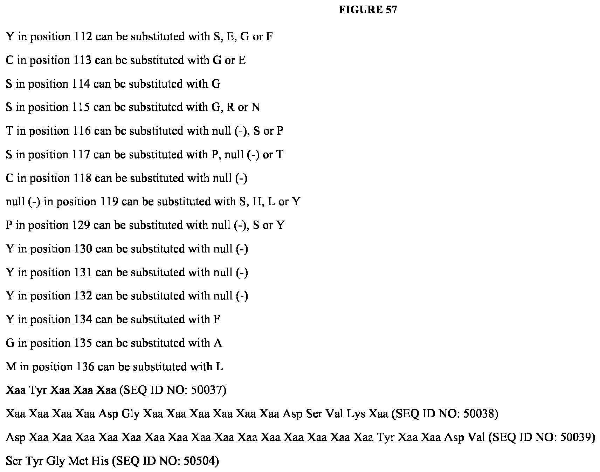

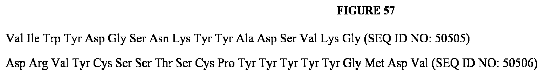

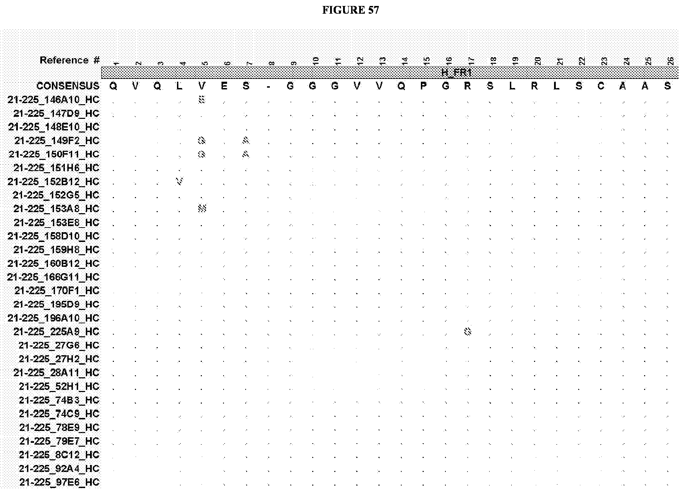

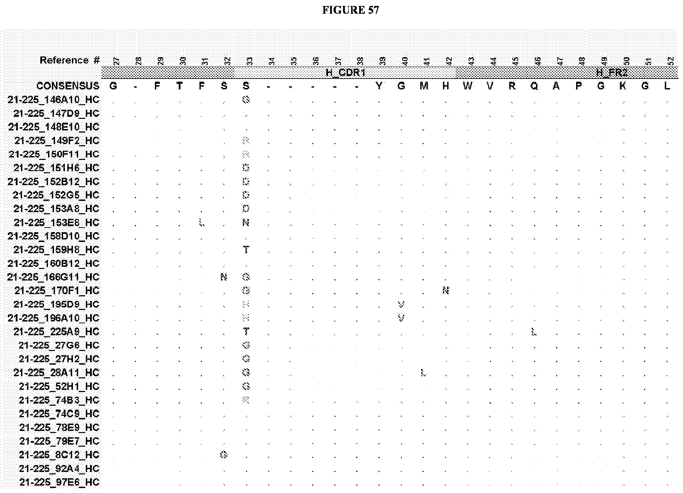

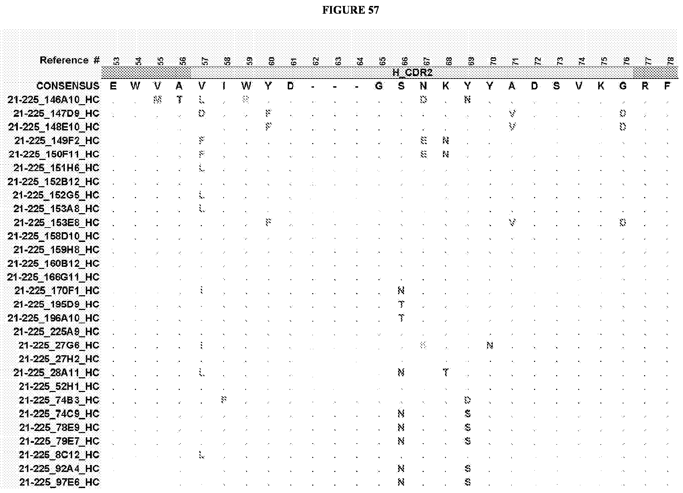

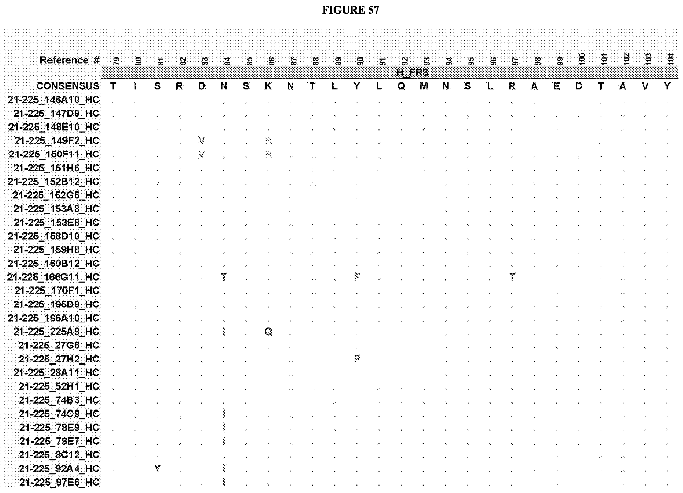

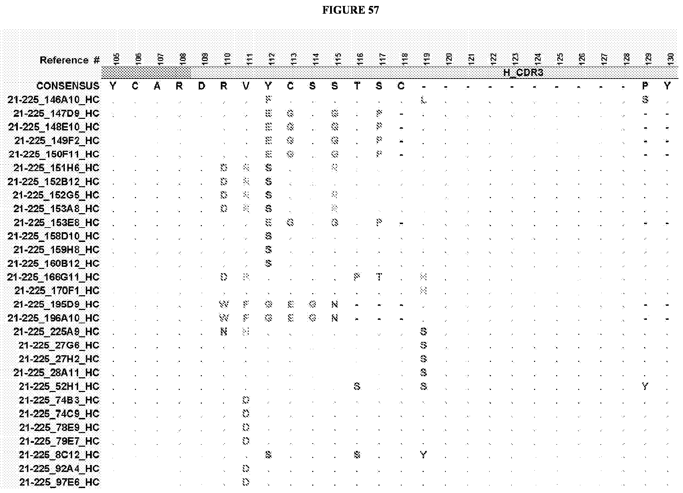

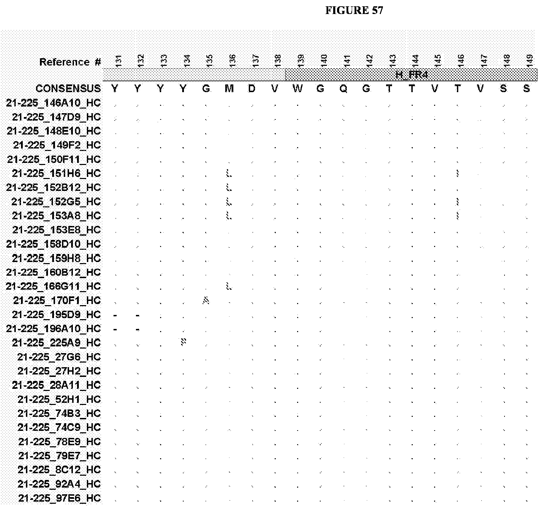

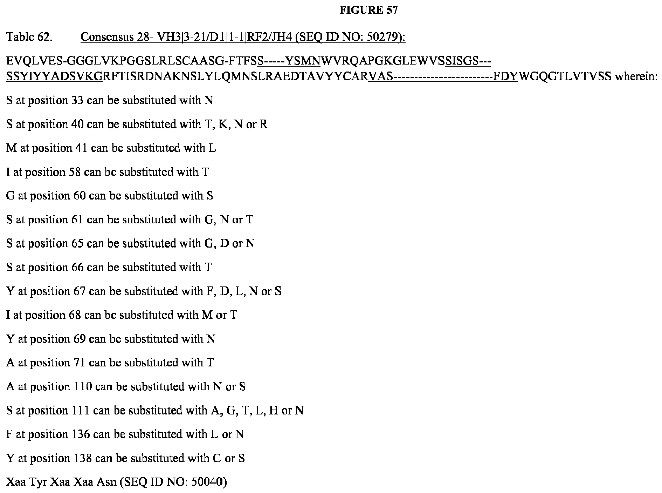

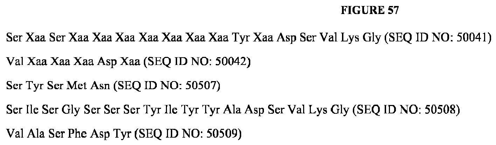

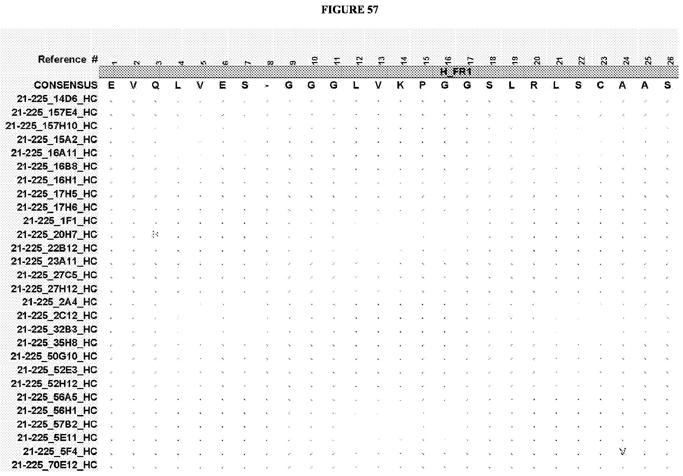

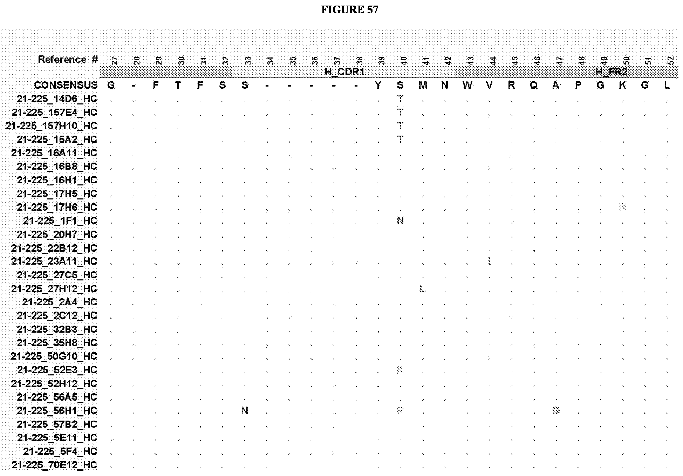

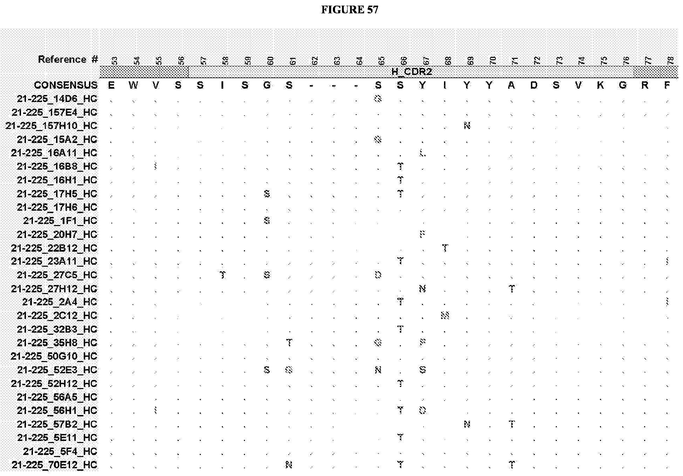

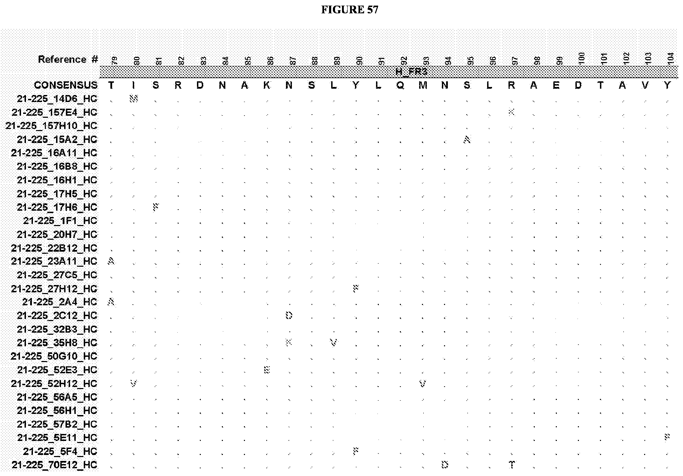

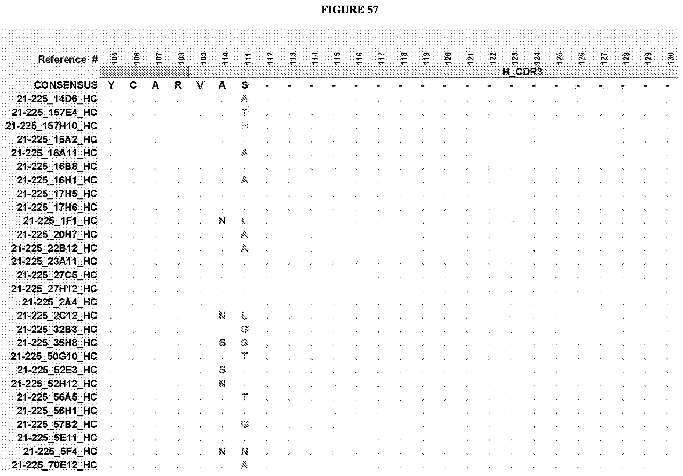

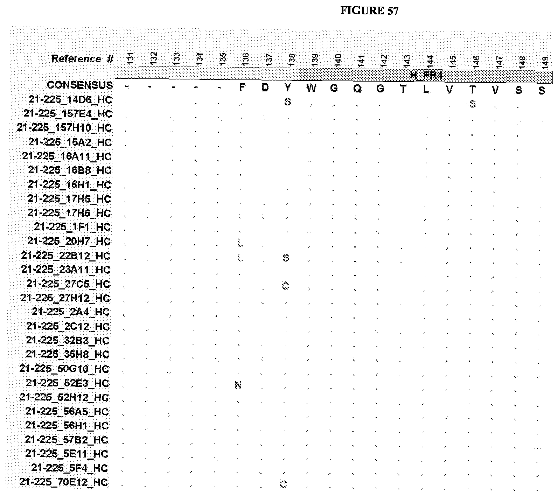

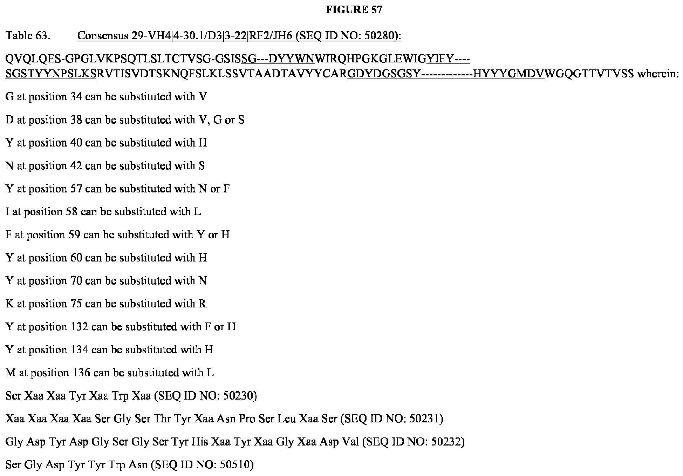

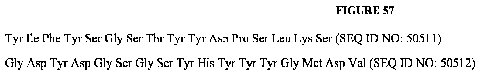

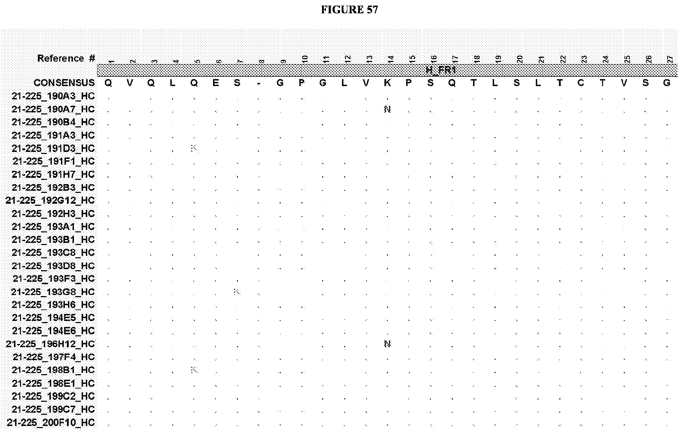

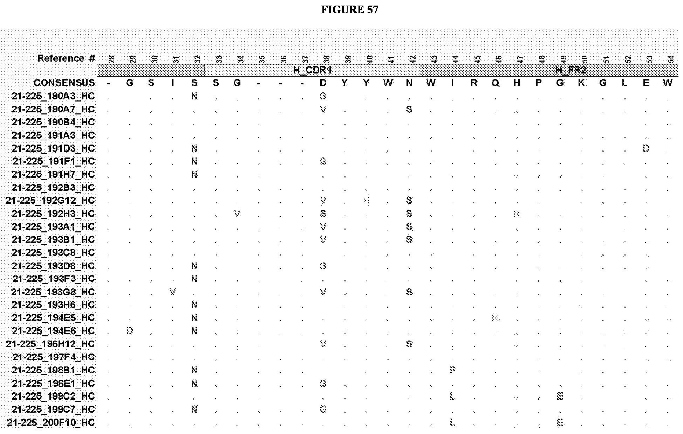

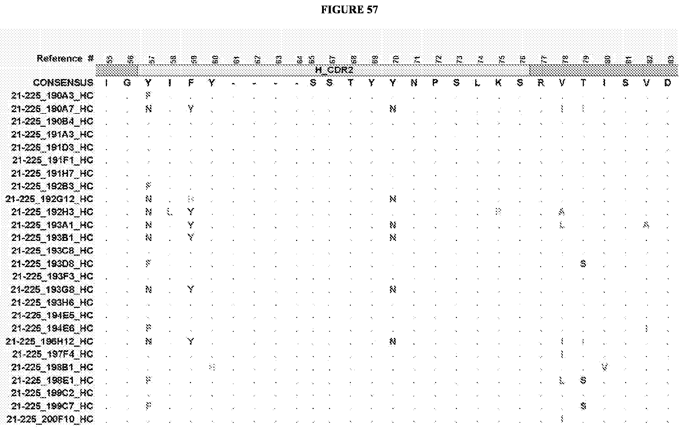

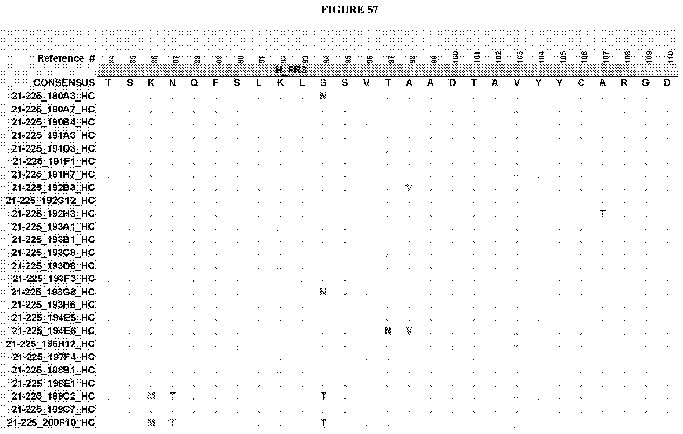

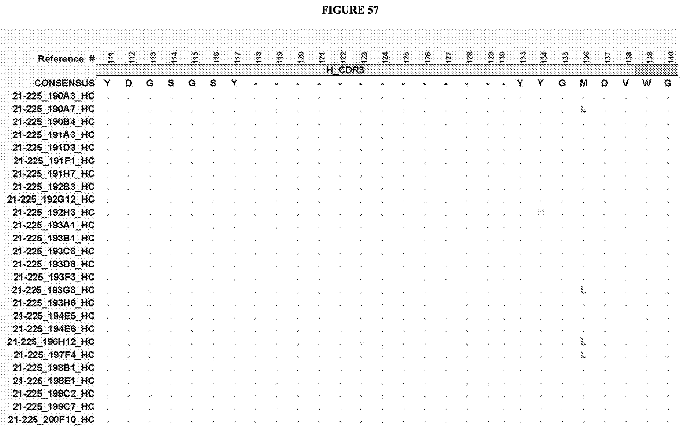

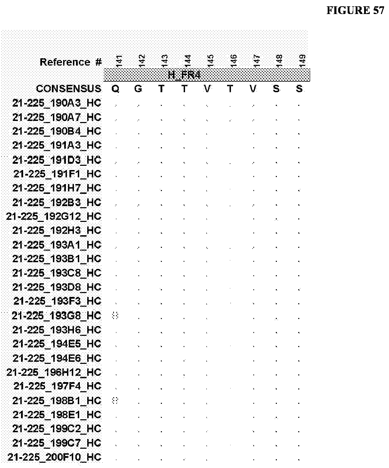

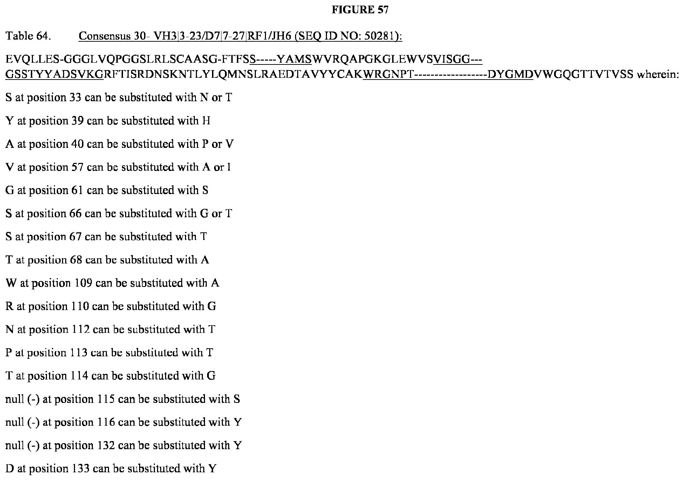

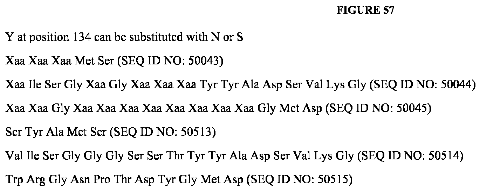

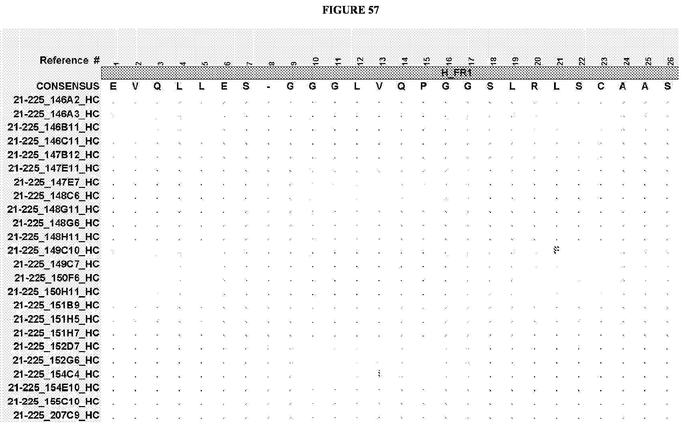

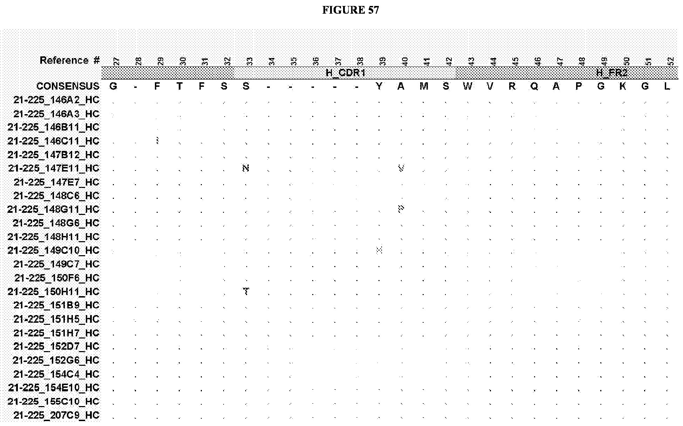

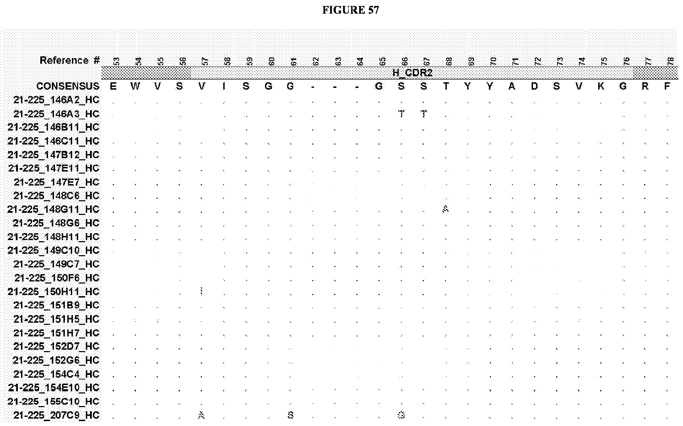

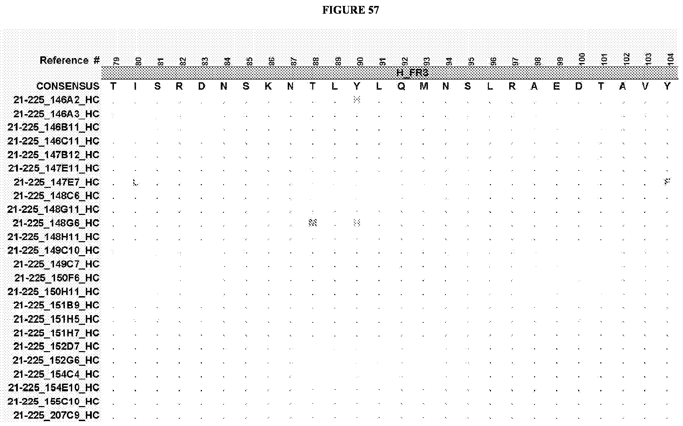

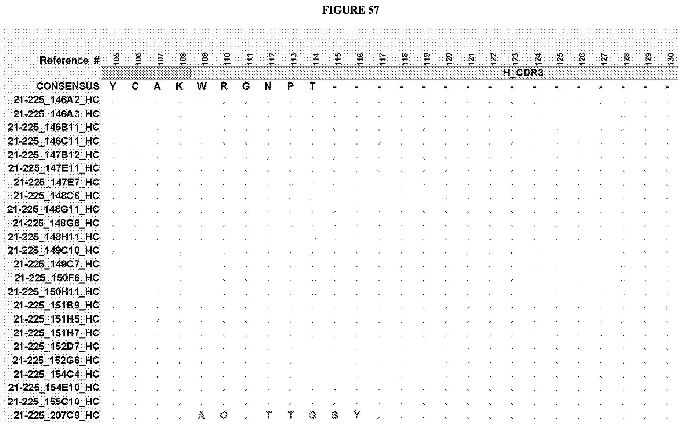

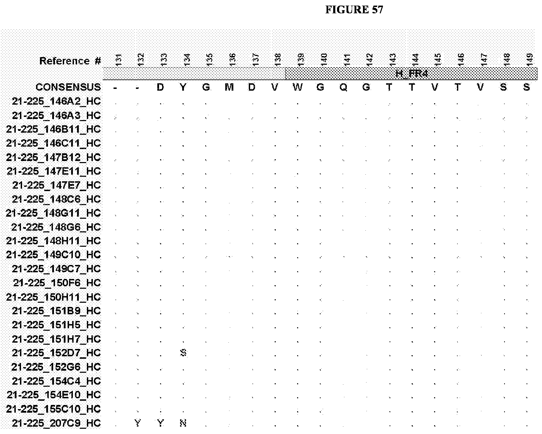

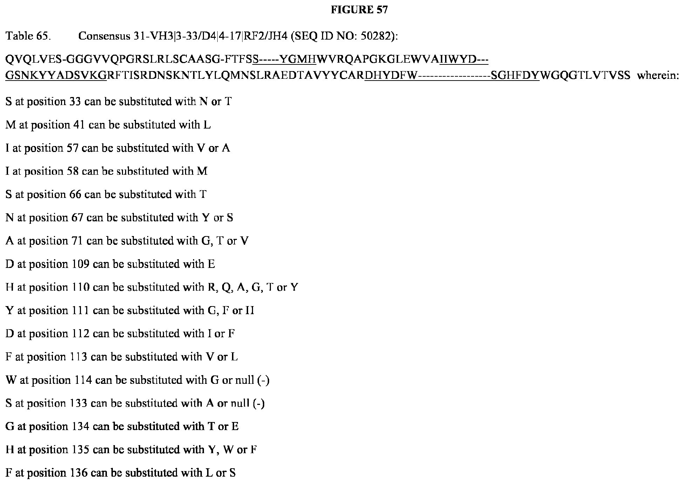

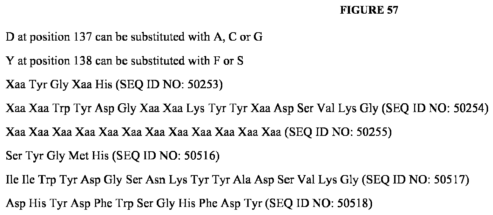

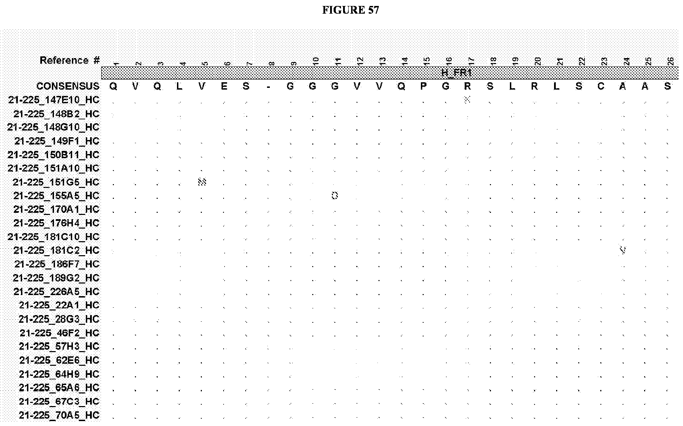

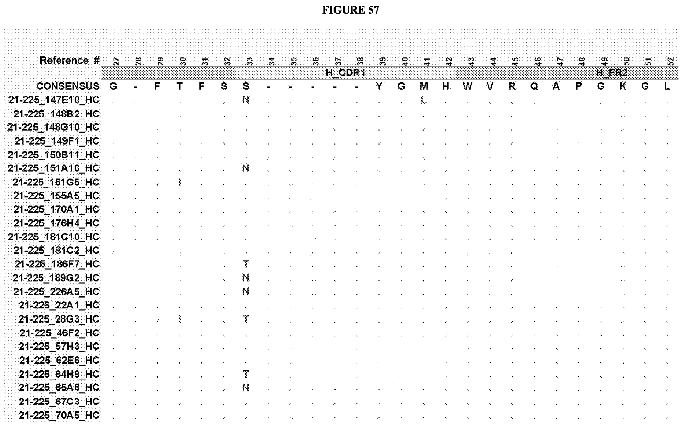

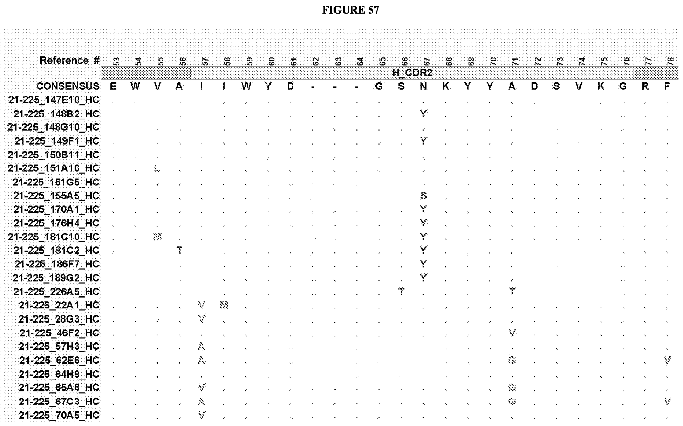

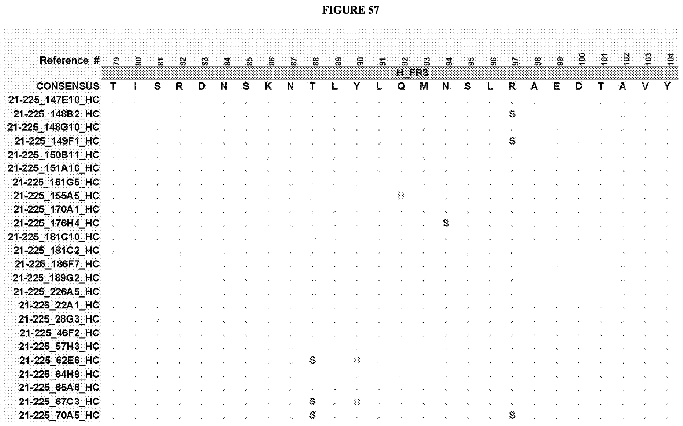

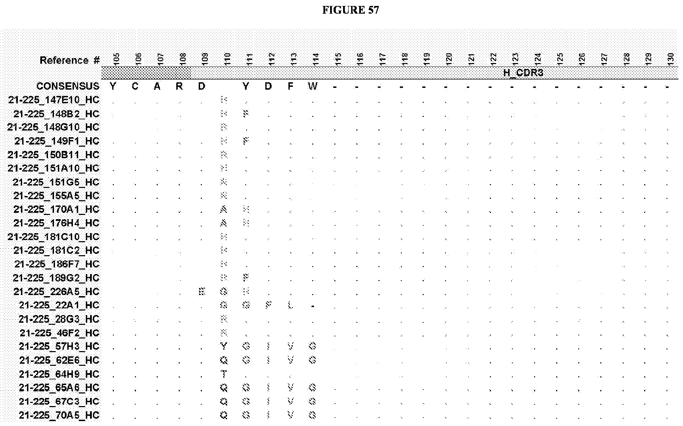

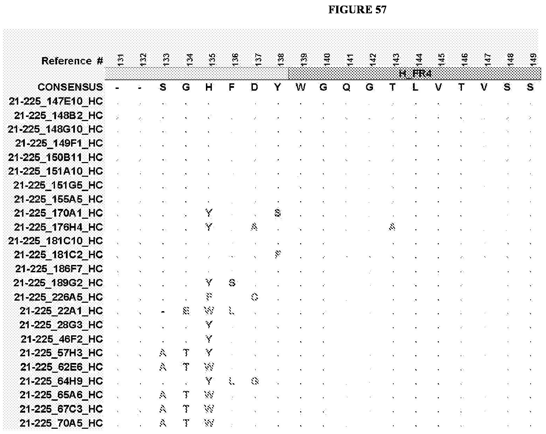

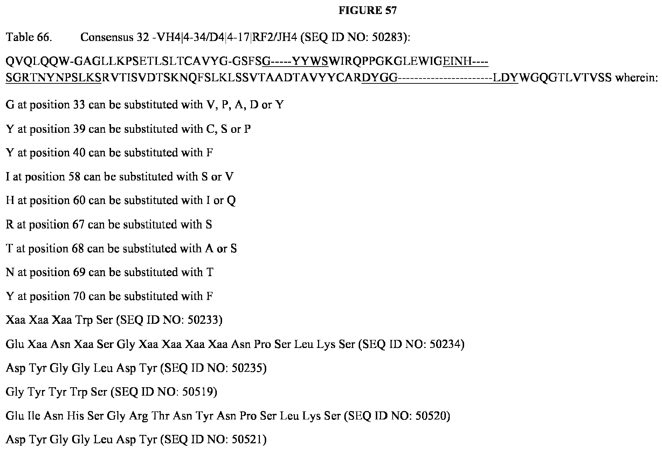

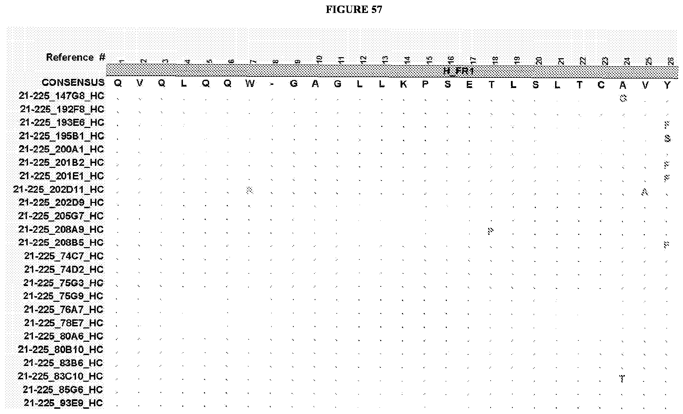

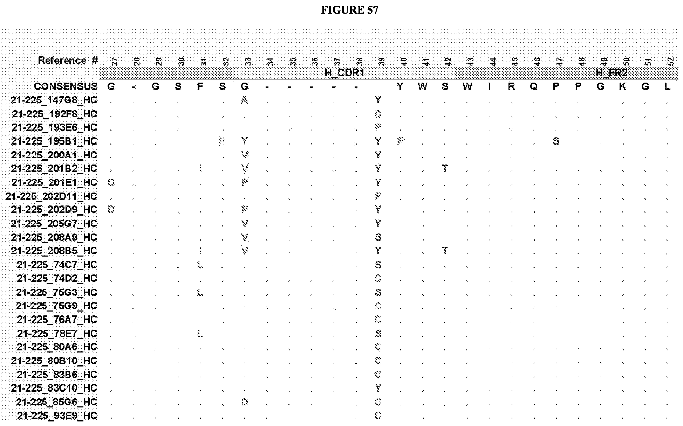

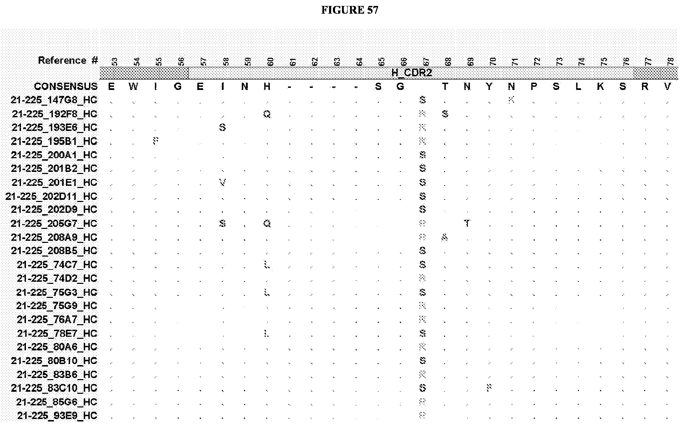

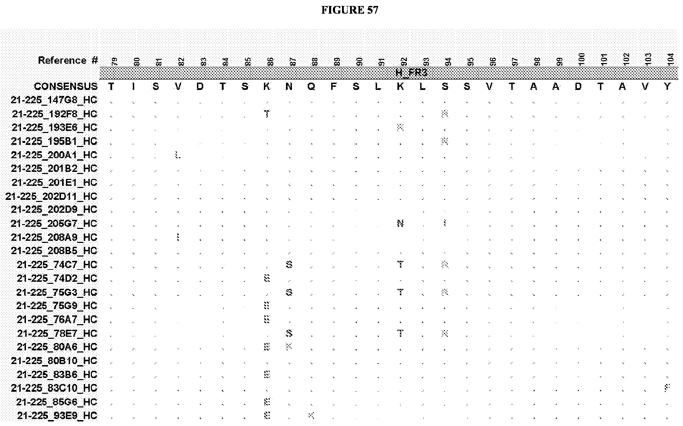

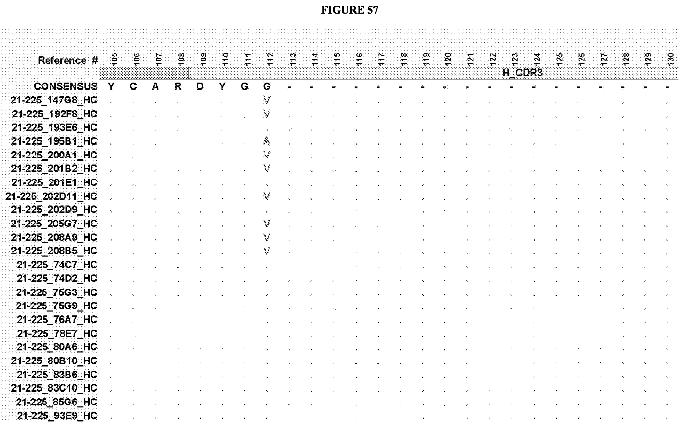

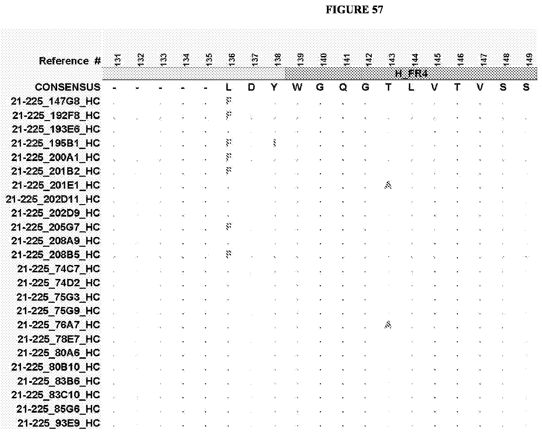

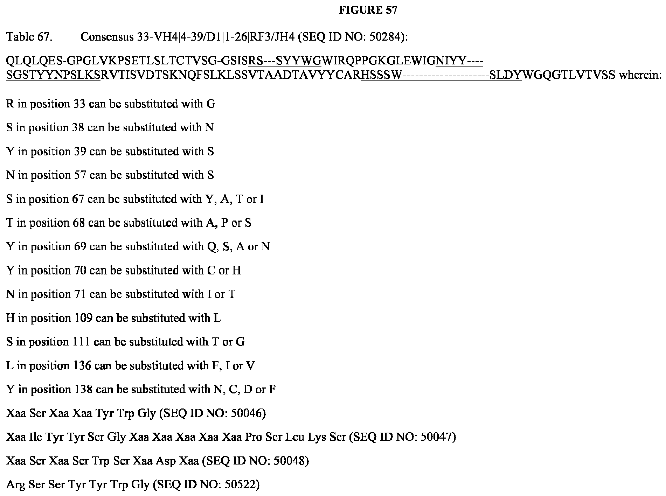

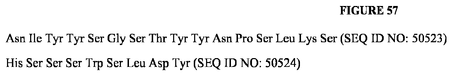

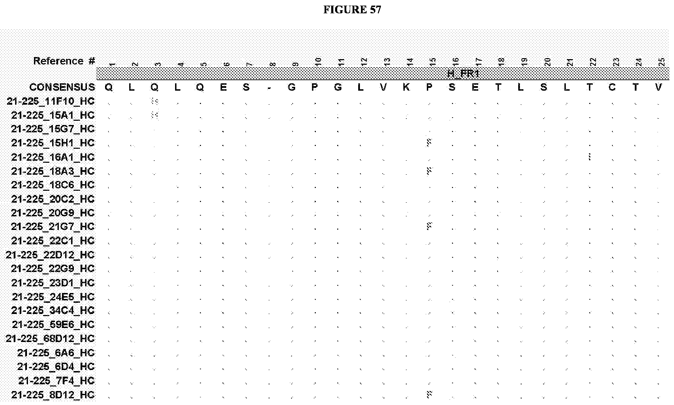

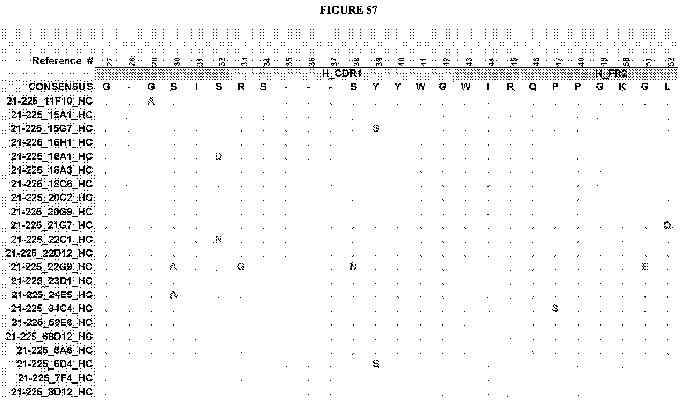

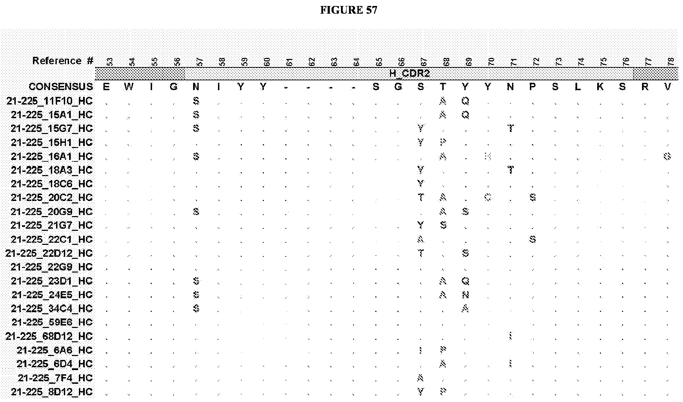

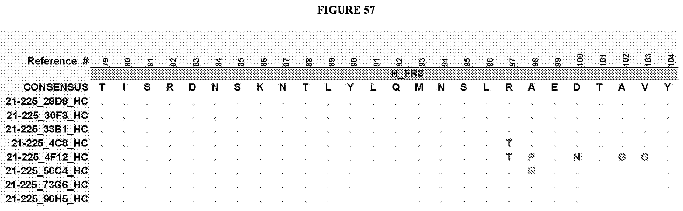

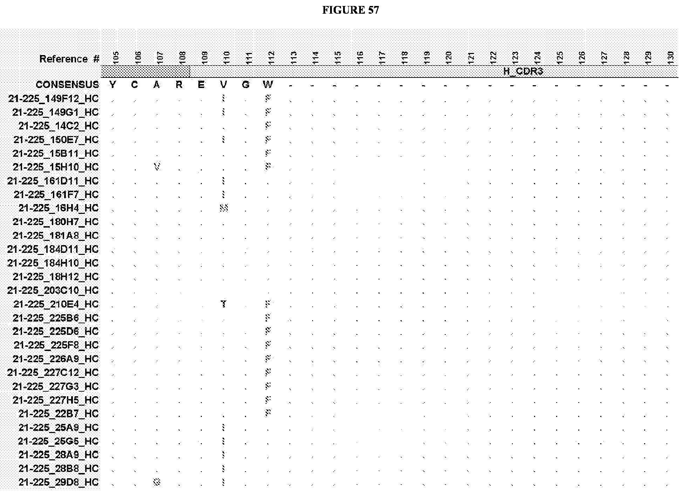

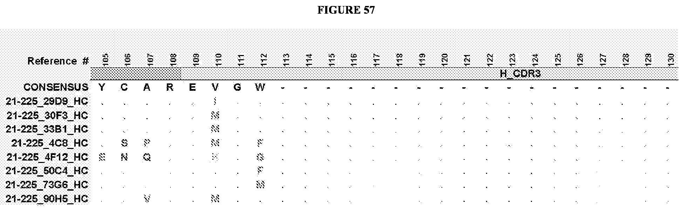

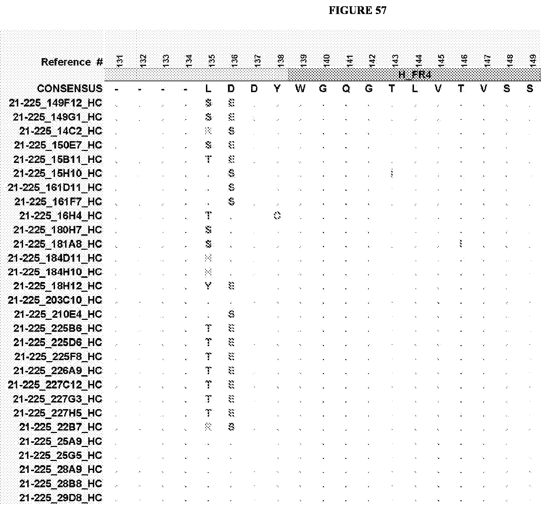

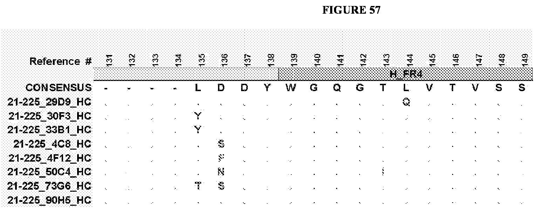

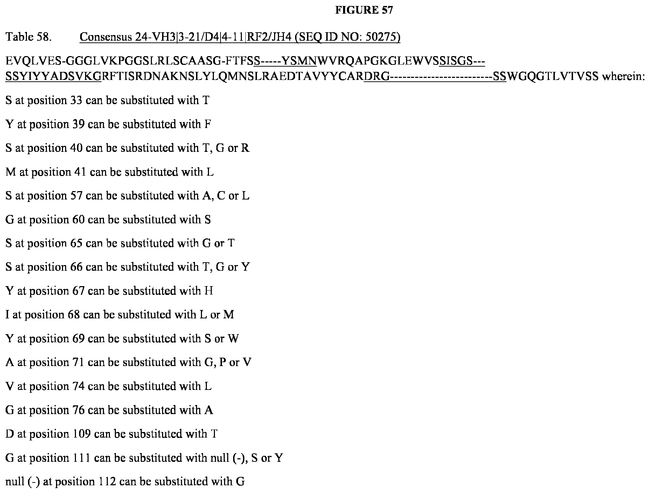

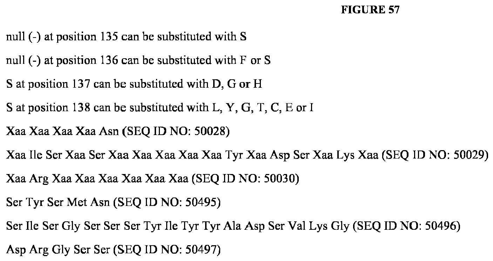

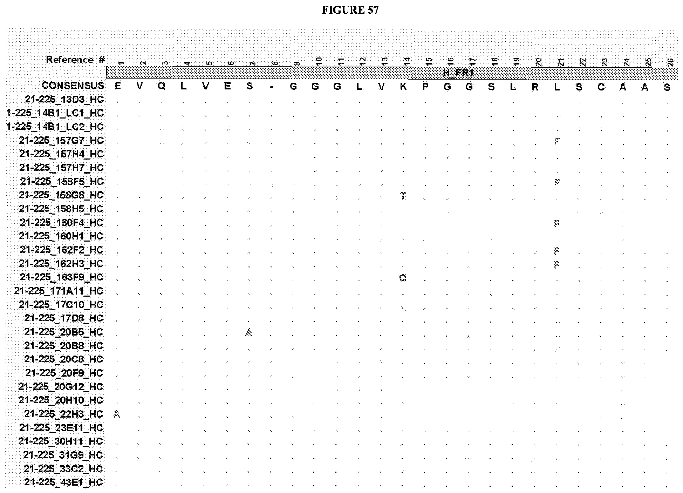

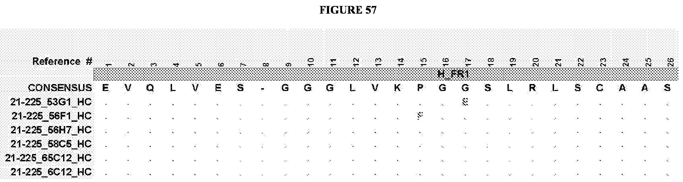

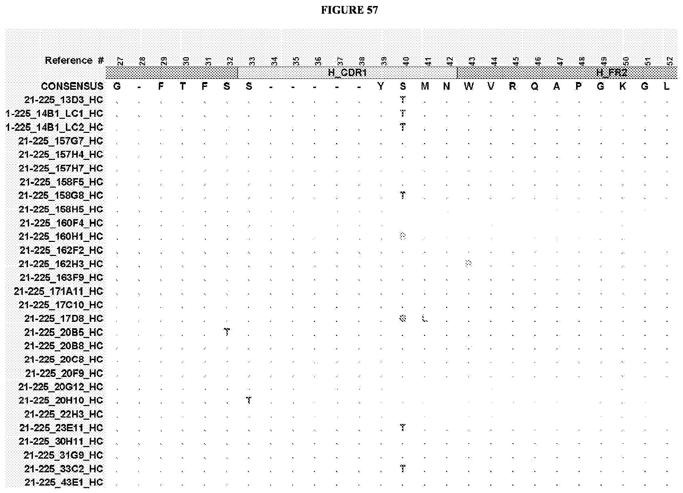

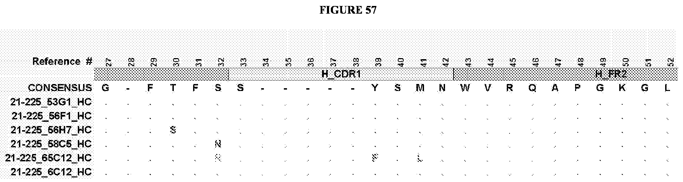

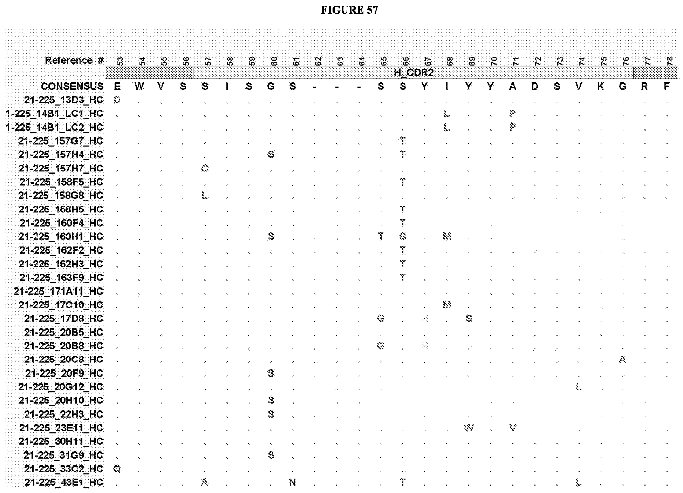

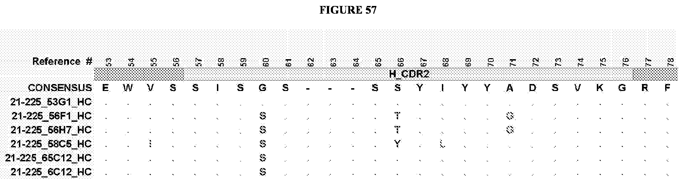

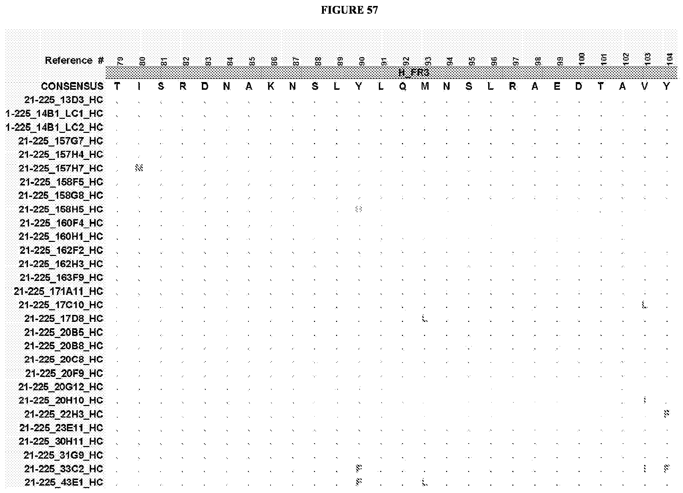

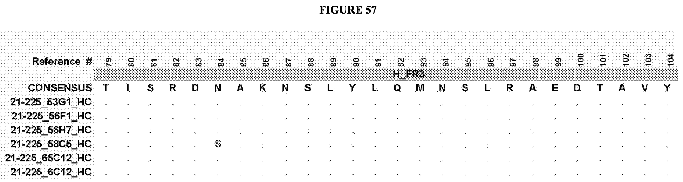

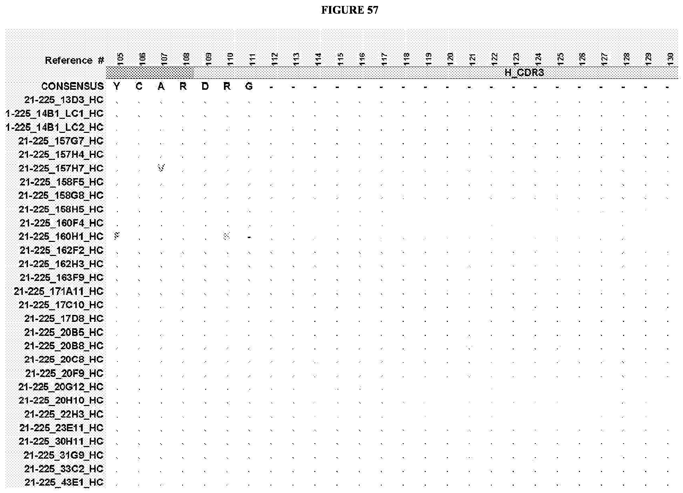

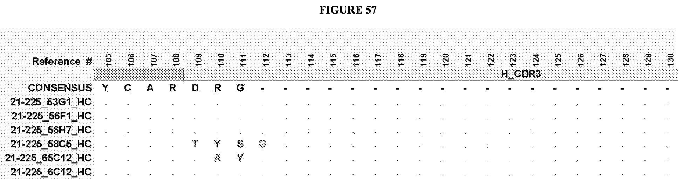

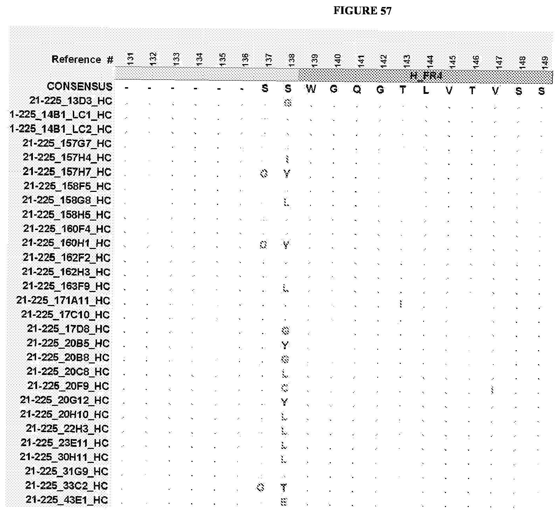

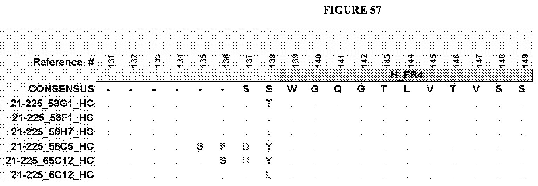

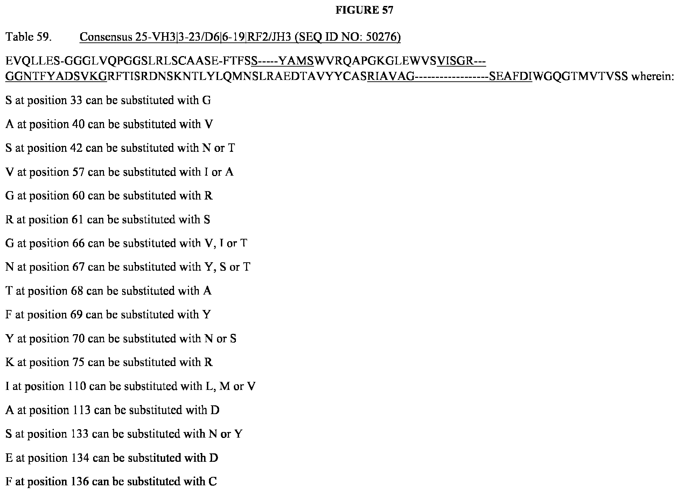

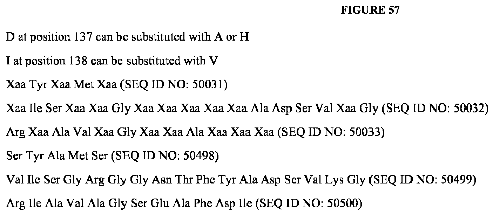

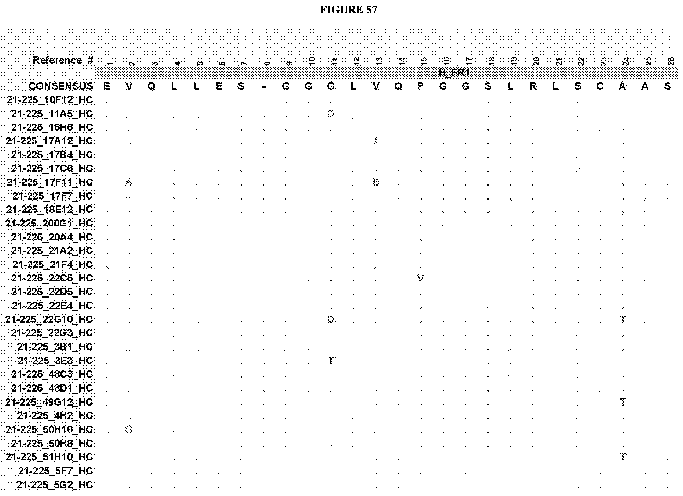

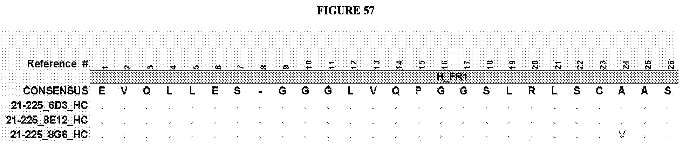

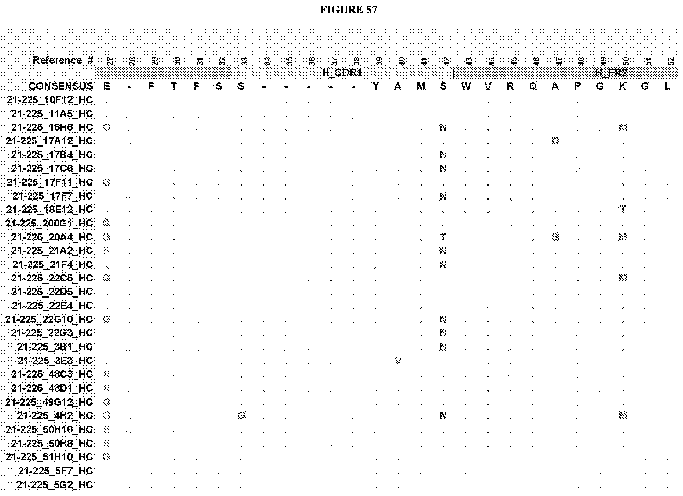

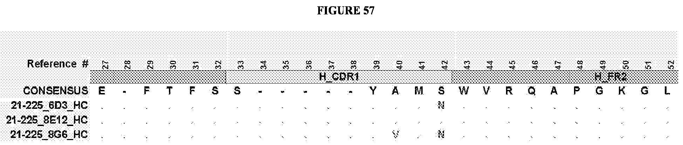

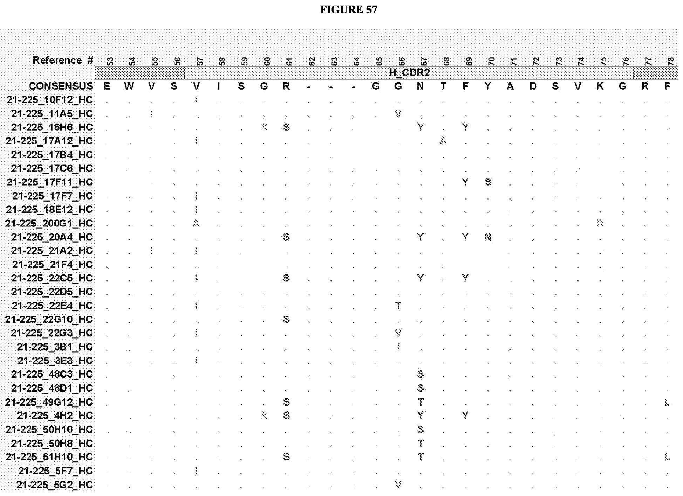

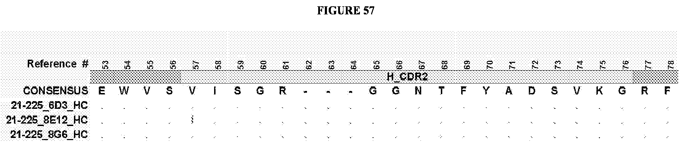

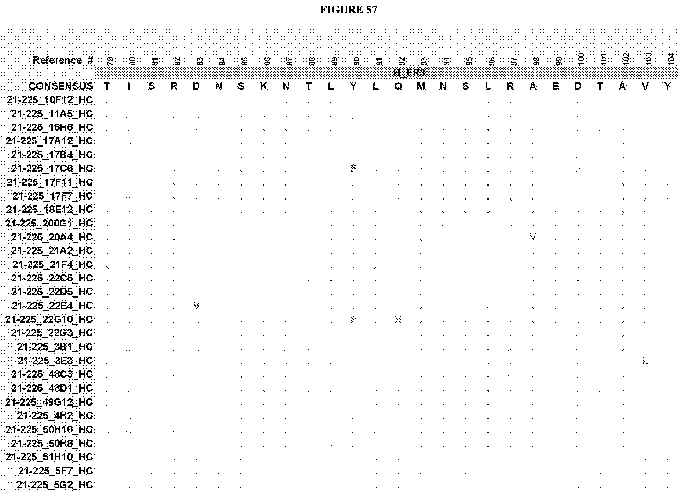

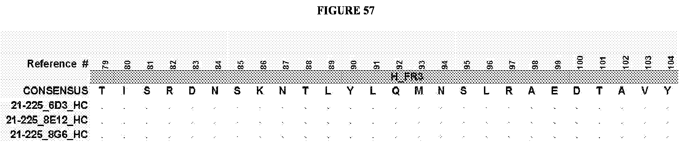

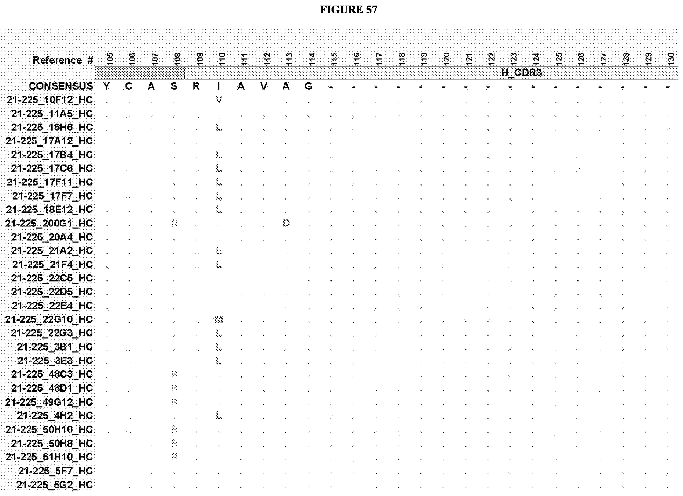

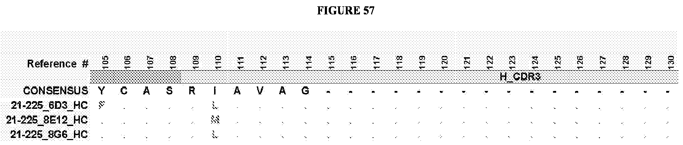

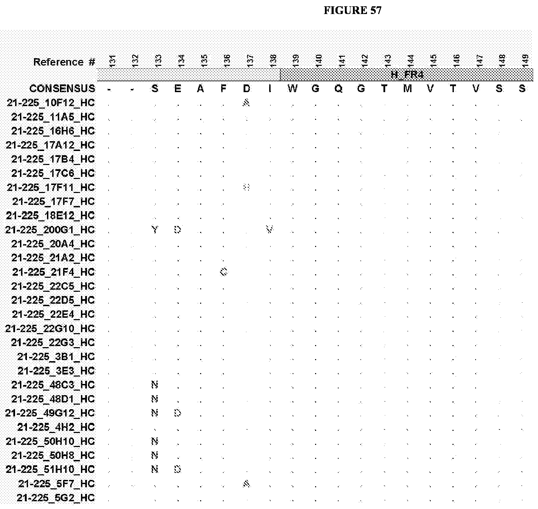

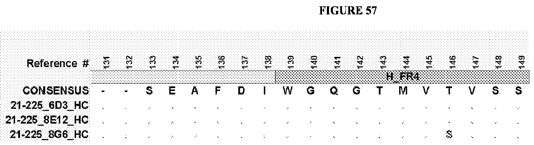

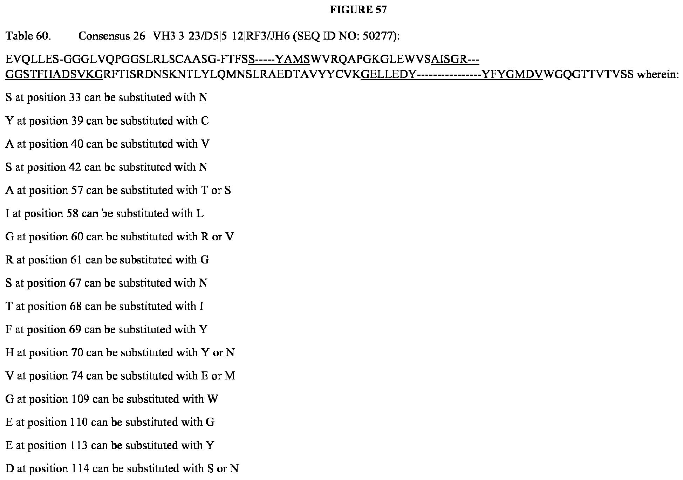

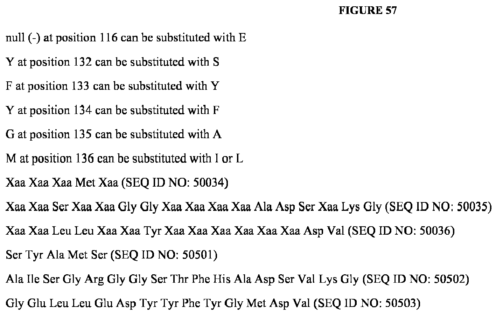

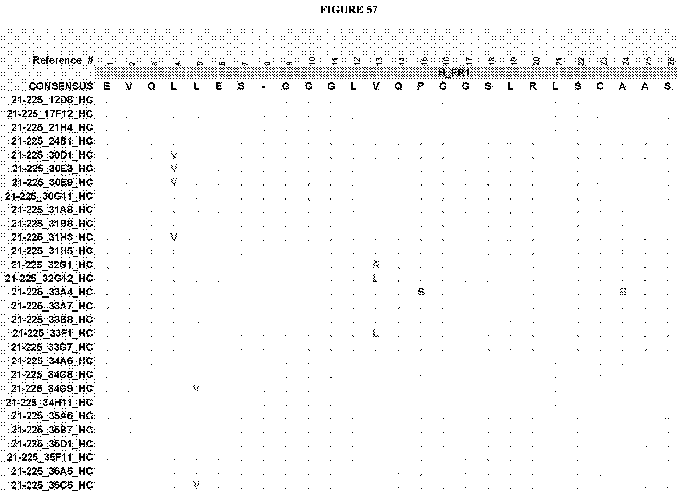

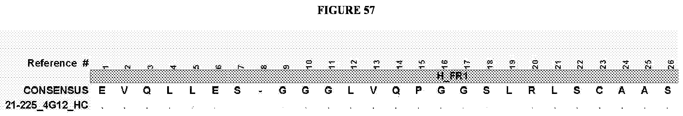

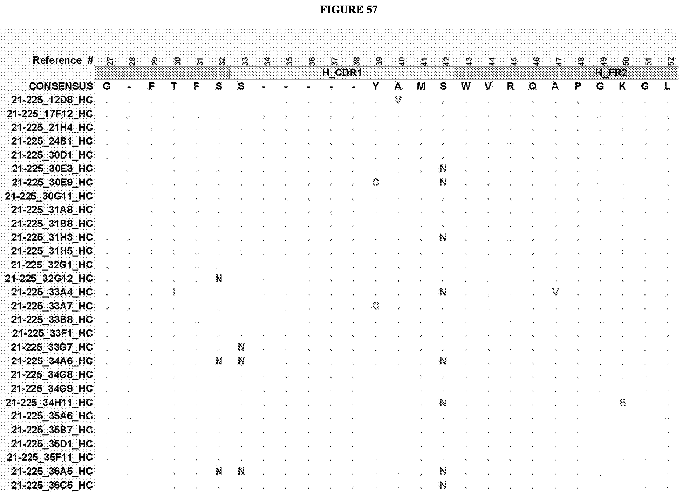

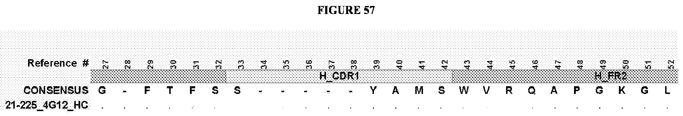

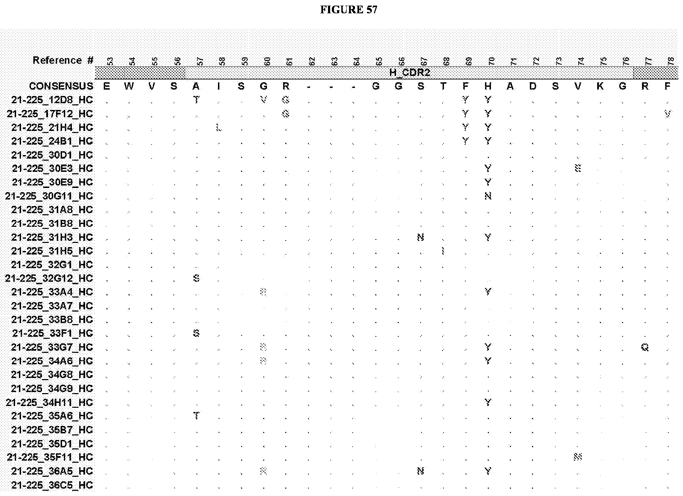

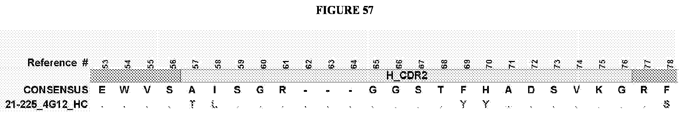

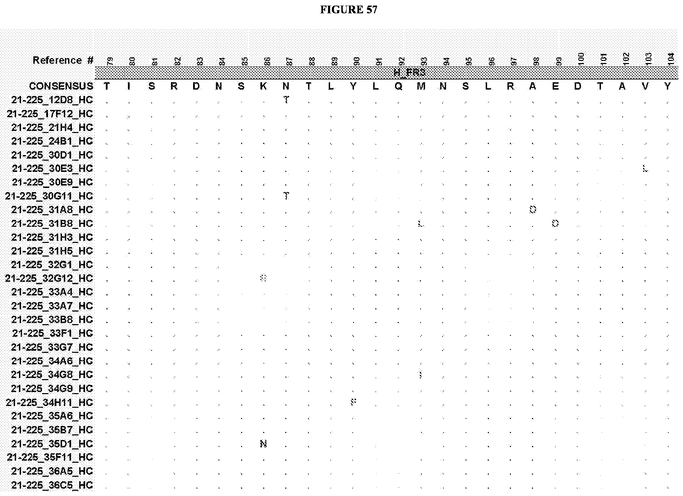

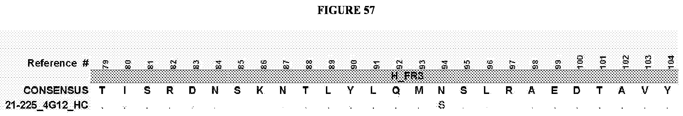

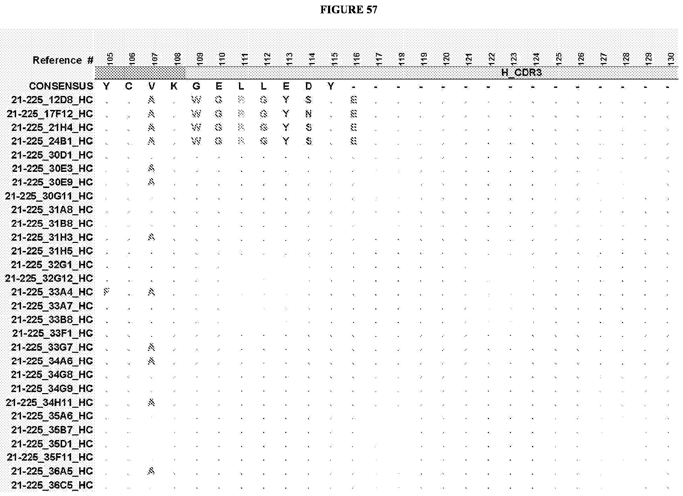

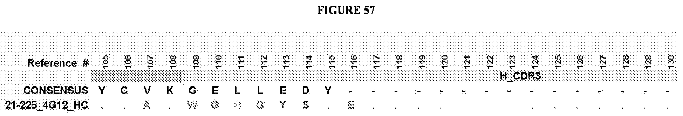

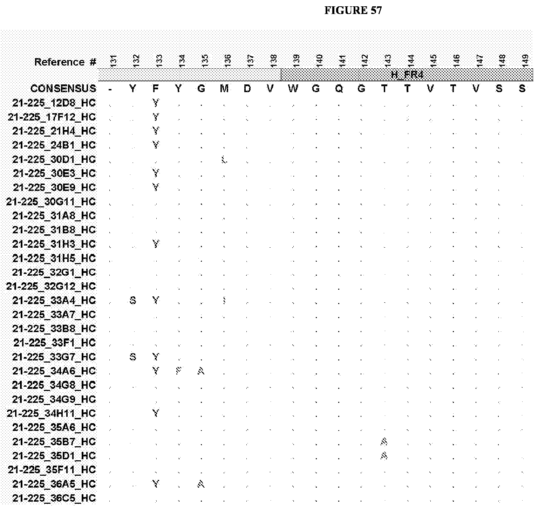

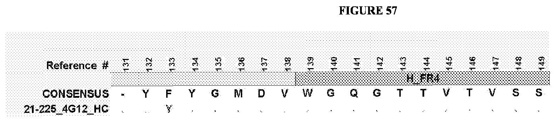

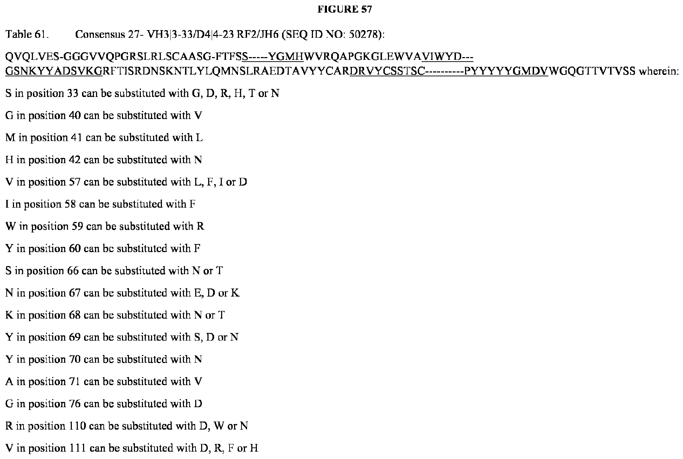

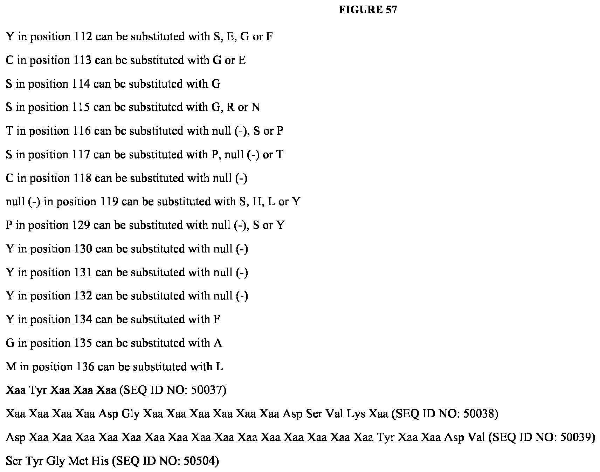

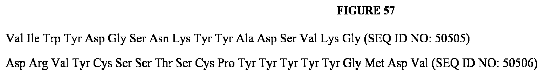

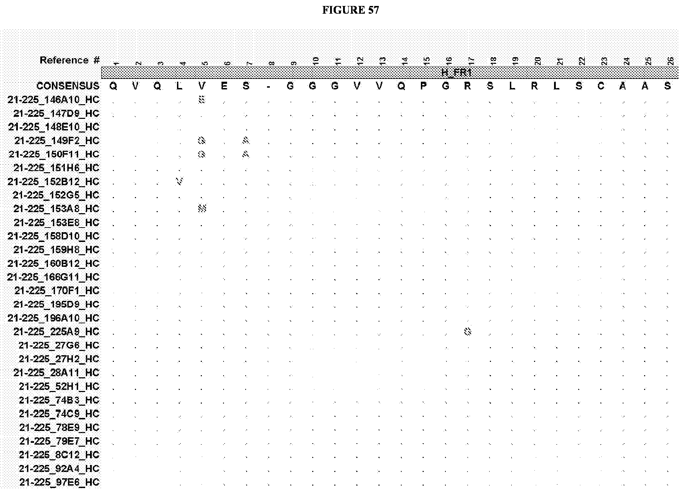

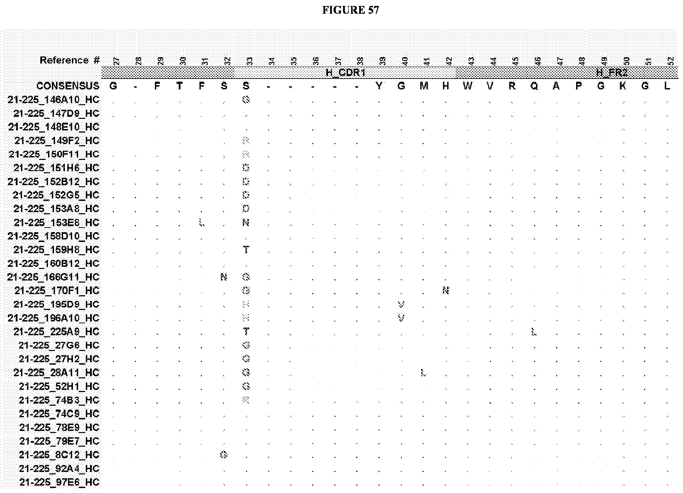

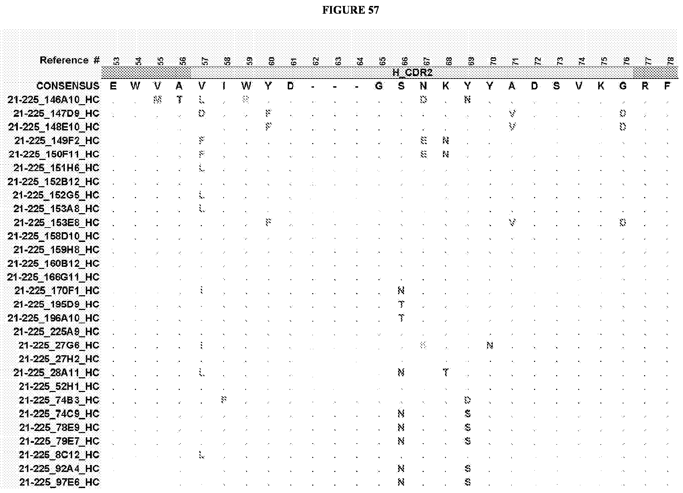

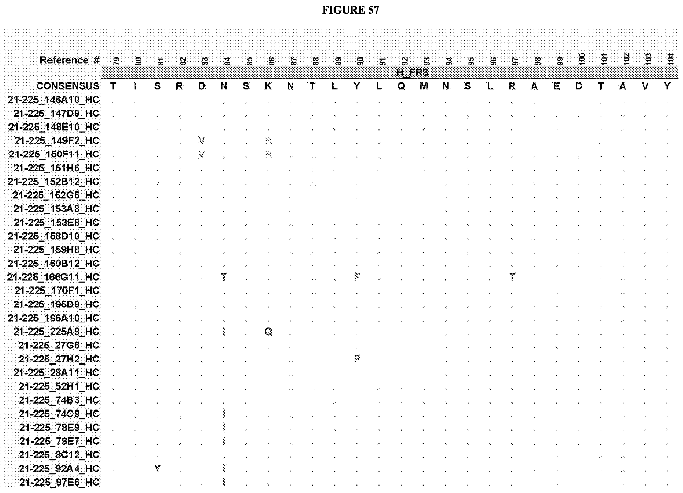

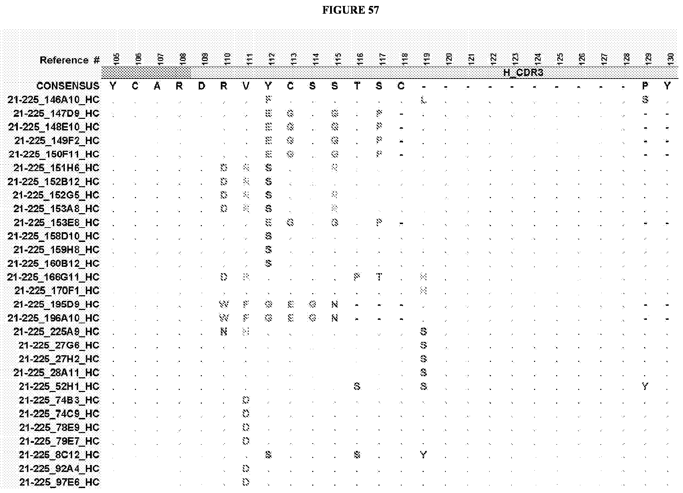

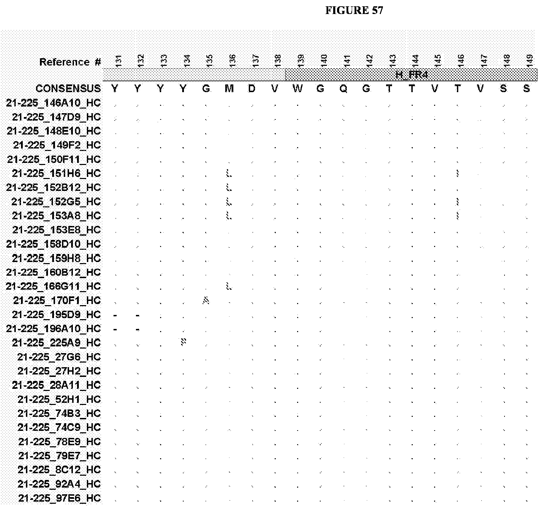

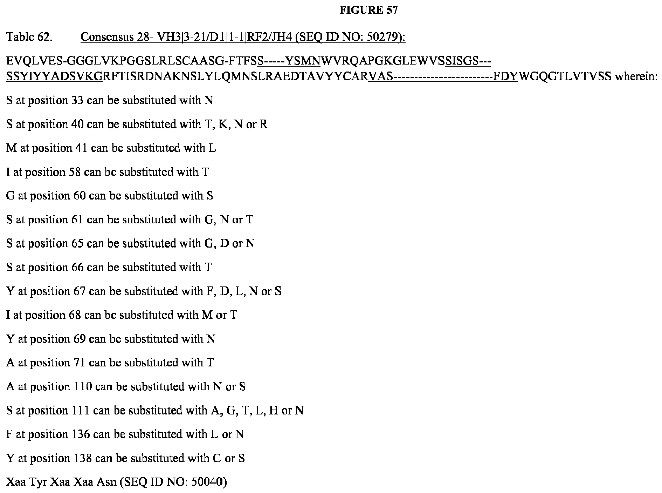

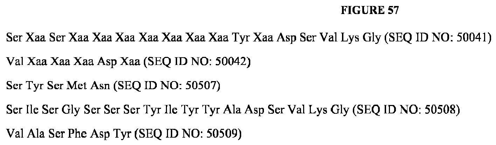

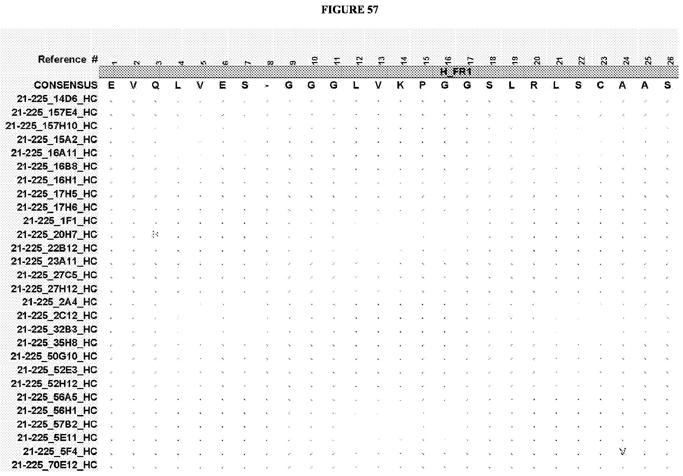

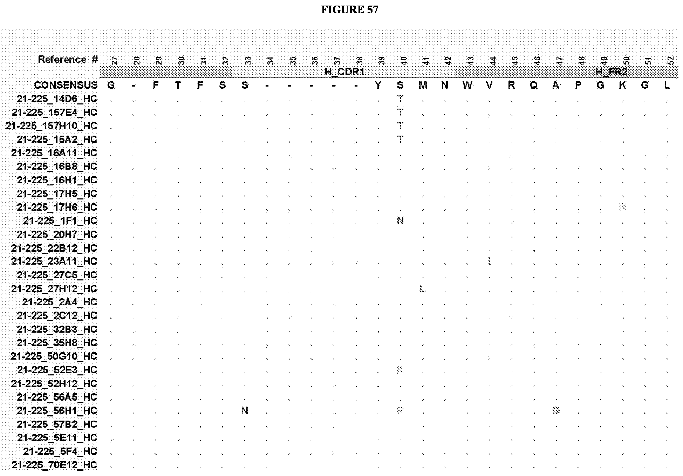

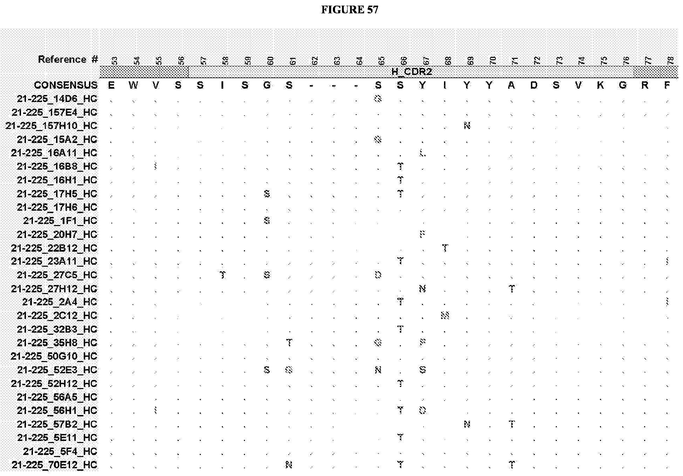

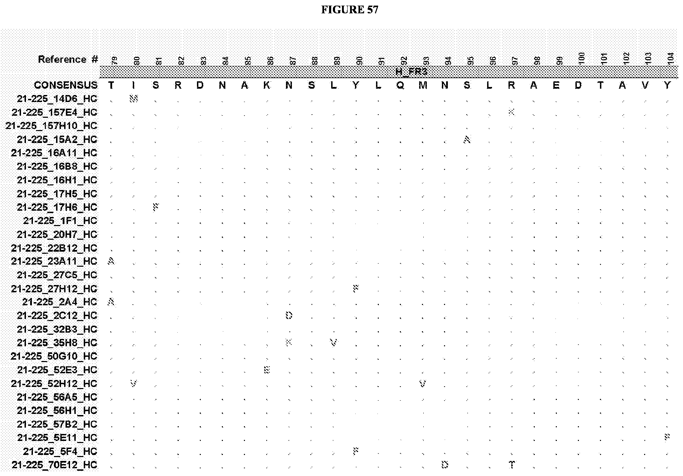

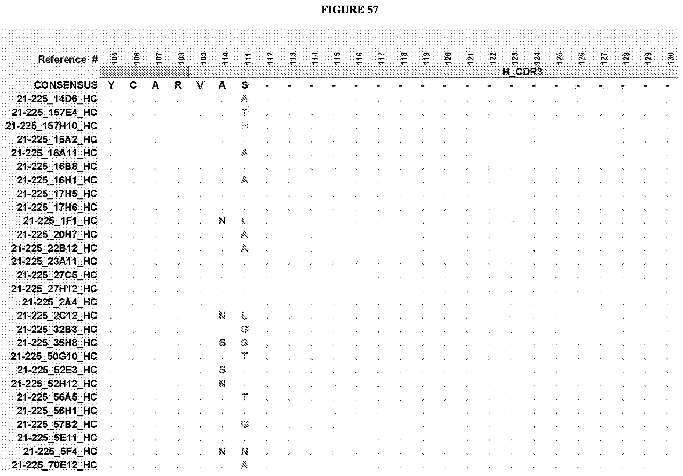

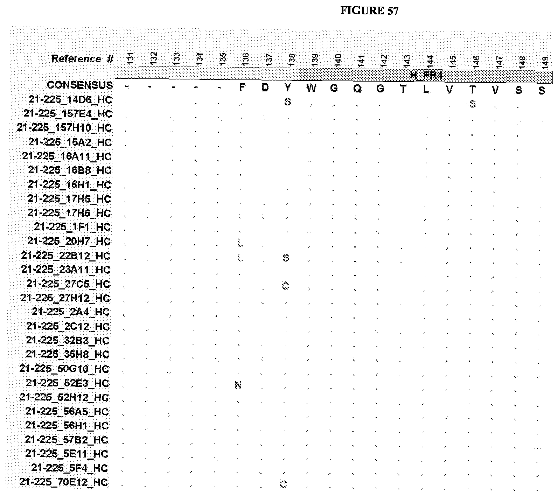

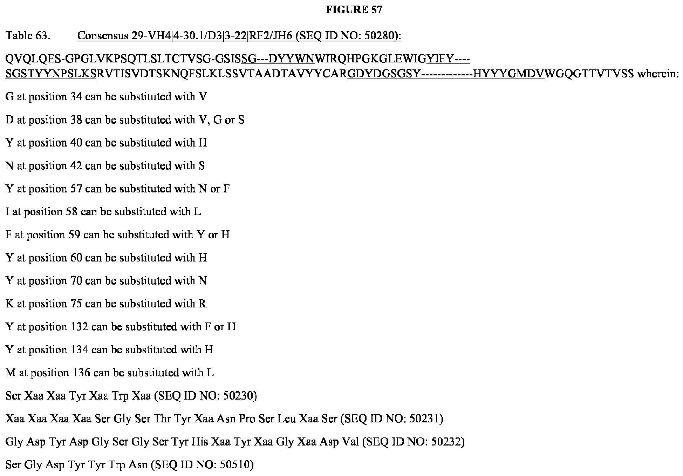

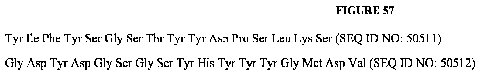

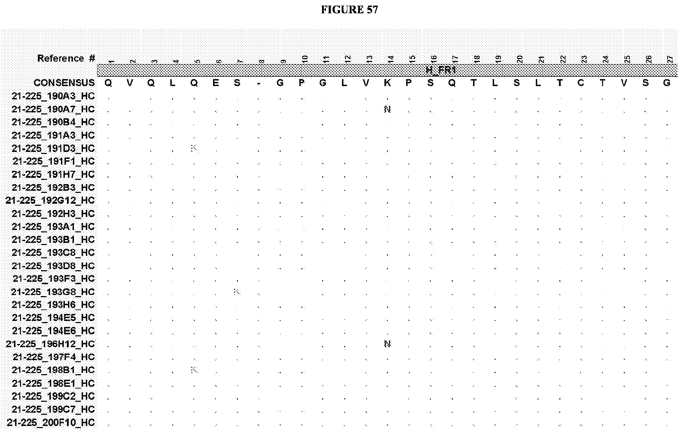

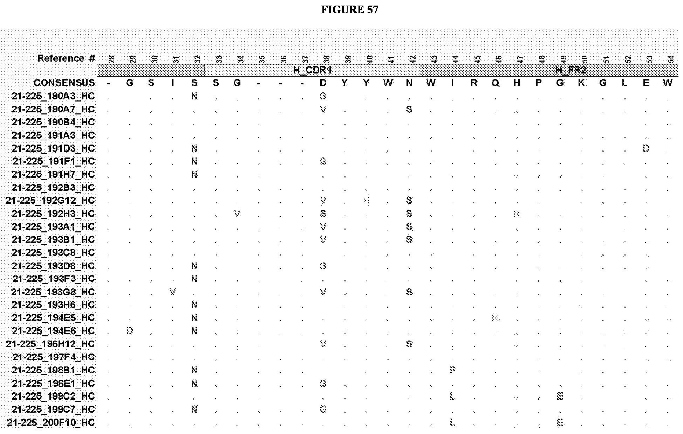

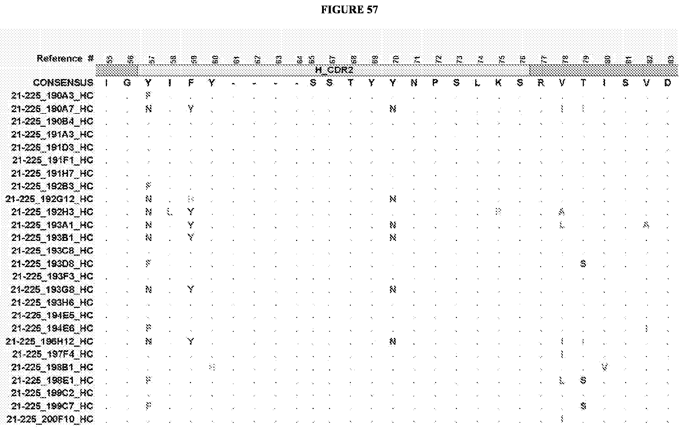

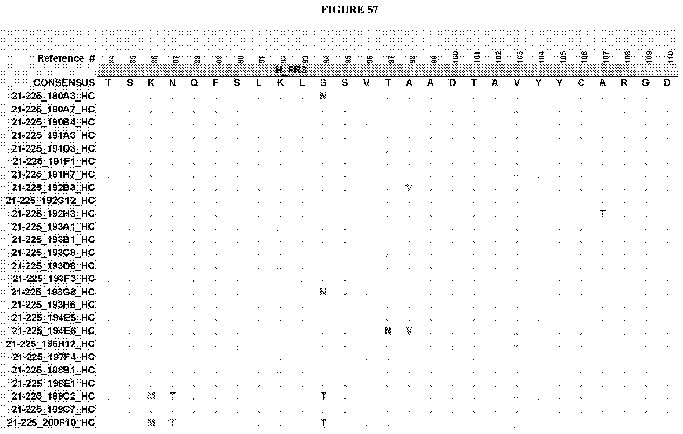

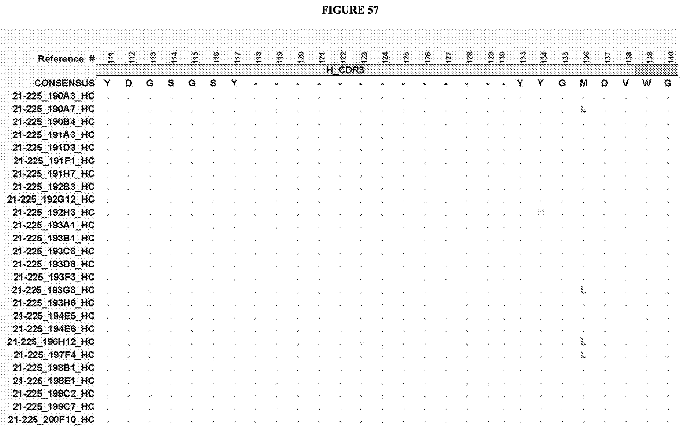

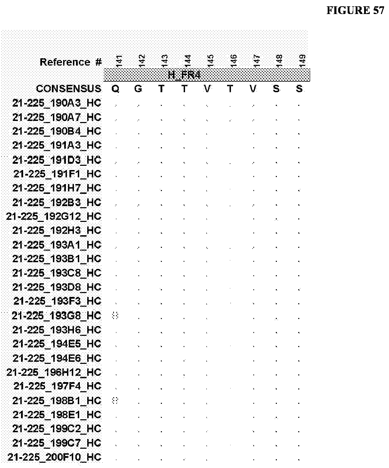

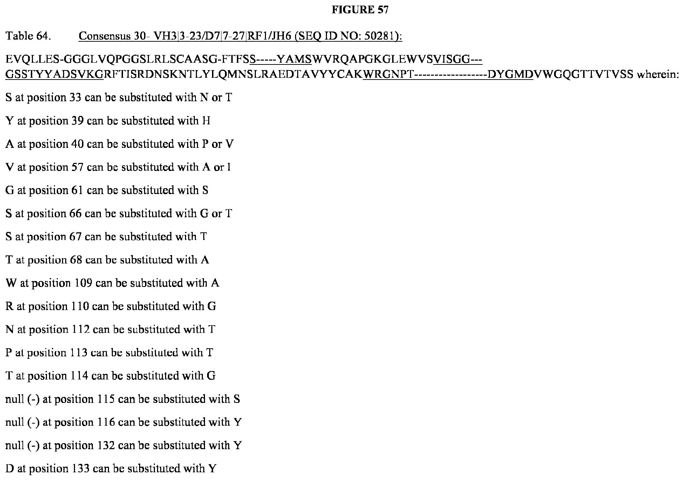

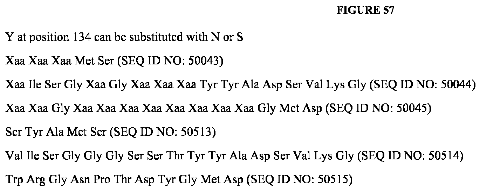

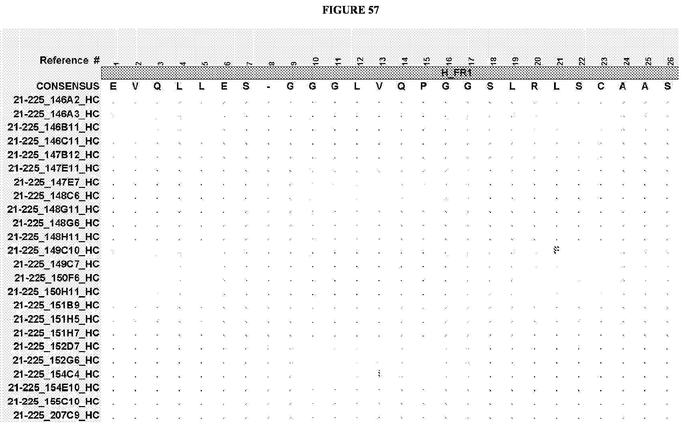

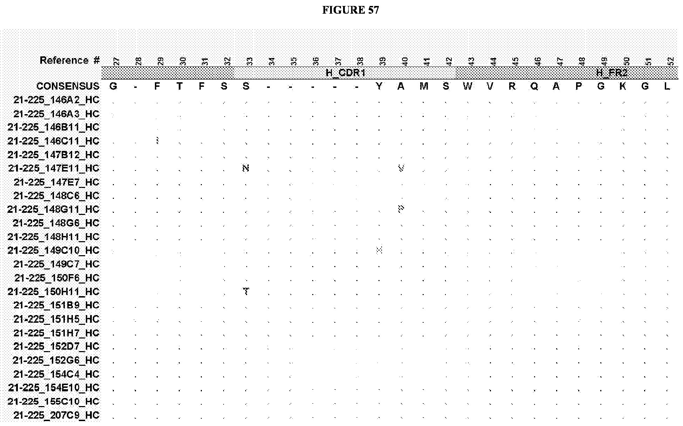

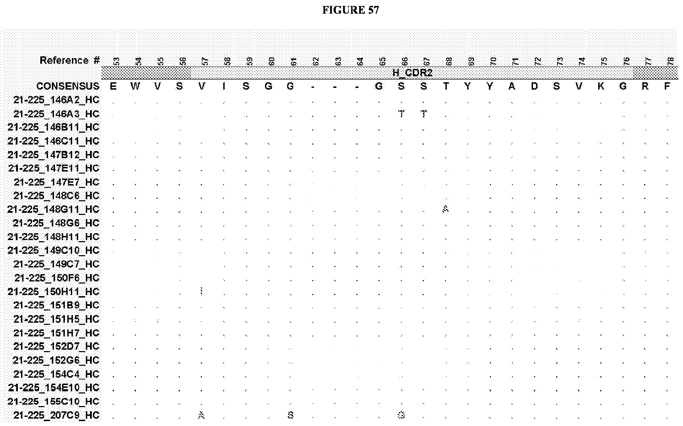

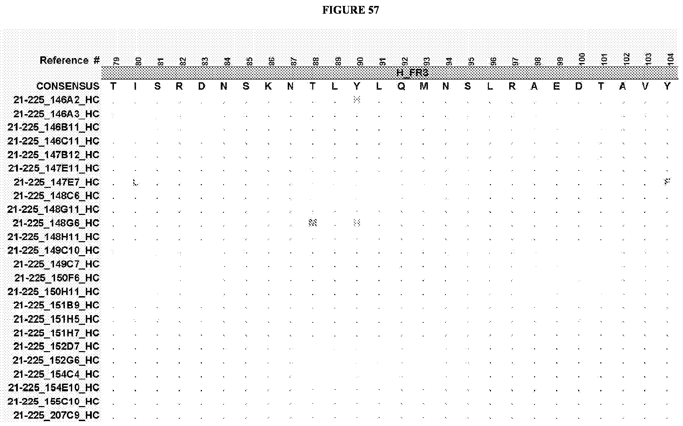

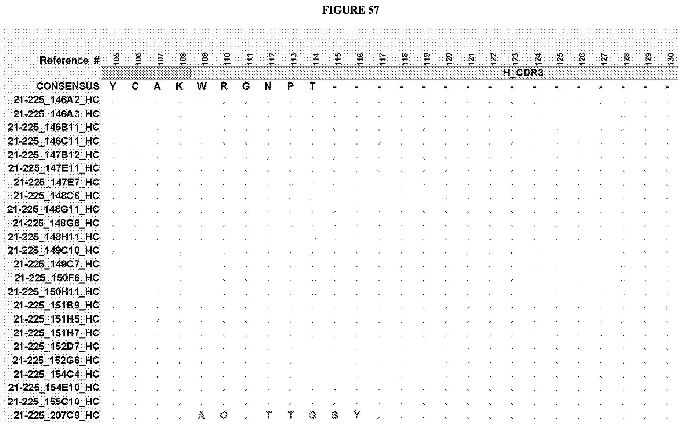

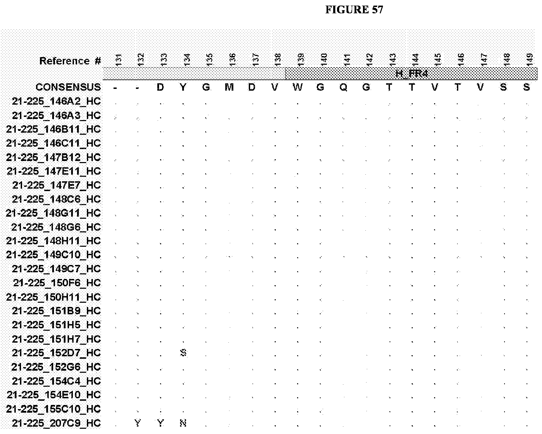

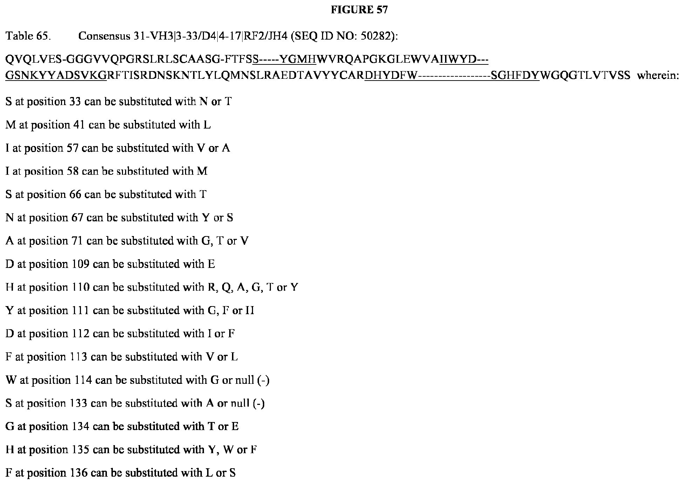

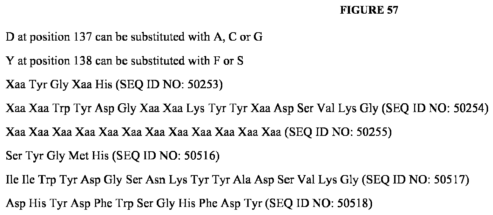

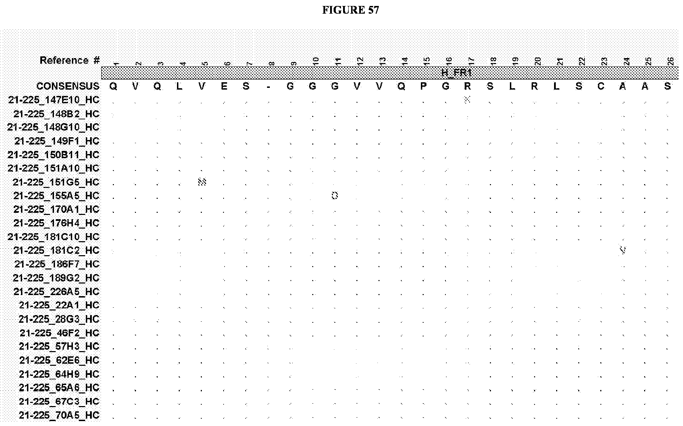

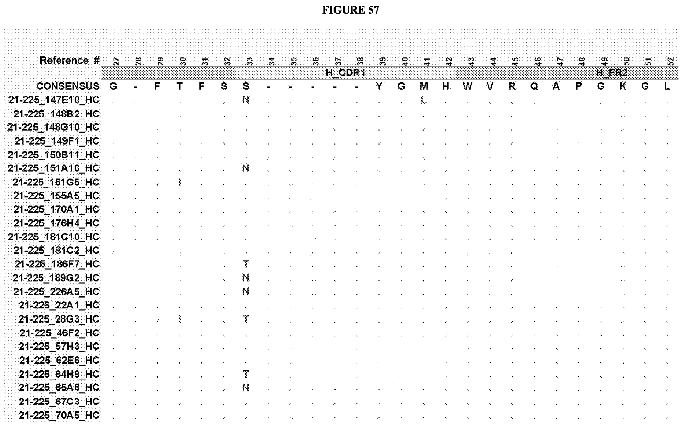

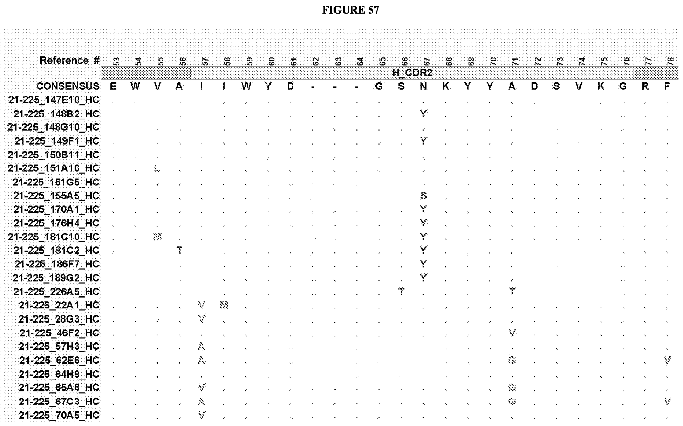

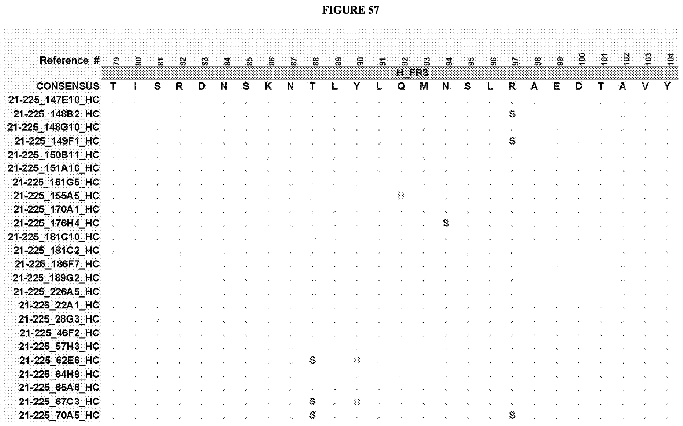

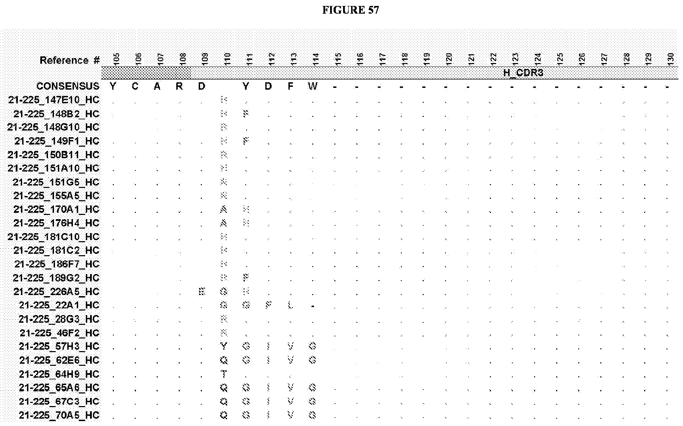

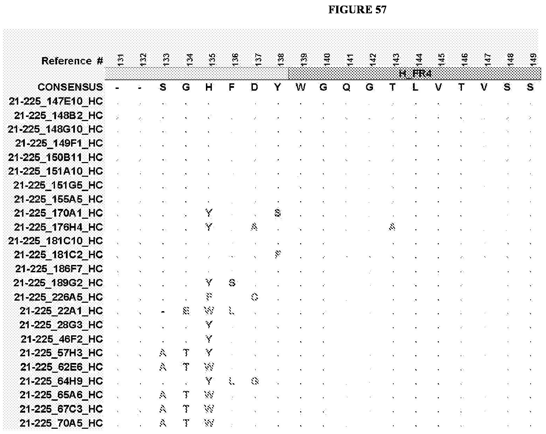

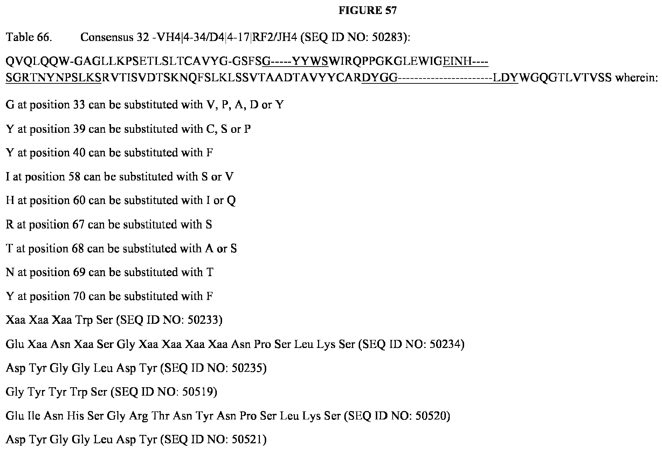

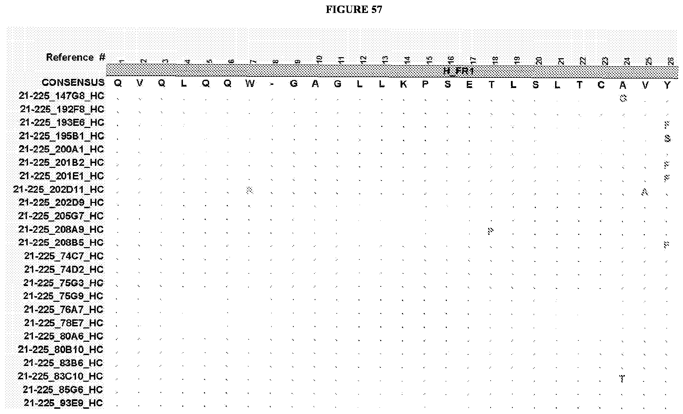

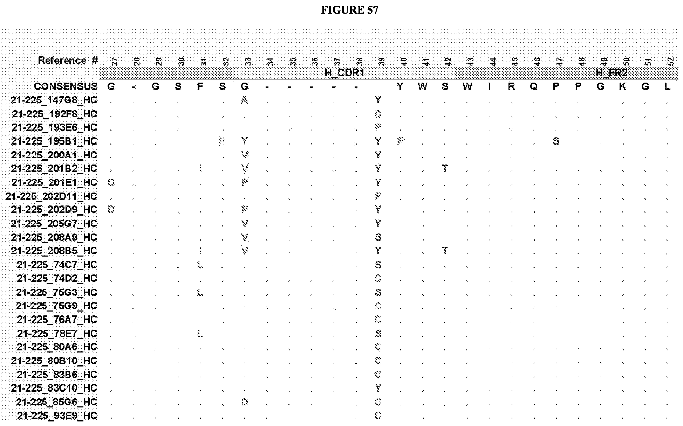

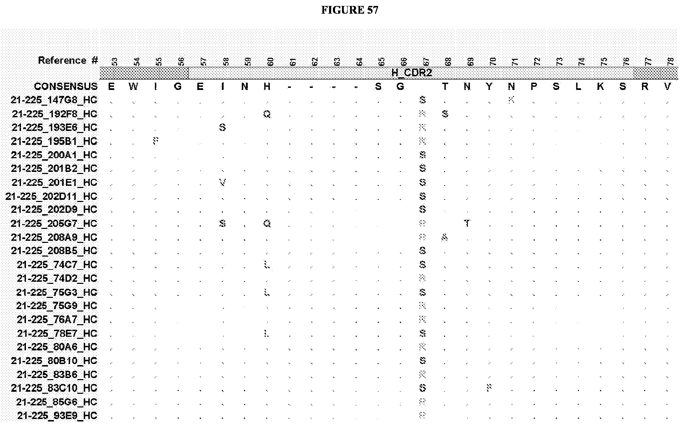

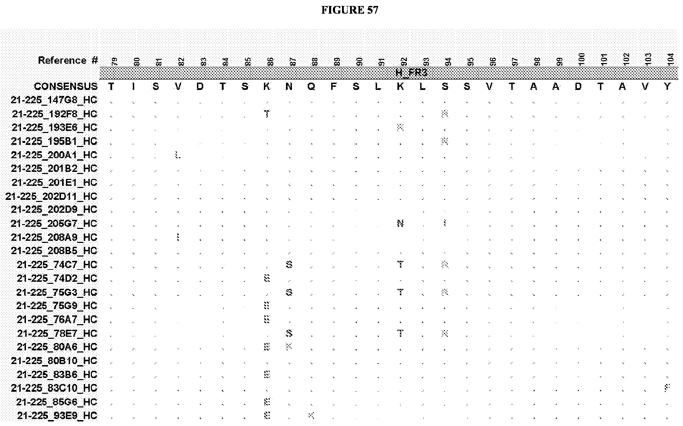

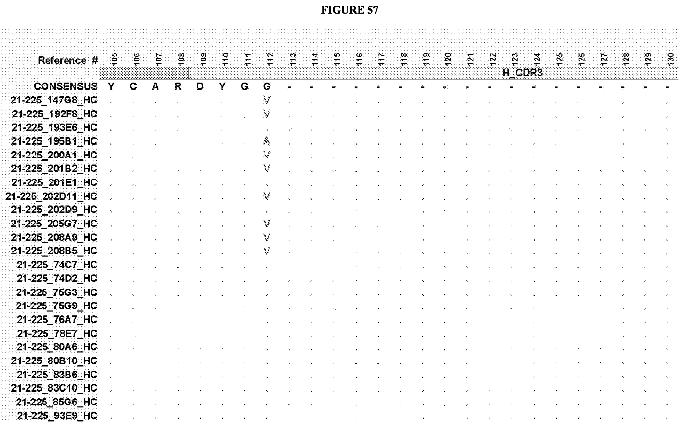

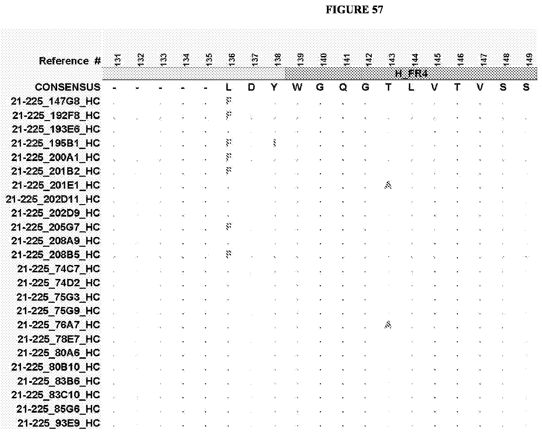

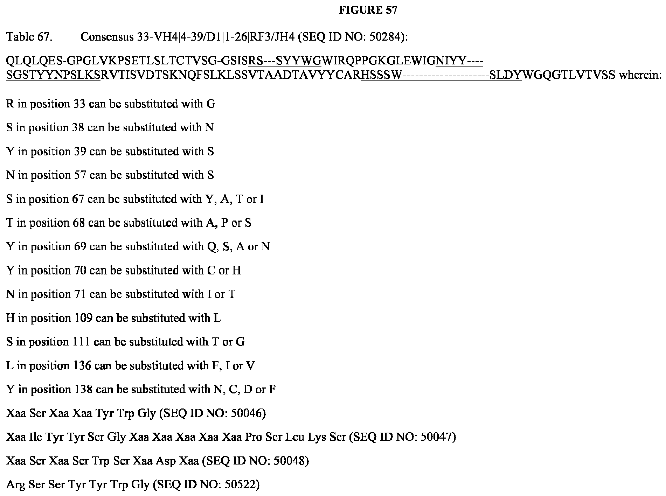

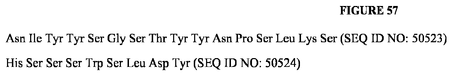

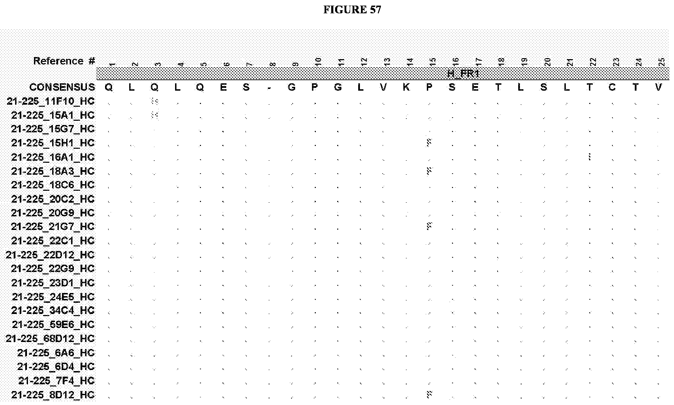

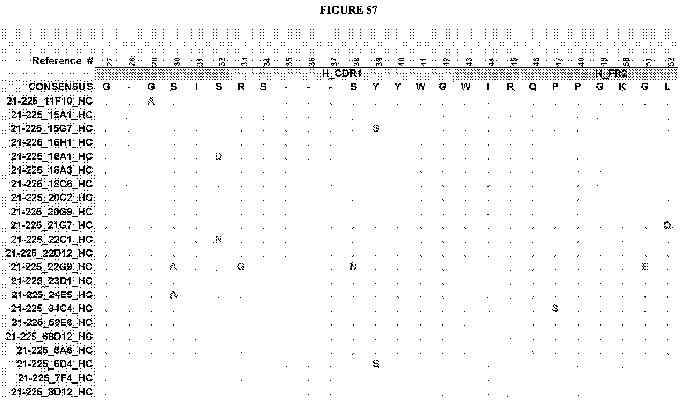

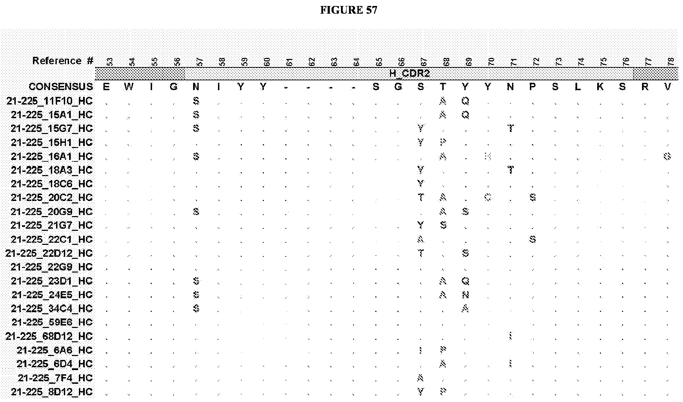

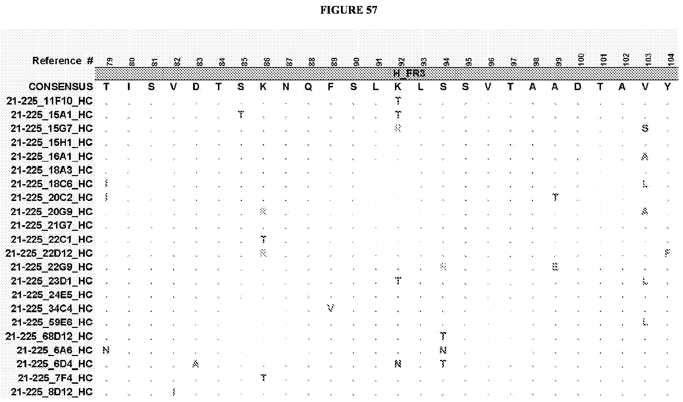

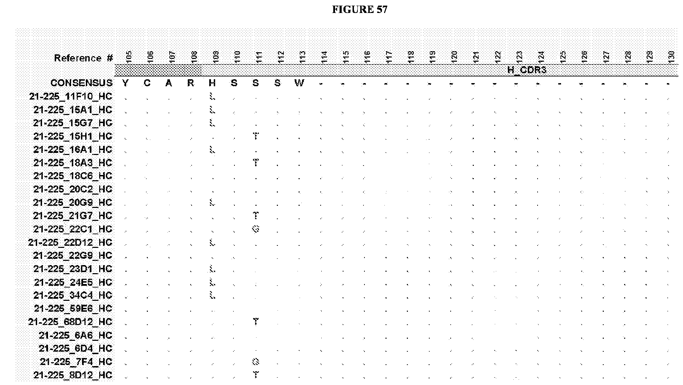

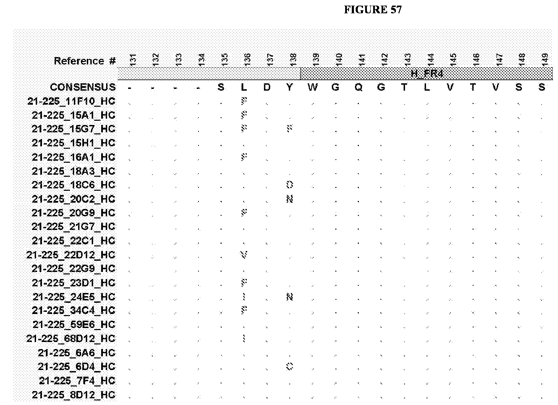

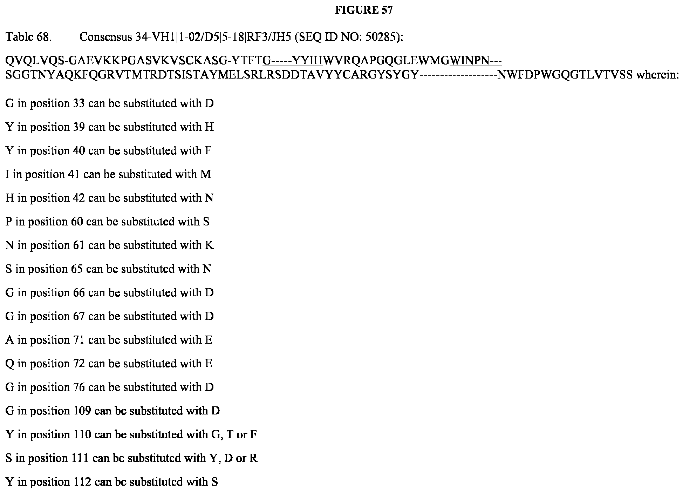

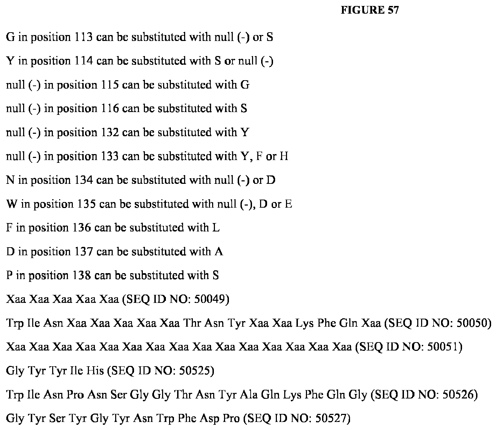

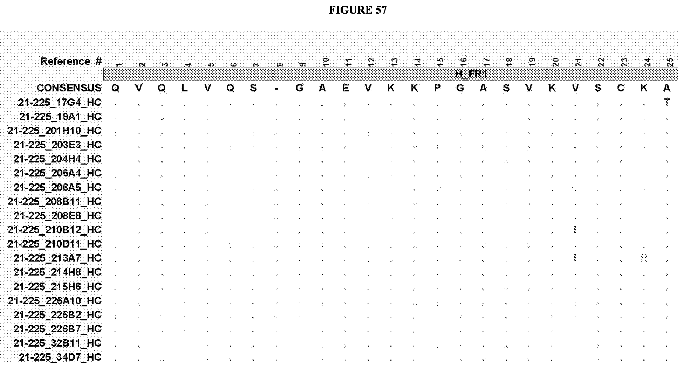

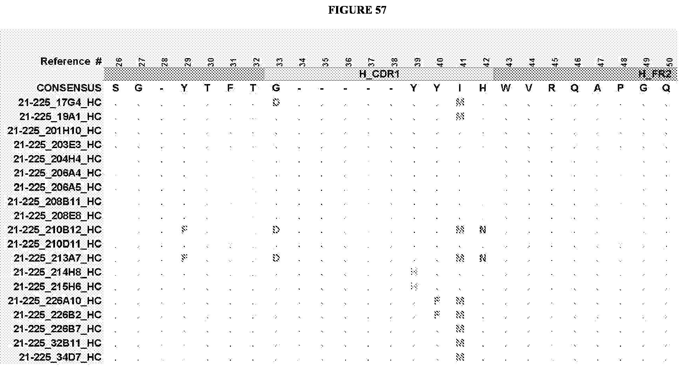

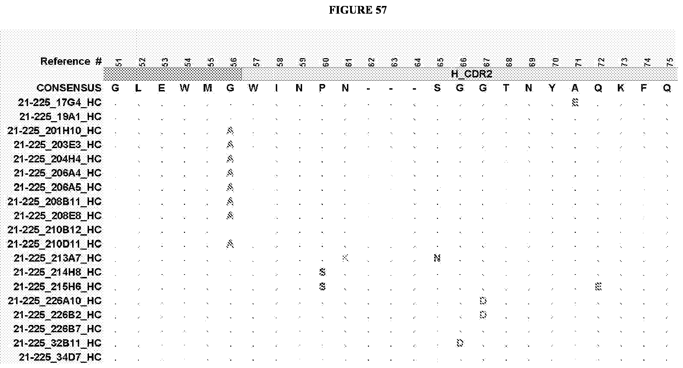

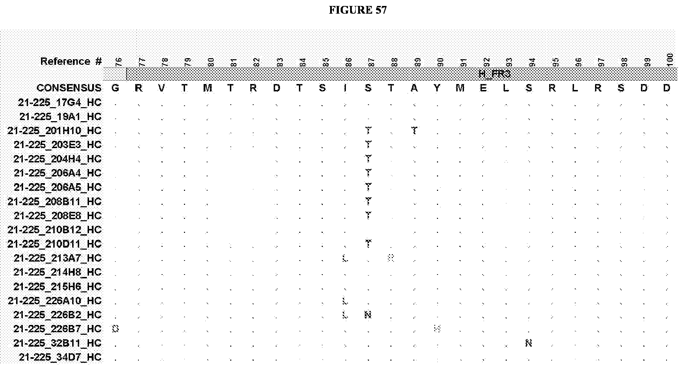

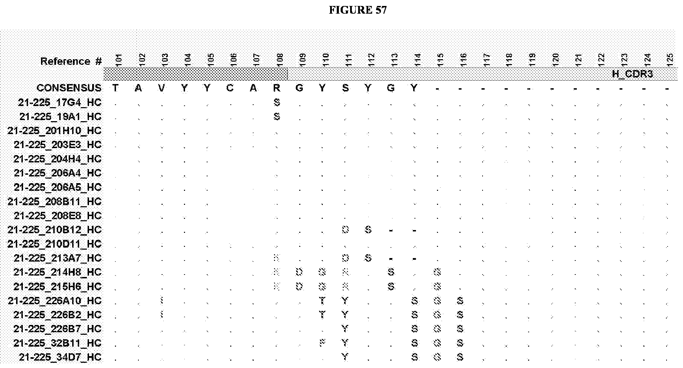

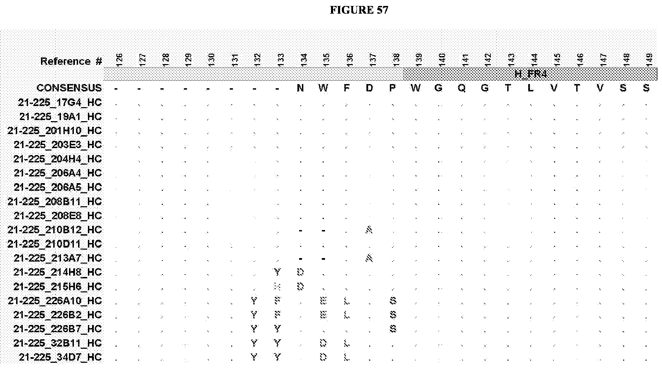

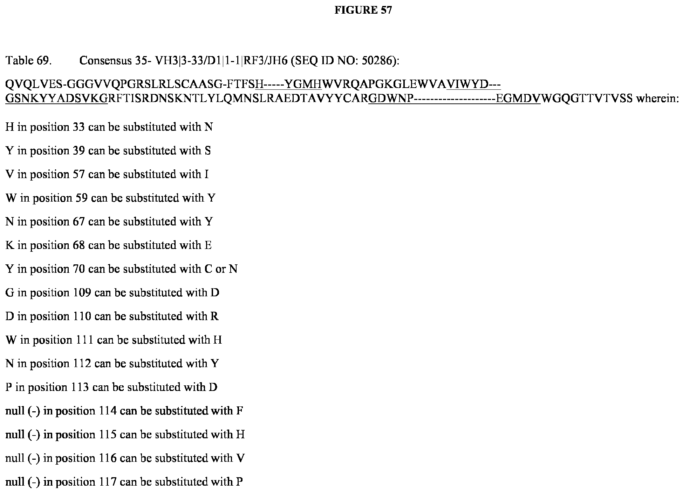

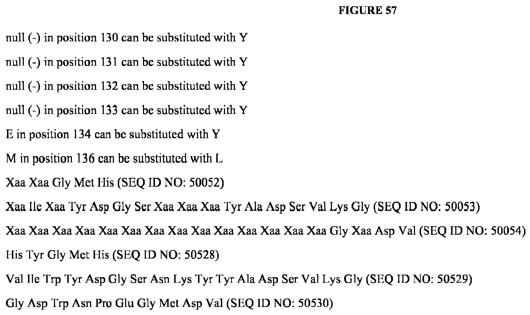

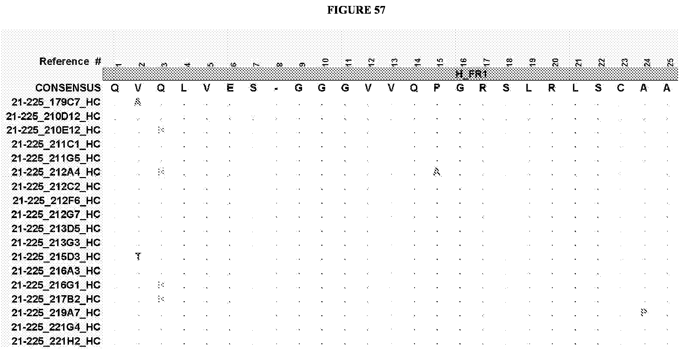

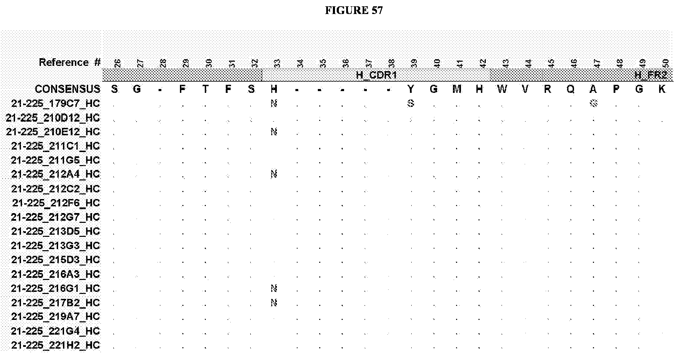

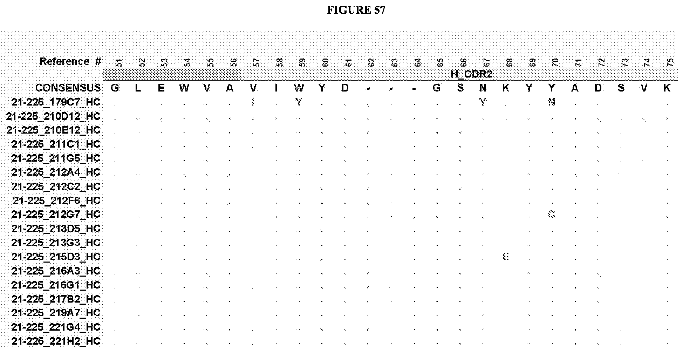

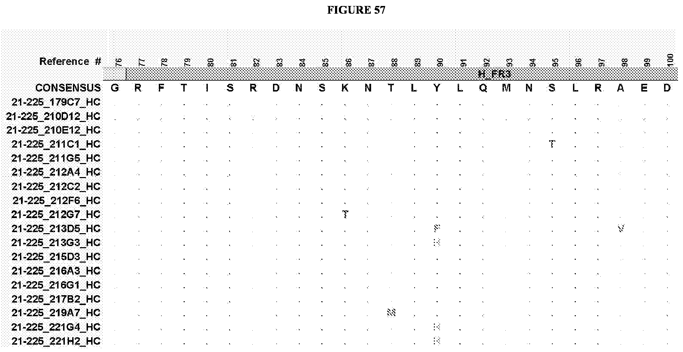

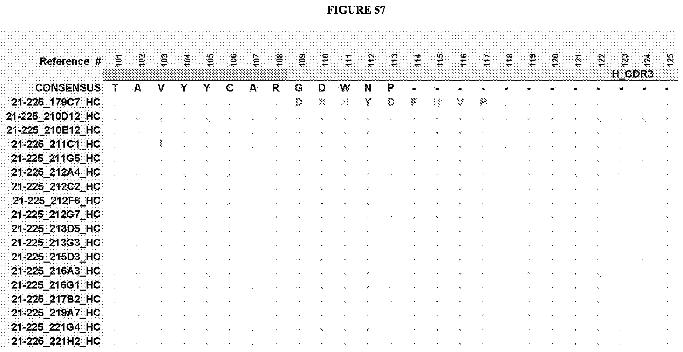

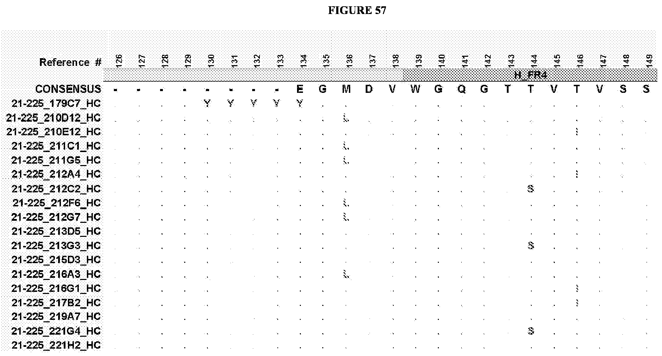

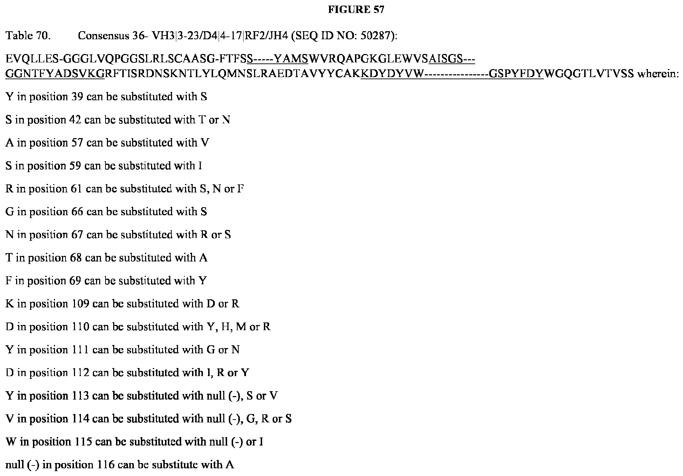

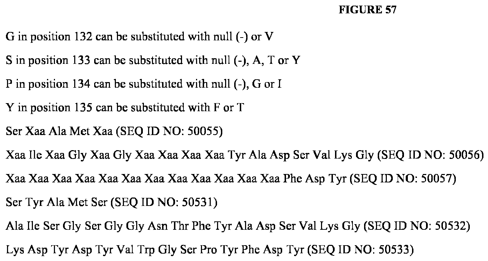

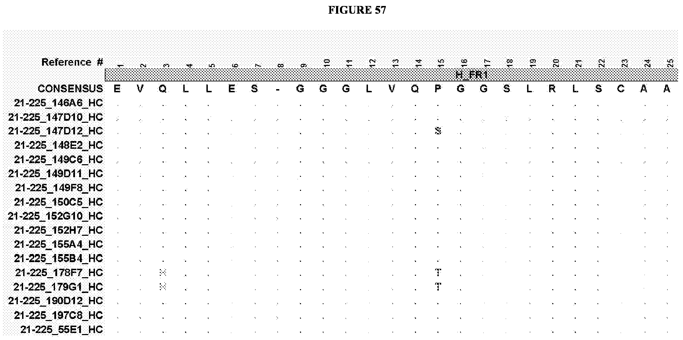

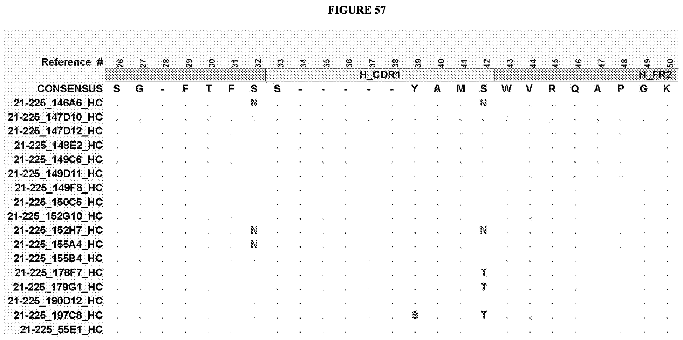

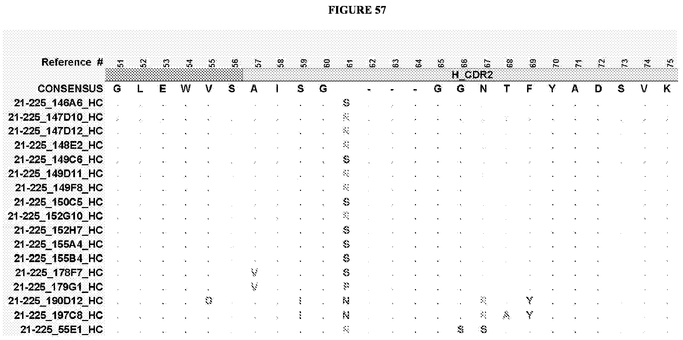

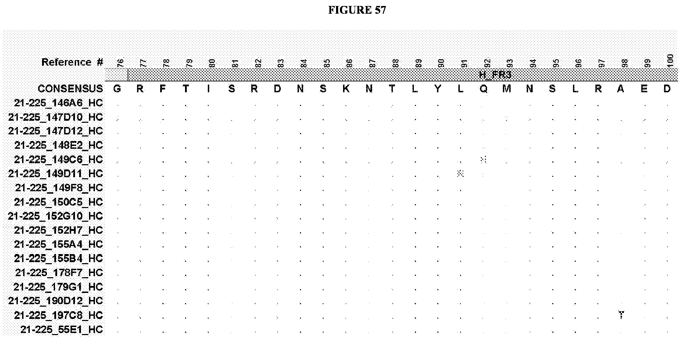

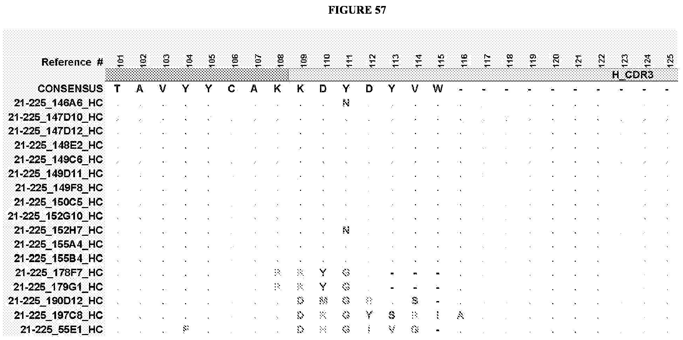

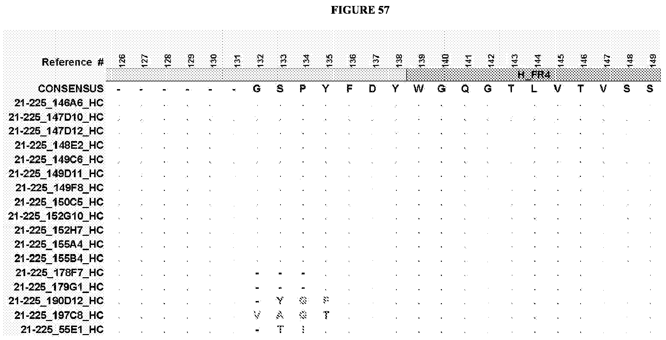

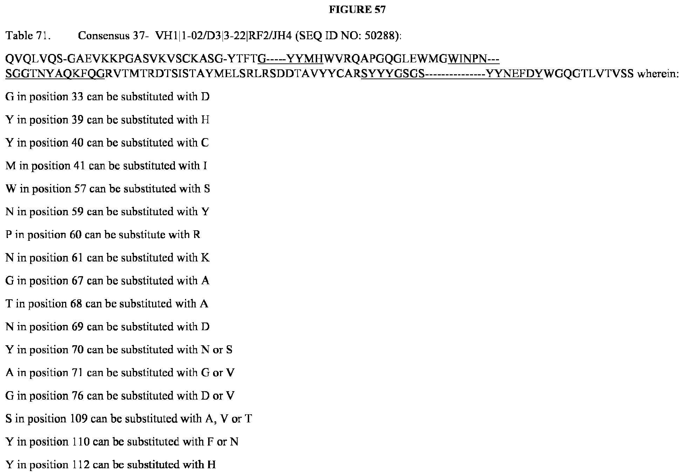

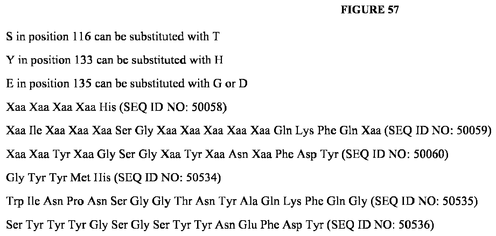

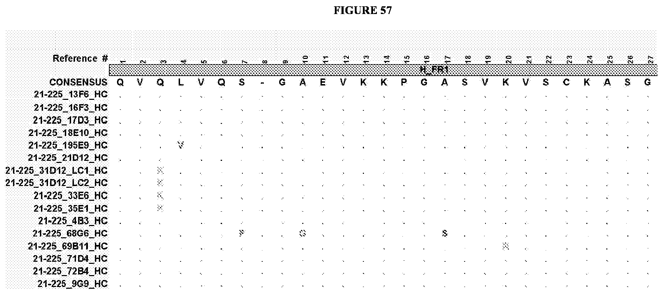

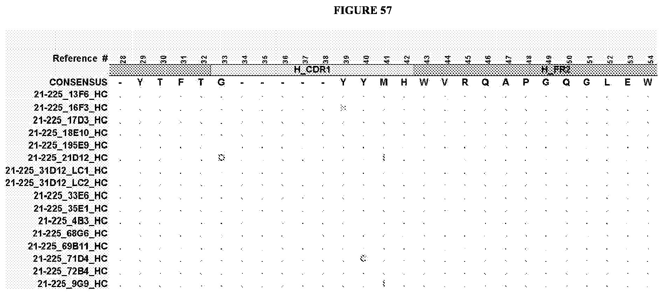

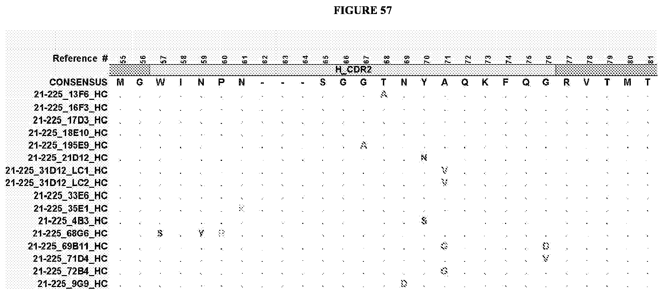

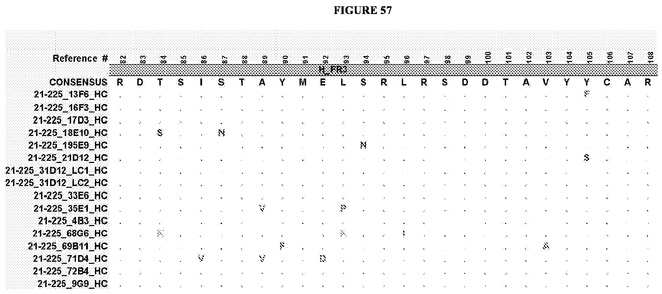

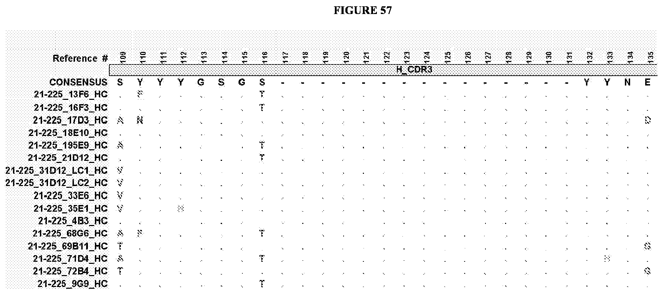

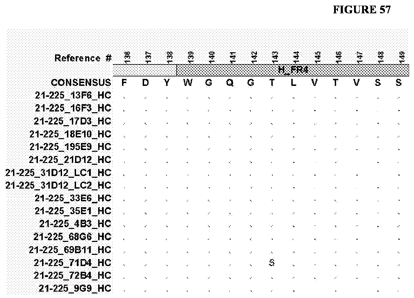

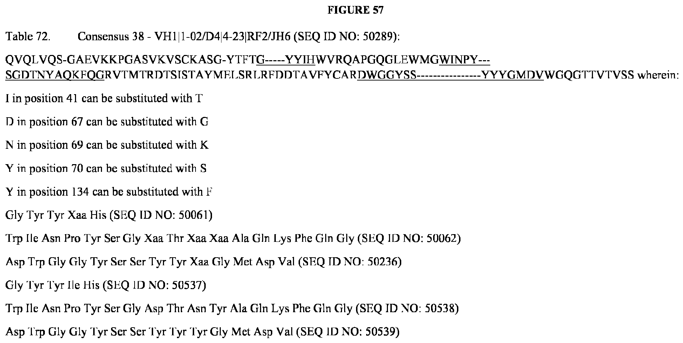

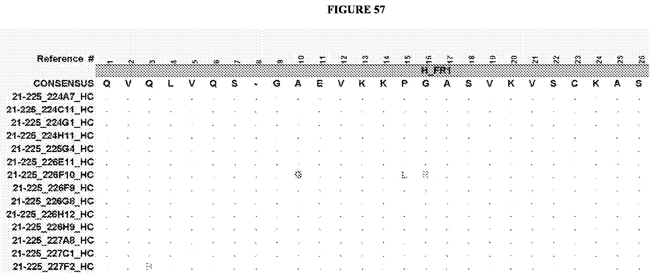

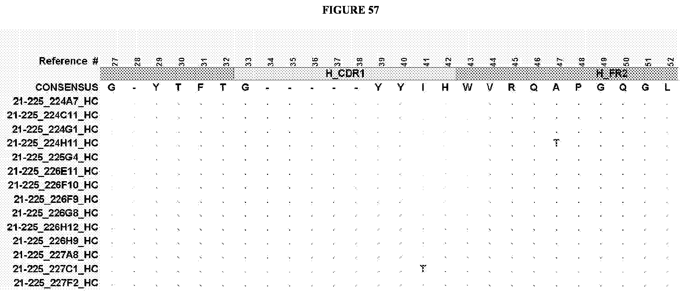

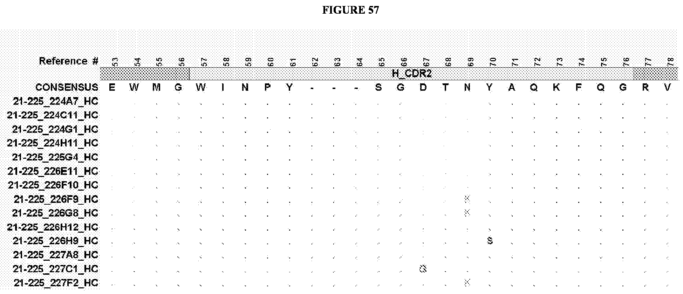

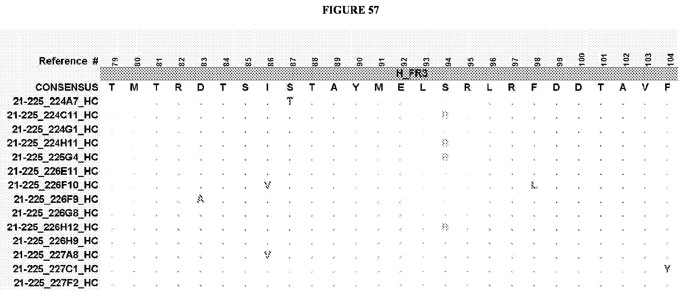

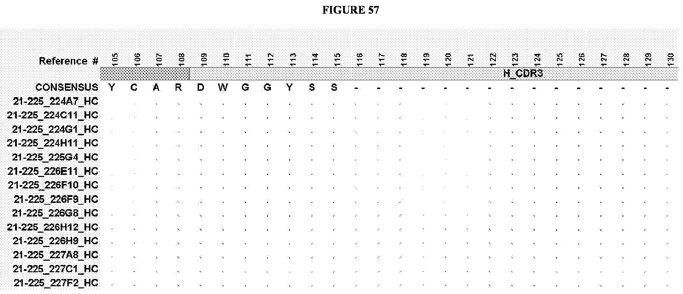

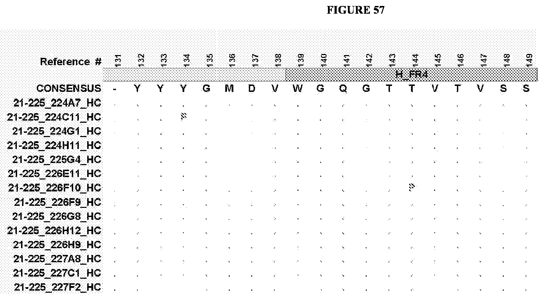

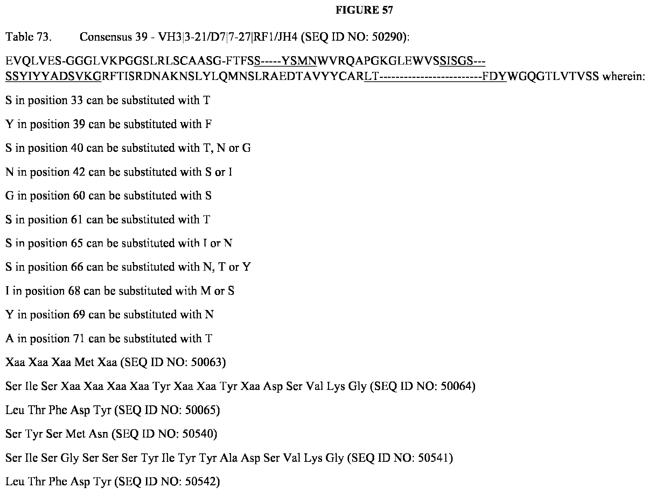

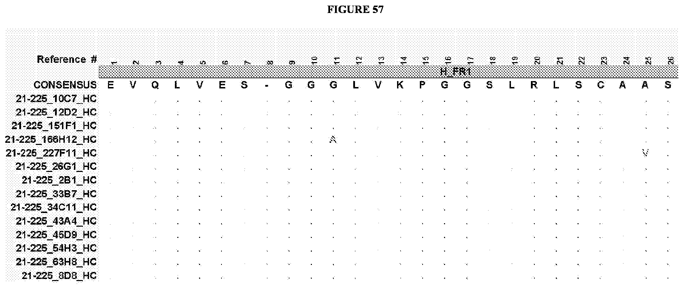

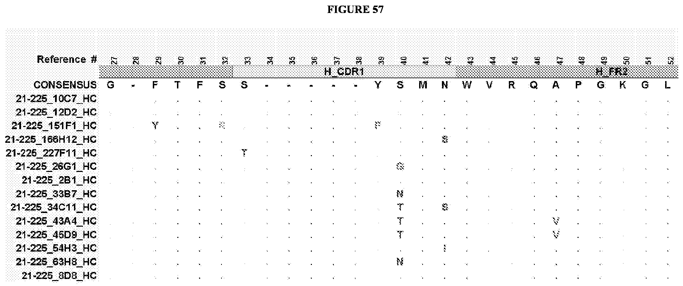

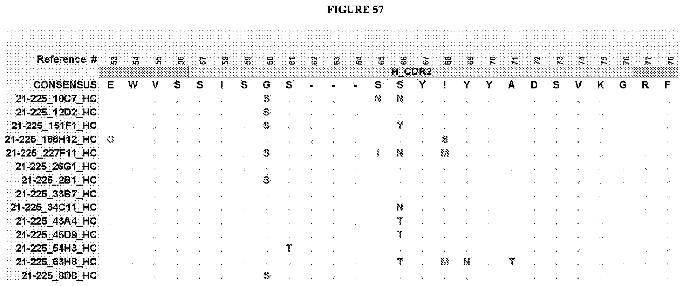

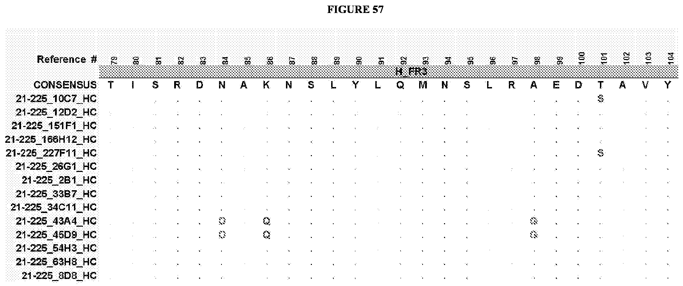

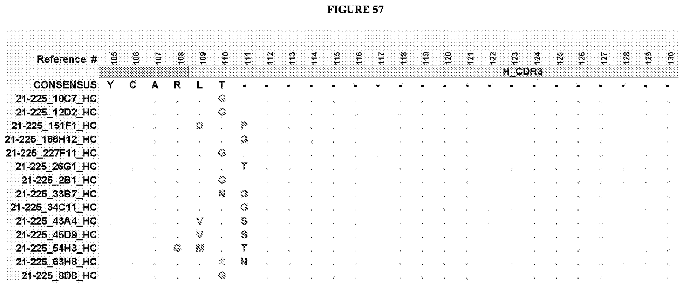

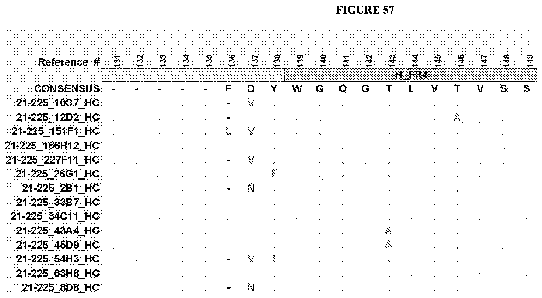

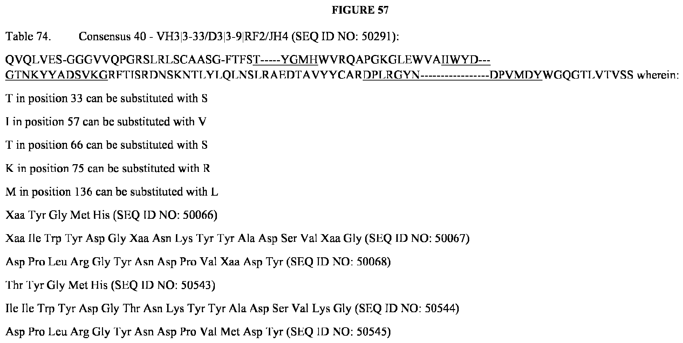

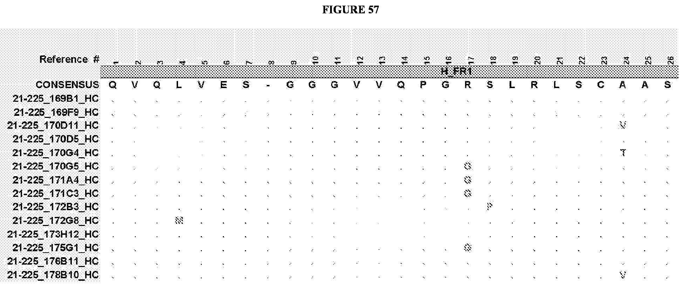

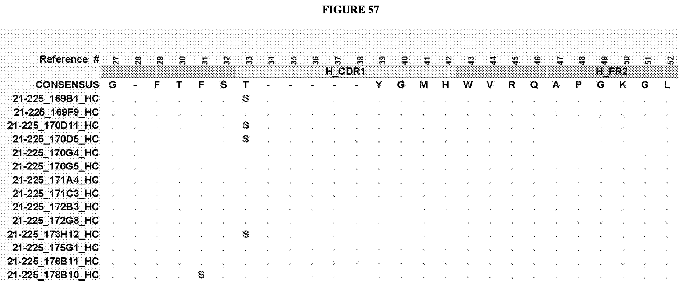

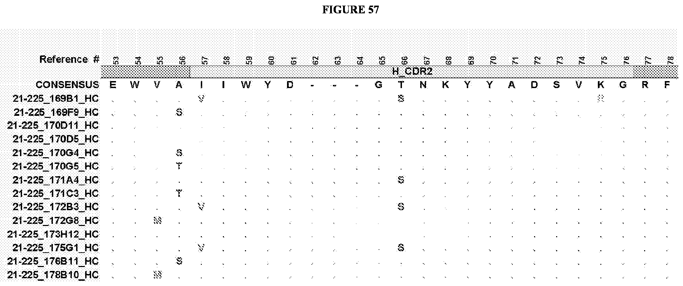

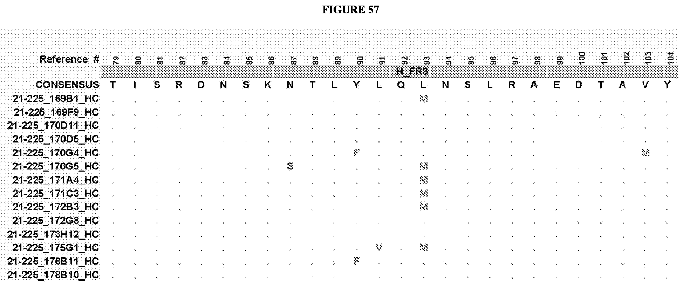

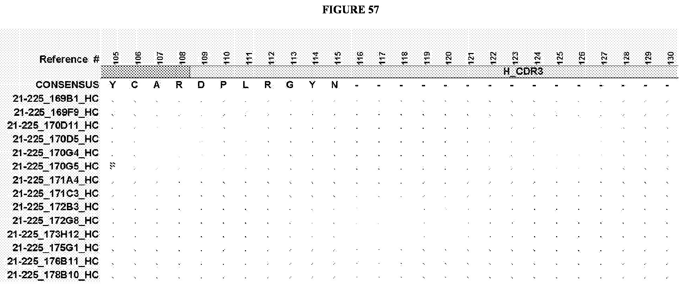

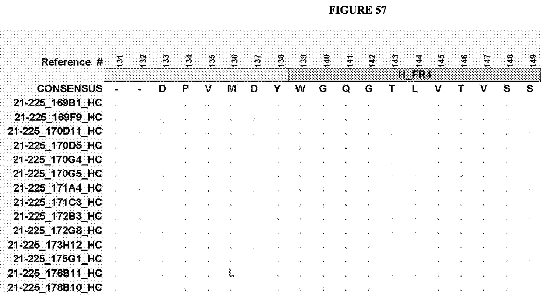

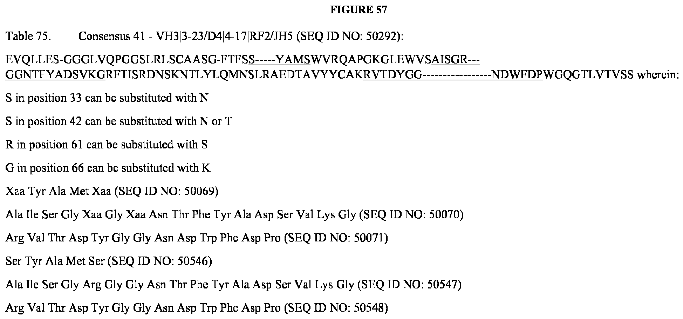

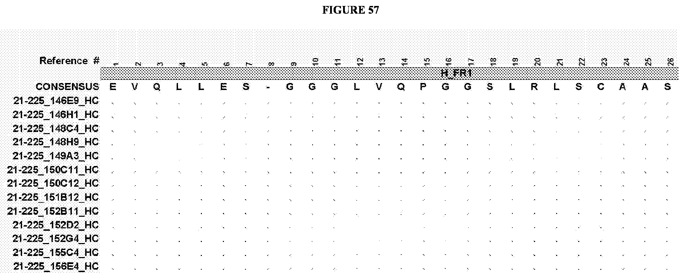

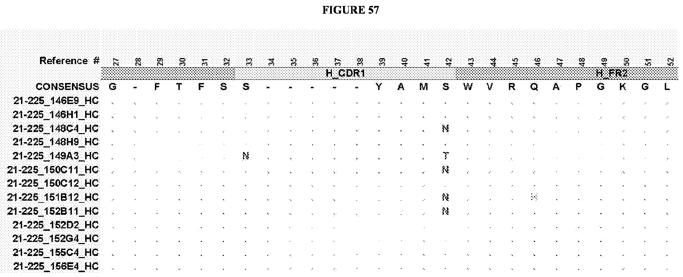

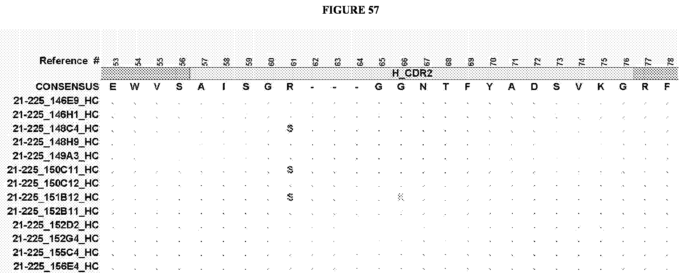

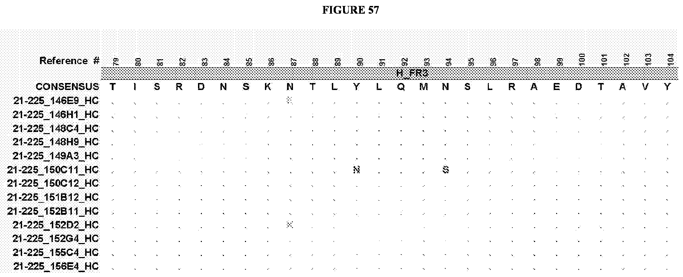

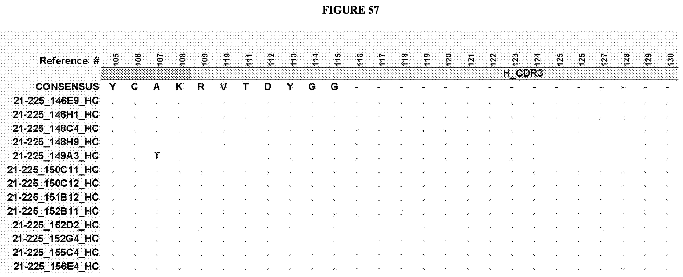

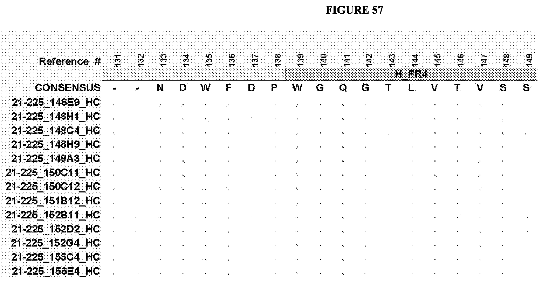

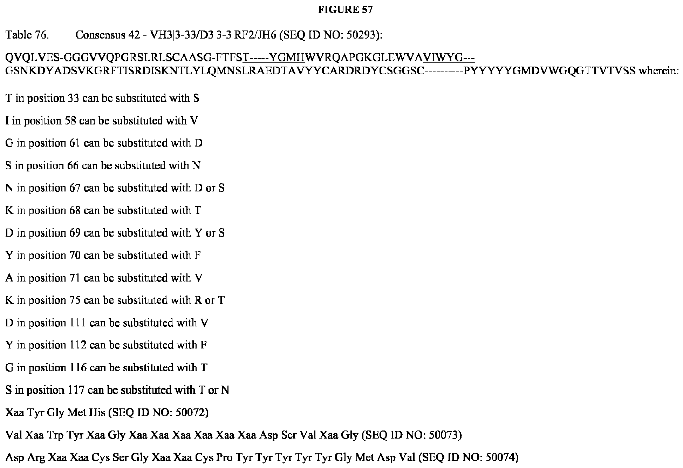

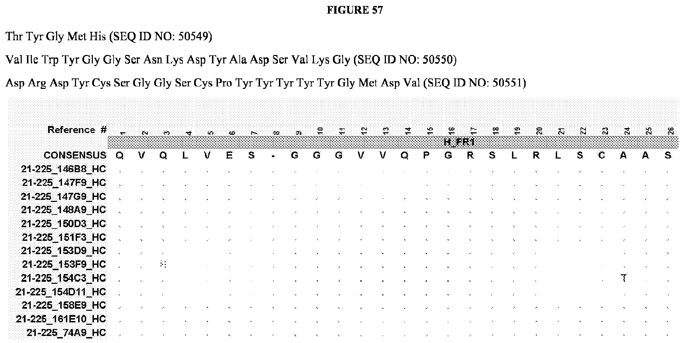

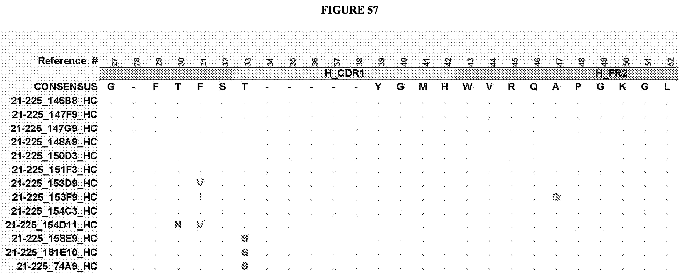

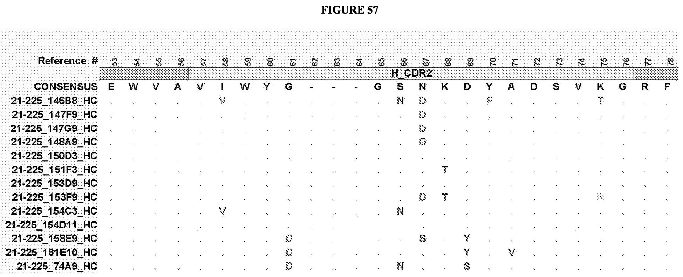

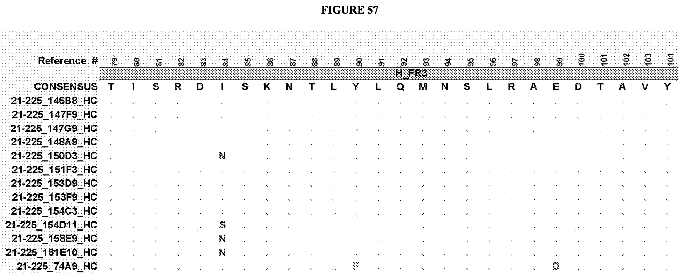

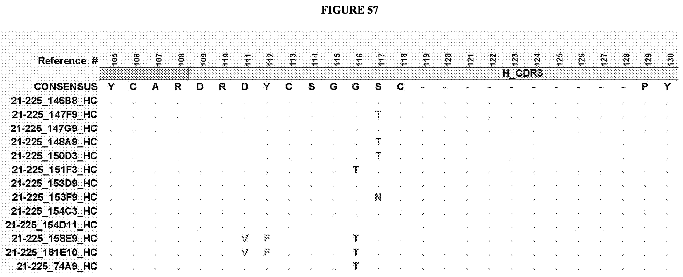

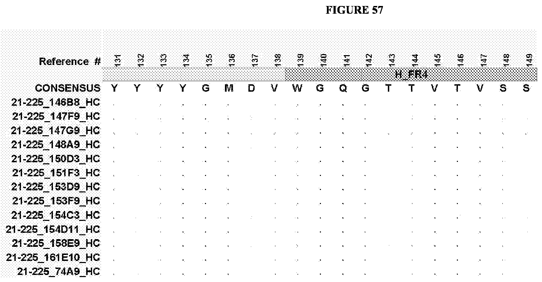

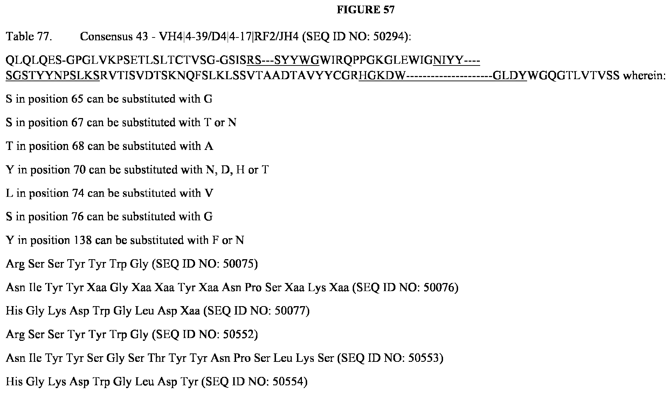

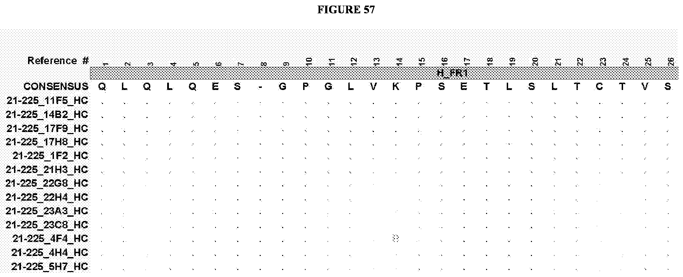

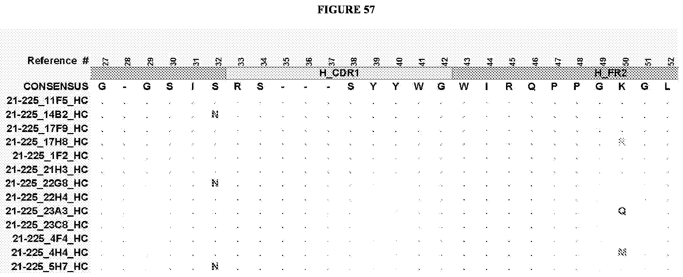

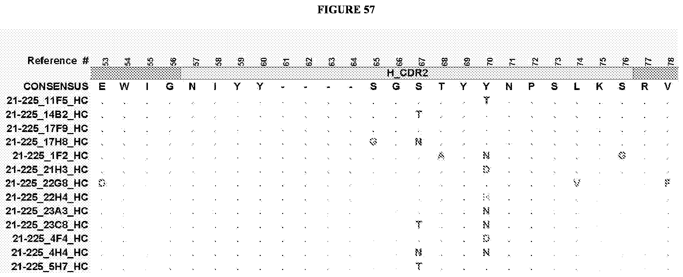

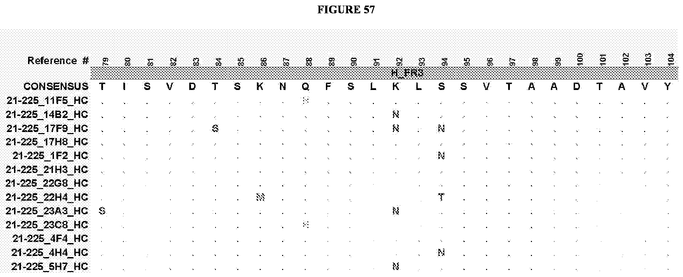

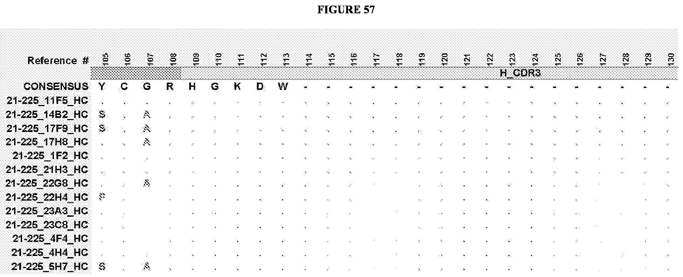

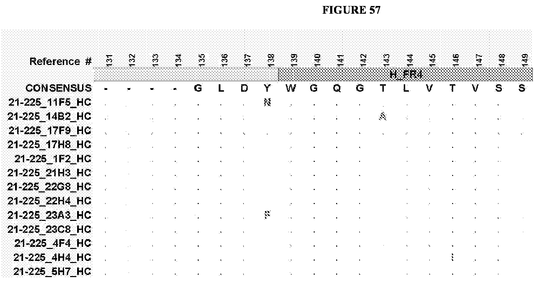

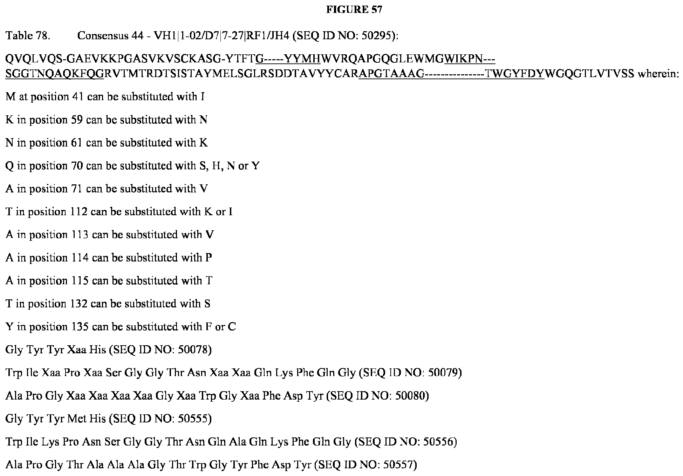

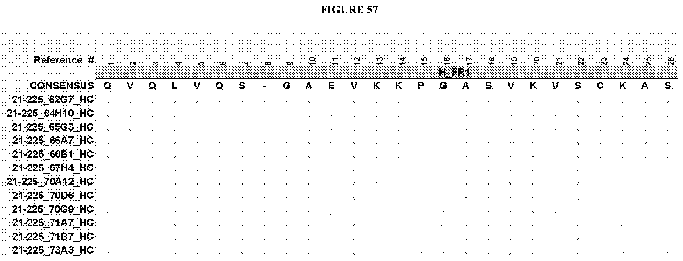

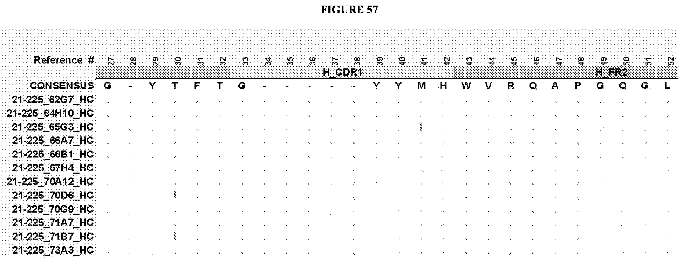

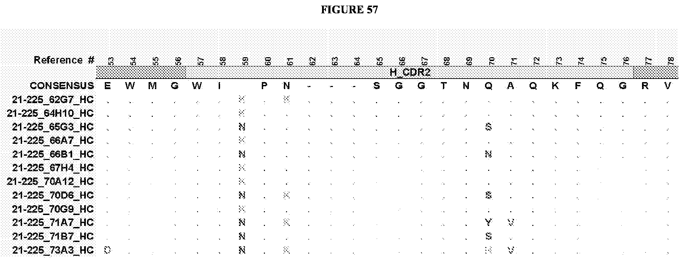

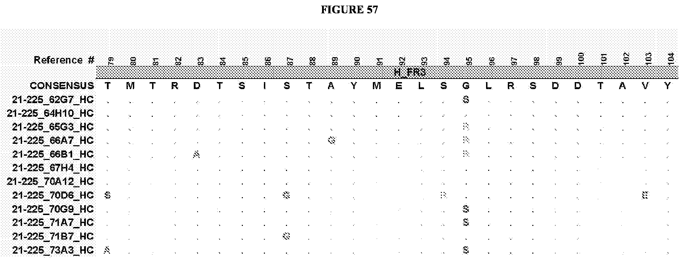

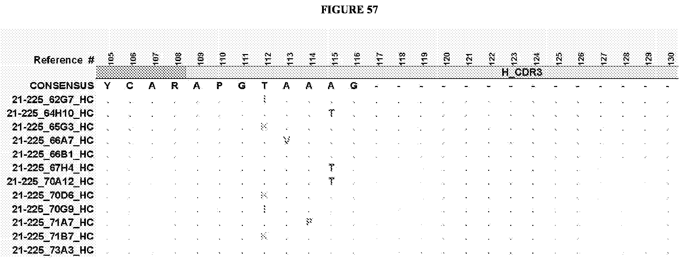

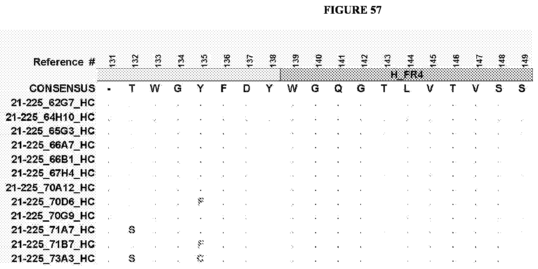

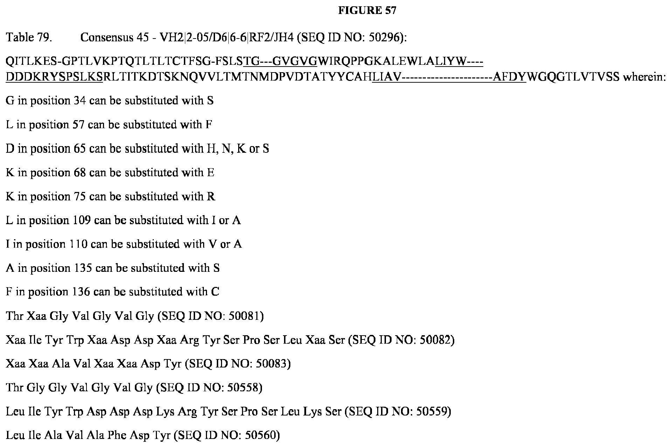

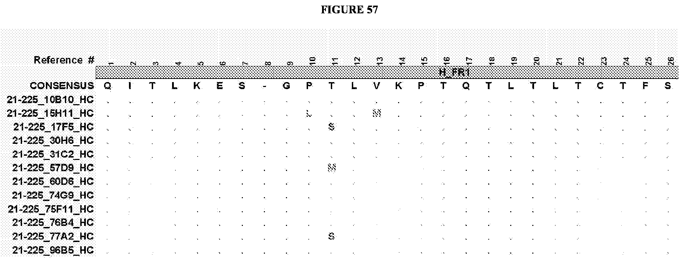

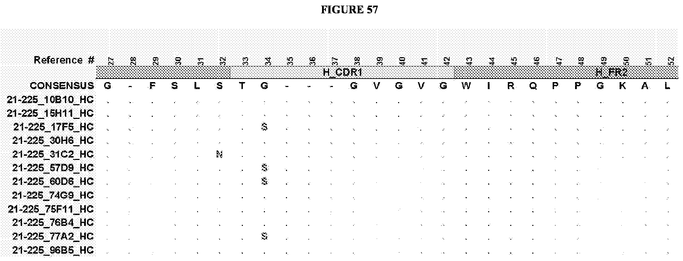

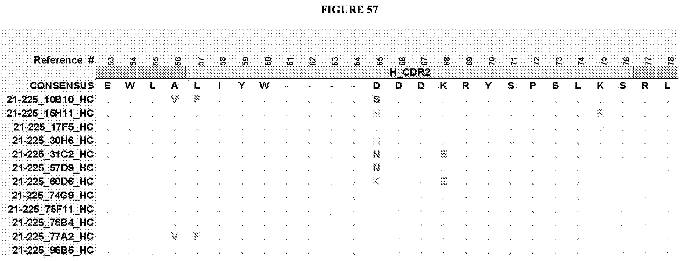

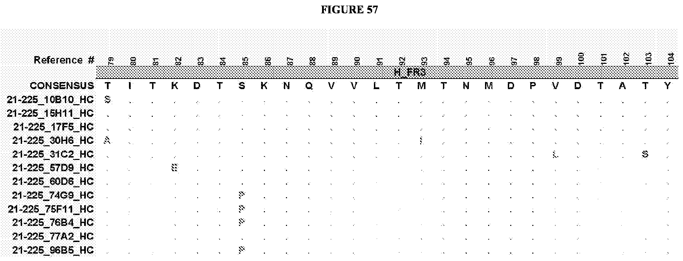

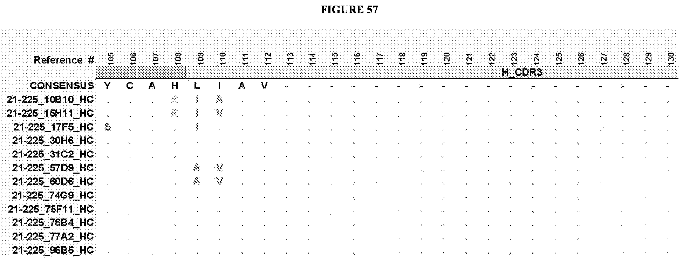

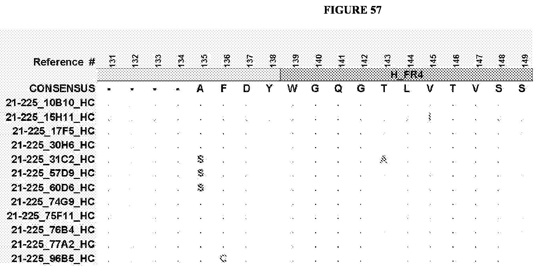

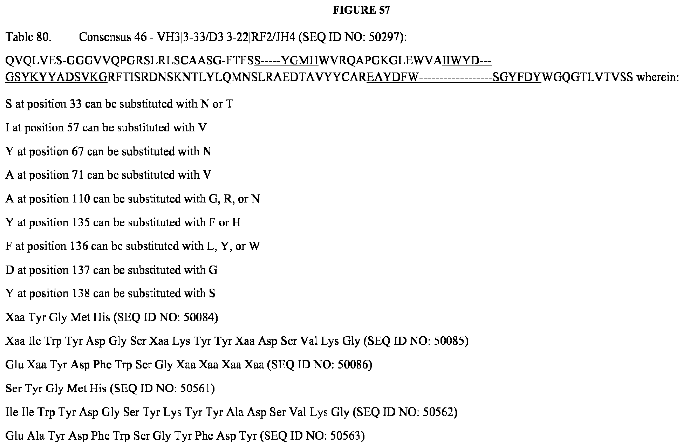

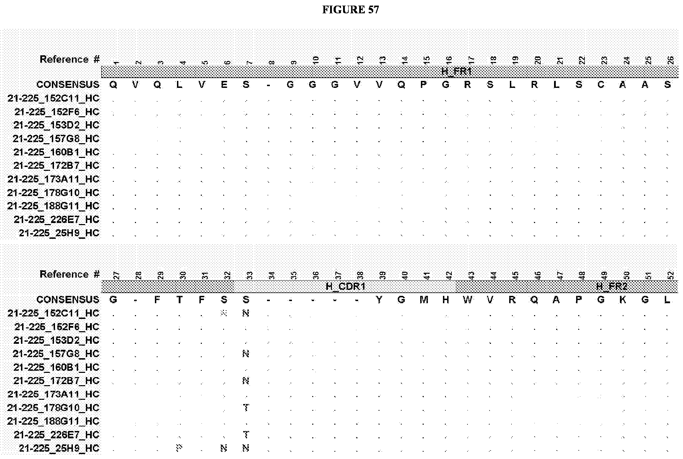

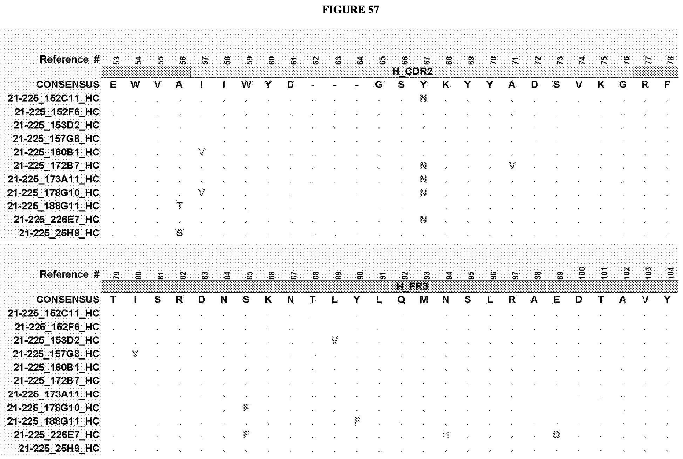

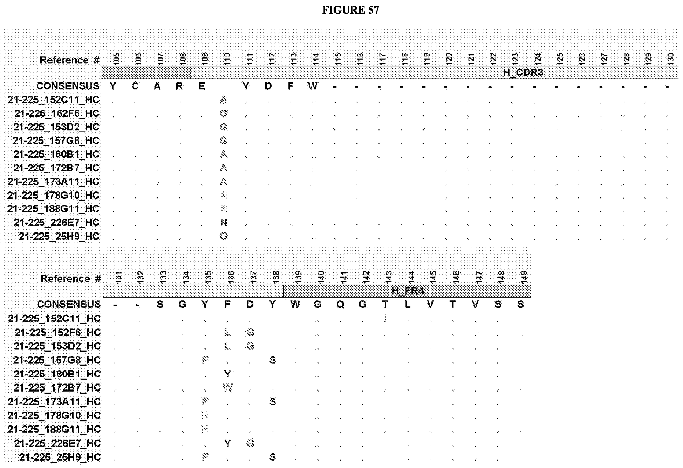

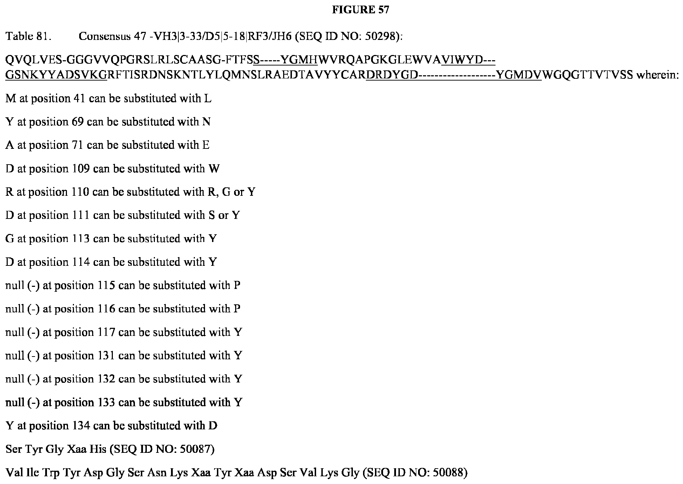

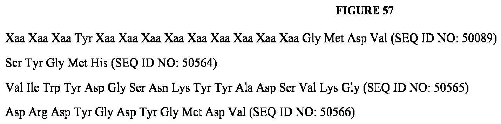

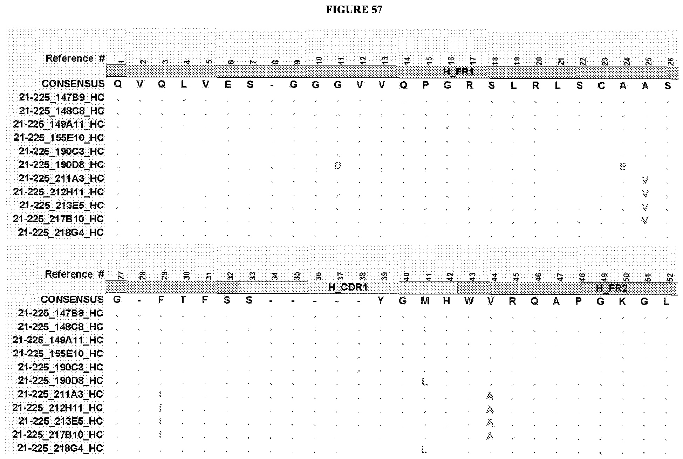

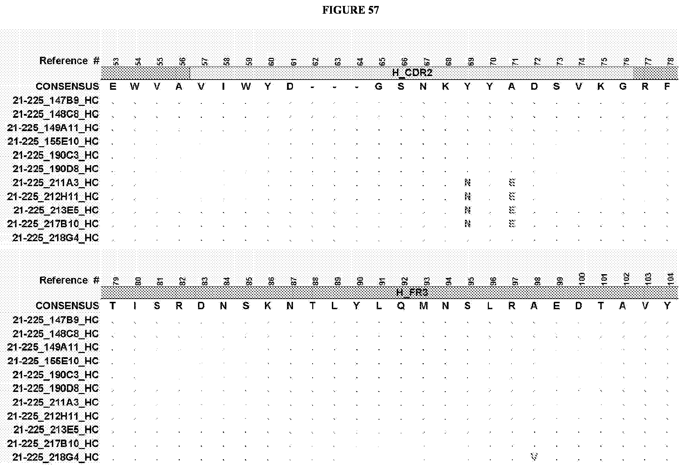

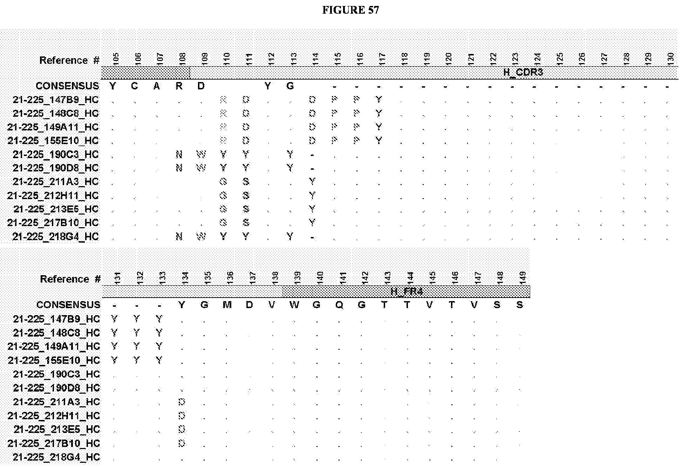

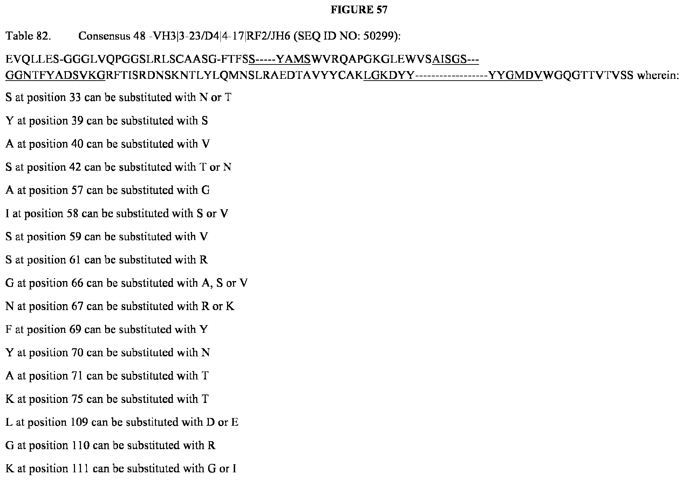

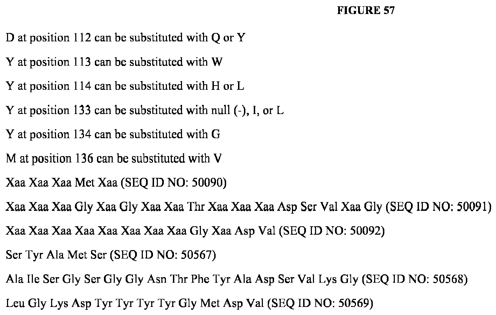

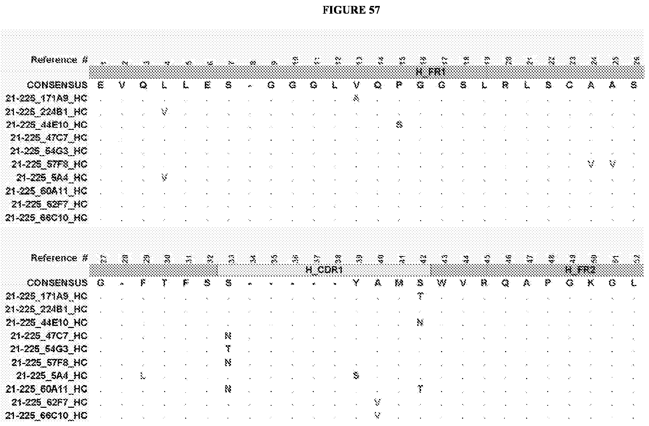

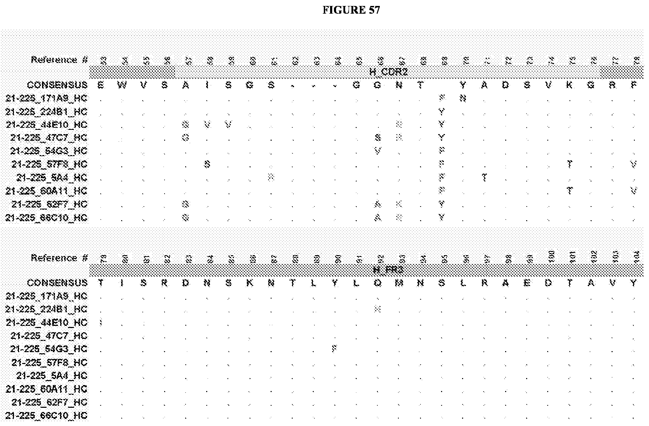

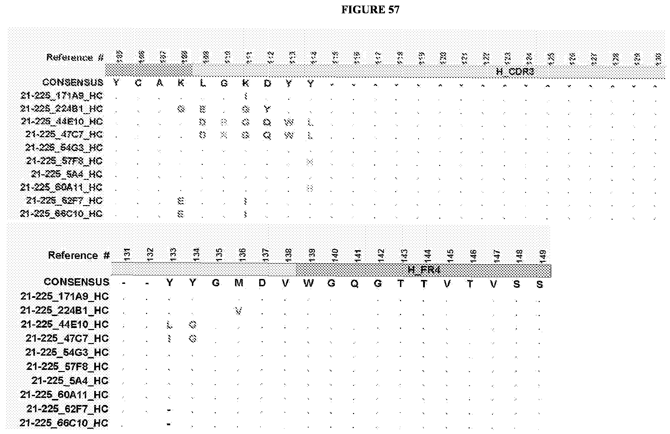

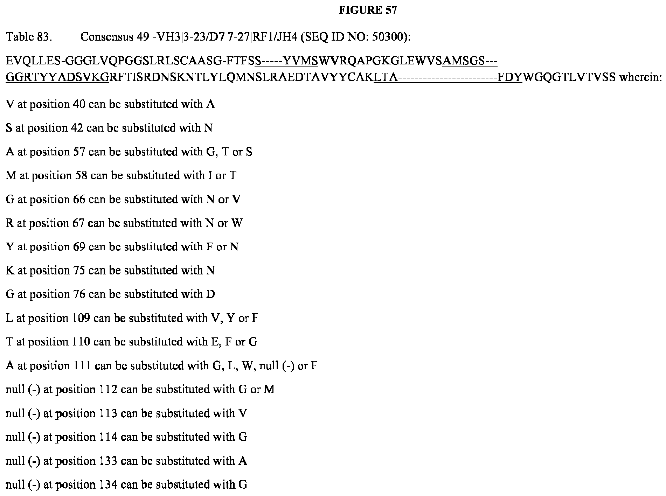

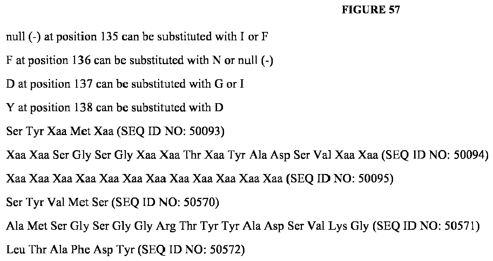

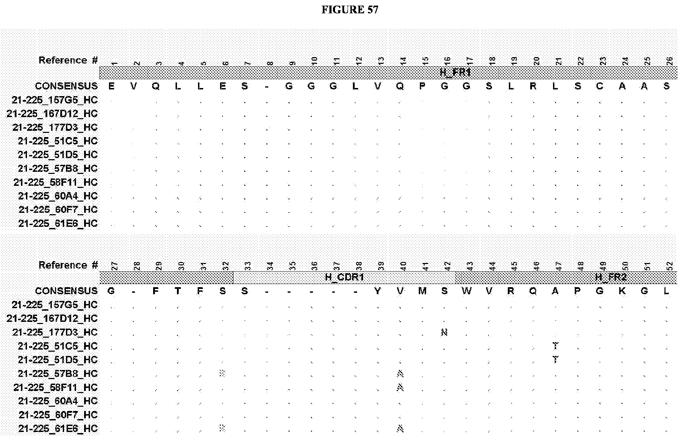

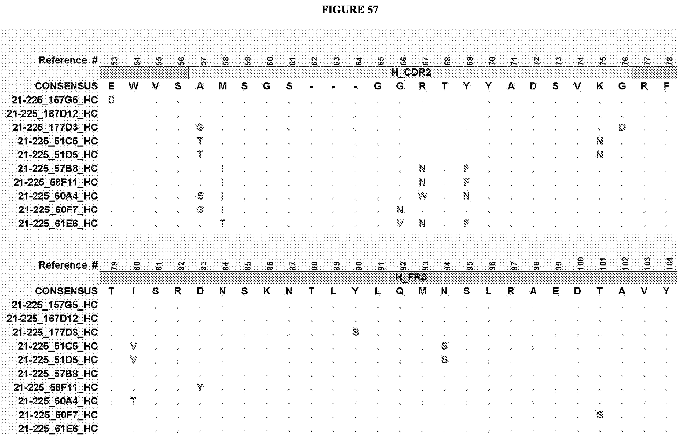

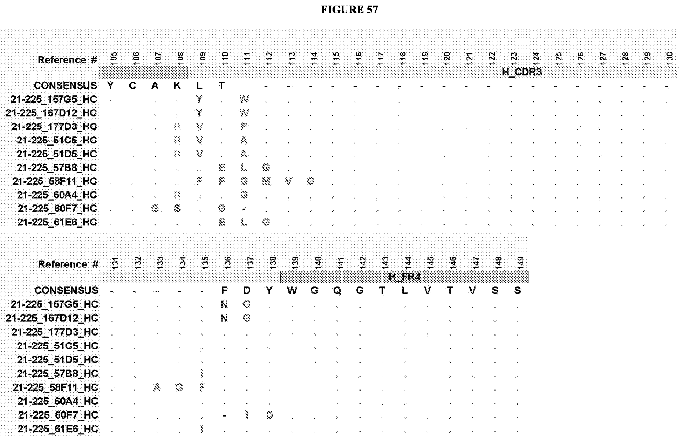

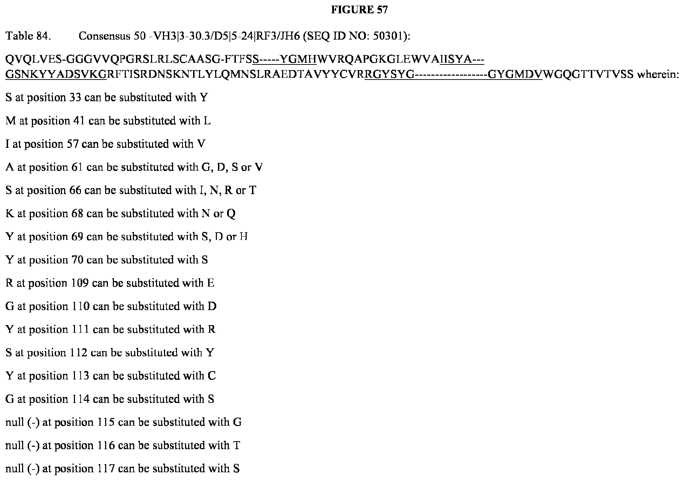

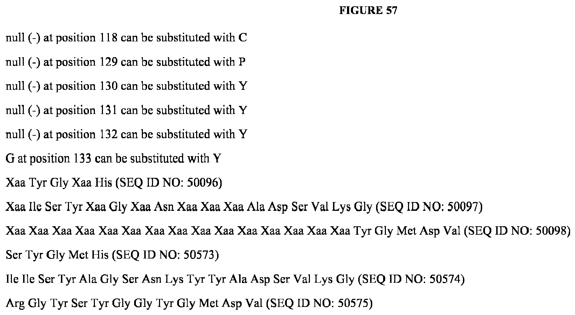

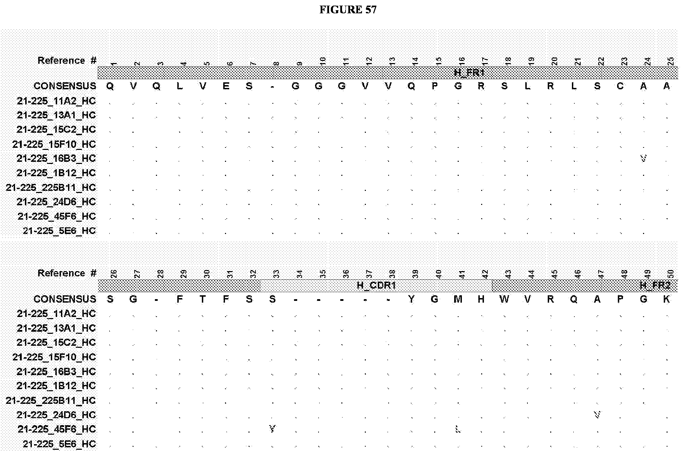

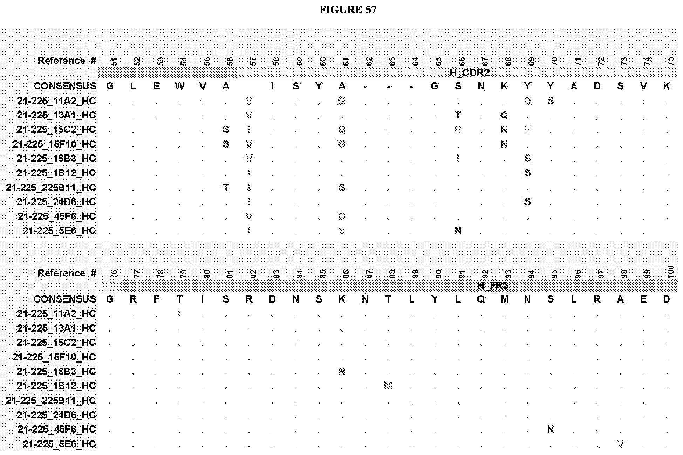

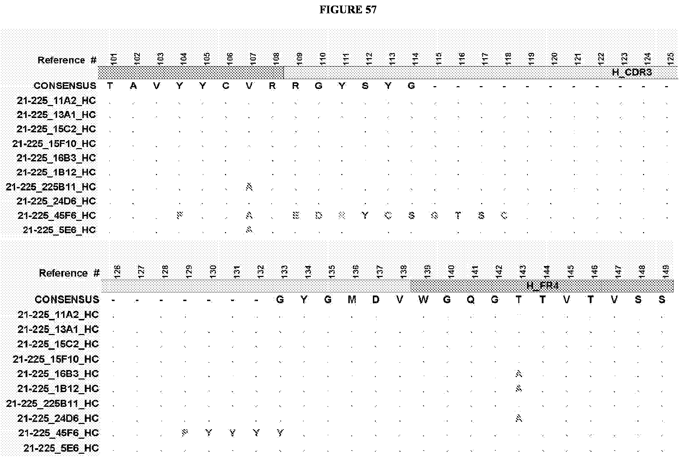

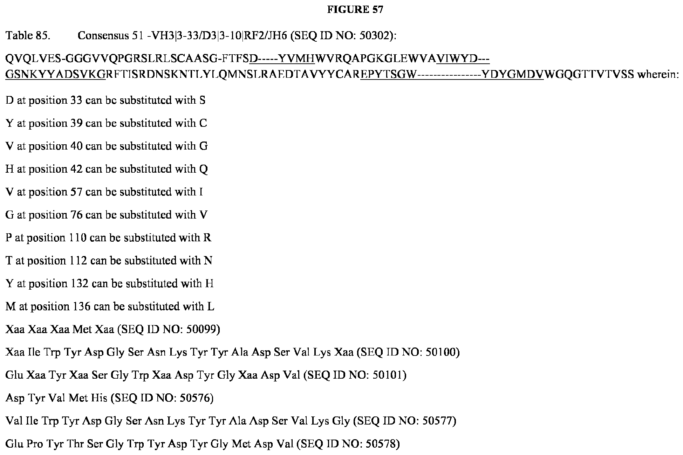

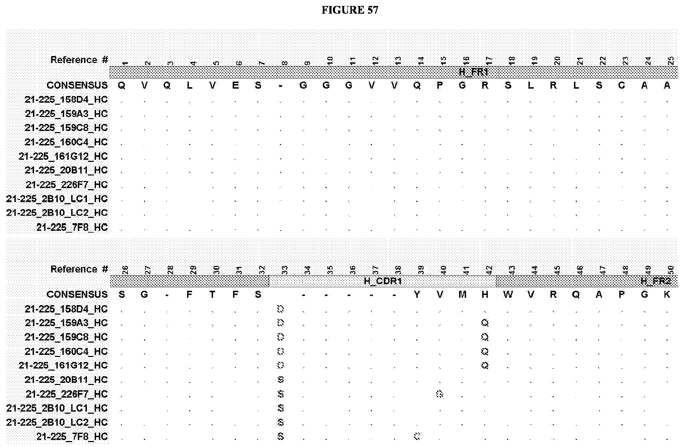

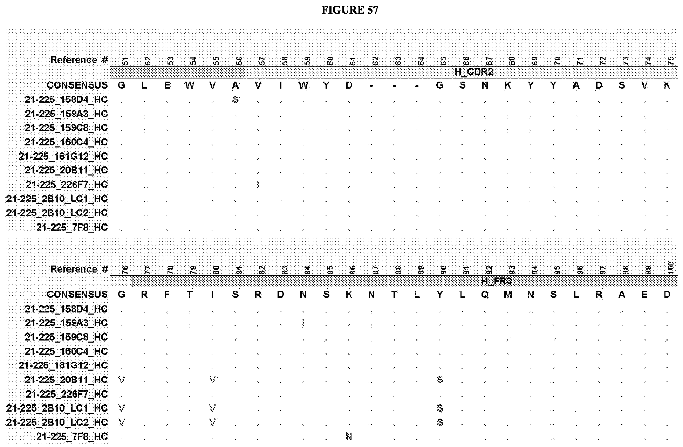

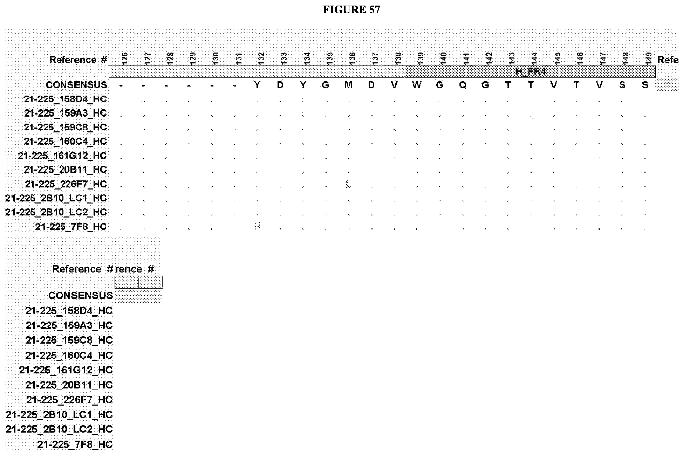

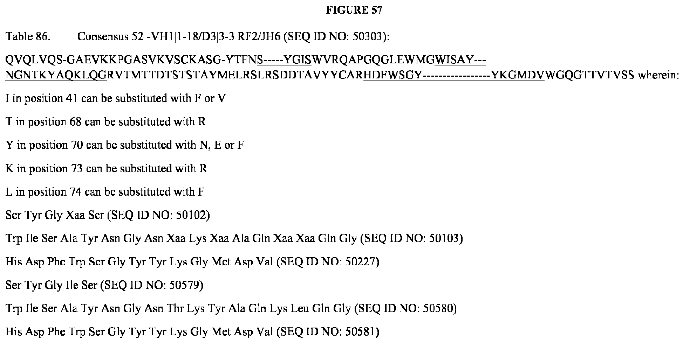

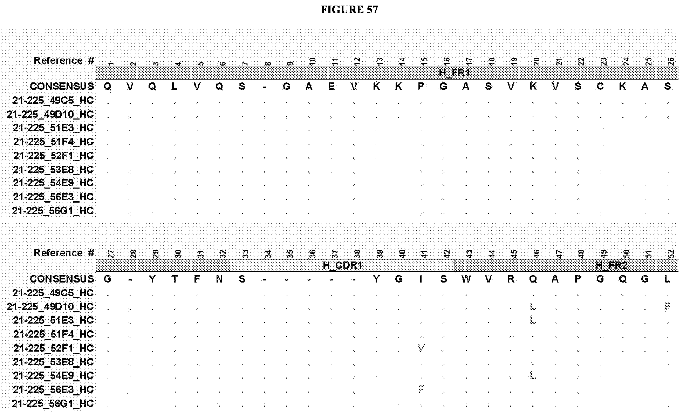

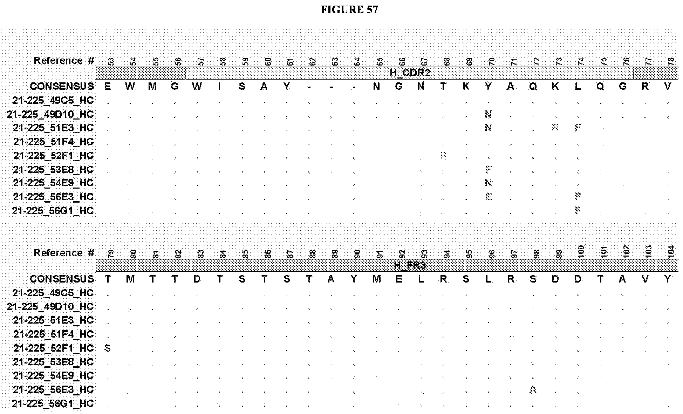

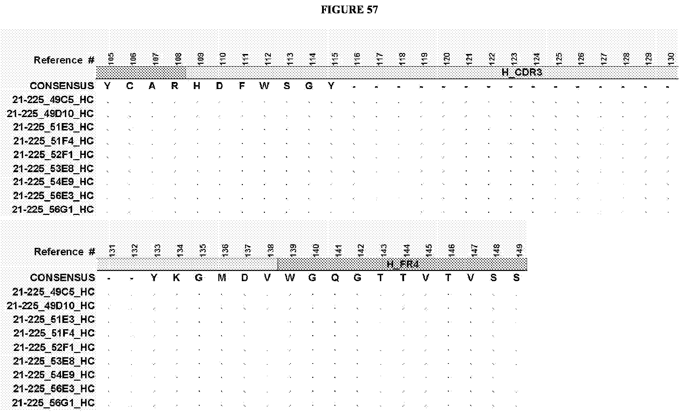

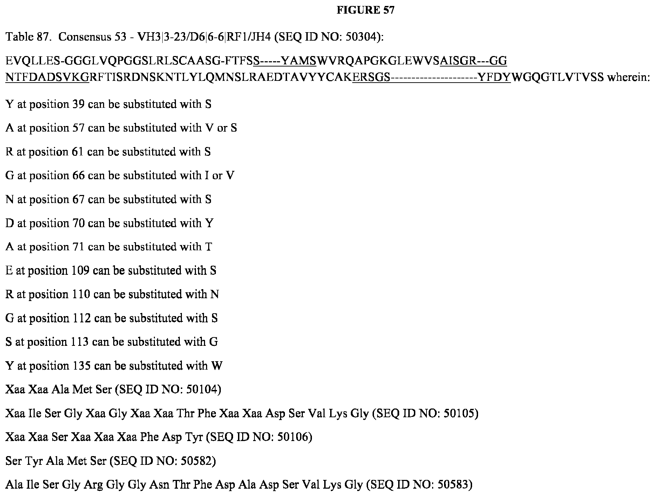

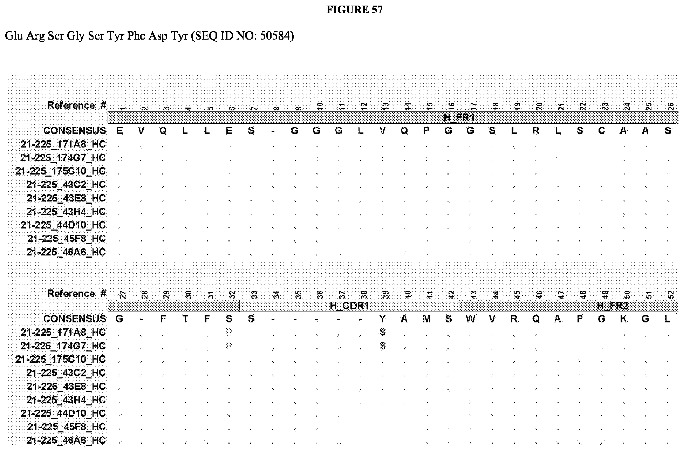

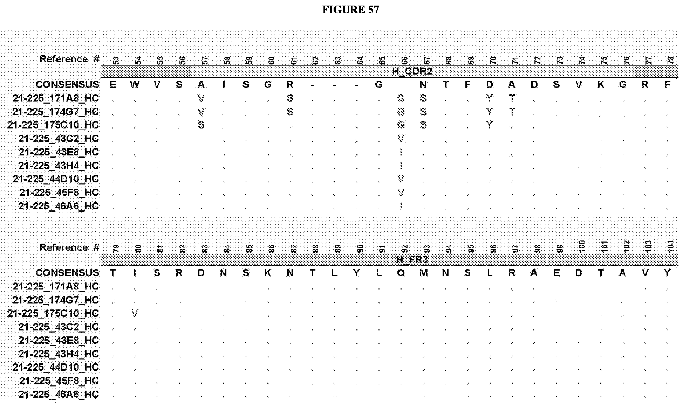

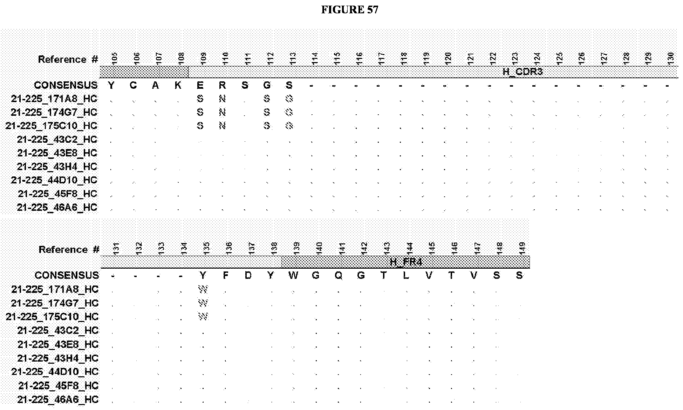

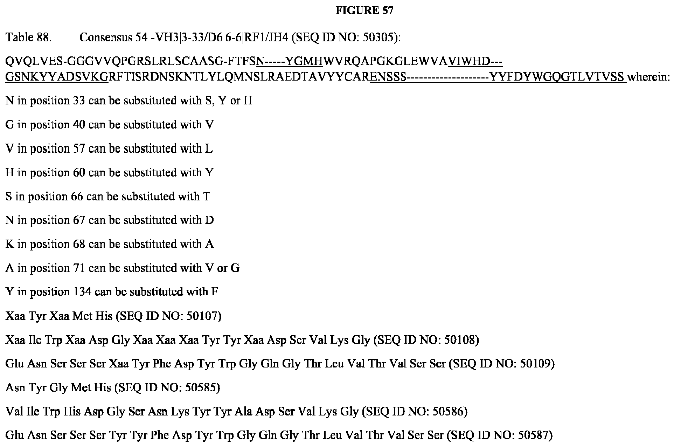

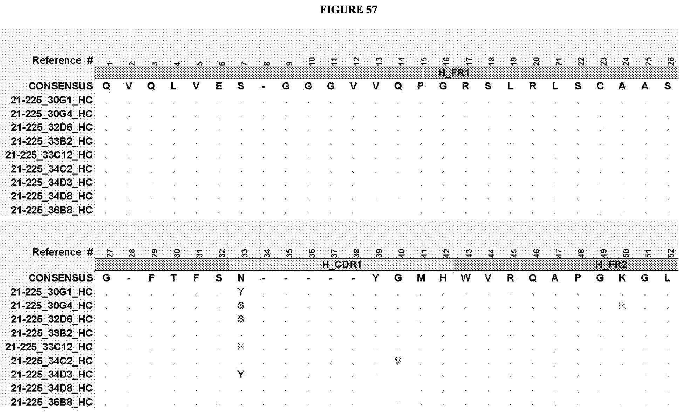

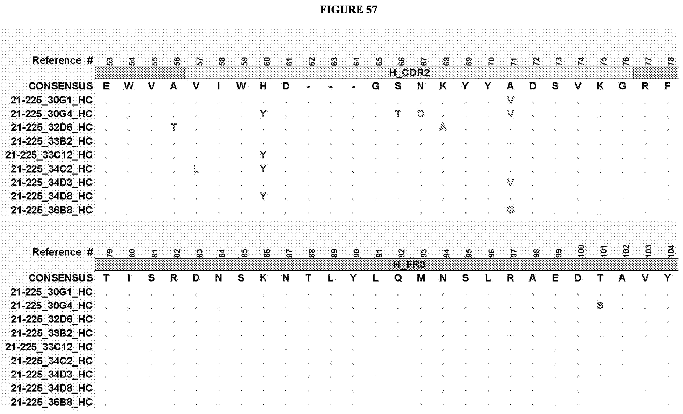

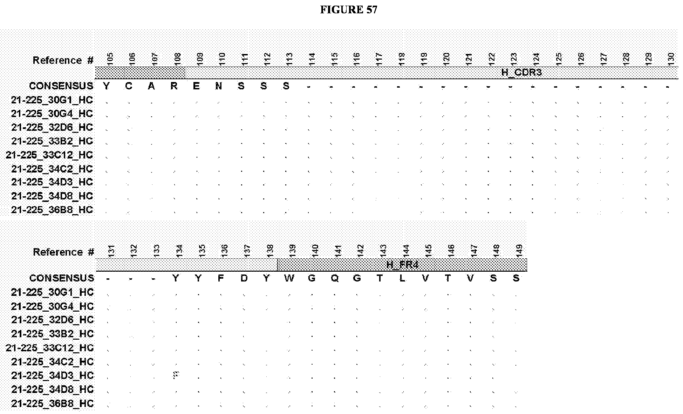

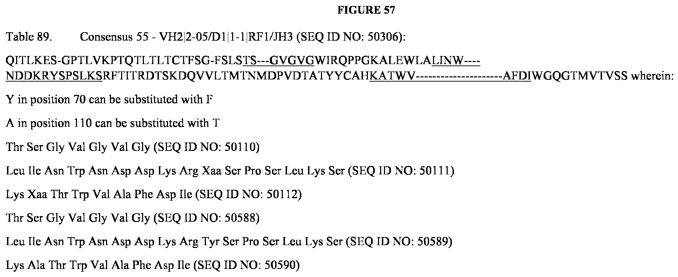

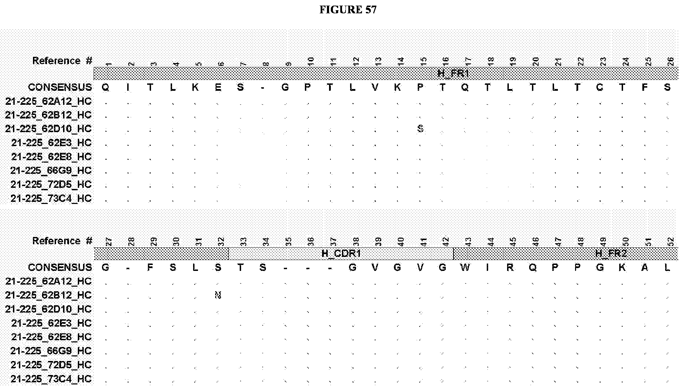

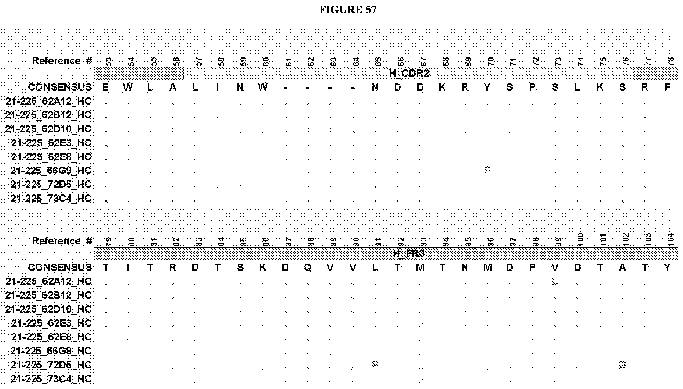

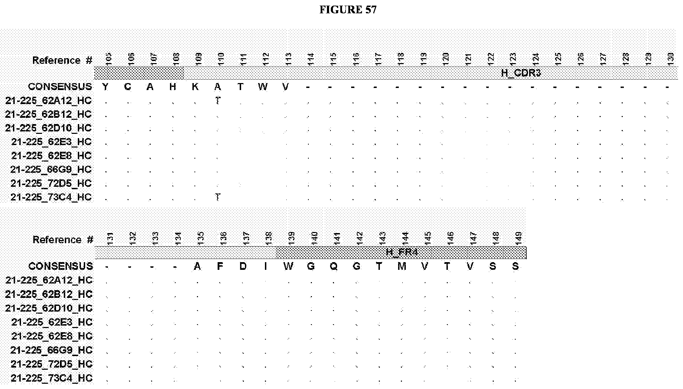

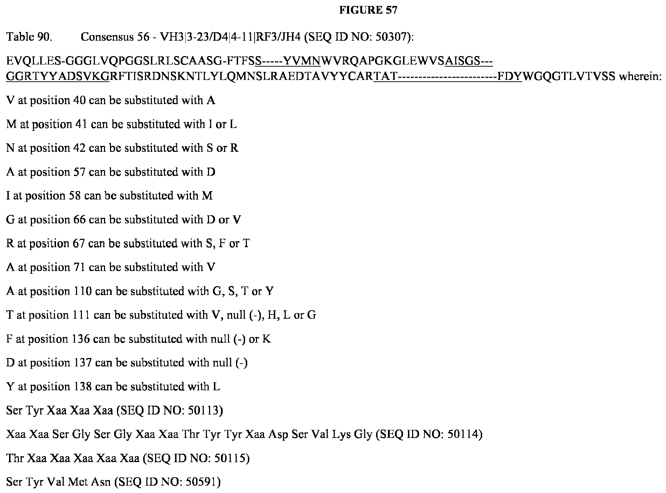

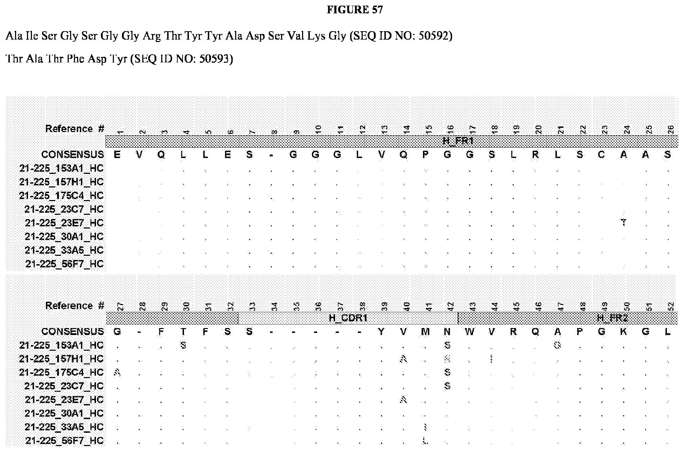

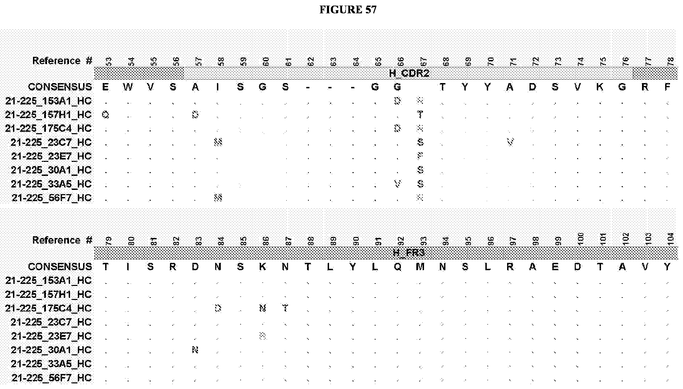

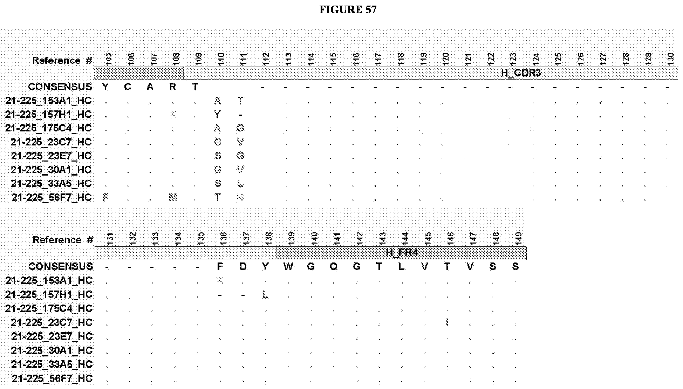

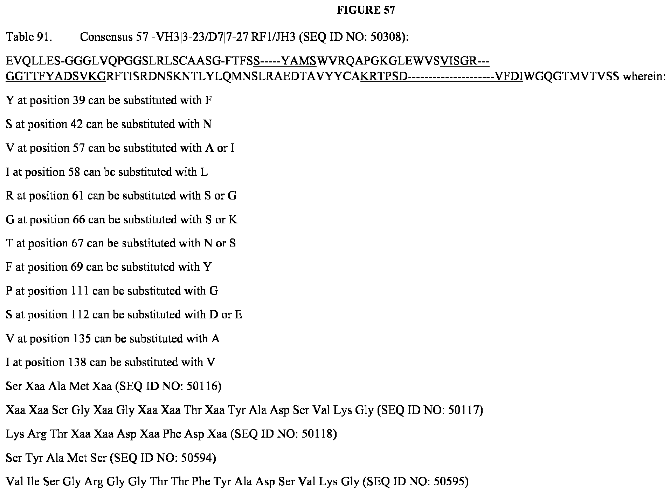

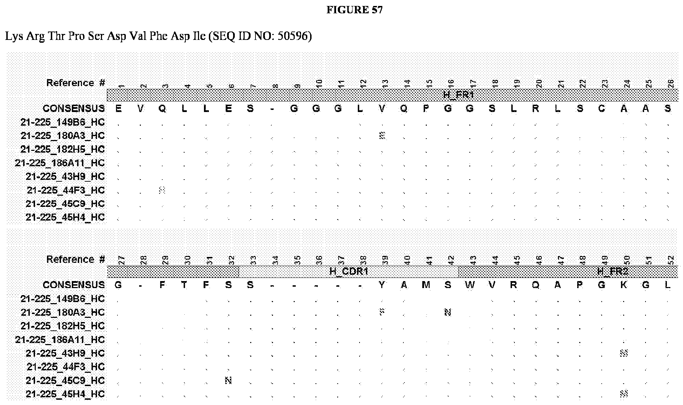

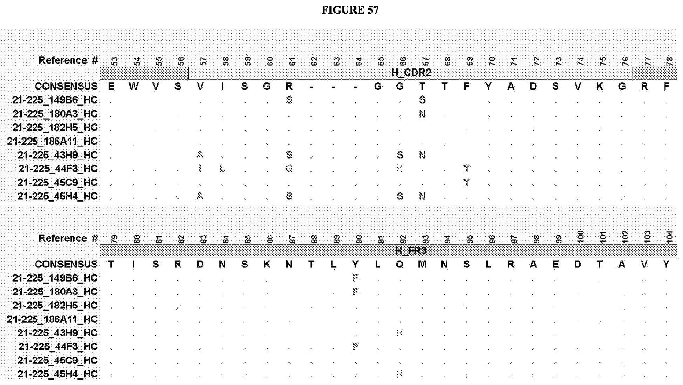

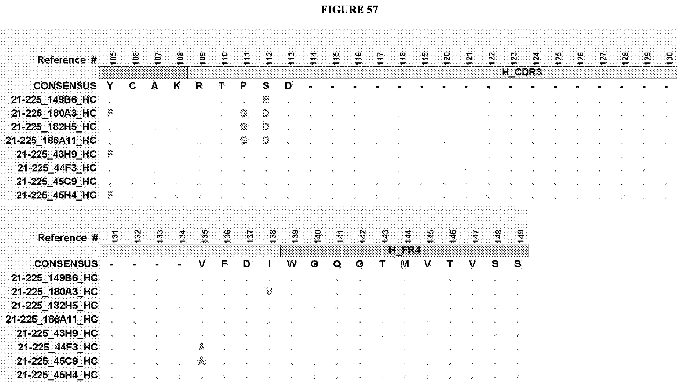

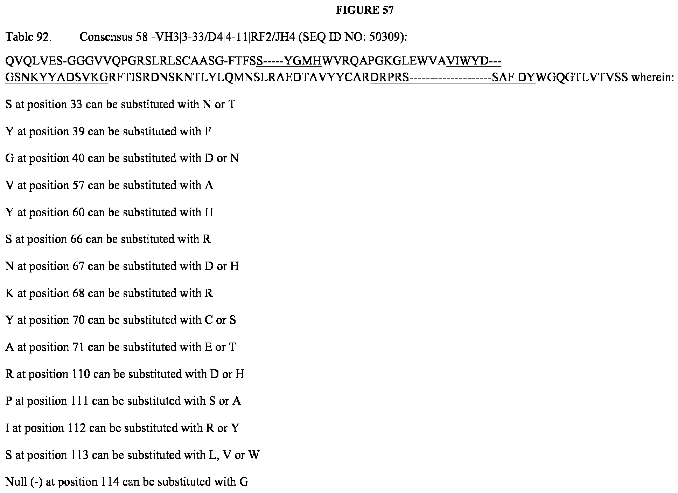

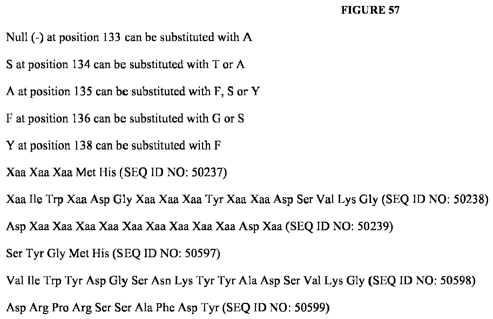

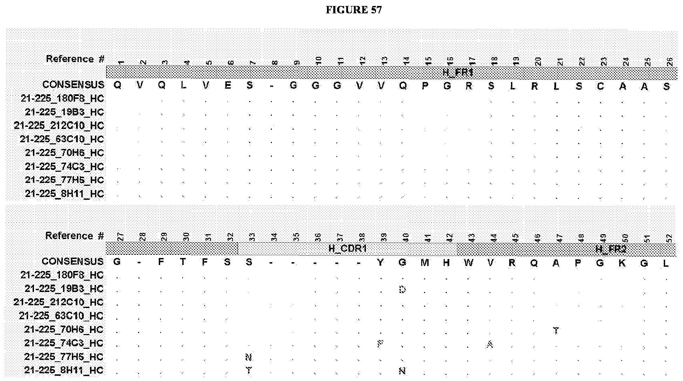

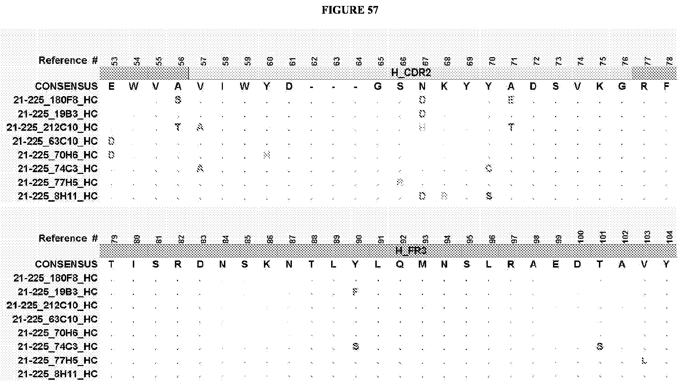

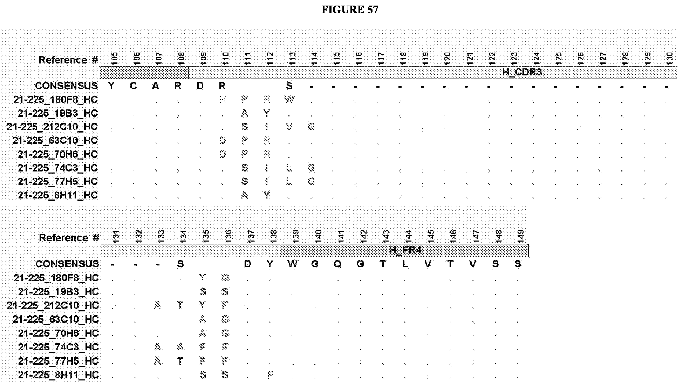

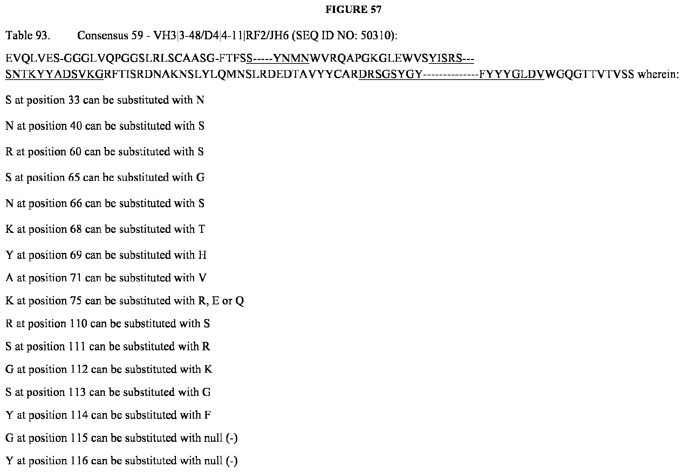

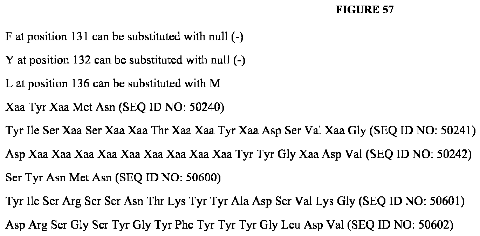

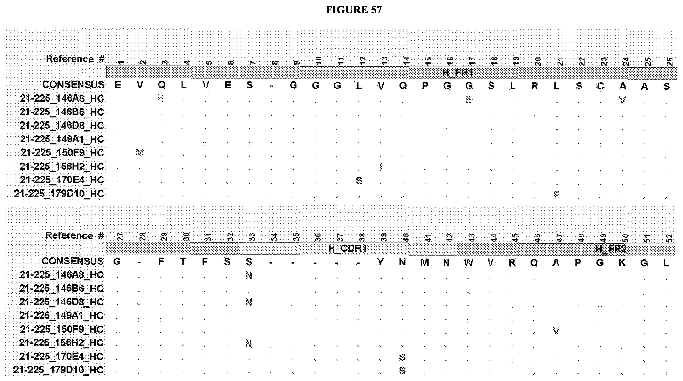

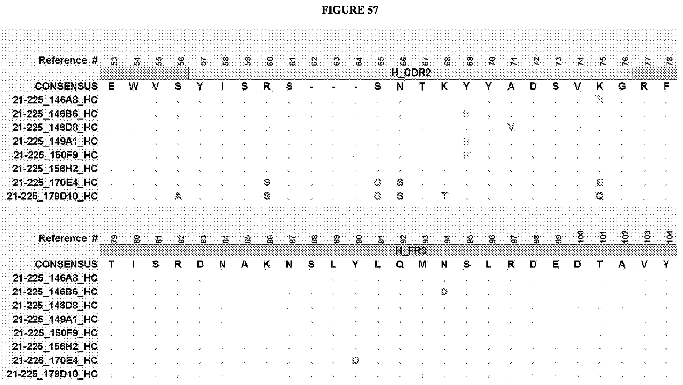

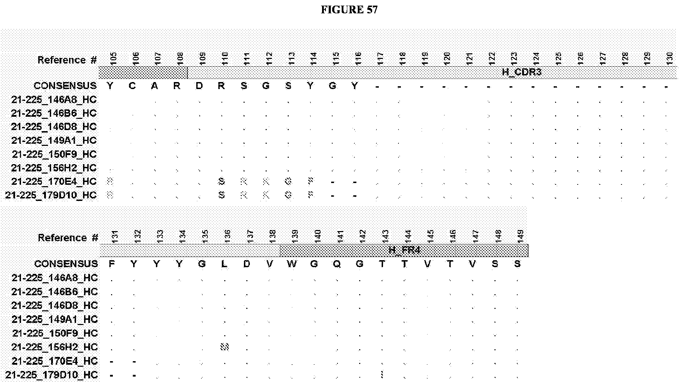

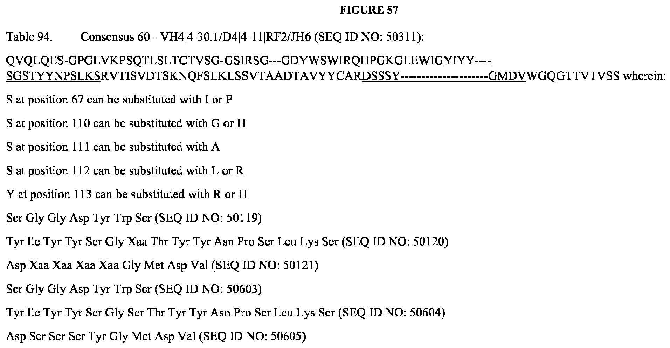

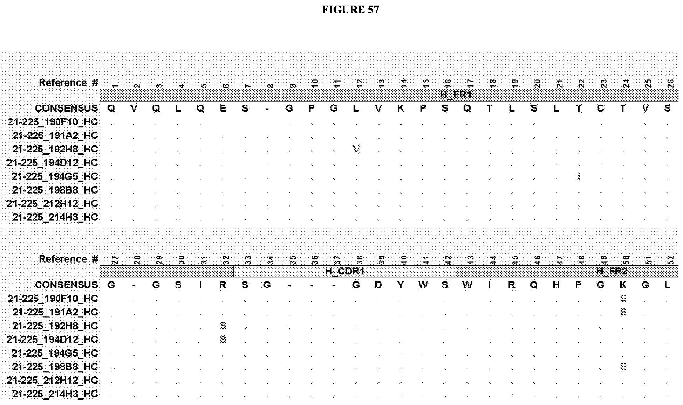

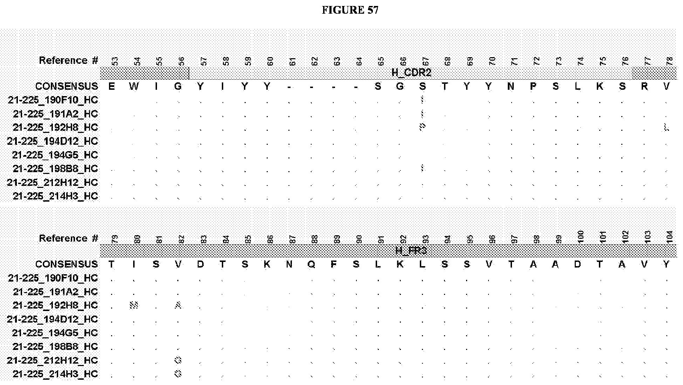

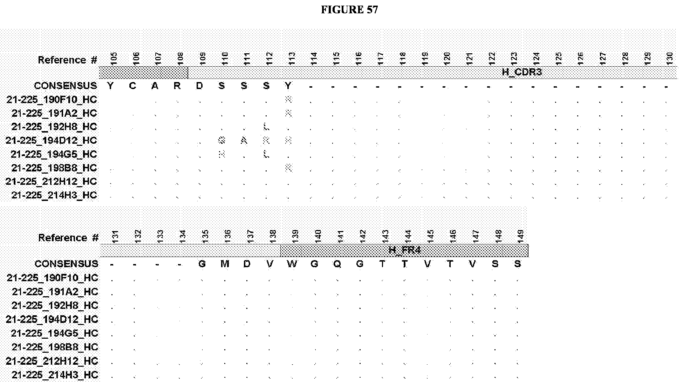

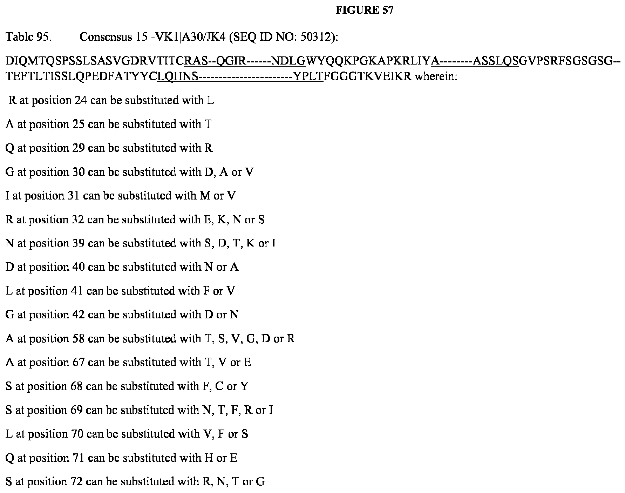

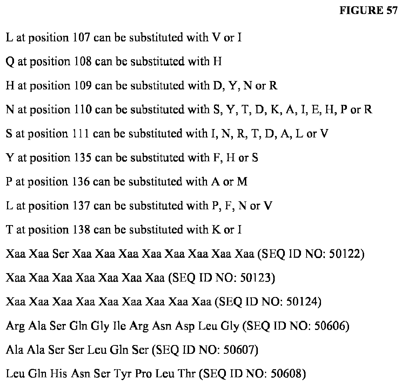

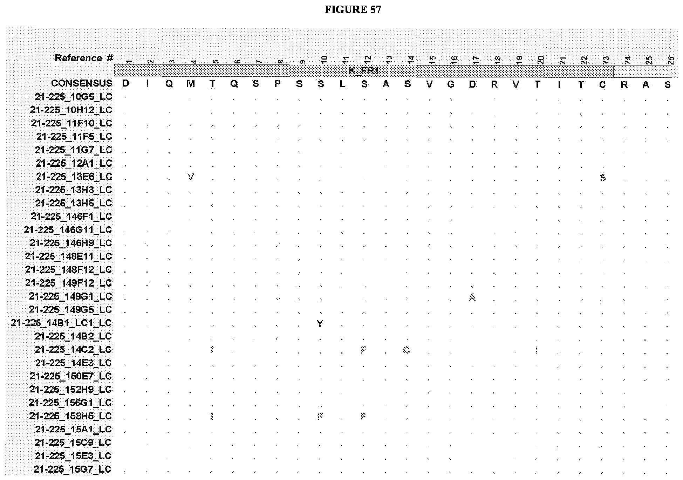

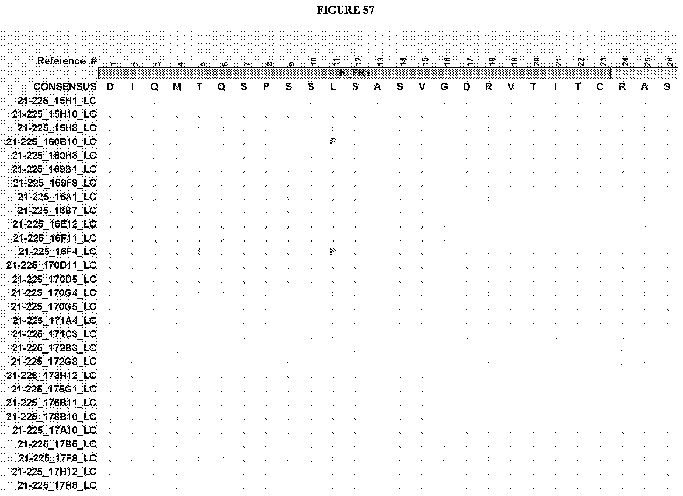

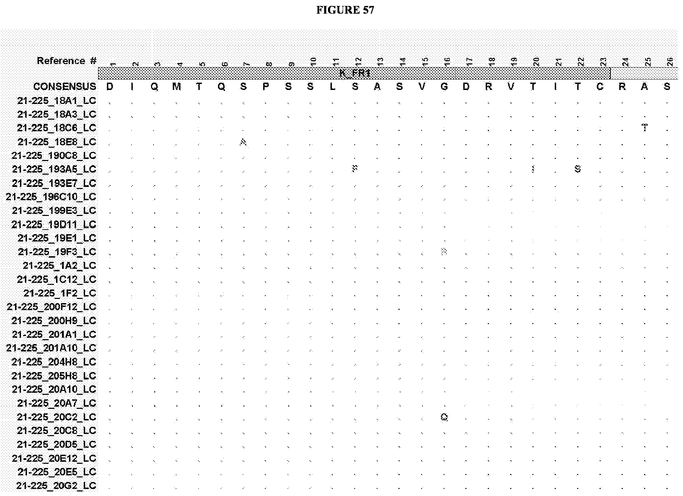

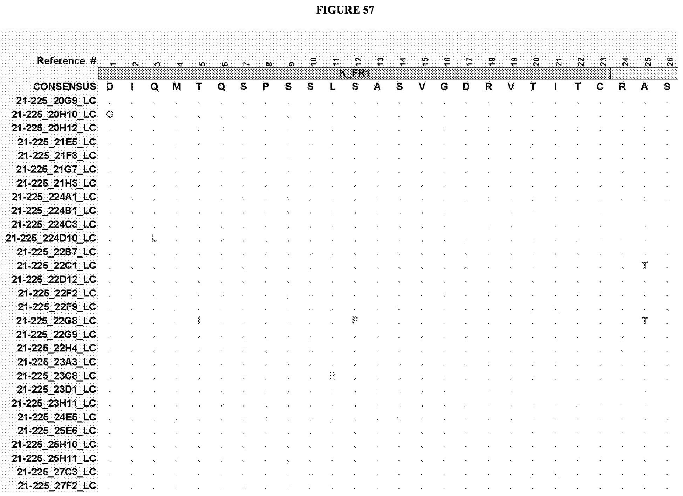

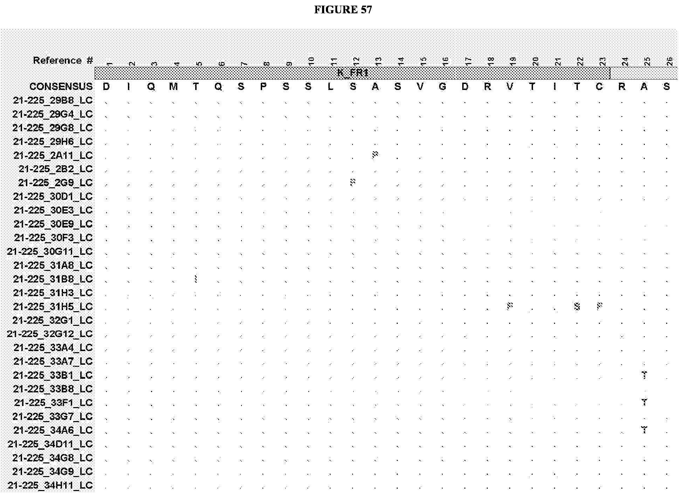

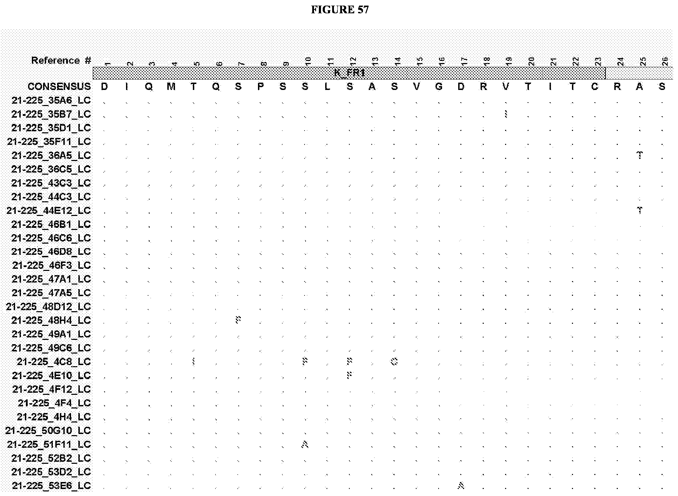

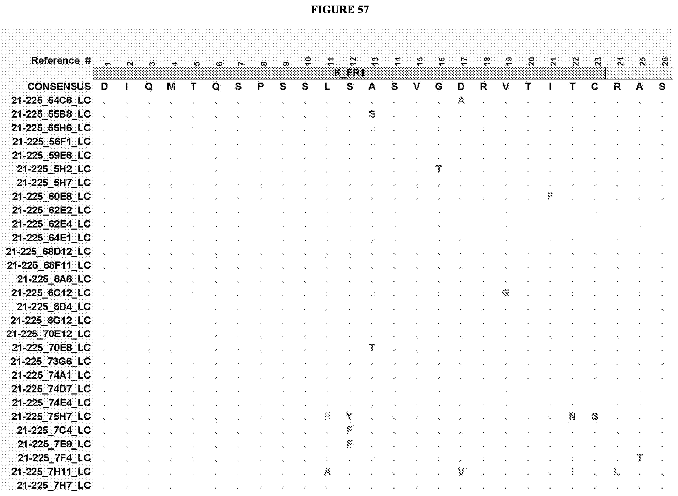

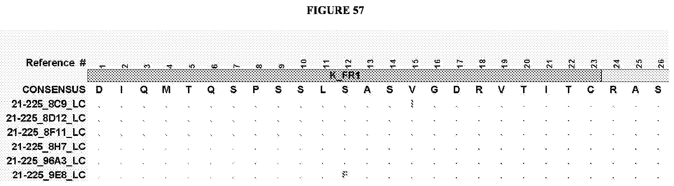

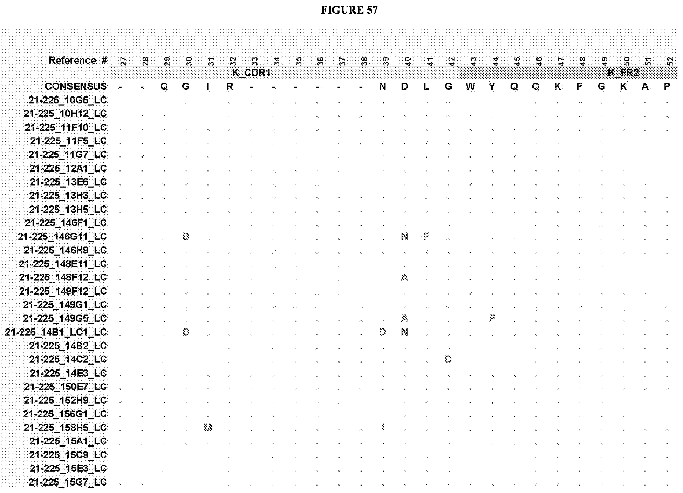

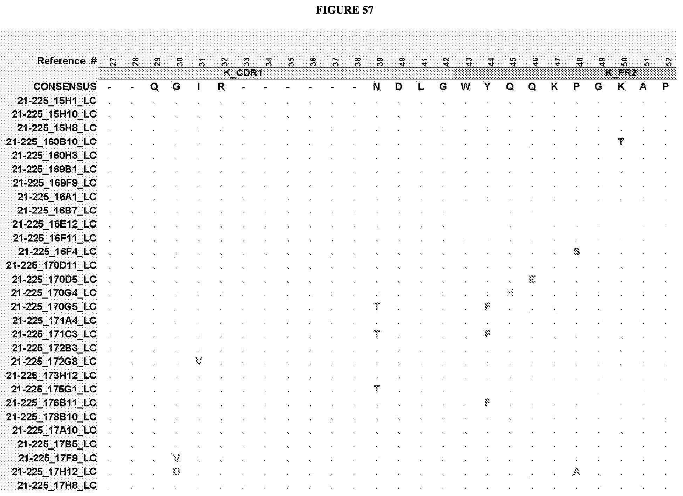

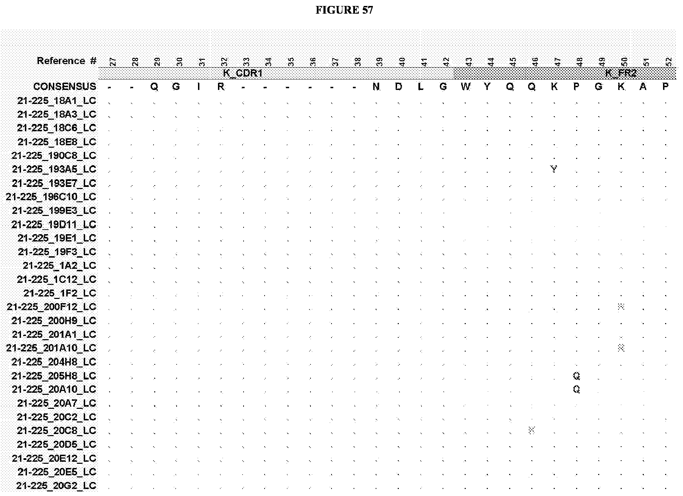

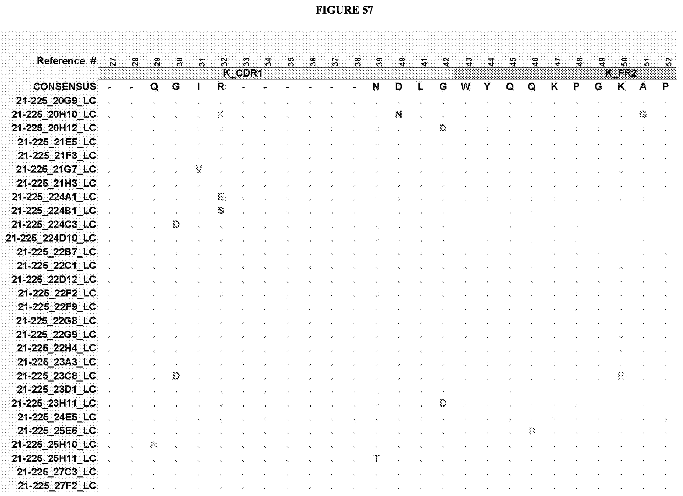

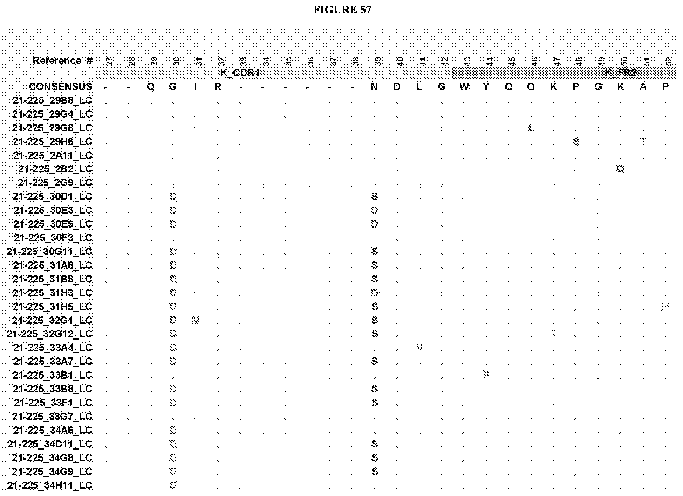

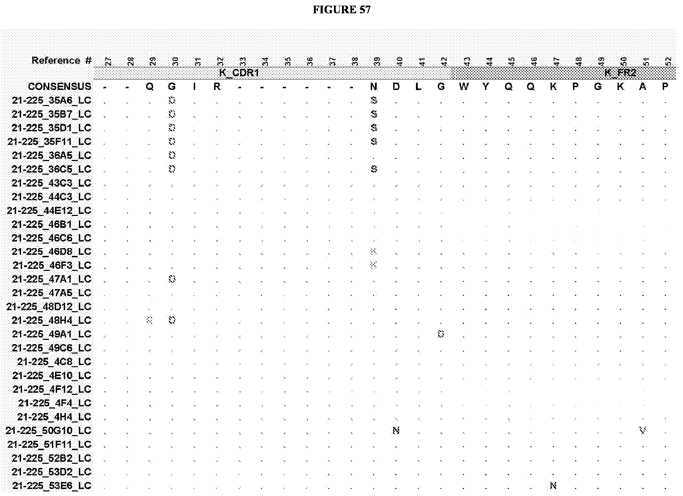

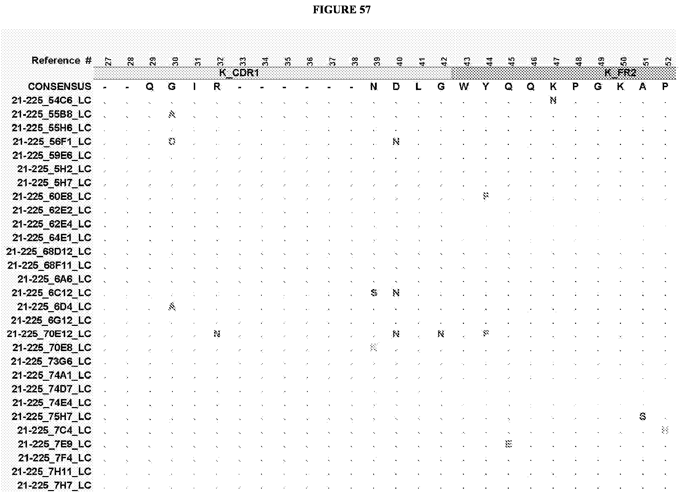

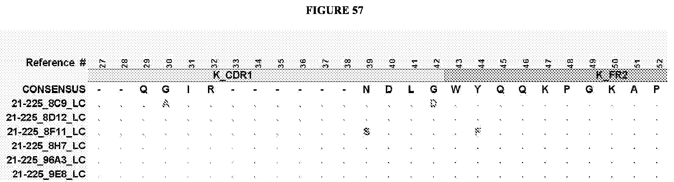

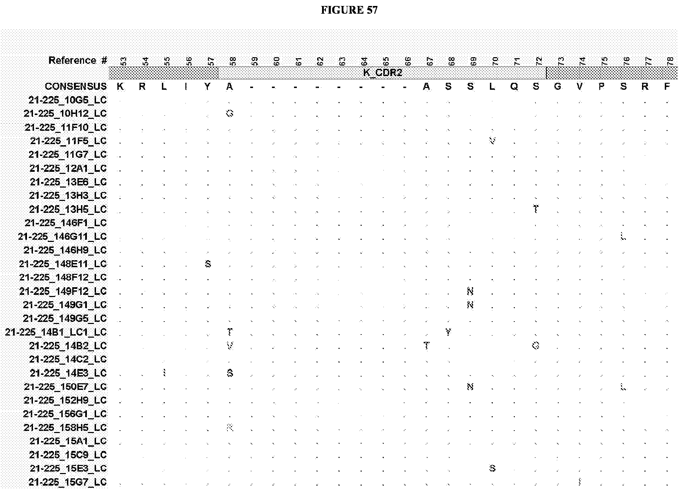

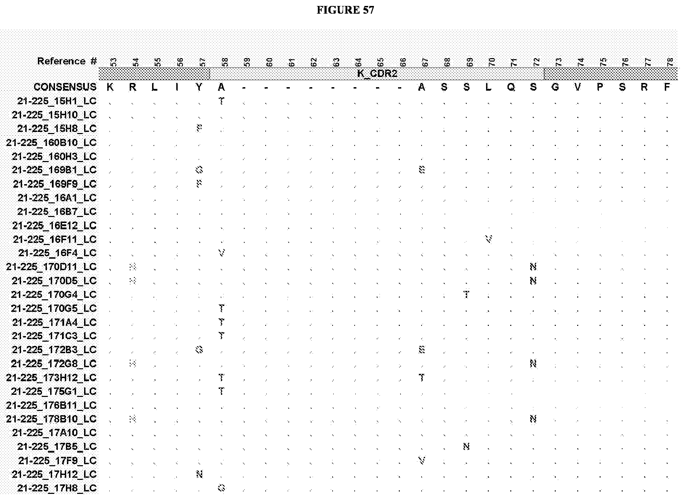

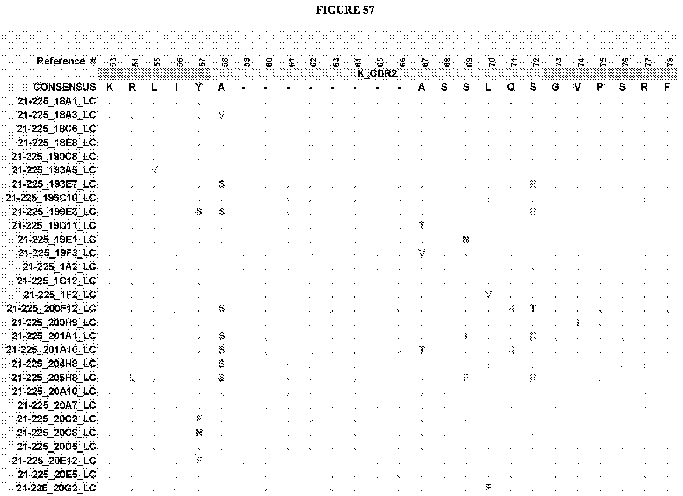

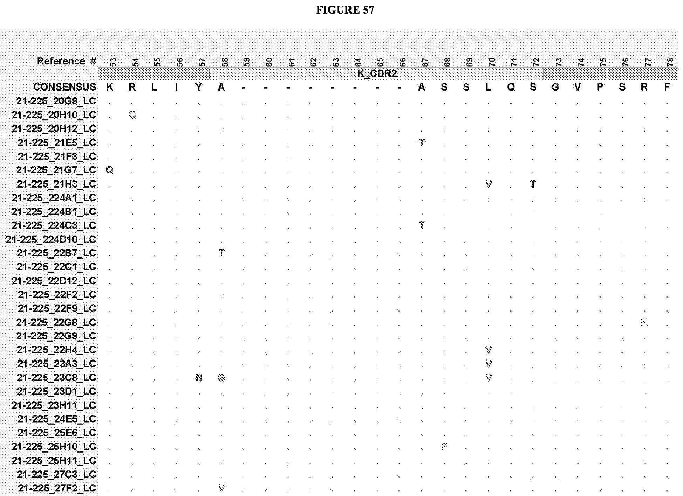

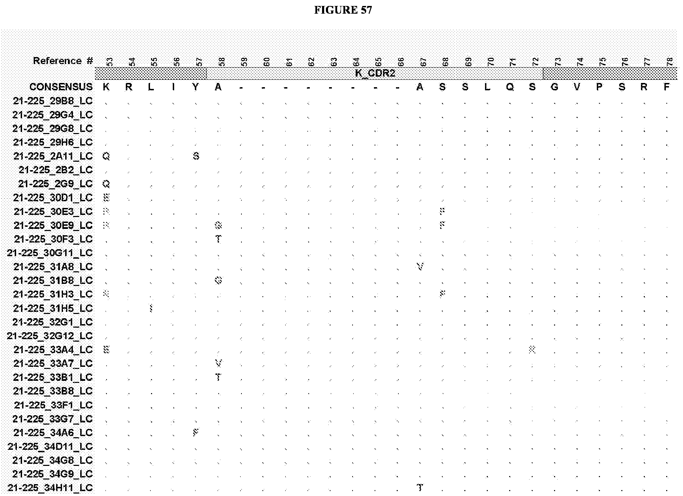

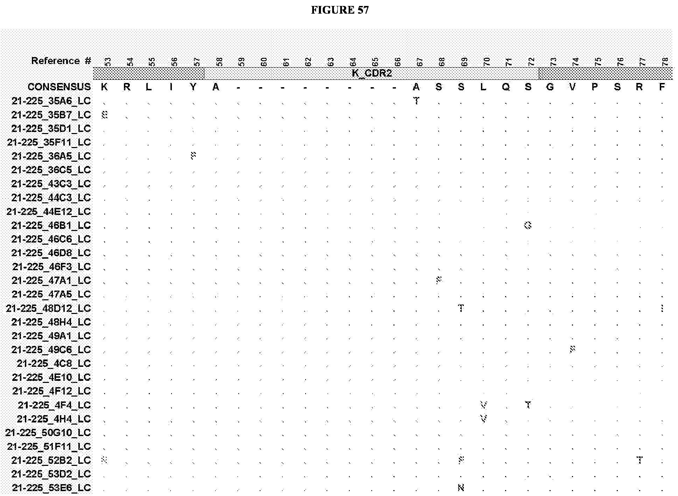

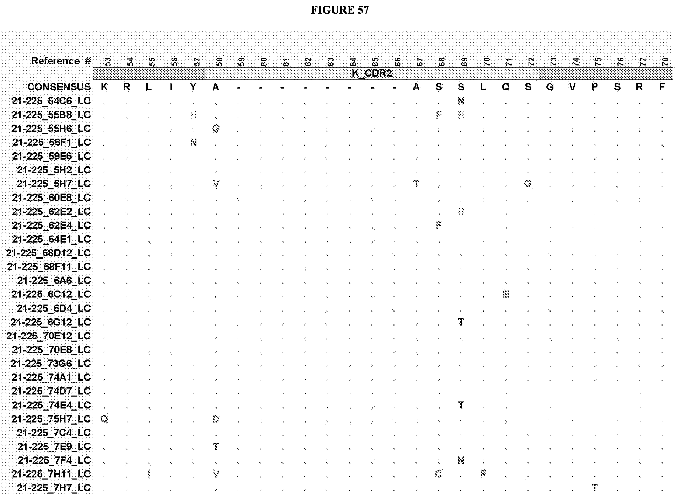

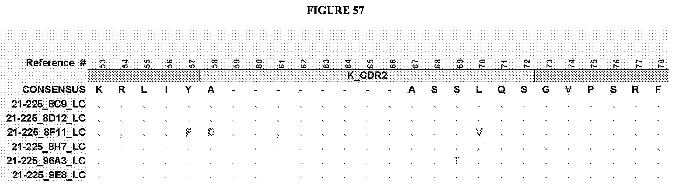

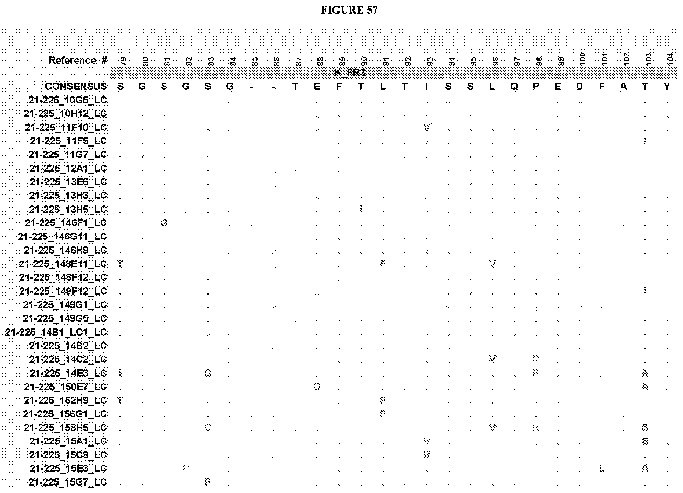

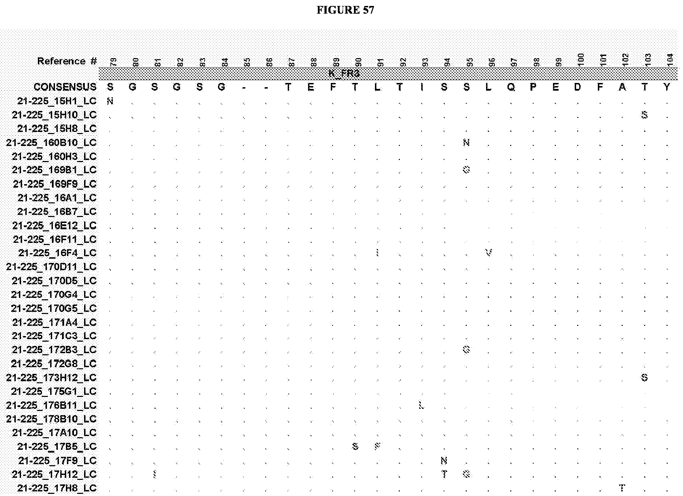

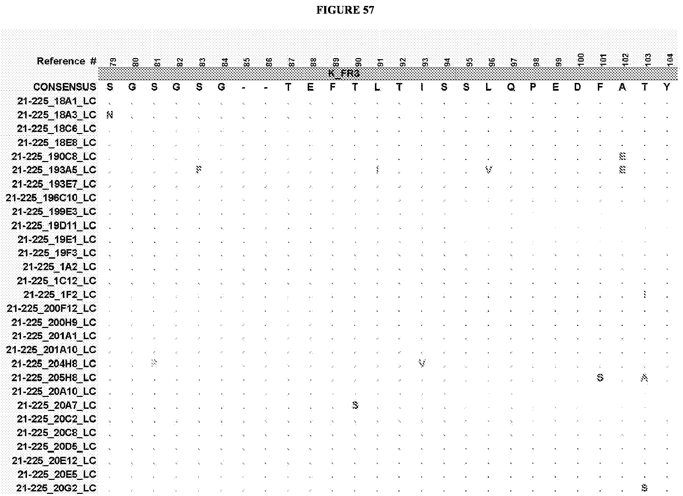

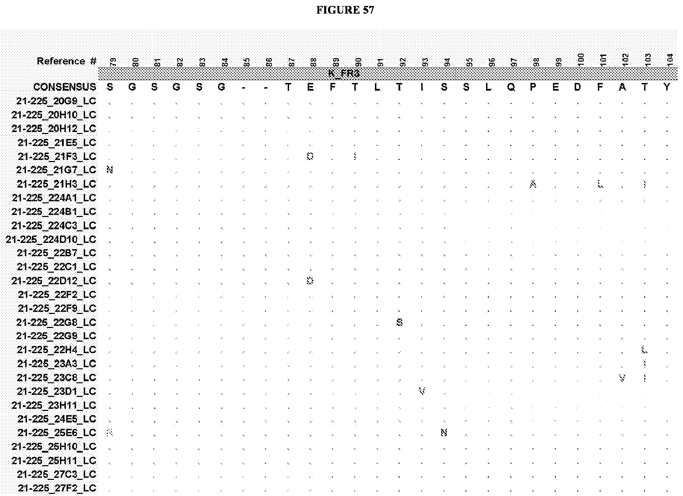

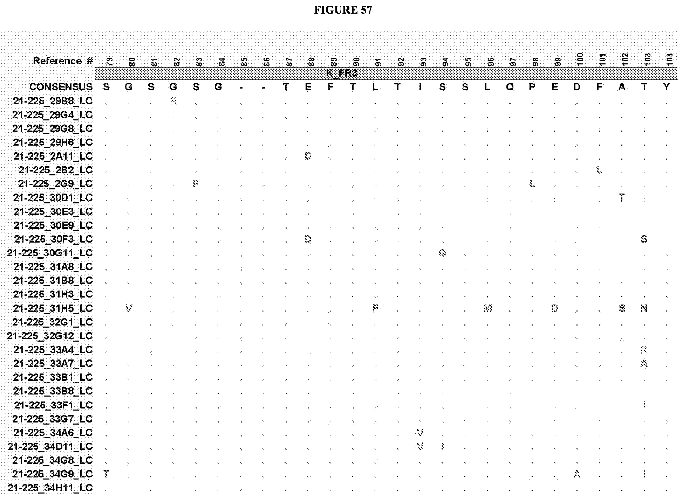

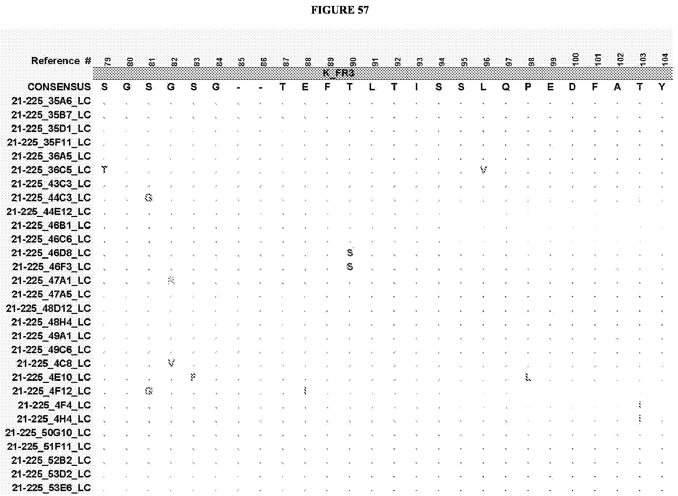

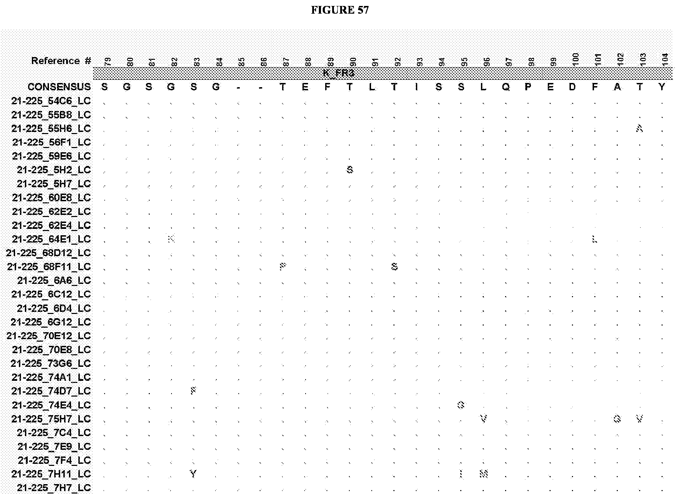

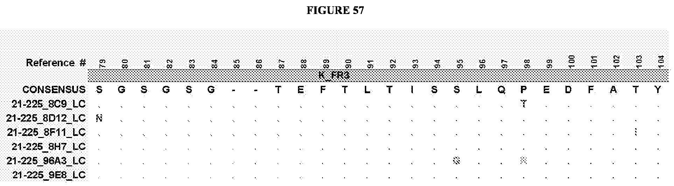

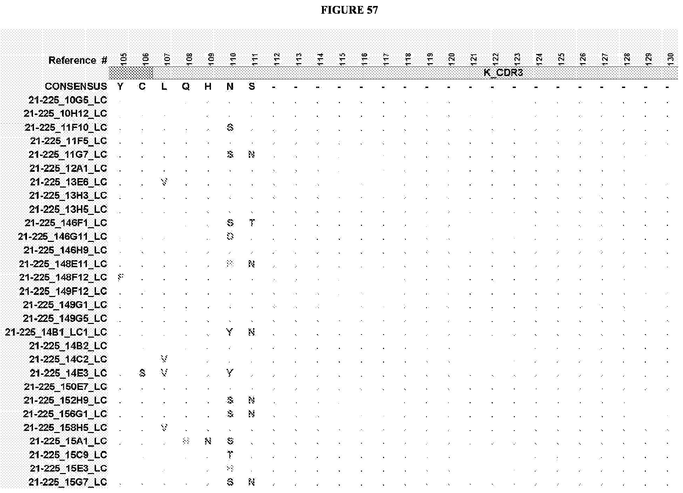

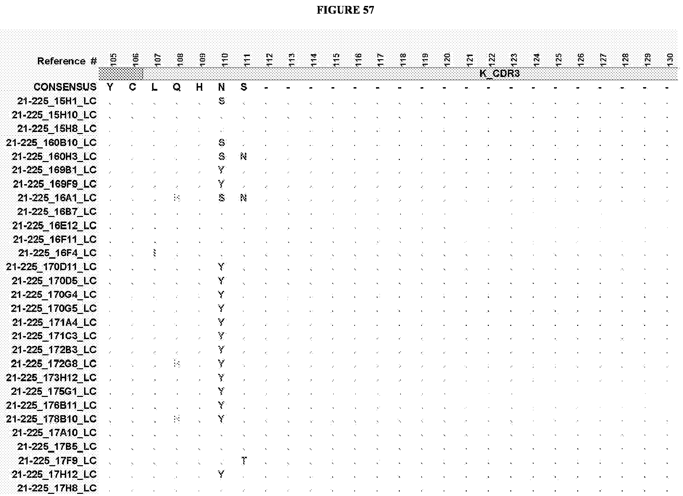

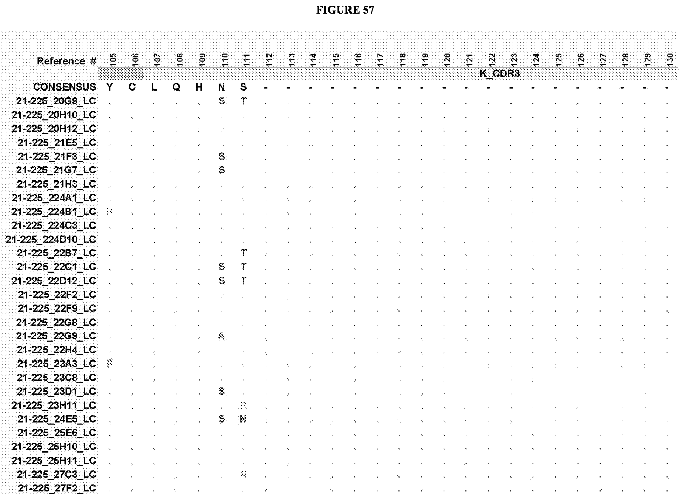

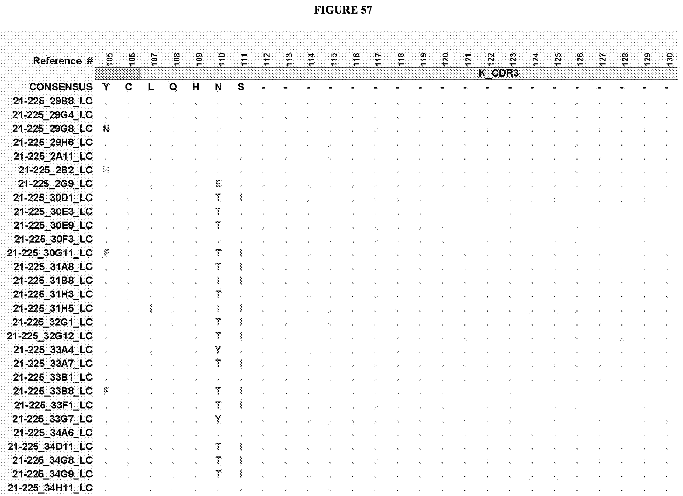

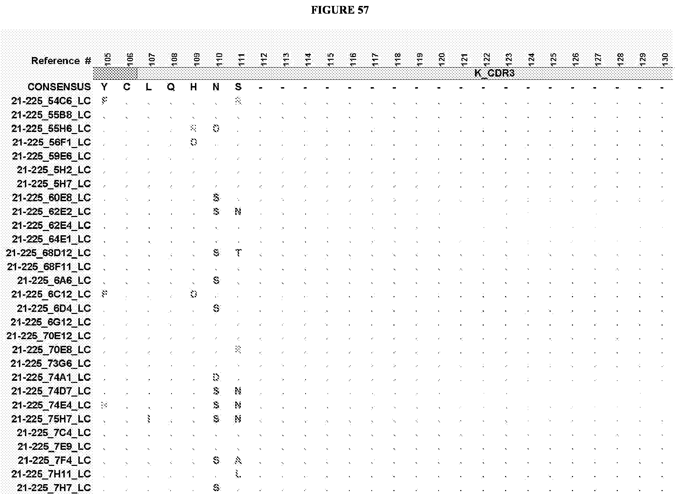

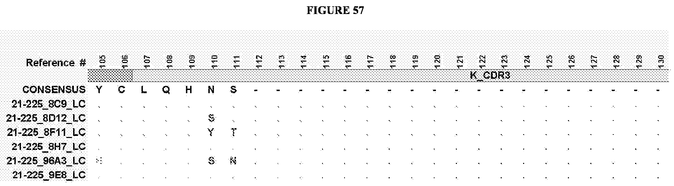

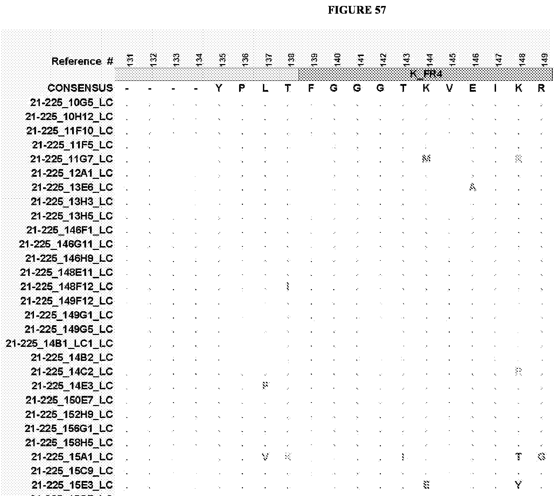

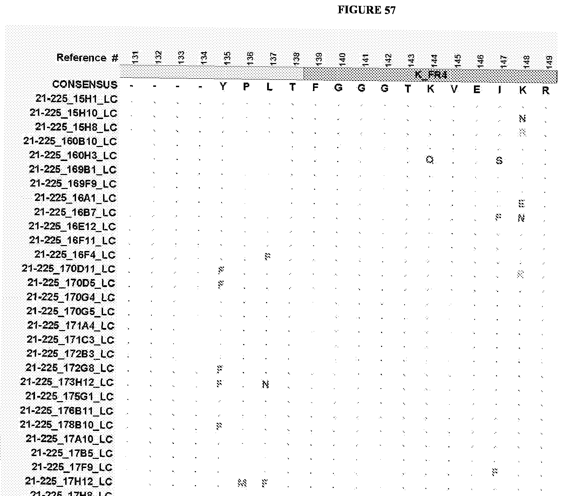

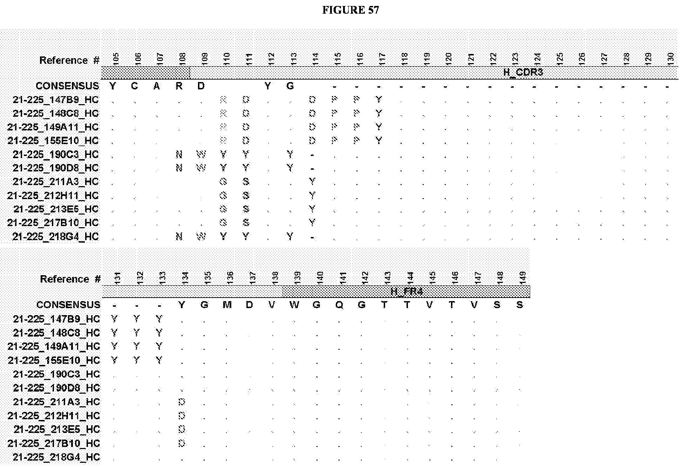

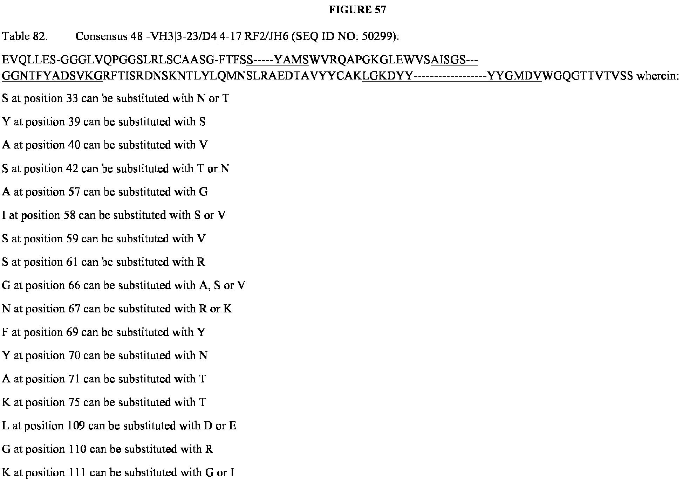

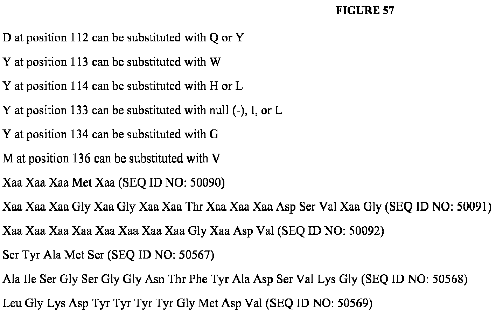

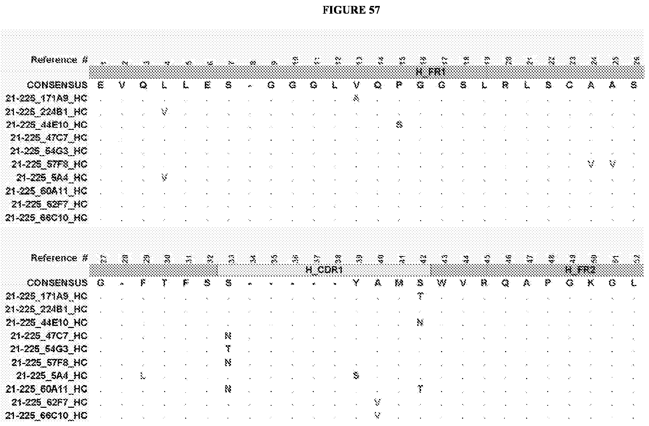

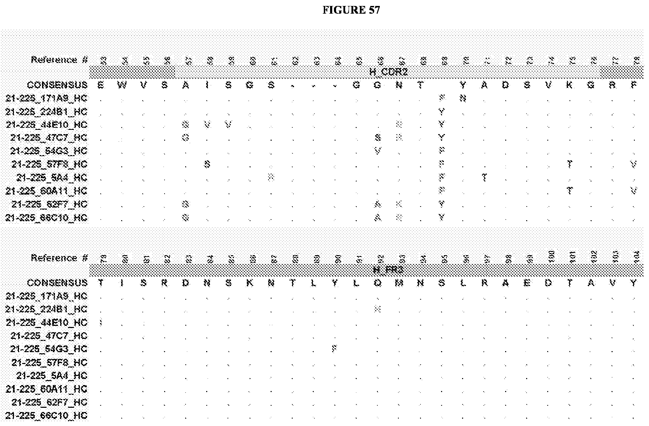

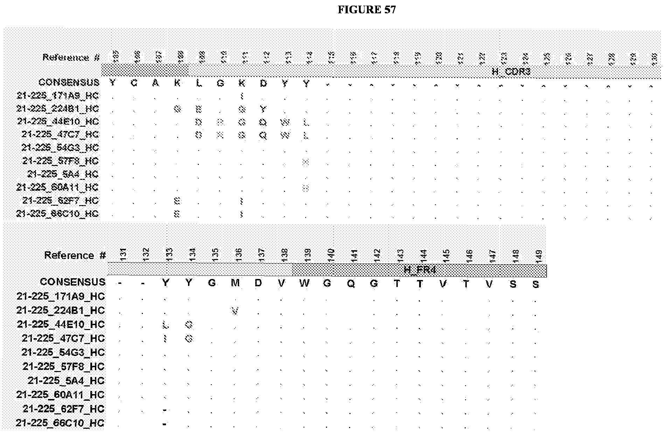

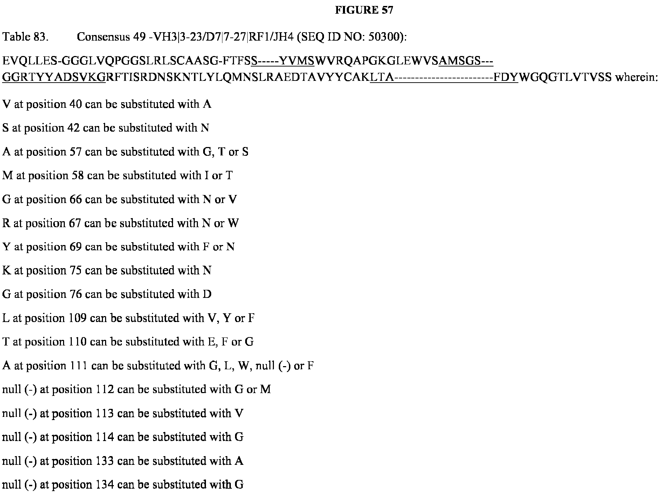

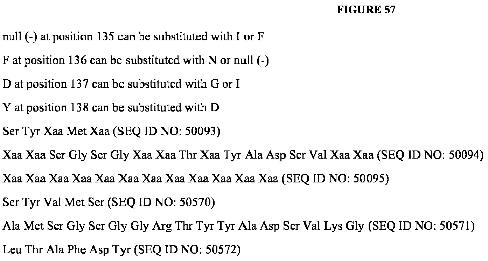

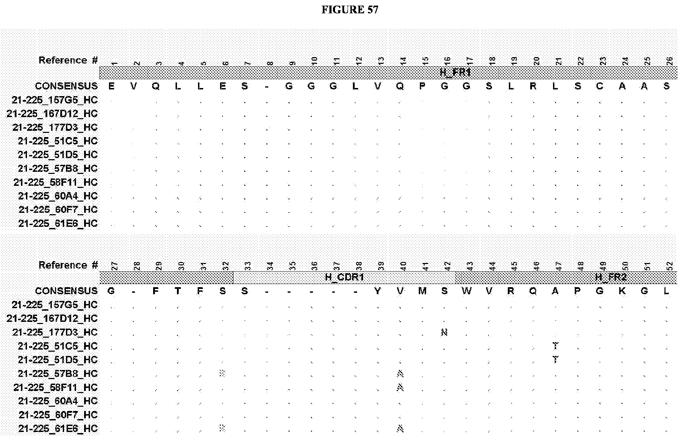

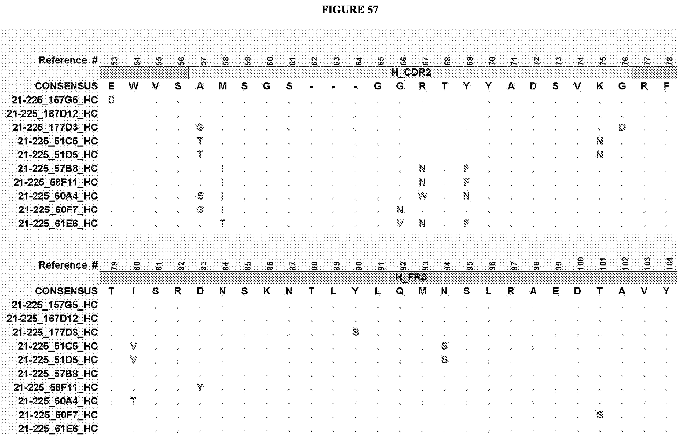

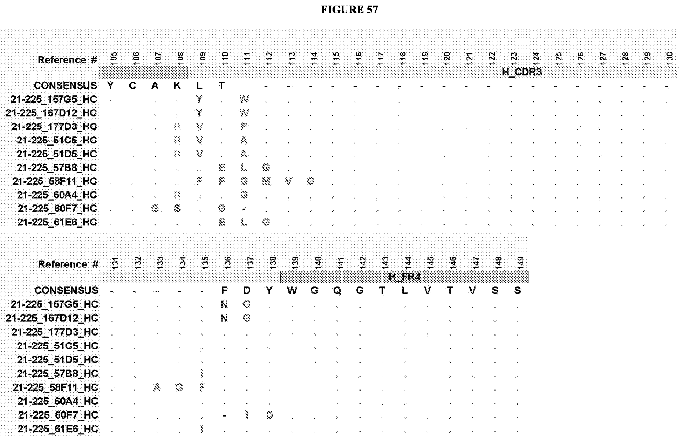

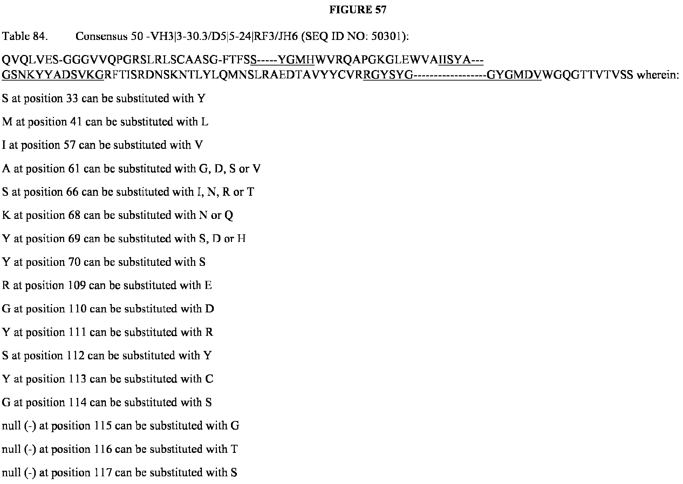

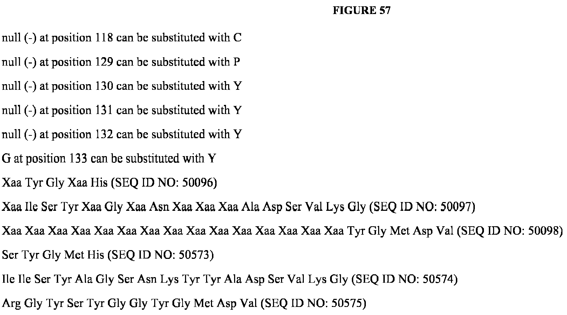

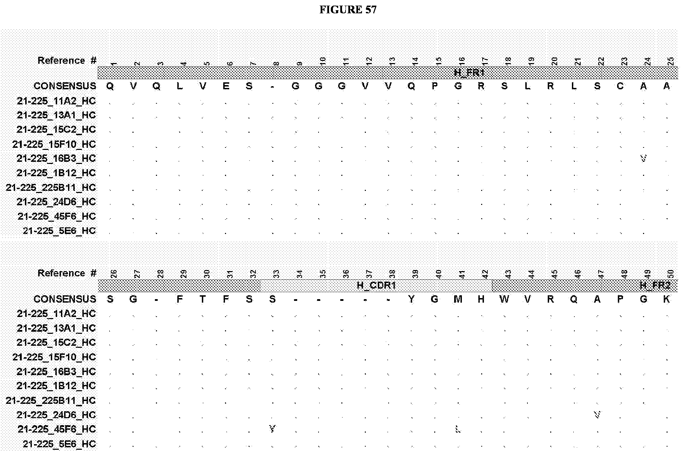

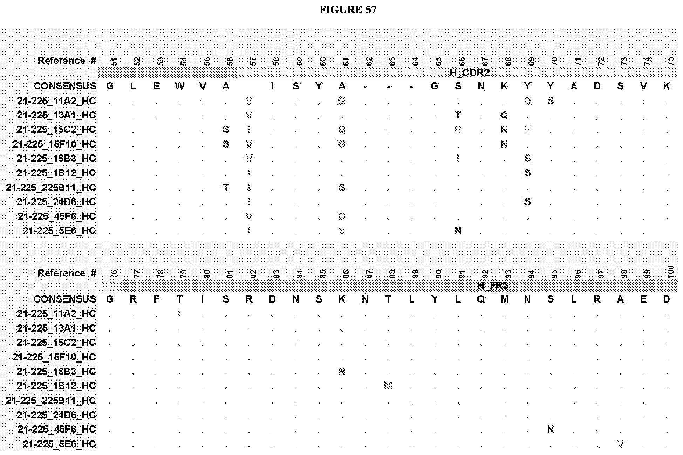

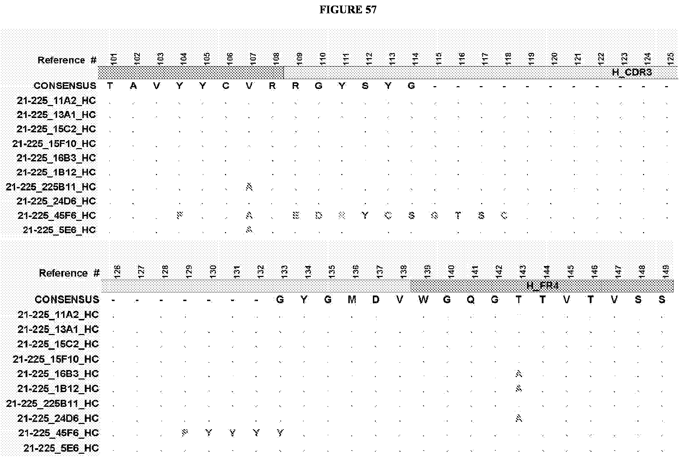

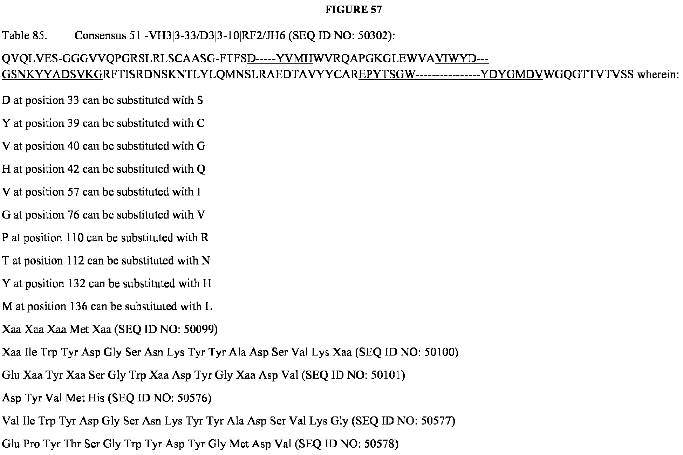

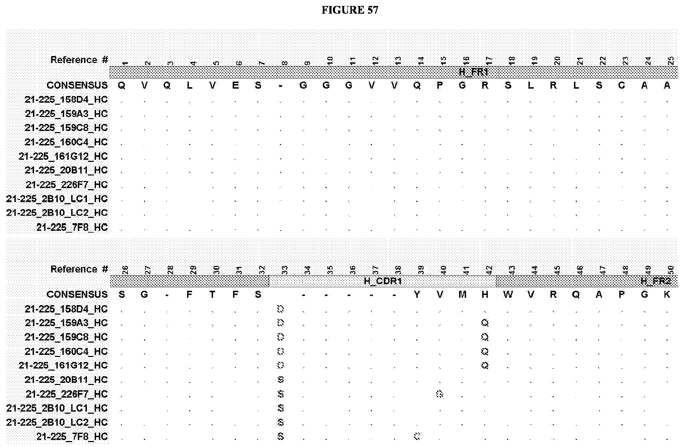

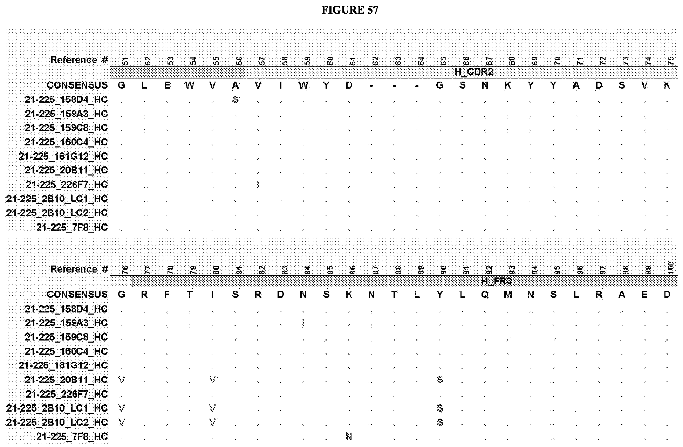

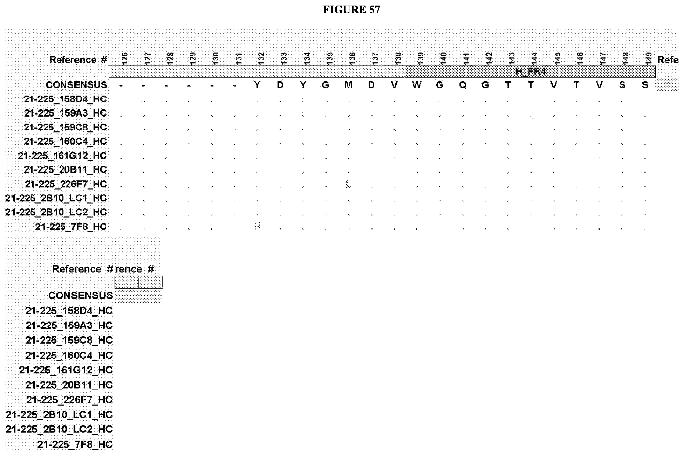

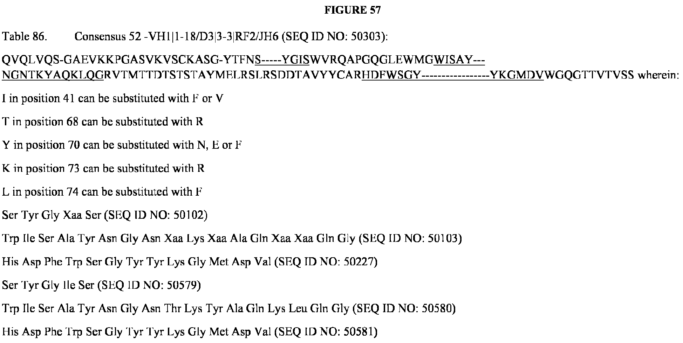

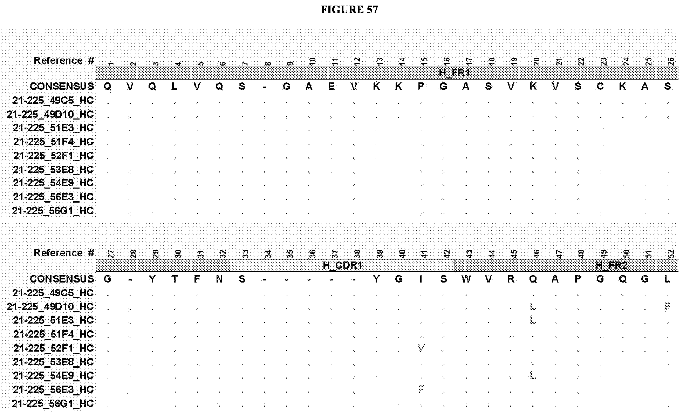

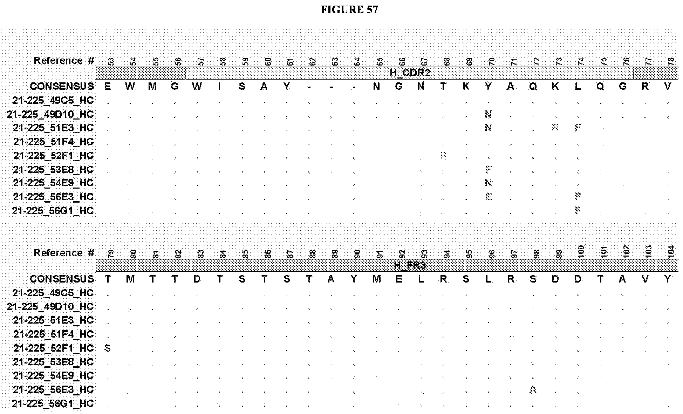

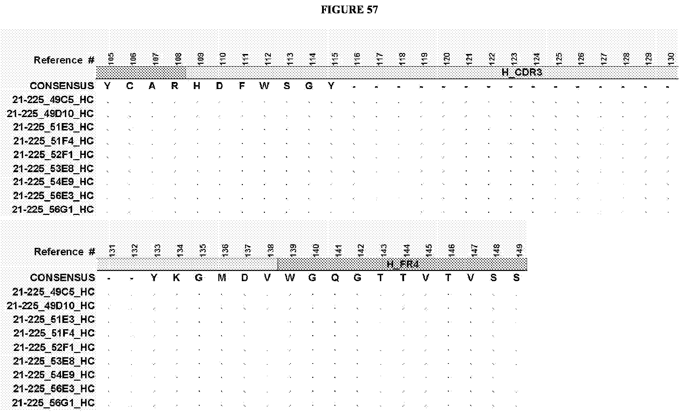

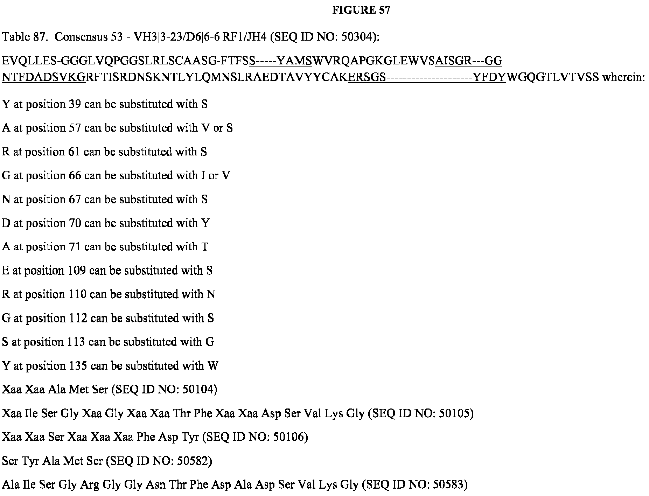

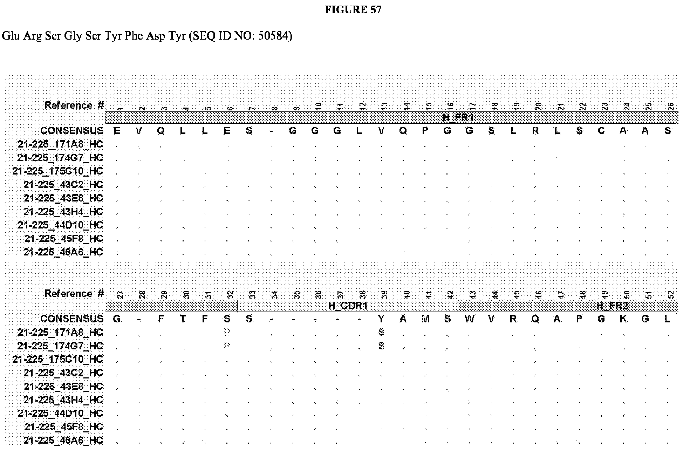

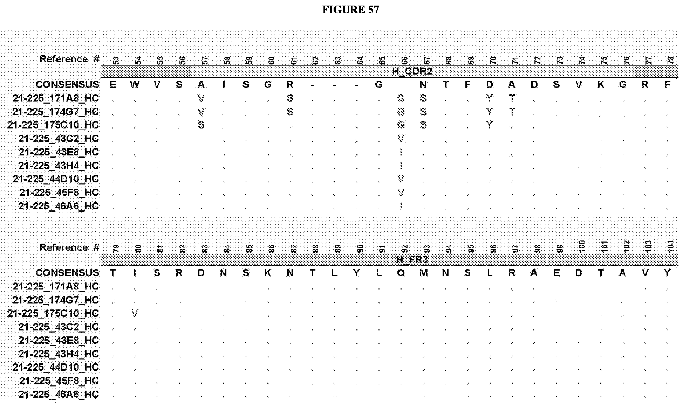

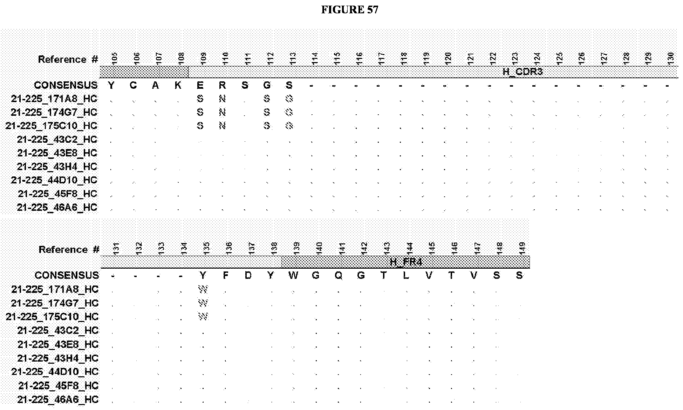

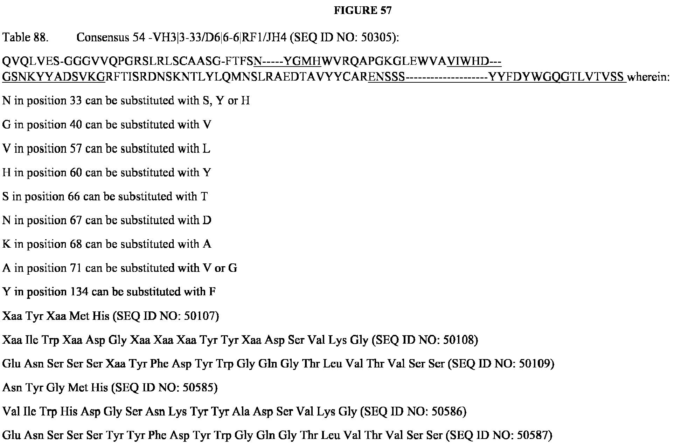

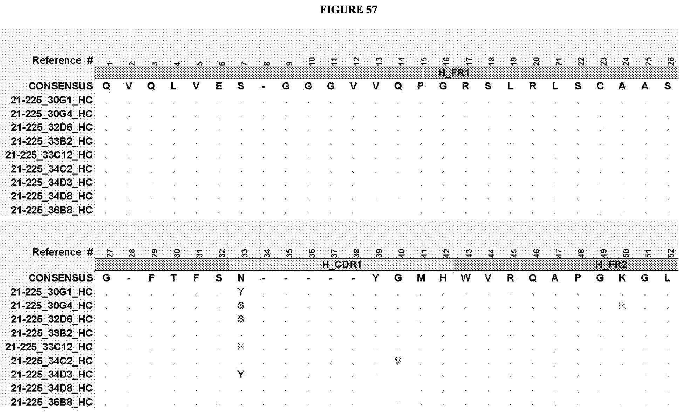

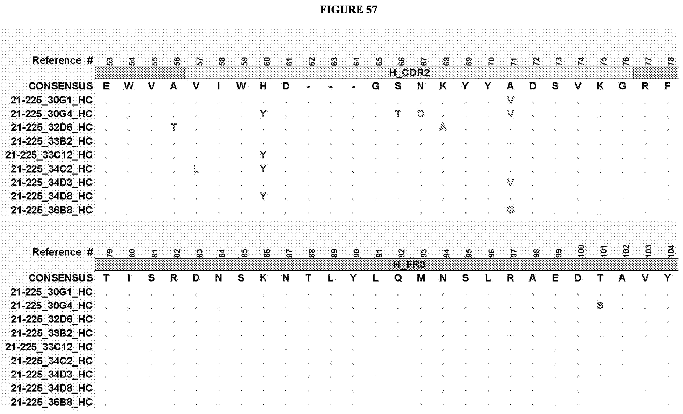

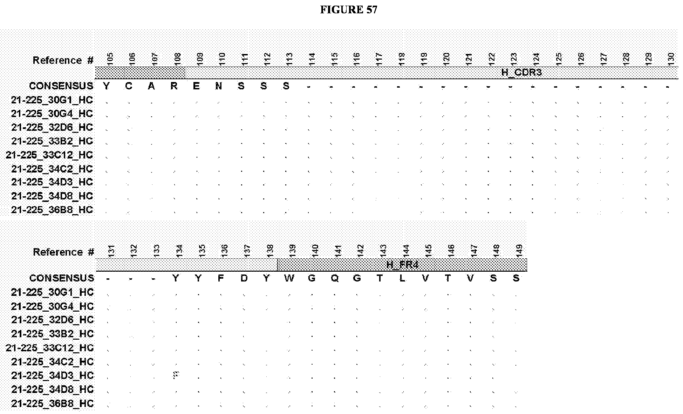

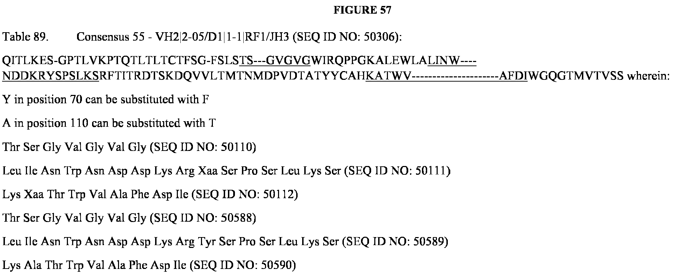

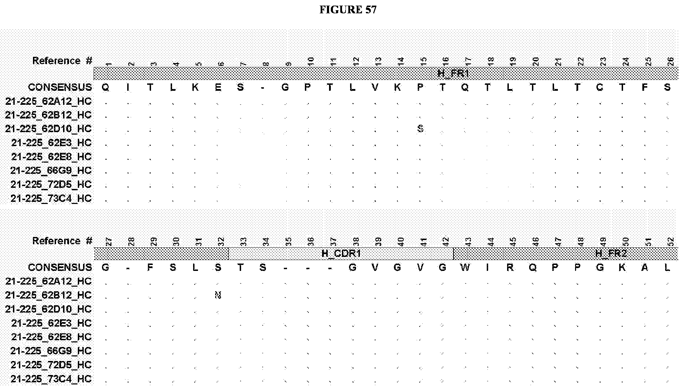

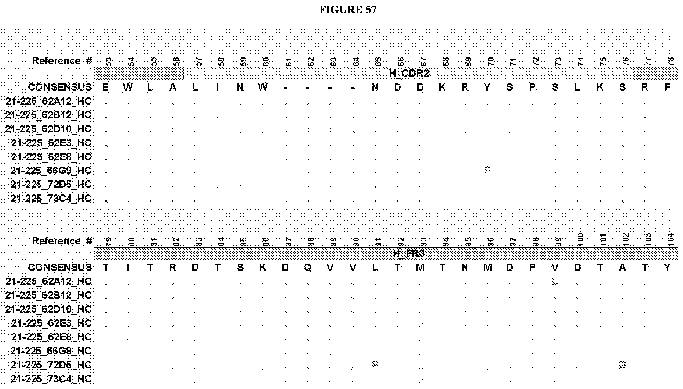

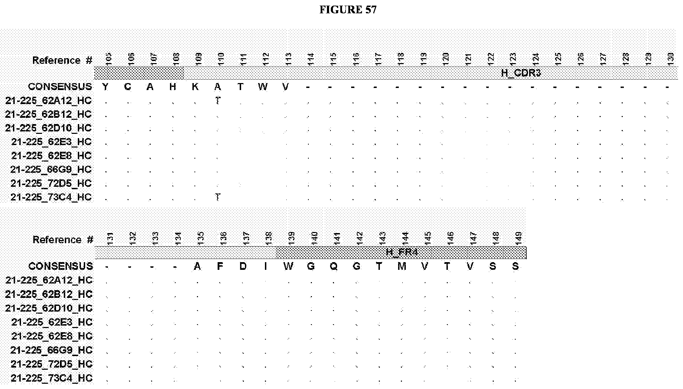

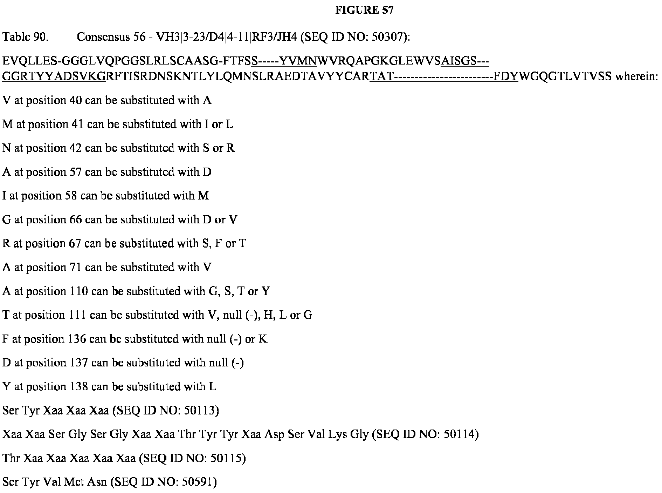

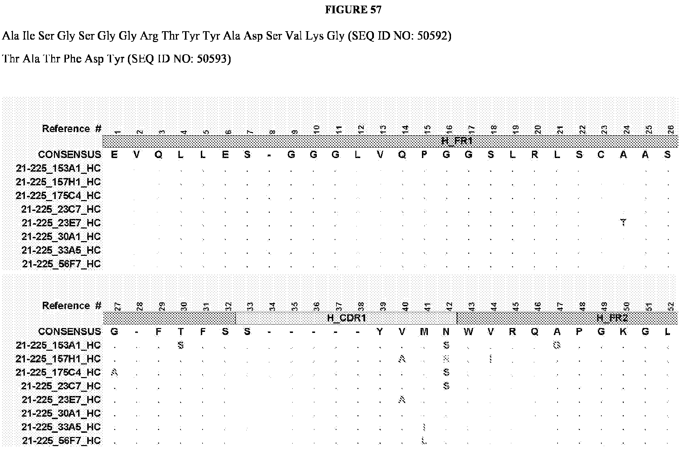

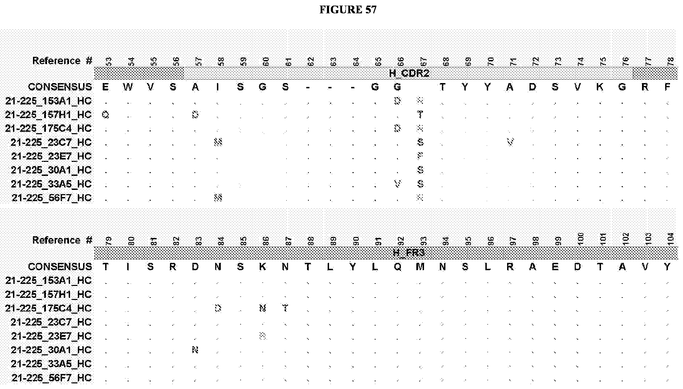

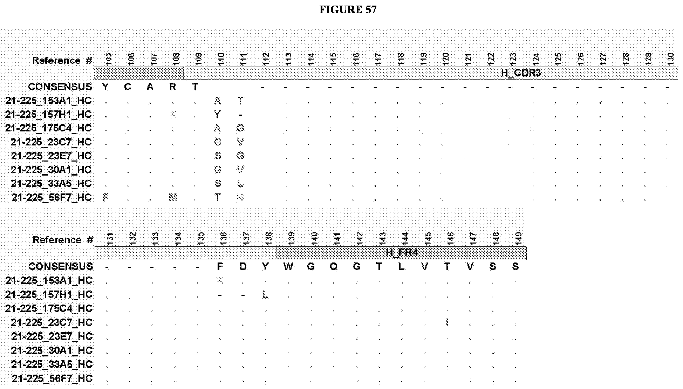

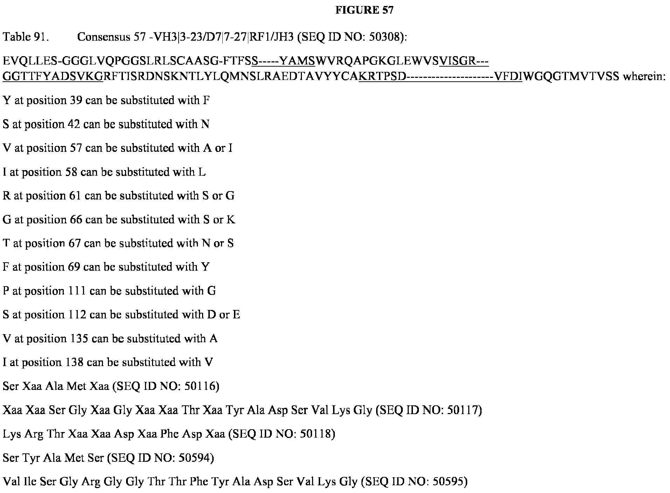

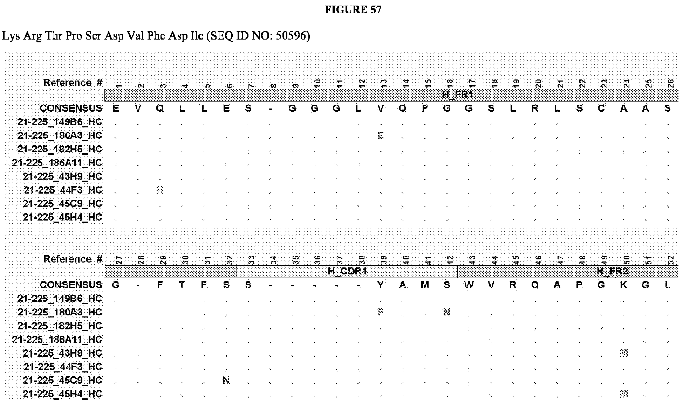

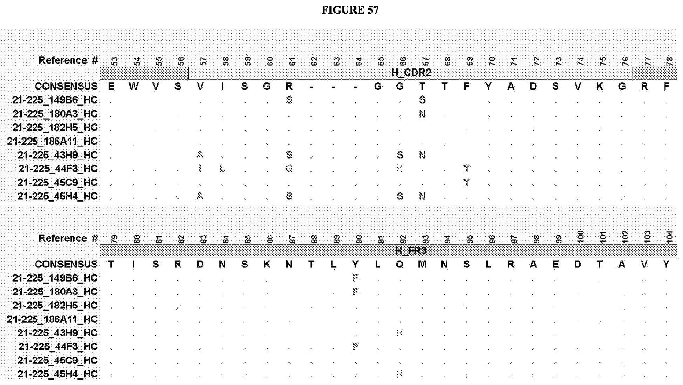

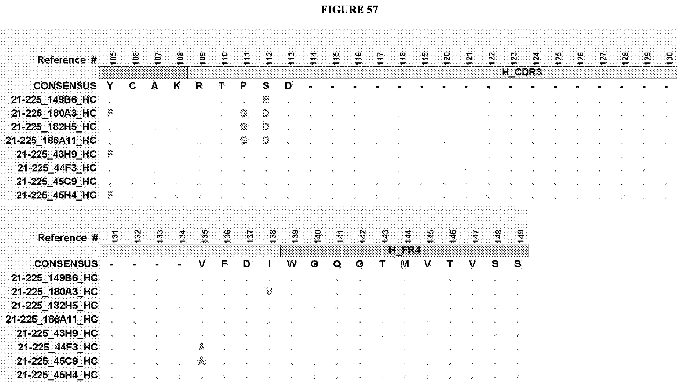

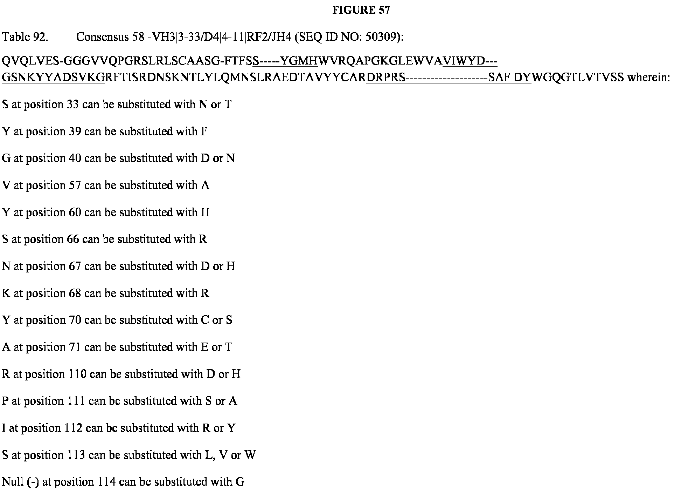

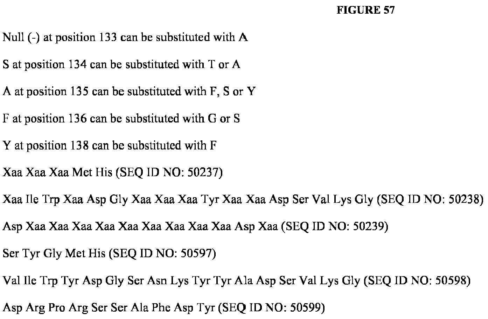

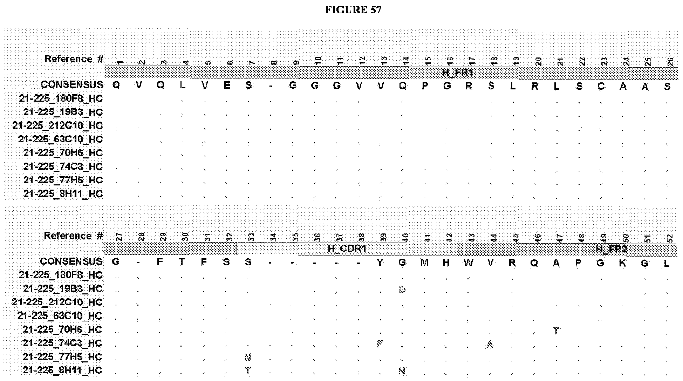

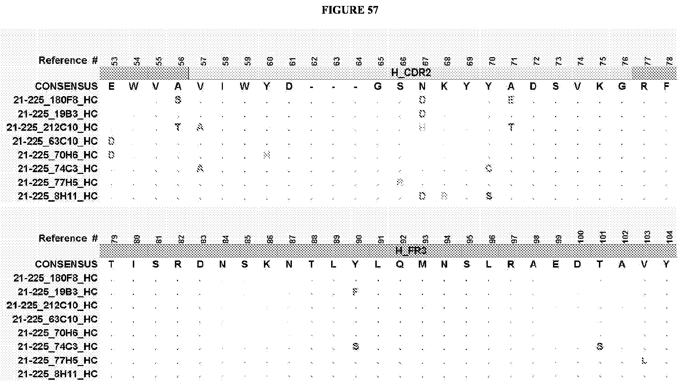

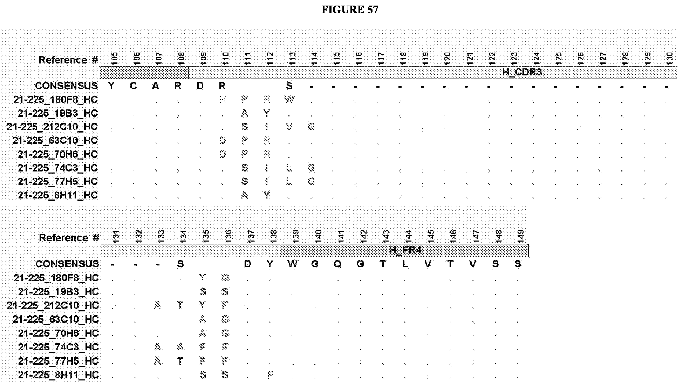

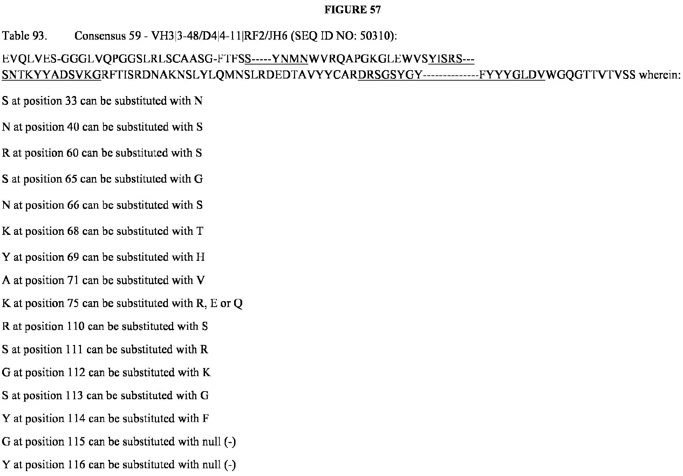

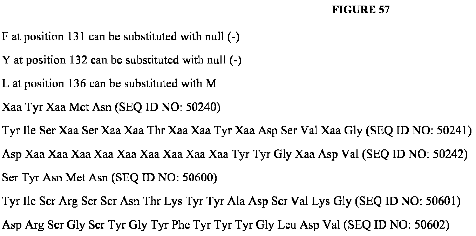

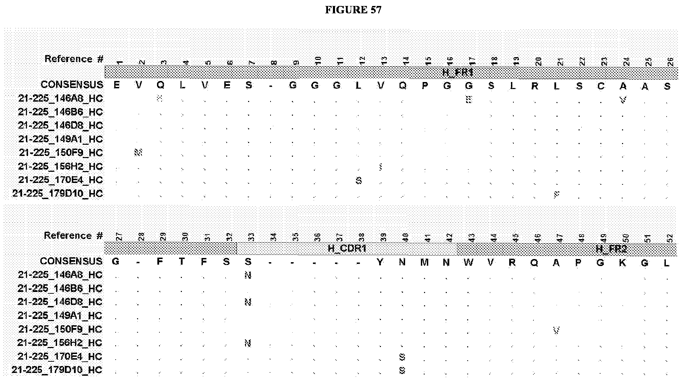

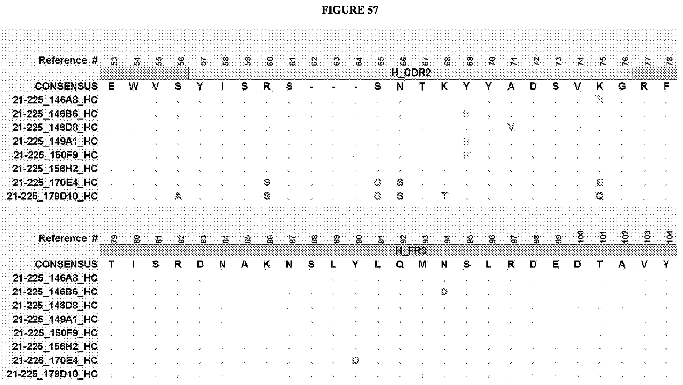

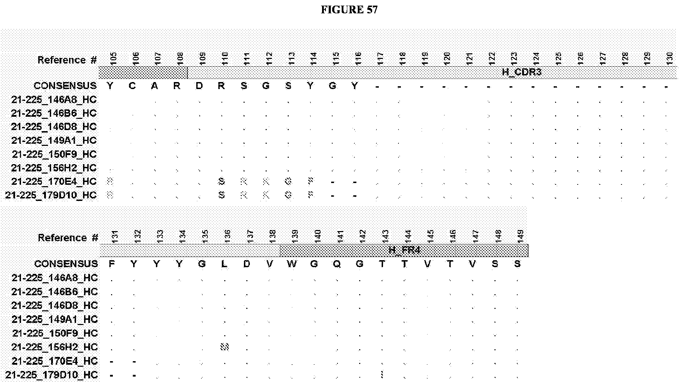

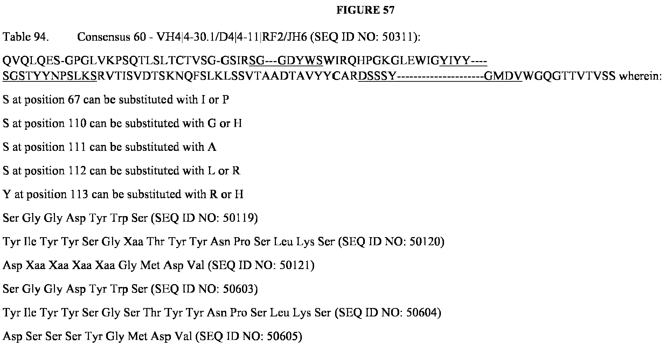

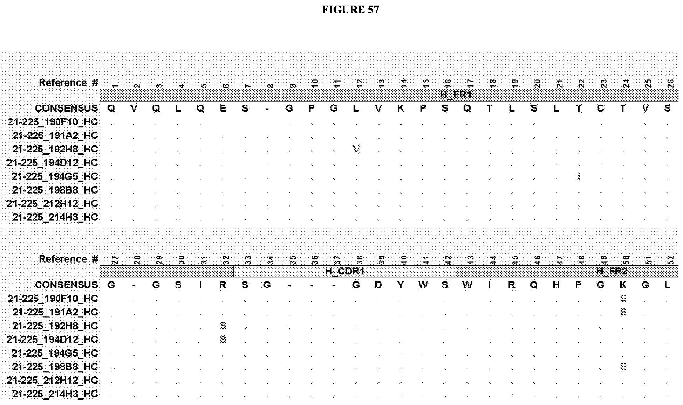

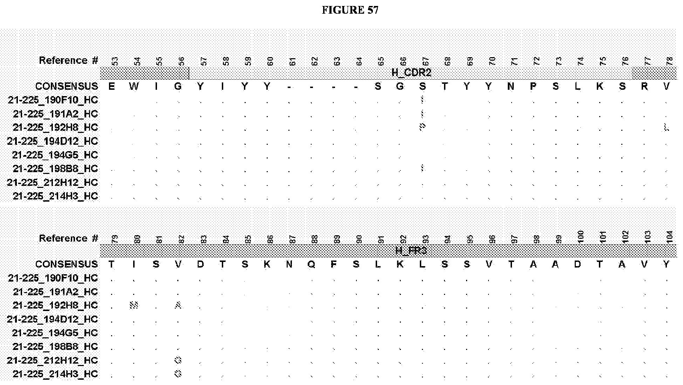

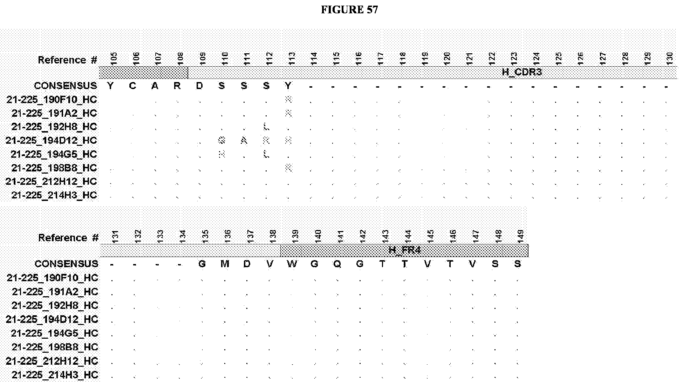

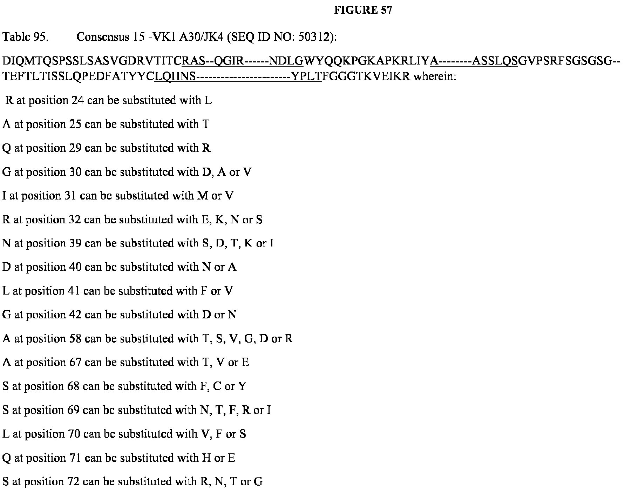

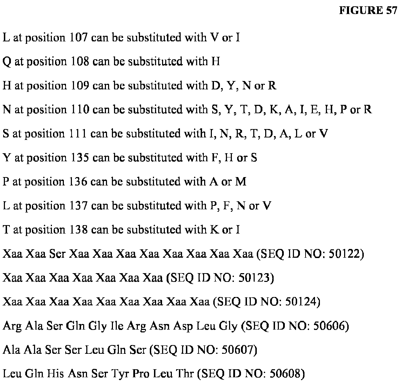

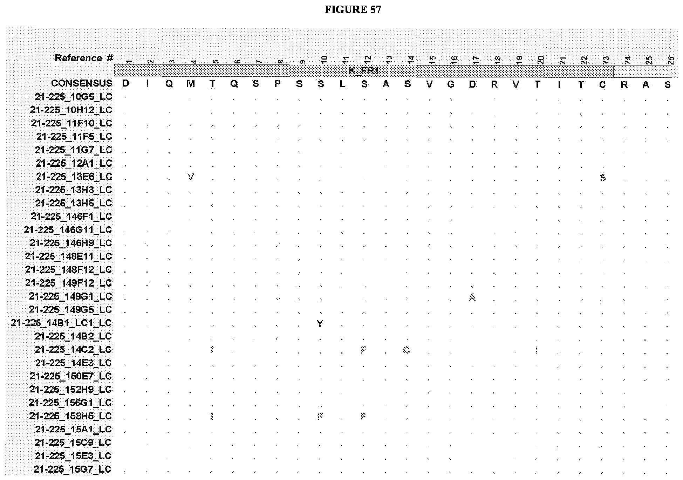

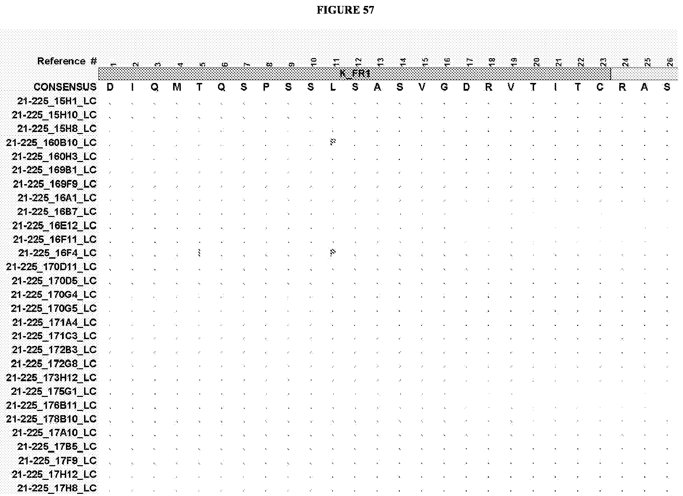

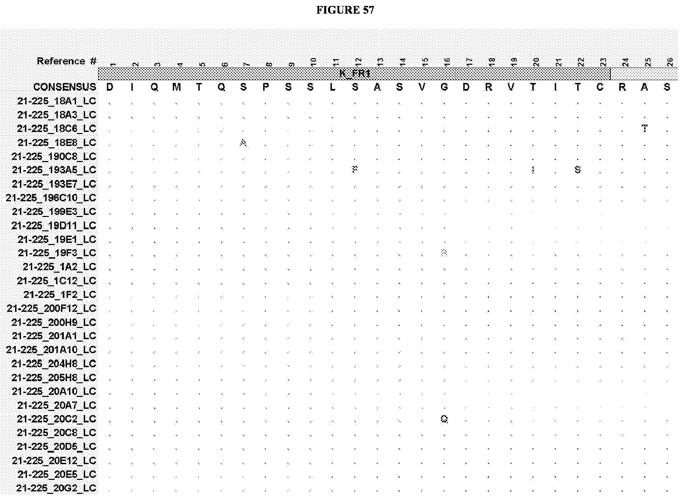

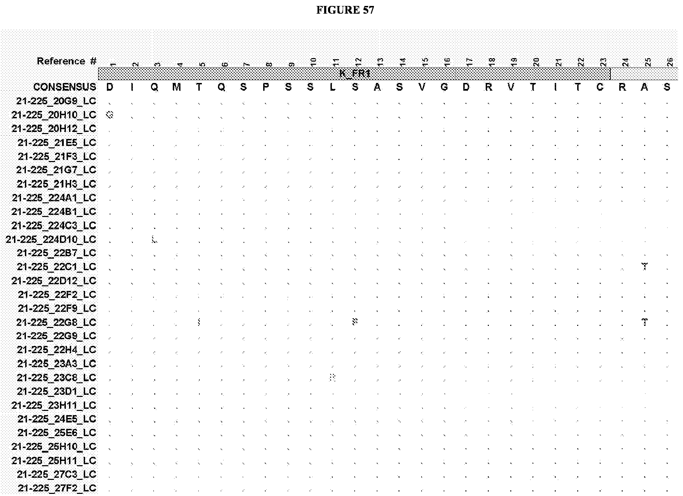

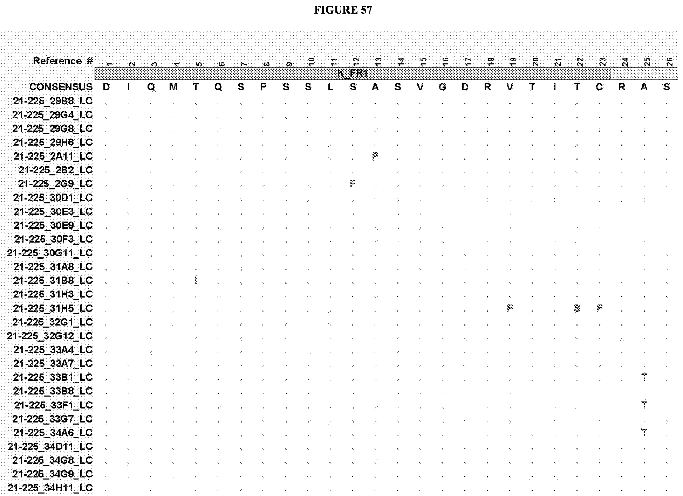

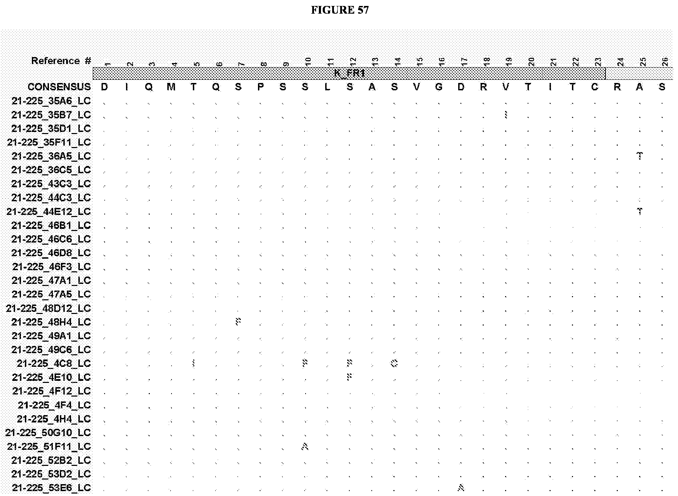

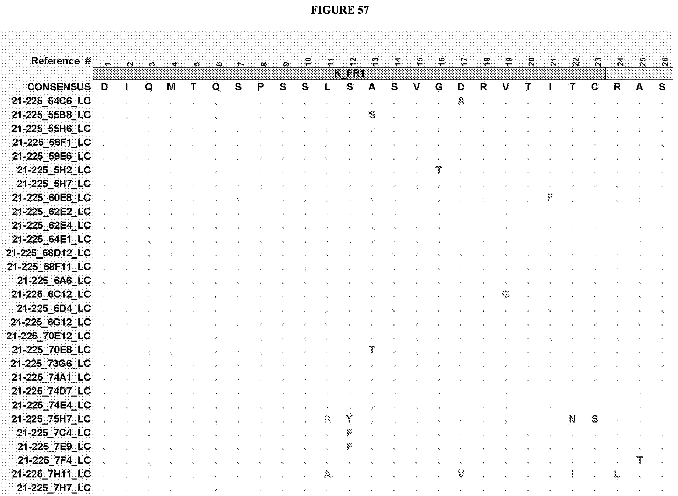

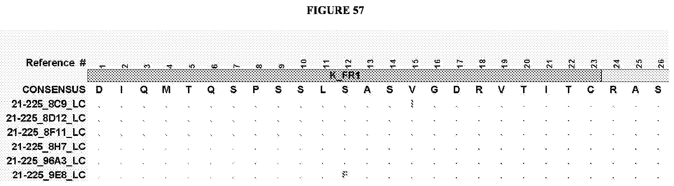

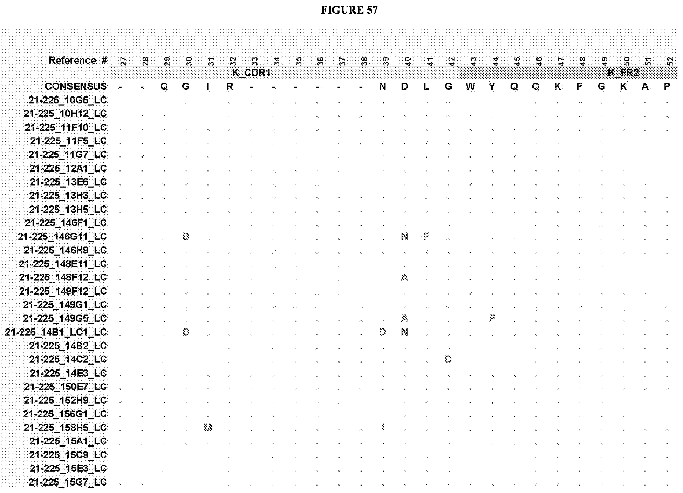

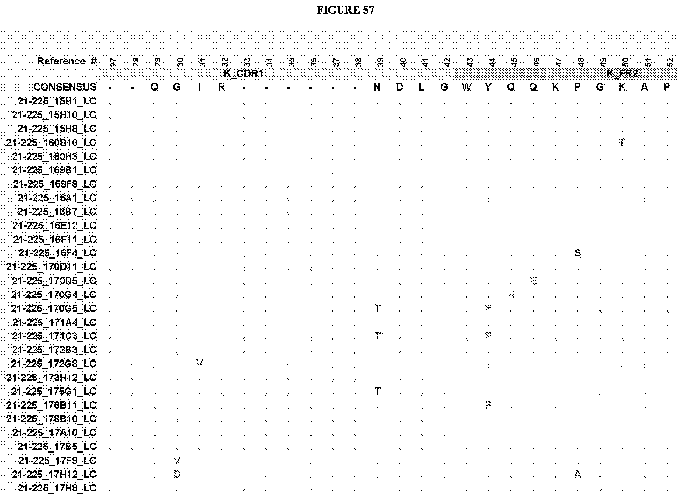

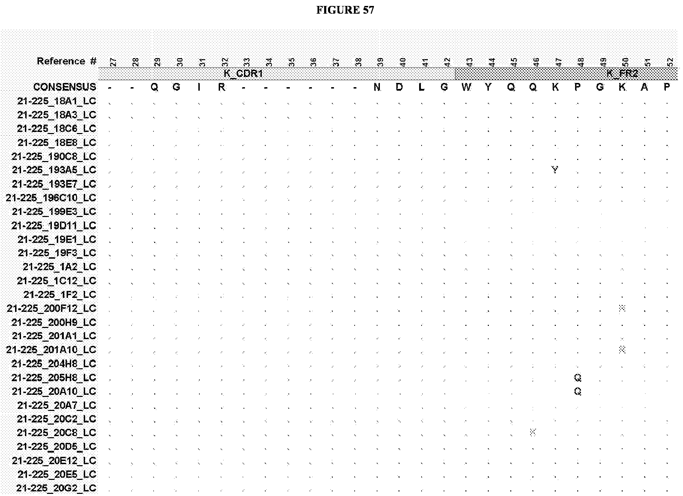

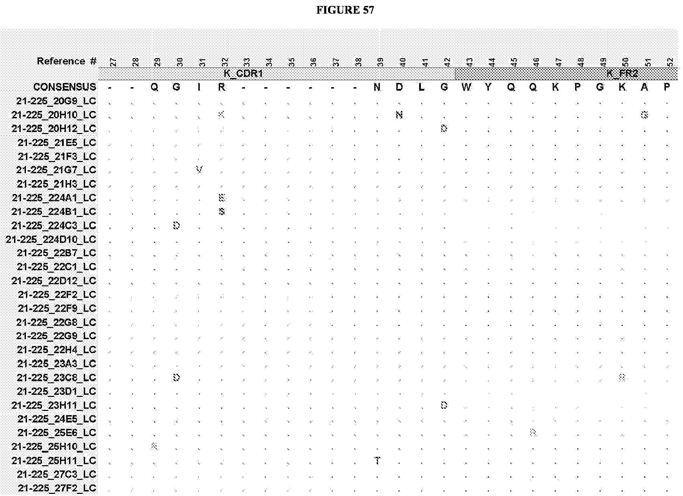

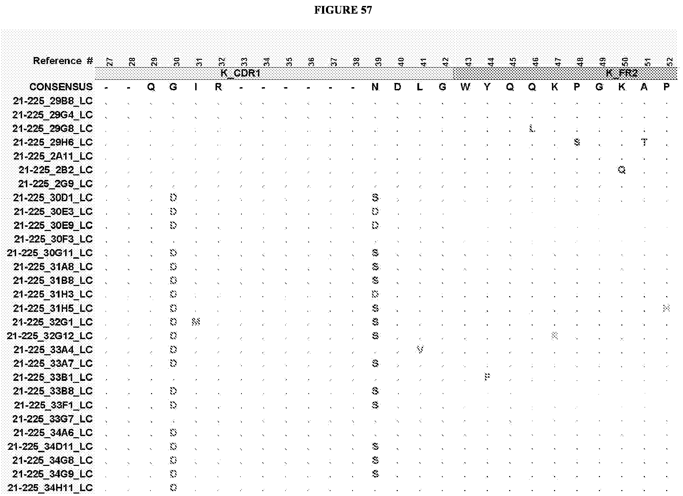

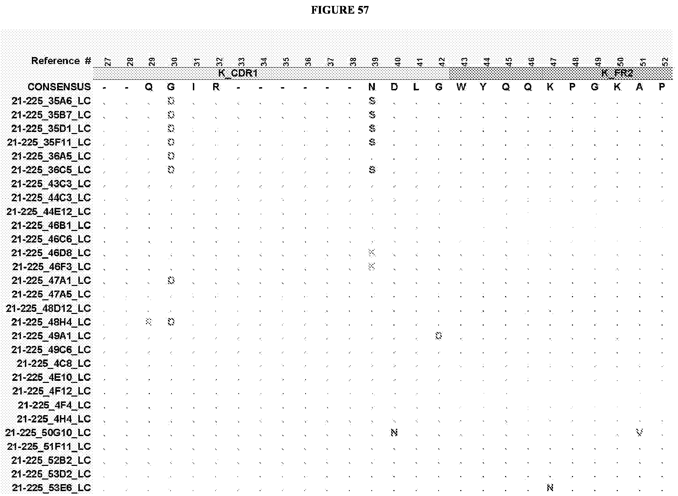

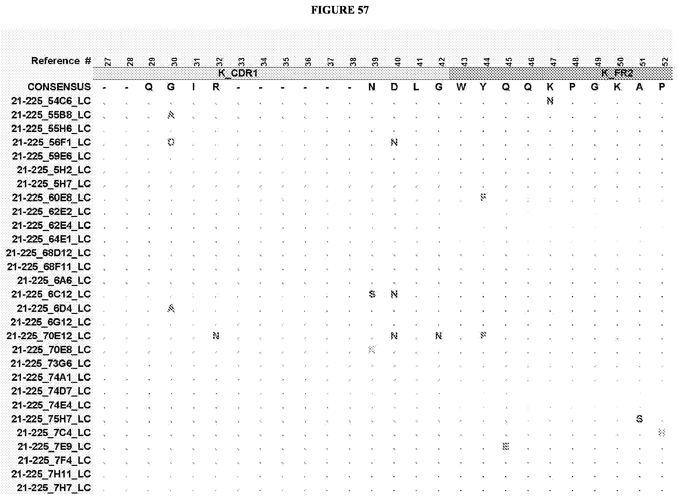

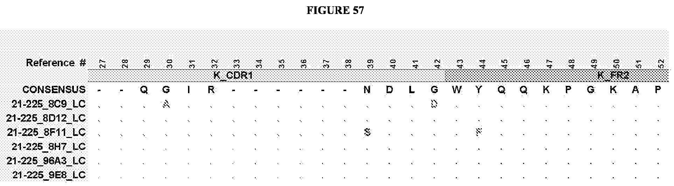

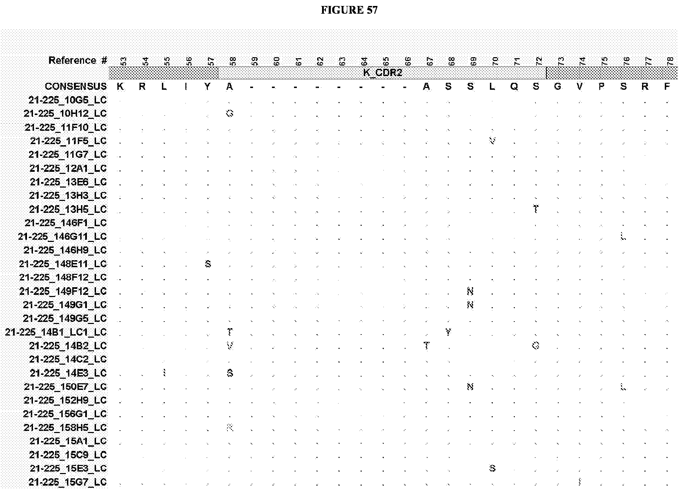

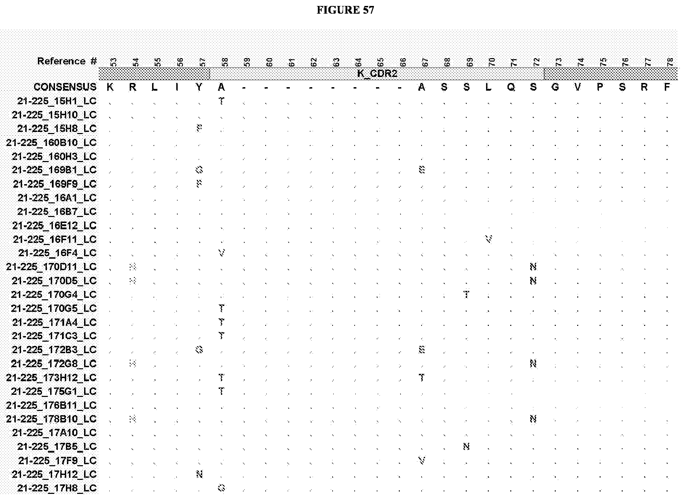

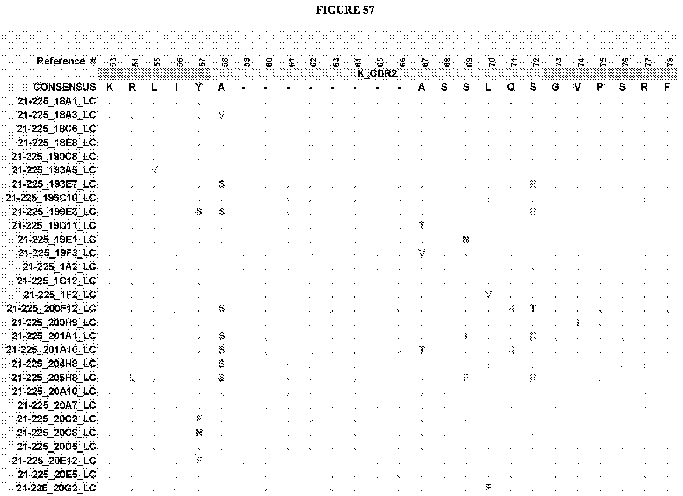

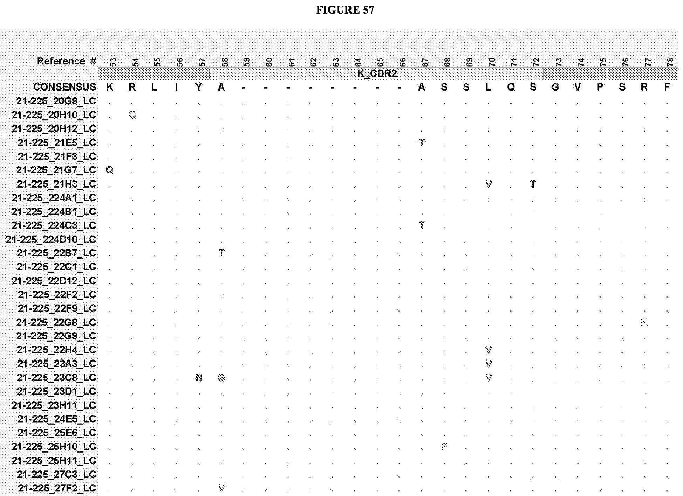

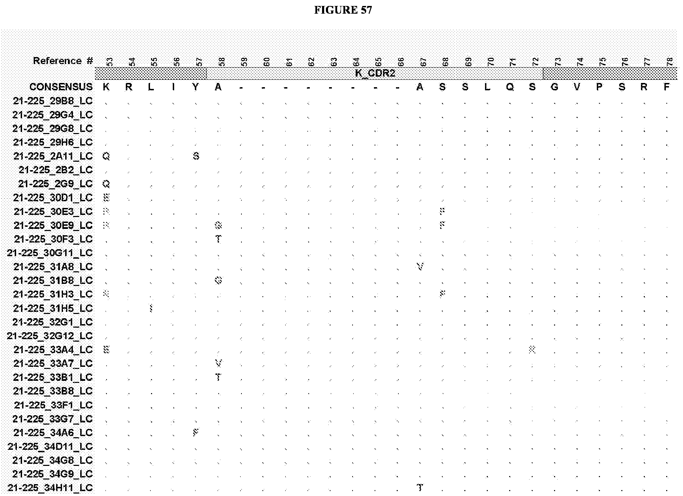

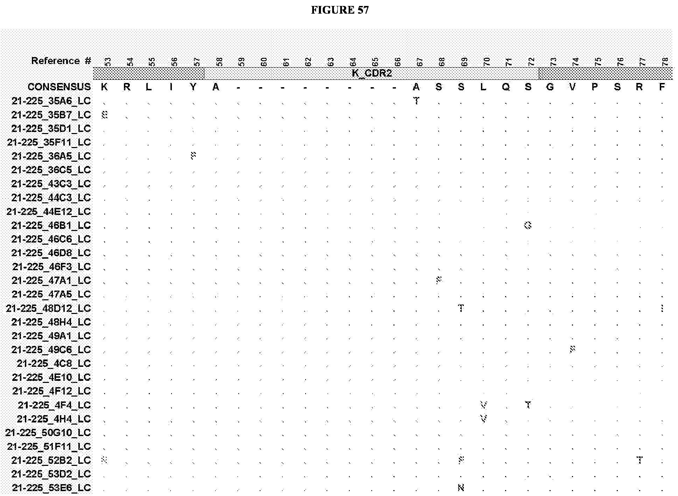

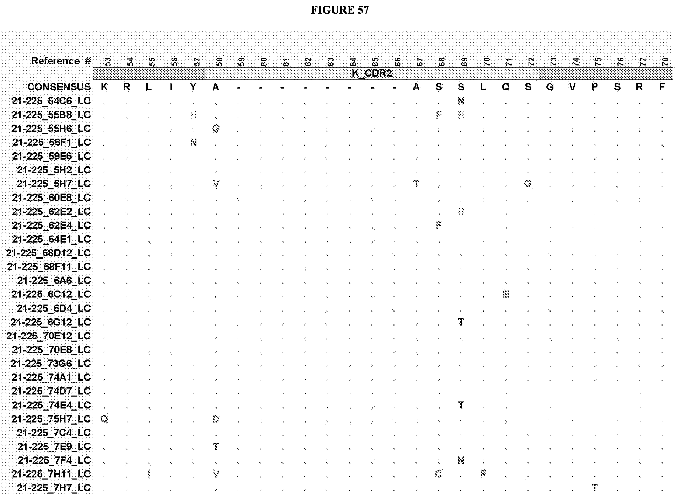

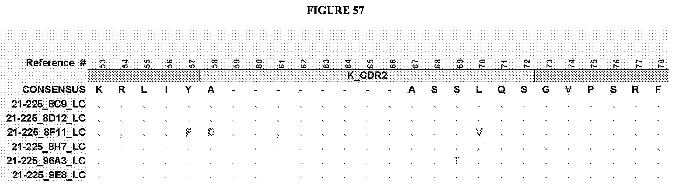

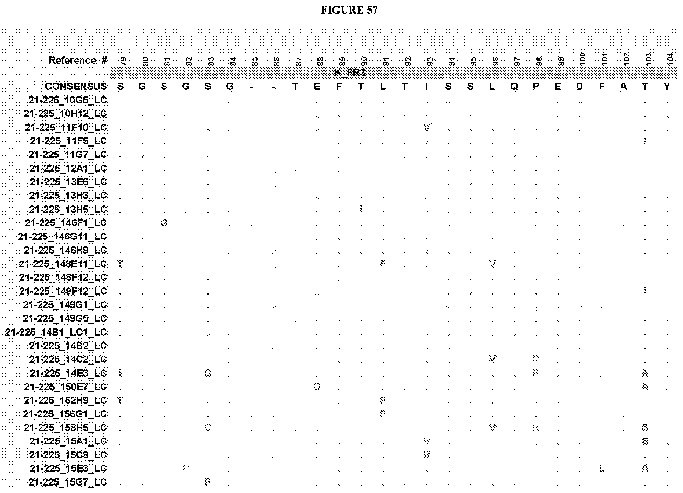

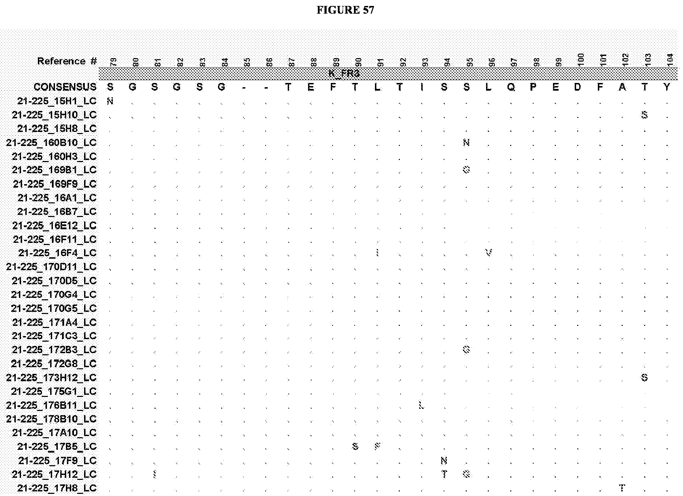

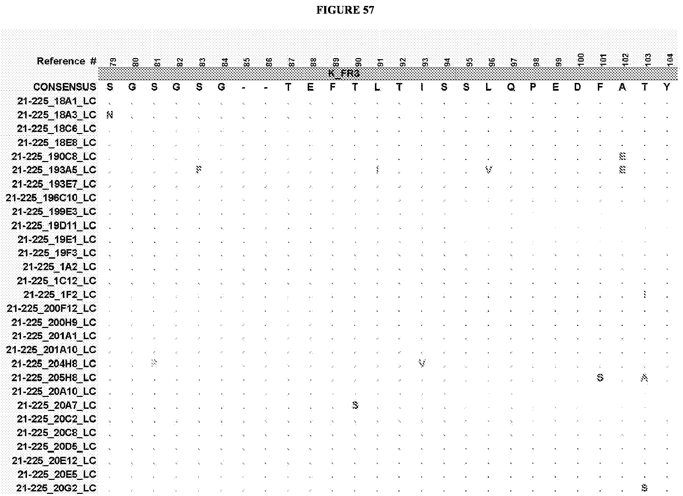

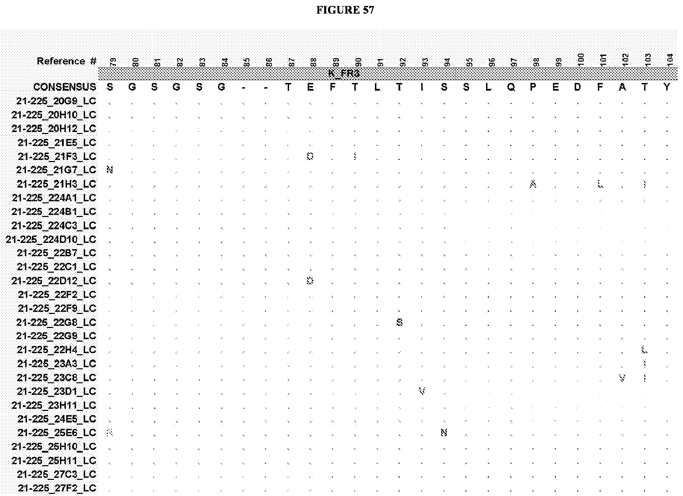

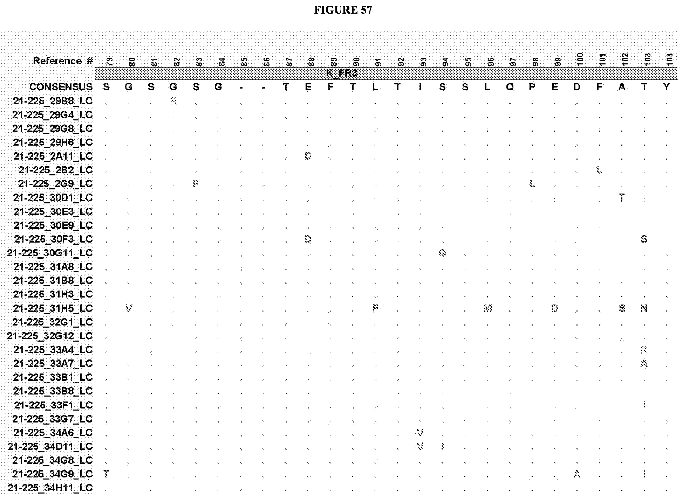

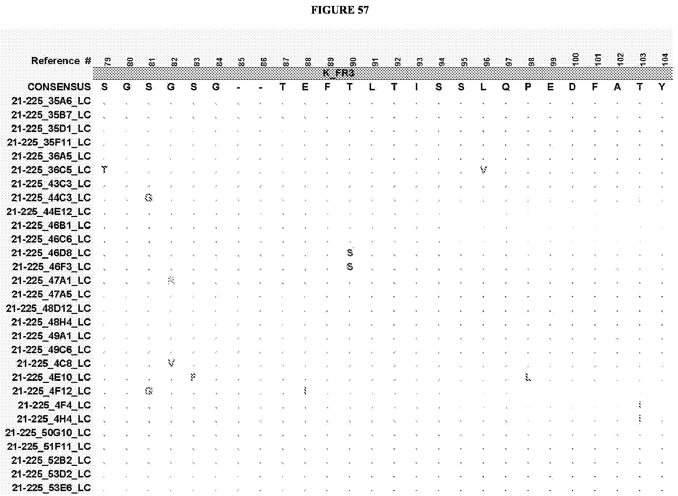

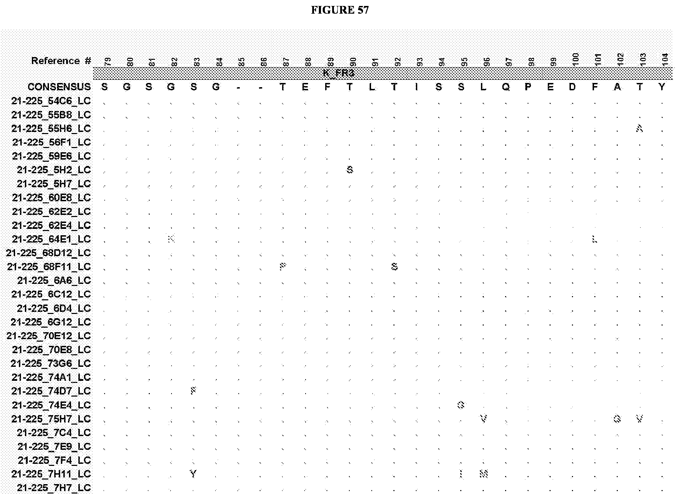

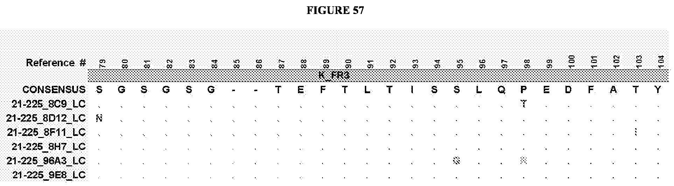

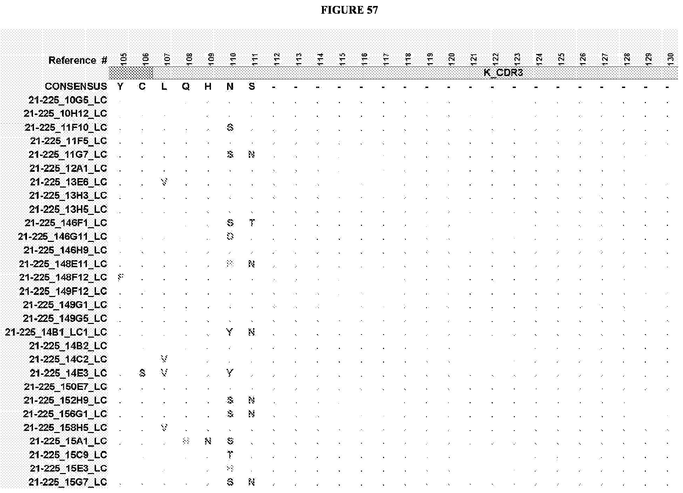

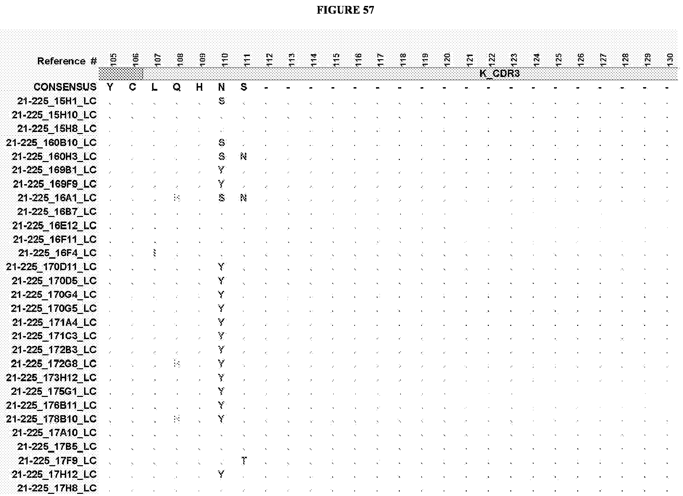

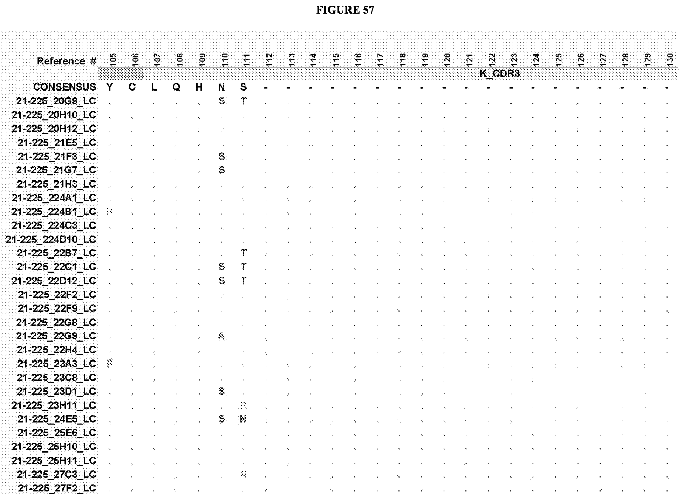

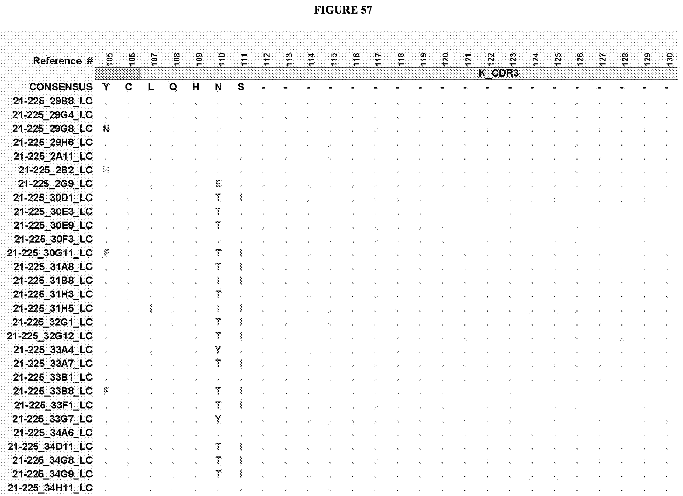

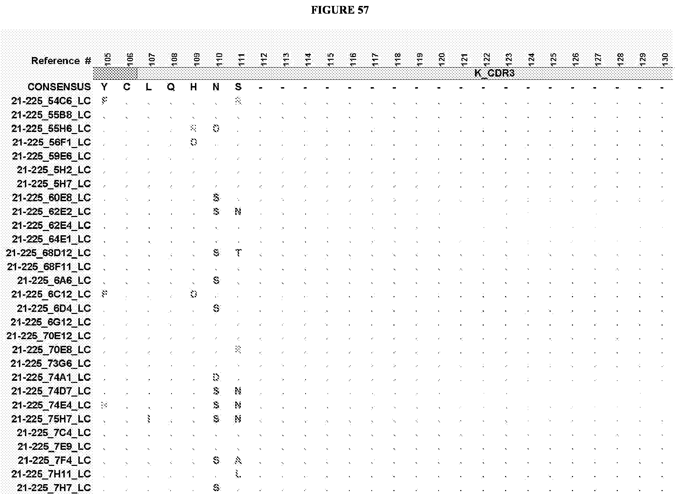

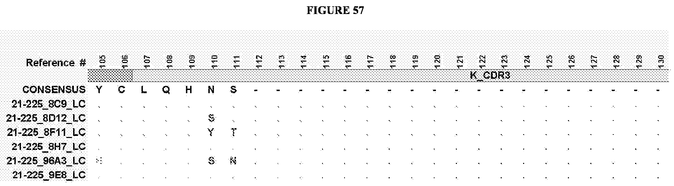

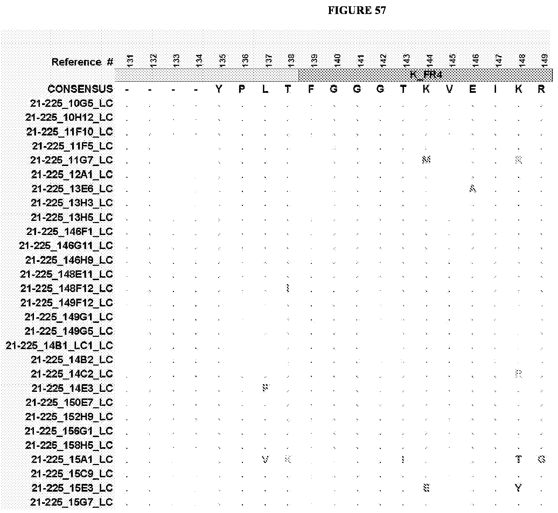

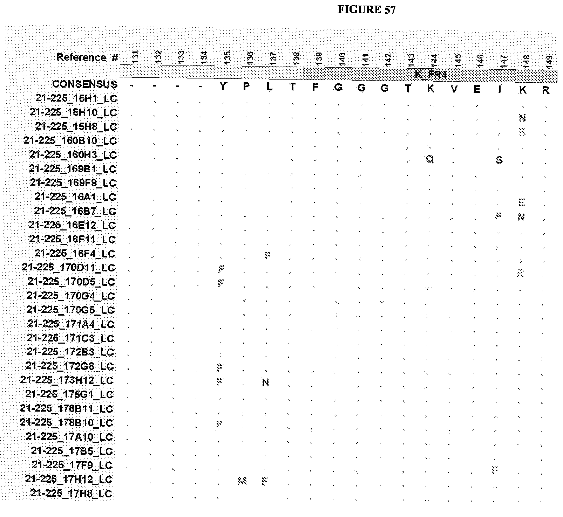

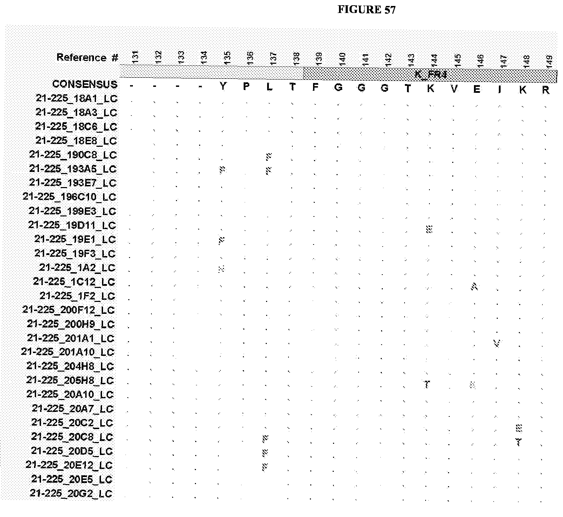

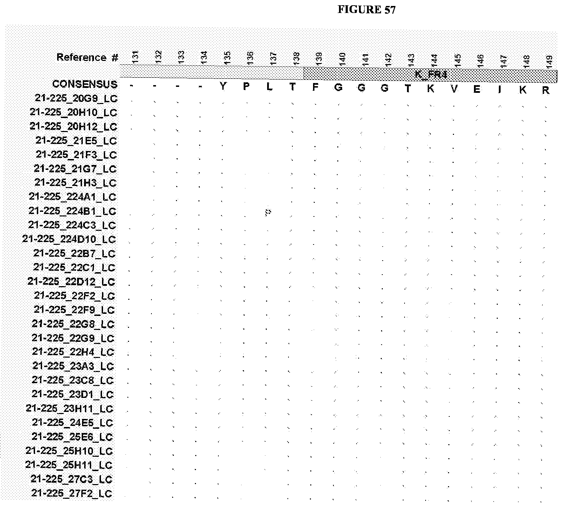

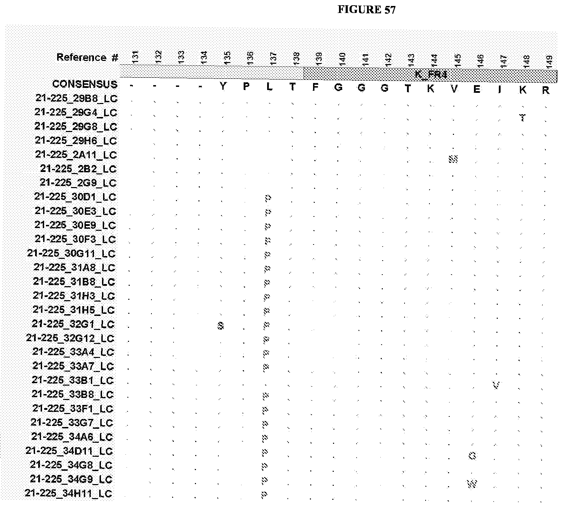

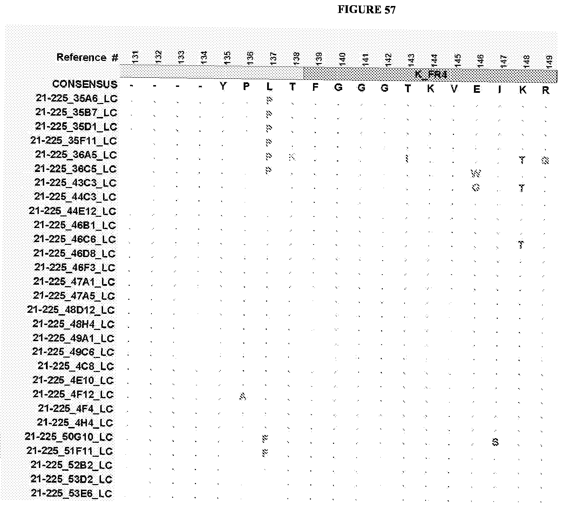

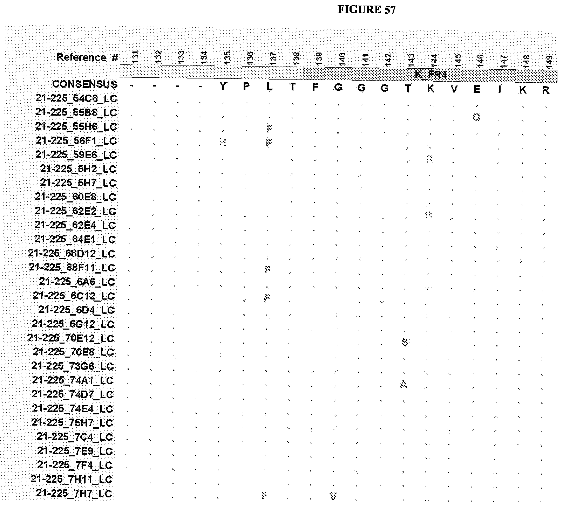

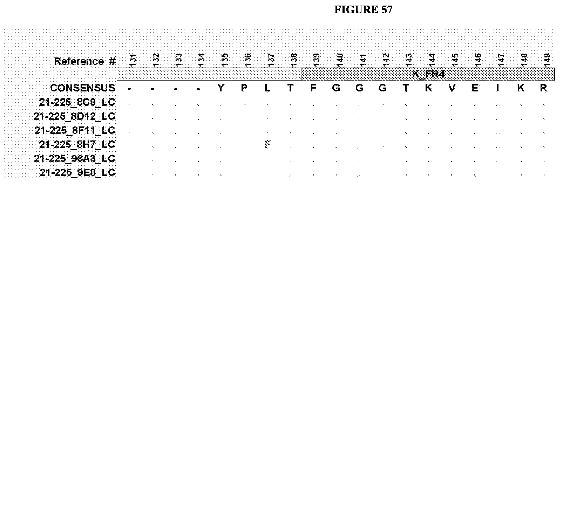

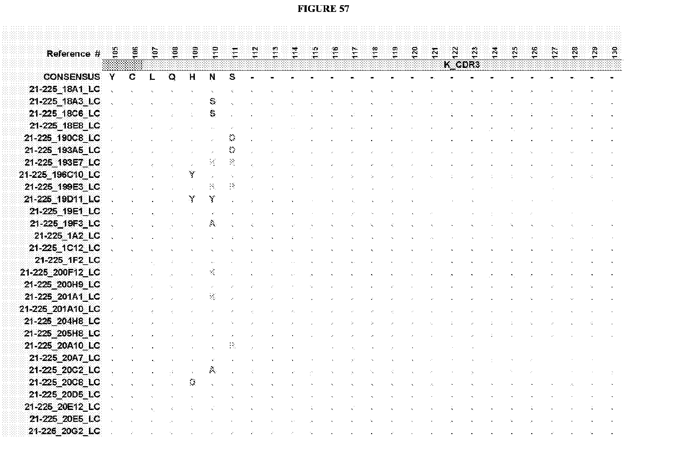

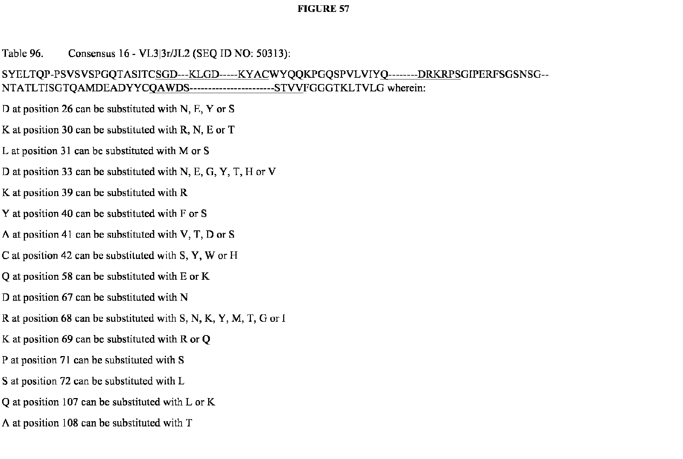

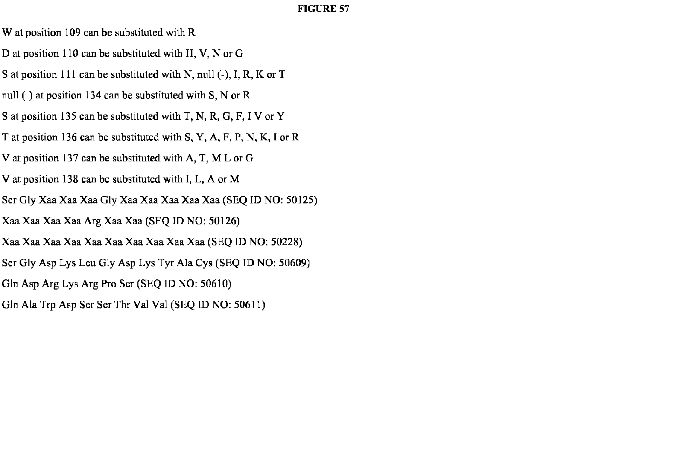

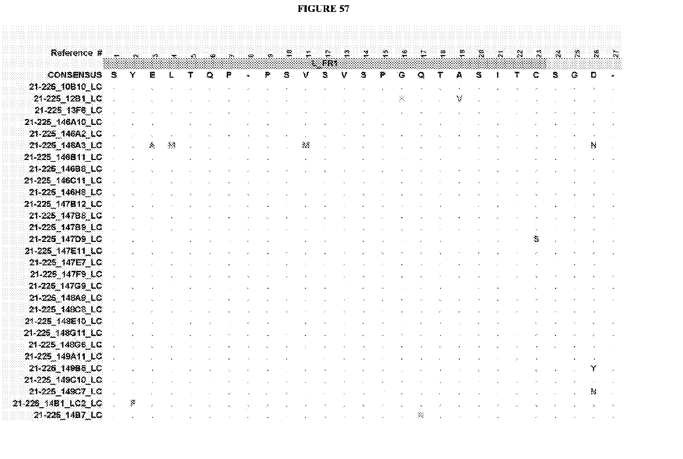

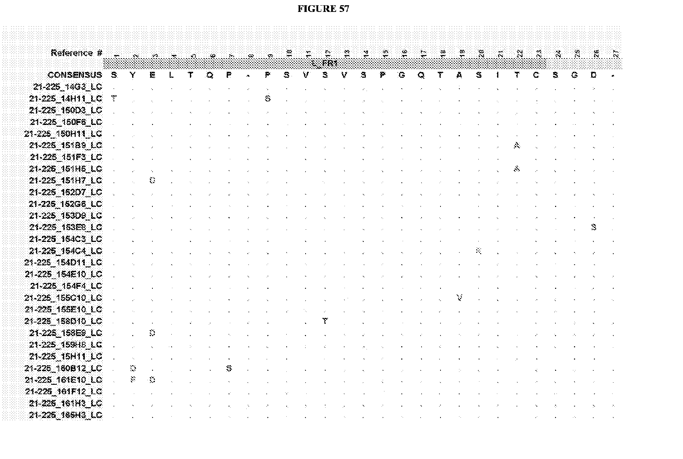

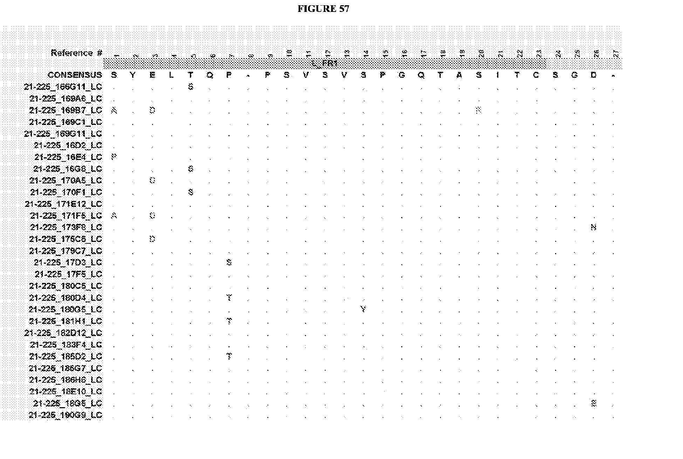

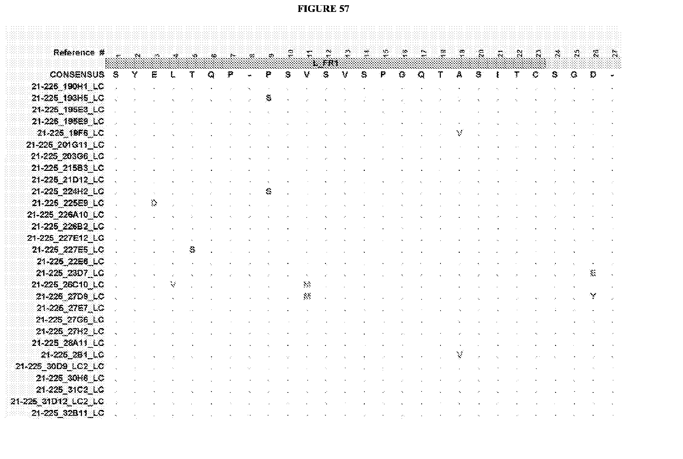

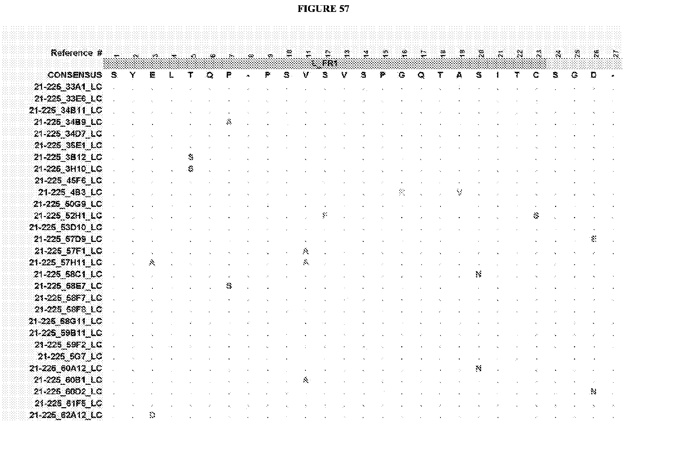

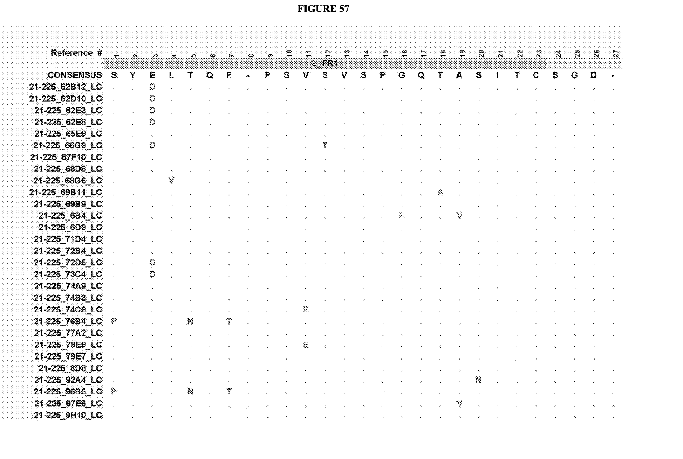

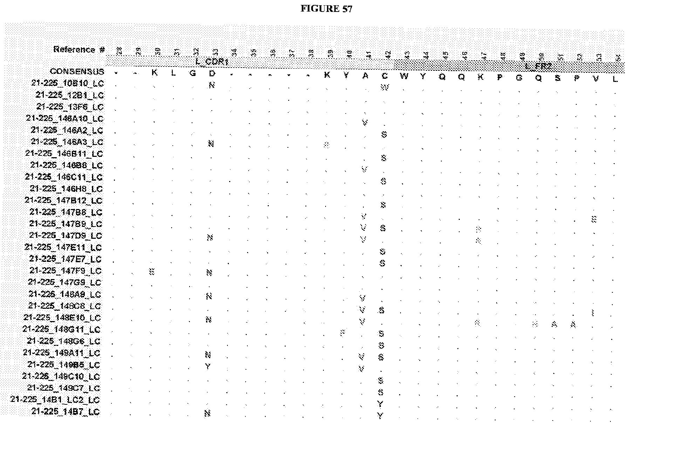

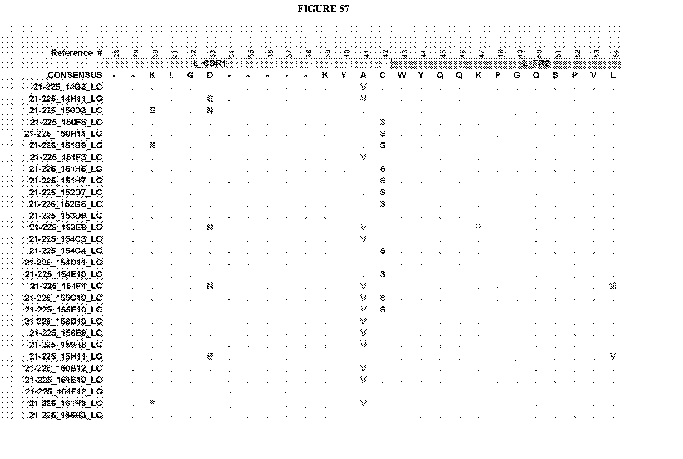

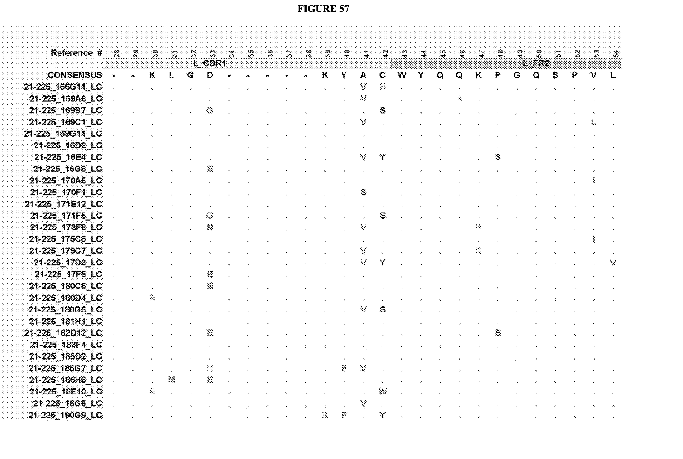

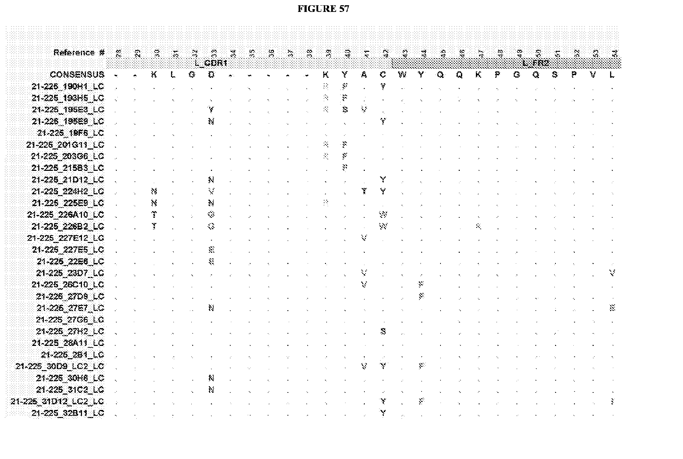

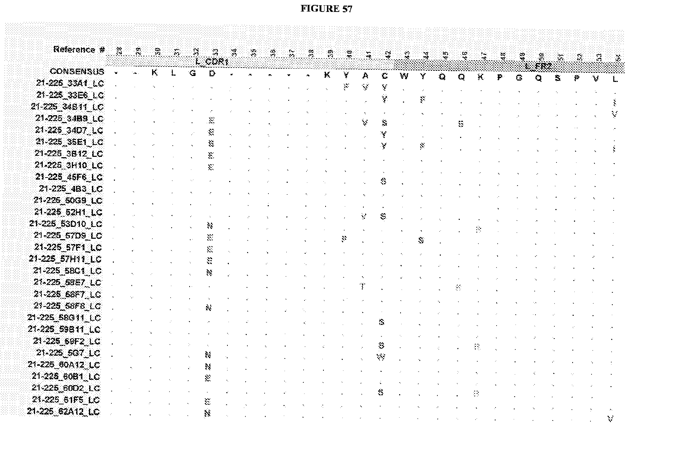

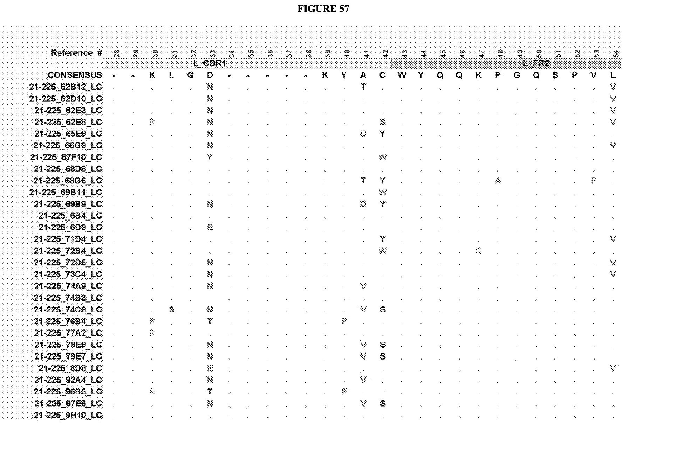

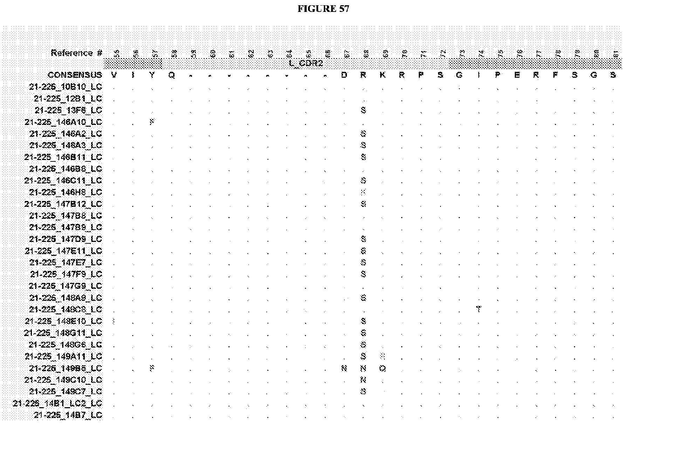

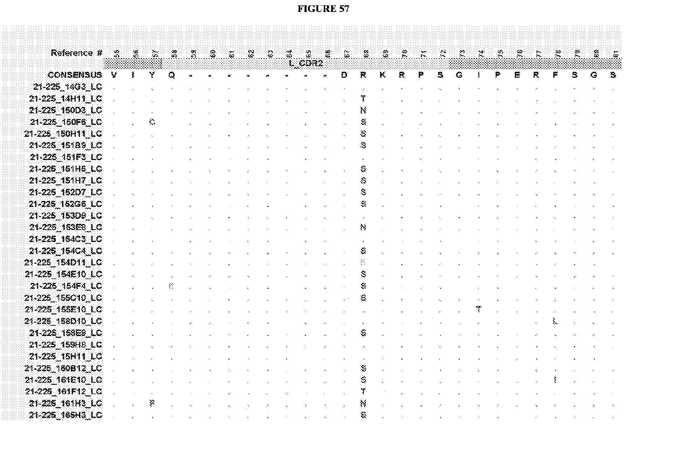

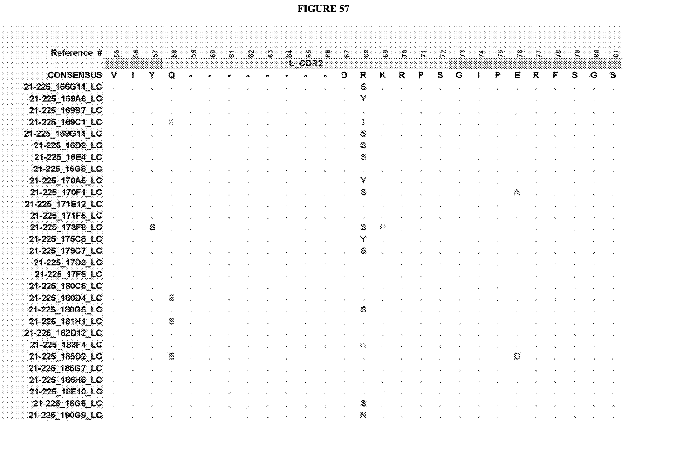

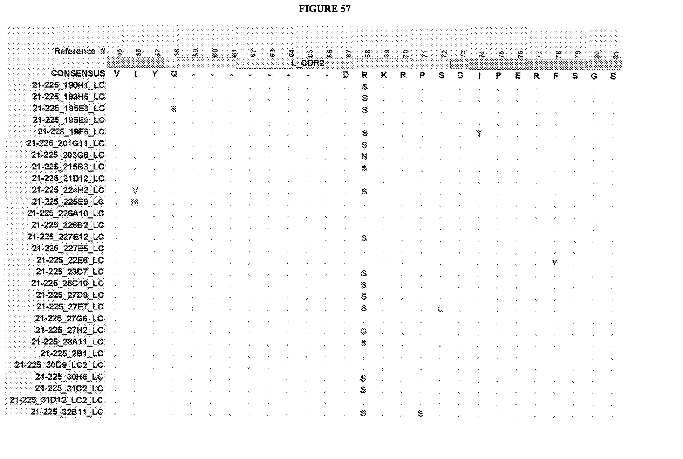

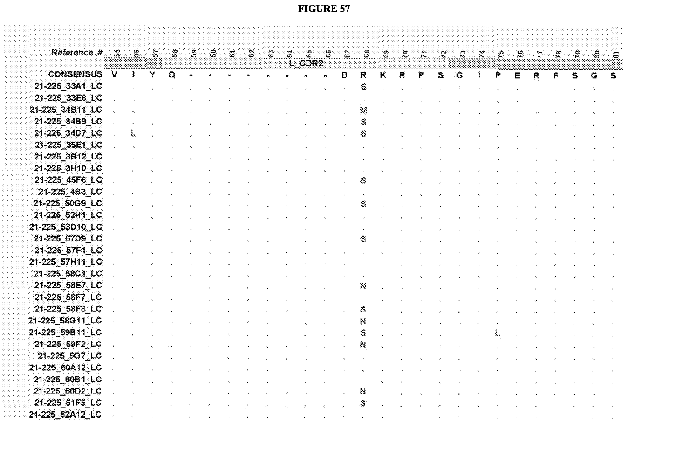

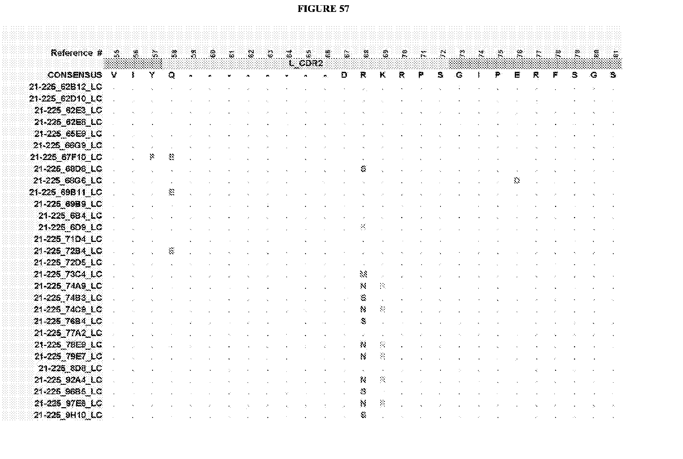

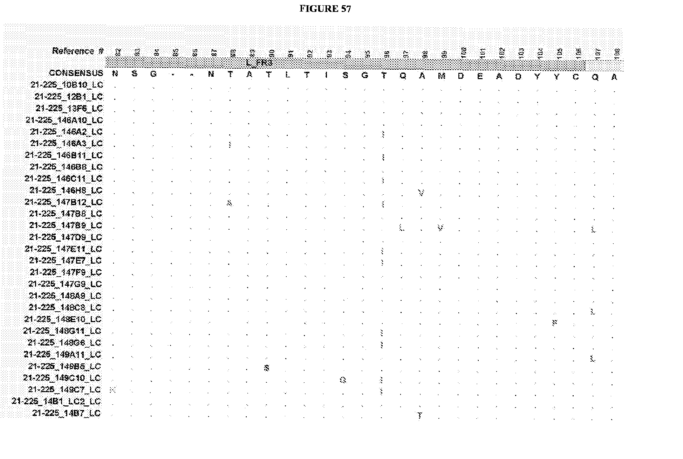

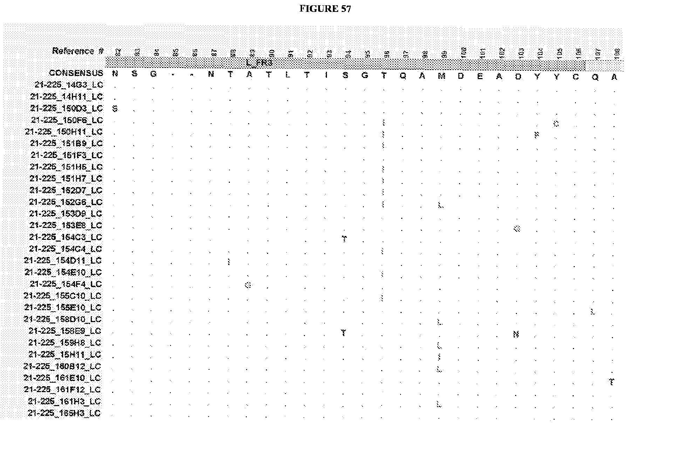

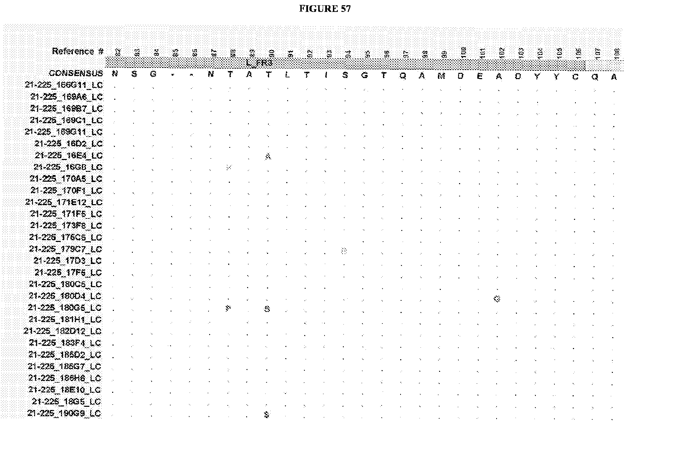

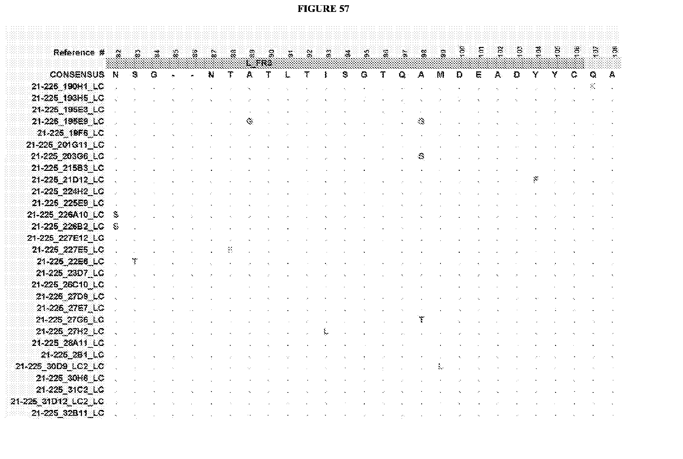

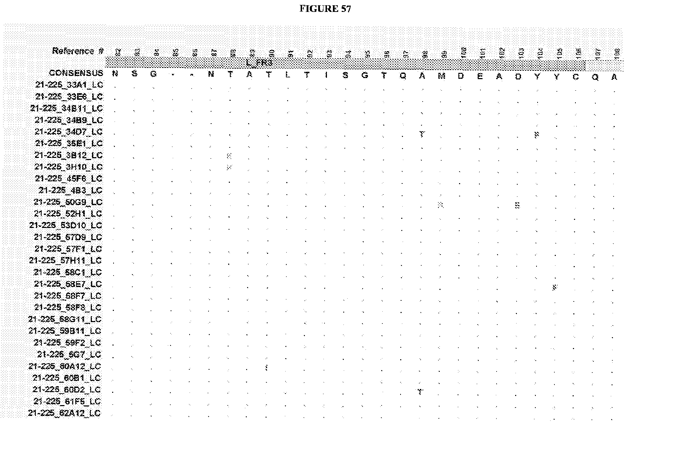

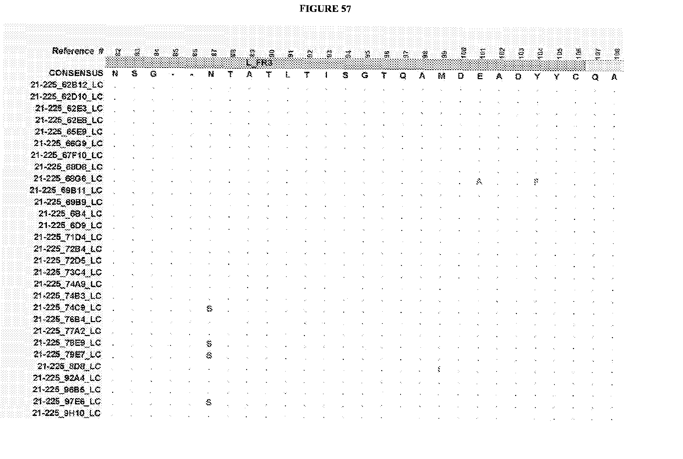

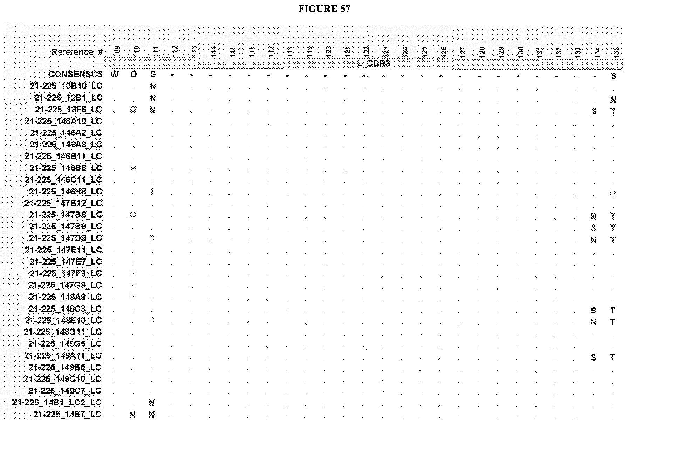

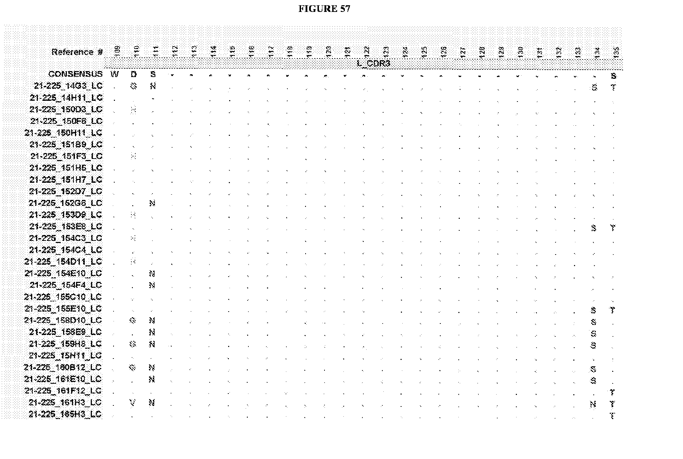

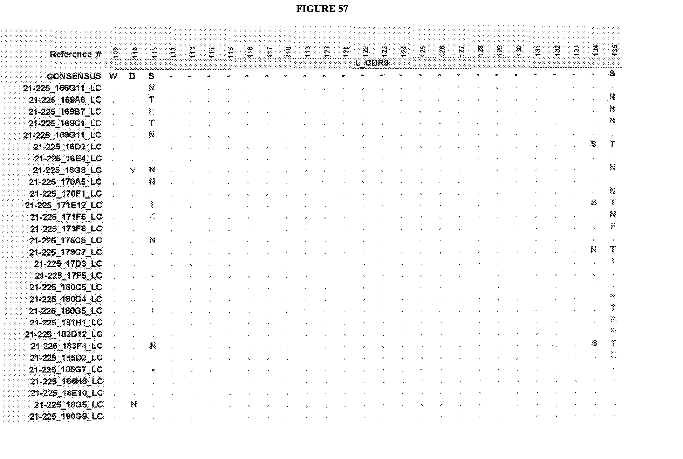

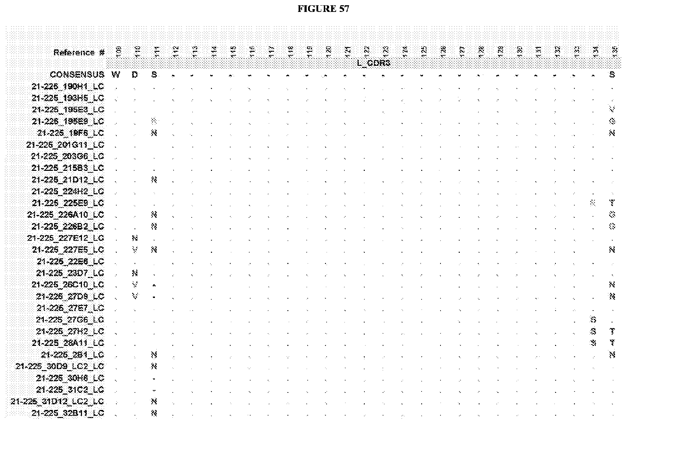

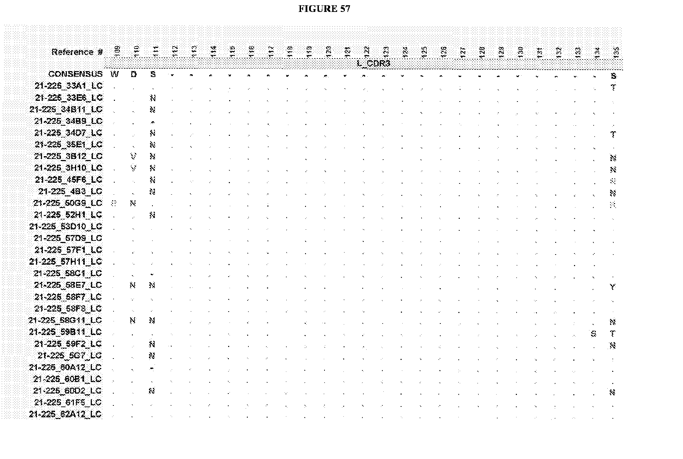

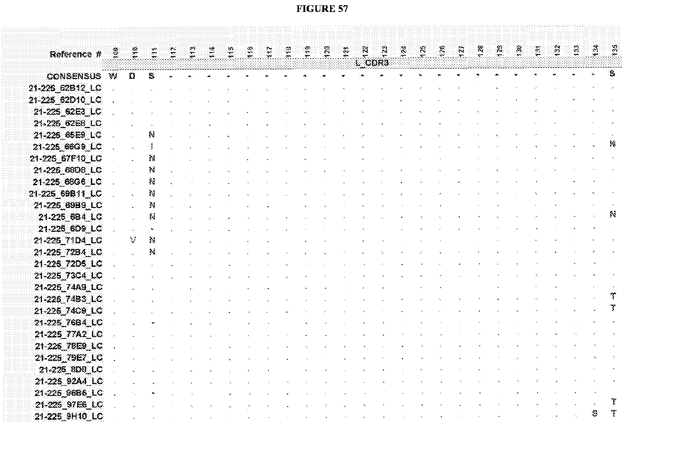

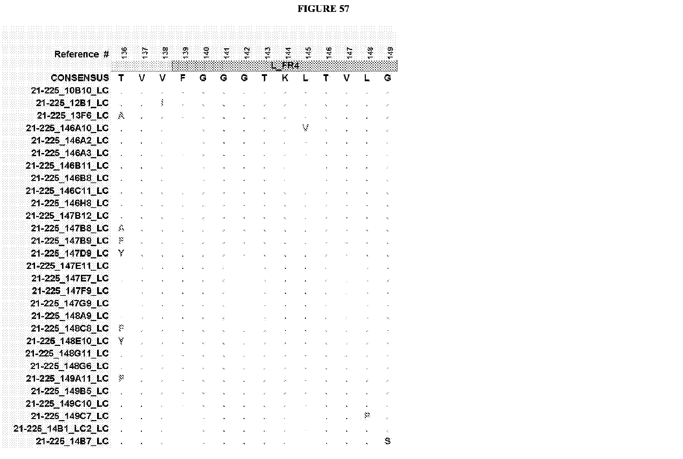

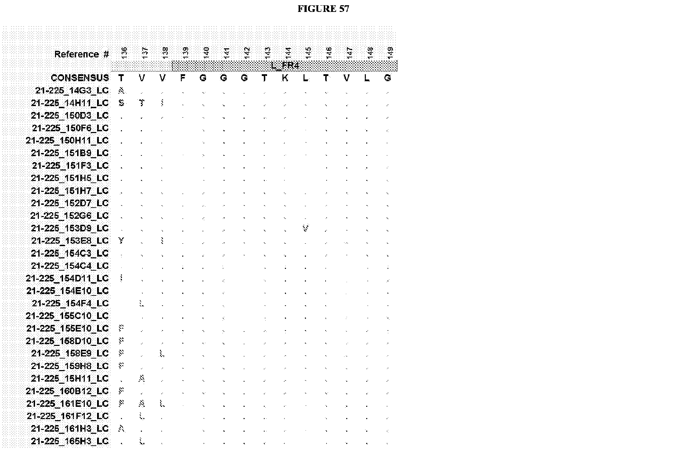

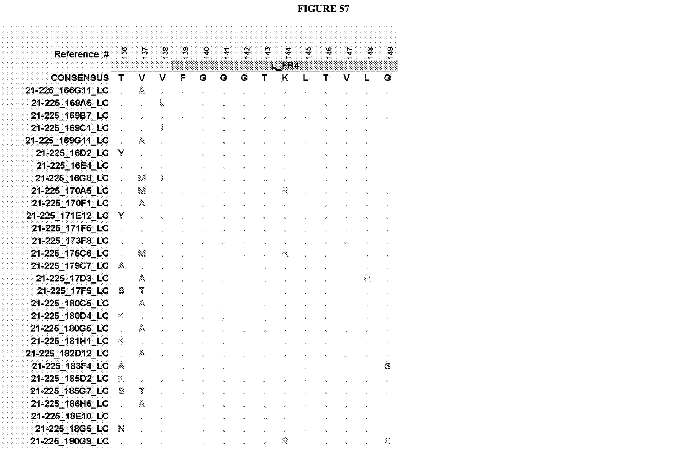

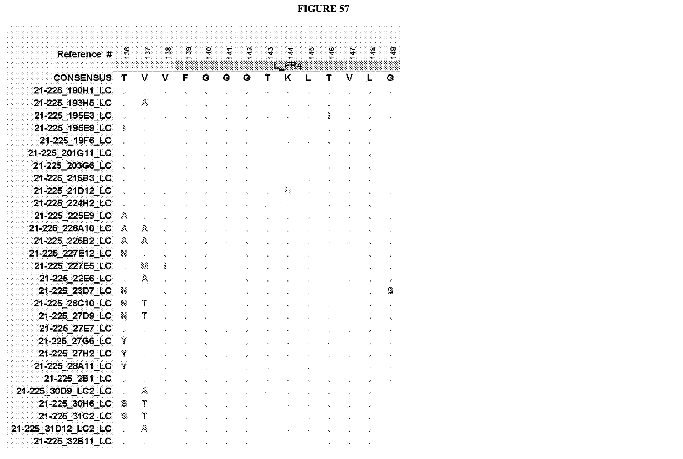

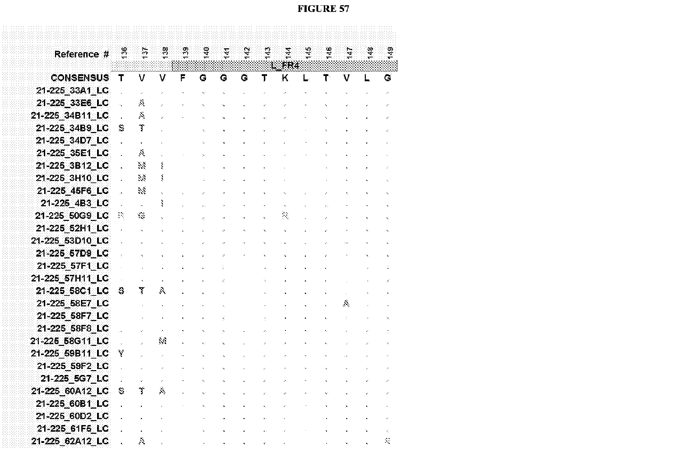

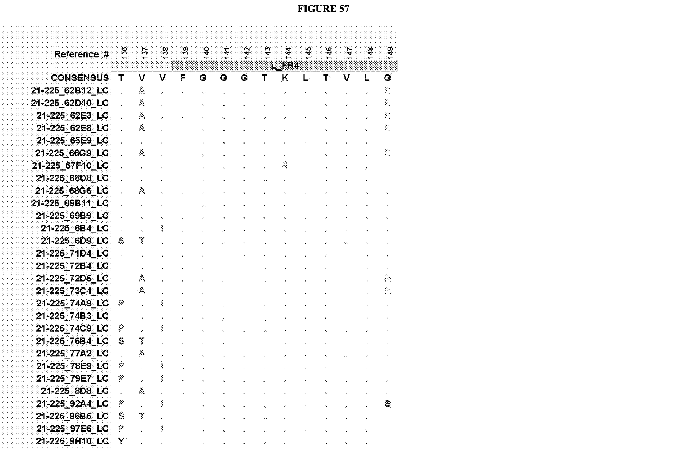

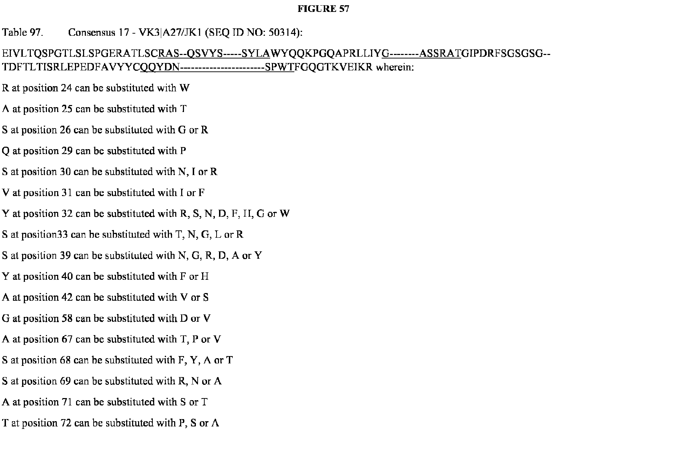

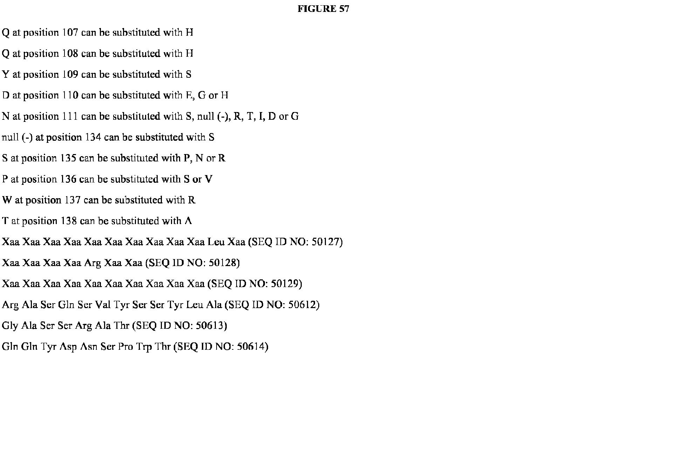

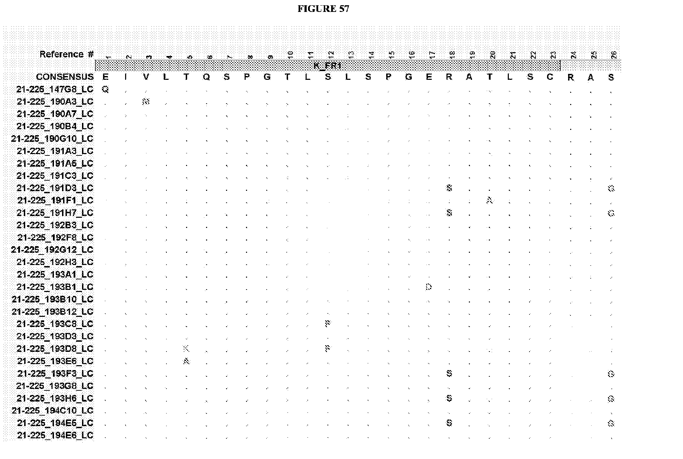

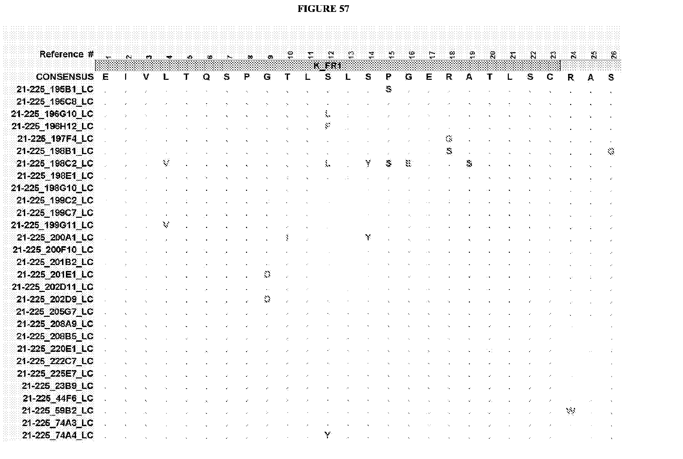

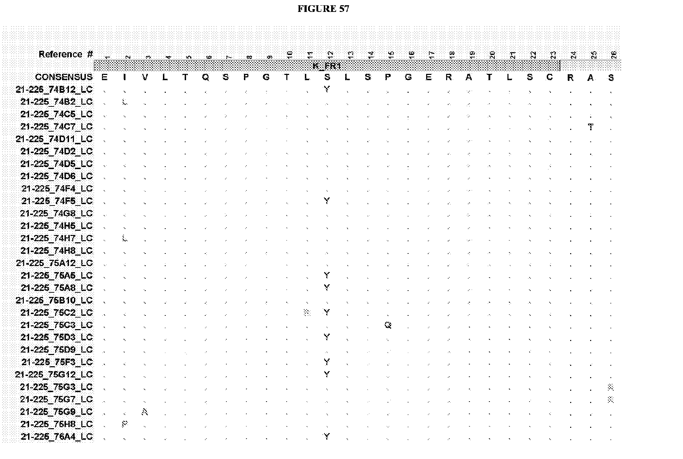

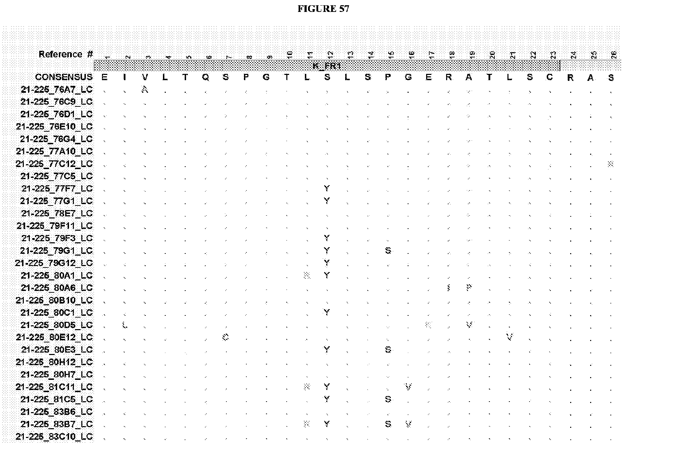

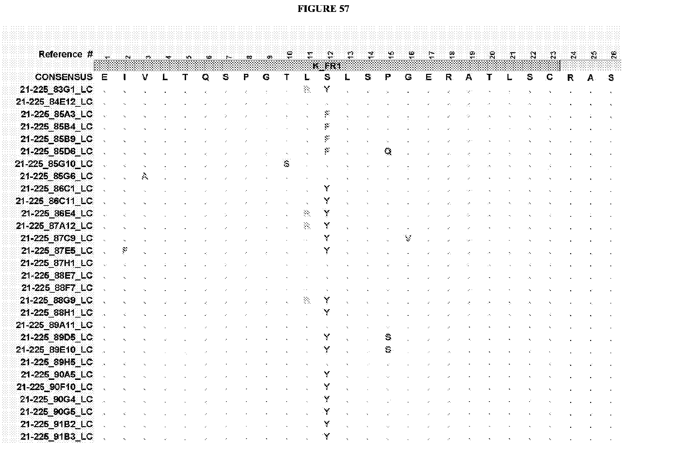

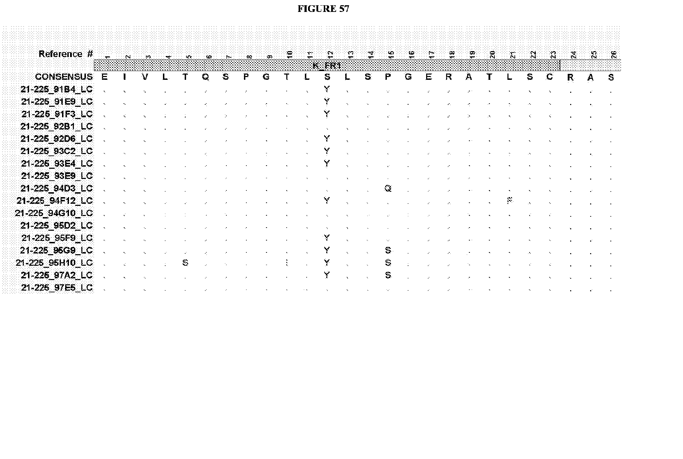

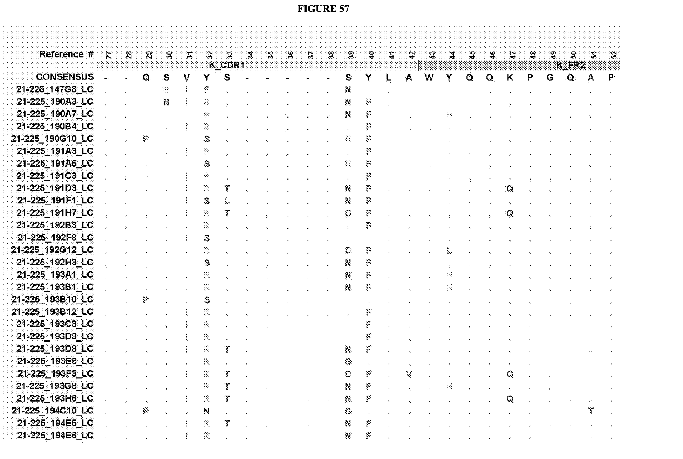

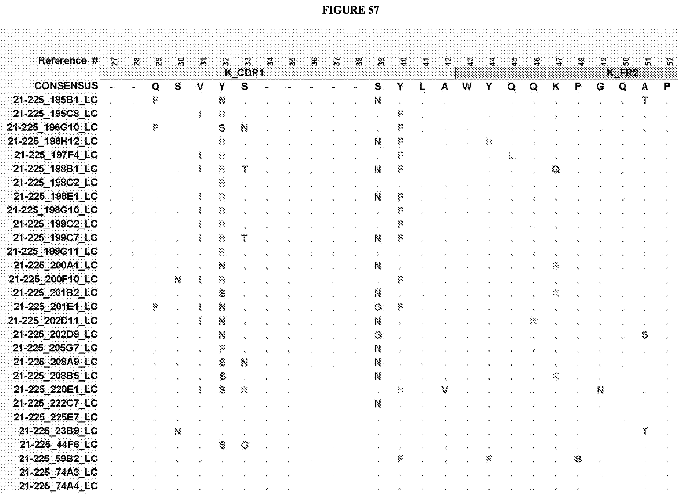

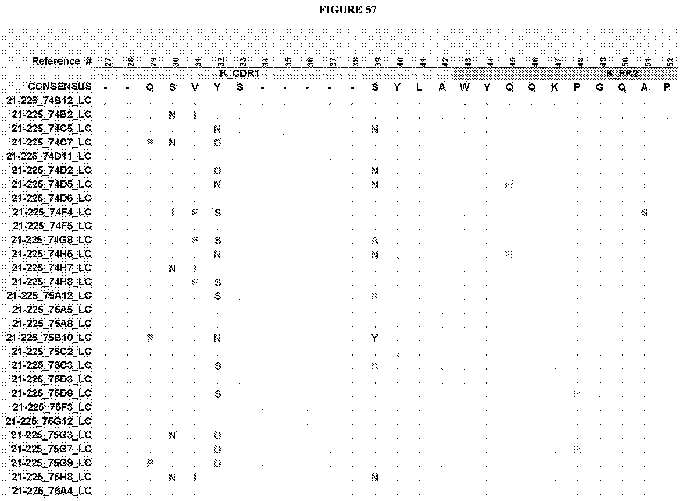

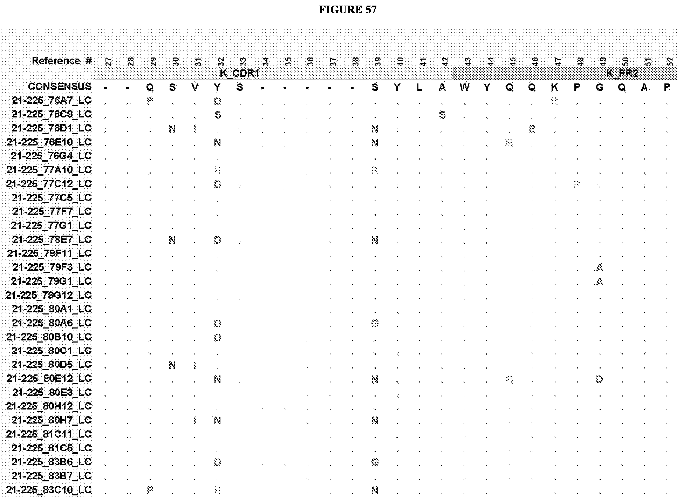

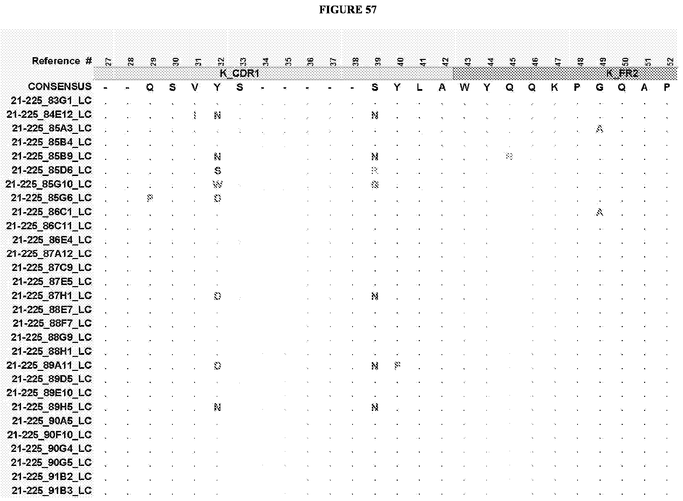

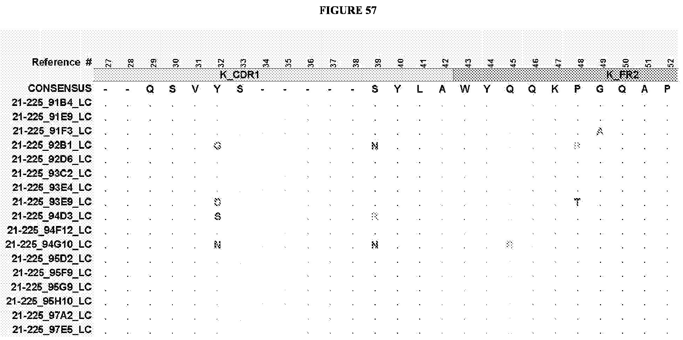

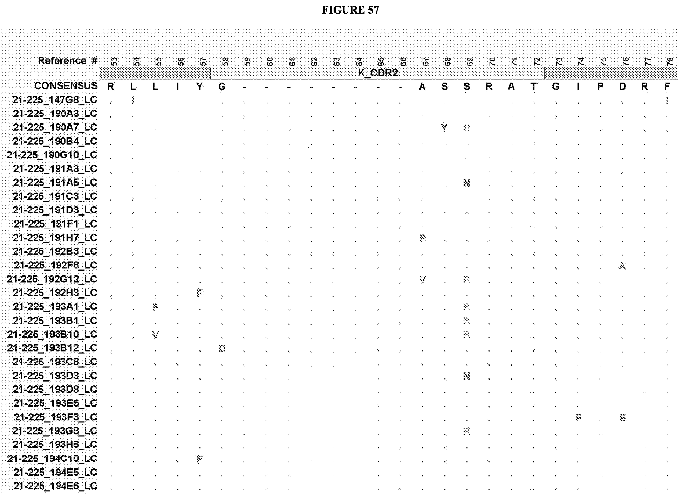

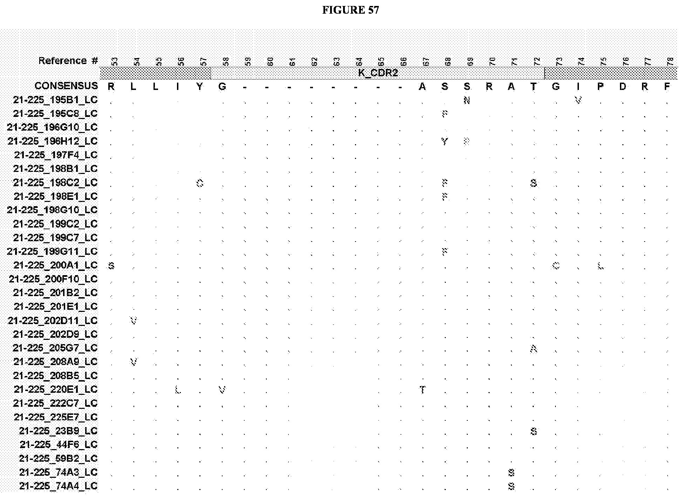

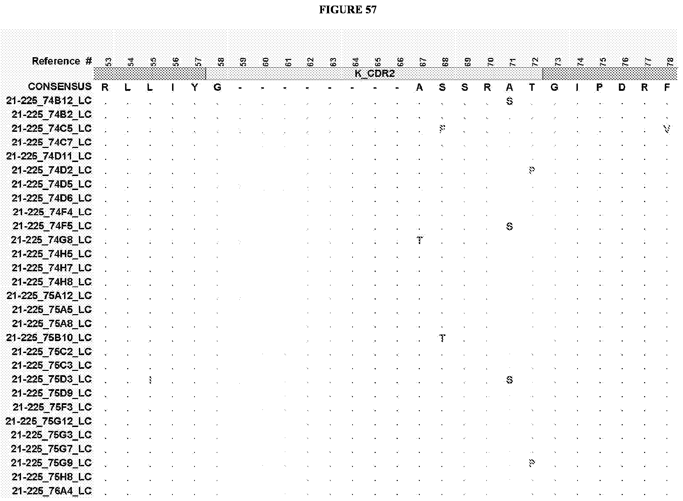

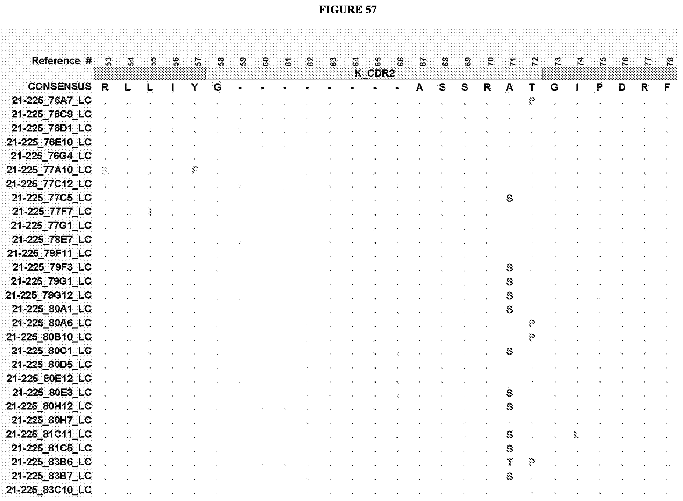

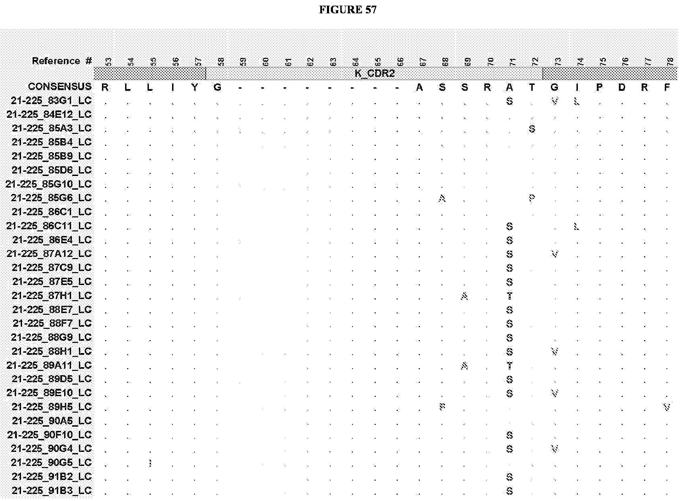

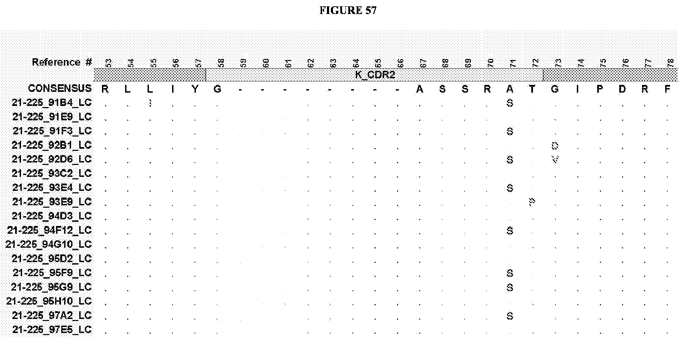

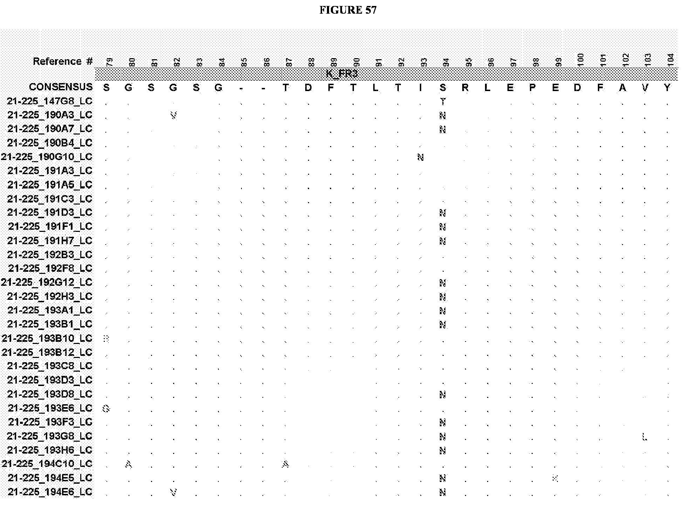

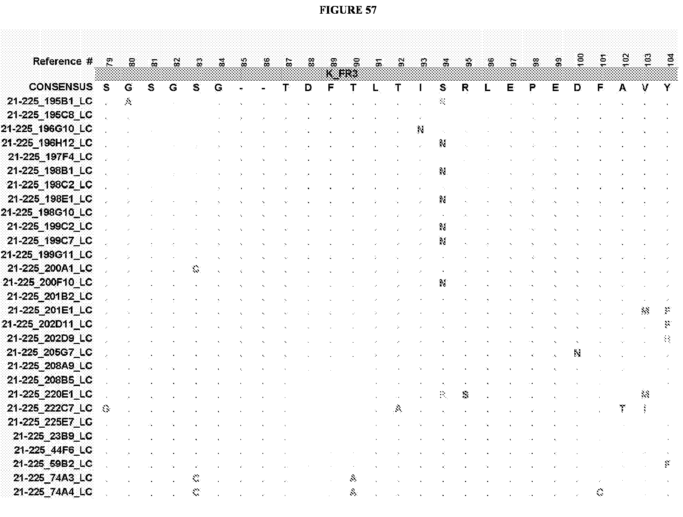

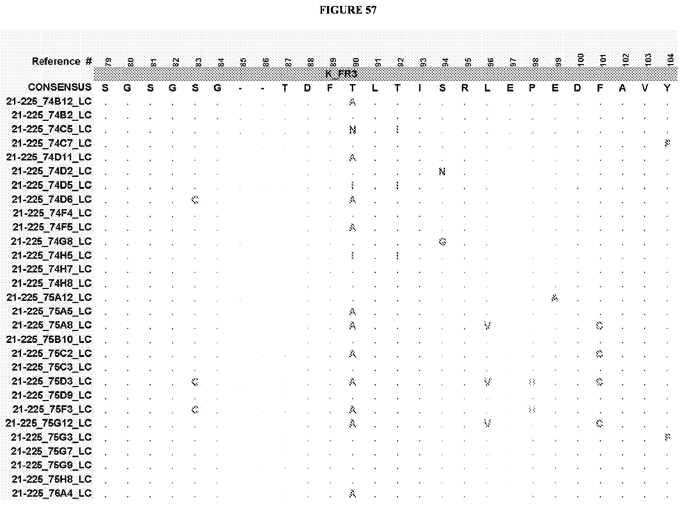

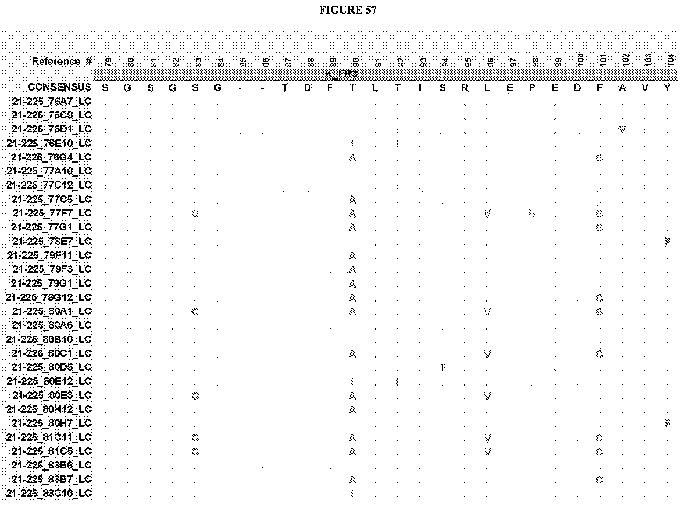

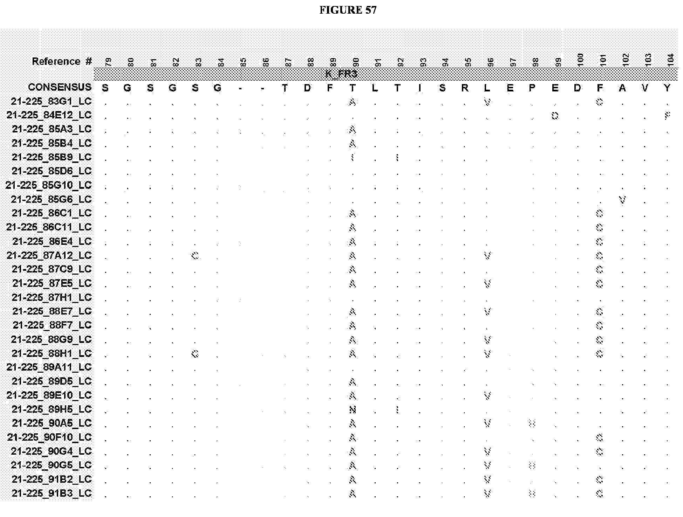

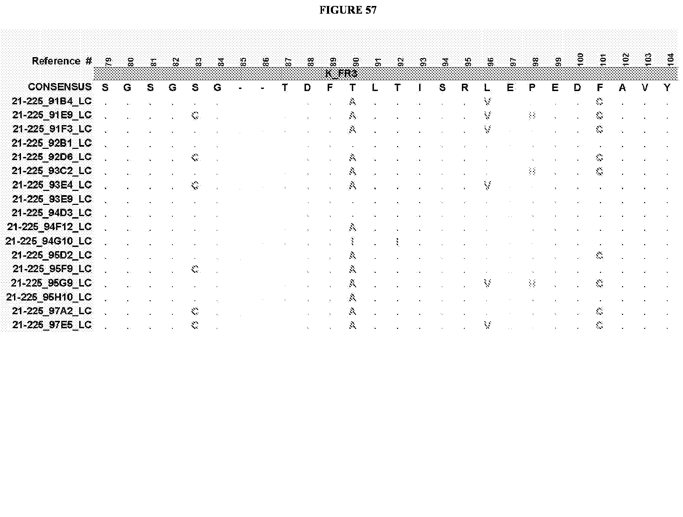

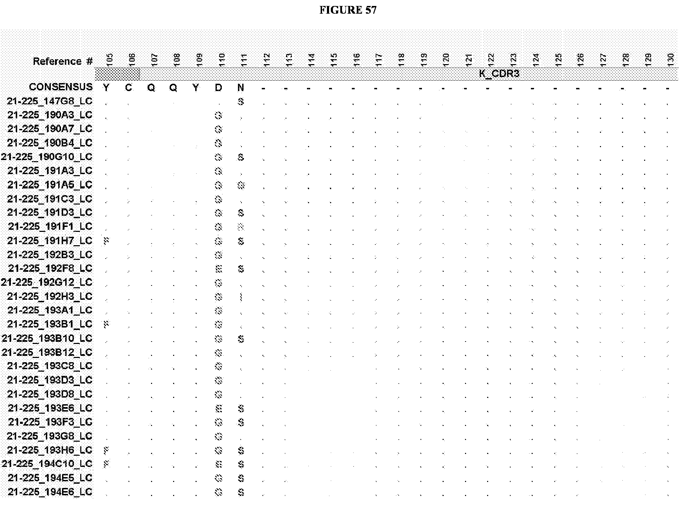

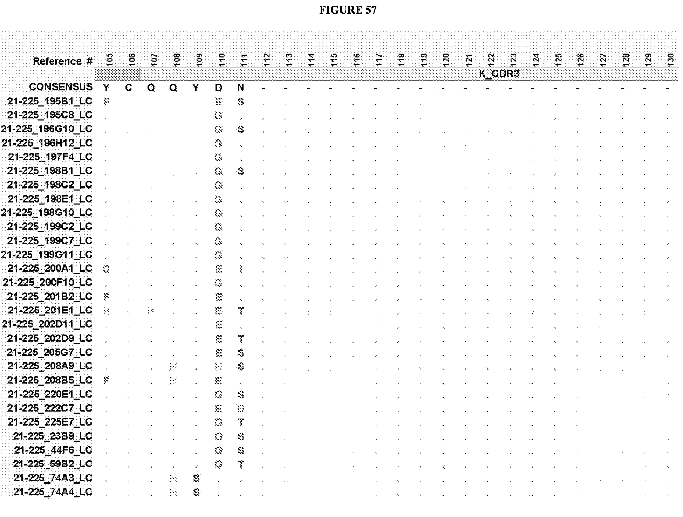

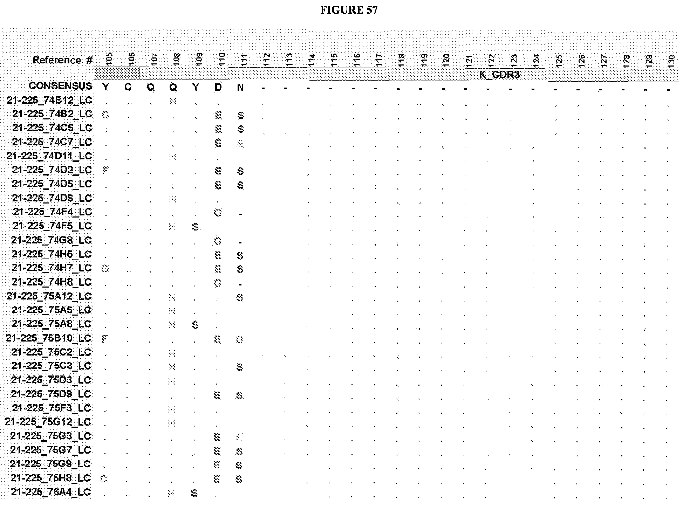

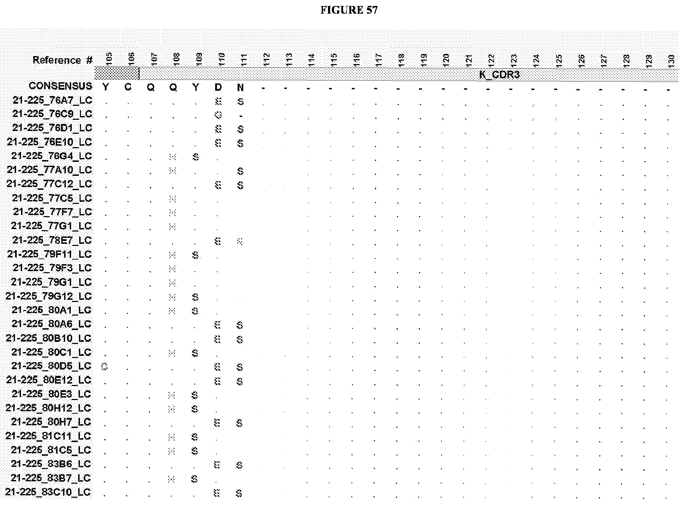

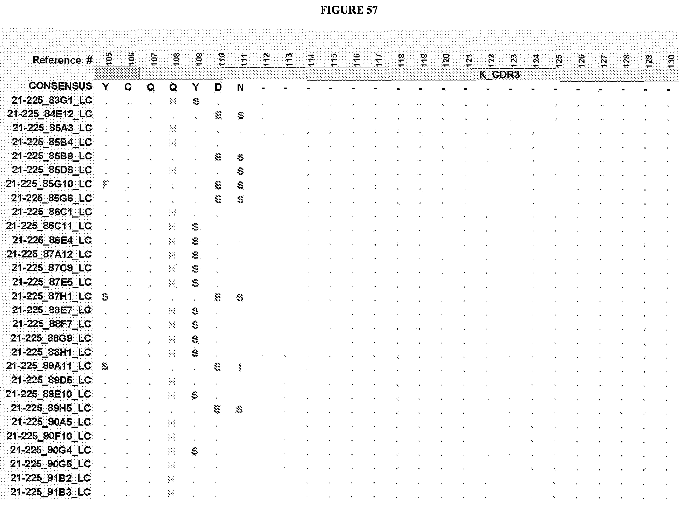

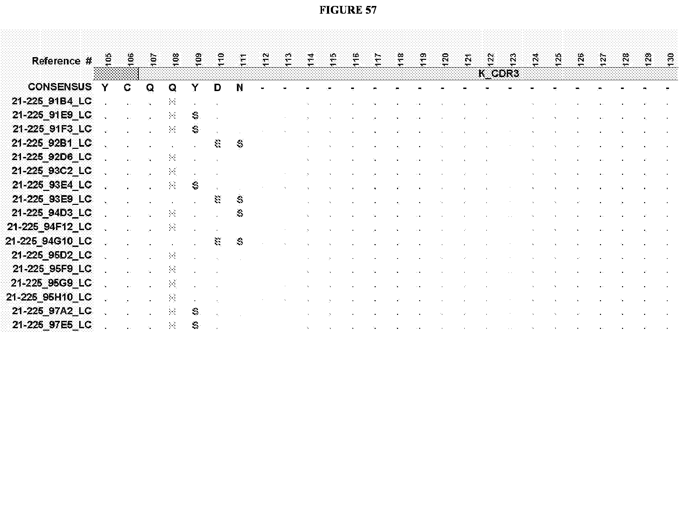

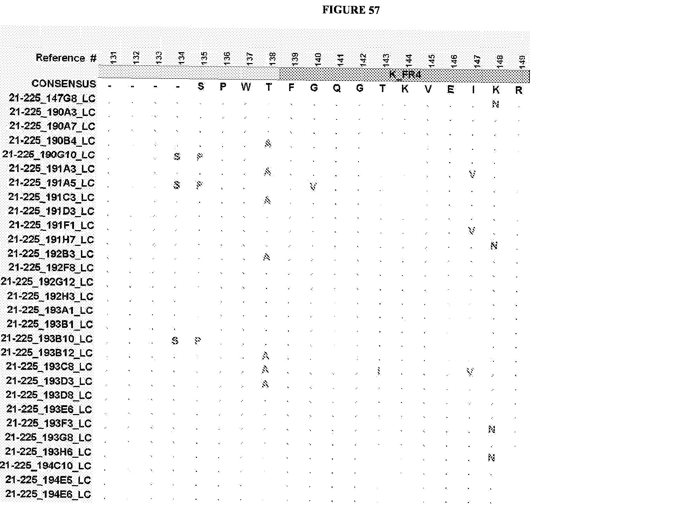

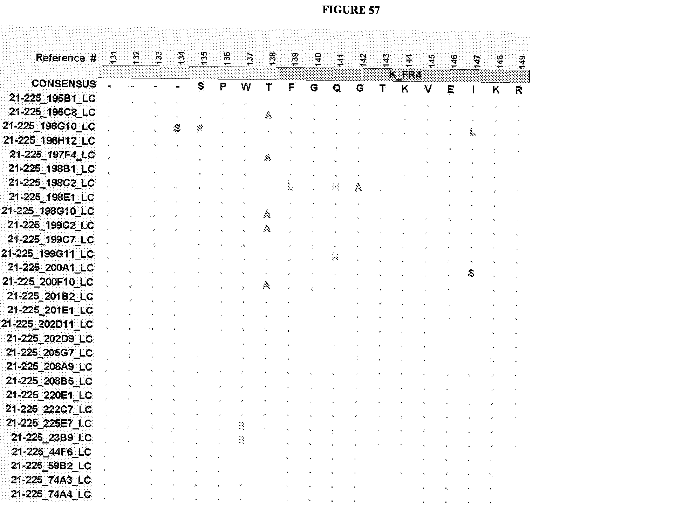

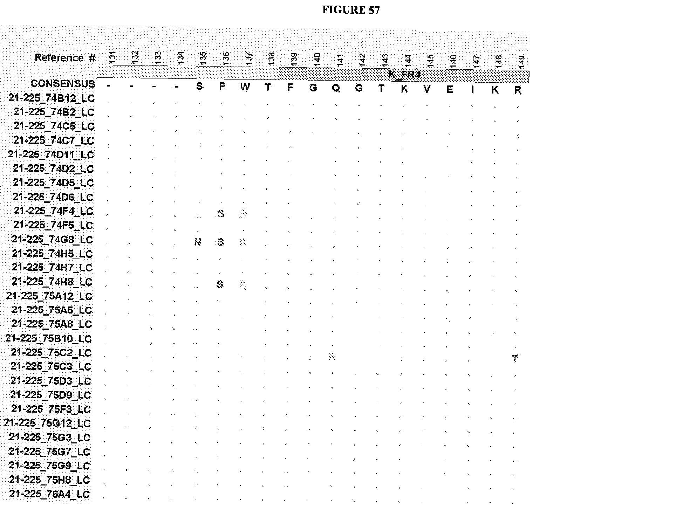

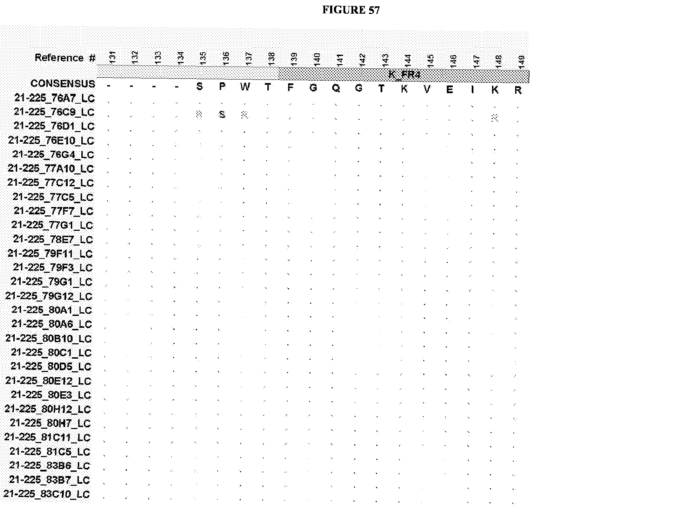

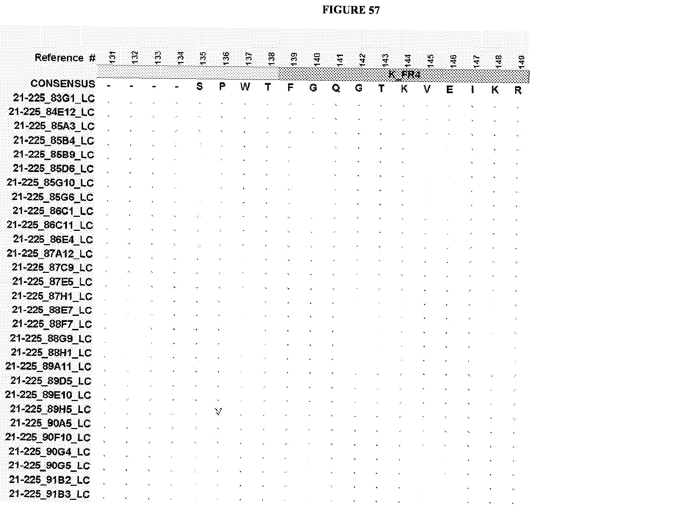

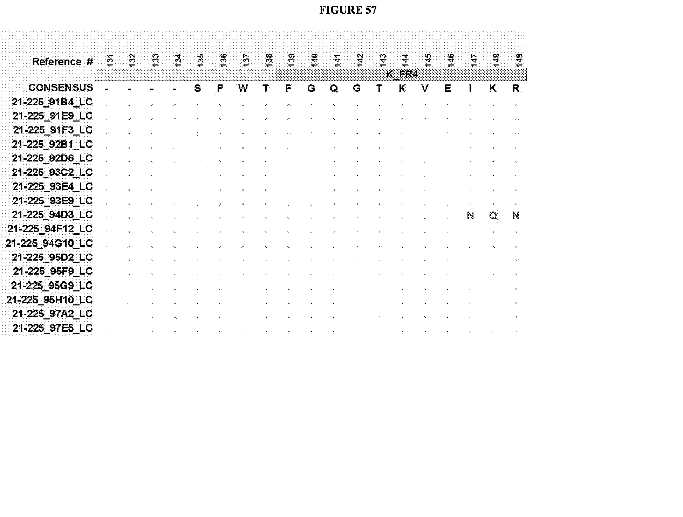

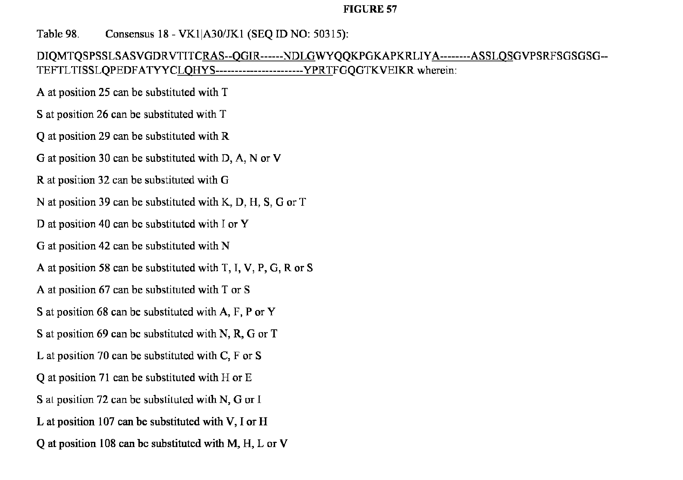

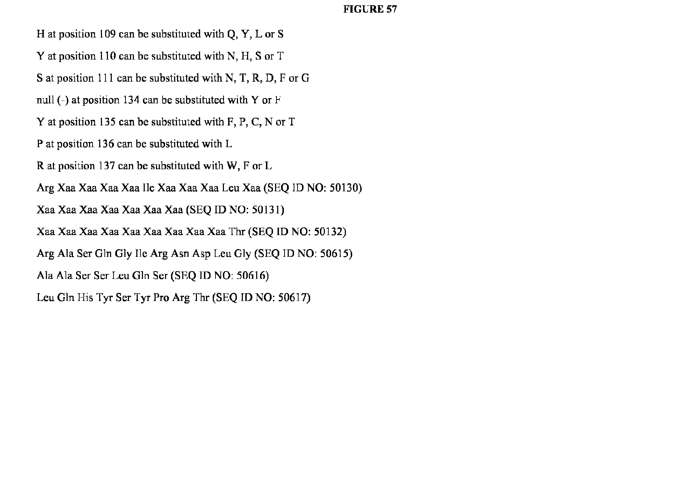

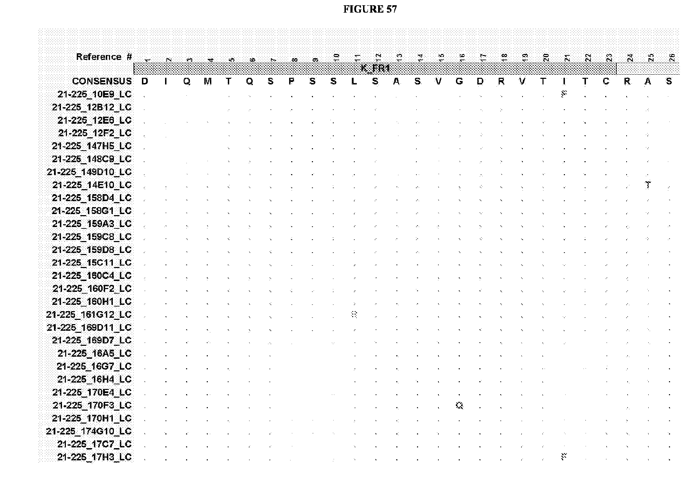

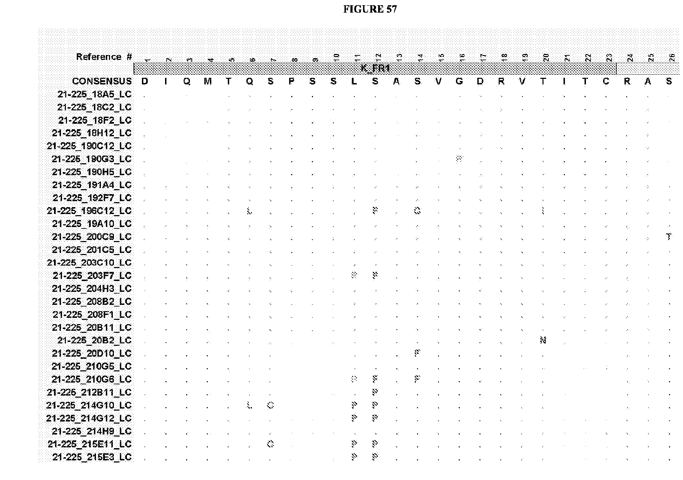

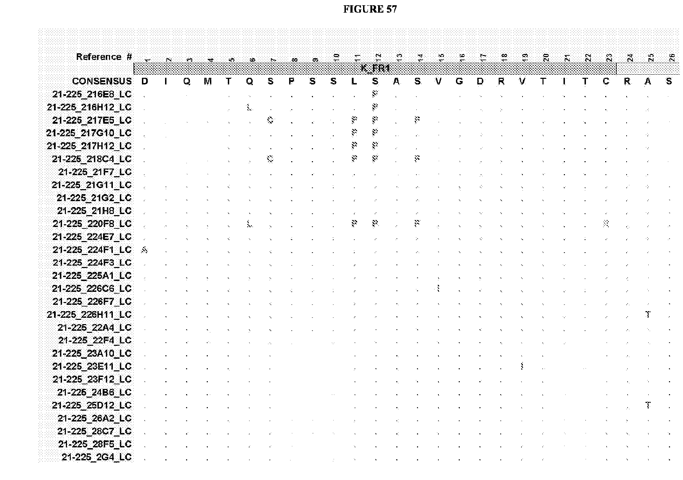

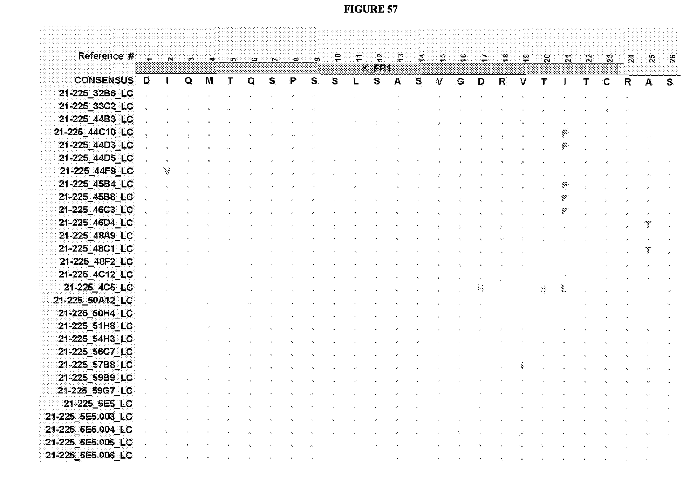

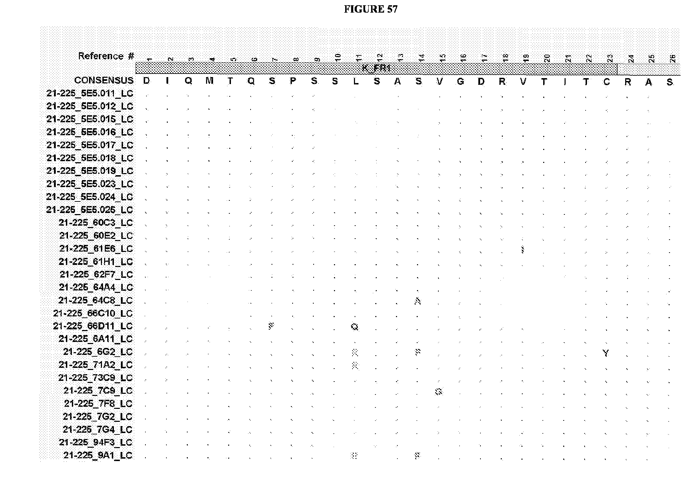

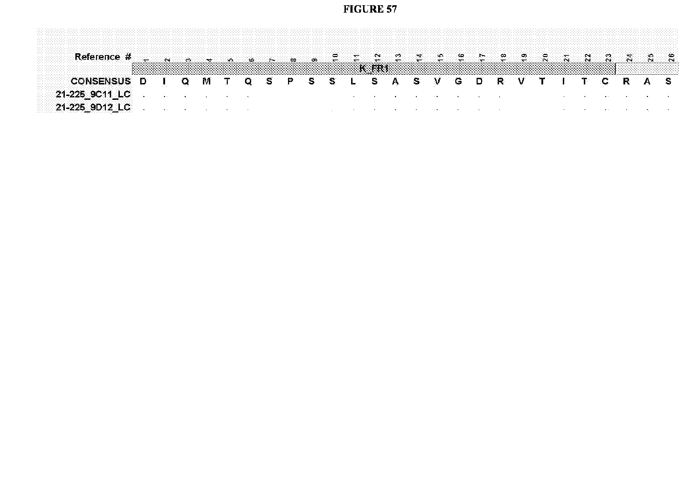

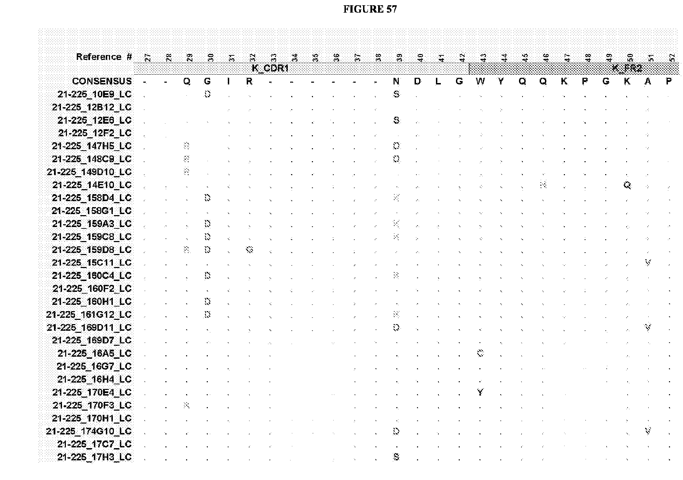

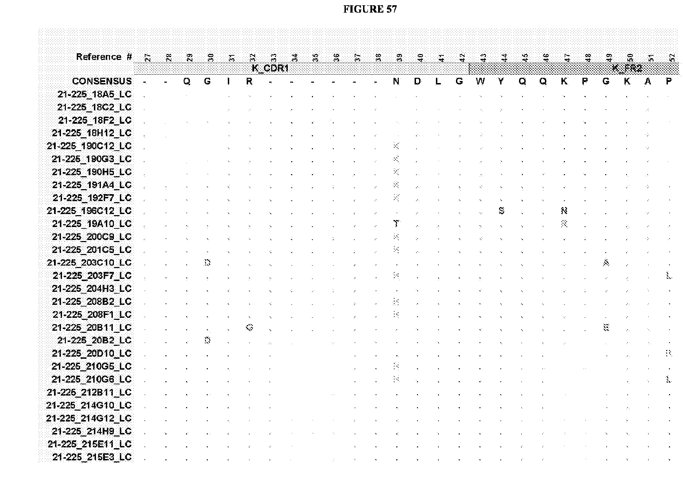

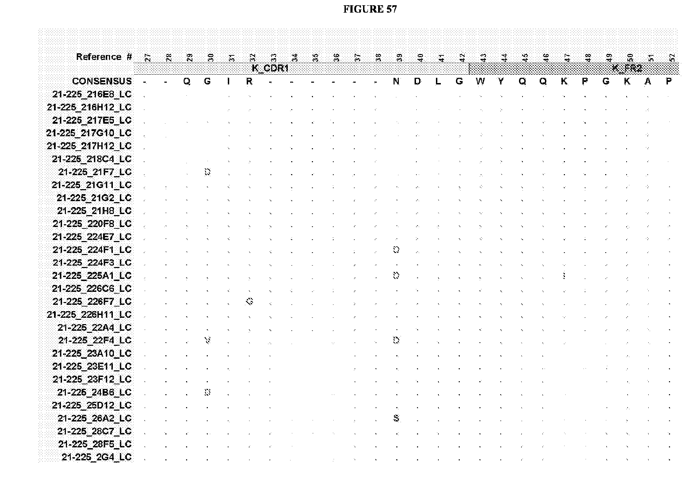

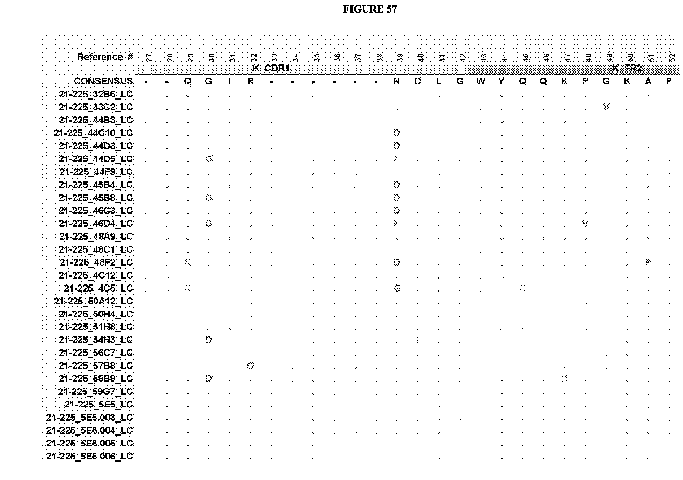

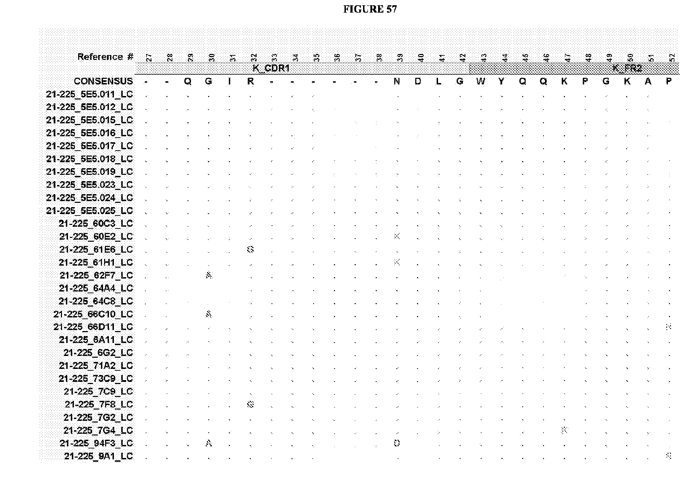

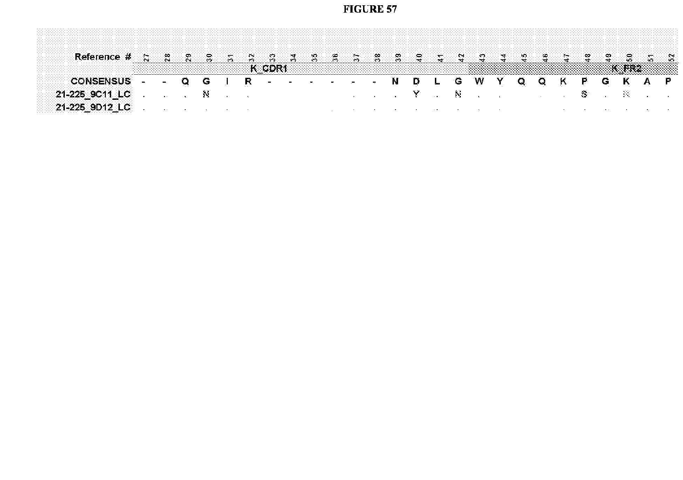

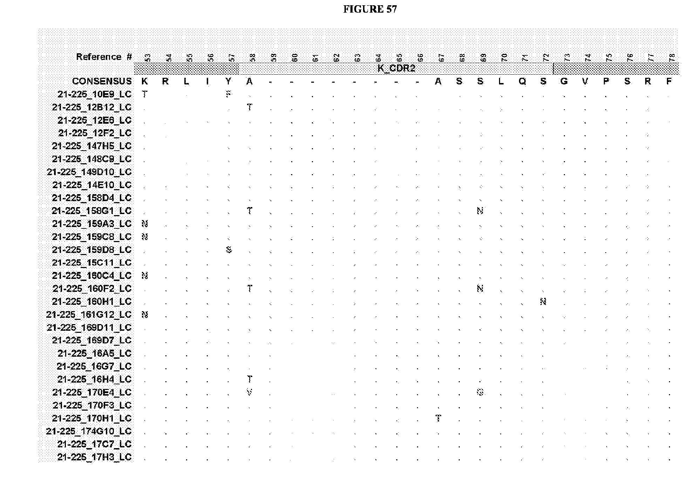

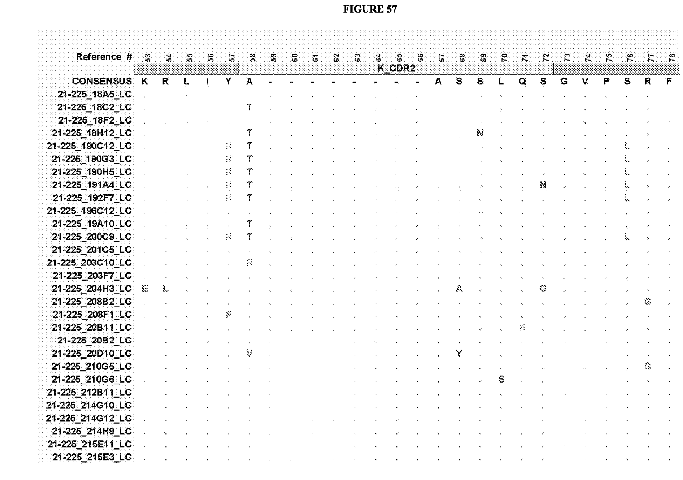

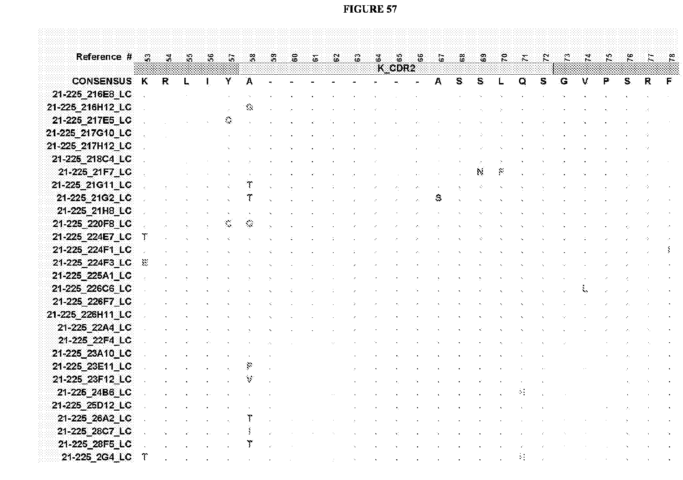

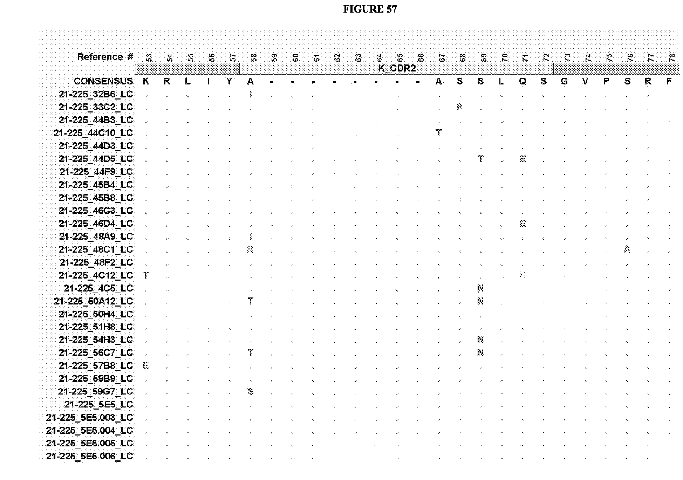

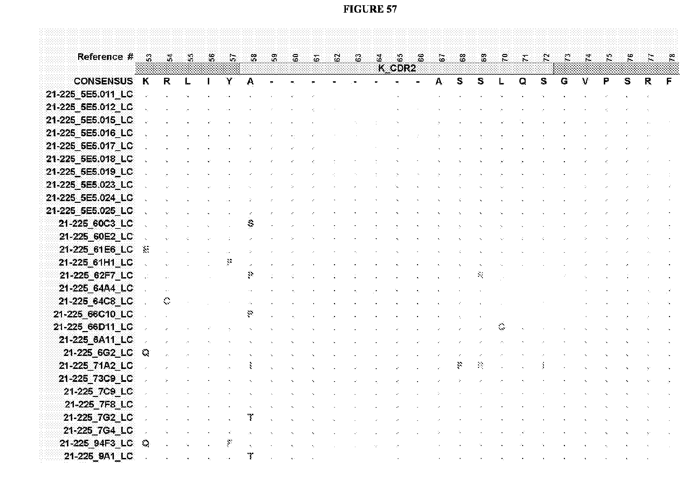

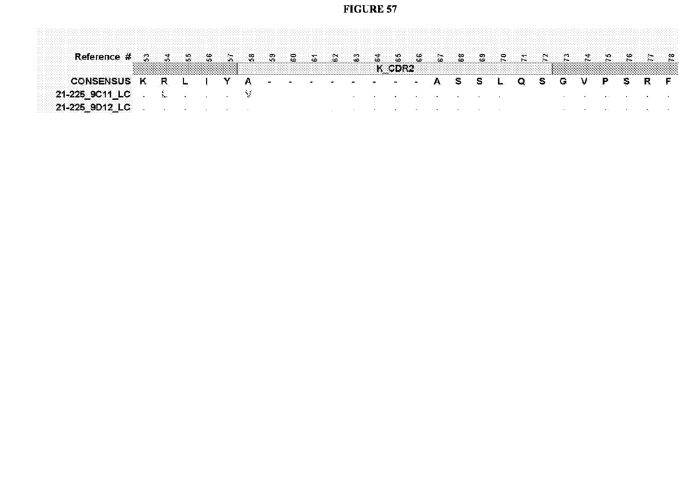

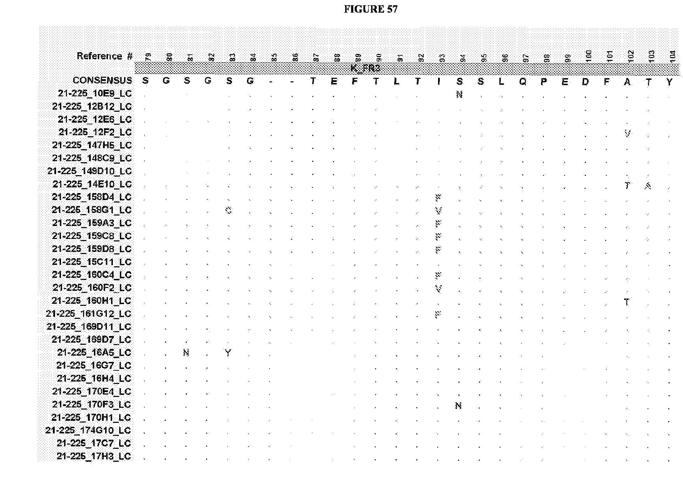

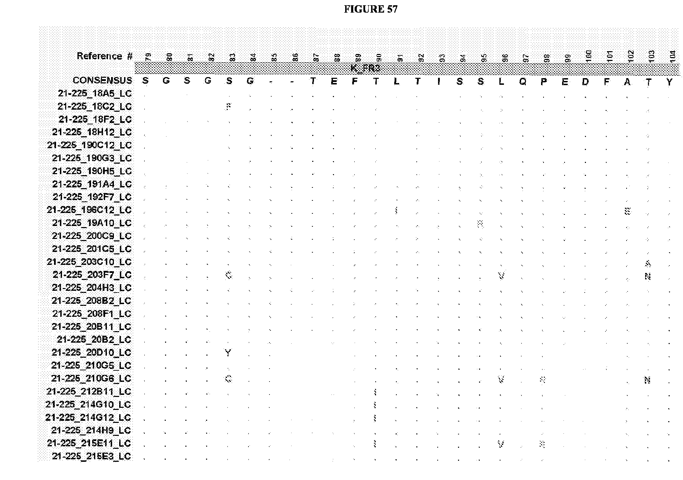

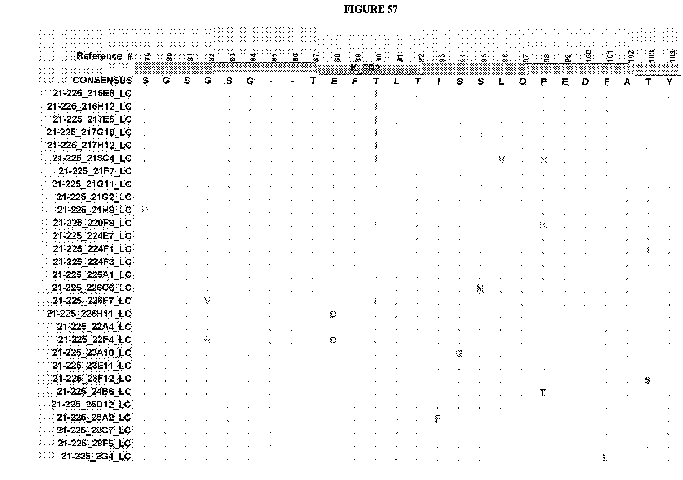

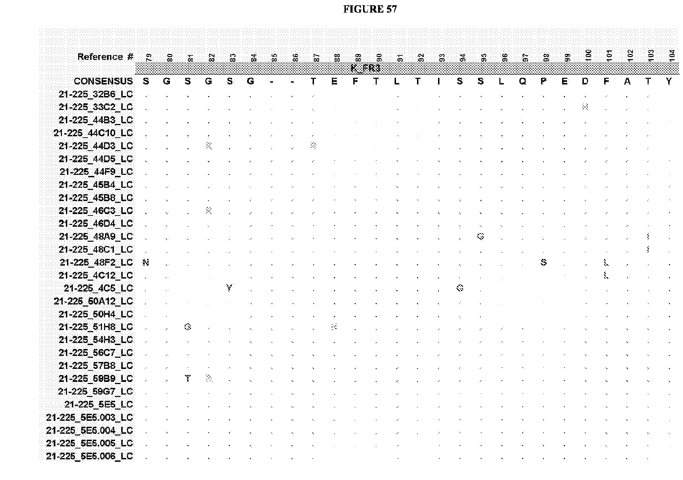

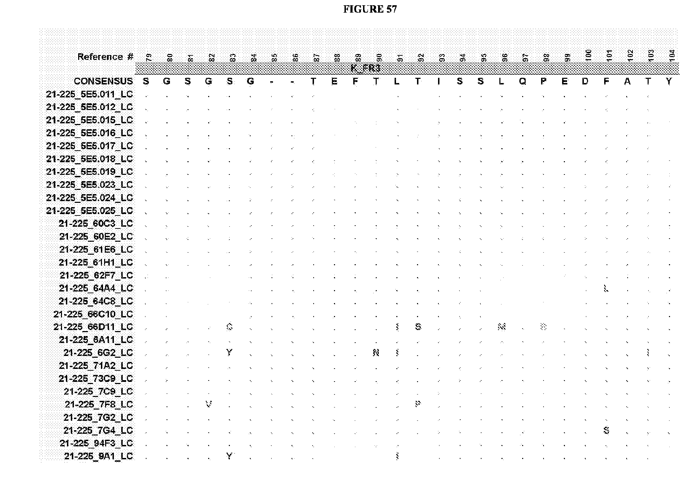

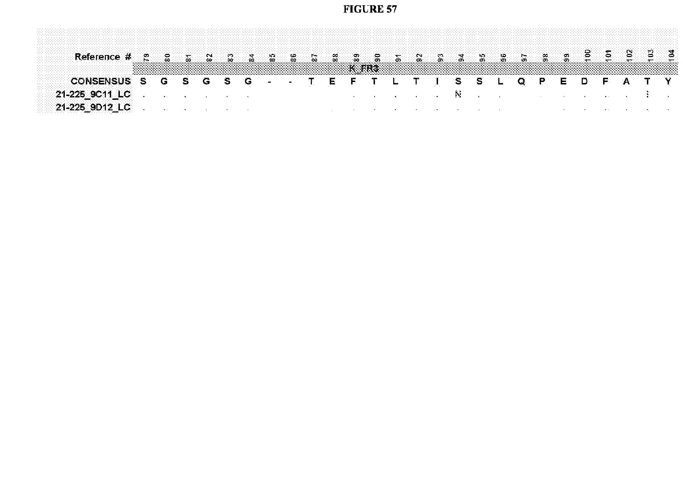

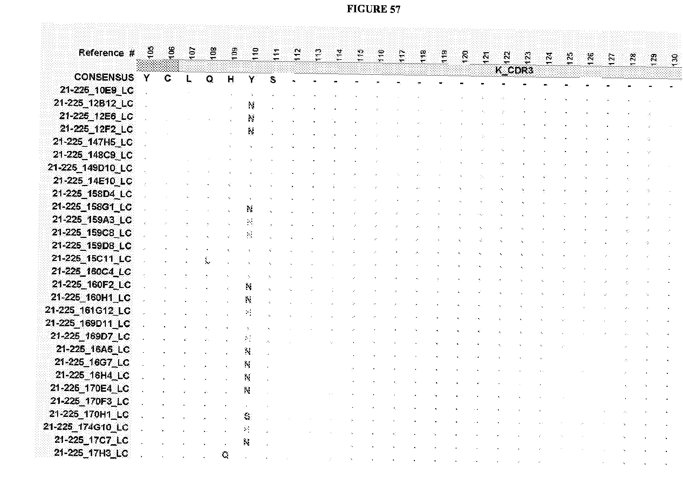

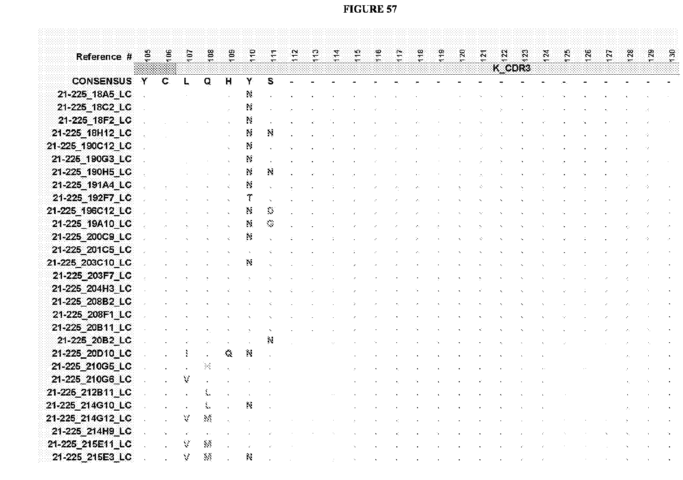

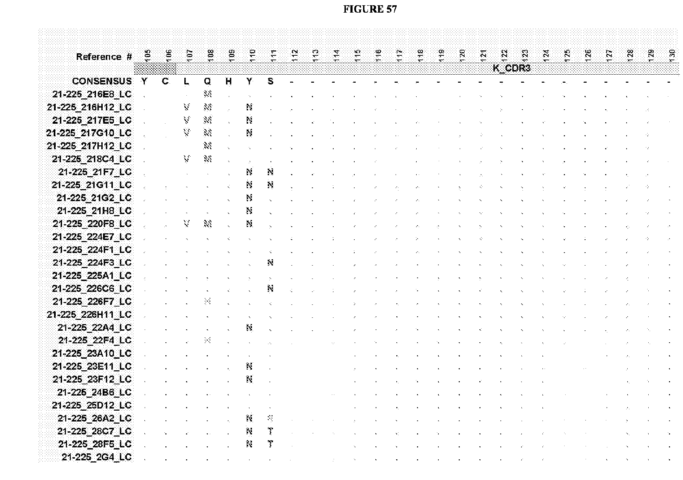

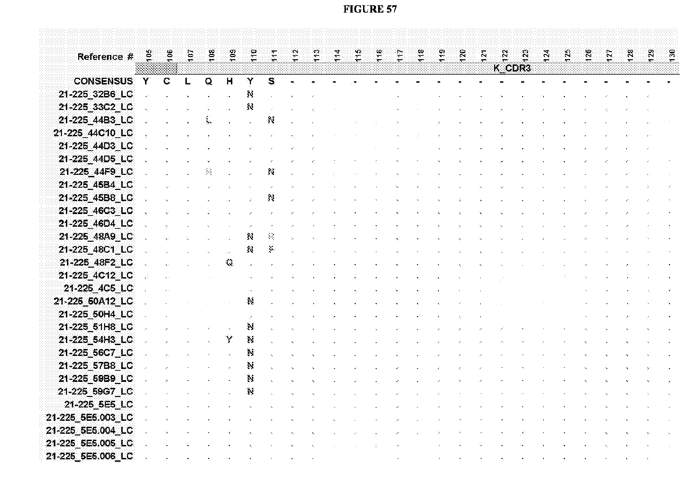

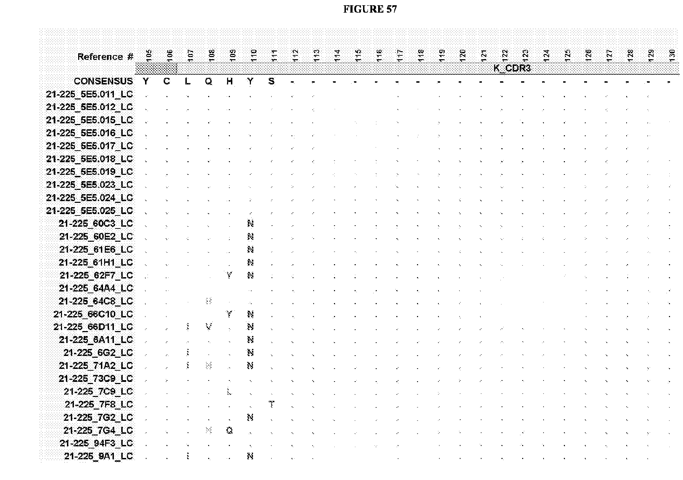

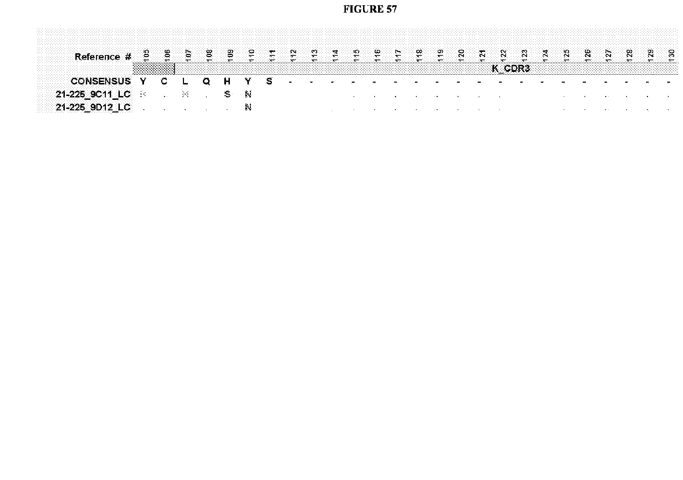

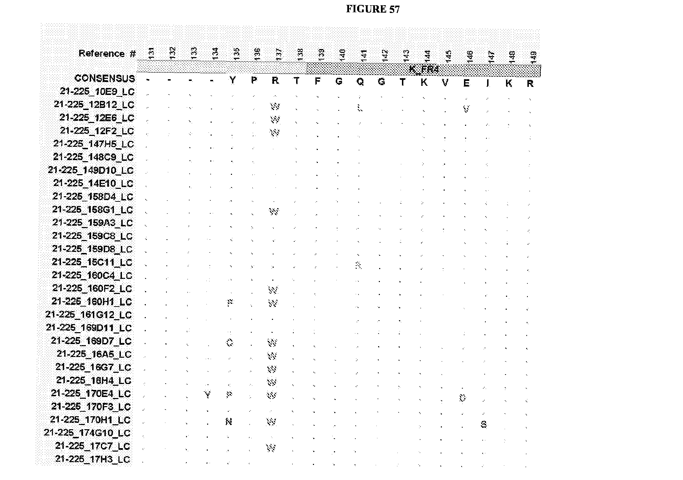

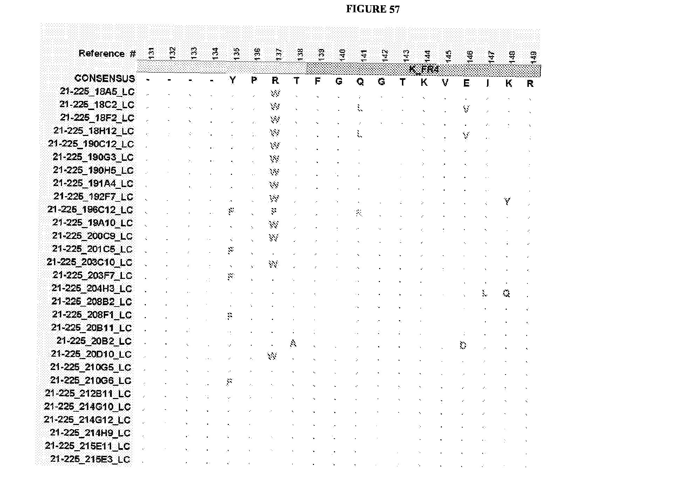

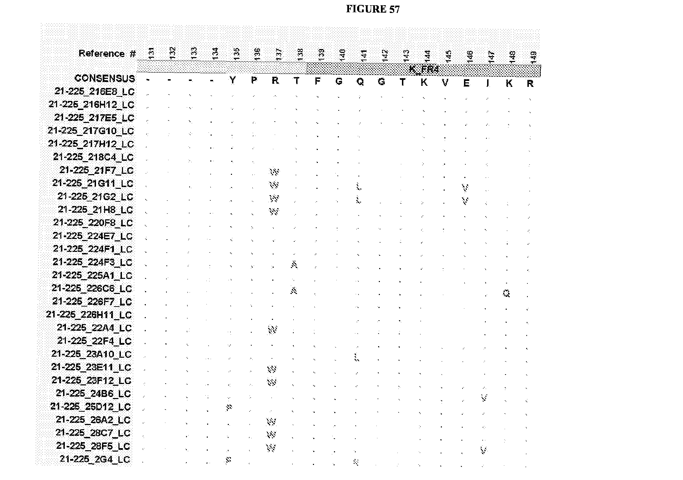

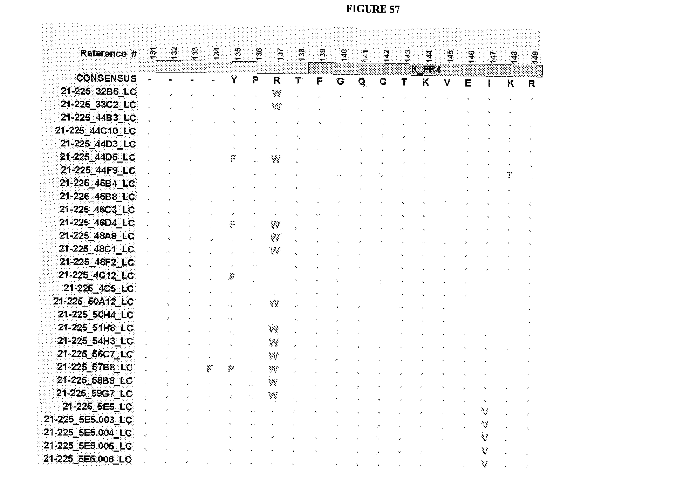

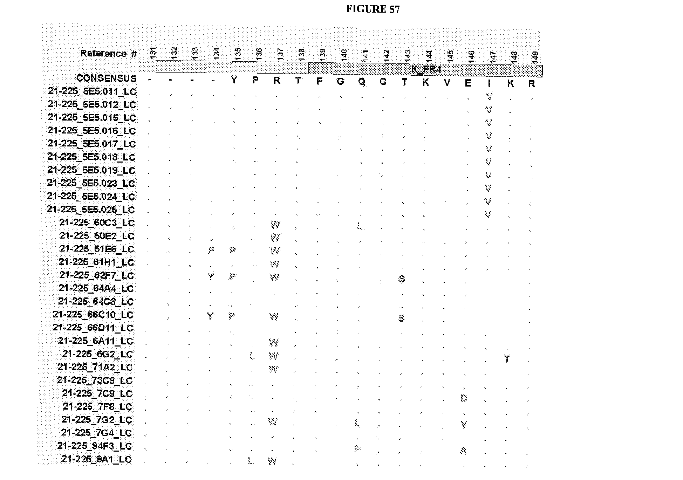

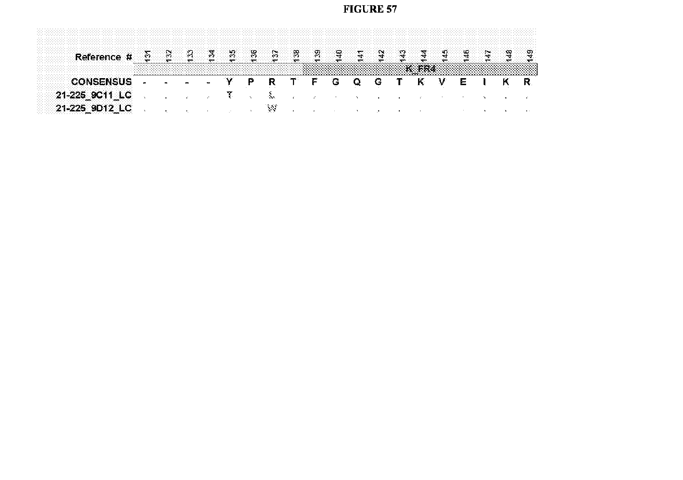

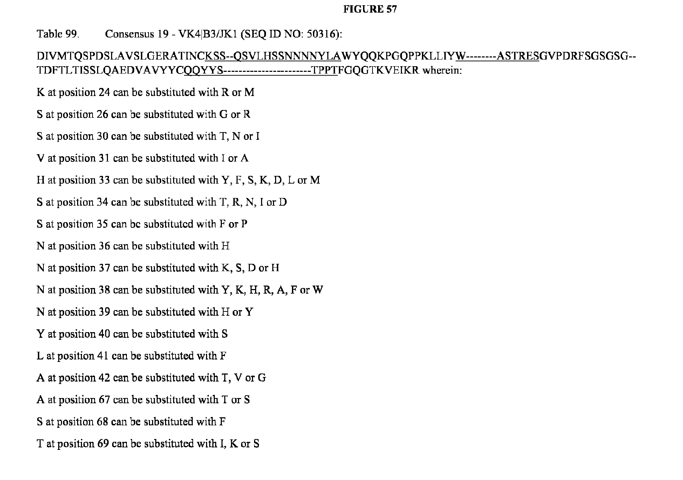

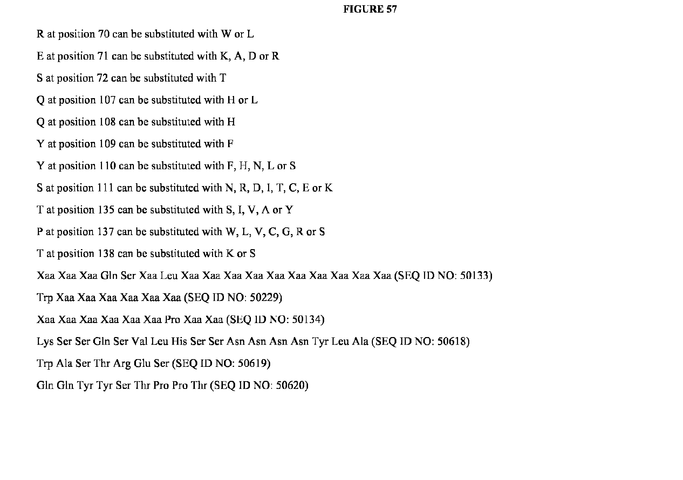

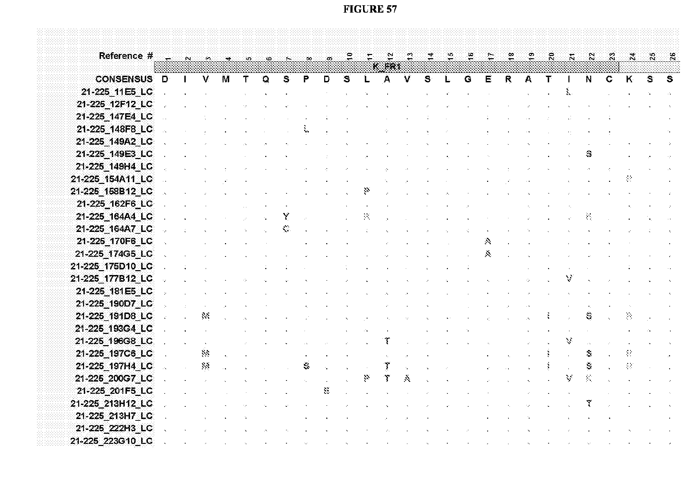

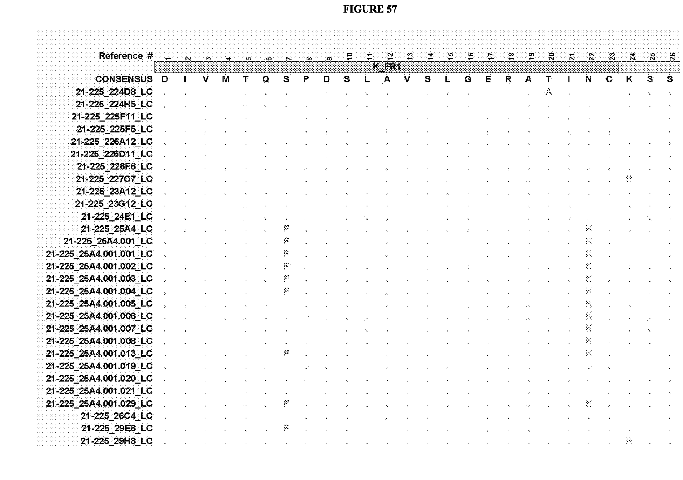

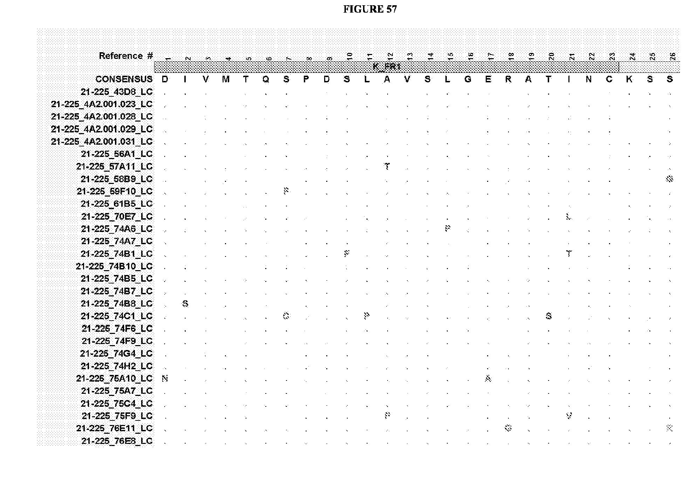

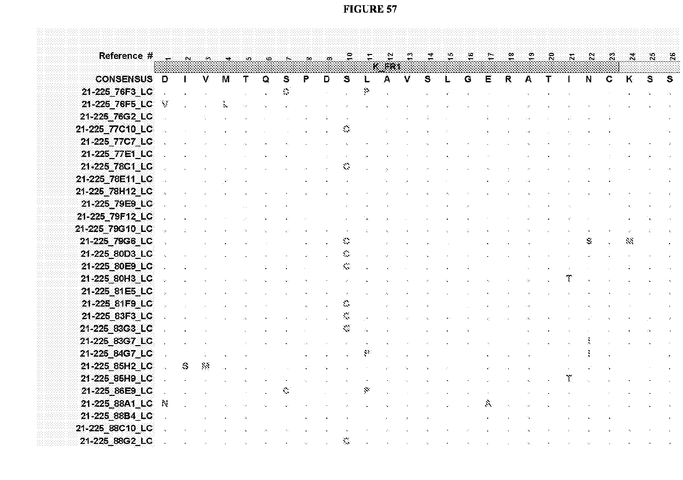

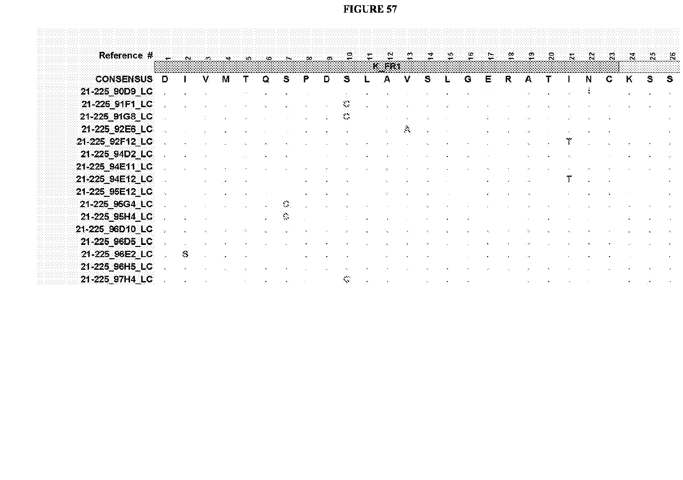

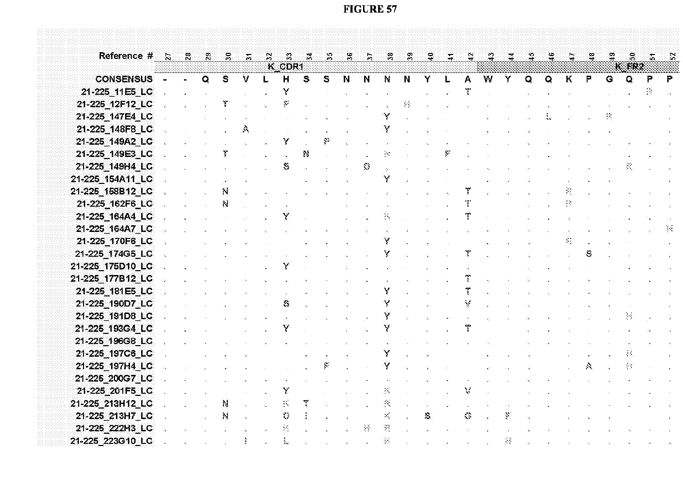

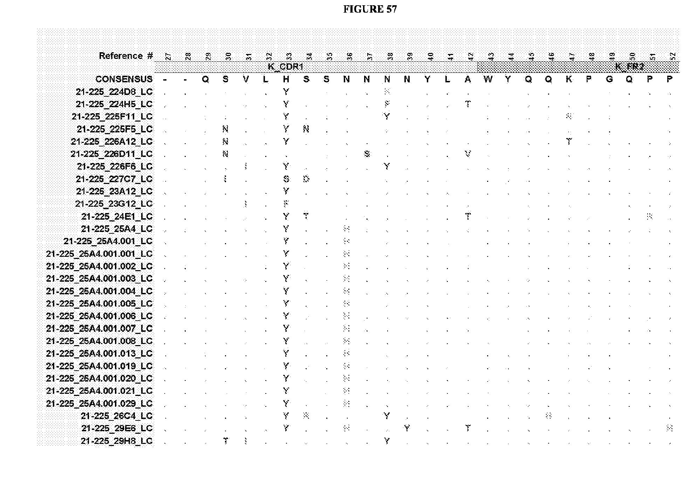

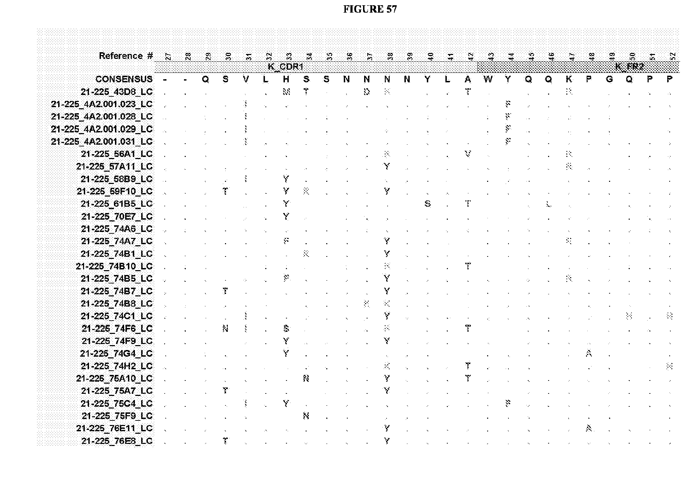

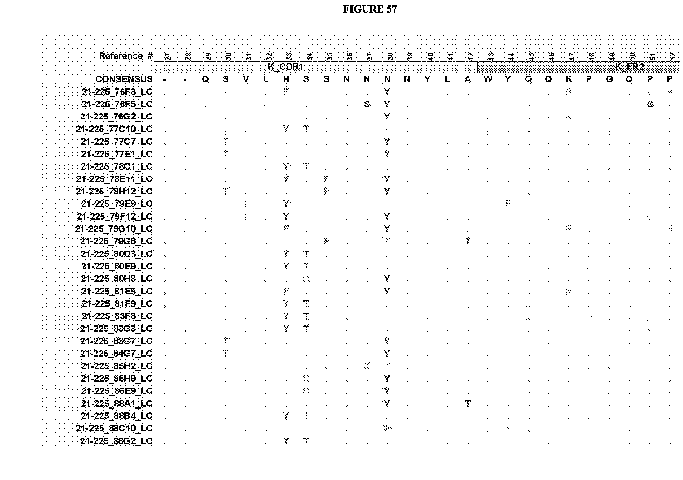

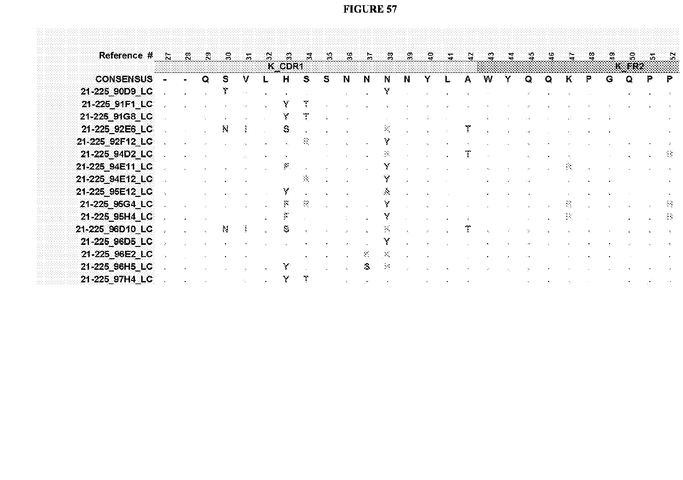

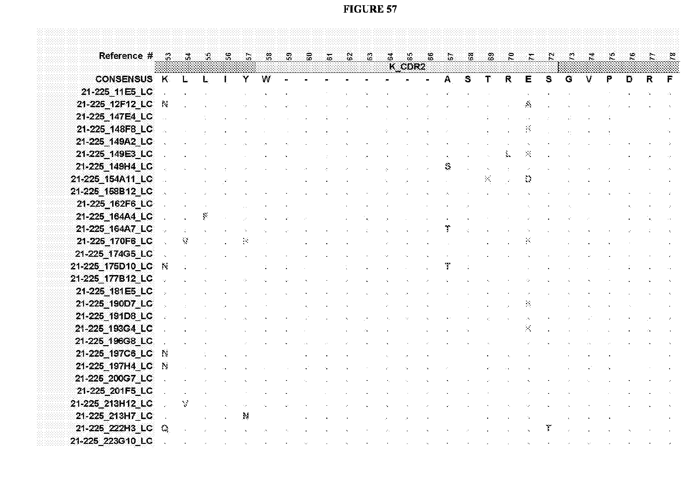

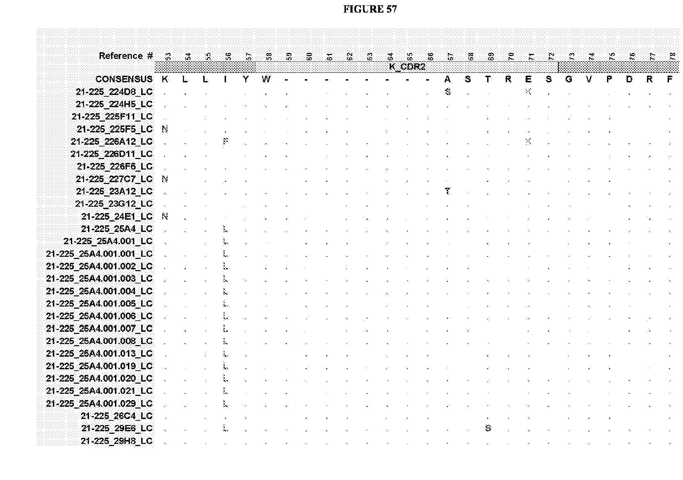

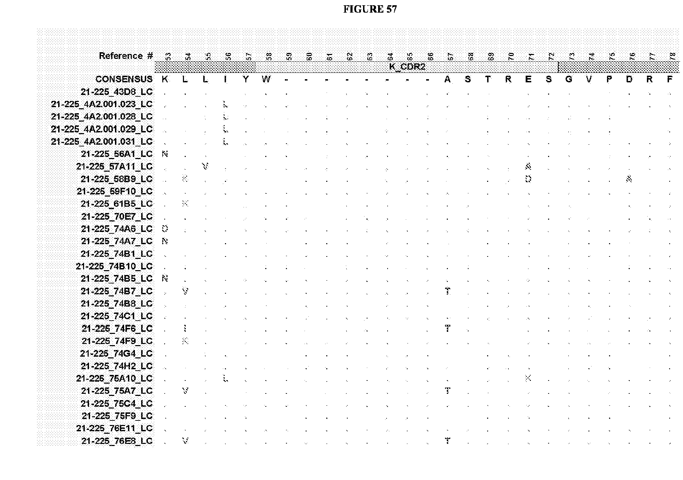

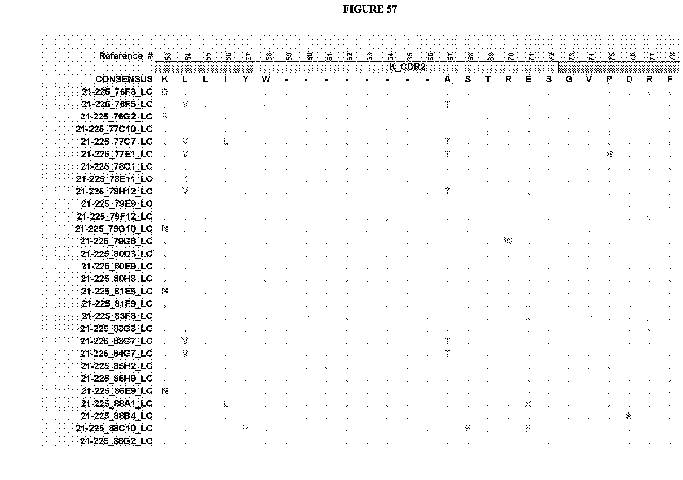

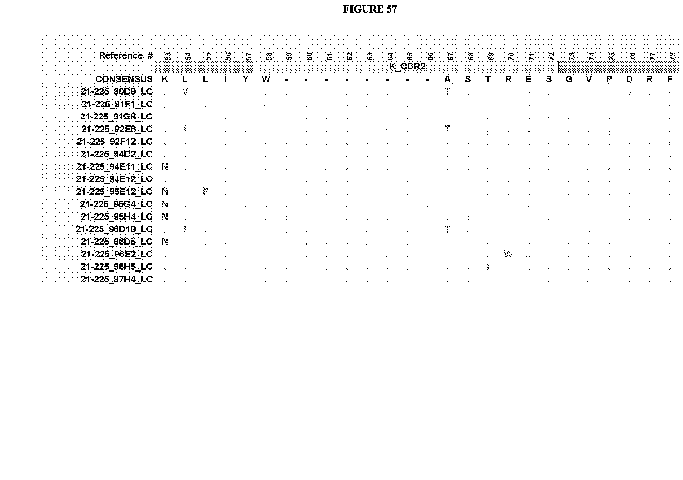

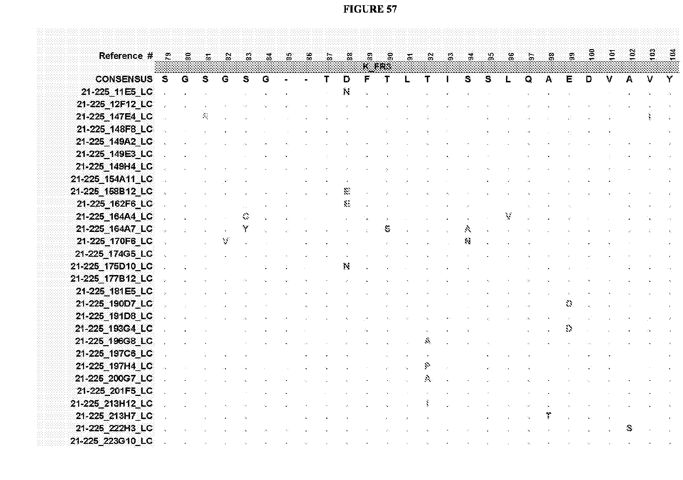

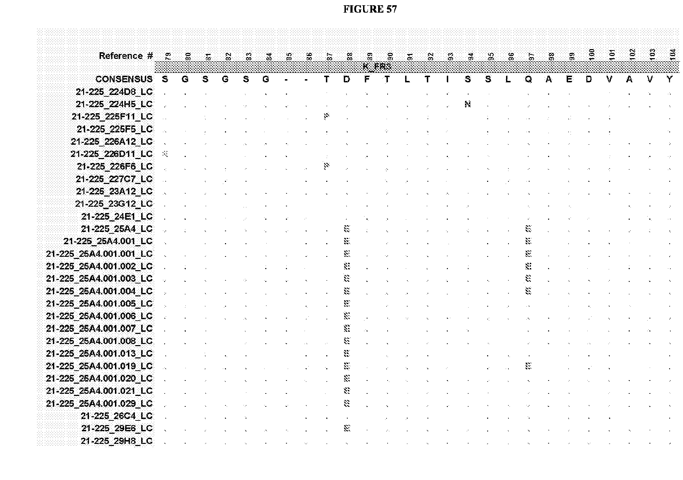

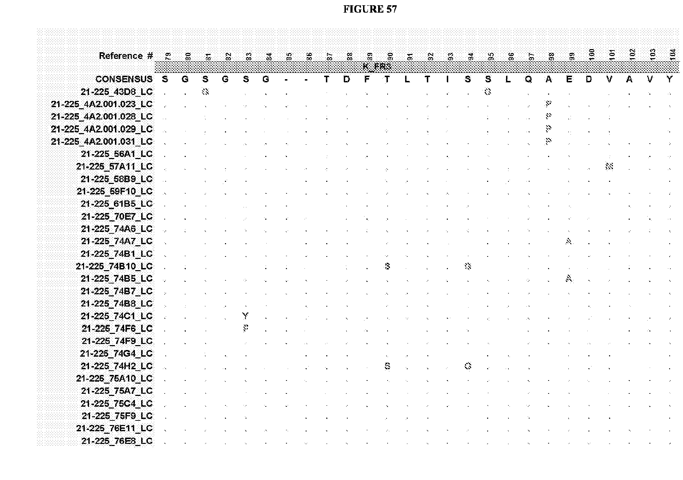

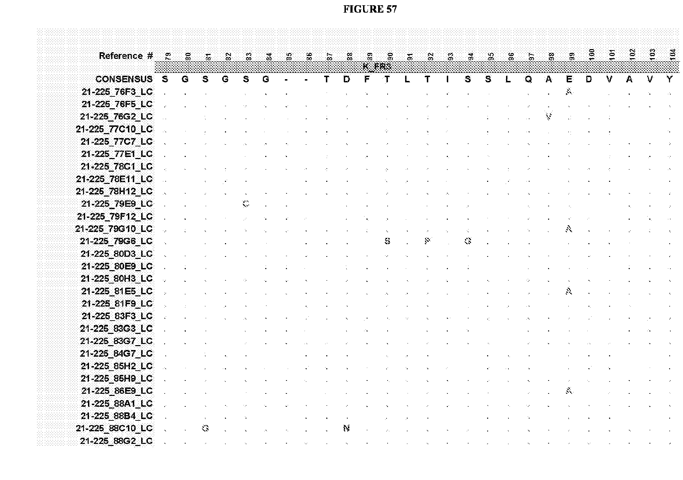

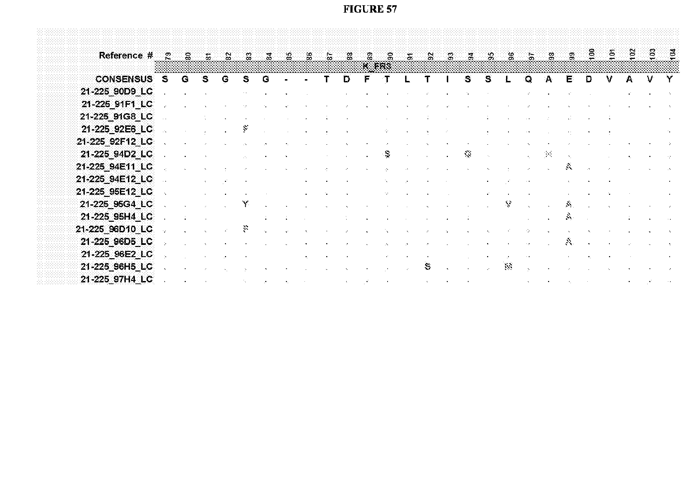

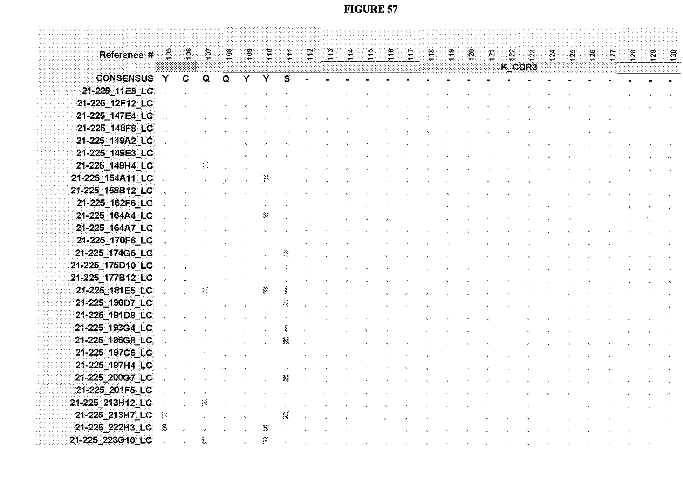

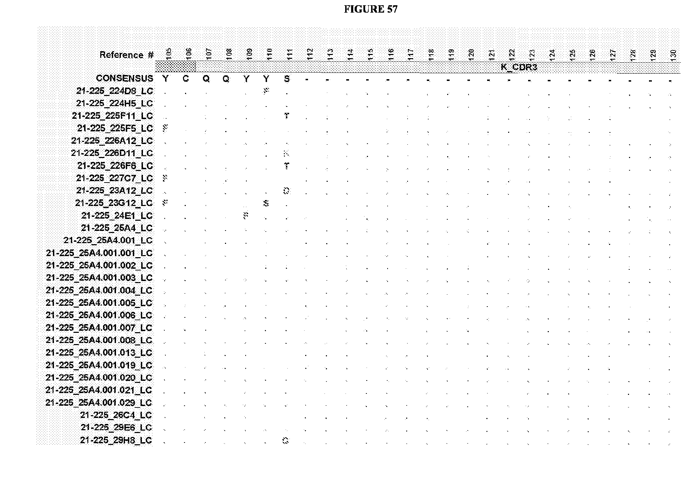

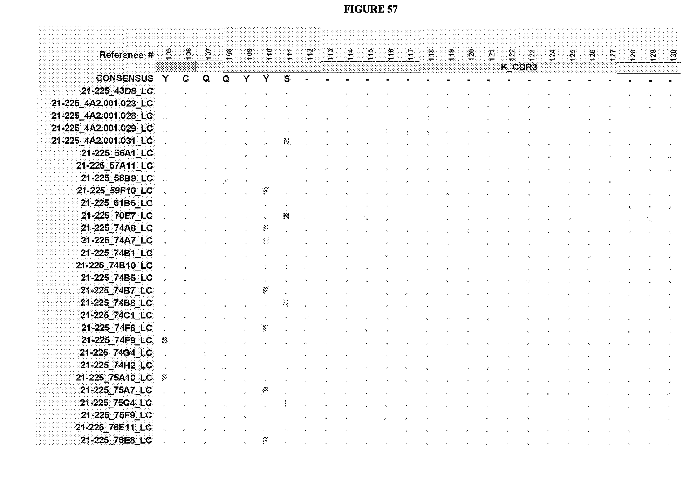

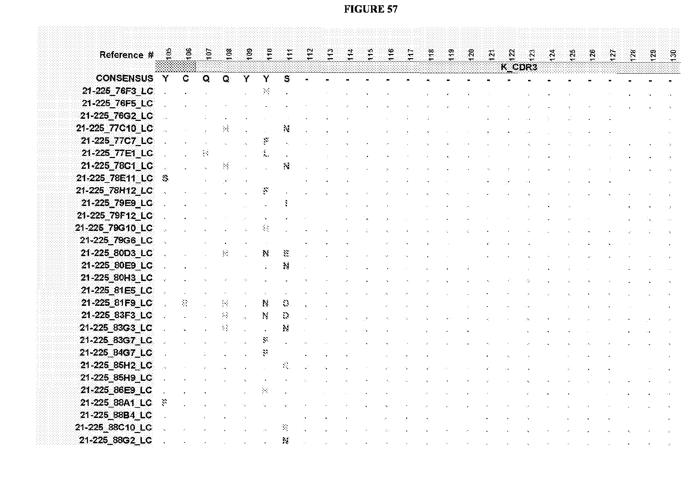

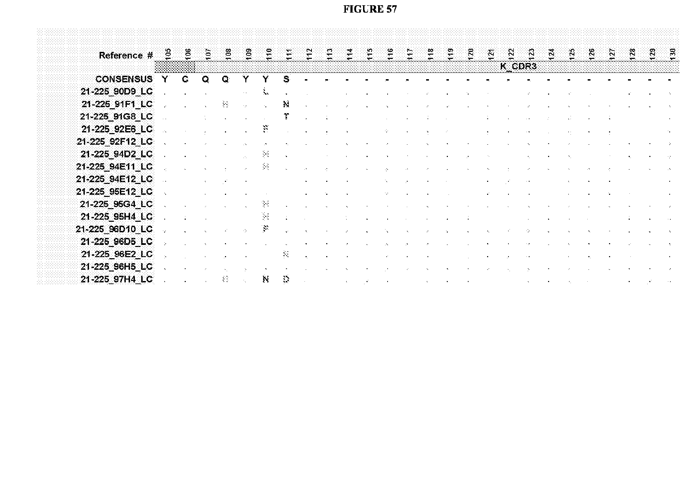

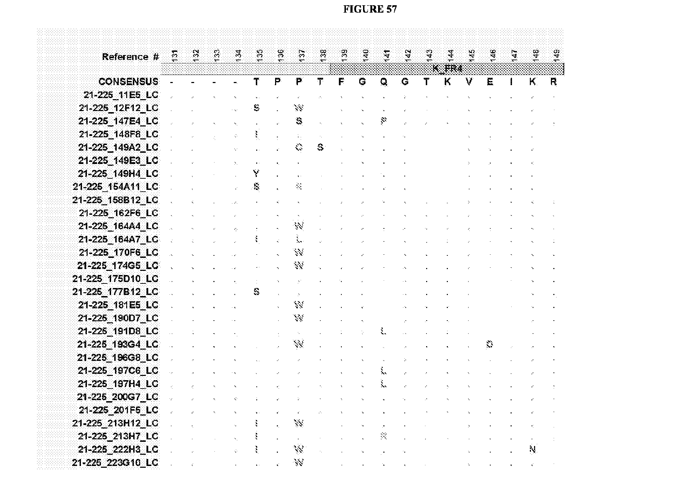

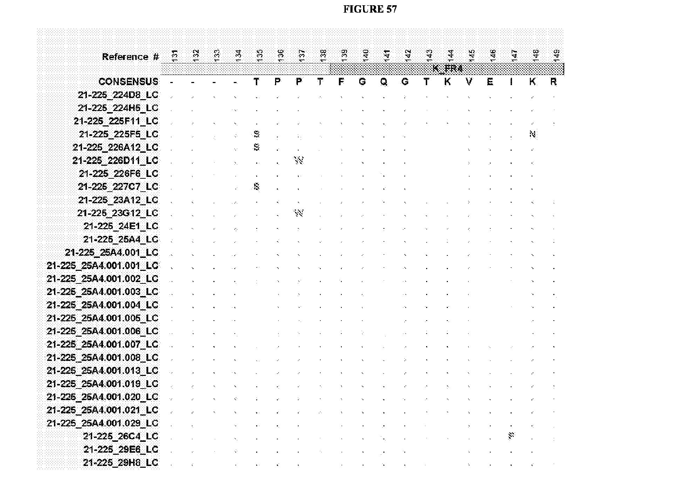

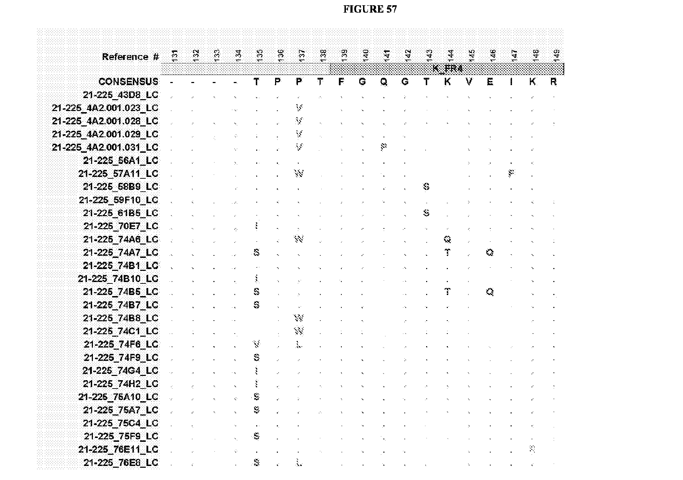

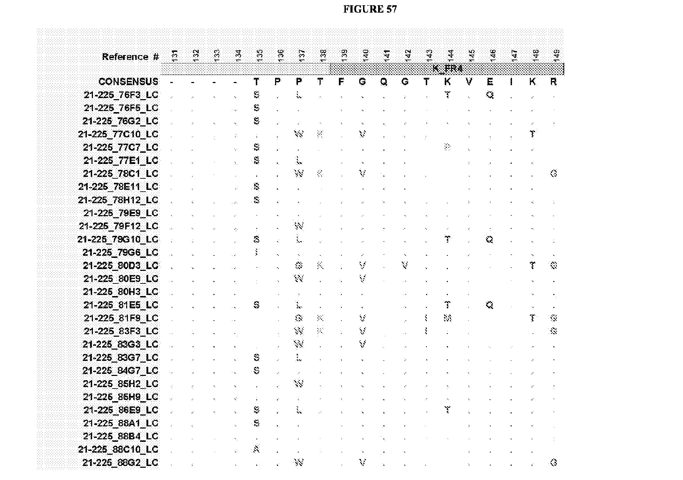

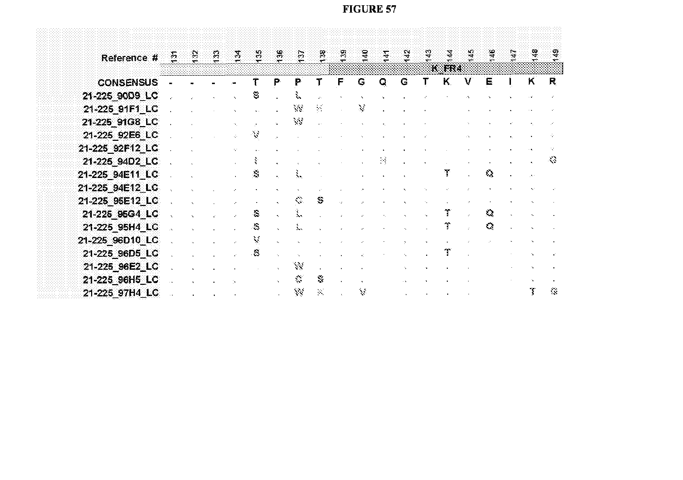

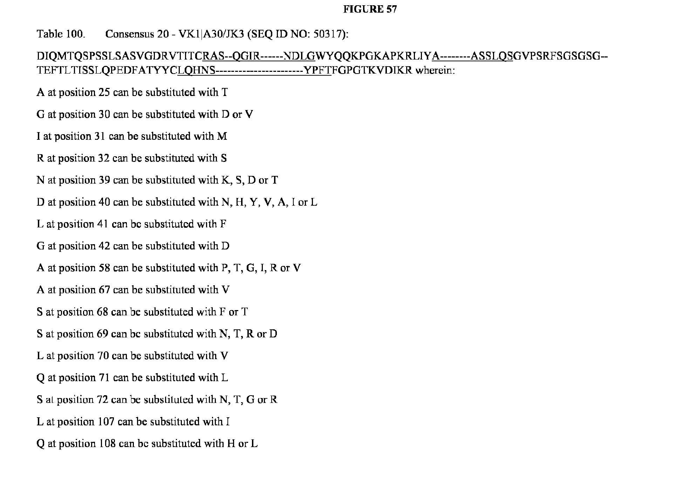

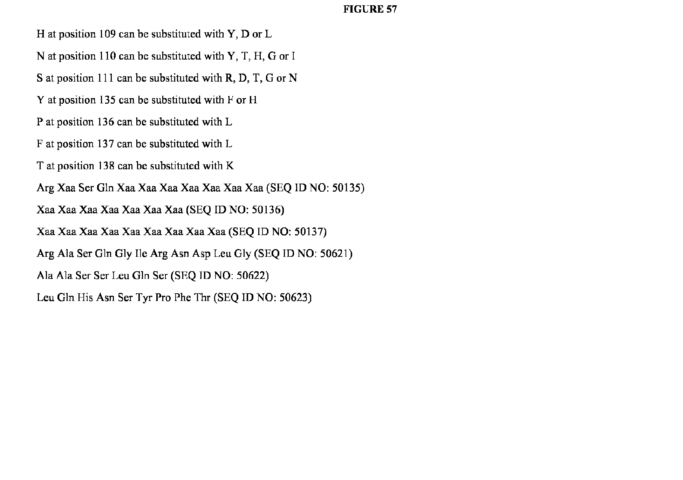

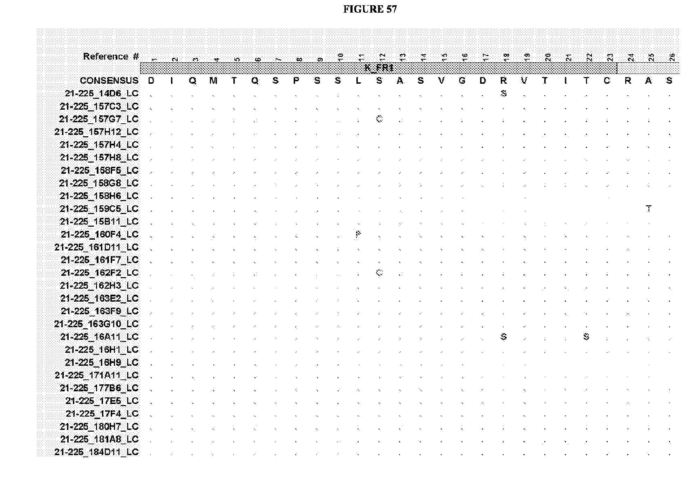

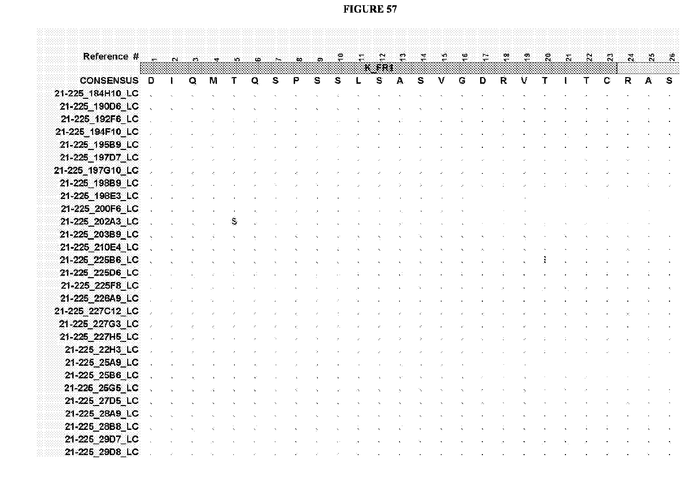

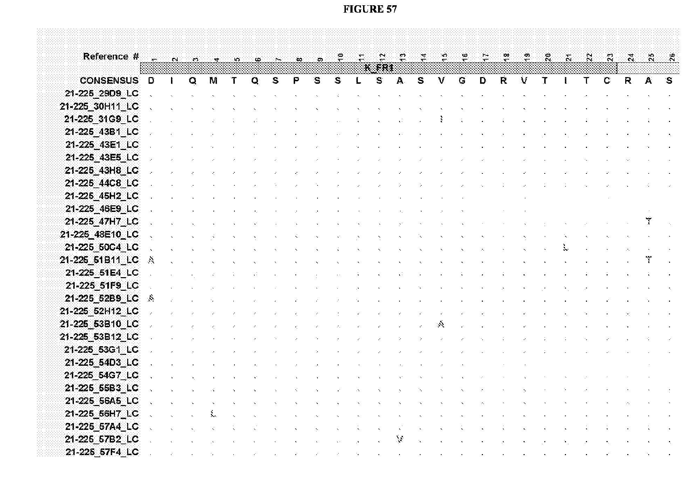

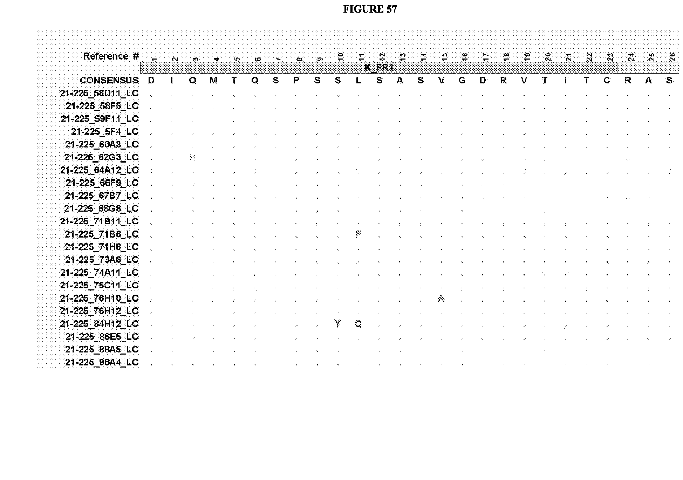

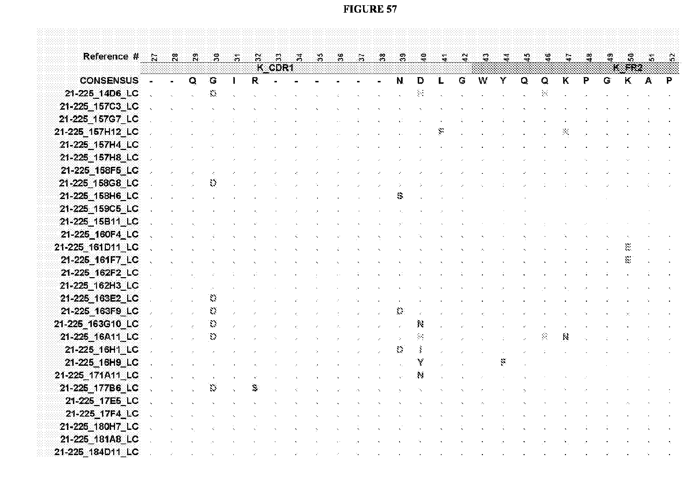

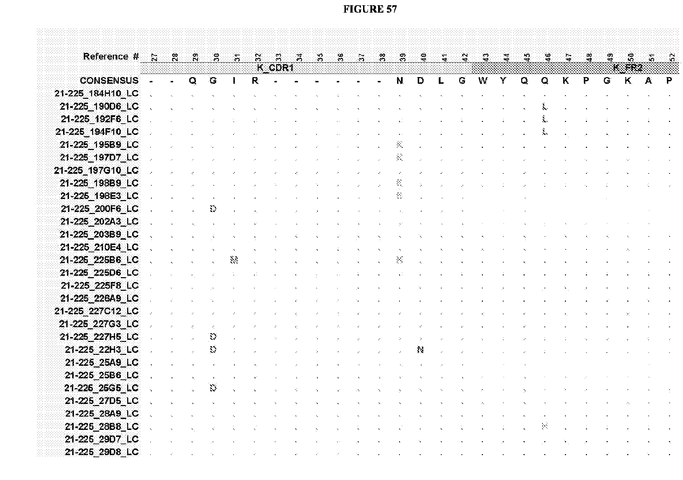

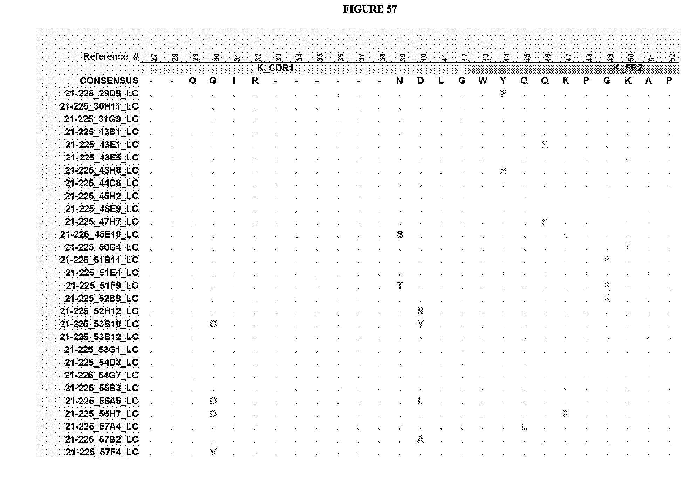

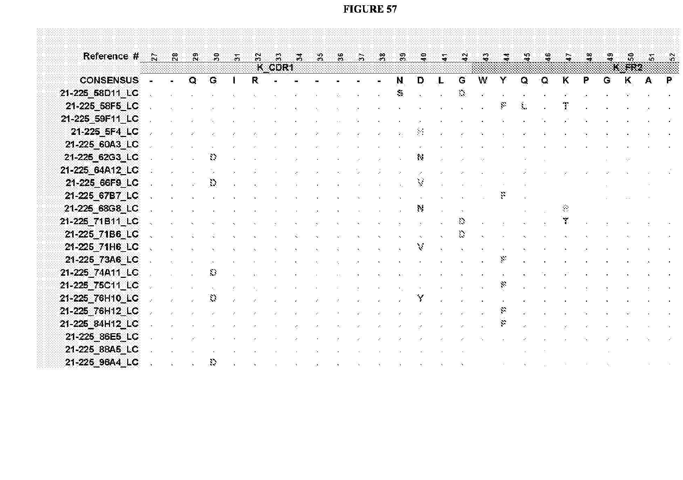

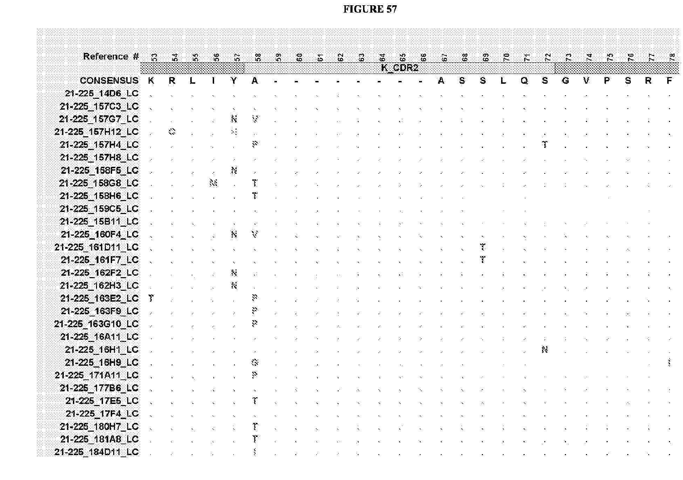

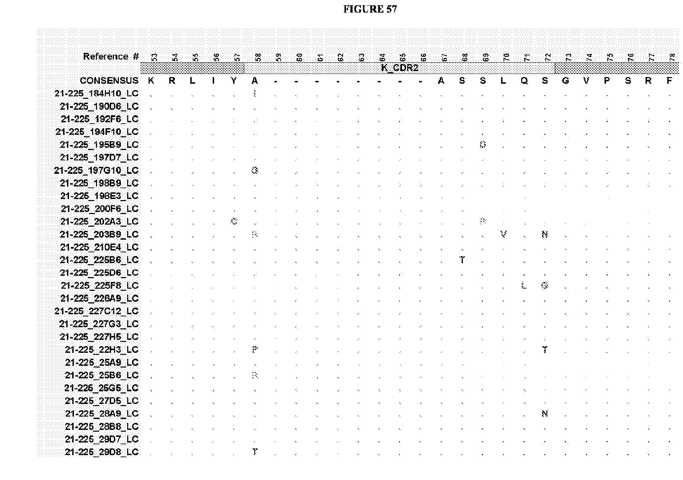

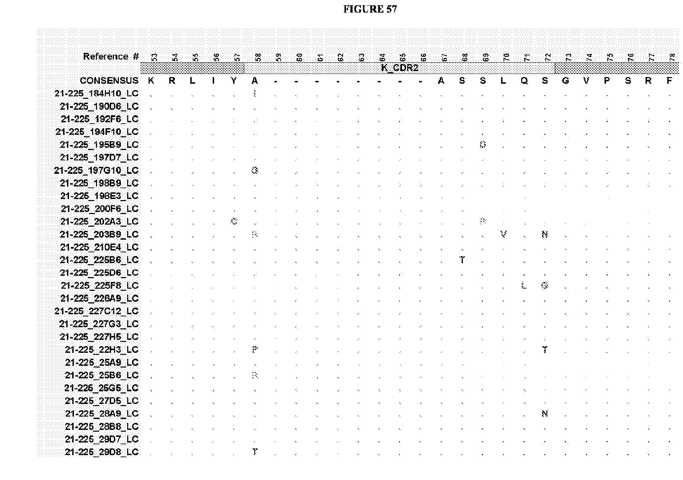

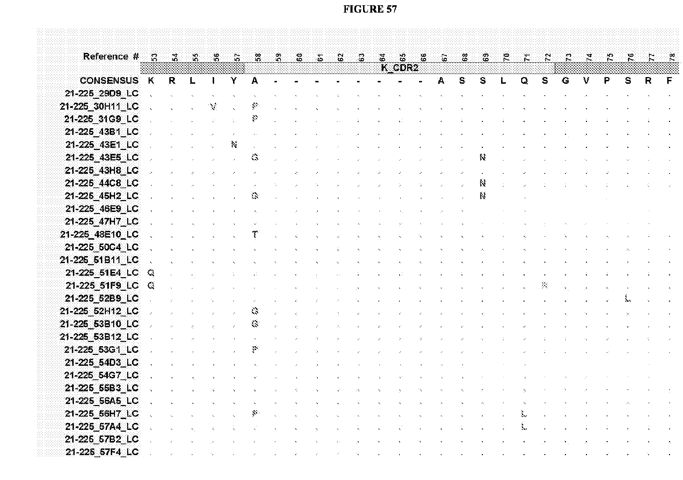

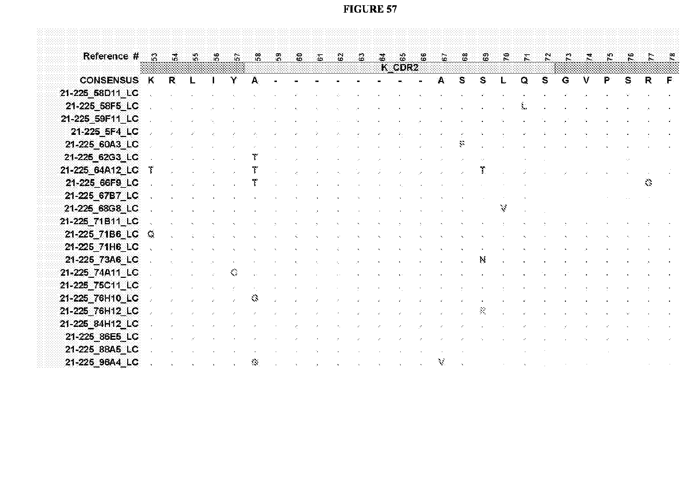

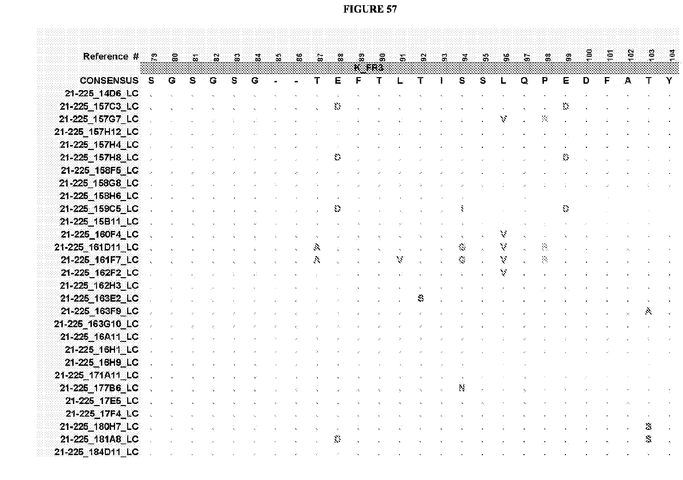

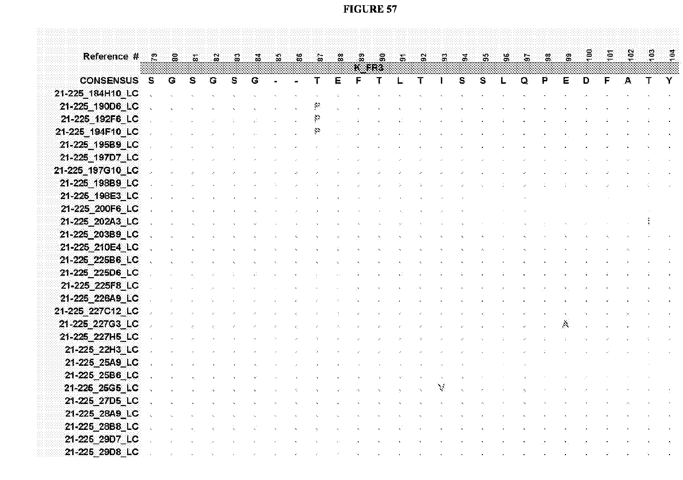

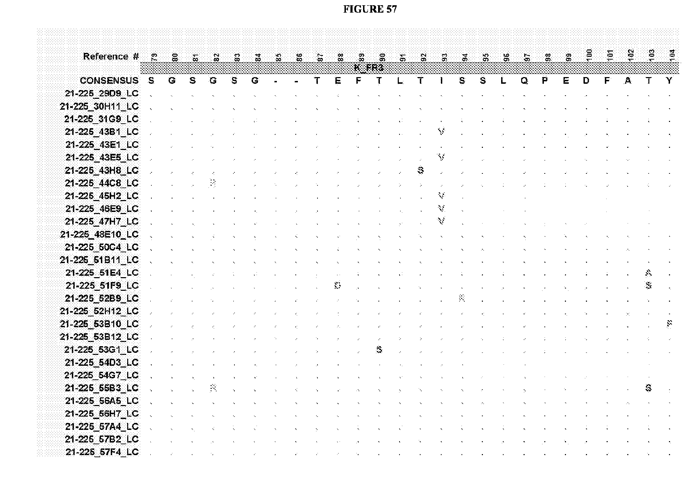

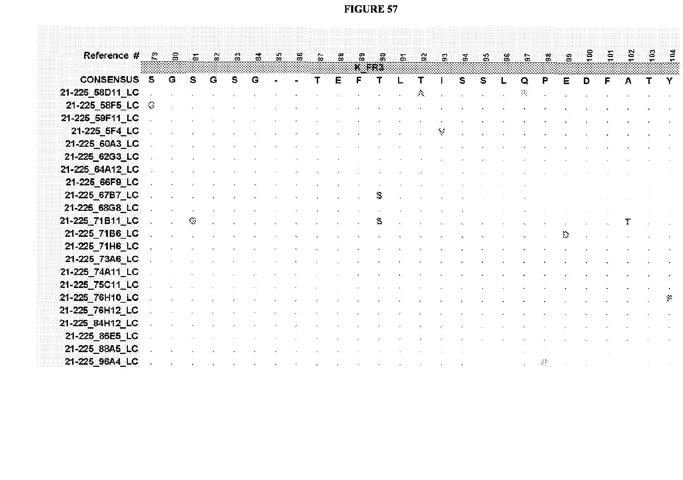

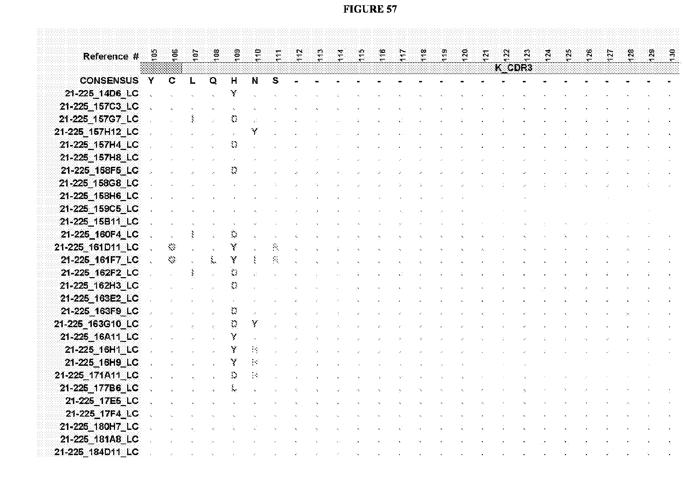

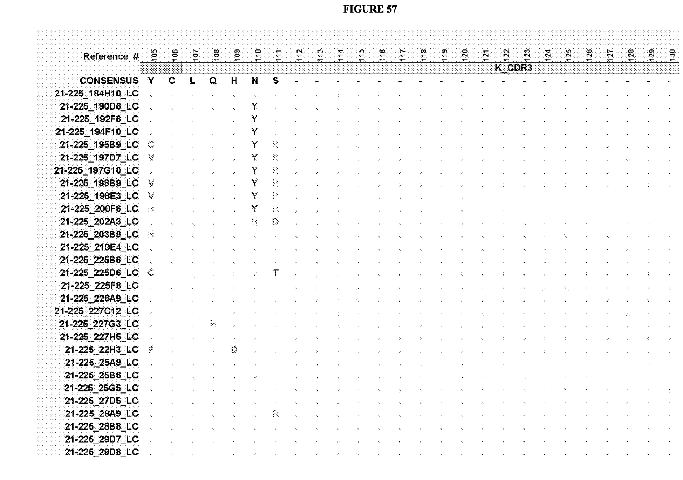

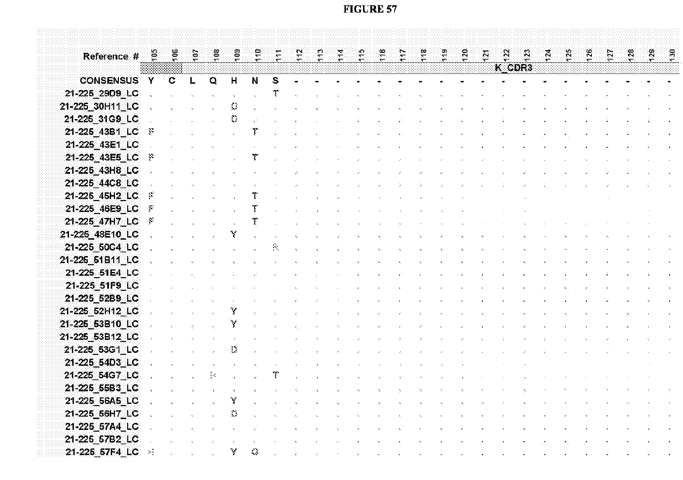

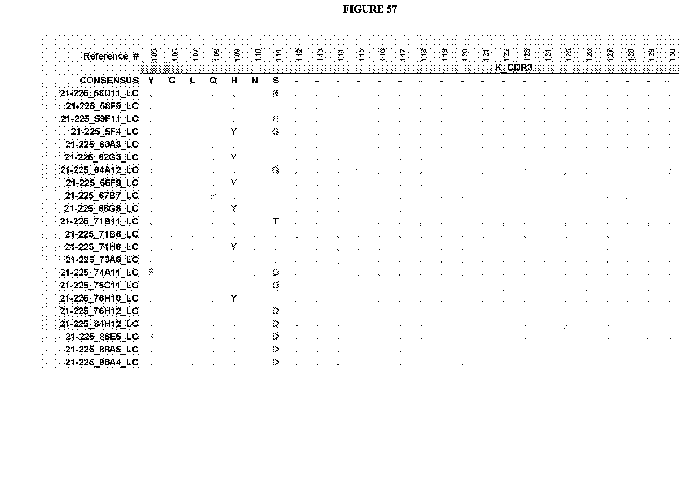

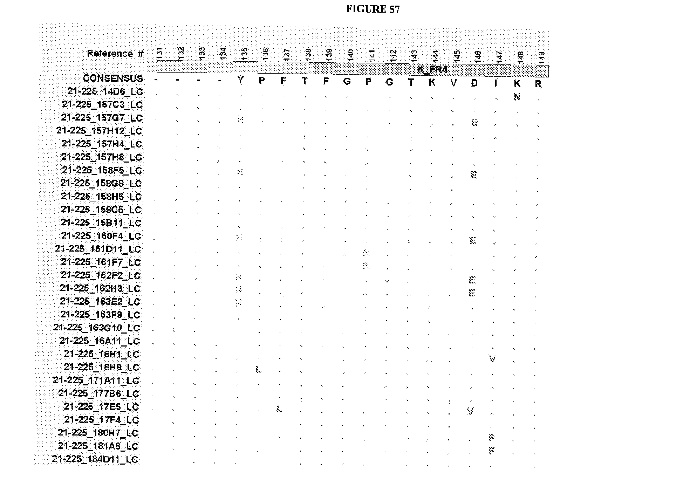

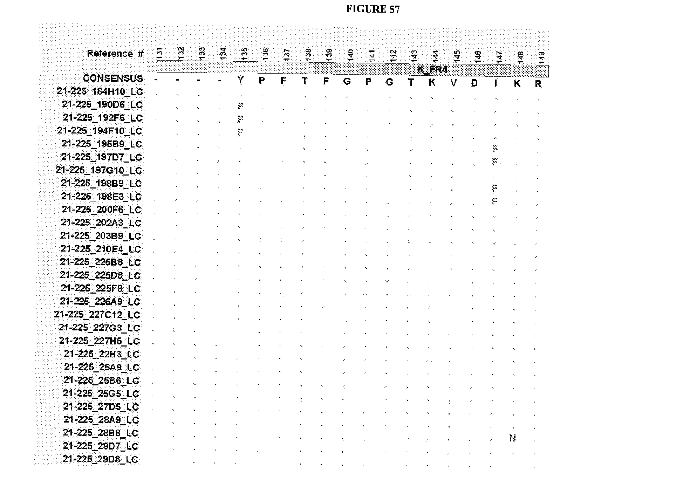

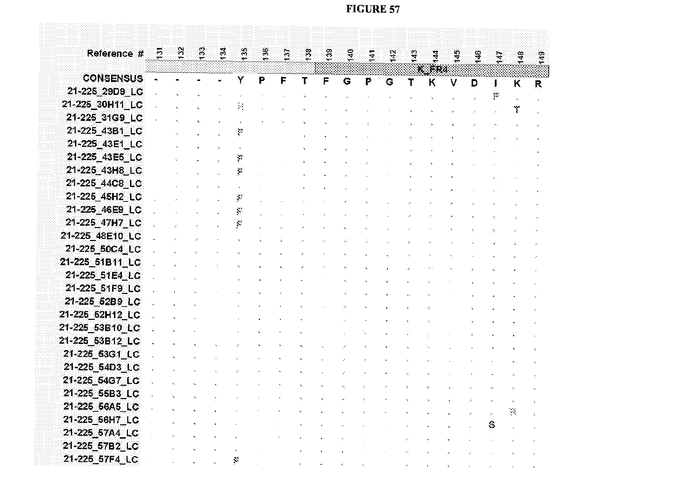

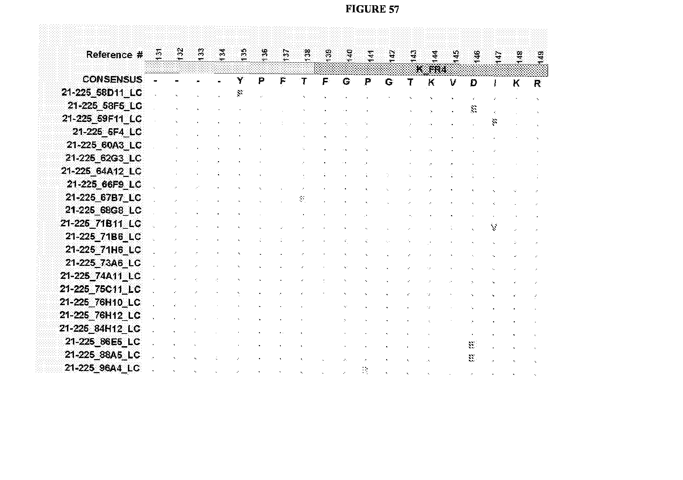

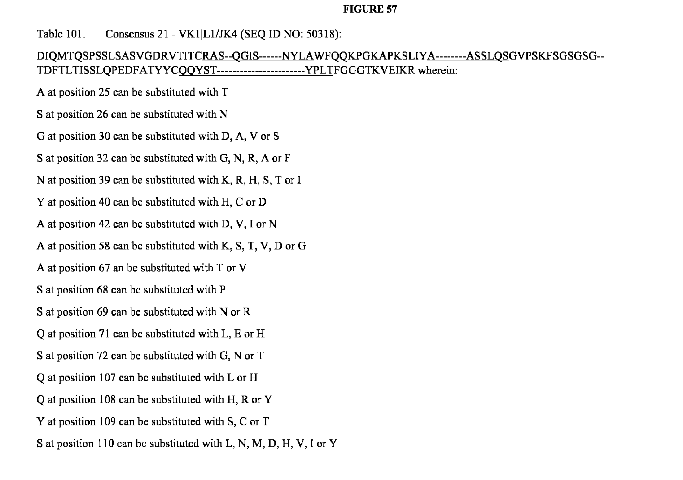

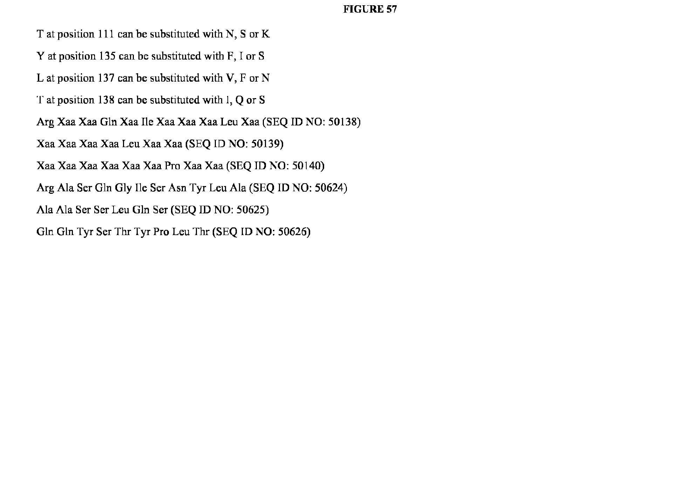

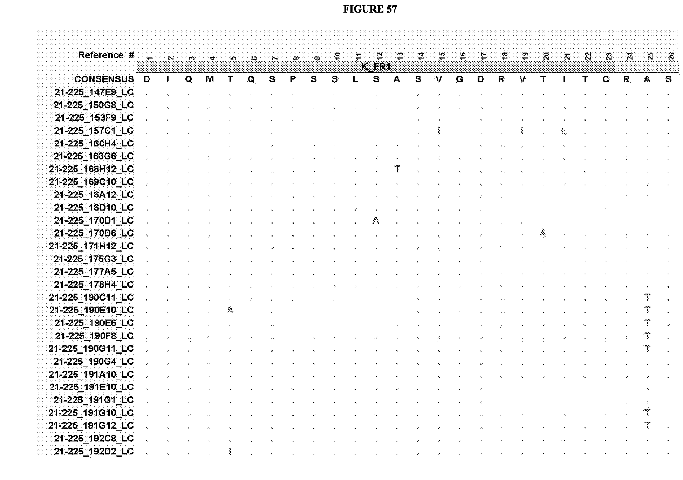

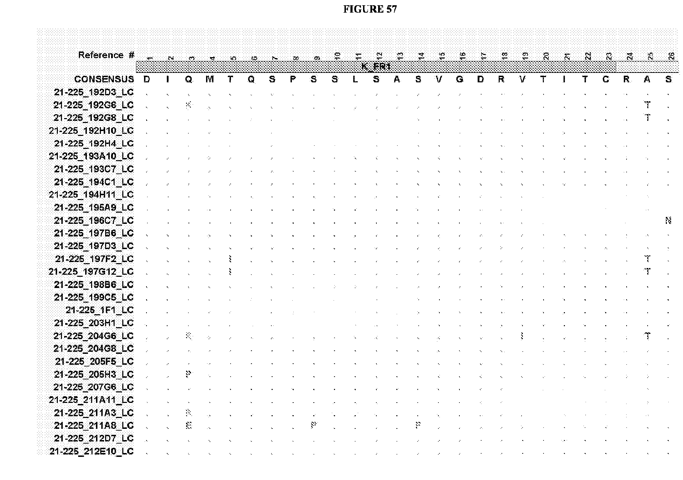

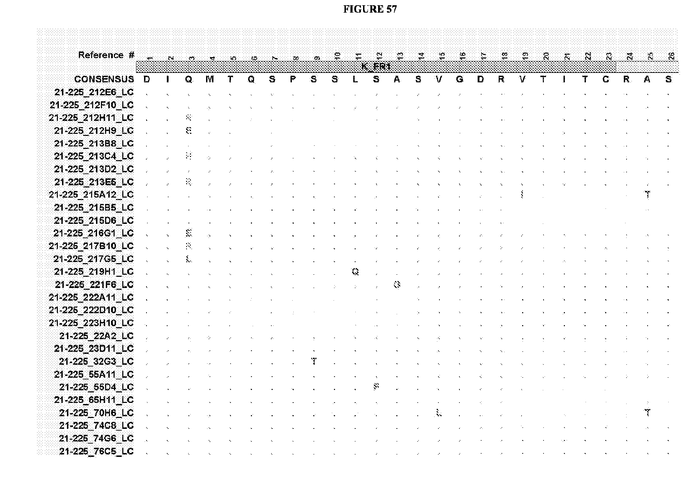

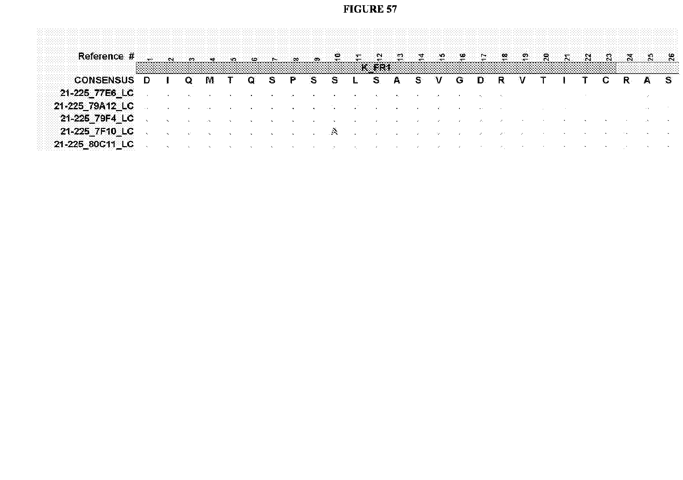

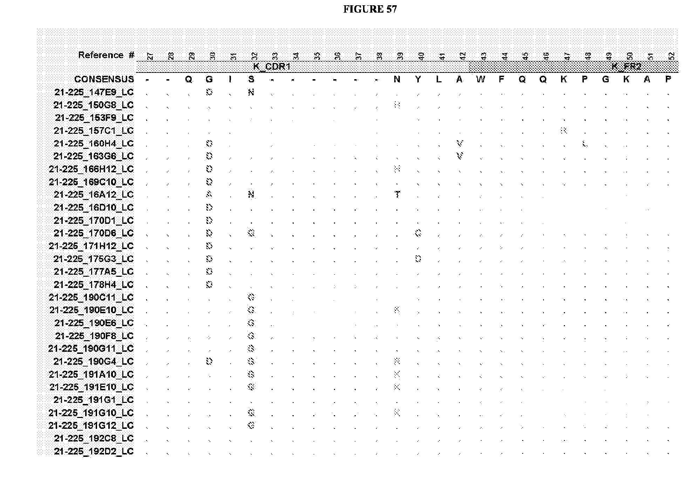

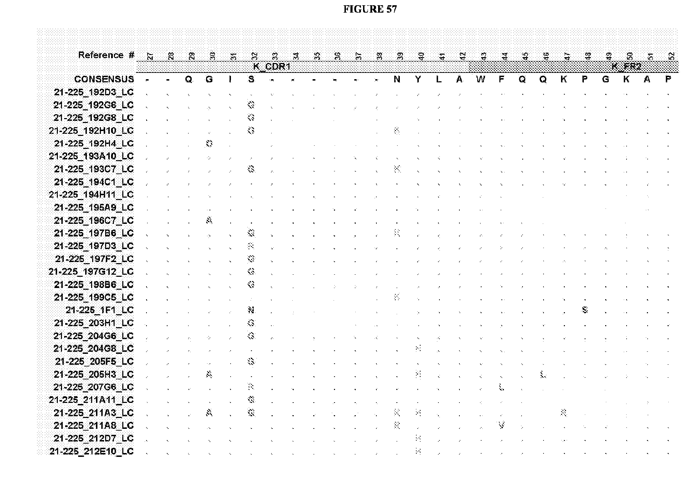

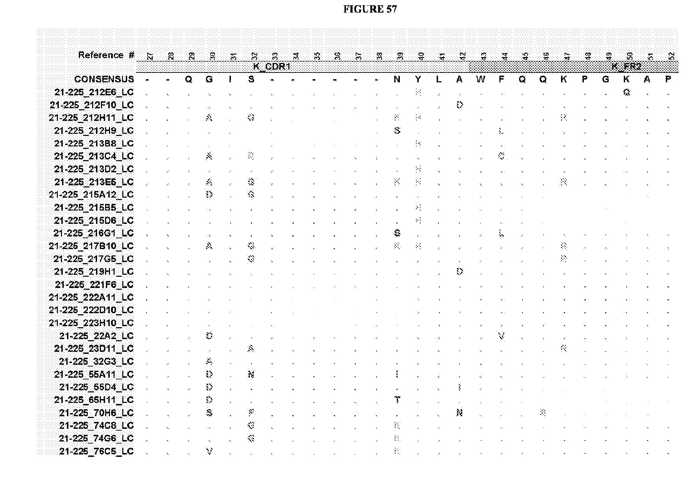

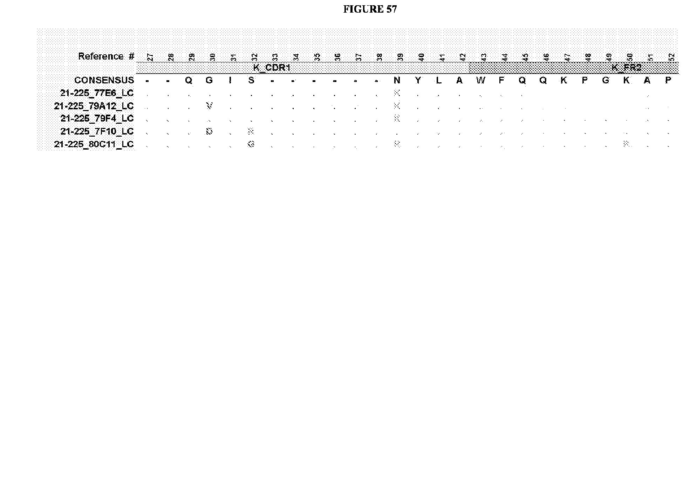

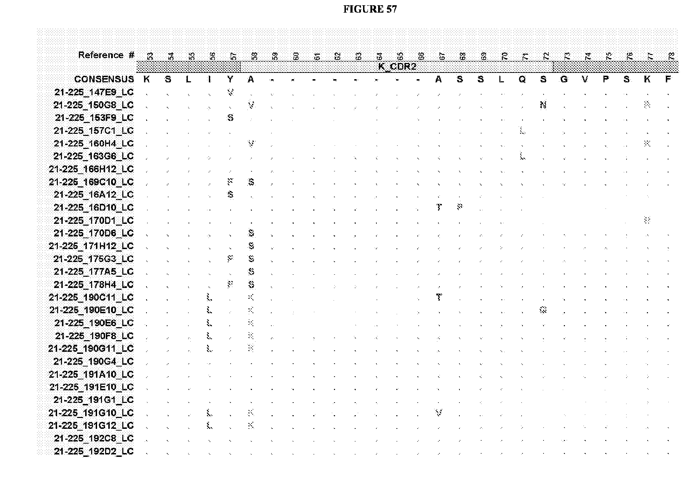

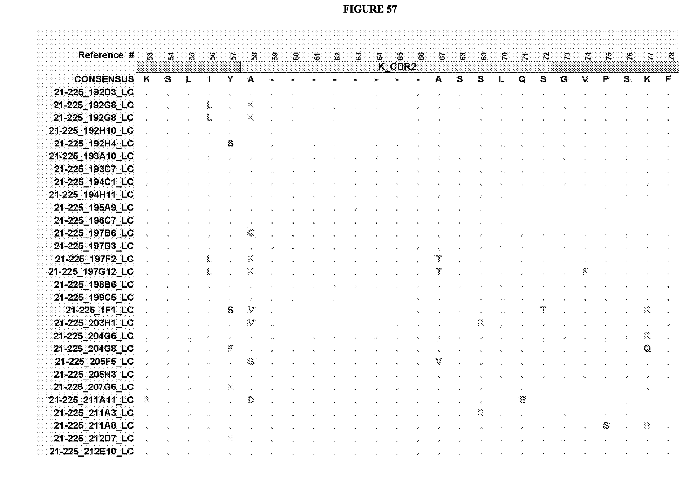

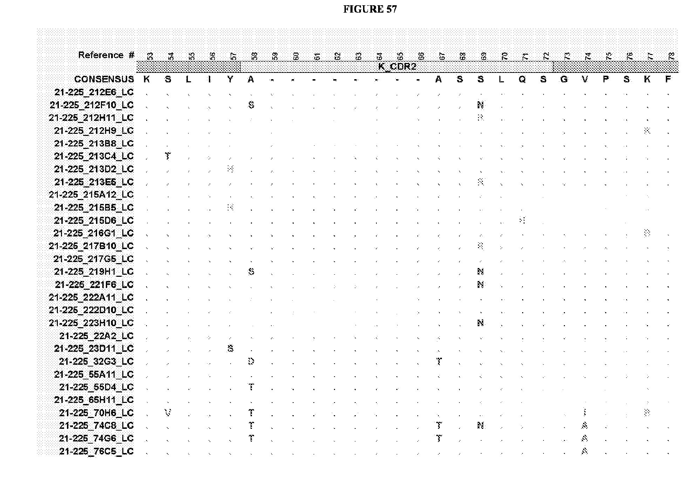

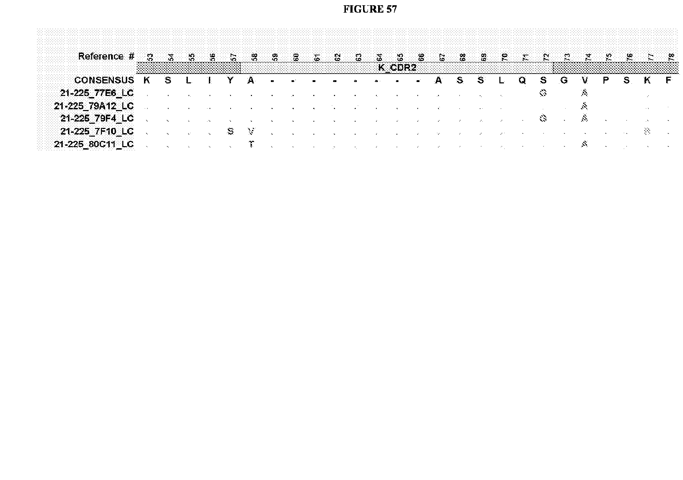

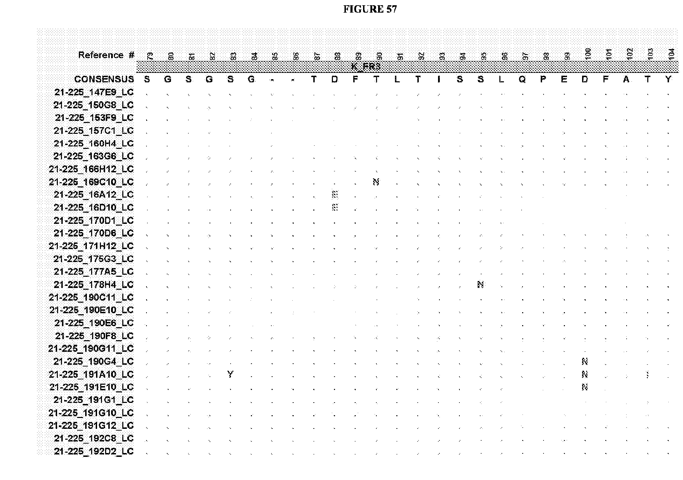

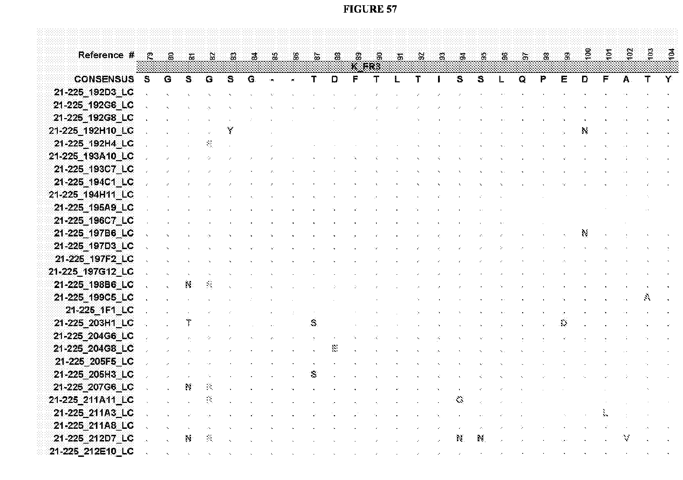

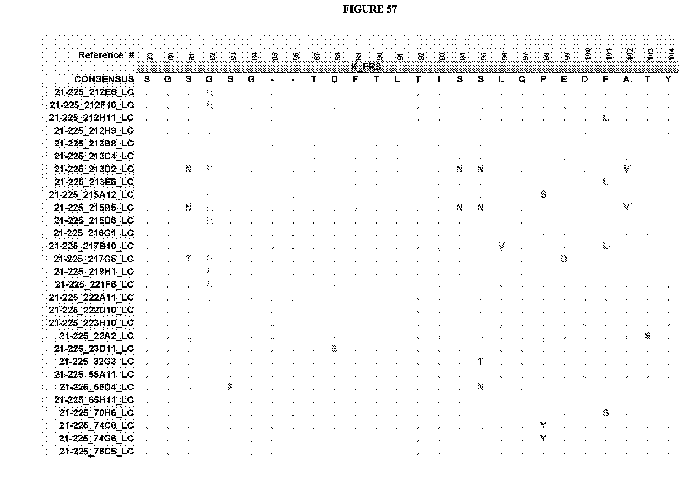

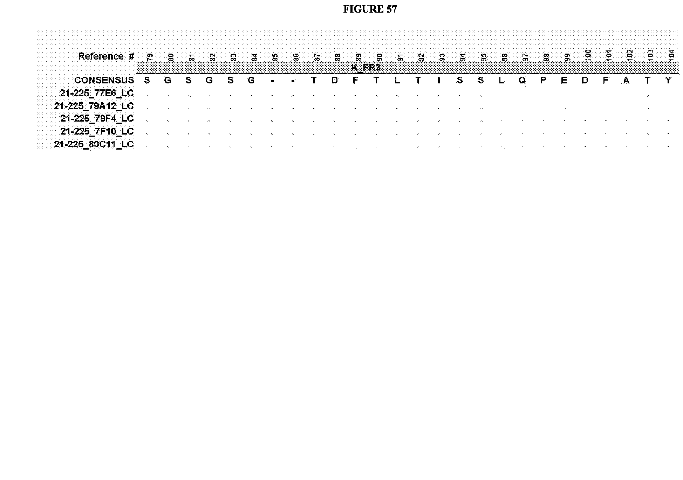

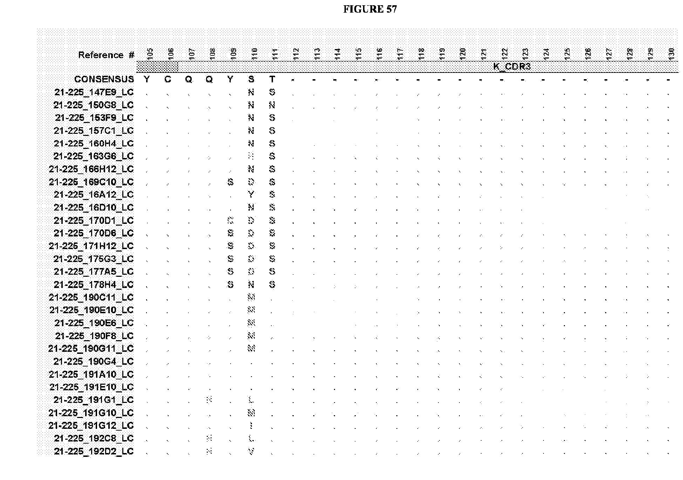

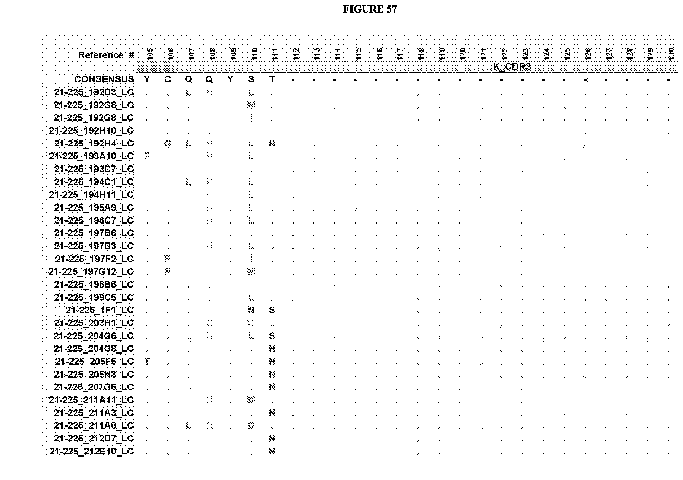

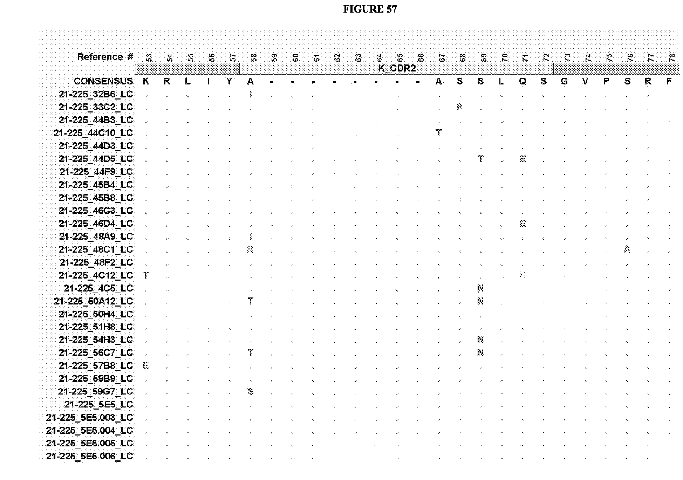

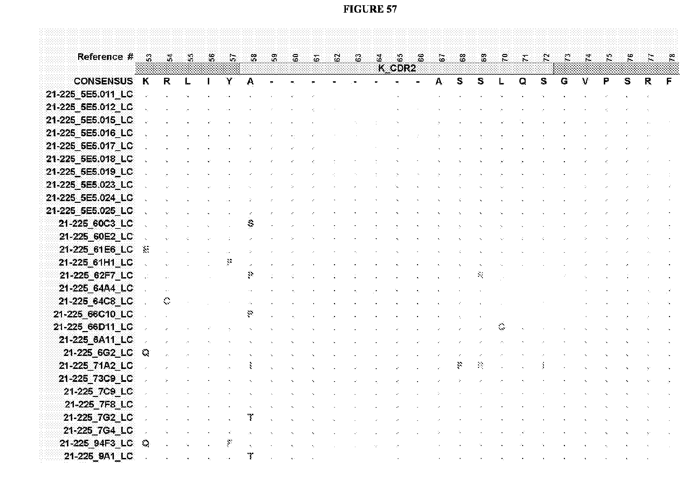

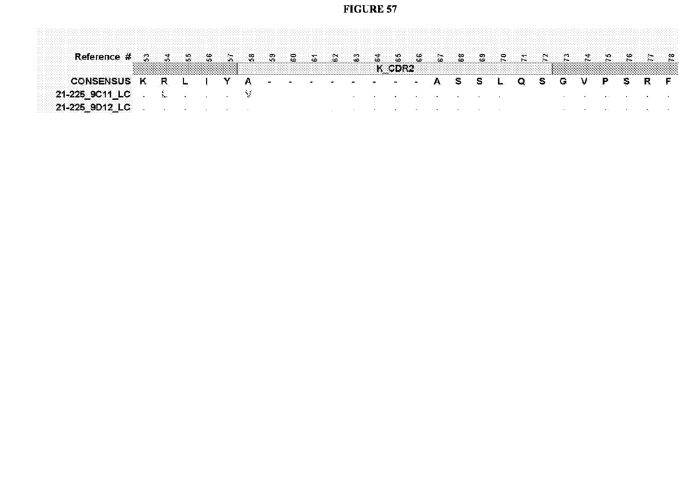

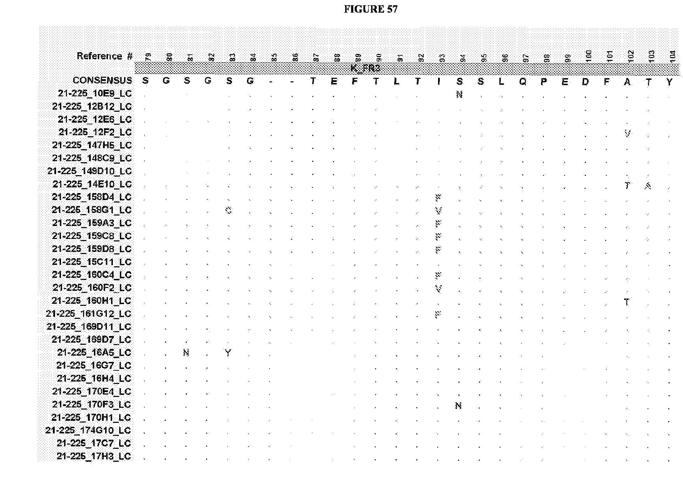

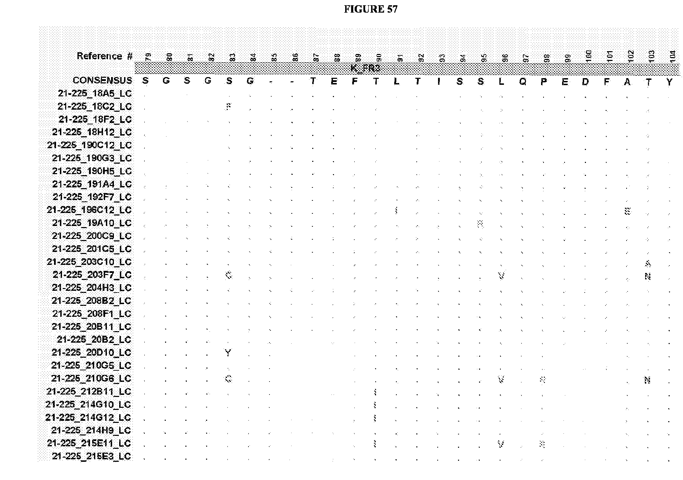

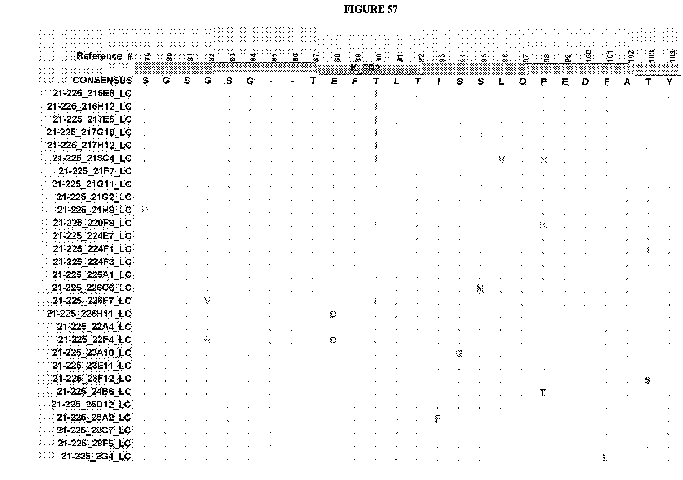

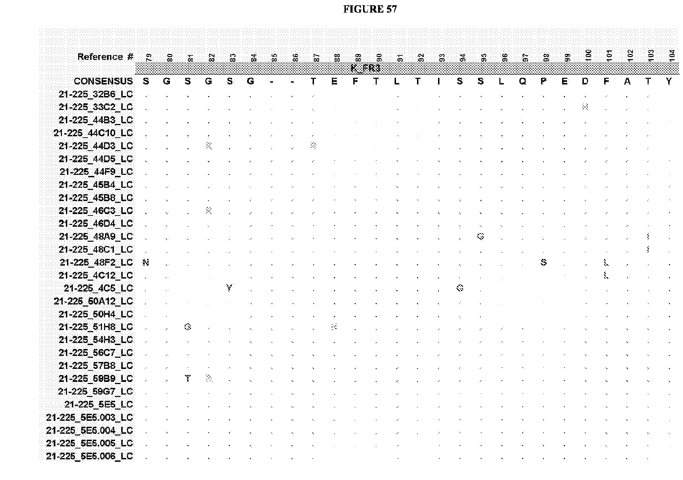

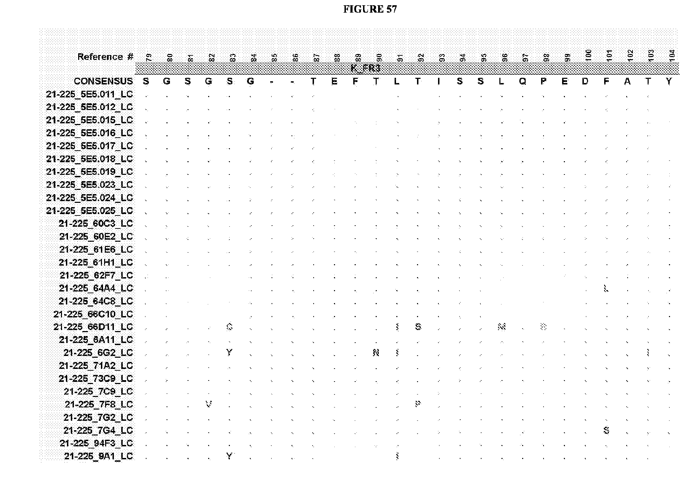

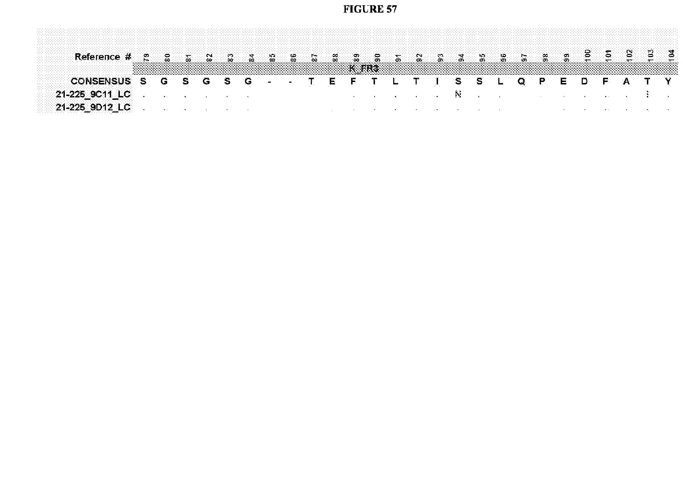

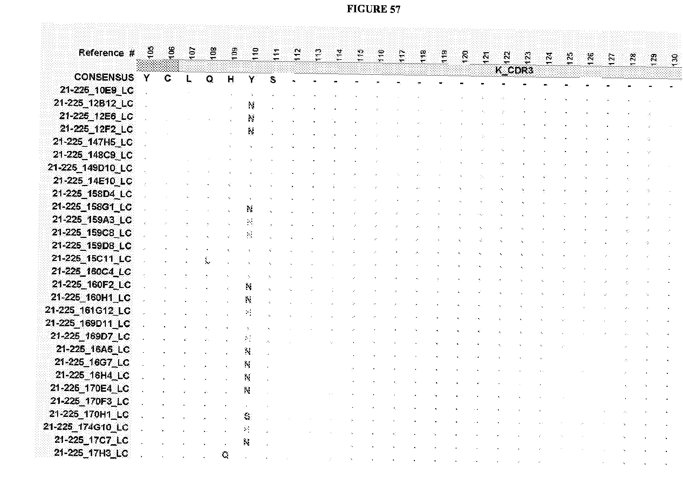

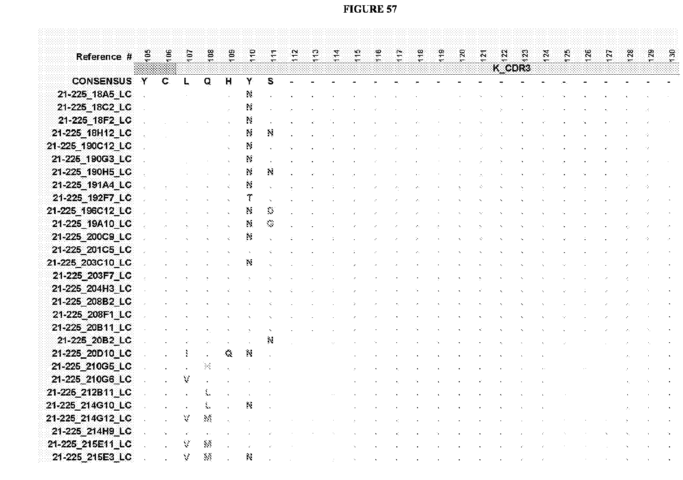

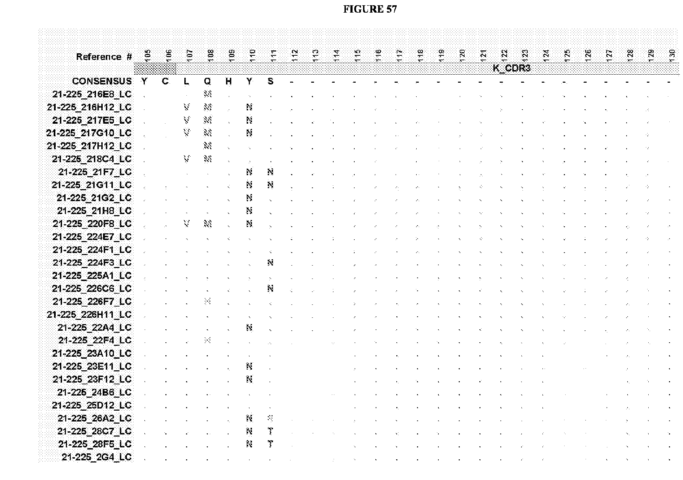

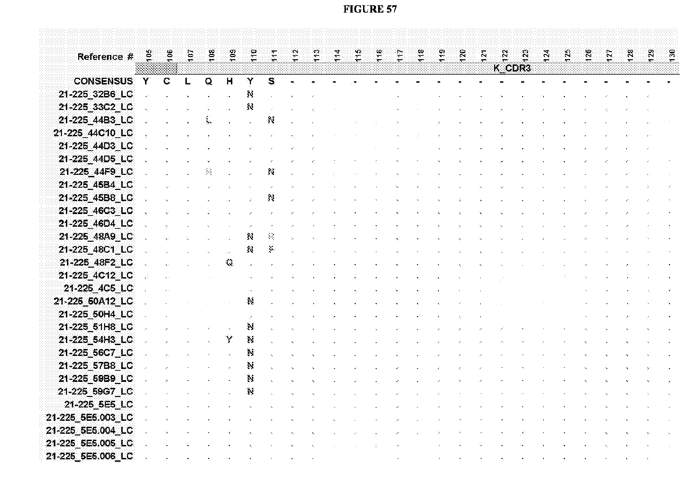

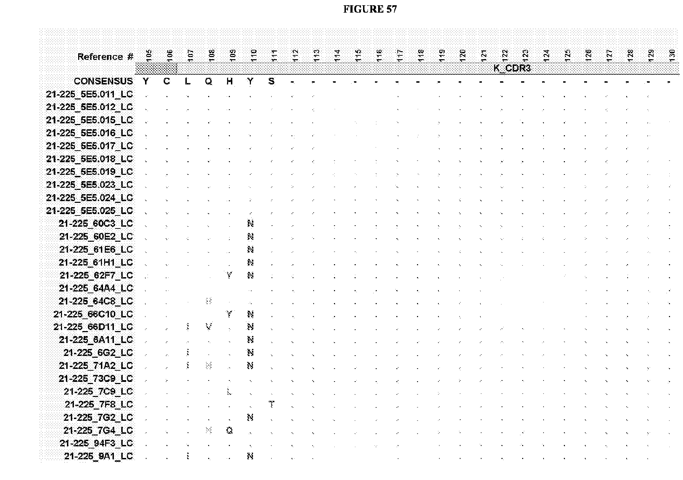

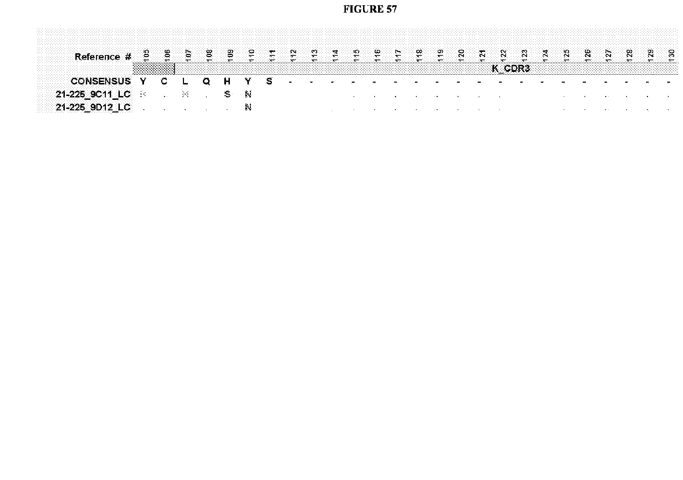

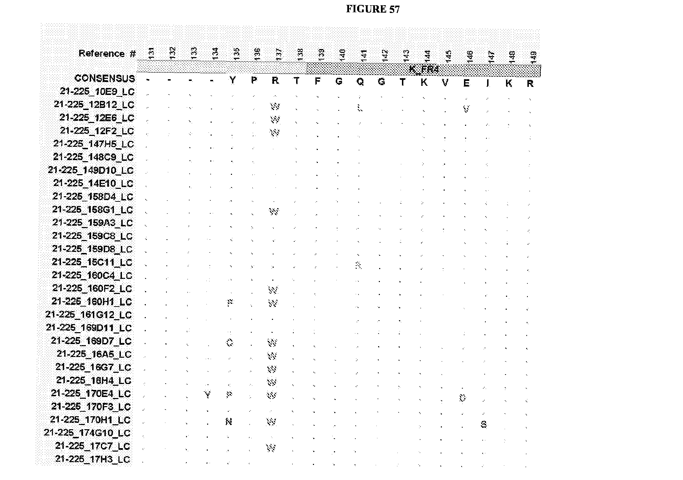

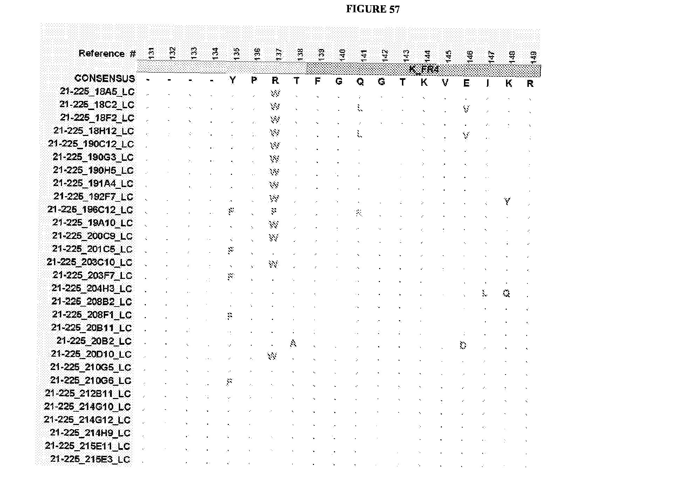

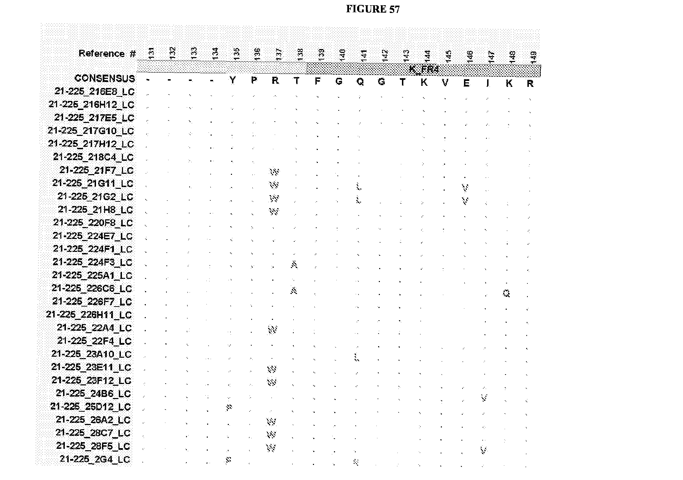

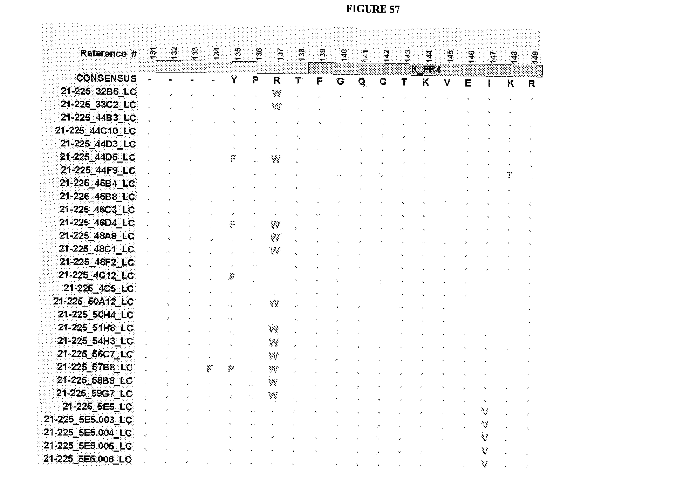

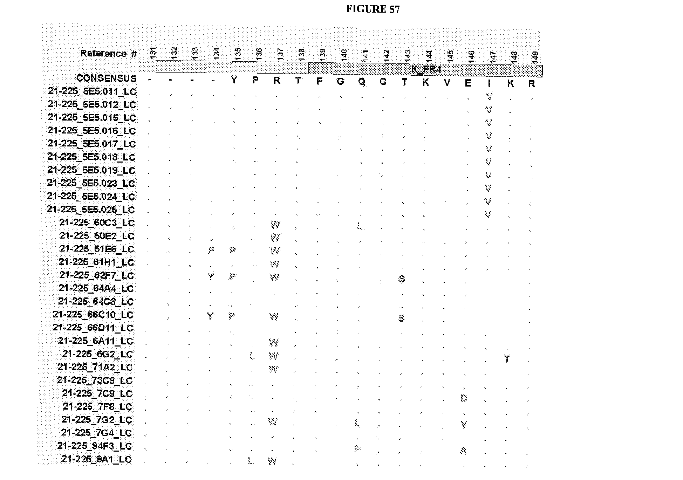

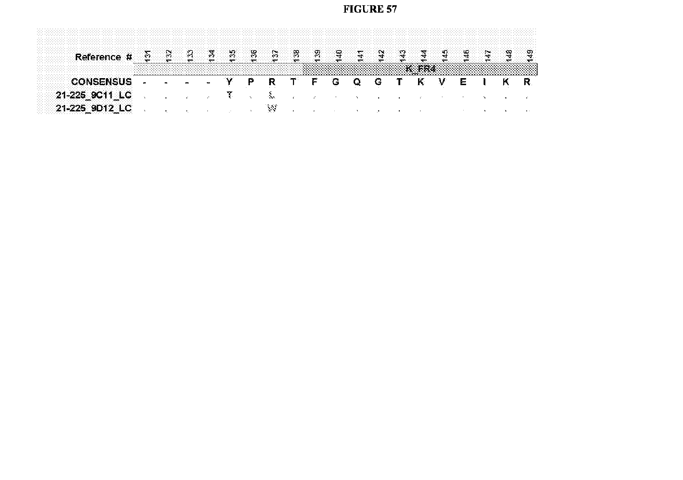

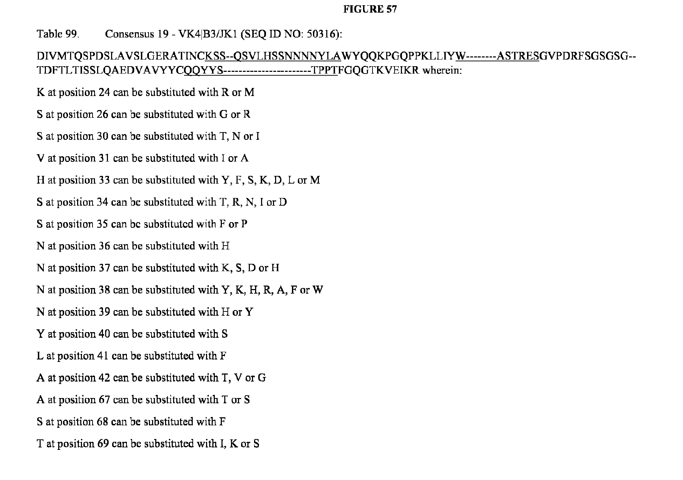

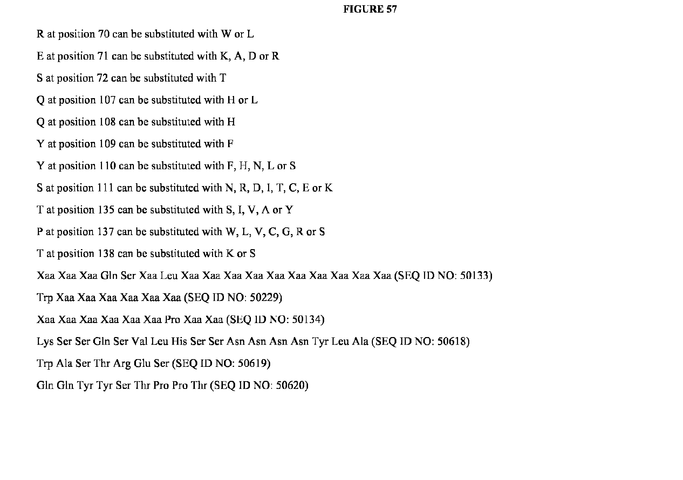

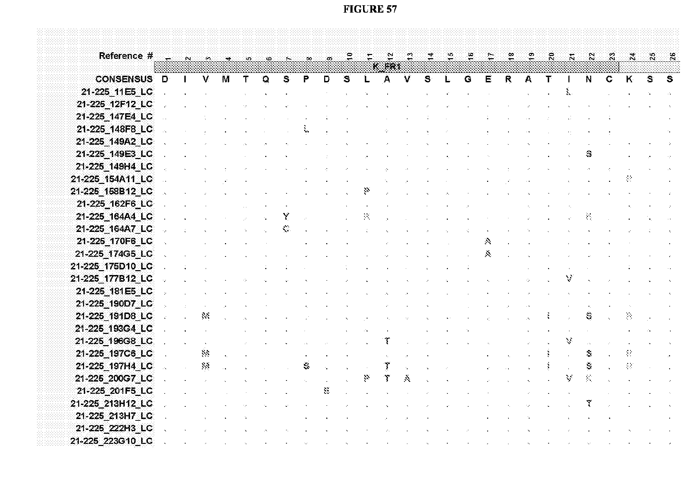

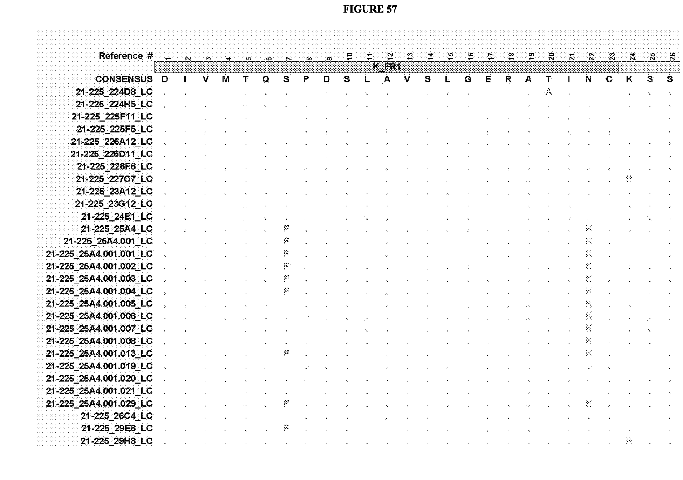

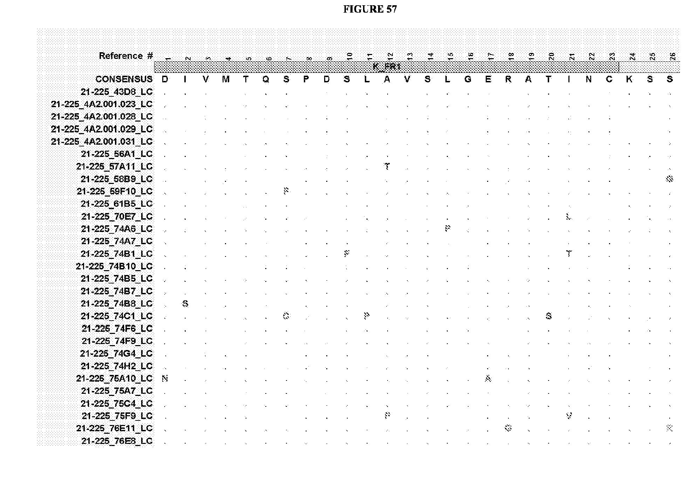

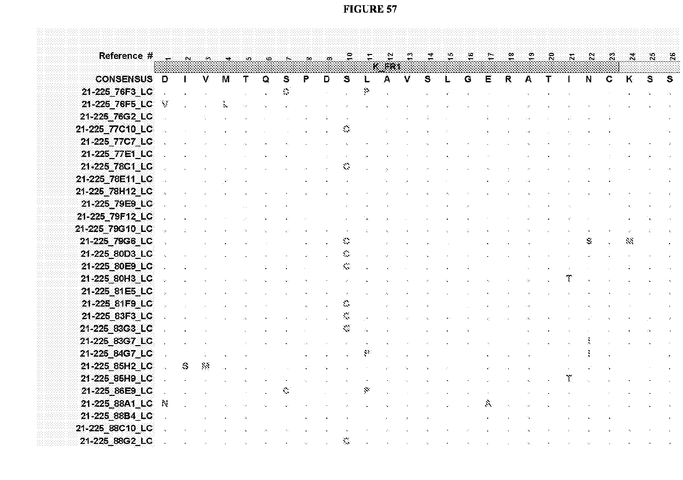

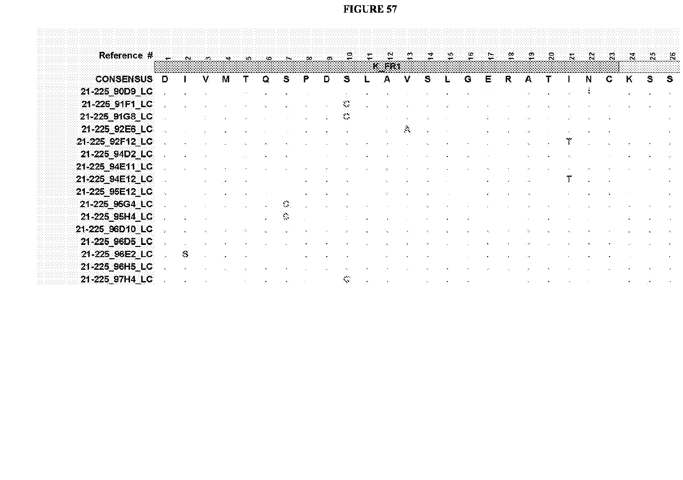

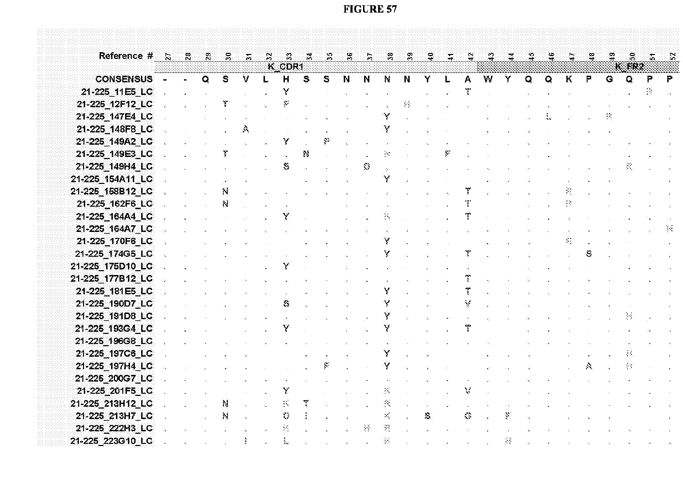

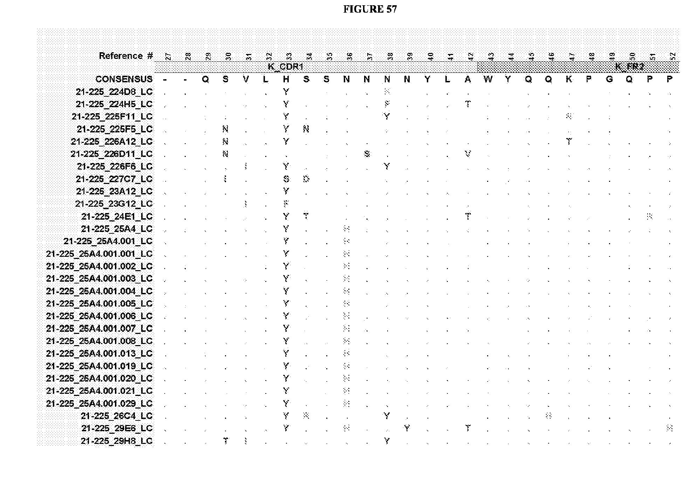

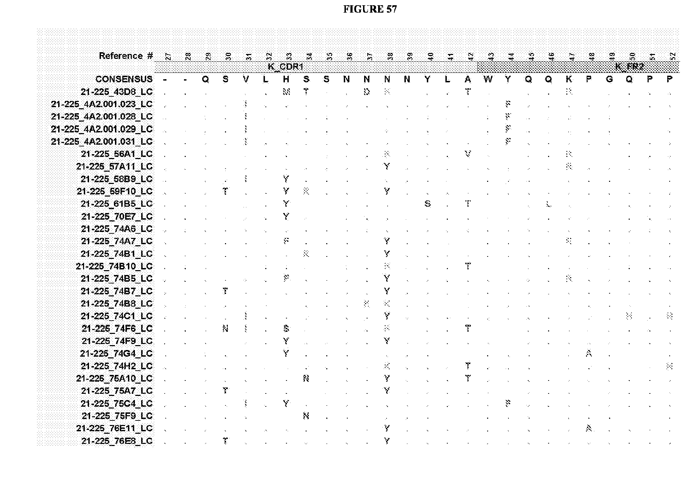

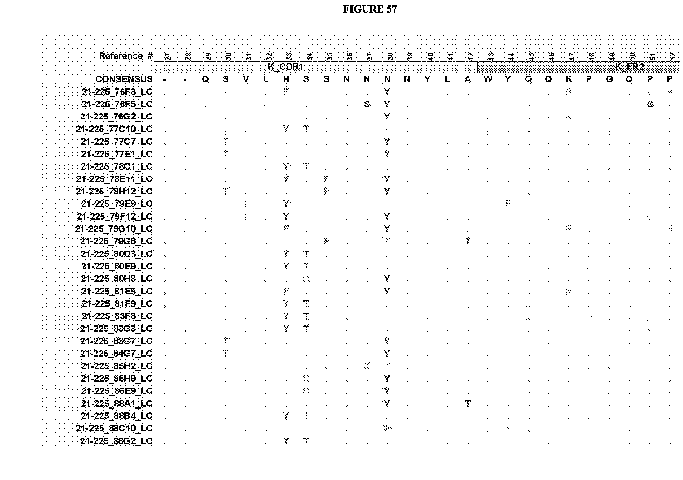

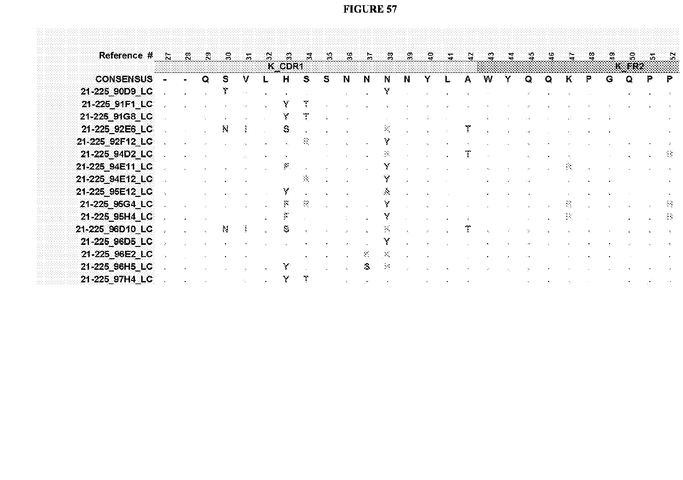

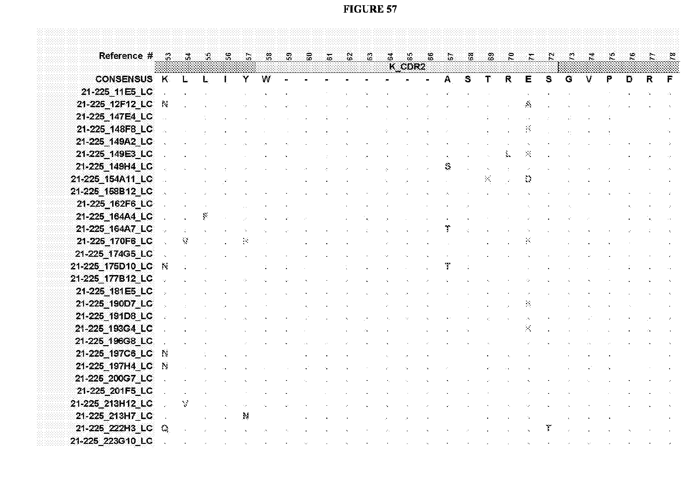

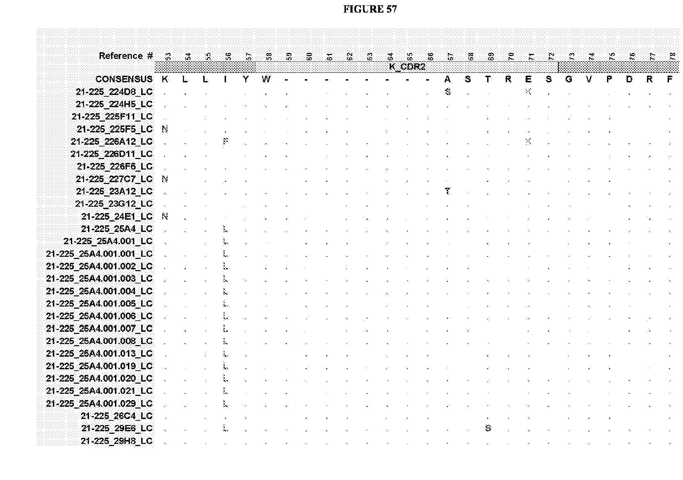

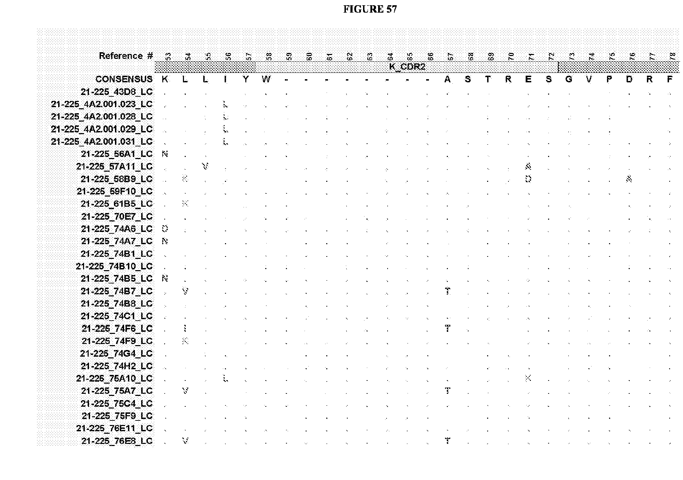

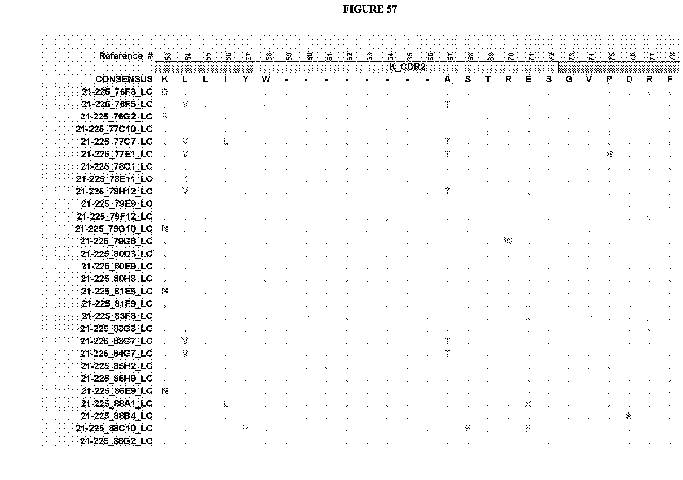

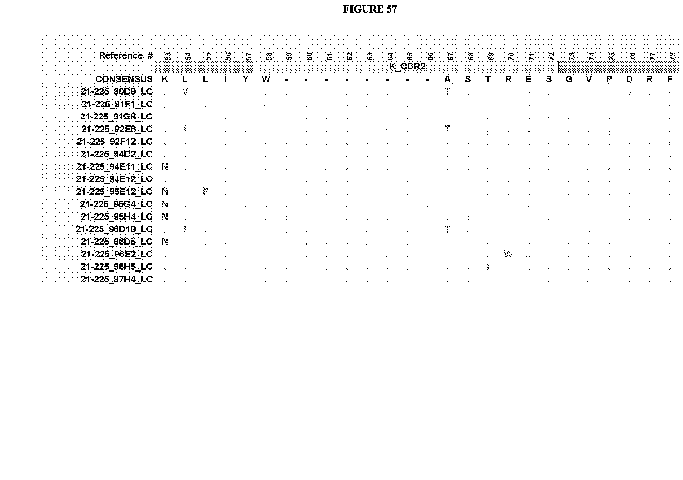

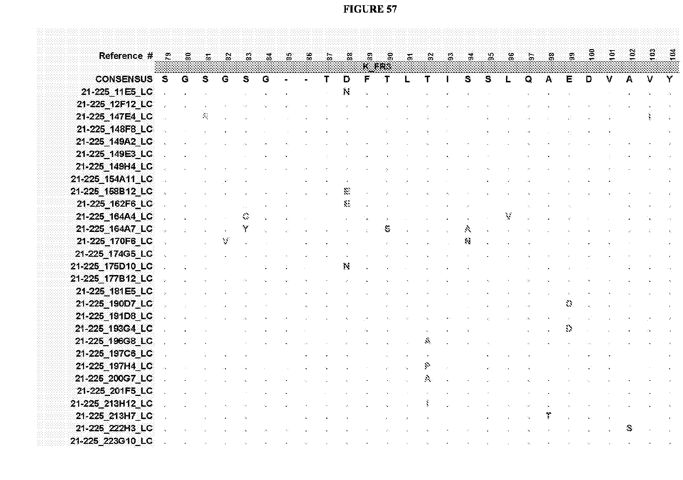

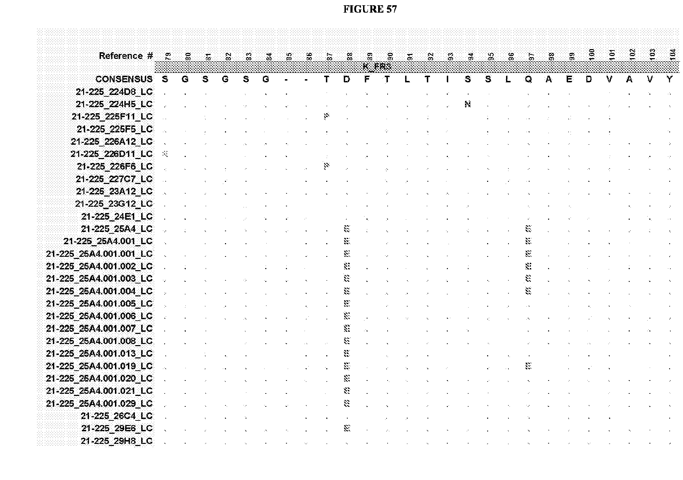

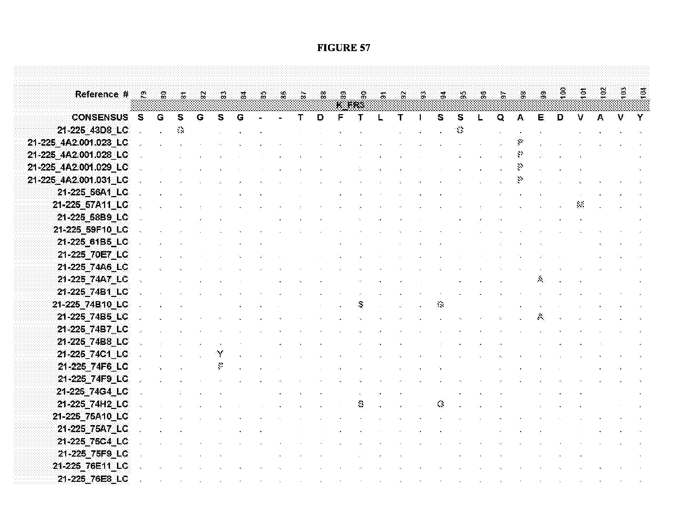

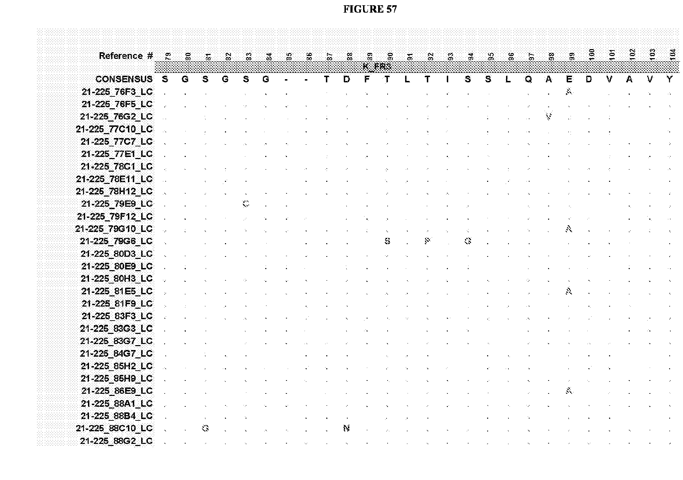

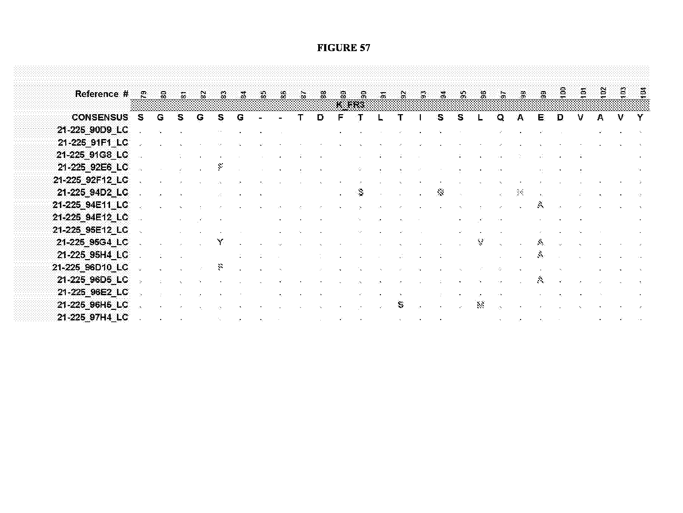

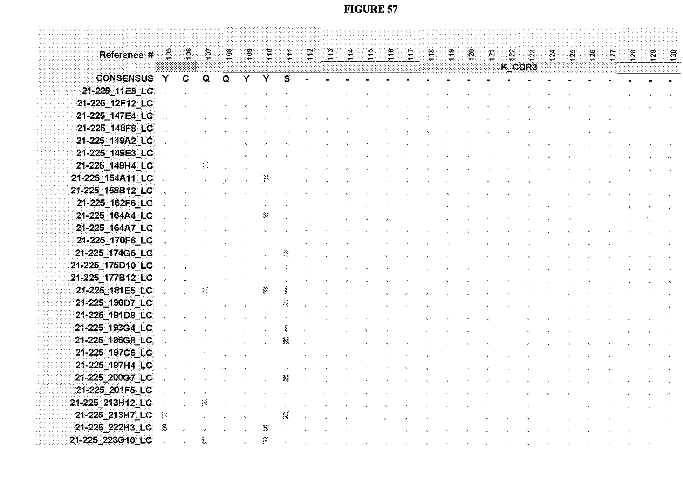

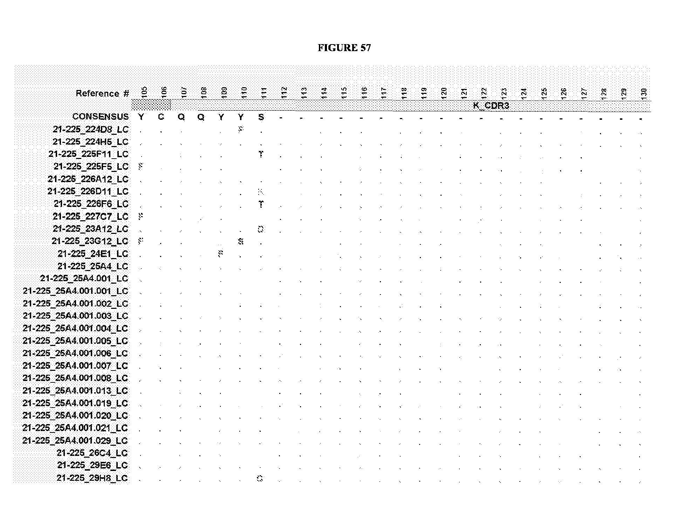

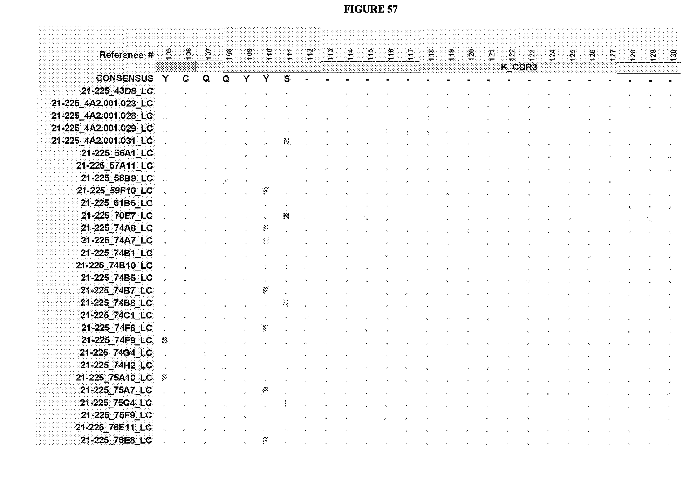

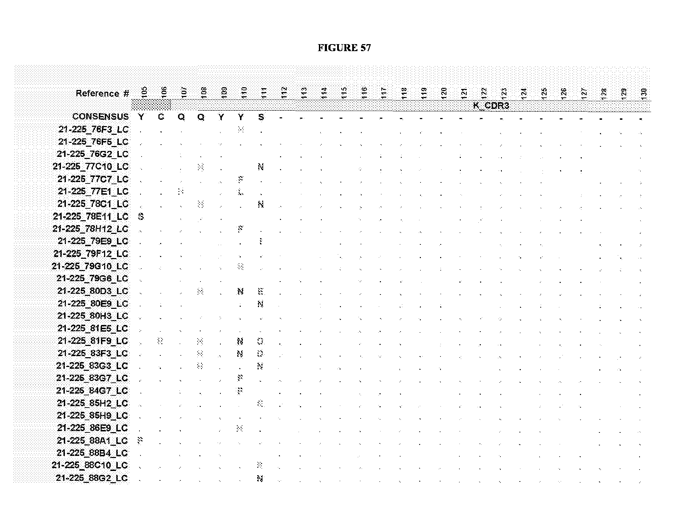

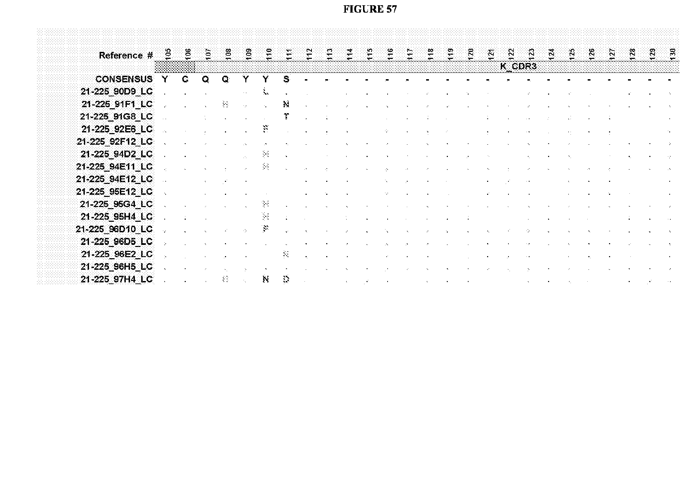

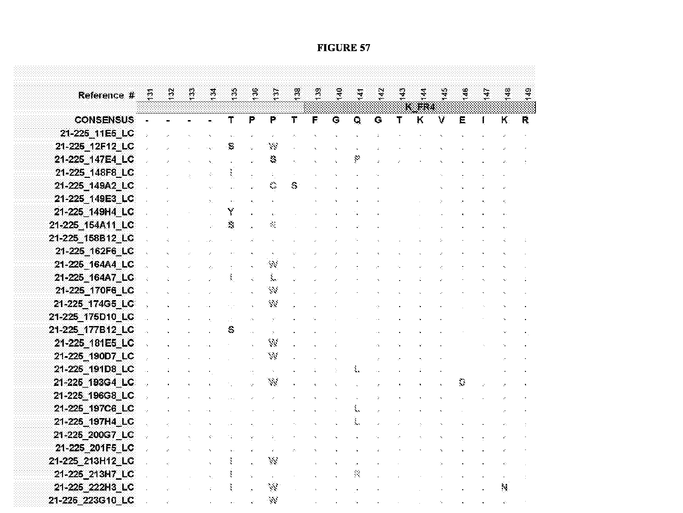

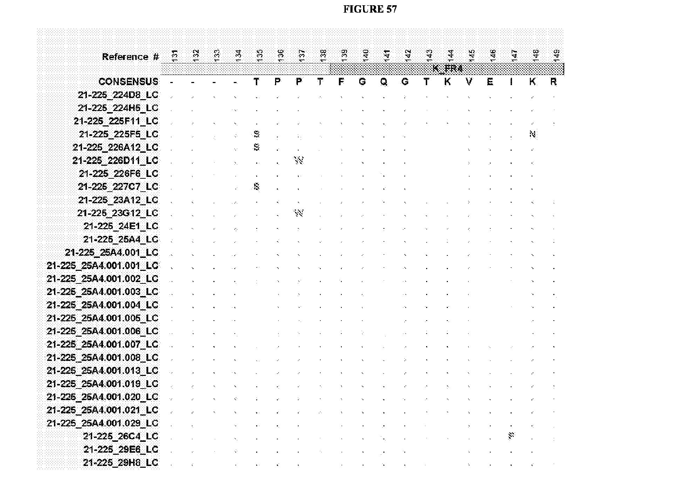

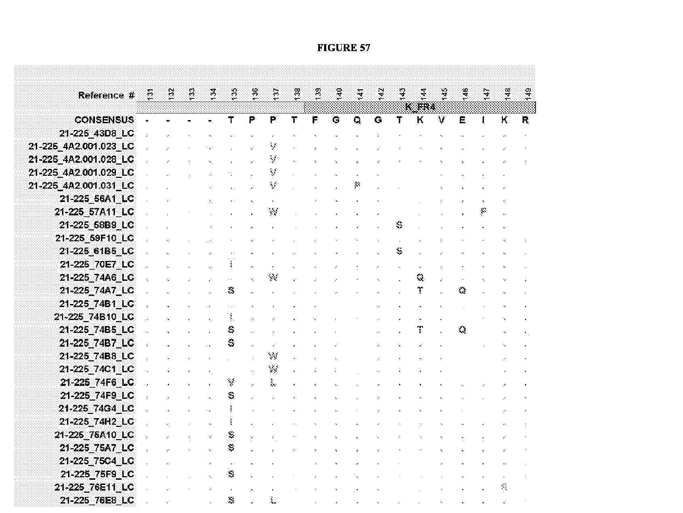

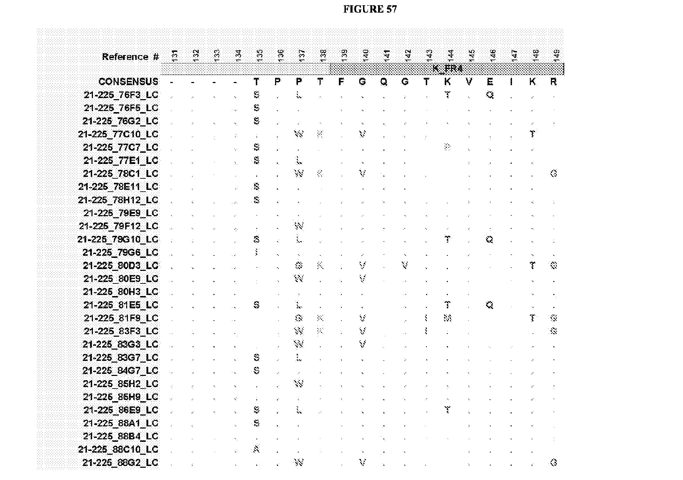

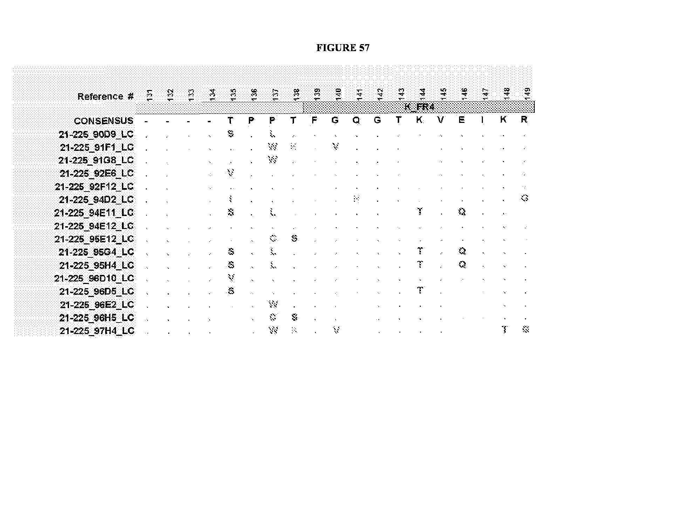

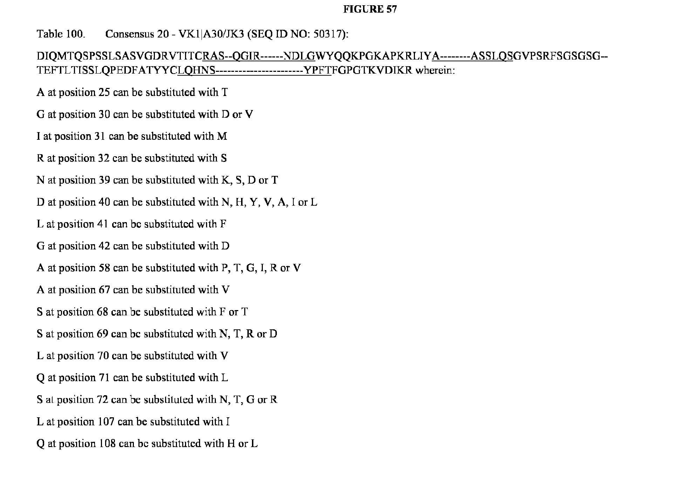

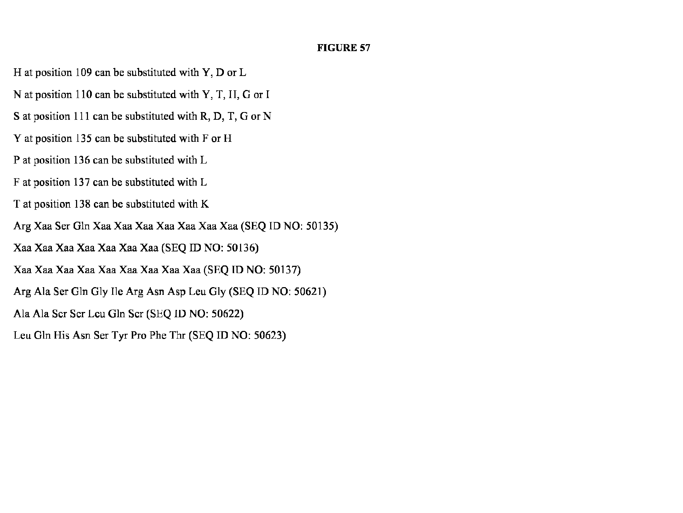

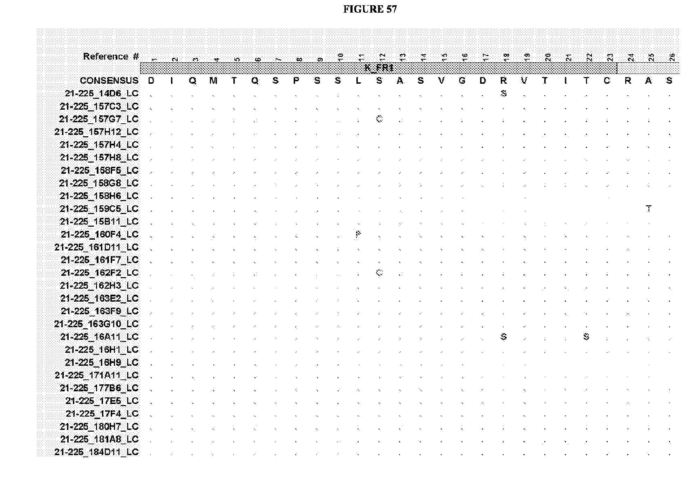

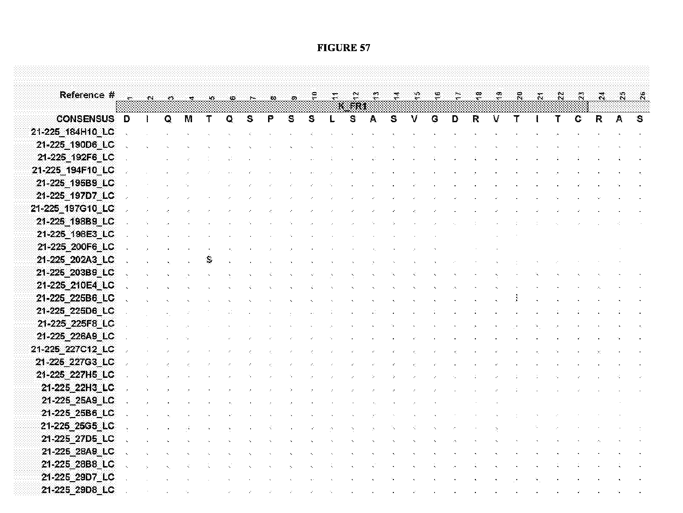

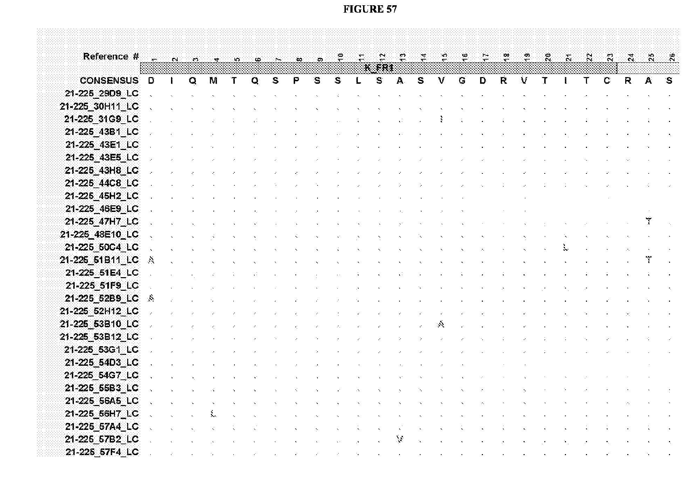

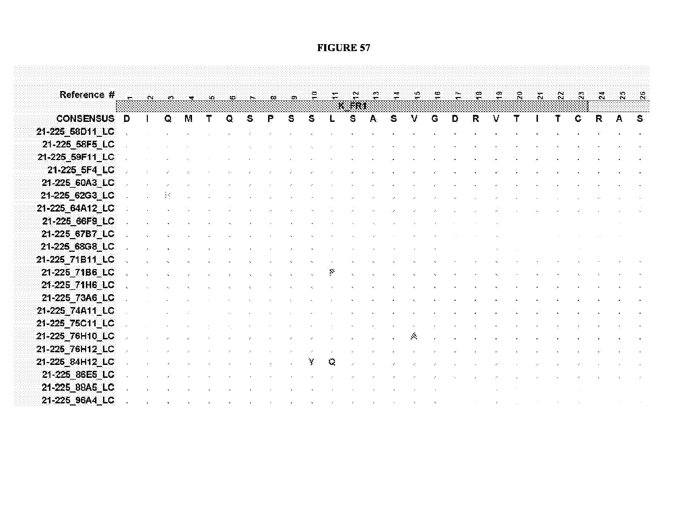

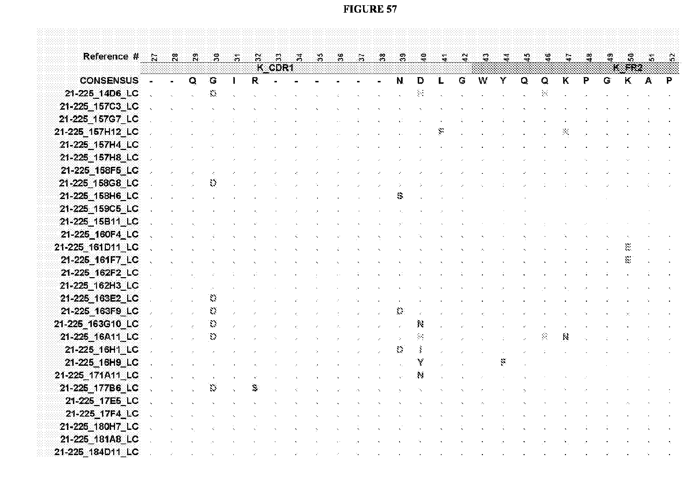

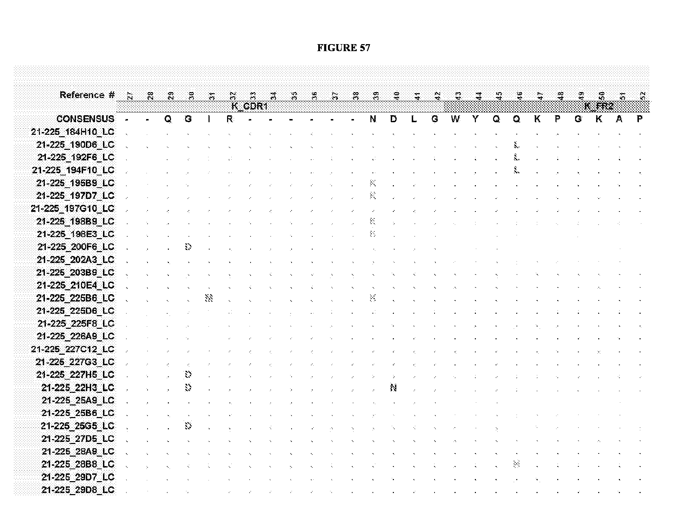

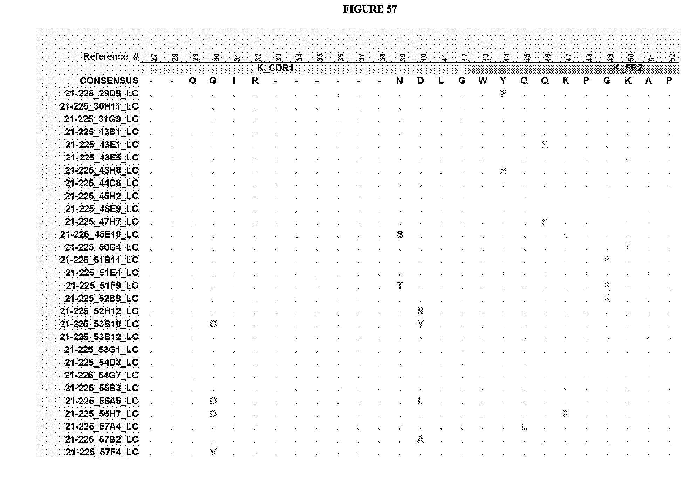

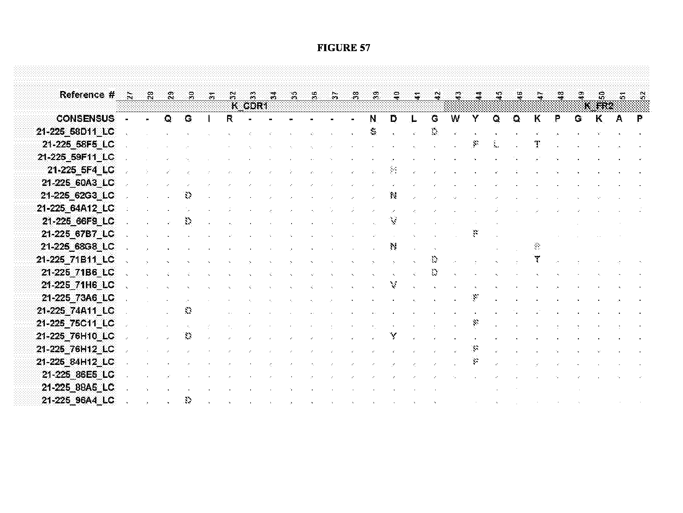

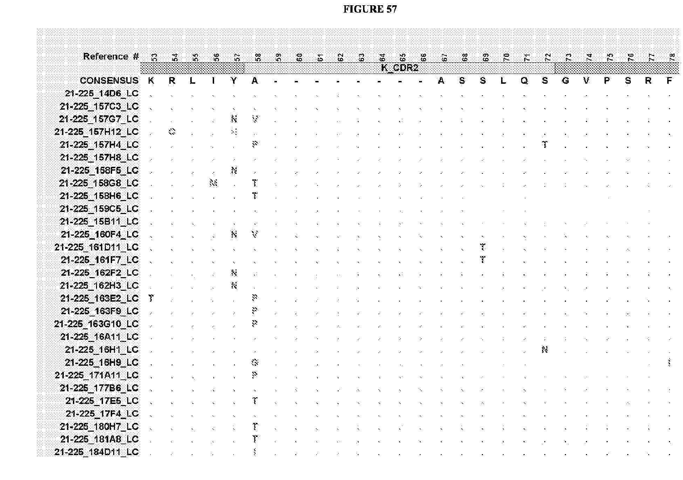

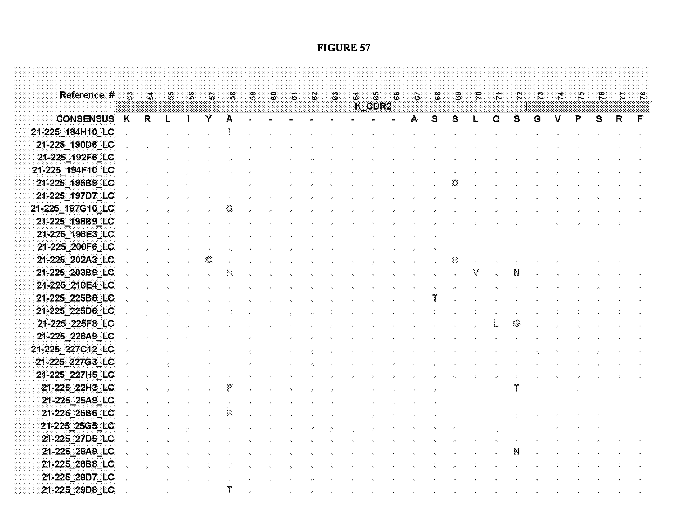

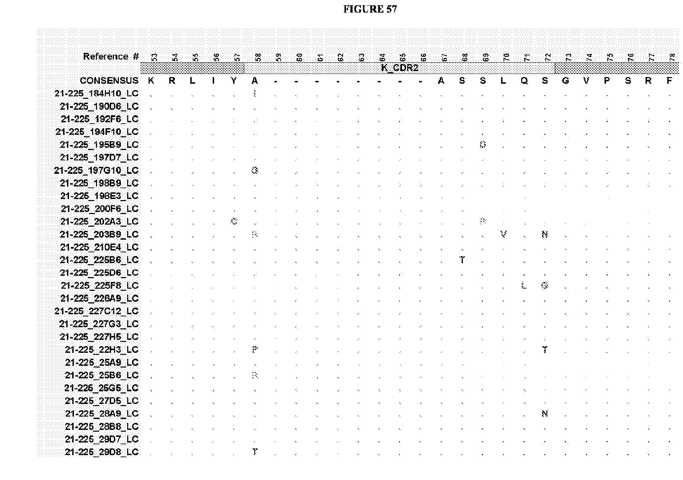

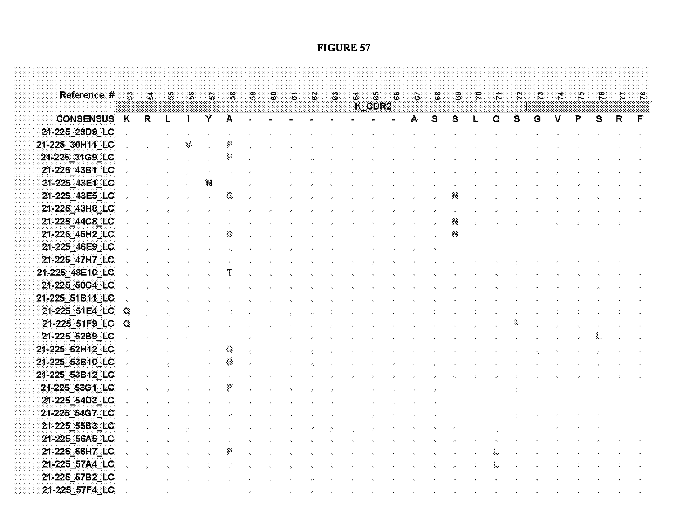

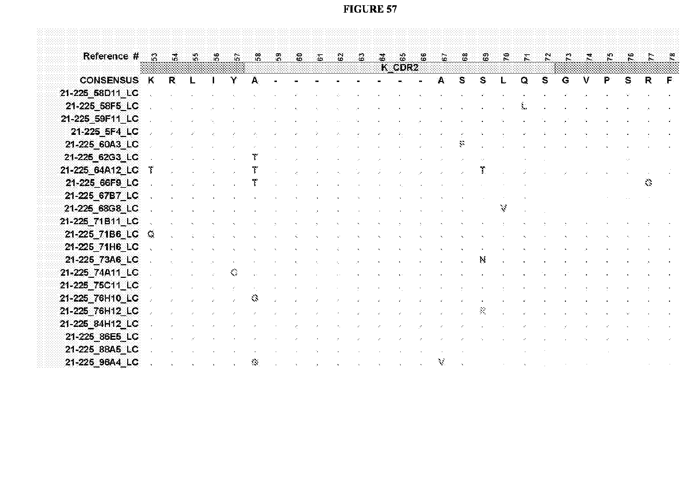

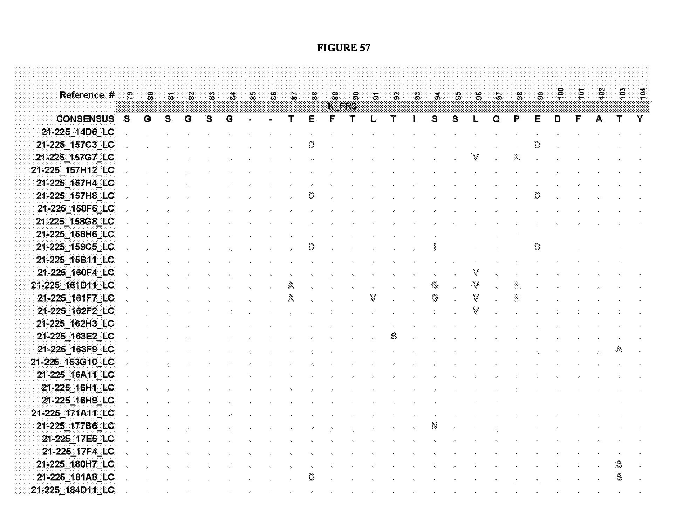

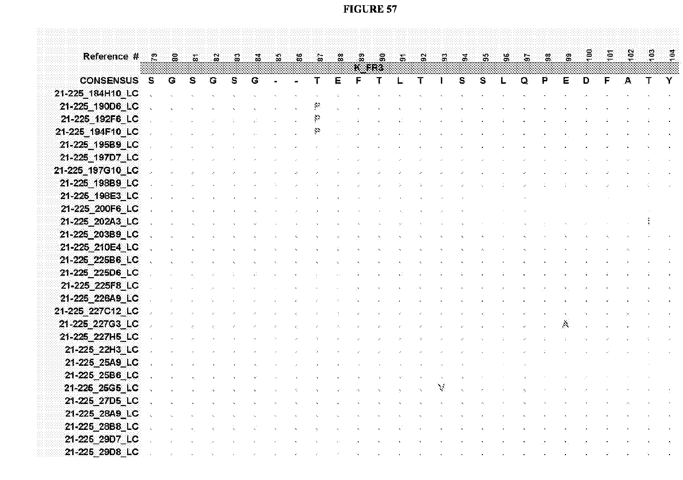

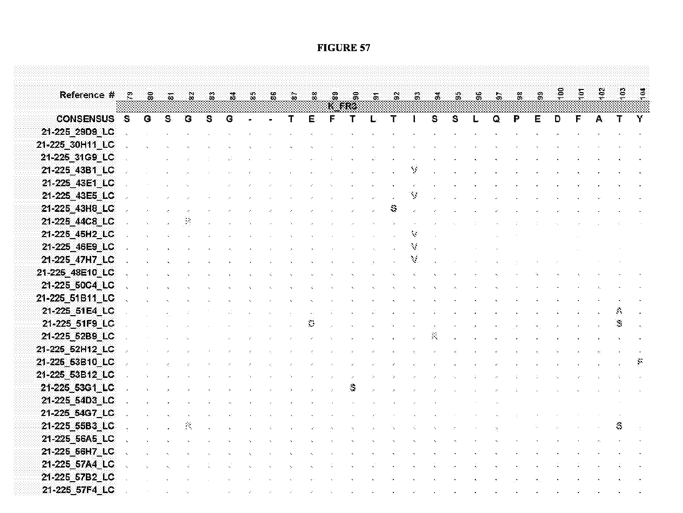

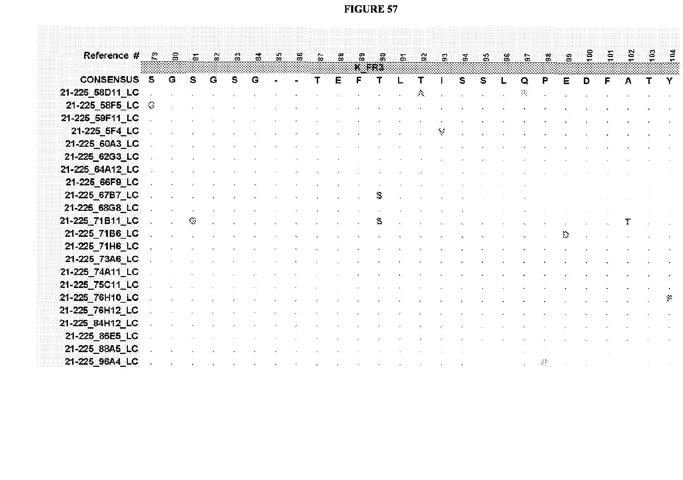

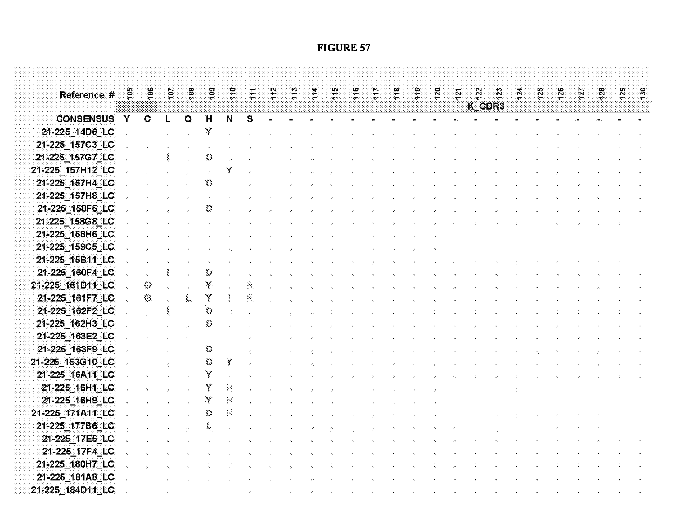

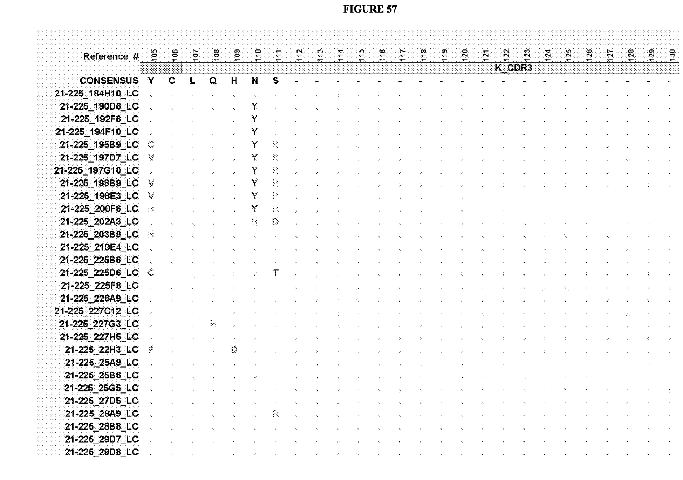

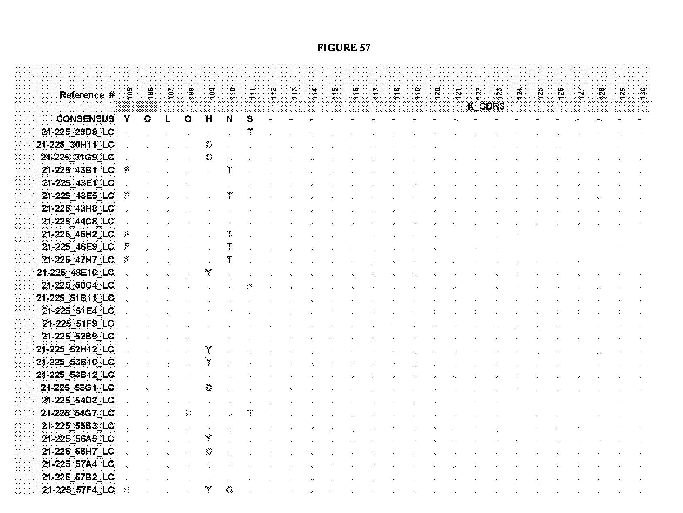

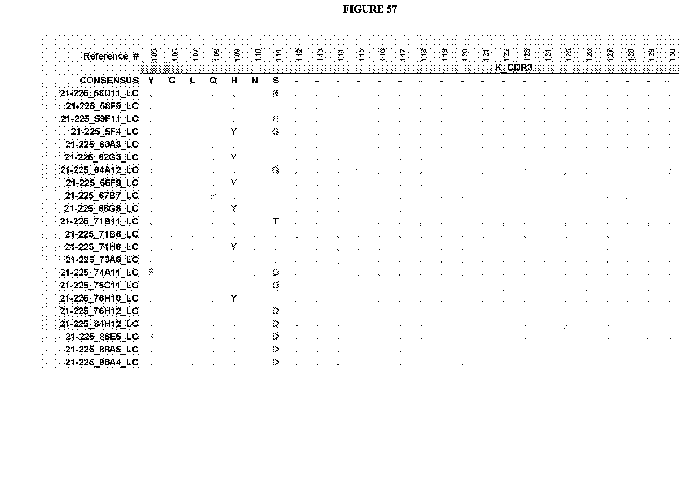

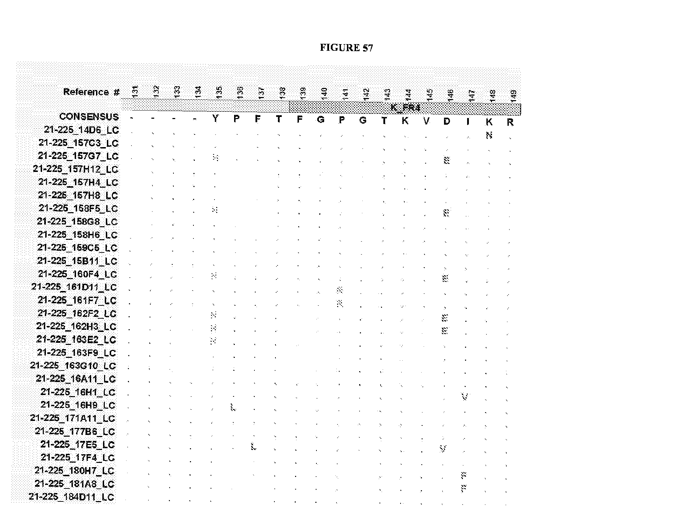

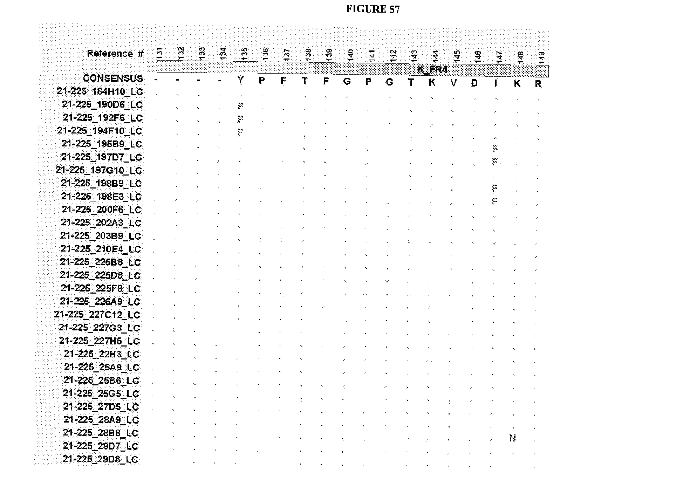

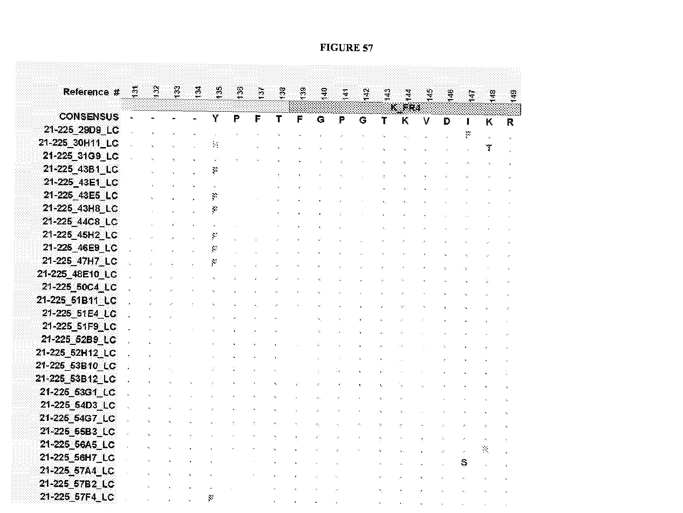

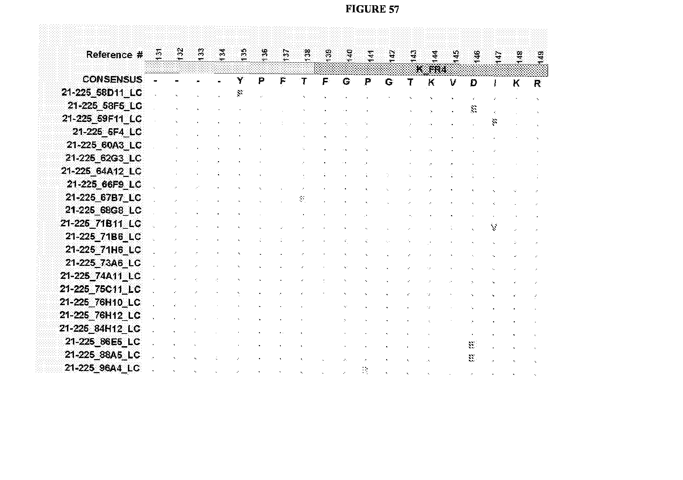

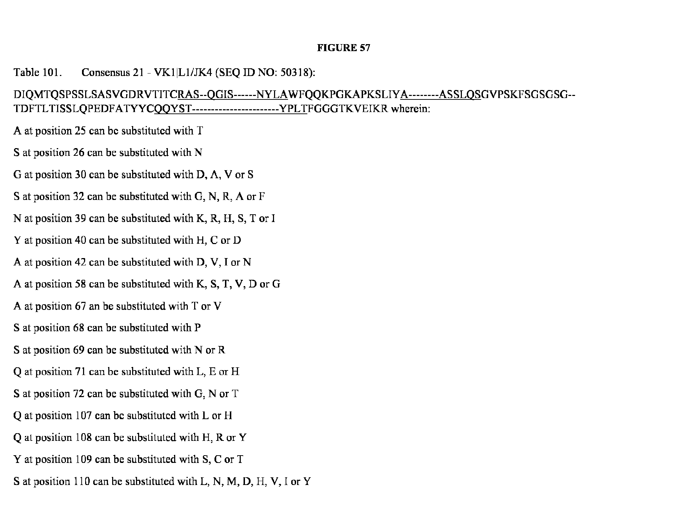

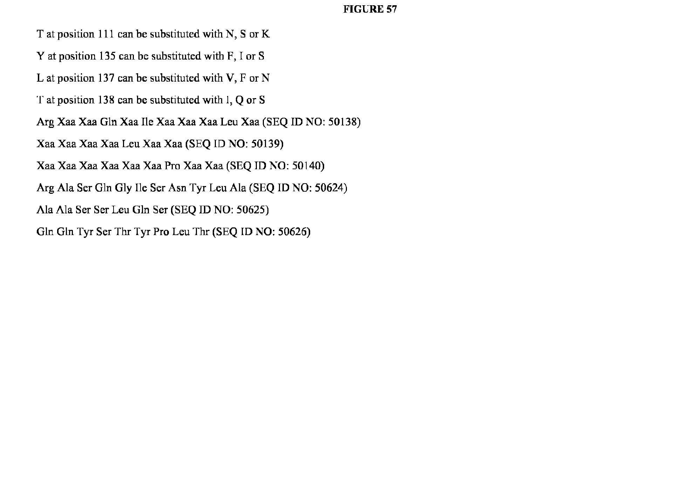

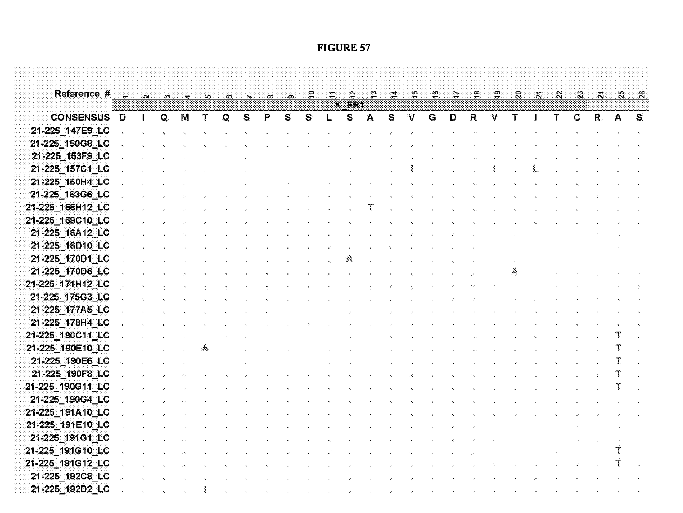

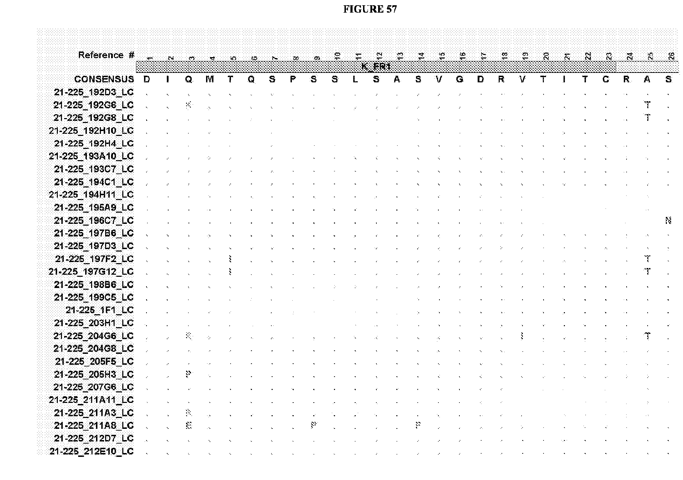

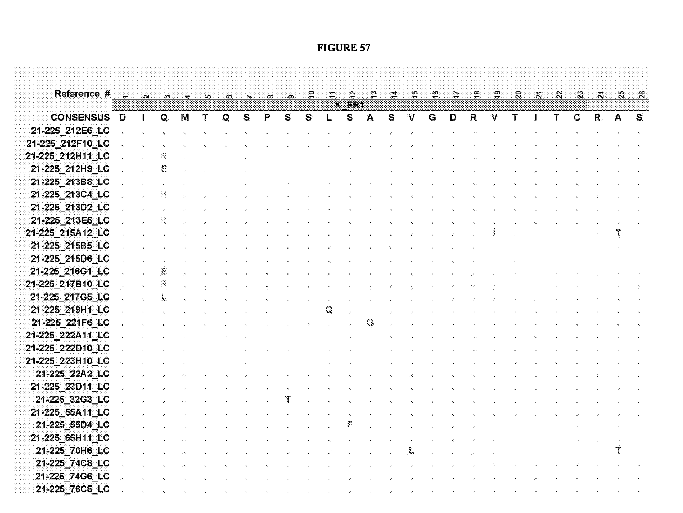

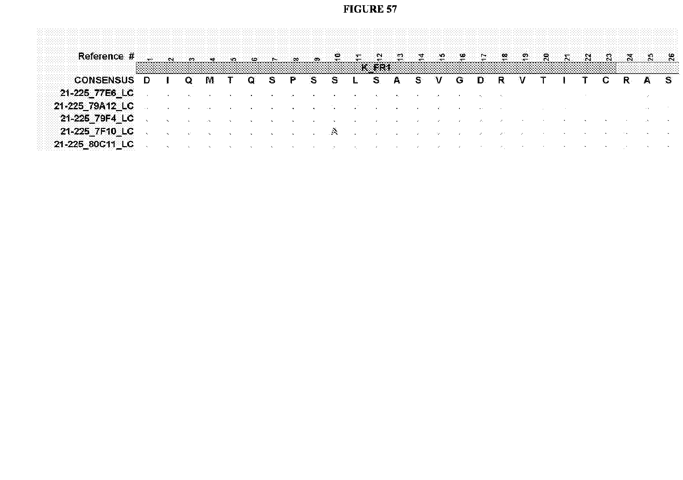

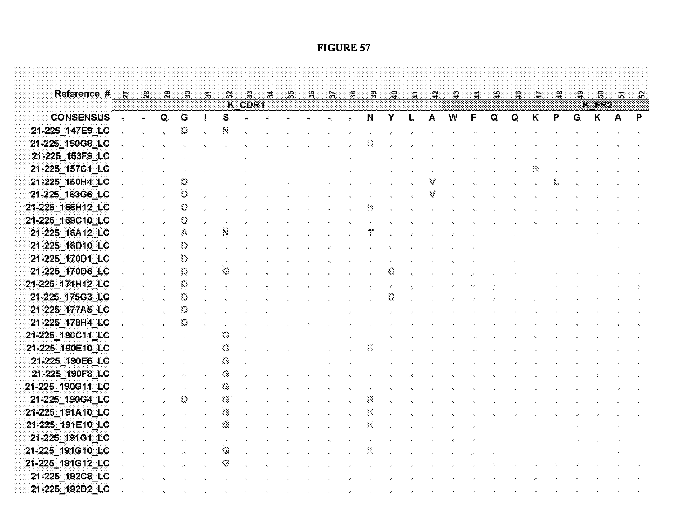

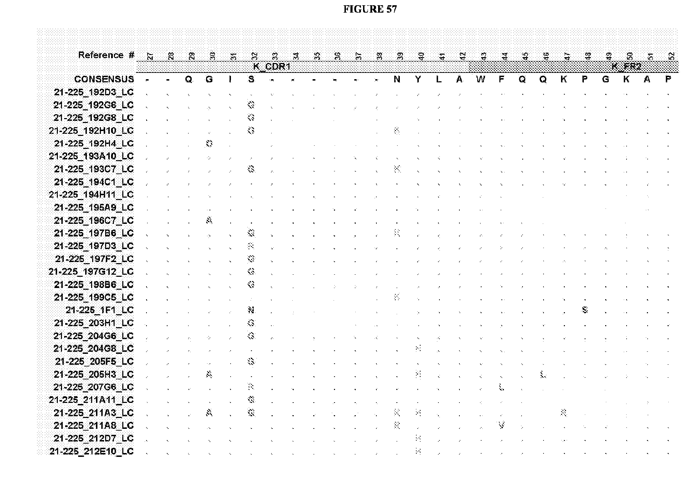

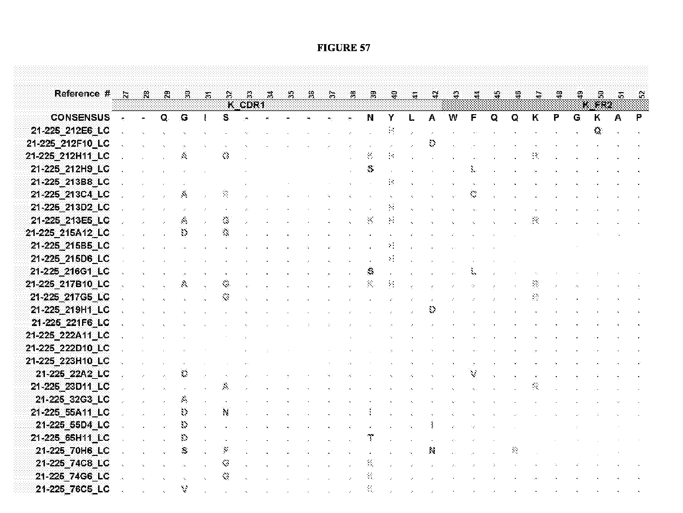

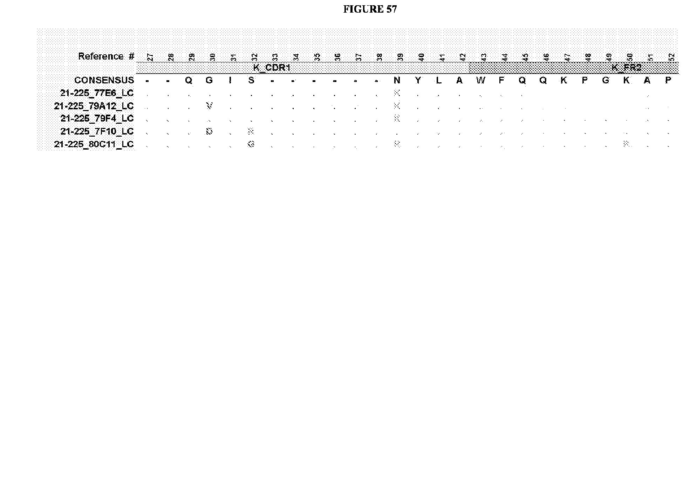

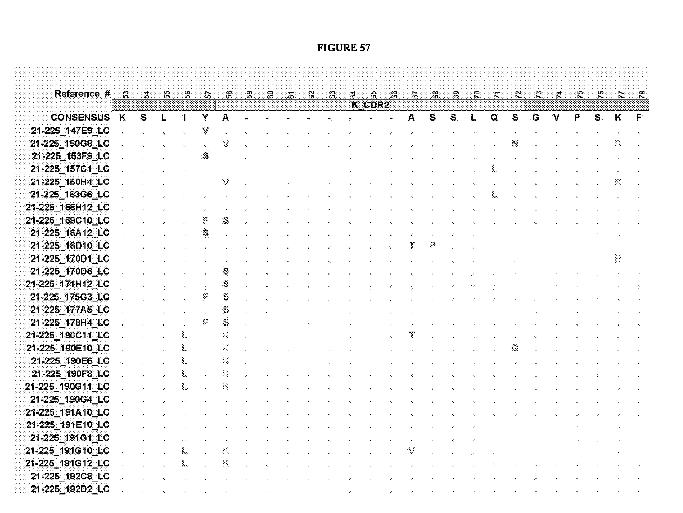

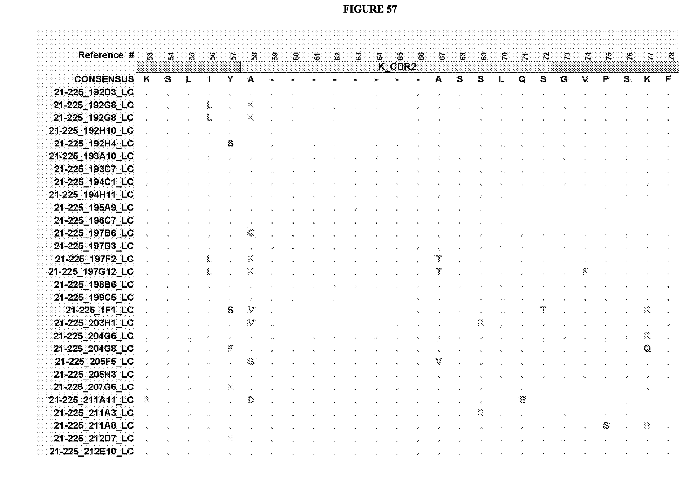

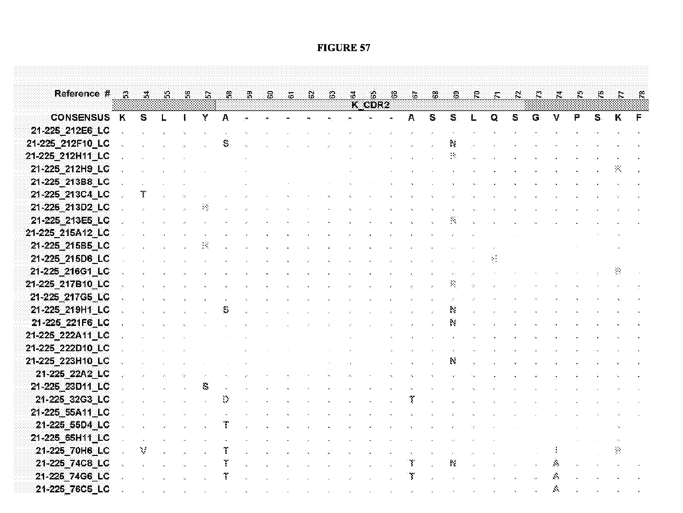

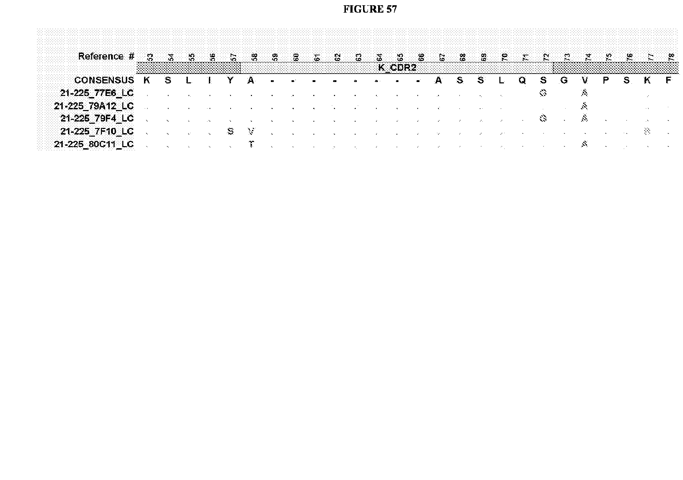

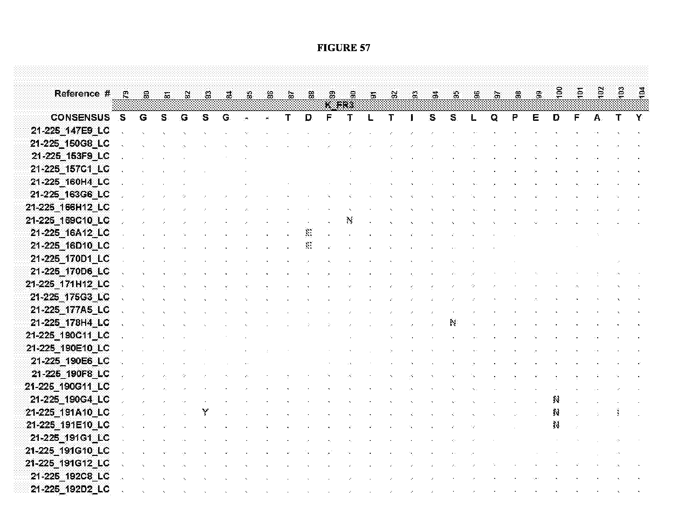

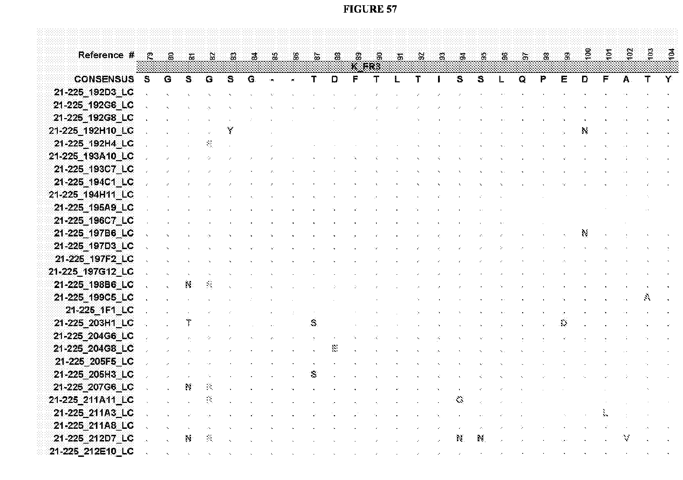

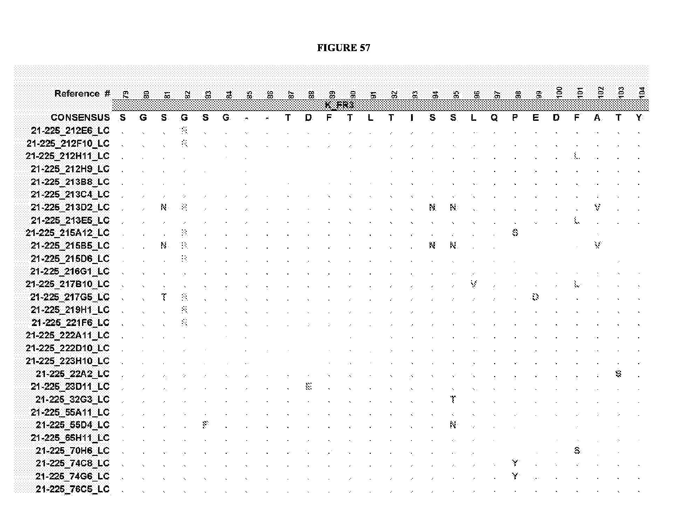

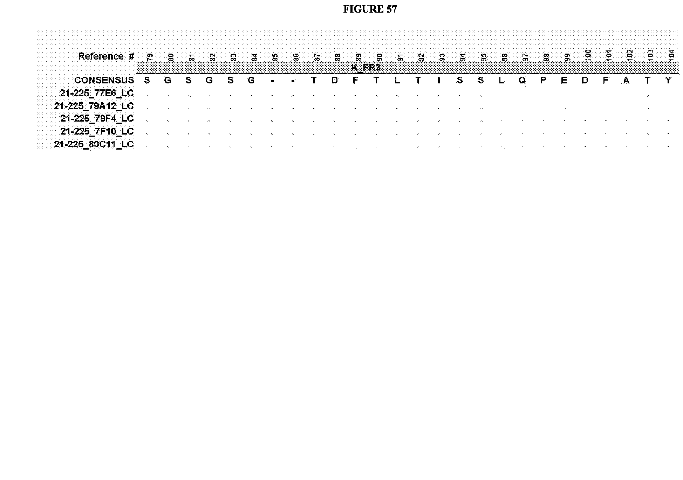

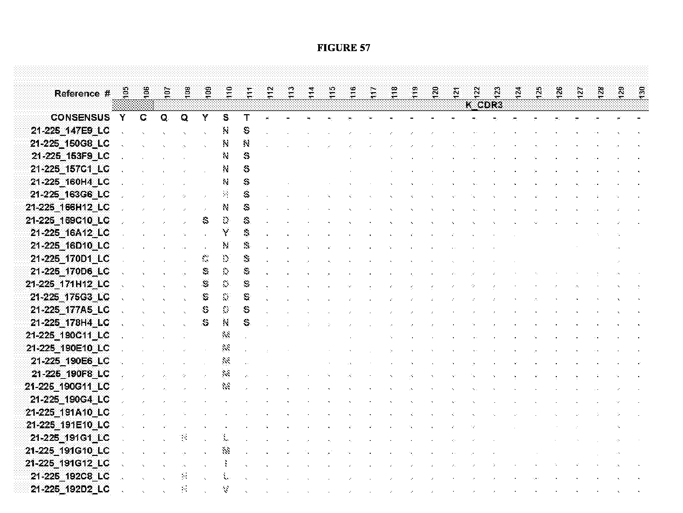

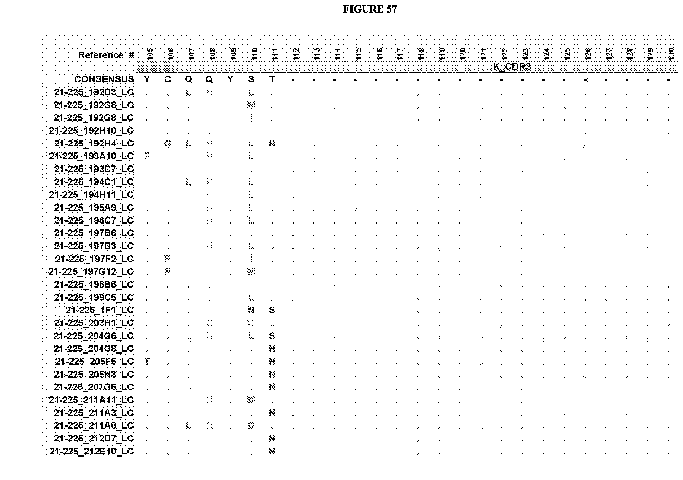

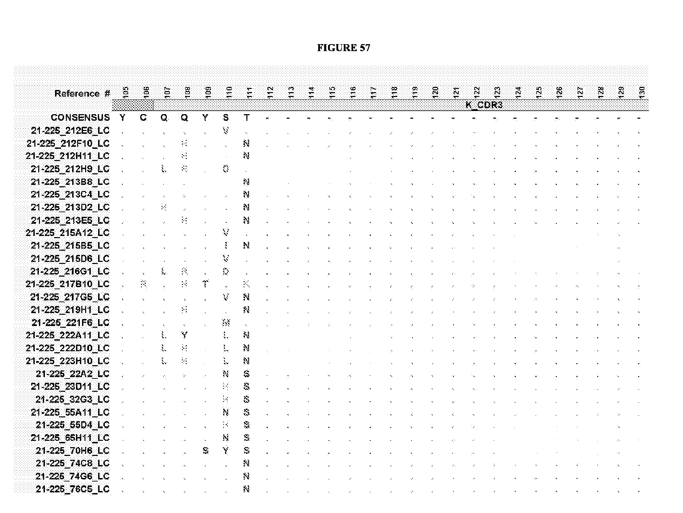

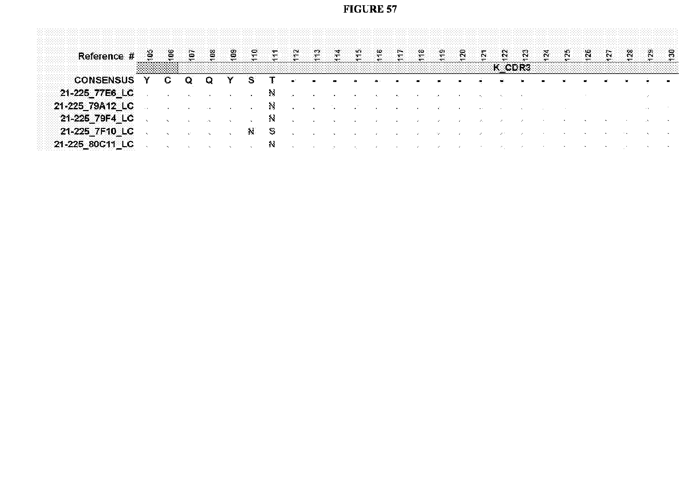

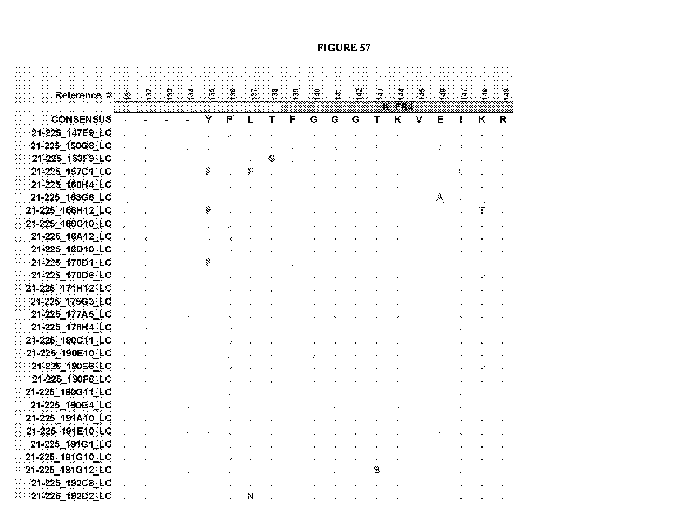

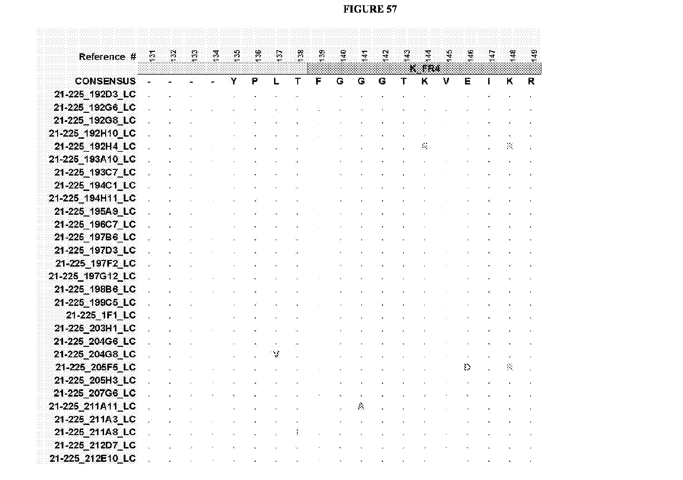

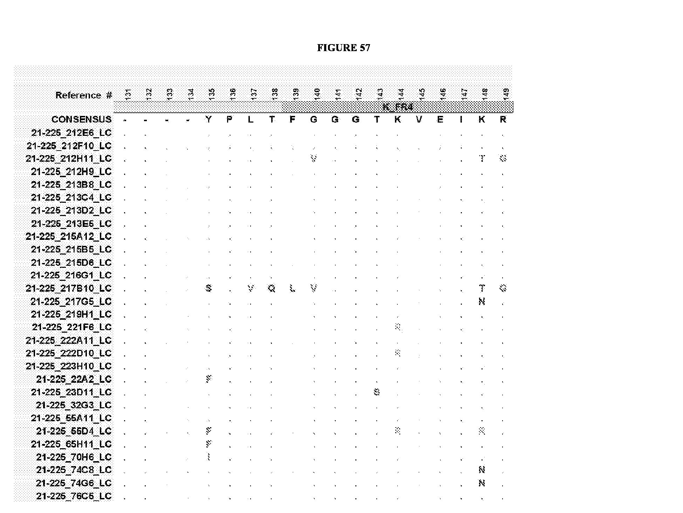

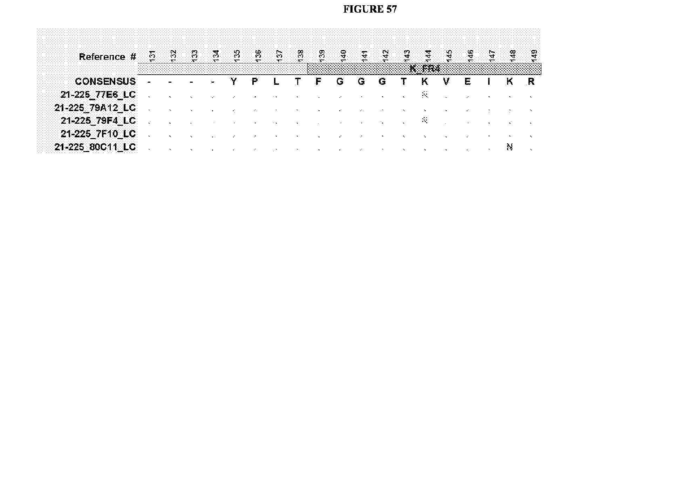

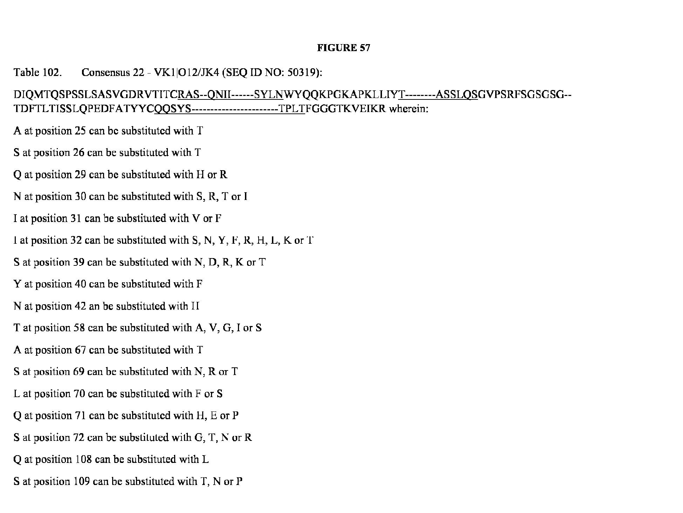

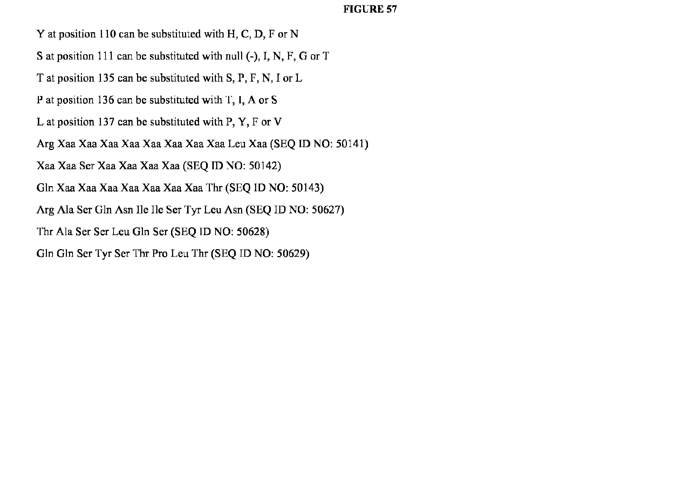

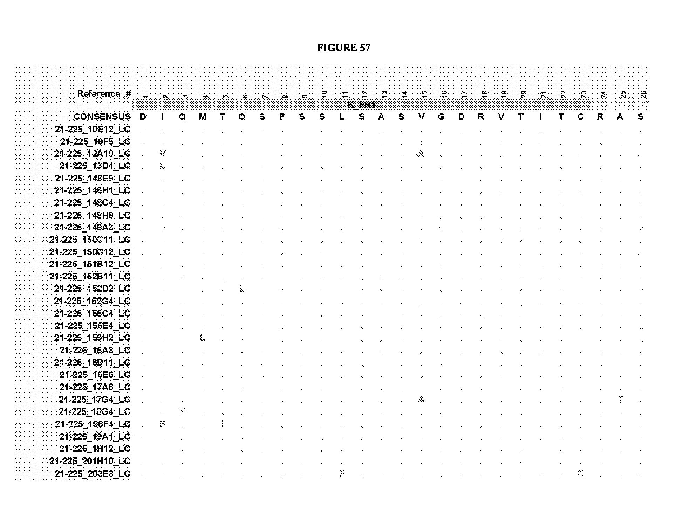

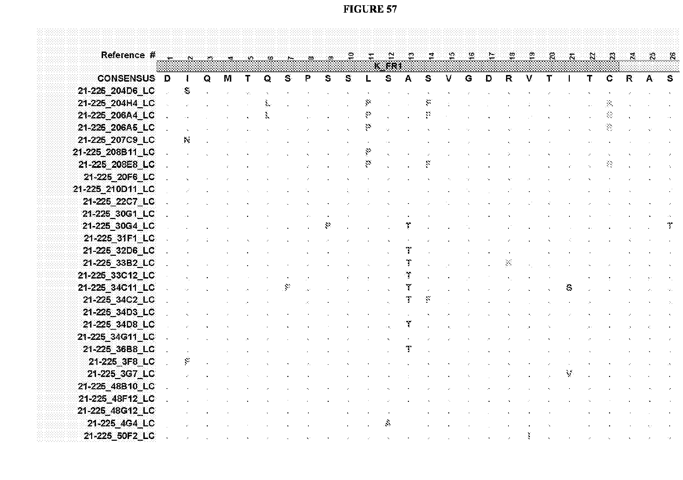

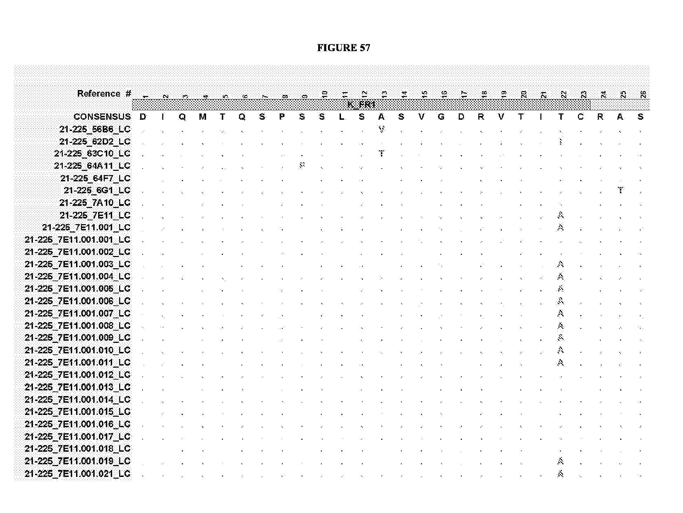

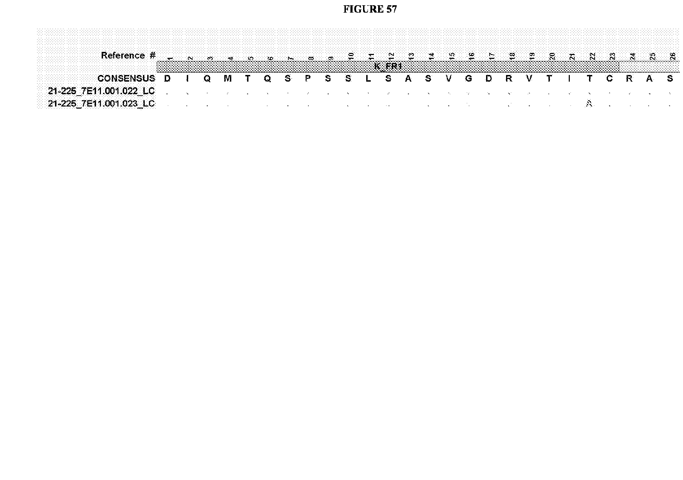

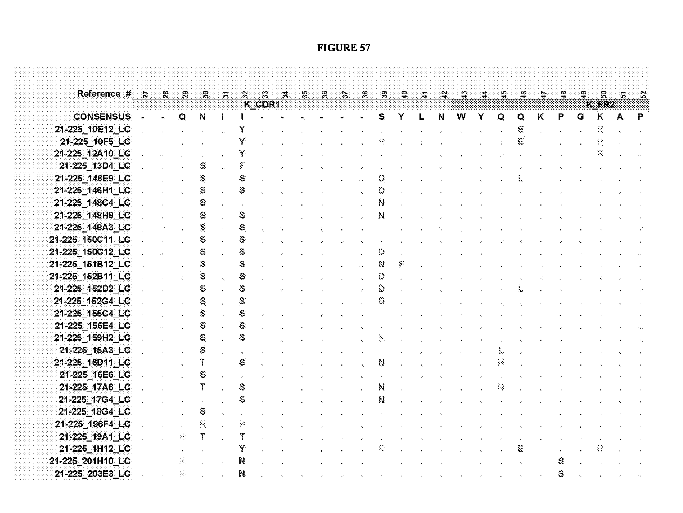

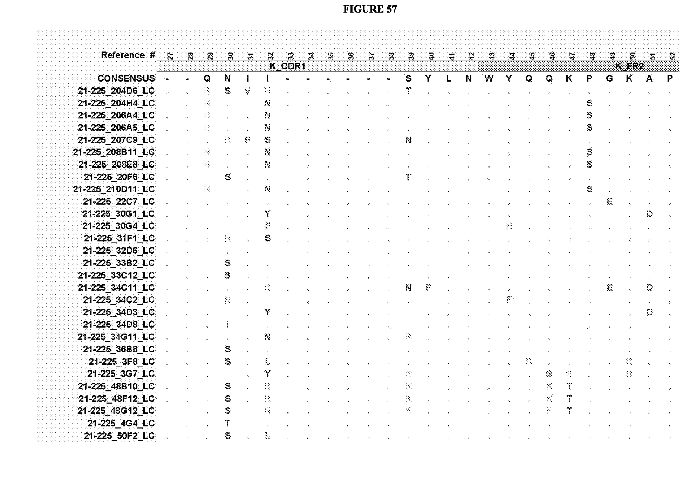

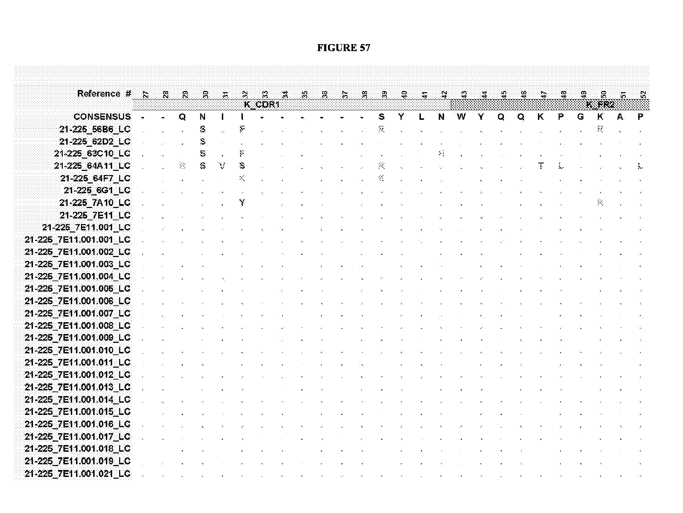

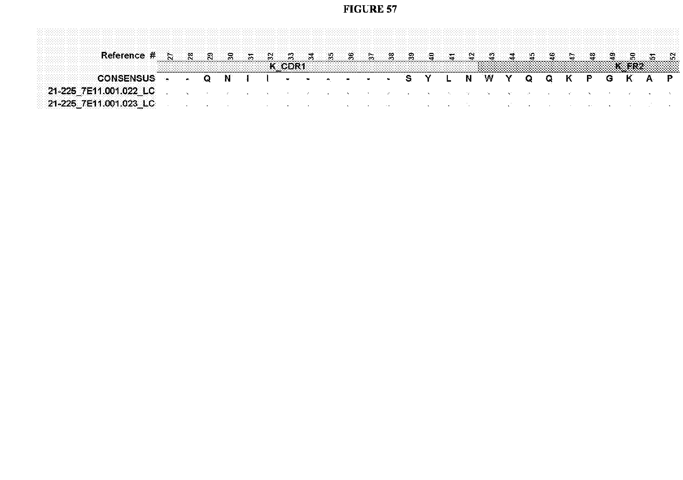

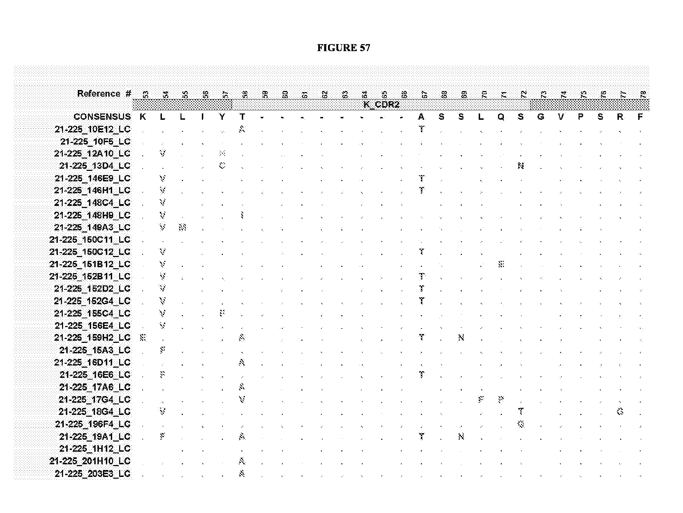

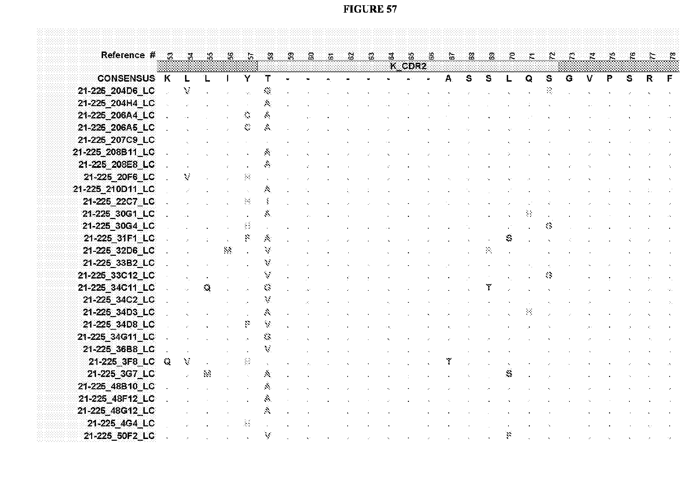

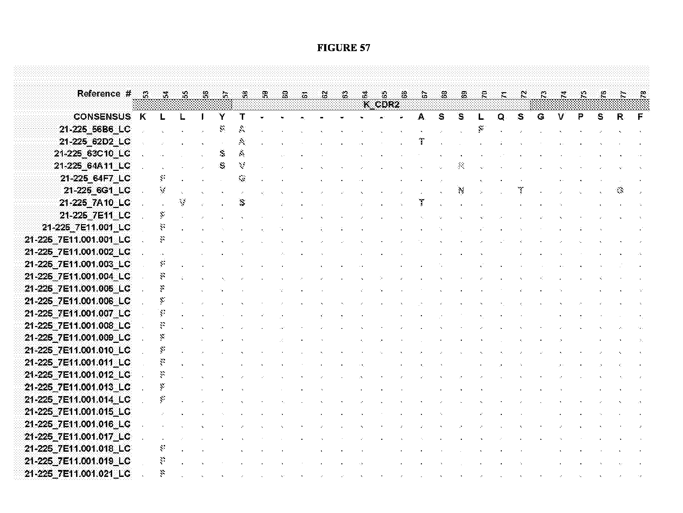

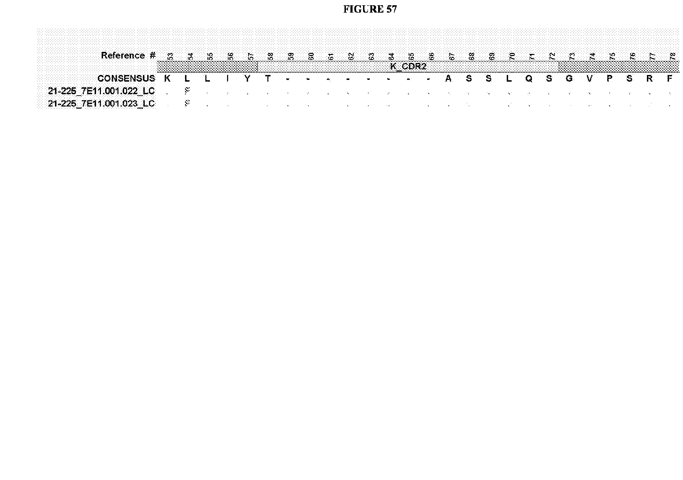

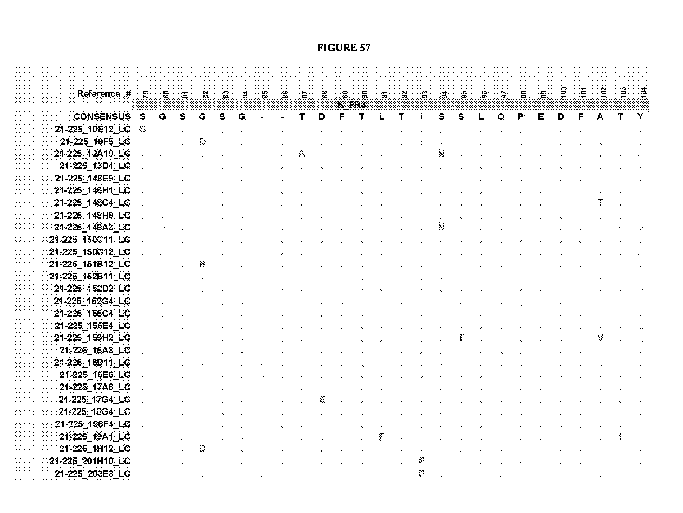

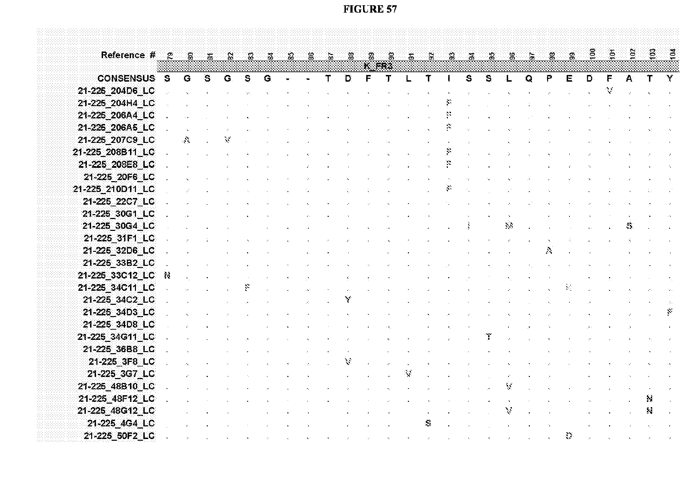

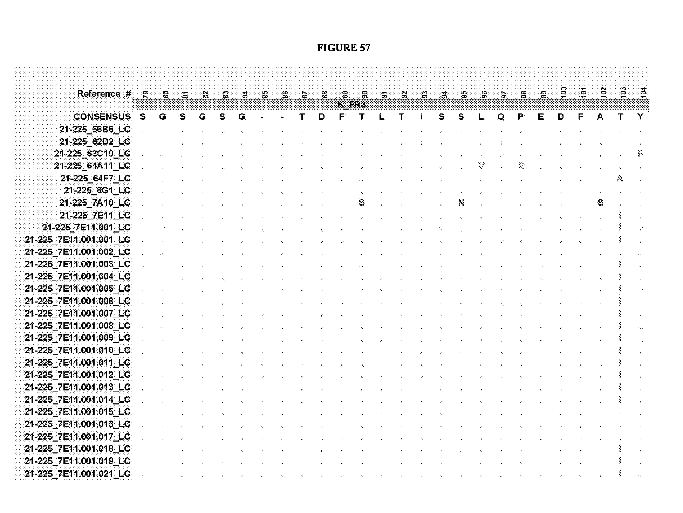

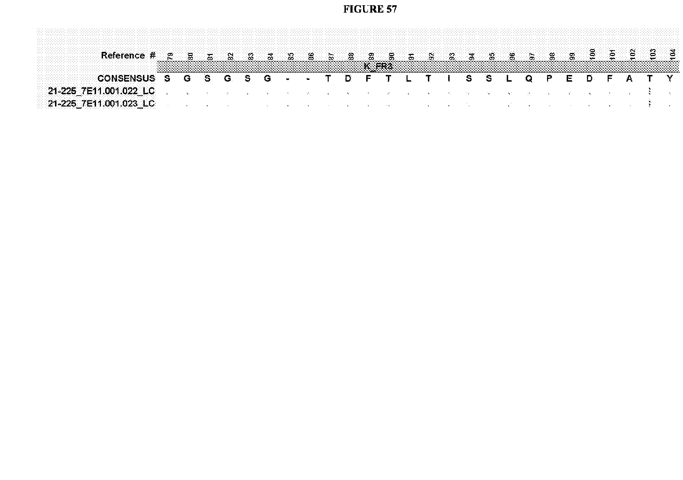

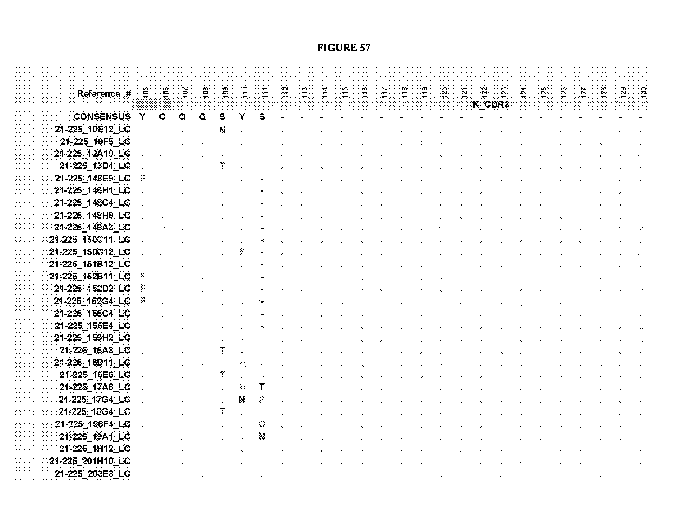

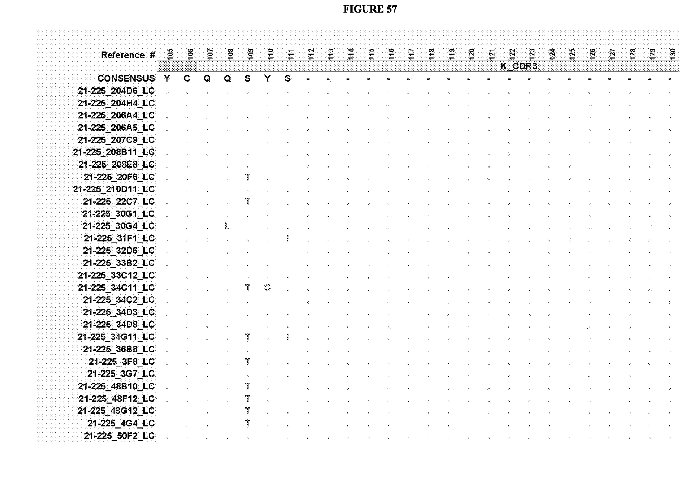

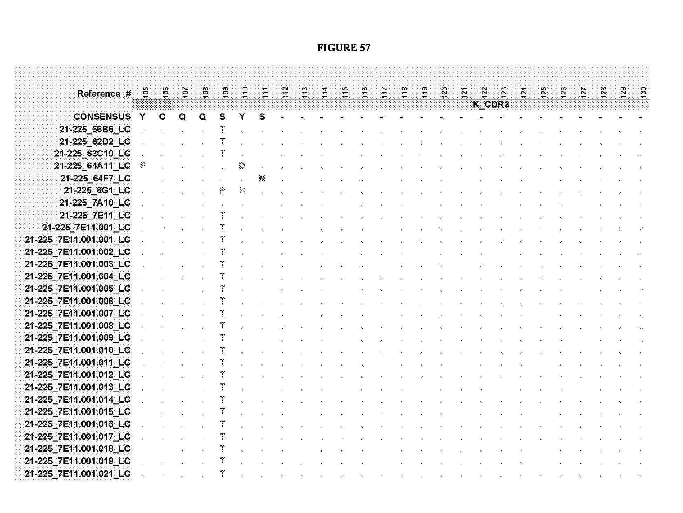

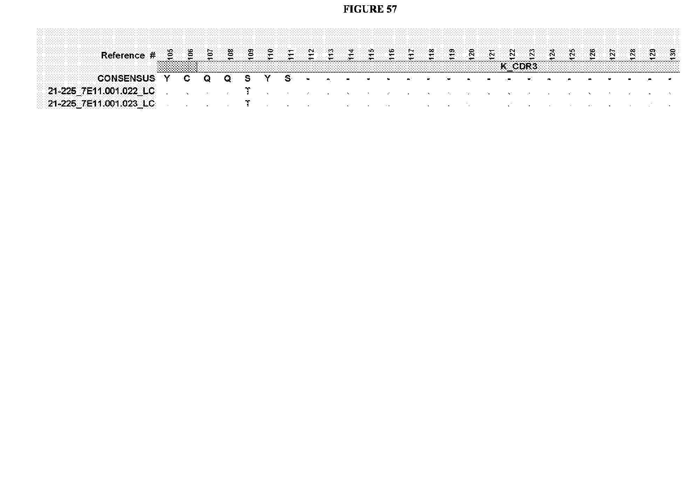

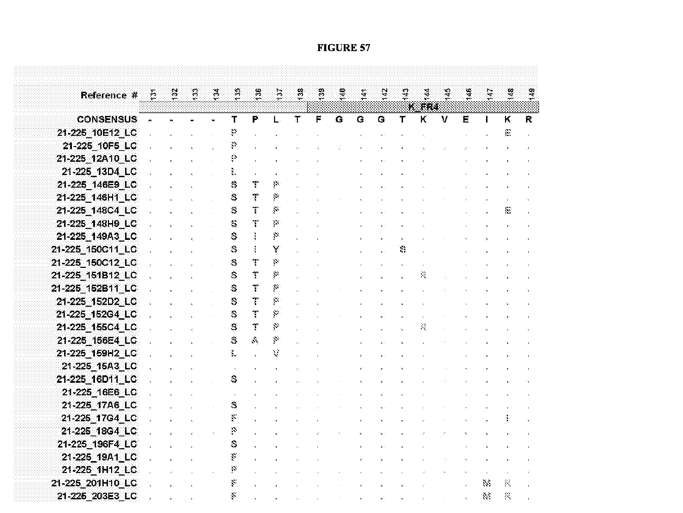

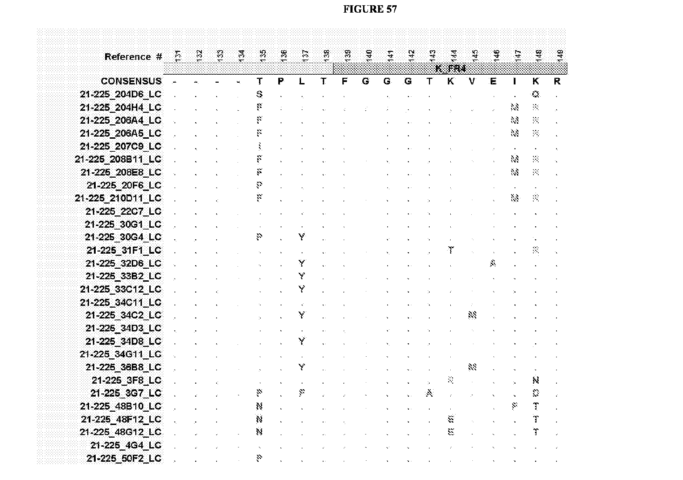

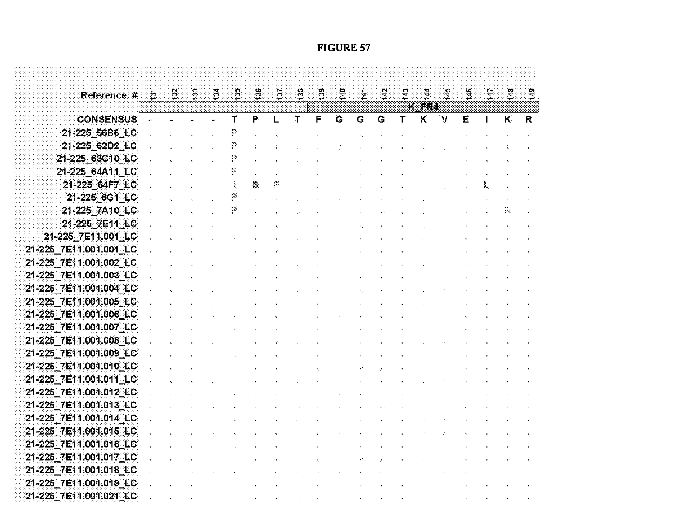

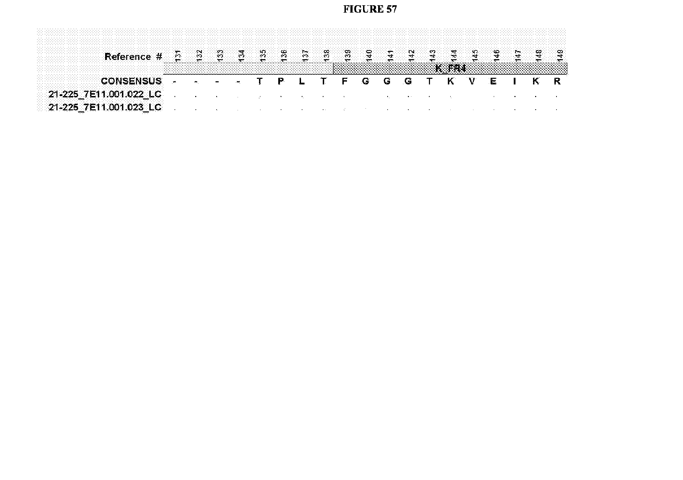

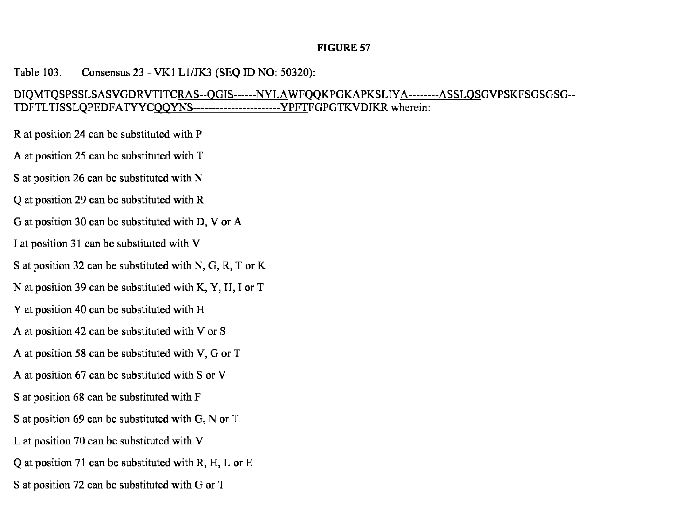

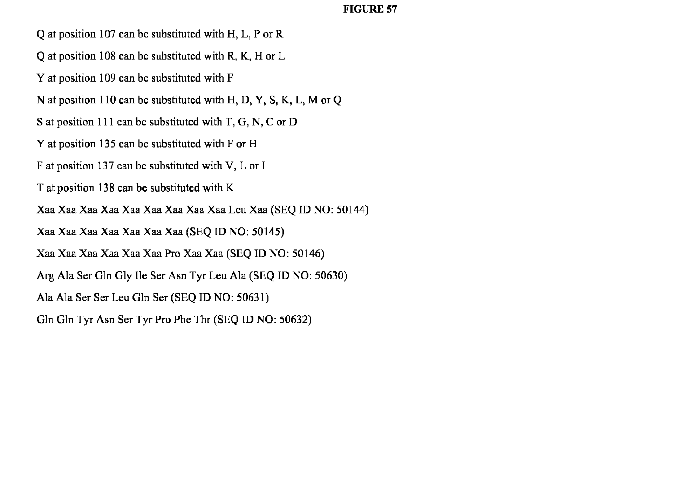

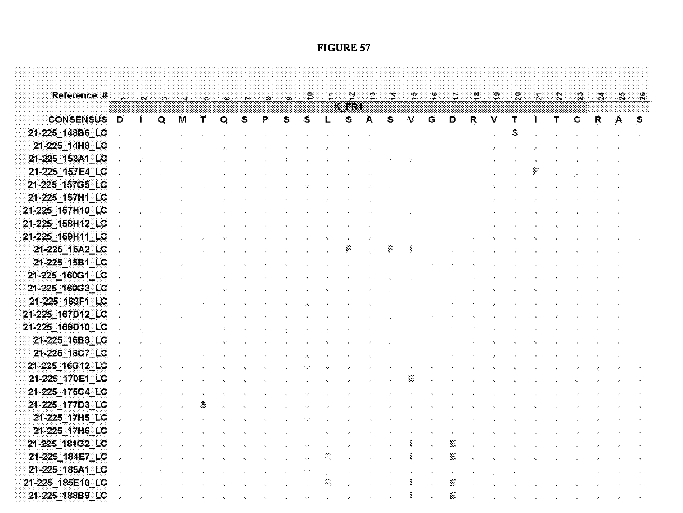

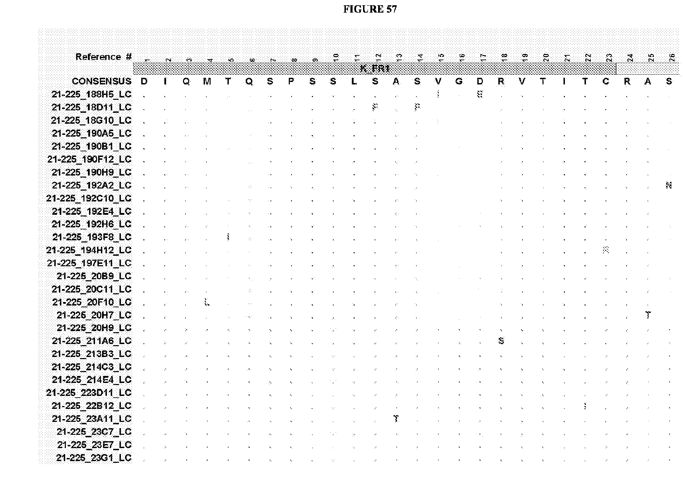

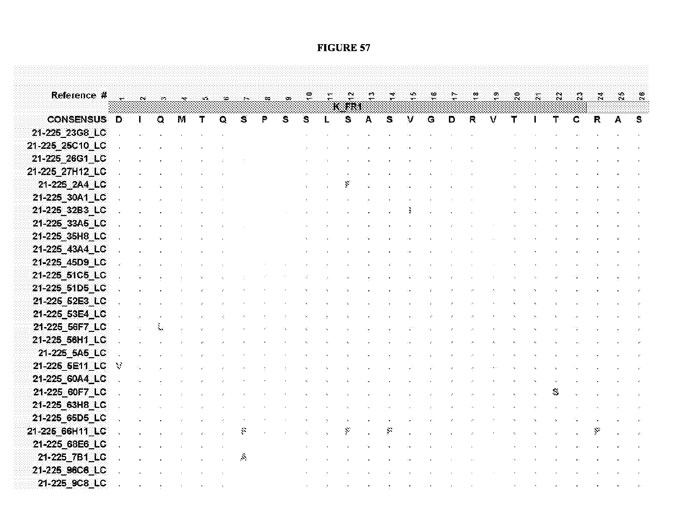

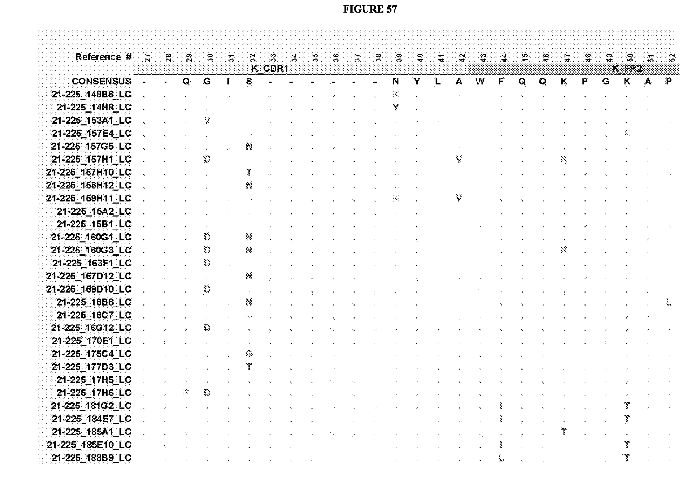

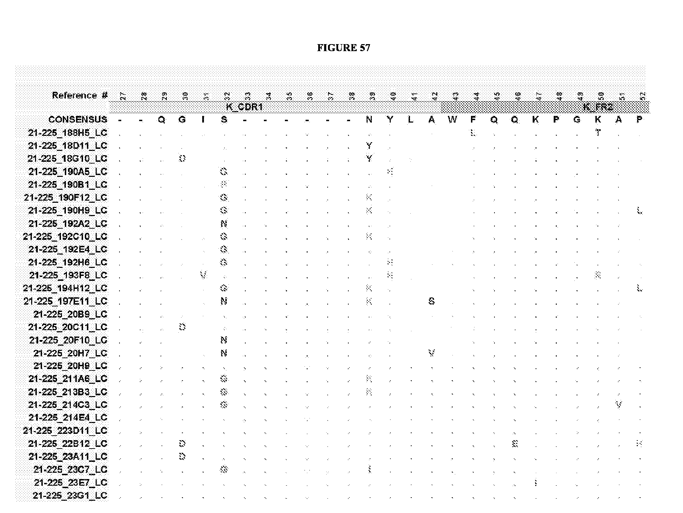

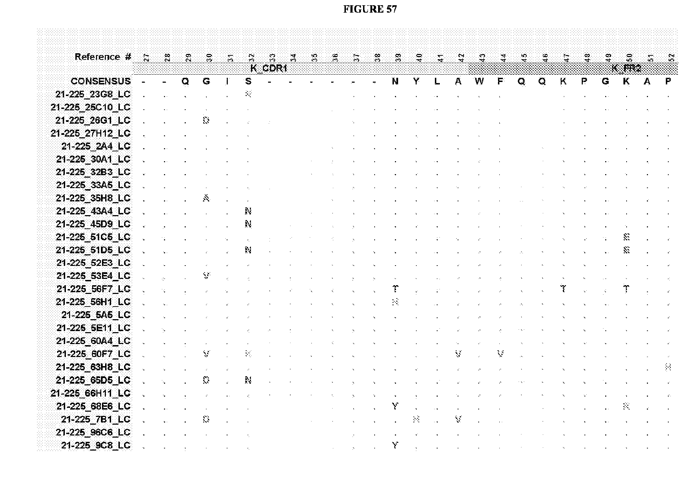

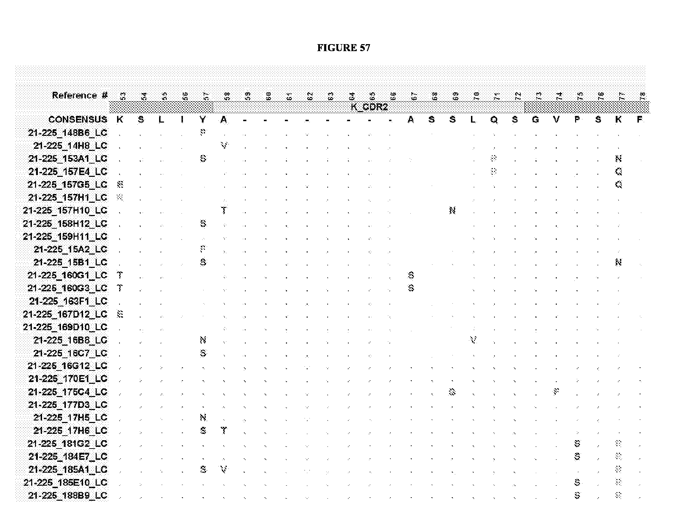

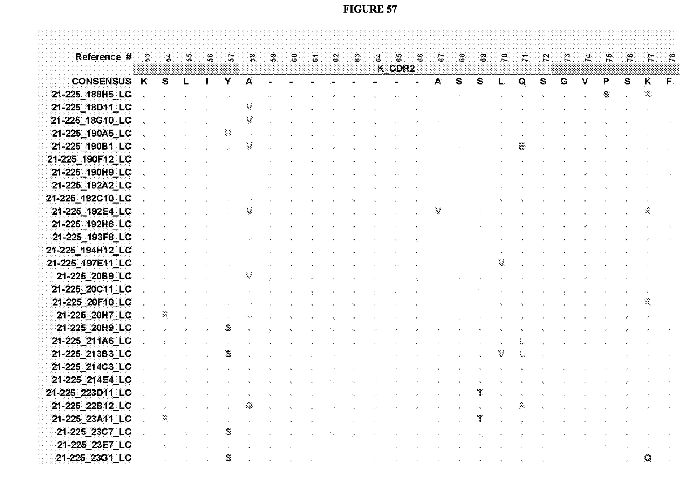

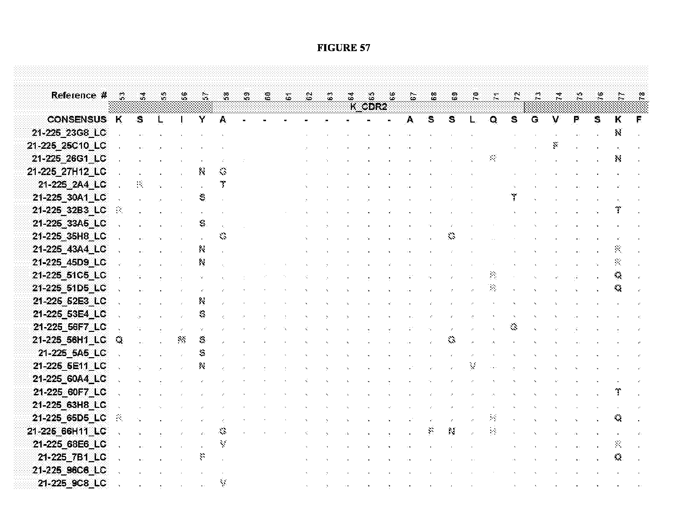

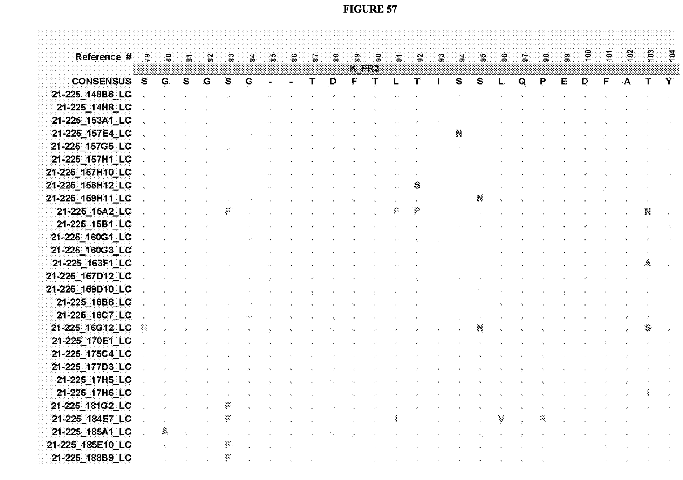

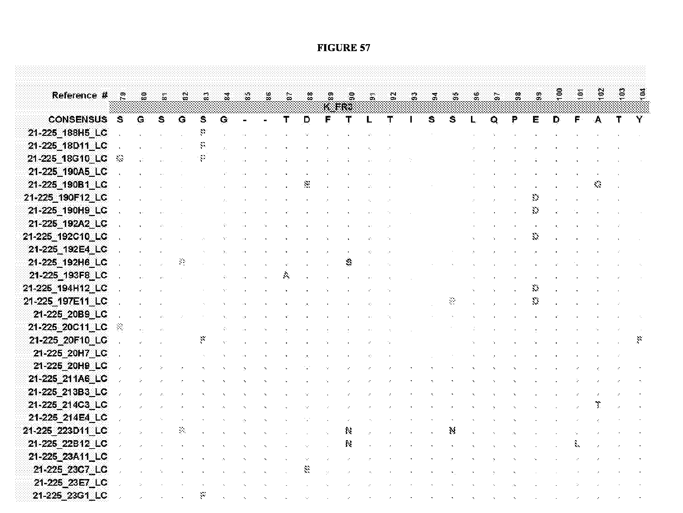

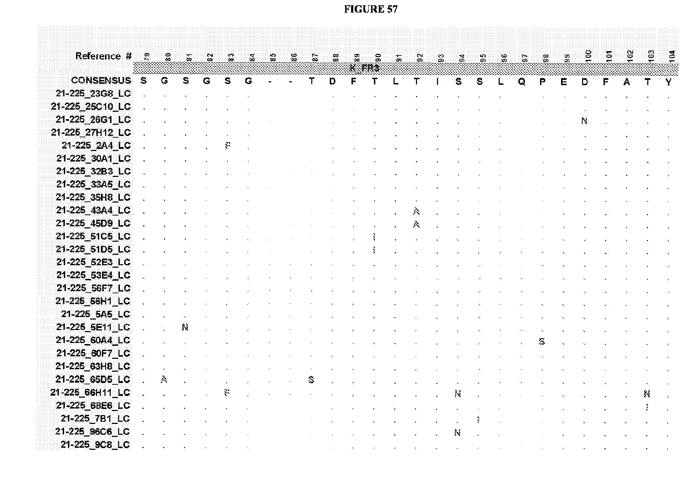

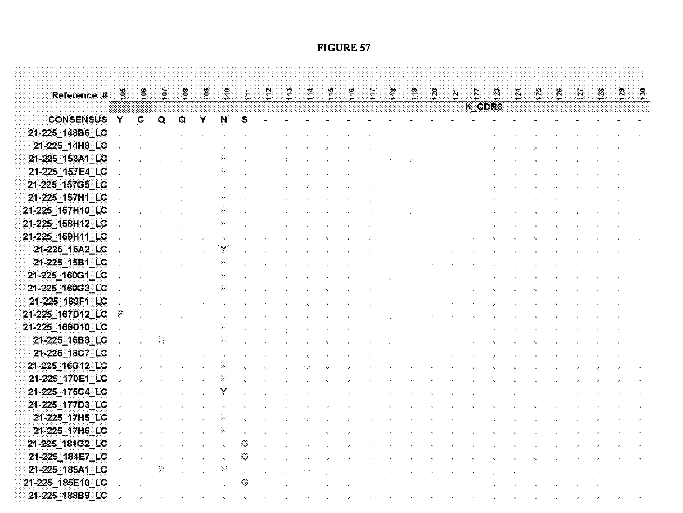

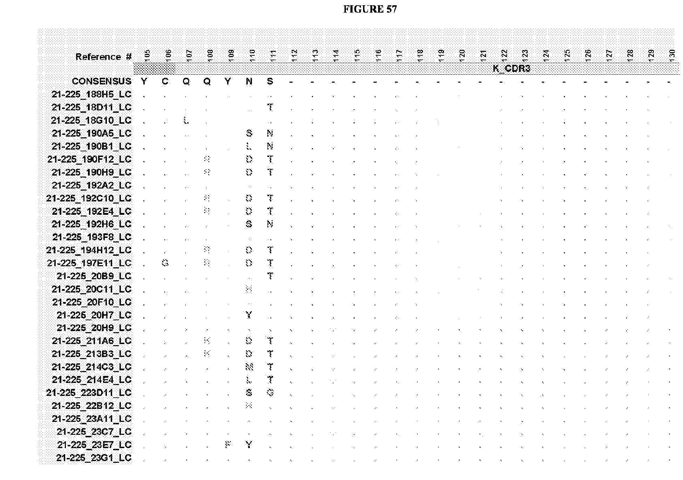

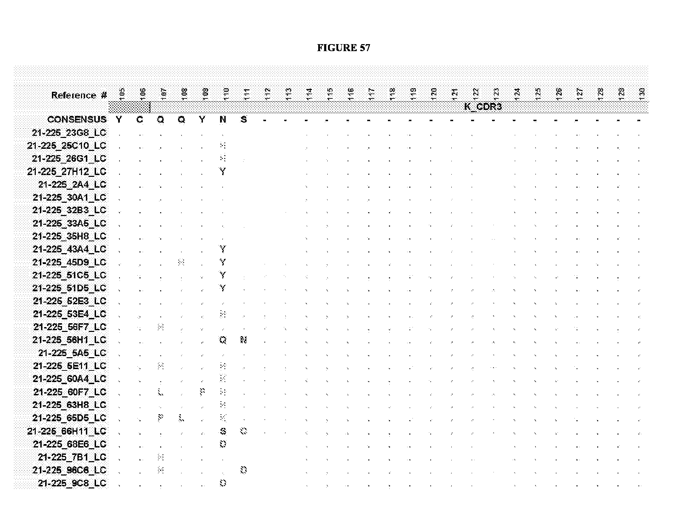

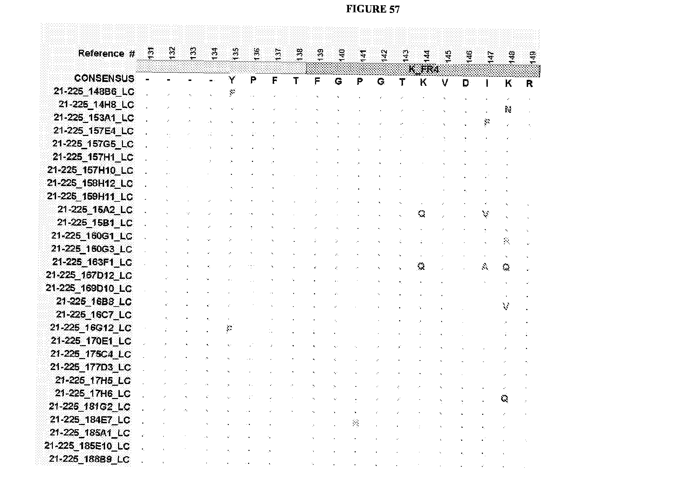

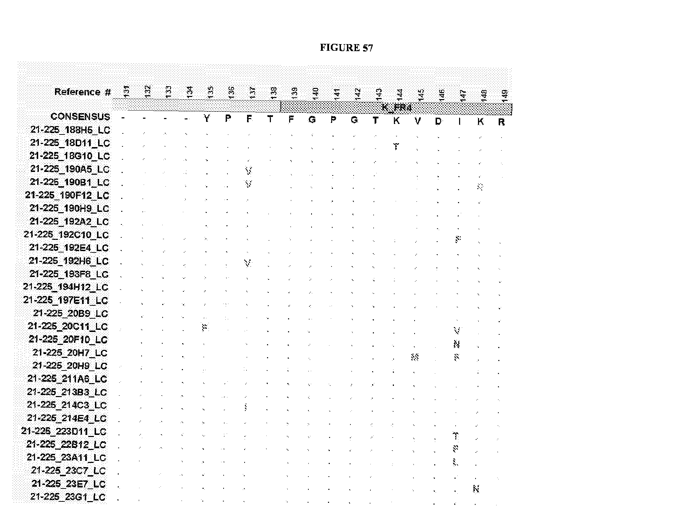

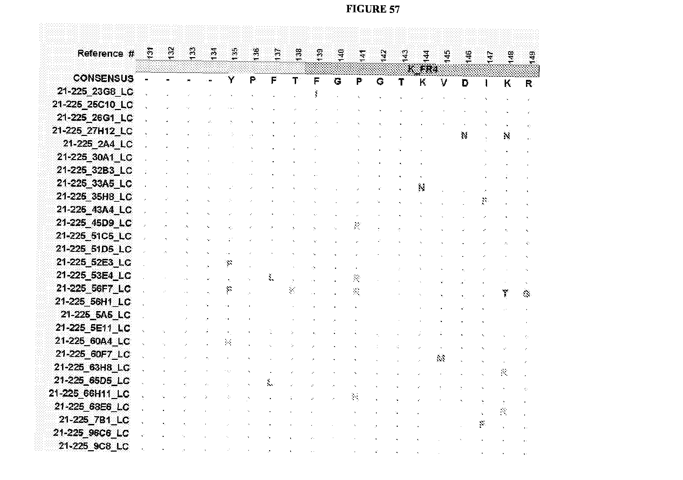

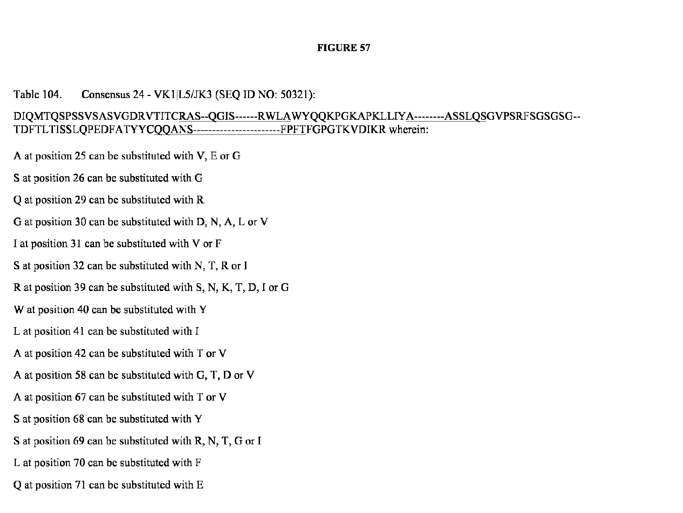

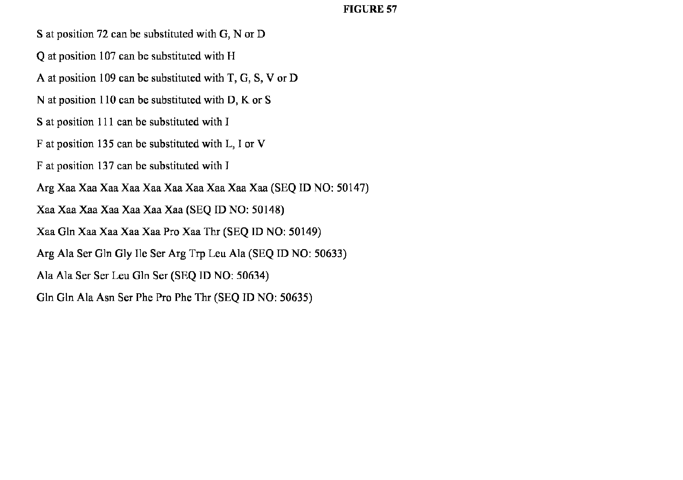

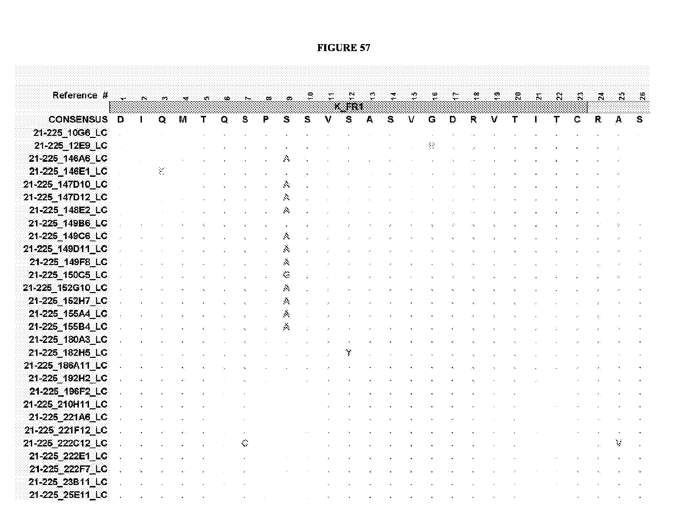

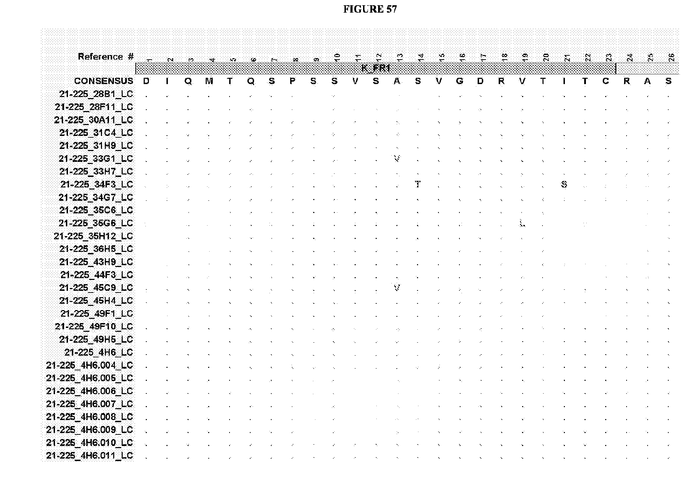

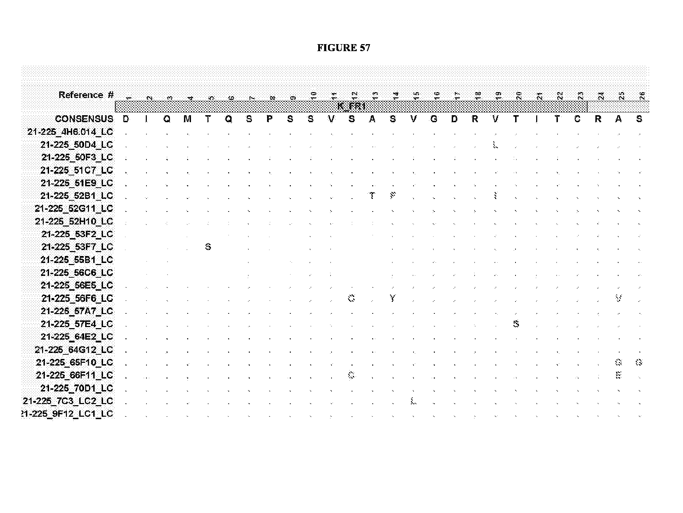

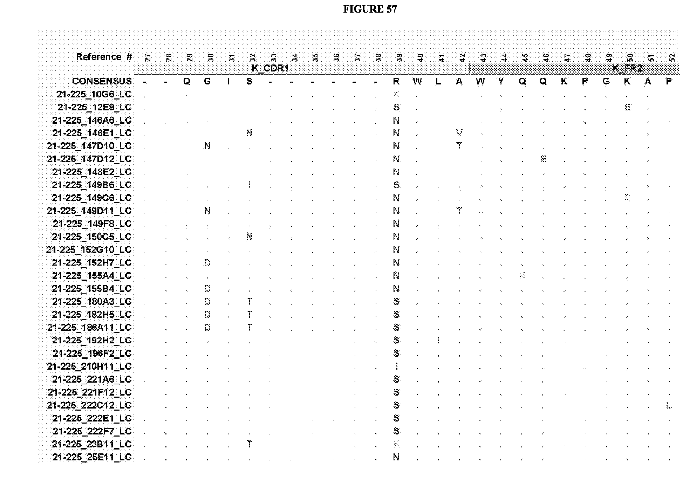

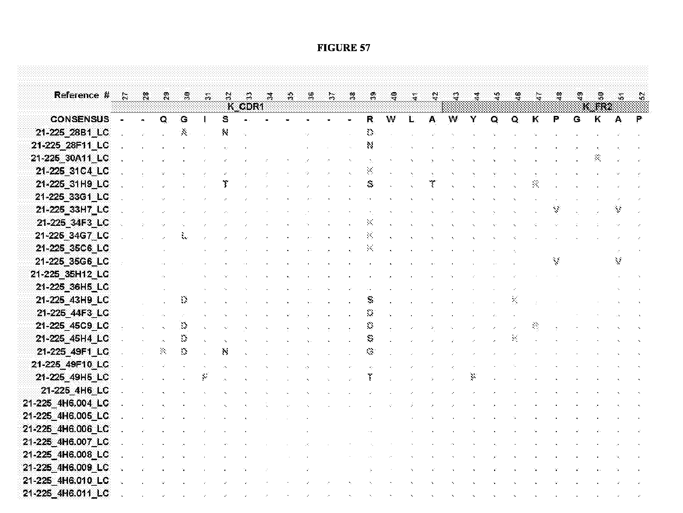

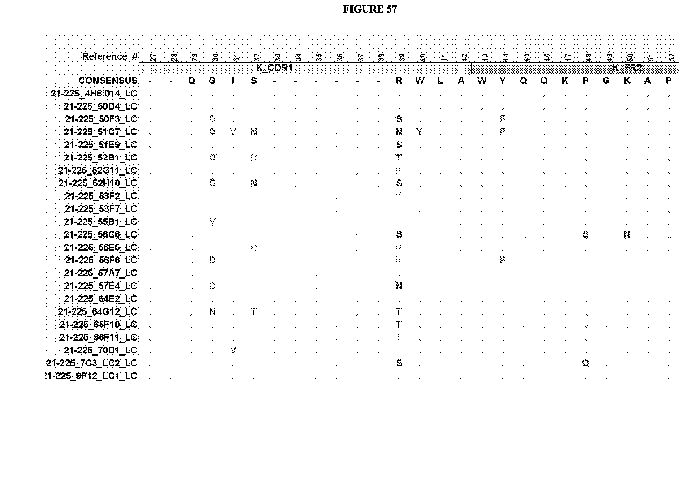

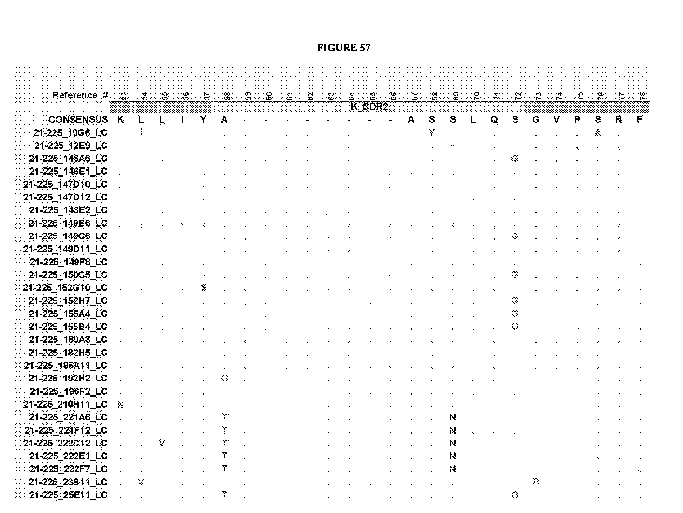

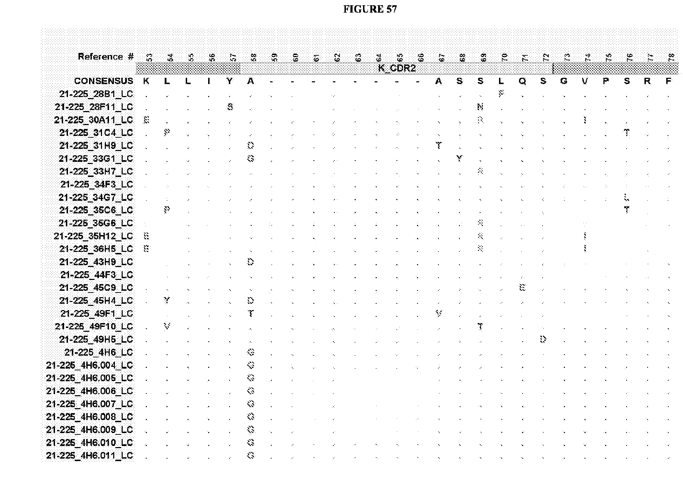

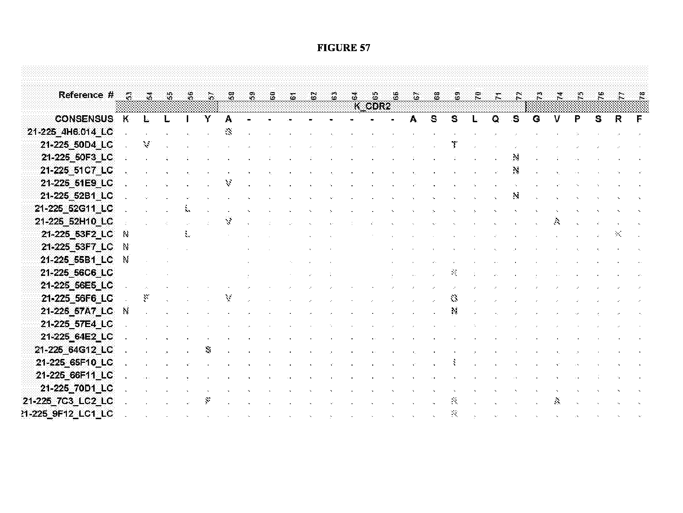

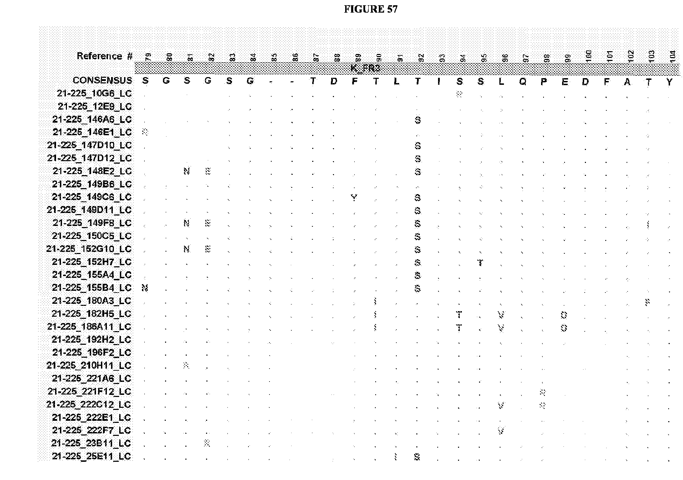

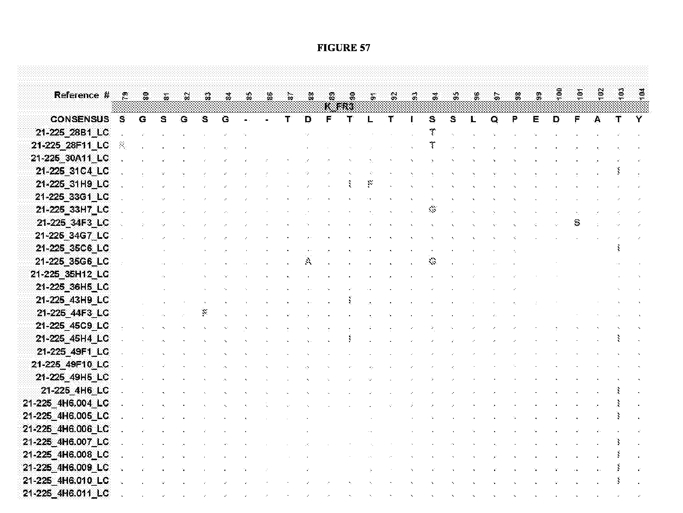

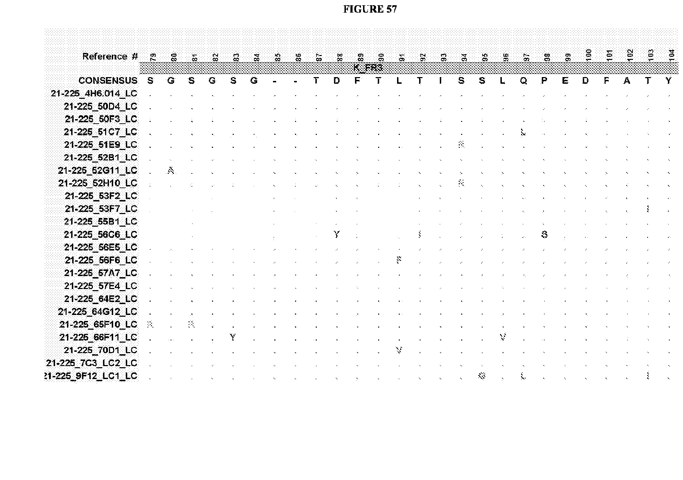

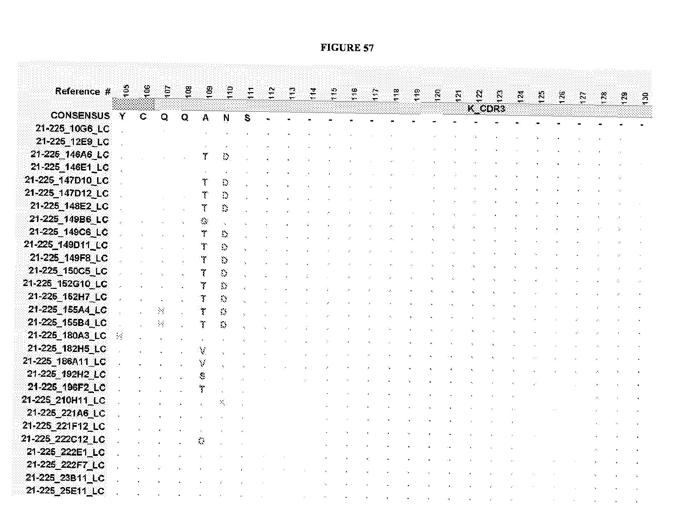

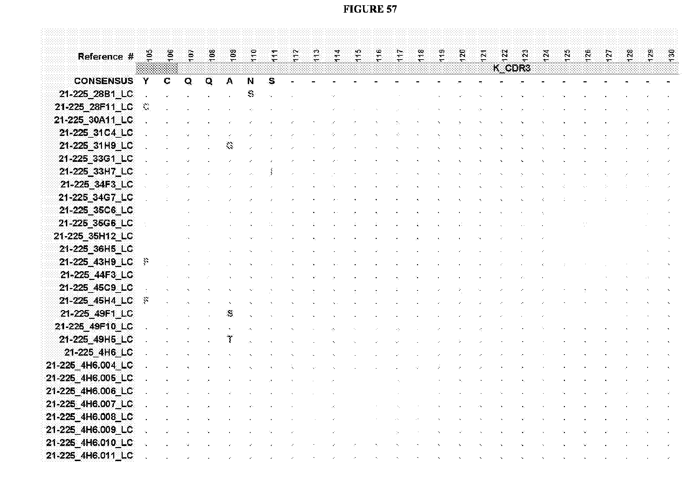

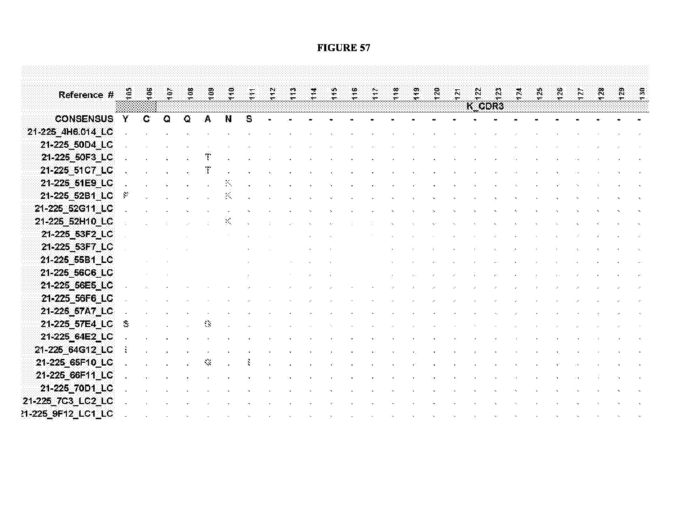

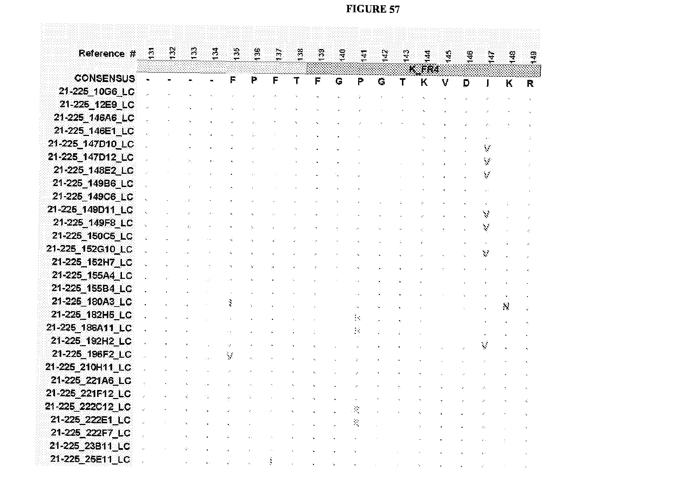

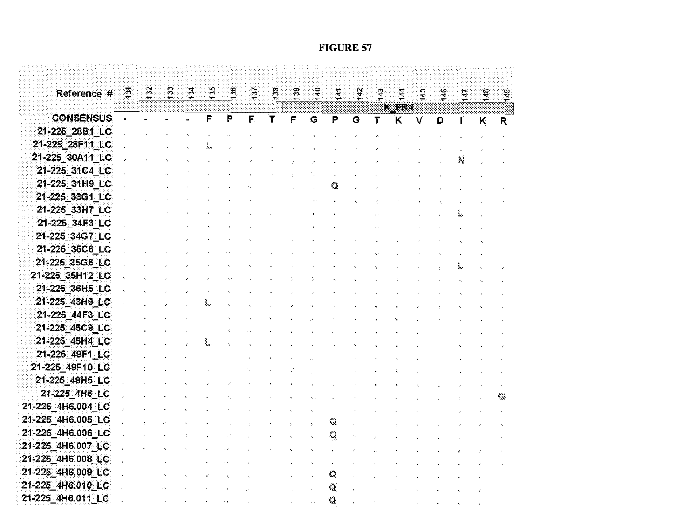

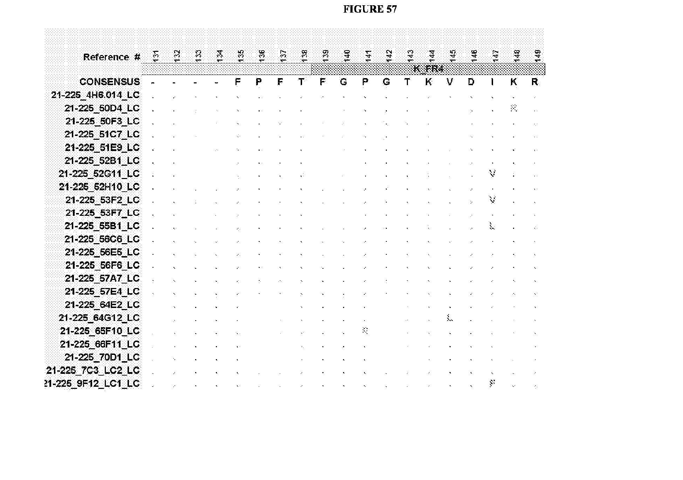

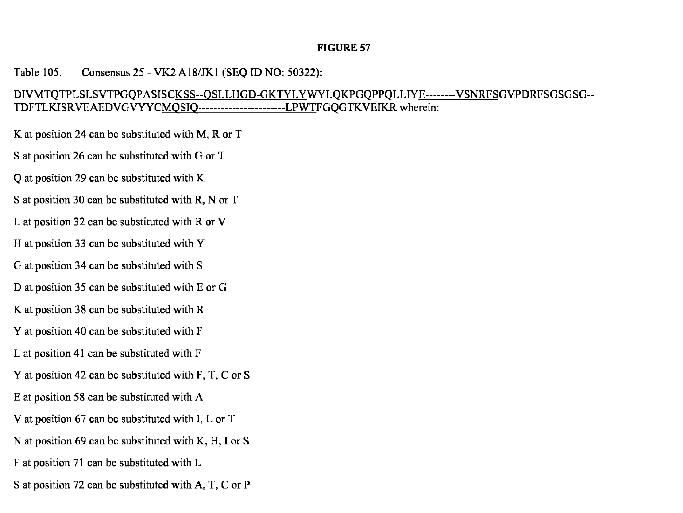

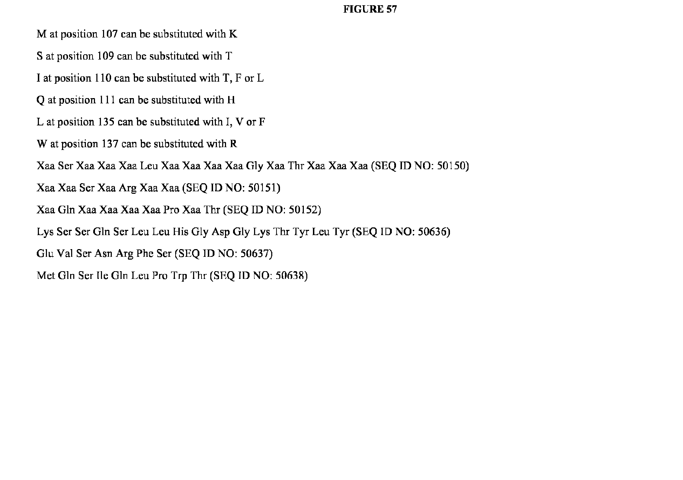

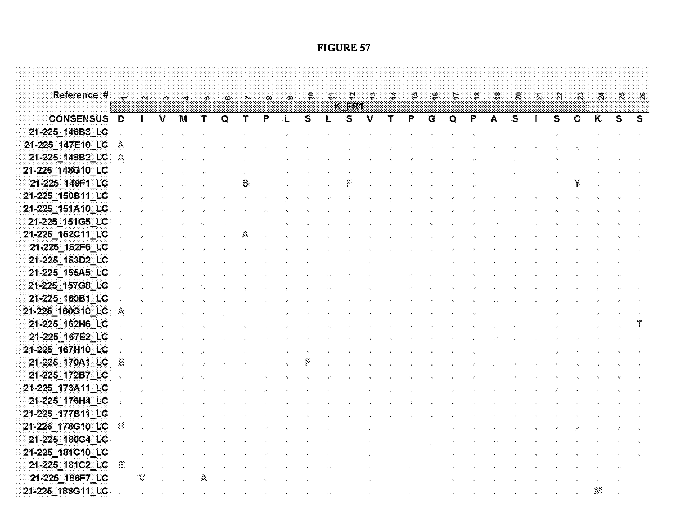

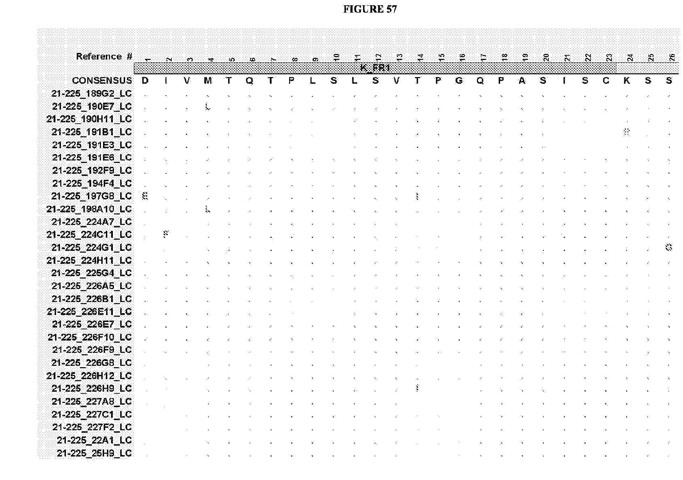

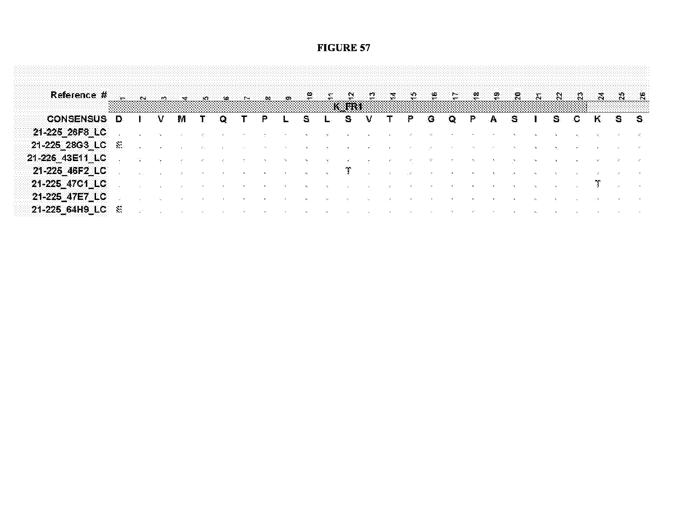

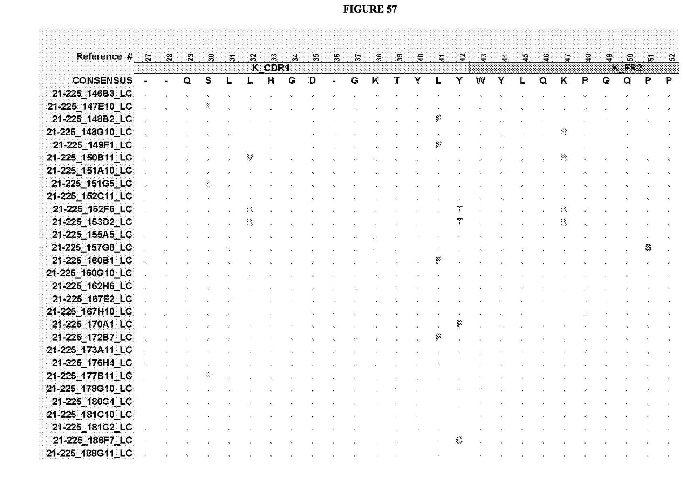

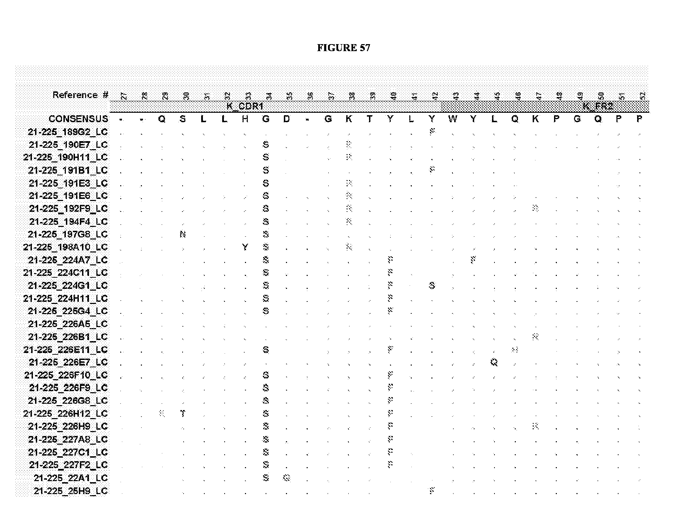

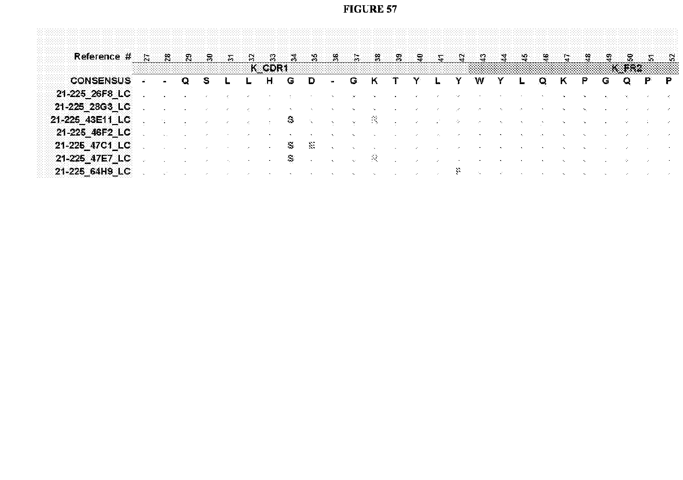

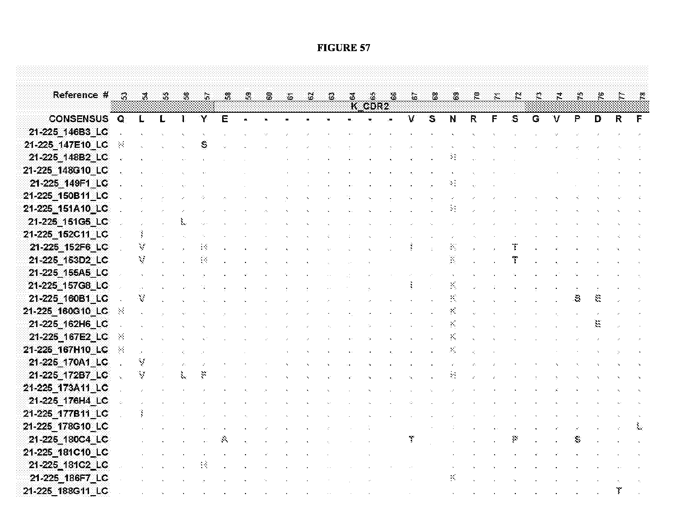

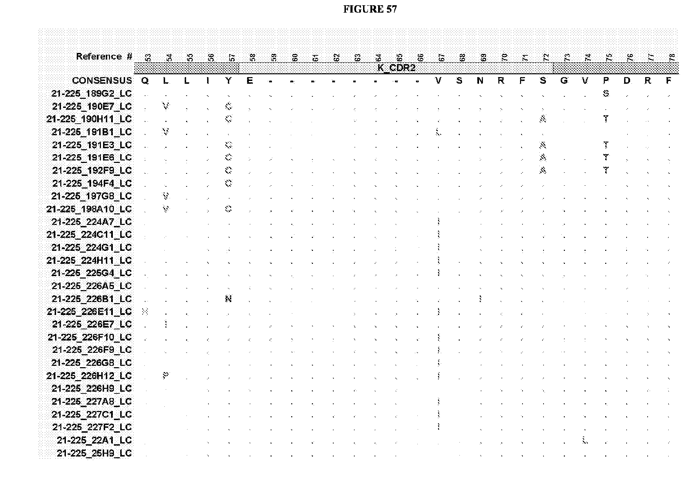

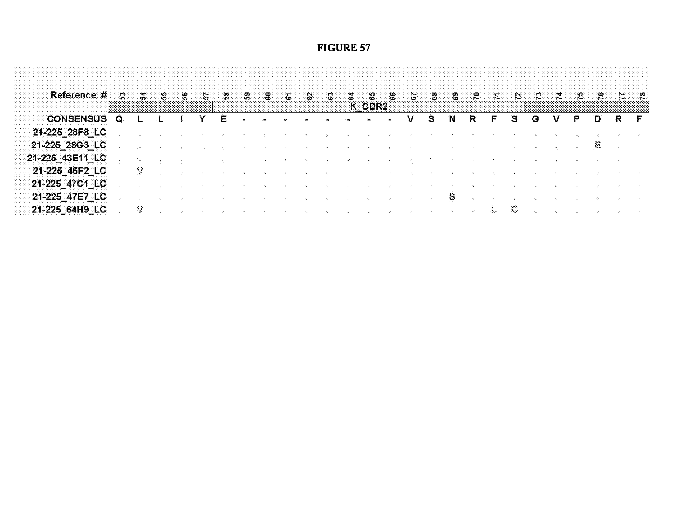

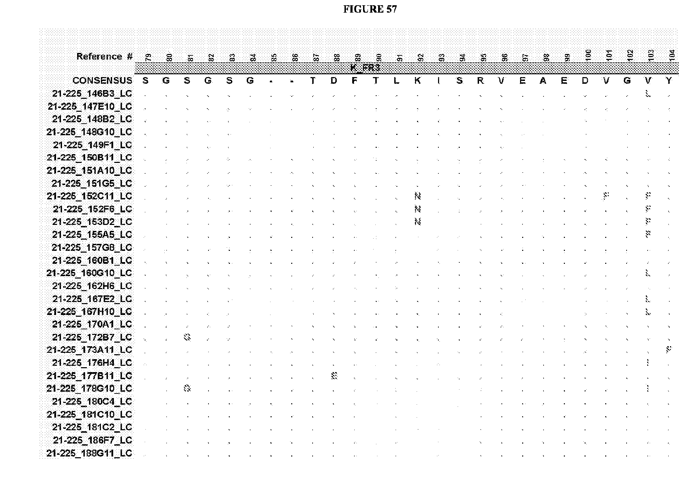

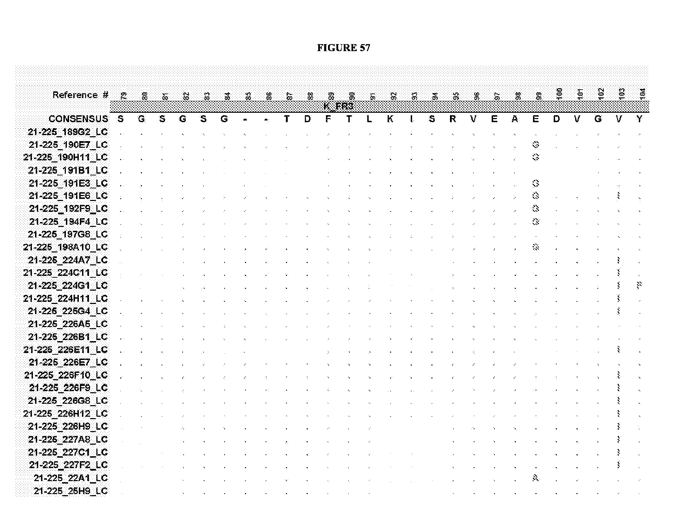

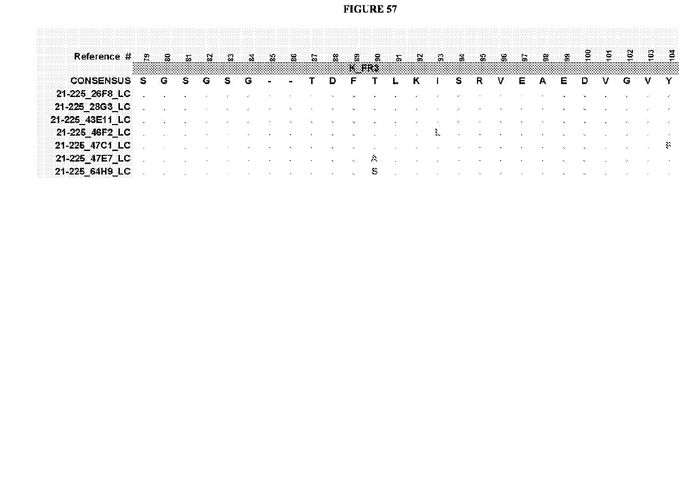

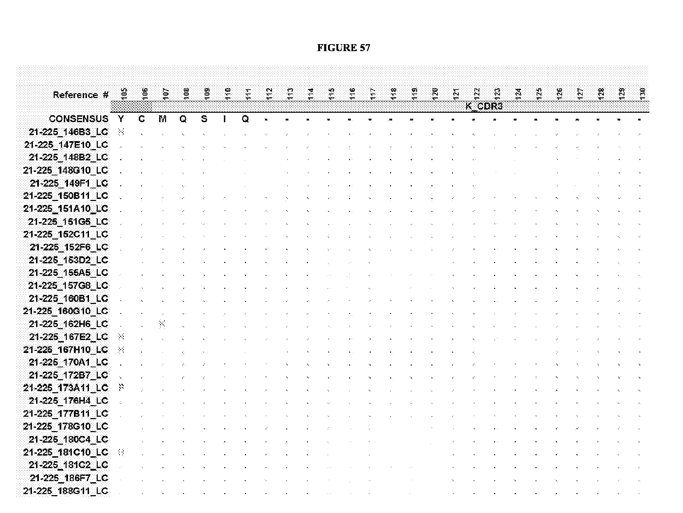

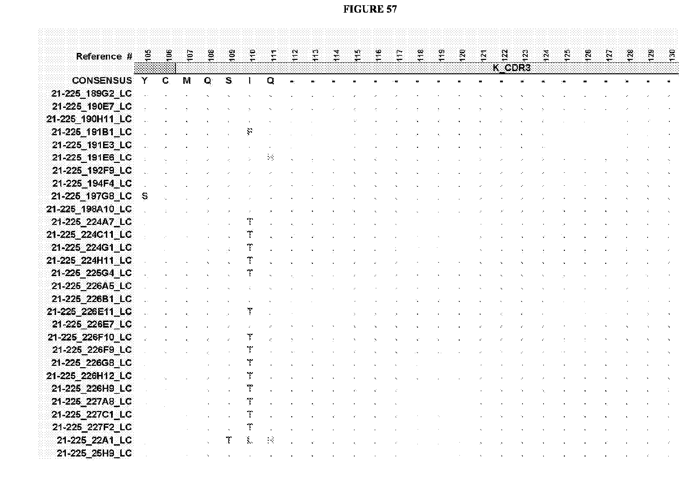

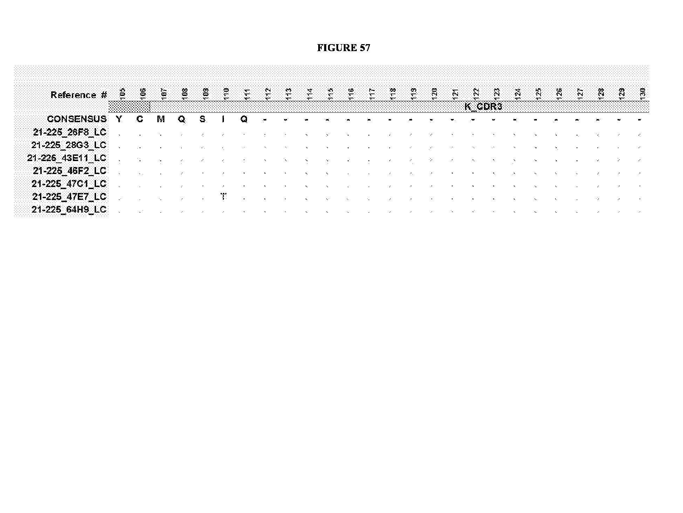

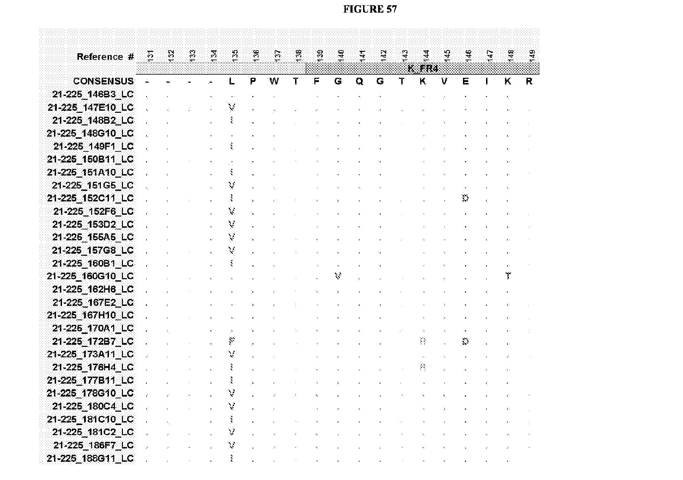

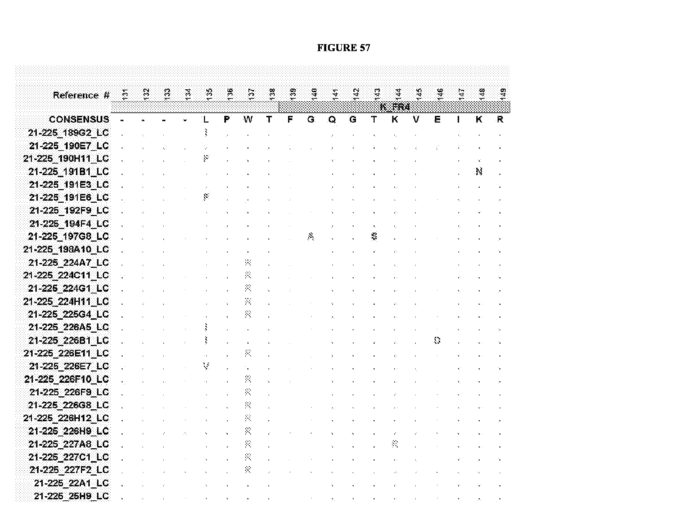

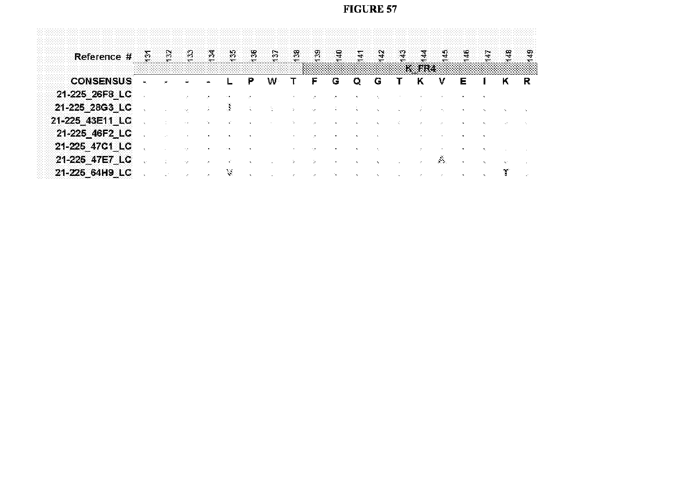

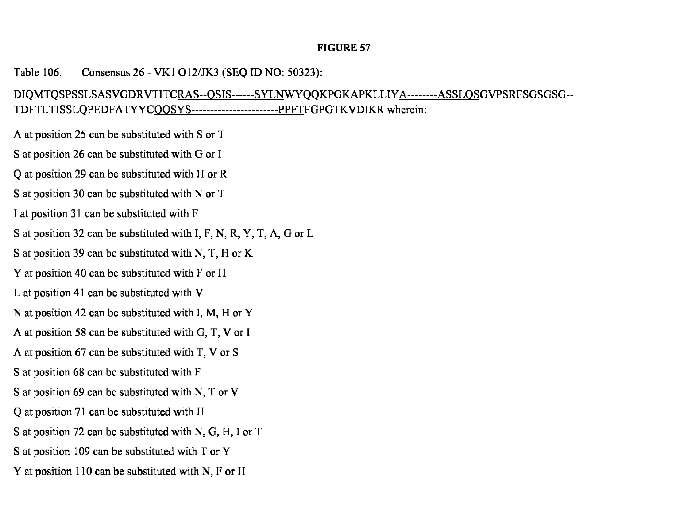

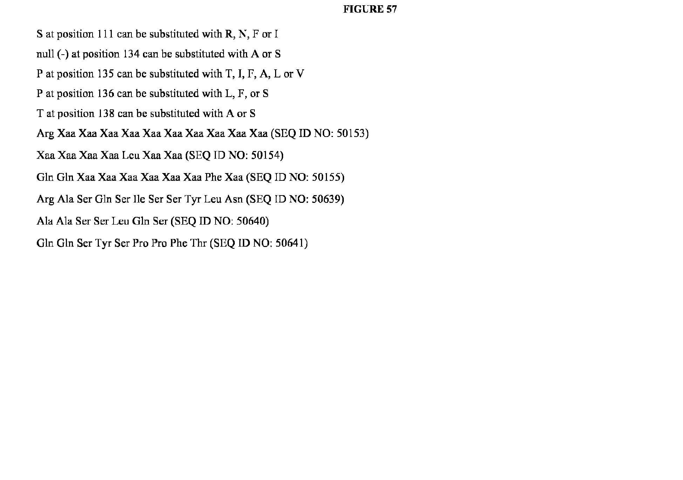

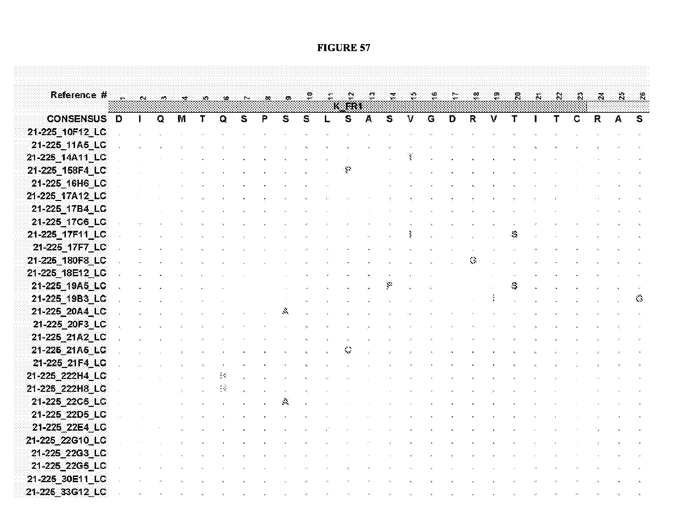

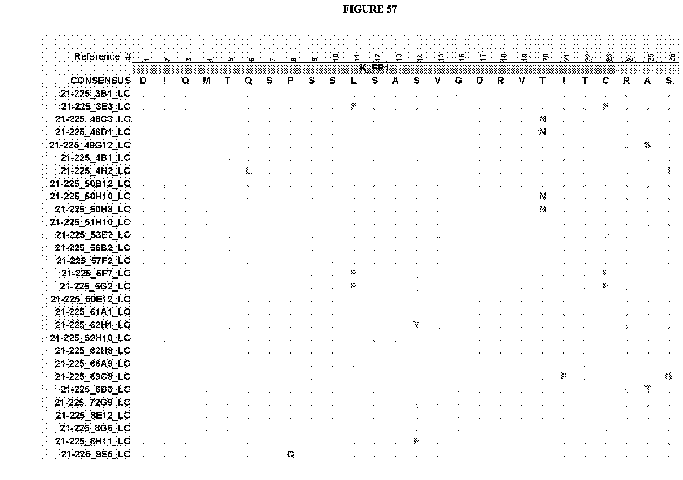

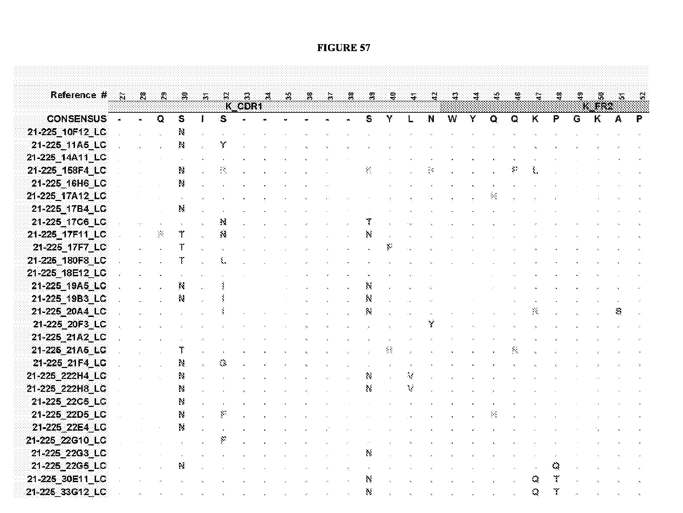

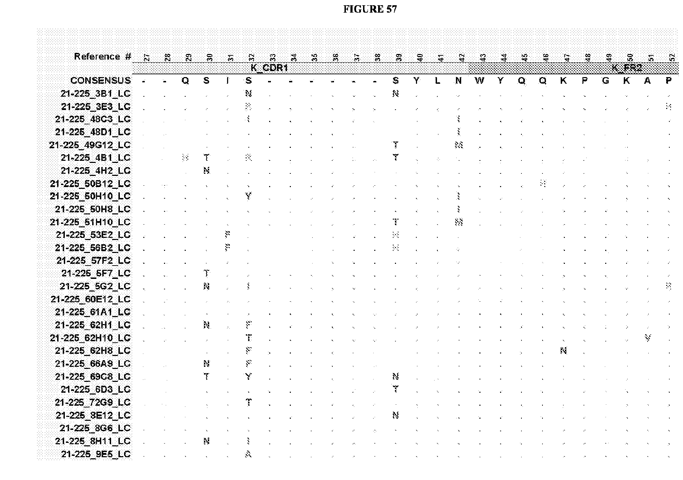

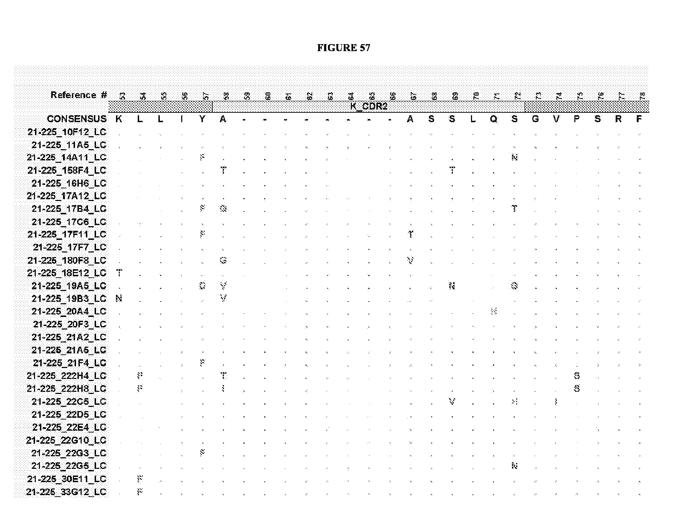

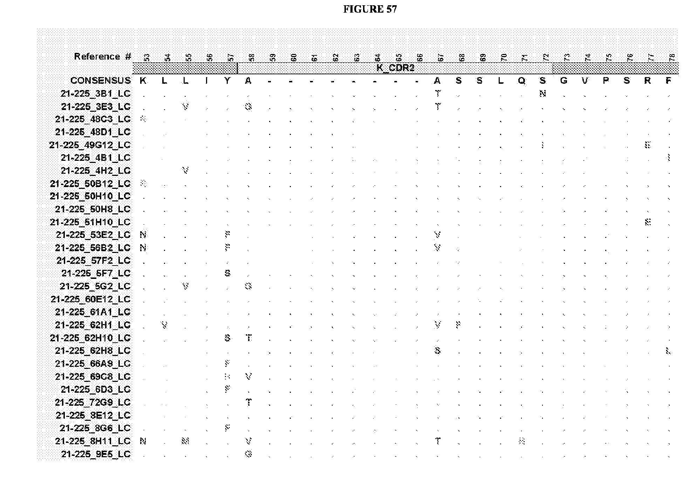

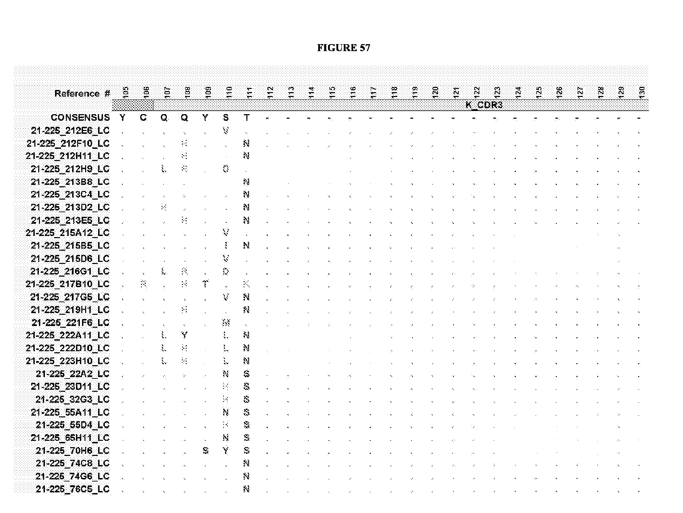

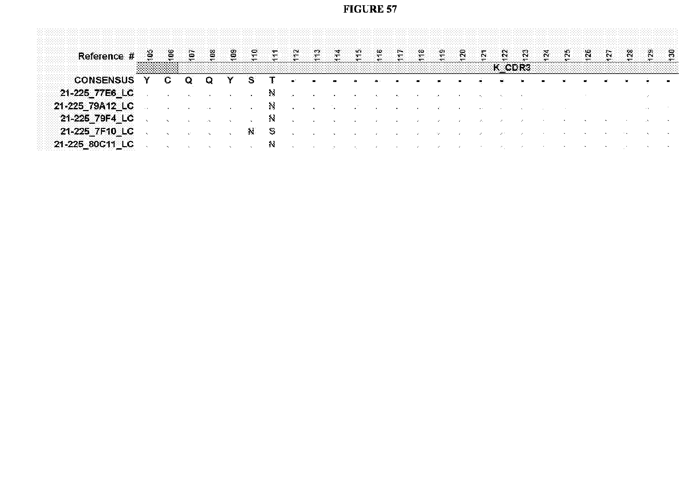

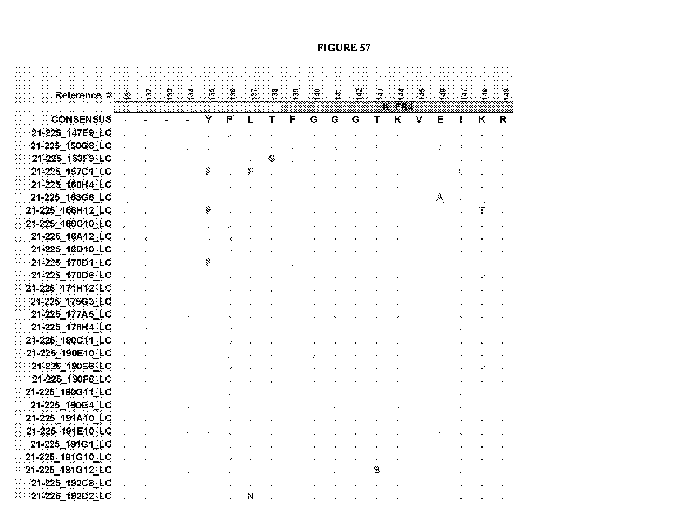

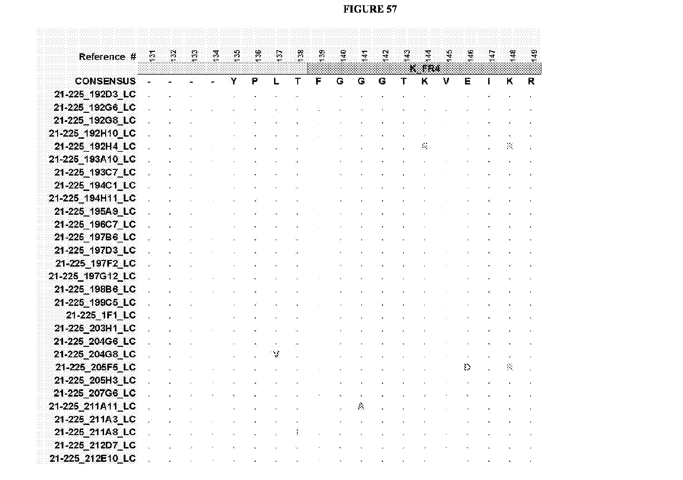

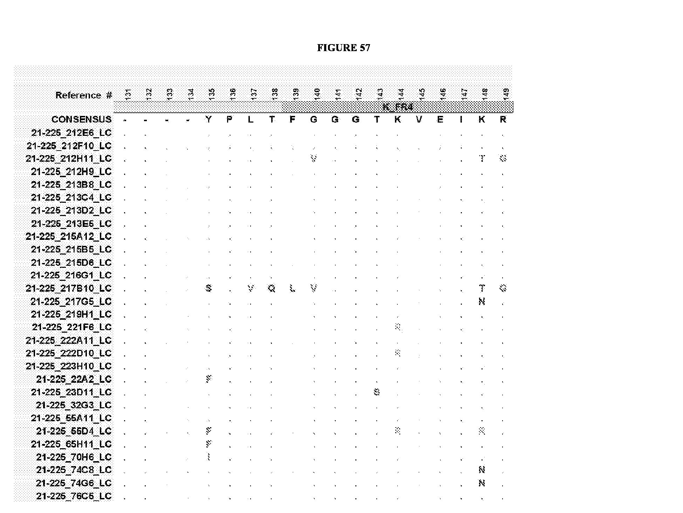

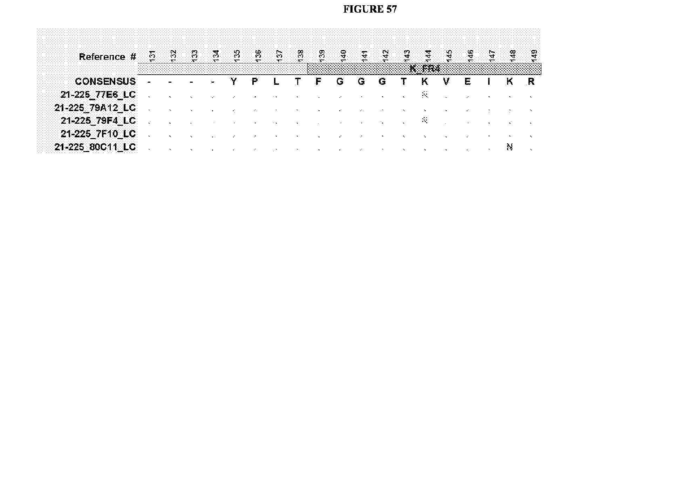

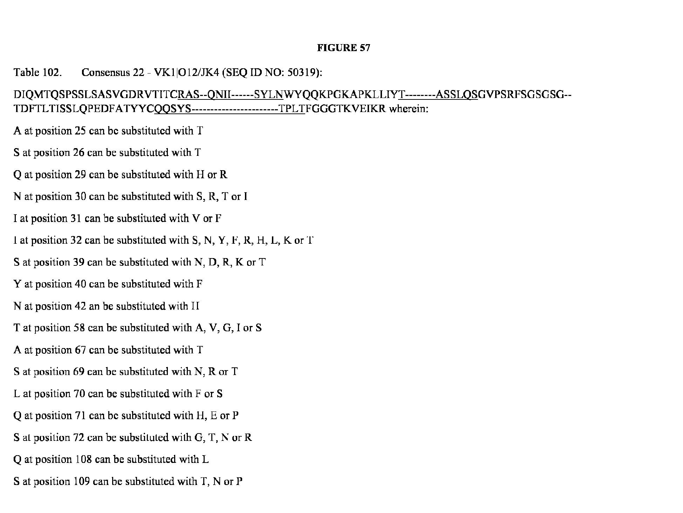

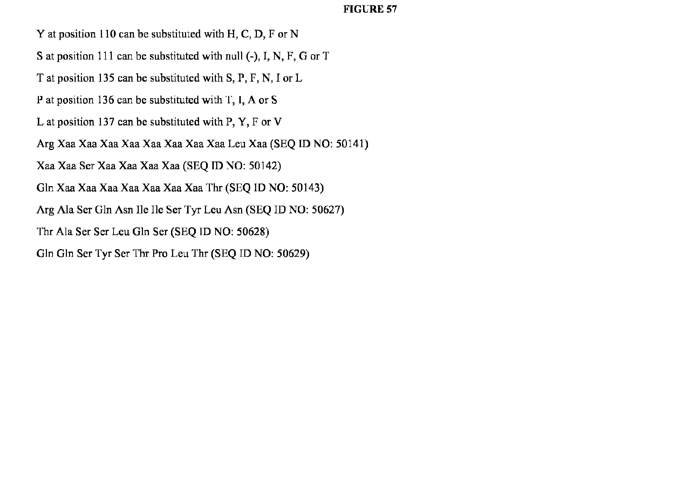

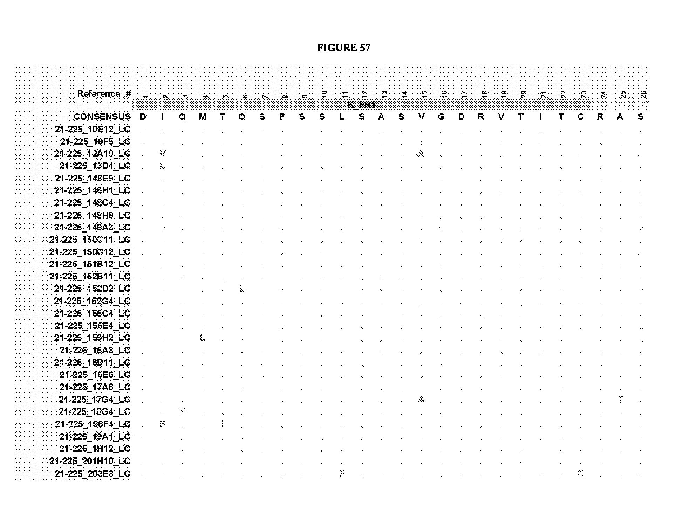

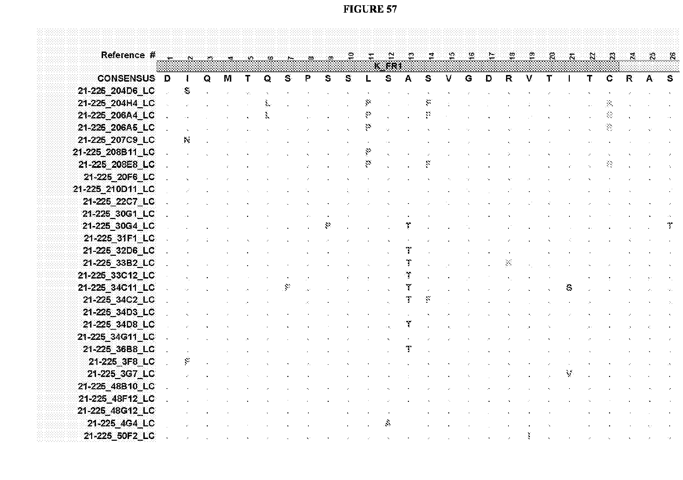

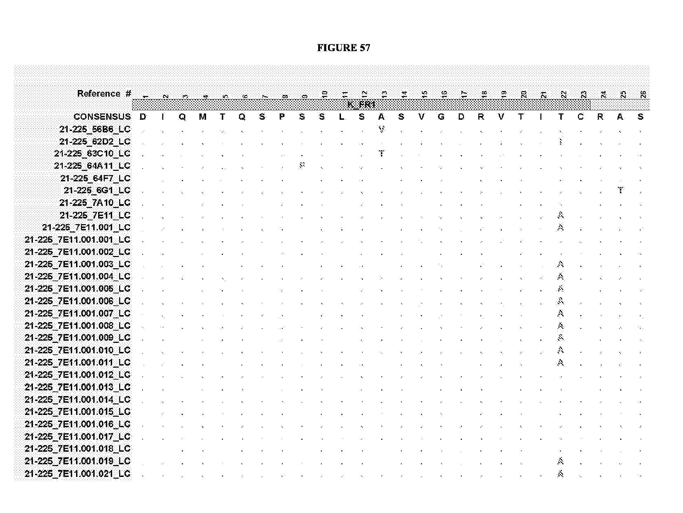

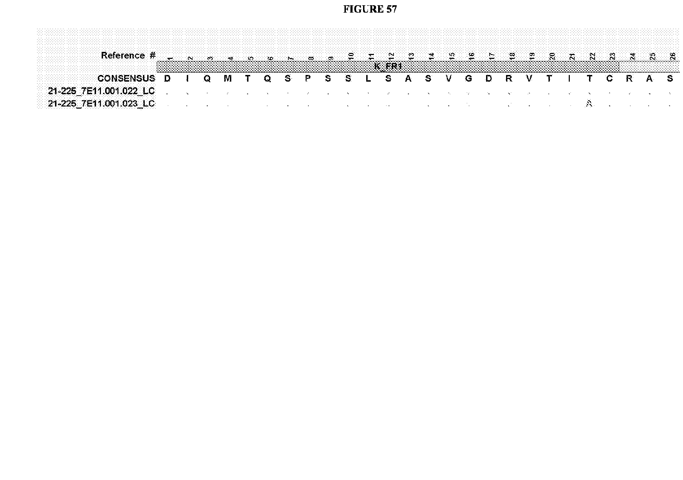

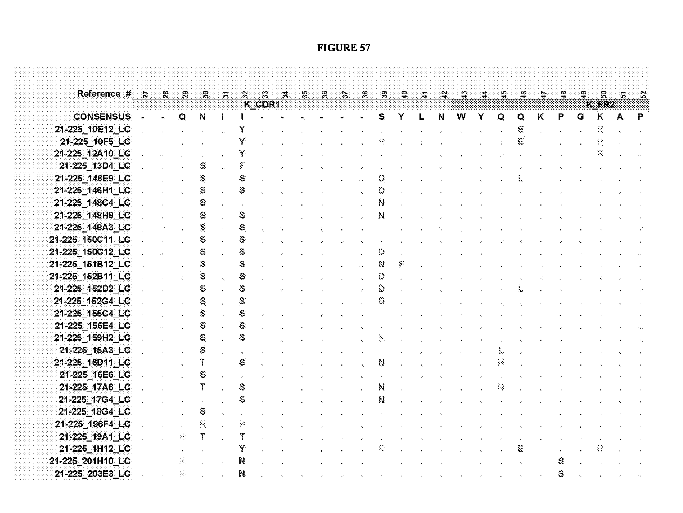

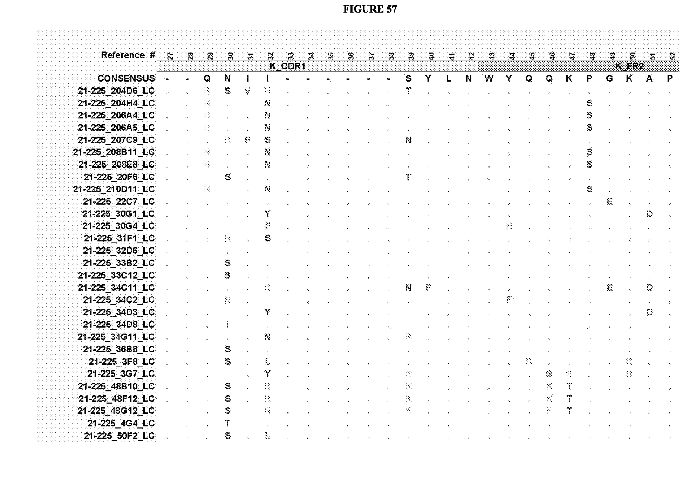

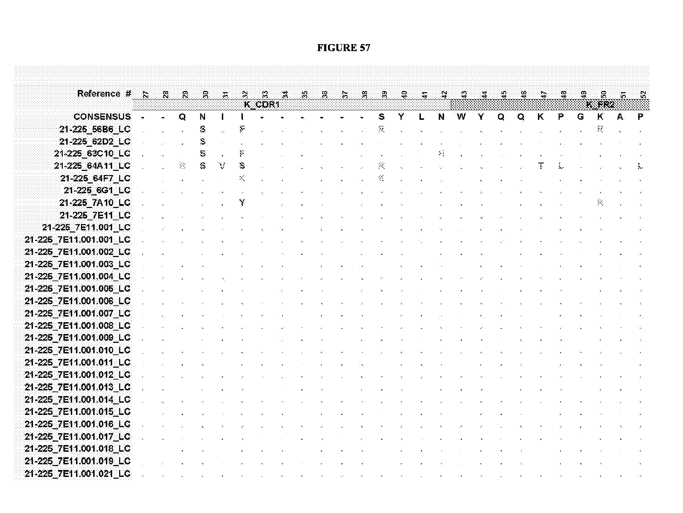

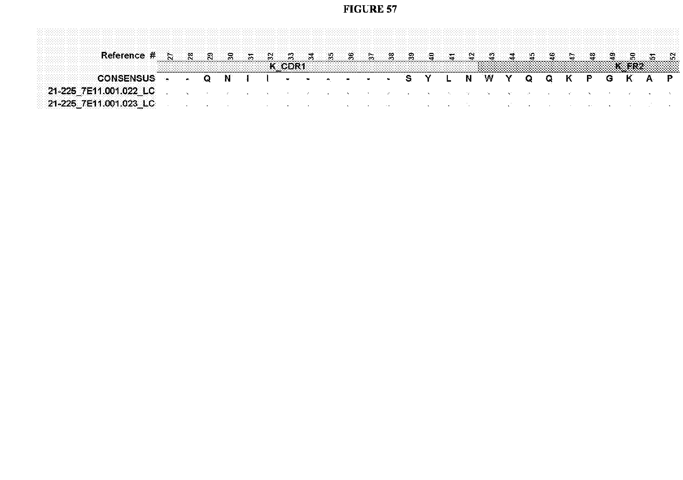

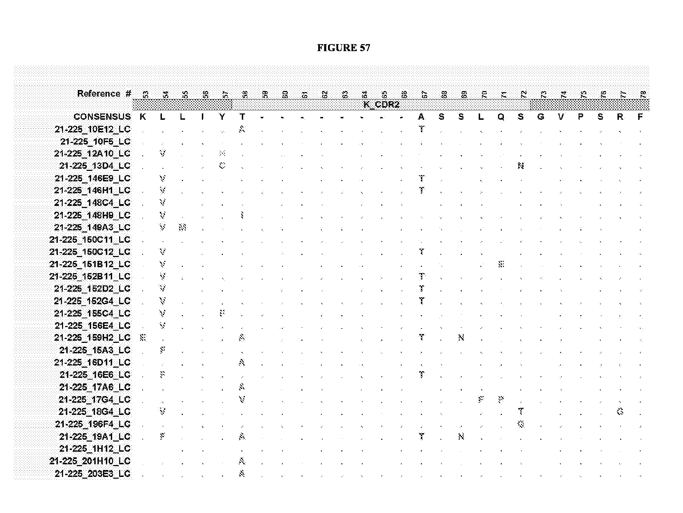

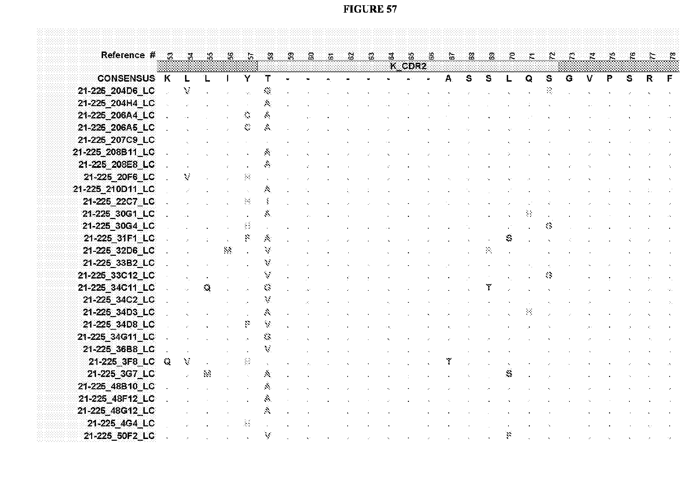

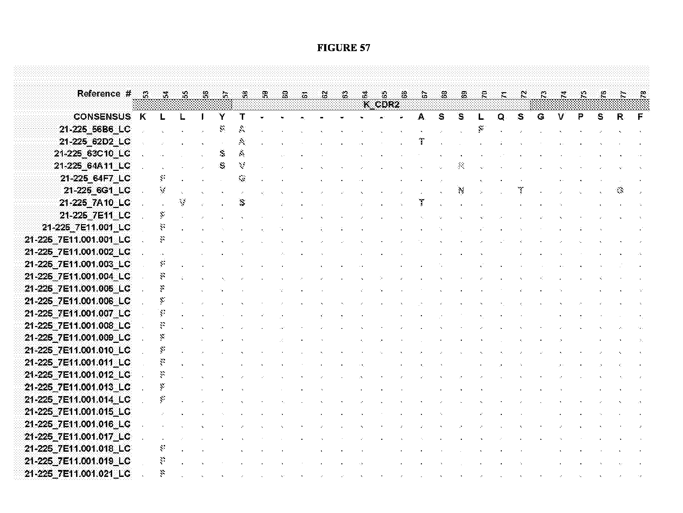

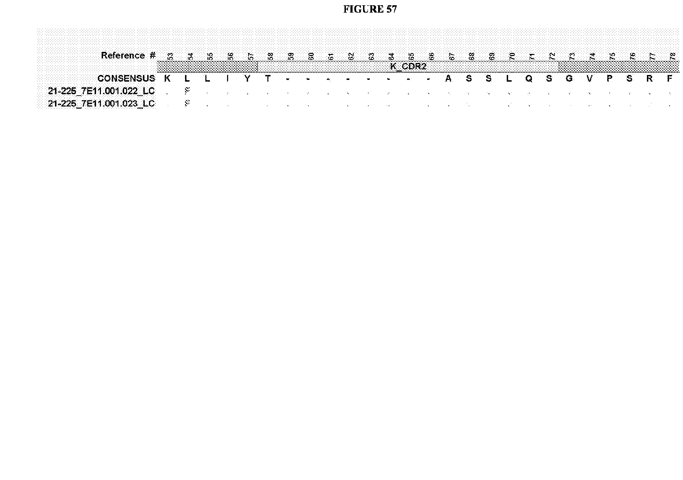

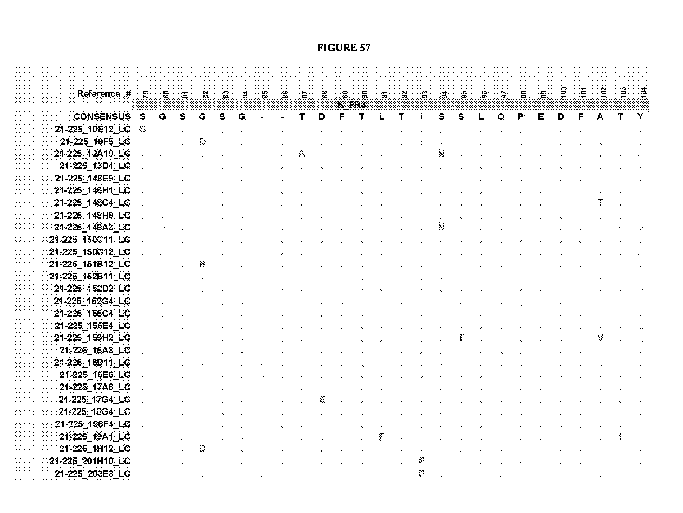

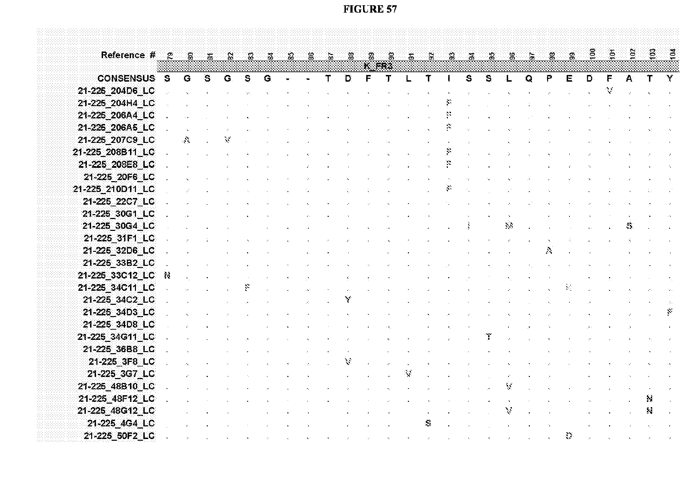

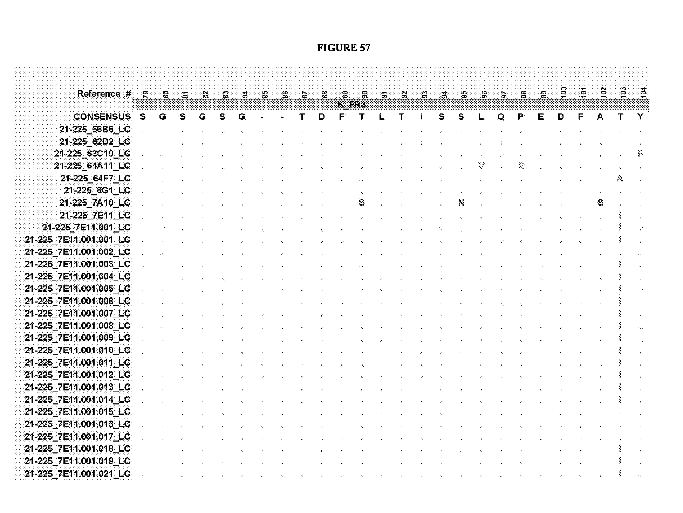

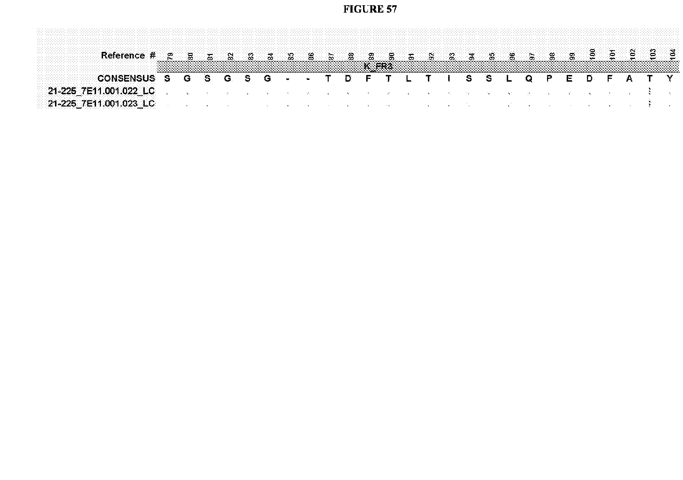

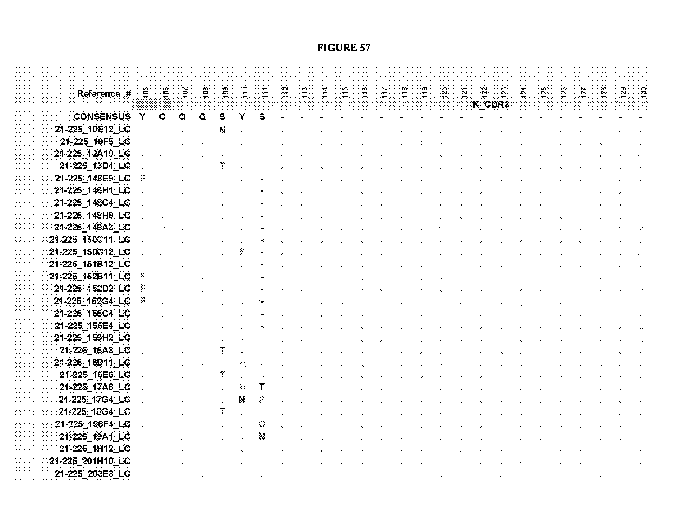

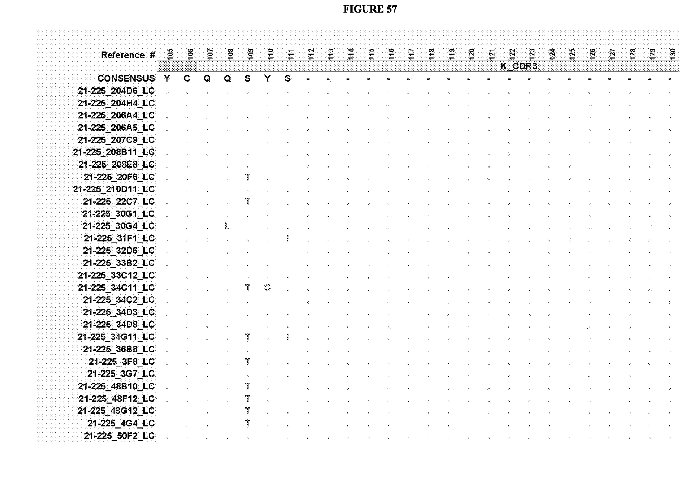

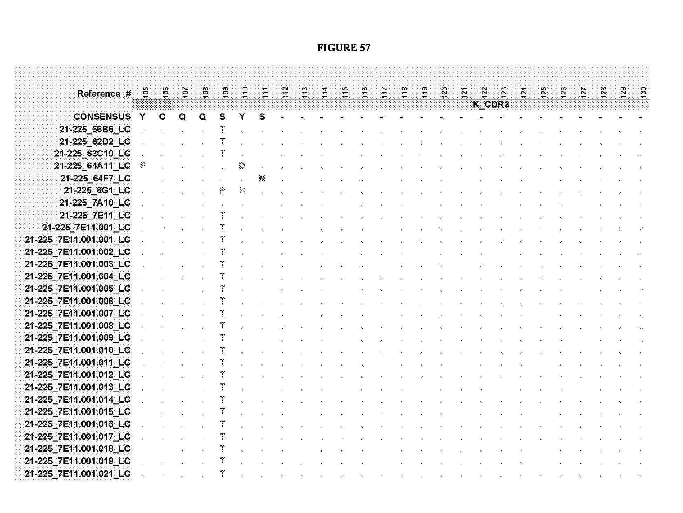

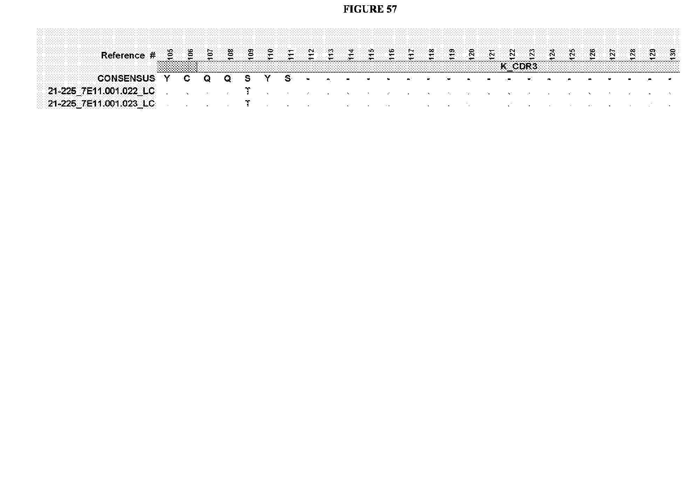

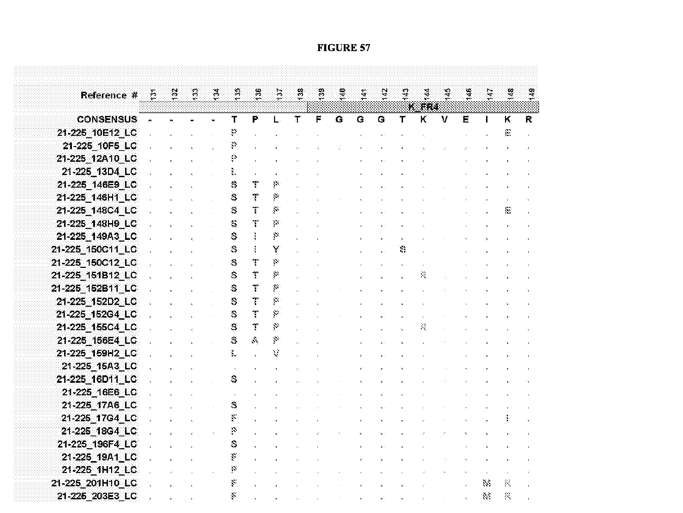

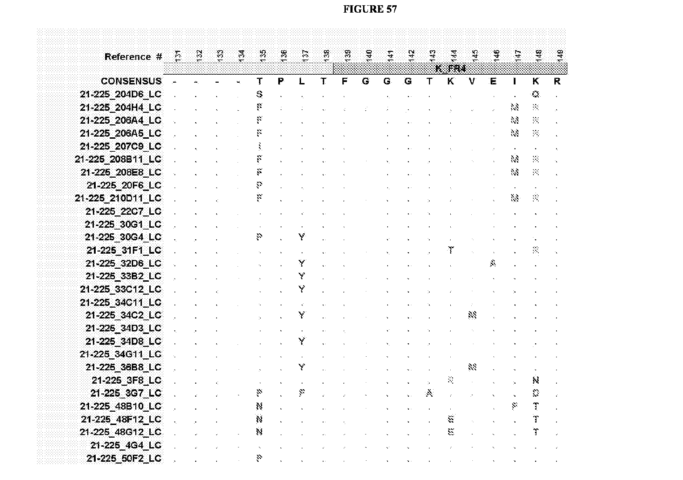

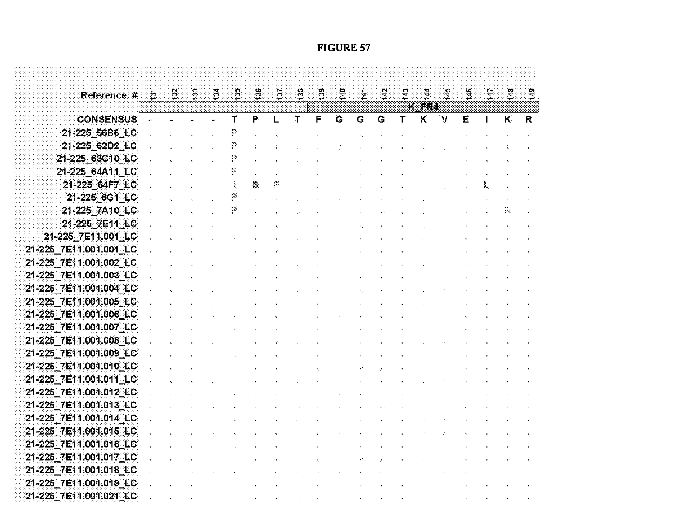

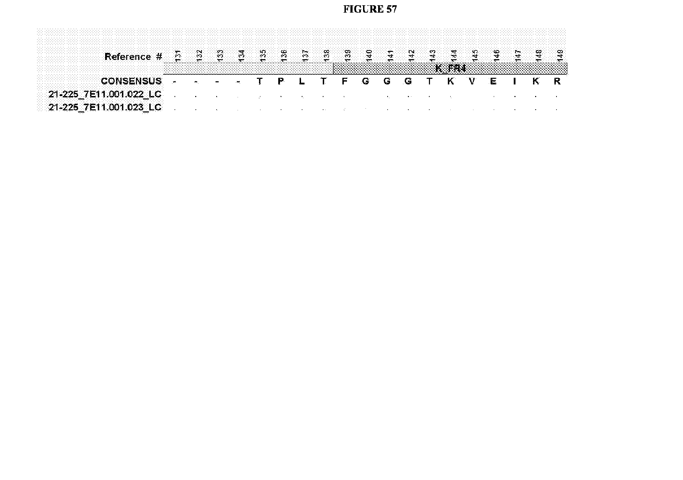

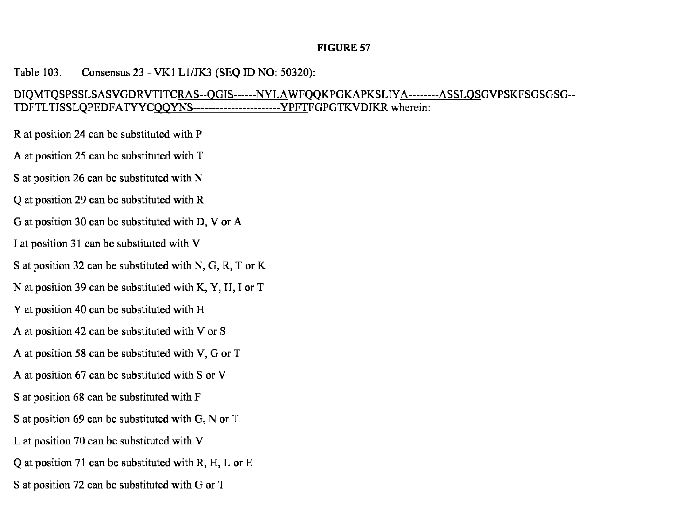

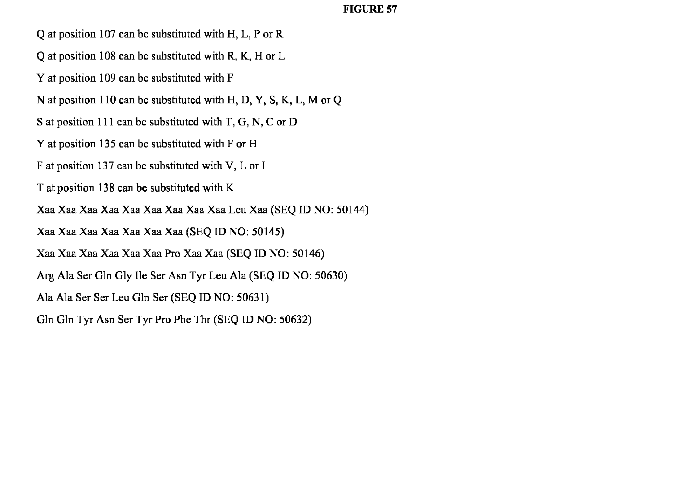

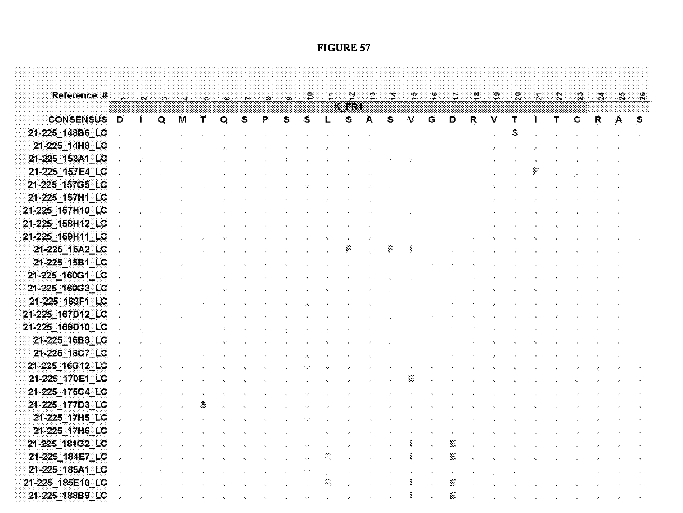

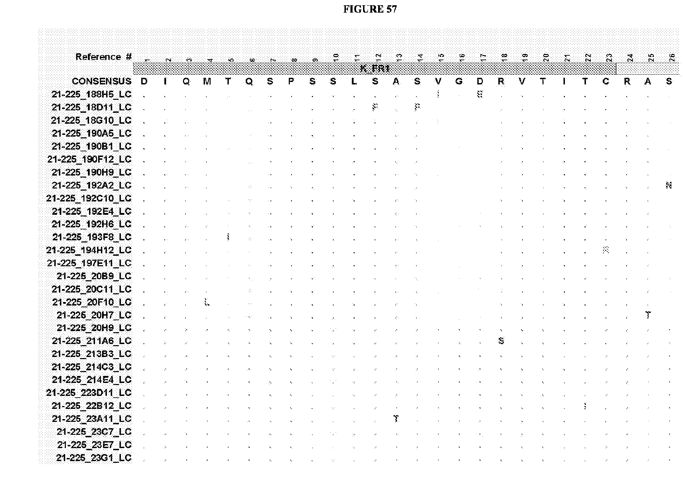

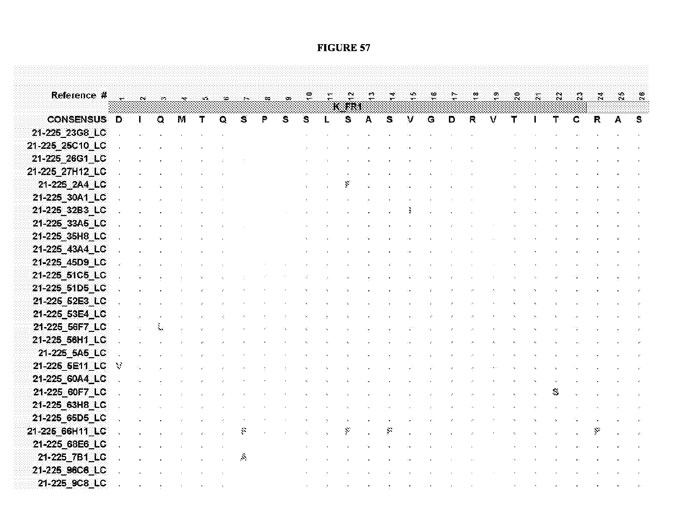

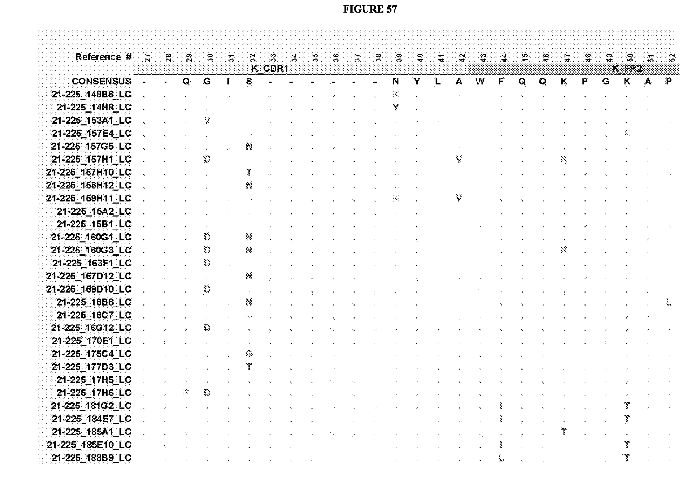

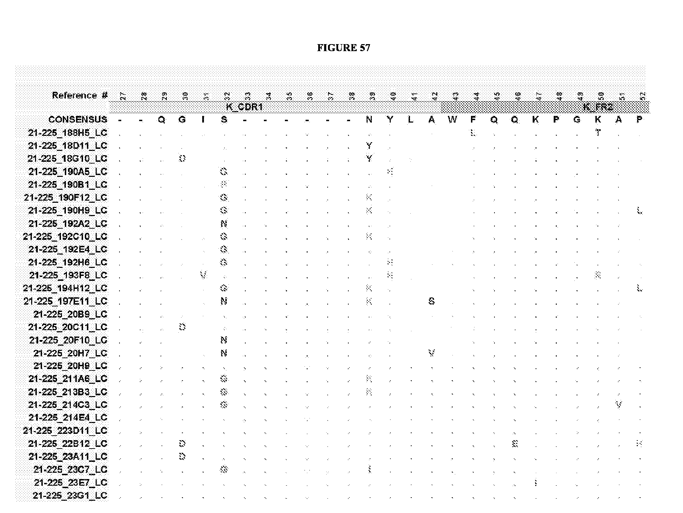

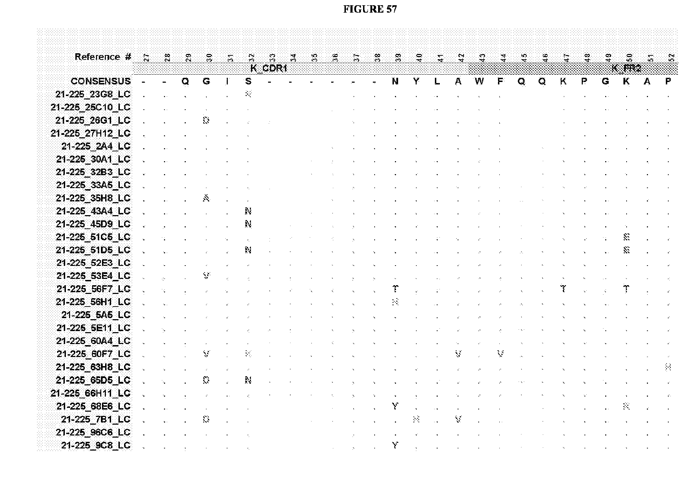

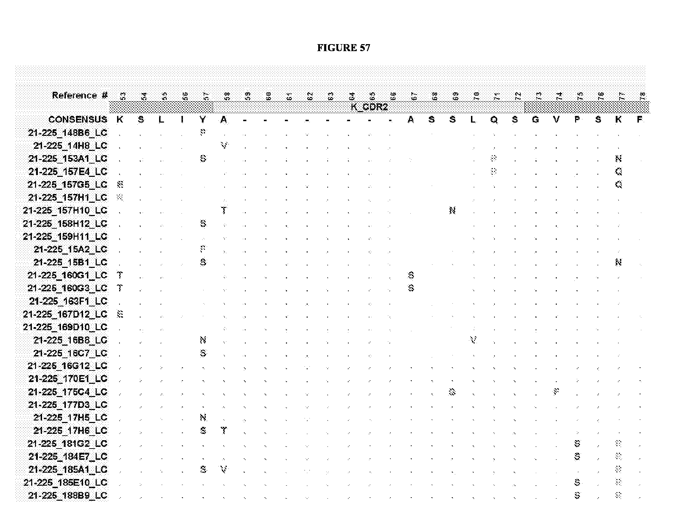

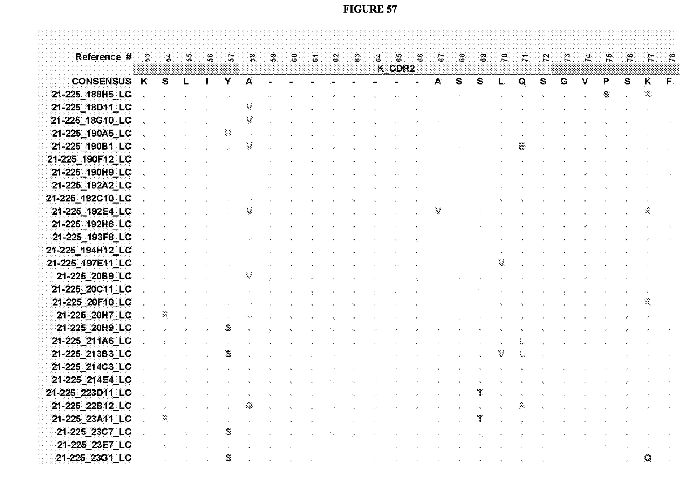

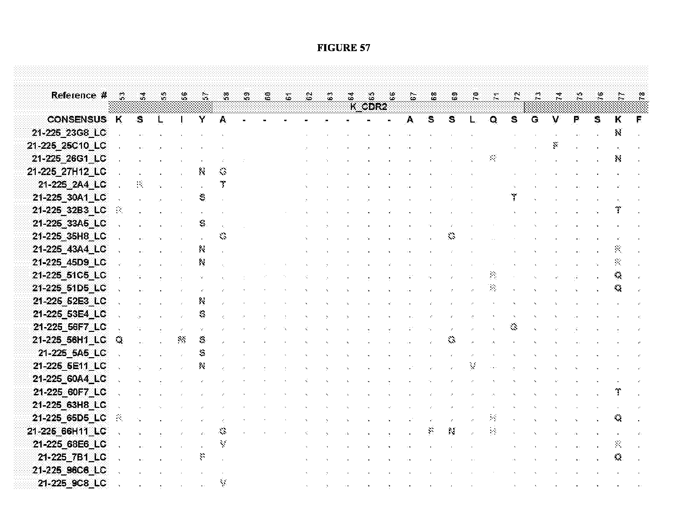

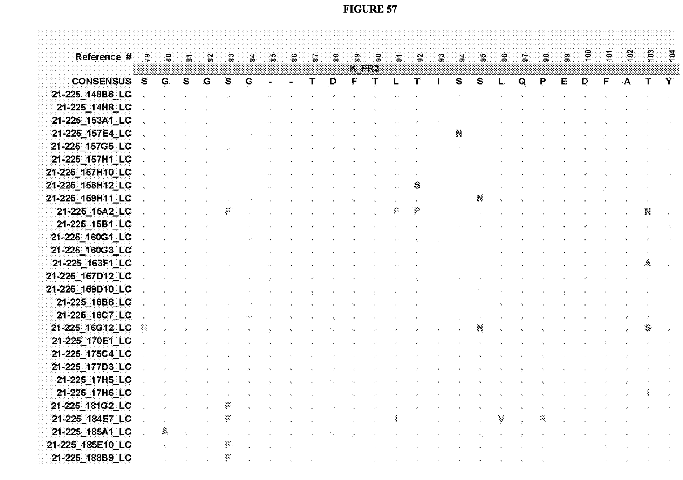

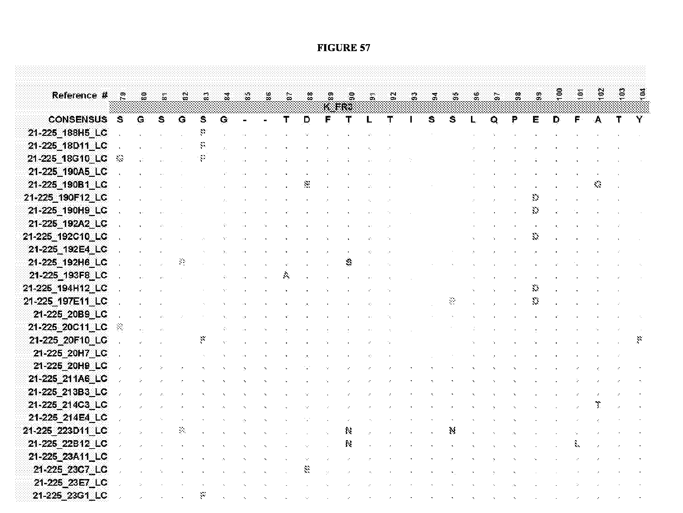

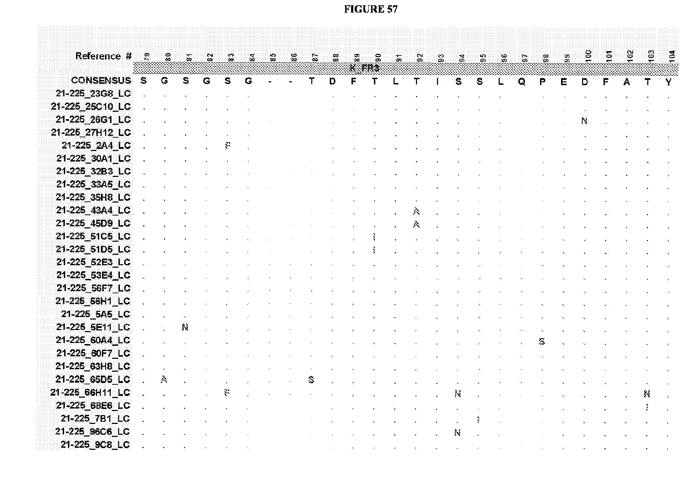

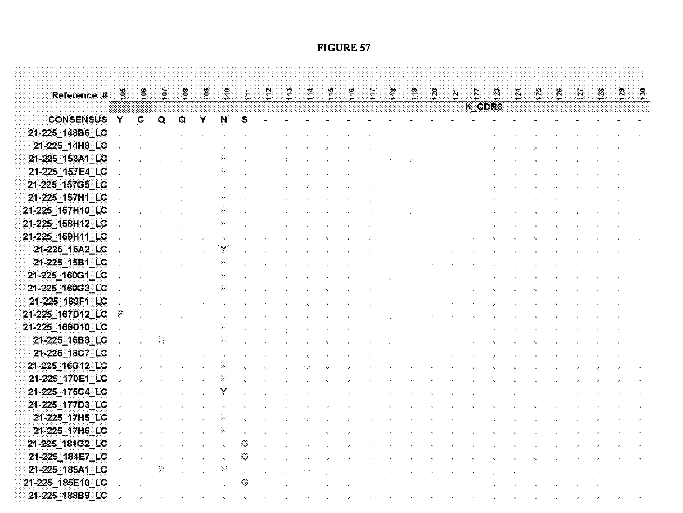

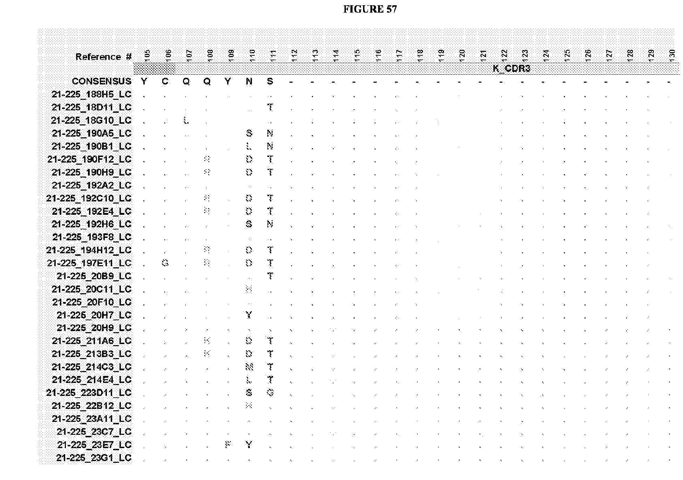

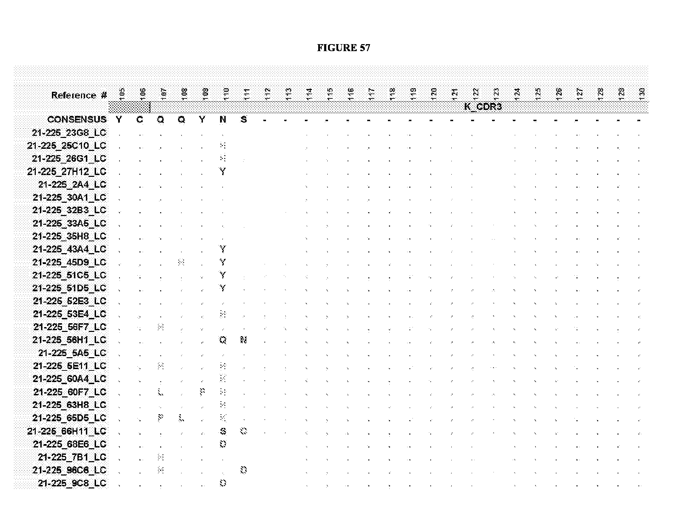

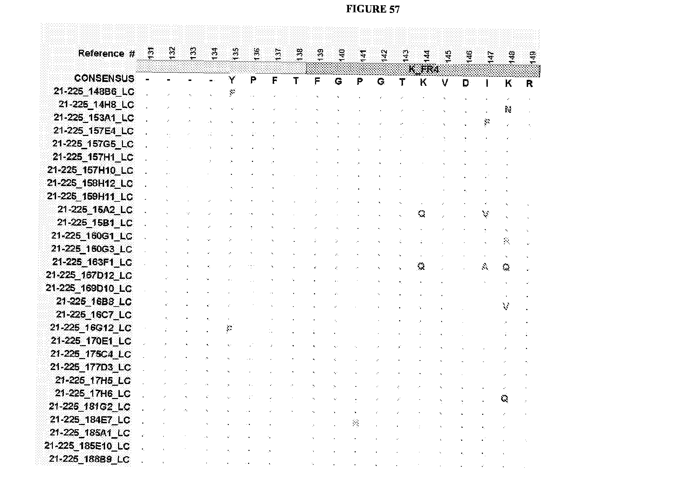

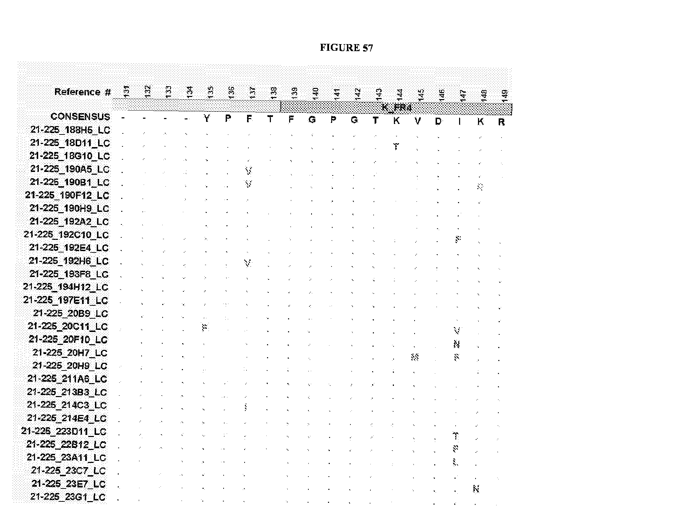

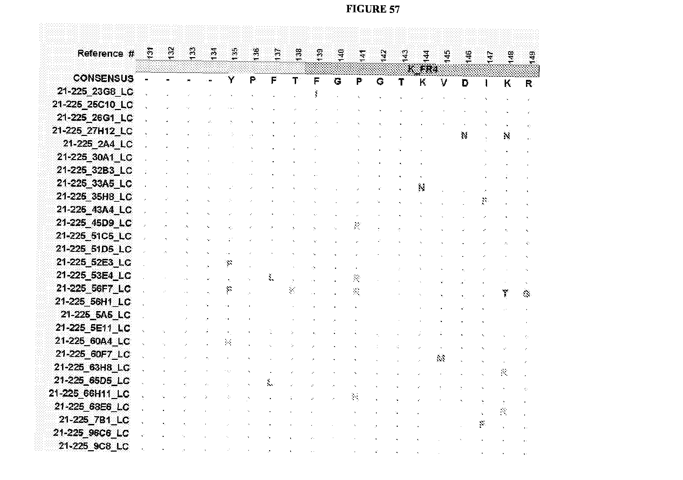

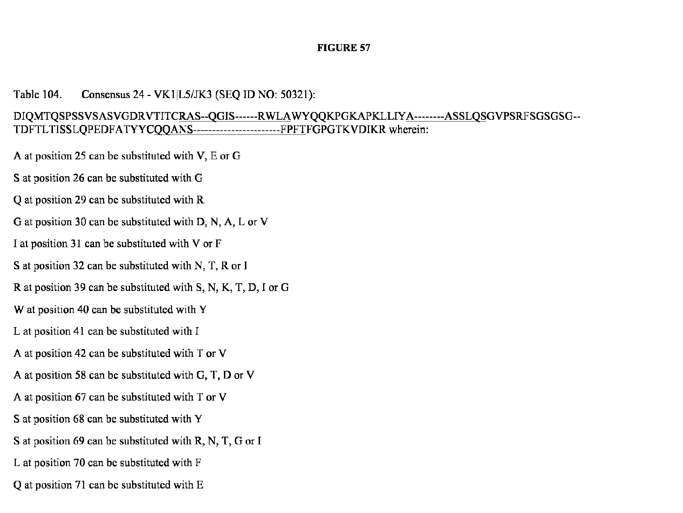

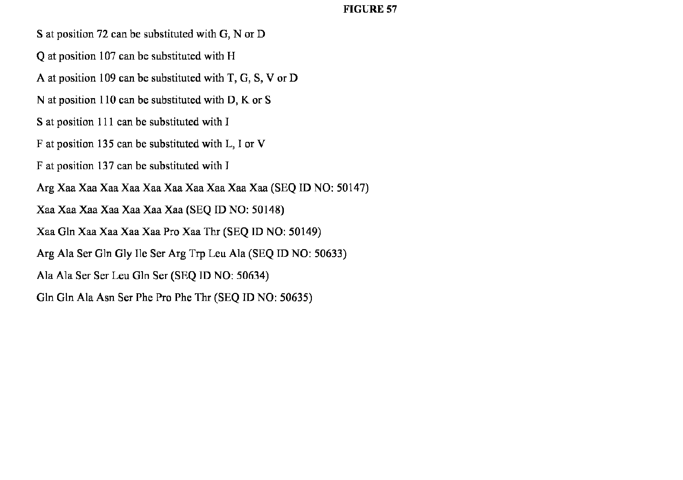

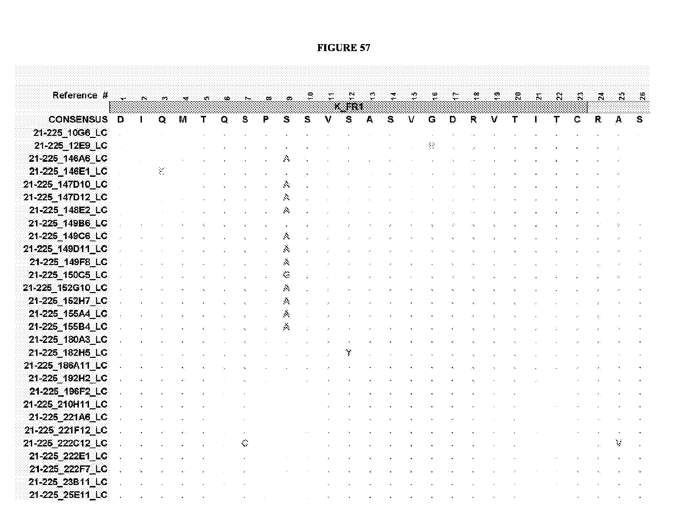

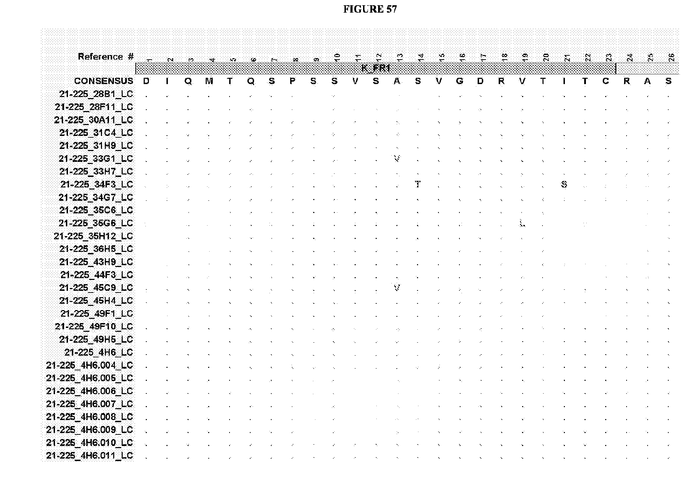

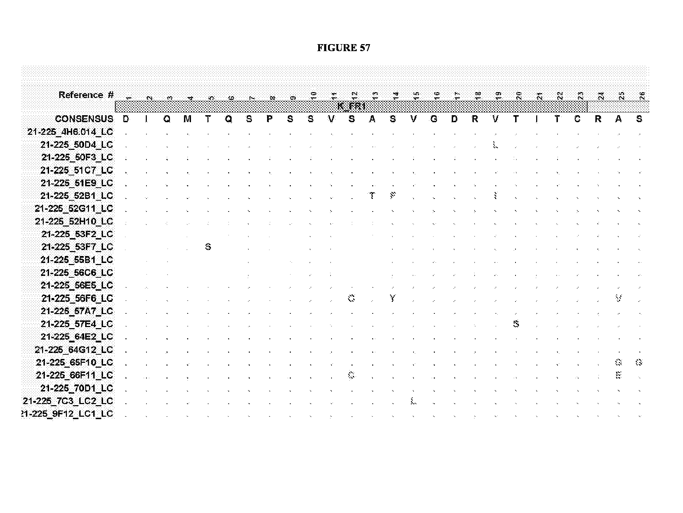

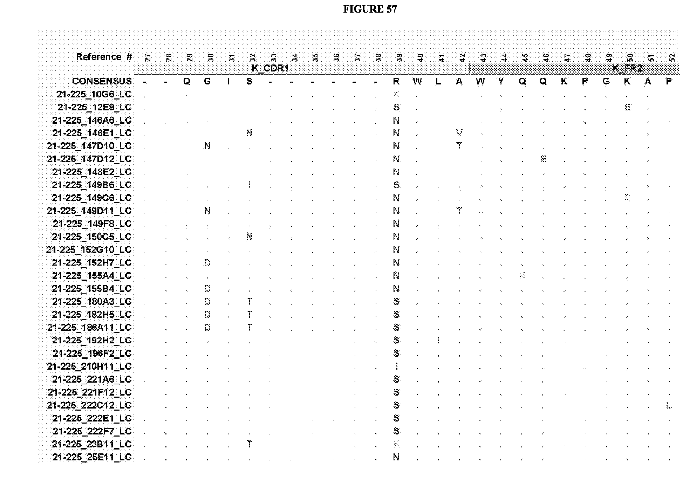

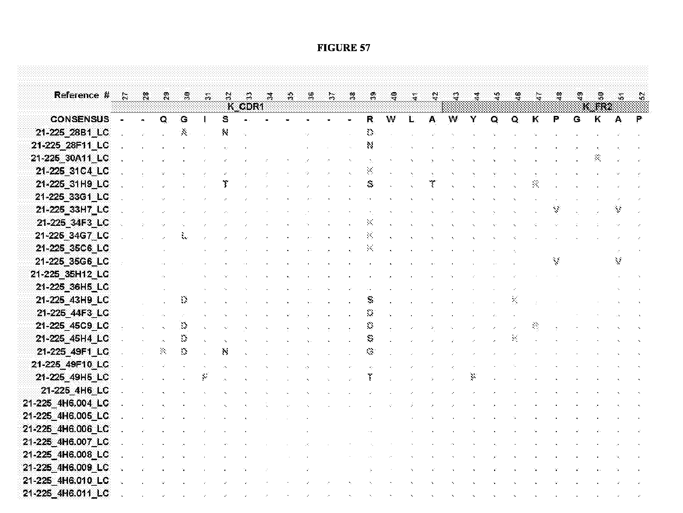

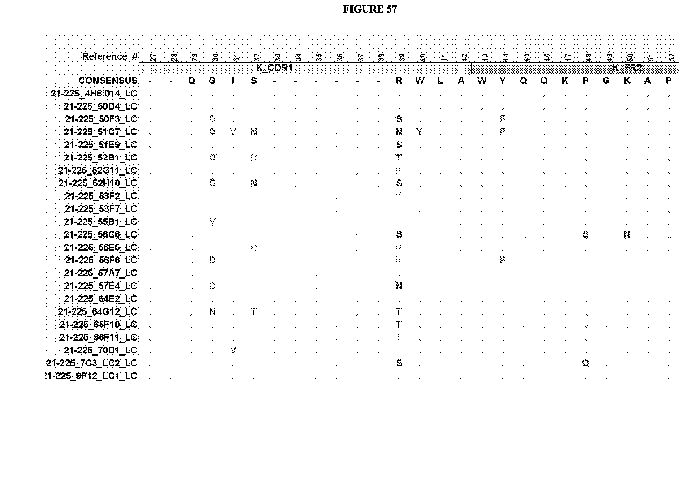

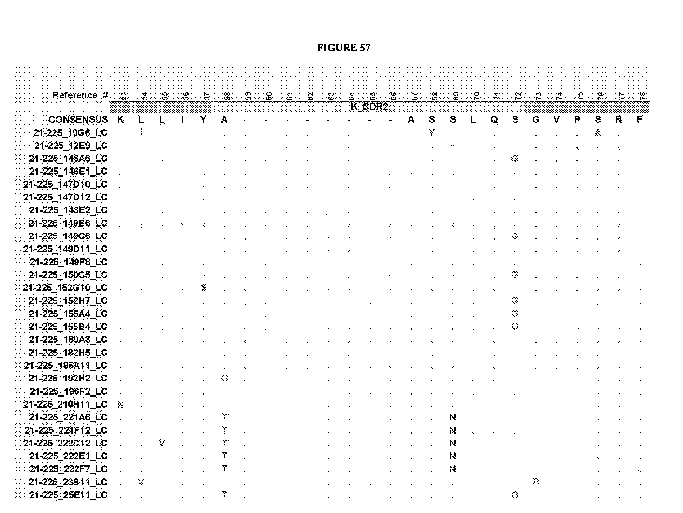

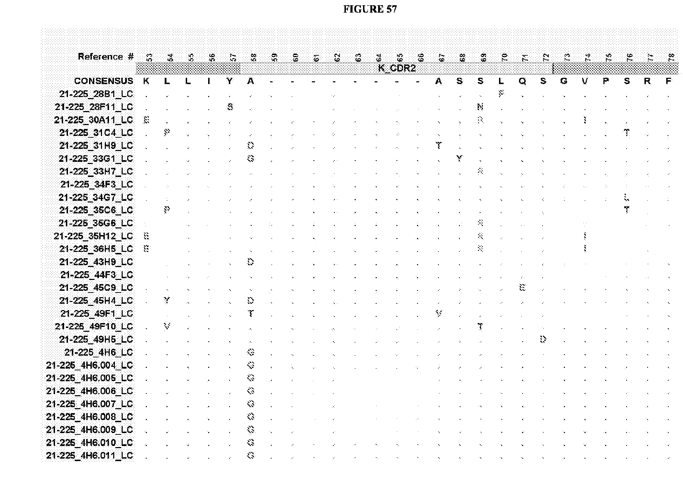

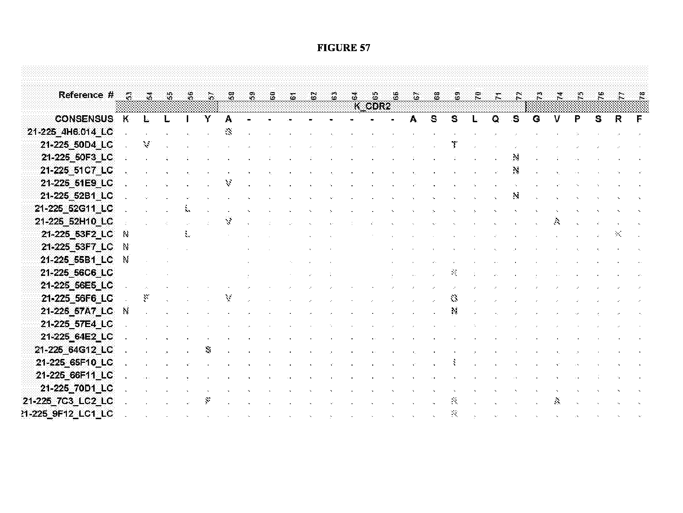

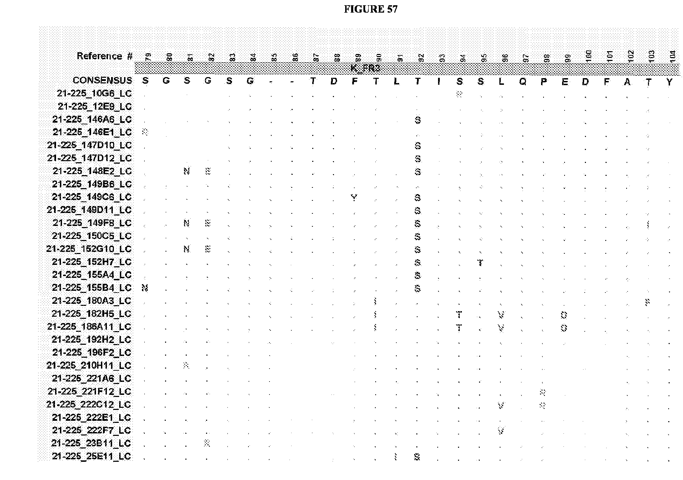

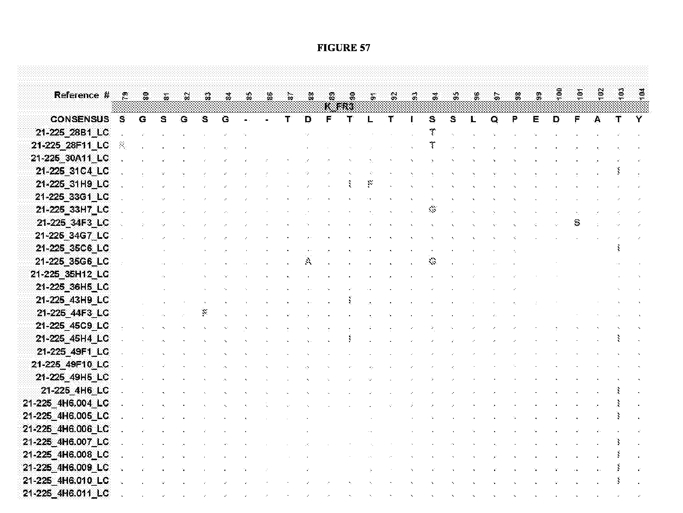

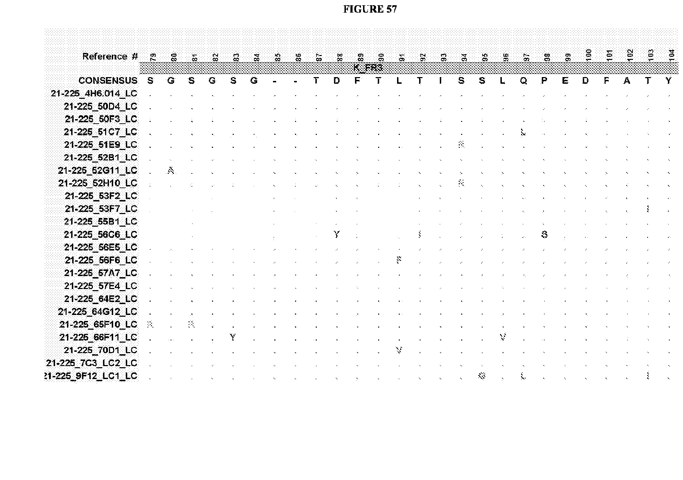

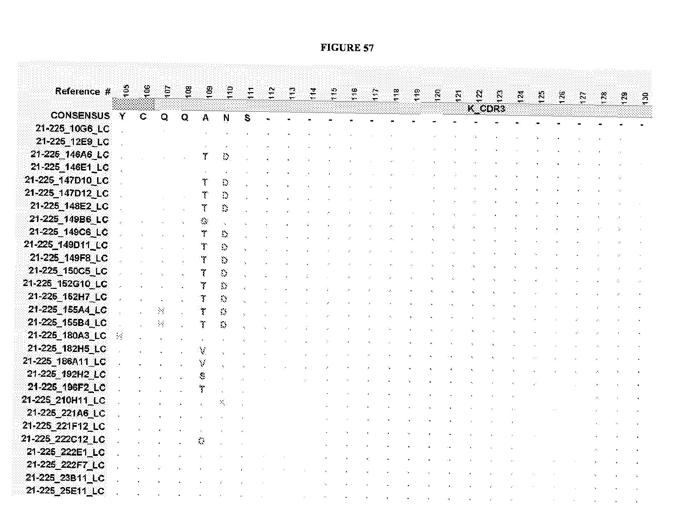

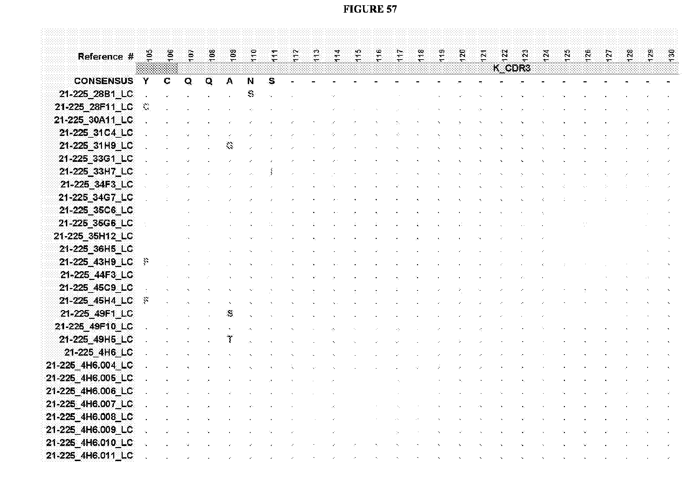

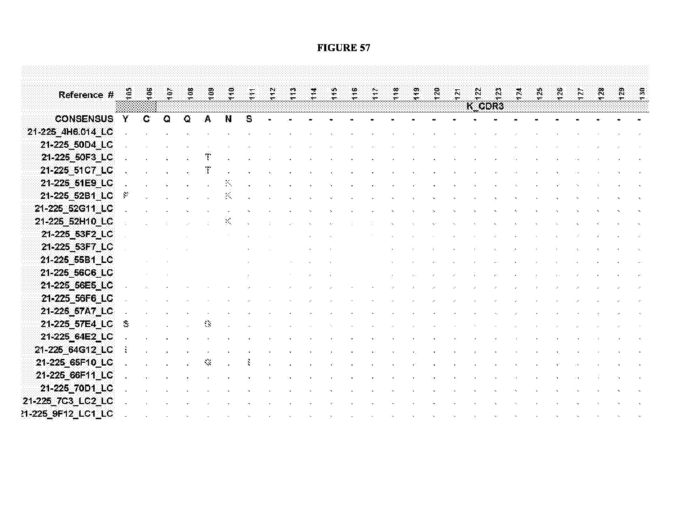

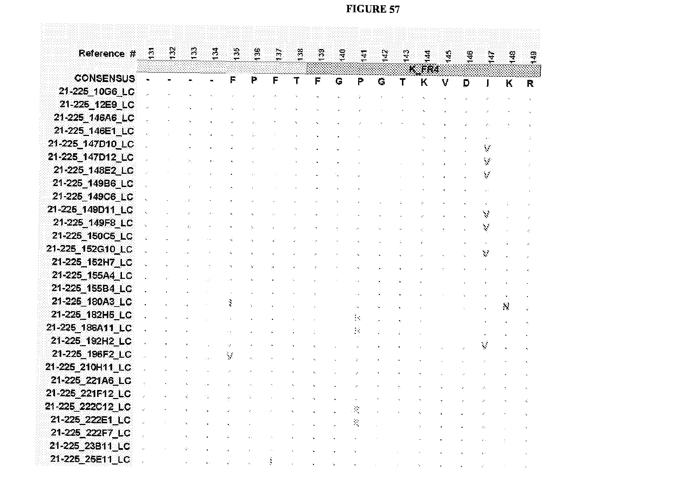

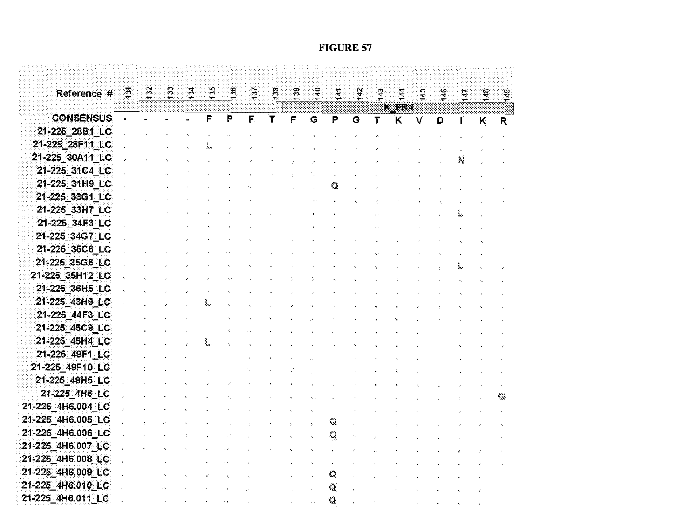

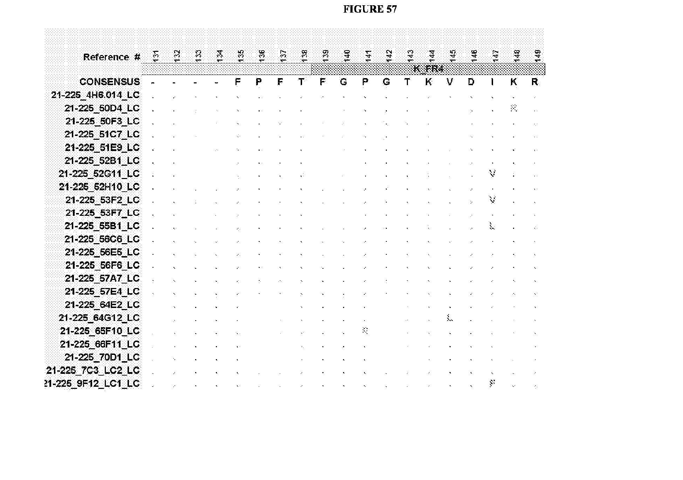

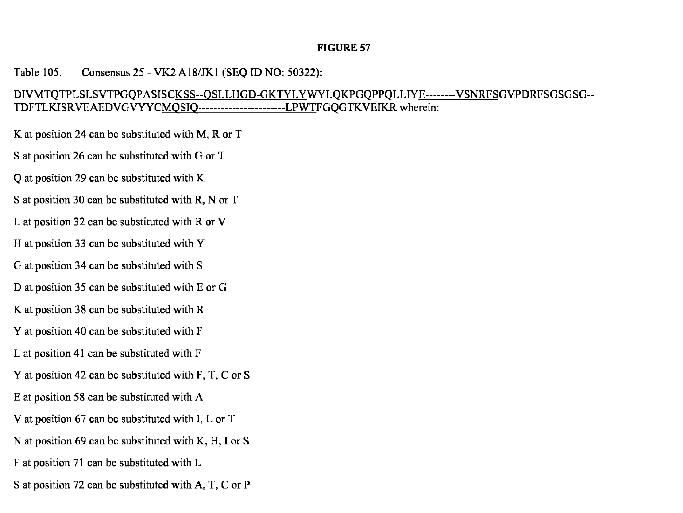

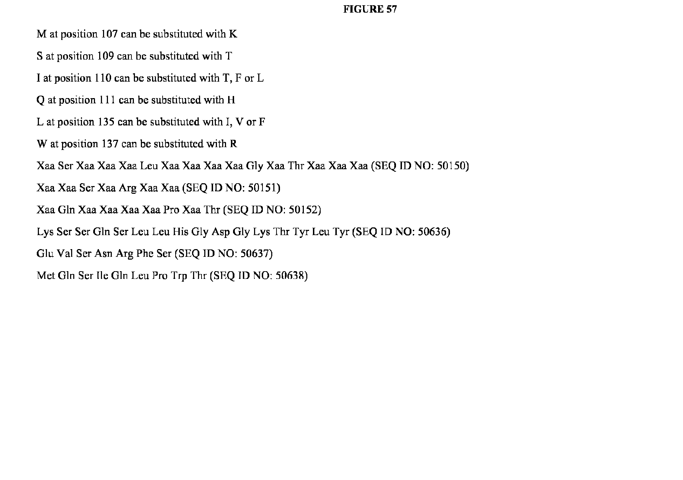

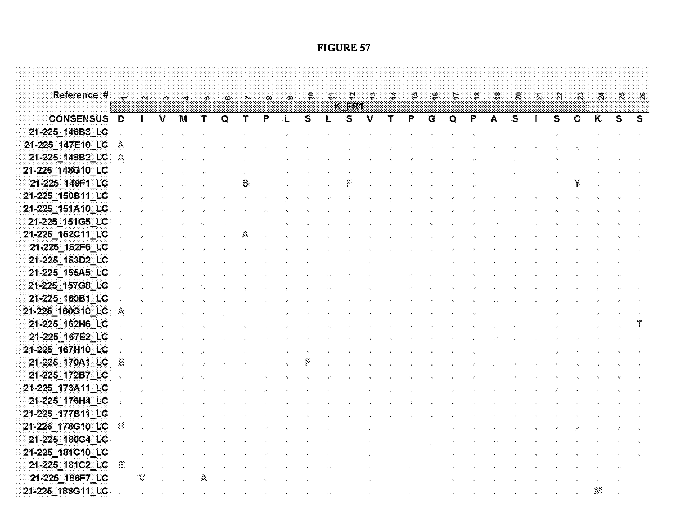

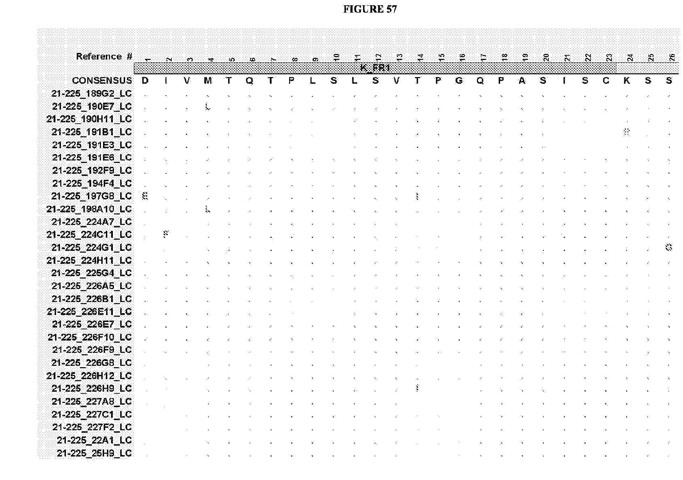

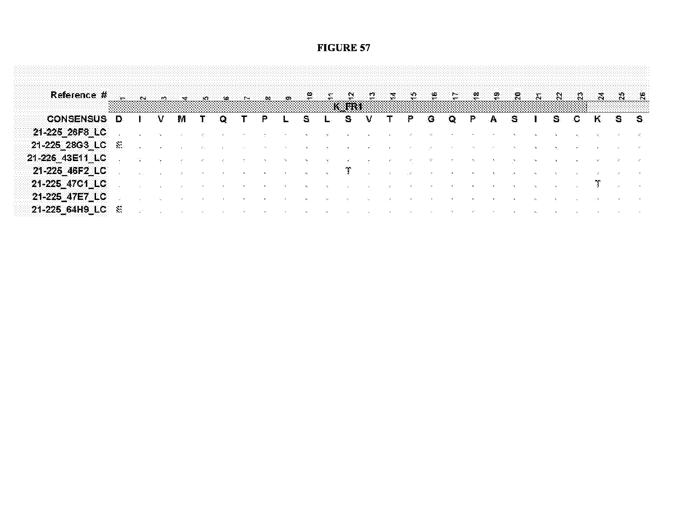

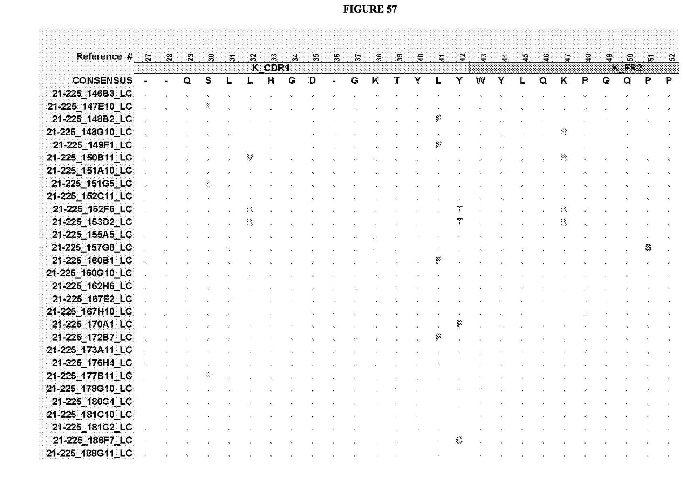

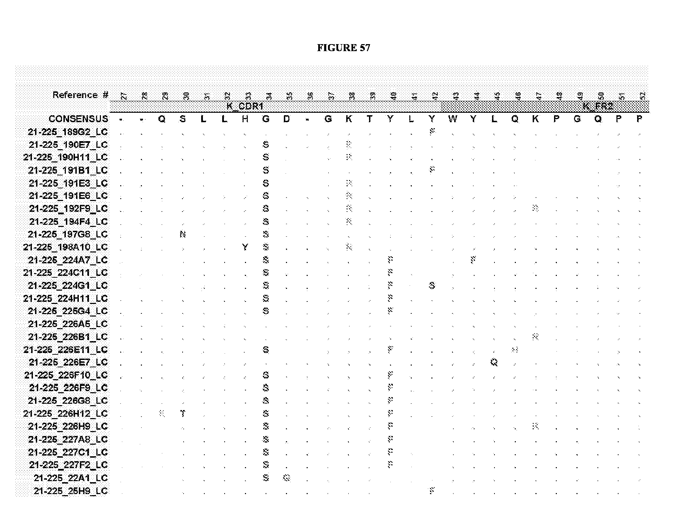

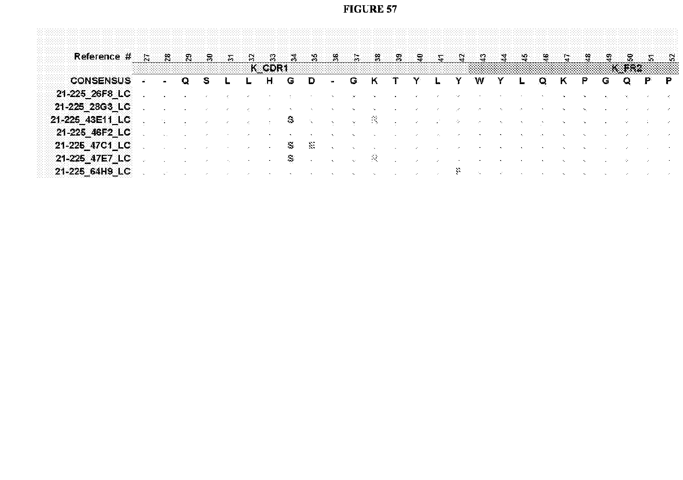

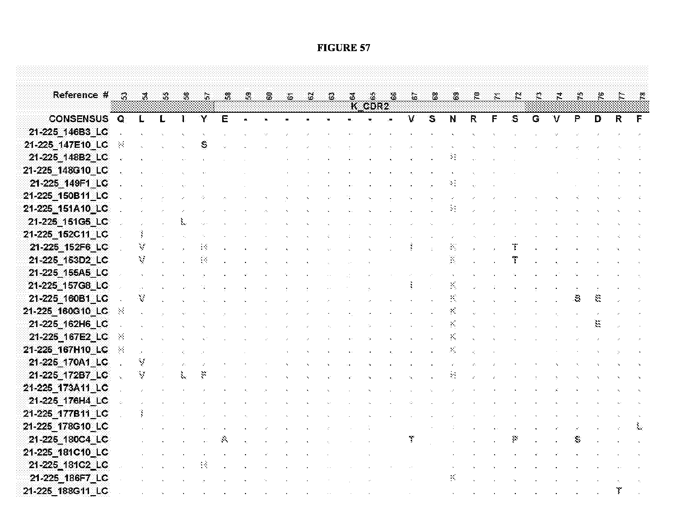

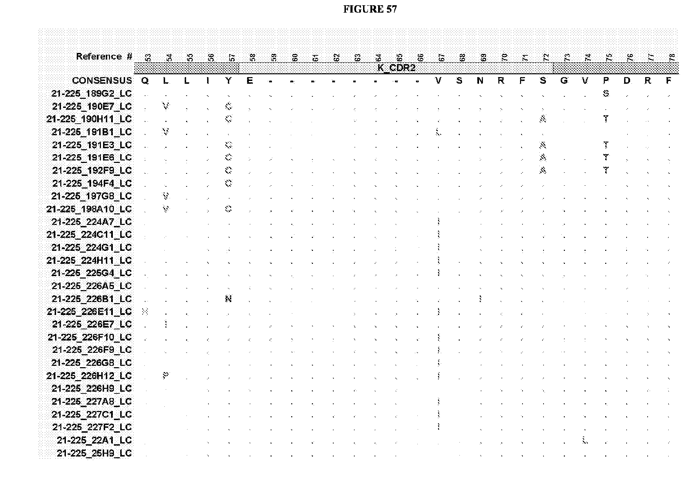

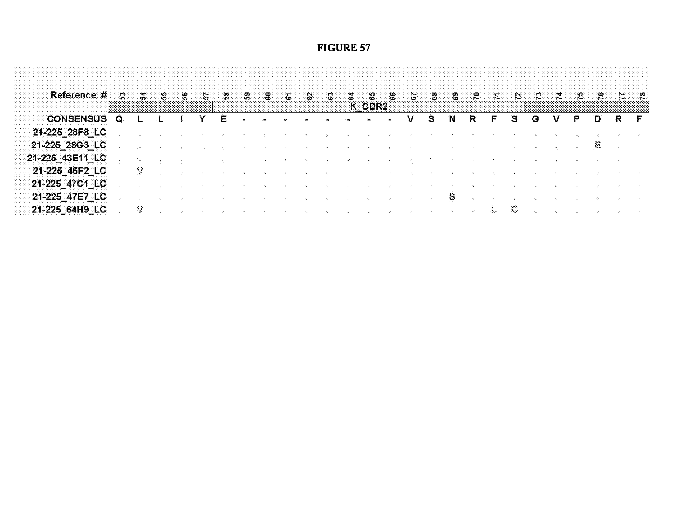

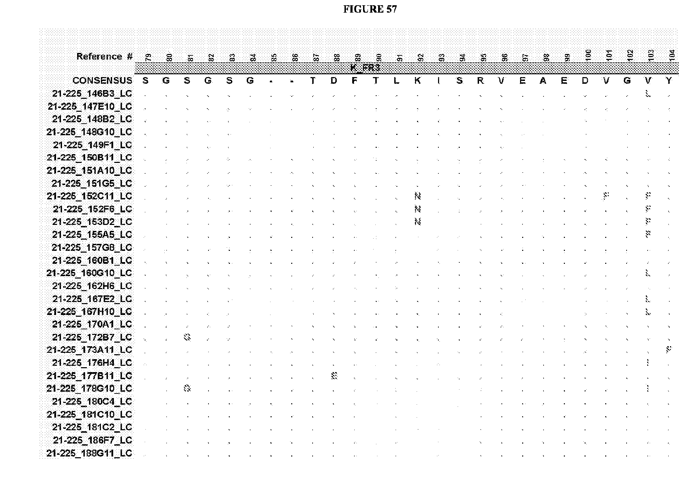

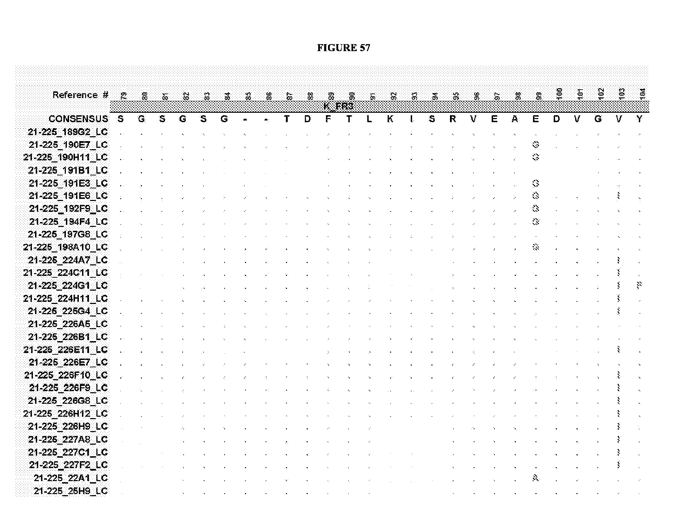

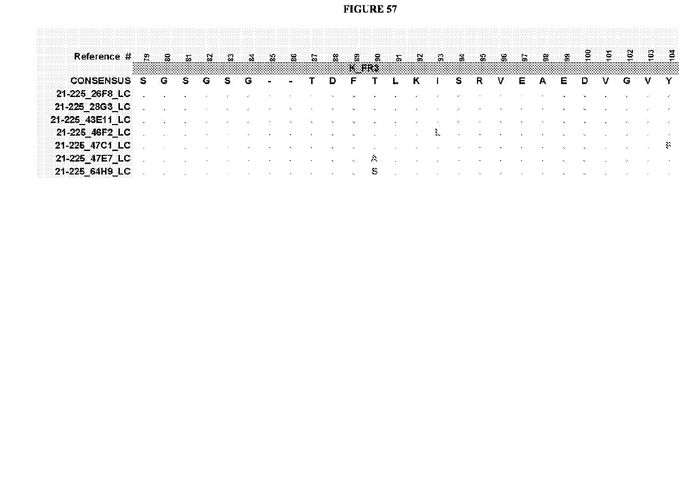

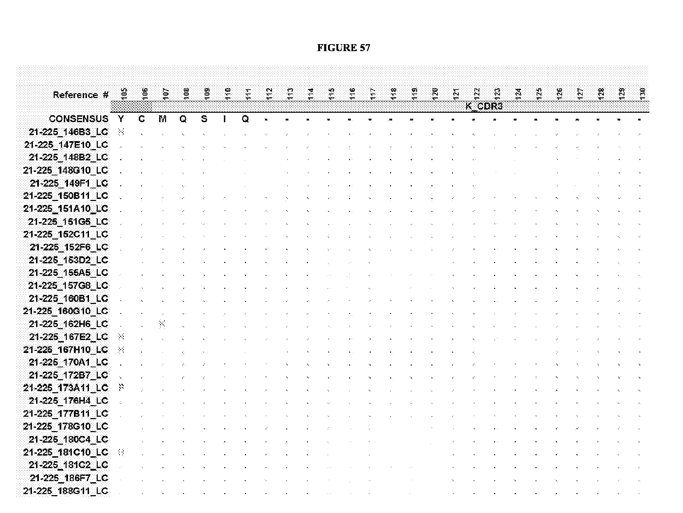

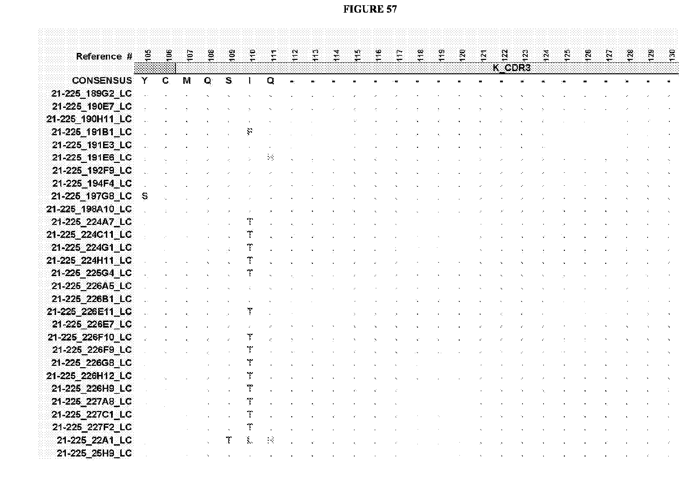

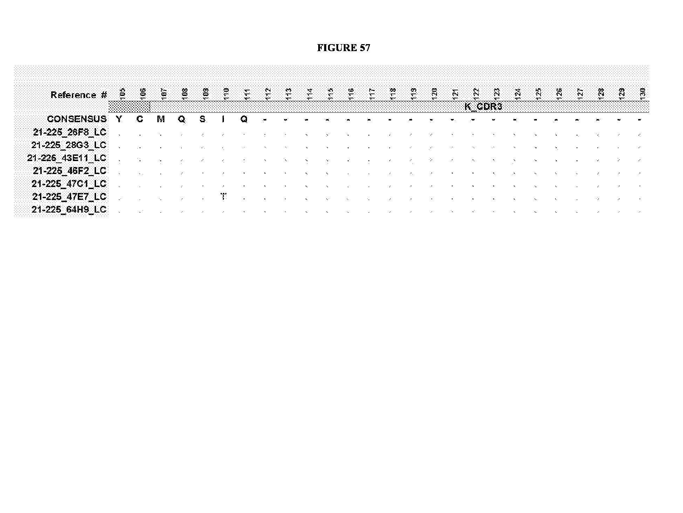

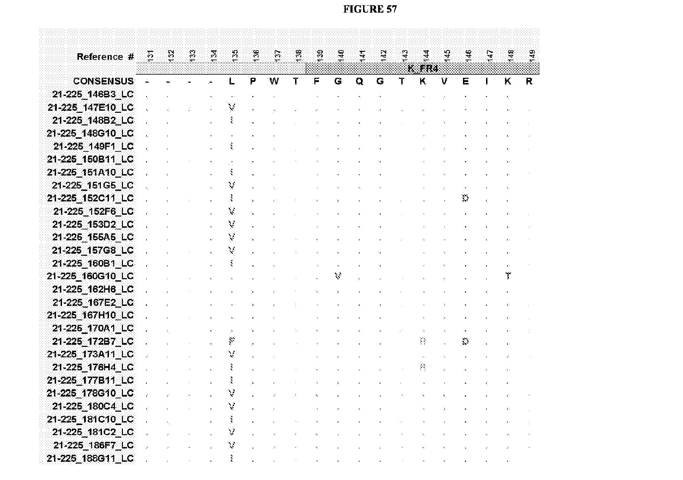

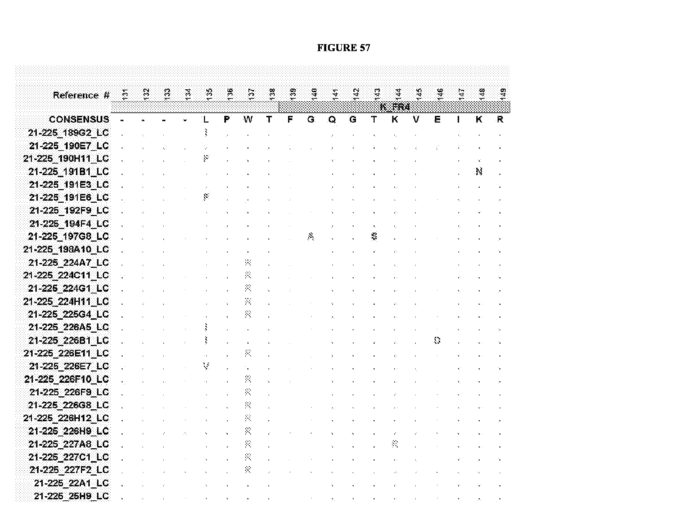

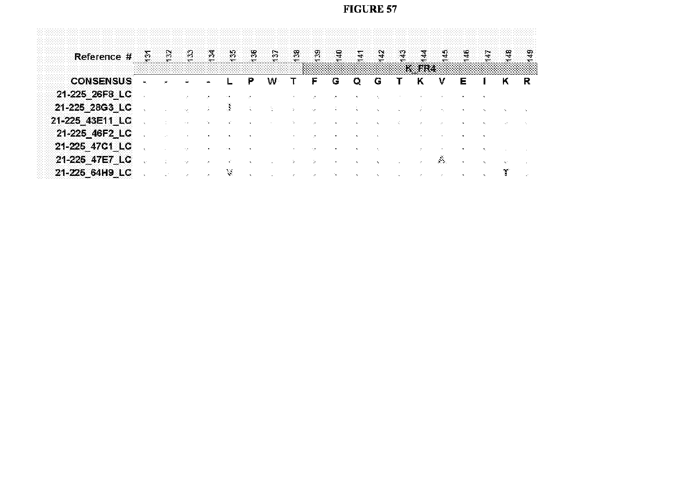

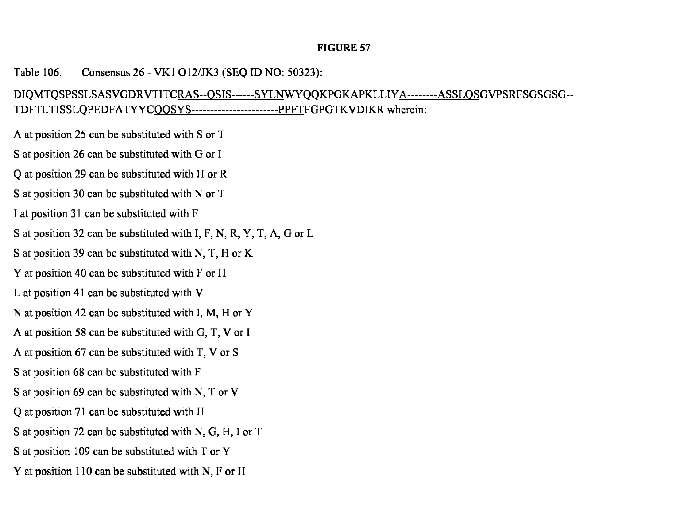

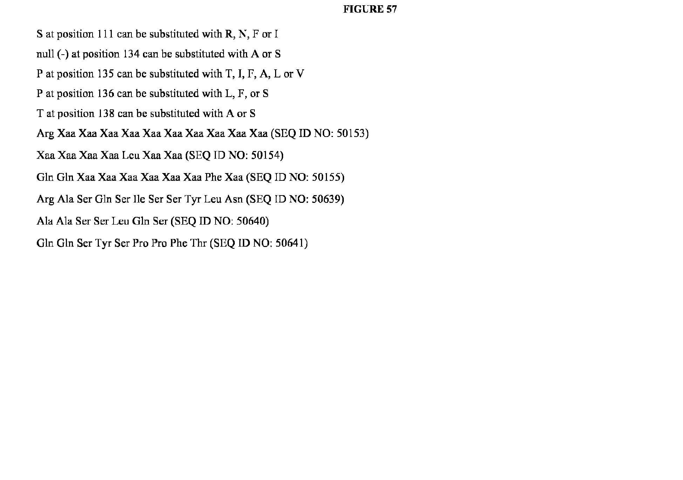

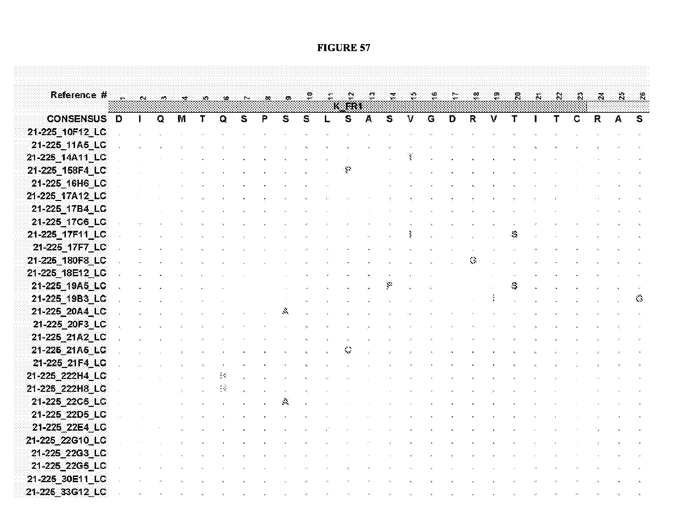

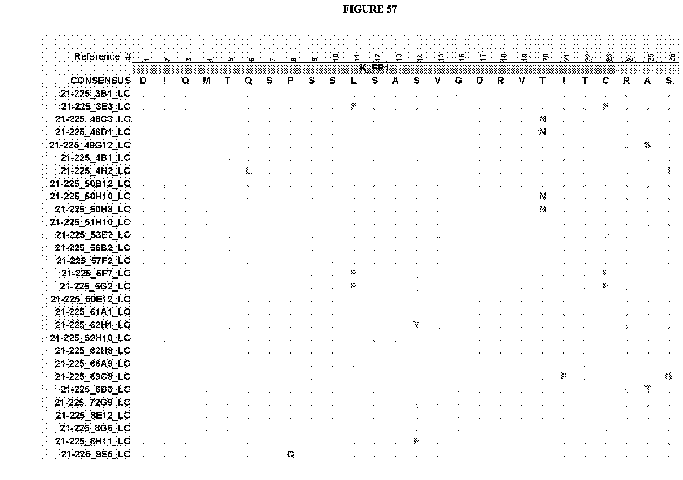

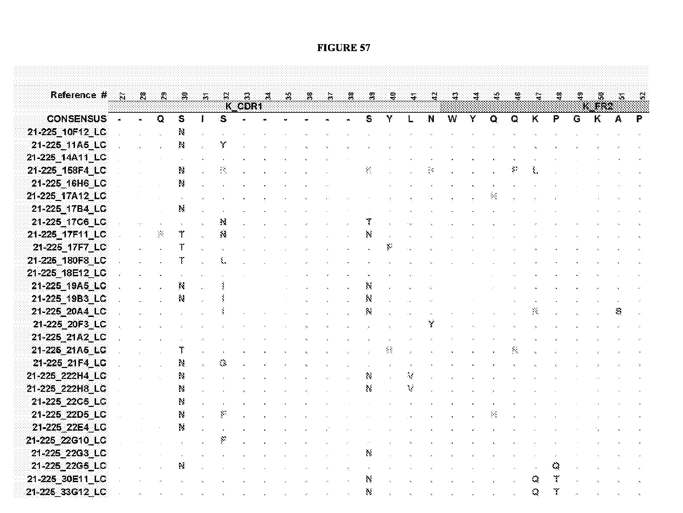

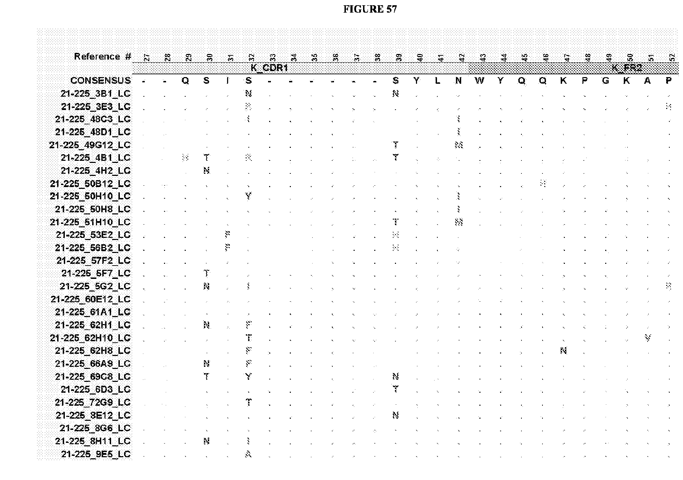

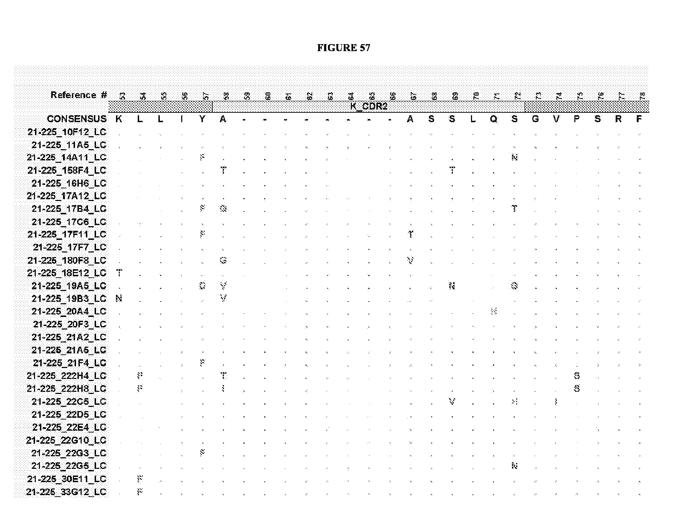

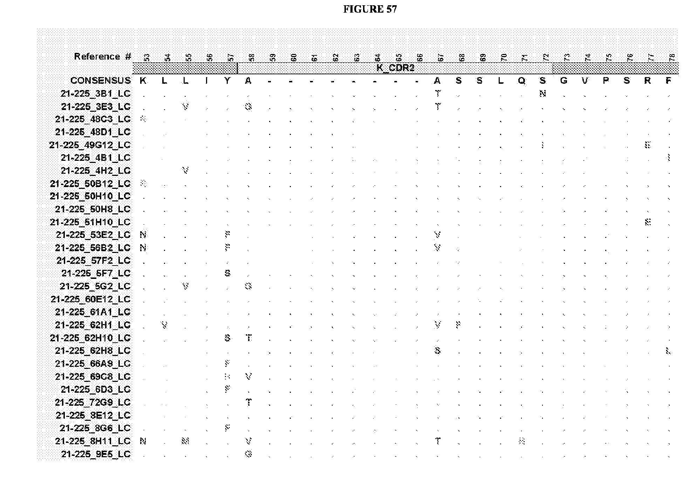

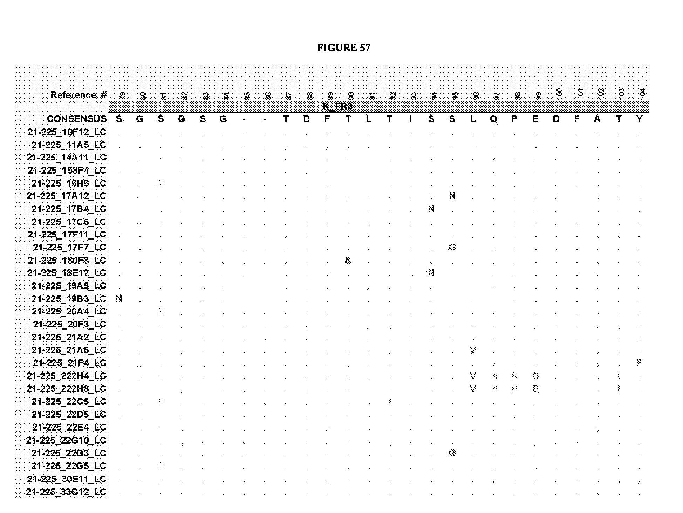

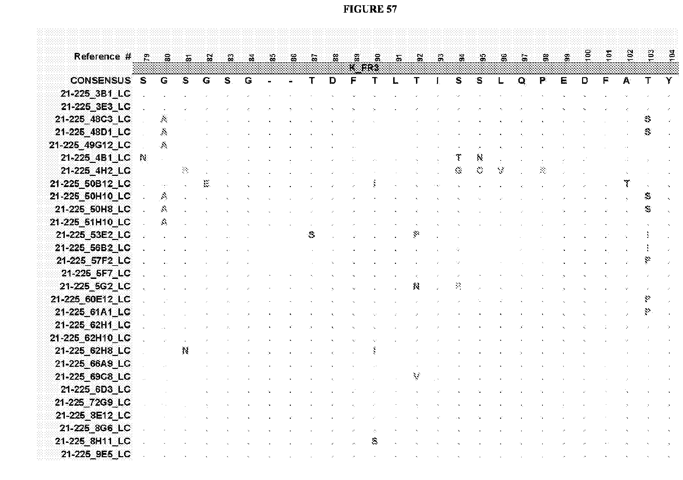

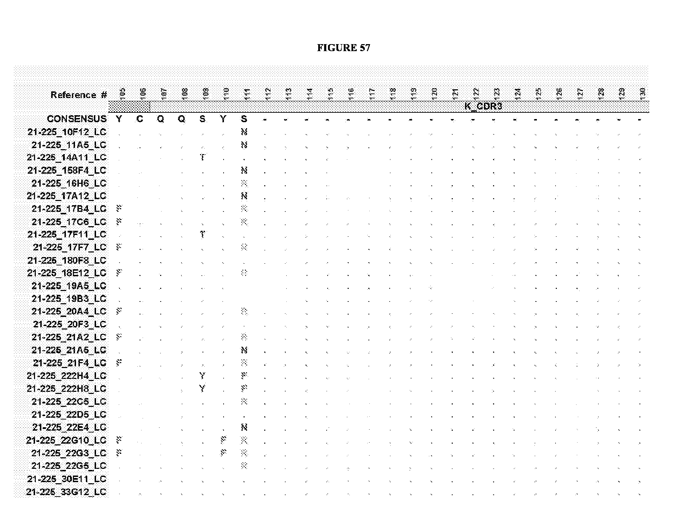

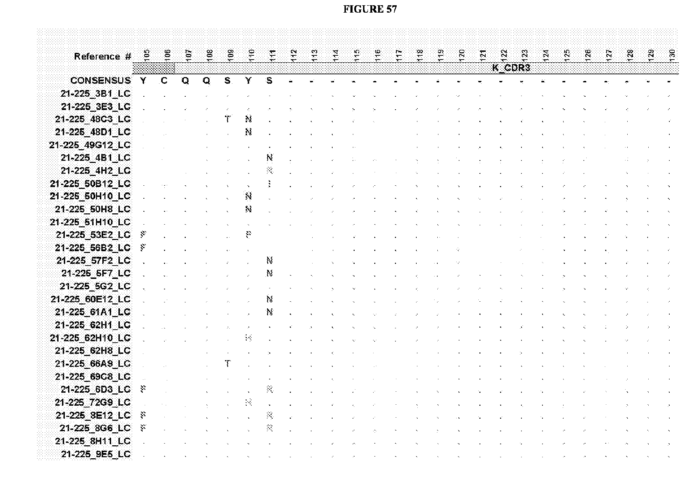

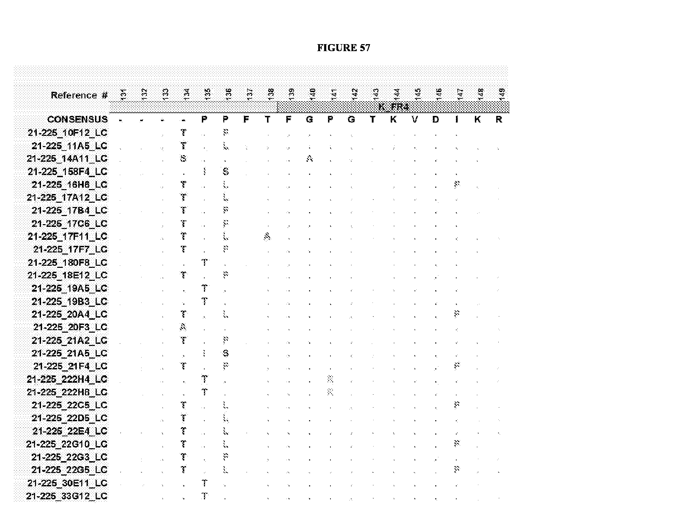

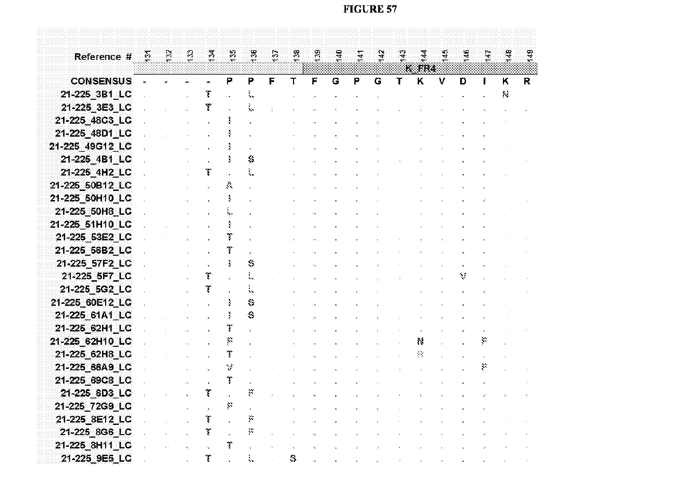

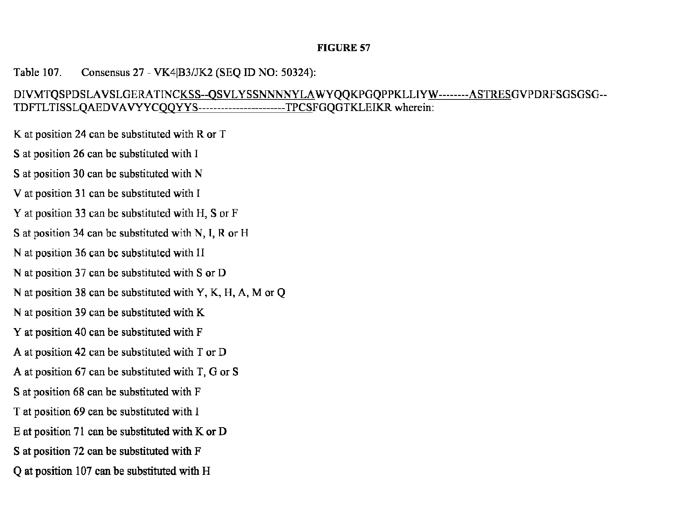

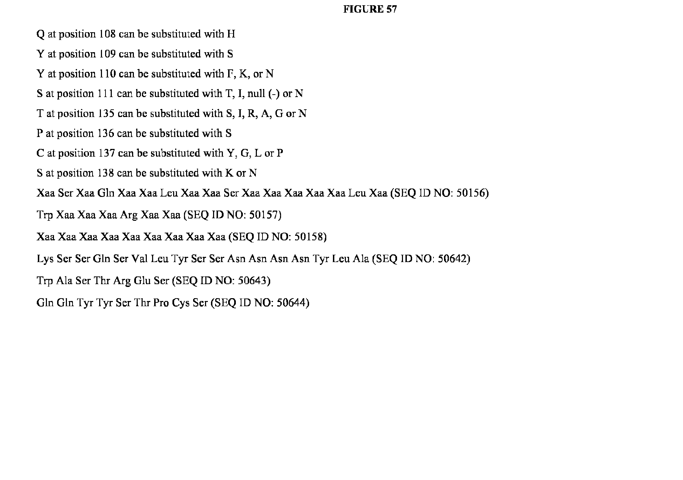

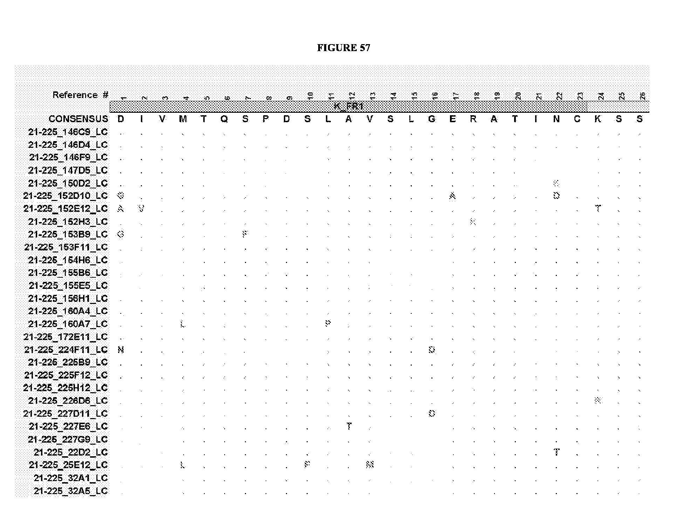

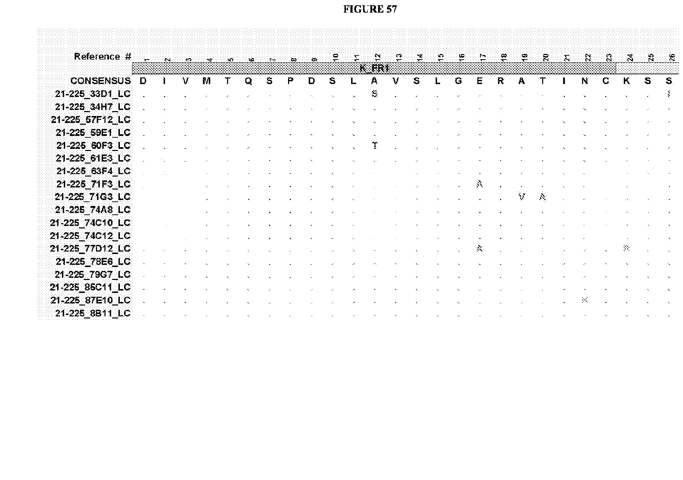

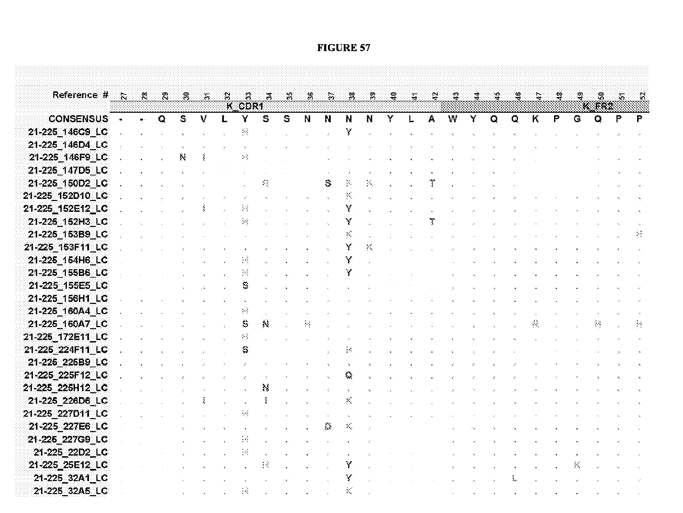

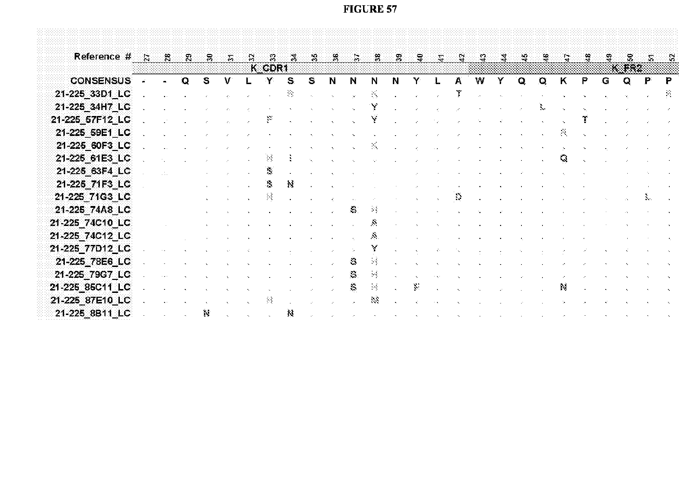

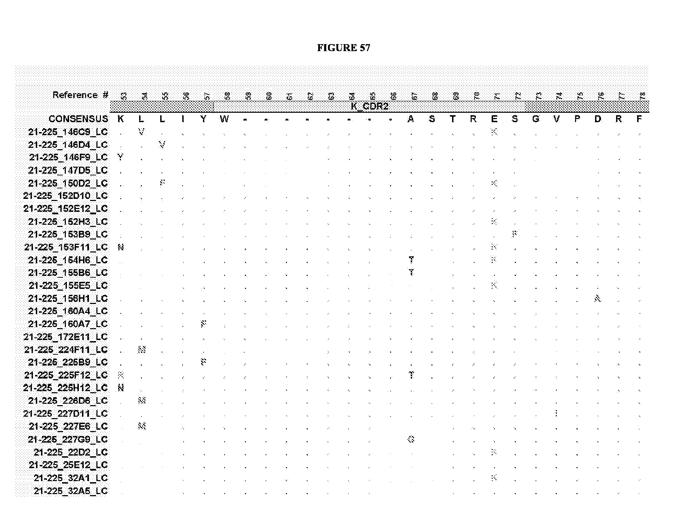

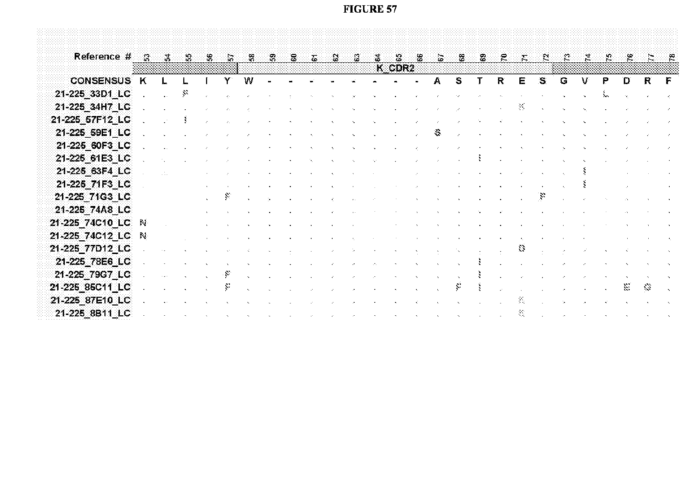

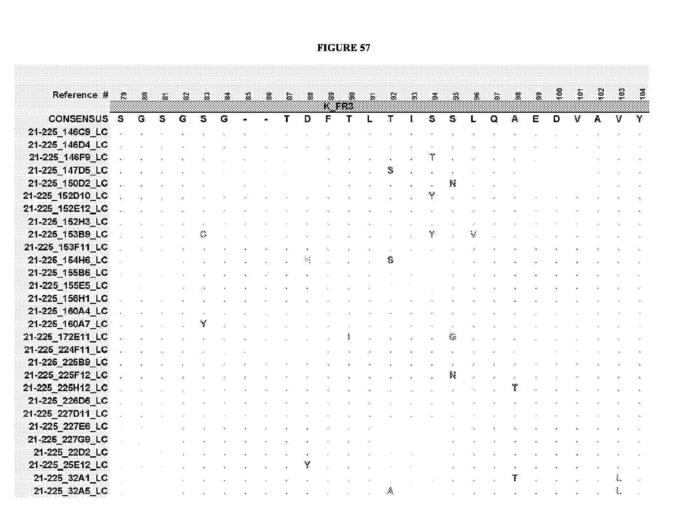

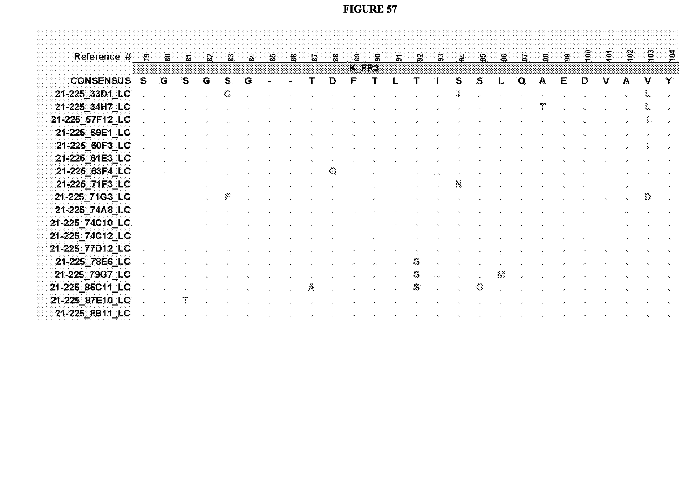

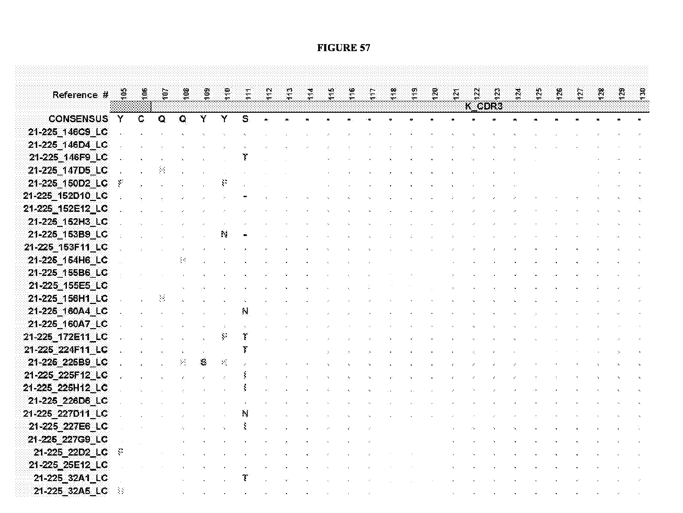

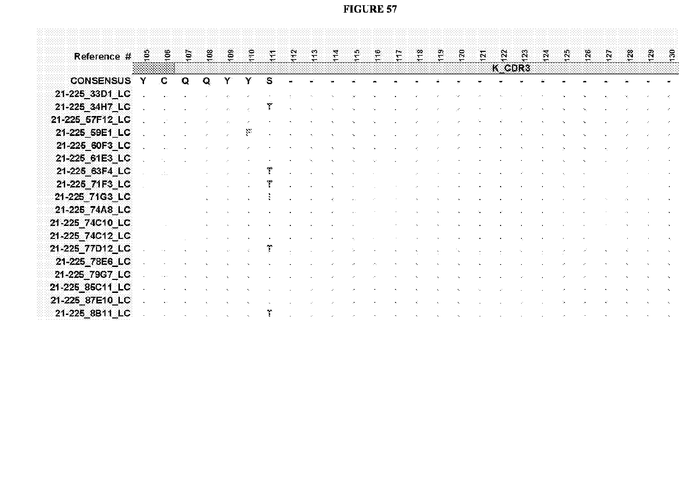

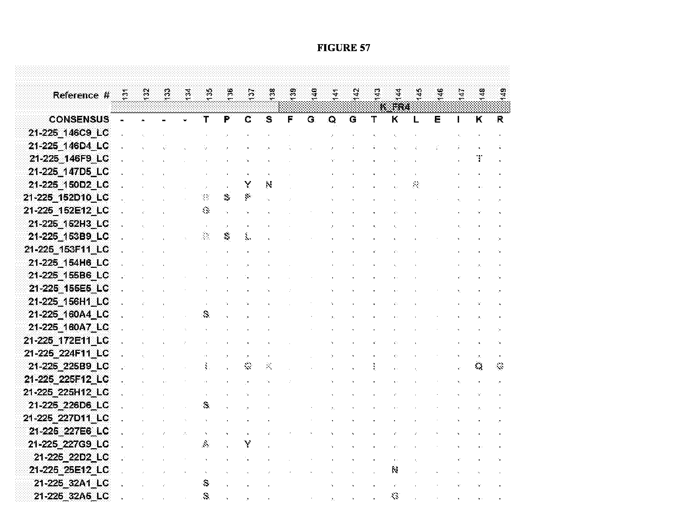

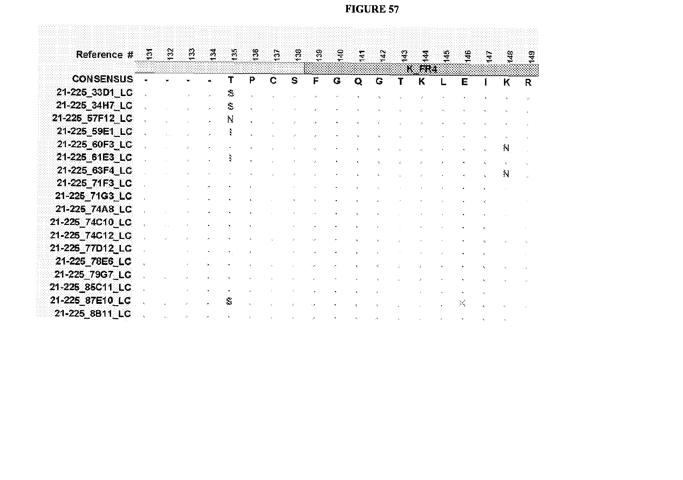

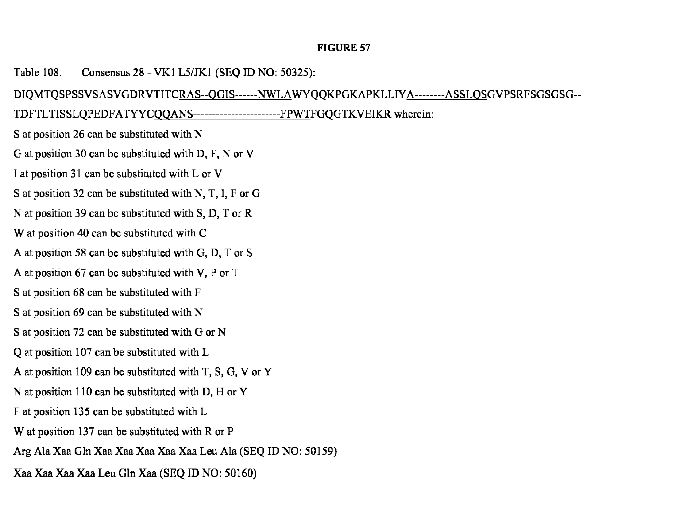

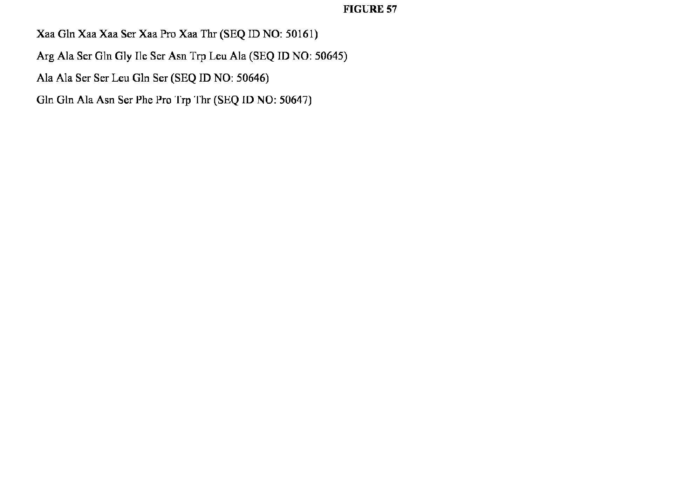

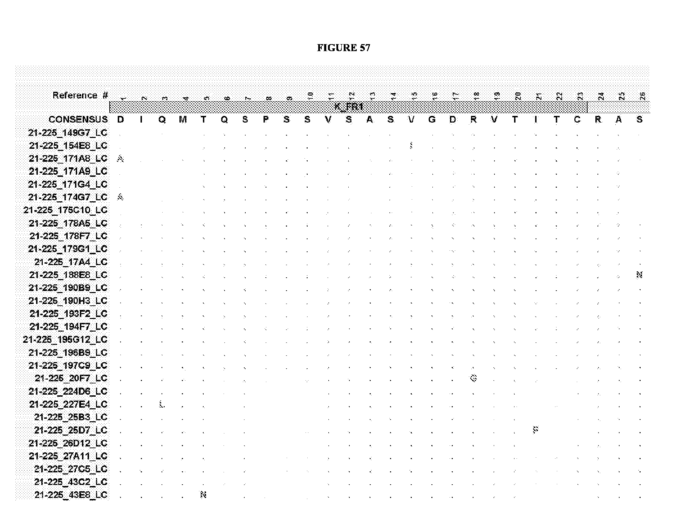

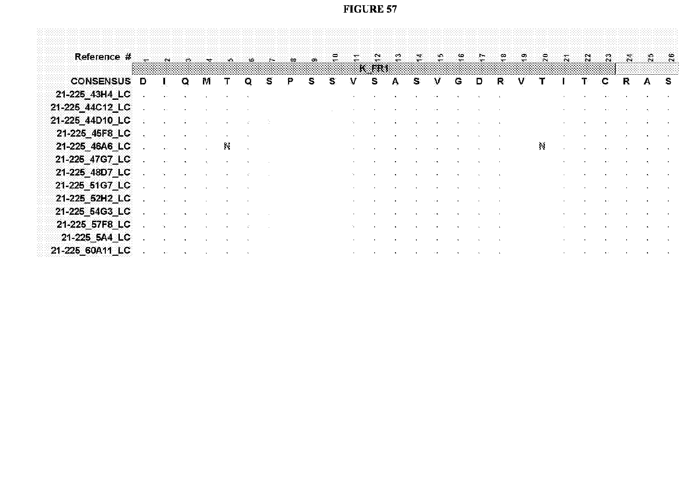

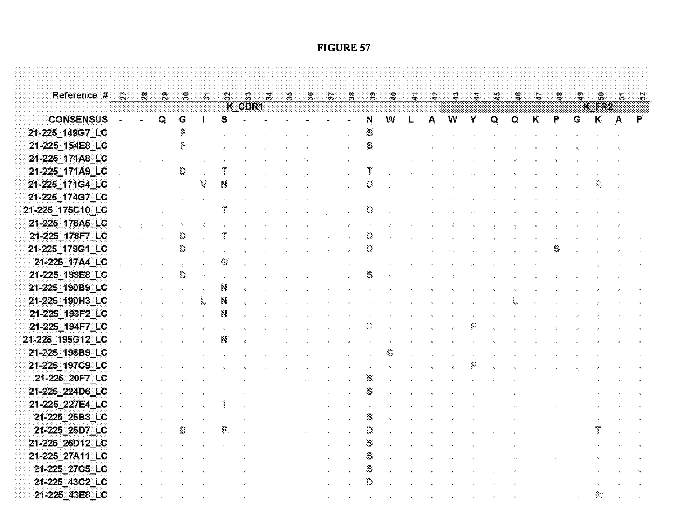

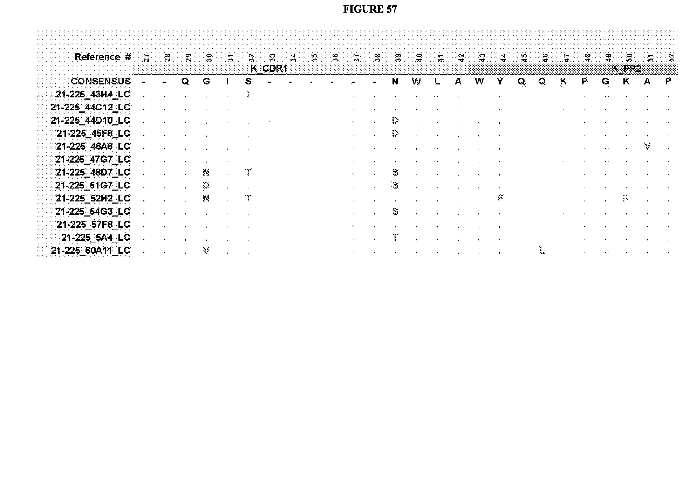

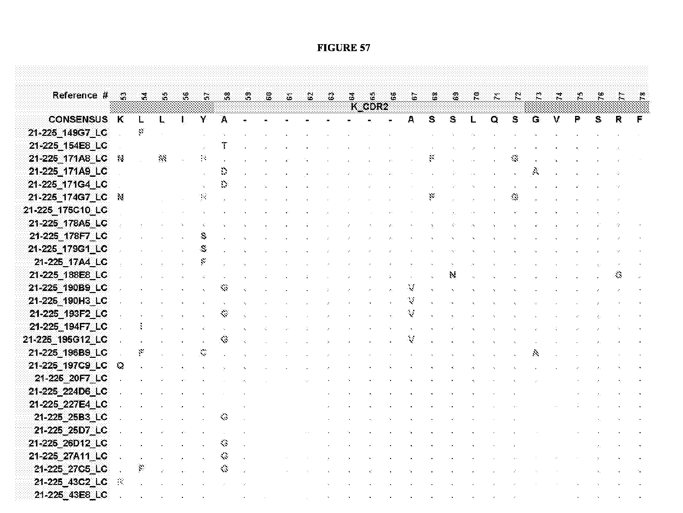

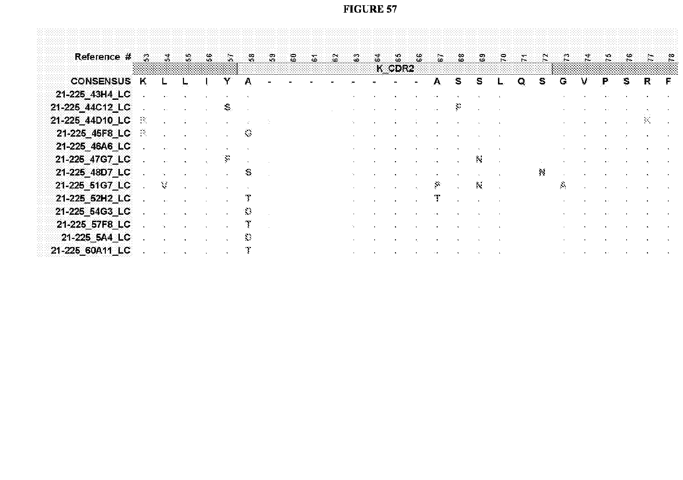

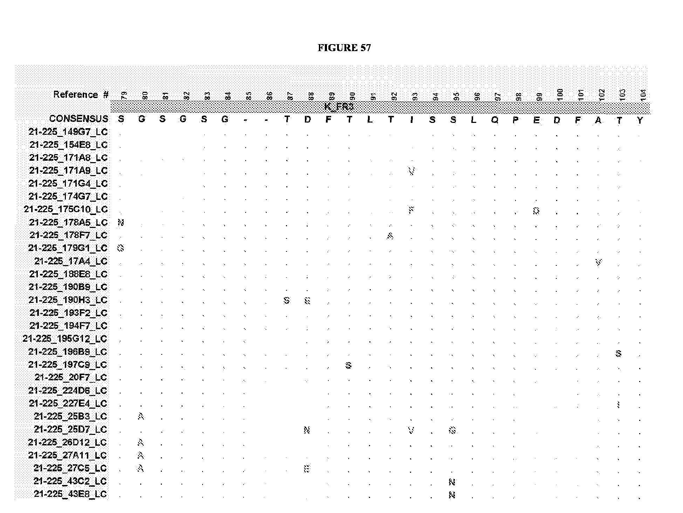

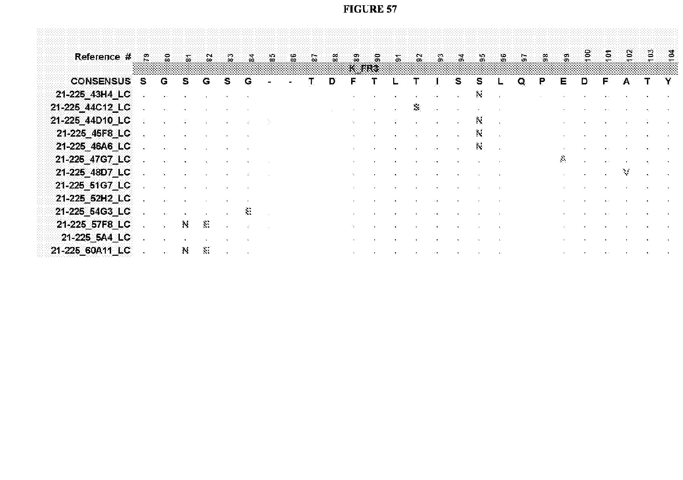

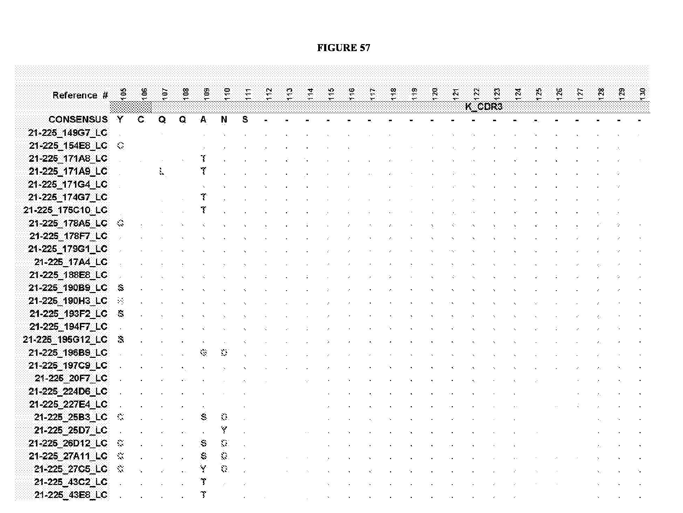

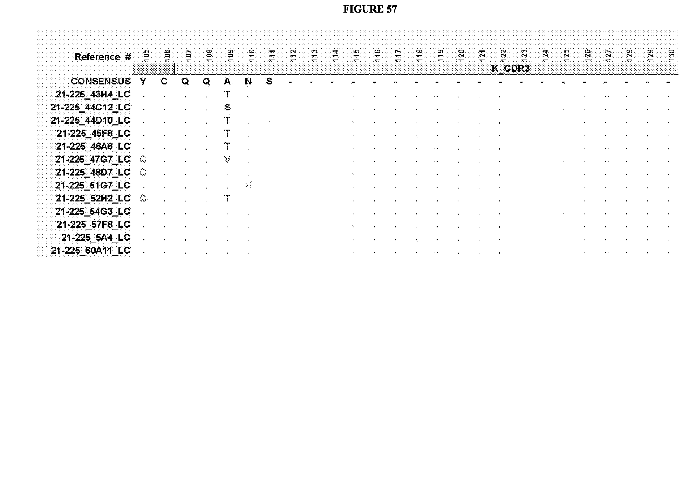

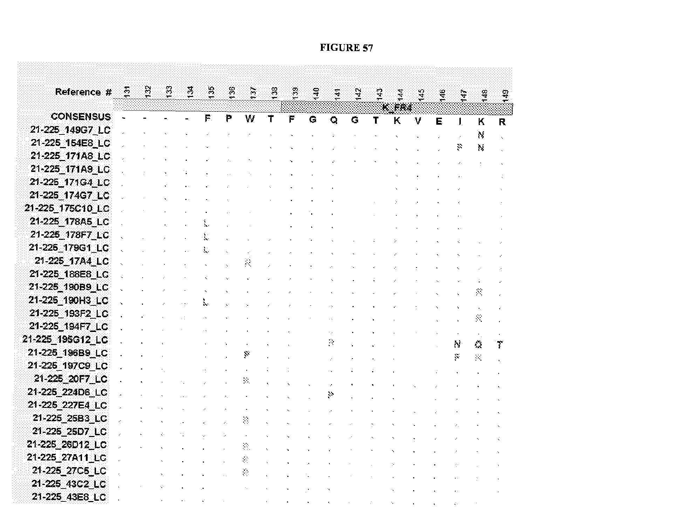

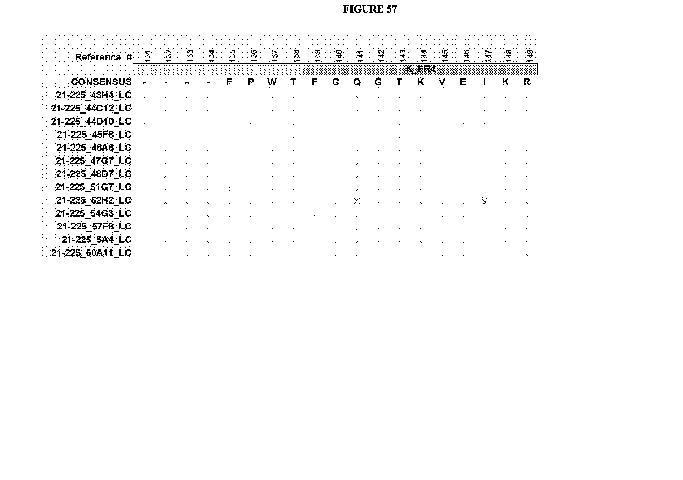

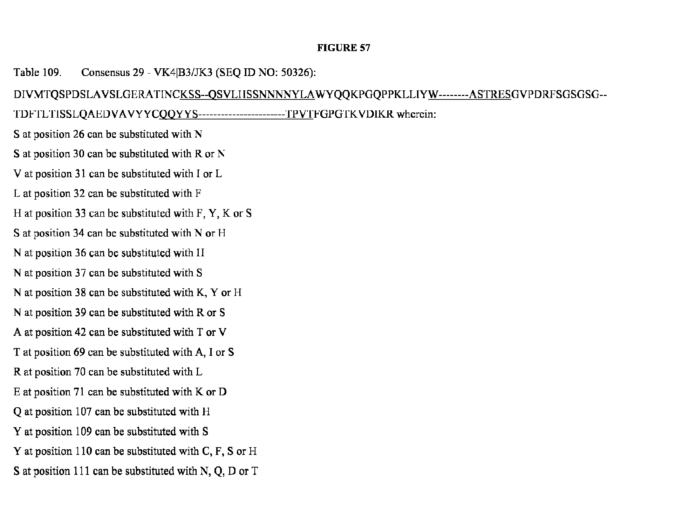

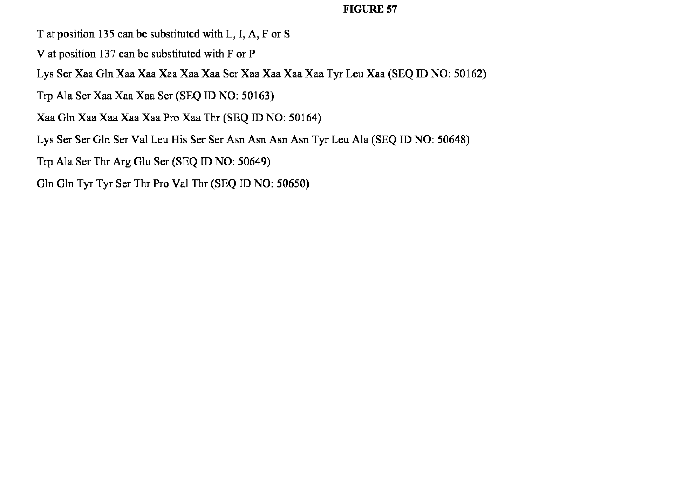

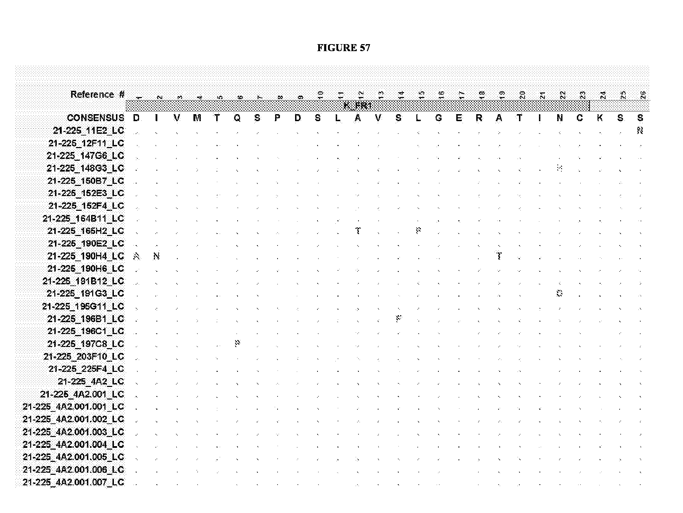

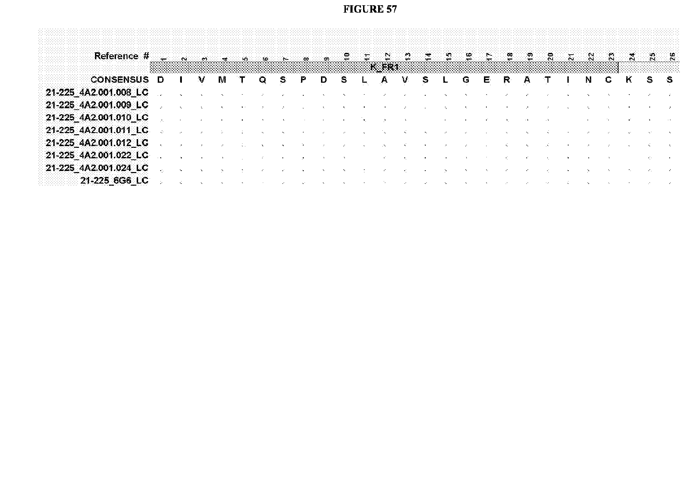

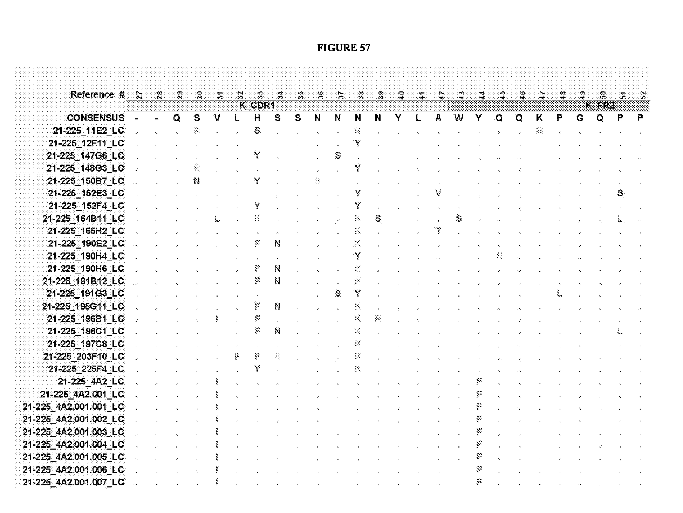

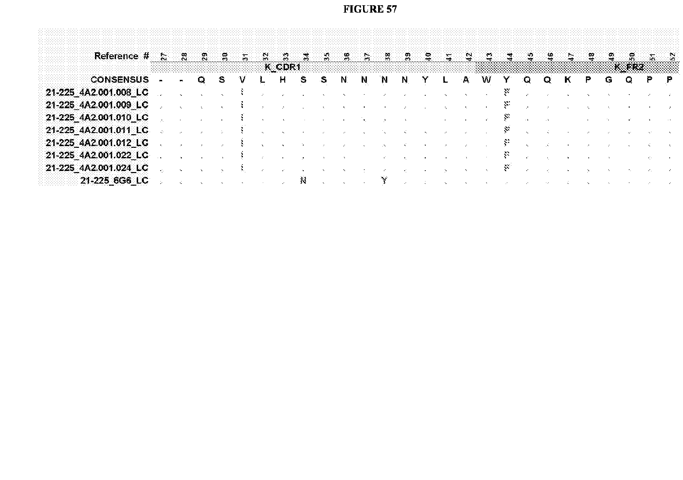

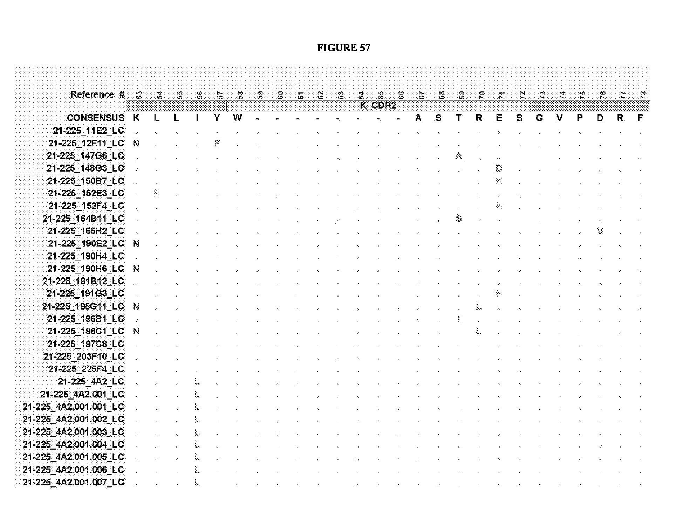

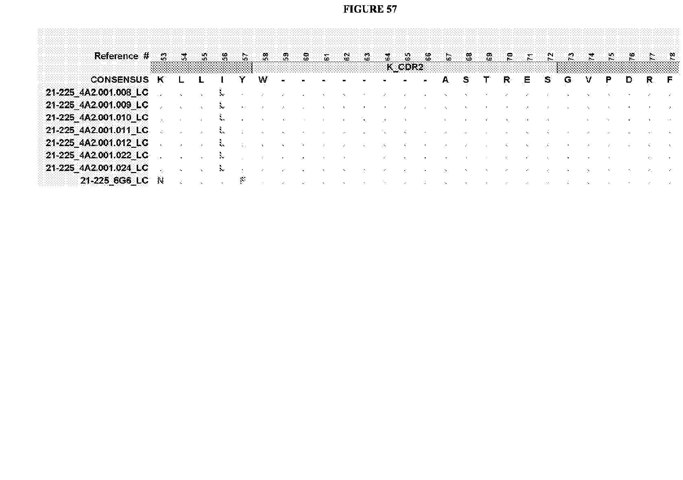

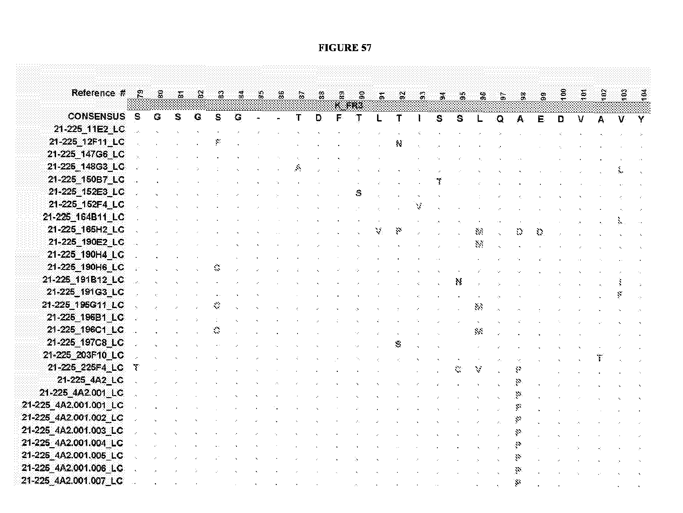

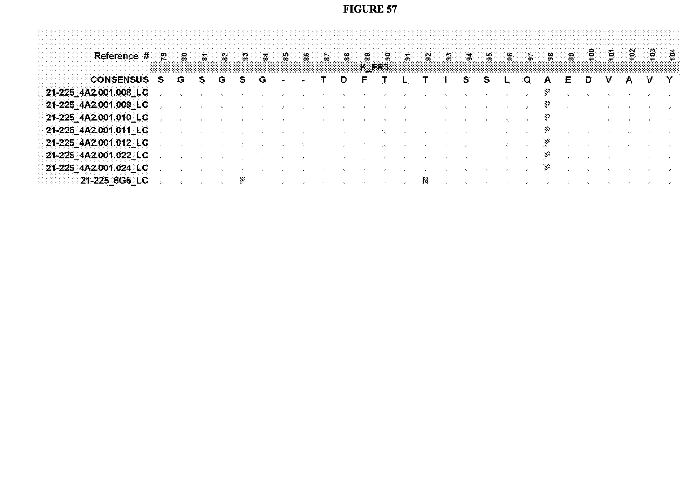

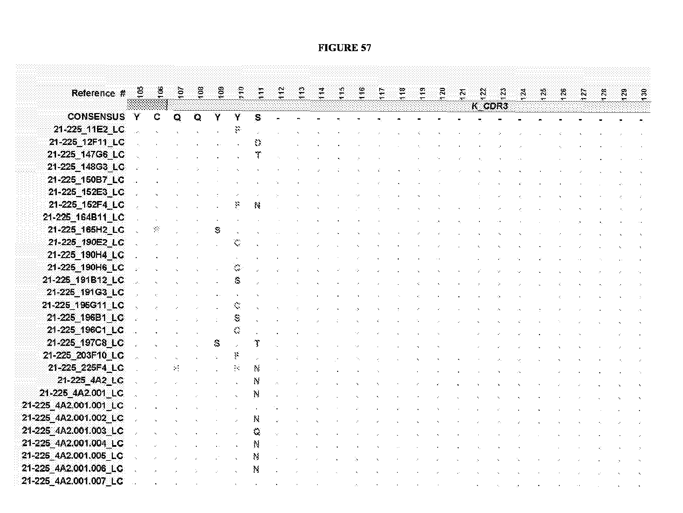

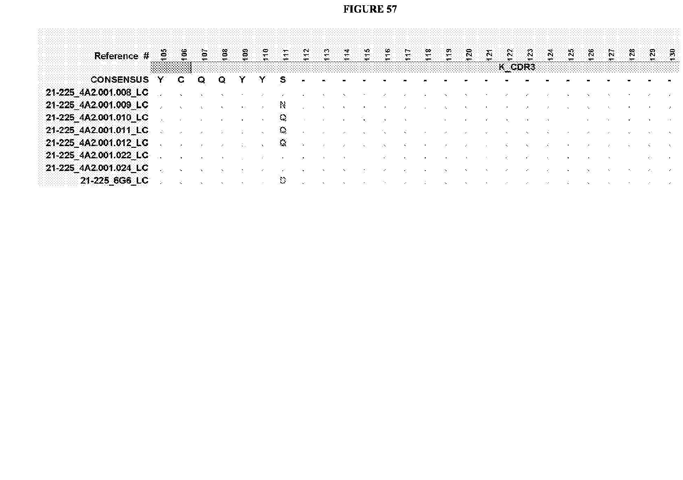

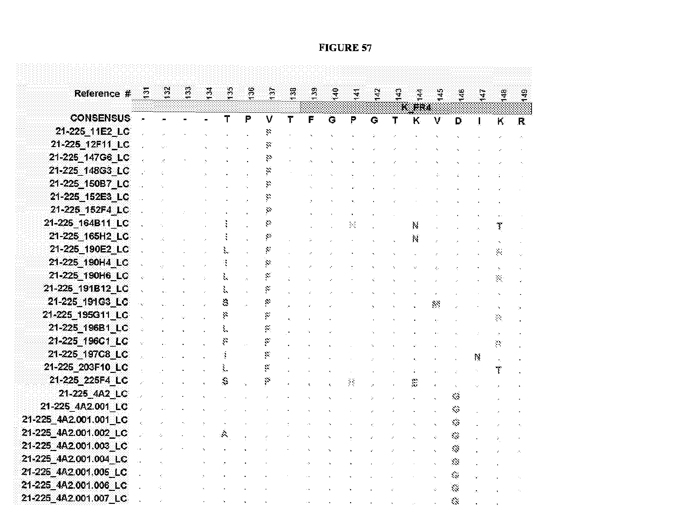

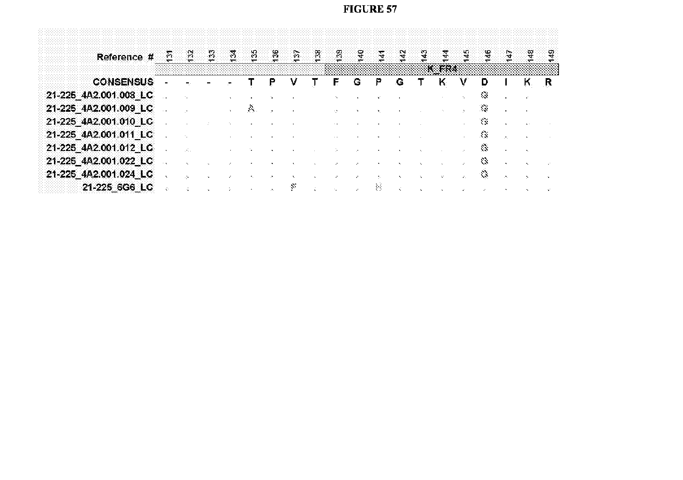

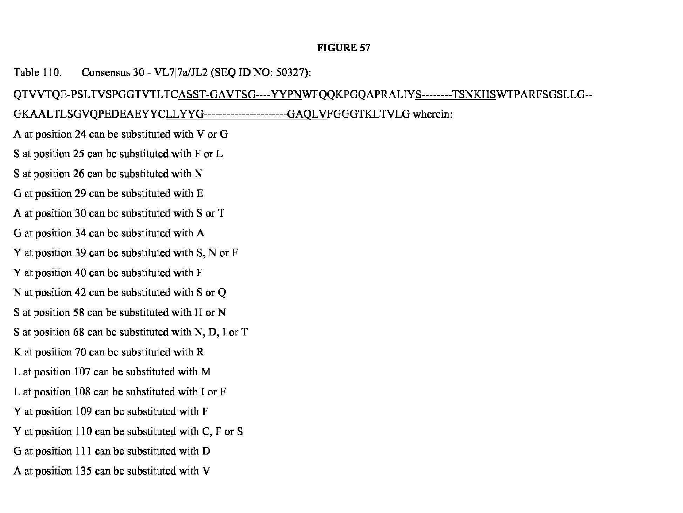

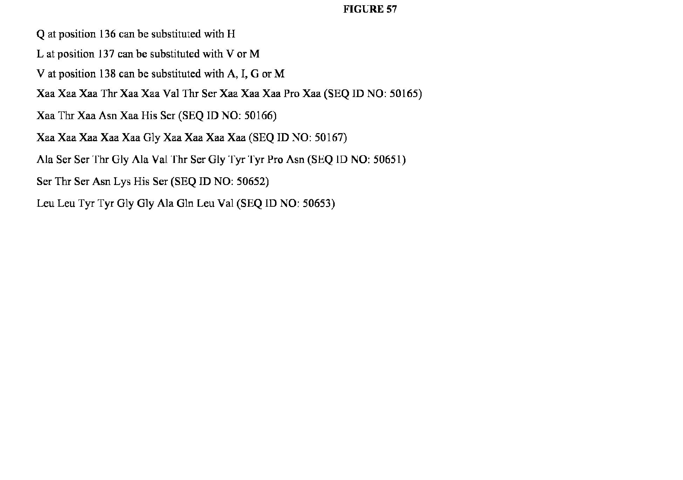

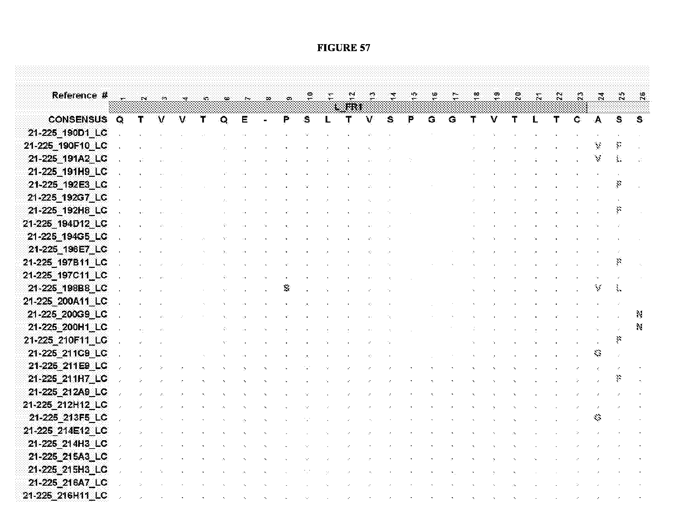

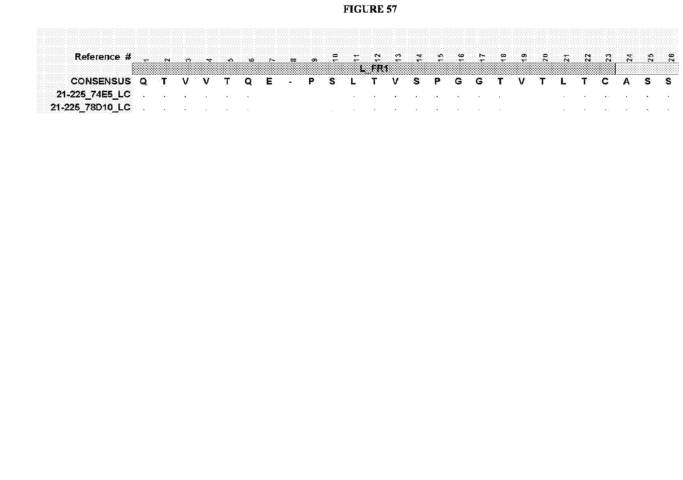

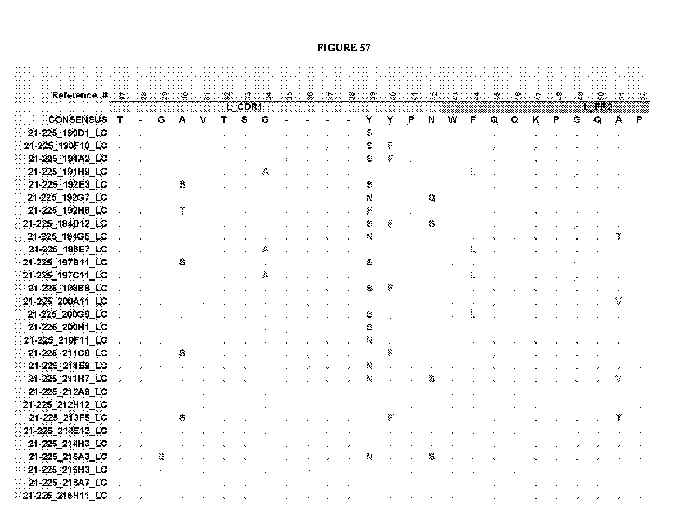

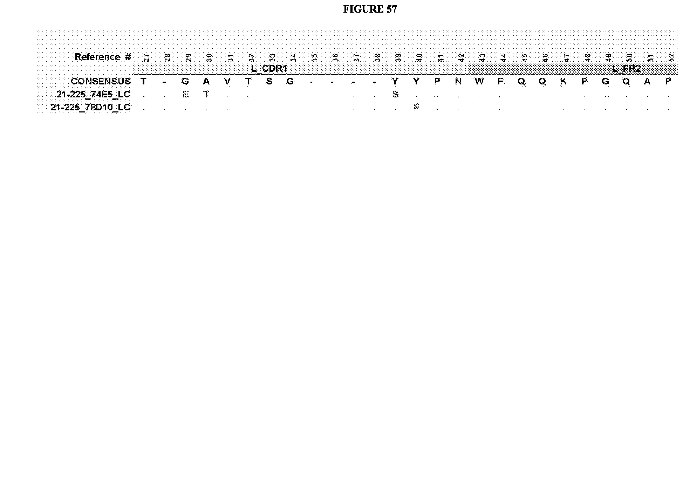

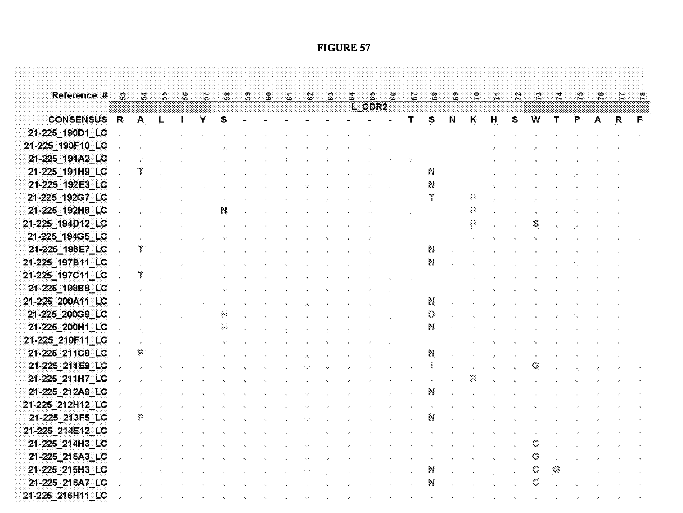

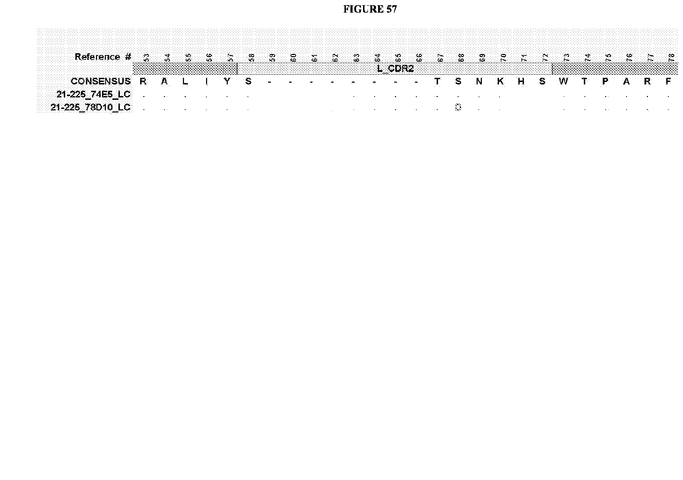

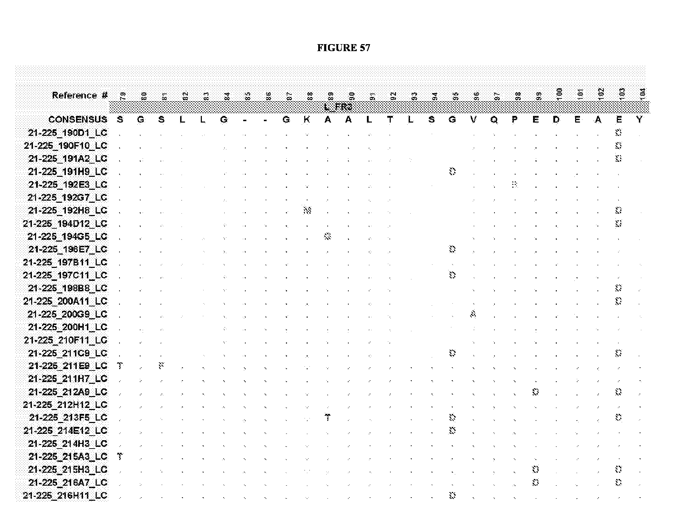

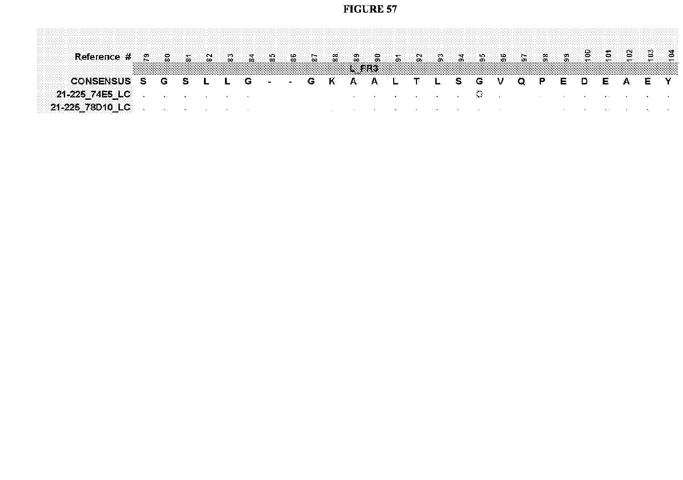

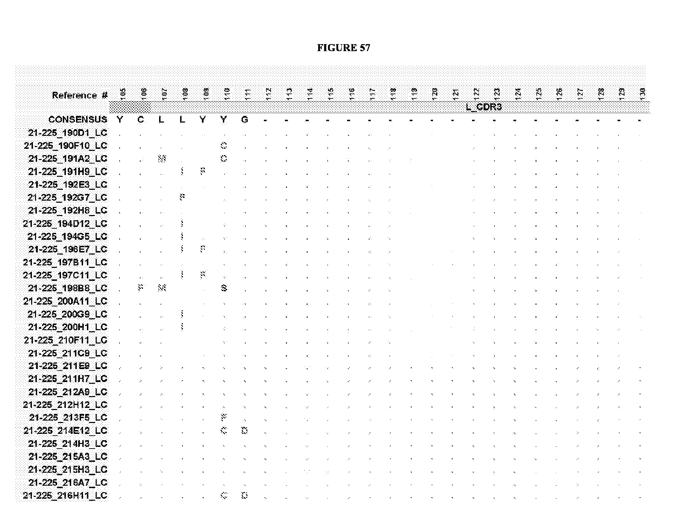

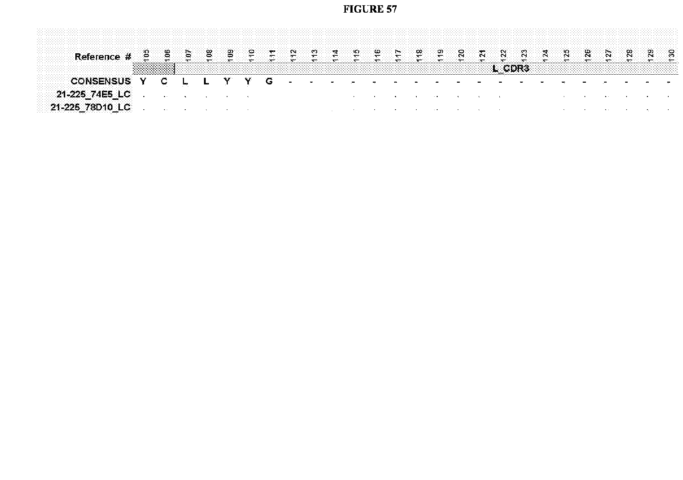

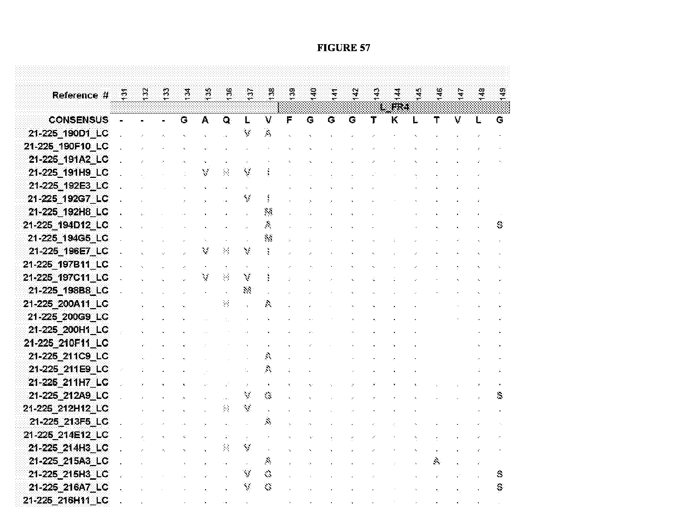

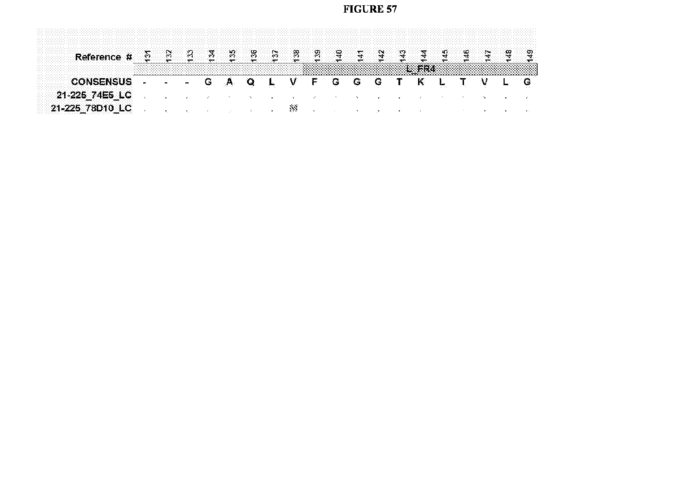

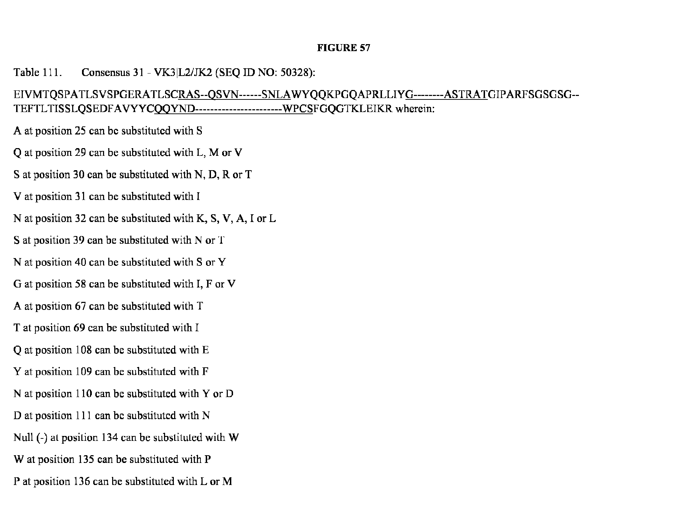

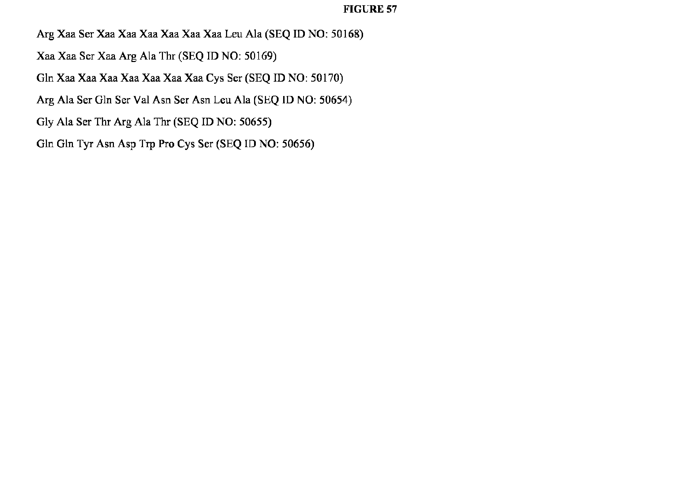

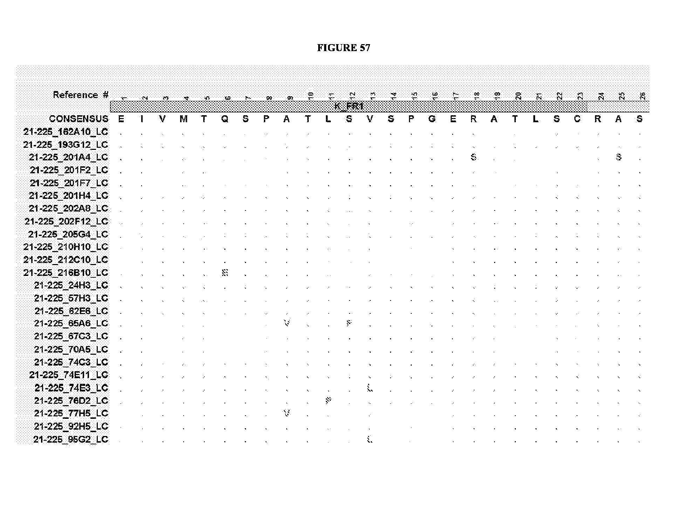

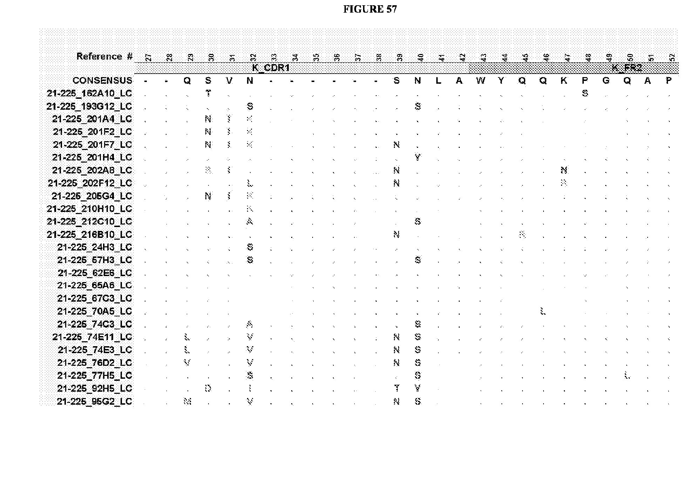

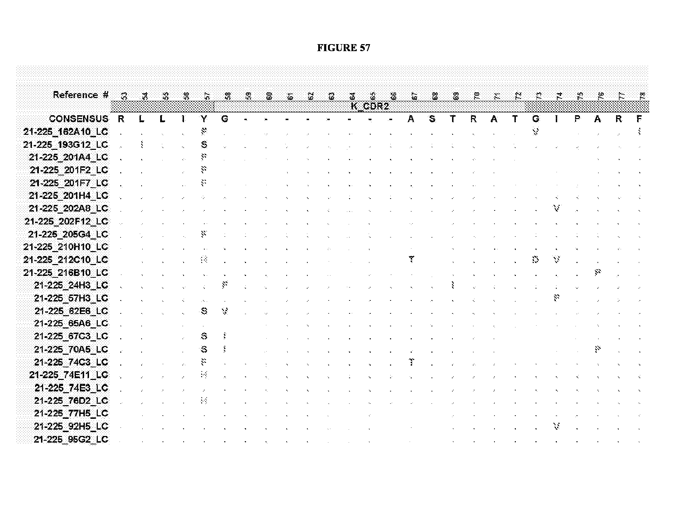

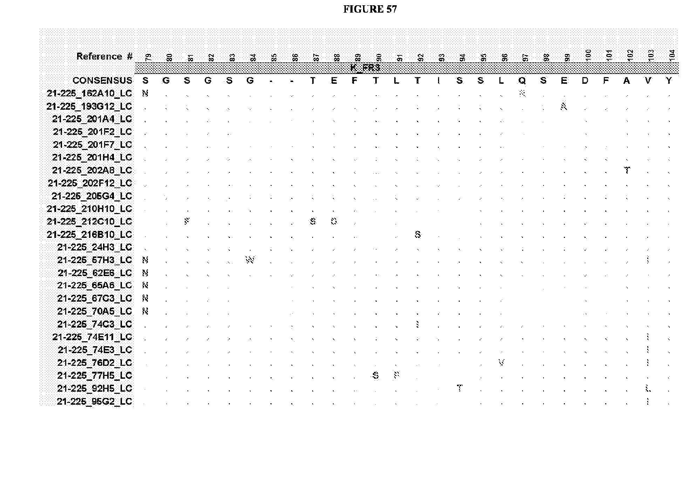

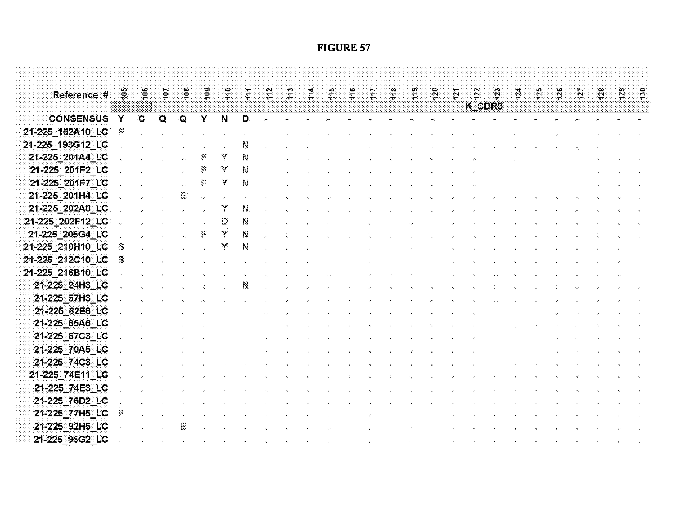

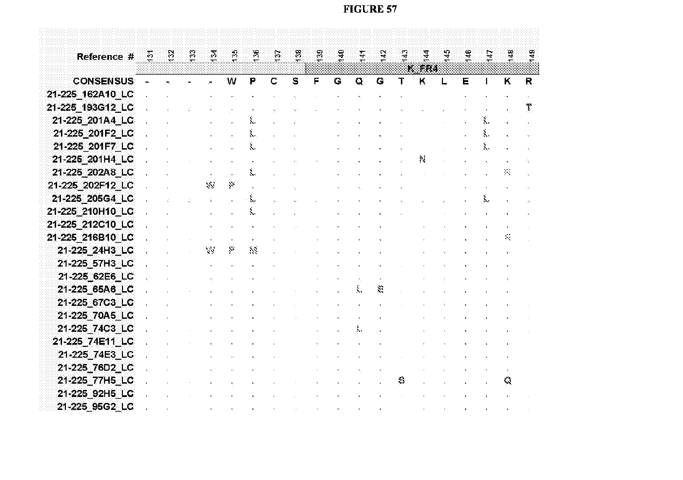

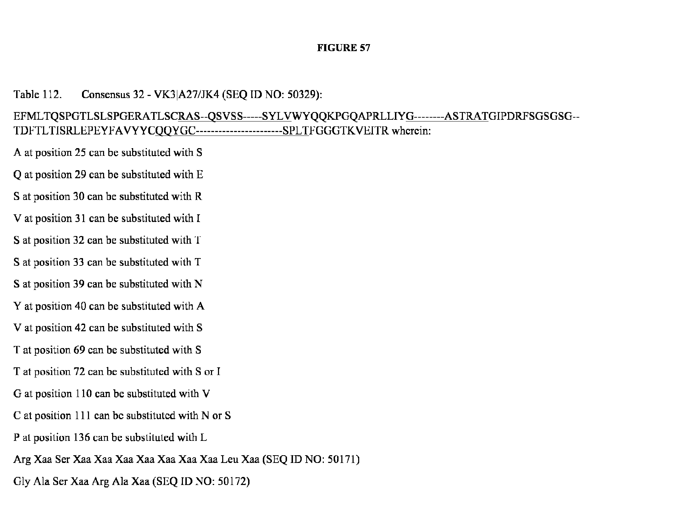

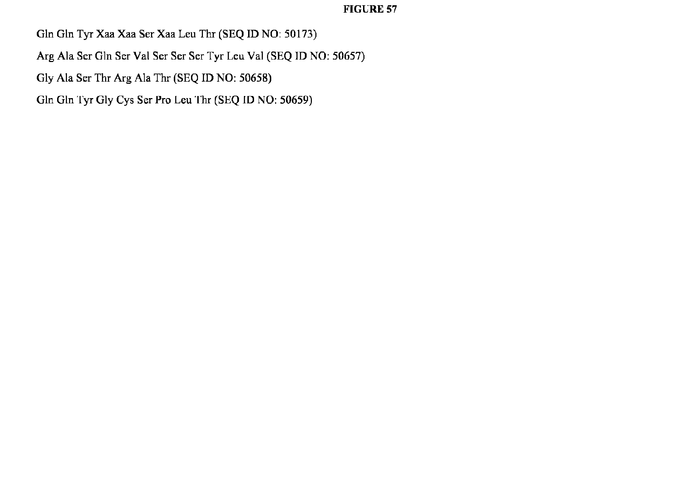

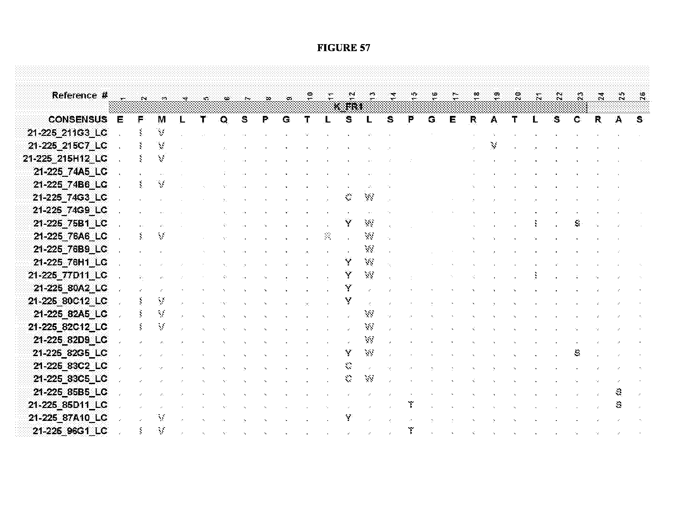

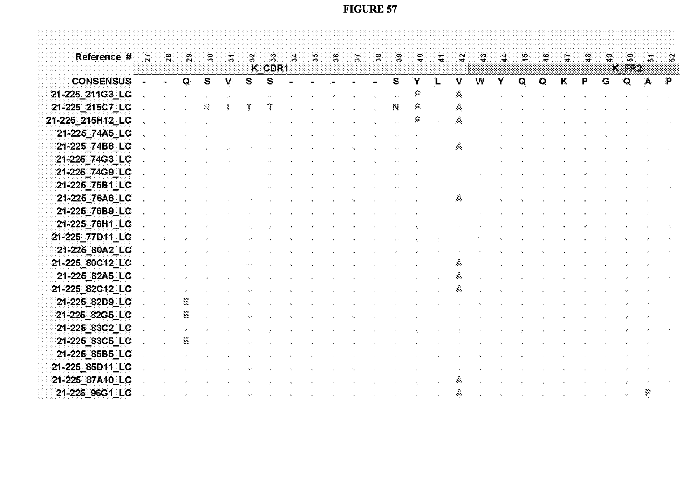

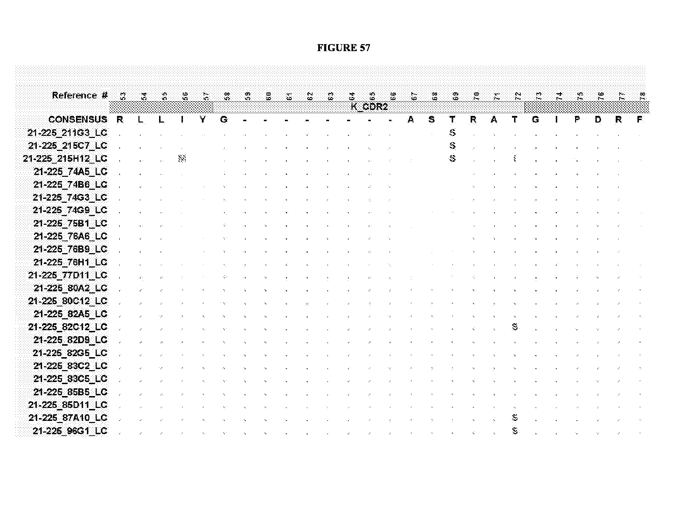

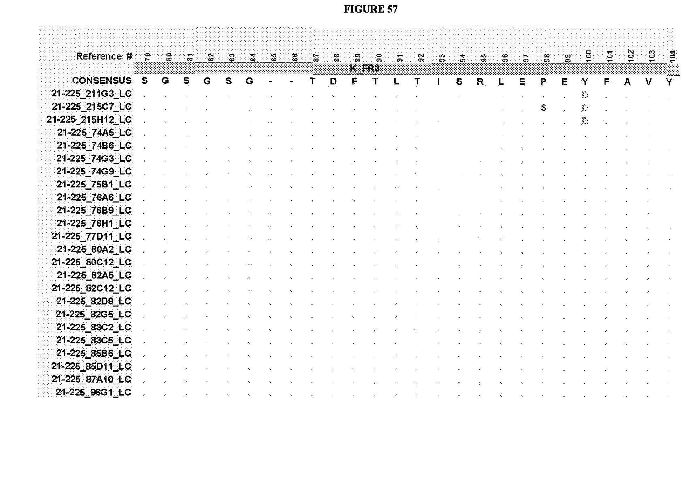

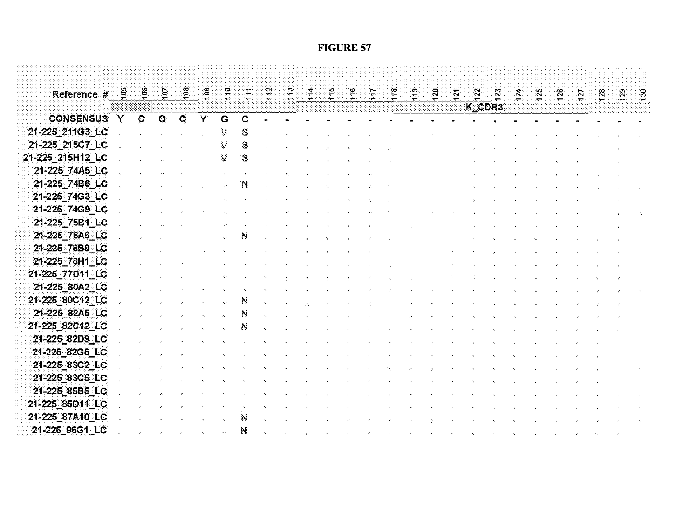

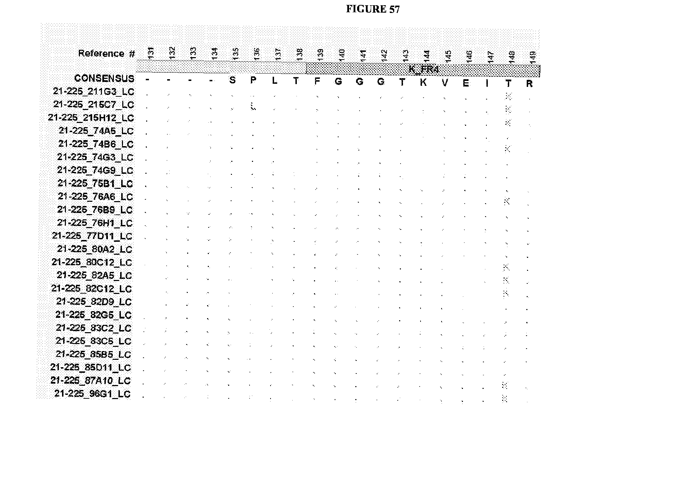

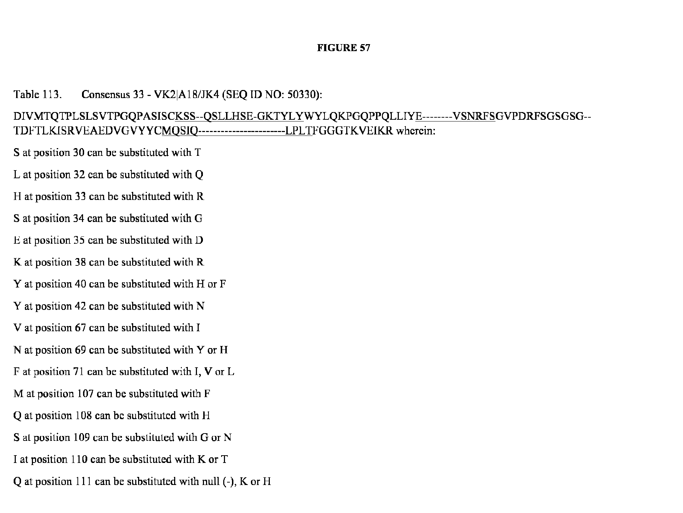

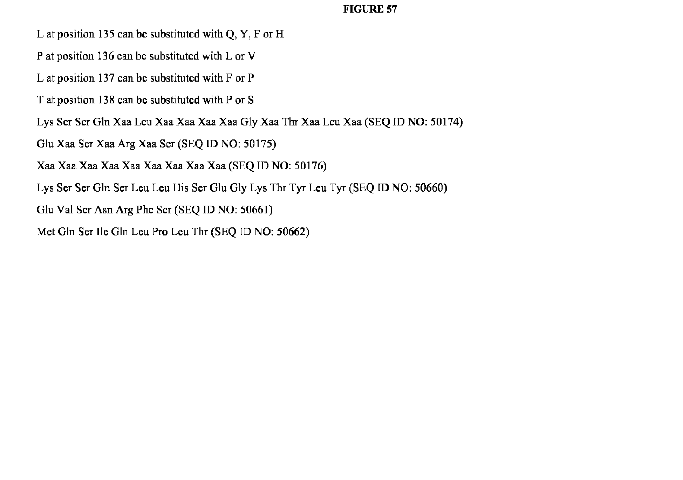

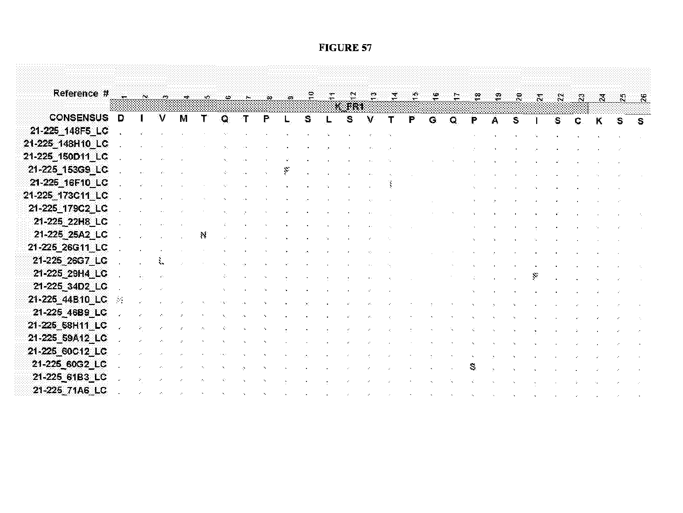

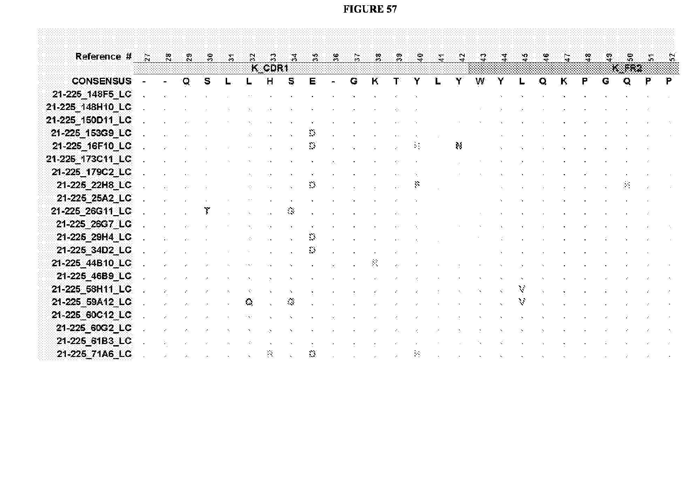

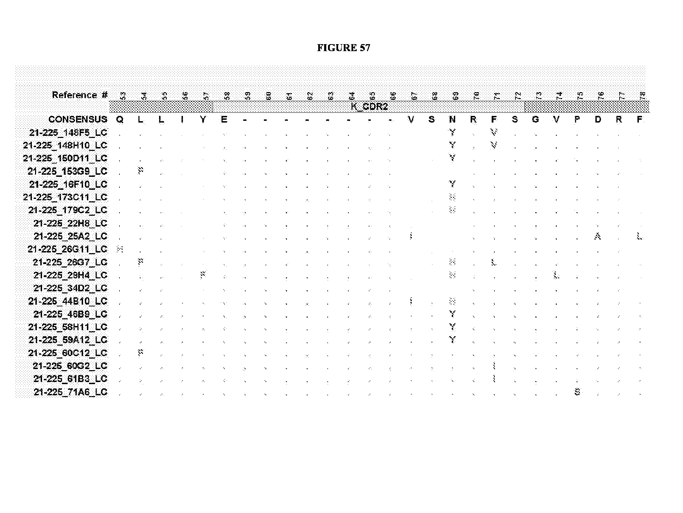

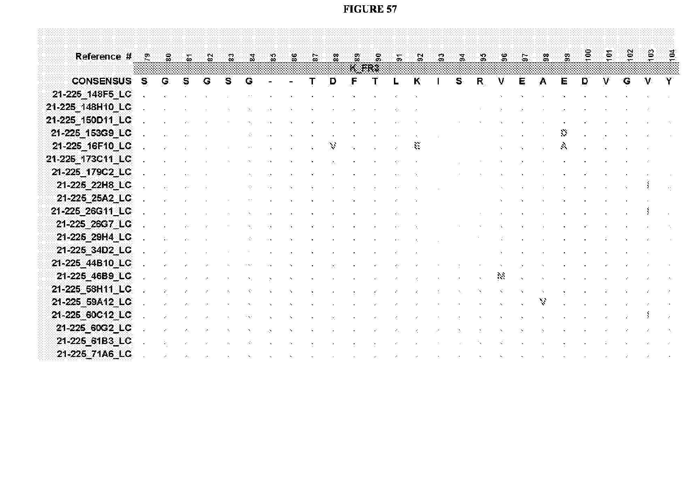

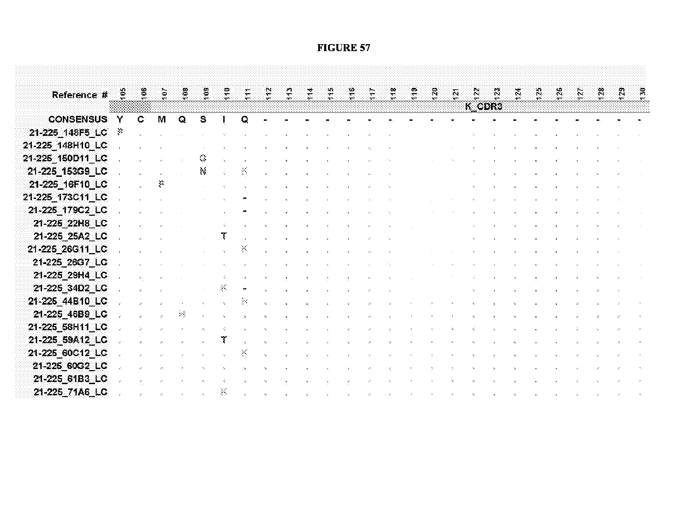

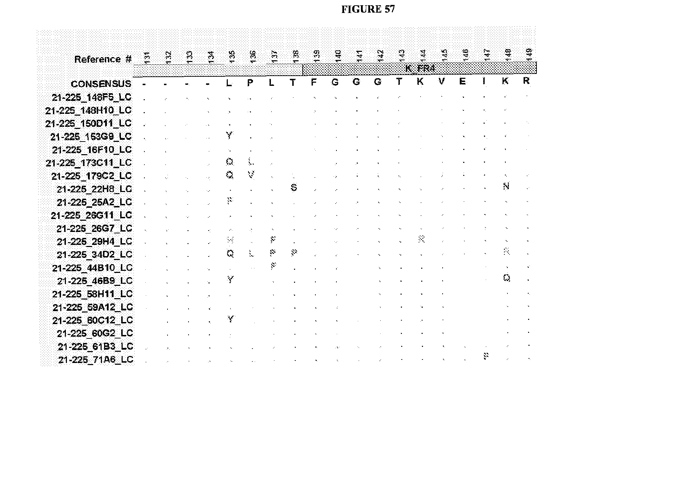

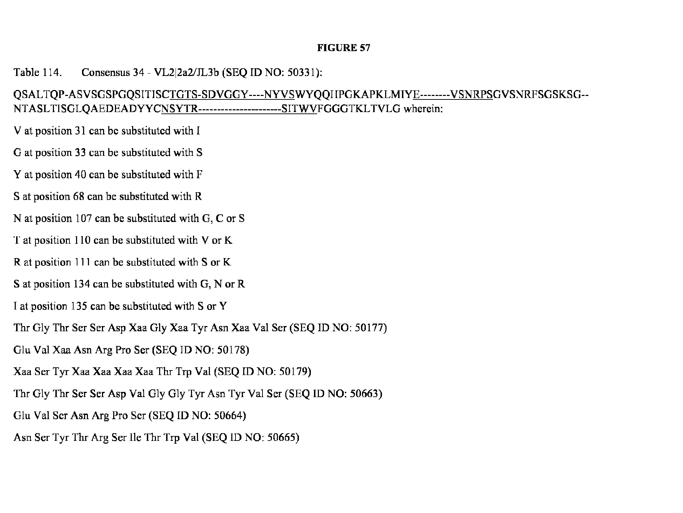

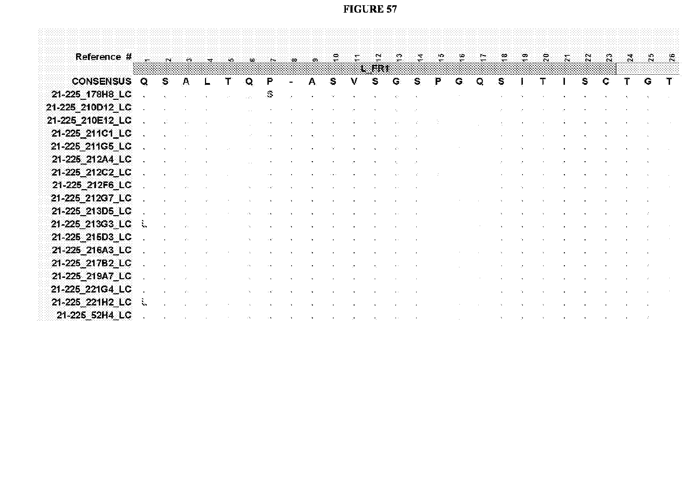

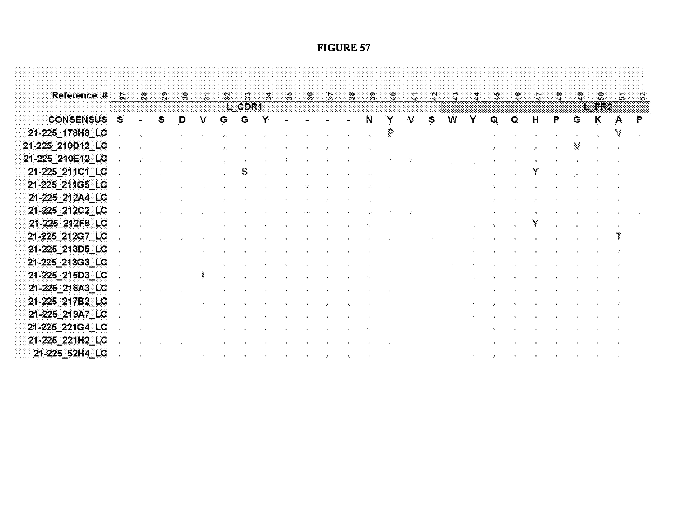

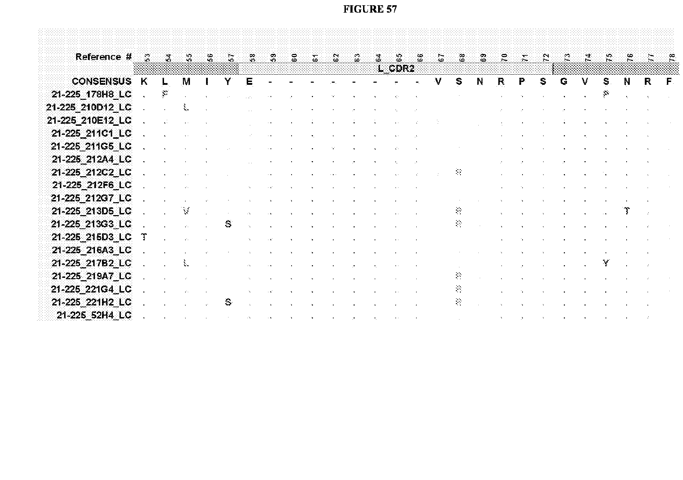

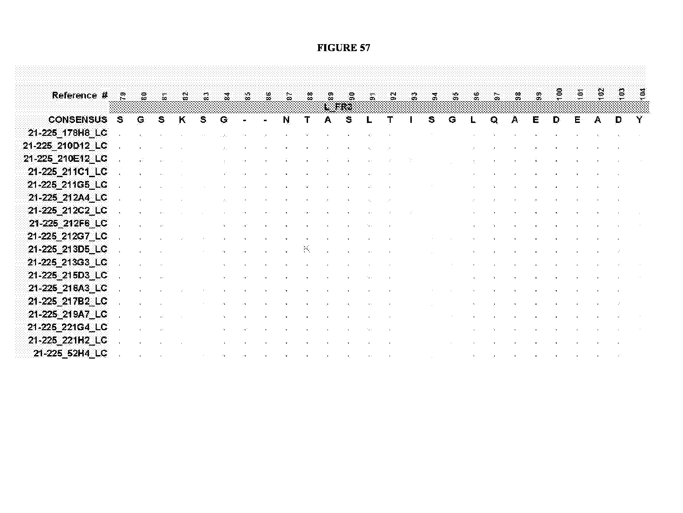

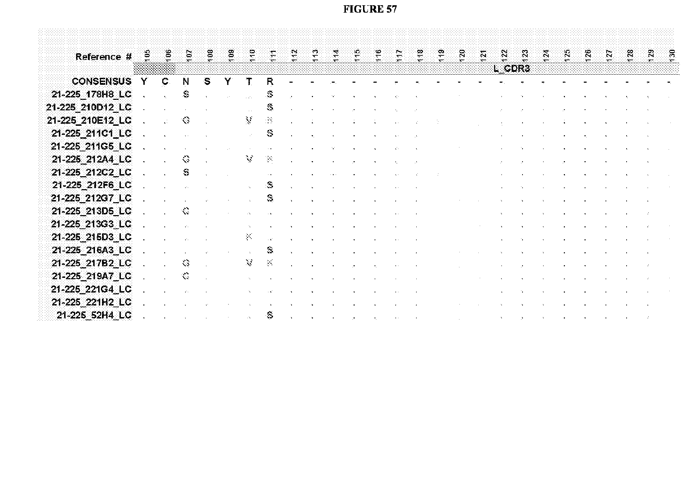

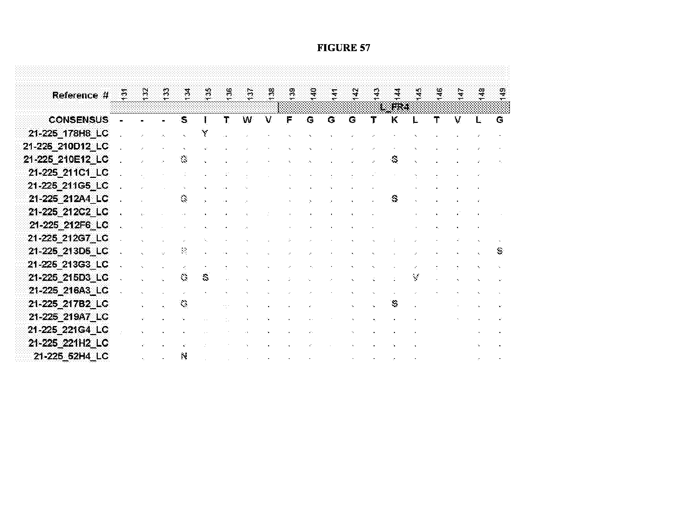

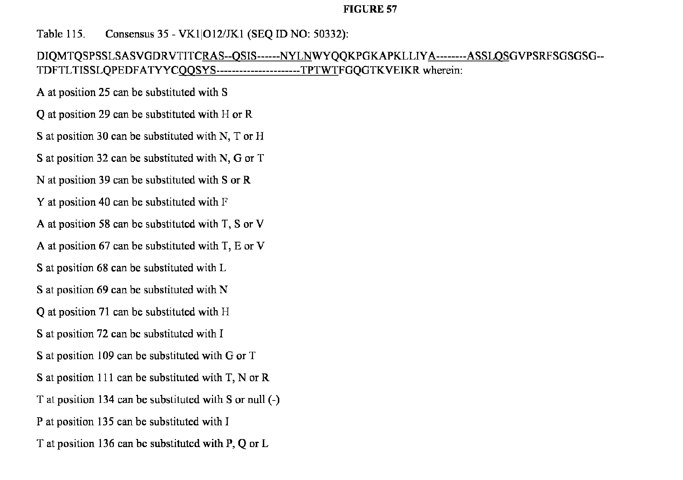

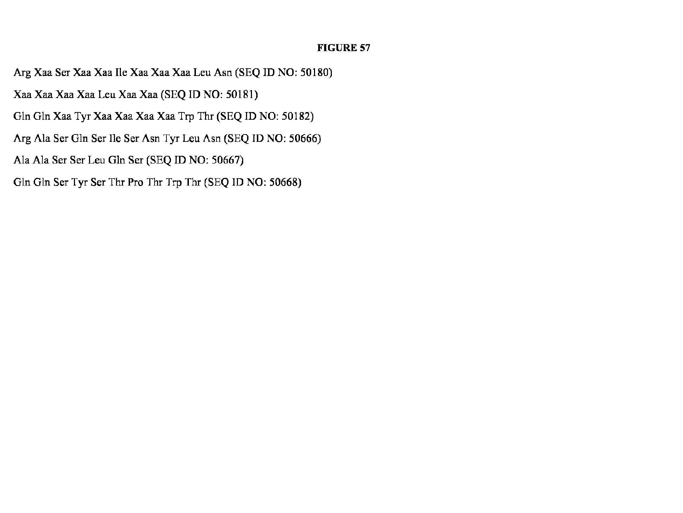

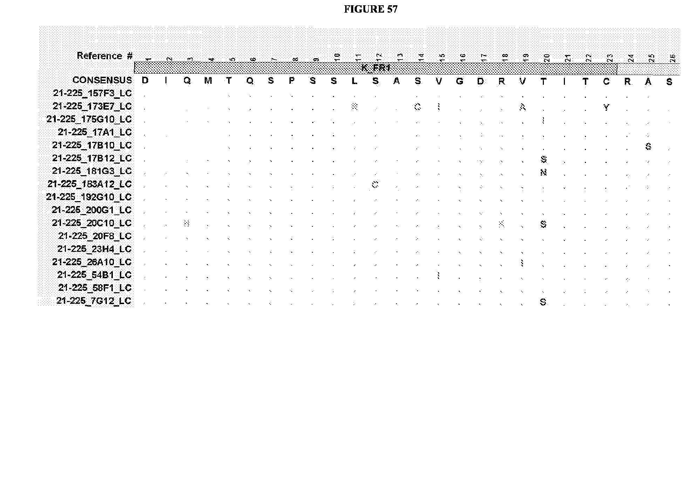

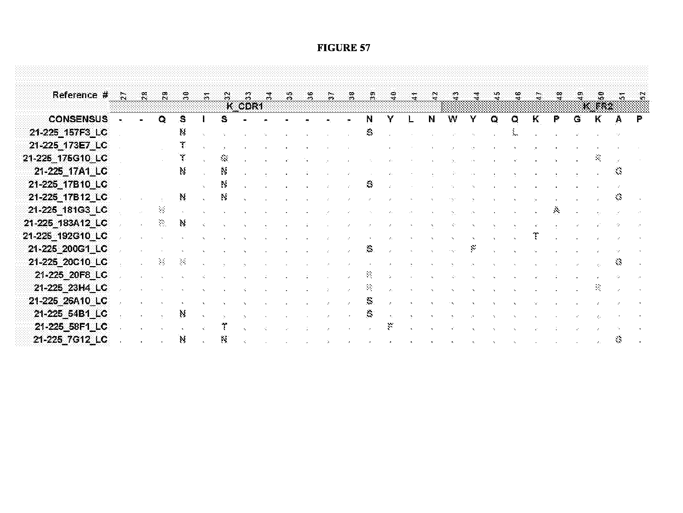

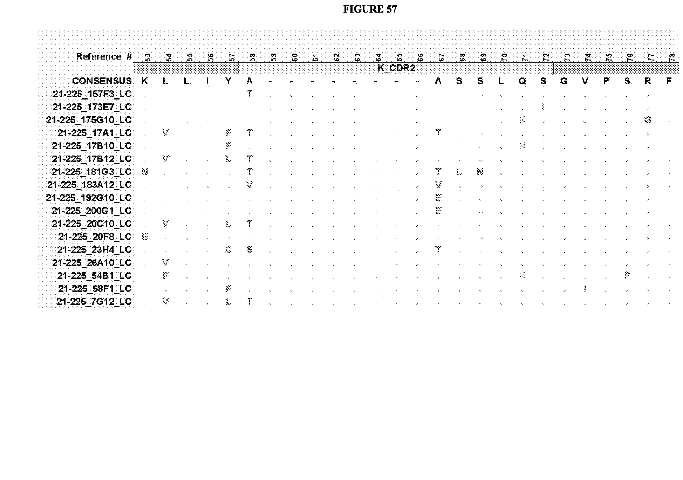

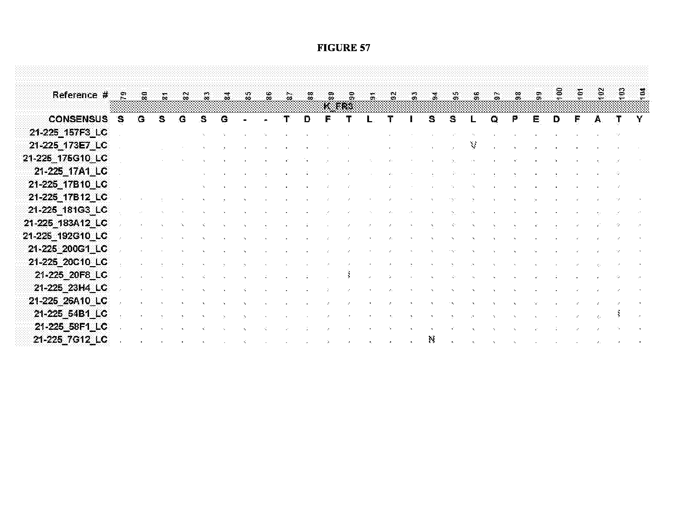

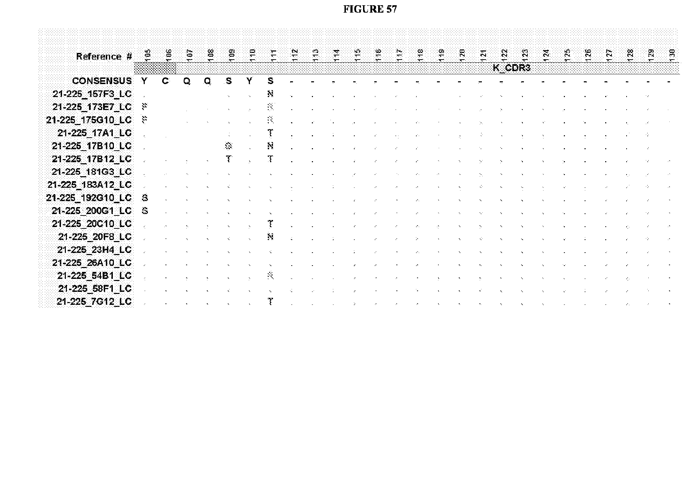

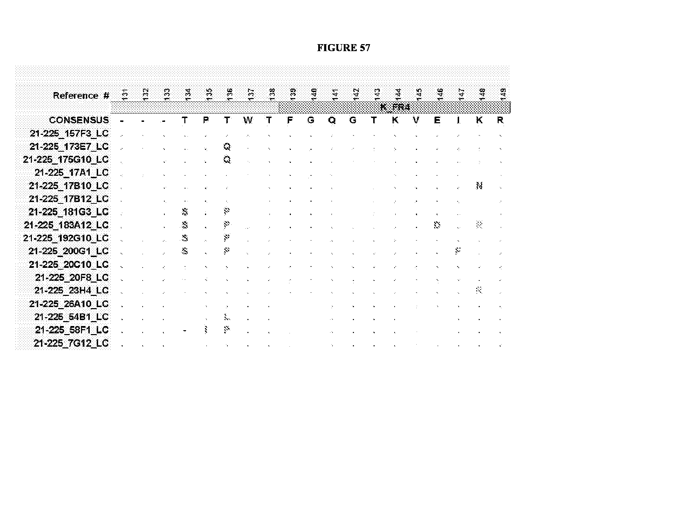

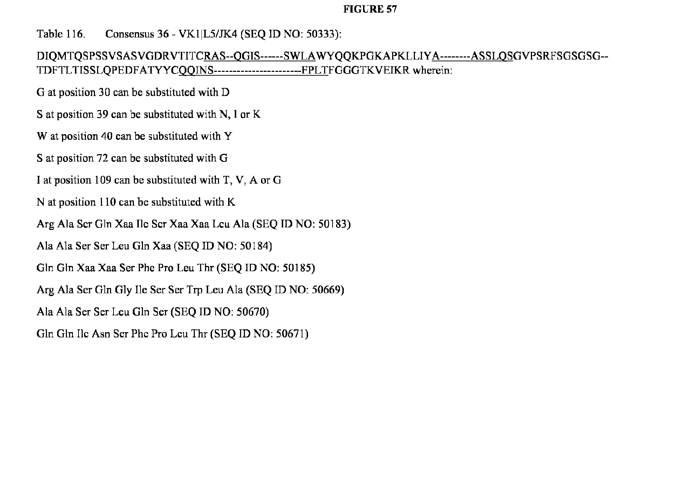

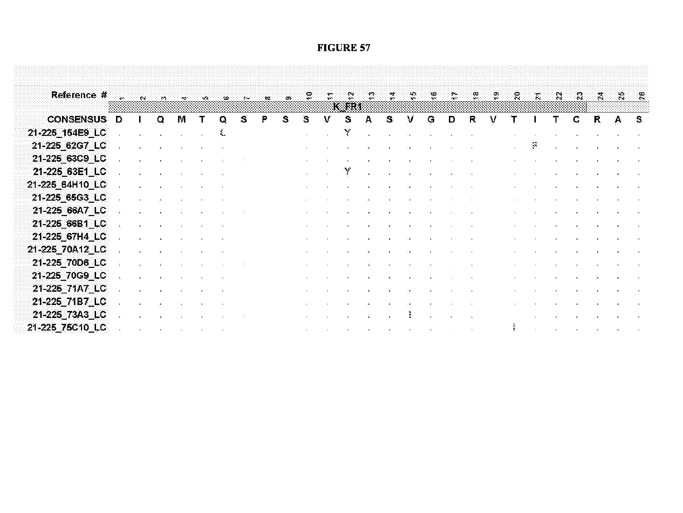

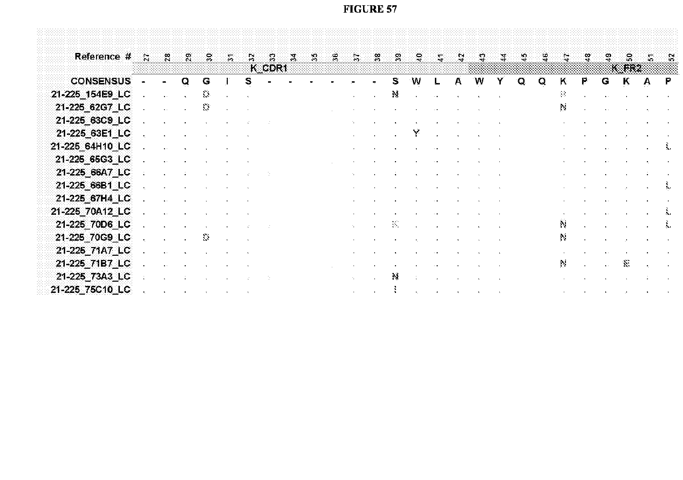

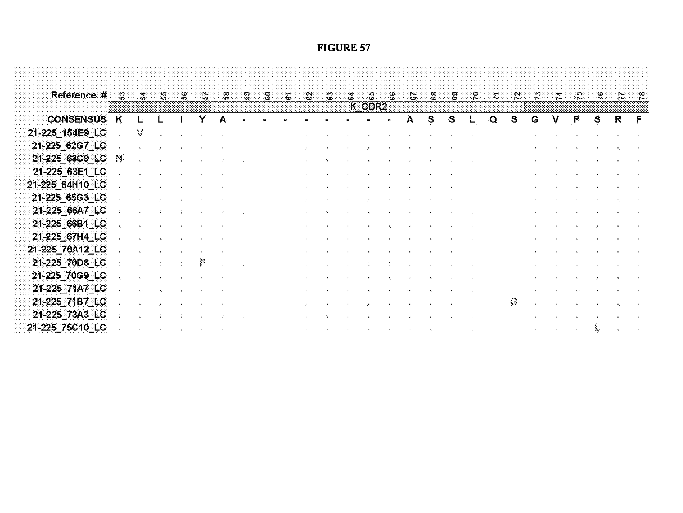

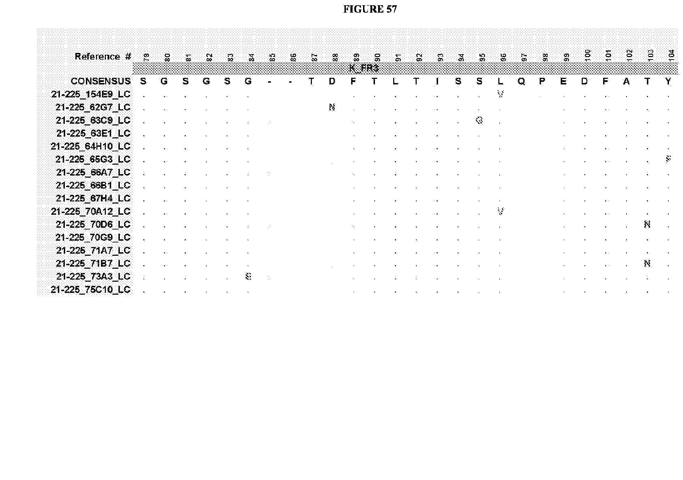

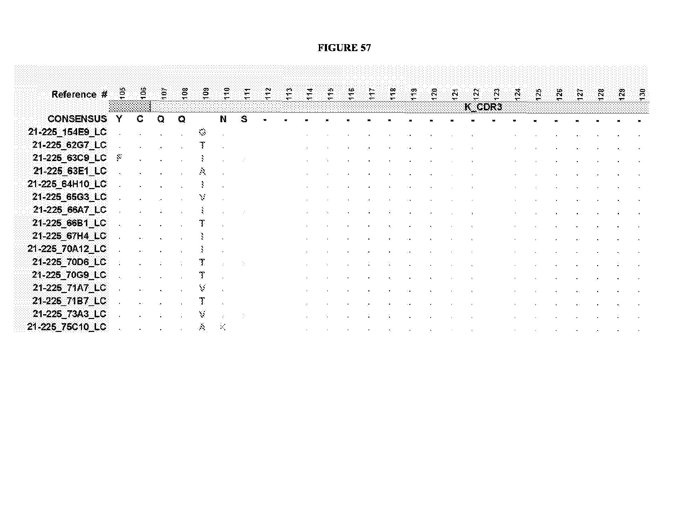

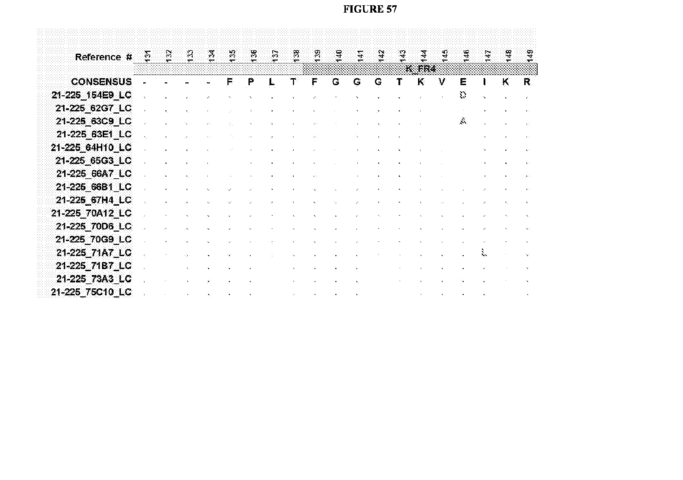

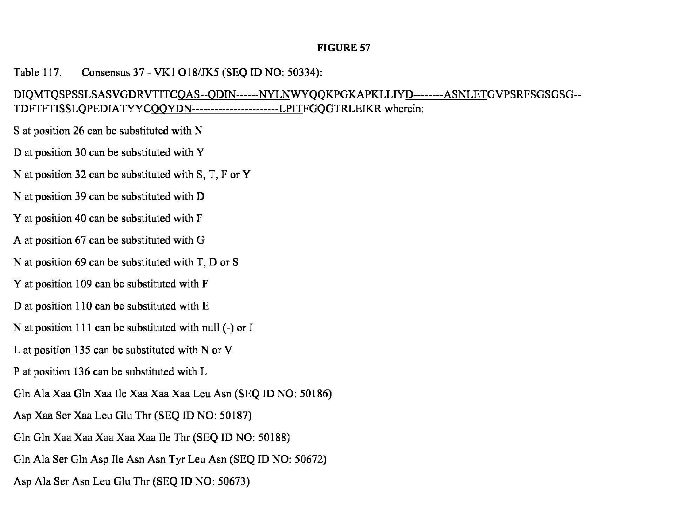

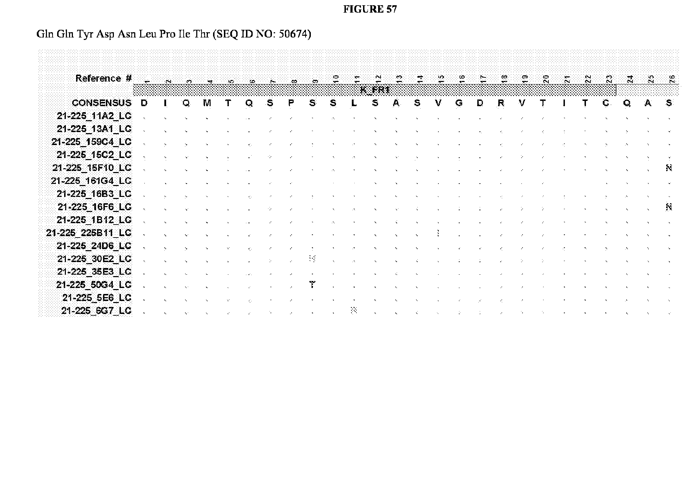

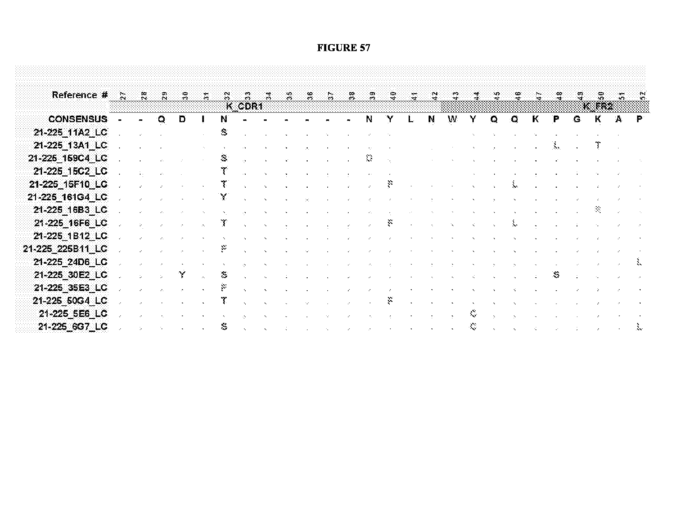

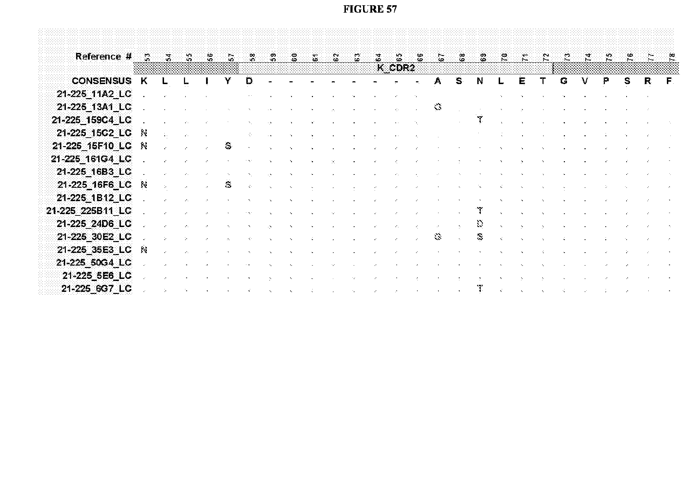

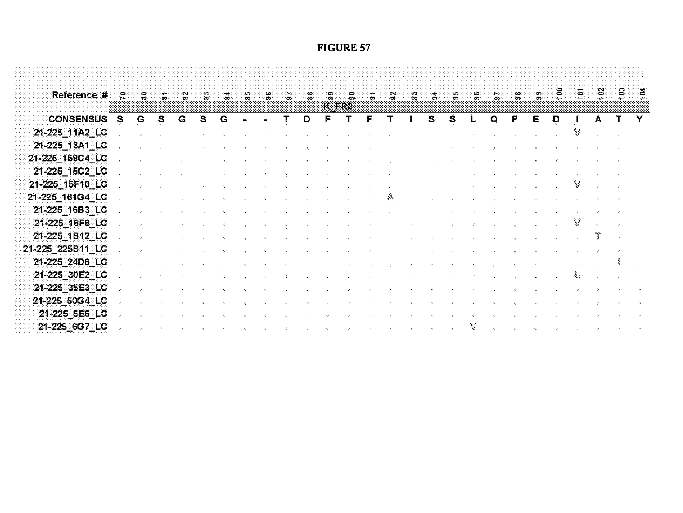

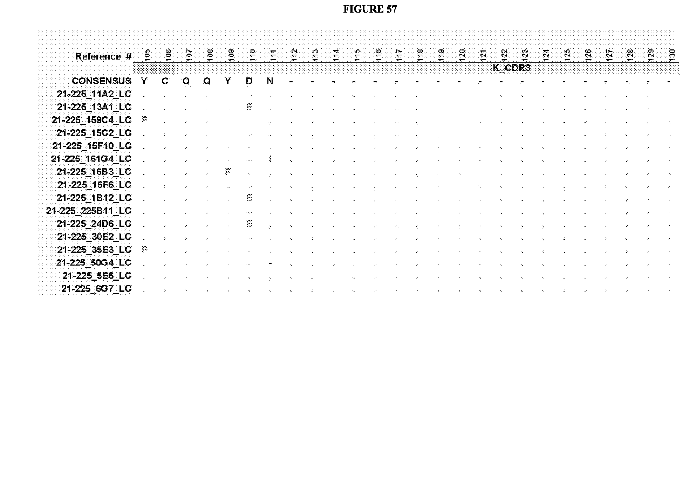

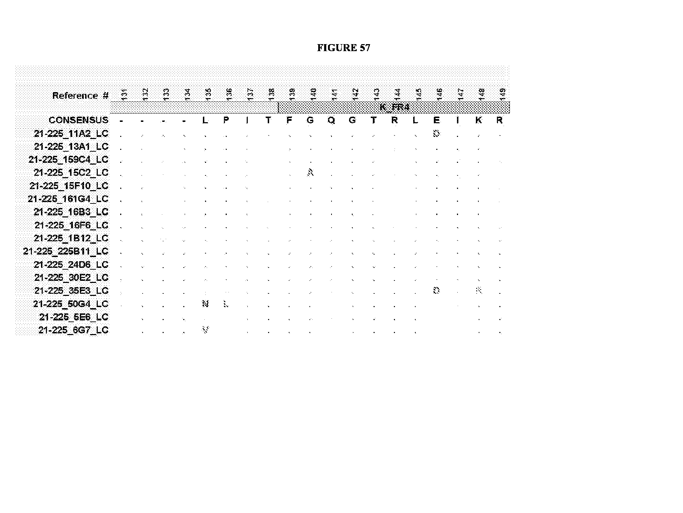

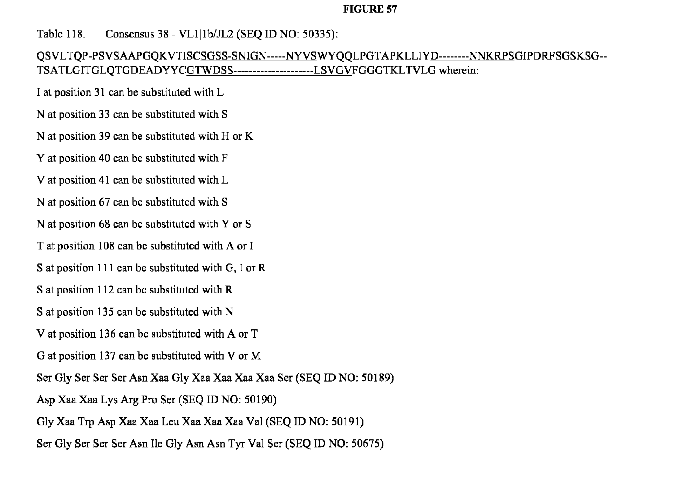

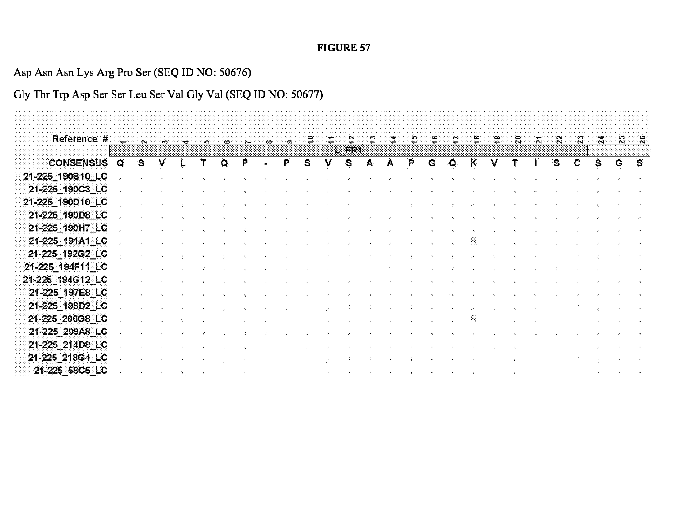

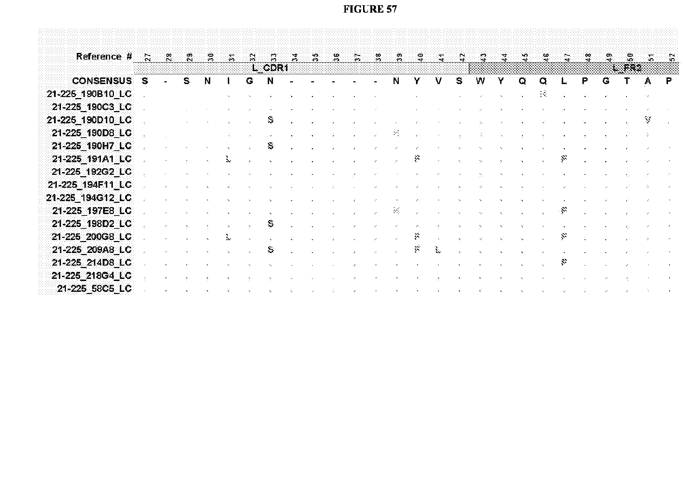

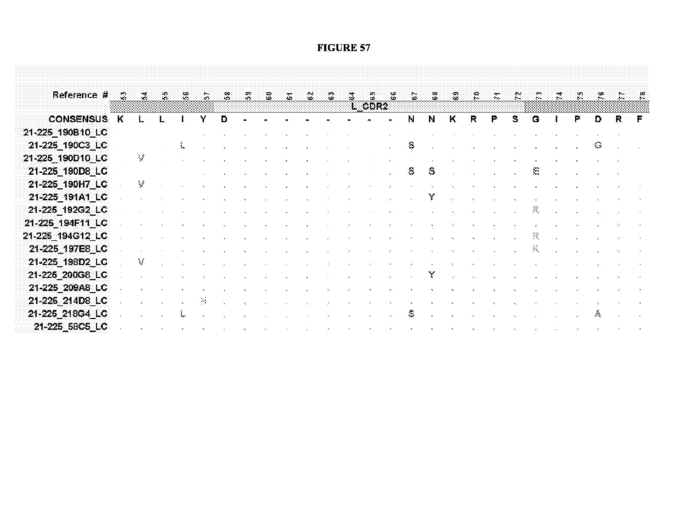

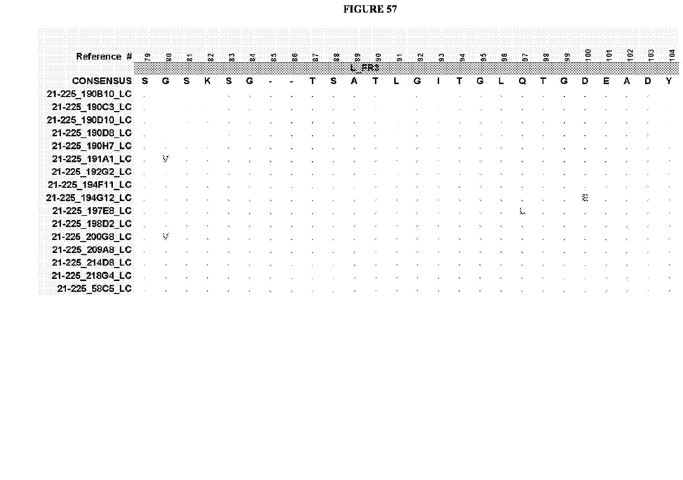

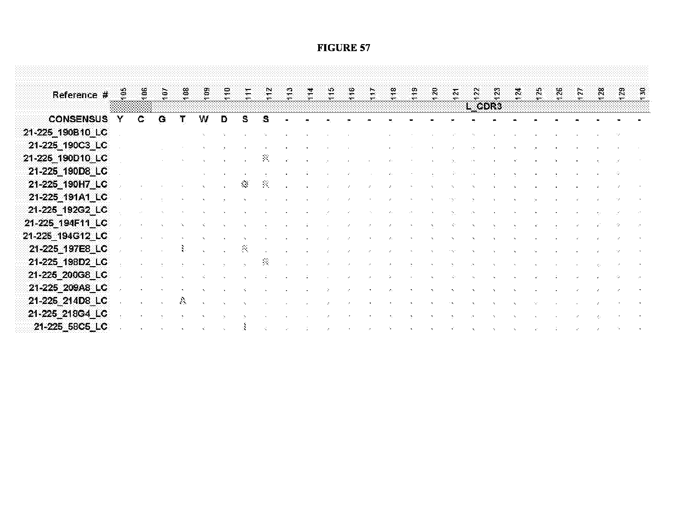

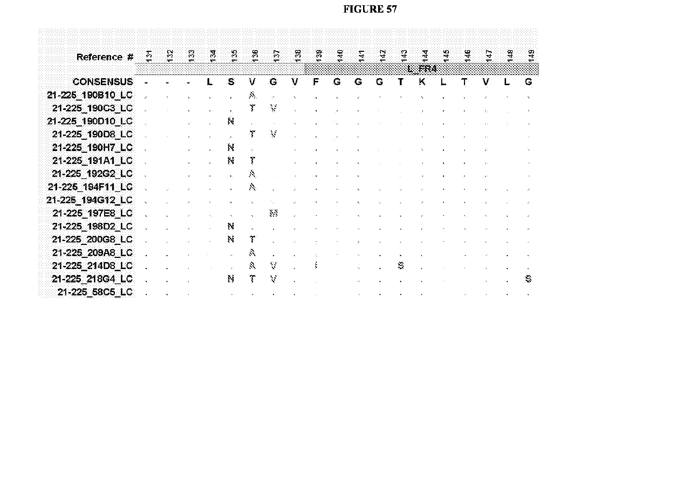

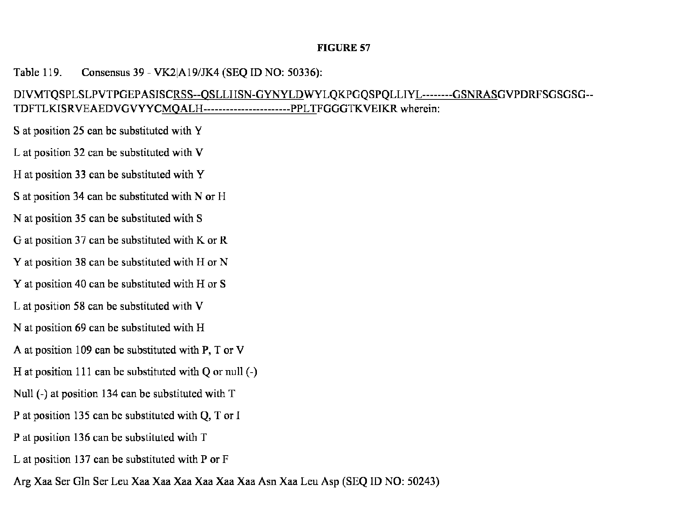

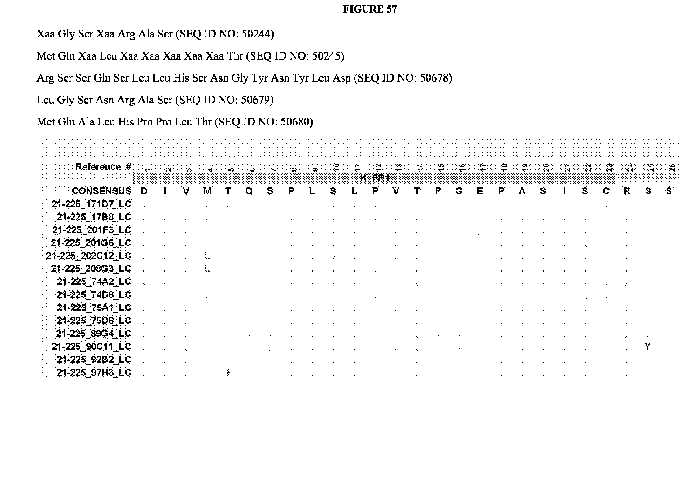

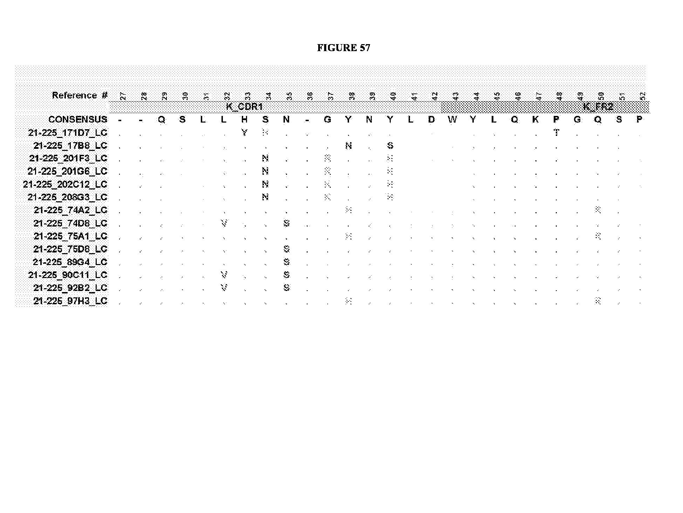

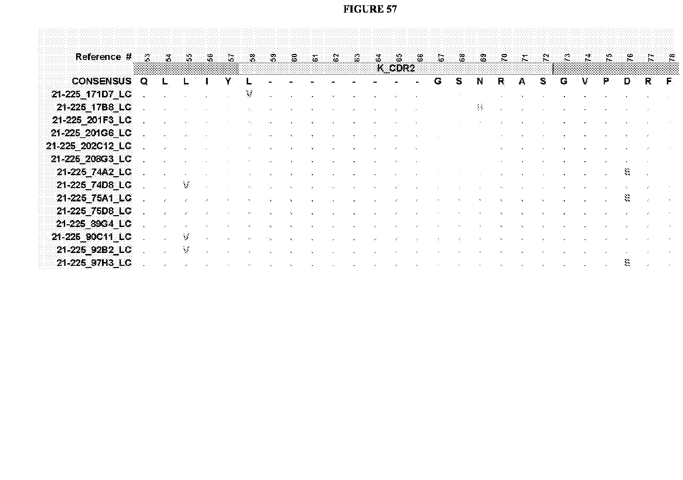

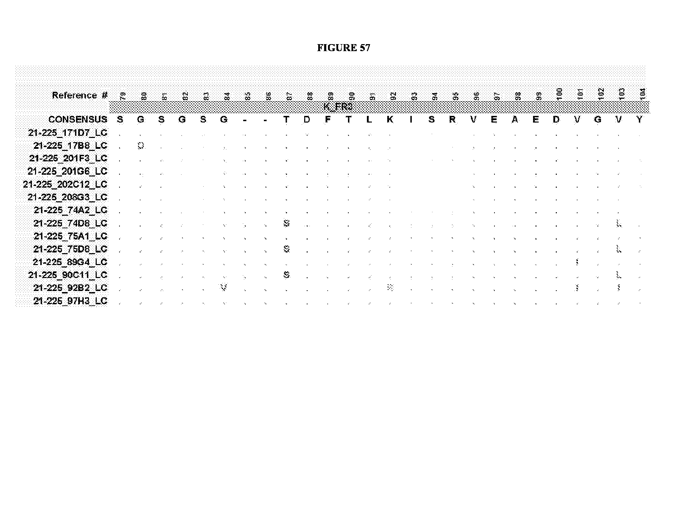

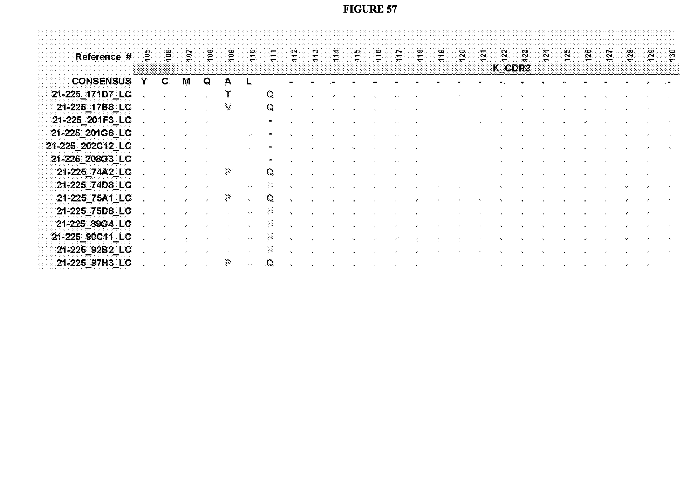

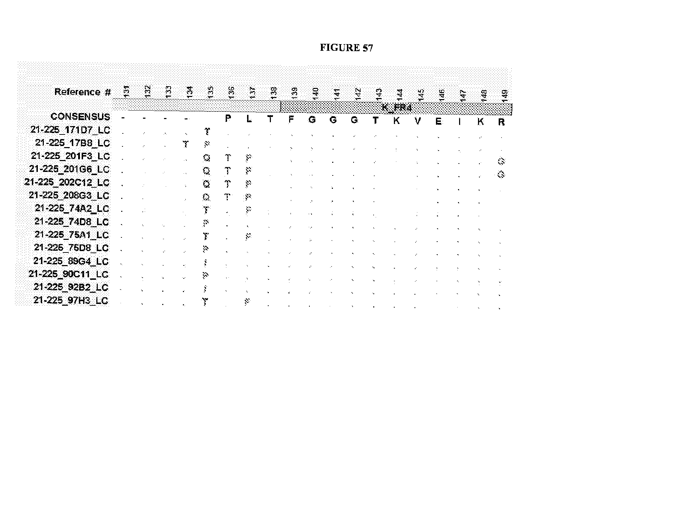

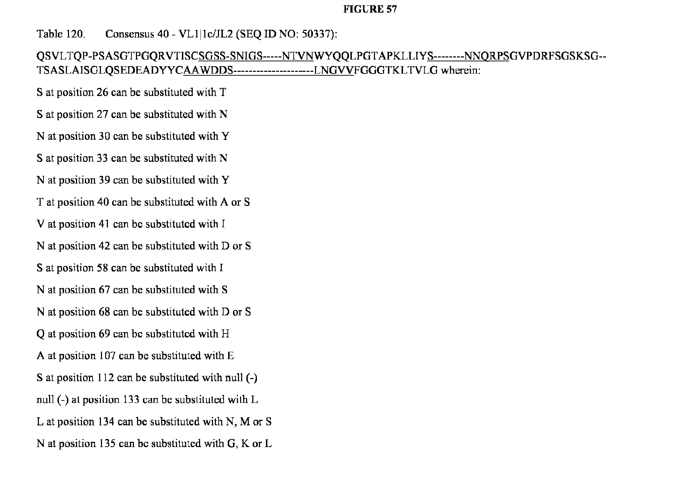

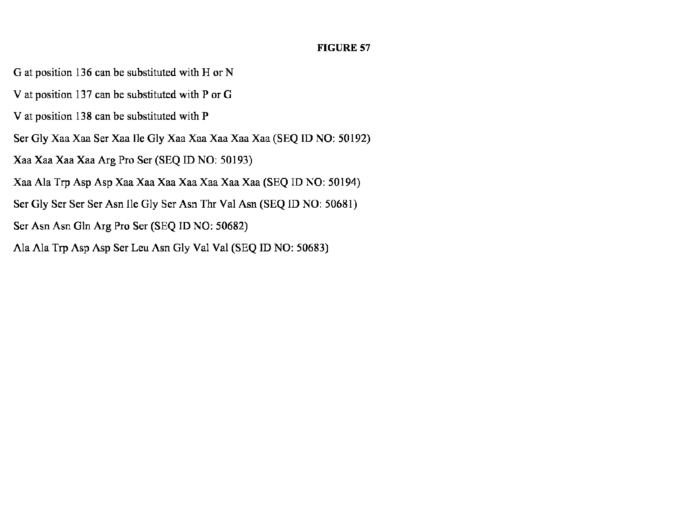

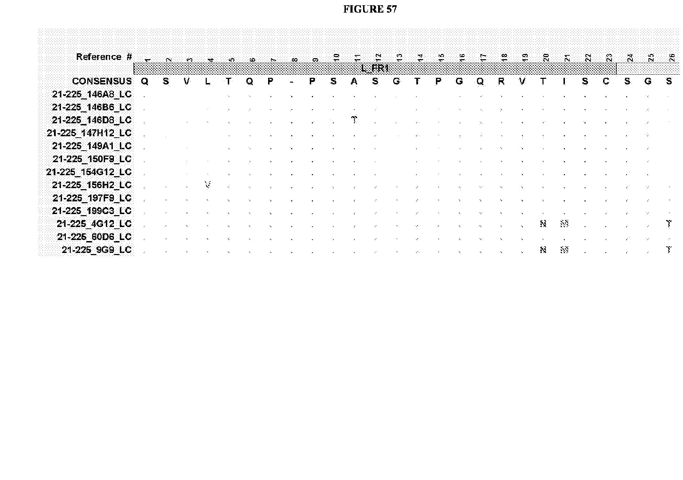

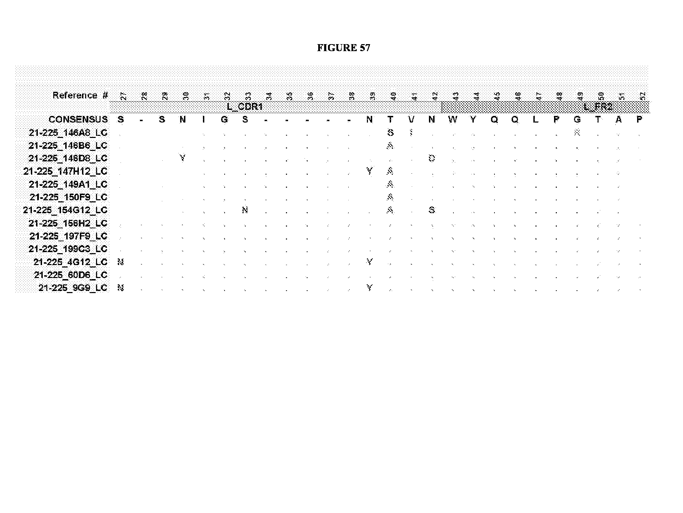

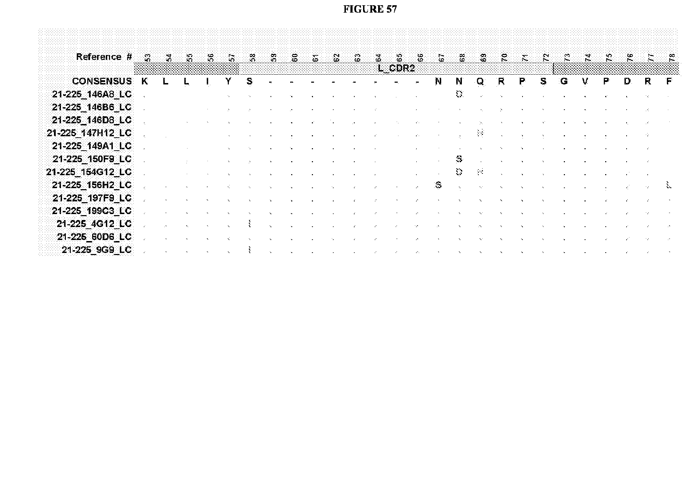

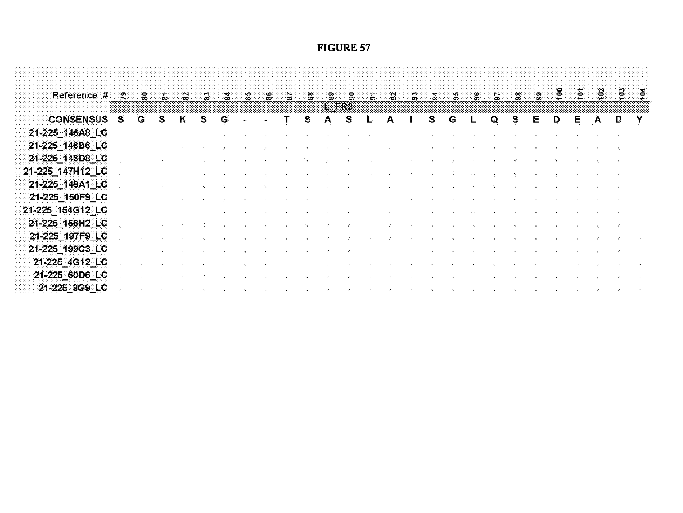

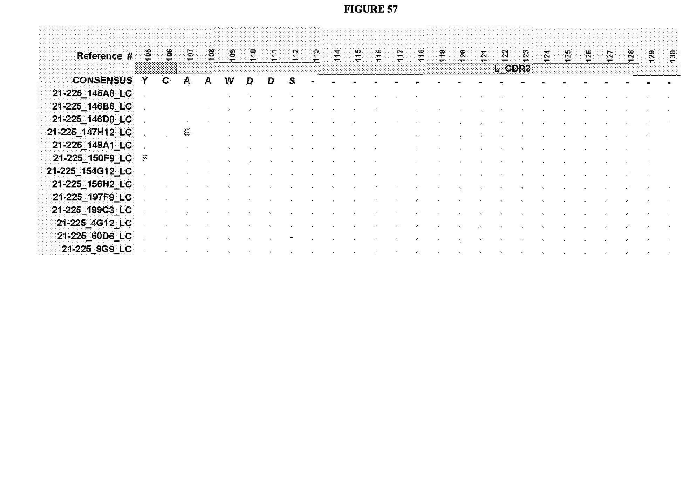

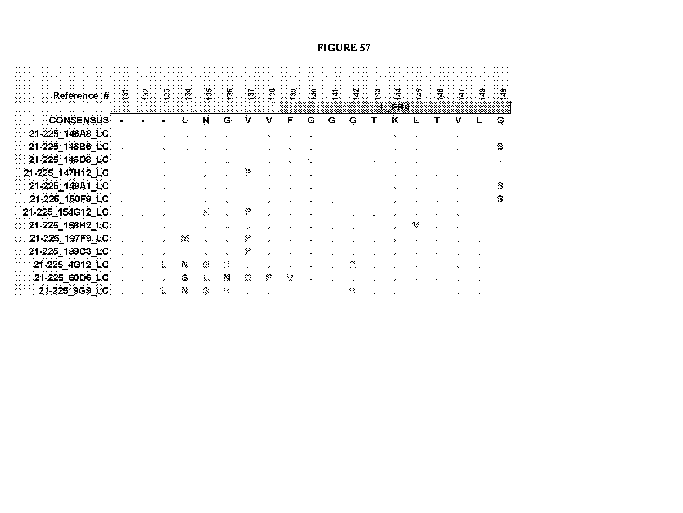

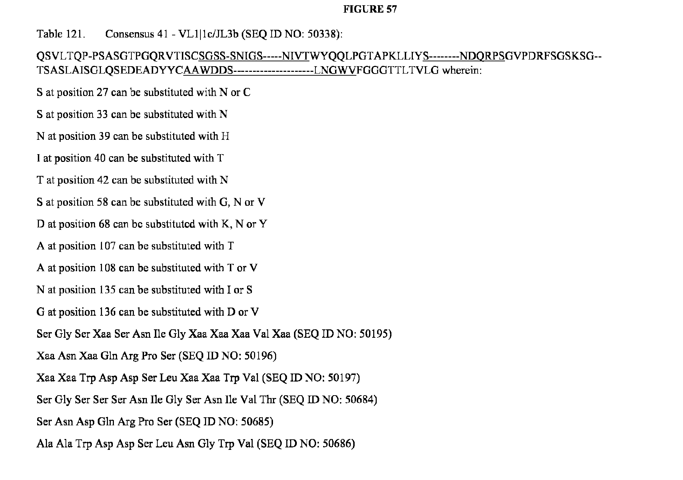

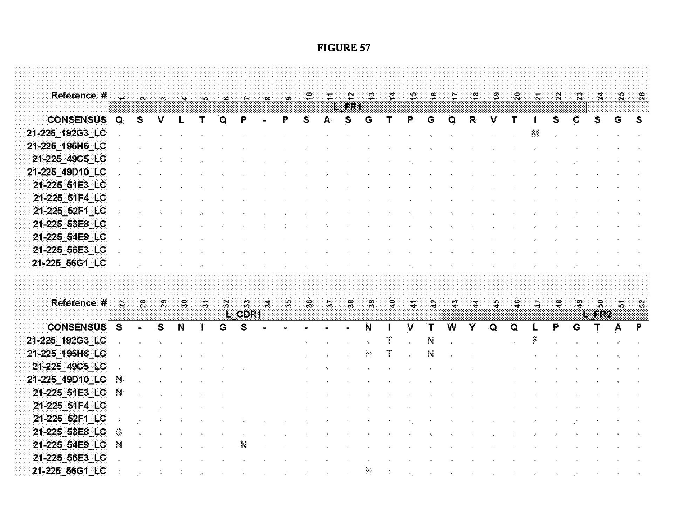

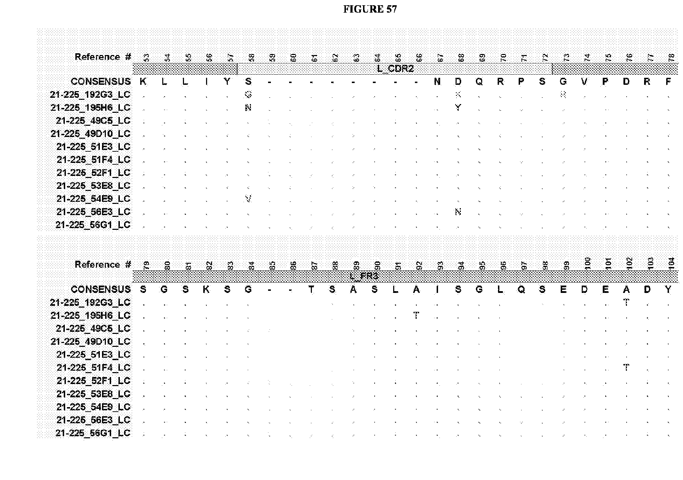

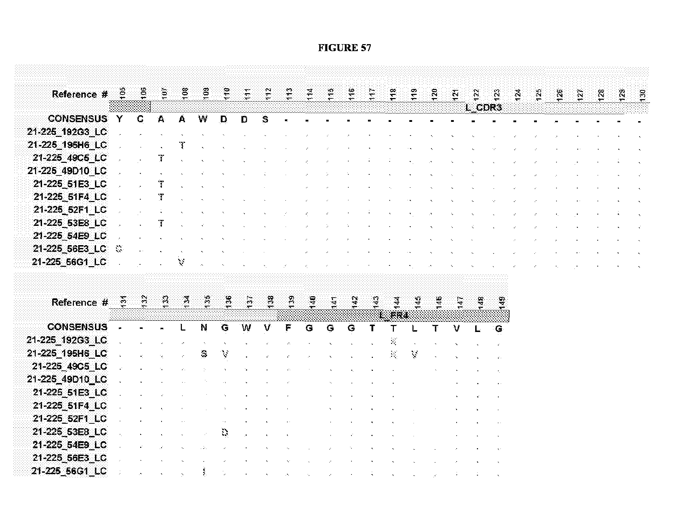

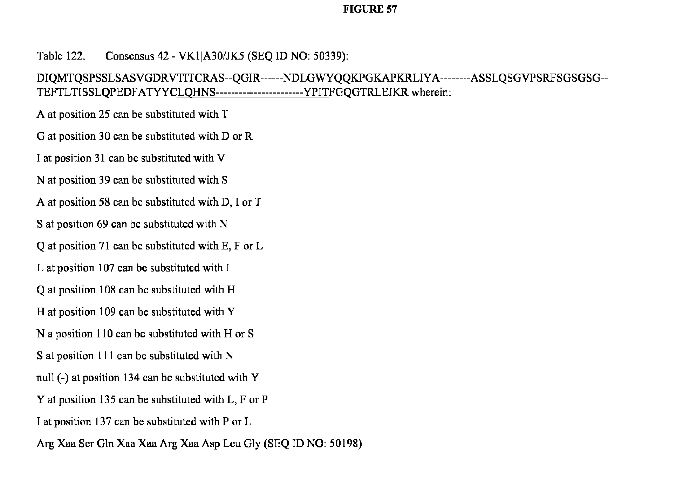

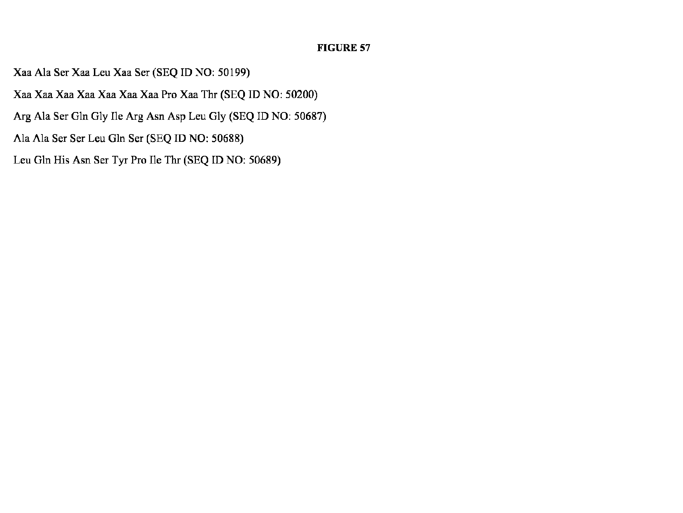

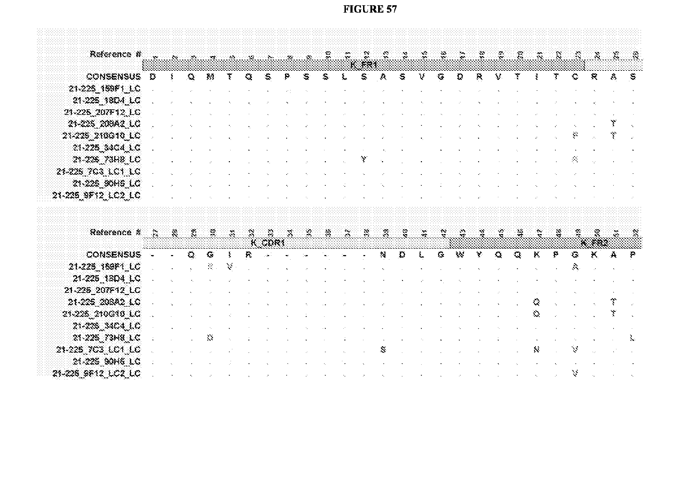

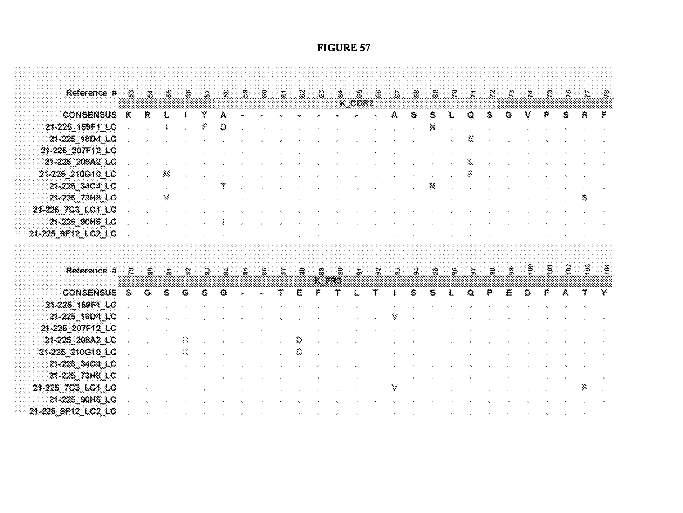

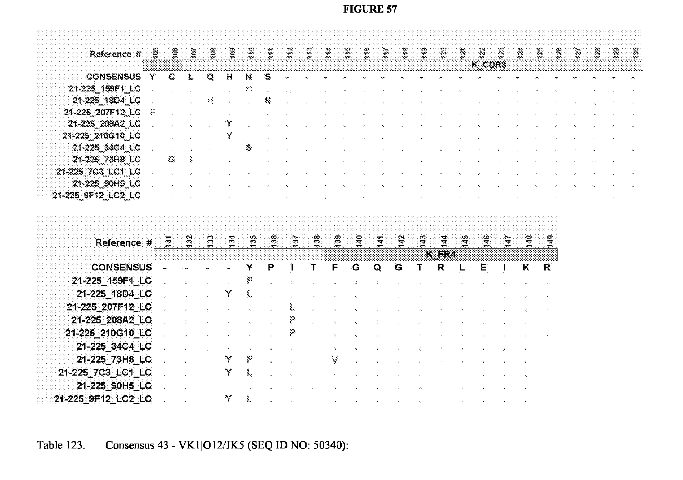

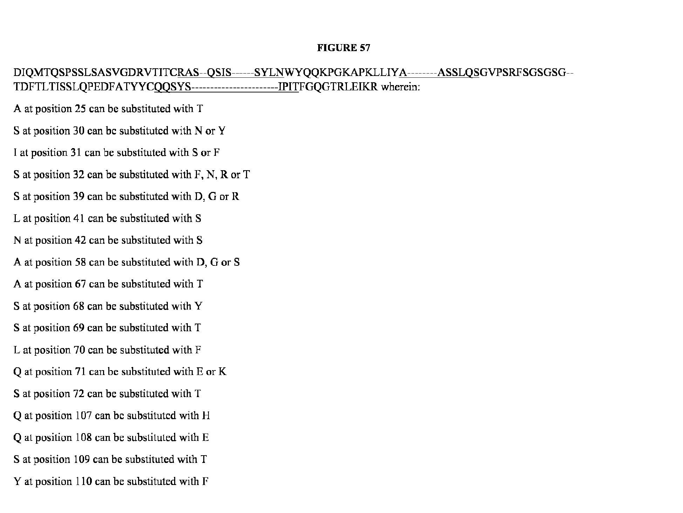

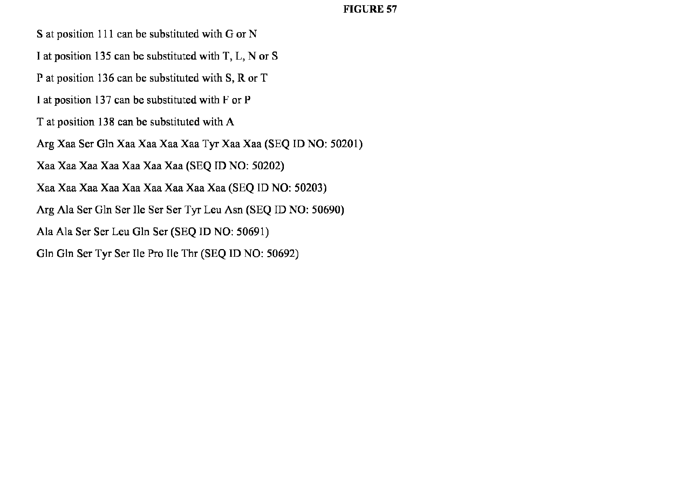

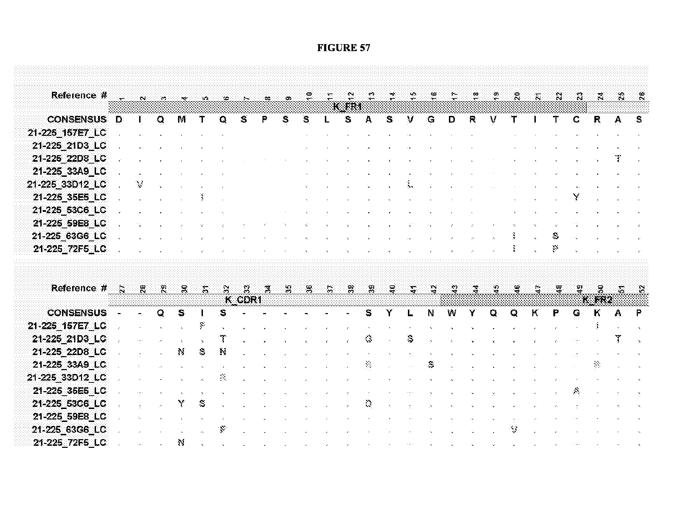

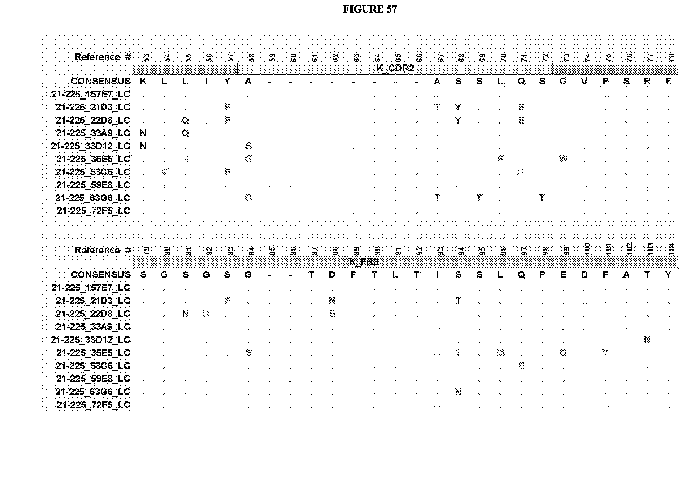

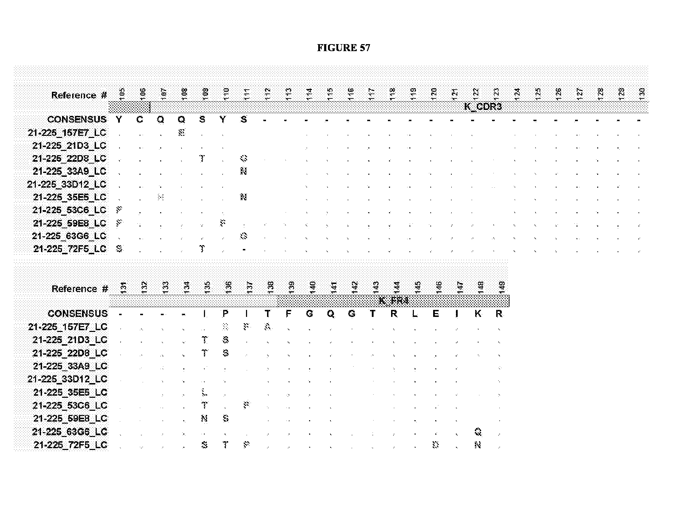

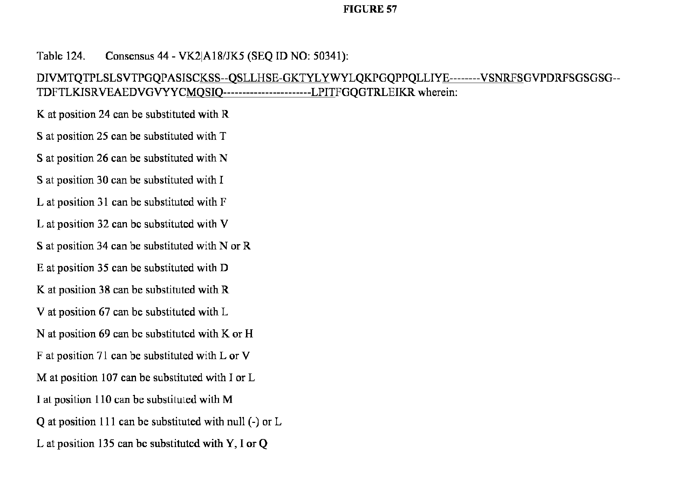

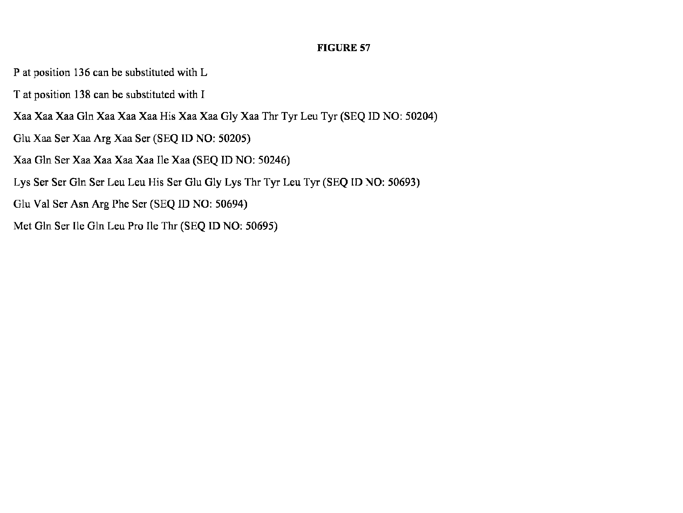

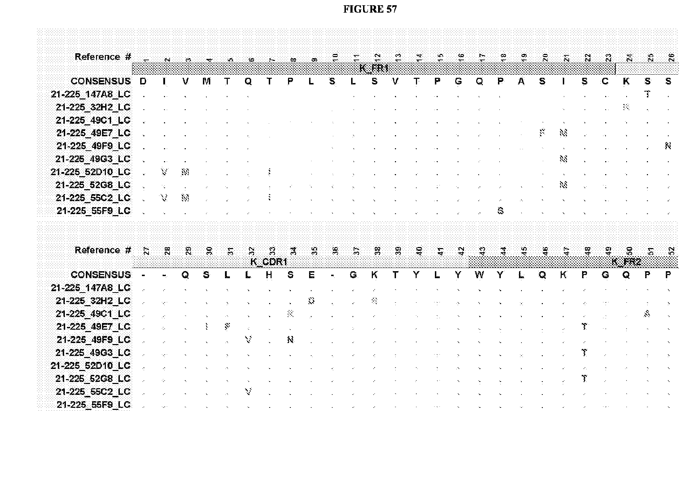

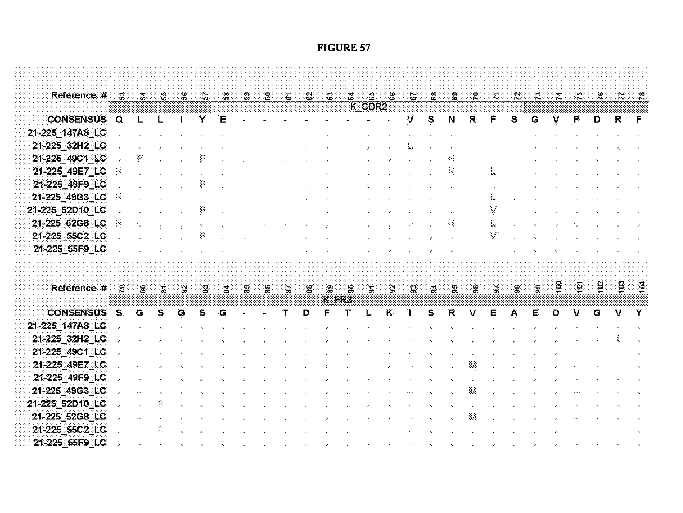

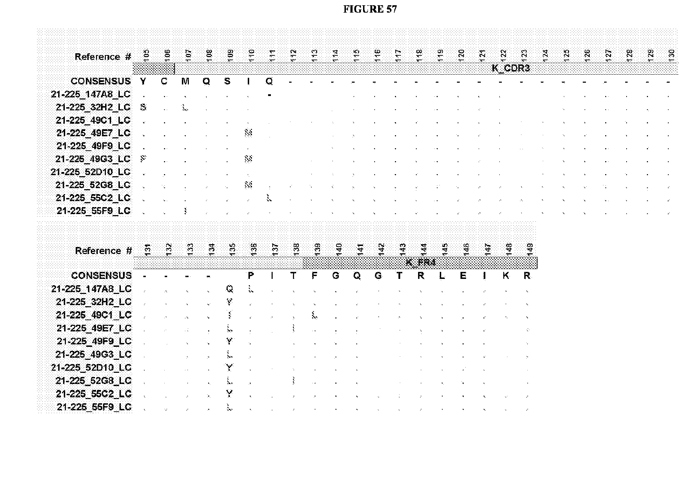

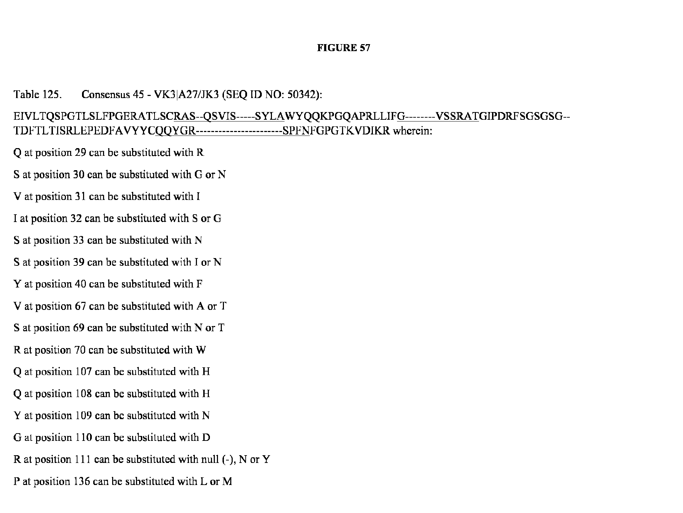

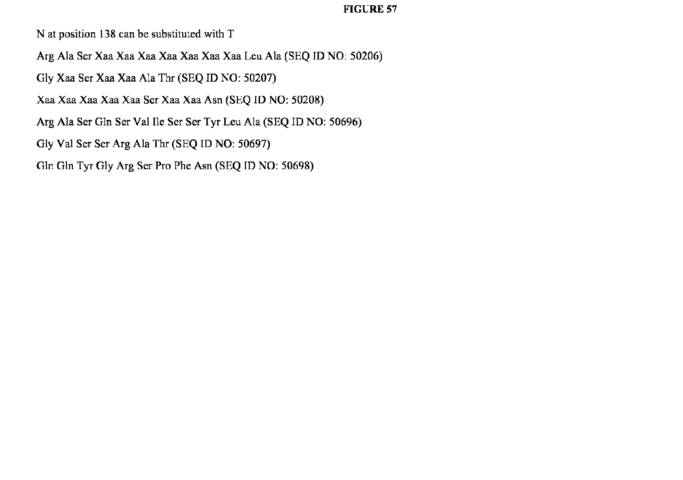

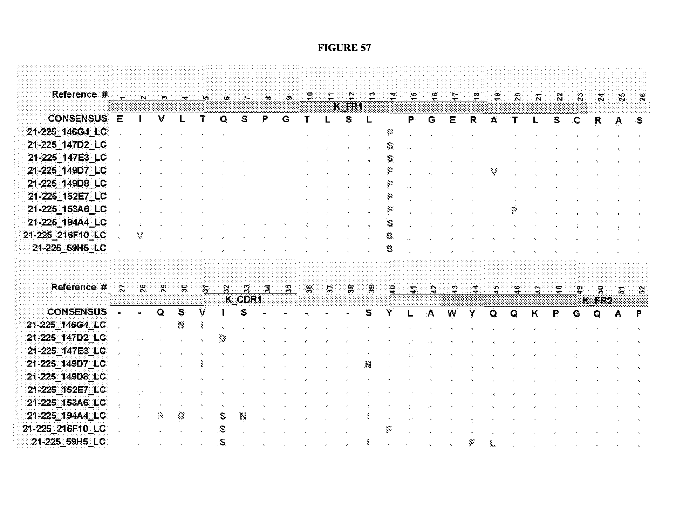

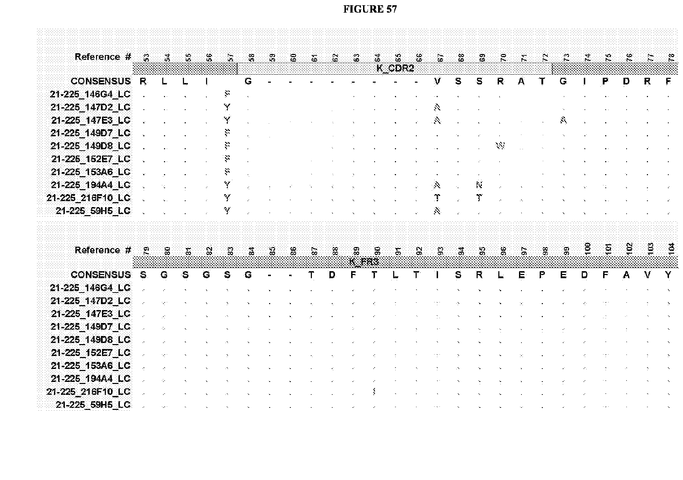

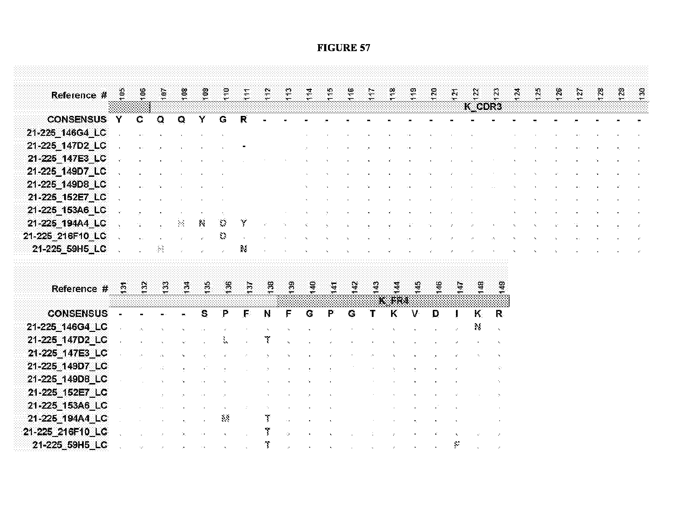

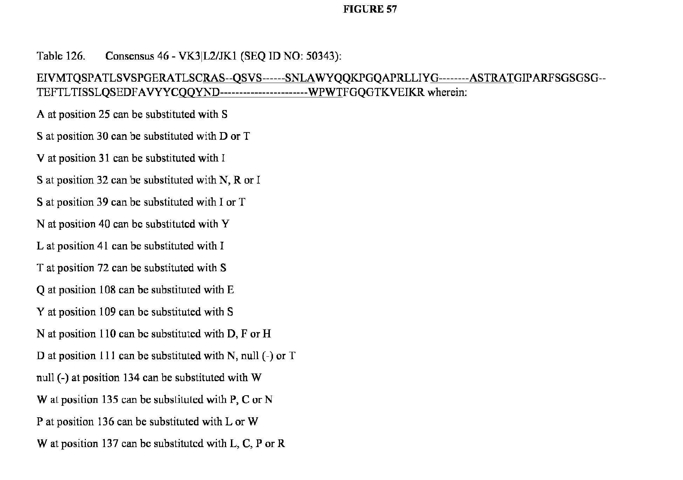

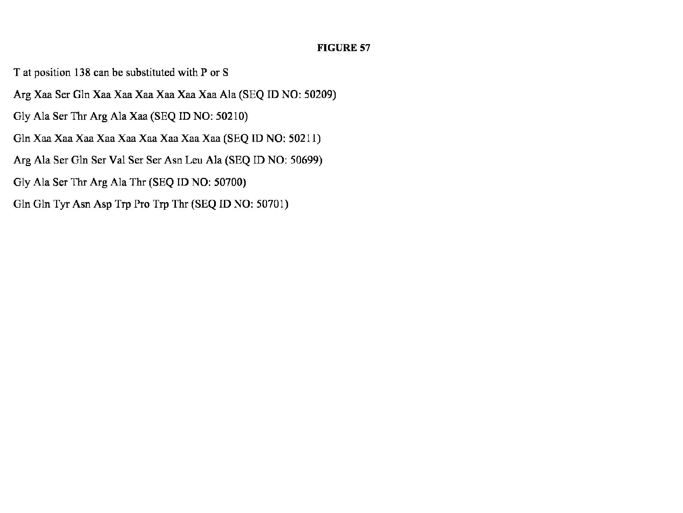

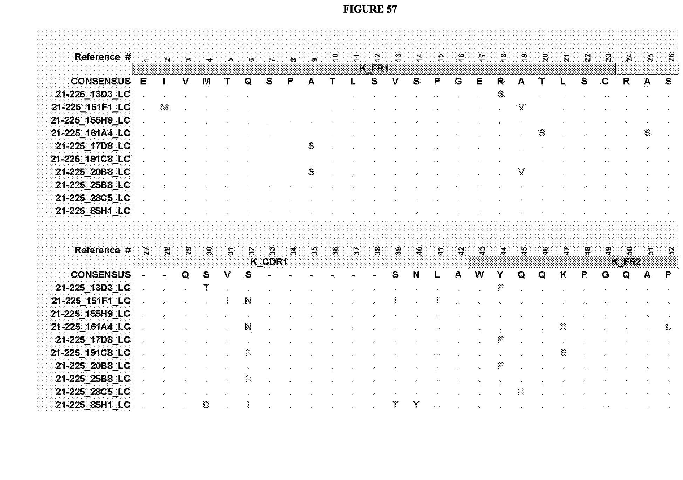

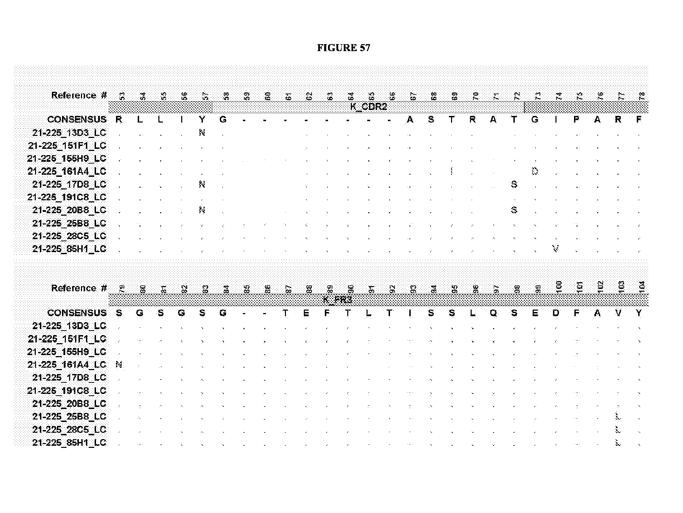

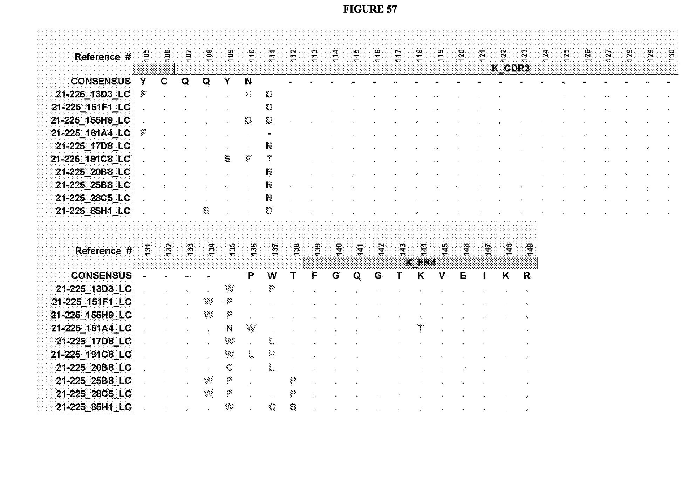

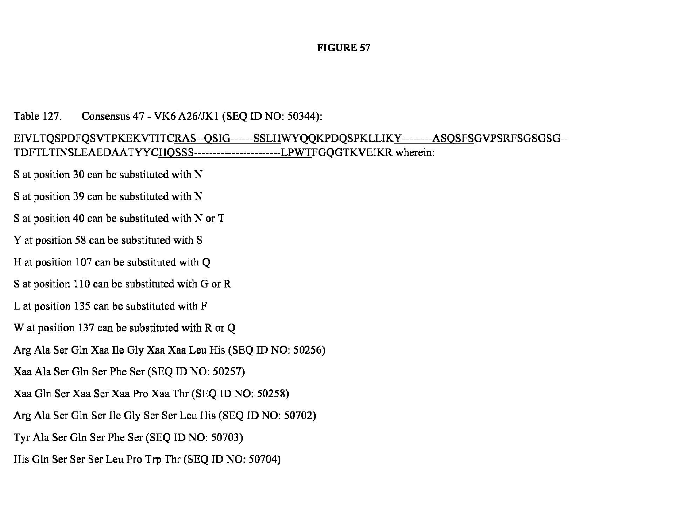

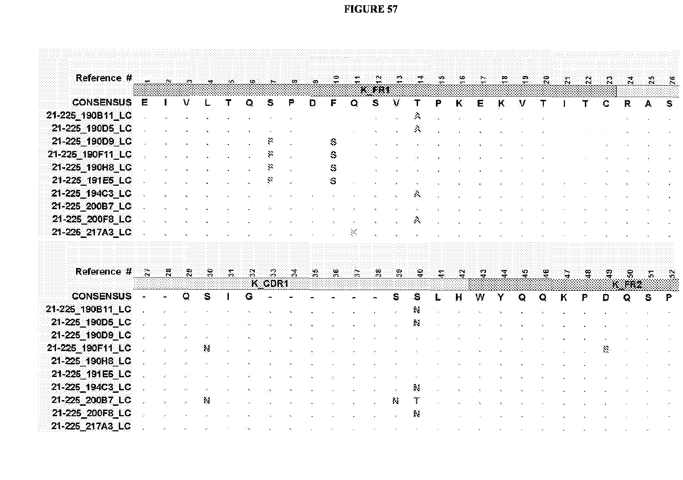

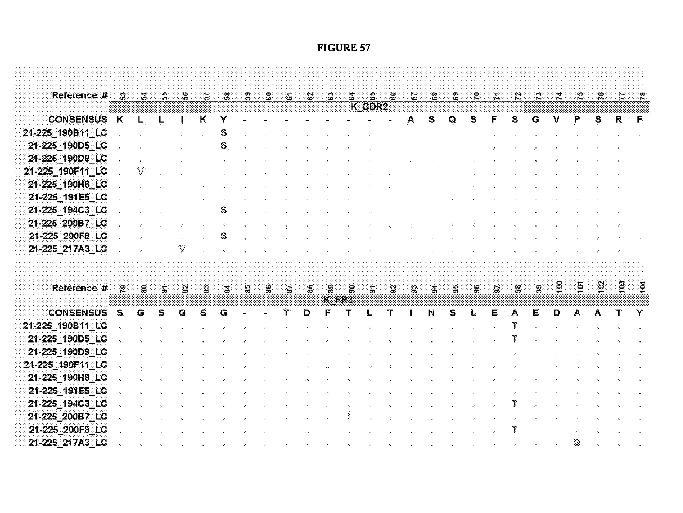

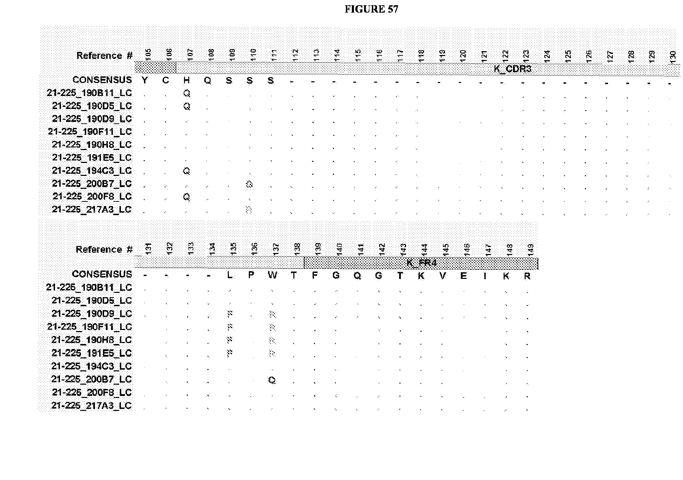

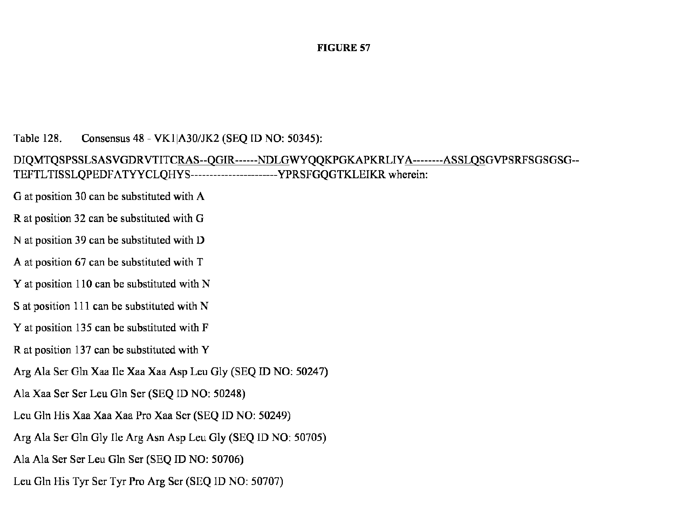

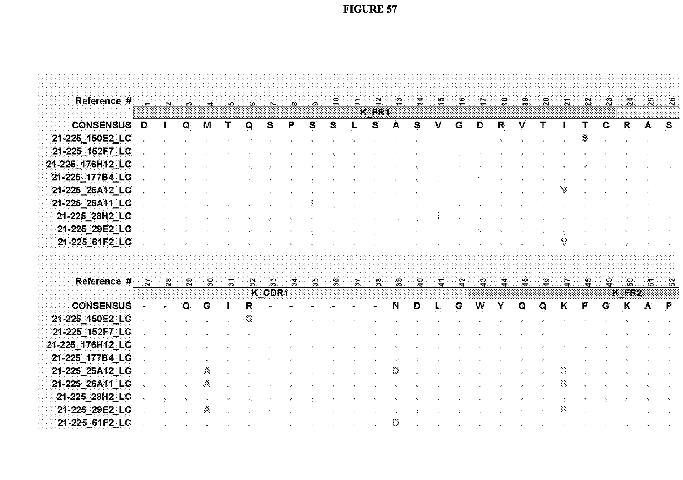

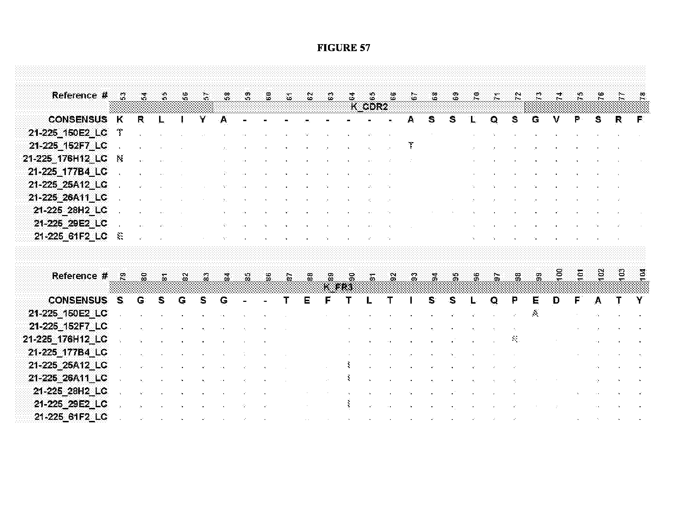

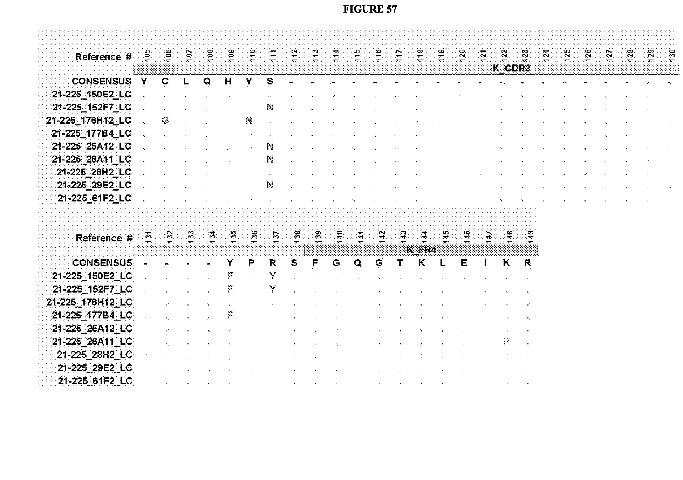

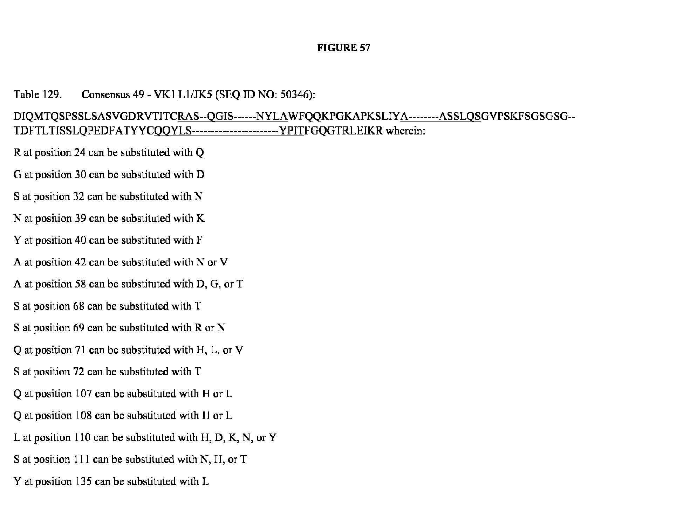

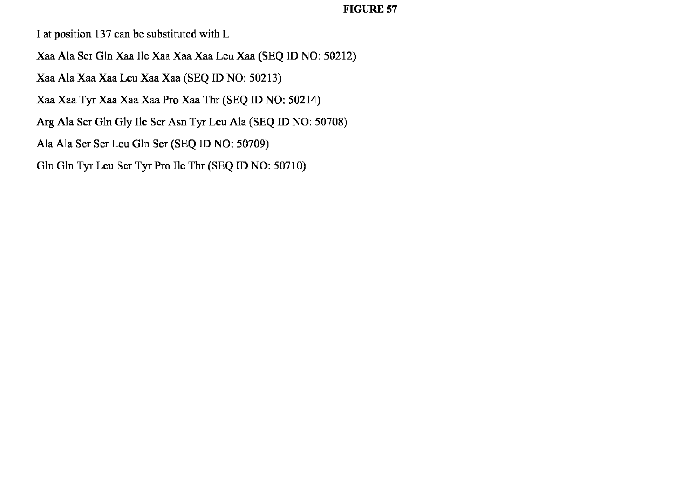

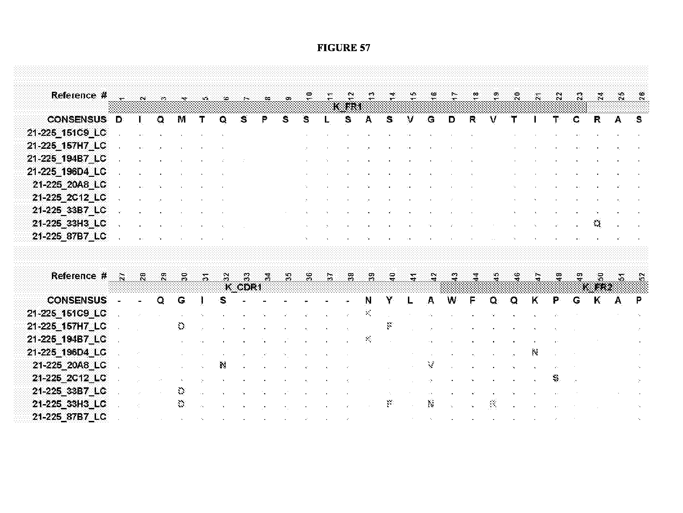

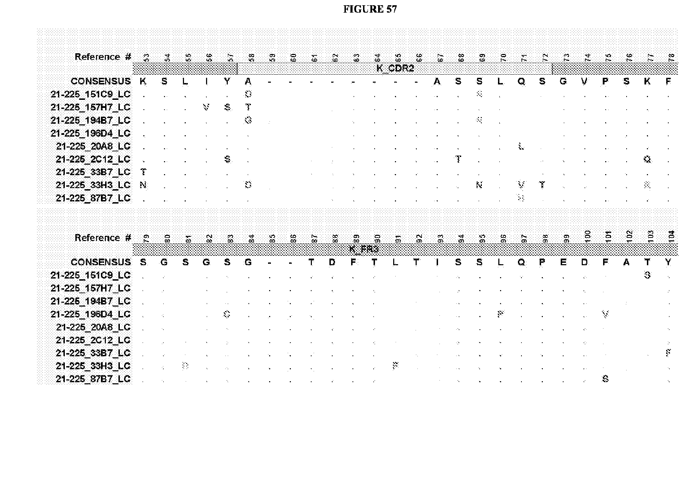

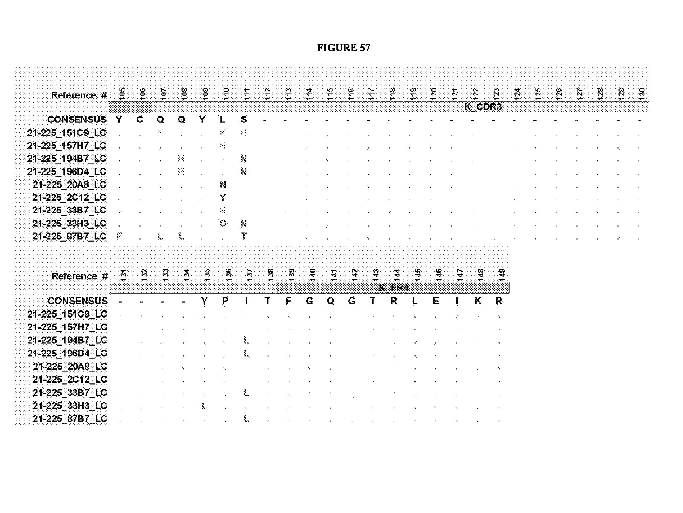

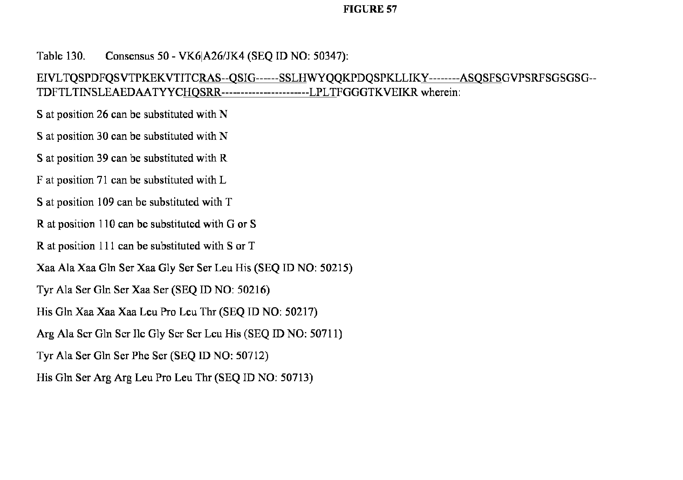

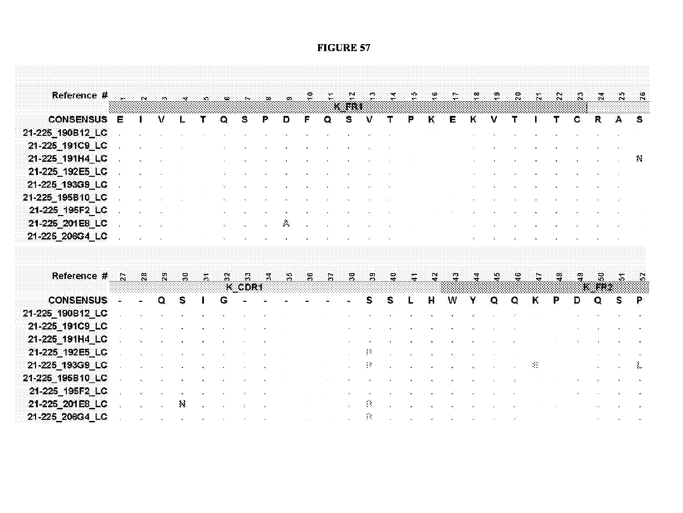

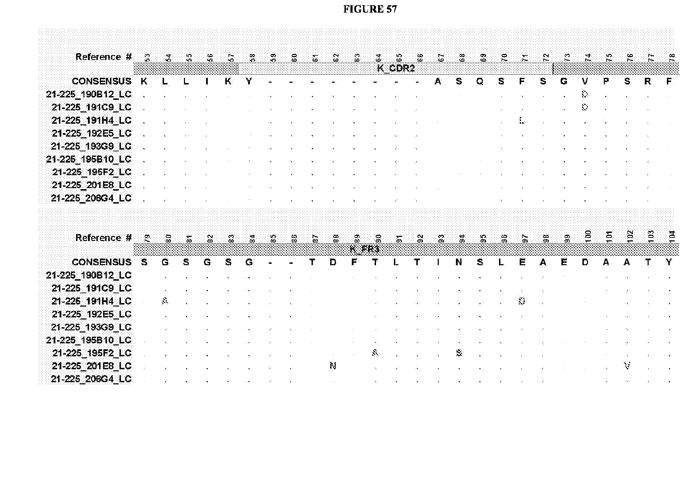

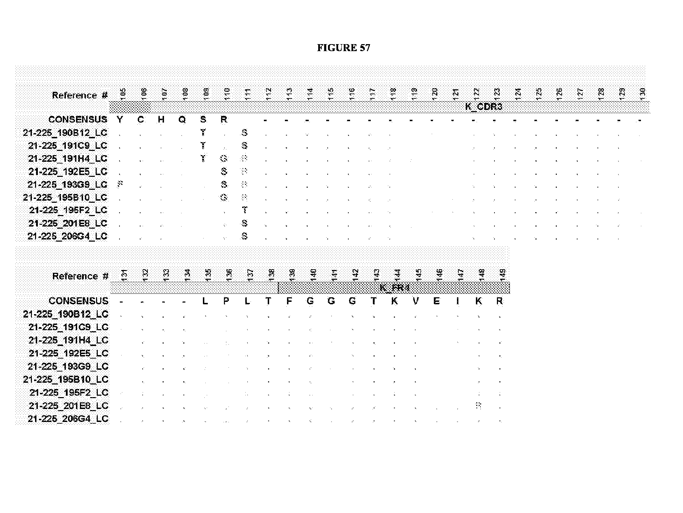

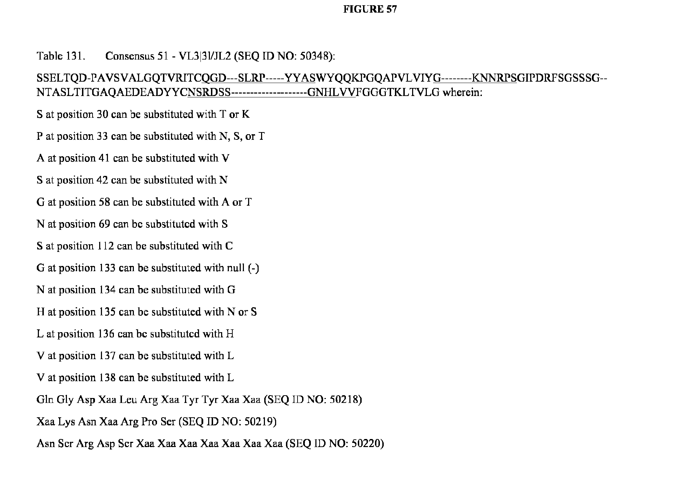

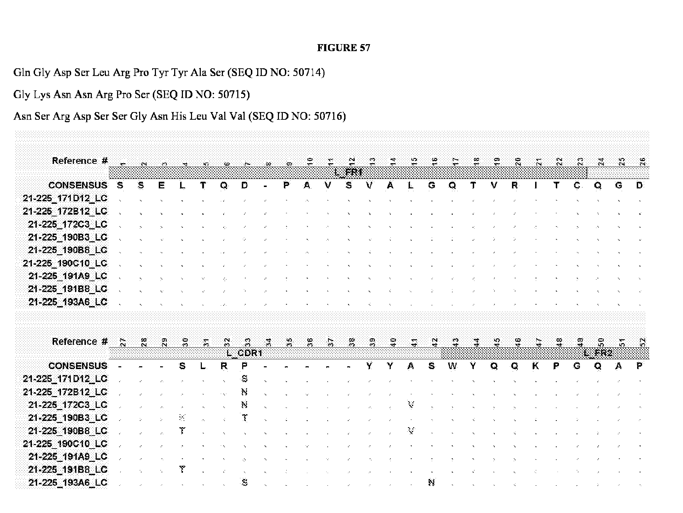

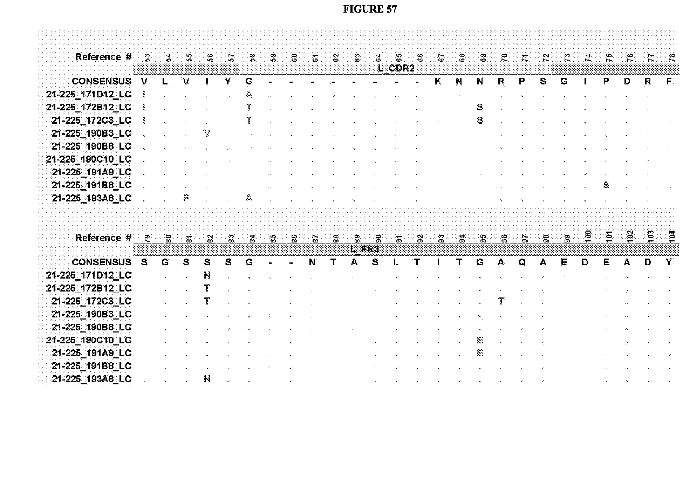

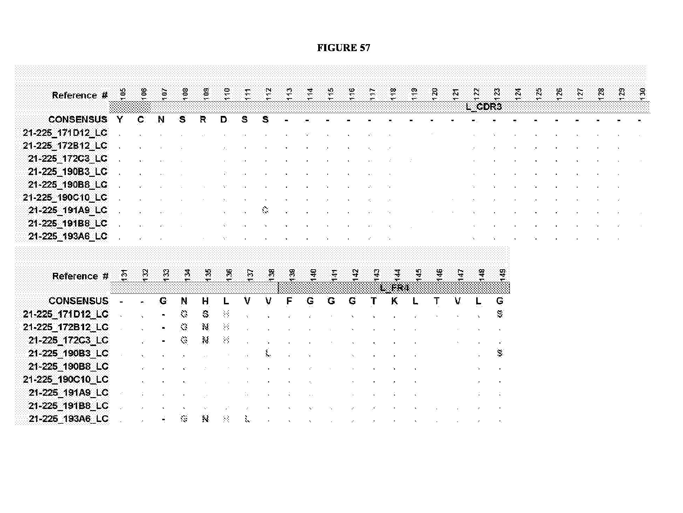

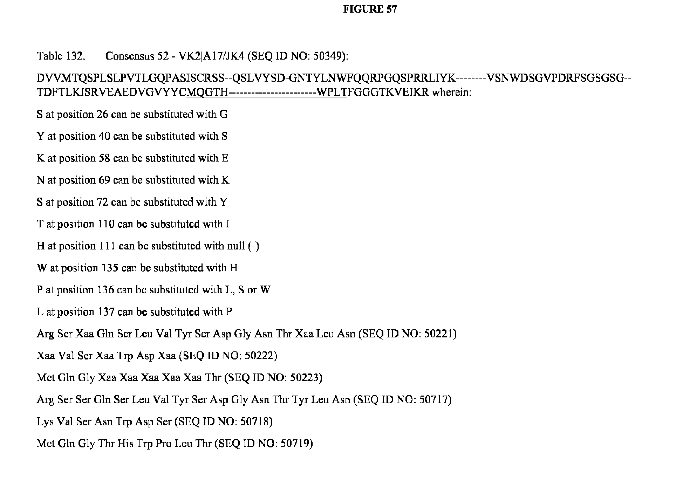

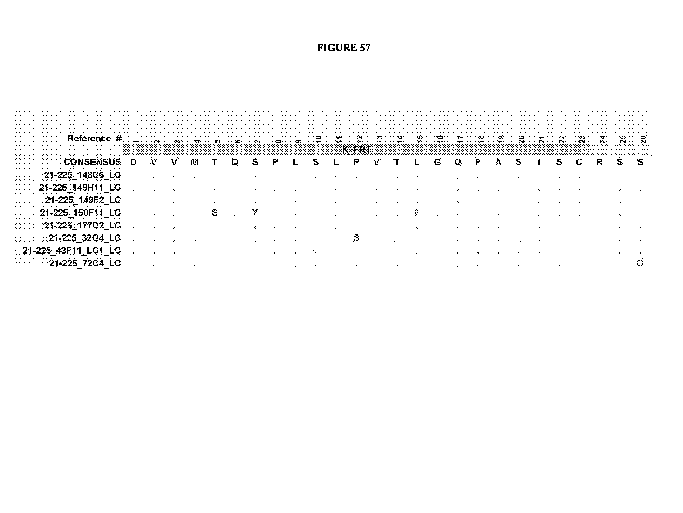

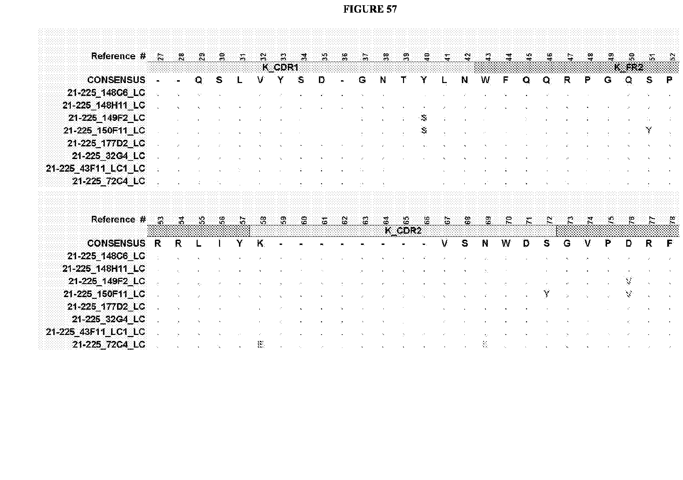

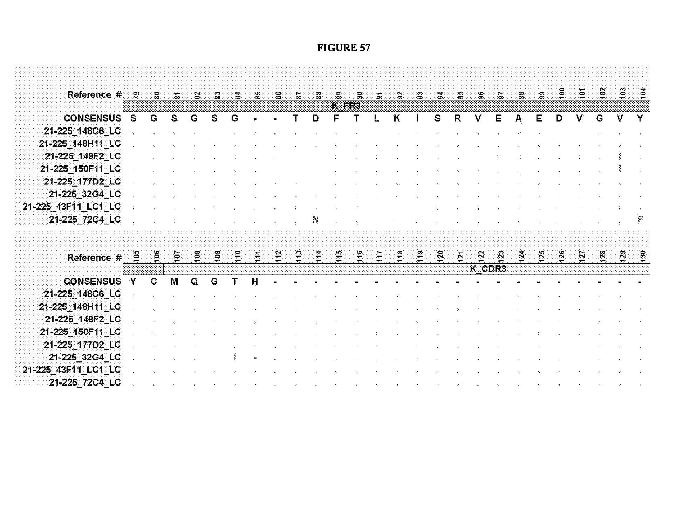

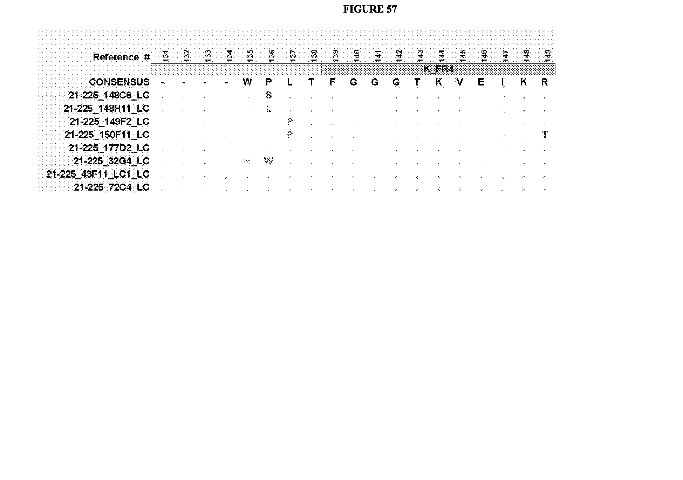

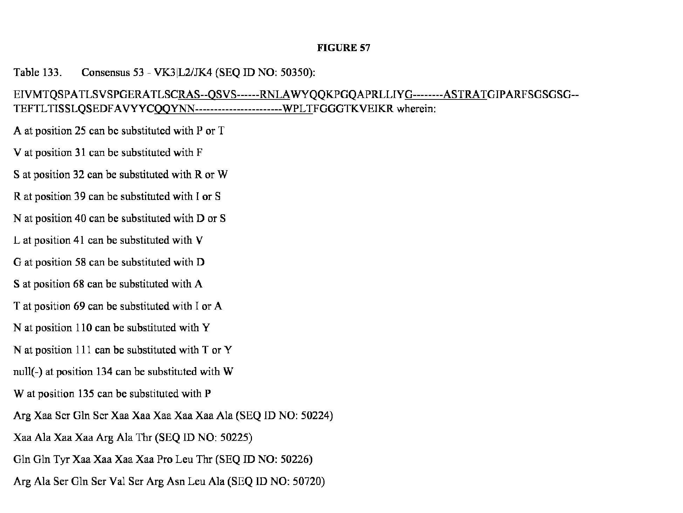

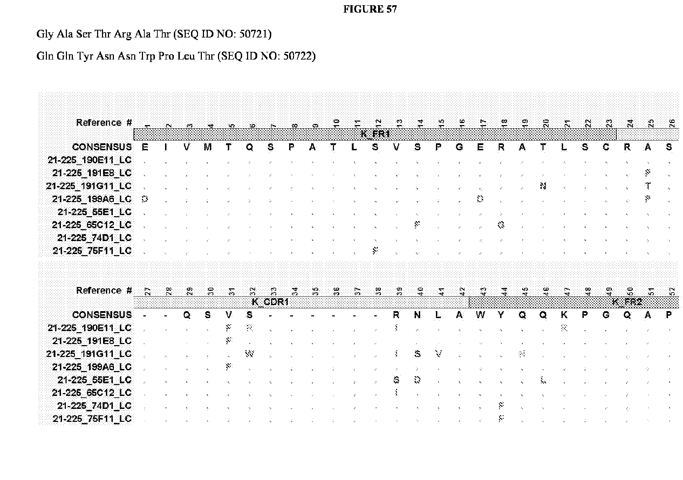

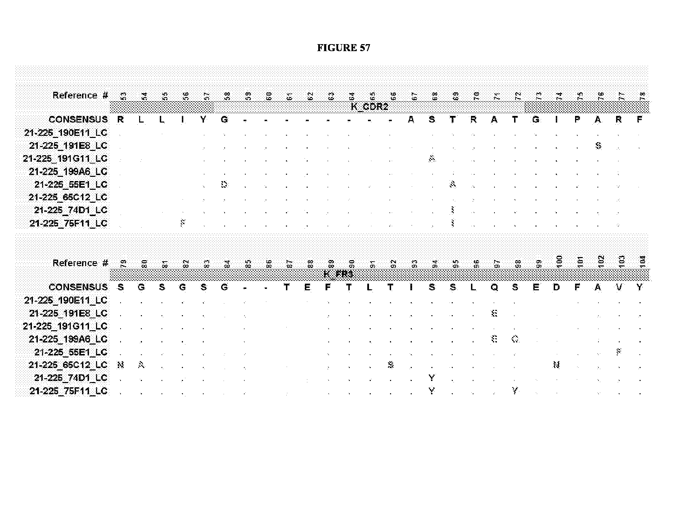

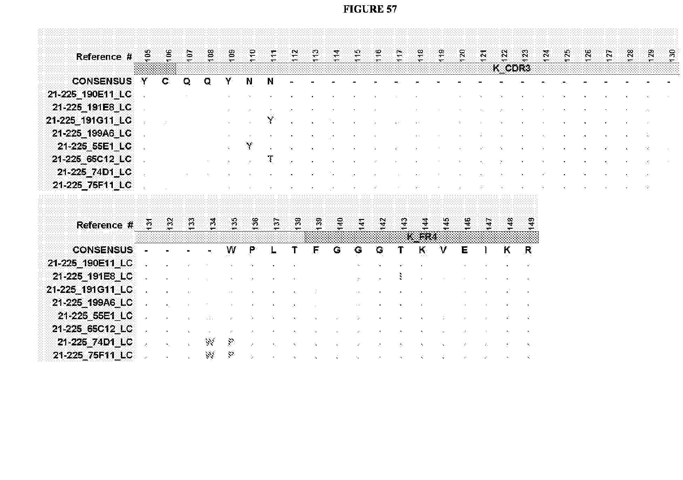

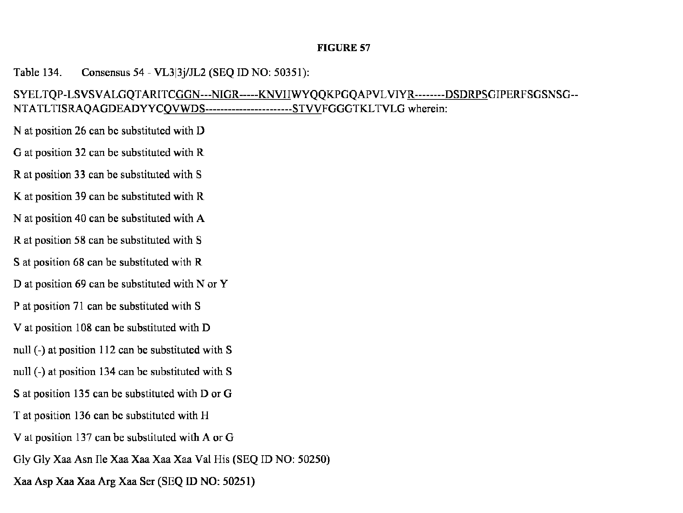

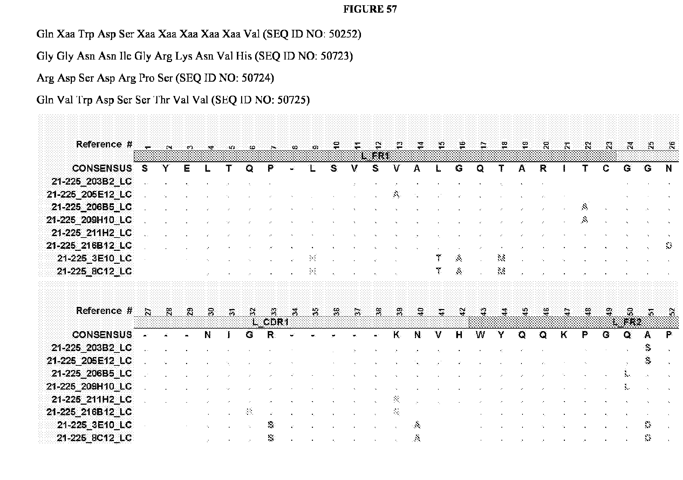

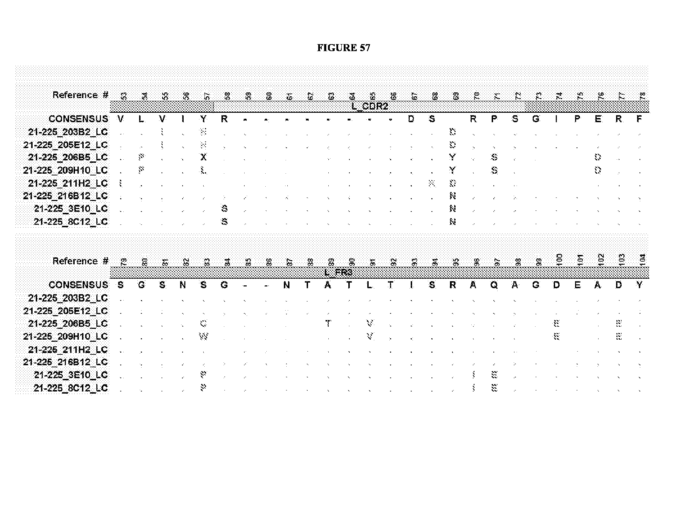

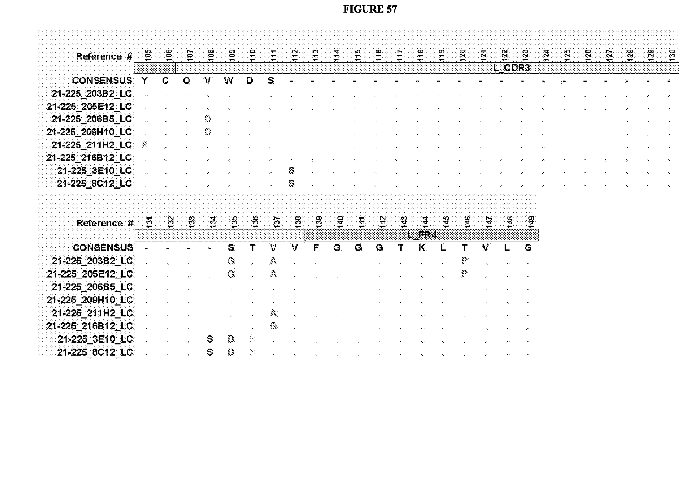

In some aspects, the invention provides an isolated antigen binding protein, wherein the antigen binding protein specifically binds human ASGR-1 and comprises a heavy chain variable domain having at least 90% identity to any of the VH domain amino acid sequences set forth in Tables 19A, as depicted in Figure 55 or in Tables 21-34 as depicted in Figure 56 or in Tables 49-95 as depicted in Figure 56. In some aspects, the invention provides an isolated antigen binding protein, wherein the antigen binding protein specifically binds human ASGR-1 and comprises a light chain variable domain having at least 90% identity to any of the VL domain amino acid sequences set forth in Table 20A, as depicted in Figure 55, or in Tables 35-48, as depicted in Figure 56 or in Tables 96-134 as depicted in Figure 57. In some embodiments, the antigen binding protein comprises a heavy chain variable domain having at least 90% identity to any of the VH domain amino acid sequences set forth in Tables 19A as depicted in Figure 55, or in Tables 21-34 as depicted in Figure 56 or in Tables 49-95 as depicted in Figure 57, and a light chain variable domain having at least 90% identity to any of the VL domain amino acid sequences set forth in Table 20A as depicted in Figure 55 or in Tables 35-48 as depicted in Figure 56 or in Tables 96-134 as depicted in Figure 57. In some embodiments, the antigen binding protein comprises a heavy chain variable domain having any of the VH domain amino acid sequences set forth in Tables 19A as depicted in Figure 55, or in Tables 21-34 as depicted in Figure 56 or in Tables 49-95 as depicted in Figure 57, and a light chain variable domain having any of the VL domain amino acid sequences set forth in Table 20A as depicted in Figure 55 or in Tables 35-48 as depicted in Figure 56 or inTables 96-134 as depicted in Figure 57.

In some aspects, the invention provides an antigen binding protein that specifically binds to human ASGR-1 at an epitope that is bound by any of the antigen binding proteins disclosed herein. In some embodiments, the invention provides an isolated antigen binding protein that specifically binds to human ASGR-1 at an epitope that is bound by at least one of the antigen binding proteins set forth in Tables 2-7. In some embodiments, the invention provides an isolated antigen binding protein that specifically binds to human ASGR-1 at an epitope that is bound by at least one of the antigen binding proteins set forth in Table A. In some embodiments, the invention provides an isolated antigen binding protein that specifically binds to human ASGR-1 at an epitope that is bound by at least one of the antigen binding proteins set forth in Table B. In some embodiments, the invention provides an isolated antigen binding protein that specifically binds to human ASGR-1 at an epitope that is bound by at least one of the antigen binding proteins set forth in Table C. In some embodiments, the invention provides an isolated antigen binding protein that specifically binds to human ASGR-1 at an epitope that is bound by at least one of the antigen binding proteins set forth in Table 6.

In some aspects, the invention provides an isolated antigen binding protein that competes for binding to human ASGR-1 with any of the antigen binding proteins disclosed herein. In some embodiments, the invention provides an isolated antigen binding protein that competes for binding with any of the antigen binding proteins set forth in Tables 2-7. In some embodiments, the invention provides an isolated antigen binding protein that competes for binding with any of the antigen binding proteins set forth in Table A. In some embodiments, the invention provides an isolated antigen binding protein that competes for binding with any of the antigen binding proteins set forth in Table B. In still some embodiments, the invention provides an isolated antigen binding protein that competes for binding with any of the antigen binding proteins set forth in Table C. In yet another embodiment, the invention provides an isolated antigen binding protein that competes for binding with any of the antigen binding proteins set forth in Table 6.

In some aspects, the invention provides an isolated antigen binding protein that binds to human ASGR-1 within the carbohydrate recognition domain ("CRD") (also known as the carbohydrate binding domain or "CBD") and inhibits human ASGR-1 binding to ligand. In some embodiments, the antigen binding protein binds to human ASGR-1 within residues 148-291, or 149-291, or 150-291, or 151-291, or 152-291, or 153-291, or 154-291, or 155-291 of SEQ ID NO:5. In some embodiments, the invention comprises an isolated antigen binding protein that binds to human ASGR-1 CBD within Helix $\alpha$-1. In some embodiments, the invention comprises an isolated antigen binding protein that binds to human ASGR-1 within residues 174-186 of SEQ ID NO:5. In some embodiments, the invention comprises an isolated antigen binding protein that binds to human ASGR-1 CBD within Helix $\alpha$-2. In some embodiments, the invention comprises an isolated antigen binding protein that binds to human ASGR-1 CBD within residues 194-206 of SEQ ID NO:5. In some embodiments, the invention comprises an isolated antigen binding protein that binds to human ASGR-1 within residues 237-273 or residues 240-267 of SEQ ID NO:5. In some embodiments, the antigen binding protein binds to ASGR-1 having an amino acid sequence that is at least 90% identical to SEQ ID NO:5. In some embodiments, the antigen binding protein is an antibody.

In some aspects, the invention provides an isolated antigen binding protein or an antibody that binds to human ASGR-1 and inhibits human ASGR-1 function. In some embodiments, the isolated antigen binding protein or an antibody binds to human ASGR-1 and inhibits human ASGR-1 from binding to a ligand. In some embodiments, the antigen binding protein or antibody or a paratope in an antibody binds to human ASGR-1 at an epitope comprising at least one of the following amino acid residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, Y273, P238, E239, P241, D243, Y245, G246, H247, G252, C255, F258, D260, R263, W264, V268, R271, D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, W275, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, Y273, D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, R274, N157, R170, S171, G172, Q202, H203, H204, I205, G206, P207, V208, N209, T210, D260, R271, P272, Y273, R274 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, Y273, P238, E239, P241, D243, Y245, G246, H247, G252, C255, F258, D260, R263, W264, V268, R271, D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, Y273, D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, or Y273 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, or Y273 (SEQ ID NO:5). In some embodiments, the antigen binding protein or antibody or paratope in an antibody binds to human ASGR-1 at an epitope comprising at least one of the following residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, : H161, E162, V191, V192, E197, Q198, D216, G219, K222, W223, D225, R263, W264, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, W275, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments, the antigen binding protein or antibody or paratope in an antibody binds to human ASGR-1 at an epitope comprising at least one of the following residues: D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, , R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, R274, N157, R170, S171, G172, Q202, H203, H204, I205, G206, P207, V208, N209, T210, D260, R271, P272, Y273, or R274 (SEQ ID NO:5). In some embodiments, the antigen binding protein or antibody or paratope in an antibody binds to human ASGR-1 at an epitope comprising at least one of the following residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments, the antigen binding protein or antibody or paratope in an antibody binds to human ASGR-1 at an epitope comprising at least one of the following residues: D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5). In some embodiments when the antigen binding protein or antibody or paratope in an antibody binds to human ASGR-1 at an epitope comprising at least one of the following residues:, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments, the antigen binding protein or antibody or paratope in an antibody binds to human ASGR-1 at an epitope comprising at least one of the following residues: H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, N157, R170, S171, G172, Q202, H203, H204, I205, G206, P207, V208, N209, T210, D260, R271, P272, Y273, or R274 (SEQ ID NO:5). In some embodiments, the antigen binding protein or antibody or a paratope in an antibody binds to human ASGR-1 at an epitope comprising at least one of the following amino acid residues: H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, or W264 (SEQ ID NO:5). In some embodiments, the antigen binding protein or antibody or a paratope in an antibody binds to human ASGR-1 at an epitope comprising at least one of the following amino acid residues: H161, E162, W195, E196, Q198, K199, F200, Q202, H203, H204, G232, F233, K234, N235, W236, R237, P238, D261, G262, or R263 (SEQ ID NO:5). In some embodiments, the antigen binding protein or antibody or a paratope in an antibody binds to human ASGR1 at an epitope comprising at least one of the following amino acid residues: W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, or T279 (SEQ ID NO:5). In some embodiments, the antigen binding protein or antibody or a paratope in an antibody binds to human ASGR-1 at an epitope comprising at least one of the following amino acid residues: W167, S171, G172, K173, A174, A176, D177, N180, Y181, R183, L184, E185, D186, Q270, P272, or W275 (SEQ ID NO:5). In some embodiments, the antigen binding protein or antibody or a paratope in an antibody binds to human ASGR-1 at an epitope comprising at least one of the following amino acid residues: R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, or W264 (SEQ ID NO:5). In some embodiments, the antigen binding protein or antibody or a paratope in an antibody binds to human ASGR-1 at an epitope comprising at least one of the following amino acid residues: R170, W195, E196, K199, Q202, H203, H204, I205, G206, P207, V208, F233, K234, N235, W236, P238, D260, D261, G262, R263, or R274 (SEQ ID NO:5). In some embodiments, the antigen binding protein or antibody or a paratope in an antibody binds to human ASGR-1 at an epitope comprising at least one of the following amino acid residues: H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, or W264 (SEQ ID NO:5). In some embodiments, the antigen binding protein or antibody or a paratope in an antibody binds to human ASGR-1 at an epitope comprising at least one of the following amino acid residues: H161, S194, W195, E196, Q198, K199, F200, Q202, H203, F233, K234, N235, W236, R237, P238, or R263 (SEQ ID NO:5). In some embodiments, the antigen binding protein or antibody or a paratope in an antibody binds to human ASGR-1 at an epitope comprising at least one of the following amino acid residues: H161, E162, T193, S194, W195, E196, K199, Q202, T231, G232, F233, K234, N235, P238, D261, R263, R163, V192, E197, Q198, H203, P207, D228, E230, W236, R237, D260, G262, or W264 (SEQ ID NO:5). In some embodiments, the antigen binding protein or antibody or a paratope in an antibody binds to human ASGR-1 at an epitope comprising at least one of the following amino acid residues: H161, E162, T193, S194, W195, E196, K199, Q202, T231, G232, F233, K234, N235, P238, D261, or R263 (SEQ ID NO:5). In some embodiments, the antigen binding protein or antibody or a paratope in an antibody binds to human ASGR-1 at an epitope comprising at least one of the following amino acid residues: T193, S194, W195, E196, P220, W221, G226, T227, D228, Y229, E230, T231, G232, F233, K234, N235, W236, R237, P238, E239, G252, : H161, E162, V191, V192, E197, Q198, D216, G219, K222, W223, D225, R263, or W264 (SEQ ID NO:5). In some embodiments, the antigen binding protein or antibody or a paratope in an antibody binds to human ASGR-1 at an epitope comprising at least one of the following amino acid residues: T193, S194, W195, E196, P220, W221, G226, T227, D228, Y229, E230, T231, G232, F233, K234, N235, W236, R237, P238, E239, or G252 (SEQ ID NO:5). In some embodiments, the antigen binding protein or antibody or a paratope in an antibody binds to human ASGR-1 at an epitope comprising at least one of the following amino acid residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, or C269 (SEQ ID NO:5). In some embodiments, the antigen binding protein or antibody or a paratope in an antibody binds to human ASGR-1 at an epitope comprising at least one of the following amino acid residues: D216, Q217, N218, G219, P220, W221, Y229, E230, K234, W236, E239, Q240, P241, D242, D243, W244, Y245, G246, L249, G250, G251, G252, D254, or Q270 (SEQ ID NO:5). In some embodiments, the antigen binding protein or antibody or a paratope in an antibody binds to human ASGR-1 at an epitope comprising at least one of the following amino acid residues: W195, N209, N235, R237, P238, E239, Q240, D242, H257, T259, D260, D261, R263, N265, D267, R271, Y273, Q198, Q202, P207, V208, F233, W236, D243, E253, F258, G262, W264, or D266 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: W195, N209, N235, R237, P238, E239, Q240, D242, H257, T259, D260, D261, R263, N265, D267, R271, or Y273 (SEQ ID NO:5). In some embodiments, the antigen binding protein or antibody or a paratope in an antibody binds to human ASGR-1 at an epitope comprising at least one of the following amino acid residues: N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments, the antigen binding protein or antibody or a paratope in an antibody binds to human ASGR-1 at an epitope comprising at least one of the following amino acid residues: N157, R170, S171, G172, Q202, H203, H204, I205, G206, P207, V208, N209, T210, D260, R271, P272, Y273, or R274 (SEQ ID NO:5). In some embodiments, the antigen binding protein or antibody or a paratope in an antibody binds to human ASGR-1 at an epitope comprising at least one of the following amino acid residues: R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, or W275 (SEQ ID NO:5). In some embodiments, the antigen binding protein or antibody or a paratope in an antibody binds to human ASGR-1 at an epitope comprising at least one of the following amino acid residues: R170, S171, G172, A174, H204, I205, G206, P207, V208, N209, H257, D260, N265, D267, Q270, R271, P272, Y273, or R274 (SEQ ID NO:5). In some embodiments, the antigen binding protein or antibody or a paratope in an antibody binds to human ASGR-1 at an epitope comprising at least one of the following amino acid residues: R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments, the antigen binding protein or antibody or a paratope in an antibody binds to human ASGR-1 at an epitope comprising at least one of the following amino acid residues: R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5).

In some aspects, the invention provides an isolated antigen binding protein or an antibody or a paratope in an antibody that specifically binds to human ASGR-1 and inhibits human ASGR-1 function. In some embodiments, the isolated antigen binding protein or an antibody or a paratope in an antibody specifically binds to human ASGR-1 and inhibits human ASGR-1 from binding to a ligand. In some embodiments, the antigen binding protein or antibody or a paratope in an antibody specifically binds to human ASGR-1 within residues 148-291 of SEQ ID NO:5. In some embodiments when the antigen binding protein or antibody or a paratope in an antibody is bound to human ASGR-1, the antigen binding protein or antibody or a paratope in an antibody is positioned 8 angstroms or less from at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, Y273, P238, E239, P241, D243, Y245, G246, H247, G252, C255, F258, D260, R263, W264, V268, R271, D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, W275, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, Y273, D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, R274, N157, R170, S171, G172, Q202, H203, H204, I205, G206, P207, V208, N209, T210, D260, R271, P272, Y273, or R274 (SEQ ID NO:5).

In some embodiments when the antigen binding protein or antibody or a paratope in an antibody is bound to human ASGR-1, the antigen binding protein or antibody or a paratope in an antibody is positioned 8 angstroms or less from at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, Y273, P238, E239, P241, D243, Y245, G246, H247, G252, C255, F258, D260, R263, W264, V268, R271, D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, Y273, D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, or Y273 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, or Y273 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, or Y273 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: Q240, D242, W244, E253, N265, D266, or D267 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: R237, Q240, D242, W244, E253, N265, D266, D267, N209, P238, E239, P241, D243, Y245, G246, H247, G252, C255, H257, T259, V268, R271, or Y273 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: R237, Q240, D242, W244, E253, N265, D266, or D267 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: N209, R237, Q240, D242, W244, E253, H257, T259, N265, D266, D267, Y273, P238, E239, P241, D243, Y245, G246, H247, G252, C255, F258, D260, R263, W264, V268, or R271 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: N209, R237, Q240, D242, W244, E253, H257, T259, N265, D266, D267, or Y273 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, or C269 (SEQ ID NO:5). In some embodiments when the antigen binding protein or antibody is bound to human ASGR-1, the antigen binding protein or antibody or a paratope in an antibody is positioned 5 angstroms or less from at least one of the following residues: D216, Q217, N218, G219, P220, W221, Y229, E230, K234, W236, E239, Q240, P241, D242, D243, W244, Y245, G246, L249, G250, G251, G252, D254, or Q270 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: W195, N209, N235, R237, P238, E239, Q240, D242, H257, T259, D260, D261, R263, N265, D267, R271, Y273, Q198, Q202, P207, V208, F233, W236, D243, E253, F258, G262, W264, or D266 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: W195, N209, N235, R237, P238, E239, Q240, D242, H257, T259, D260, D261, R263, N265, D267, R271, or Y273 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: R170, S171, G172, A174, H204, I205, G206, P207, V208, N209, H257, D260, N265, D267, Q270, R271, P272, Y273, or R274 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, W275, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues of human ASGR-1 (SEQ ID NO:5): D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, R274, N157, R170, S171, G172, Q202, H203, H204, I205, G206, P207, V208, N209, T210, D260, R271, P272, Y273, or R274 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, N157, R170, S171, G172, Q202, H203, H204, I205, G206, P207, V208, N209, T210, D260, R271, P272, Y273, R274, V156, W158, V159, H161, W167, F168, S169, K173, (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, or W264 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues of human ASGR-1 (SEQ ID NO:5): H161, E162, W195, E196, Q198, K199, F200, Q202, H203, H204, G232, F233, K234, N235, W236, R237, P238, D261, G262, or R263 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, or T279 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: W167, S171, G172, K173, A174, A176, D177, N180, Y181, R183, L184, E185, D186, Q270, P272, or W275 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, or W264 (SEQ ID NO:5). In some embodiments when the antigen binding protein or antibody or or a paratope in an antibody is positioned 5 angstroms or less from at least one of the following residues: R170, W195, E196, K199, Q202, H203, H204, I205, G206, P207, V208, F233, K234, N235, W236, P238, D260, D261, G262, R263, or R274 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, or W264 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: H161, S194, W195, E196, Q198, K199, F200, Q202, H203, F233, K234, N235, W236, R237, P238, or R263 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: H161, E162, T193, S194, W195, E196, K199, Q202, T231, G232, F233, K234, N235, P238, D261, R263, R163, V192, E197, Q198, H203, P207, D228, E230, W236, R237, D260, G262, or W264 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: H161, E162, T193, S194, W195, E196, K199, Q202, T231, G232, F233, K234, N235, P238, D261, or R263 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: T193, S194, W195, E196, P220, W221, G226, T227, D228, Y229, E230, T231, G232, F233, K234, N235, W236, R237, P238, E239, G252, : H161, E162, V191, V192, E197, Q198, D216, G219, K222, W223, D225, R263, or W264 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: T193, S194, W195, E196, P220, W221, G226, T227, D228, Y229, E230, T231, G232, F233, K234, N235, W236, R237, P238, E239, or G252 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270 or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: N157, R170, S171, G172, Q202, H203, H204, I205, G206, P207, V208, N209, T210, D260, R271, P272, Y273 or R274 (SEQ ID NO:5).

In some aspects, the invention comprises an isolated antigen binding protein or antibody that specifically binds to human ASGR-1 and inhibits human ASGR-1 function. In some embodiments, the isolated antigen binding protein or antibody that specifically binds to human ASGR-1 inhibits binding of human ASGR-1 binding to a ligand. In some embodiments, the antigen binding protein or antibody specifically binds to human ASGR-1 at a location that overlaps with a location where a ligand binds to human ASGR-1. In some embodiments, the location where a ligand binds to ASGR-1 includes at least one amino acid residue selected from the group consisting of N209, R237, Q240, D242, W244, E253, H257, T259, N265, D266, D267, Y273, P238, E239, P241, D243, Y245, G246, H247, G252, C255, F258, D260, R263, W264, V268, or R271 (SEQ ID NO:5). In some embodiments, an isolated antigen binding protein or an antibody specifically binds to human ASGR-1 at a location that overlaps with a location that a ligand binds to ASGR-1. In some embodiments, the location that a ligand binds to human ASGR-1 includes at least one amino acid residue selected from the group consisting of: N209, R237, Q240, D242, W244, E253, H257, T259, N265, D266, D267, and Y273 (SEQ ID NO:5).

In some aspects, the invention comprises an isolated antigen binding protein that binds to human ASGR-1 and inhibits human ASGR, ASGR-1 and/or ASGR-2 function, wherein the antigen binding protein does not bind to a variant ASGR-1 protein, and wherein said variant ASGR-1 protein comprises a single mutation of a residue selected the group consisting of R170, S171, G172, R183, L184, W195, E196, K199, H203, H204, P207, V208, N209, H215, D216, P220, D225, D228, R237, P238, E239, P241, D242, D243, Y245, G246, H247, G248, L249, G251, E253, T259, D260, R263, N265, Q270, R271, P272, R274, and E280 as shown in SEQ ID NO:5. In some embodiments, an isolated antigen binding protein or an antibody is contemplated. An antigen binding protein "does not bind" to a variant ASGR-1 protein when the measured reduction in antibody binding signal to a variant ASGR-1 protein (compared to that determined for binding to wild type ASGR-1) is statistically significant as measured by any number of methods known to one skilled in the art, such as the method described in Example 7E below. In some embodiments, the variant ASGR-1 protein comprises a single mutation of a residue at a position selected from the group consisting of: W195, E196, K199, H203, H204, P207, P220, G251, and R263 as shown in SEQ ID NO:5. In some embodiments, the single mutation is selected from the group consisting of H203, H204, P220, and G251. In some embodiments, the single mutation is selected from the group consisting of W195, E196 and K199. In some embodiments, the single mutation is selected from the group consisting of W195, E196 and H204. In some embodiments, the single mutation is selected from the group consisting W195, K199, and R263. In some embodiments, the single mutation is selected from the group consisting of W195 and E196. In some embodiments, the single mutation is selected from the group consisting of W195 and K199. In some embodiments, the single mutation is selected from the group consisting of W195 or P207. In some embodiments, the single mutation is selected from the group consisting of W195 and R263. In some embodiments, the single mutation is selected from the group consisting of H203 and H204. In some embodiments, the single mutation is selected from the group consisting of K199 and R263. In some embodiments, the single mutation is a mutation of residue W195. In some embodiments, the variant ASGR-1 protein comprises a single mutation of a residue selected the group consisting of R170, S171, R183, L184, H215, P220, P238, G246, H247, G248, G251, and N265 as shown in SEQ ID NO:5. In some embodiments, the single mutation is selected from the group consisting of R183, L184, H215, P220, G246, G248, G251, and N265. In some embodiments, the single mutation is selected from the group consisting of L184, P220, P238, H247, and G251. In some embodiments, the single mutation is selected from the group consisting of R170, S171, and L184. In some embodiments, the single mutation is a mutation of residue R183. In some embodiments, the single mutation is a mutation of residue L184. In some embodiments, the variant ASGR-1 protein comprises a single mutation of a residue at a position selected from the group consisting of: P241, D242, D243, Y245, G251, E253 and D260 as shown in SEQ ID NO:5. In some embodiments, the single mutation is selected from the group consisting of P241, D243, Y245, G251, E253 and D260. In some embodiments, the single mutation is selected from the group consisting of P241, D243, and E253. In some embodiments, the single mutation is a mutation of residue D260. In some embodiments, the variant ASGR-1 protein comprises a single mutation of a residue at a position selected from the group consisting or comprising:_R170, R237, E239, P241, T259, D260, R263, and N265 as shown in SEQ ID NO:5. In some embodiments, the single mutation is selected from the group consisting of R237, D260 and R263. In some embodiments, the single mutation is selected from the group consisting of R237, T259, D260 and R263. In some embodiments, the single mutation is selected from the group consisting of R170, R237, P241, T259, D260, R263 and N265. In some embodiments, the single mutation is selected from the group consisting of R237, E239, P241, T259, D260, R263 and N265. In some embodiments, the variant ASGR-1 protein comprises a single mutation of a residue at a position selected from the group consisting or comprising: R170, S171, G172, E196, H204, P207, V208, N209, H215, D216, D225, D228, P238, P241, D242, D243, H247, G248, L249, G251, D260, R263, N265, Q270, R271, P272, R274 and E280 as shown in SEQ ID NO:5. In some embodiments, the single mutation is selected from the group consisting of R170, S171, G172, E196, H204, P207, V208, N209, H215, D216, D225, D228, P238, P241, D242, D243, H247, G248, L249, G251, D260, R263, N265, Q270, R271, P272, R274 and E280 as shown in SEQ ID NO:5 . In some embodiments, the single mutation is selected from the group consisting ofR170, S171, G172, E196, H204, P207, H215, D216, D225, D228, D243, G248, L249, G251, D260, Q270, R271, P272, R274 and E280. In some embodiments, the single mutation is selected from the group consisting of G172, V208, R271, P272 and R274. In some embodiments, the single mutation is selected from the group consisting of G172, R271 and R274. In some embodiments, the single mutation is selected from the group consisting of G172, N209, and R271. In some embodiments, the single mutation is selected from the group consisting of R170, G172, V208, R271 and P272. In some embodiments, the single mutation is selected from the group consisting of G172, V208, P238, R271, P272 and R274. In some embodiments, the single mutation is selected from the group consisting of G172, P238, R271, P272 and R274. In some embodiments, the variant ASGR-1 protein comprises a single mutation of a residue at a position selected from the group consisting or comprising:_G172, P238, R271 and R274 as shown in SEQ ID NO:5. In some embodiments, the variant ASGR-1 protein comprises a single mutation of a residue at a position selected from the group consisting or comprising: R170, G172, V208 and R274 as shown in SEQ ID NO:5. In some embodiments, the variant ASGR-1 protein comprises a single mutation of a residue at a position selected from the group consisting or comprising: R170, R183, H215 and Q270 as shown in SEQ ID NO:5. In some embodiments, the variant ASGR-1 protein comprises a single mutation of a residue at a position selected from the group consisting or comprising: P241, T259, and N265 as shown in SEQ ID NO:5. In some embodiments, the variant ASGR-1 protein comprises a single mutation of a residue at a position selected from the group consisting or comprising: P207 and R263 as shown in SEQ ID NO:5. In some embodiments, the variant ASGR-1 protein comprises a single mutation of a residue at a position selected from the group consisting or comprising: G172, P241, D242, H247, L249, N265, R271 and P272 as shown in SEQ ID NO:5. In some embodiments, the antigen binding protein or antibody does not bind to two or more variant ASGR-1 proteins, wherein the variant ASGR-1 proteins comprise the single mutations of the group individually.

In some aspects, the invention comprises a vector comprising a nucleic acid molecule as described herein. In some embodiments, the invention comprises a host cell comprising a nucleic acid molecule as described herein.

In some aspects, the invention comprises a nucleic acid molecule encoding the antigen binding protein as described herein.

In some aspects, the invention comprises a pharmaceutical composition comprising at least one antigen binding protein described herein.

In some aspects, the invention provides a method of treating or preventing a cardiovascular disease comprising administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described herein. In some embodiments, the ASGR, ASGR-1 and/or ASGR-2 inhibitor is an interfering RNA (e.g., siRNA or shRNA) as described herein. In some embodiments, the relative risk reduction of a cardiovascular event is at least about 5%, at least about 10%, at least about 15%, at least about 20%, at least about 25%, at least about 30%, at least about 35%, at least about 40%, at least about 45%, at least about 50%, at least about 55%, at least about 60% in the patient.

In some aspects, the invention provides a method of decreasing the risk of acquiring coronary artery disease or having a myocardial infarction (MI) comprising administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described herein. In some embodiments, the ASGR, ASGR-1 and/or ASGR-2 inhibitor is an interfering RNA (e.g., siRNA or shRNA) as described herein. In some embodiments, the relative risk reduction of coronary artery disease or MI is at least about 5%, at least about 10%, at least about 15%, at least about 20%, at least about 25%, at least about 30%, at least about 35%, at least about 40%, at least about 45%, at least about 50%, at least about 55%, at least about 60% in the patient.

In other aspects, the invention provides a method of reducing blood LDL cholesterol levels in a patient comprising administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described herein. In some embodiments, the ASGR, ASGR-1 and/or ASGR-2 inhibitor is an interfering RNA (e.g., siRNA or shRNA) as described herein. In some embodiments, blood LDL cholesterol is reduced by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose level of blood LDL cholesterol in the patient.

In still other aspects, the invention provides a method of reducing non-HDL cholesterol levels in a patient comprising administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described herein. In some embodiments, the ASGR, ASGR-1 and/or ASGR-2 inhibitor is an interfering RNA (e.g., siRNA or shRNA) as described herein. In some embodiments, non-HDL cholesterol is reduced by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose level of non-HDL cholesterol in the patient.

In some aspects, the invention provides a method of increasing alkaline phosphatase ("ALP") levels in a patient comprising administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described herein. In some embodiments, the ASGR, ASGR-1 and/or ASGR-2 inhibitor is an interfering RNA (e.g., siRNA or shRNA) as described herein. In some embodiments, ALP levels are increased at least about 40%, at least about 45%, at least about 50%, at least about 55%, at least about 60%, at least about 65%, at least about 70%, at least about 75%, at least about 80%, at least about 85%, or at least about 90% as compared to a predose ALP level in the patient. In some embodiments, ALP levels are increased at least about 1.25x, 1.5x, 2x, 2.5x, 3x, 3.5x, 4x, 4.5x, and 5x over pretreatment.

In some aspects, the invention provides a method of antagonizing ASGR, ASGR-1 and/or ASGR-2 in a patient comprising administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described herein. In some embodiments, the ASGR, ASGR-1 and/or ASGR-2 inhibitor is an interfering RNA (e.g., siRNA or shRNA) as described herein.

BRIEF DESCRIPTION OF THE FIGURES

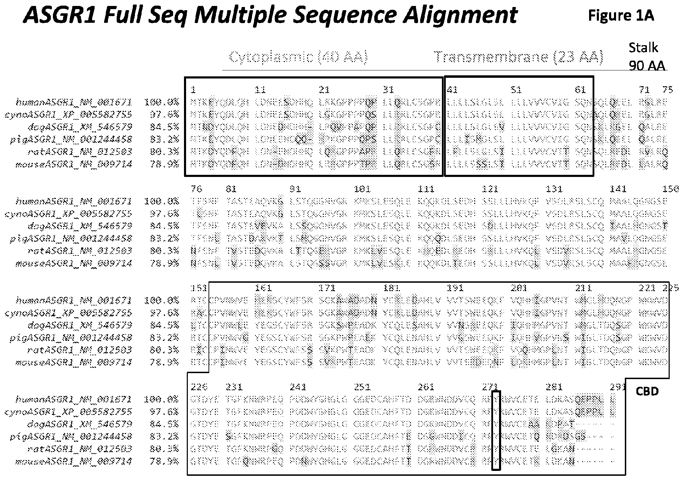

**Figure 1A.** ASGR-1 sequence alignments of human (SEQ ID NO: 32699), cynomolgus monkey (cyno) (SEQ ID NO: 32700), dog (SEQ ID NO: 32701), pig (SEQ ID NO: 32702), rat (SEQ ID NO: 32703) and mouse ASGR-1 (SEQ ID NO: 32704). The boxed areas denoting different regions of ASGR-1 (i.e., cytoplasmic, transmembrane, and the carbohydrate binding domain (CBD; also called the carbohydrate recognition domain, or CRD) are representative of the approximate amino acid locations of these regions; the human Y273 amino acid is boxed.

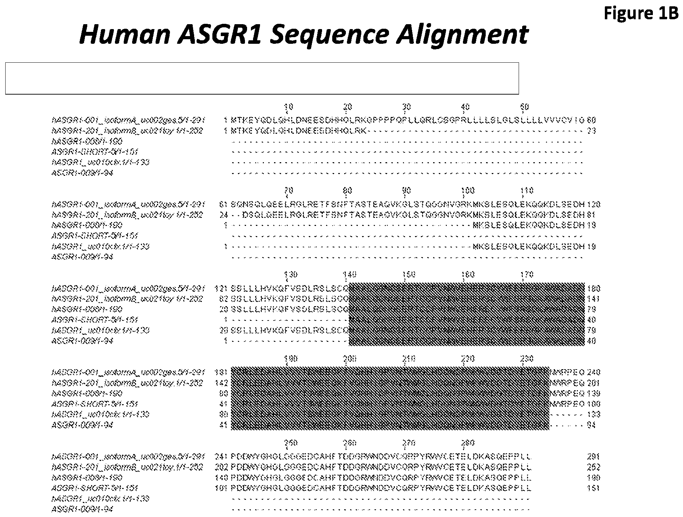

**Figure 1B.** Human ASGR-1 sequence alignments (SEQ ID NOS 32705-32710, respectively, in order of appearance).

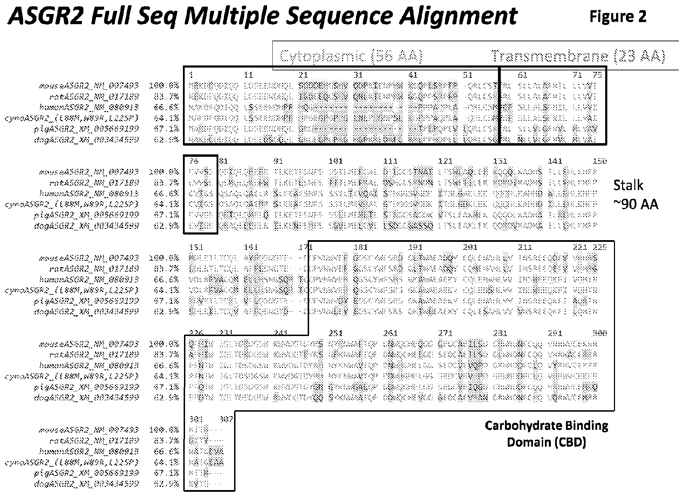

**Figure 2.** ASGR-2 sequence alignments of human (SEQ ID NO: 32713), cyno (SEQ ID NO: 32714), dog (SEQ ID NO: 32716), pig (SEQ ID NO: 32715), rat (SEQ ID NO: 32712) and mouse ASGR-2 (SEQ ID NO: 32711). The boxed areas denoting different regions of ASGR-2 (i.e., cytoplasmic, transmembrane, and the carbohydrate binding domain (CBD; also called the carbohydrate recognition domain, or CRD) are representative of the approximate amino acid locations of these regions.

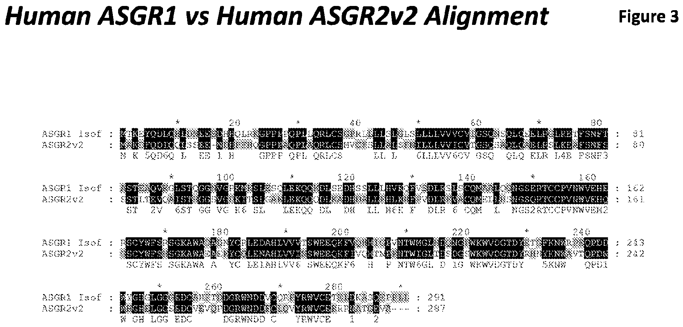

**Figure 3.** Human ASGR-1 (SEQ ID NO: 32717) vs. human ASGR-2v2 (SEQ ID NO: 32718) alignments are provided.

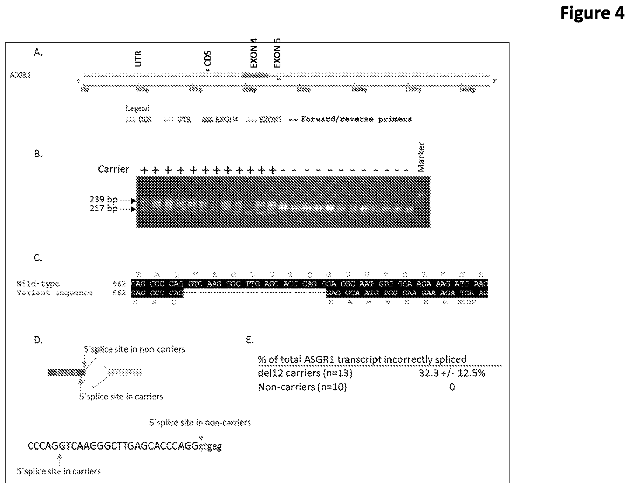

**Figure 4.** The del12 variant is associated with a splicing error and frameshift in ASGR-1. (A) Overview of the structure of the ASGR-1 mRNA. Exons 4 and 5 are highlighted (the del12 variant lies within intron 4 between exons 4 and 5 in the unspliced RNA) along with the positions of the PCR primers (red arrows) used to amplify the cDNA. (B) Agarose gel showing the PCR products generated by amplifying cDNA generated from RNA isolated from the blood of del12 carriers and non-carriers. Arrows indicate both the size of the expected PCR product (239 bp) along with the size of the truncated band (217 bp) observed only in del12 heterozygote carriers. (C) Shown is the sequence difference between the full-length (239 bp) and variant (217 bp) cDNA fragments based on Sanger sequencing. The variant sequence in del12 carriers lacks 22 bp at the end of exon 4 compared to the wild-type sequence that results in frame-shift and introduction of a stop codon. (D) Diagrammatic representation of the splicing defect observed in del12 carriers. The sequence around the exon 4-intron 4 boundary (exon 4 sequence in capital letters and intron 4 sequence in small letters) is shown along with the 5'splice site in non-carriers and the cryptic 5' splice site activated in del12 carriers. (E) Quantification of the full-length (239 bp) and variant (217 bp) cDNA fragments from heterozygote del12 carriers and non-carriers by direct digital counting of sequencing reads generated following sequencing of the amplified cDNA product from carriers and non-carriers of del12 using the Illumina TruSeq method. The percentage of incorrectly spliced ASGR-1 transcript is shown. Note that the incorrectly spliced form was completely undetectable in non-carriers.

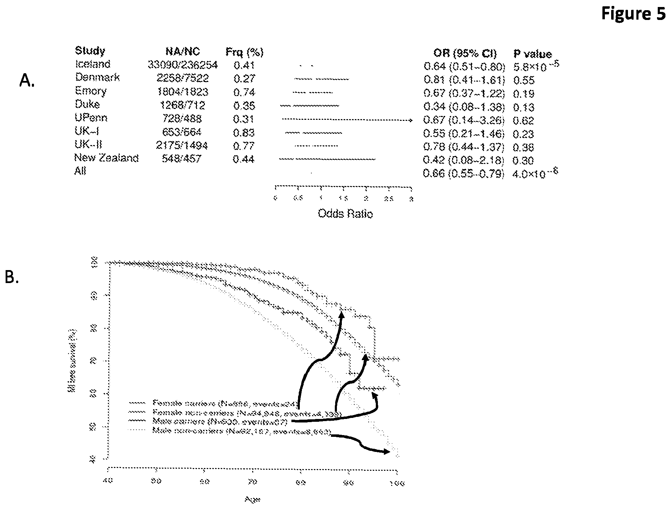

**Figure 5.** (A) The del12 variant was typed in the indicated populations a total of 41,648 CAD cases and 247,374 controls. For each cohort, the square (diamond in the case of the combined estimate) indicates the estimated odds ratio and the line shows the 95% confidence interval. There was no evidence of heterogeneity across the eight study populations (Phet = 0.96). (B) Kaplan-Meier curves for survival to first myocardial infarction in carriers and non-carriers of del12 in ASGR-1 stratified by sex. The proportion of individuals that have not had a myocardial infarction is shown on the y-axis and plotted against age on the x-axis. Males and females are represented separately and a distinction is made between del12 carriers and non-carriers in each case.

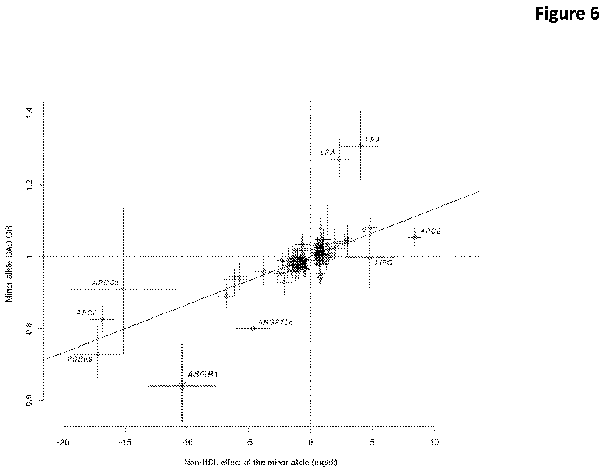

**Figure 6.** Comparison of relationship between CAD and non-HDL cholesterol levels between previously identified sequence variants and del12 in ASGR-1. Based on the Icelandic population, the estimated odds ratio (OR) of the minor allele for coronary artery disease (CAD, 41,648 cases and 247,374 controls) as a function of the estimated effect of the minor allele on non-HDL cholesterol levels (N=119,146). A full list of the sequence variants included is provided in Table 1.7. The error bars represent 95% confidence intervals. The del12 variant in ASGR-1 is shown. The line indicates the best linear regression fit through the origin.

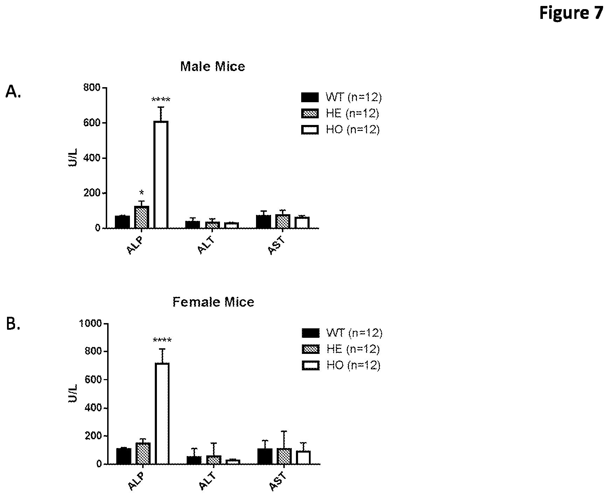

**Figure 7.** Analysis of serum ALP, ALT, and AST from ASGR-1 knockout mice is provided. Panel A is data from the male mice studied and Panel B is data from the female mice.

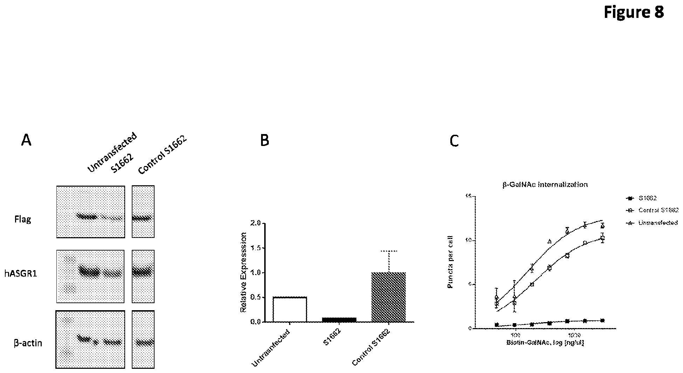

**Figure 8.** RNAi *in vitro* data in CHO cells transfected with hASGR-1 using construct S1662. Panel A is a western blot demonstrating reduction of expression of human ASGR-1. Panel B is a graphical representation of the relative reduction in expression of human ASGR-1. Panel C demonstrates that CHO cells receiving construct S1662 displays a dramatic reduction in internalization of ligand ($\beta$-GalNAc).

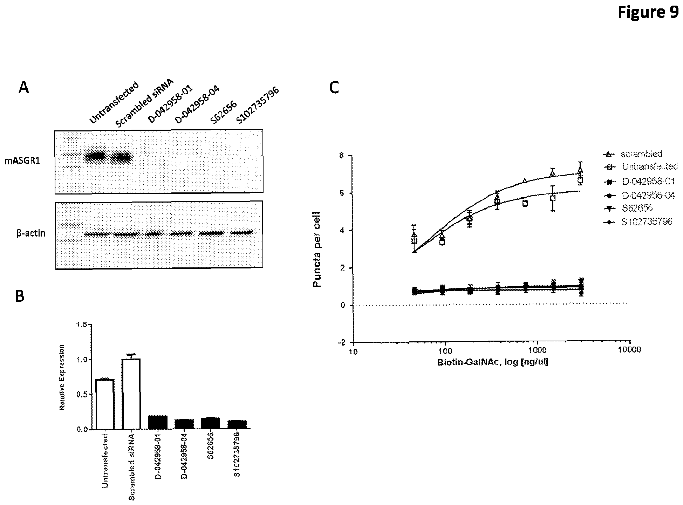

**Figure 9.** RNAi *in vitro* data in CHO cells transfected with mASGR-1 using various constructs. Panel A is a western blot demonstrating reduction of expression of mouse ASGR-1. Panel B is a graphical representation of the relative reduction in expression of mouse ASGR-1. Panel C demonstrates that CHO cells receiving the various constructs display a dramatic reduction in internalization of ligand ($\beta$-GalNAc).

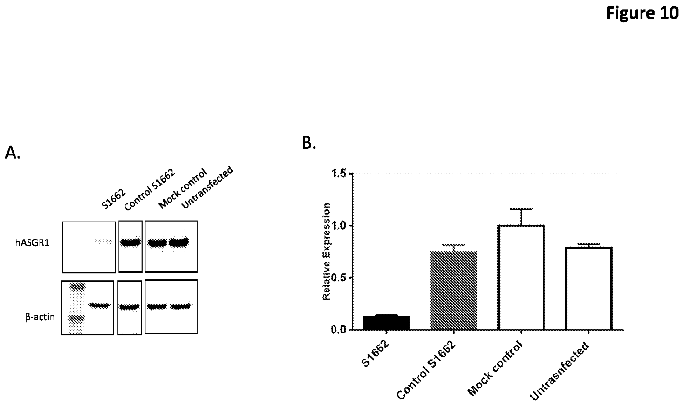

**Figure 10.** RNAi *in vitro* data in HepG2 cells using construct S1662. Panel A is a western blot demonstrating reduction of expression of human ASGR-1. Panel B is a graphical representation of the relative reduction in expression of human ASGR-1.

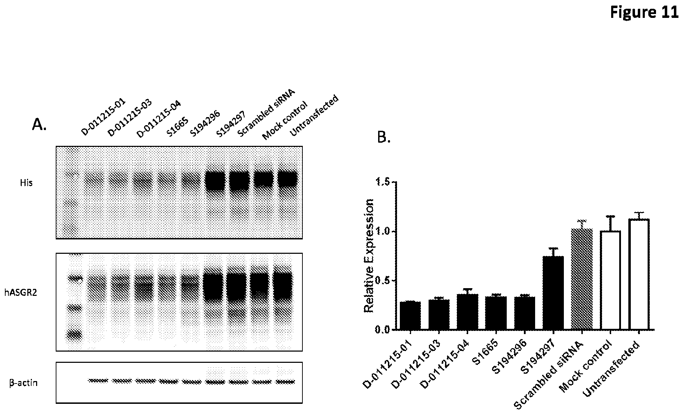

**Figure 11.** RNAi *in vitro* data in CHO cells transfected with hASGR-2 using various constructs. Panel A is a western blot demonstrating reduction of expression of human ASGR-2. Panel B is a graphical representation of the relative reduction in expression of human ASGR-2 by the various constructs.

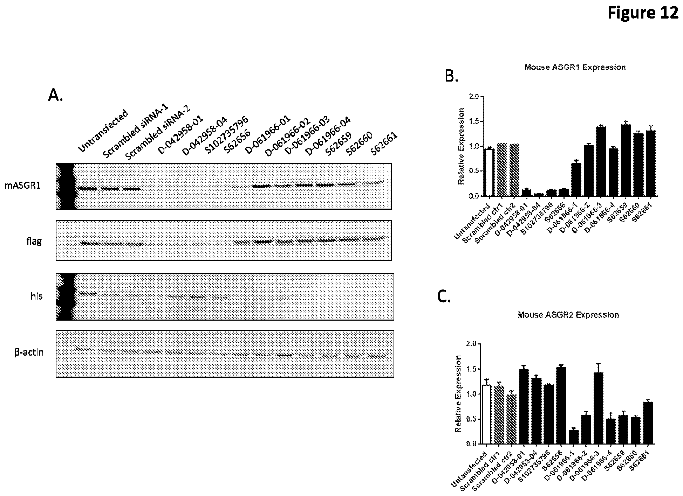

**Figure 12.** RNAi *in vitro* data in CHO cells transfected with mASGR-1 and mASGR-2 using various other constructs. Panel A is a western blot demonstrating reduction of expression of mouse ASGR-1 (anti-mouse ASGR-1 or anti-flag) or mouse ASGR-2 (anti-his). Panel B is a graphical representation of the relative reduction in expression of mouse ASGR-1 by the various constructs. Panel C is a graphical representation of the relative reduction in expression of mouse ASGR-2 by the various constructs.

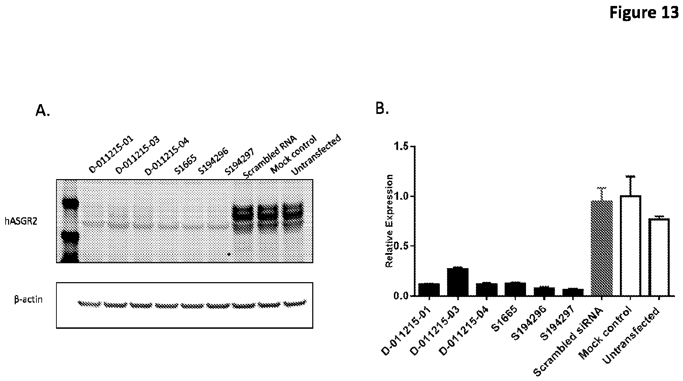

**Figure 13.** RNAi *in vitro* data in HepG2 cells using various constructs. Panel A is a western blot demonstrating reduction of expression of human ASGR-2. Panel B is a graphical representation of the relative reduction in expression of human ASGR-2 by the various constructs.

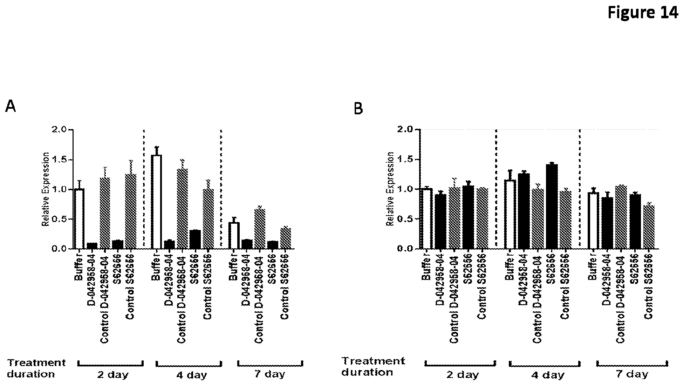

**Figure 14.** RNAi *in vivo* data in in C57BL/6J mice using various constructs over the course of 7 days with three injections total, one injection at day 0, one injection at day 2 and one injection at day 4. Panel A is a graphical representation of quantitative per data showing the relative reduction in expression of mASGR-1 RNA in the liver. Panel B is a graphical representation of the relative reduction in expression of mASGR-2 RNA in the liver.

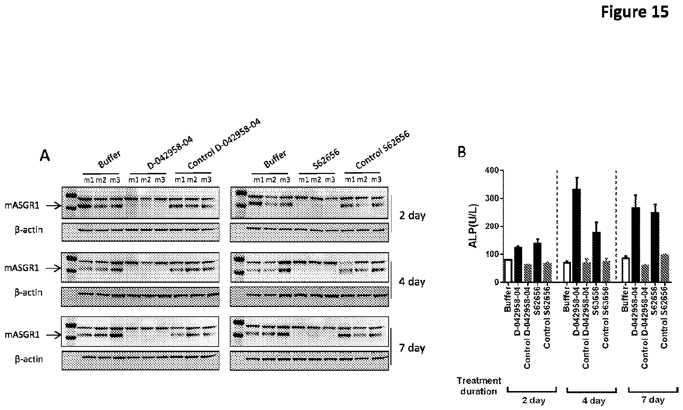

**Figure 15.** RNAi *in vivo* data in in C57BL/6J mice using various constructs over the course of 7 days with three injections total, one injection at day 0, one injection at day 2, and one injection at day 4. Panel A is a western blot demonstrating reduction of expression of mouse ASGR-1 protein. Panel B is a graphical representation of the relative increase of serum ALP activity.

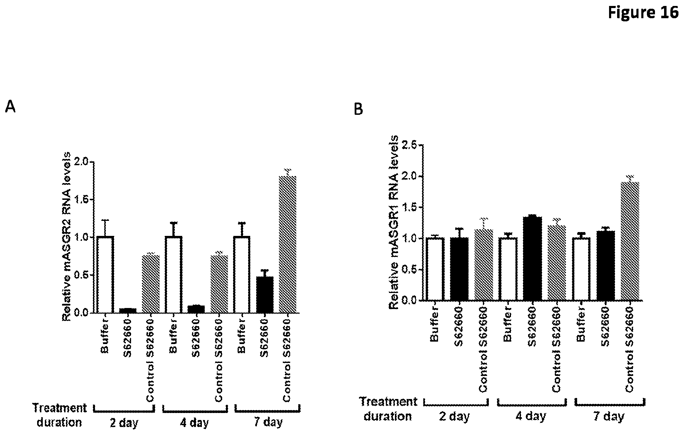

**Figure 16.** RNAi *in vivo* data in C57BL/6J mice using various constructs over the course of 7 days with one injection at day 0. Panel A is a graphical representation of the relative reduction in expression of mASGR-2 in the liver. Panel B is a graphical representation of the relative reduction in expression of mASGR-1 in the liver.

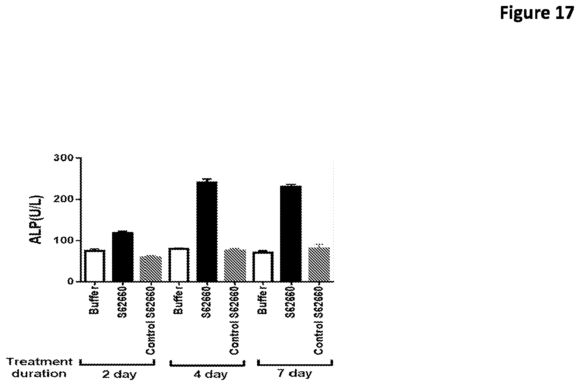

**Figure 17.** RNAi *in vivo* data in C57BL/6J mice using various ASGR-2 constructs over the course of 7 days with one injection at day 0. The figure is a graphical representation of the relative increase in serum ALP activity.

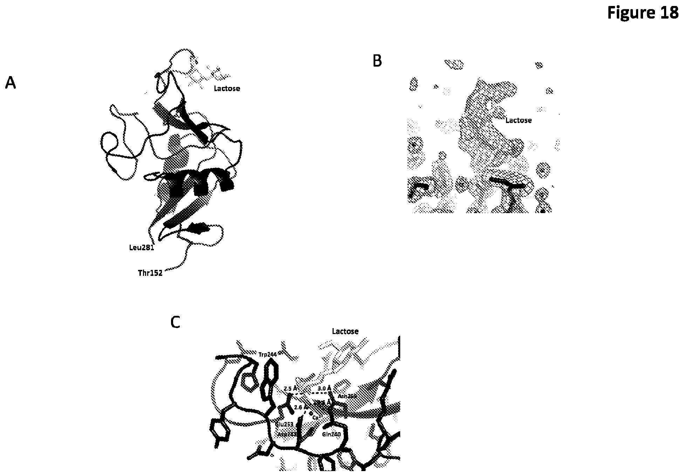

**Figure 18.** Panel A shows a computer representation of the crystal structure of the ASGR-1/lactose complex. Panel B is a computer representation of the observed electron density. Panel C is an enlarged view of the carbohydrate binding domain.

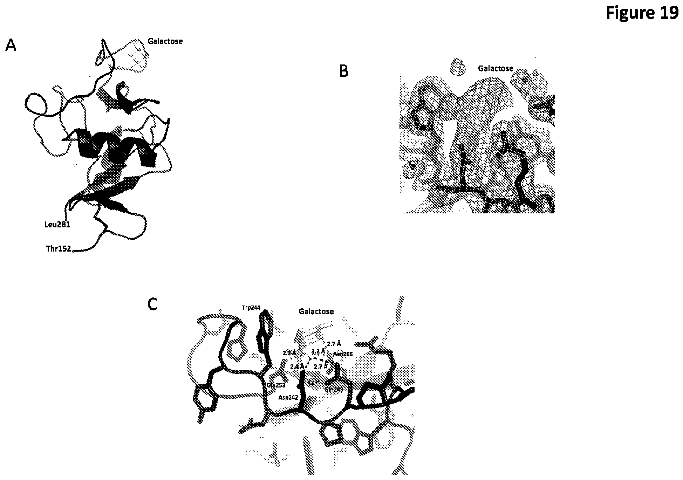

**Figure 19.** Panel A shows a computer representation of the crystal structure of the ASGR-1/galactose complex. Panel B is a computer representation of the observed electron density. Panel C is an enlarged view of the carbohydrate binding domain.

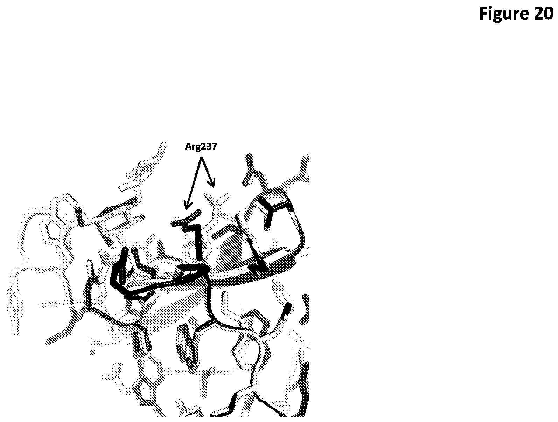

**Figure 20.** A computer representation of the crystal structure of an enlarged view of the conformational difference of R237 between the ASGR-1/lactose (white) complex and ASGR-1/galactose (black) complex.

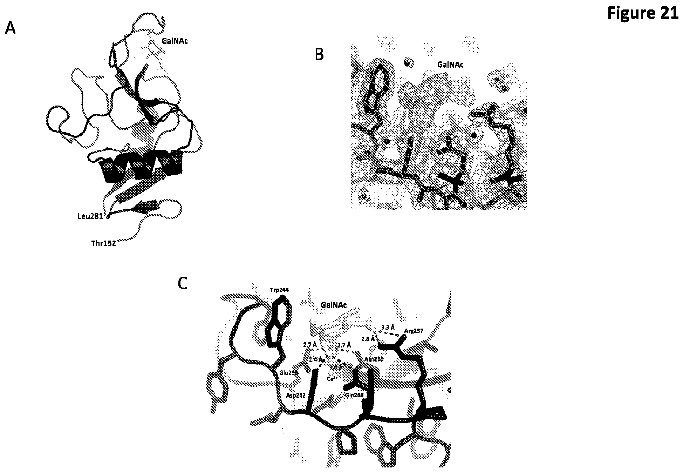

**Figure 21.** Panel A shows a computer representation of the crystal structure of the ASGR-1/GalNAc complex. Panel B is a computer representation of the observed electron density. Panel C is an enlarged view of the carbohydrate binding domain.

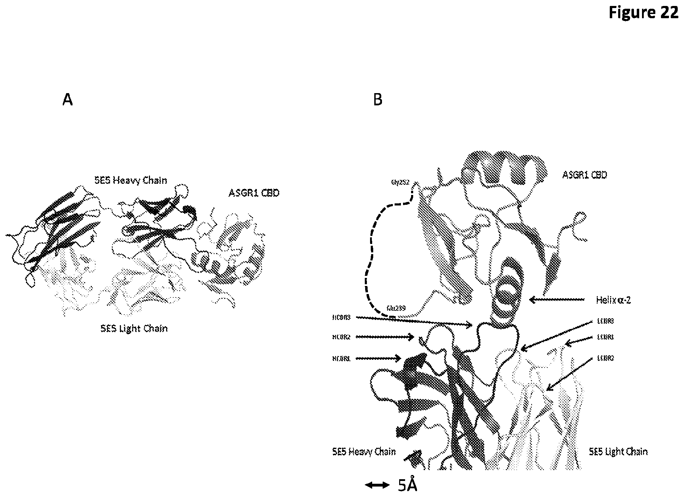

**Figure 22.** Panel A shows a depiction of the structure of the ASGR-1 CBD and the 5E5 Fab. Panel B is an enlarged view of the ASGR-1 CBD and 5E5 Fab that represents a disordered carbohydrate binding loop with a dashed line and highlights the indirect inhibition of ASGR-1 CBD and the ligand (GalNAc) binding. Panel B also incorporates a double-headed arrow which represents a 5 angstrom distance from tip to tip.

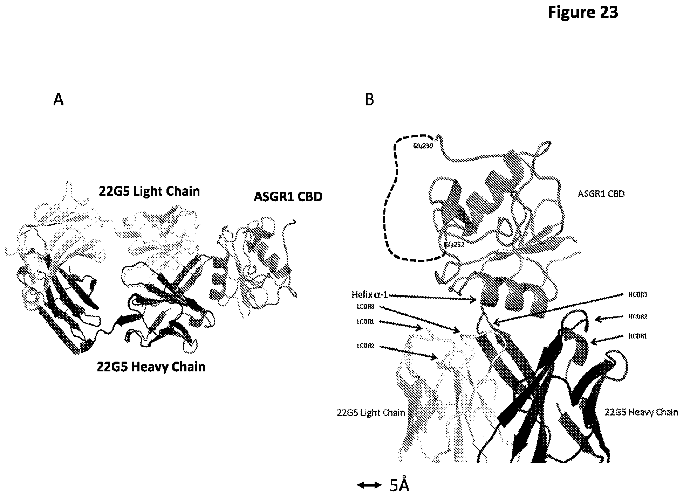

**Figure 23.** Panel A shows a depiction of the structure of the ASGR-1 CB and the 22G5 Fab. Panel B is an enlarged view of the ASGR-1 CBD and 22G5 Fab that represents a disordered carbohydrate binding loop with a dashed line and highlights the indirect inhibition of ASGR-1 CBD and the ligand (GalNAc) binding. Panel B also incorporates a double-headed arrow which represents a 5 angstrom distance from tip to tip.

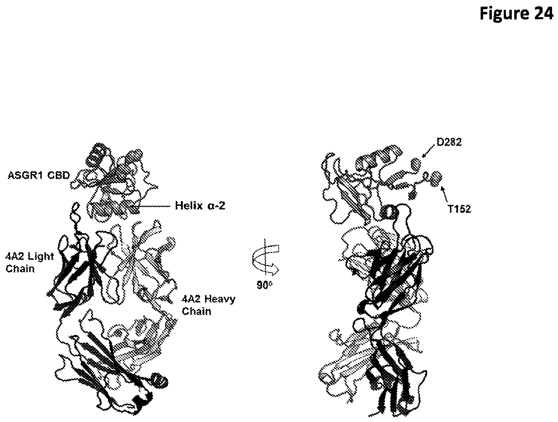

**Figure 24.** A depiction of the structure of the ASGR-1 CBD and the 4A2 Fab.

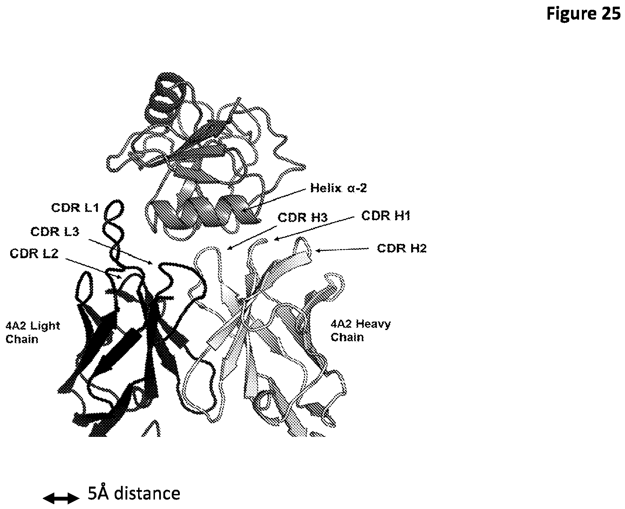

**Figure 25.** An enlarged view of the structure of the ASGR-1 CBD and the 4A2 Fab that shows the CDRs of the 4A2 Fab that interact with ASGR-1 CBD Helix alpha-2 and highlights the indirect inhibition of ASGR-1 CBD and the ligand (GalNAc) binding. The figure incorporates a double-headed arrow which represents a 5 angstrom distance from tip to tip.

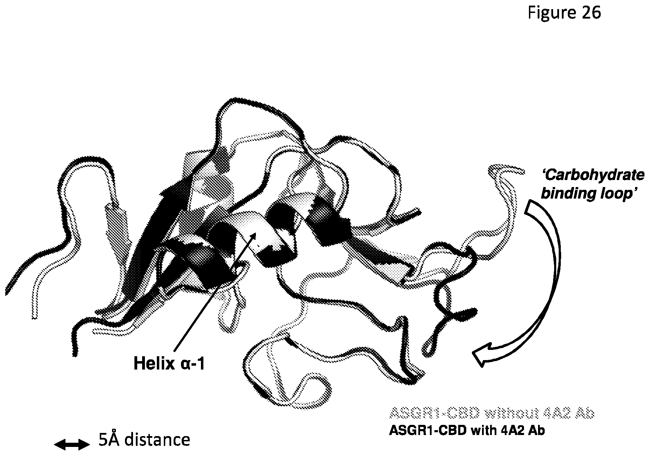

**Figure 26.** An enlarged view of the structure of the ASGR-1 CBD and the carbohydrate binding loop with and without and the 4A2 Fab that includes a double-headed arrow which represents a 5 angstrom distance from tip to tip.

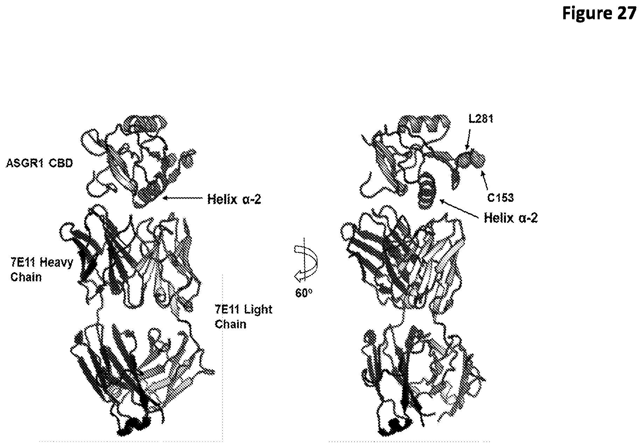

**Figure 27.** A depiction of the structure of ASGR-1 CBD and the 7E11 Fab.

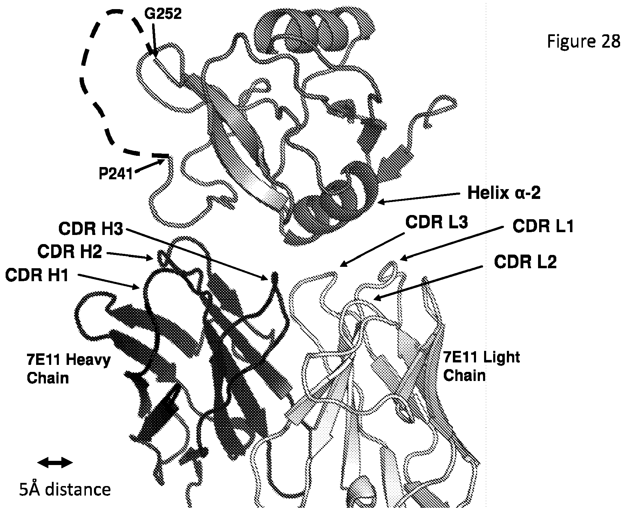

**Figure 28.** An enlarged view of the structure of the ASGR-1 CBD and the 7E11 Fab. The figure represents a disordered carbohydrate binding loop with a dashed line and highlights the indirect inhibition of ASGR-1 CBD and the ligand (GalNAc) binding. The figure incorporates a double-headed arrow which represents a 5 angstrom distance from tip to tip.

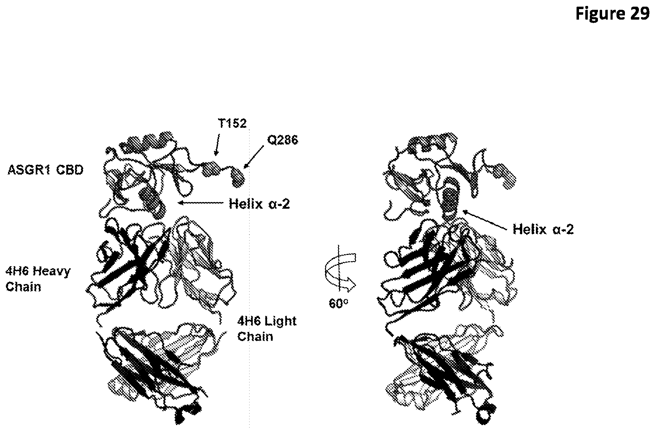

**Figure 29.** A depiction of the structure of the ASGR-1 CBD and the 4H6 Fab.

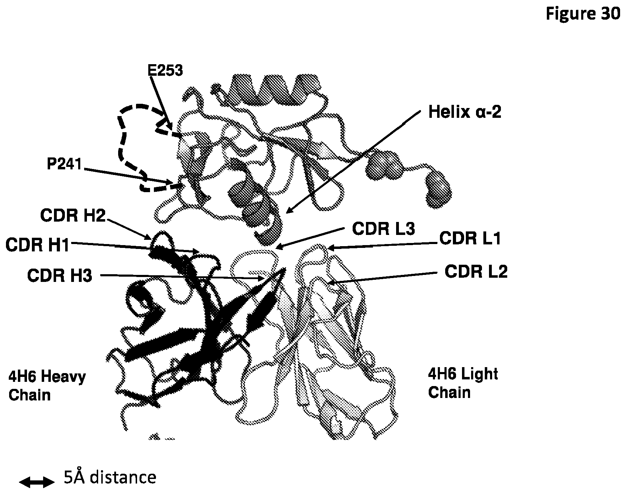

**Figure 30.** An enlarged view of structure of the ASGR-1 CBD and the 4H6 Fab. The figure represents a disordered carbohydrate binding loop with a dashed line and highlights the indirect inhibition of ASGR-1 CBD and the ligand (GalNAc) binding. The figure incorporates a double-headed arrow which represents a 5 angstrom distance from tip to tip.

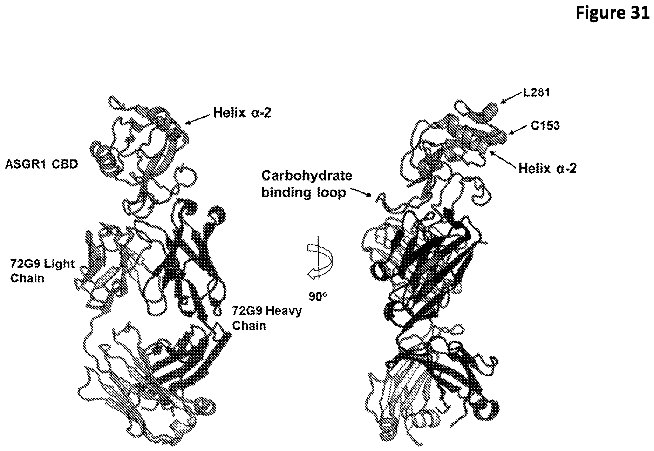

**Figure 31.** A depiction of the structure of the ASGR-1 CBD and the 72G9 Fab.

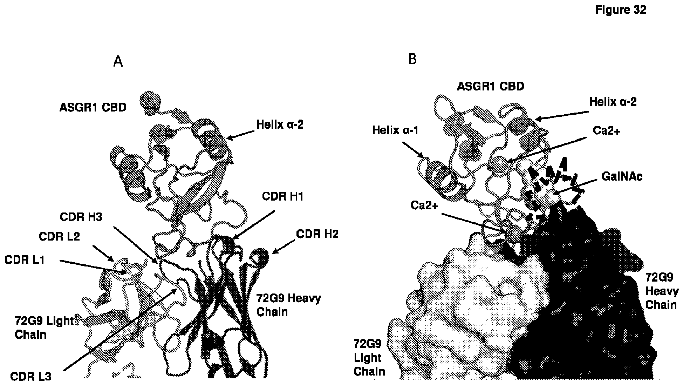

**Figure 32.** Panel A is an enlarged view of the structure of ASGR-1 CBD and the 72G9 Fab; and Panel B is a depiction of the structure of ASGR-1 CBD and the 72G9 Fab that also overlays the structure of ASGR-1 CBD and the ligand and highlights the direct inhibition of ASGR-1 CBD and the ligand (GalNAc) binding.

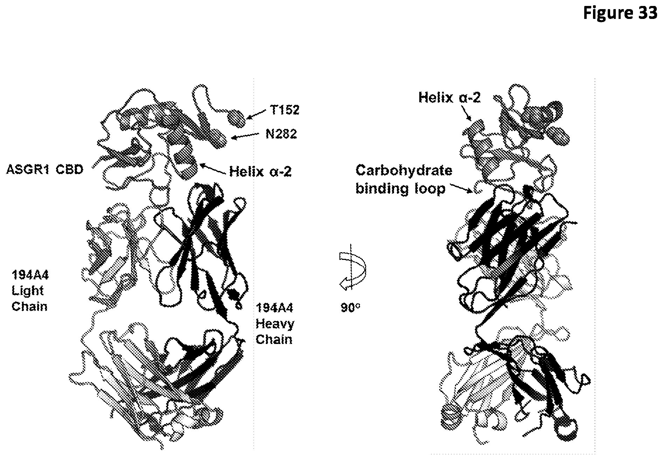

**Figure 33.** A depiction of the structure of the ASGR-1 CBD and the 194A4 Fab.

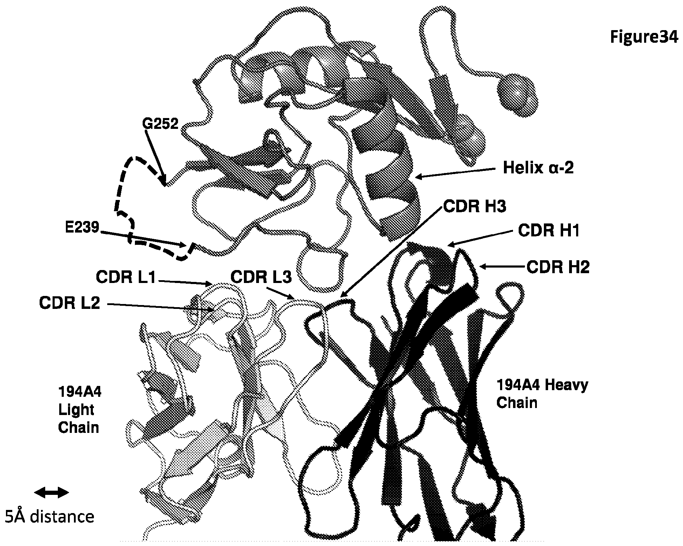

**Figure 34.** An enlarged view of the structure of the ASGR-1 CBD and the 194A4 Fab. The figure represents a disordered carbohydrate binding loop with a dashed line and highlights the indirect inhibition of ASGR-1 CBD and the ligand (GalNAc) binding. The figure incorporates a double-headed arrow which represents a 5 angstrom distance from tip to tip.

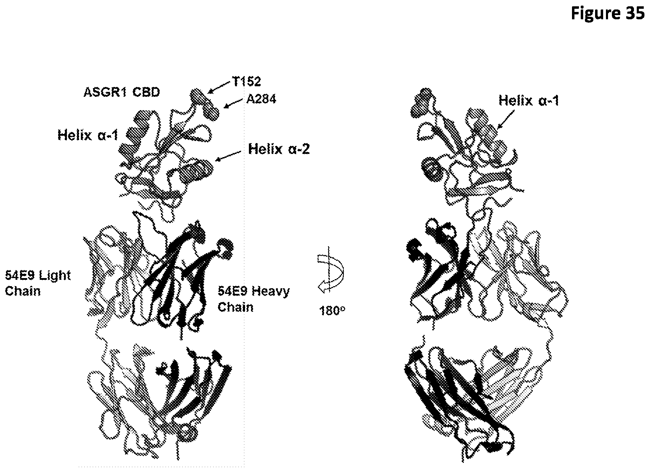

**Figure 35.** A depiction of the structure of the ASGR-1 CBD and the 54E9 Fab.

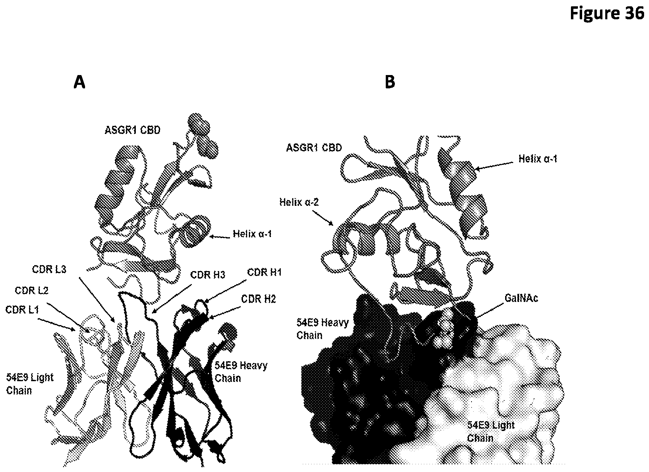

**Figure 36.** Panel A is an enlarged view of the structure of the ASGR-1 CBD and the 54E9 Fab; and Panel B is a depiction of the structure of the ASGR-1 CBD and the 54E9 Fab that also overlays the structure of ASGR-1 CBD and the ligand and highlights the direct inhibition of ASGR-1 CBD and the ligand (GalNAc) binding.

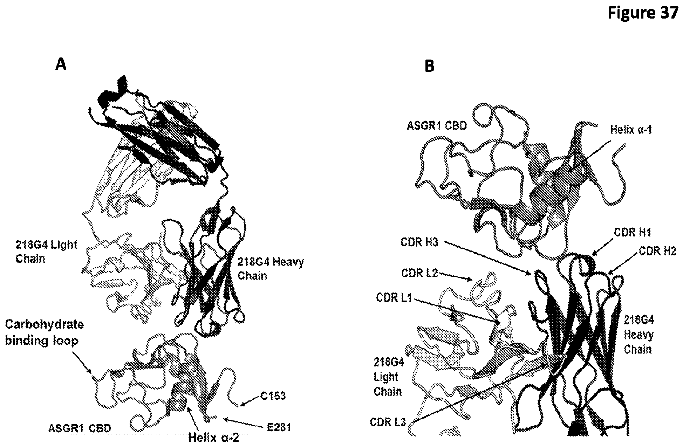

**Figure 37.** Panel A is a depiction of the structure of the ASGR-1 CBD and the 218G4 Fab; and Panel B is an enlarged view of the structure of the ASGR-1 CBD and the 218G4 Fab.

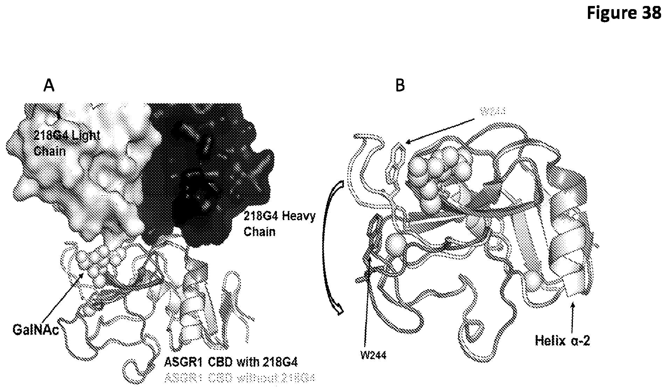

**Figure 38.** Panels A and B are enlarged views of the structure of ASGR-1 CBD and the 218G4 Fab that also overlays the structure of ASGR-1 CBD and the ligand. These figures highlight the direct inhibition of ASGR-1 CBD and the ligand (GalNAc) binding when the 218G4 Fab is present.

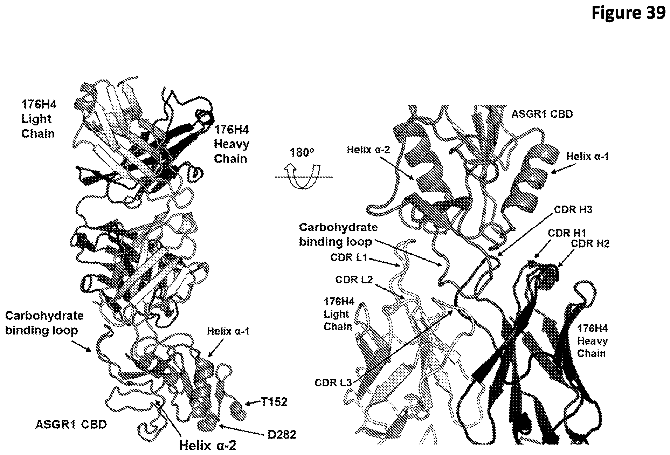

**Figure 39.** A depiction of the structure of the ASGR-1 CBD and the 176H4 Fab.

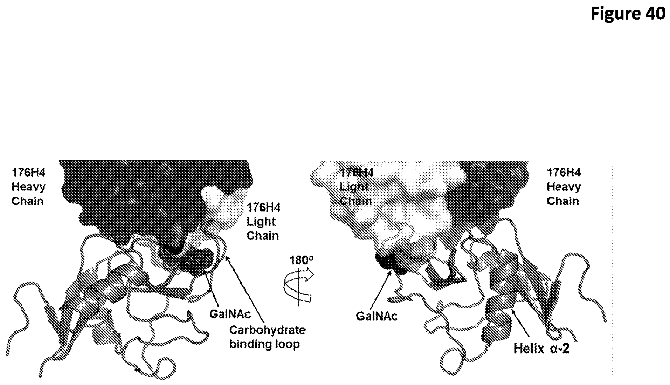

**Figure 40.** An enlarged view of the structure of the ASGR-1 CBD and the 176H4 Fab that also overlays the structure of ASGR-1 CBD and the ligand. This figure highlight the direct inhibition of ASGR-1 CBD and the ligand (GalNAc) binding when the 176H4 Fab is present.

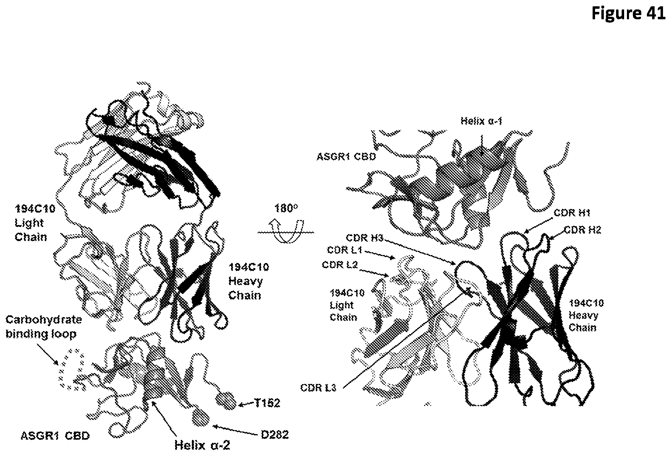

**Figure 41.** A depiction of the structure of the ASGR-1 CBD and the 194C10 Fab. This figure depicts represents a disordered carbohydrate binding loop with a dashed line and highlights possible indirect inhibition of ASGR-1 CBD and the ligand (GalNAc) binding.

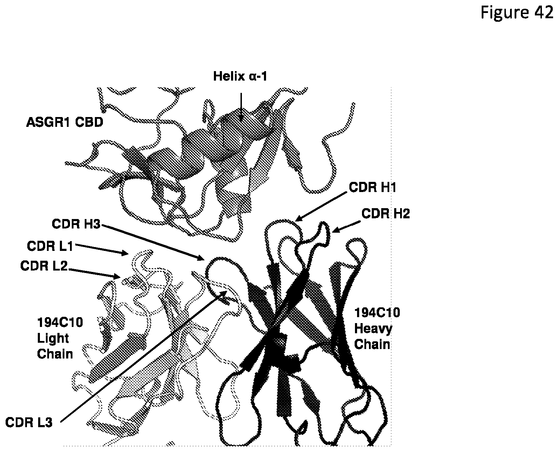

**Figure 42.** An enlarged view of the structure of the ASGR-1 CBD and the 194C10 Fab. This figure shows the CDRs of the 194C10 that interact with the ASGR-1 CBD and highlights that there may be direct inhibition of the ASGR-1 CBD and the ligand (GalNAc) binding.

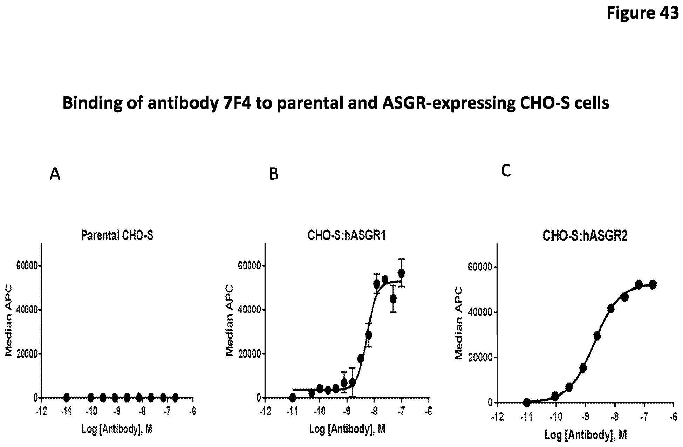

**Figure 43.** Panels A-C are graphical representations showing antibody binding results from human ASGR-1 and human ASGR-2 expressing cells.

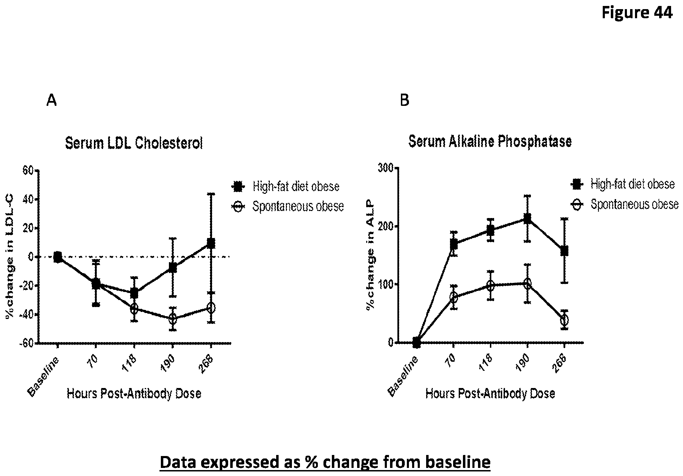

**Figure 44.** Panel A is a graphical representation of the effect of ASGR-1 antibody, 4A2, on serum LDL cholesterol levels in obese cynomologous monkeys. Panel B is a graphical representation of the effect of ASGR-1 antibody, 4A2, on serum alkaline phosphatase levels in obese cynomologous monkeys. Data is expressed in the % change from baseline.

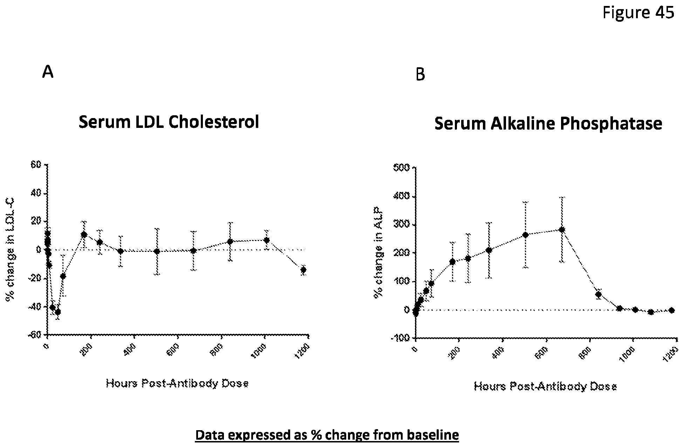

**Figure 45.** Panel A is a graphical representation of the effect of ASGR-1 antibody, 4A2, on serum LDL cholesterol levels in normal cynomologous monkeys. Panel B is a graphical representation of the effect of ASGR-1 antibody, 4A2, on serum alkaline phosphatase levels in normal cynomologous monkeys. Data is expressed in the percent change from baseline.

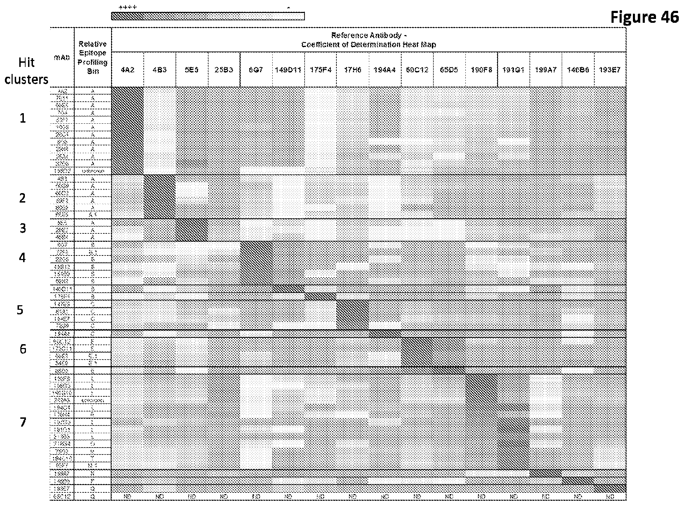

**Figure 46.** A coefficient of determination heat map representing the coefficient of determination profiles of test ASGR-1 ligand blocking antibody-reference antibody combinations from an Arginine/Glutamic Acid scanning mutagenesis (Example 7E). Dark shading represents highly similar data, while light shading represents highly dissimilar data. The relative epitope profiling (antibody competition/binding) bin assignments are also indicated.

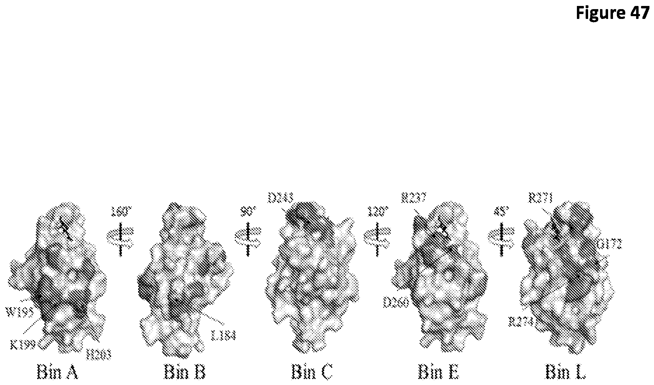

**Figure 47.** A computer representation showing alternative views of the ASGR-1 CBD protein and the surface locations of amino acid residues identified as being important for antibody binding via Arginine/Glutamic Acid scanning mutagenesis (Example 7E). The relative epitope profiling (antibody competition/binding) bin assignments are also indicated. Ligand (GalNAc) is shown as a stick representation (black). The ASGR-1 CBD is shown as a surface representation (light grey). The positions of amino acids identified by Arg/Glu mutational scanning are indicated (dark grey surface). The relative positions of key amino acids in each bin are shown for reference only.

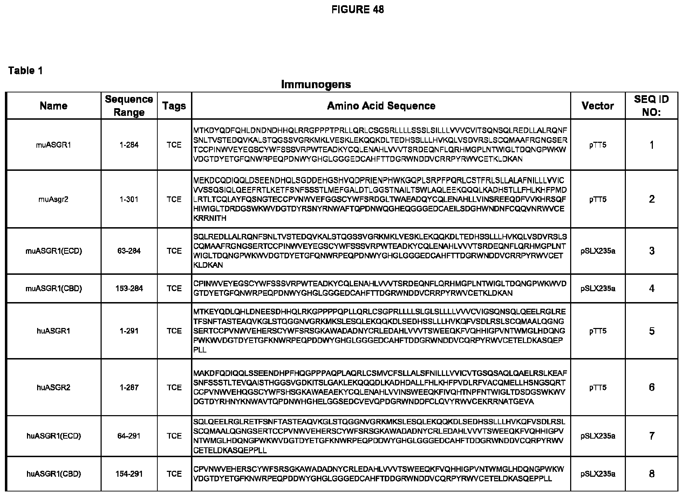

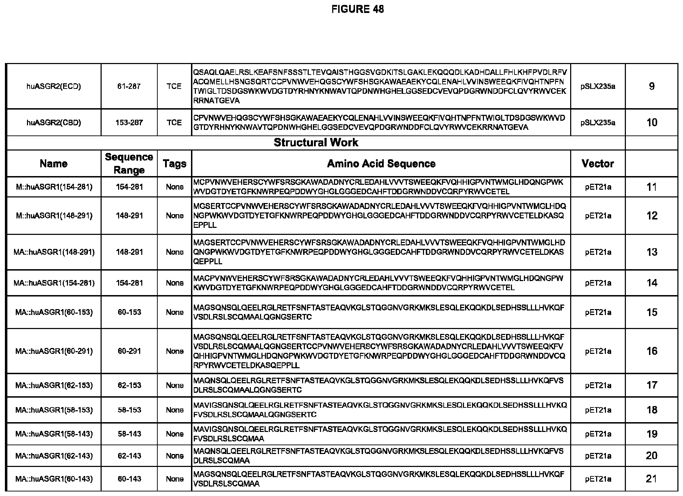

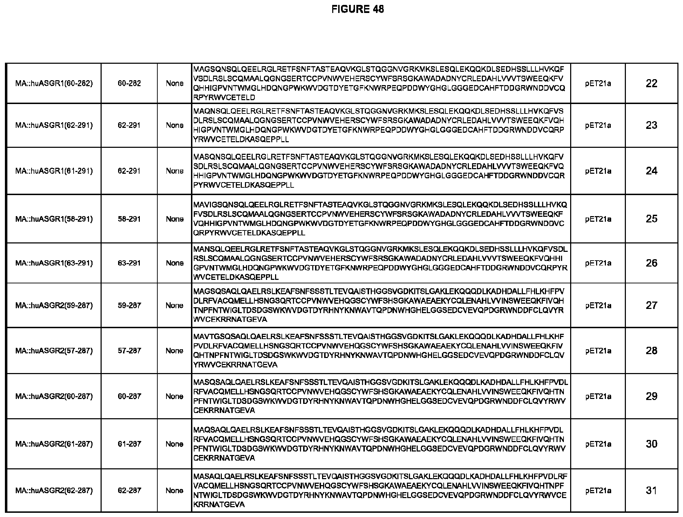

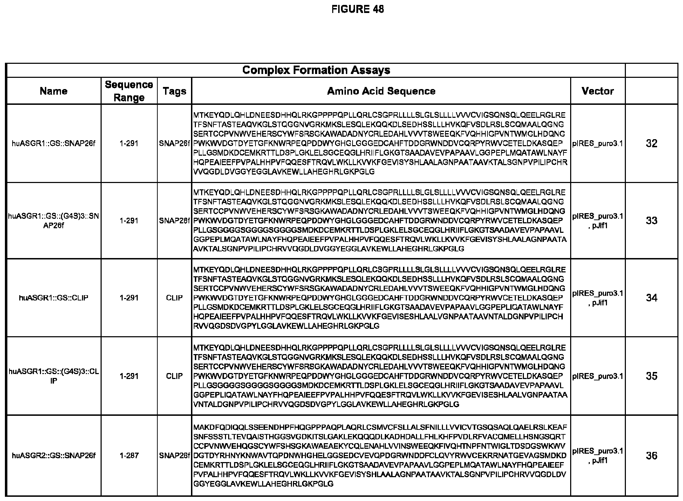

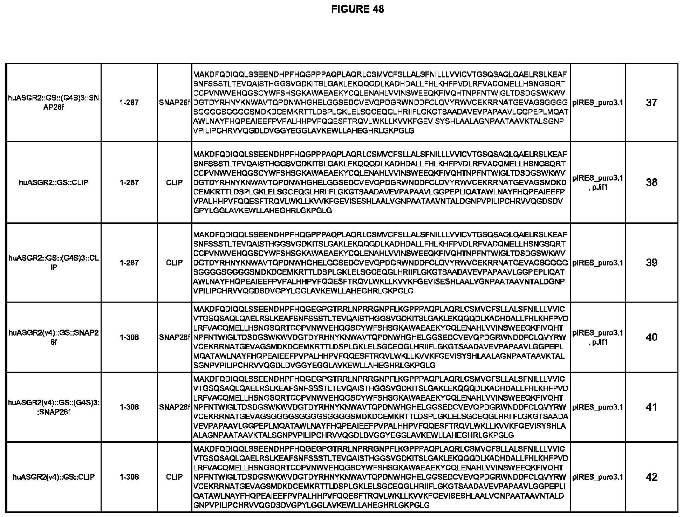

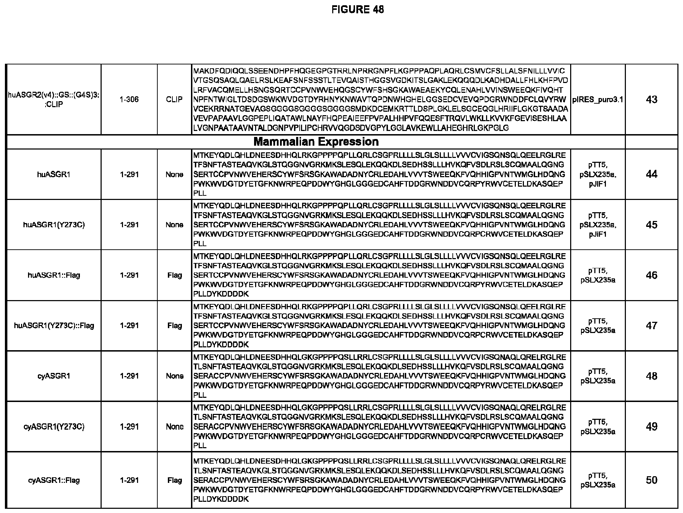

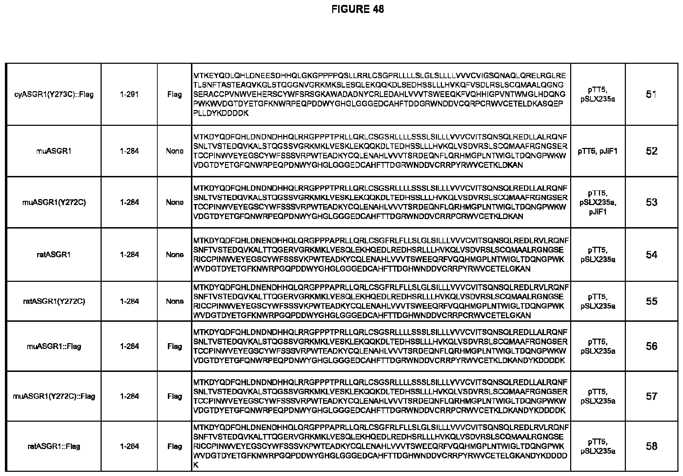

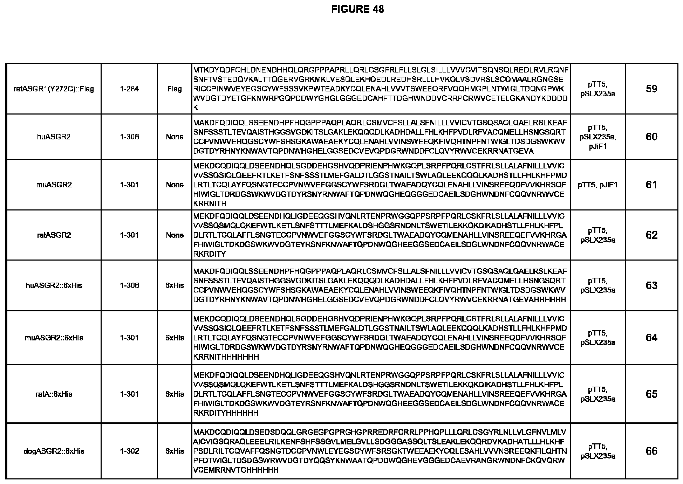

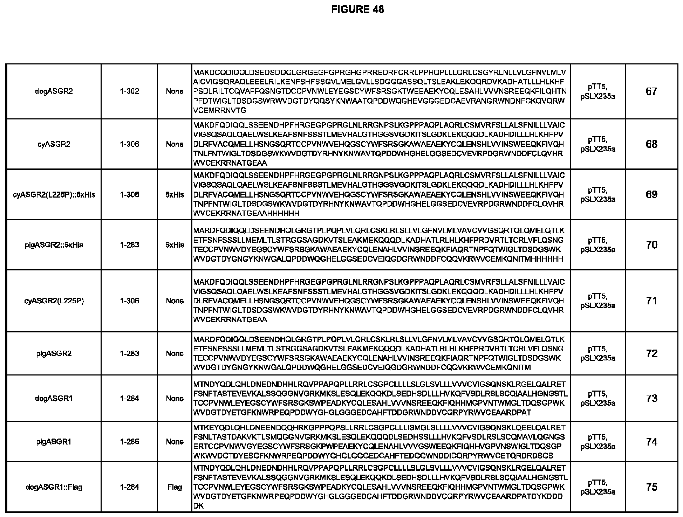

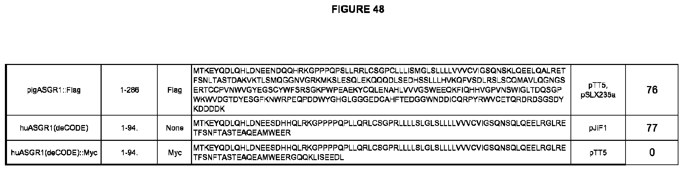

**Figure 48.** A table presenting various protein sequences for human, mouse, rat, pig, dog and cynomolgus monkey ASGR, ASGR-1 and ASGR-2 (Table 1).

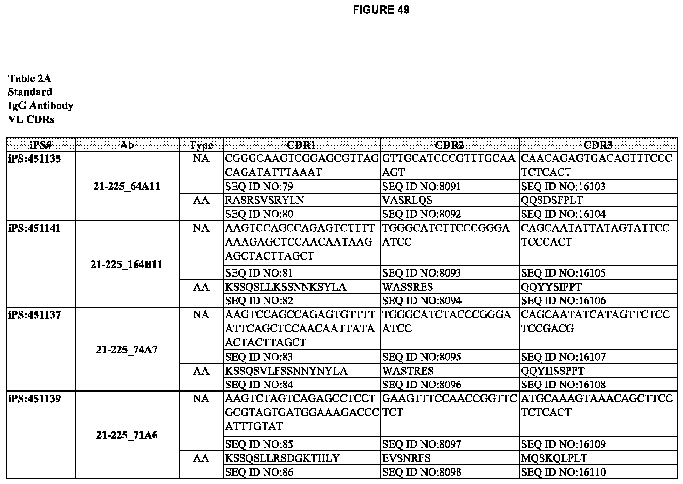

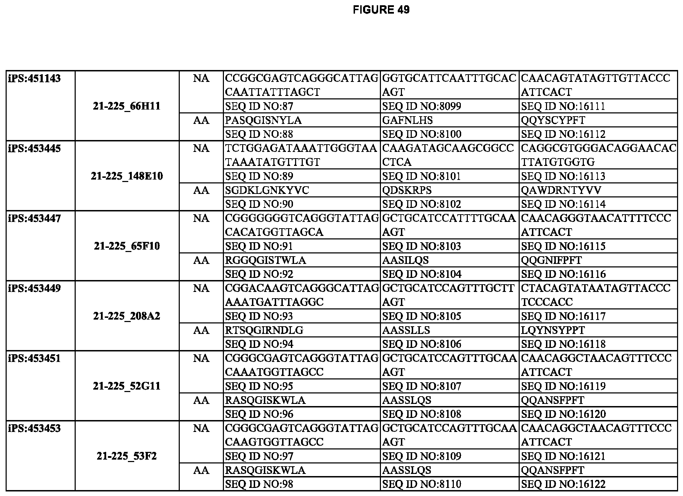

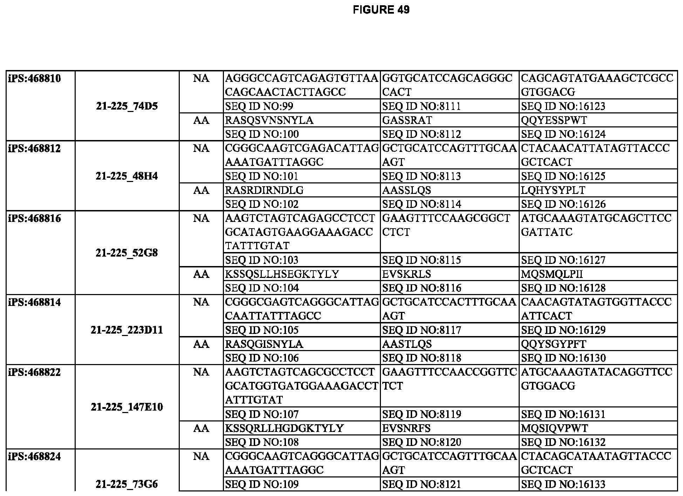

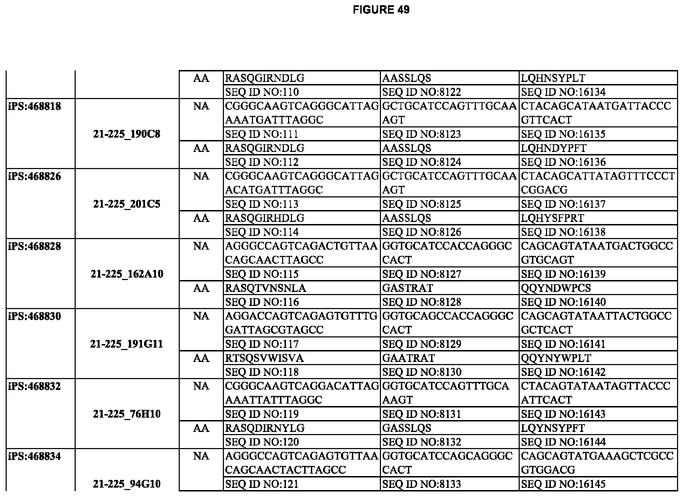

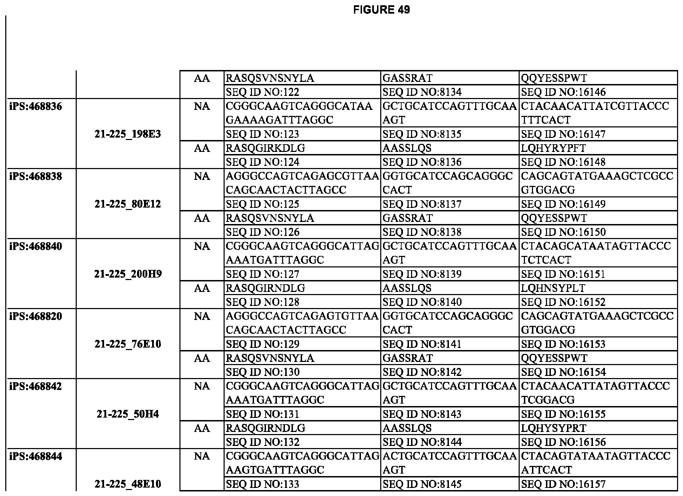

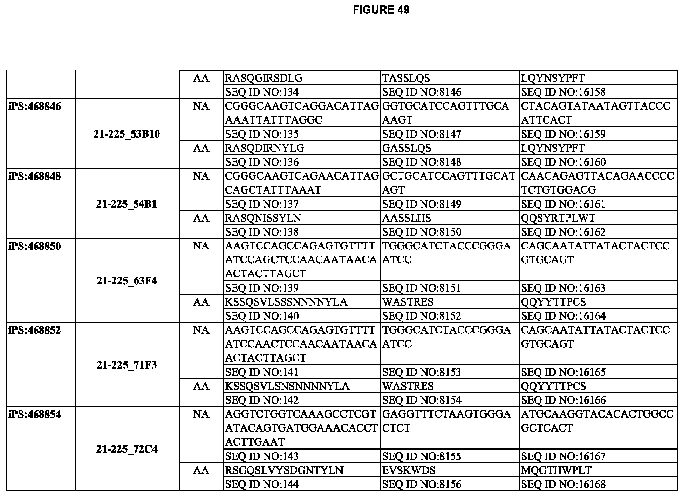

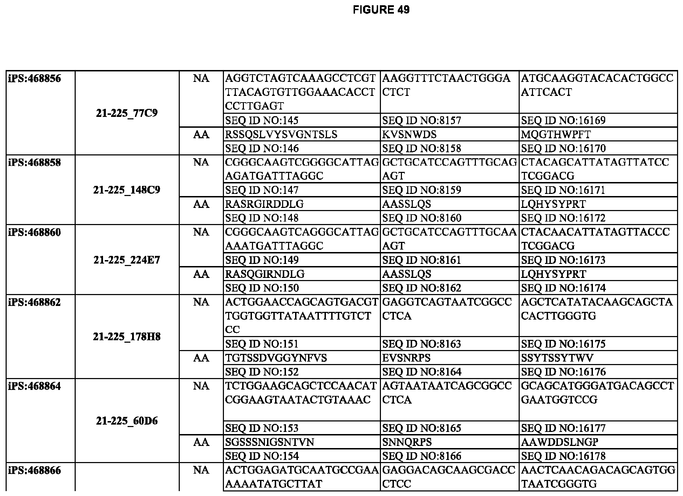

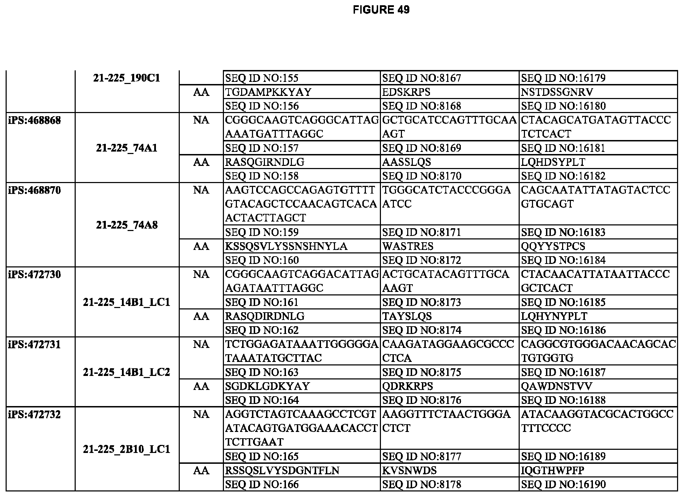

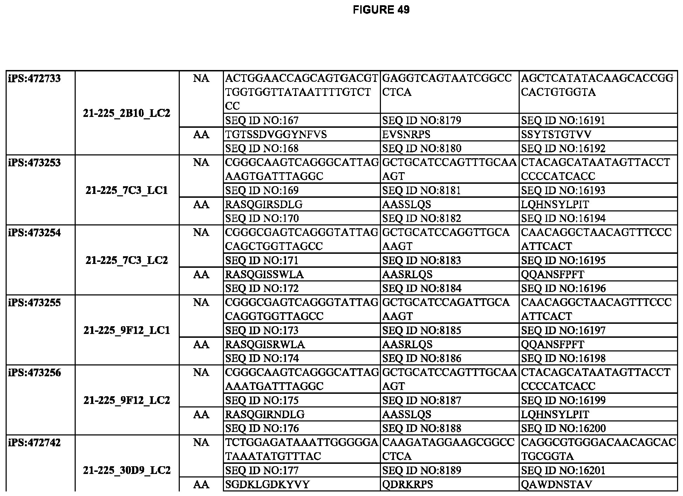

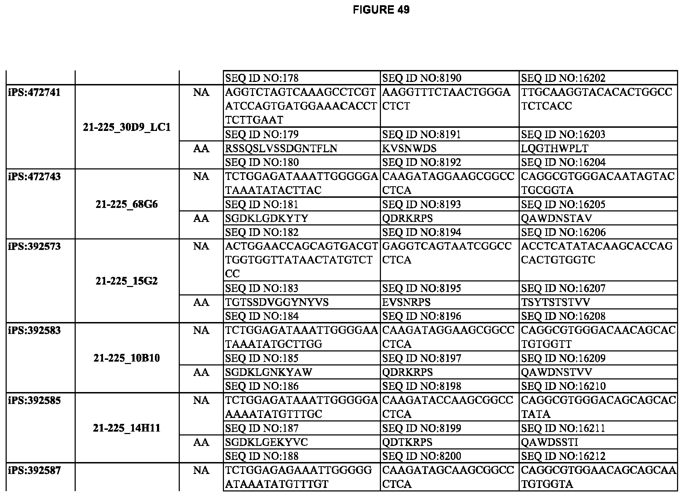

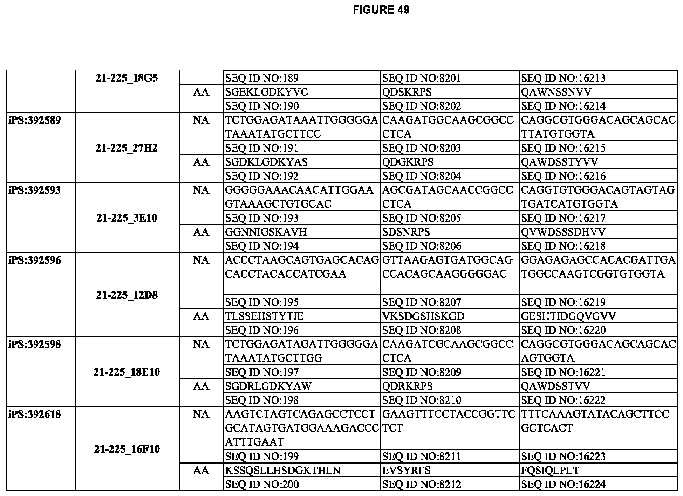

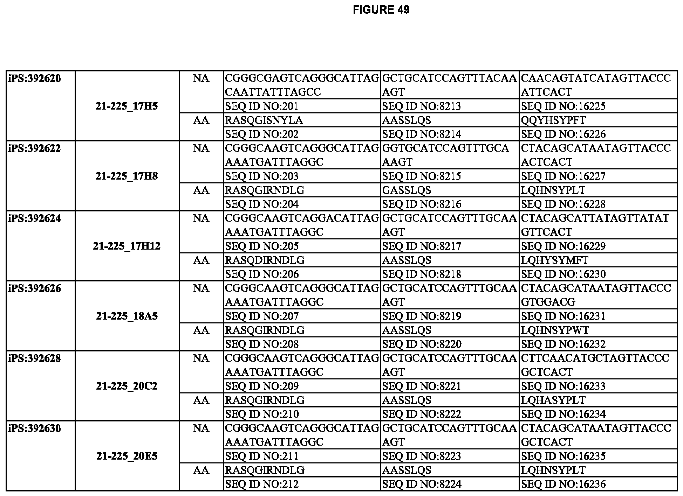

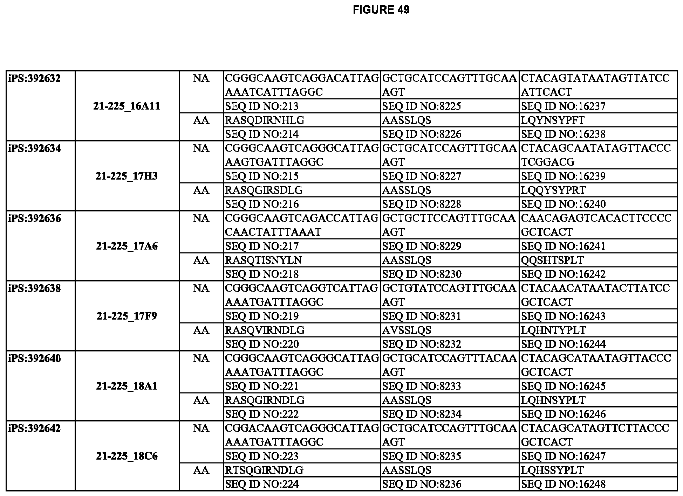

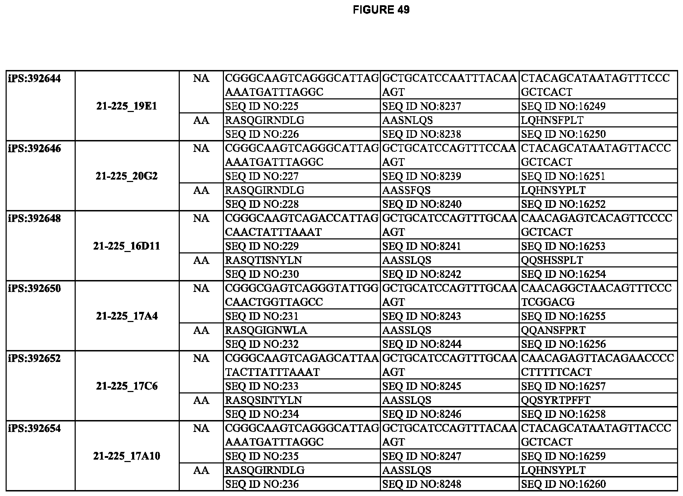

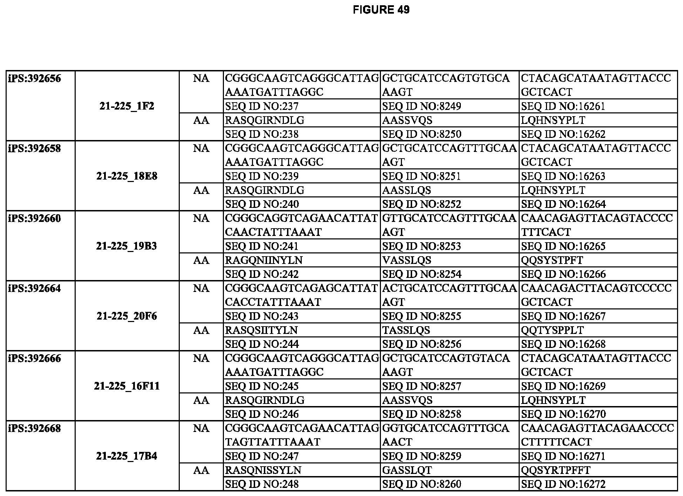

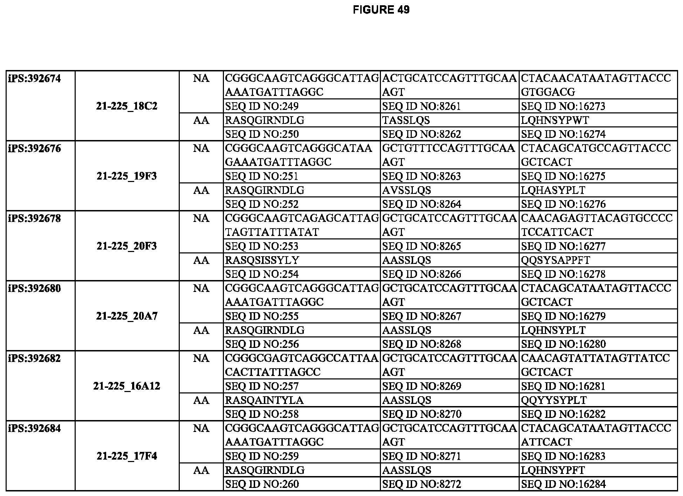

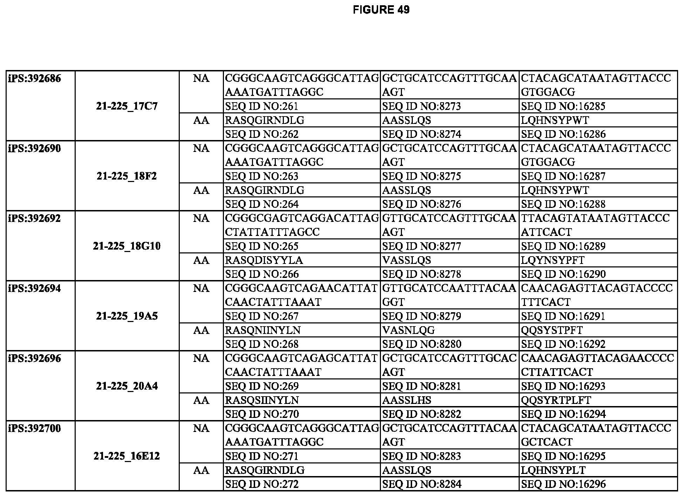

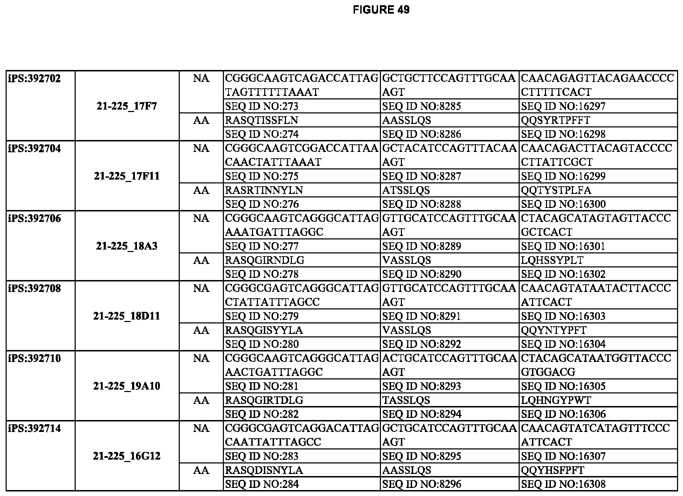

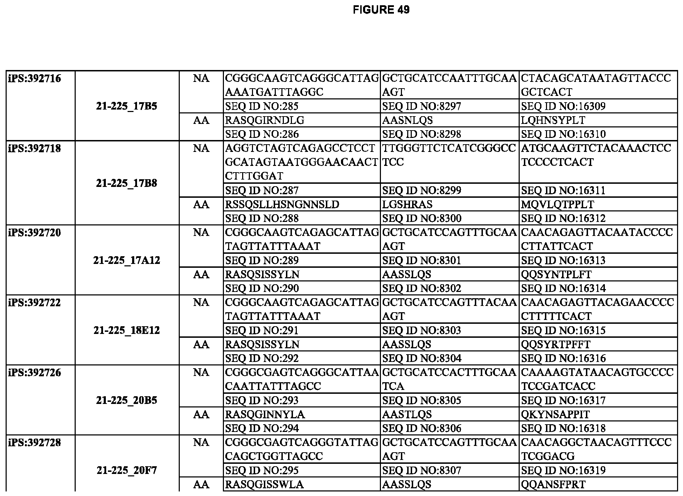

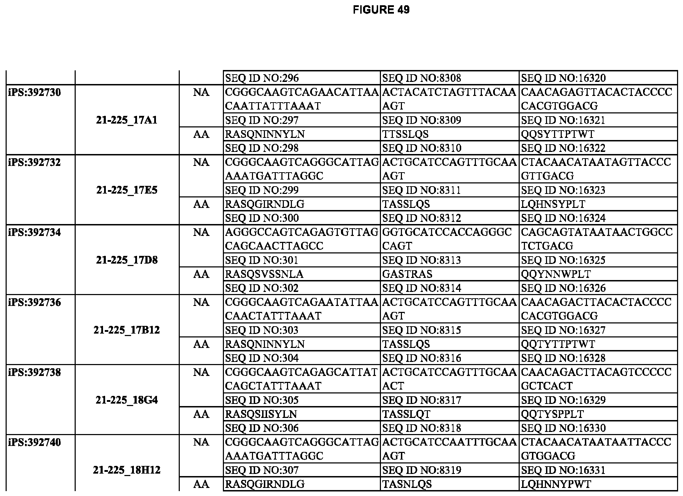

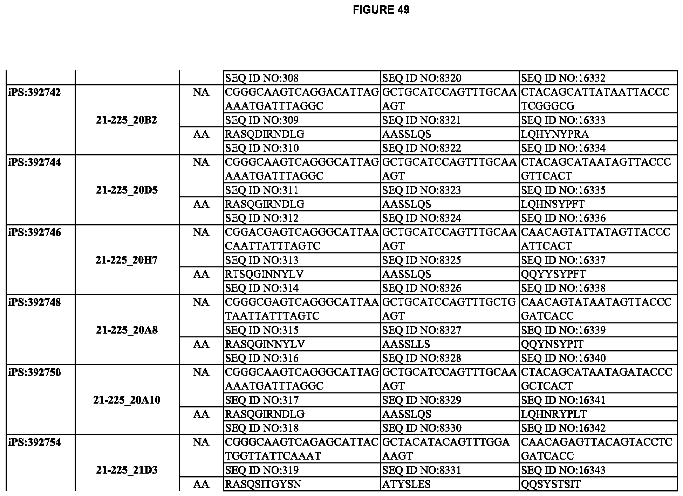

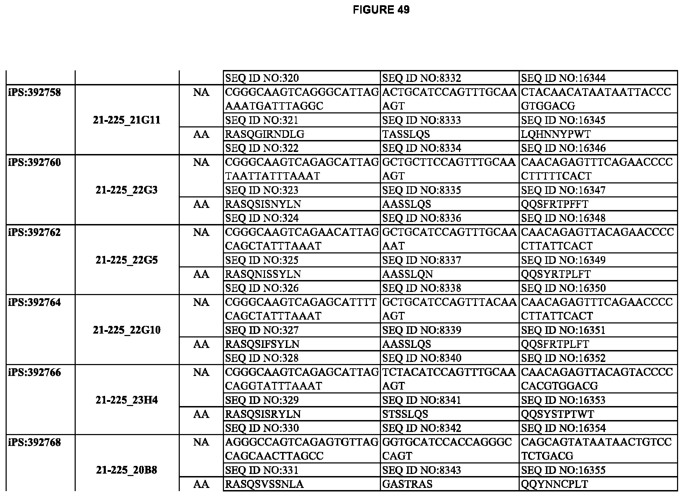

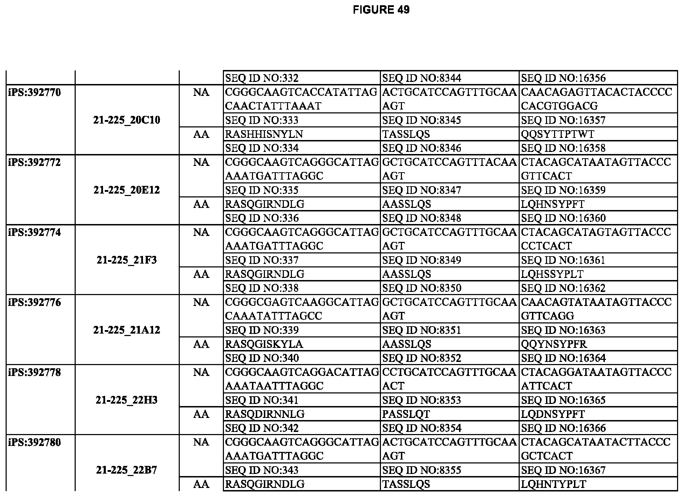

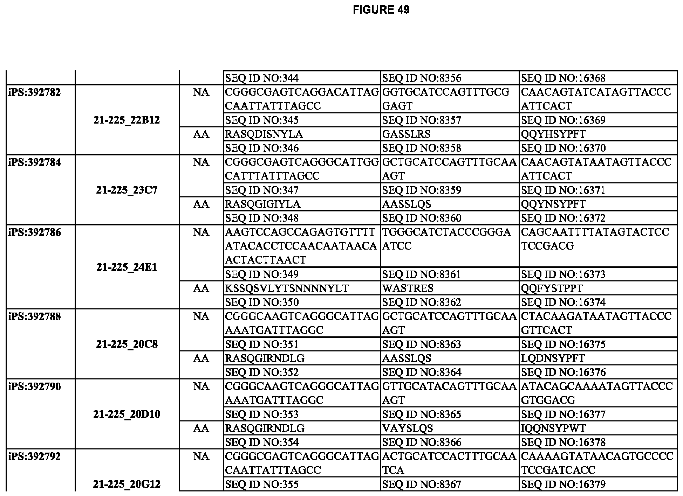

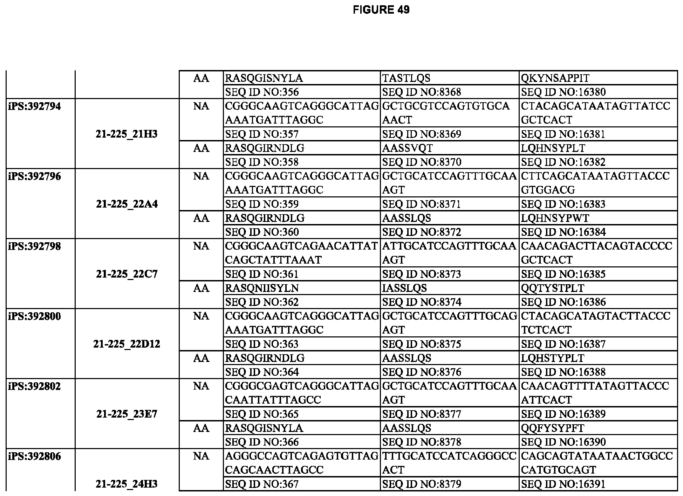

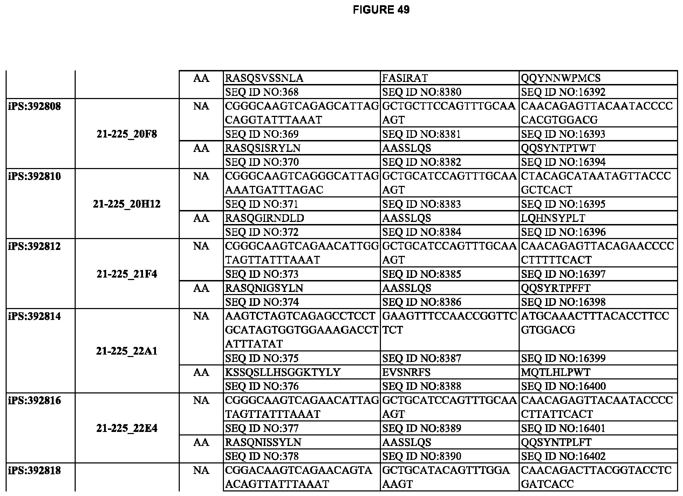

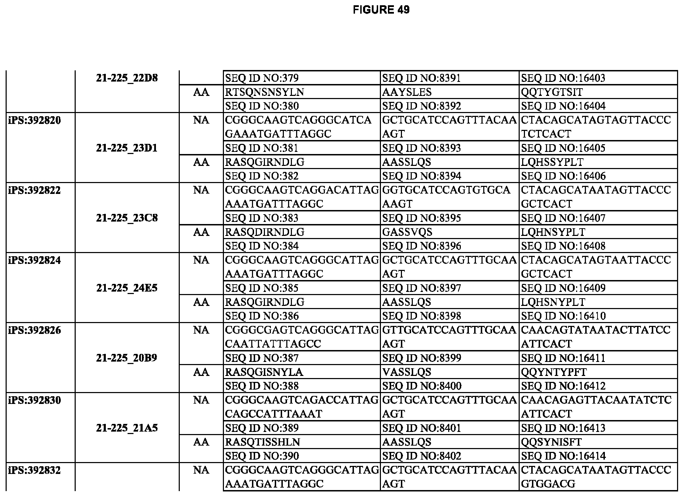

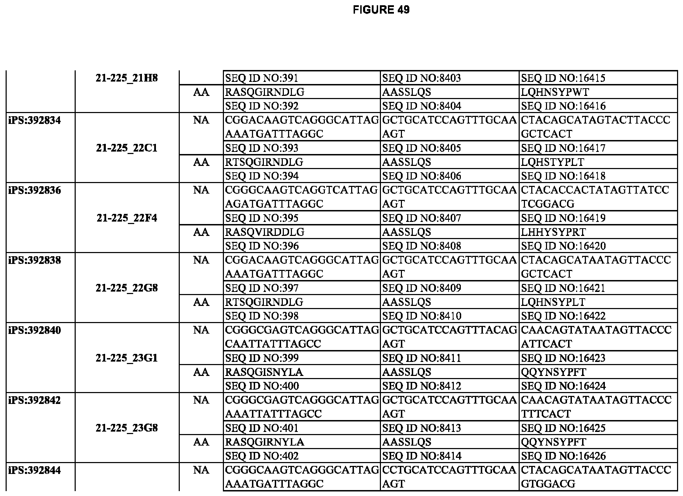

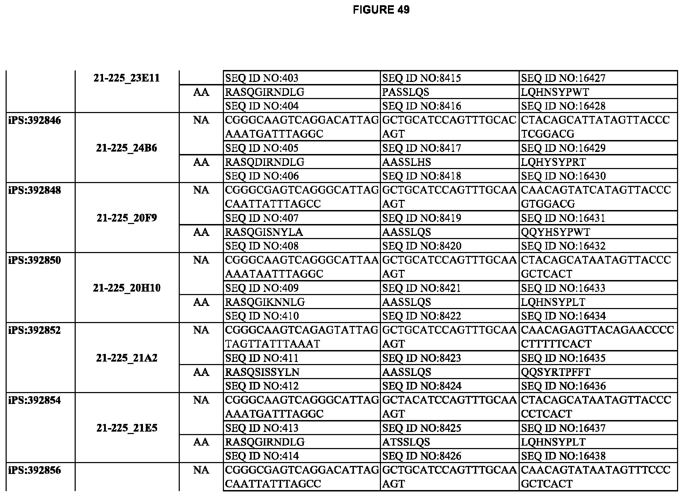

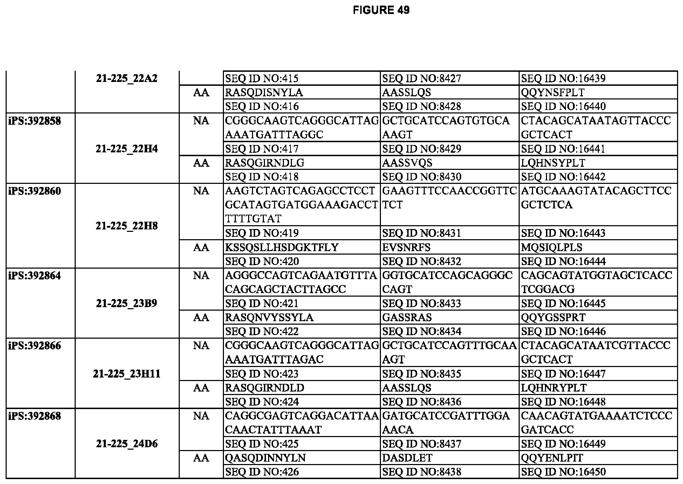

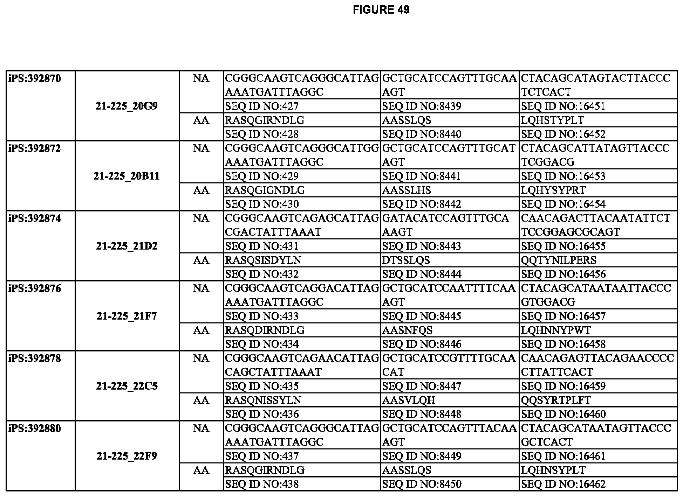

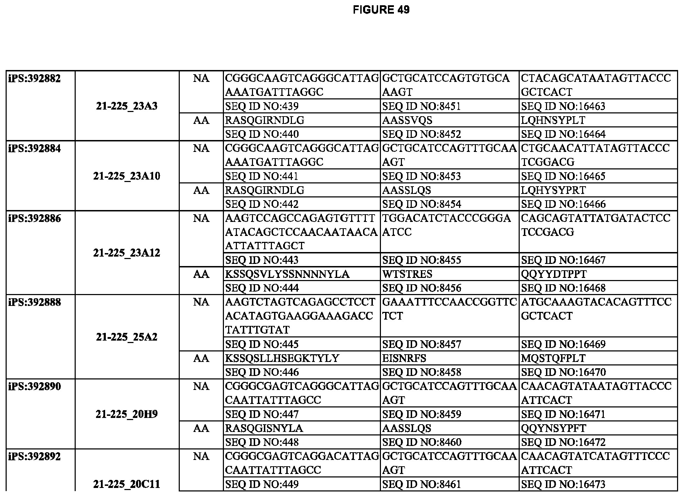

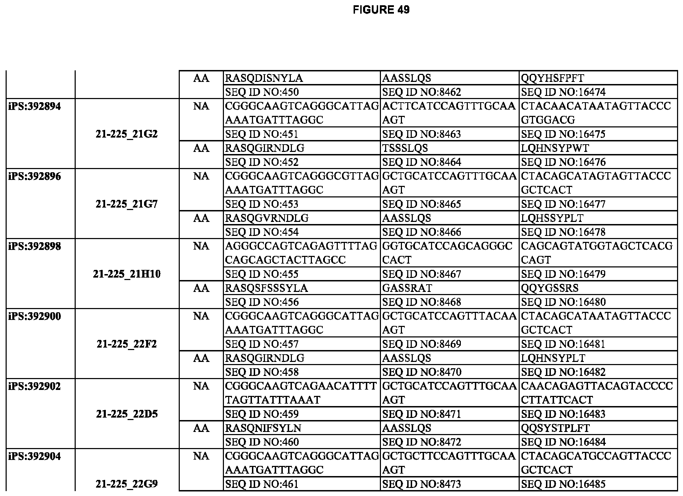

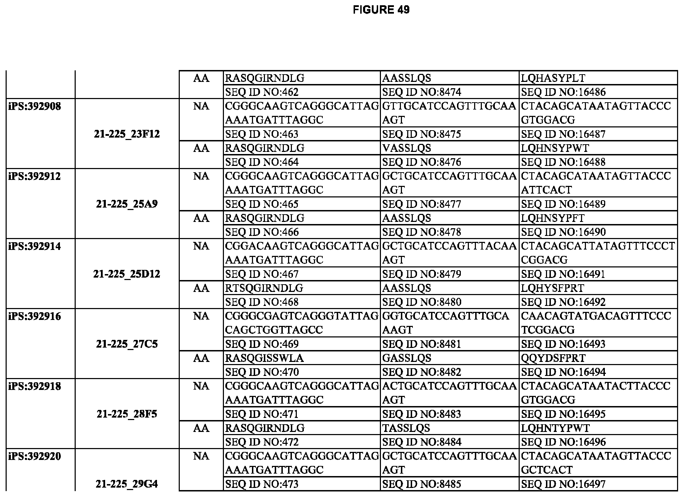

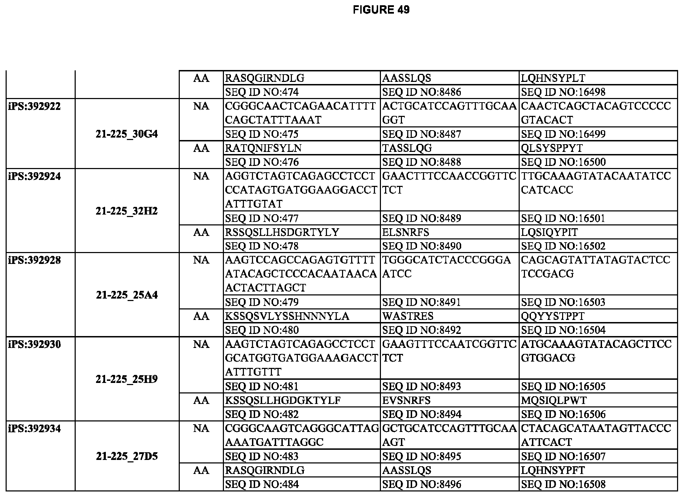

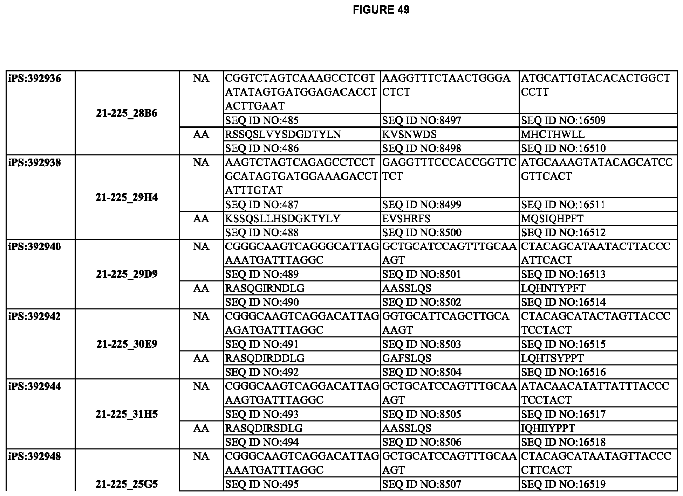

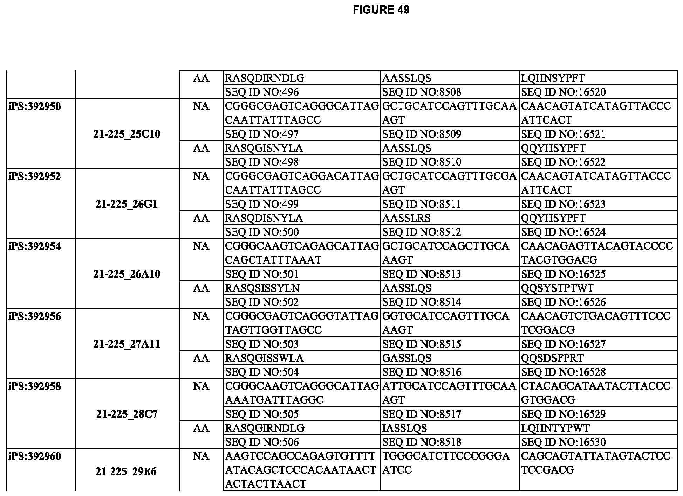

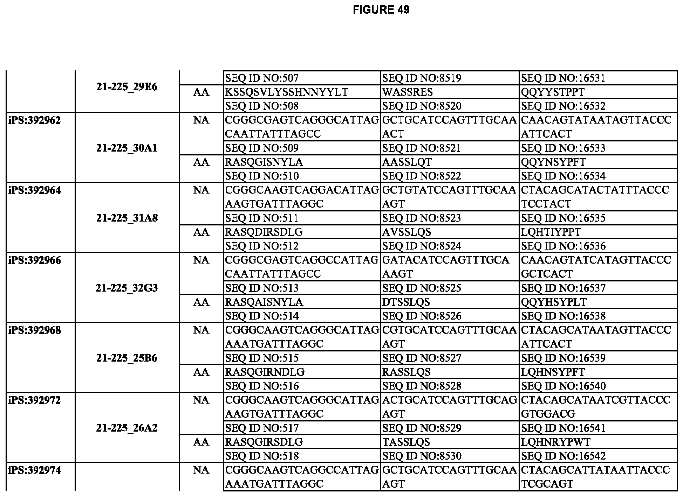

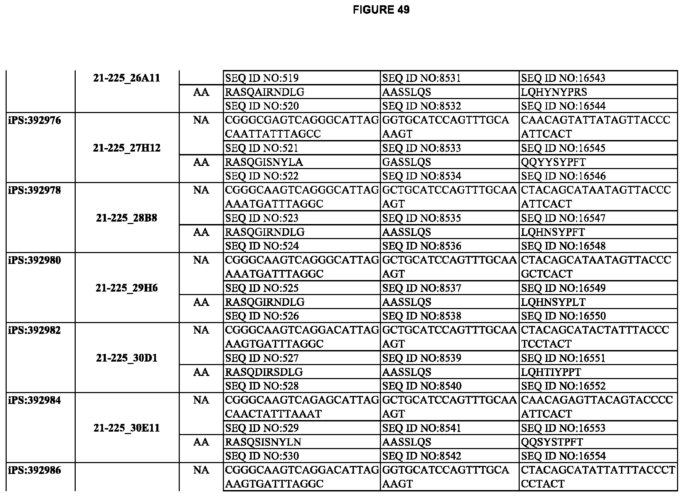

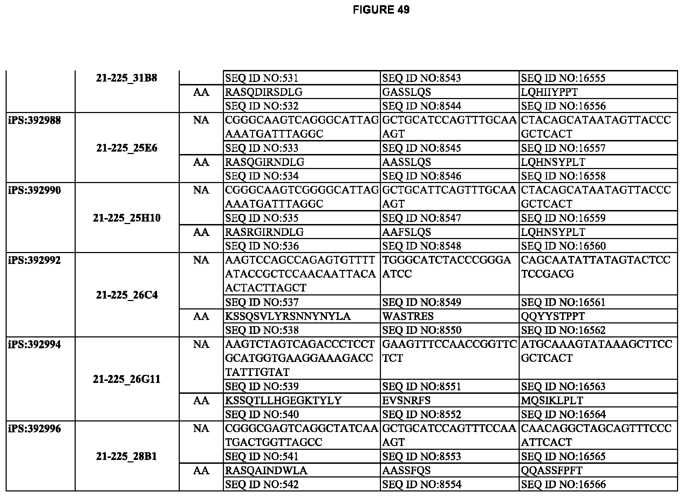

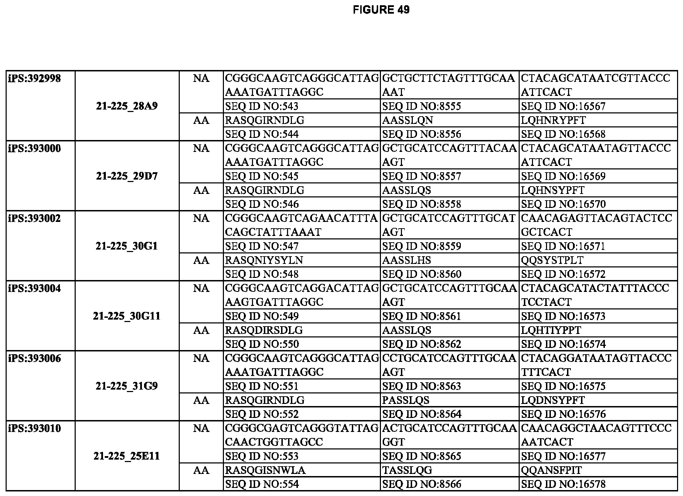

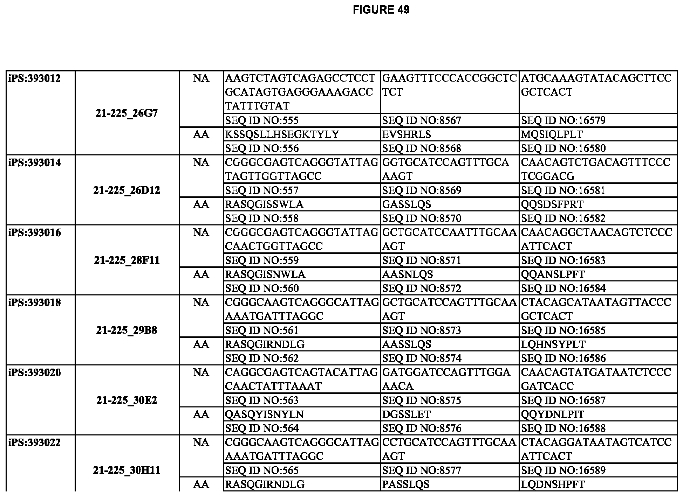

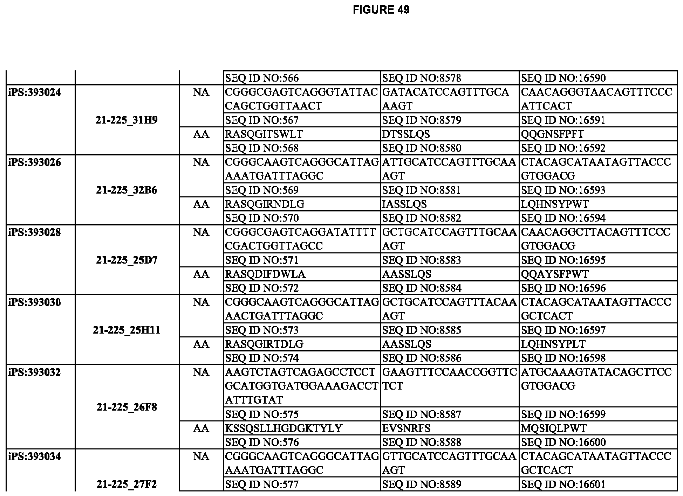

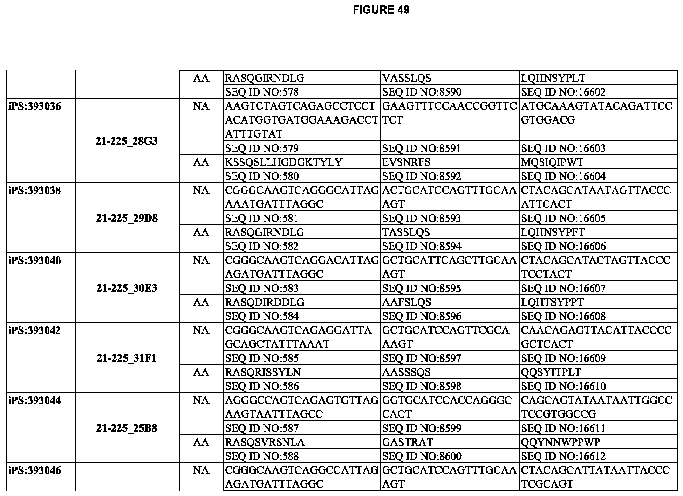

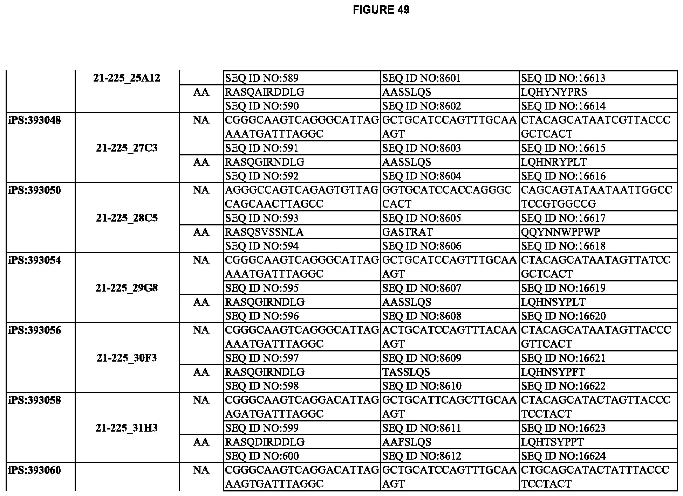

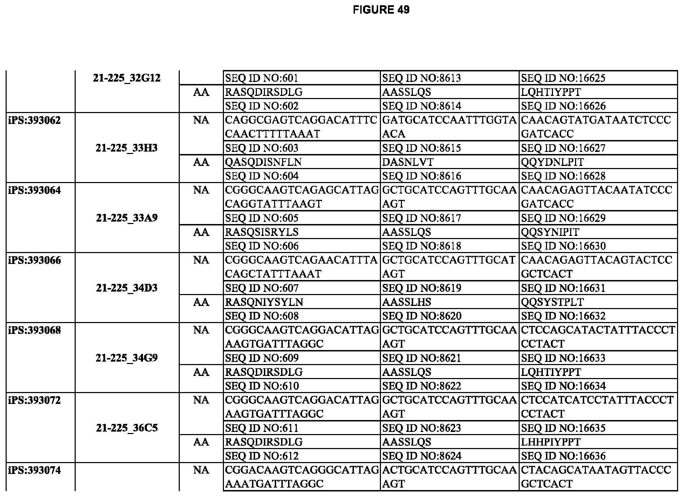

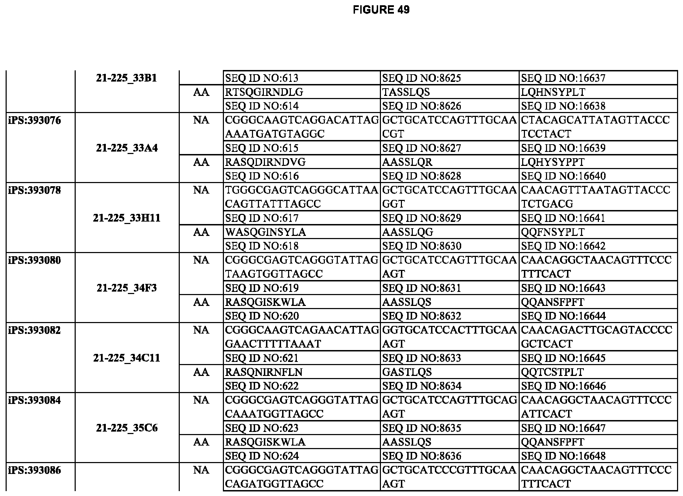

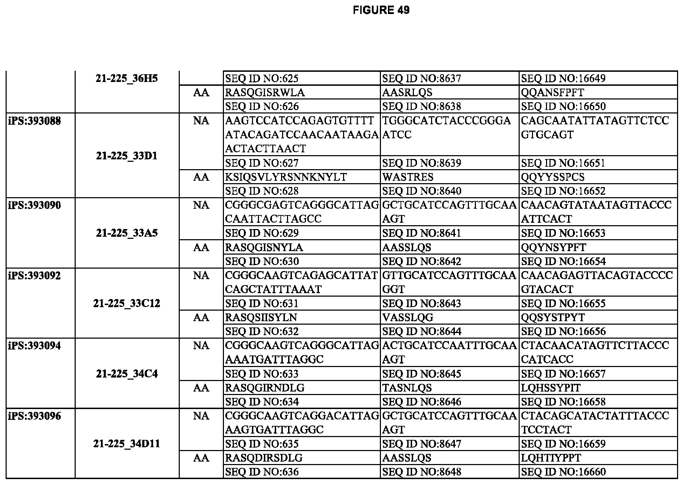

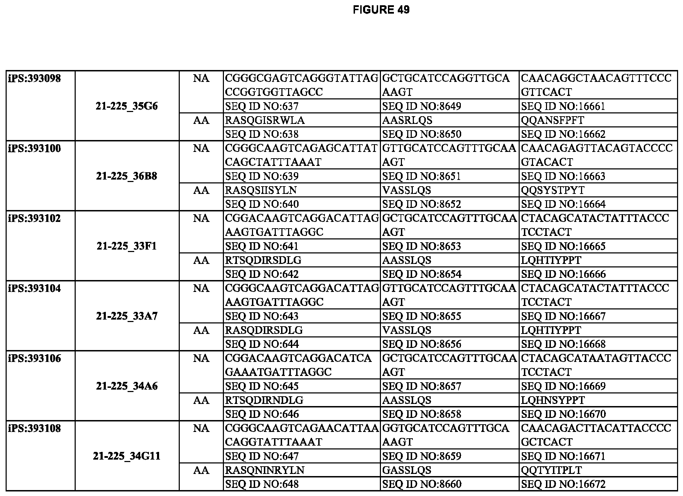

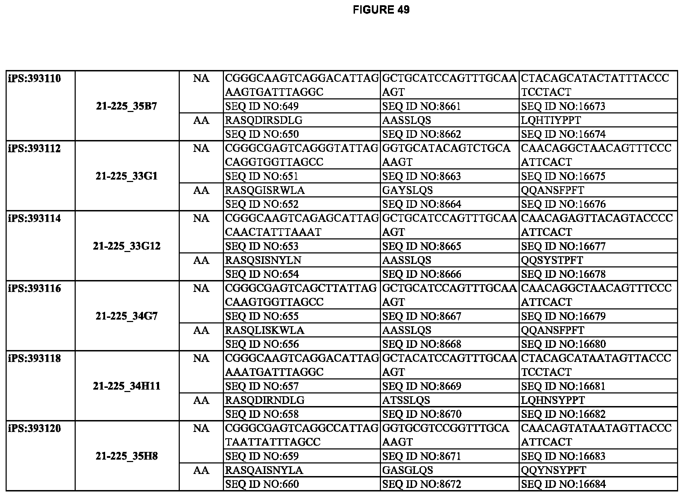

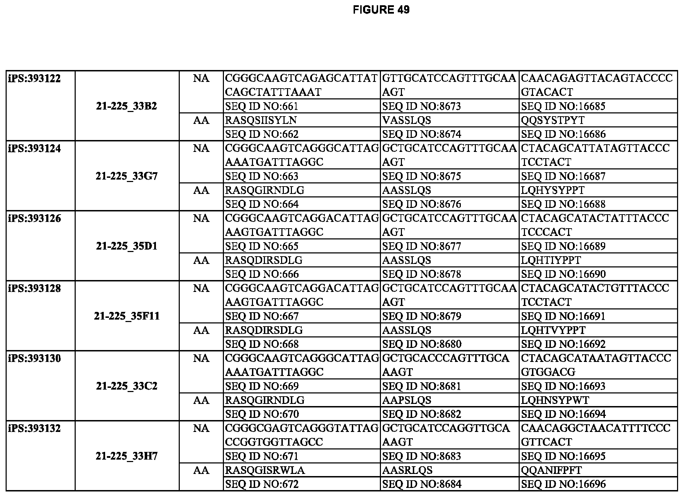

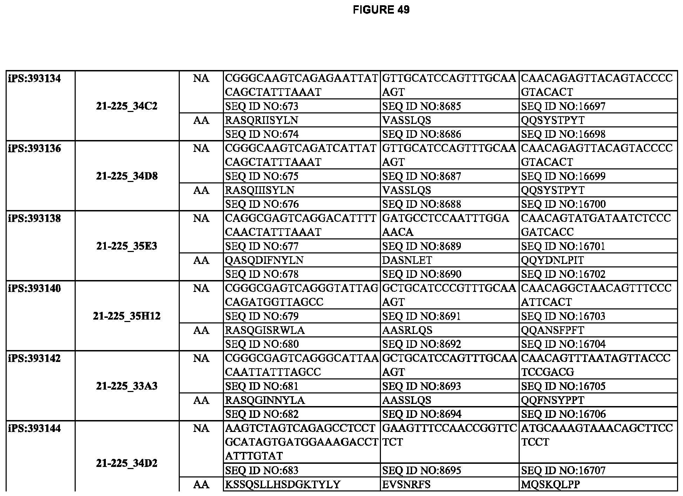

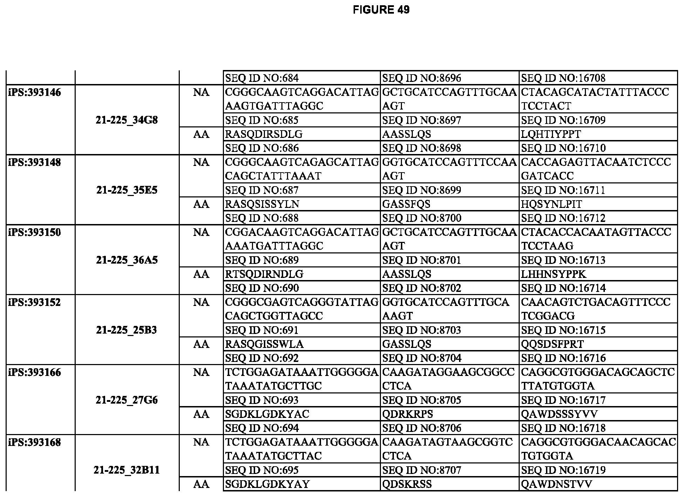

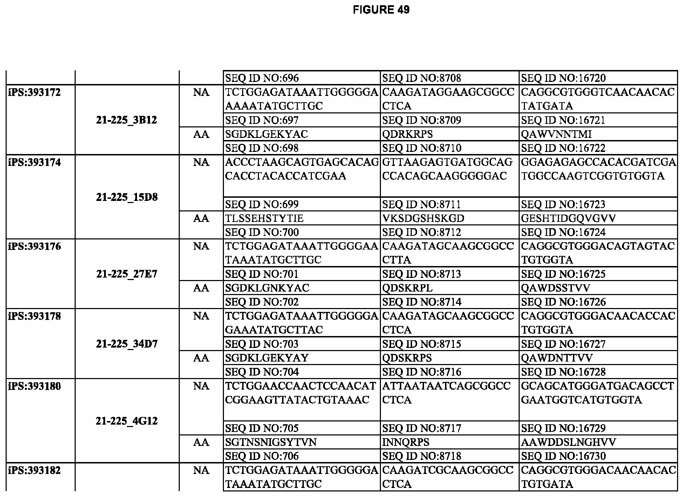

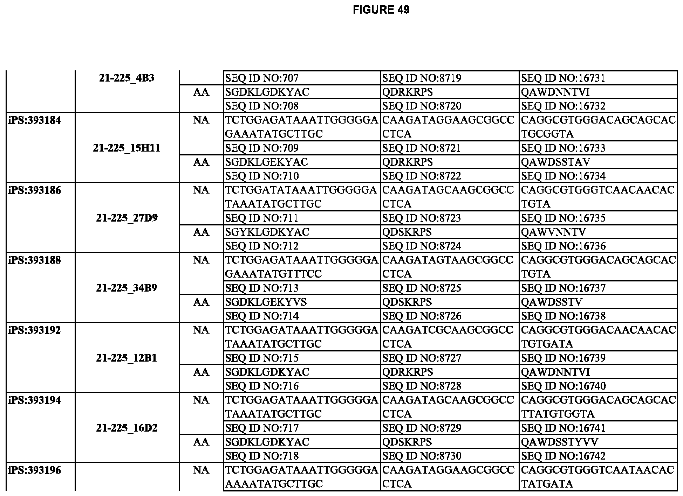

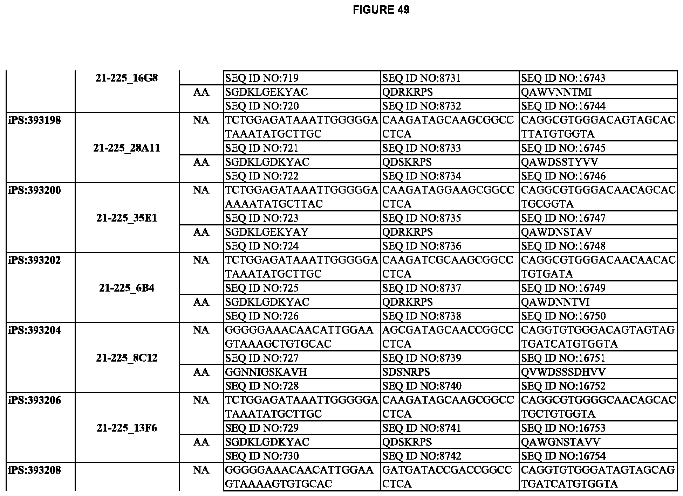

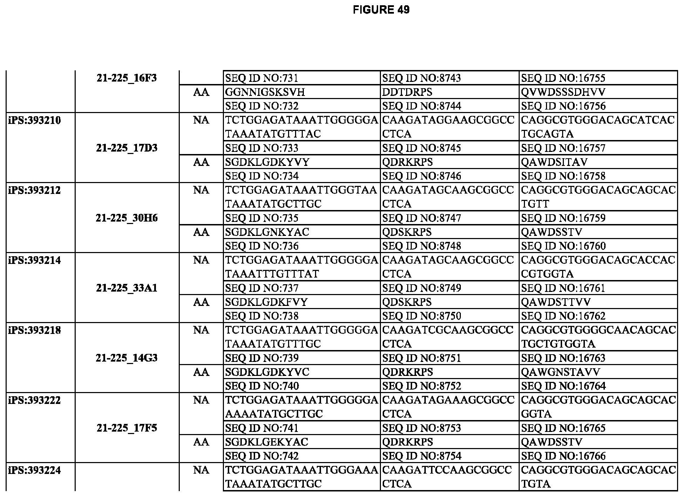

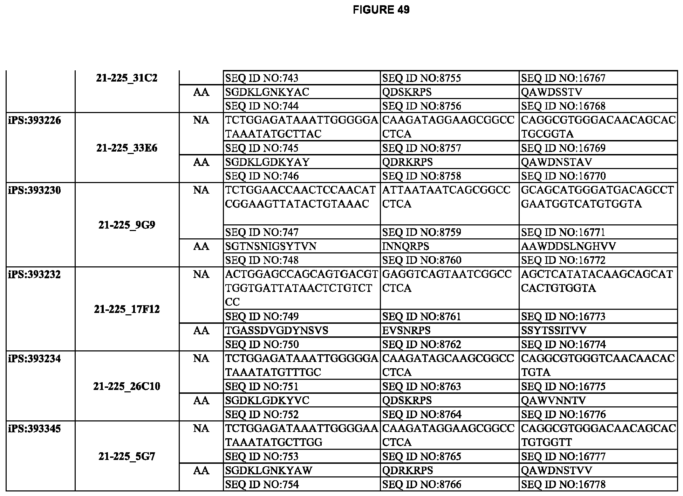

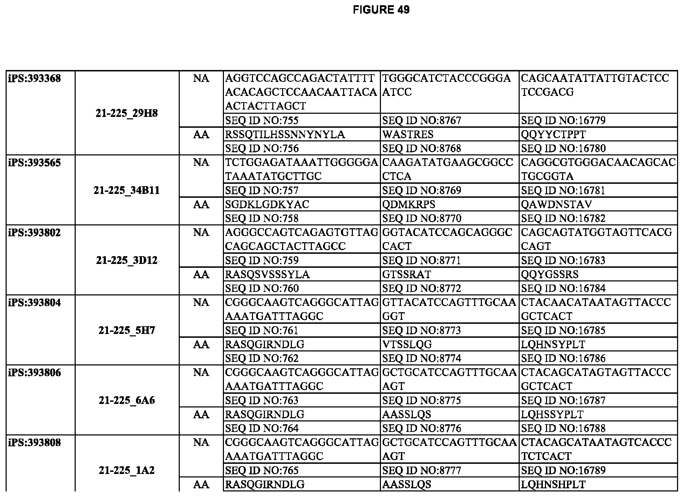

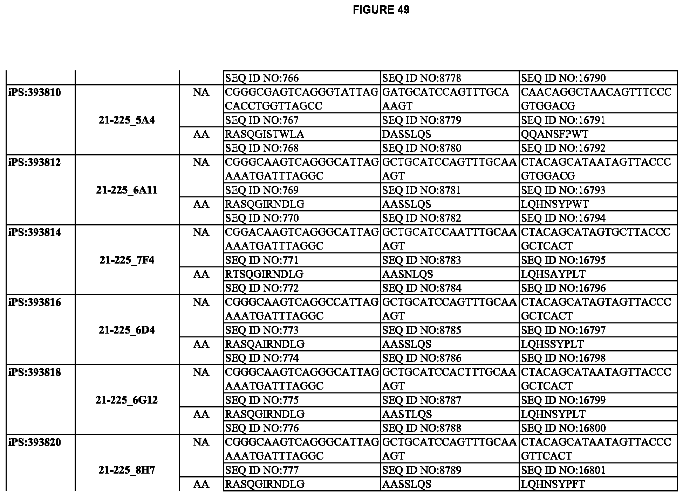

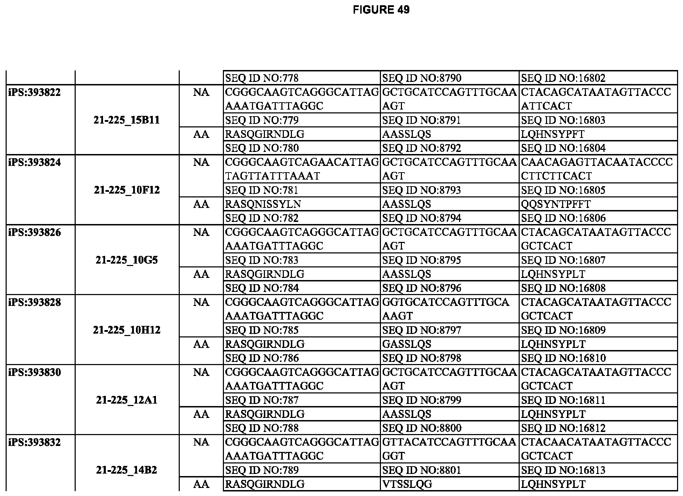

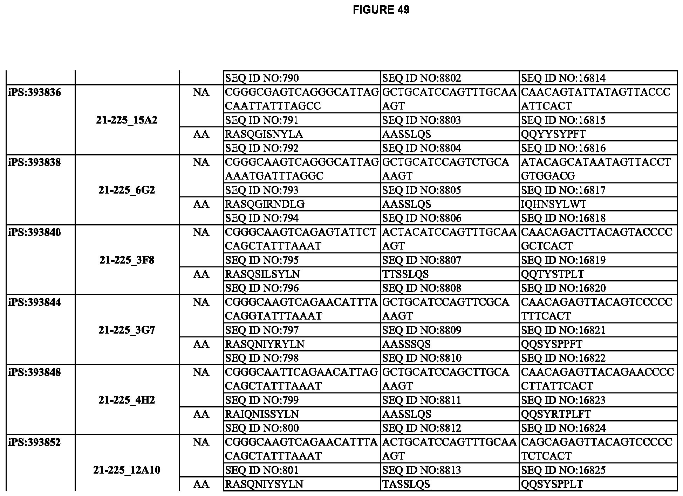

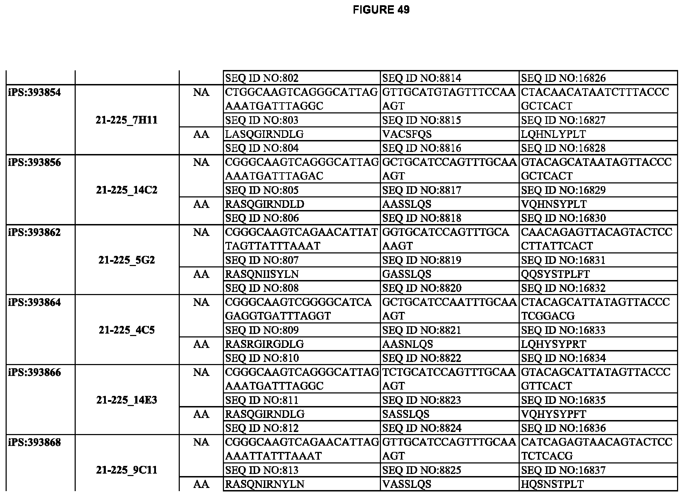

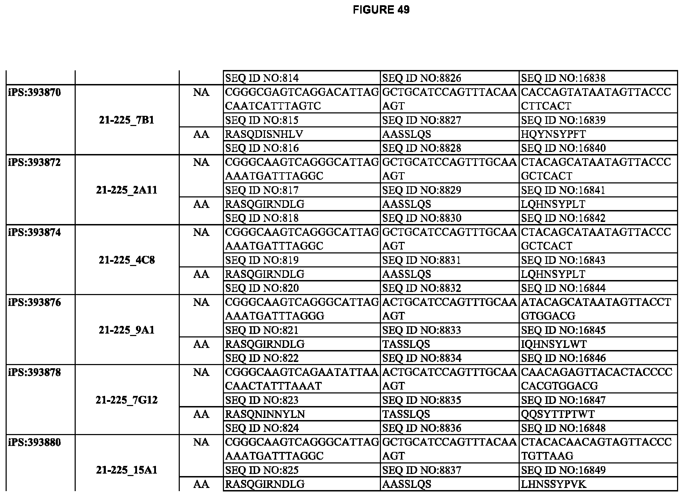

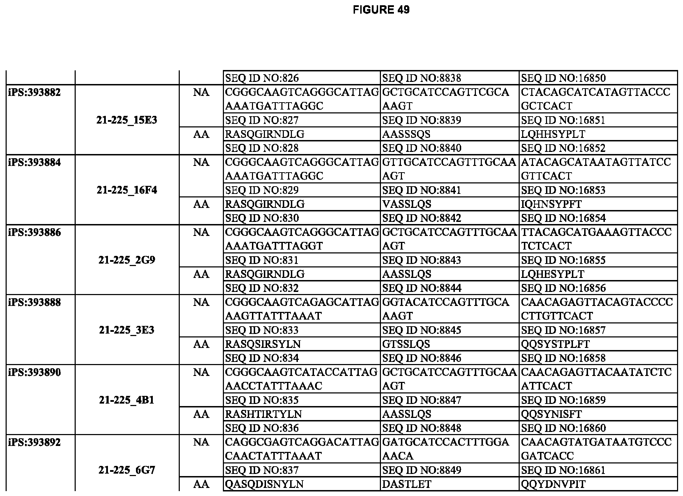

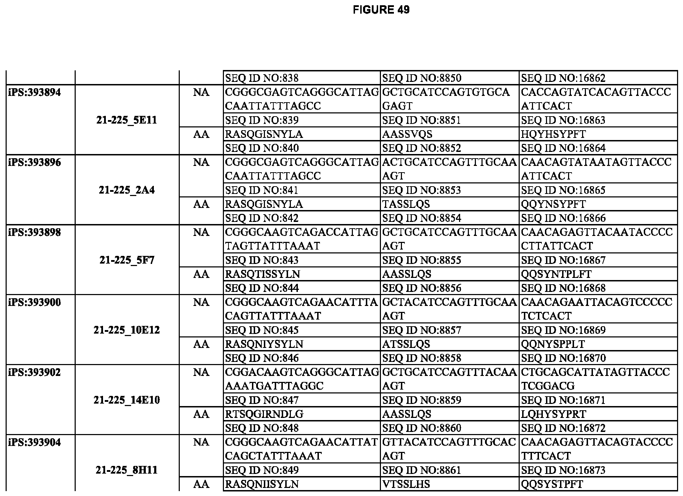

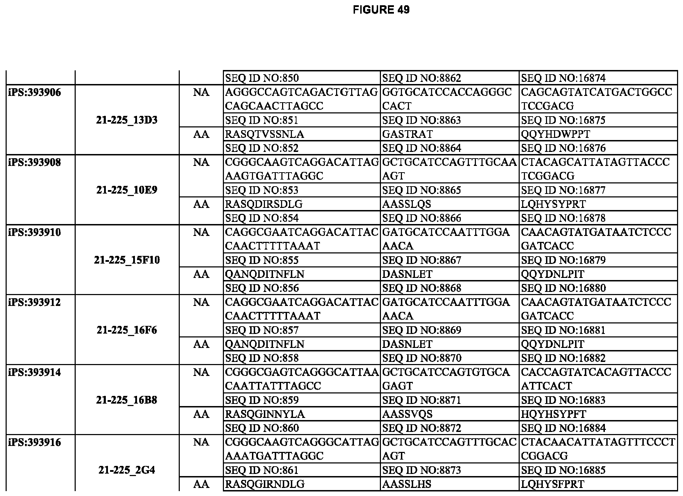

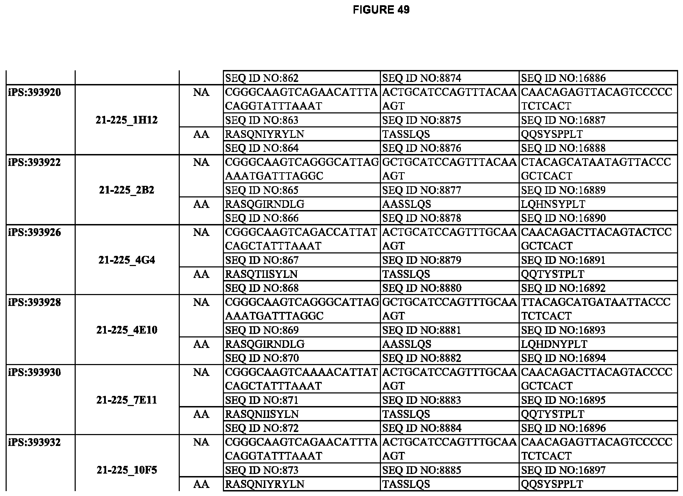

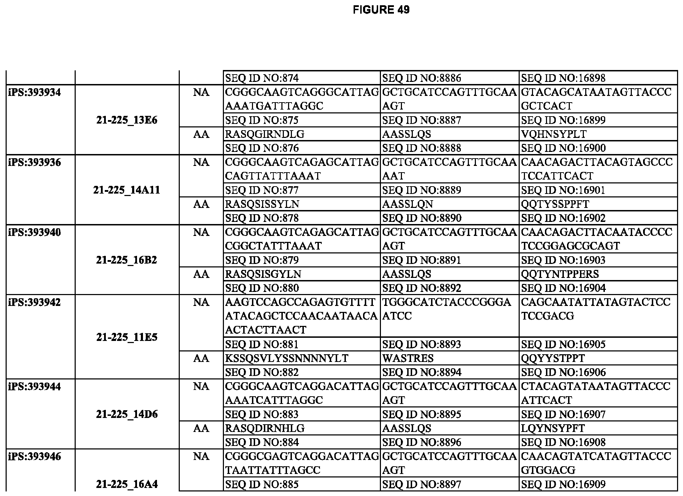

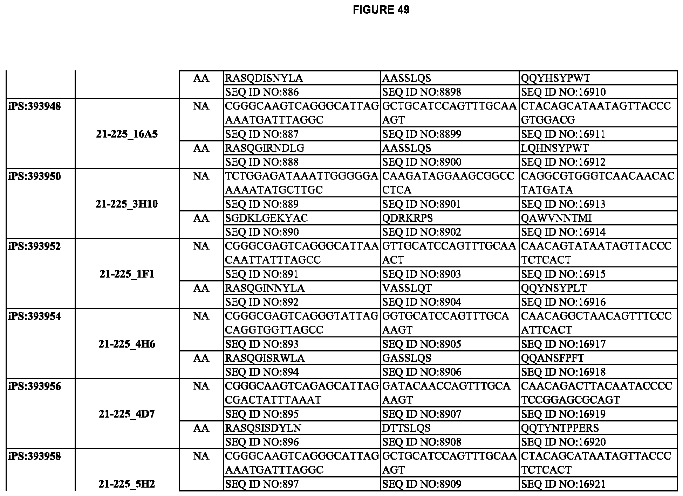

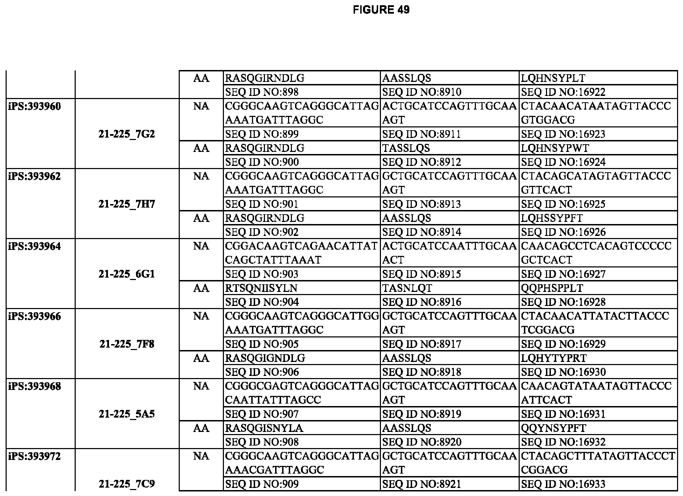

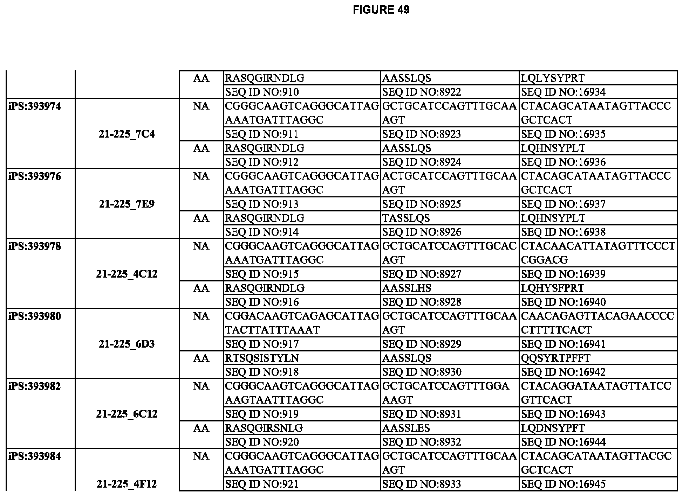

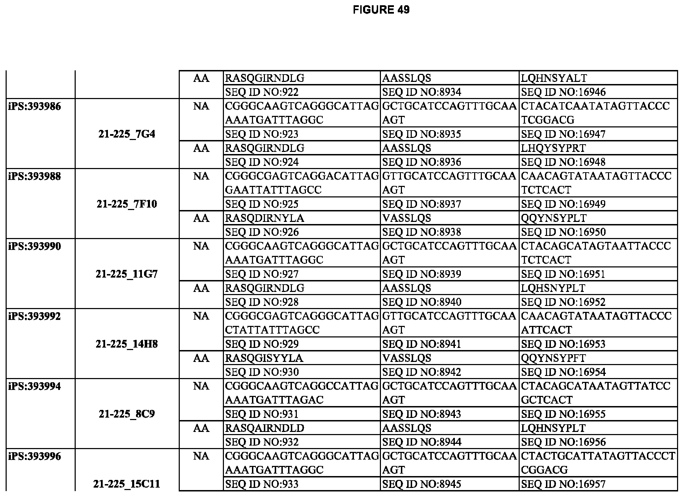

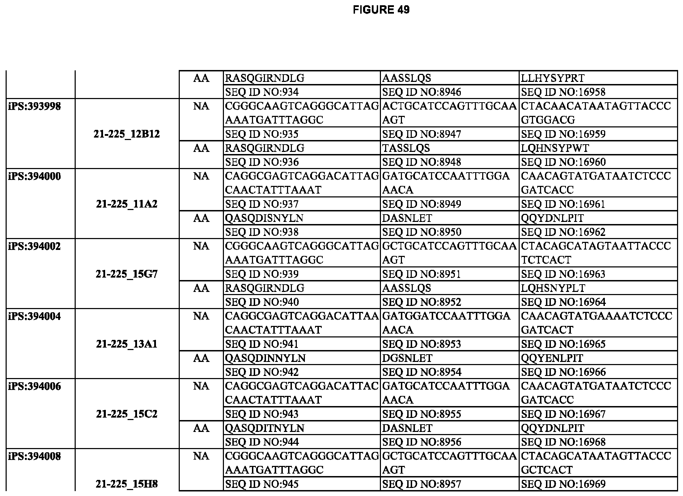

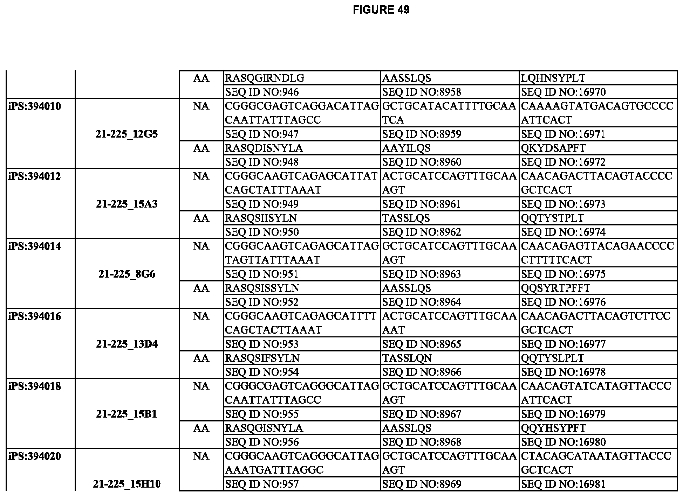

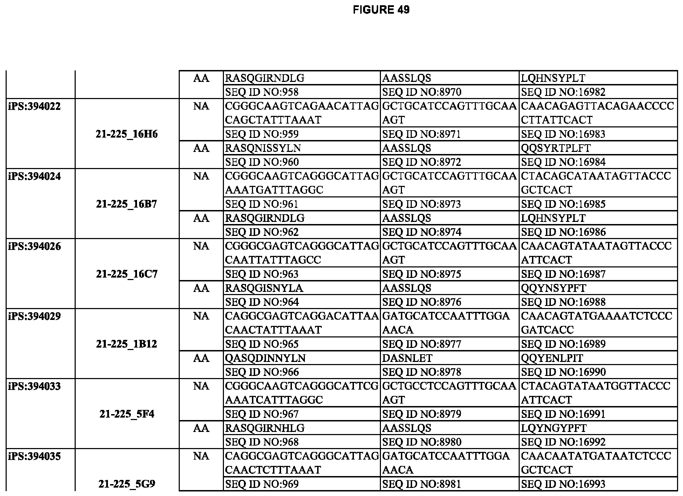

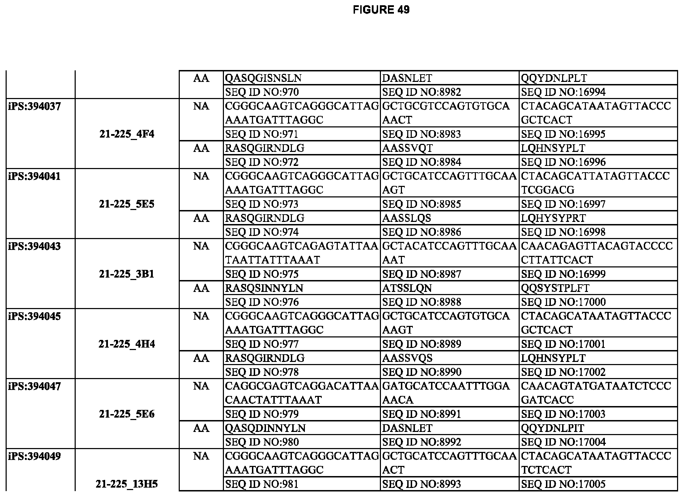

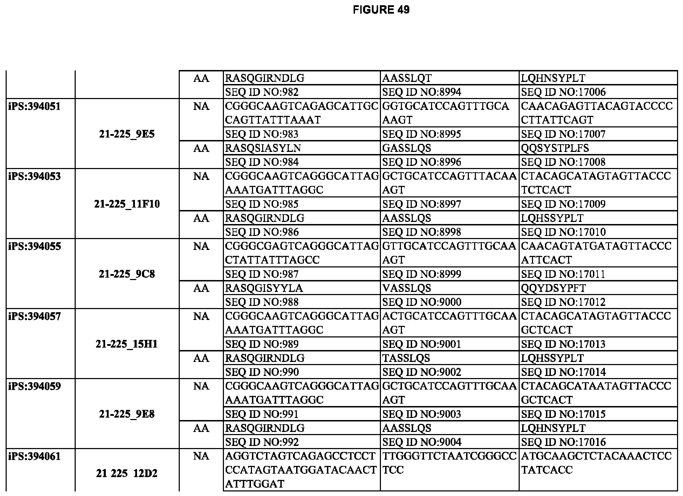

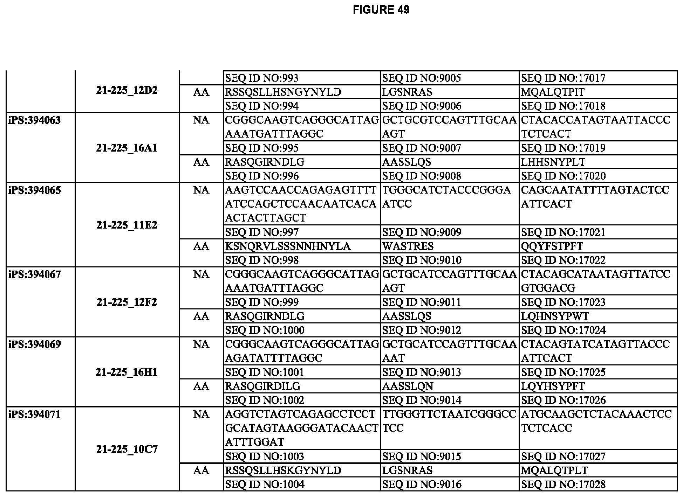

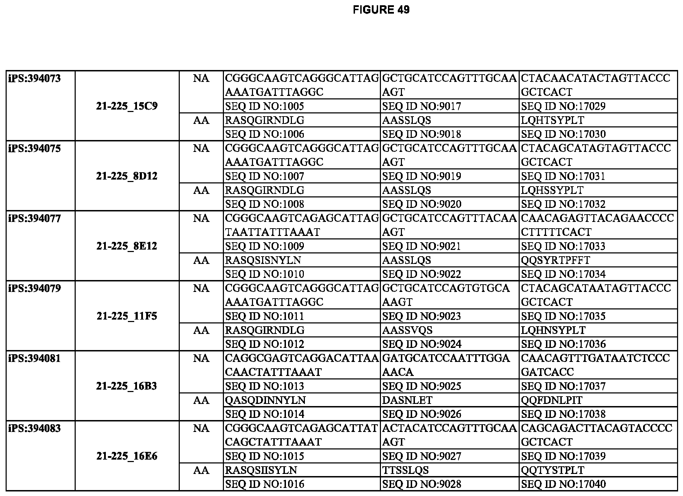

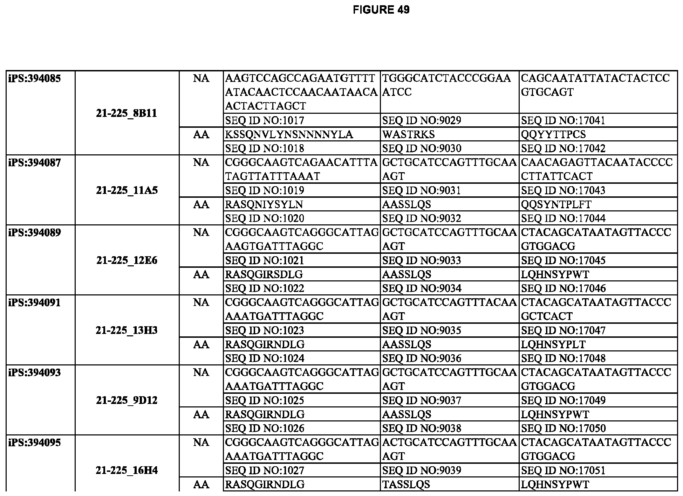

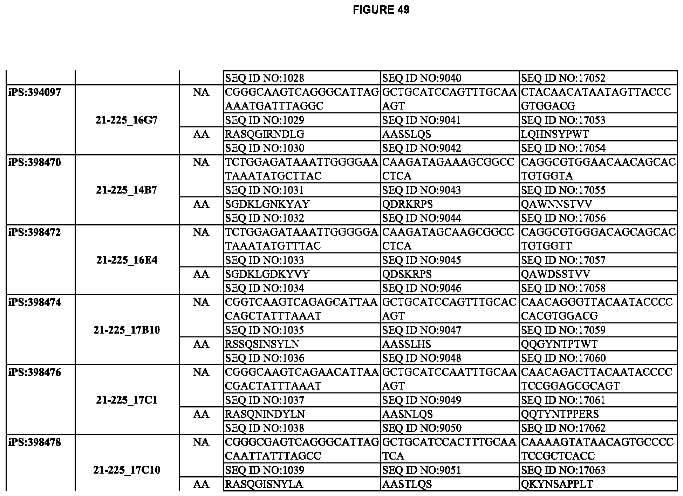

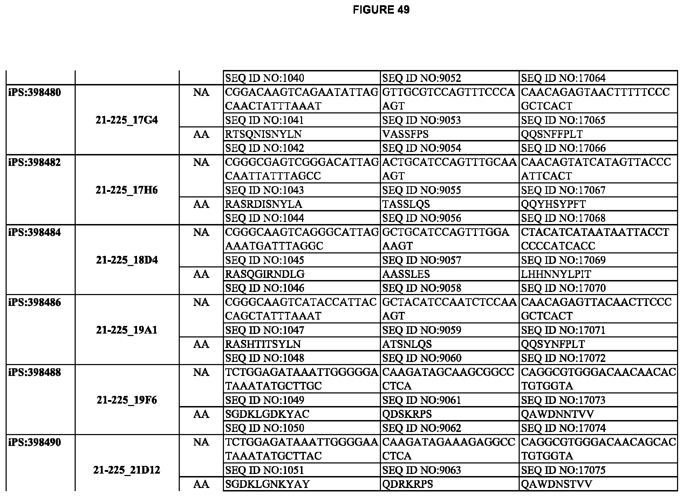

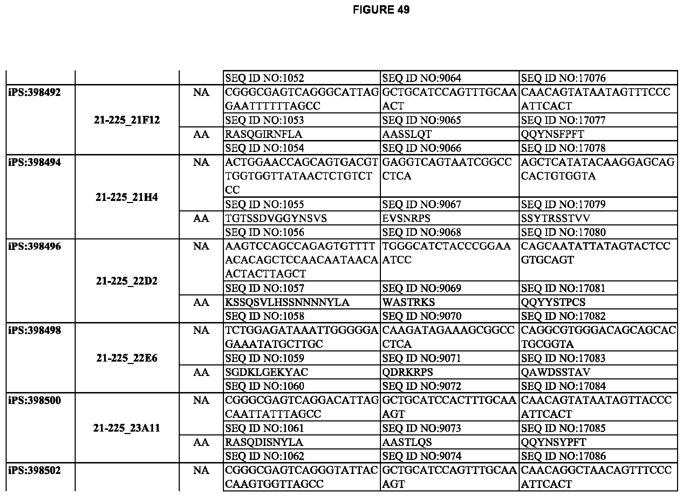

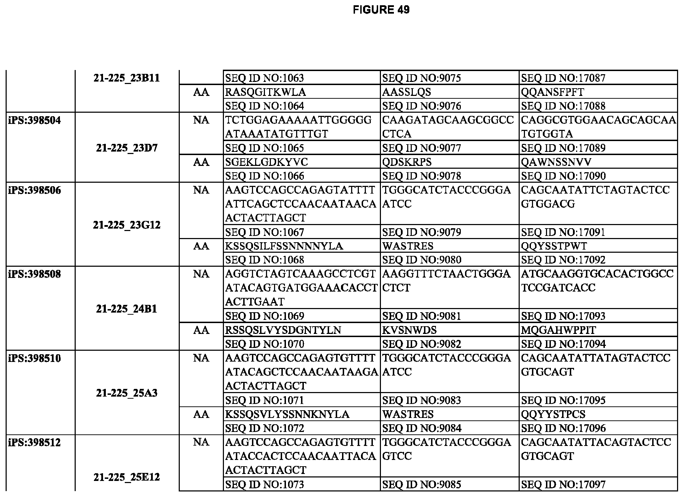

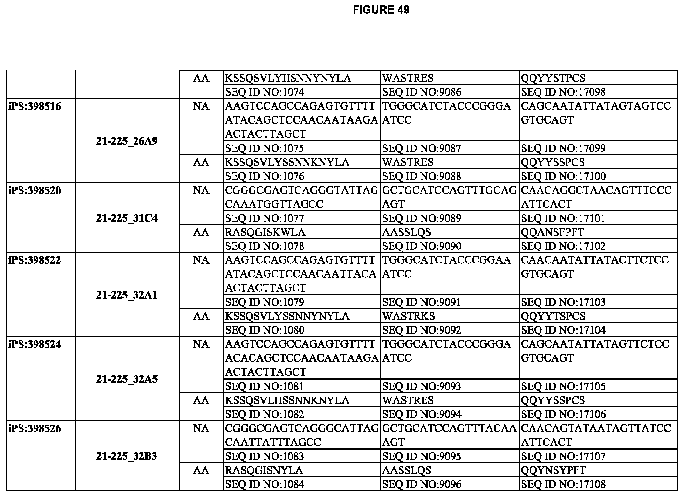

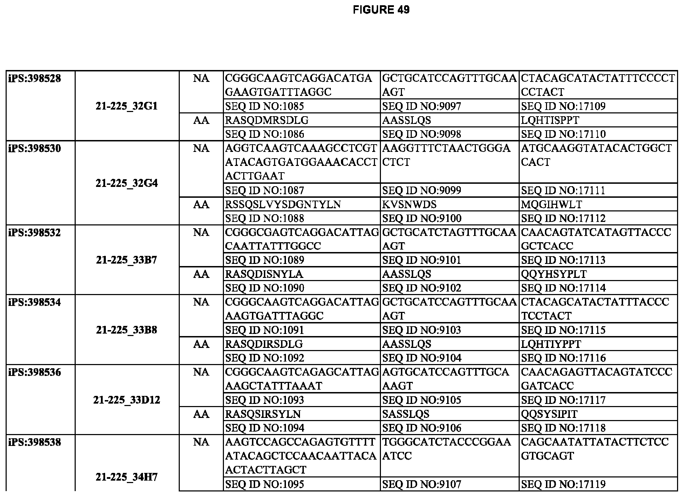

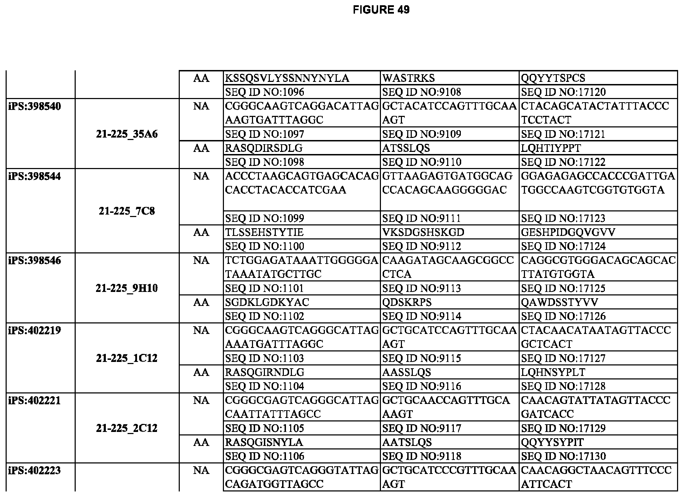

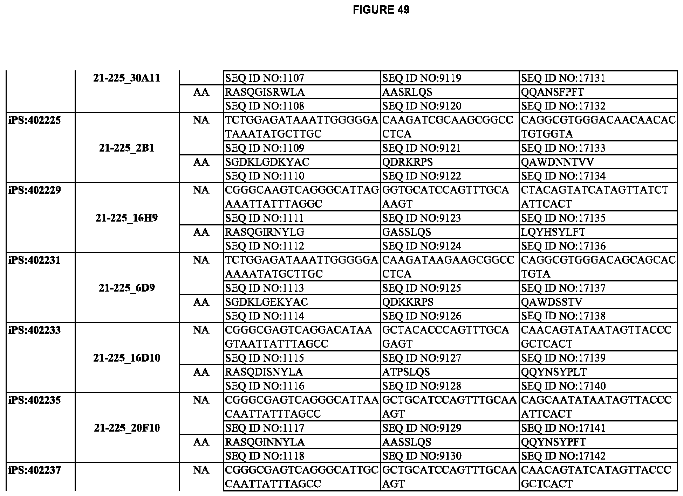

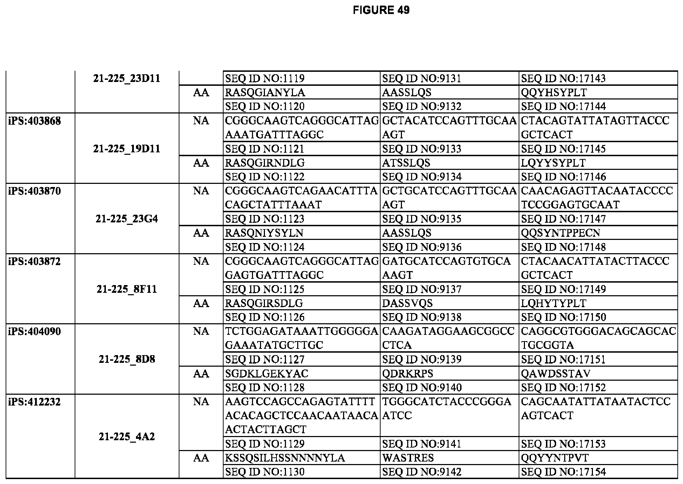

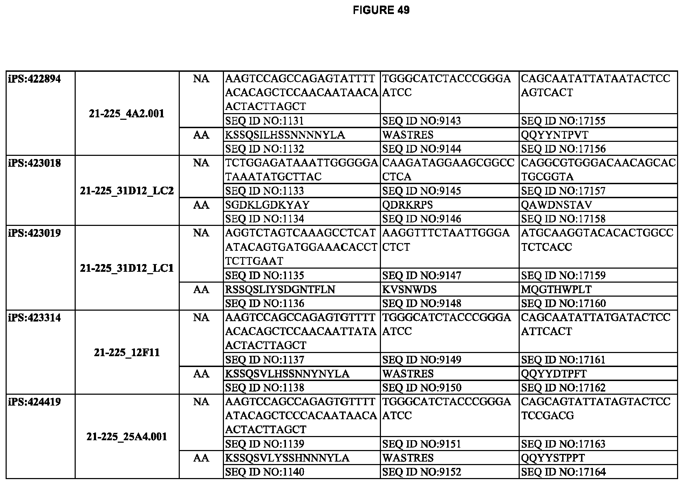

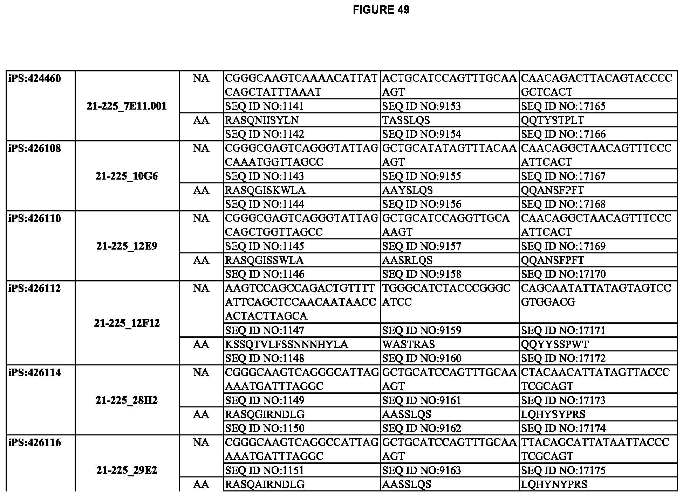

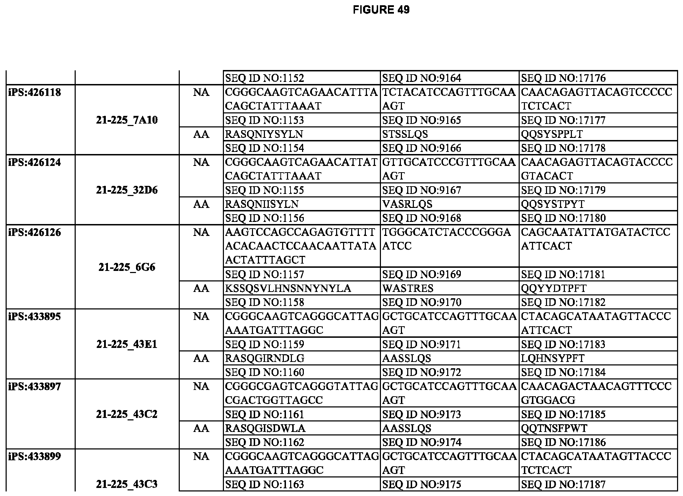

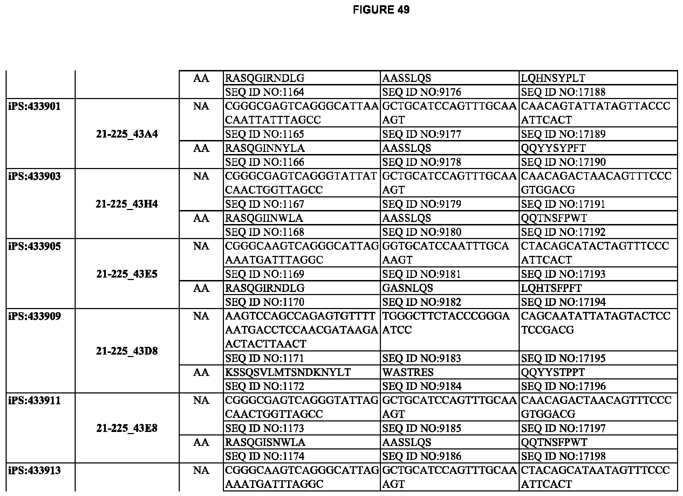

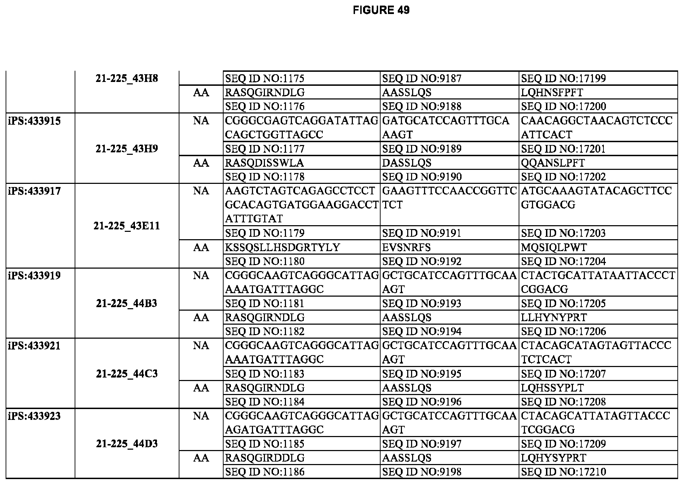

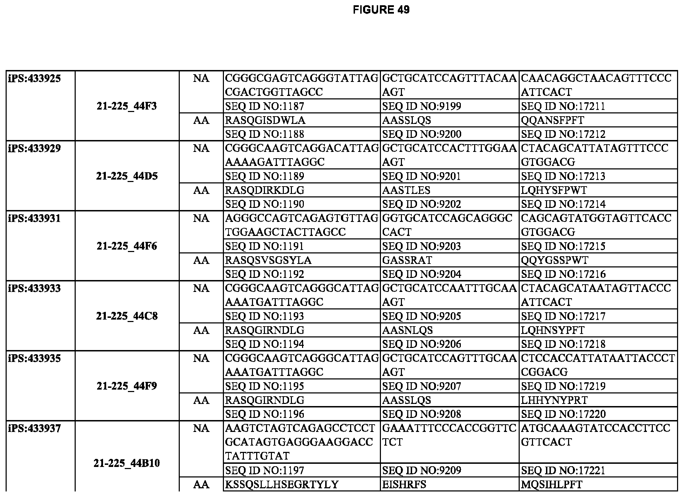

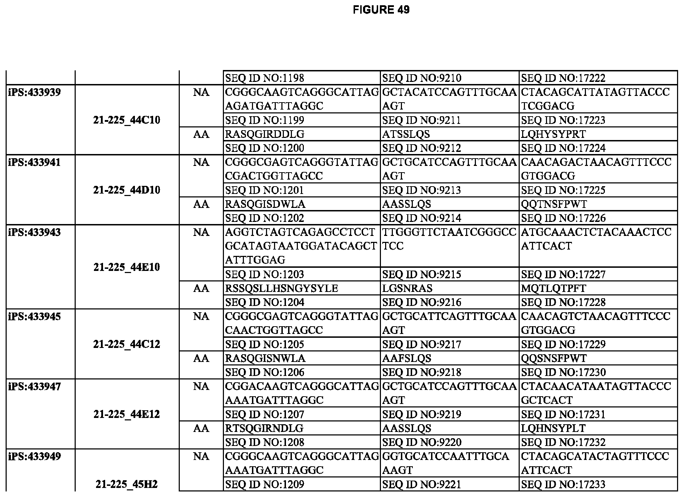

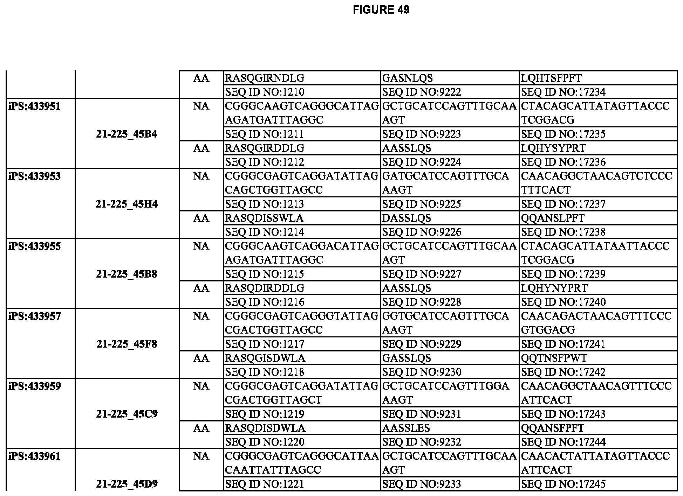

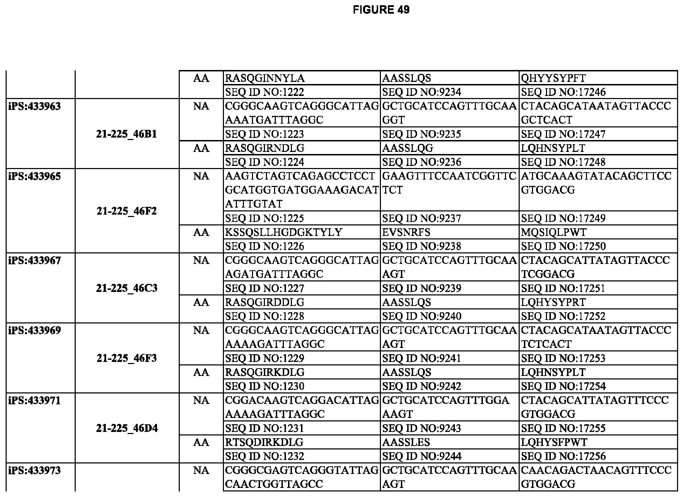

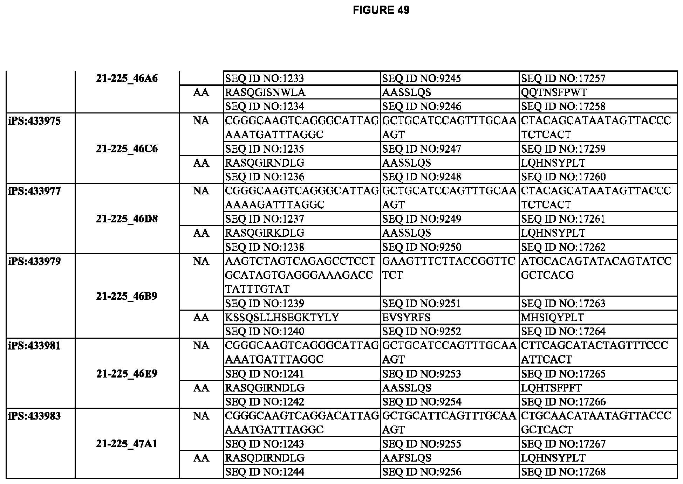

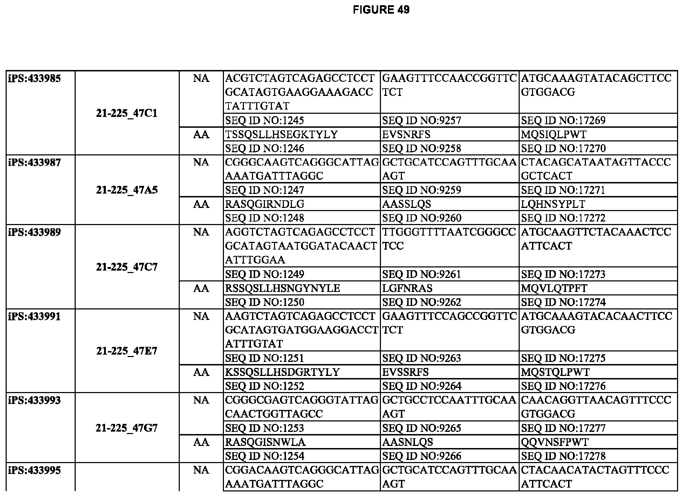

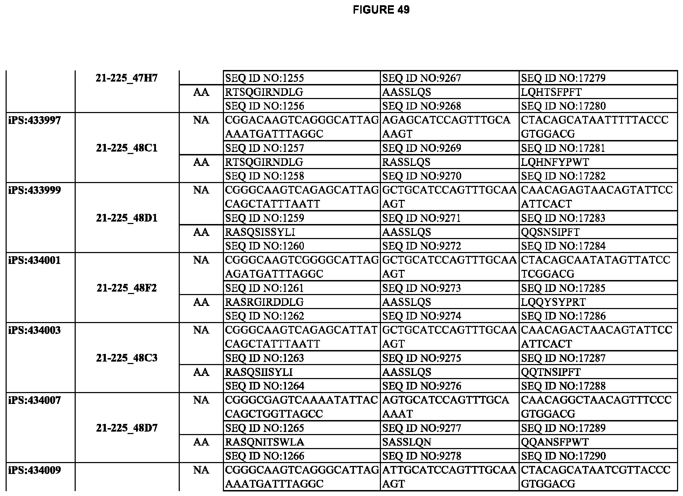

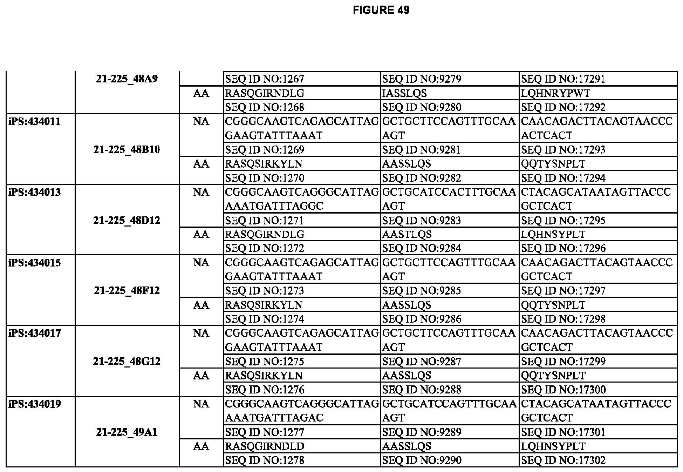

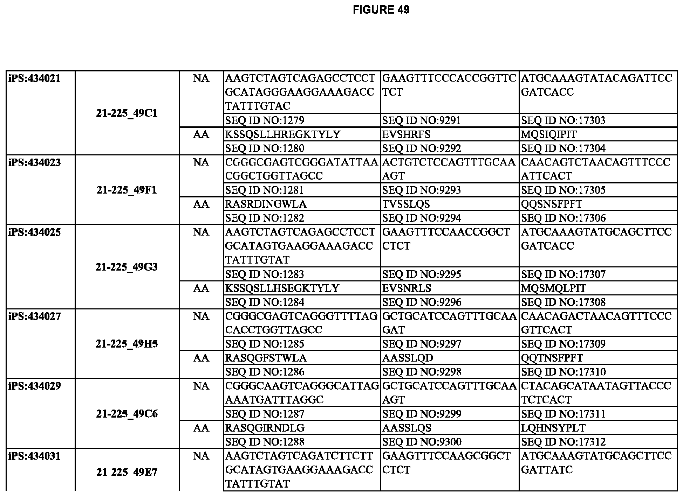

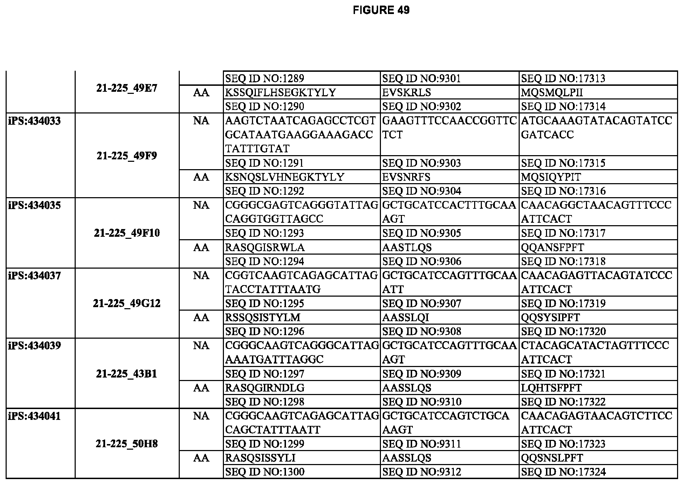

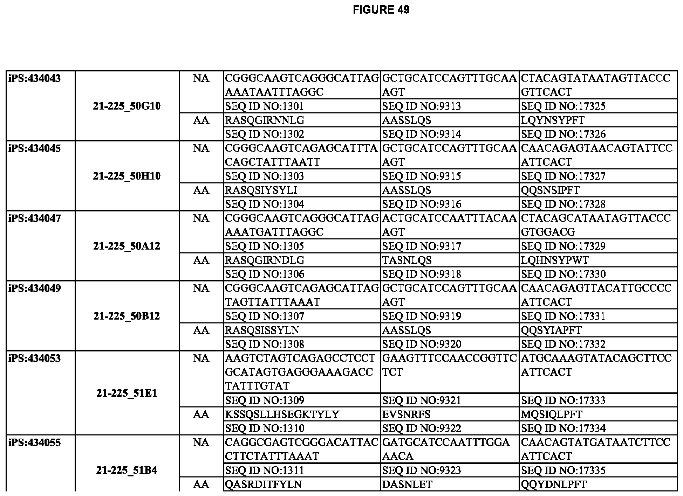

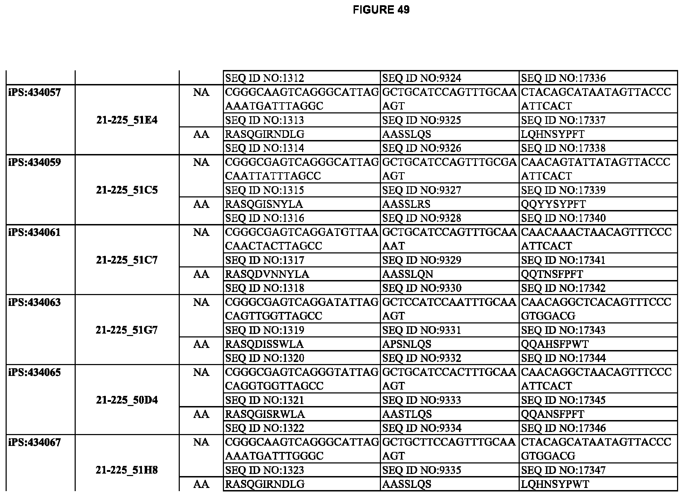

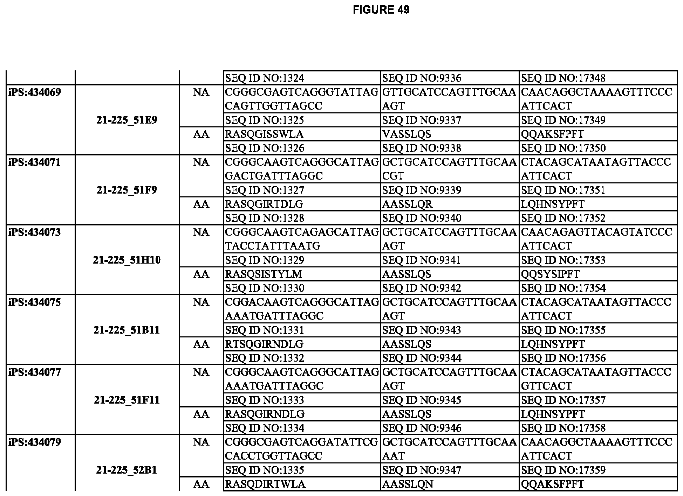

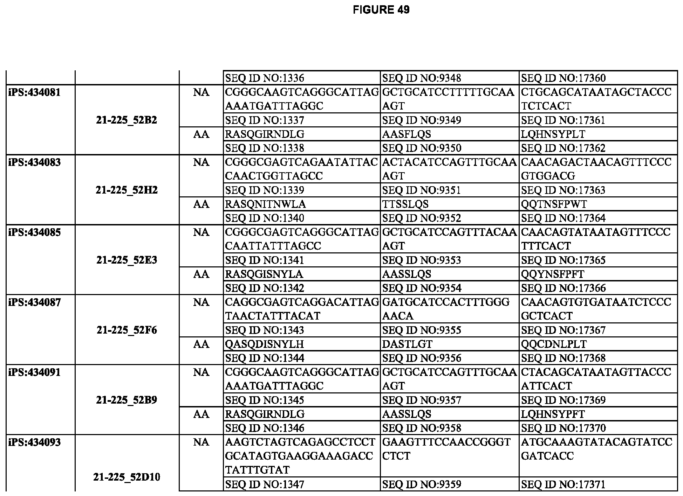

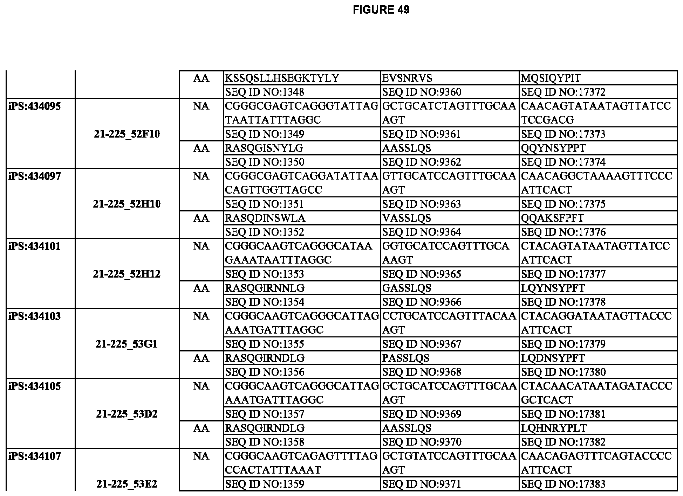

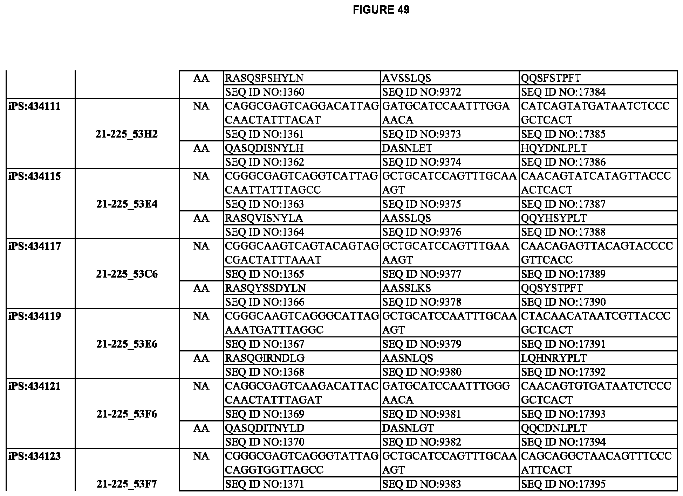

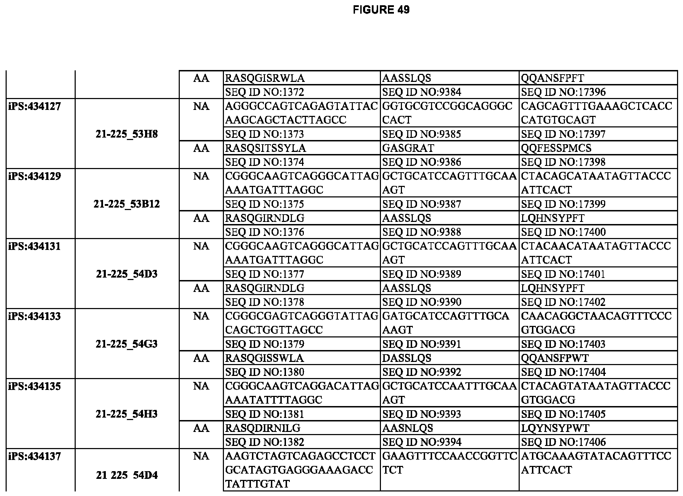

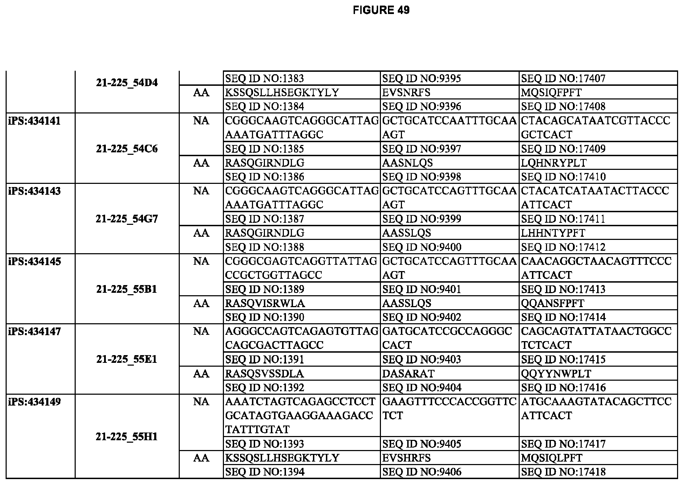

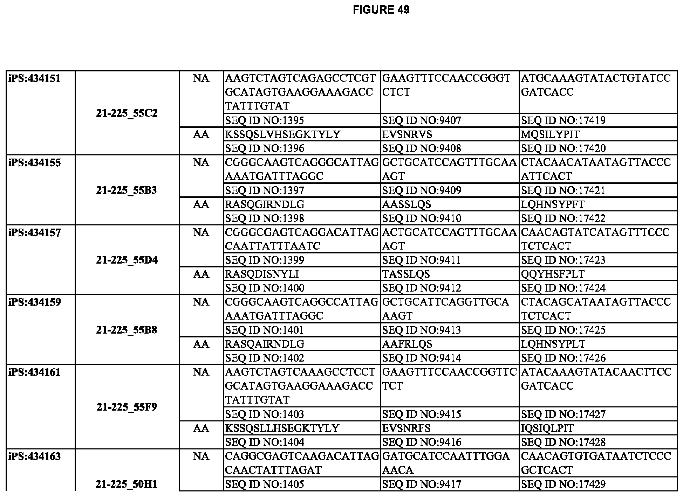

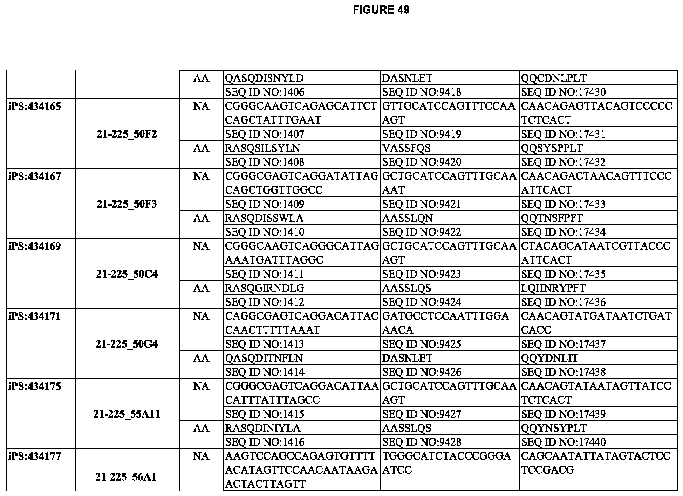

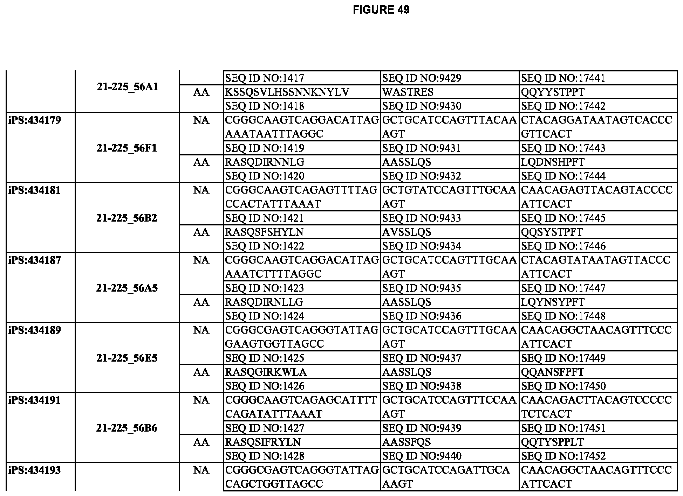

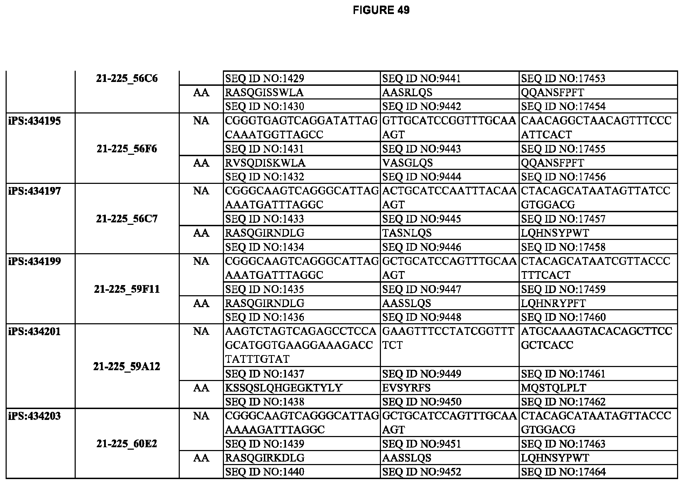

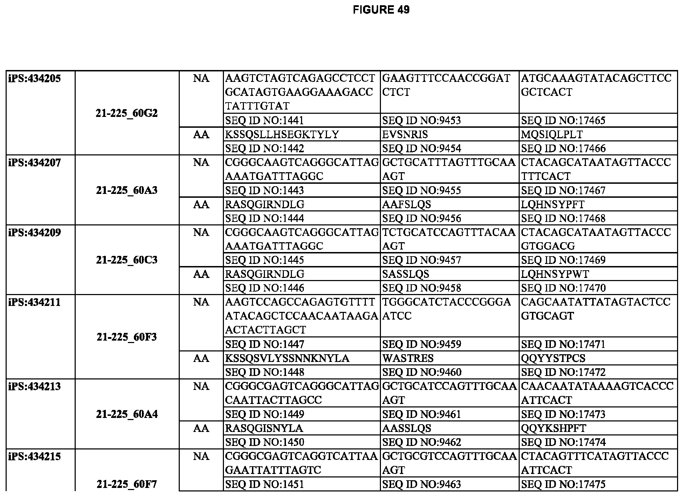

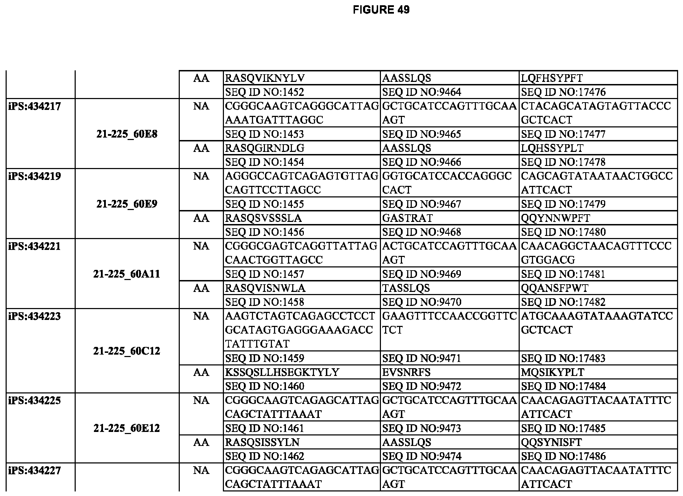

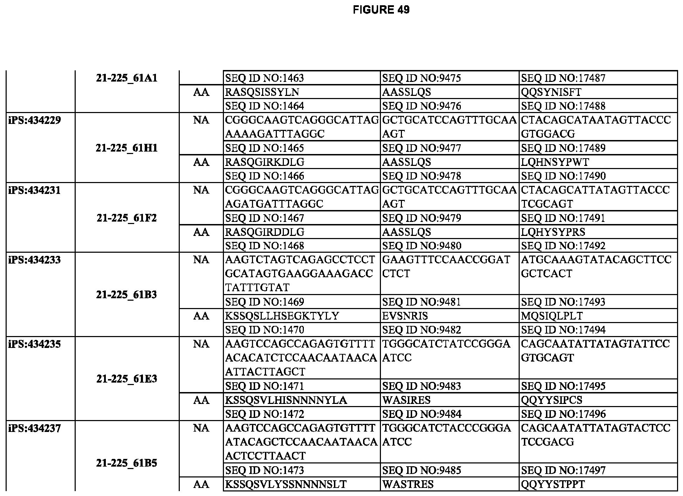

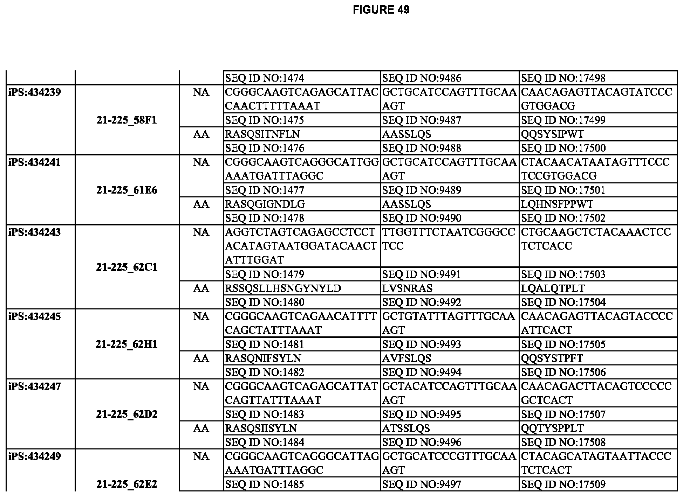

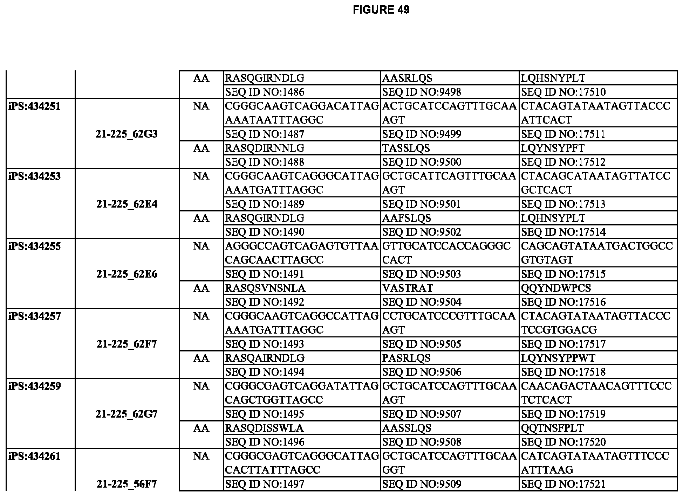

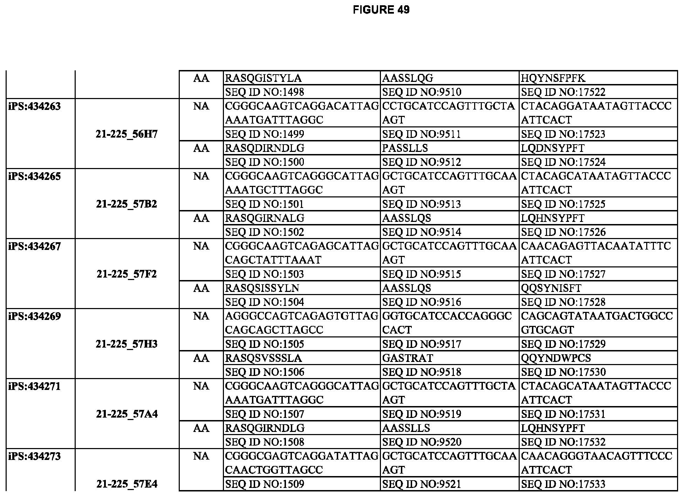

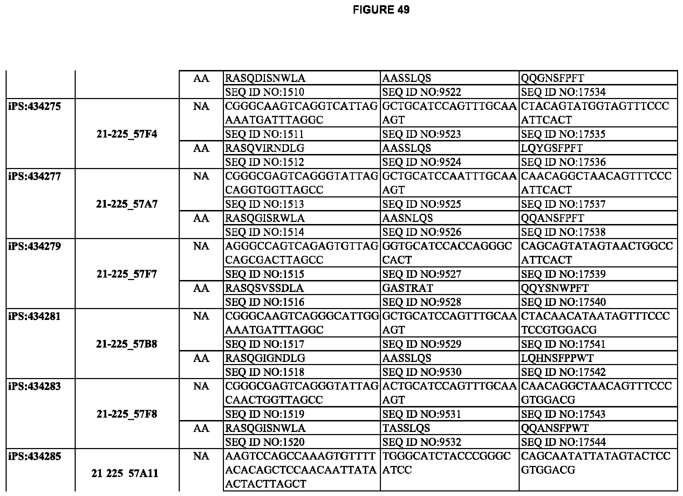

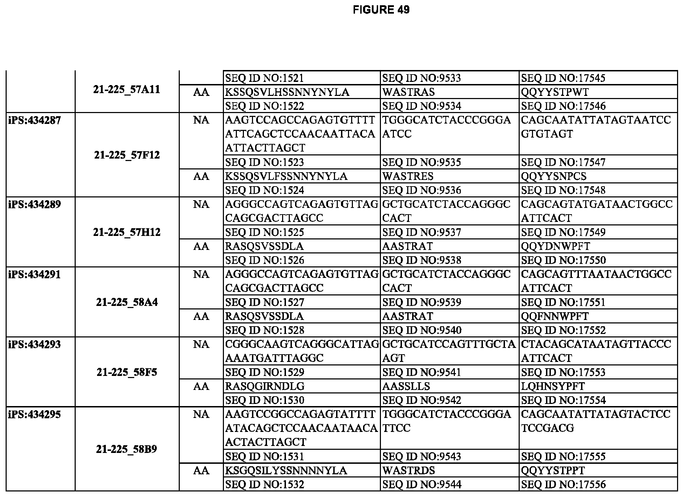

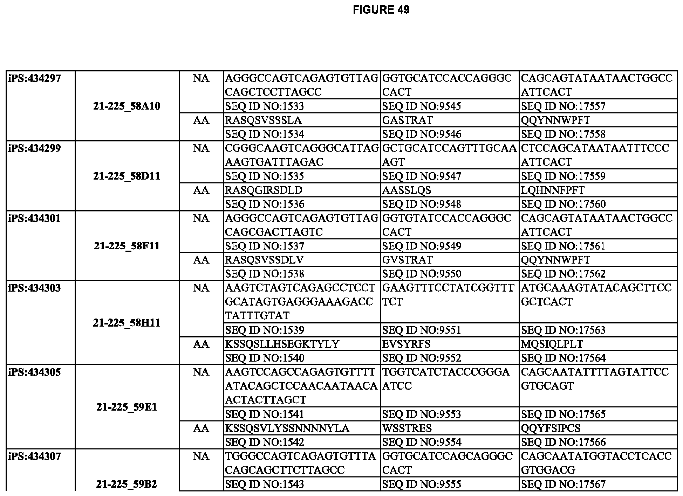

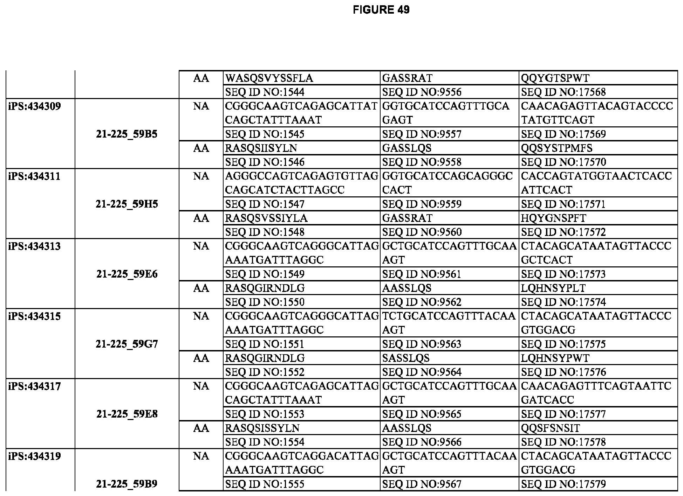

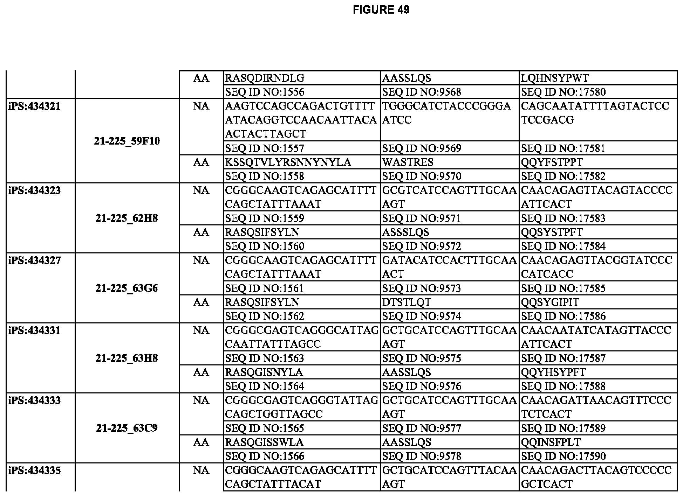

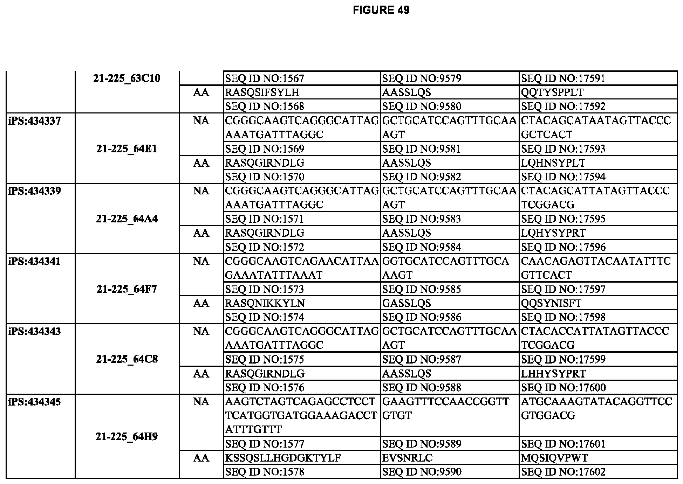

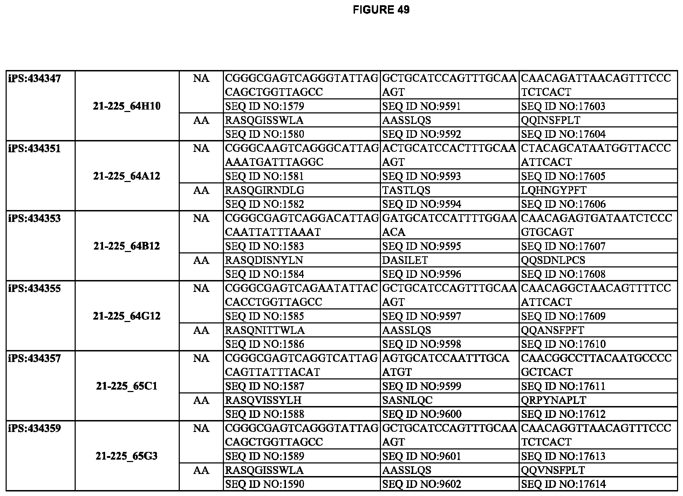

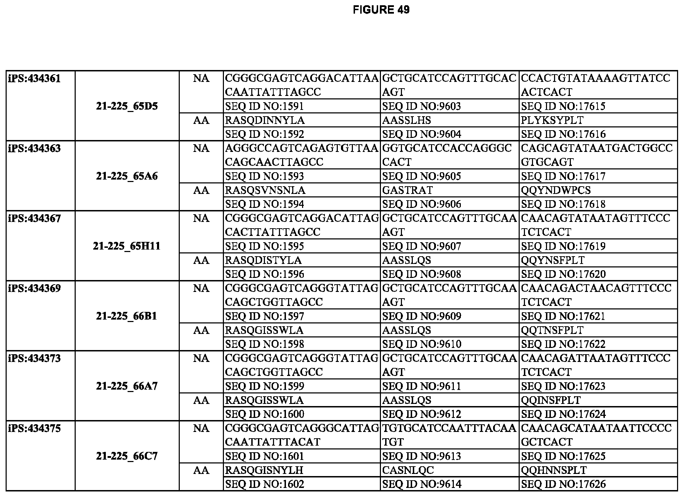

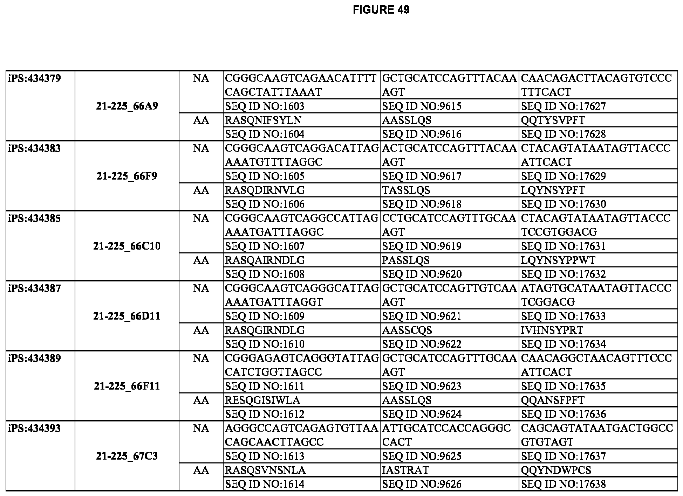

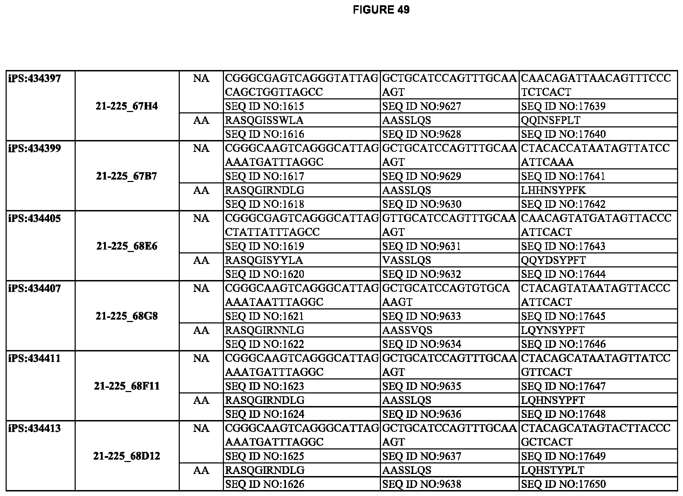

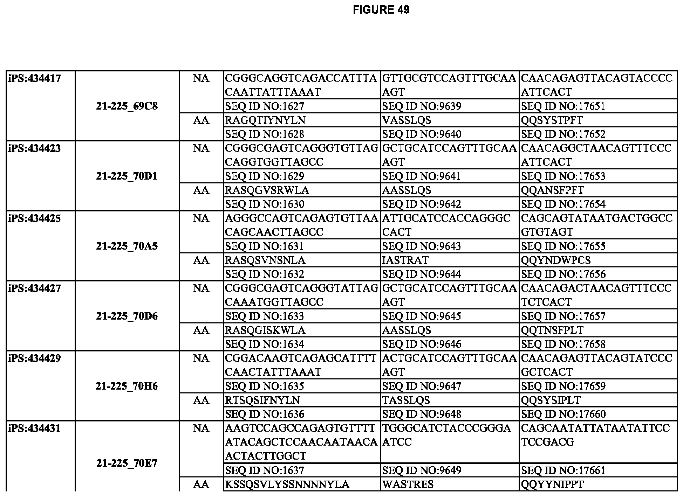

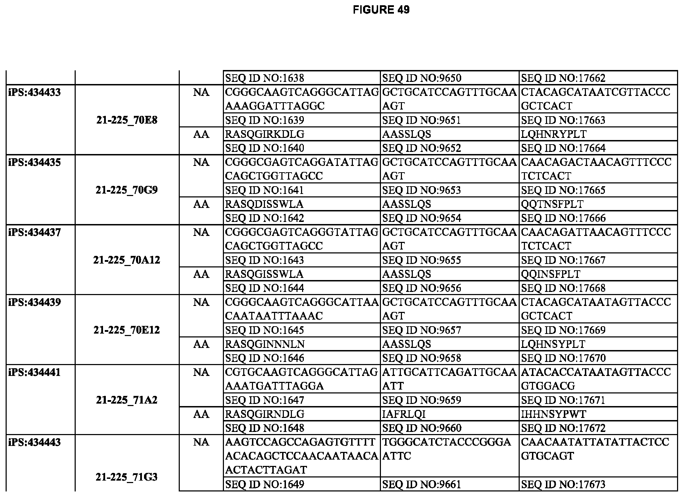

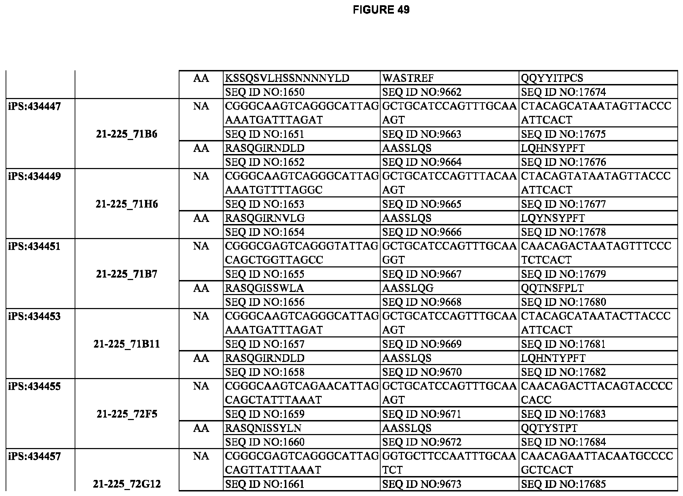

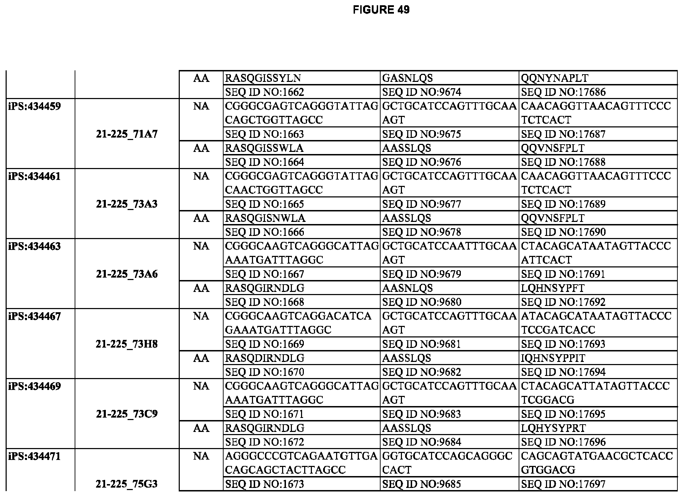

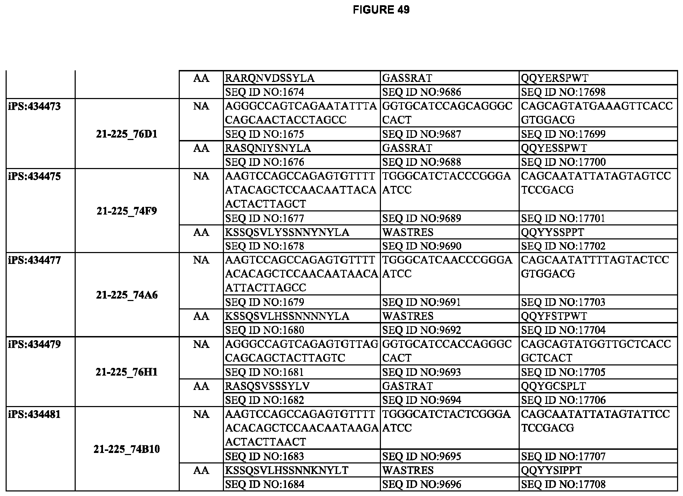

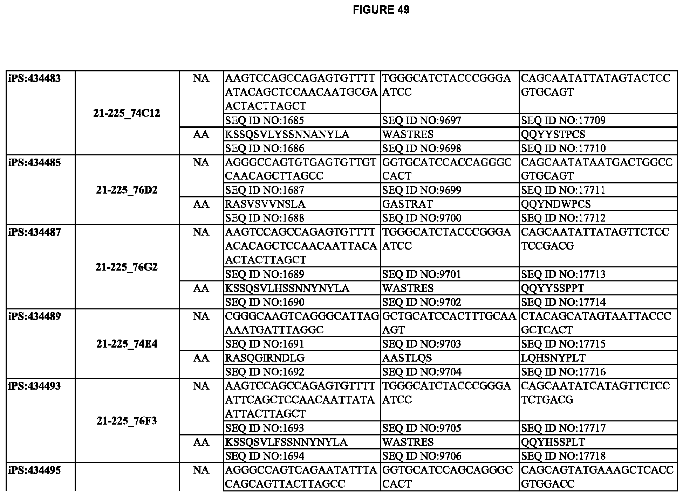

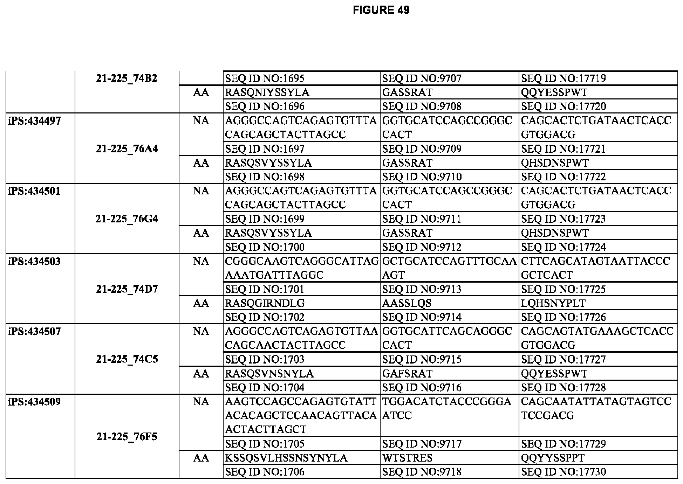

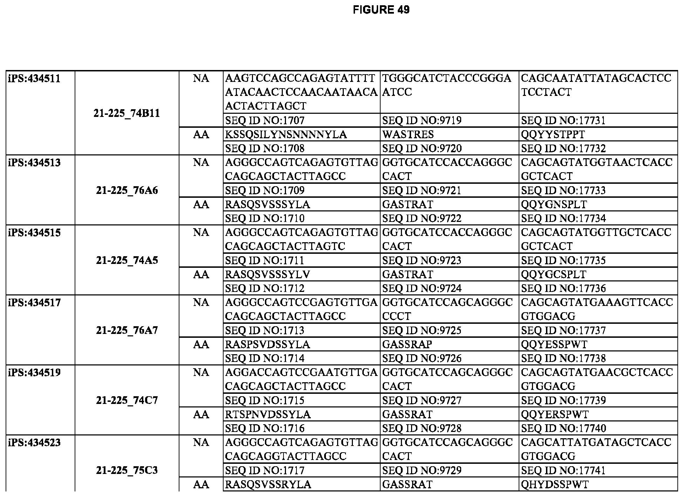

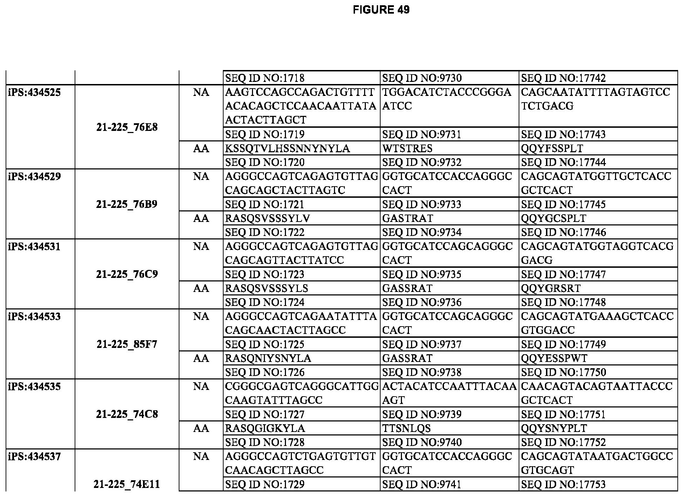

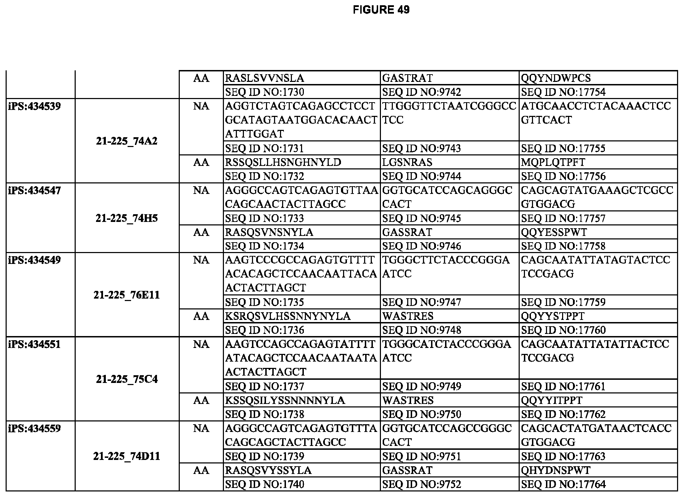

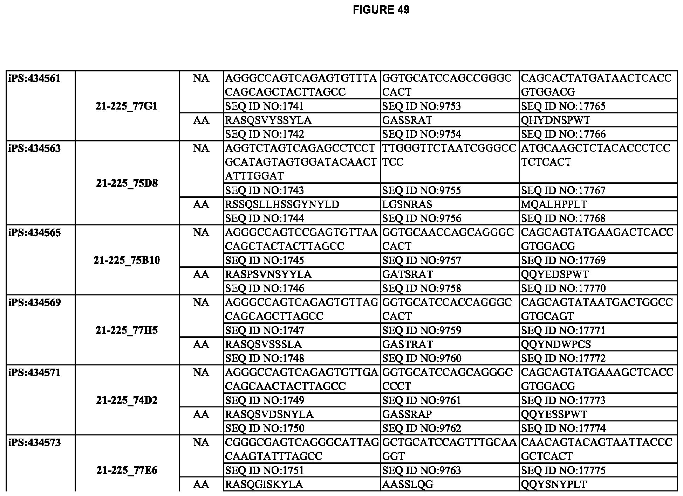

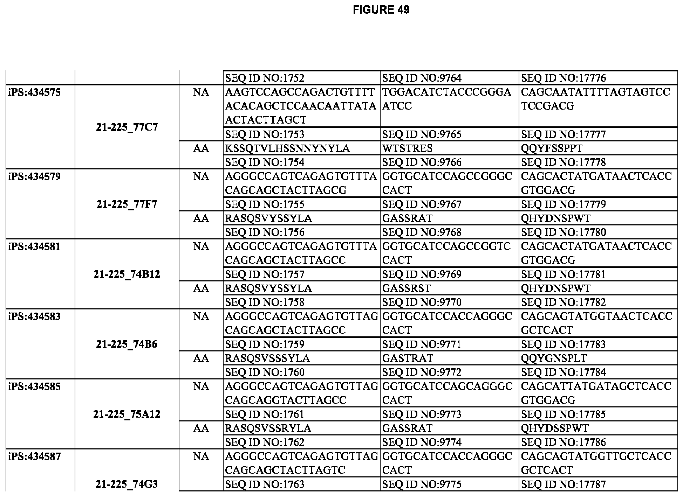

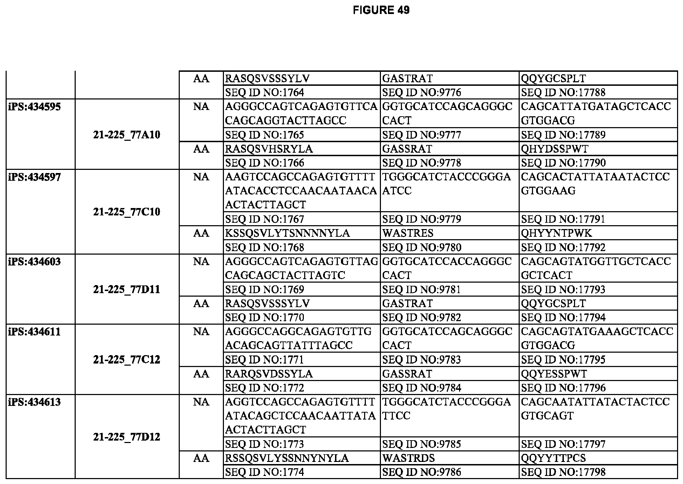

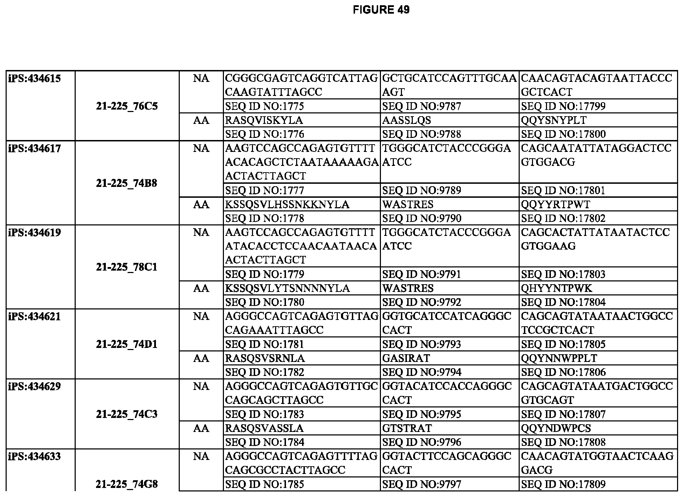

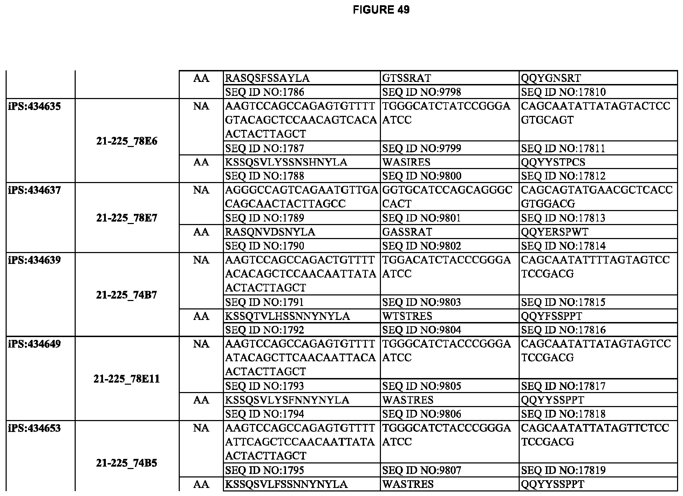

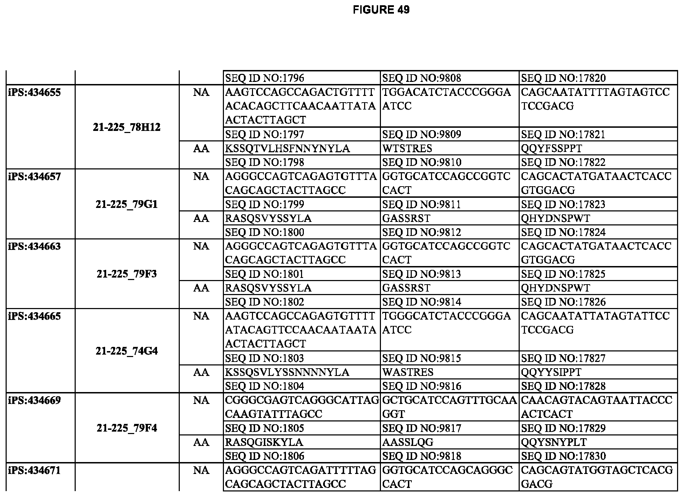

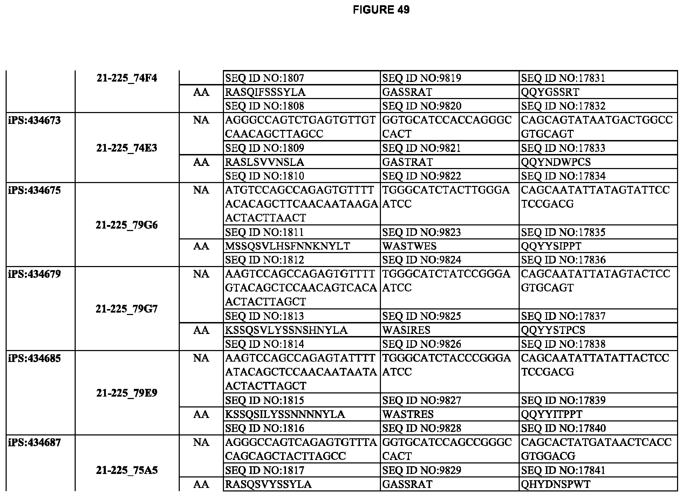

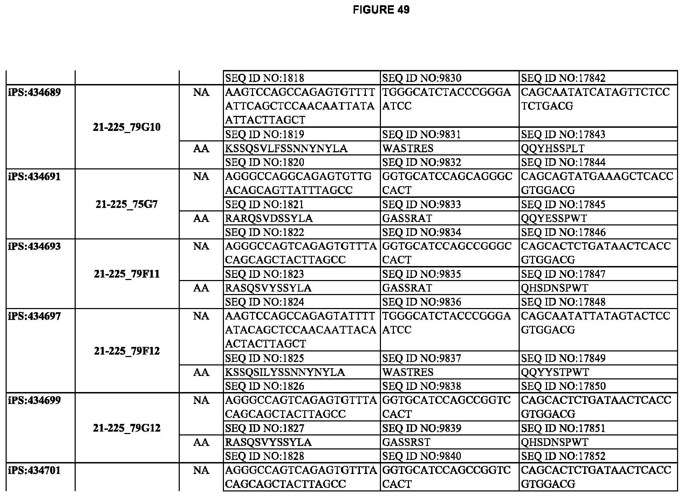

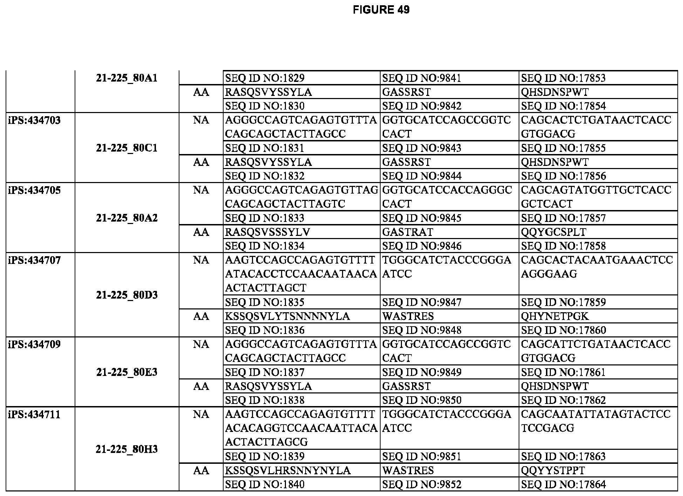

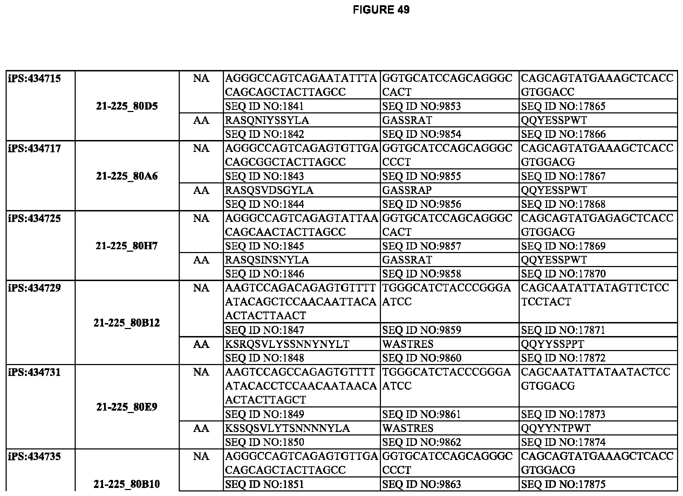

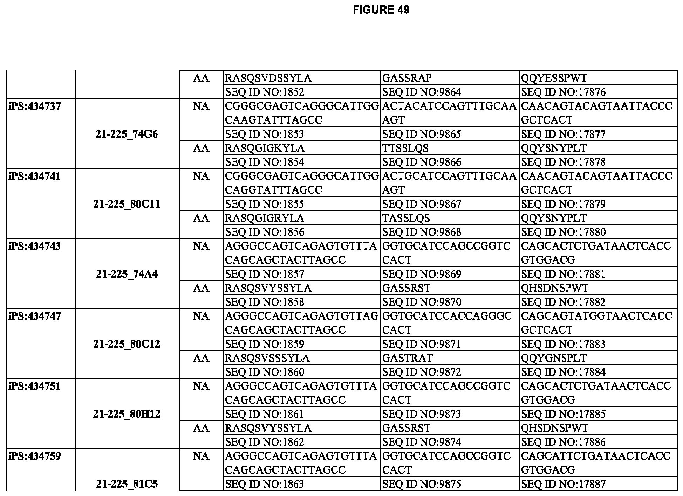

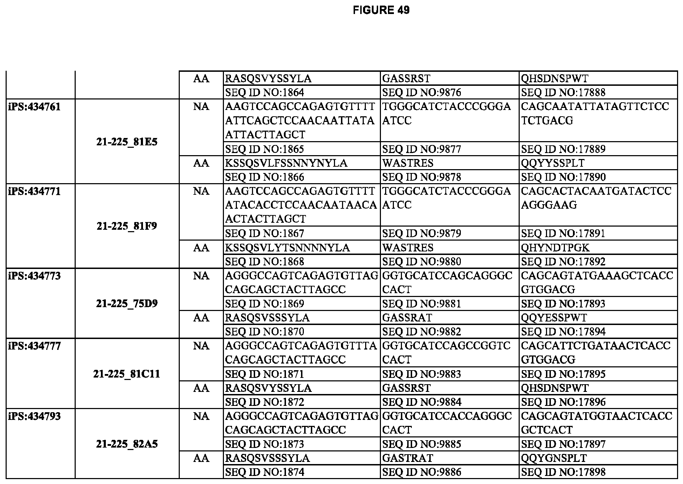

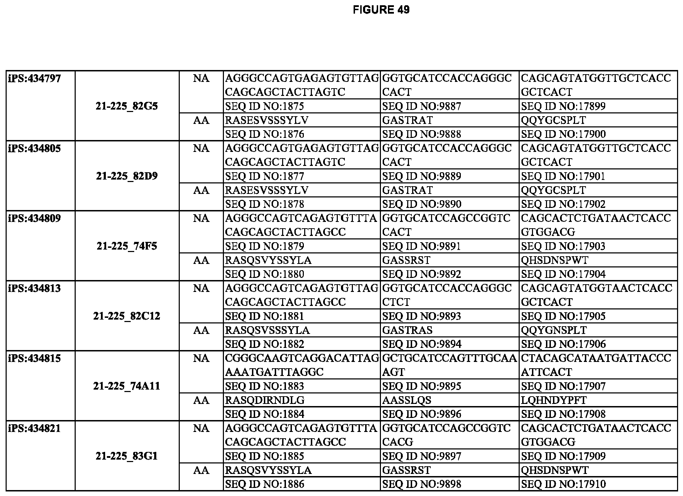

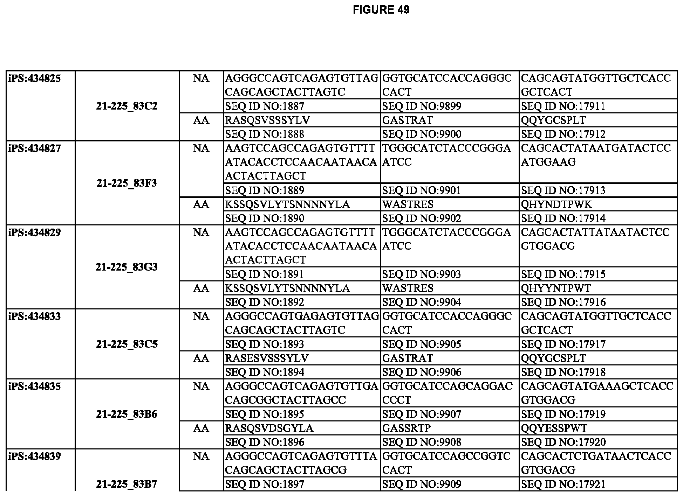

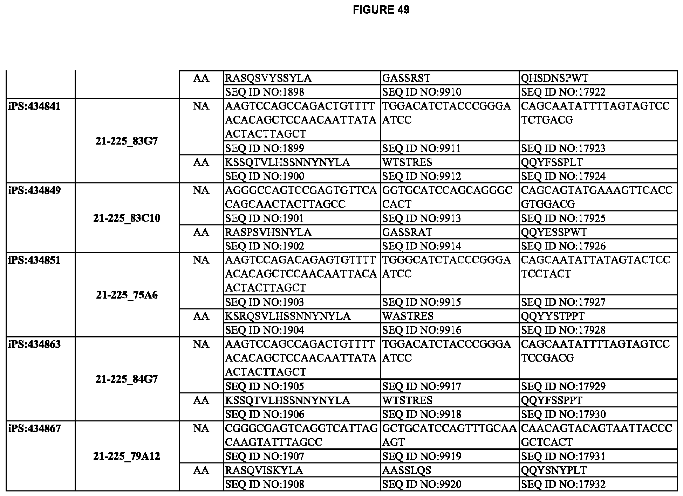

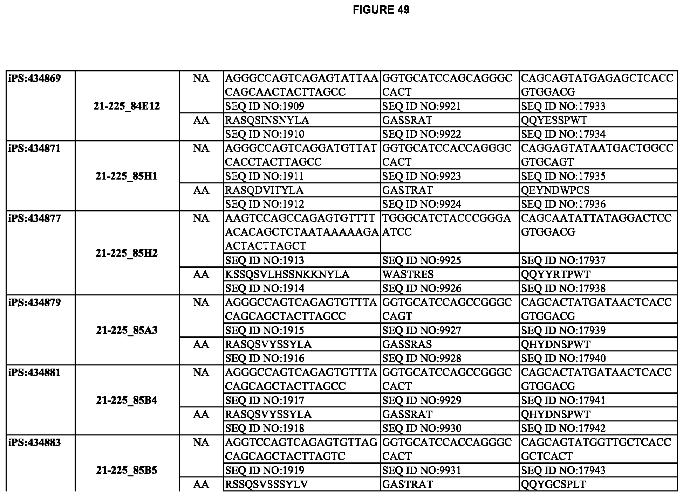

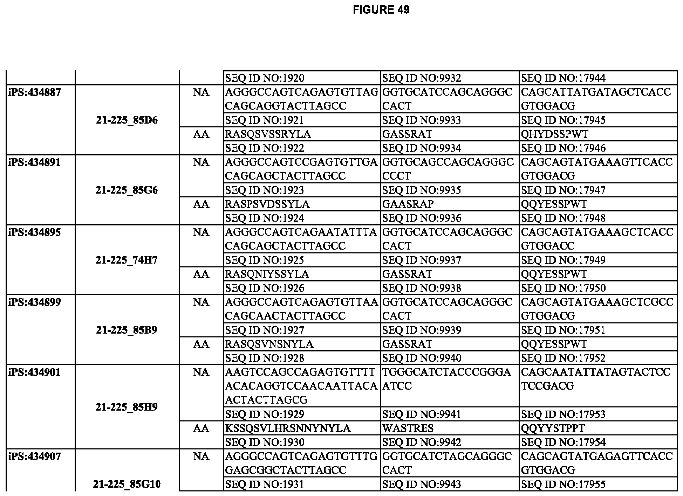

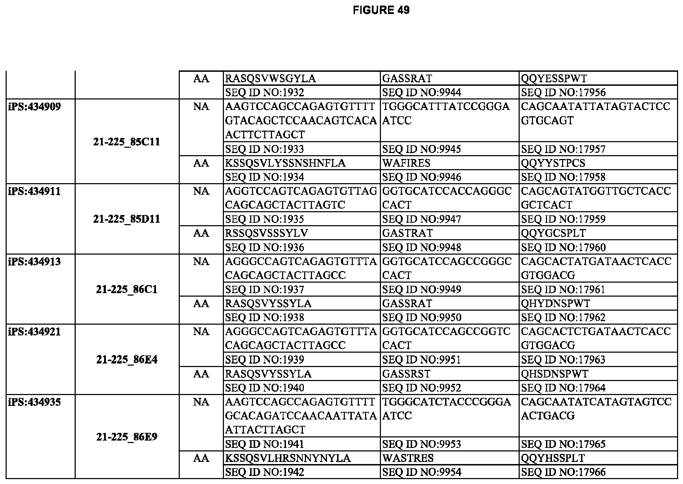

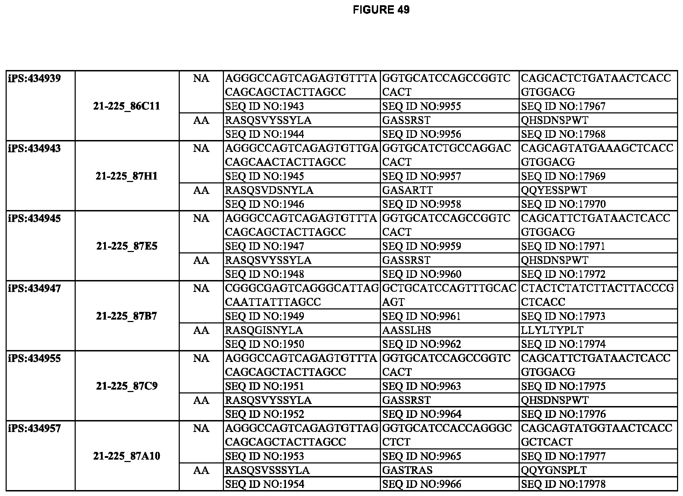

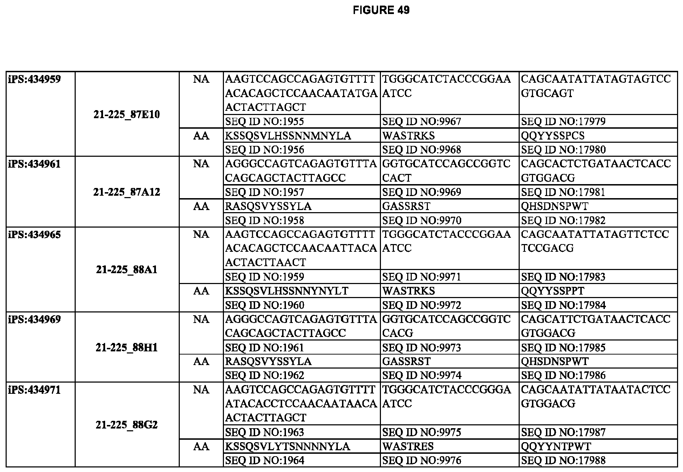

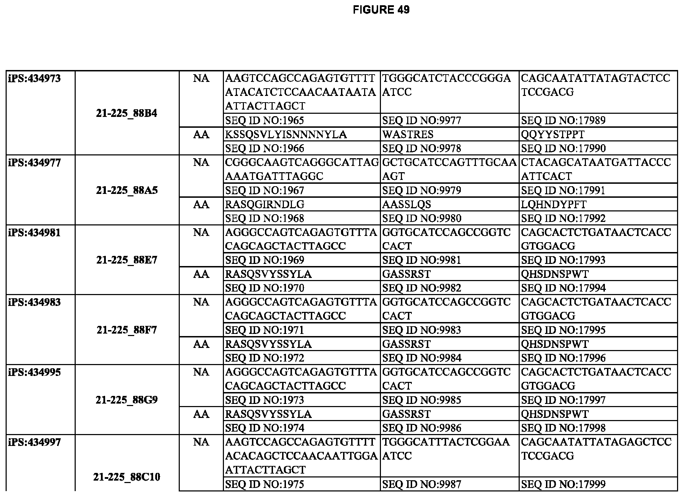

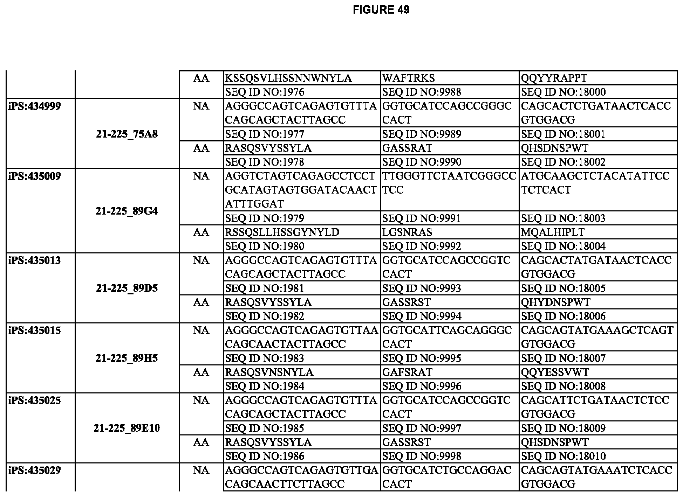

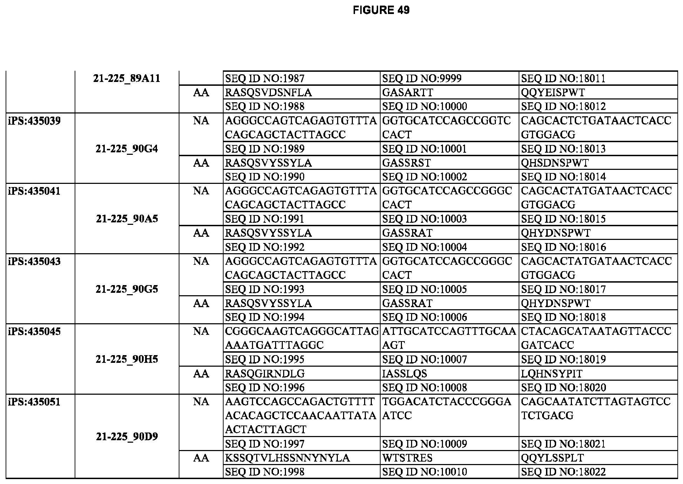

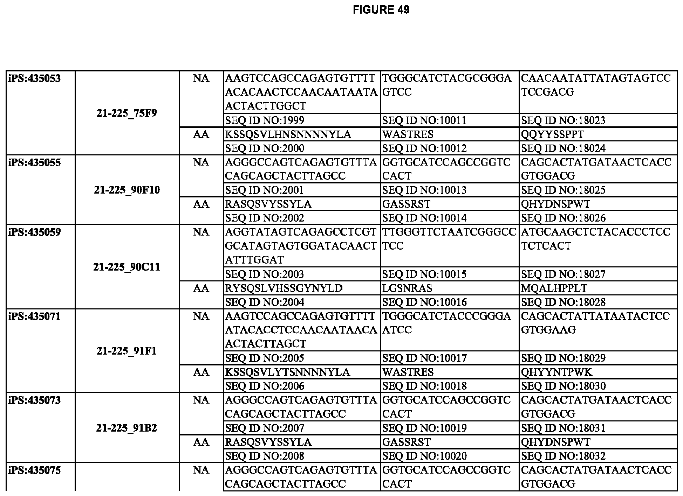

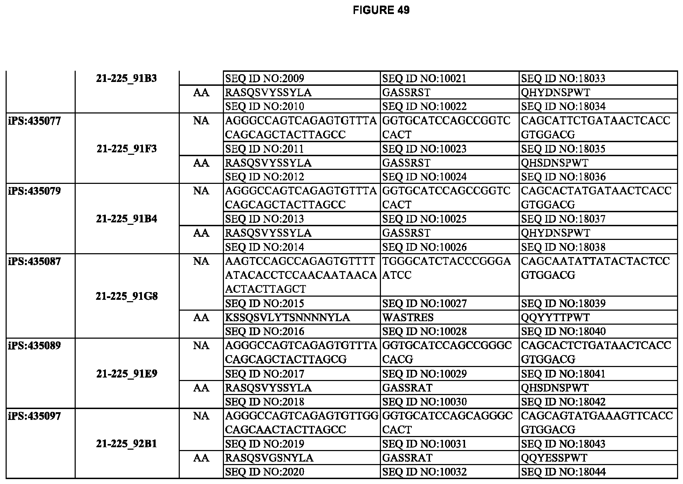

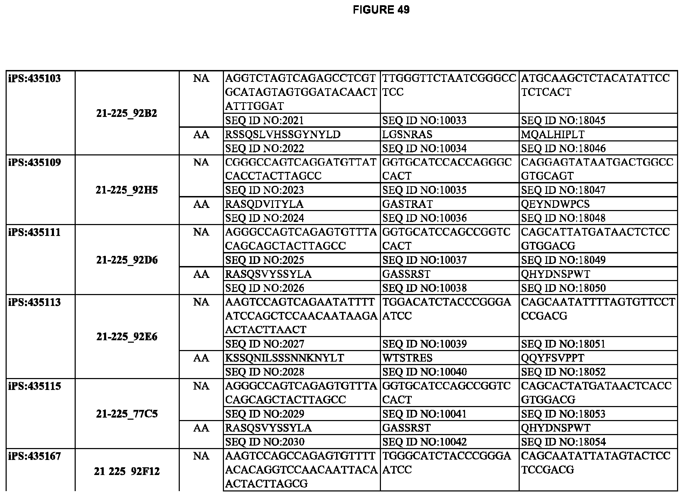

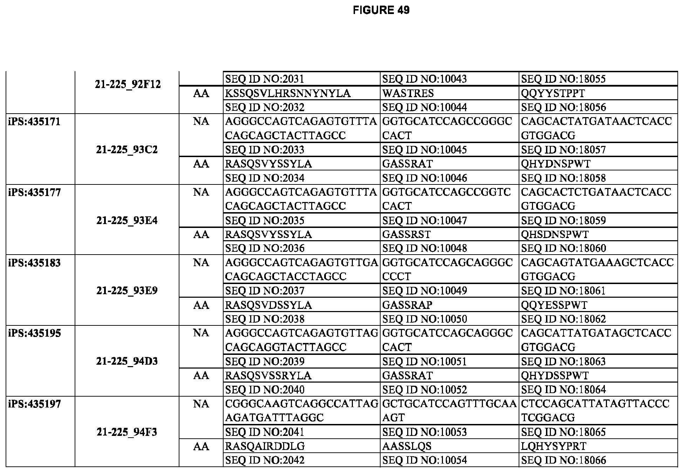

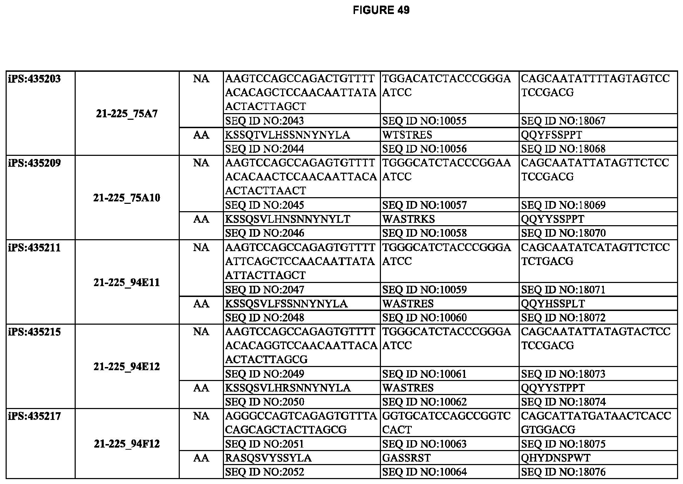

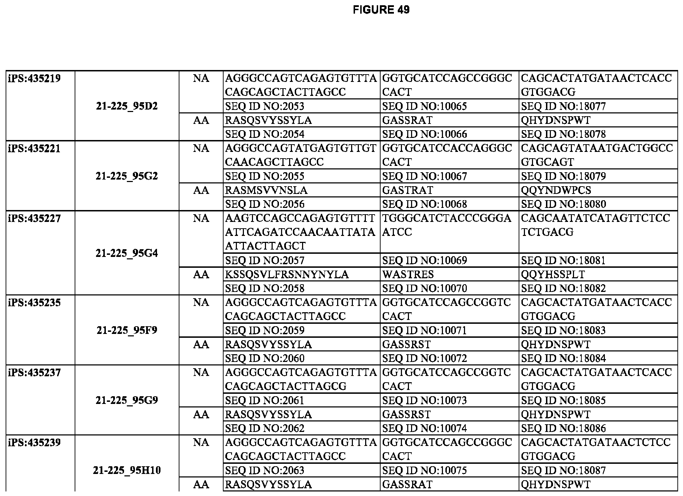

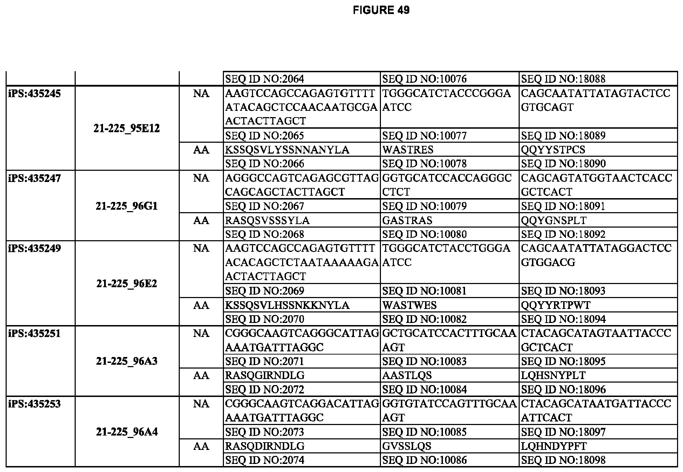

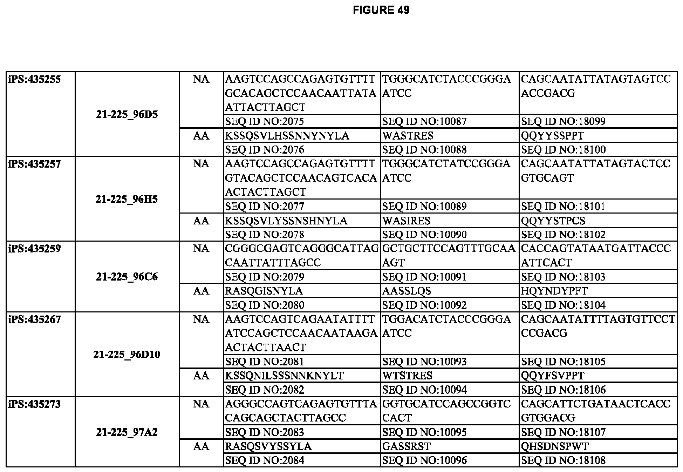

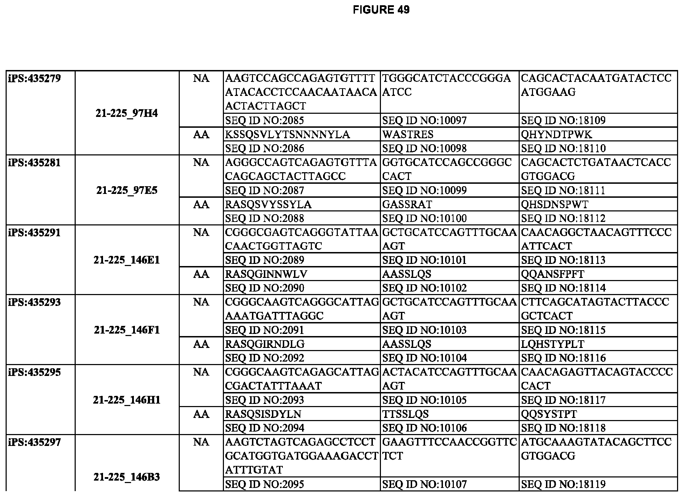

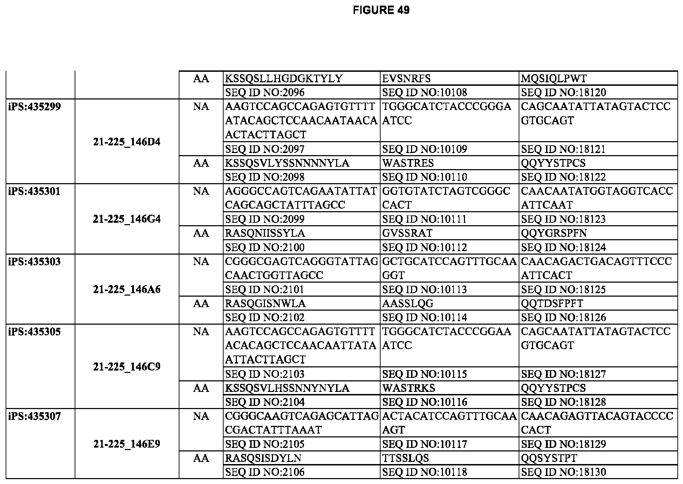

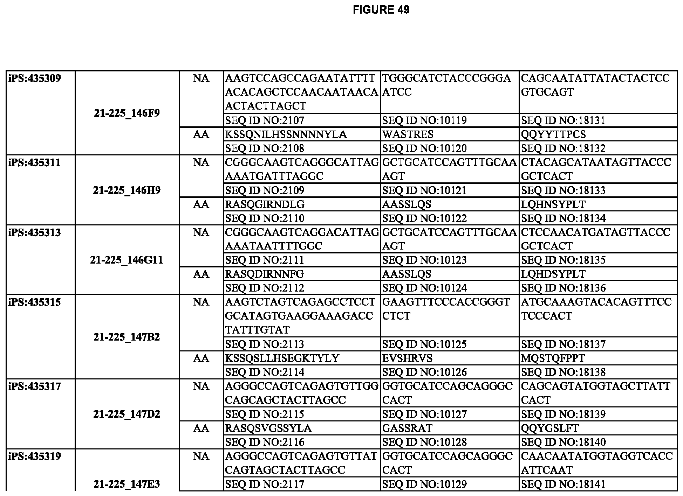

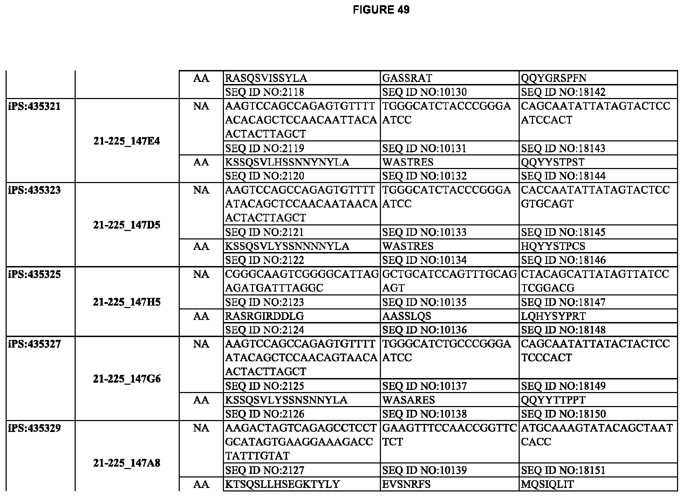

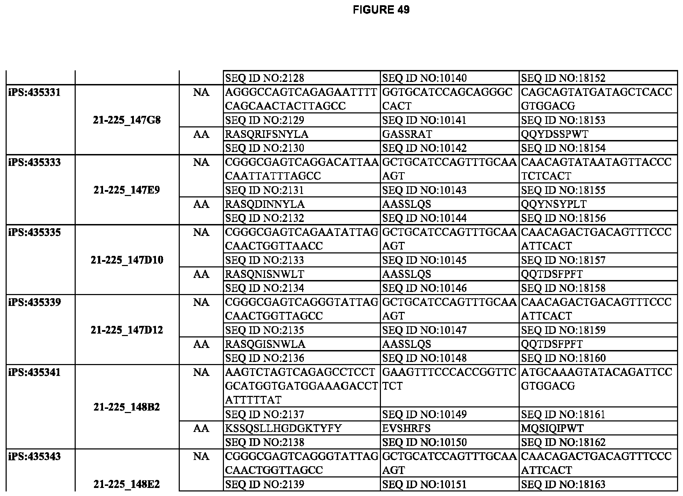

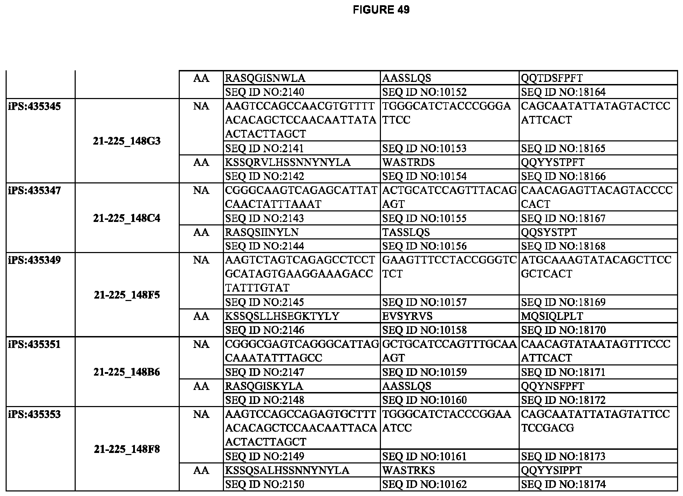

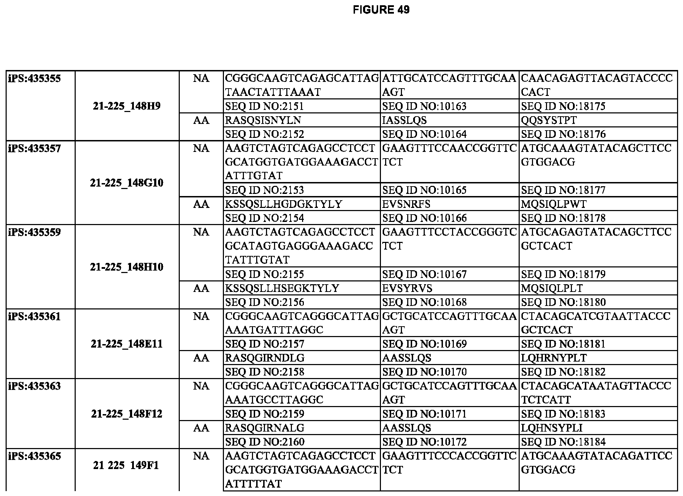

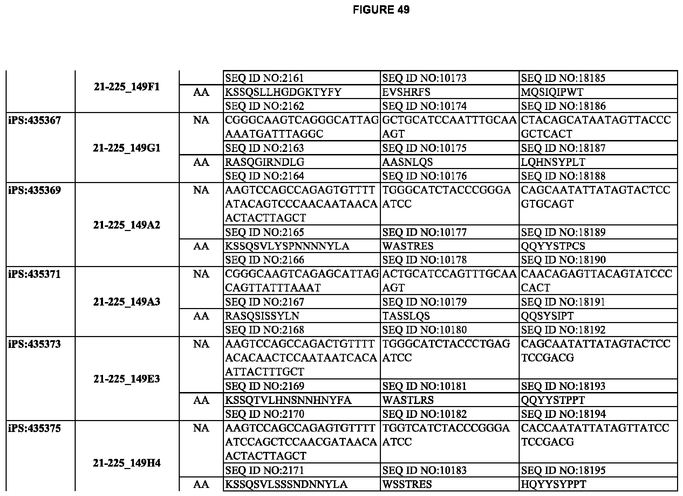

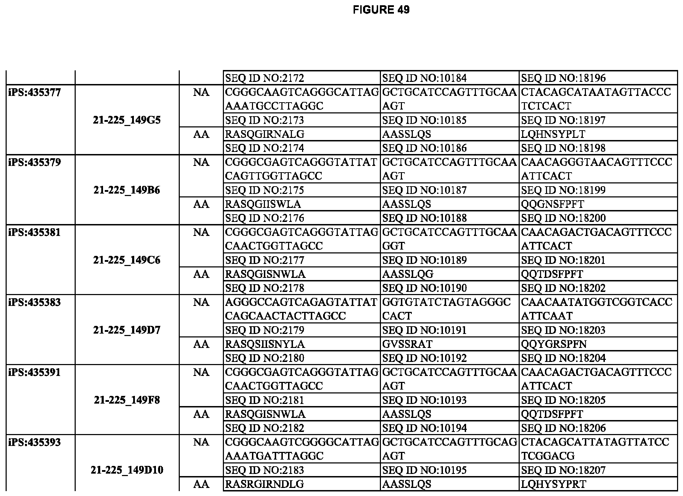

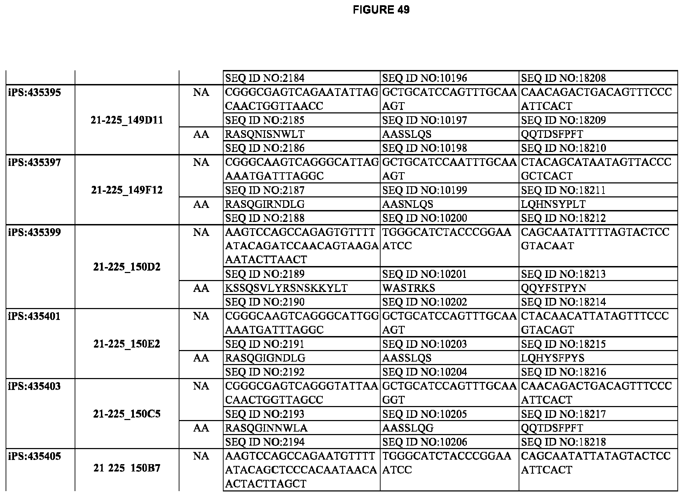

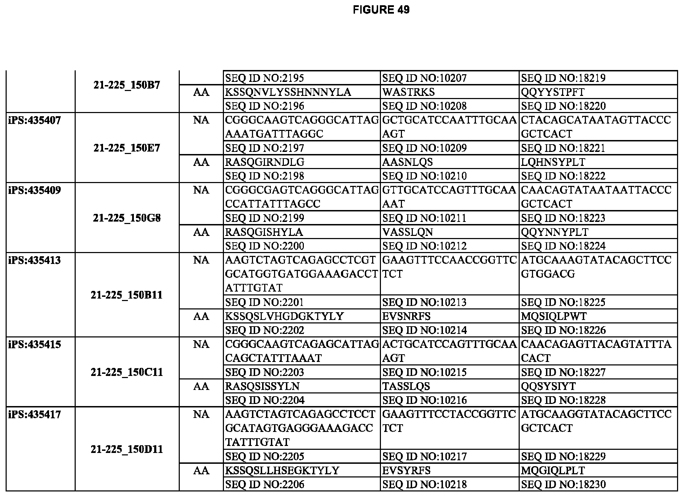

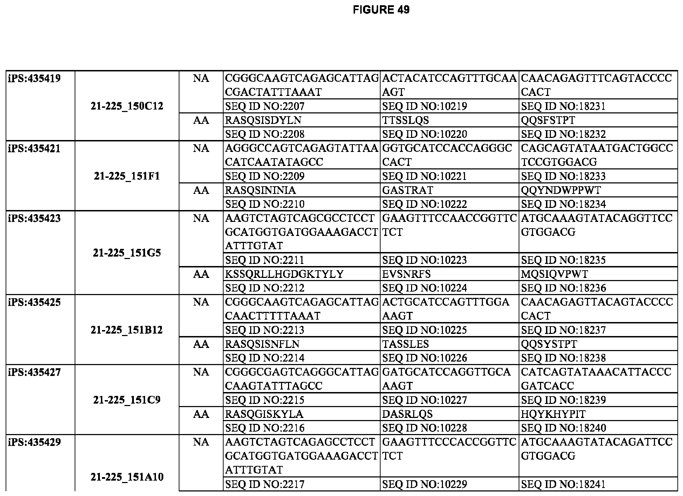

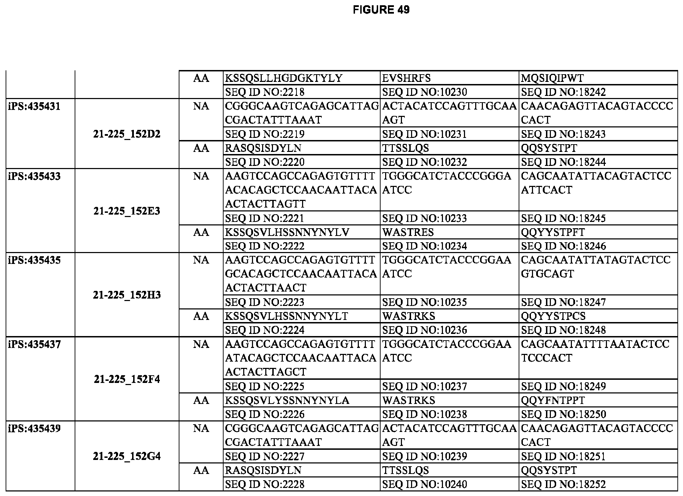

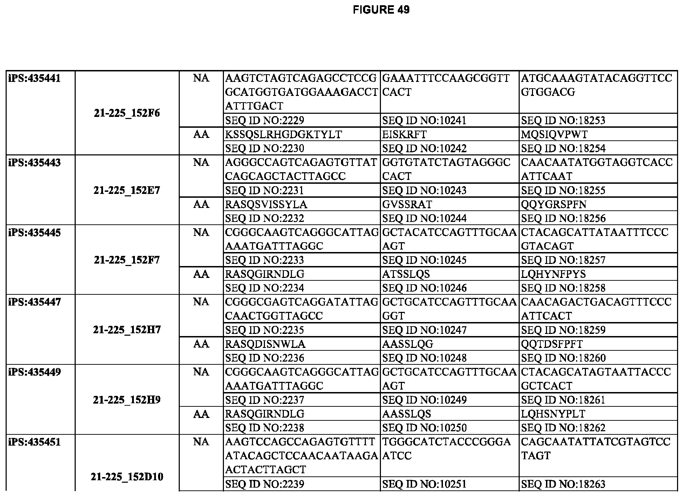

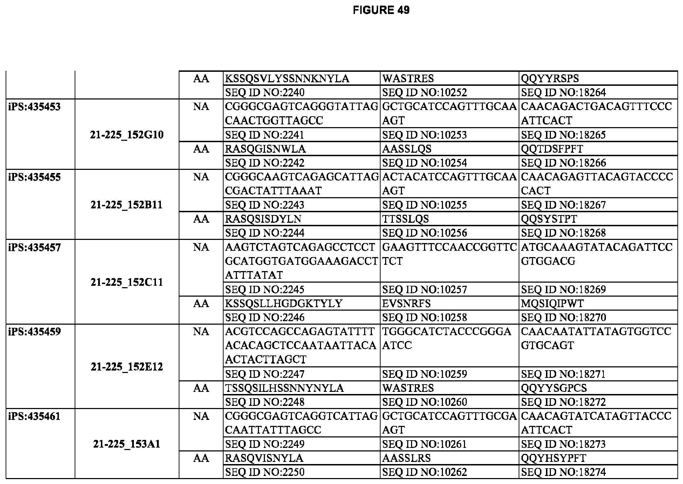

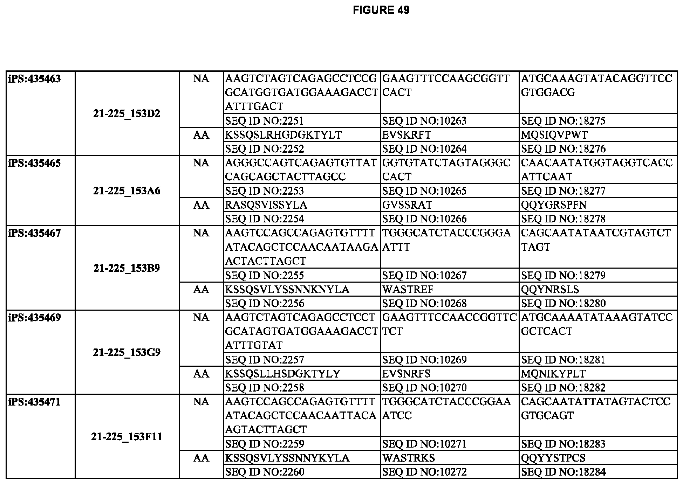

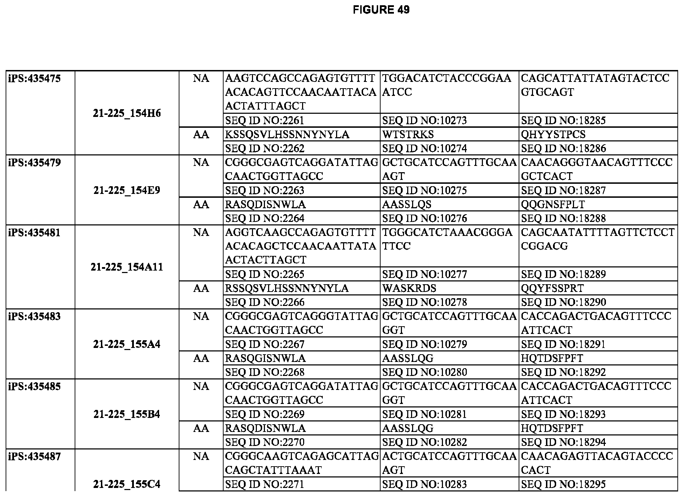

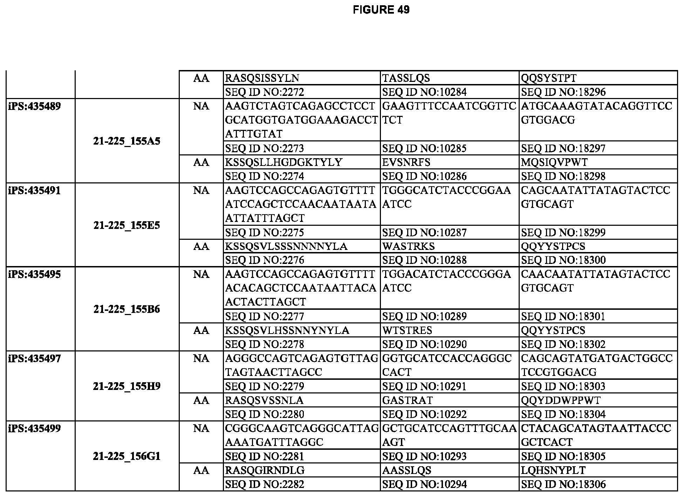

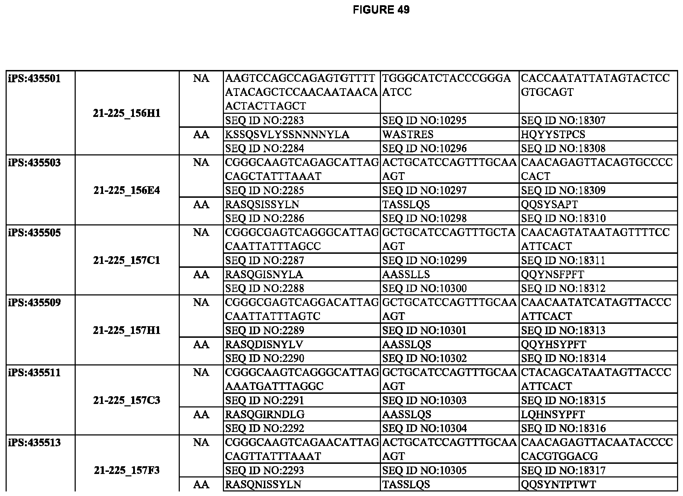

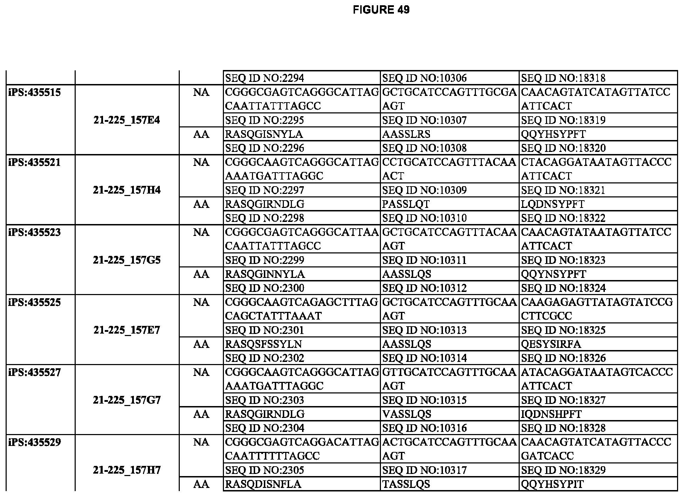

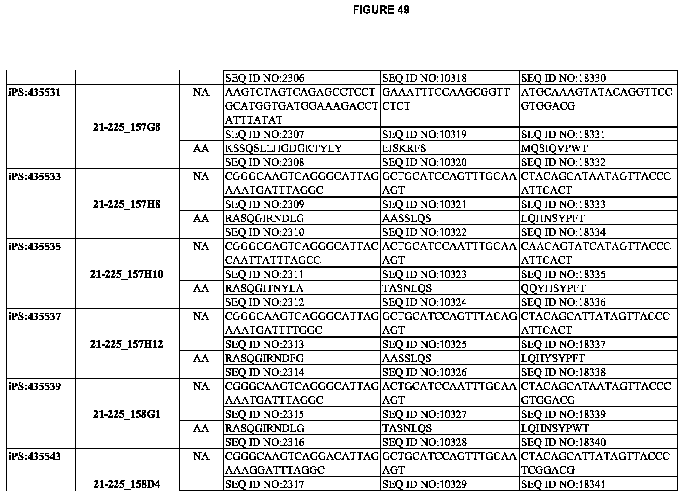

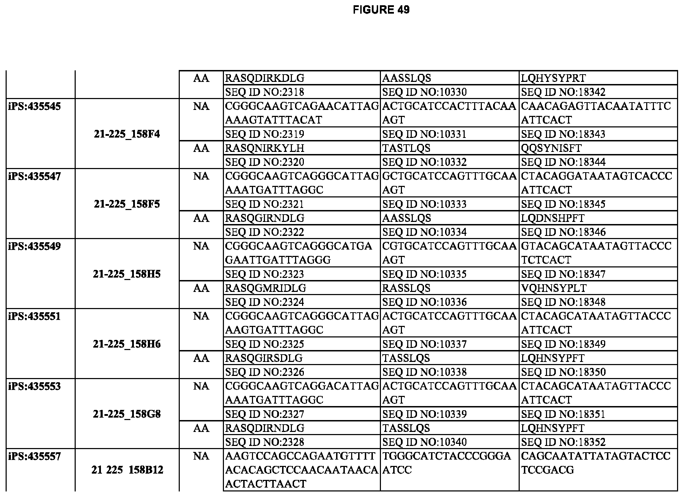

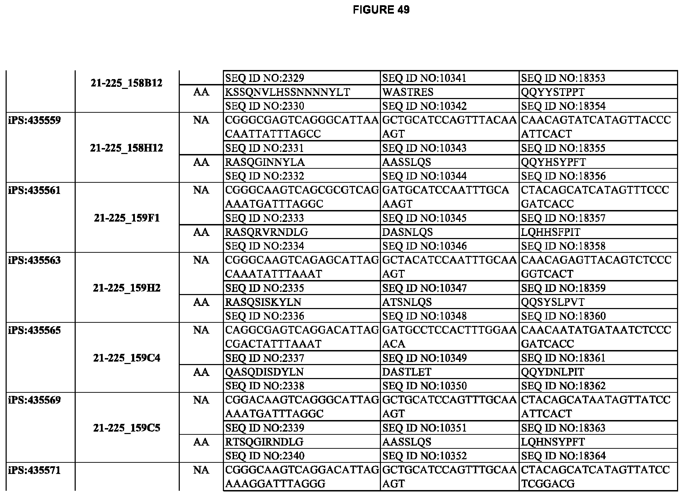

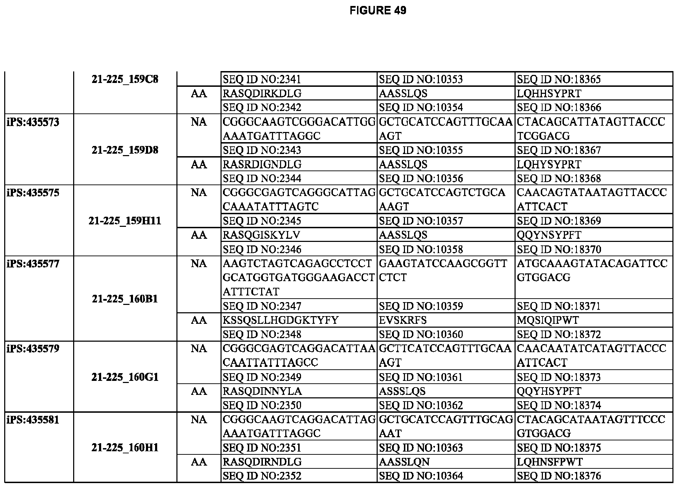

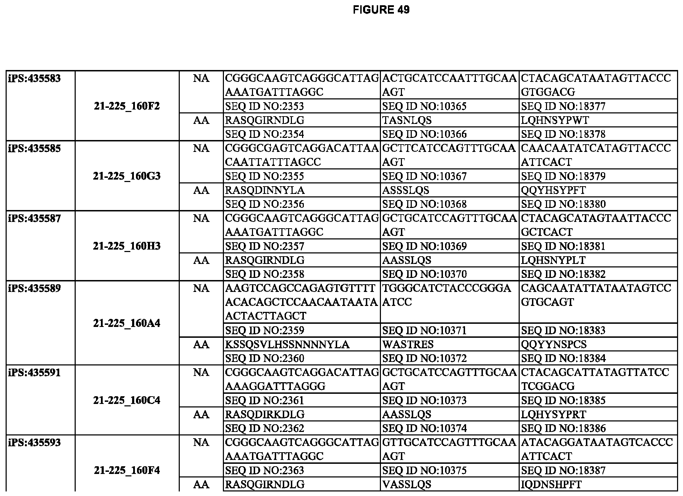

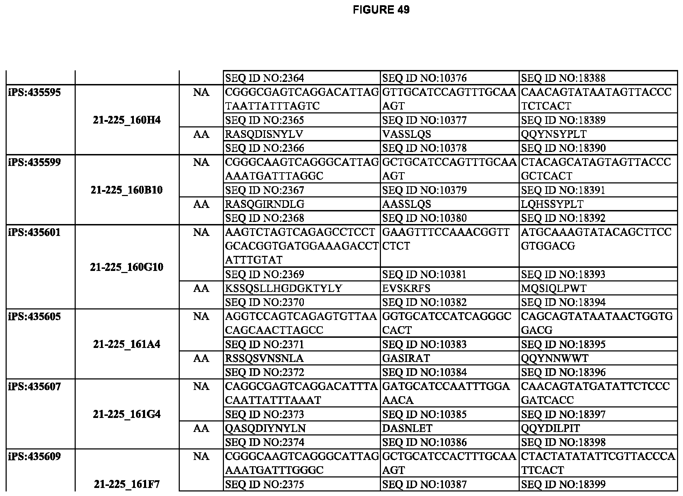

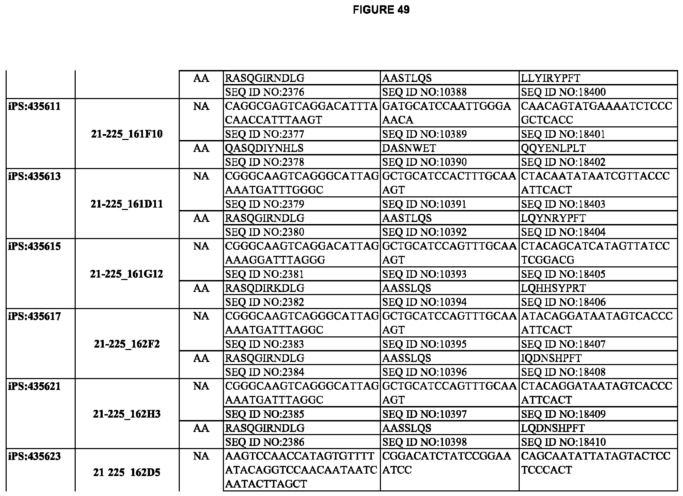

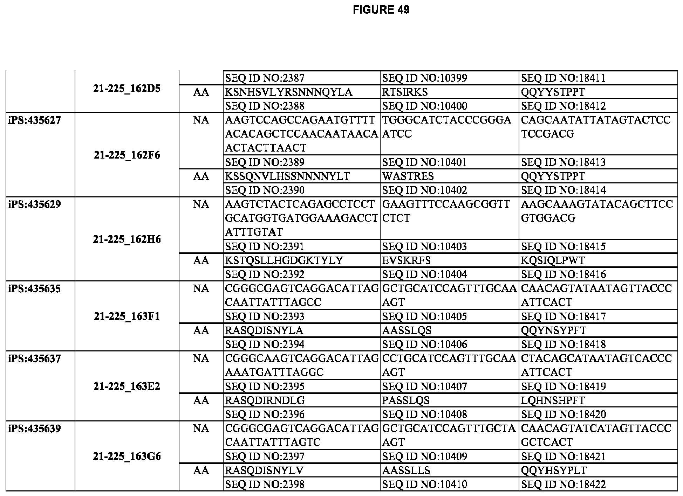

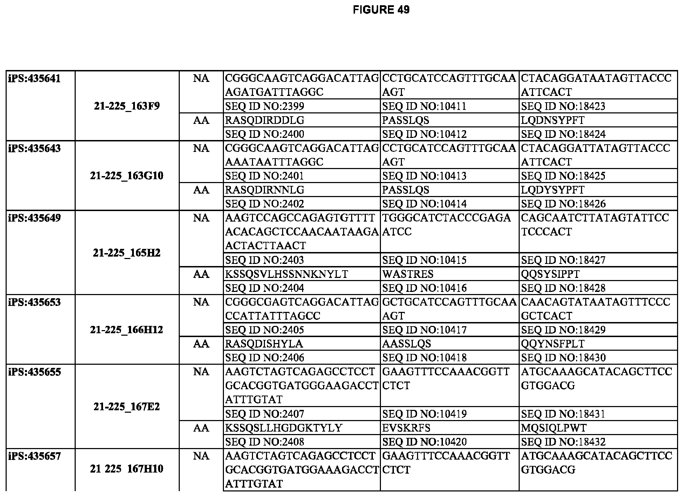

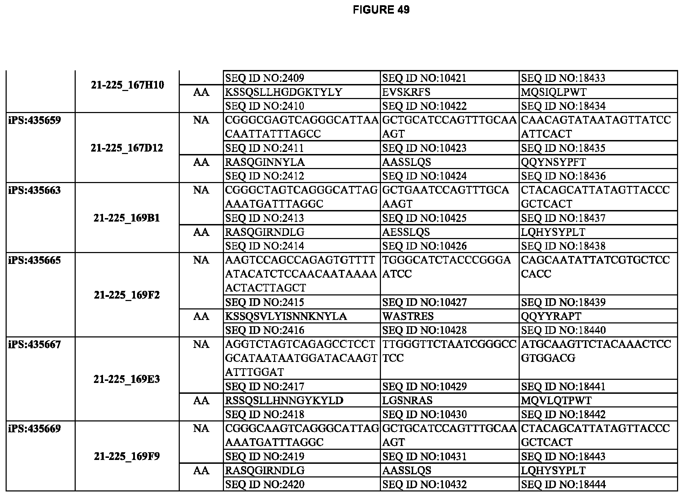

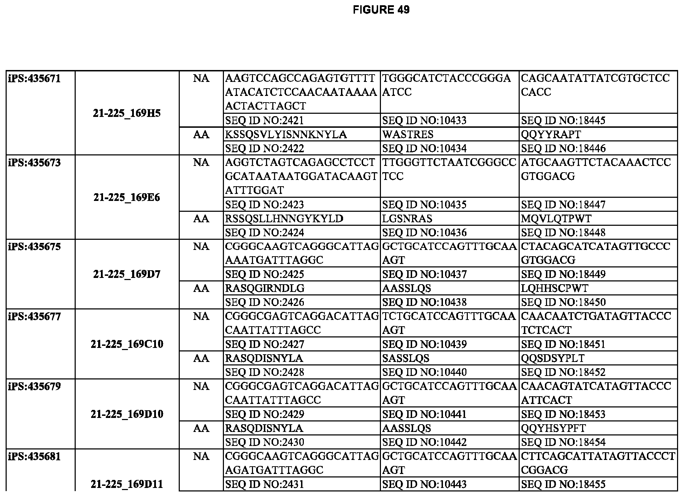

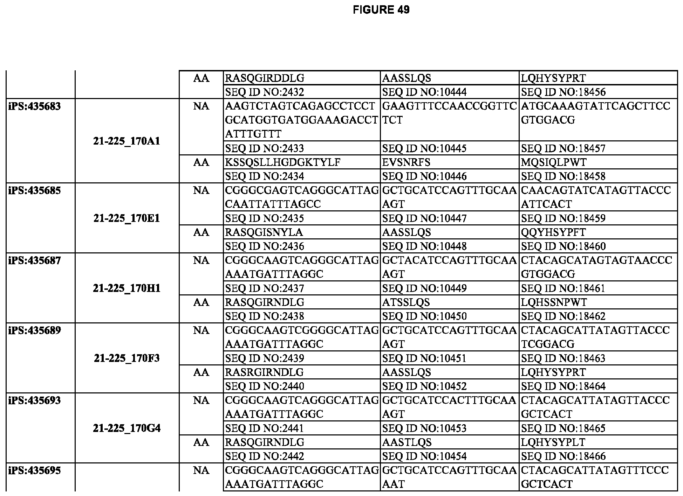

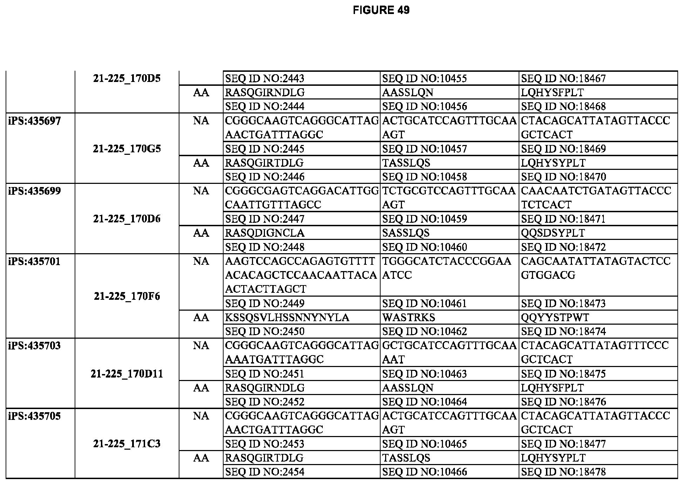

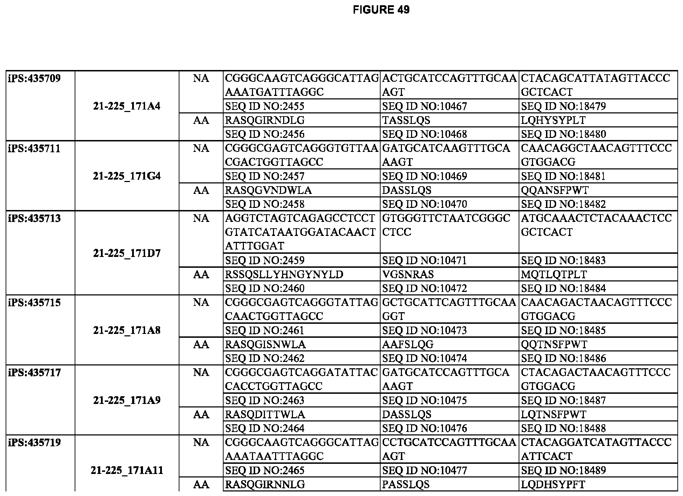

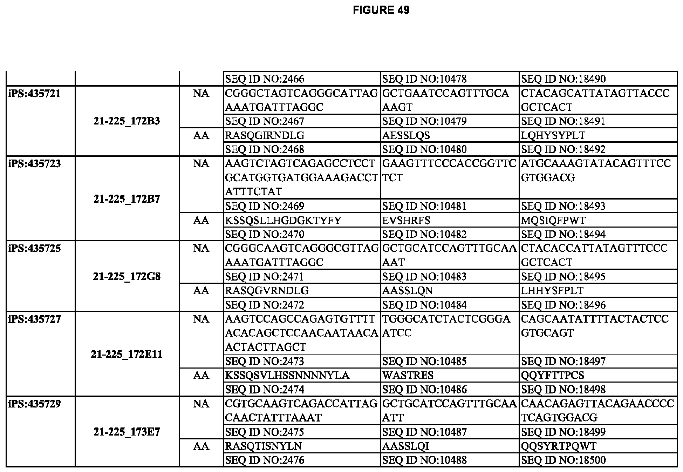

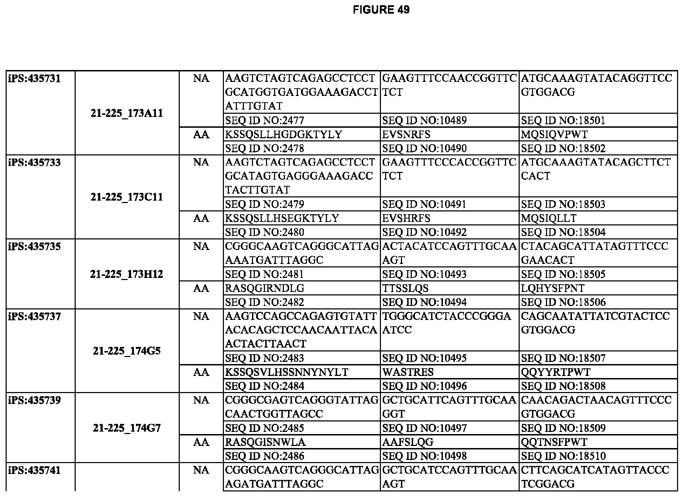

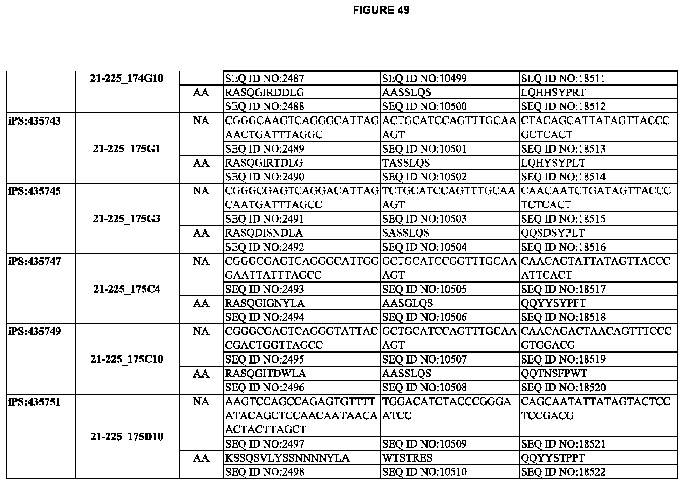

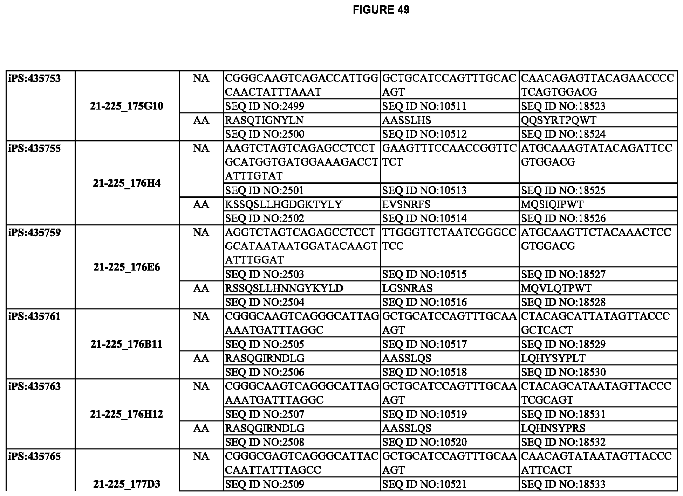

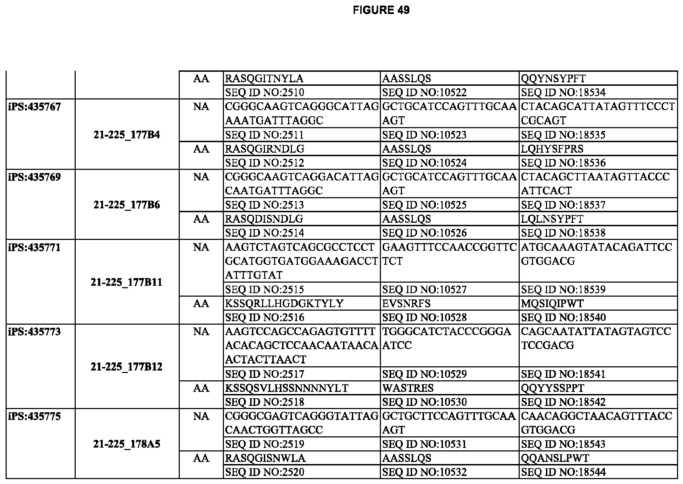

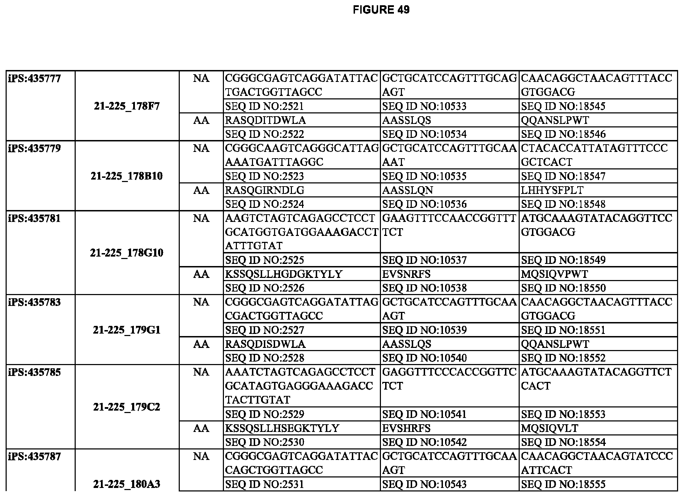

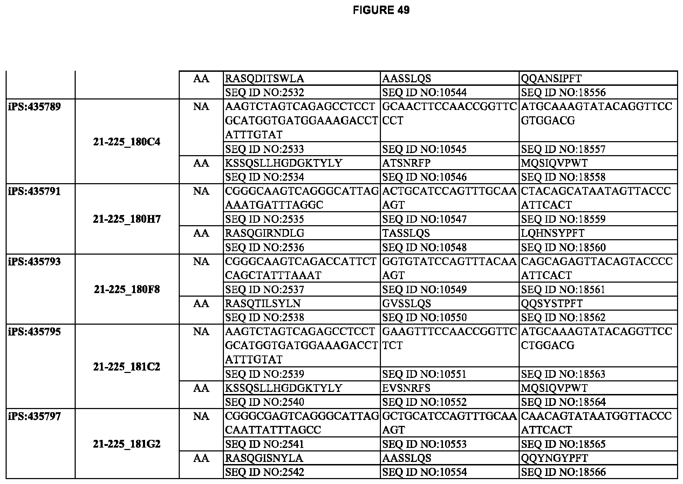

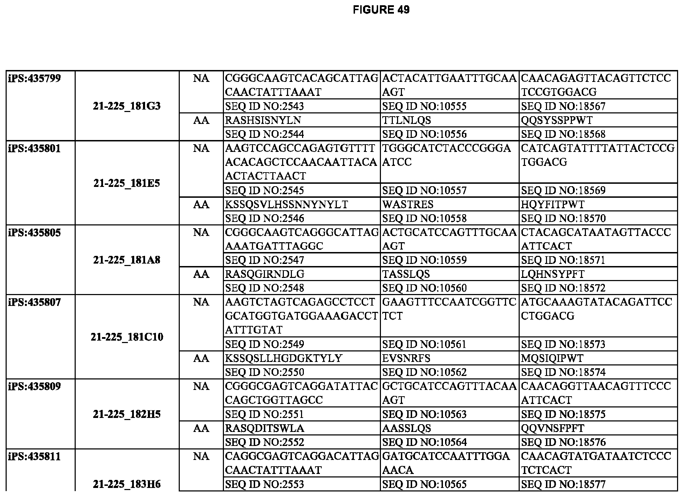

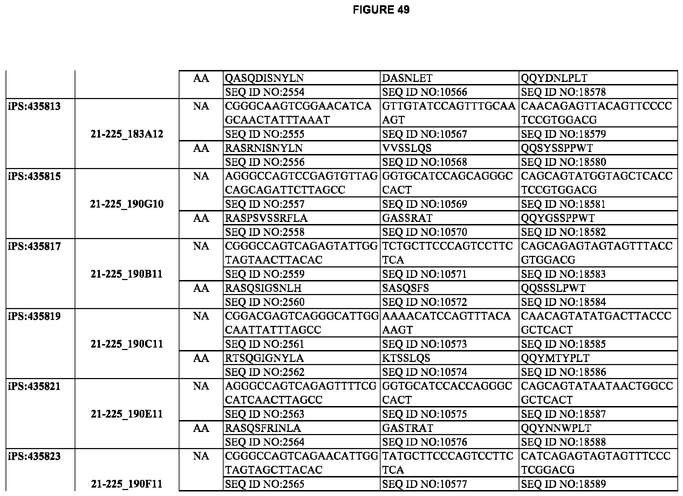

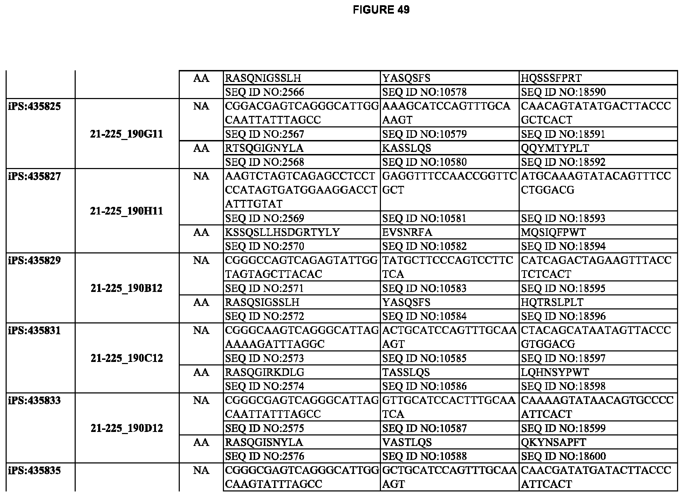

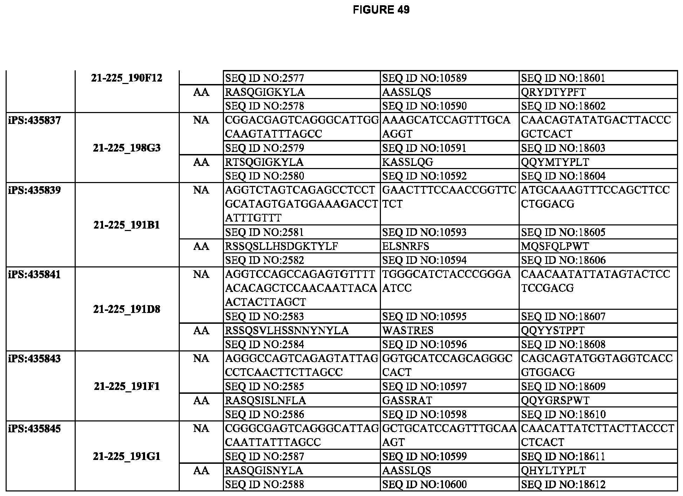

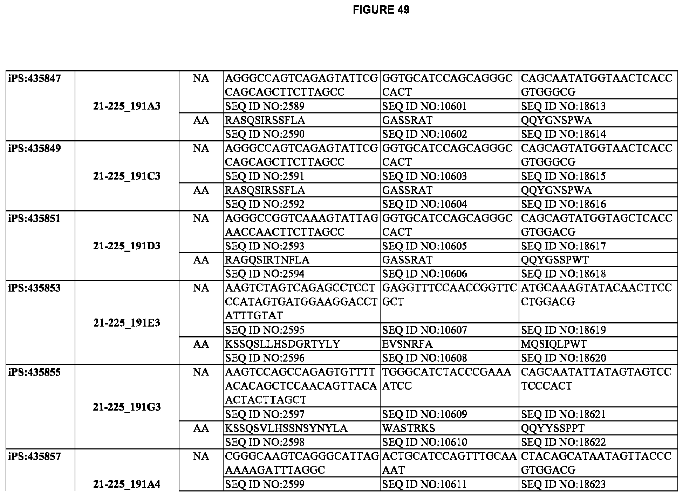

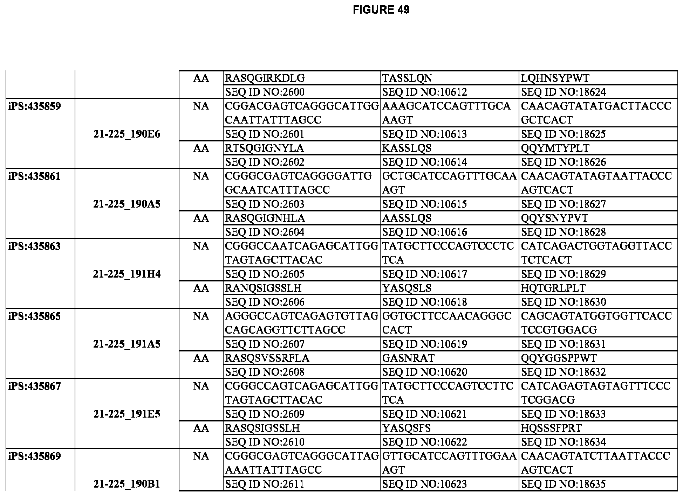

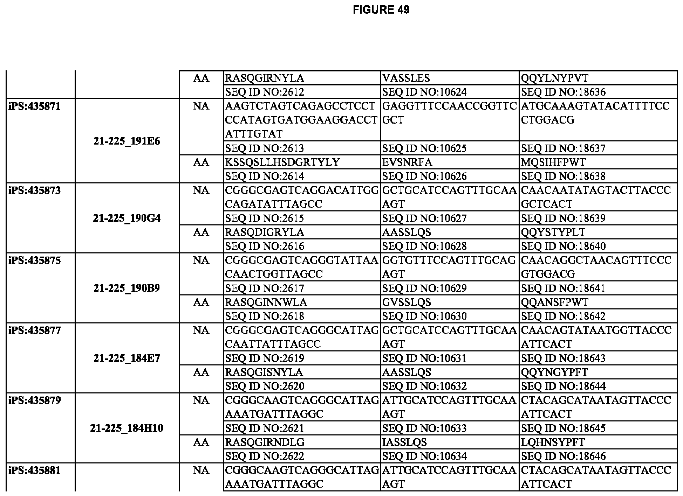

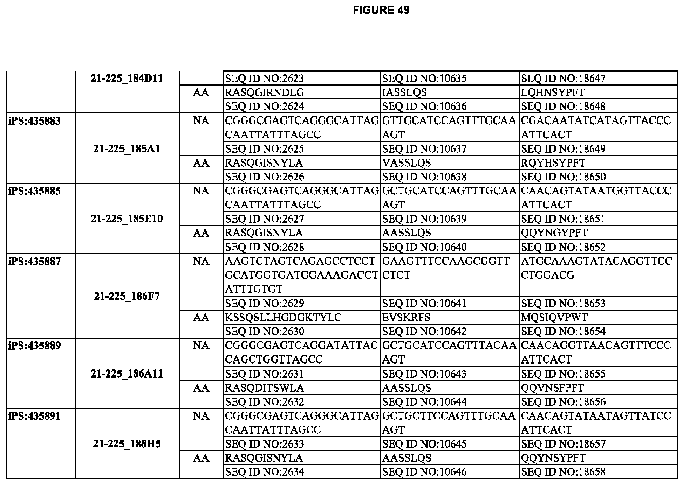

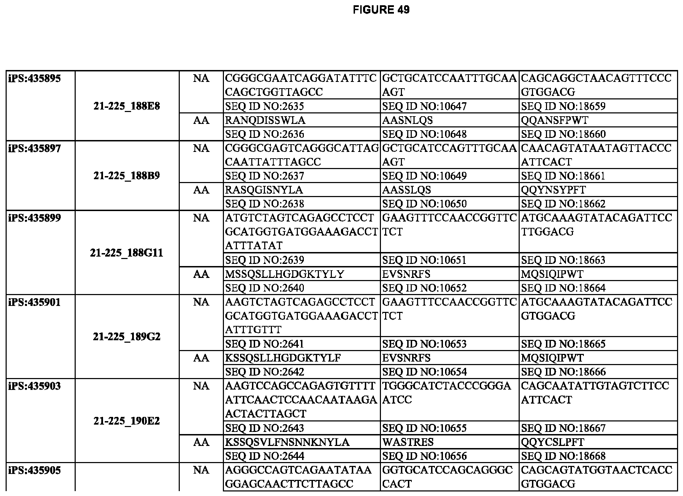

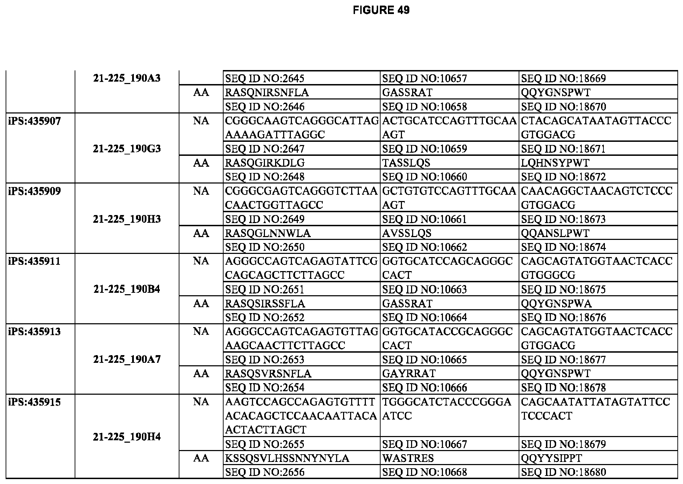

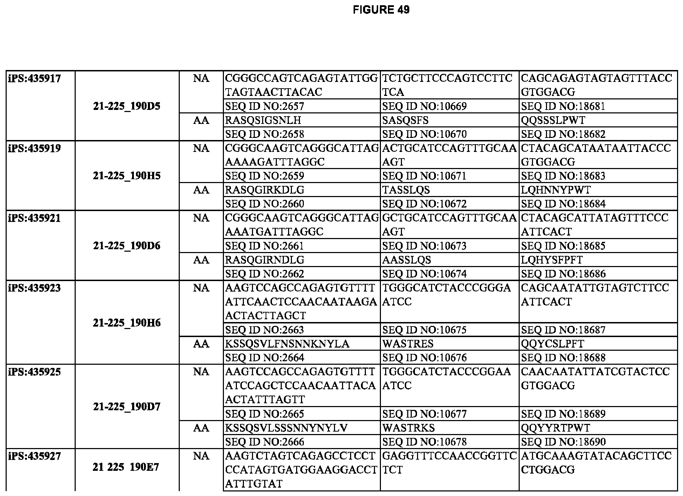

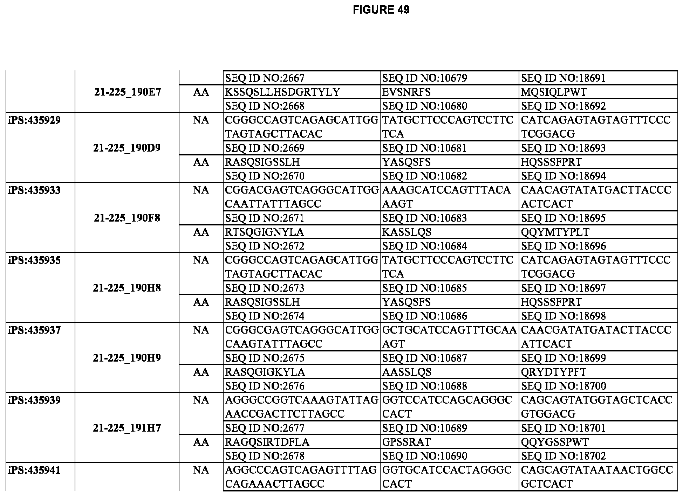

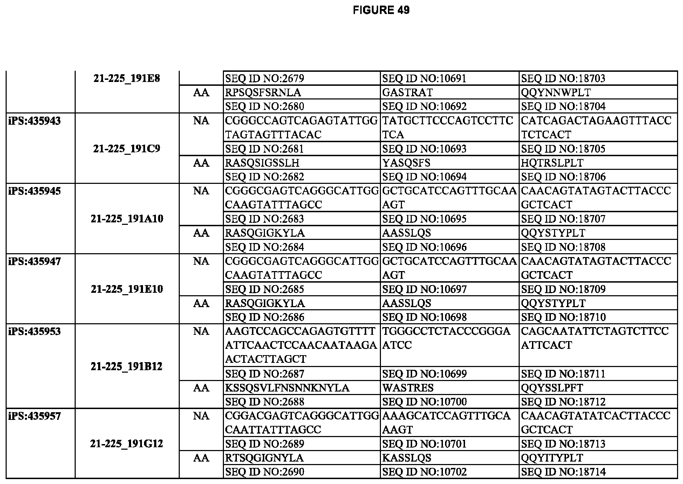

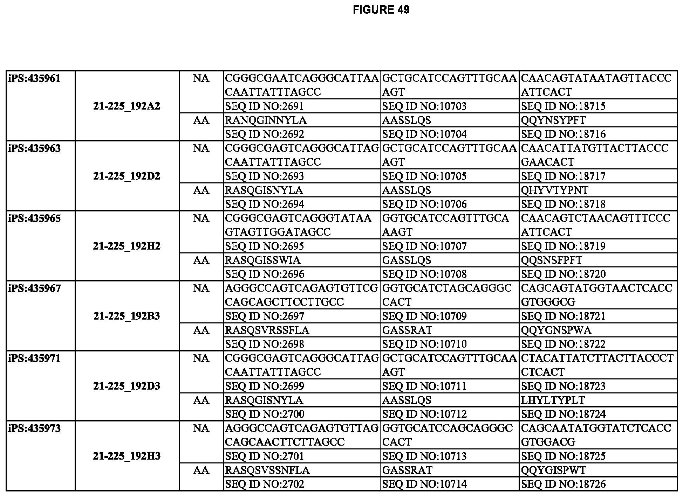

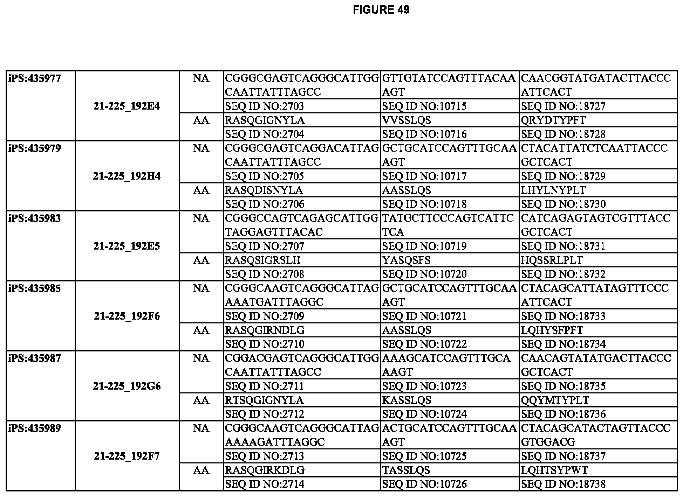

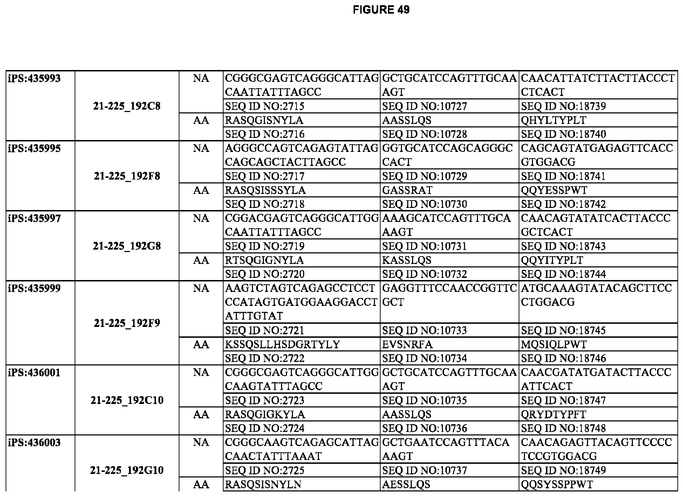

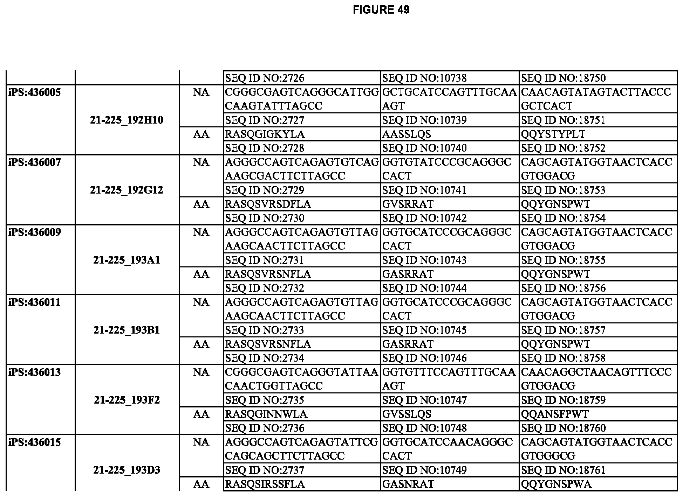

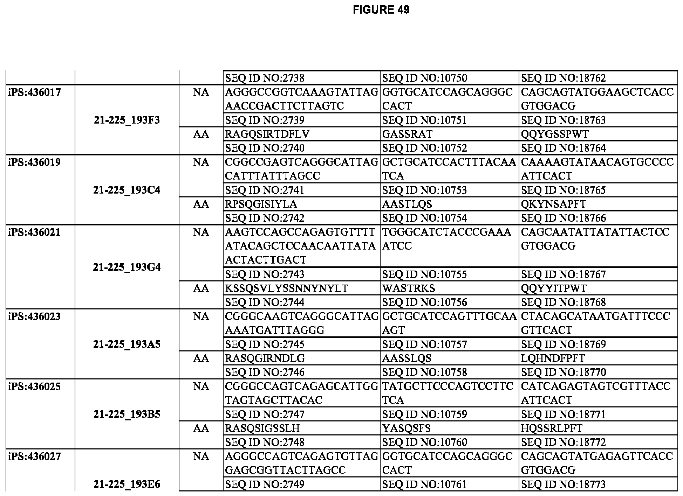

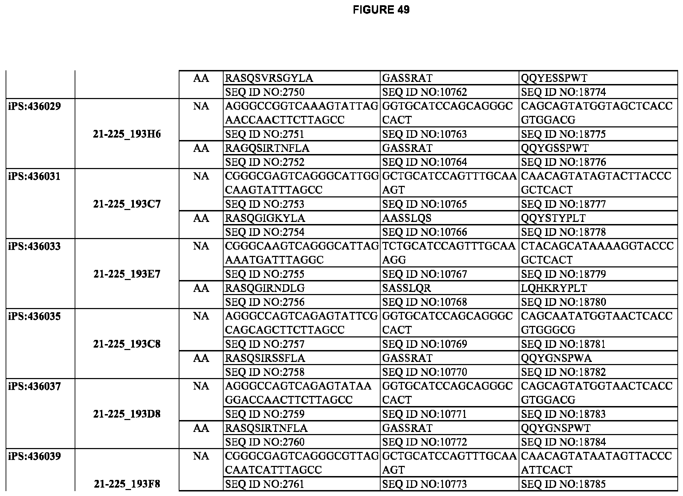

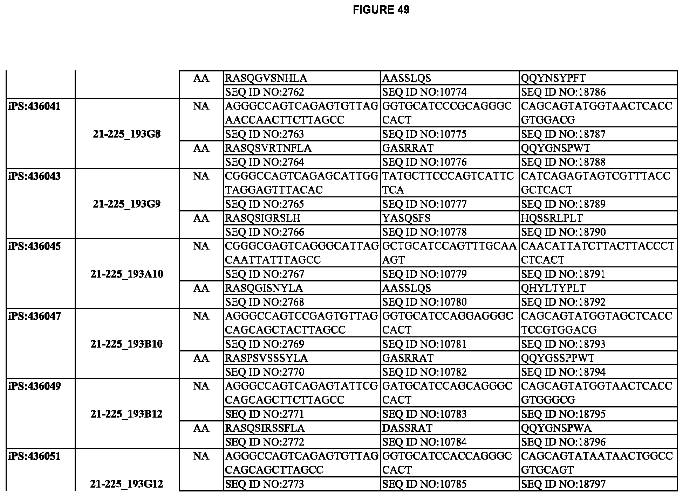

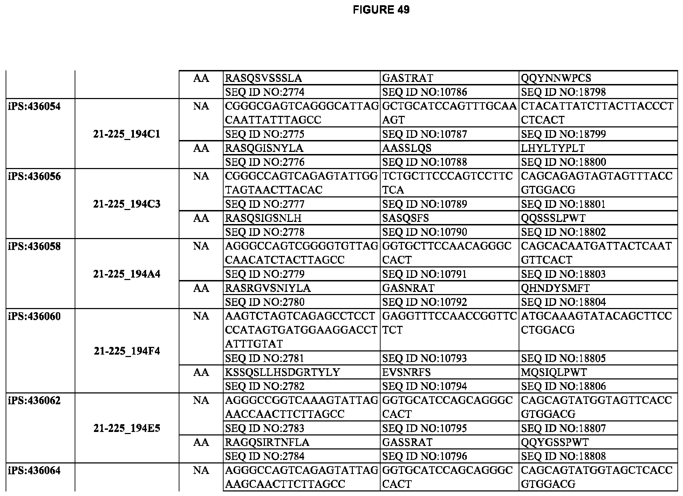

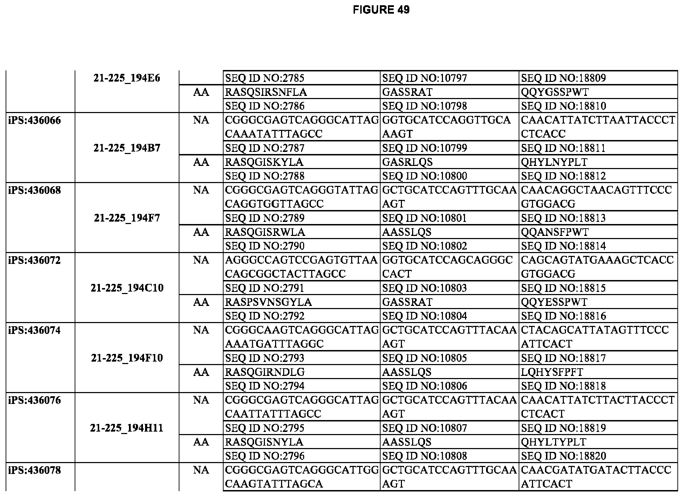

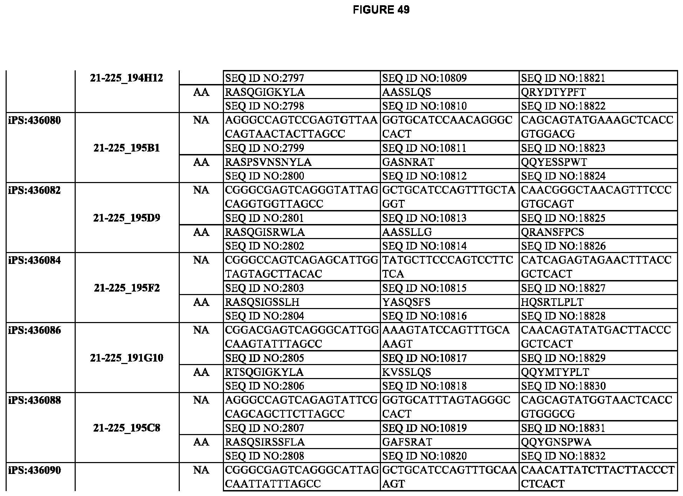

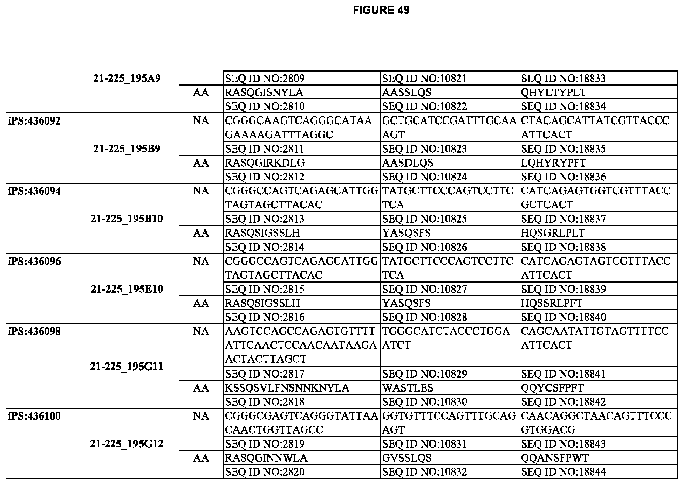

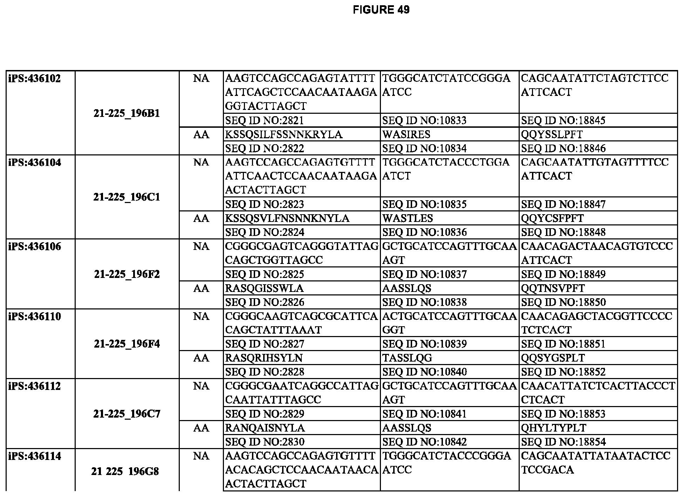

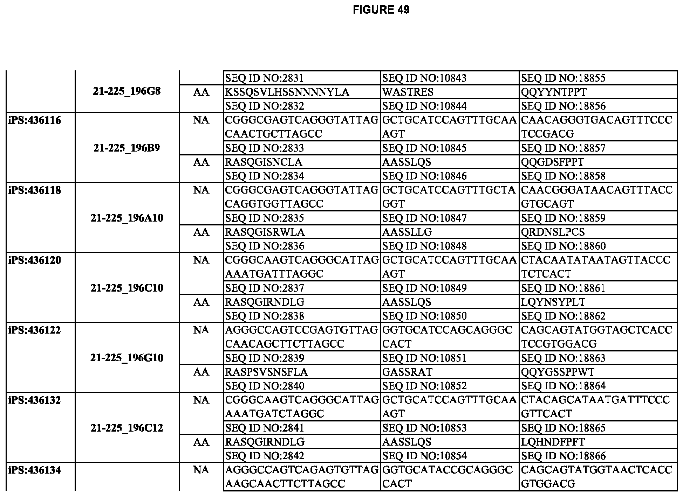

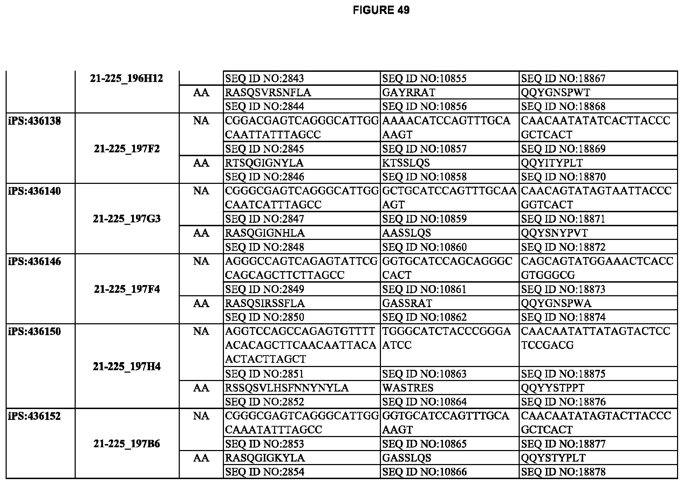

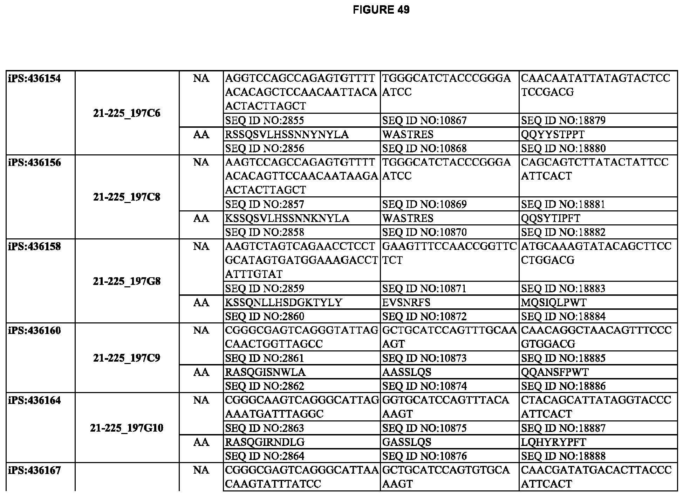

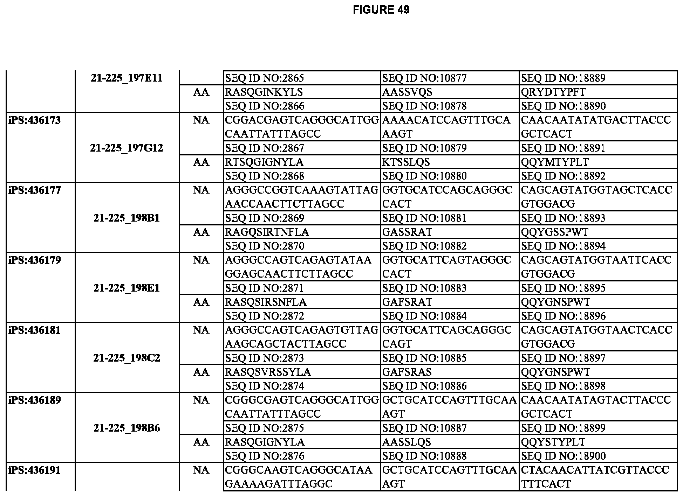

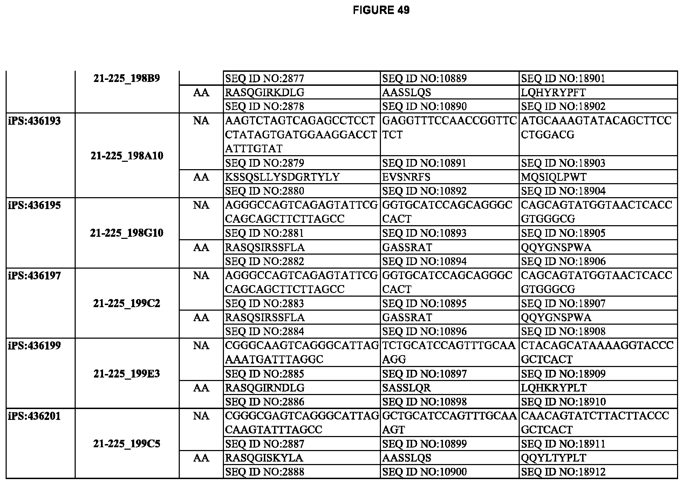

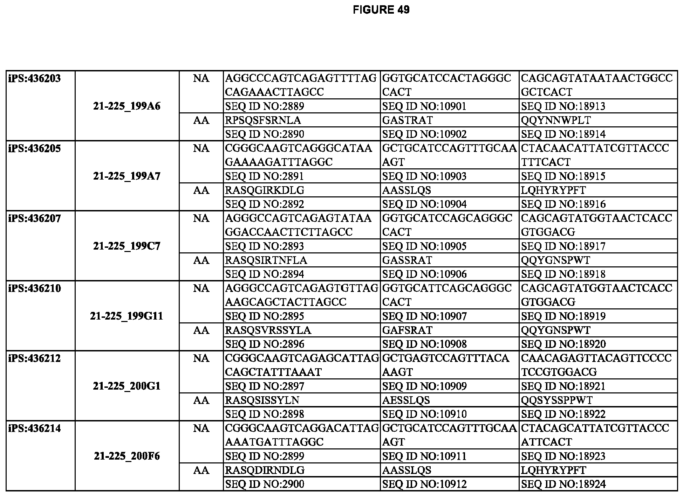

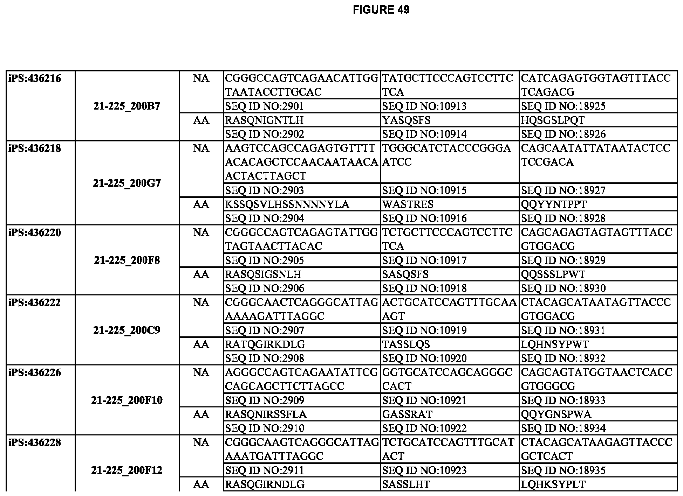

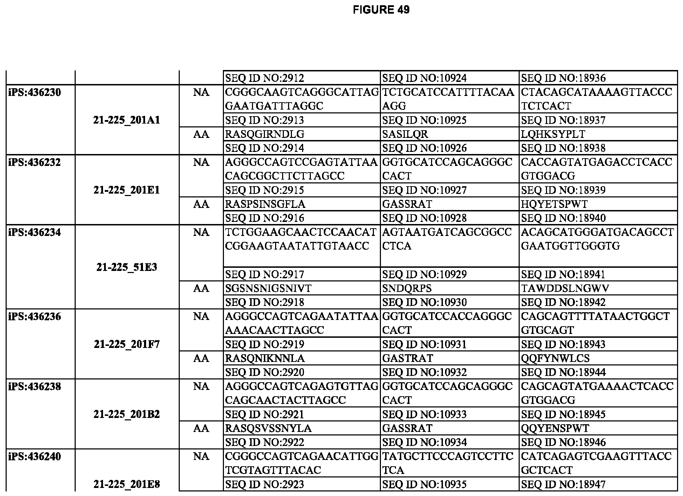

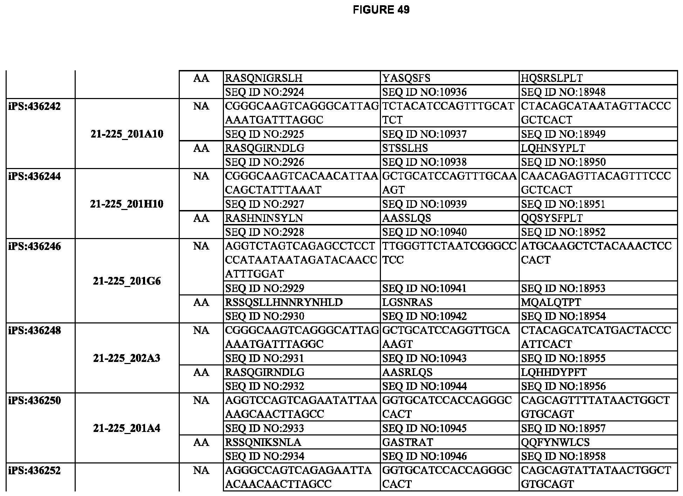

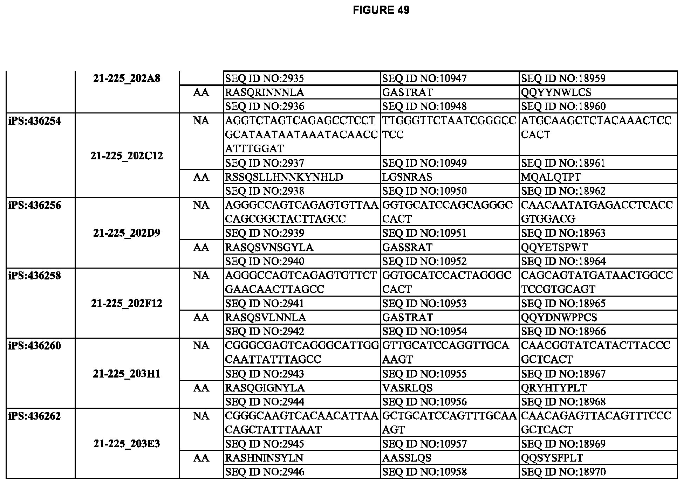

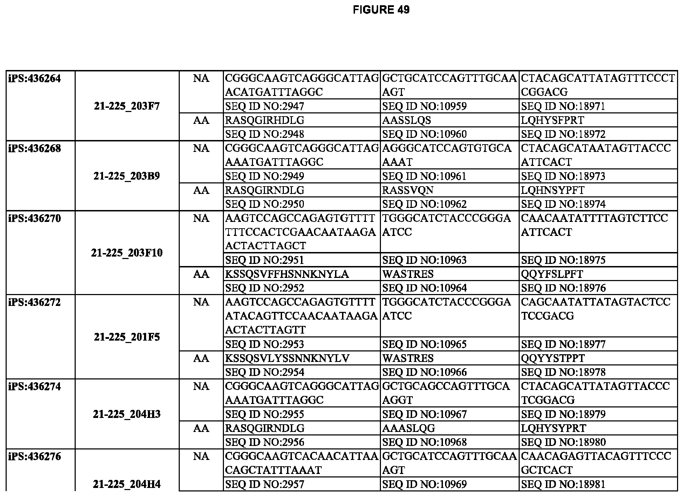

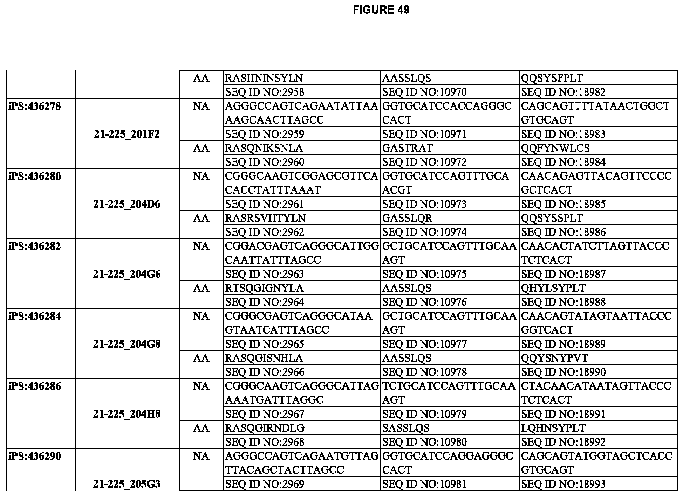

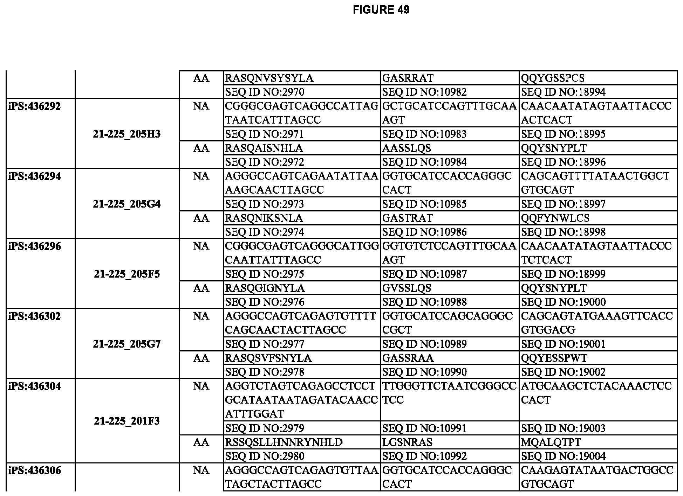

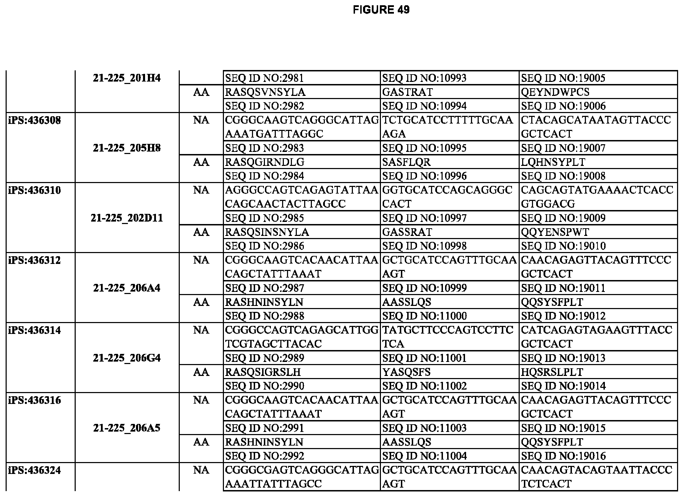

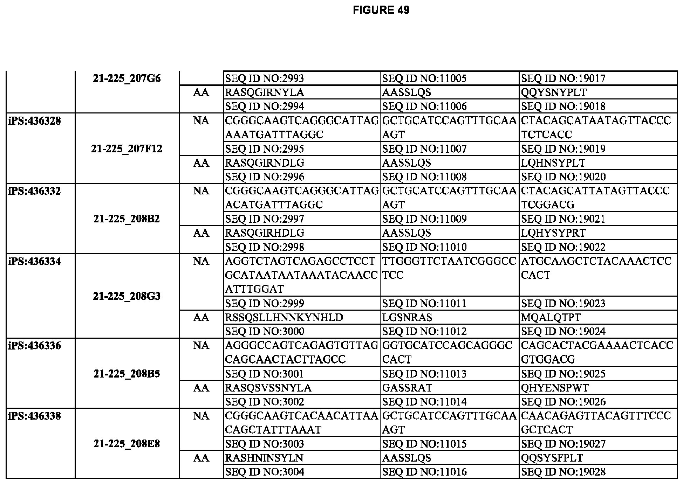

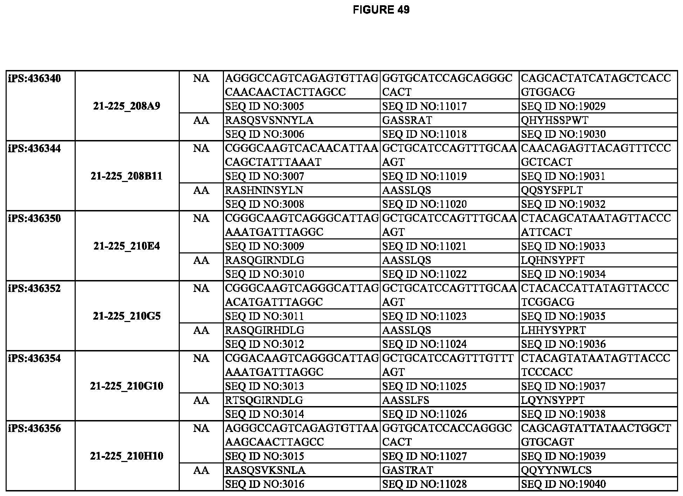

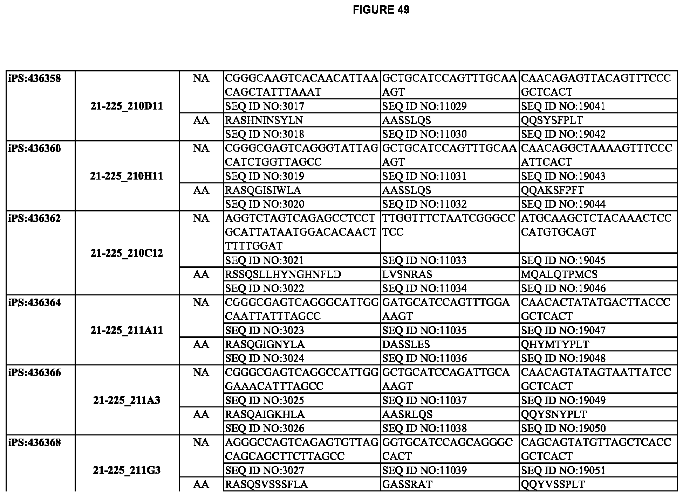

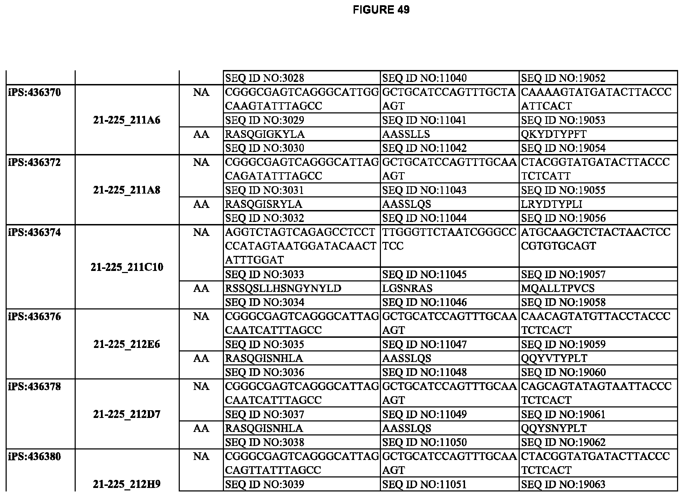

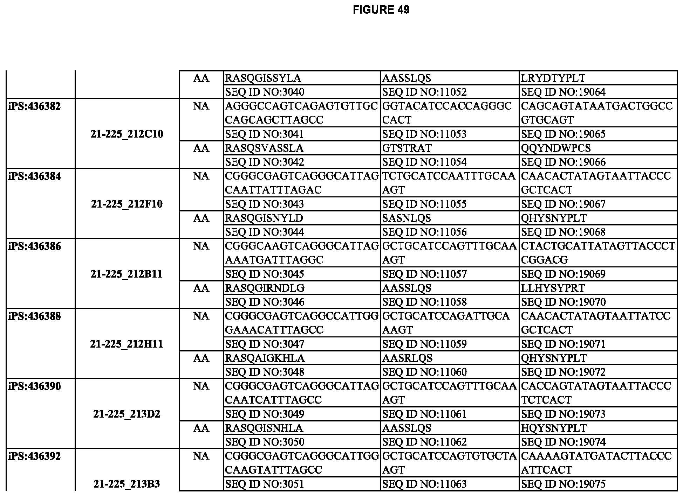

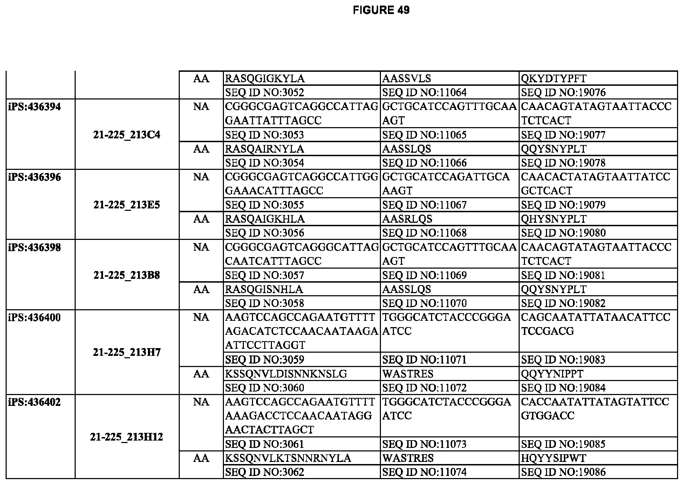

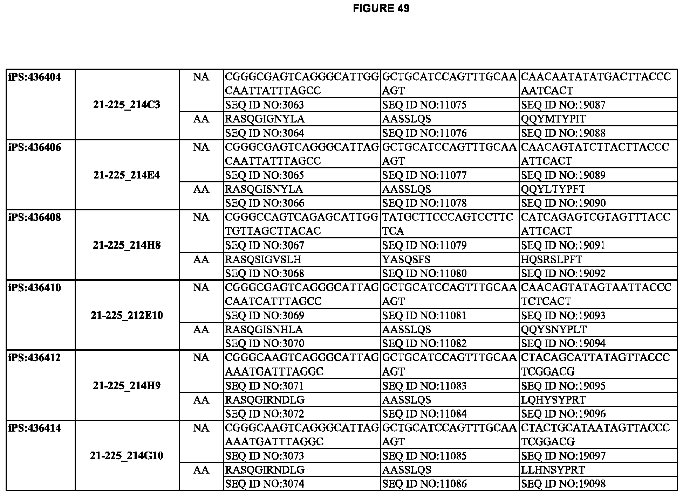

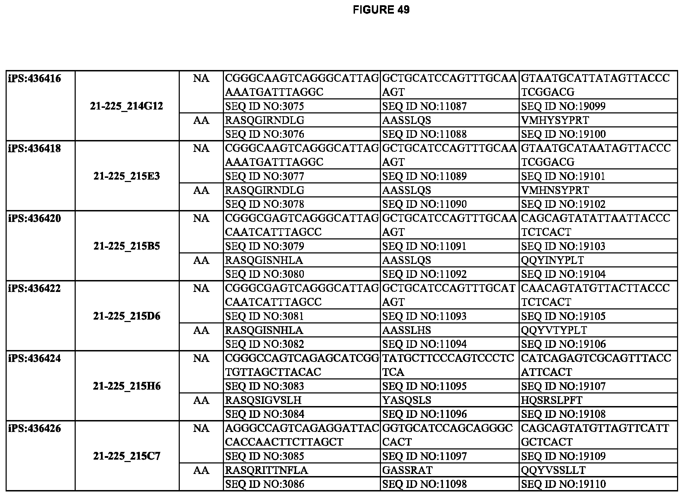

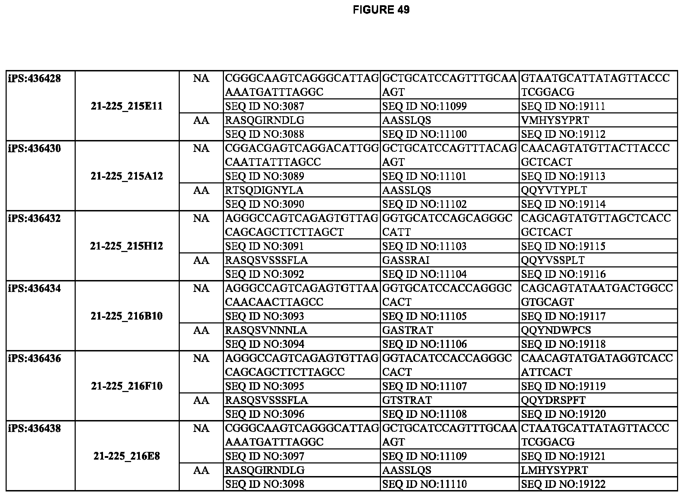

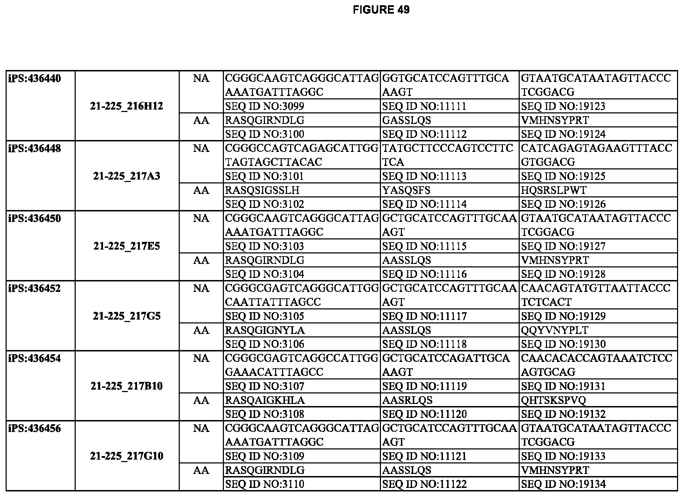

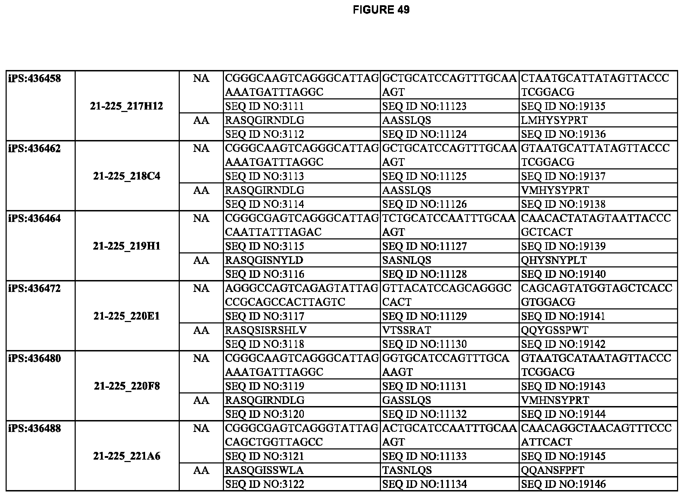

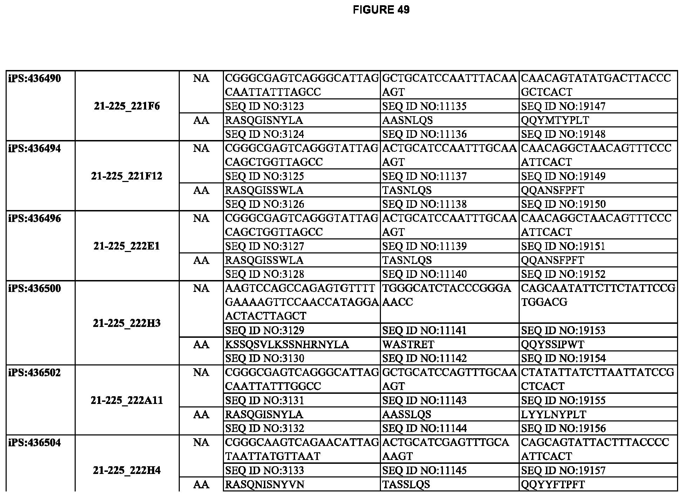

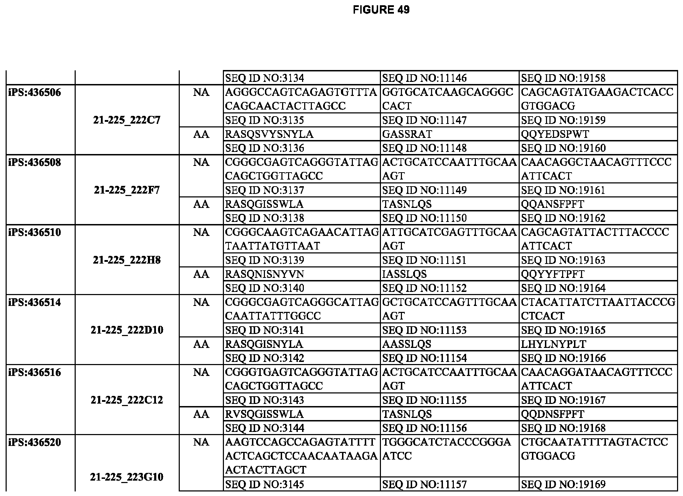

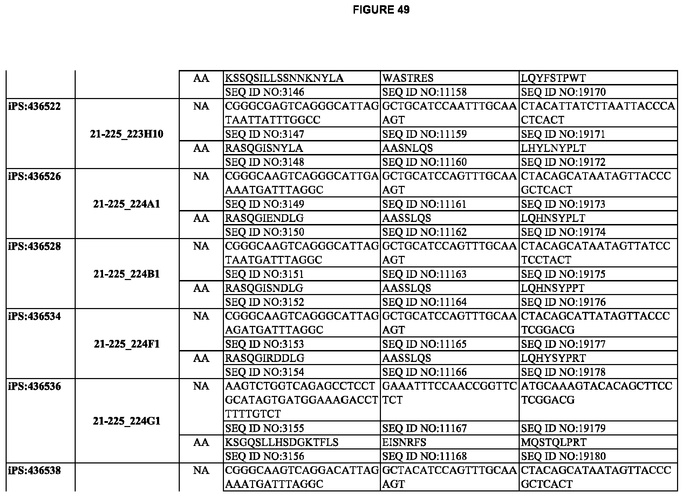

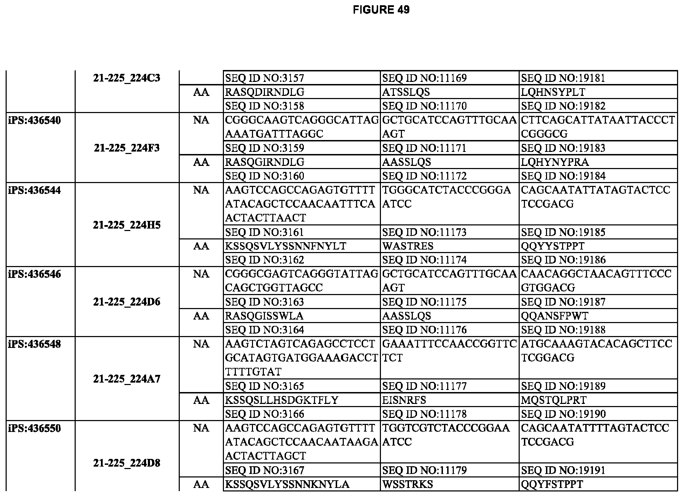

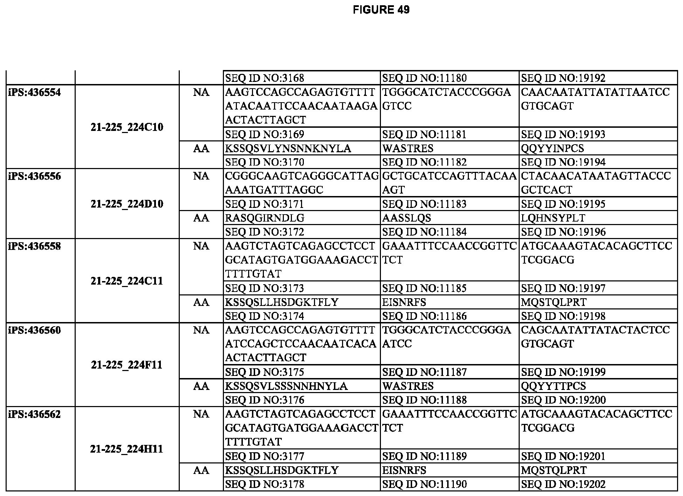

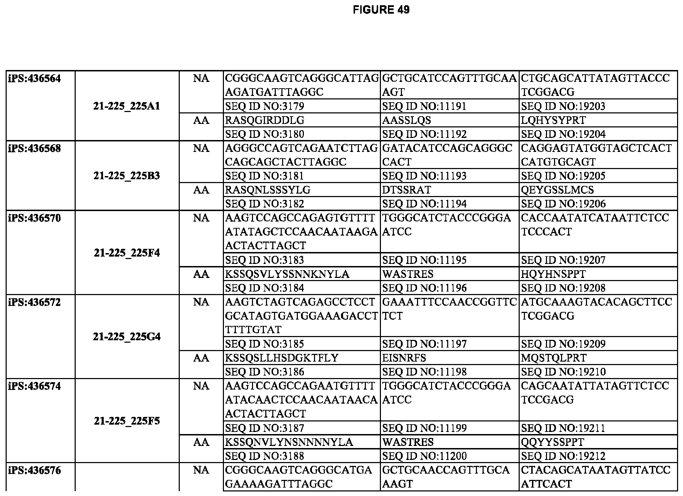

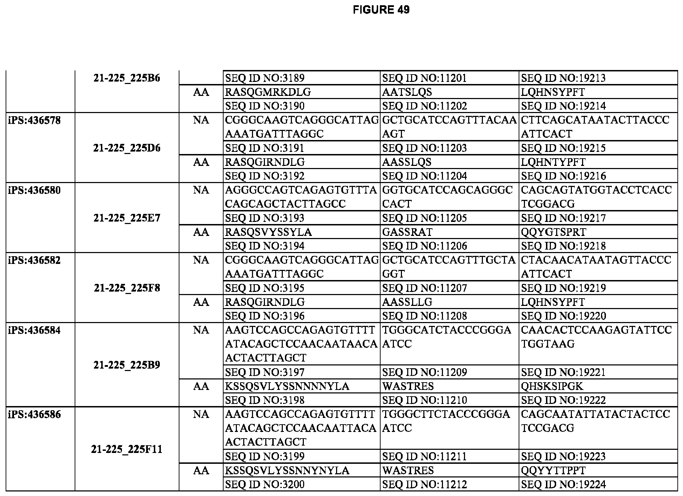

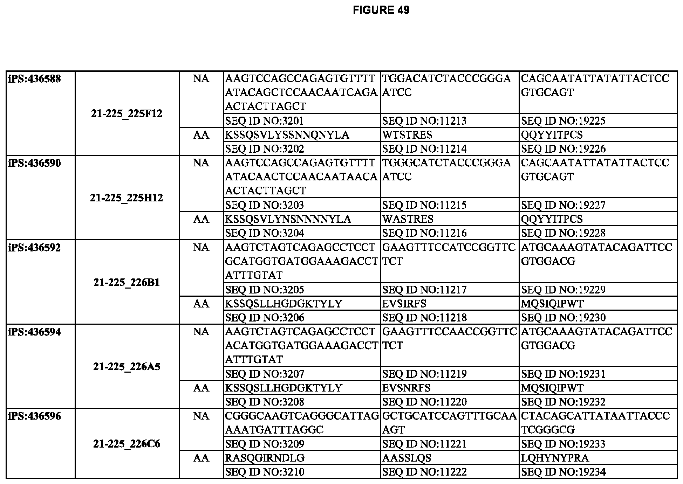

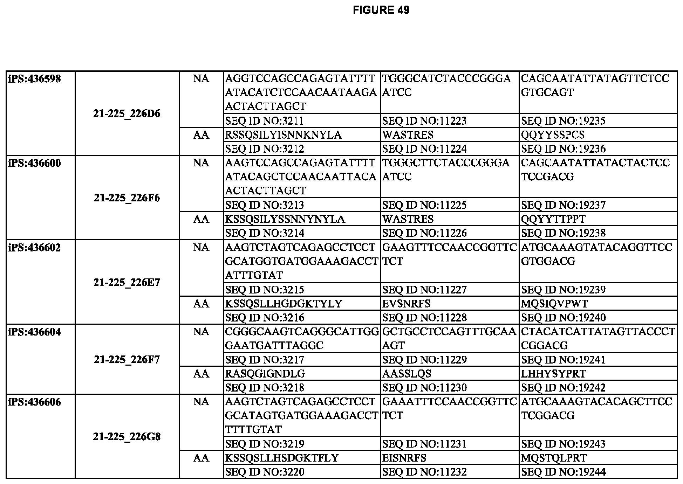

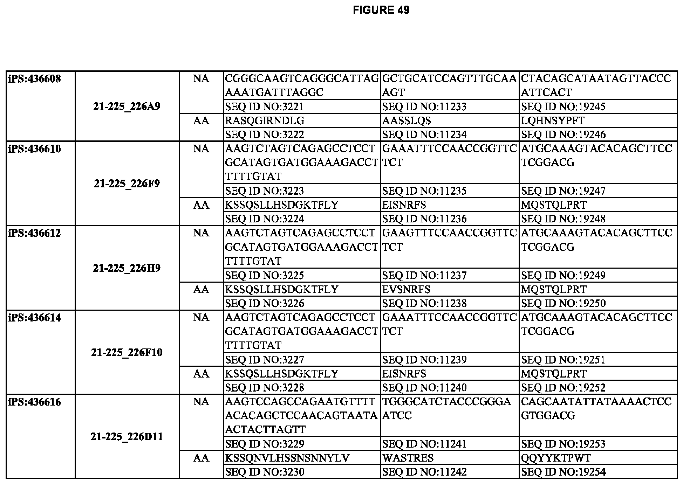

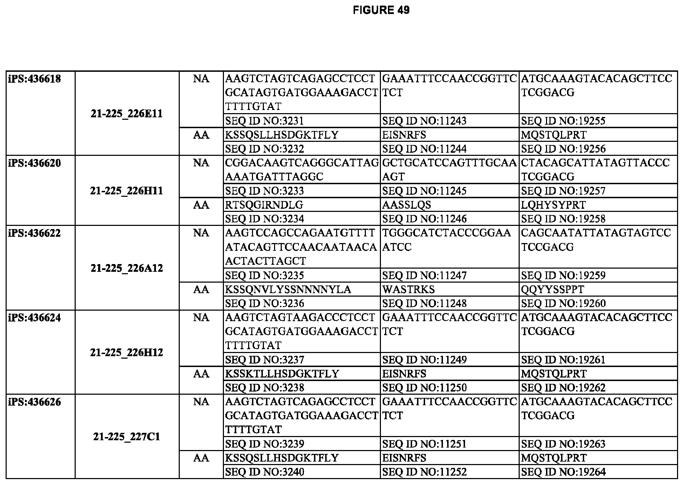

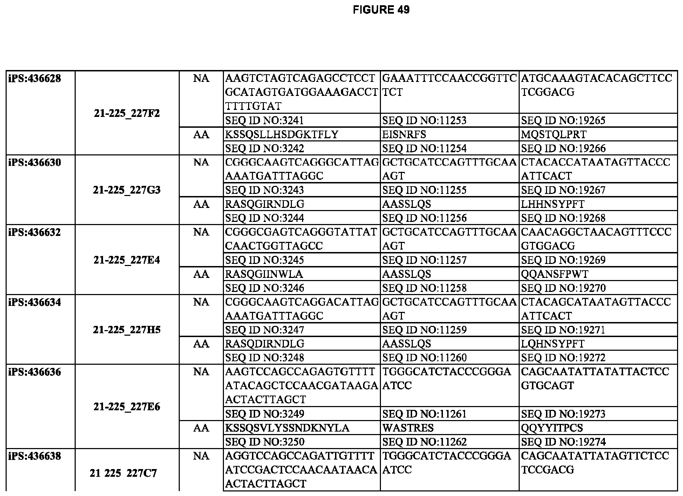

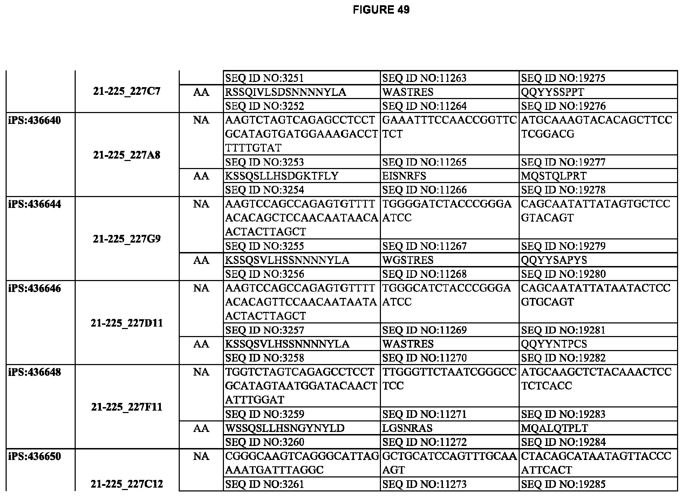

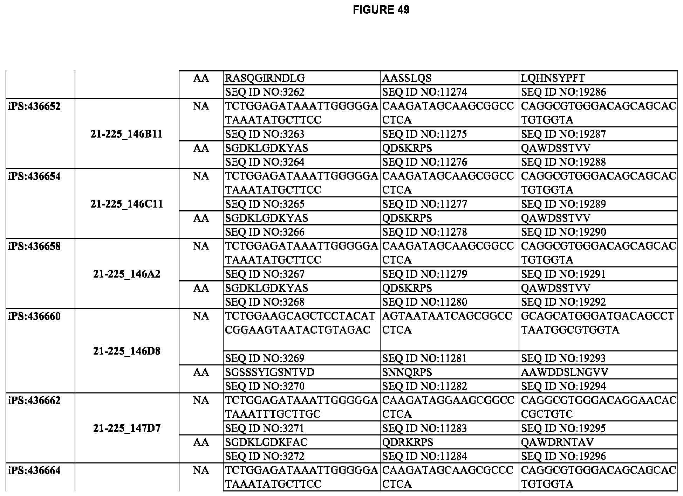

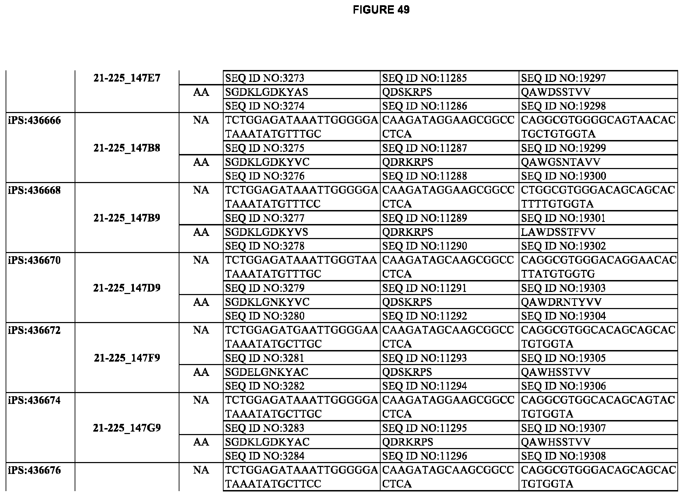

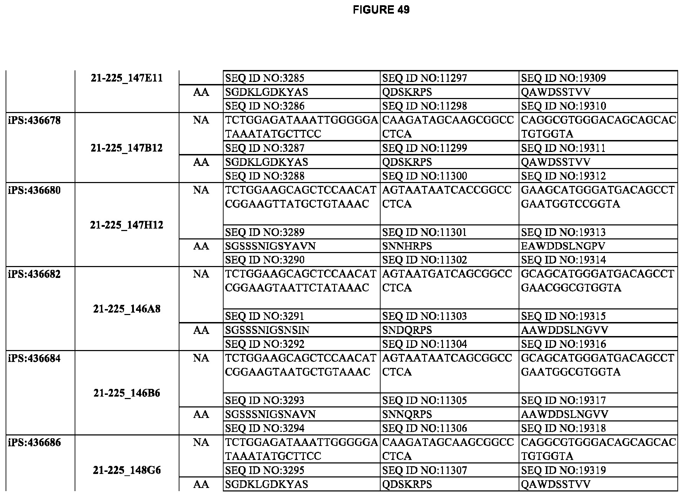

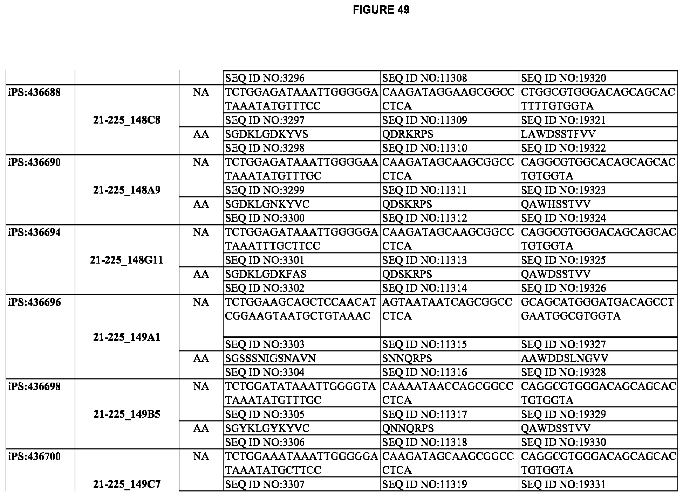

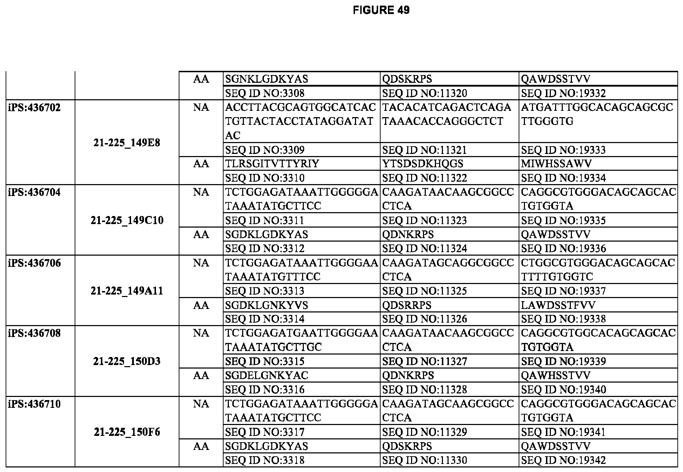

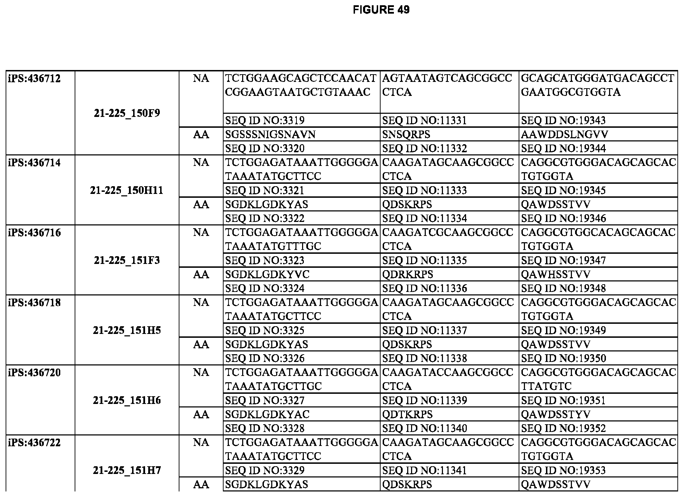

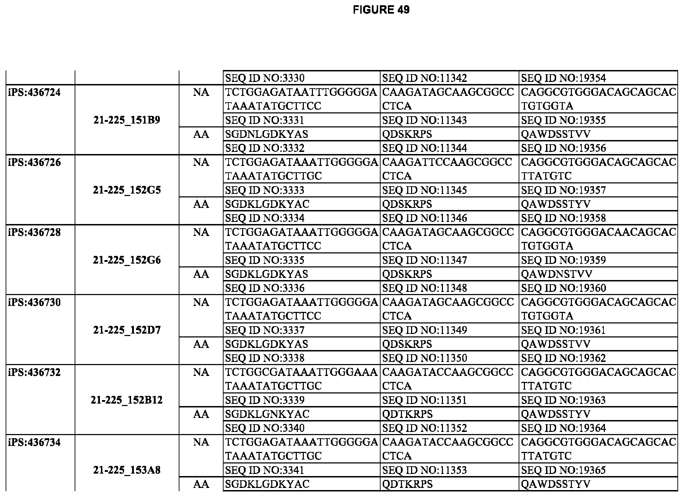

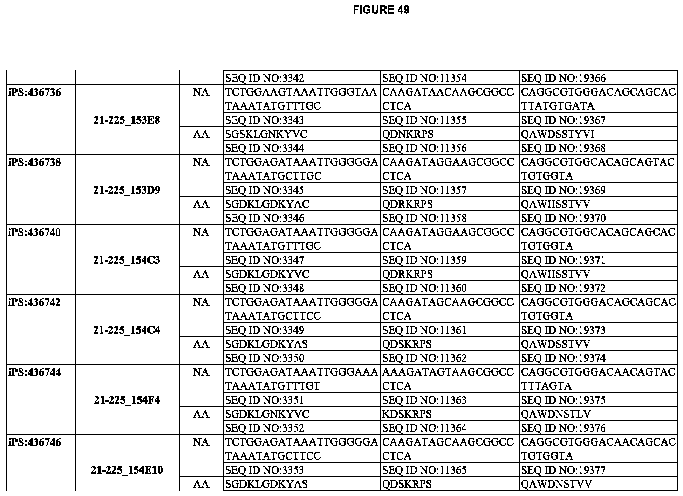

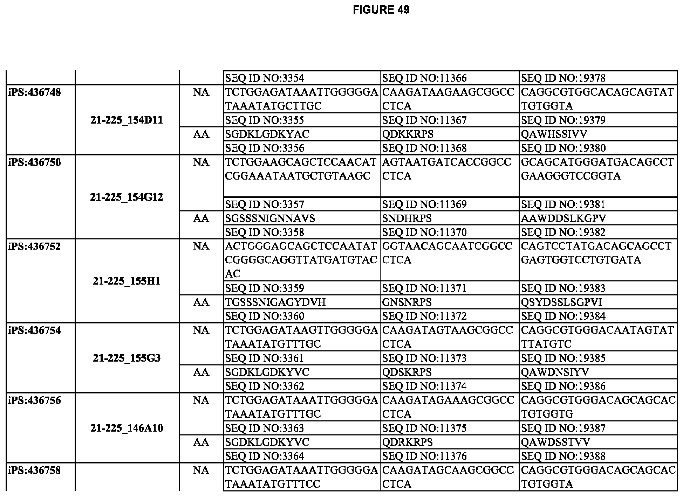

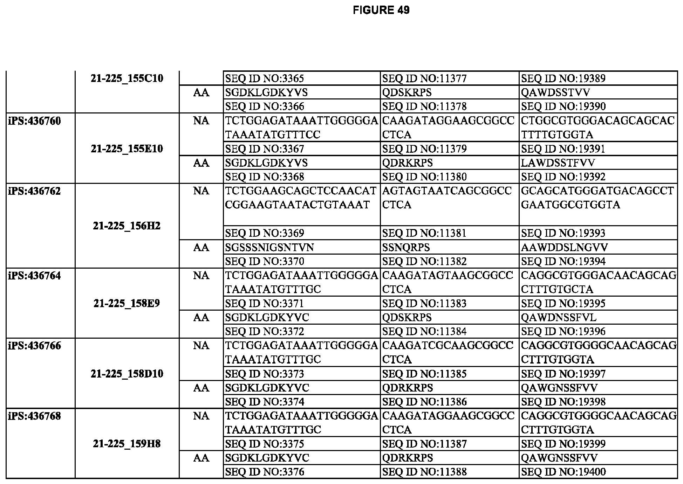

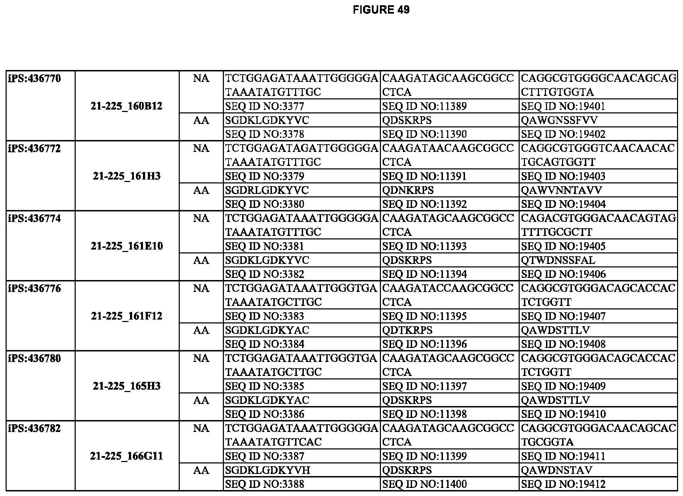

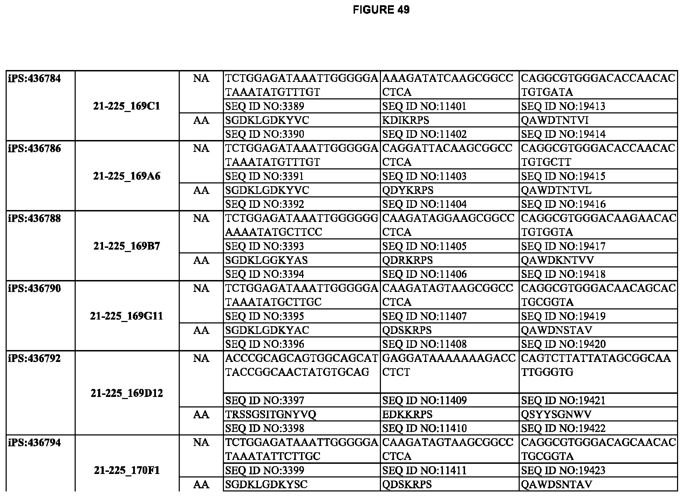

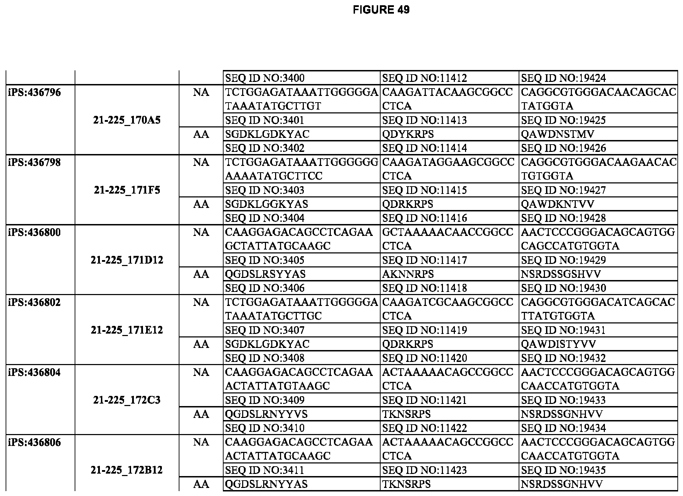

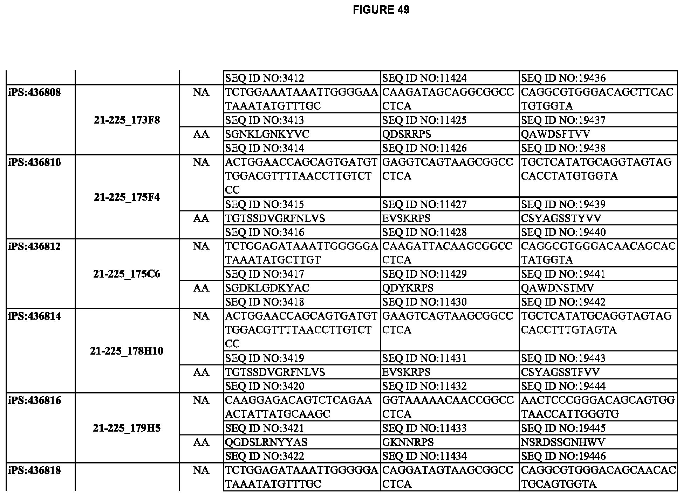

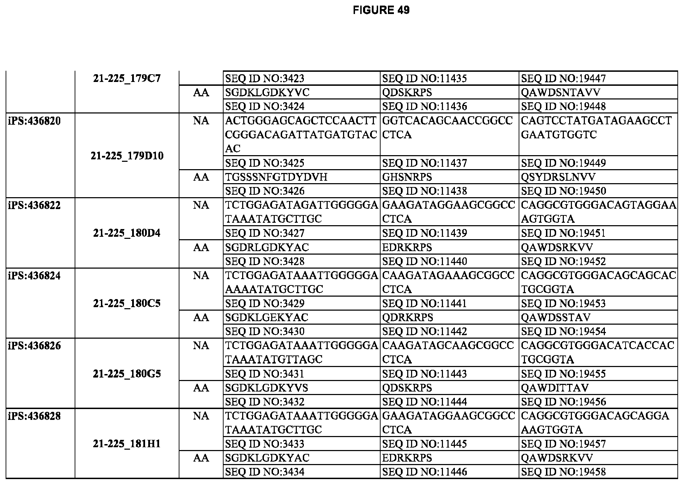

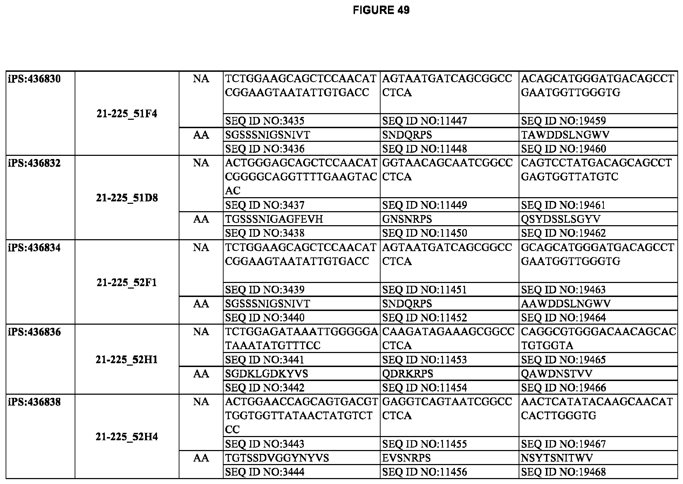

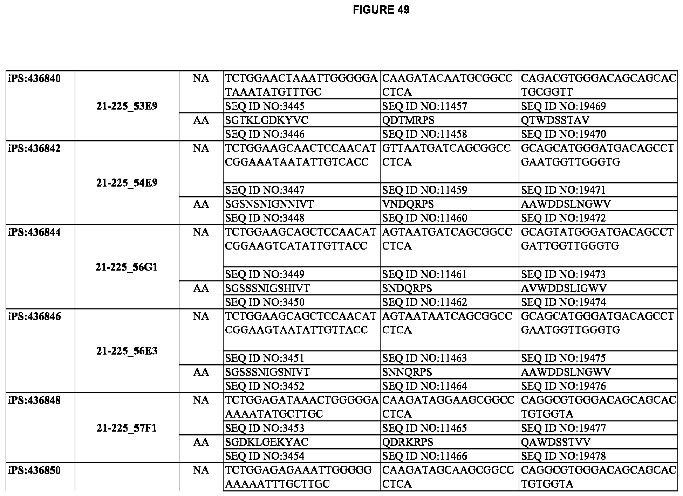

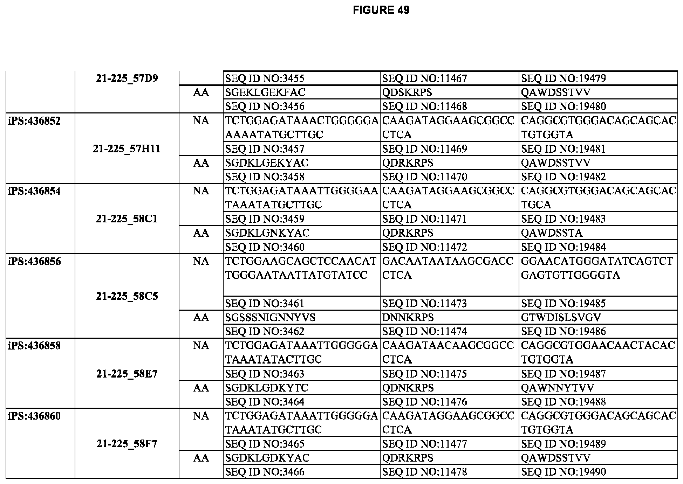

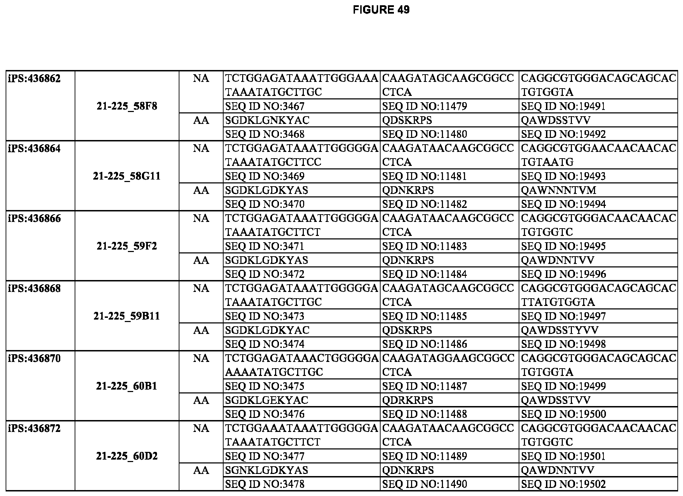

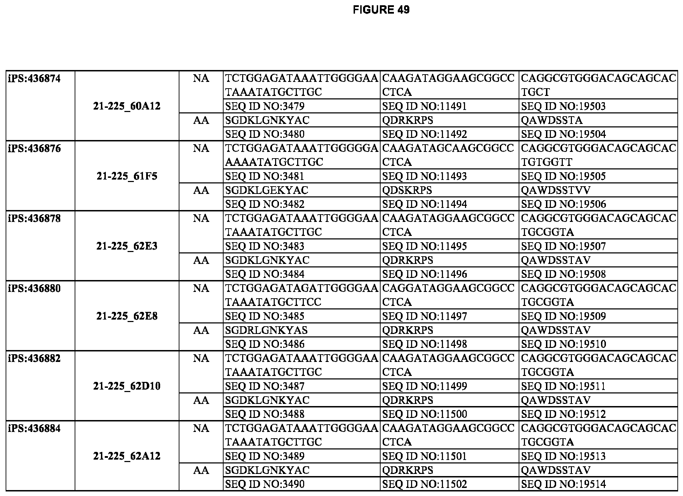

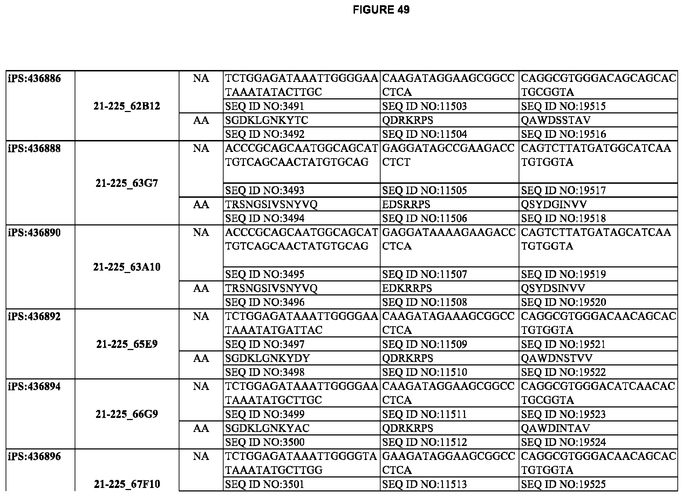

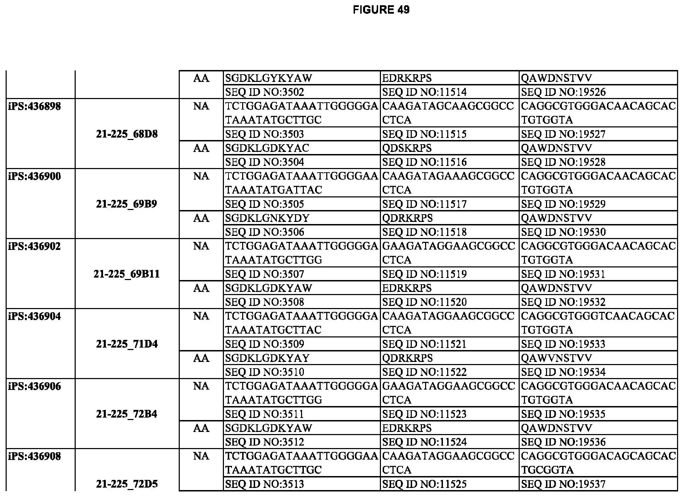

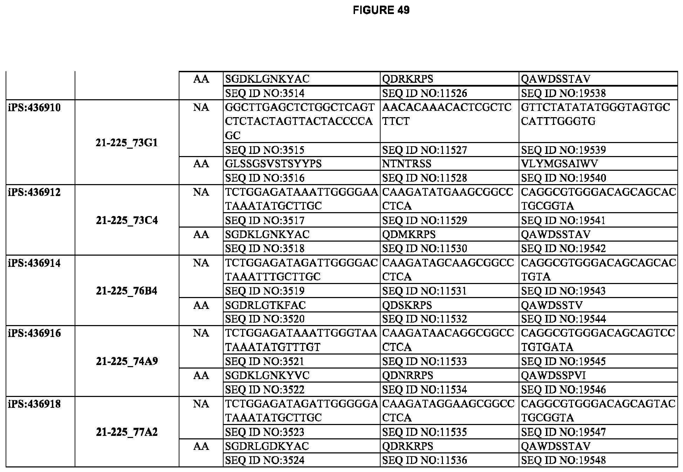

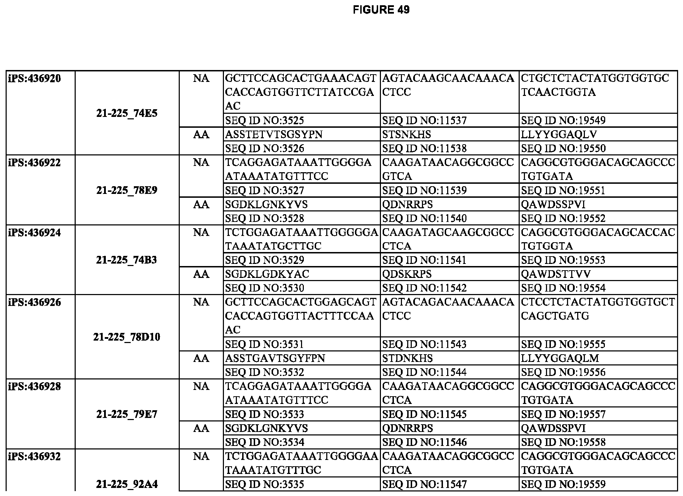

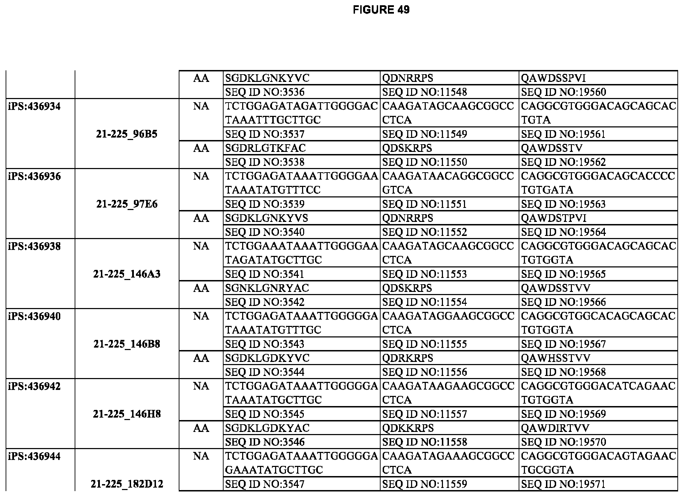

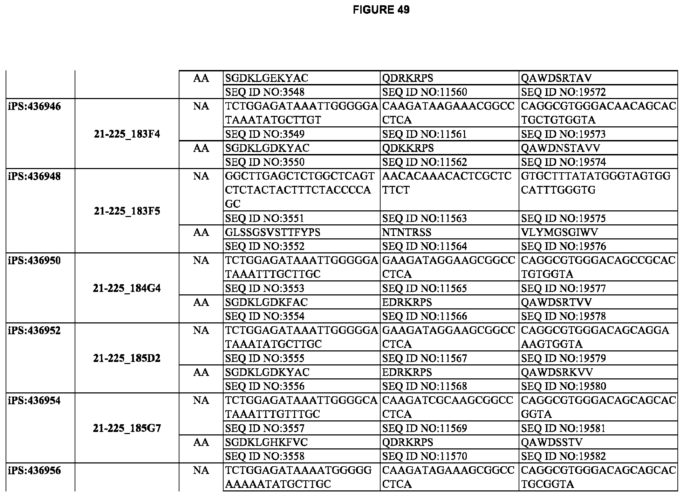

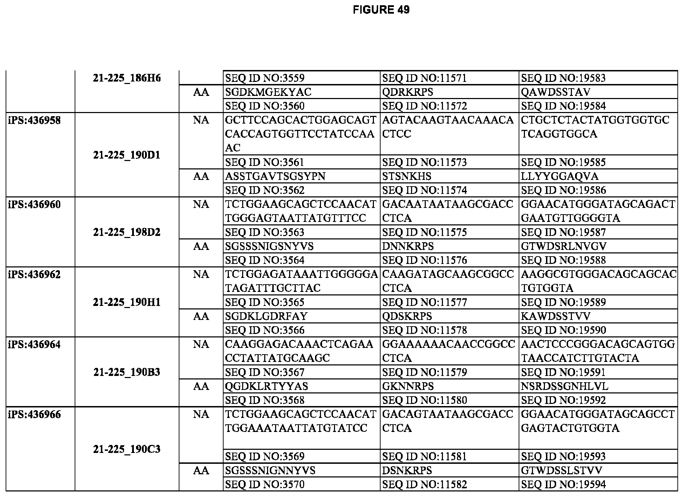

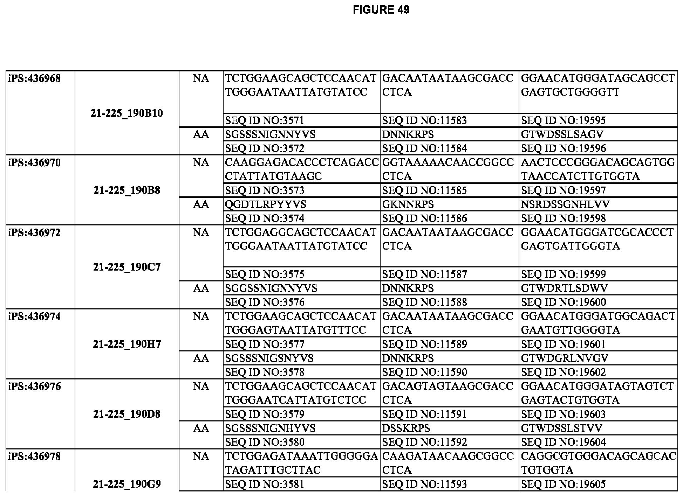

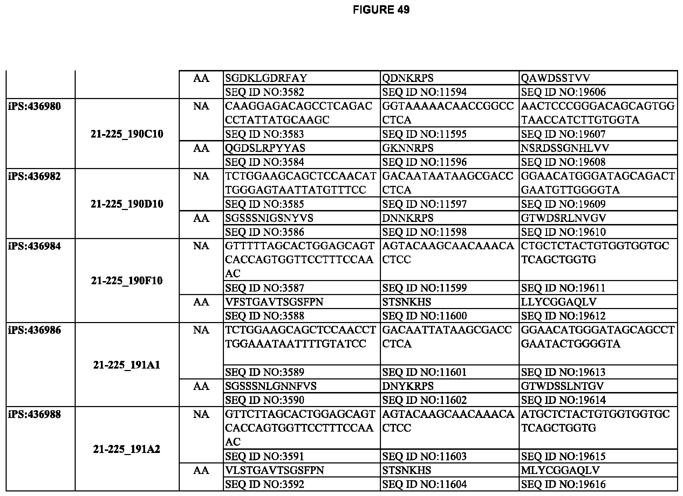

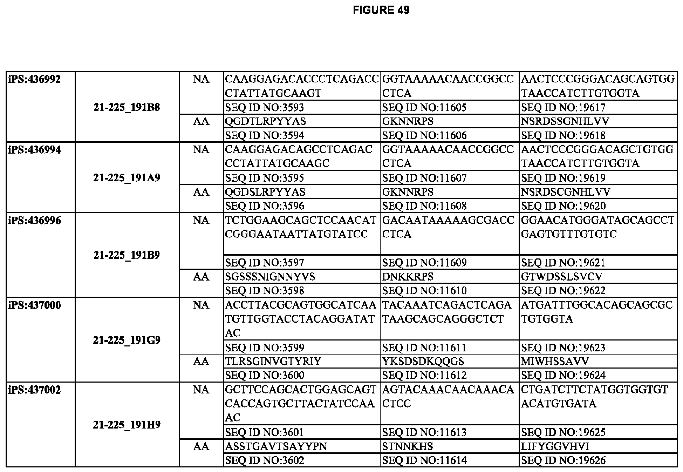

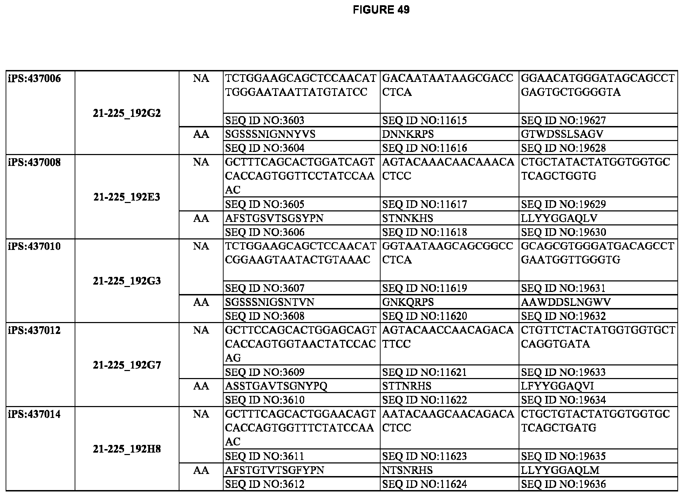

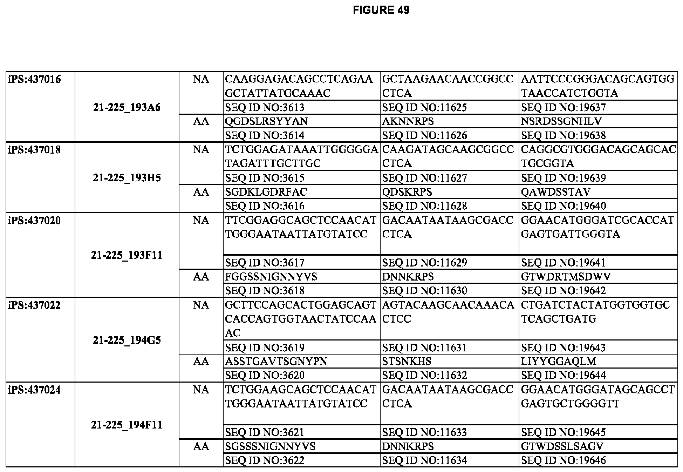

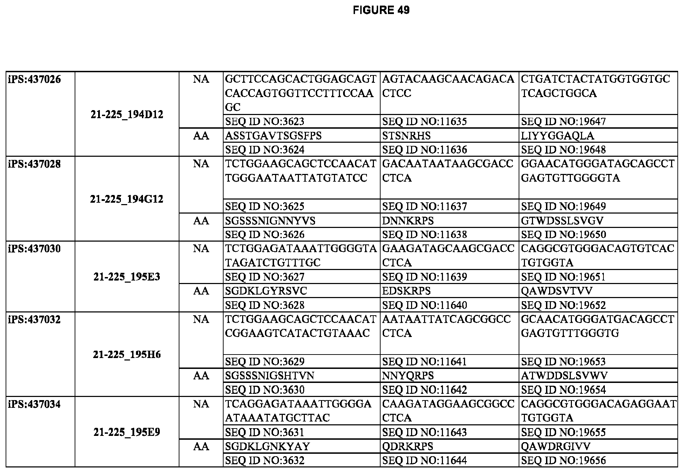

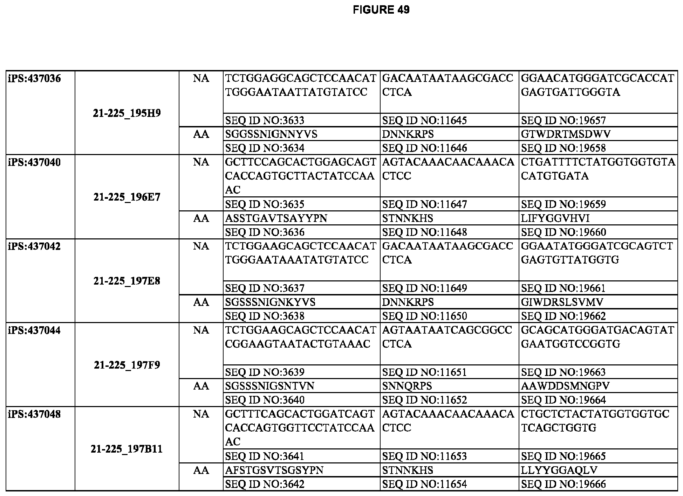

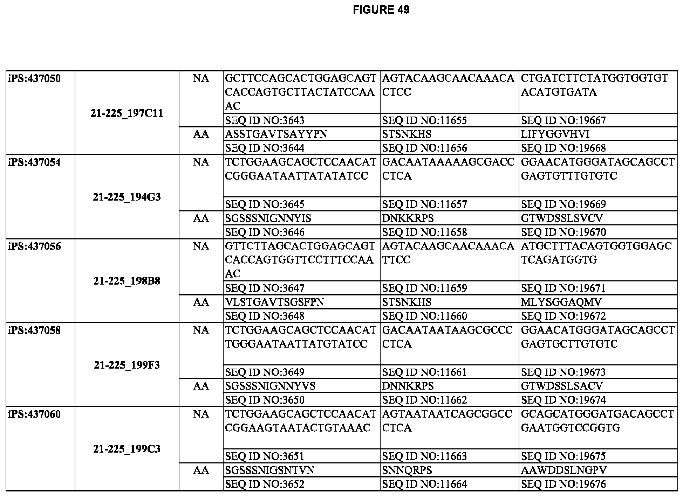

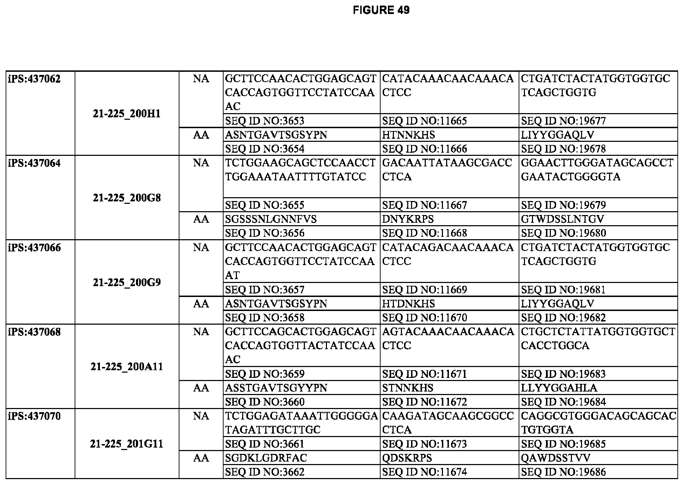

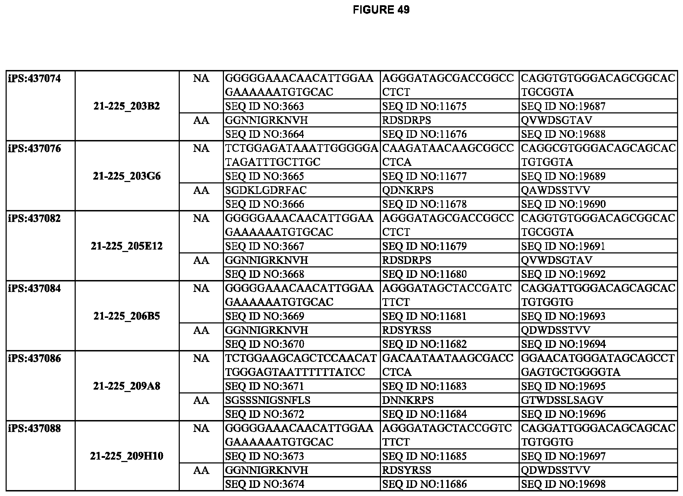

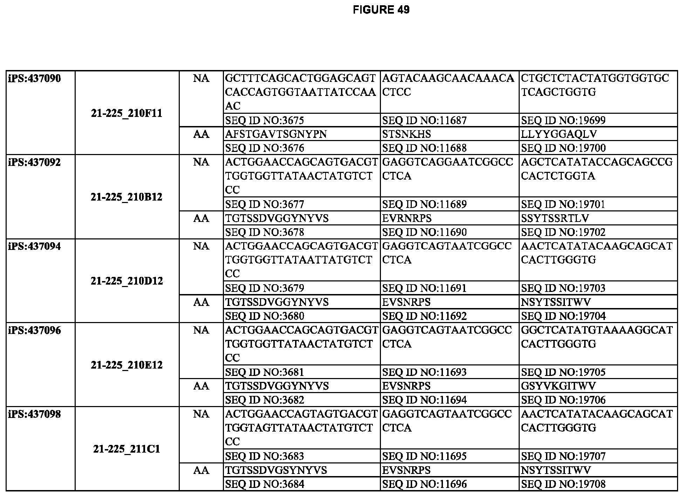

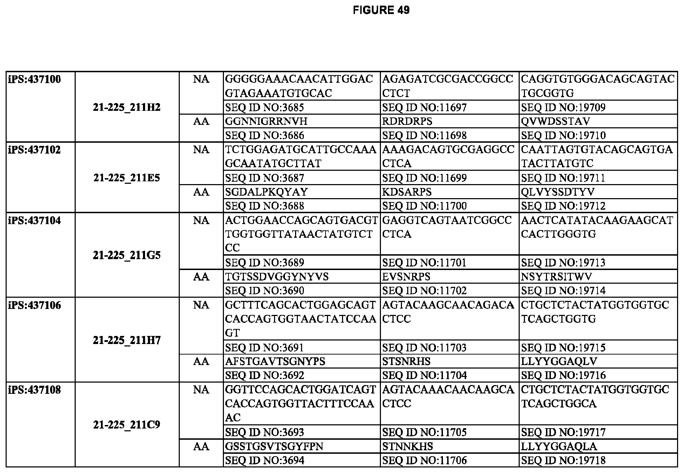

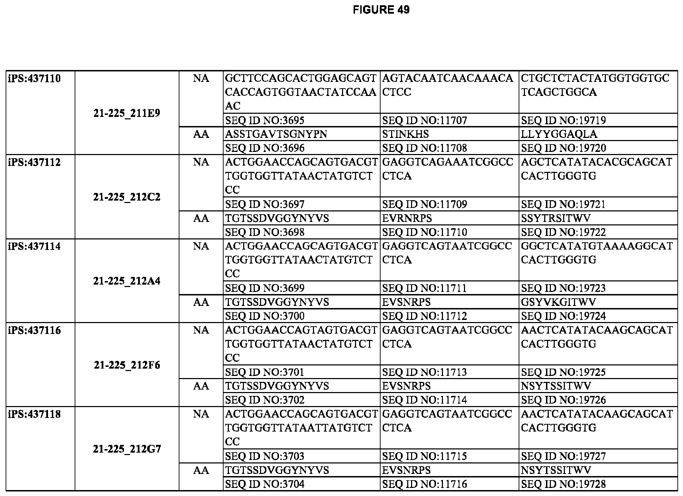

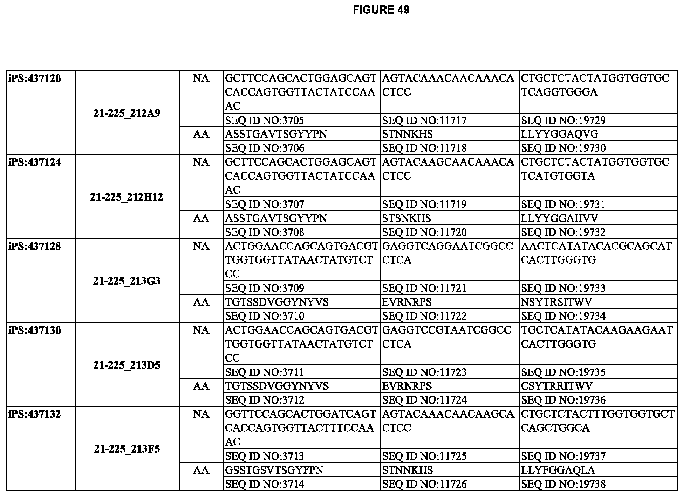

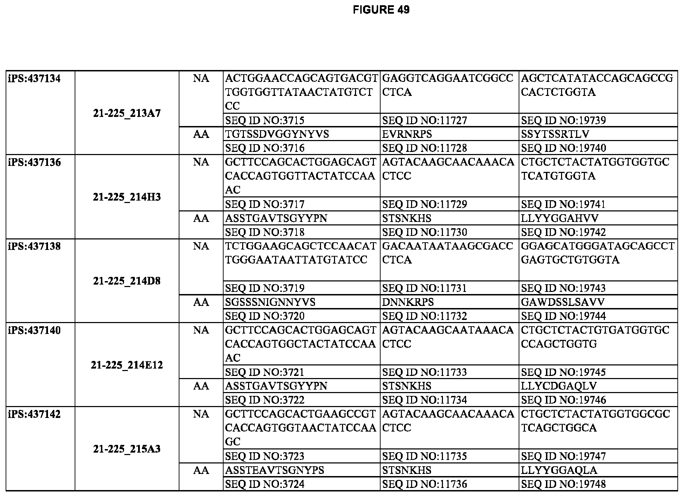

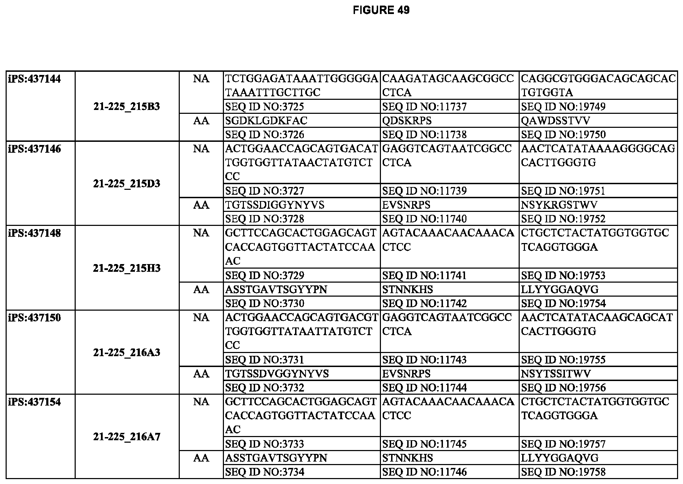

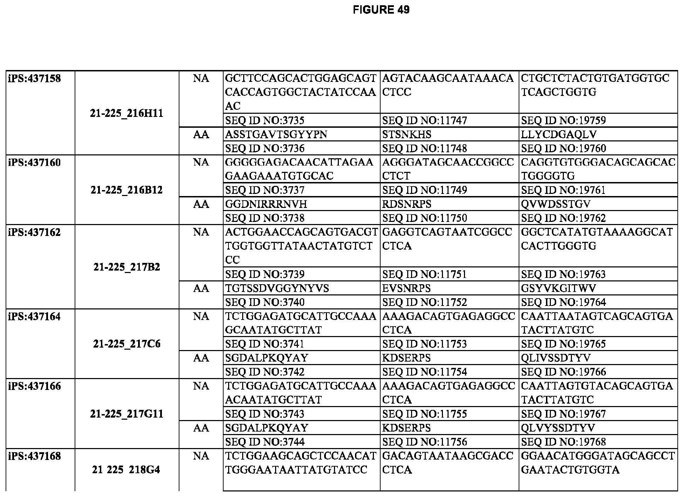

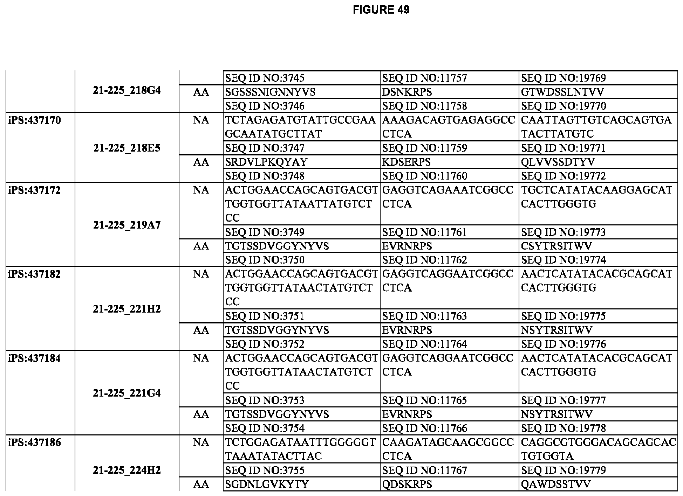

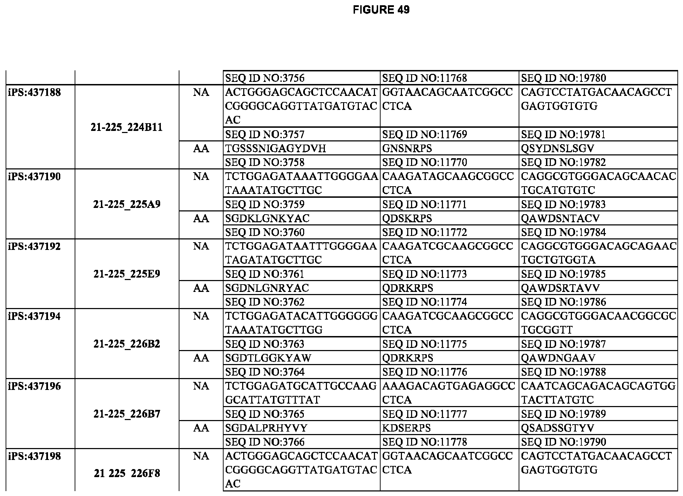

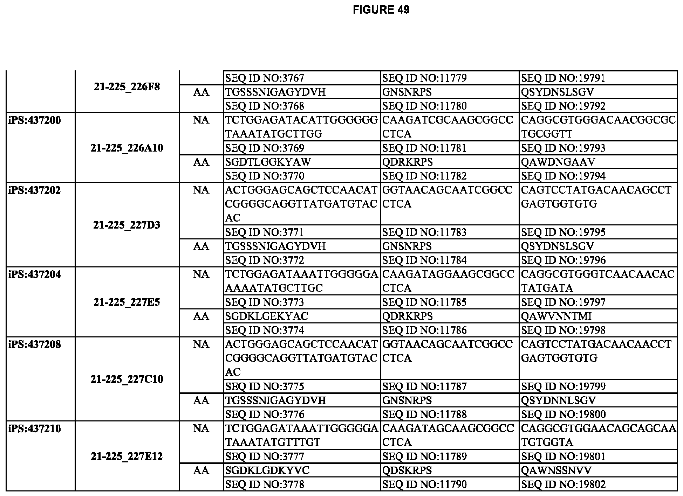

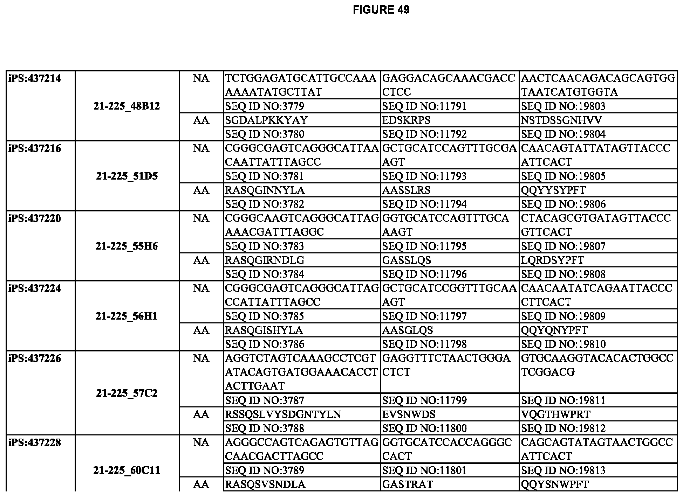

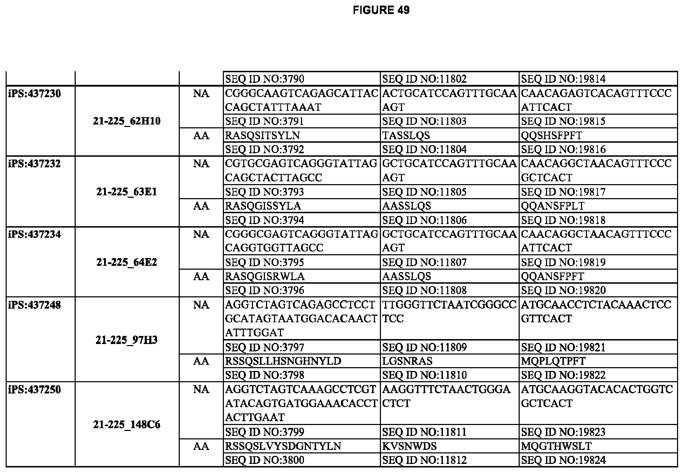

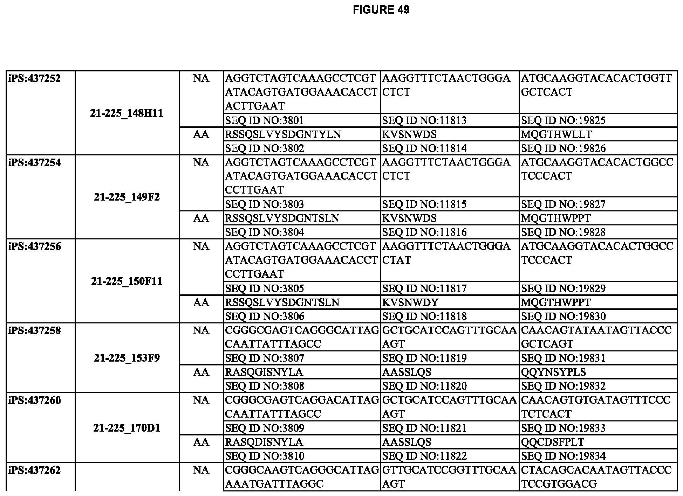

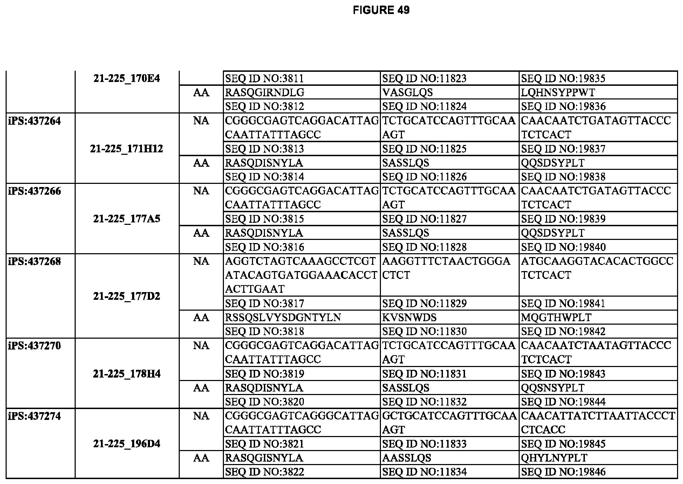

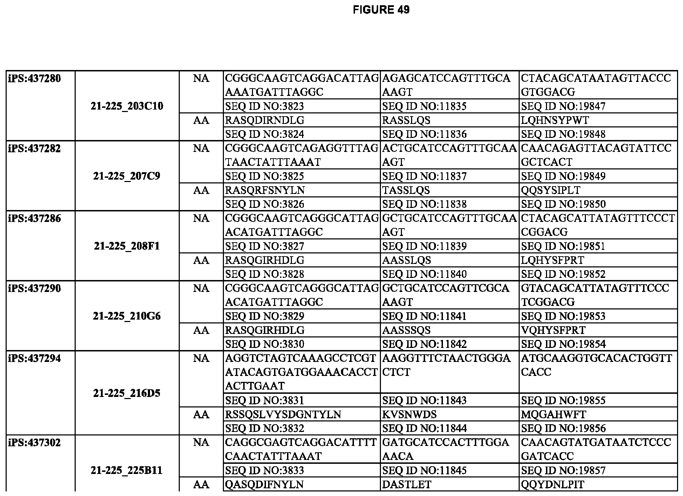

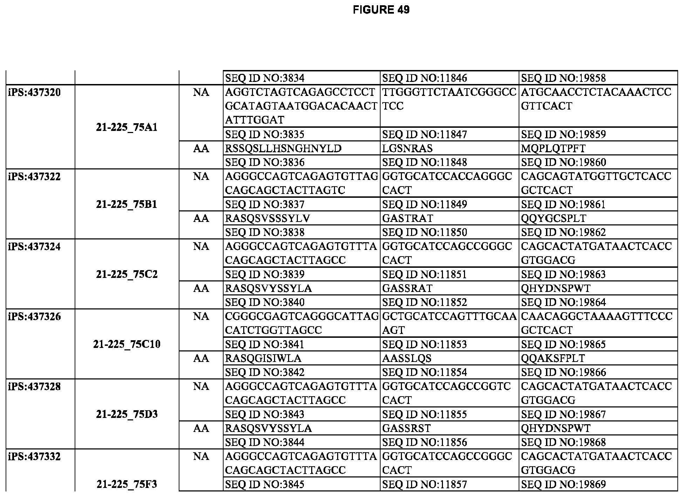

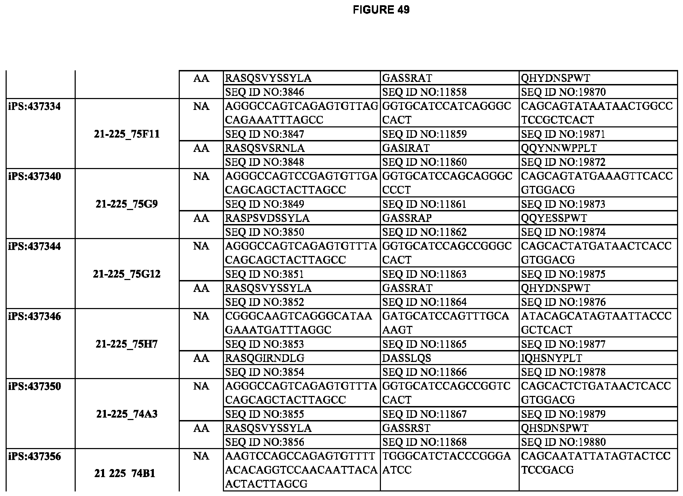

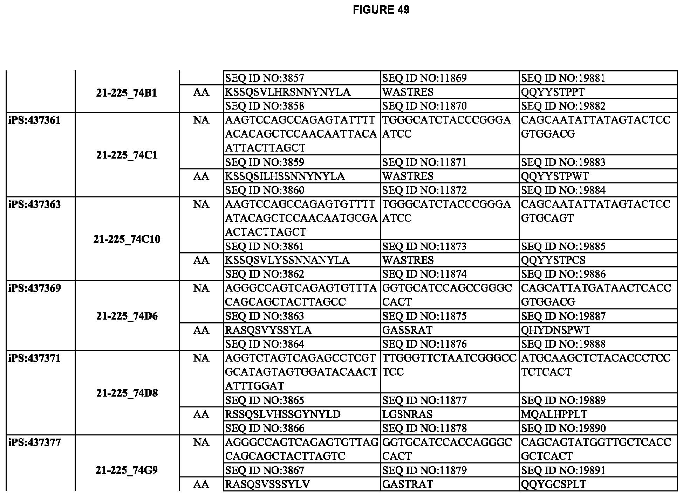

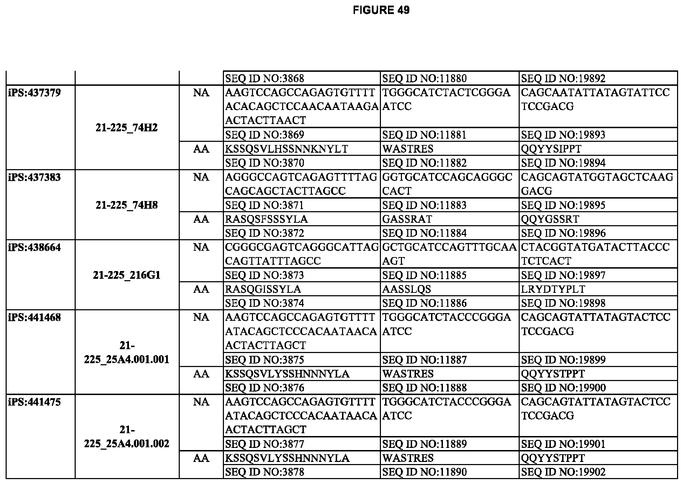

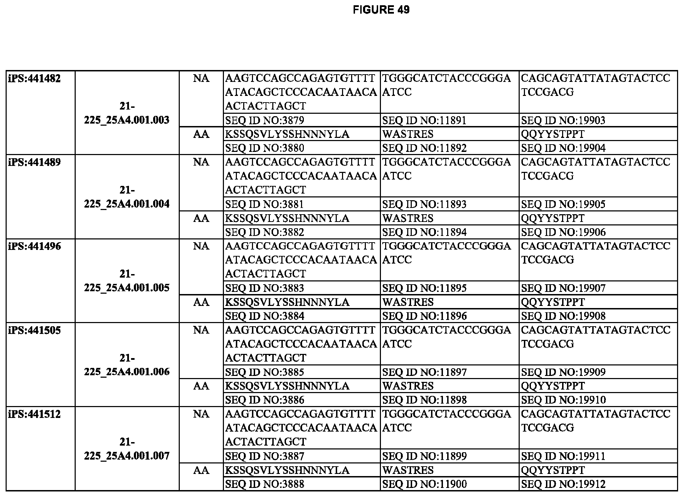

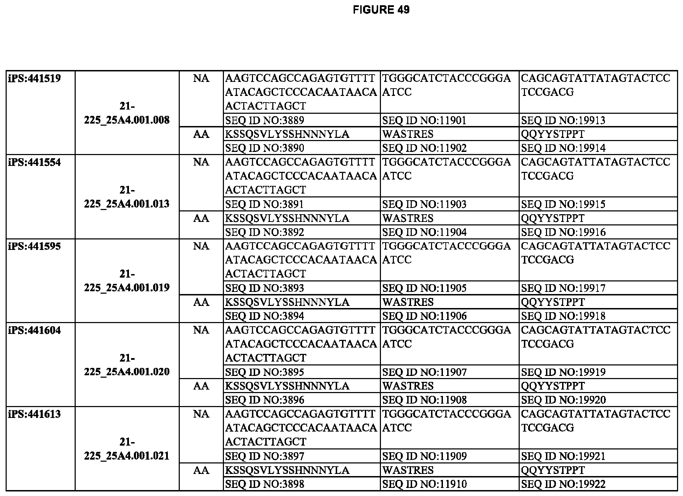

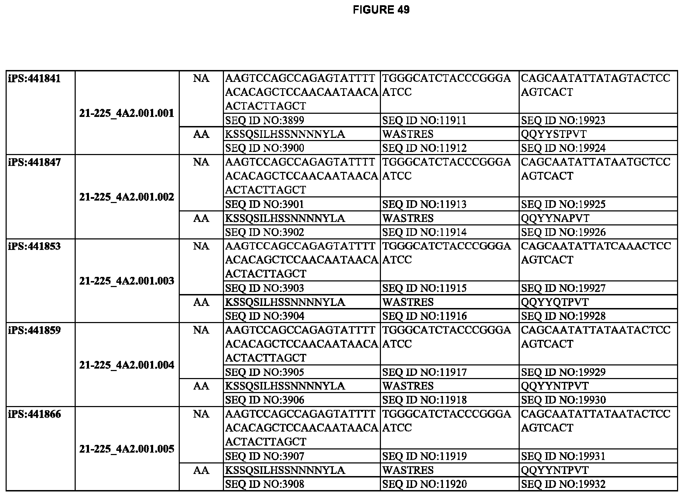

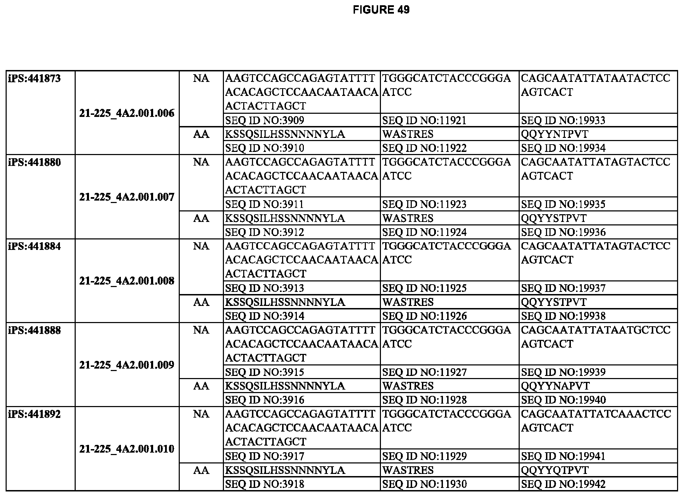

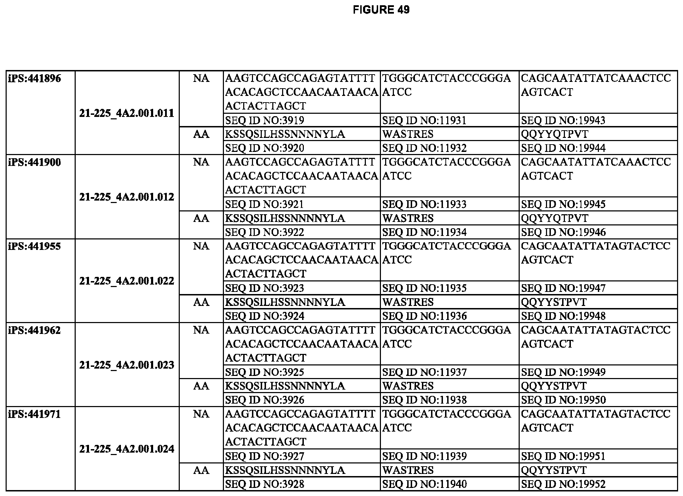

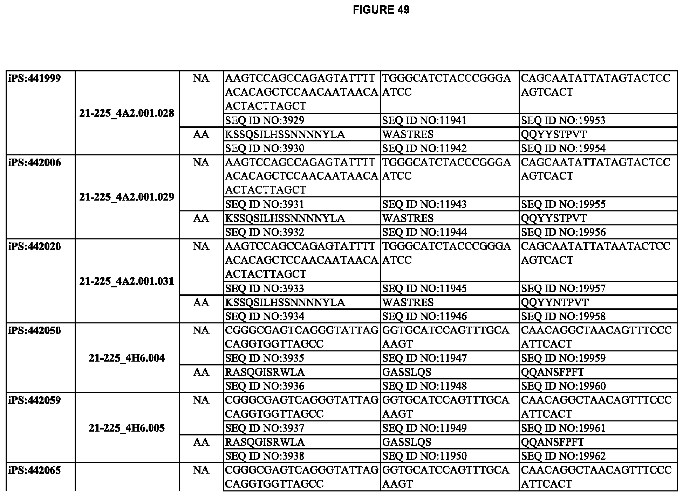

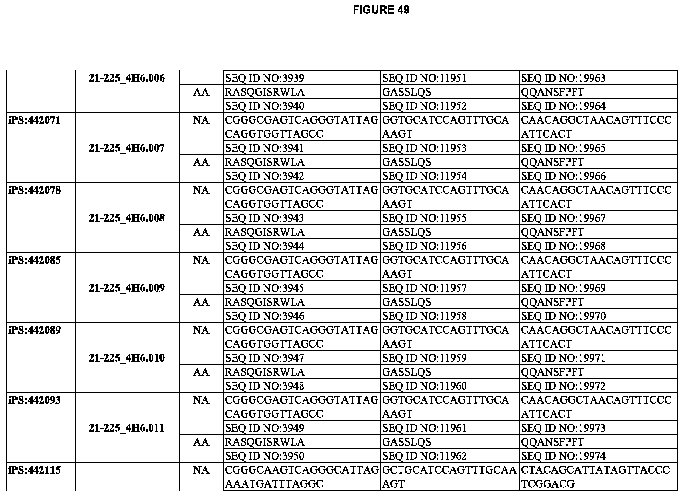

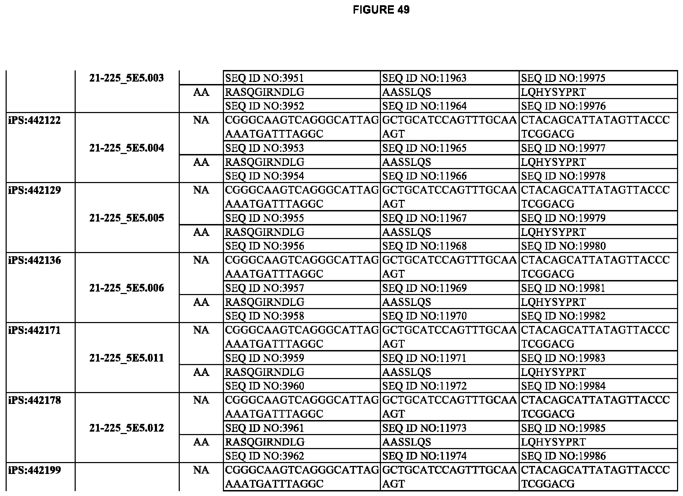

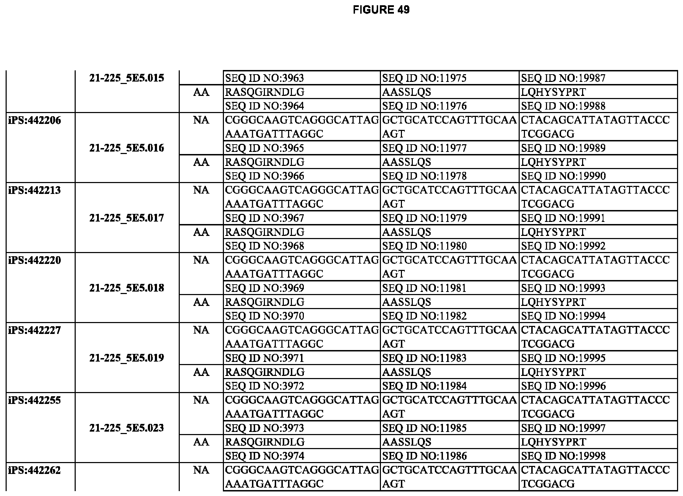

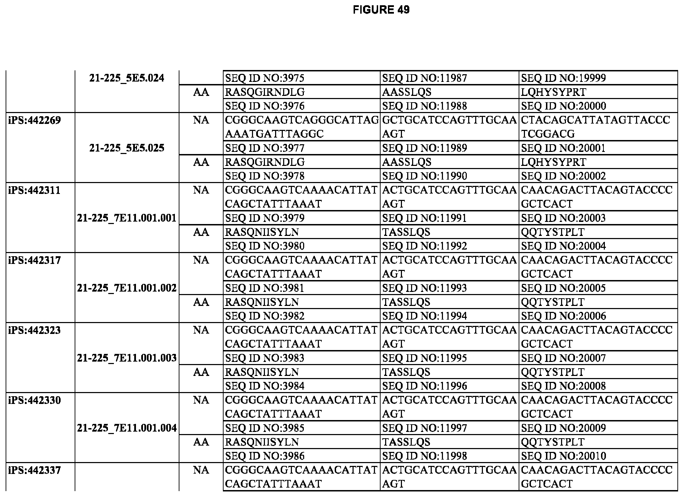

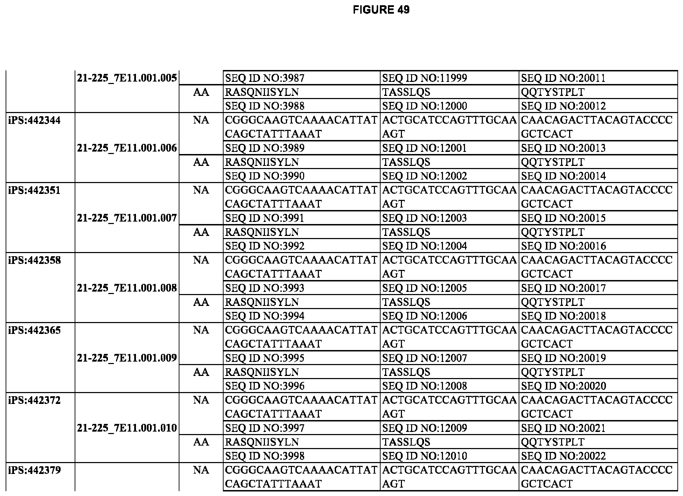

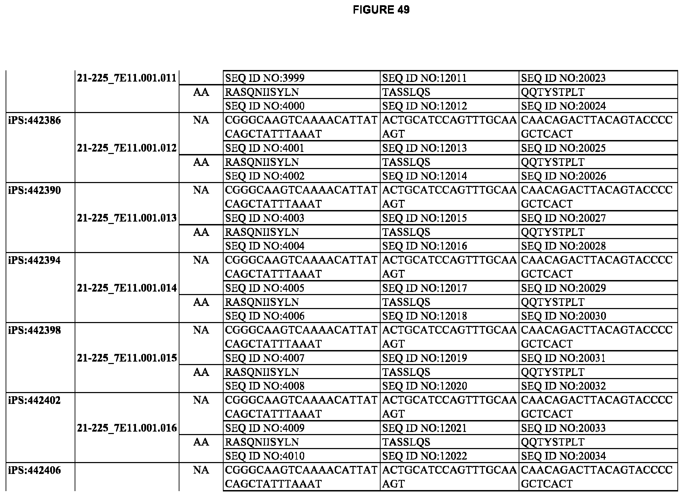

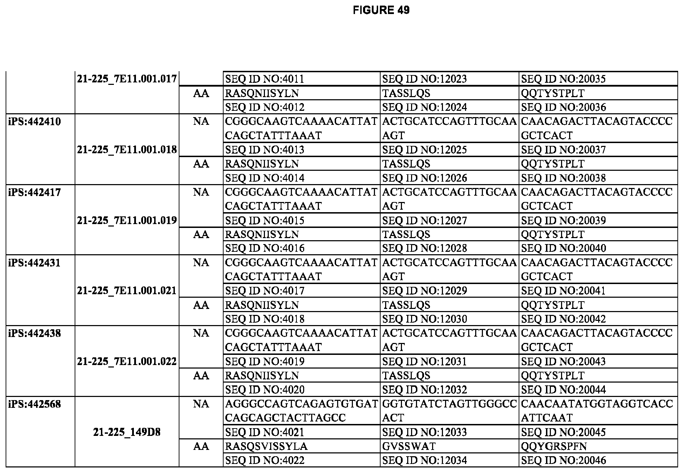

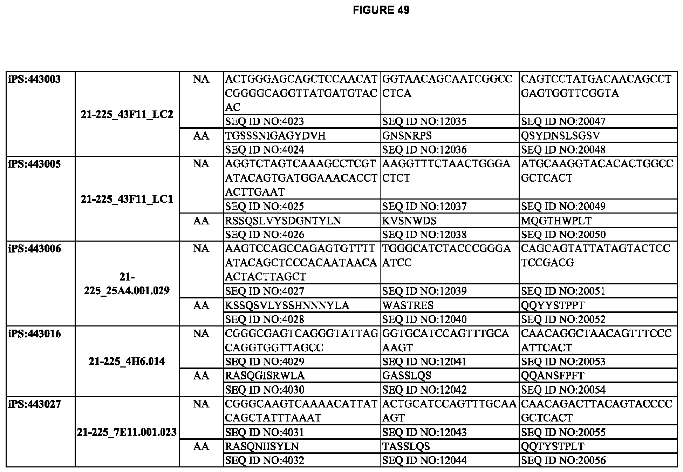

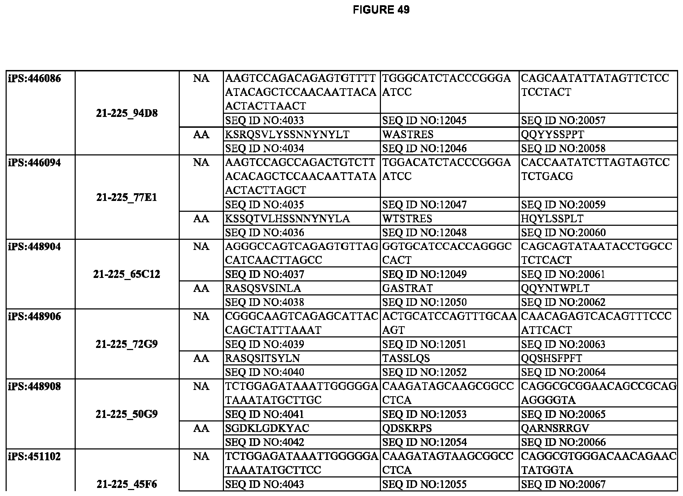

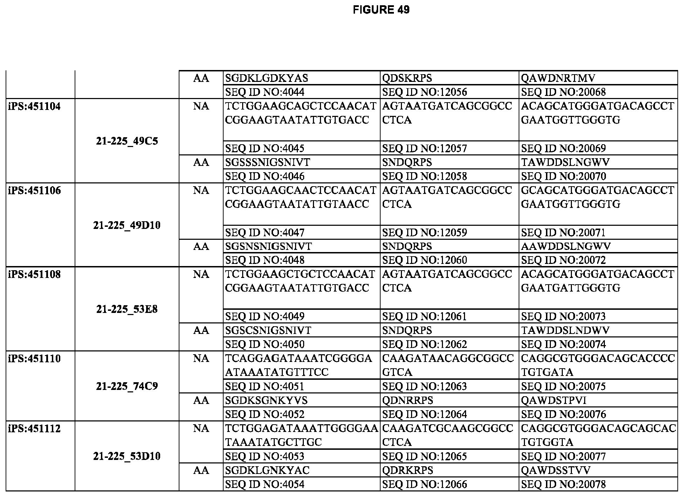

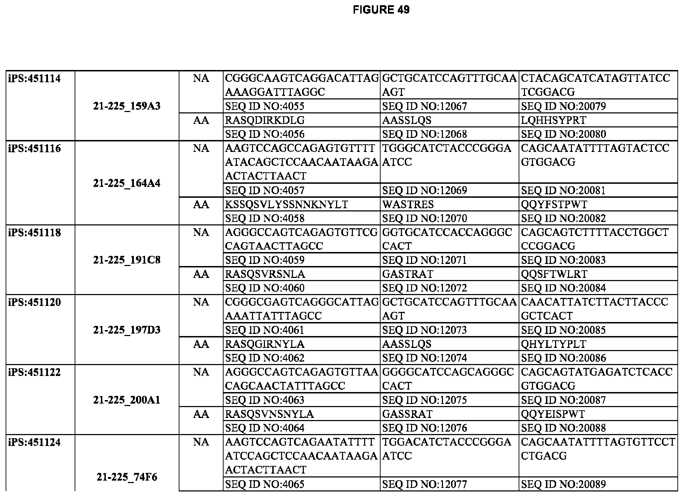

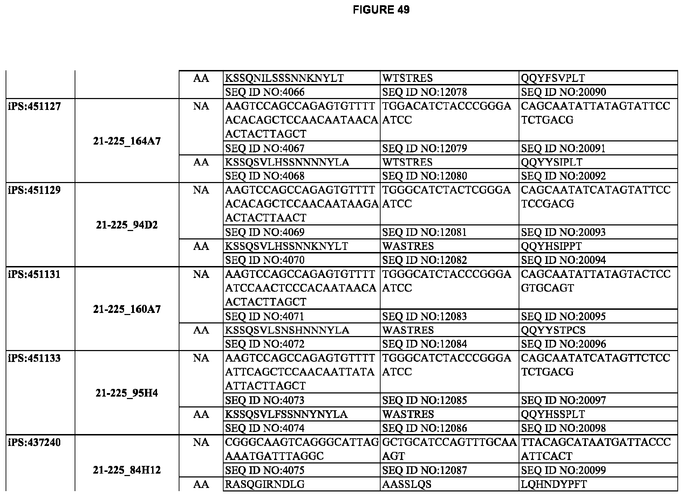

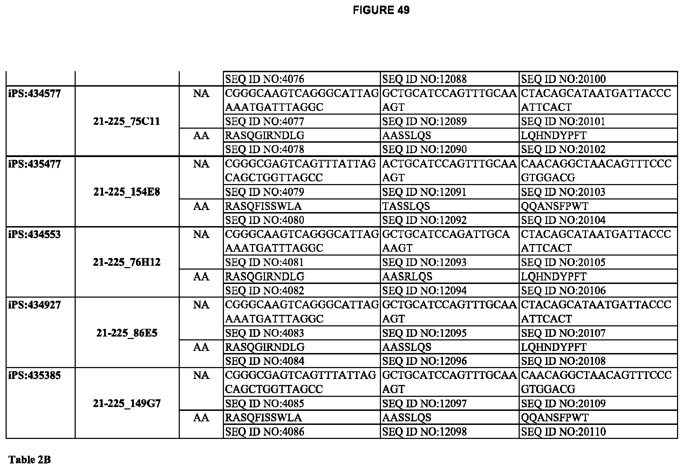

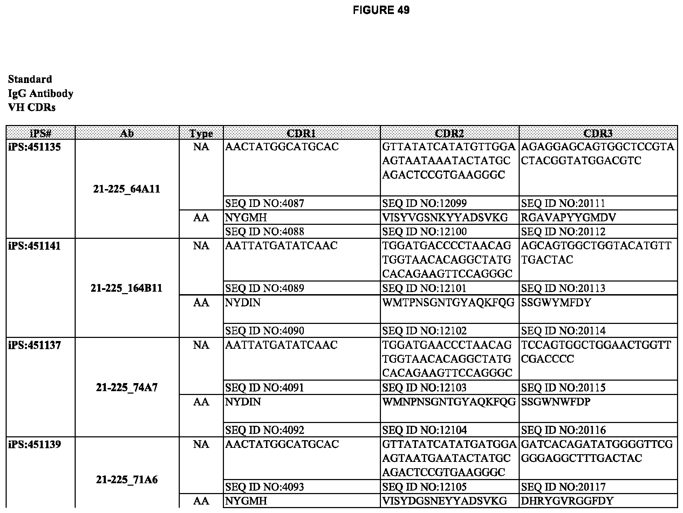

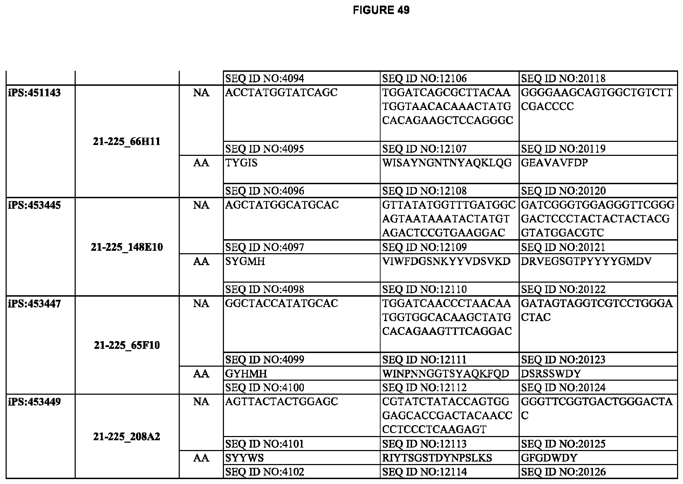

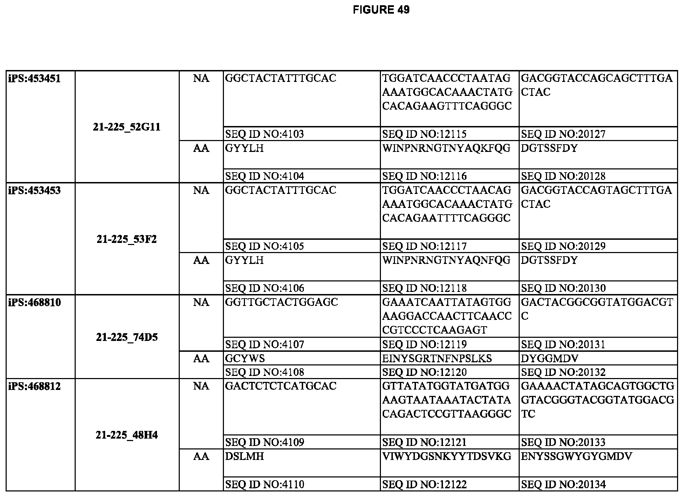

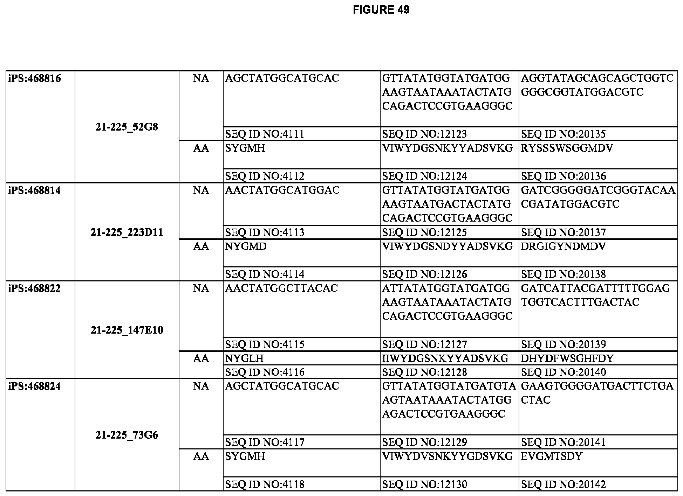

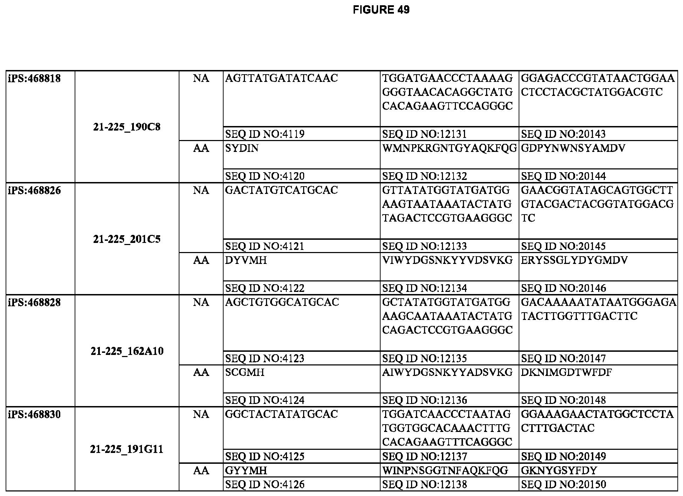

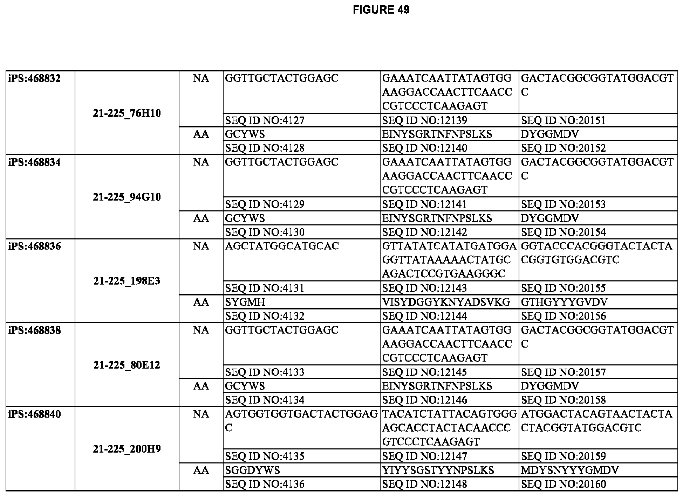

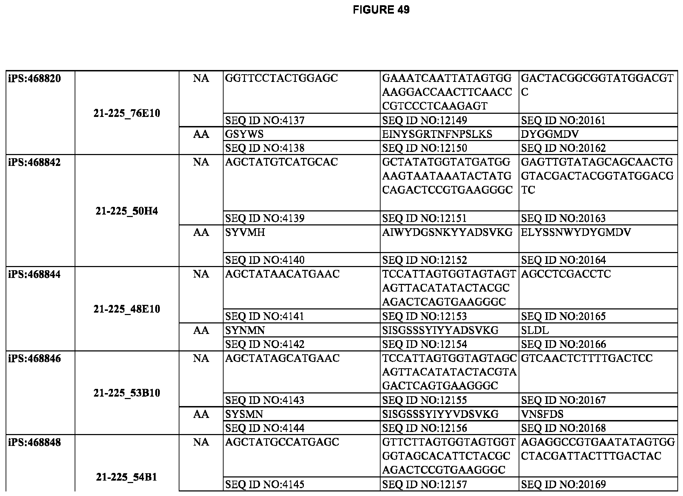

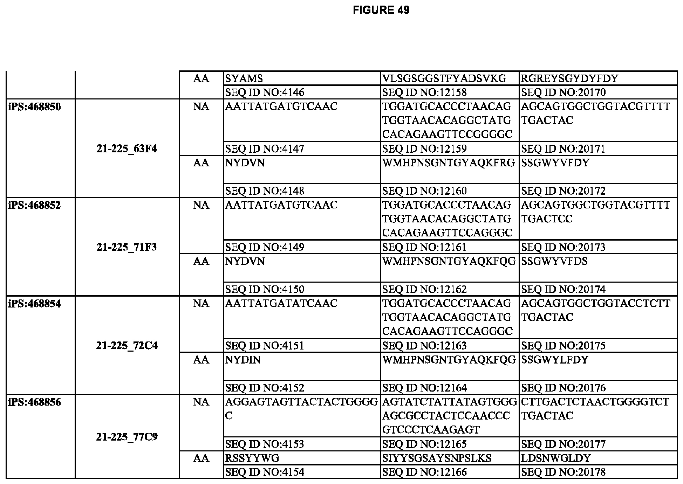

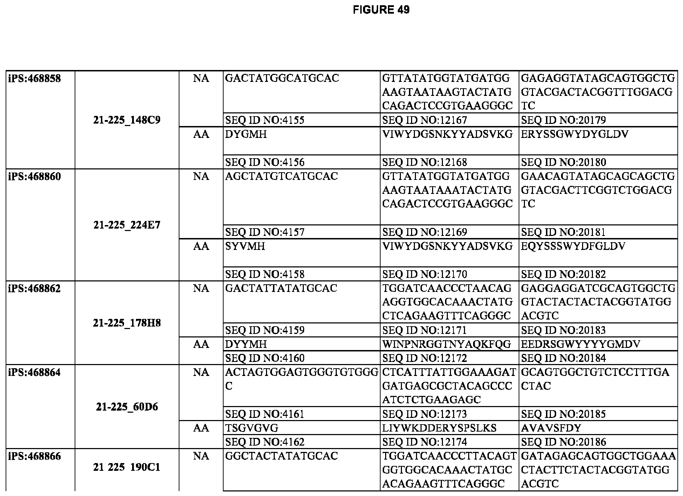

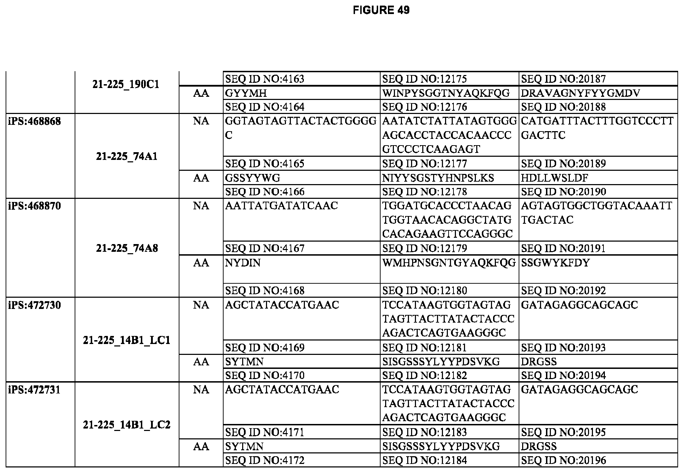

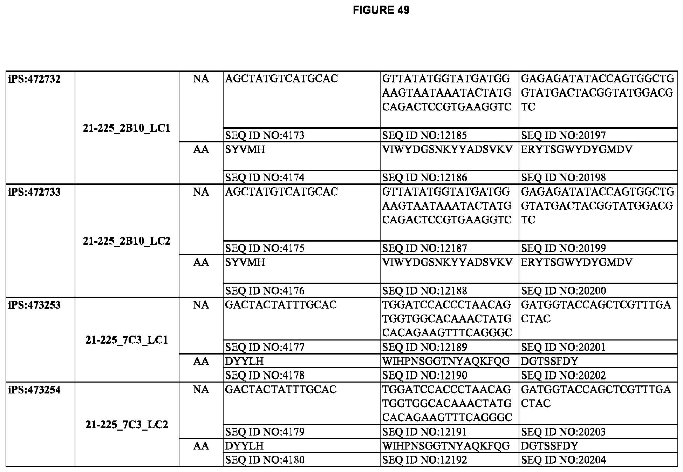

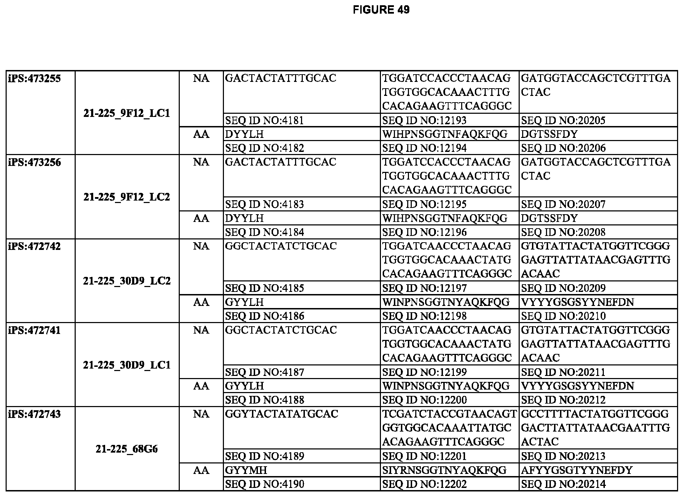

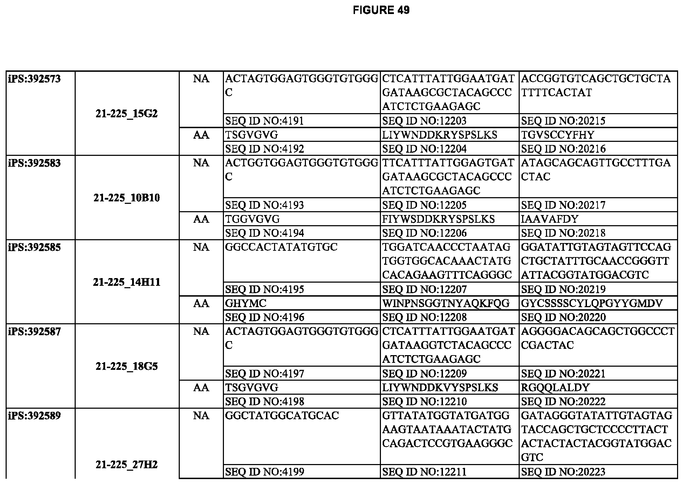

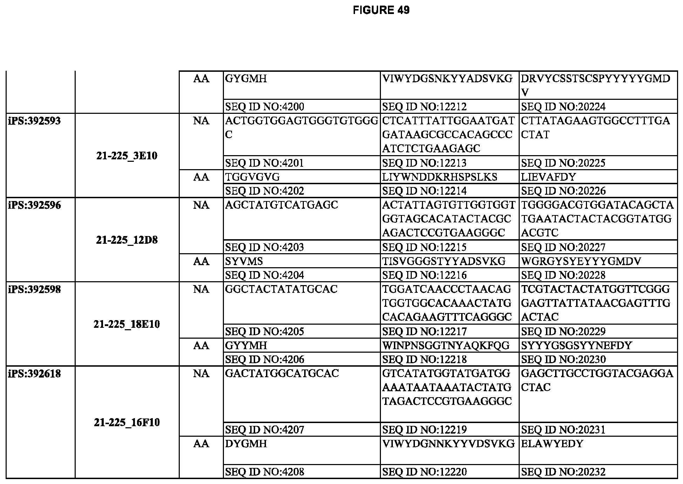

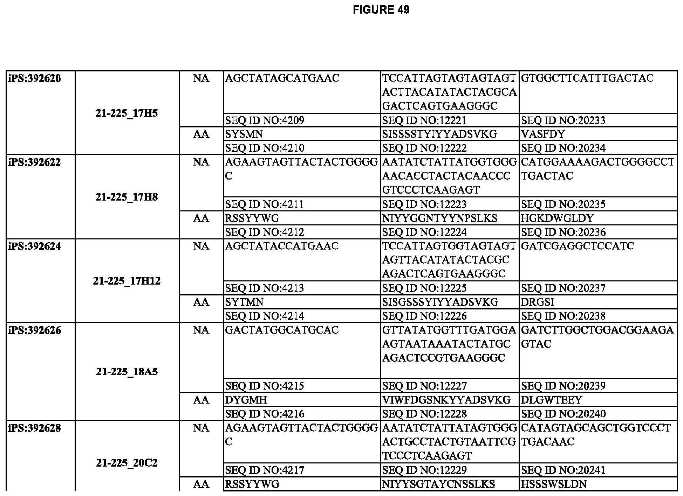

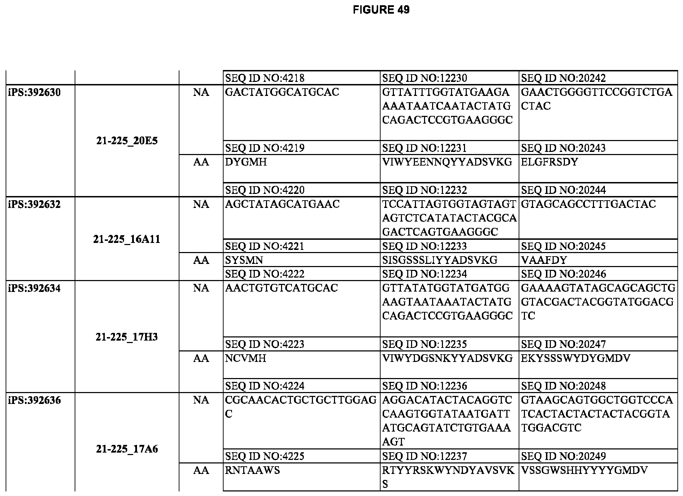

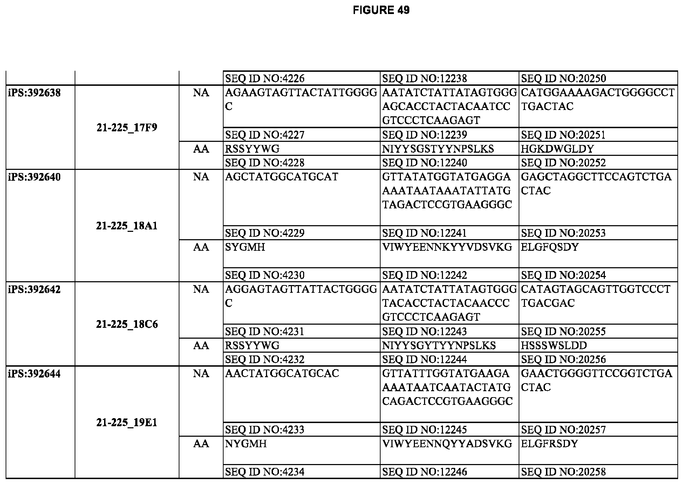

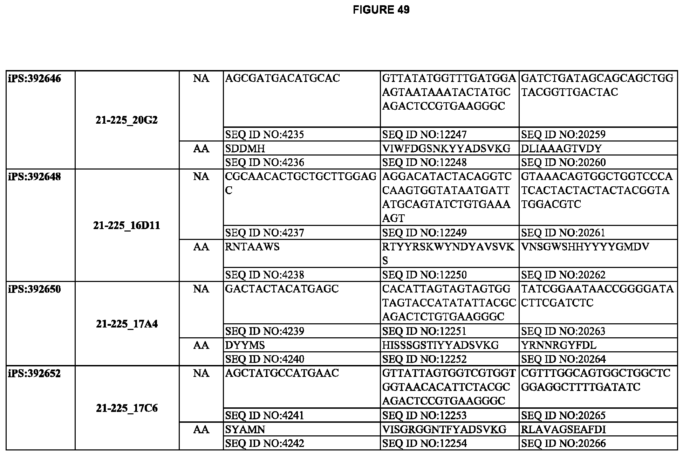

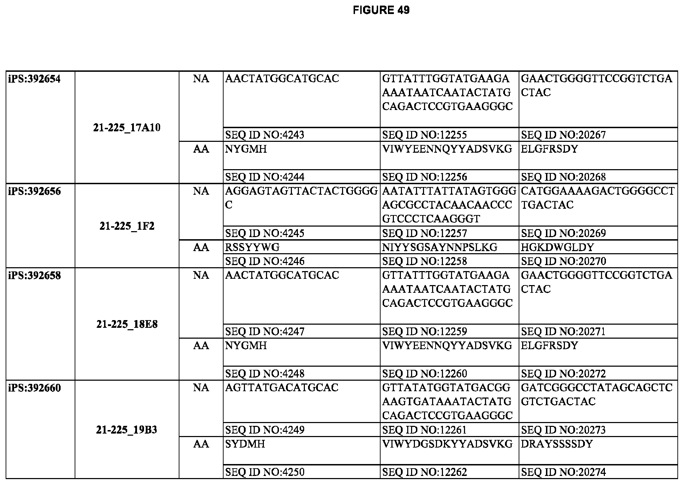

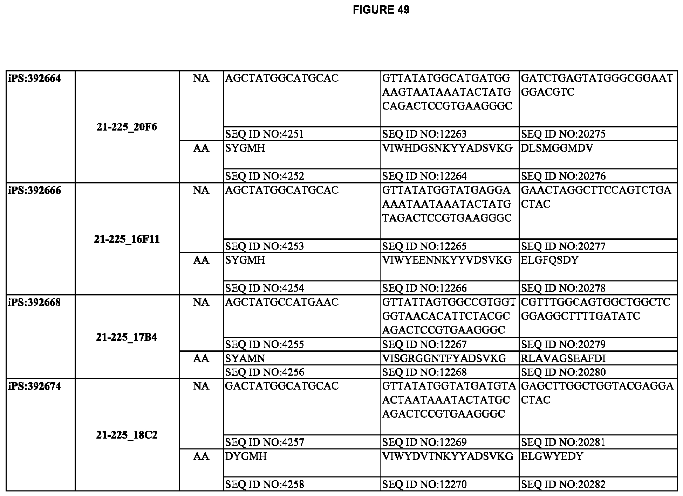

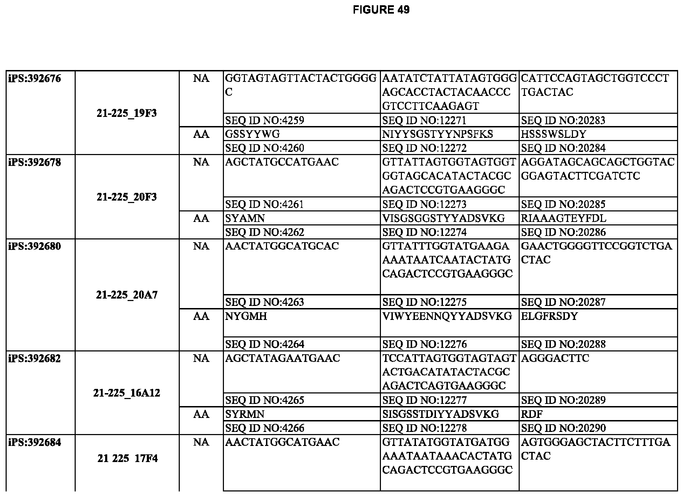

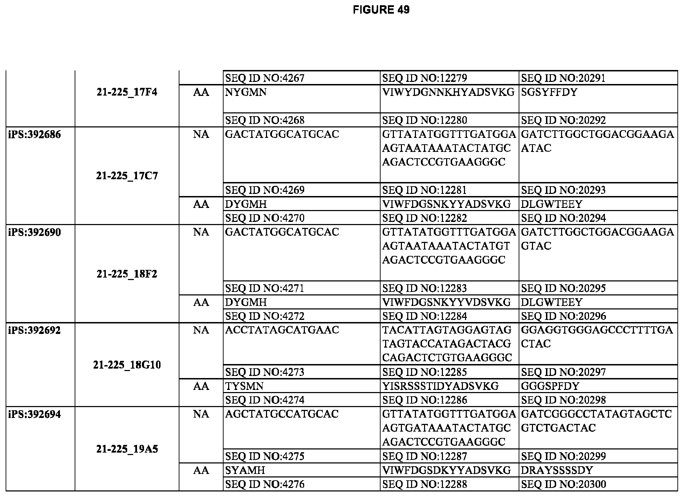

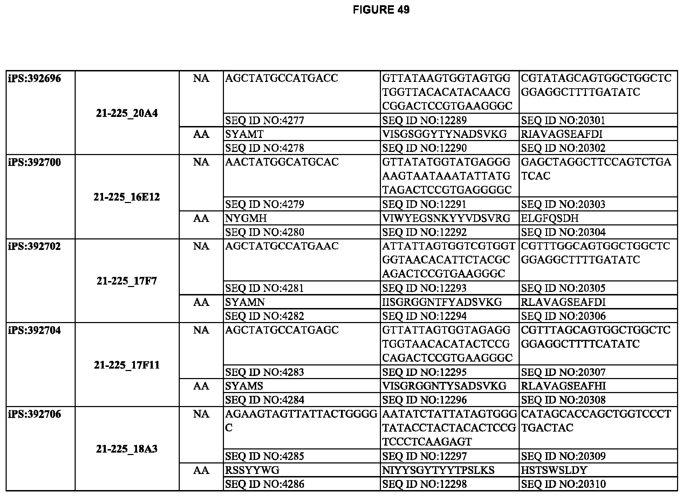

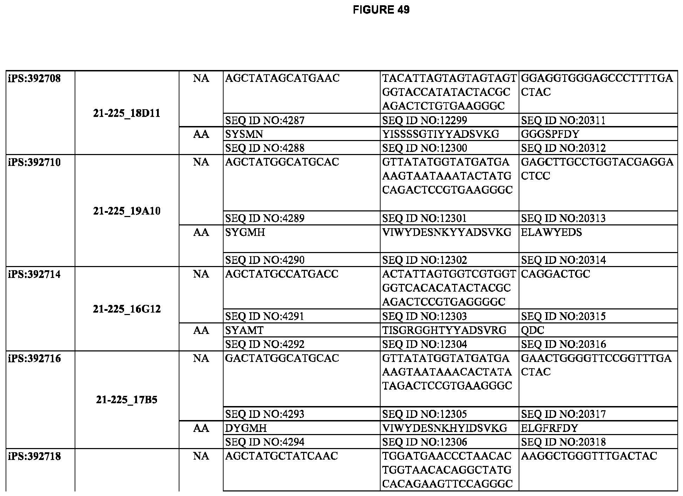

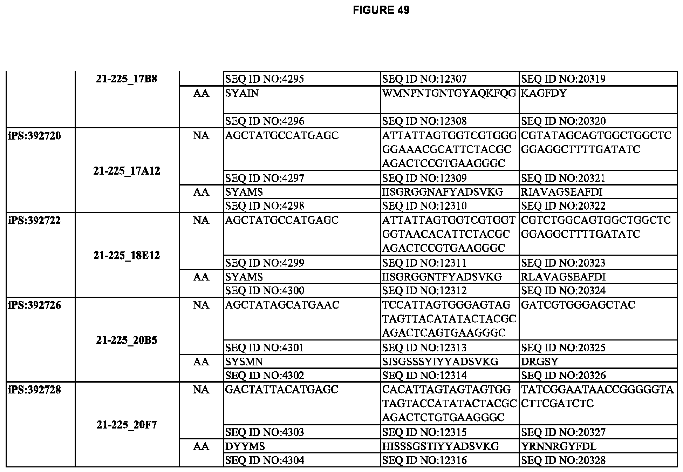

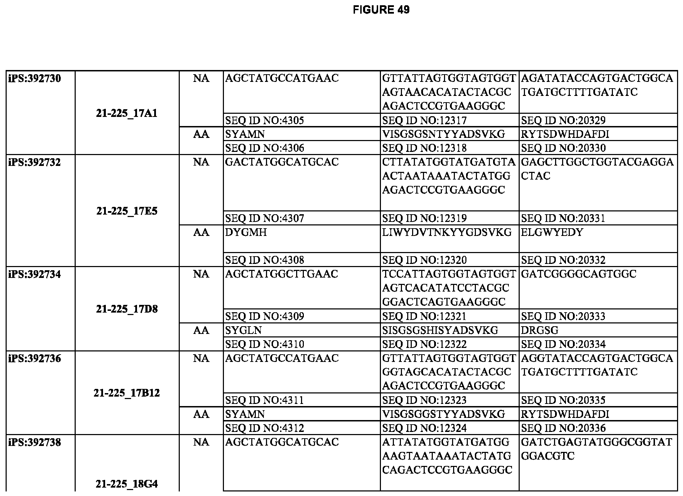

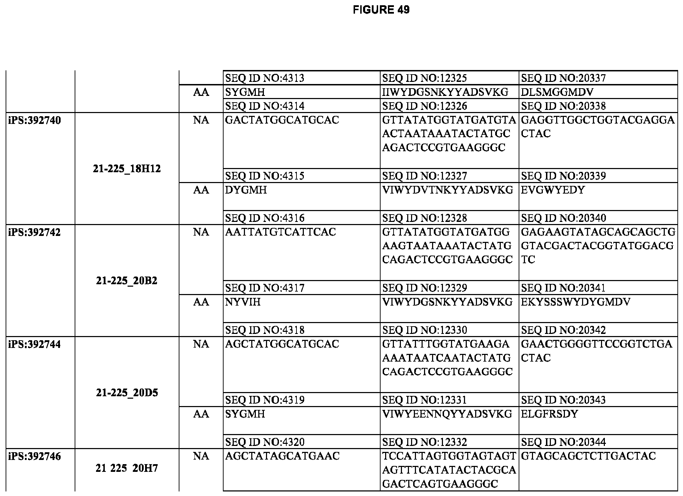

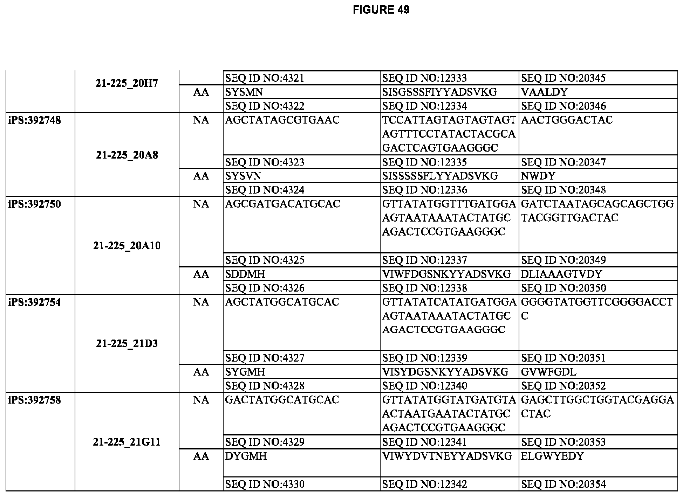

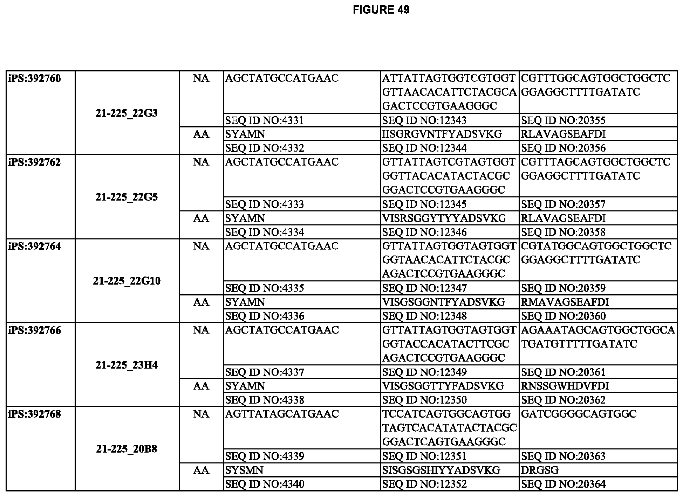

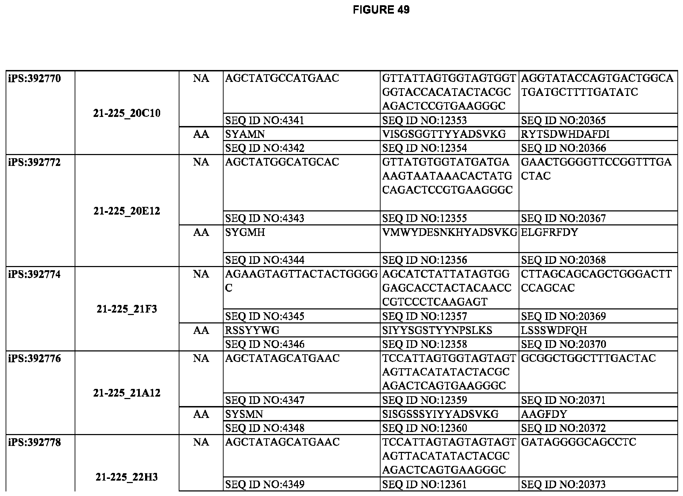

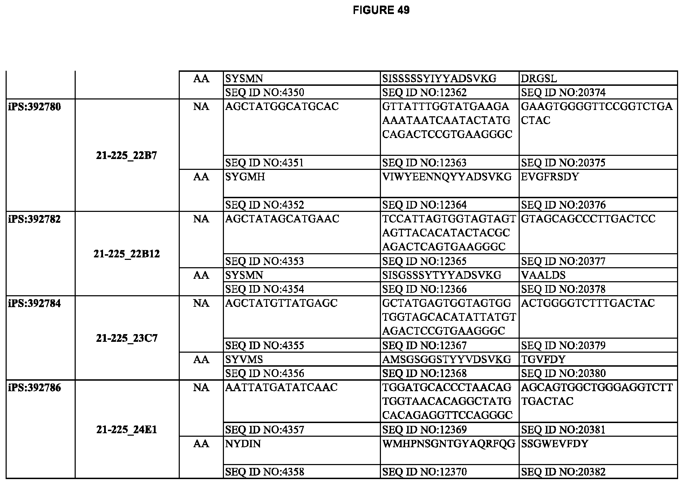

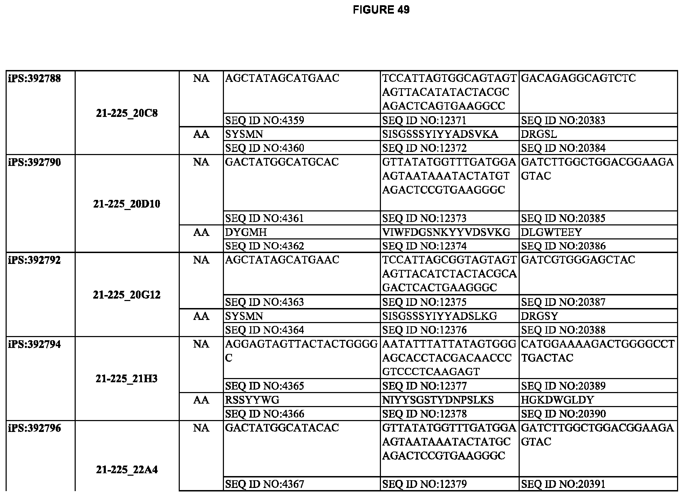

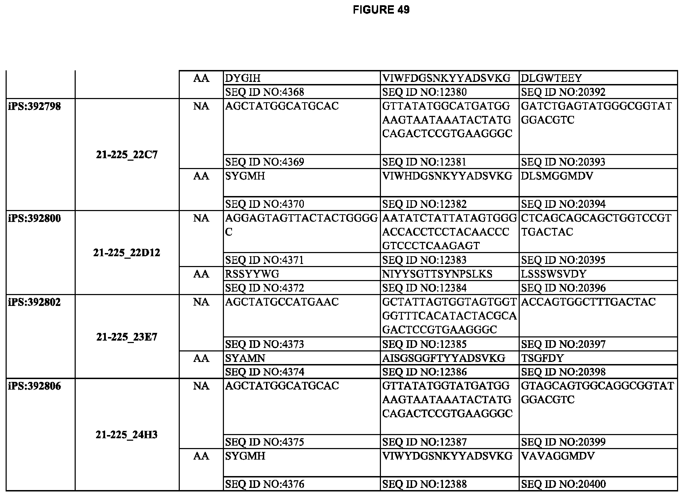

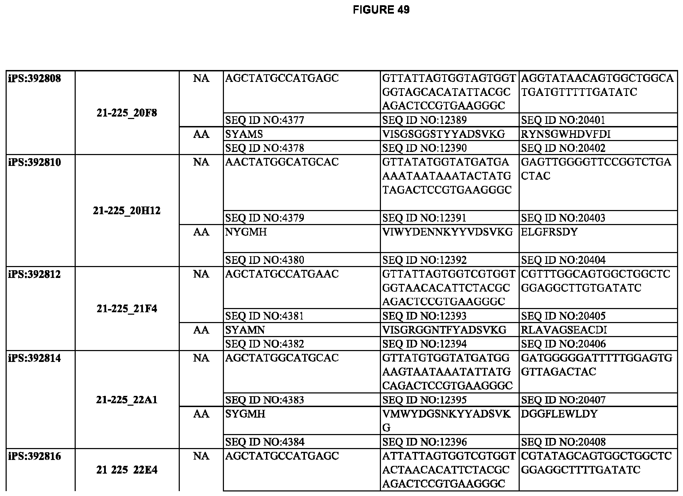

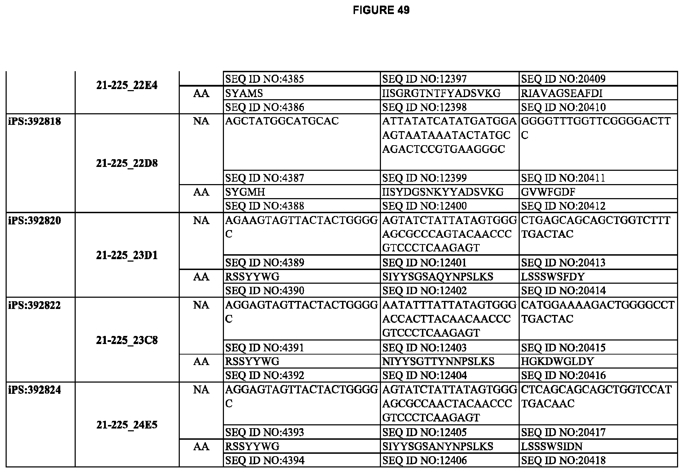

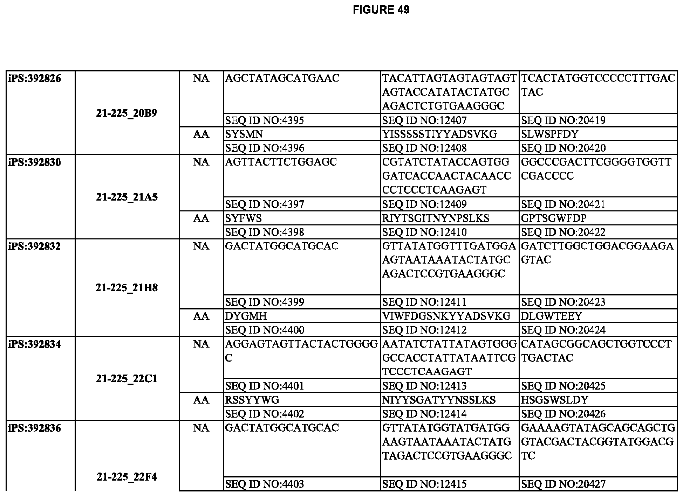

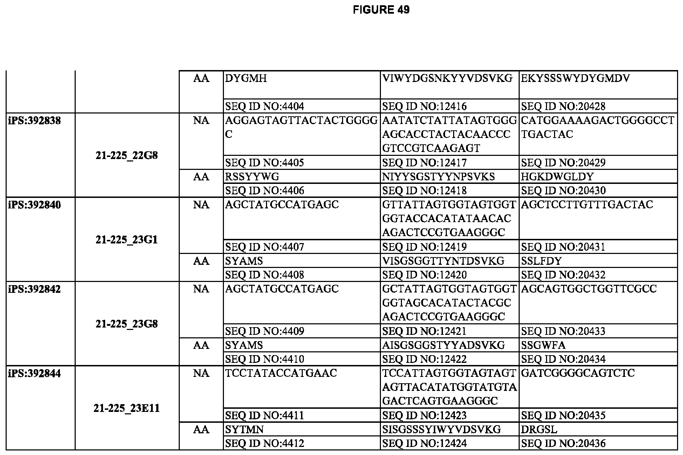

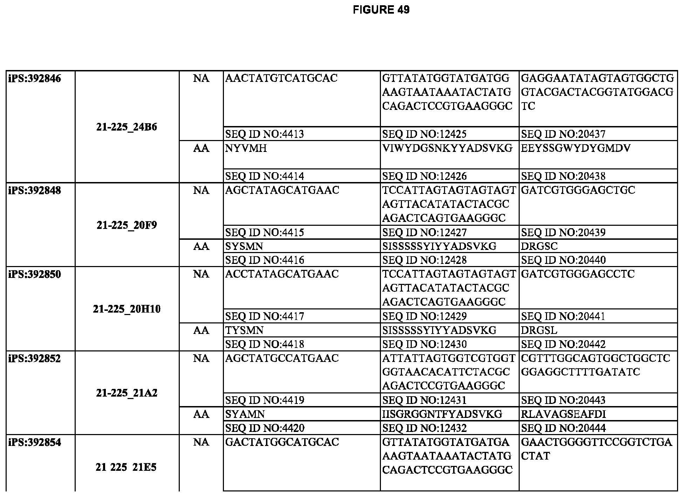

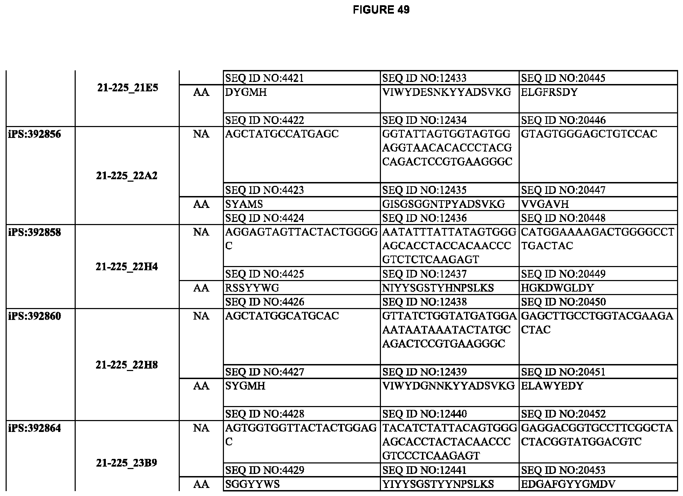

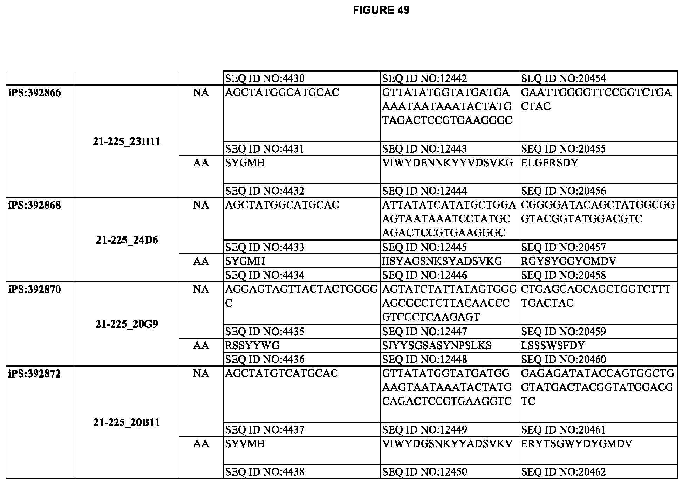

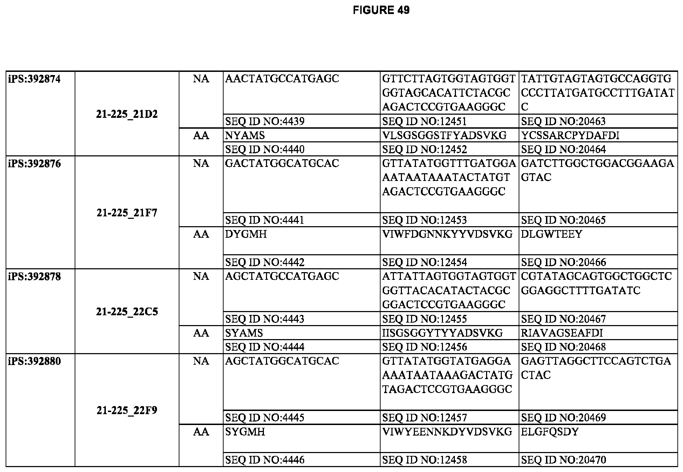

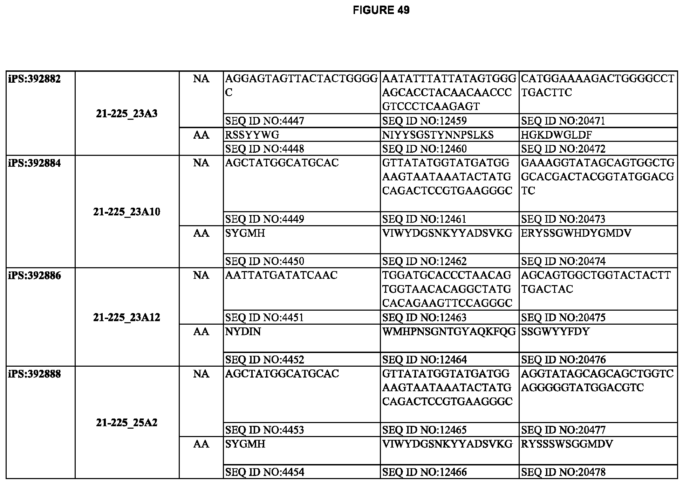

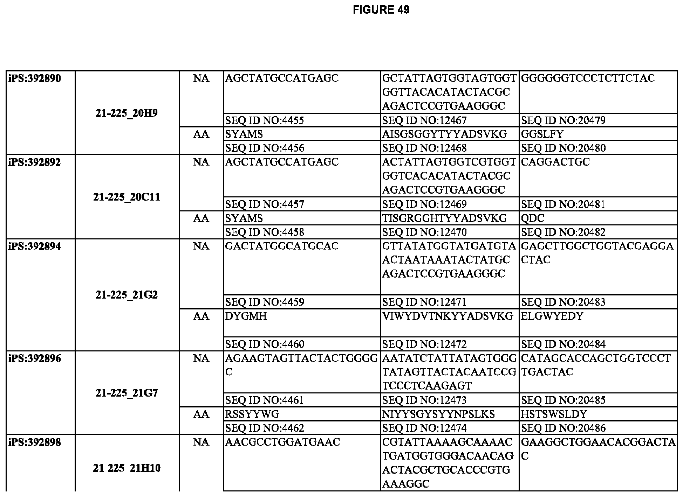

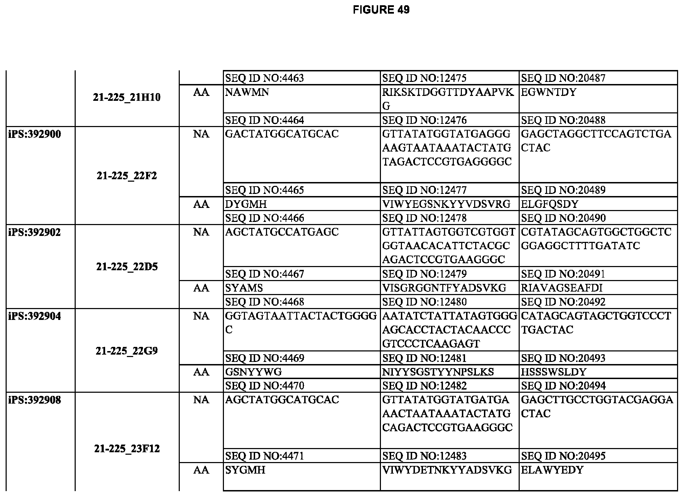

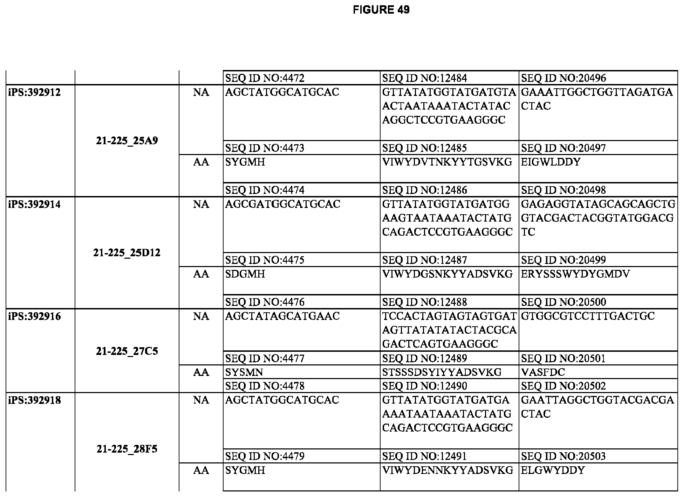

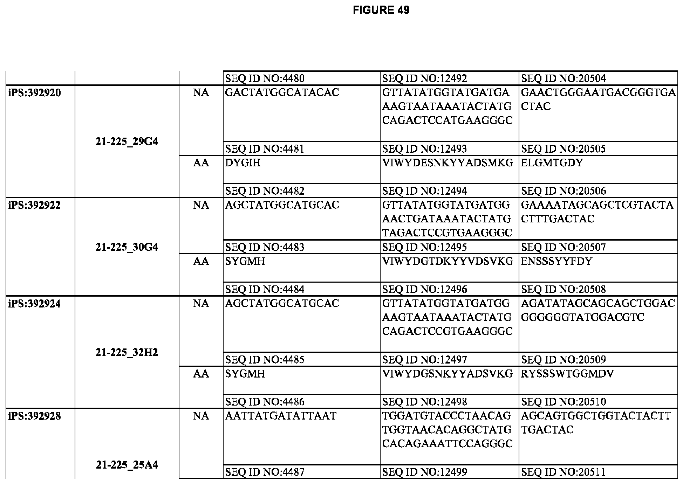

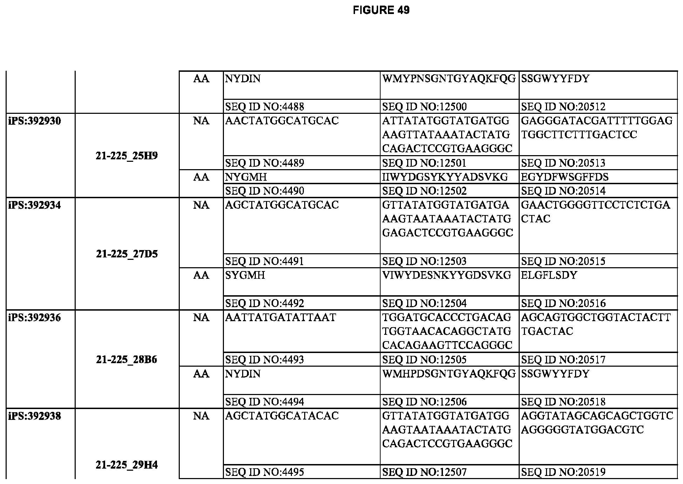

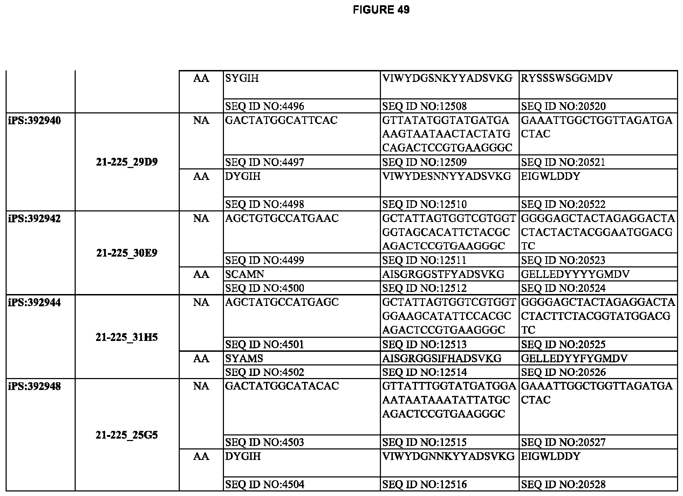

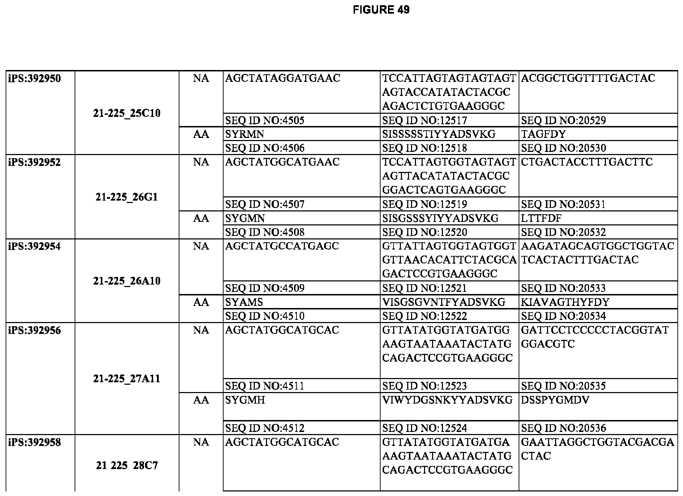

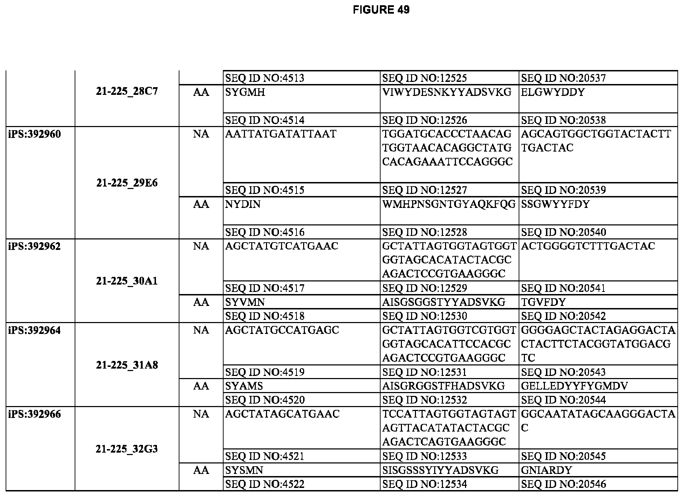

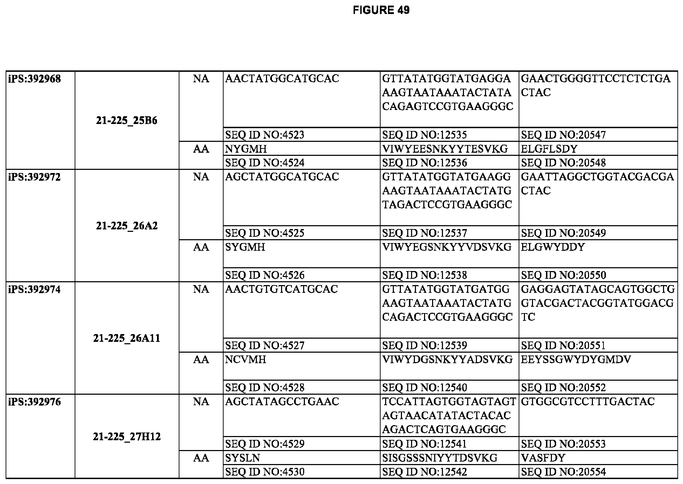

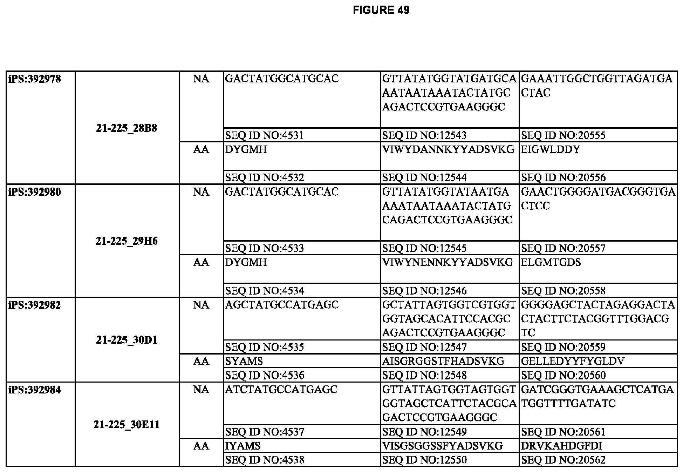

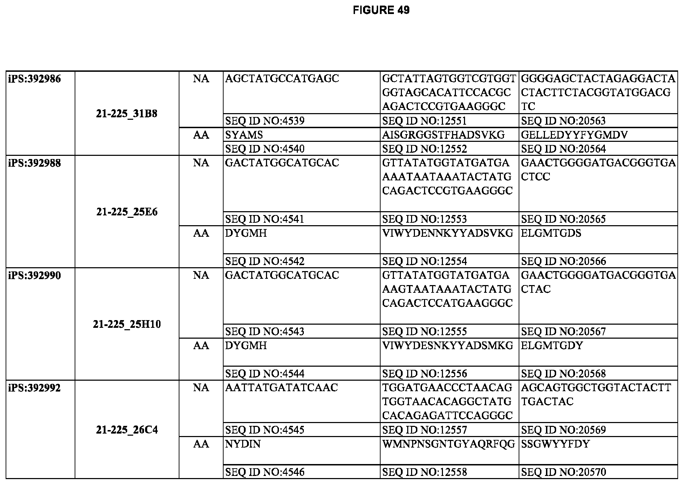

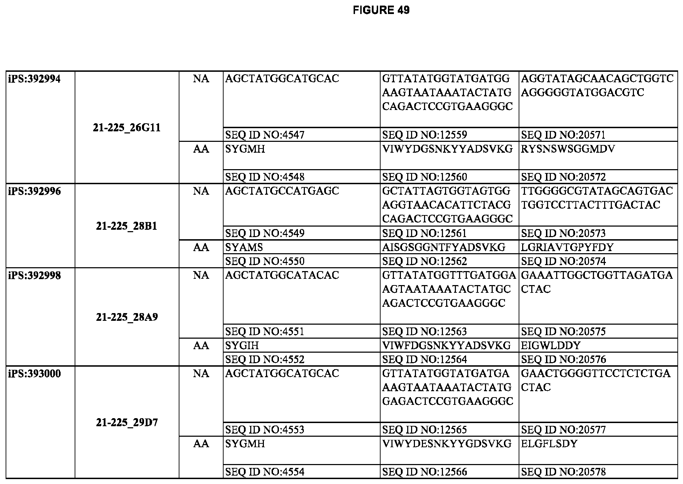

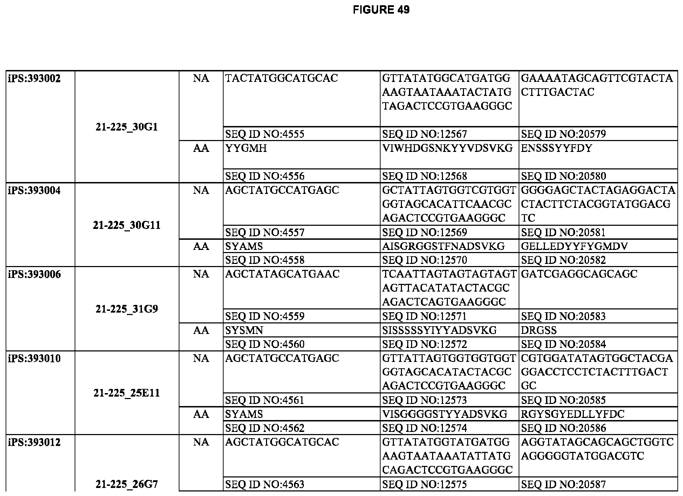

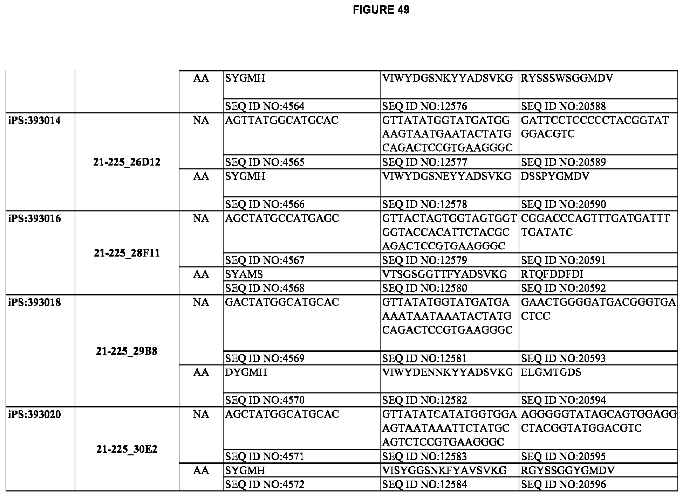

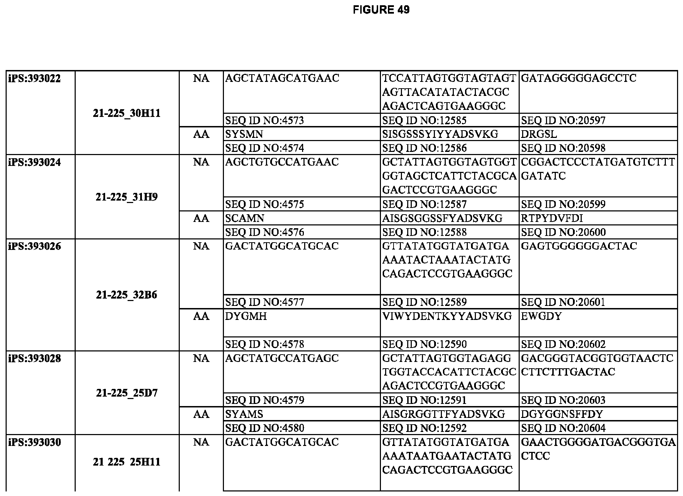

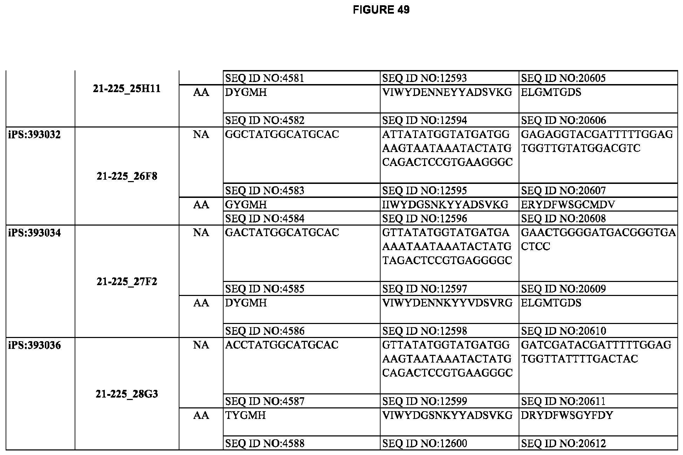

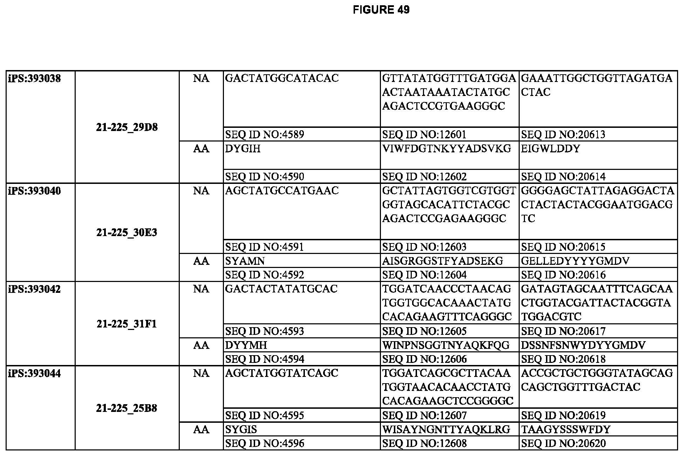

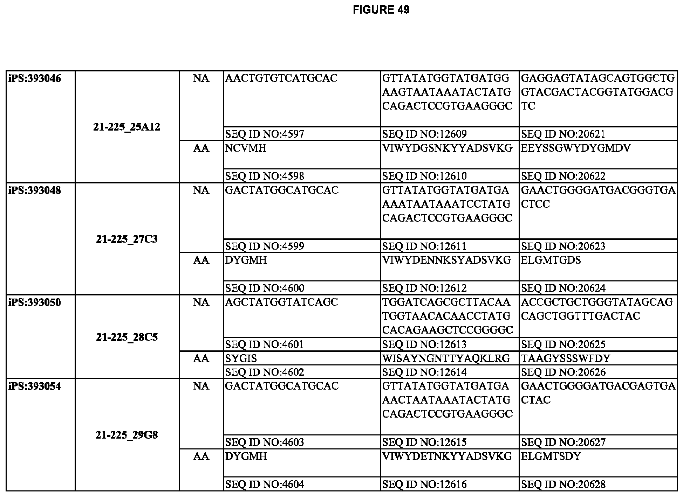

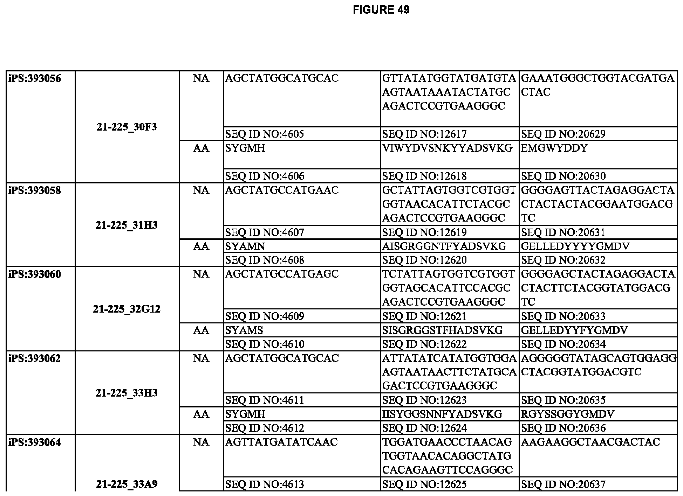

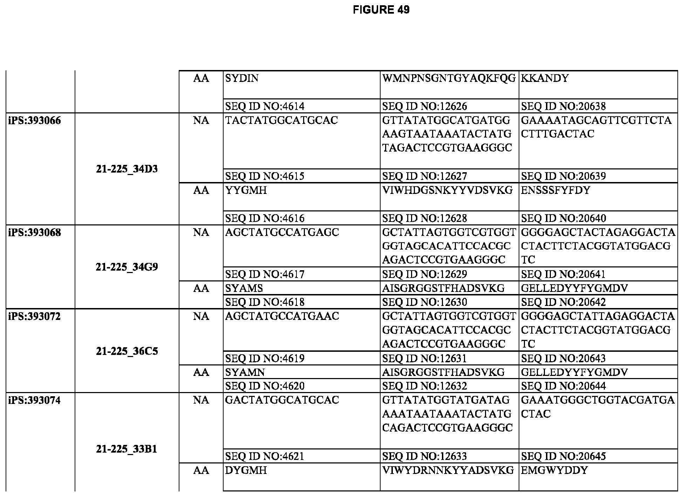

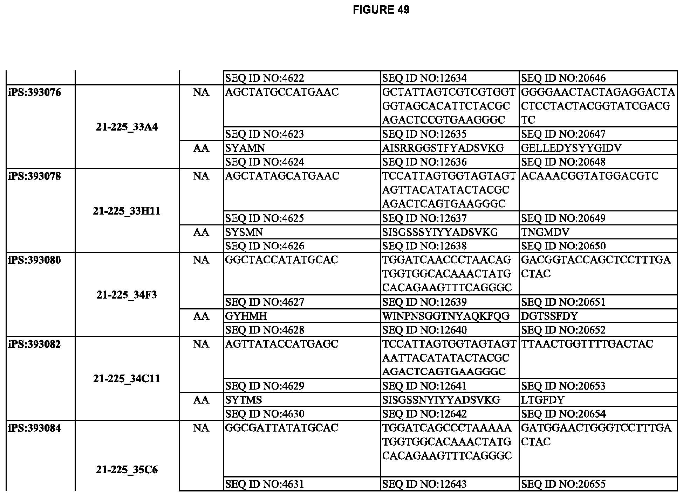

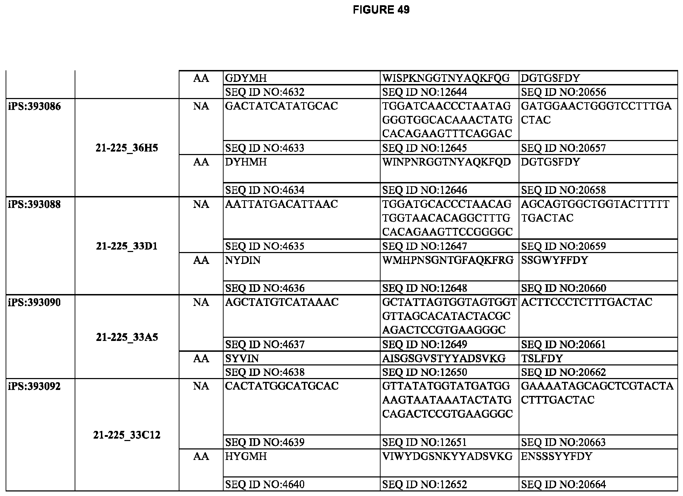

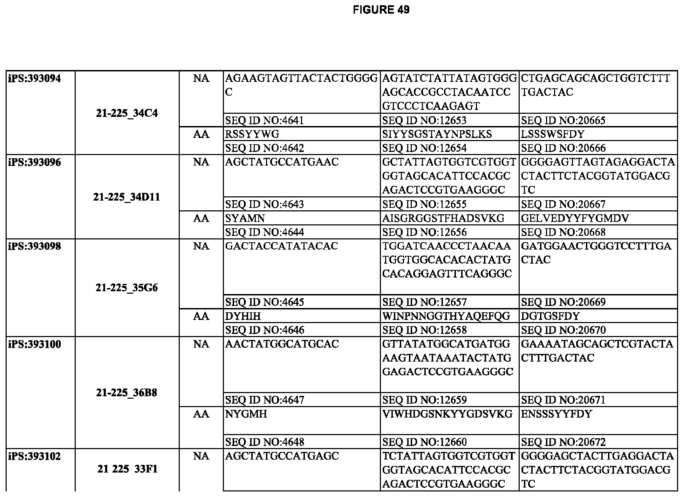

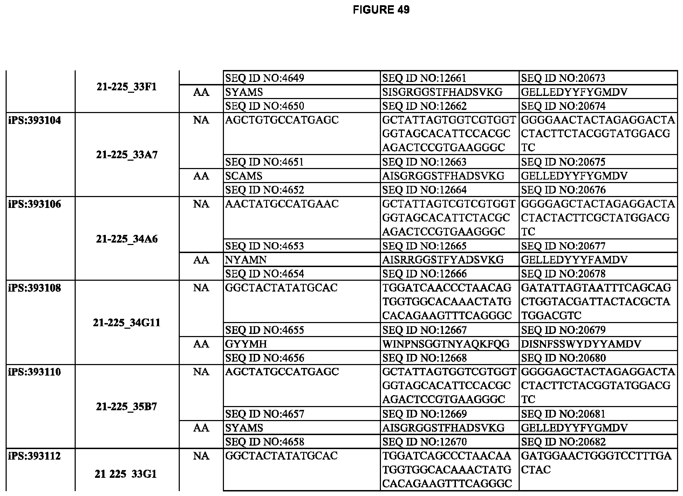

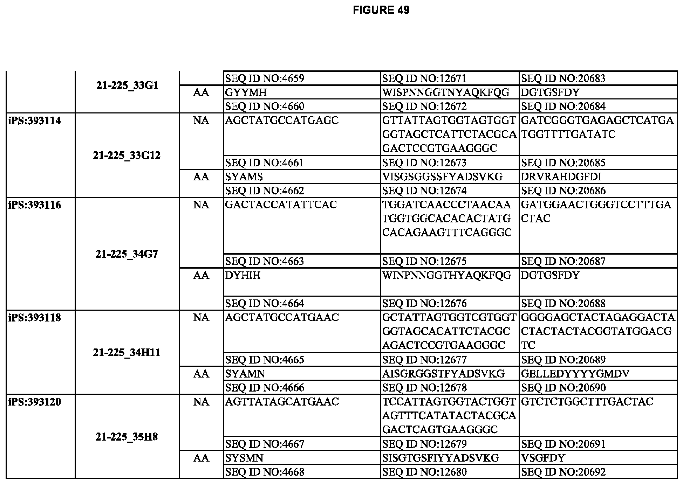

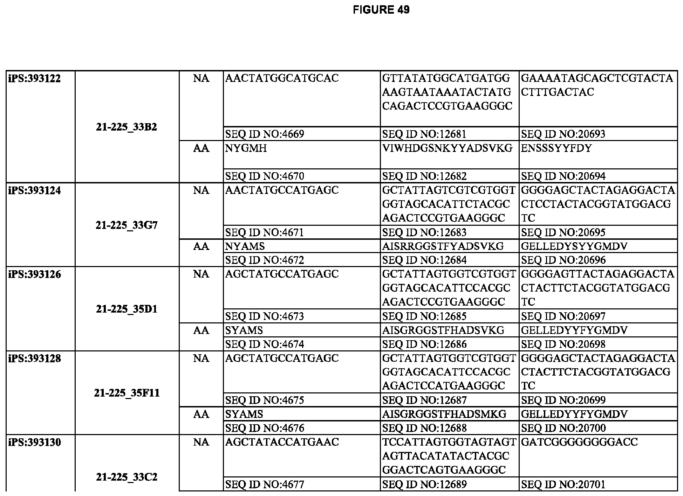

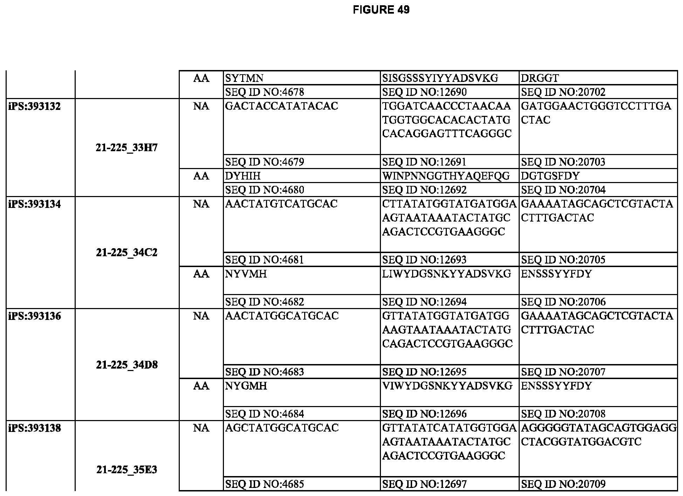

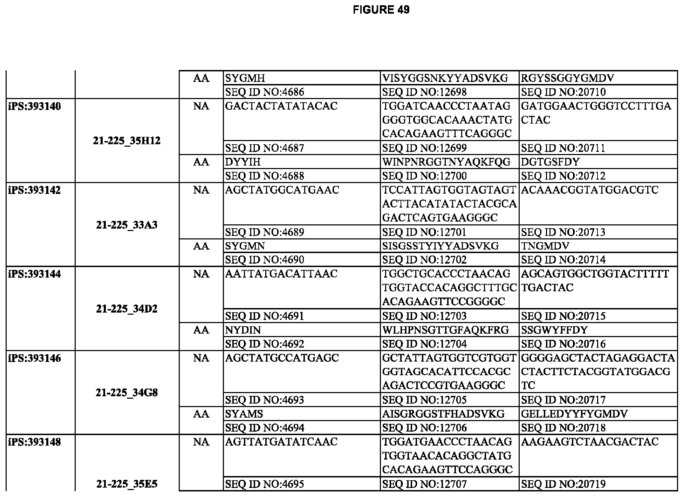

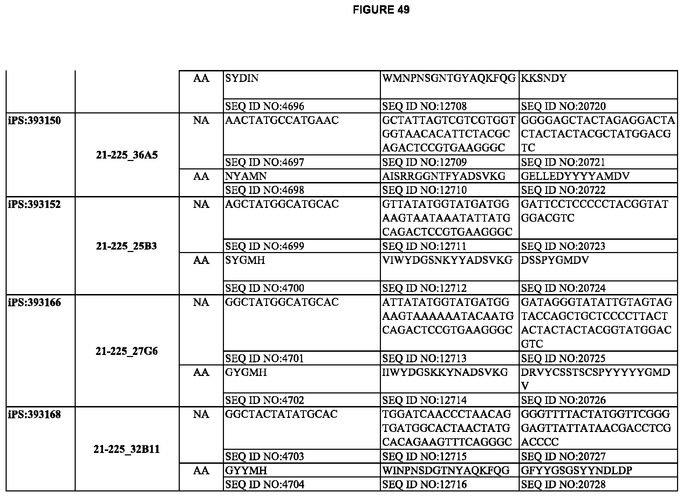

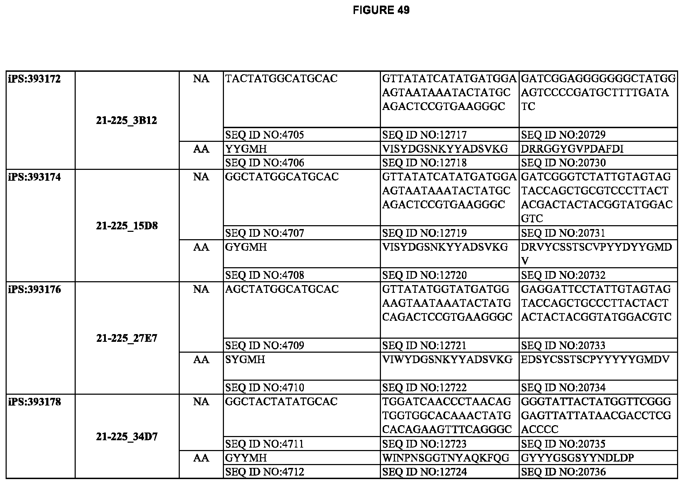

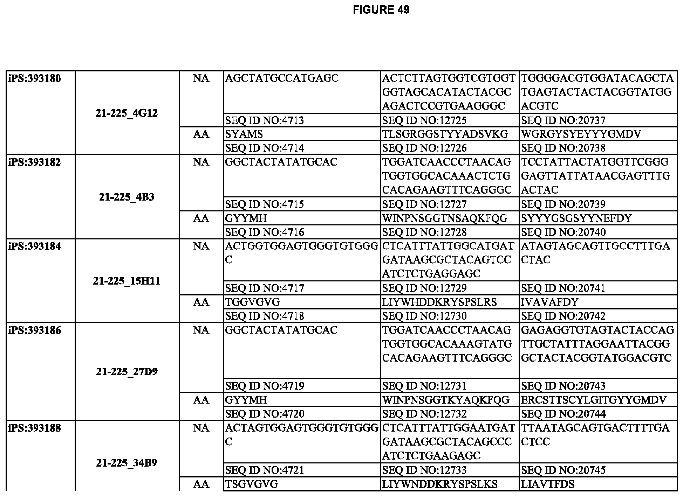

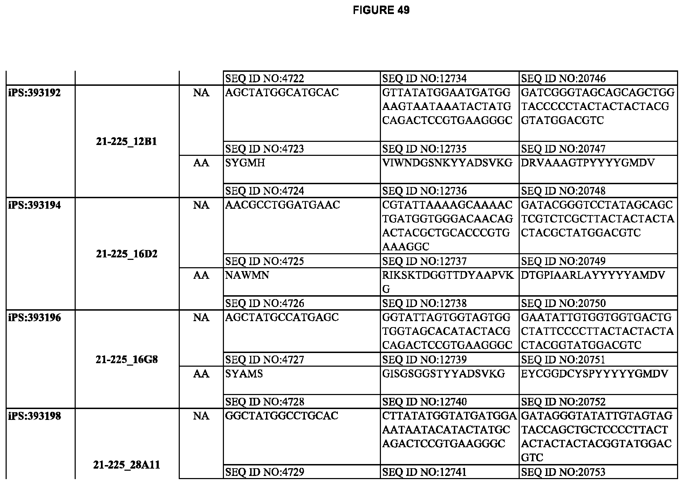

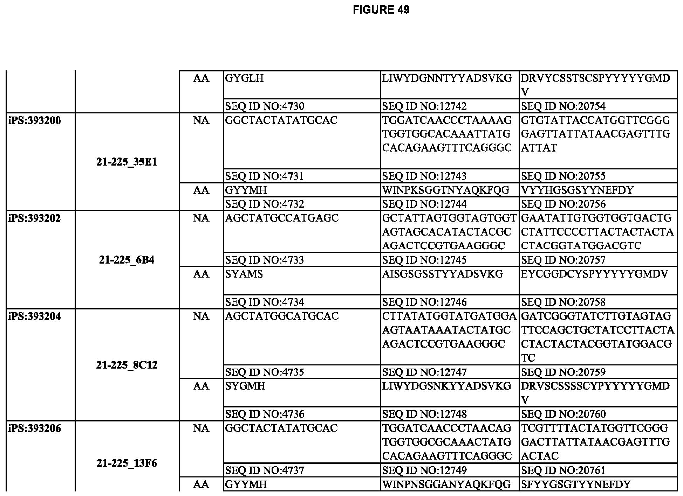

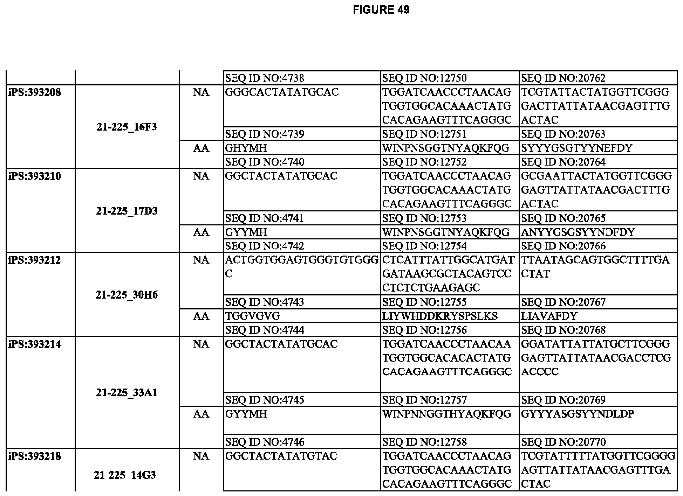

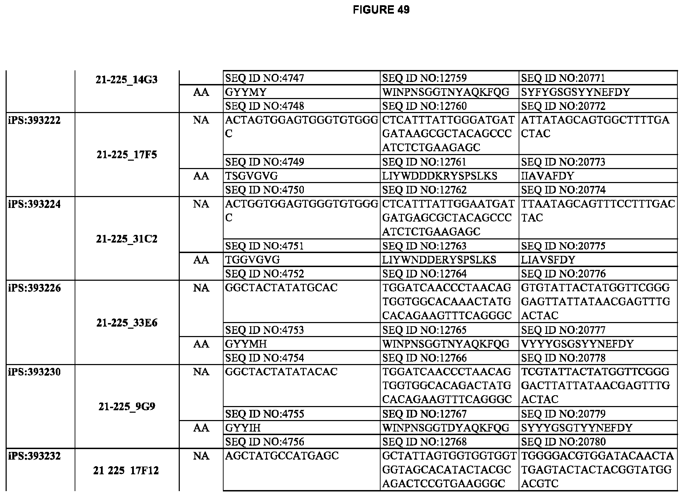

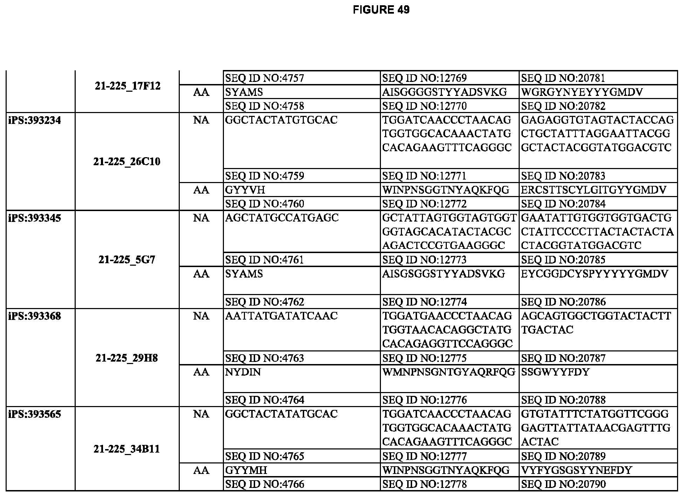

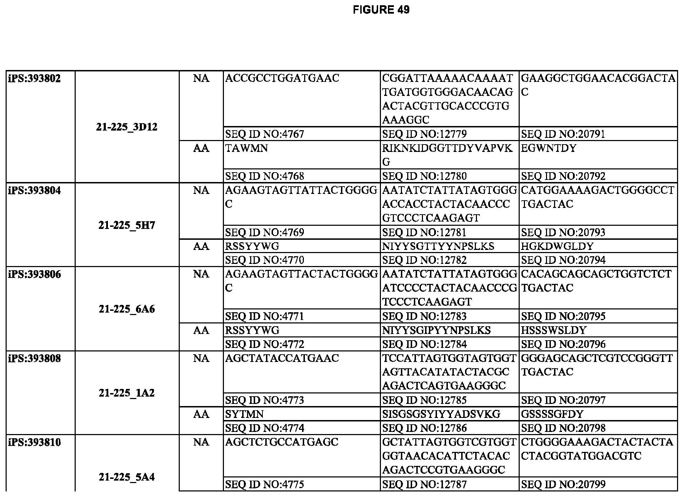

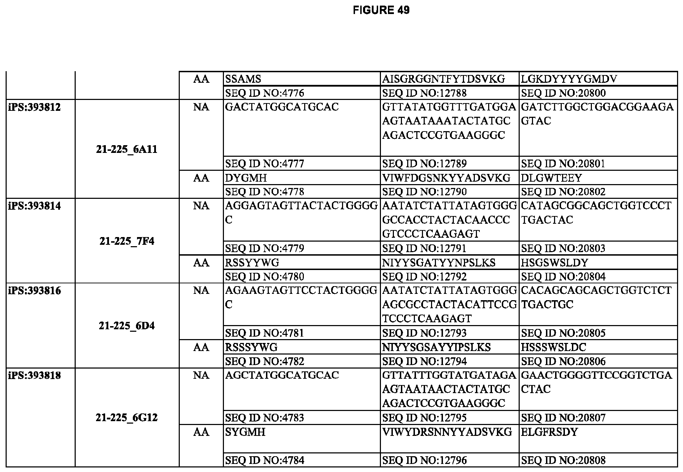

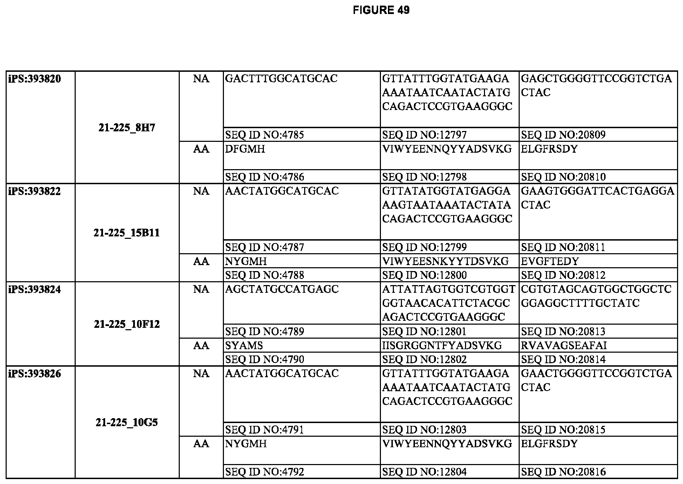

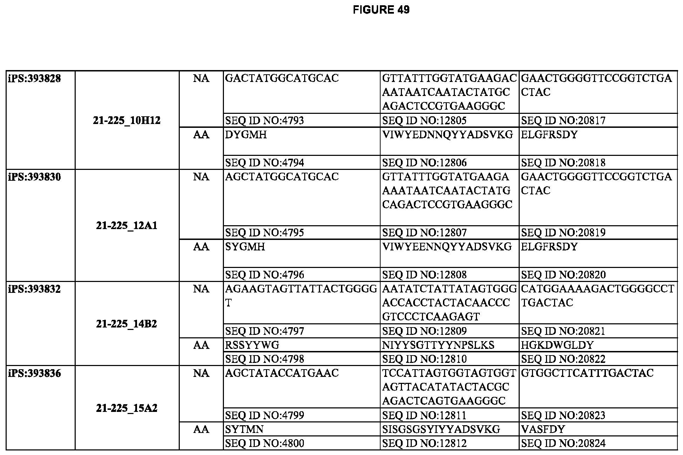

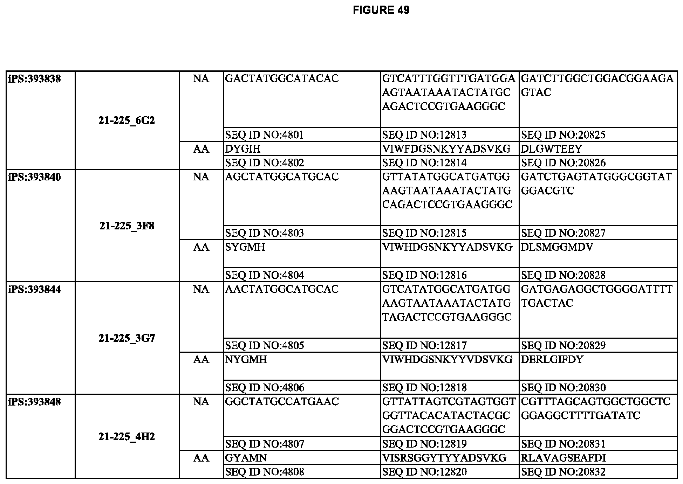

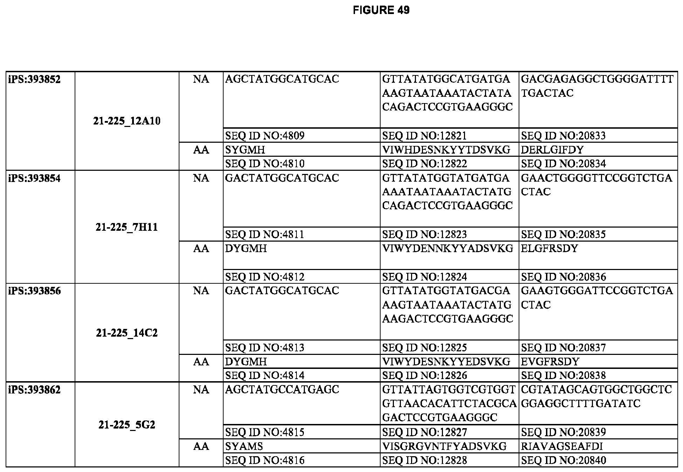

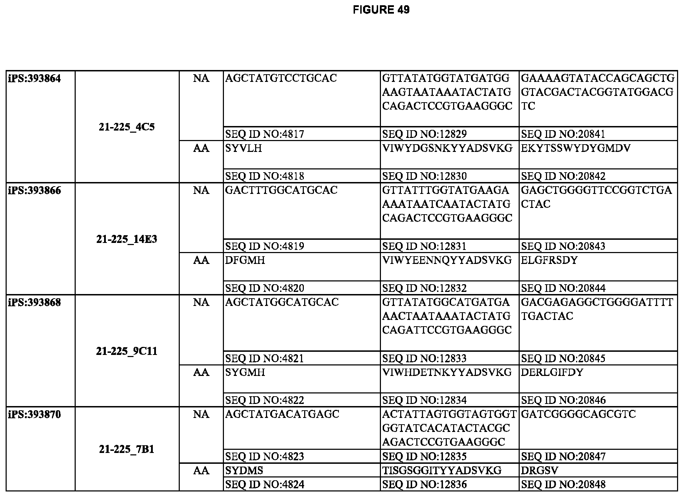

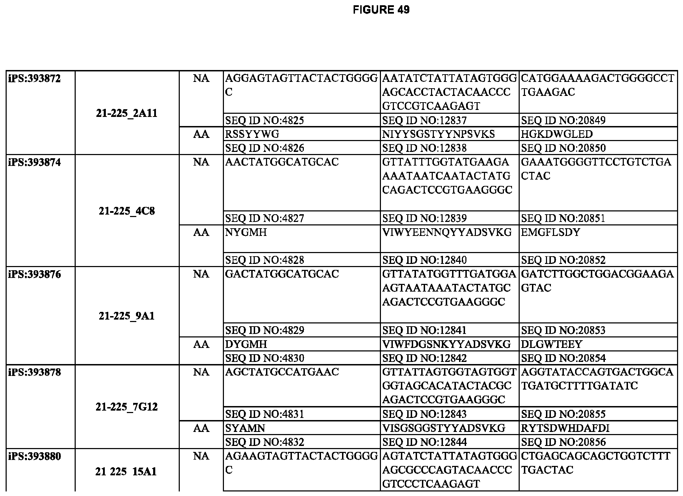

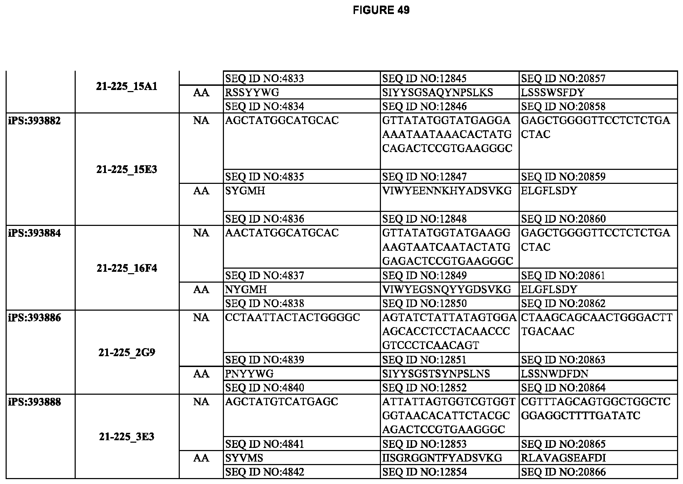

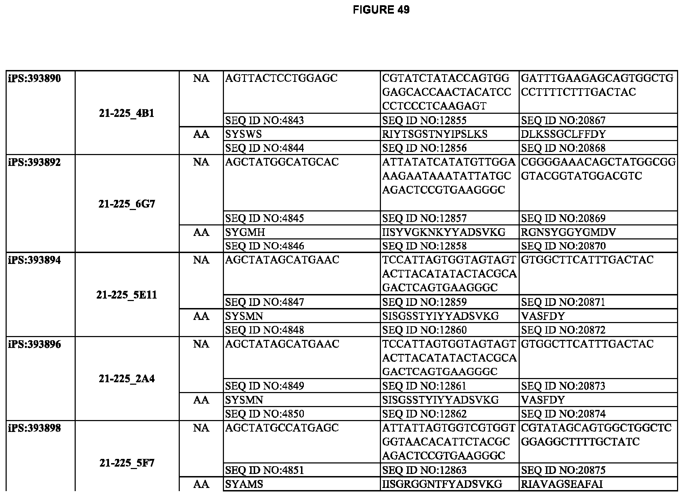

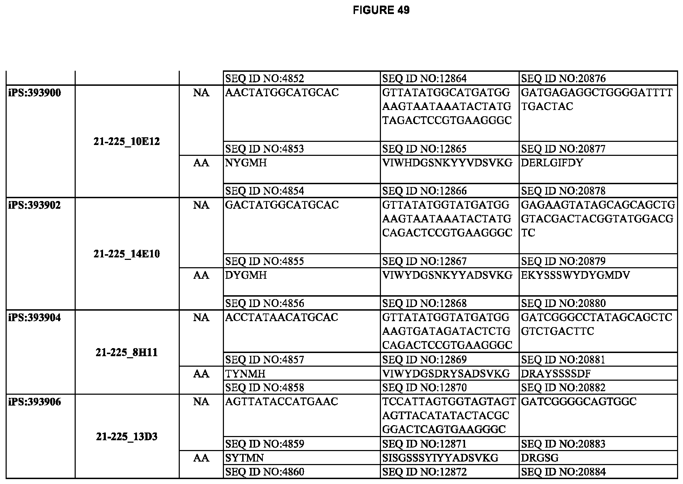

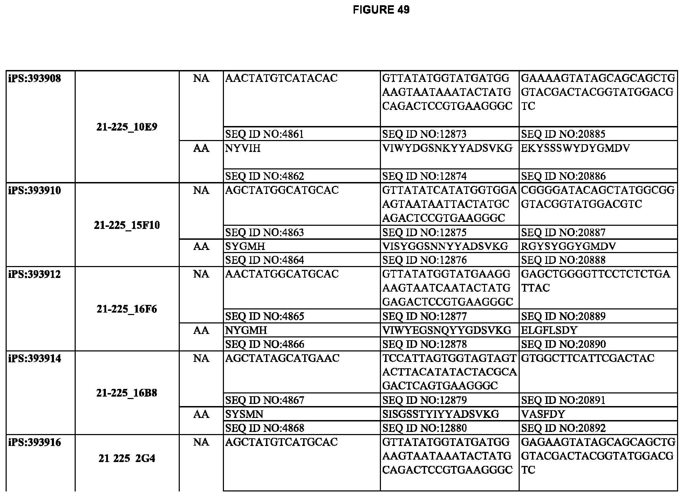

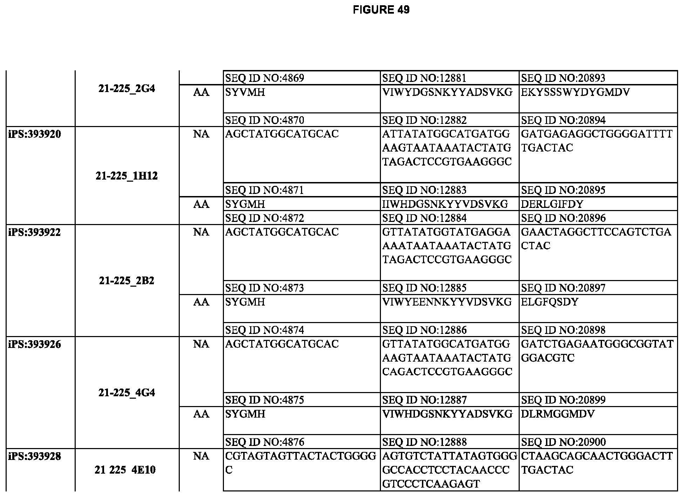

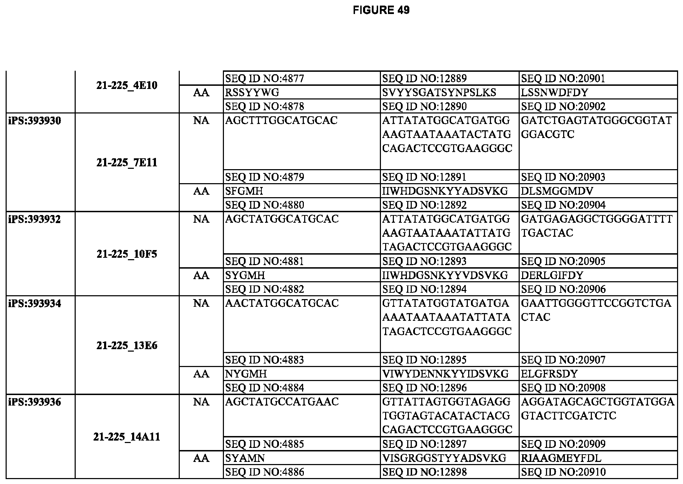

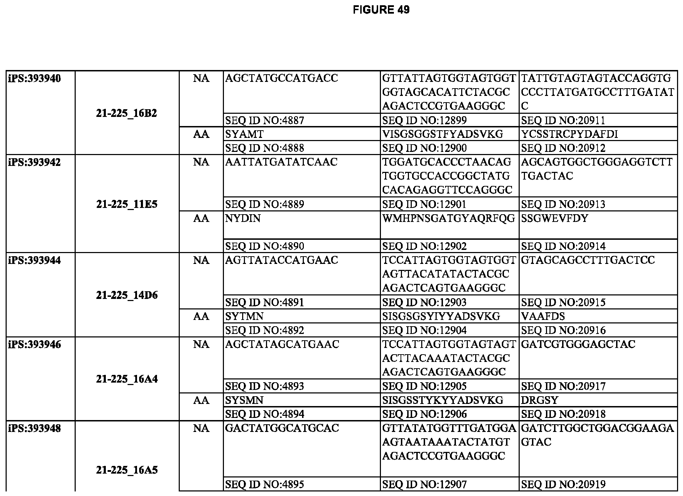

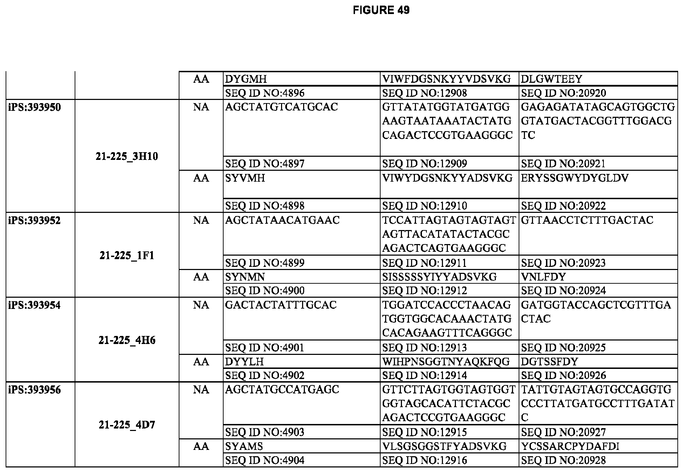

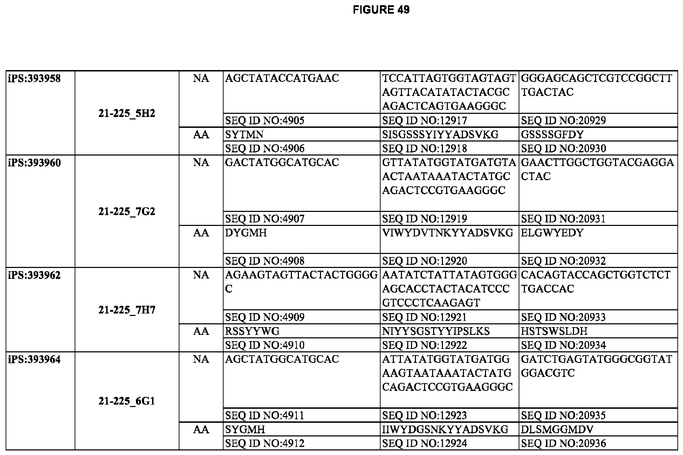

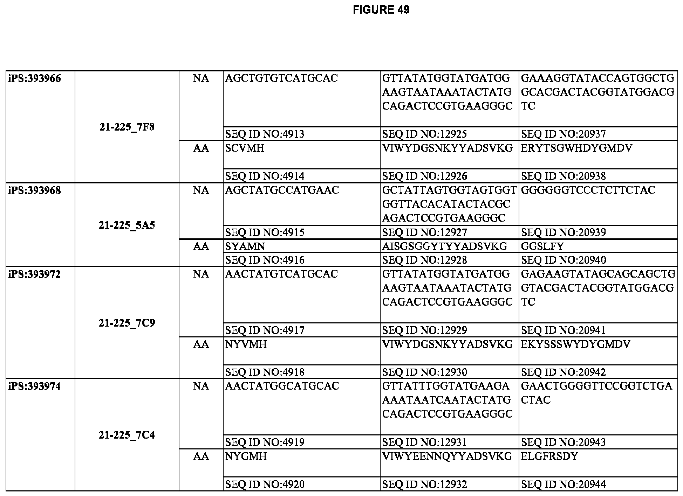

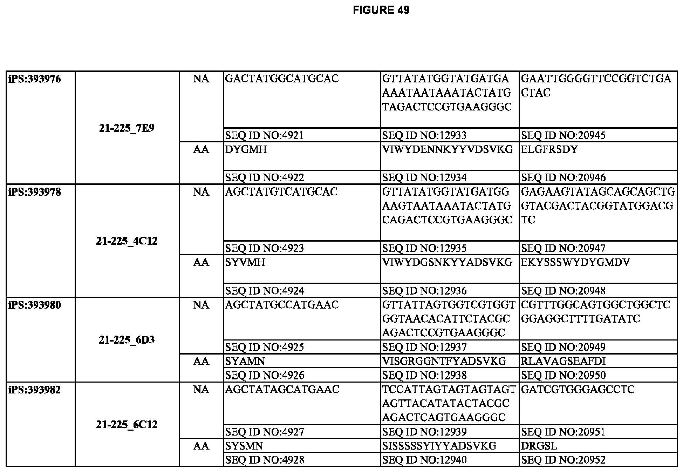

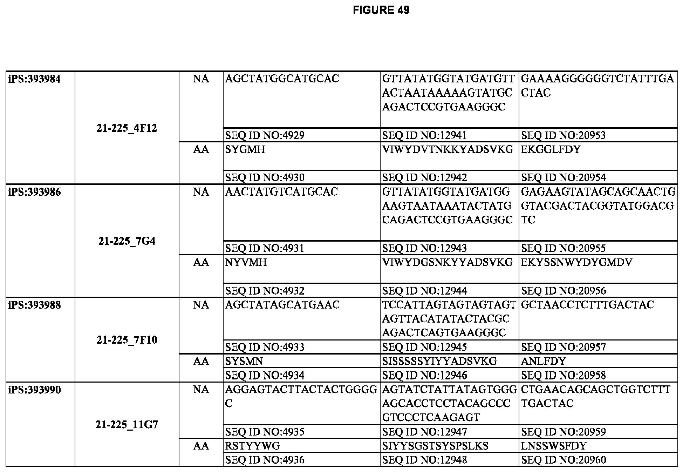

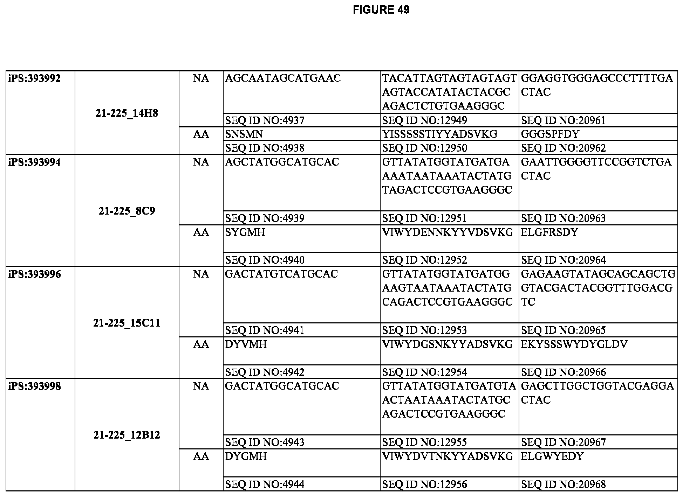

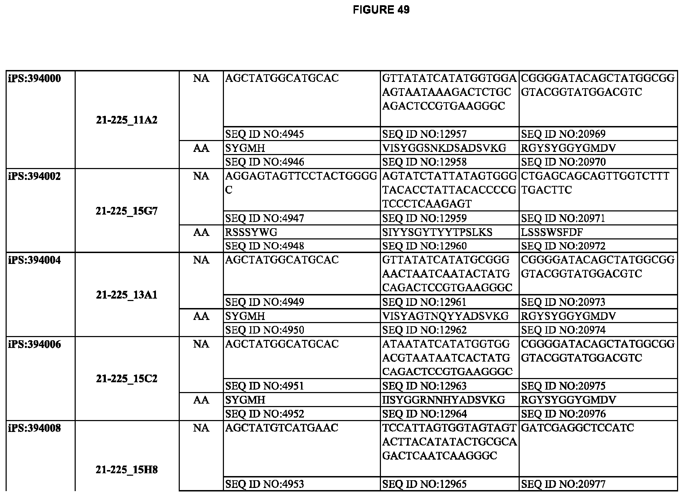

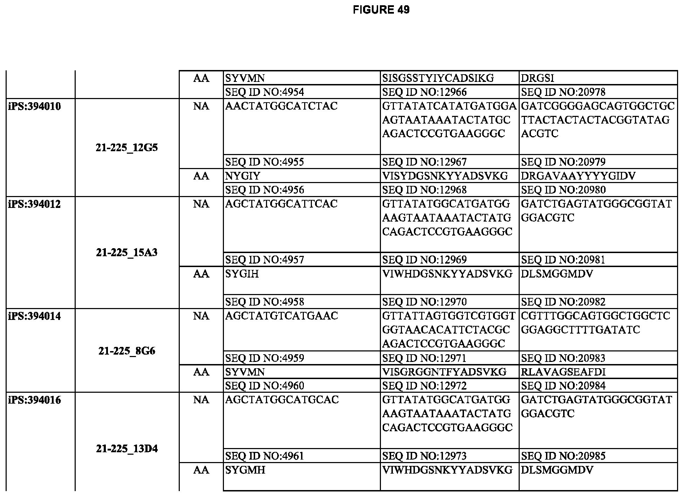

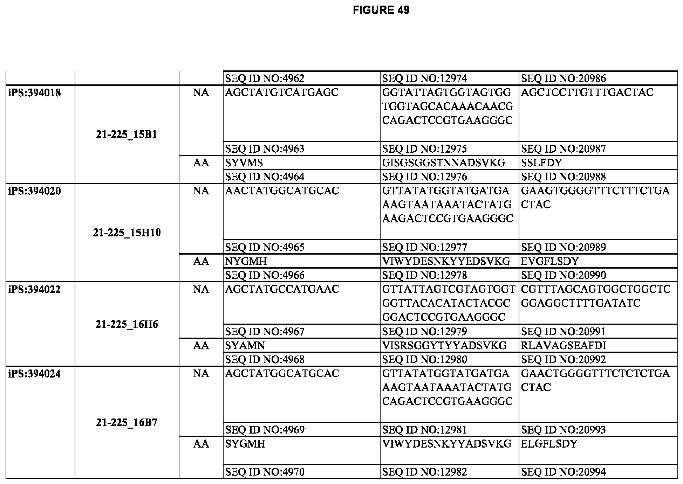

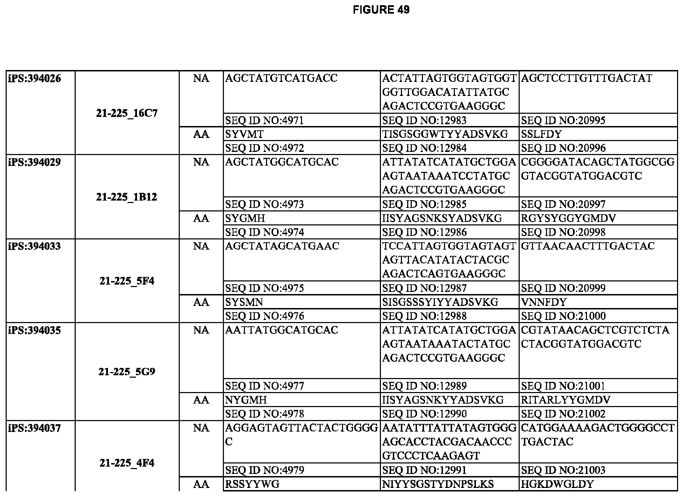

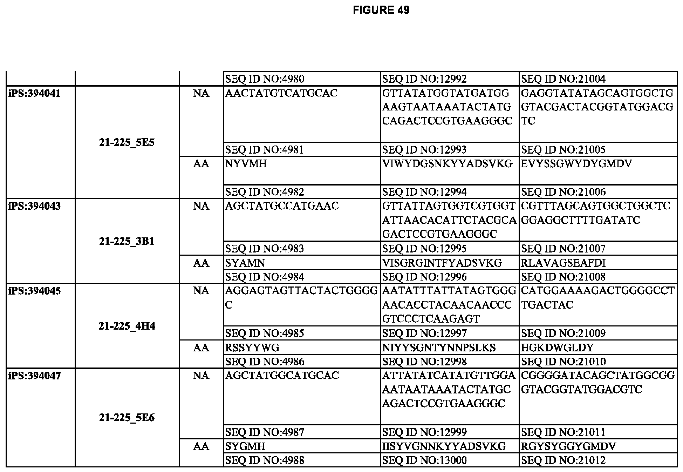

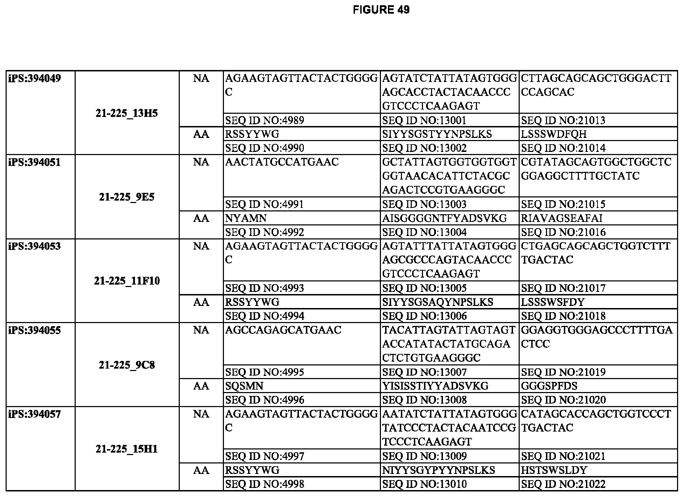

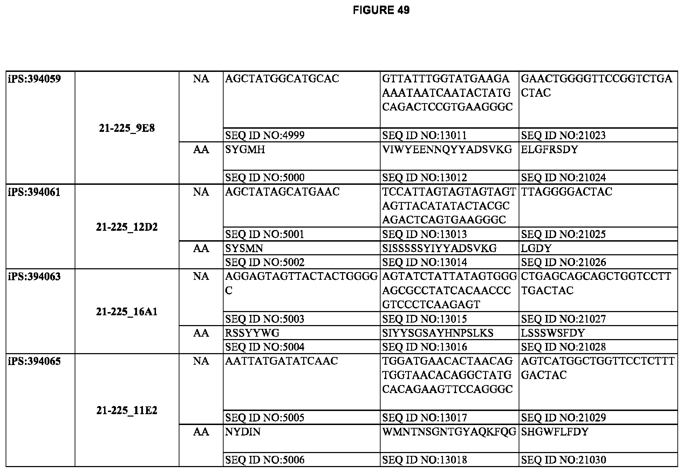

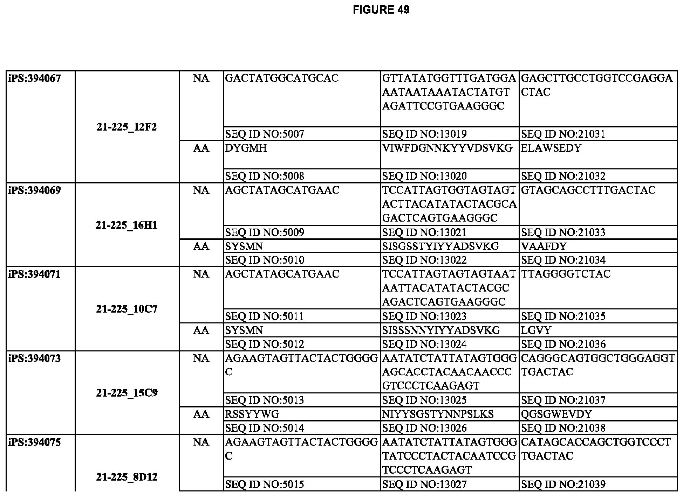

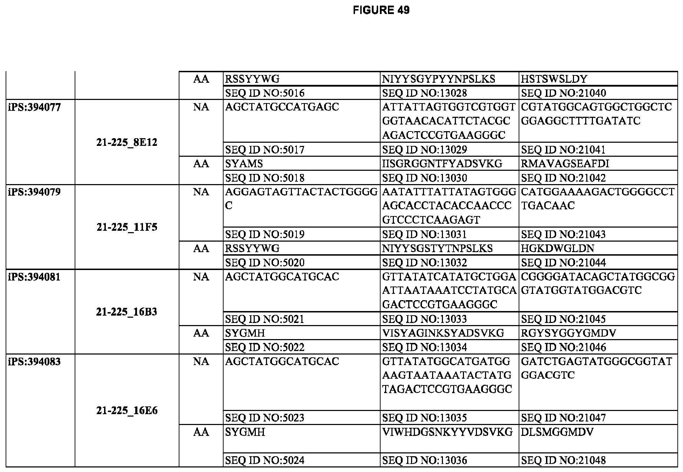

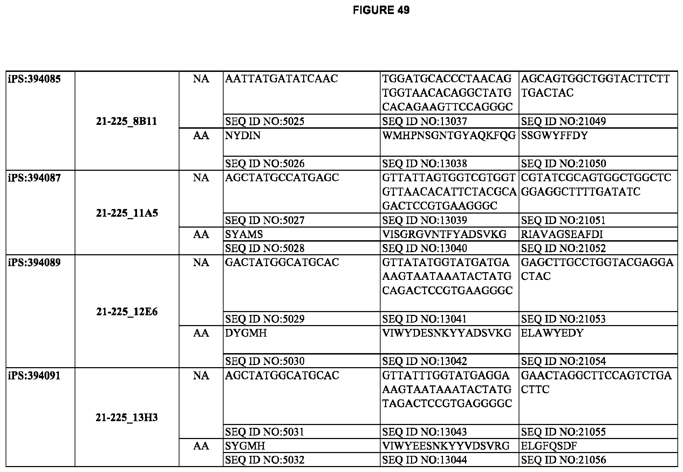

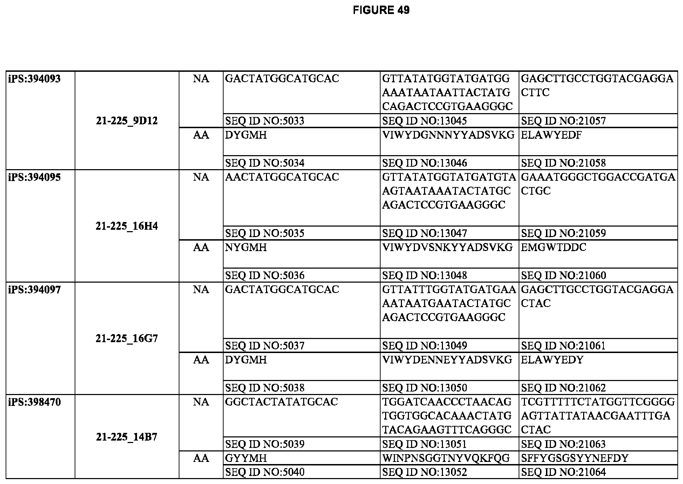

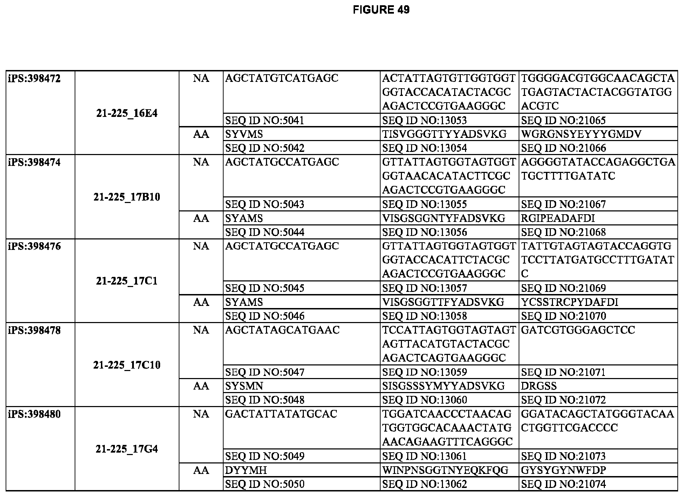

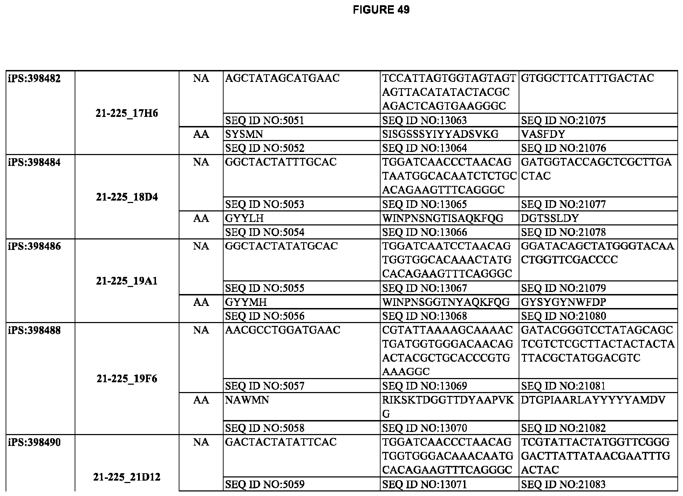

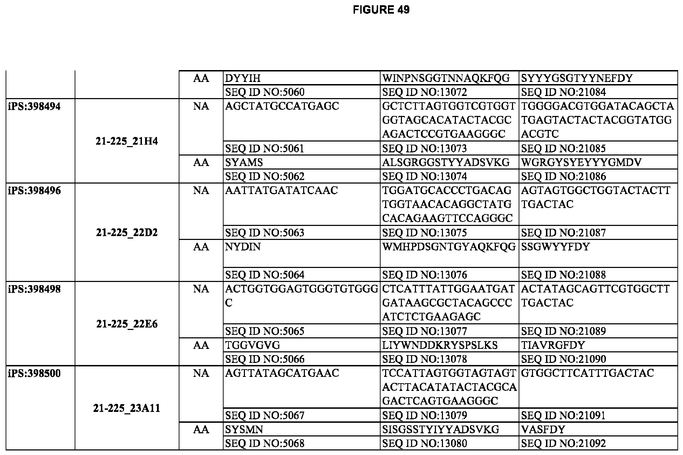

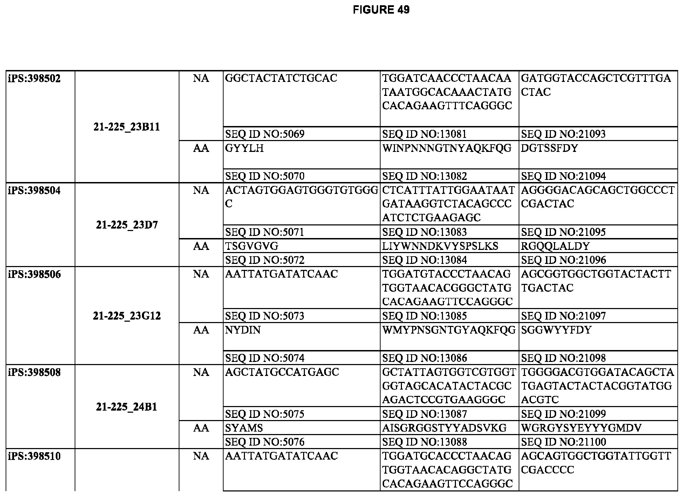

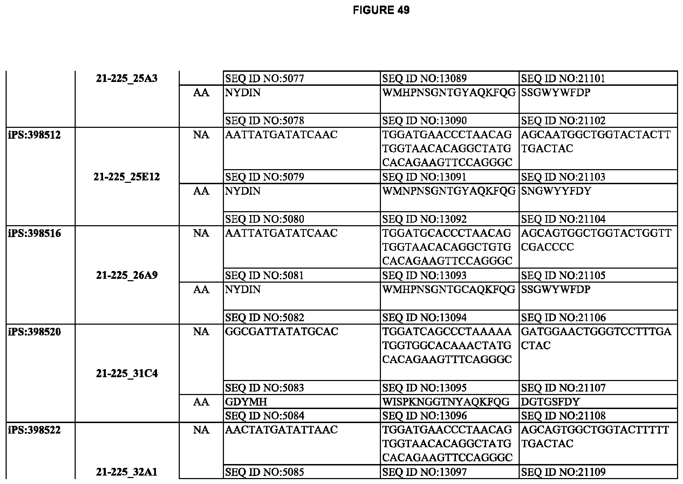

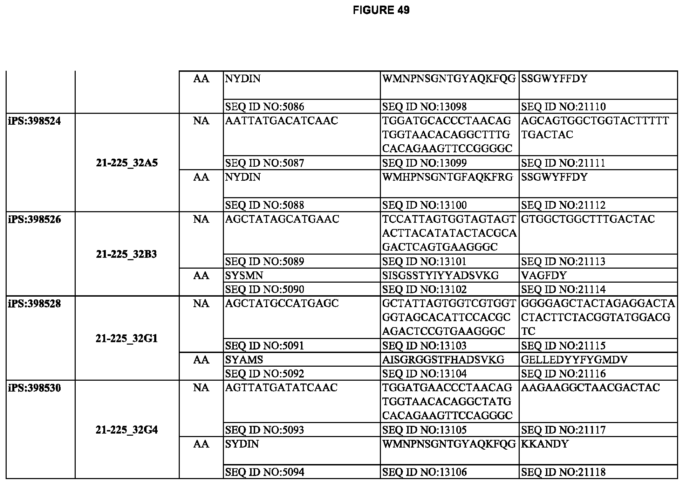

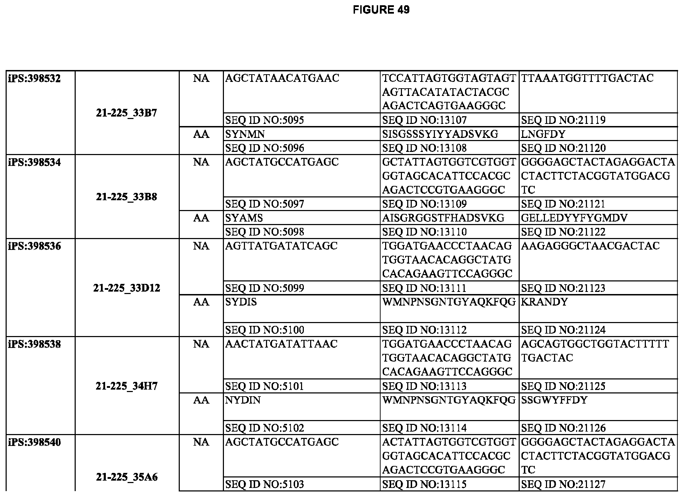

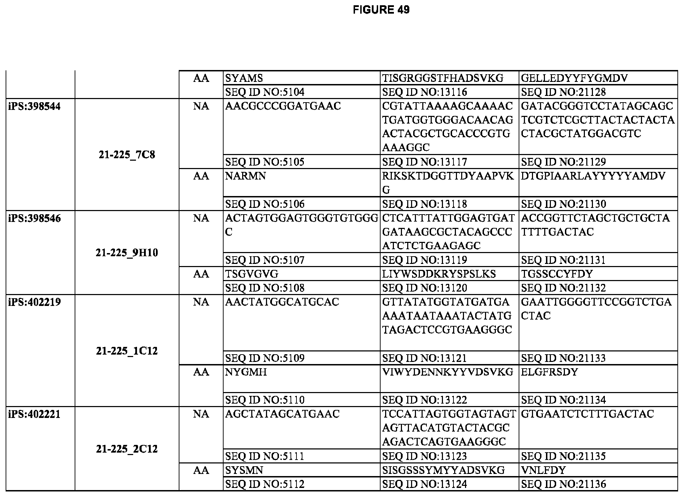

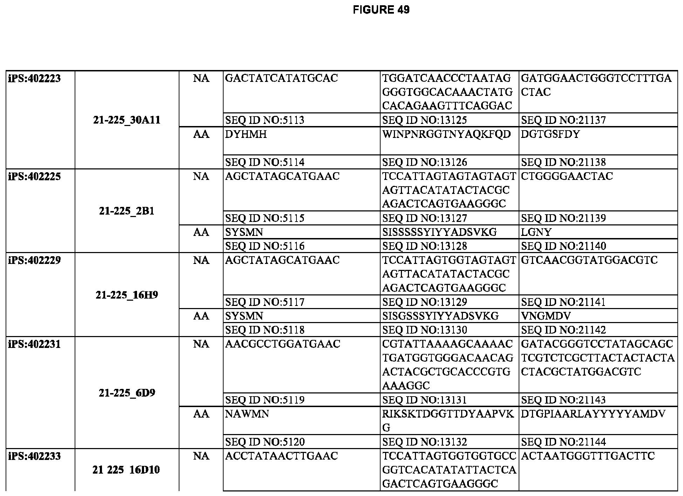

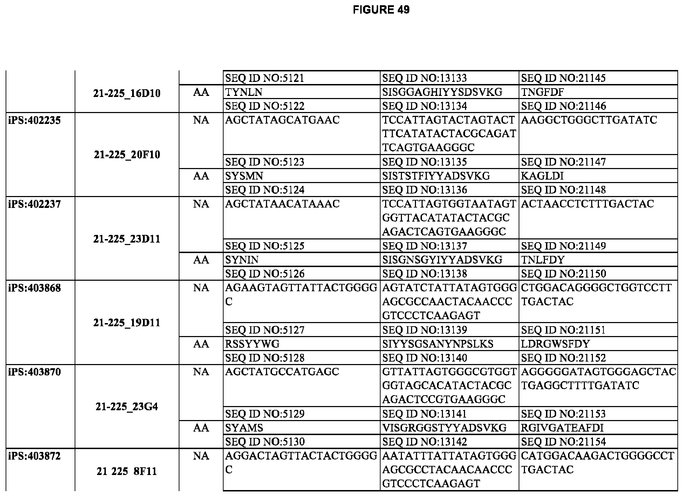

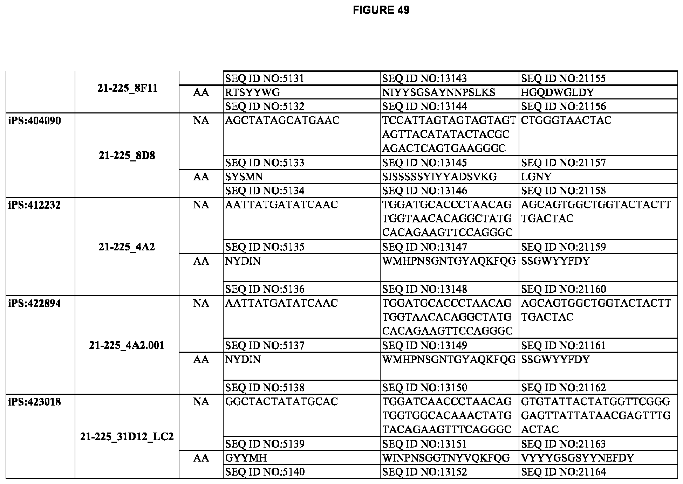

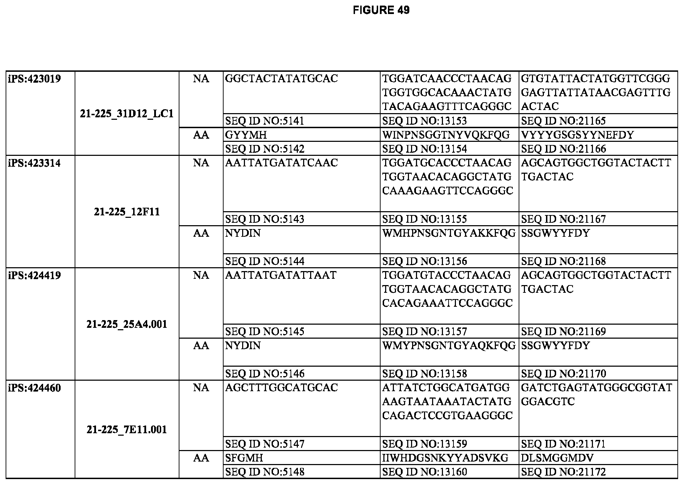

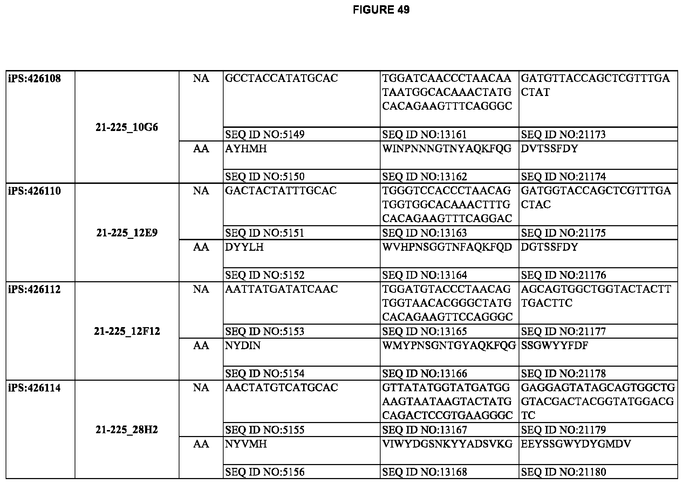

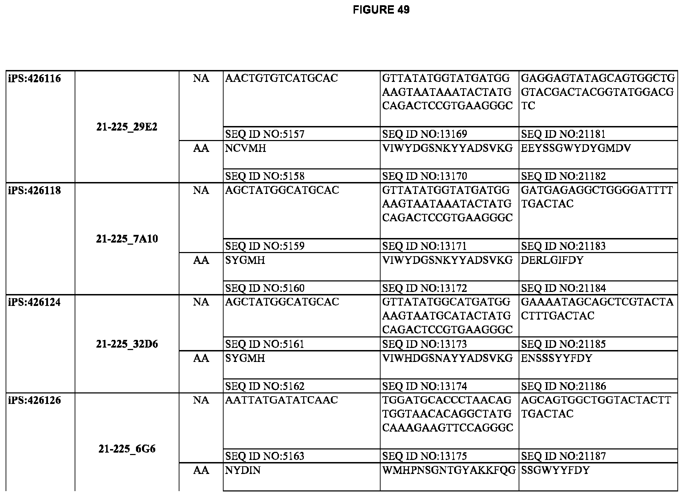

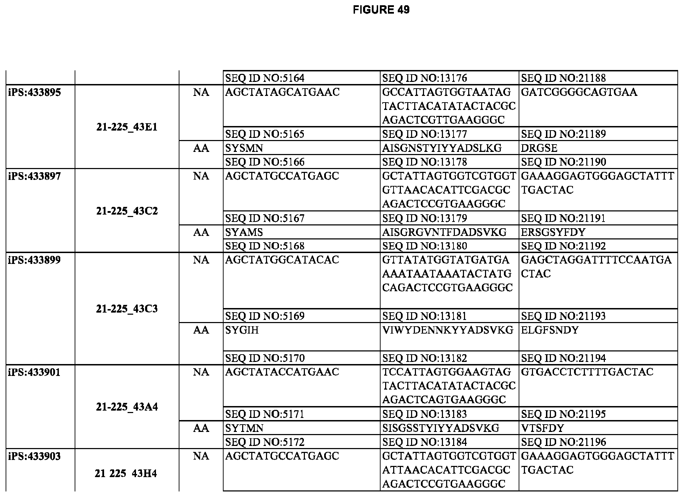

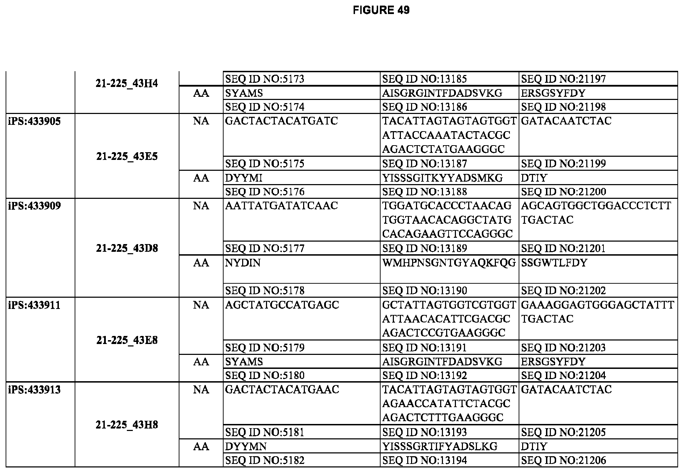

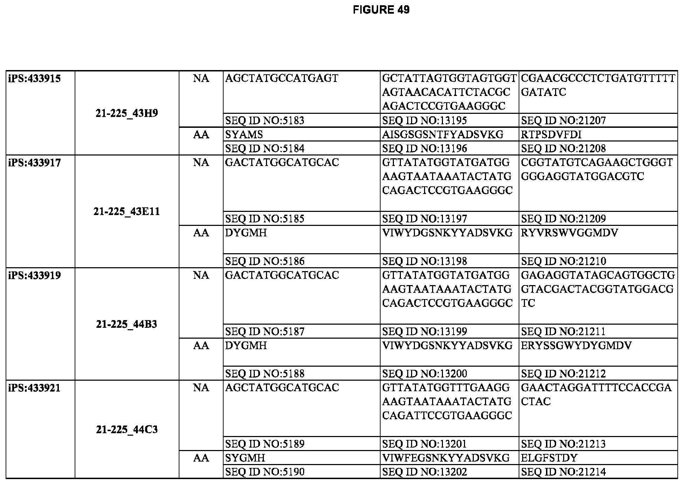

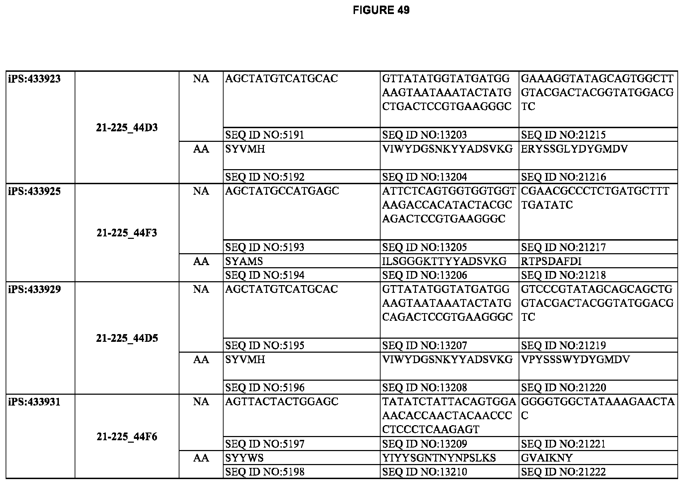

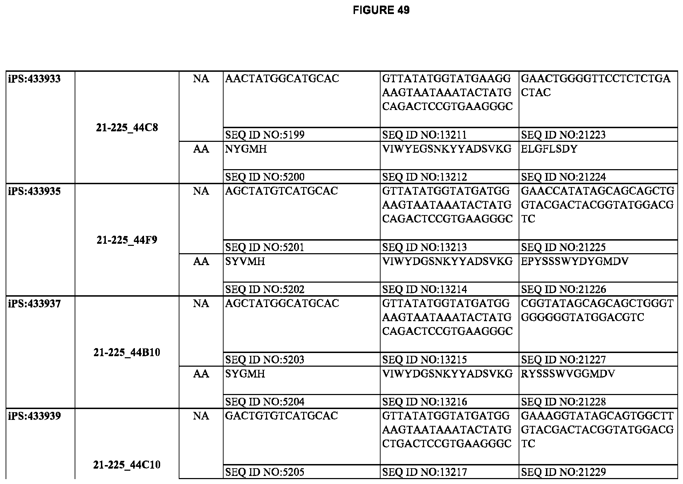

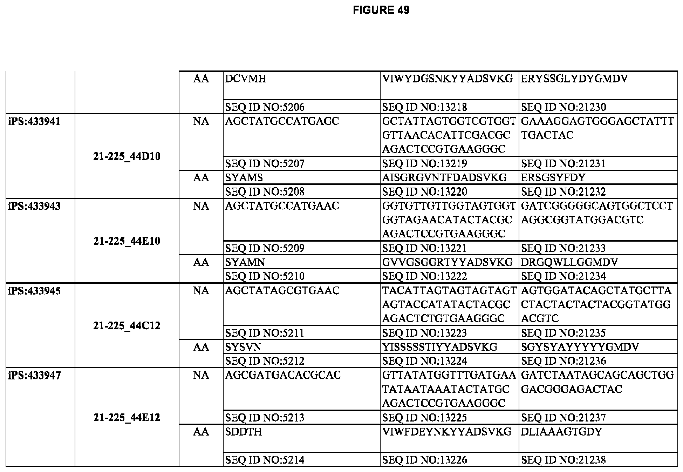

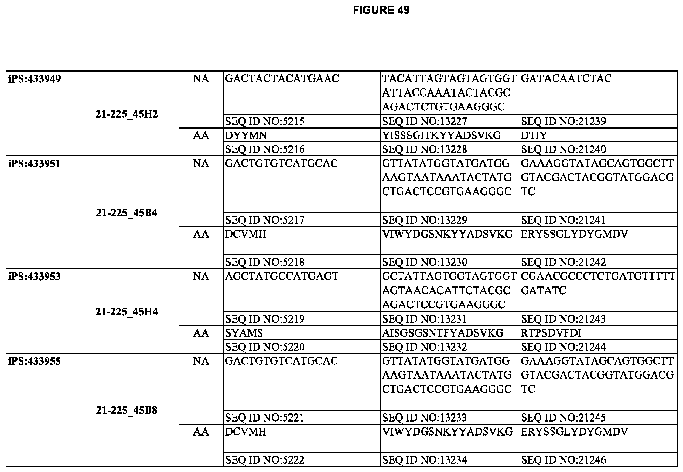

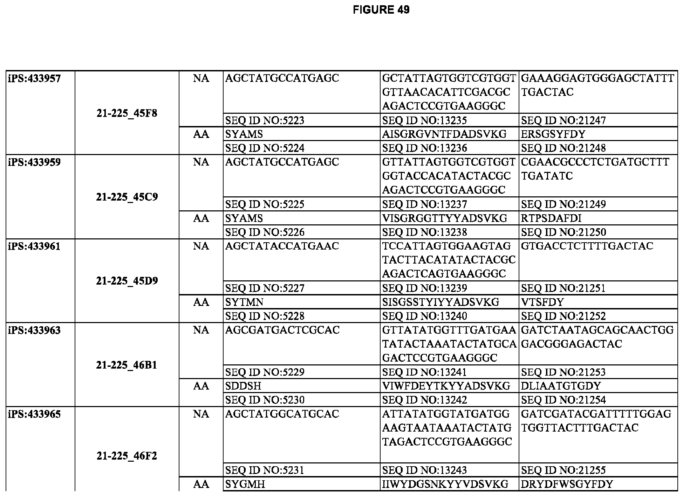

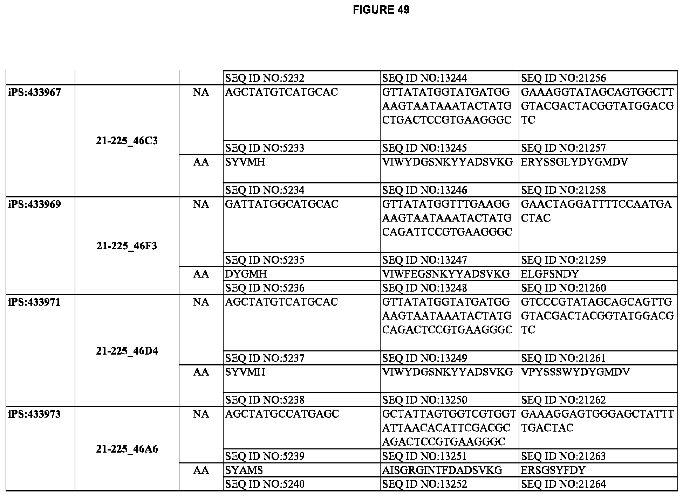

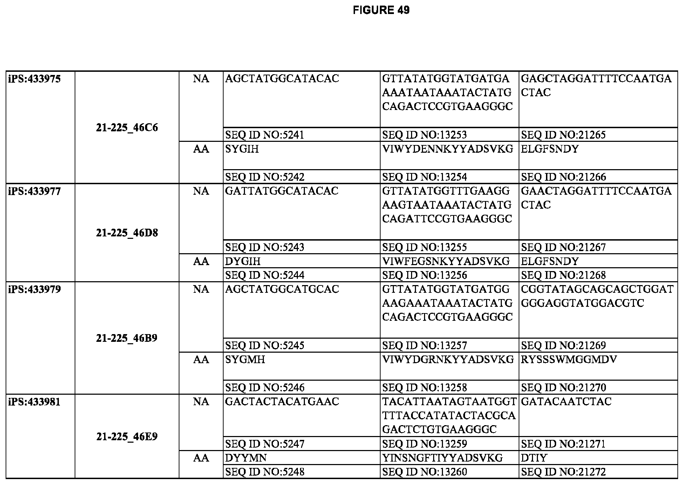

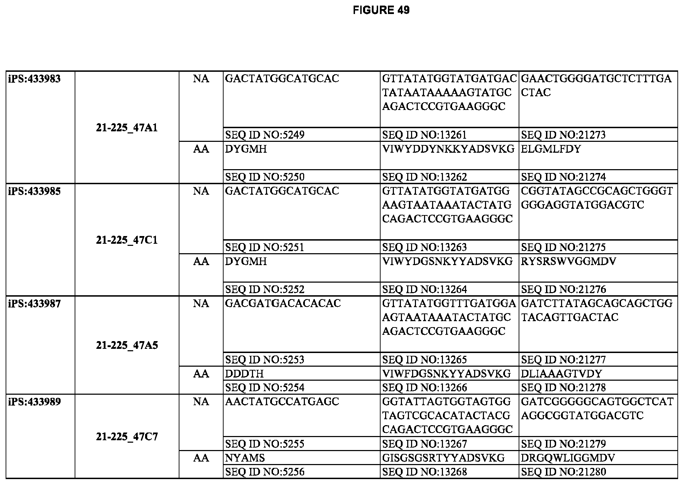

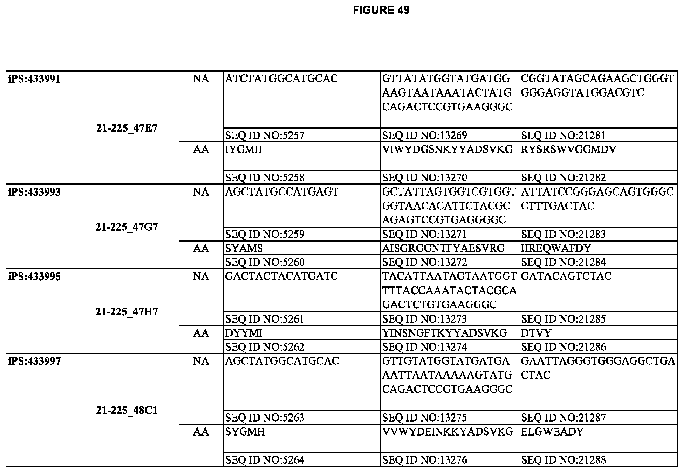

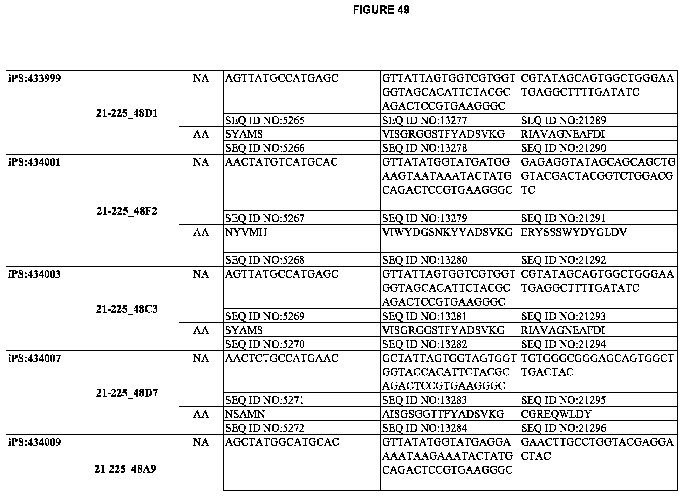

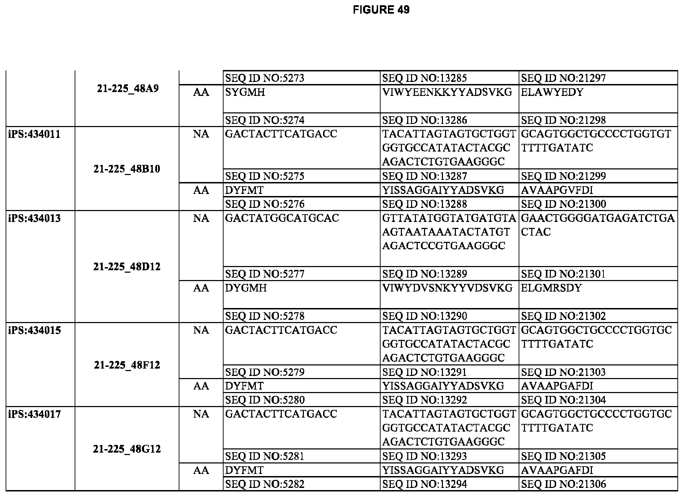

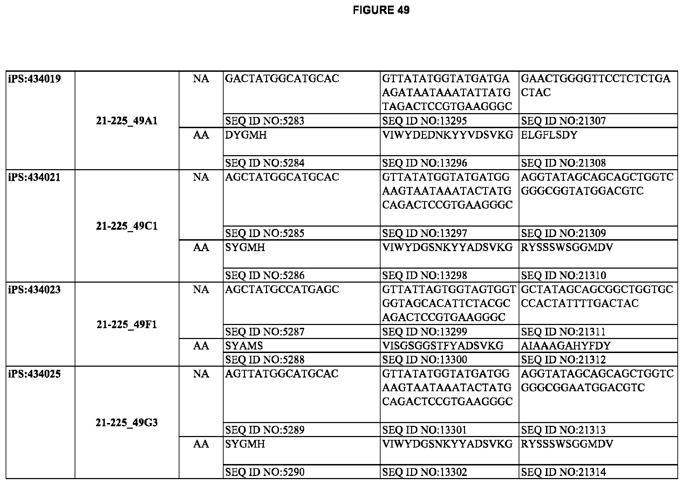

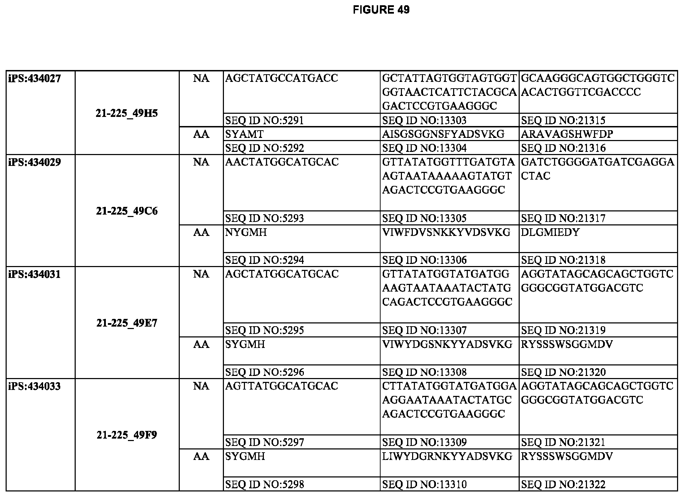

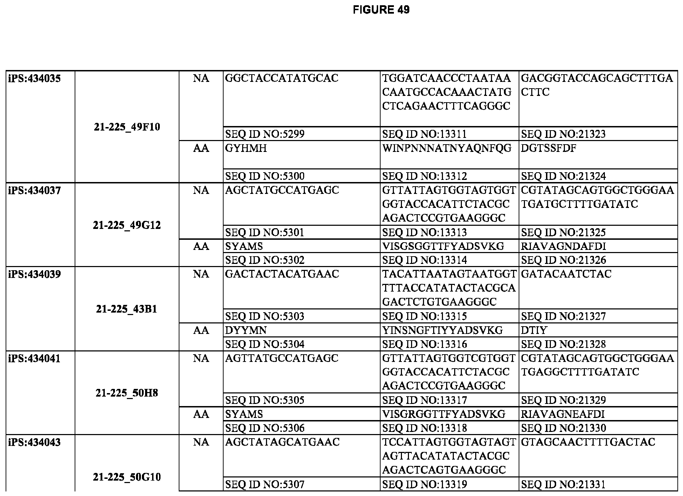

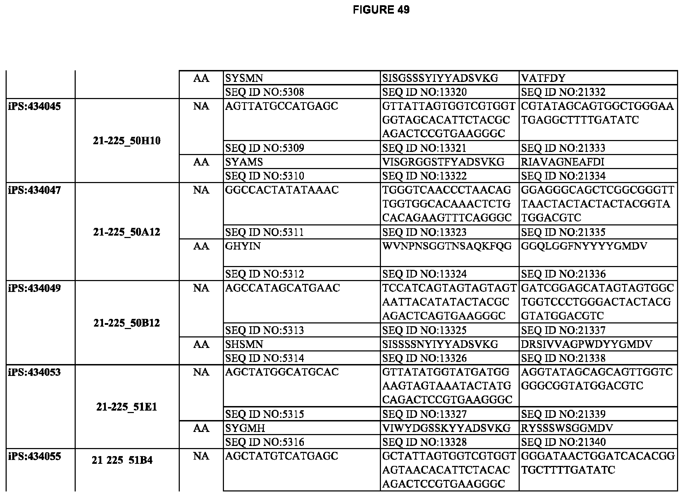

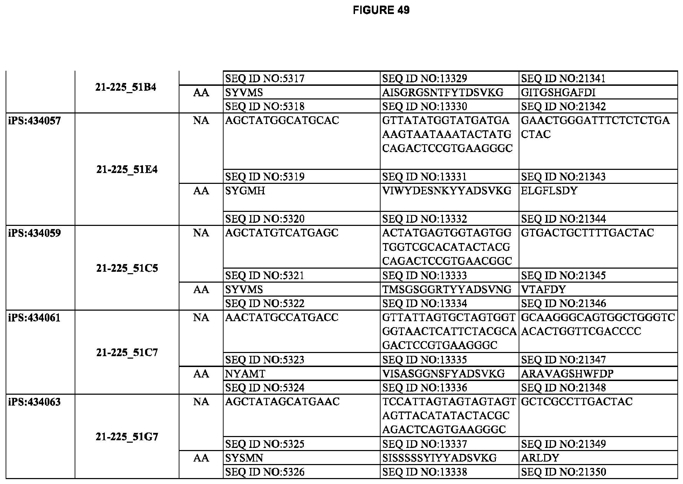

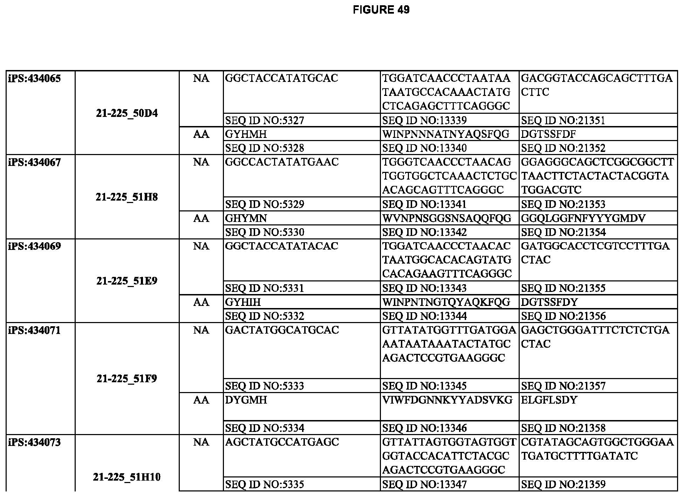

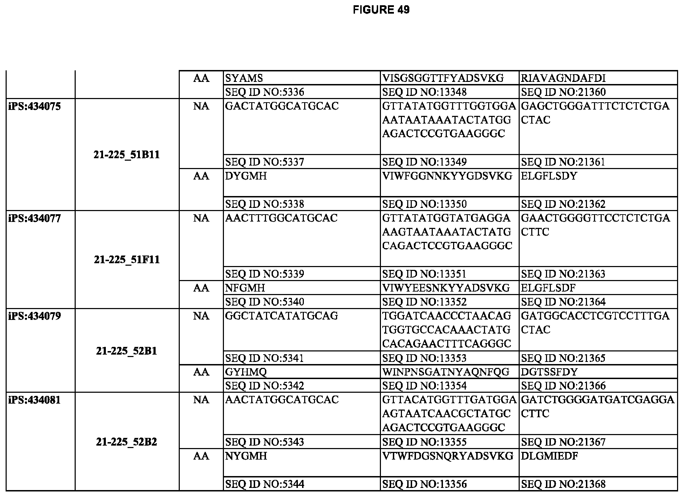

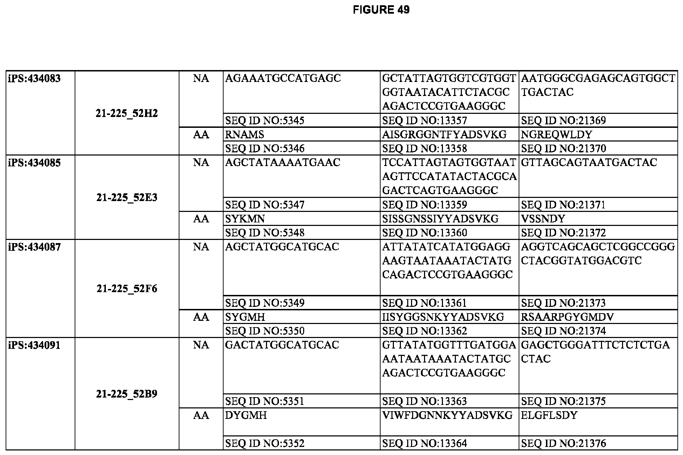

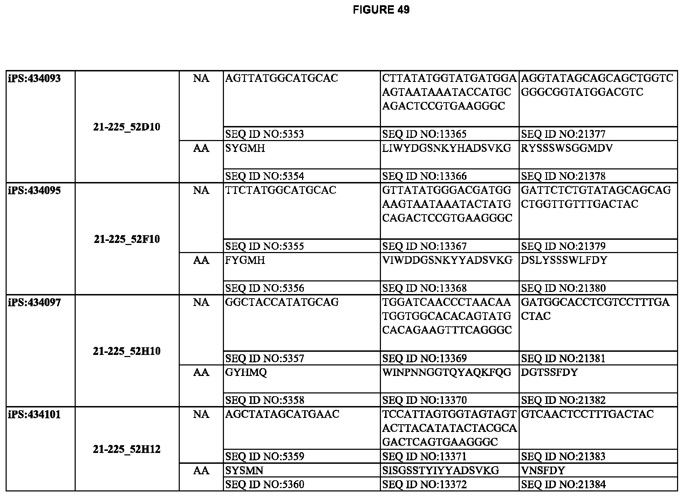

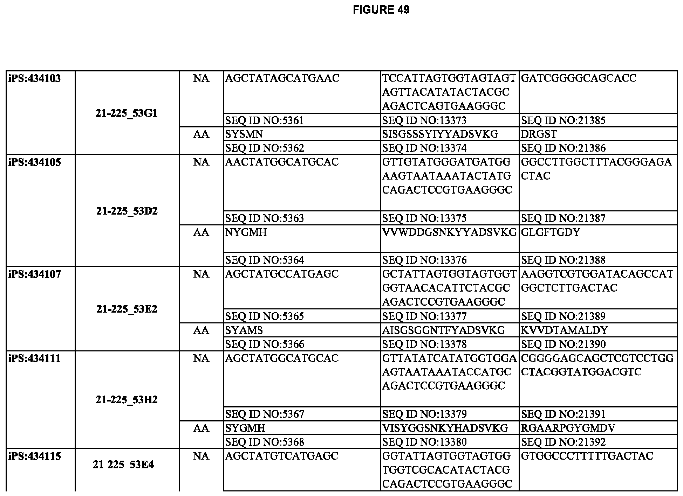

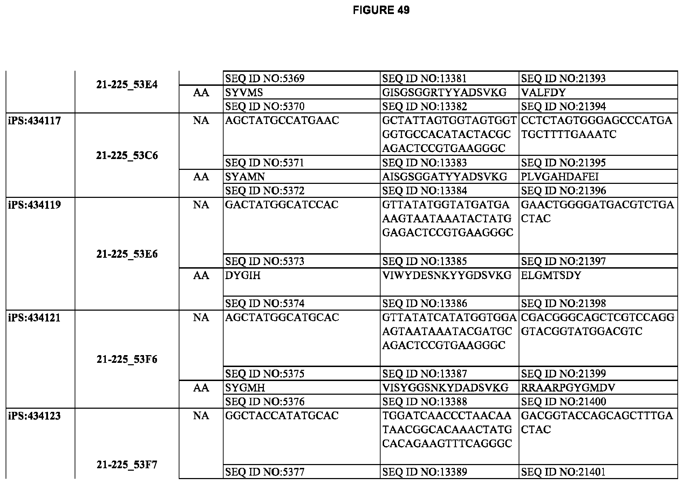

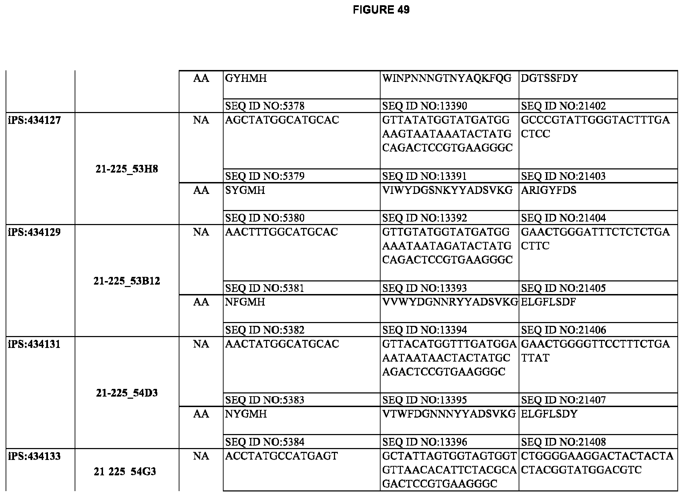

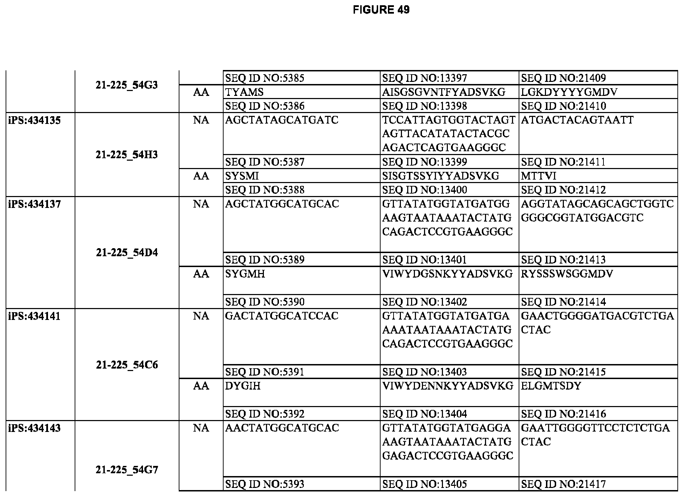

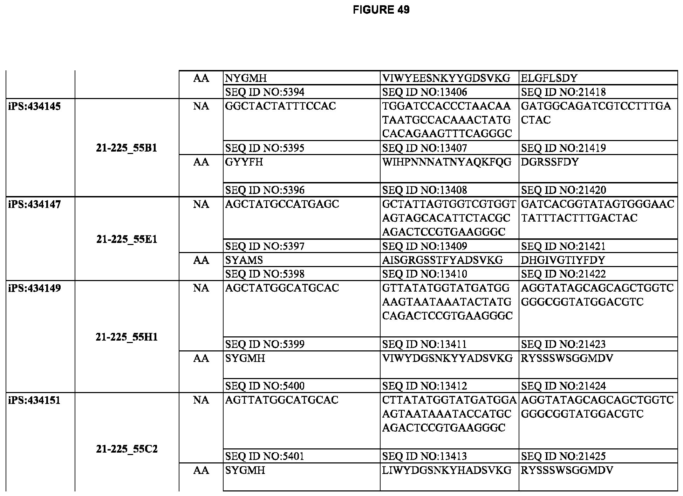

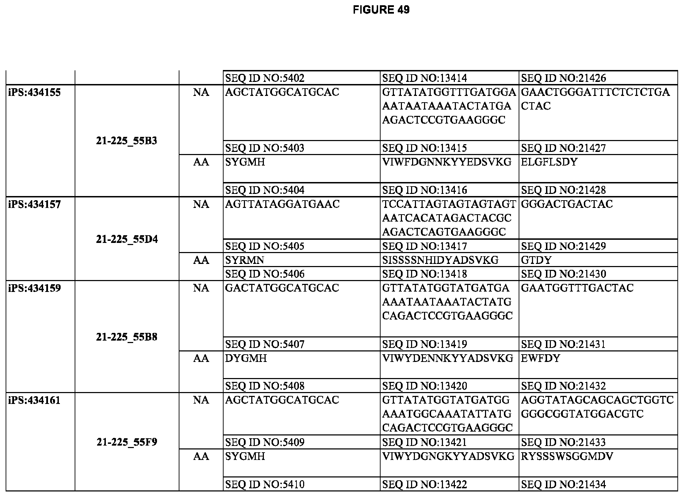

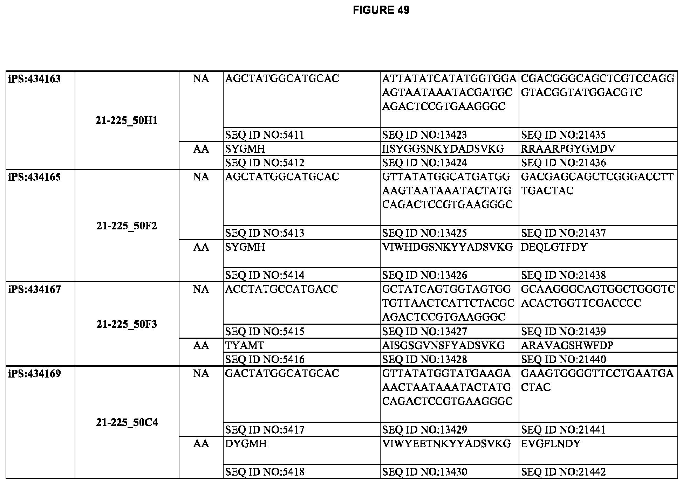

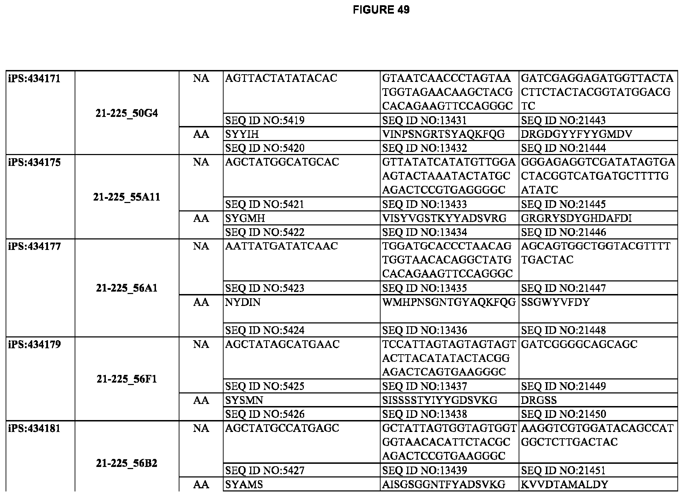

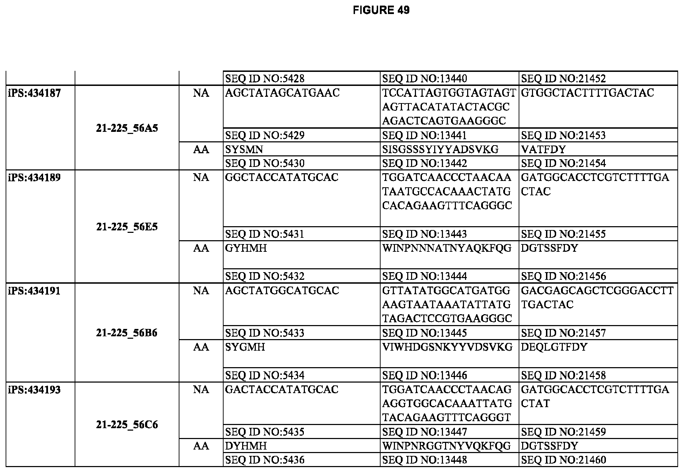

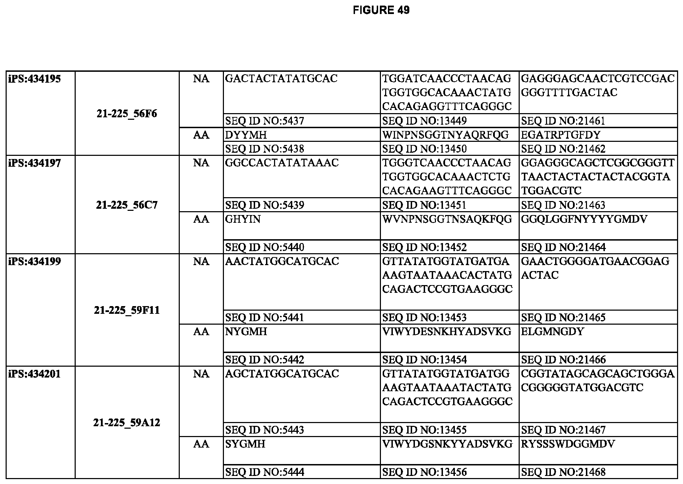

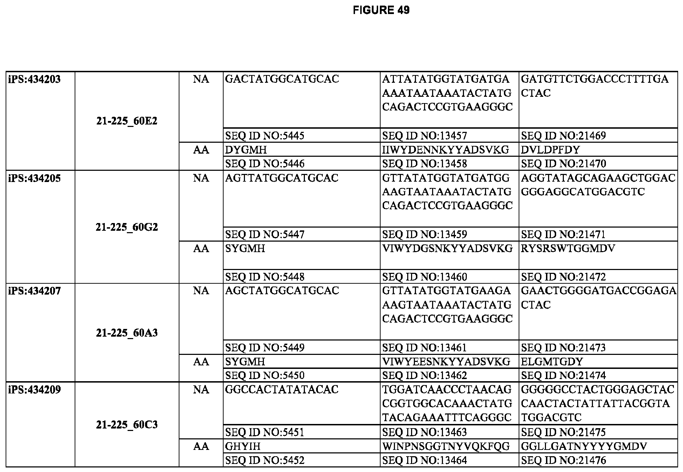

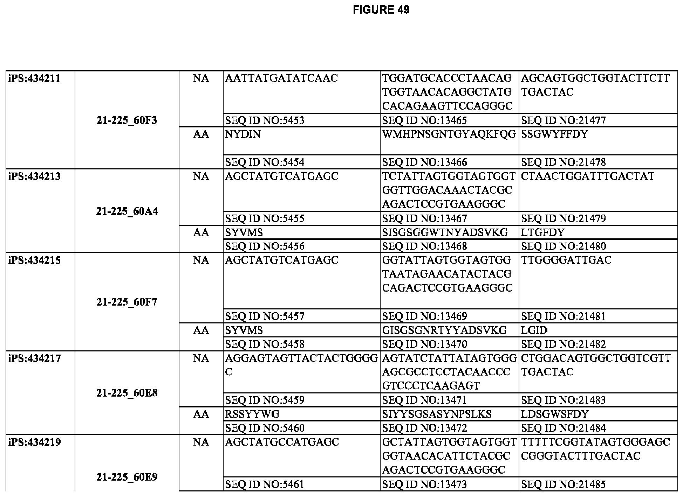

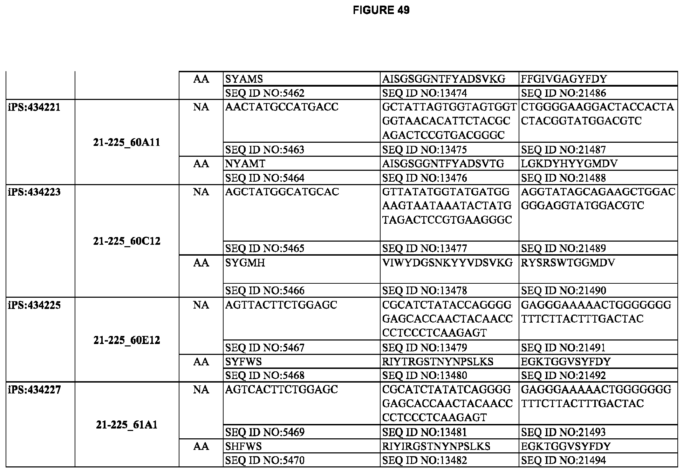

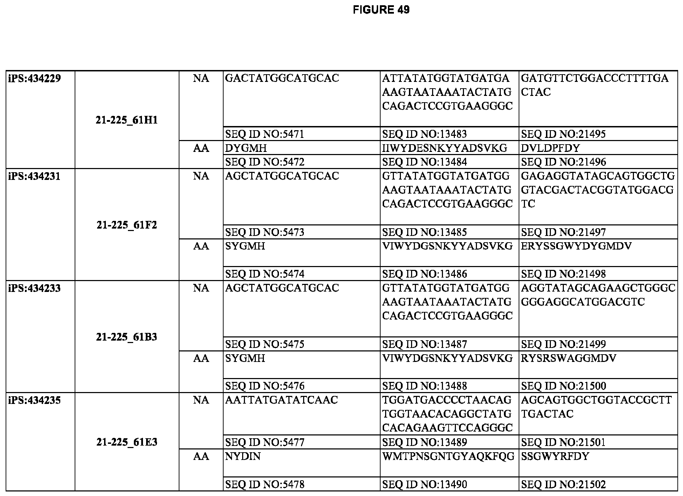

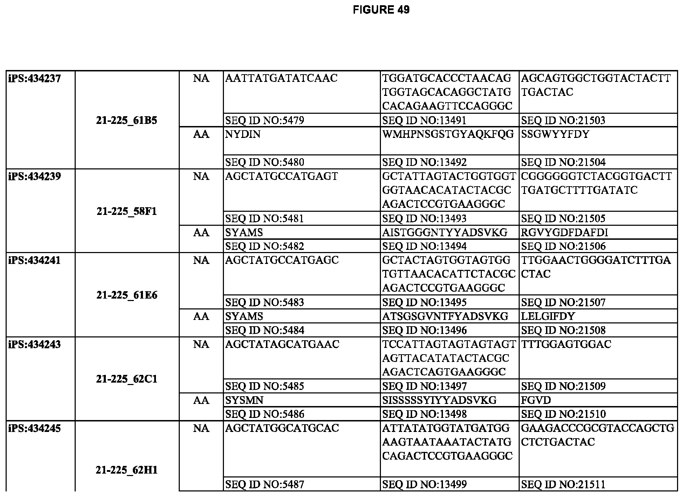

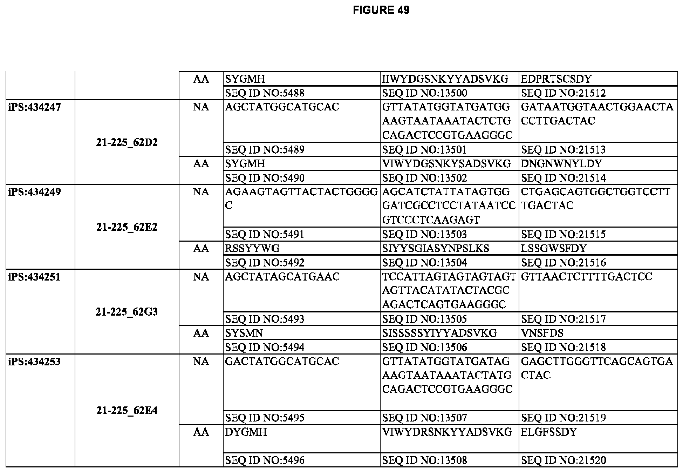

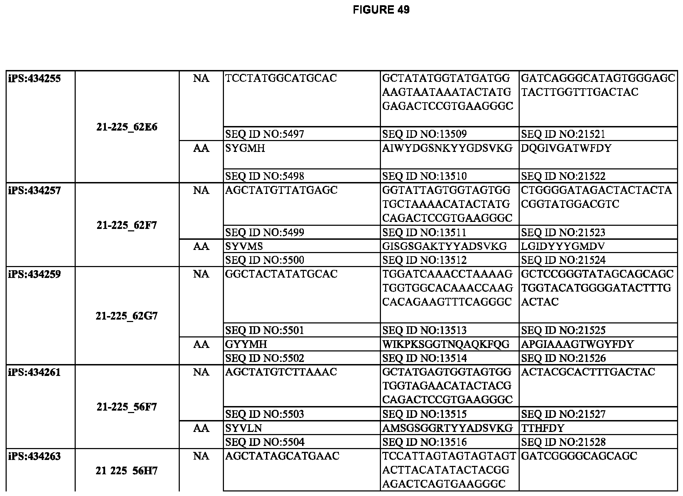

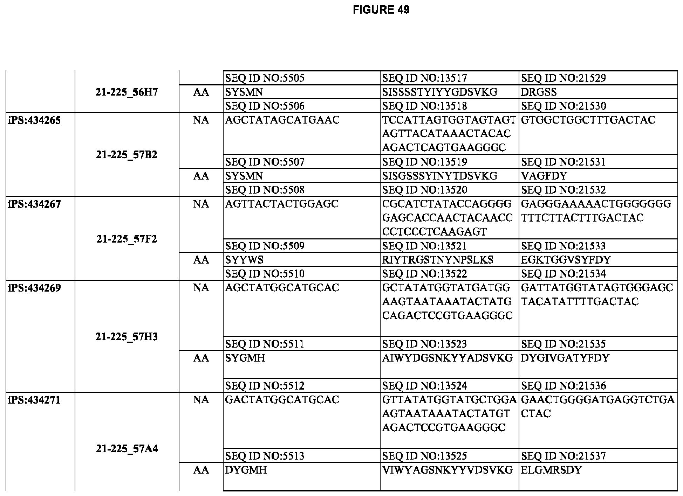

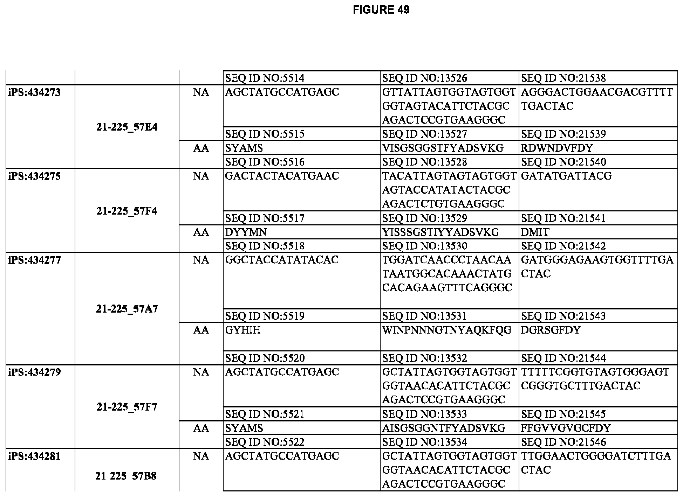

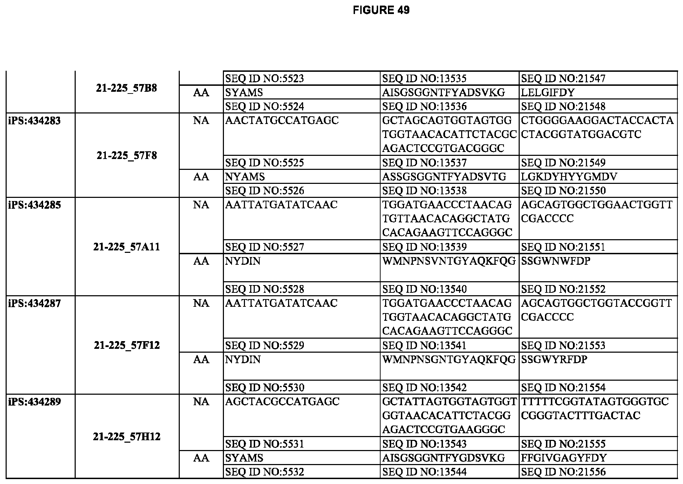

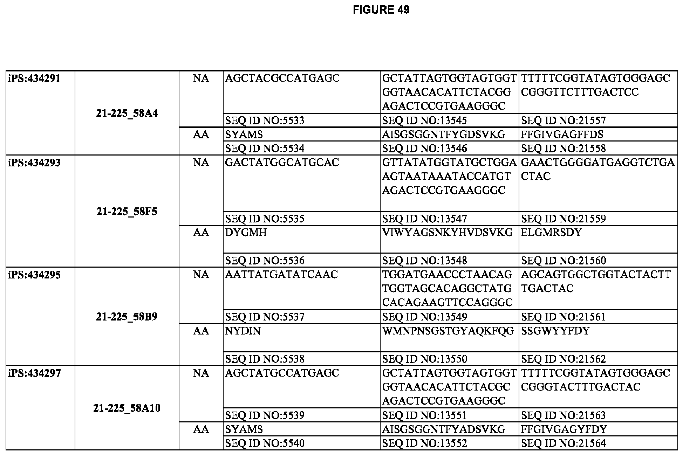

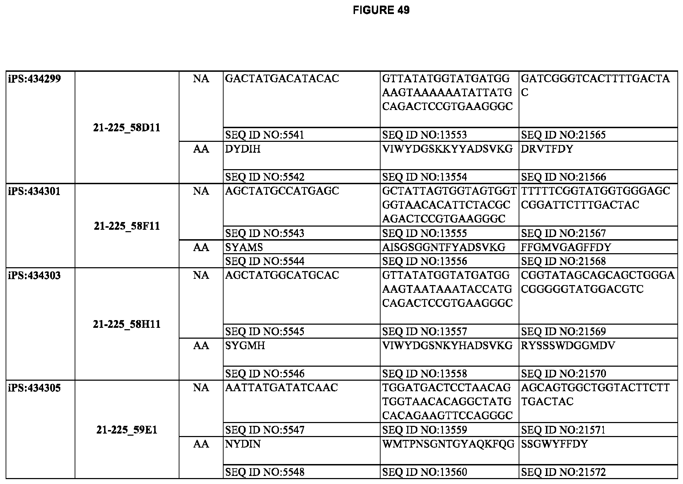

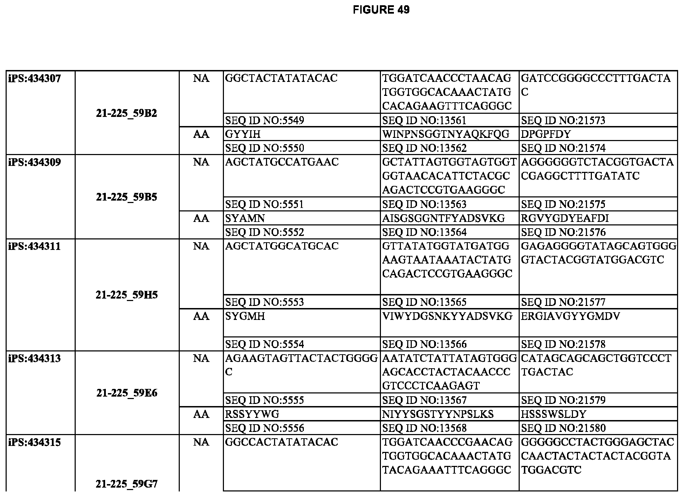

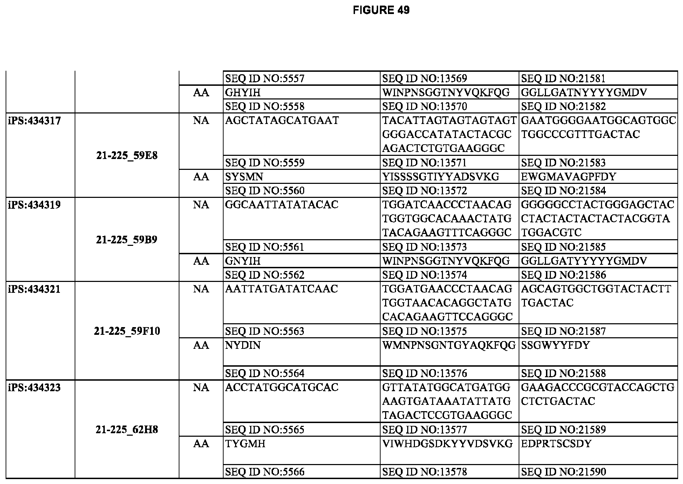

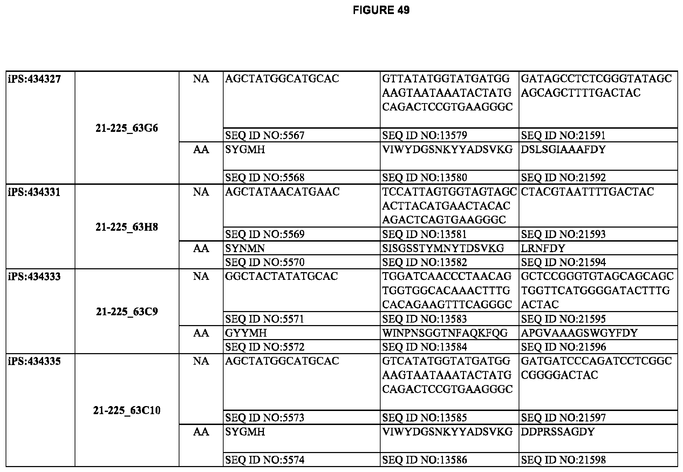

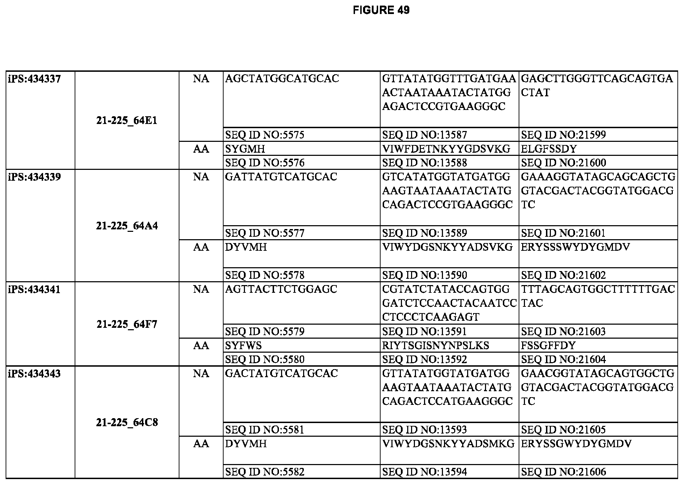

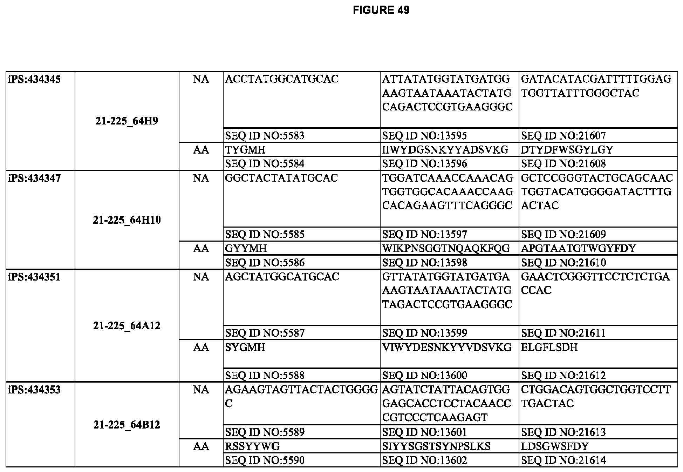

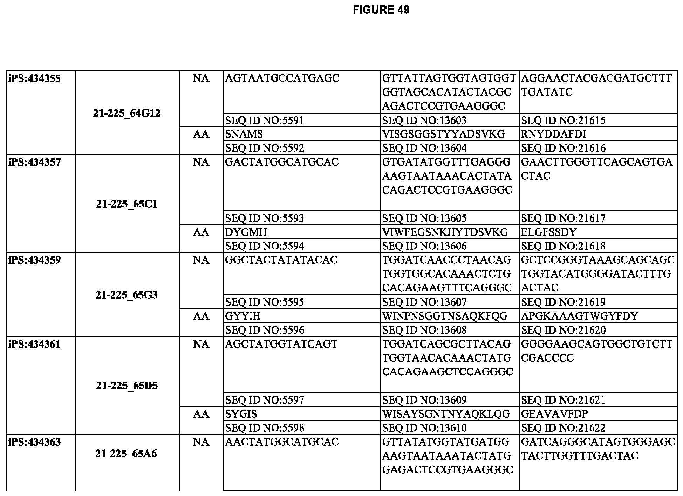

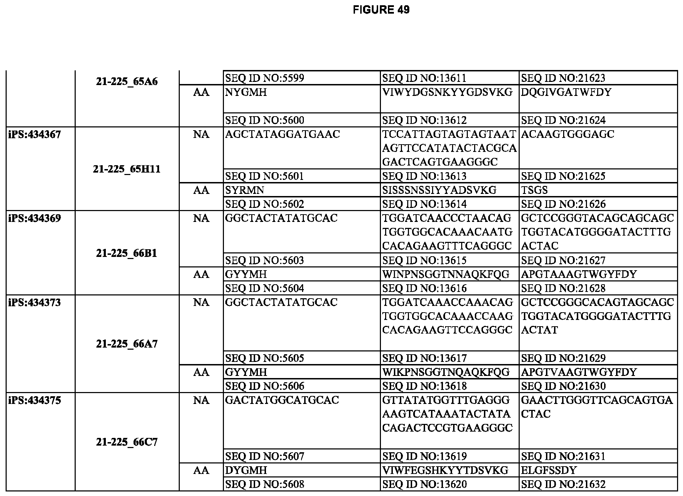

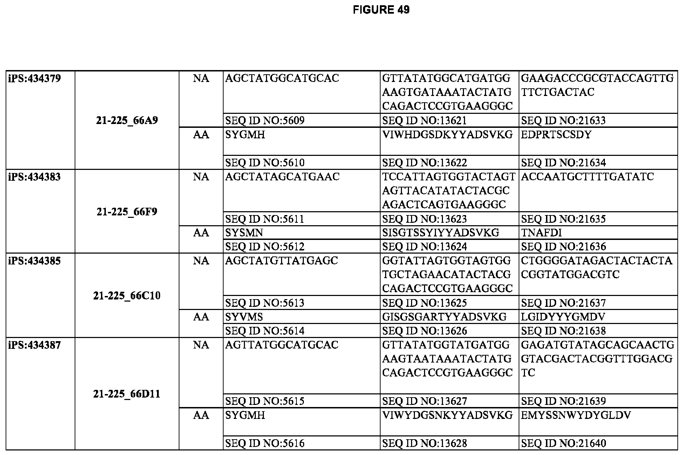

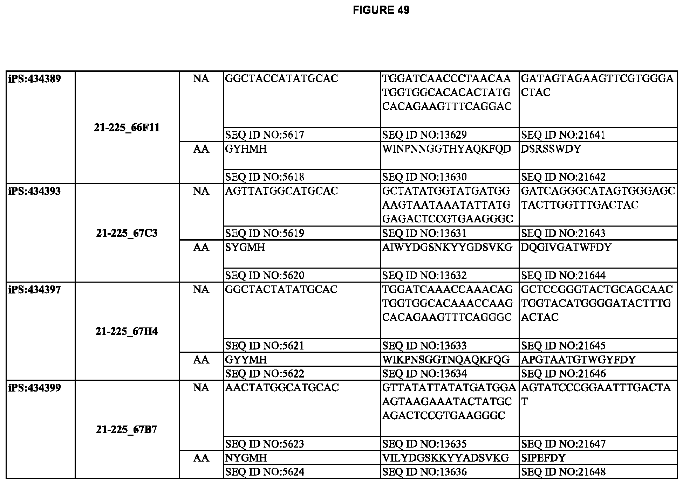

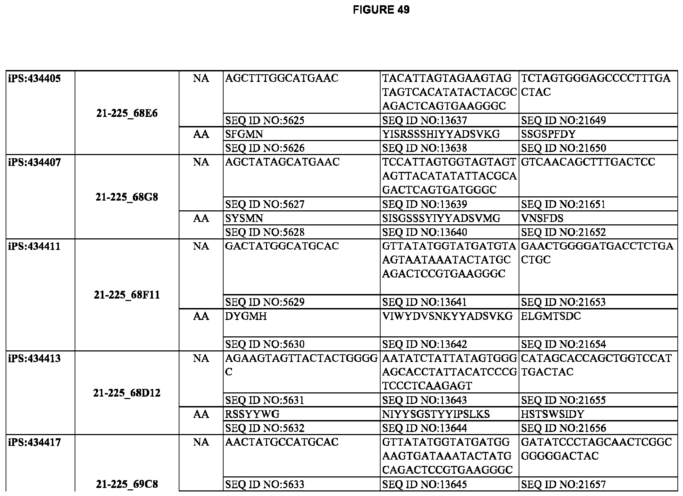

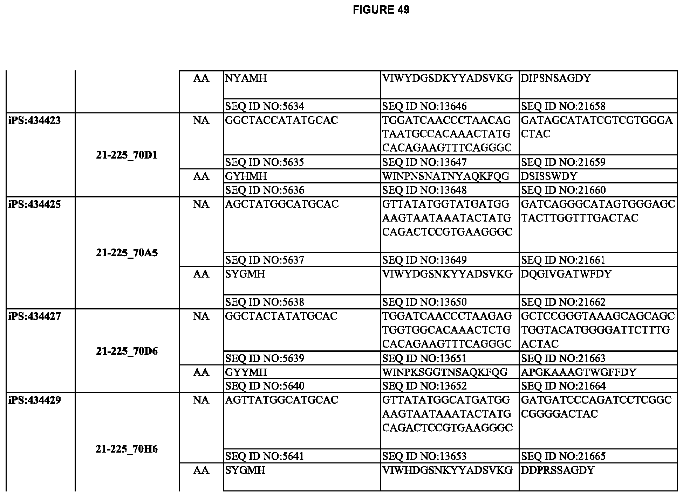

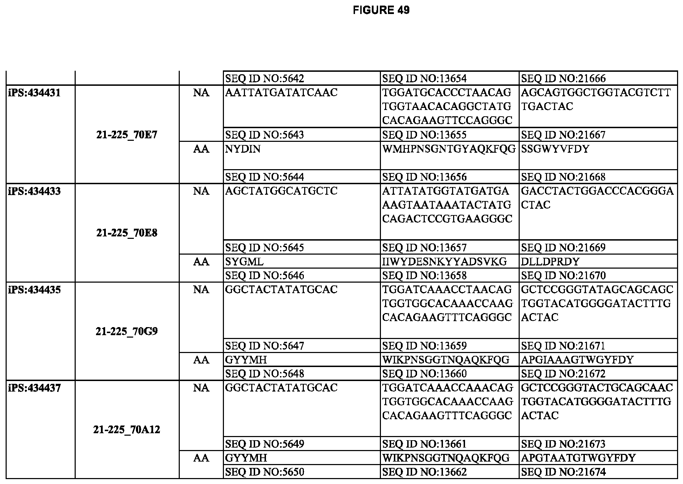

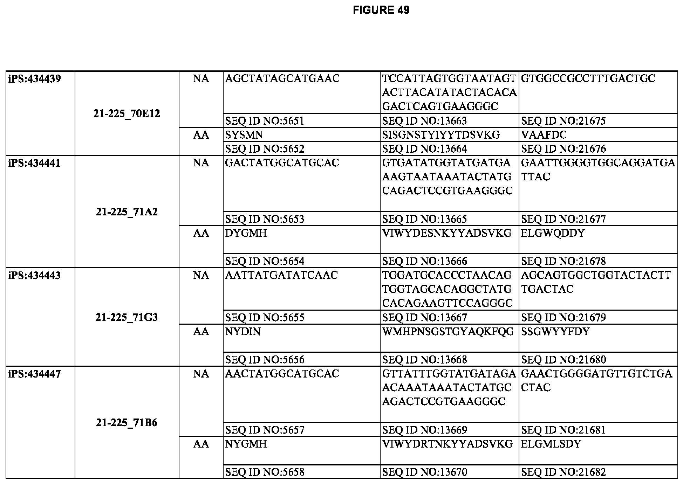

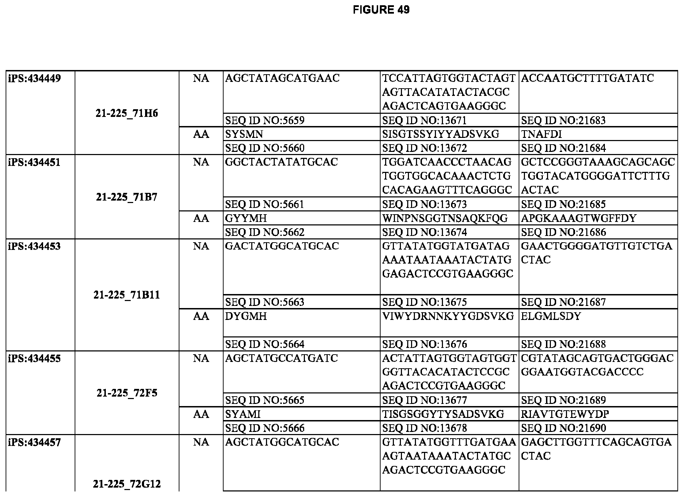

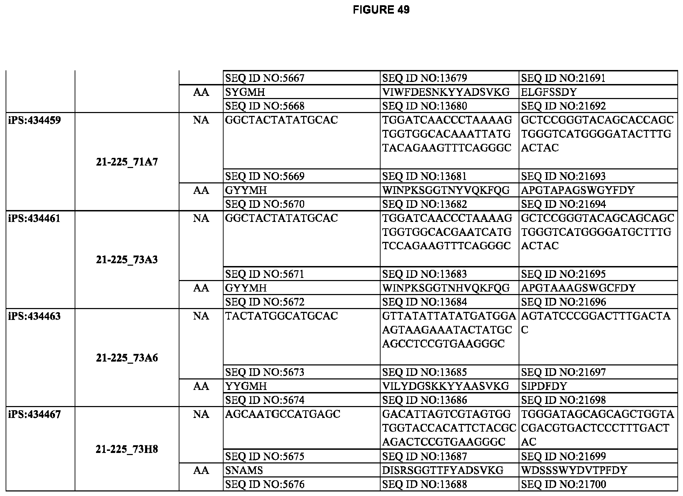

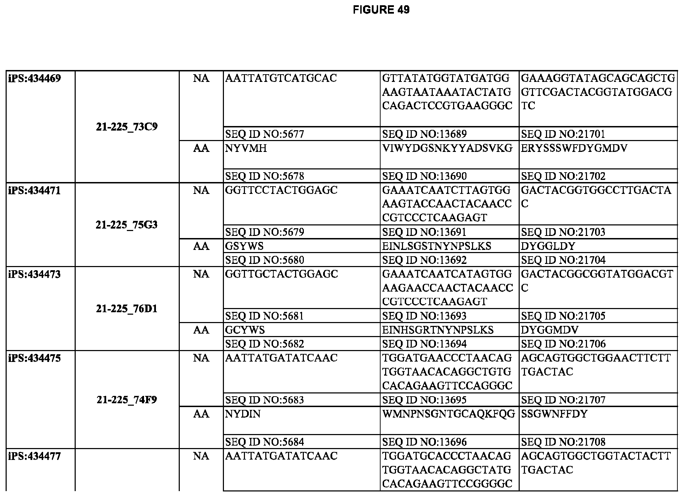

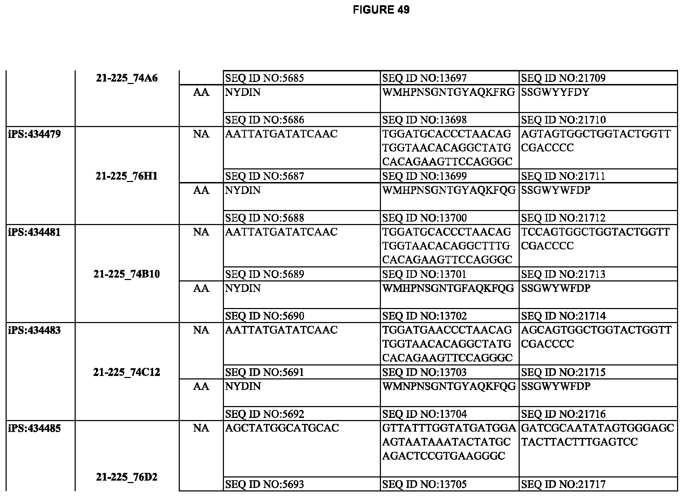

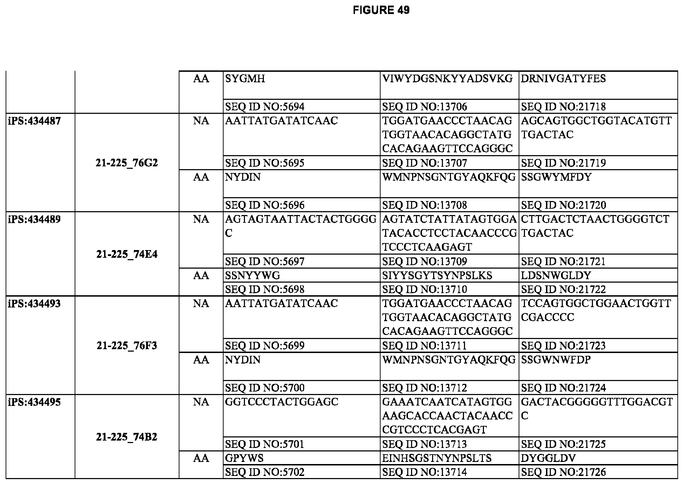

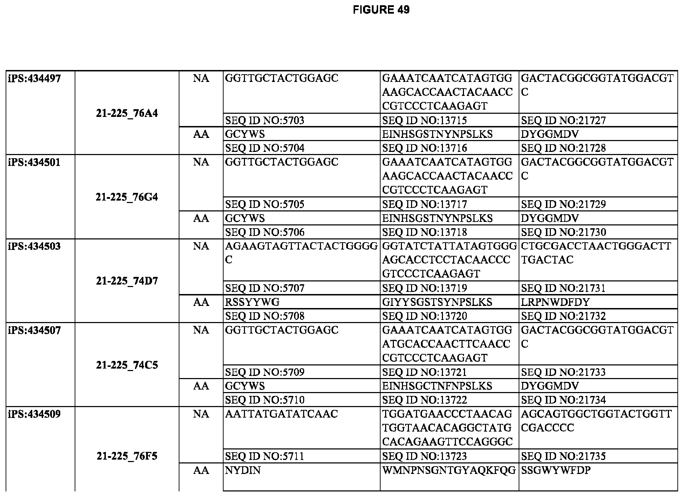

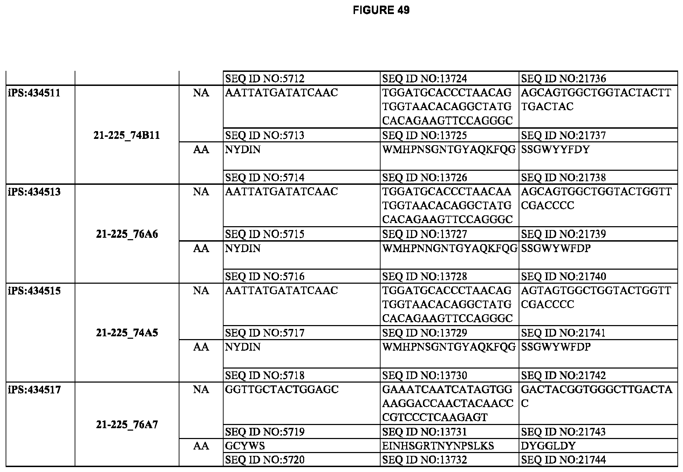

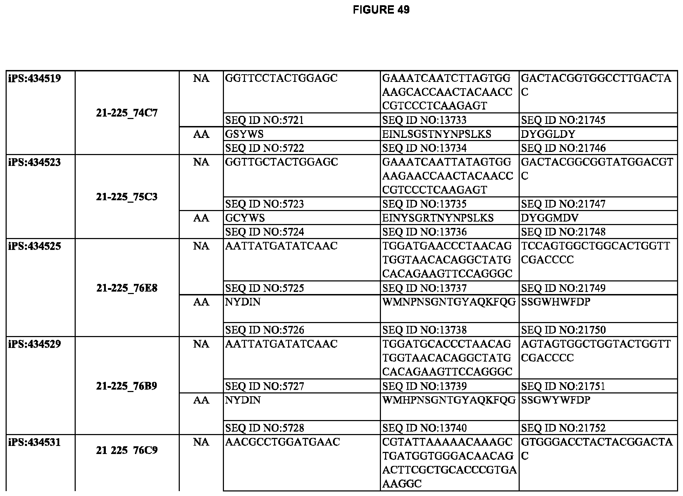

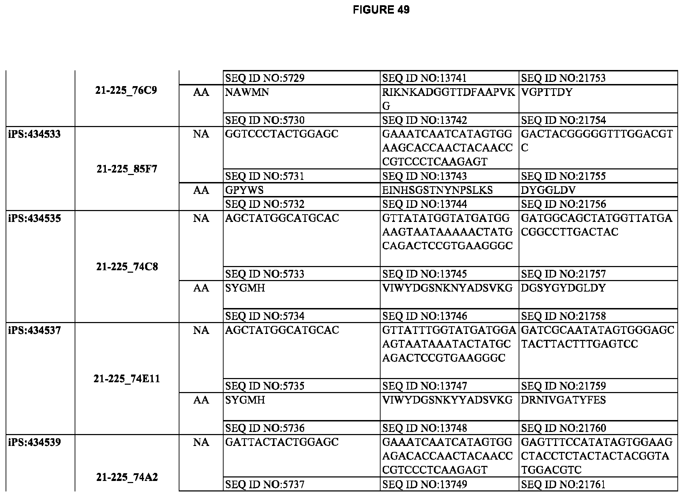

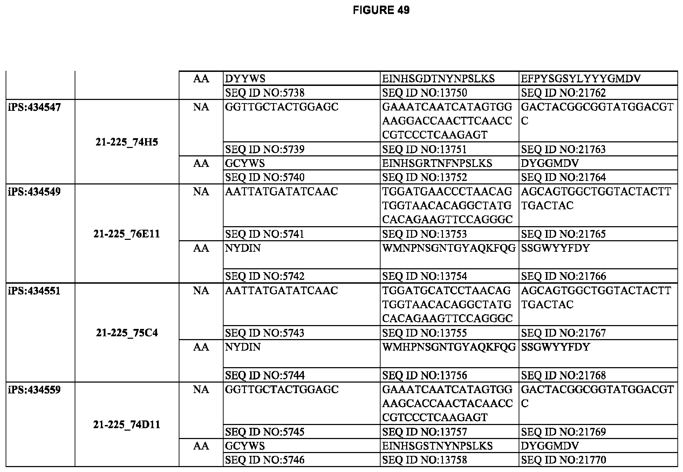

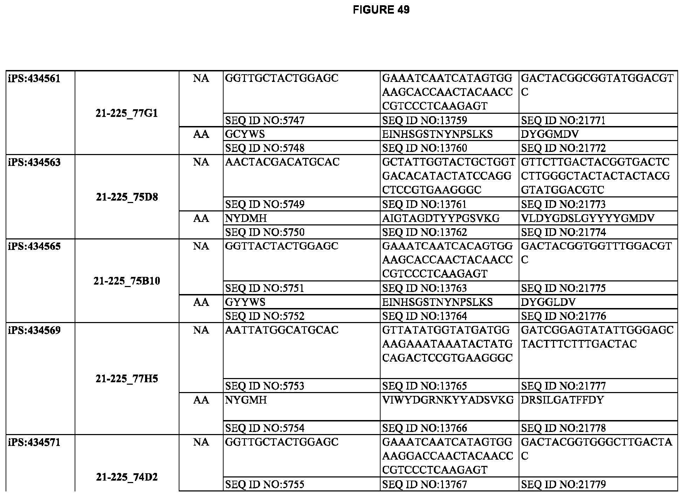

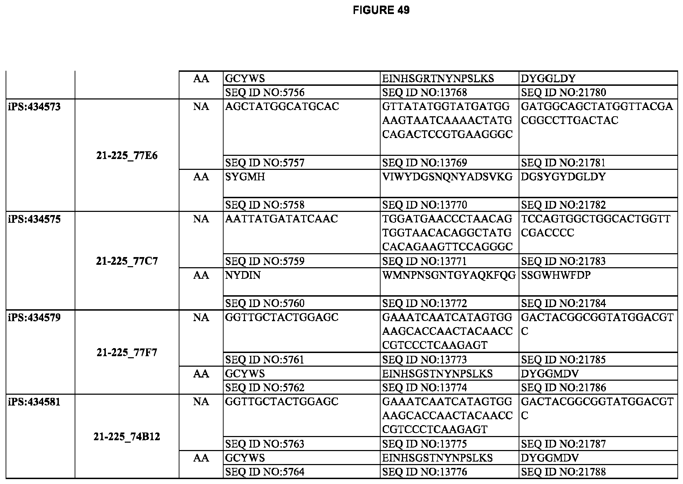

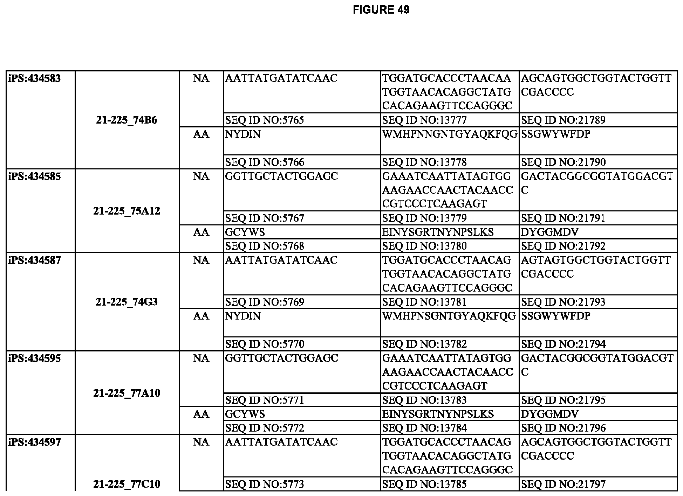

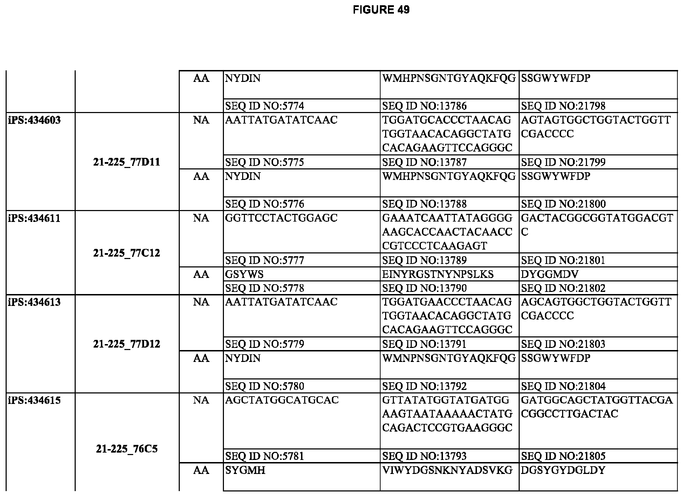

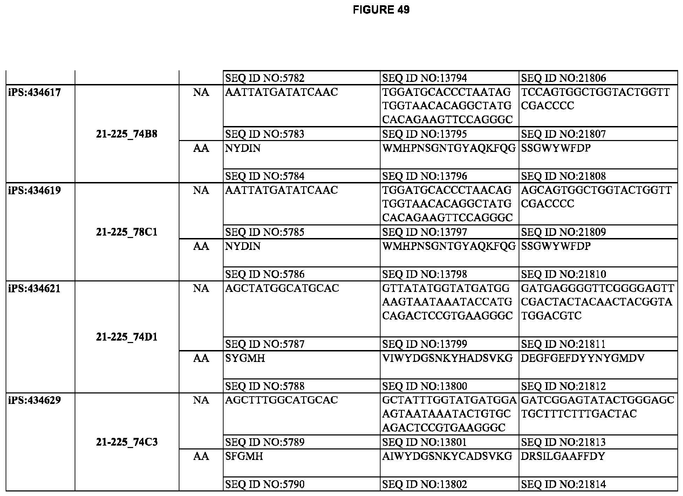

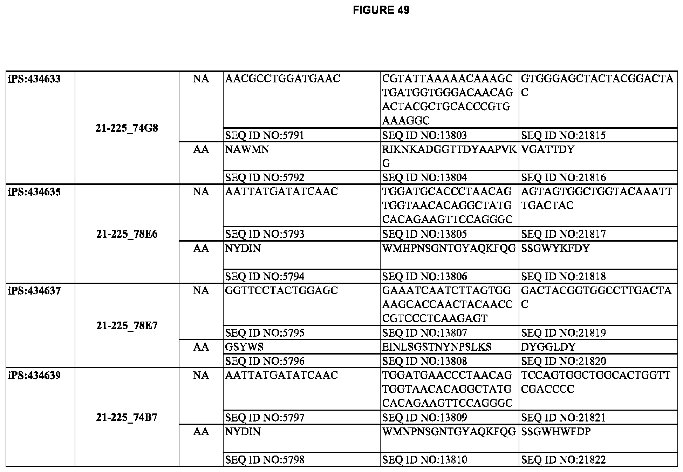

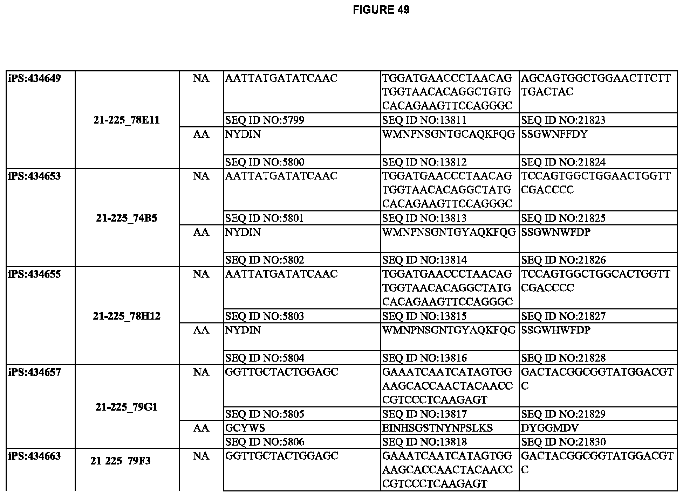

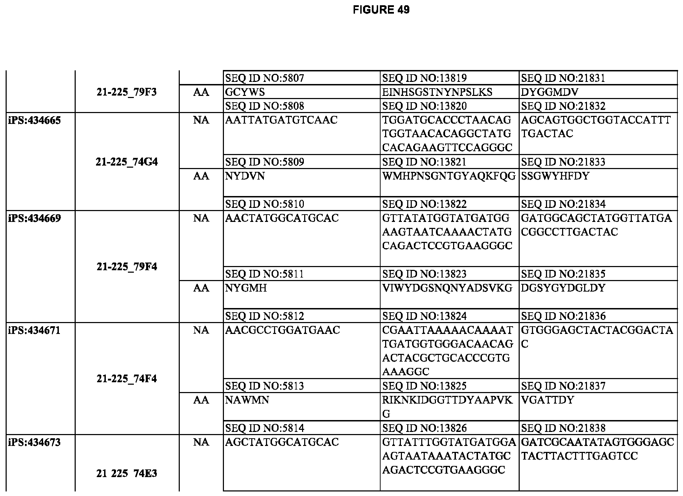

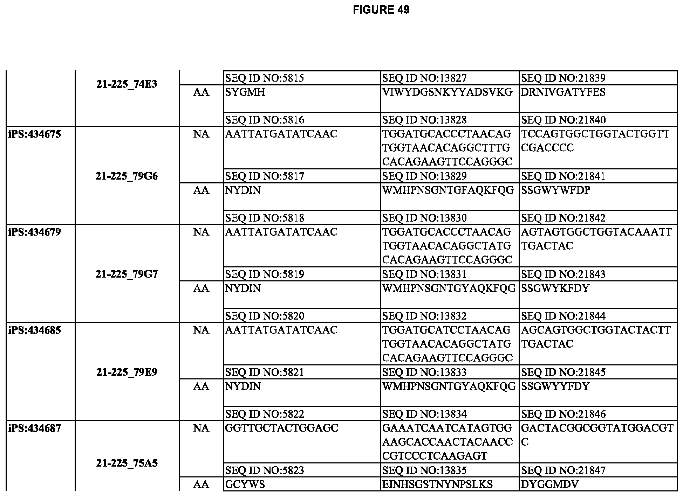

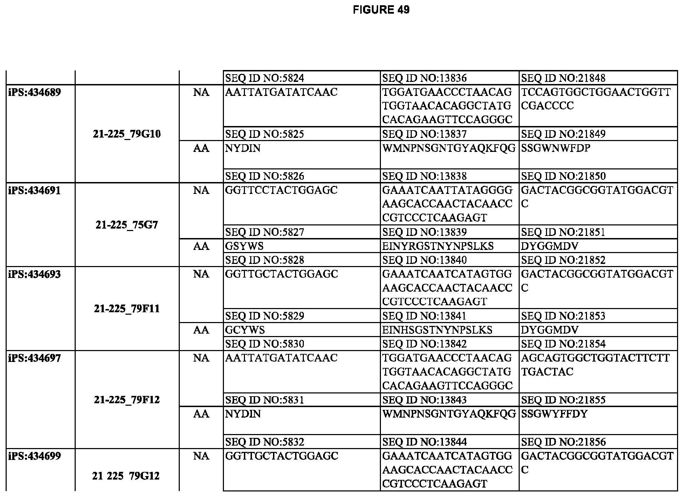

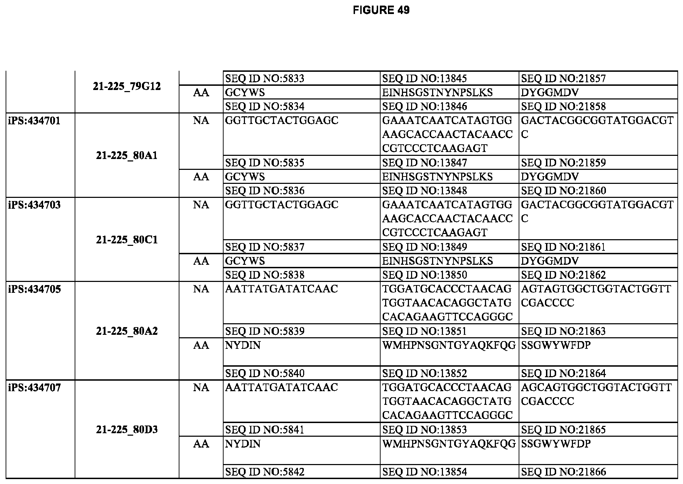

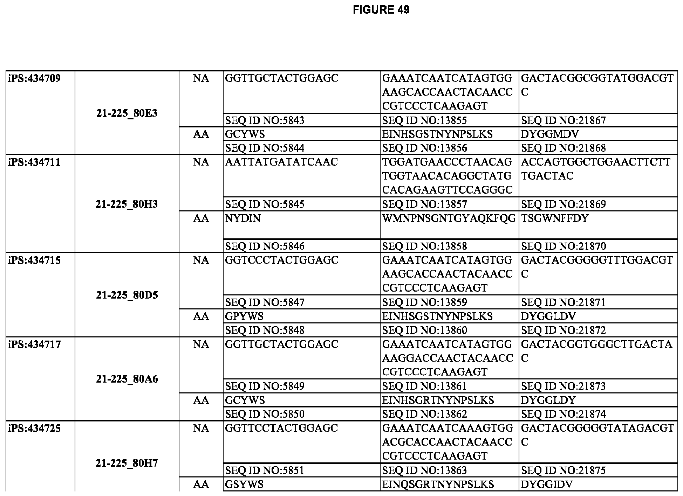

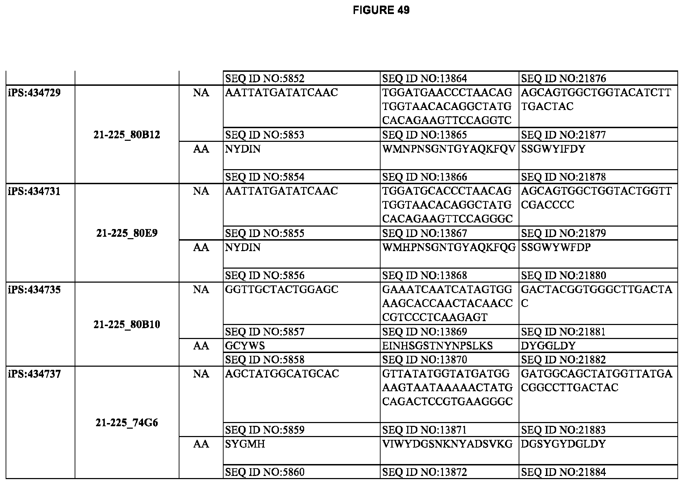

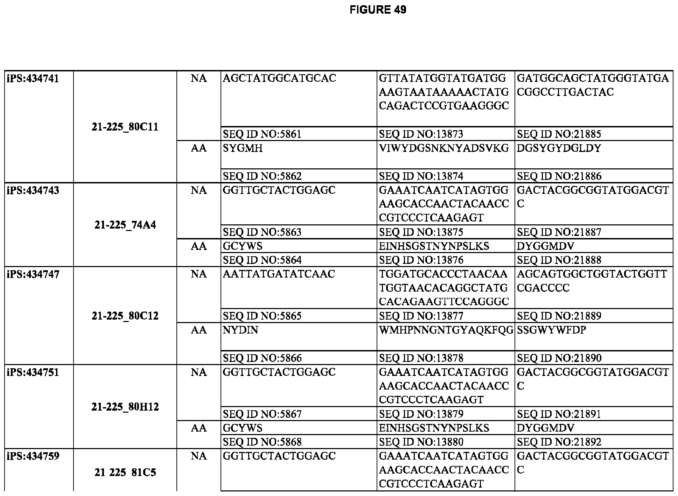

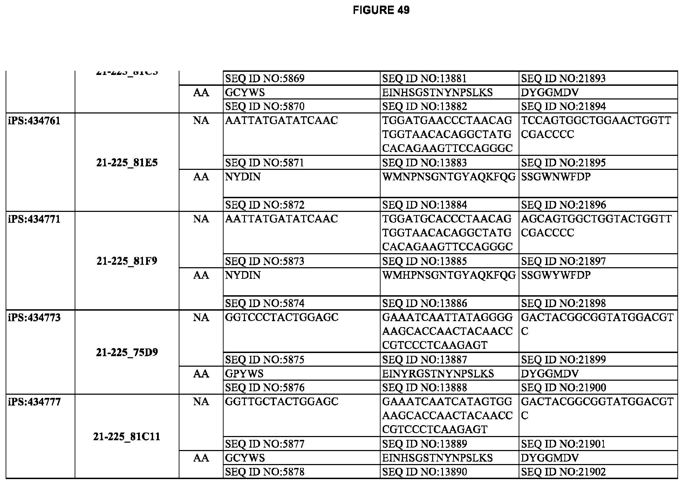

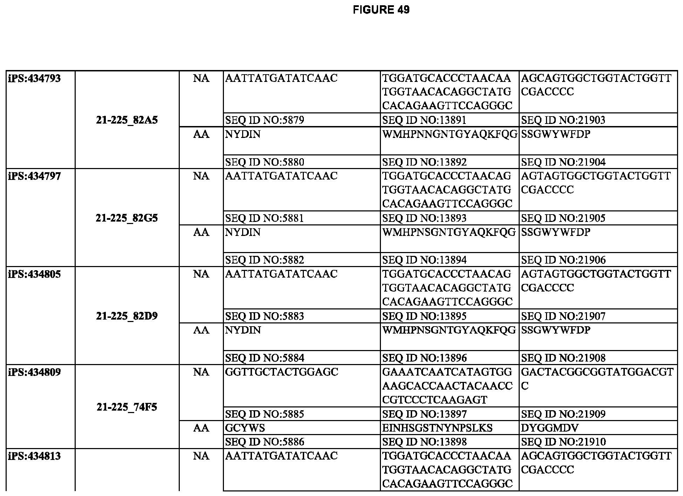

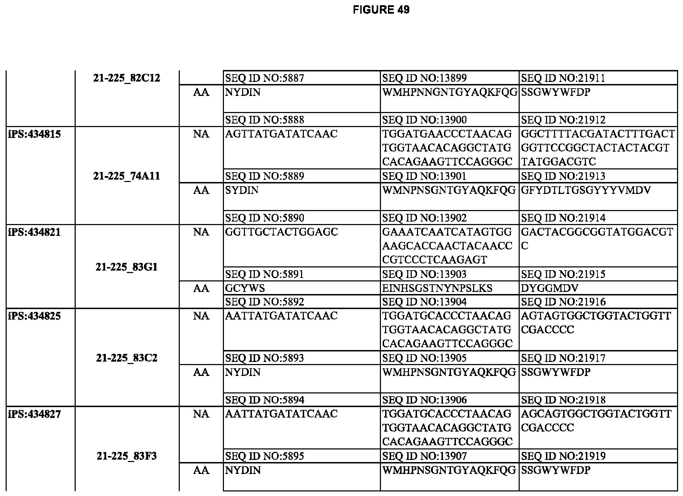

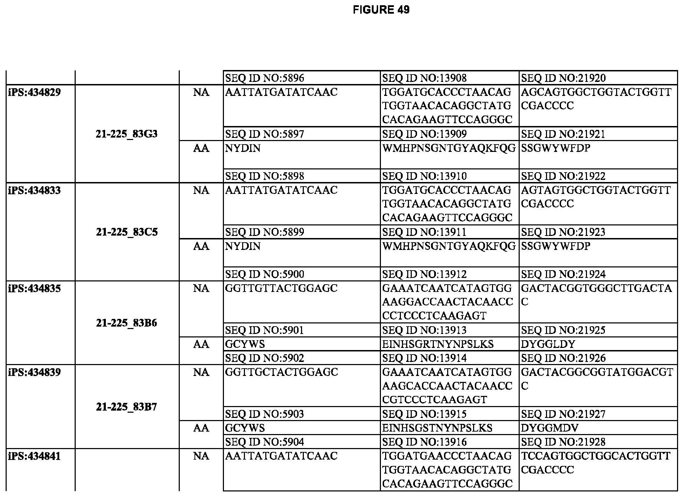

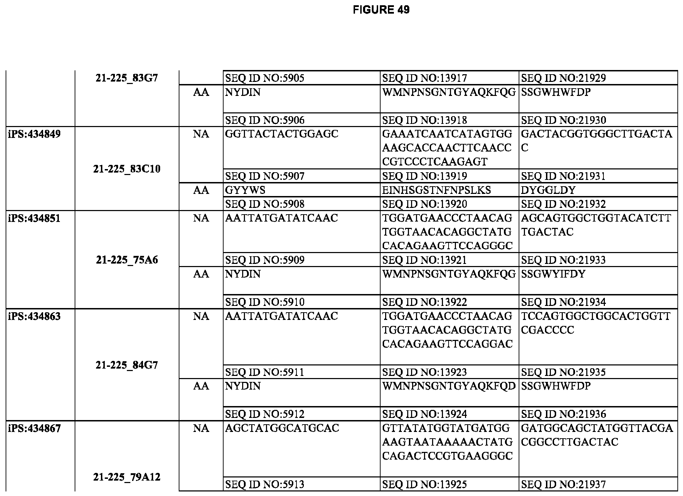

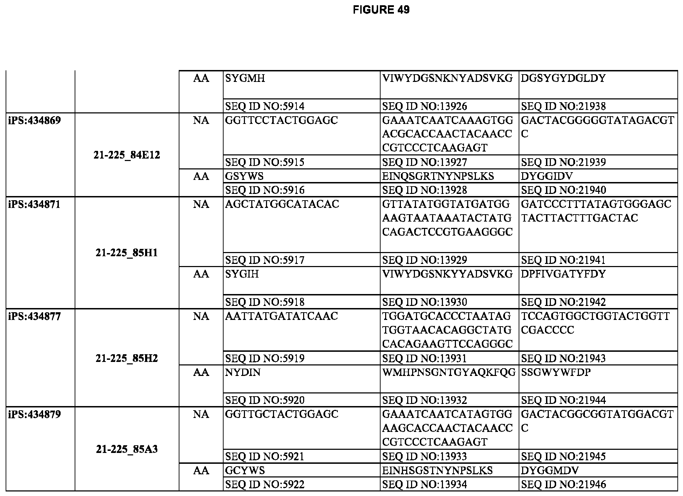

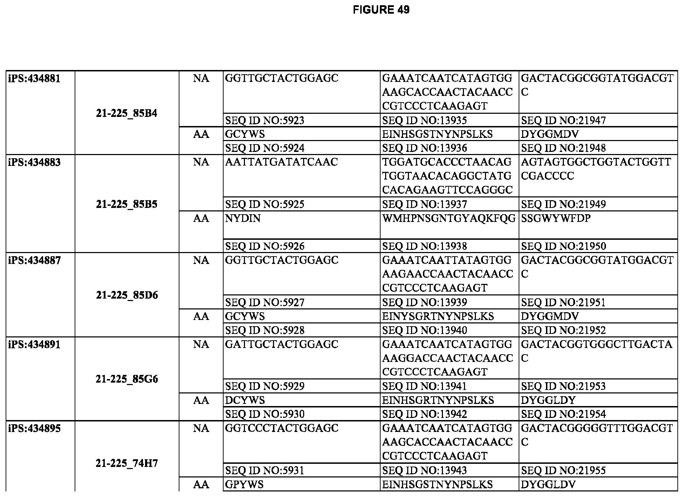

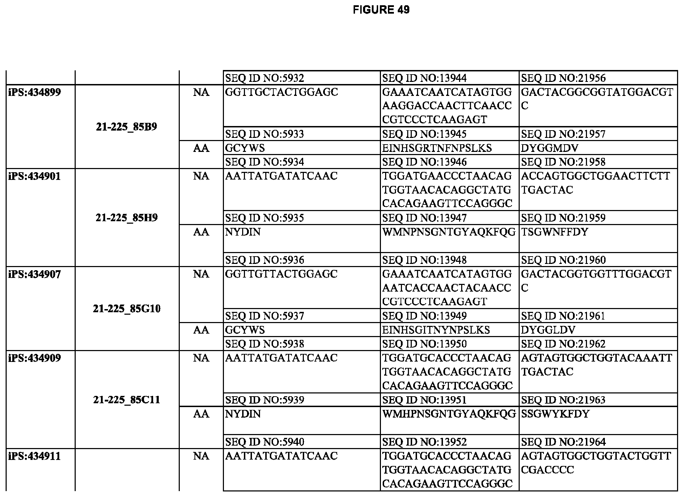

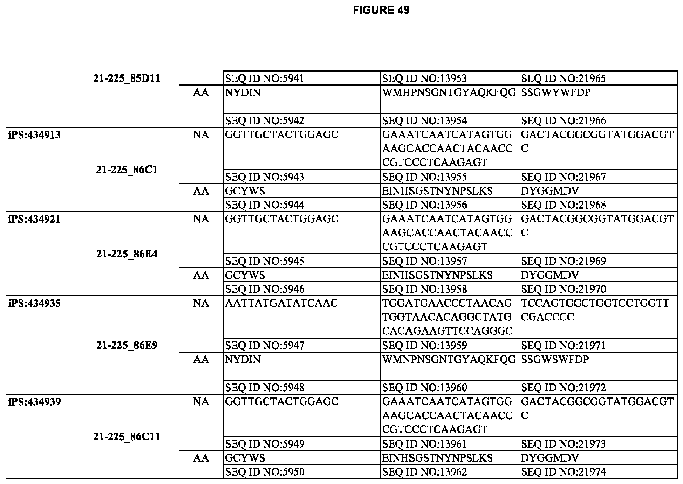

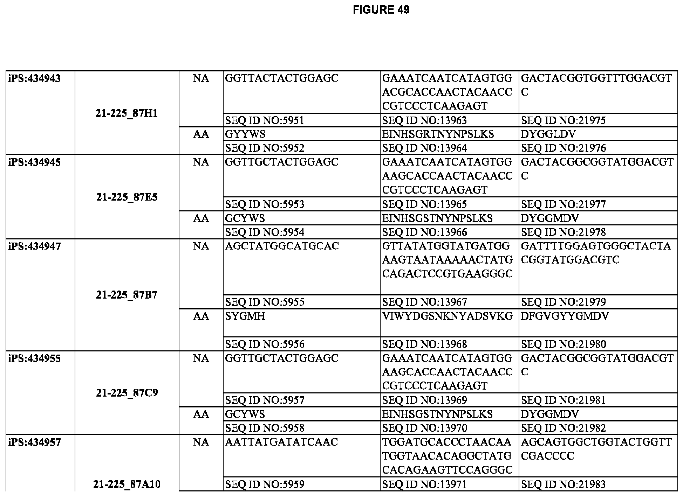

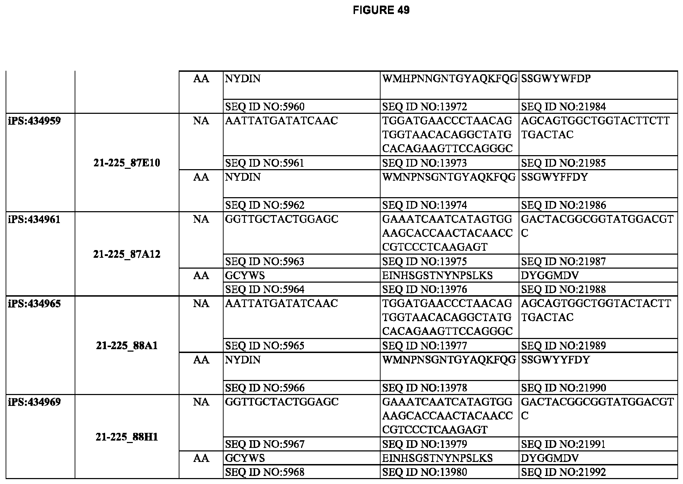

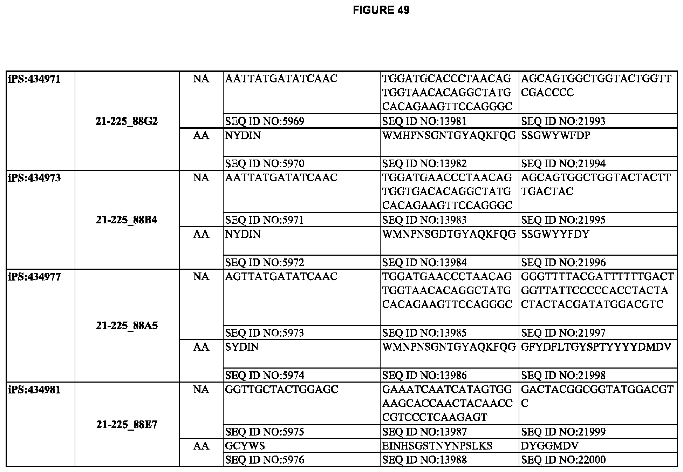

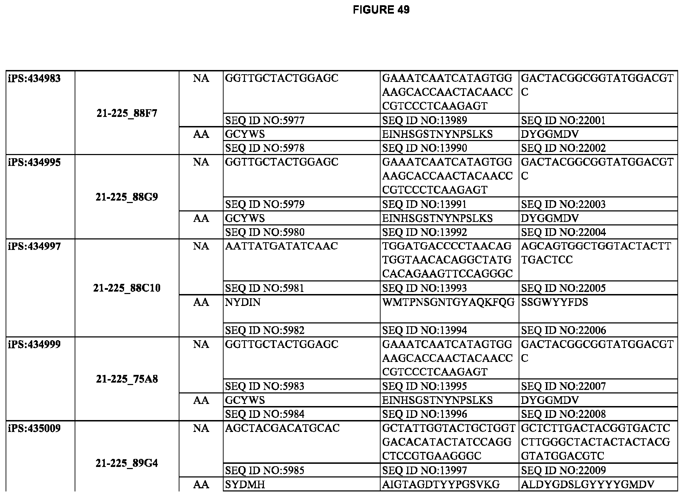

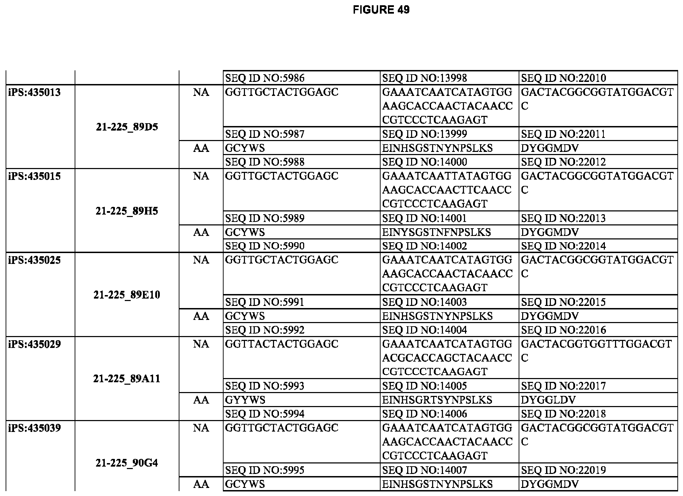

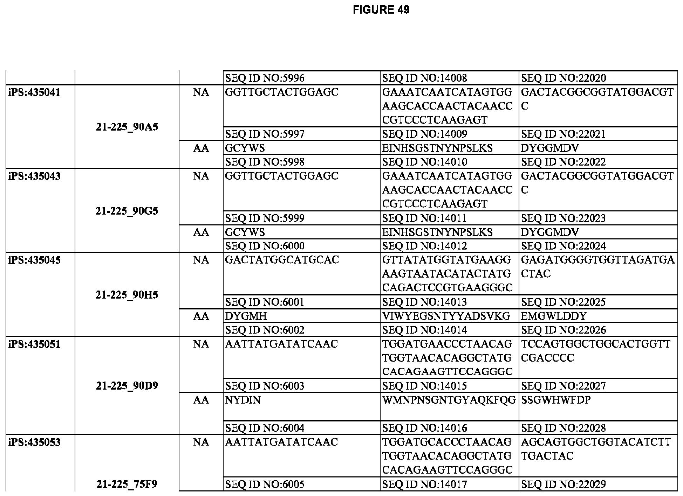

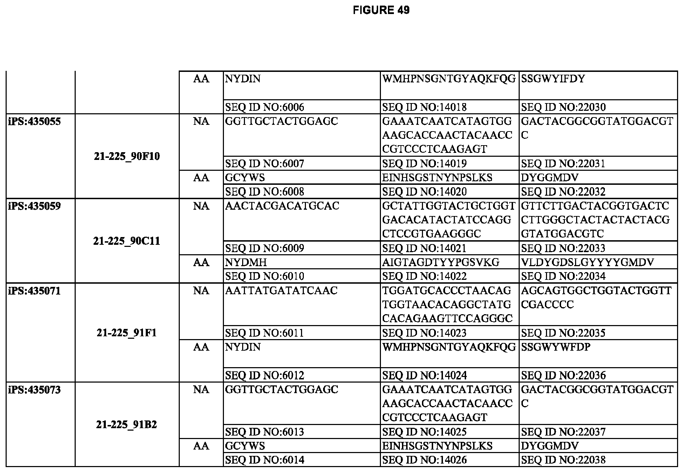

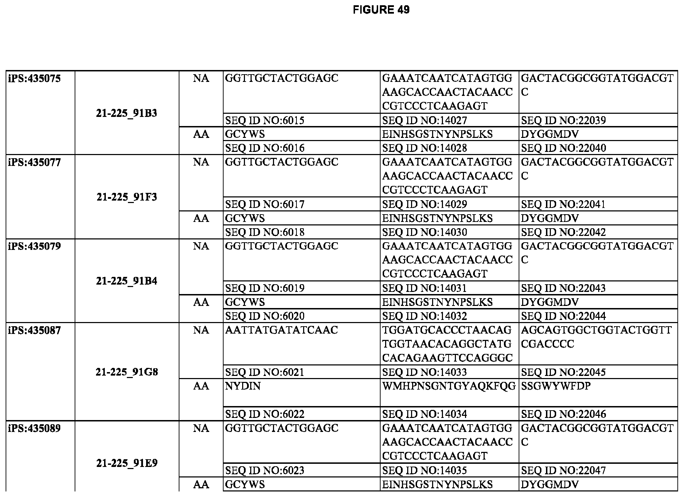

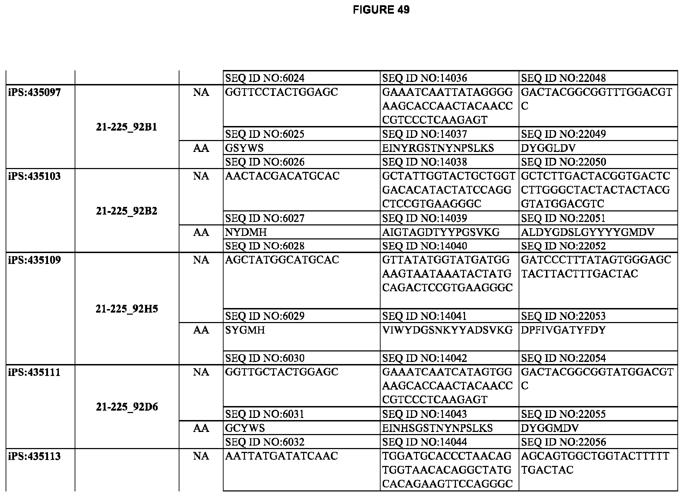

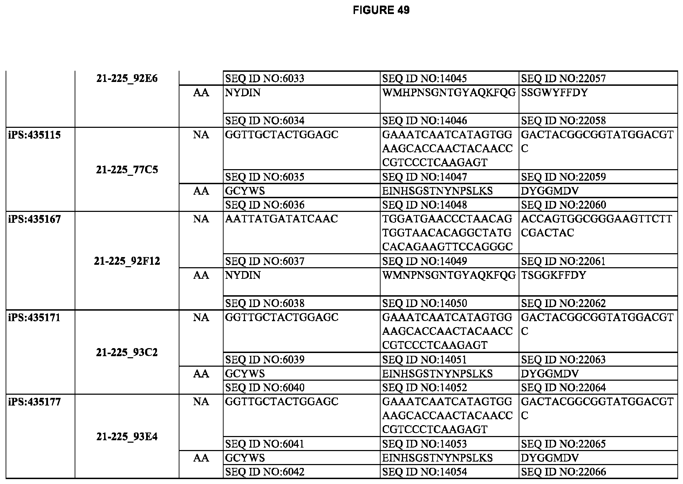

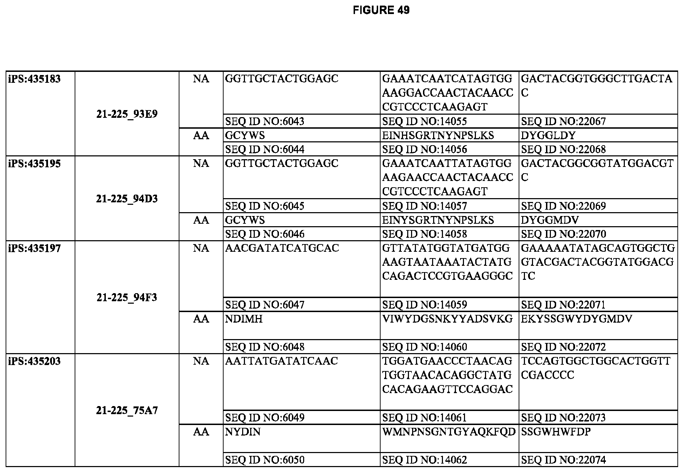

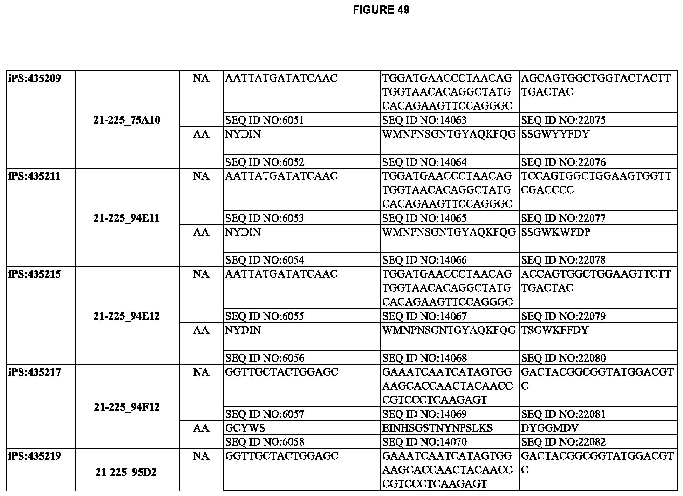

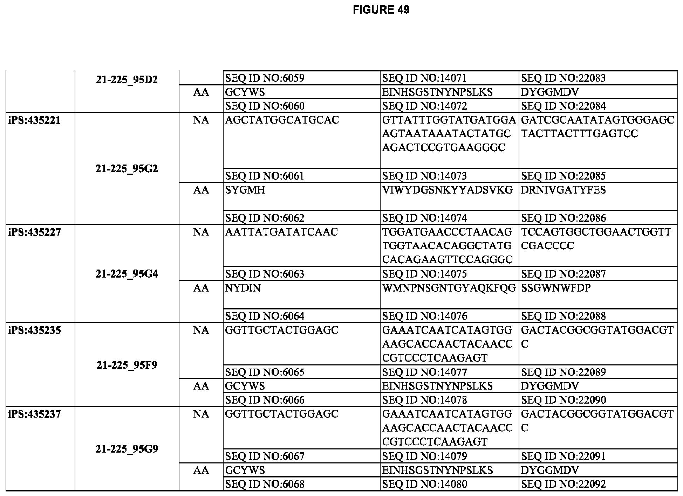

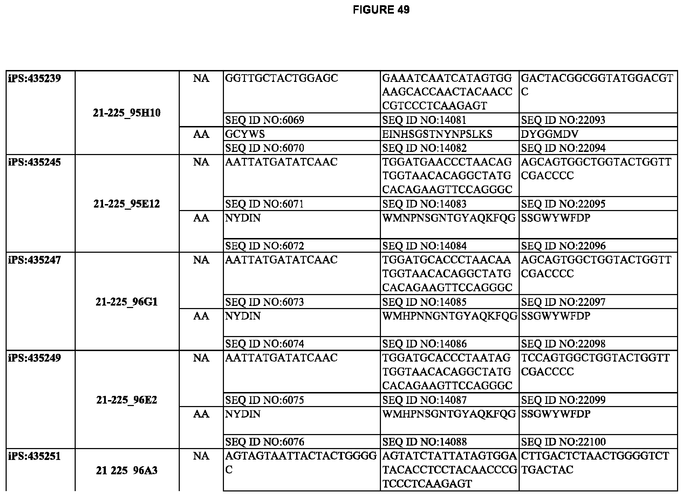

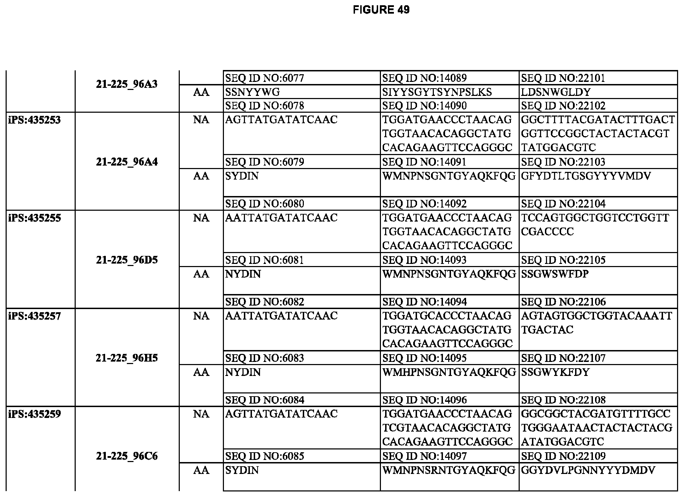

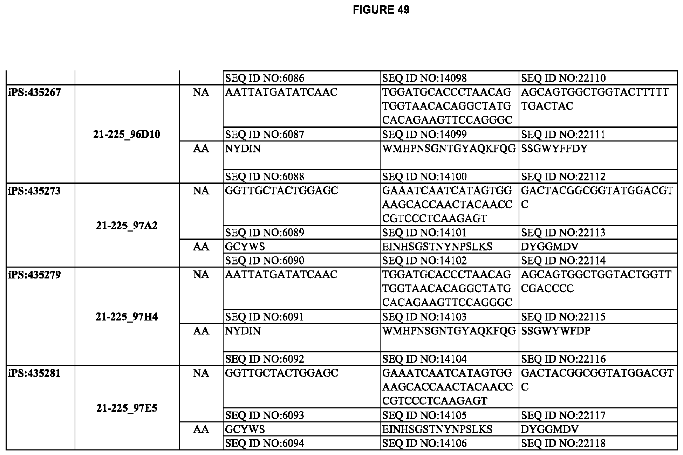

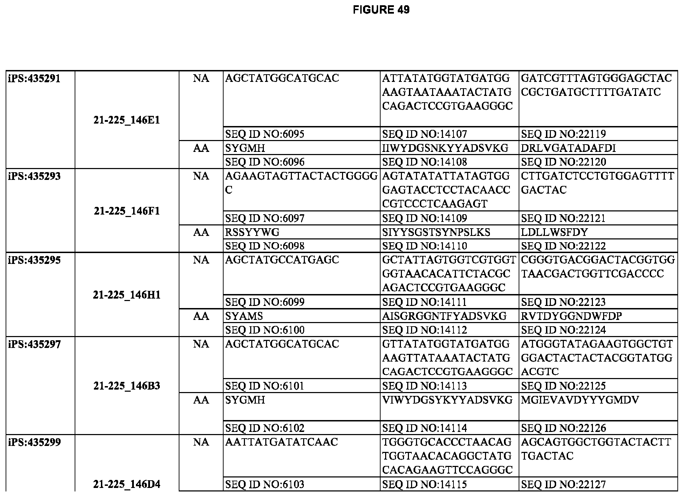

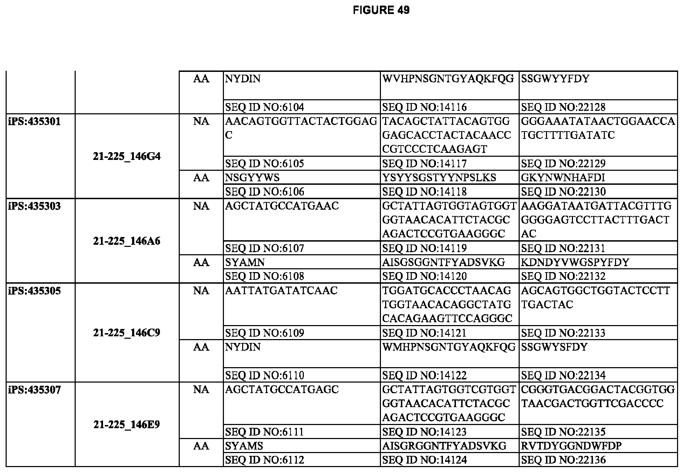

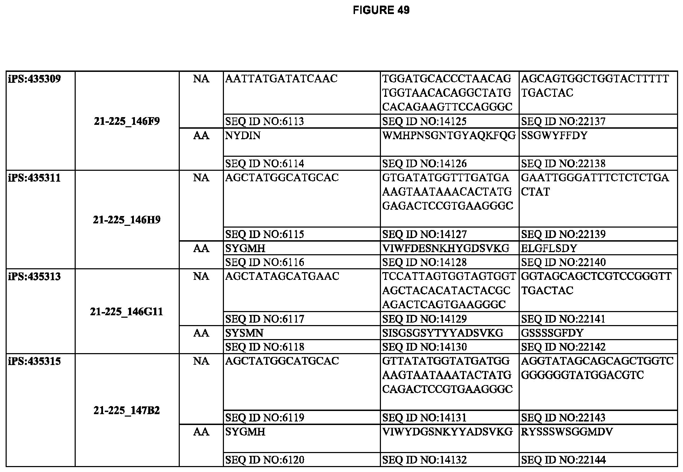

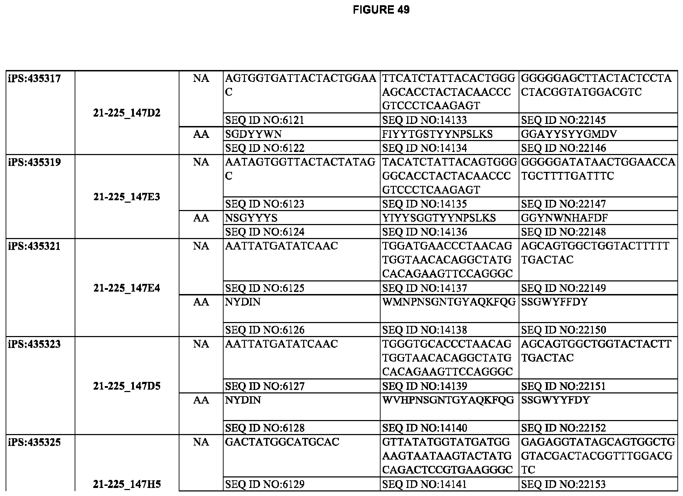

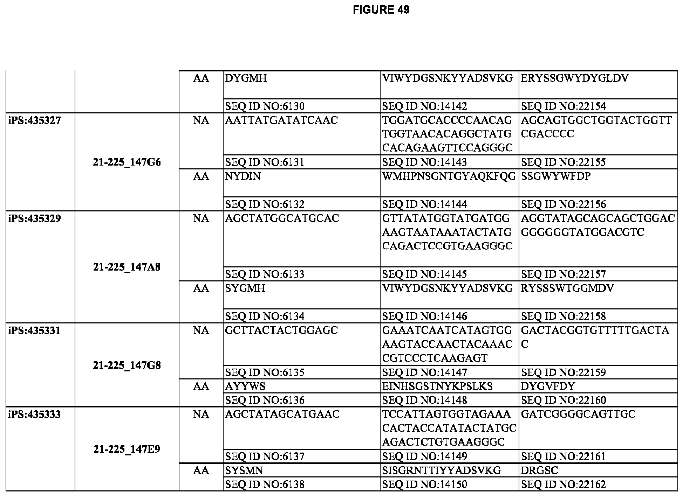

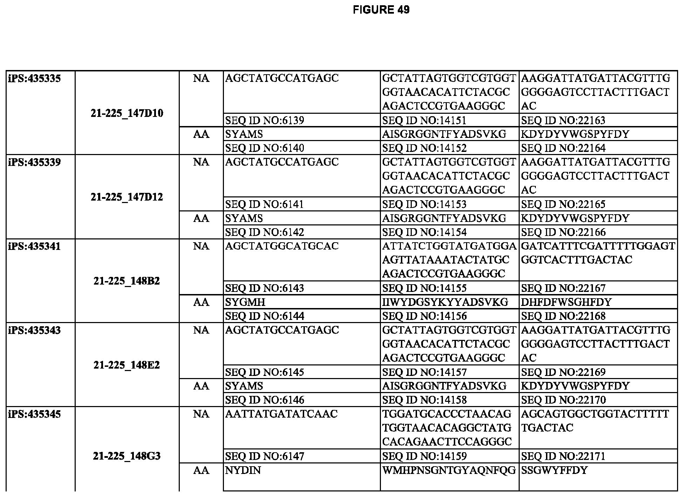

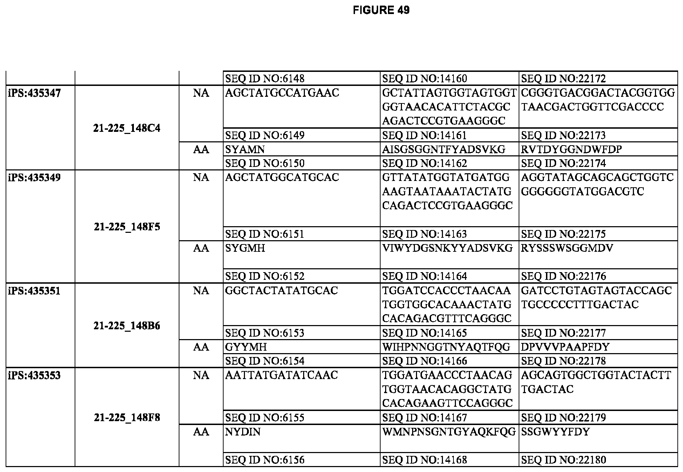

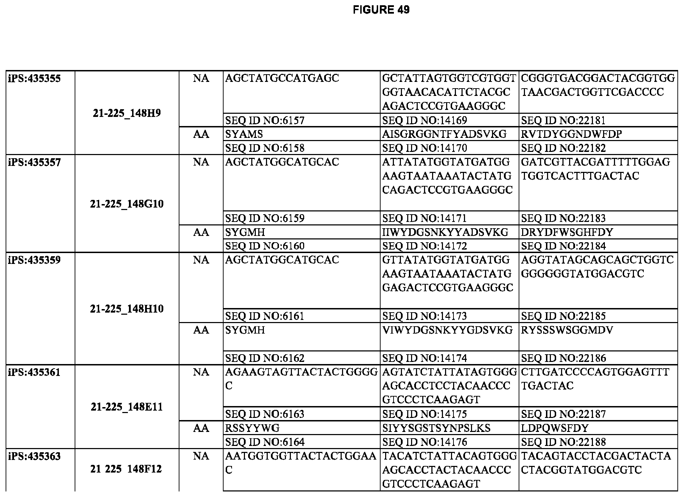

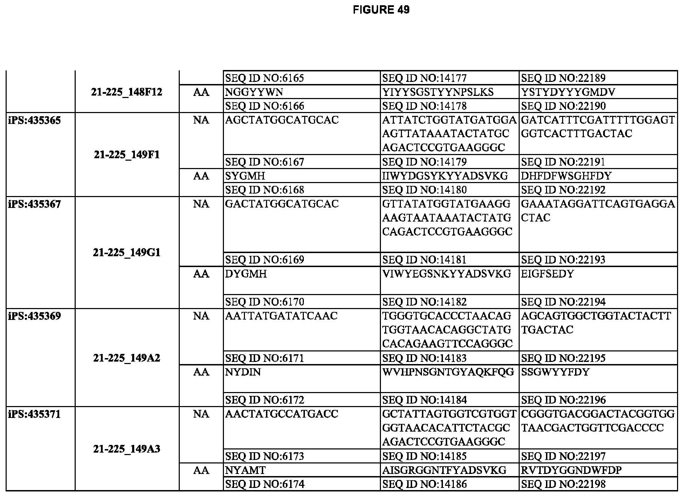

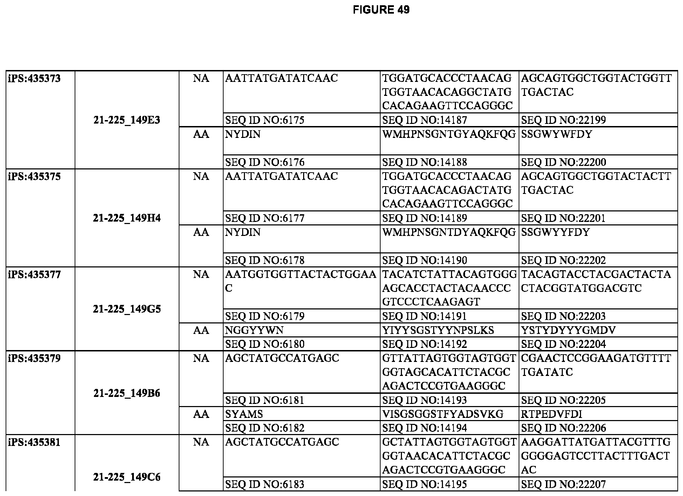

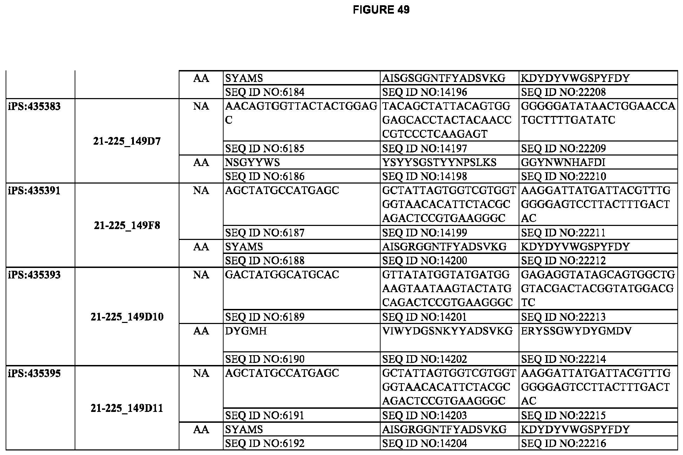

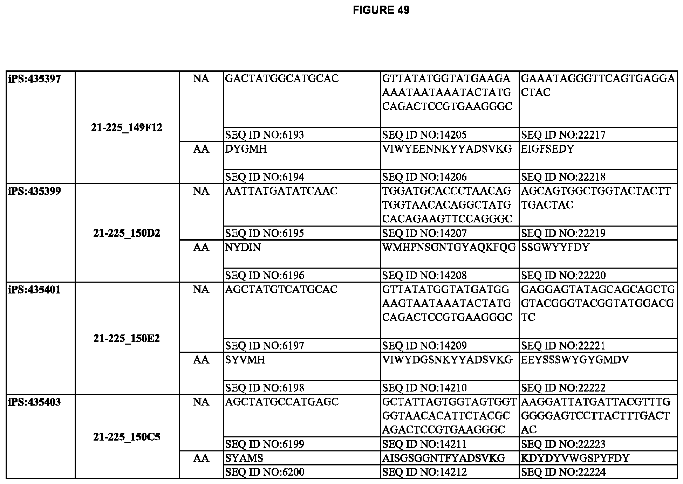

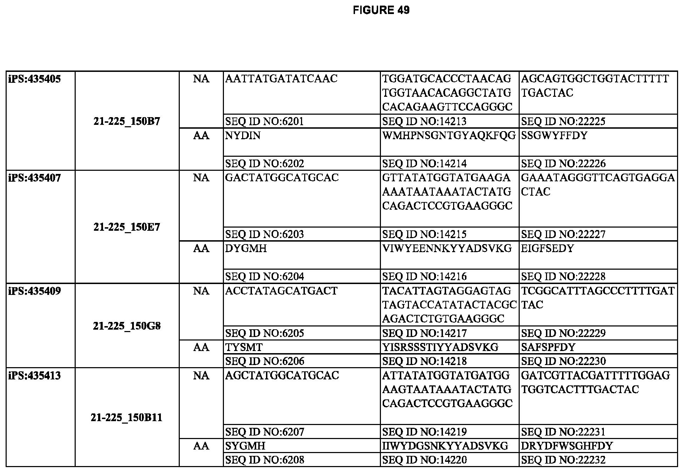

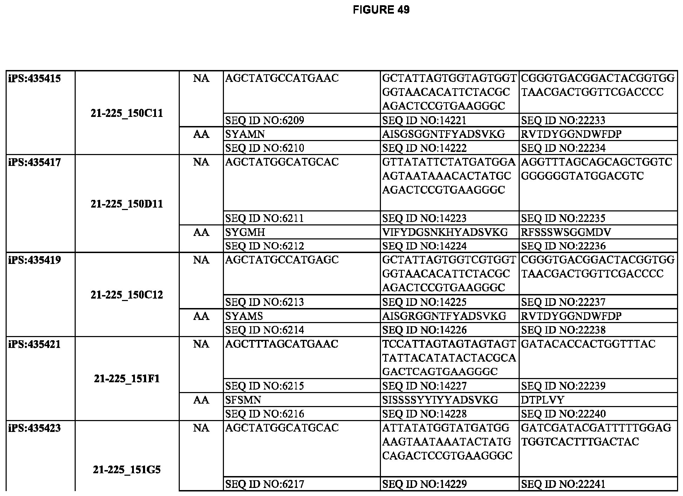

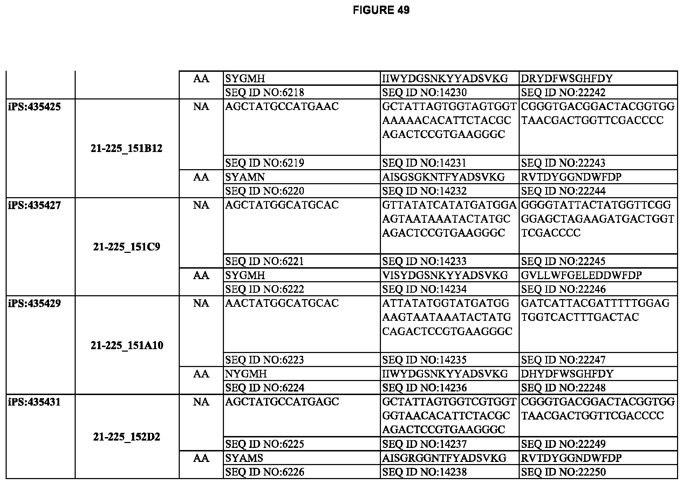

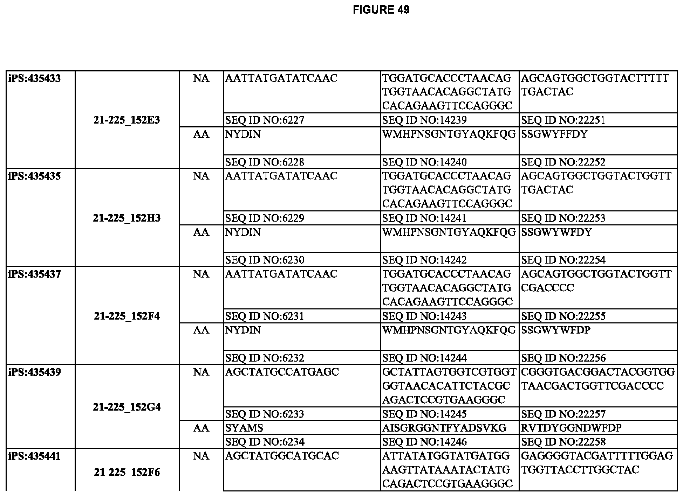

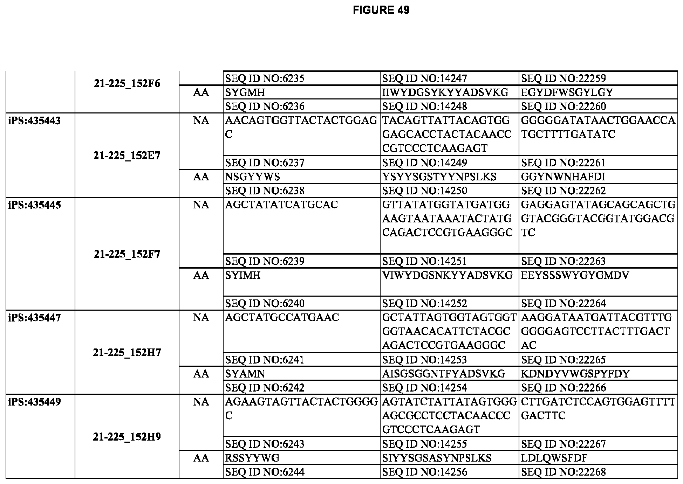

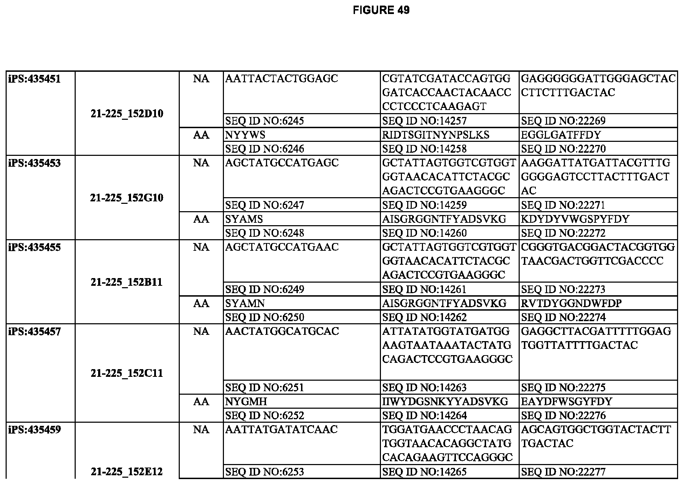

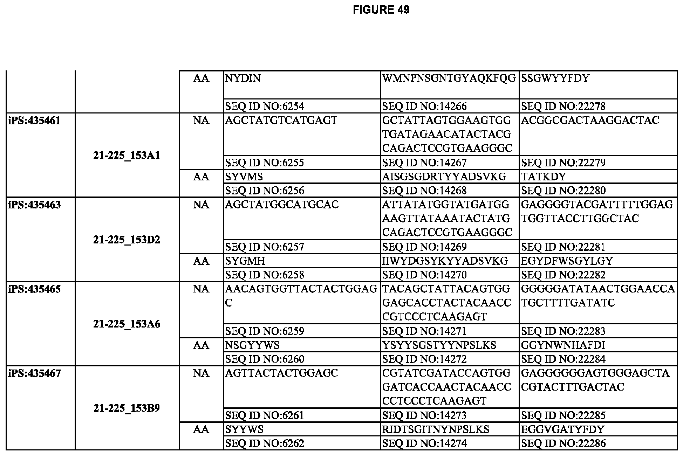

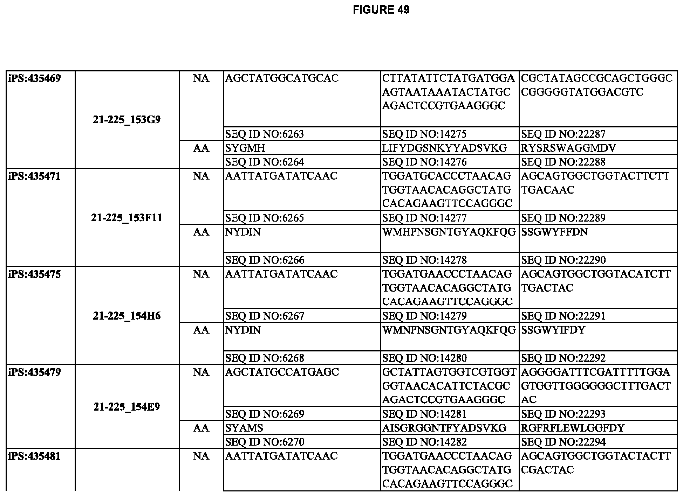

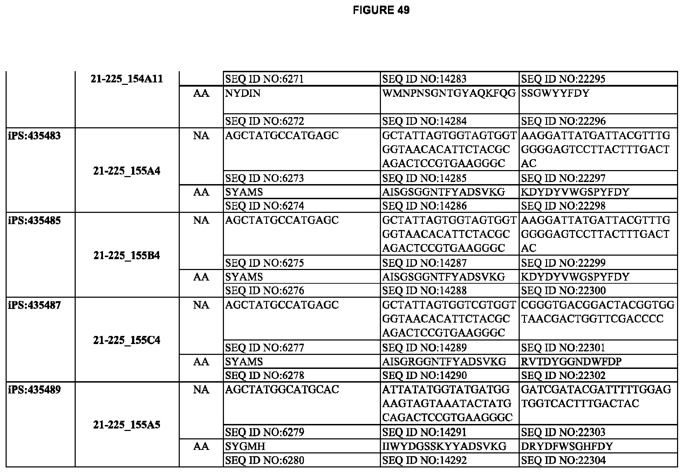

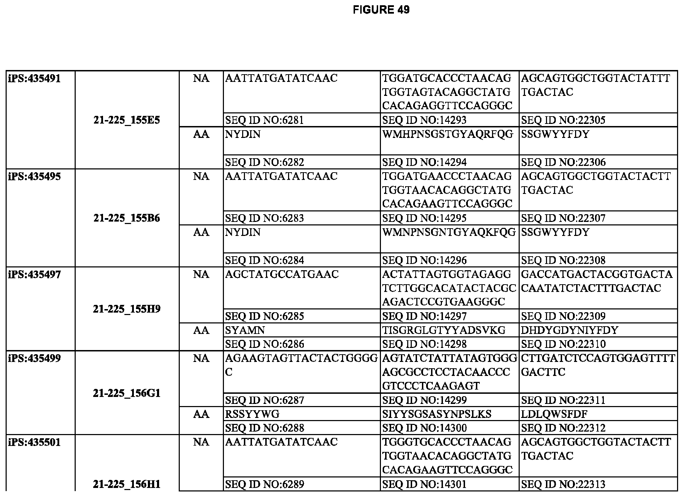

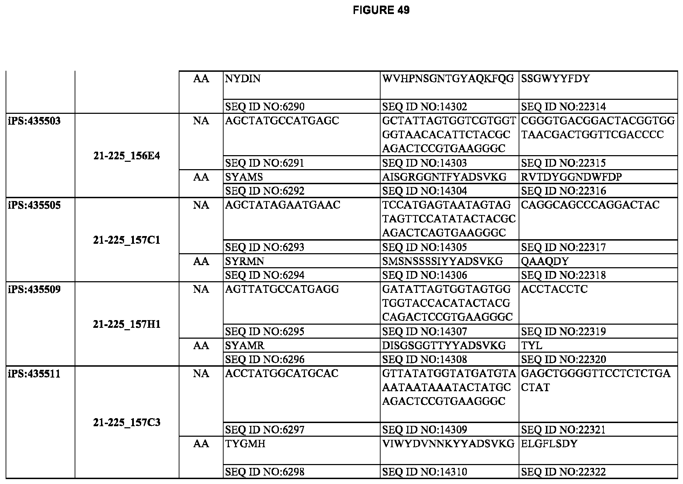

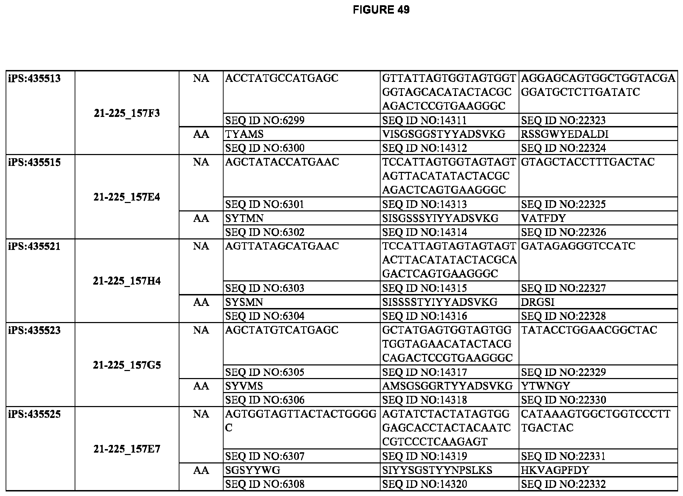

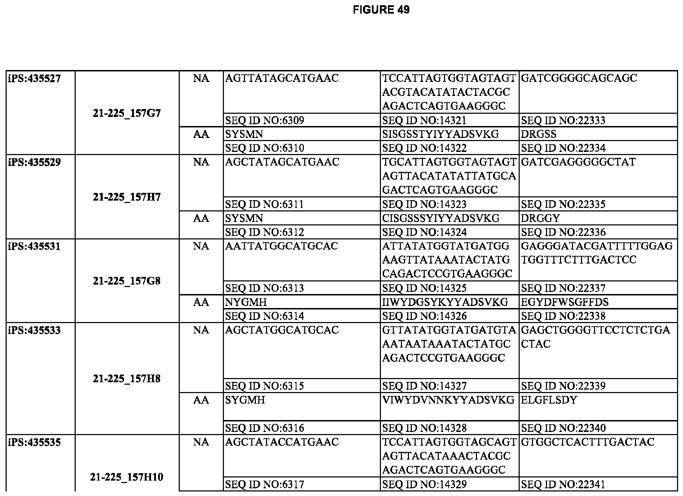

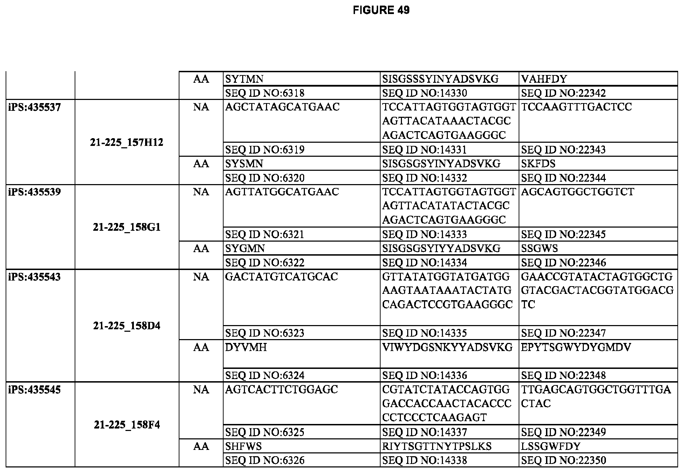

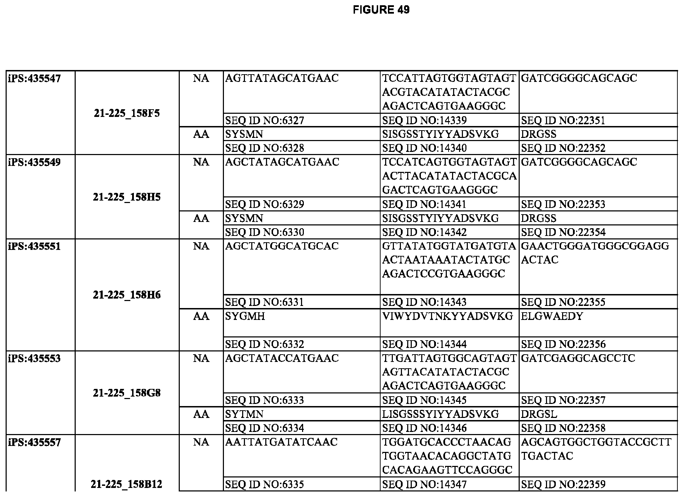

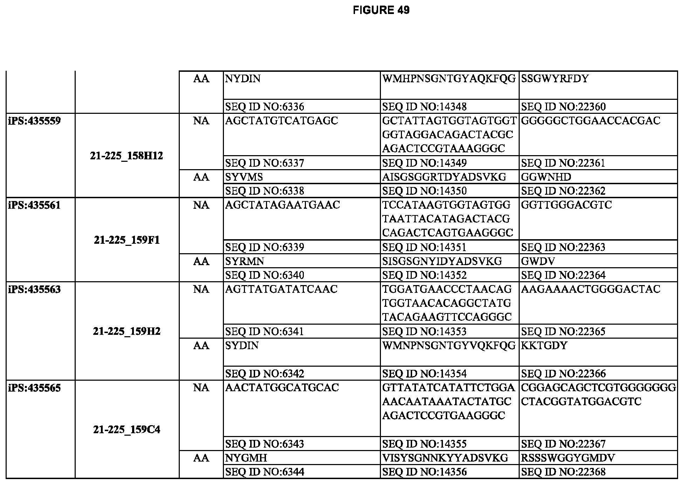

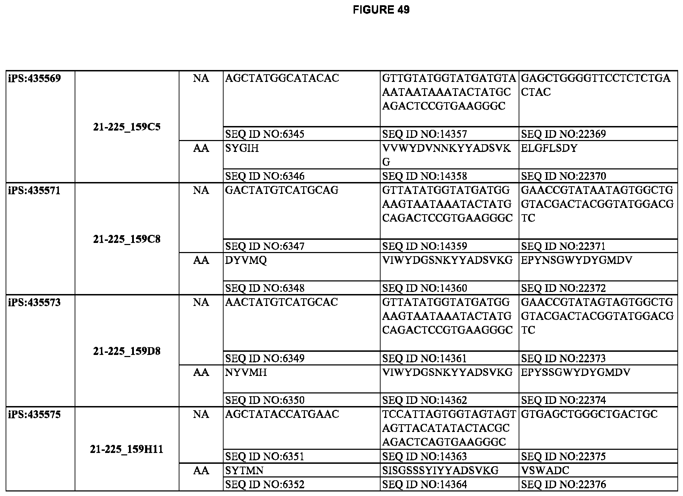

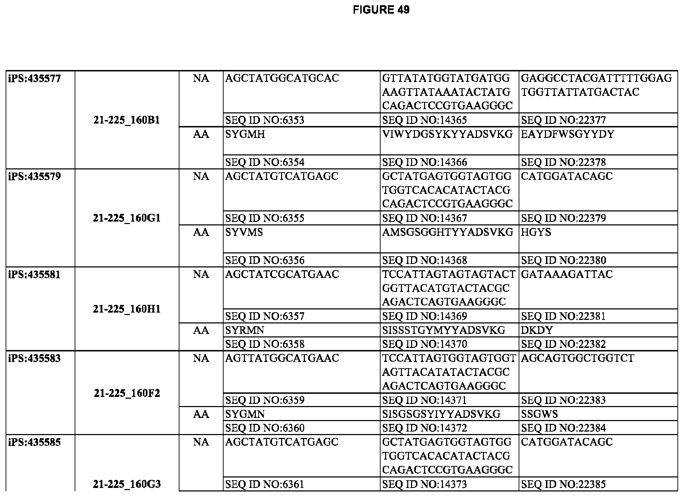

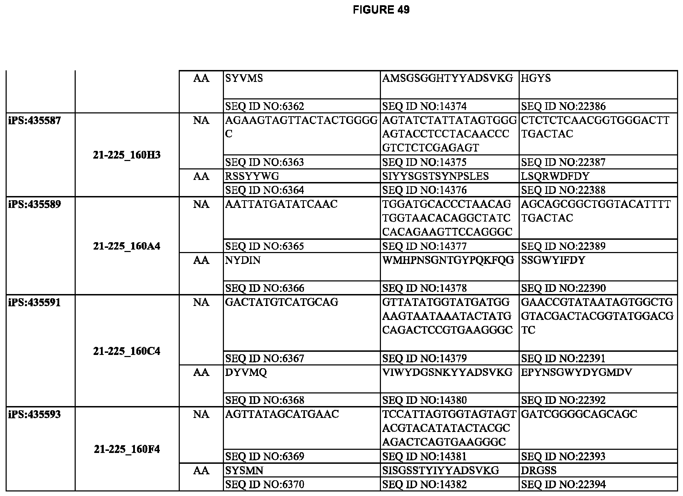

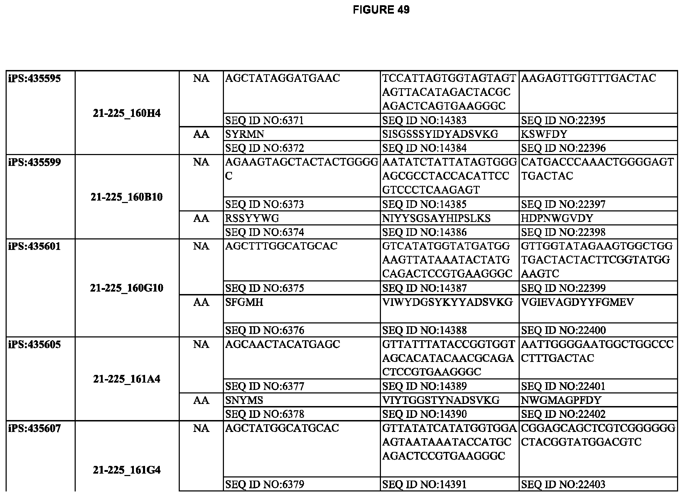

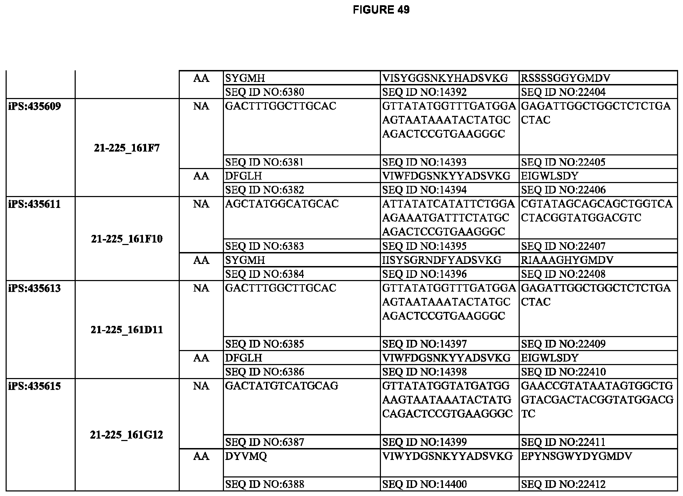

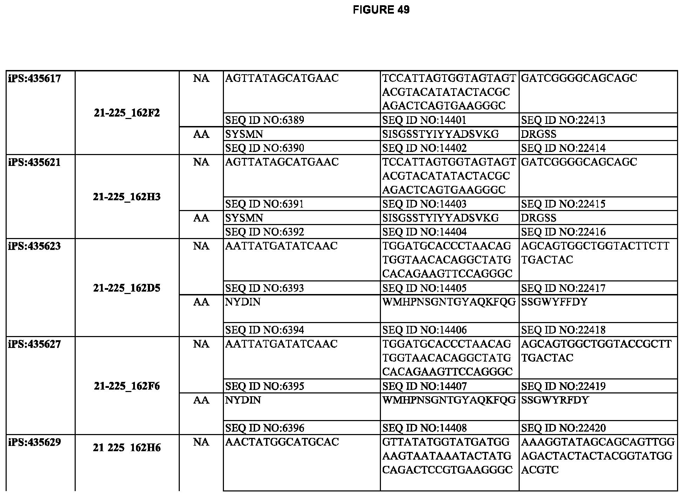

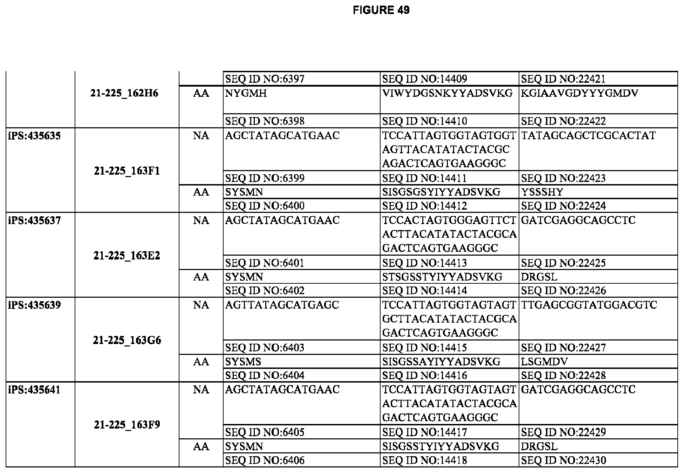

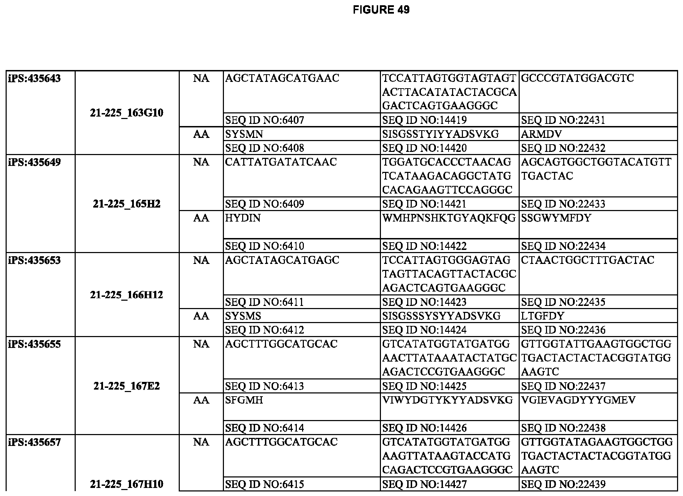

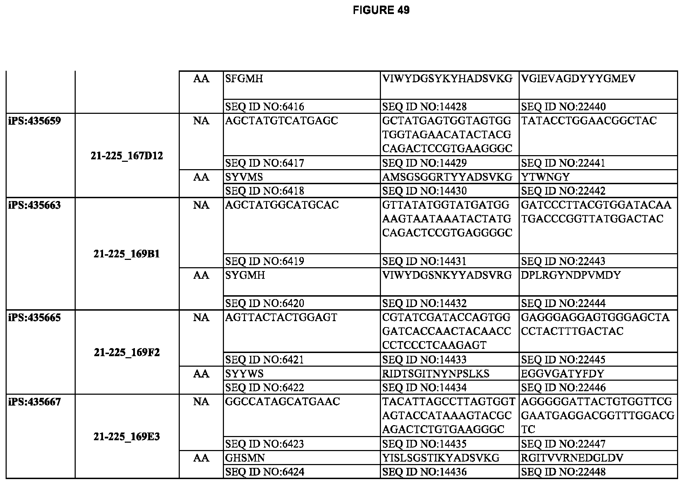

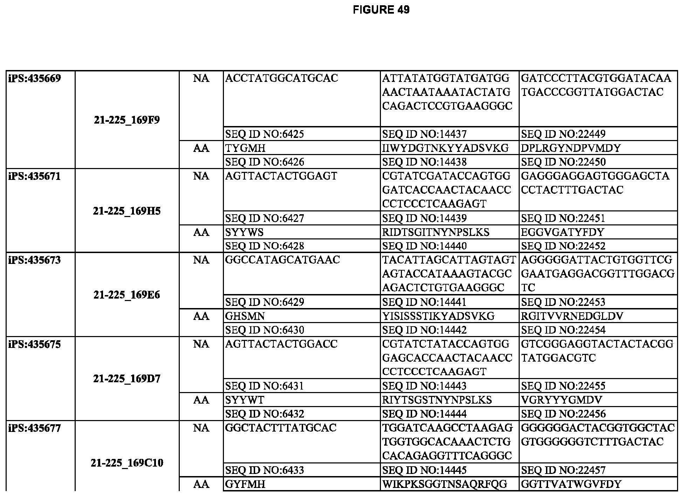

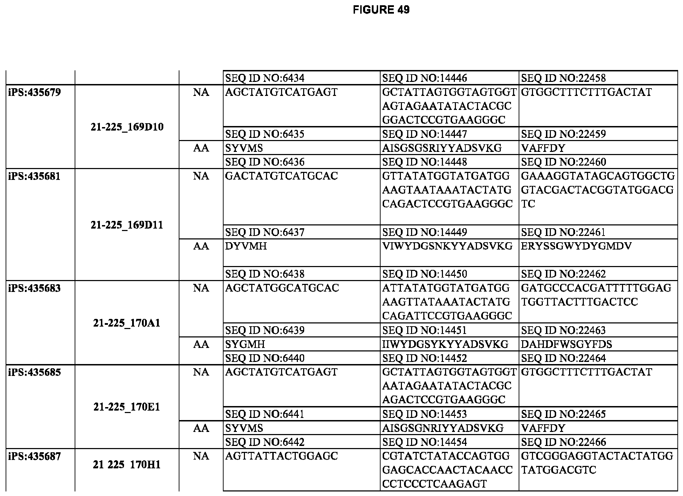

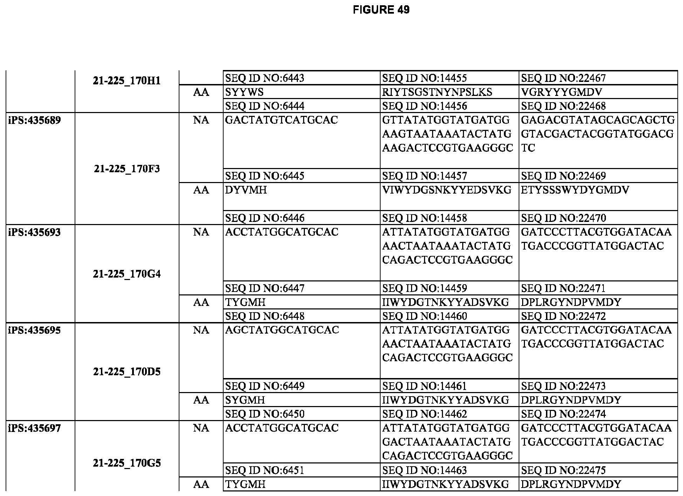

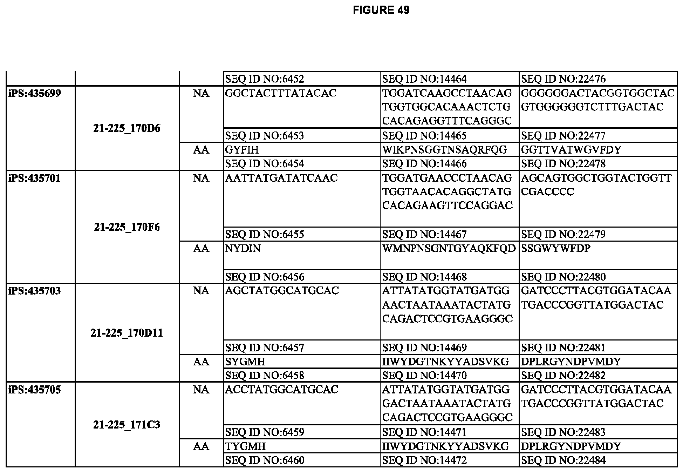

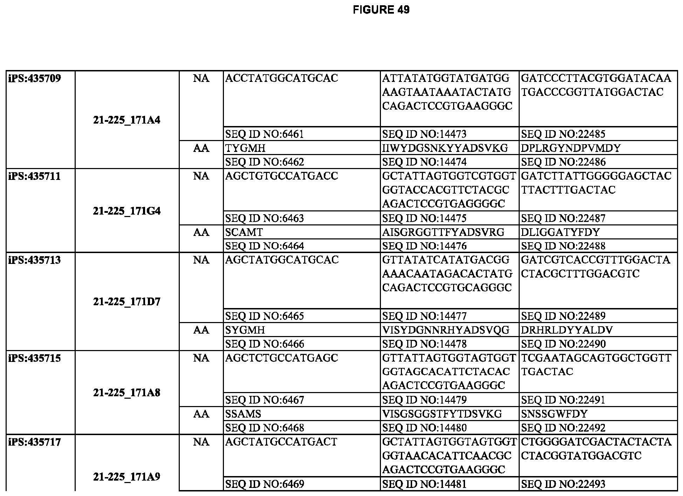

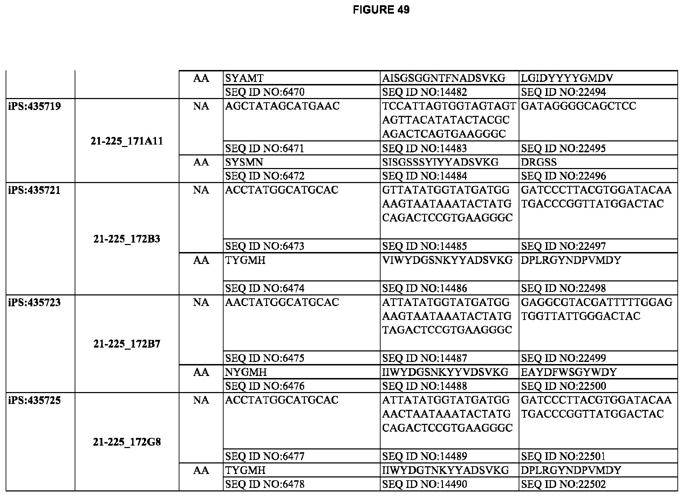

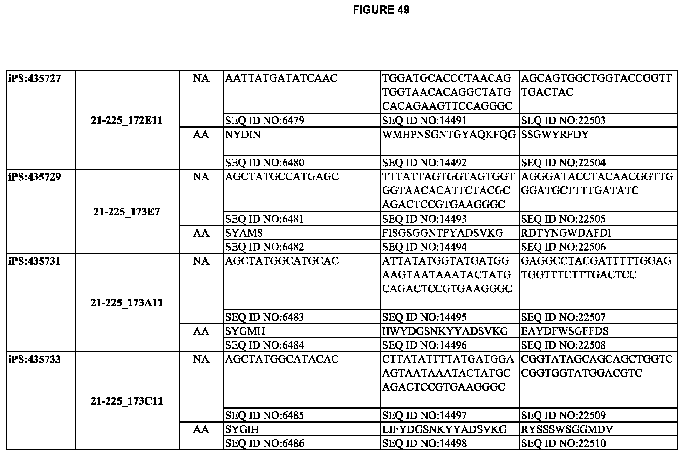

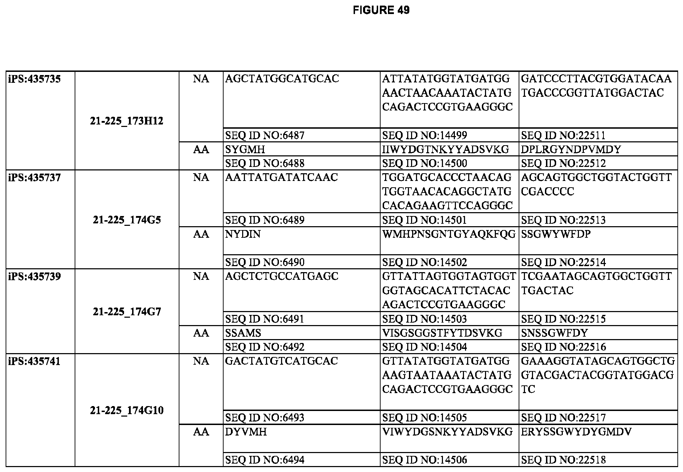

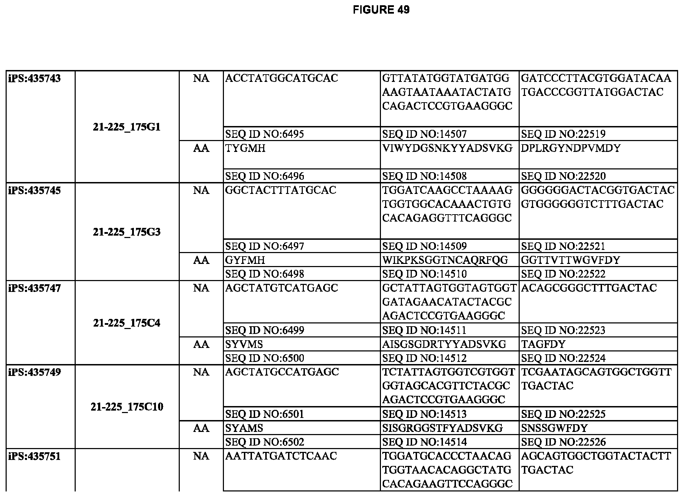

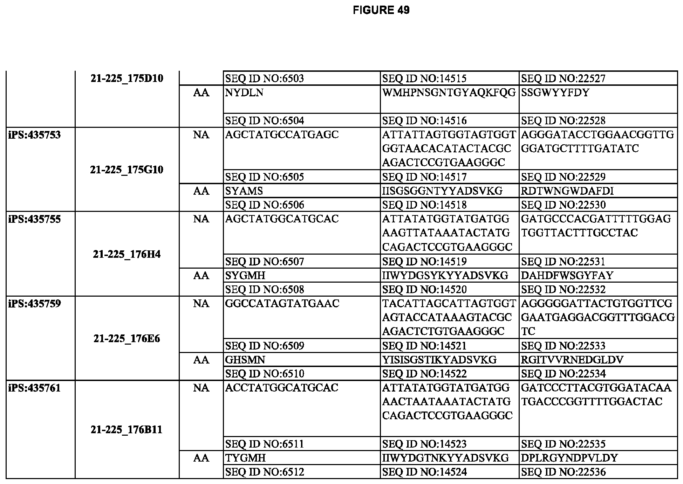

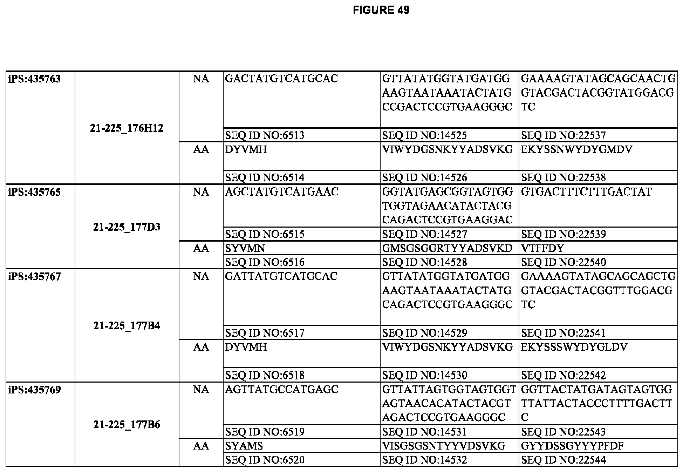

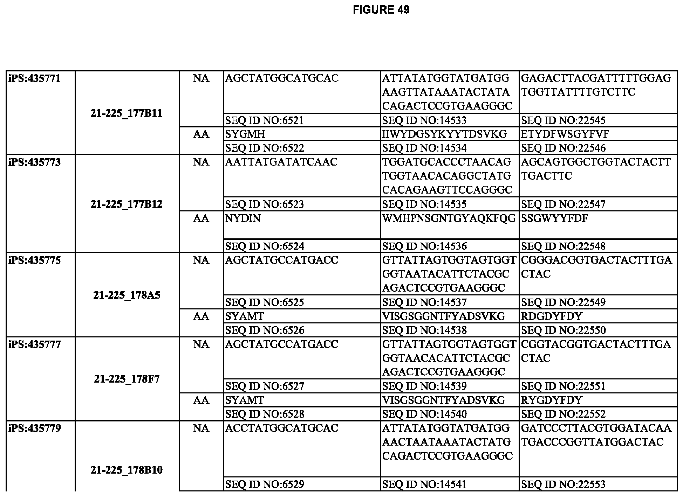

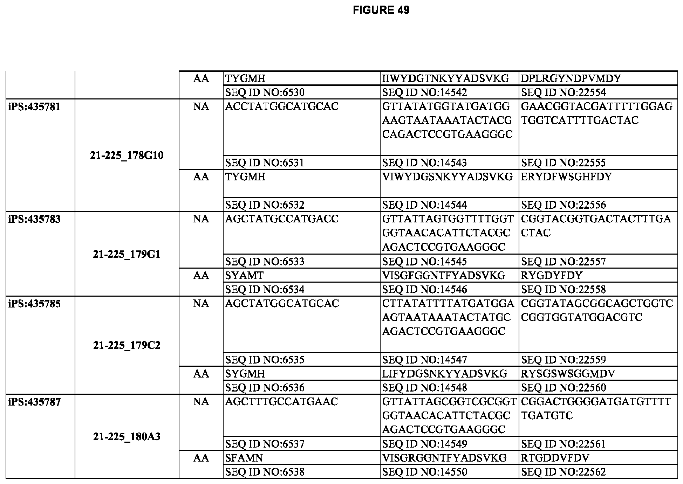

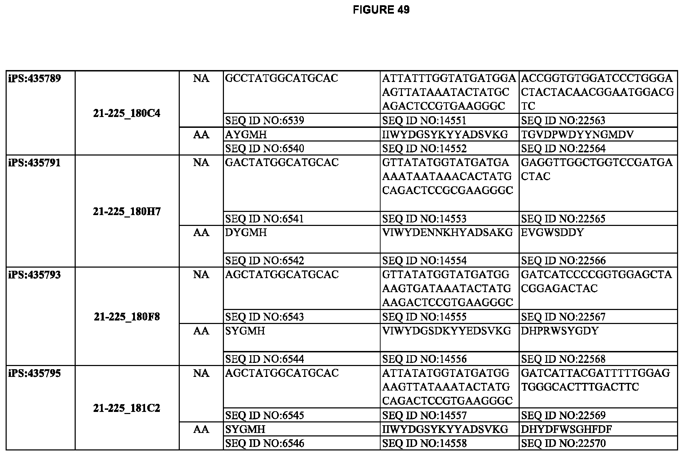

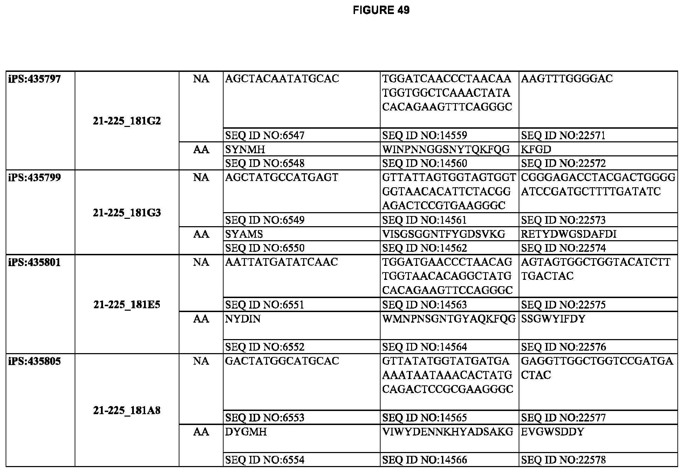

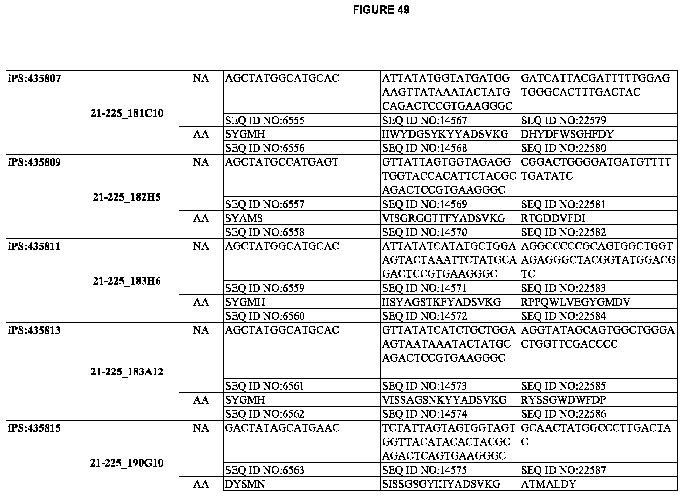

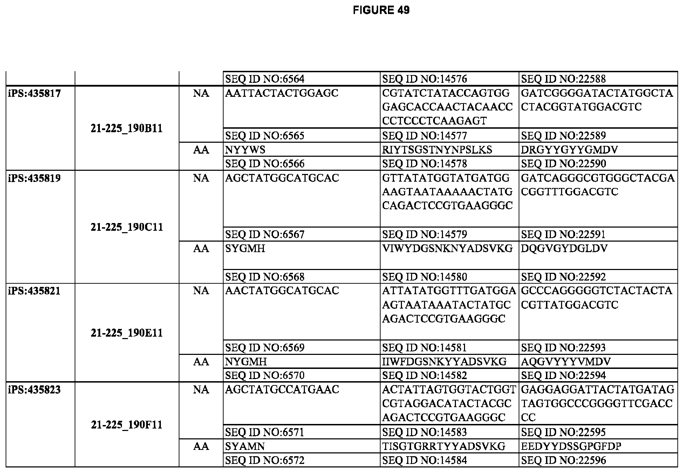

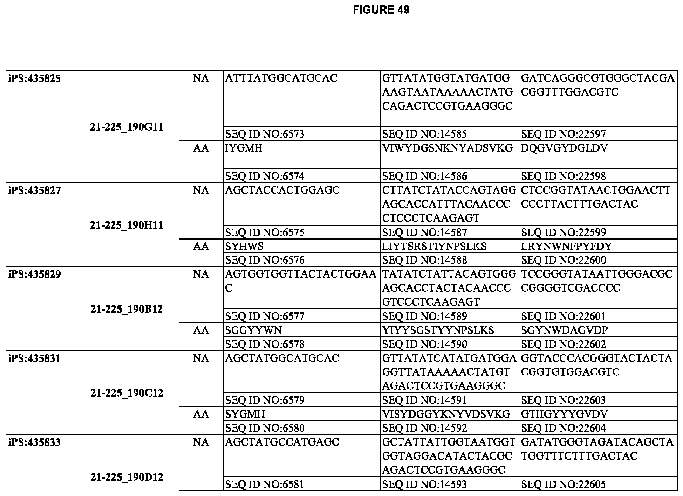

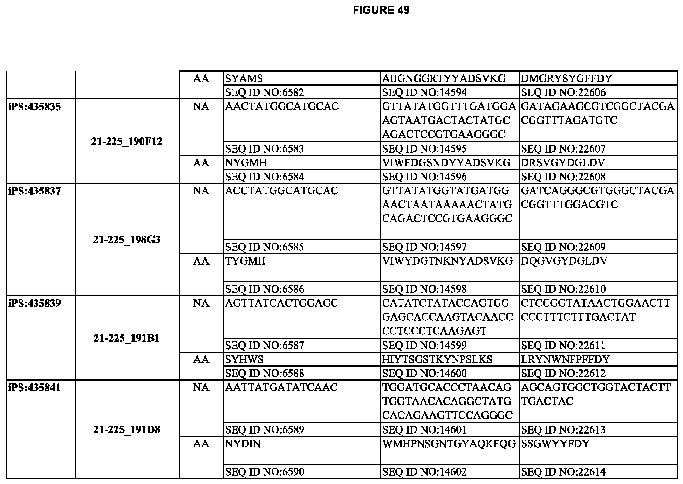

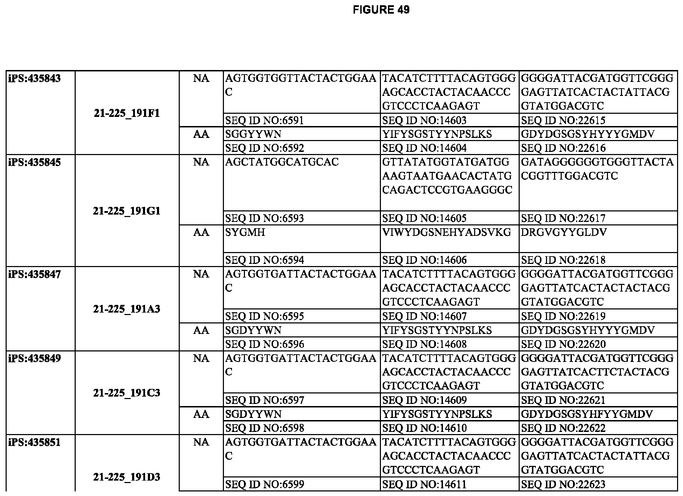

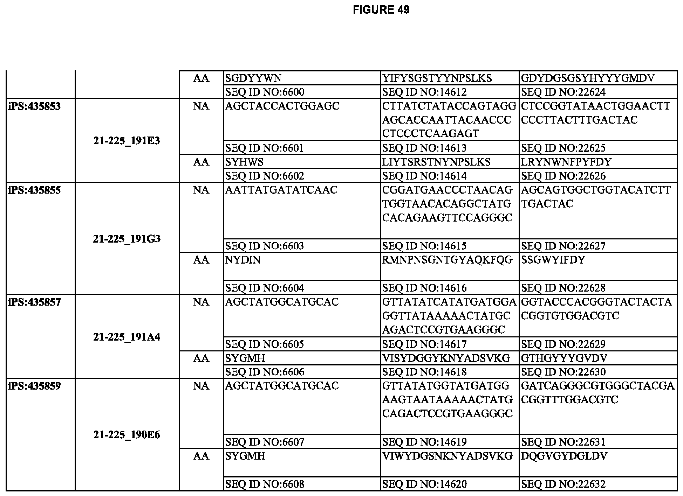

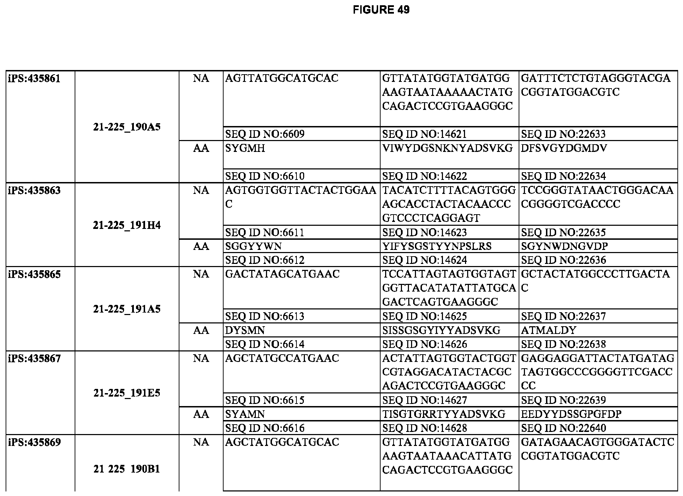

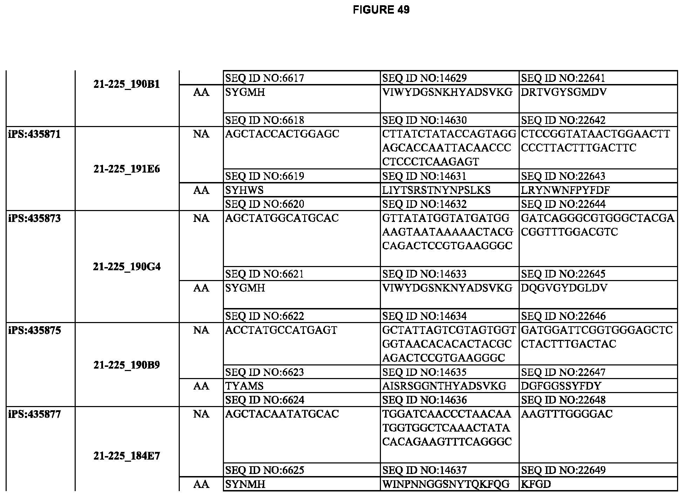

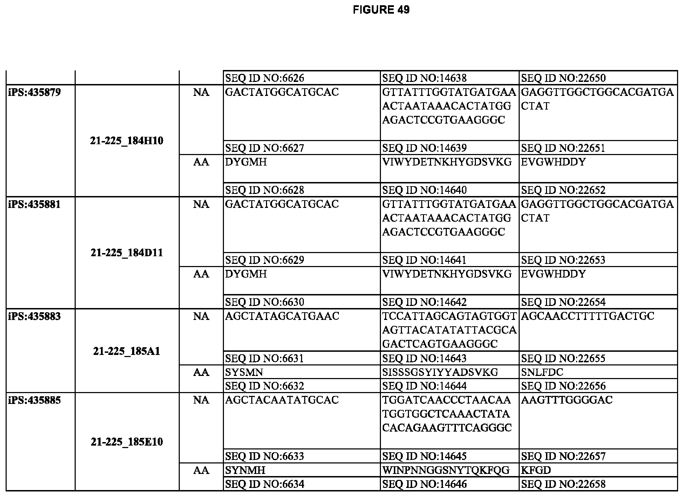

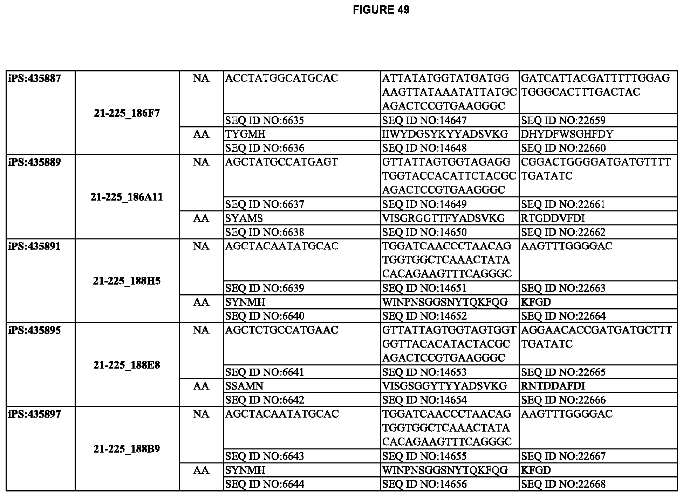

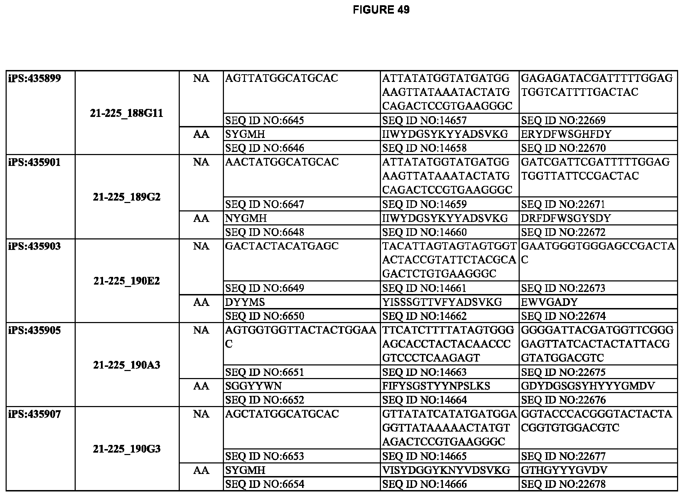

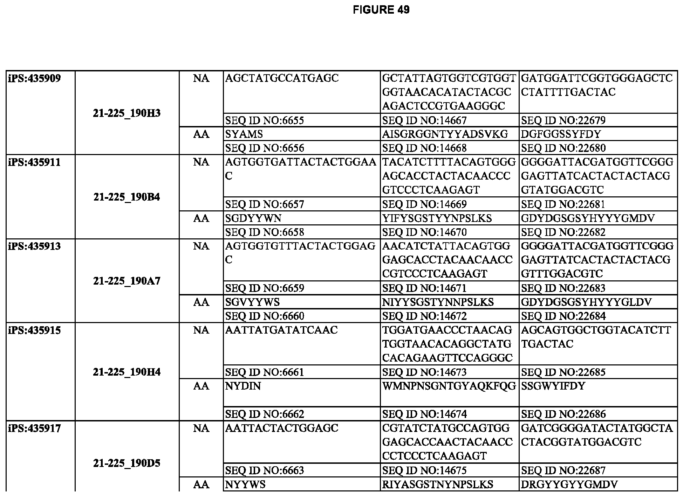

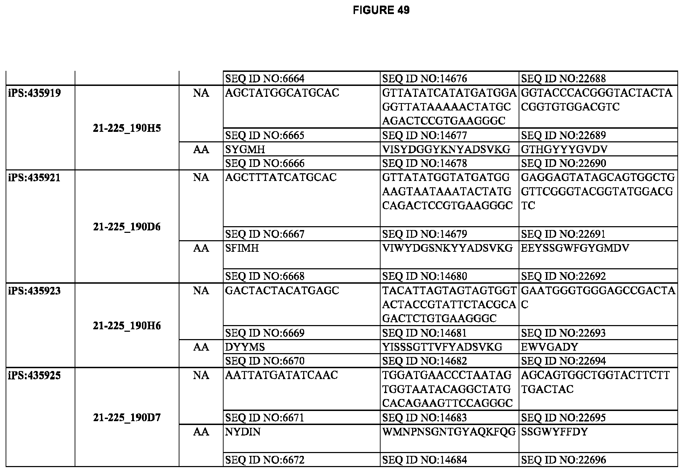

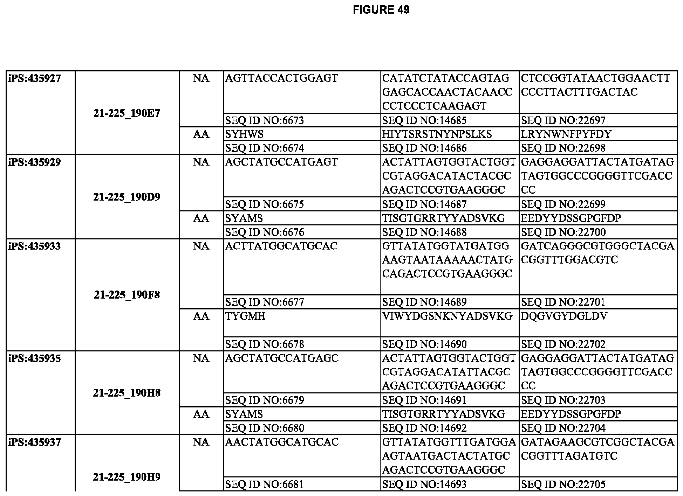

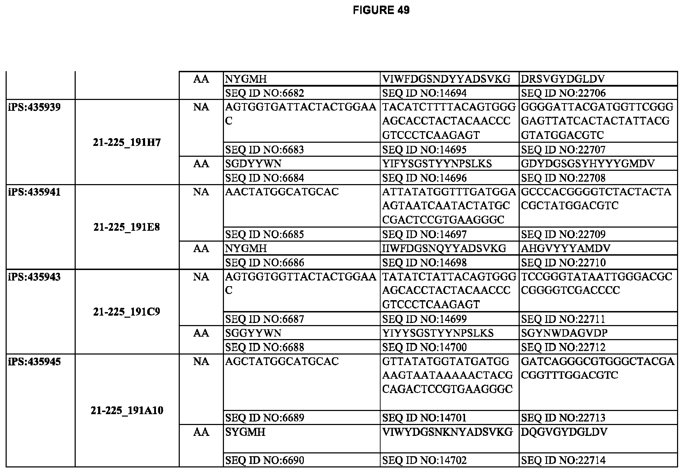

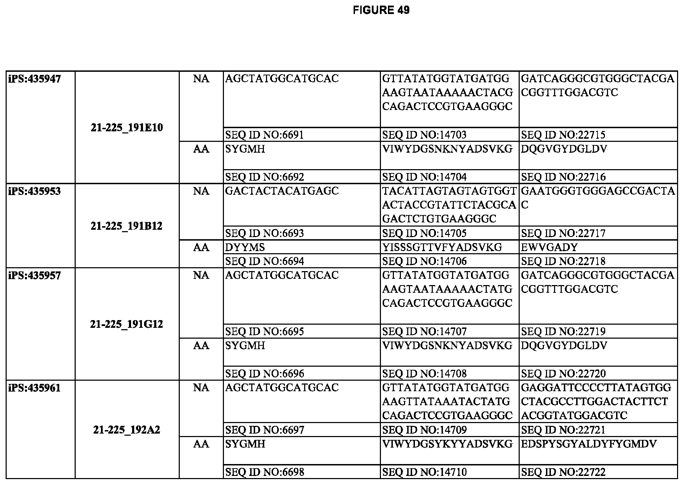

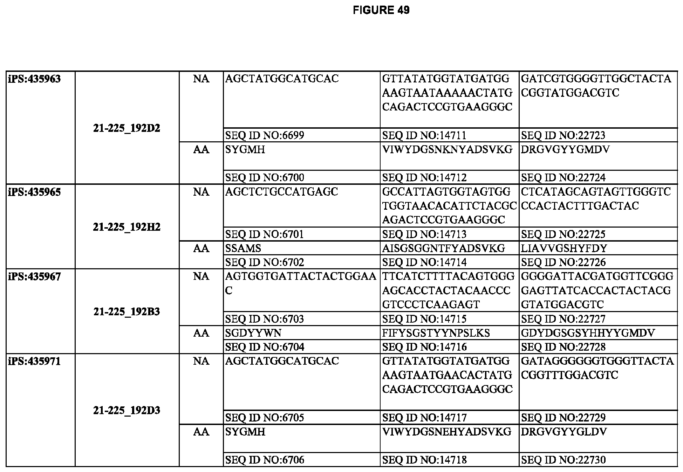

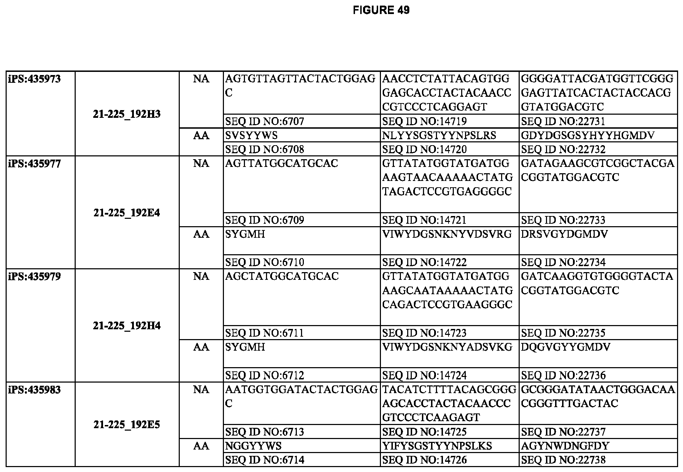

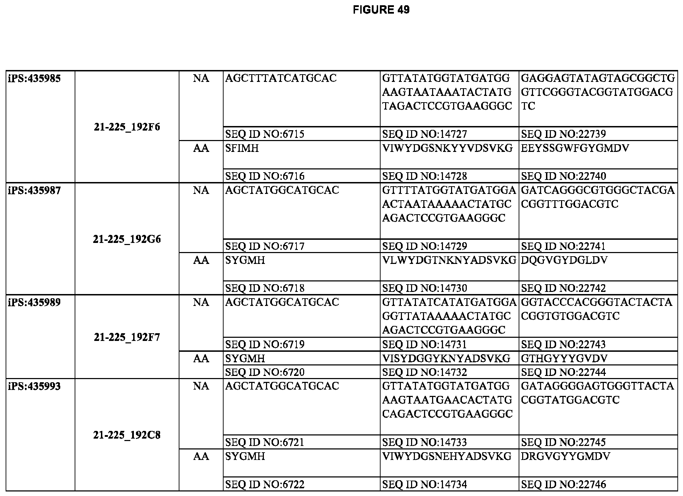

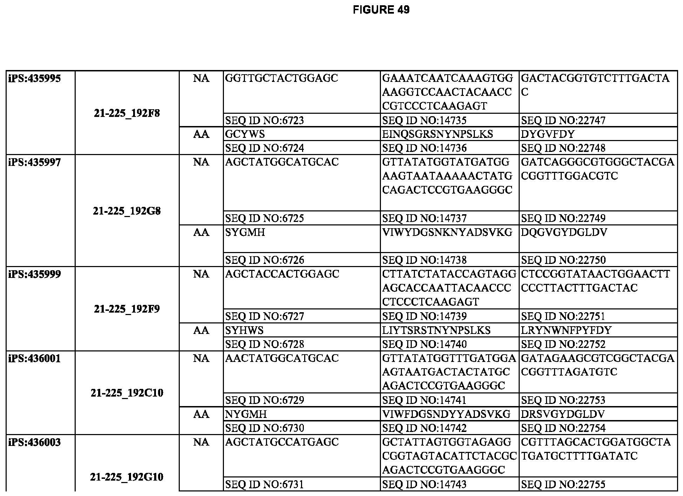

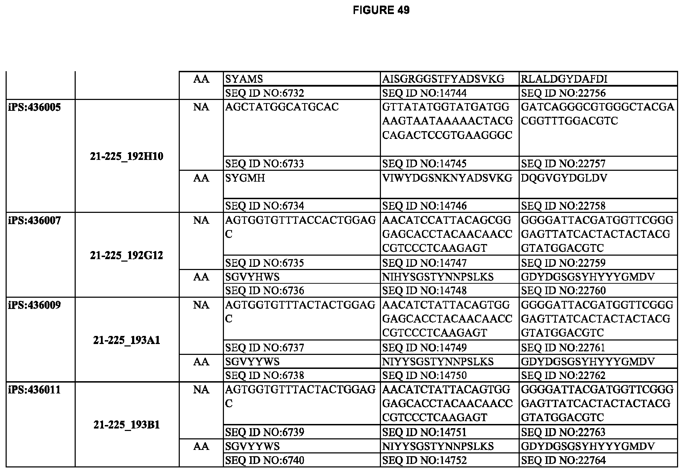

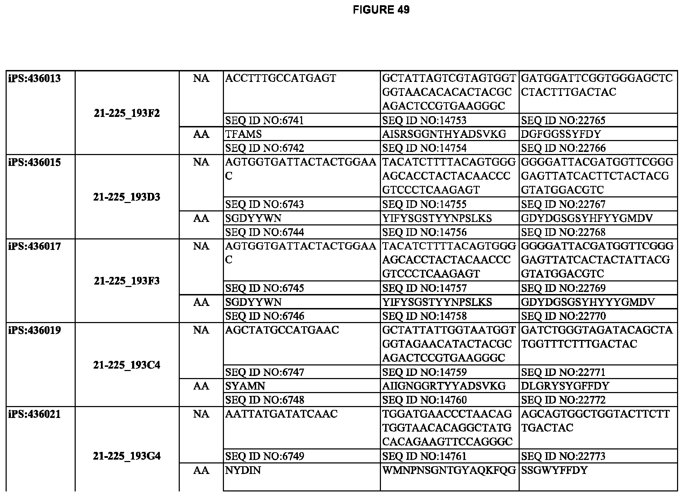

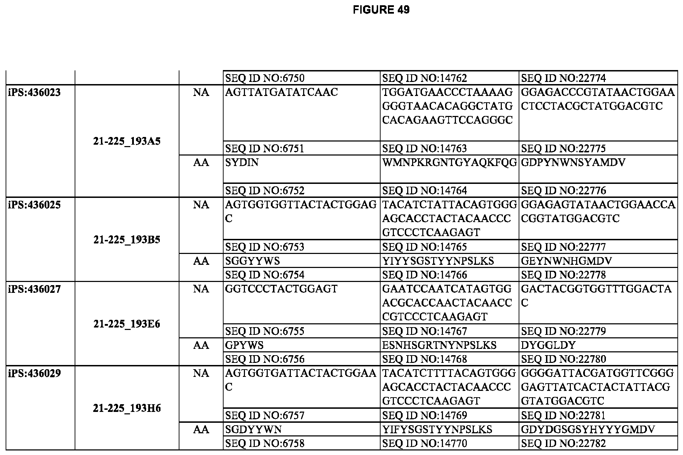

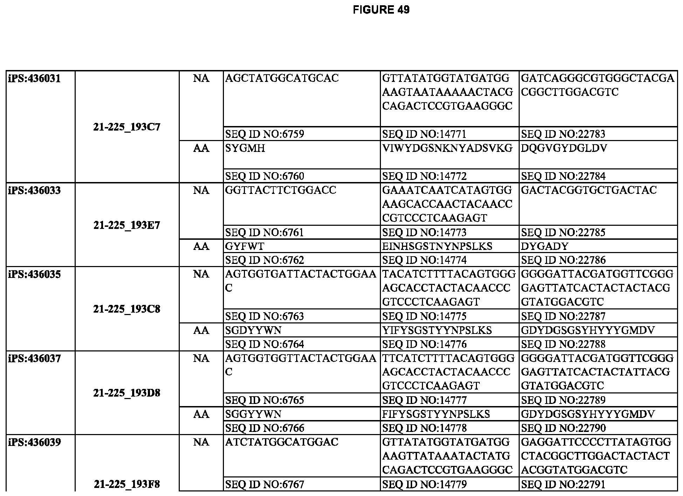

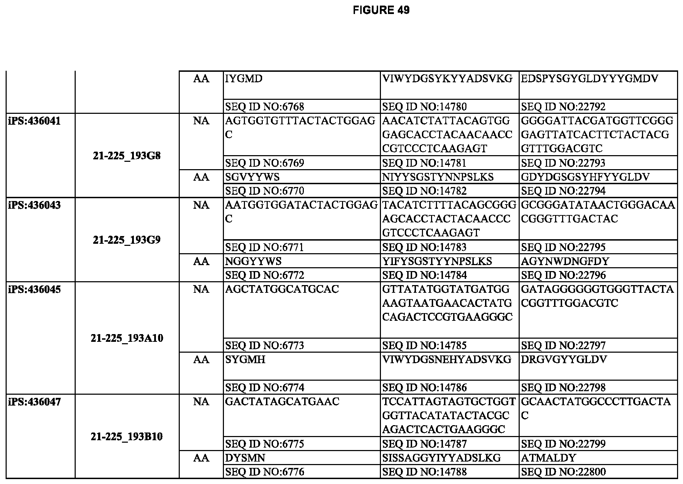

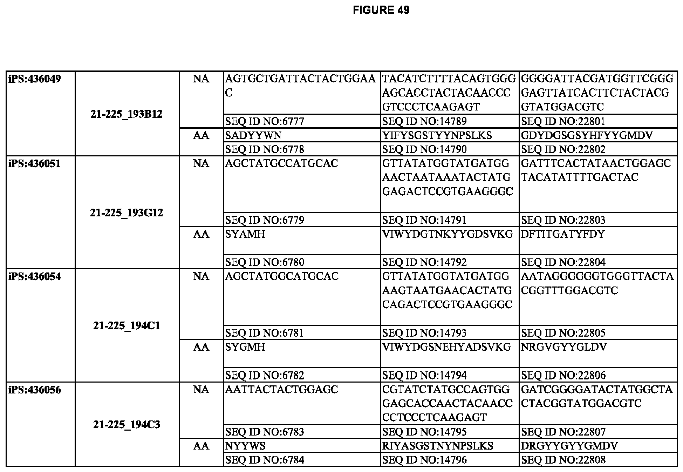

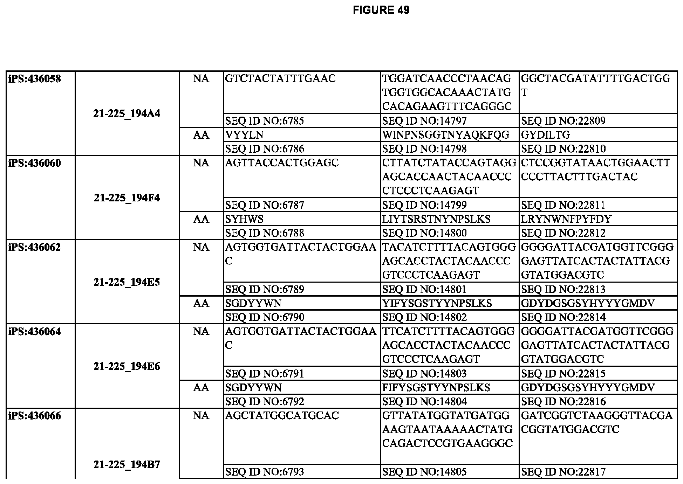

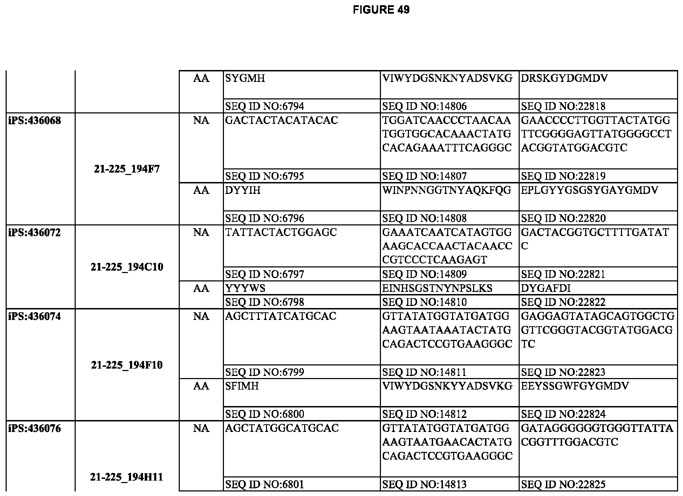

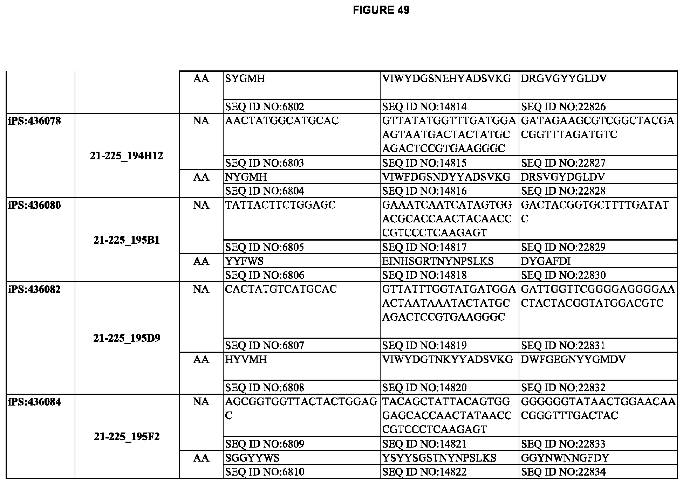

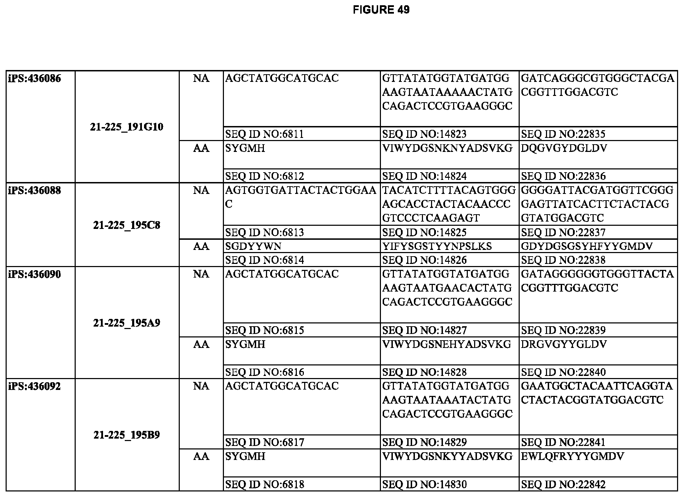

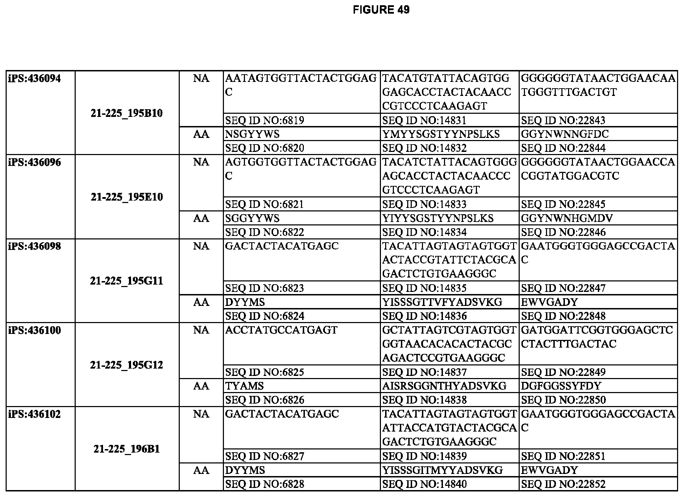

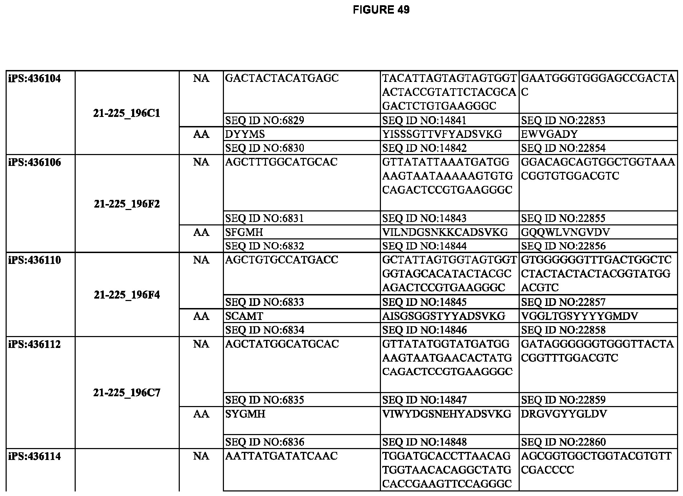

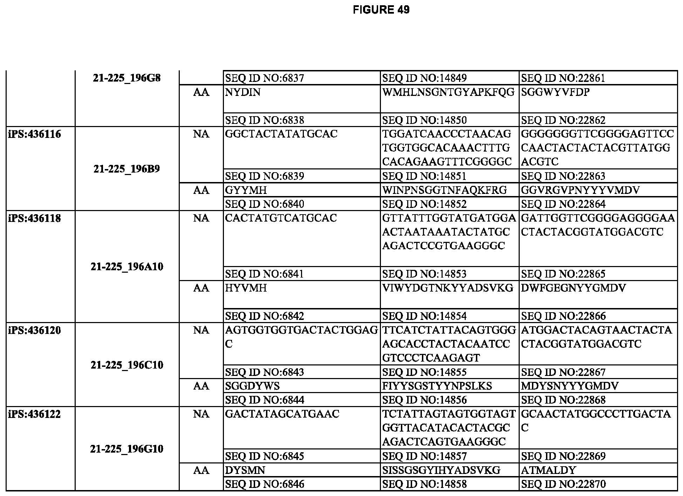

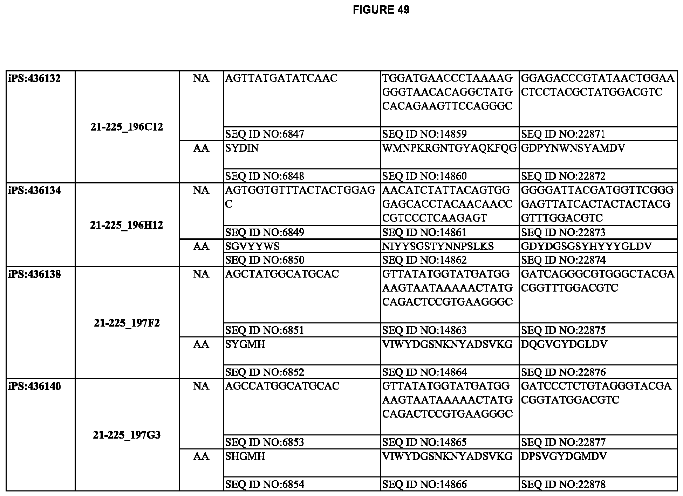

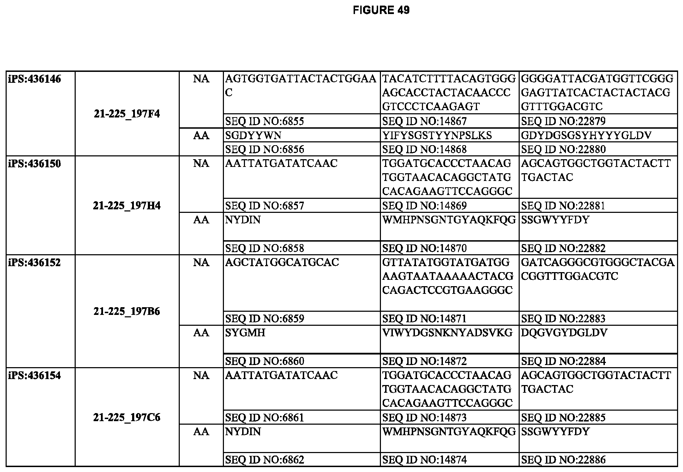

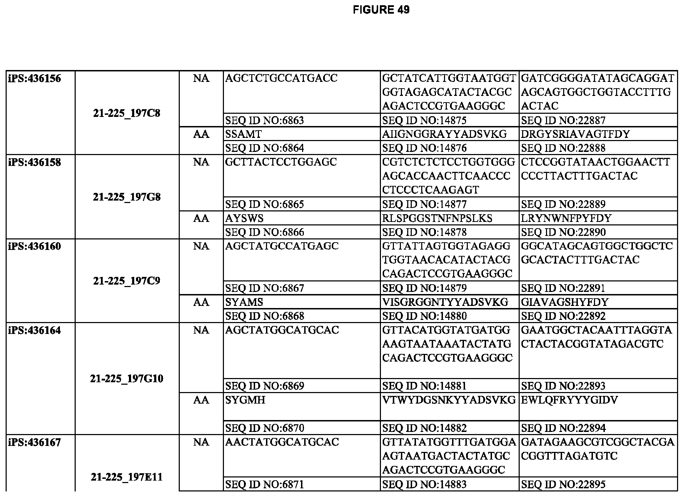

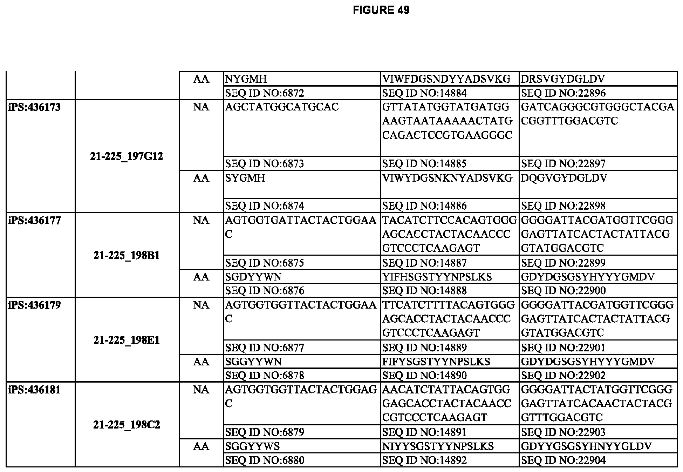

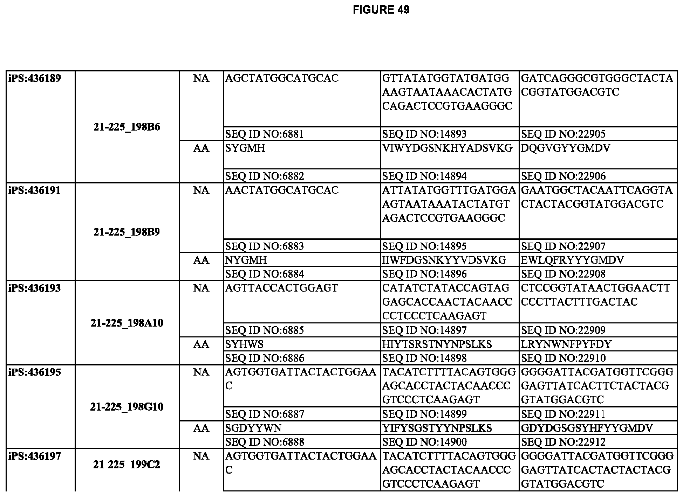

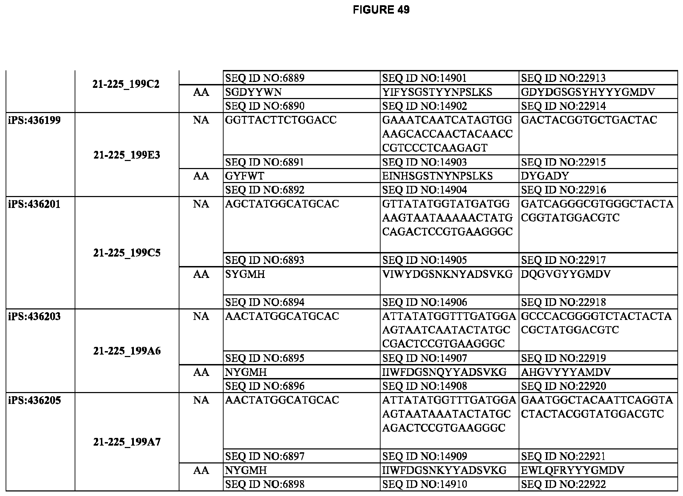

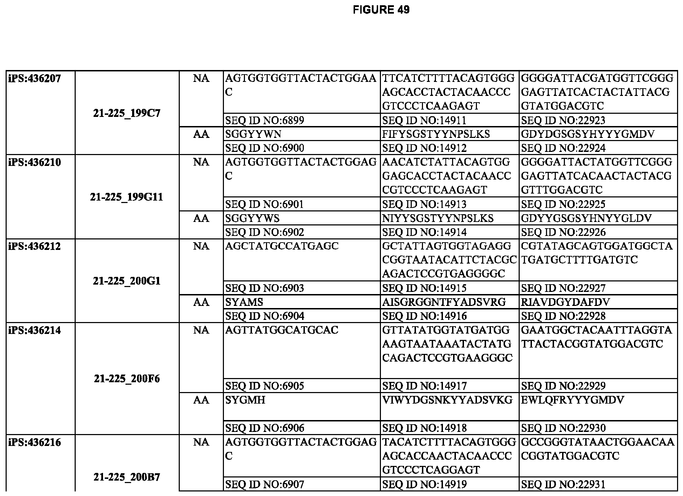

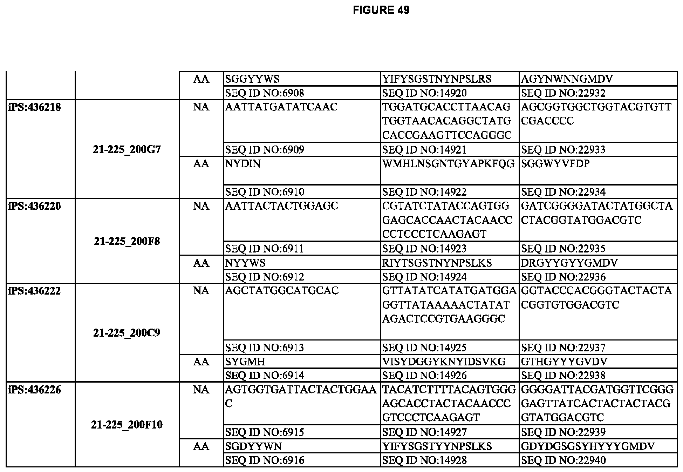

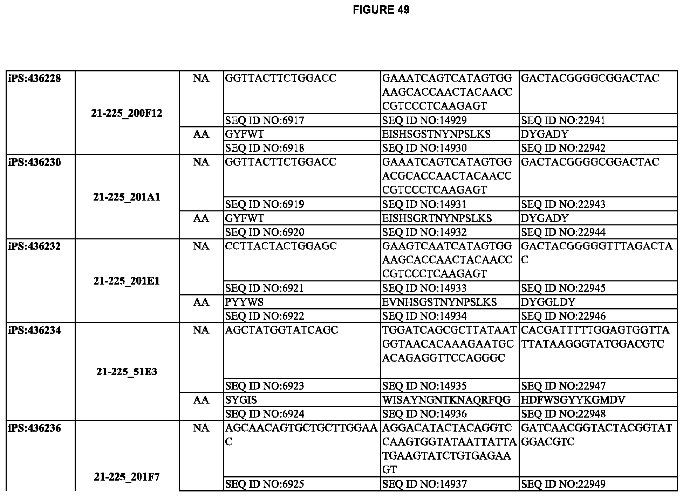

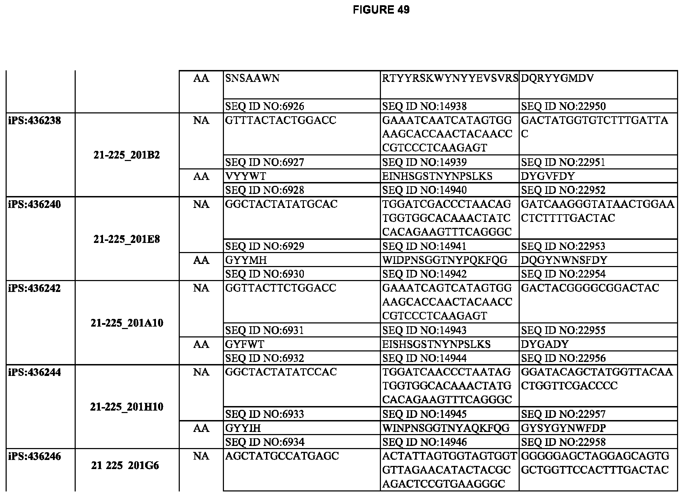

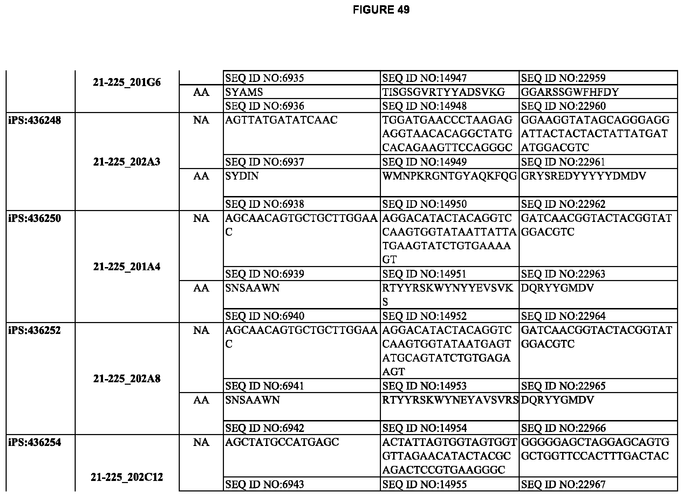

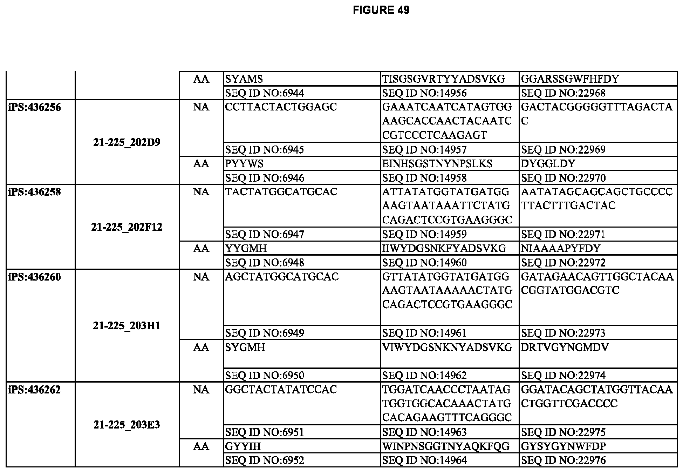

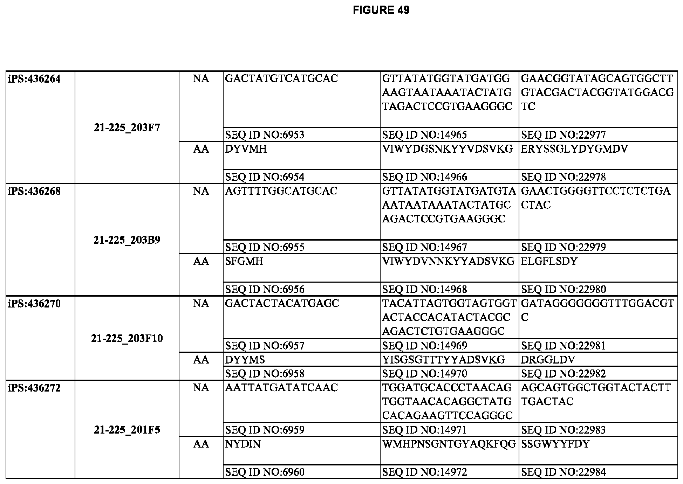

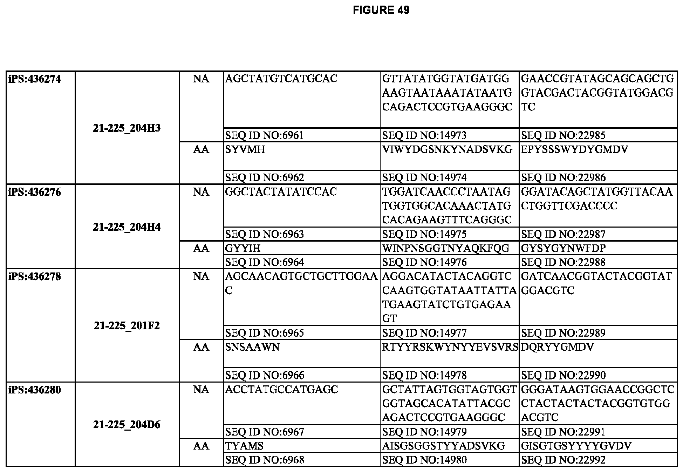

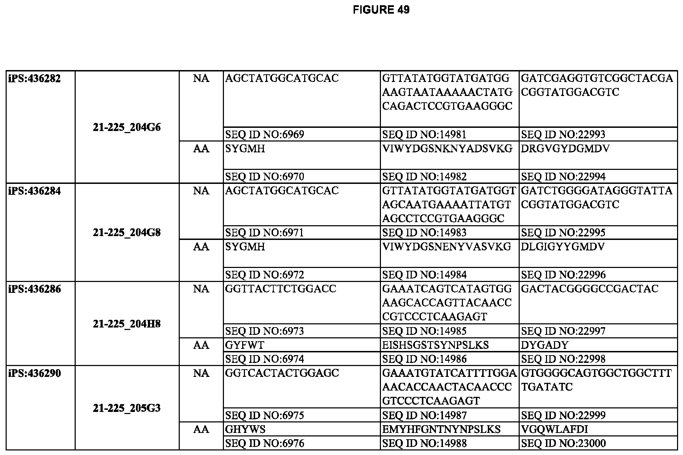

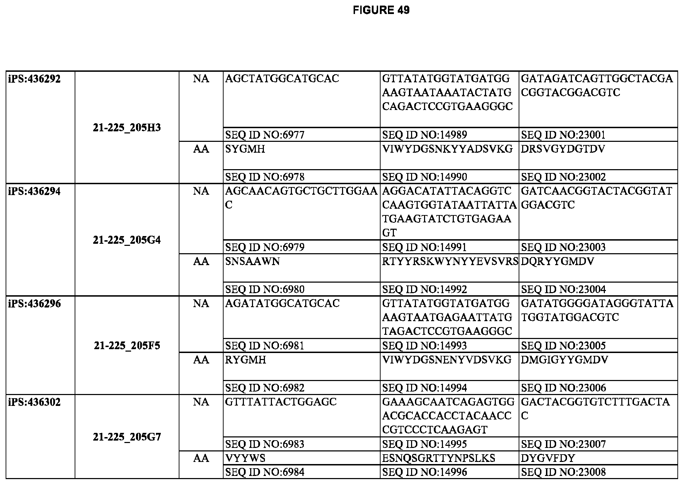

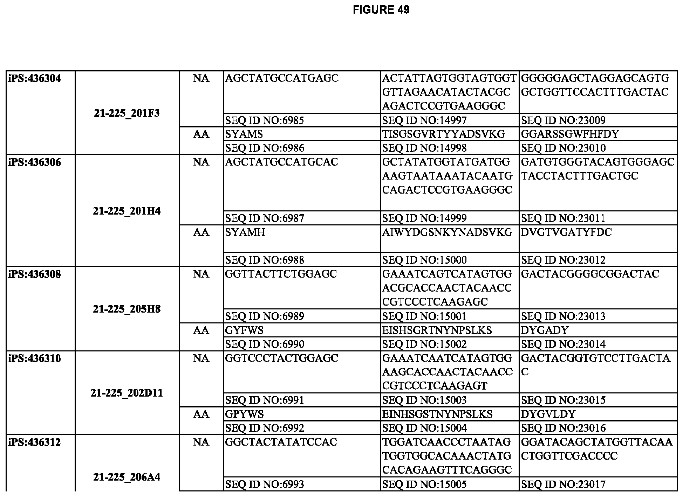

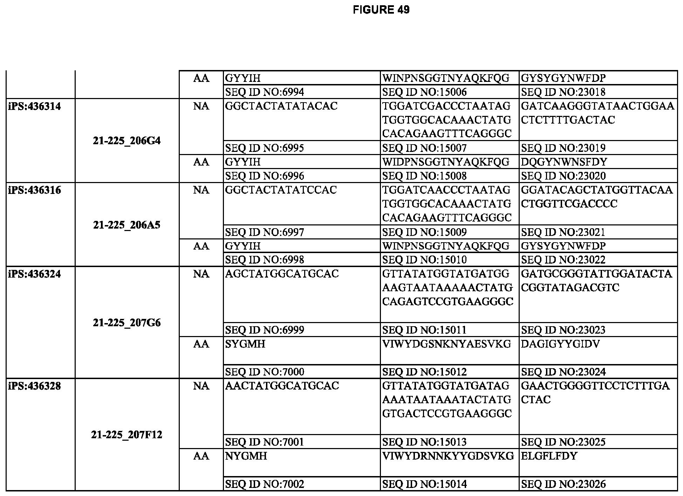

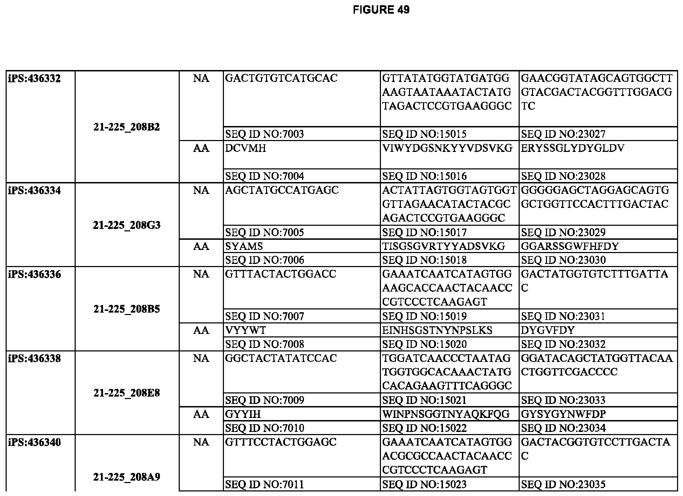

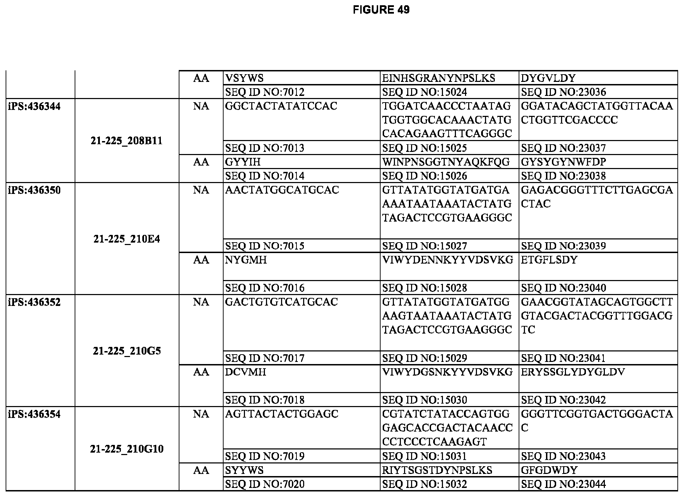

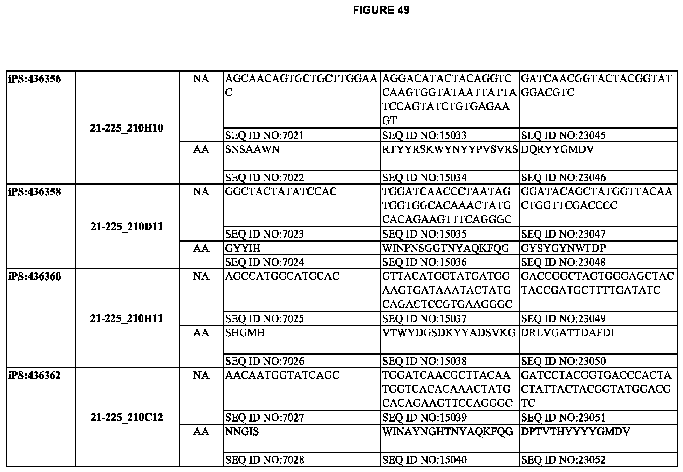

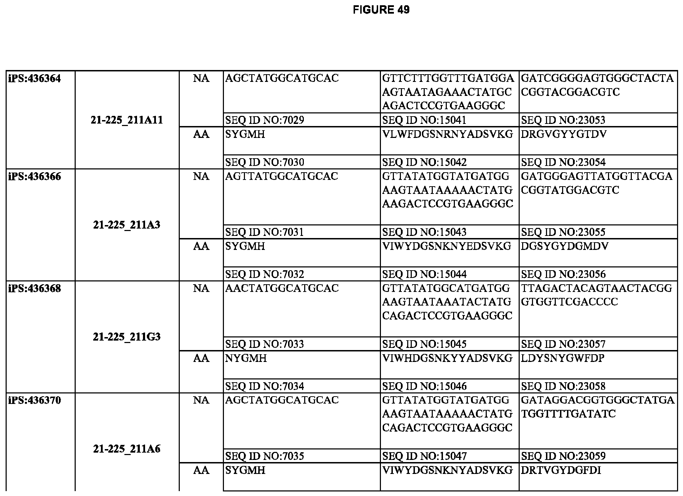

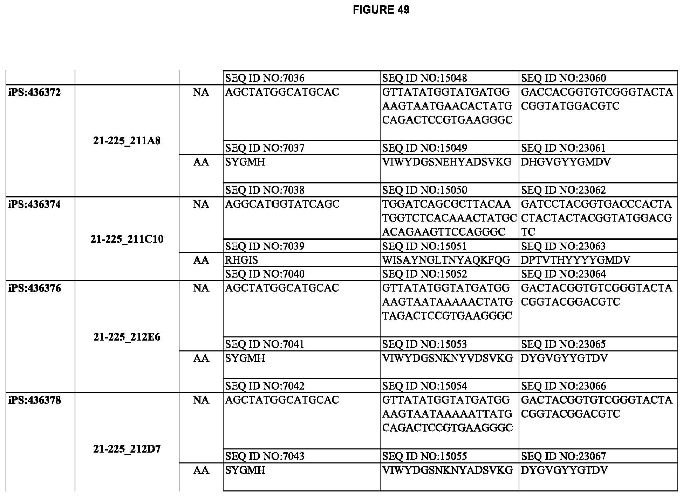

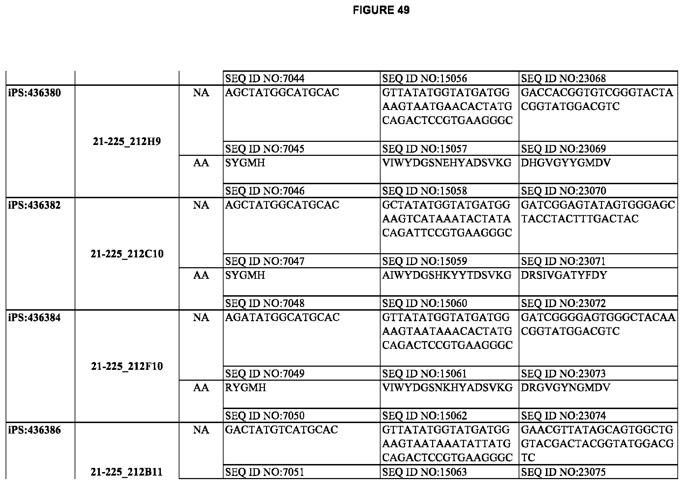

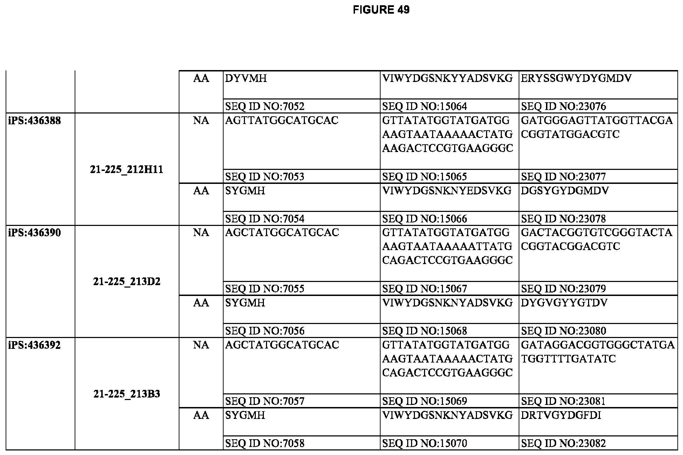

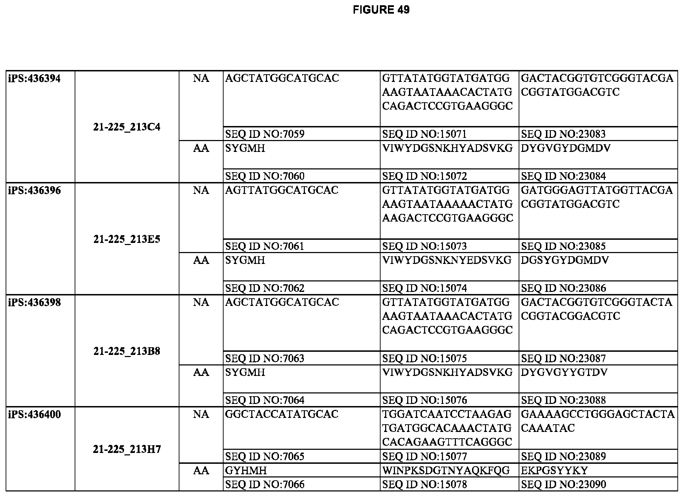

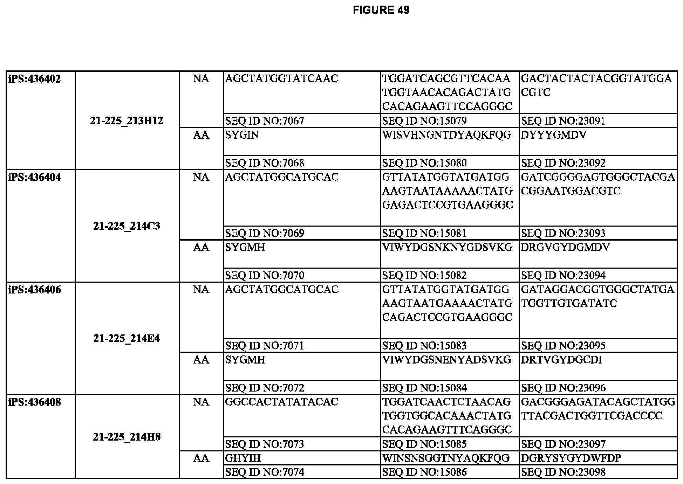

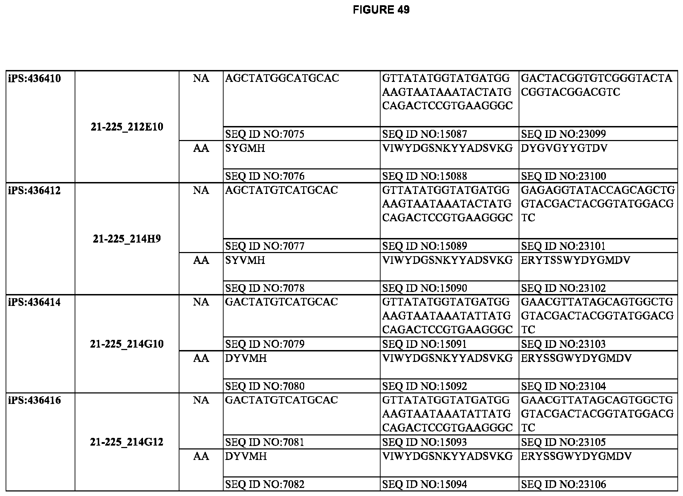

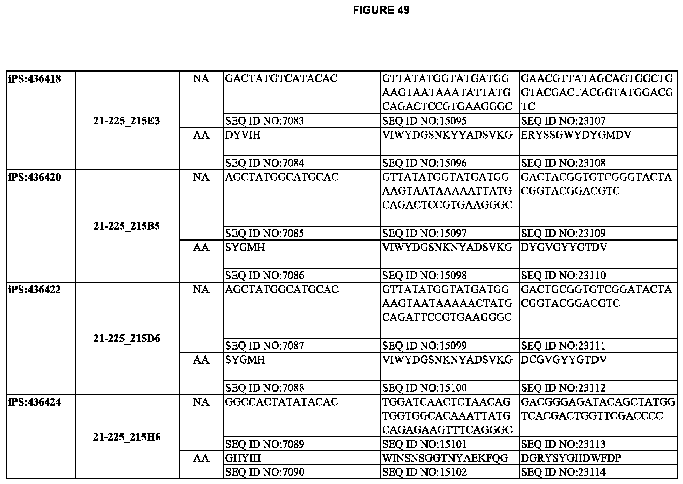

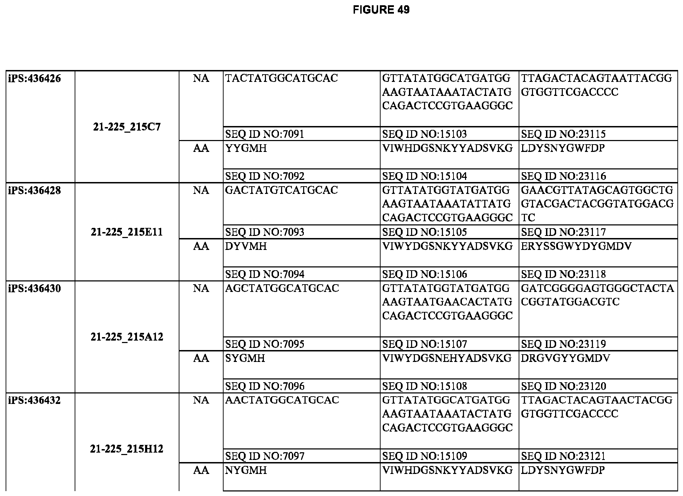

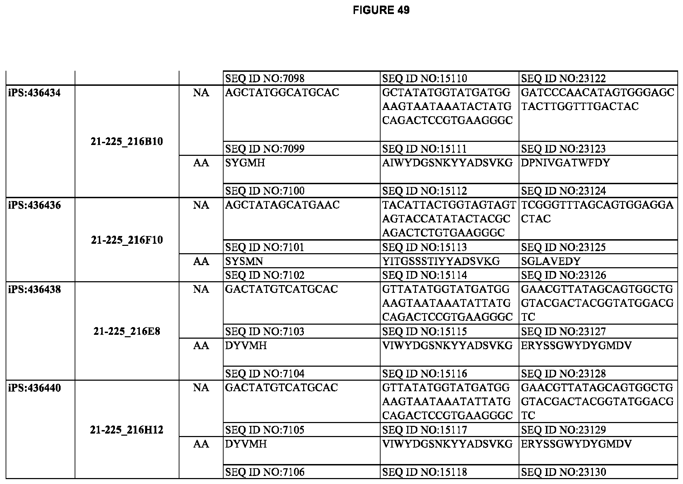

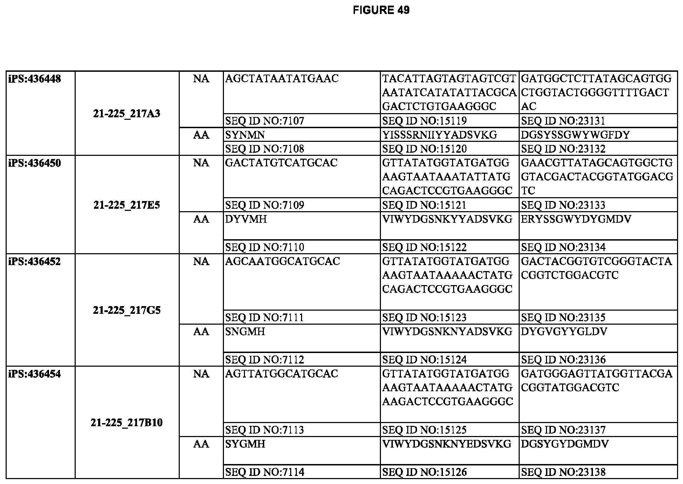

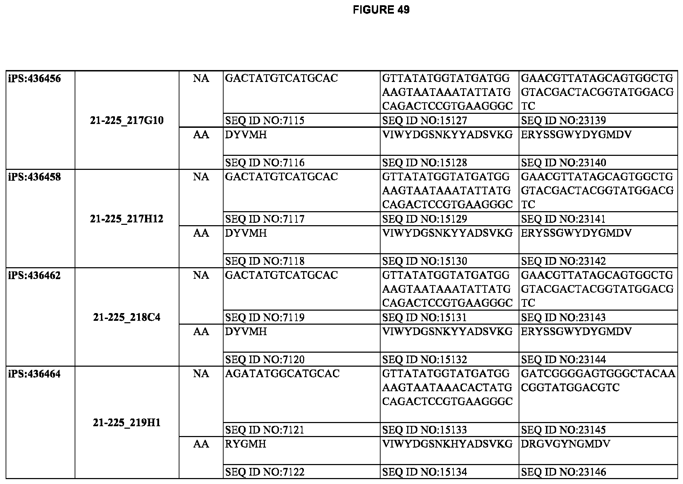

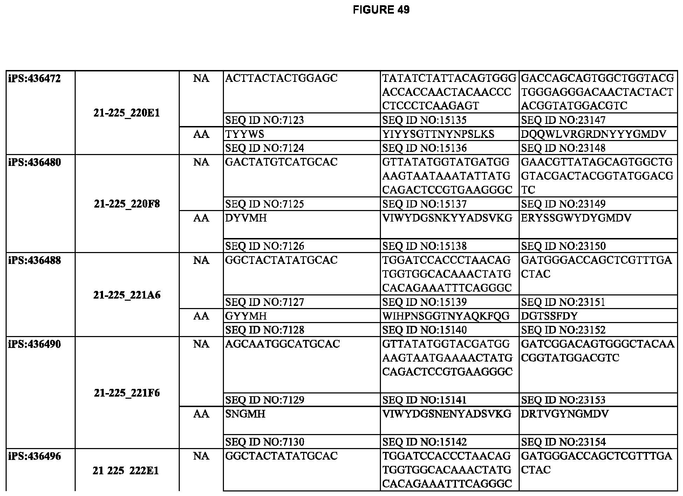

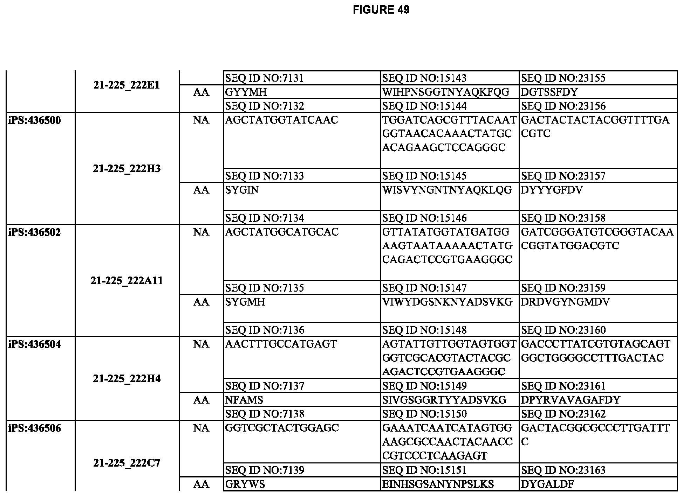

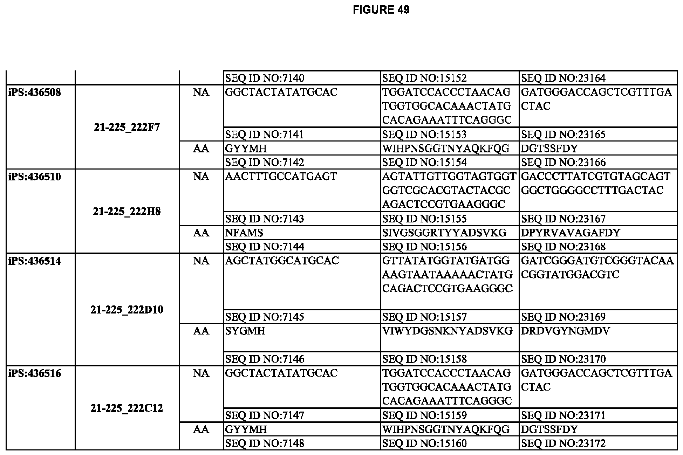

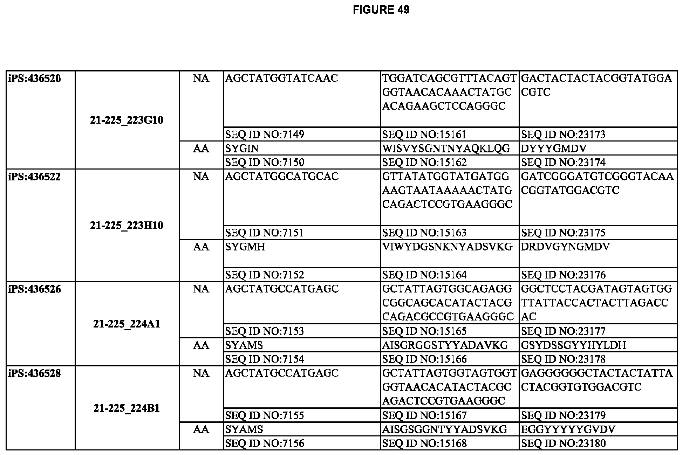

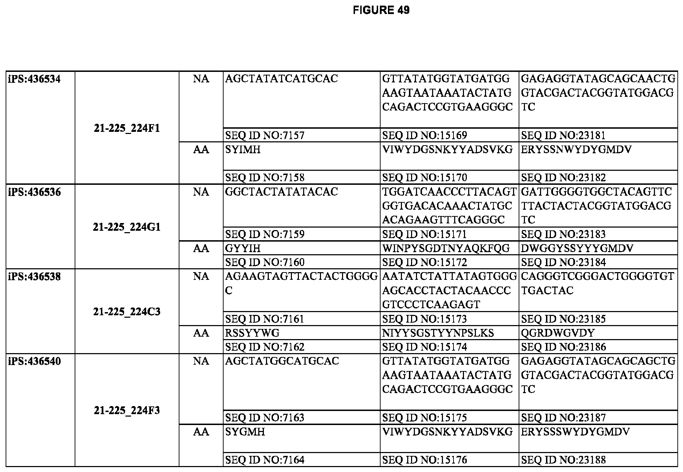

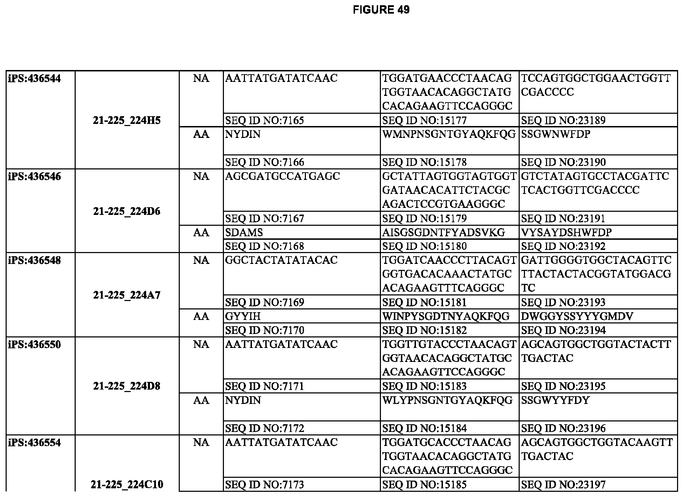

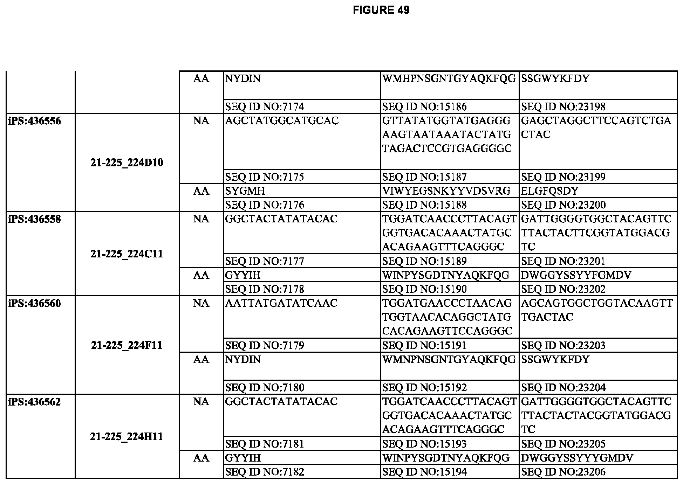

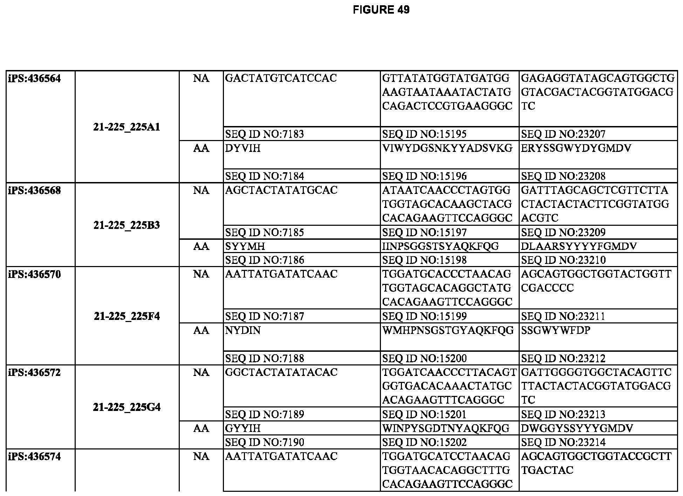

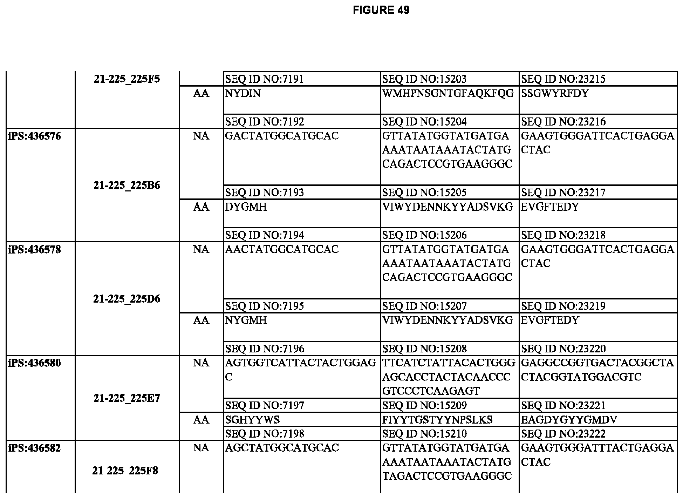

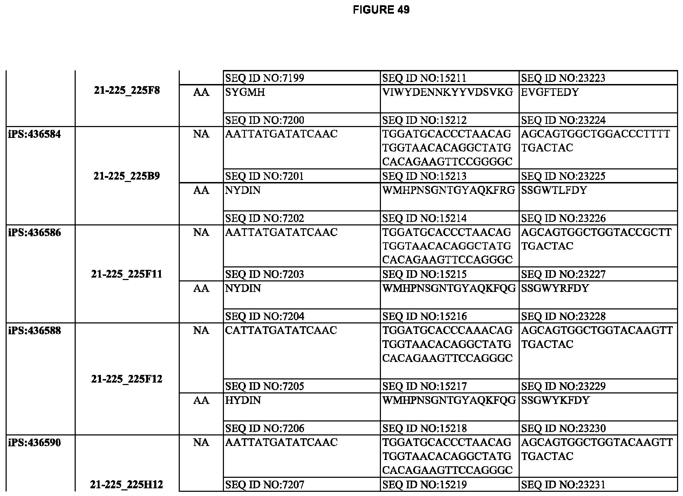

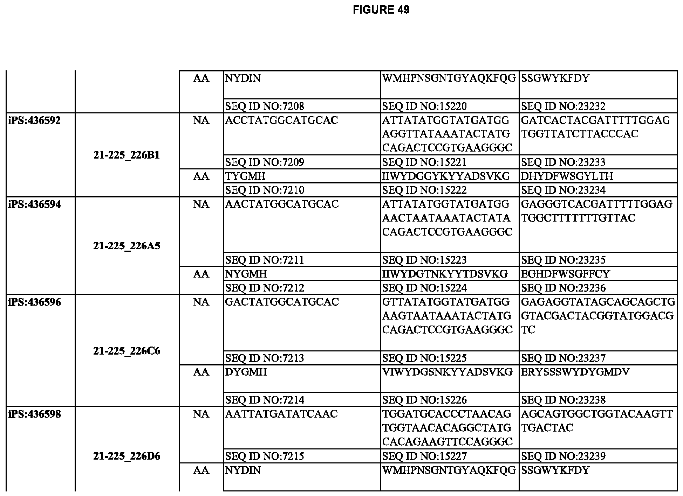

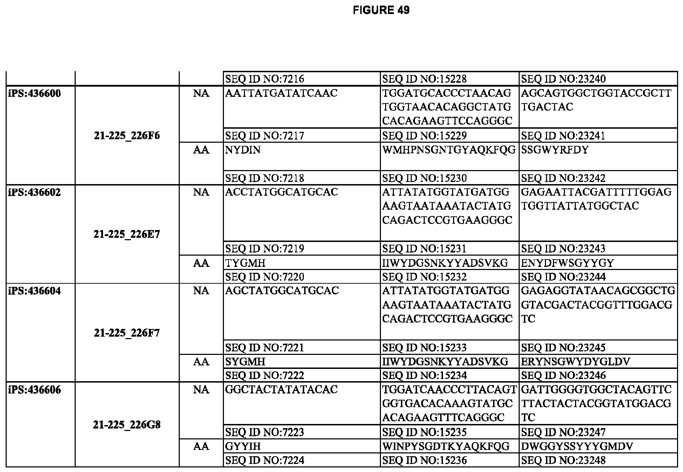

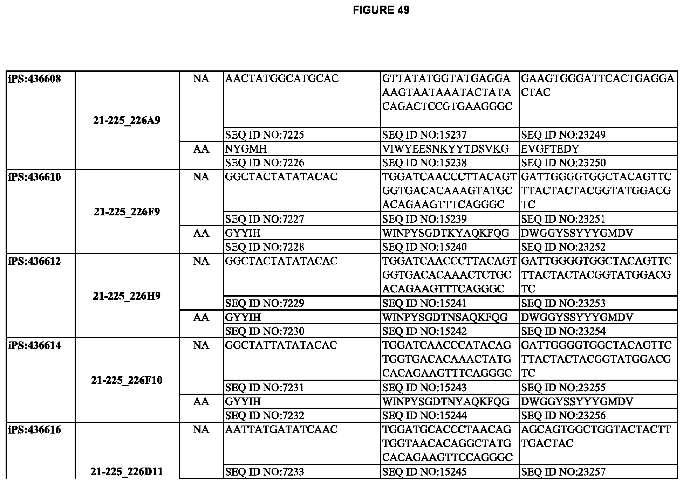

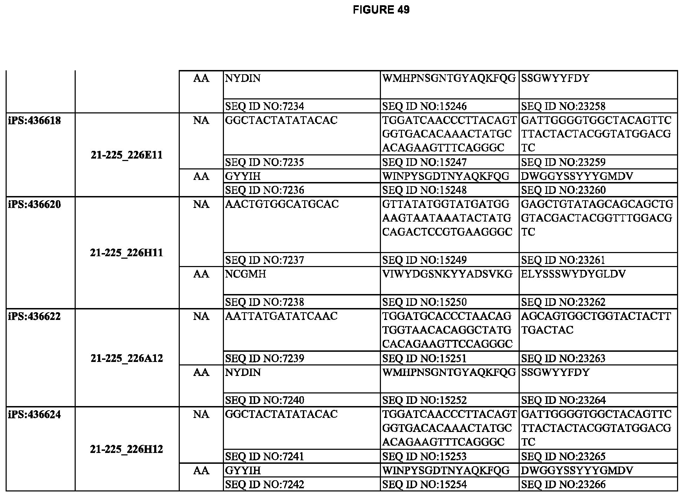

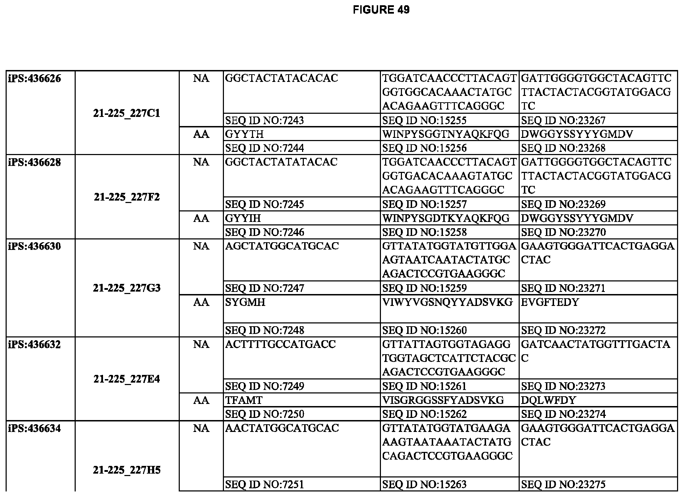

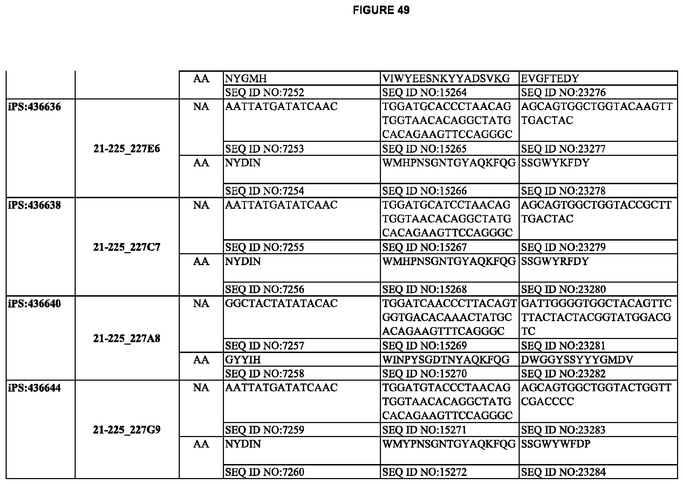

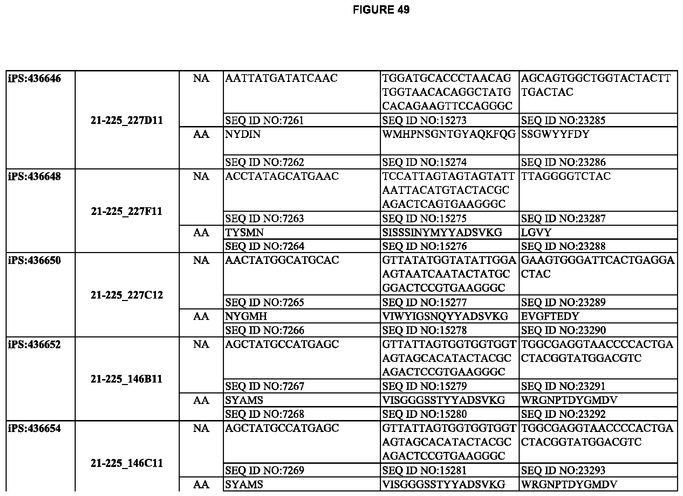

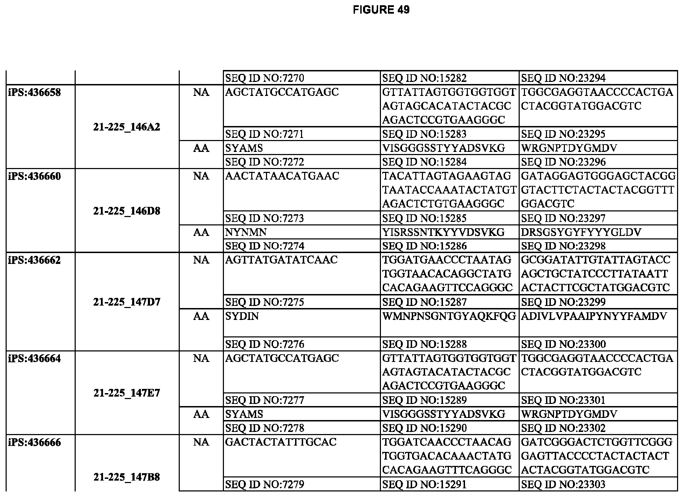

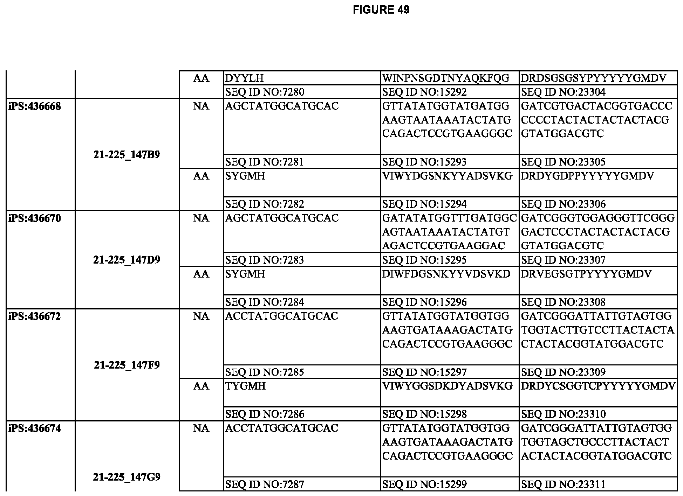

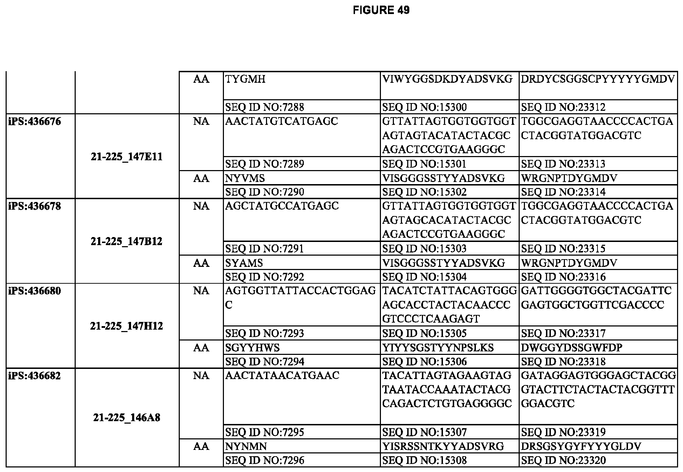

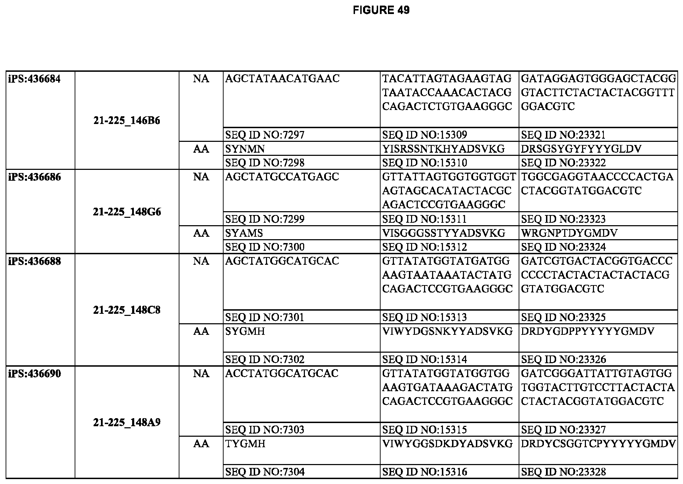

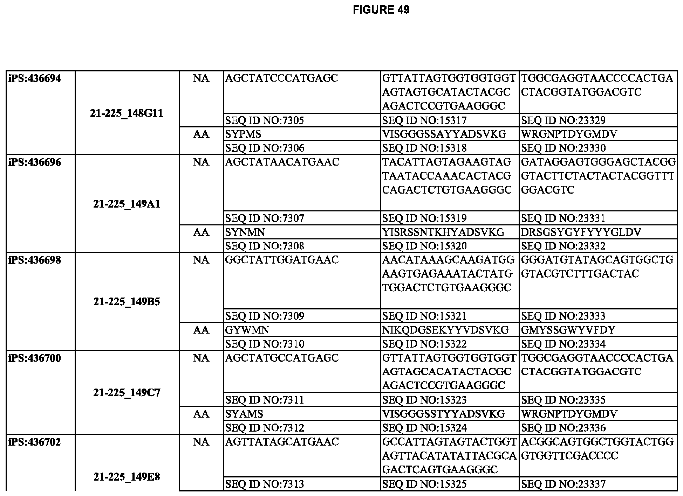

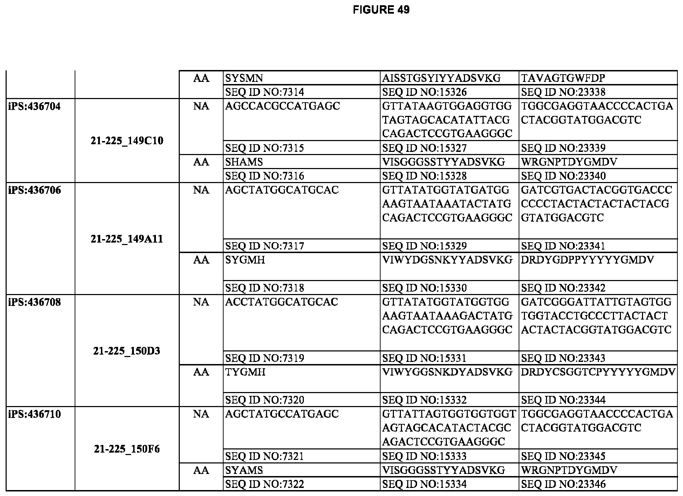

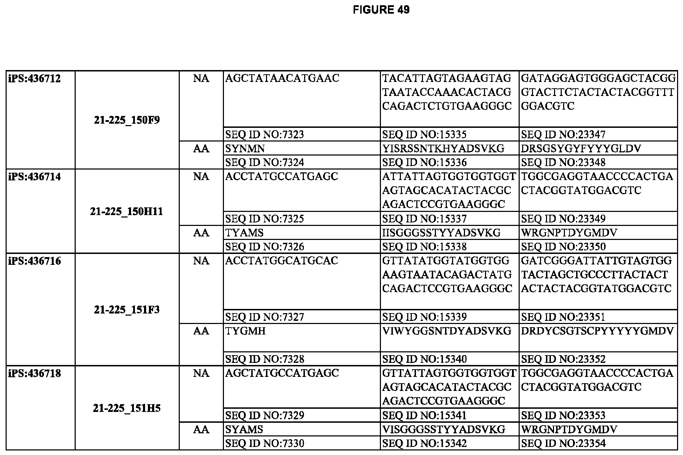

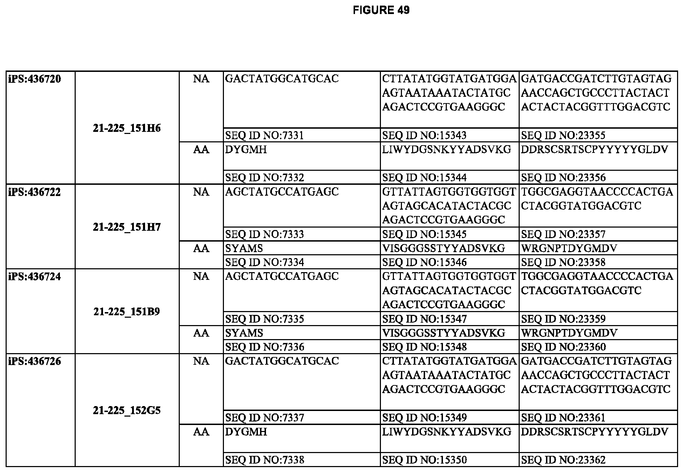

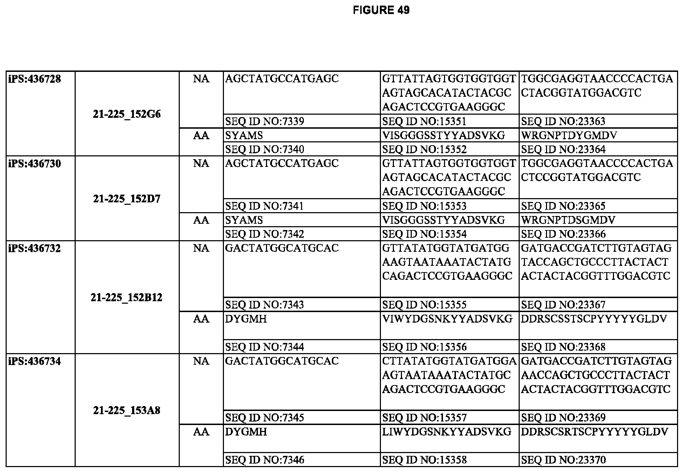

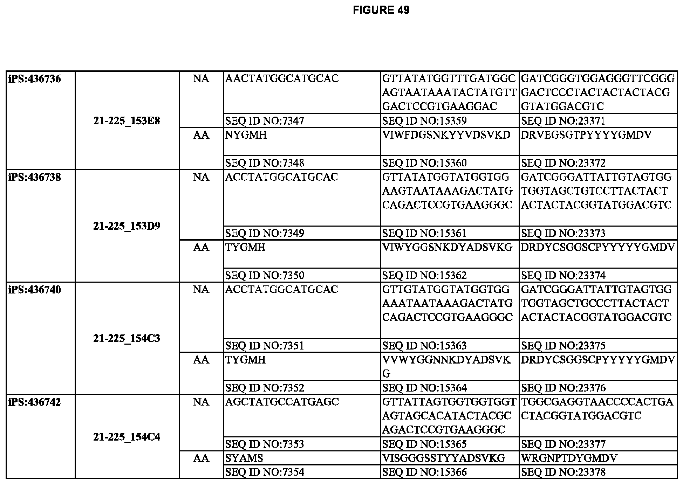

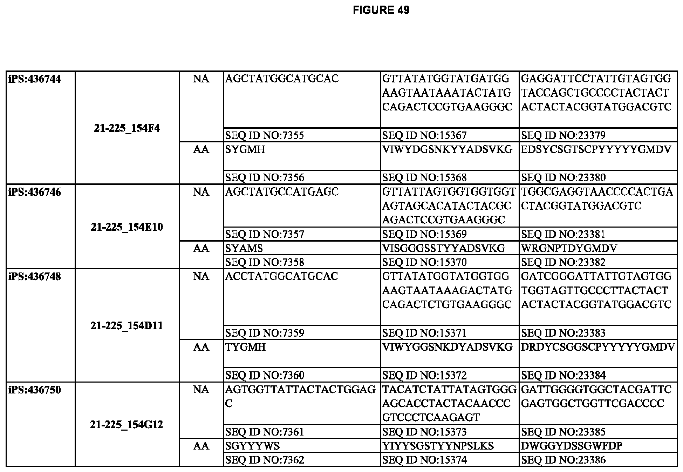

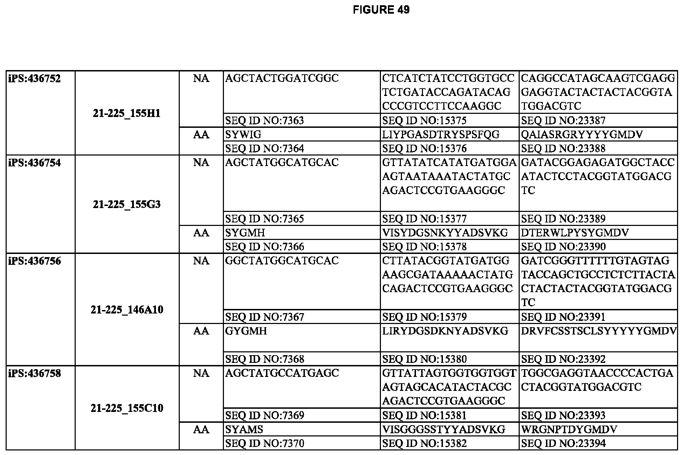

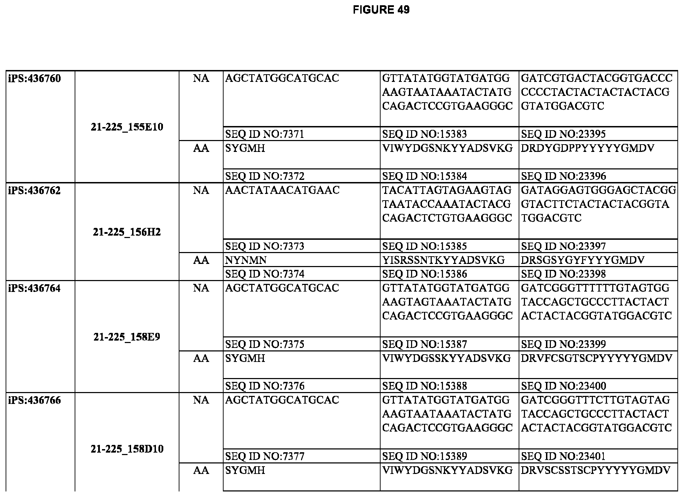

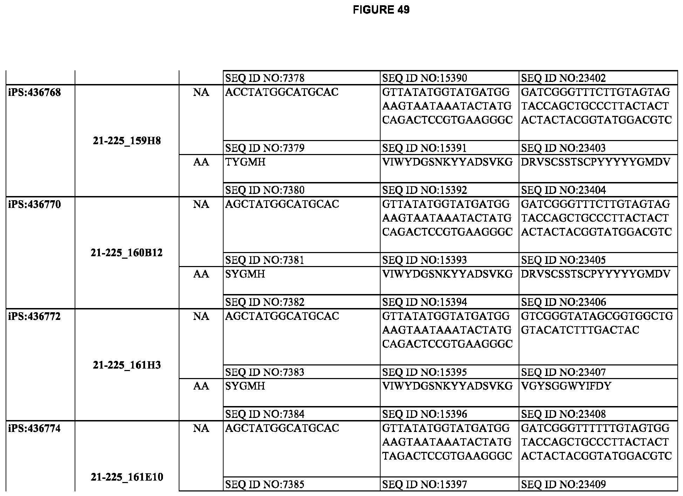

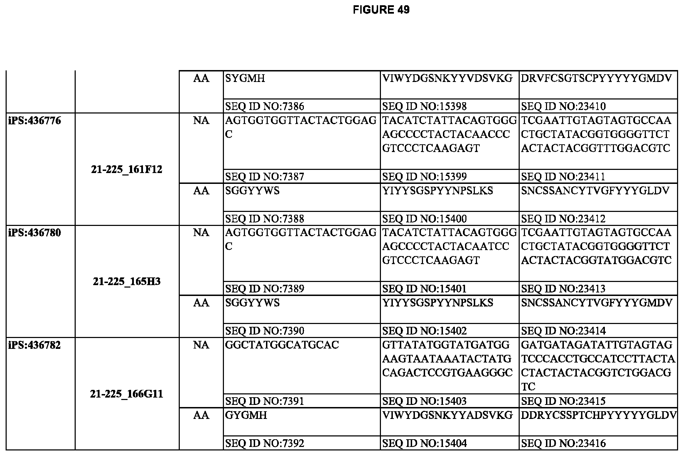

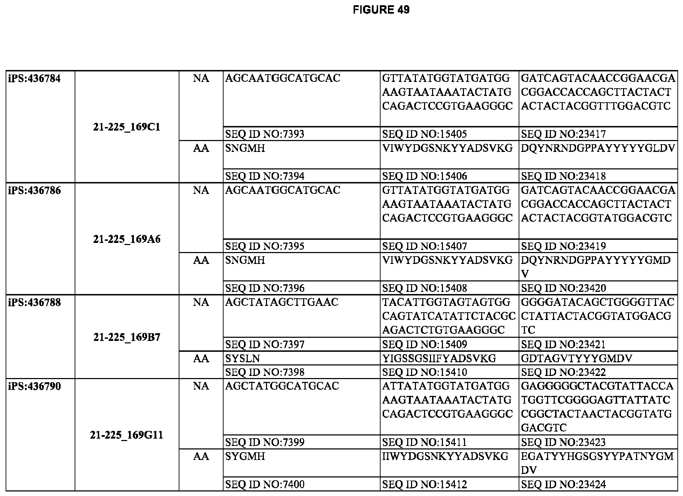

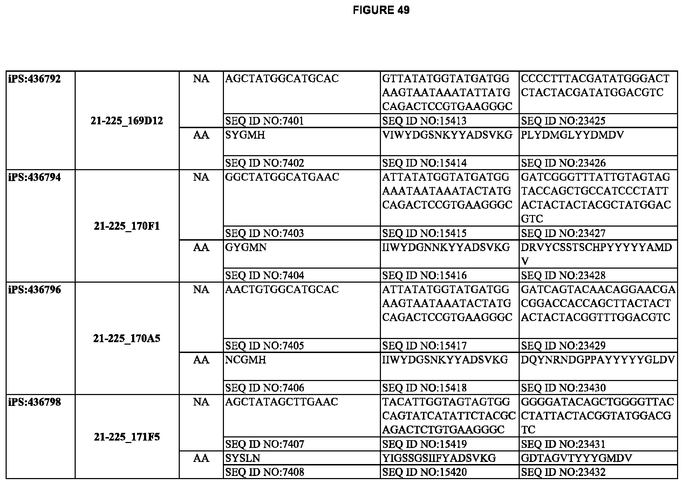

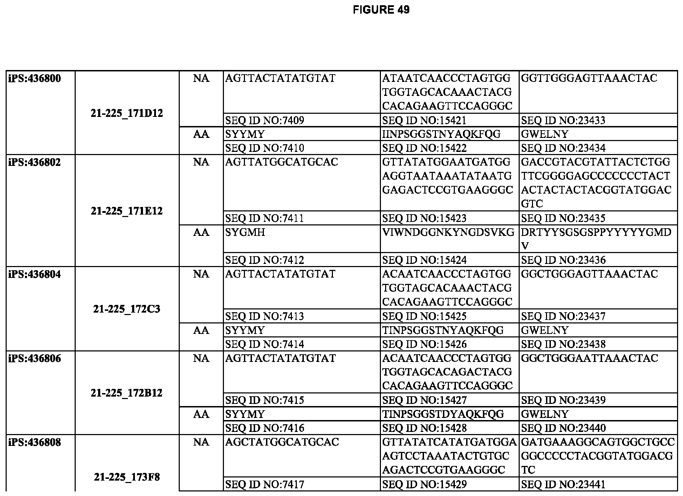

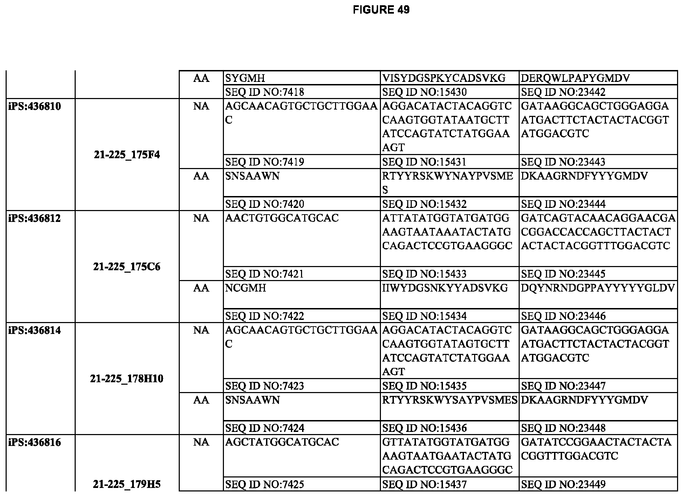

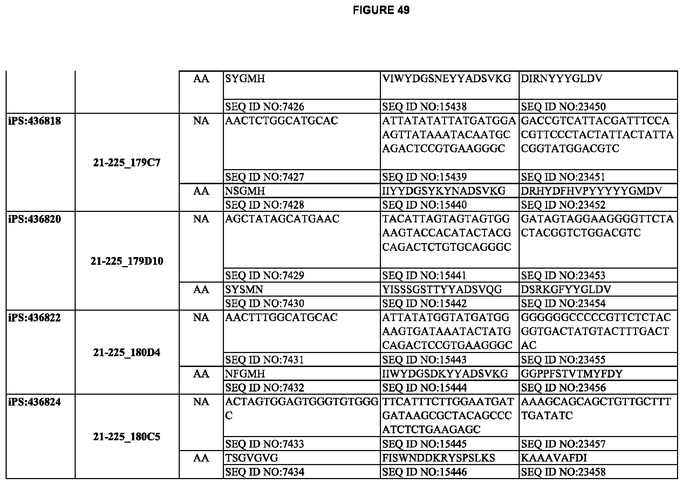

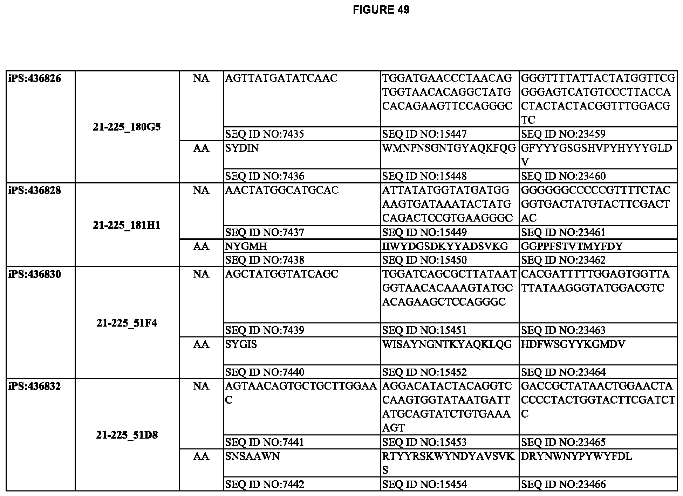

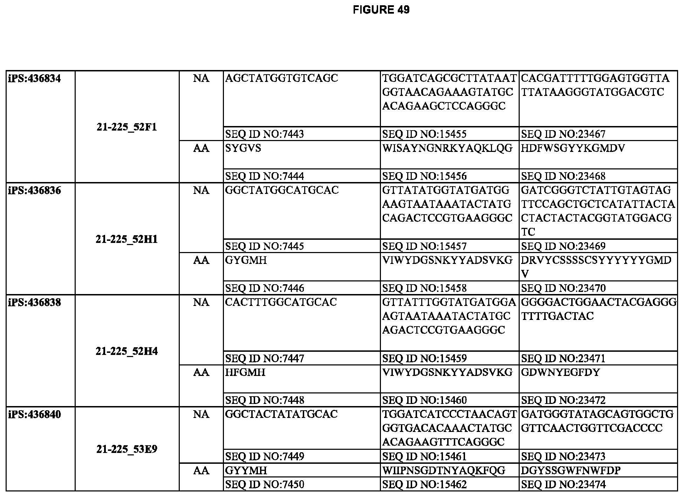

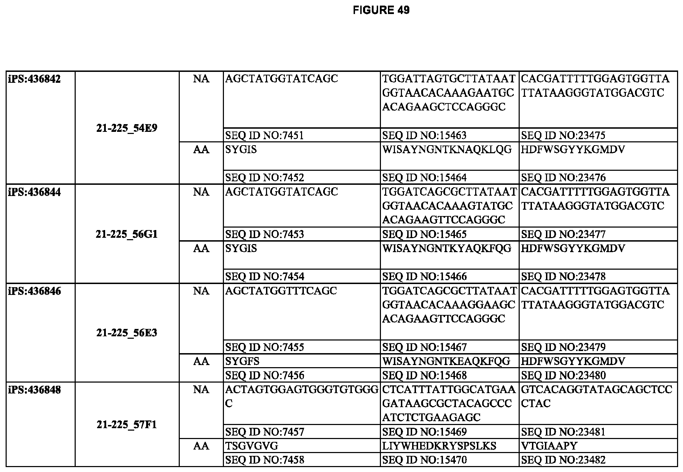

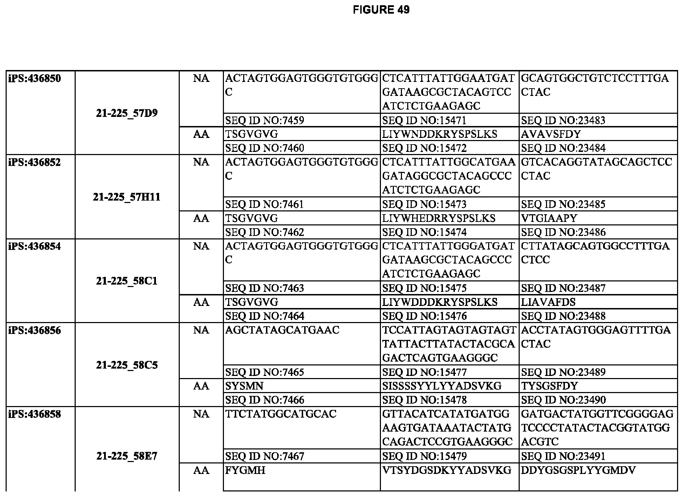

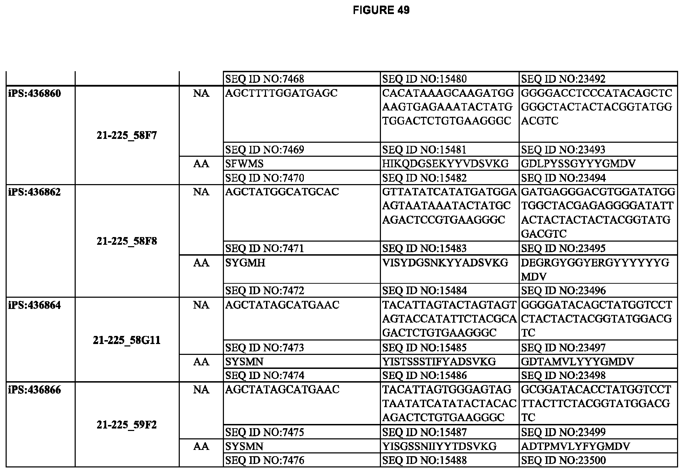

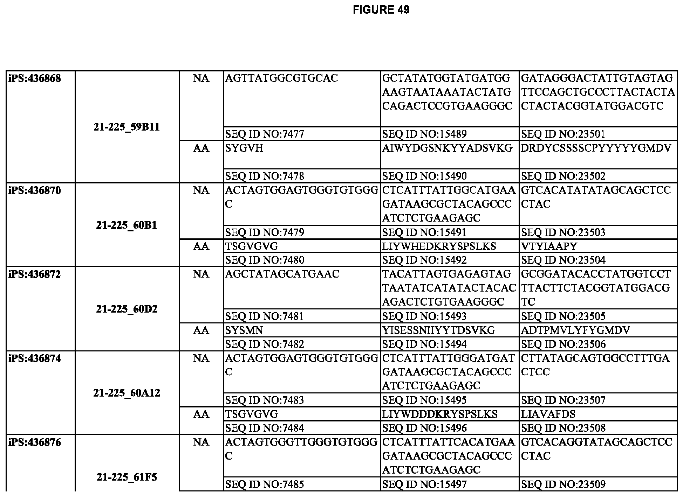

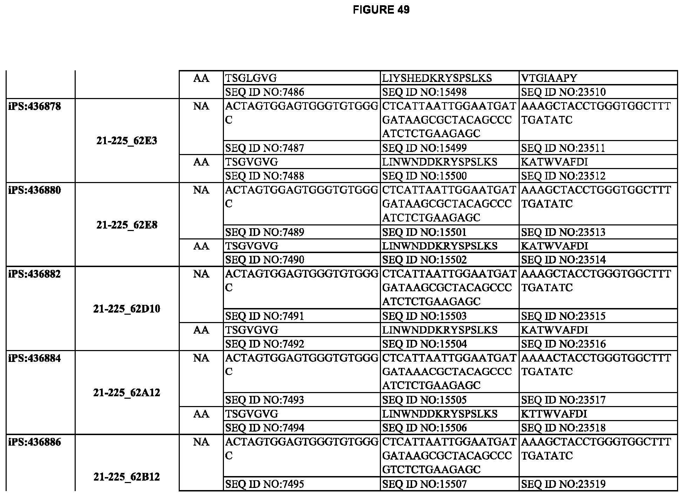

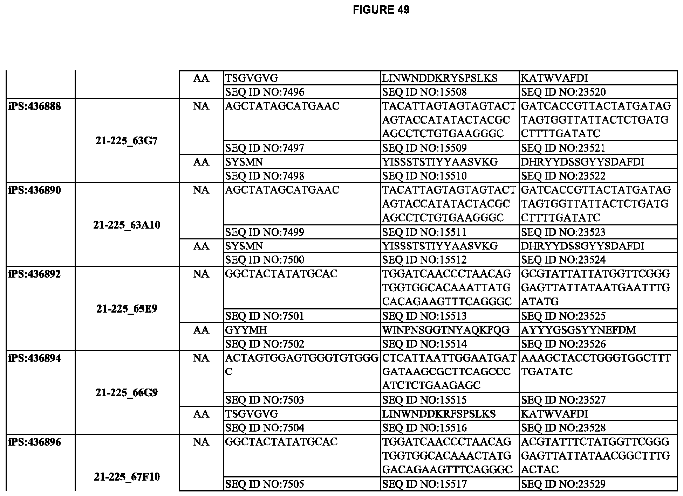

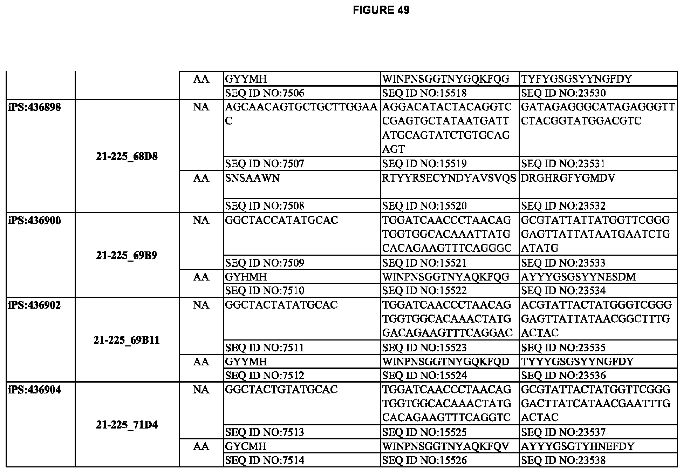

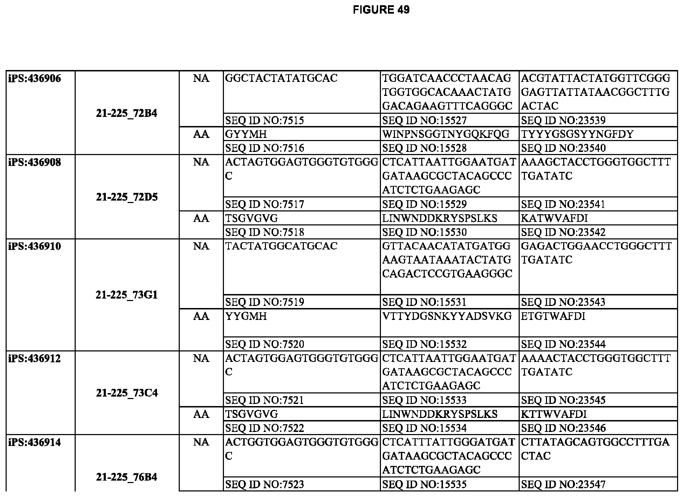

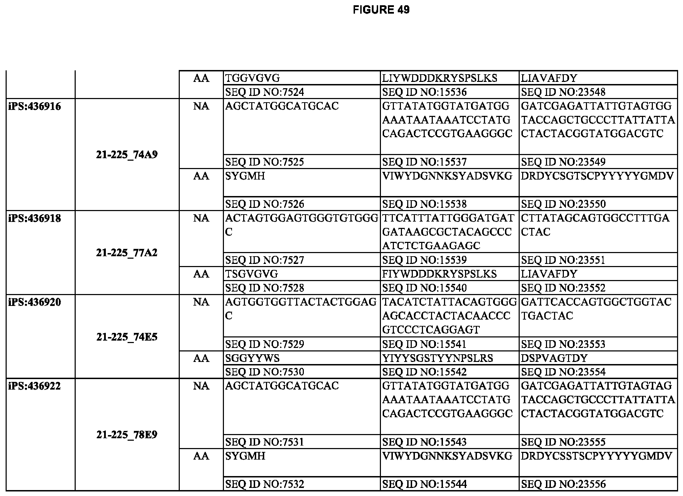

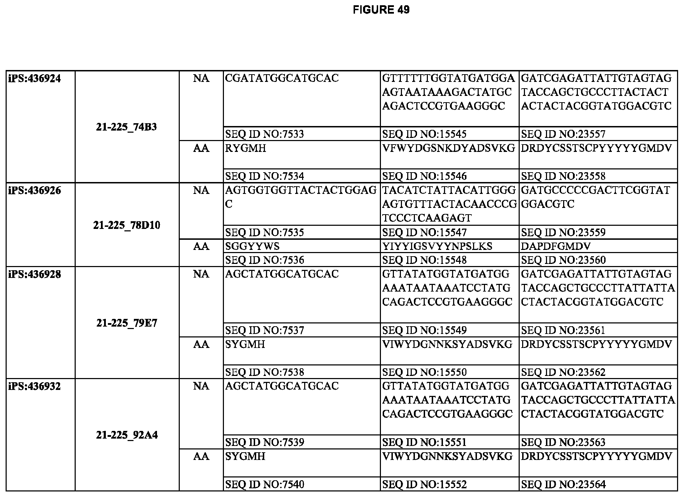

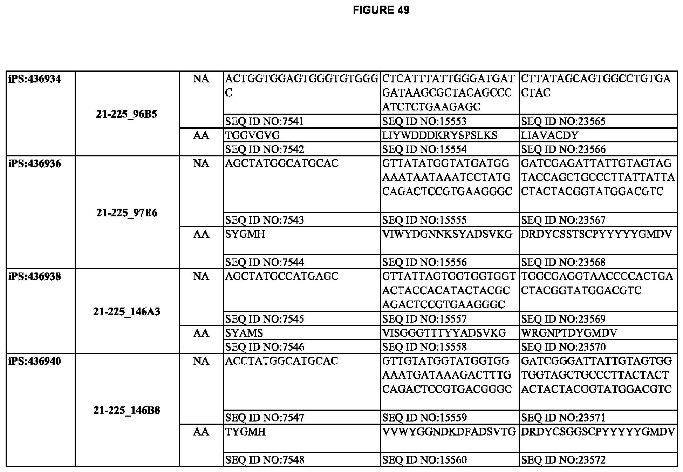

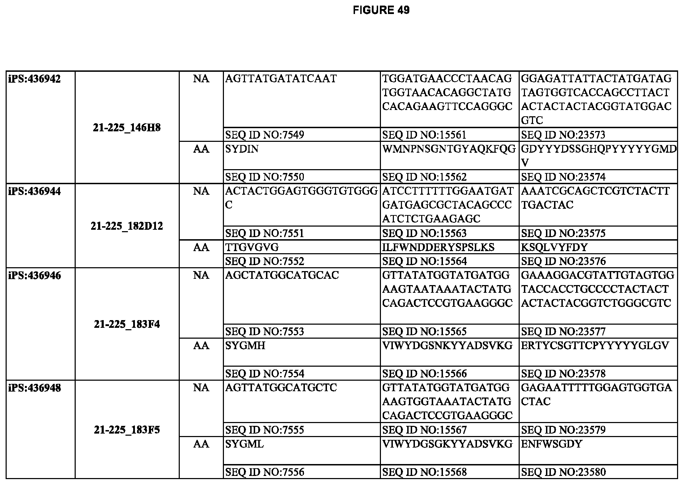

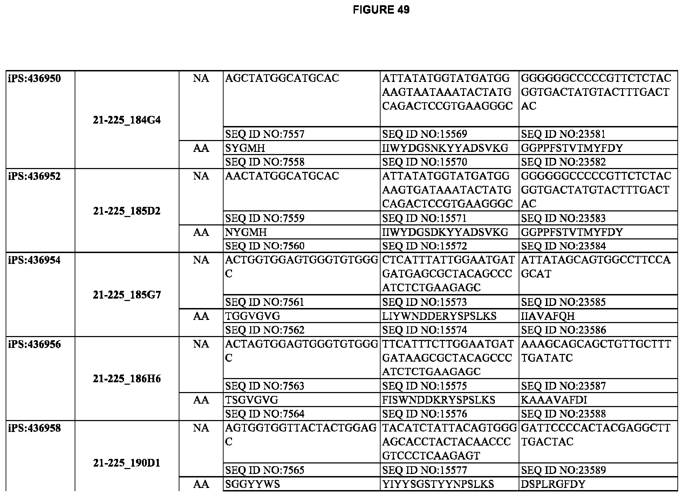

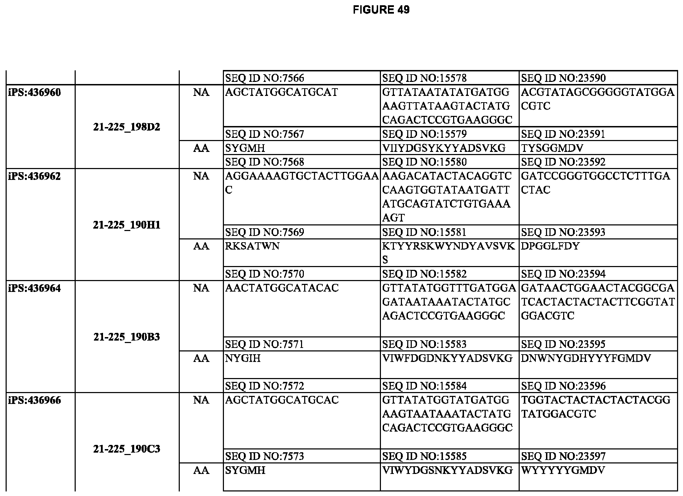

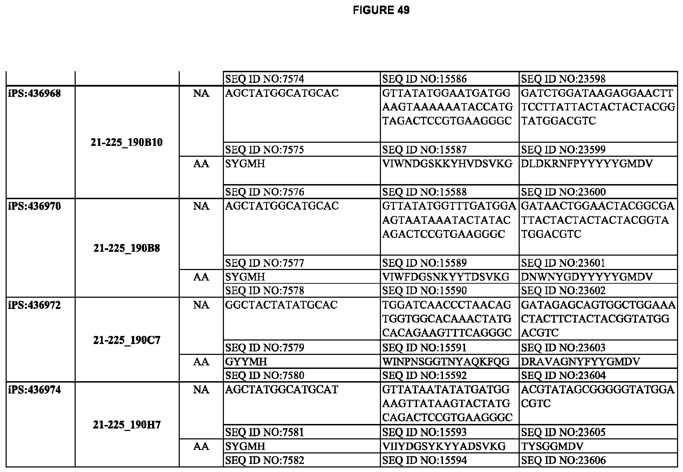

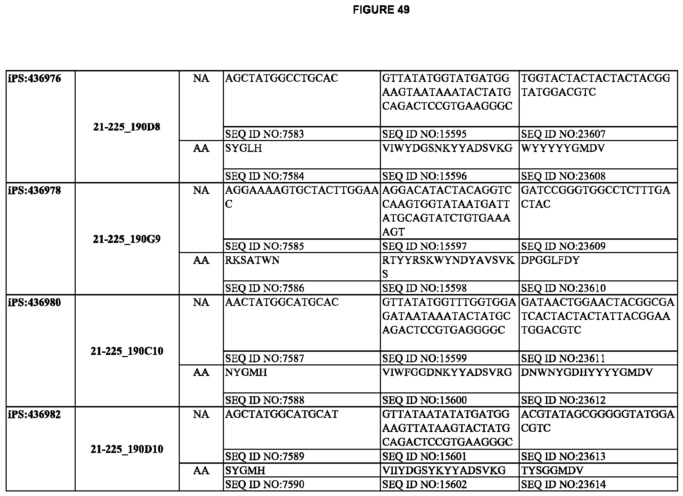

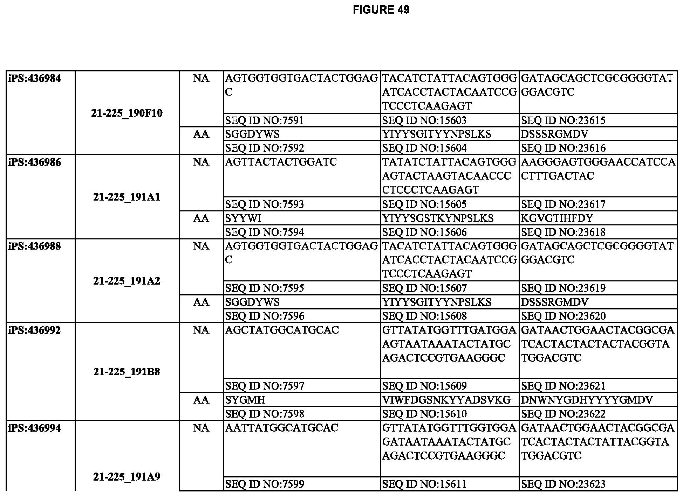

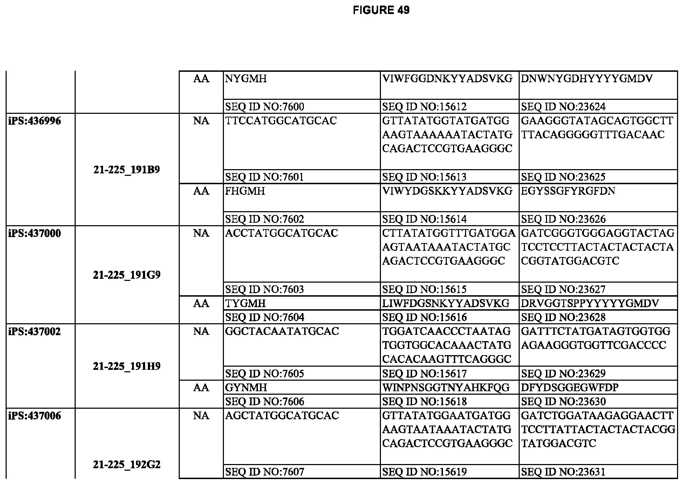

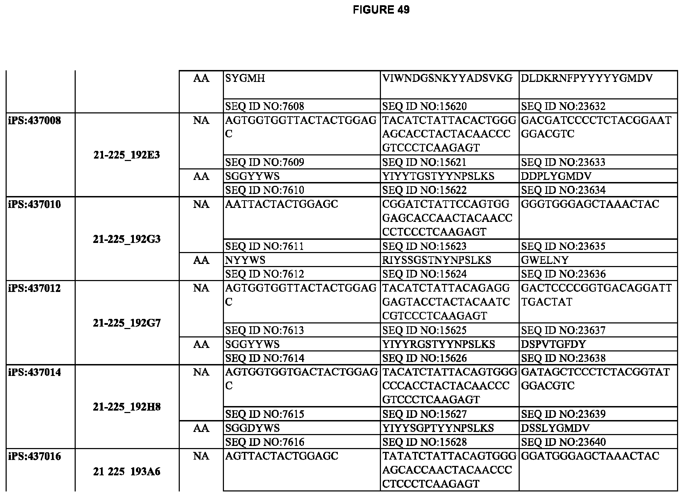

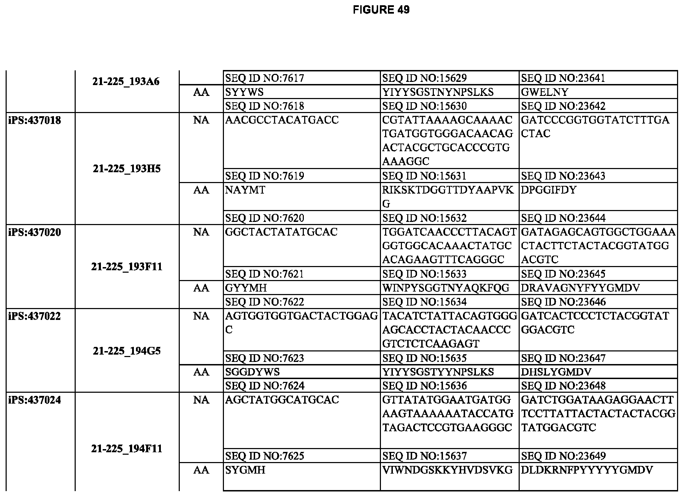

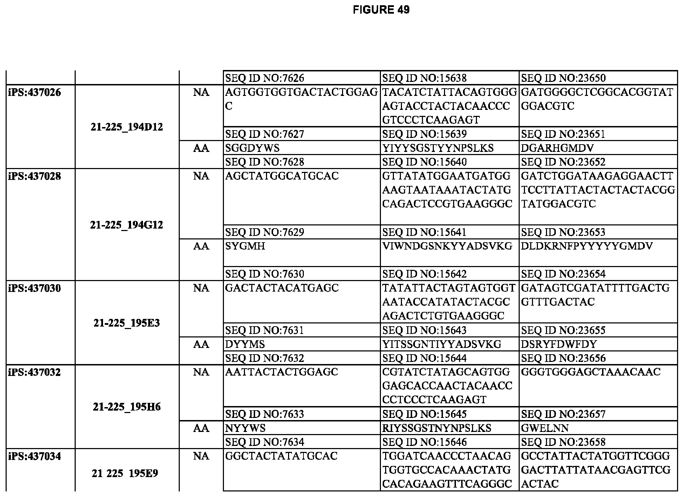

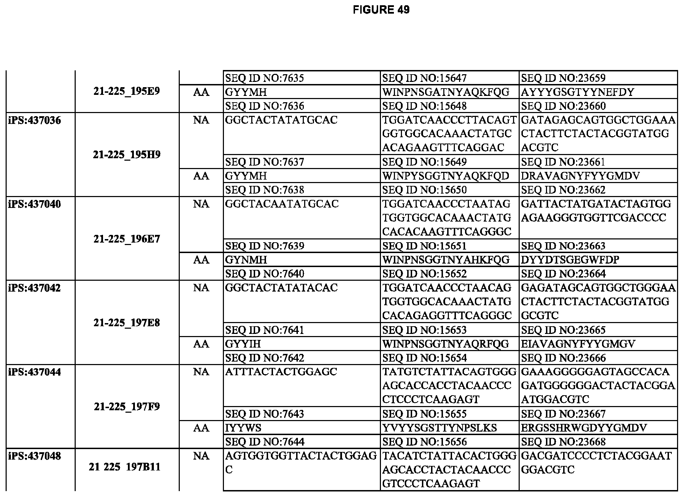

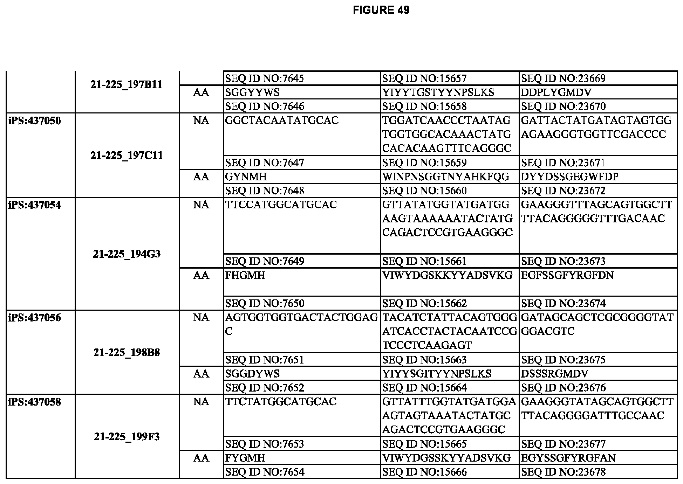

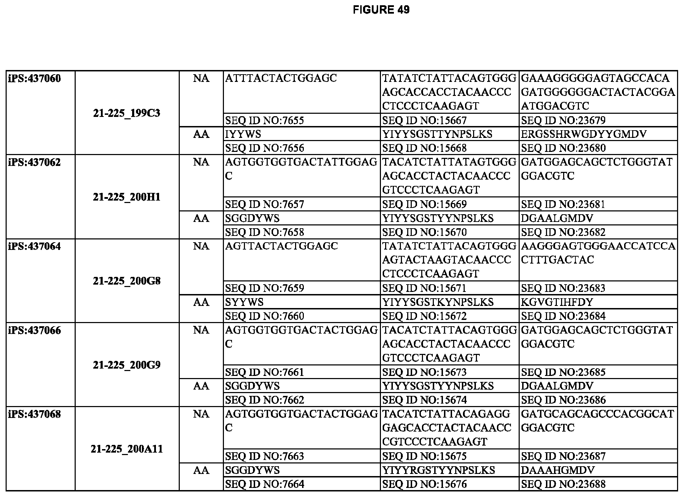

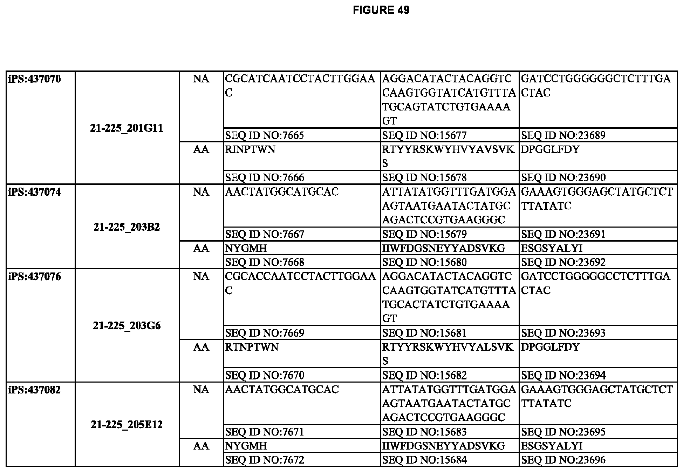

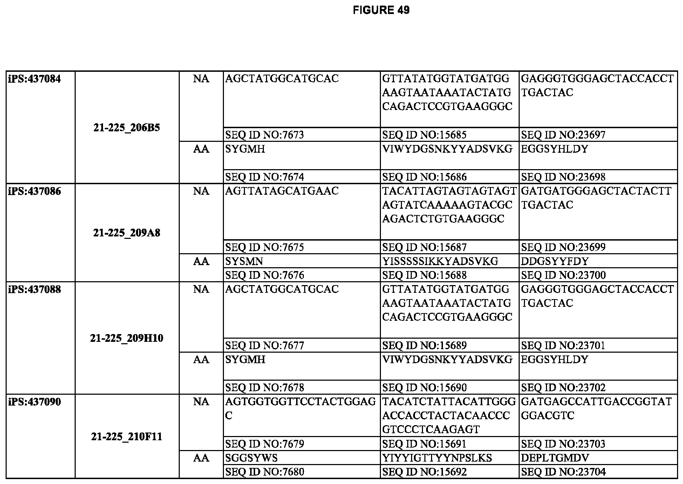

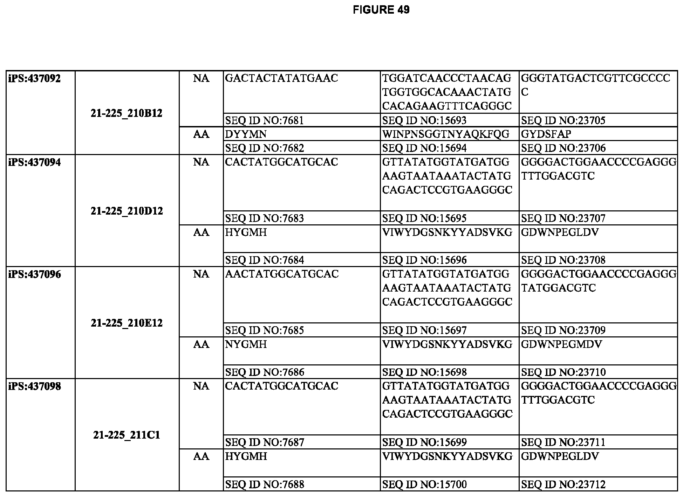

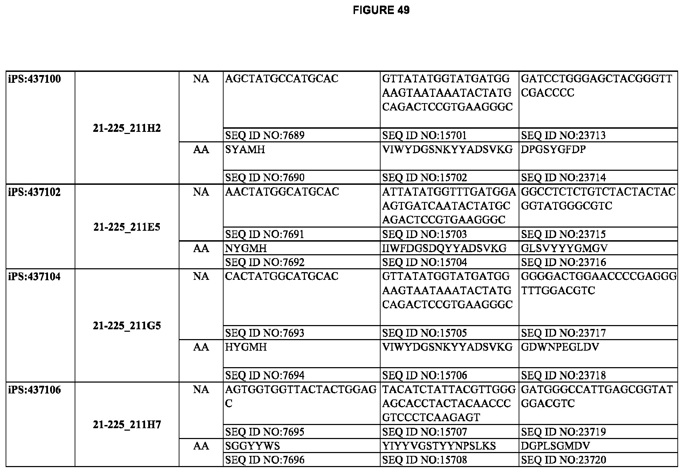

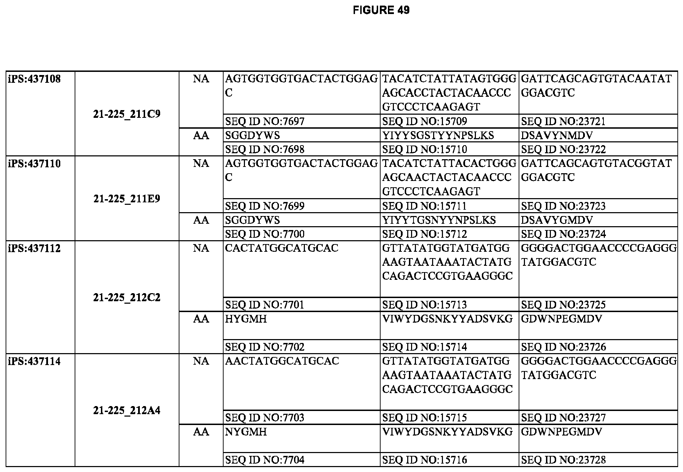

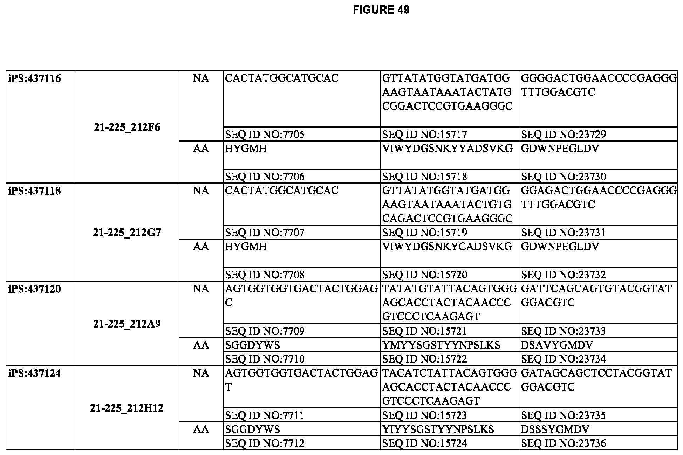

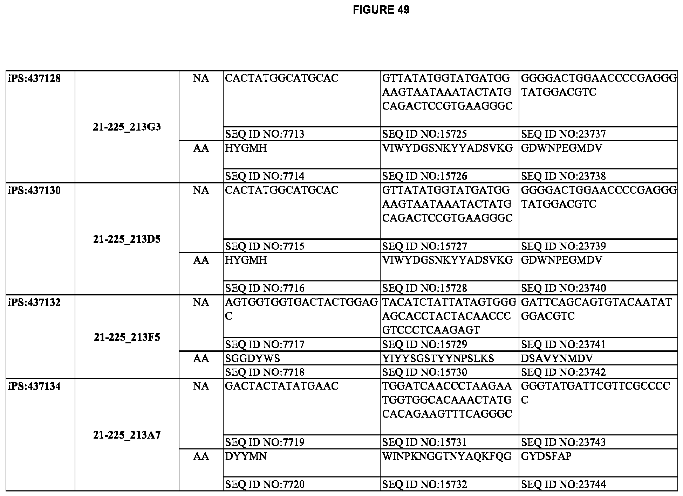

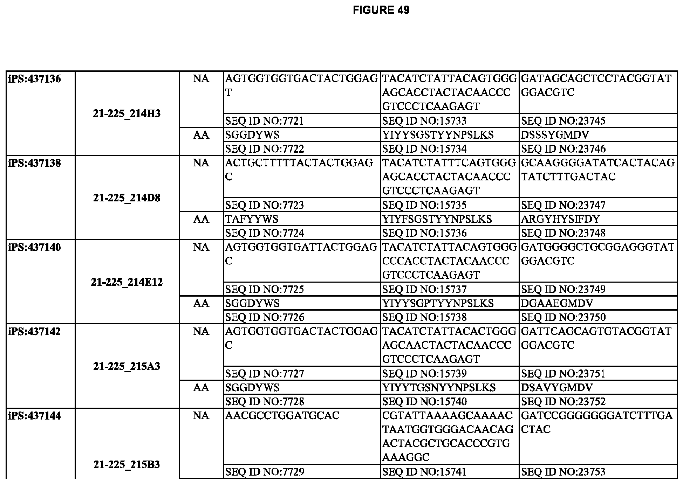

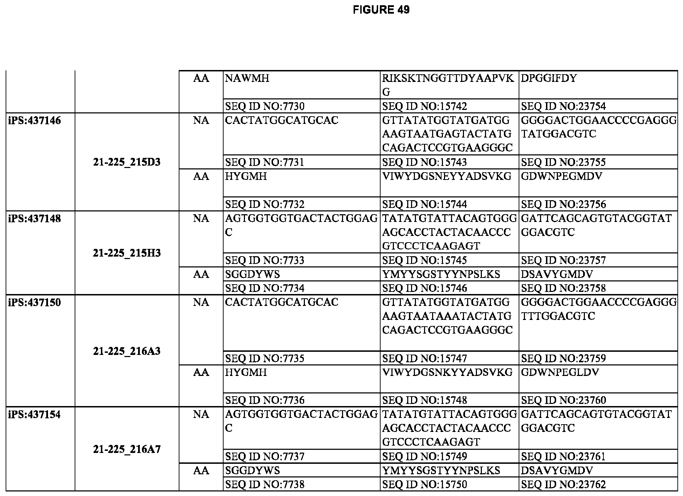

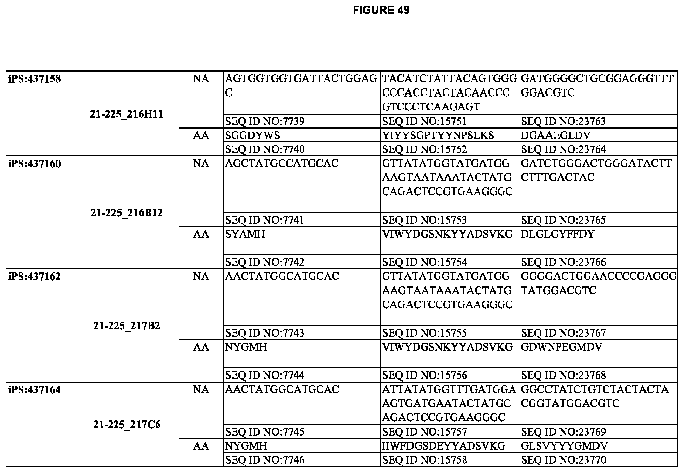

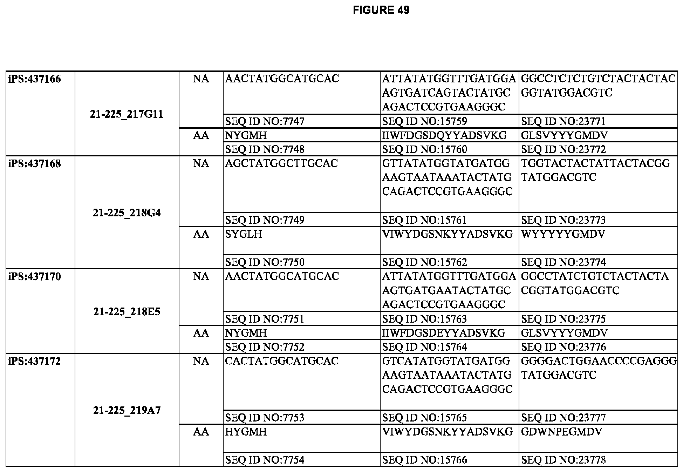

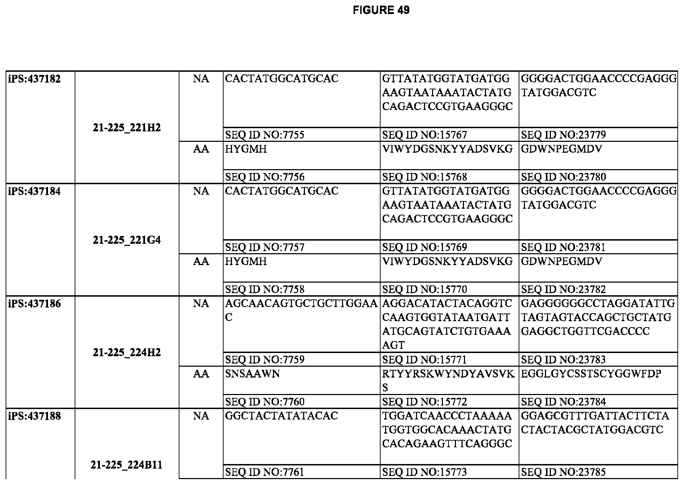

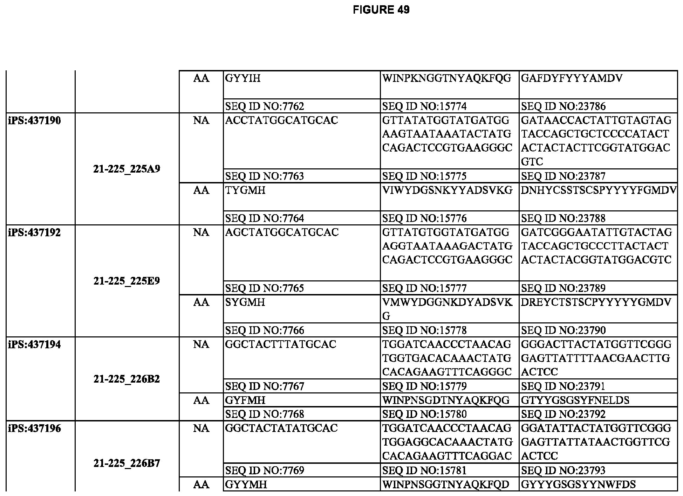

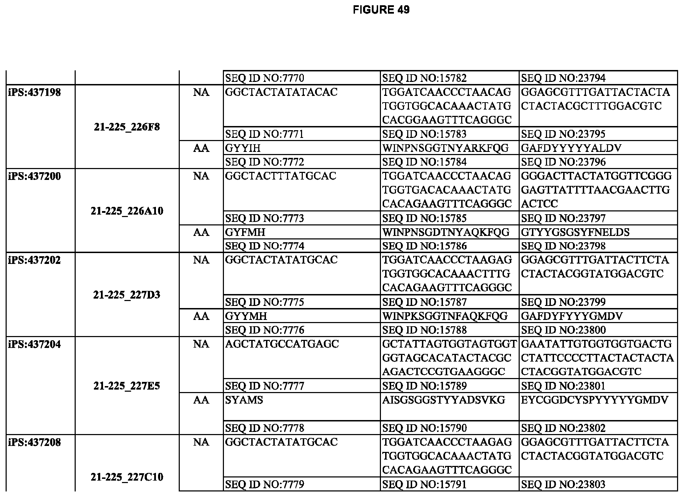

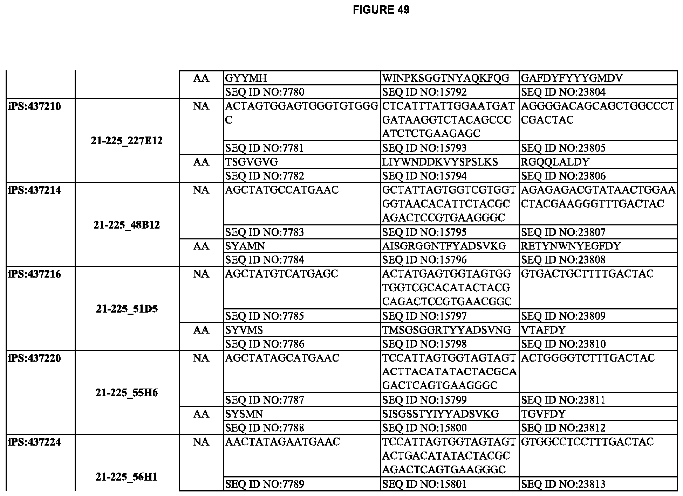

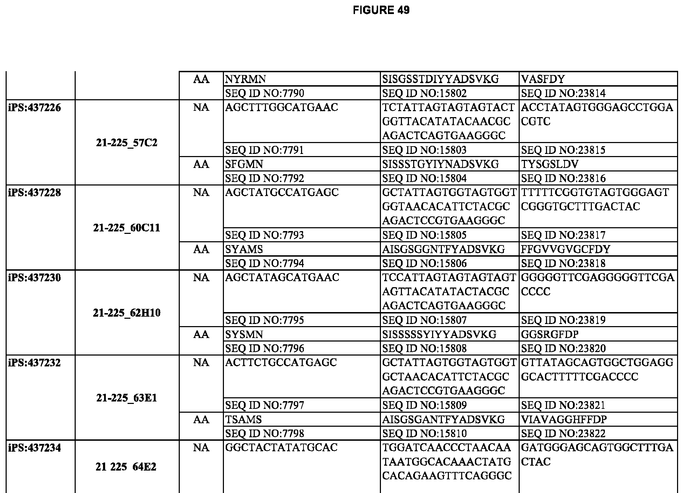

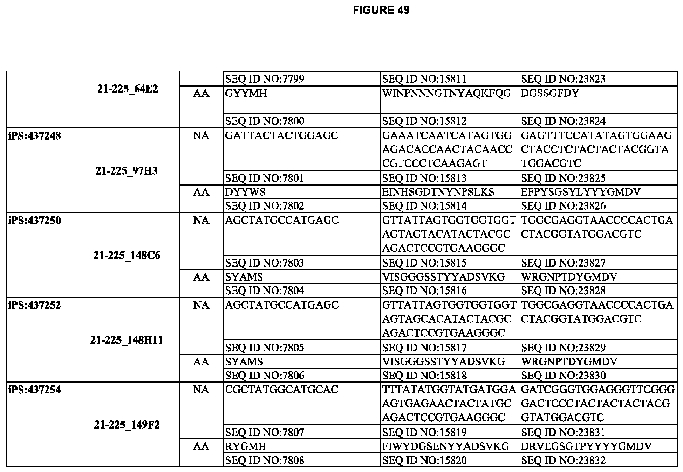

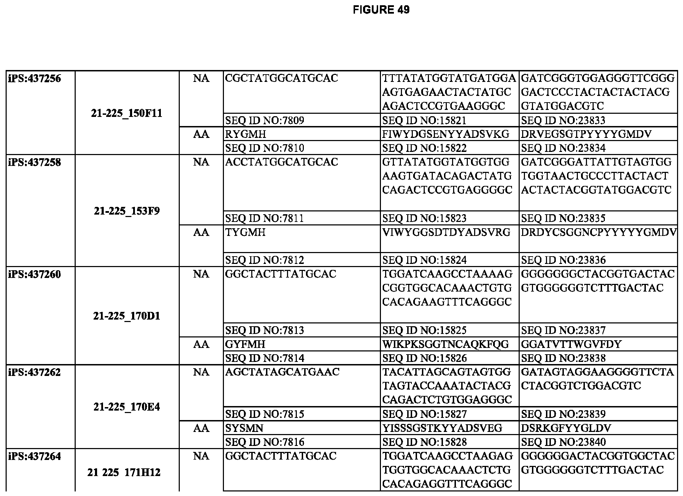

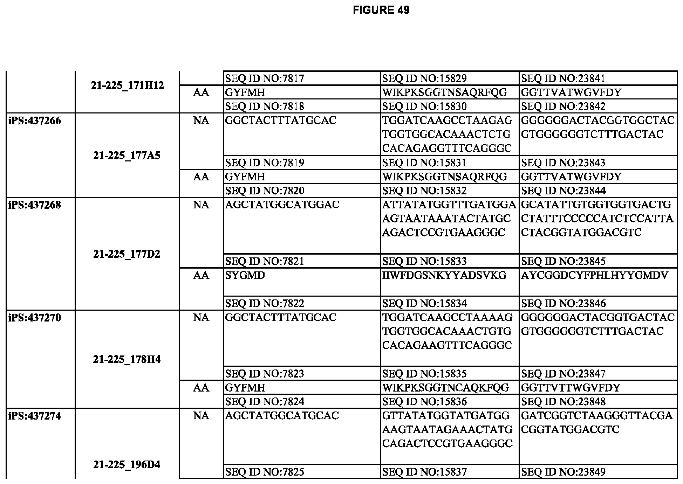

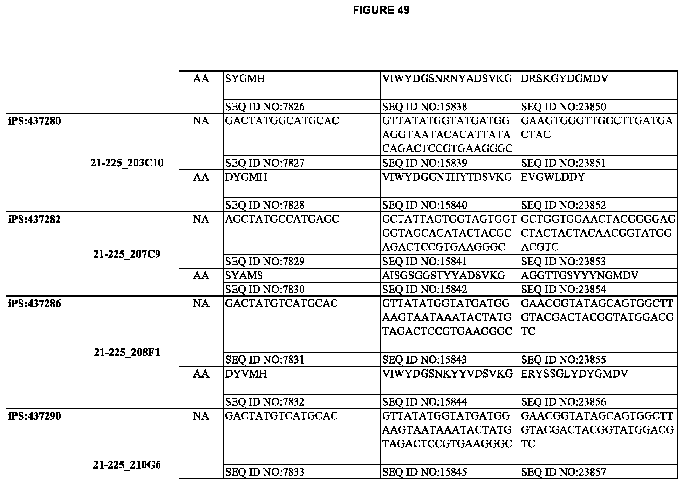

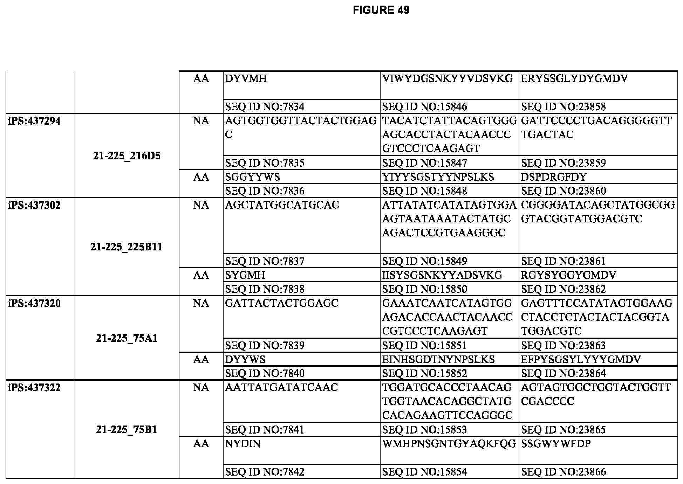

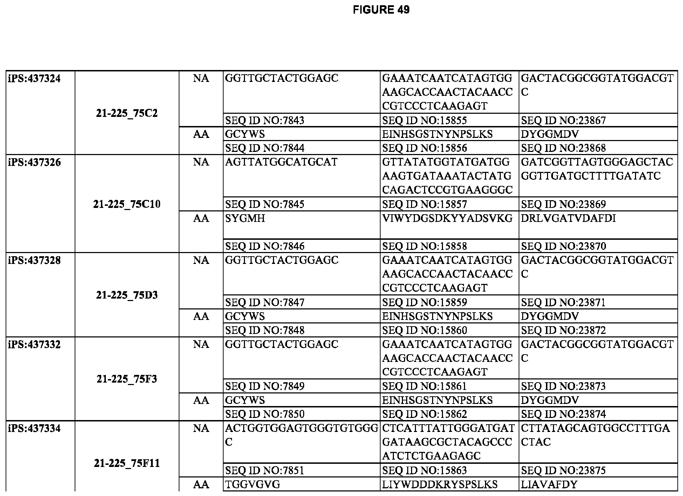

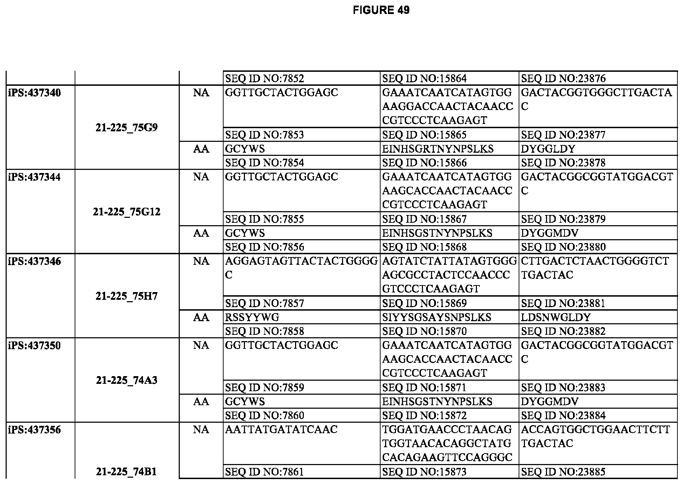

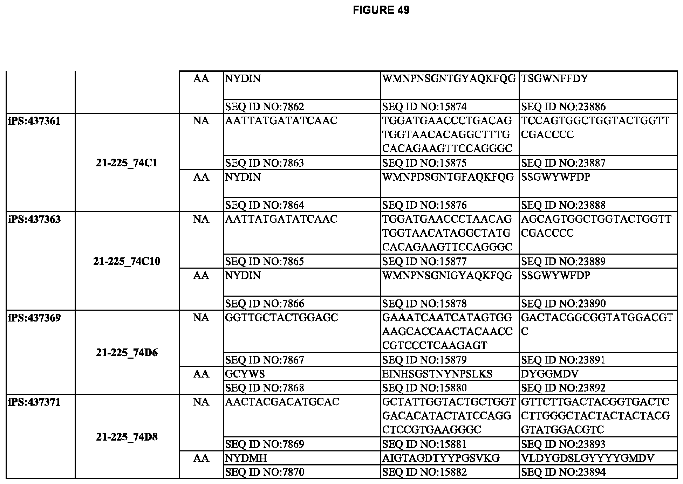

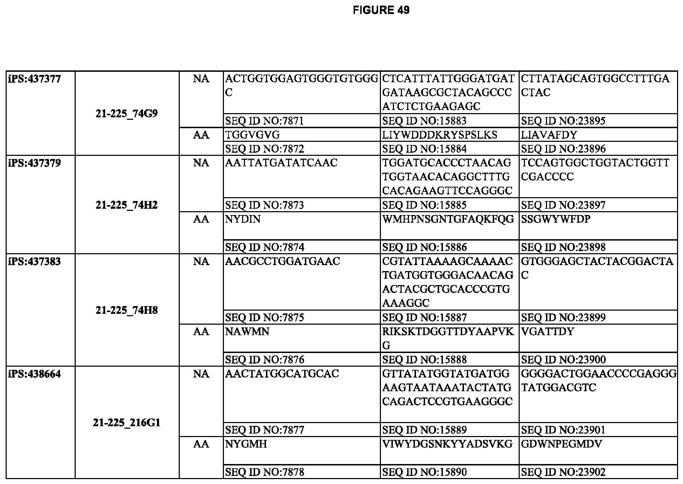

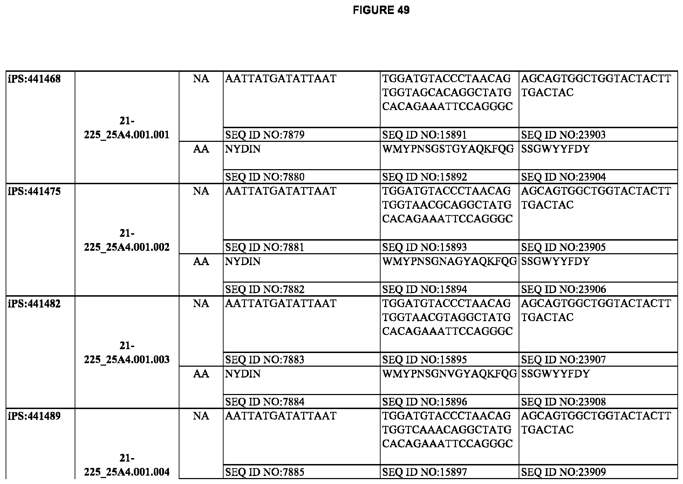

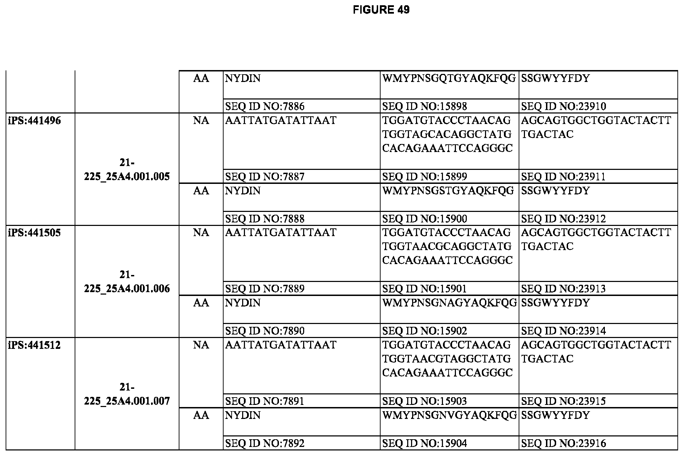

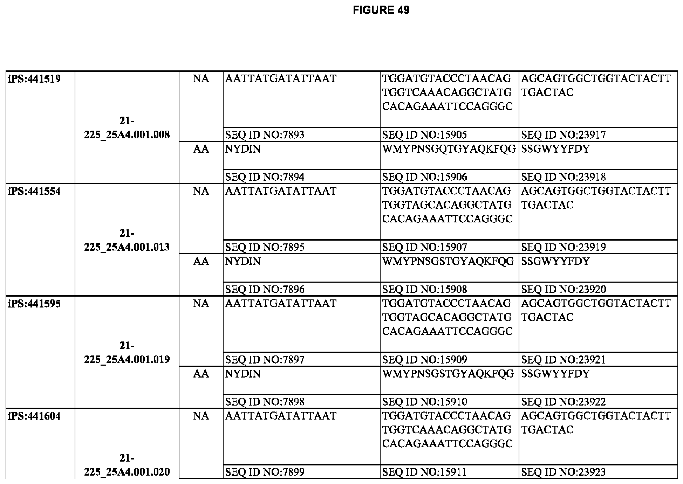

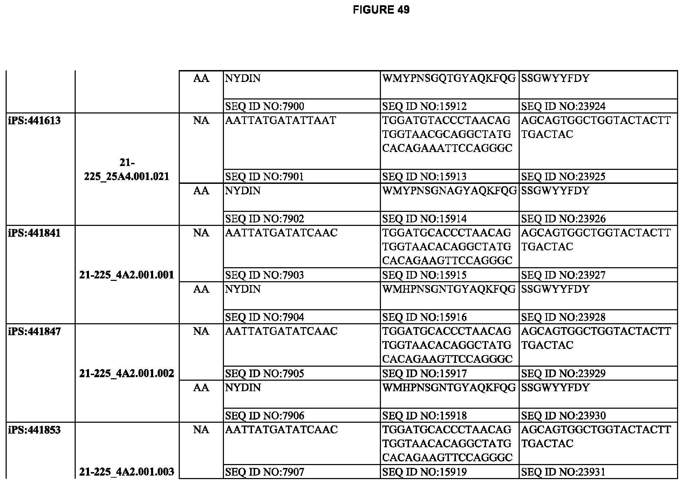

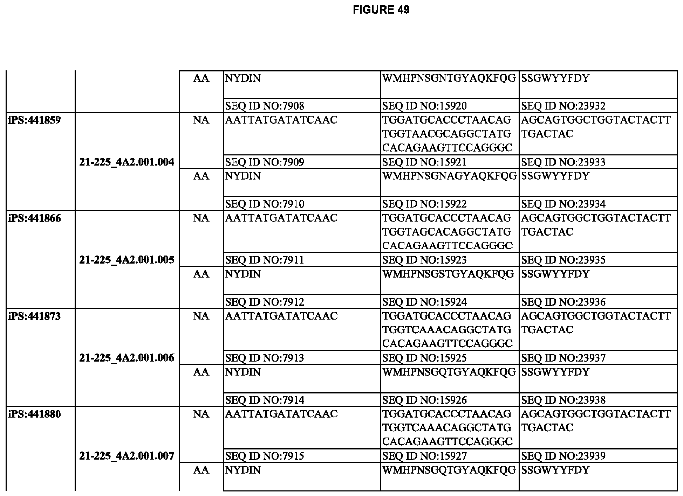

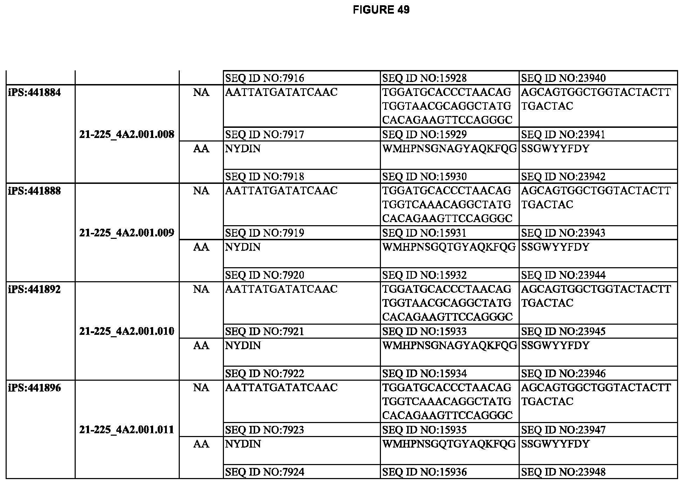

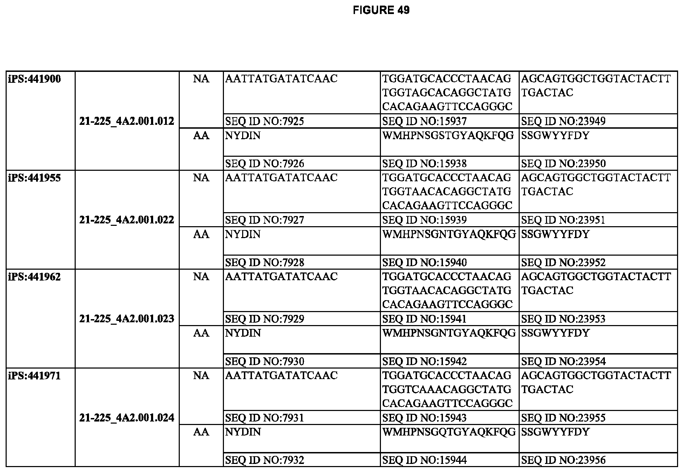

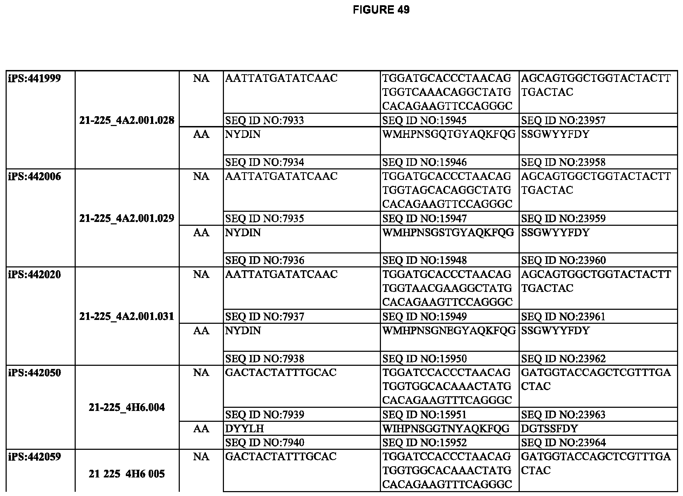

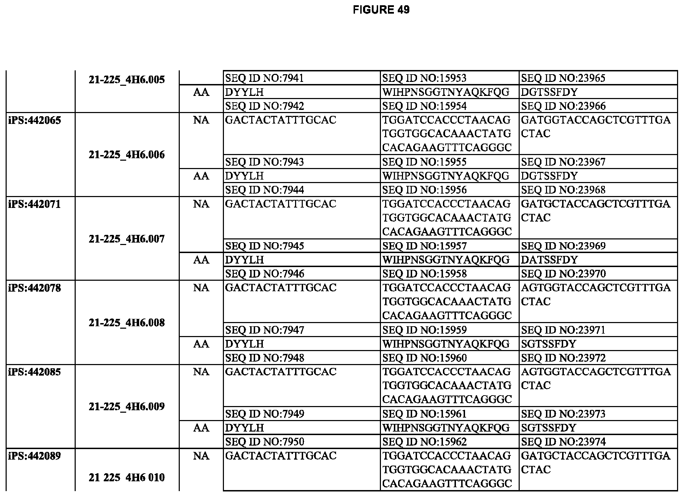

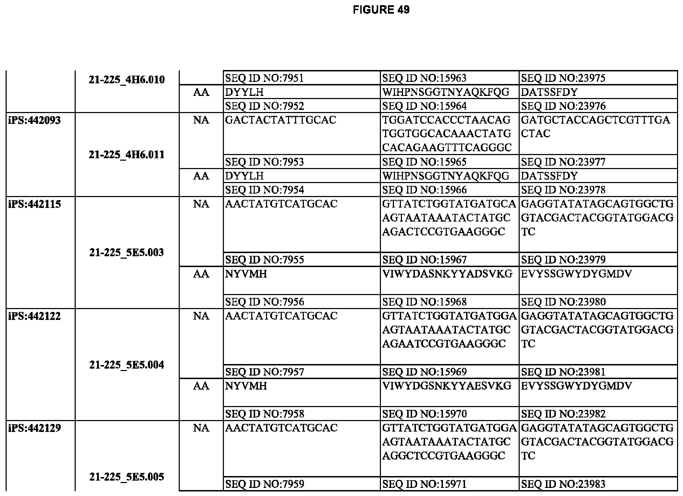

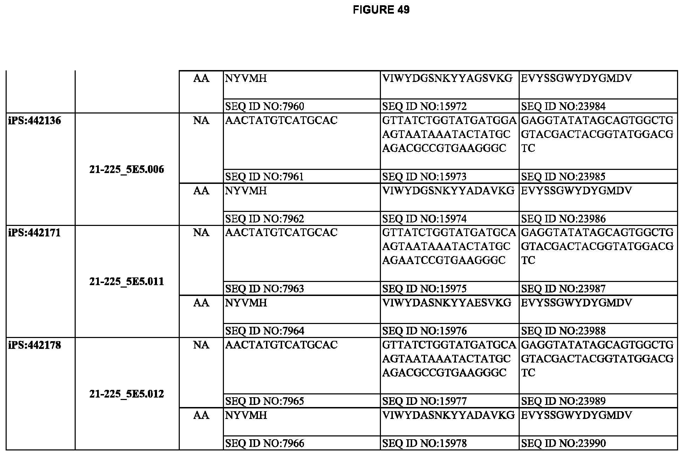

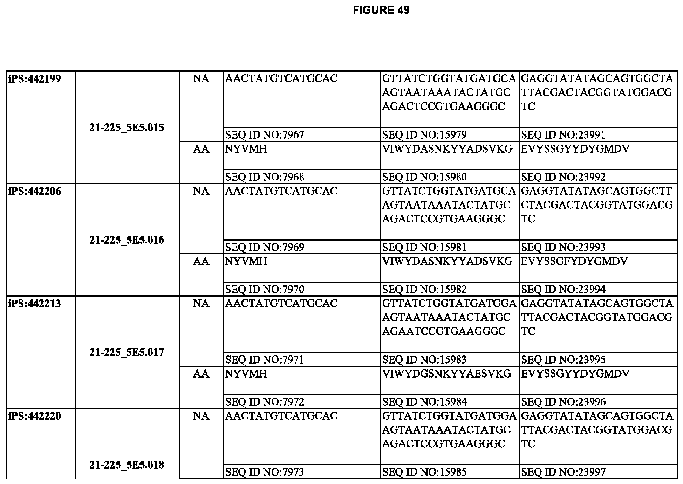

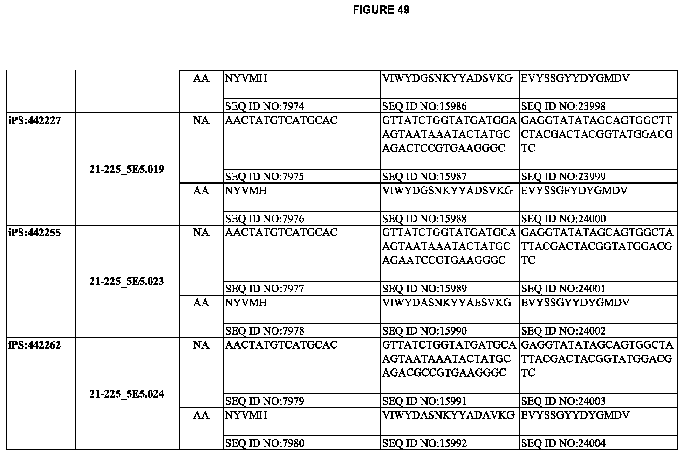

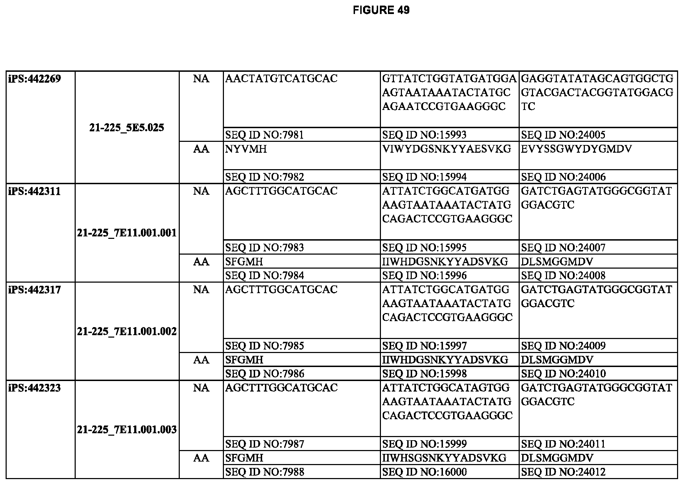

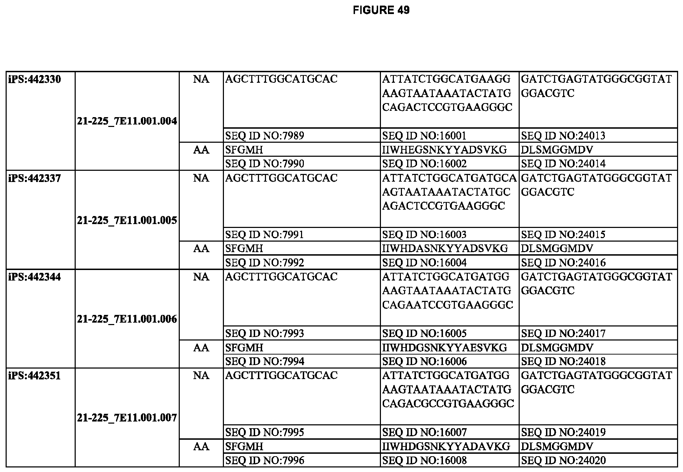

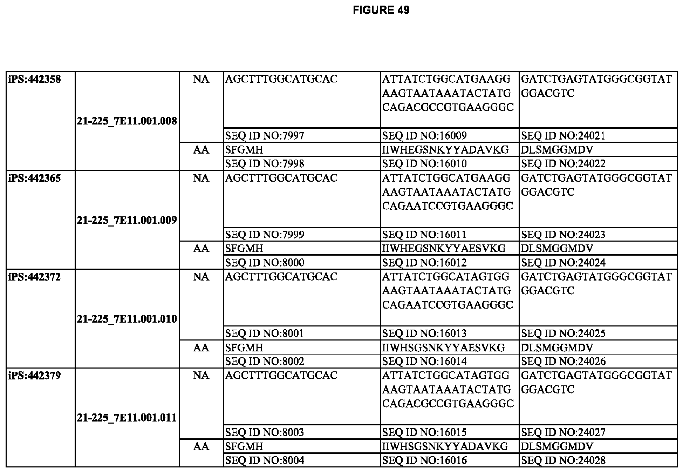

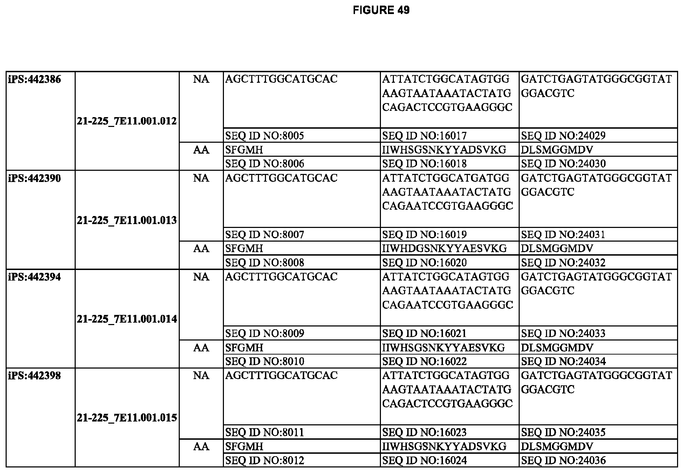

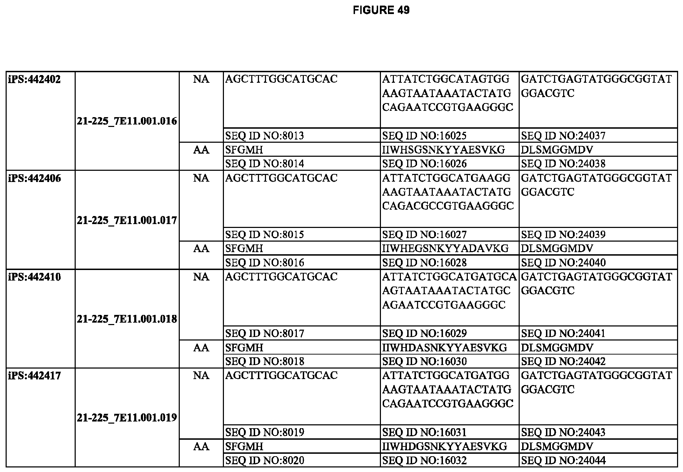

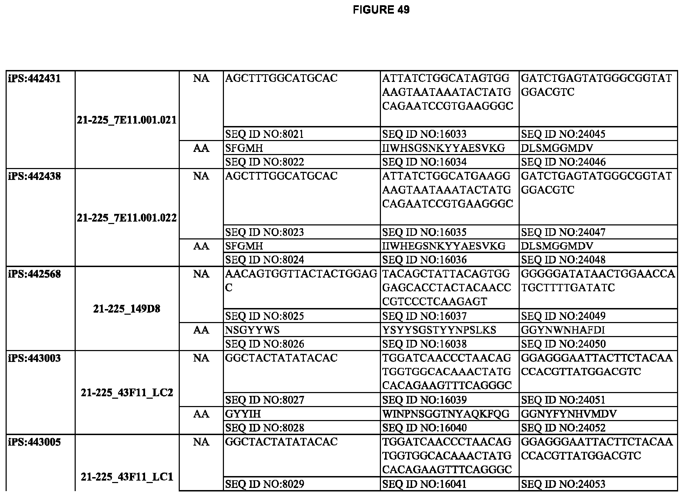

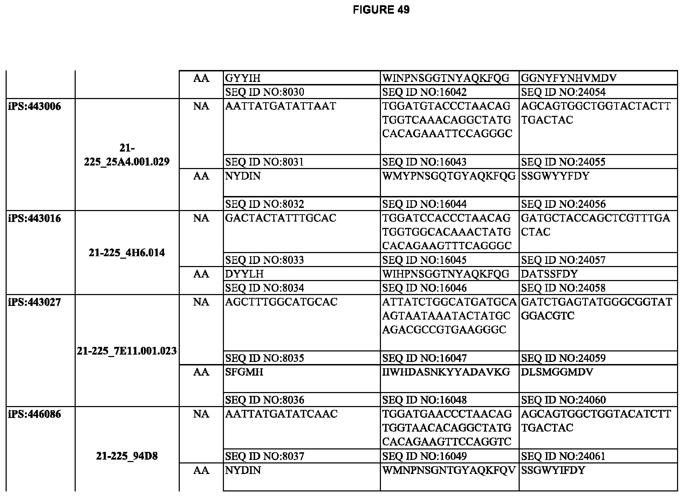

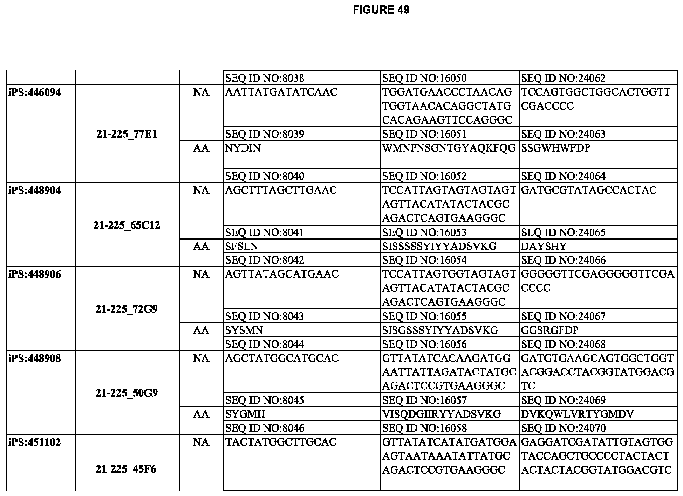

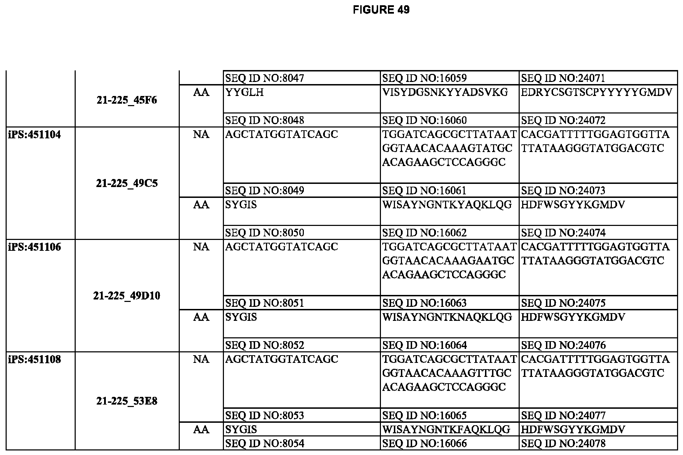

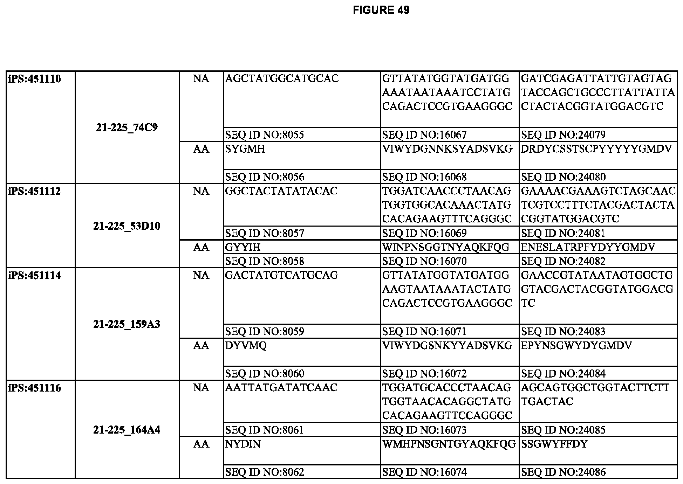

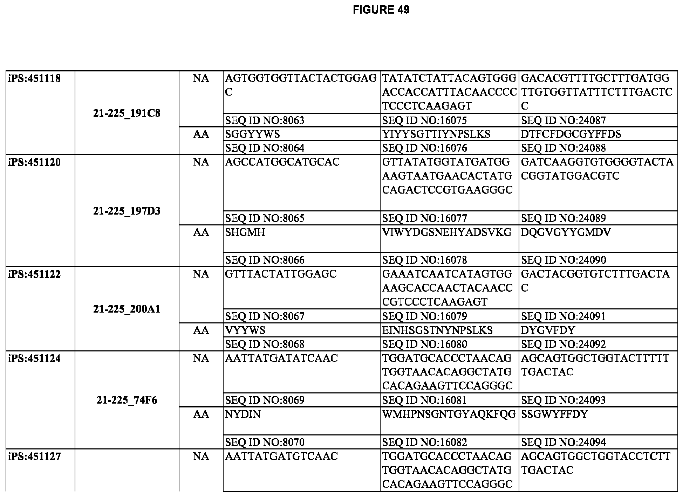

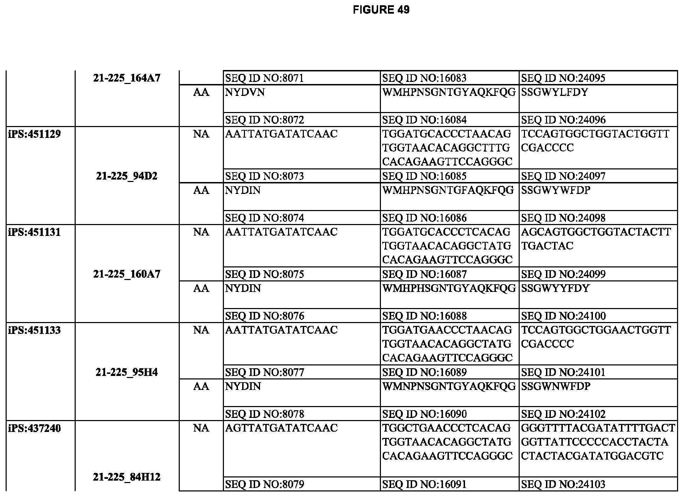

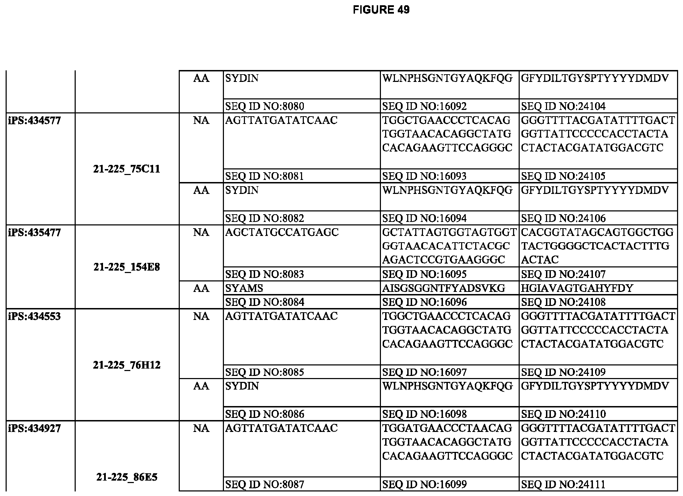

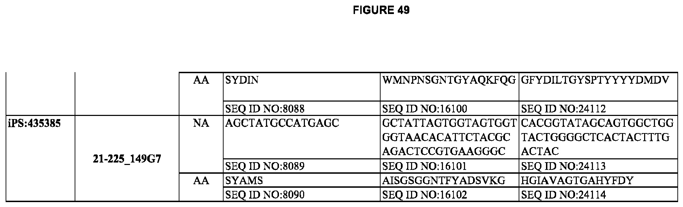

**Figure 49.** Two tables presenting variable light and heavy chain CDR1, CDR2 and CDR3 amino acid sequences for certain antigen binding proteins of the present invention (Table 2A and Table 2B). Table 2A presents the Variable Light Chain CDR1, CDR2 and CDR3, while Table 2B presents the Variable Heavy Chain CDR1, CDR2, and CDR3. The CDR sequences in Tables 2A and 2B are wrapped due to space issues, and unless stated otherwise, should be understood to be a single amino acid sequence.

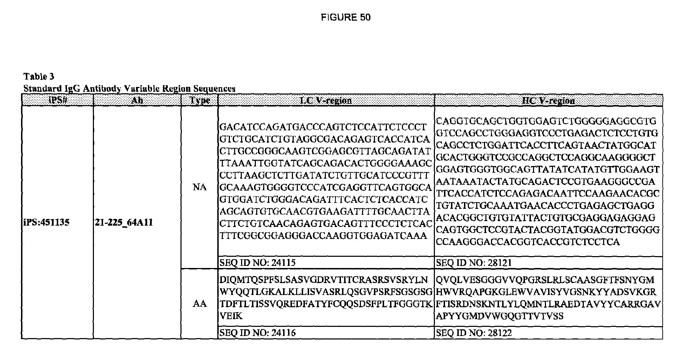

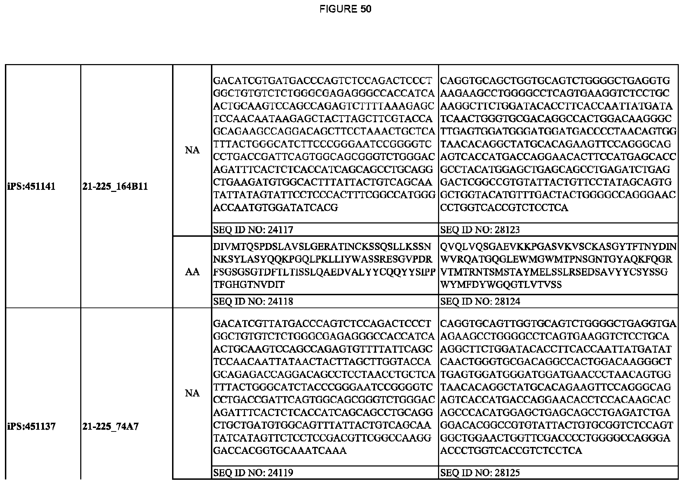

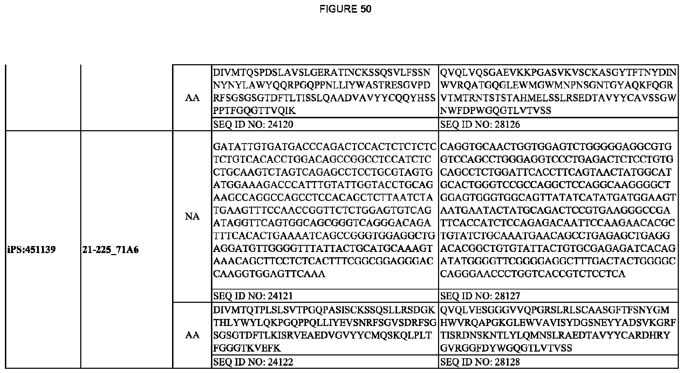

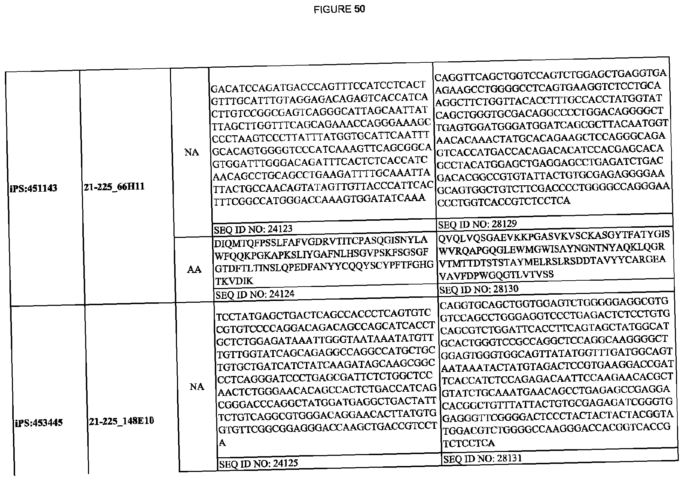

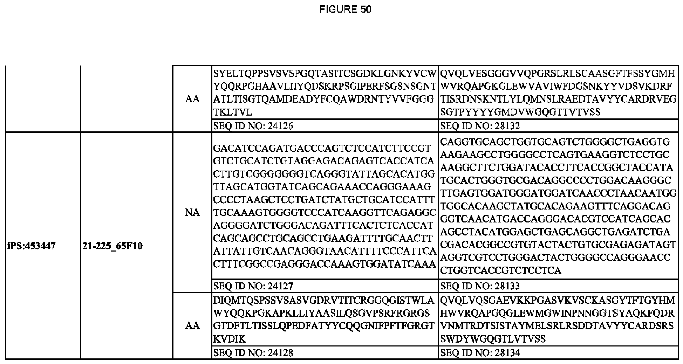

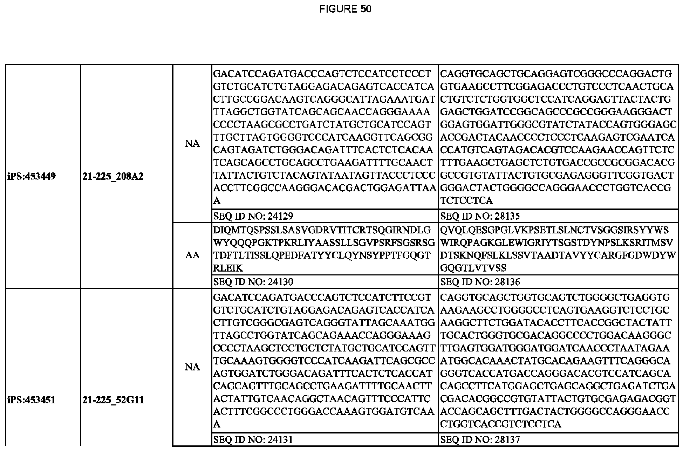

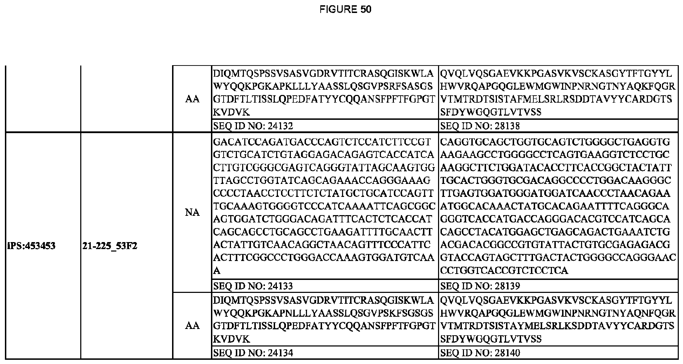

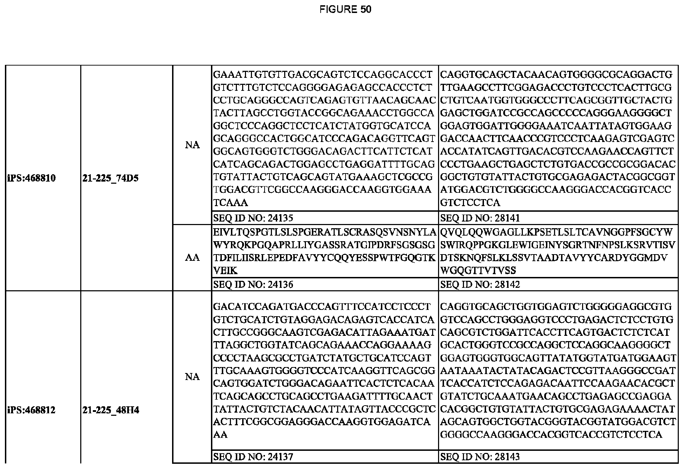

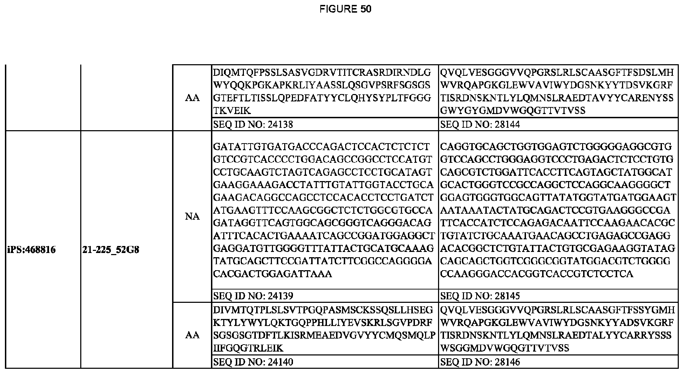

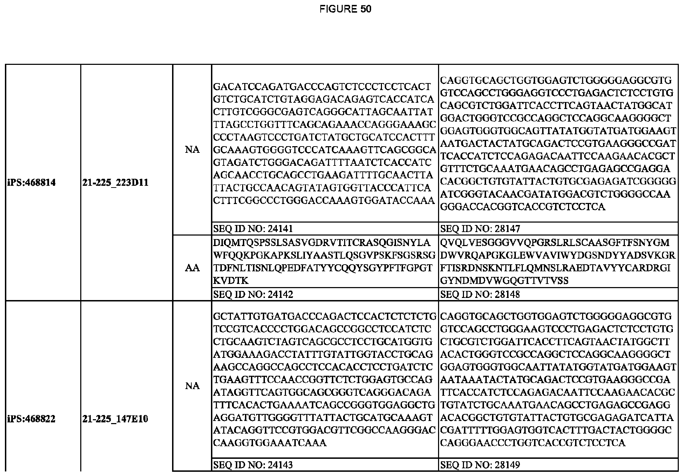

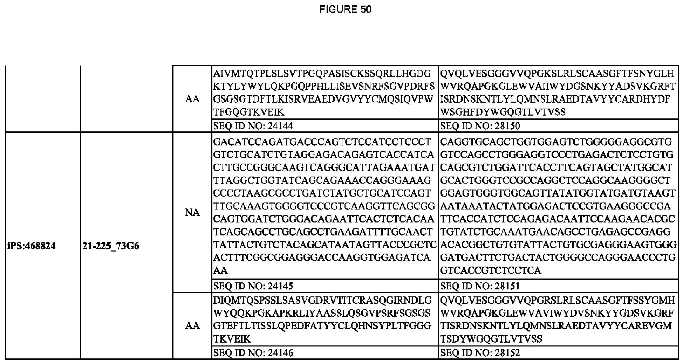

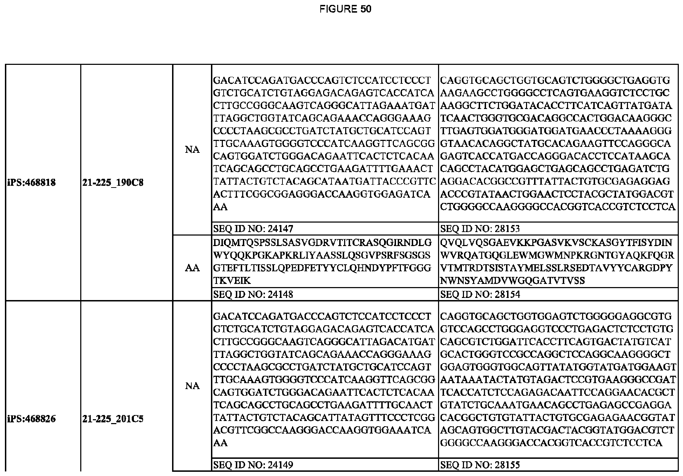

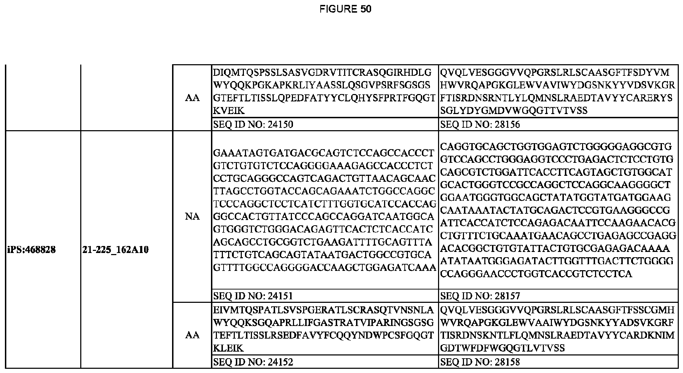

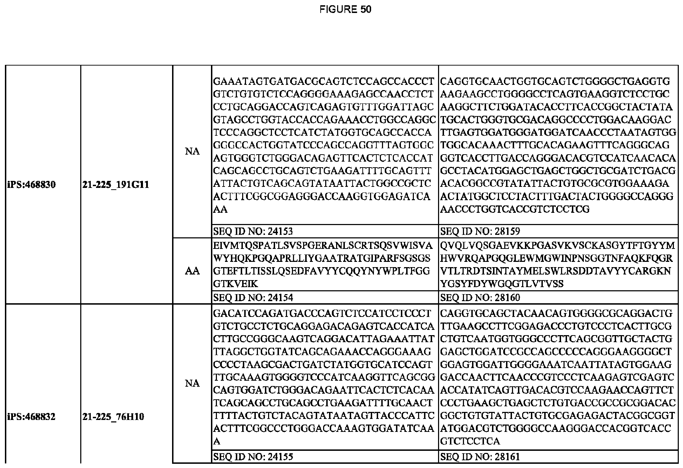

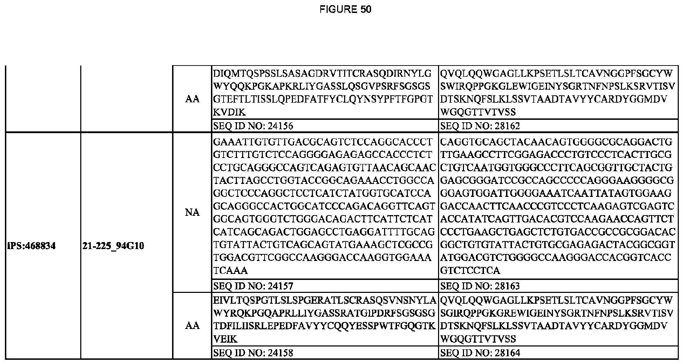

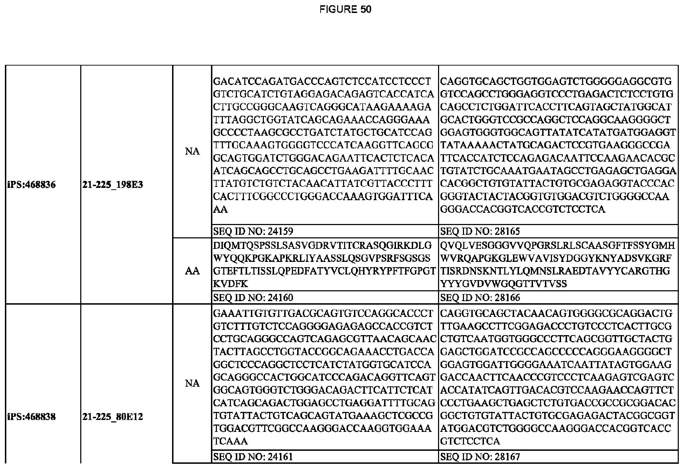

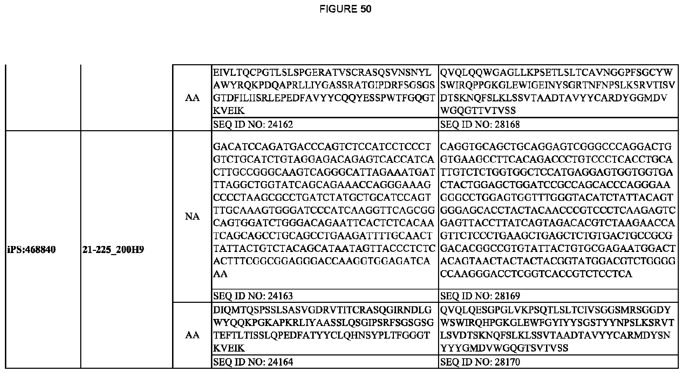

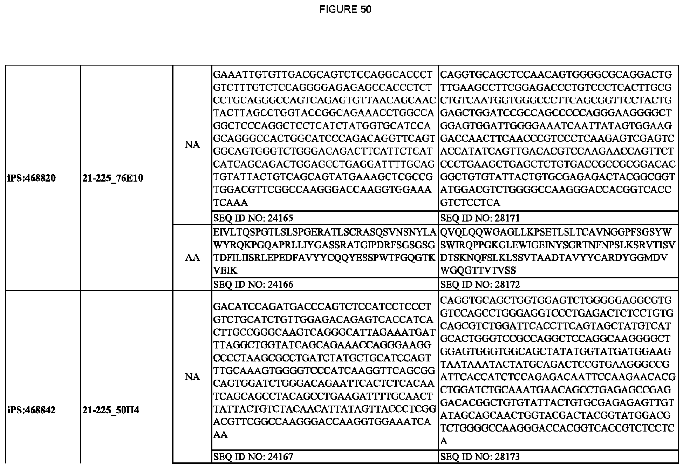

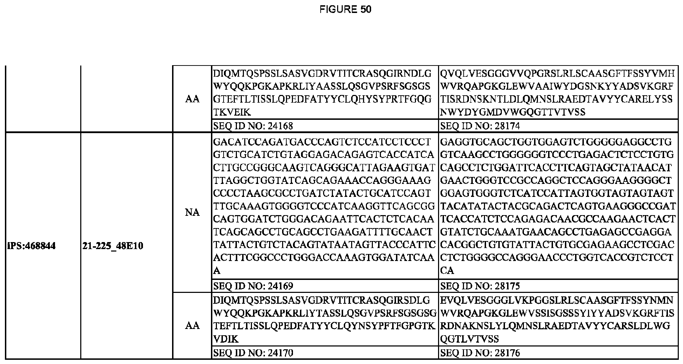

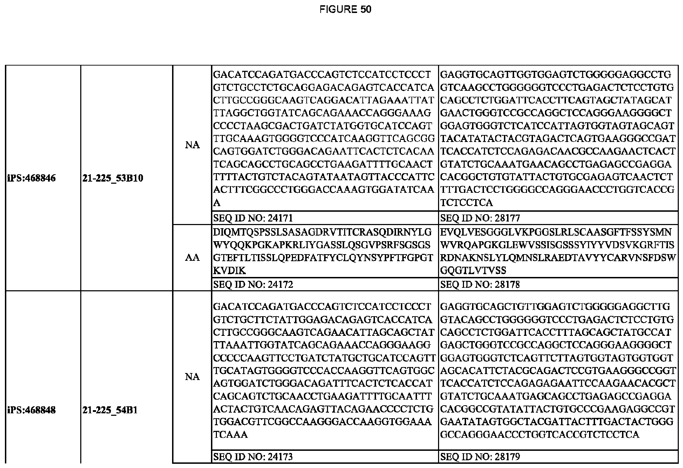

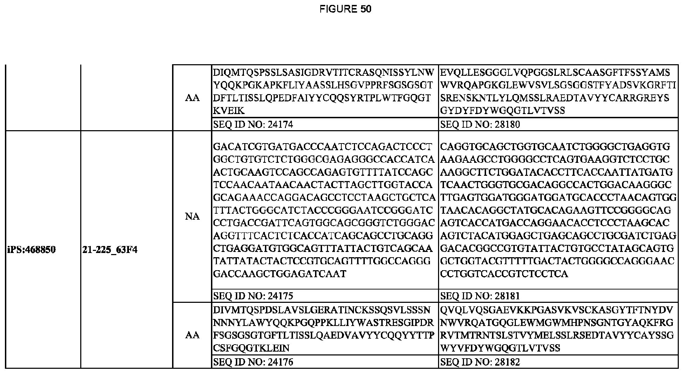

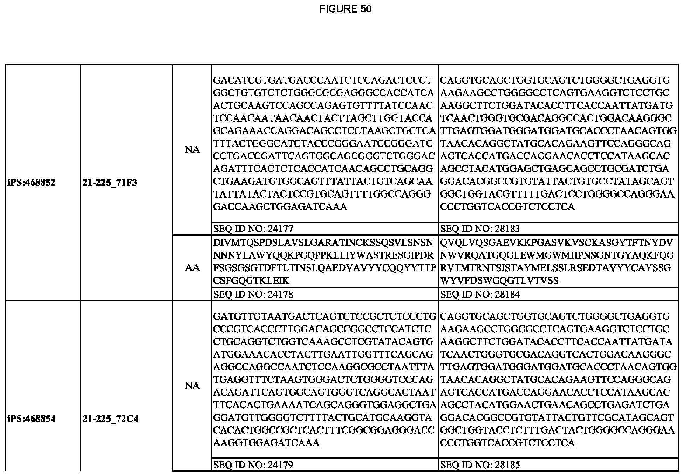

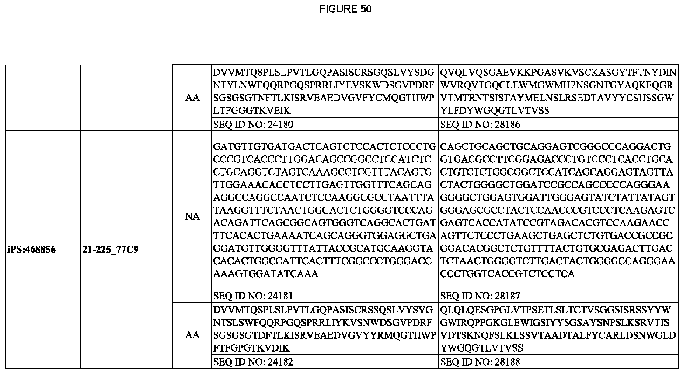

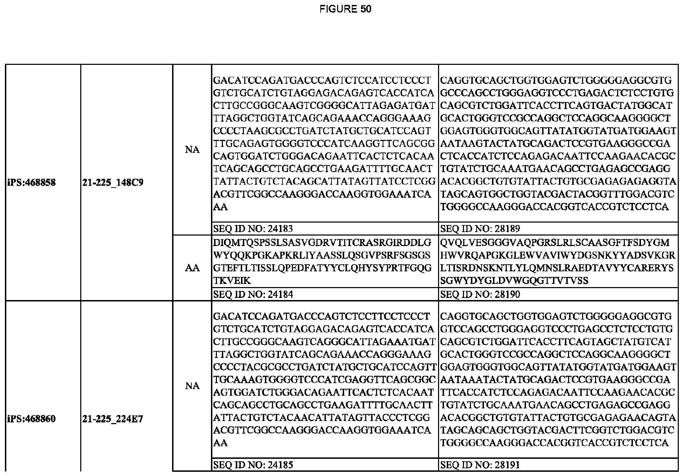

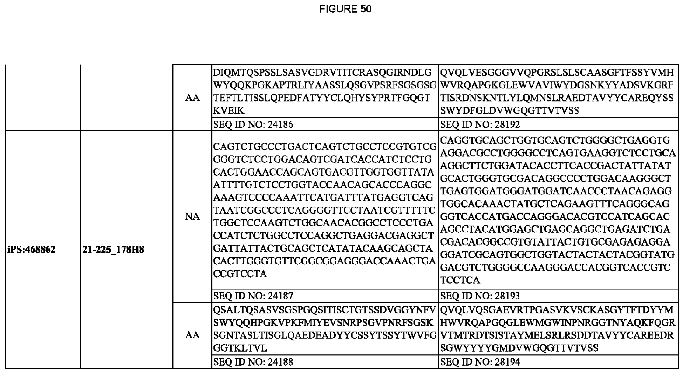

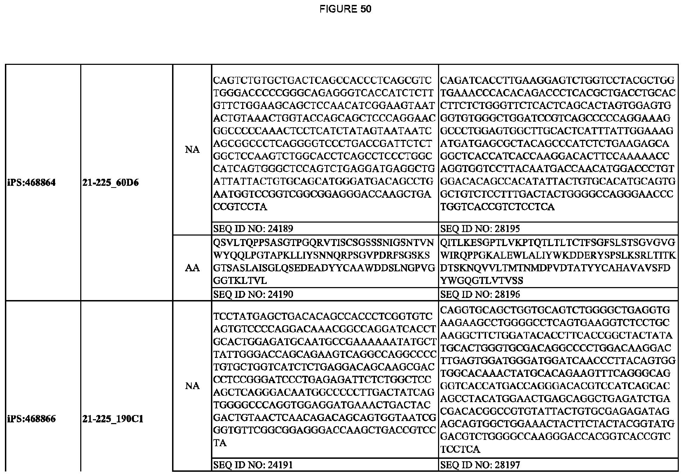

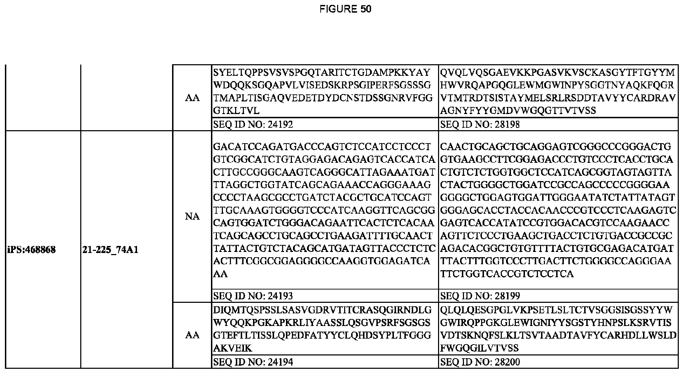

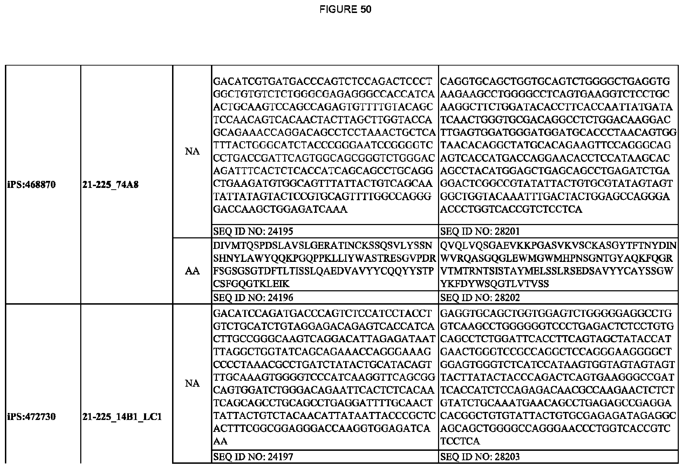

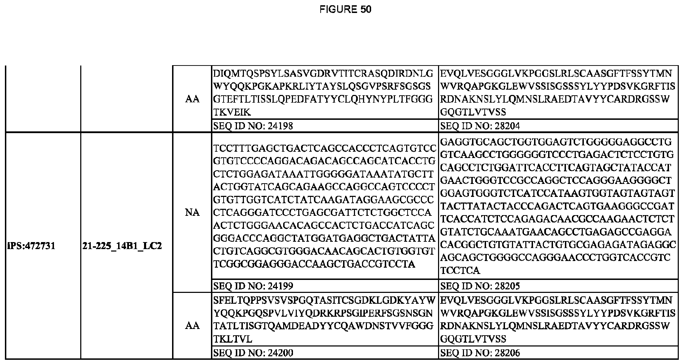

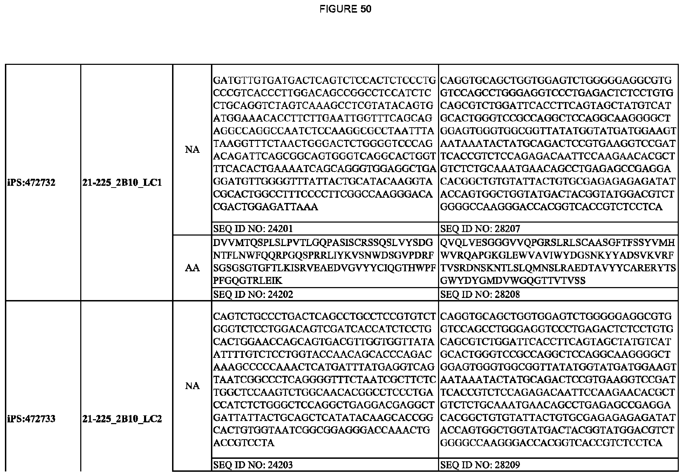

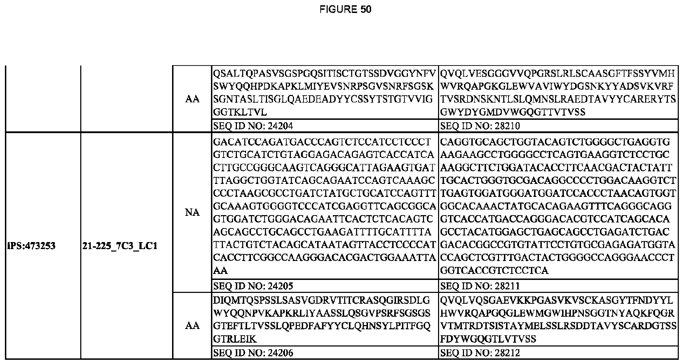

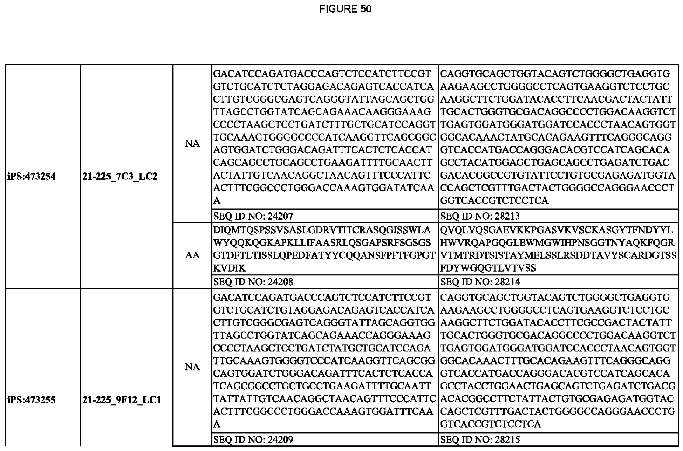

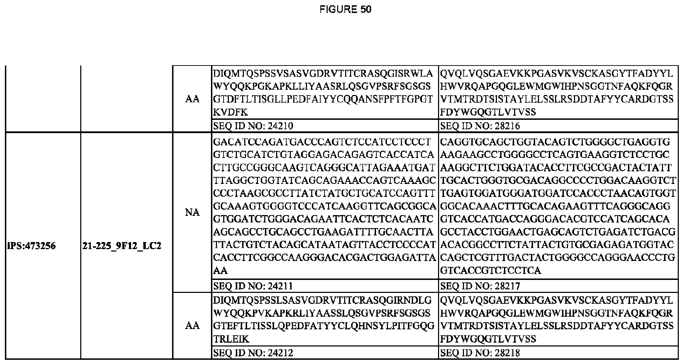

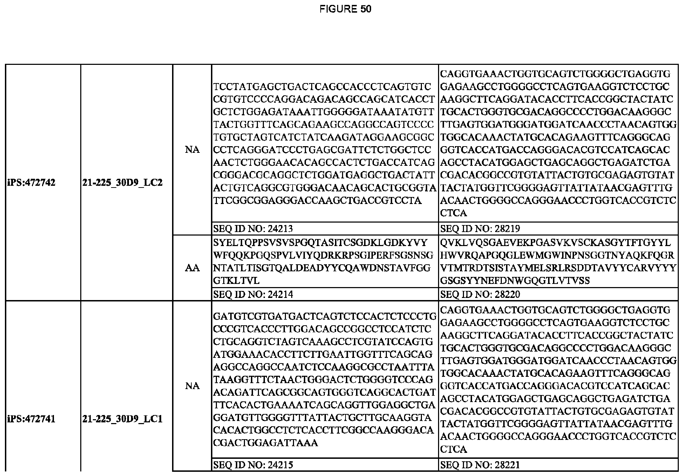

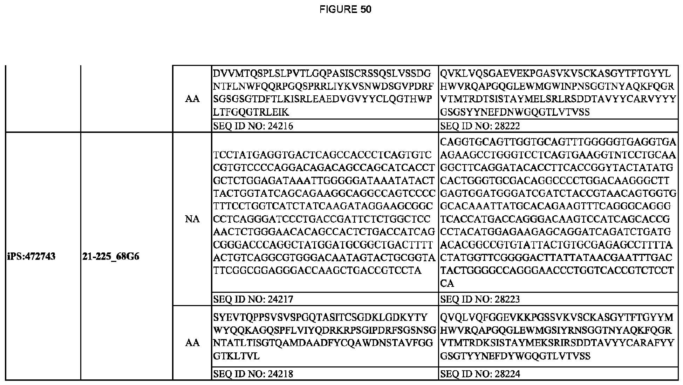

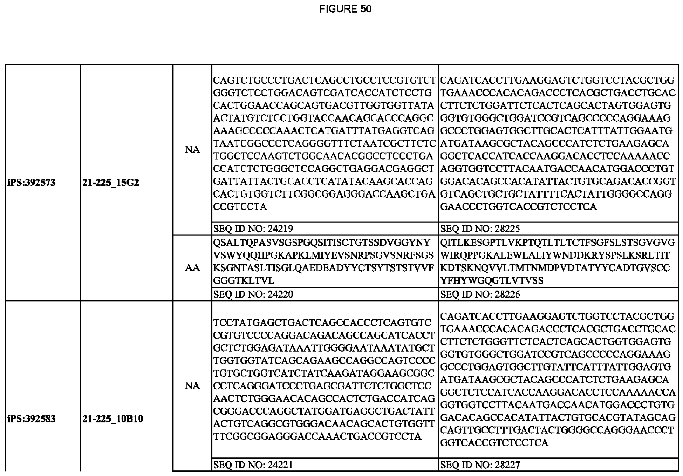

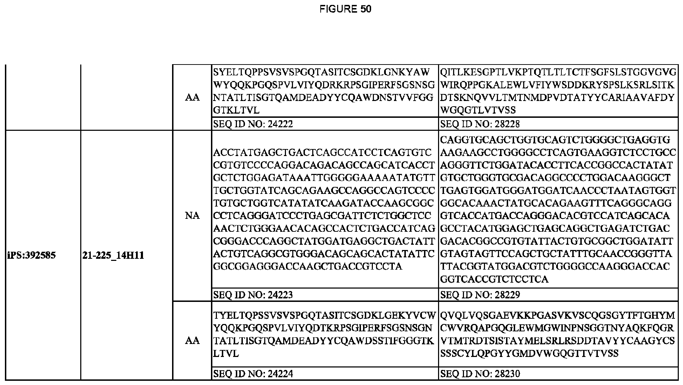

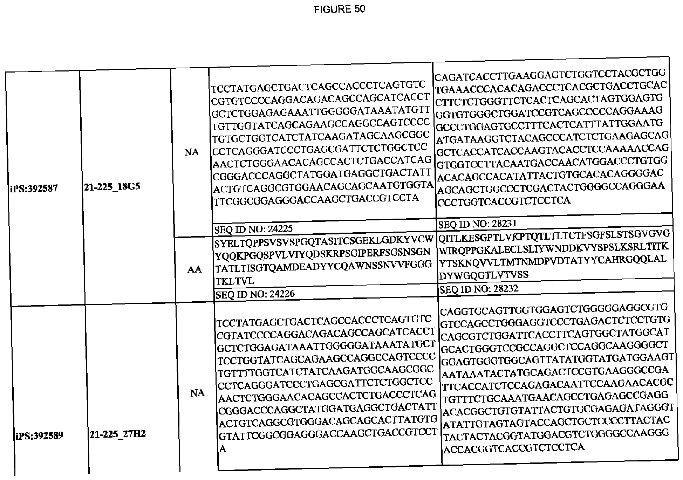

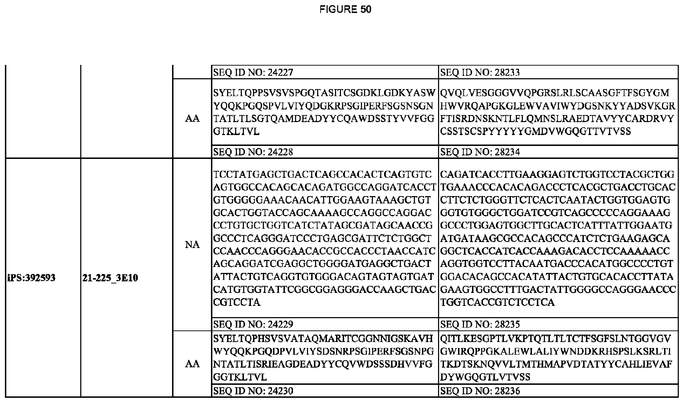

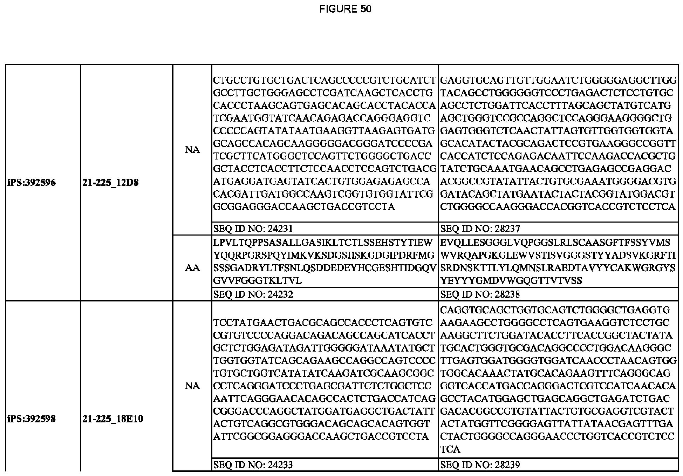

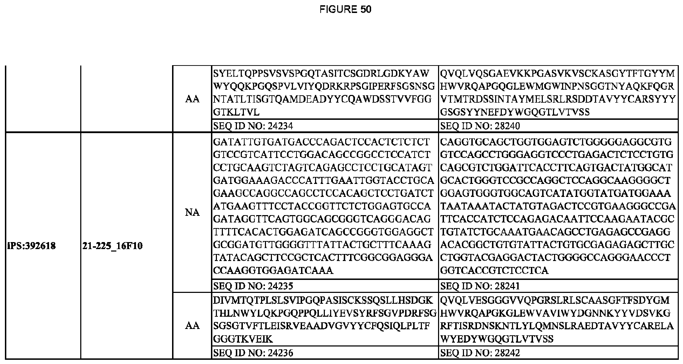

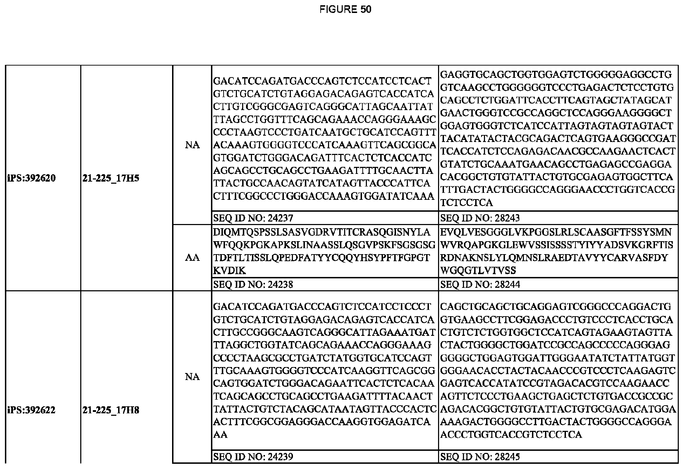

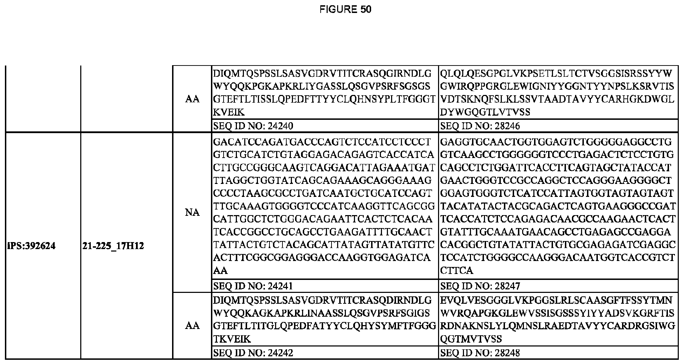

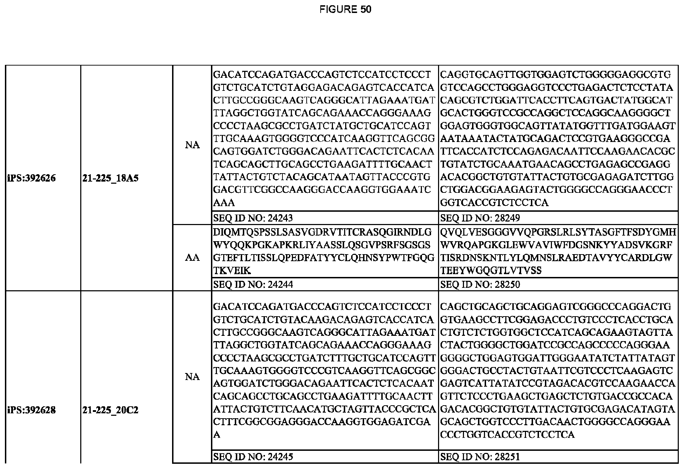

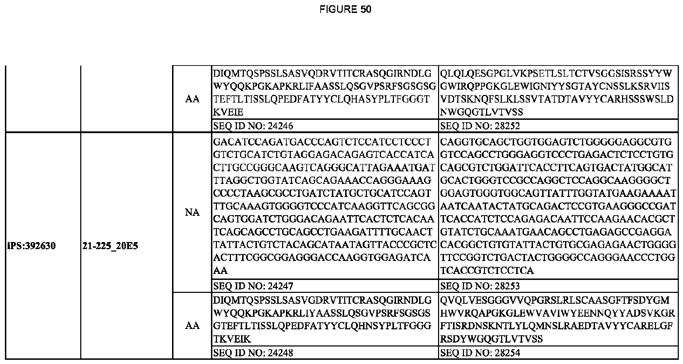

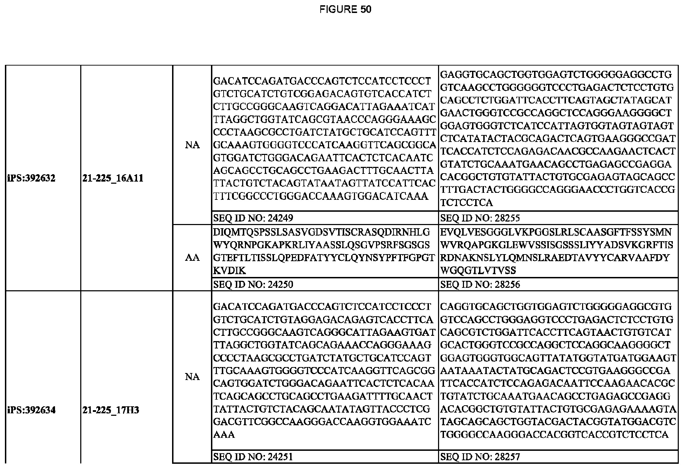

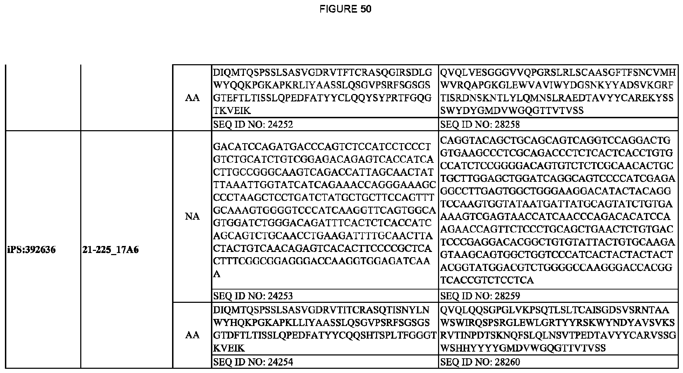

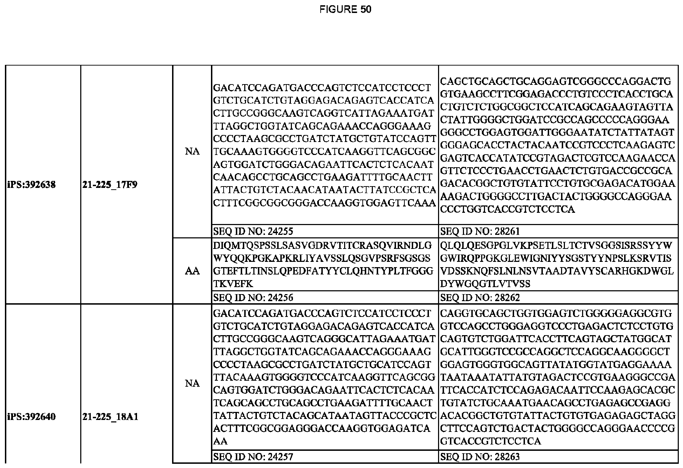

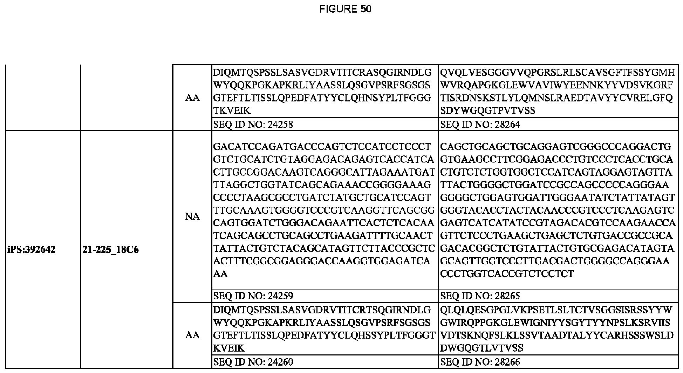

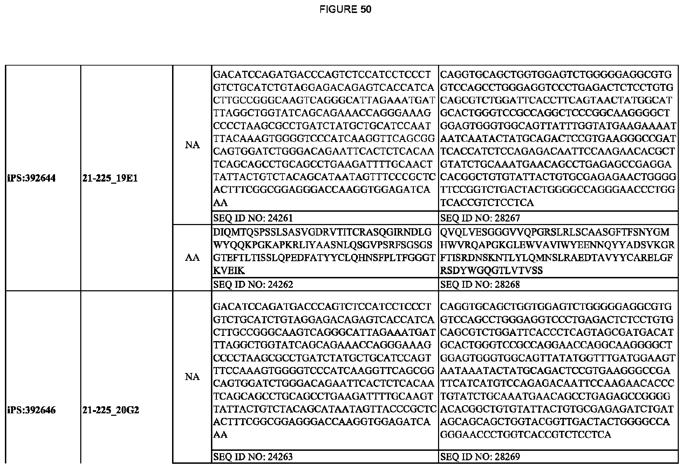

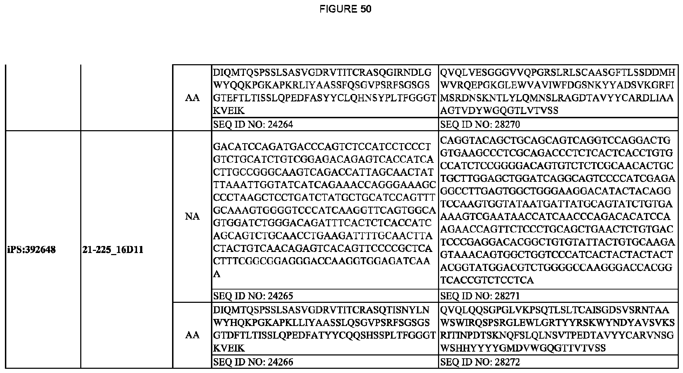

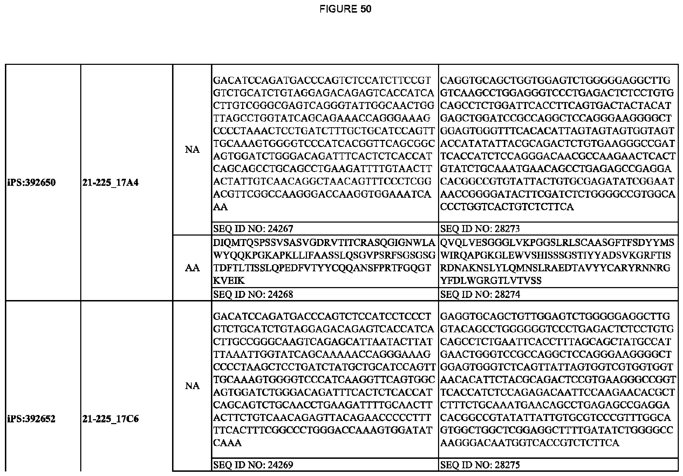

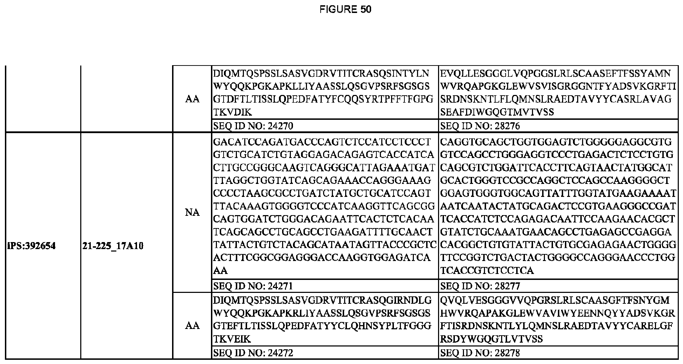

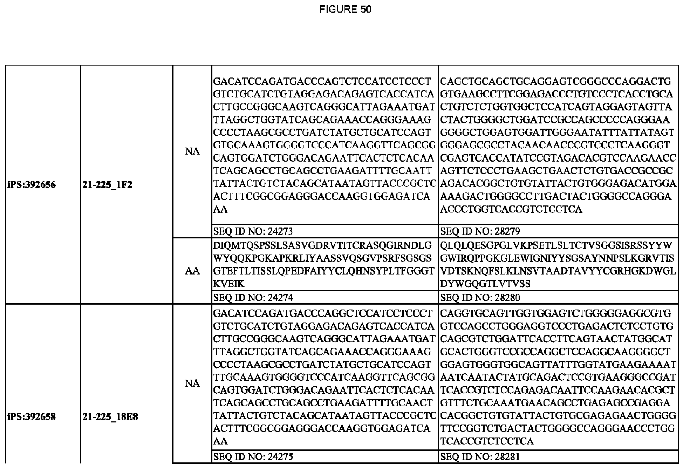

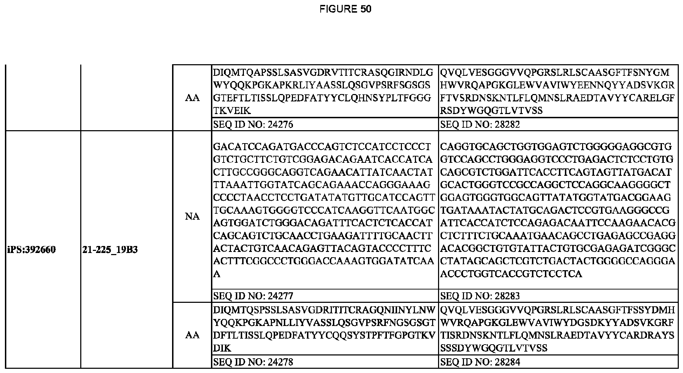

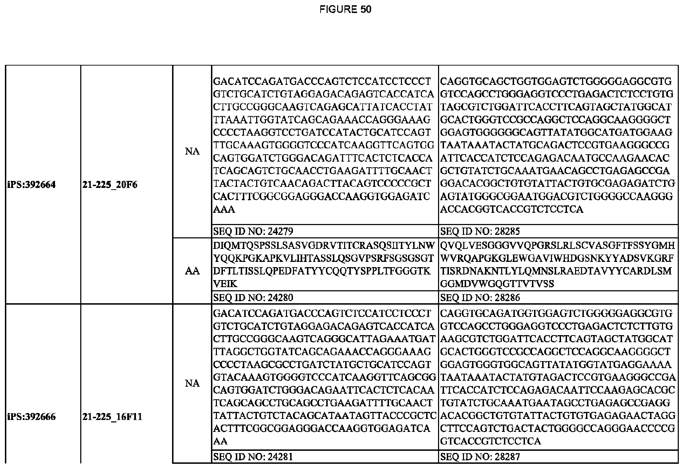

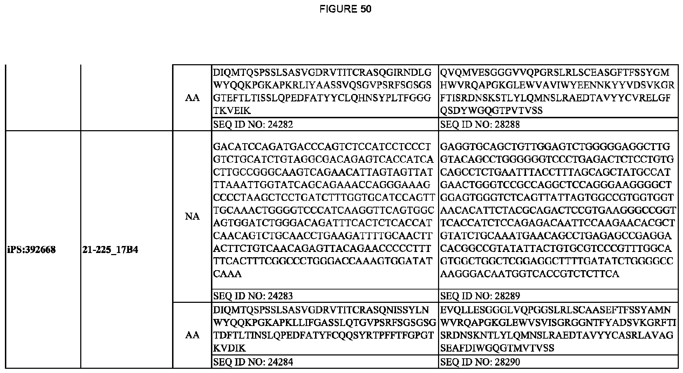

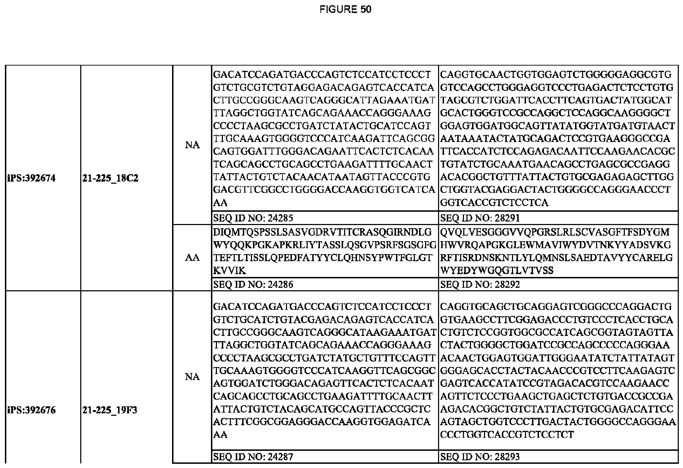

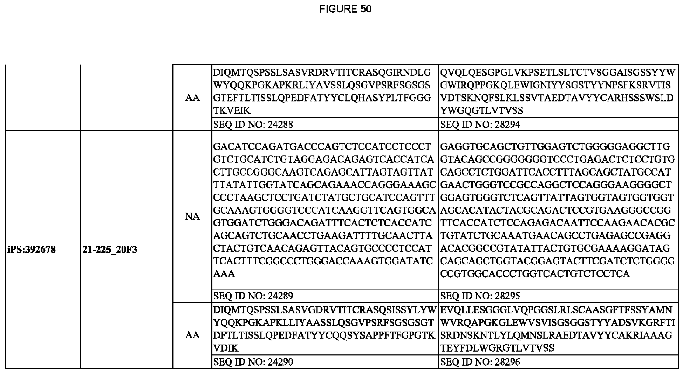

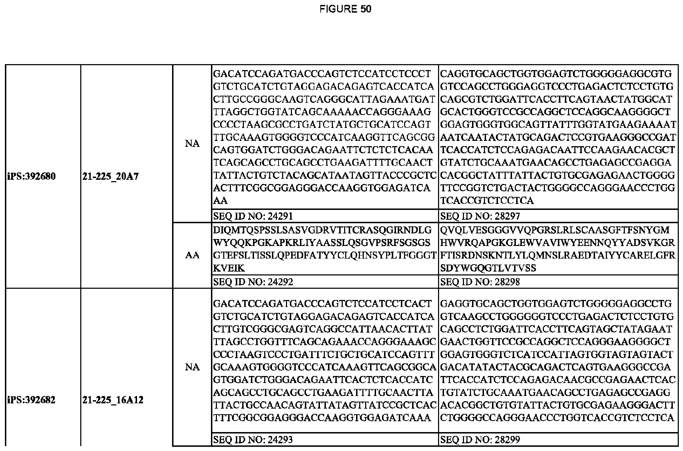

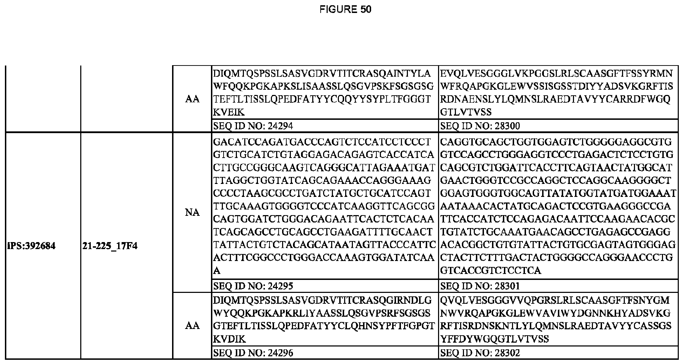

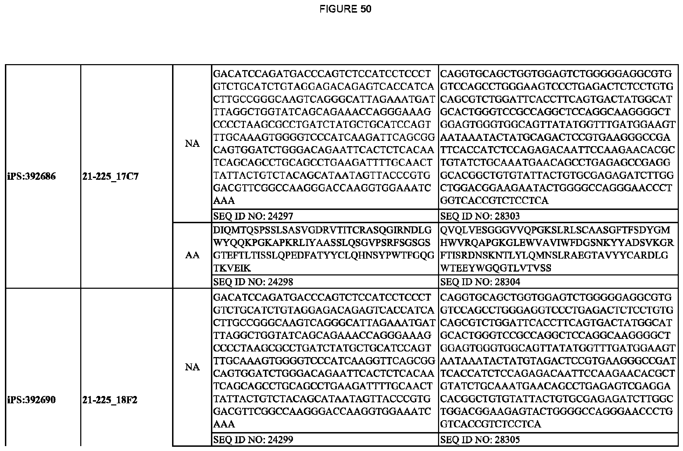

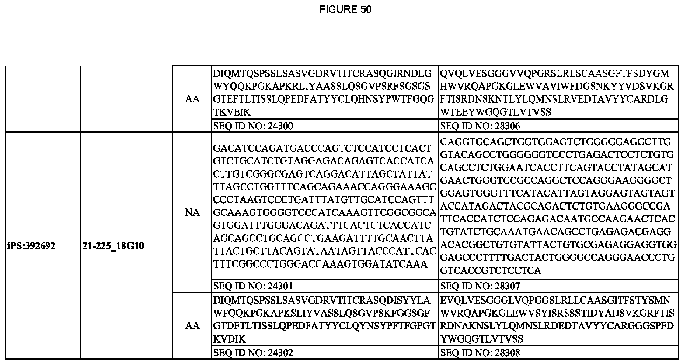

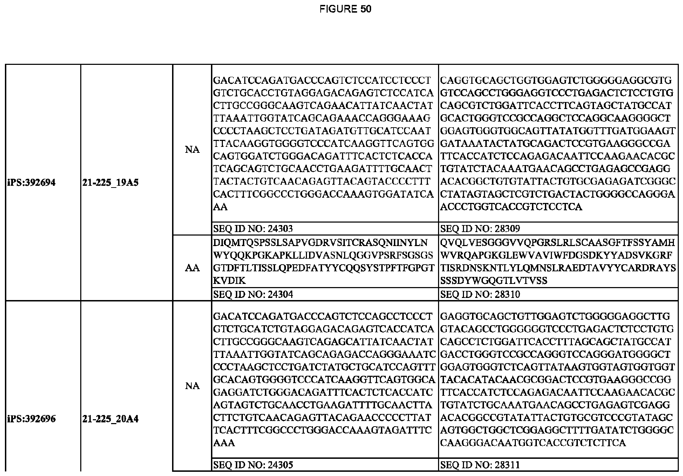

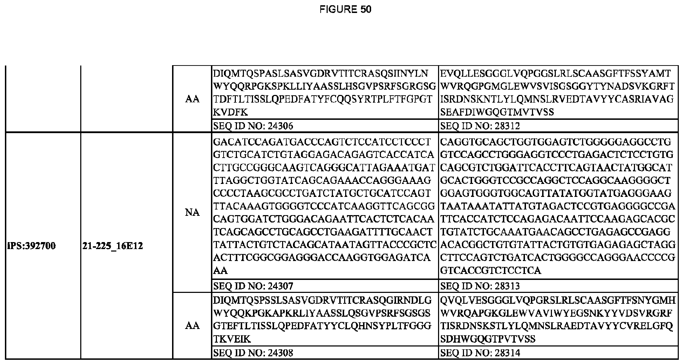

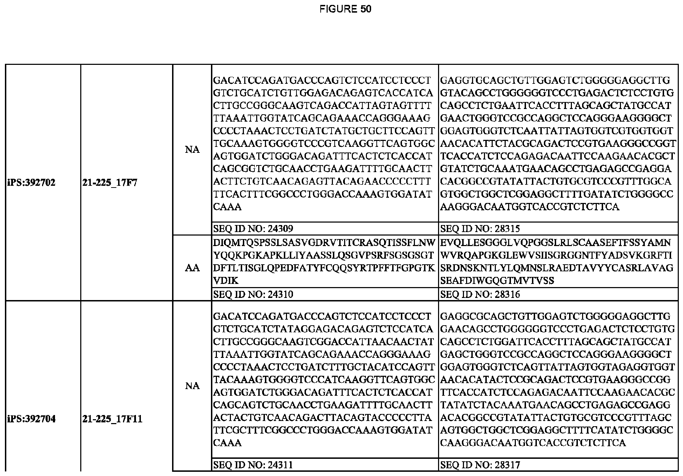

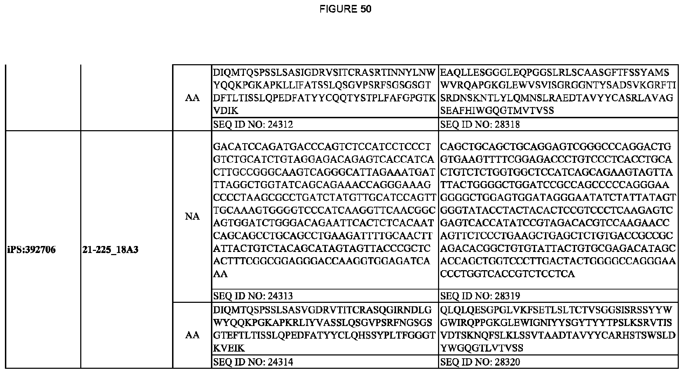

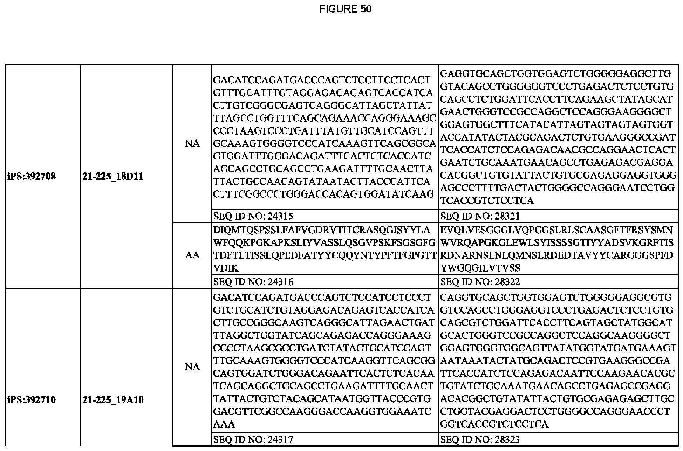

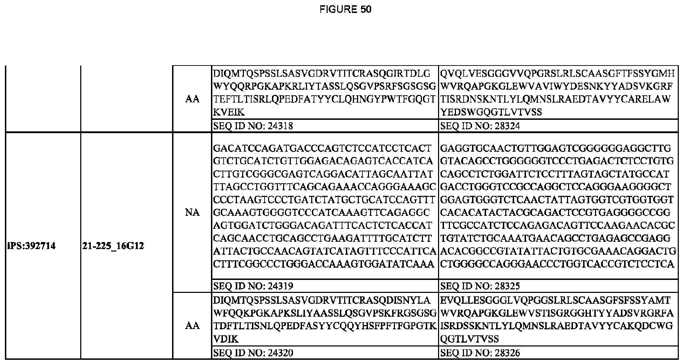

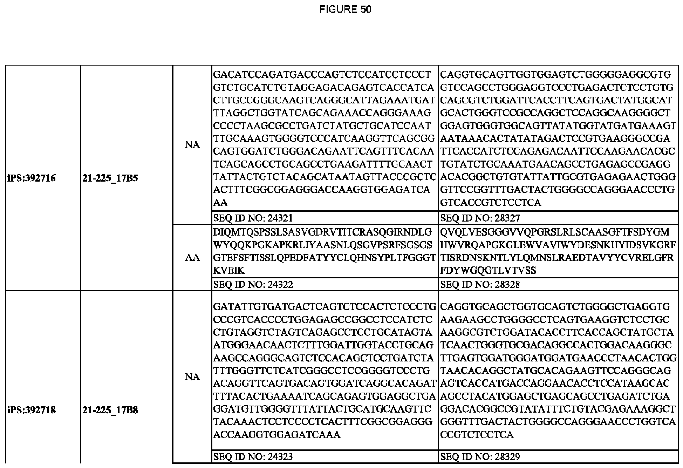

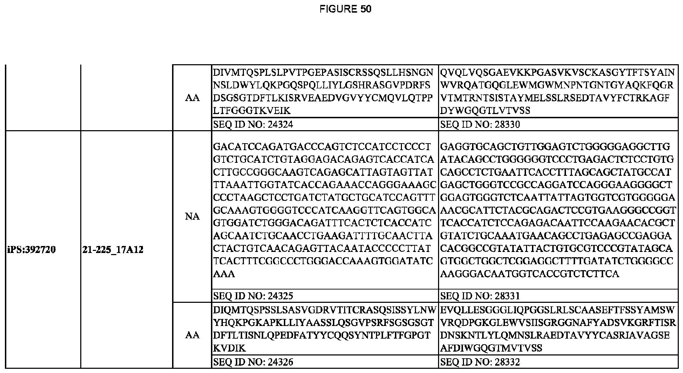

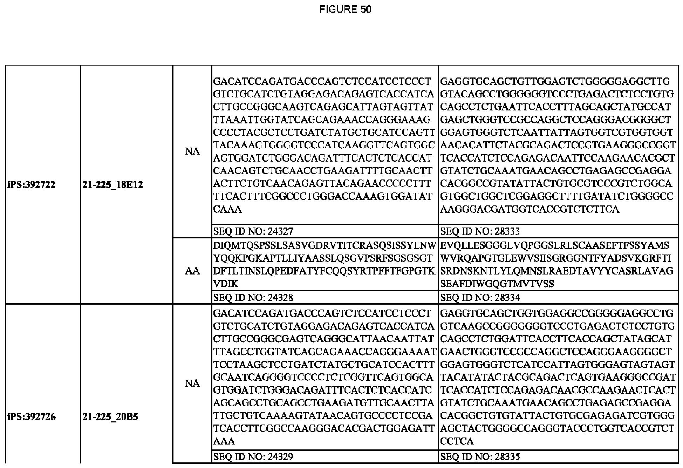

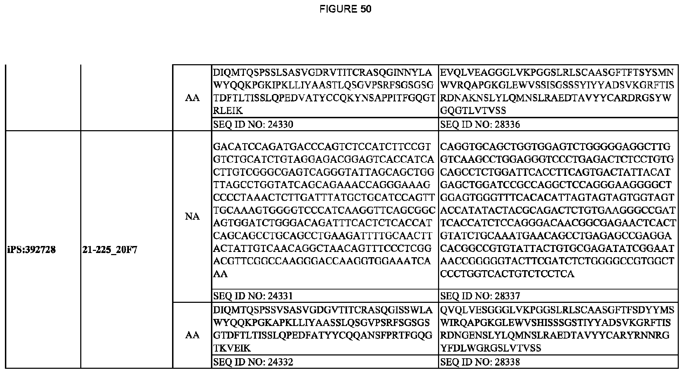

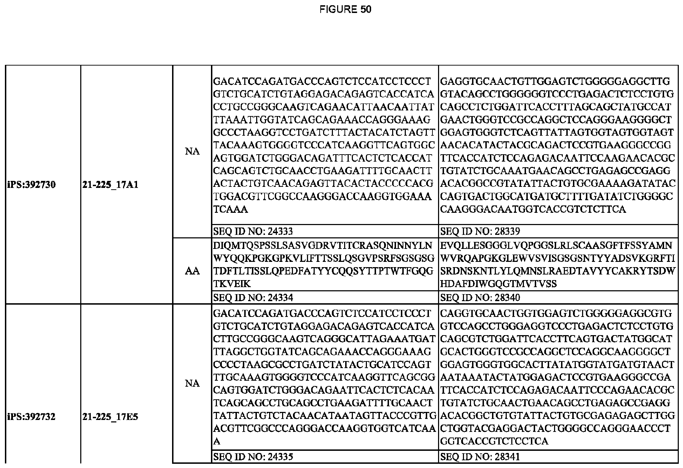

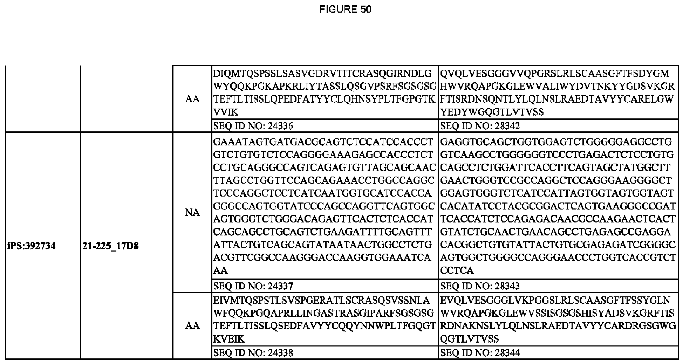

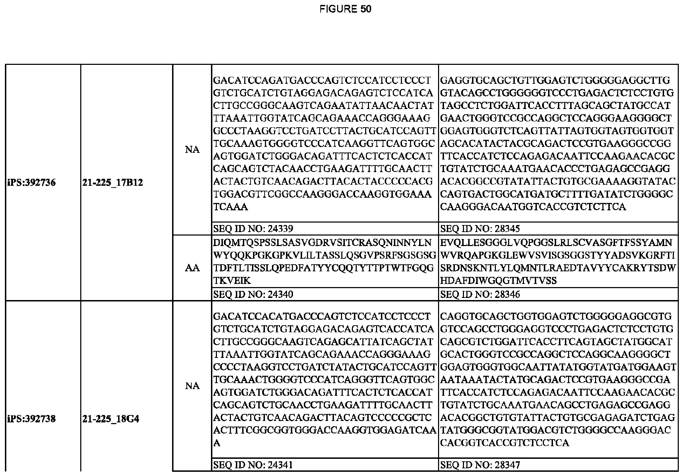

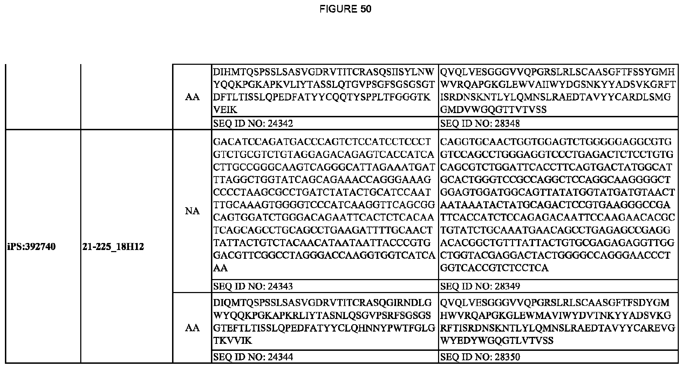

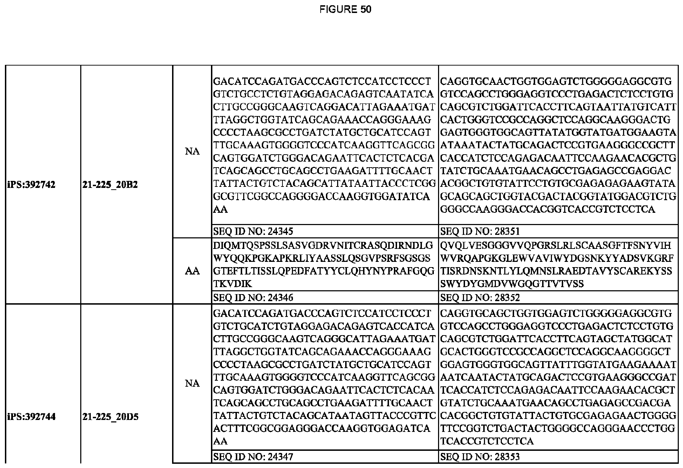

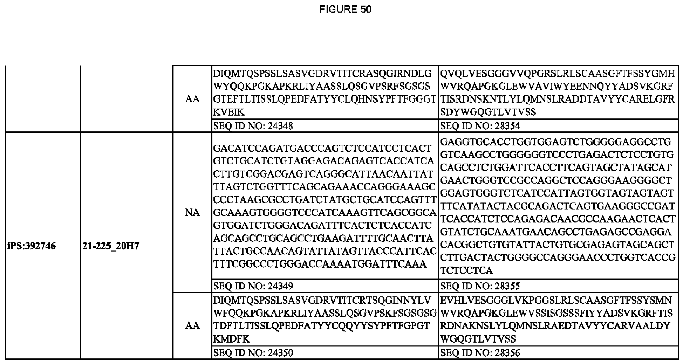

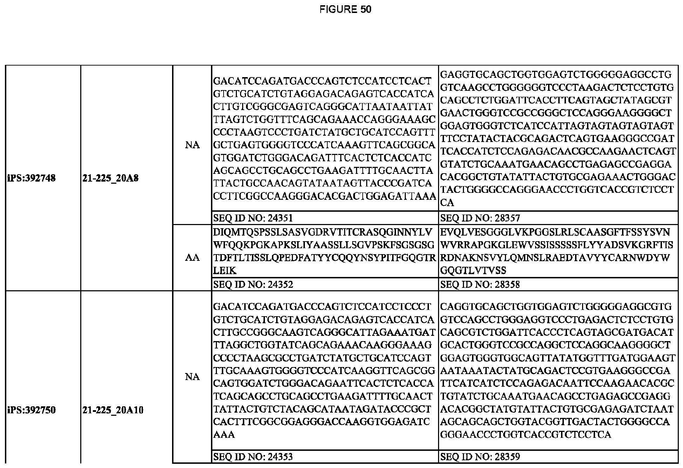

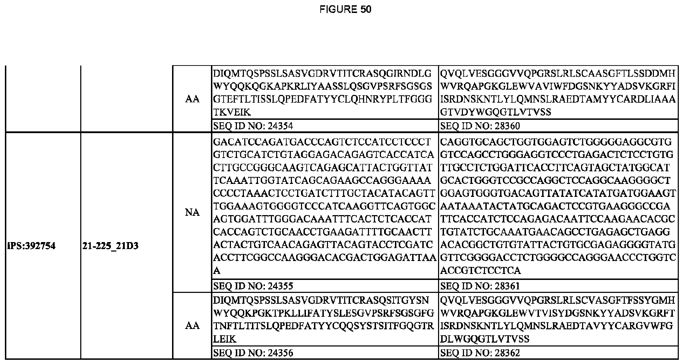

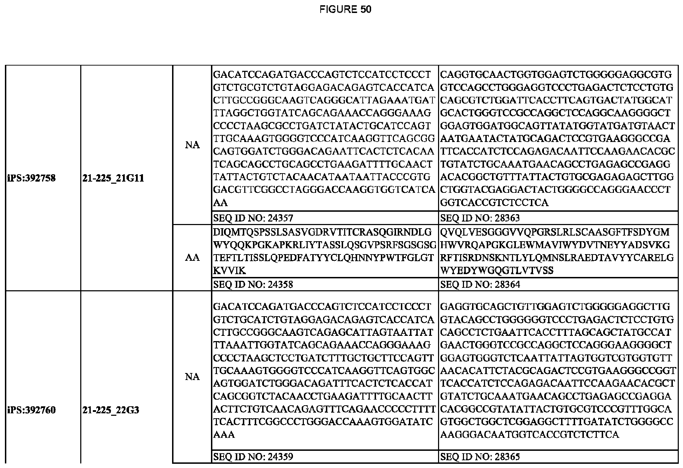

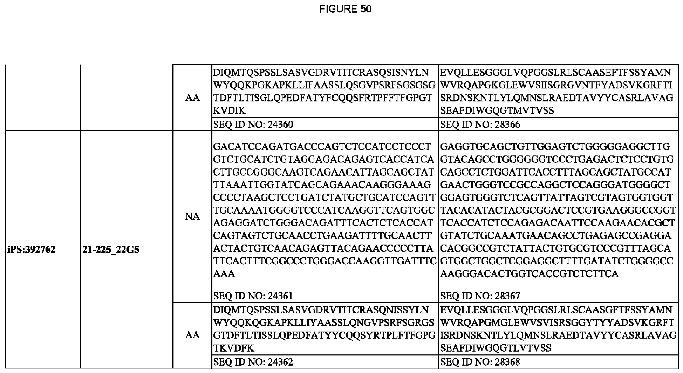

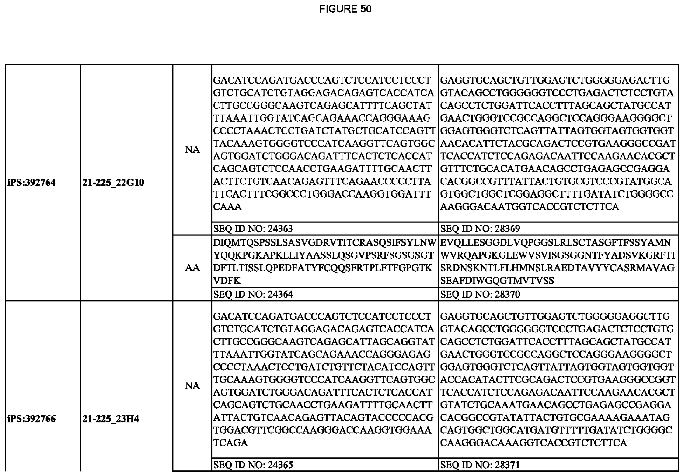

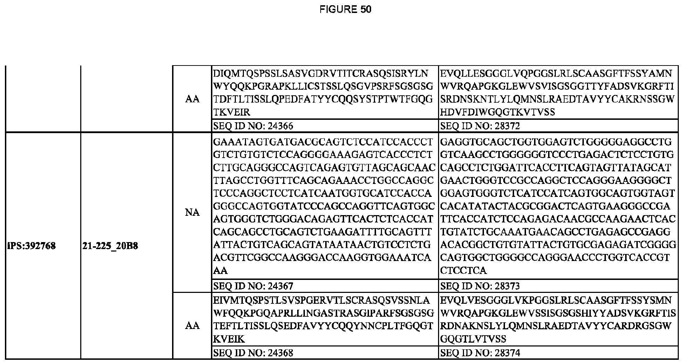

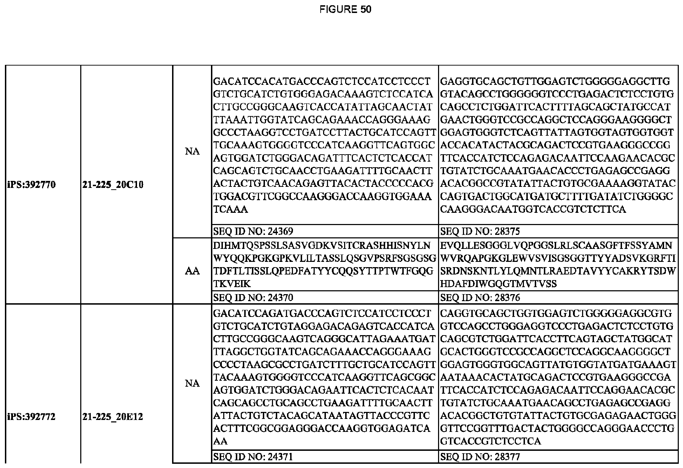

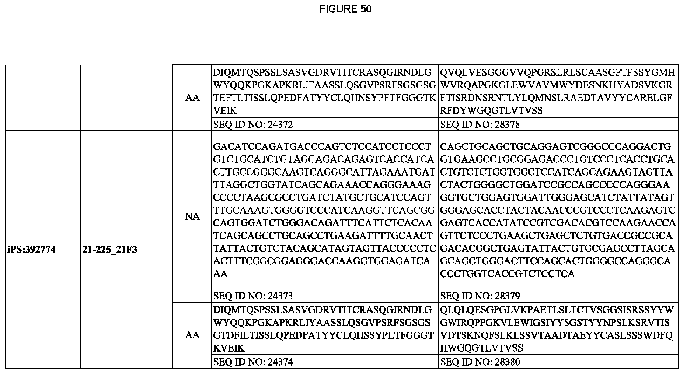

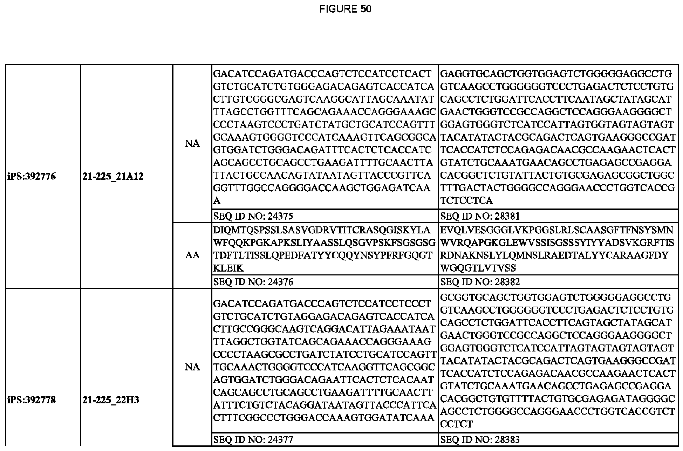

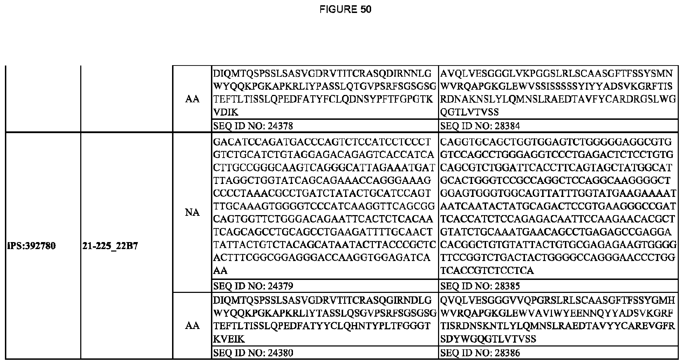

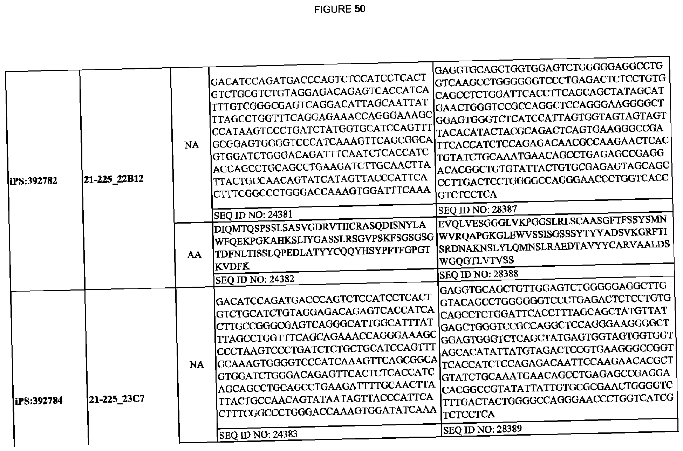

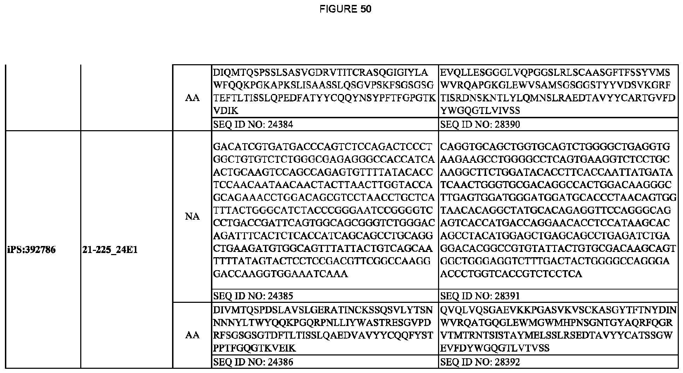

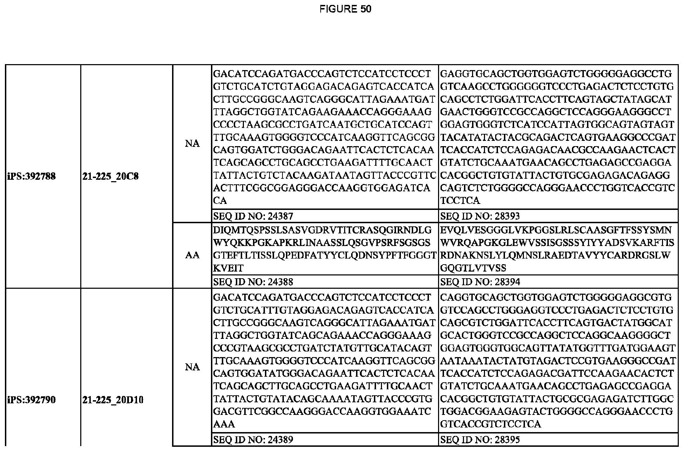

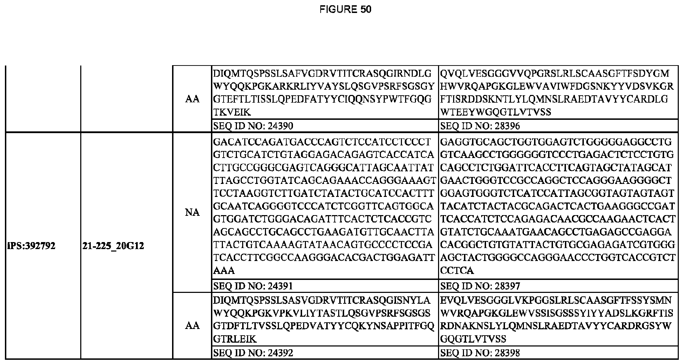

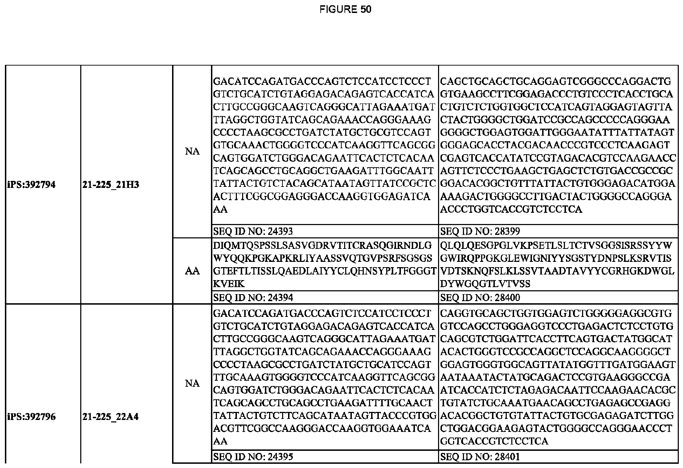

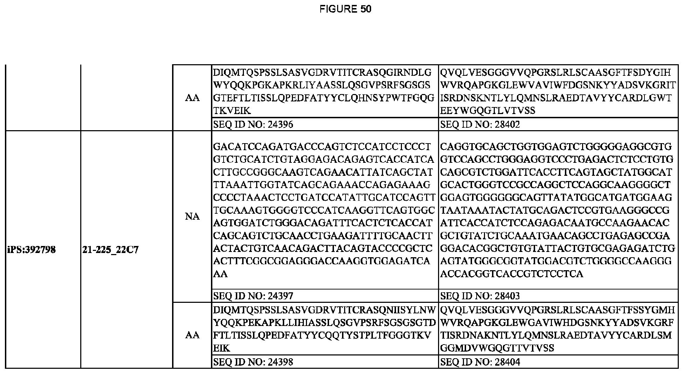

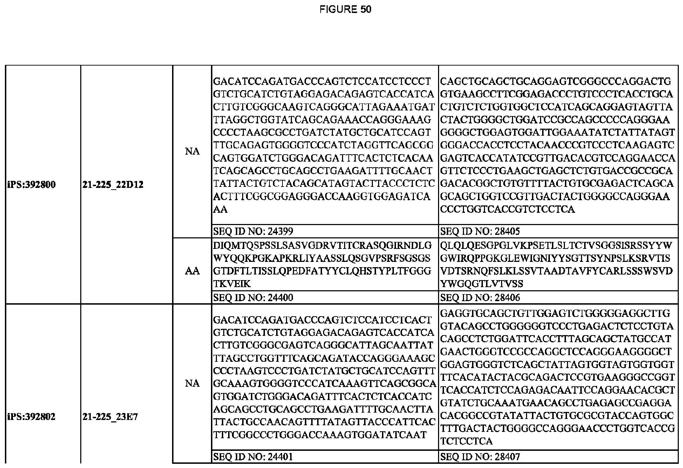

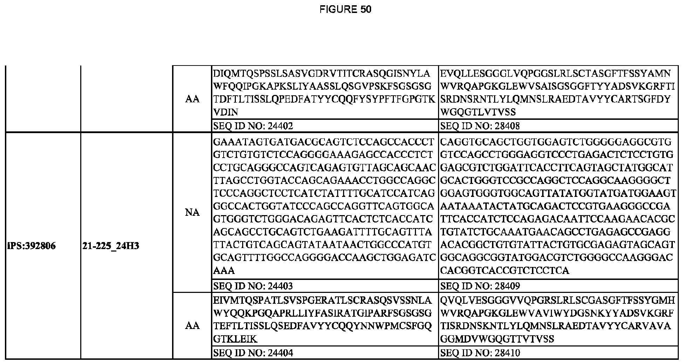

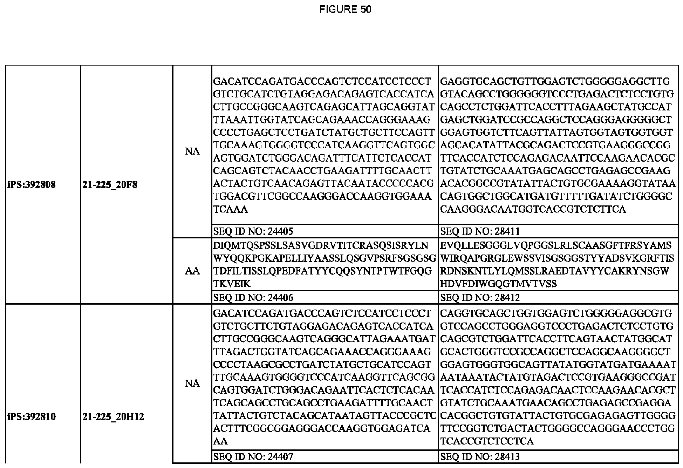

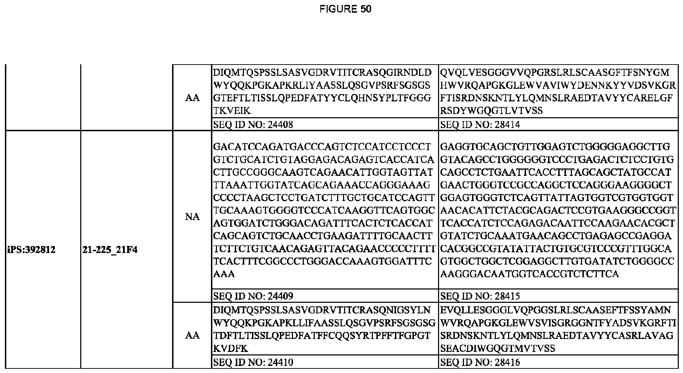

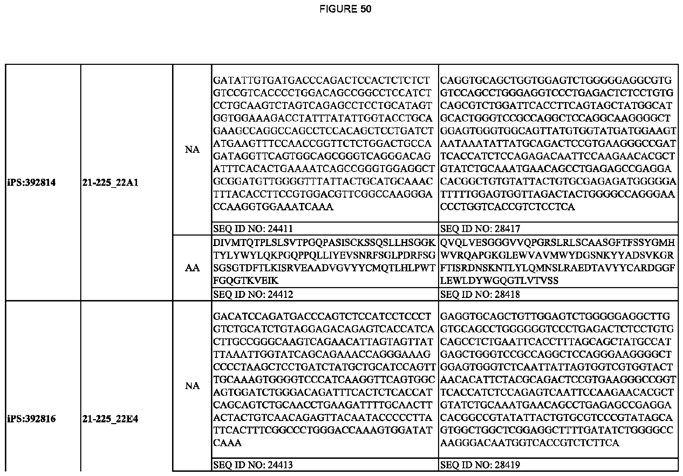

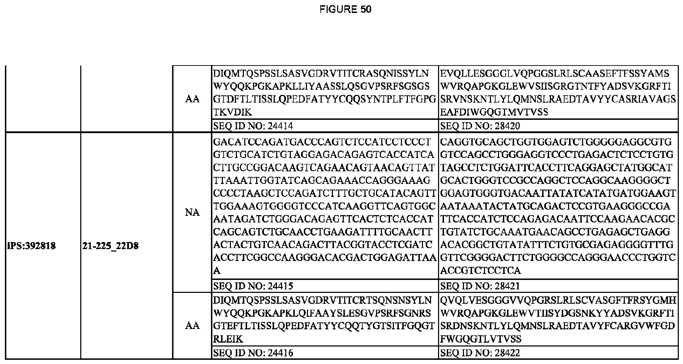

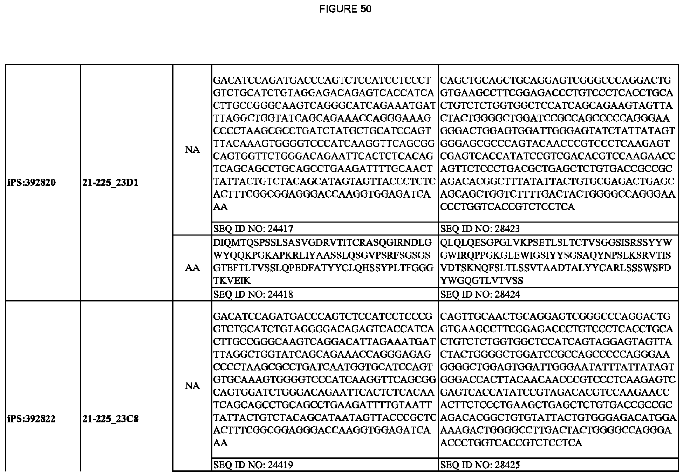

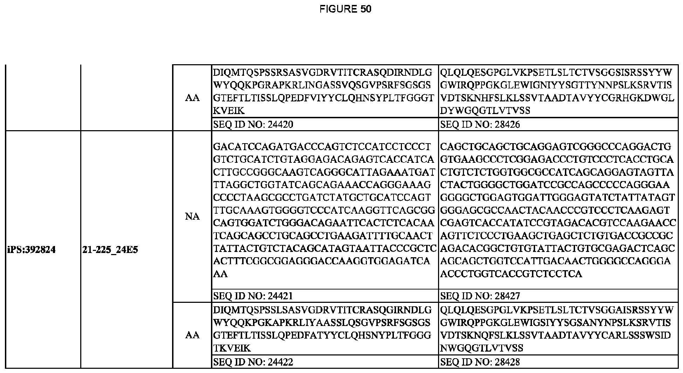

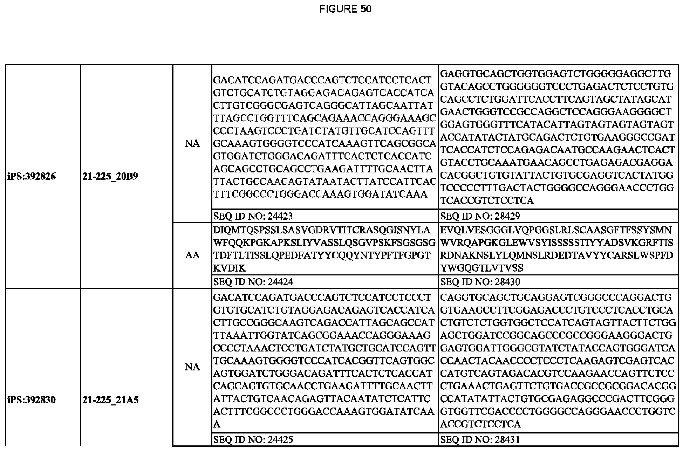

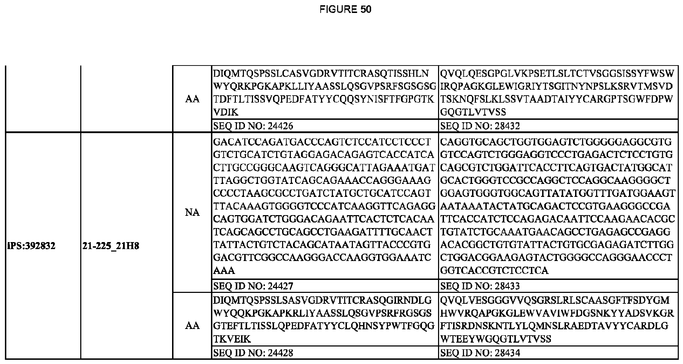

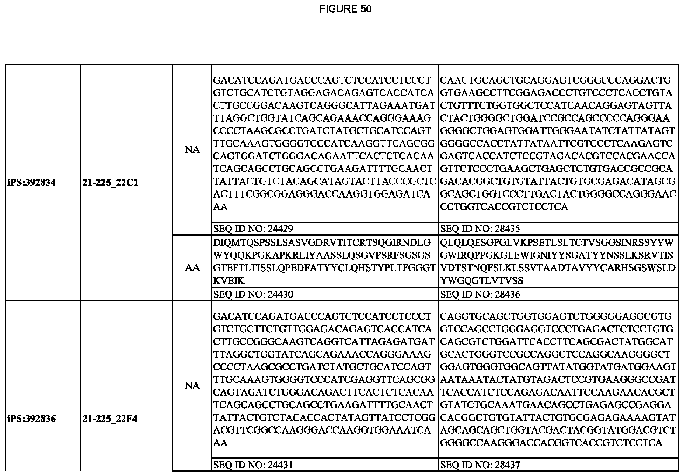

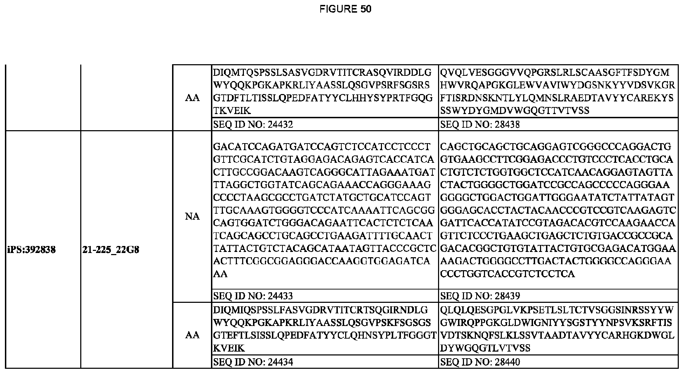

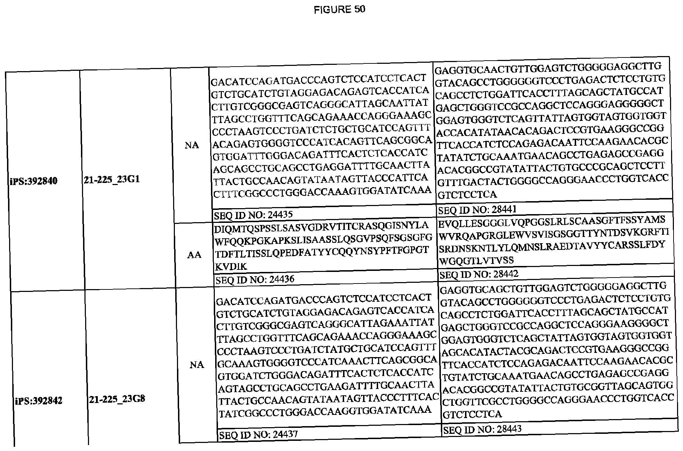

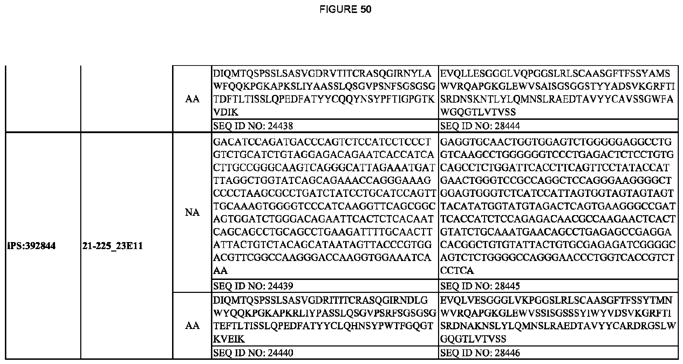

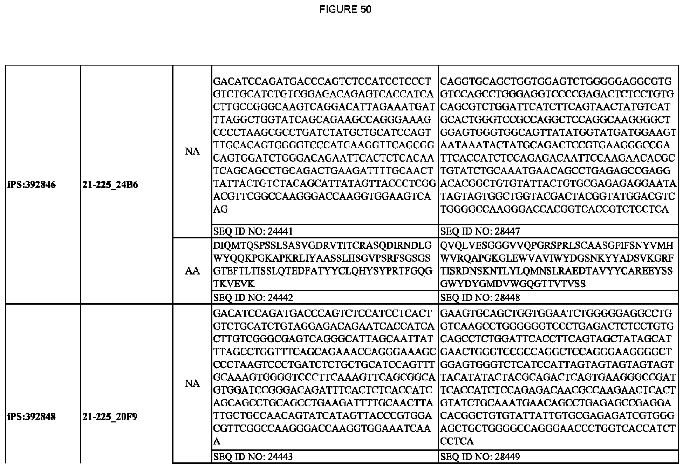

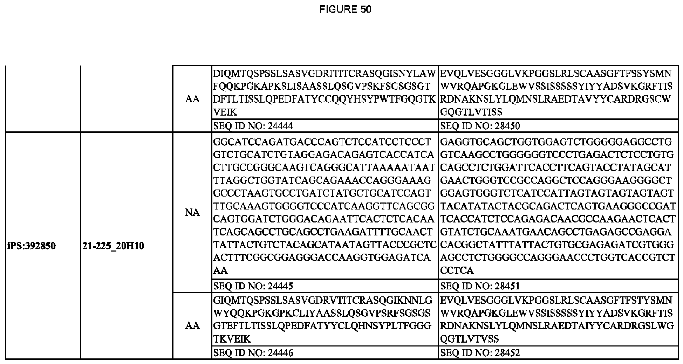

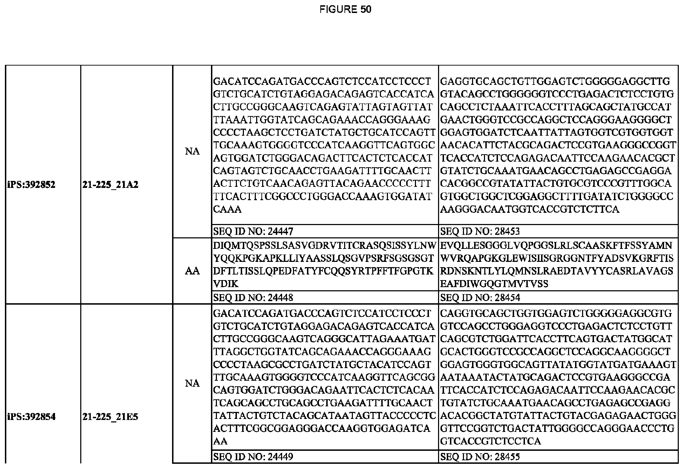

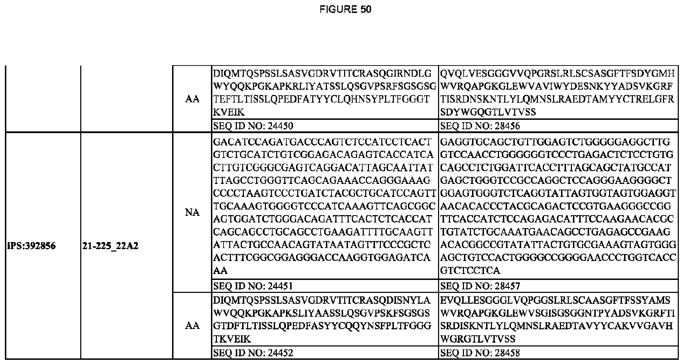

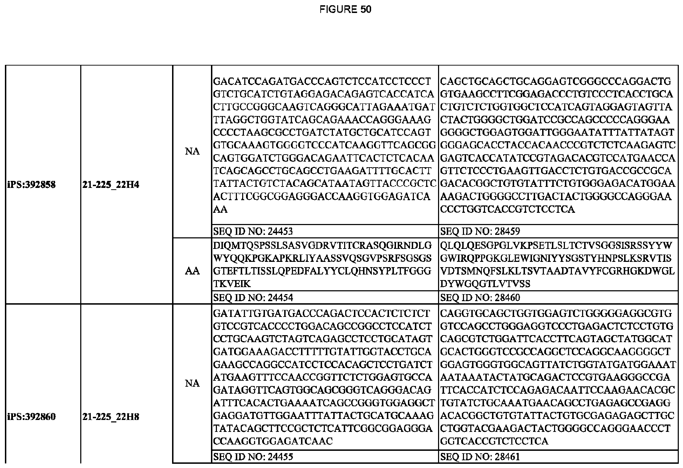

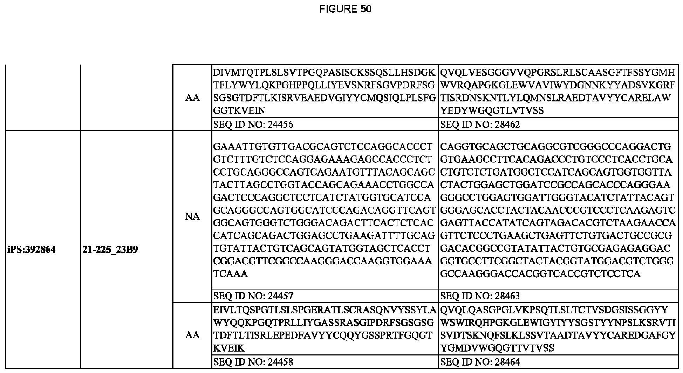

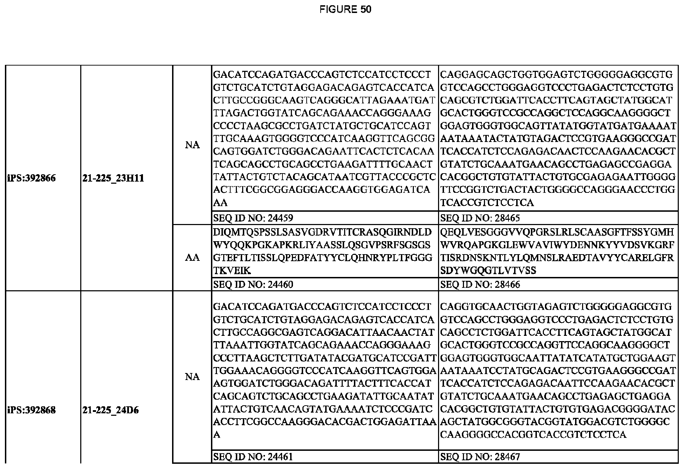

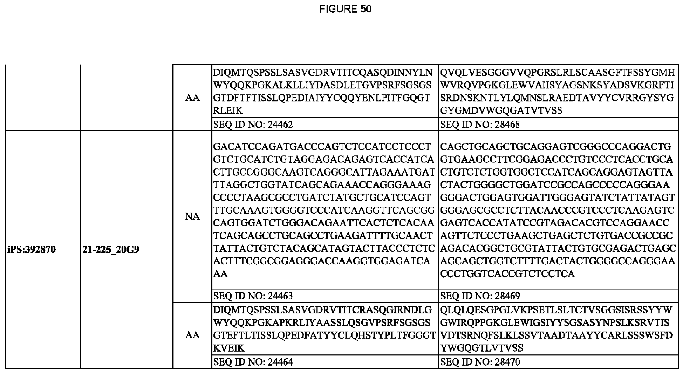

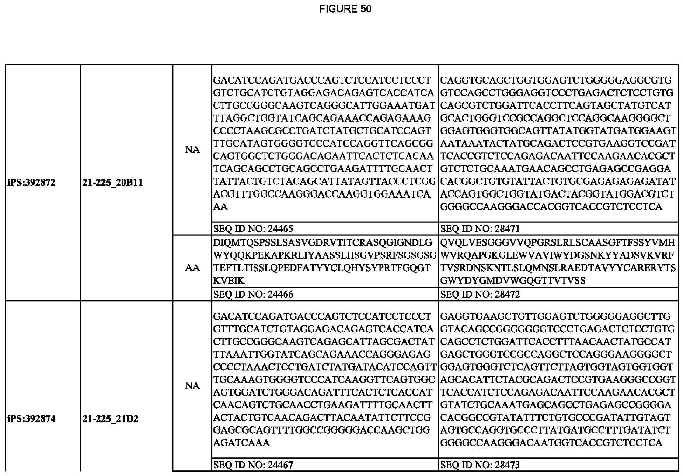

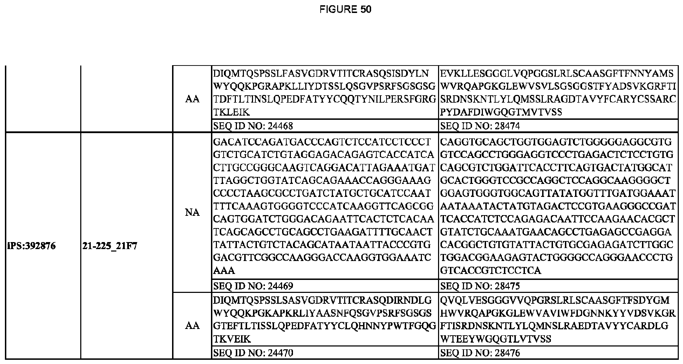

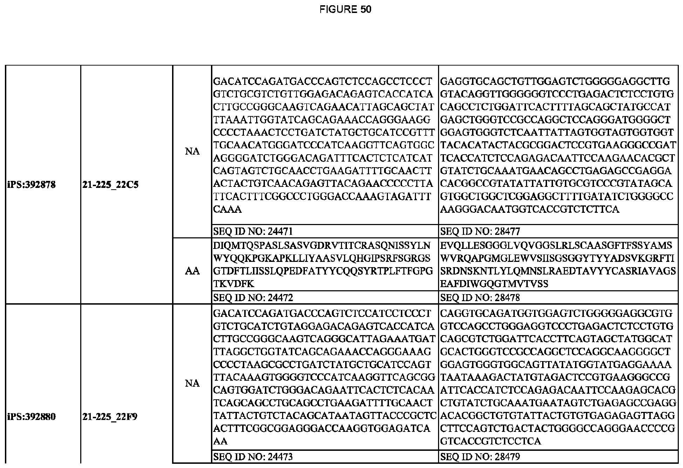

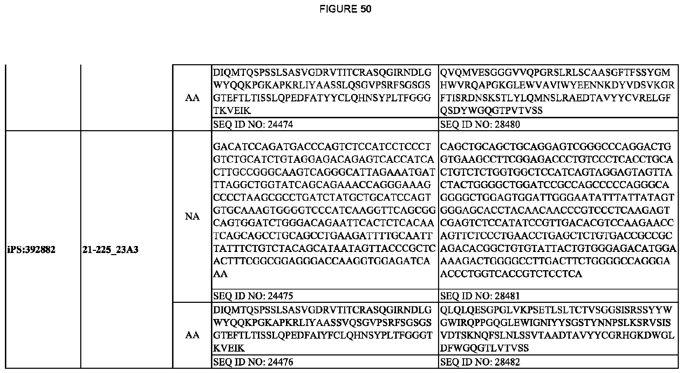

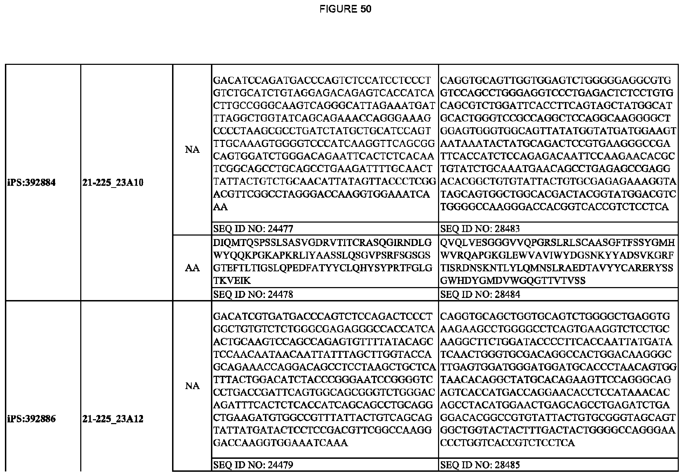

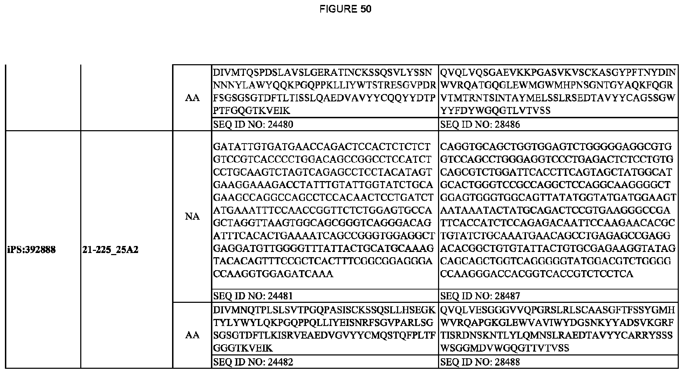

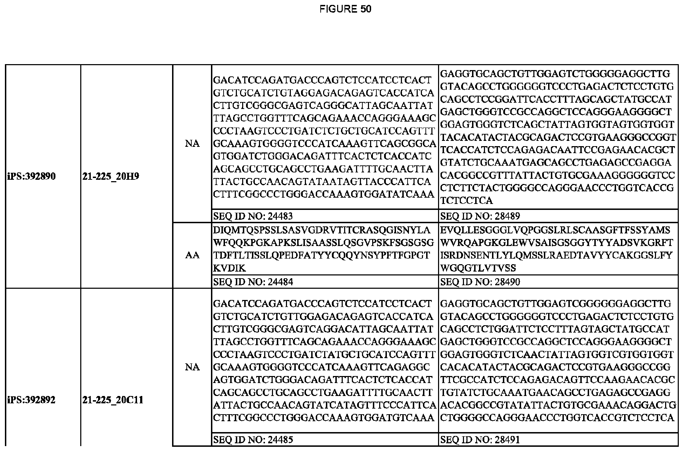

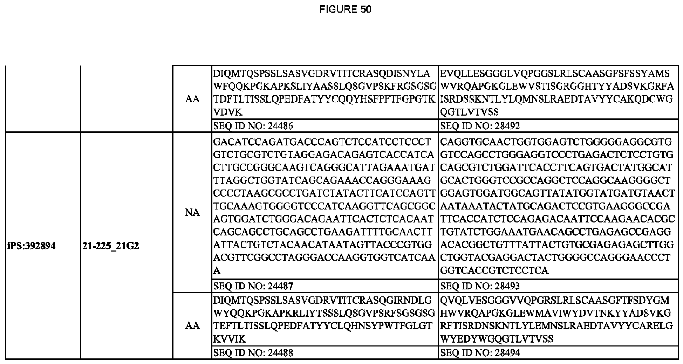

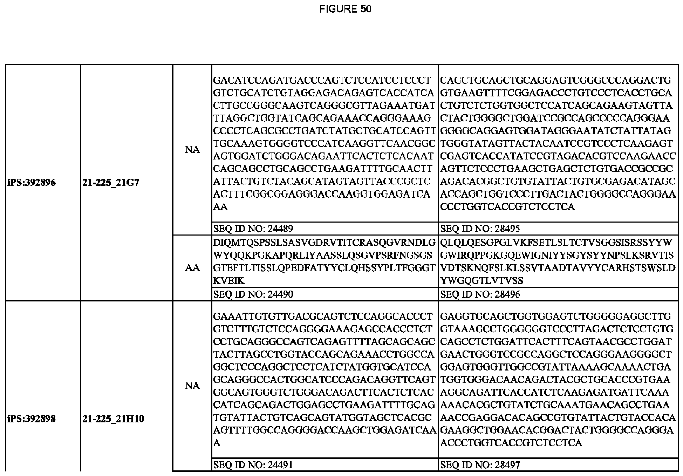

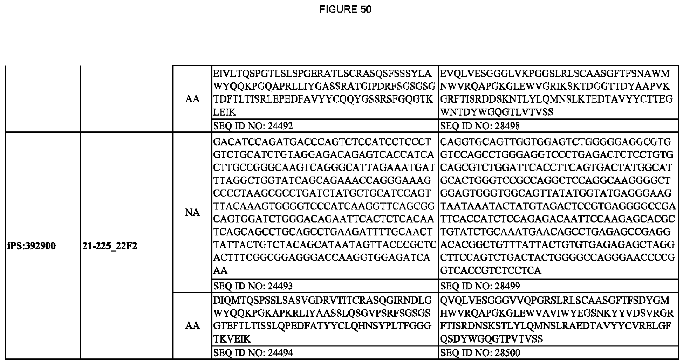

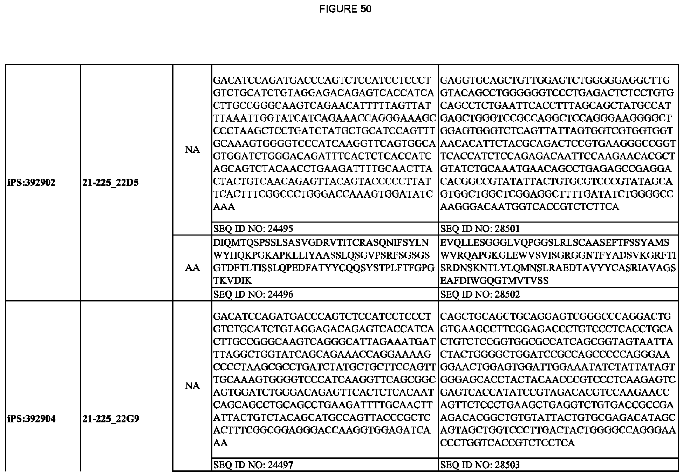

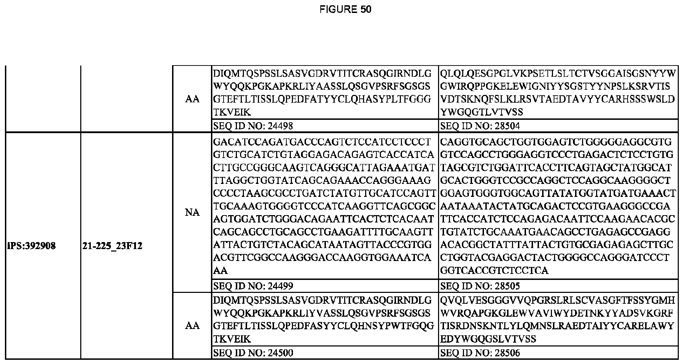

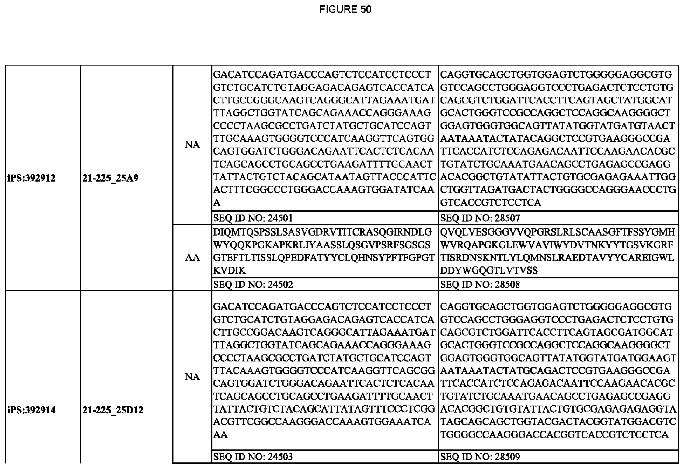

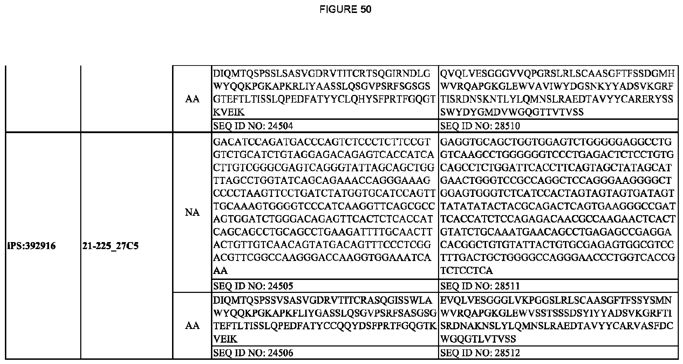

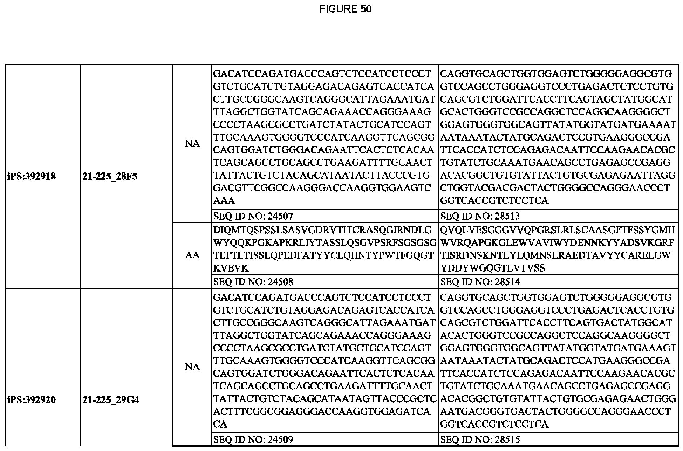

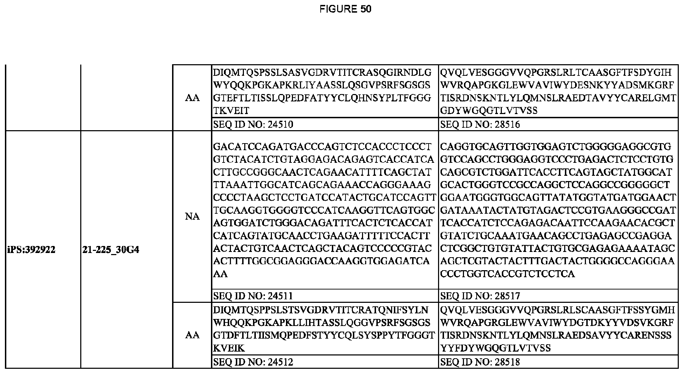

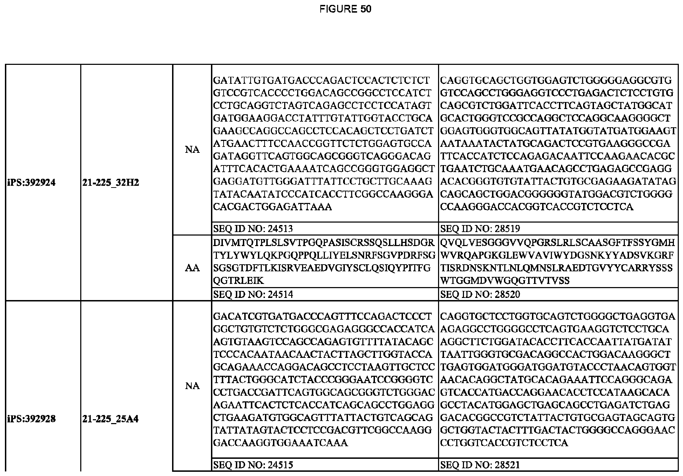

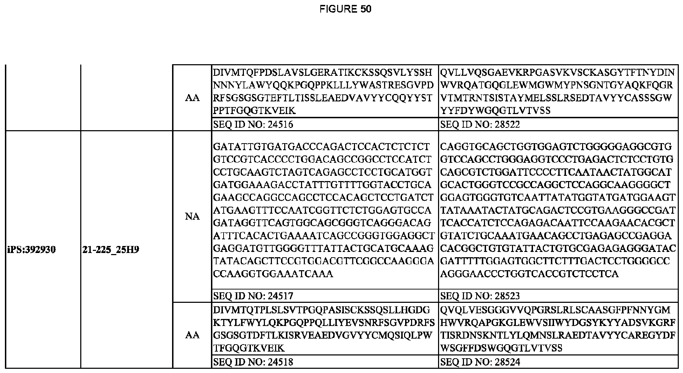

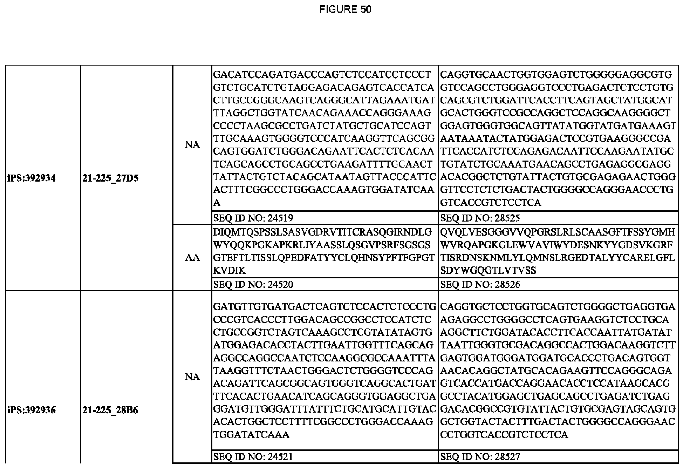

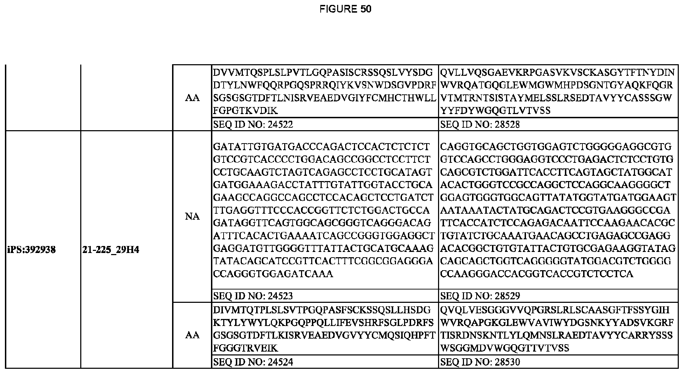

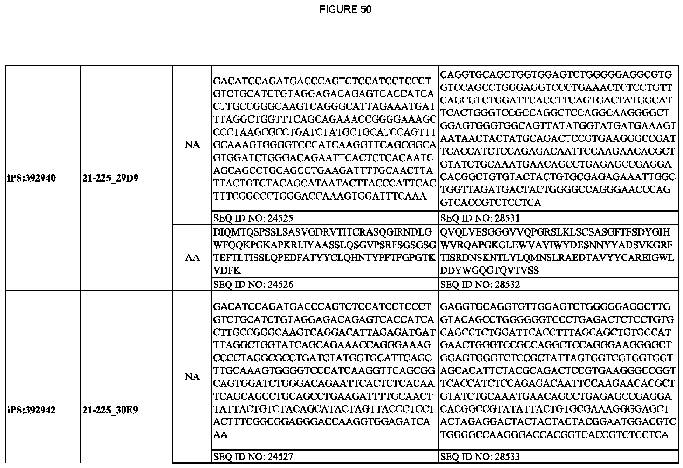

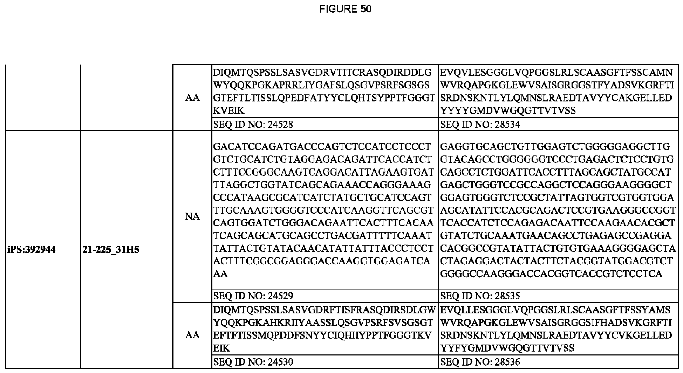

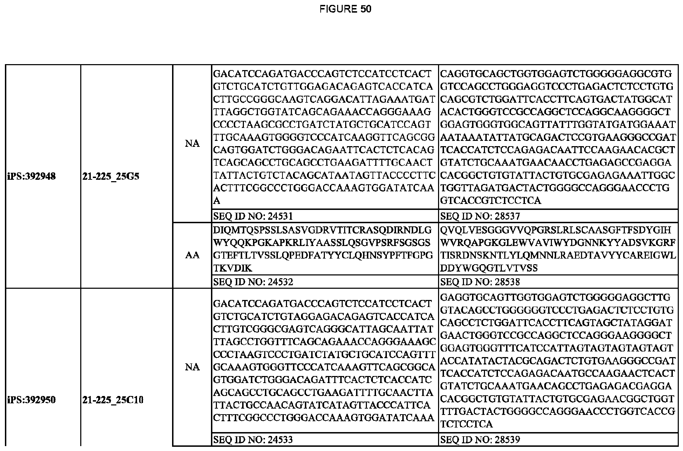

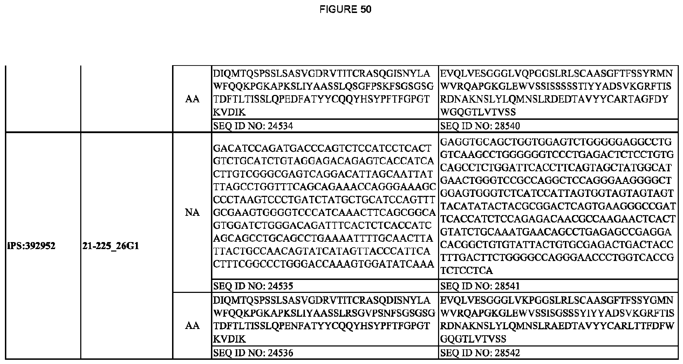

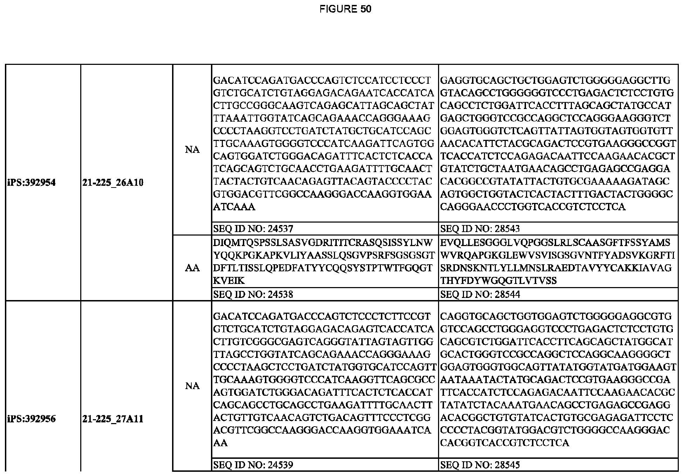

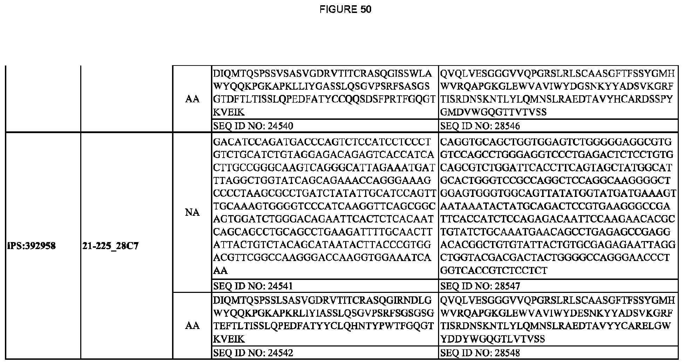

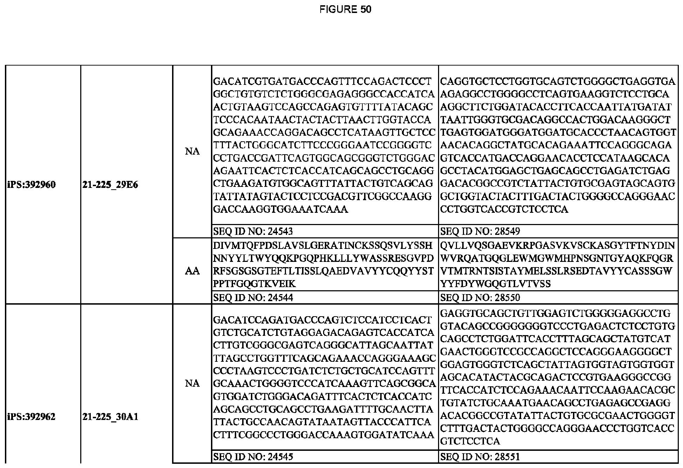

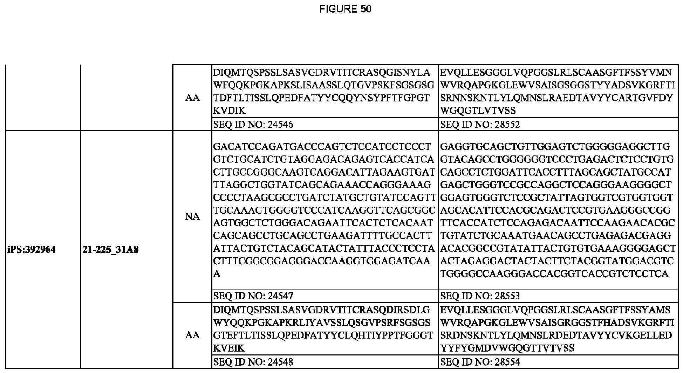

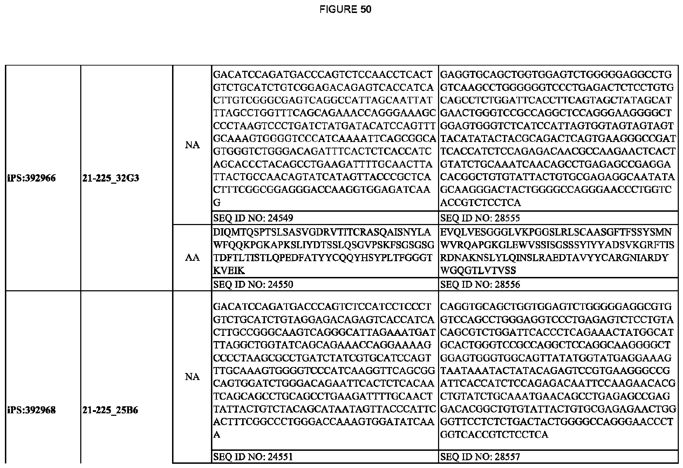

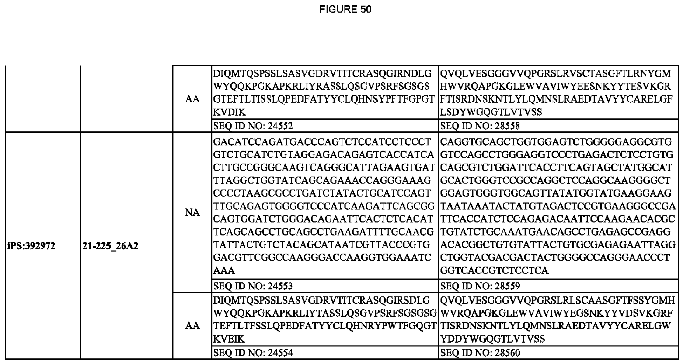

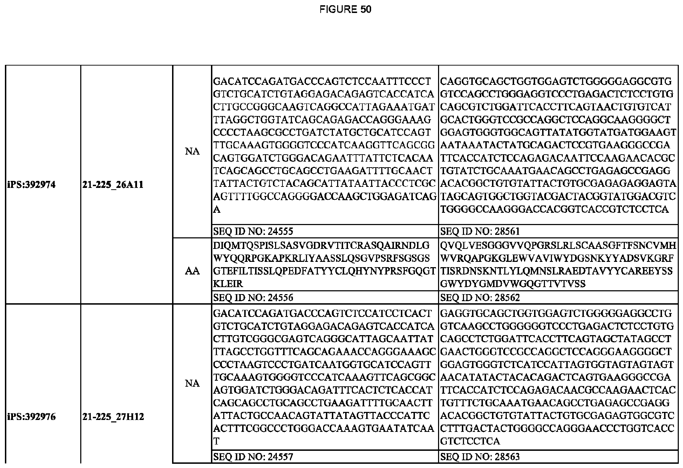

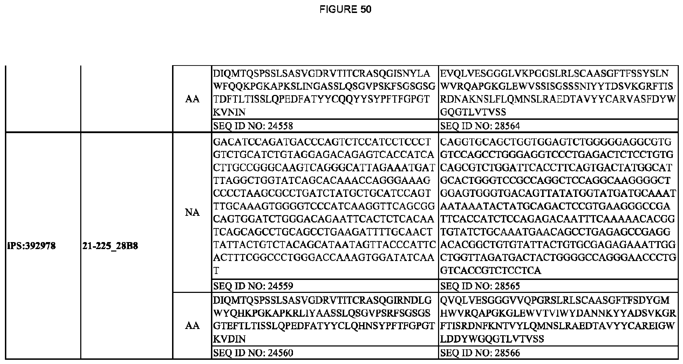

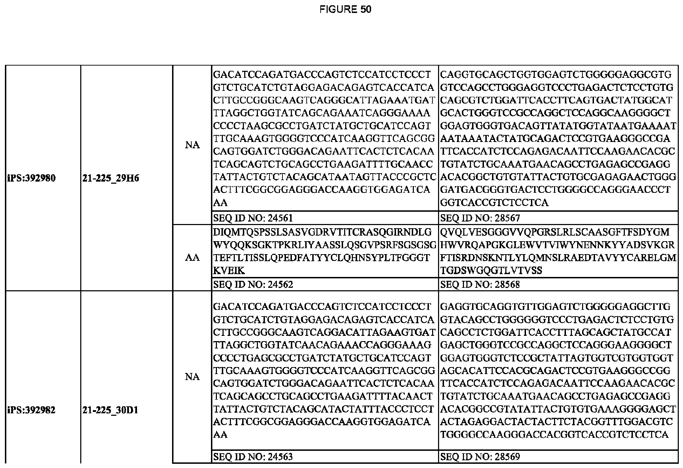

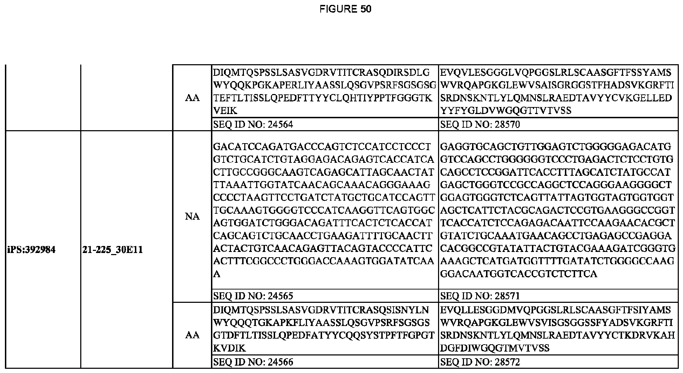

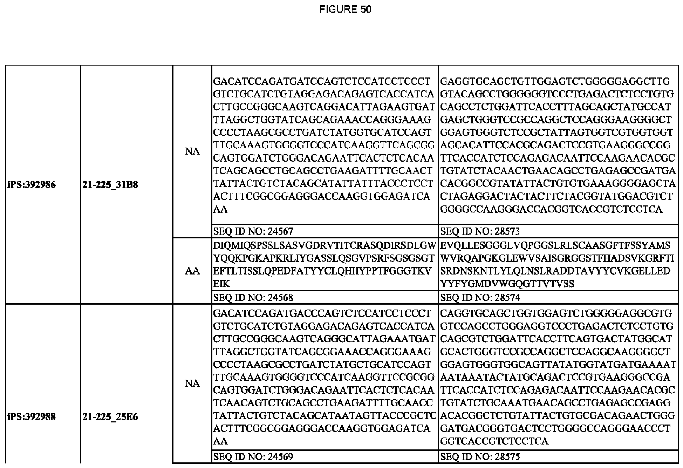

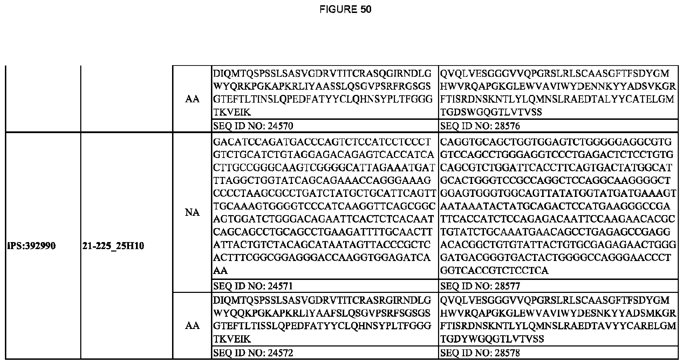

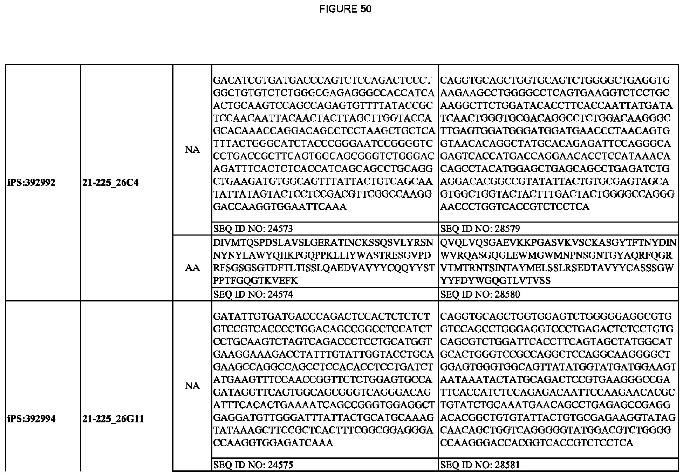

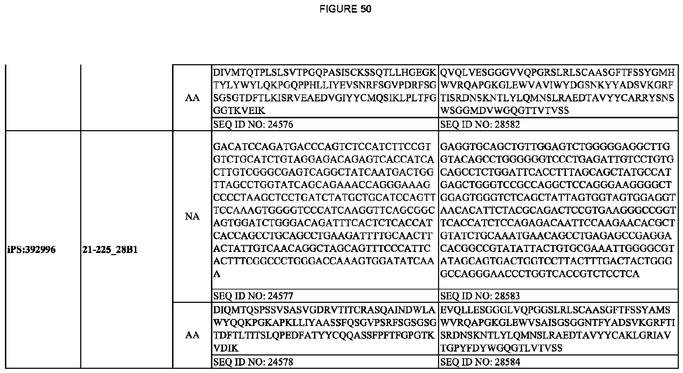

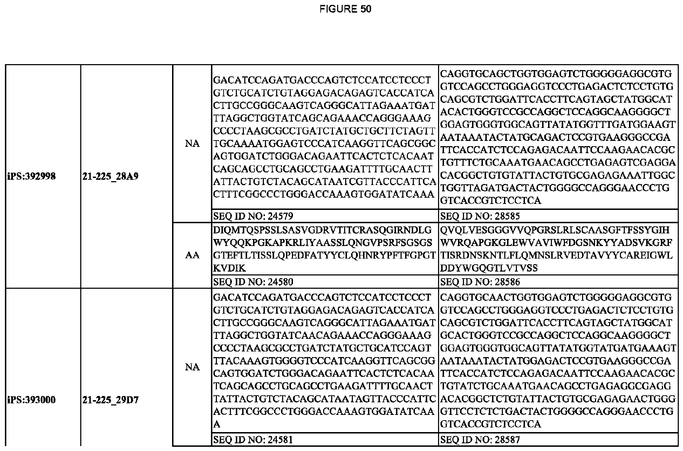

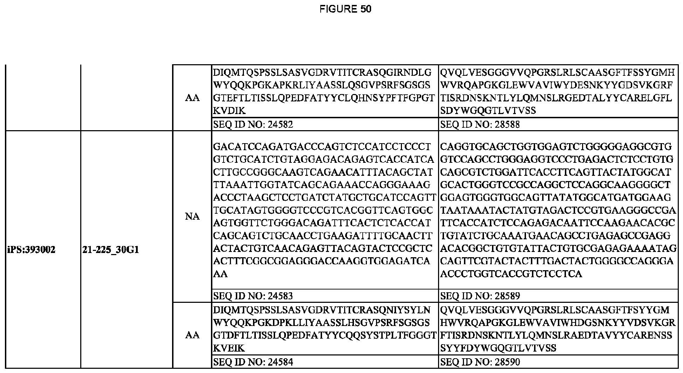

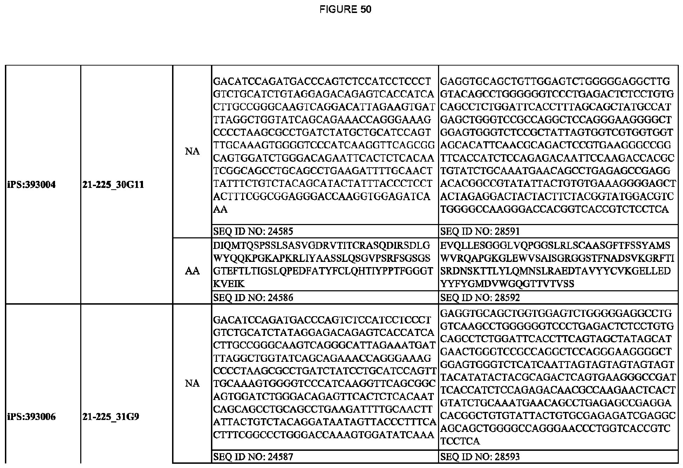

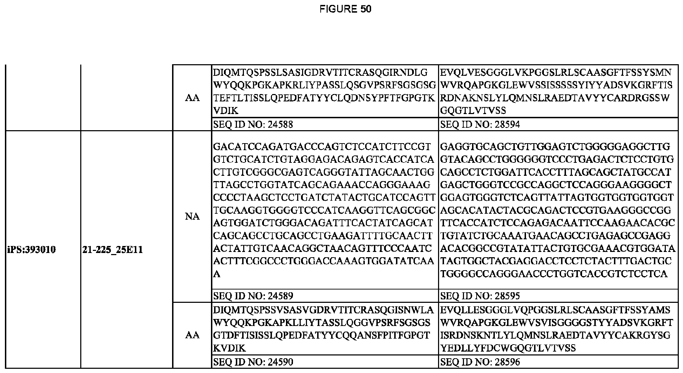

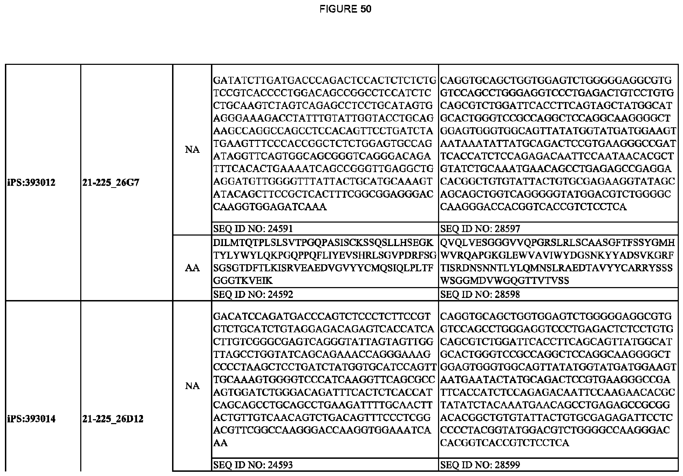

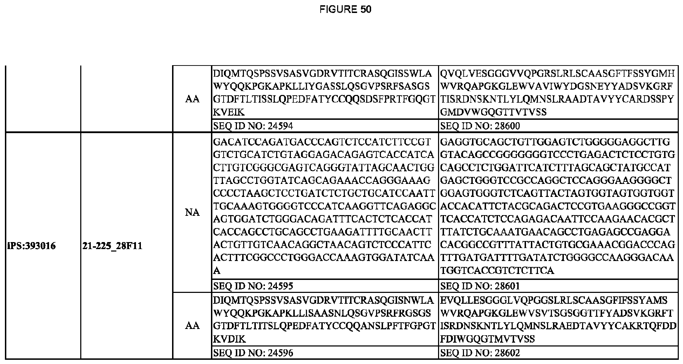

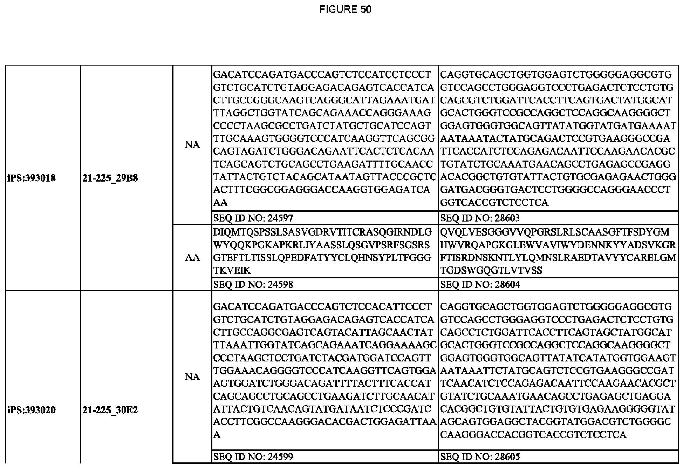

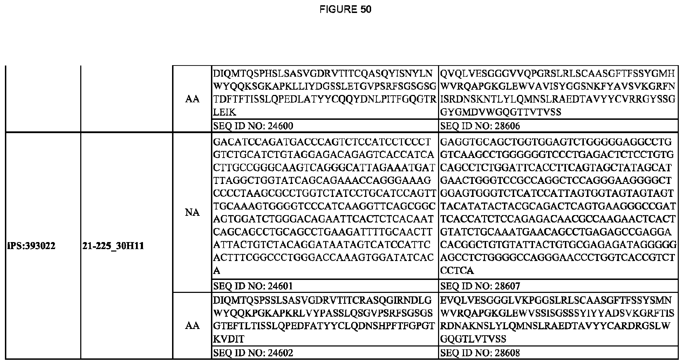

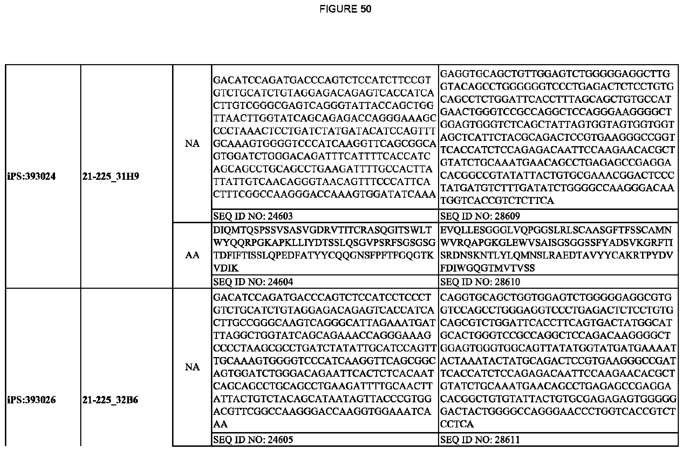

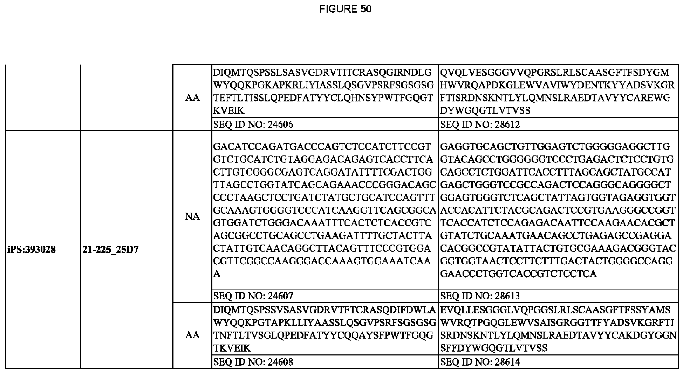

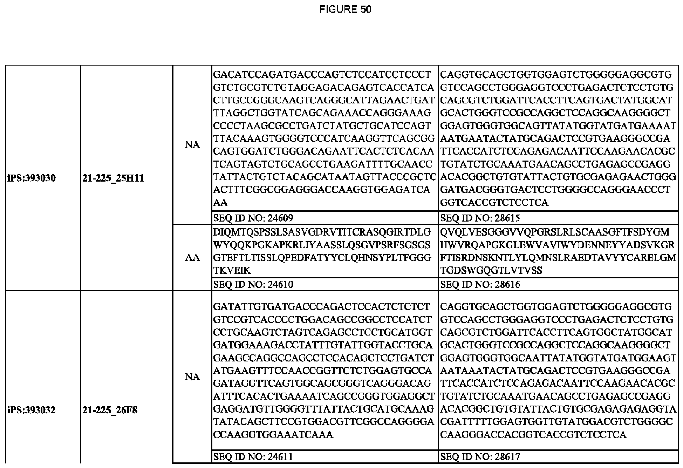

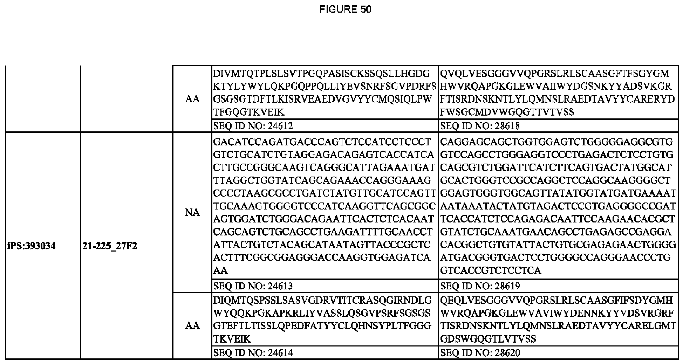

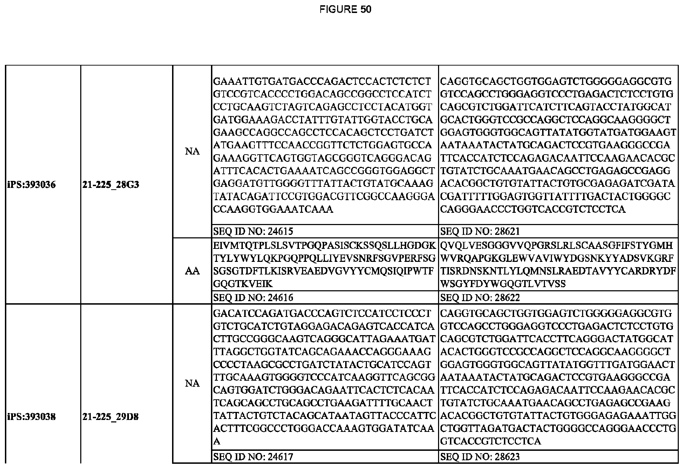

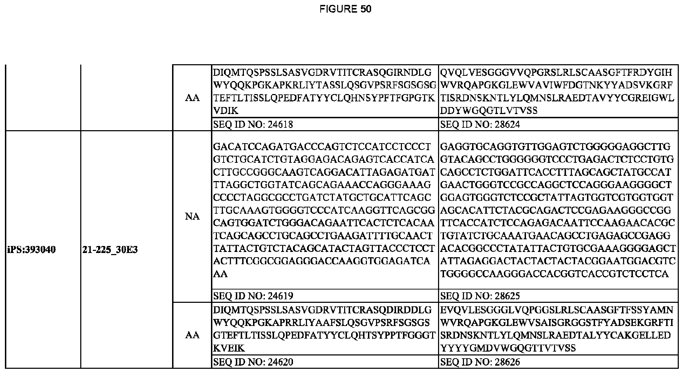

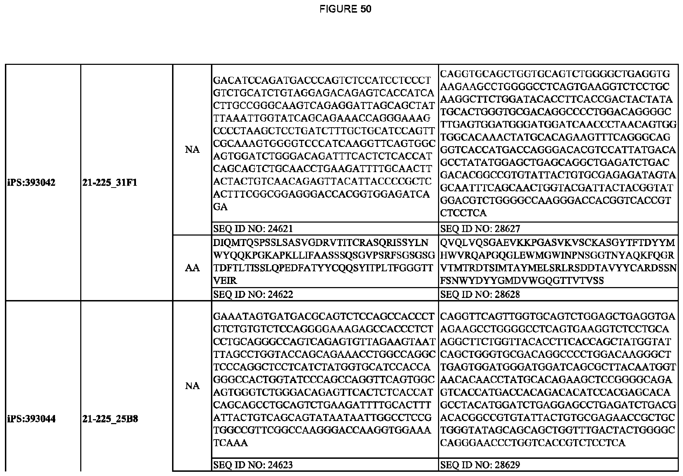

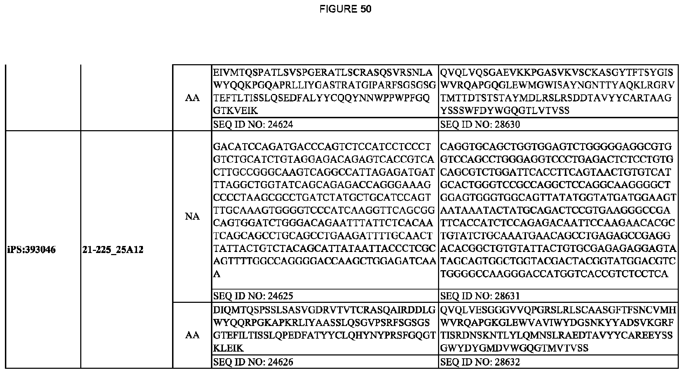

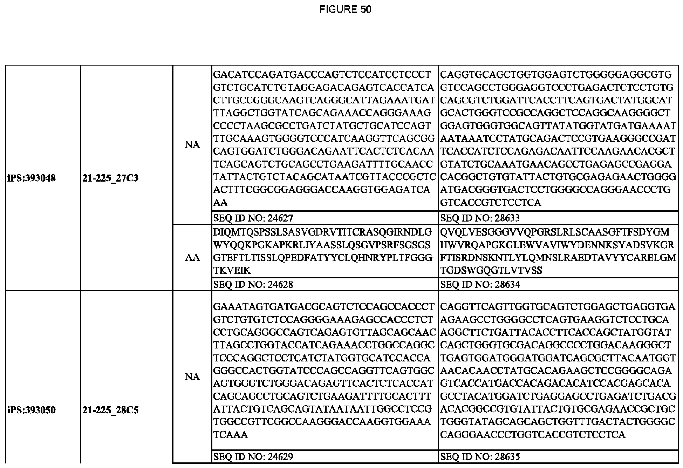

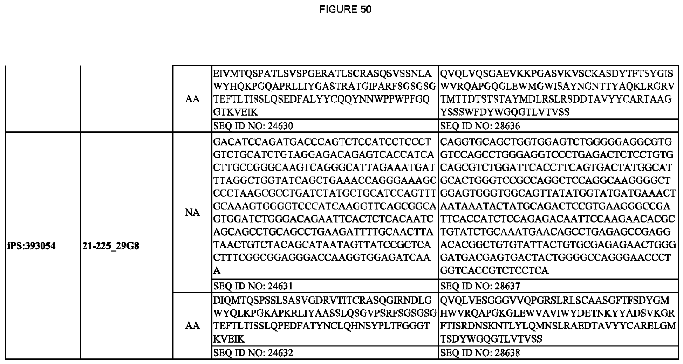

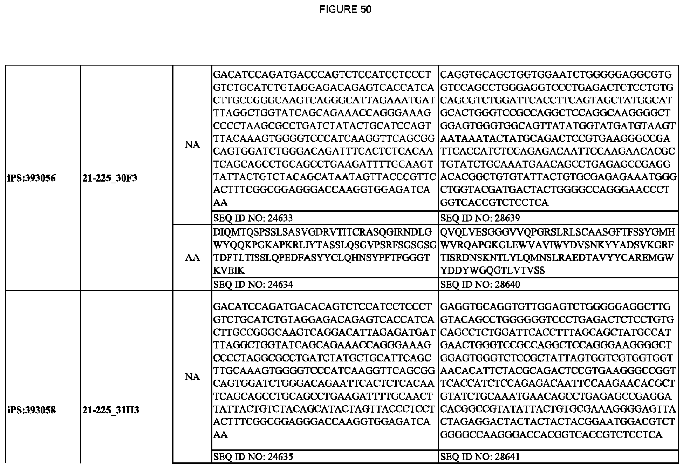

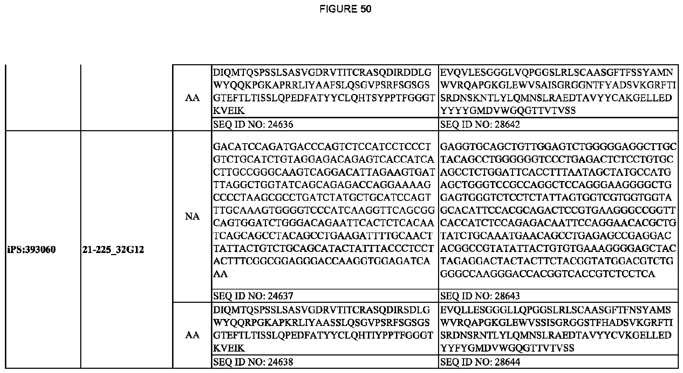

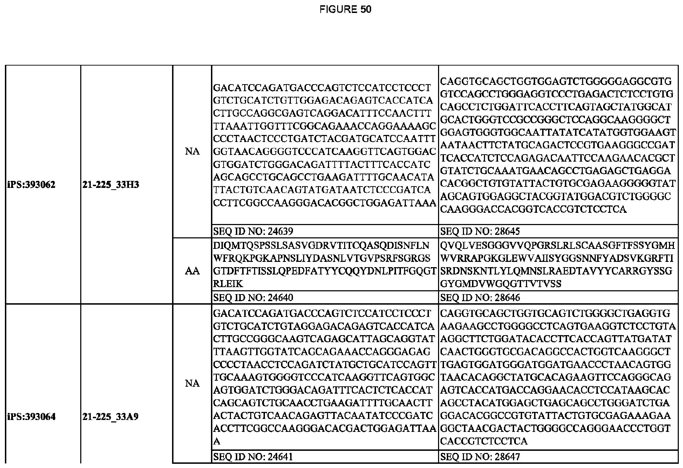

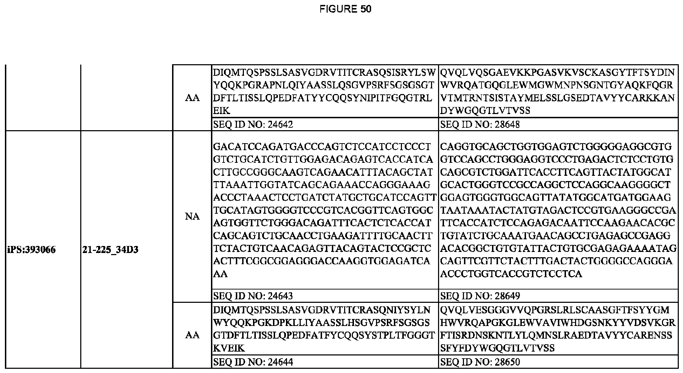

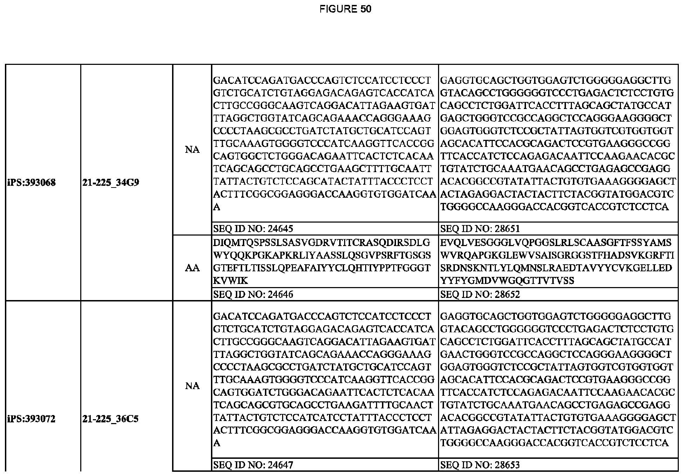

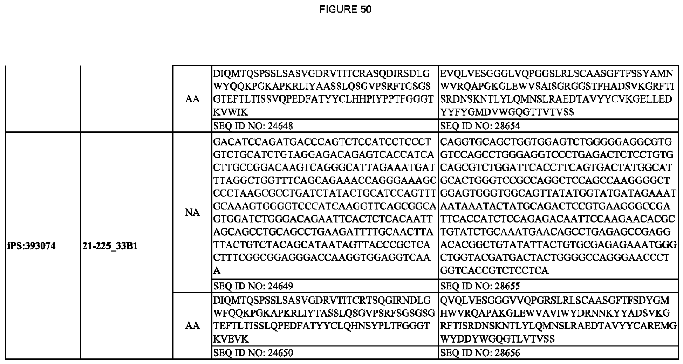

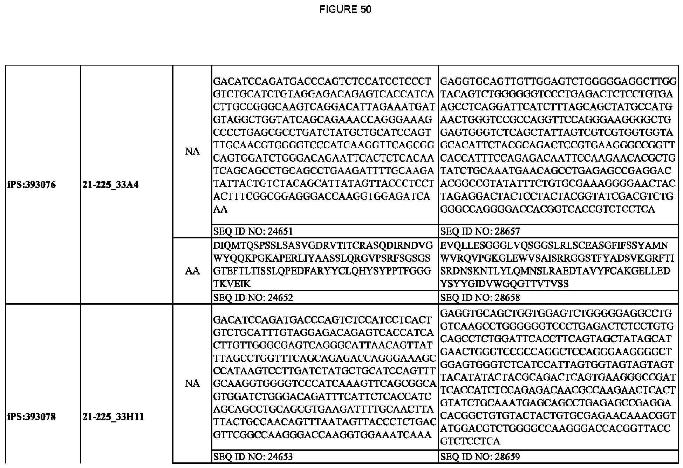

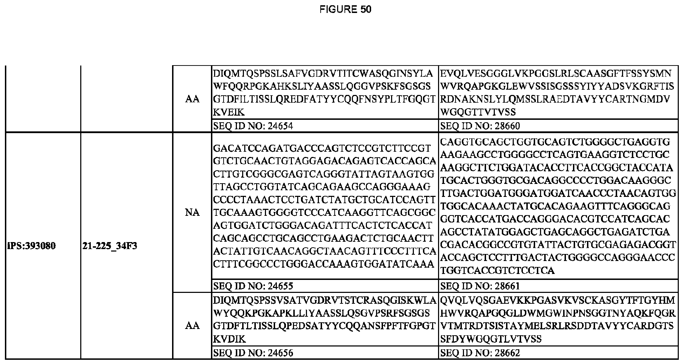

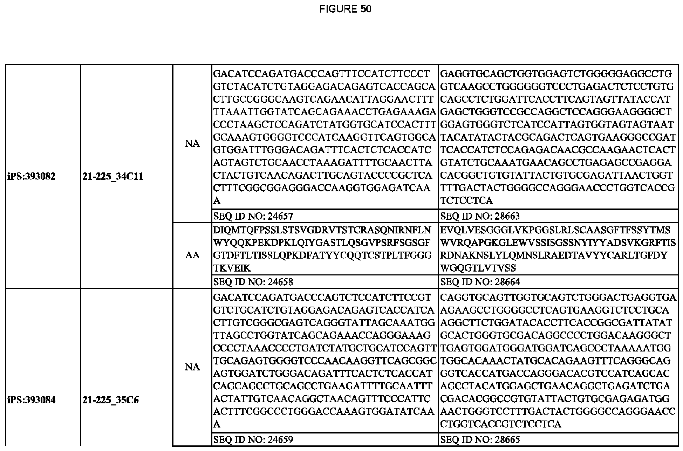

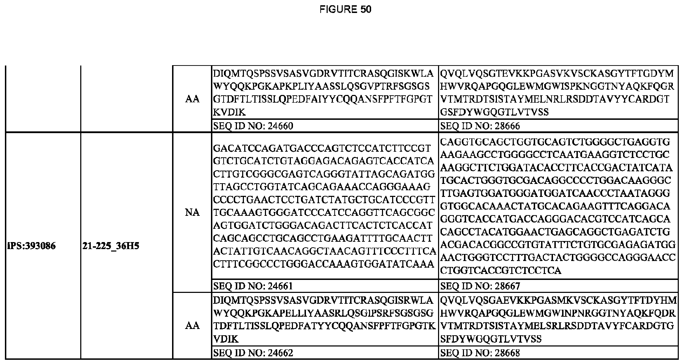

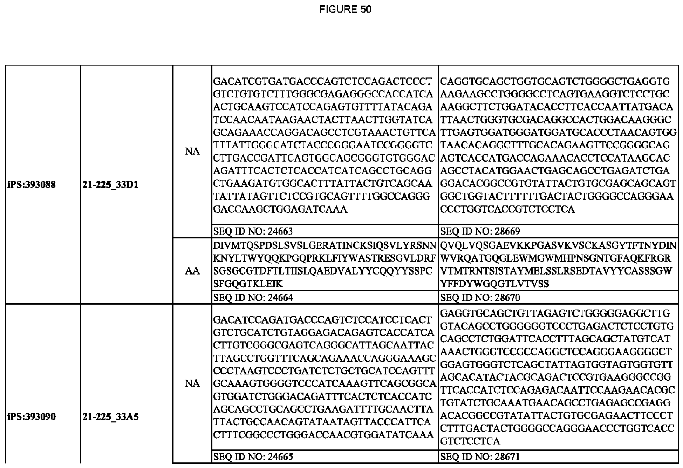

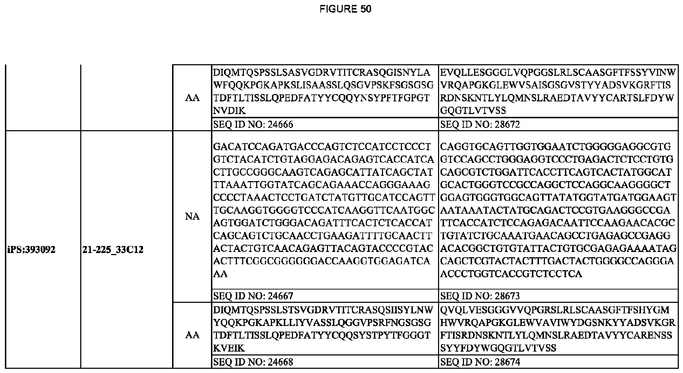

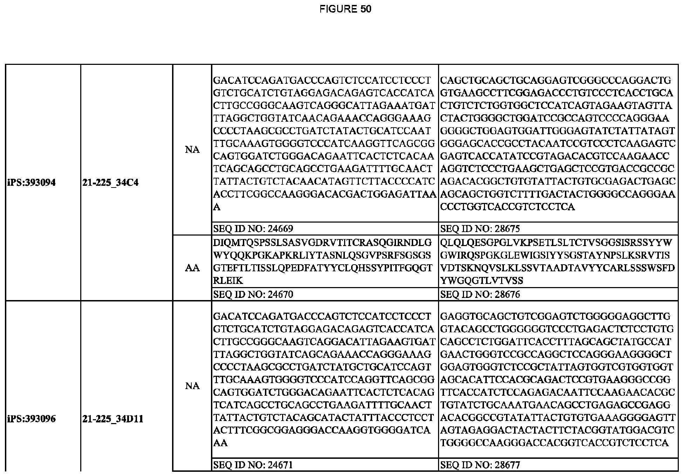

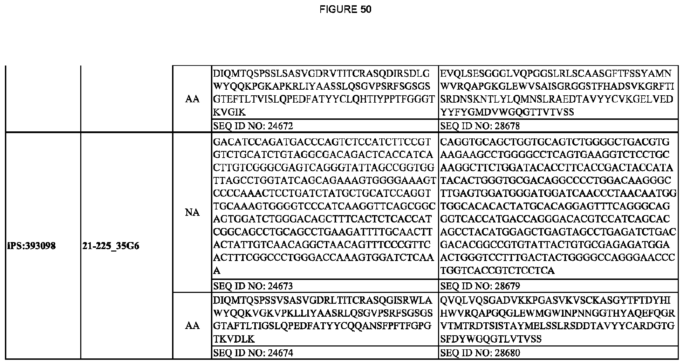

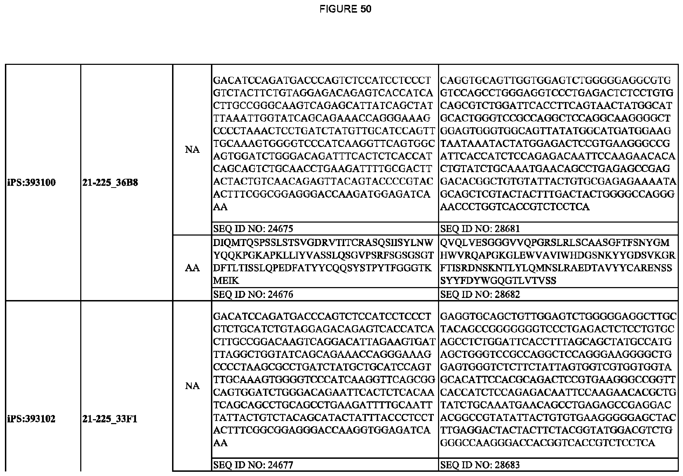

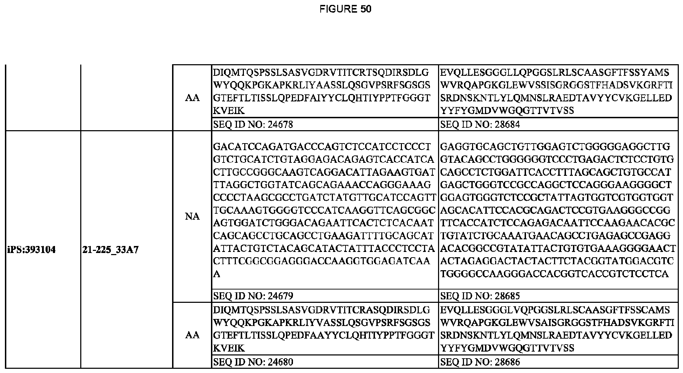

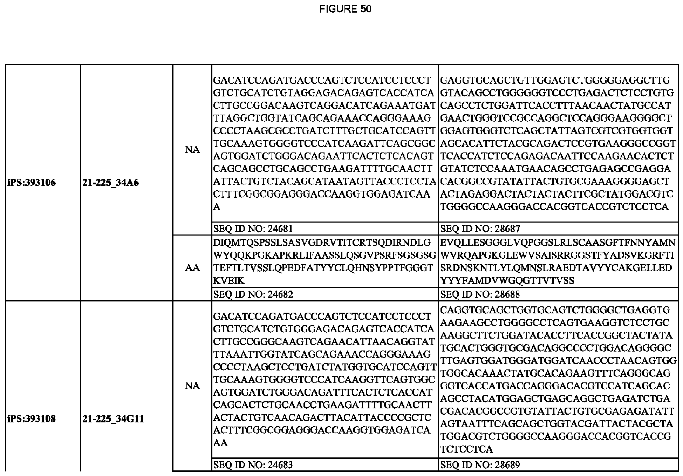

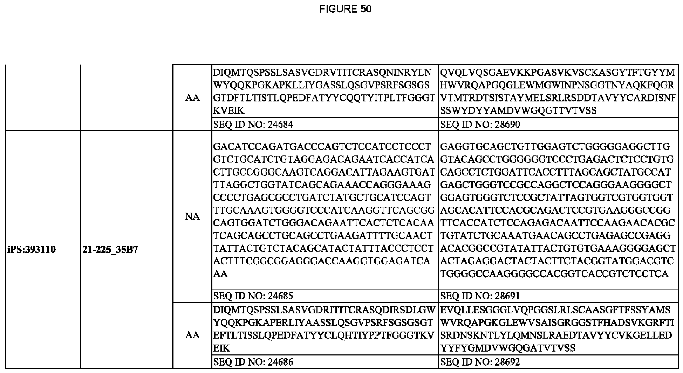

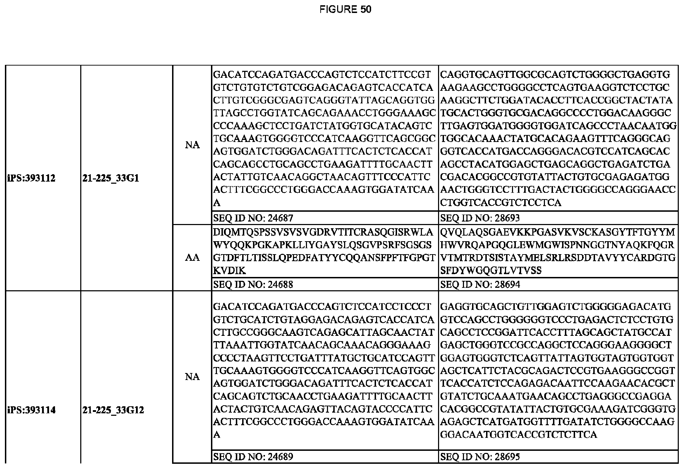

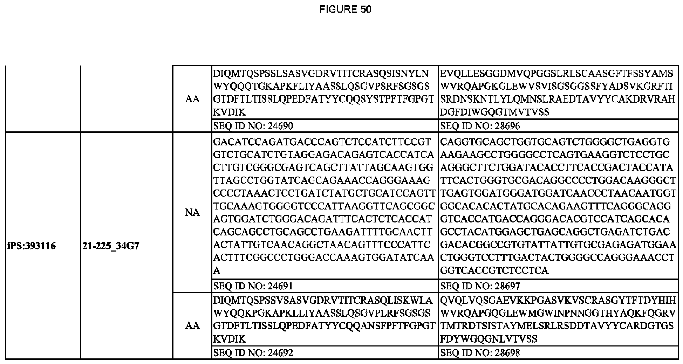

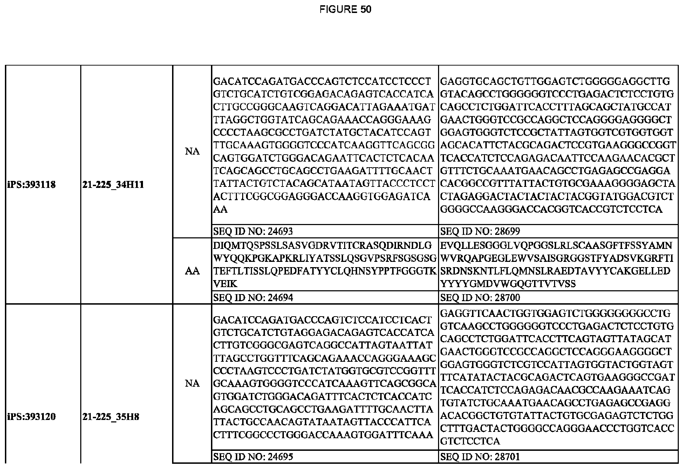

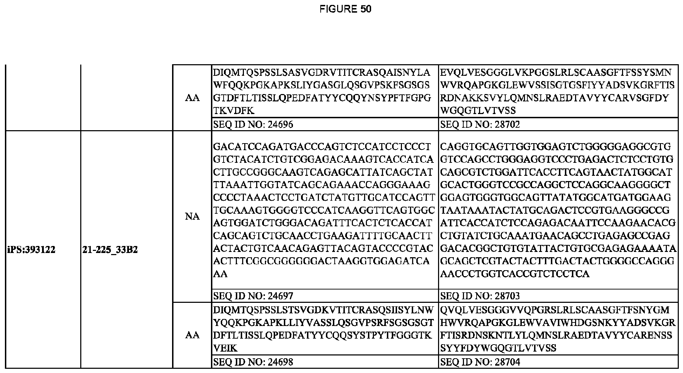

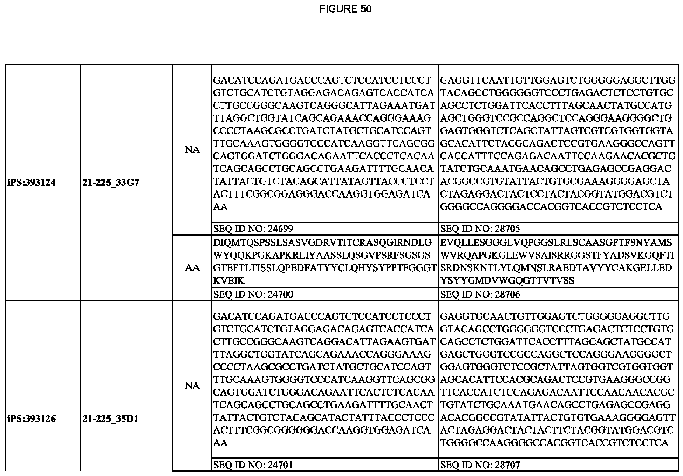

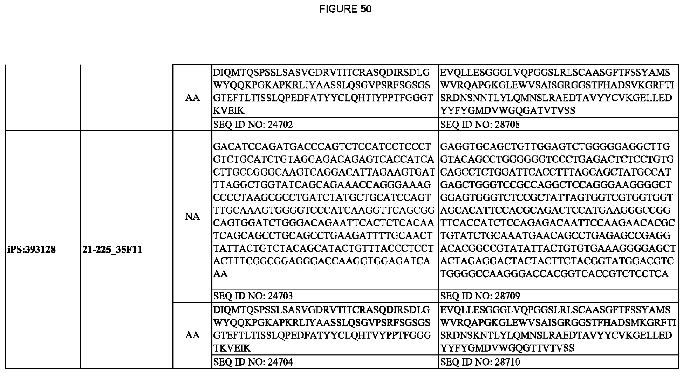

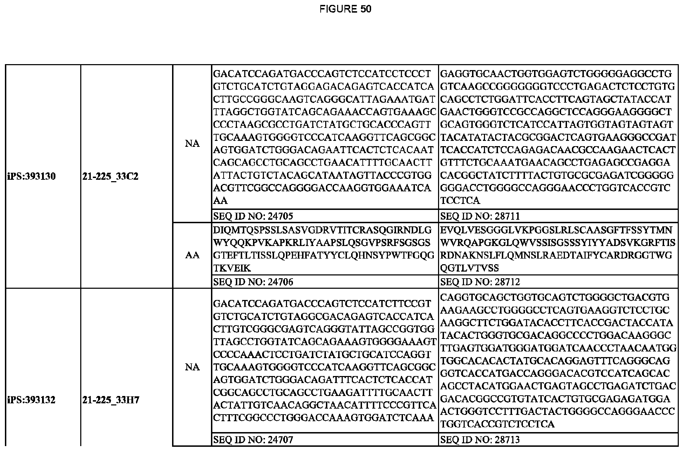

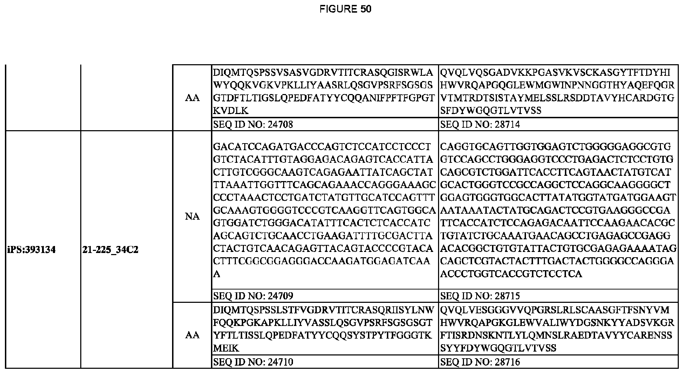

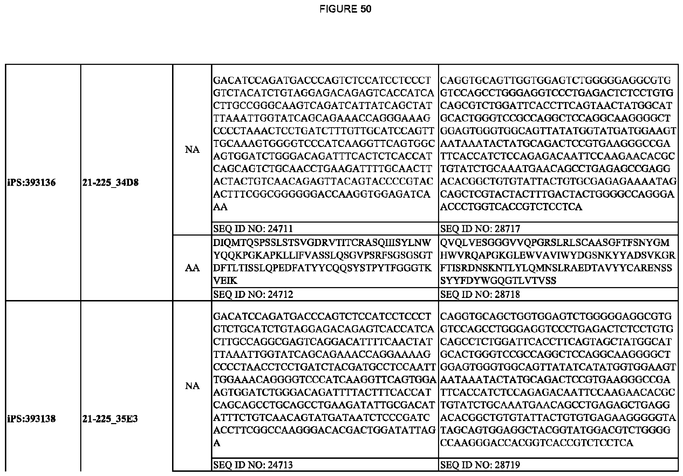

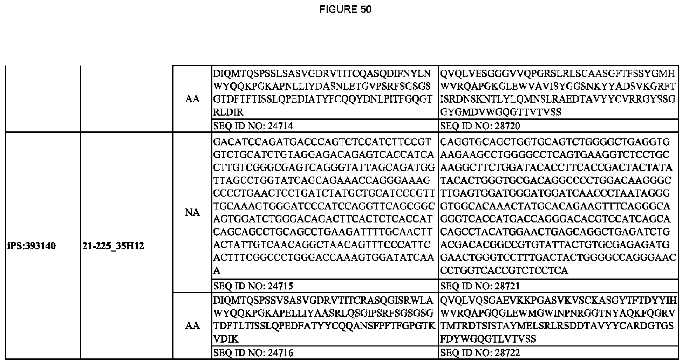

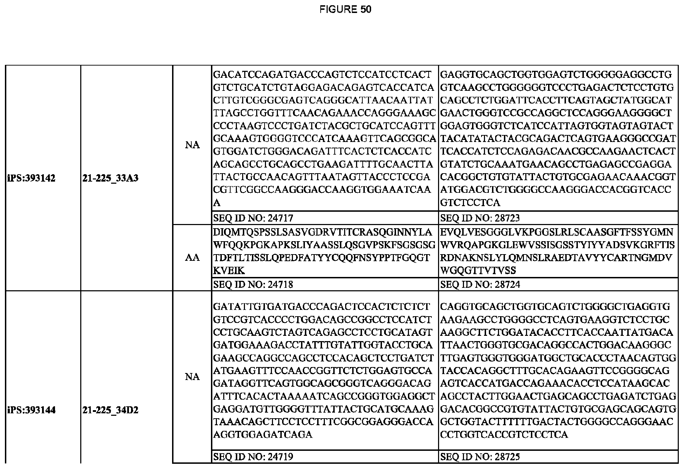

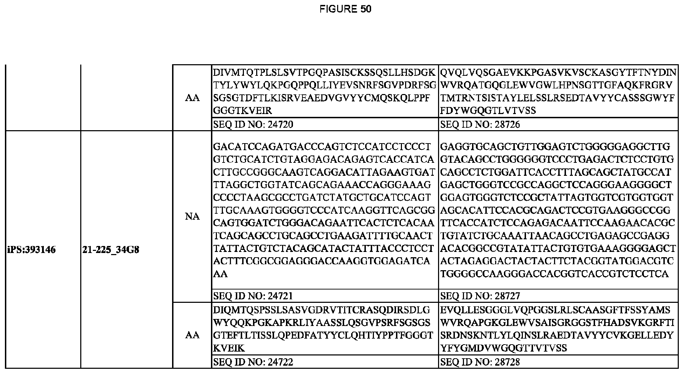

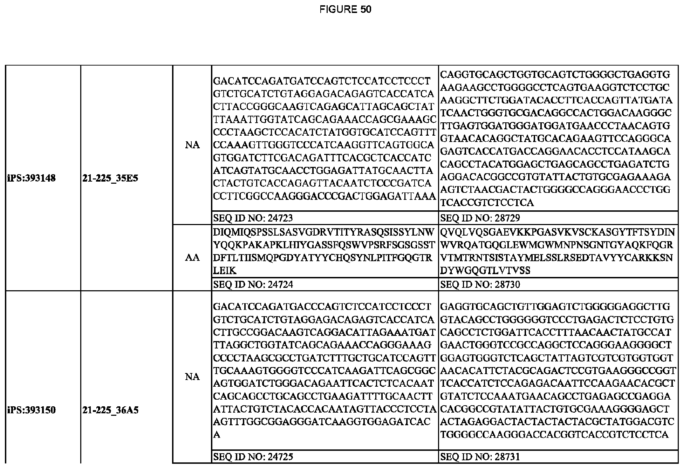

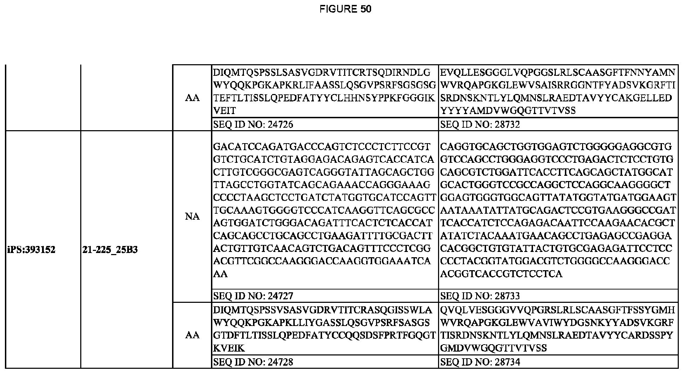

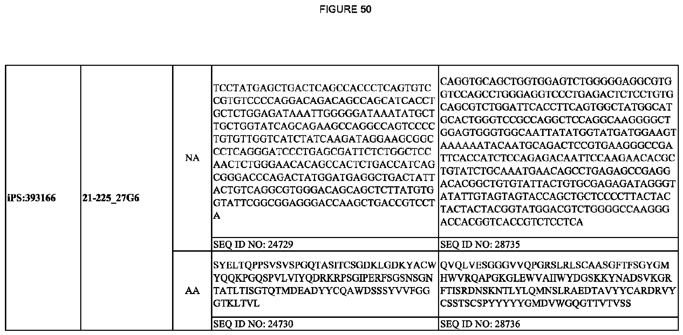

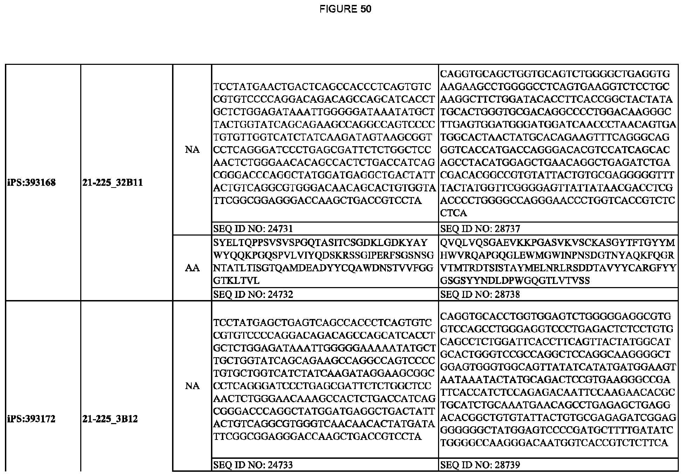

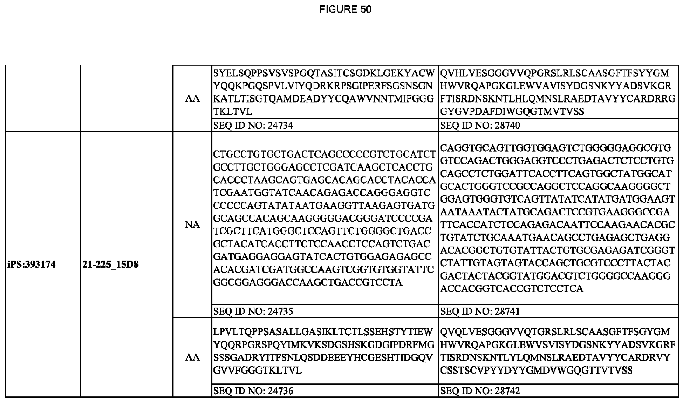

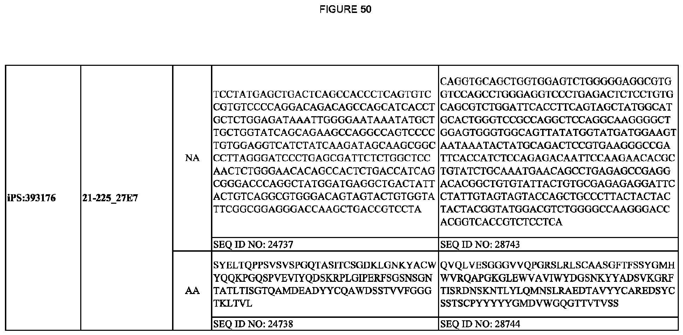

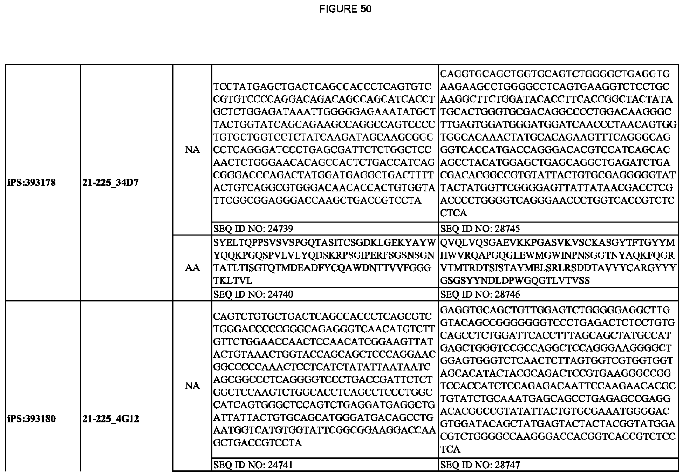

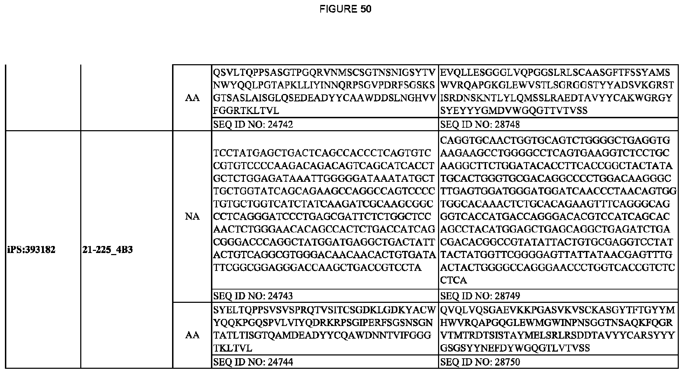

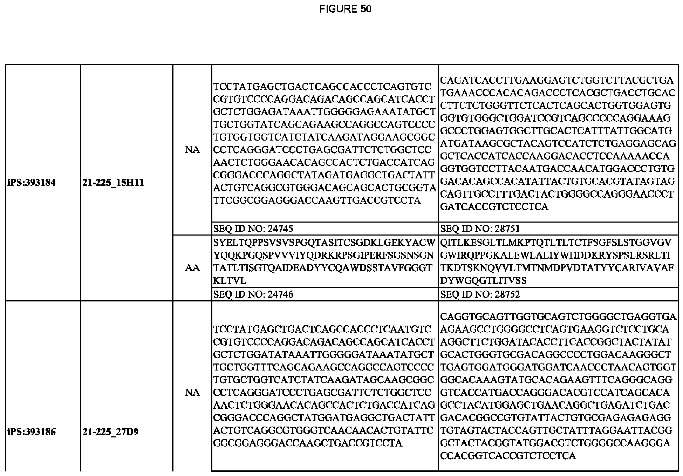

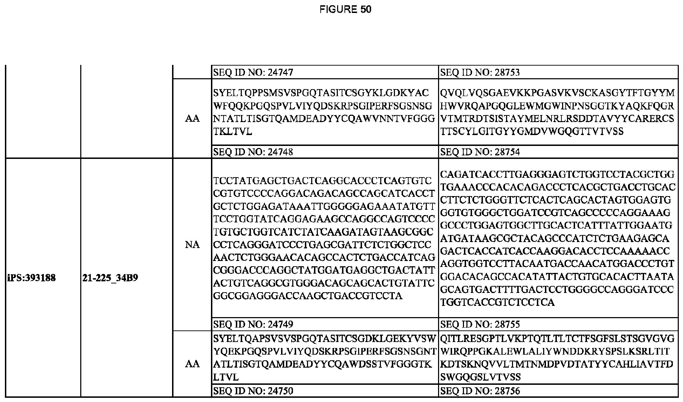

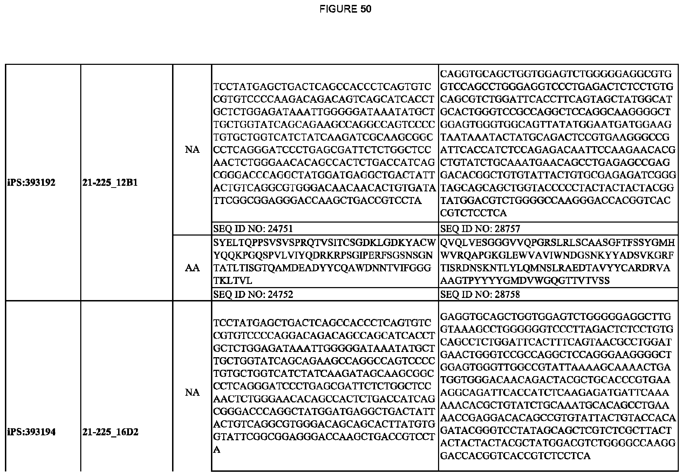

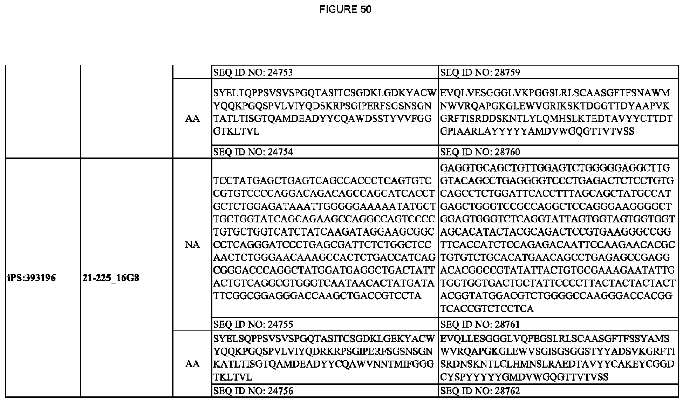

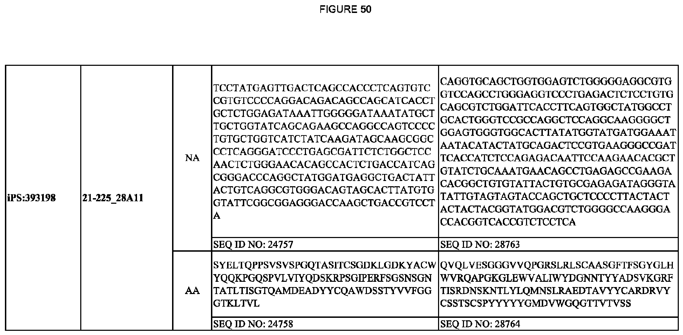

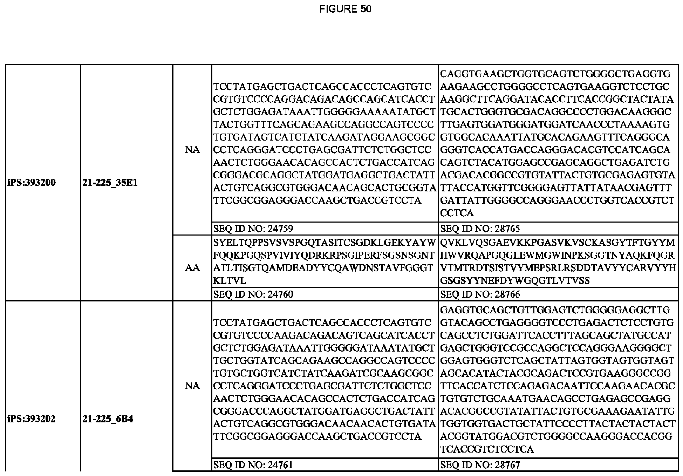

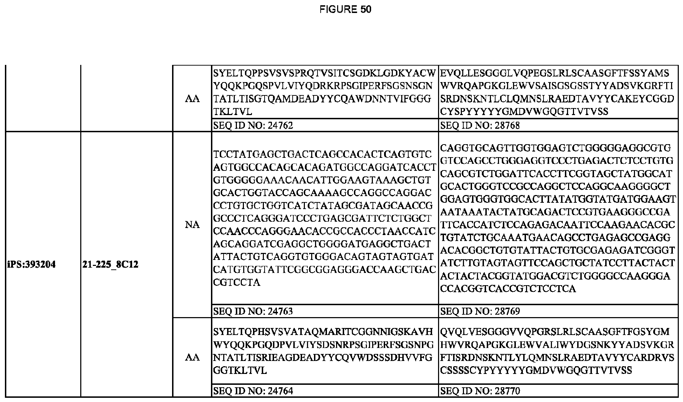

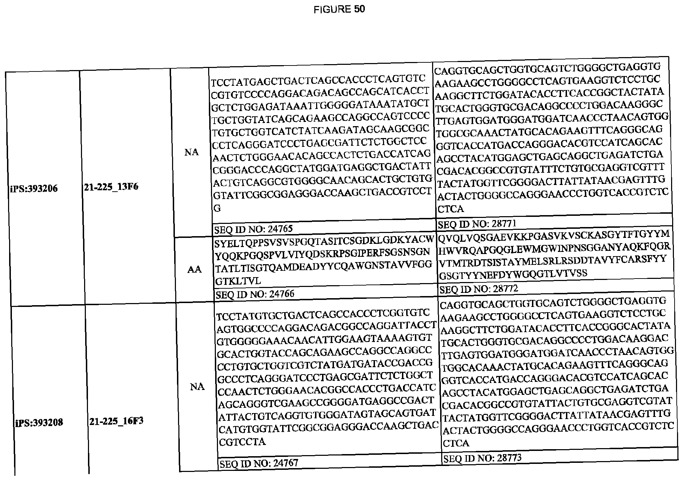

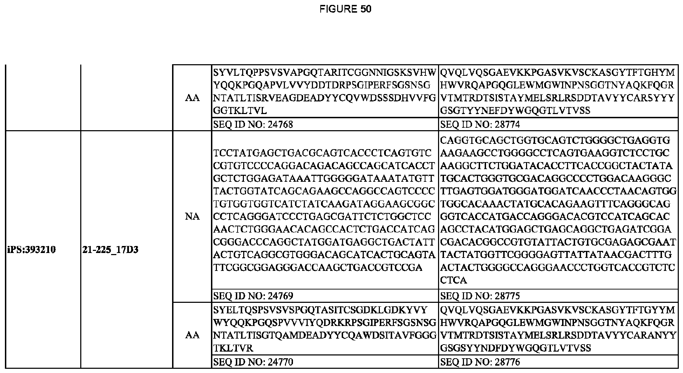

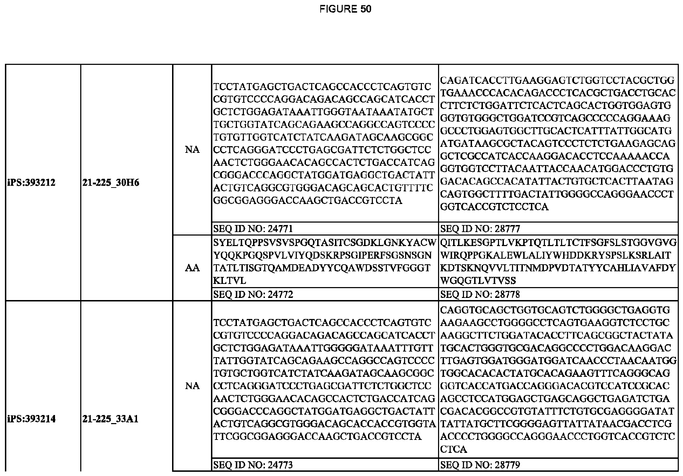

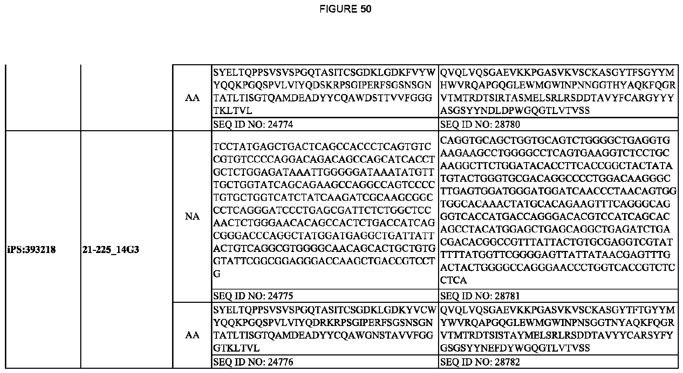

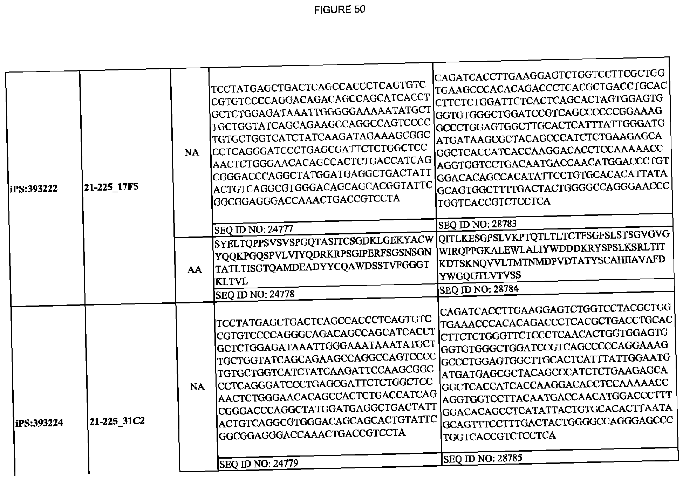

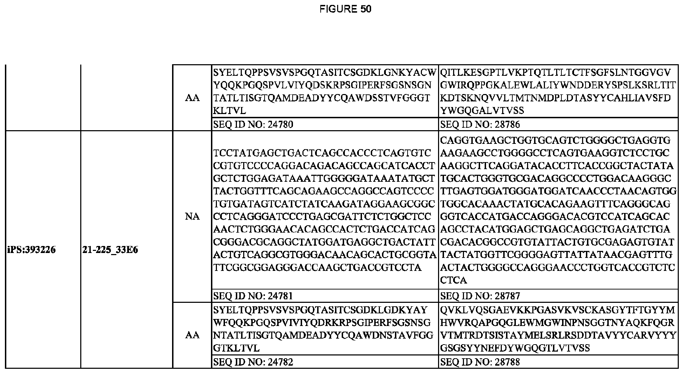

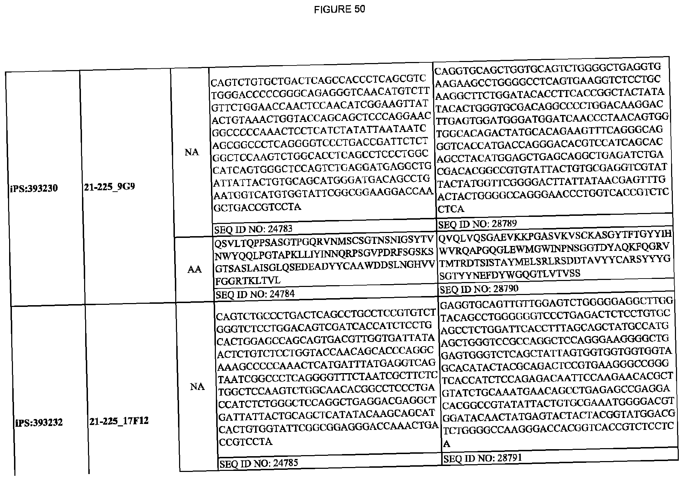

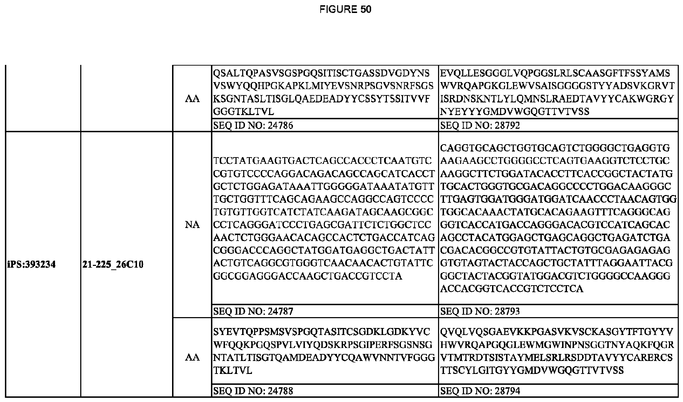

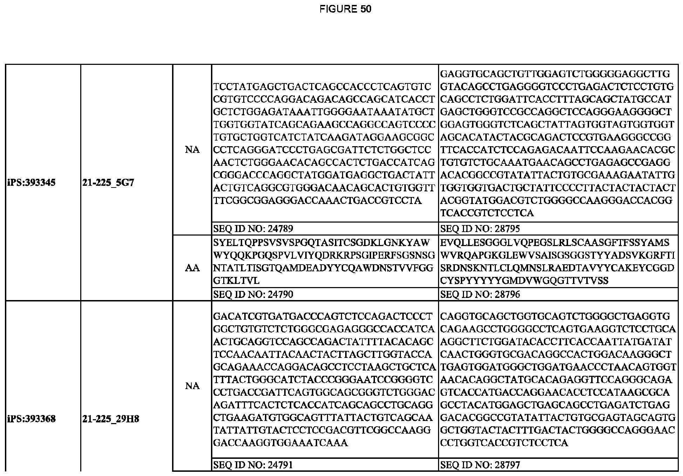

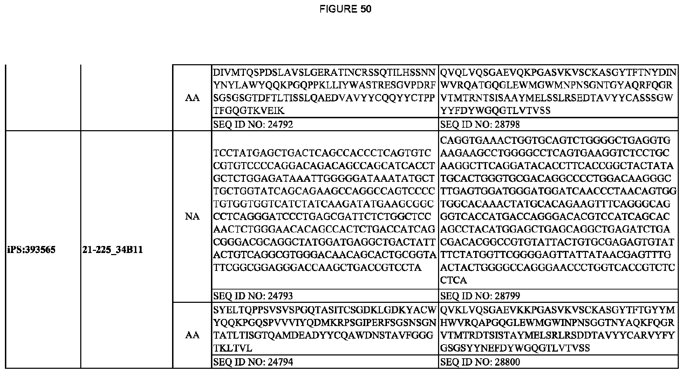

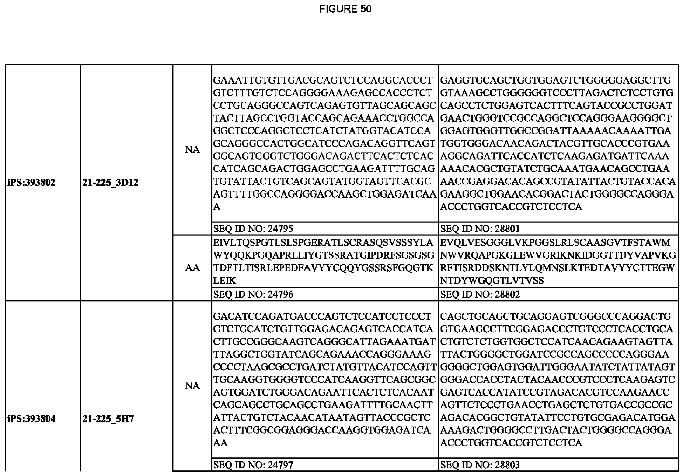

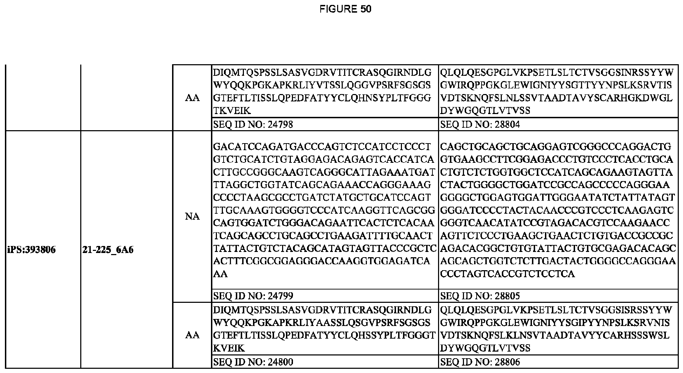

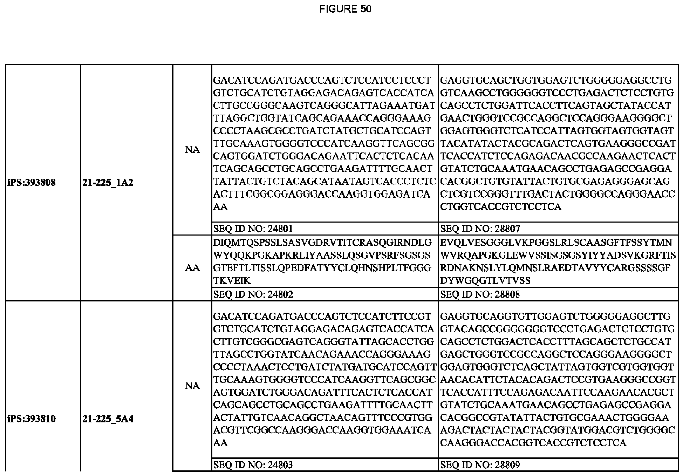

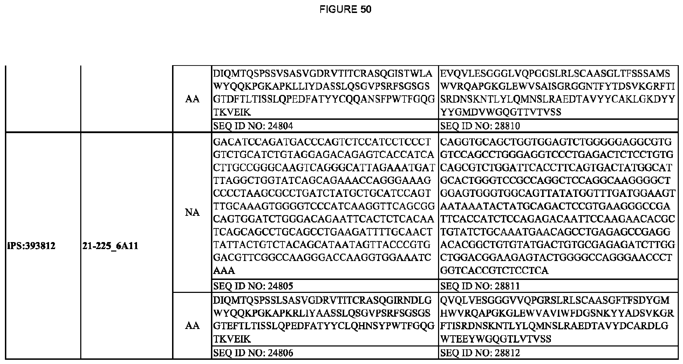

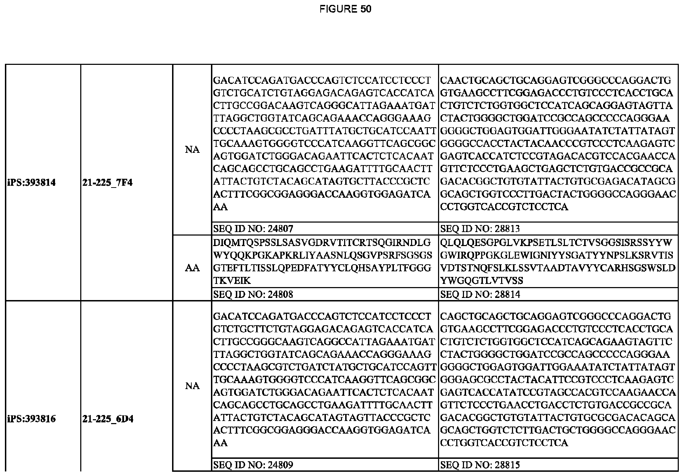

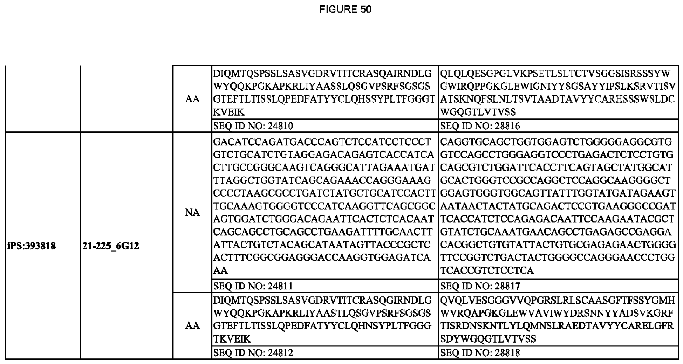

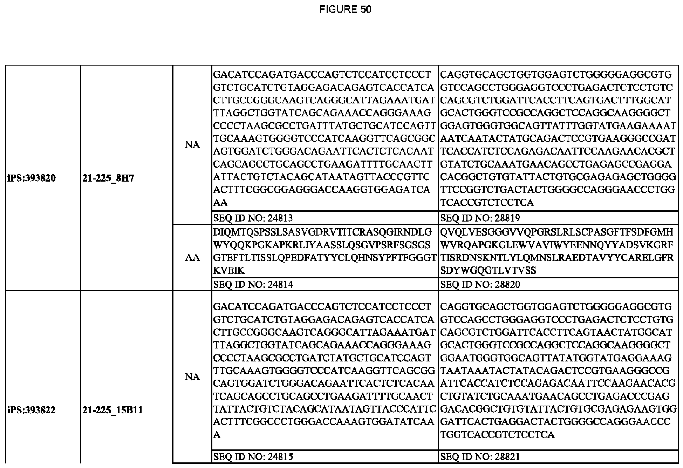

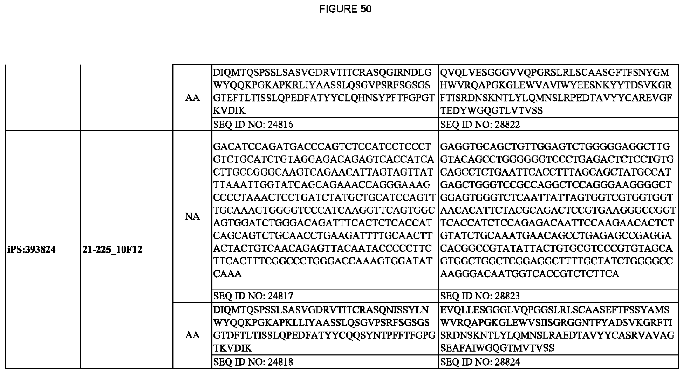

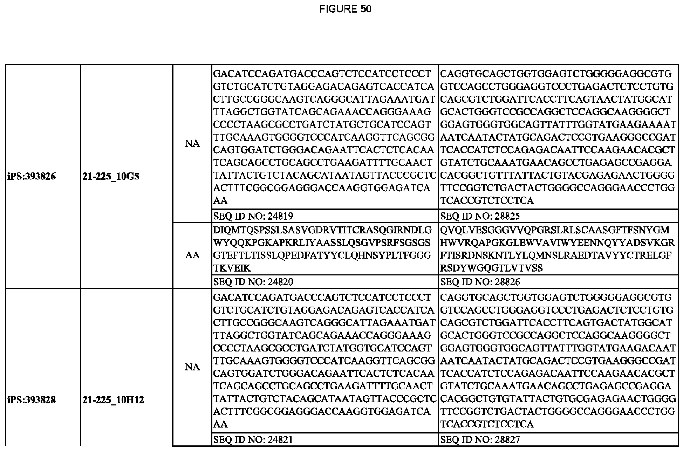

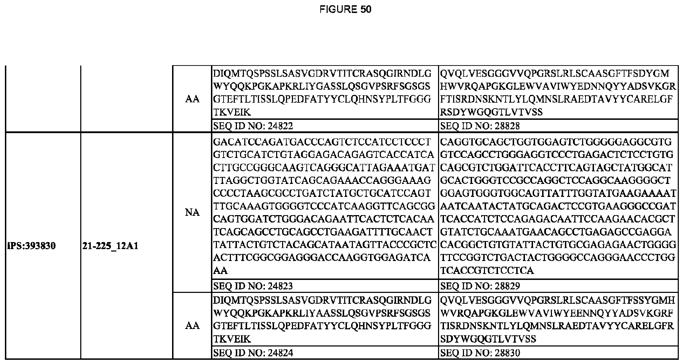

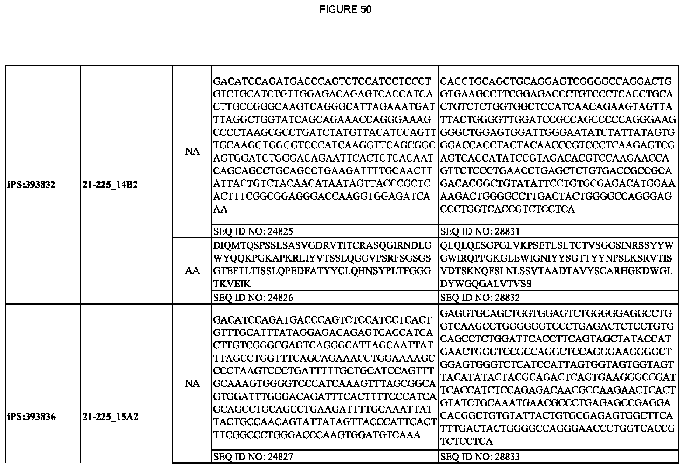

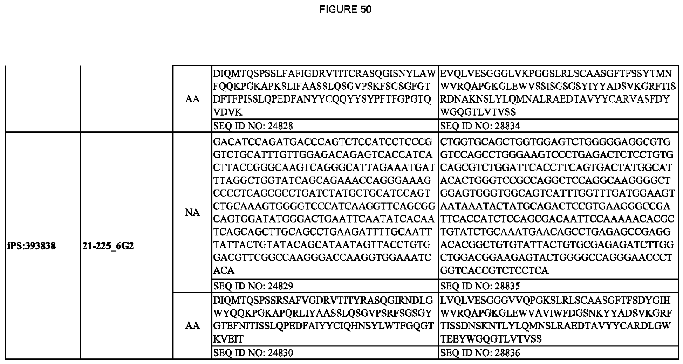

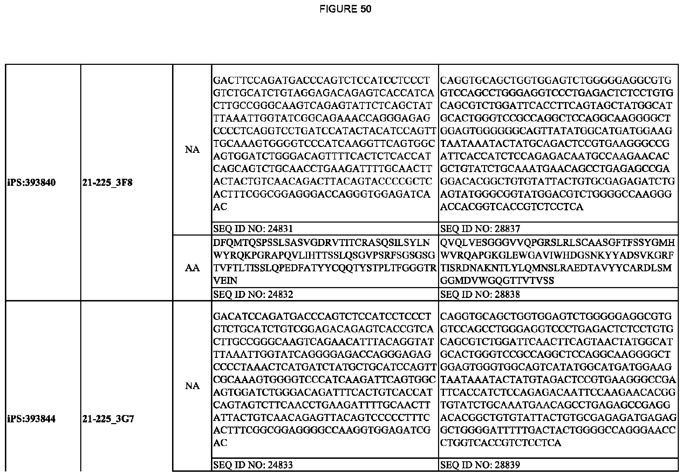

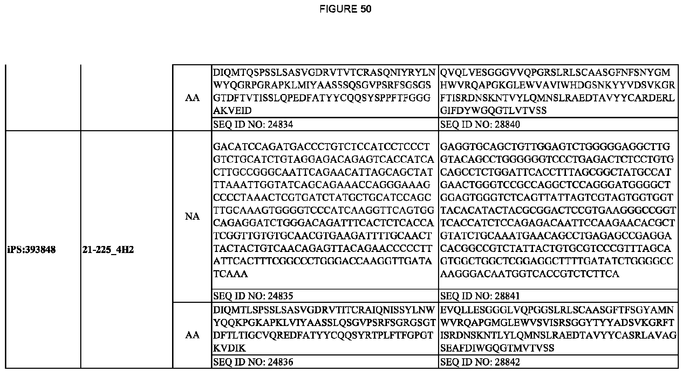

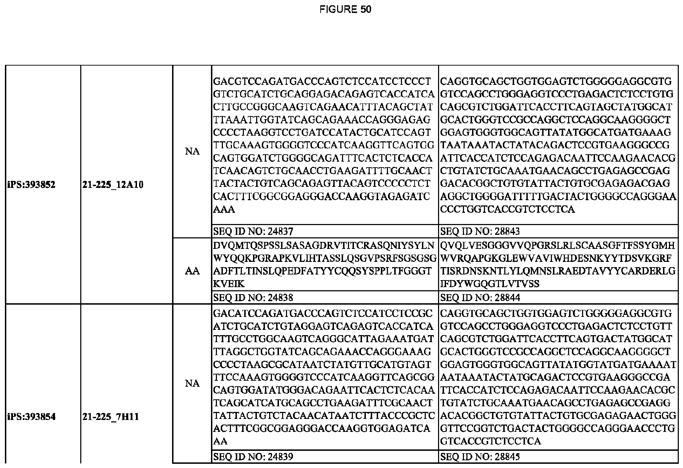

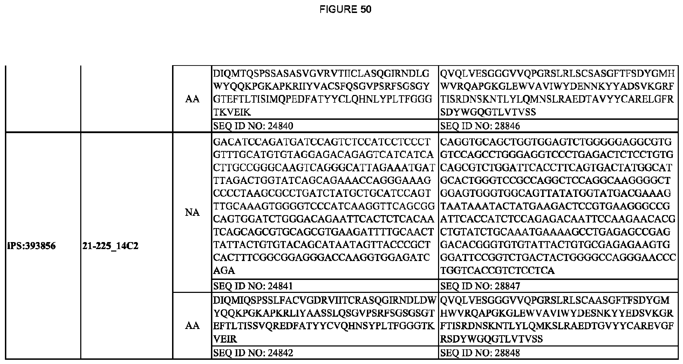

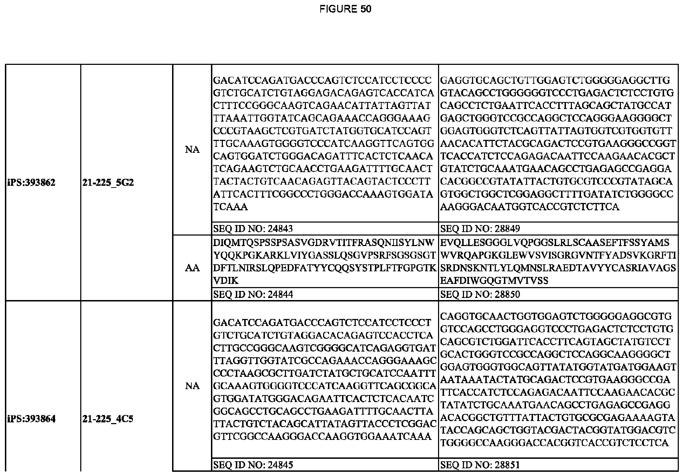

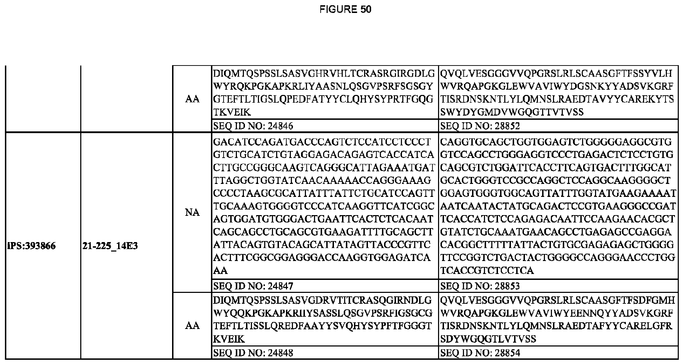

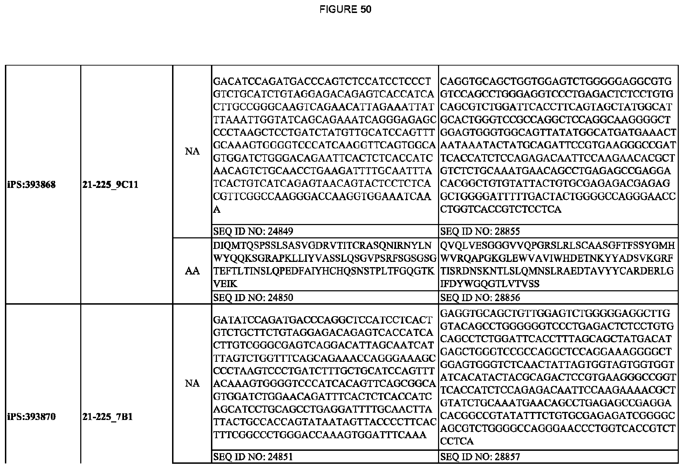

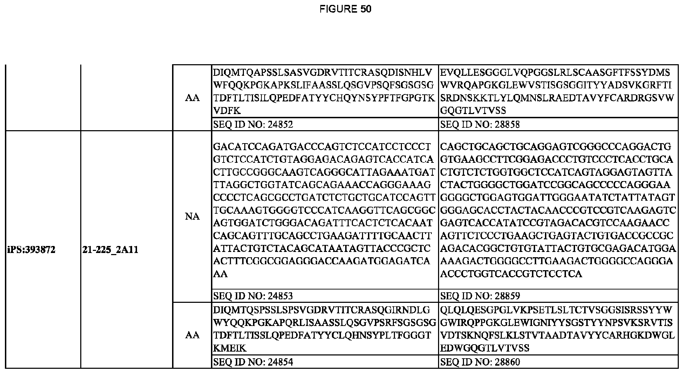

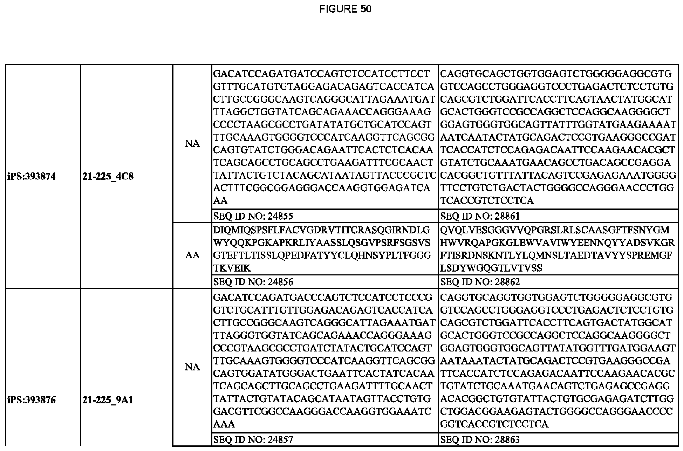

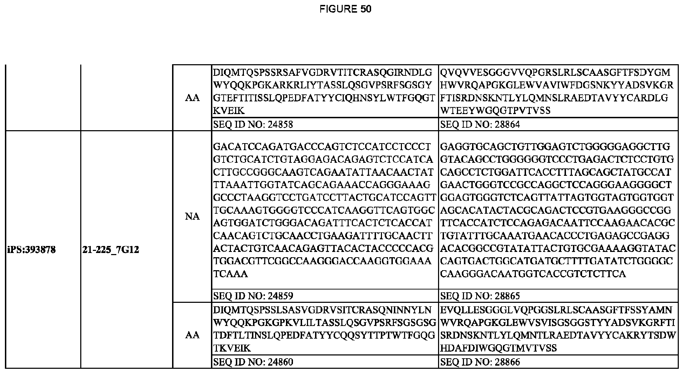

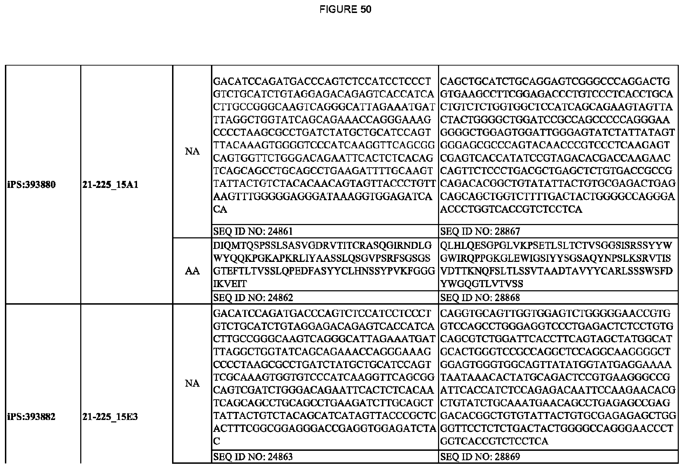

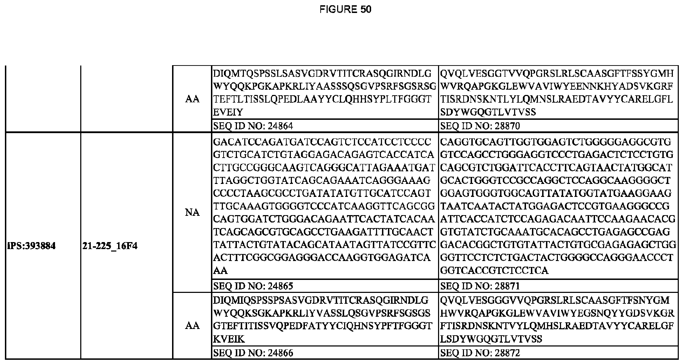

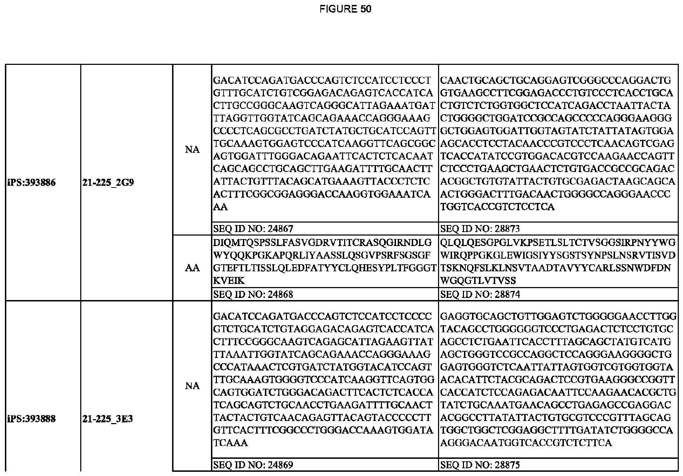

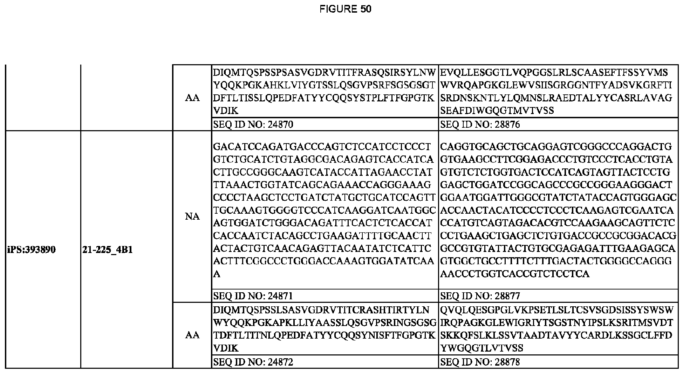

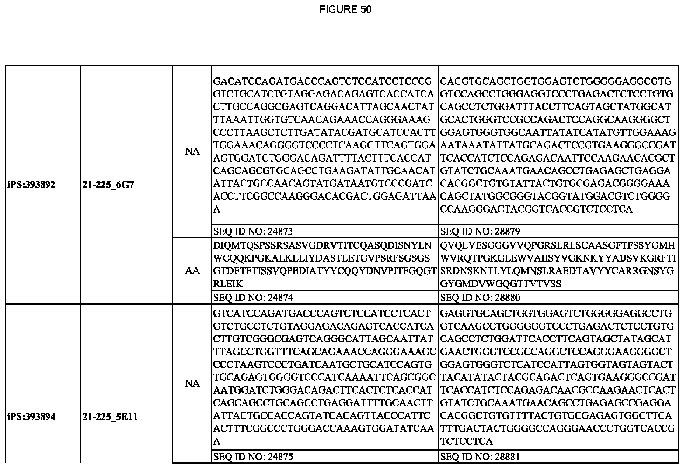

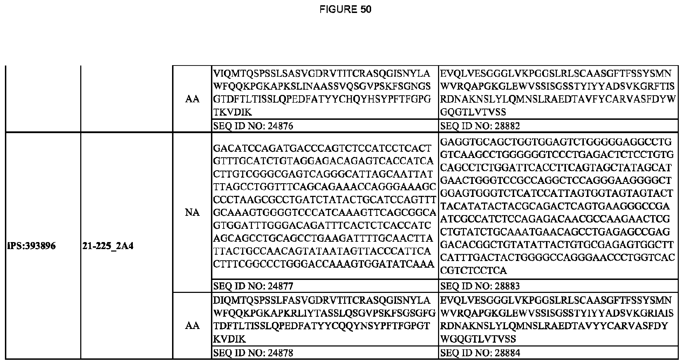

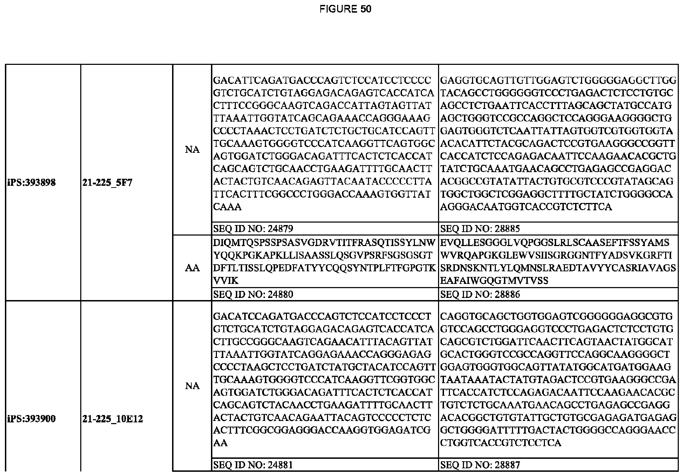

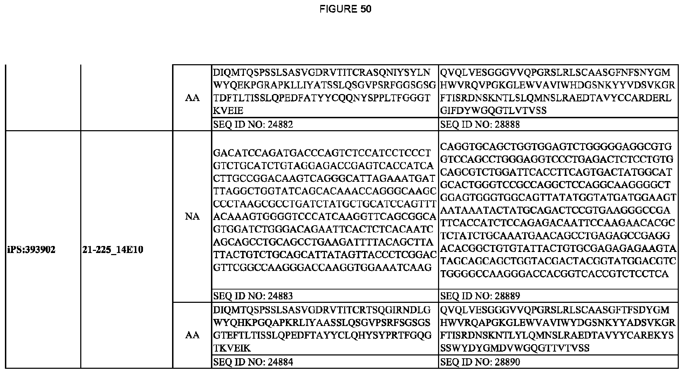

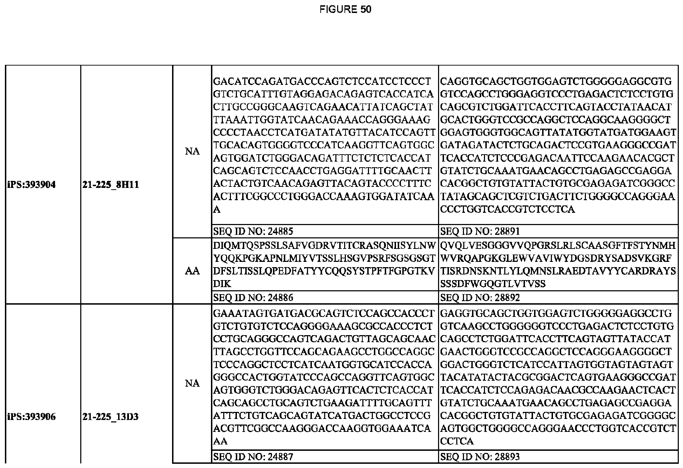

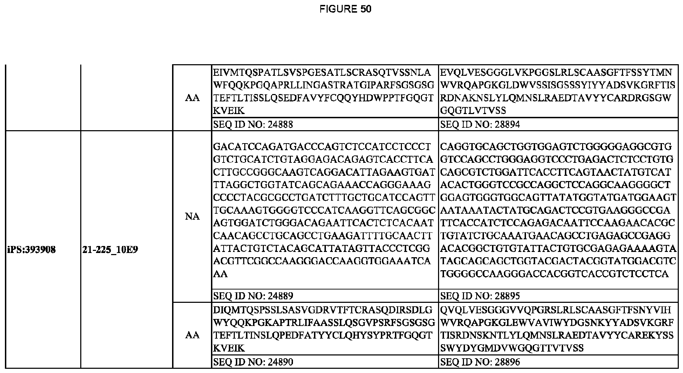

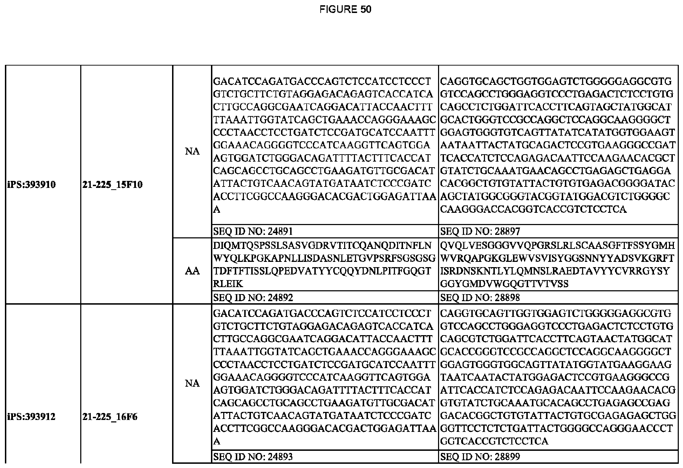

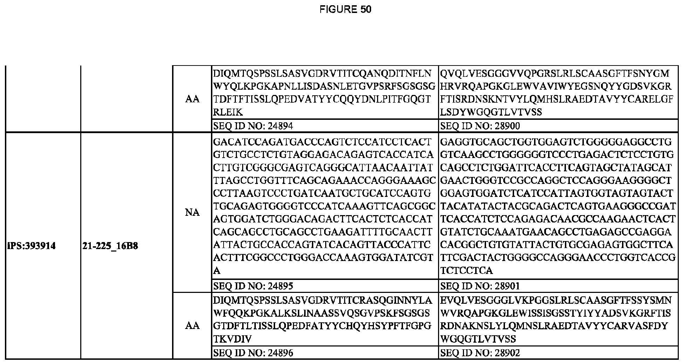

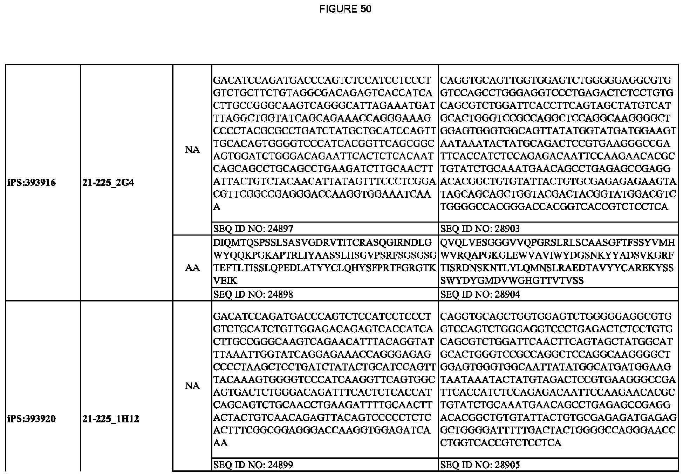

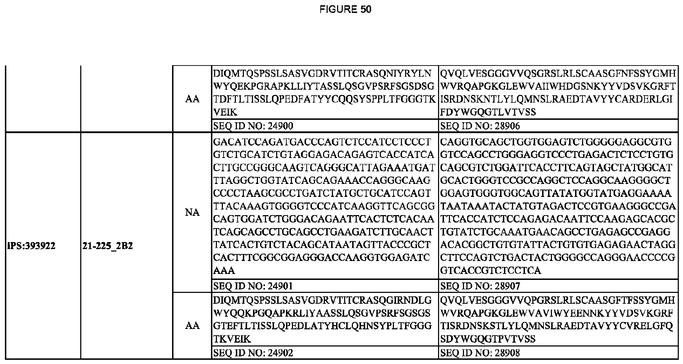

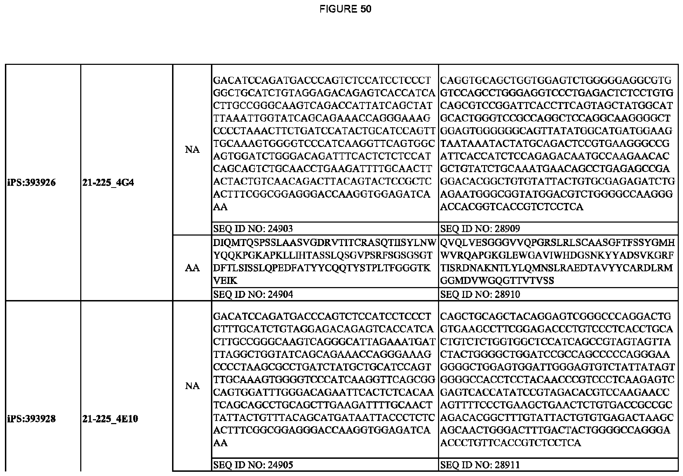

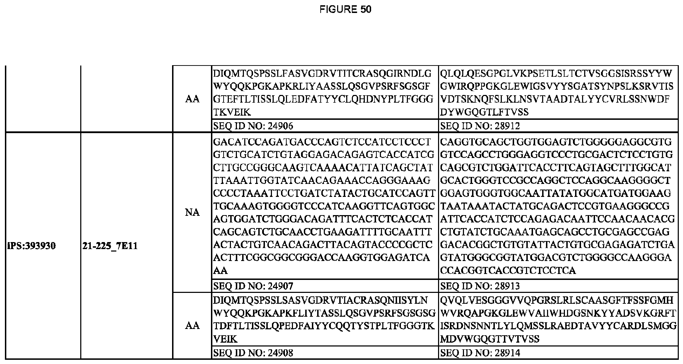

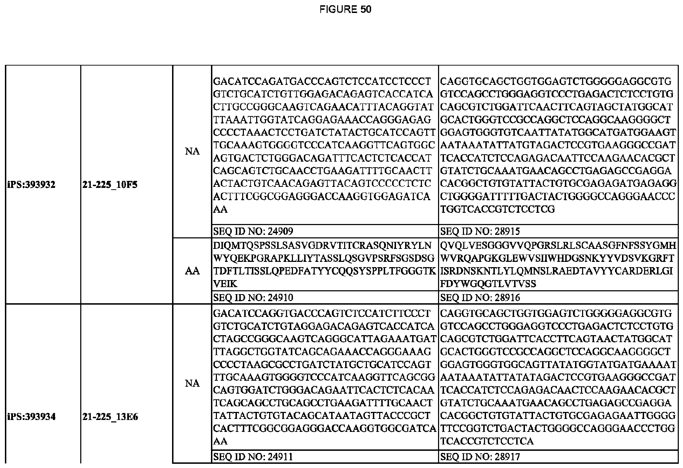

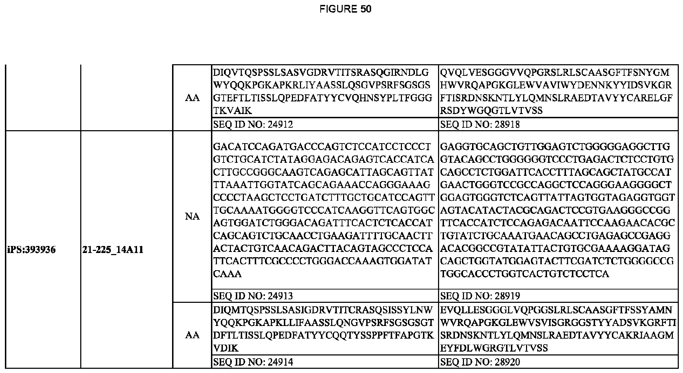

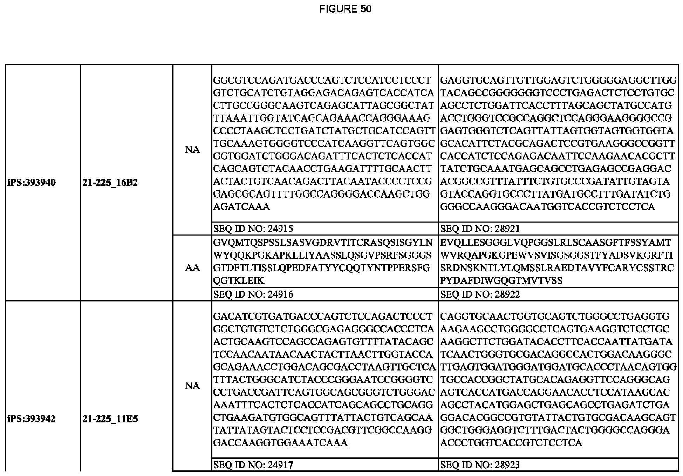

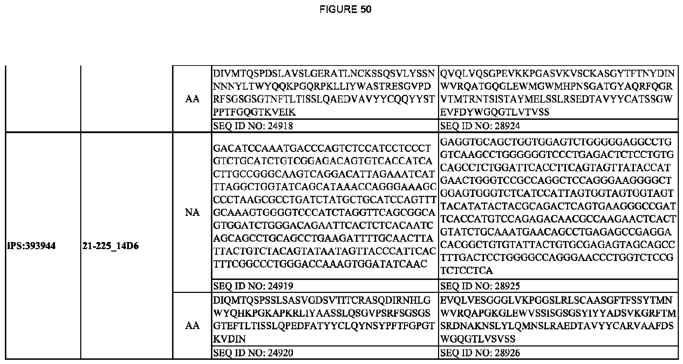

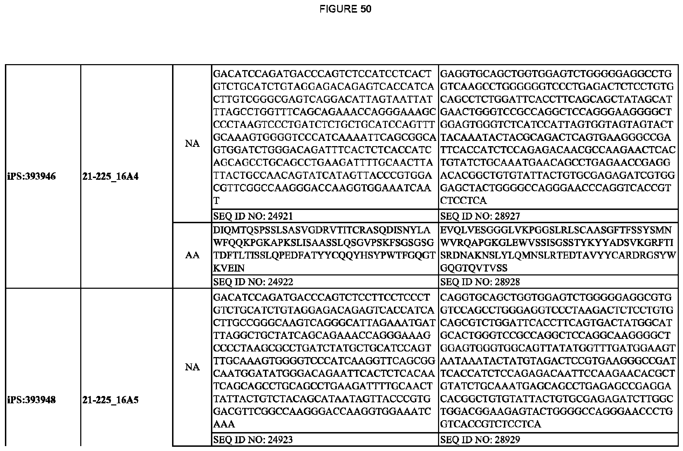

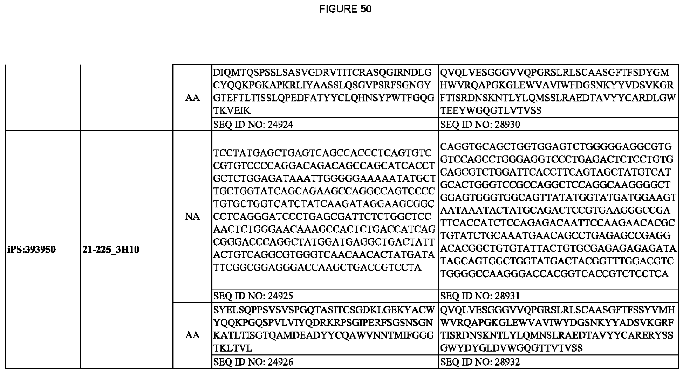

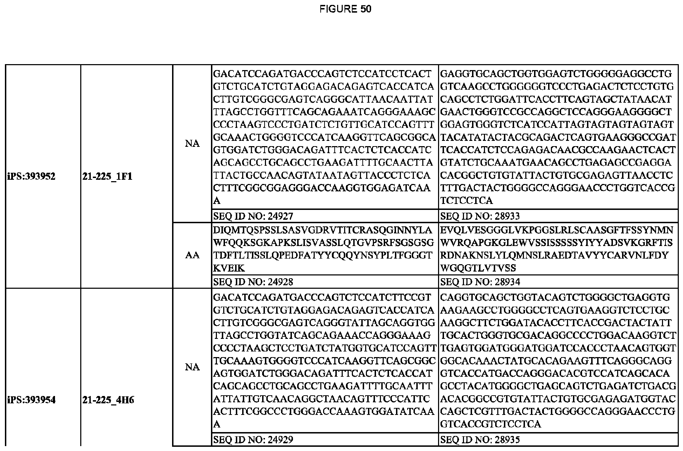

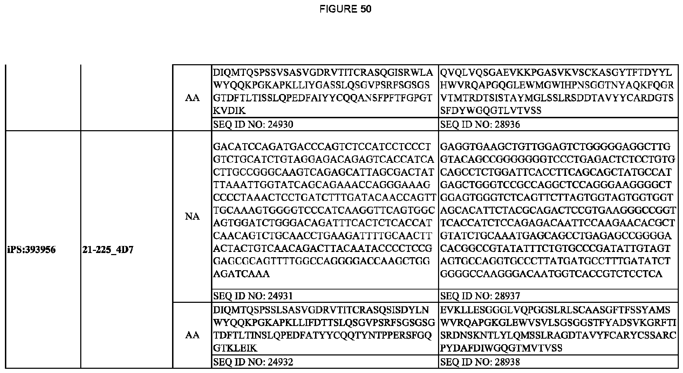

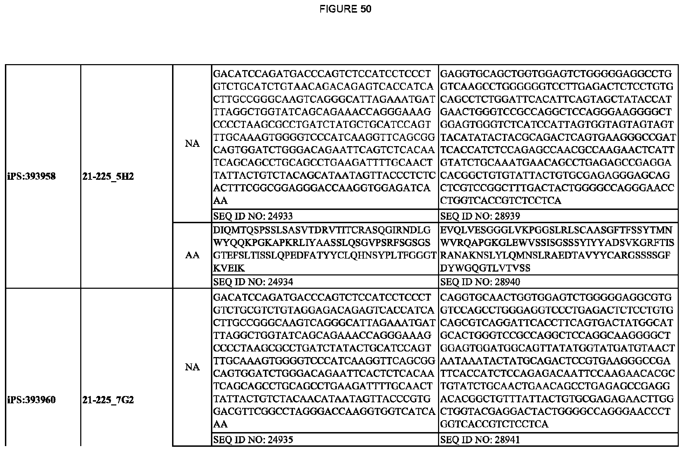

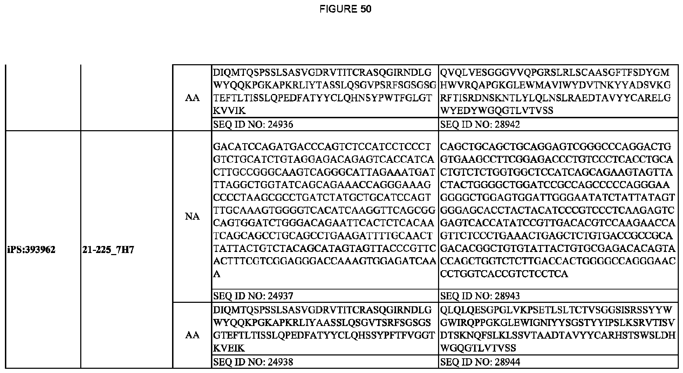

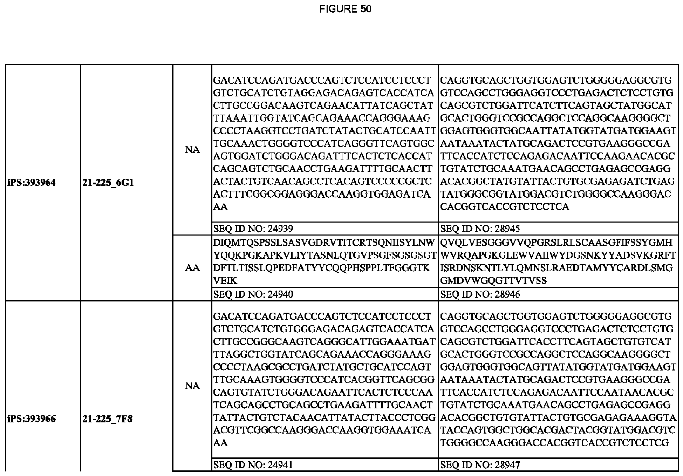

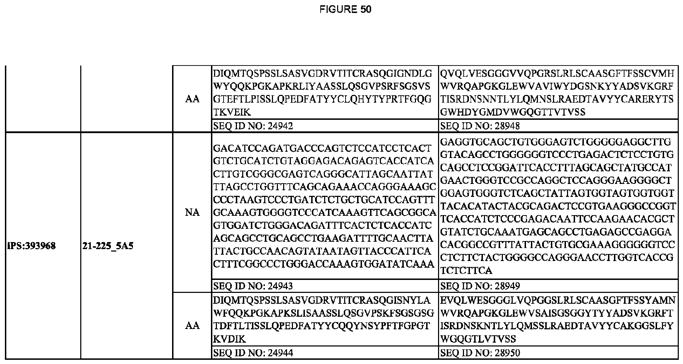

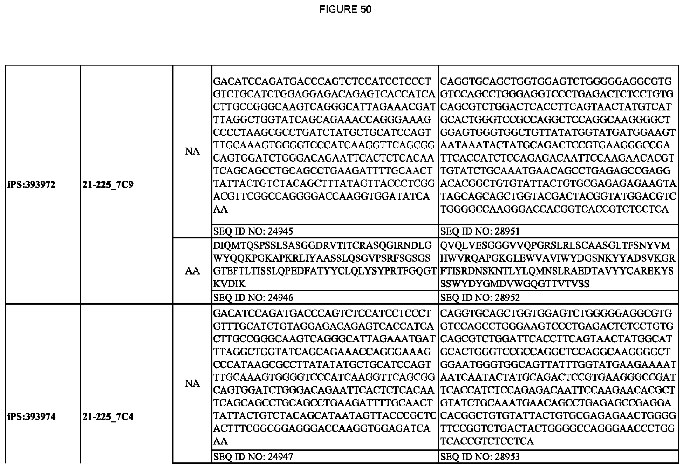

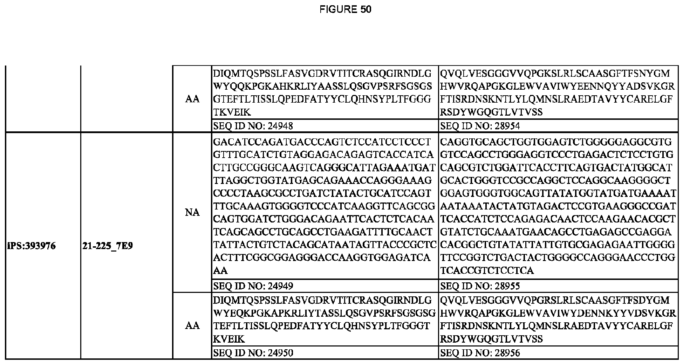

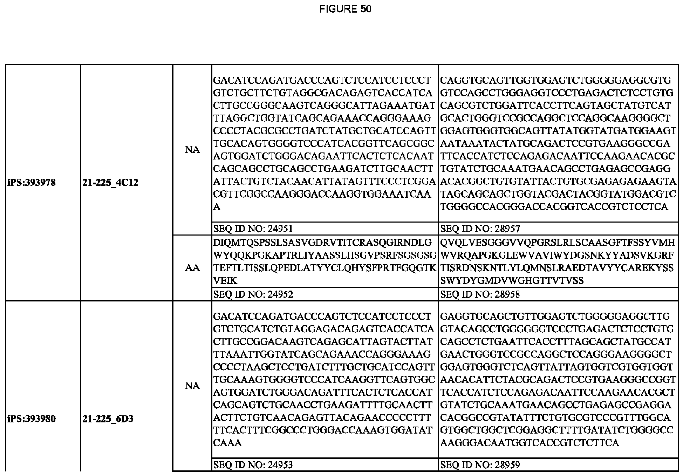

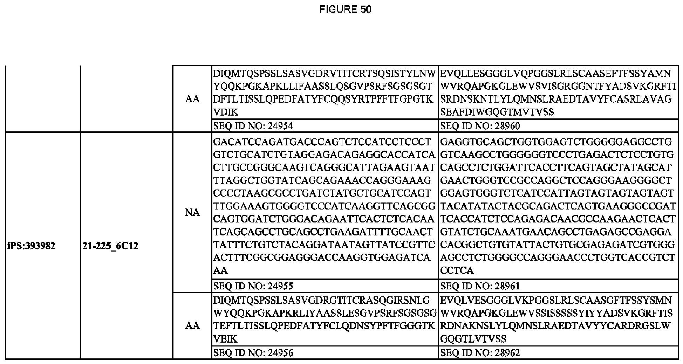

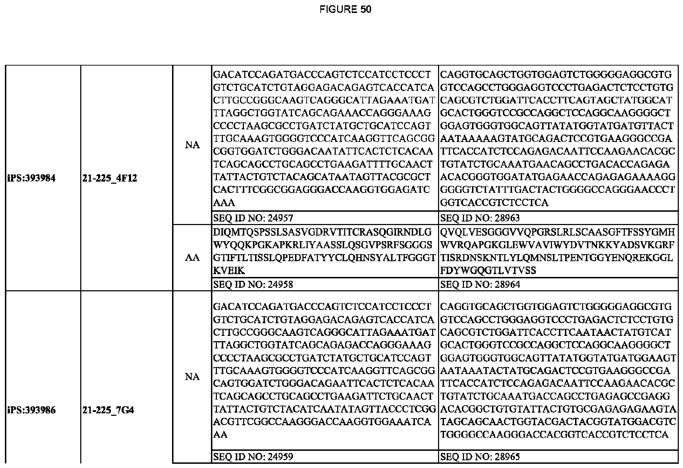

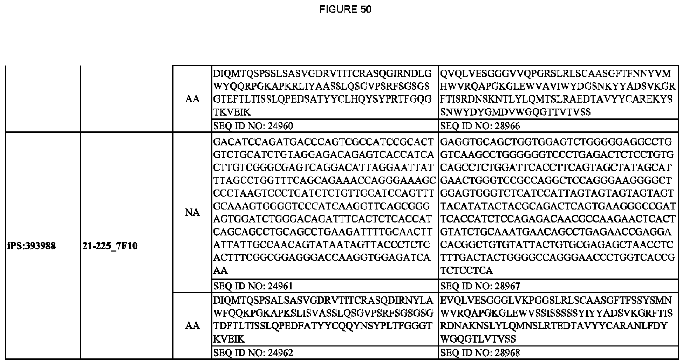

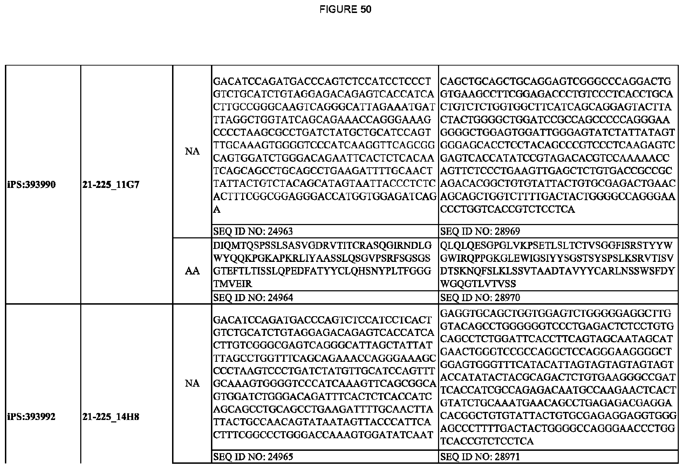

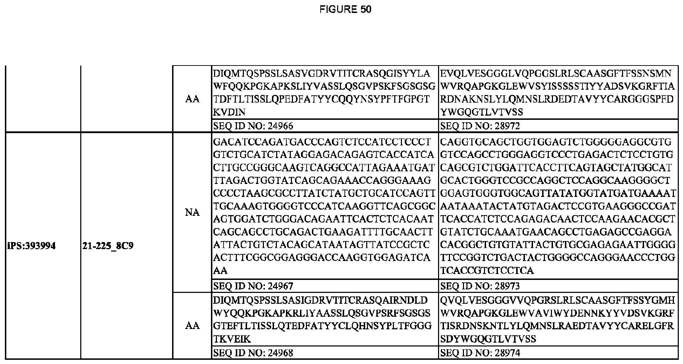

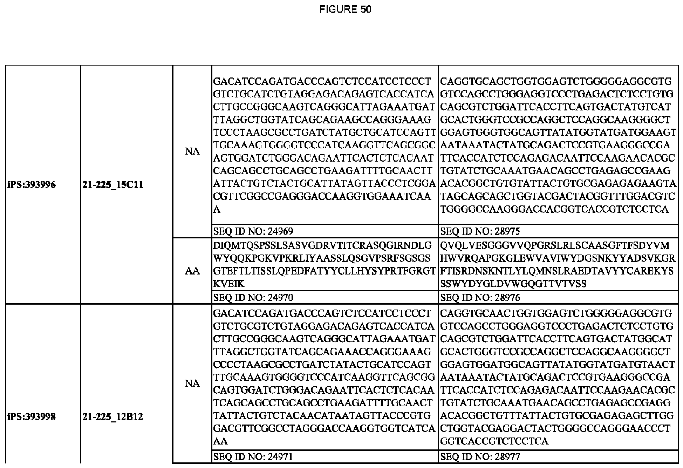

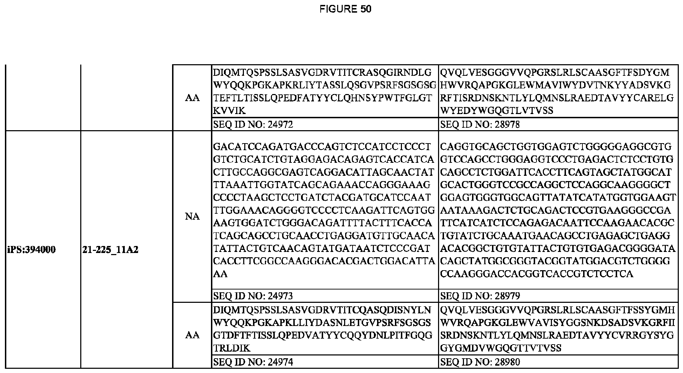

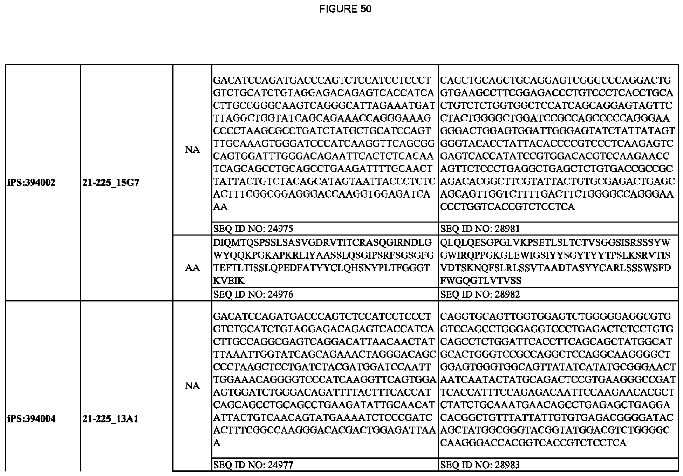

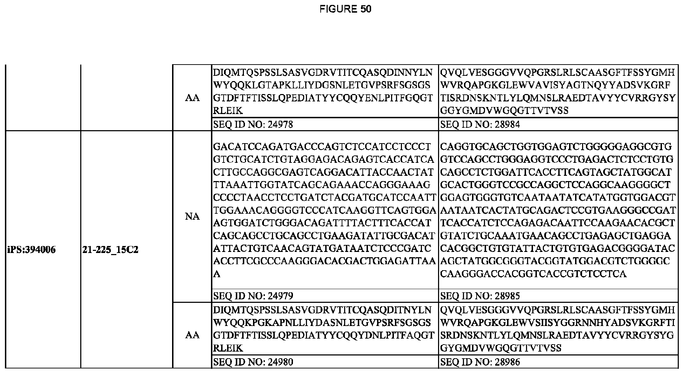

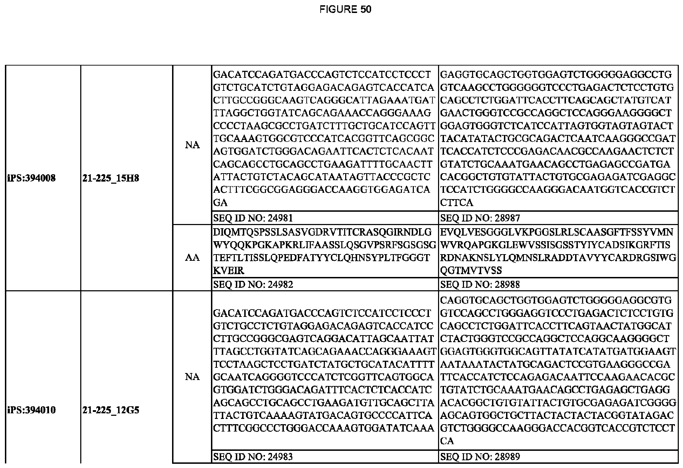

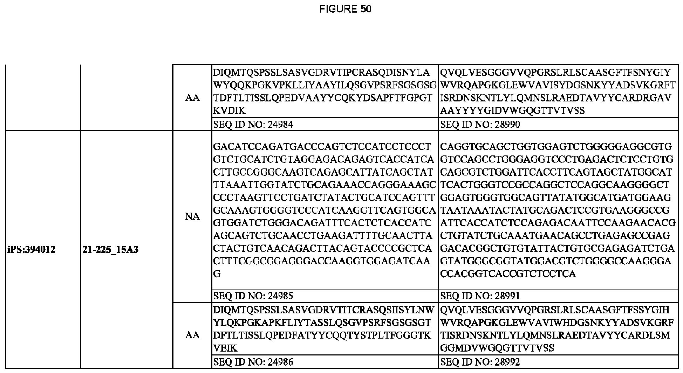

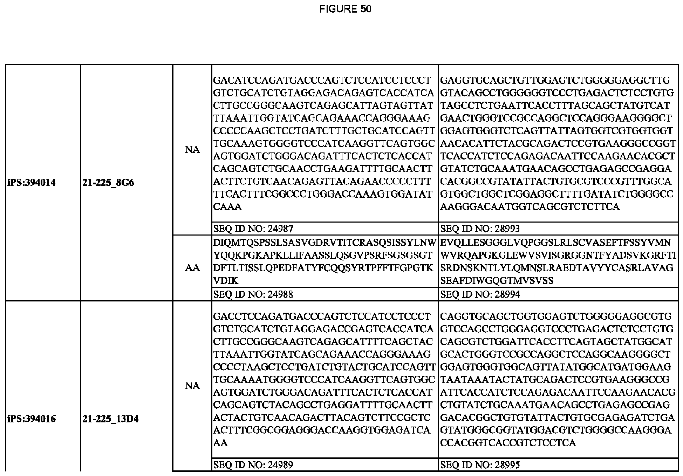

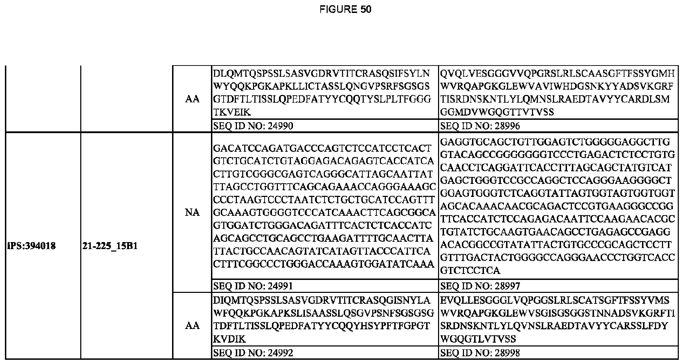

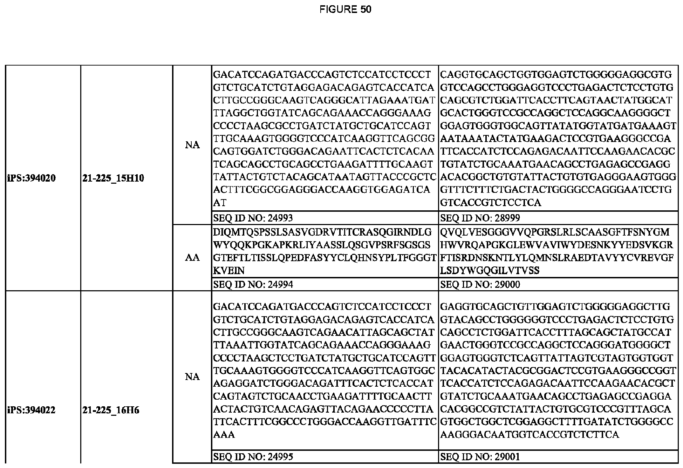

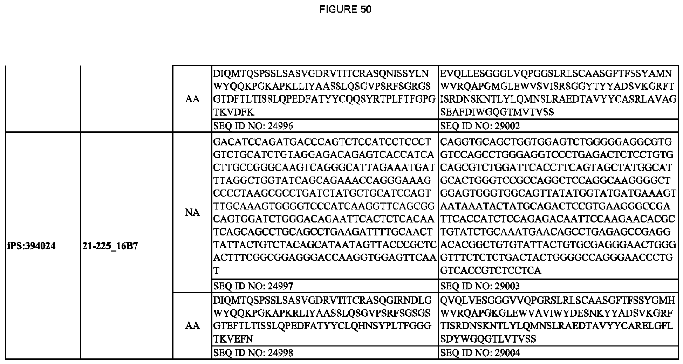

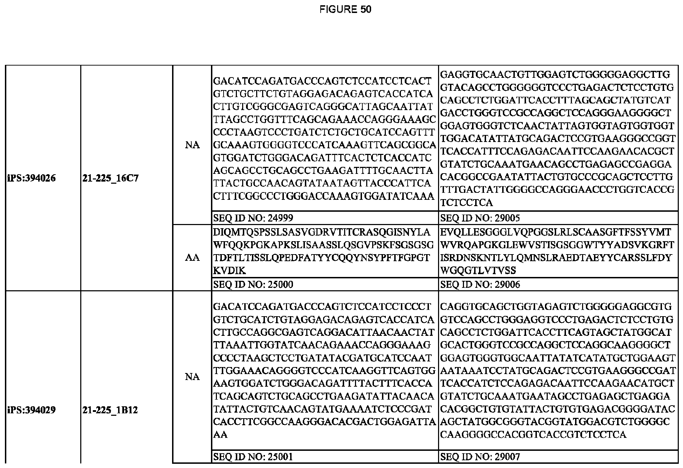

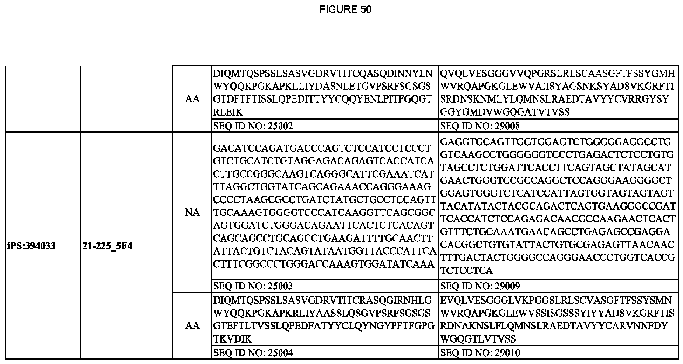

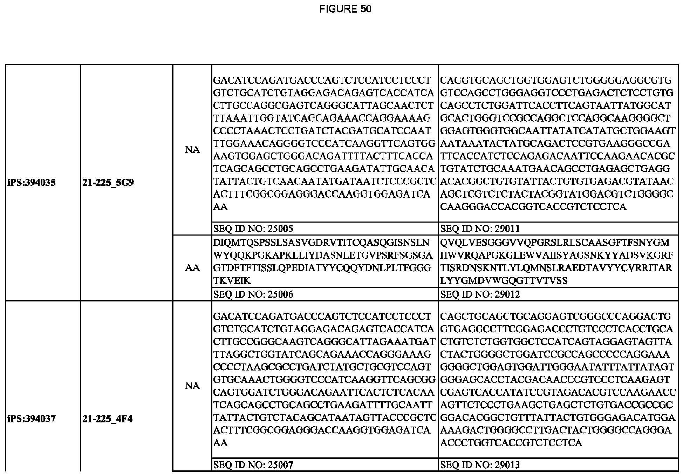

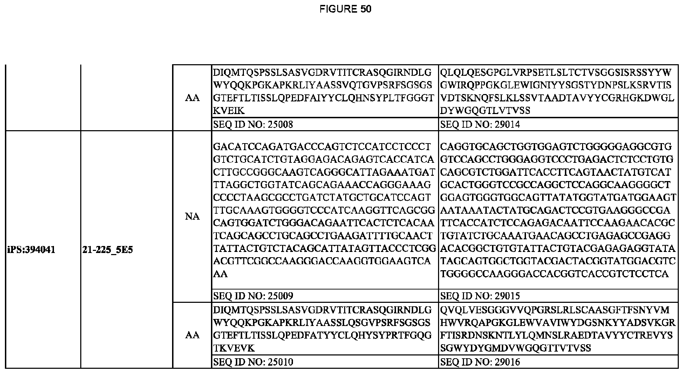

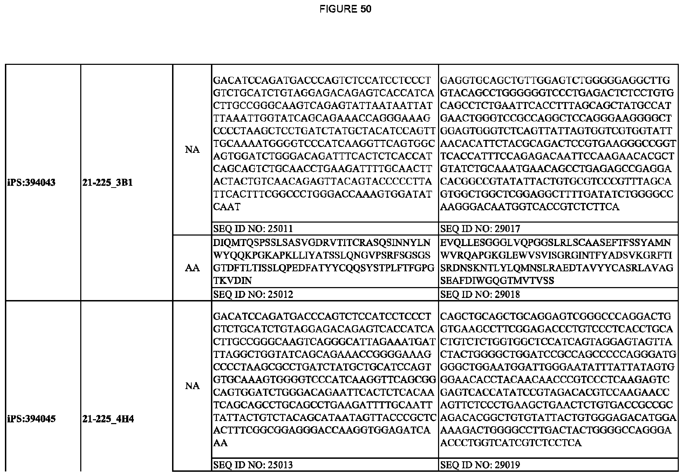

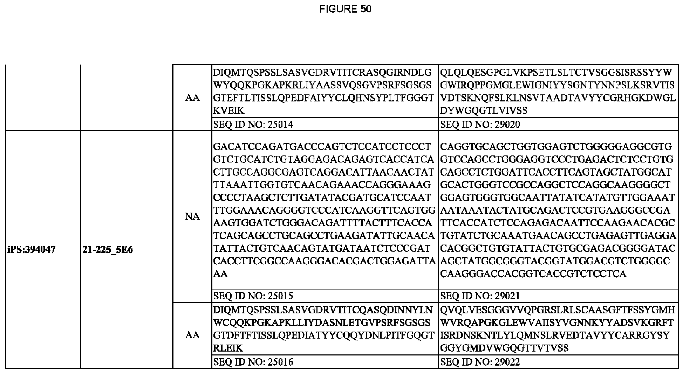

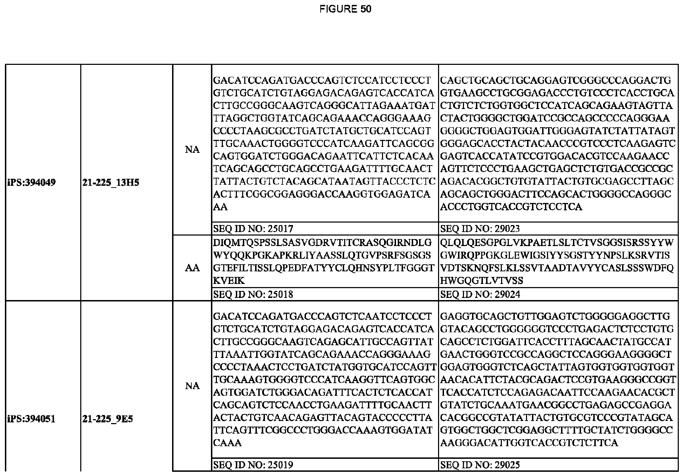

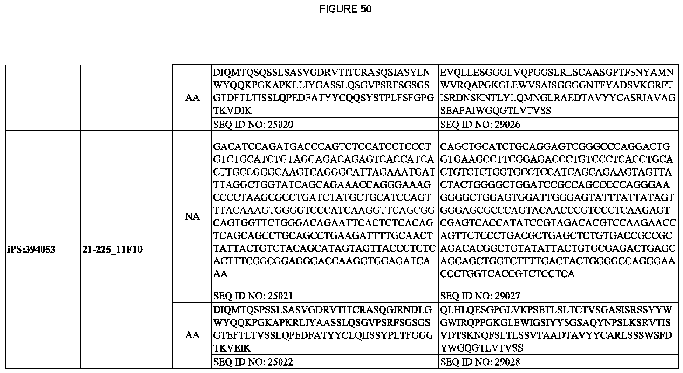

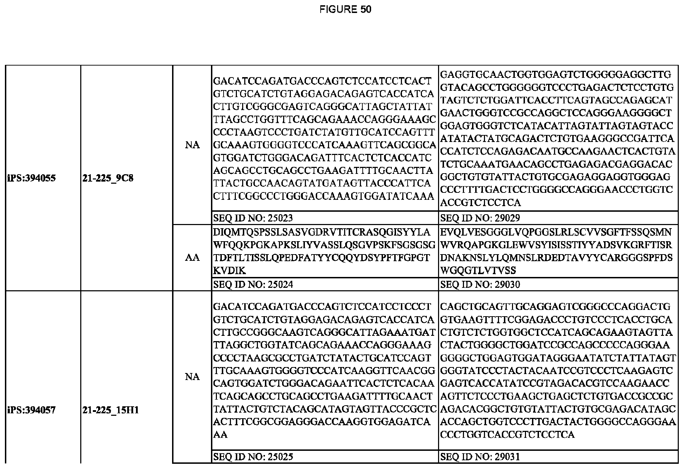

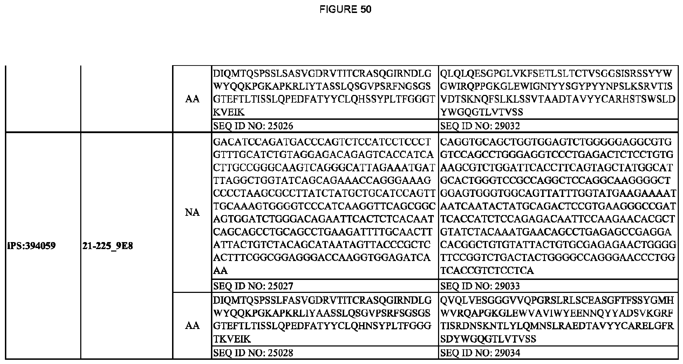

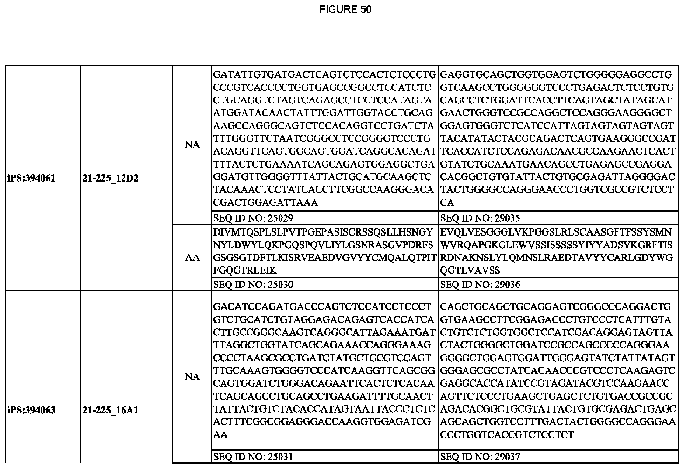

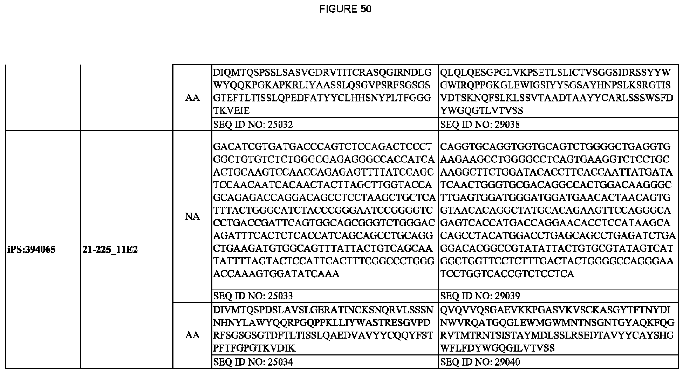

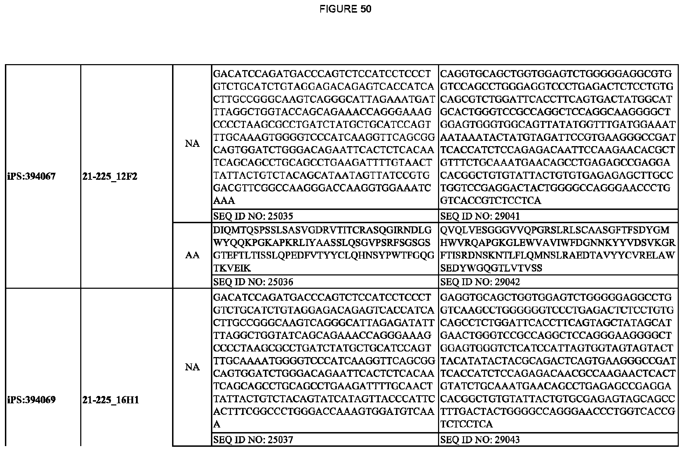

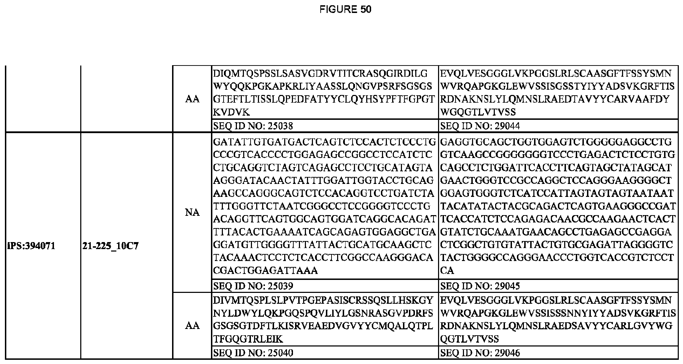

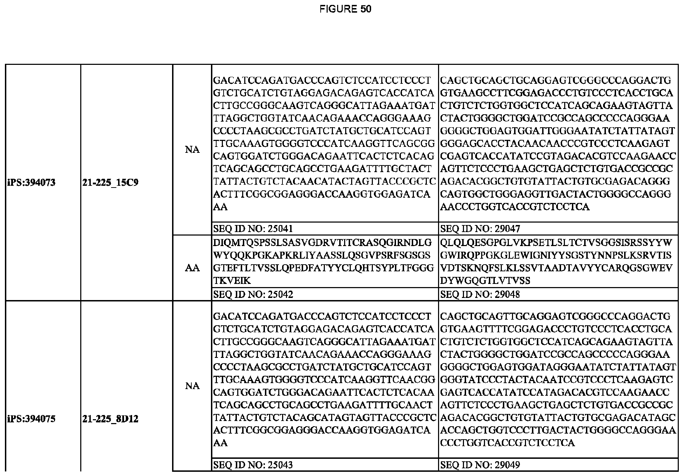

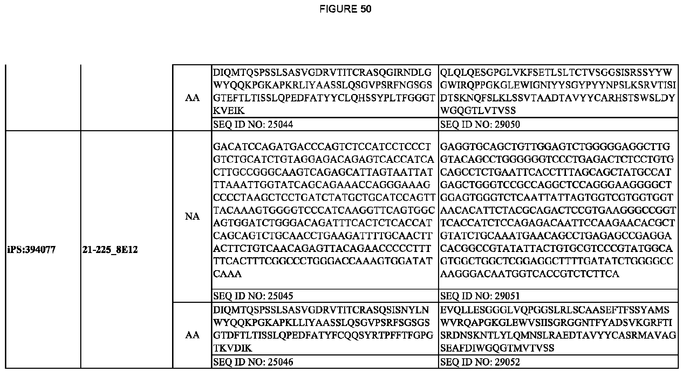

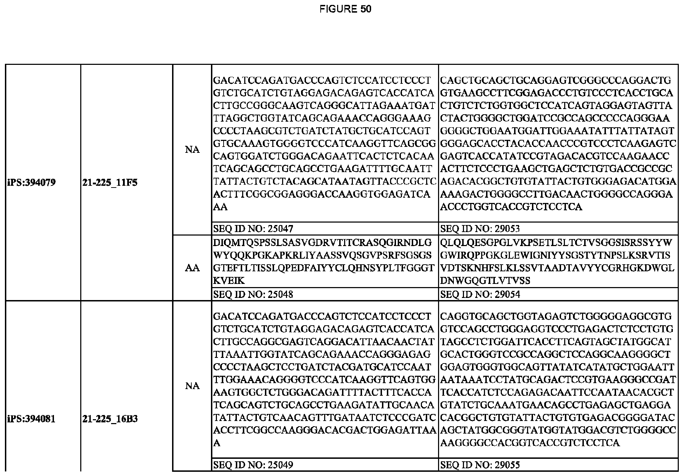

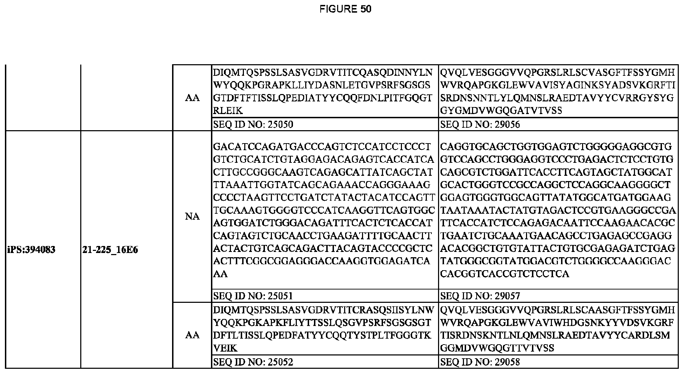

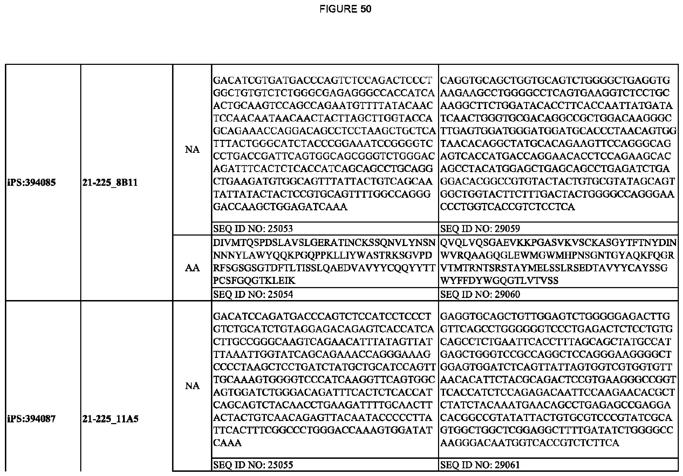

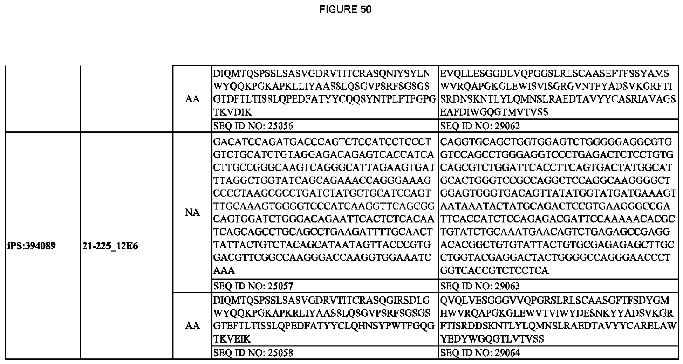

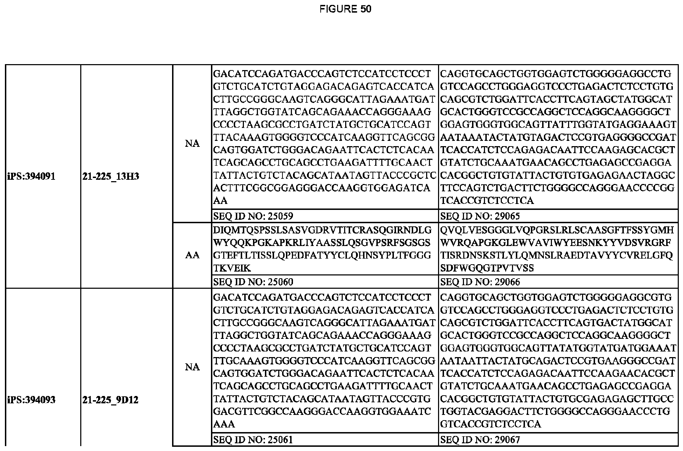

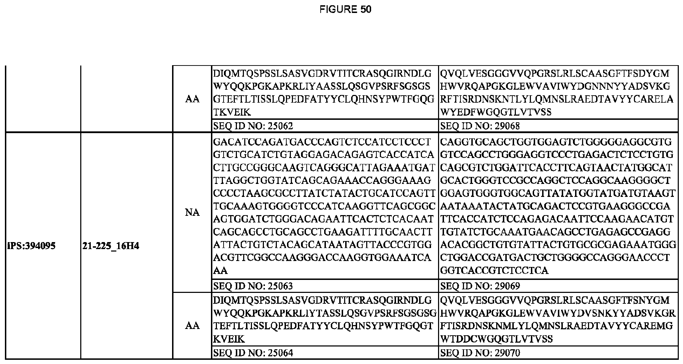

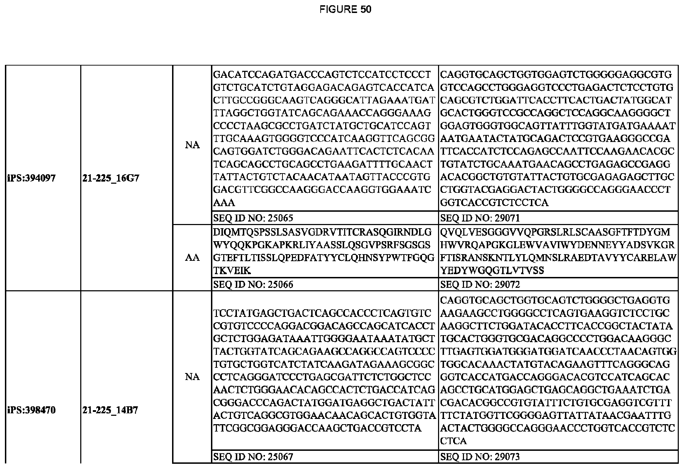

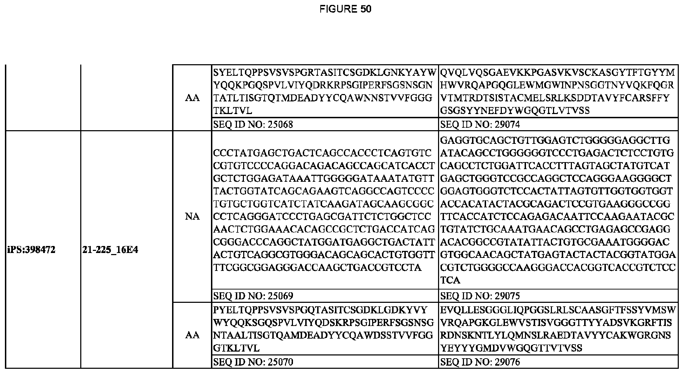

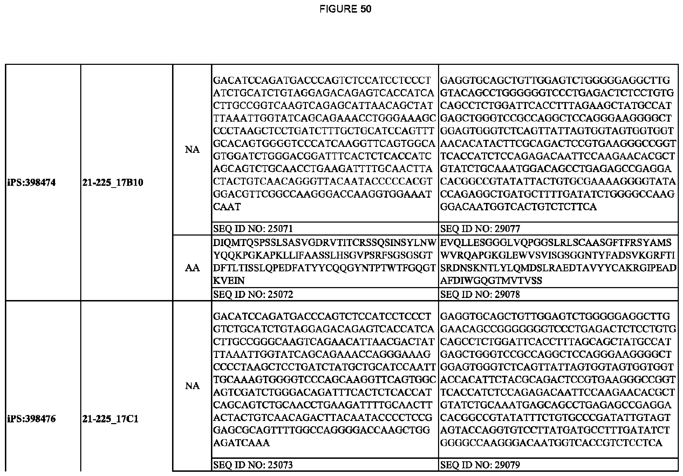

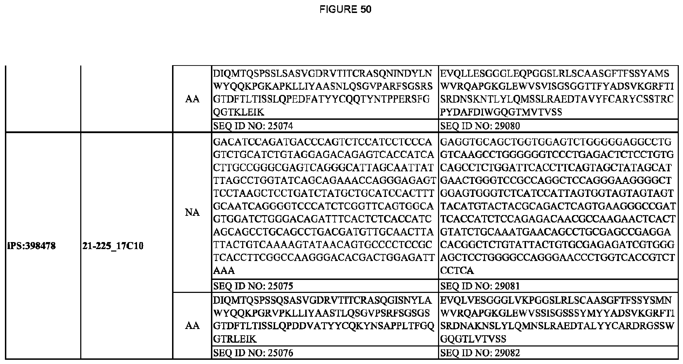

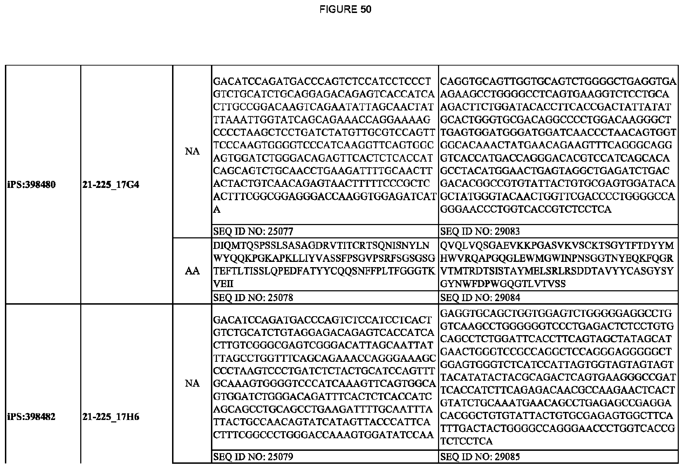

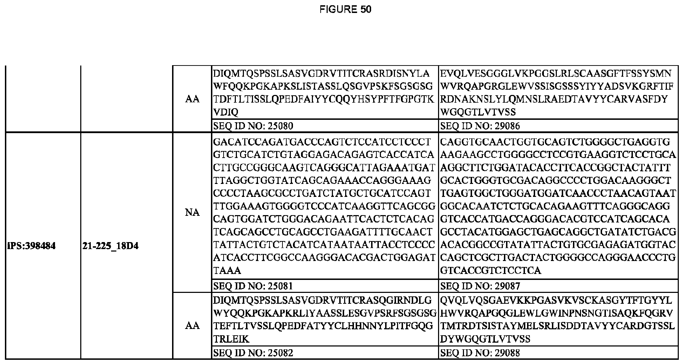

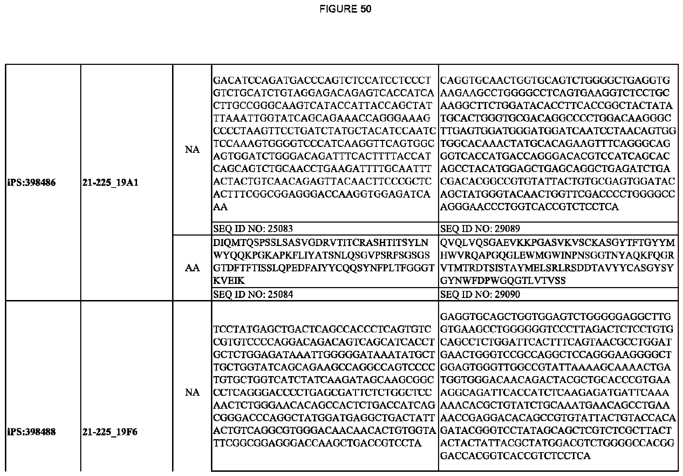

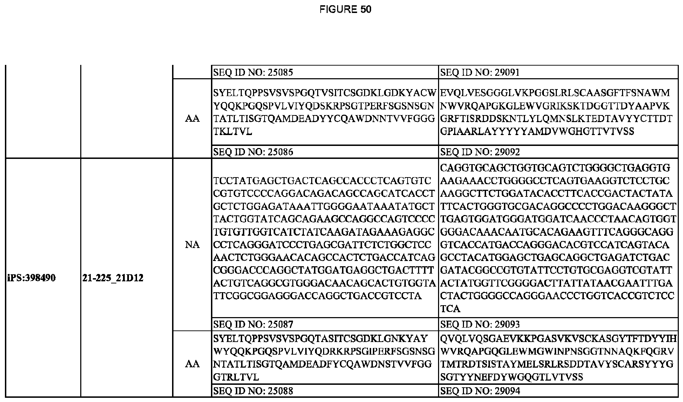

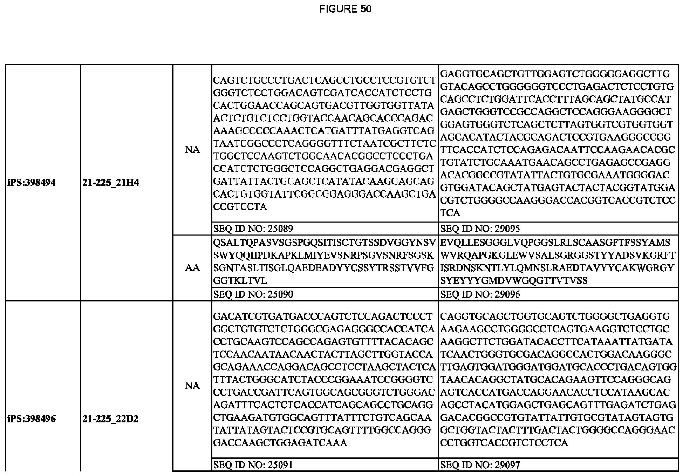

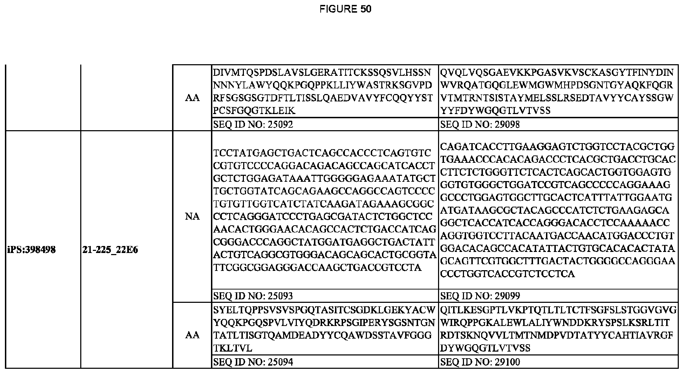

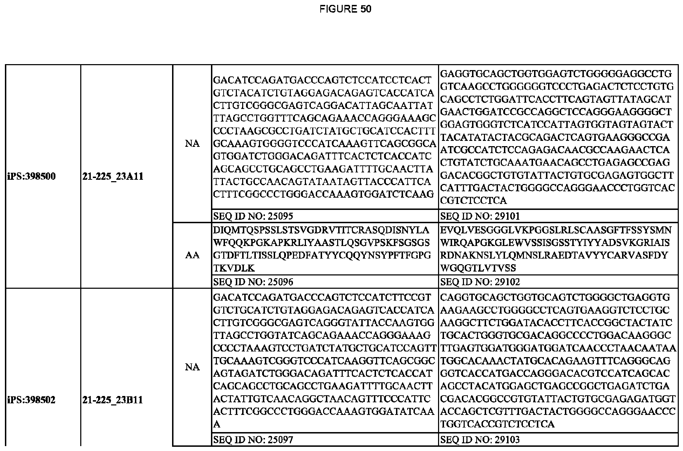

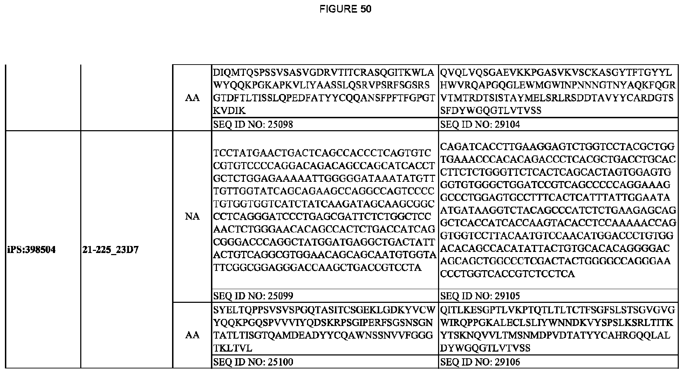

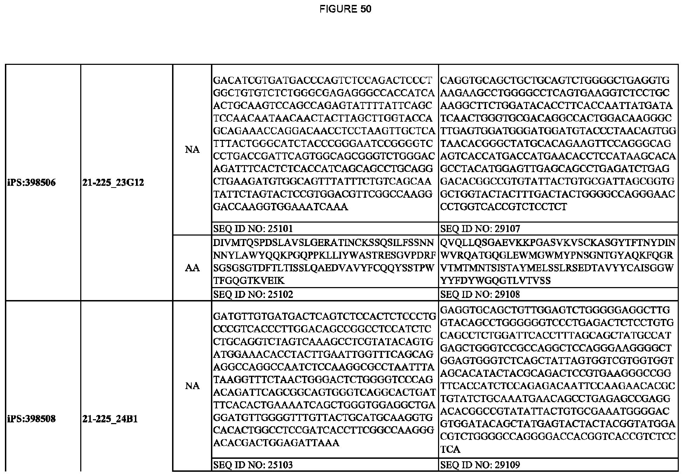

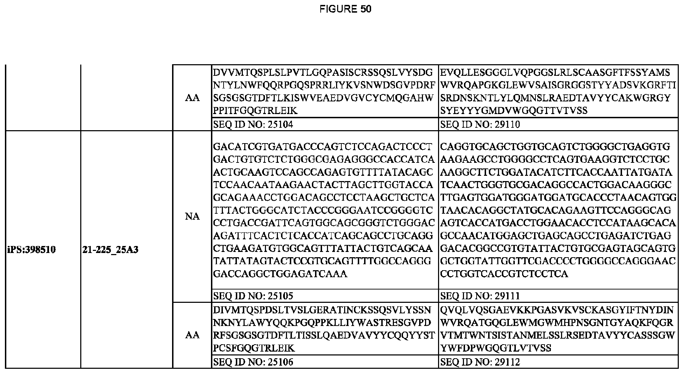

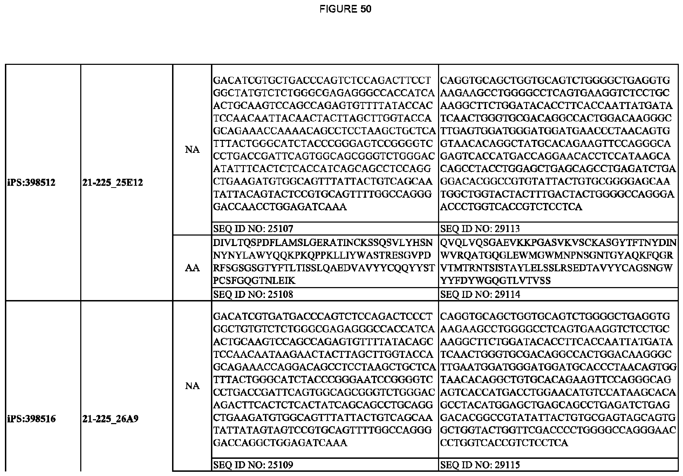

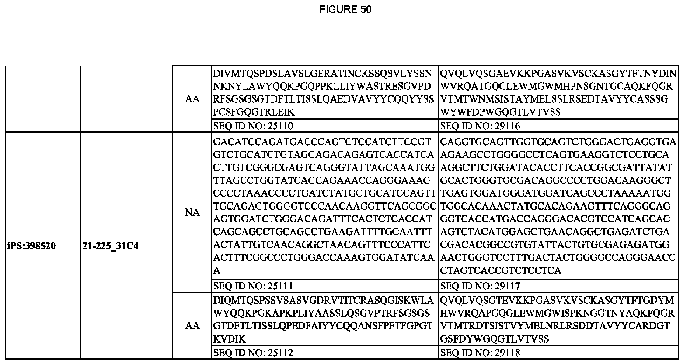

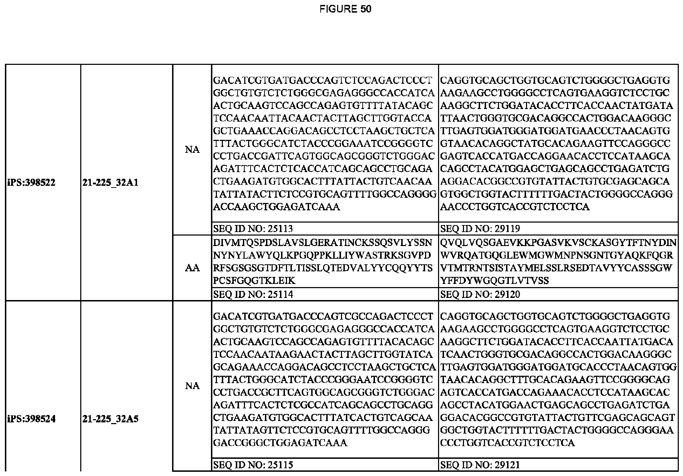

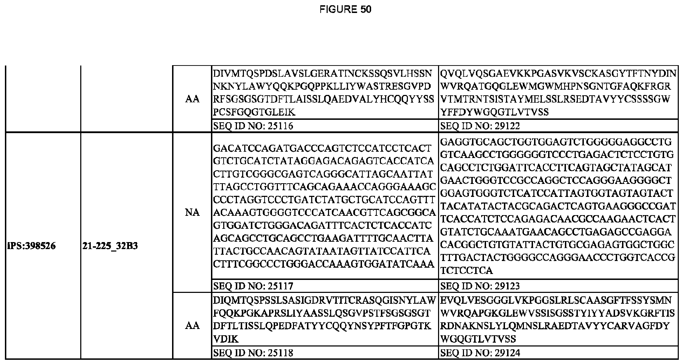

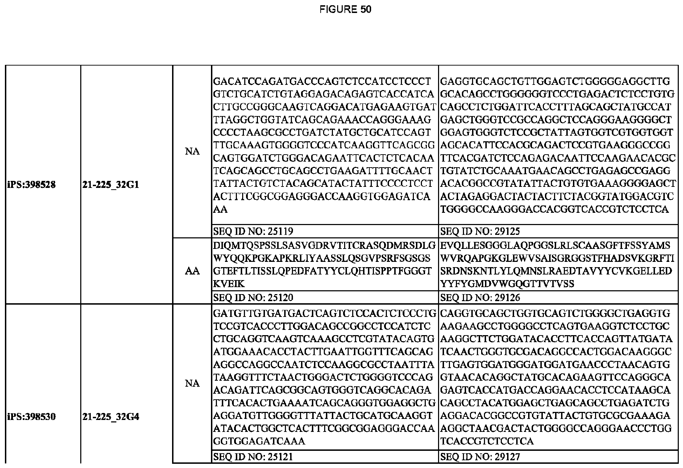

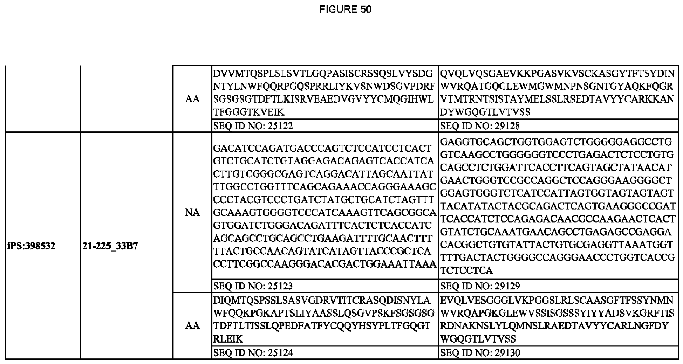

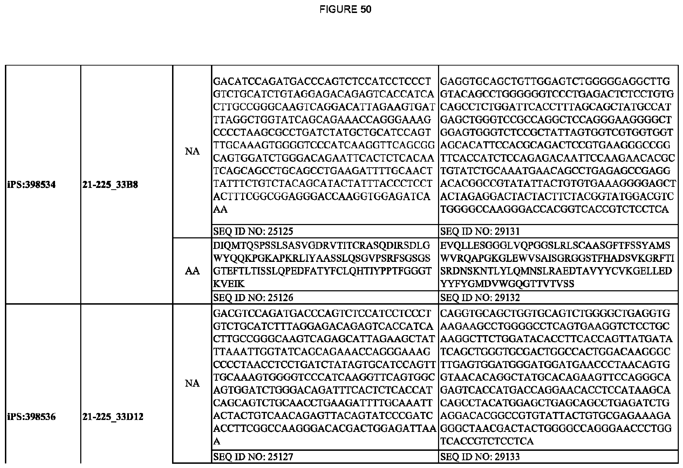

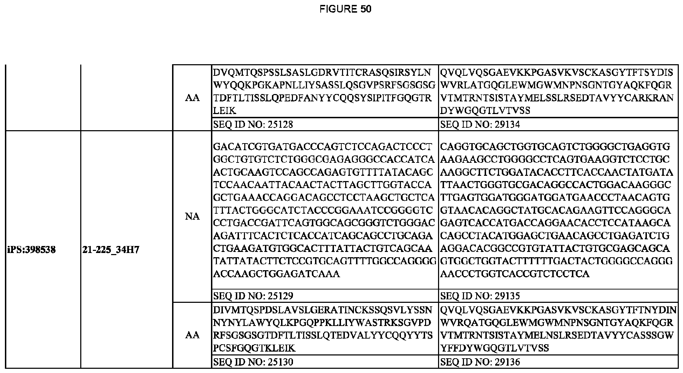

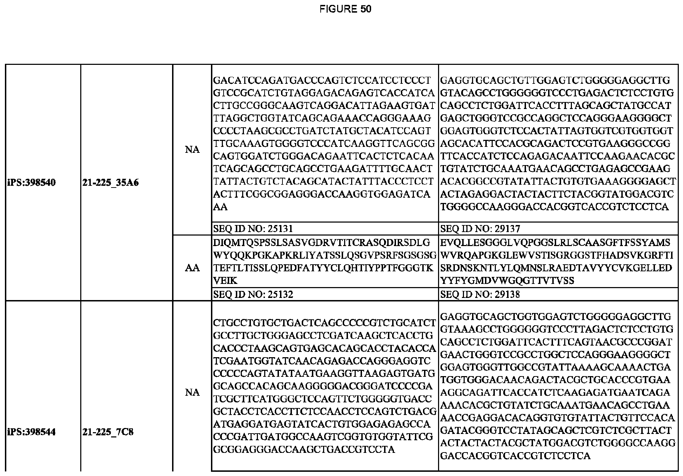

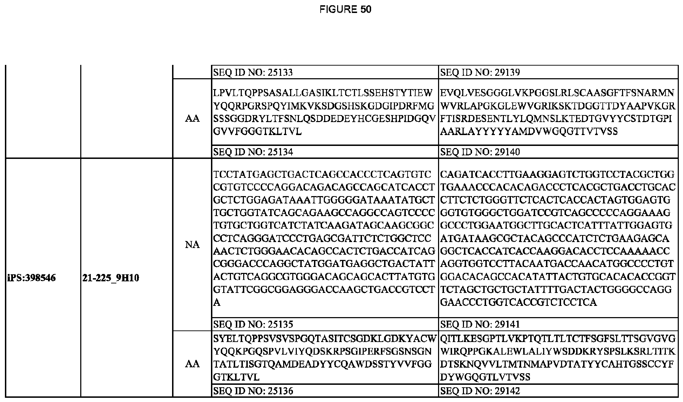

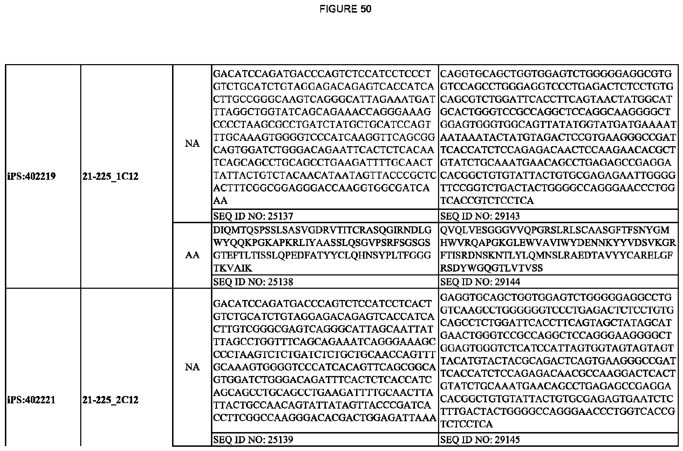

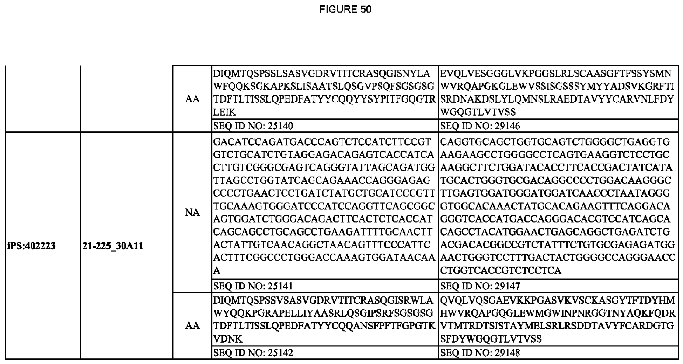

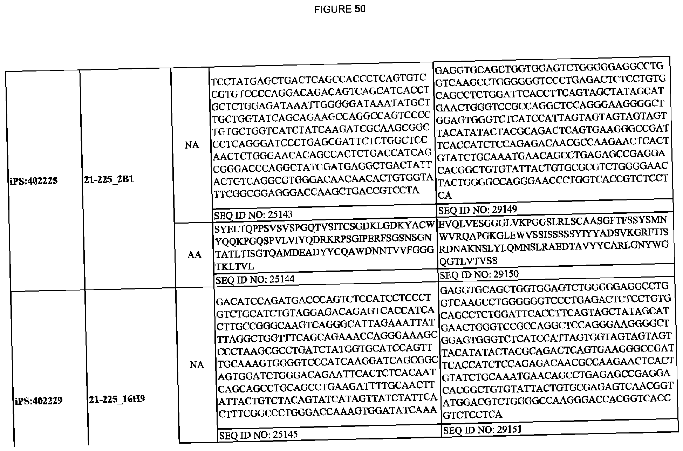

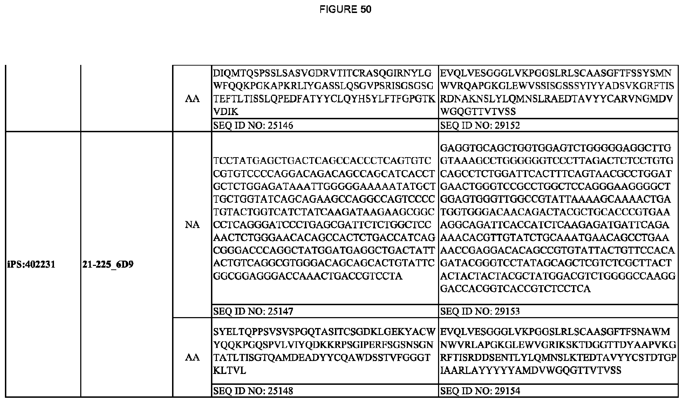

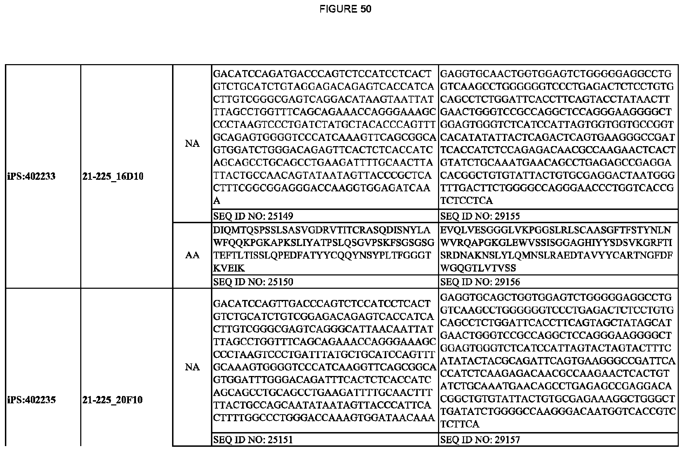

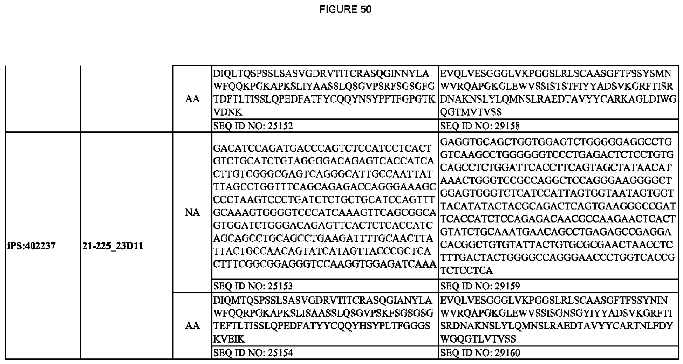

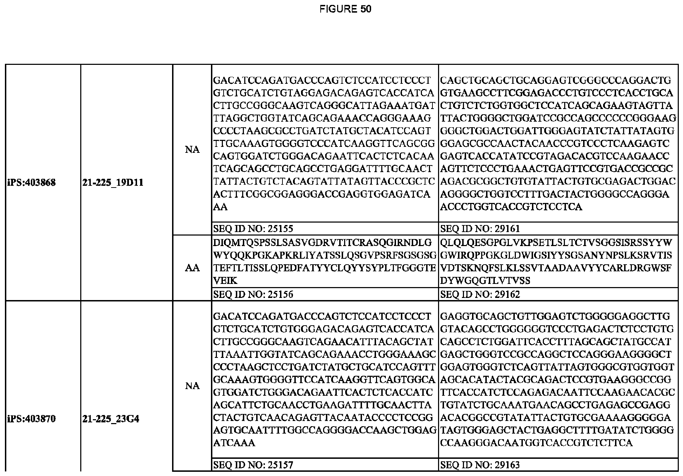

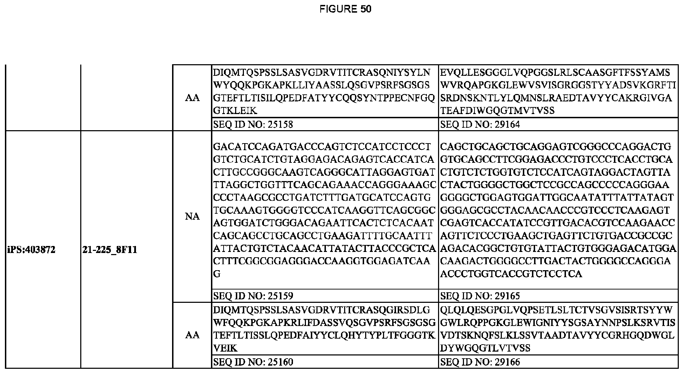

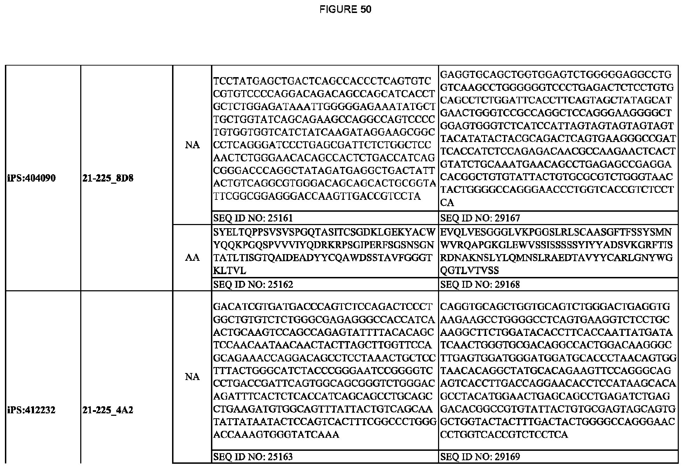

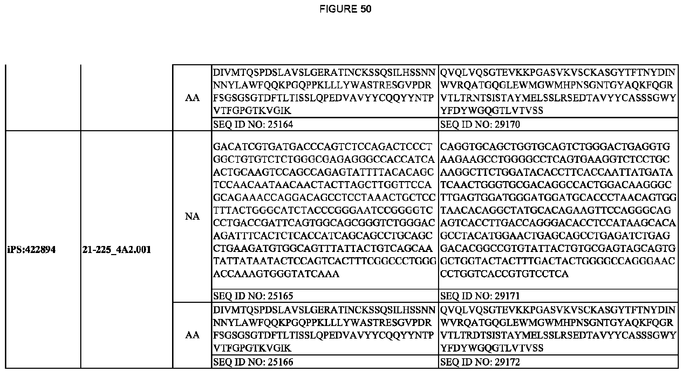

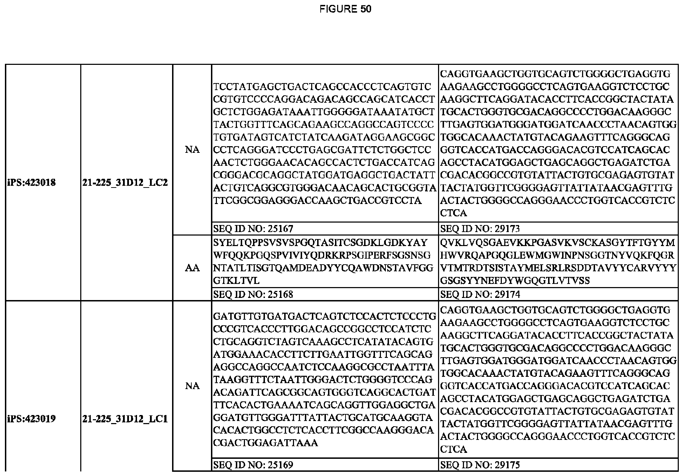

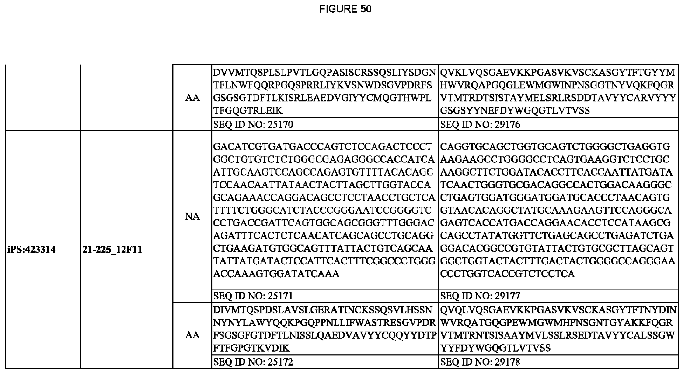

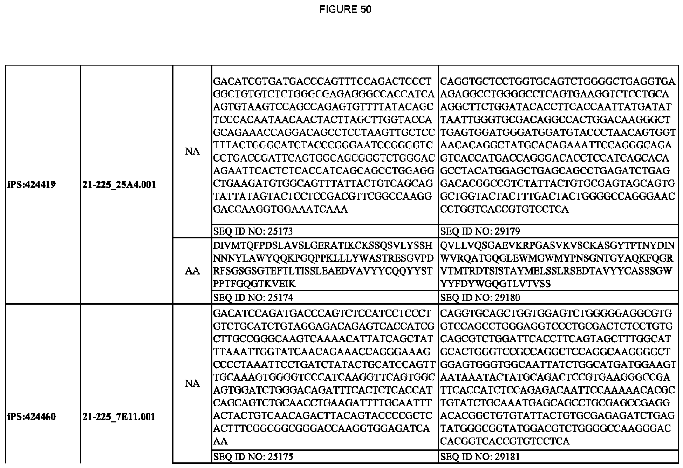

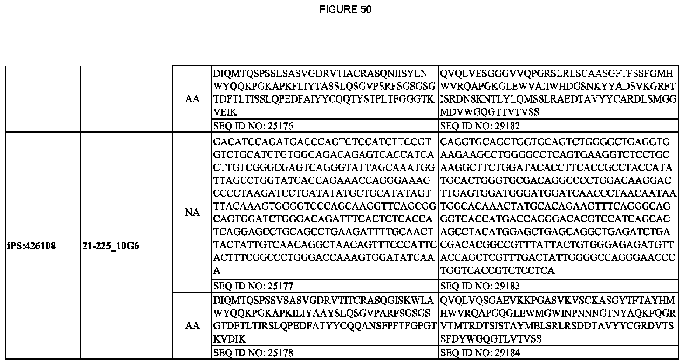

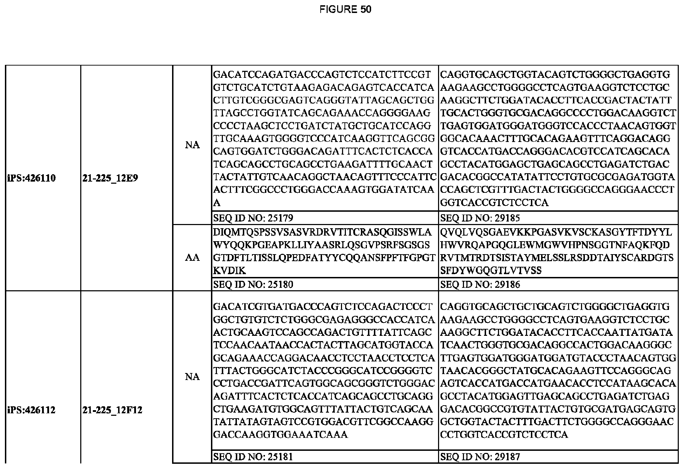

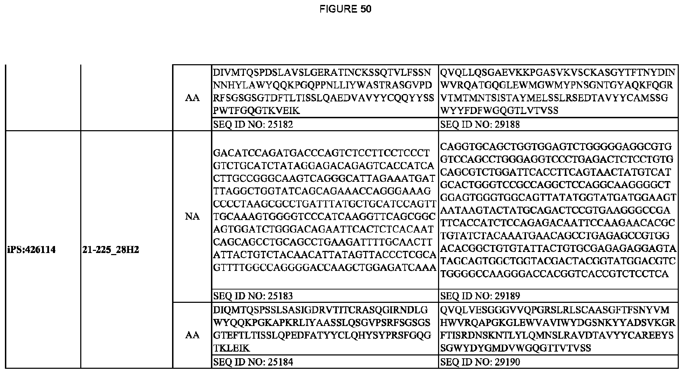

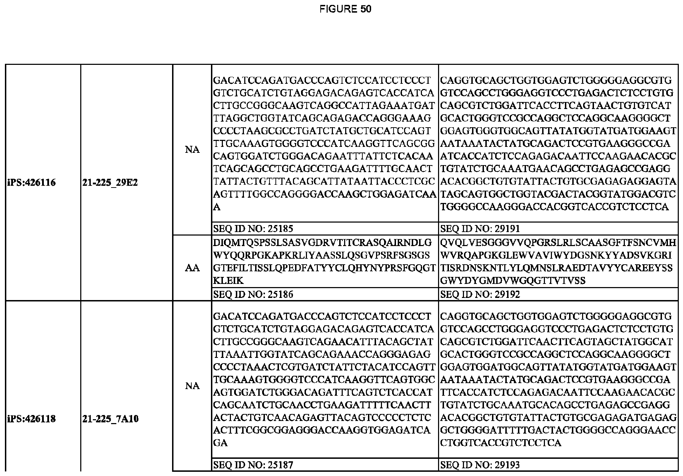

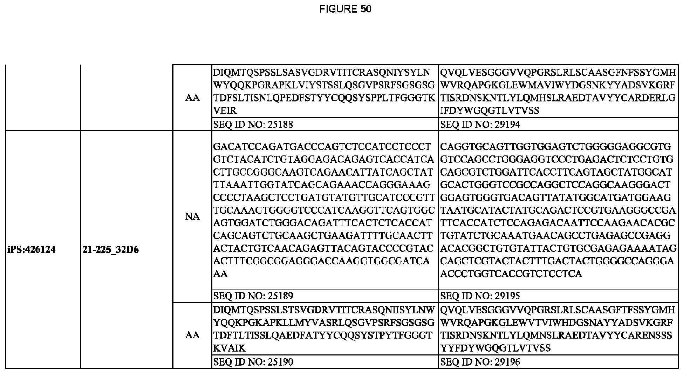

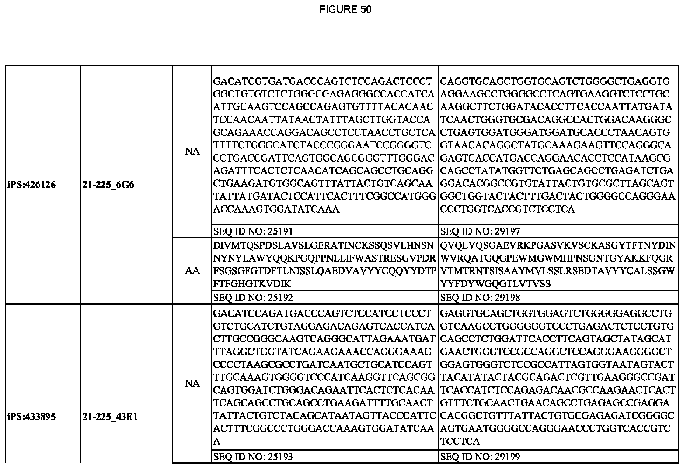

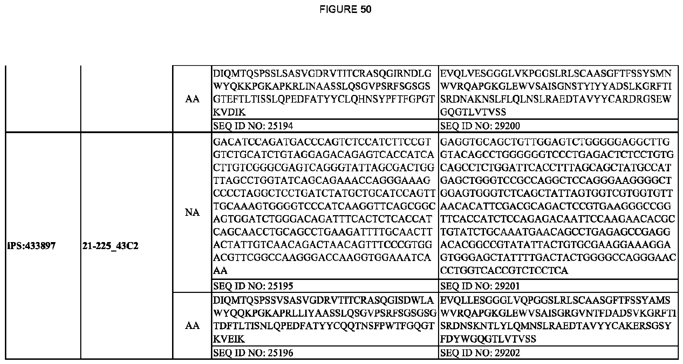

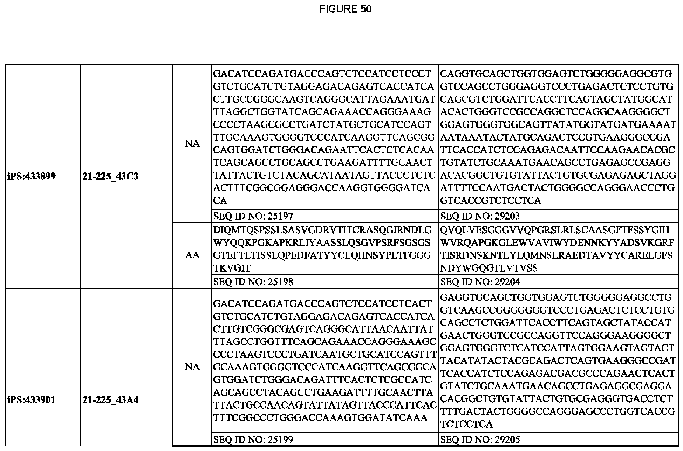

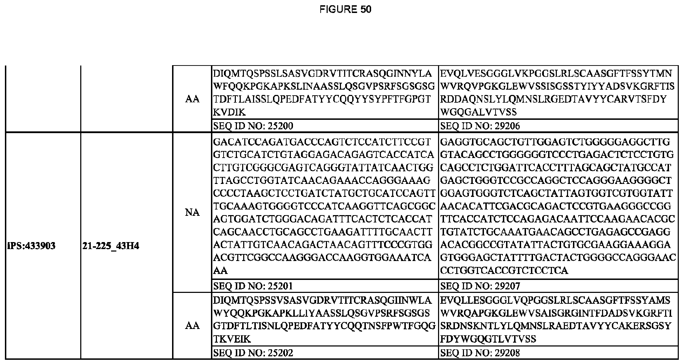

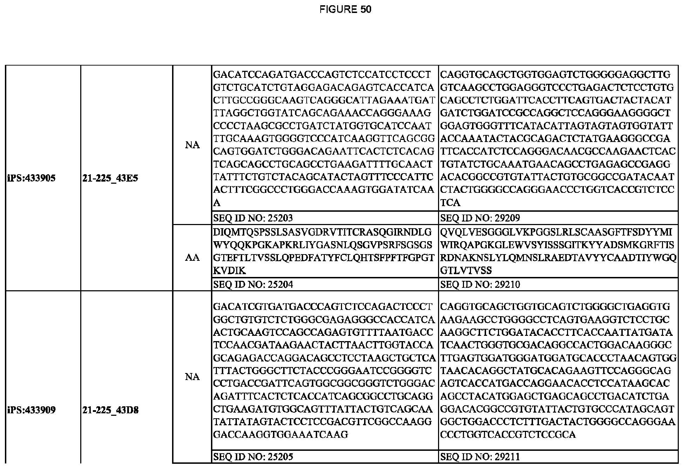

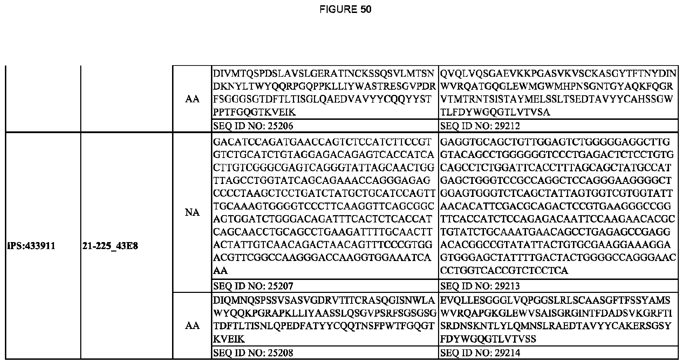

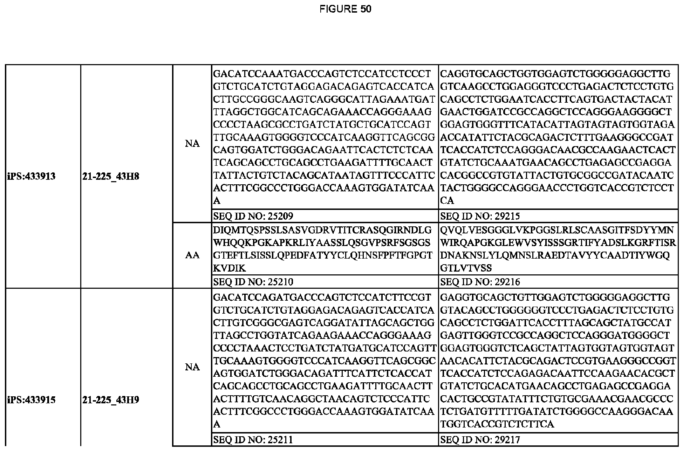

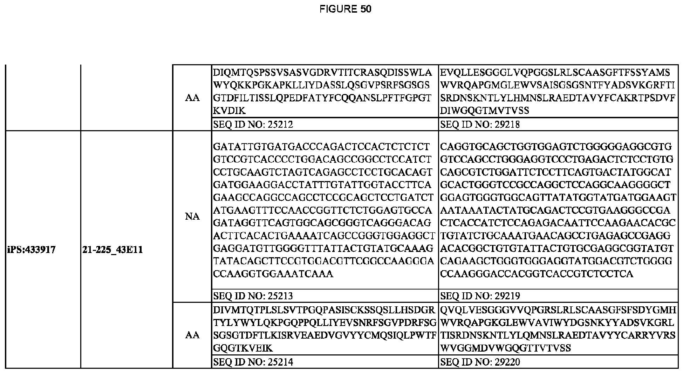

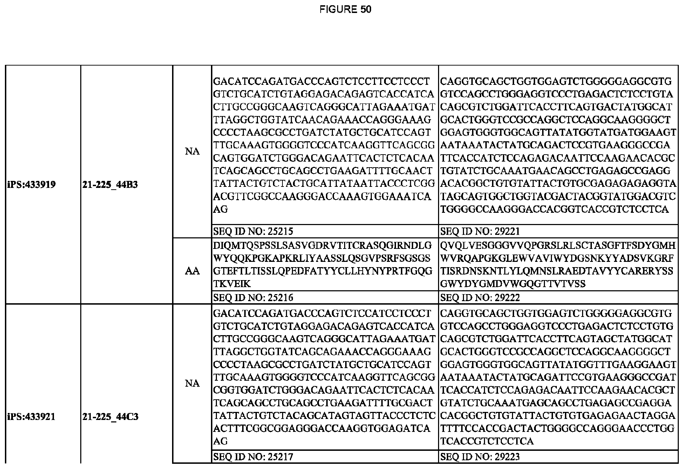

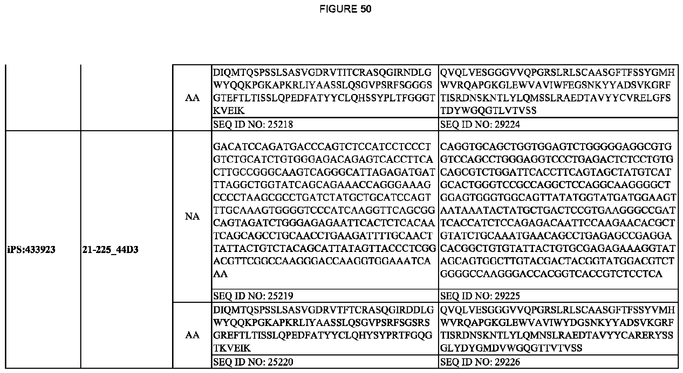

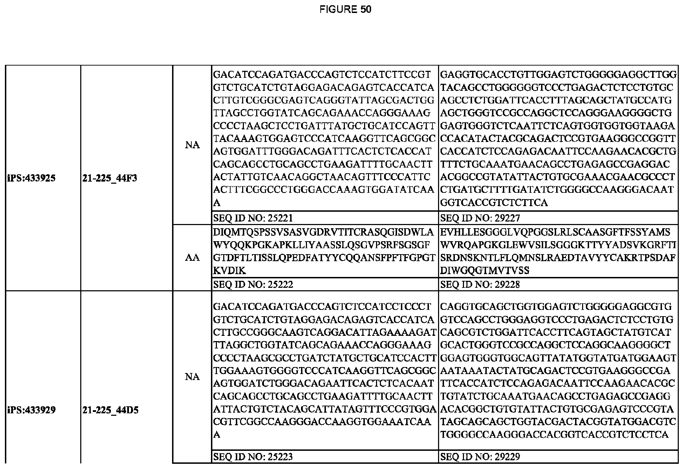

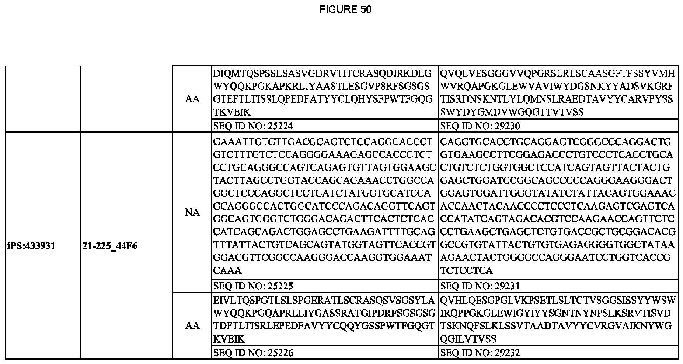

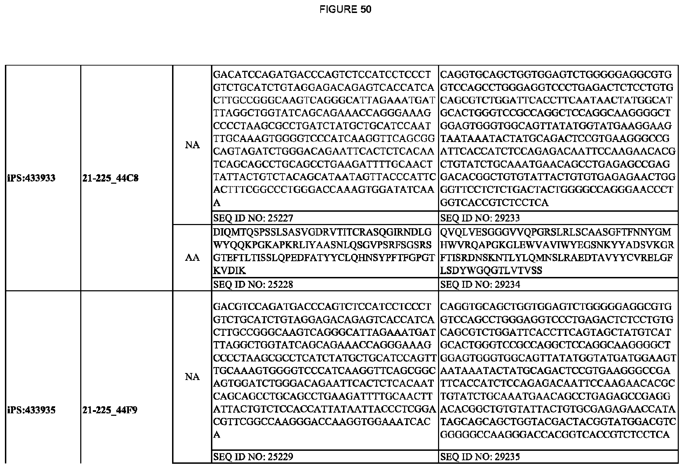

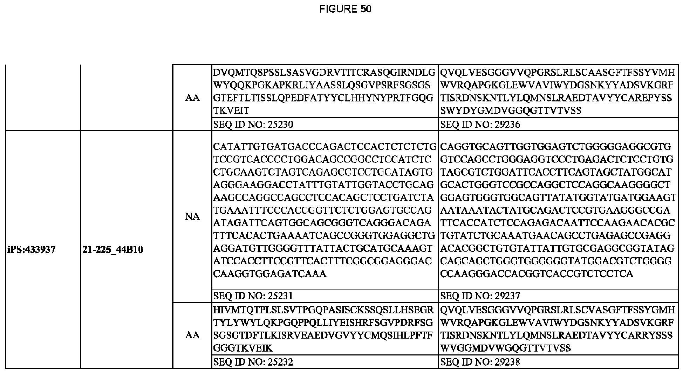

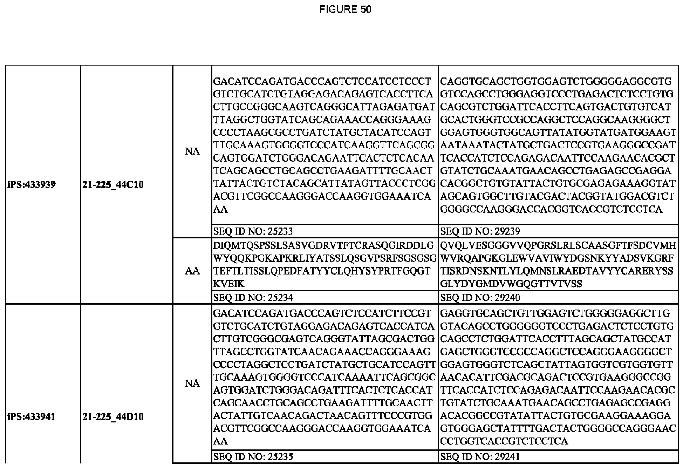

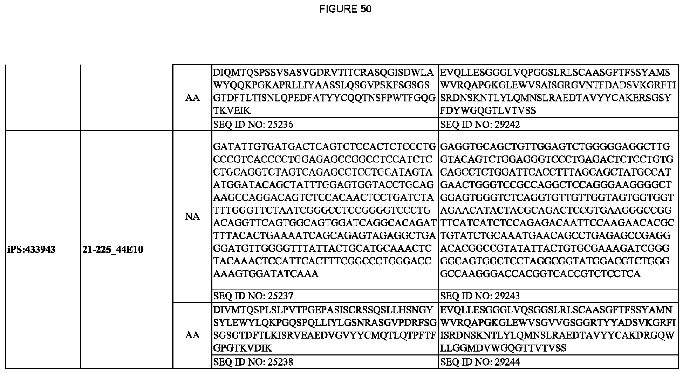

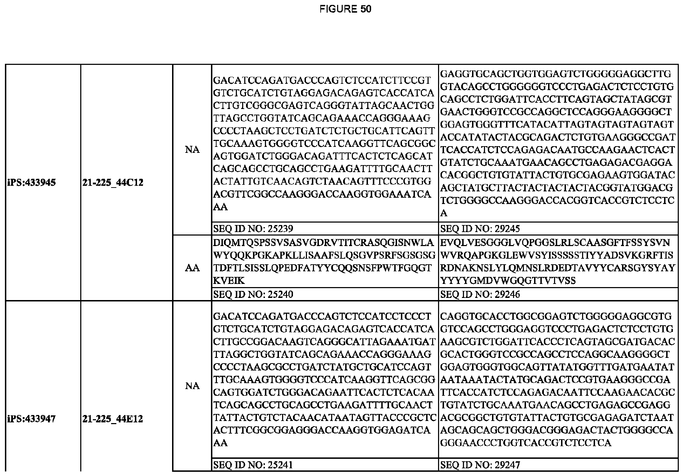

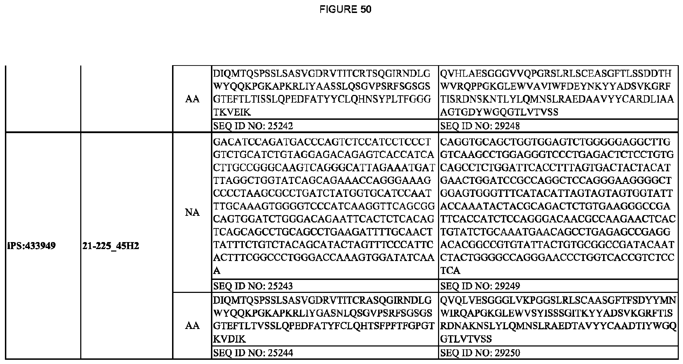

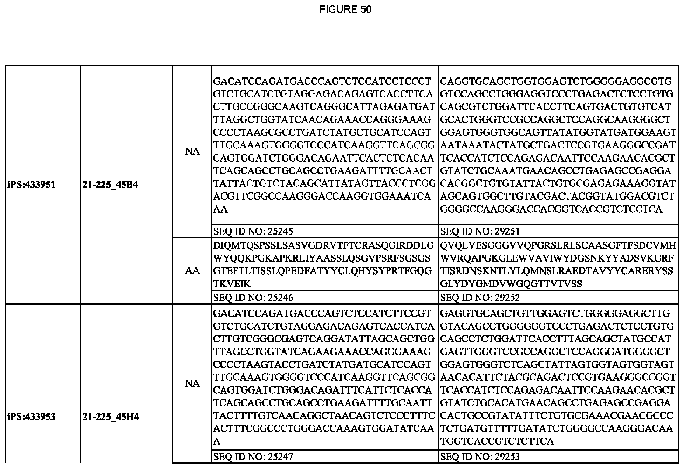

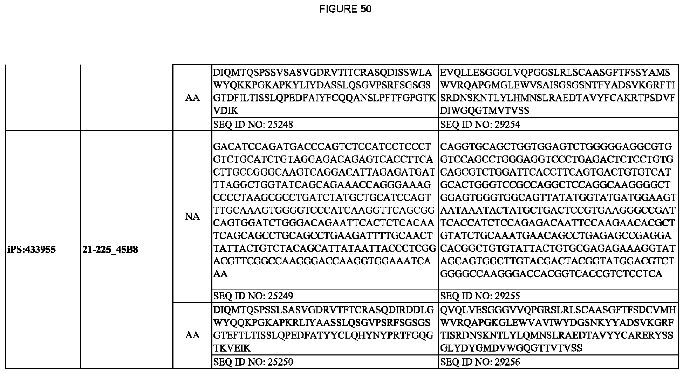

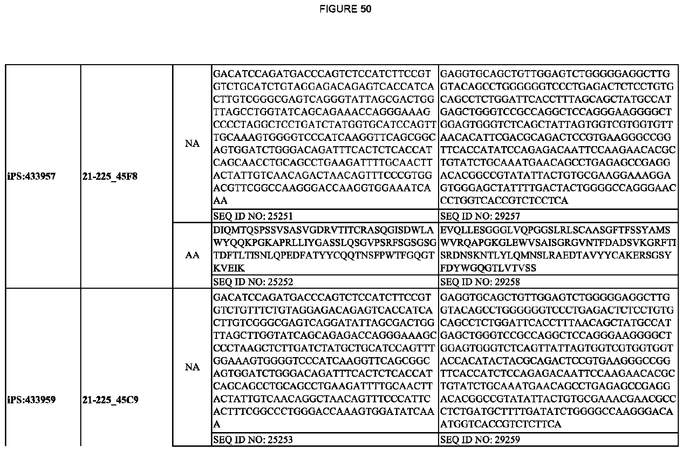

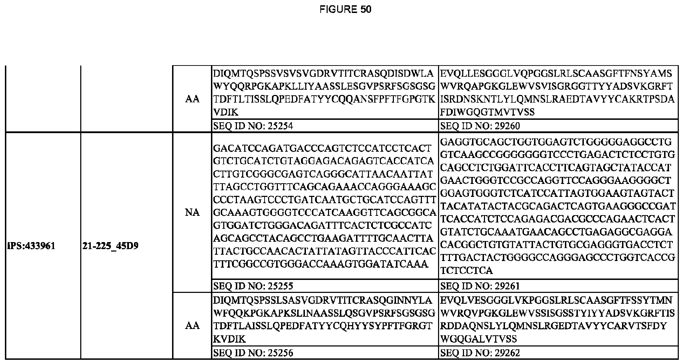

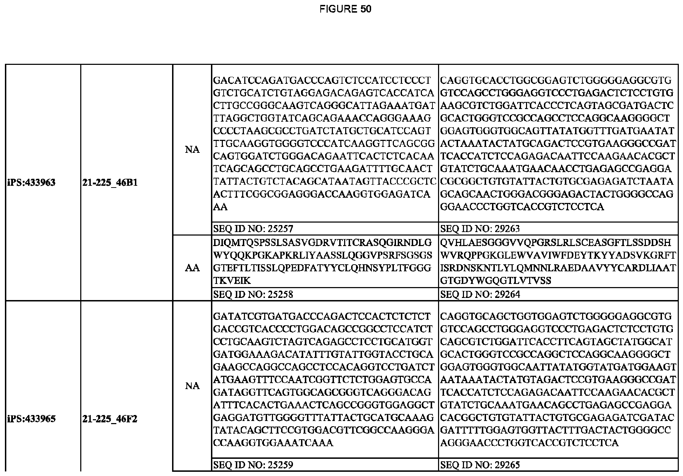

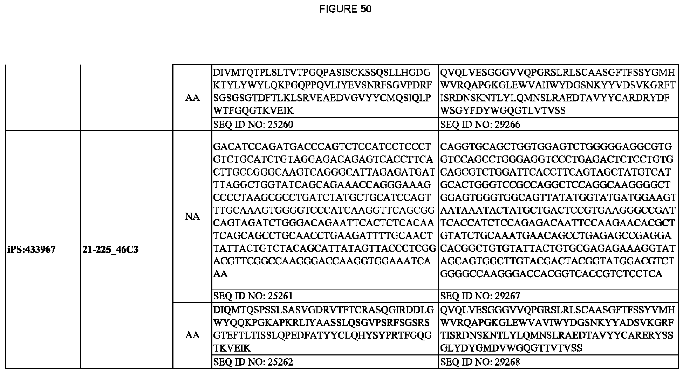

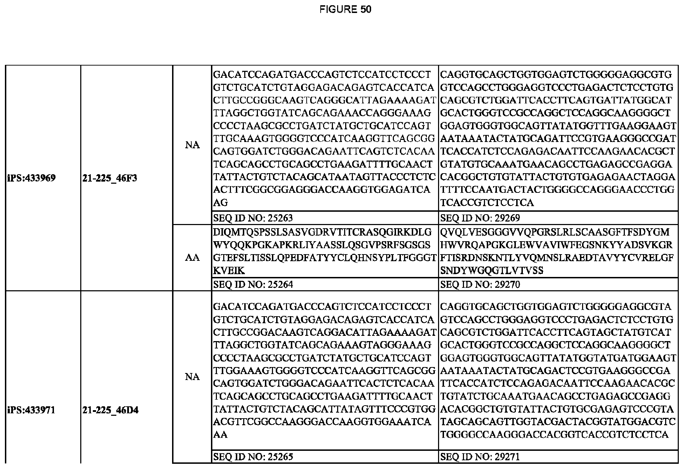

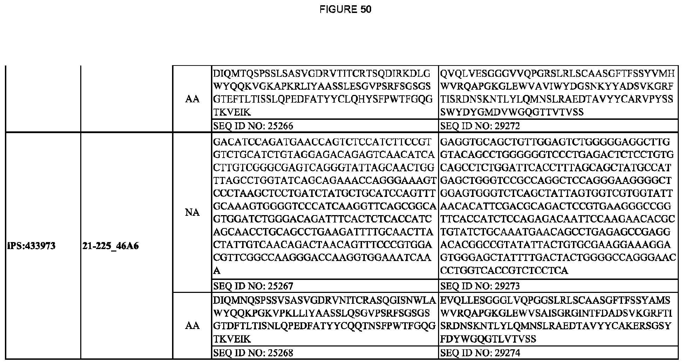

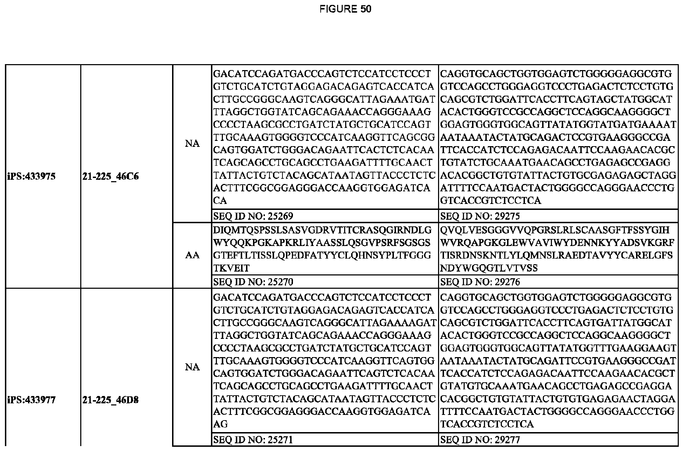

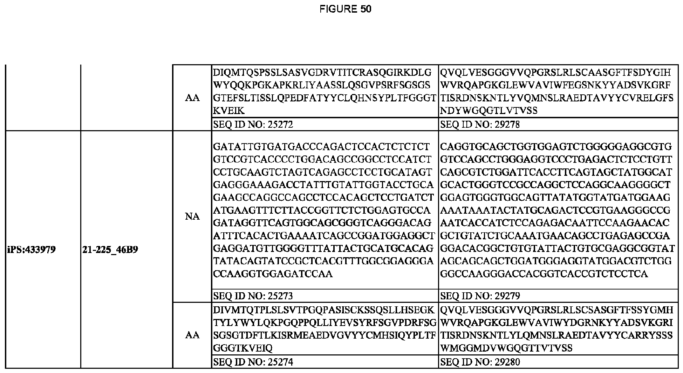

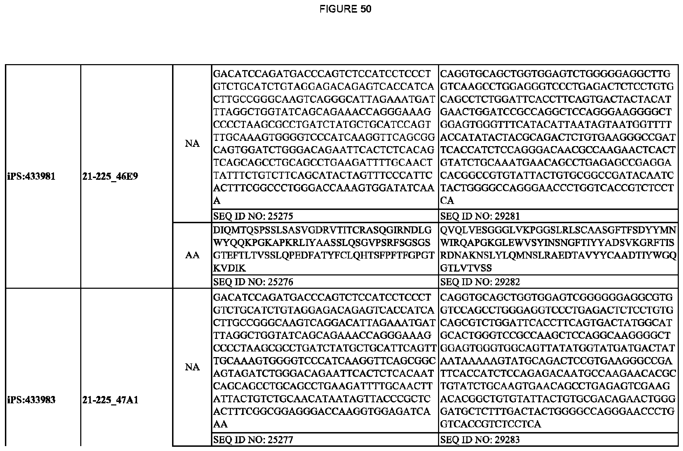

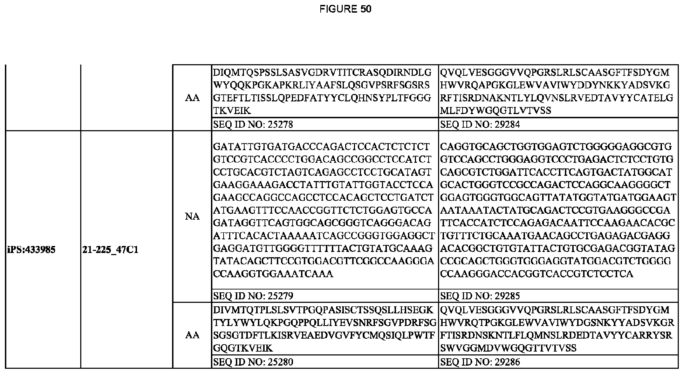

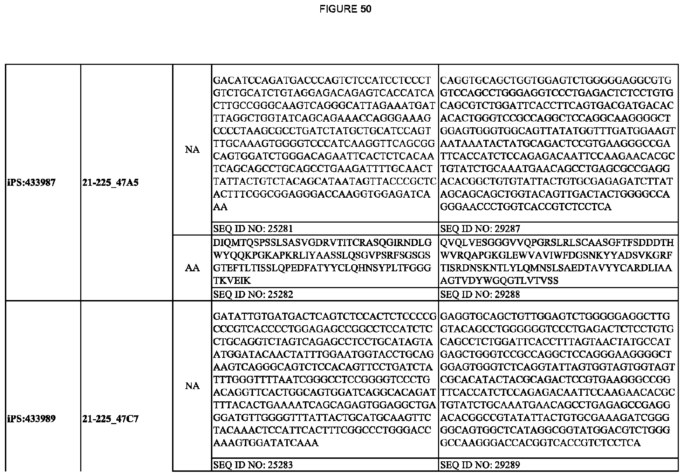

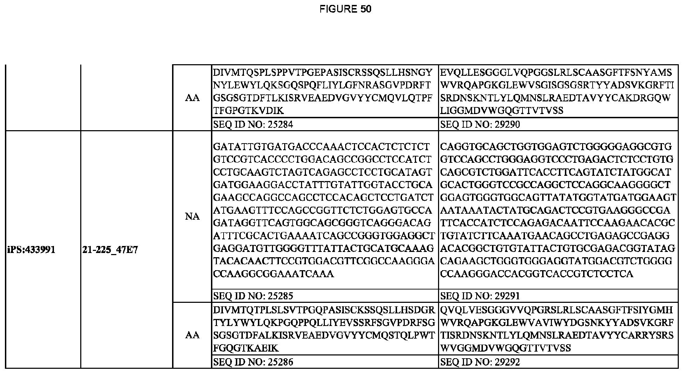

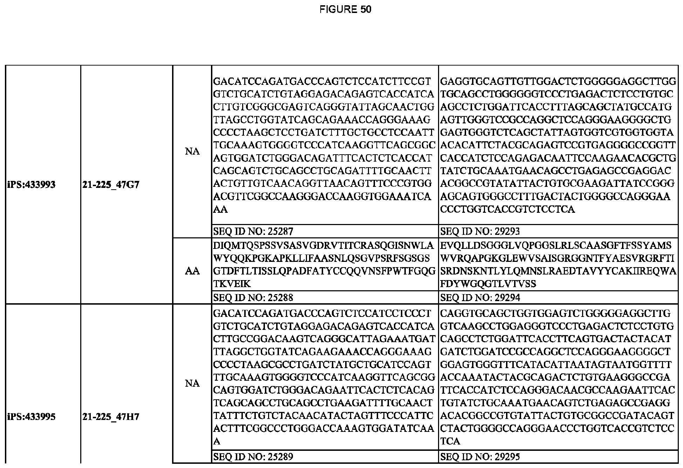

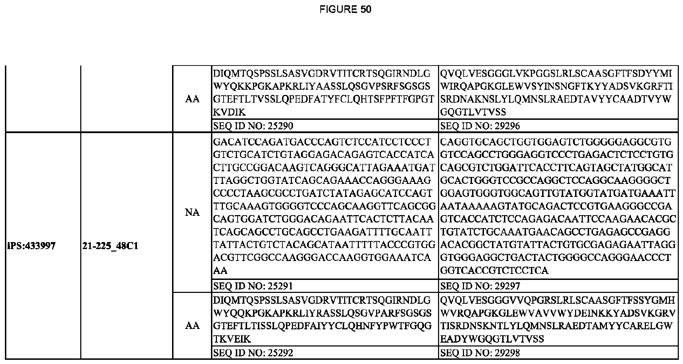

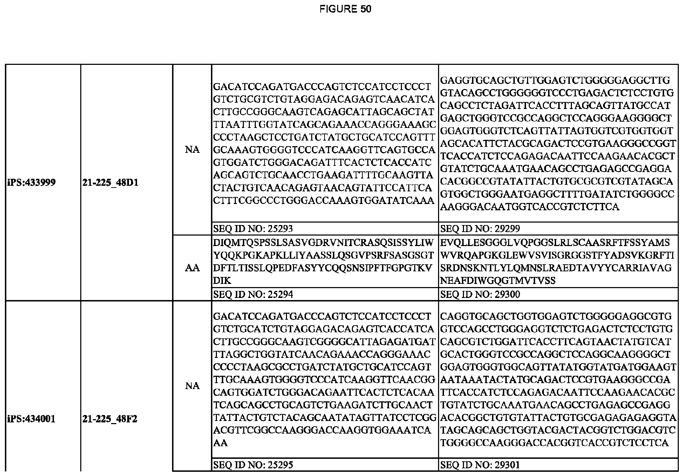

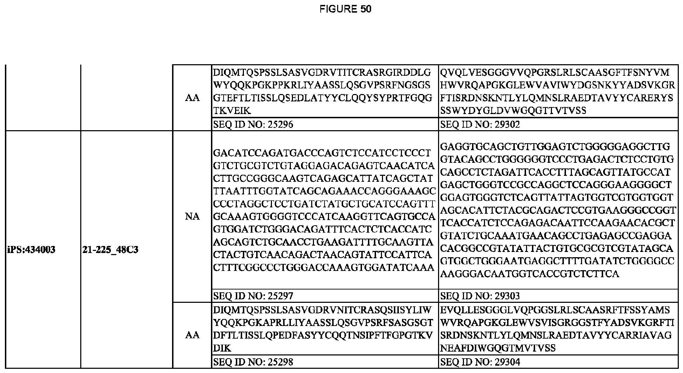

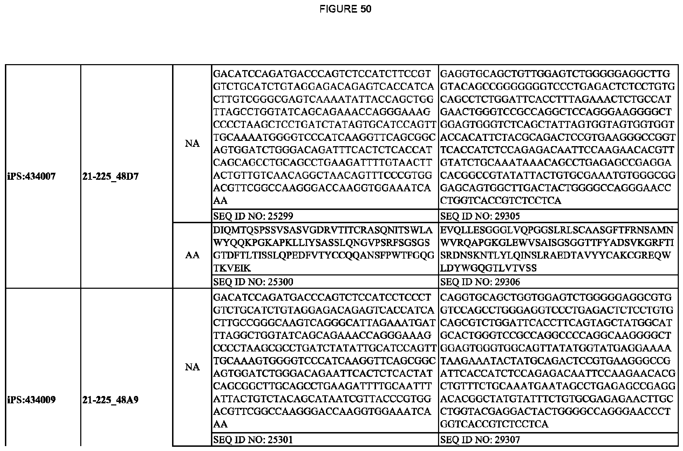

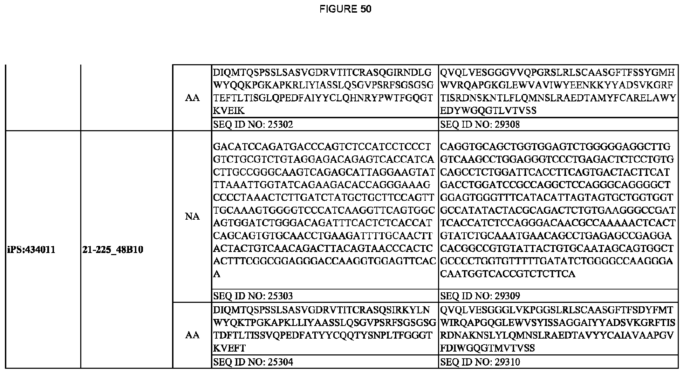

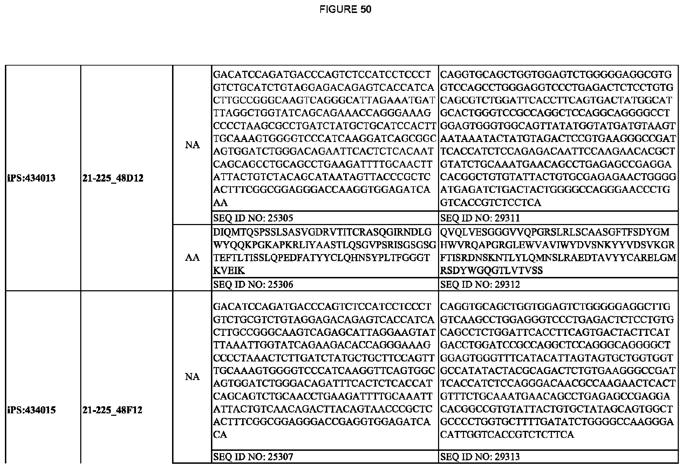

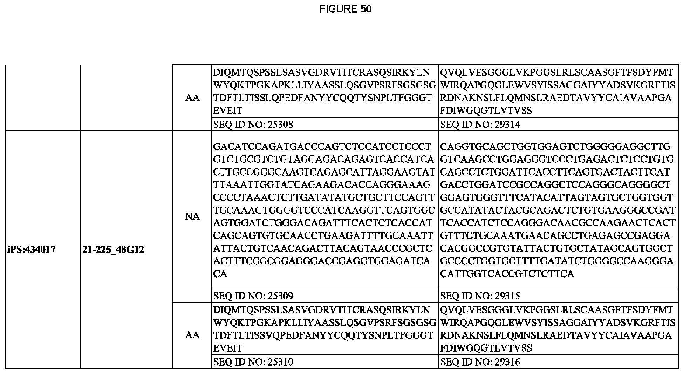

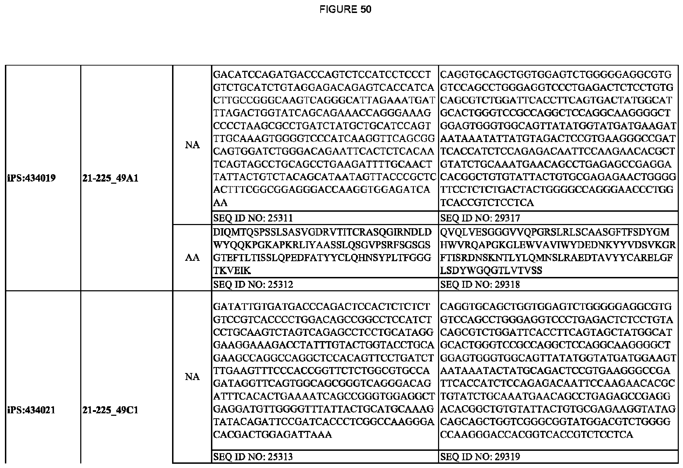

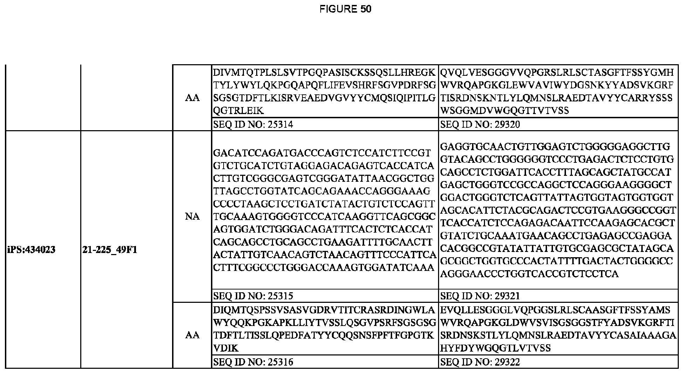

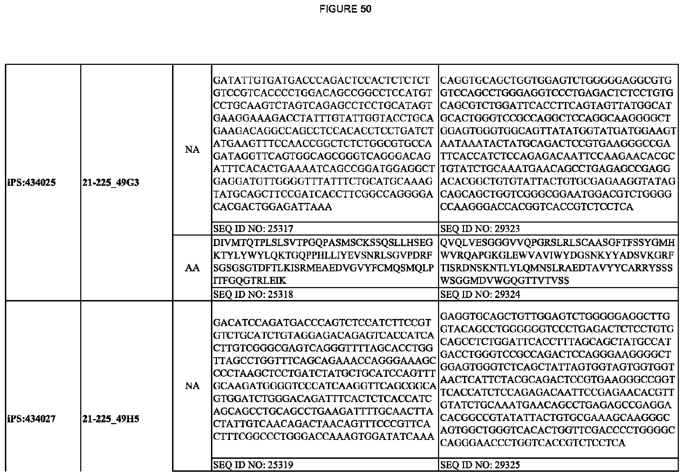

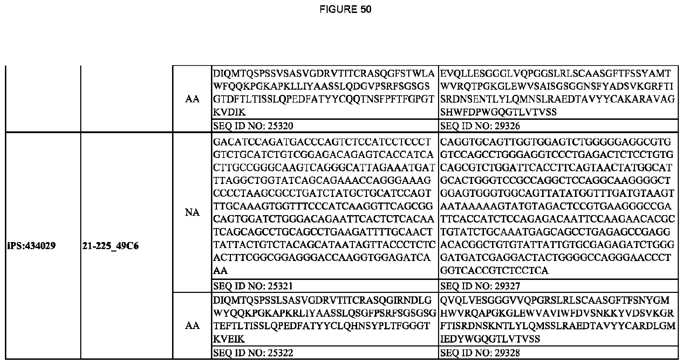

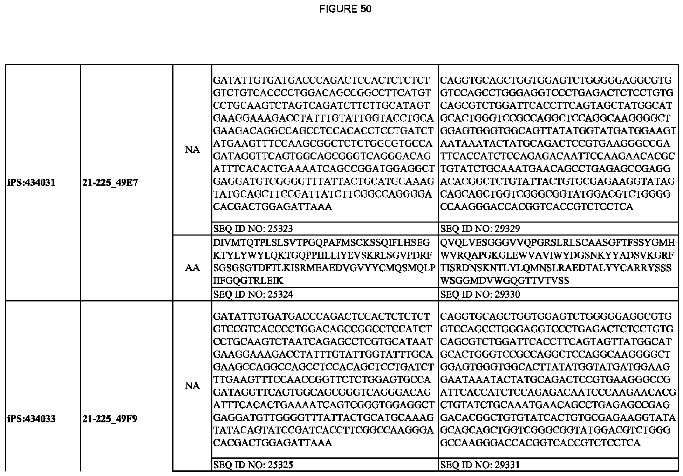

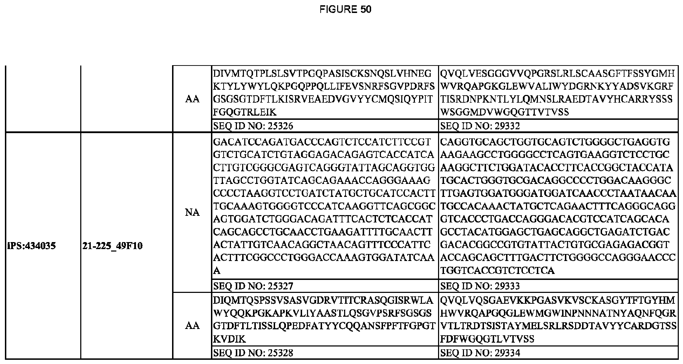

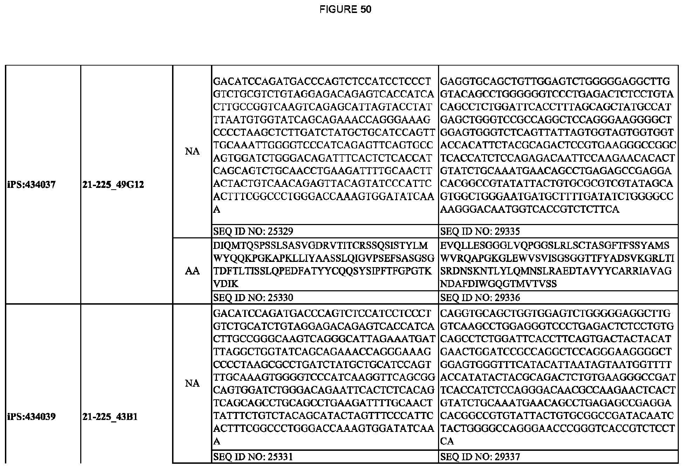

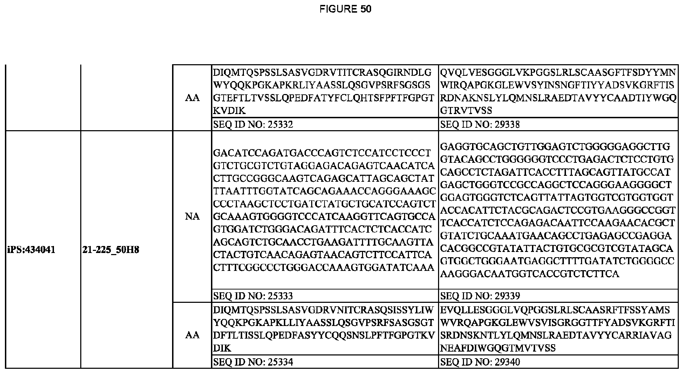

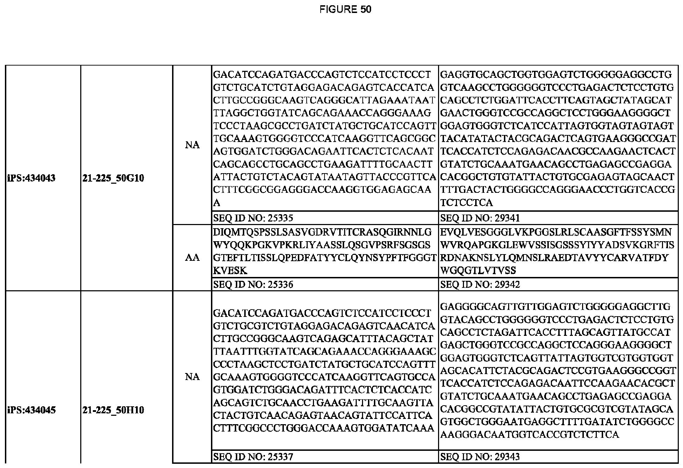

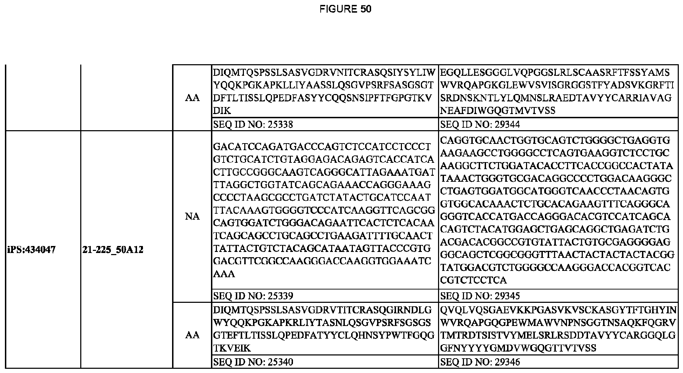

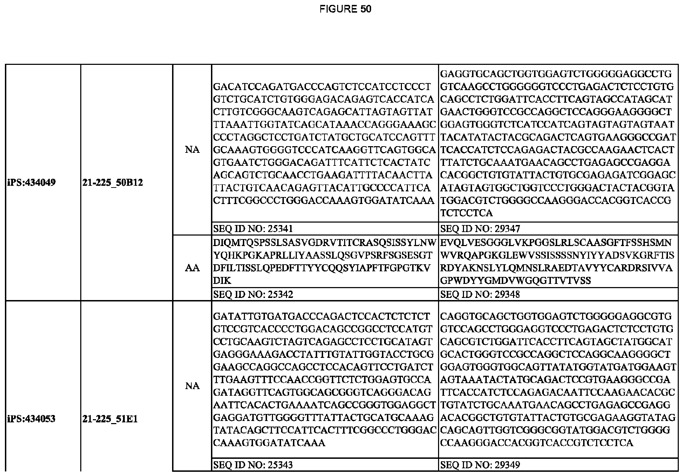

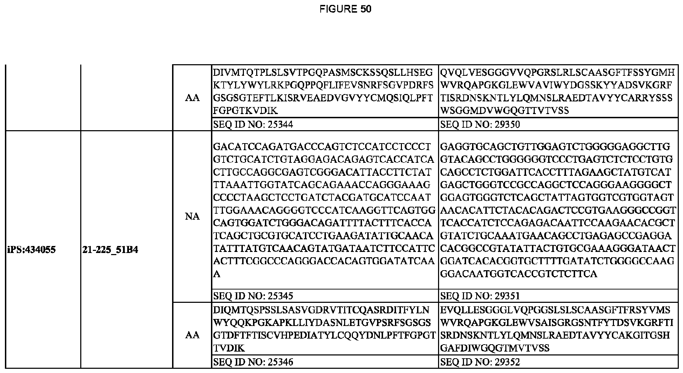

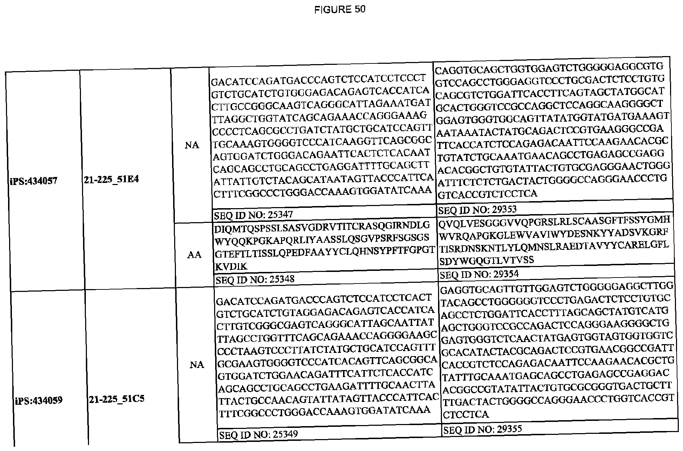

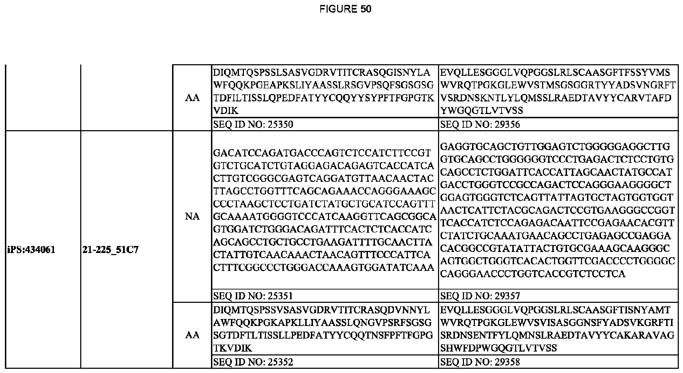

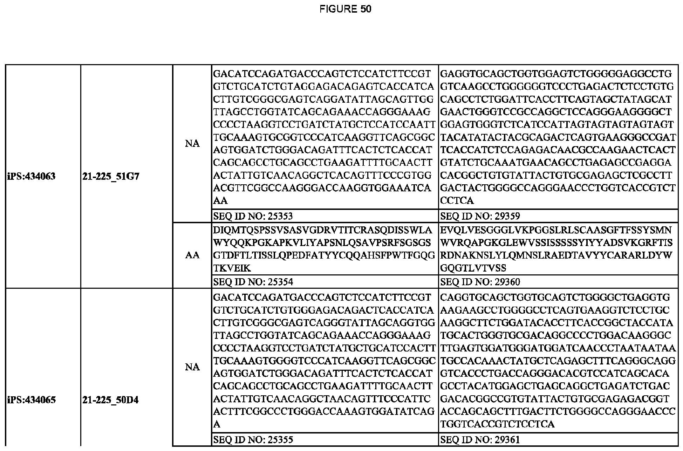

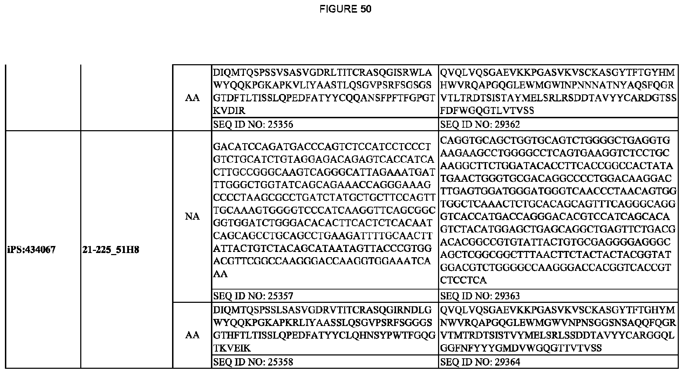

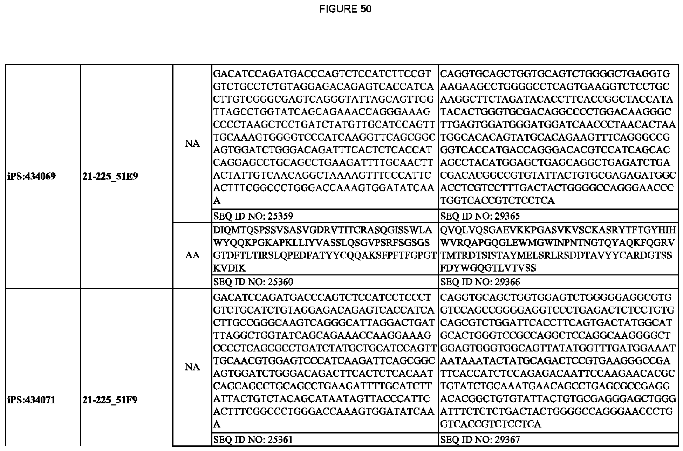

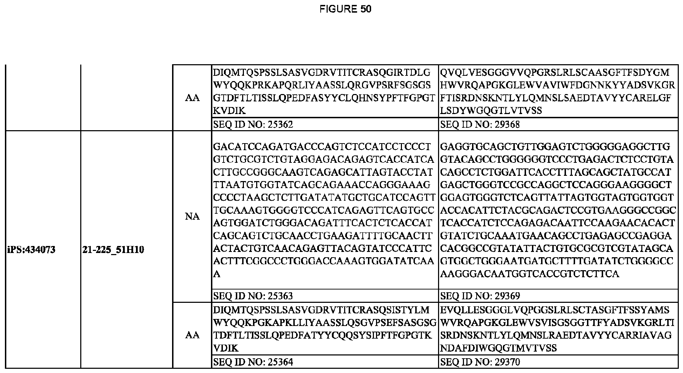

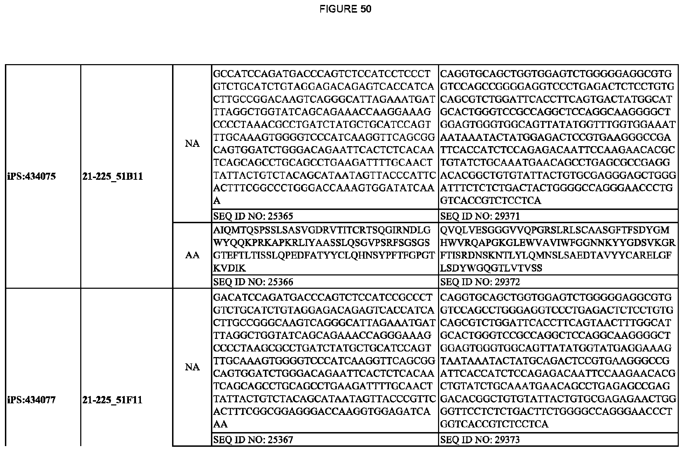

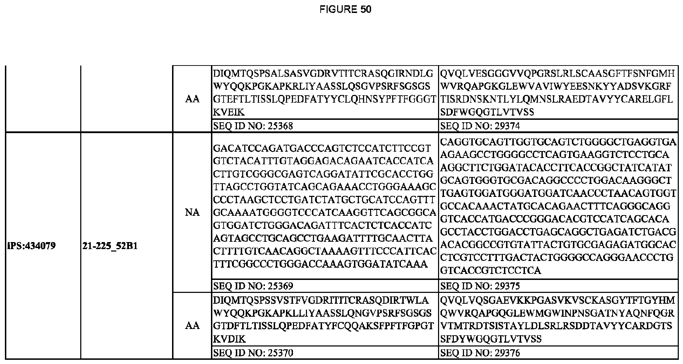

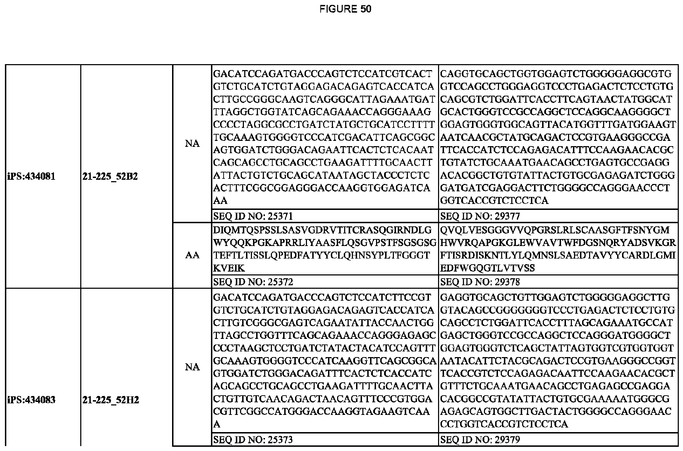

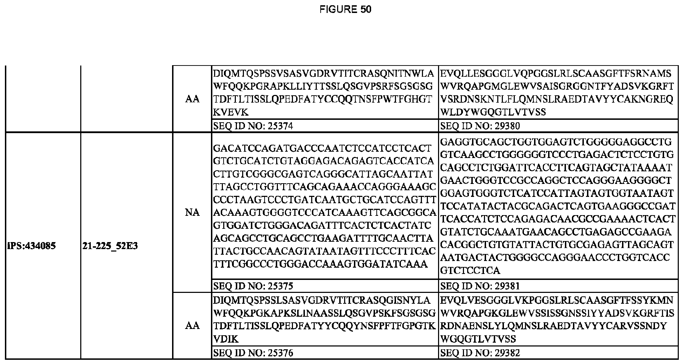

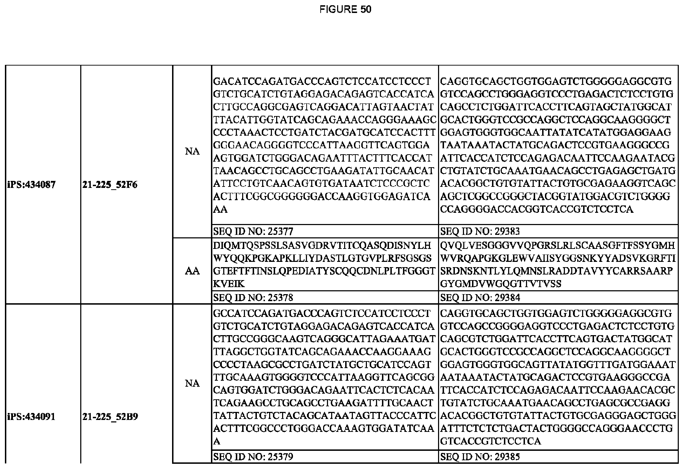

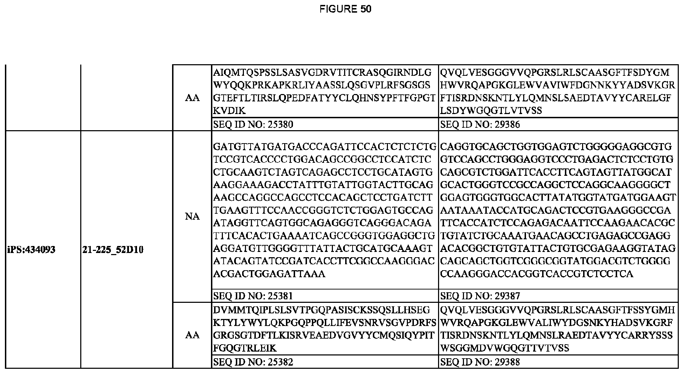

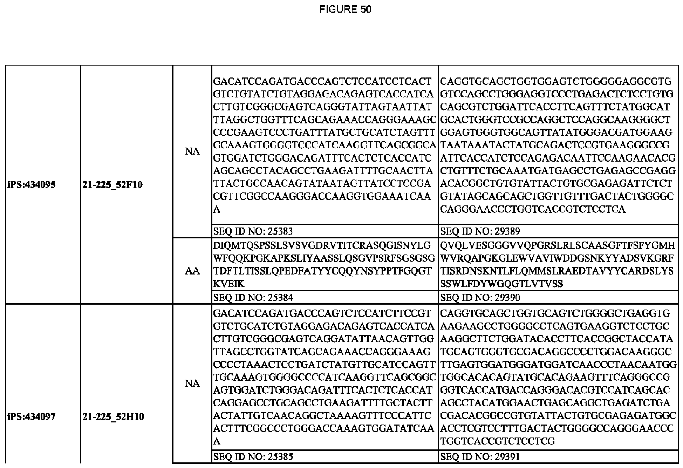

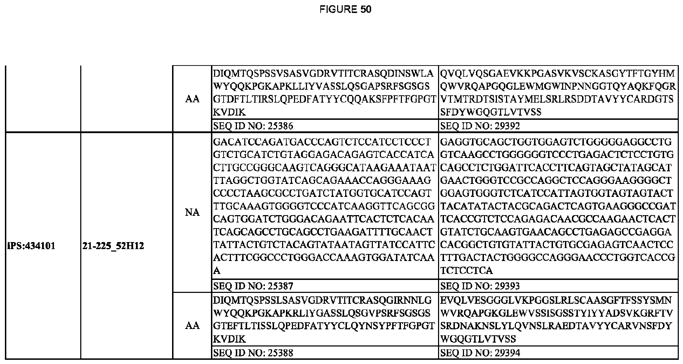

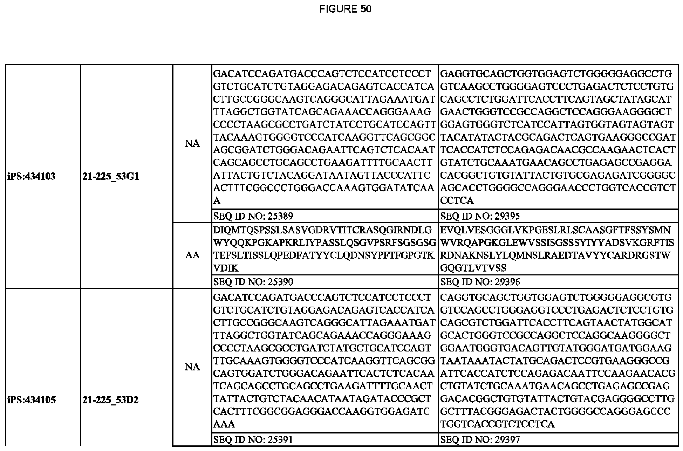

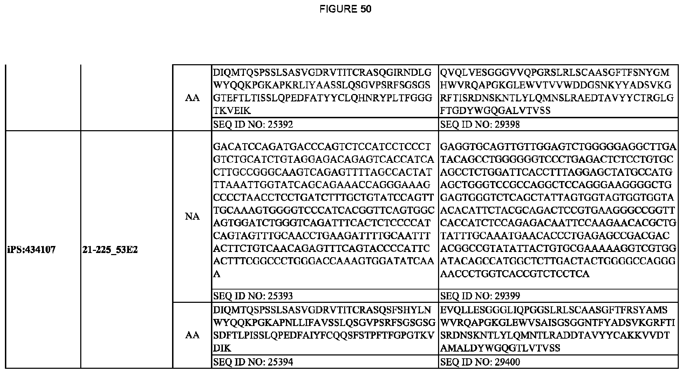

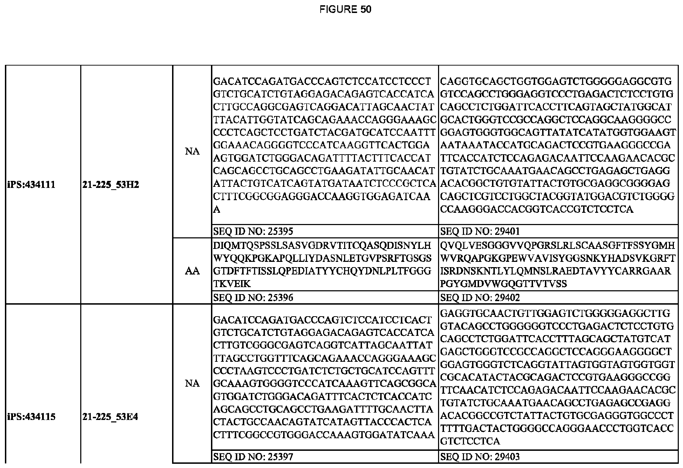

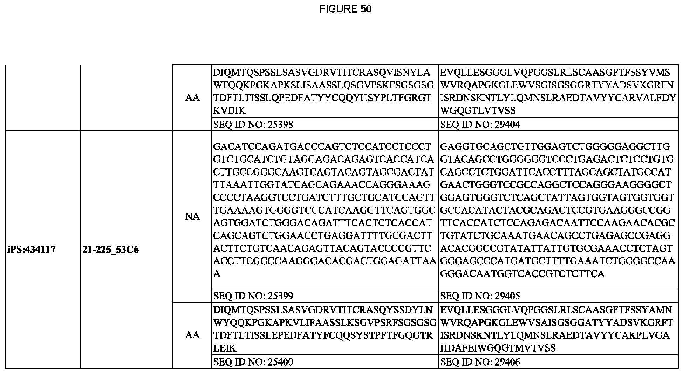

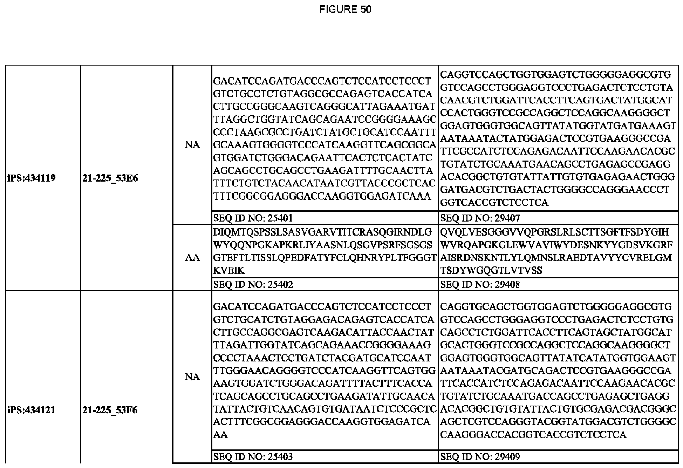

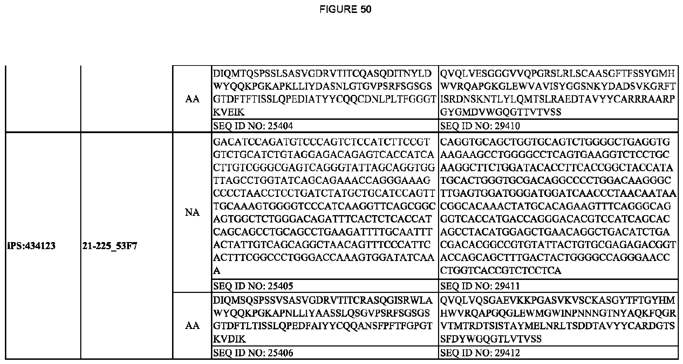

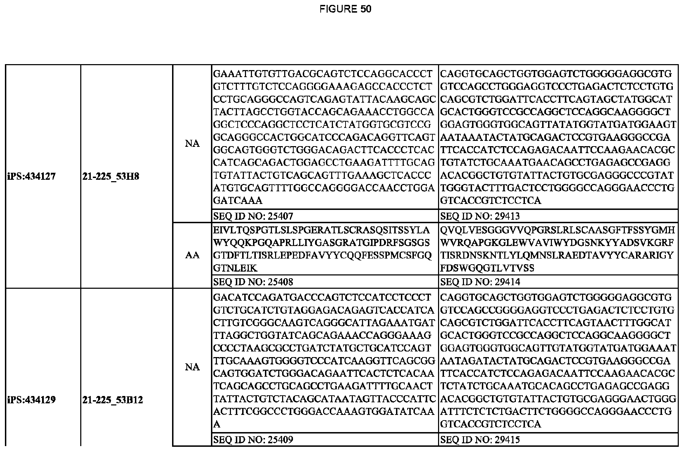

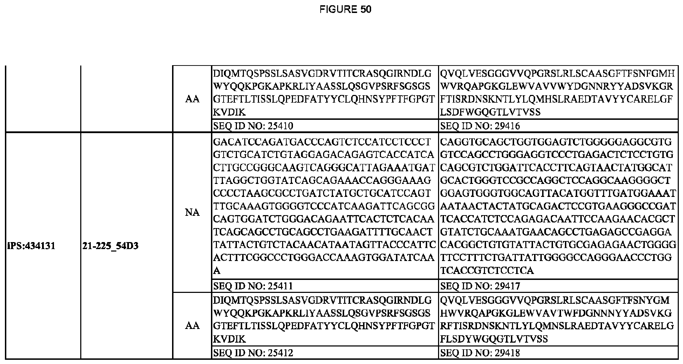

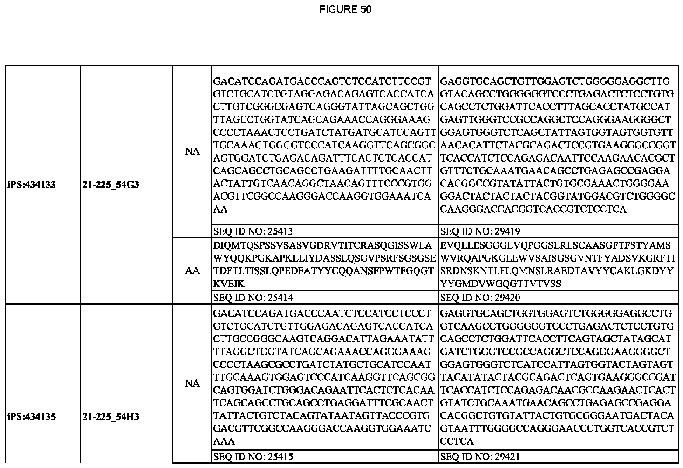

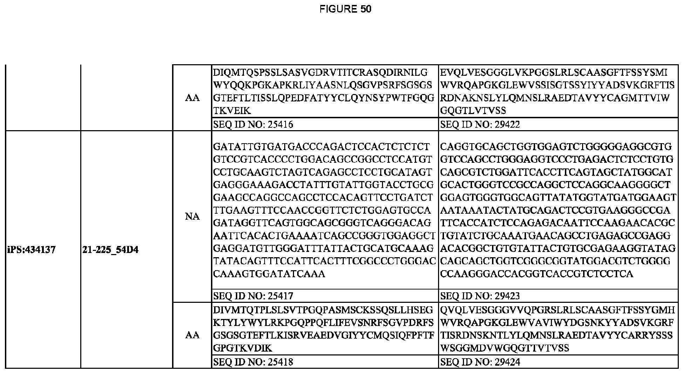

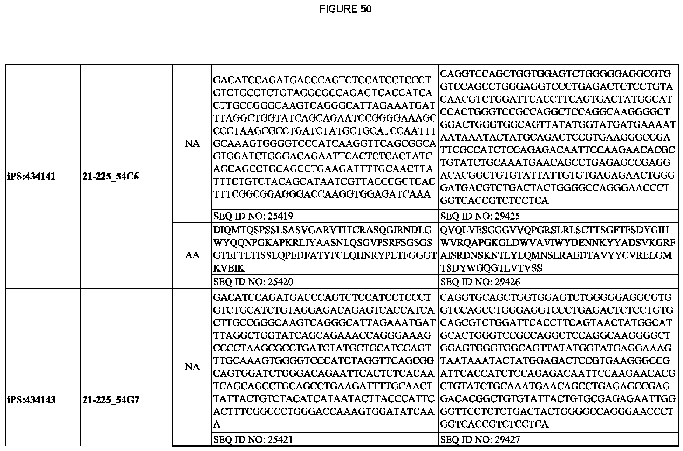

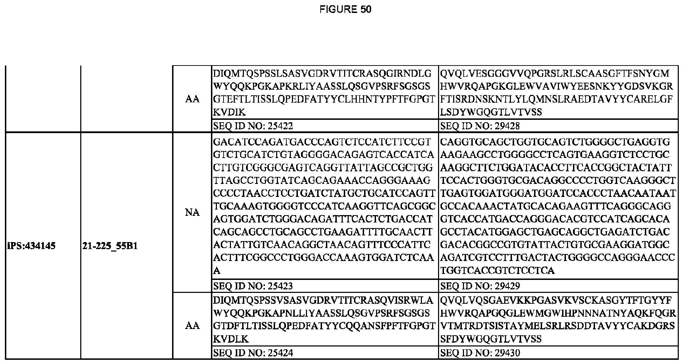

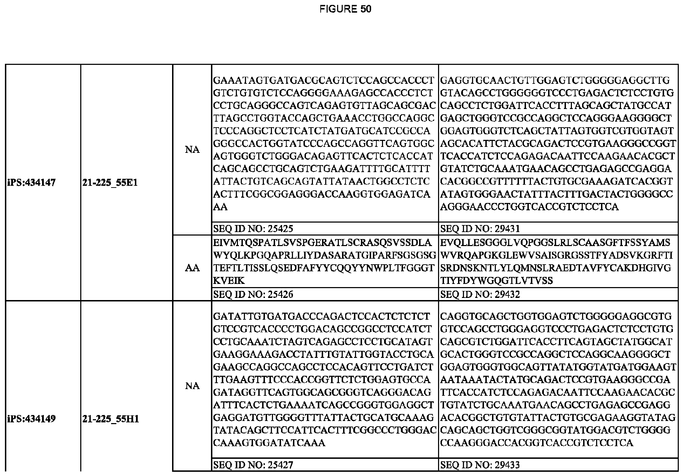

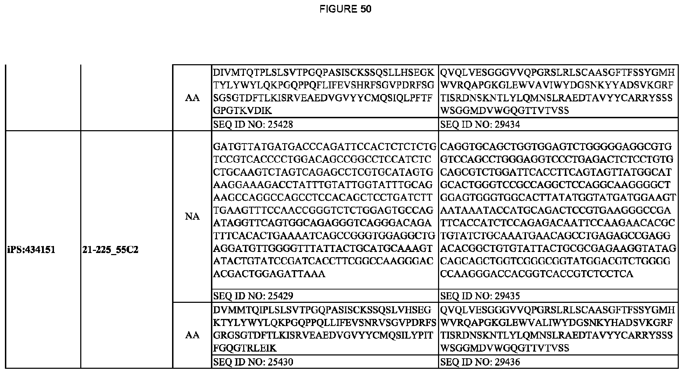

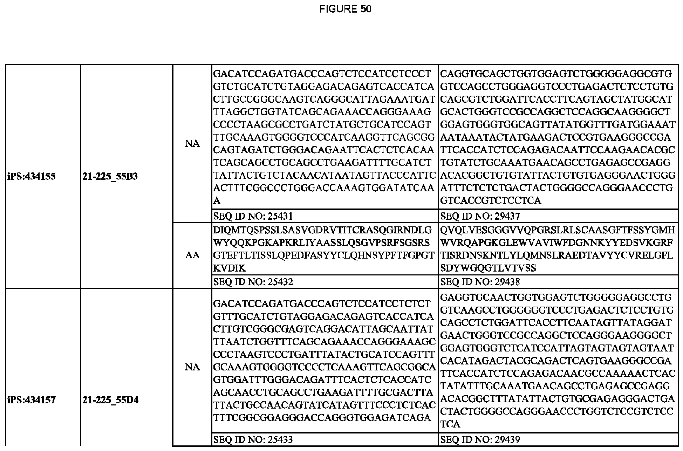

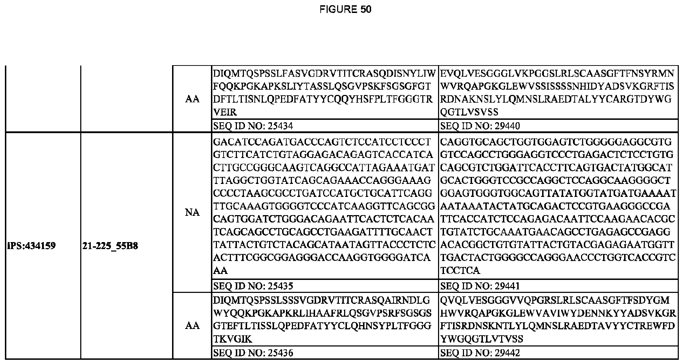

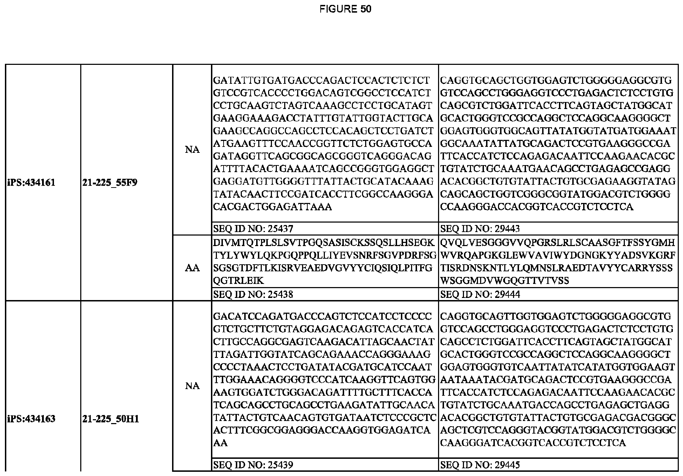

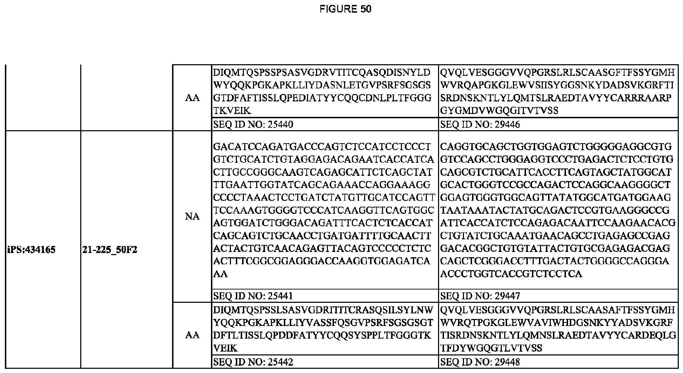

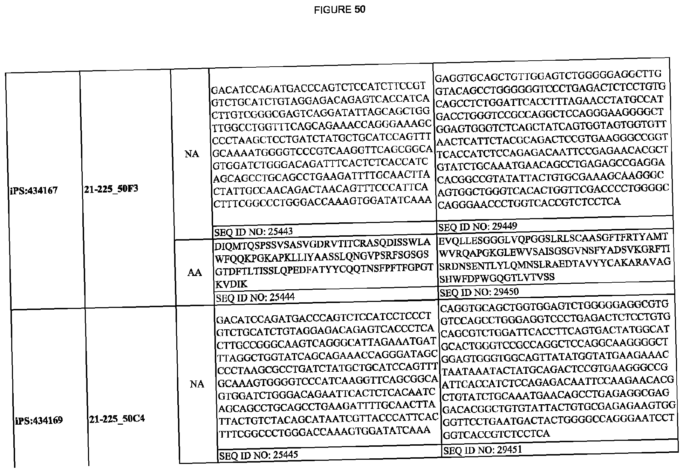

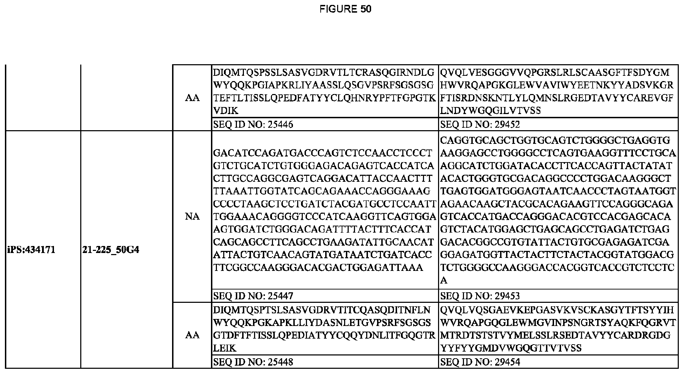

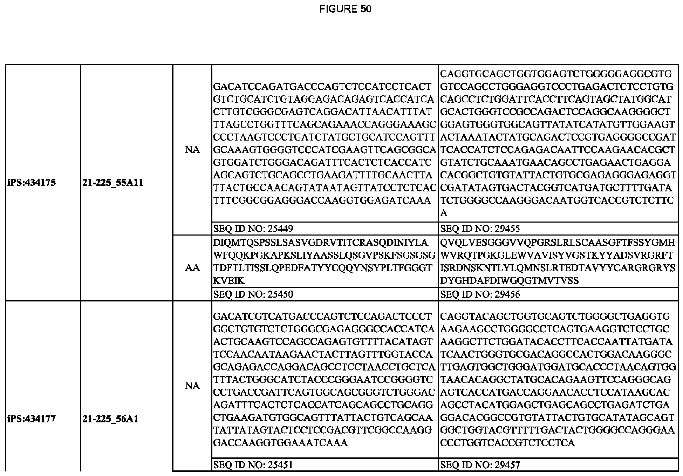

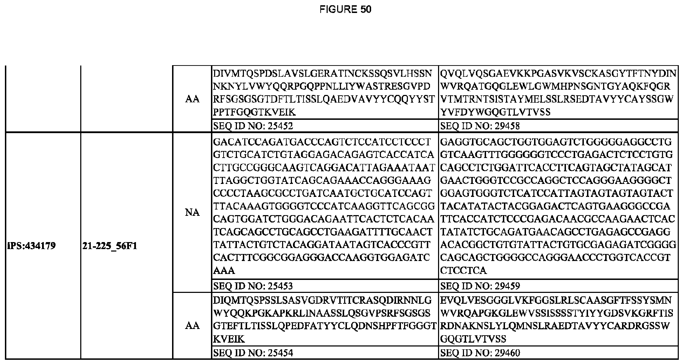

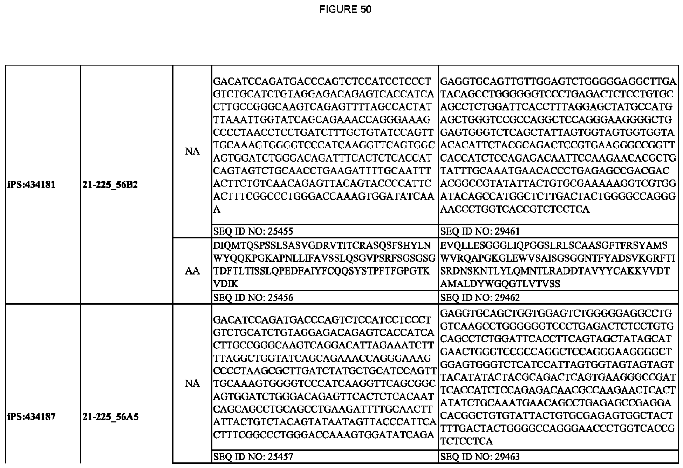

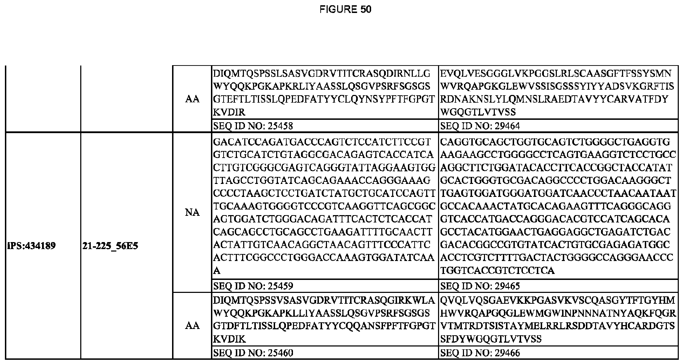

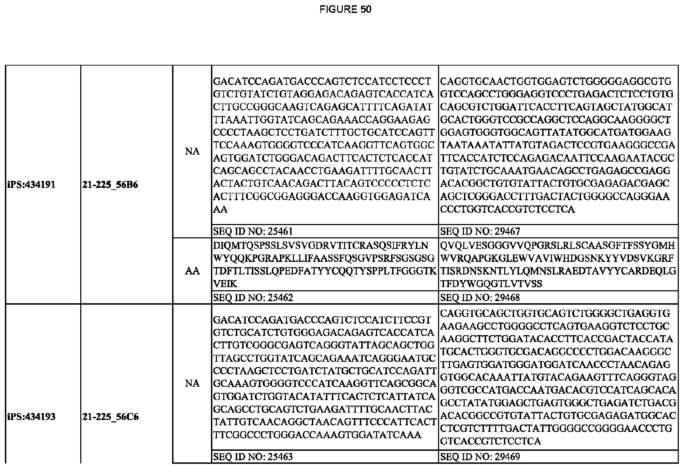

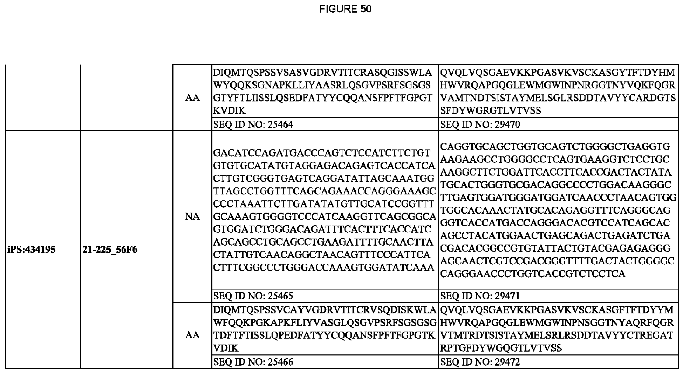

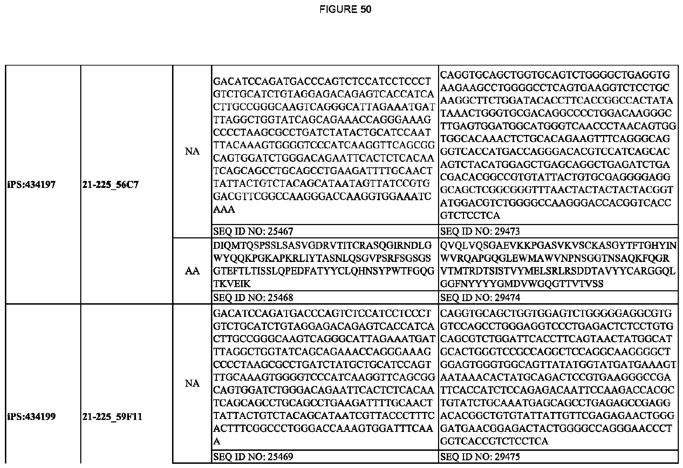

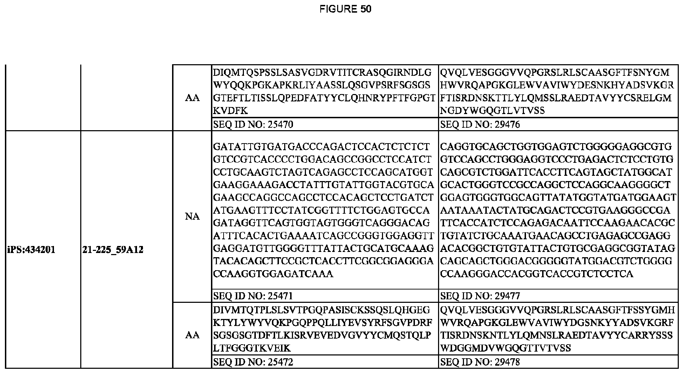

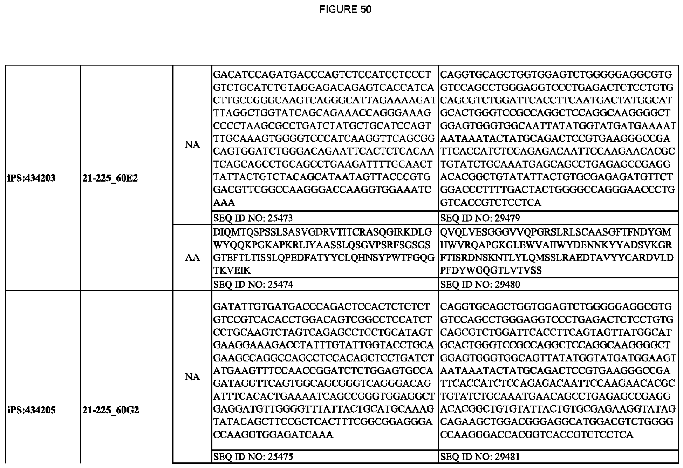

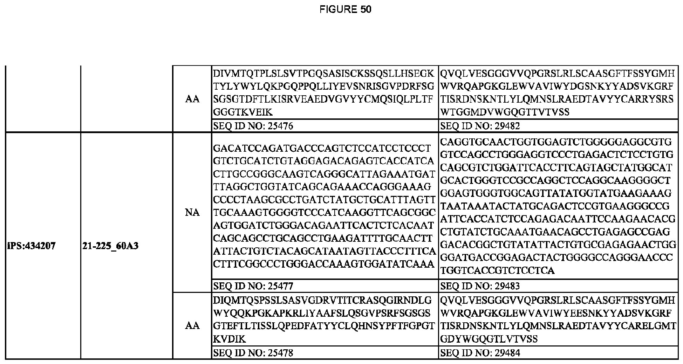

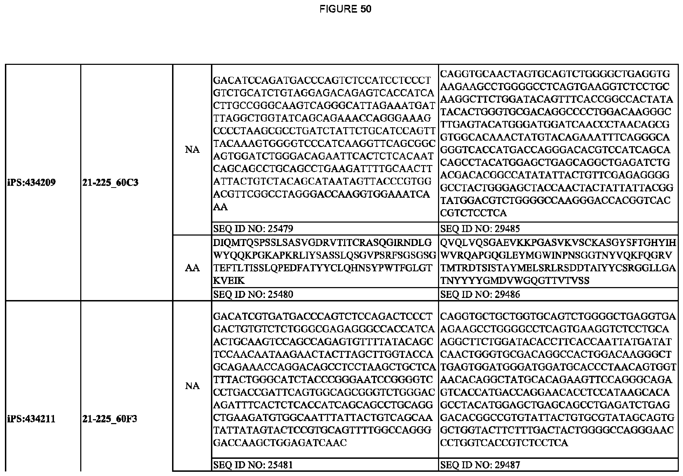

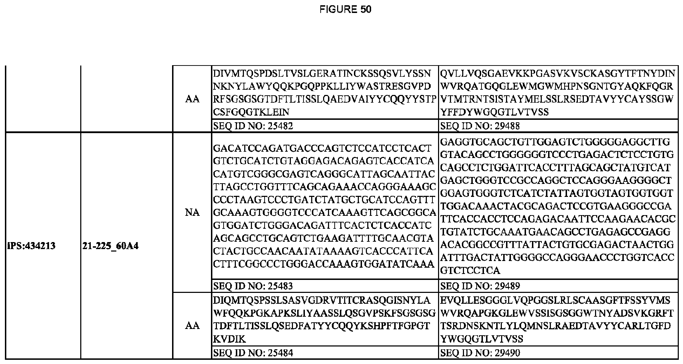

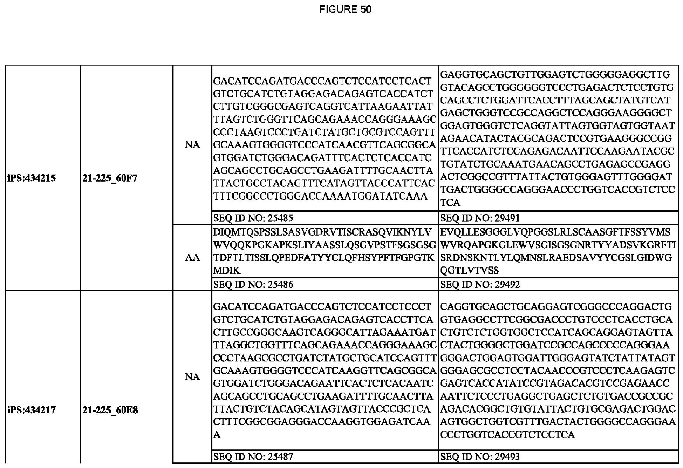

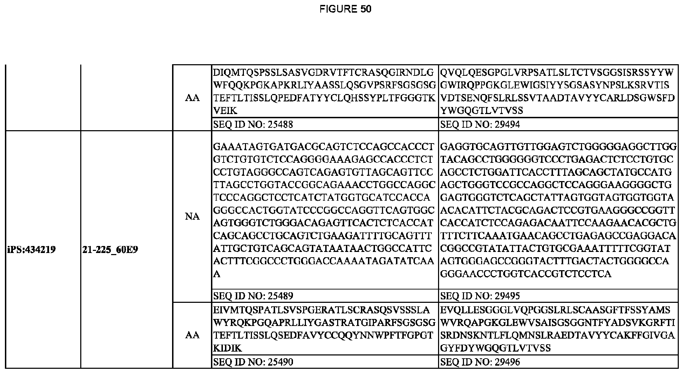

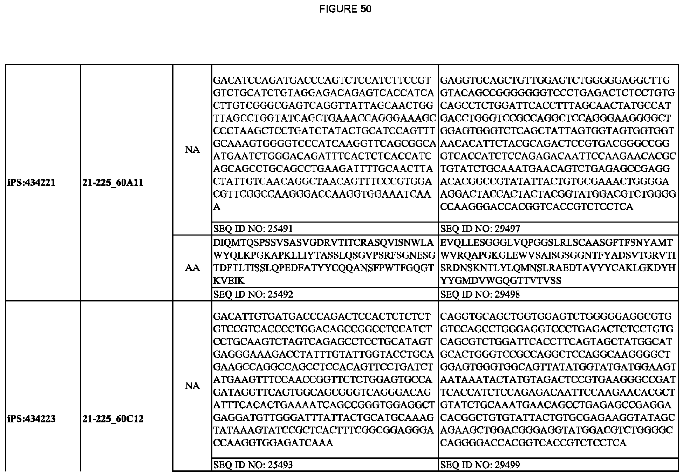

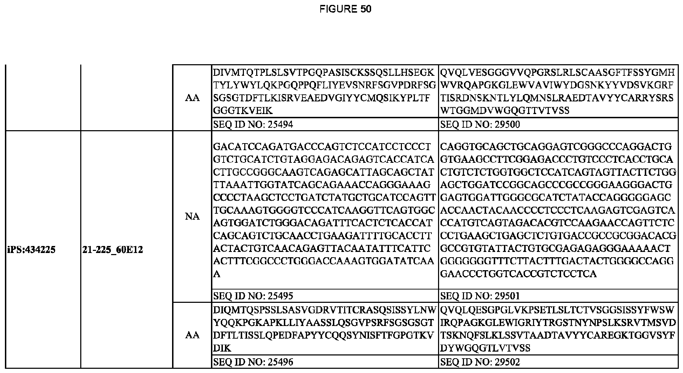

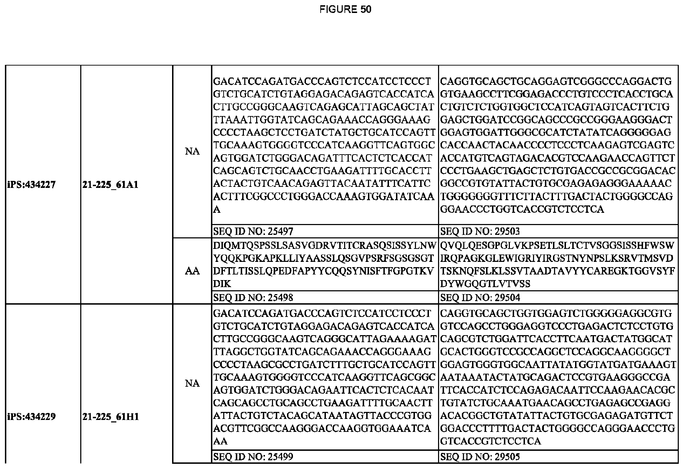

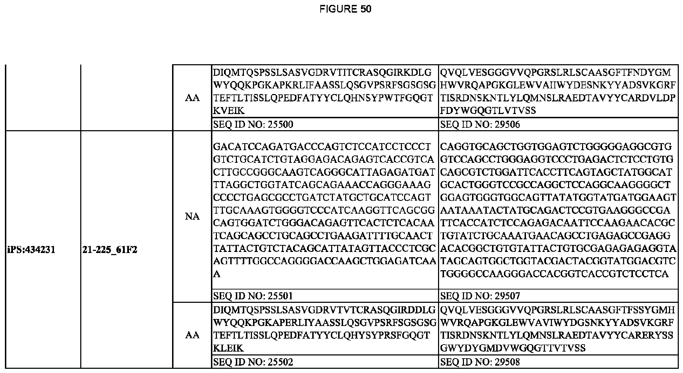

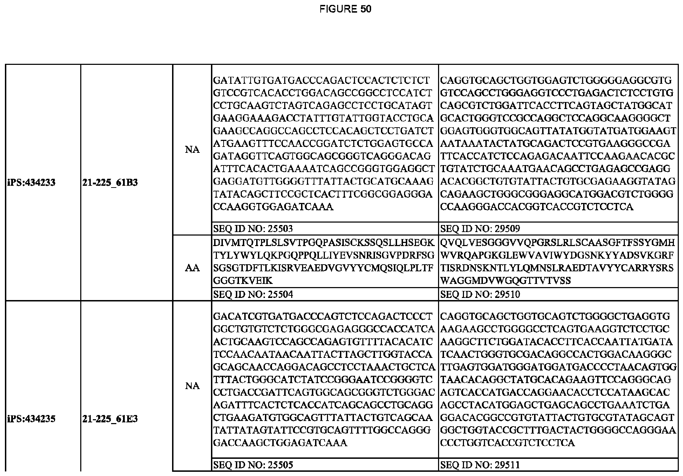

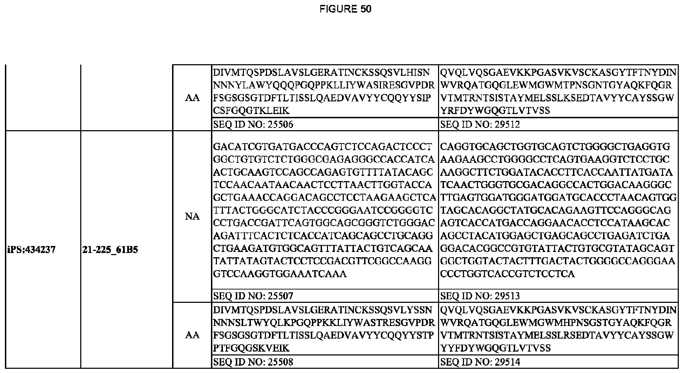

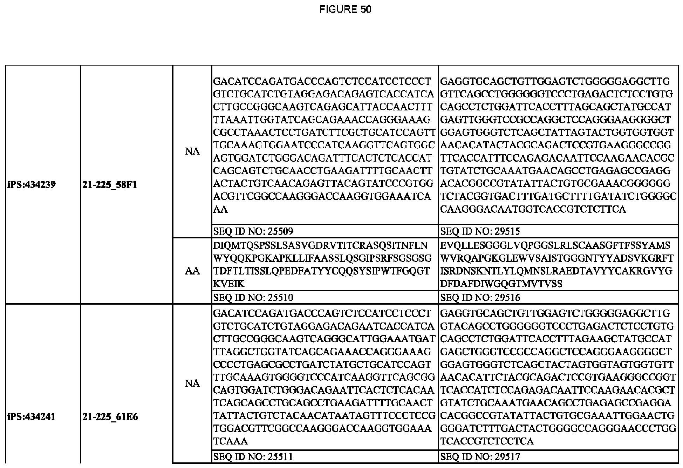

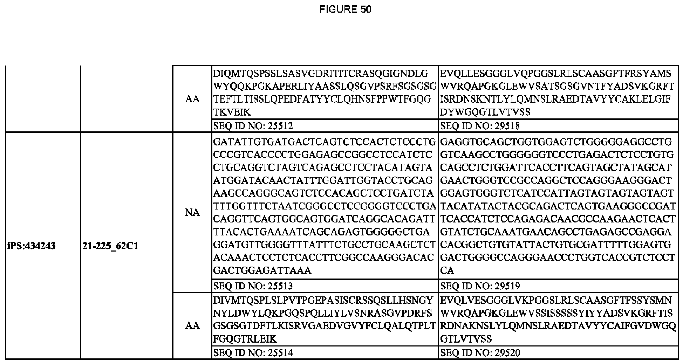

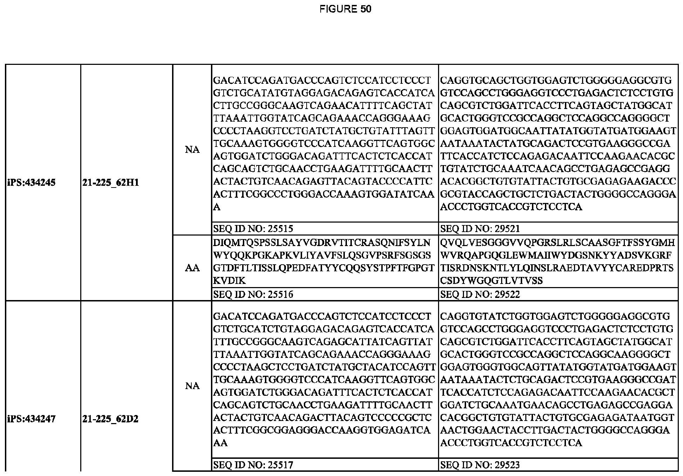

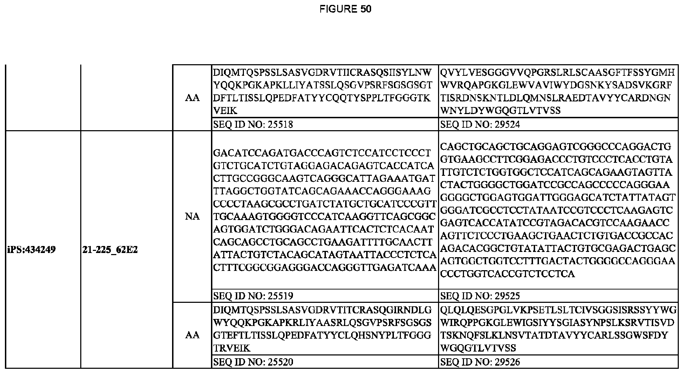

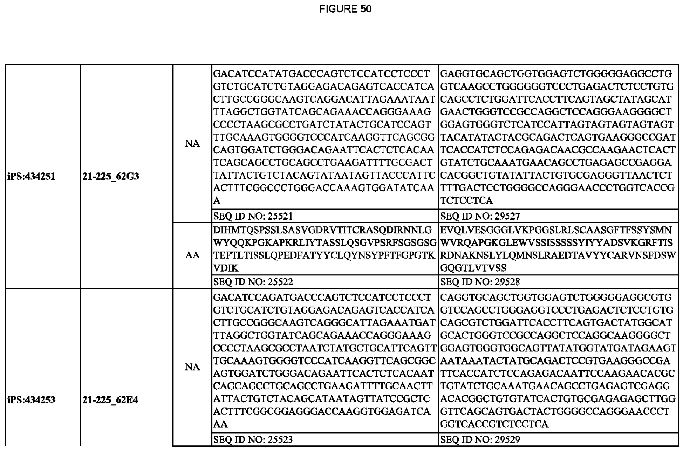

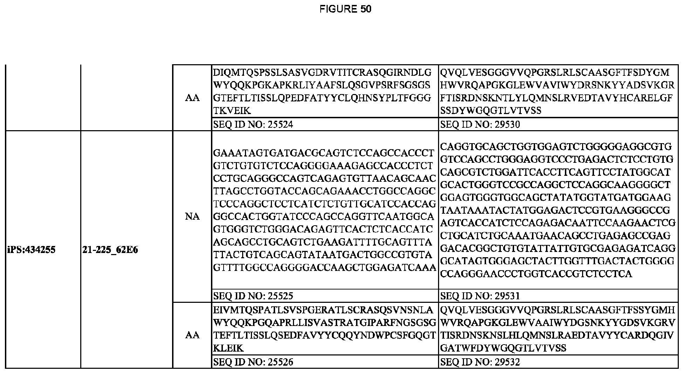

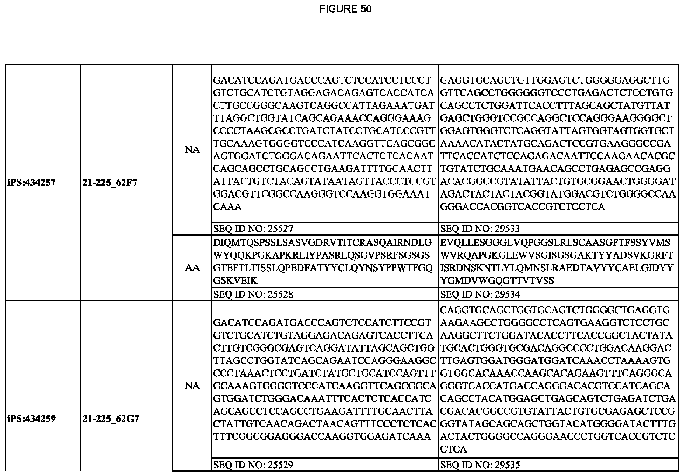

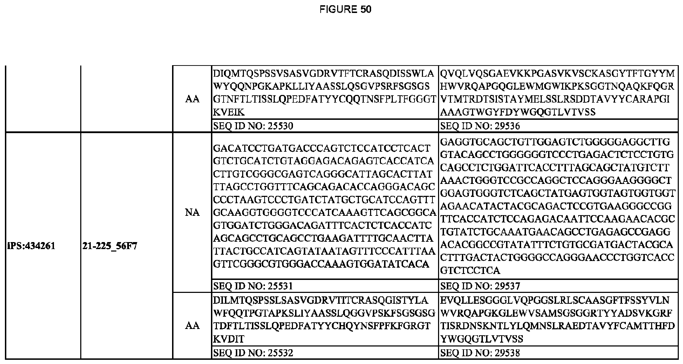

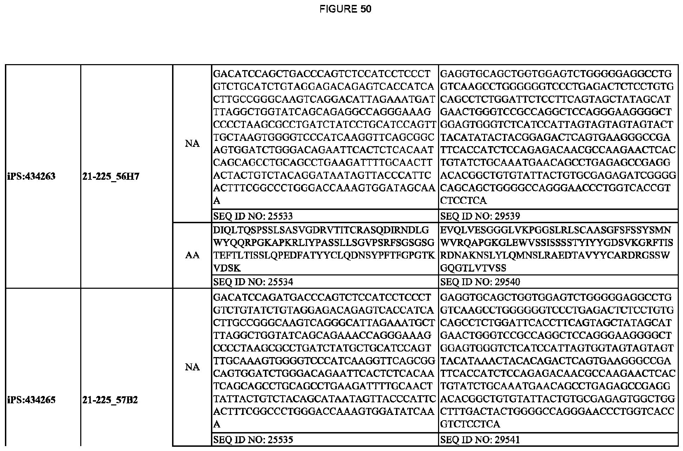

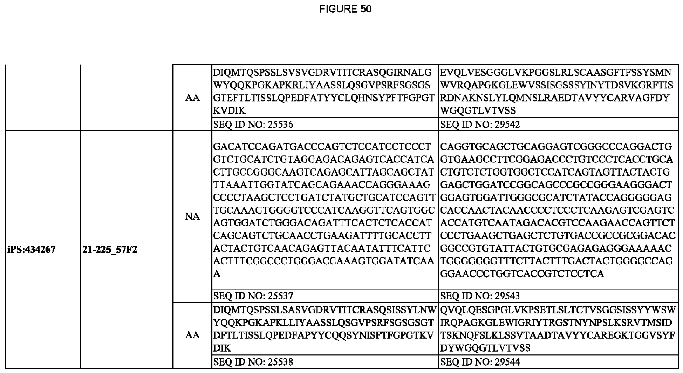

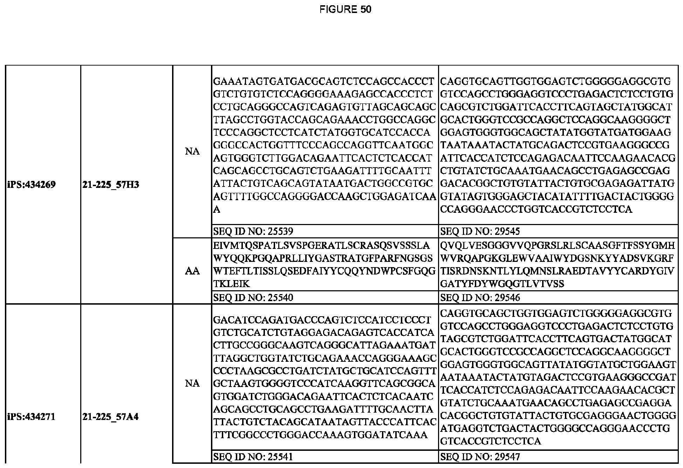

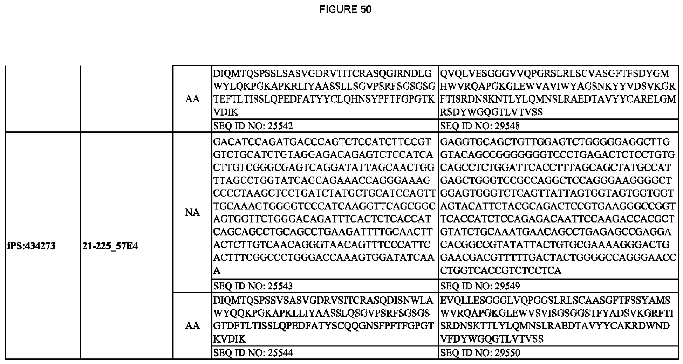

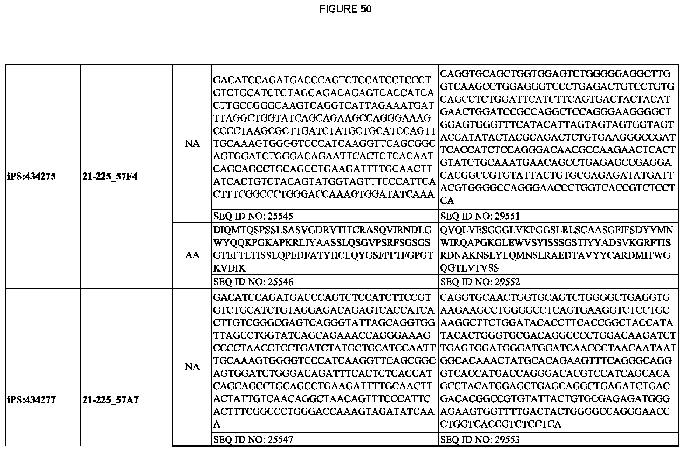

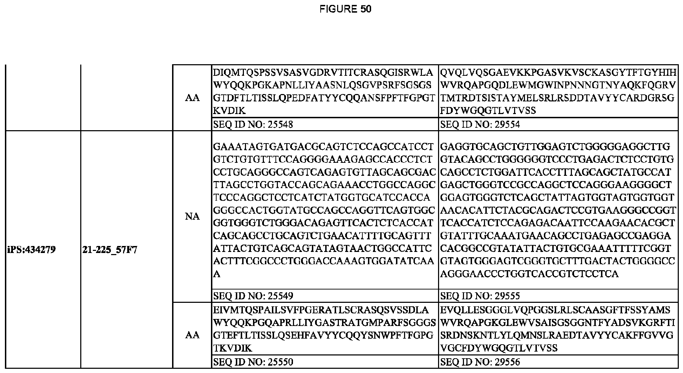

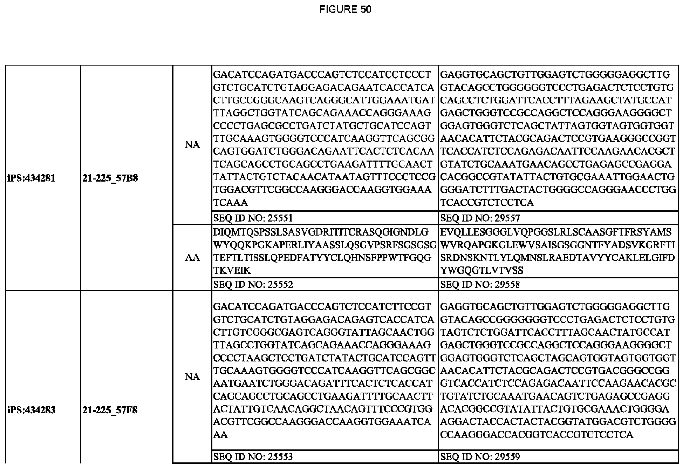

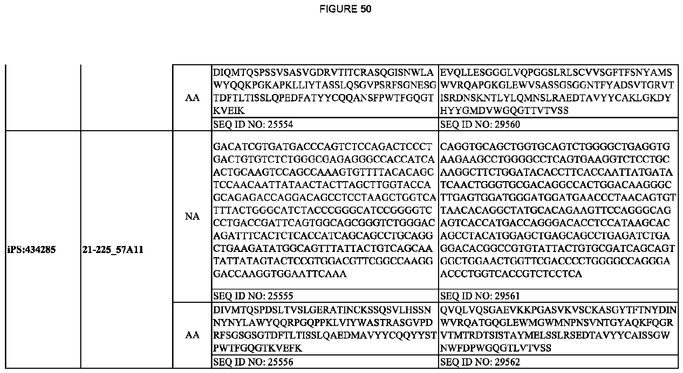

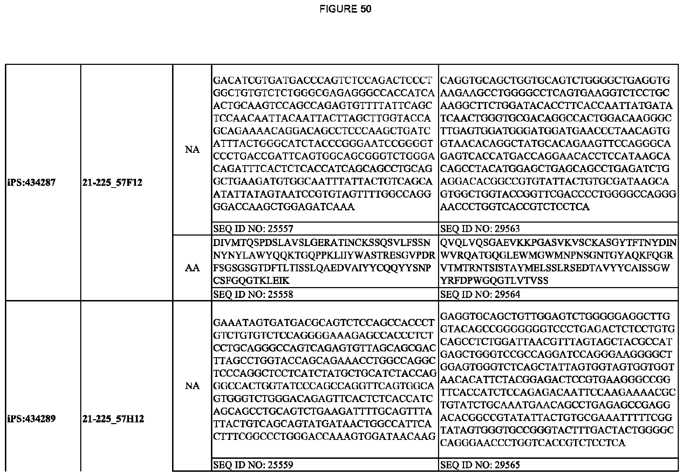

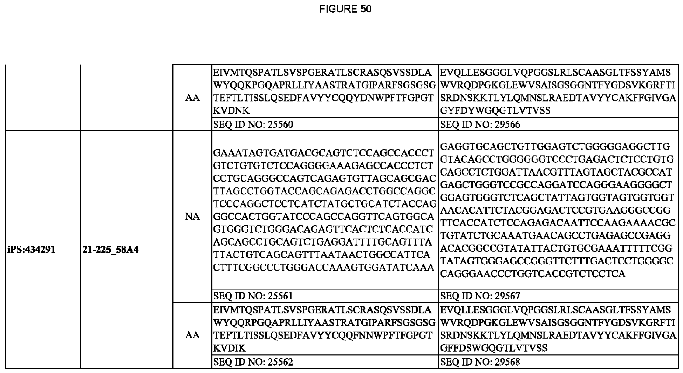

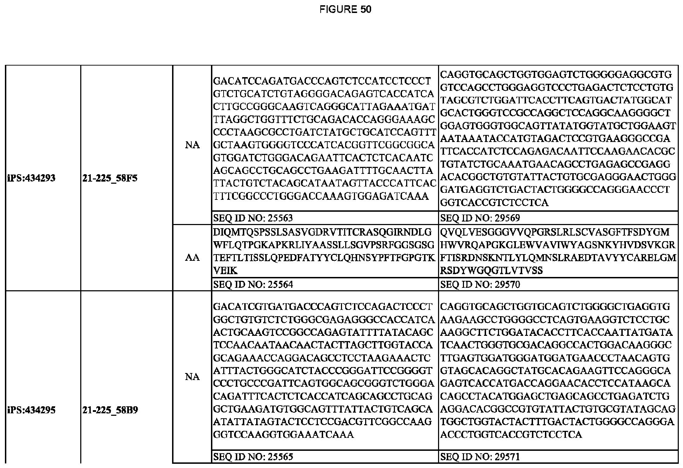

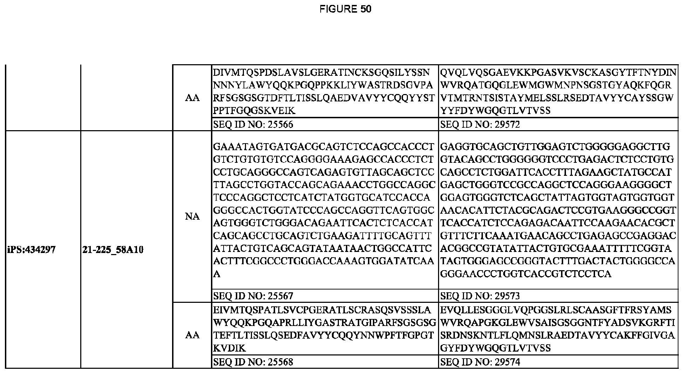

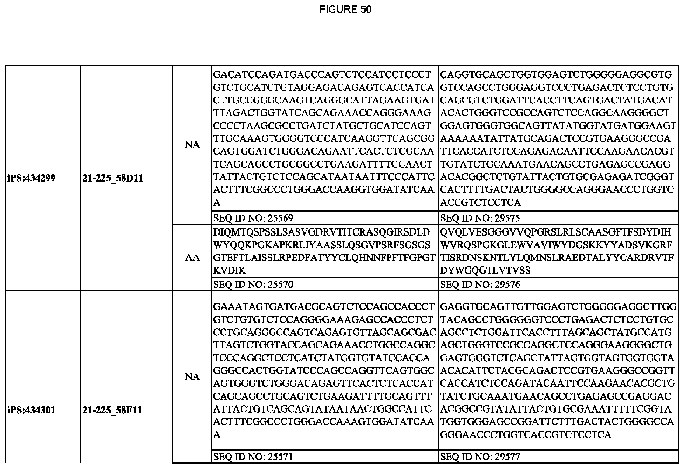

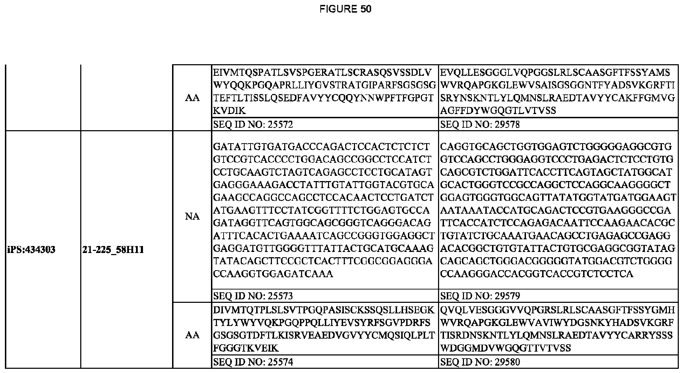

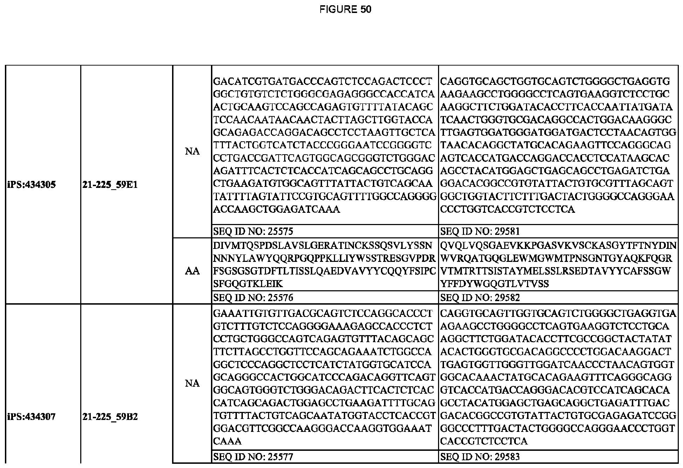

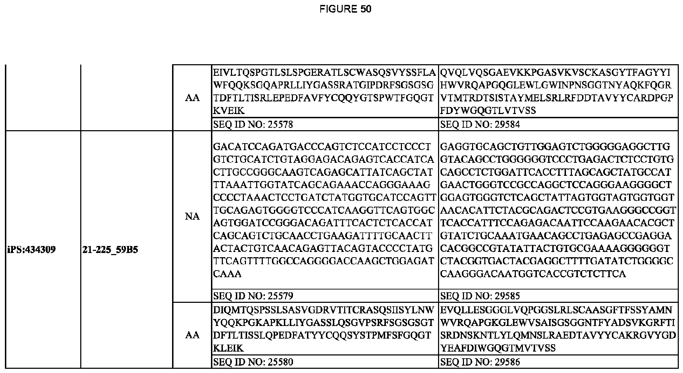

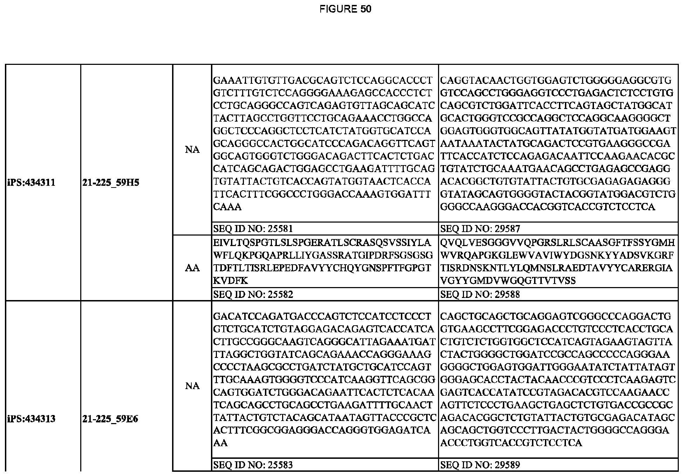

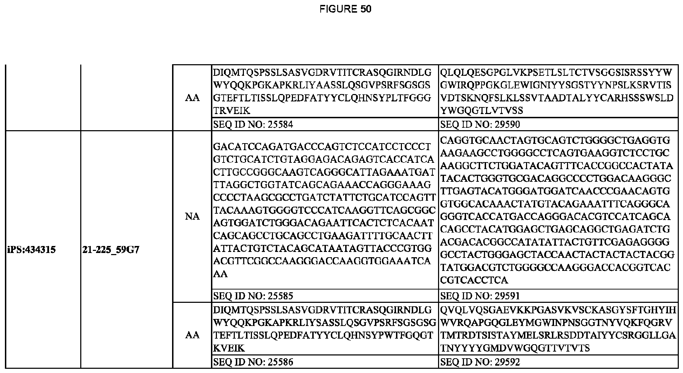

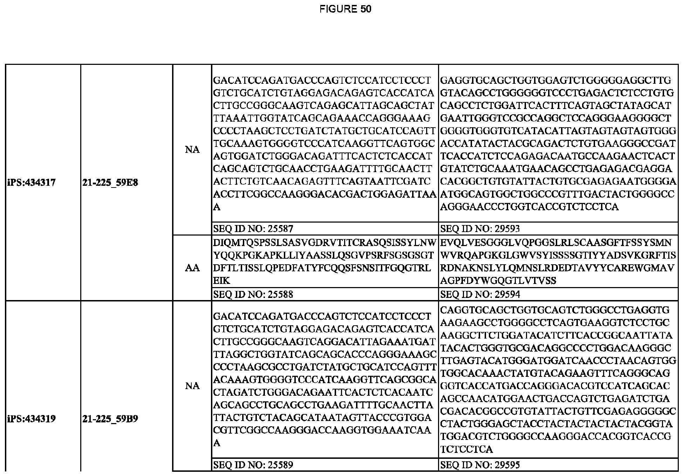

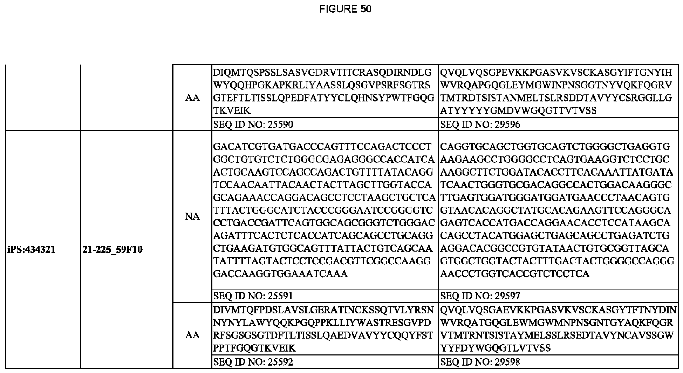

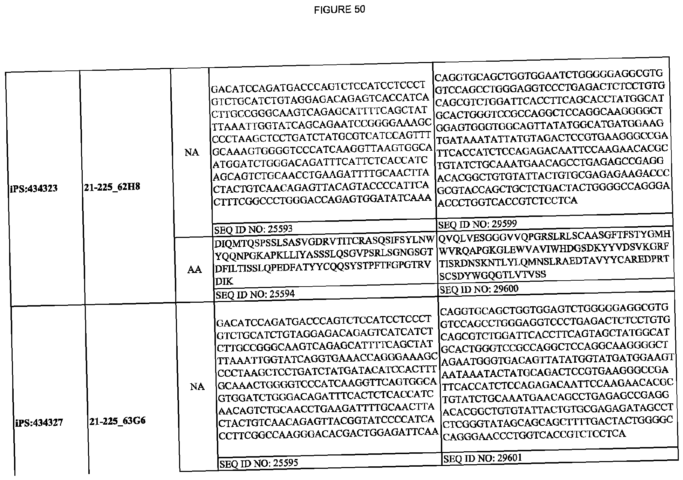

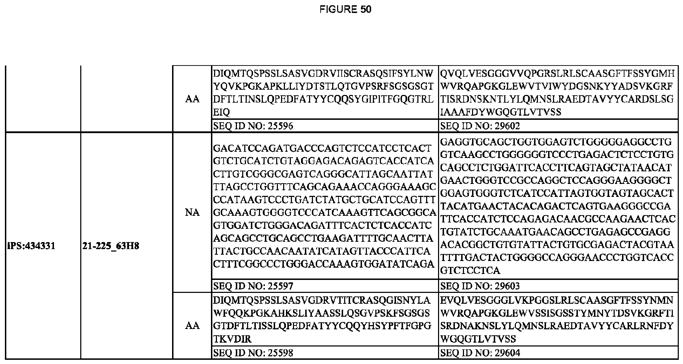

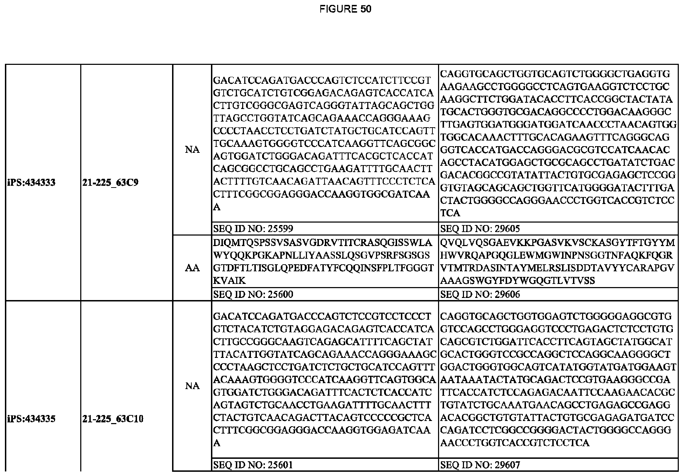

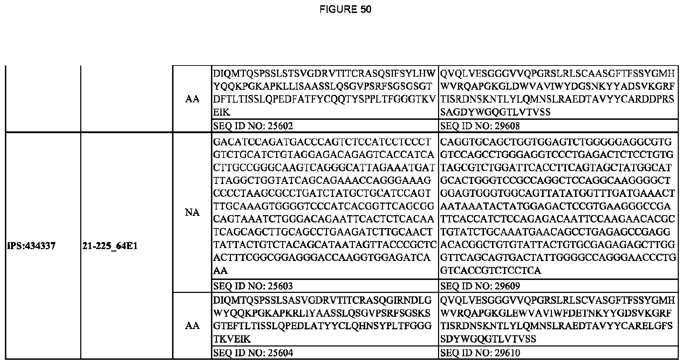

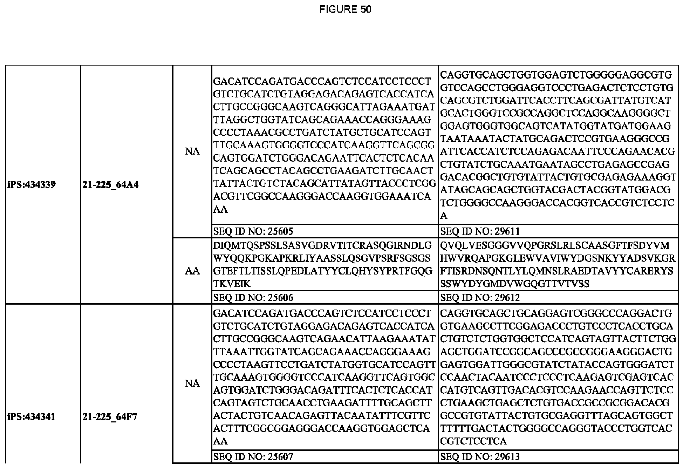

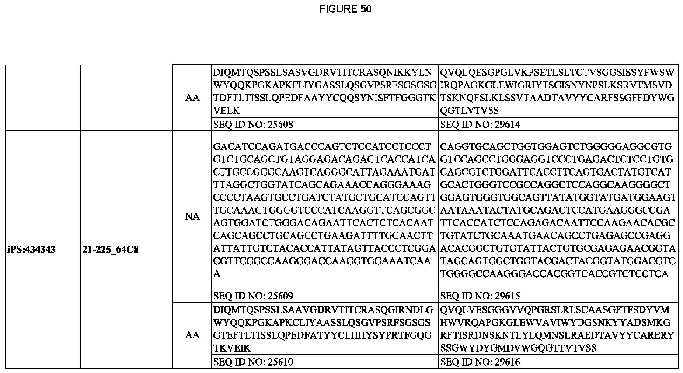

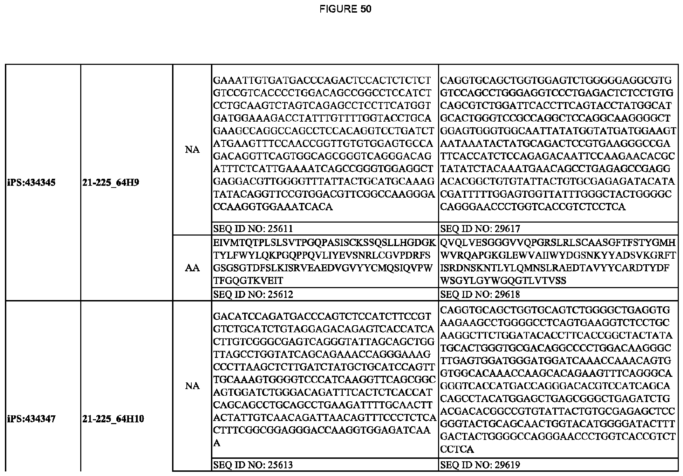

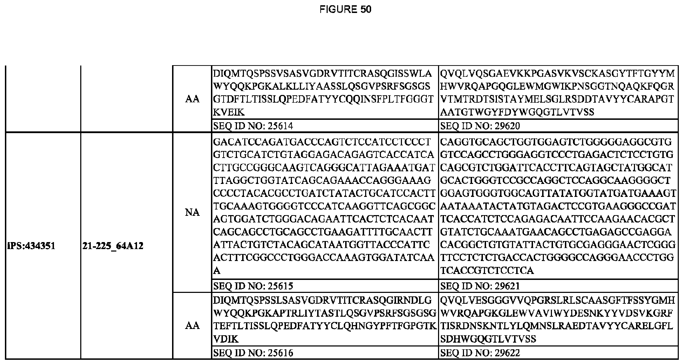

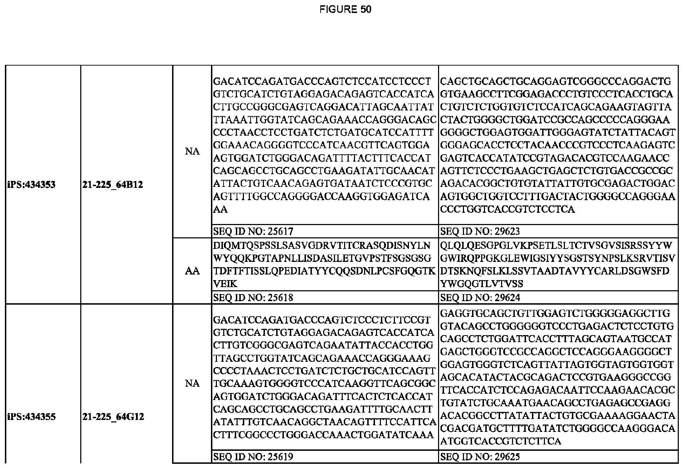

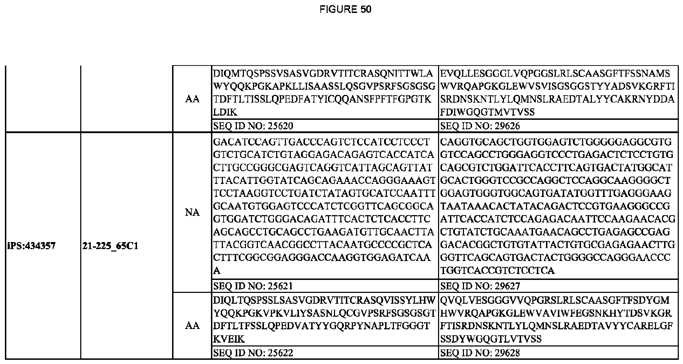

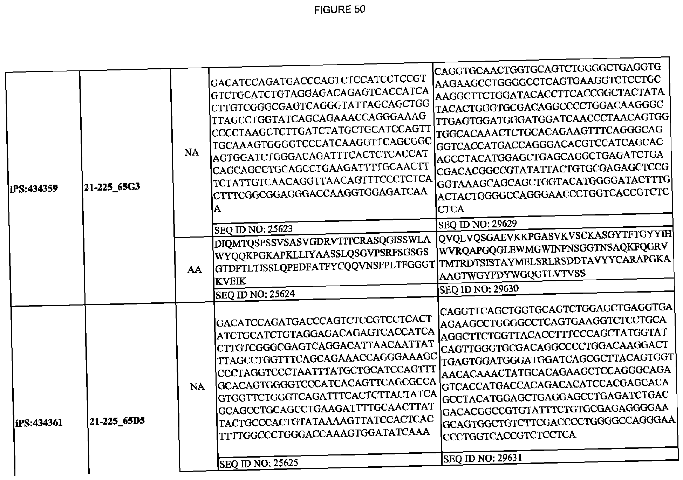

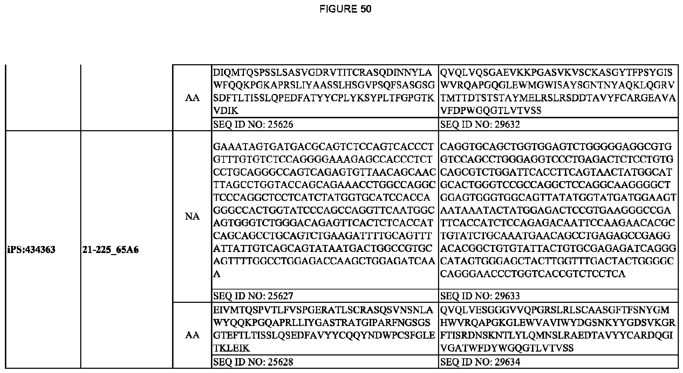

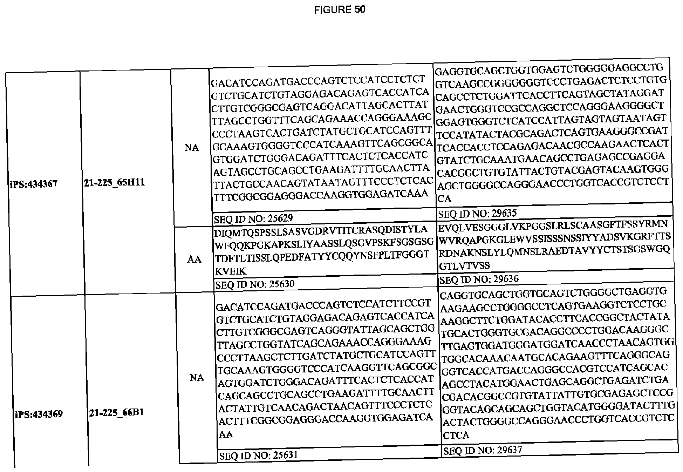

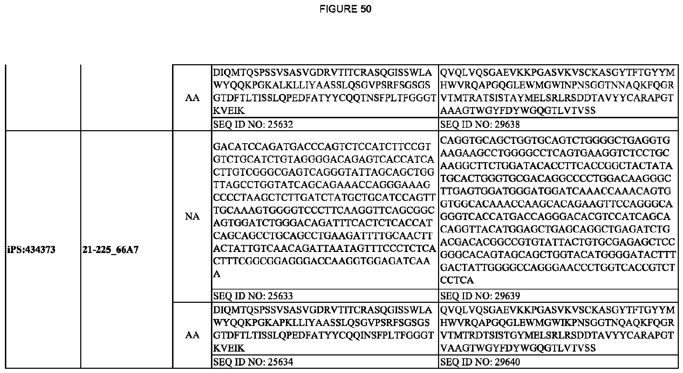

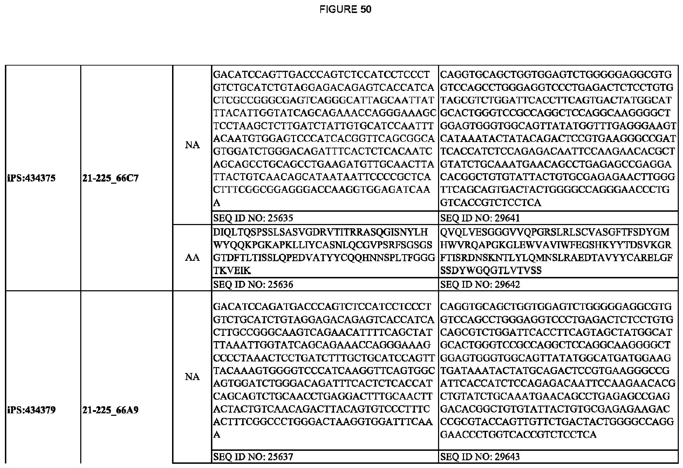

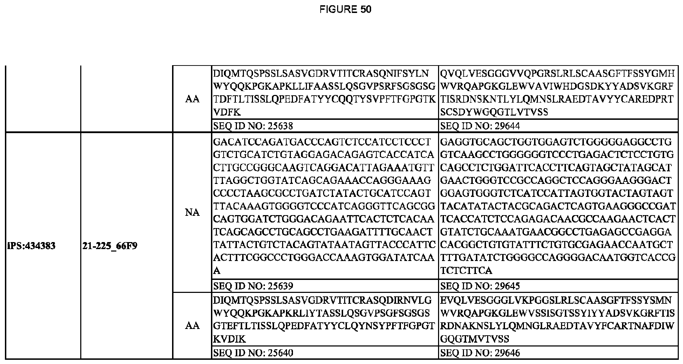

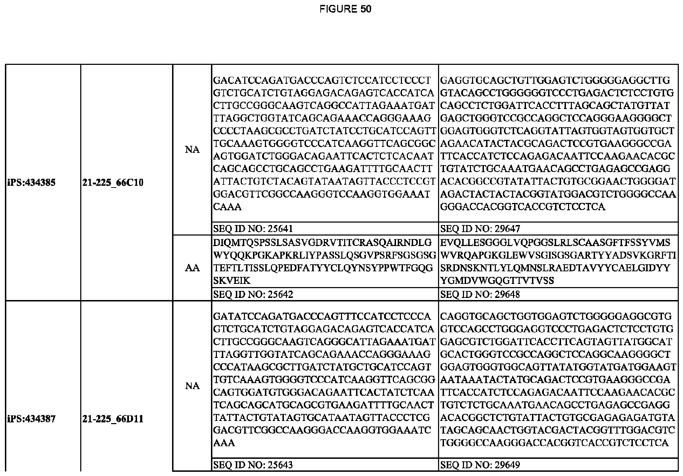

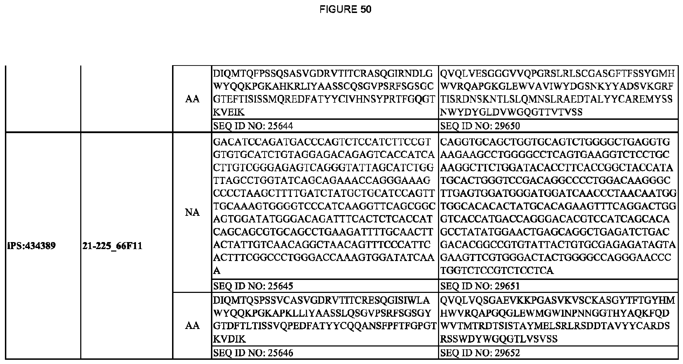

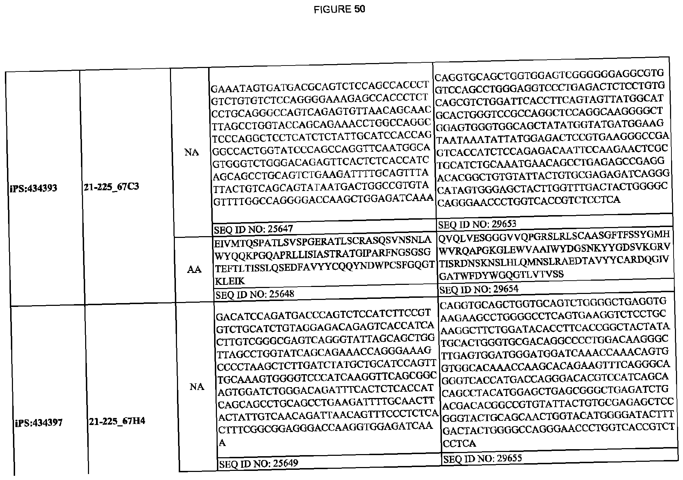

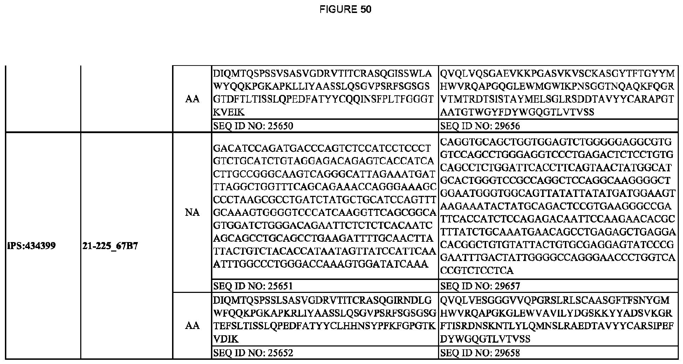

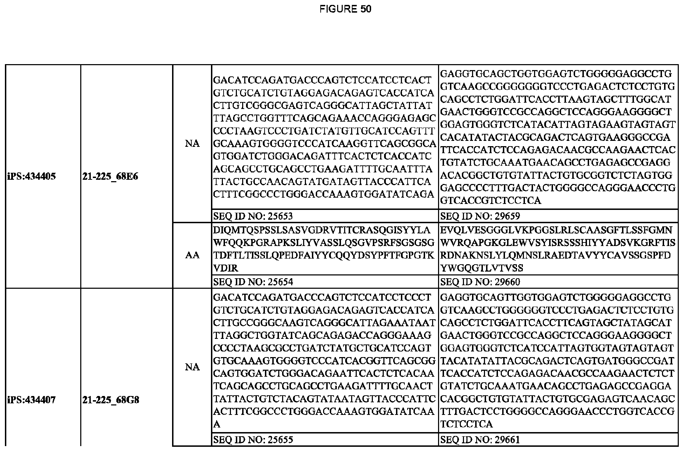

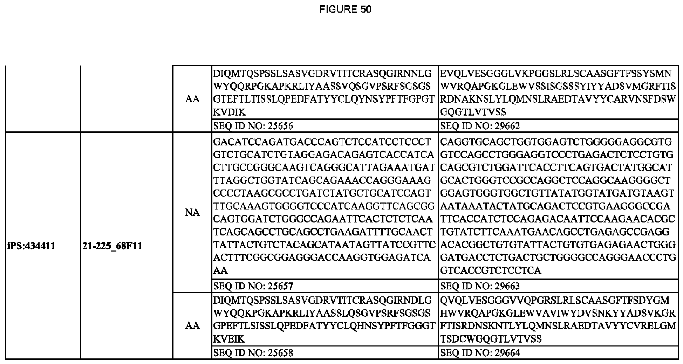

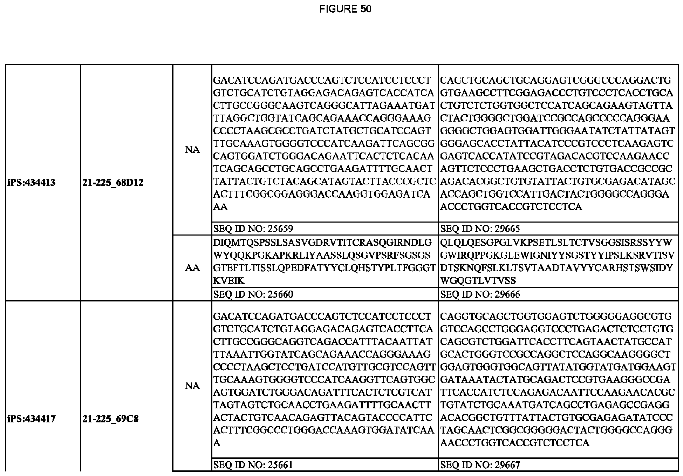

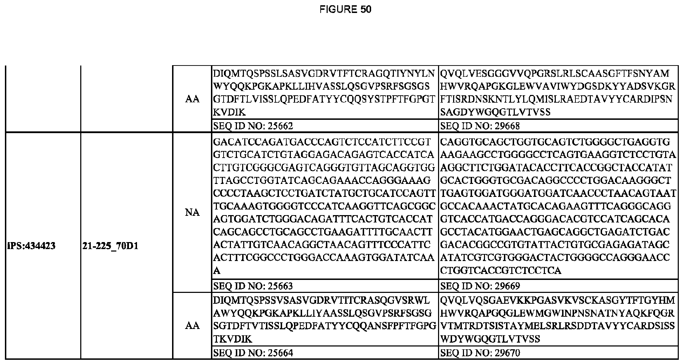

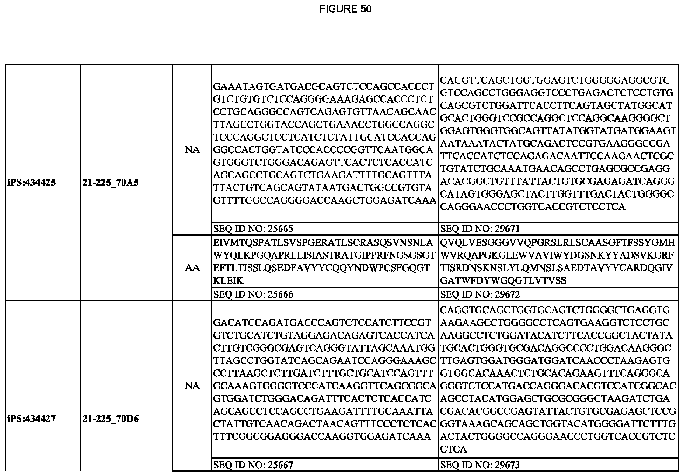

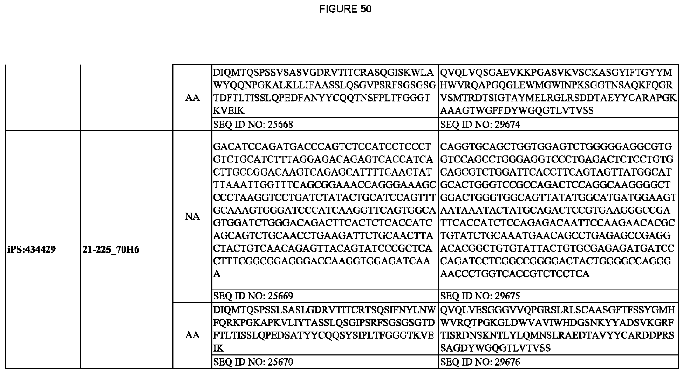

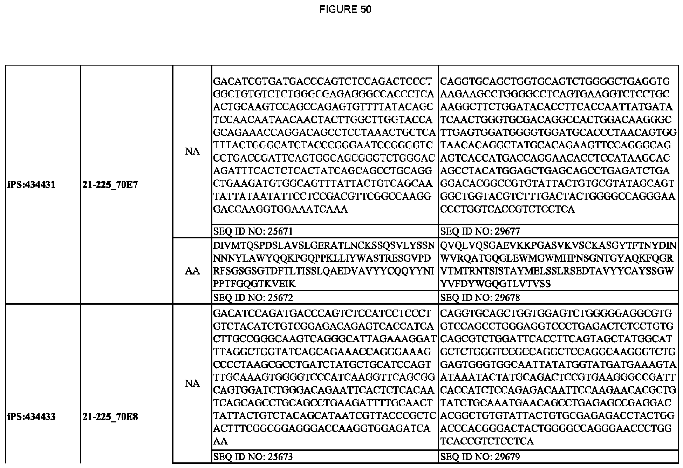

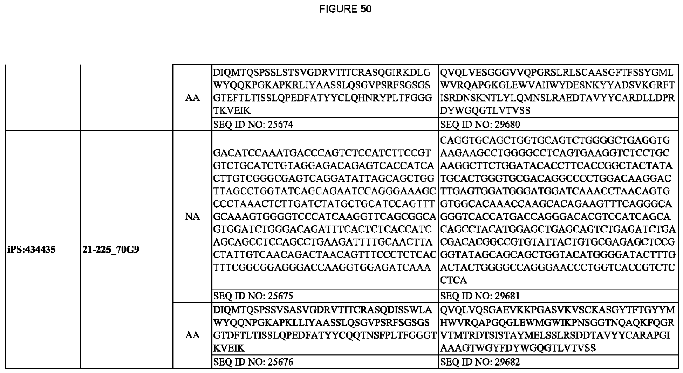

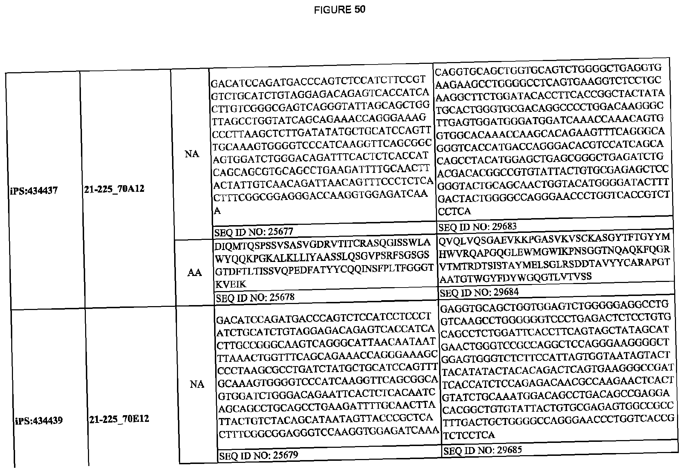

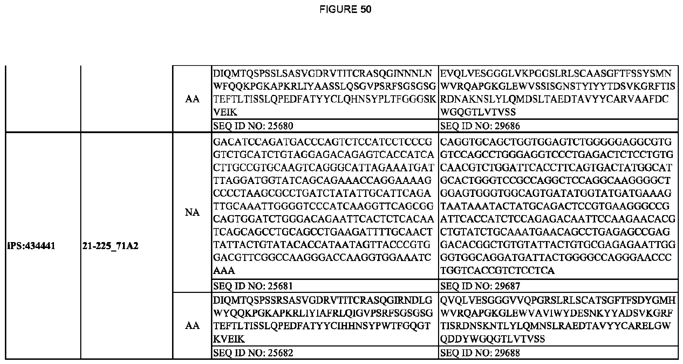

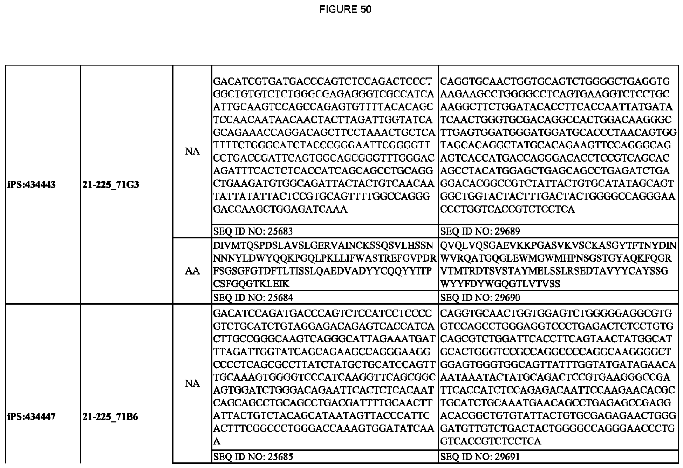

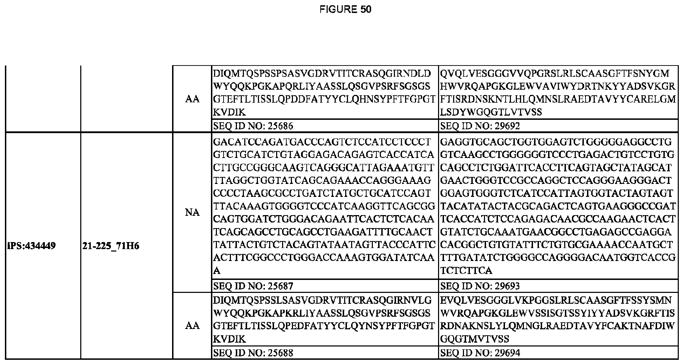

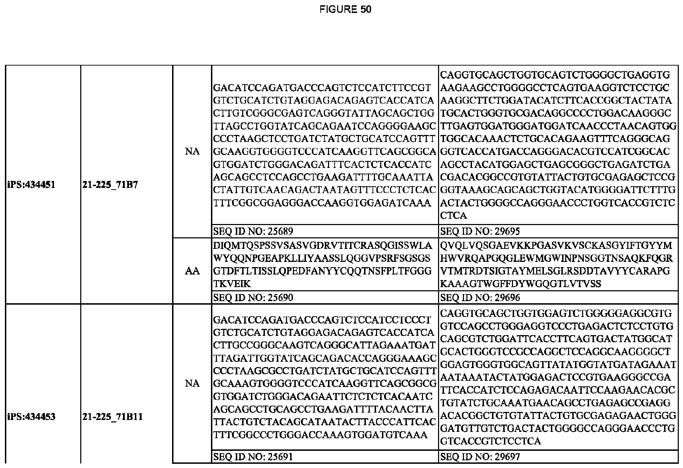

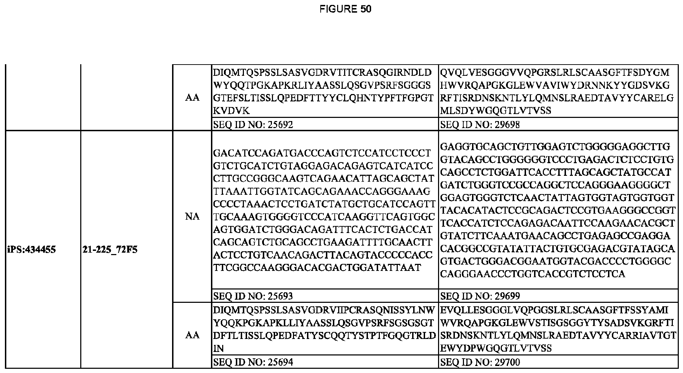

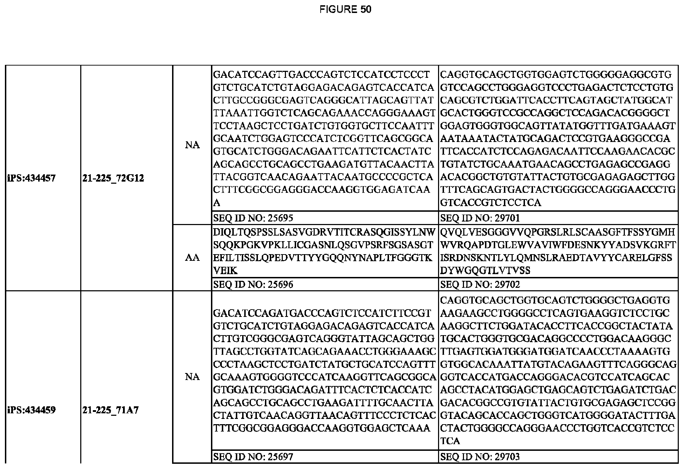

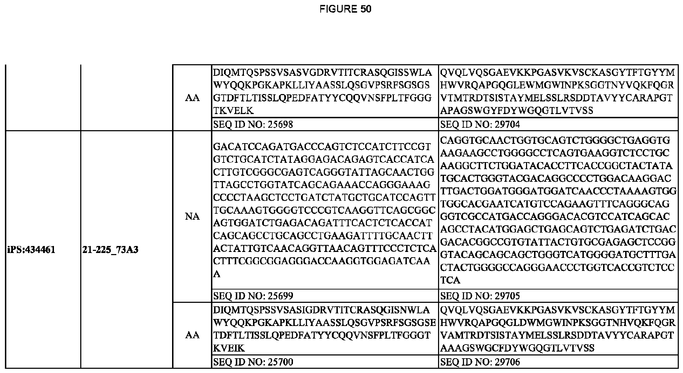

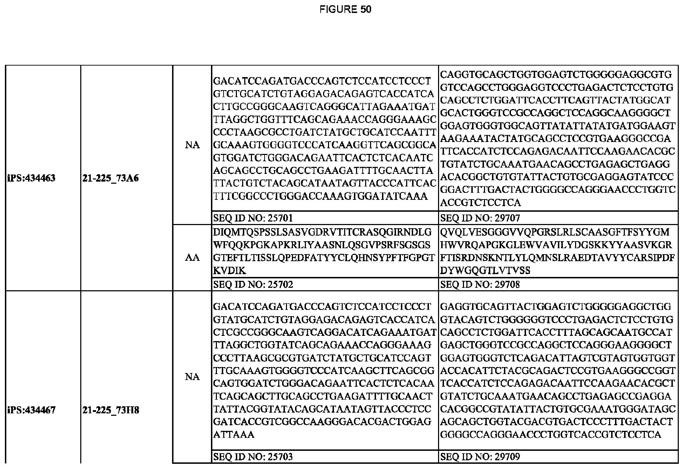

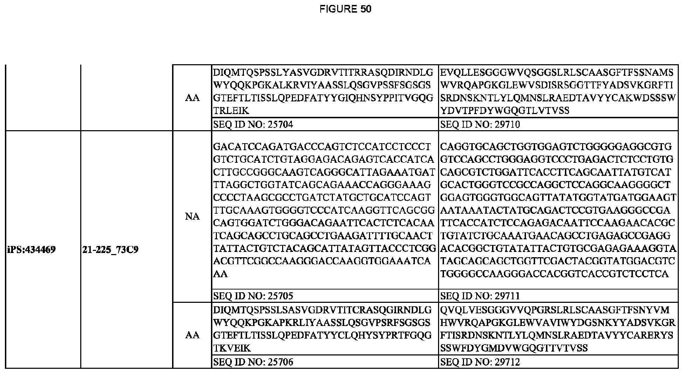

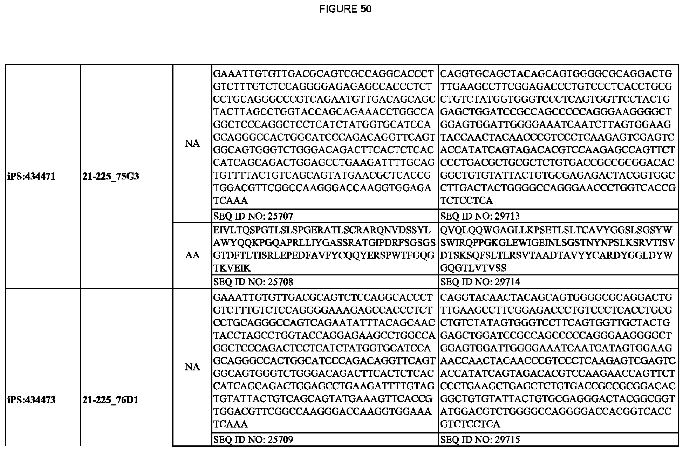

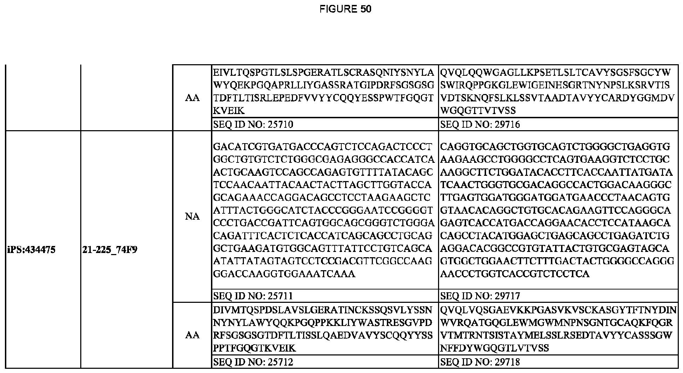

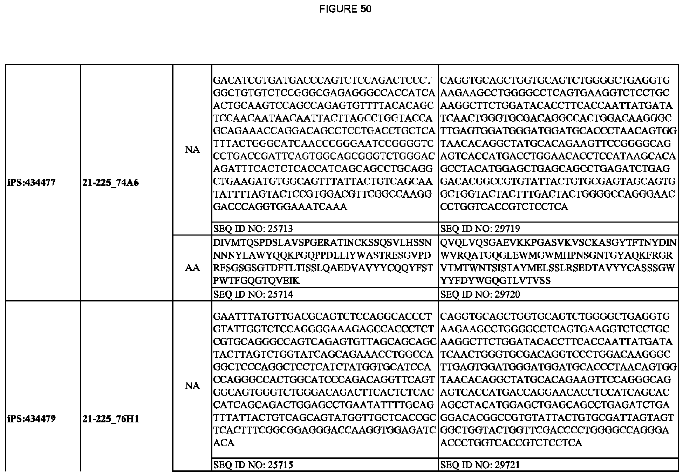

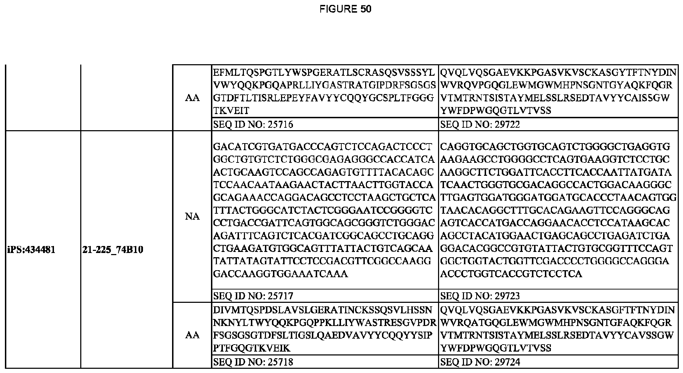

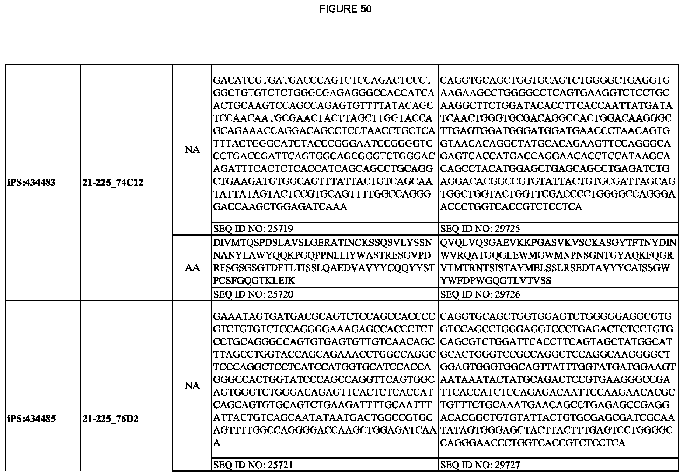

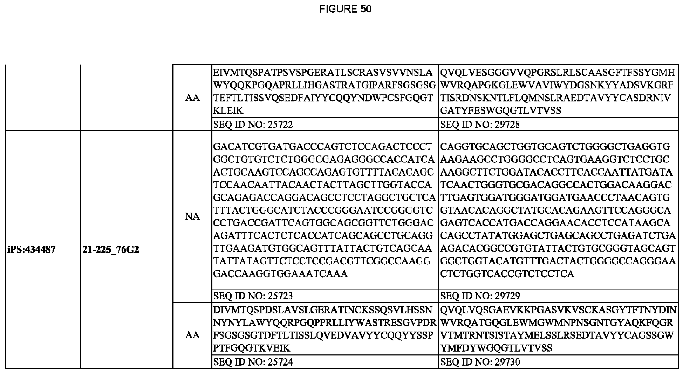

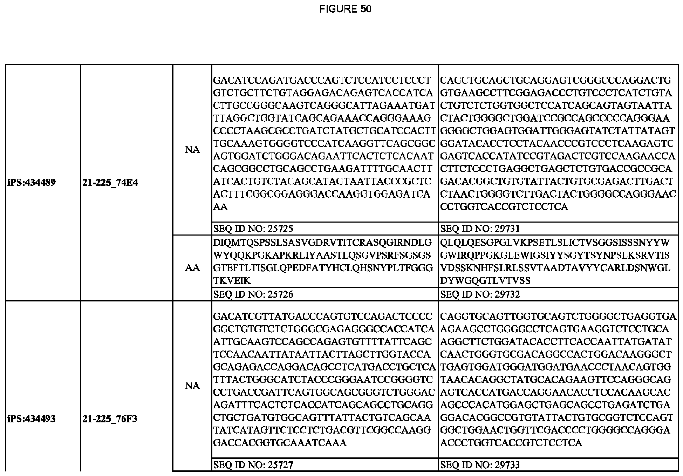

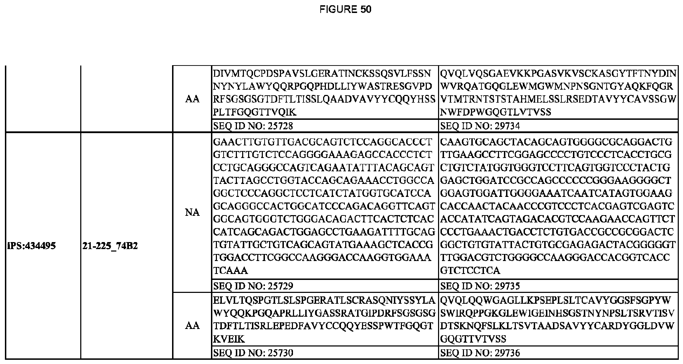

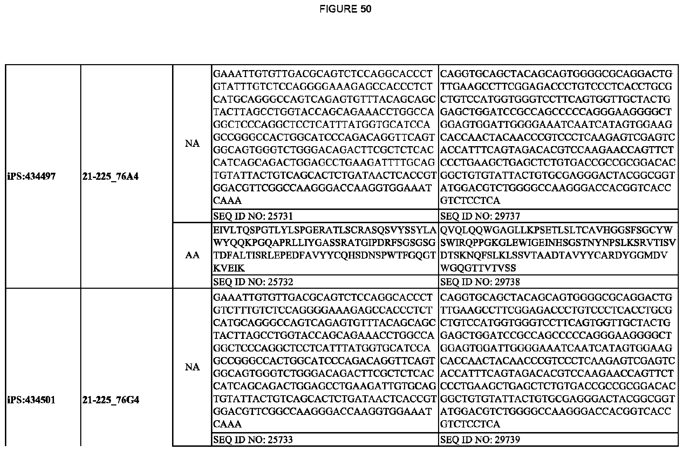

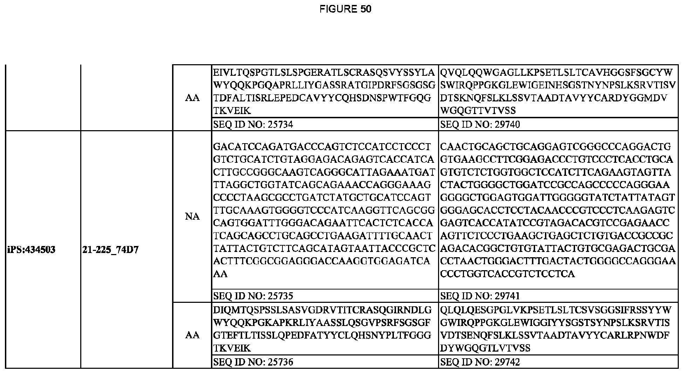

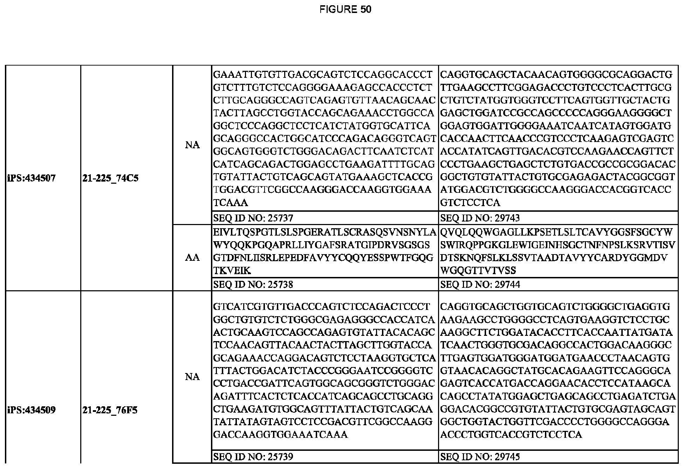

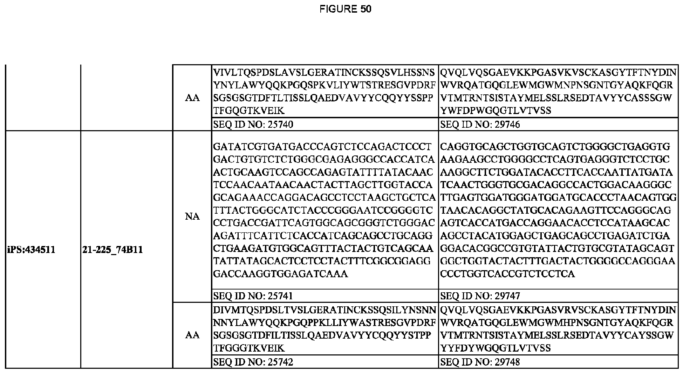

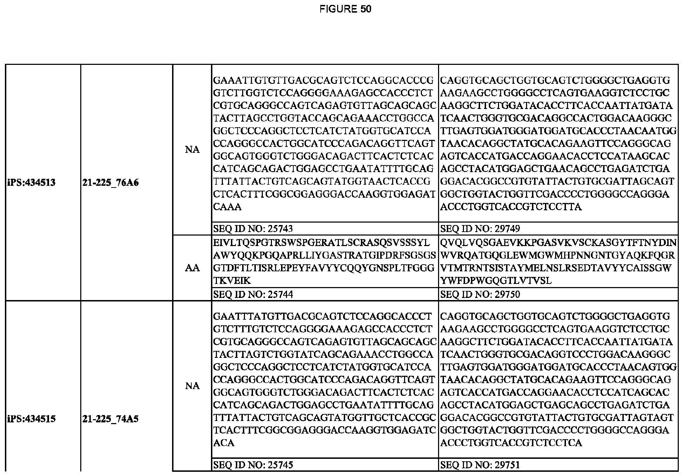

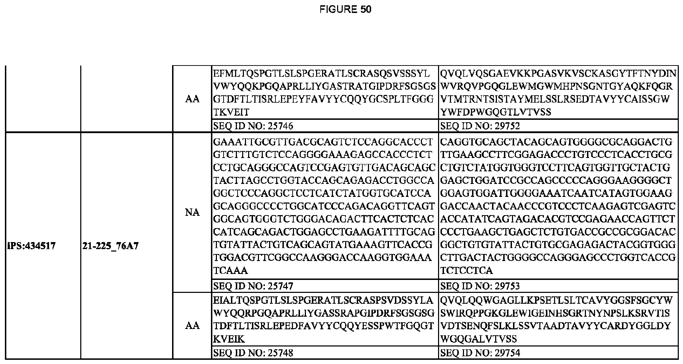

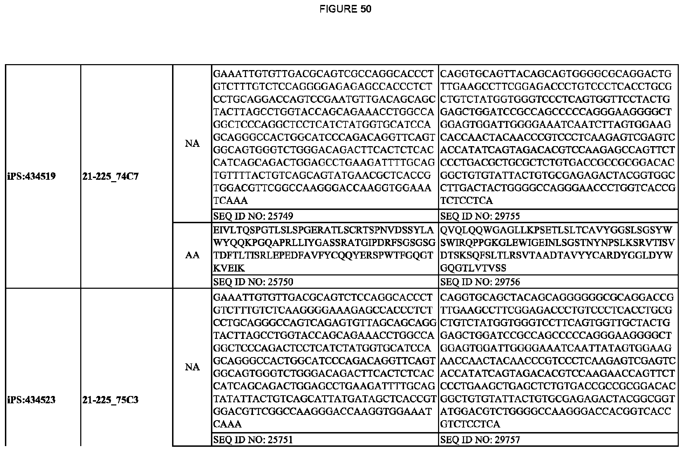

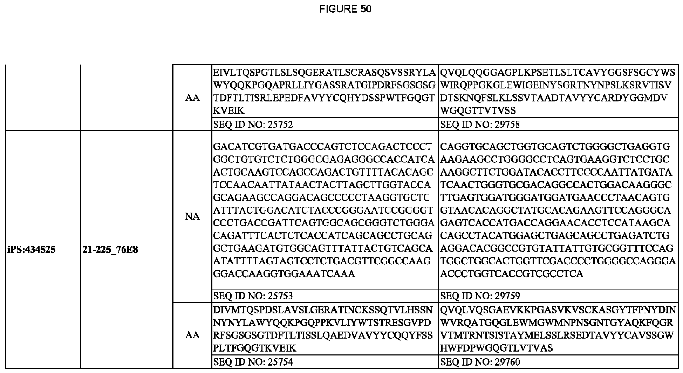

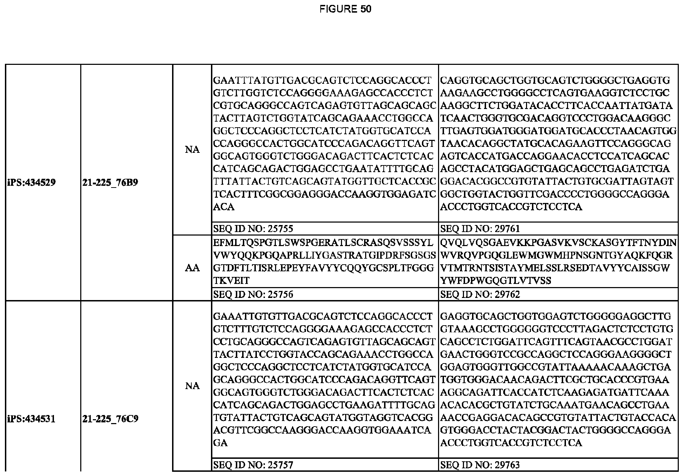

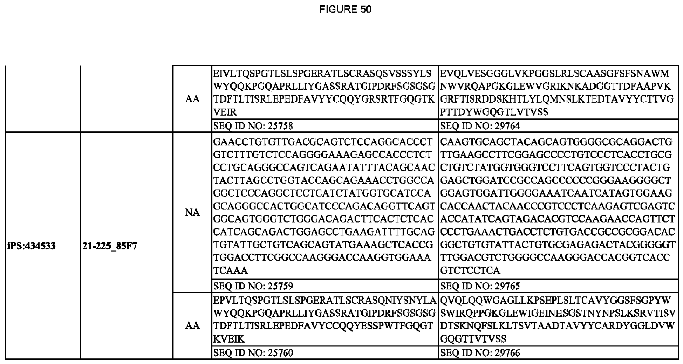

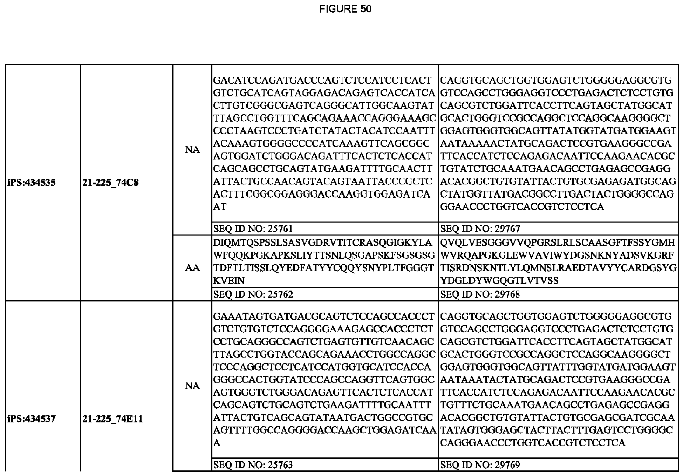

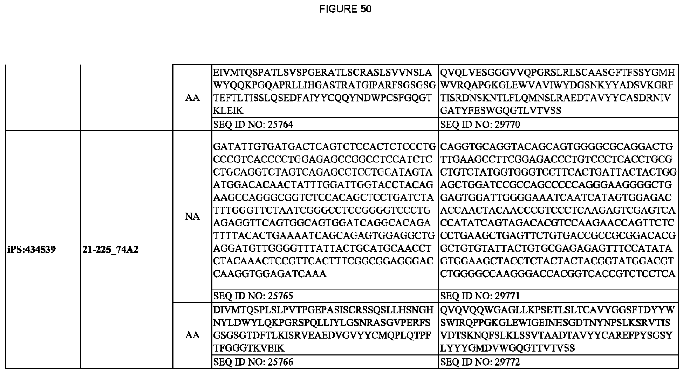

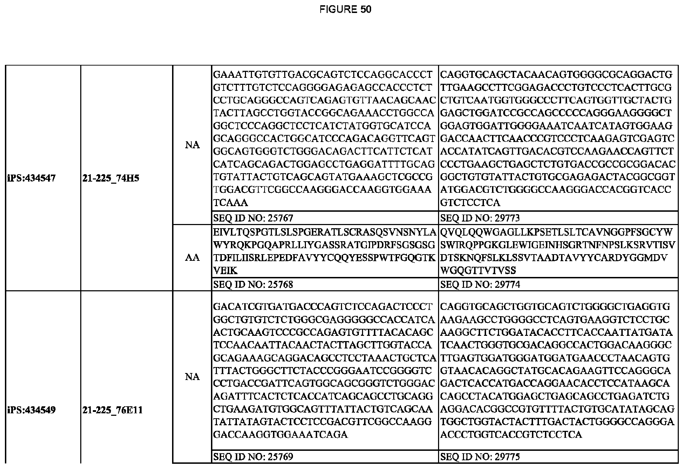

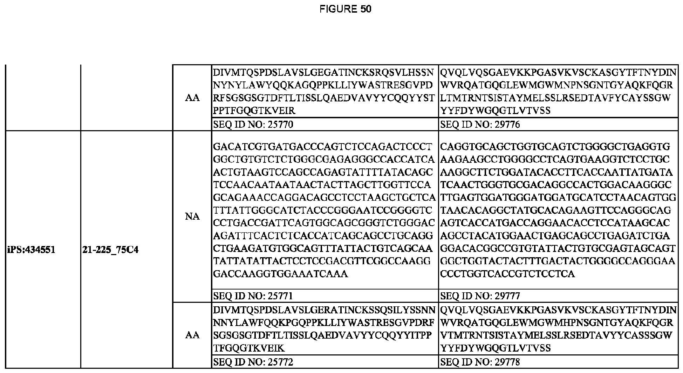

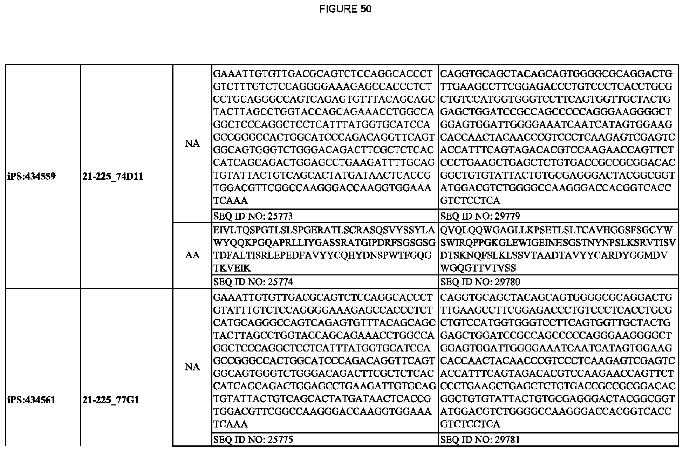

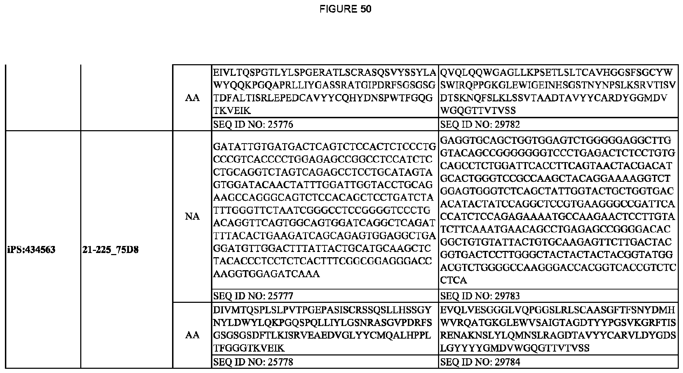

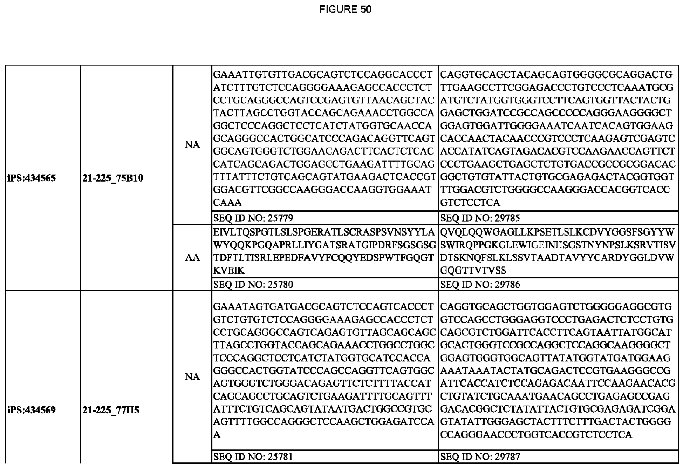

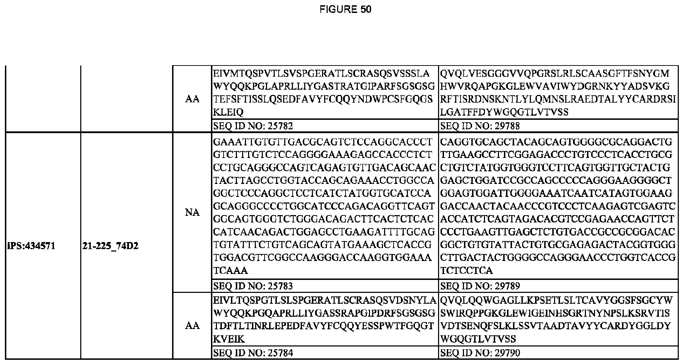

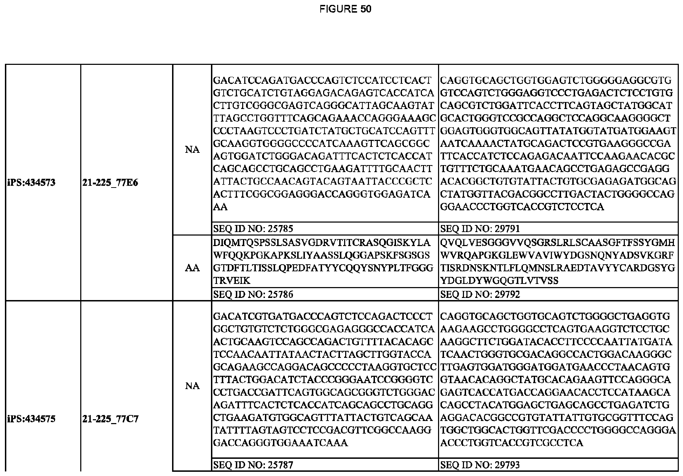

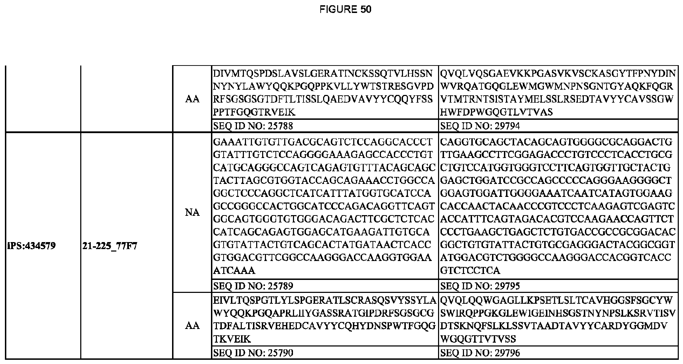

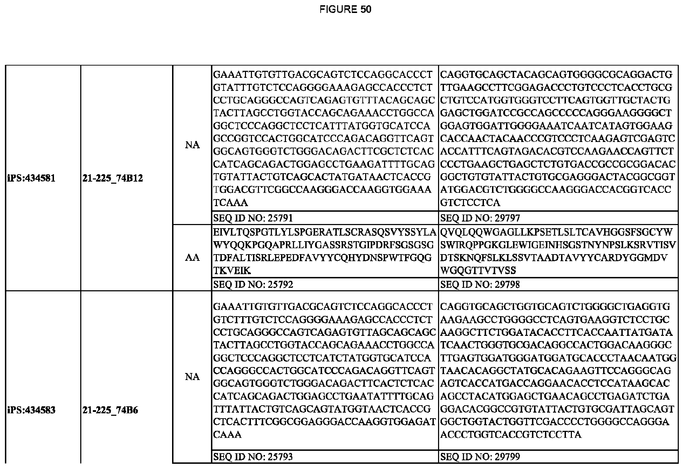

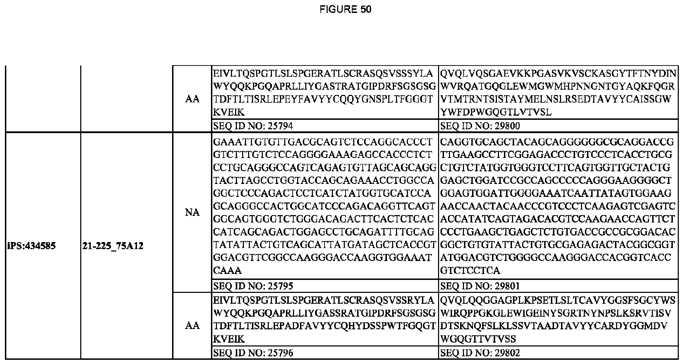

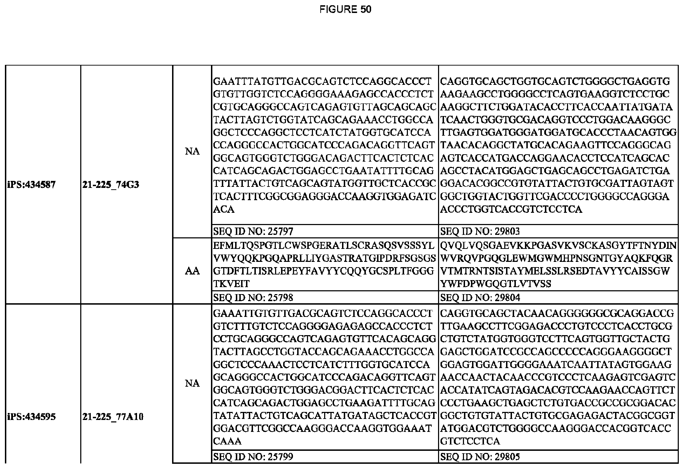

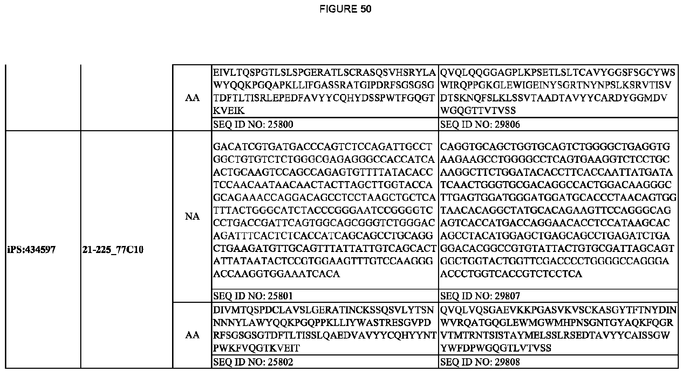

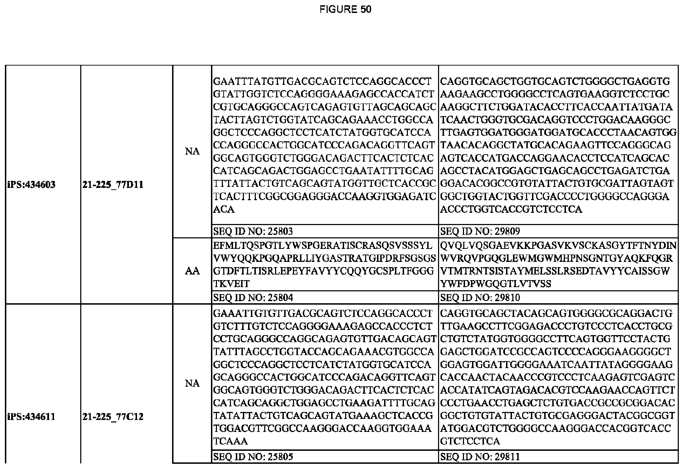

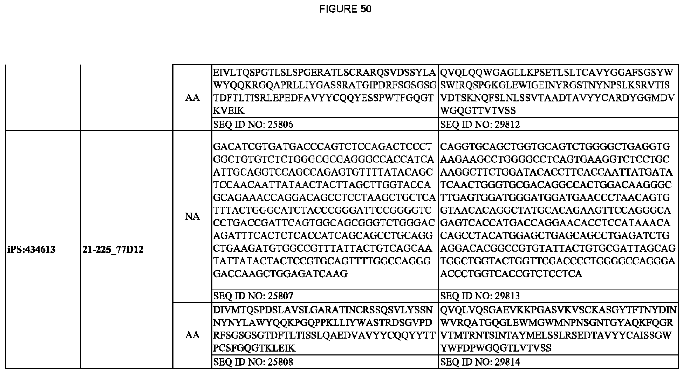

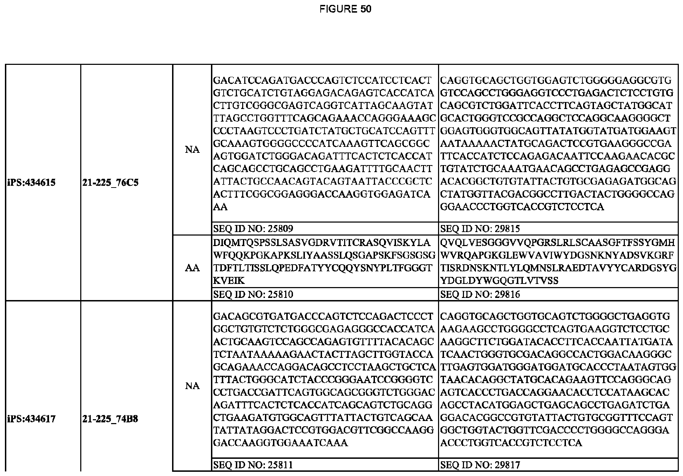

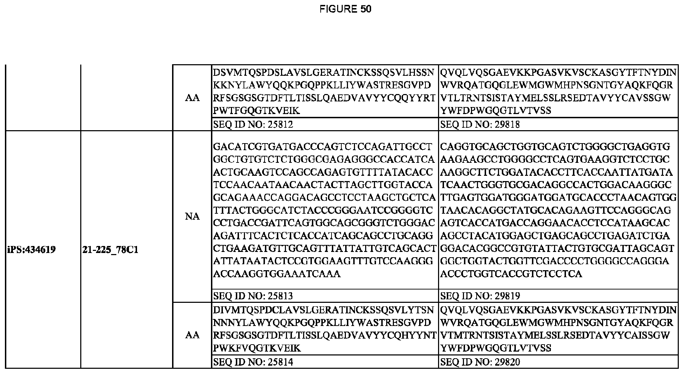

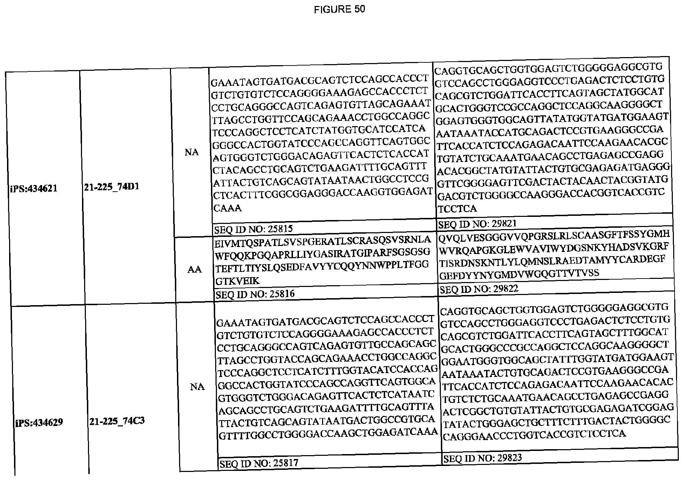

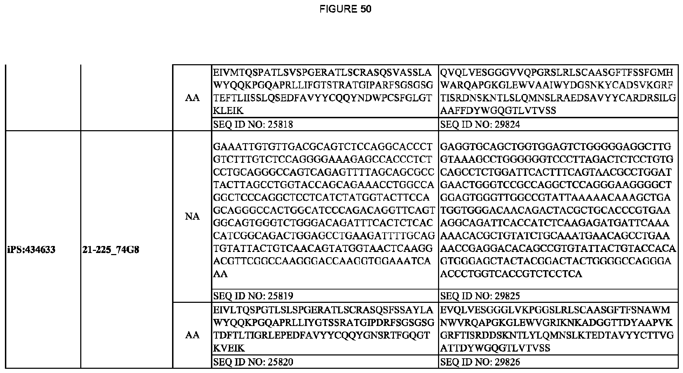

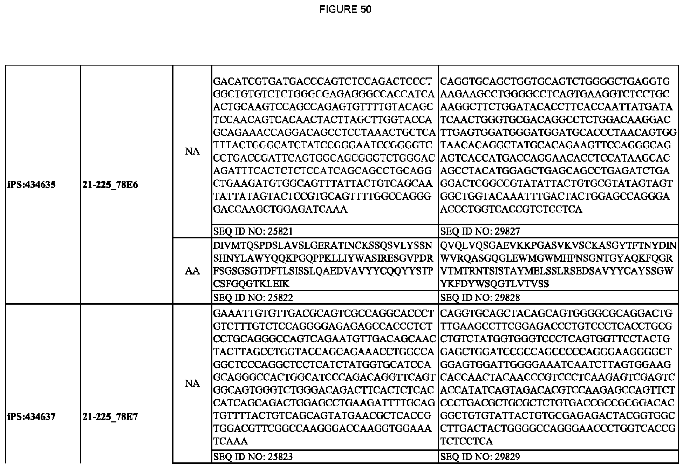

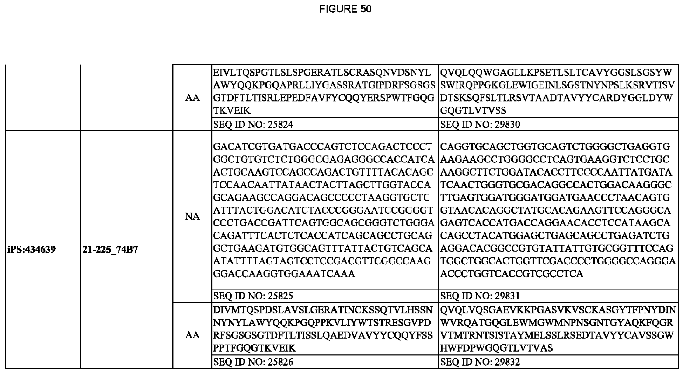

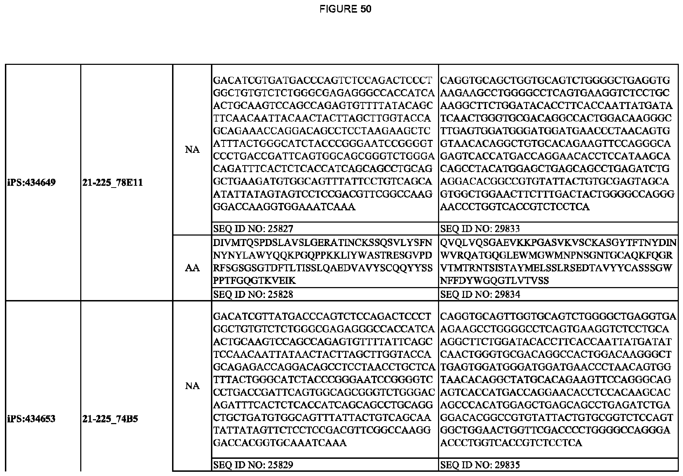

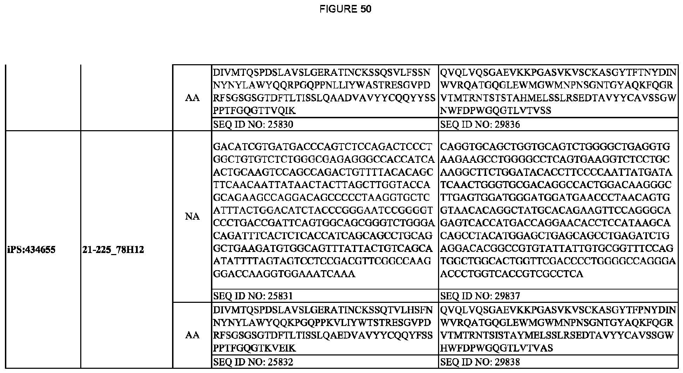

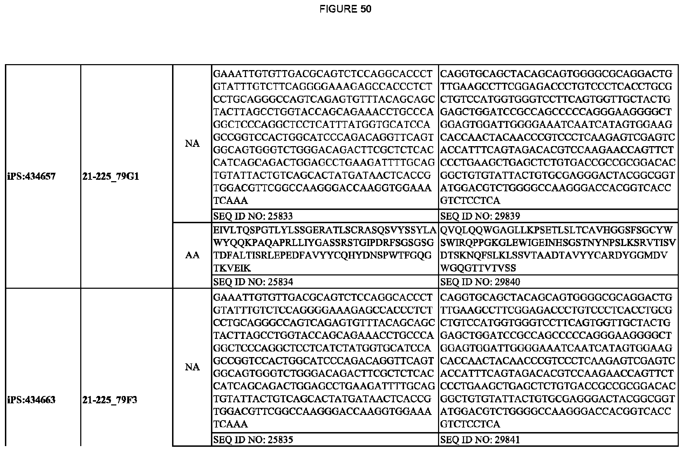

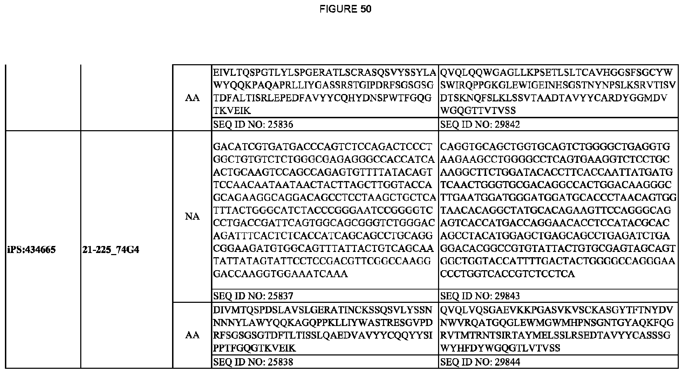

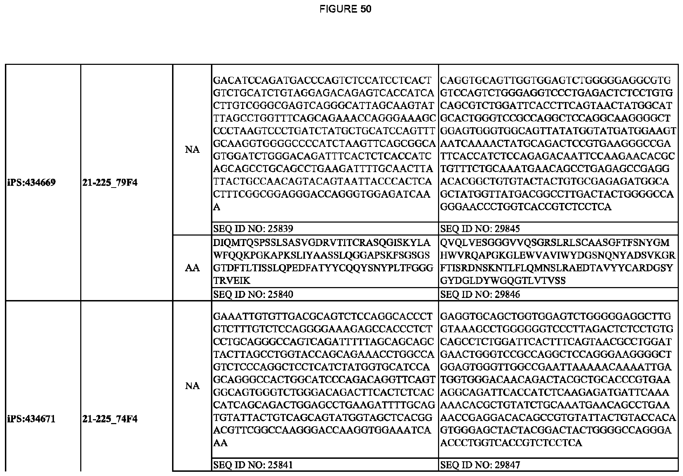

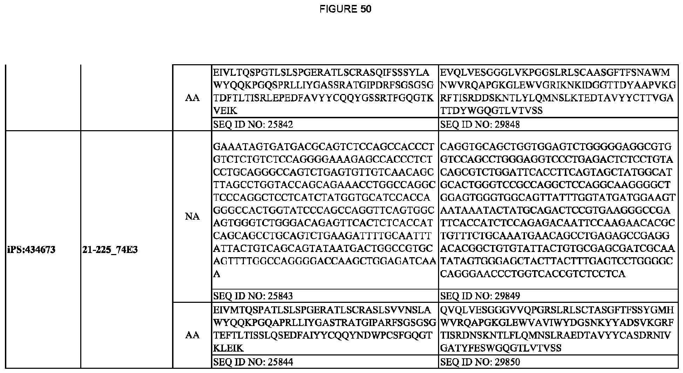

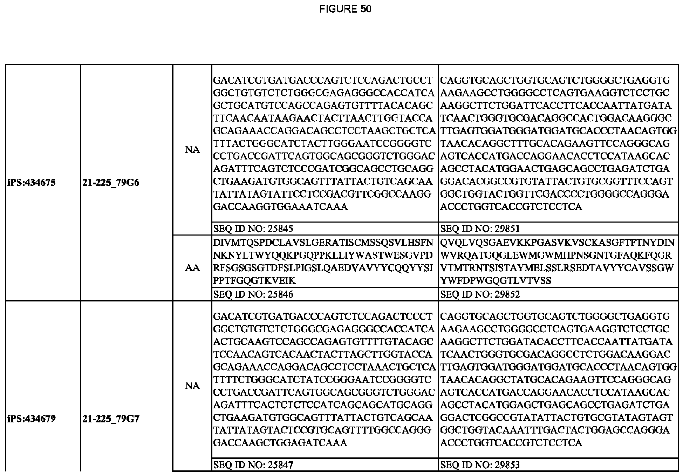

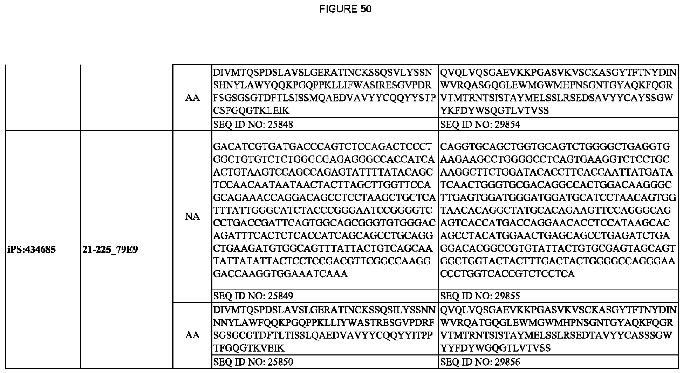

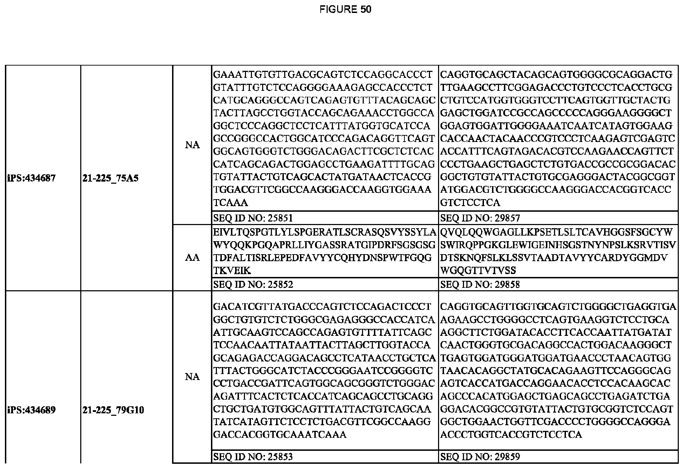

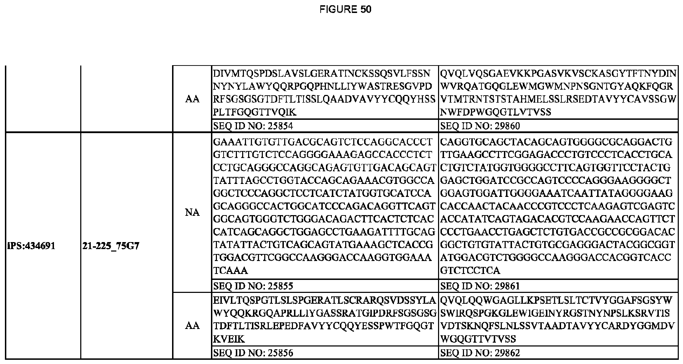

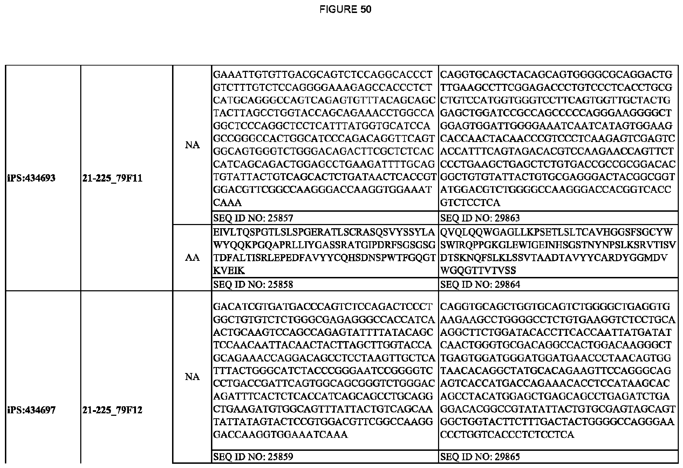

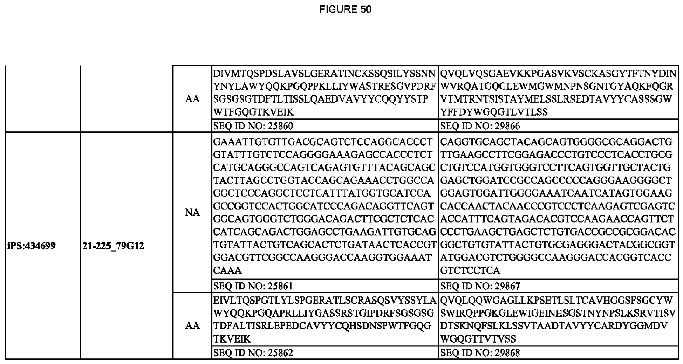

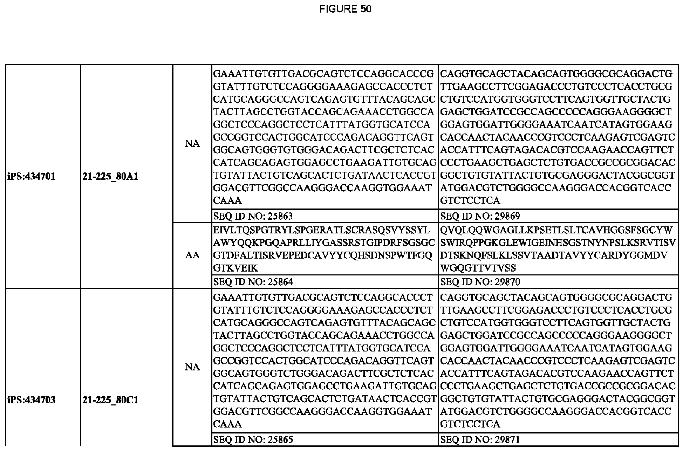

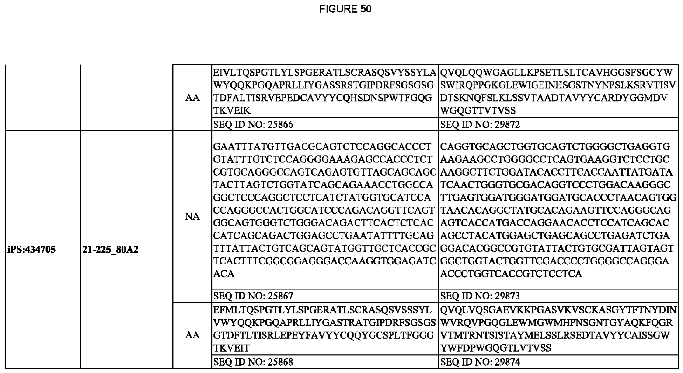

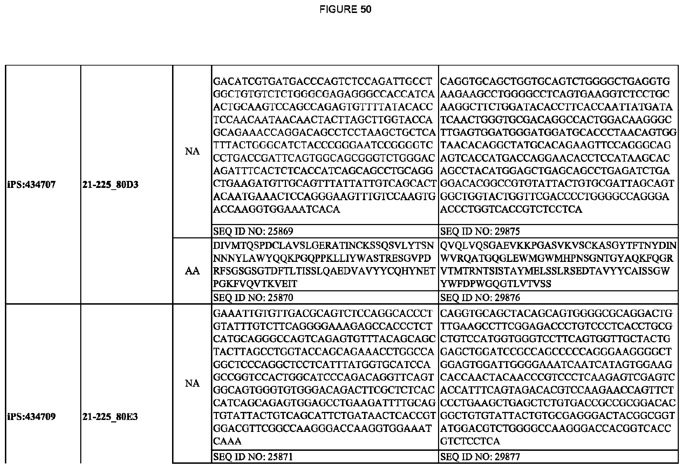

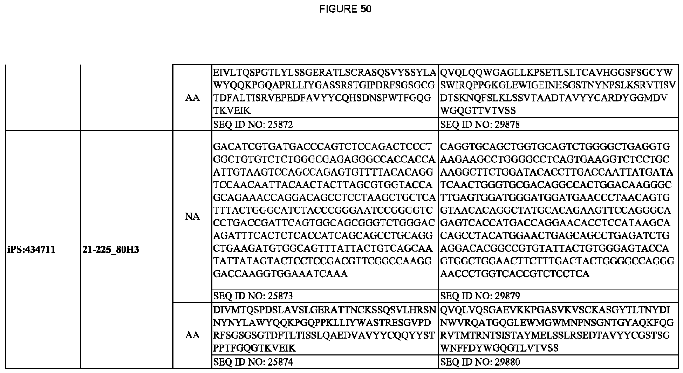

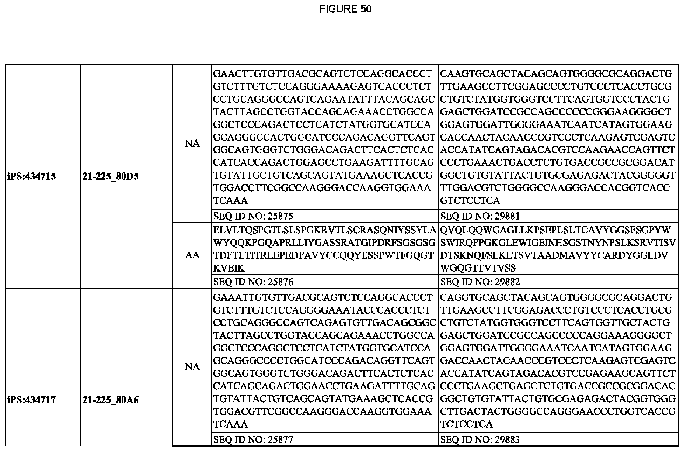

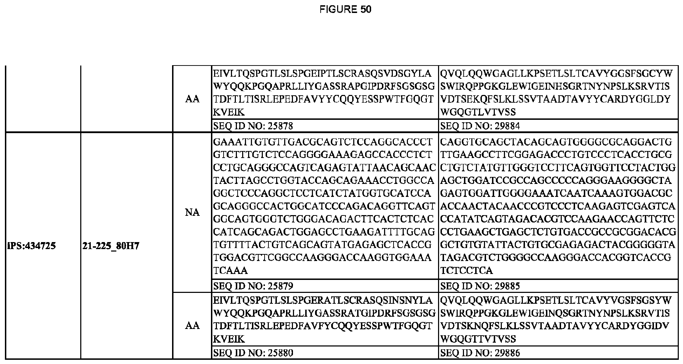

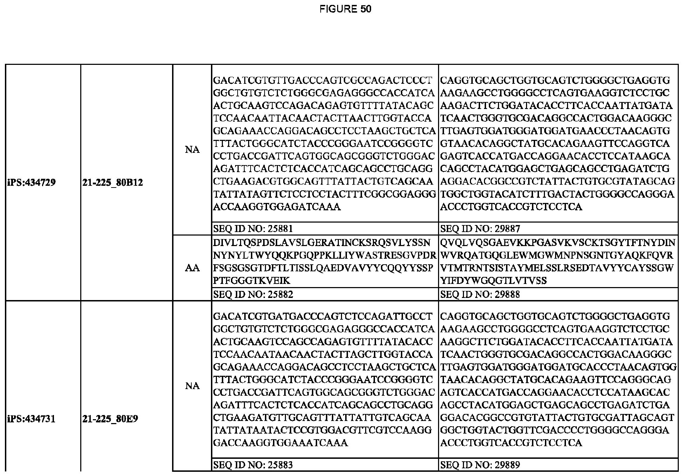

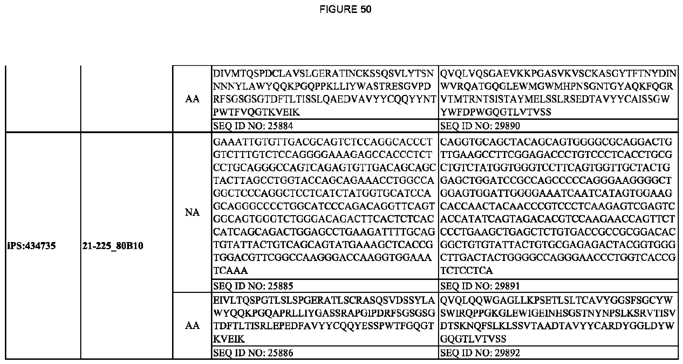

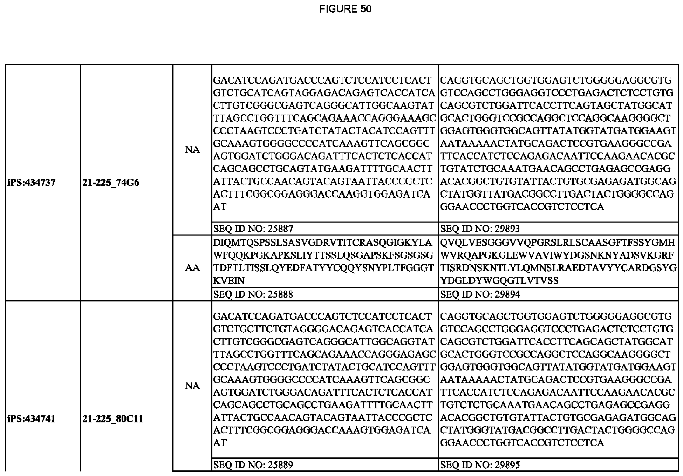

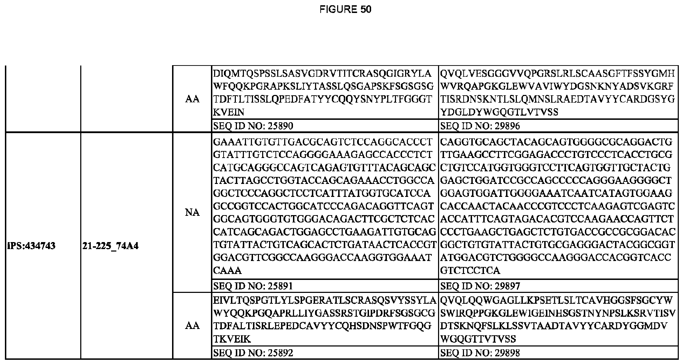

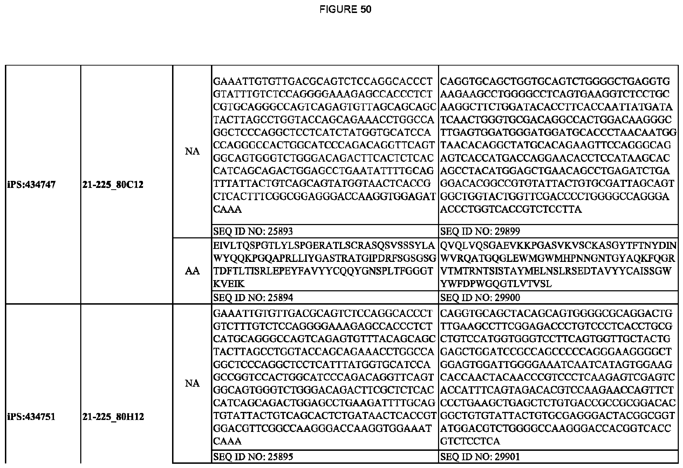

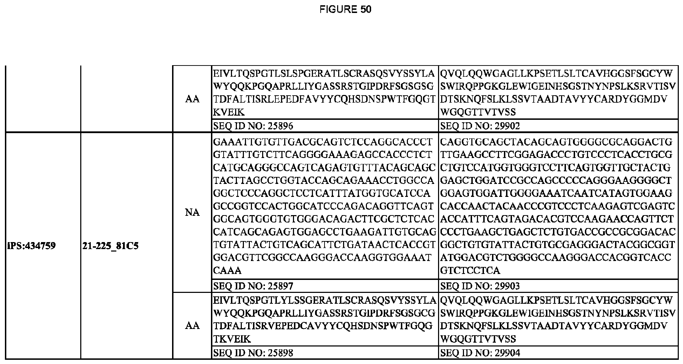

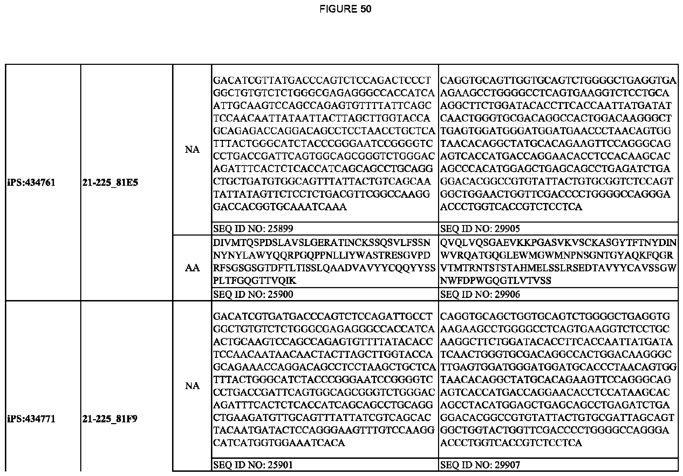

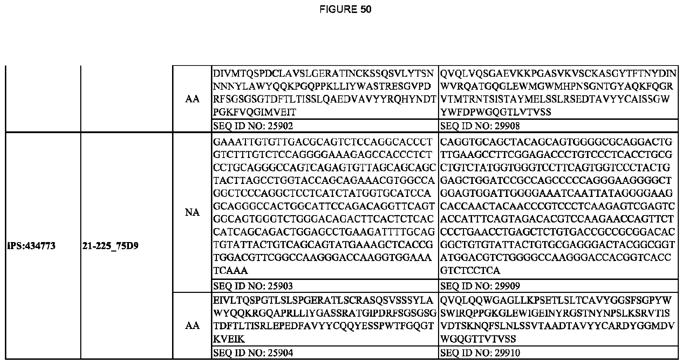

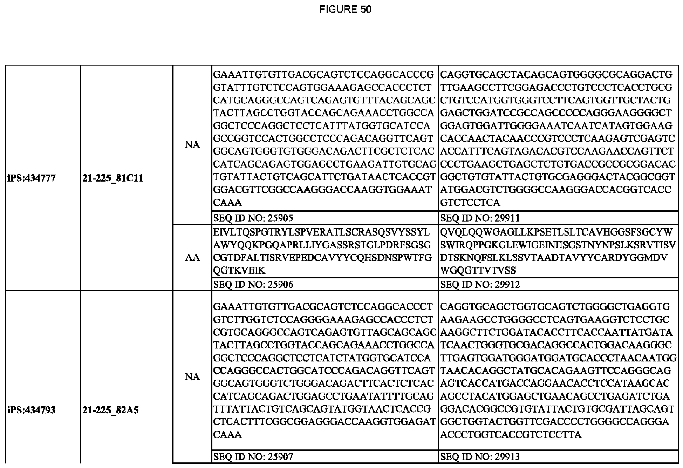

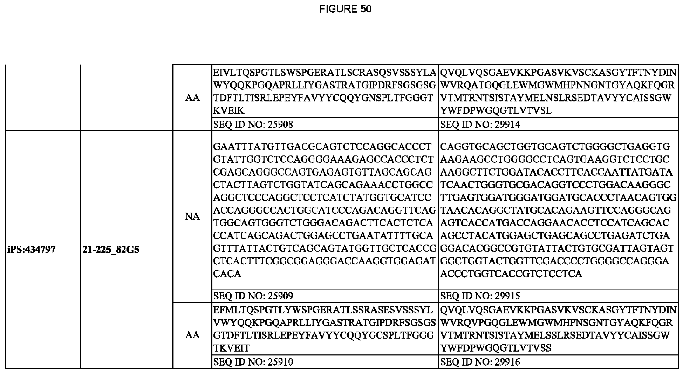

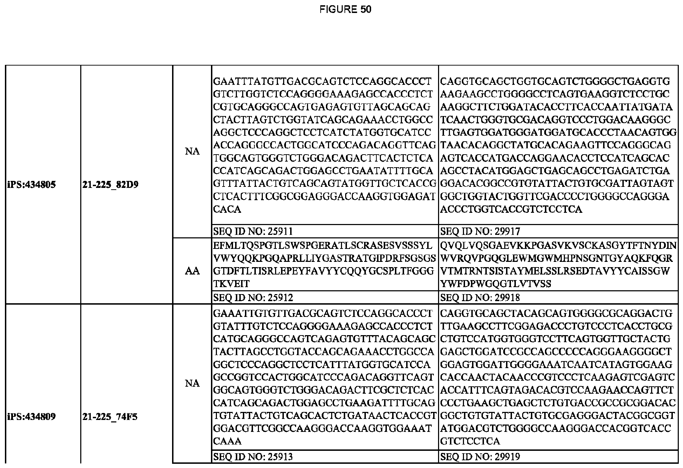

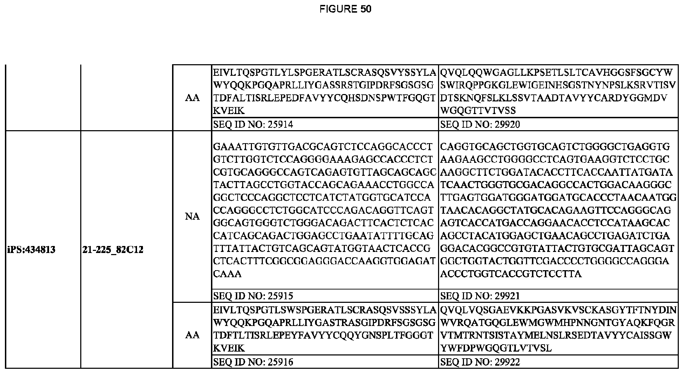

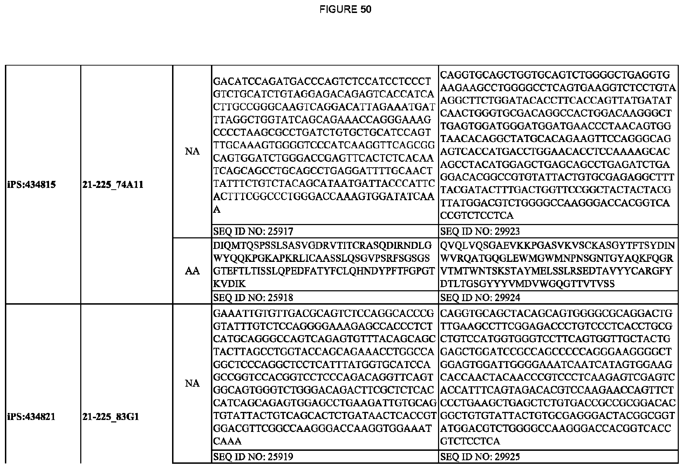

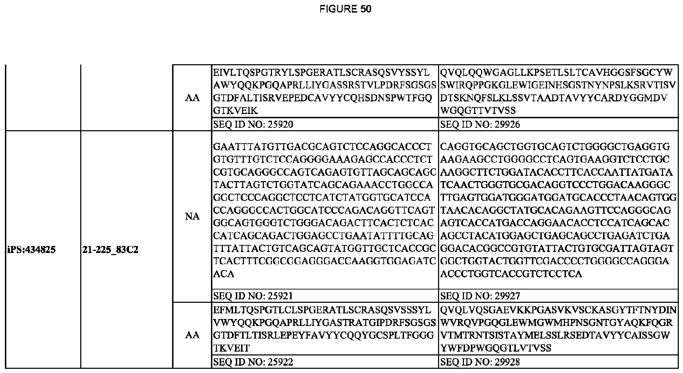

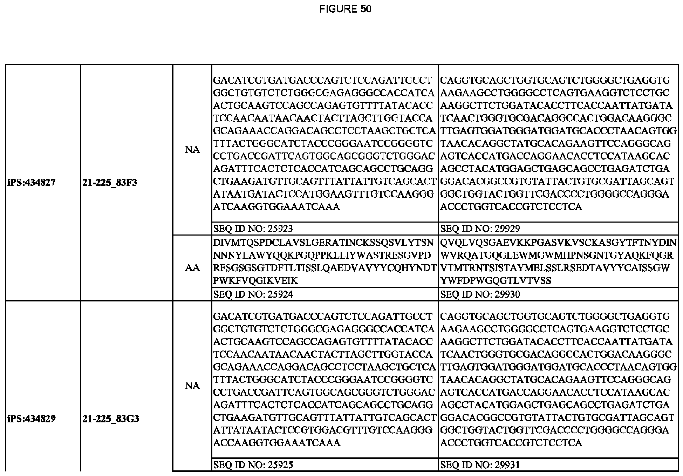

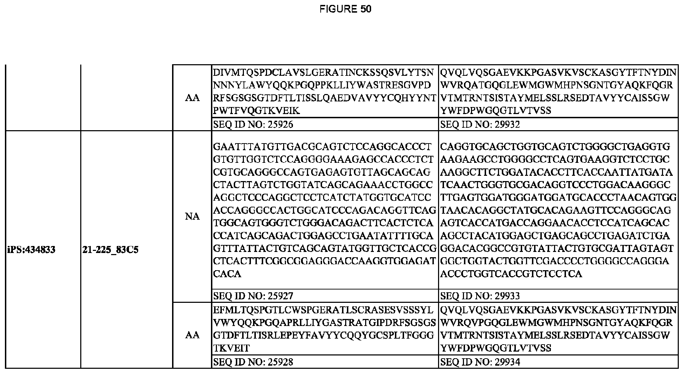

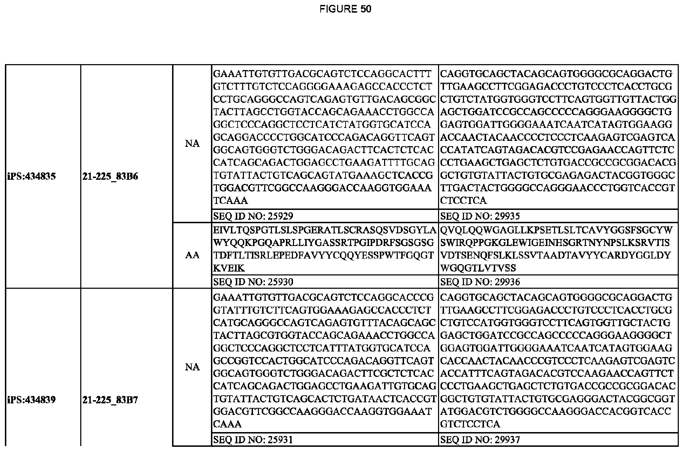

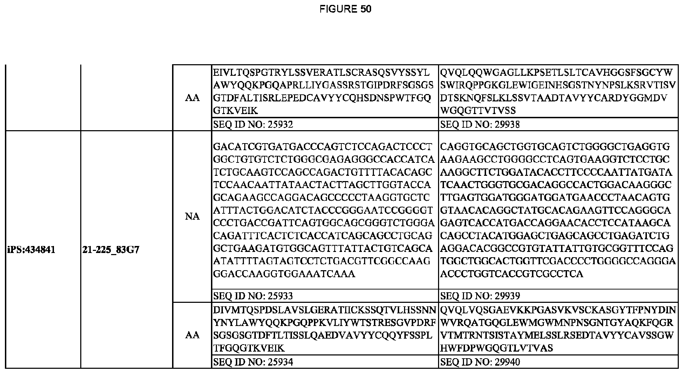

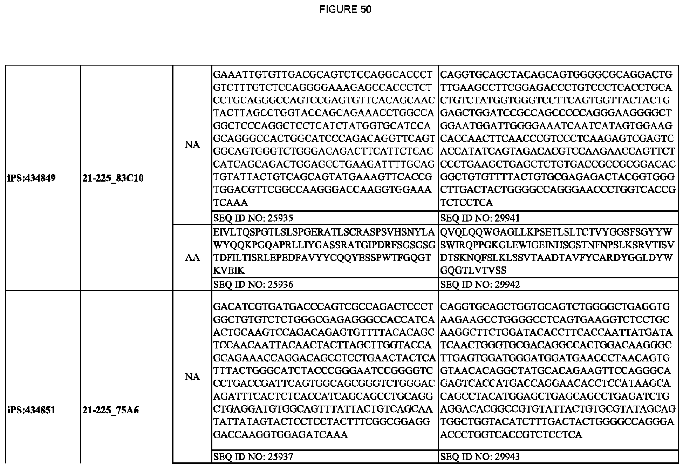

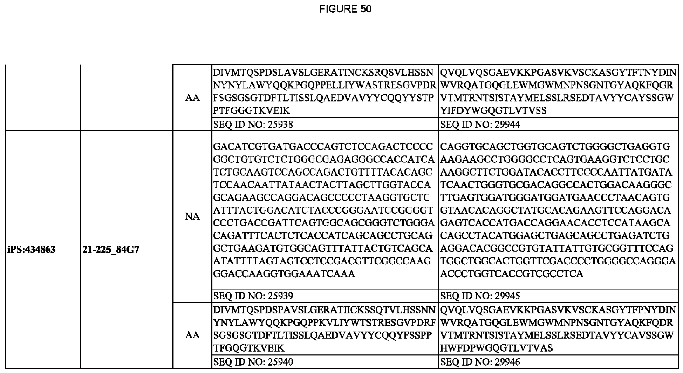

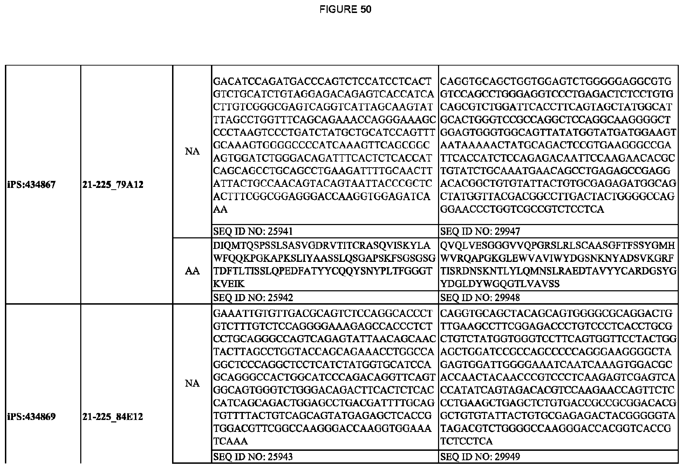

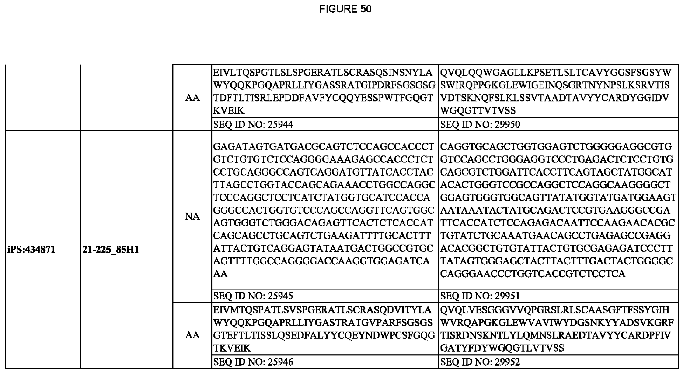

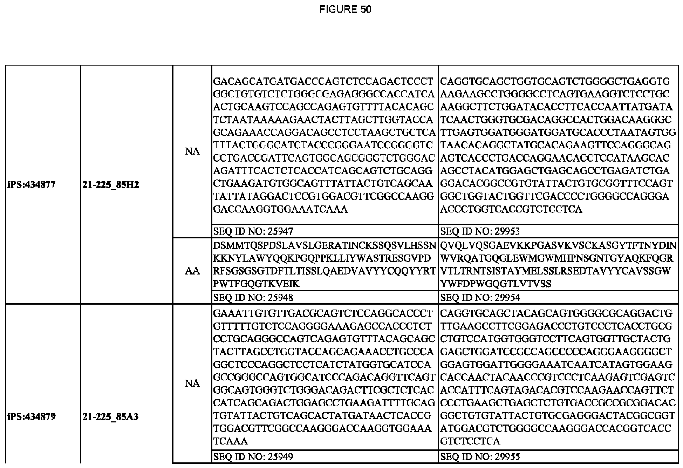

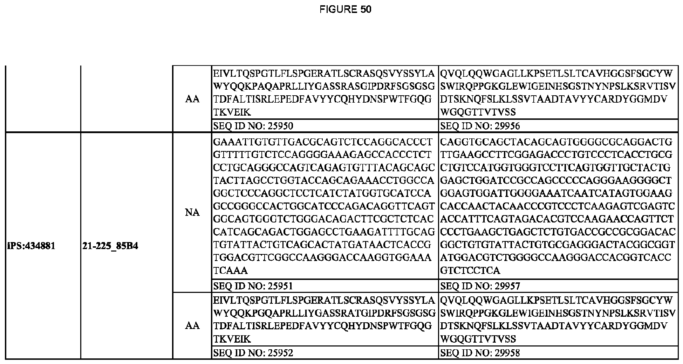

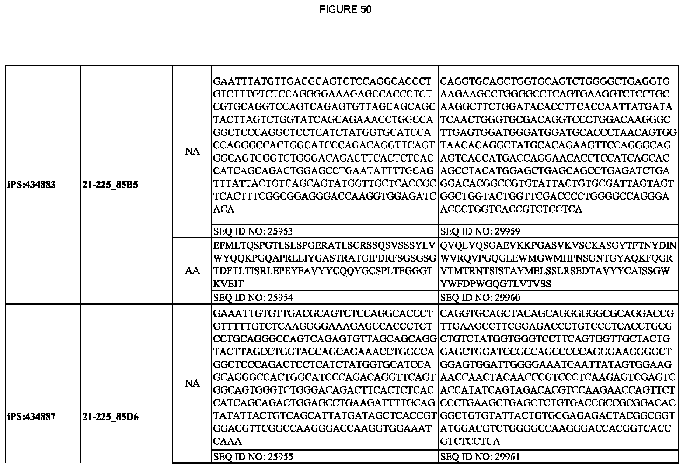

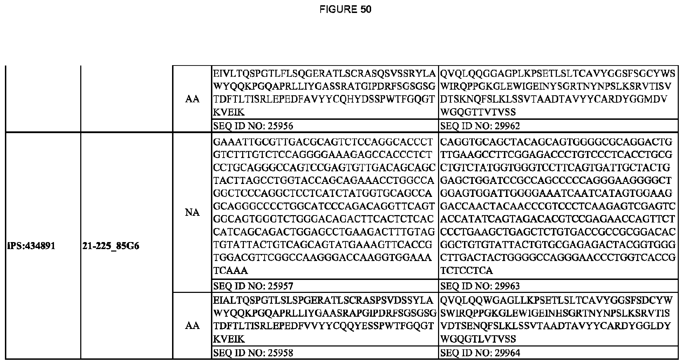

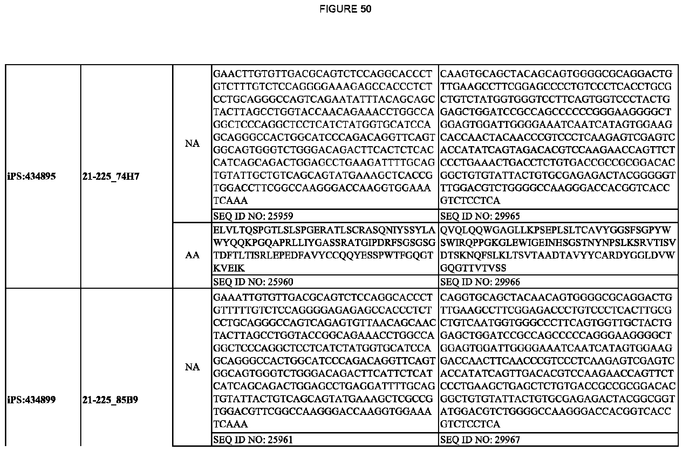

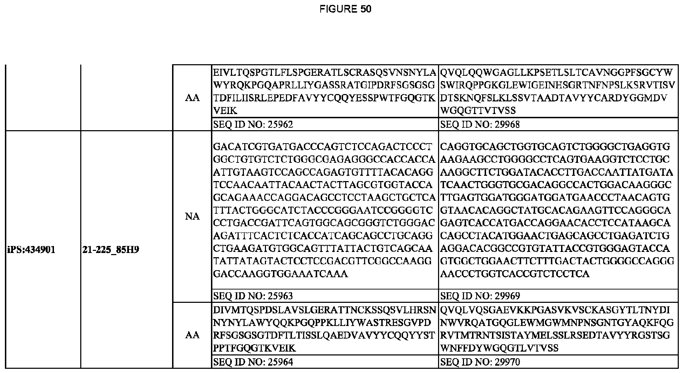

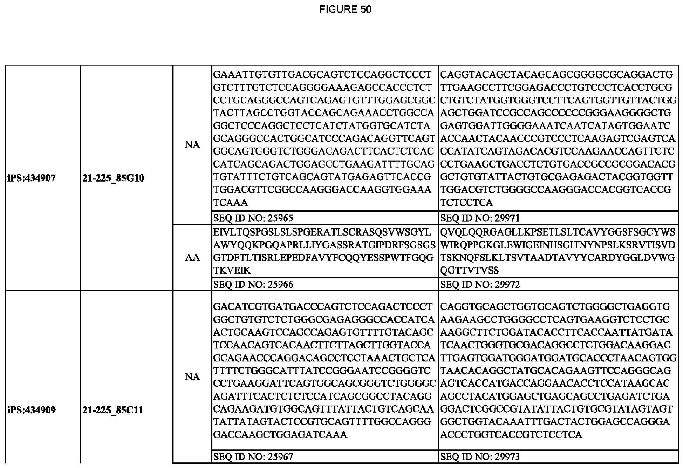

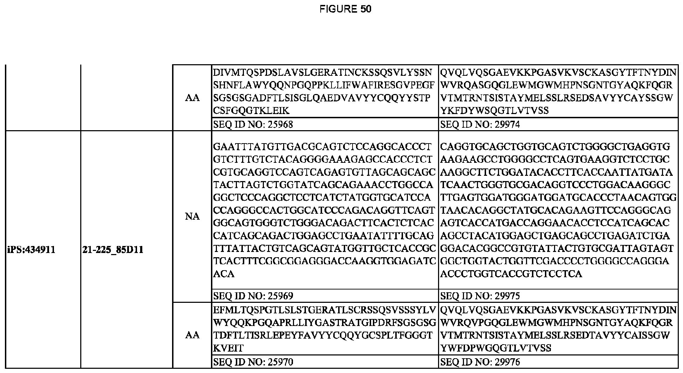

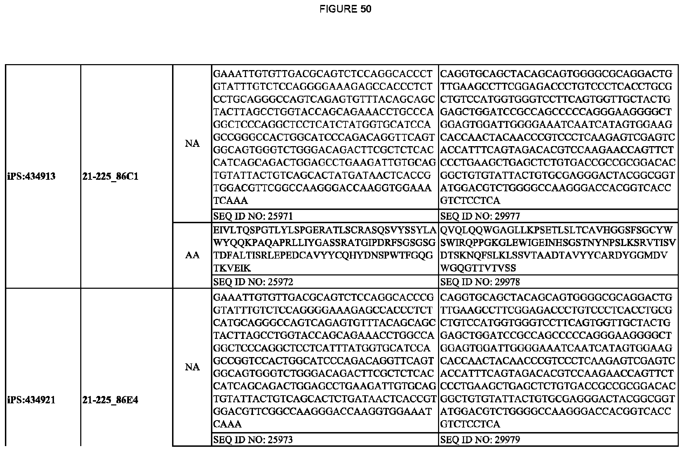

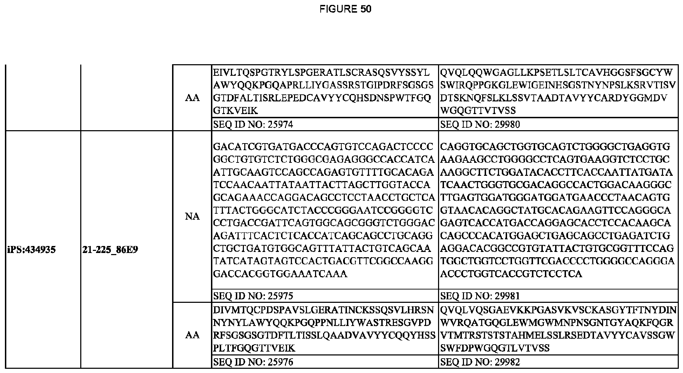

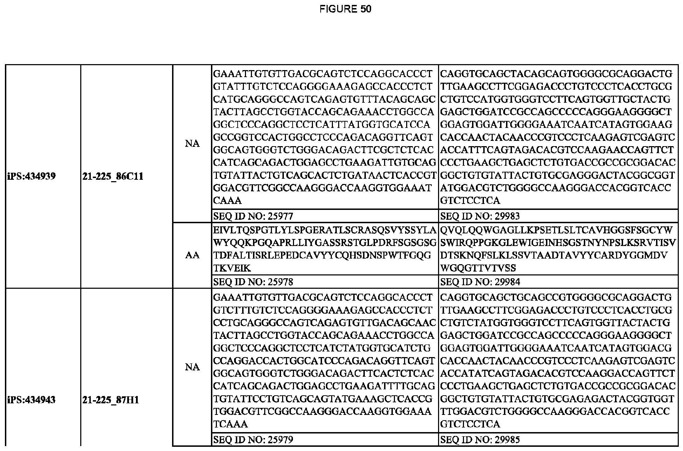

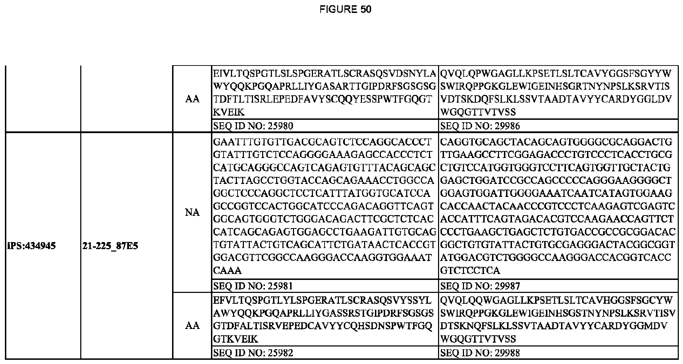

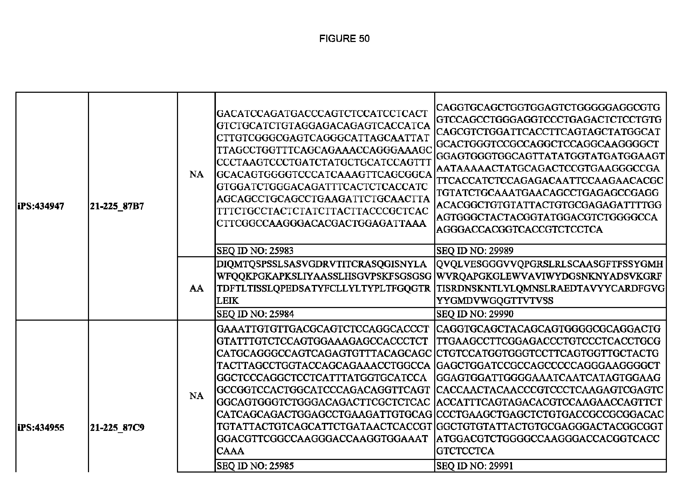

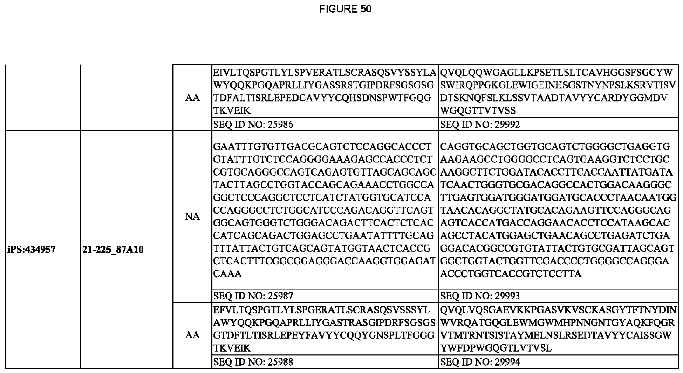

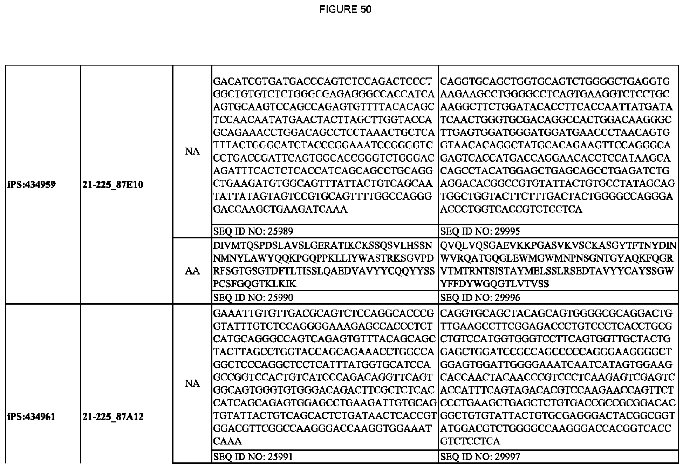

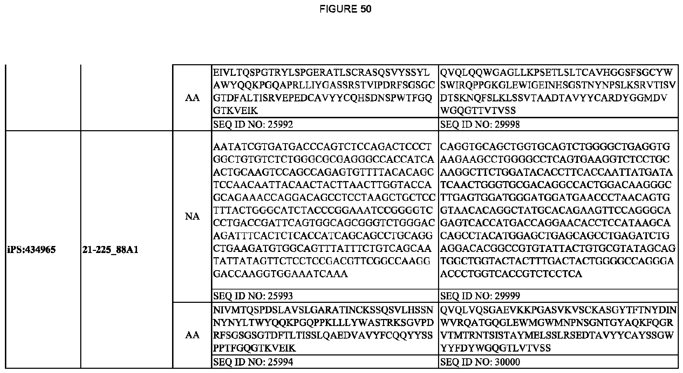

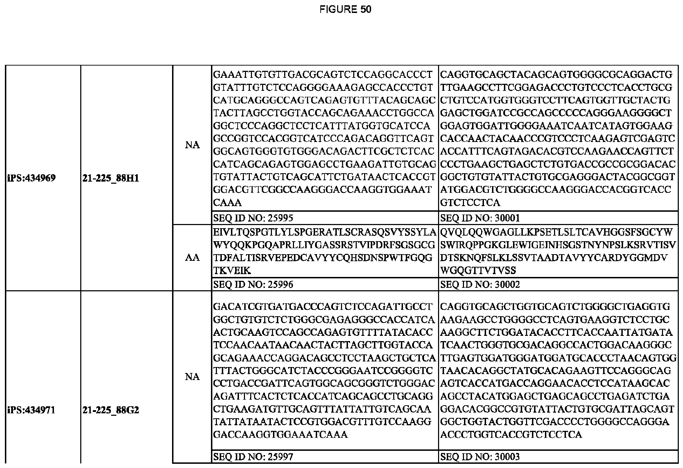

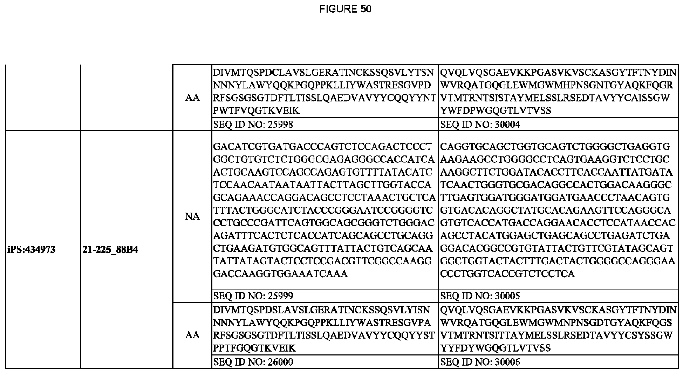

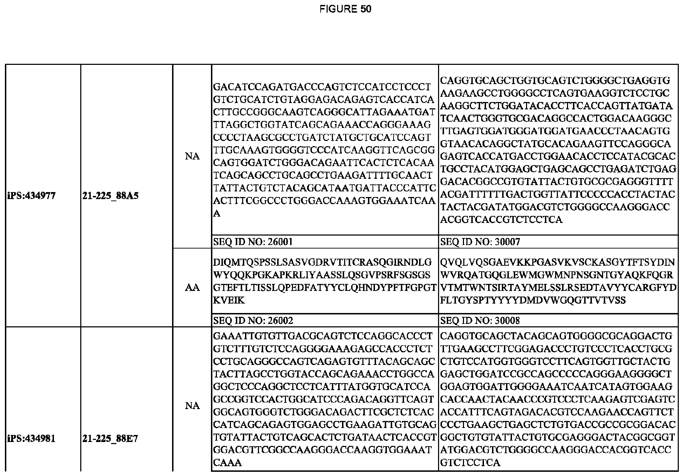

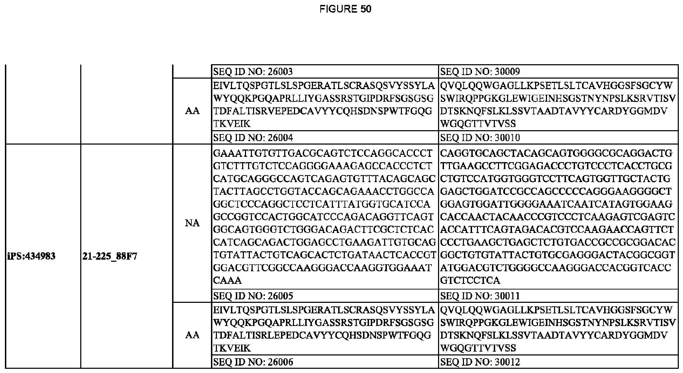

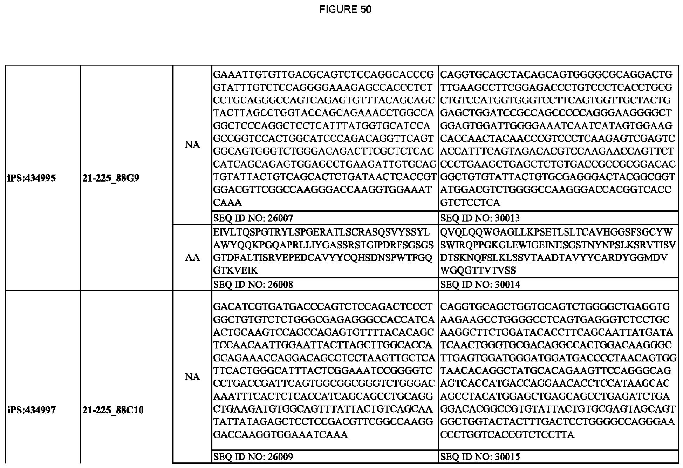

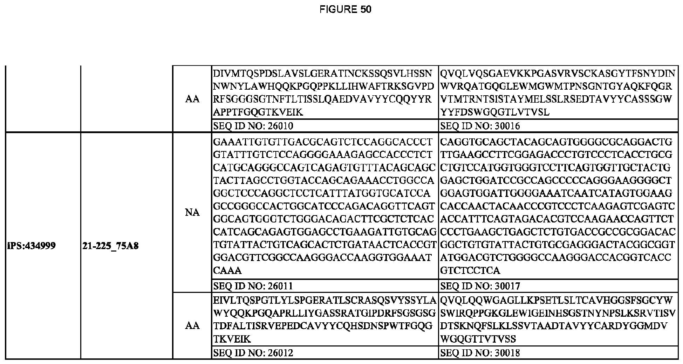

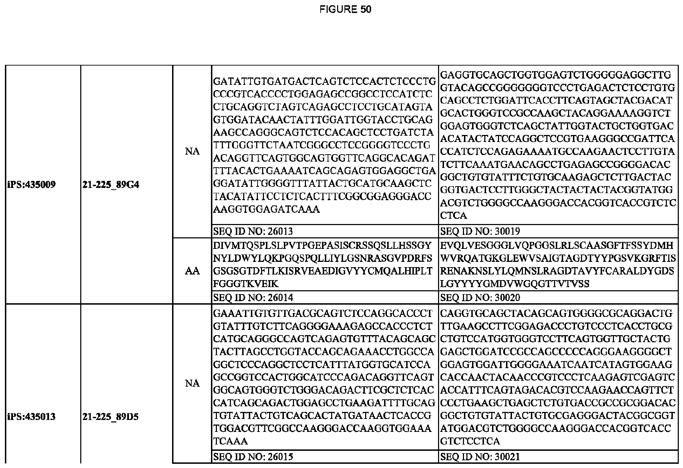

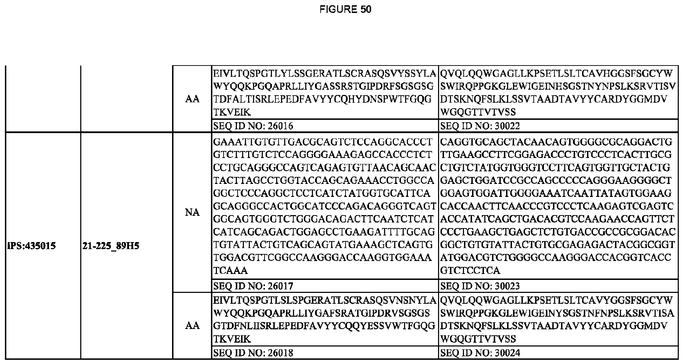

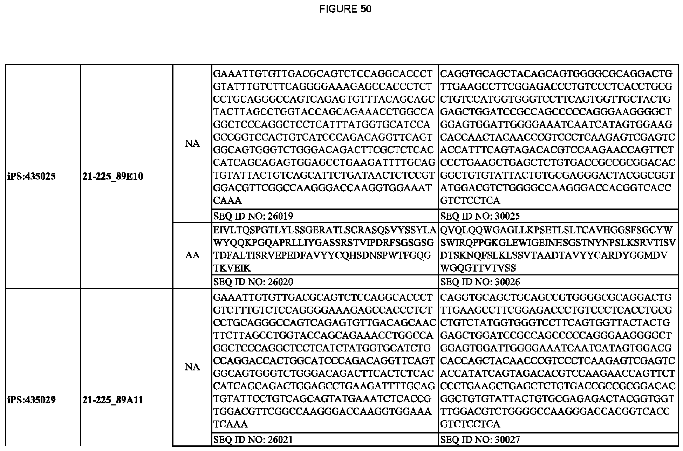

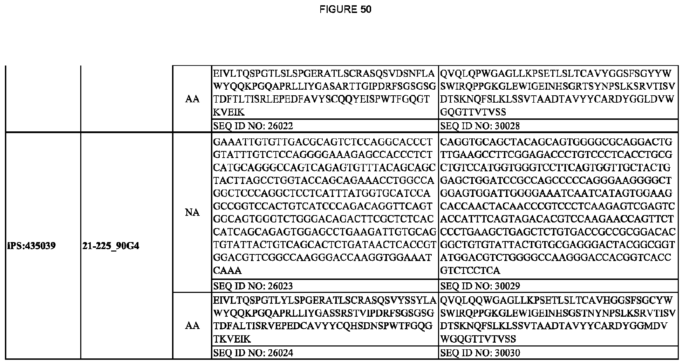

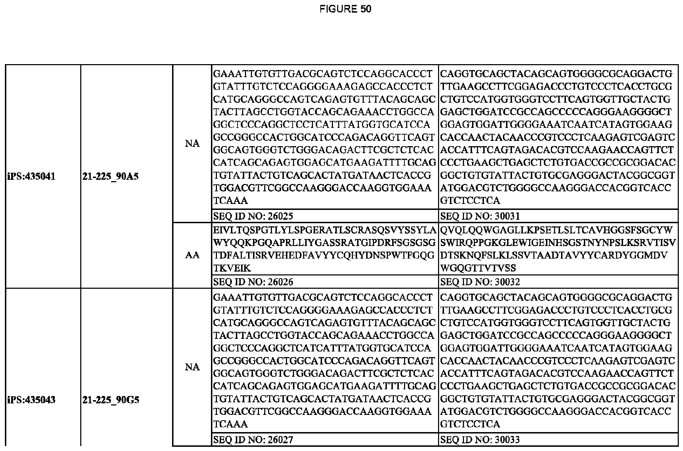

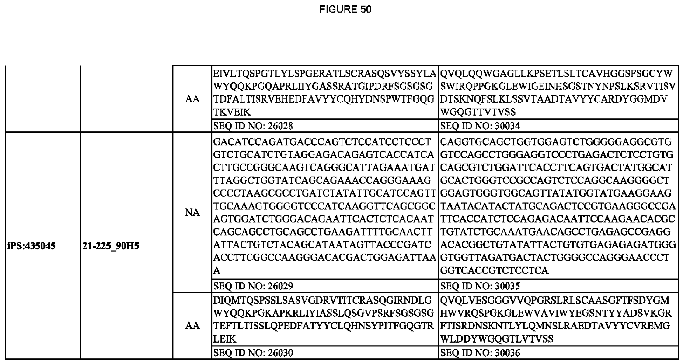

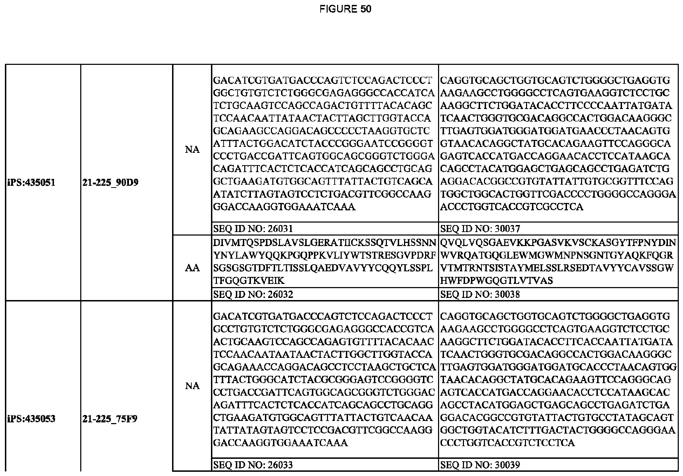

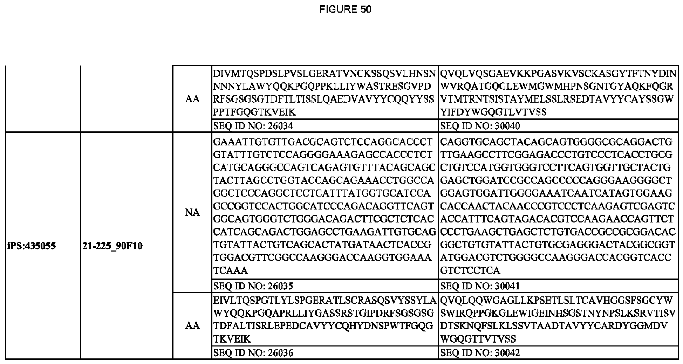

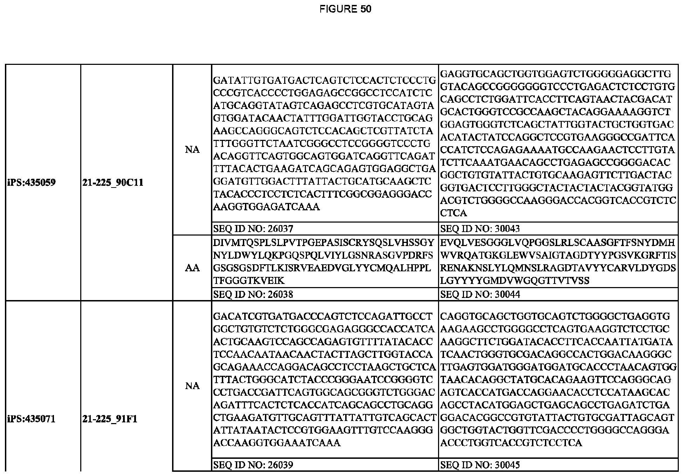

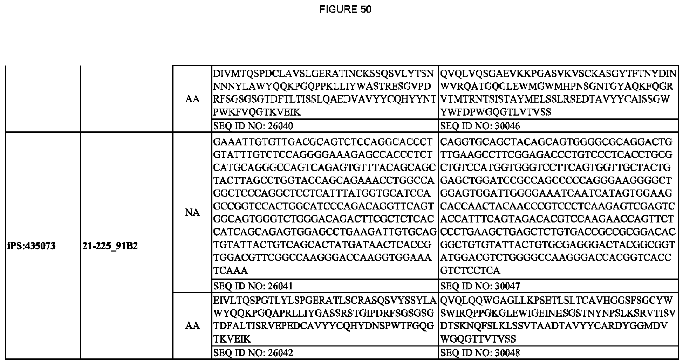

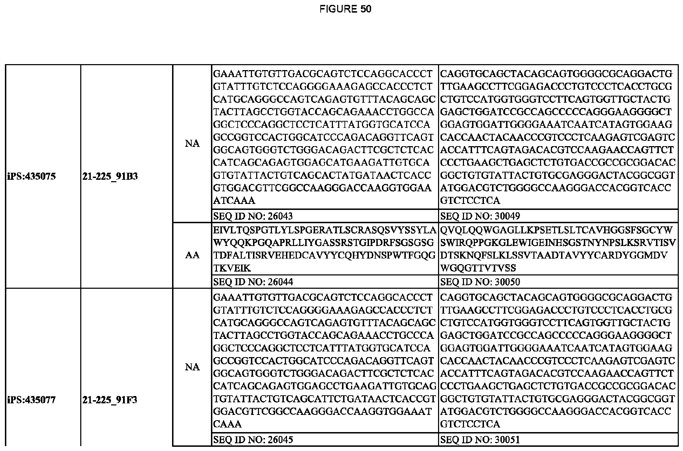

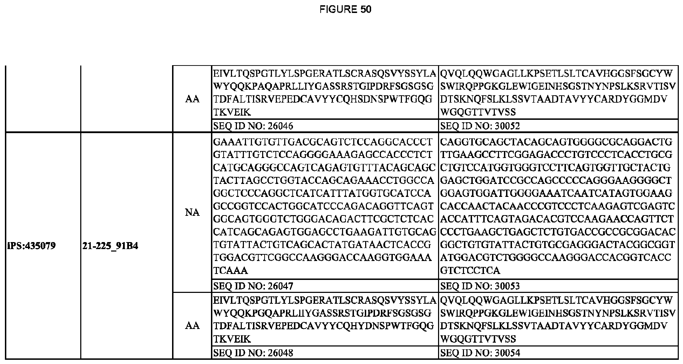

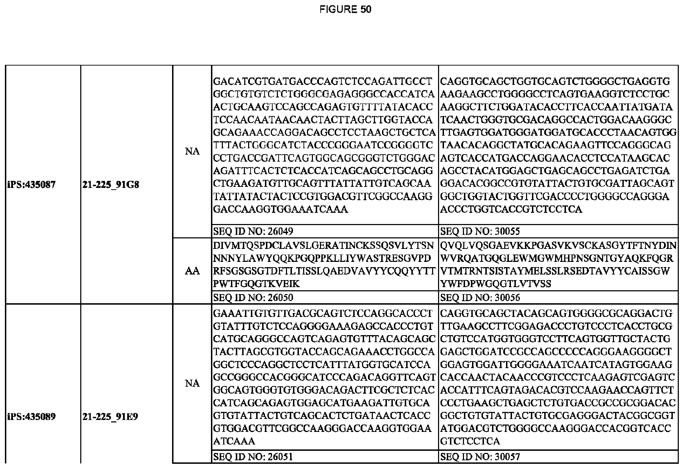

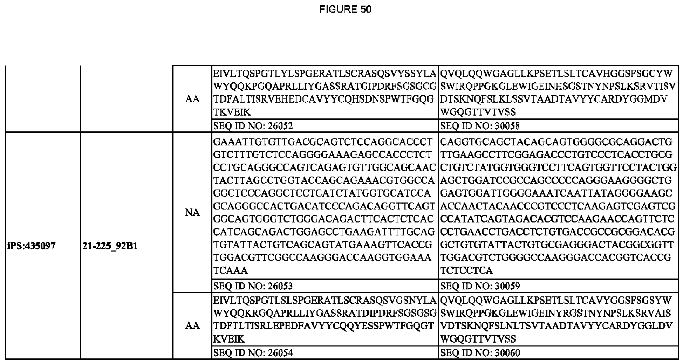

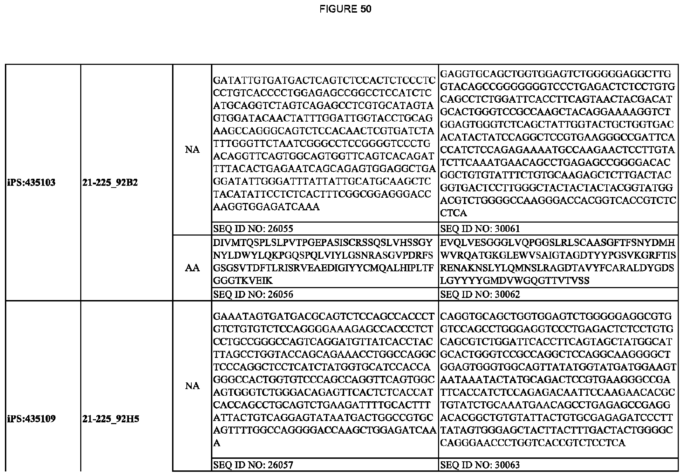

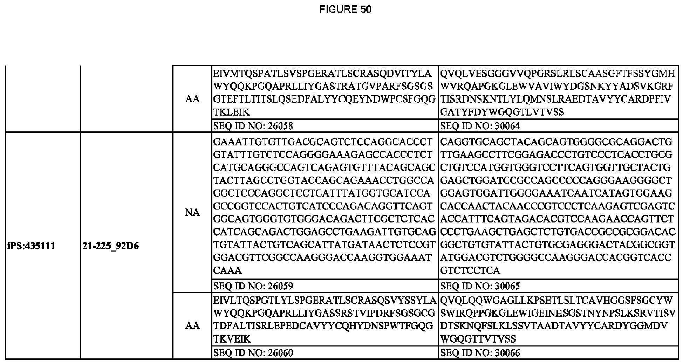

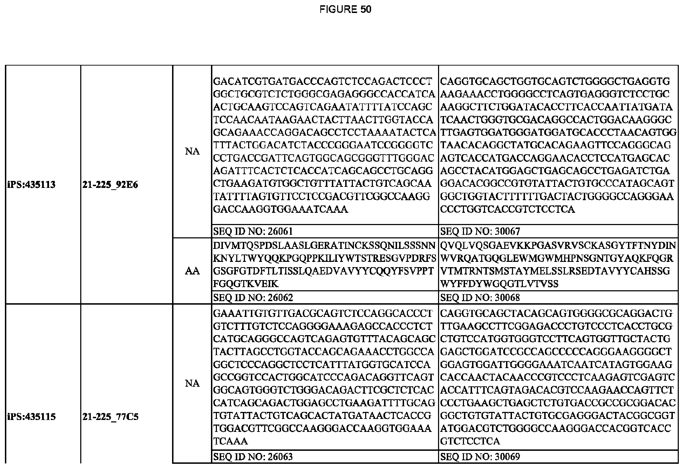

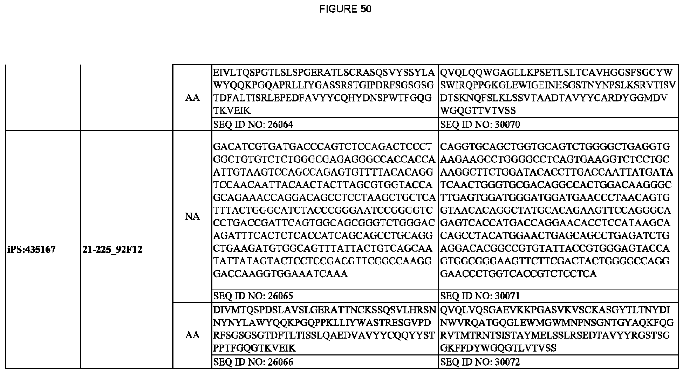

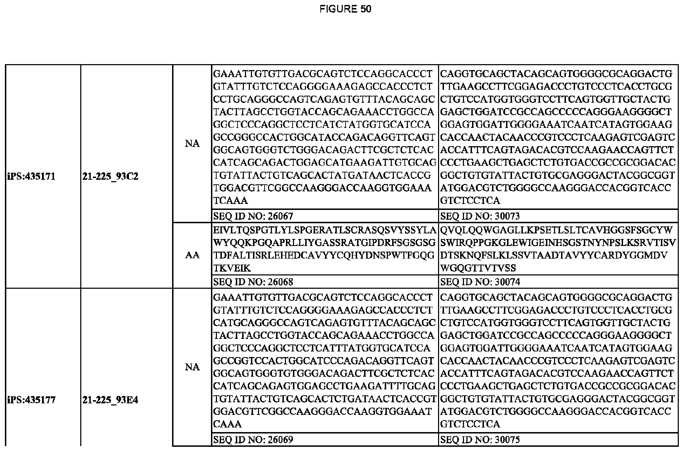

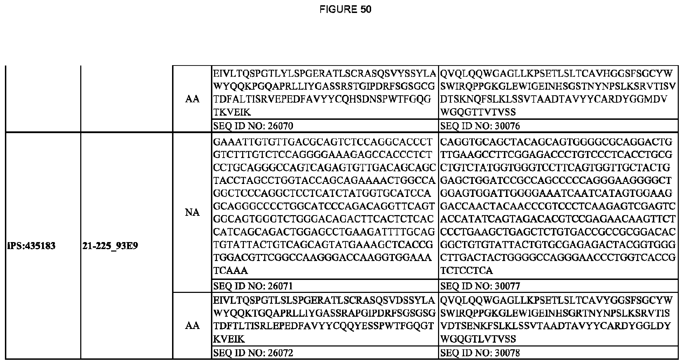

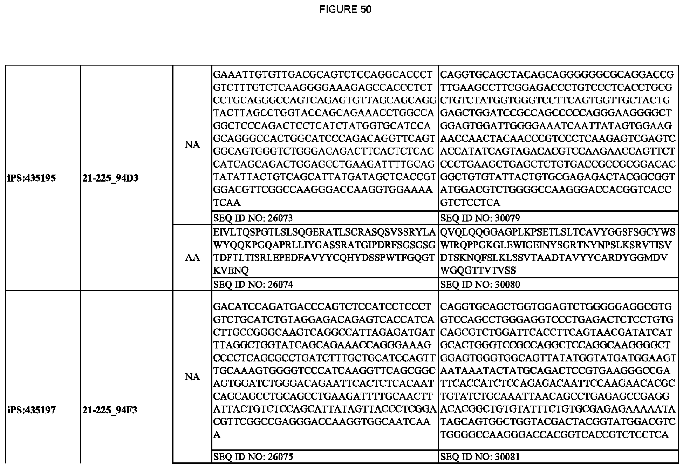

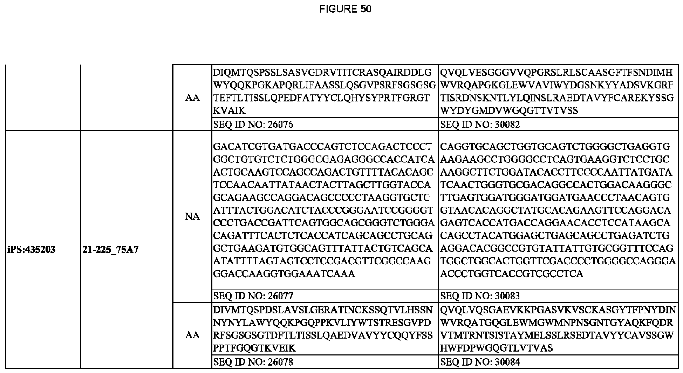

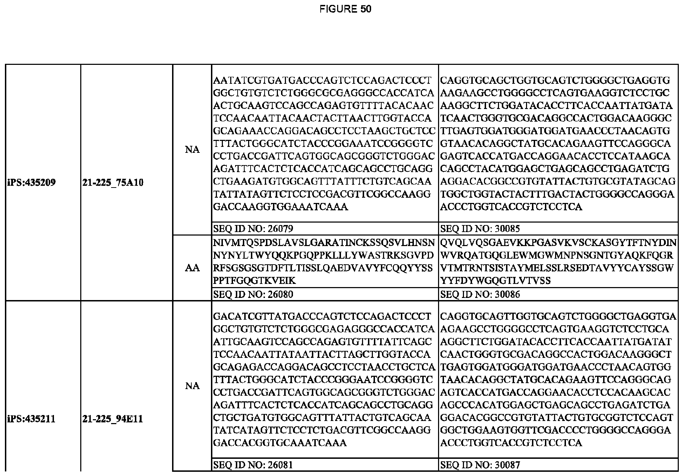

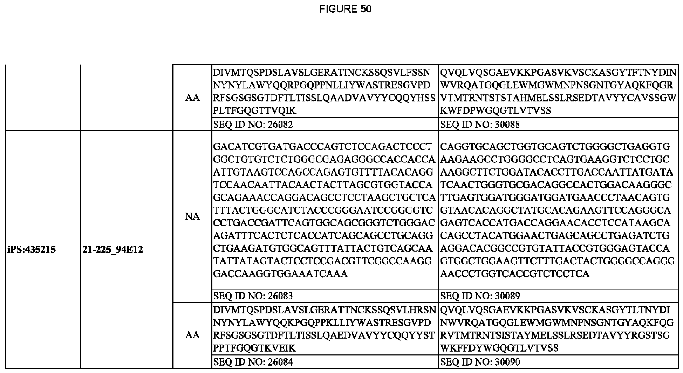

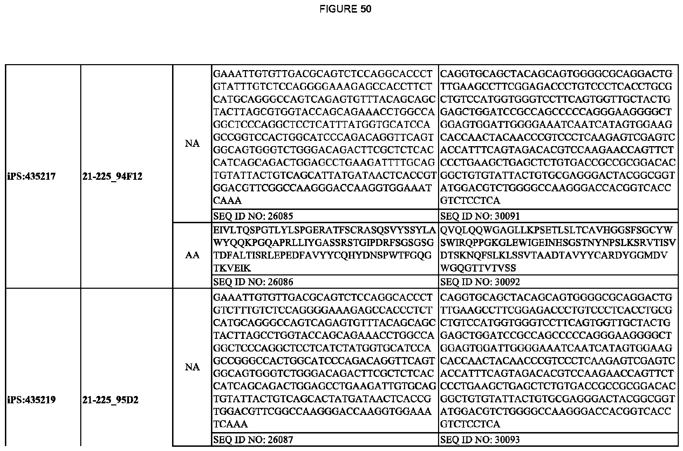

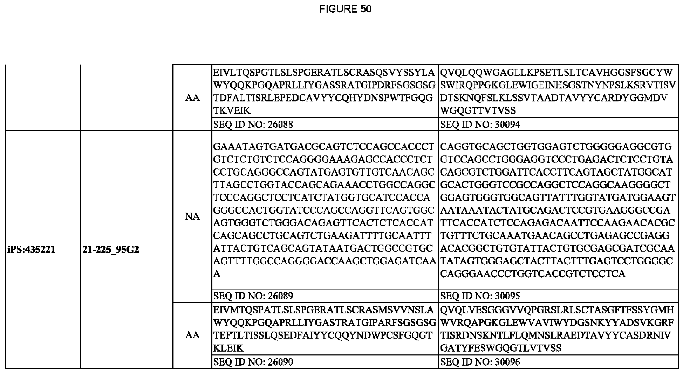

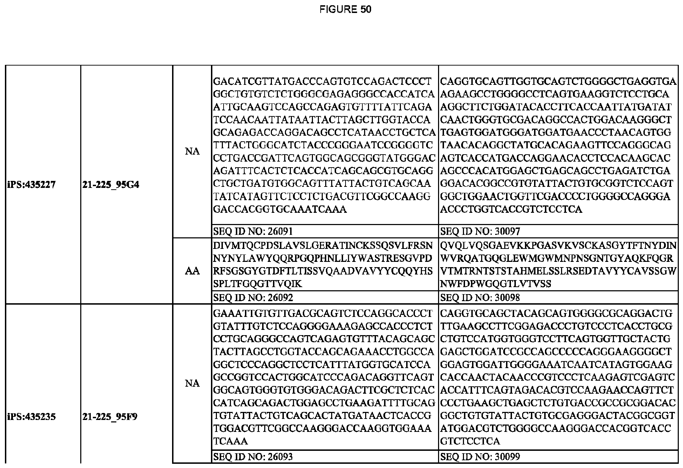

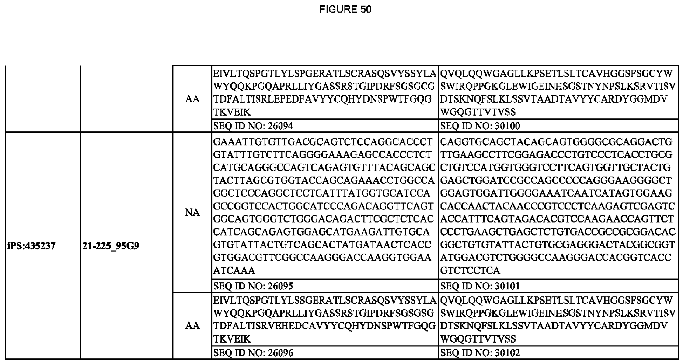

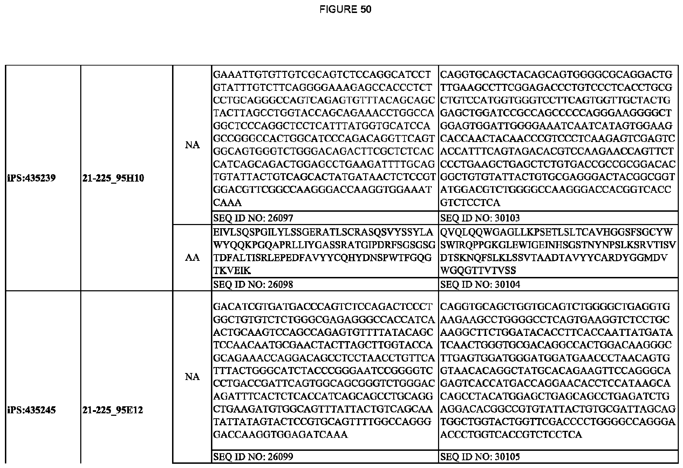

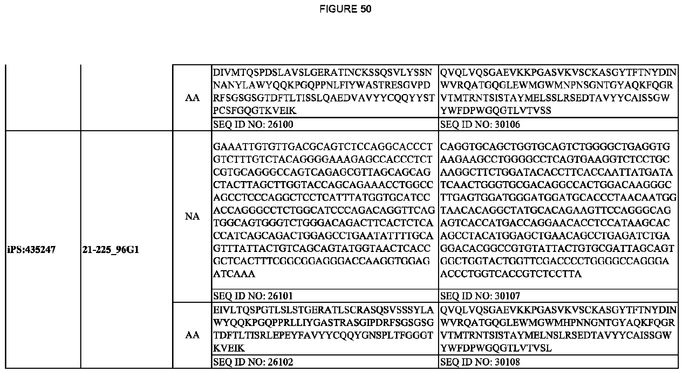

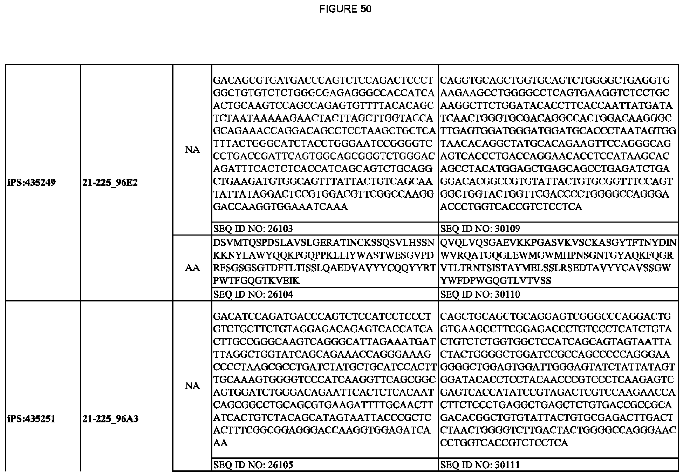

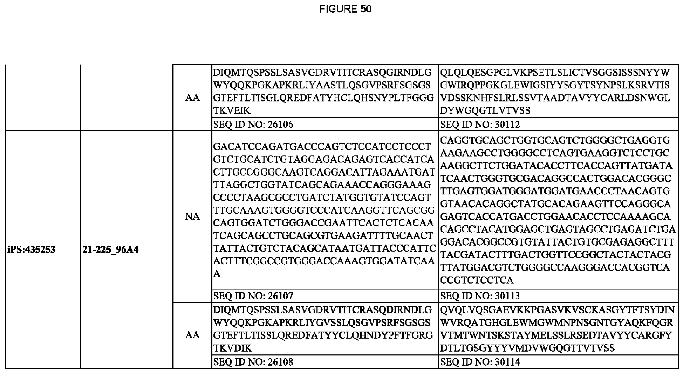

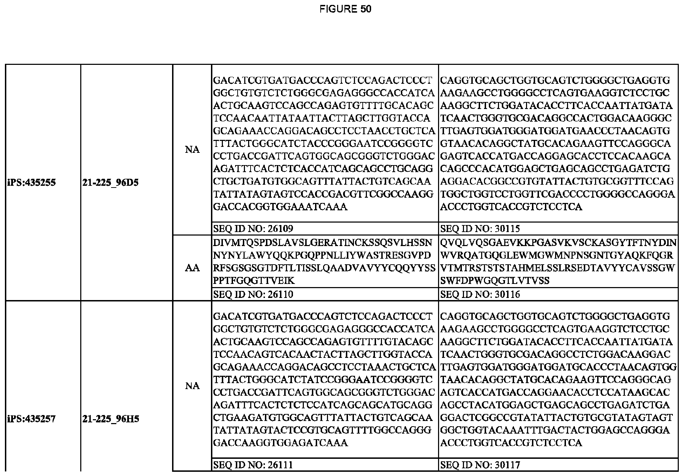

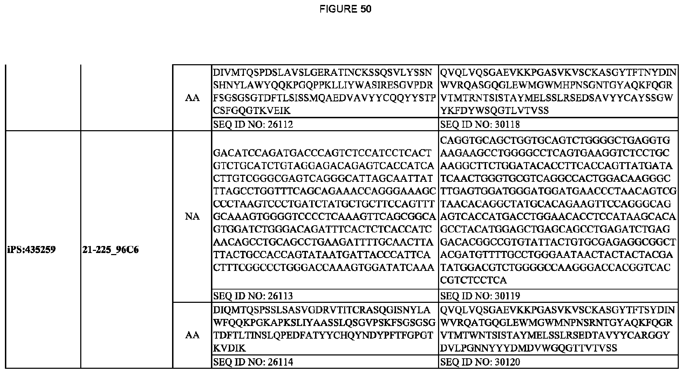

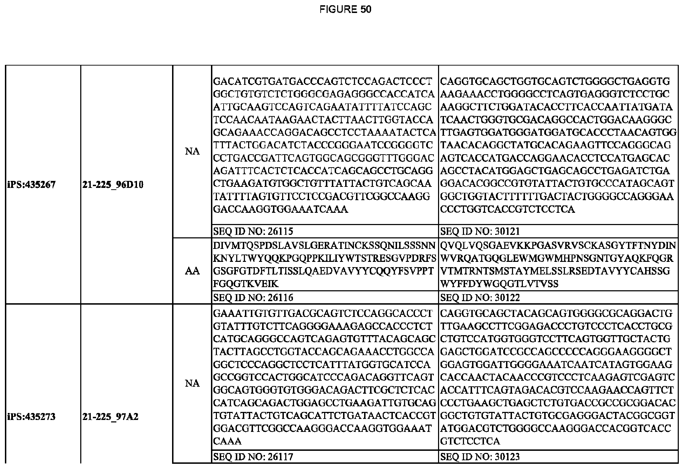

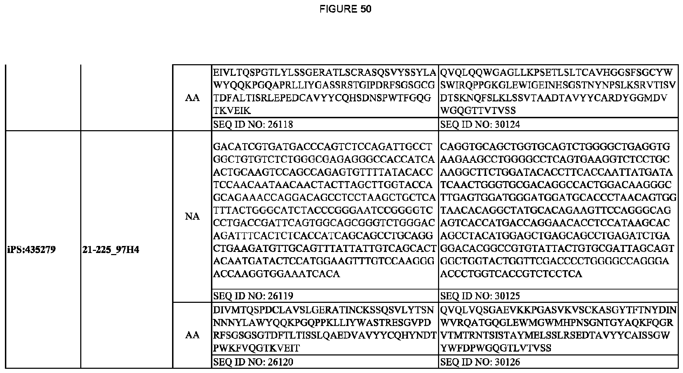

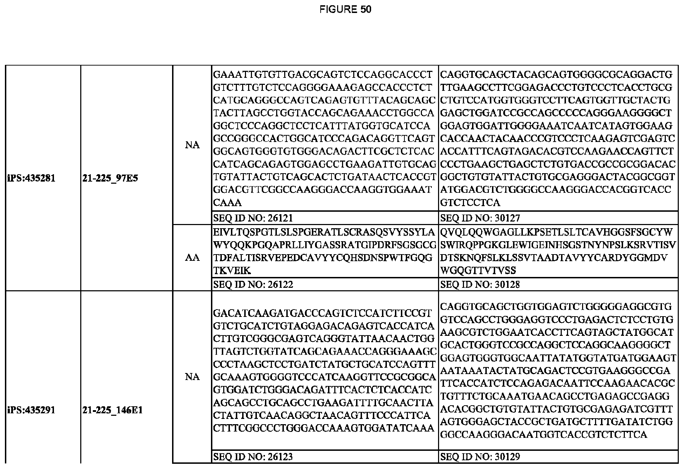

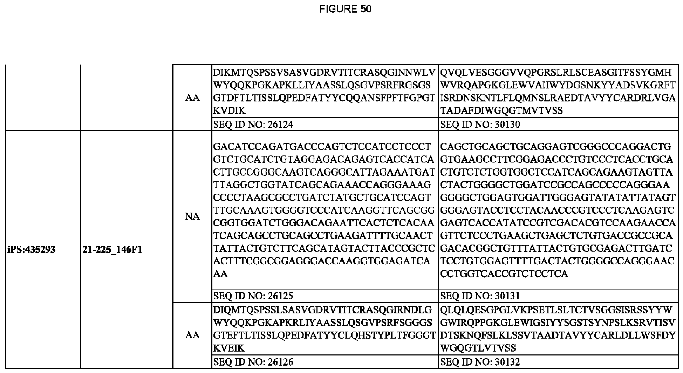

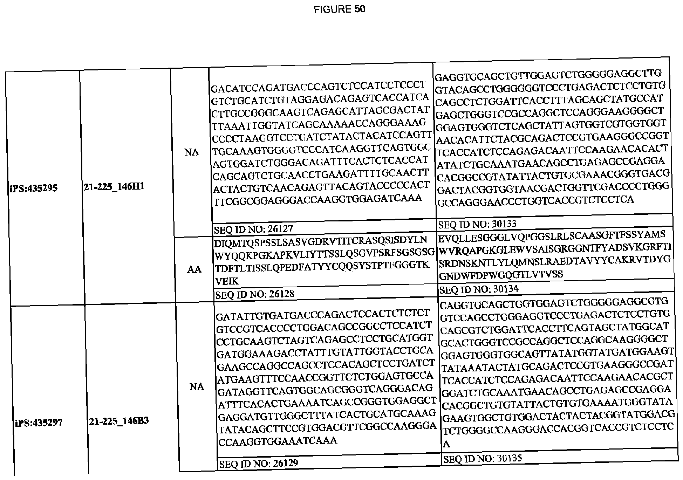

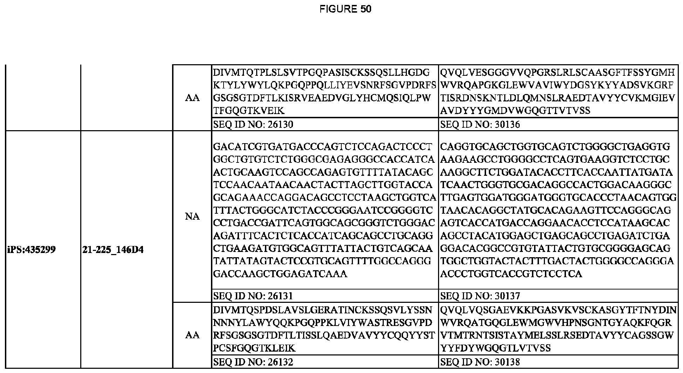

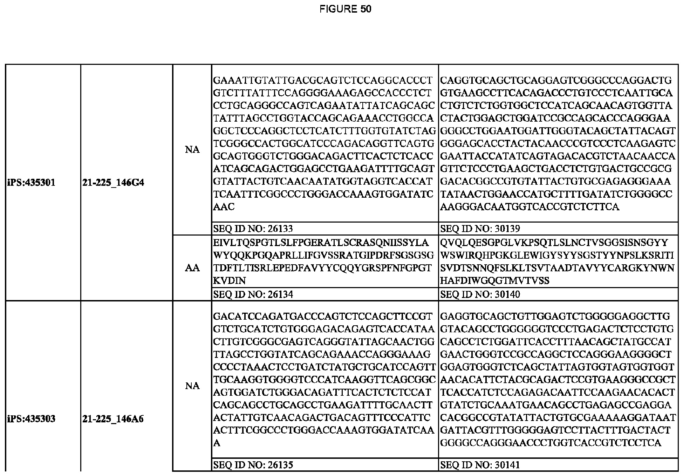

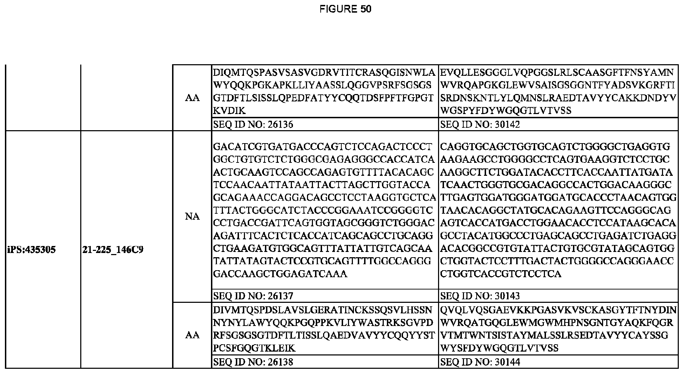

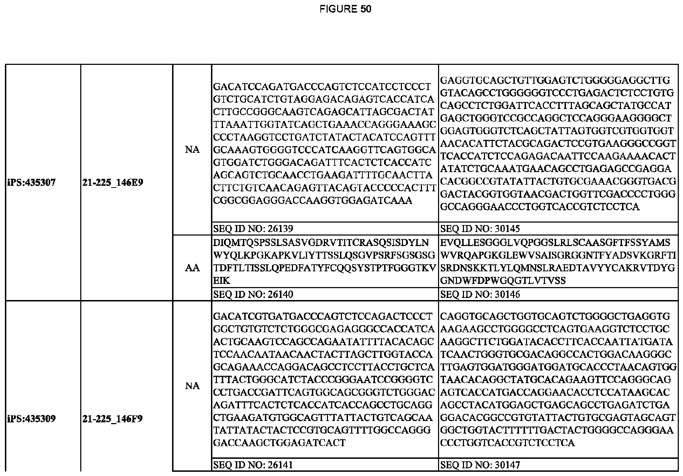

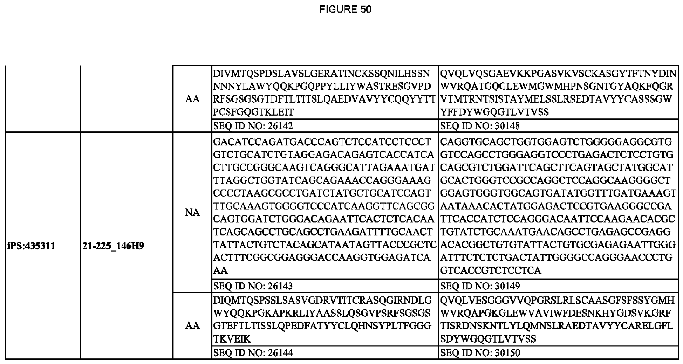

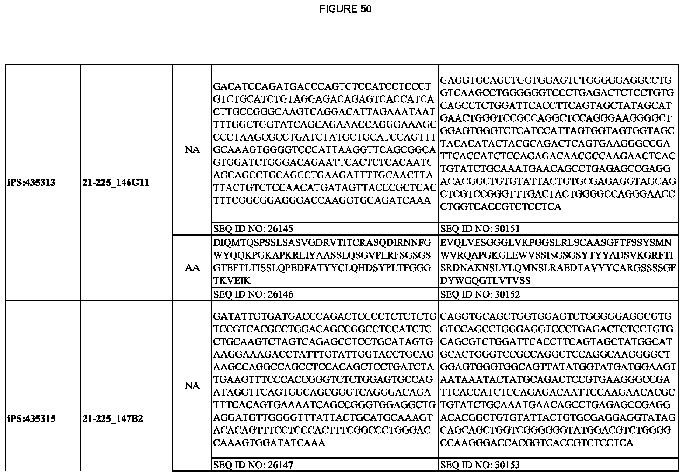

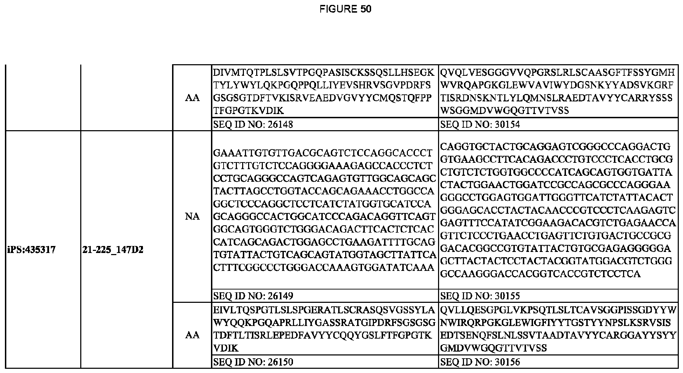

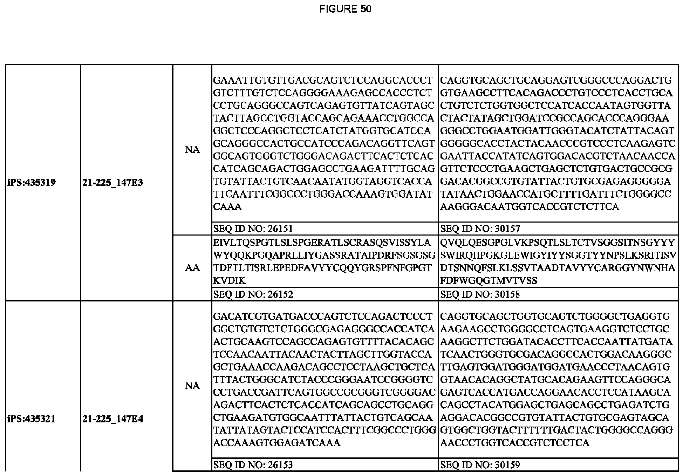

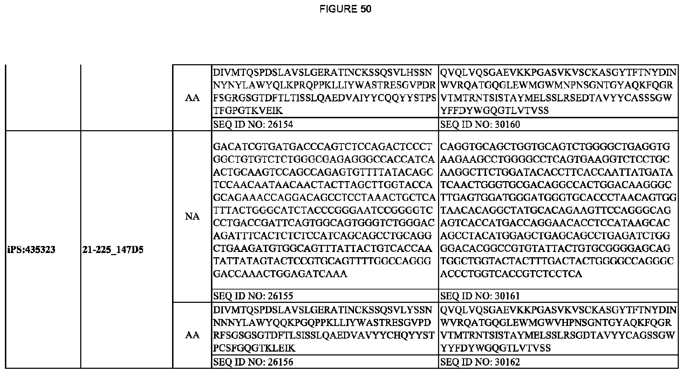

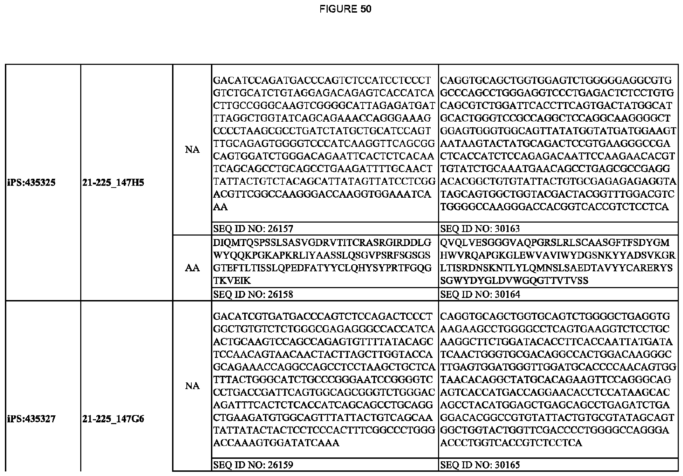

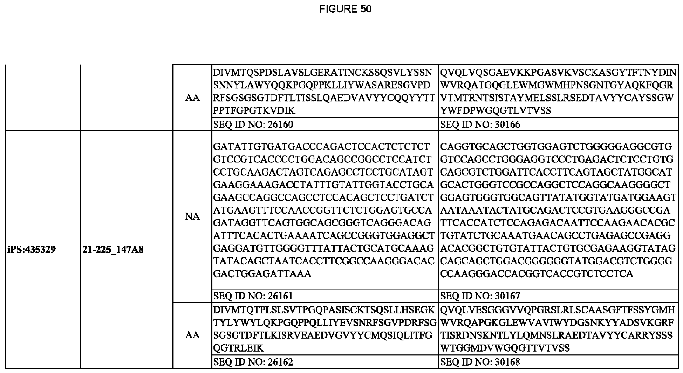

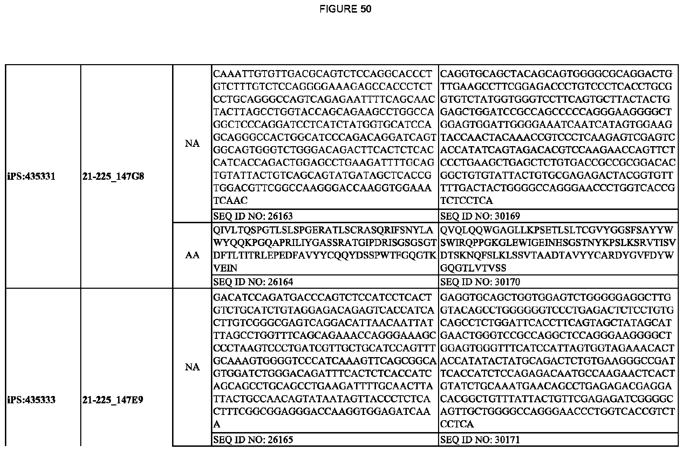

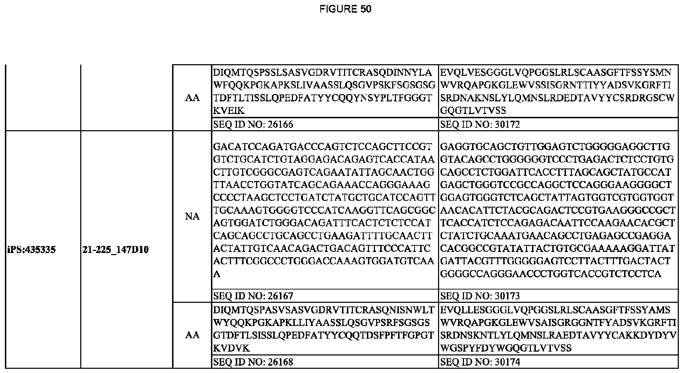

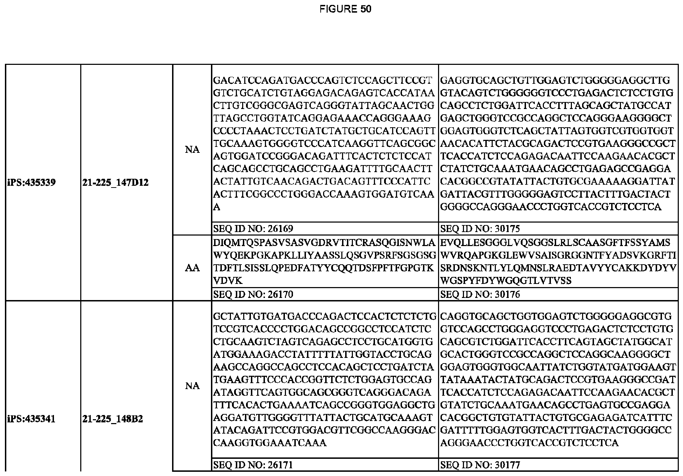

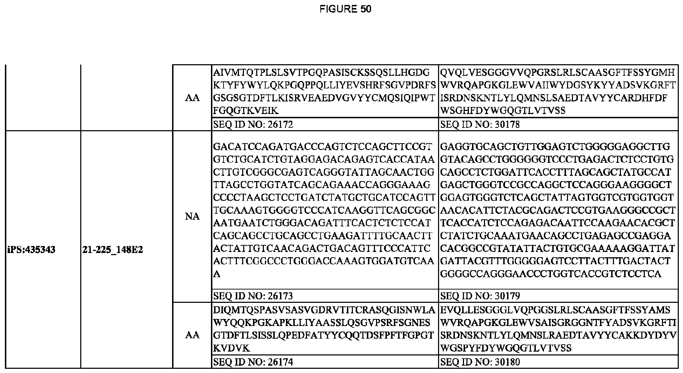

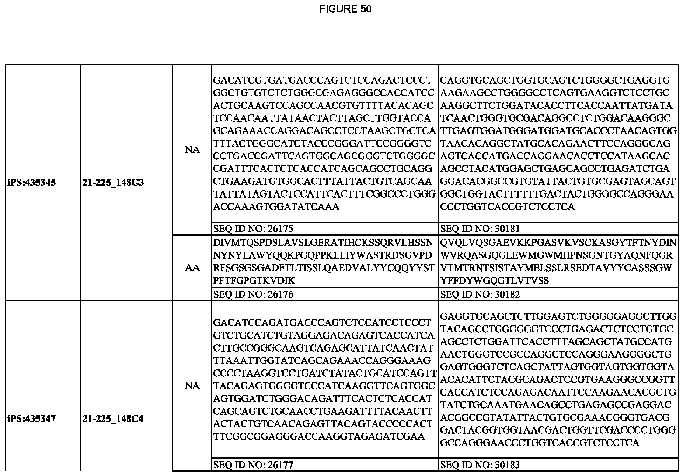

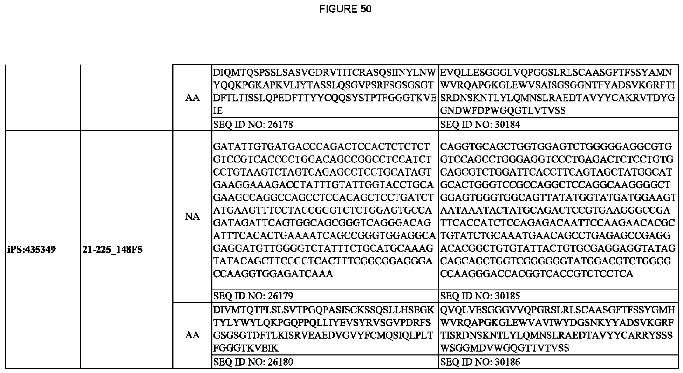

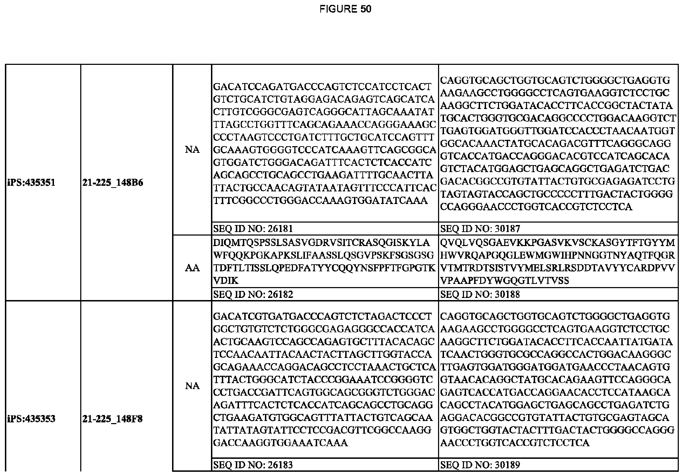

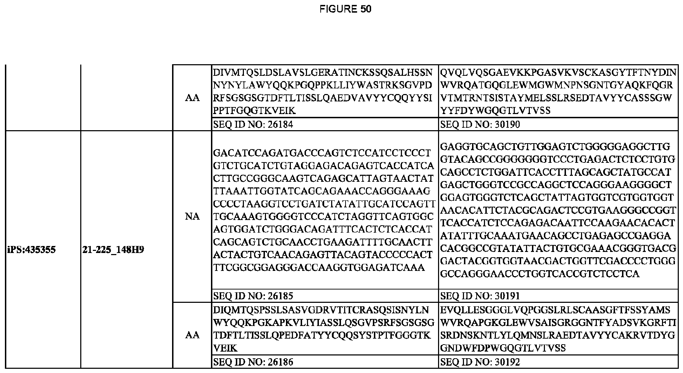

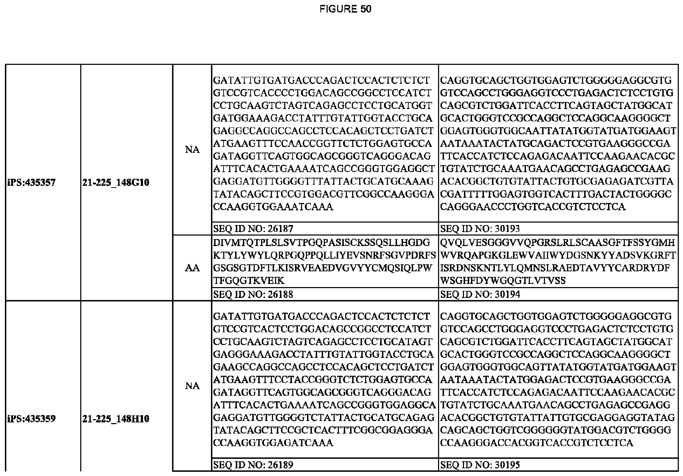

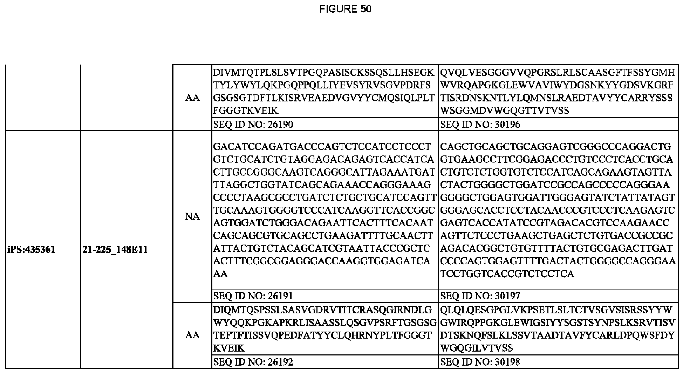

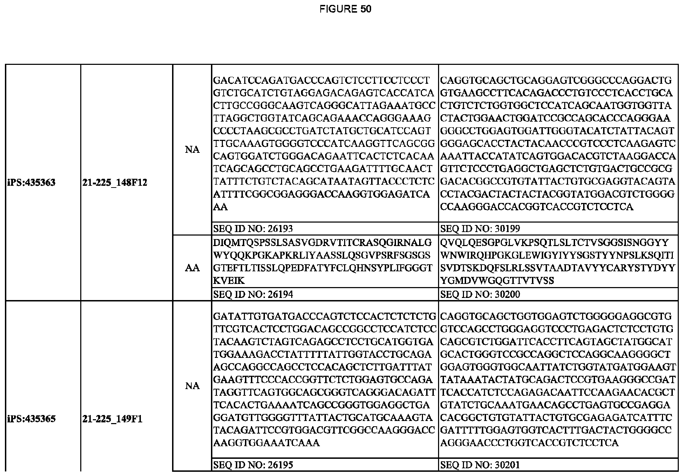

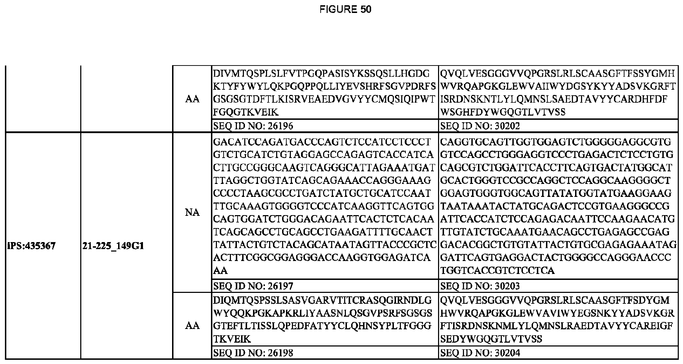

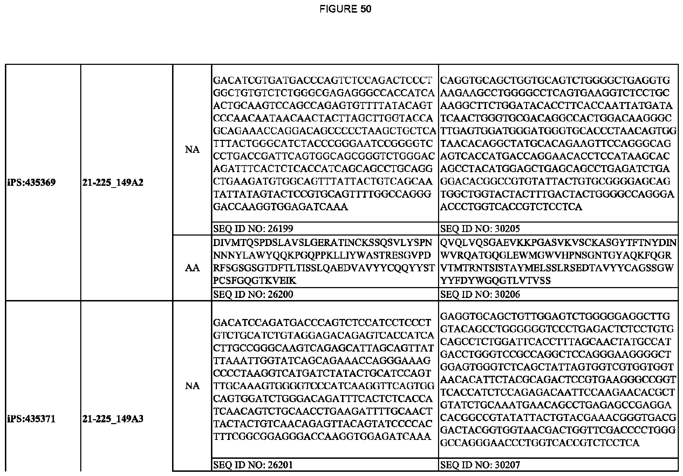

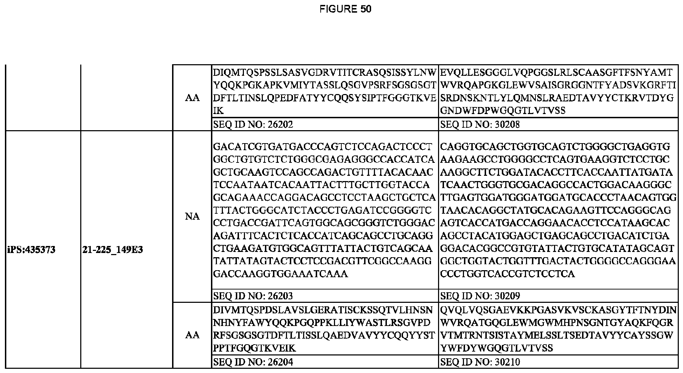

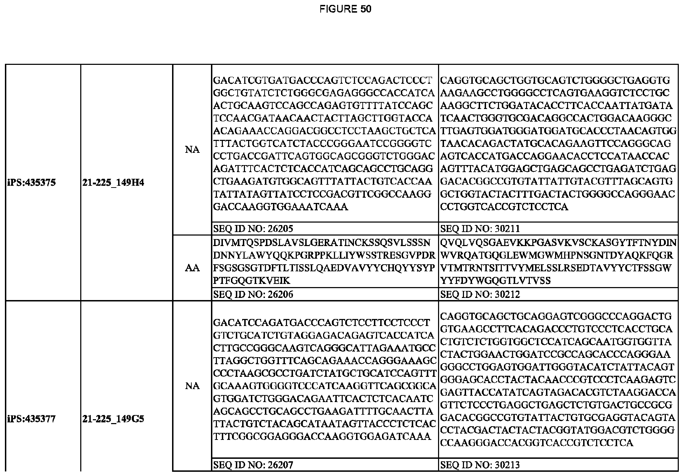

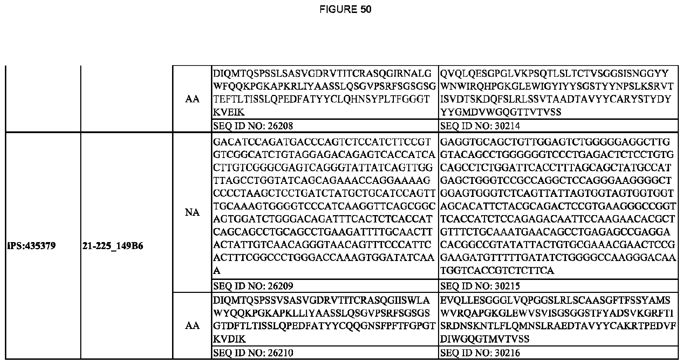

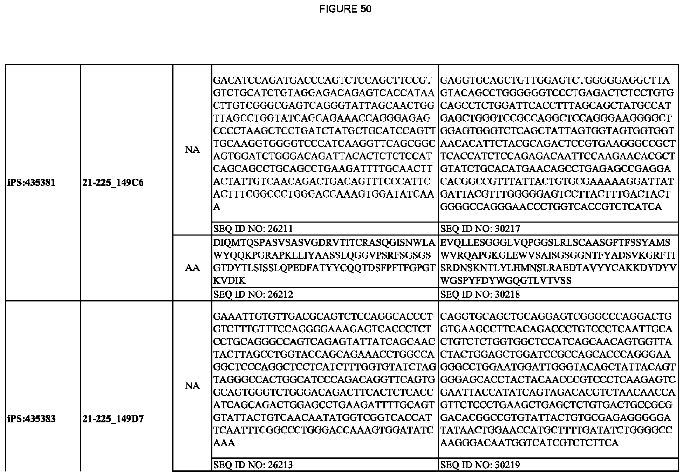

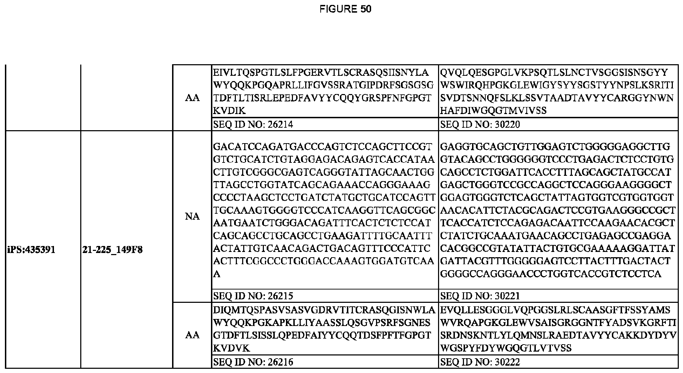

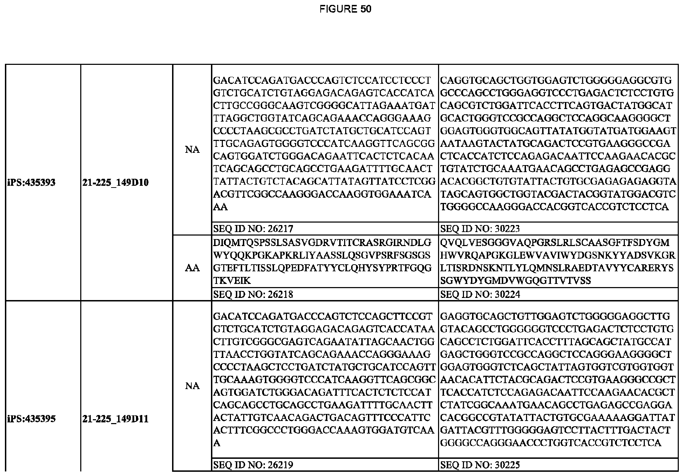

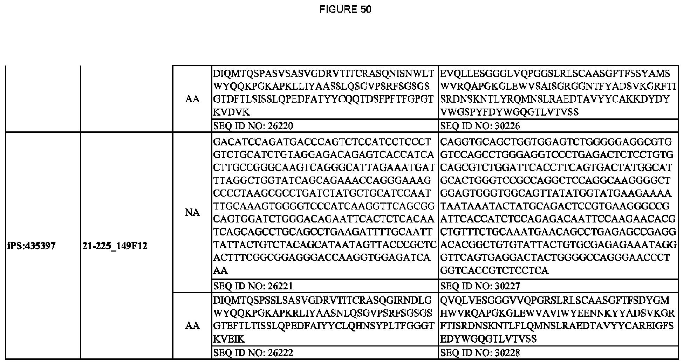

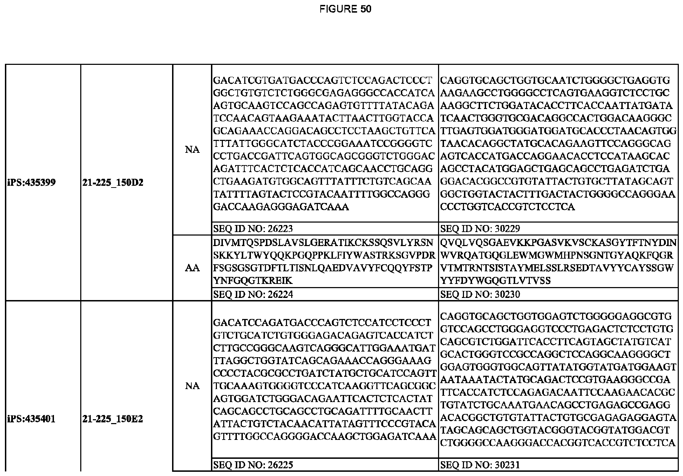

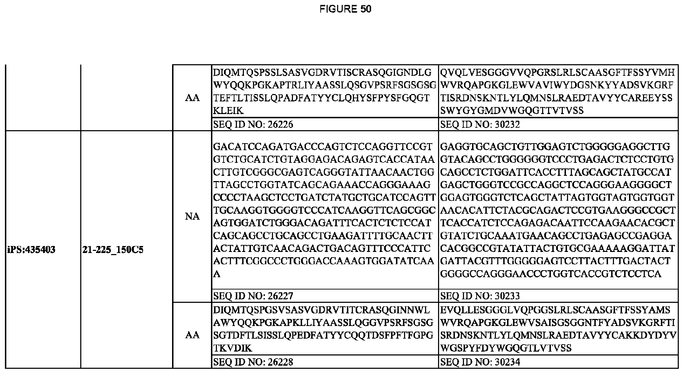

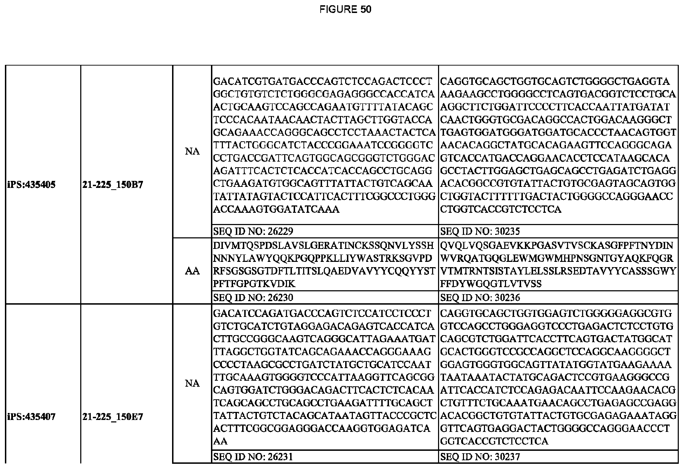

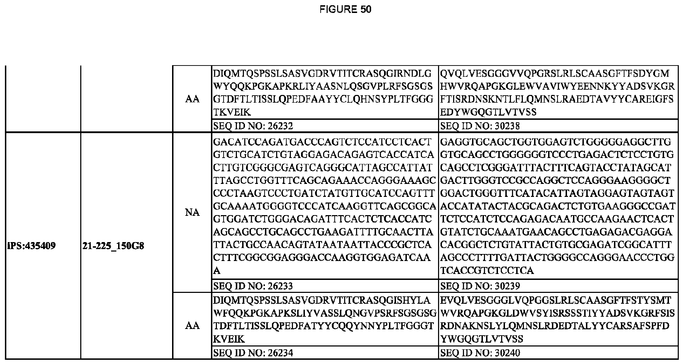

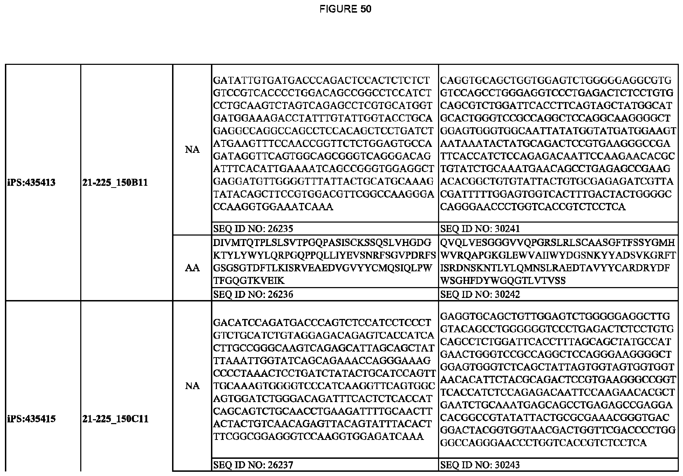

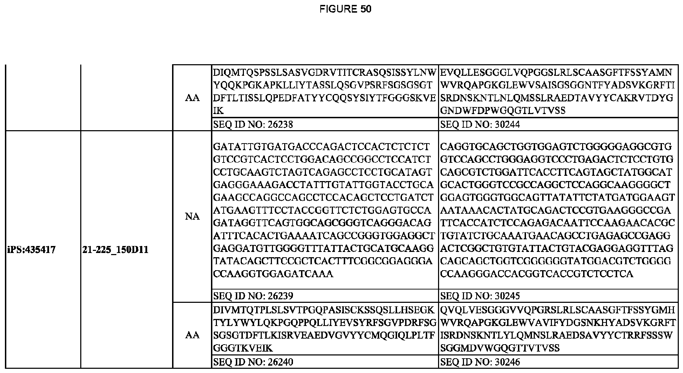

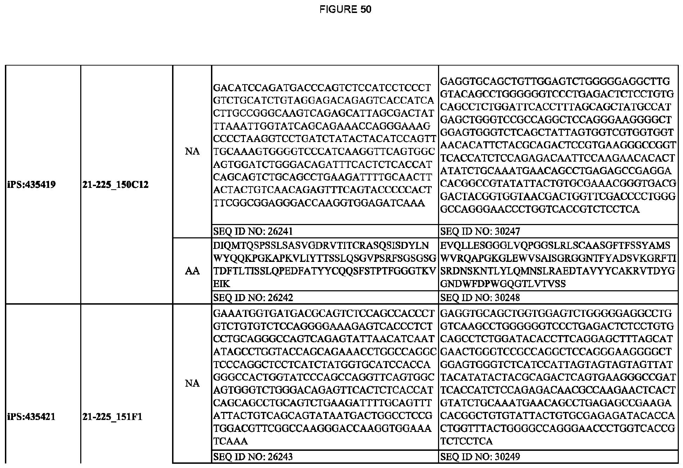

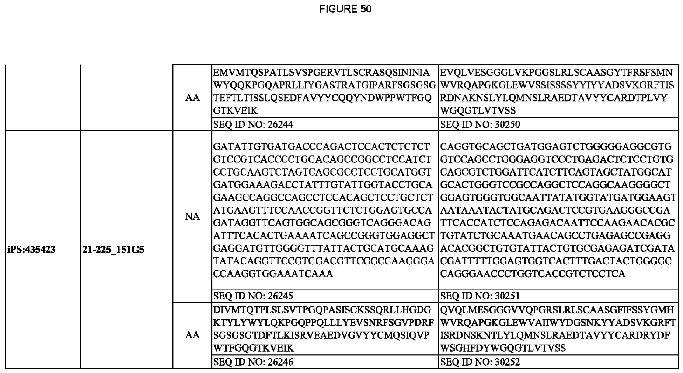

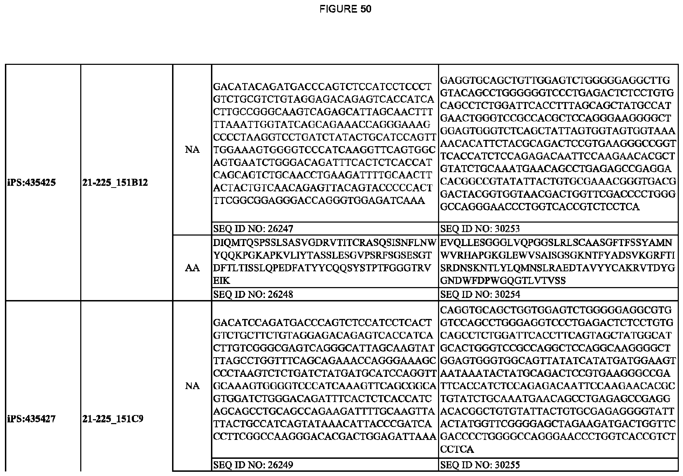

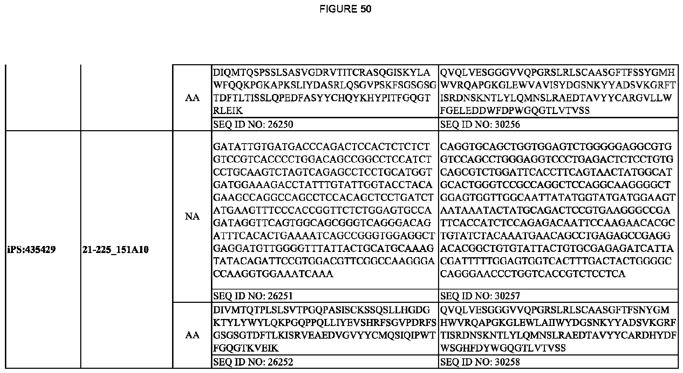

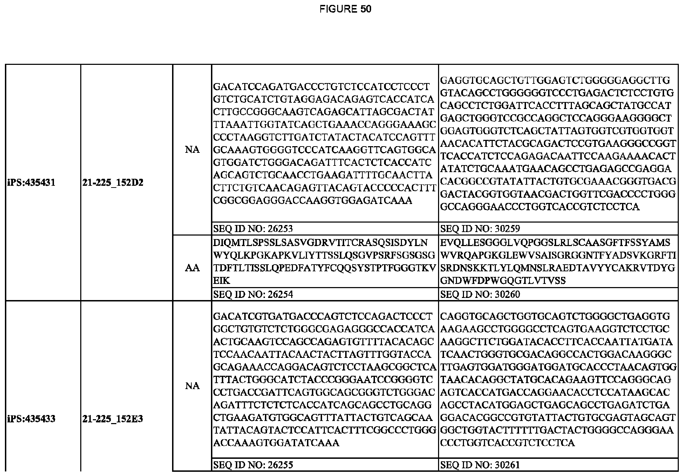

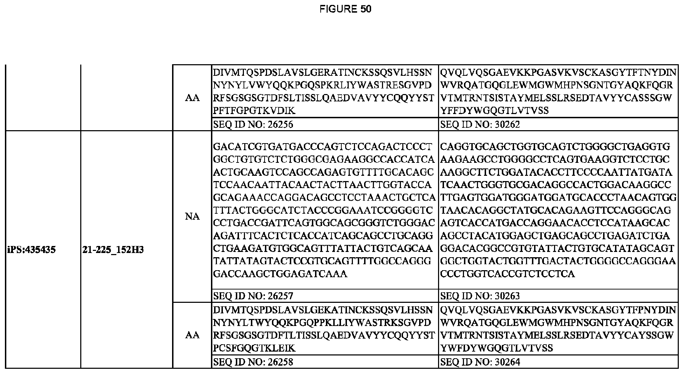

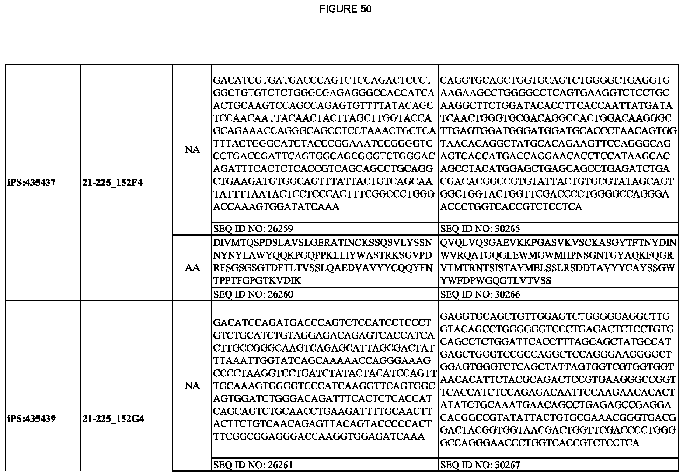

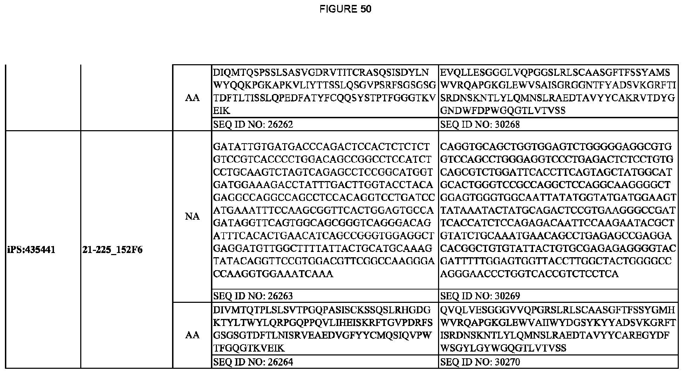

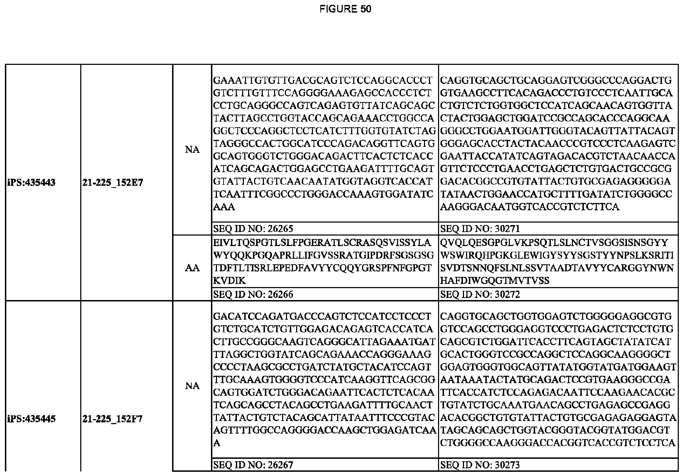

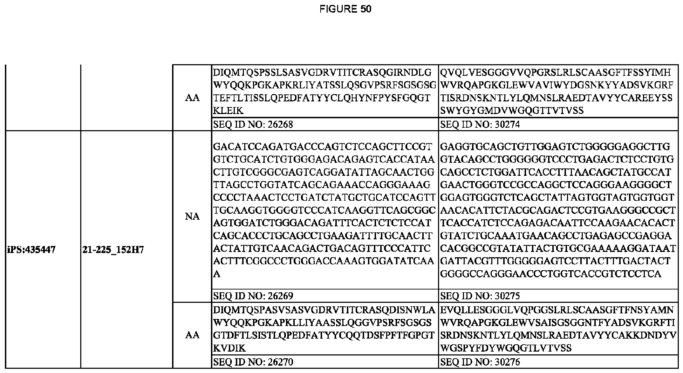

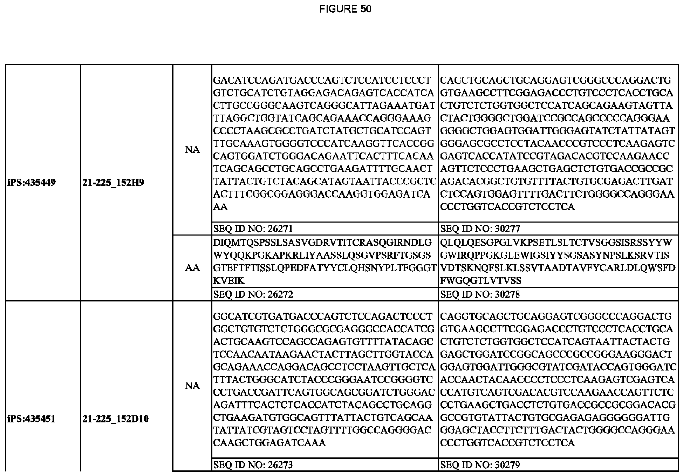

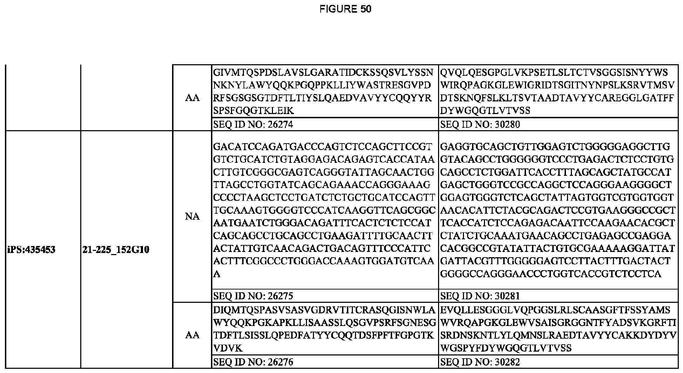

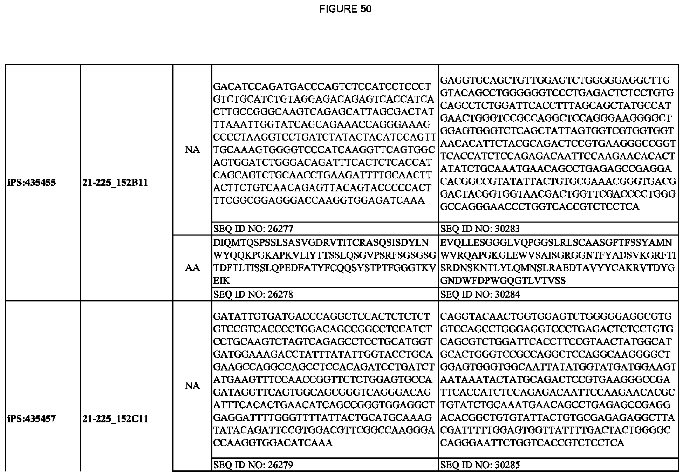

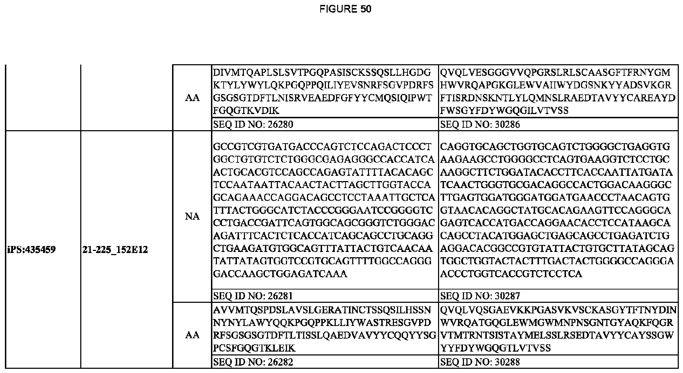

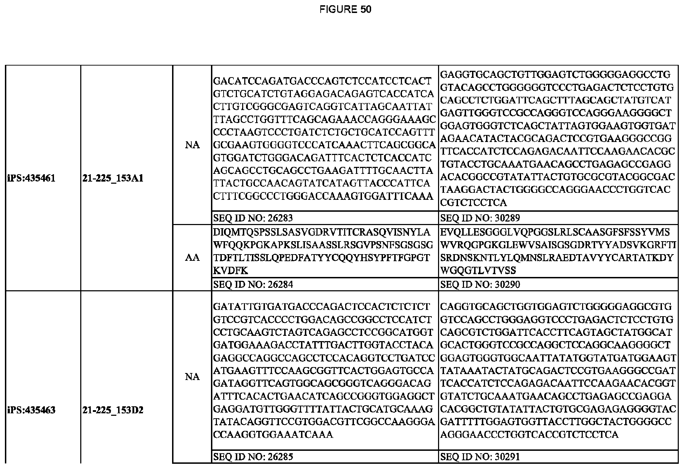

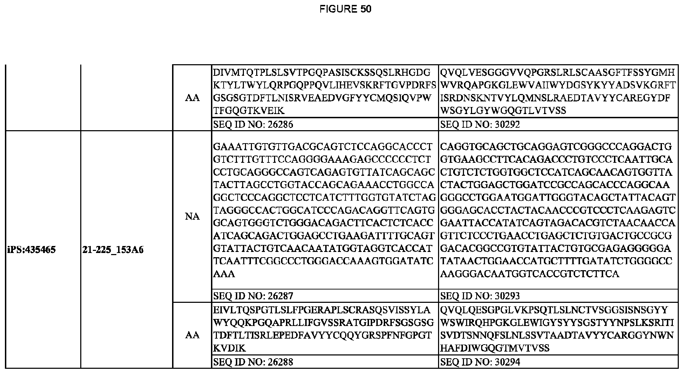

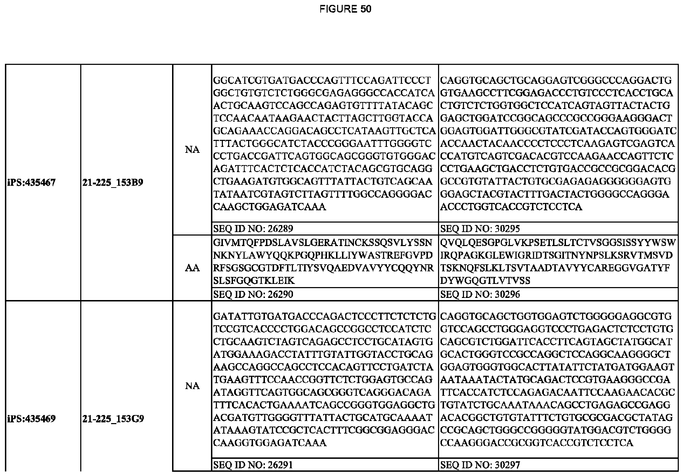

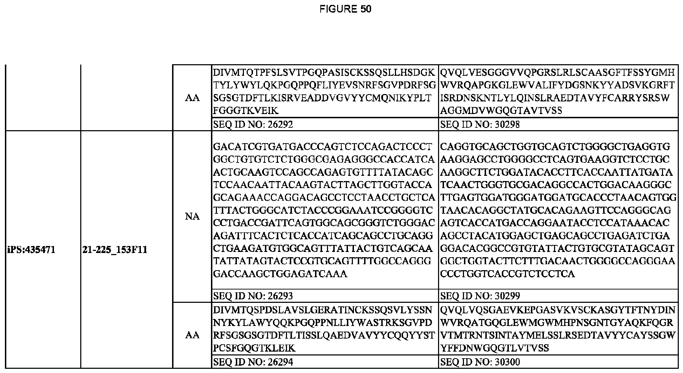

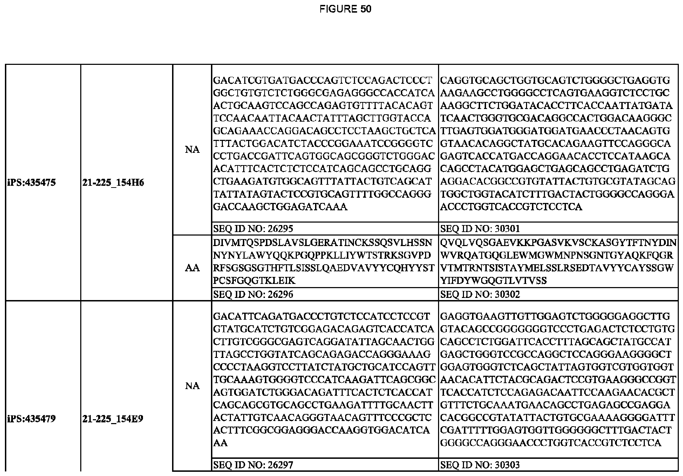

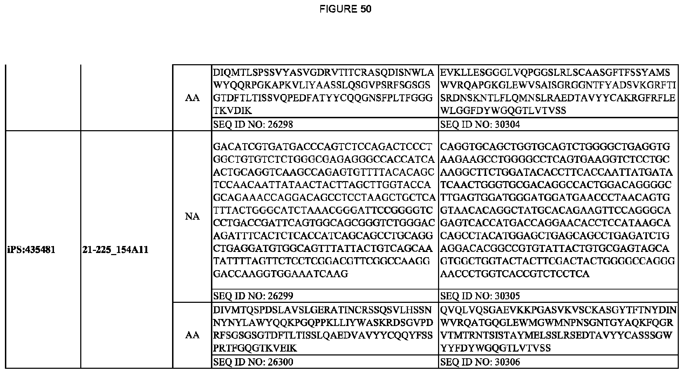

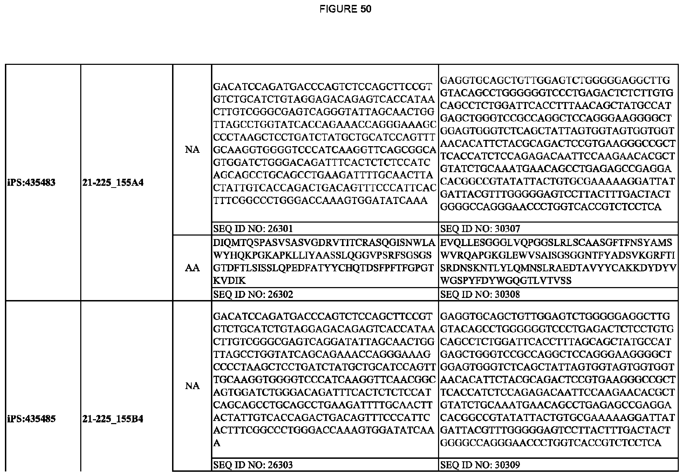

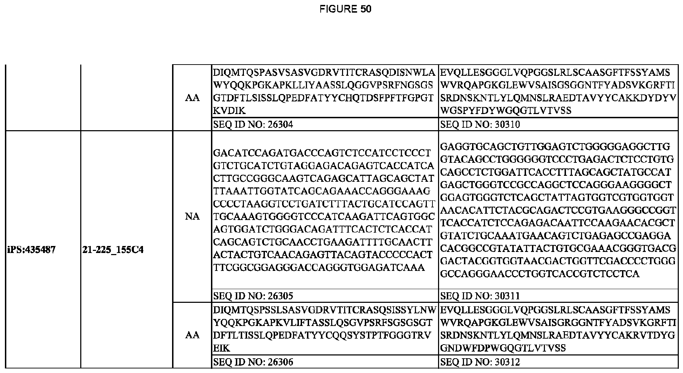

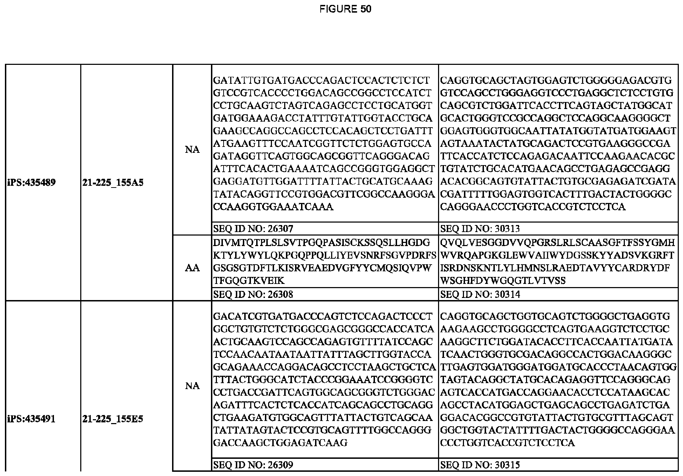

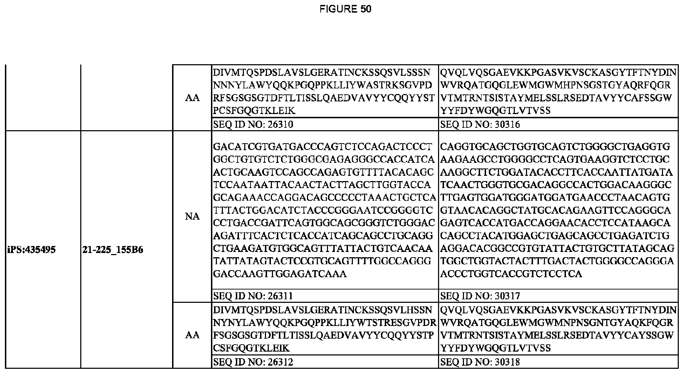

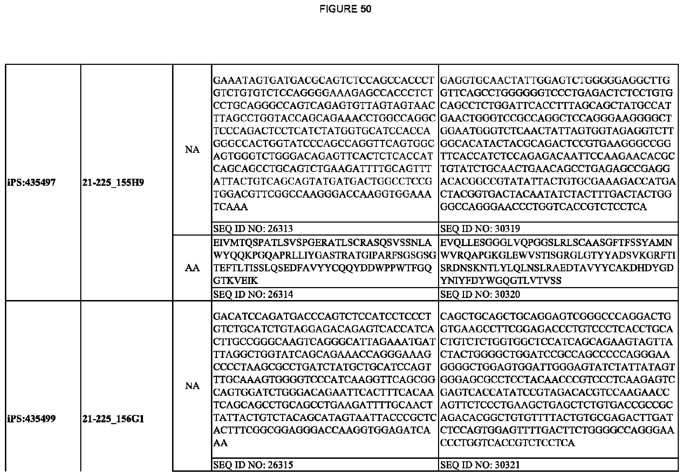

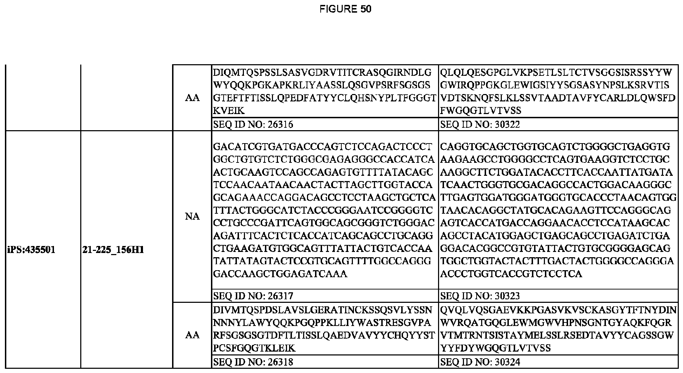

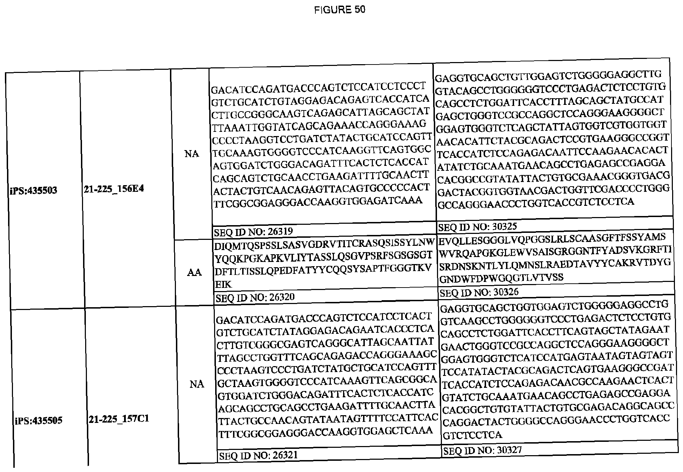

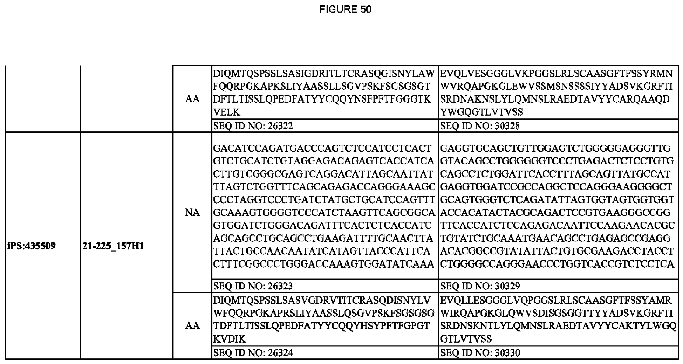

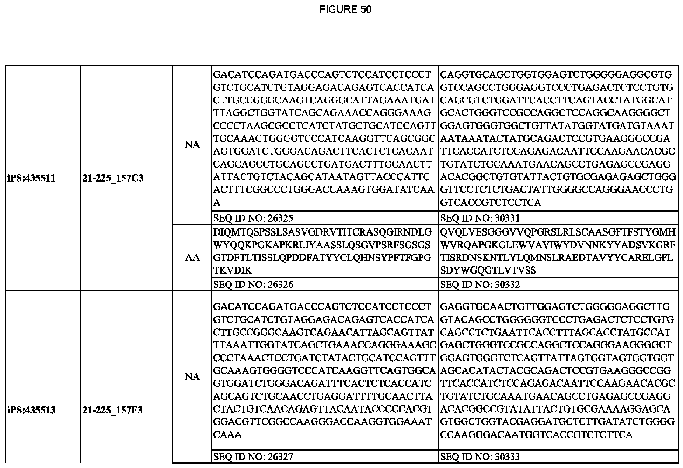

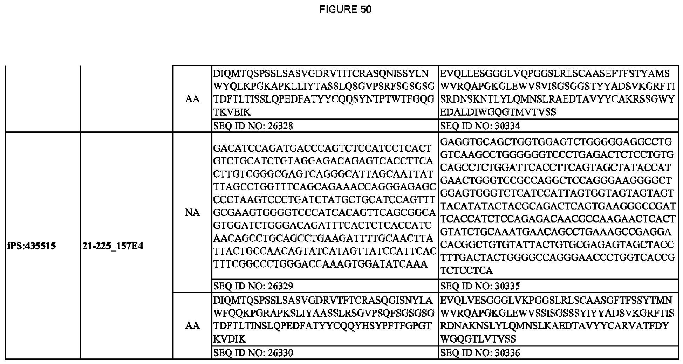

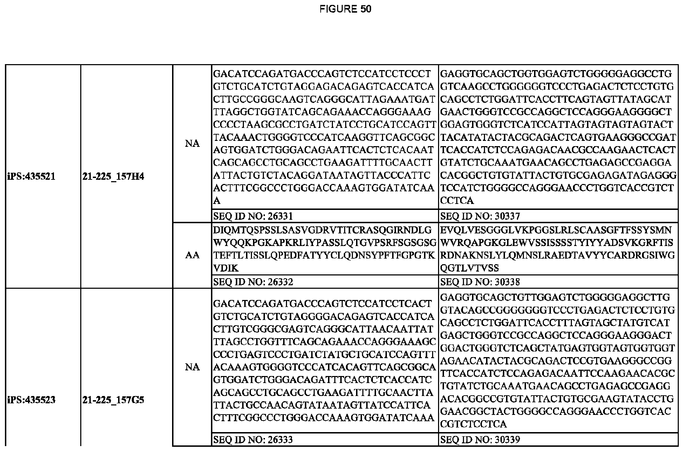

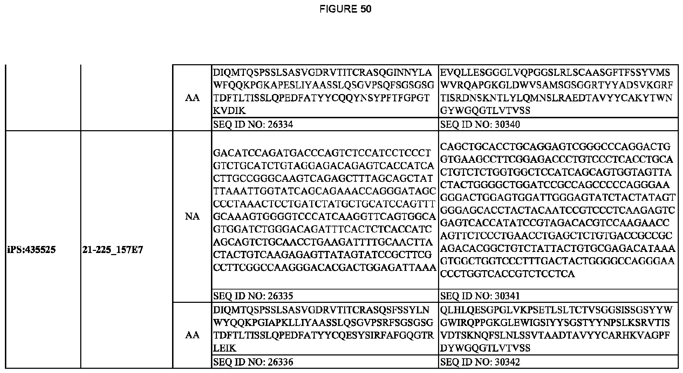

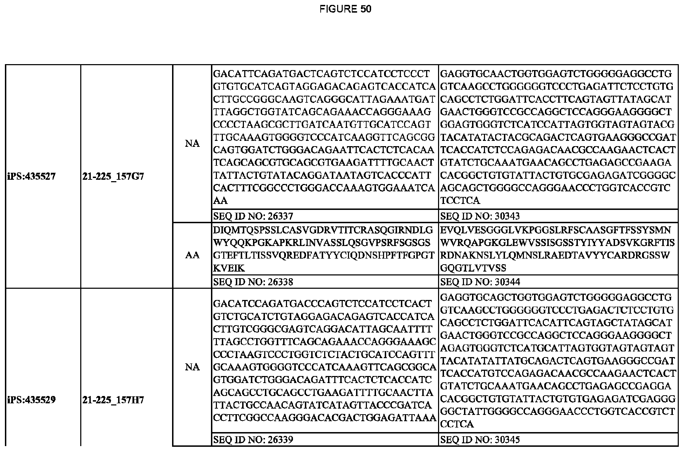

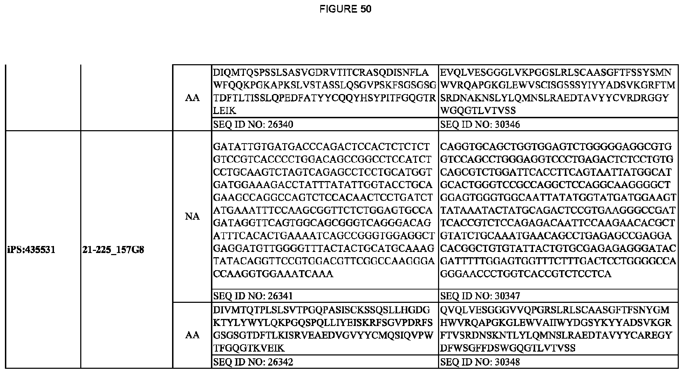

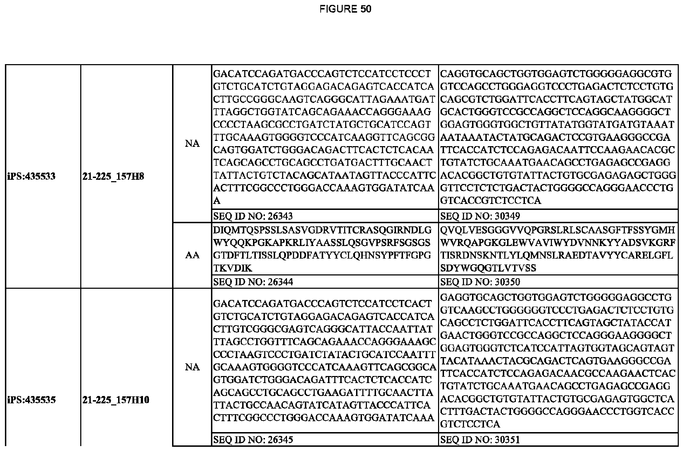

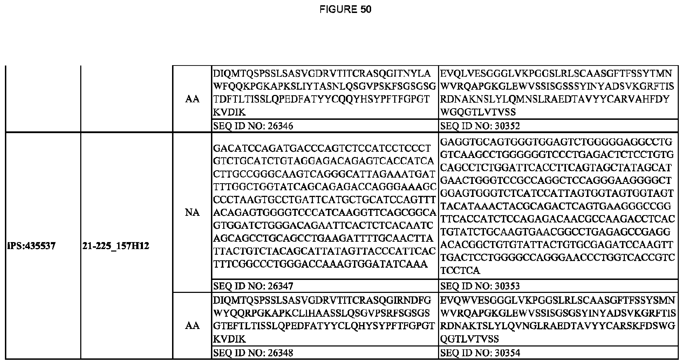

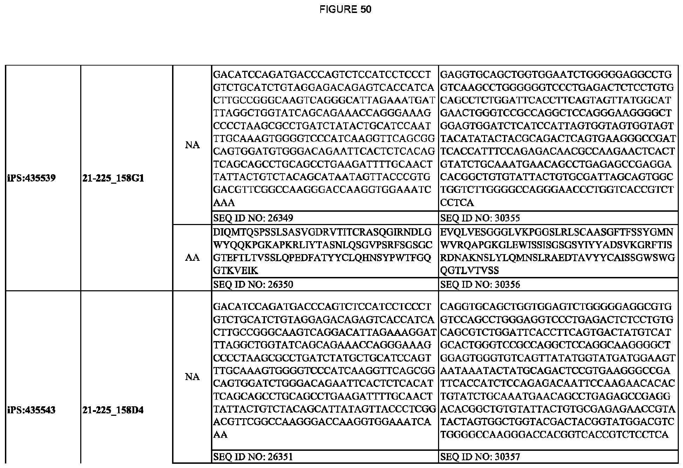

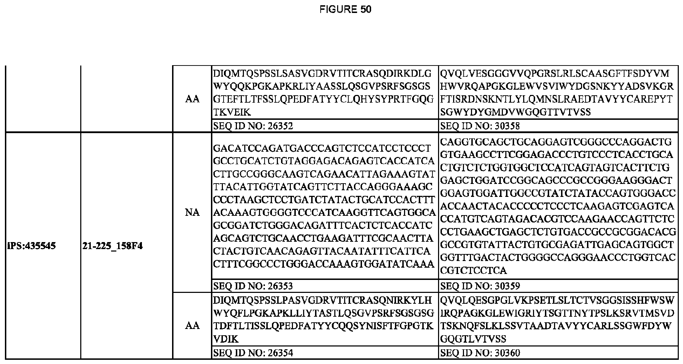

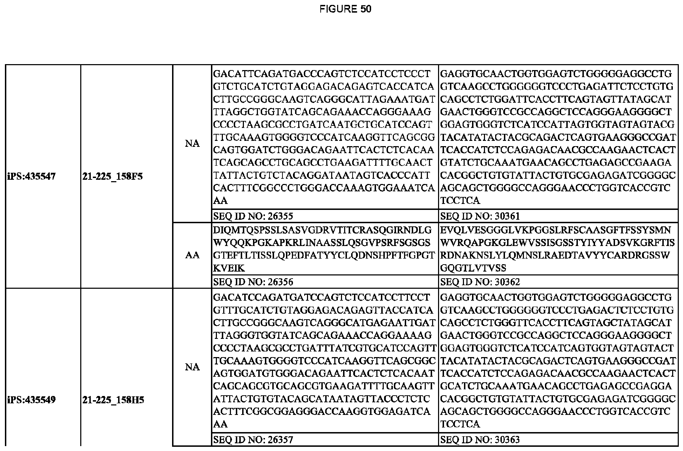

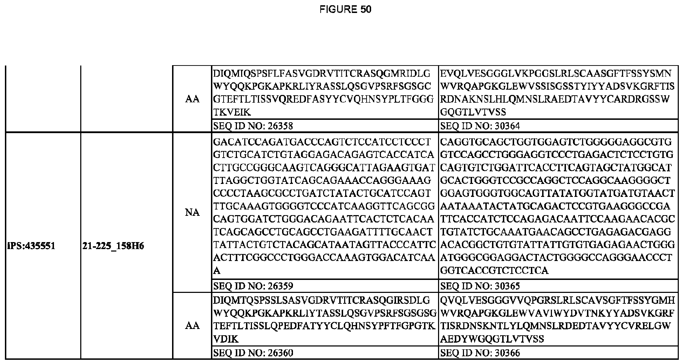

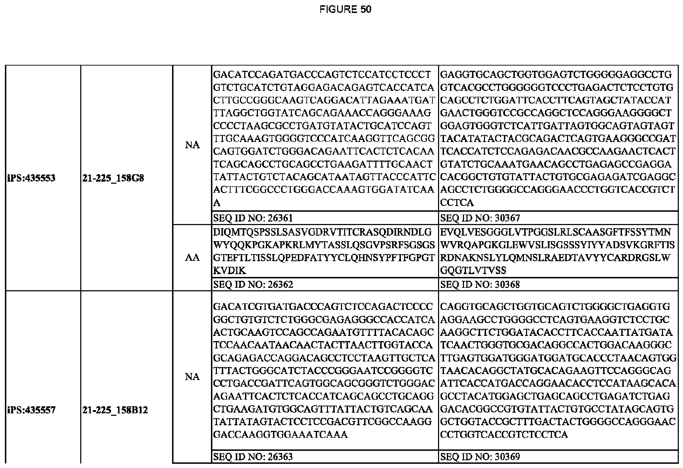

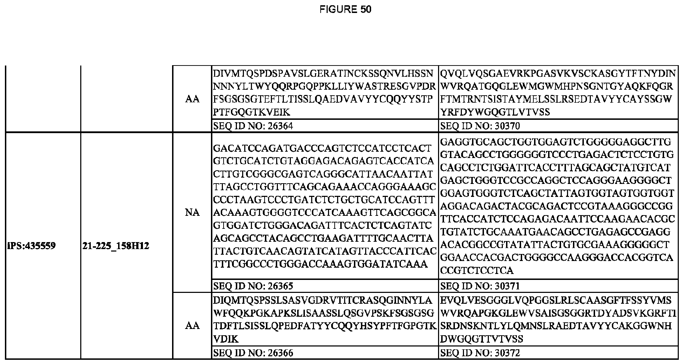

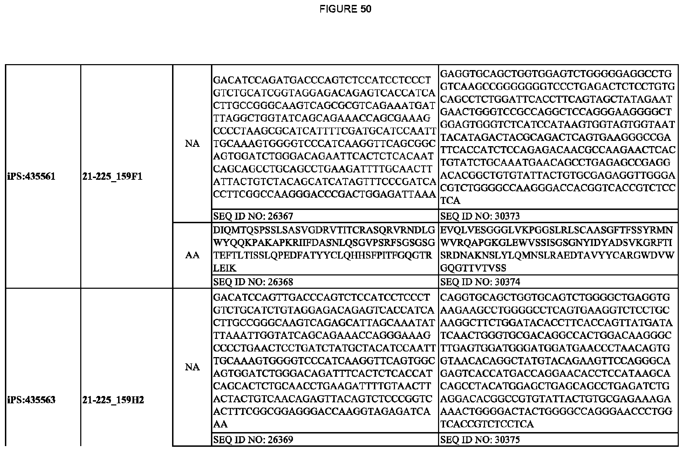

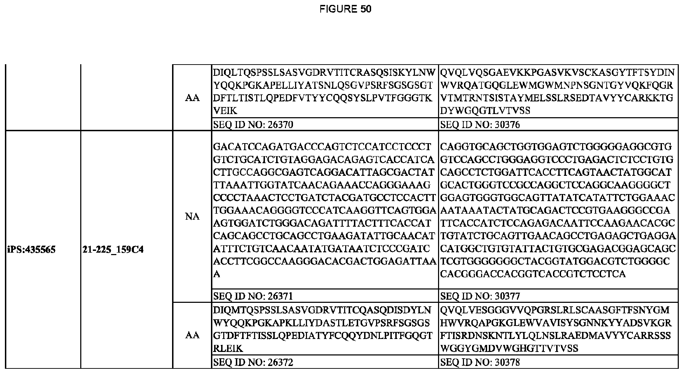

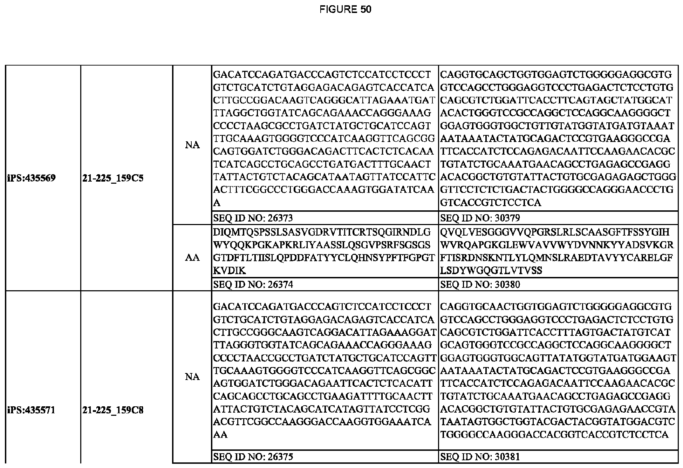

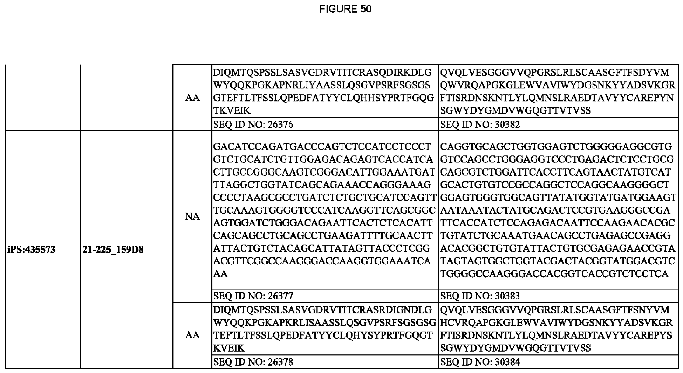

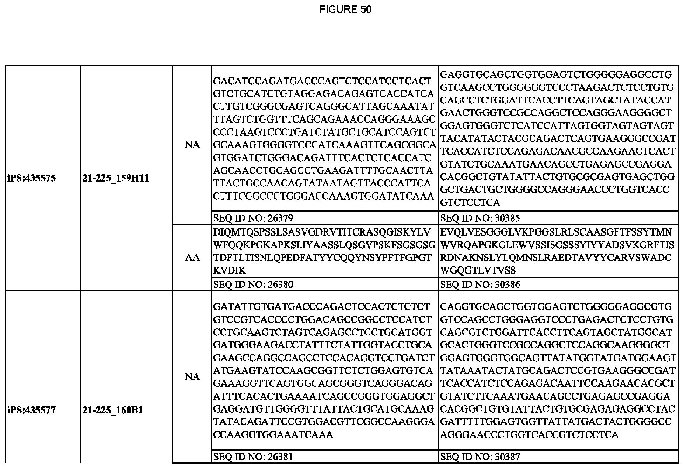

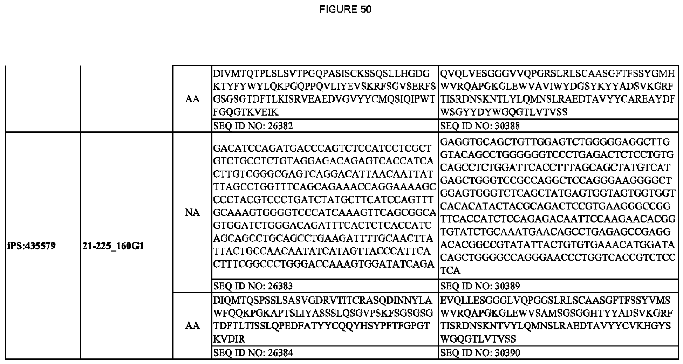

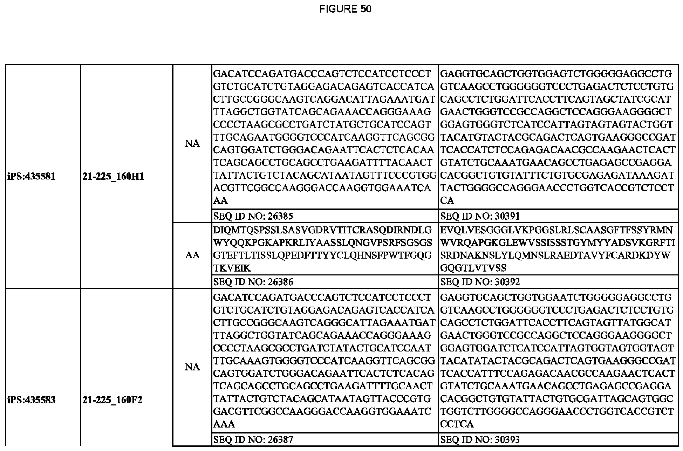

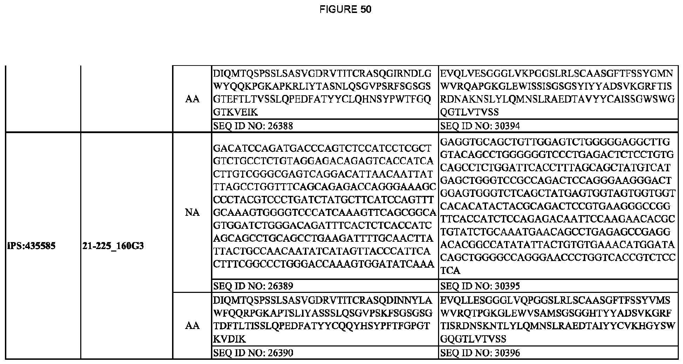

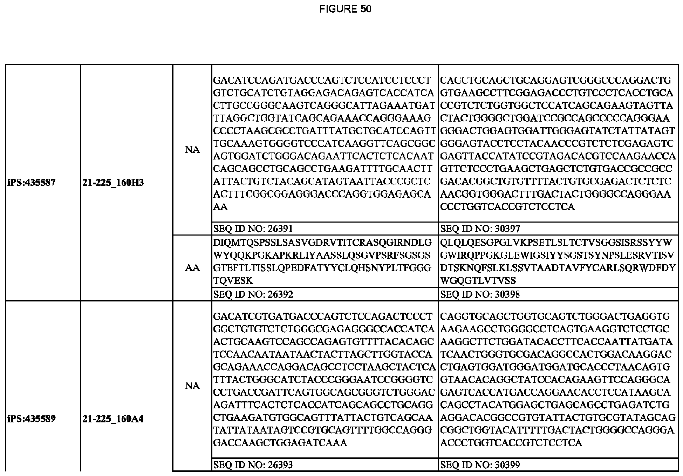

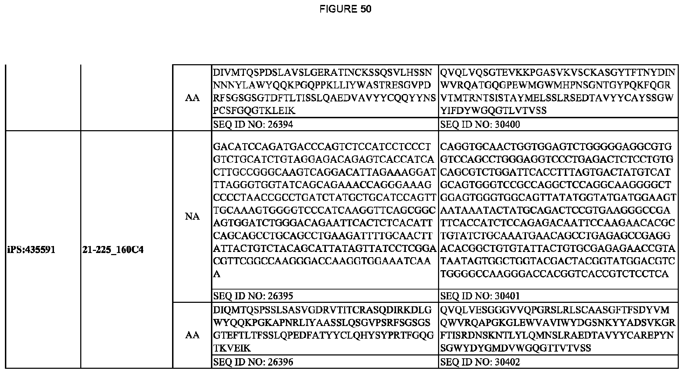

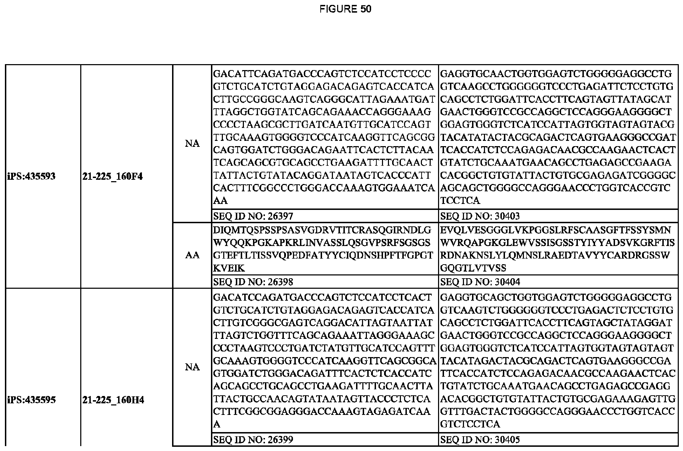

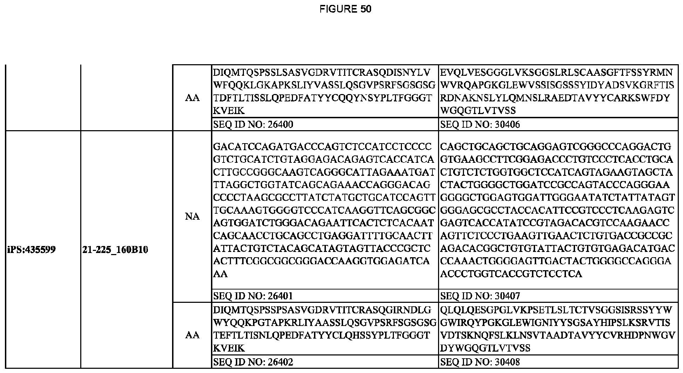

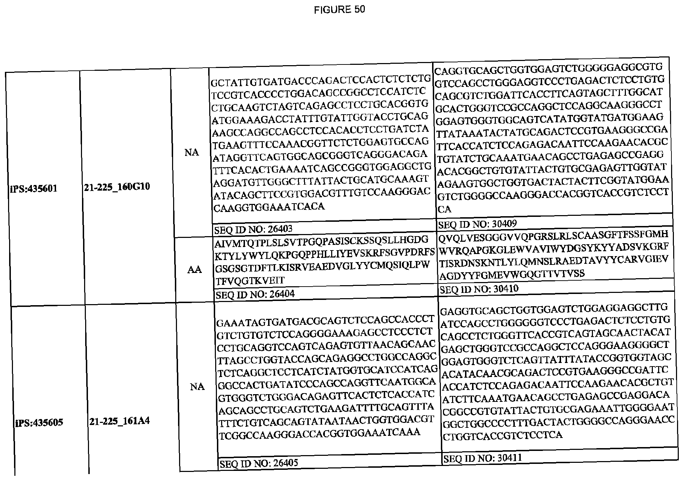

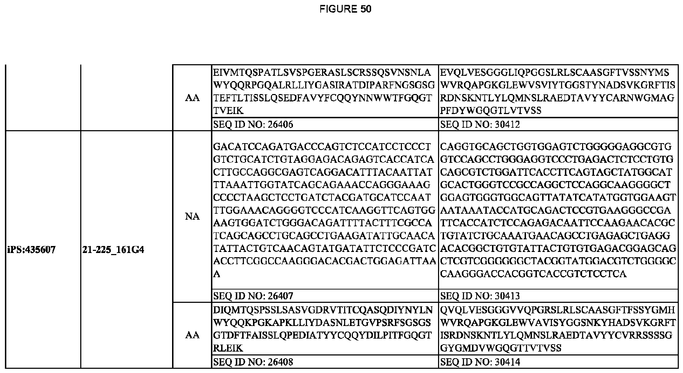

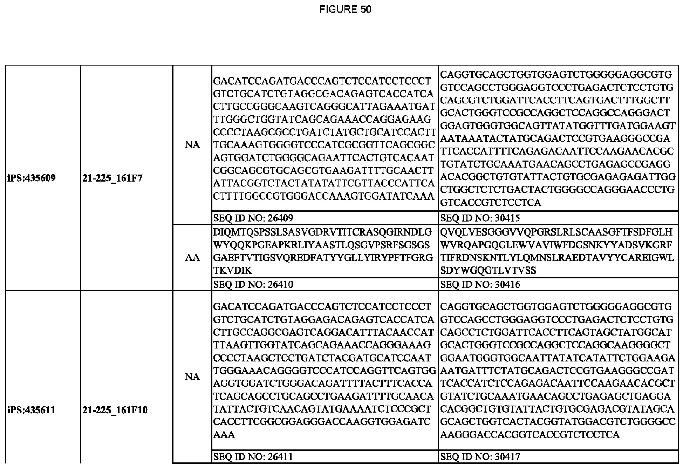

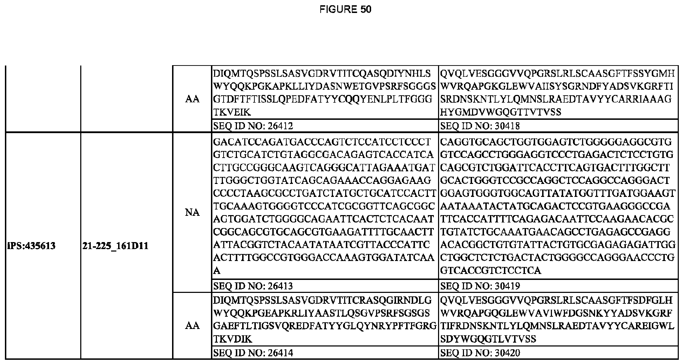

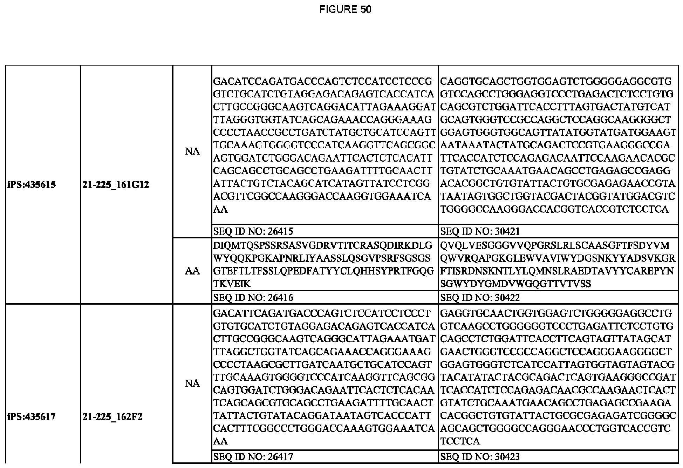

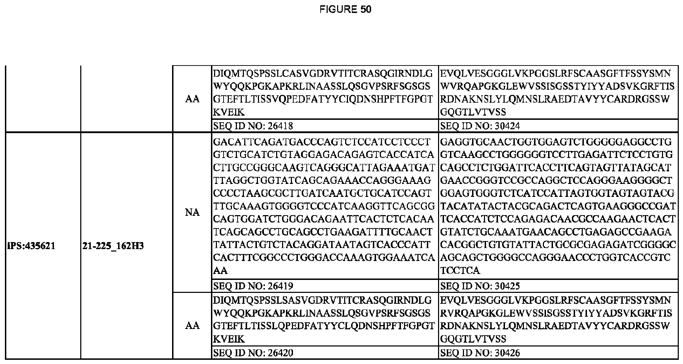

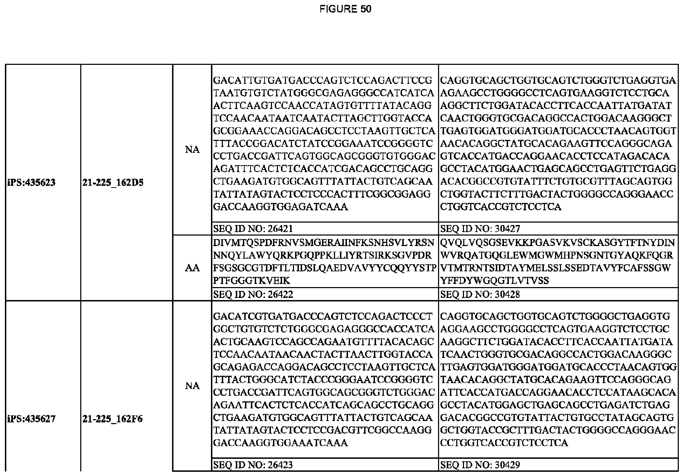

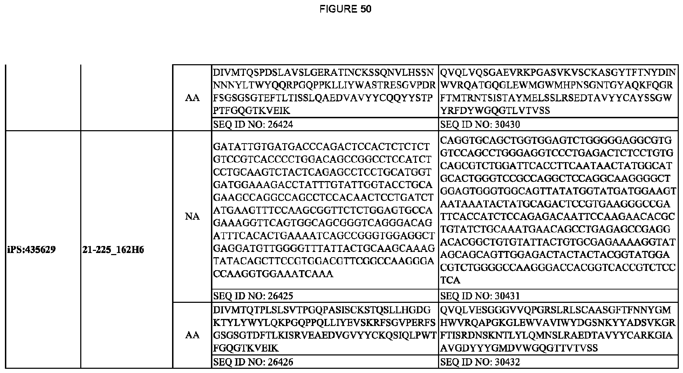

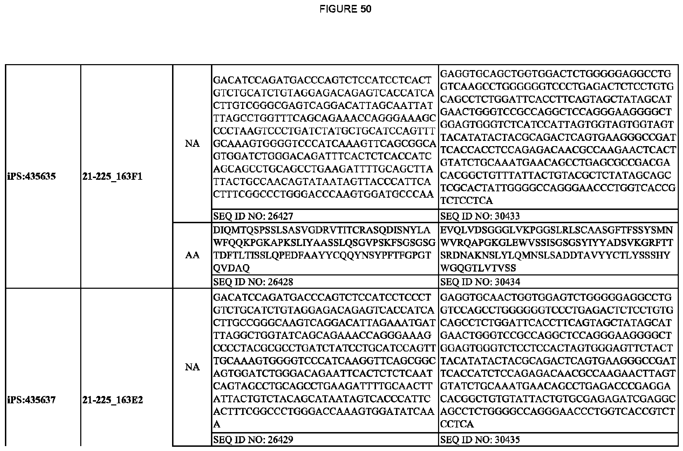

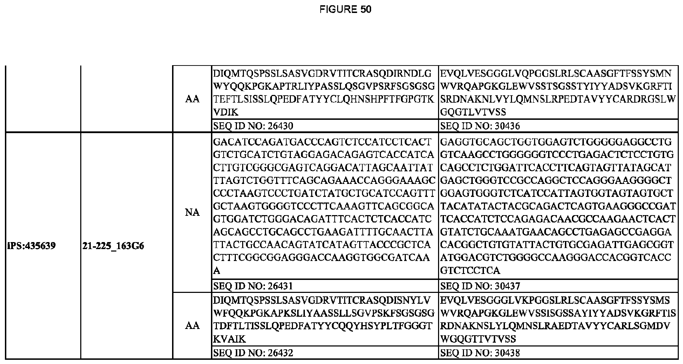

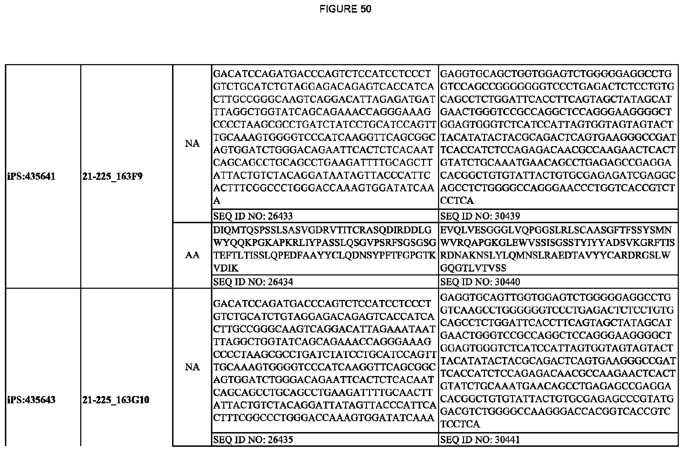

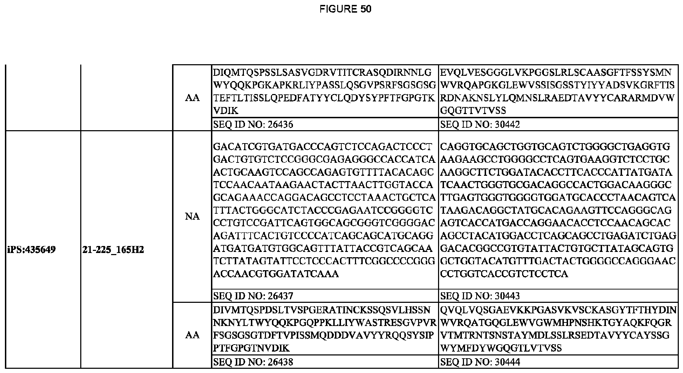

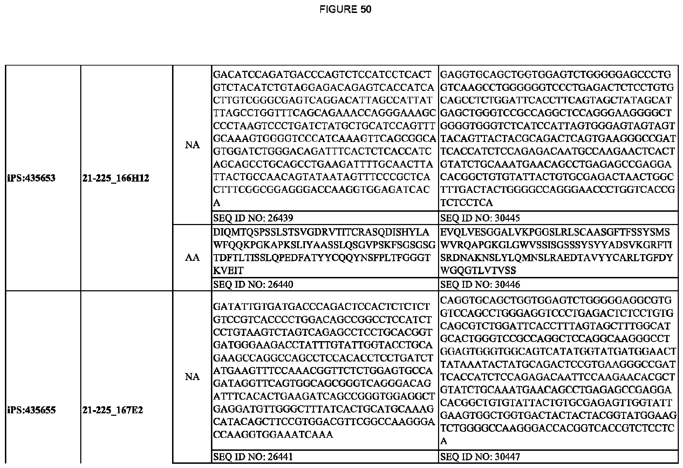

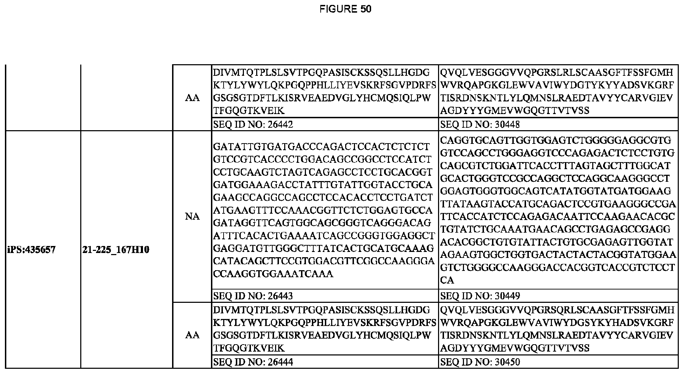

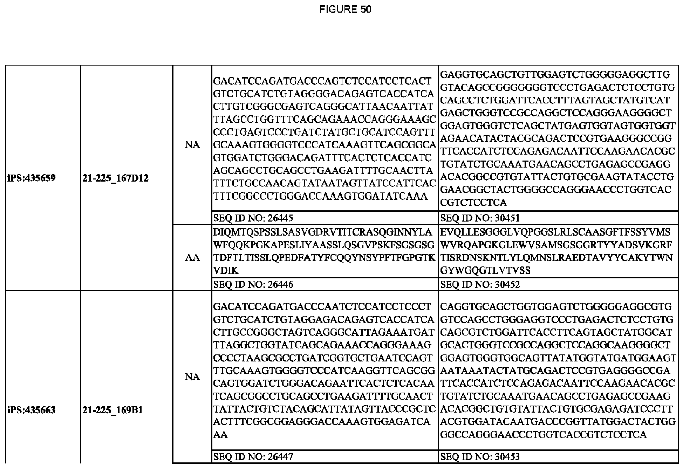

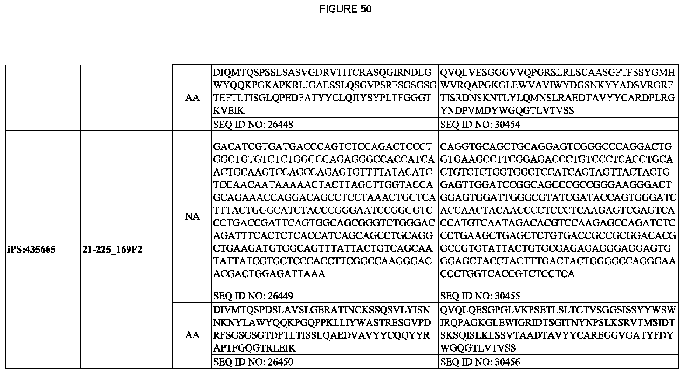

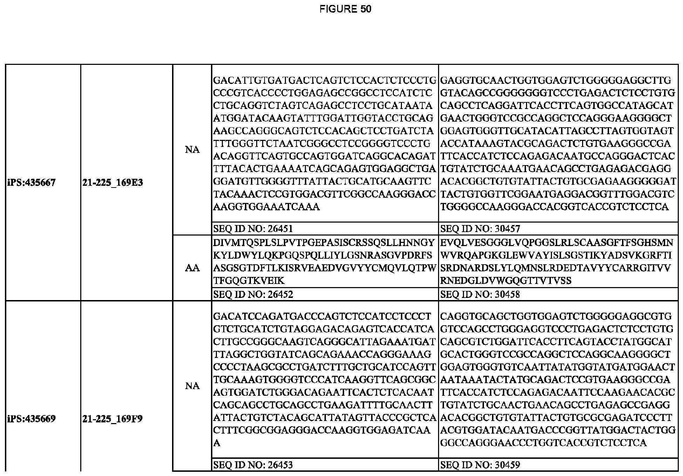

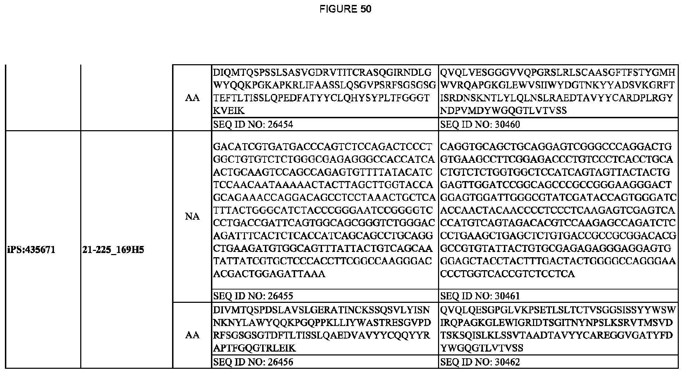

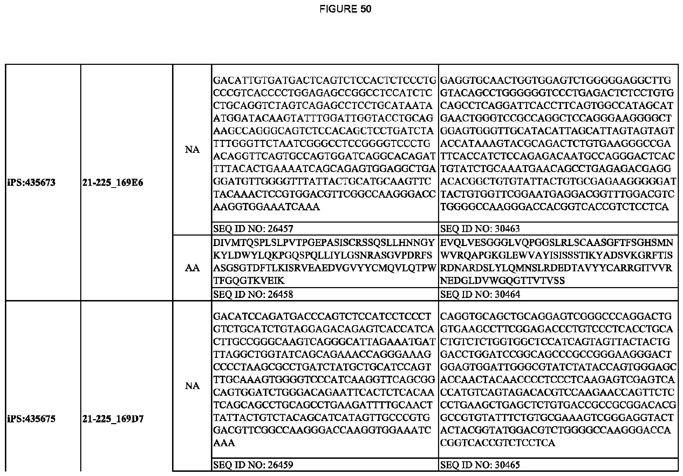

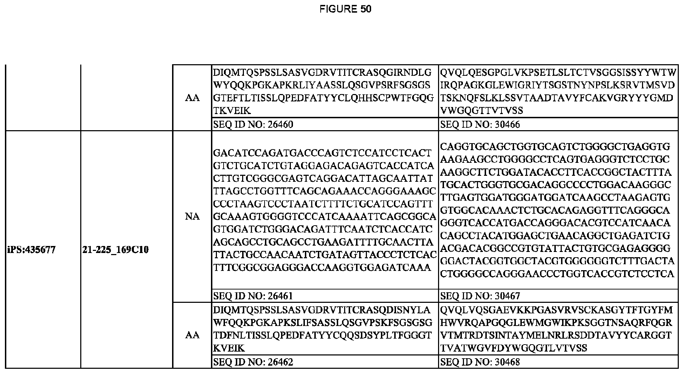

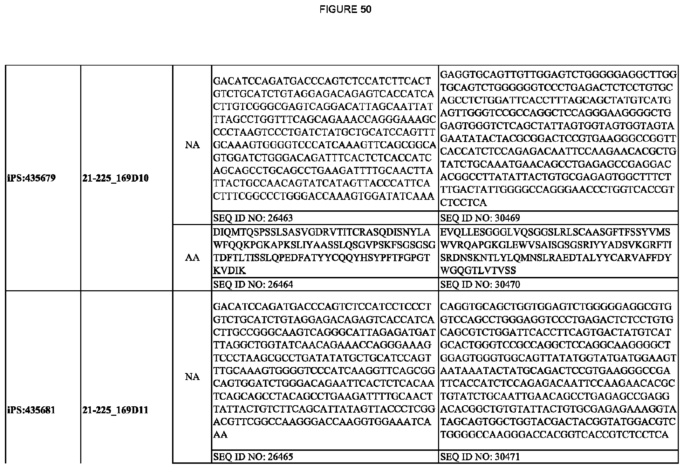

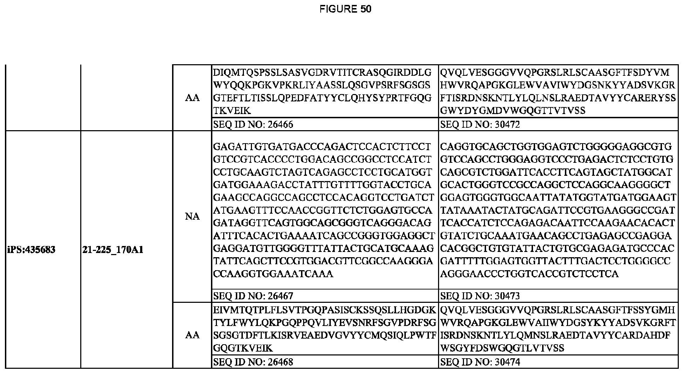

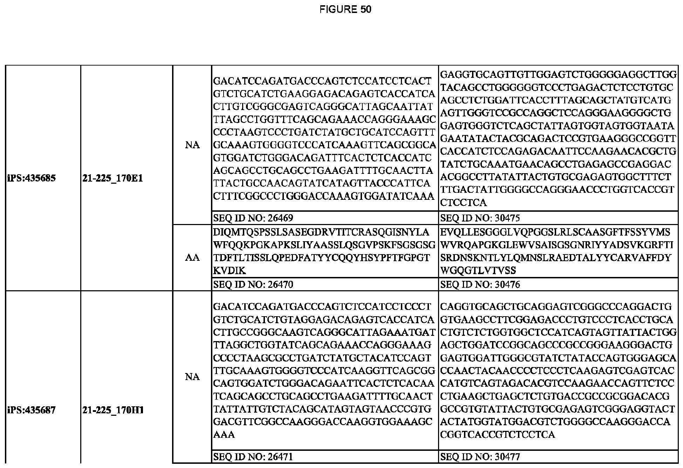

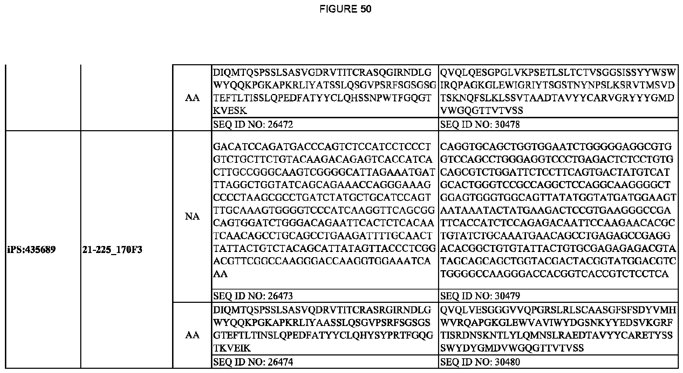

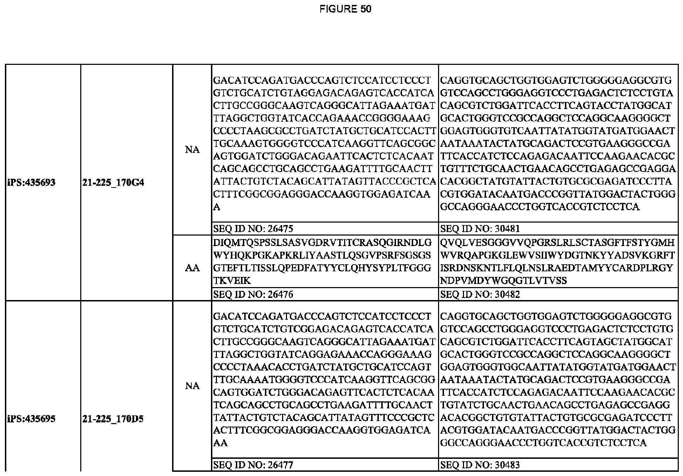

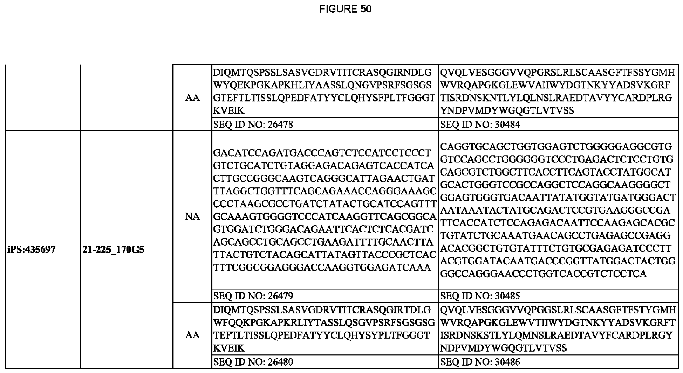

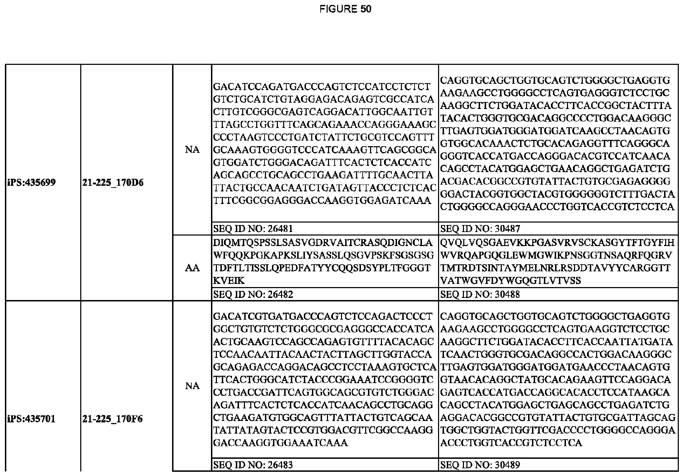

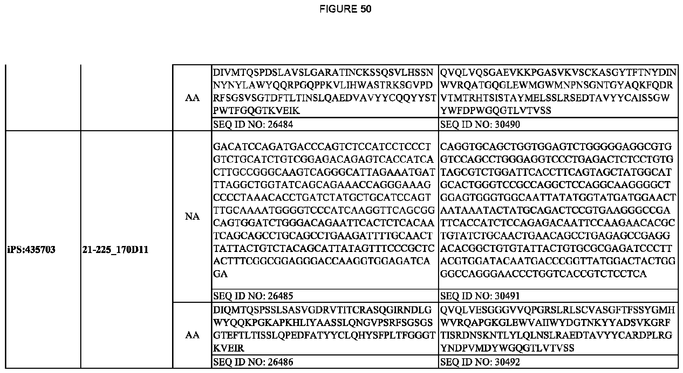

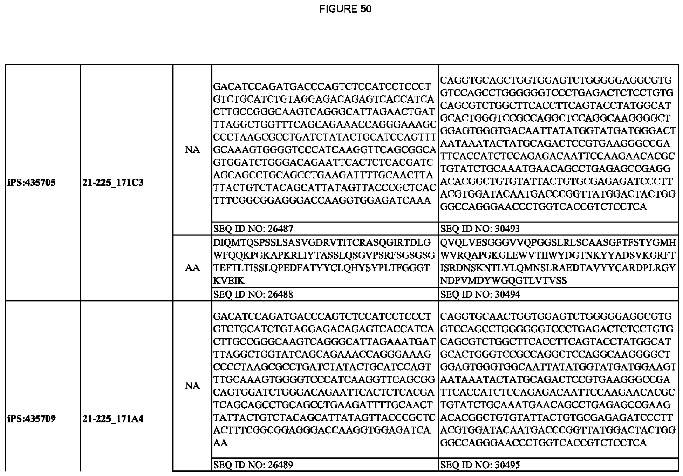

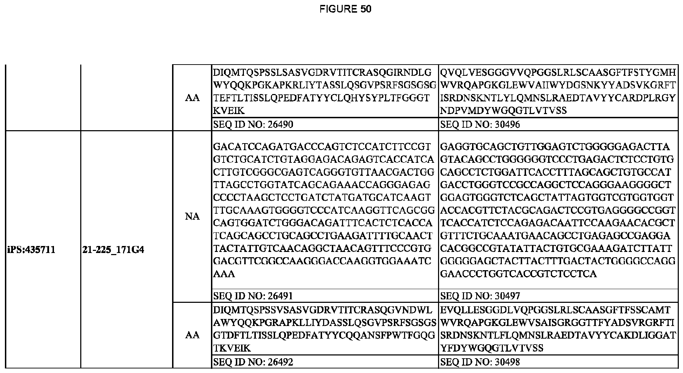

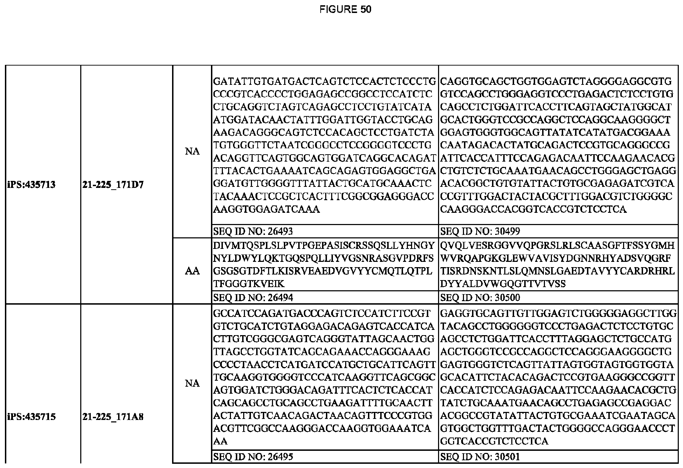

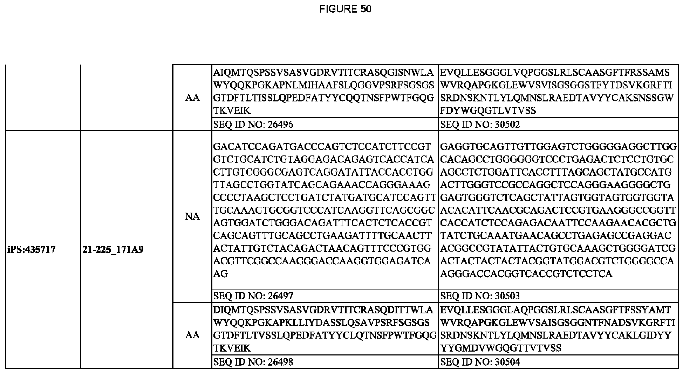

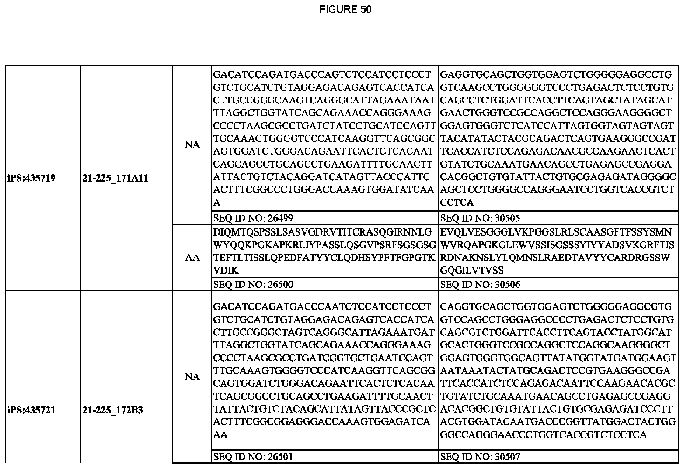

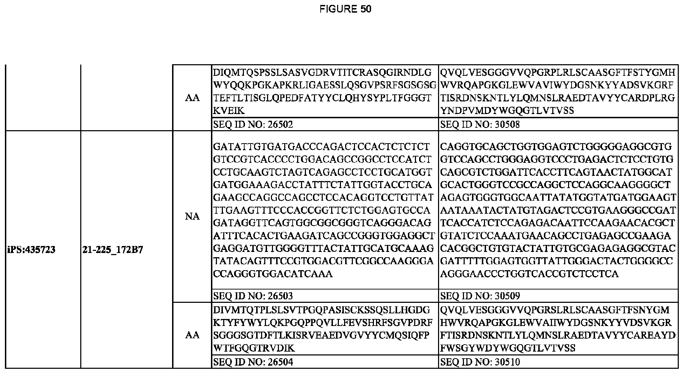

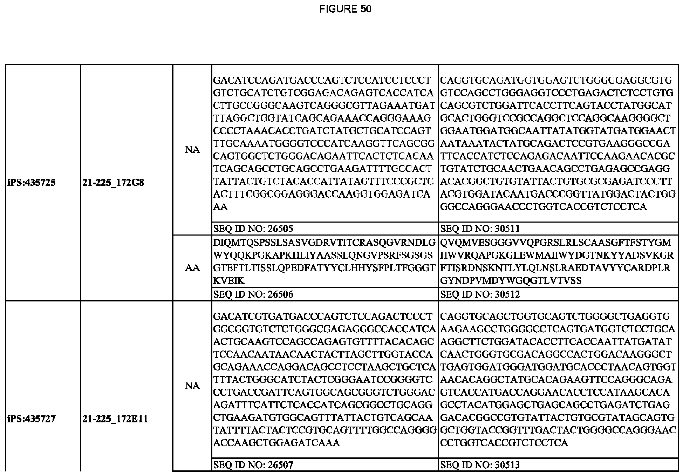

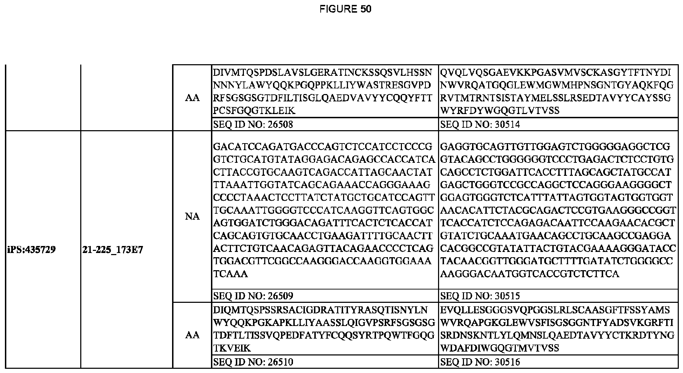

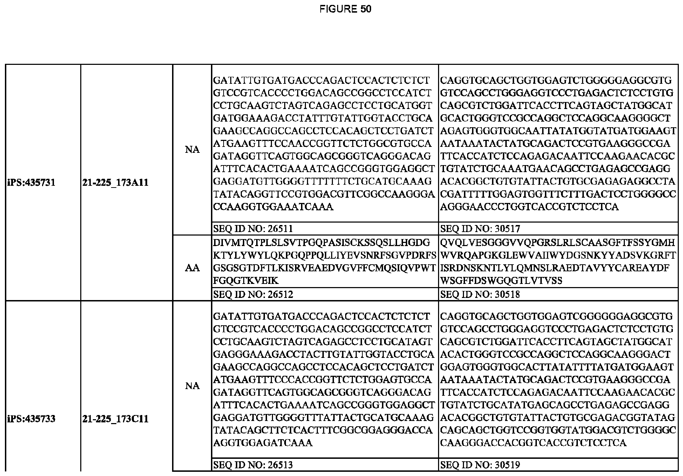

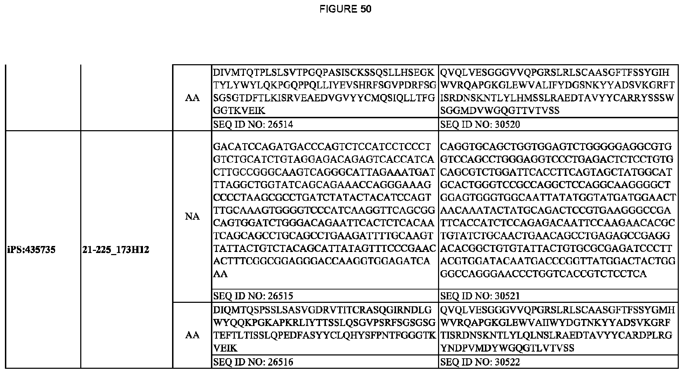

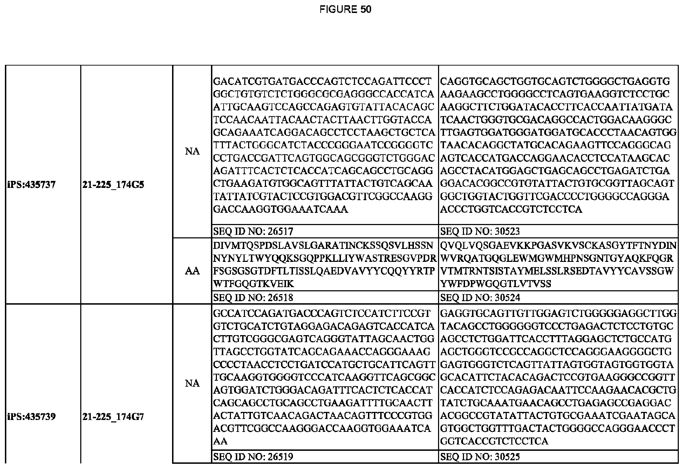

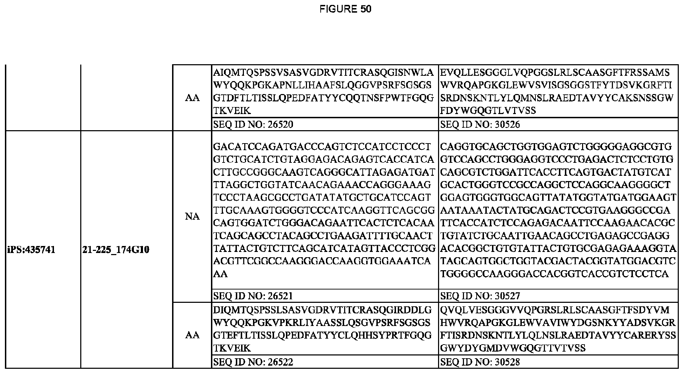

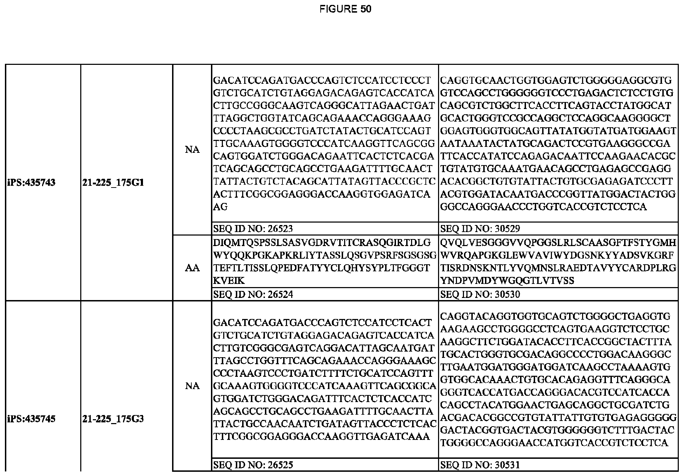

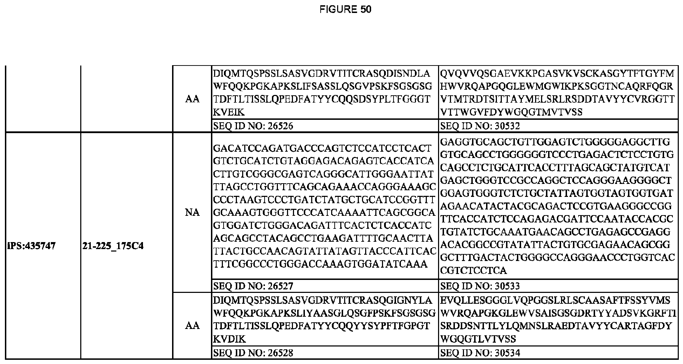

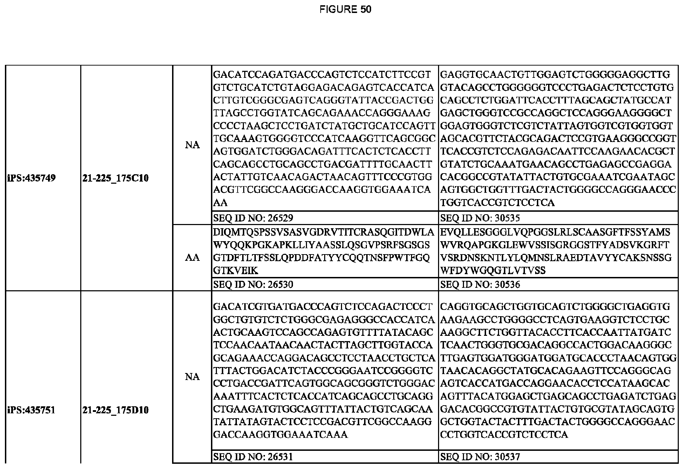

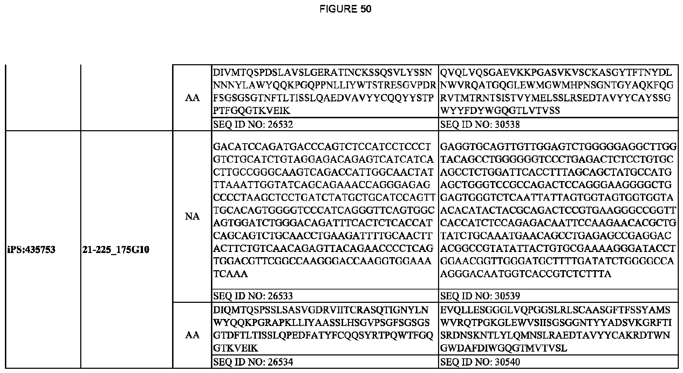

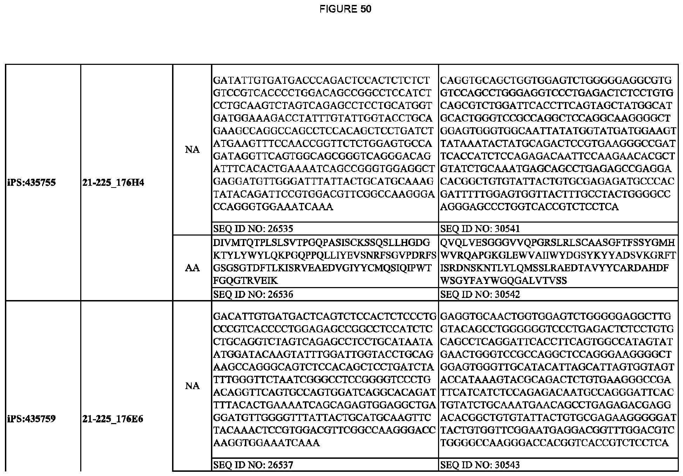

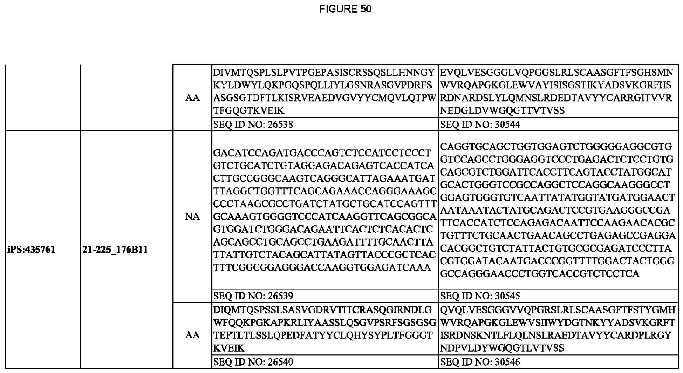

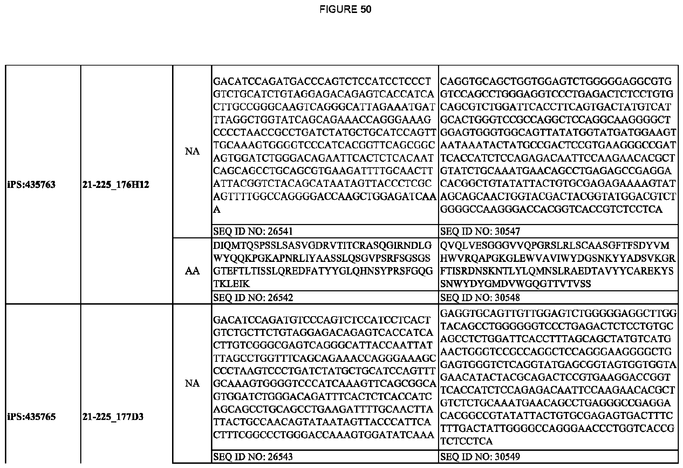

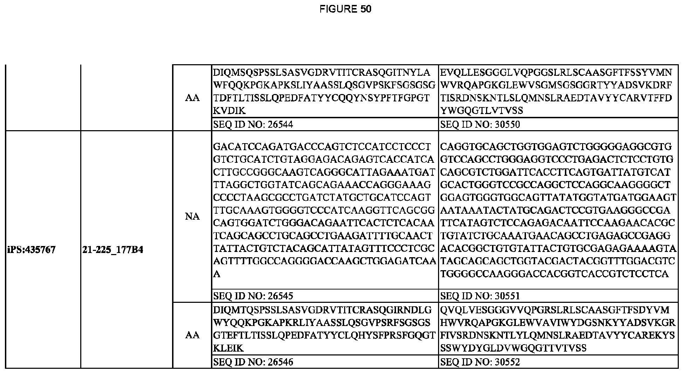

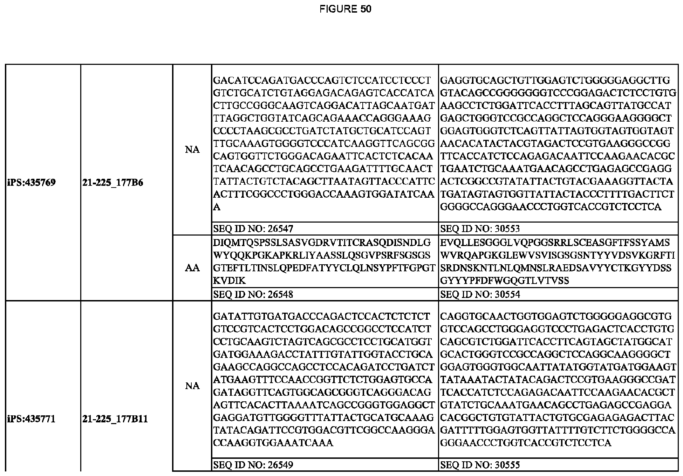

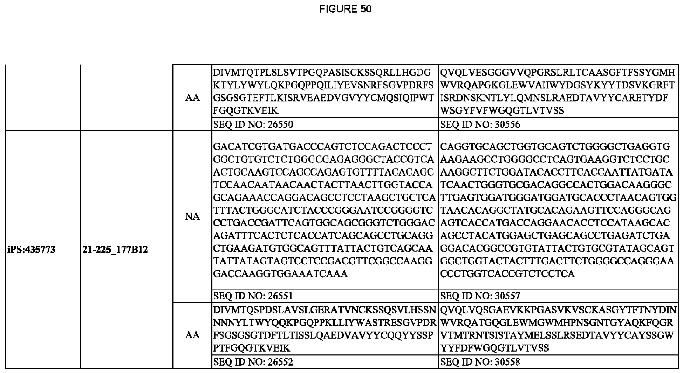

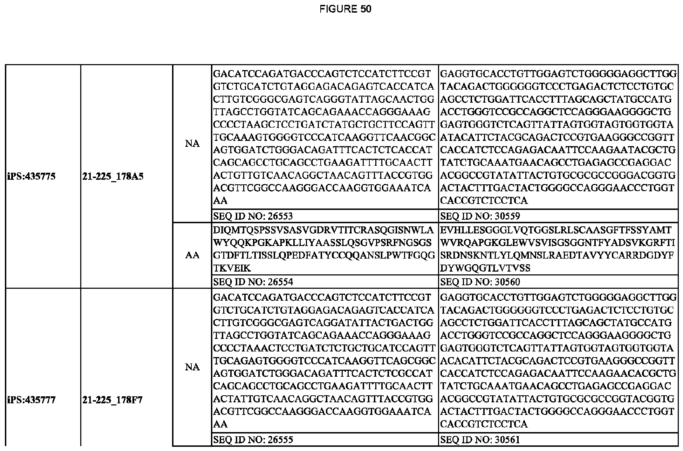

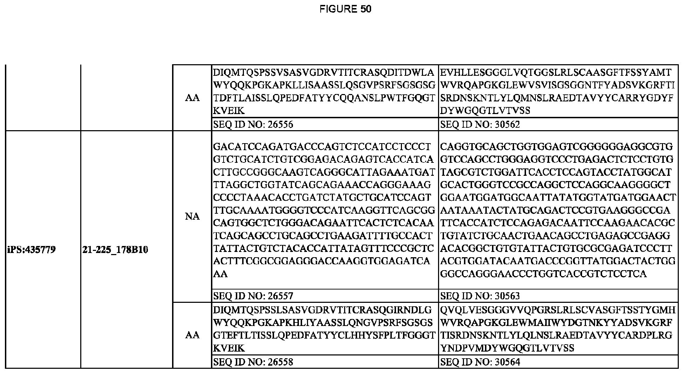

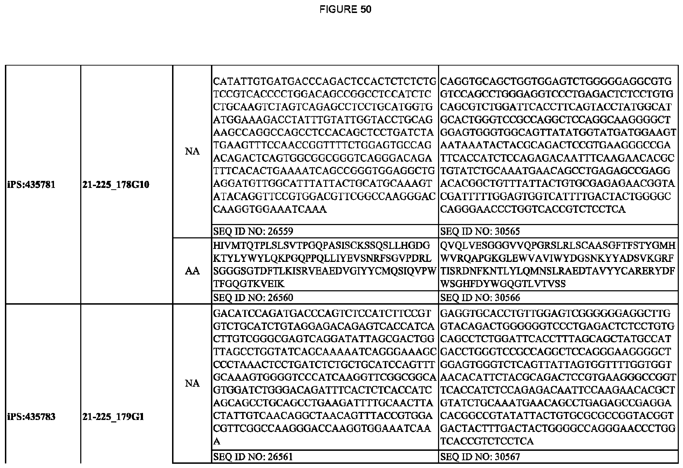

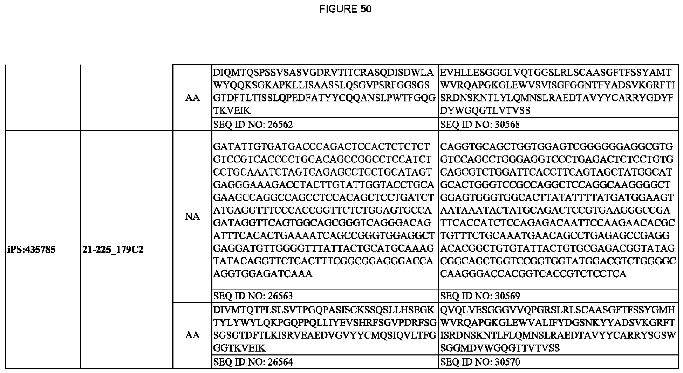

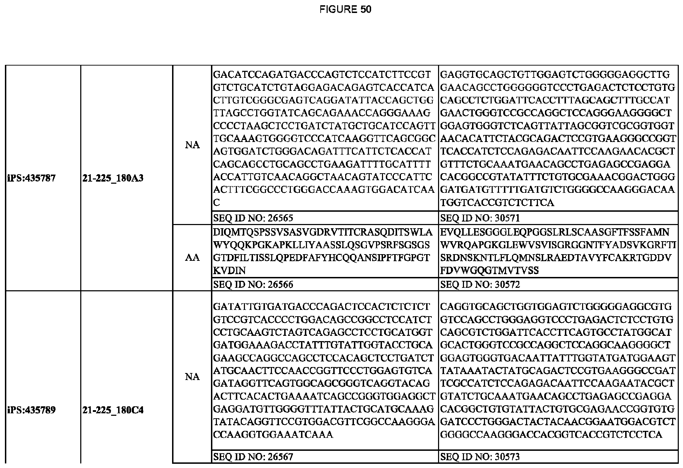

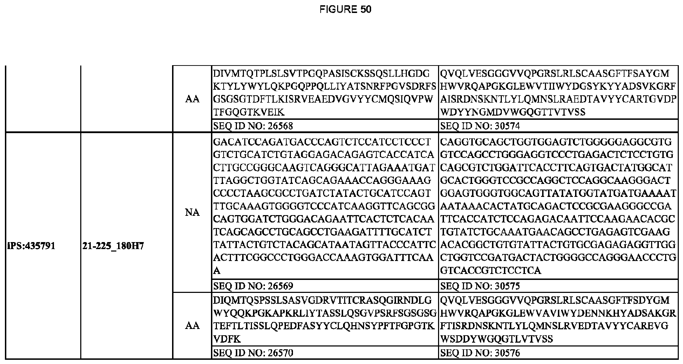

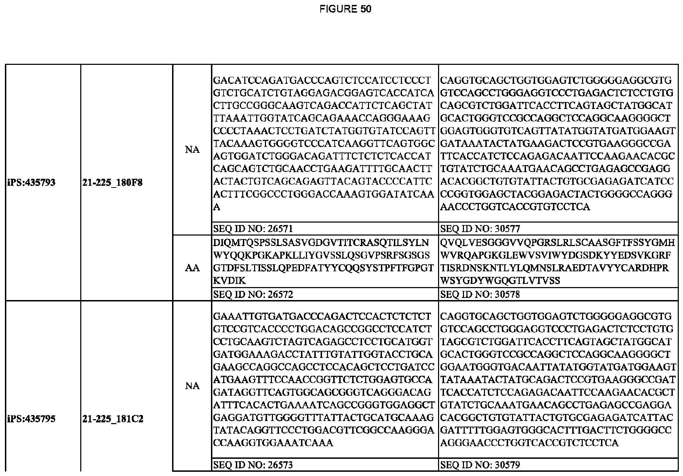

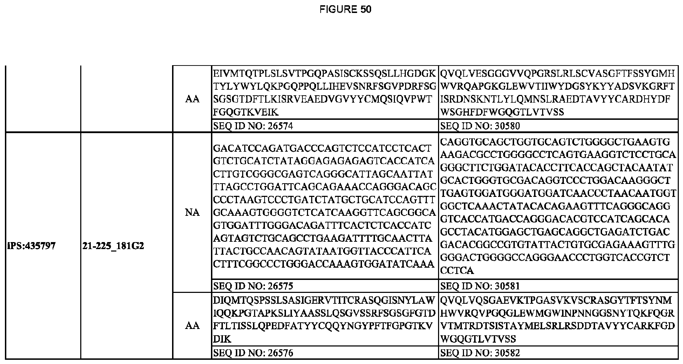

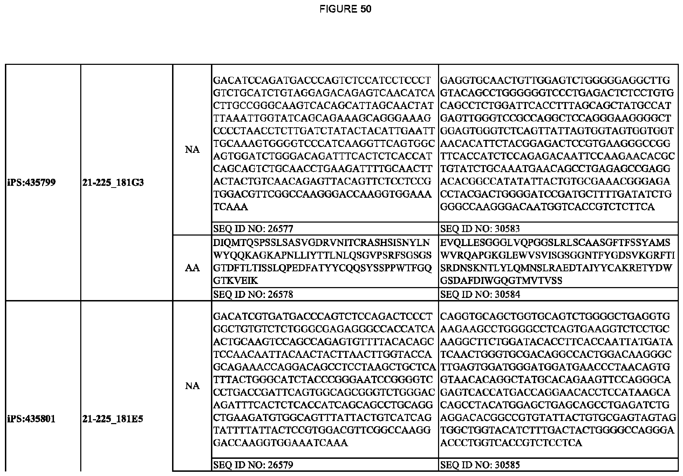

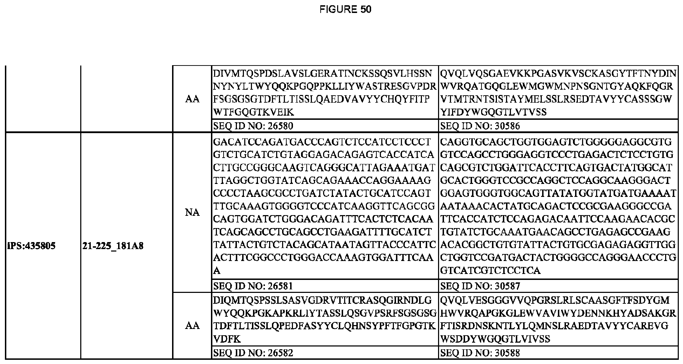

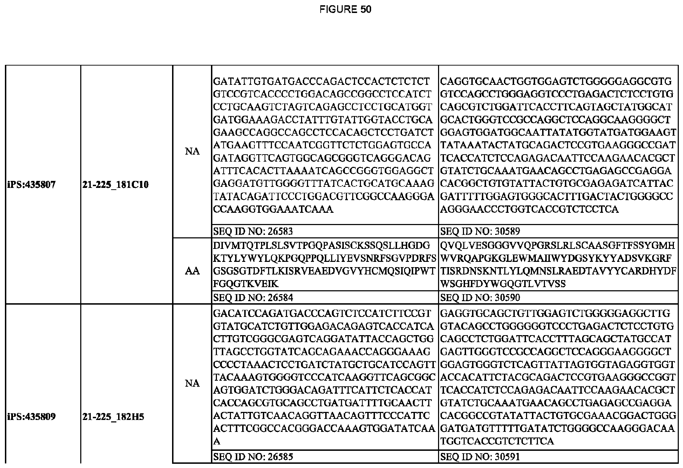

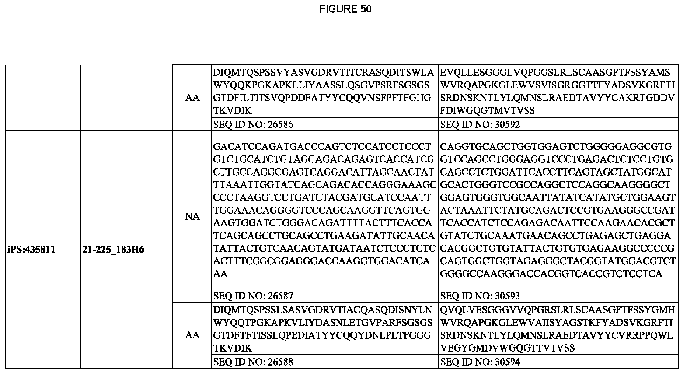

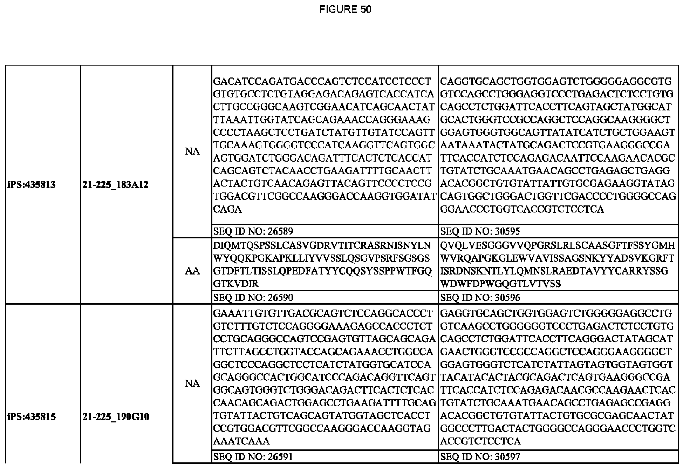

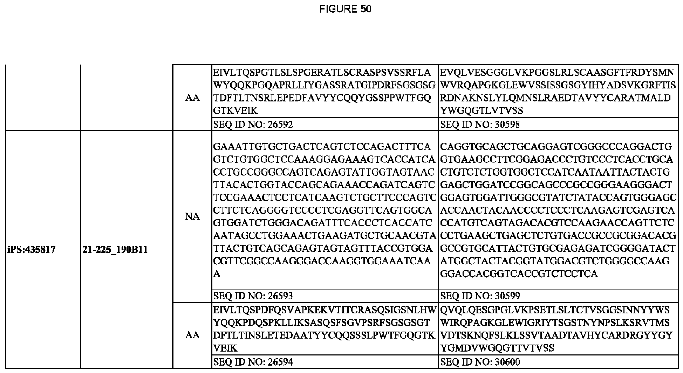

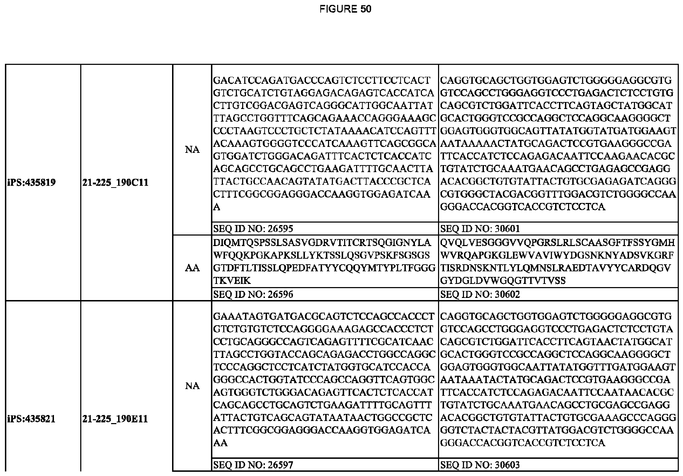

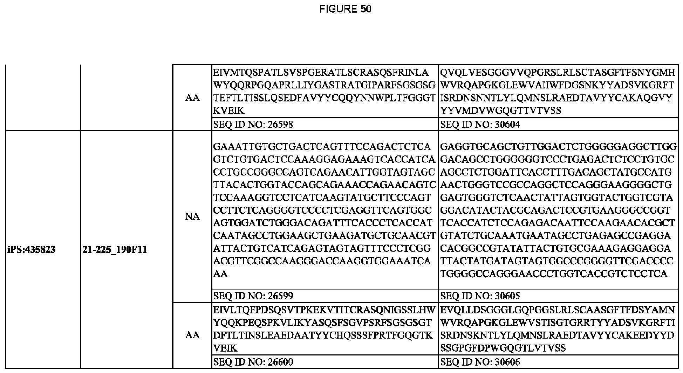

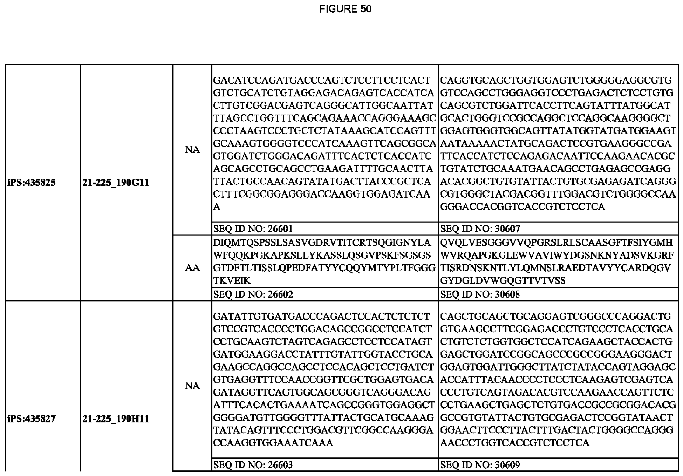

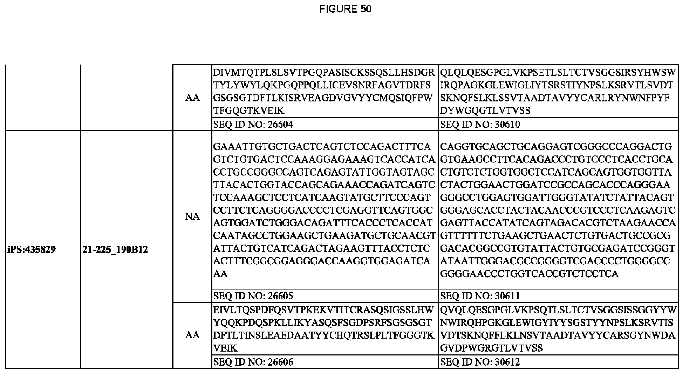

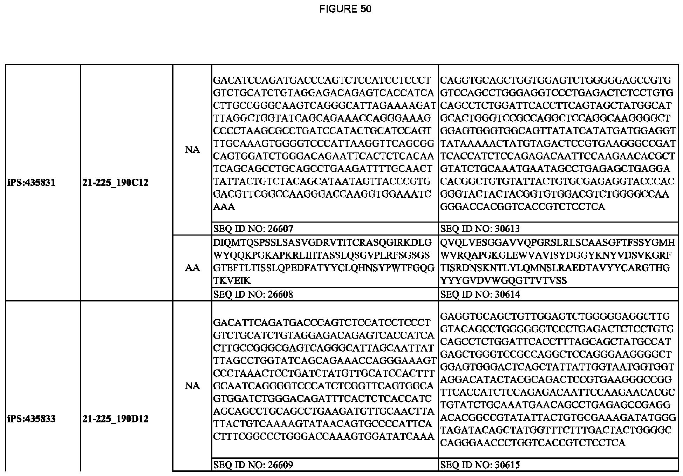

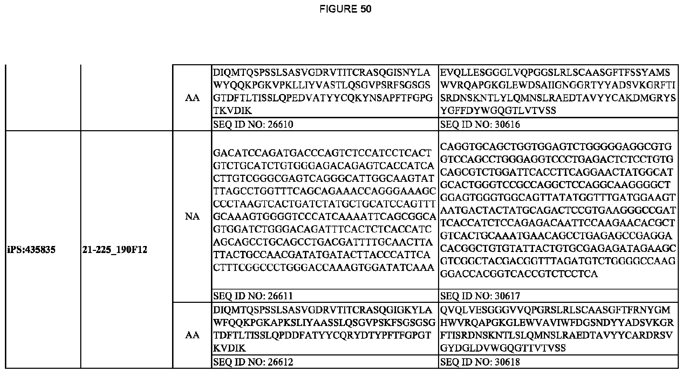

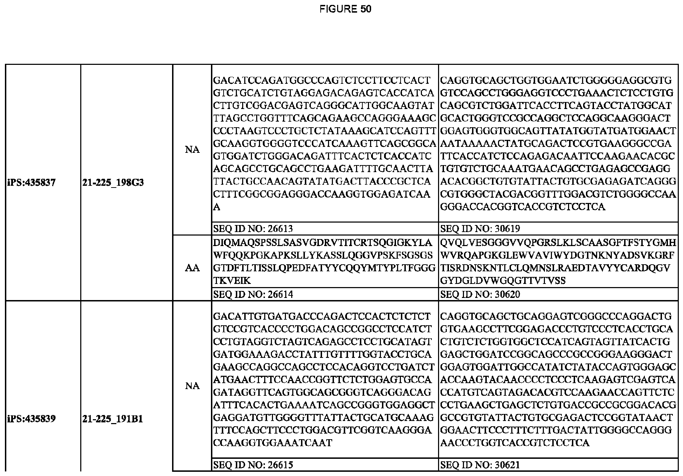

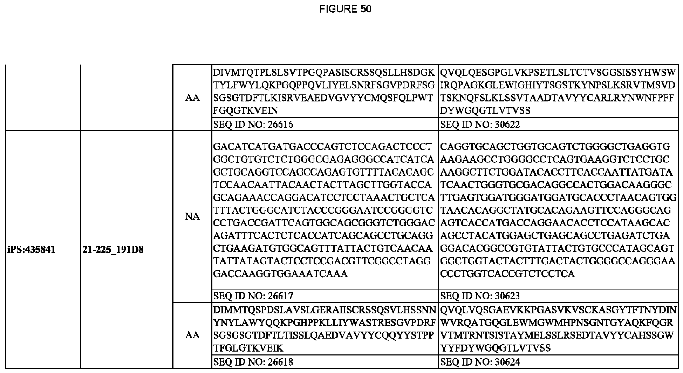

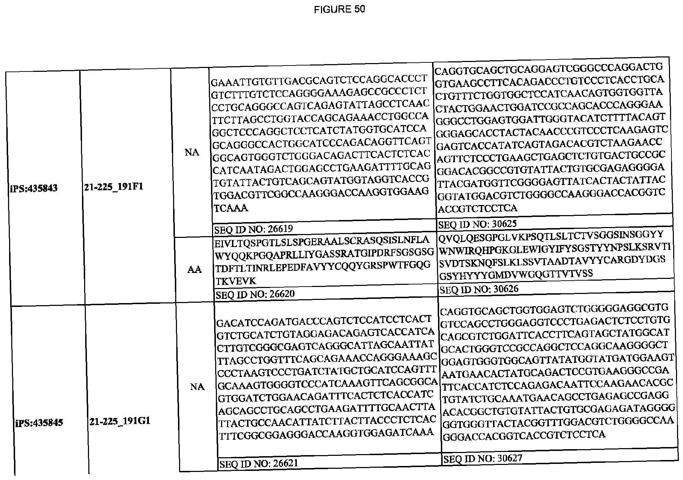

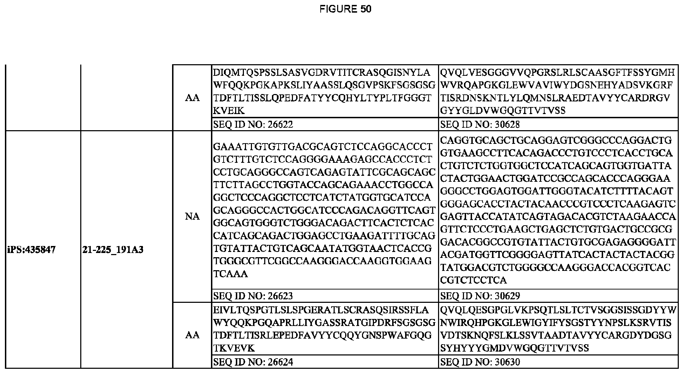

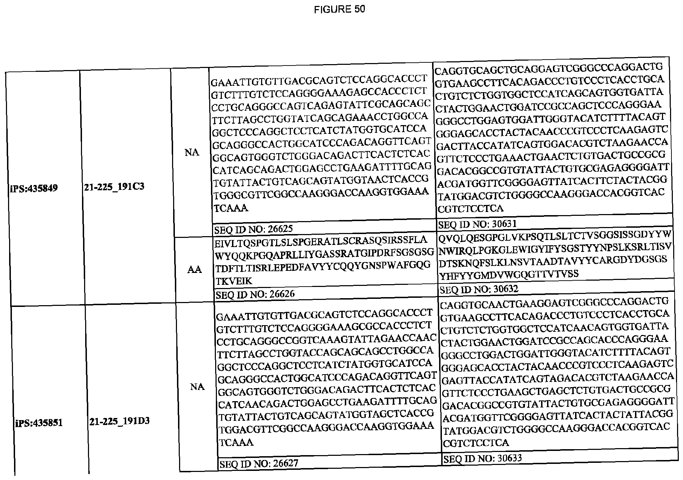

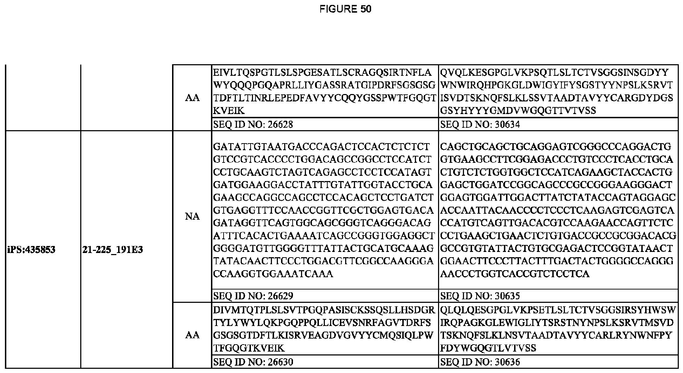

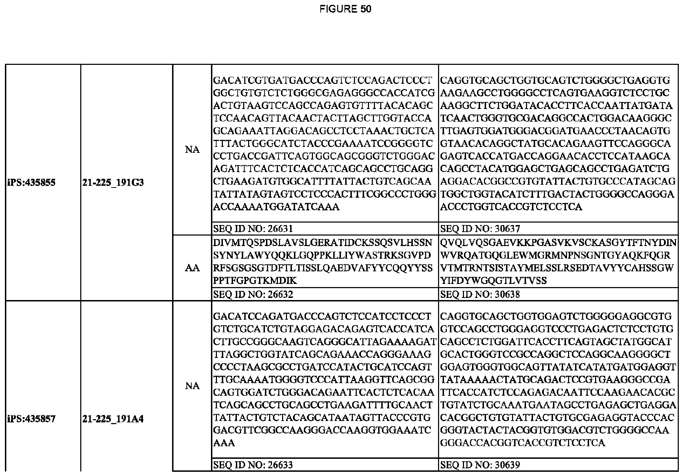

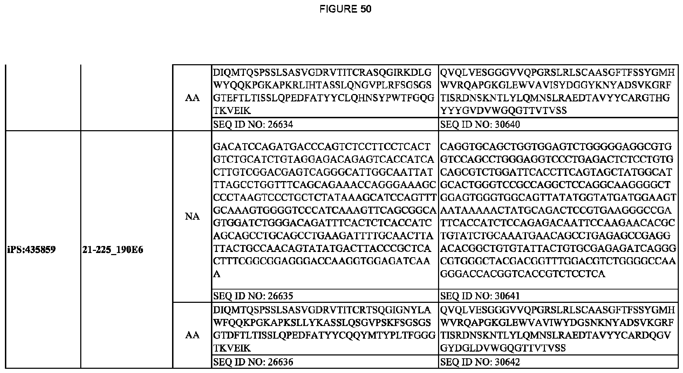

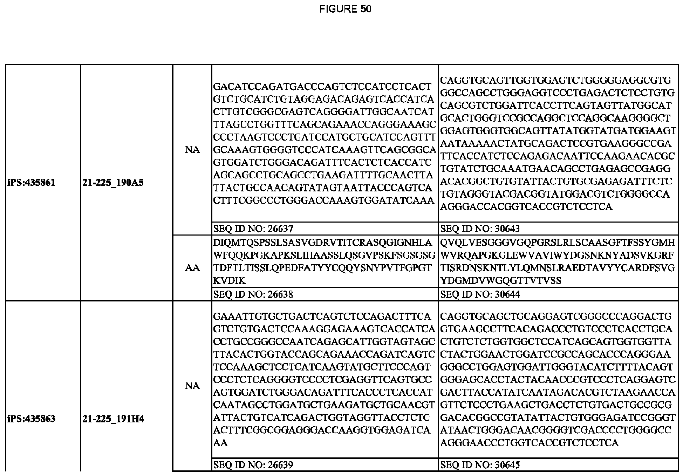

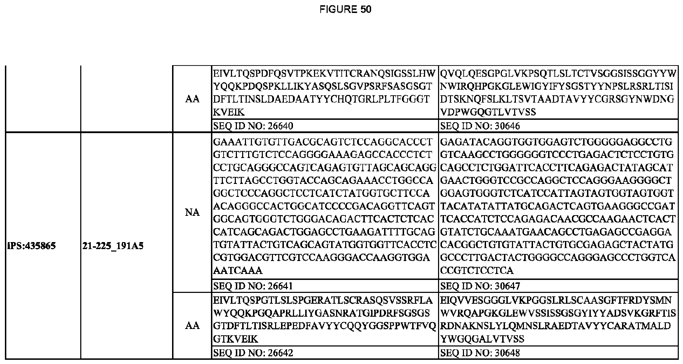

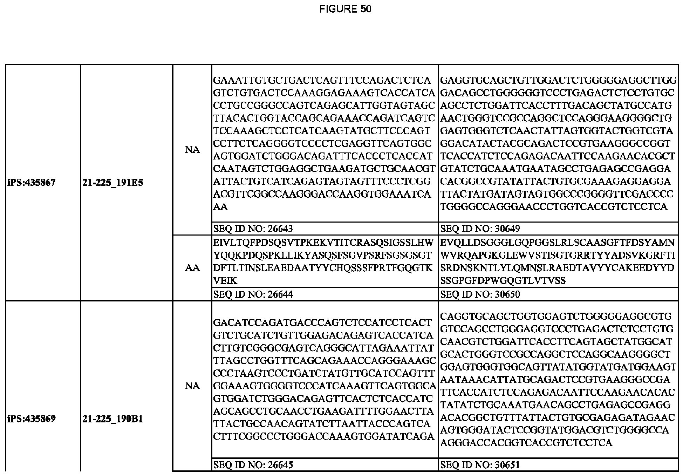

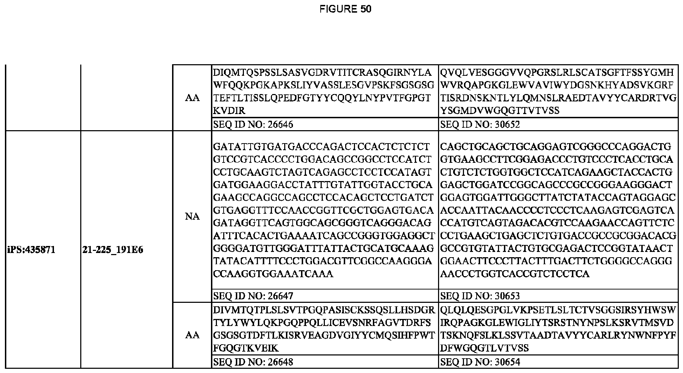

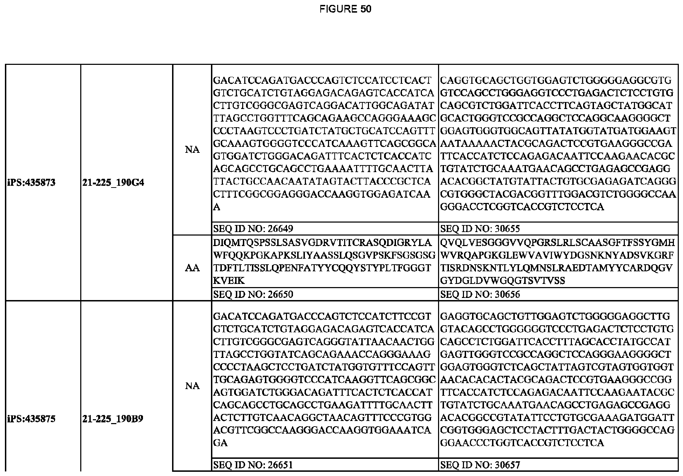

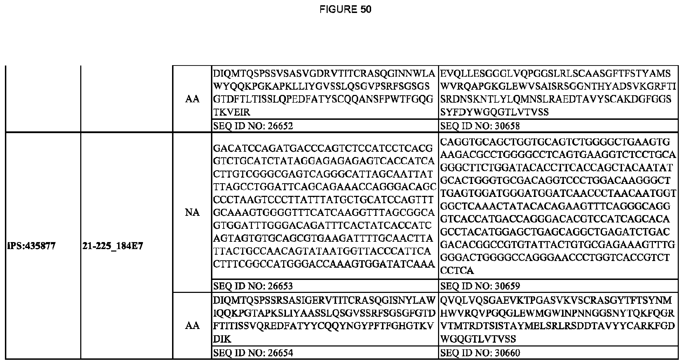

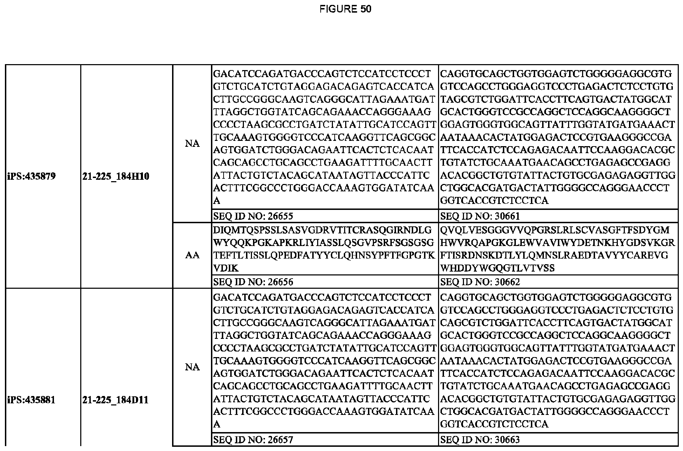

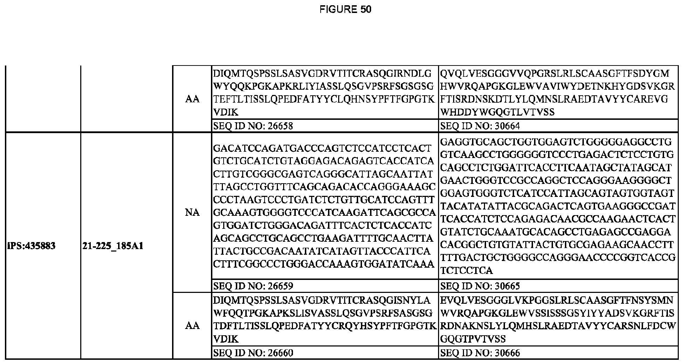

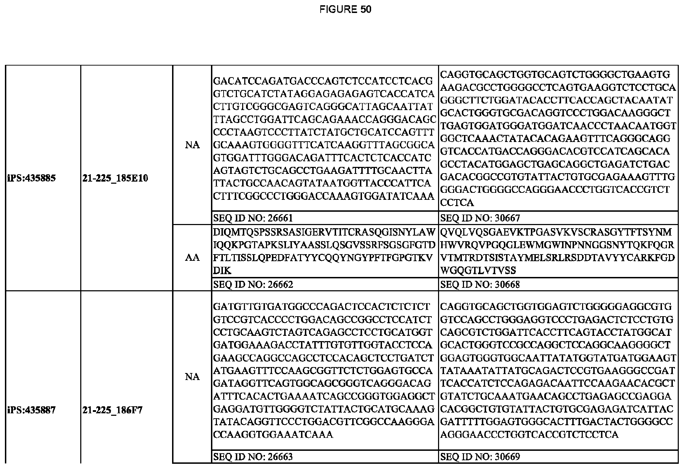

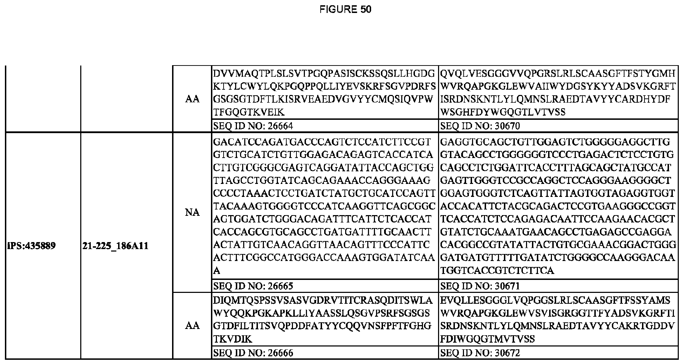

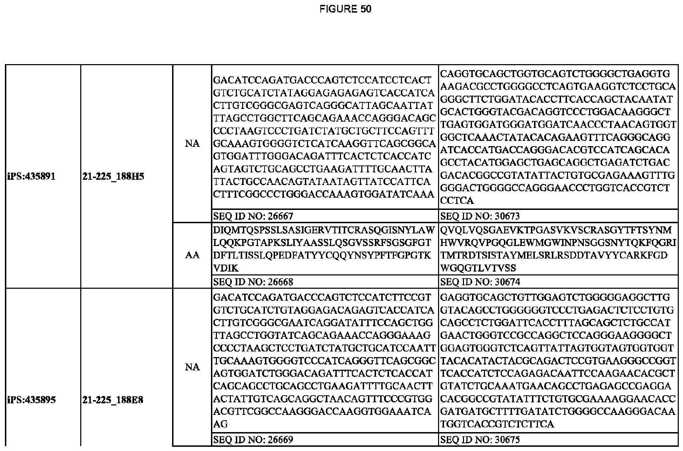

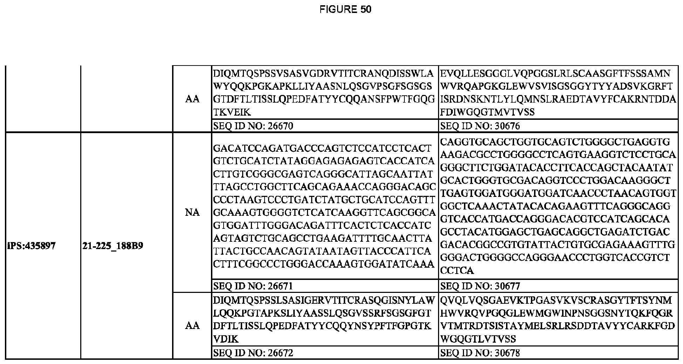

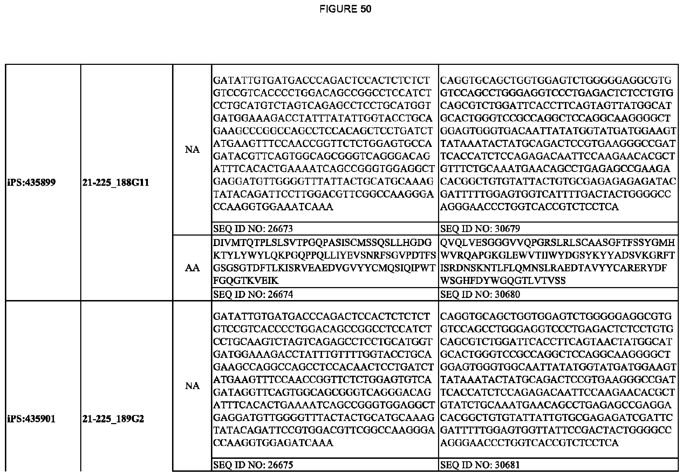

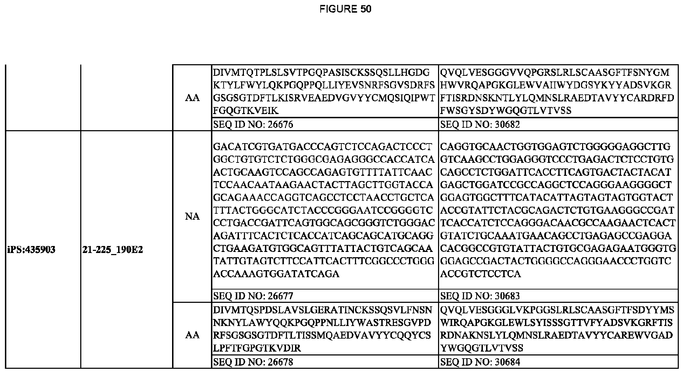

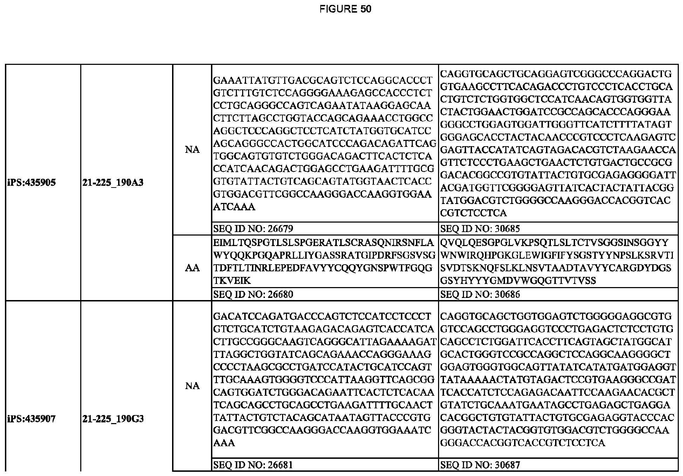

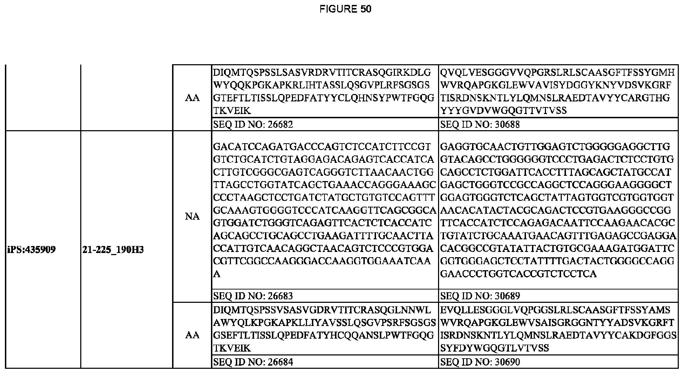

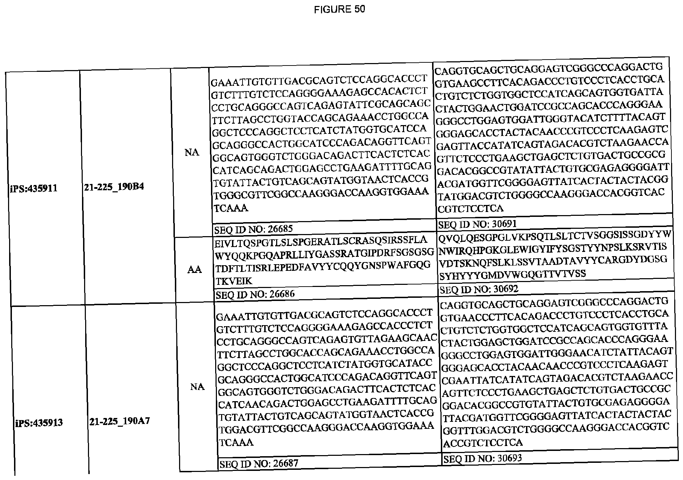

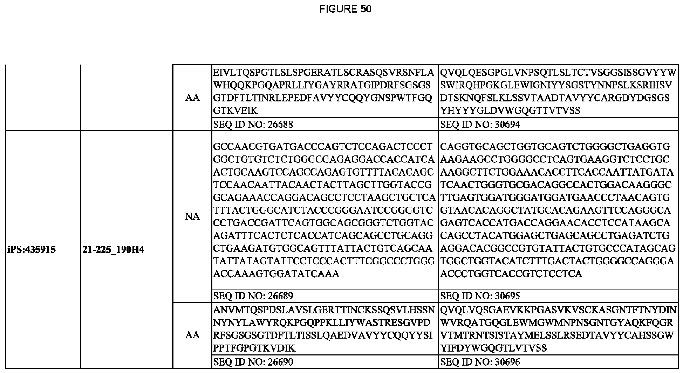

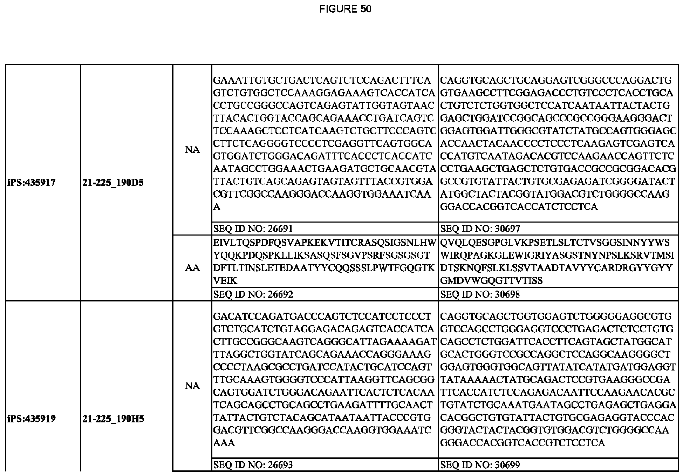

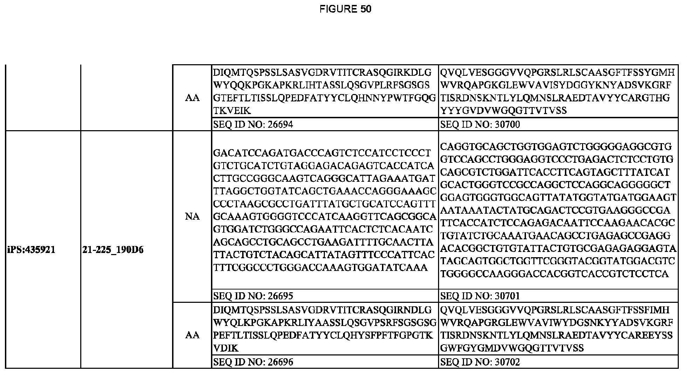

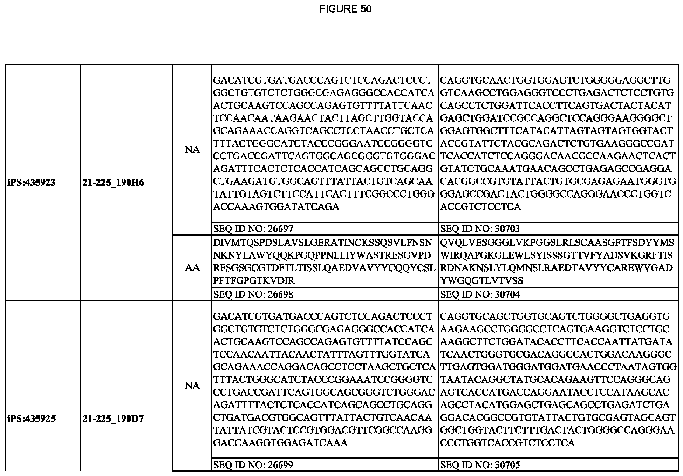

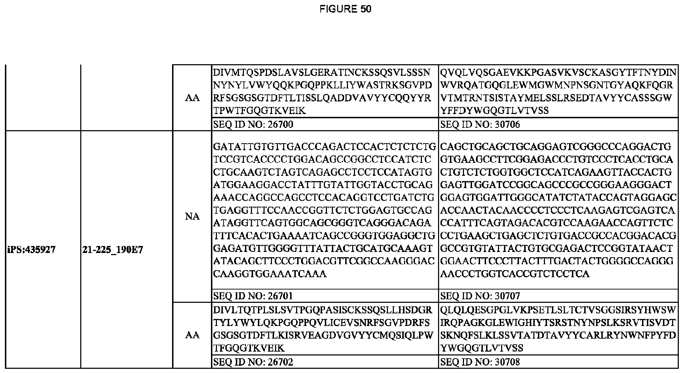

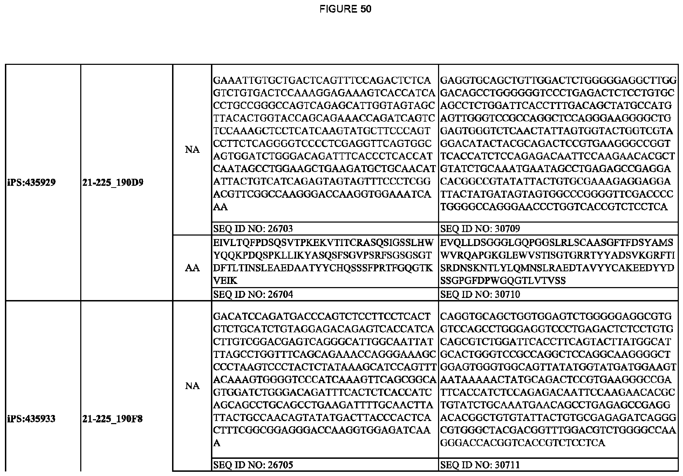

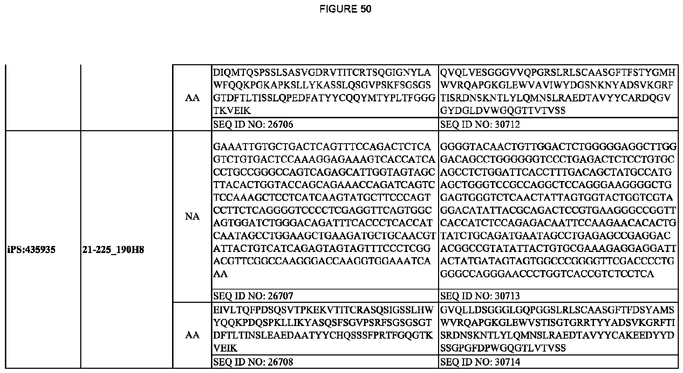

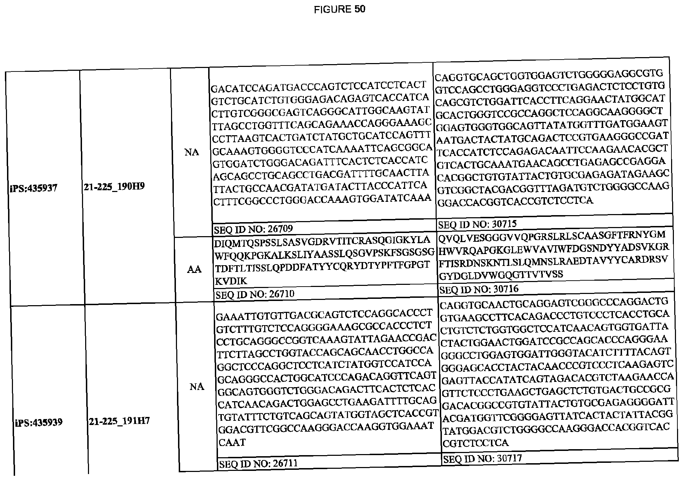

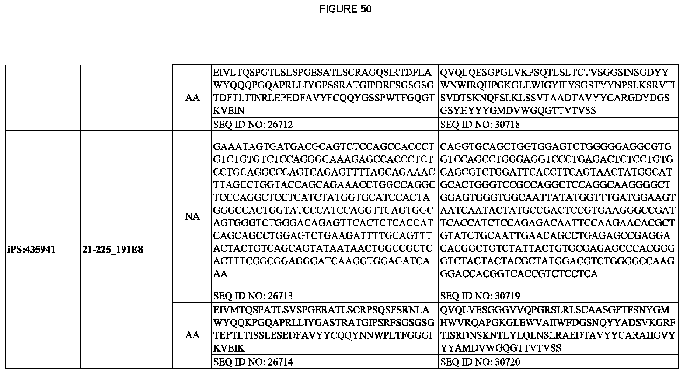

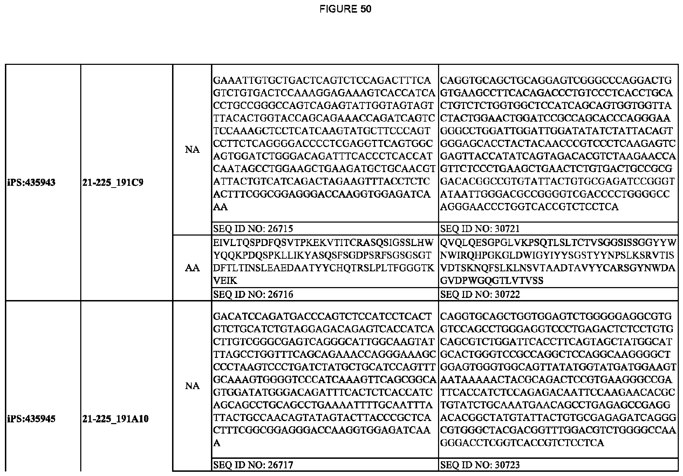

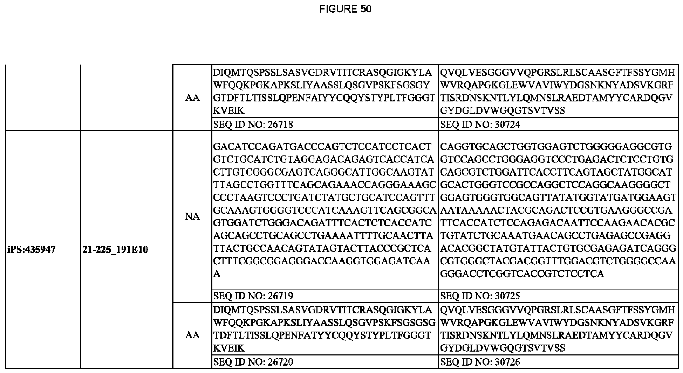

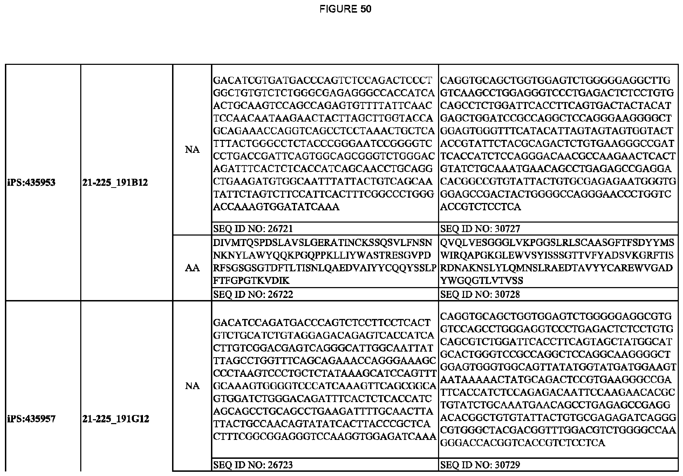

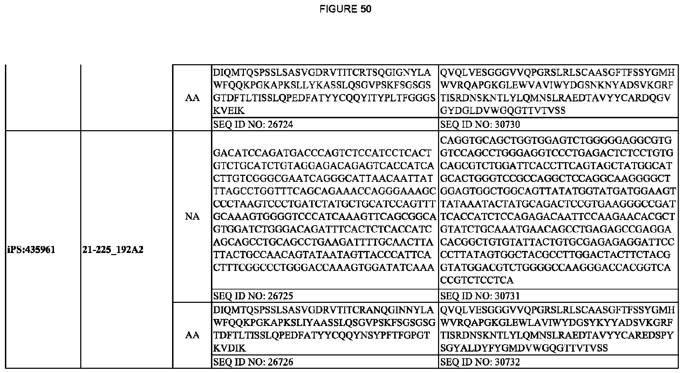

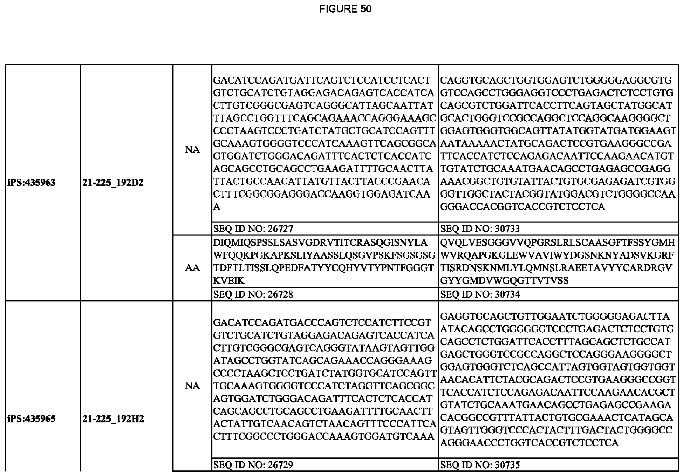

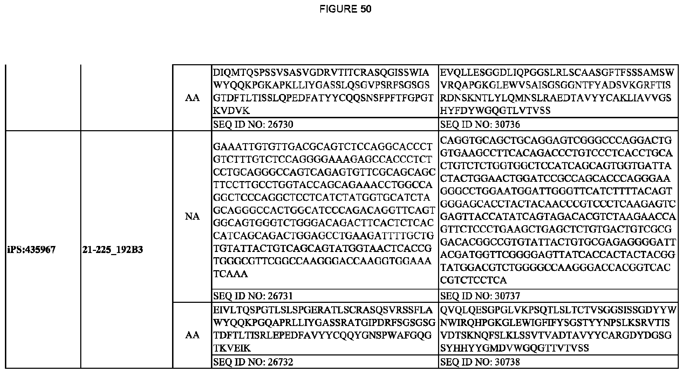

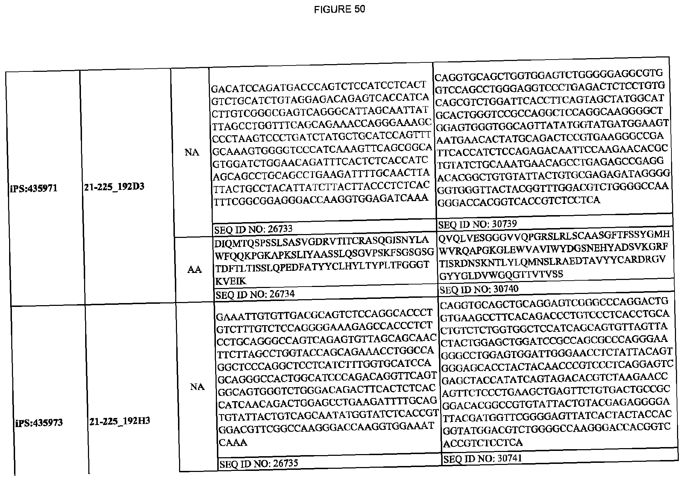

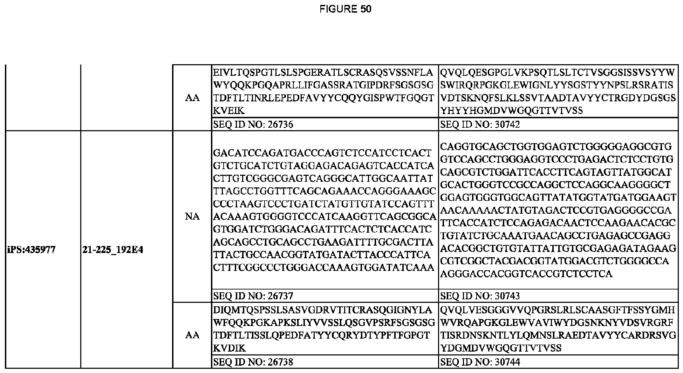

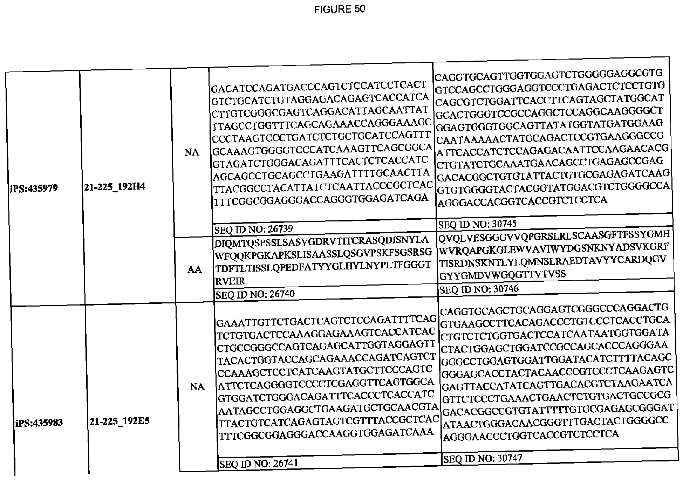

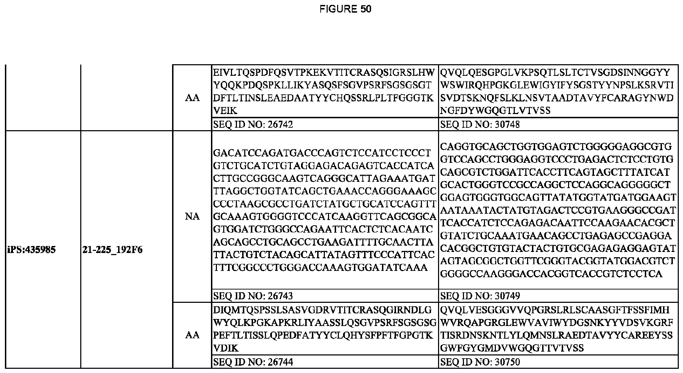

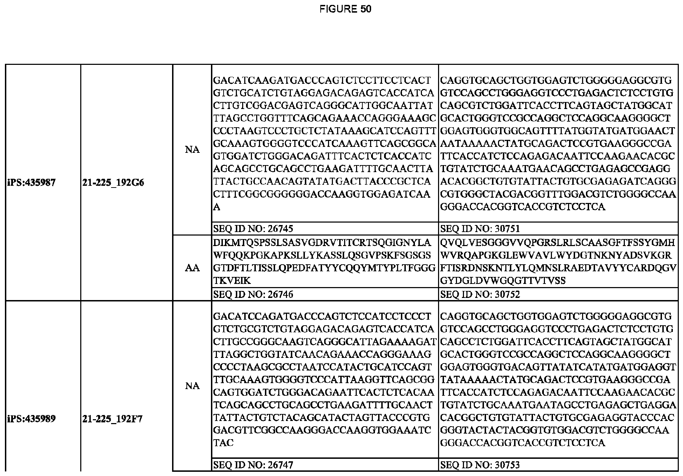

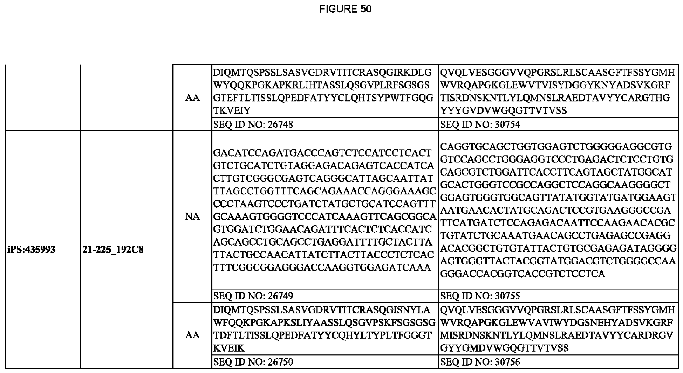

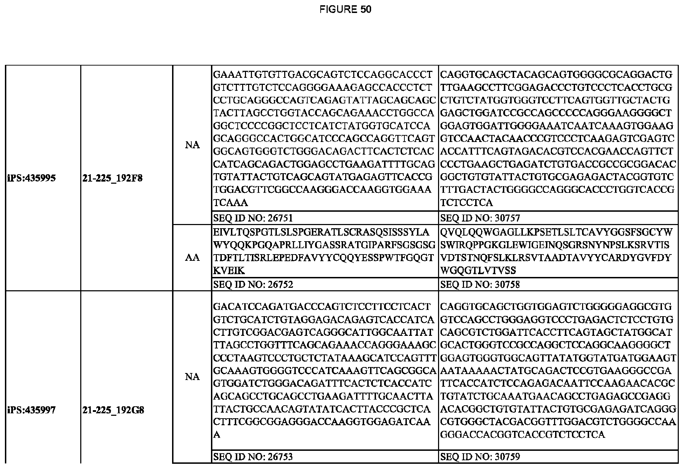

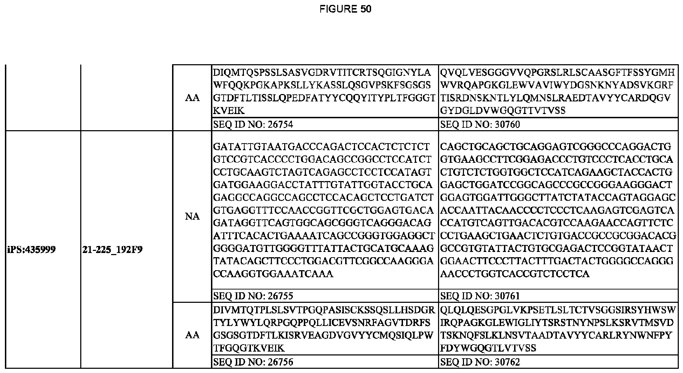

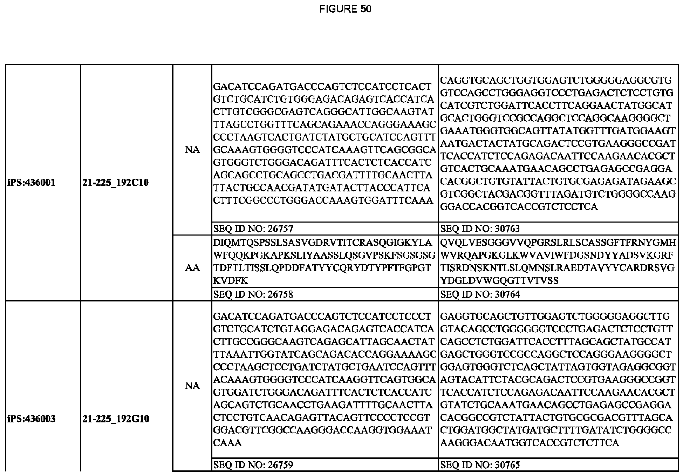

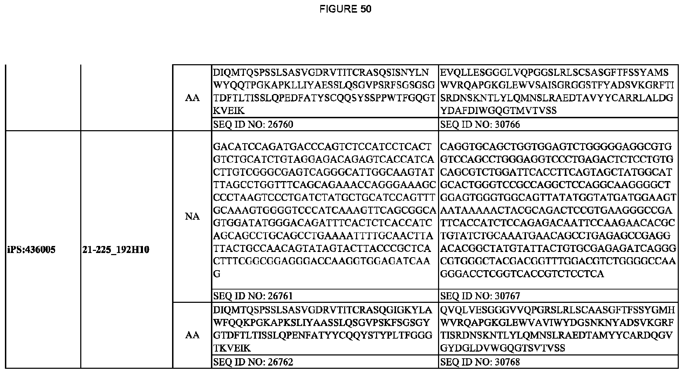

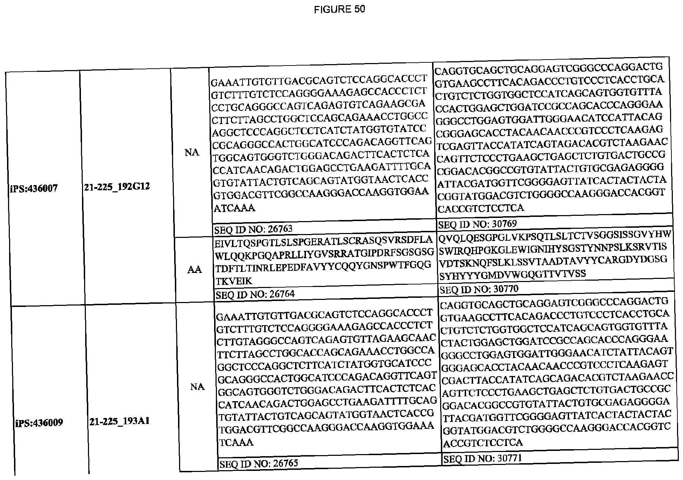

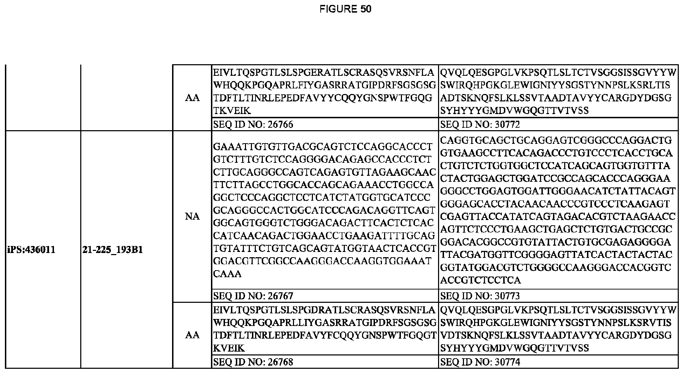

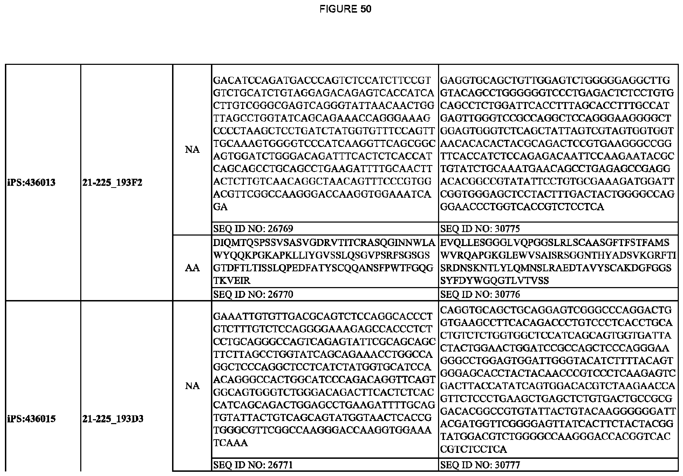

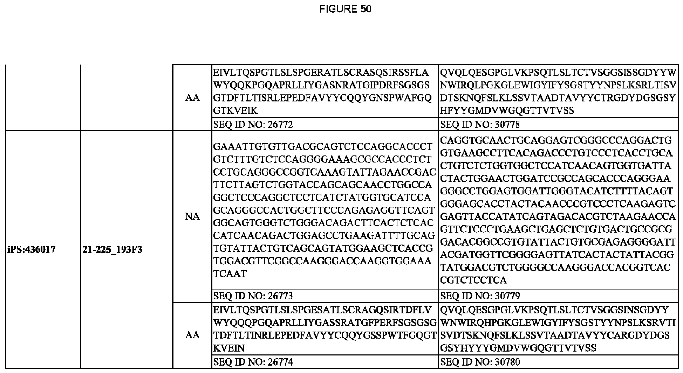

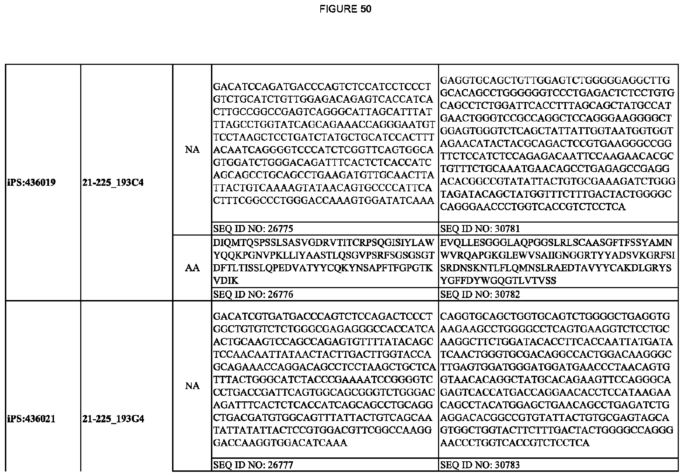

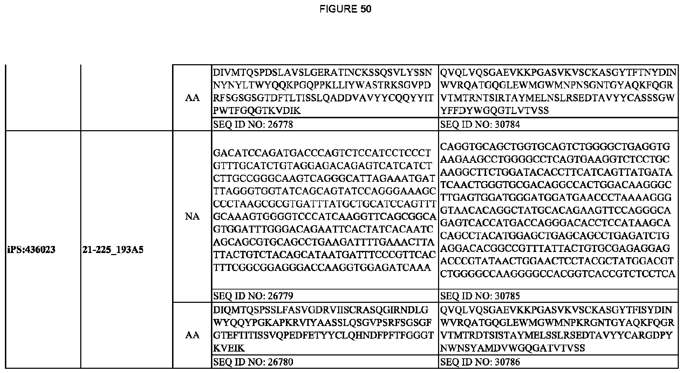

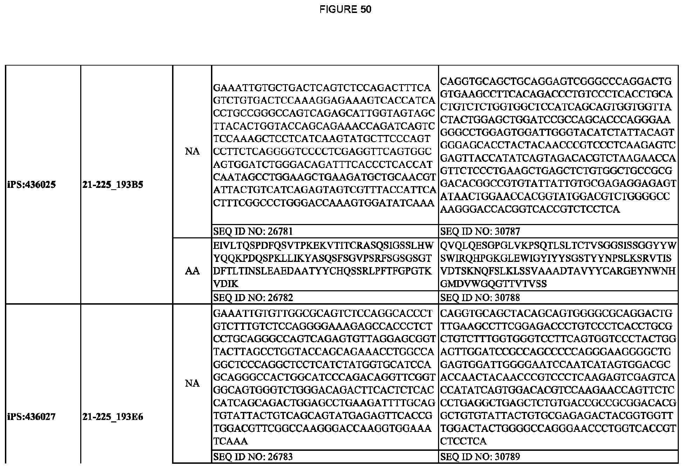

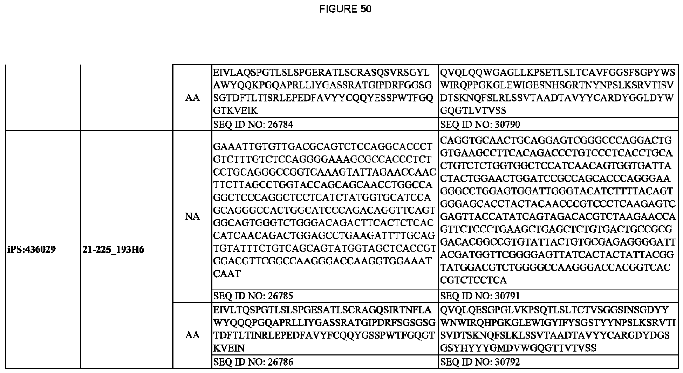

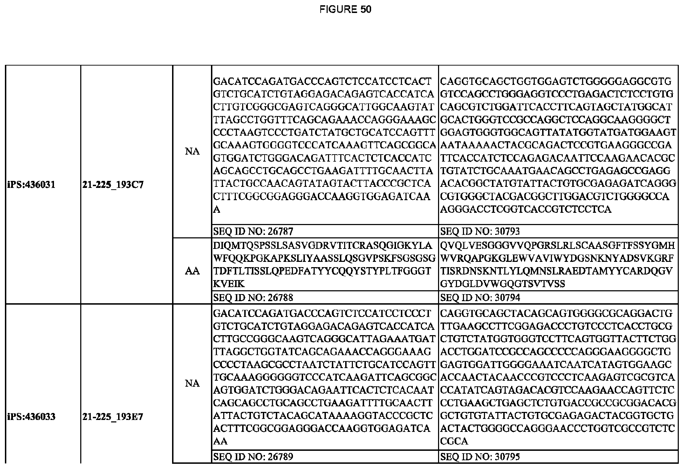

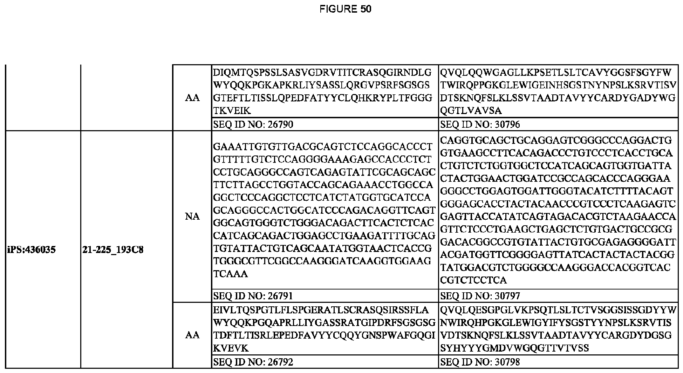

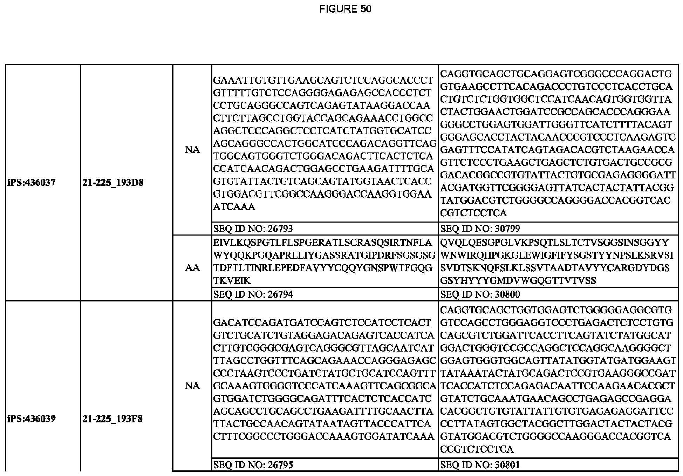

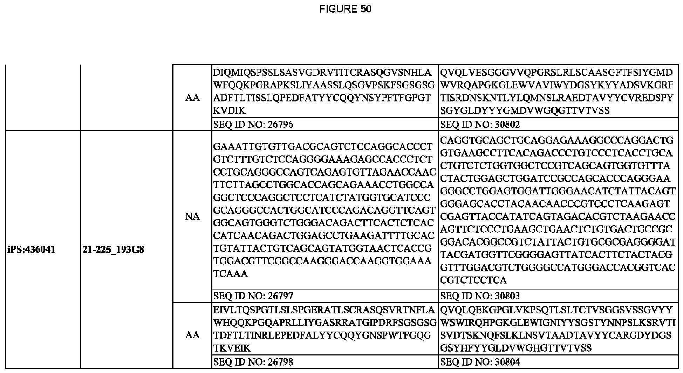

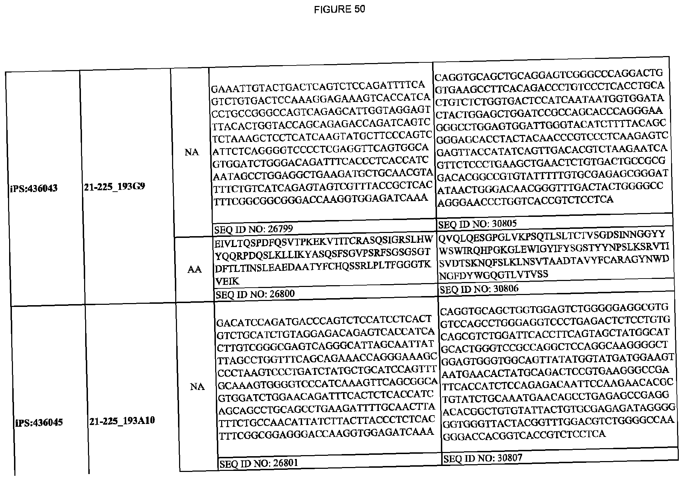

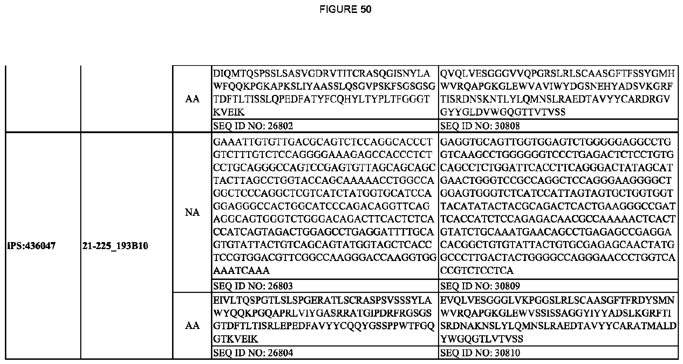

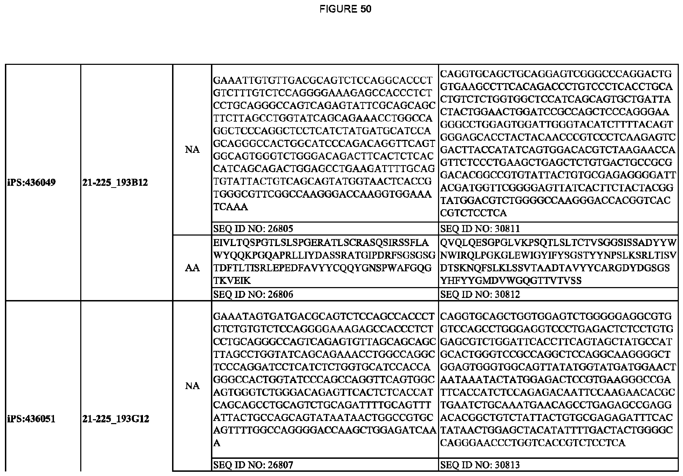

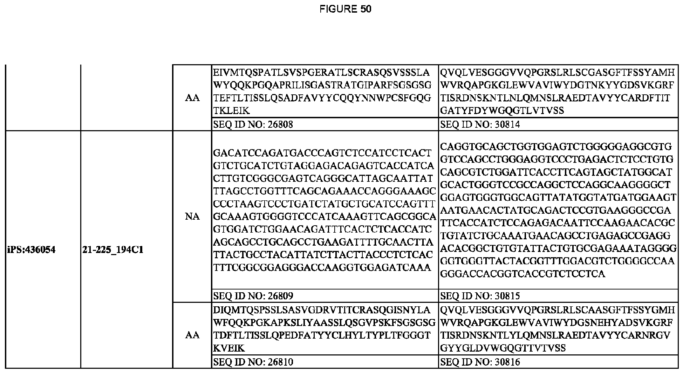

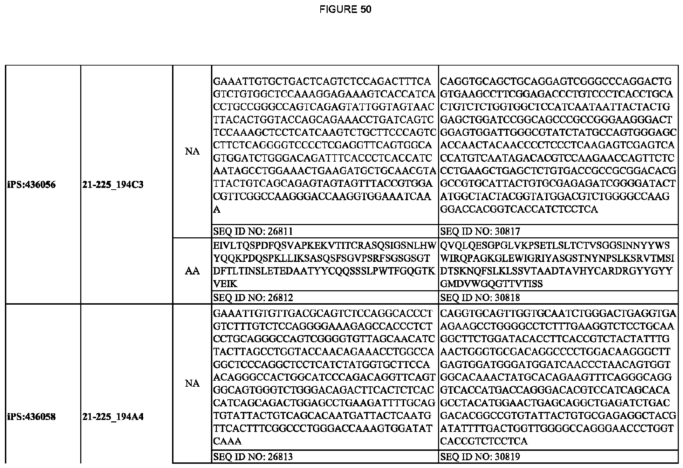

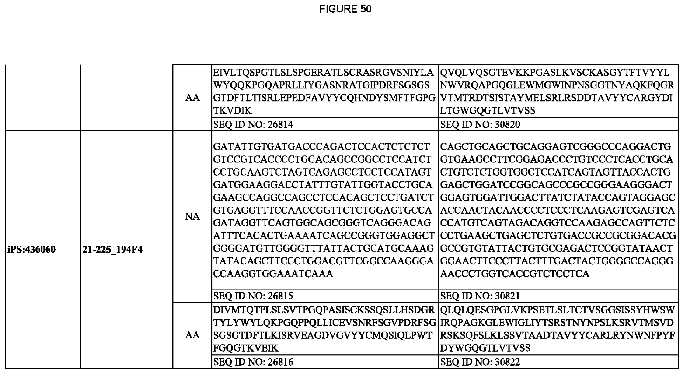

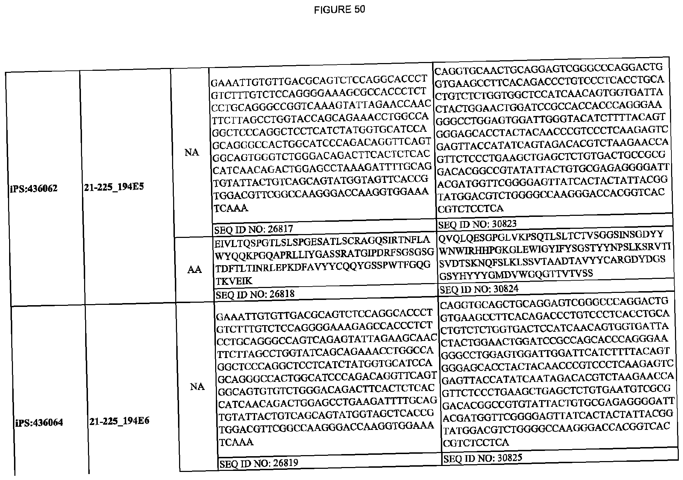

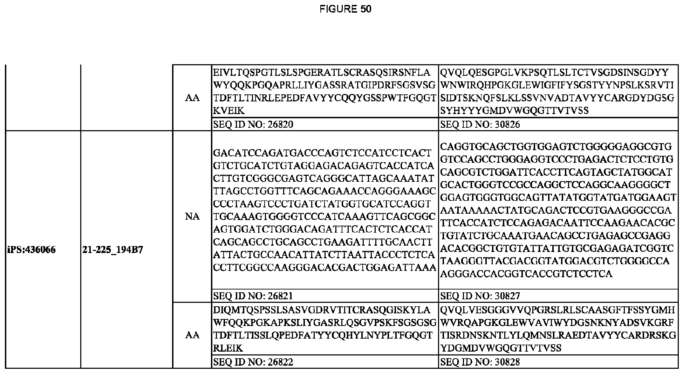

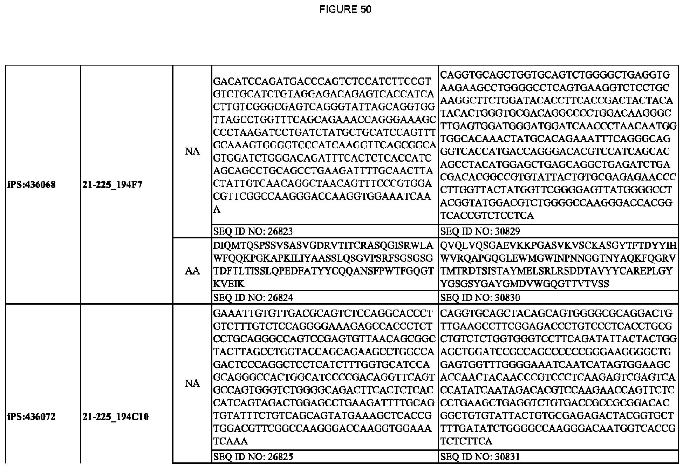

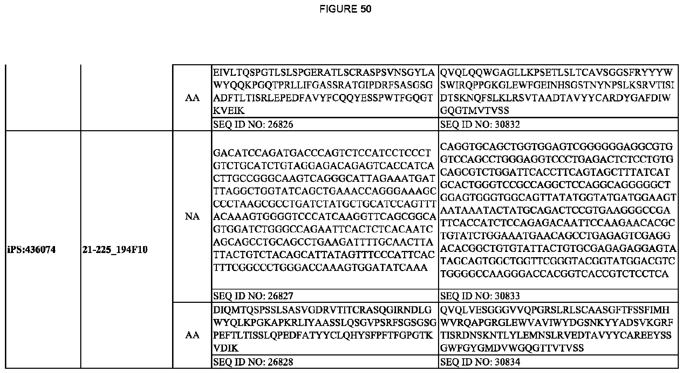

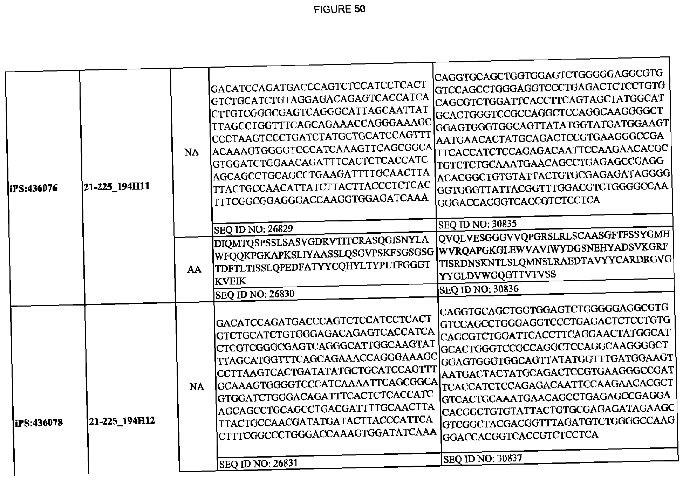

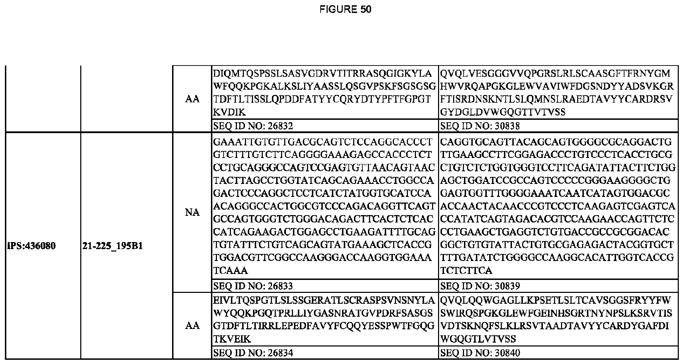

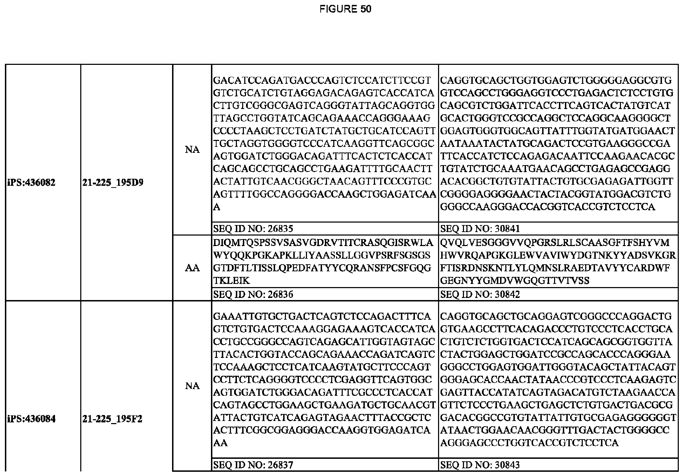

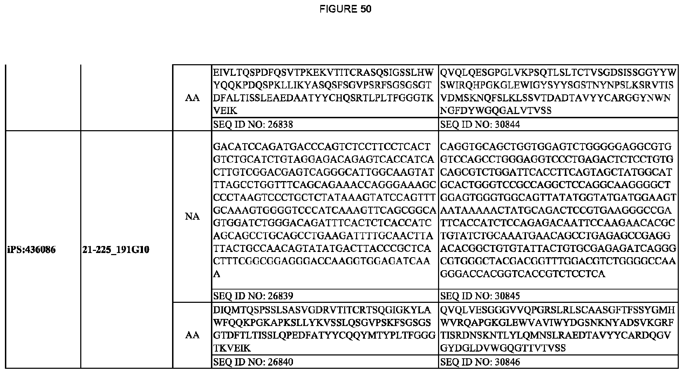

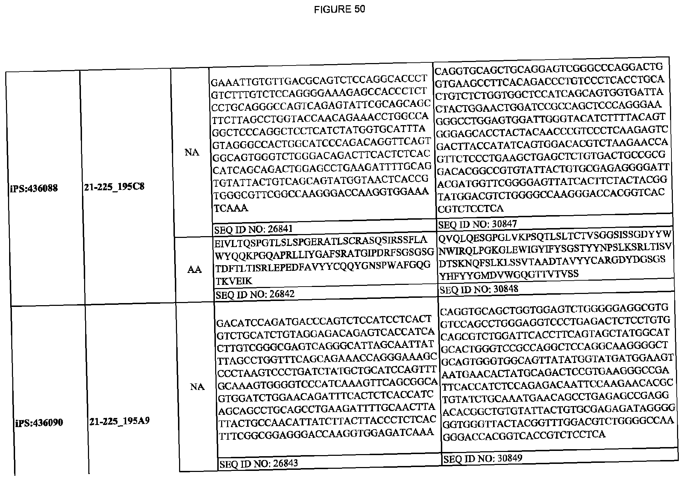

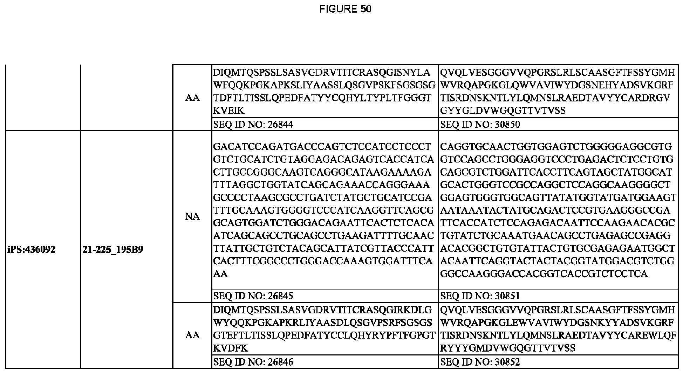

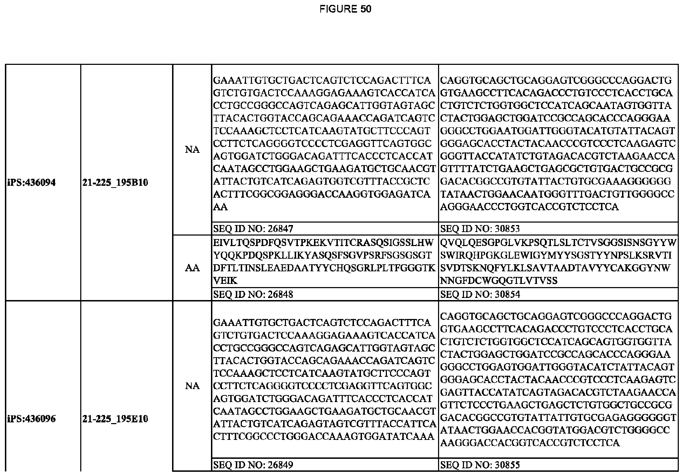

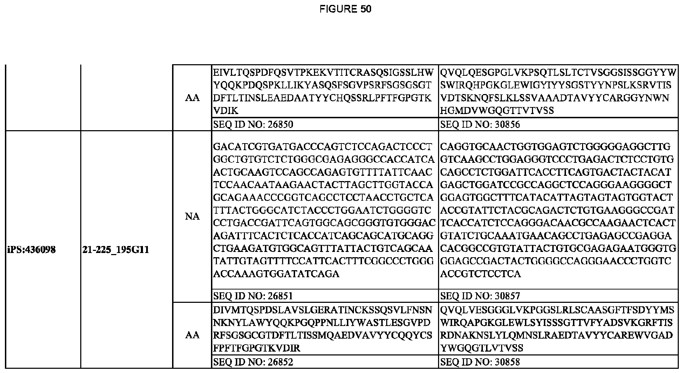

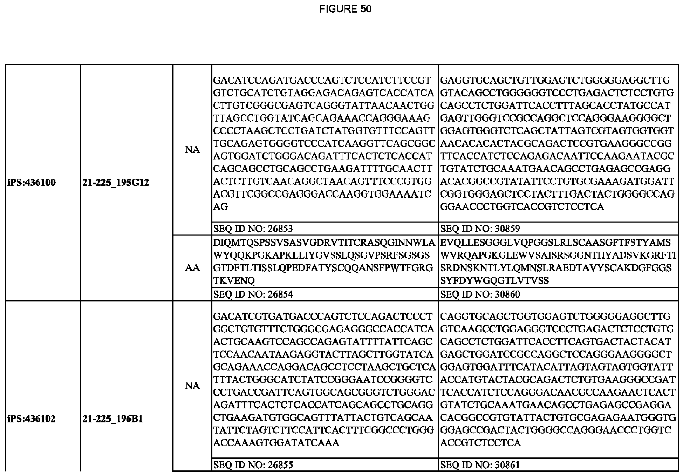

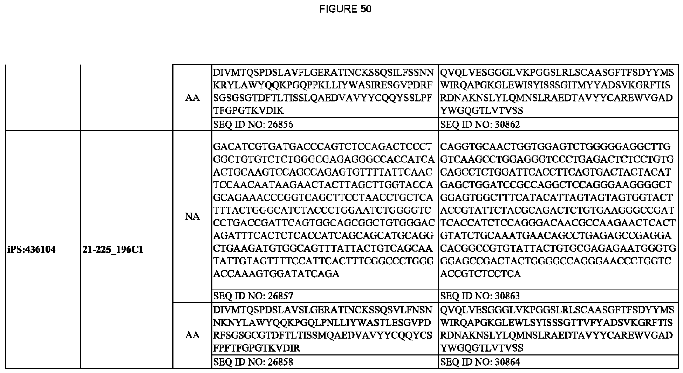

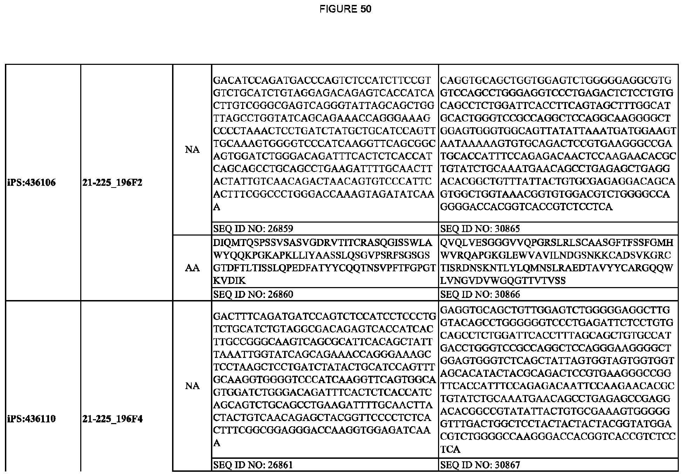

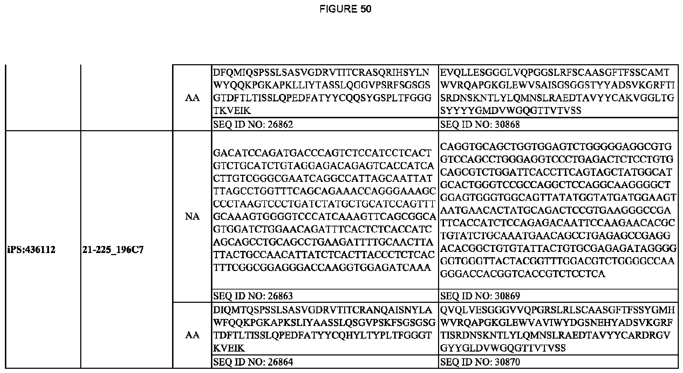

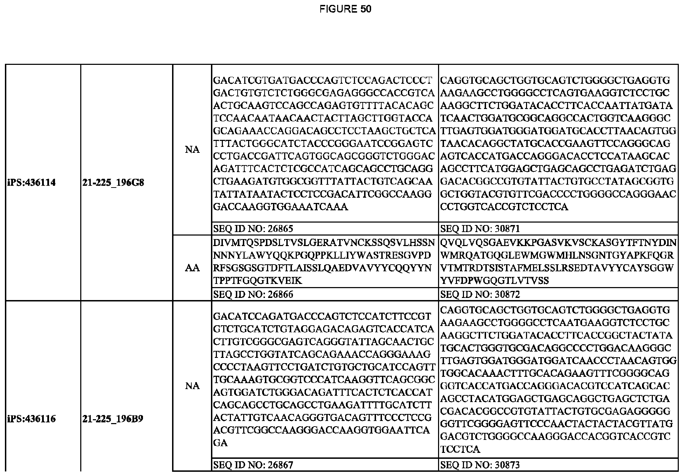

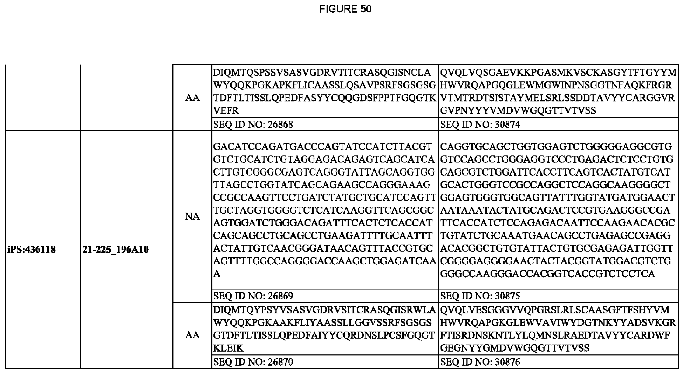

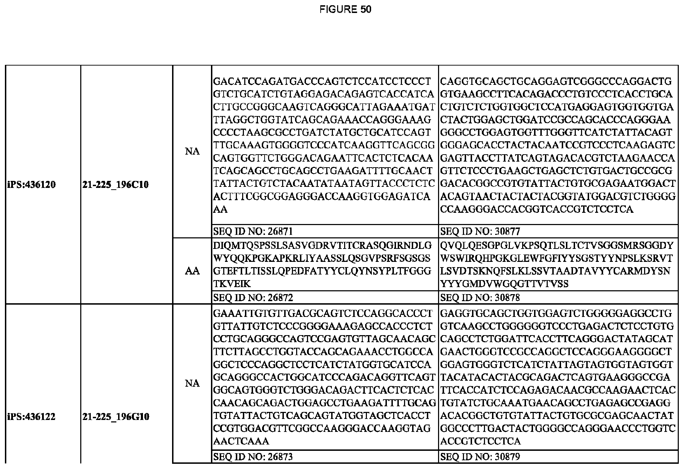

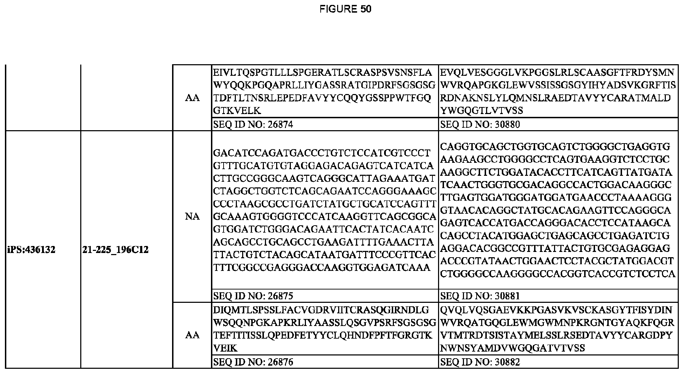

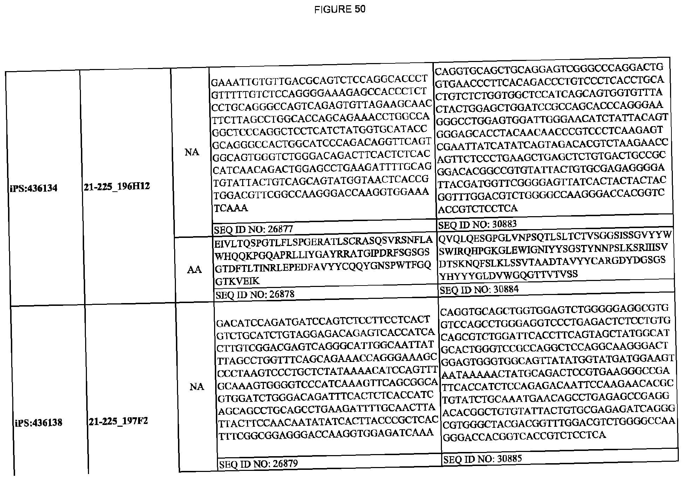

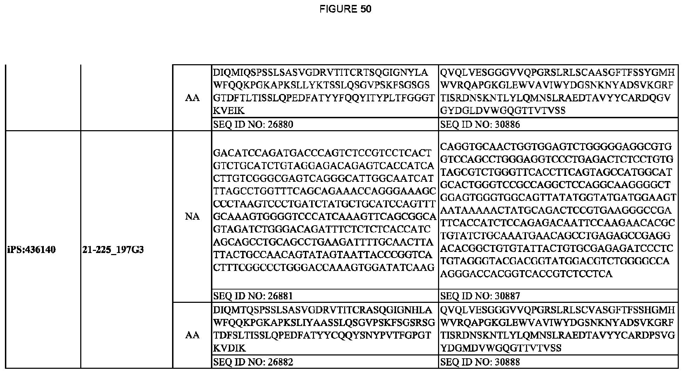

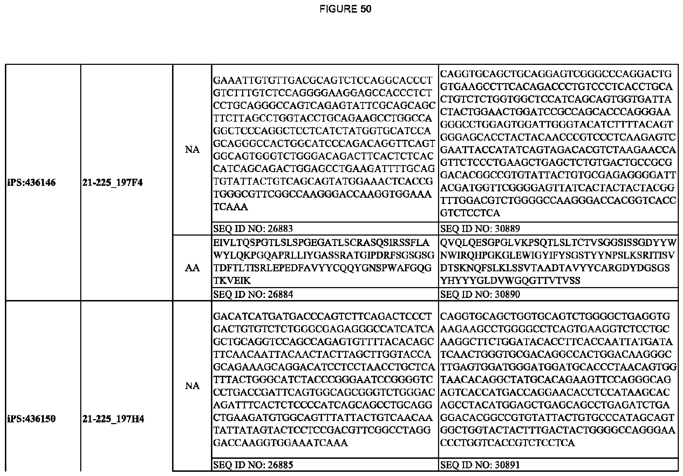

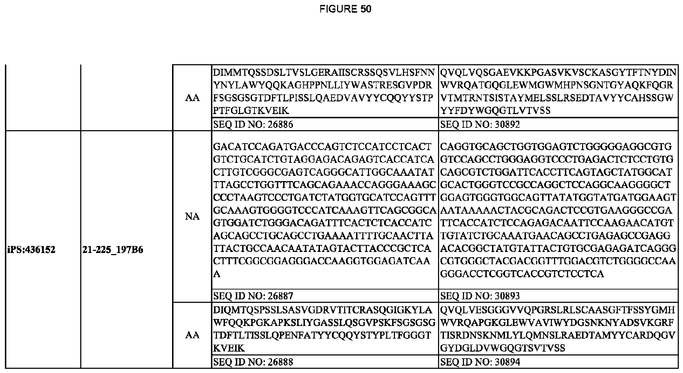

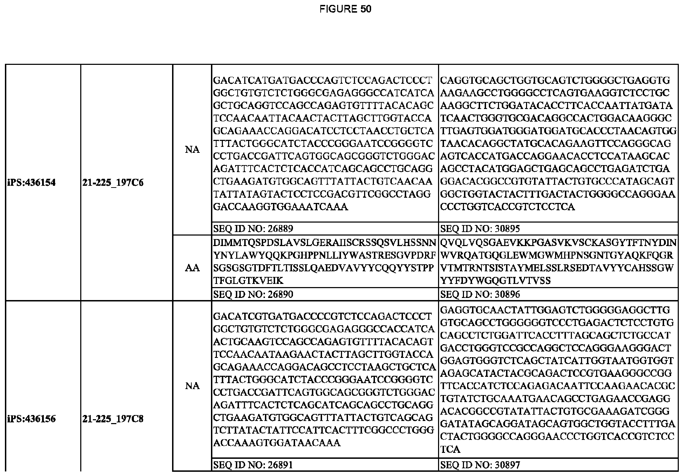

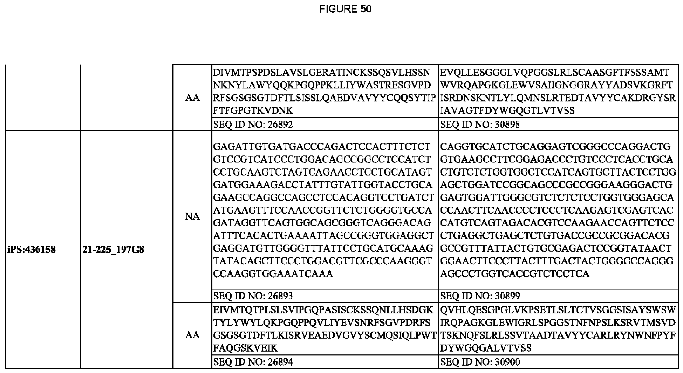

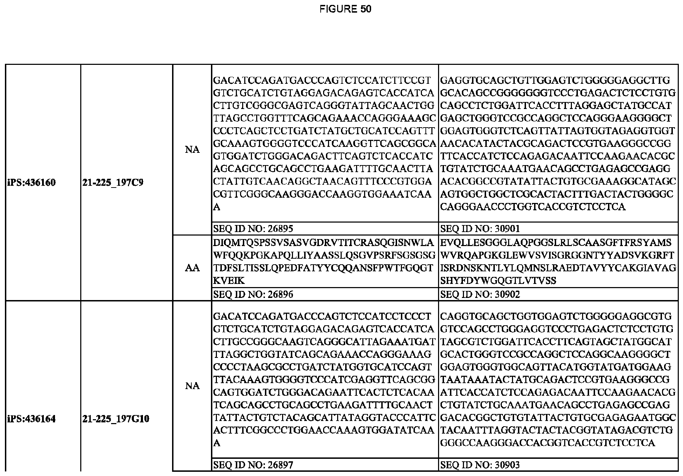

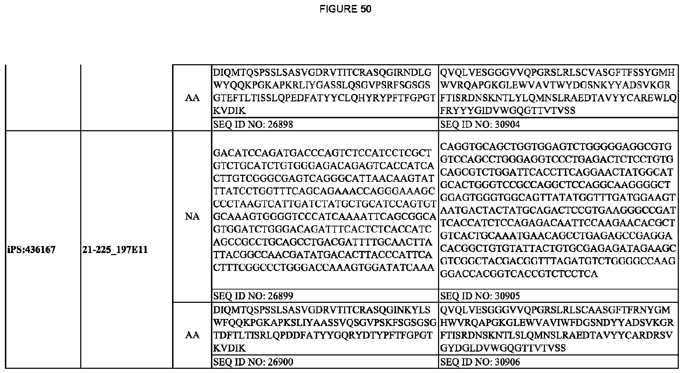

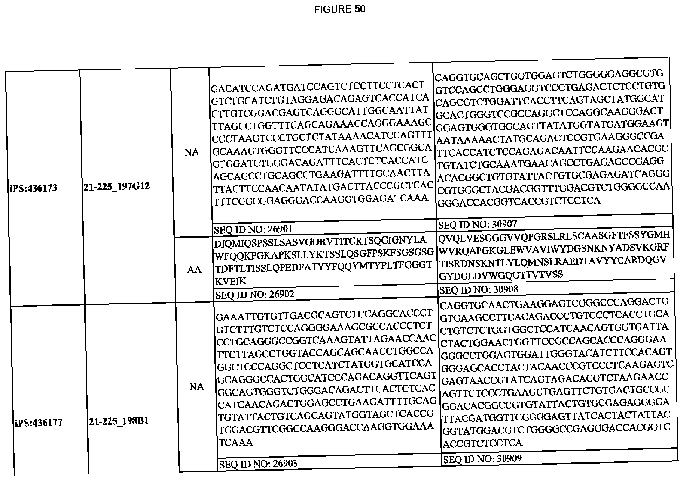

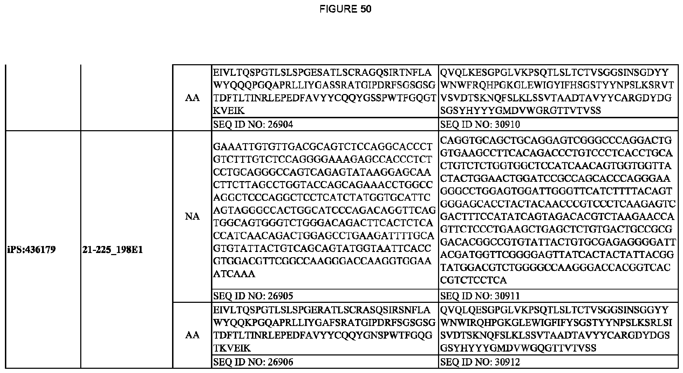

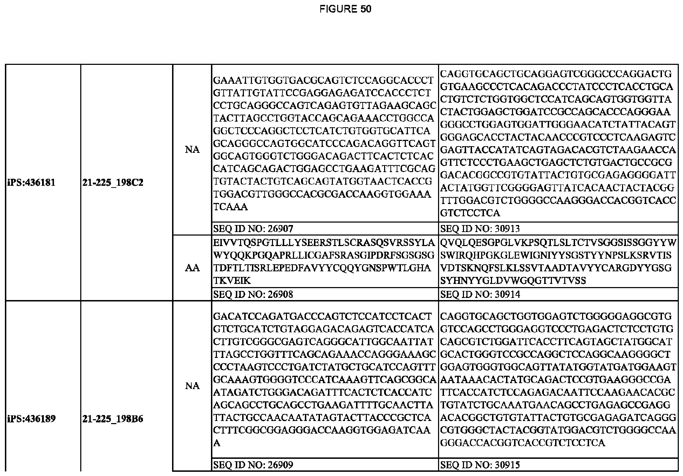

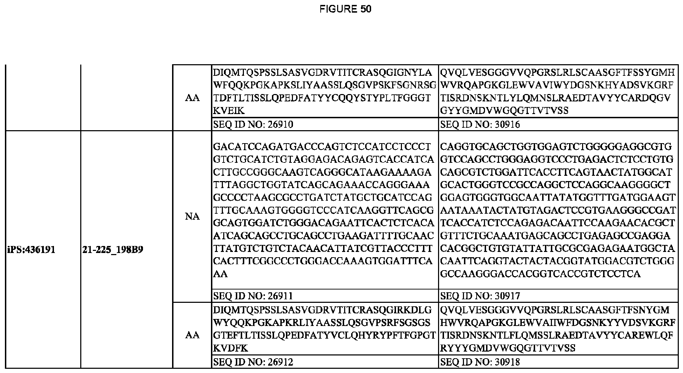

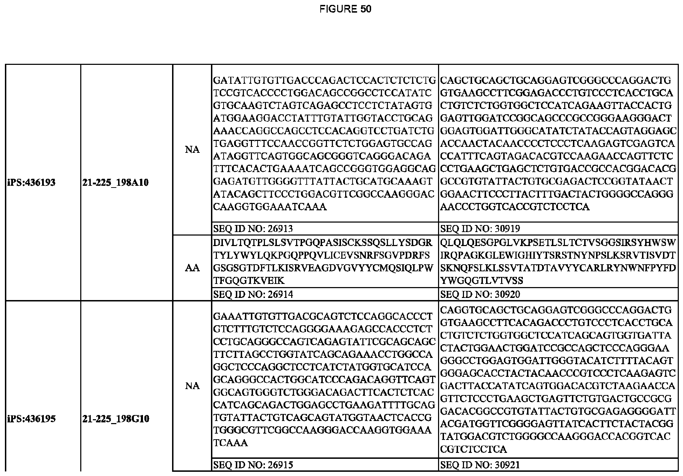

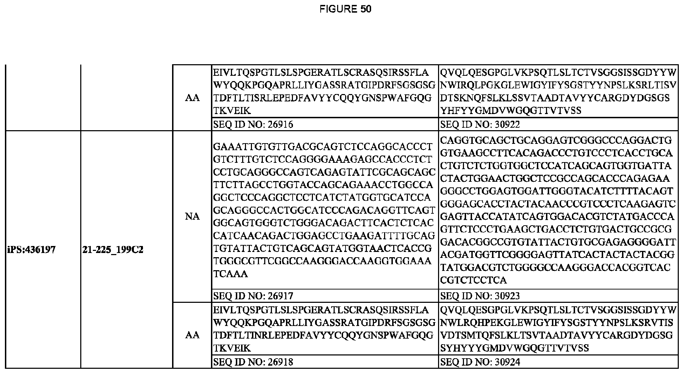

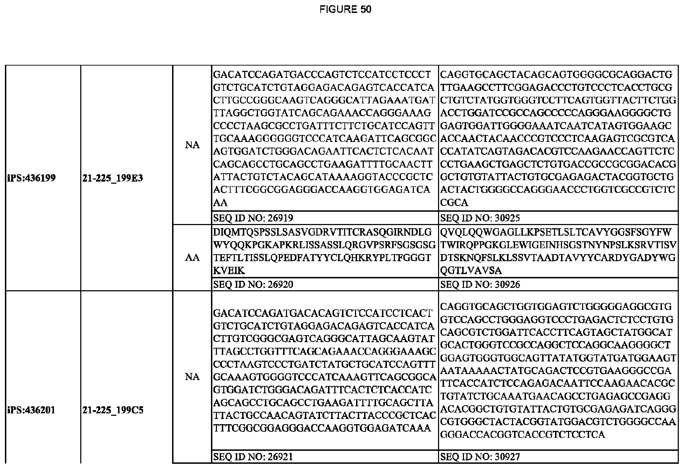

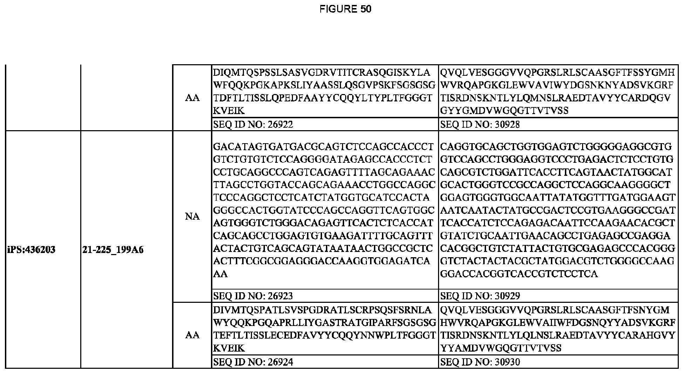

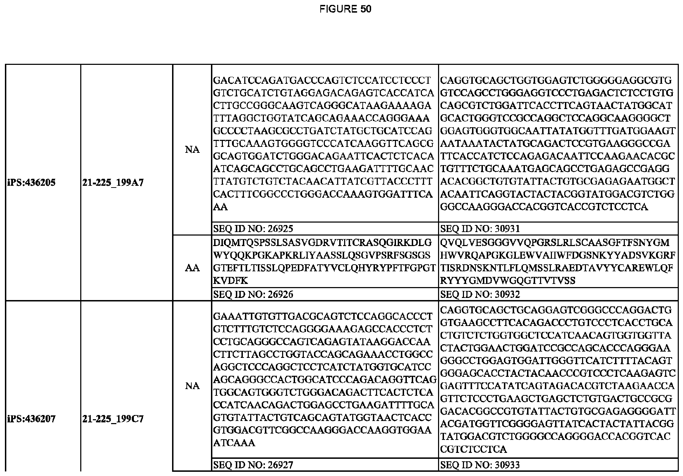

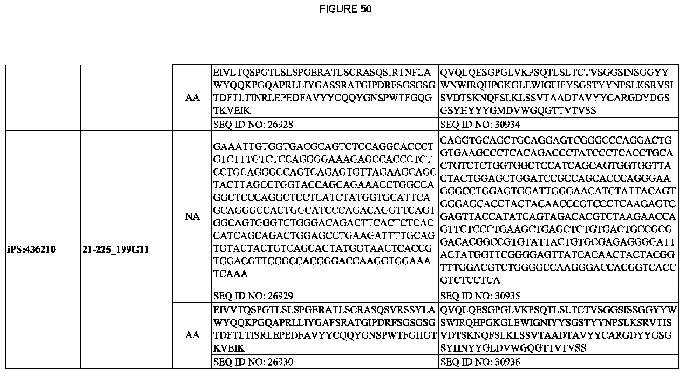

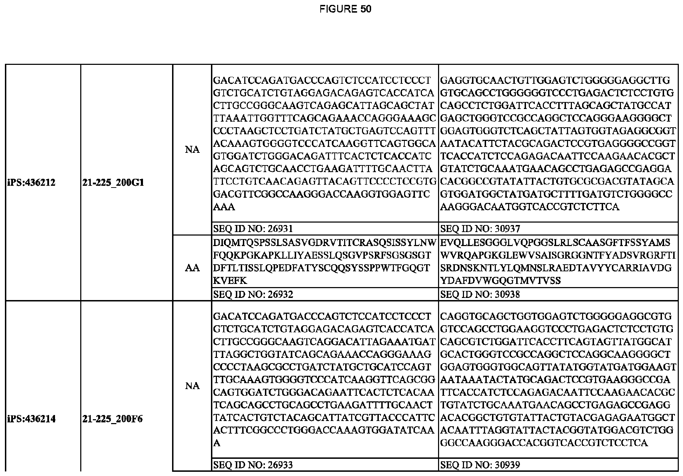

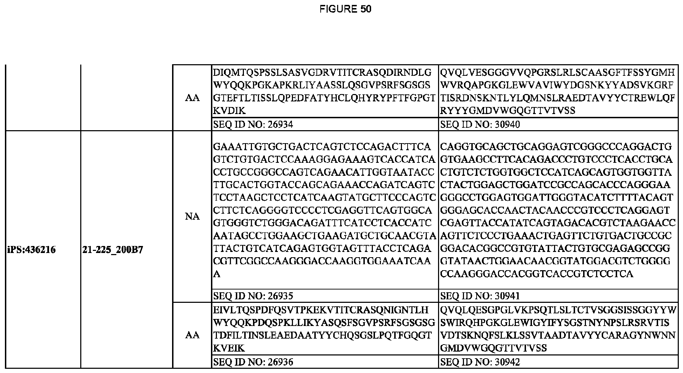

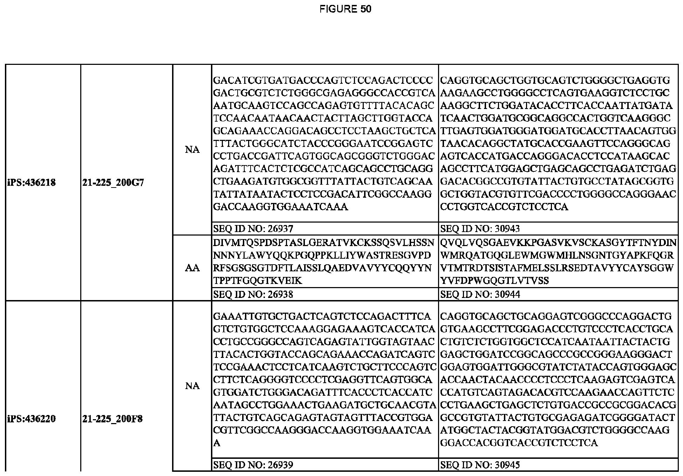

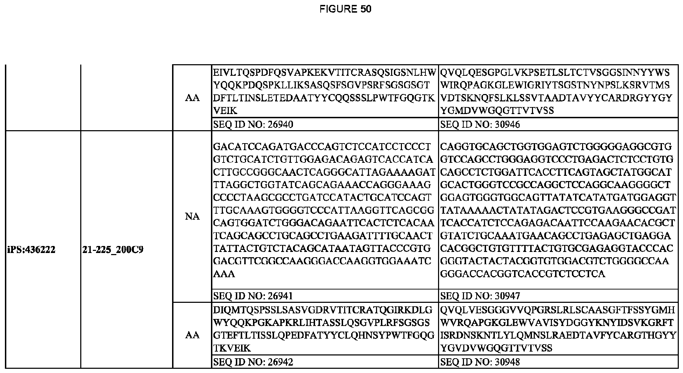

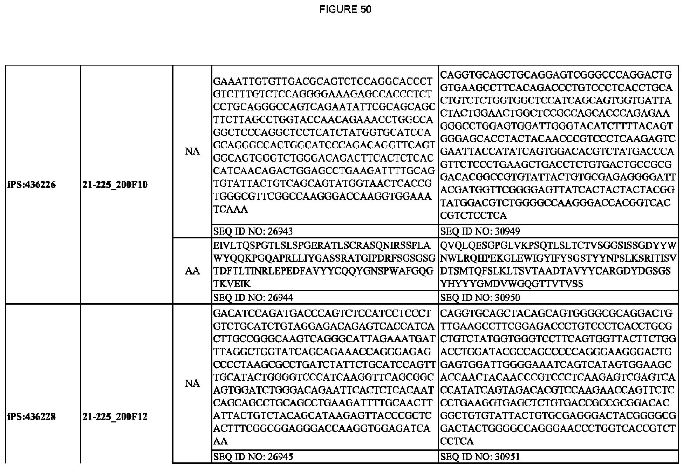

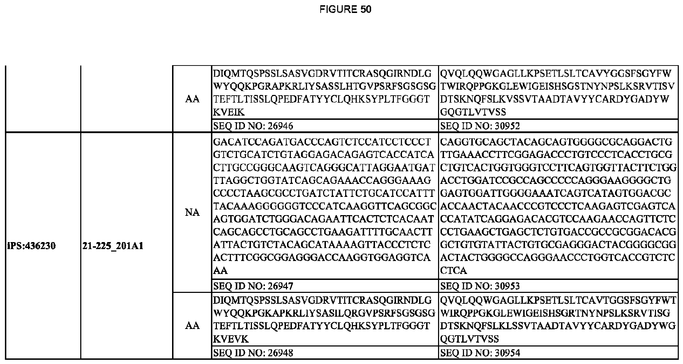

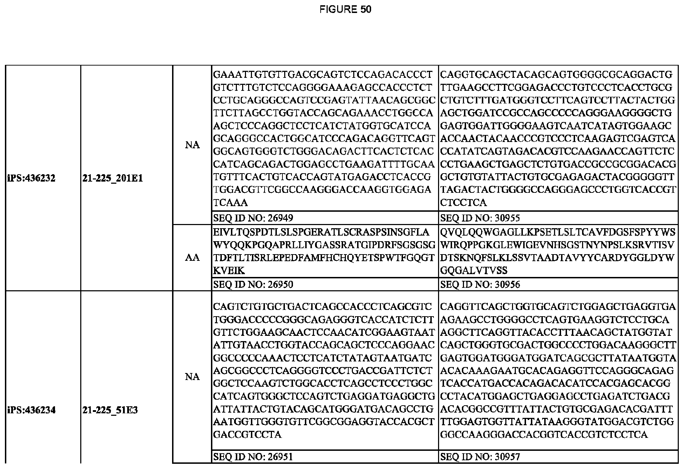

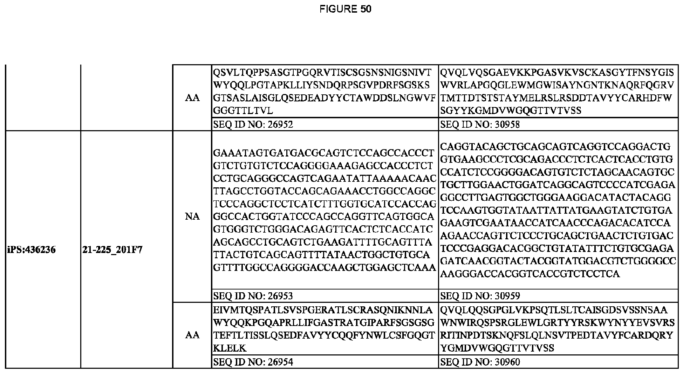

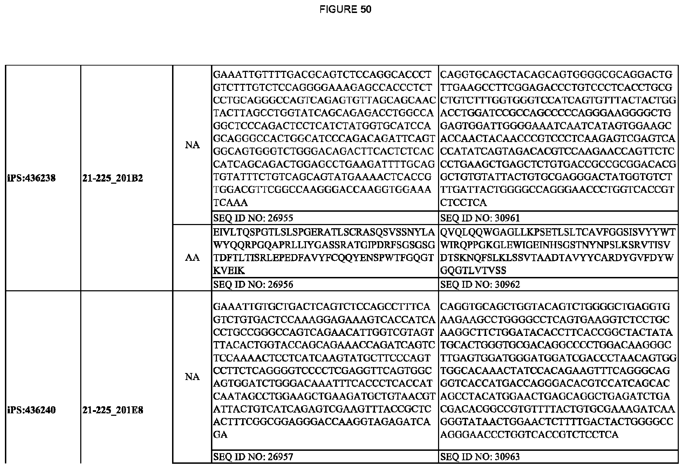

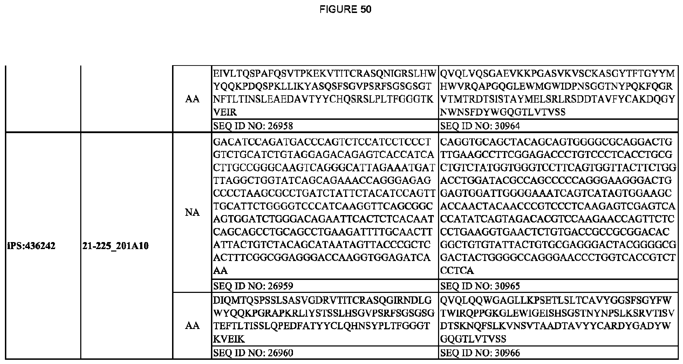

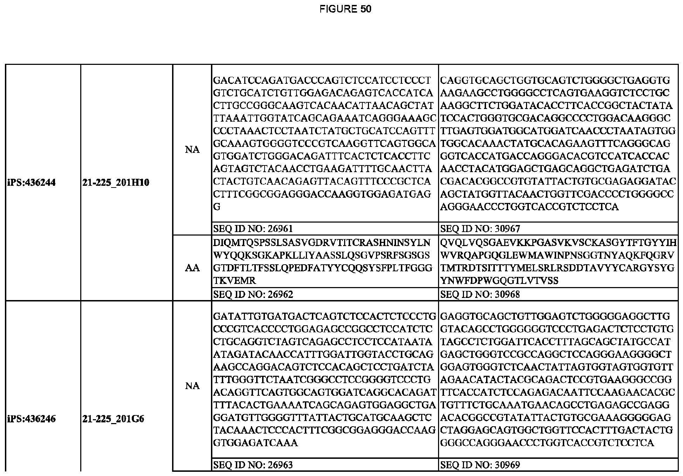

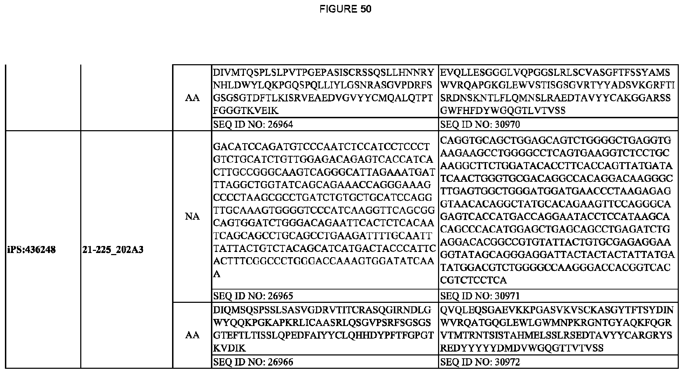

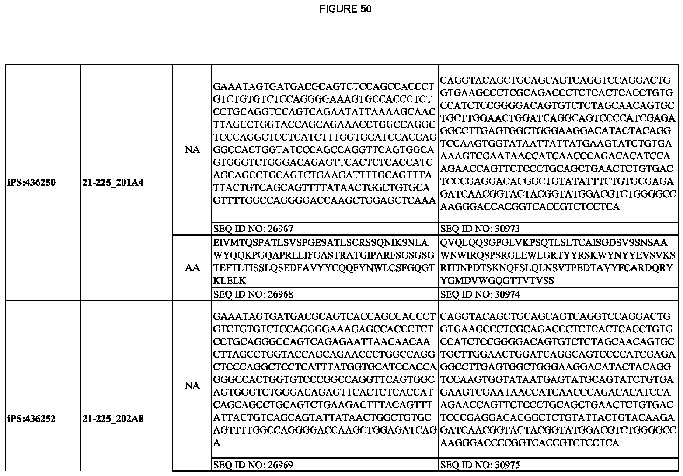

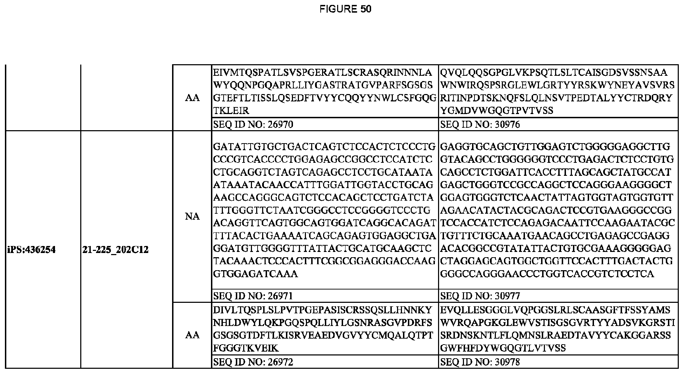

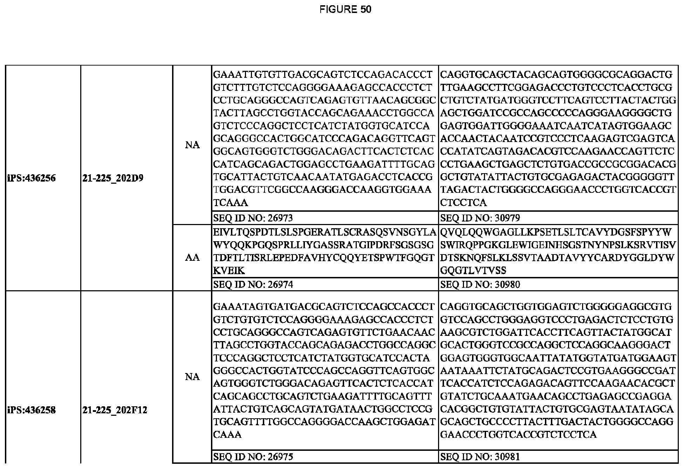

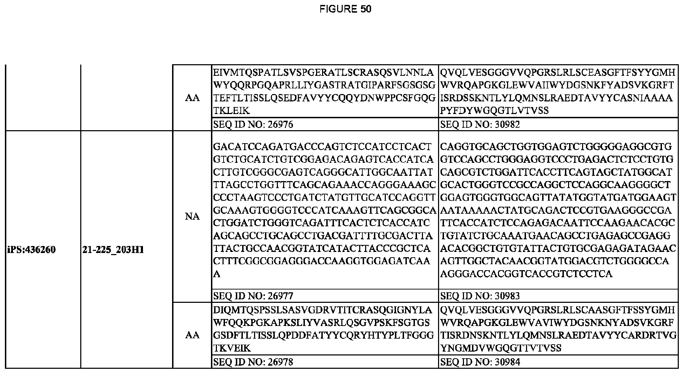

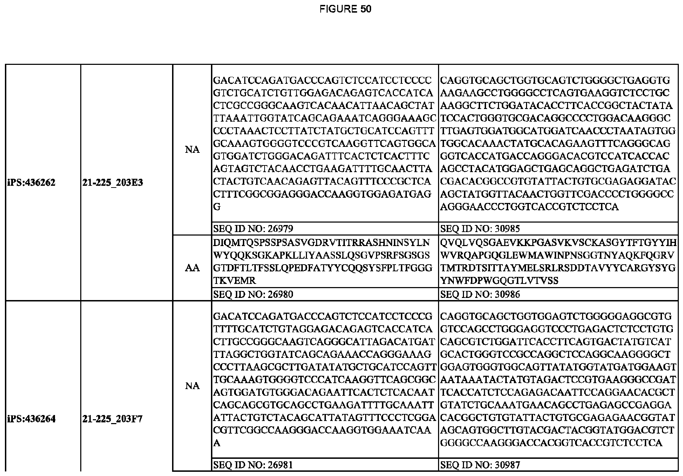

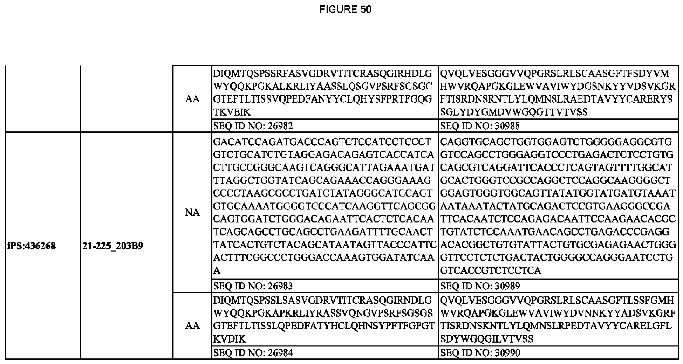

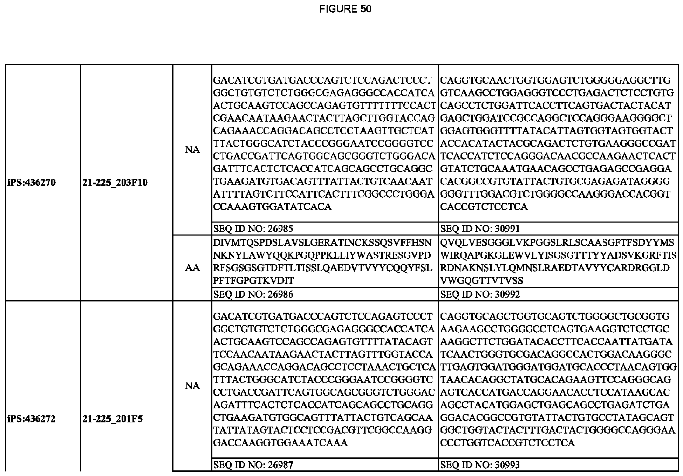

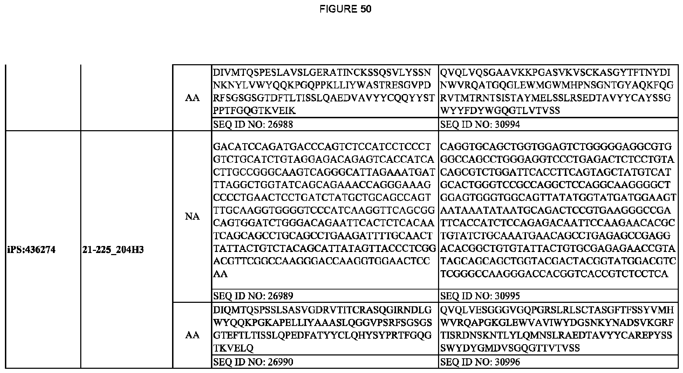

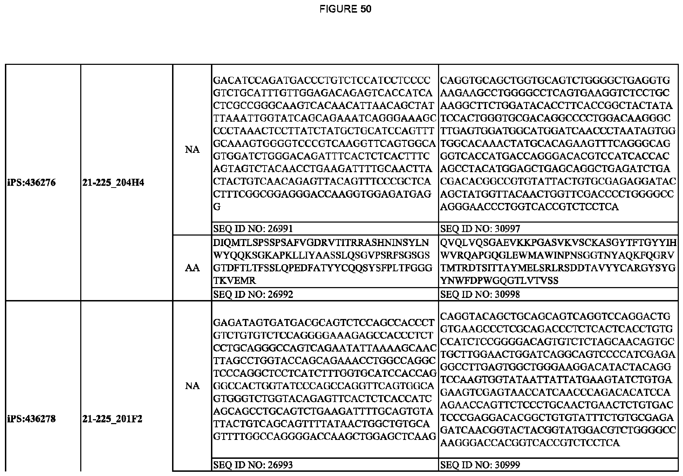

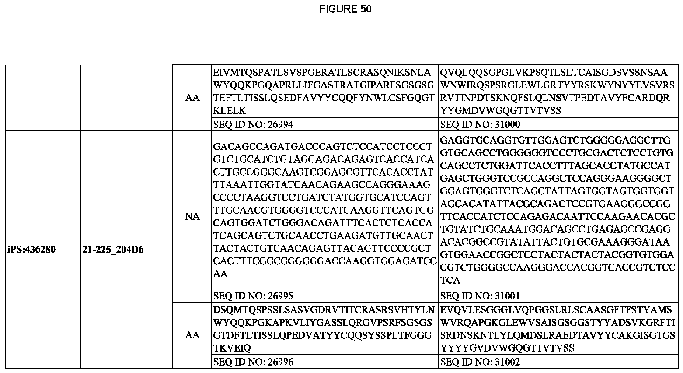

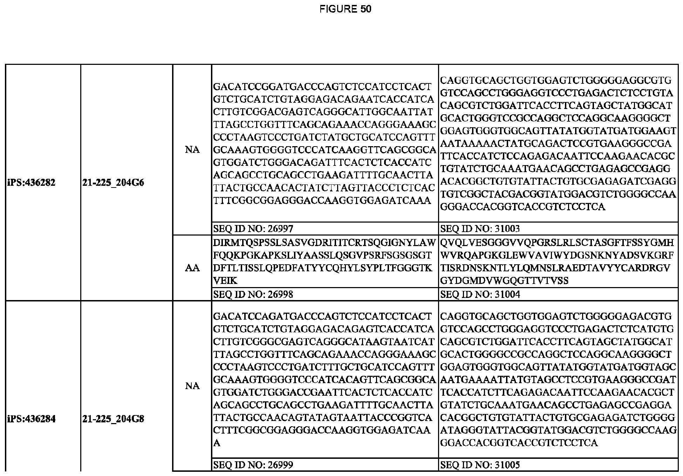

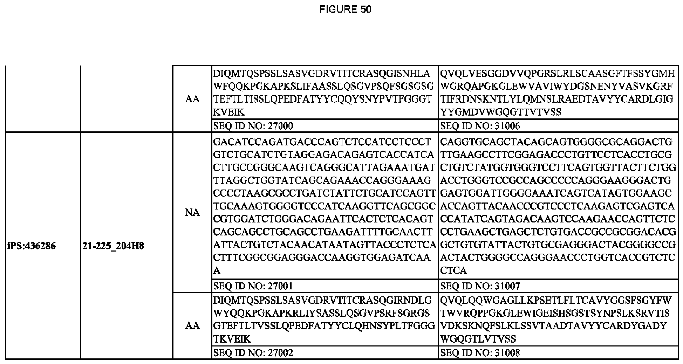

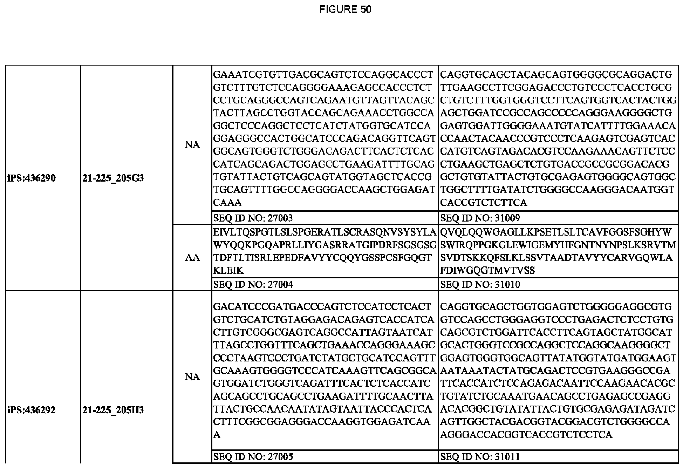

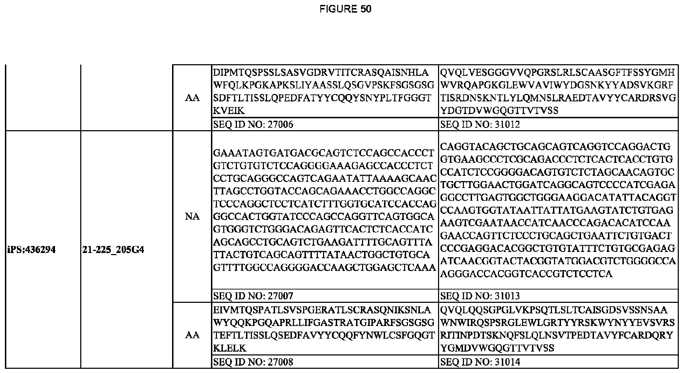

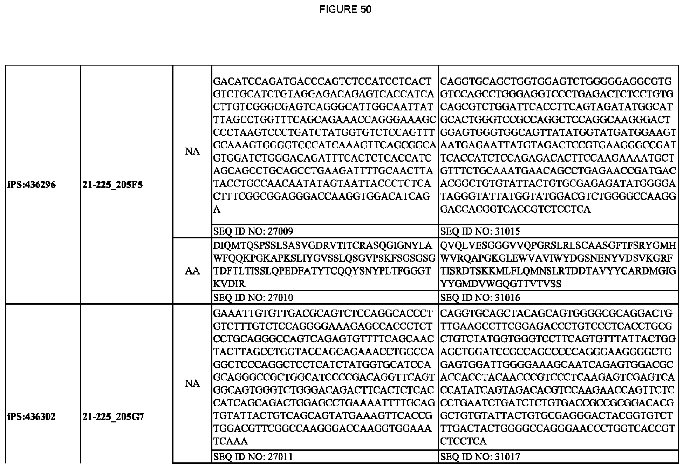

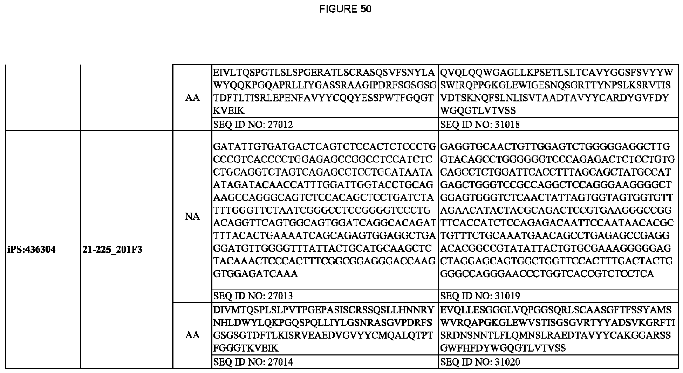

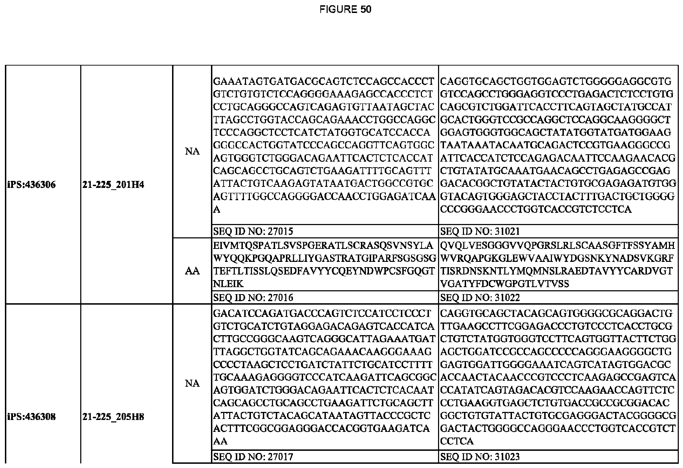

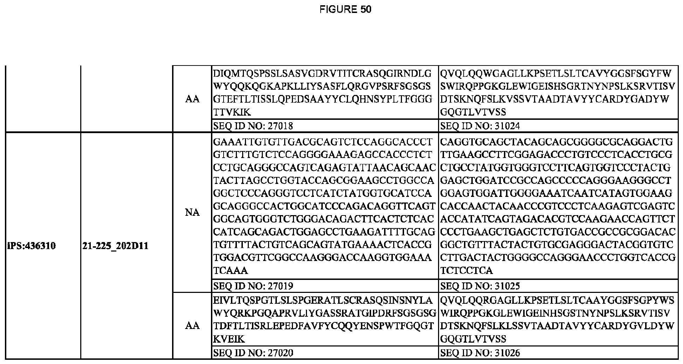

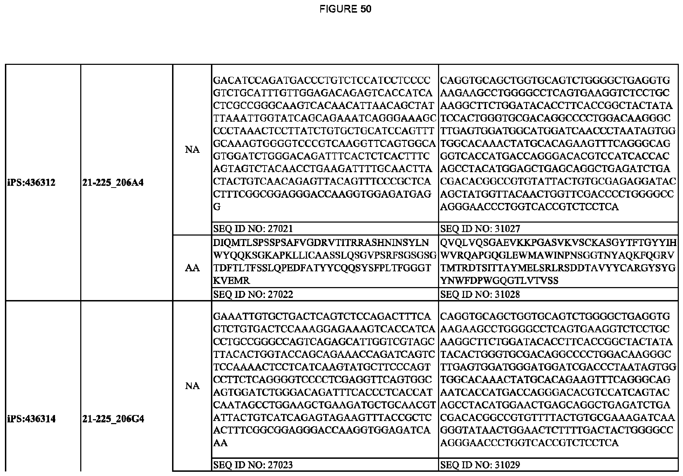

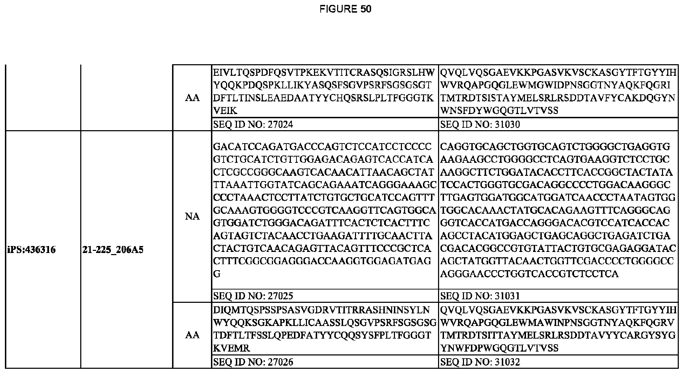

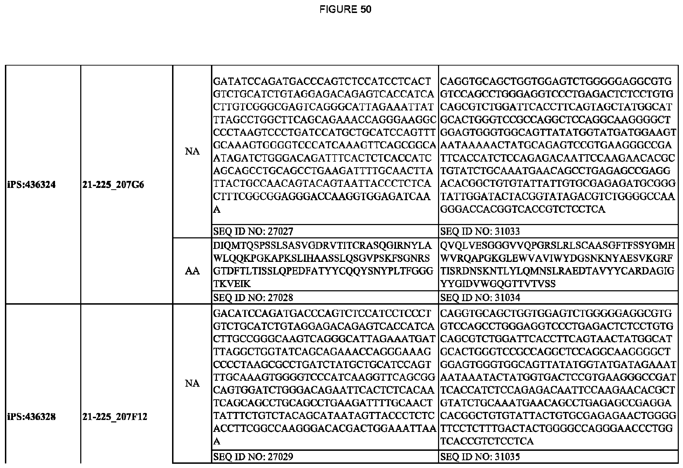

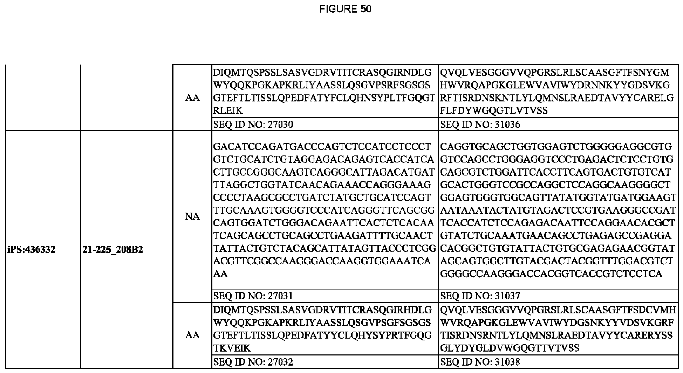

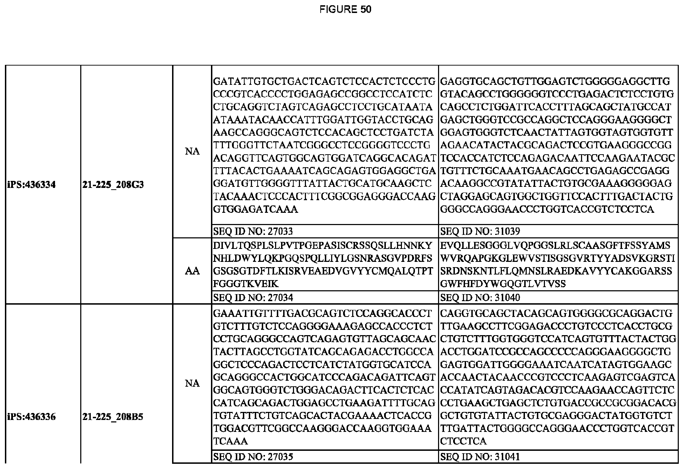

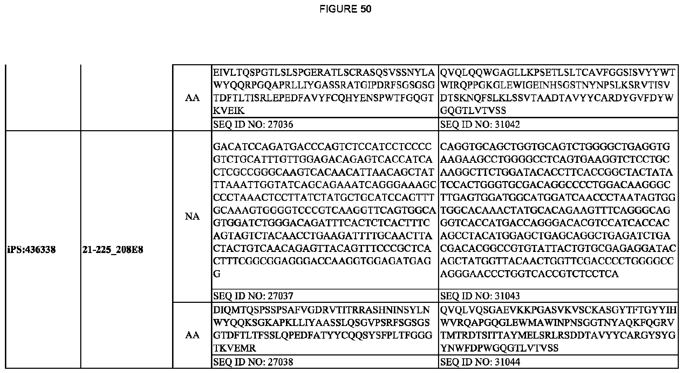

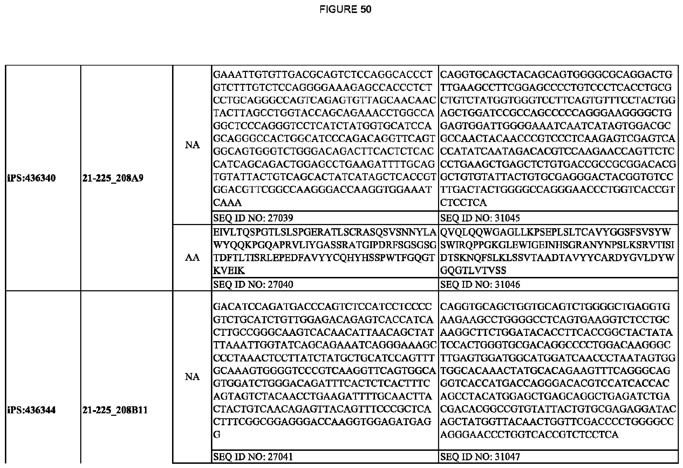

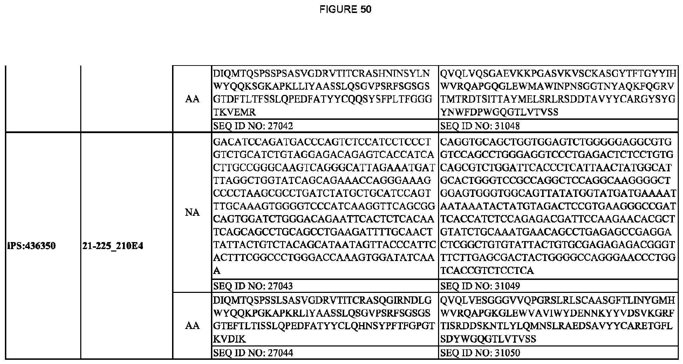

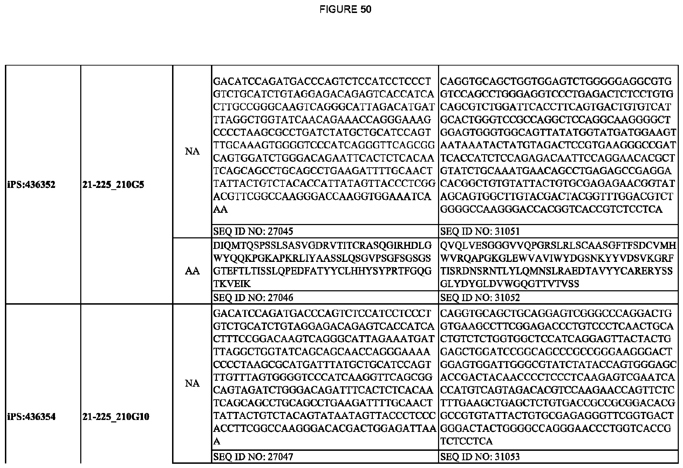

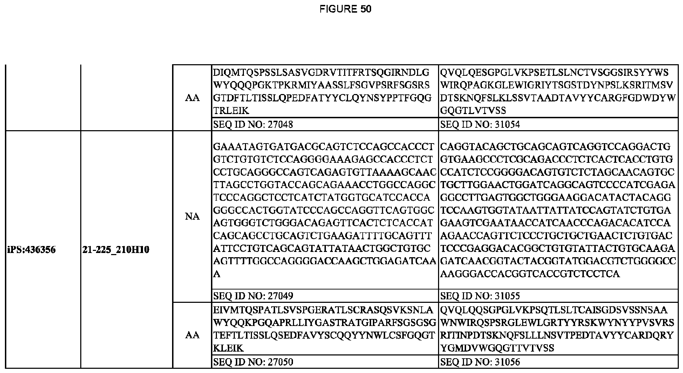

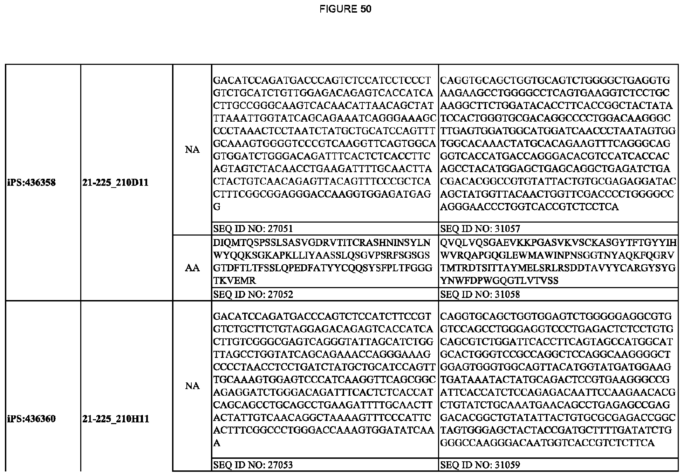

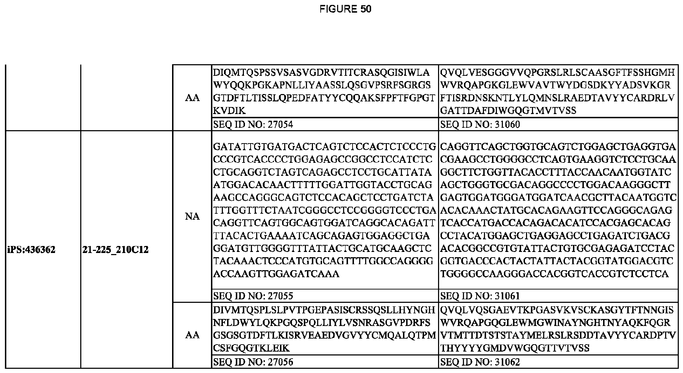

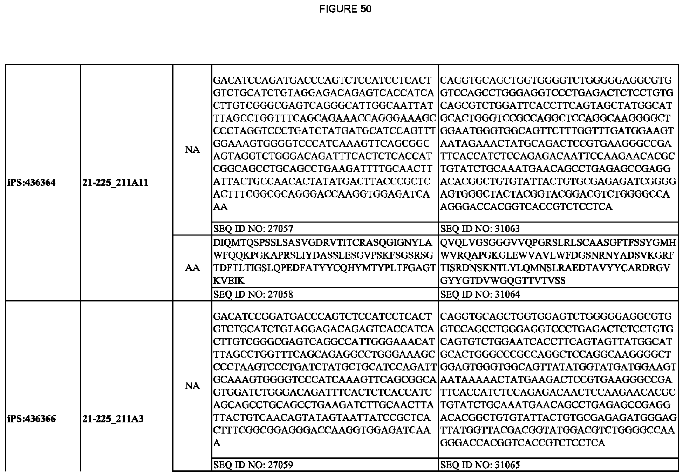

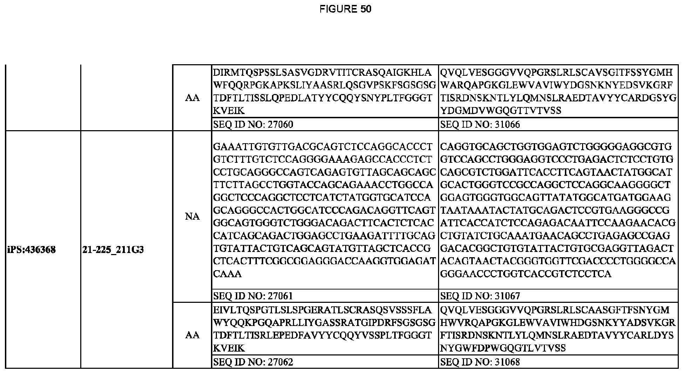

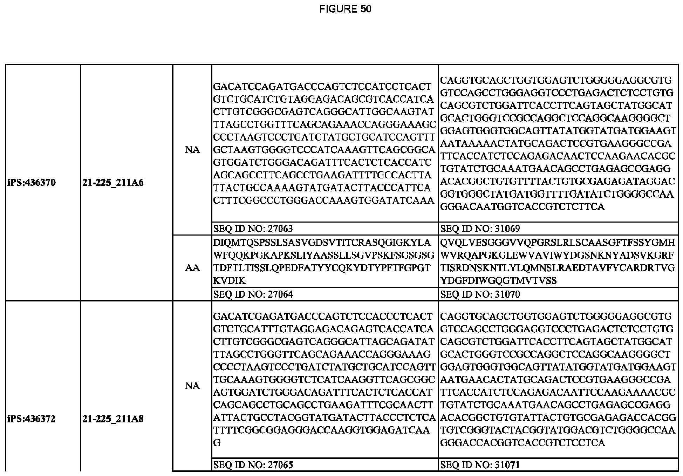

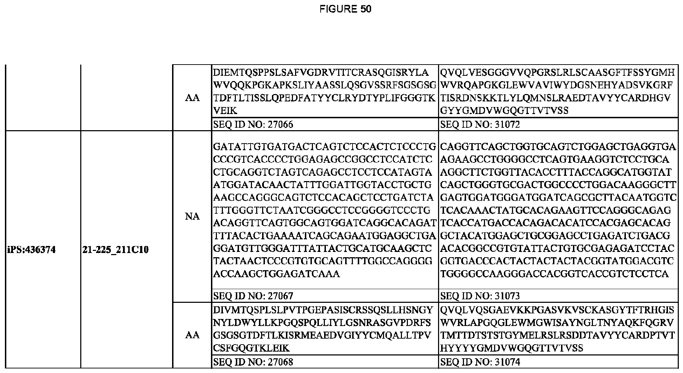

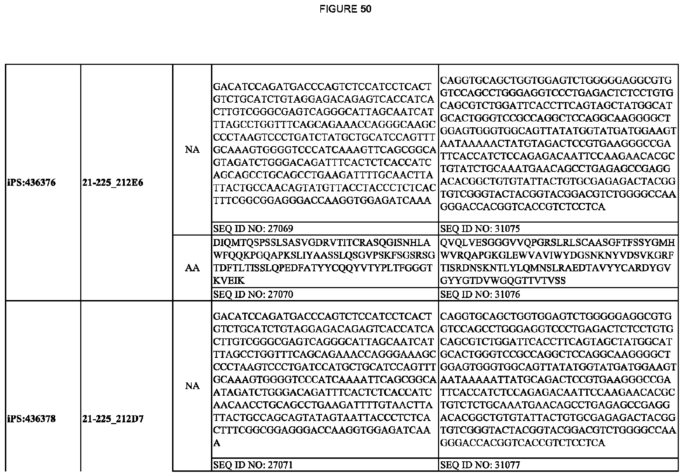

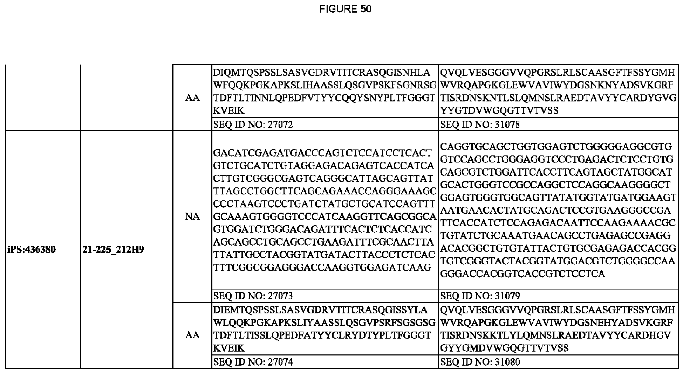

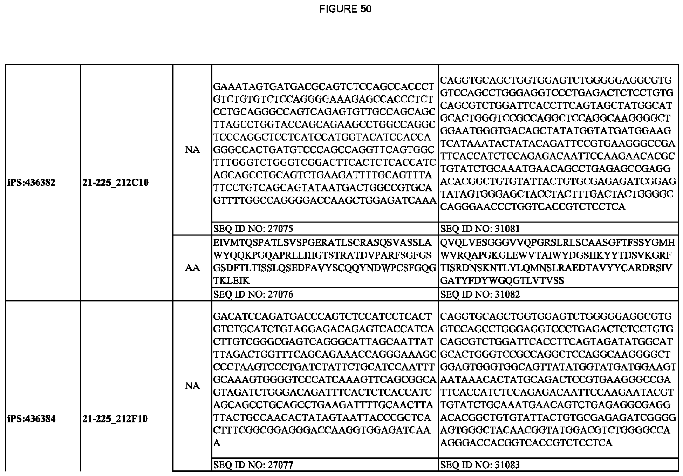

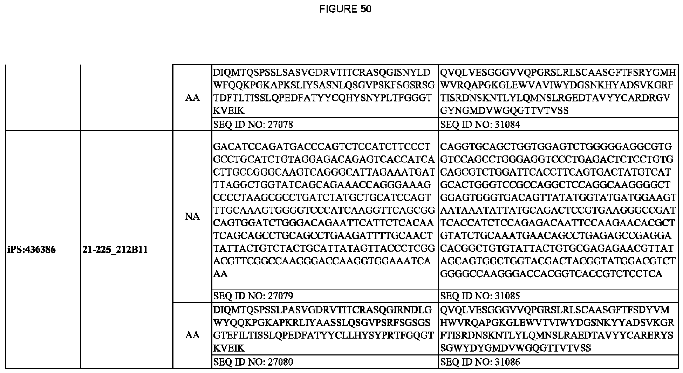

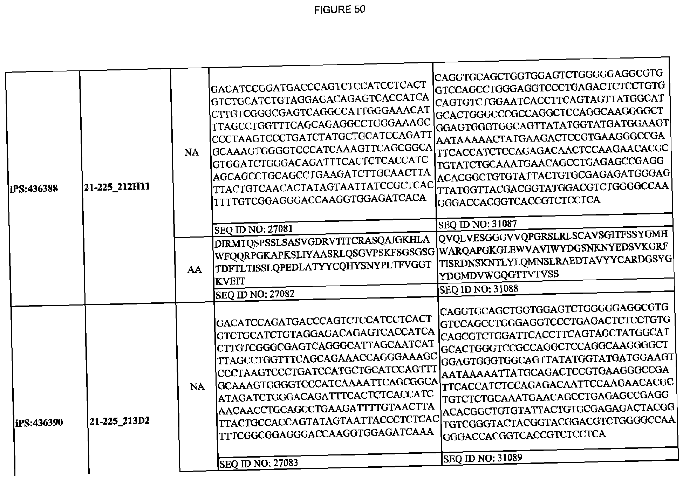

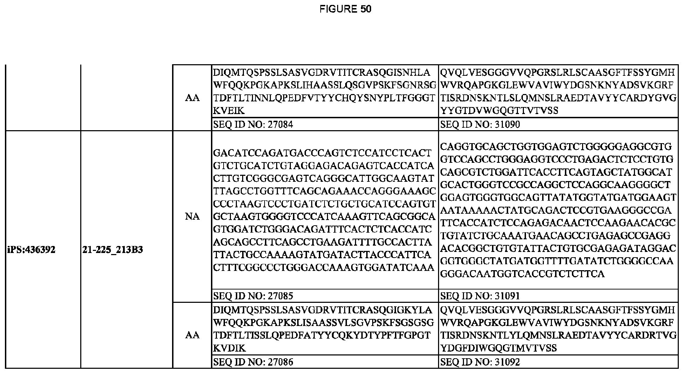

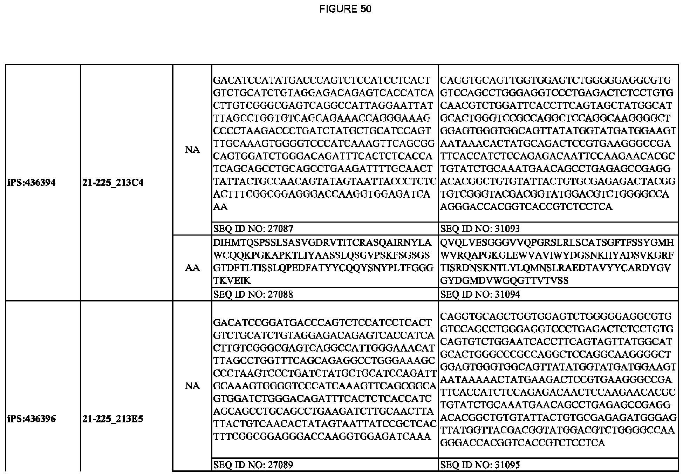

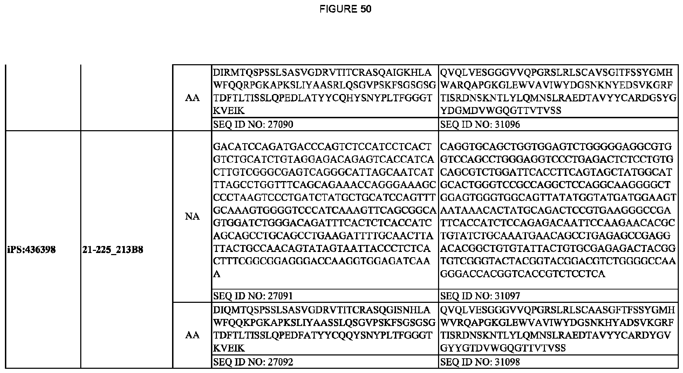

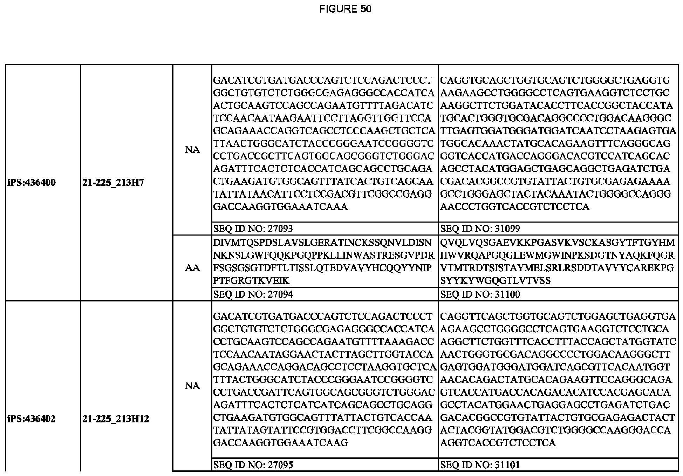

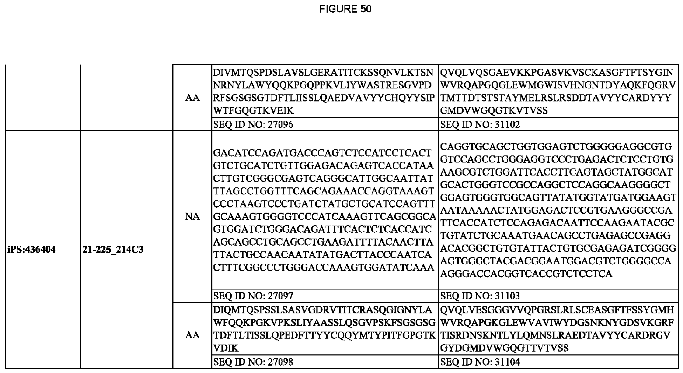

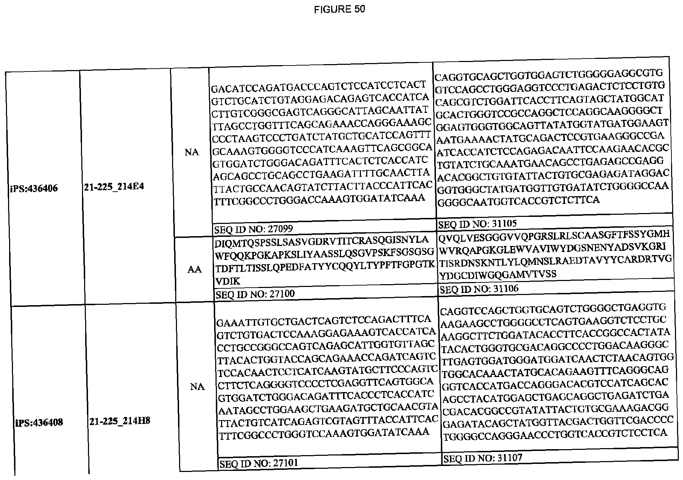

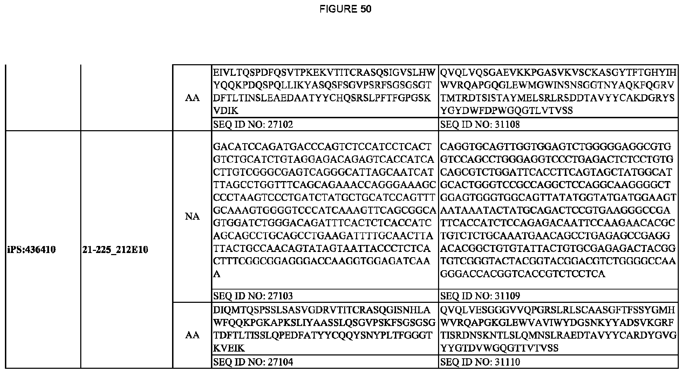

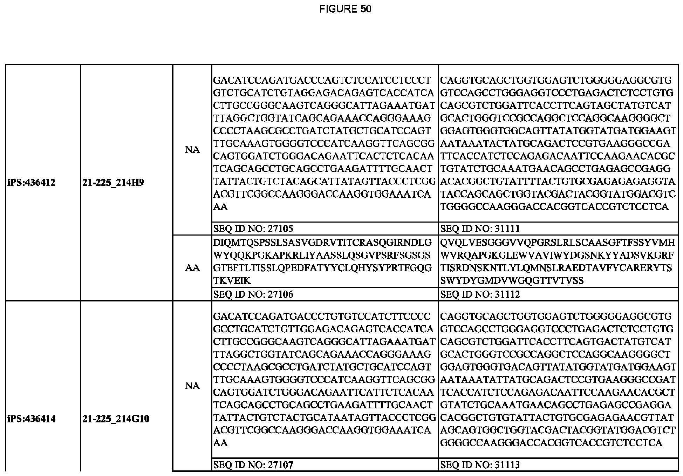

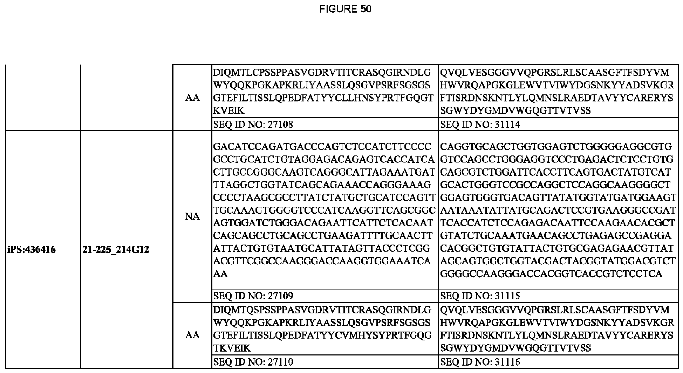

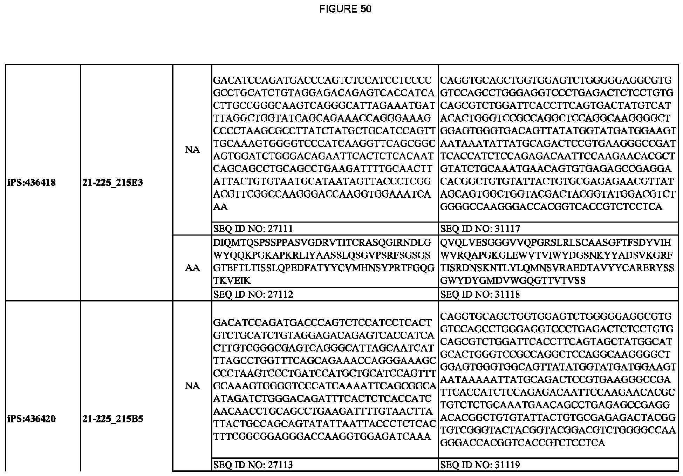

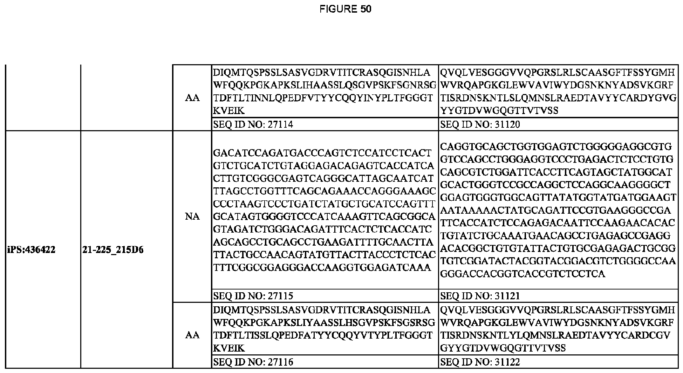

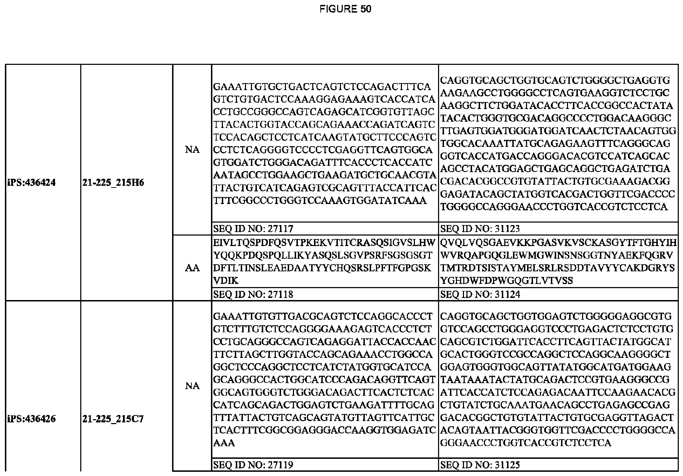

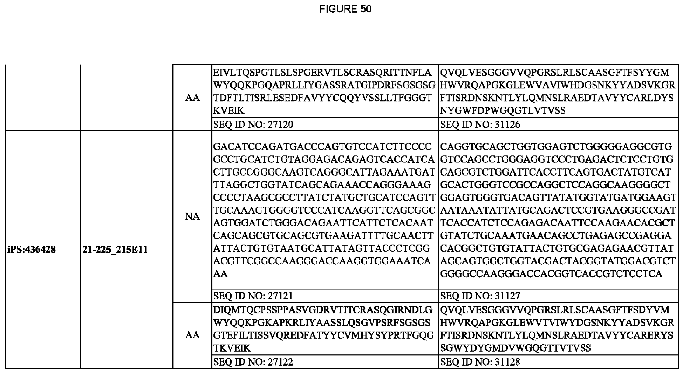

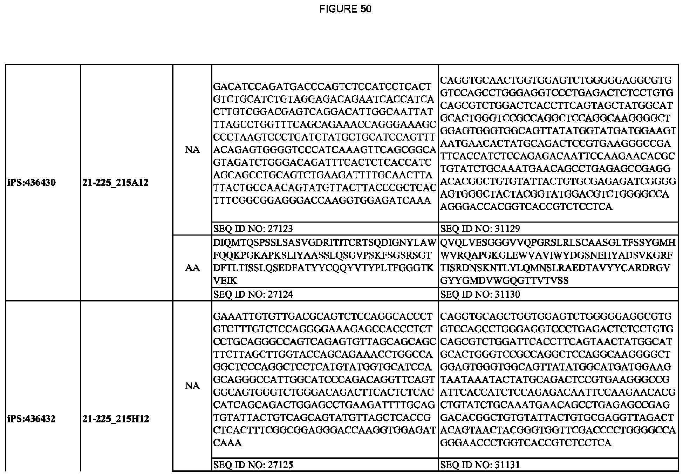

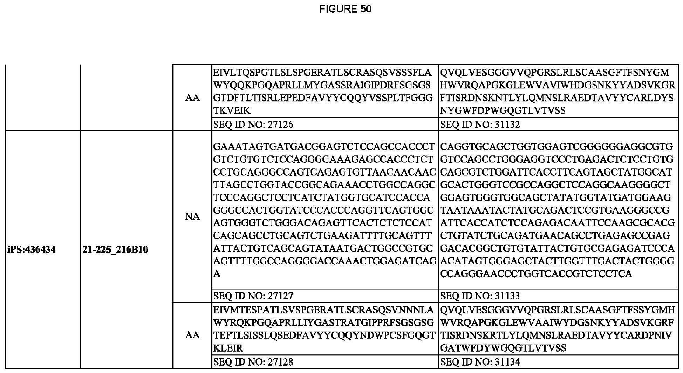

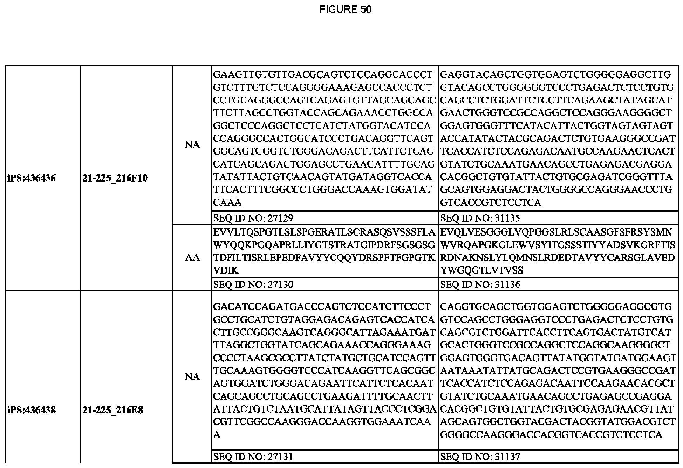

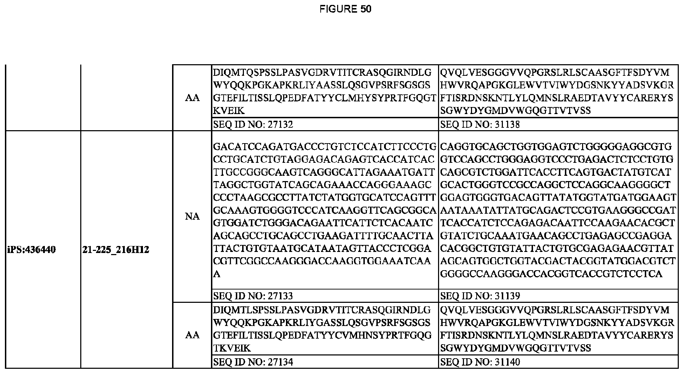

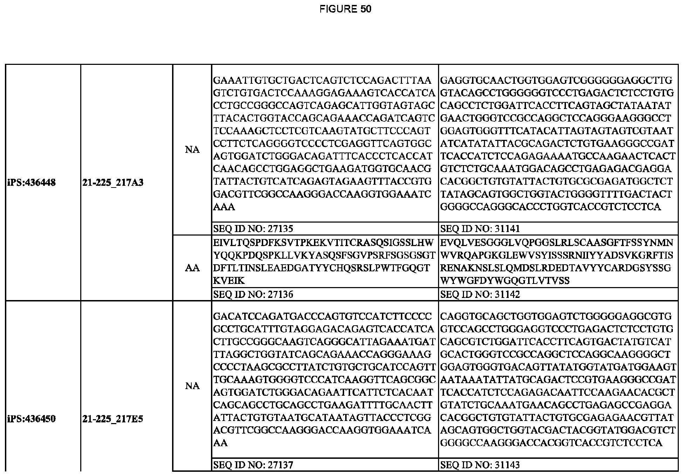

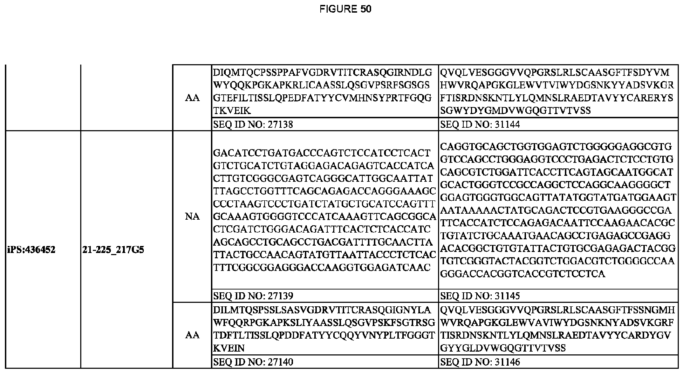

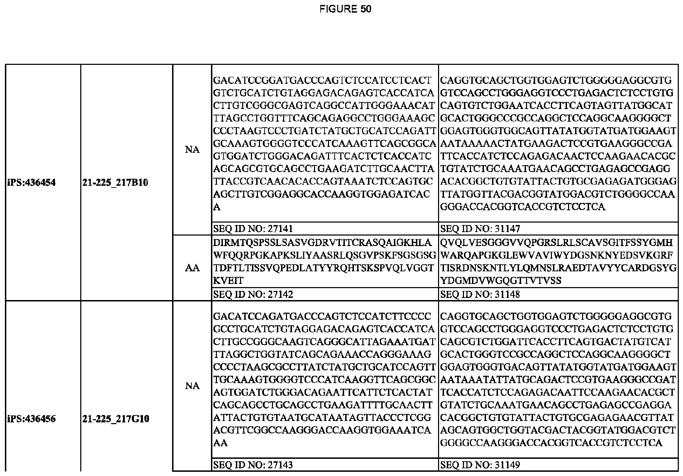

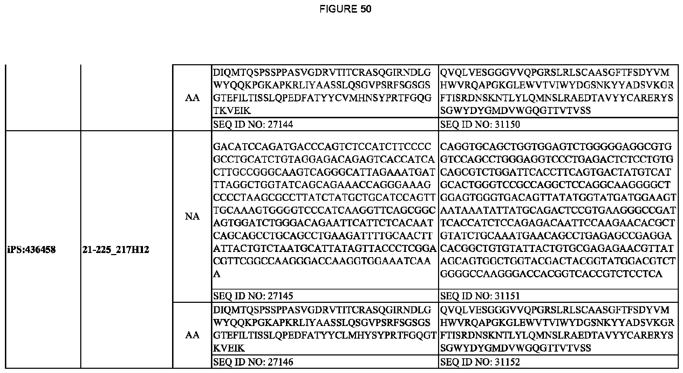

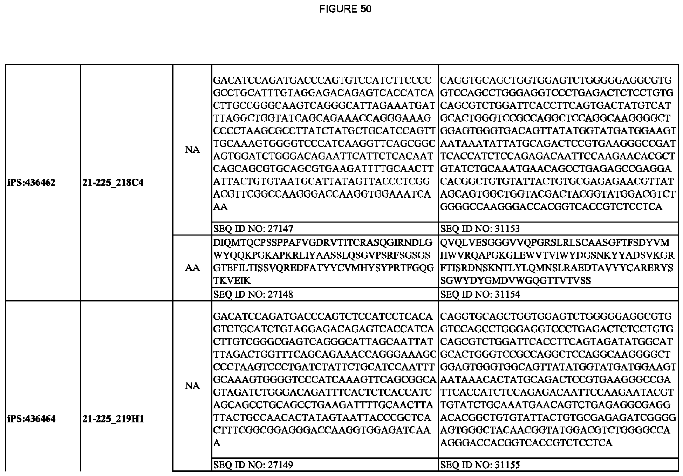

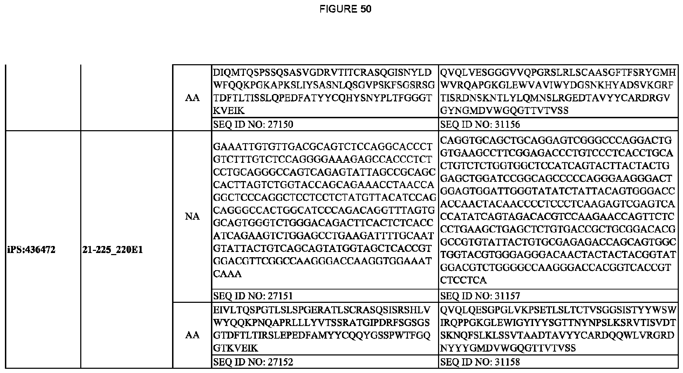

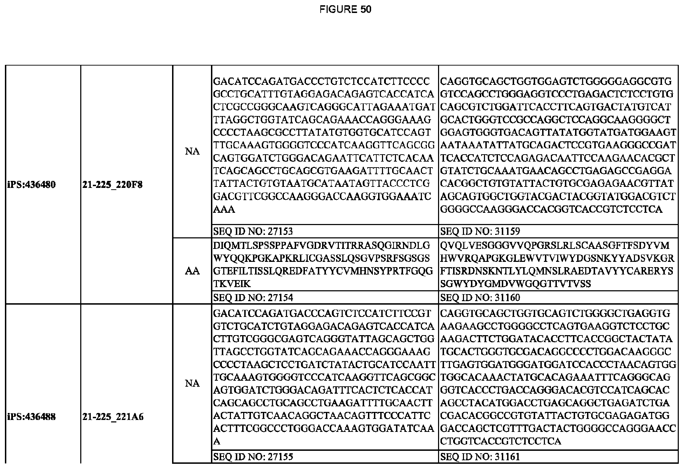

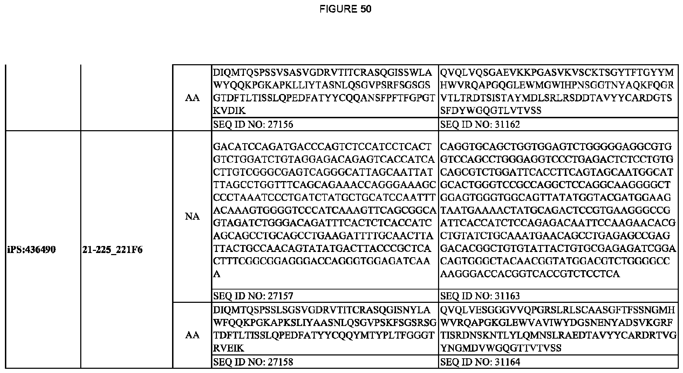

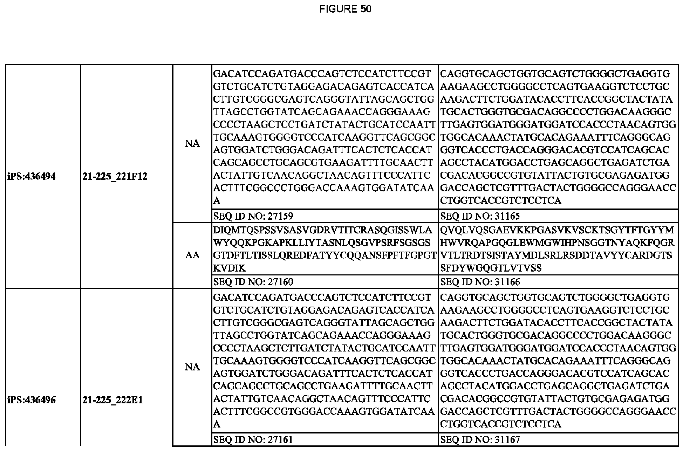

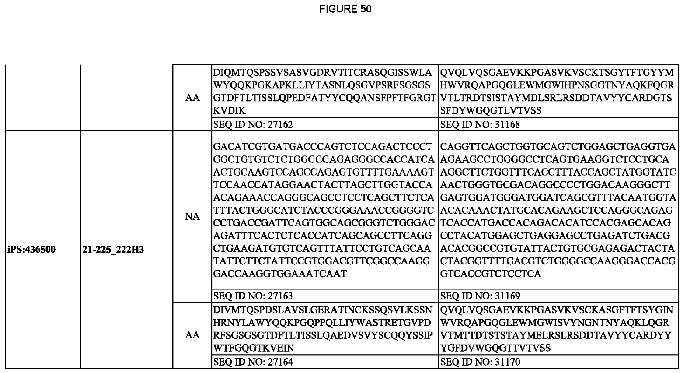

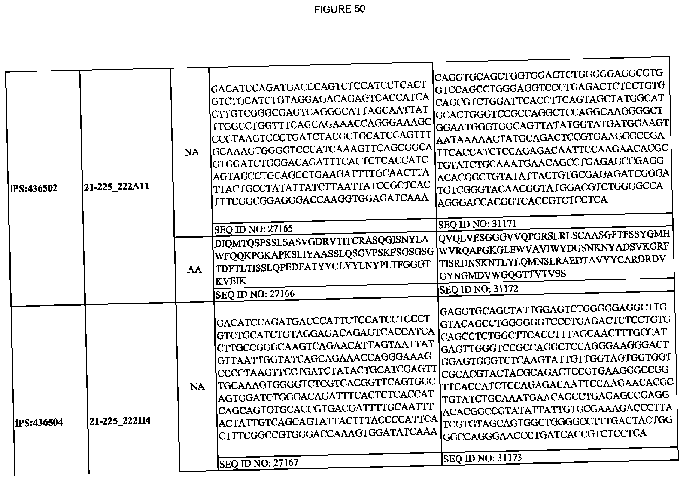

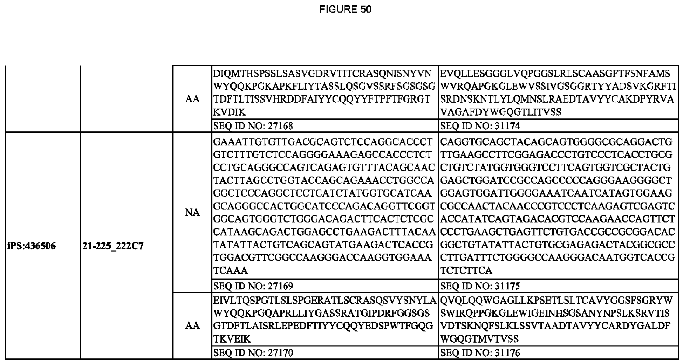

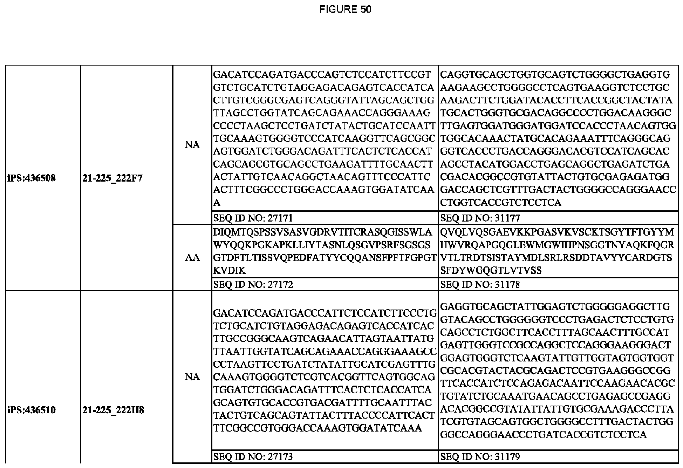

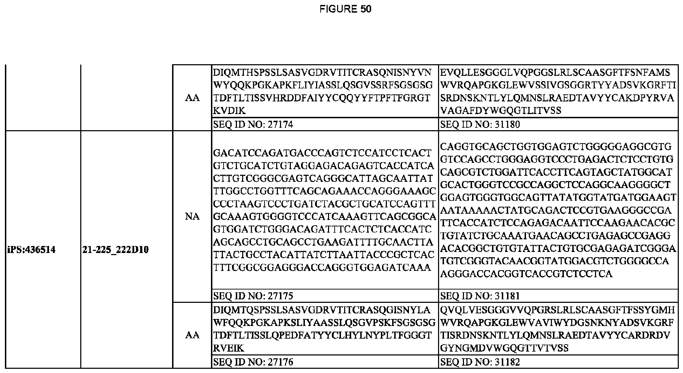

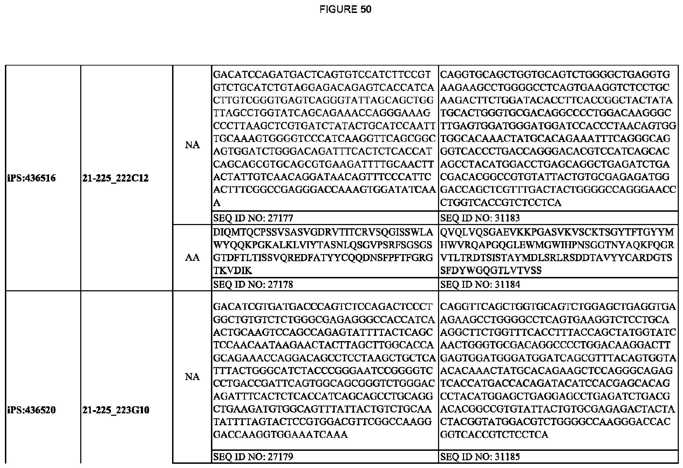

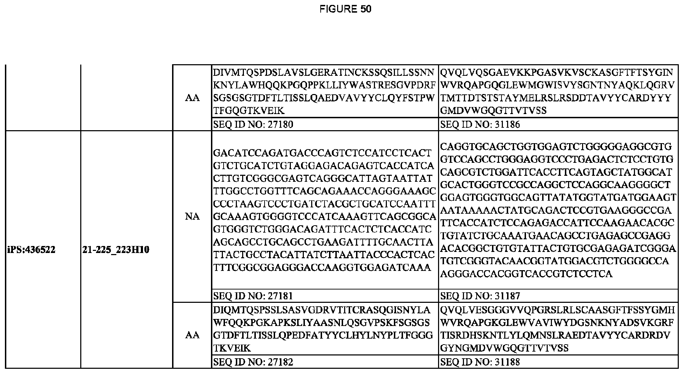

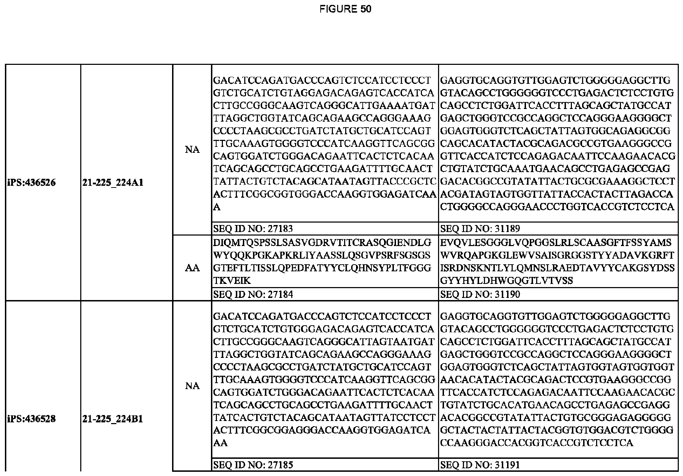

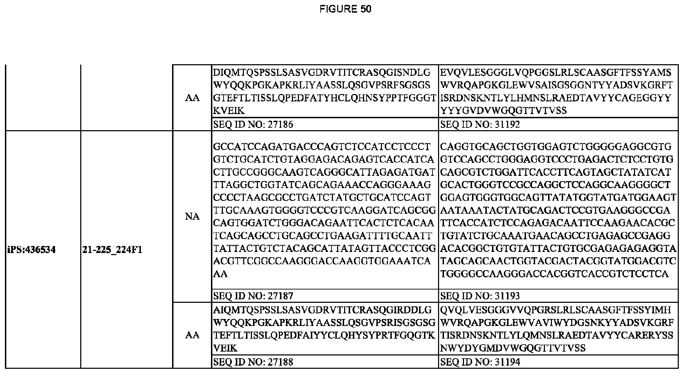

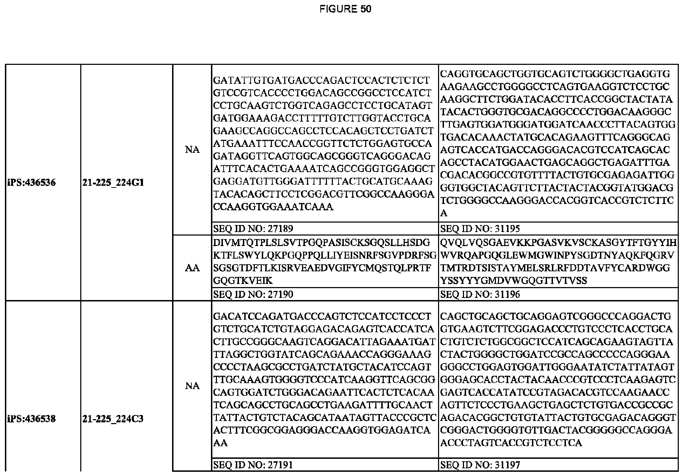

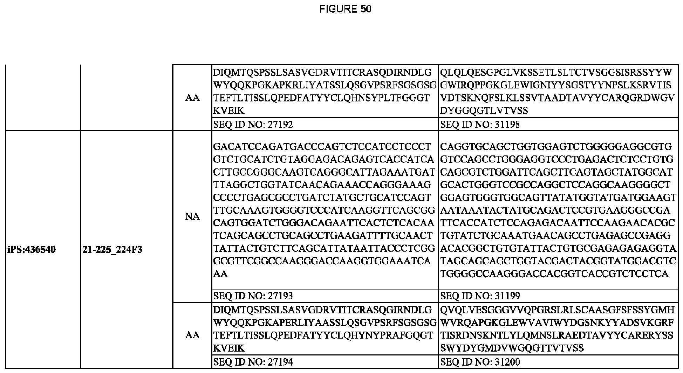

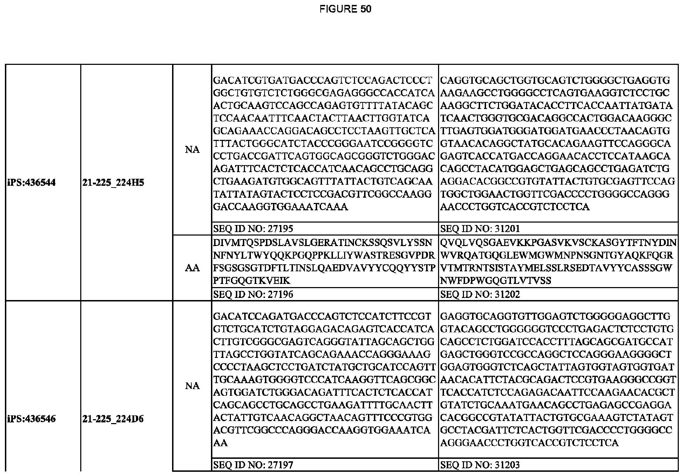

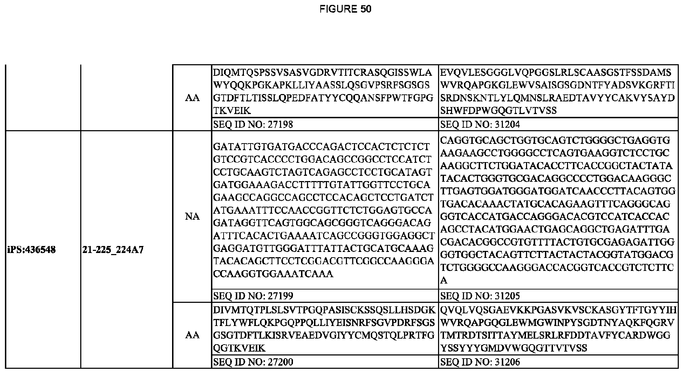

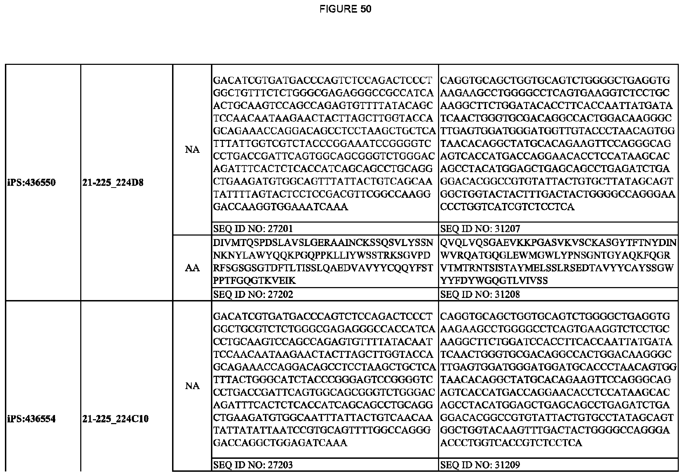

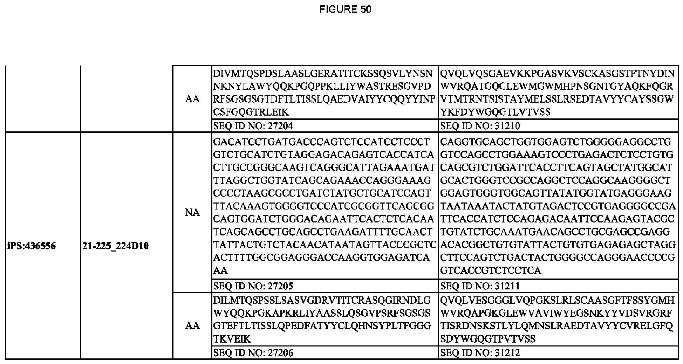

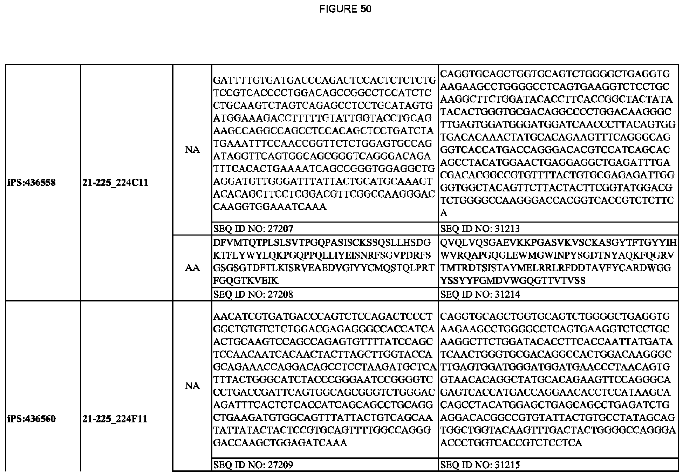

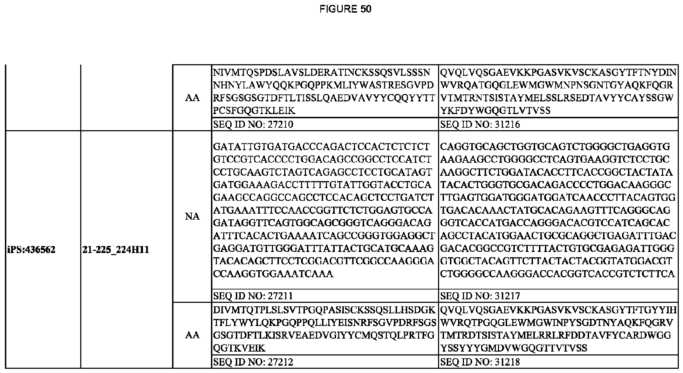

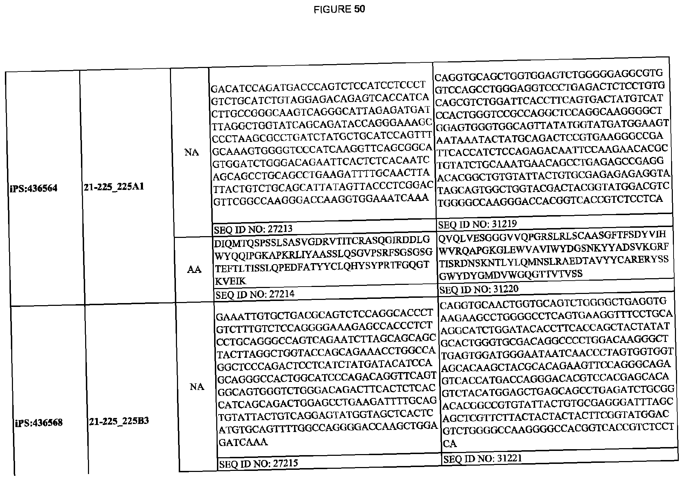

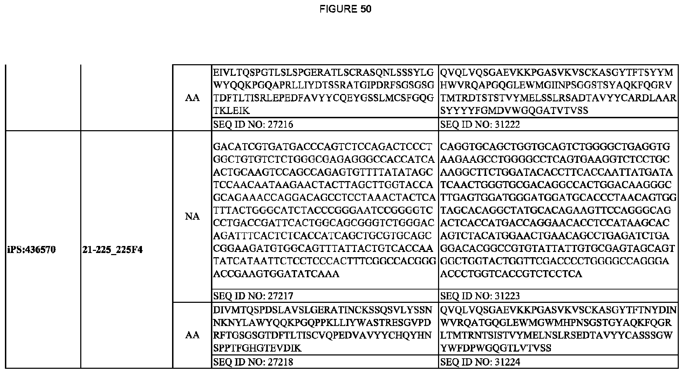

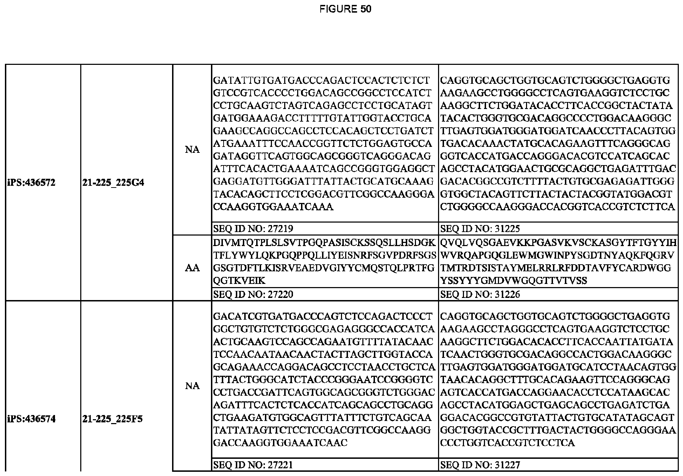

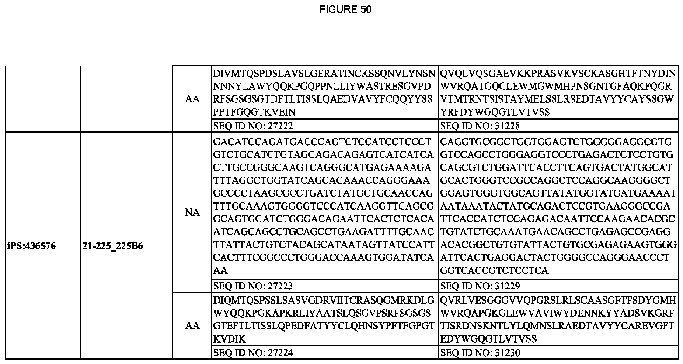

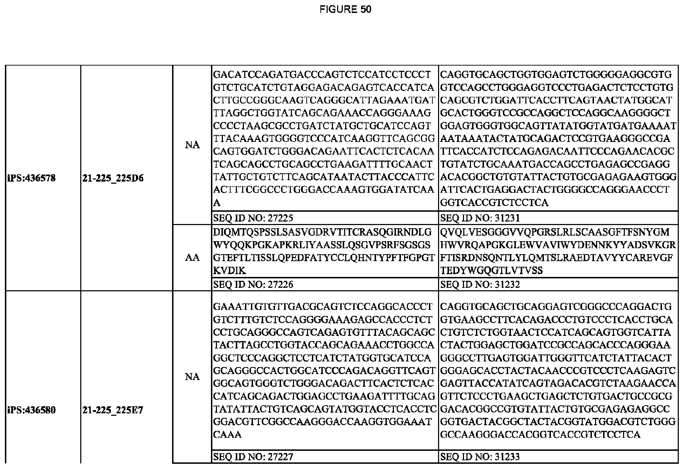

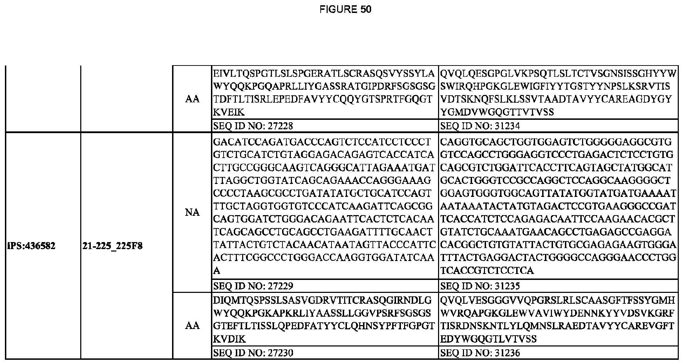

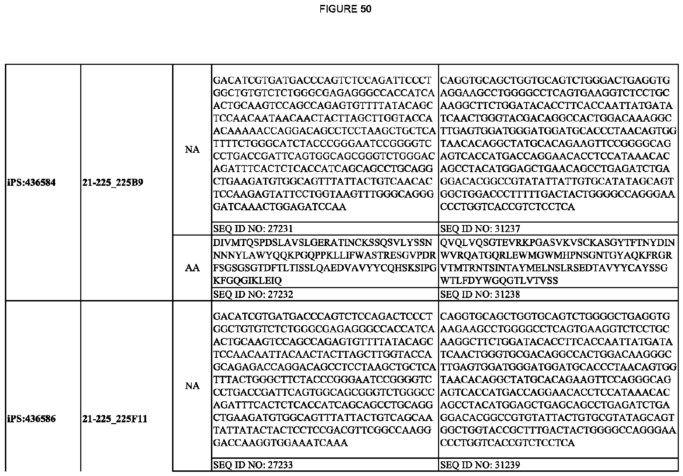

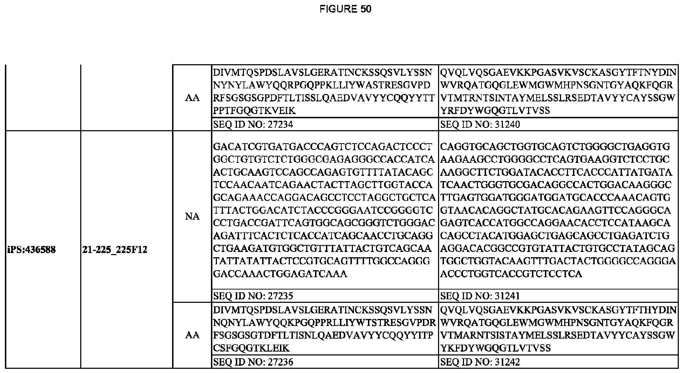

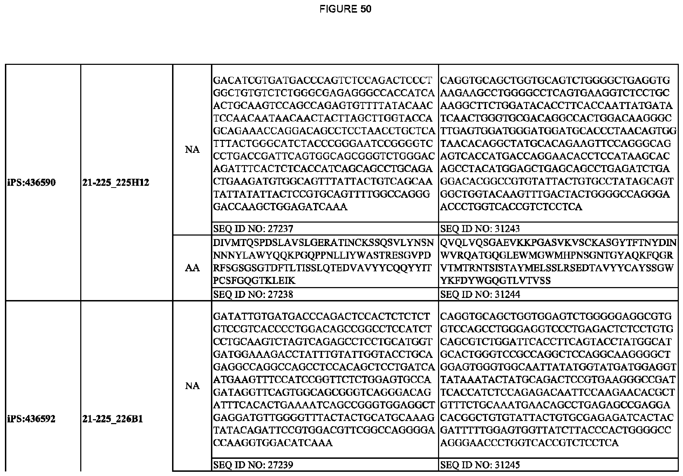

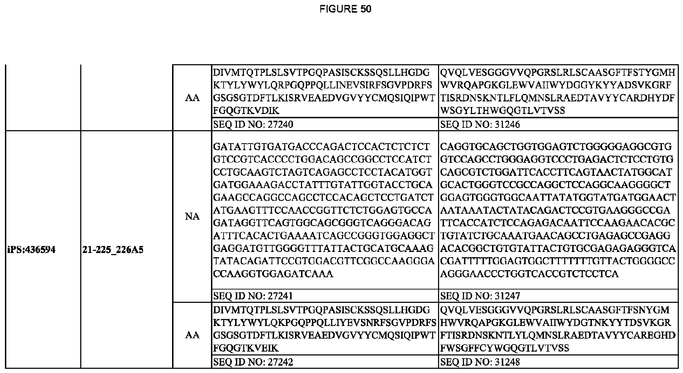

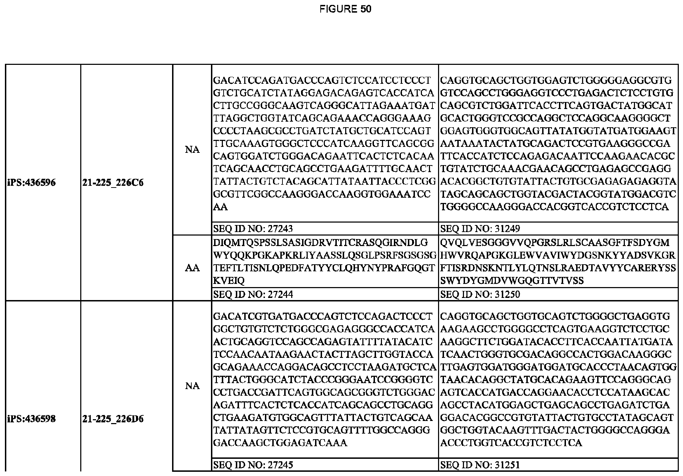

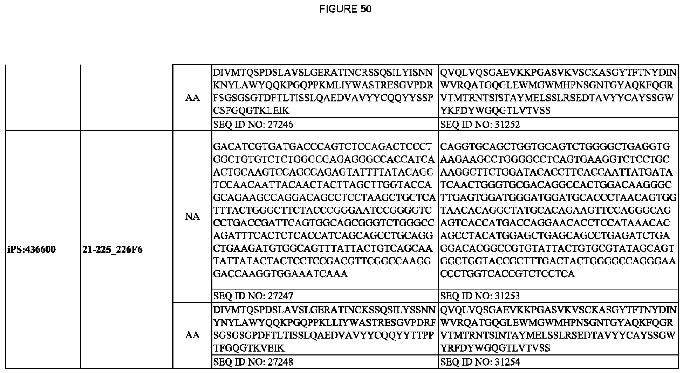

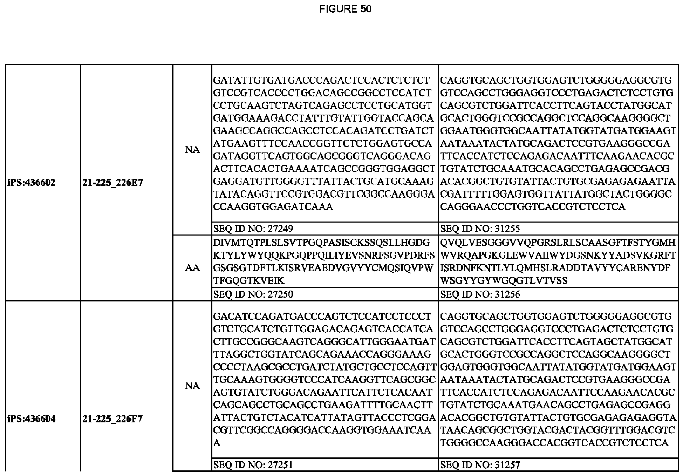

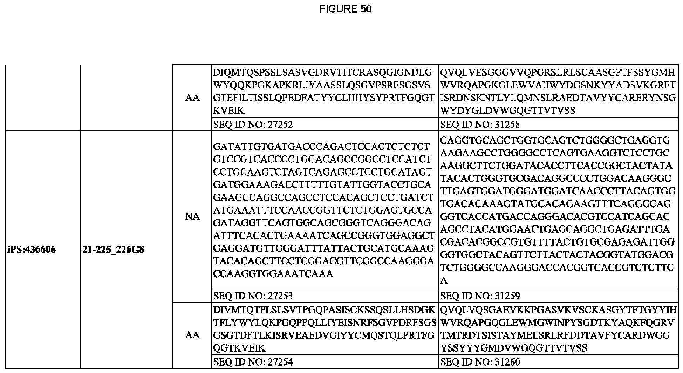

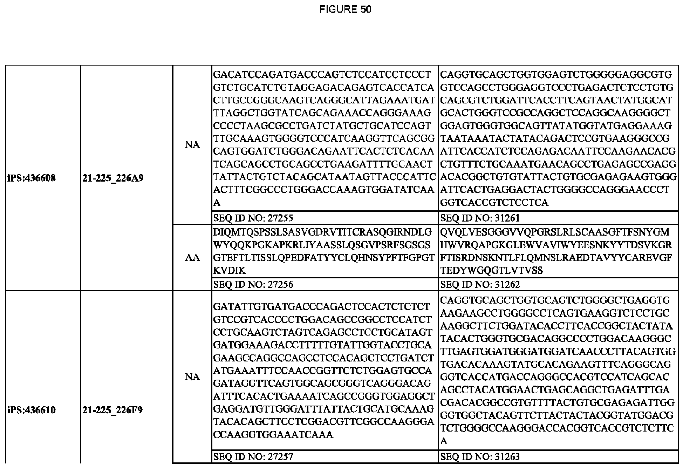

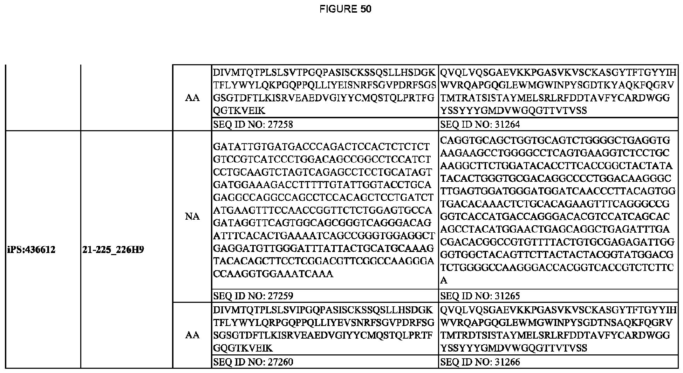

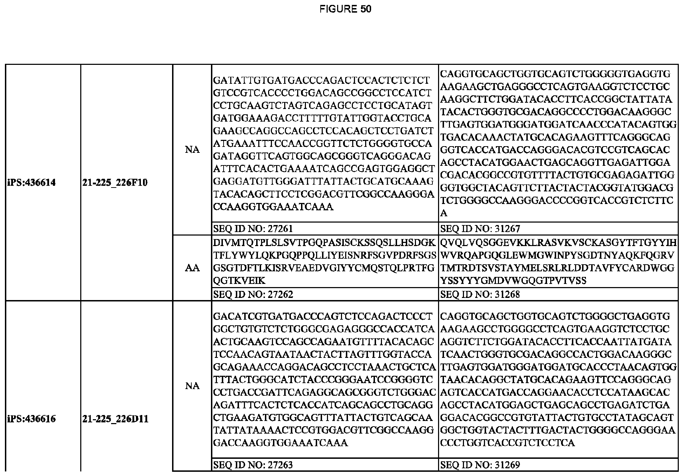

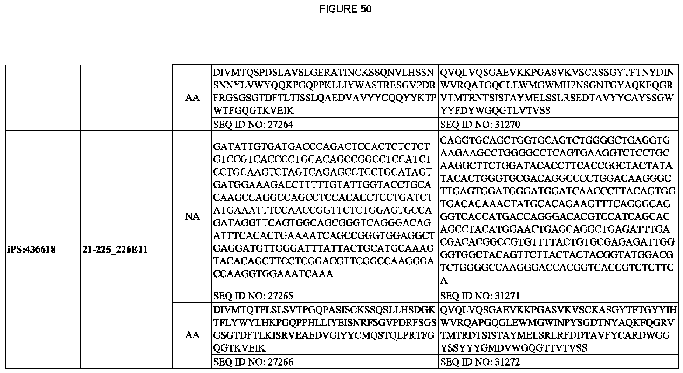

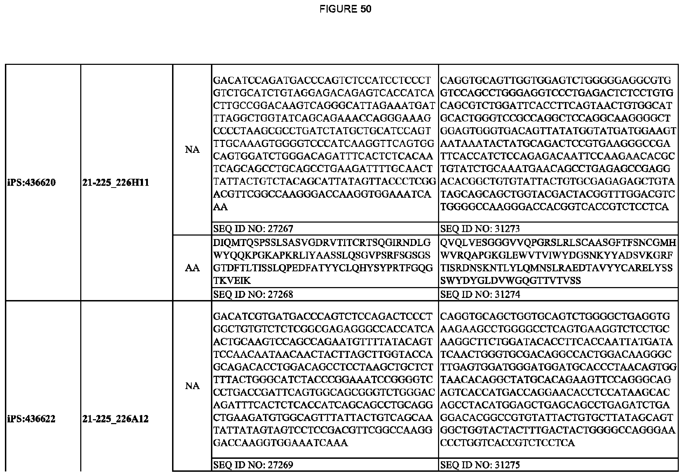

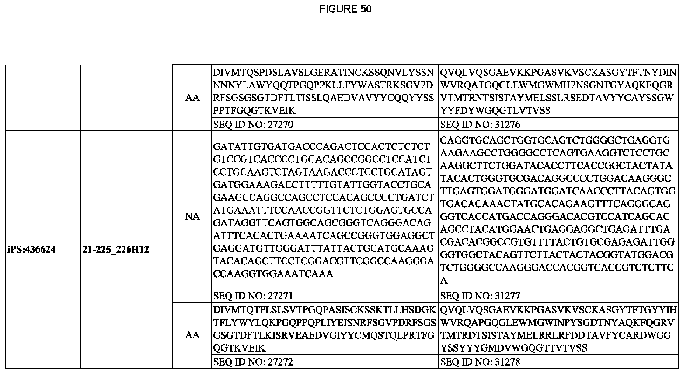

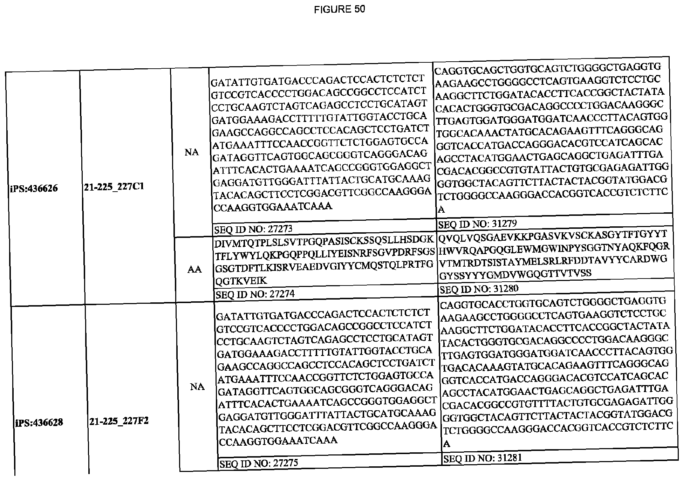

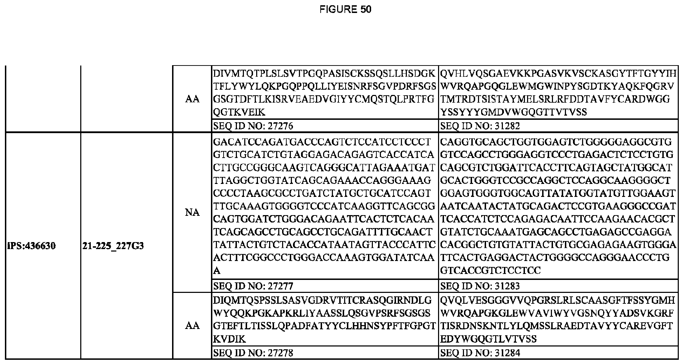

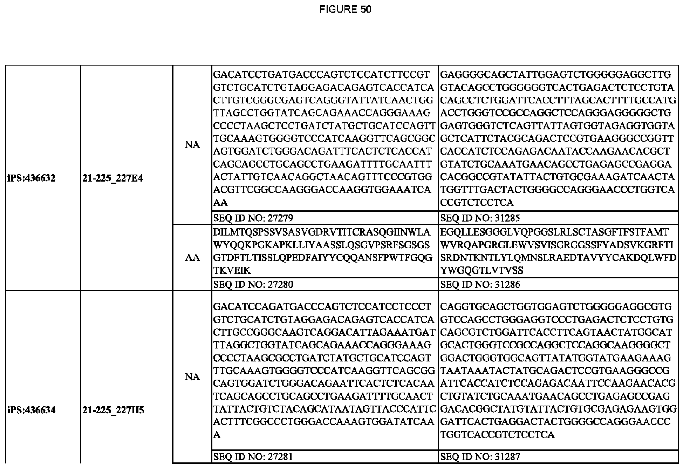

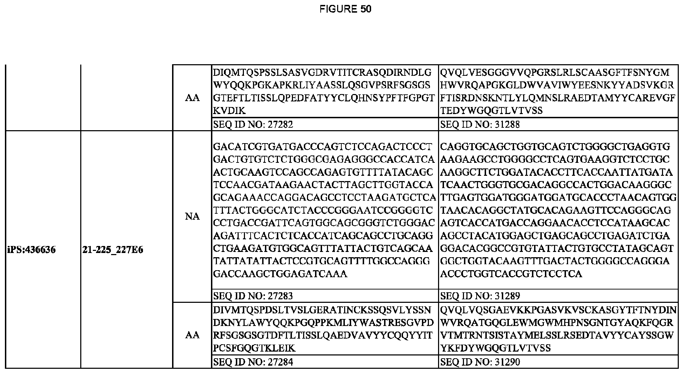

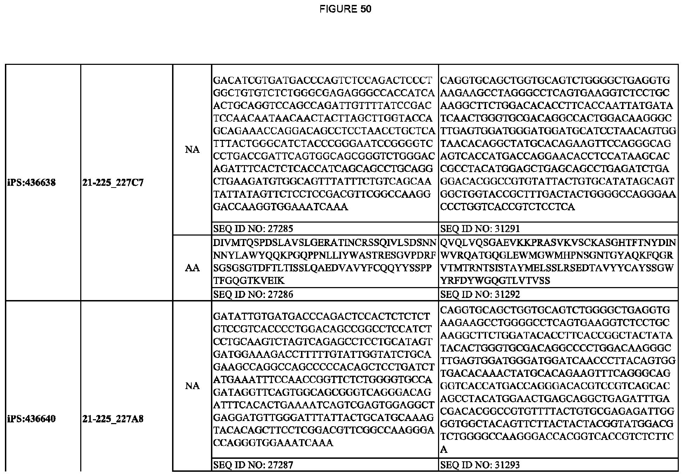

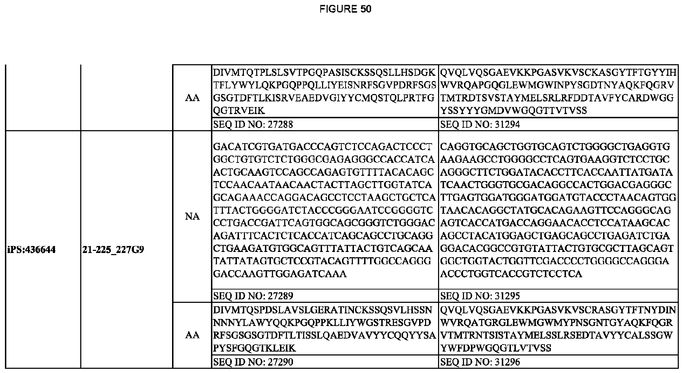

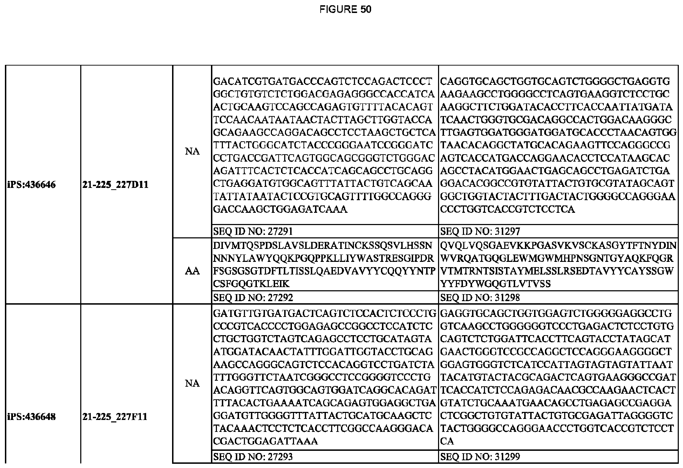

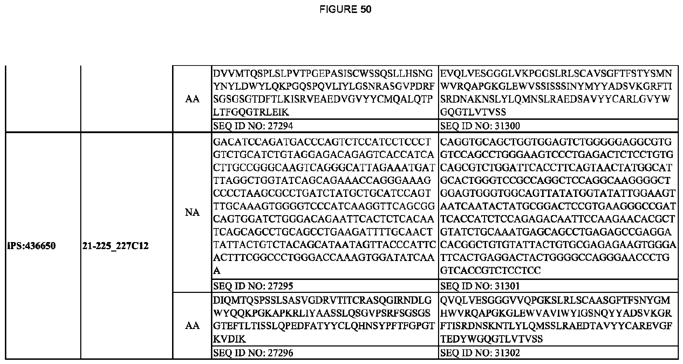

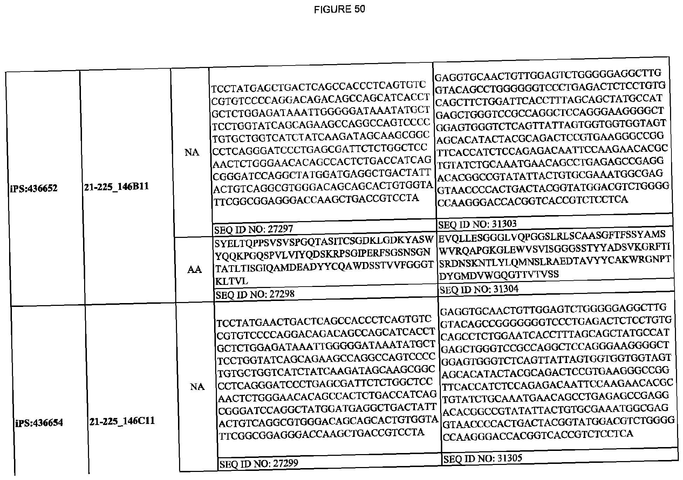

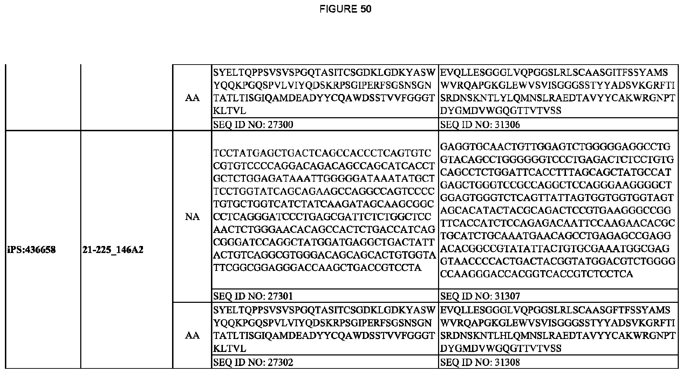

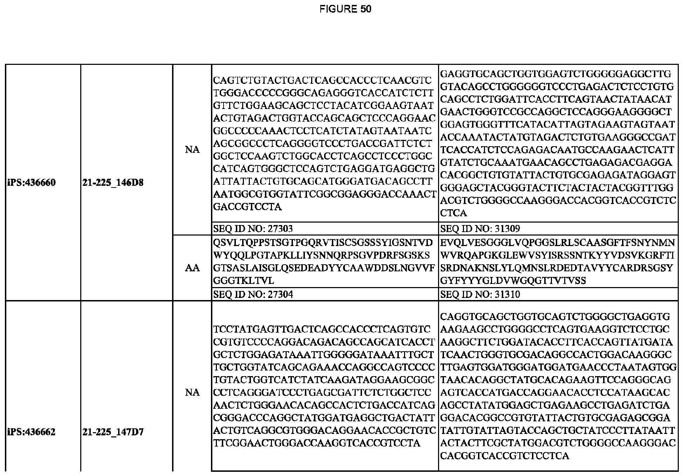

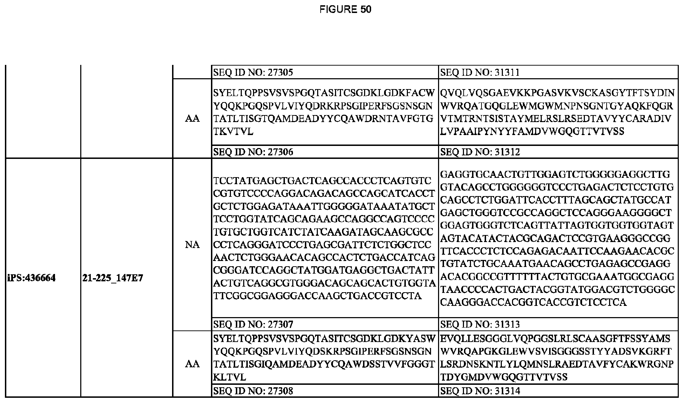

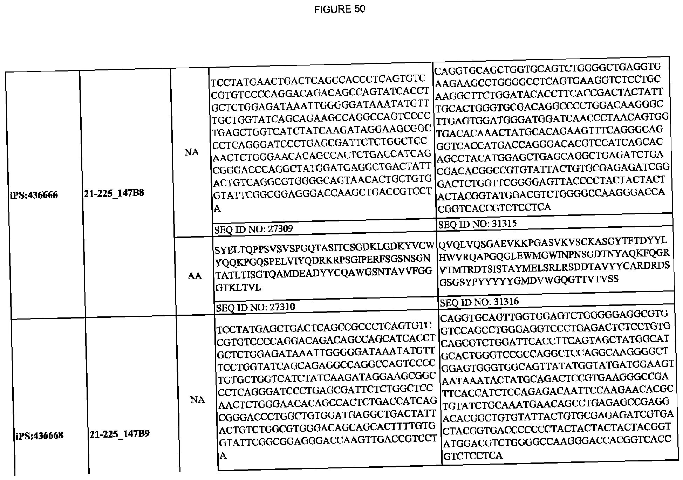

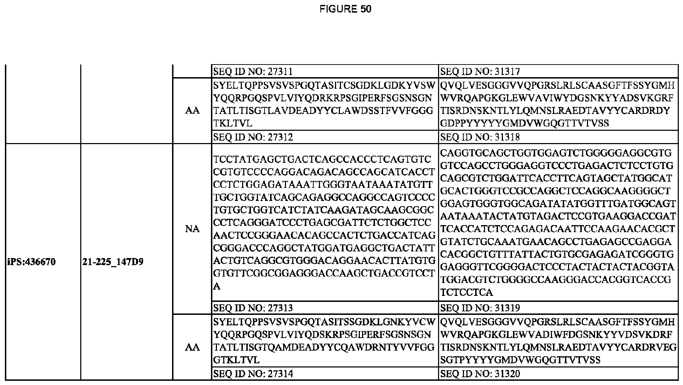

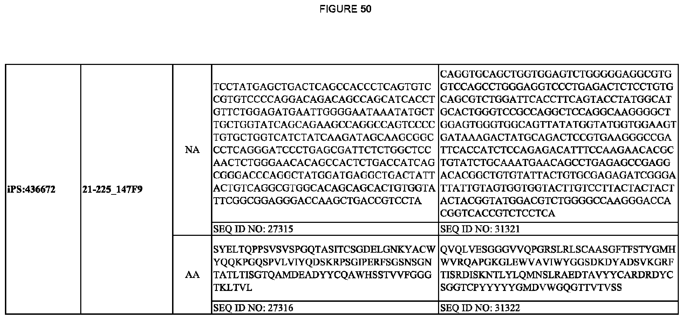

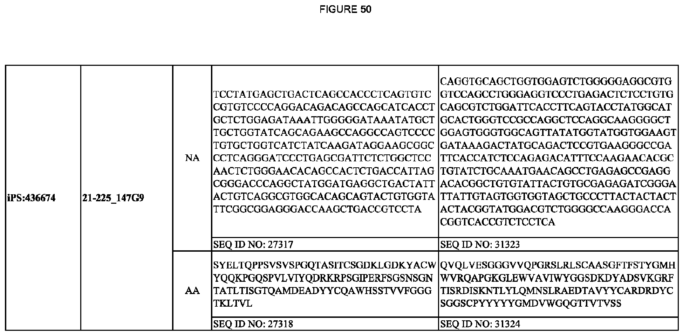

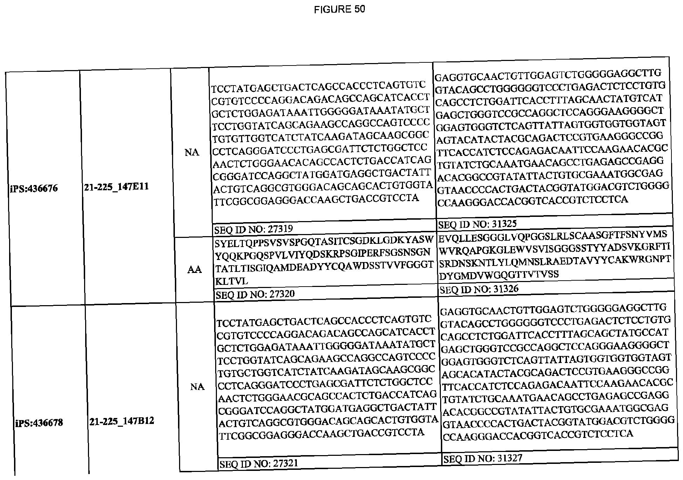

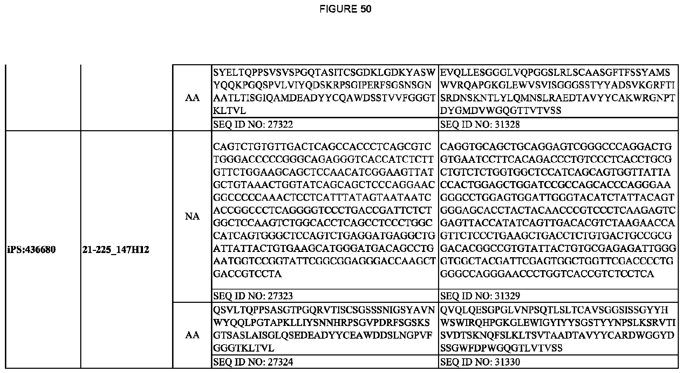

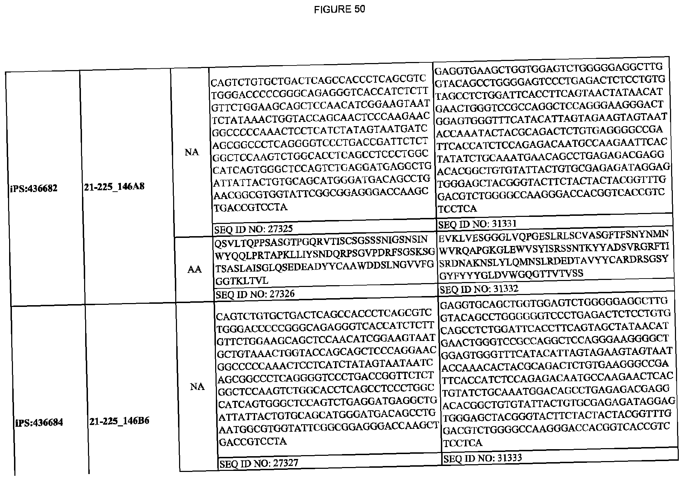

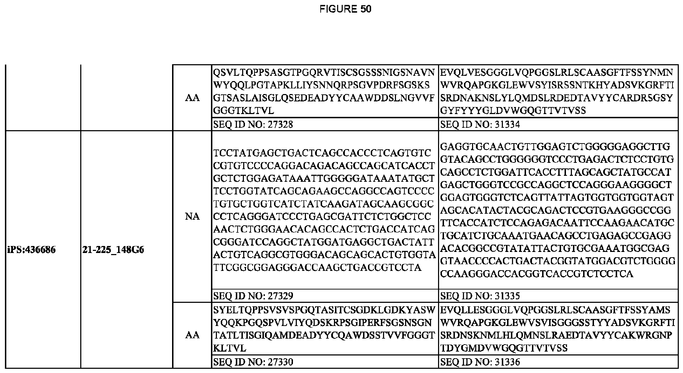

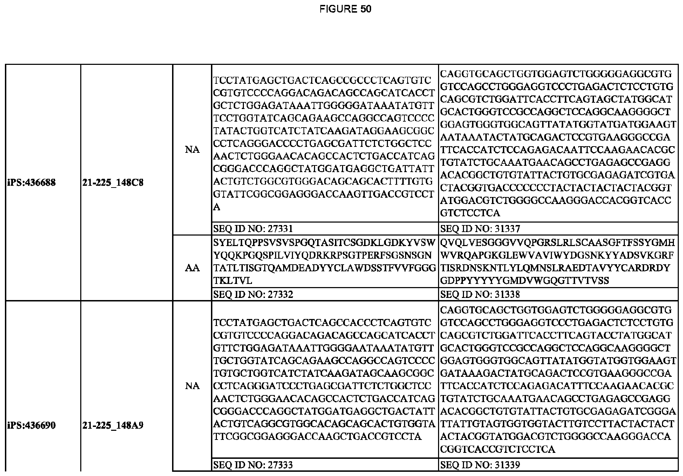

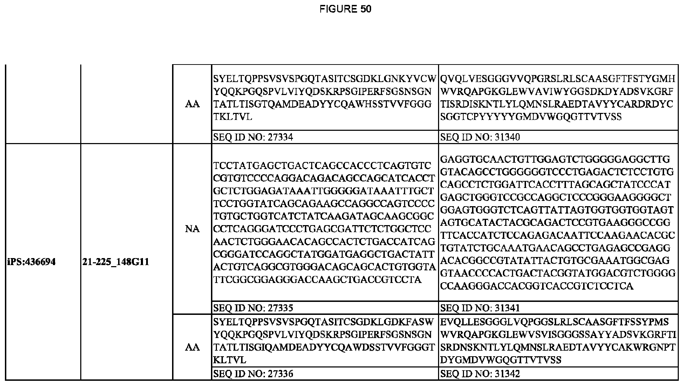

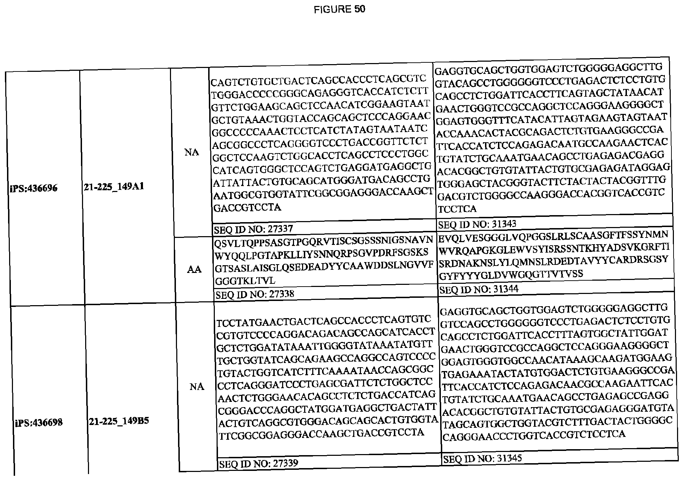

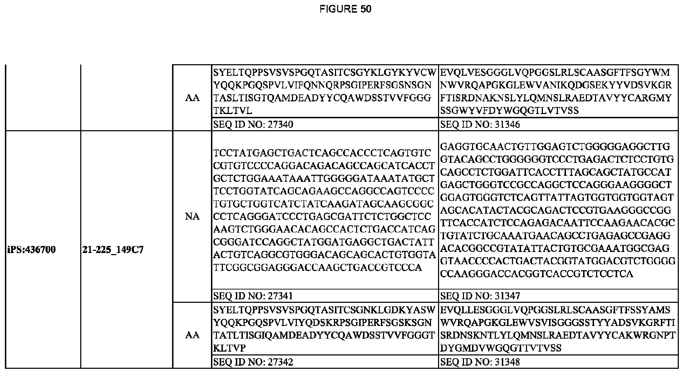

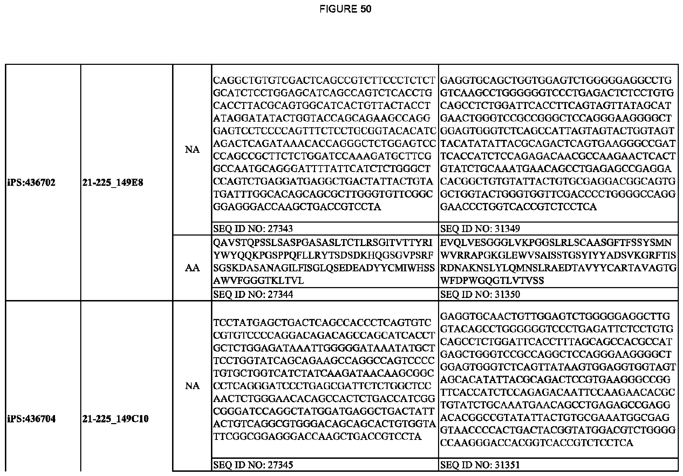

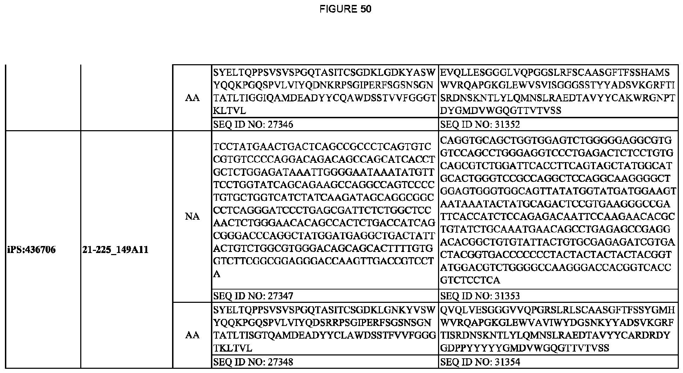

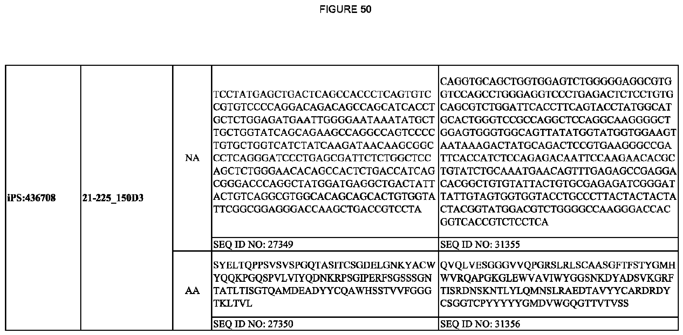

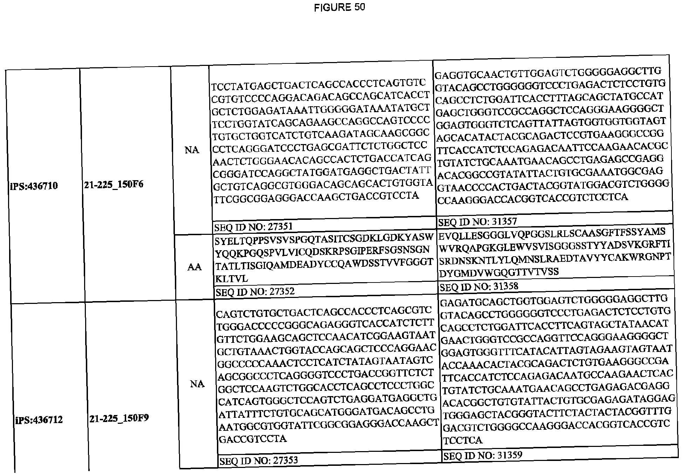

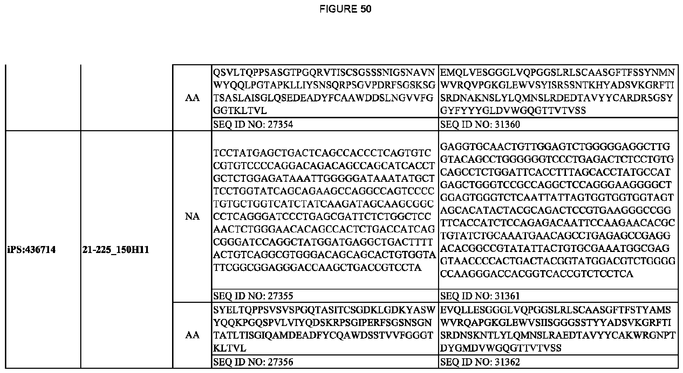

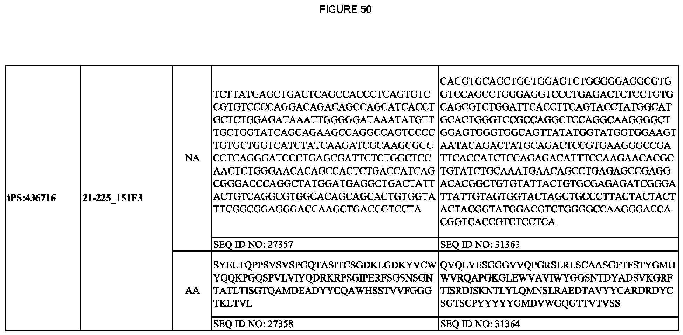

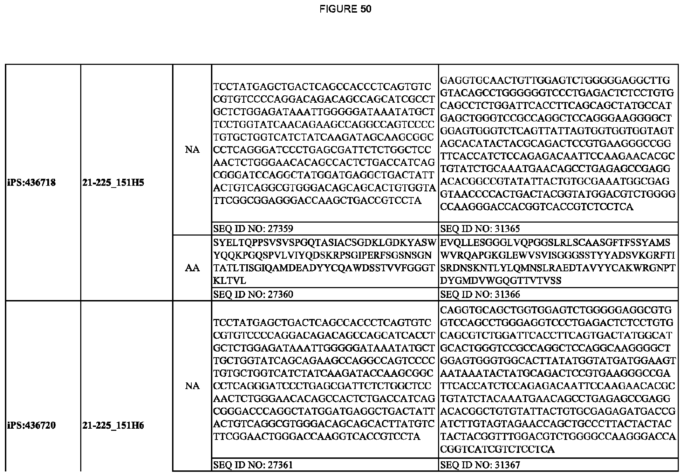

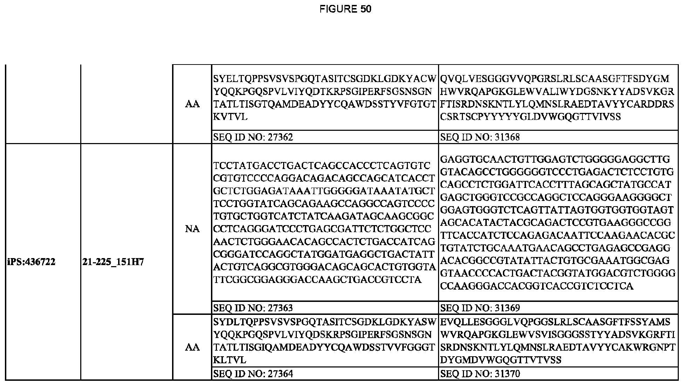

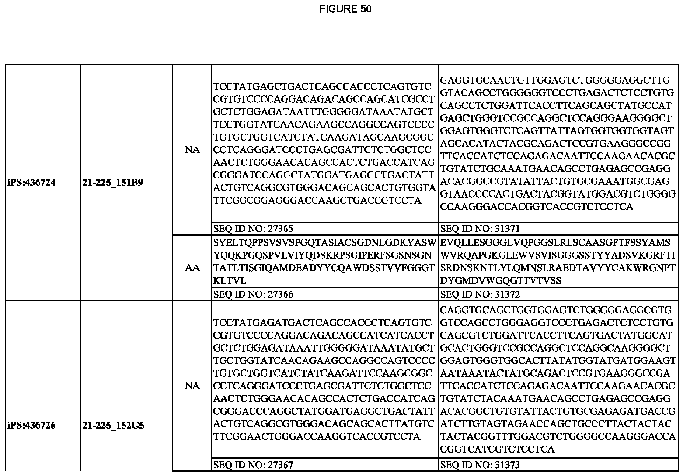

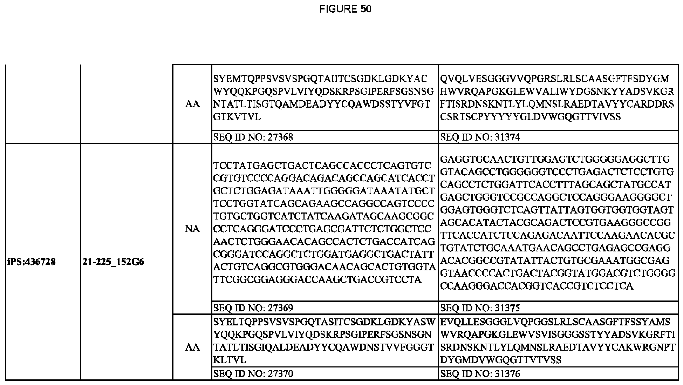

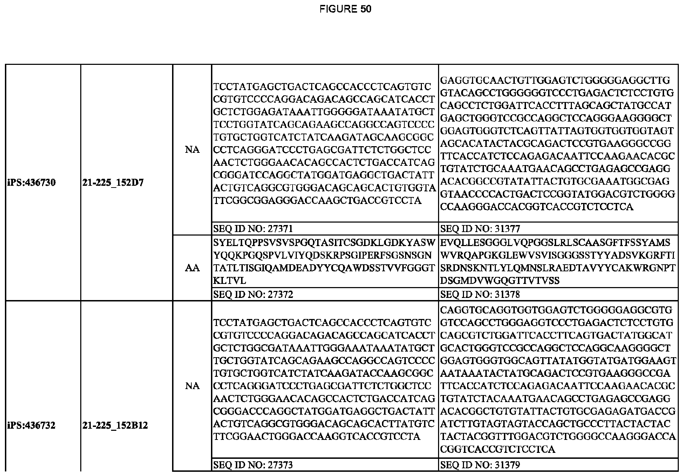

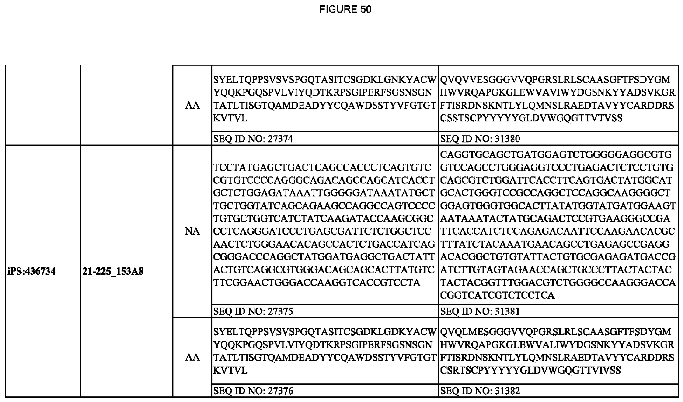

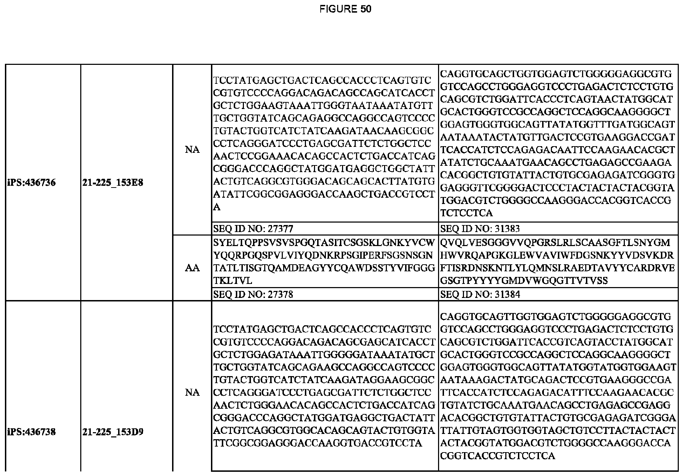

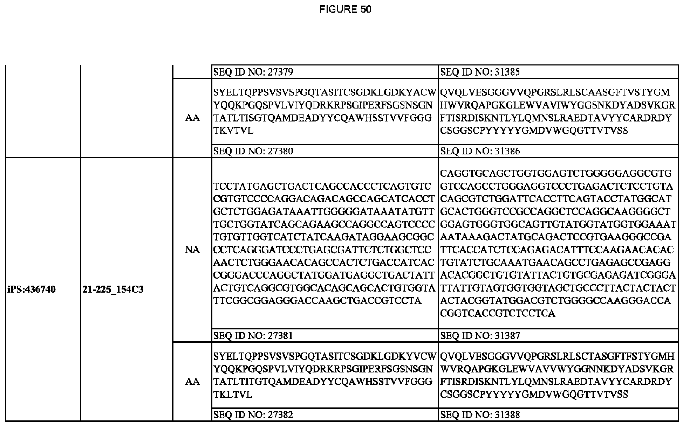

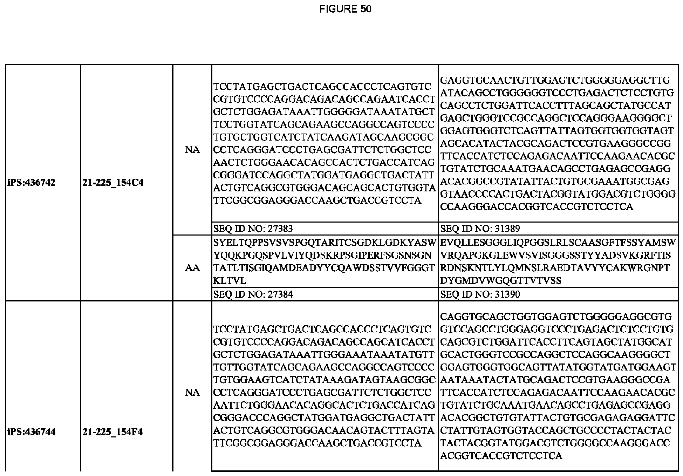

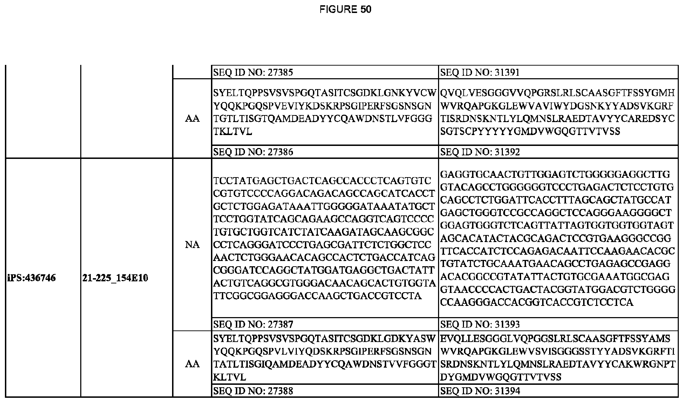

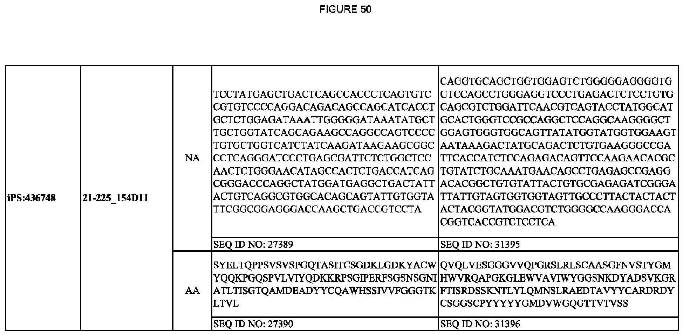

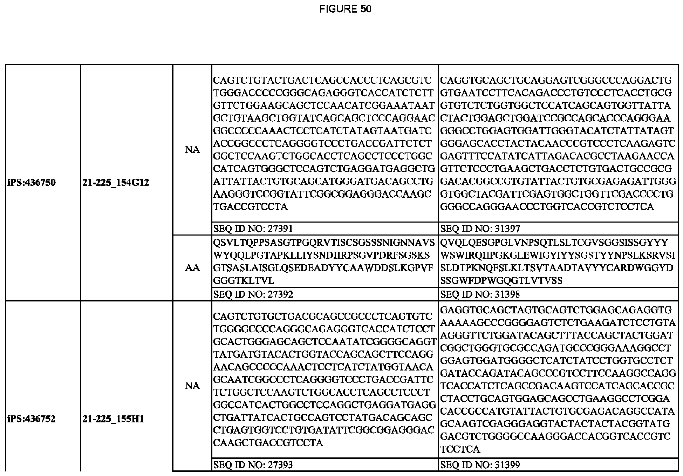

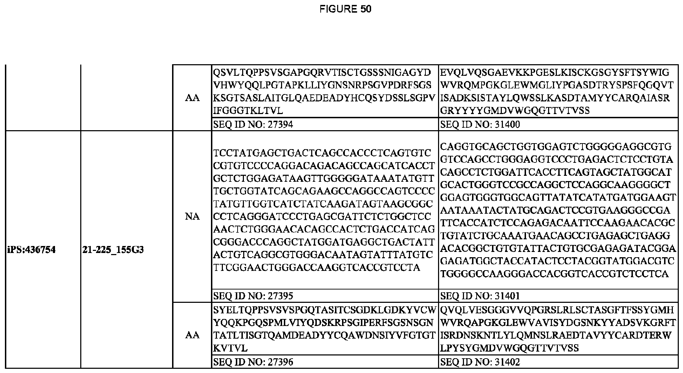

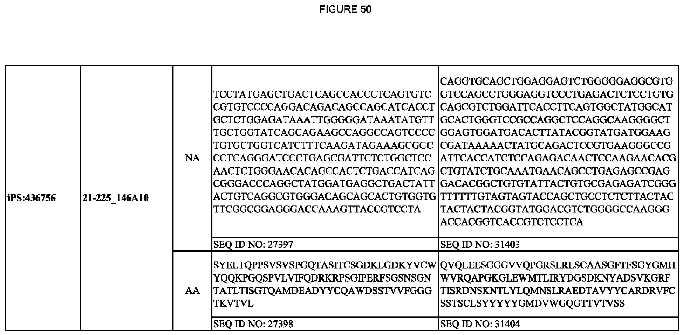

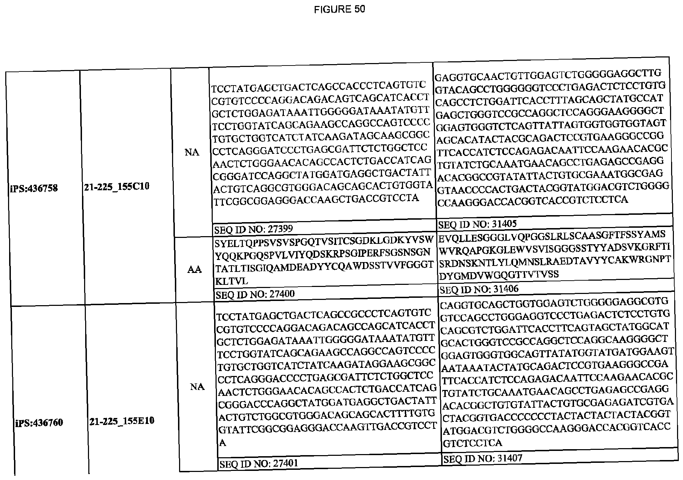

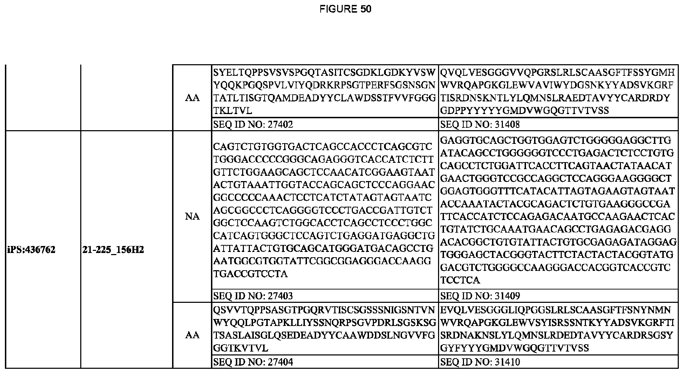

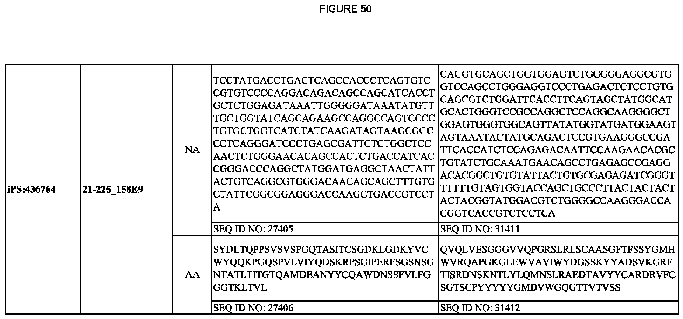

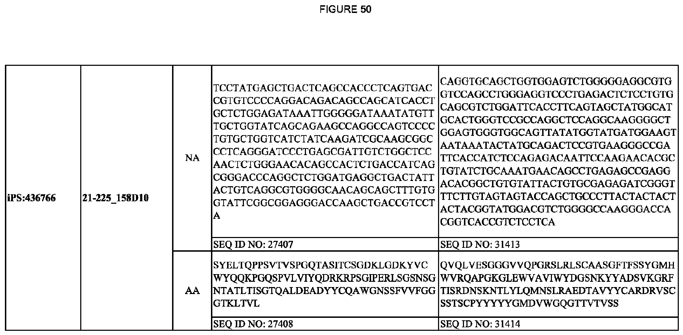

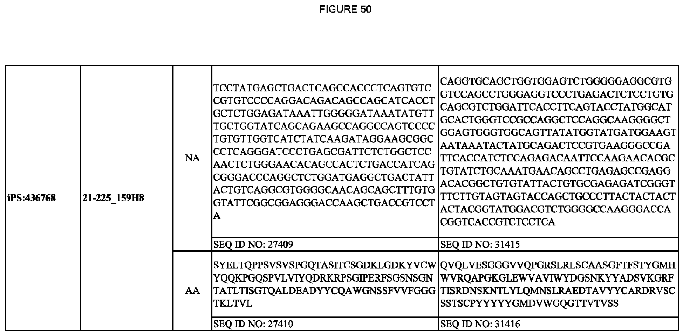

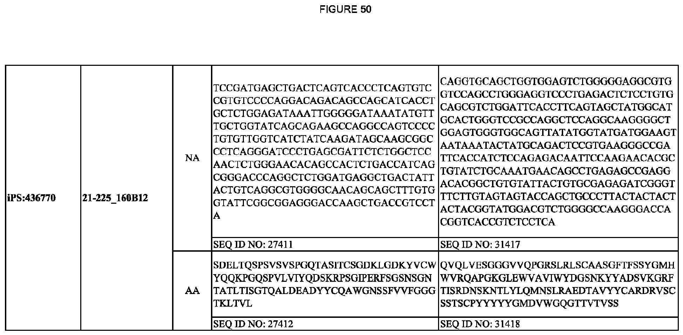

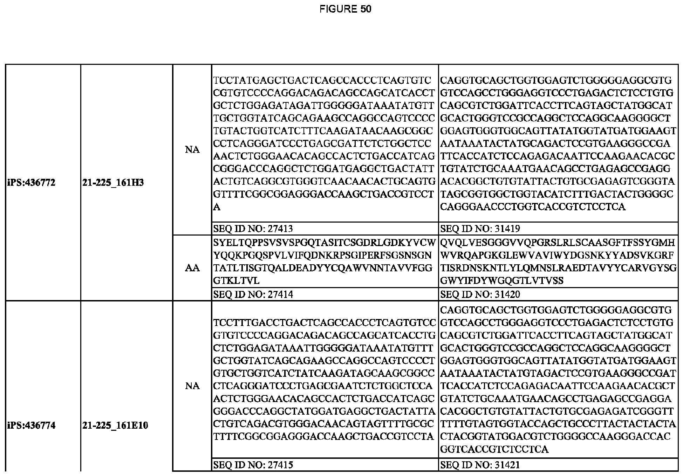

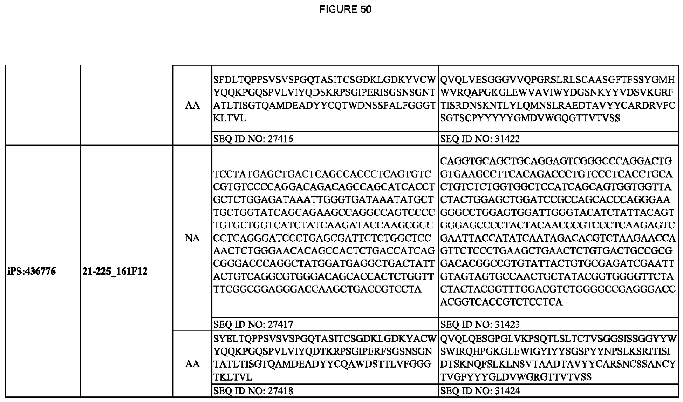

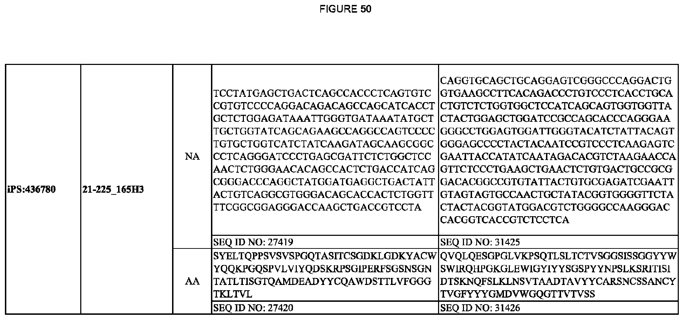

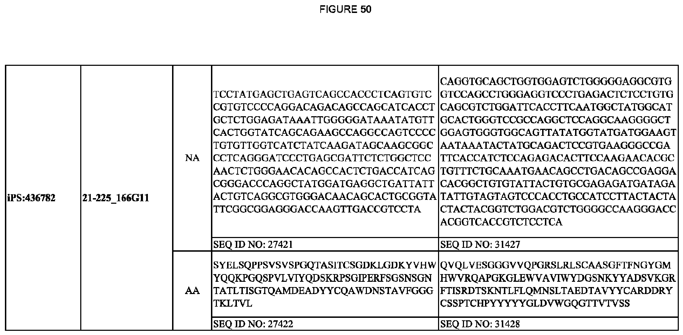

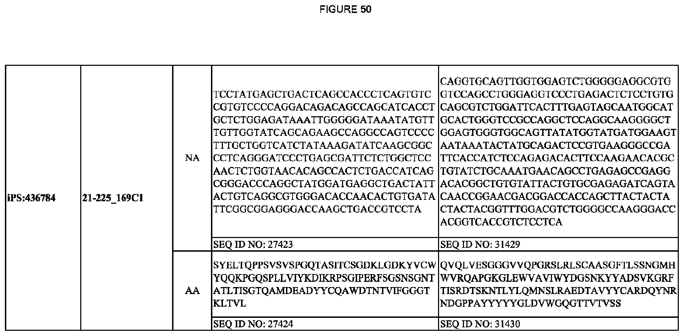

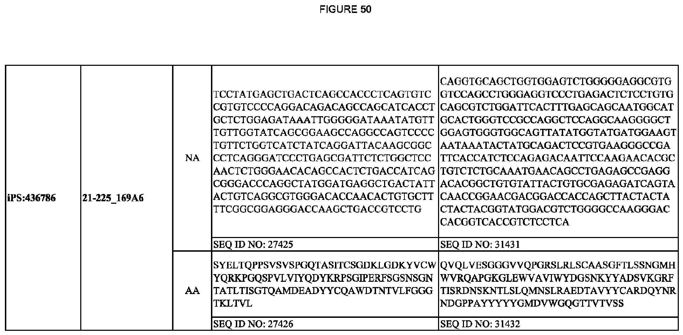

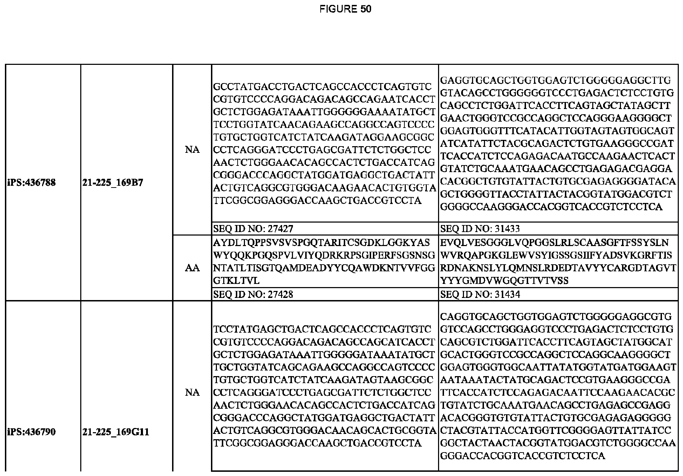

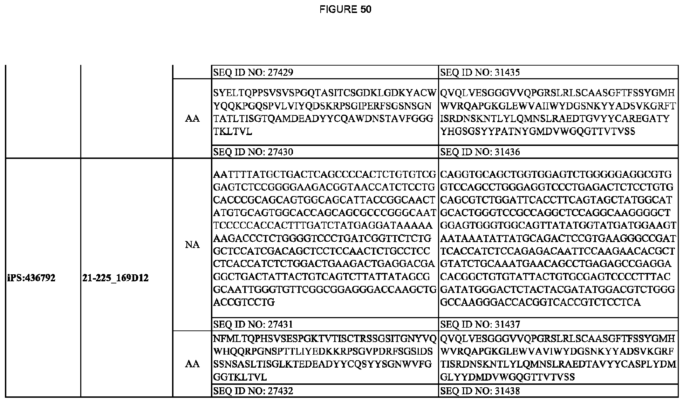

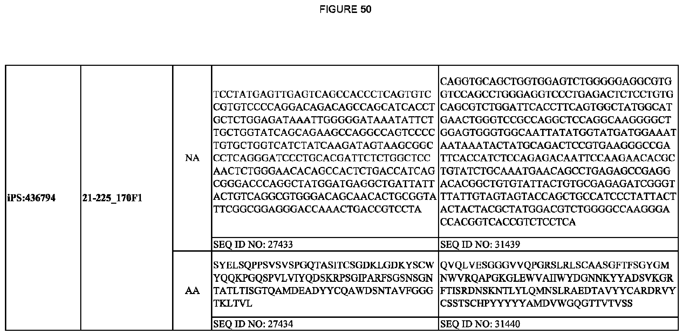

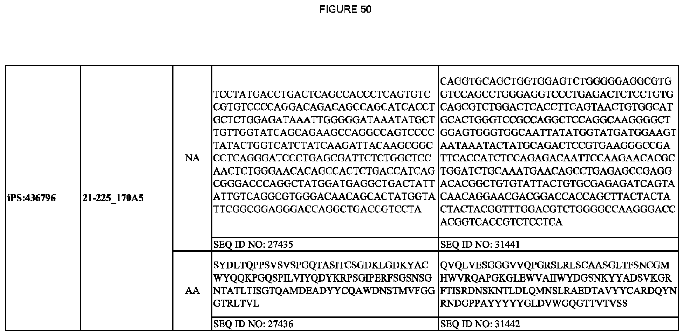

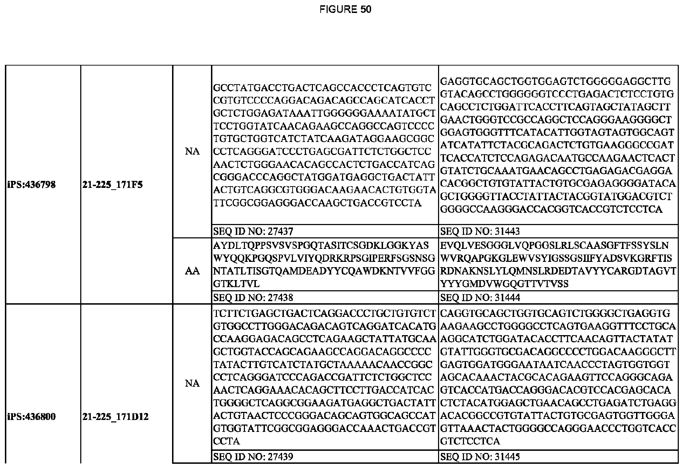

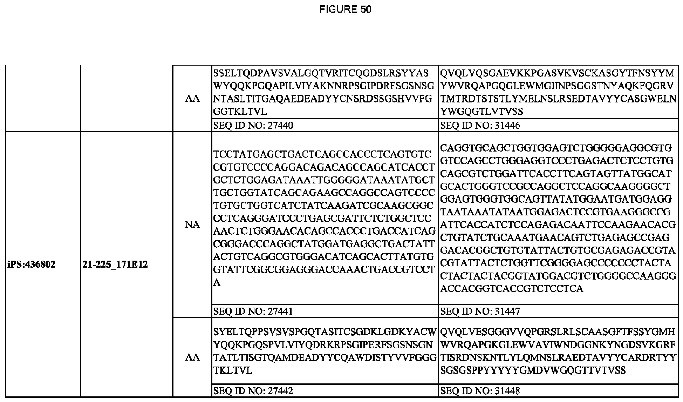

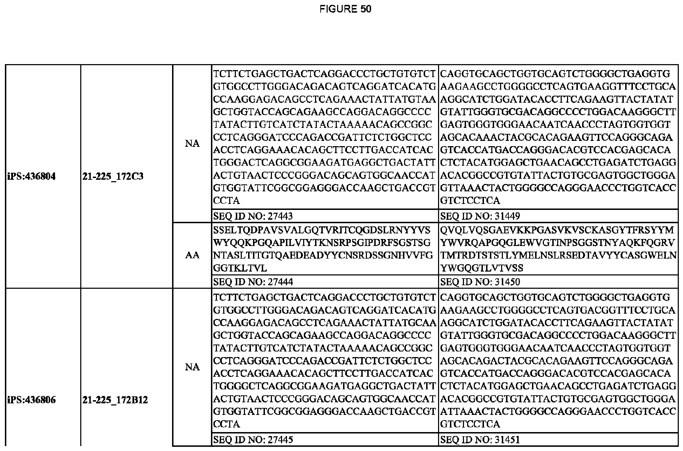

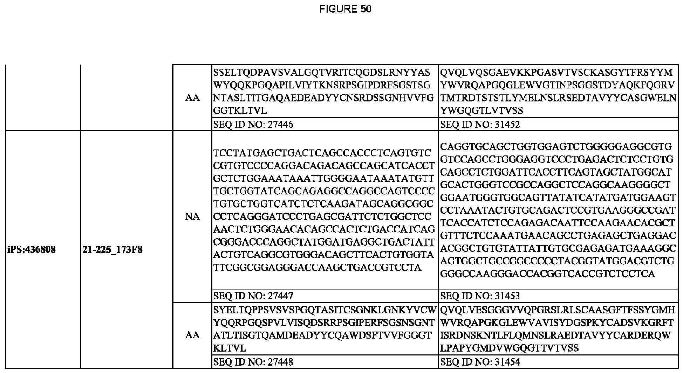

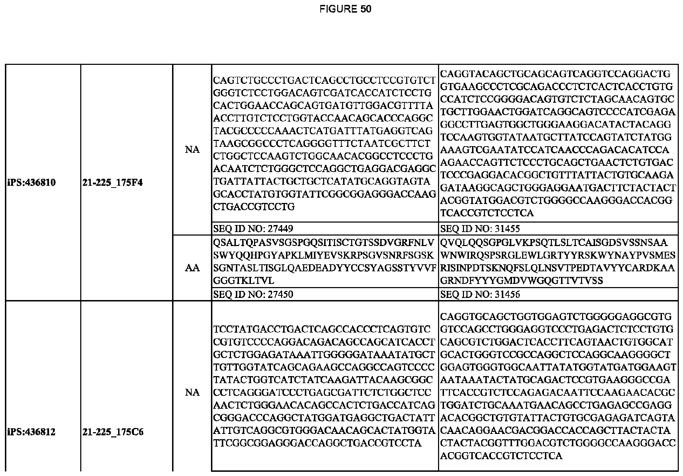

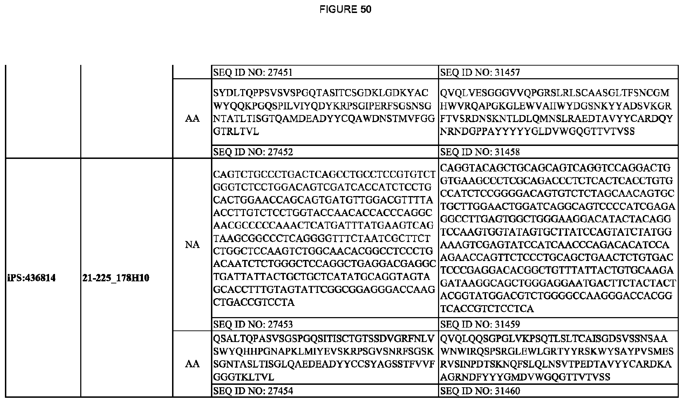

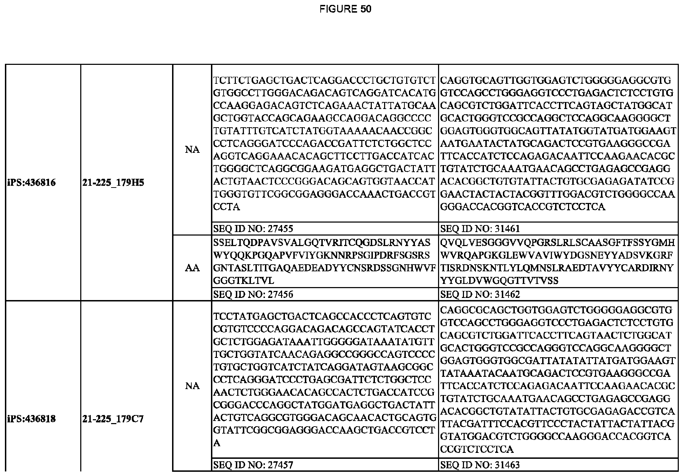

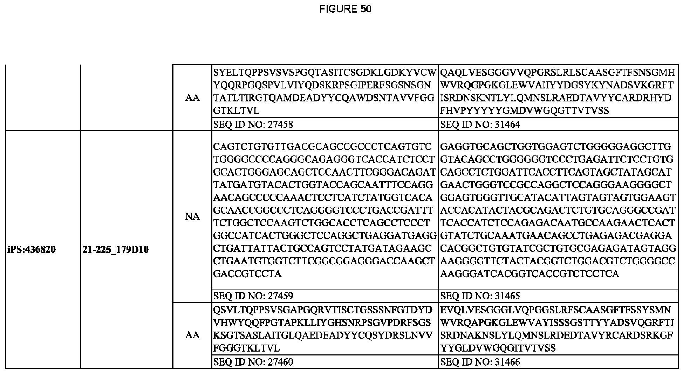

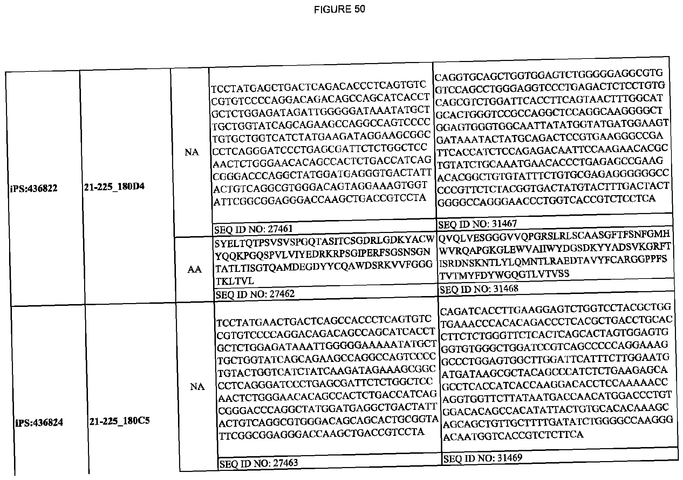

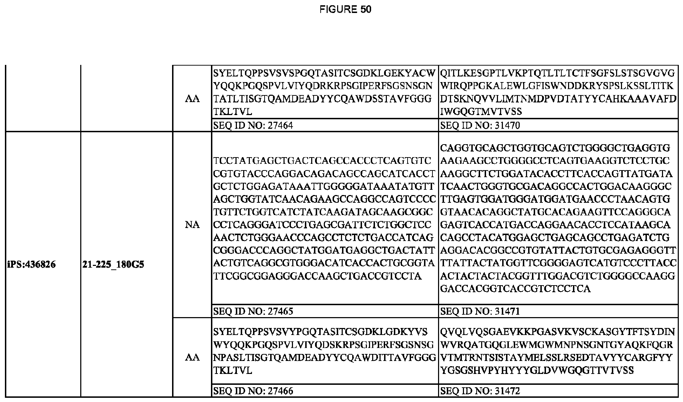

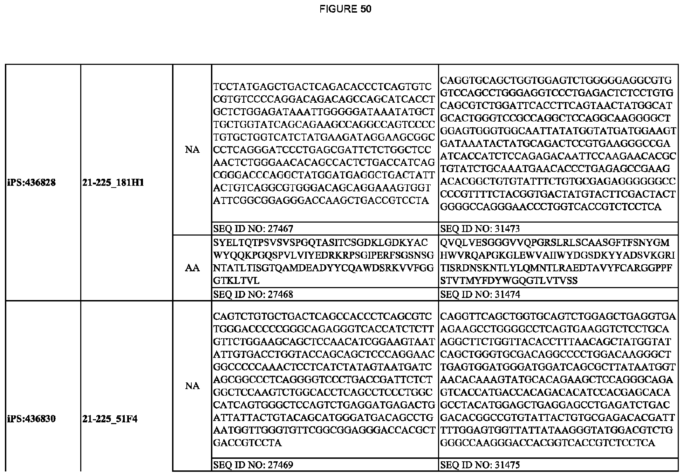

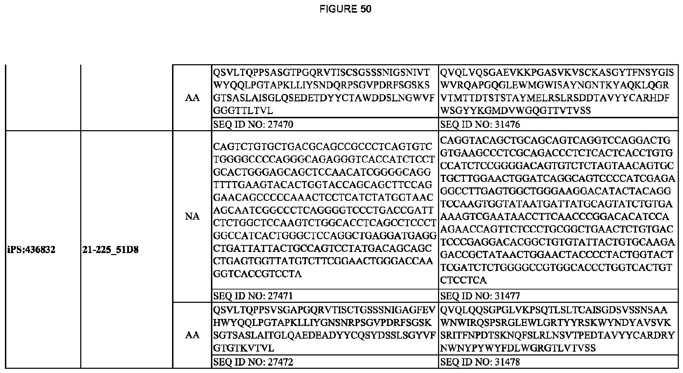

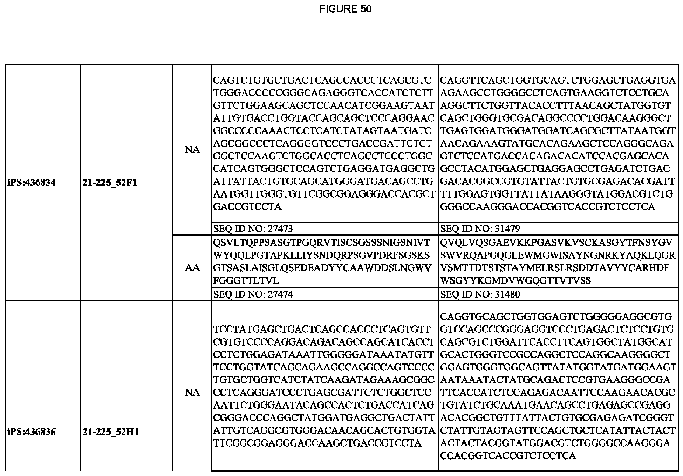

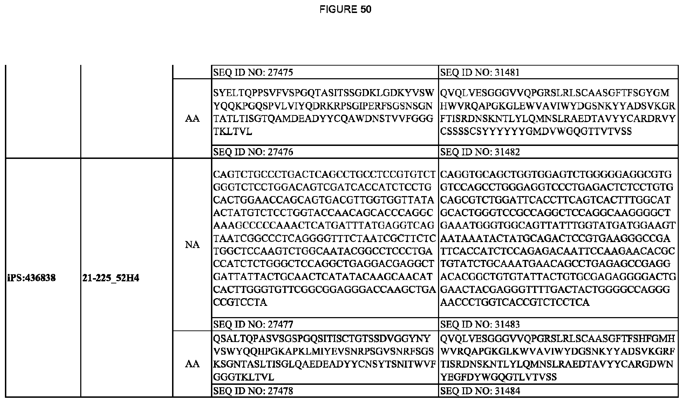

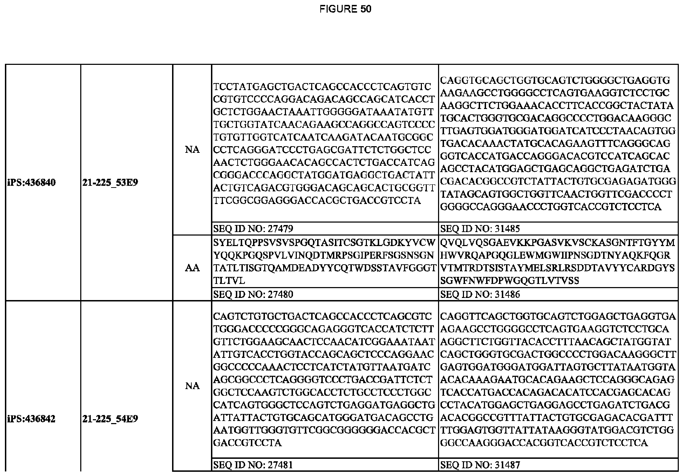

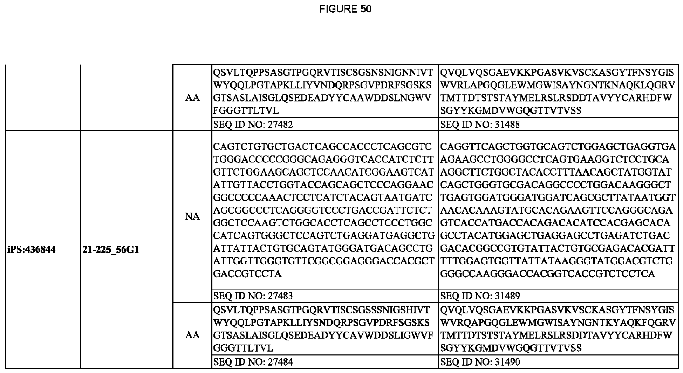

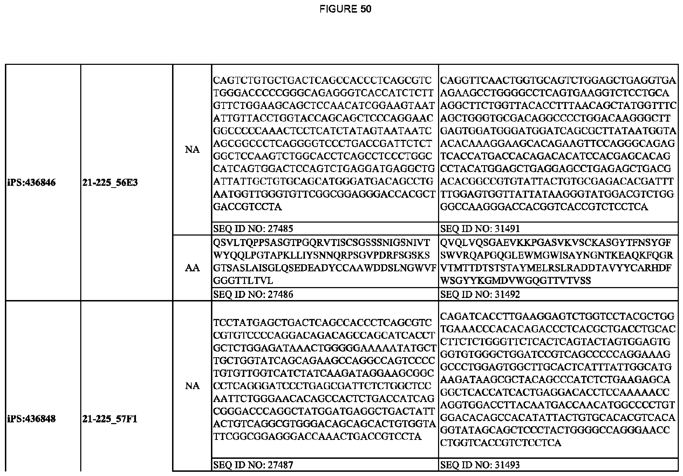

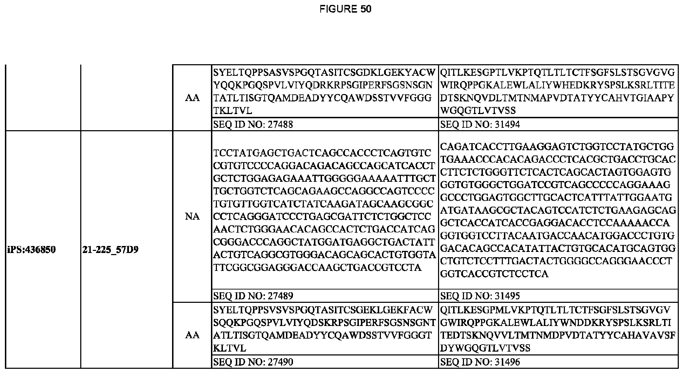

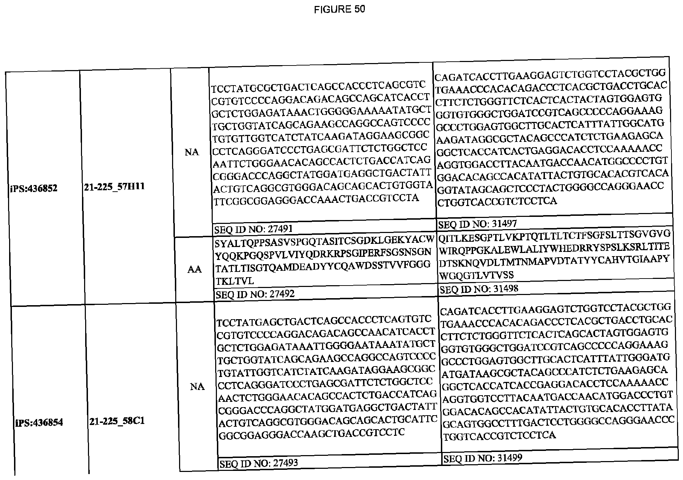

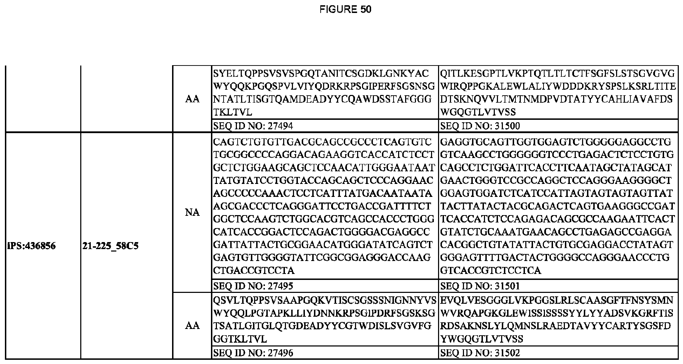

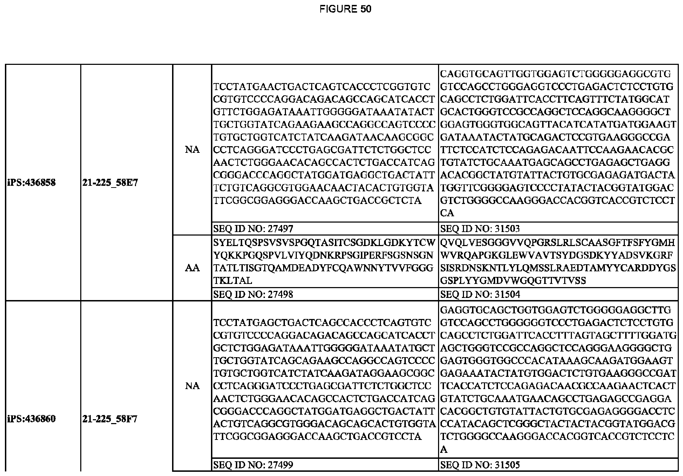

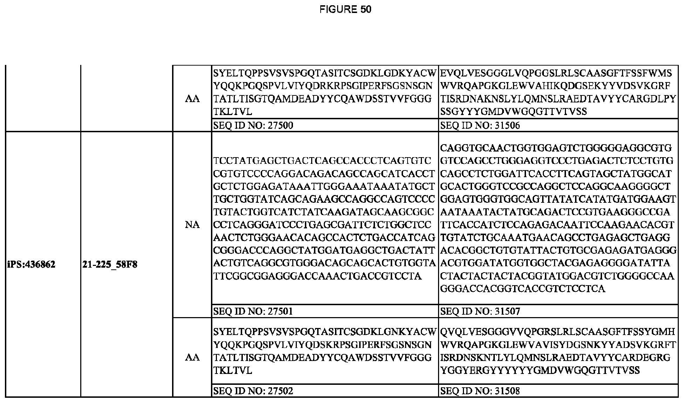

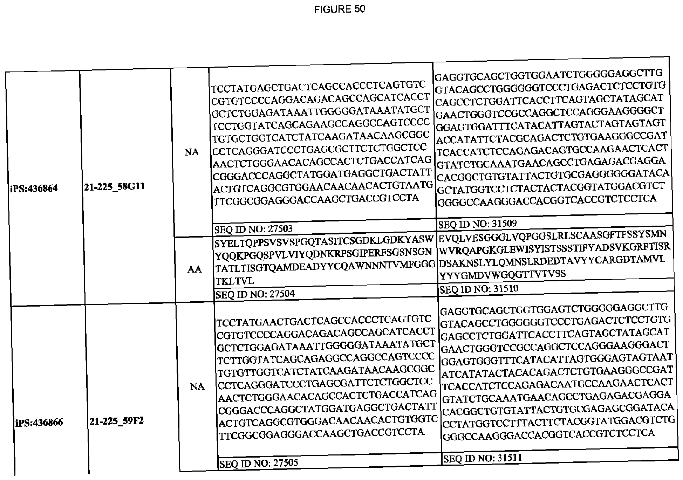

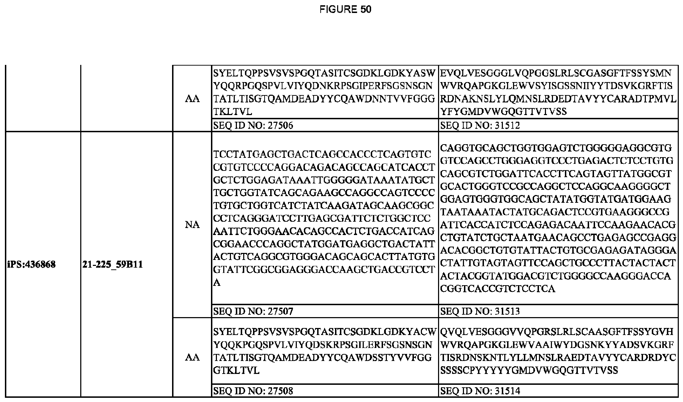

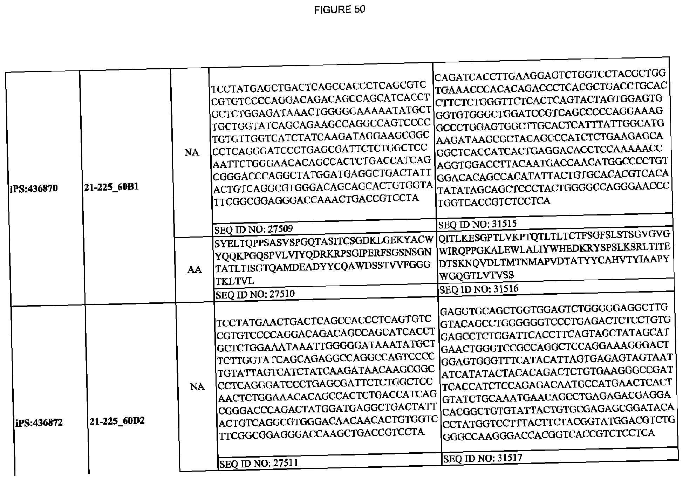

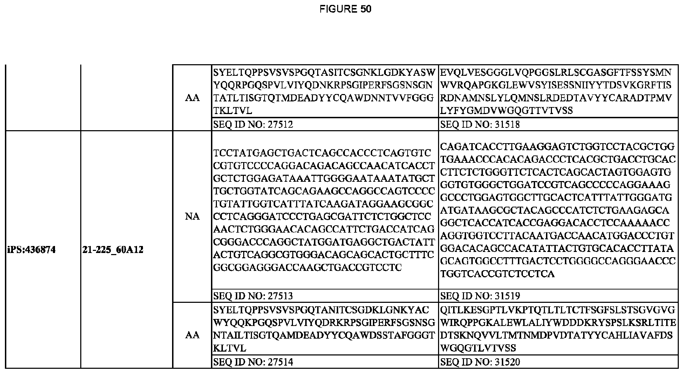

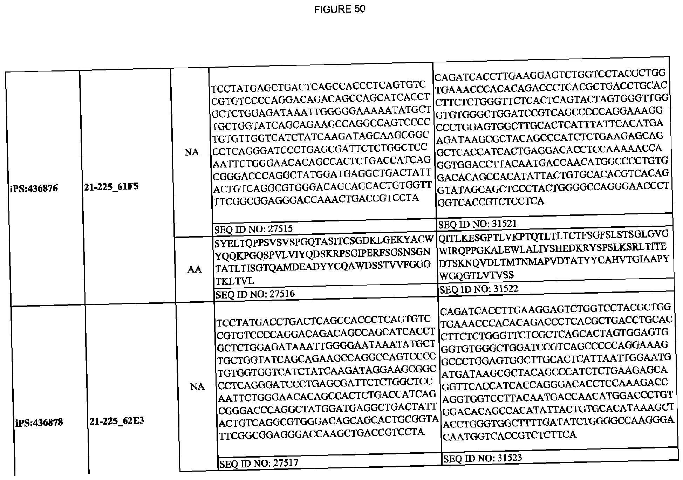

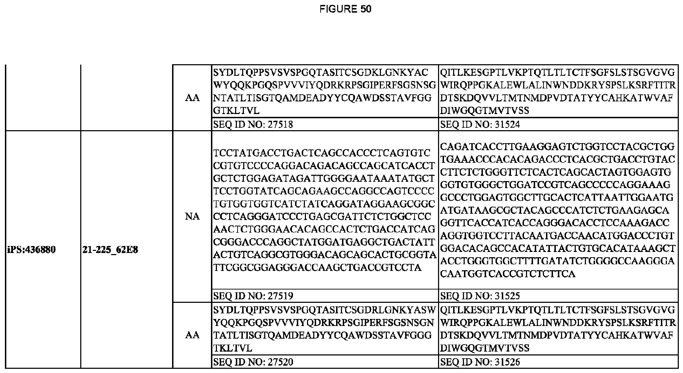

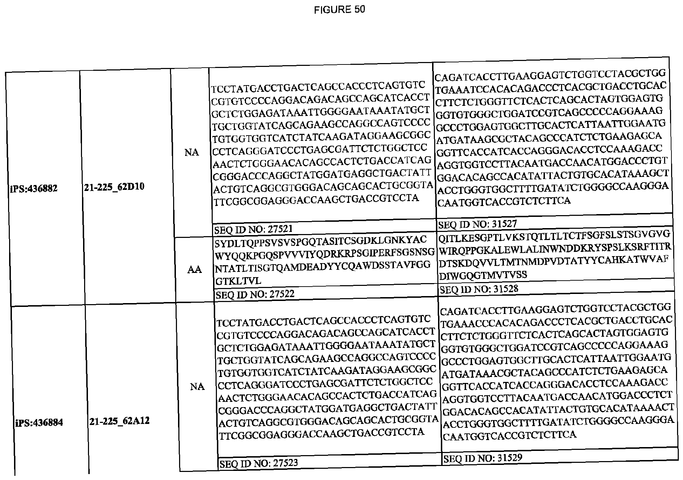

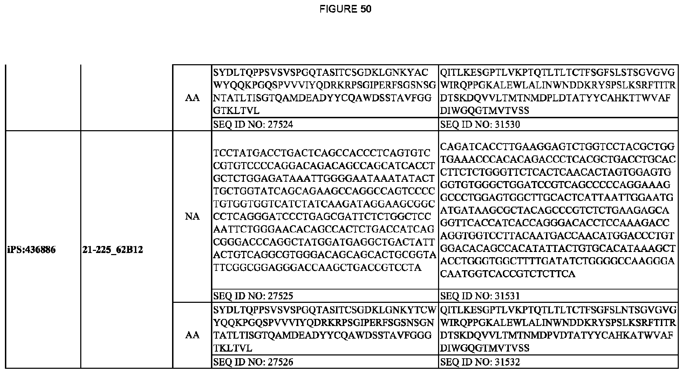

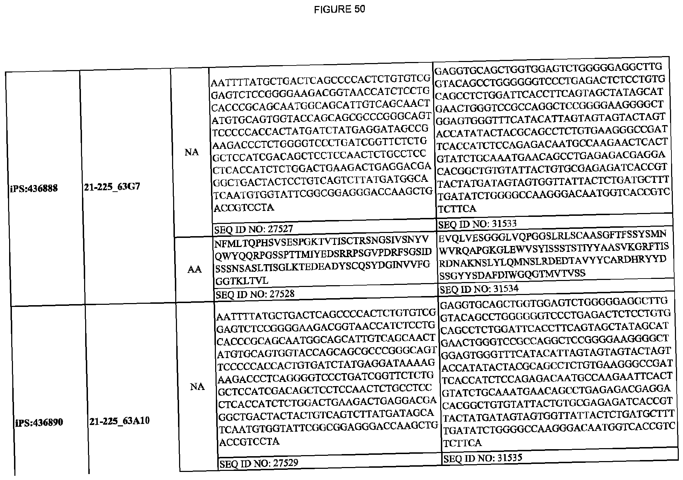

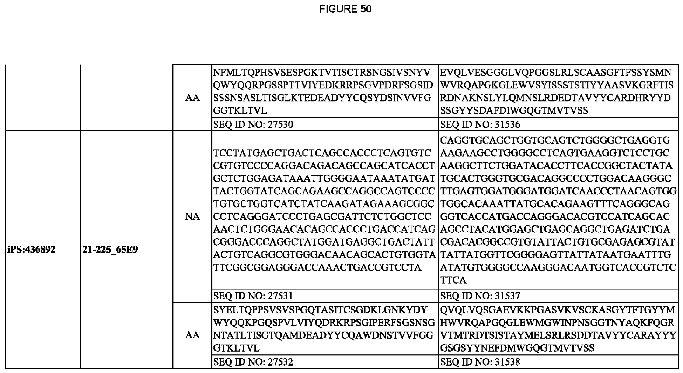

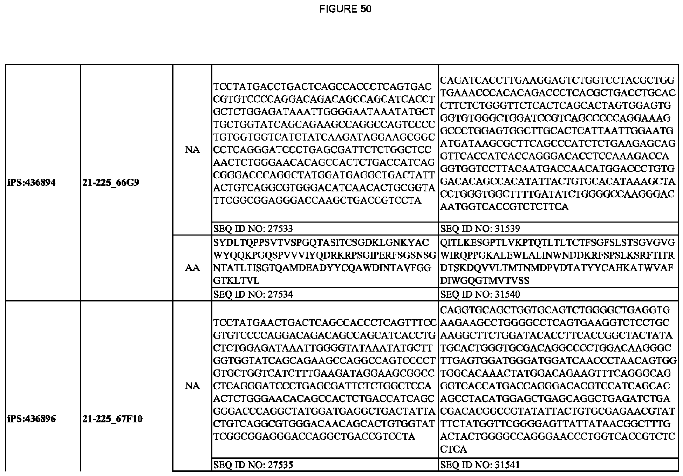

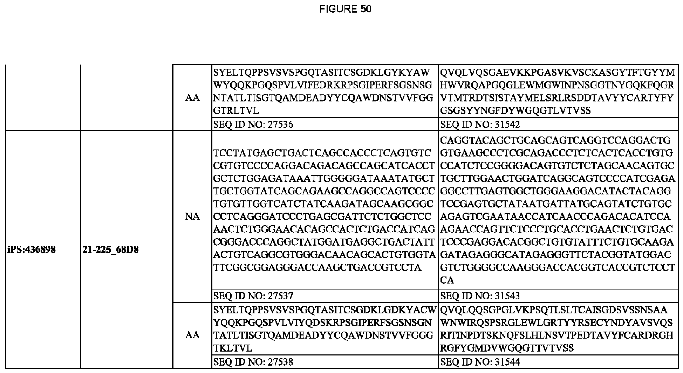

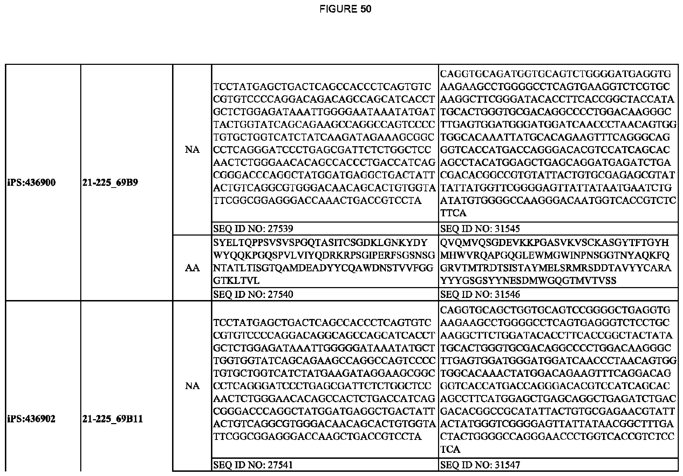

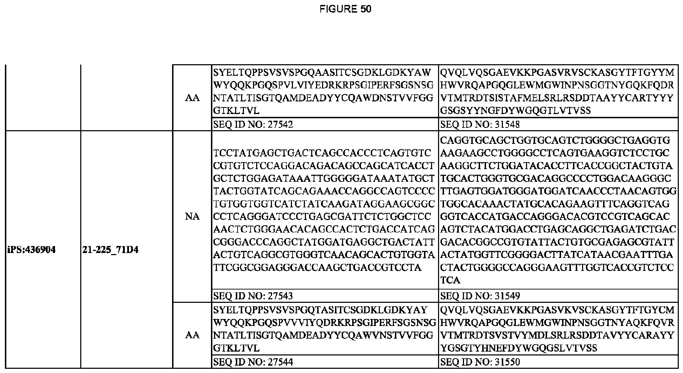

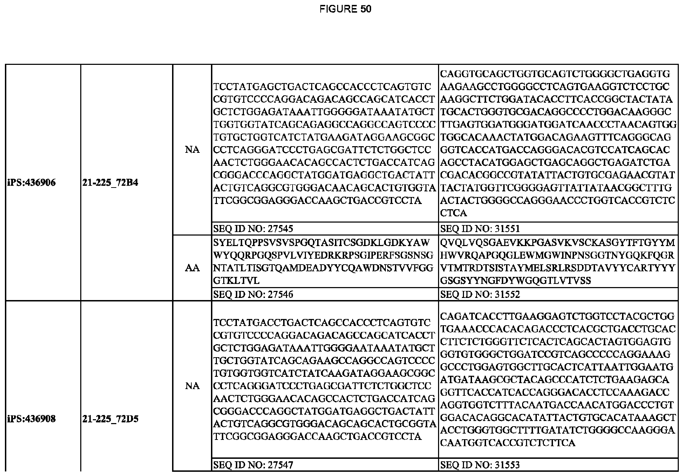

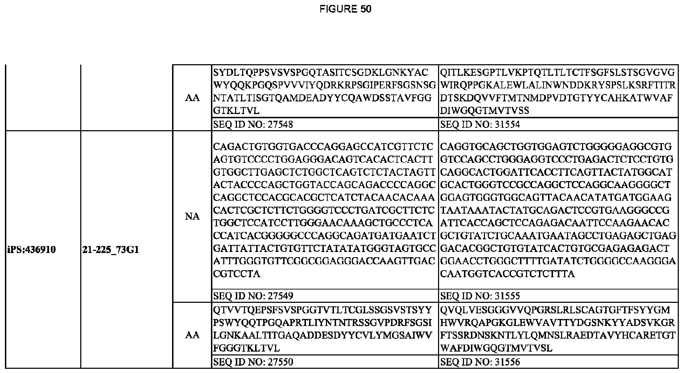

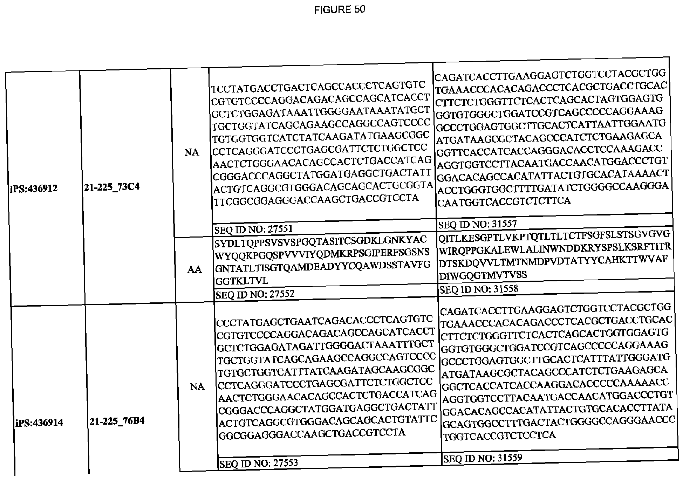

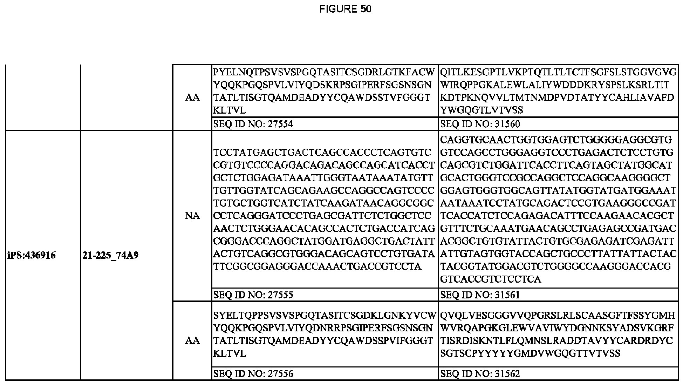

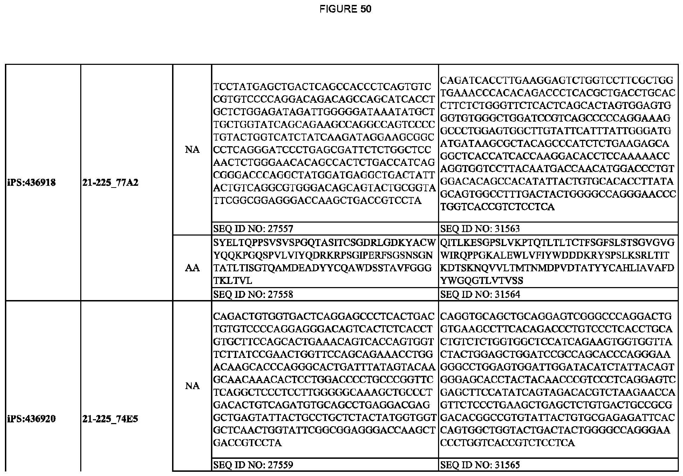

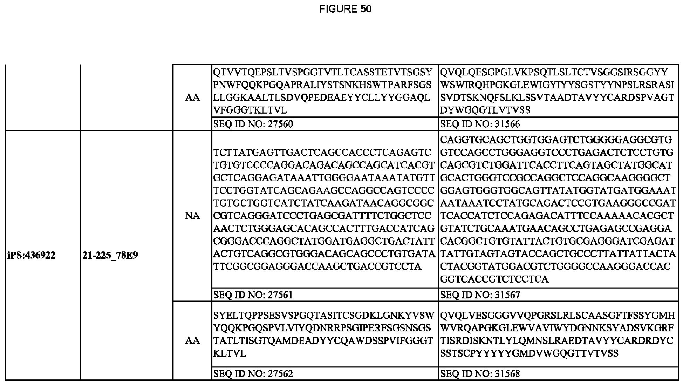

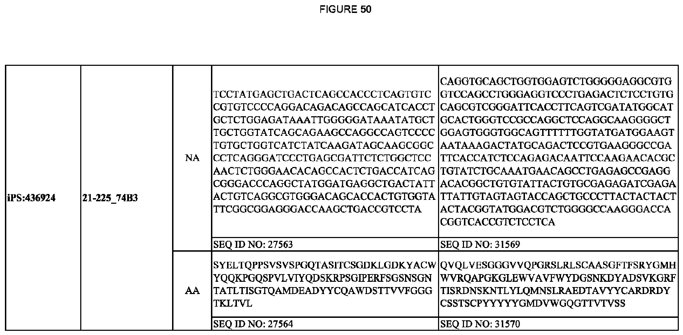

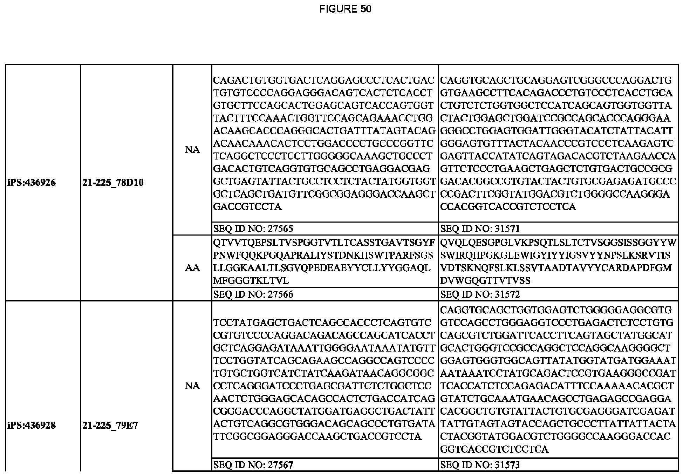

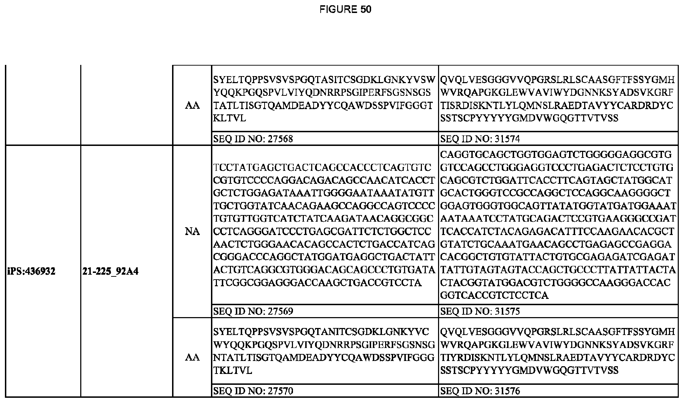

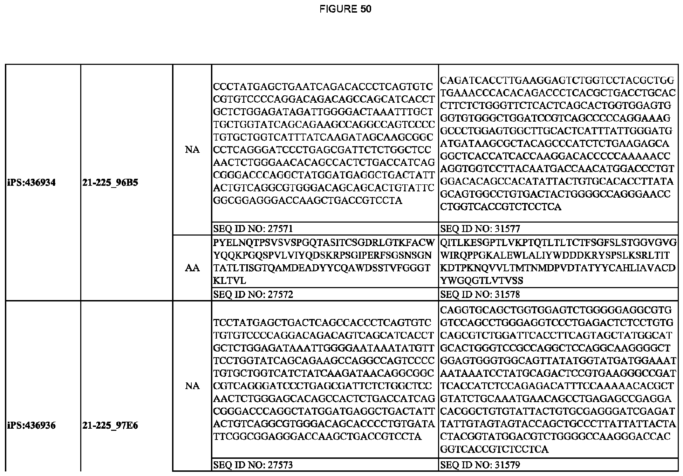

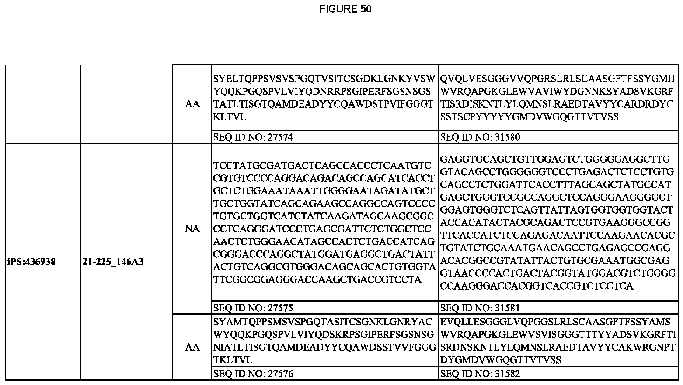

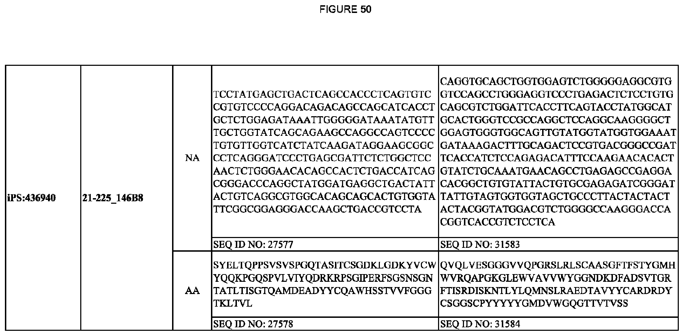

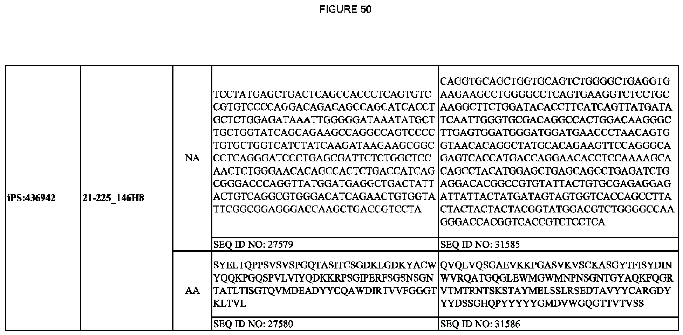

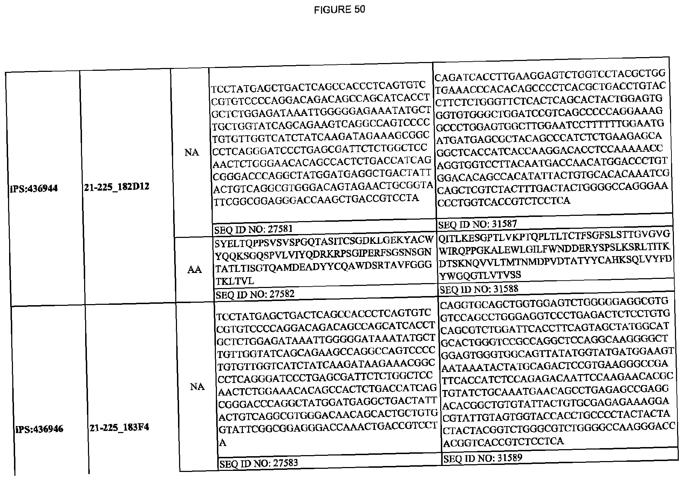

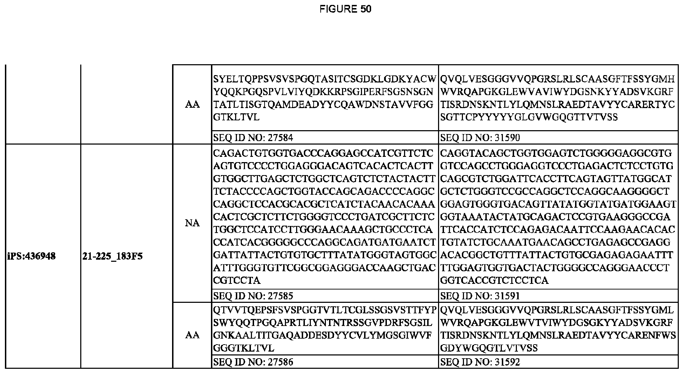

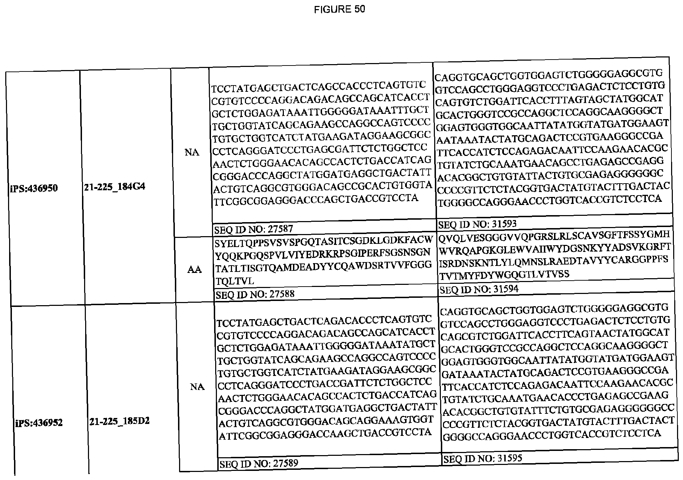

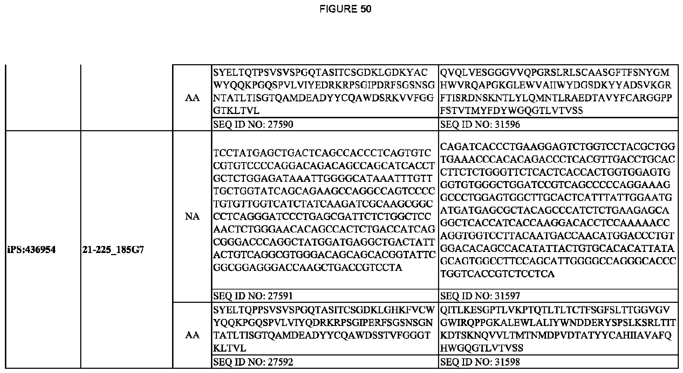

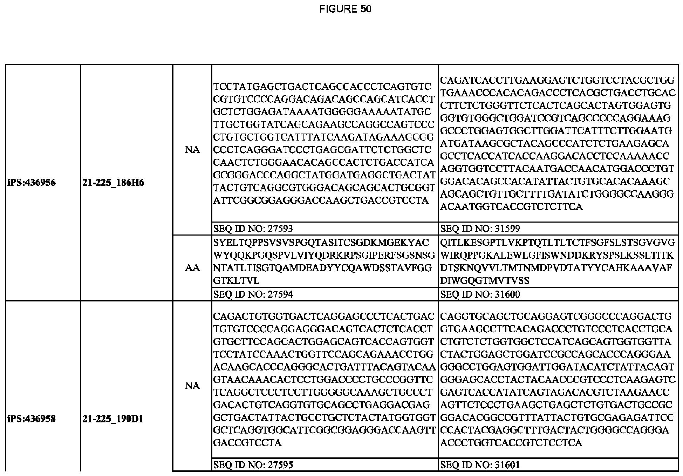

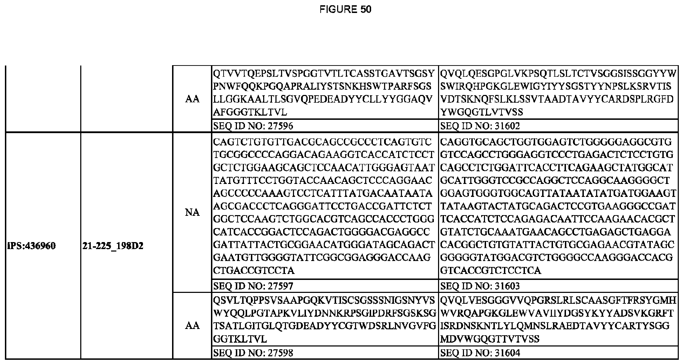

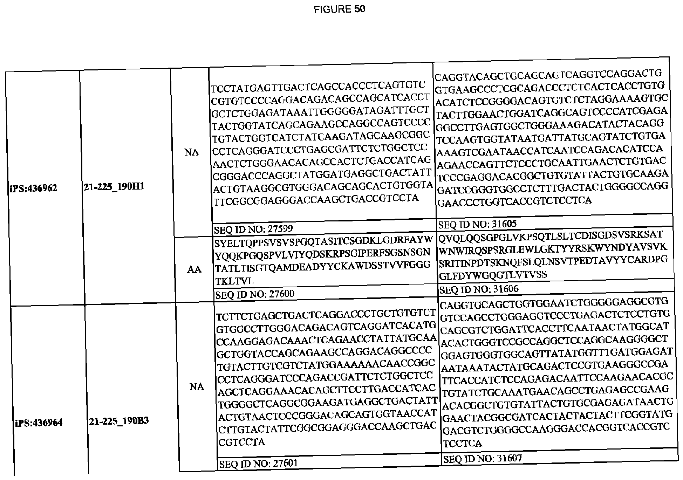

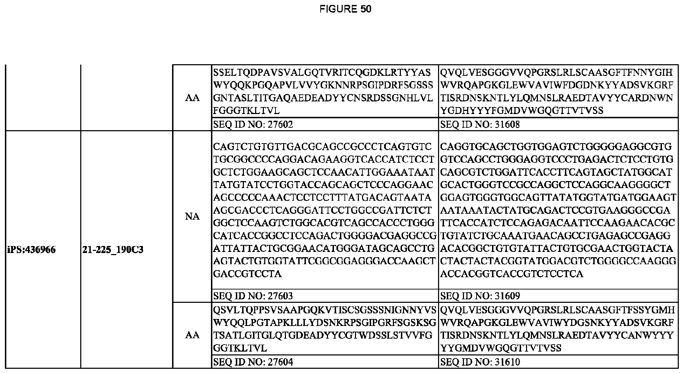

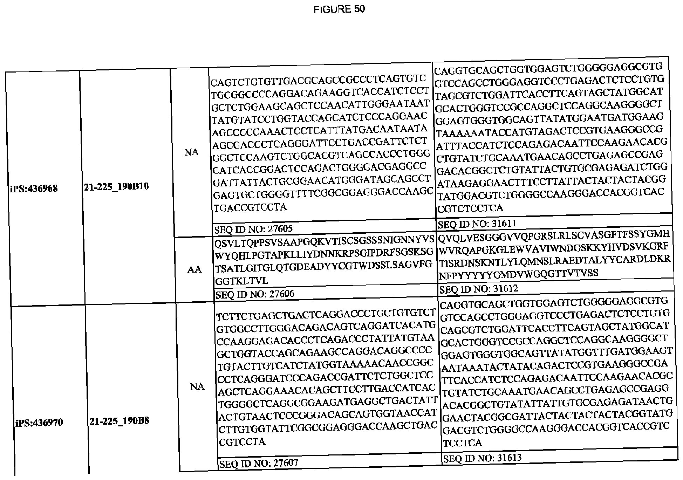

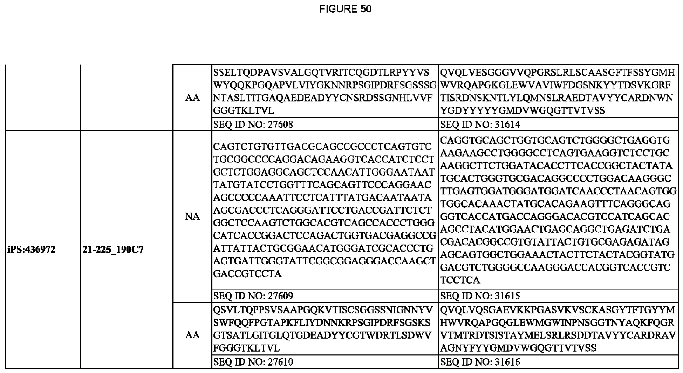

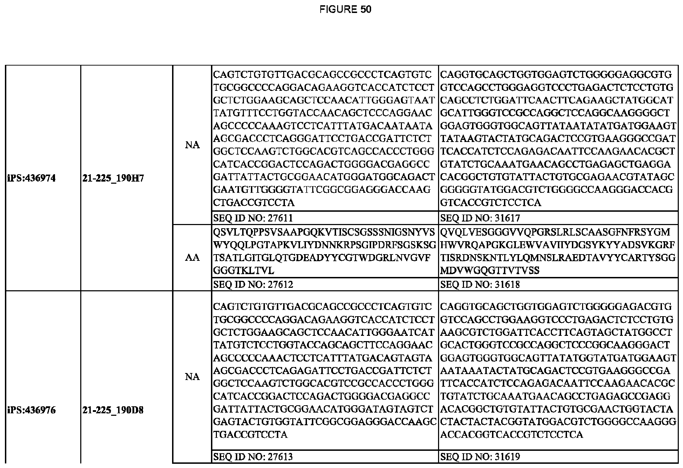

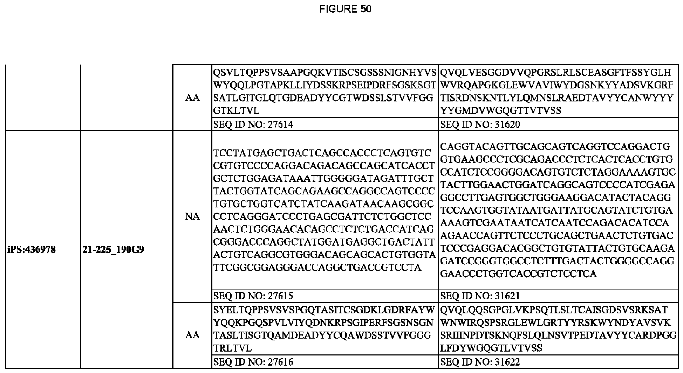

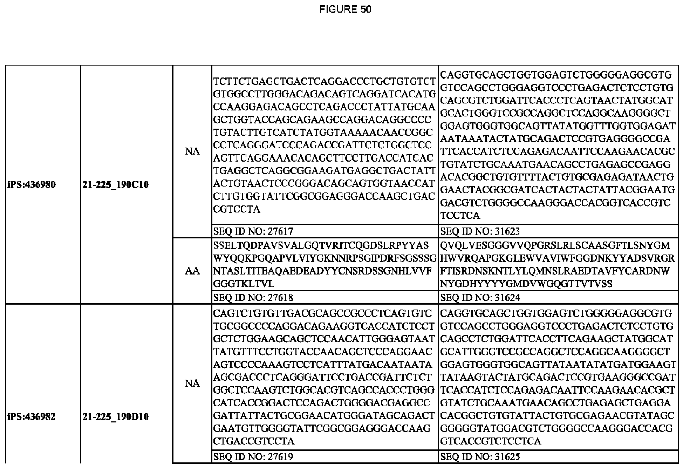

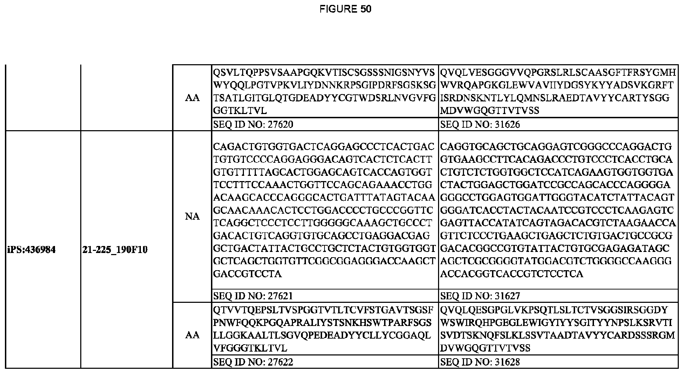

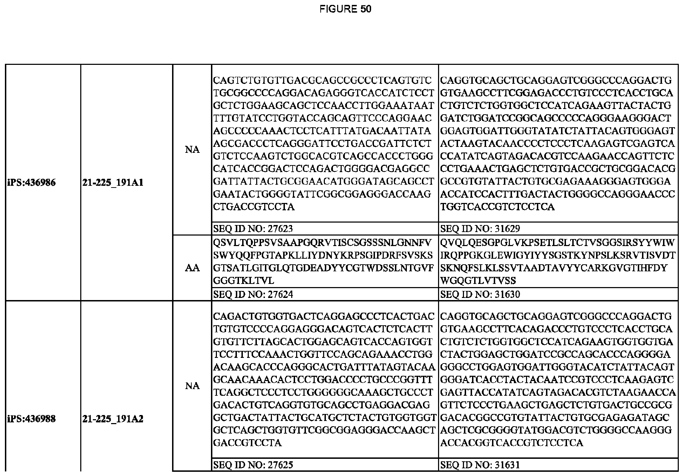

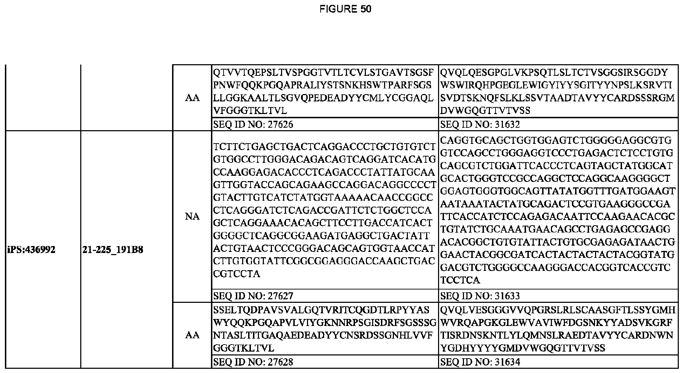

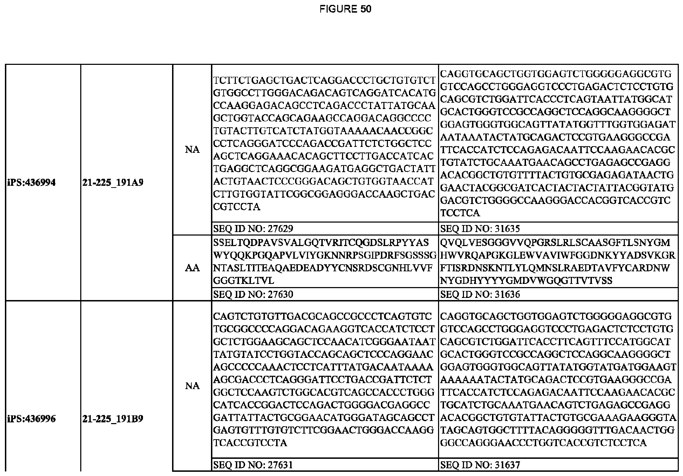

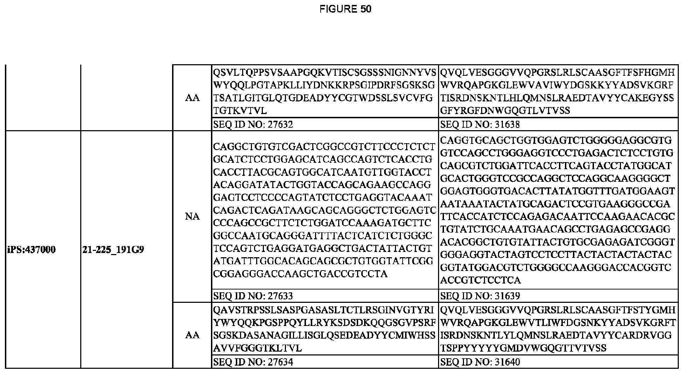

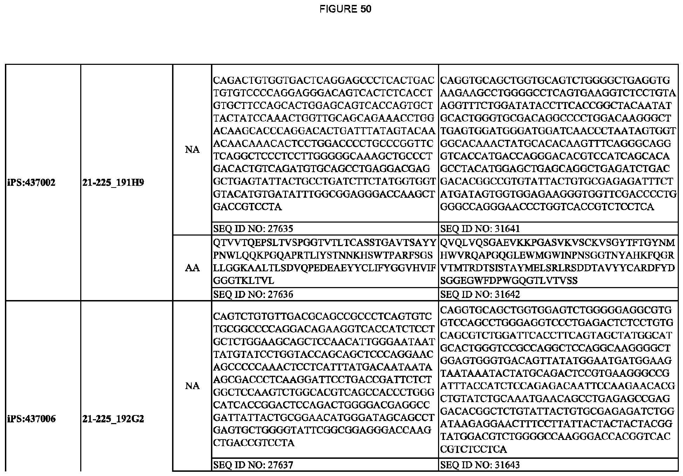

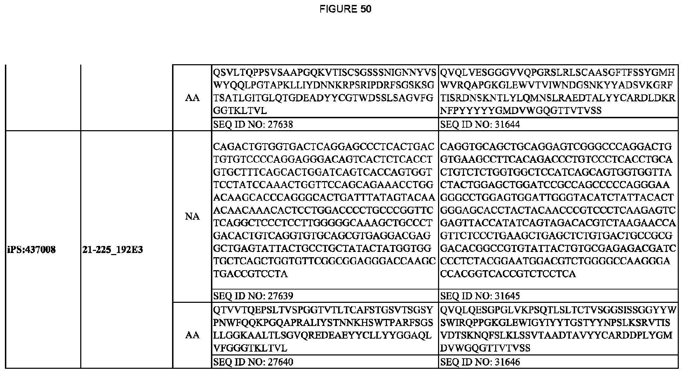

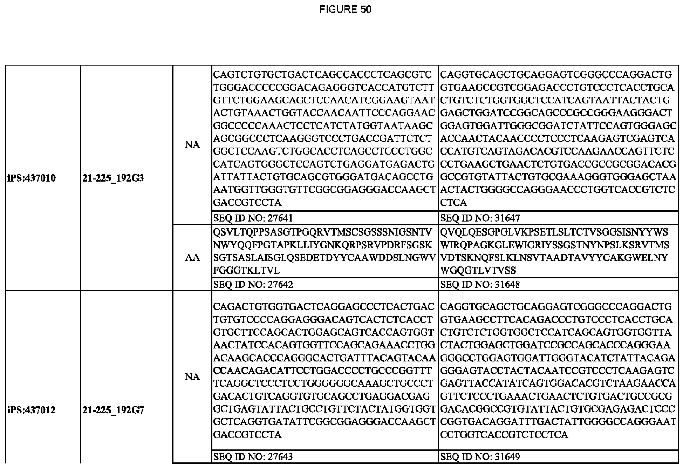

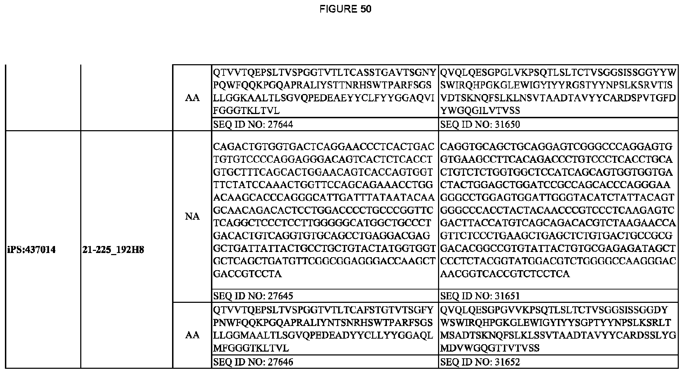

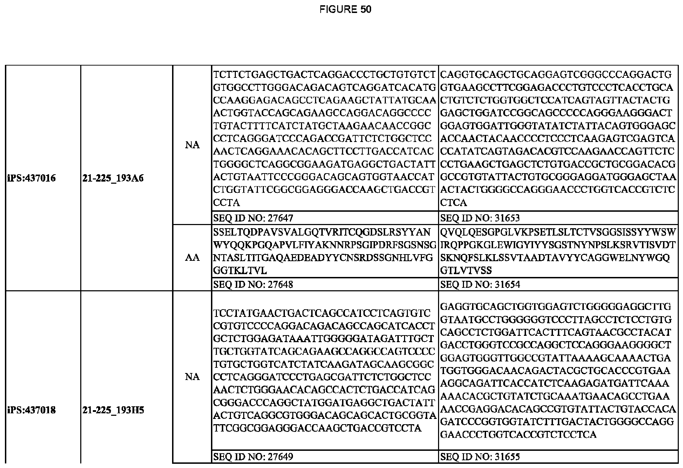

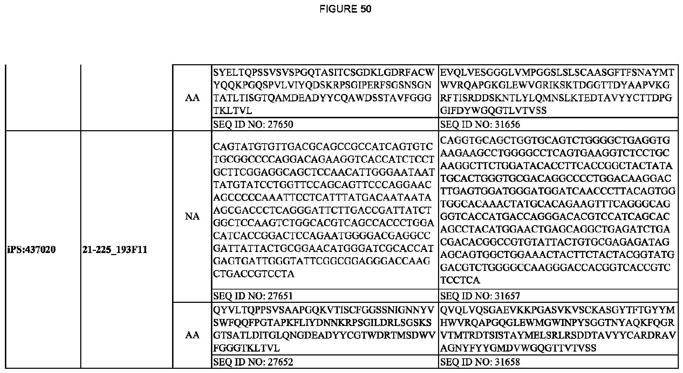

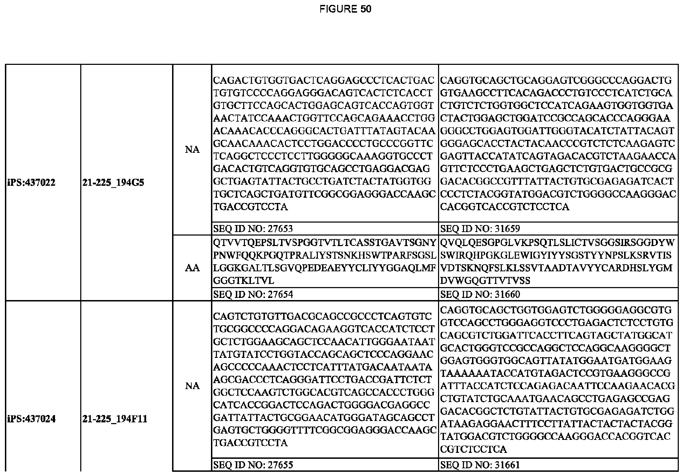

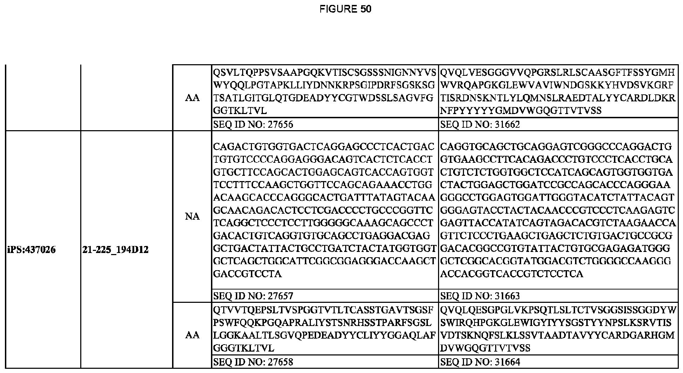

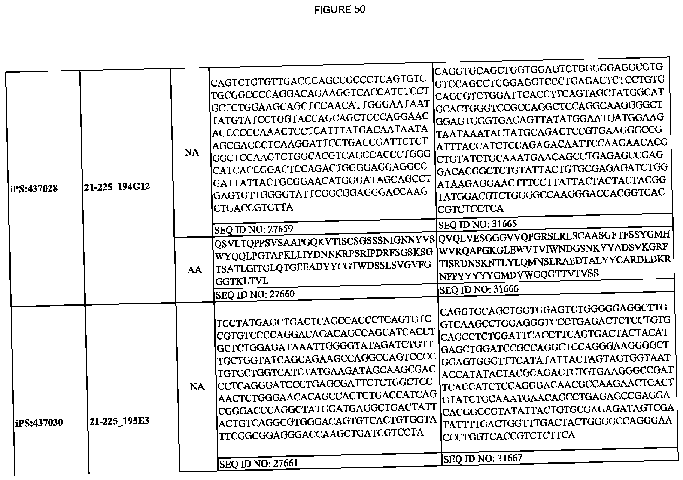

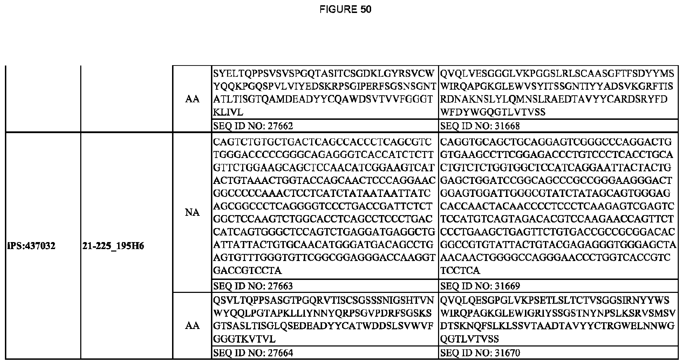

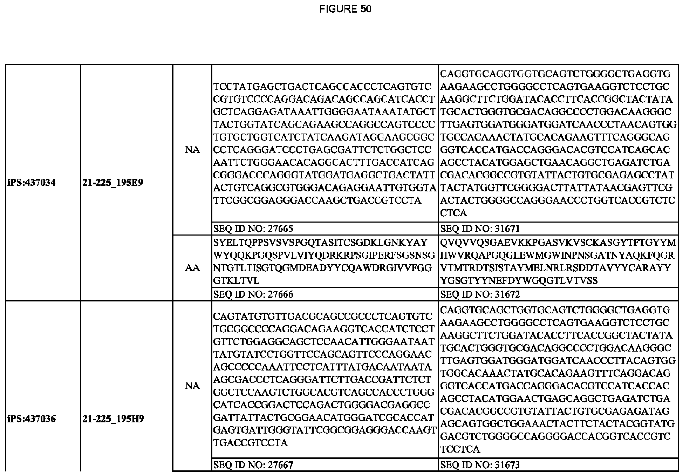

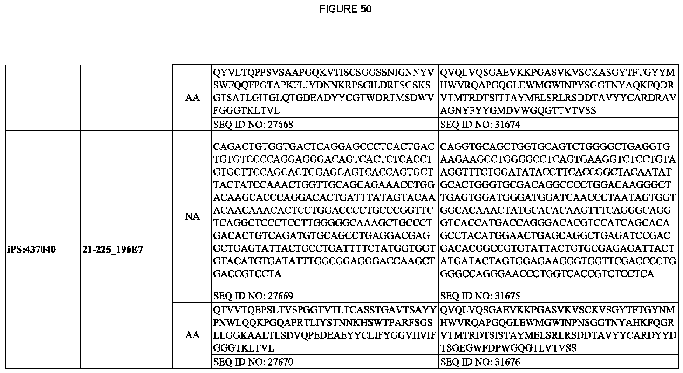

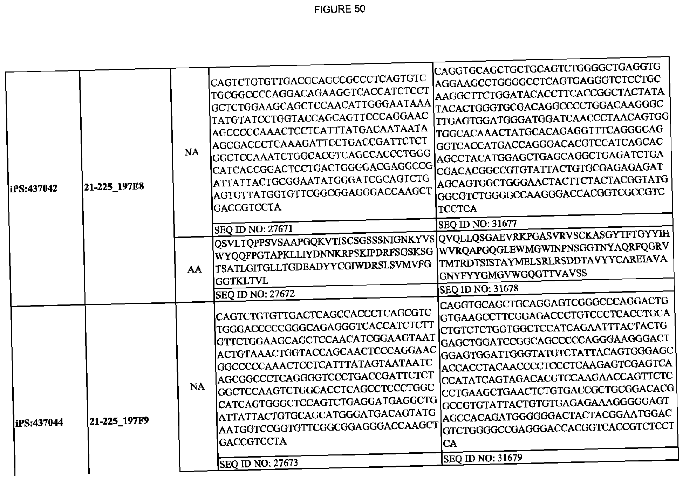

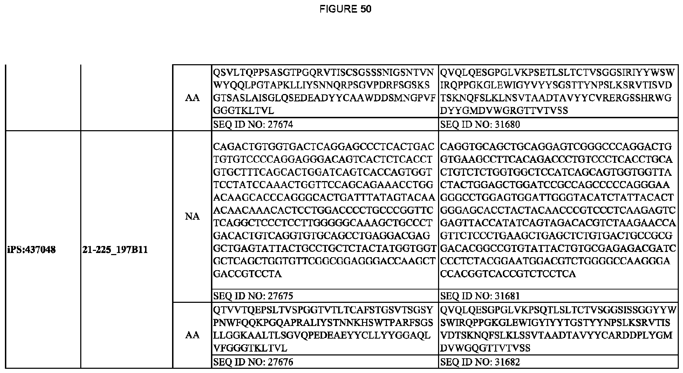

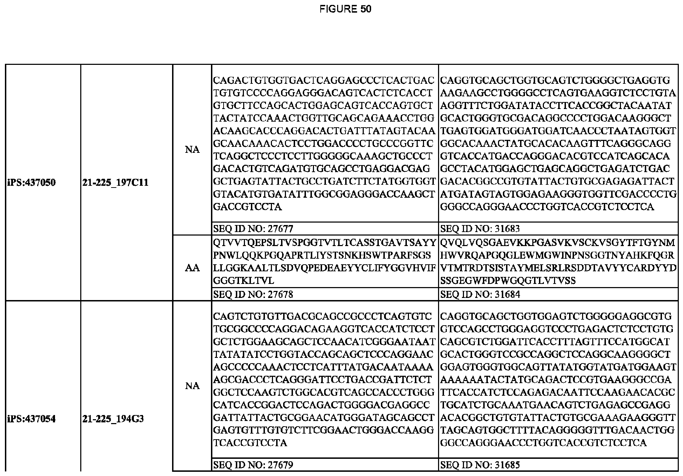

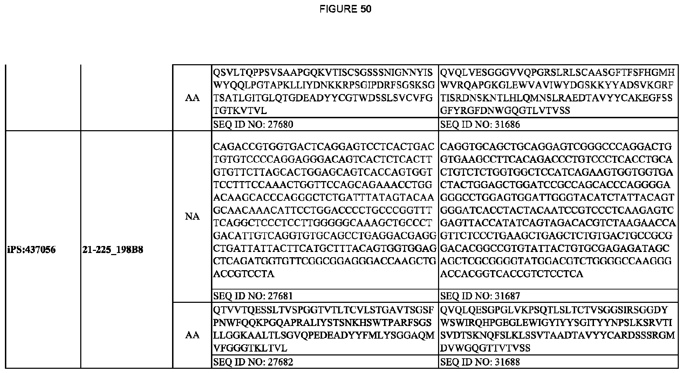

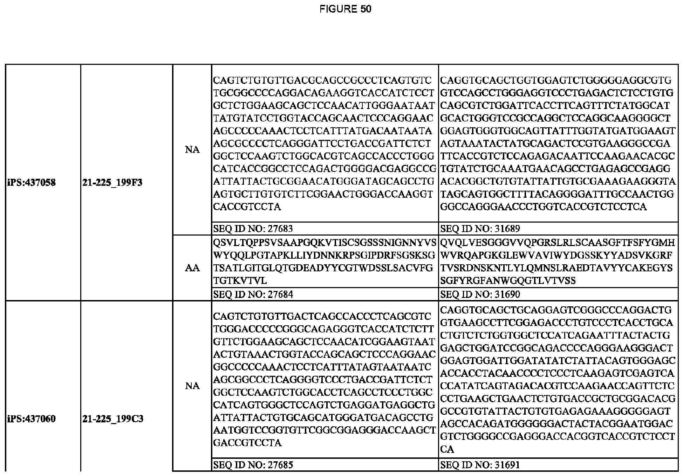

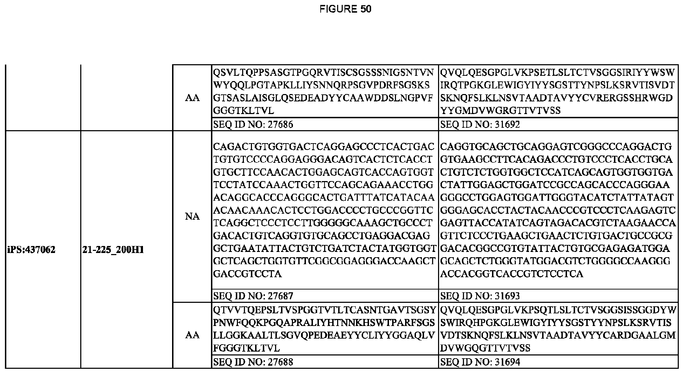

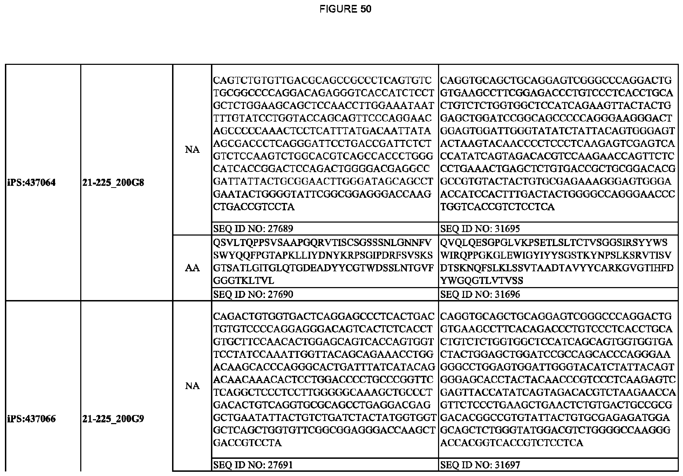

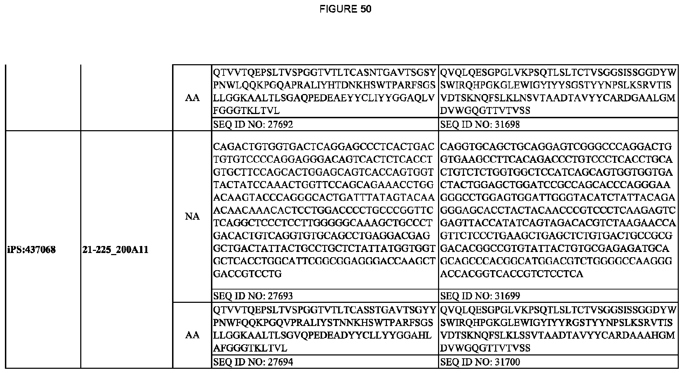

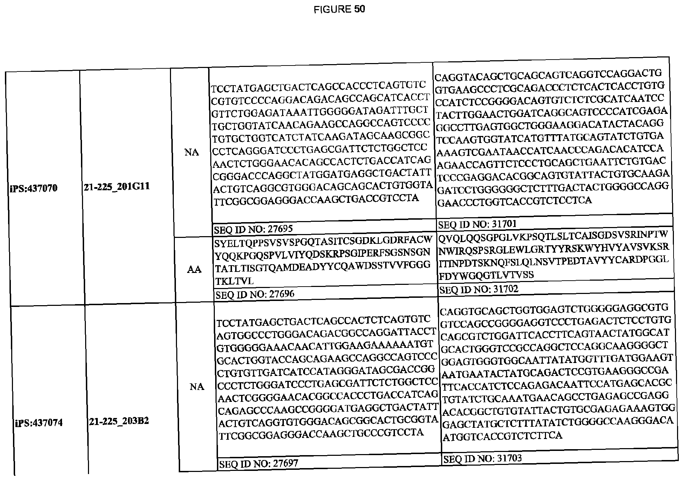

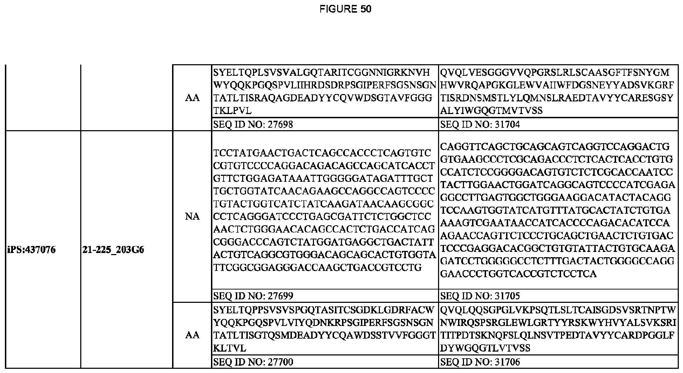

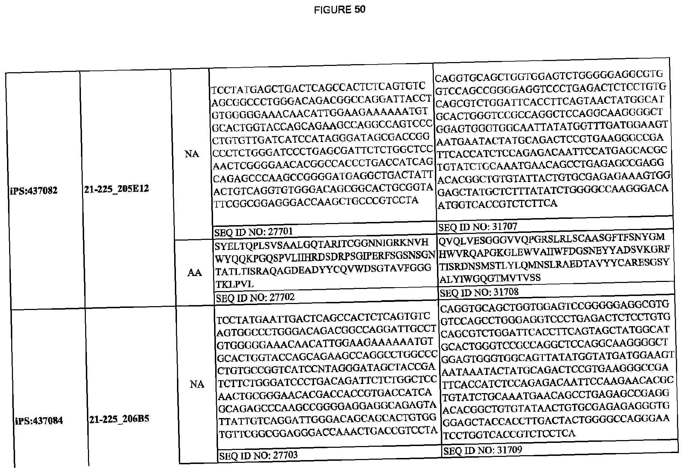

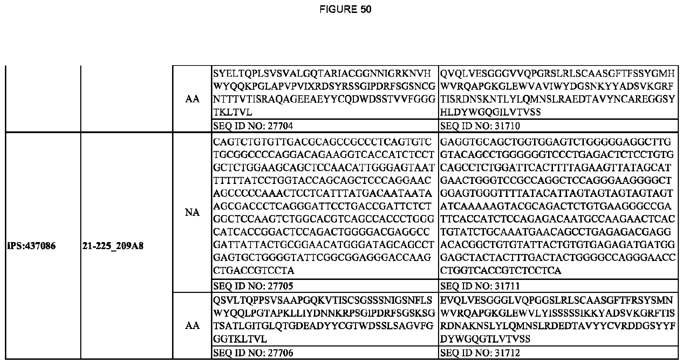

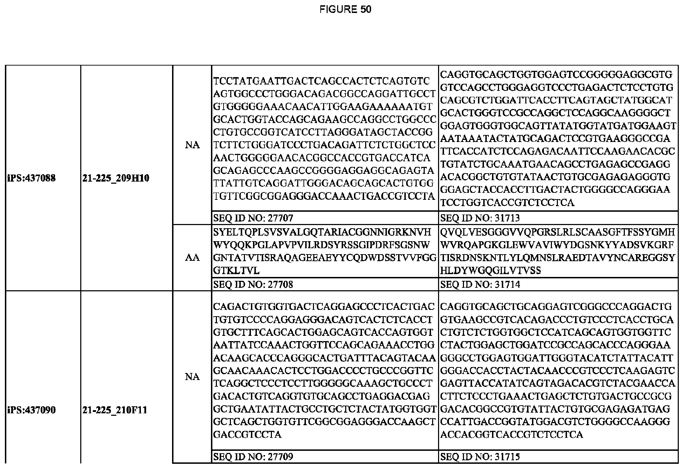

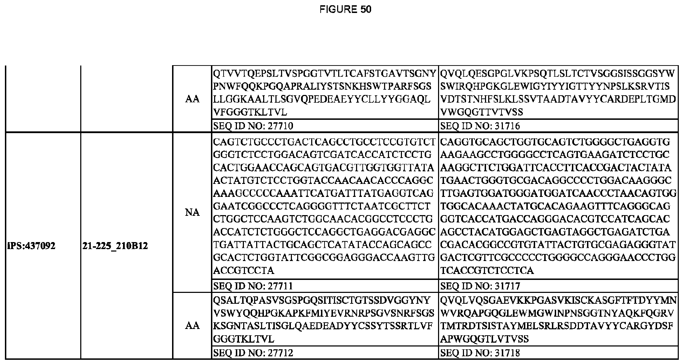

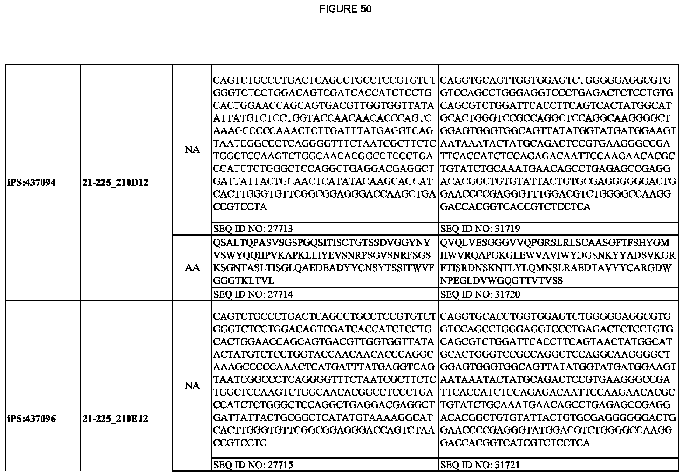

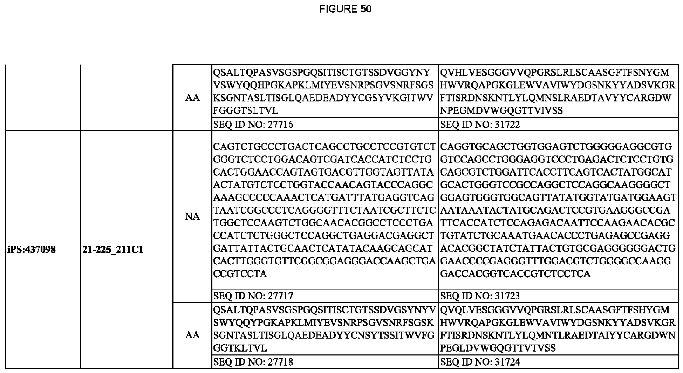

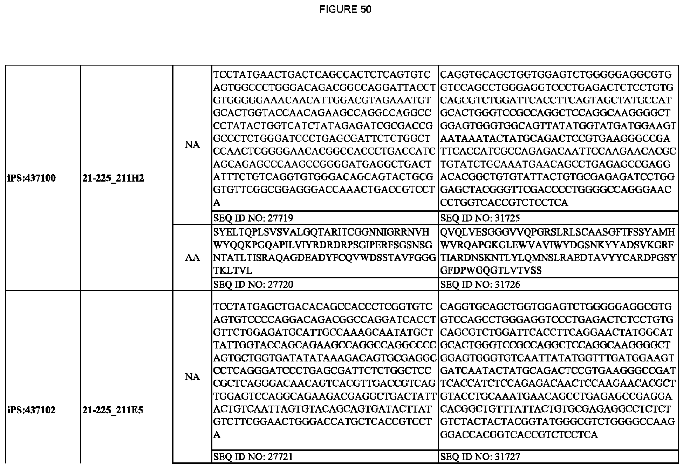

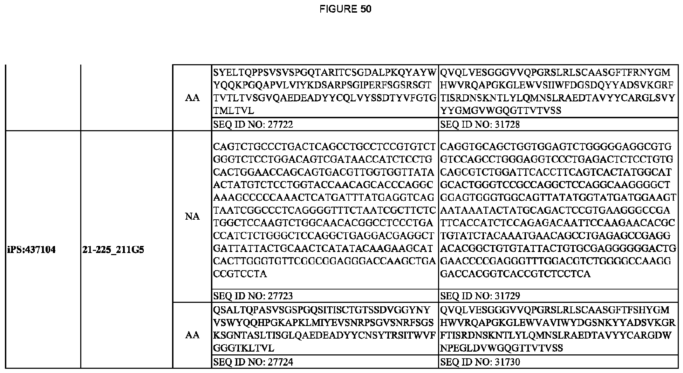

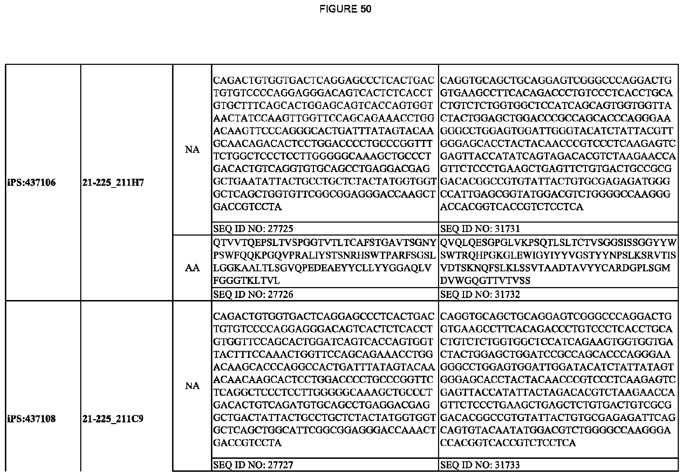

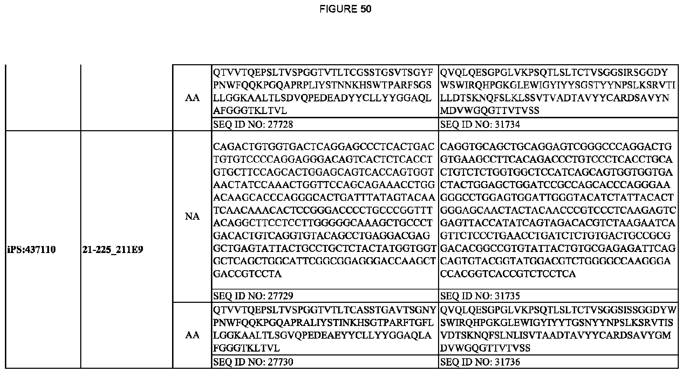

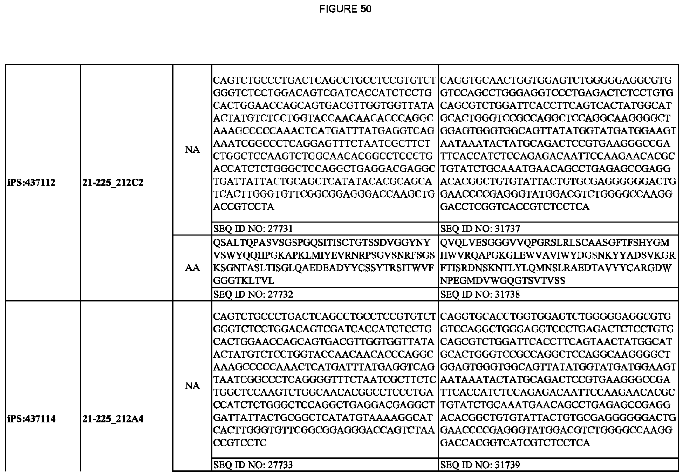

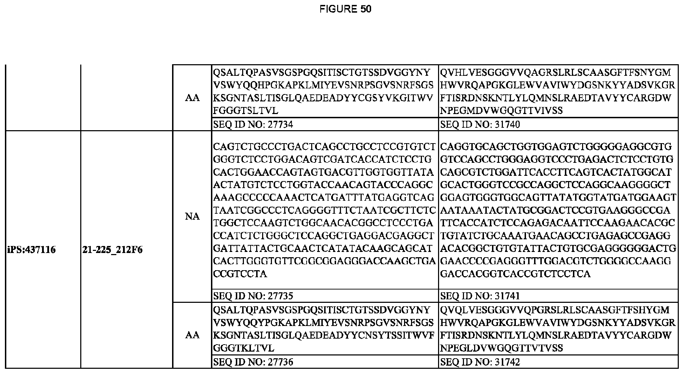

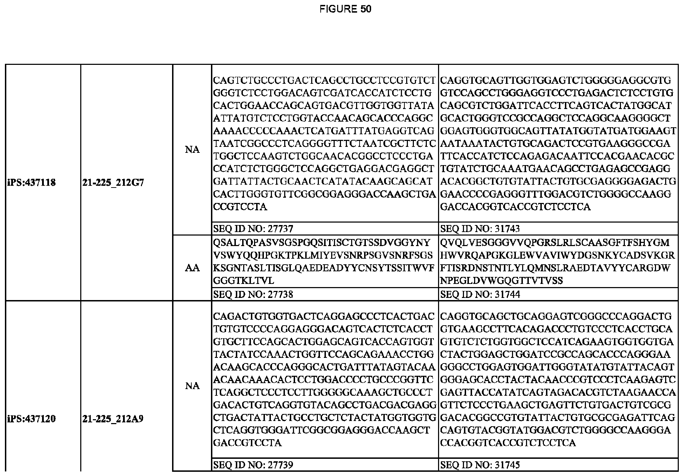

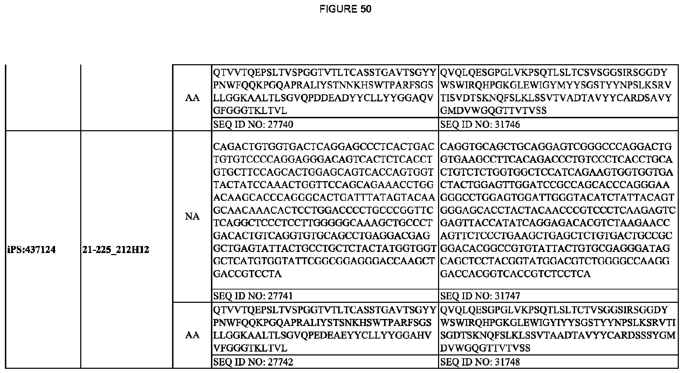

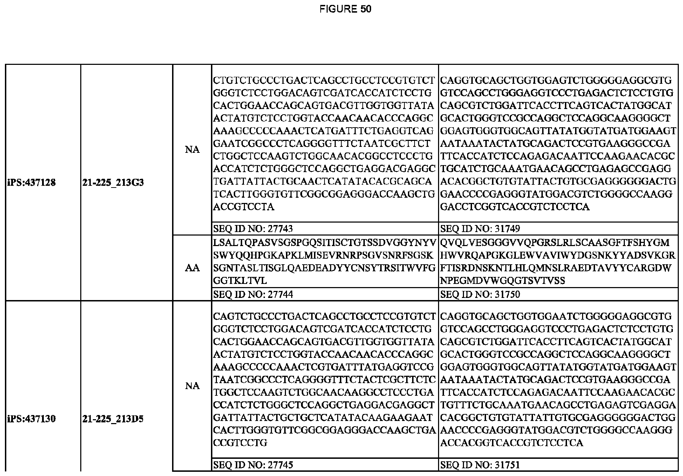

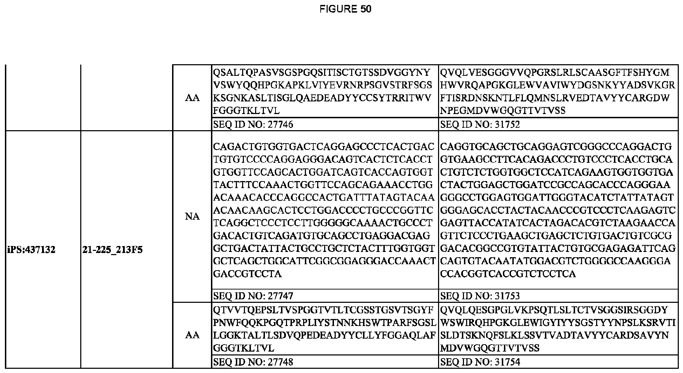

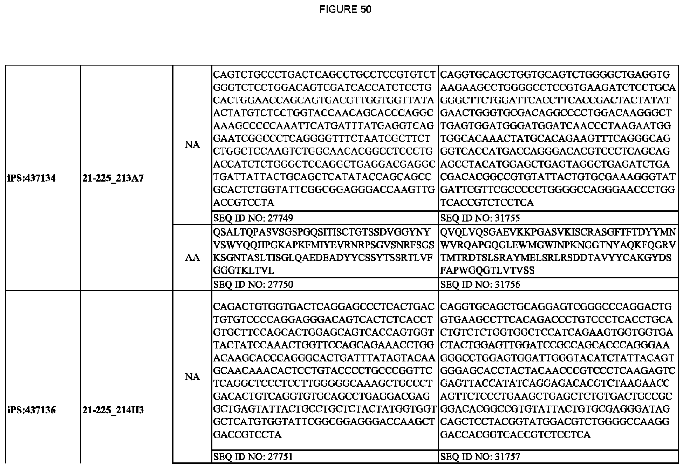

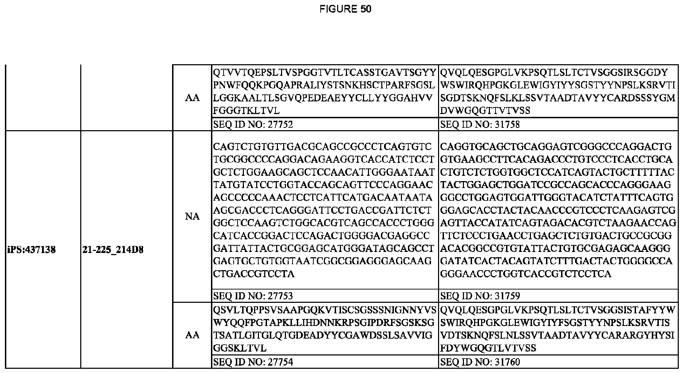

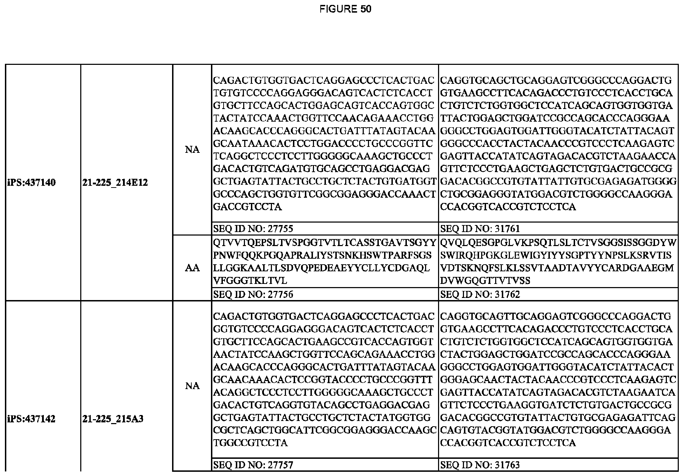

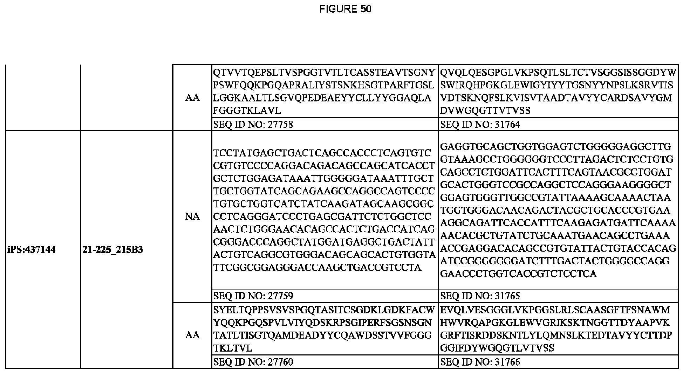

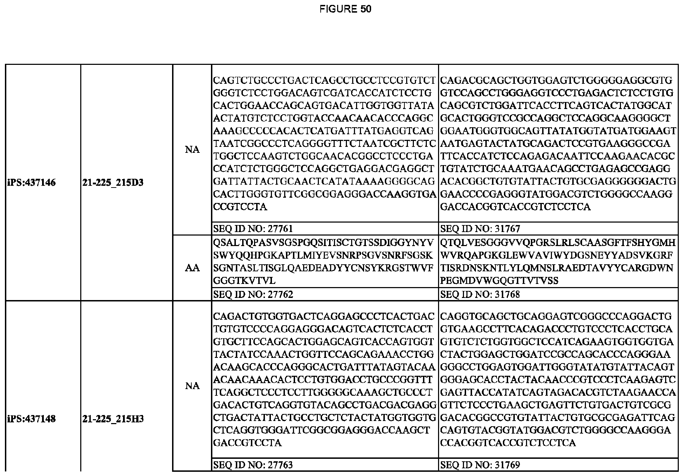

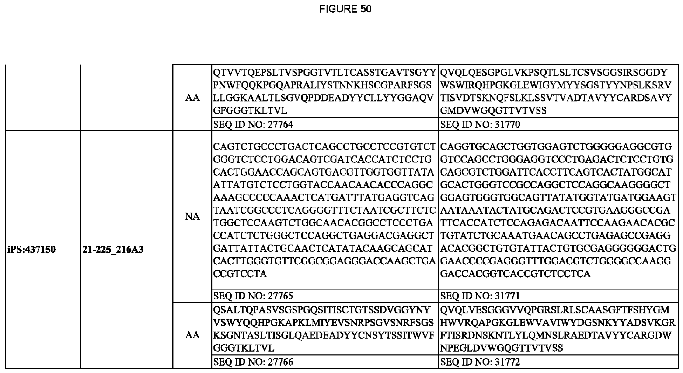

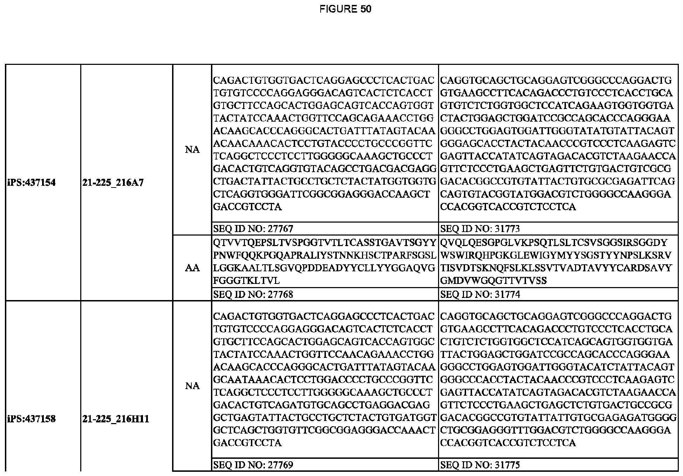

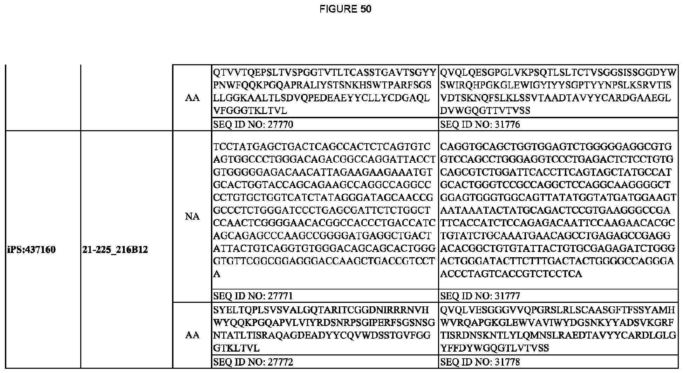

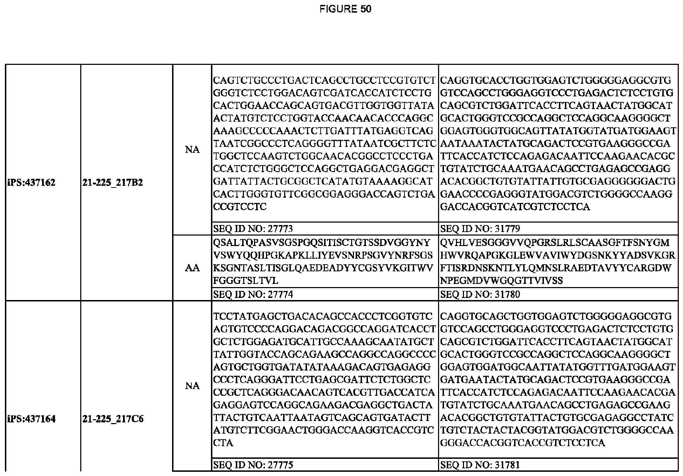

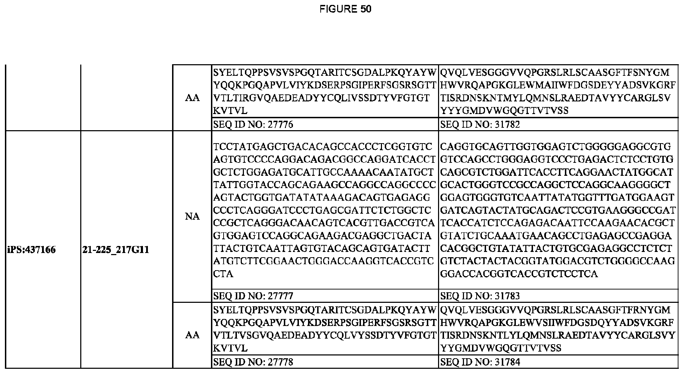

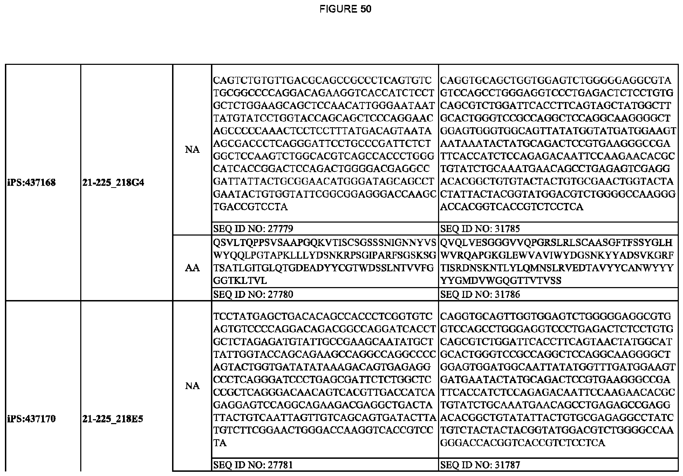

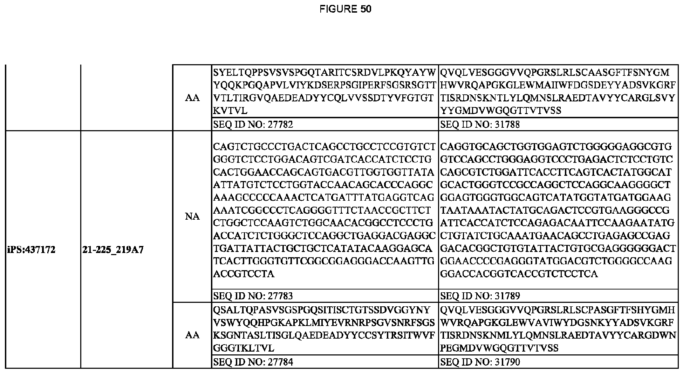

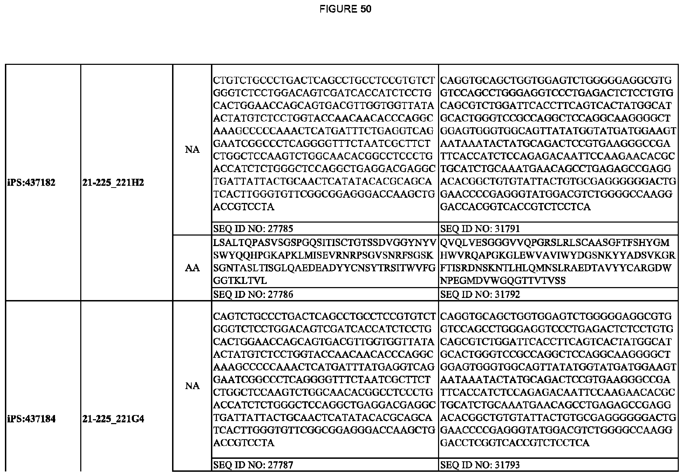

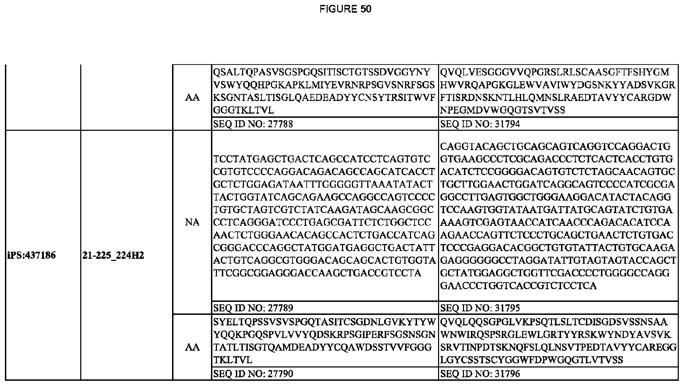

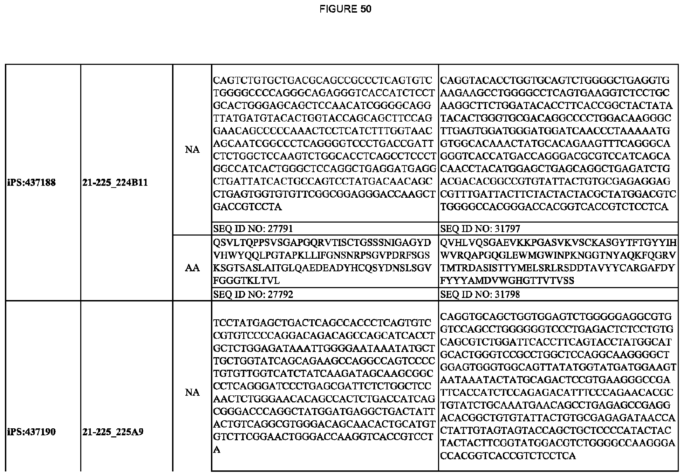

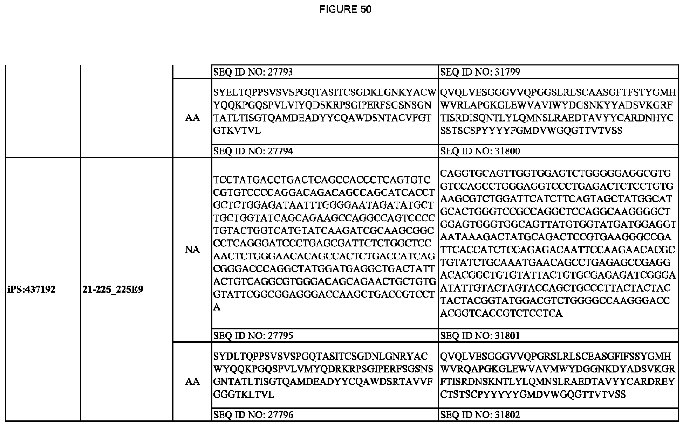

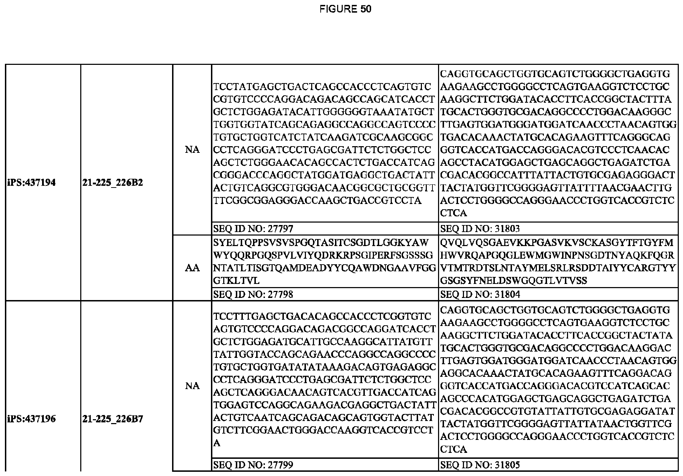

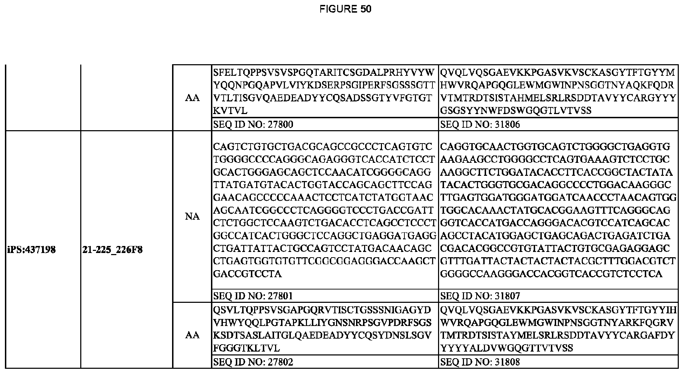

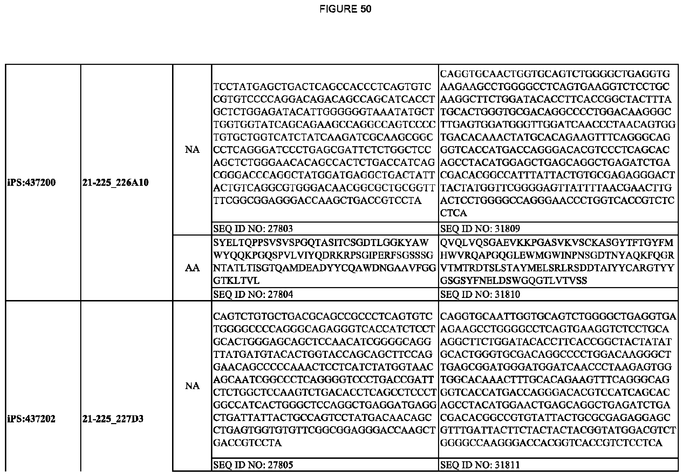

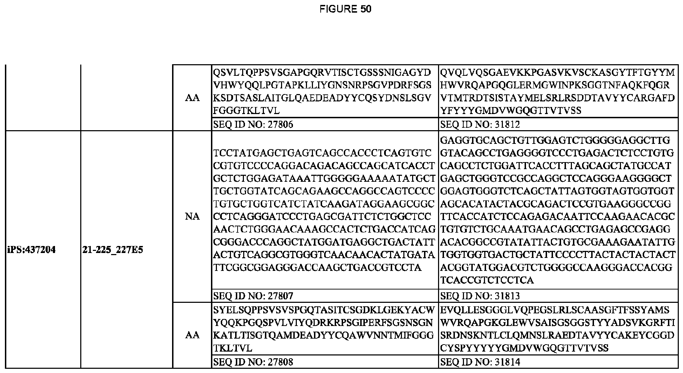

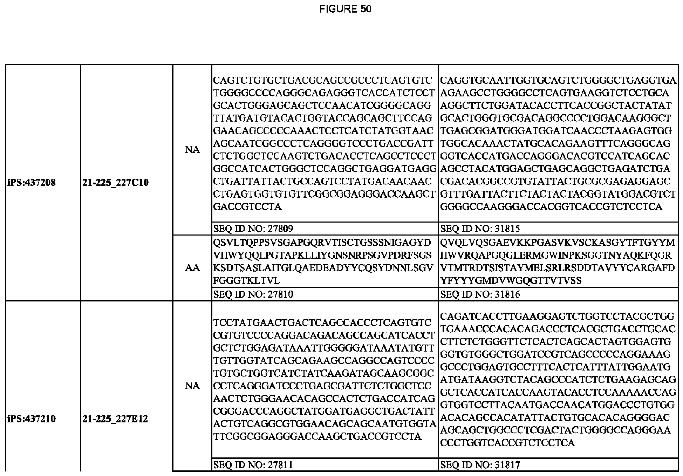

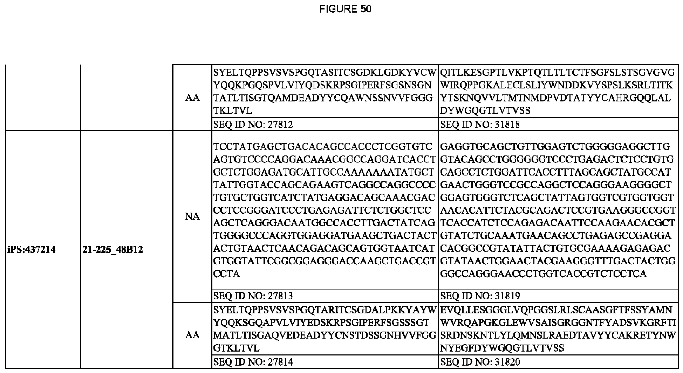

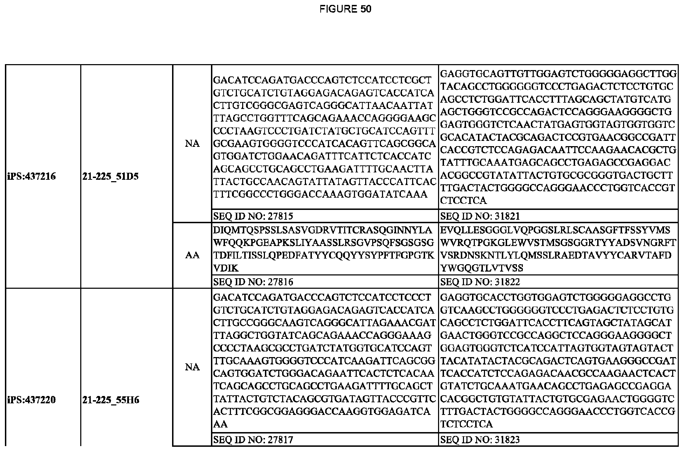

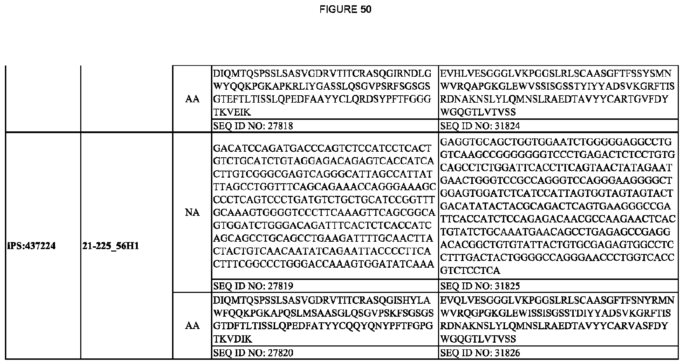

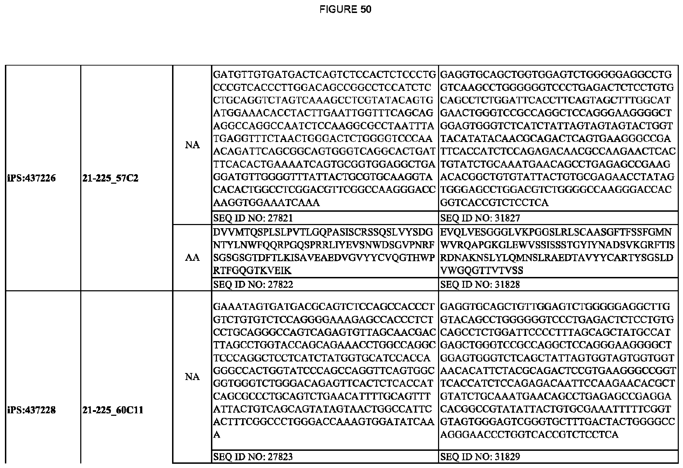

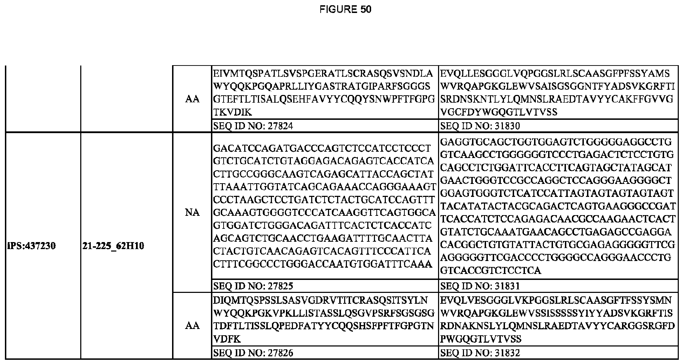

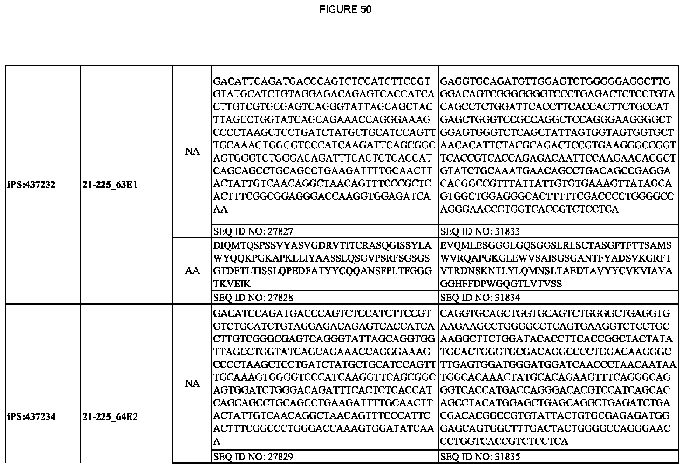

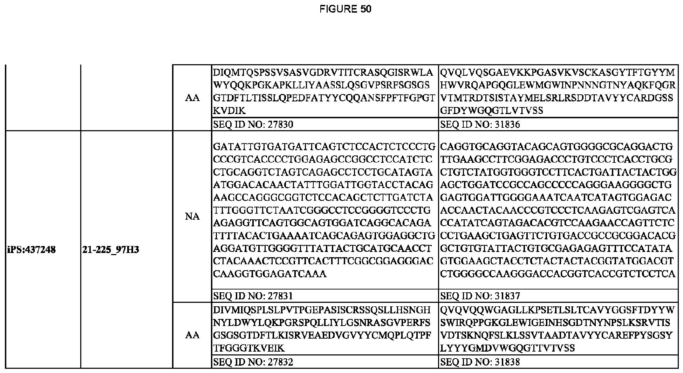

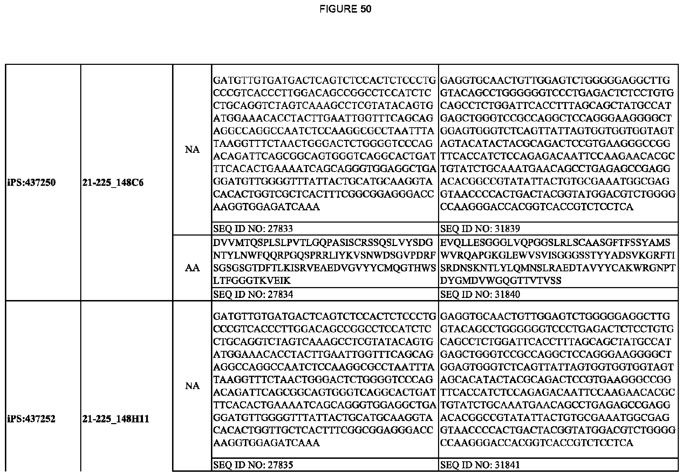

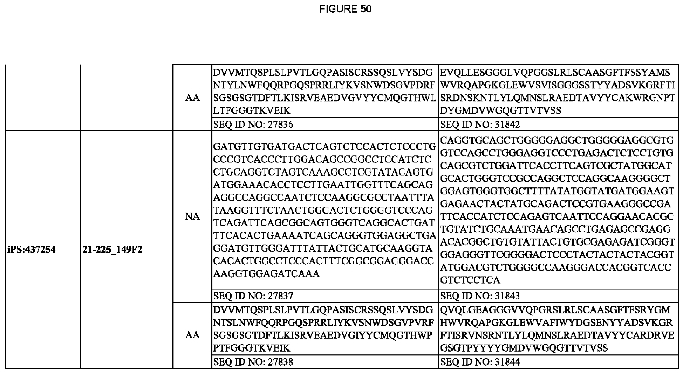

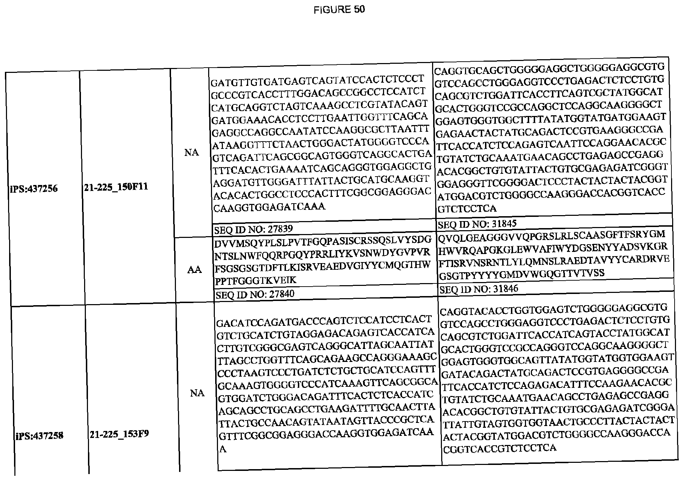

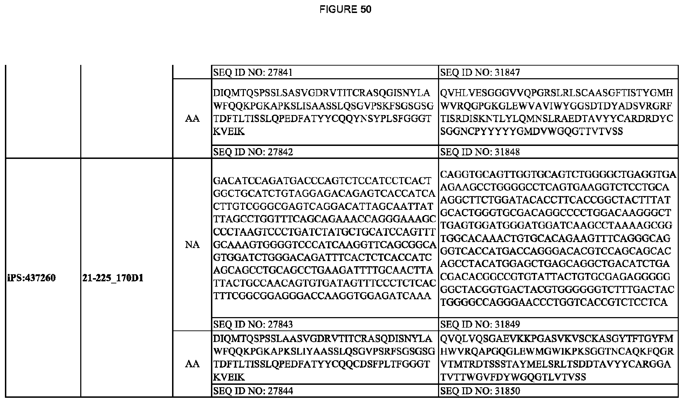

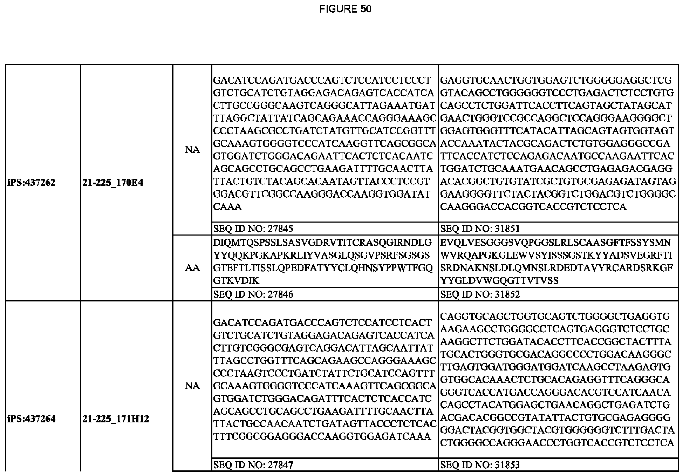

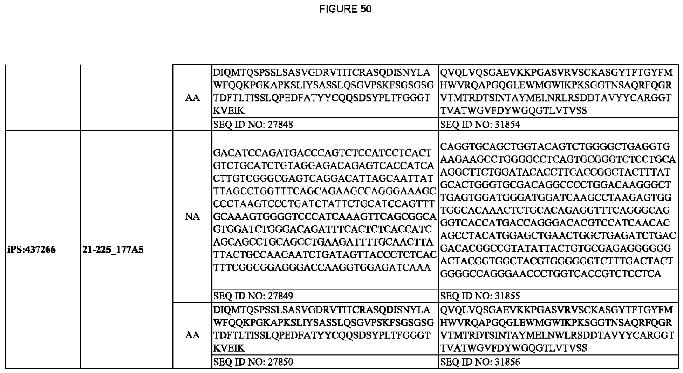

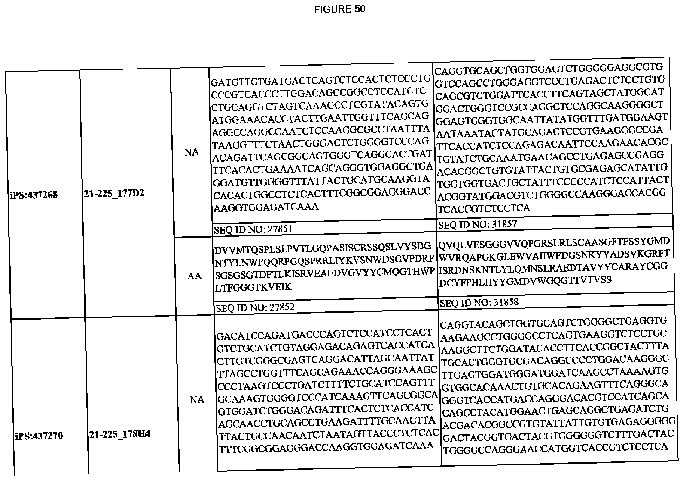

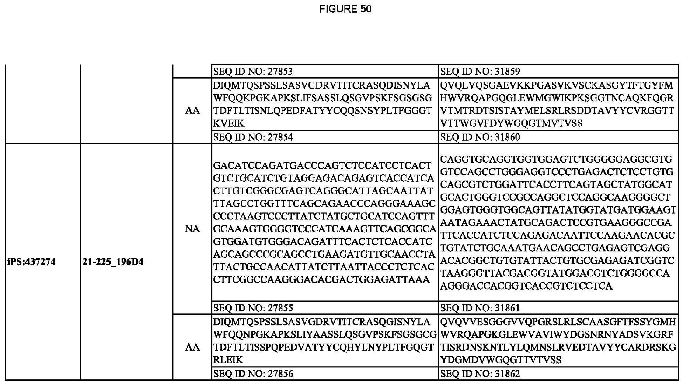

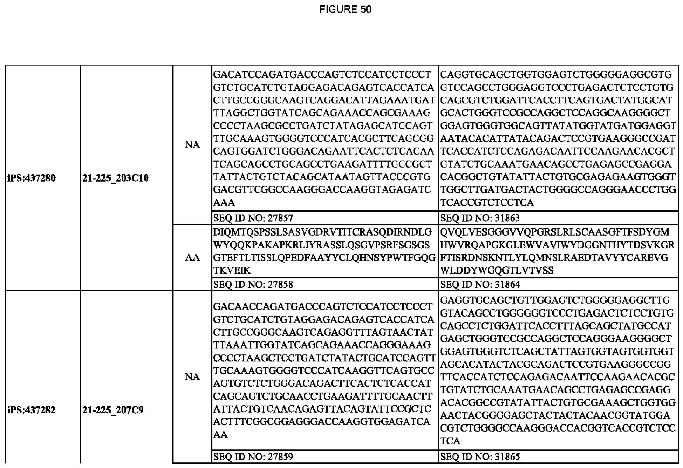

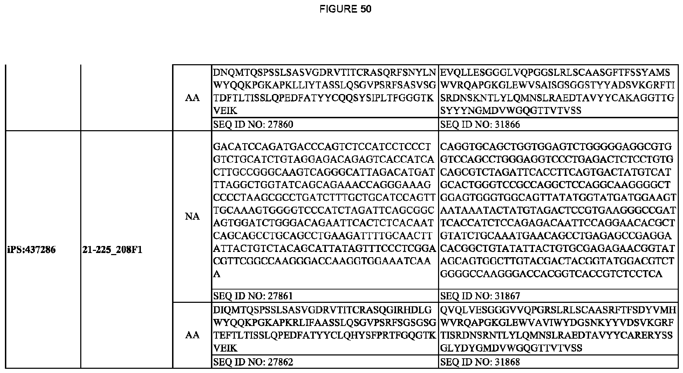

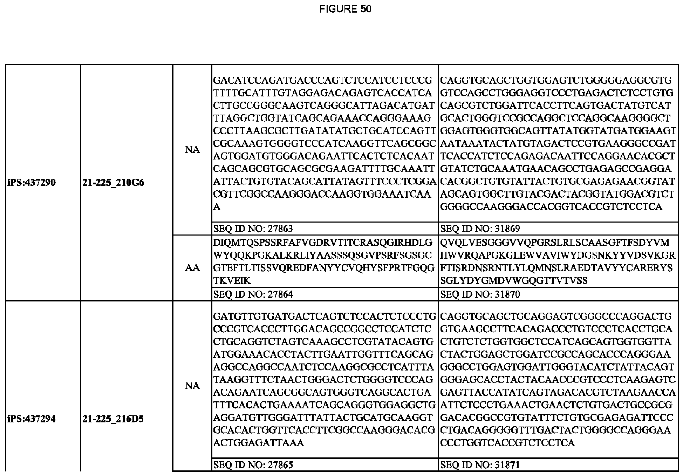

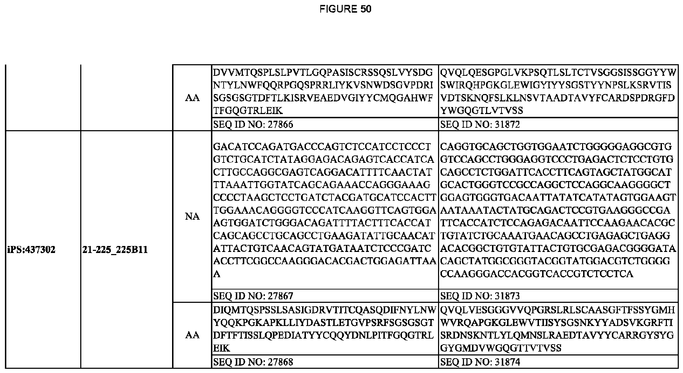

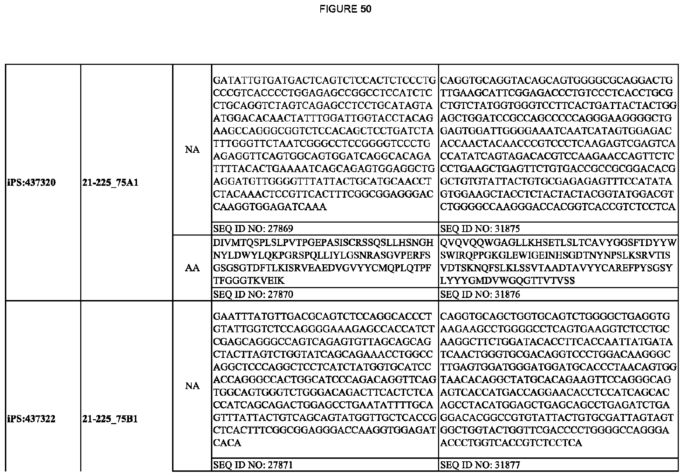

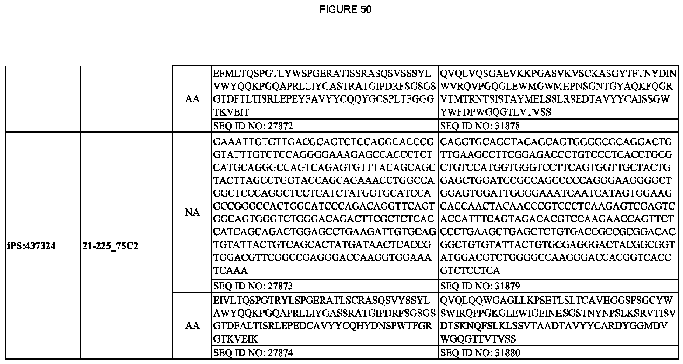

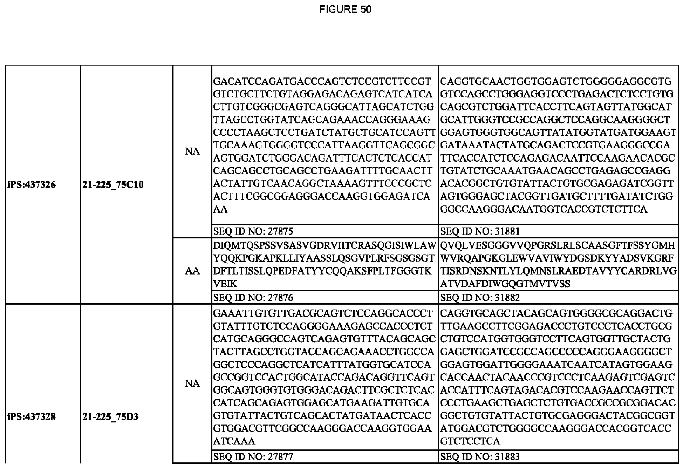

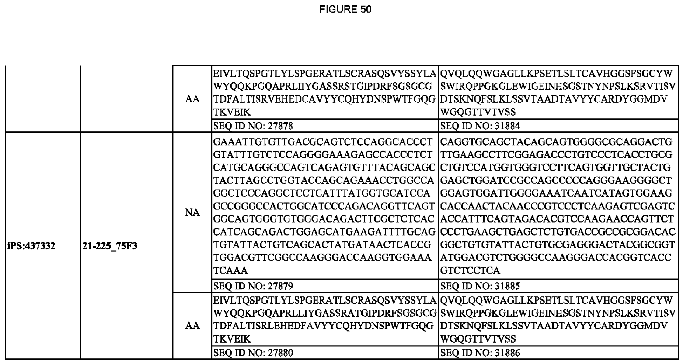

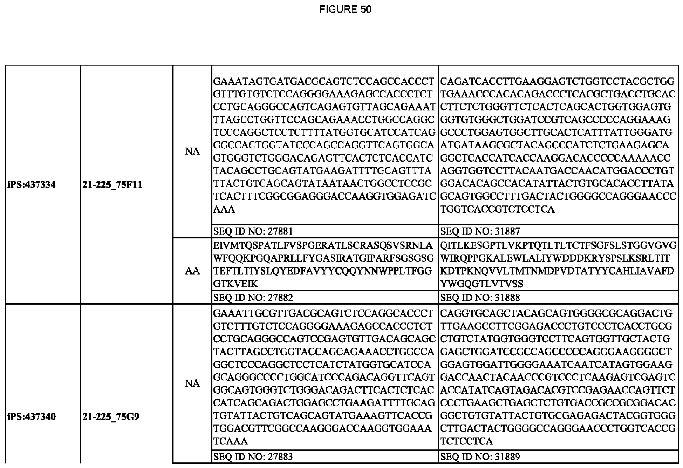

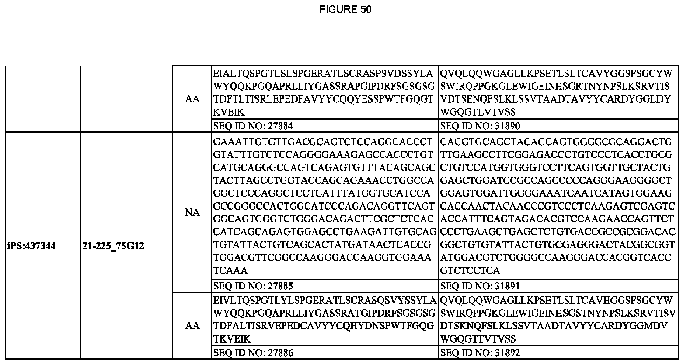

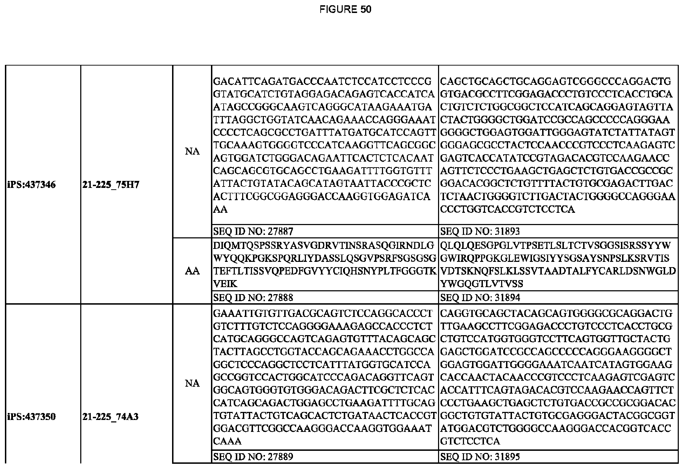

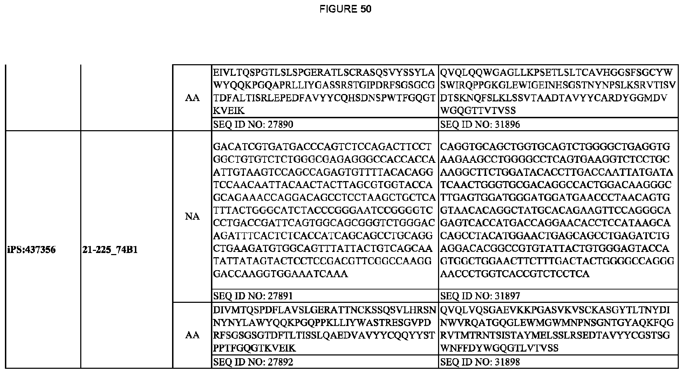

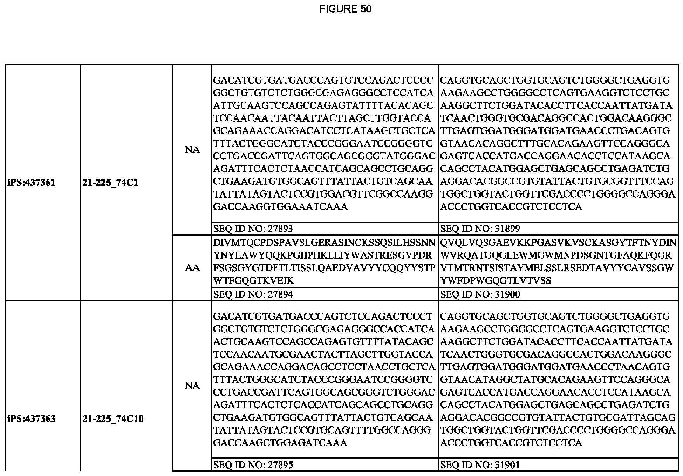

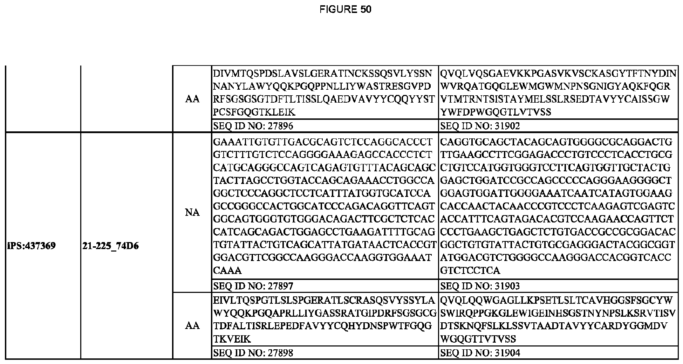

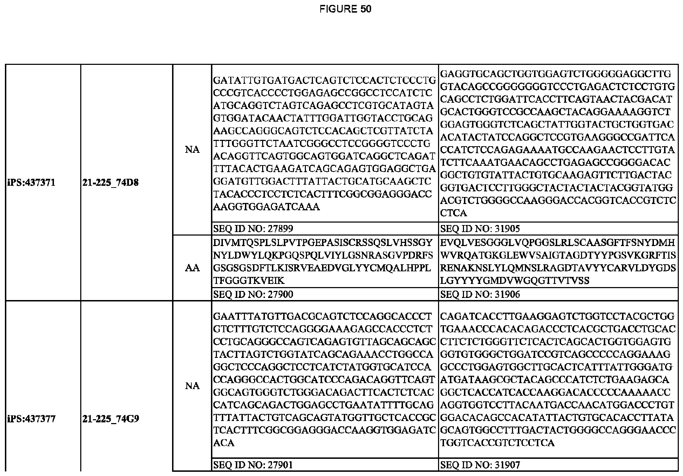

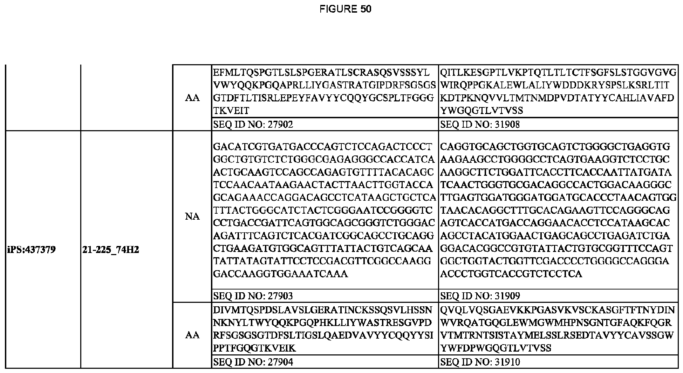

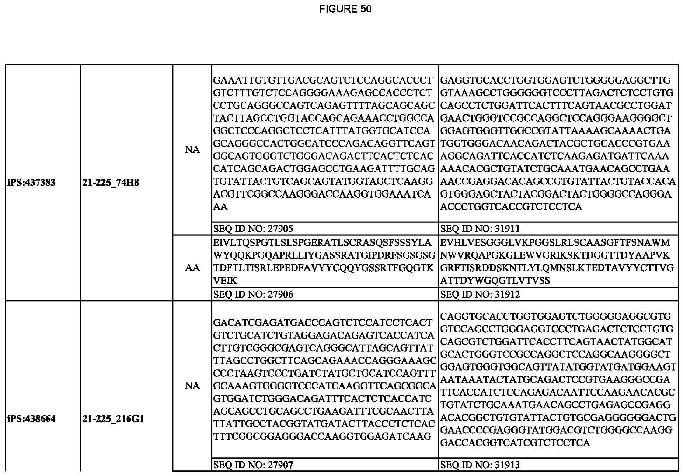

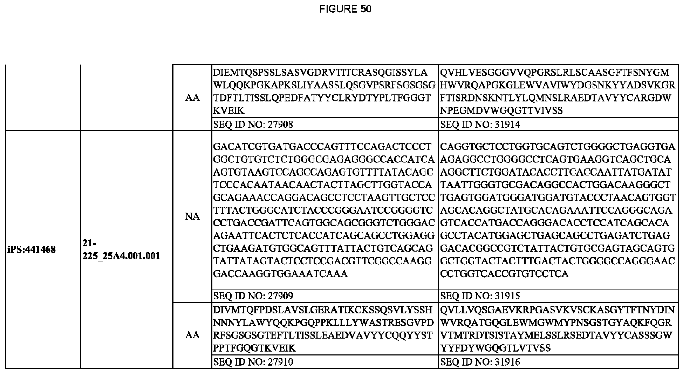

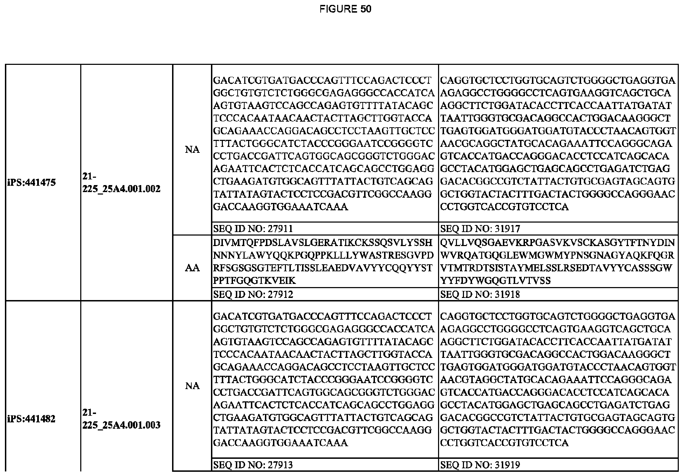

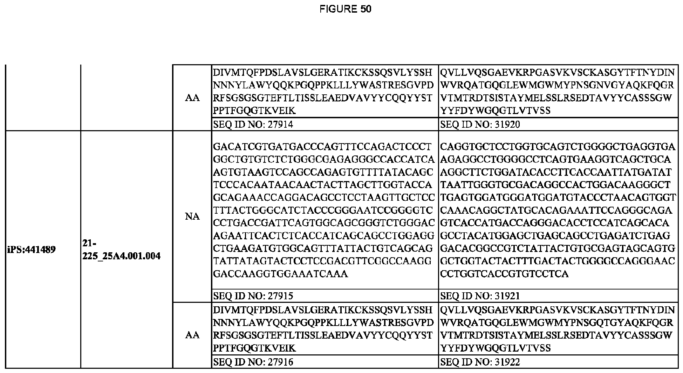

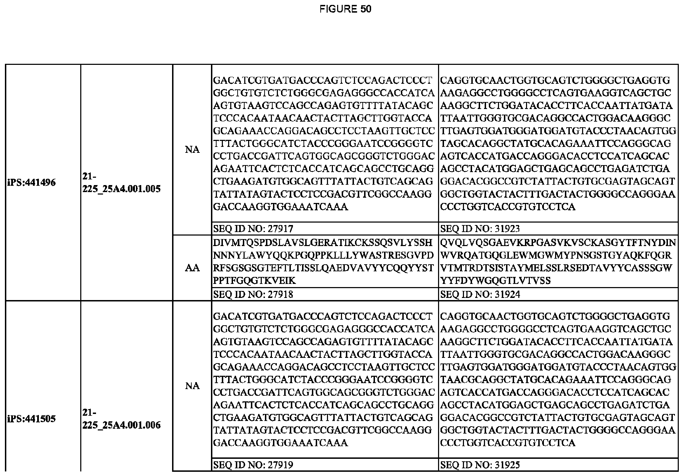

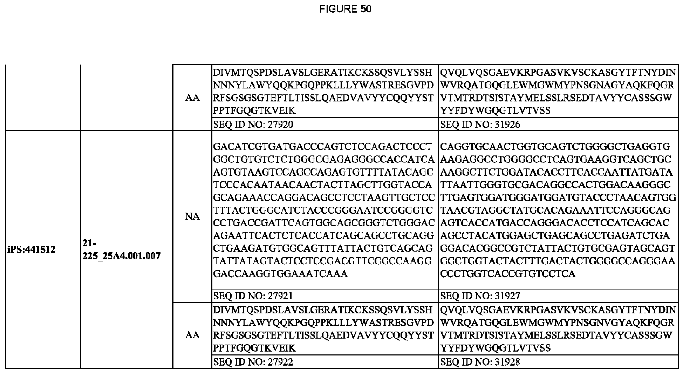

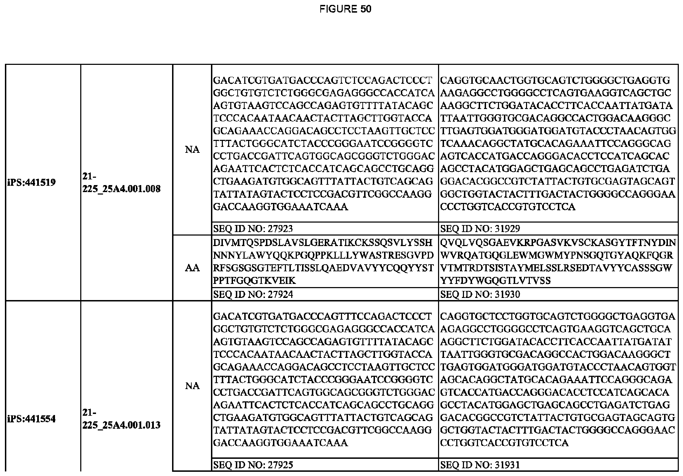

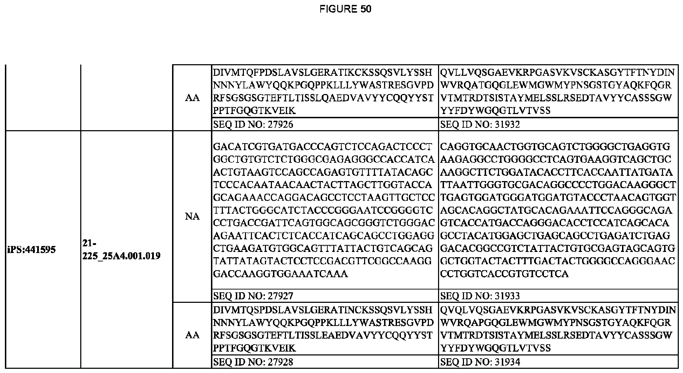

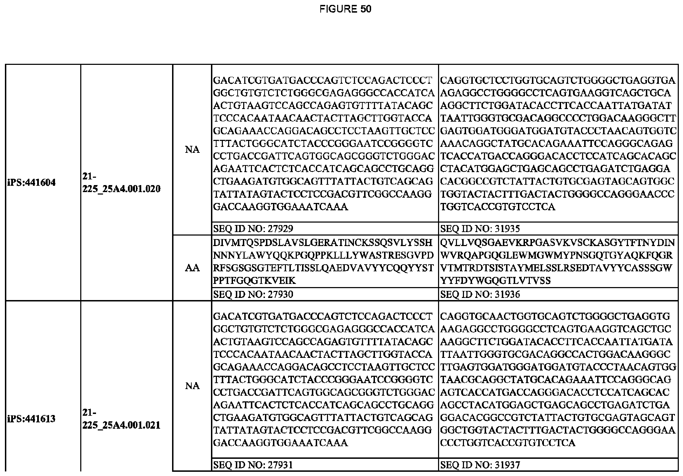

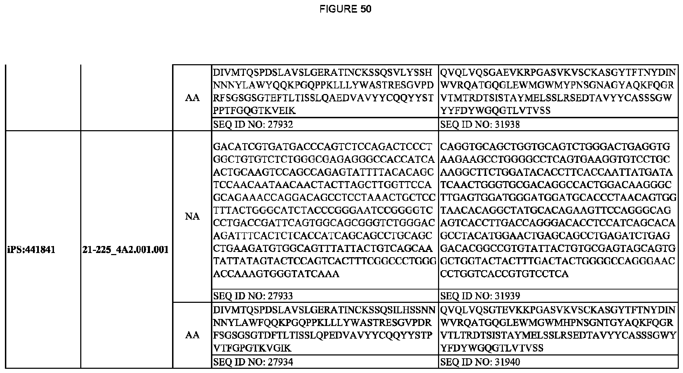

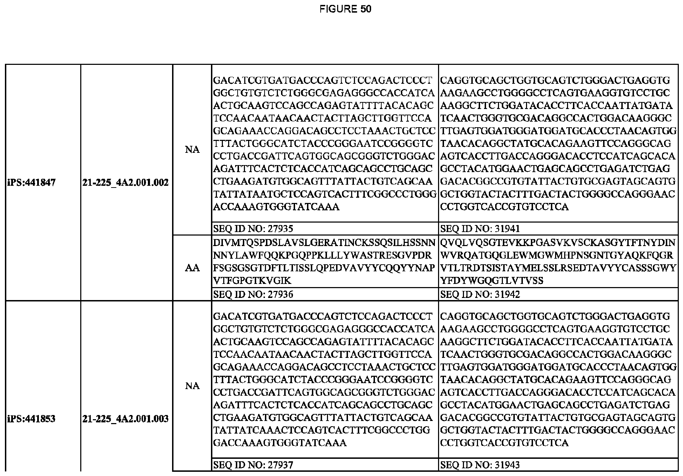

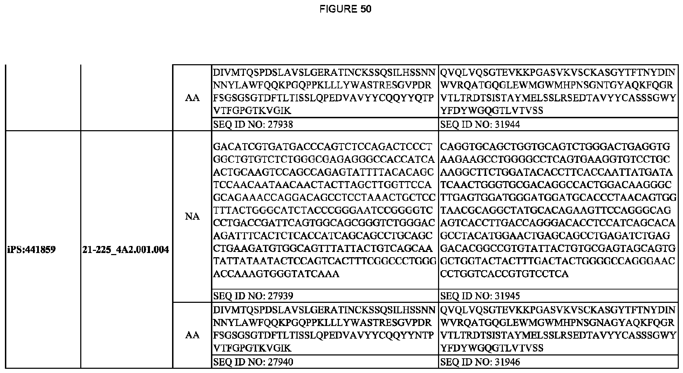

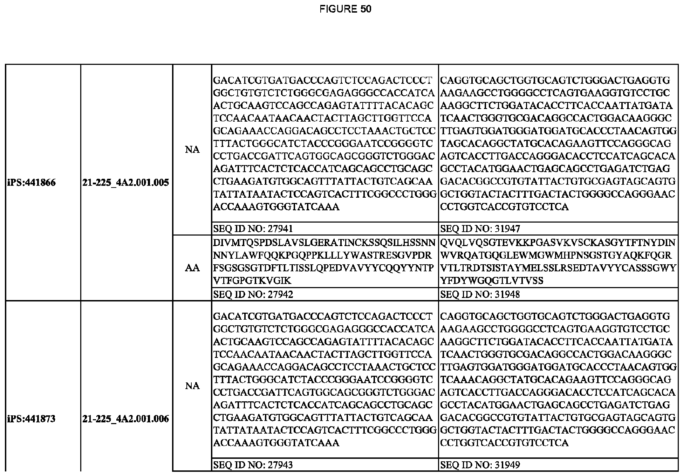

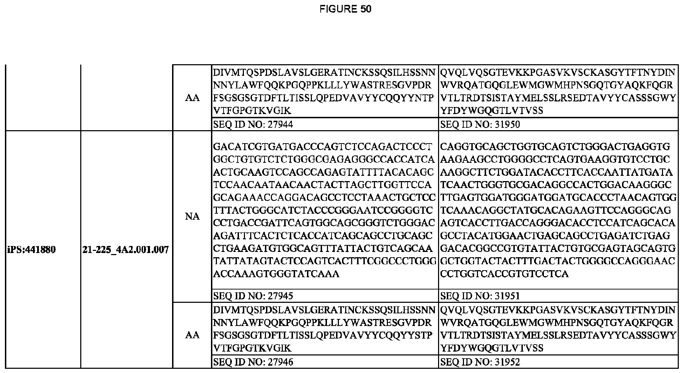

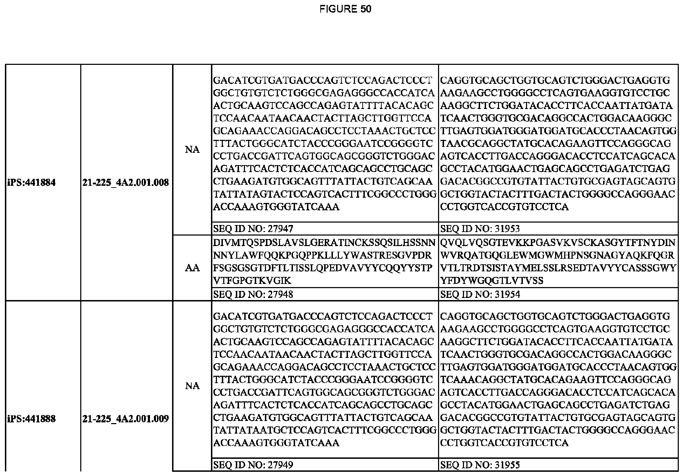

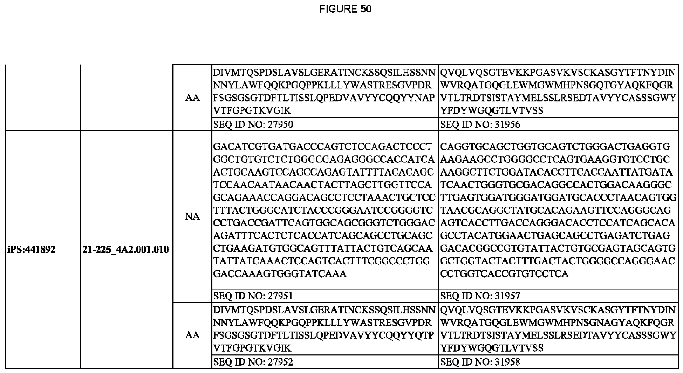

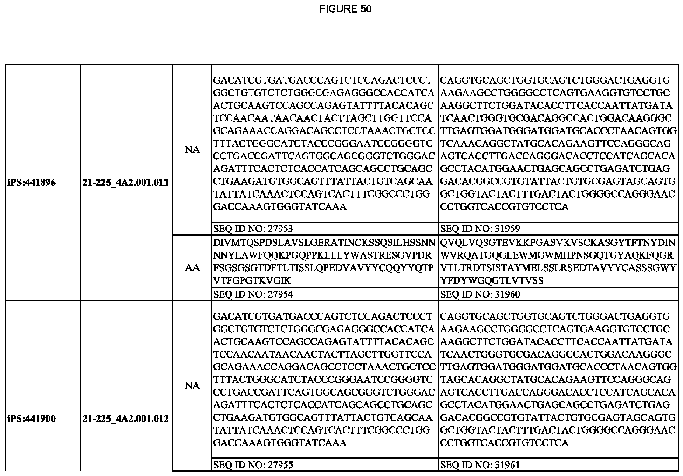

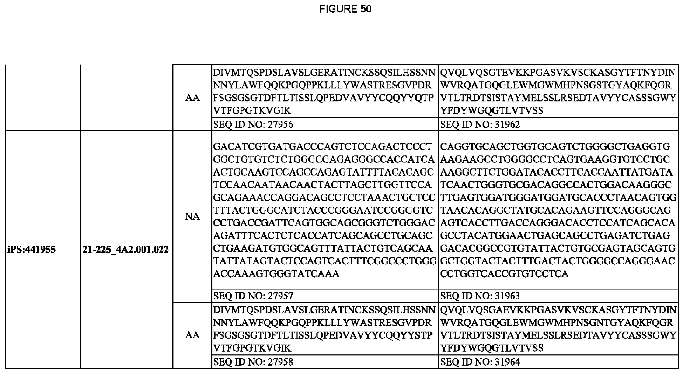

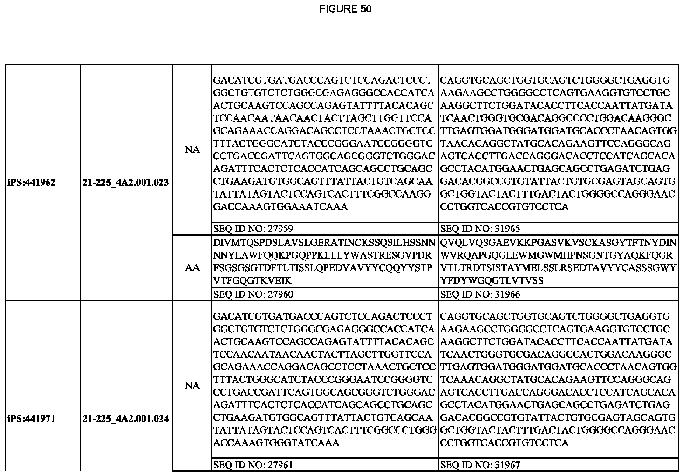

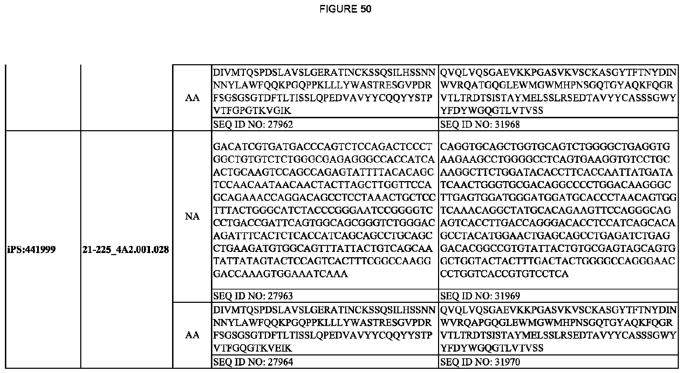

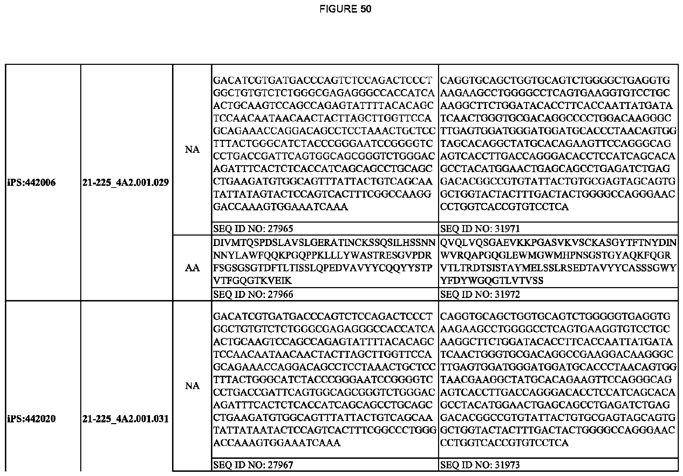

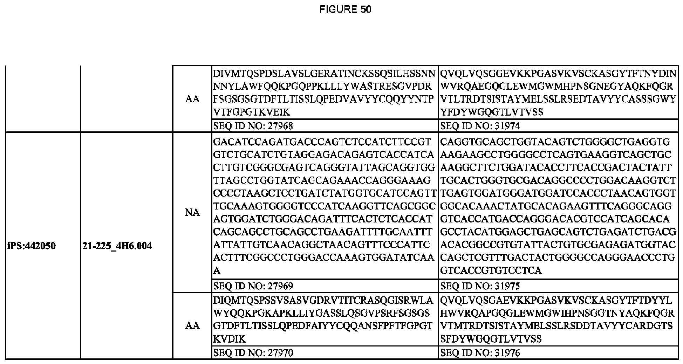

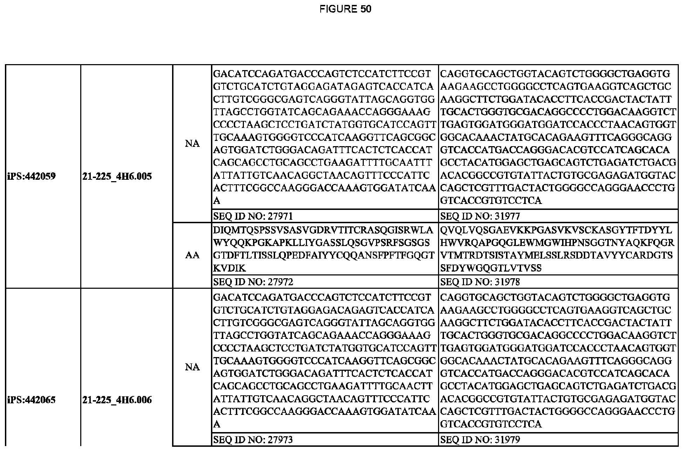

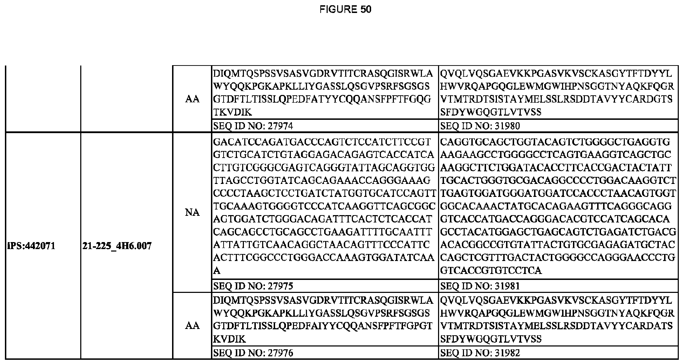

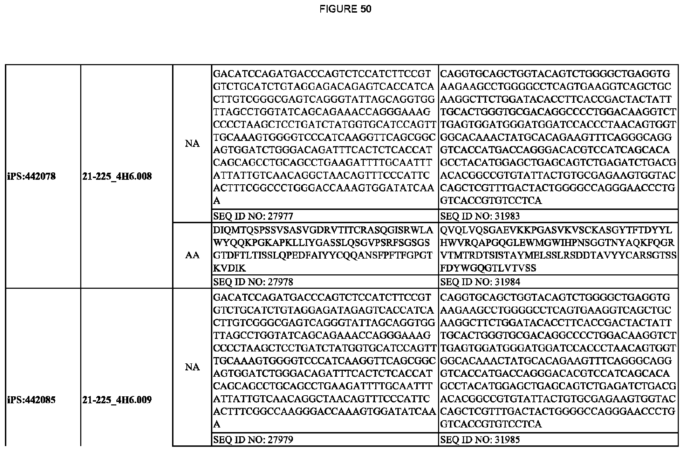

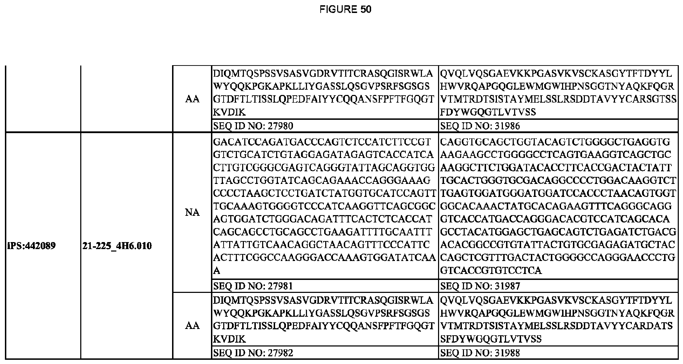

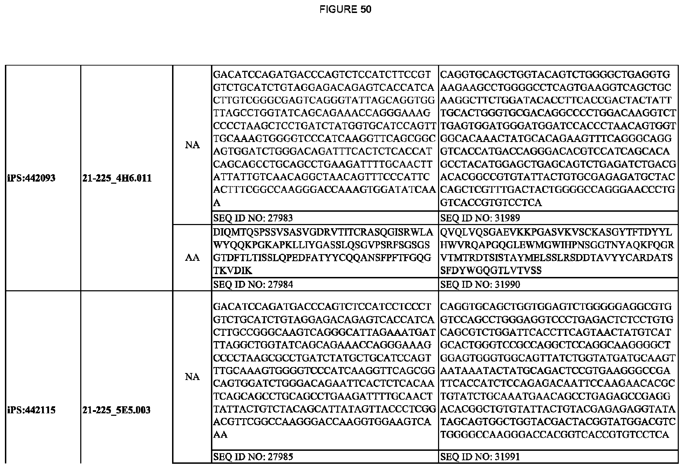

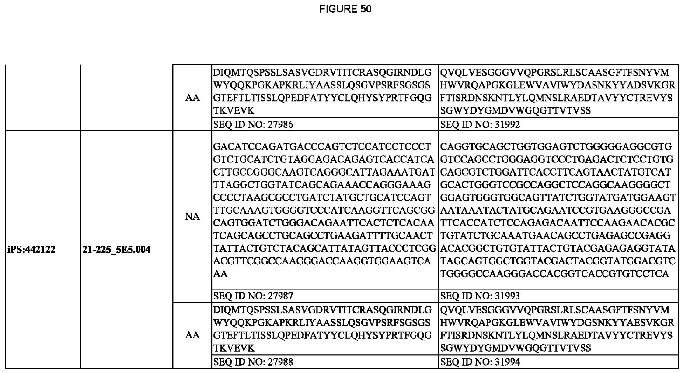

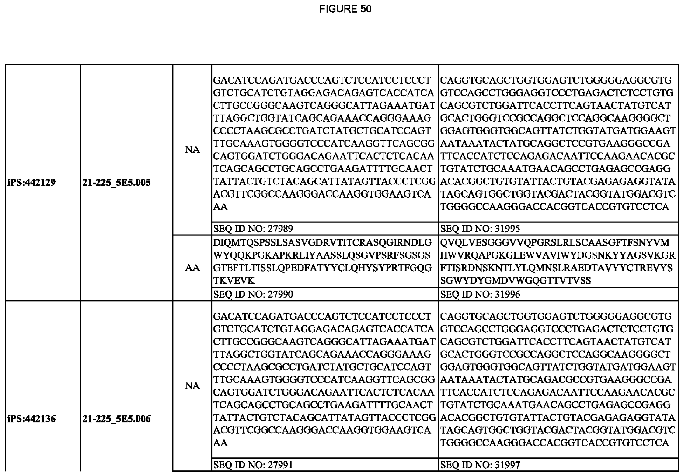

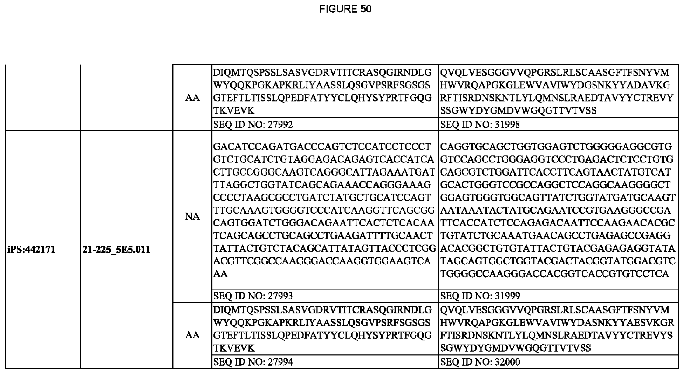

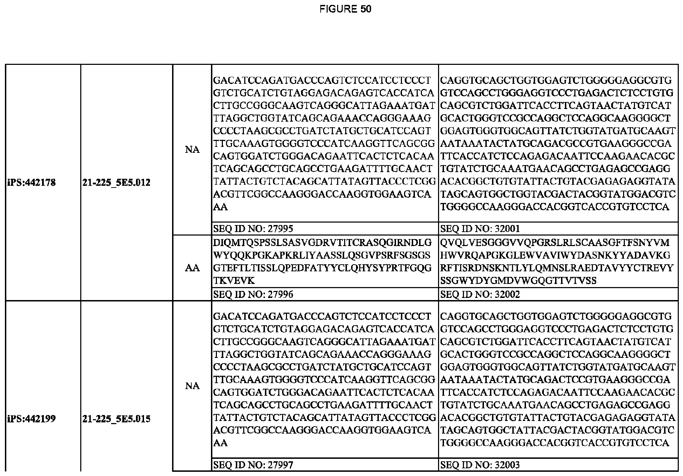

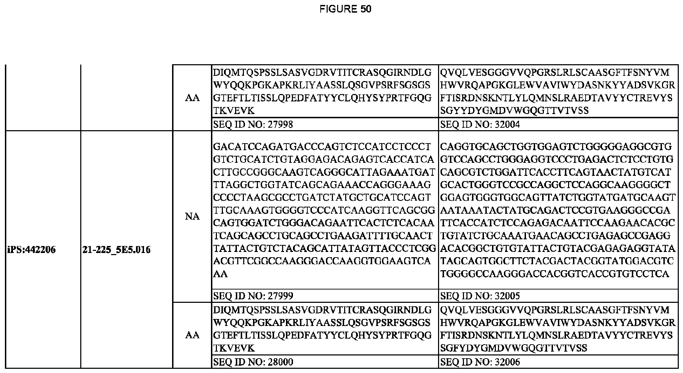

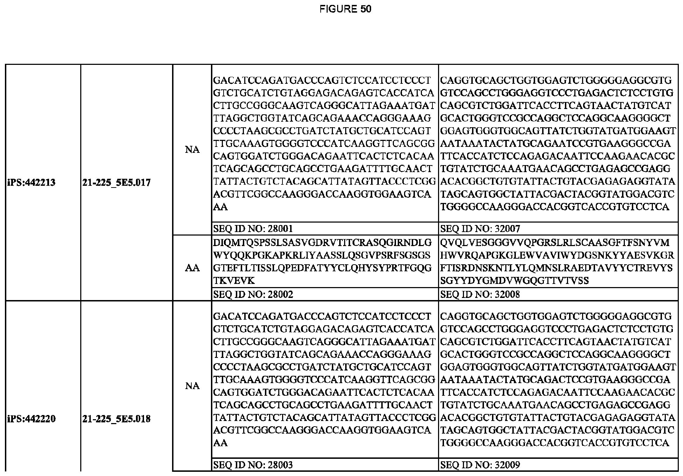

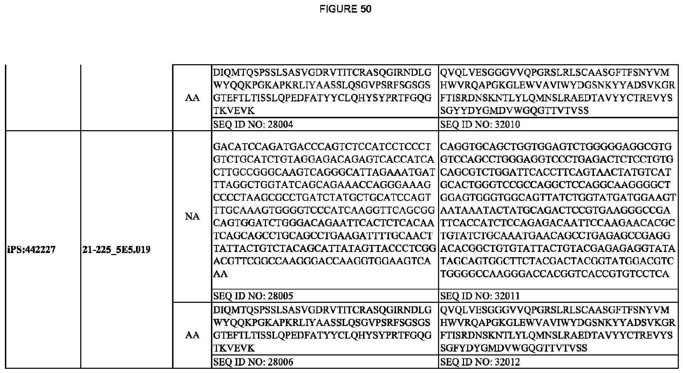

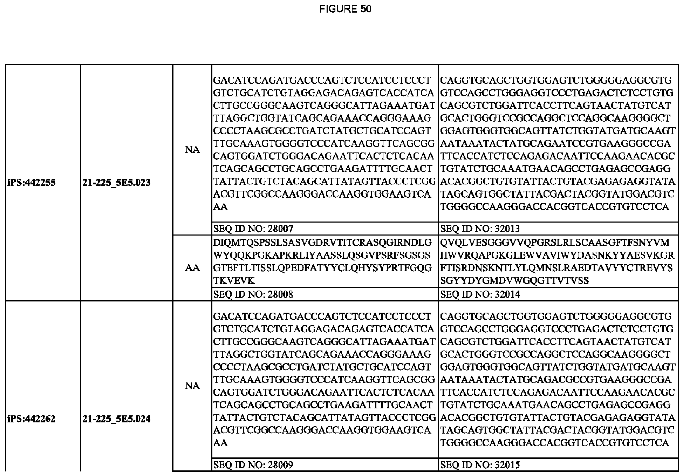

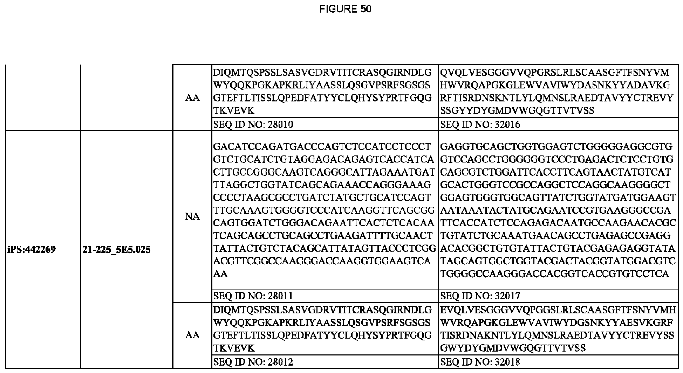

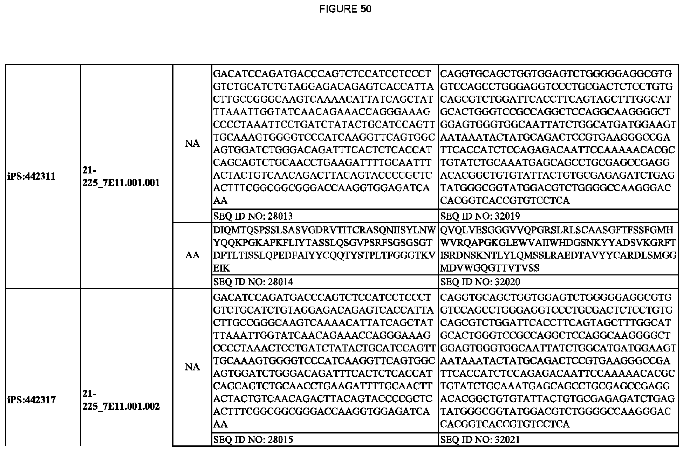

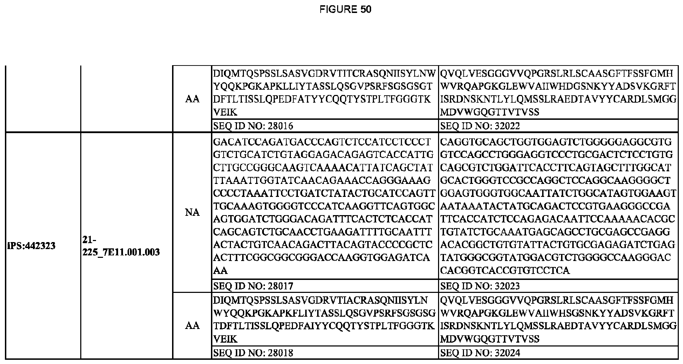

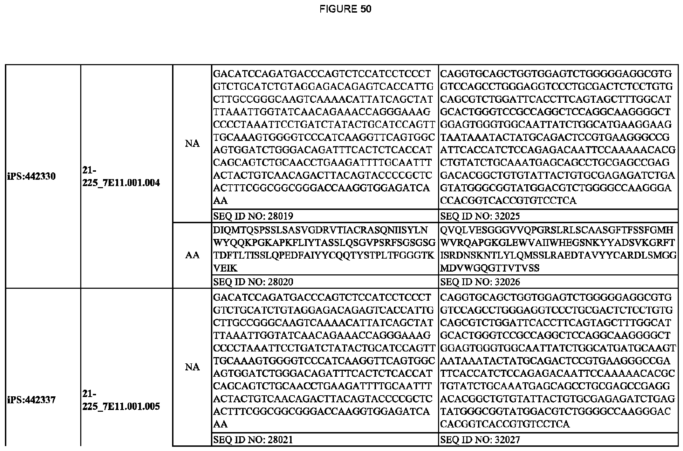

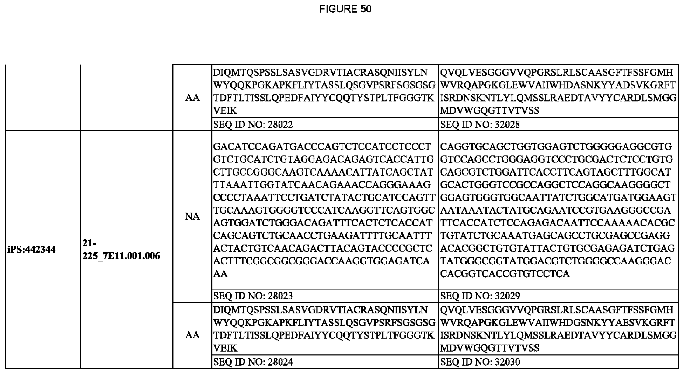

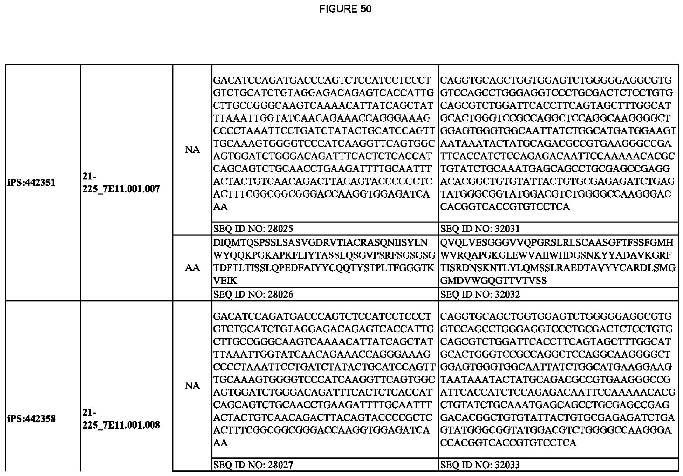

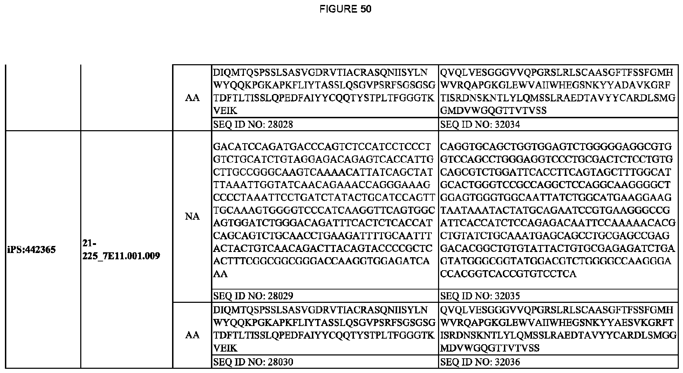

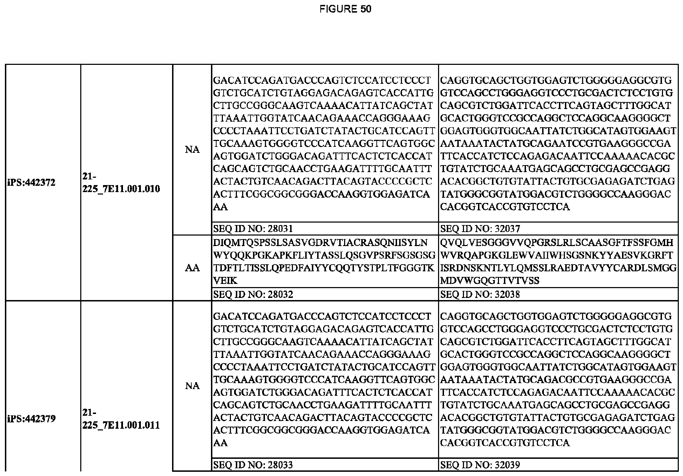

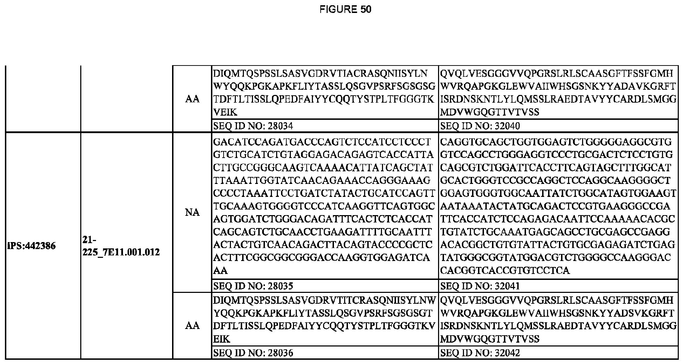

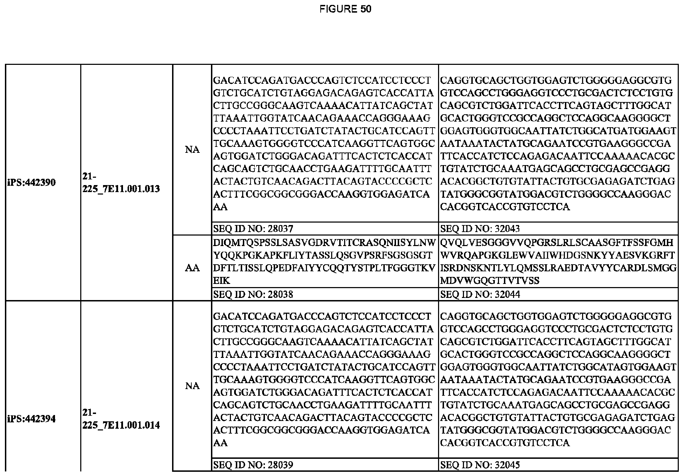

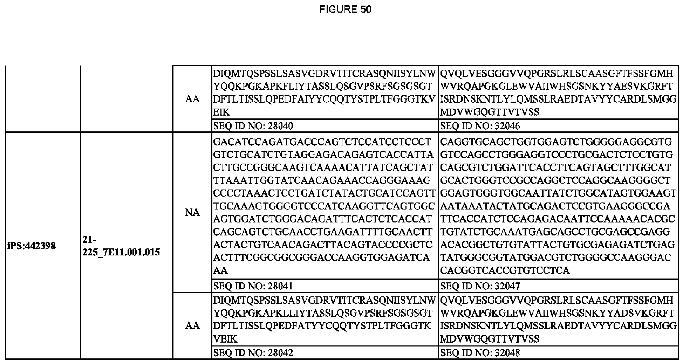

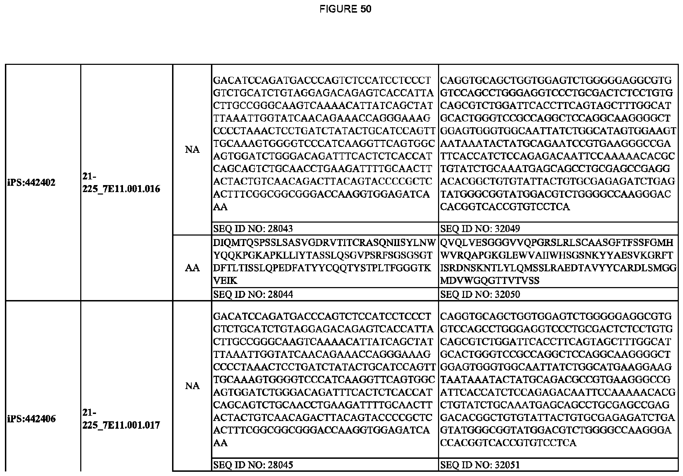

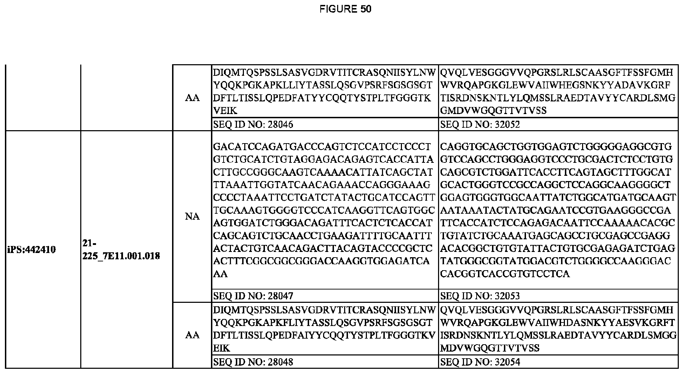

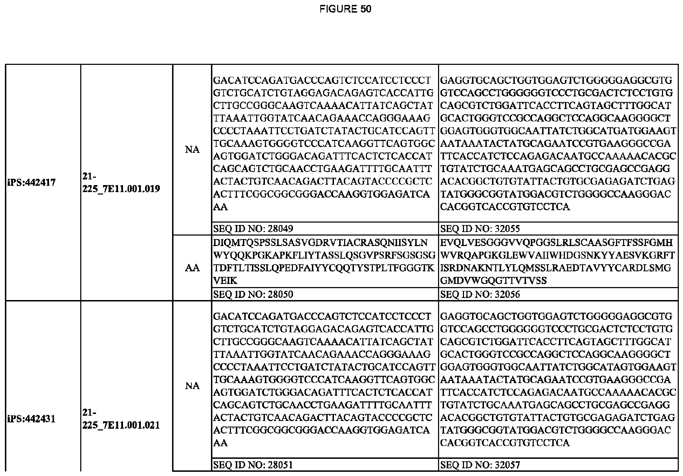

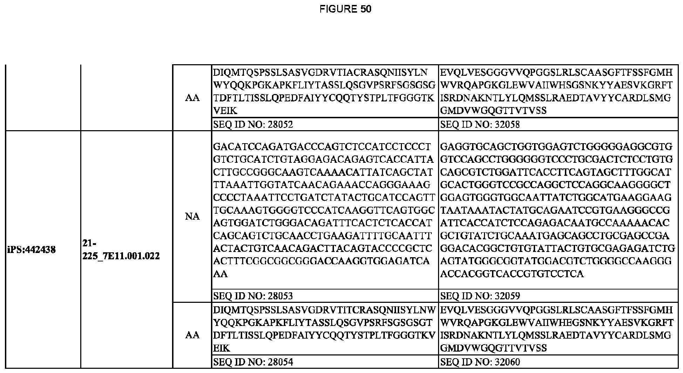

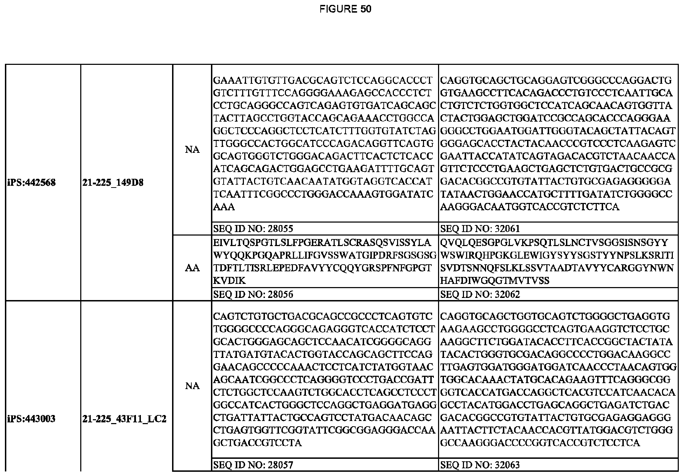

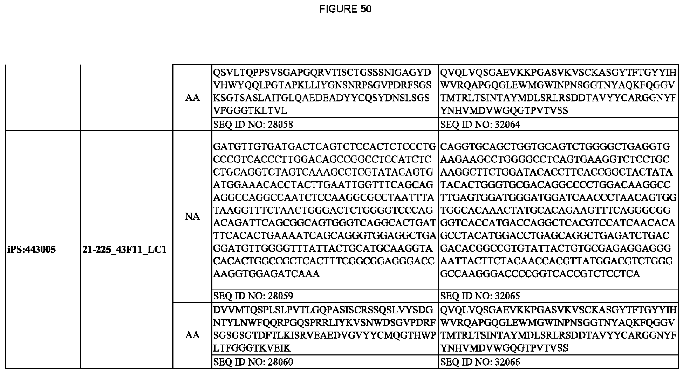

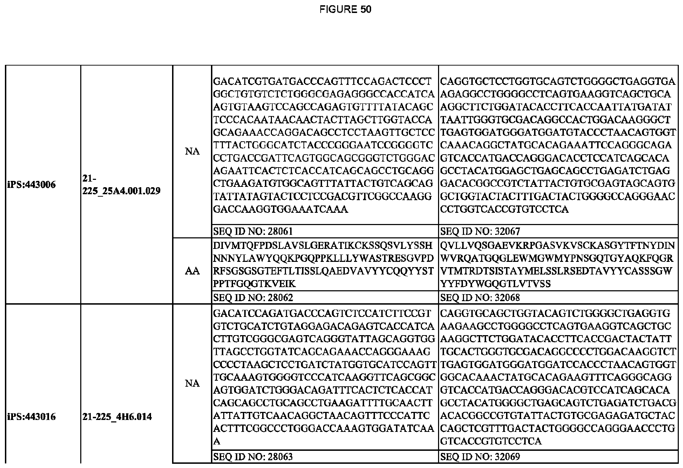

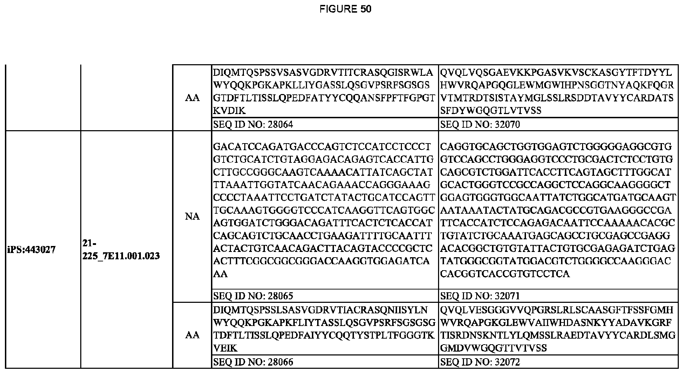

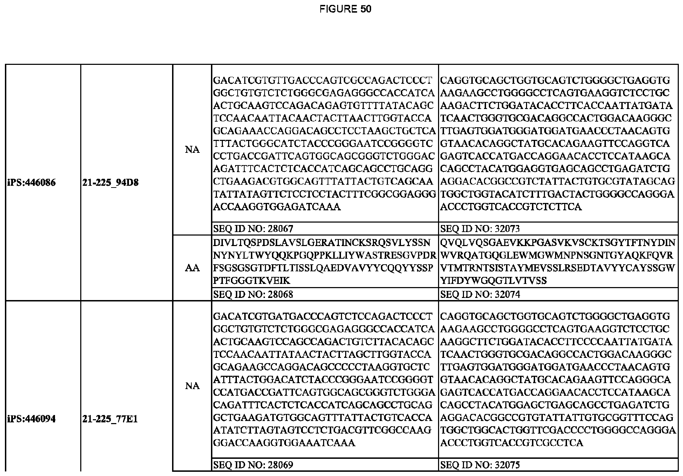

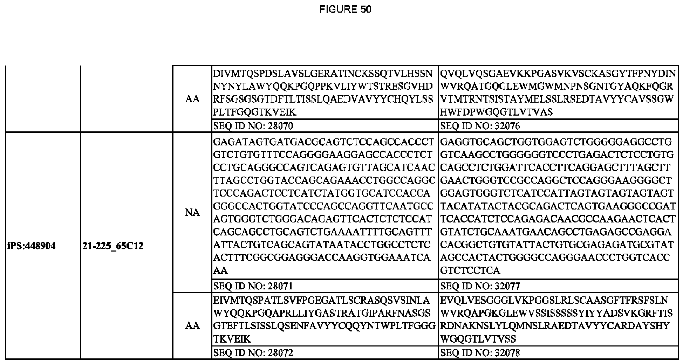

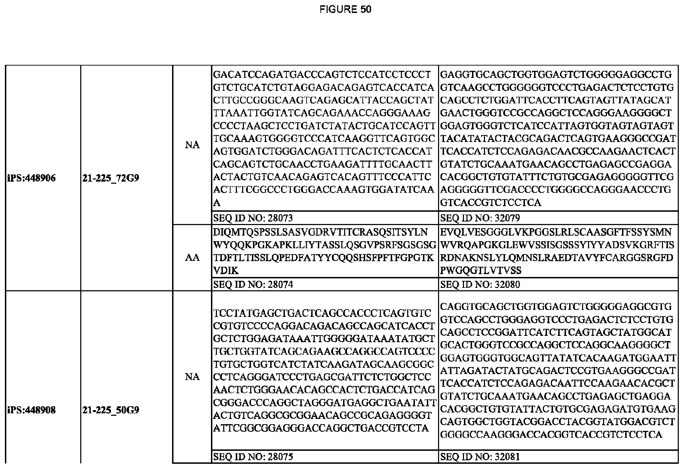

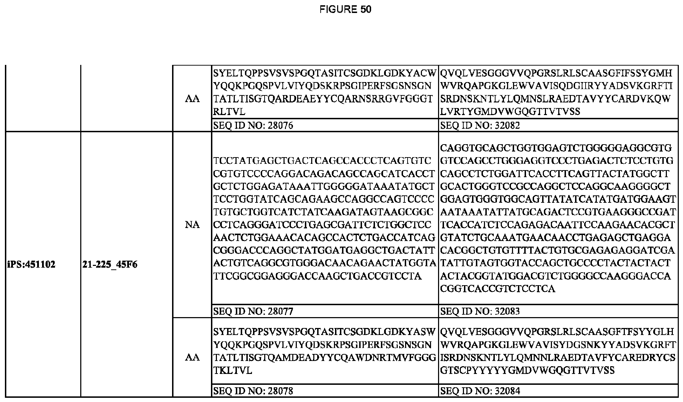

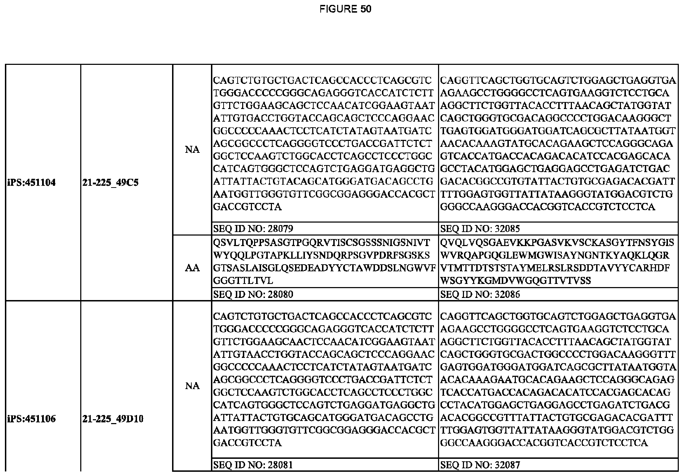

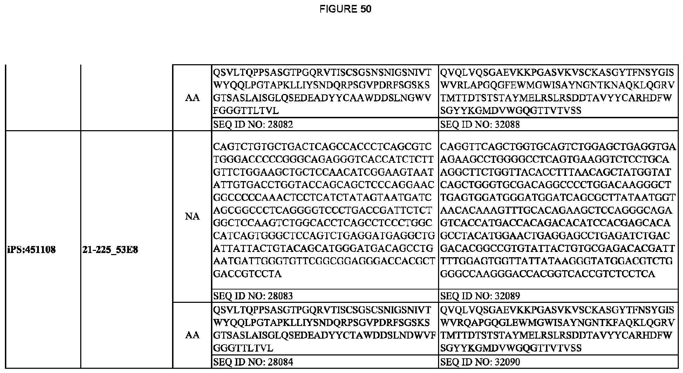

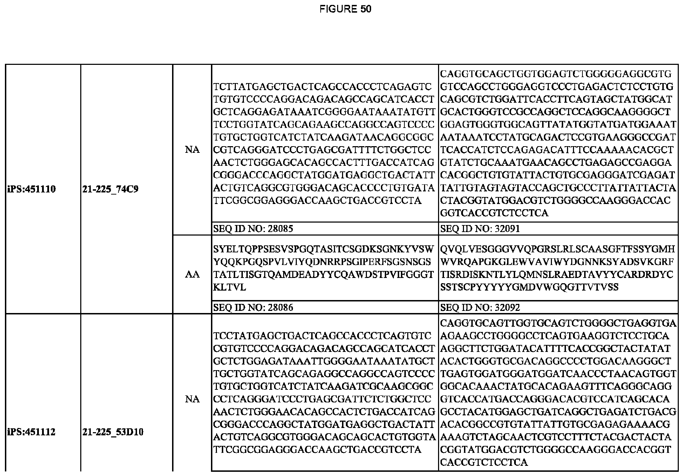

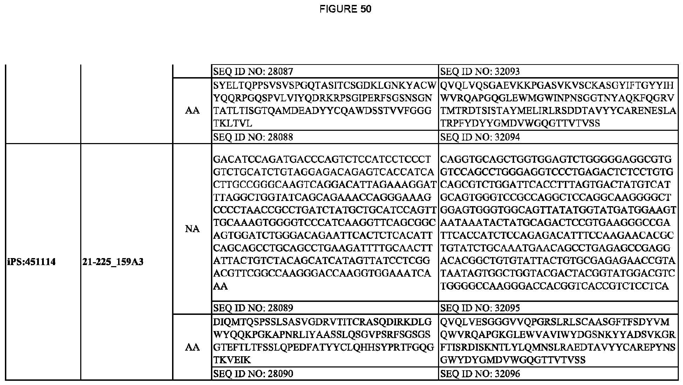

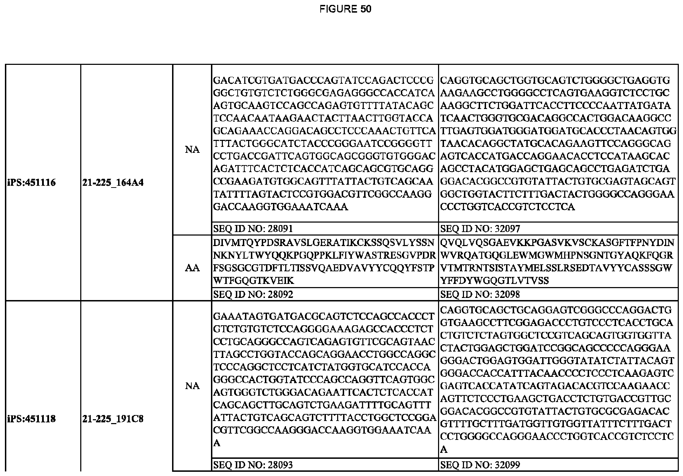

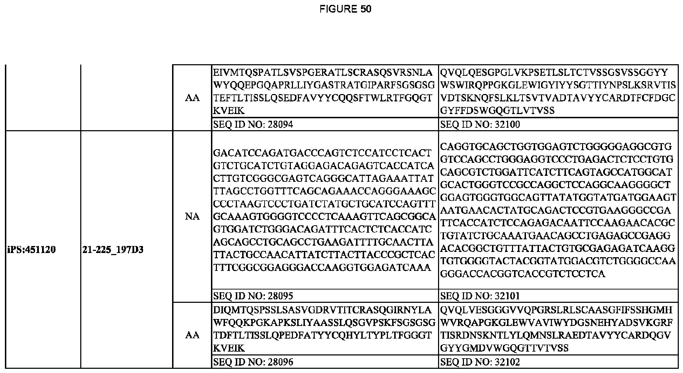

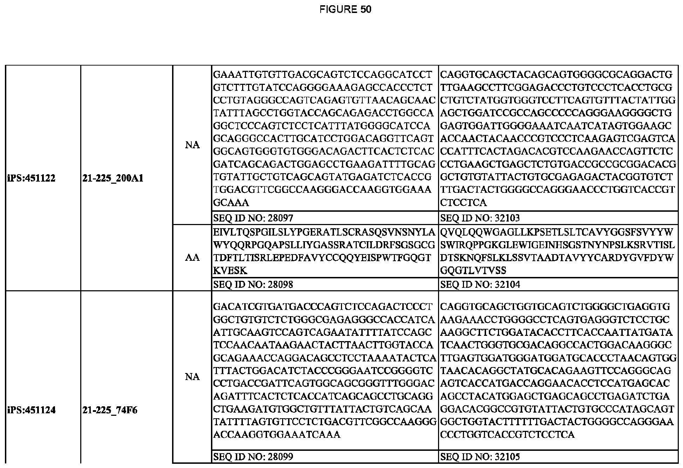

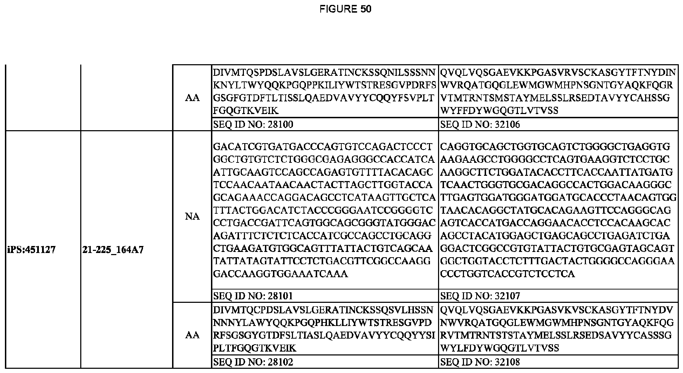

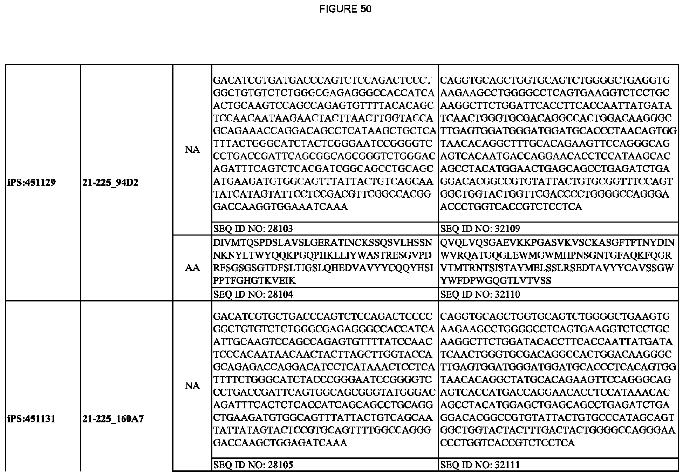

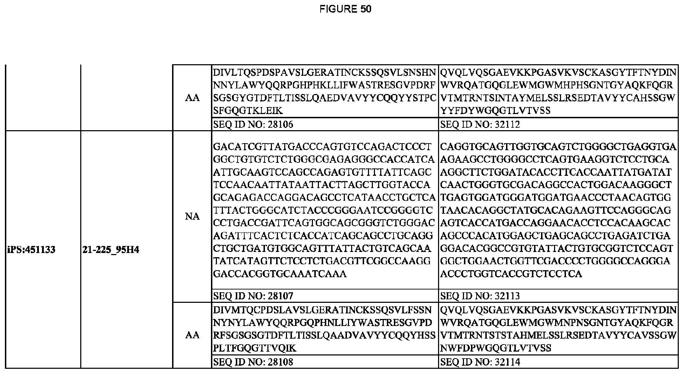

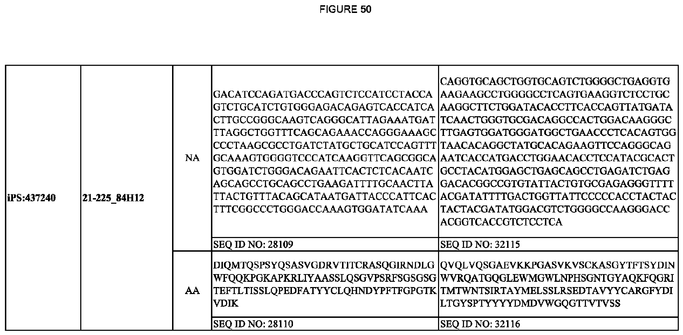

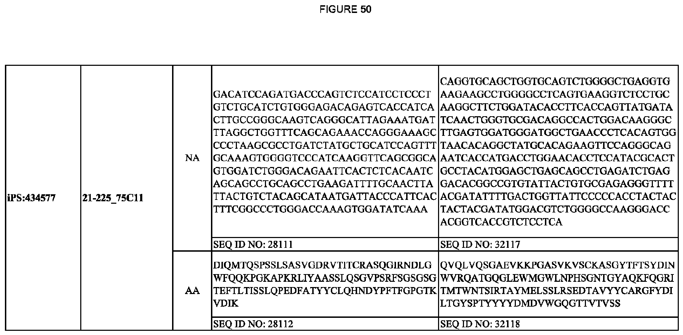

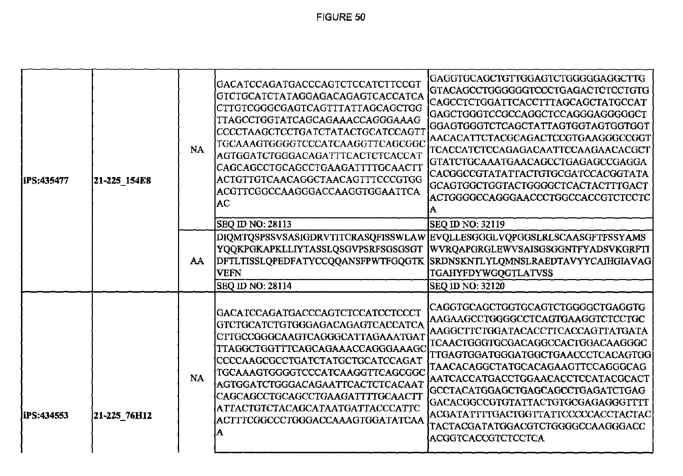

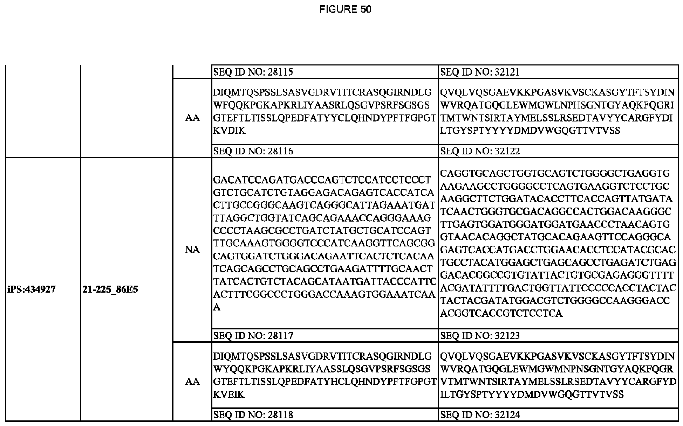

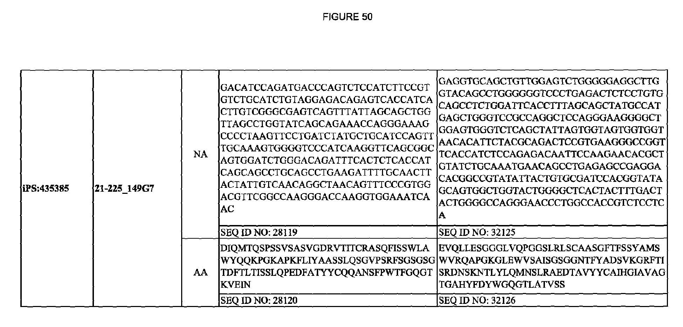

**Figure 50.** A table presenting the amino acid sequences of the light and heavy chain variable domains for certain antigen binding proteins of the present invention are displayed in a table (Table 3). The amino acid sequences of the light and heavy chain variable domains in Table 3 are wrapped due to space issues, and unless stated otherwise, should be understood to be single amino acid sequences.

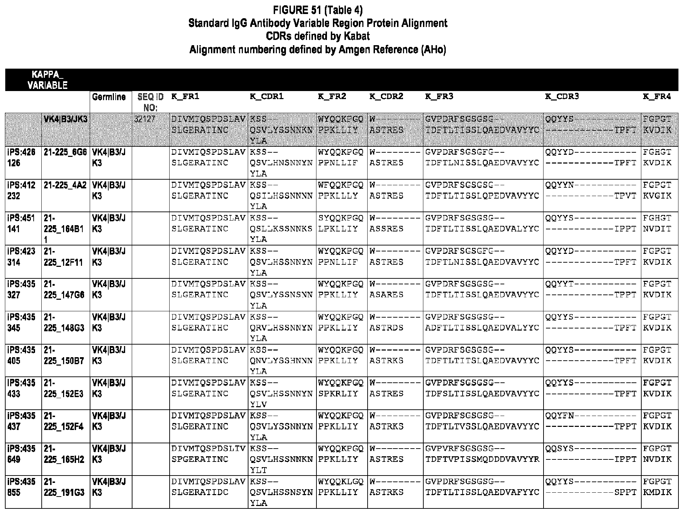

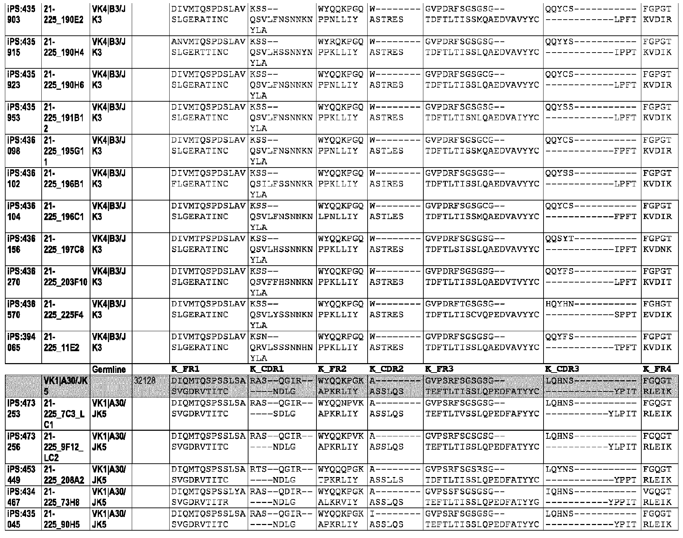

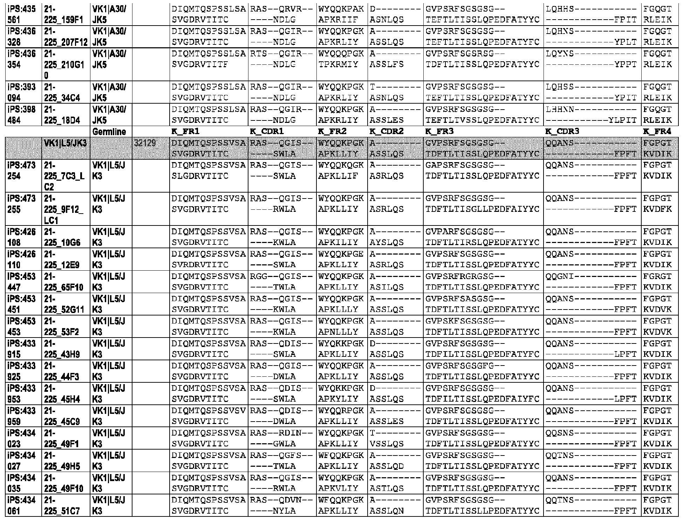

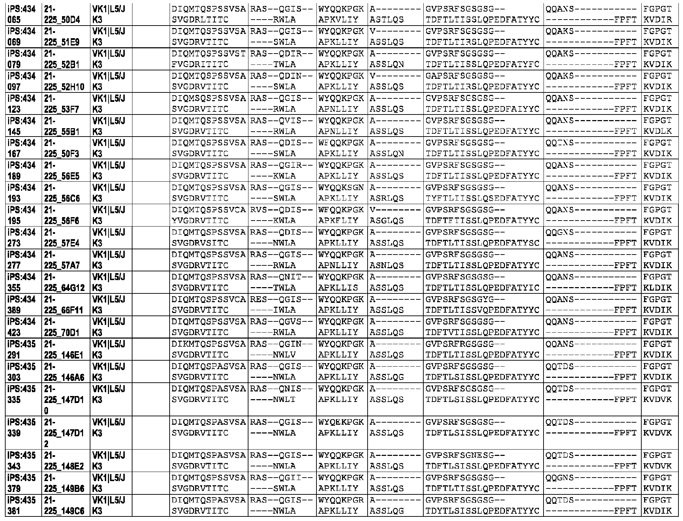

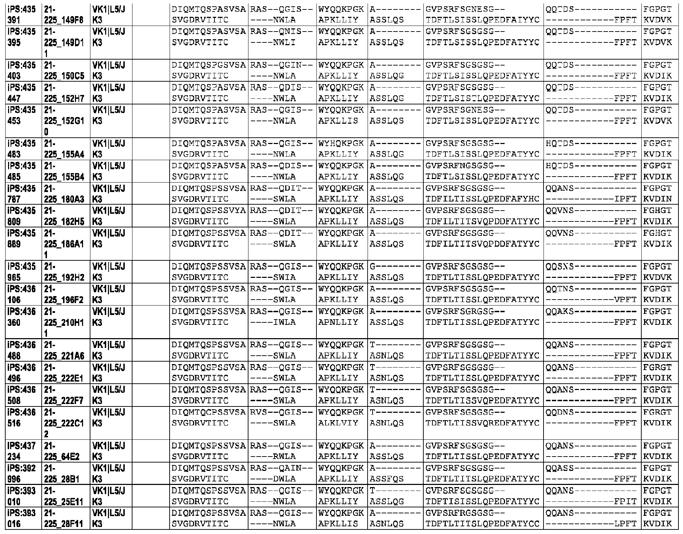

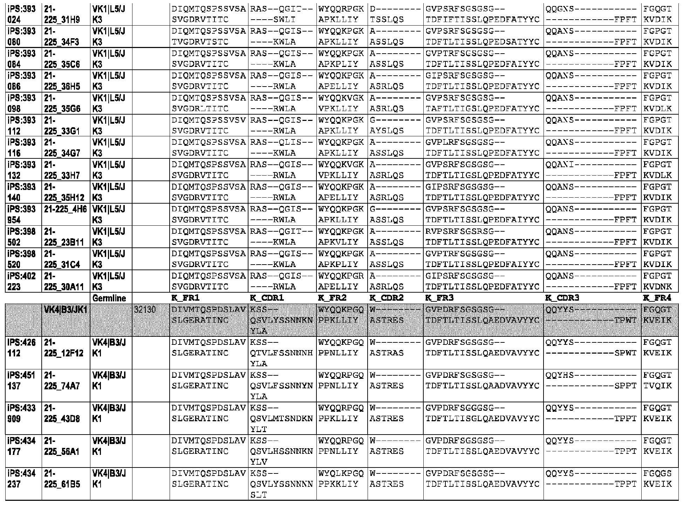

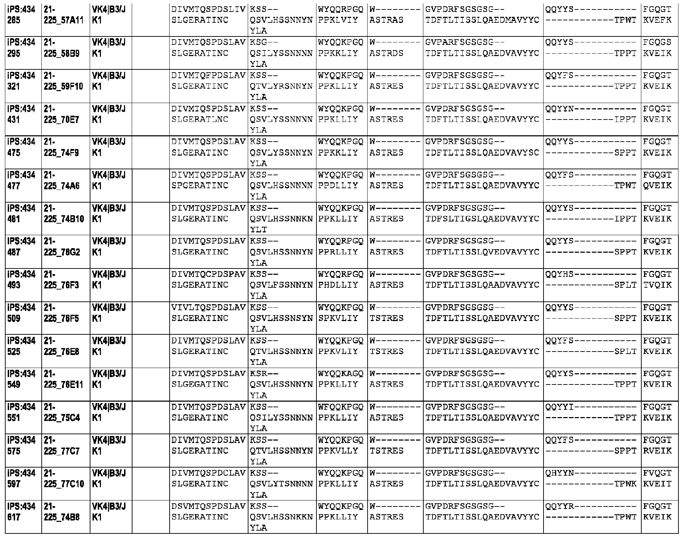

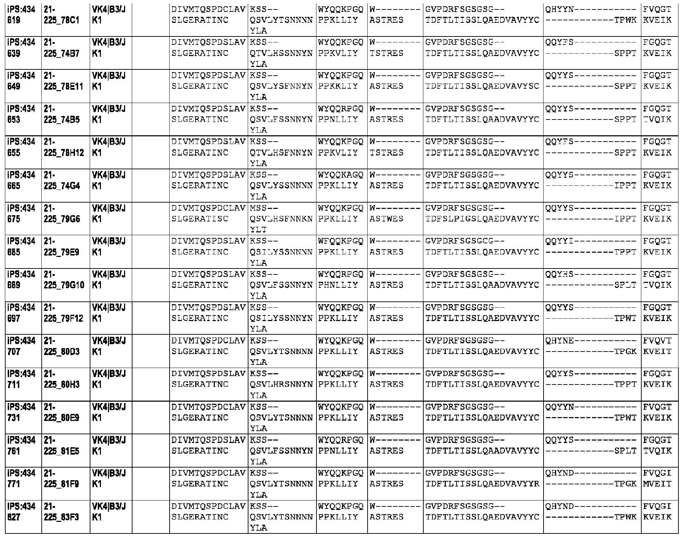

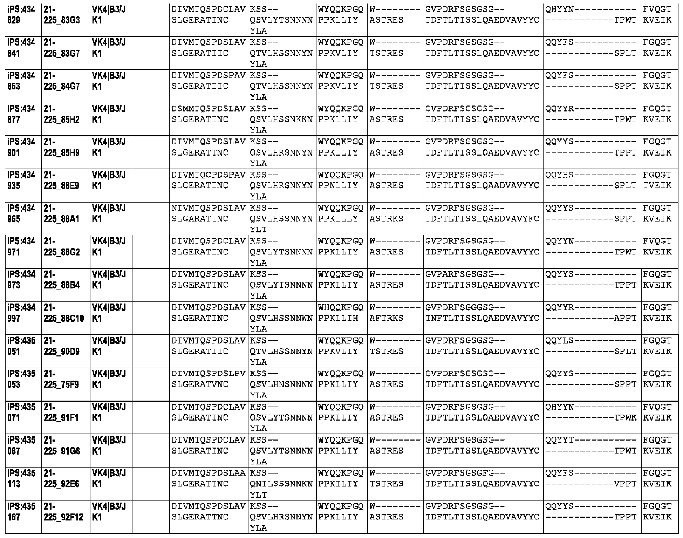

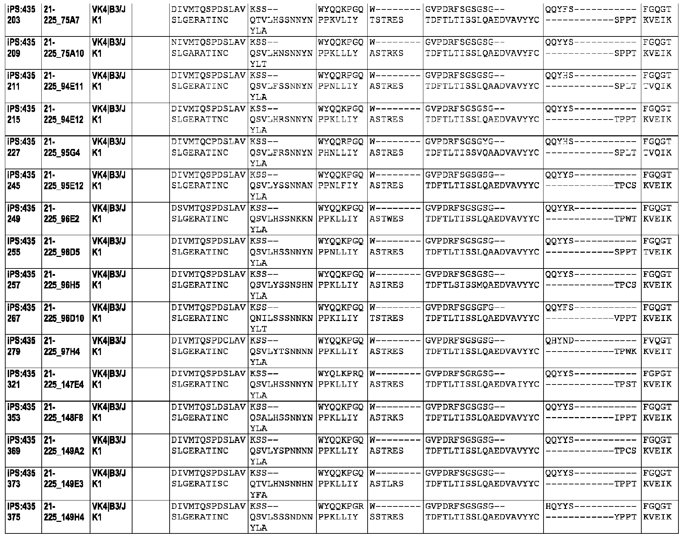

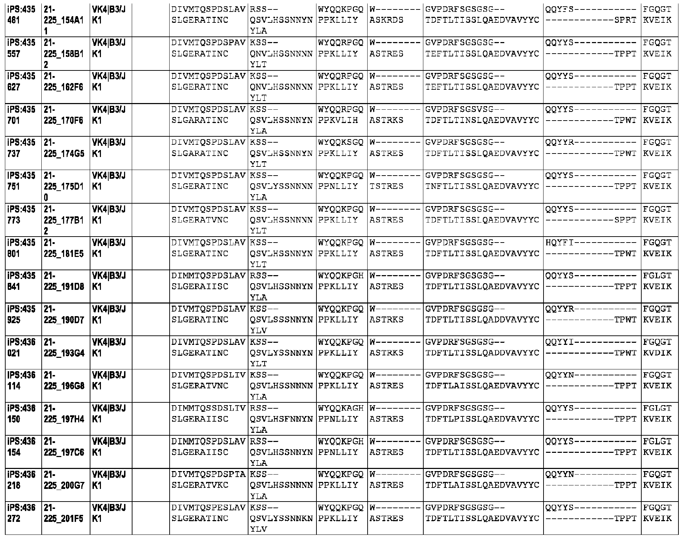

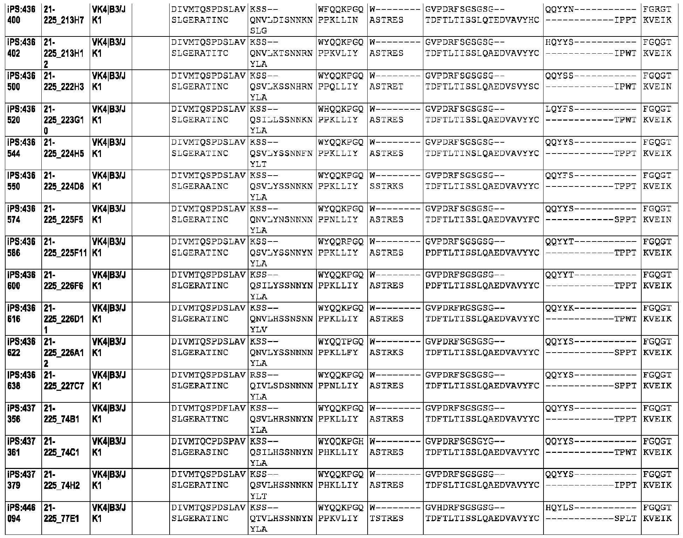

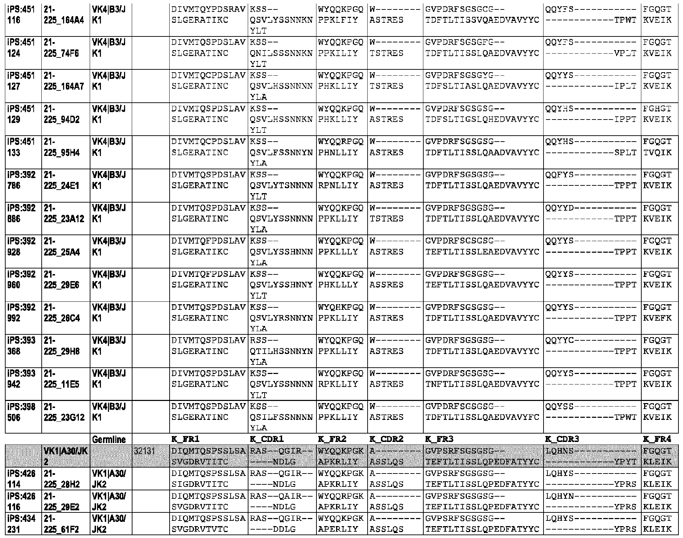

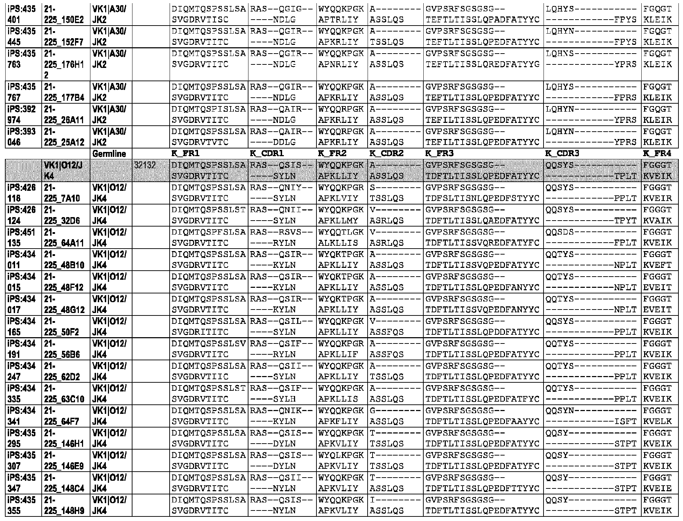

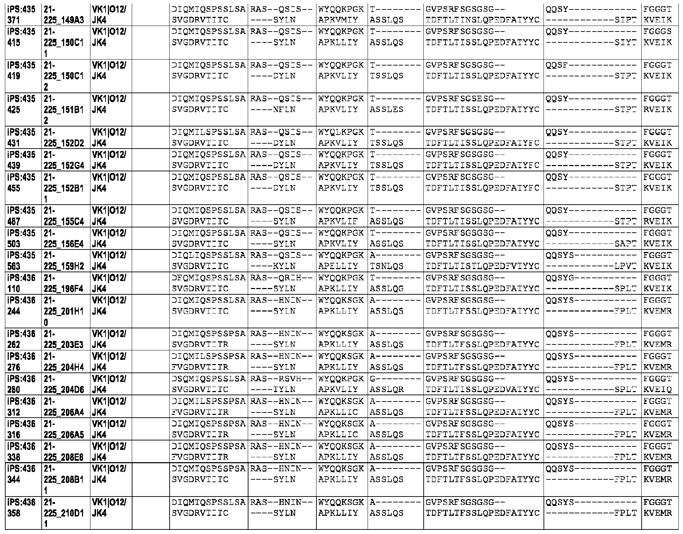

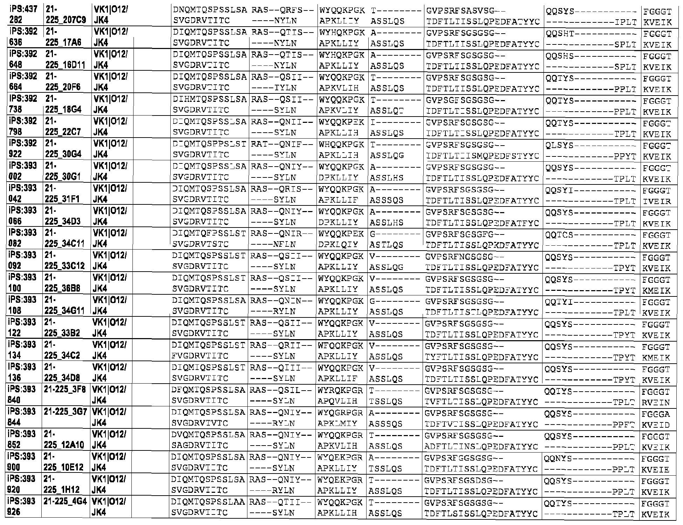

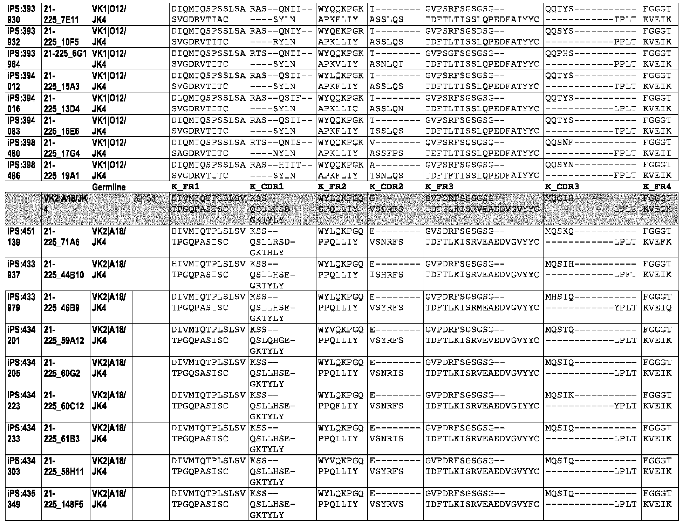

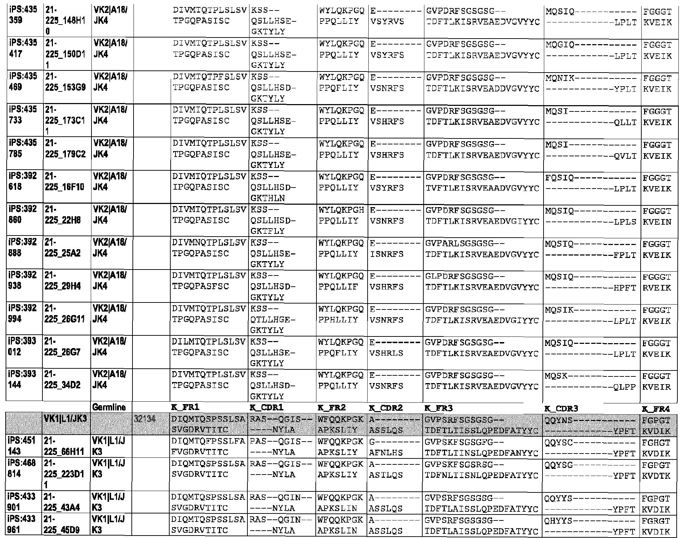

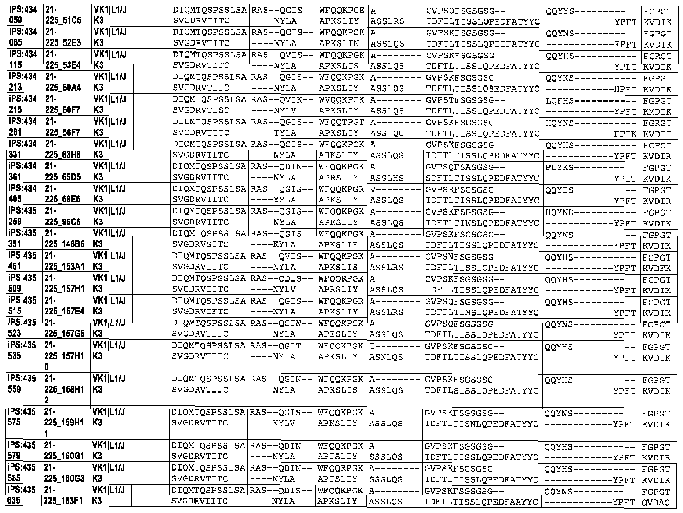

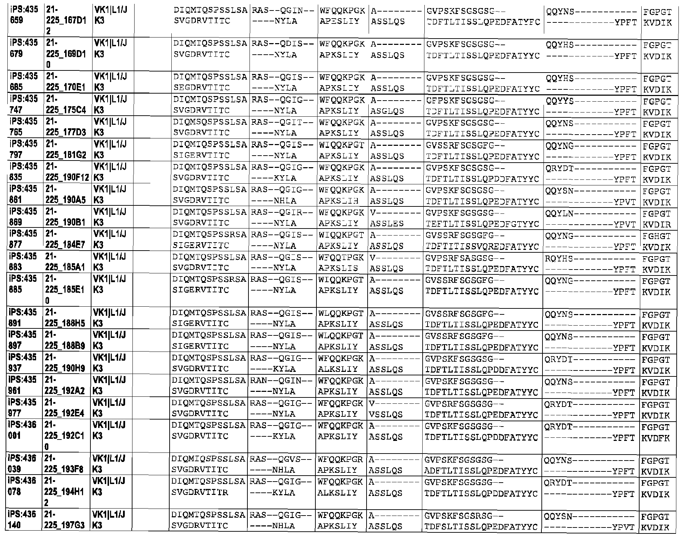

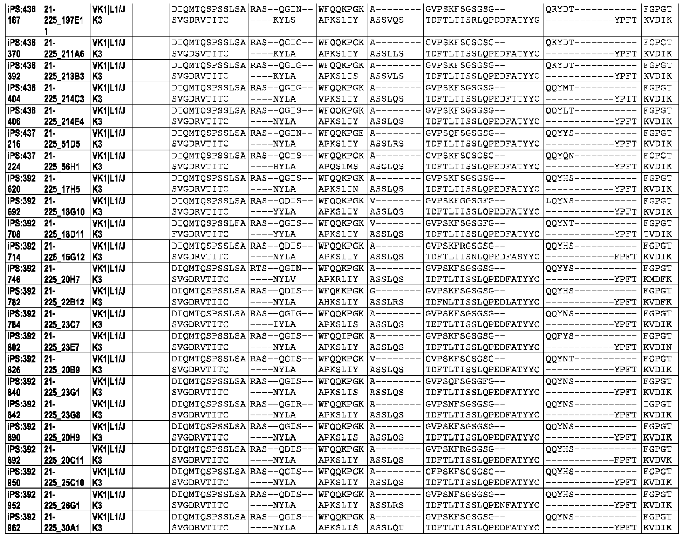

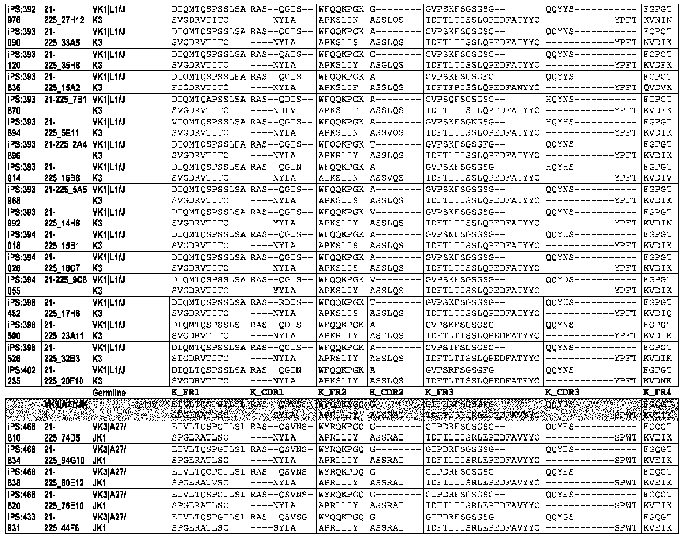

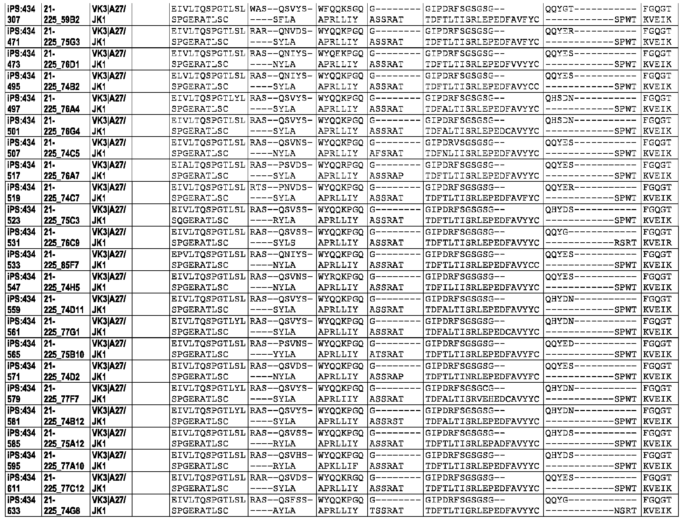

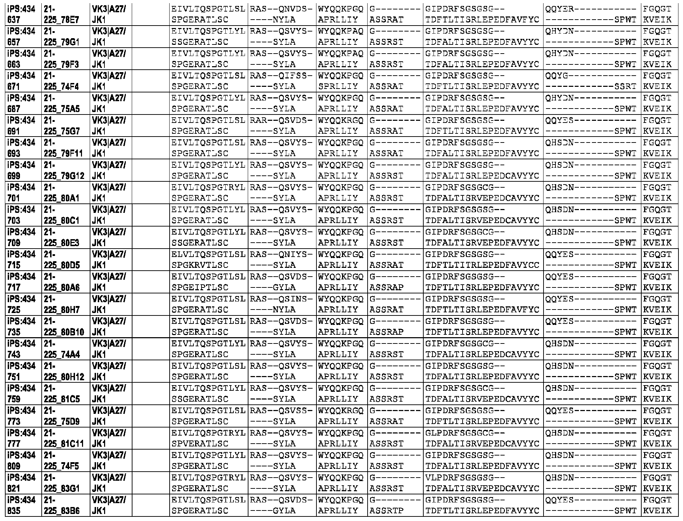

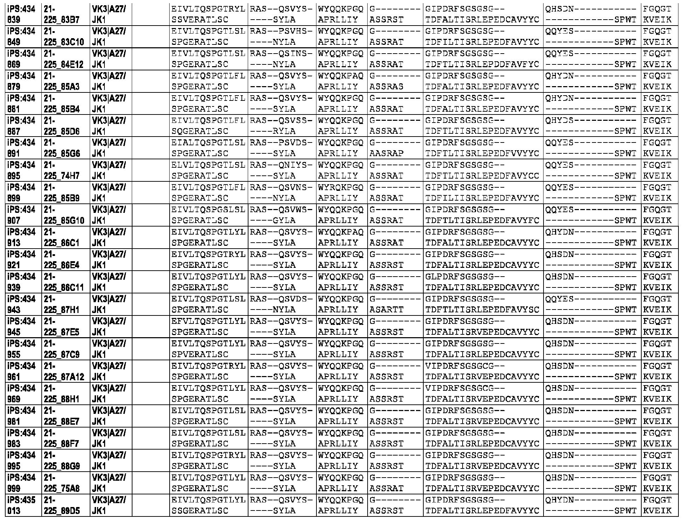

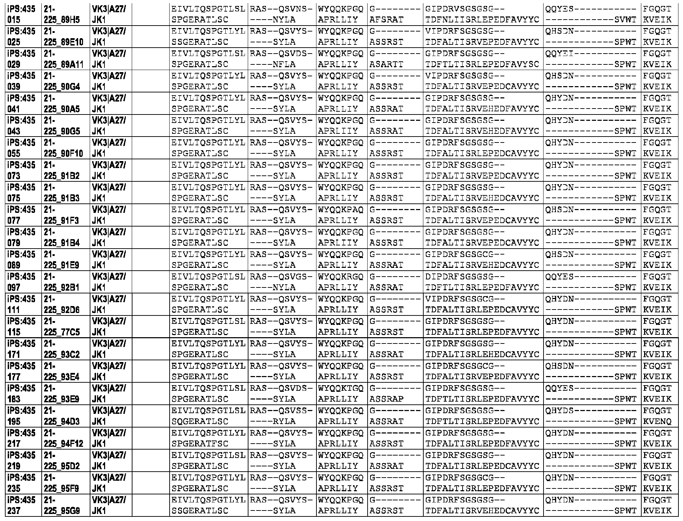

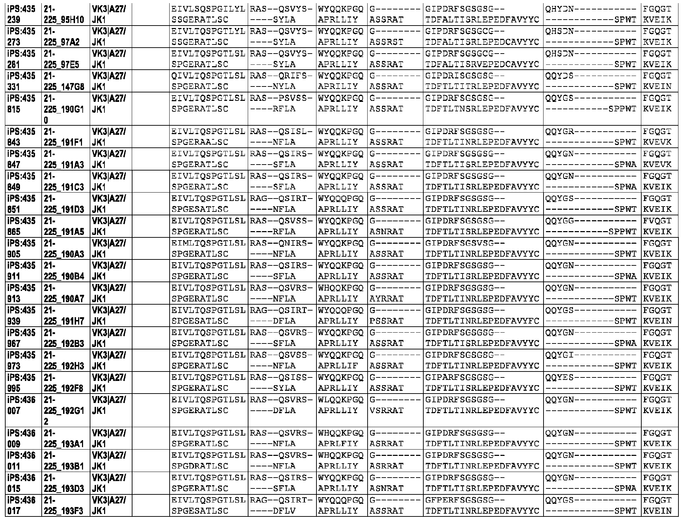

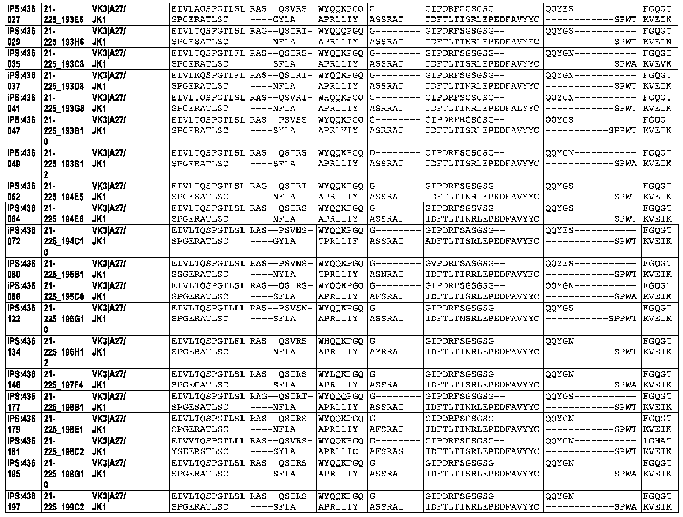

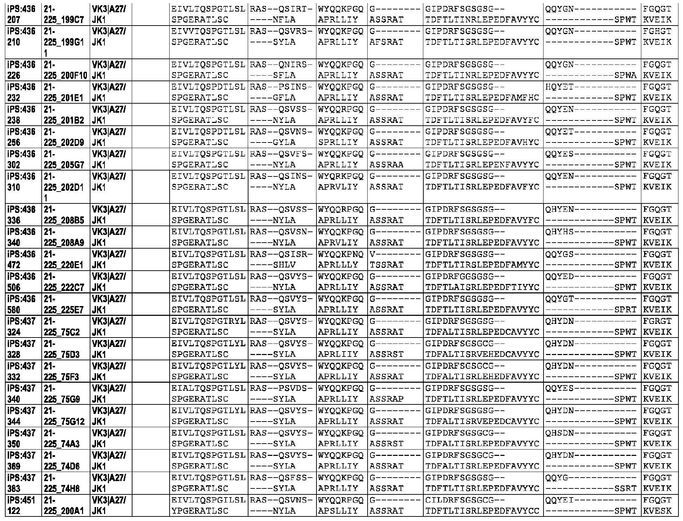

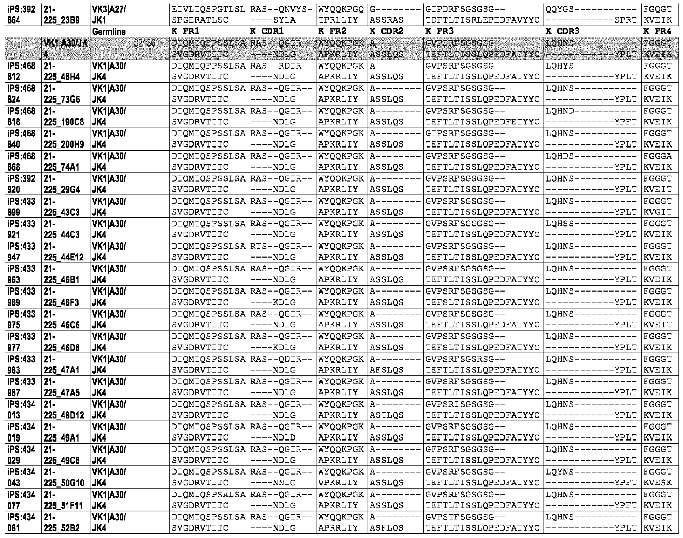

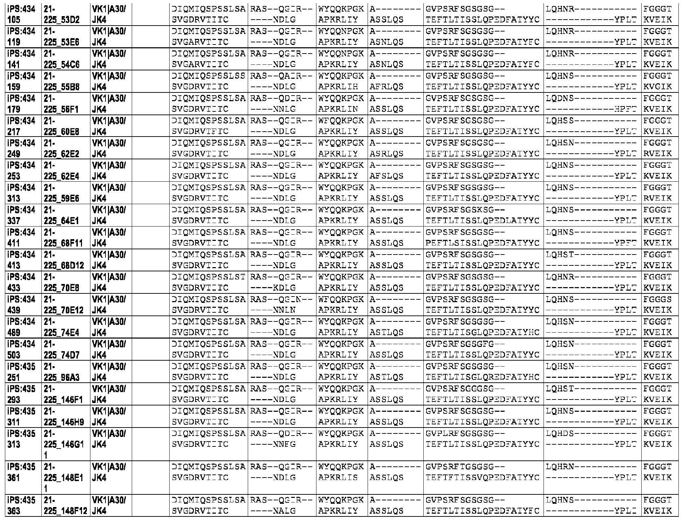

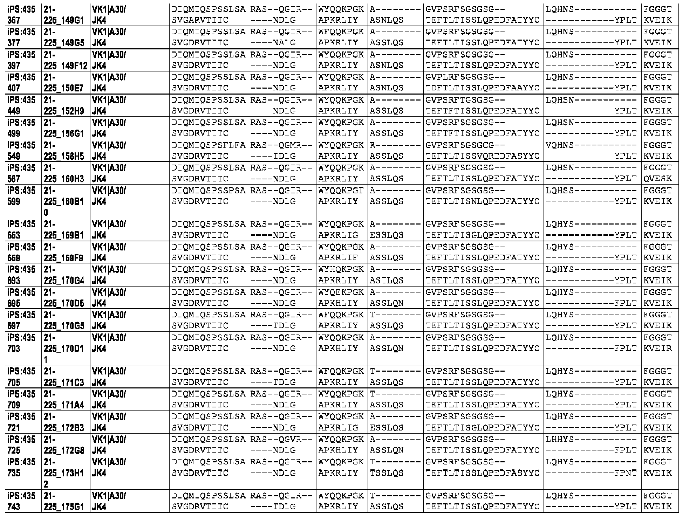

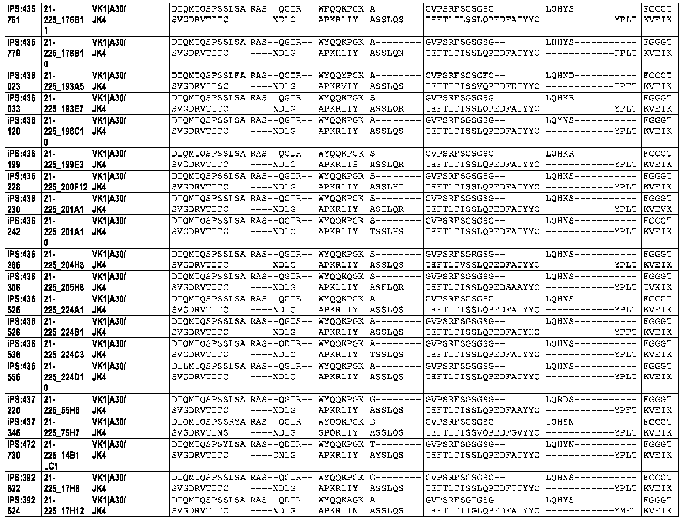

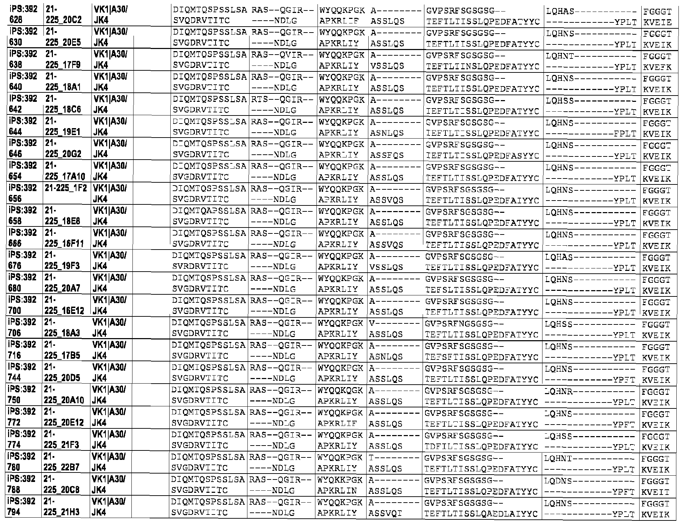

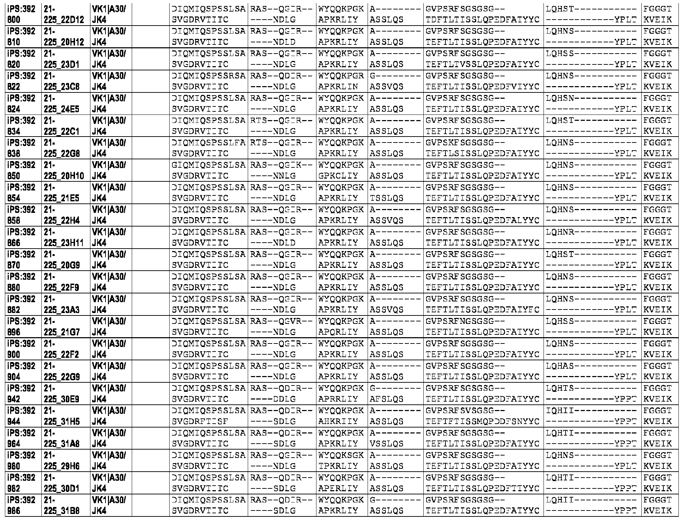

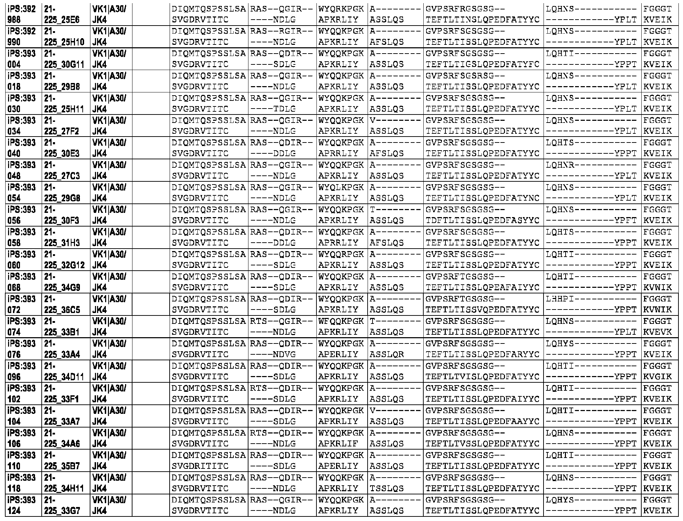

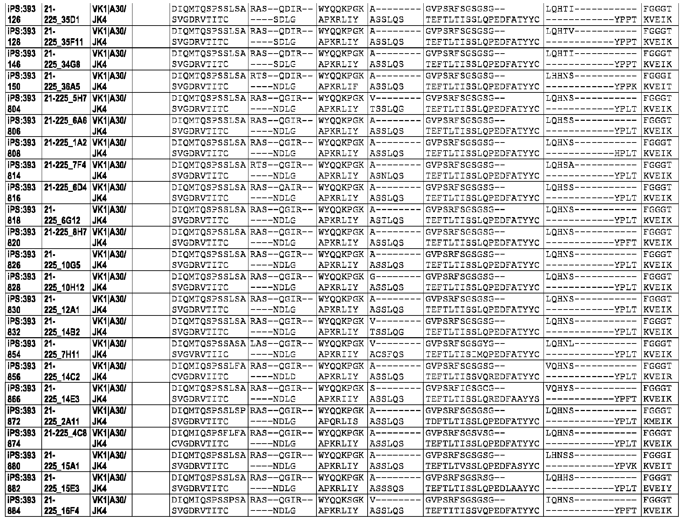

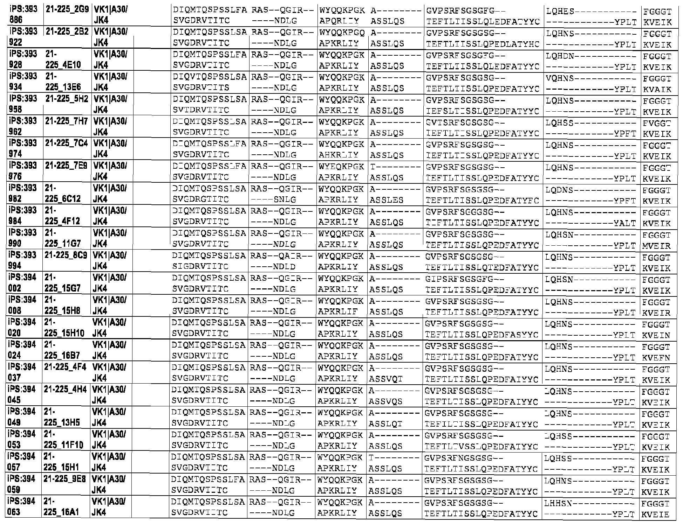

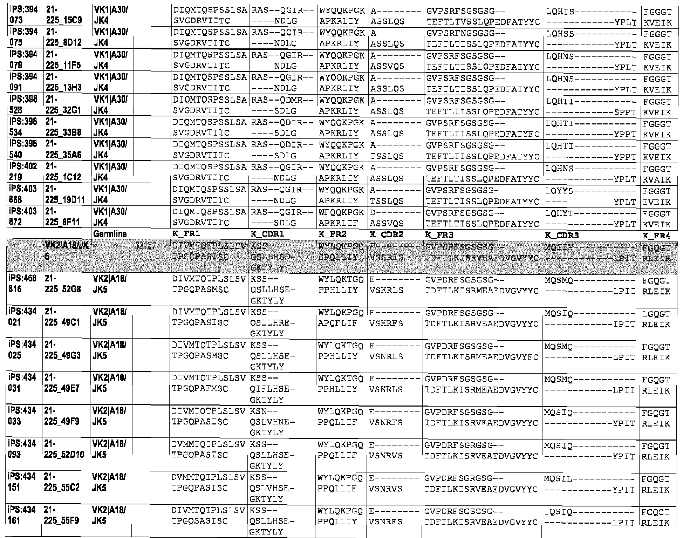

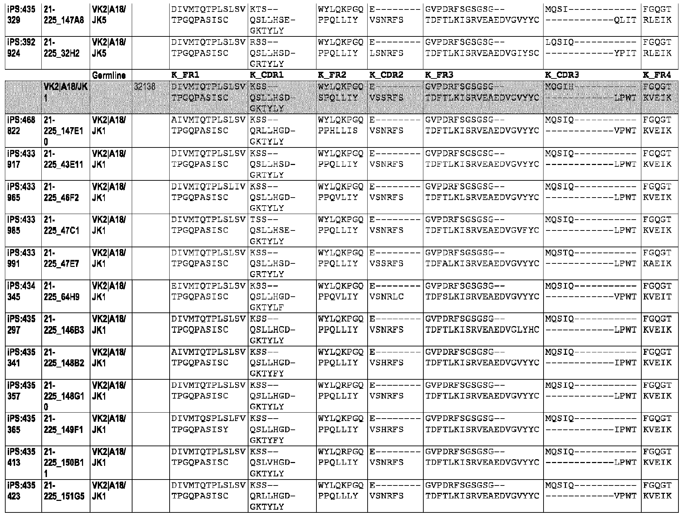

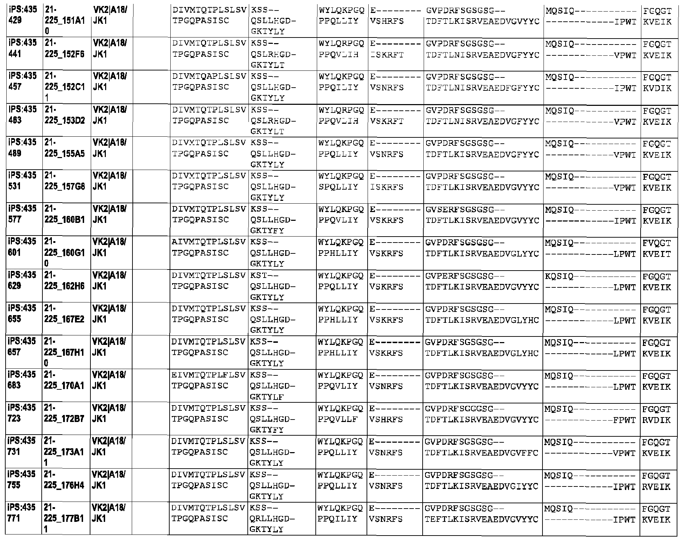

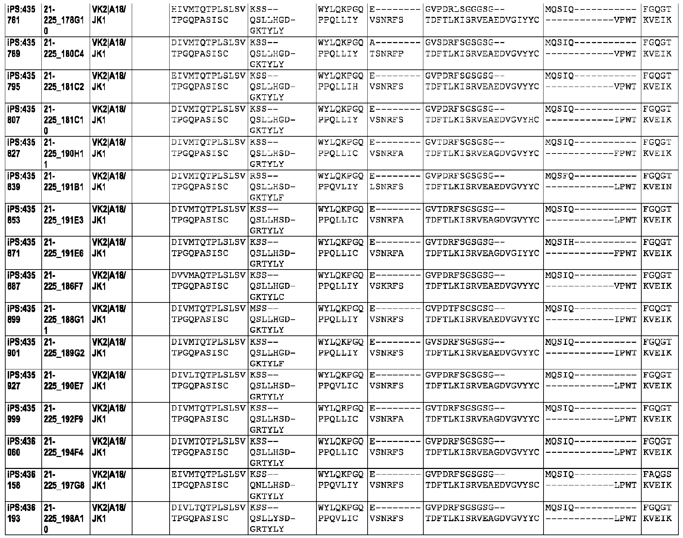

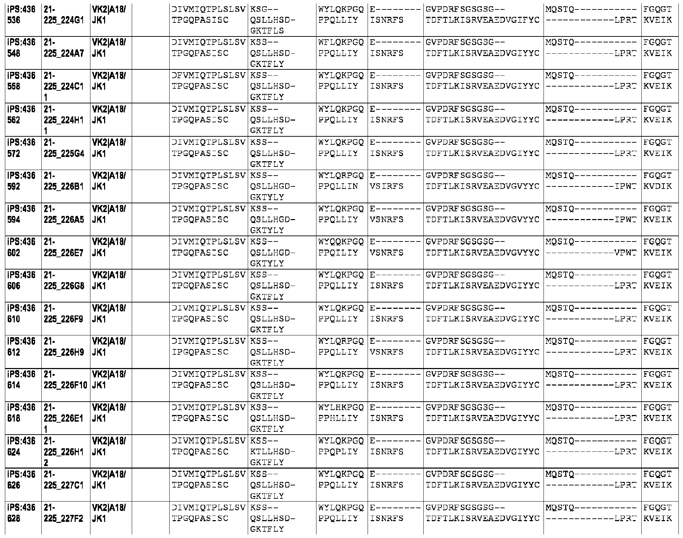

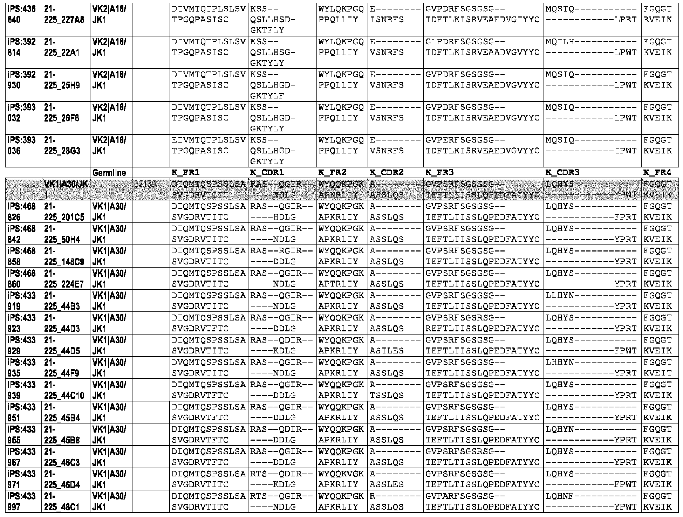

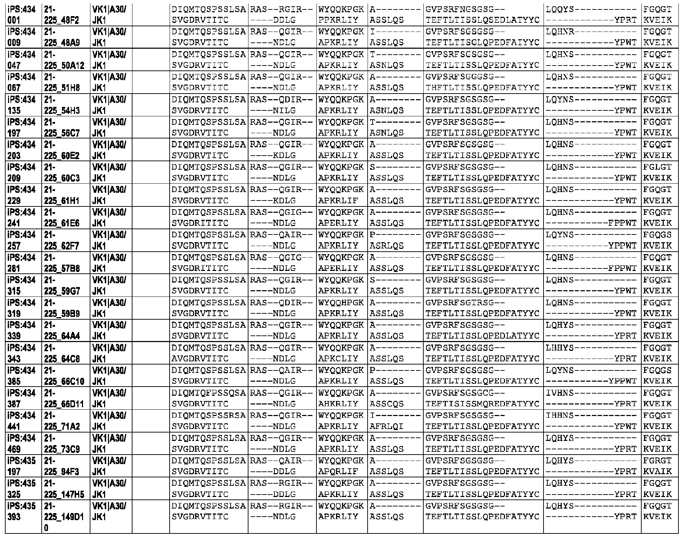

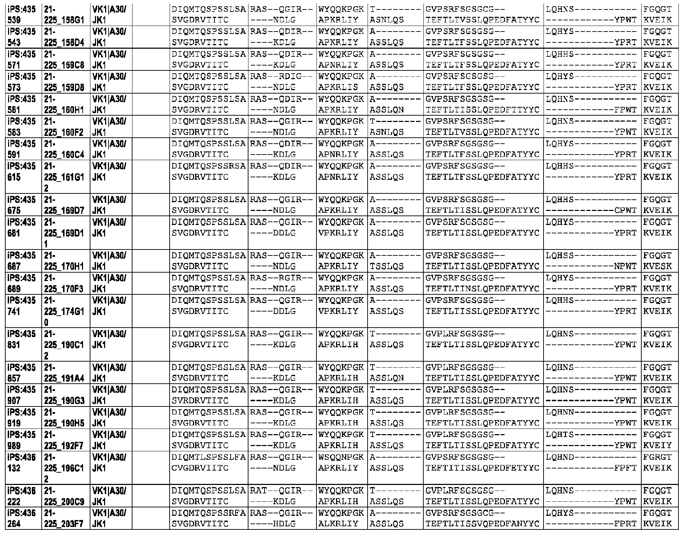

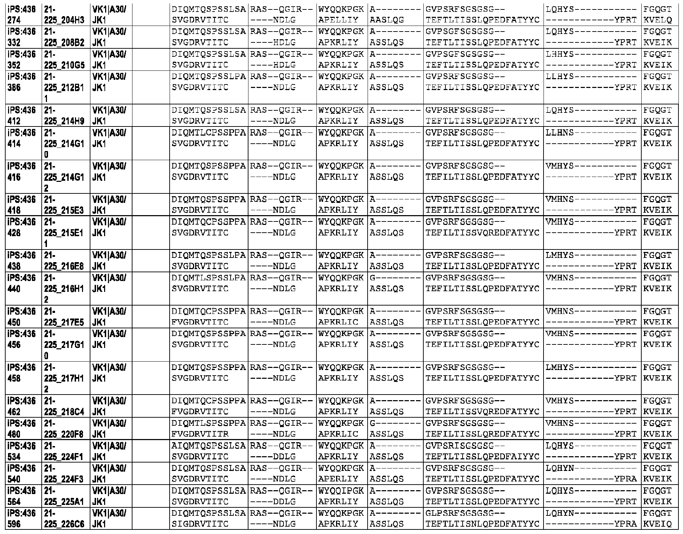

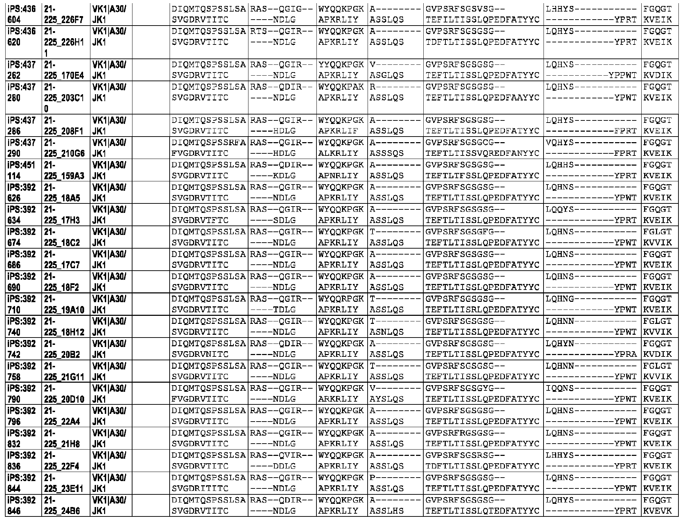

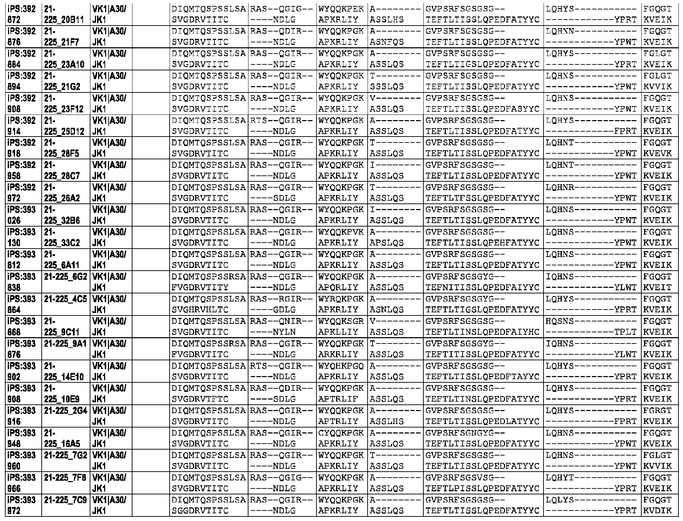

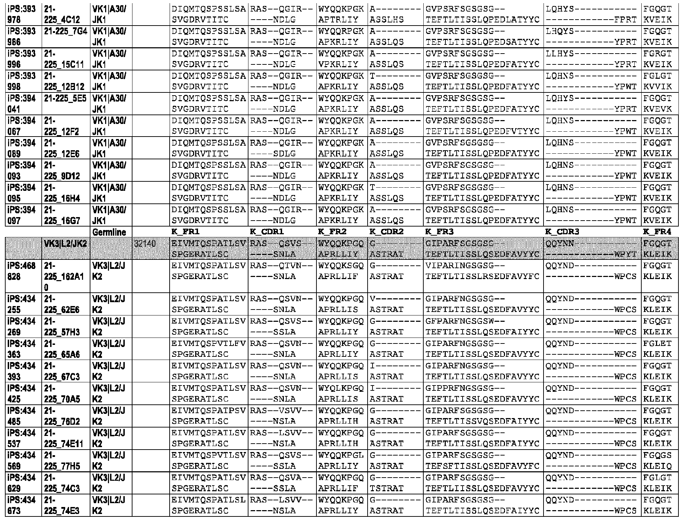

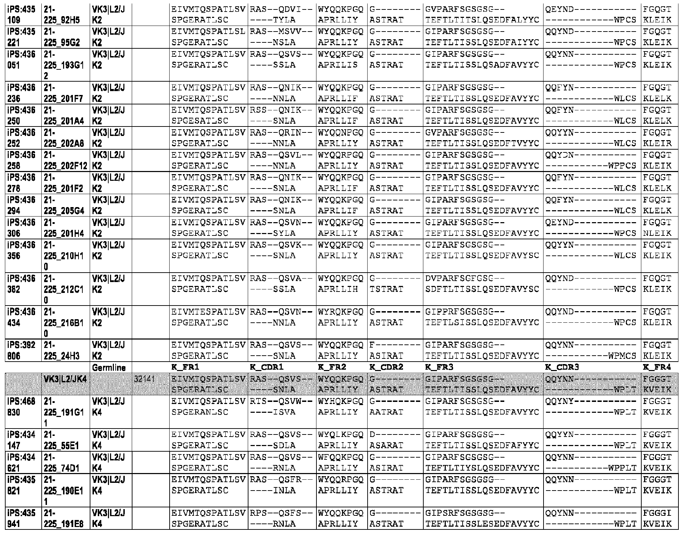

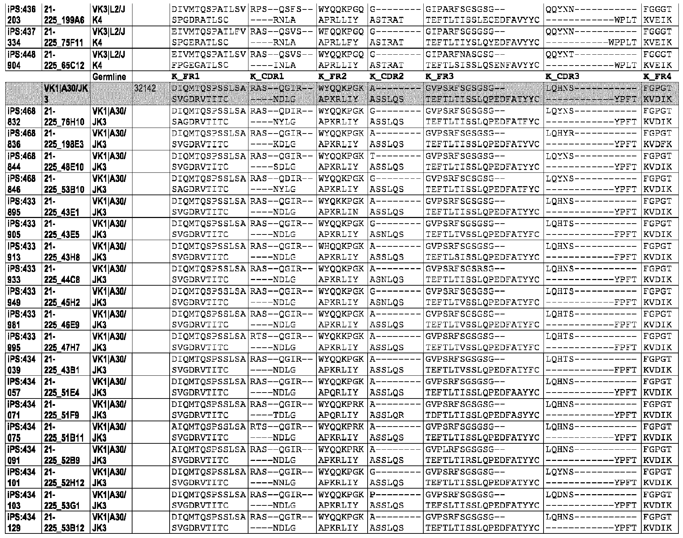

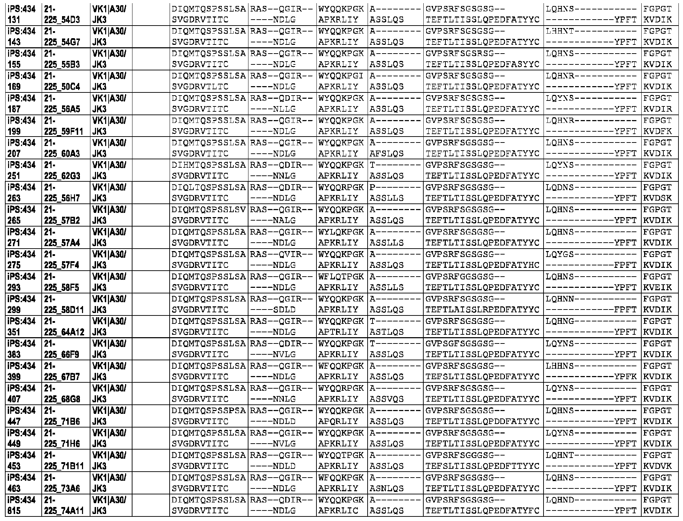

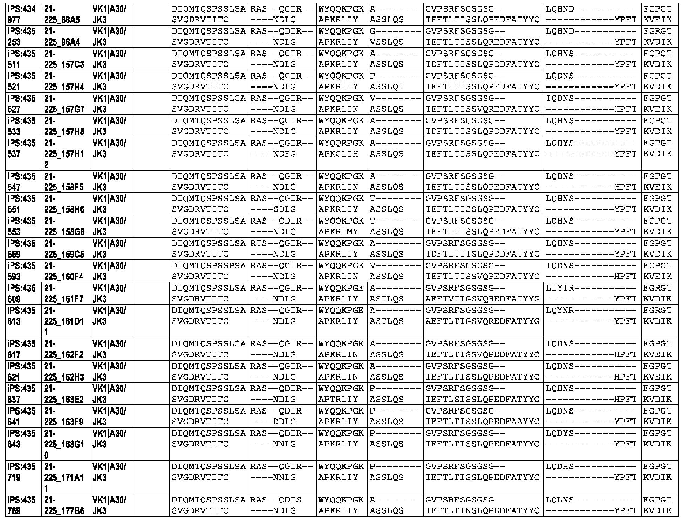

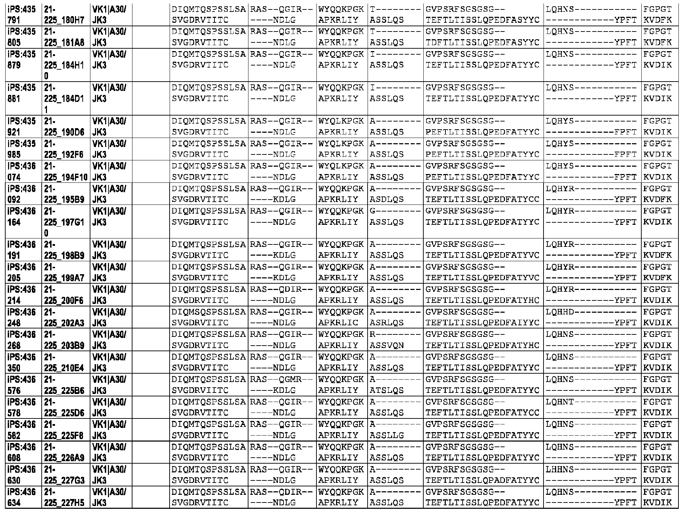

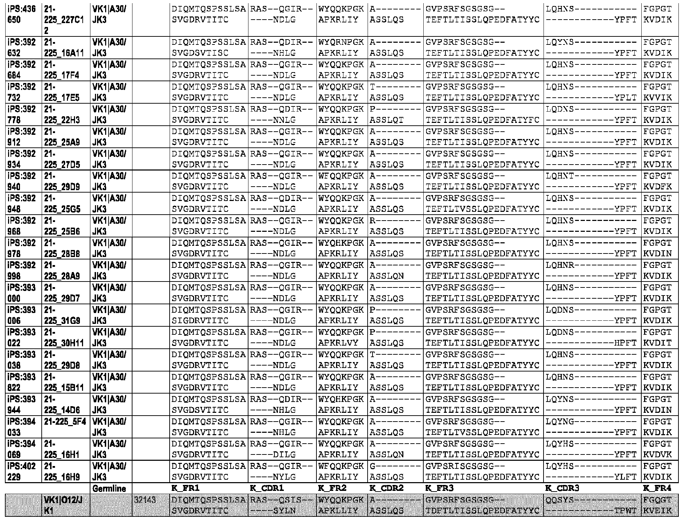

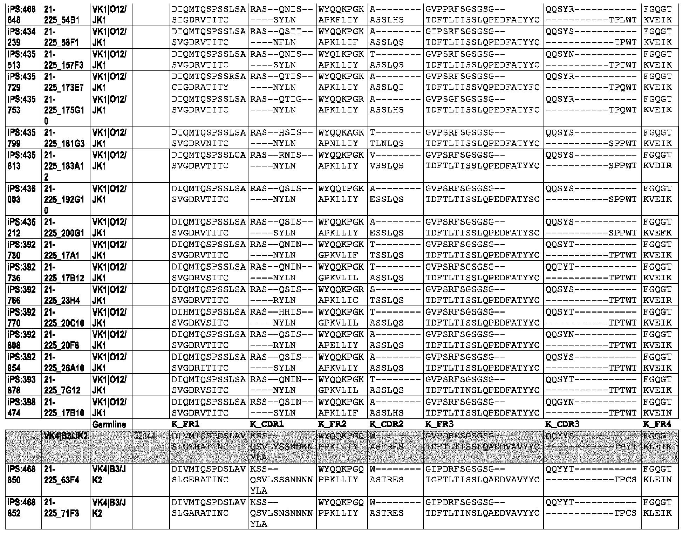

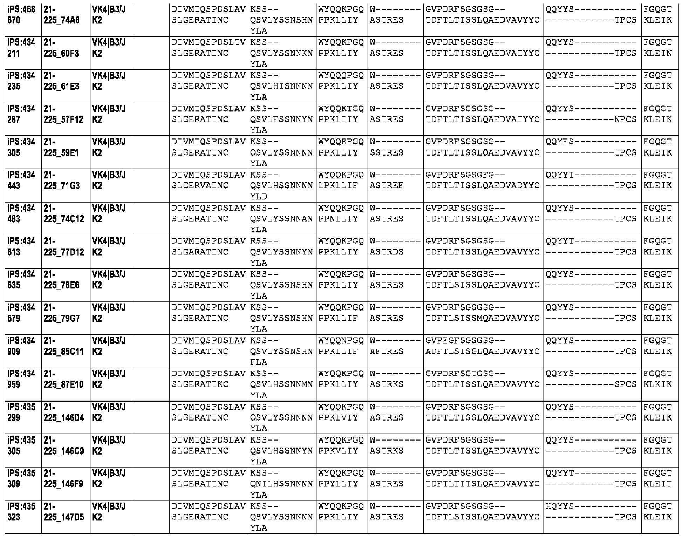

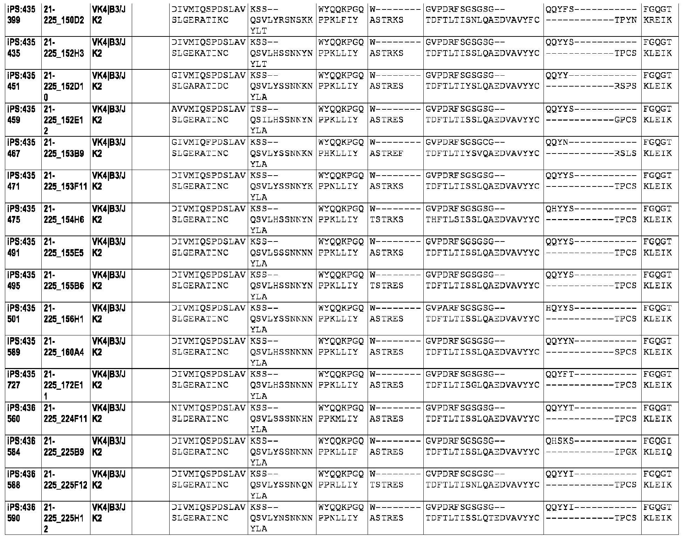

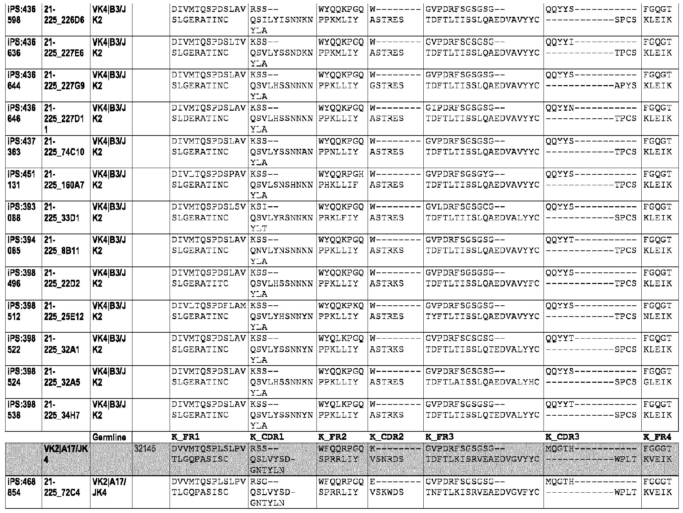

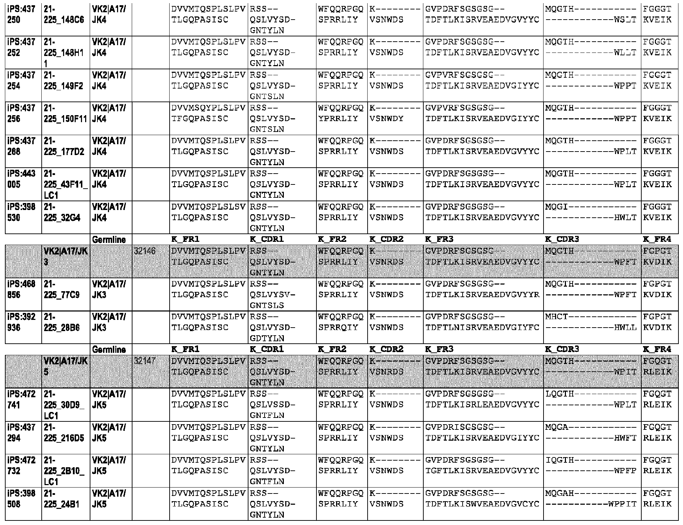

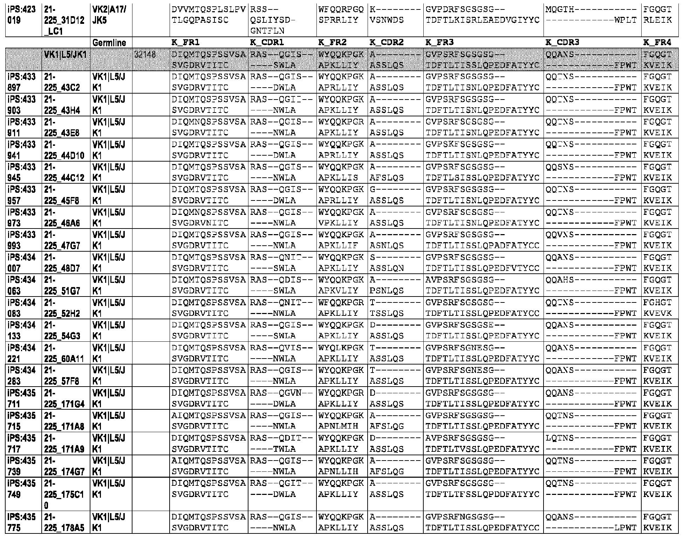

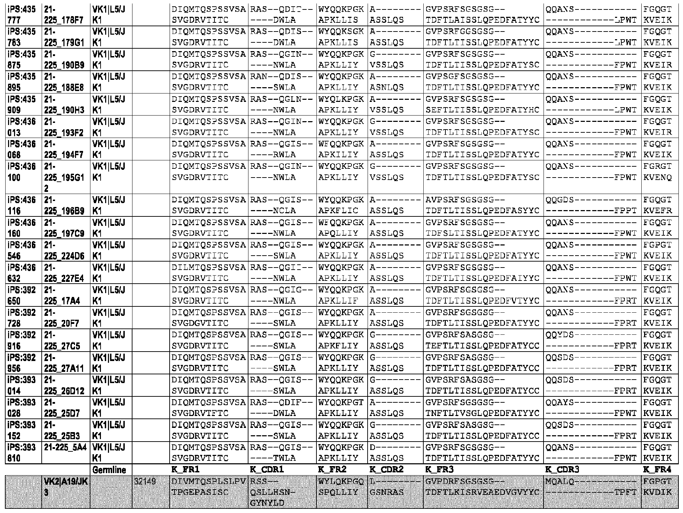

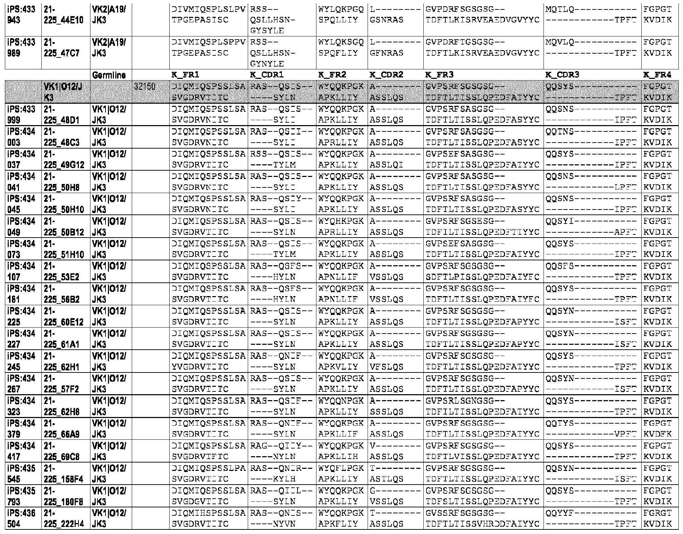

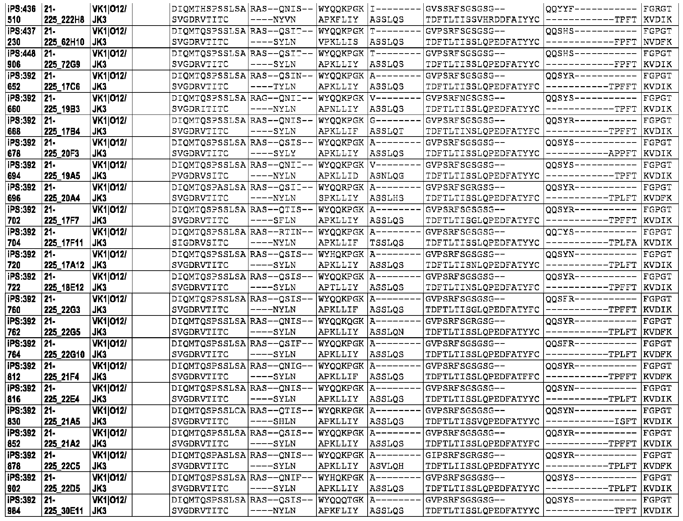

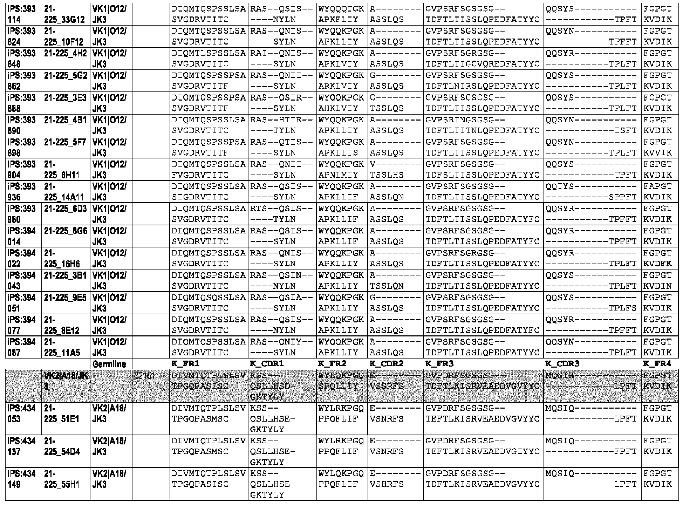

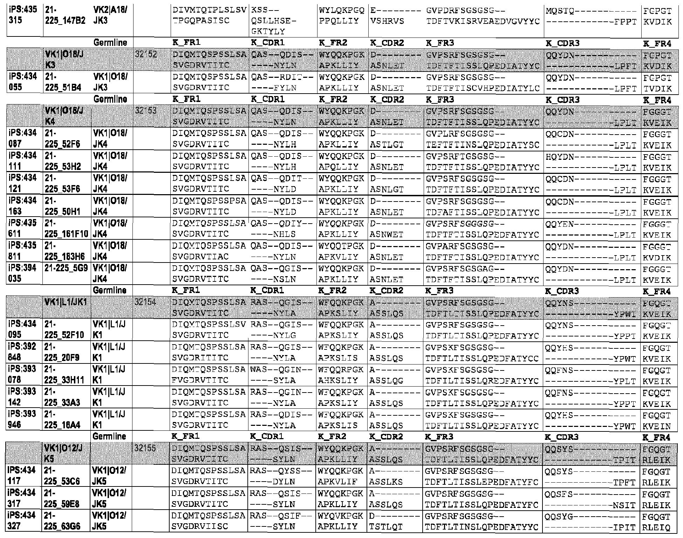

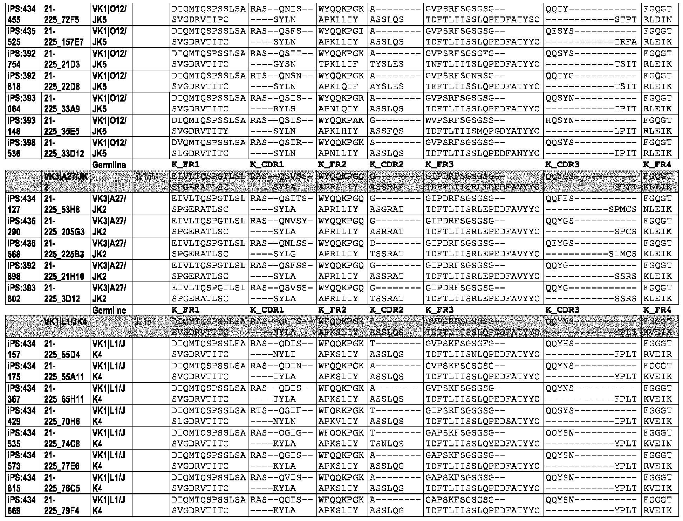

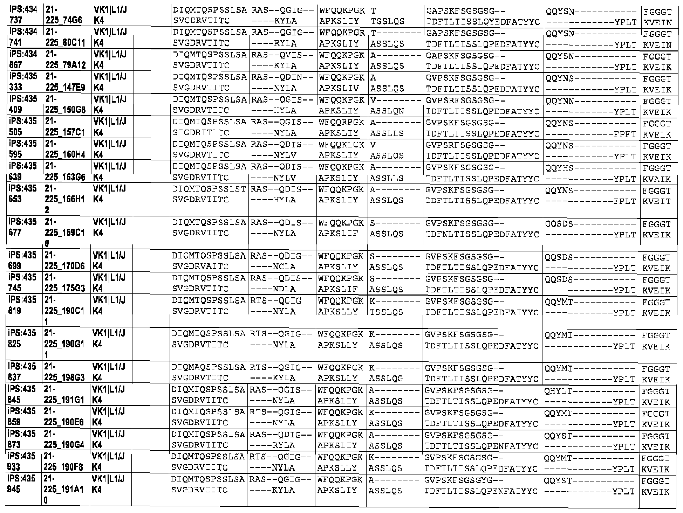

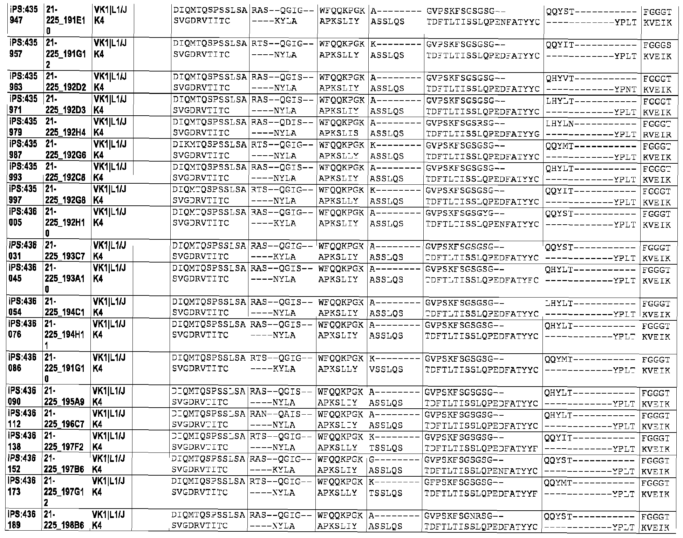

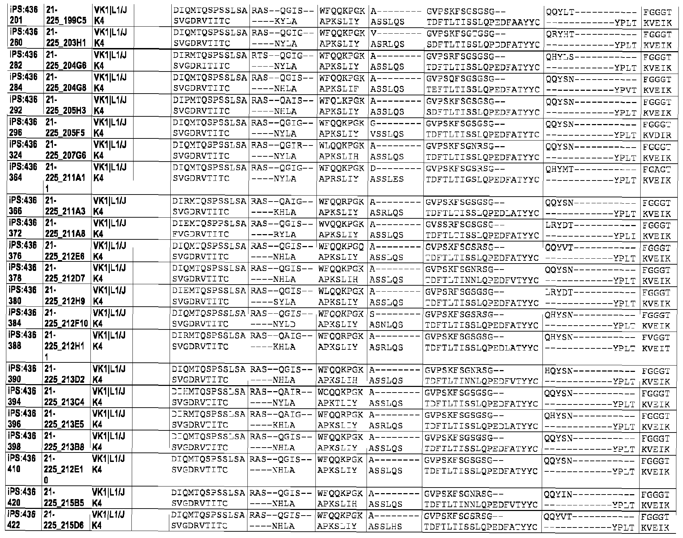

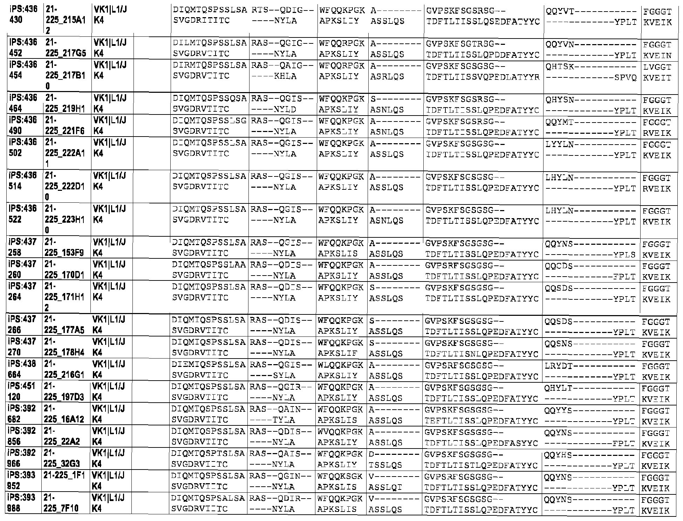

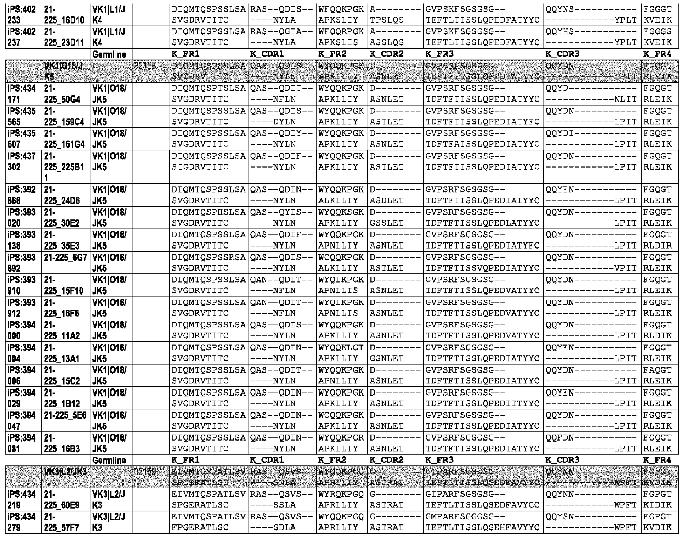

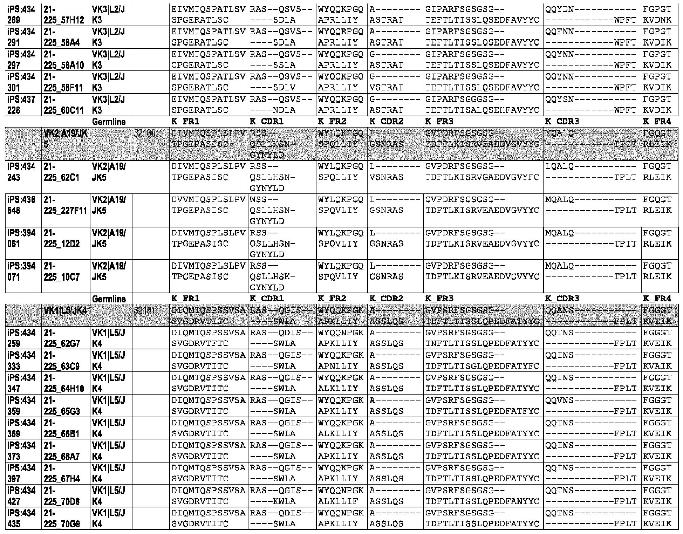

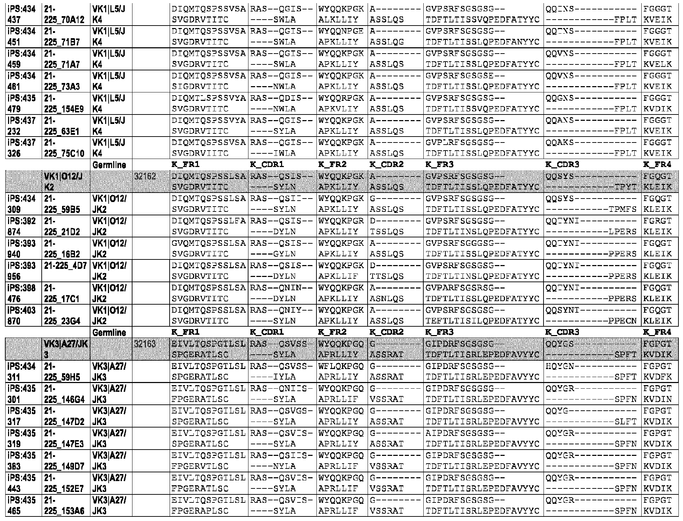

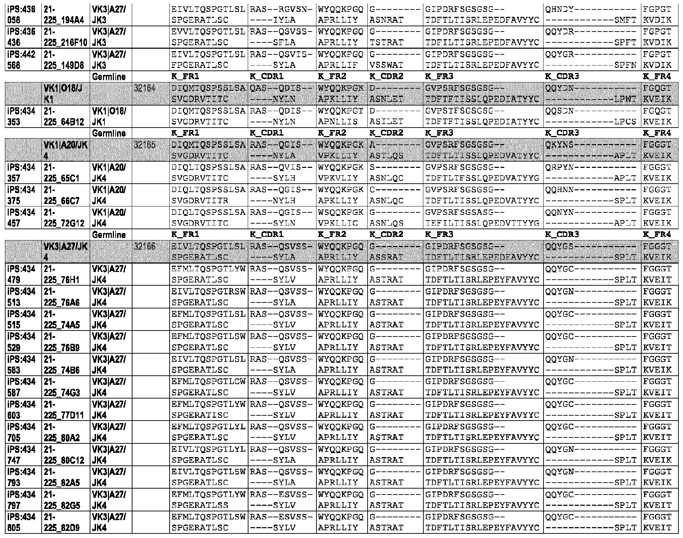

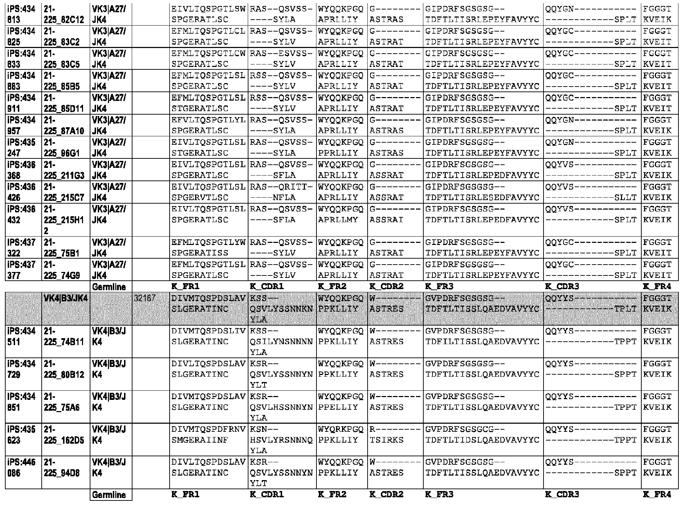

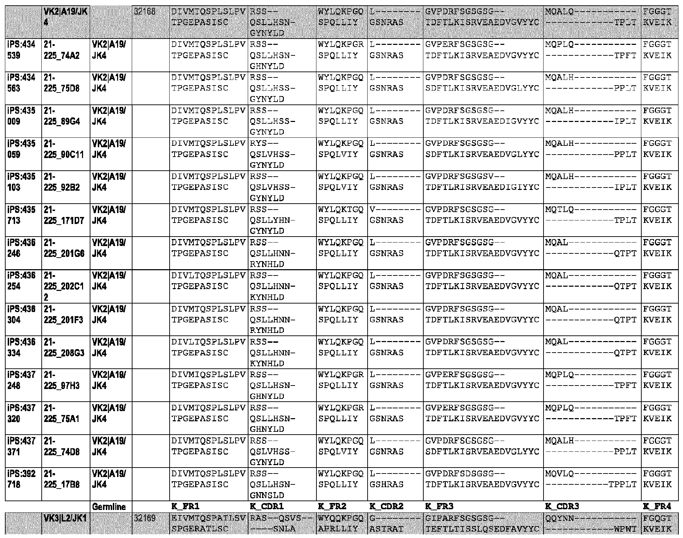

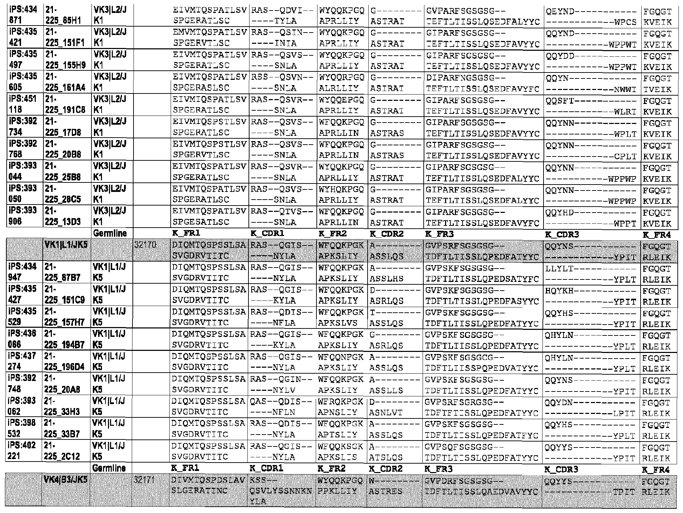

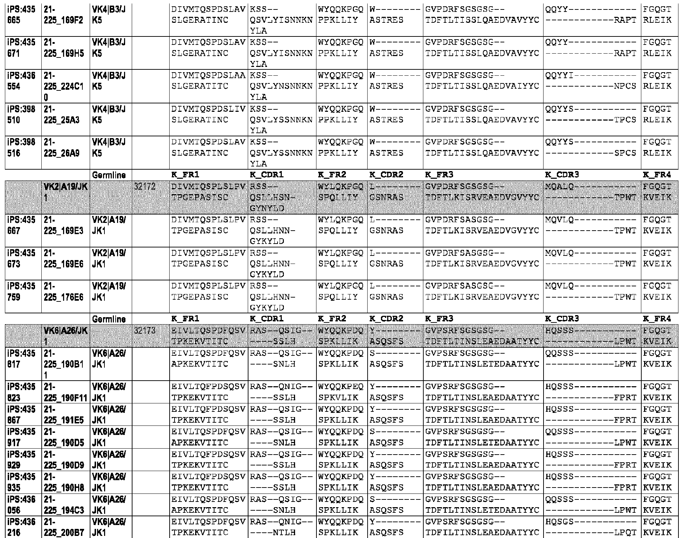

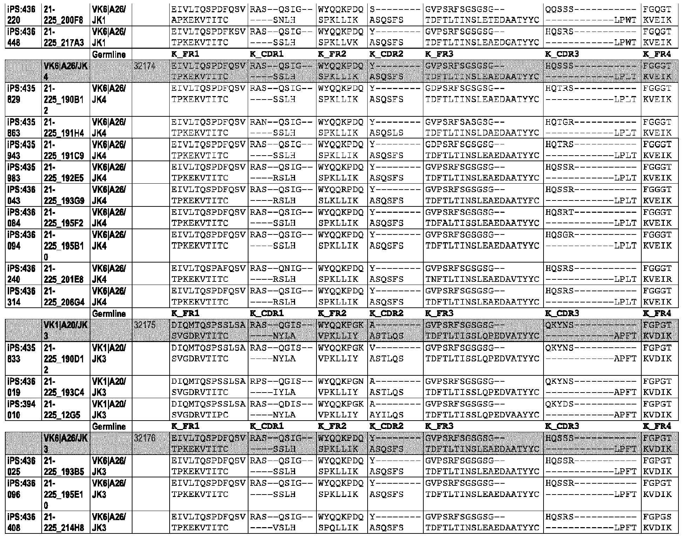

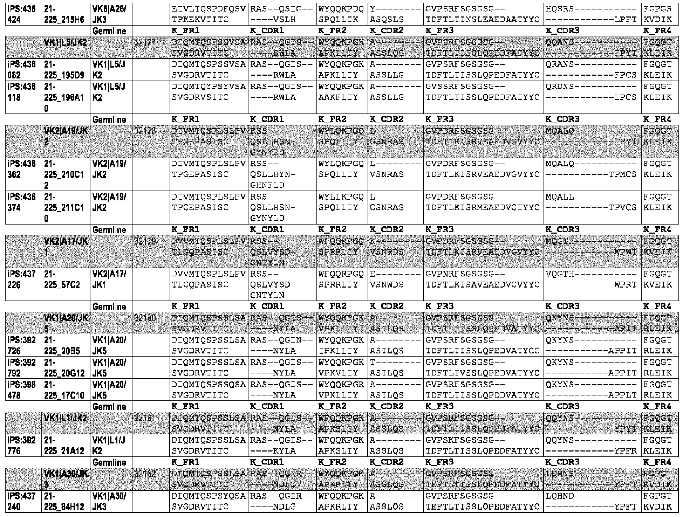

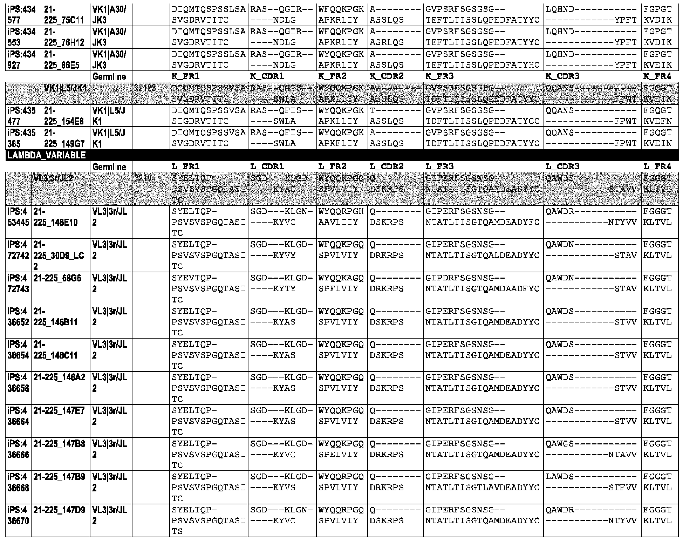

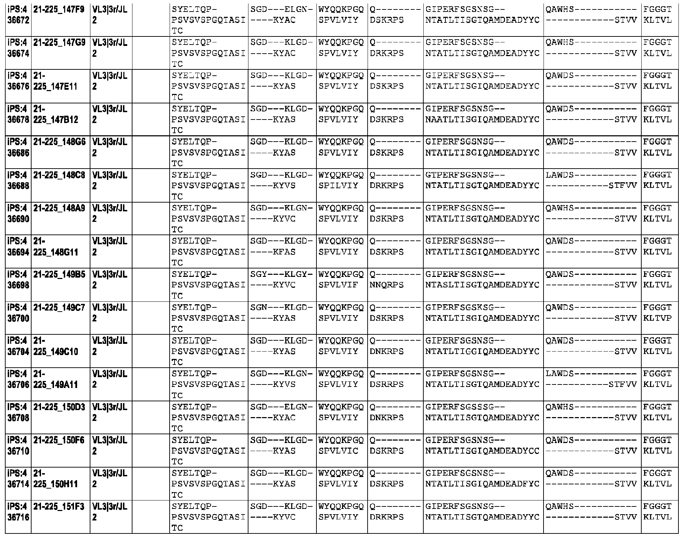

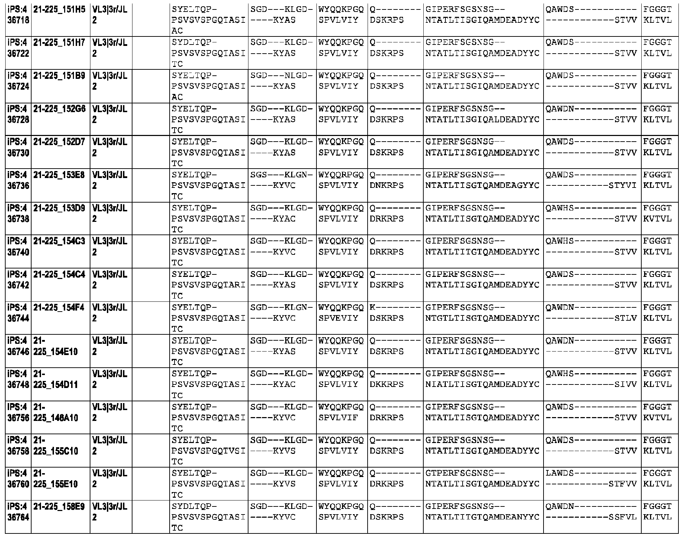

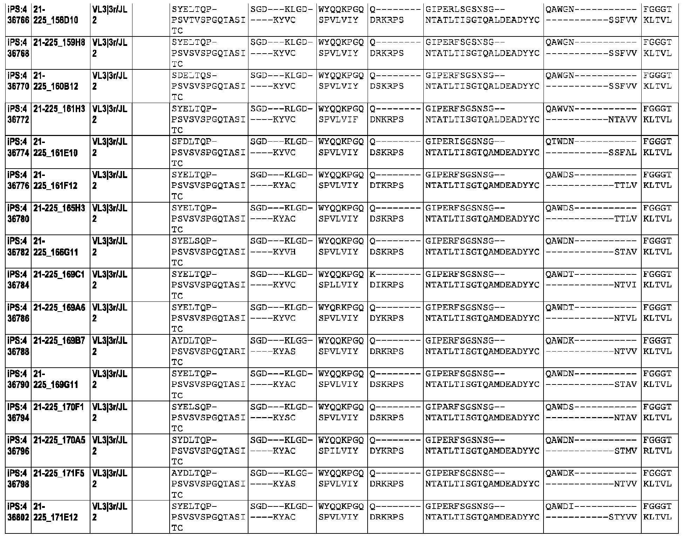

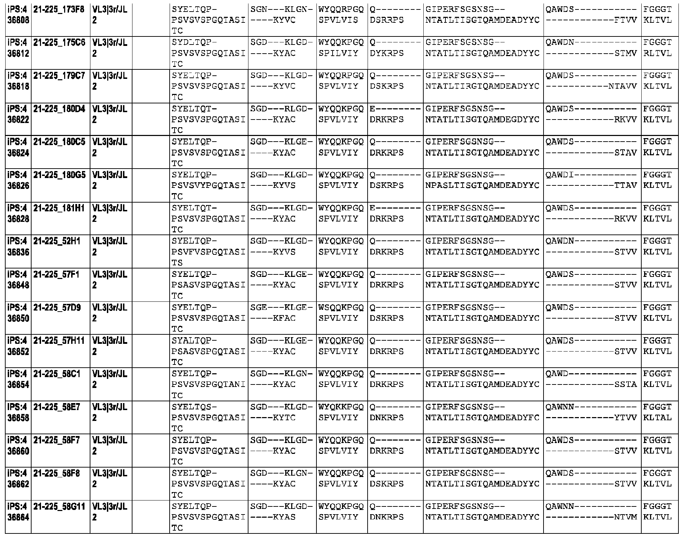

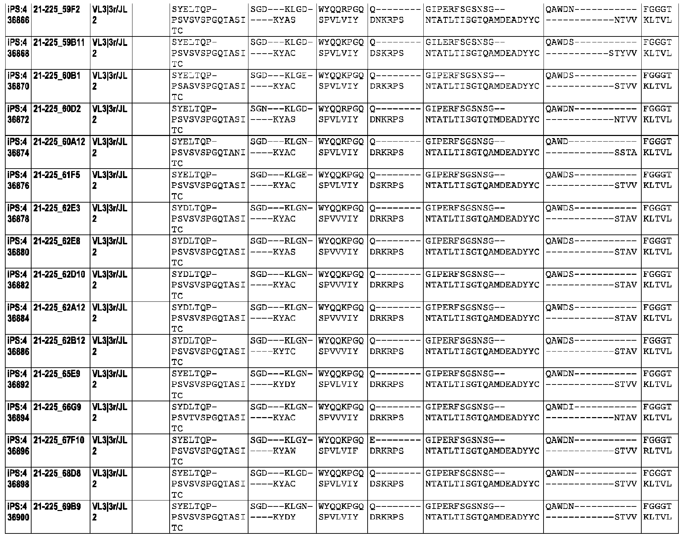

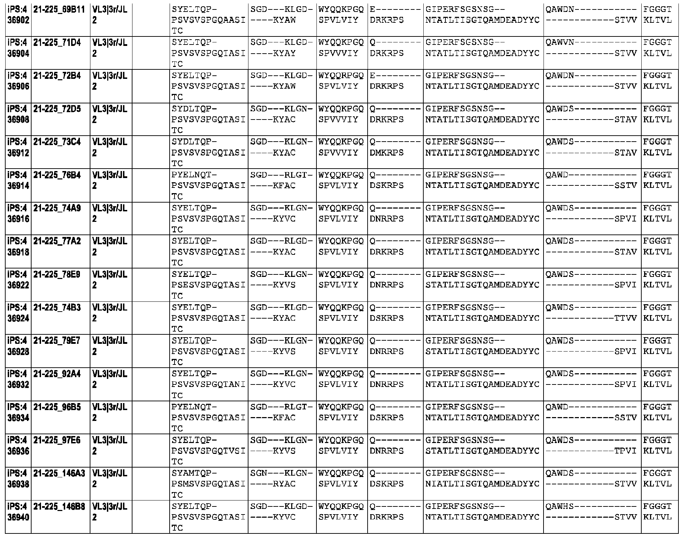

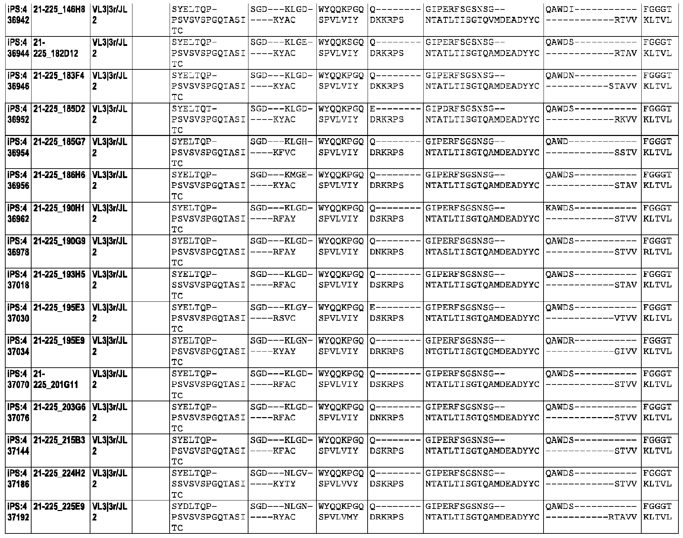

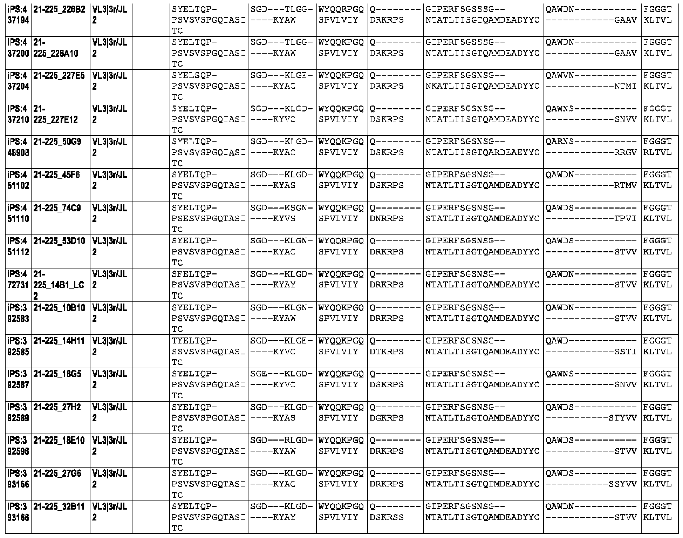

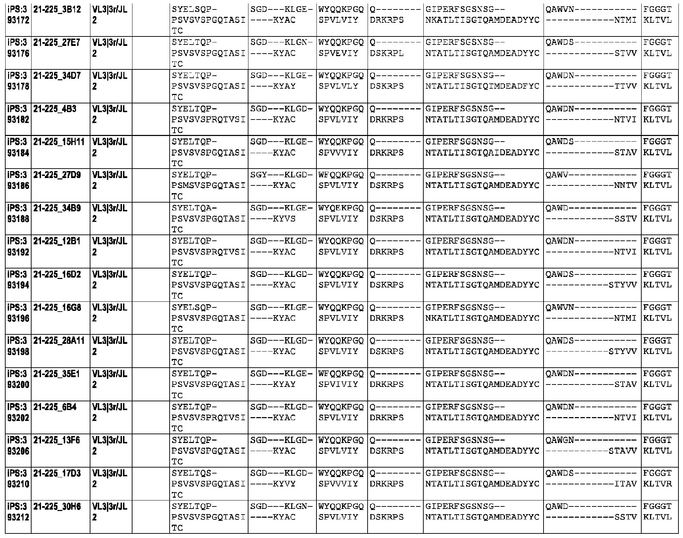

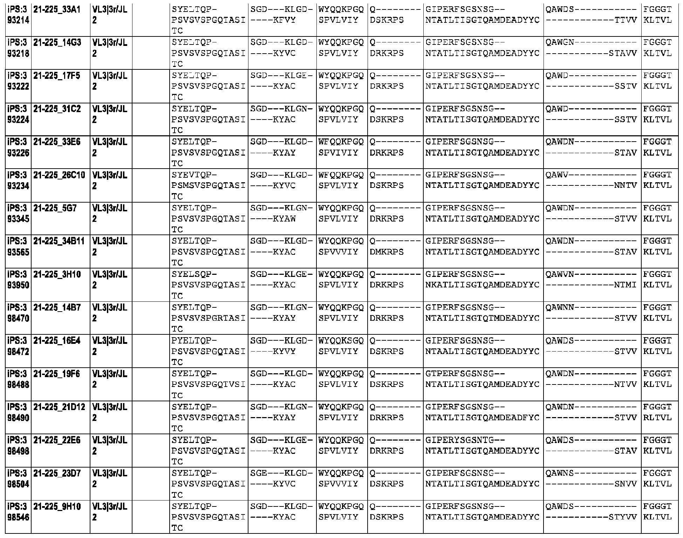

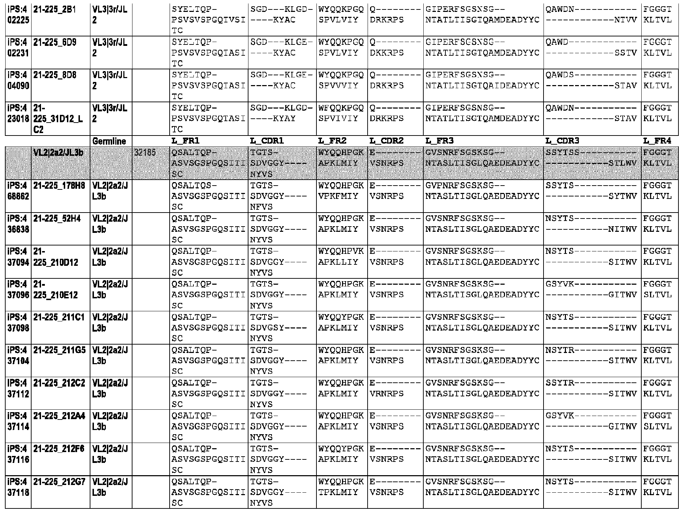

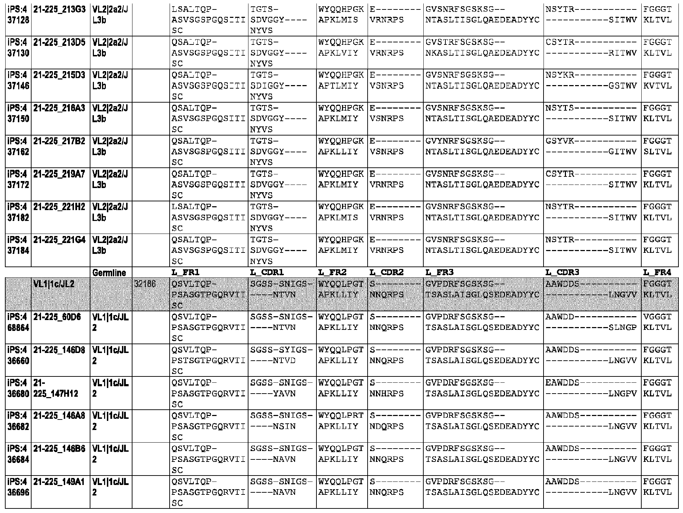

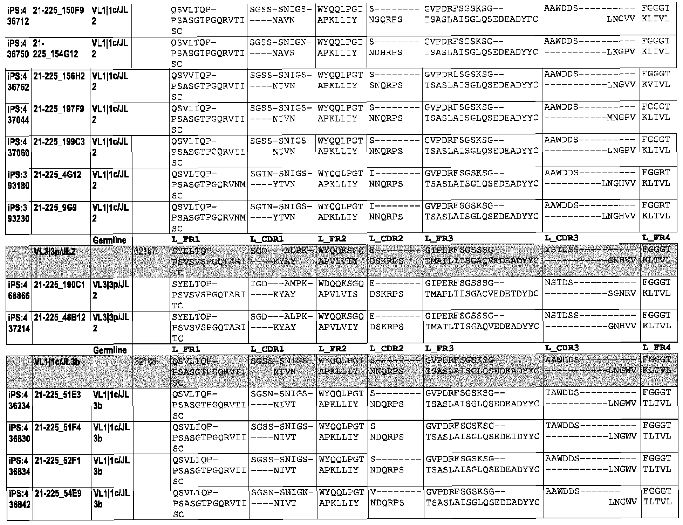

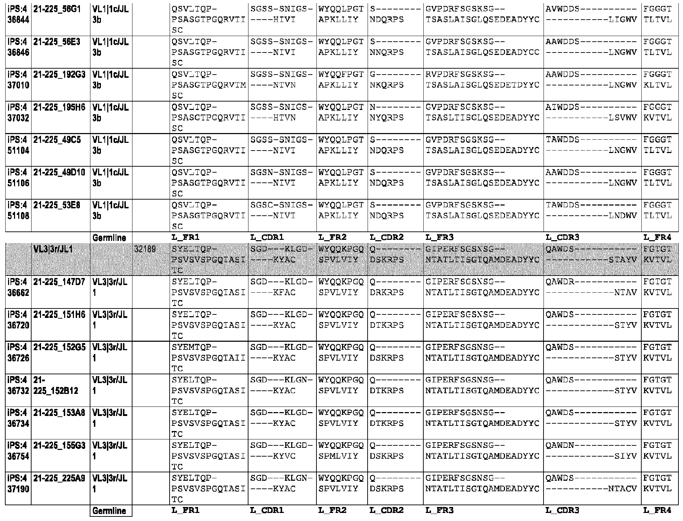

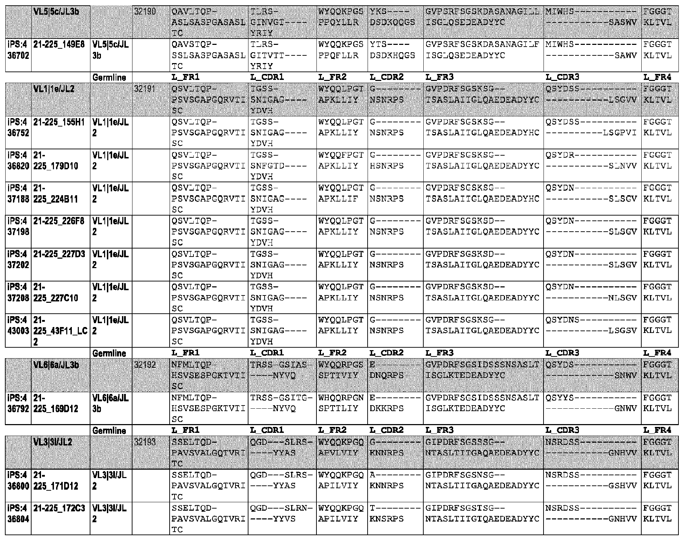

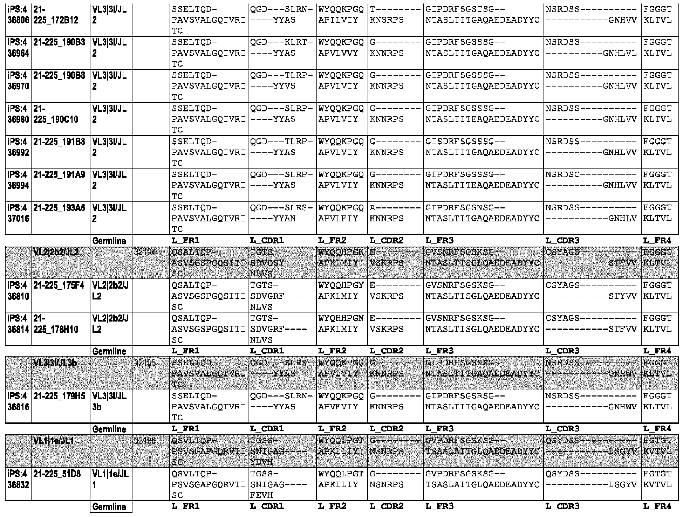

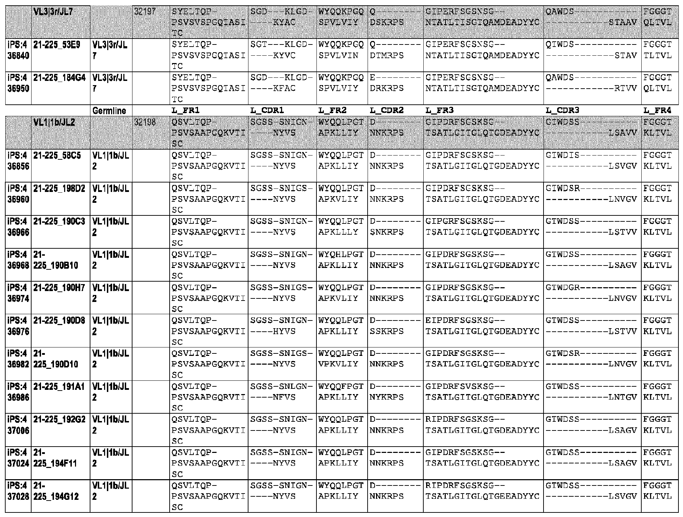

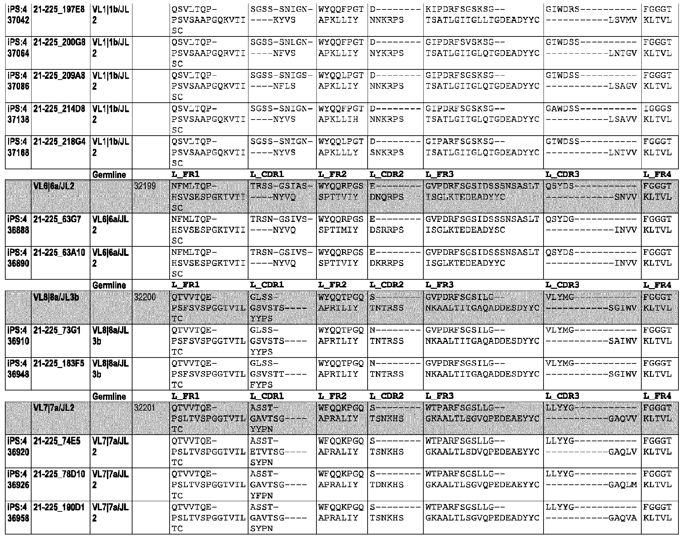

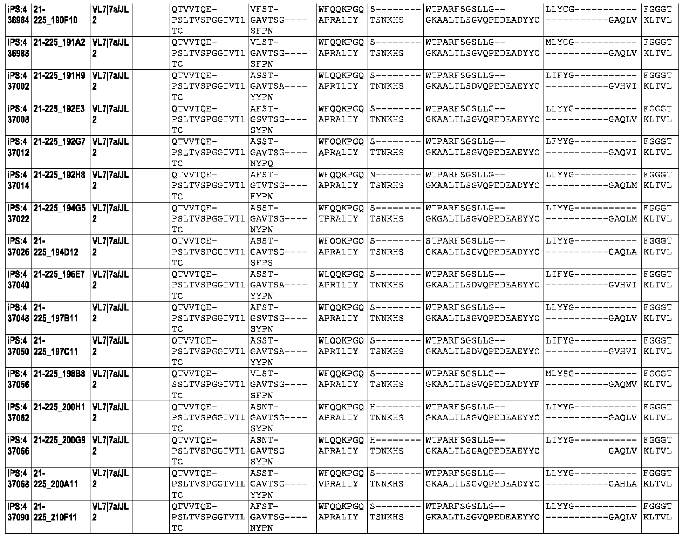

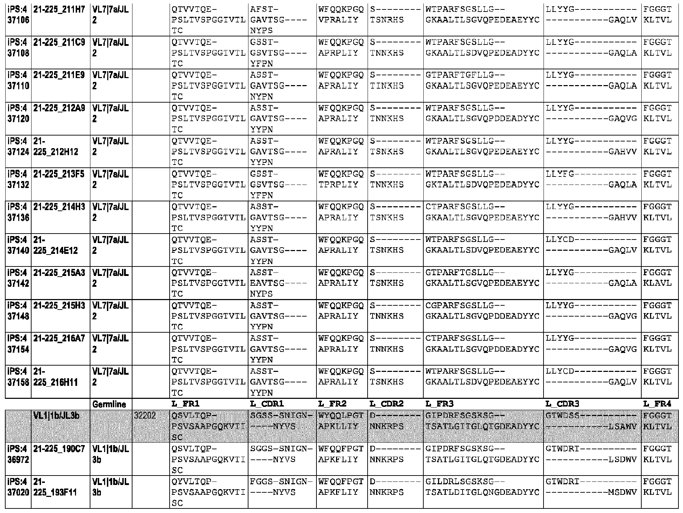

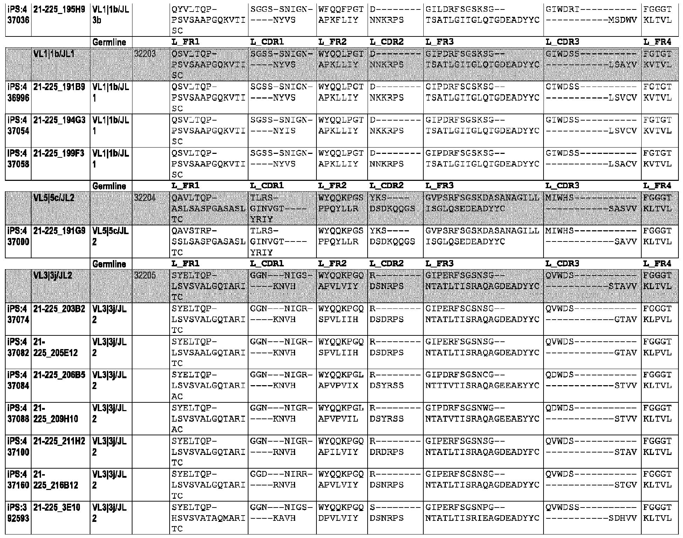

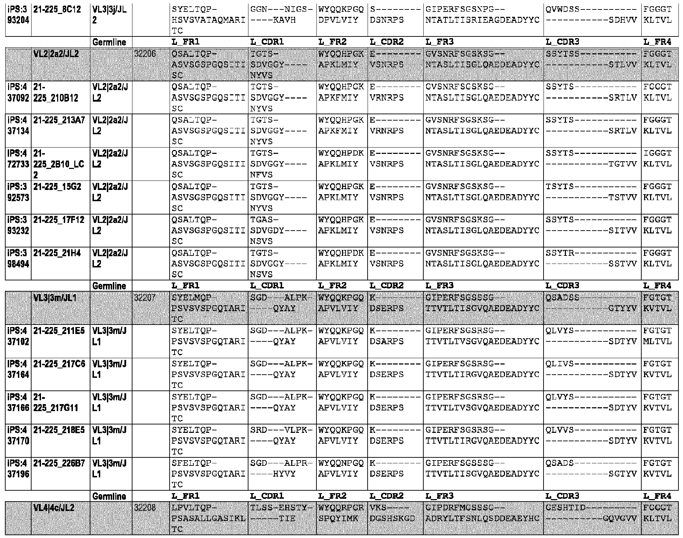

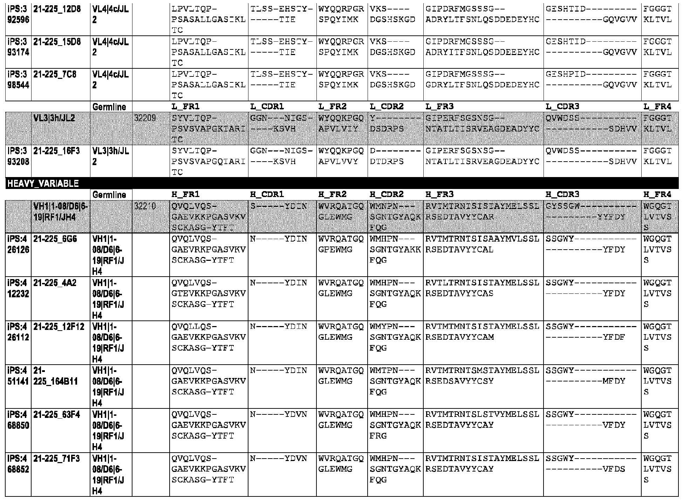

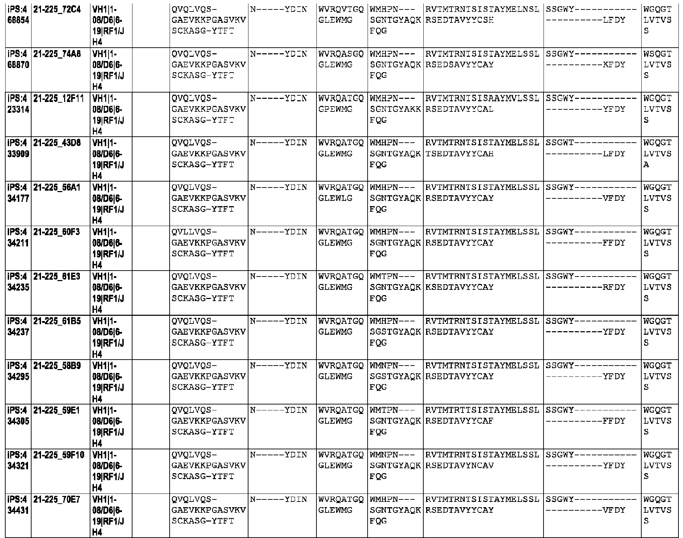

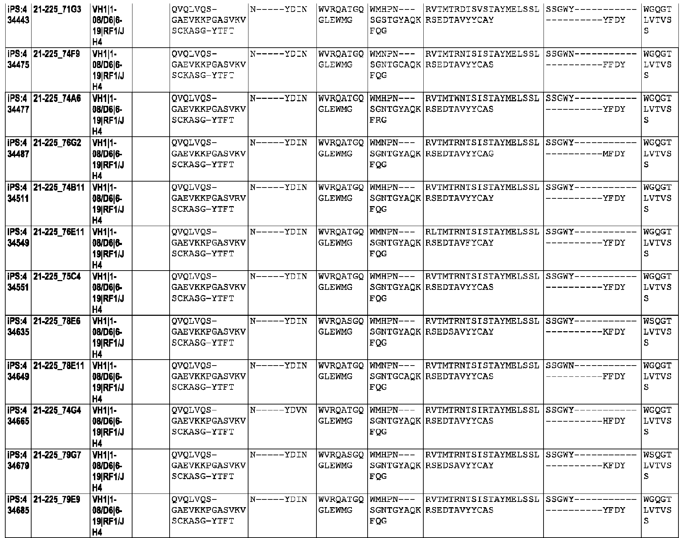

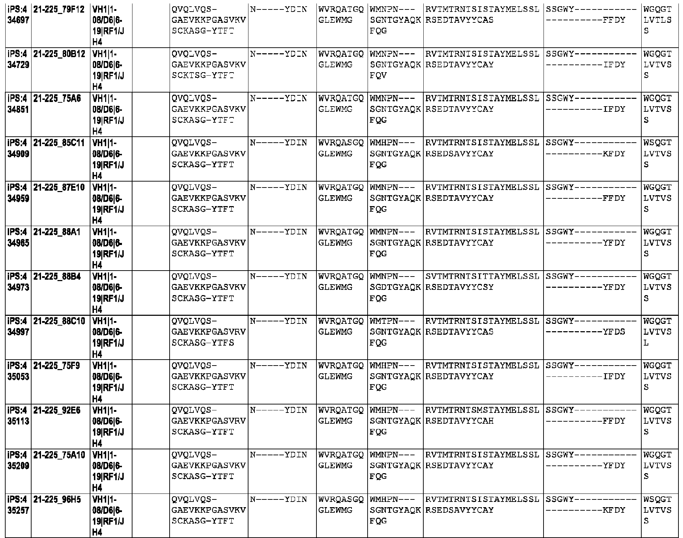

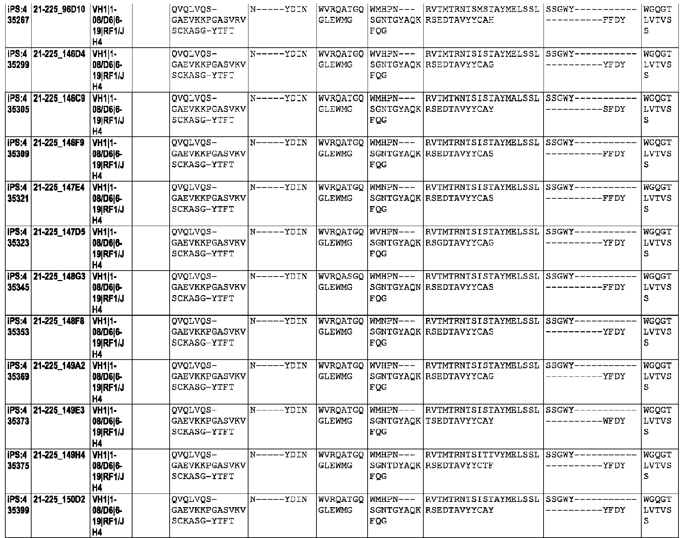

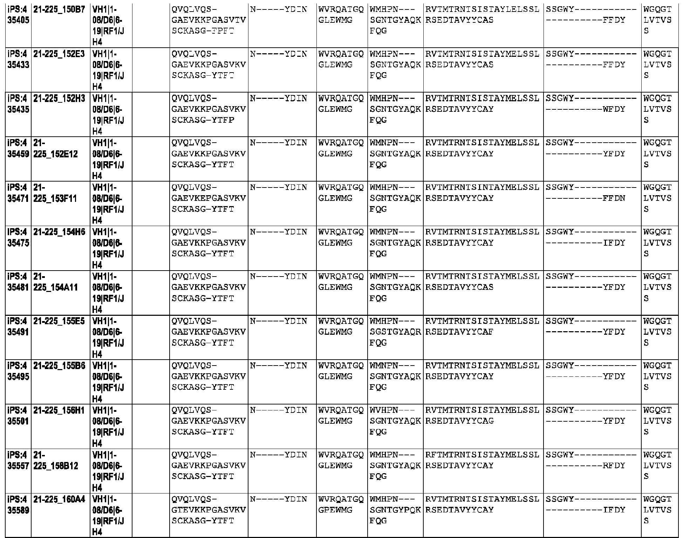

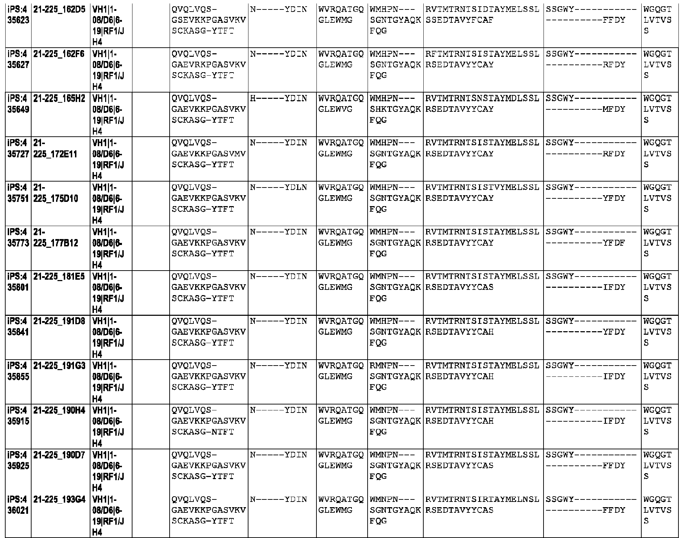

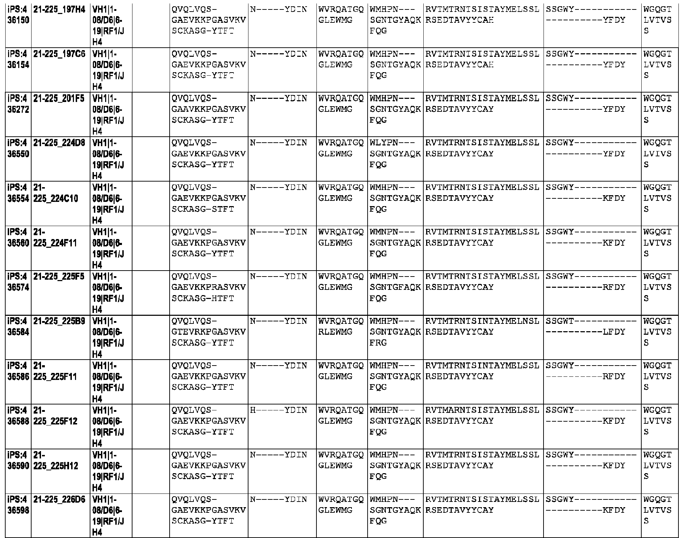

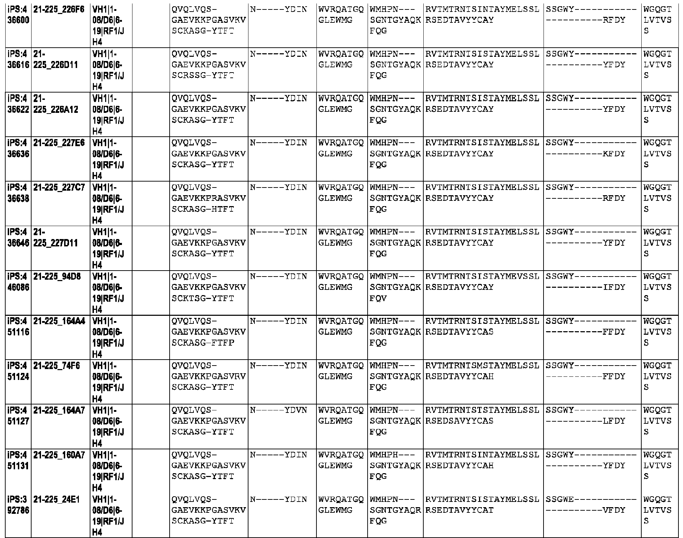

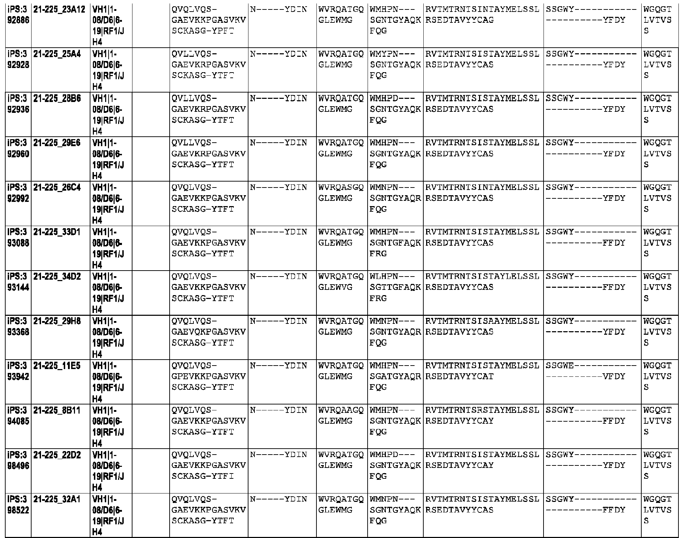

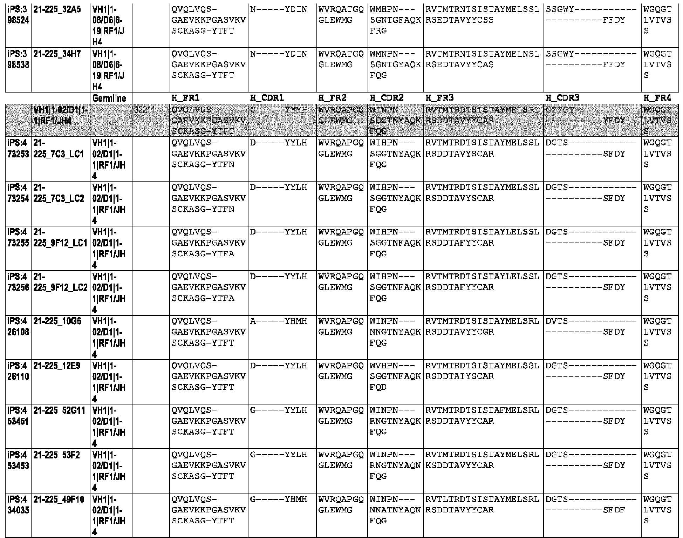

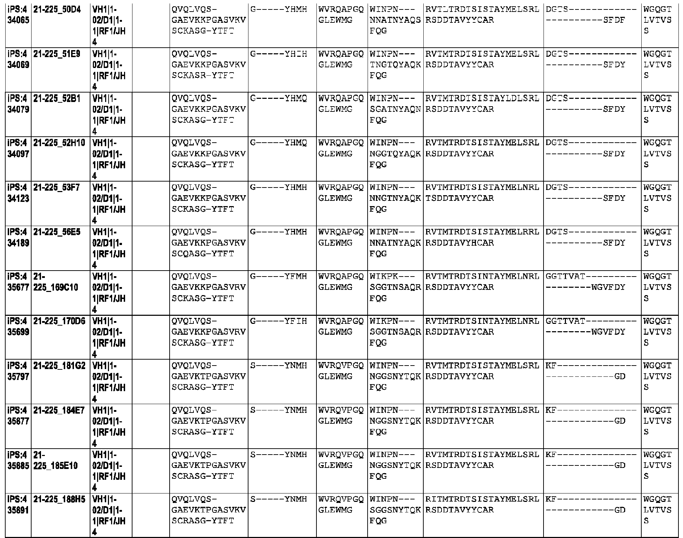

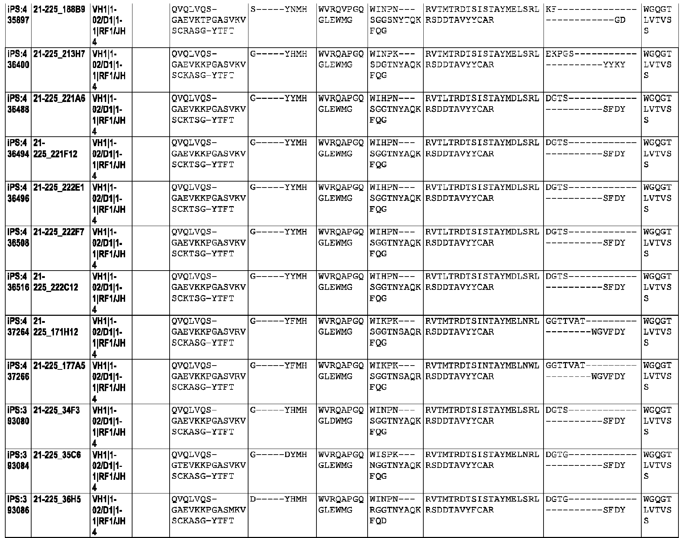

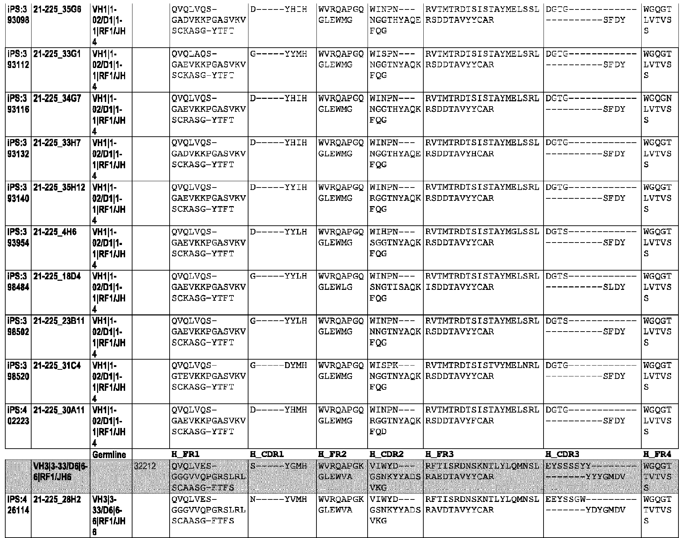

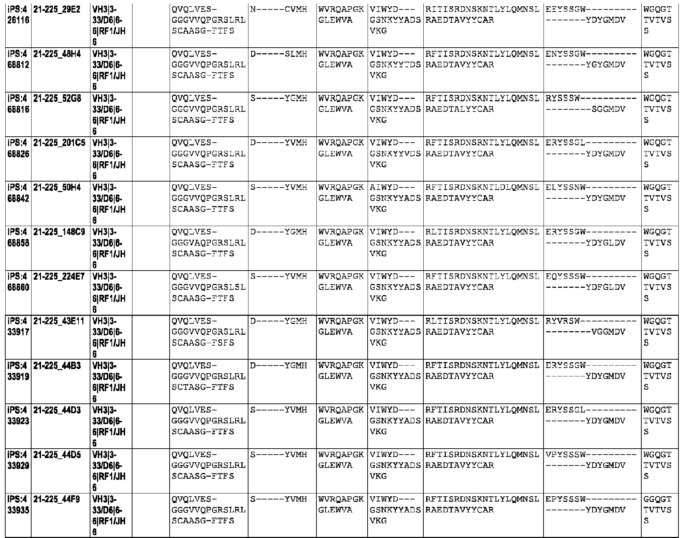

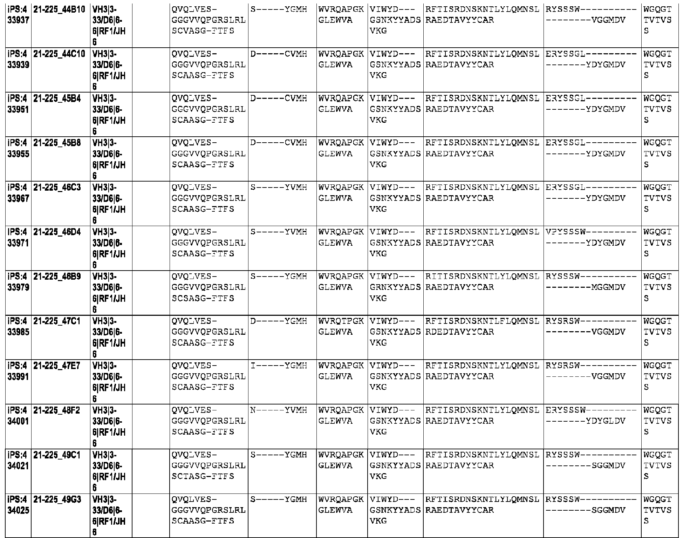

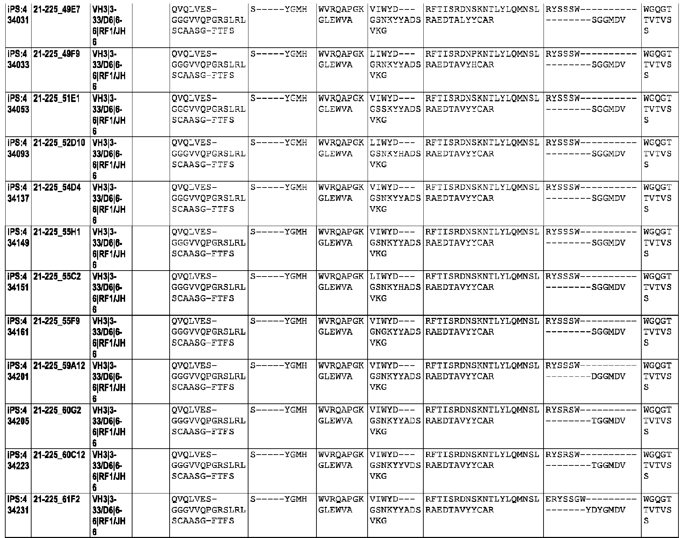

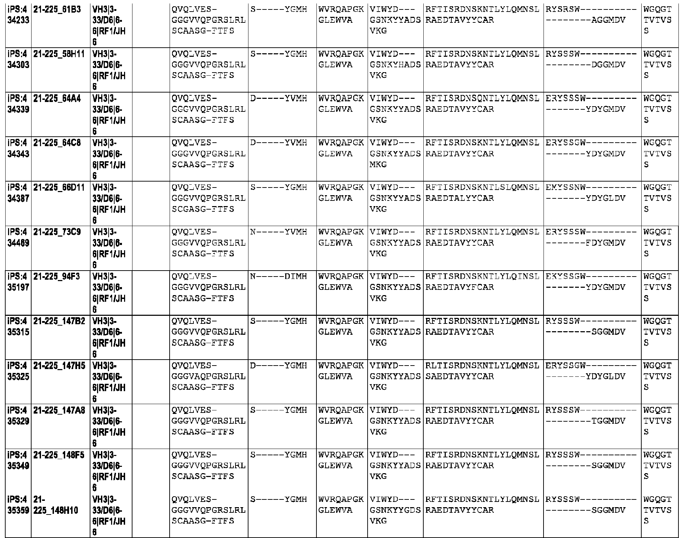

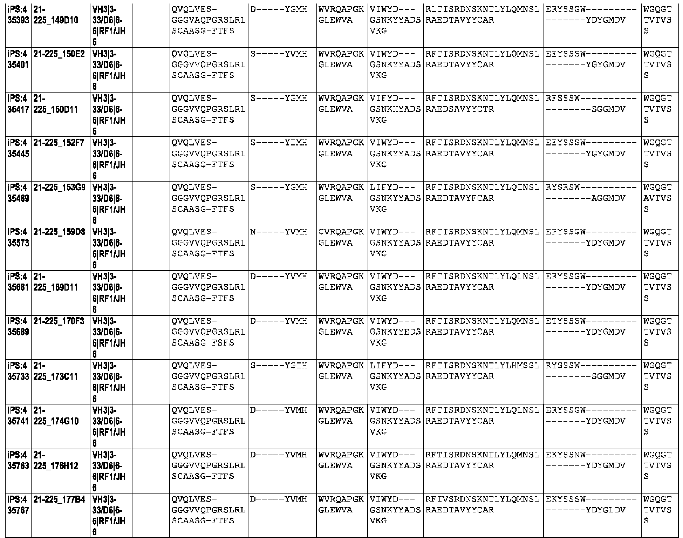

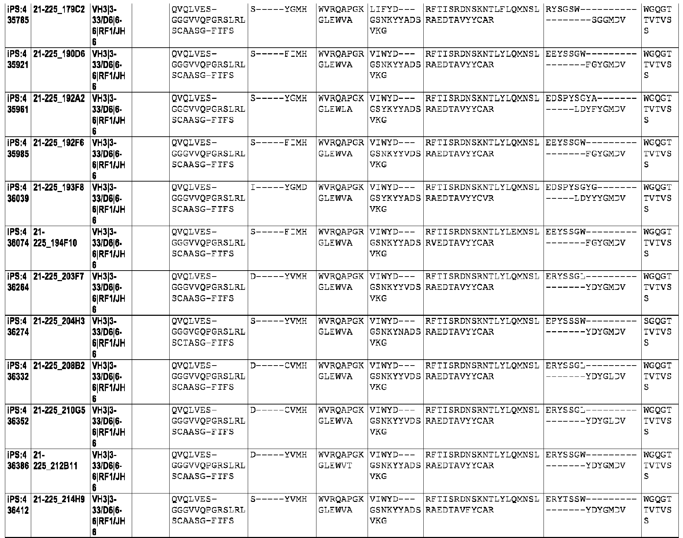

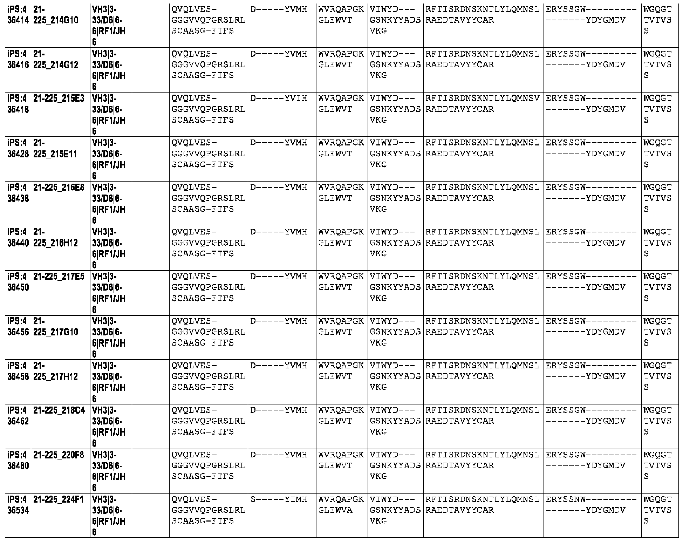

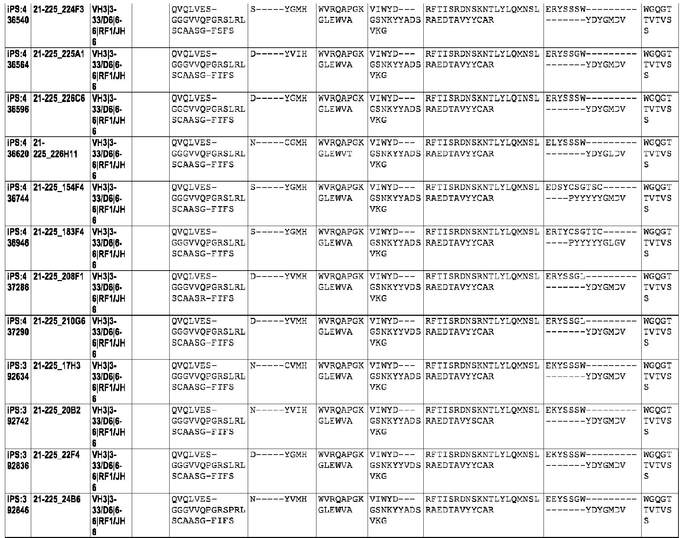

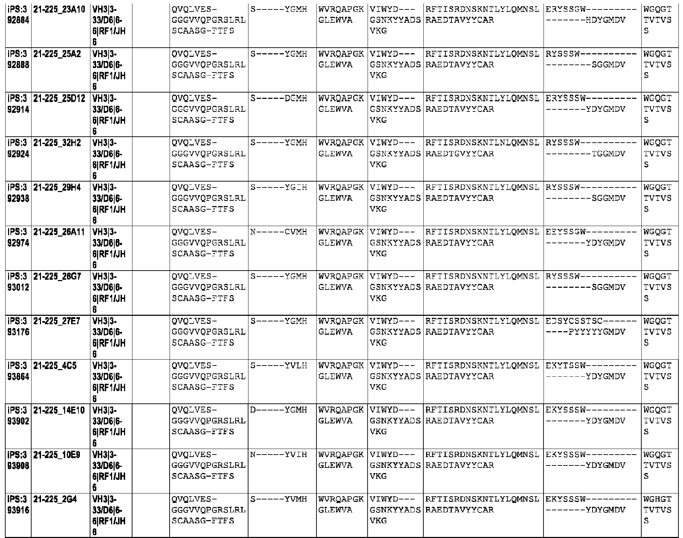

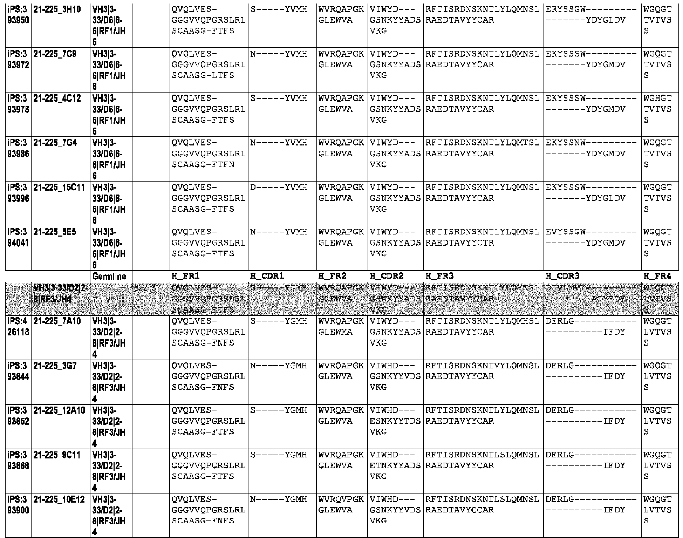

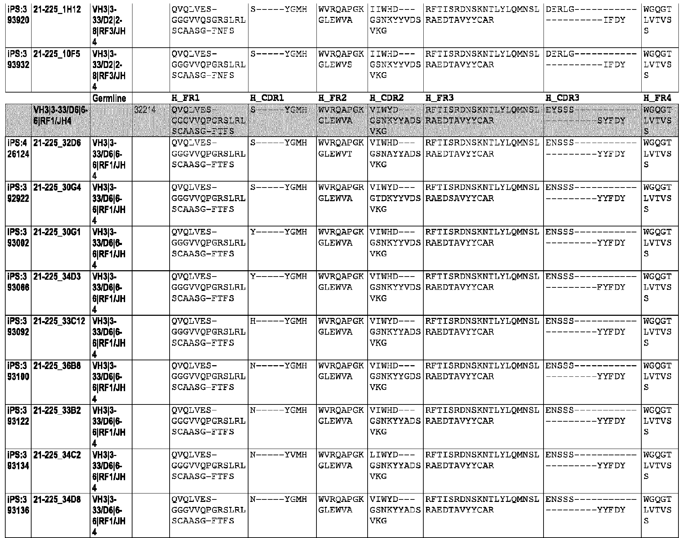

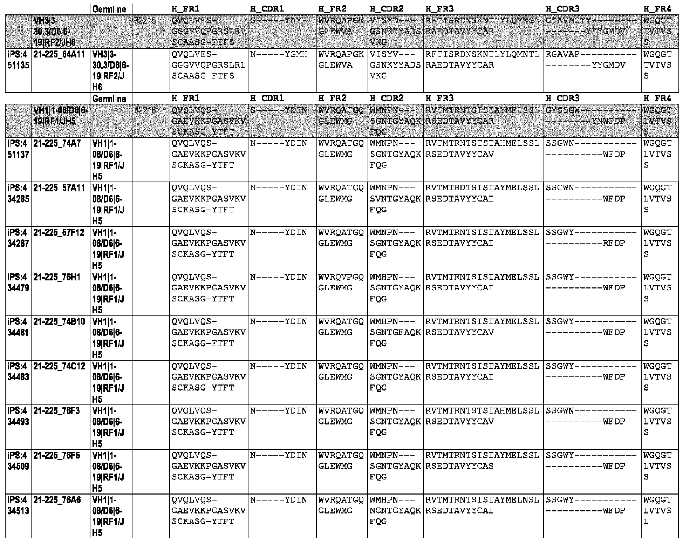

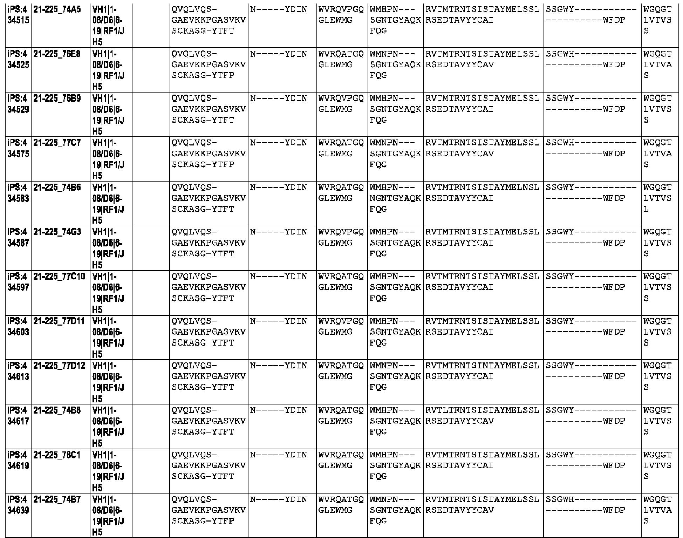

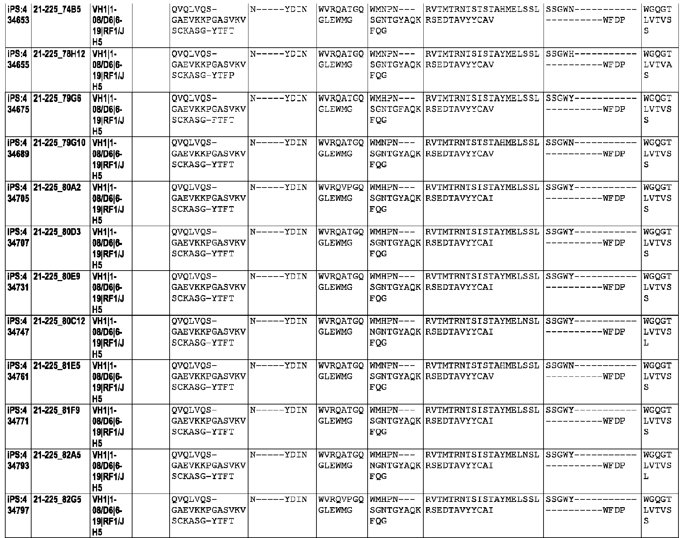

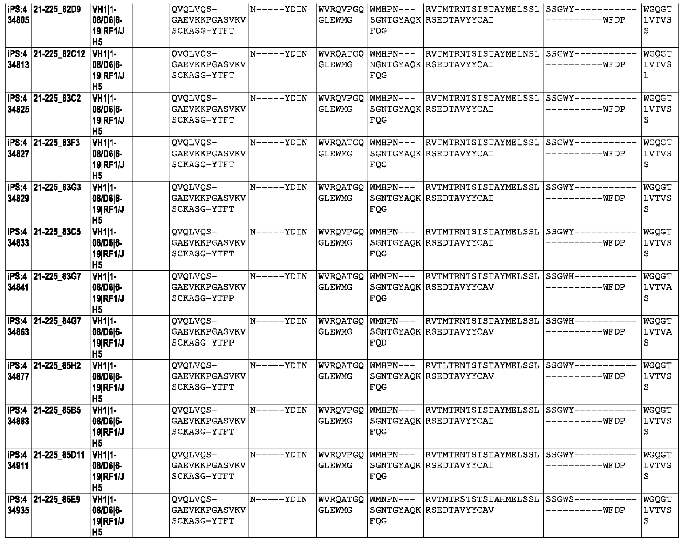

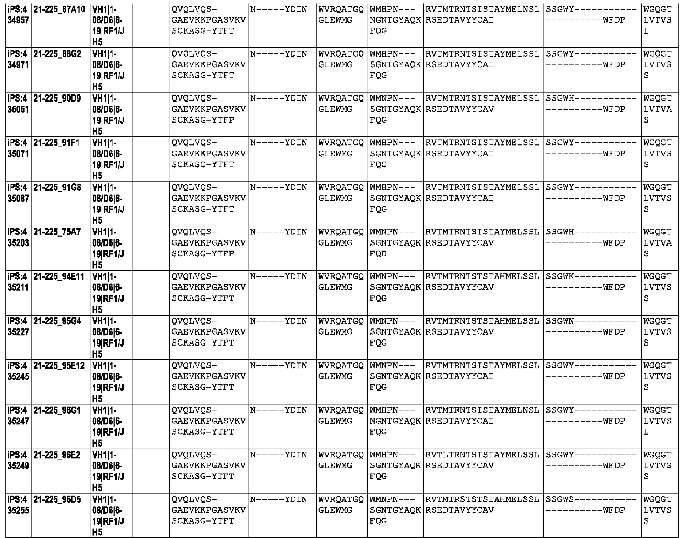

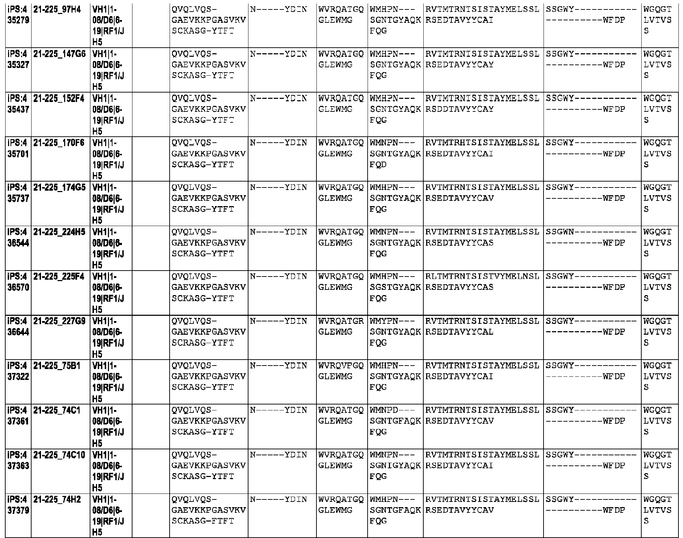

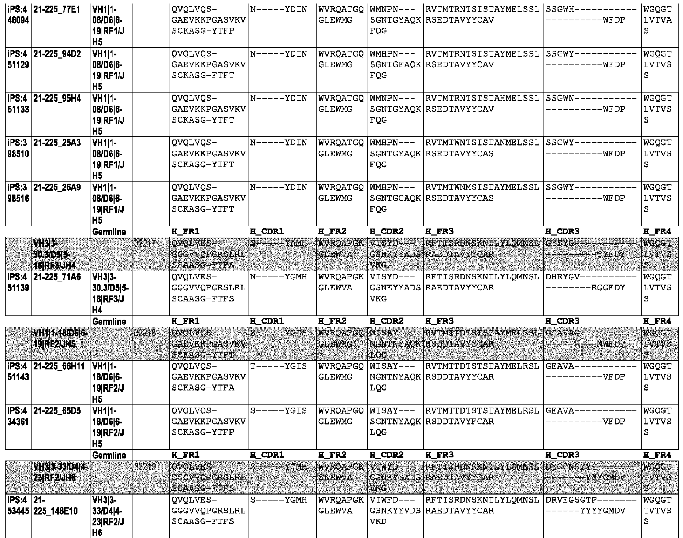

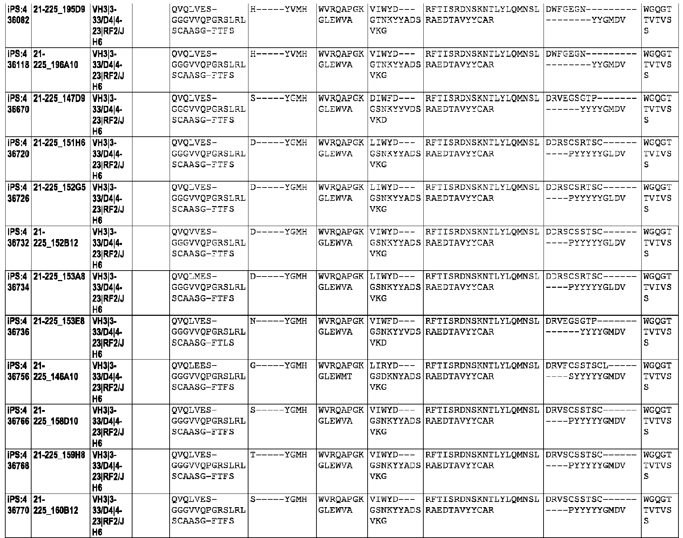

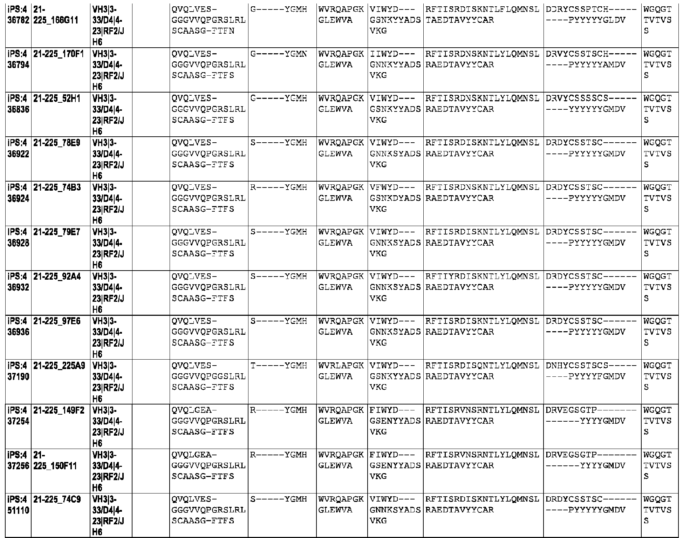

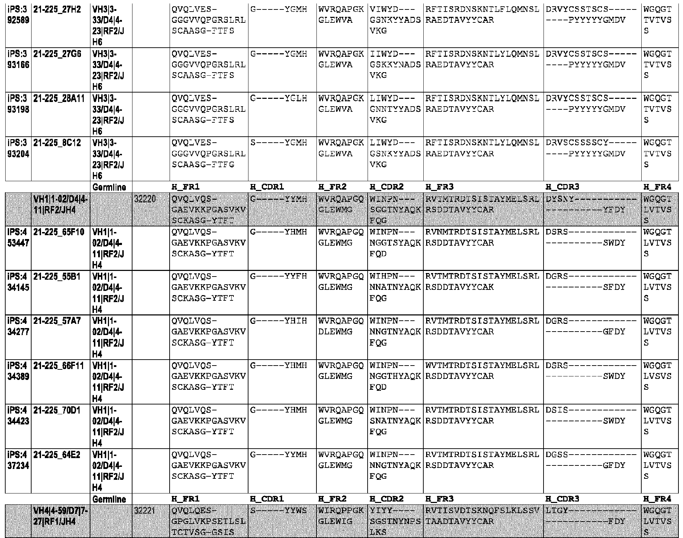

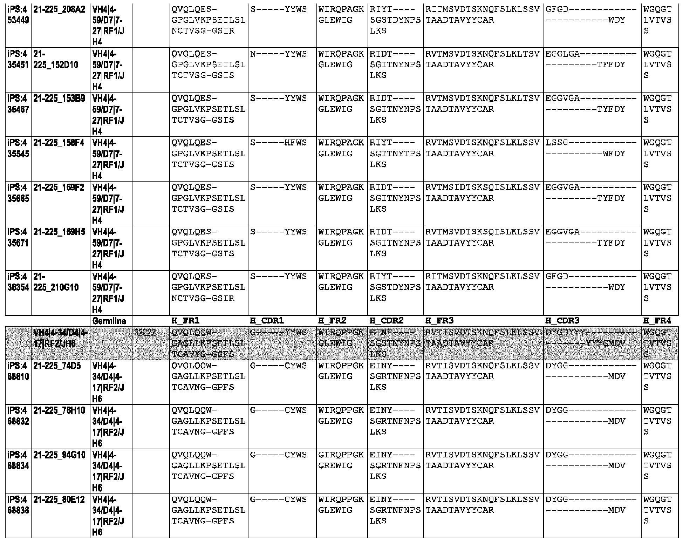

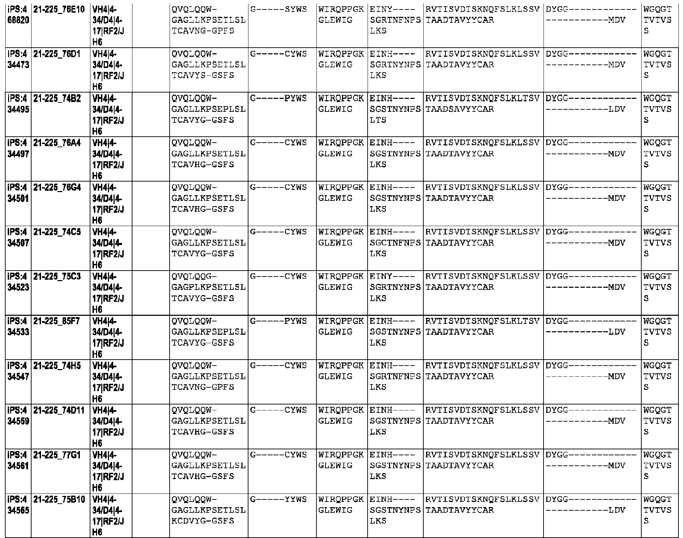

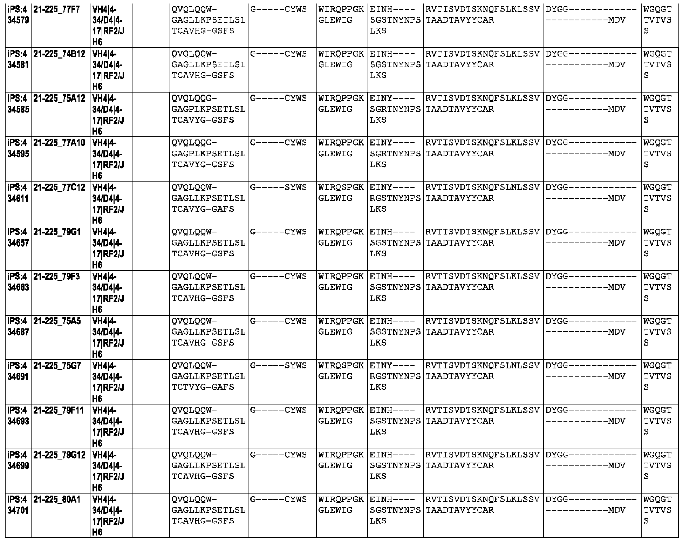

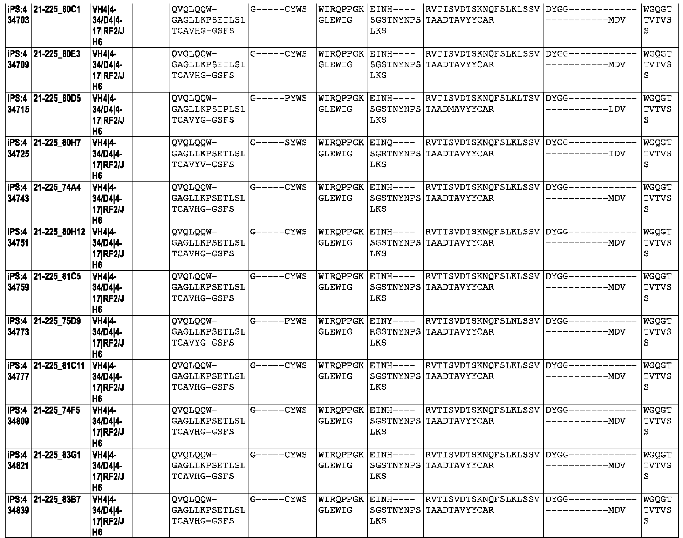

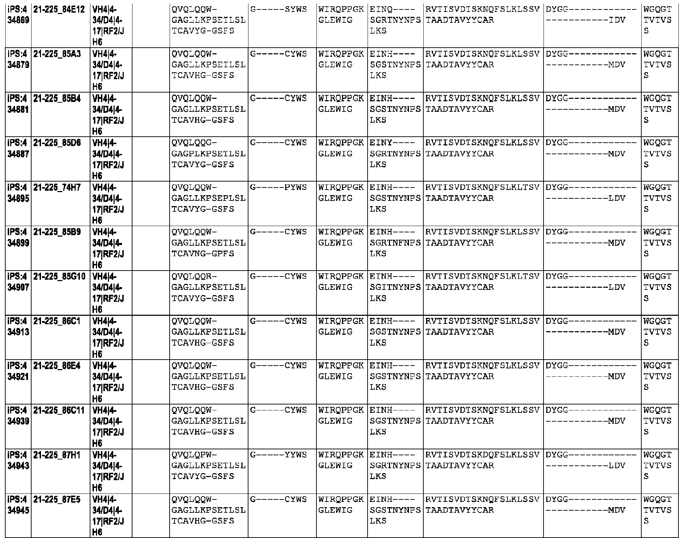

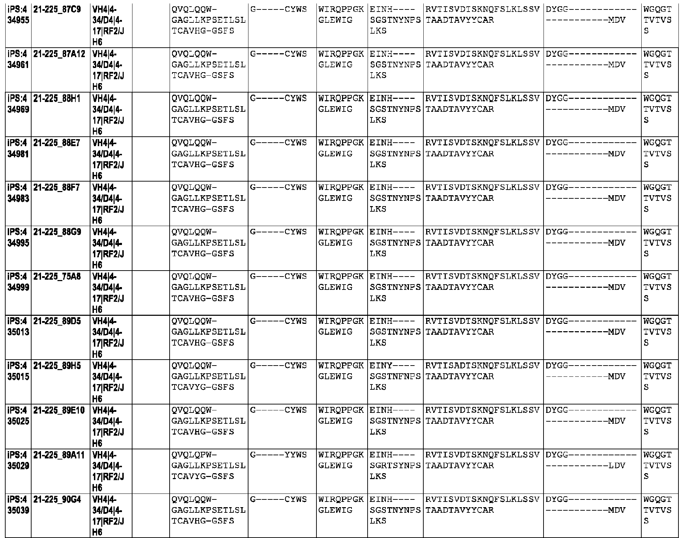

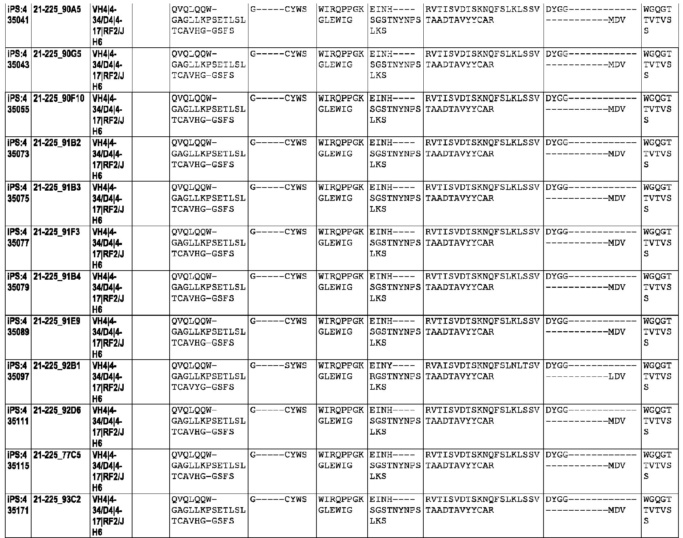

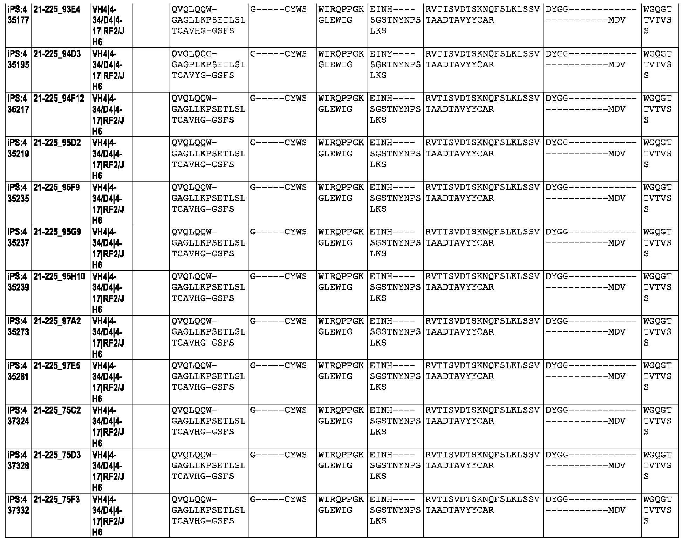

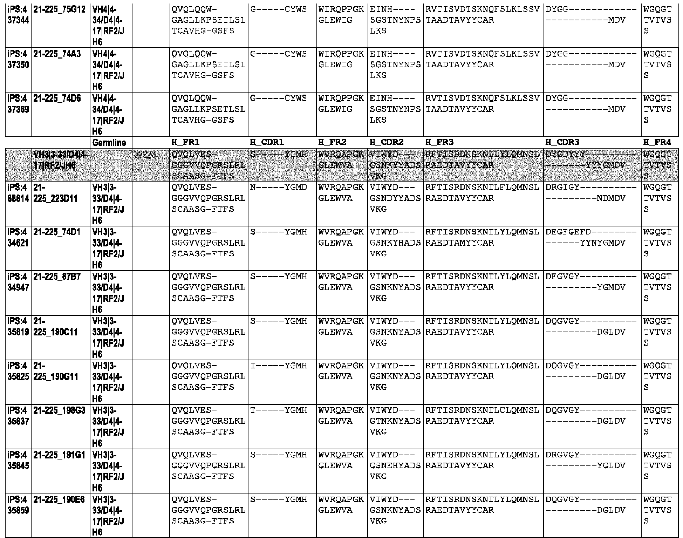

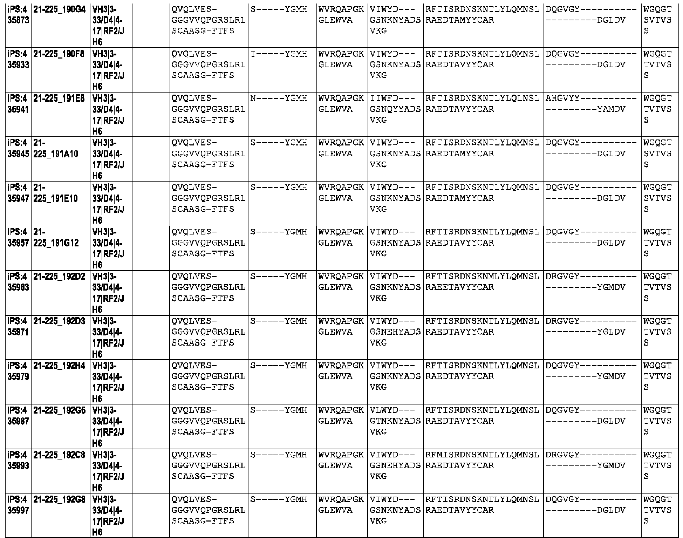

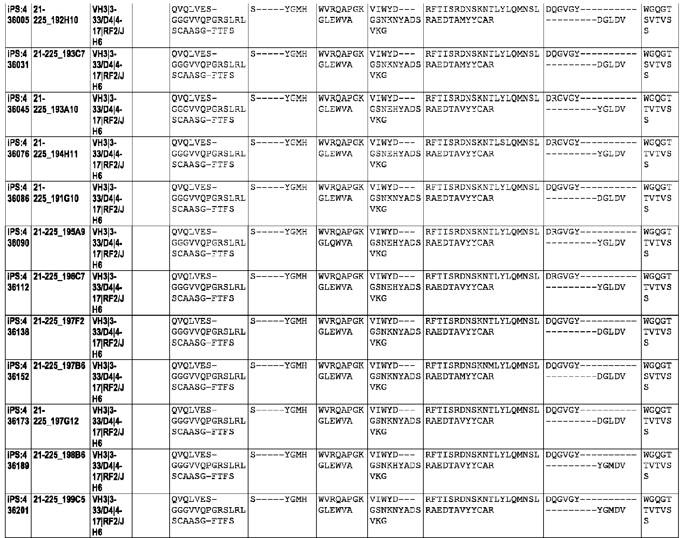

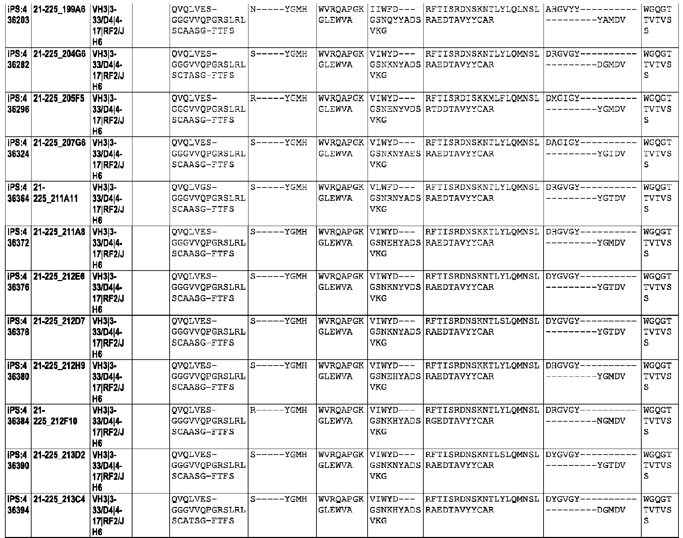

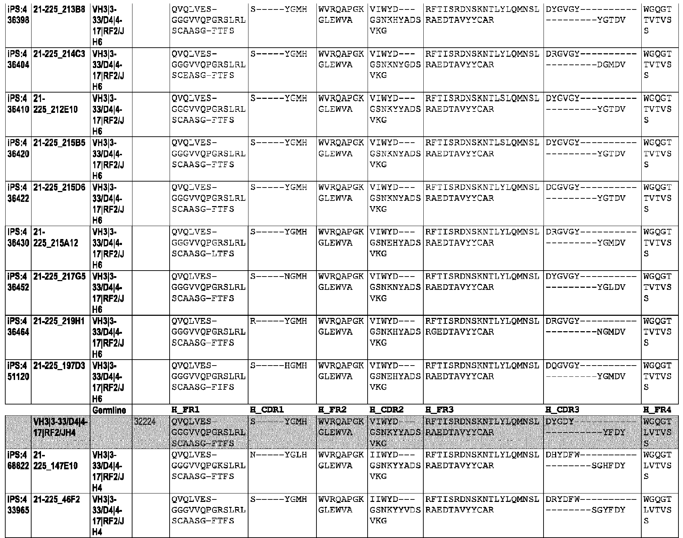

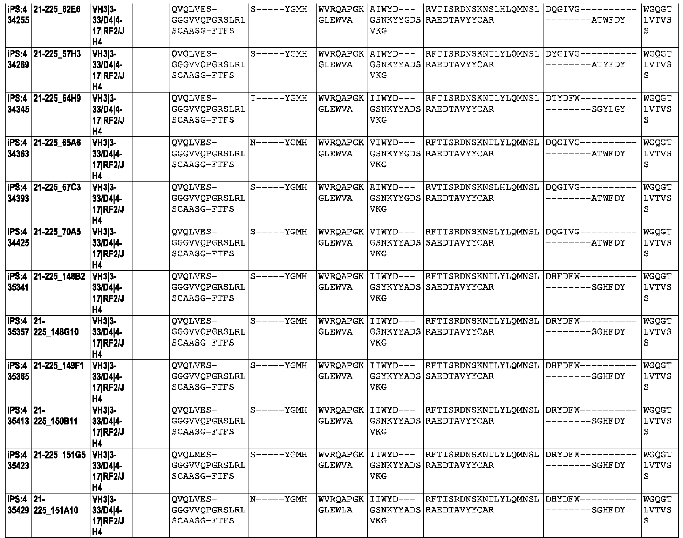

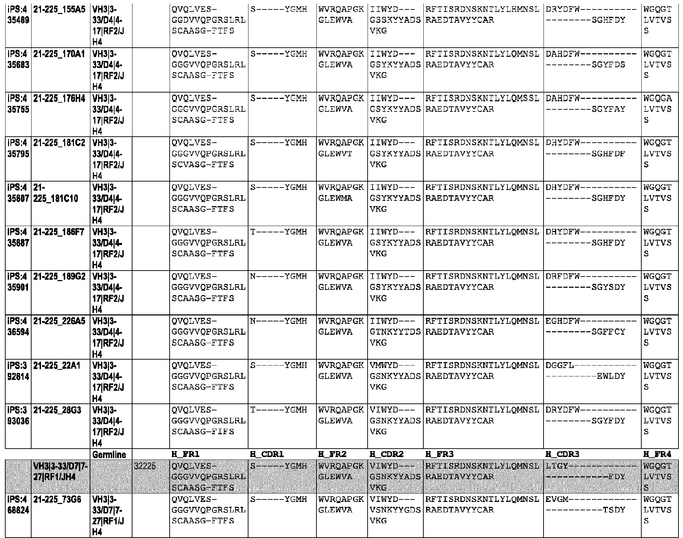

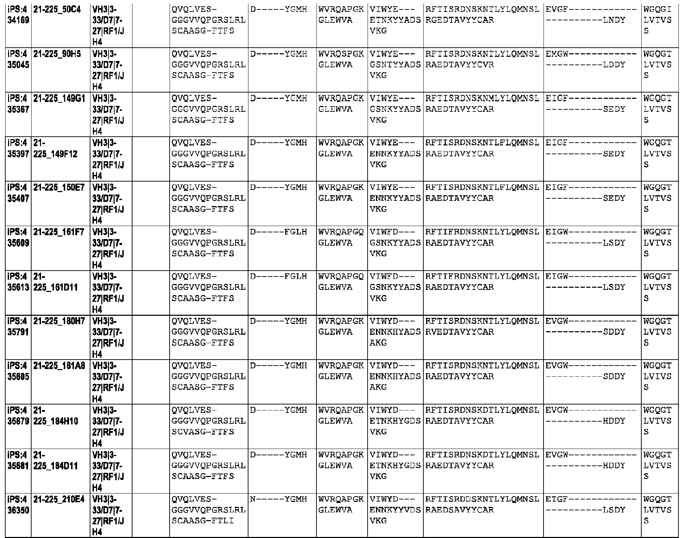

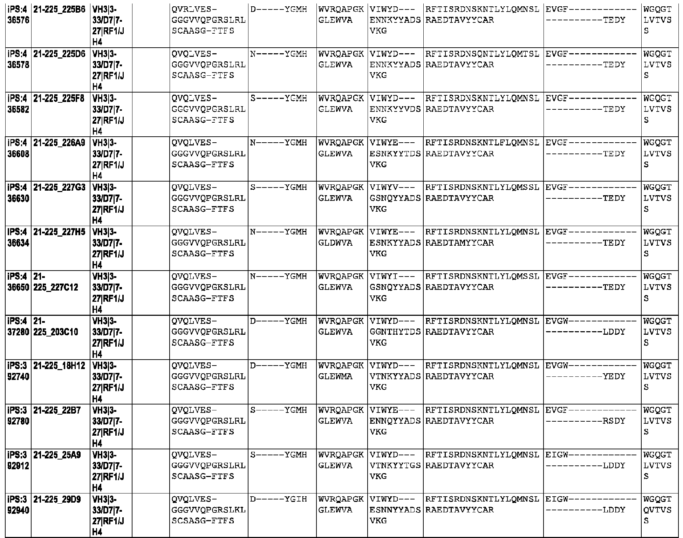

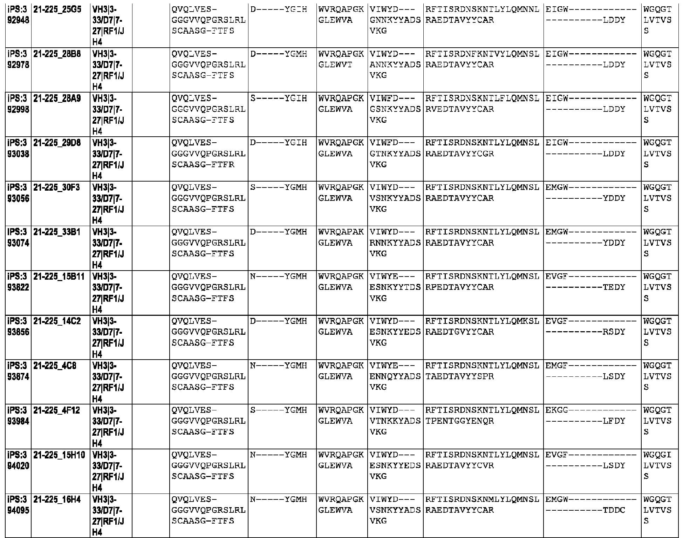

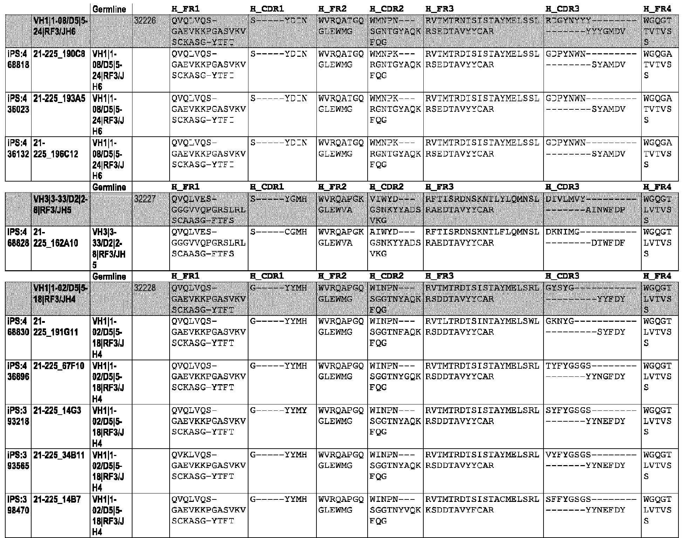

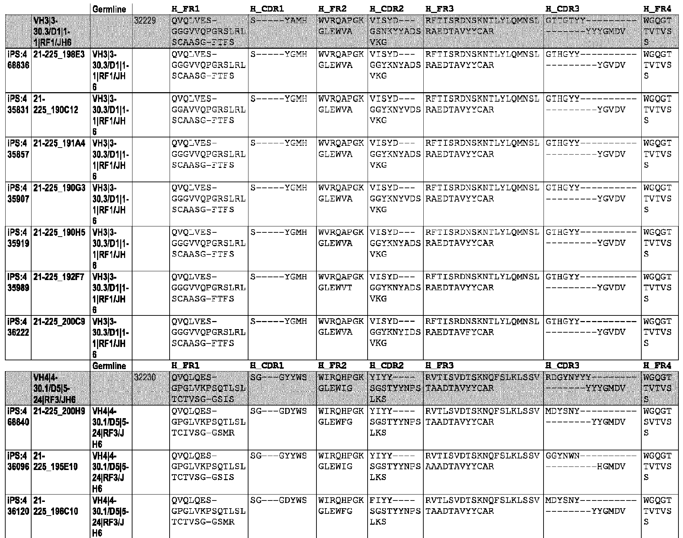

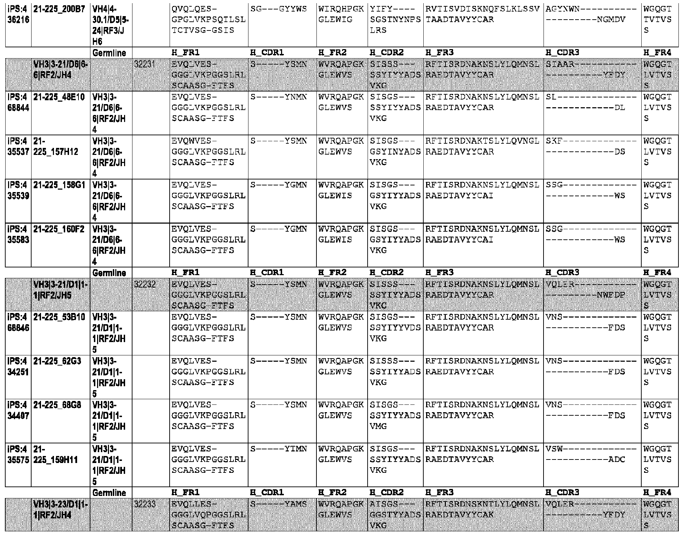

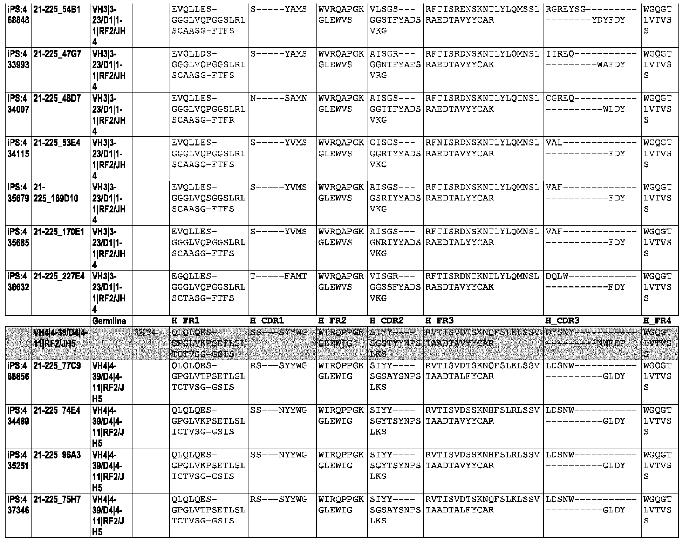

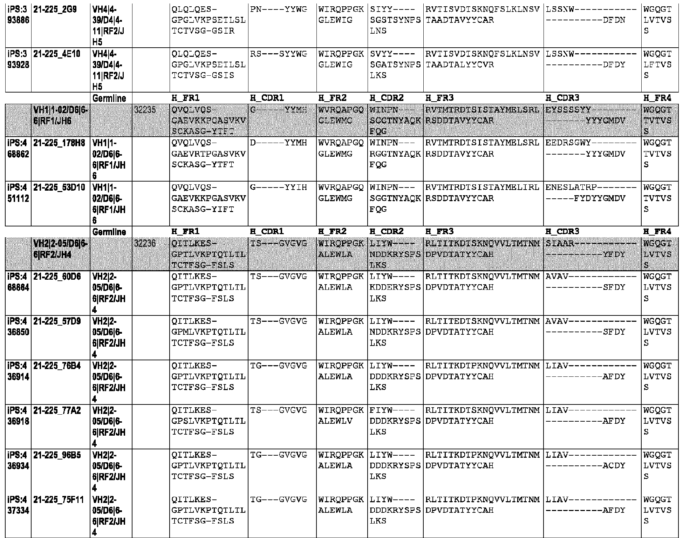

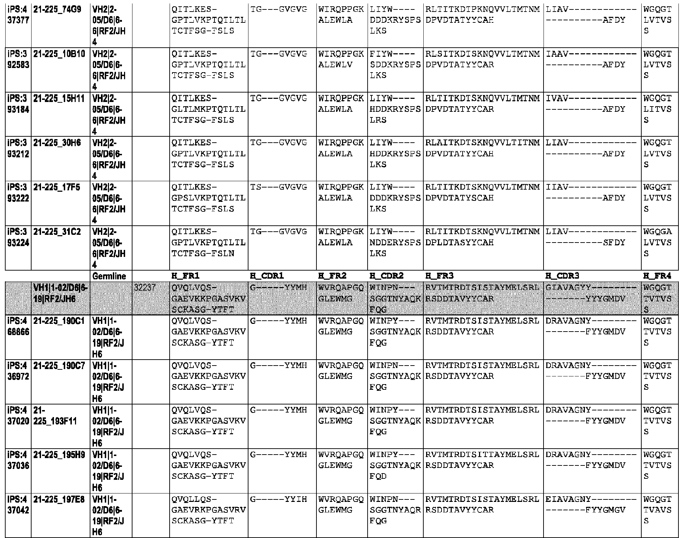

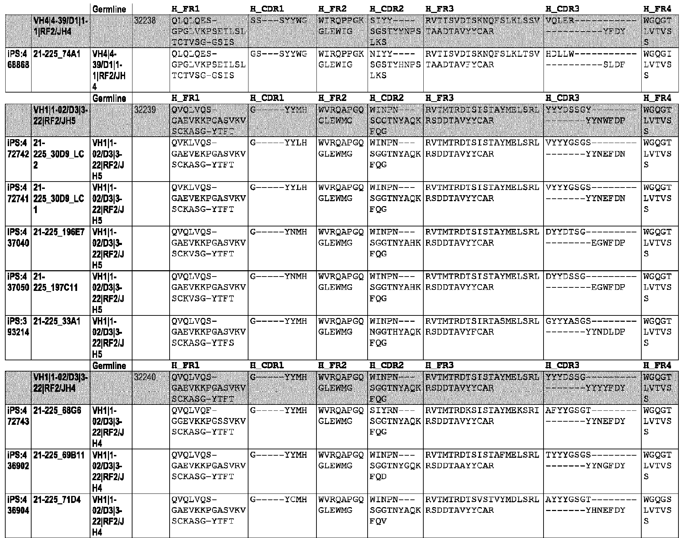

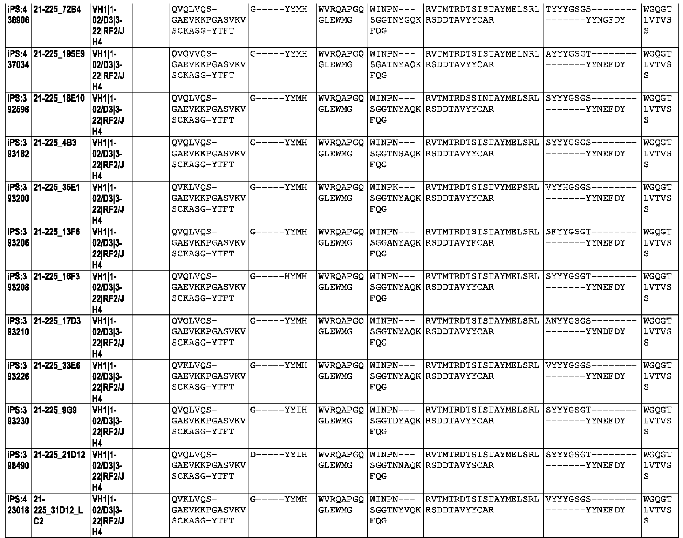

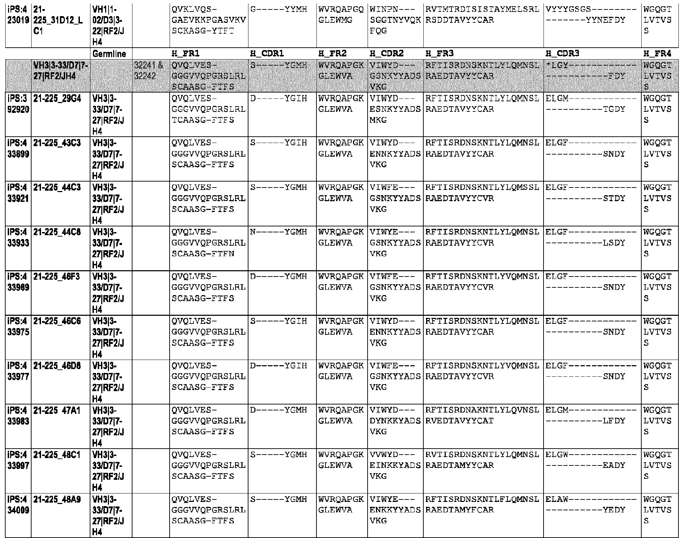

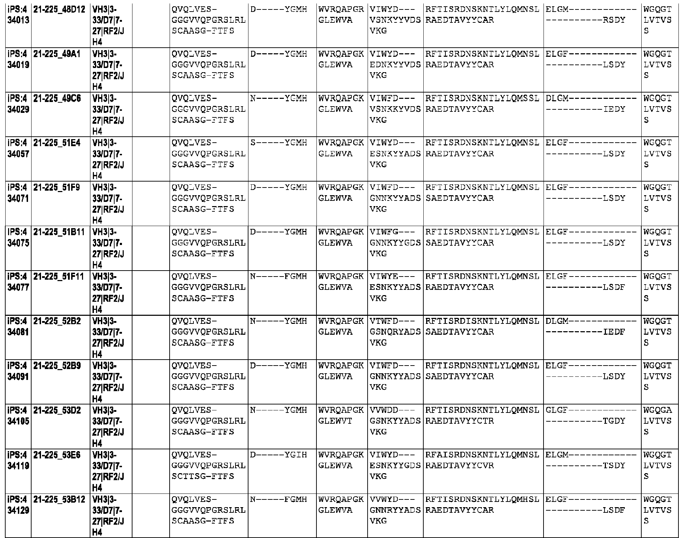

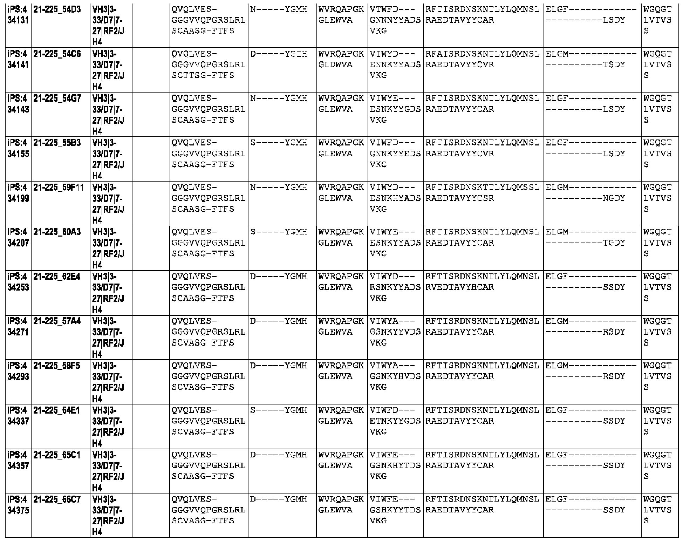

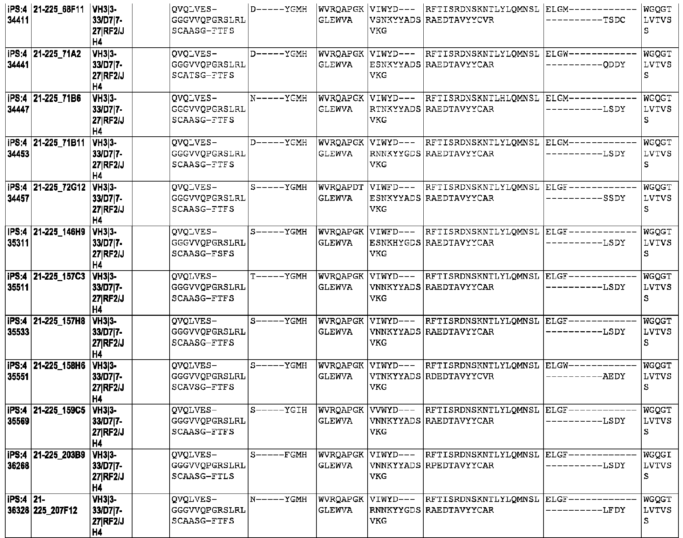

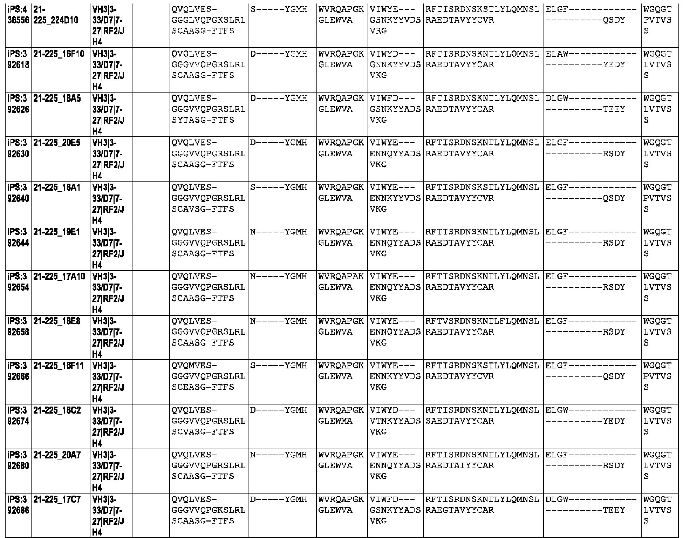

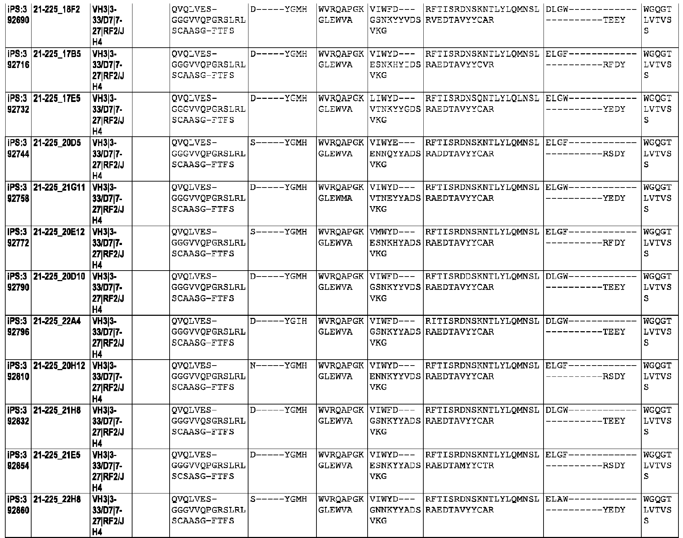

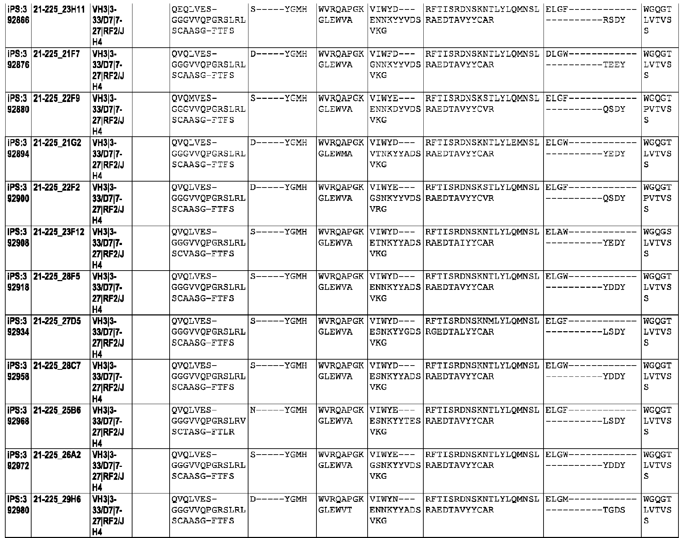

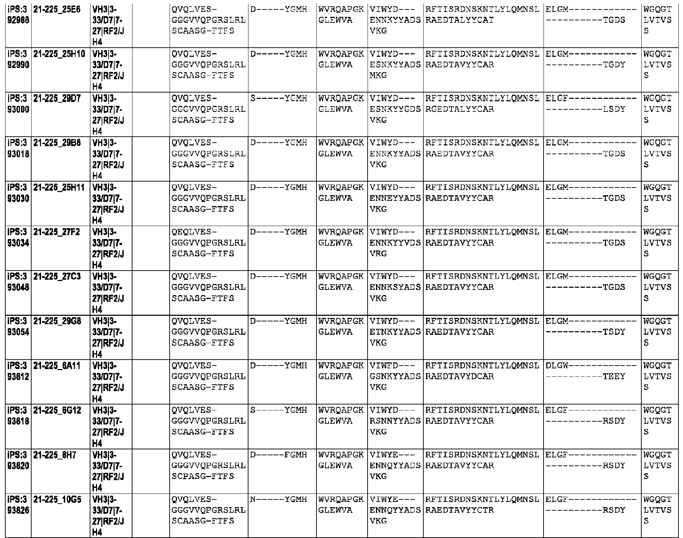

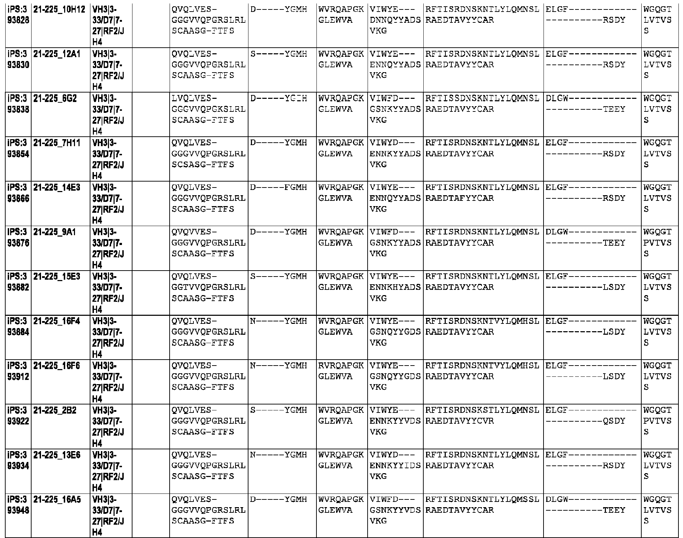

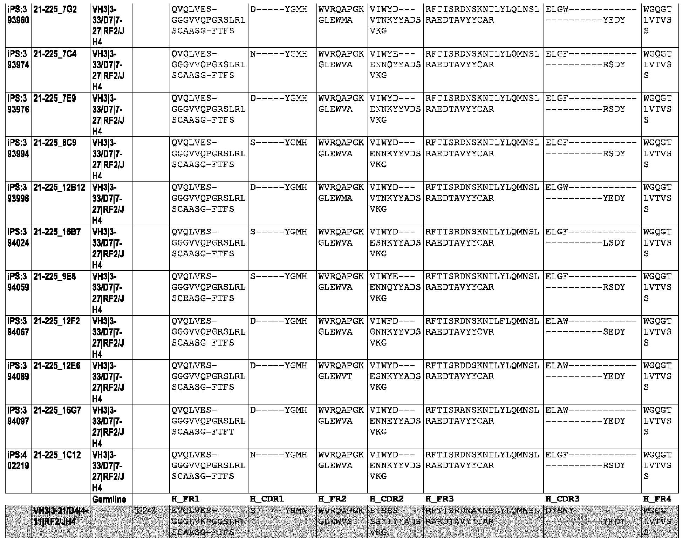

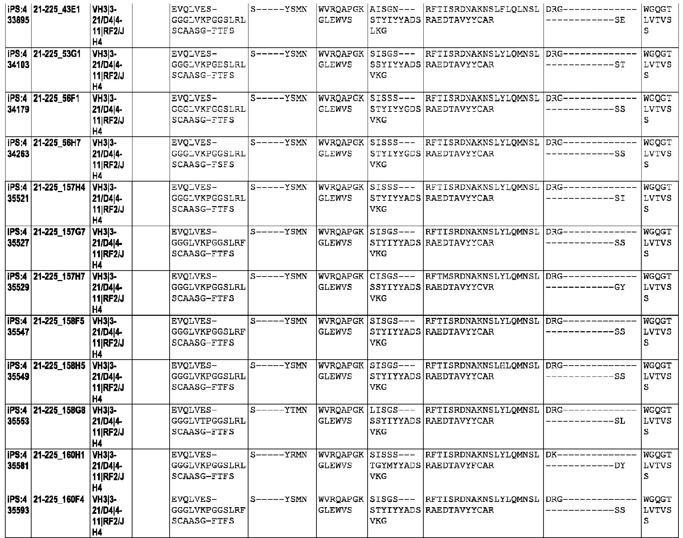

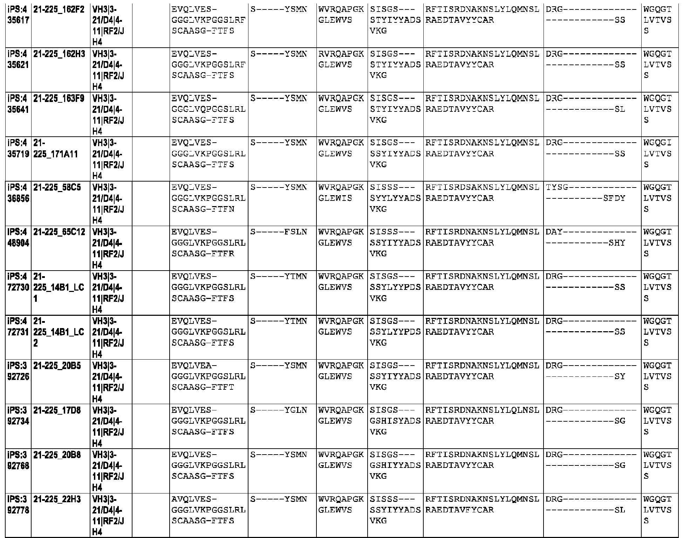

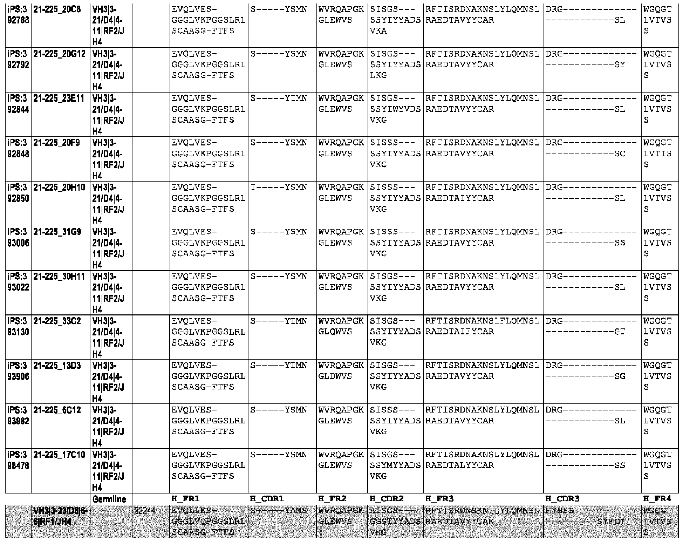

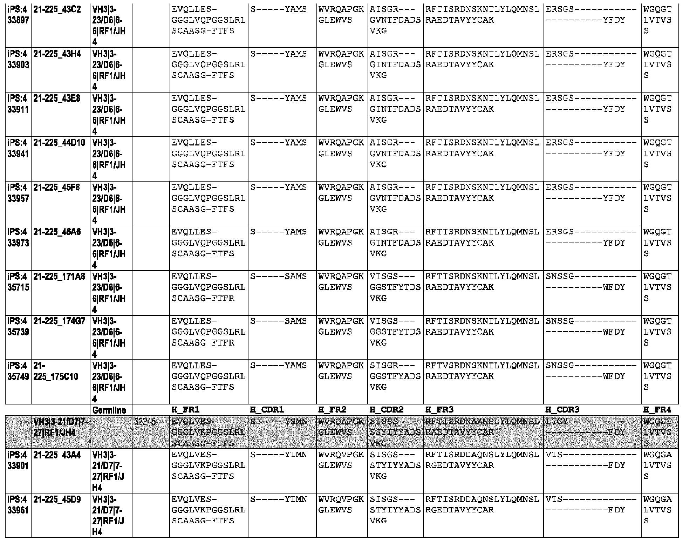

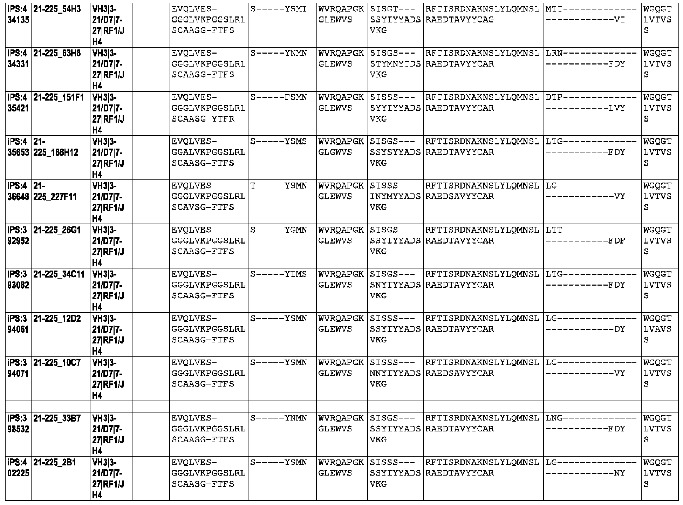

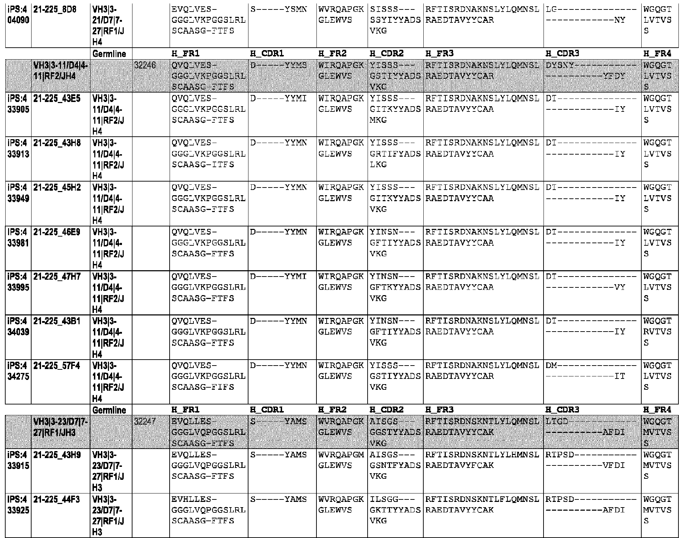

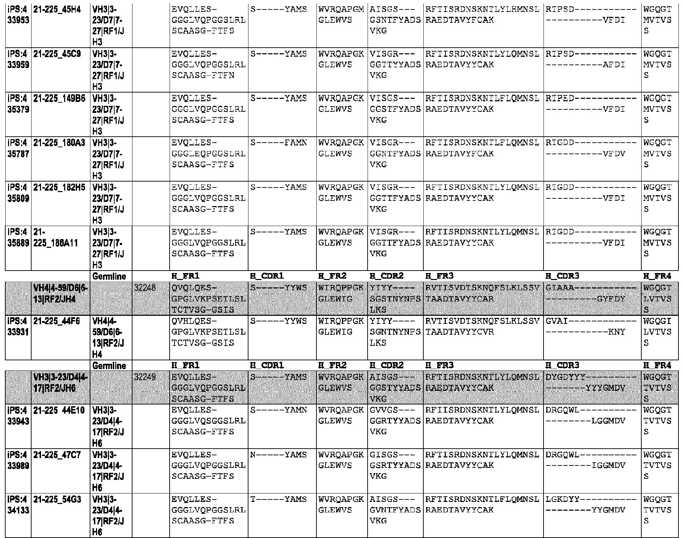

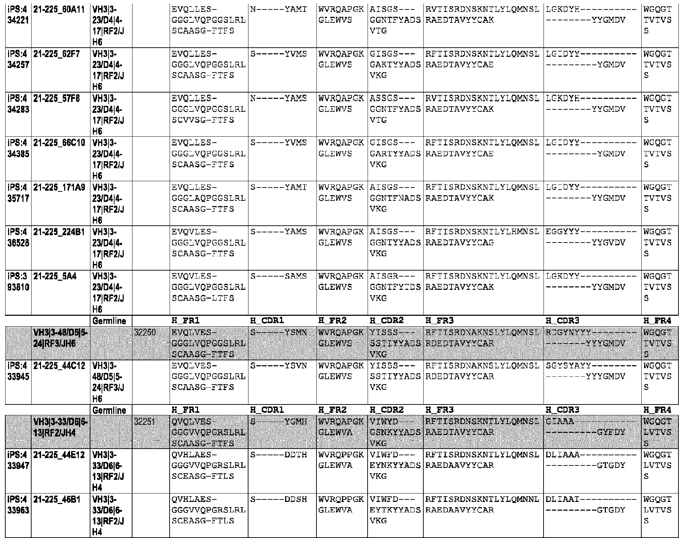

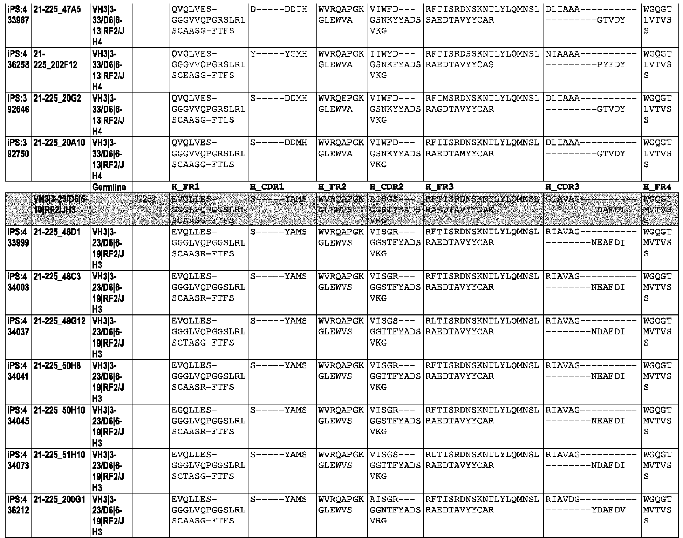

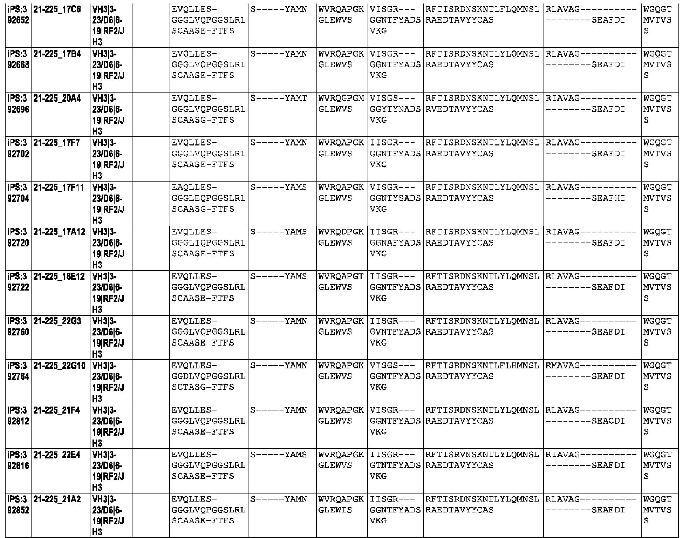

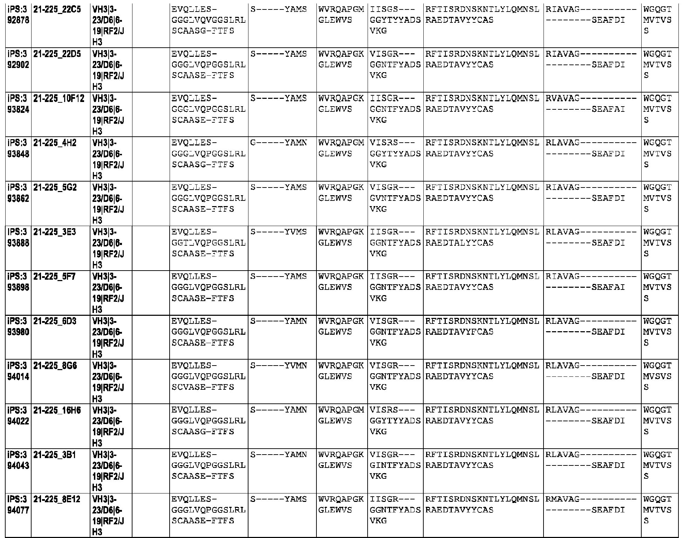

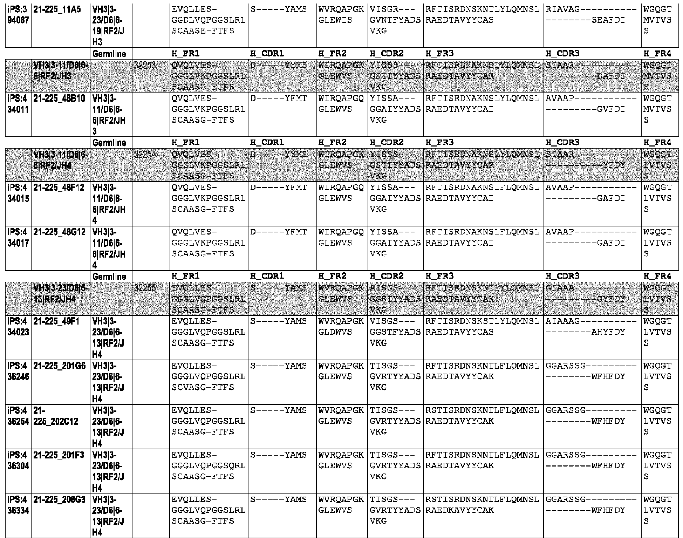

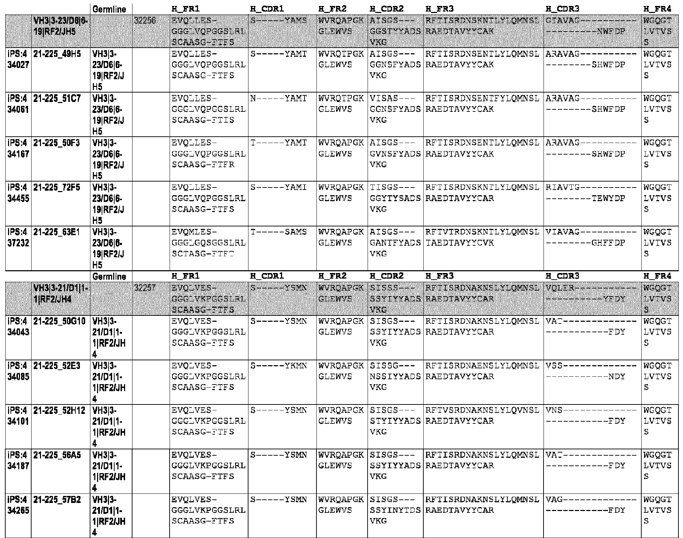

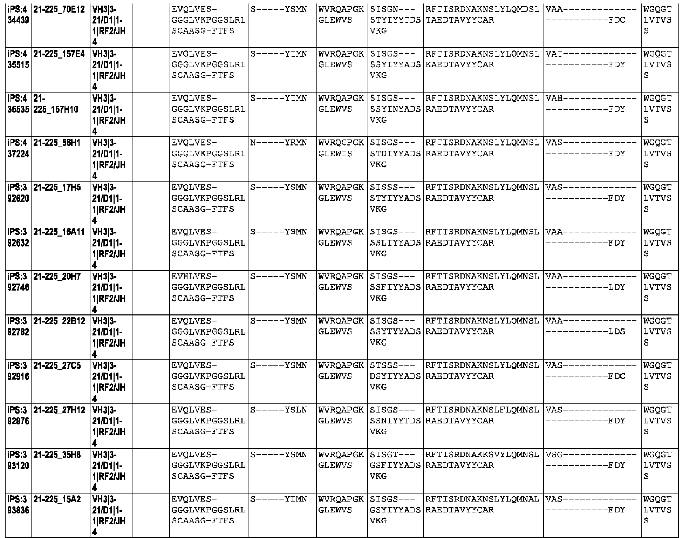

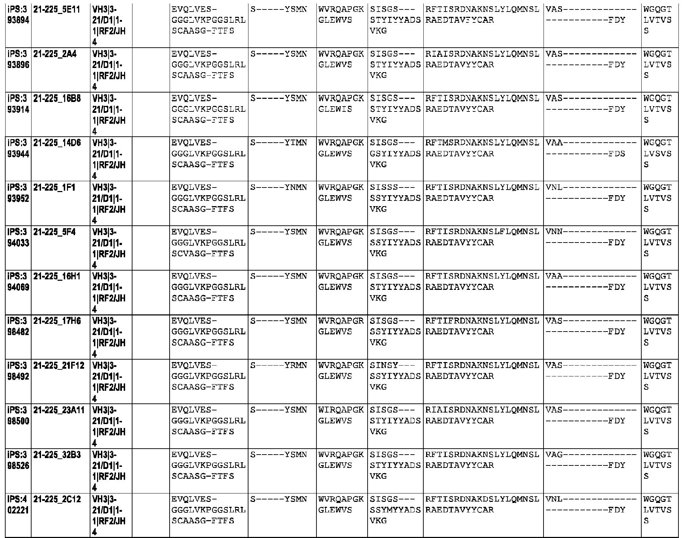

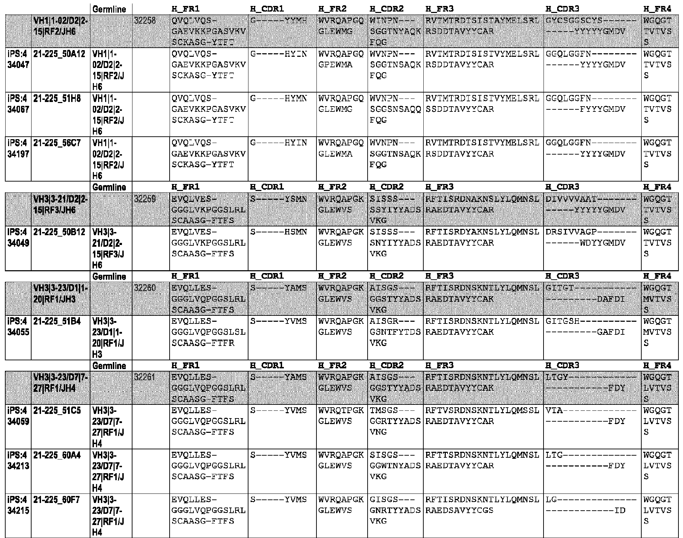

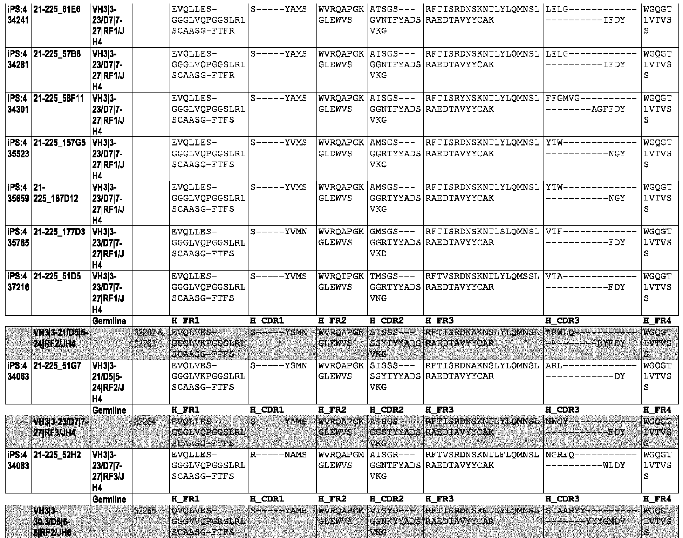

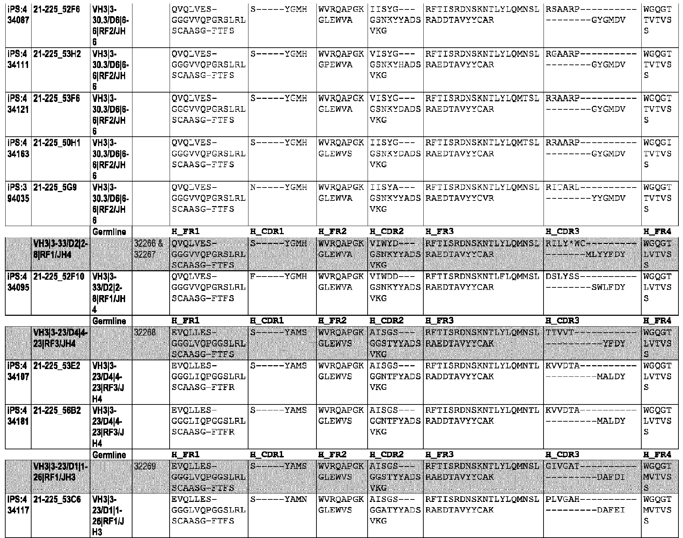

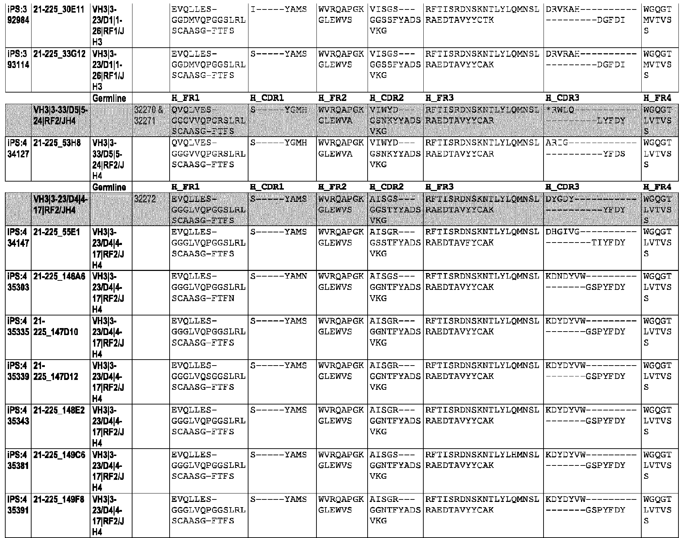

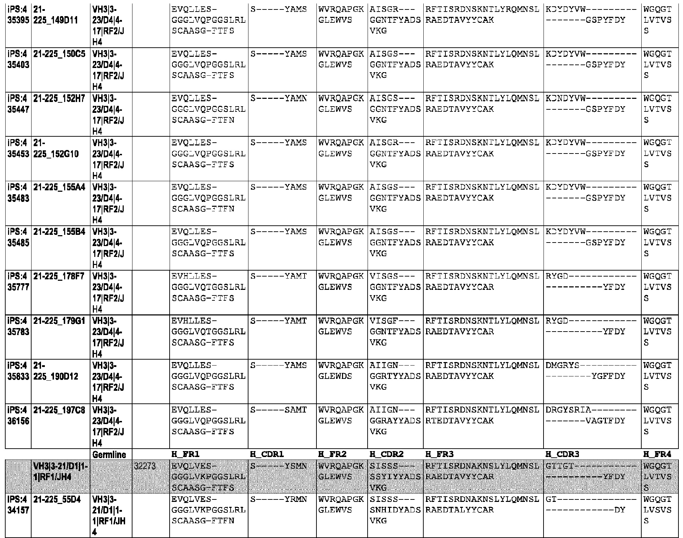

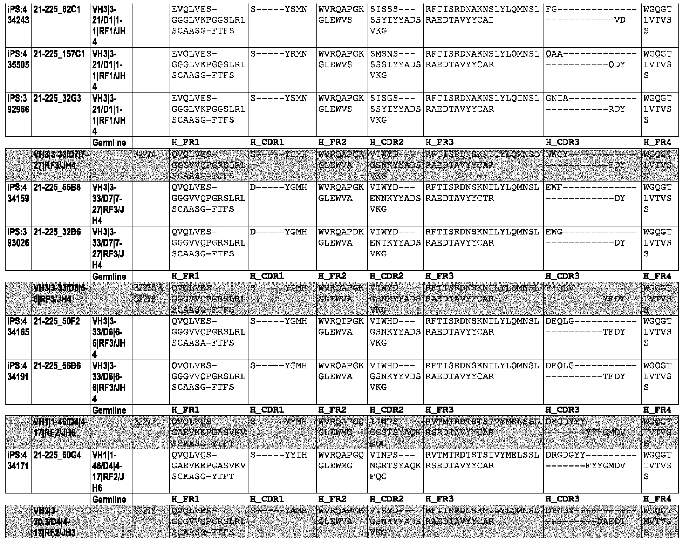

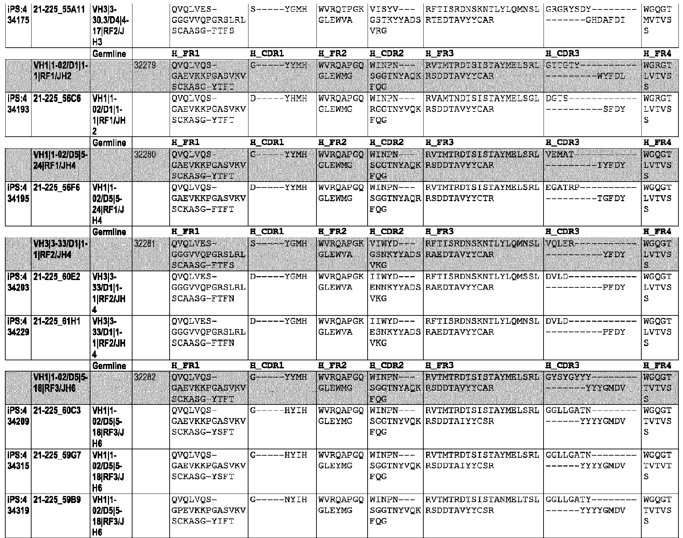

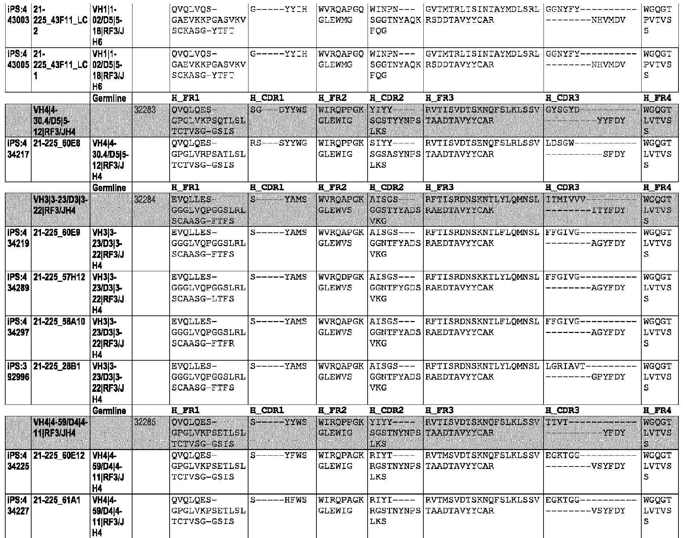

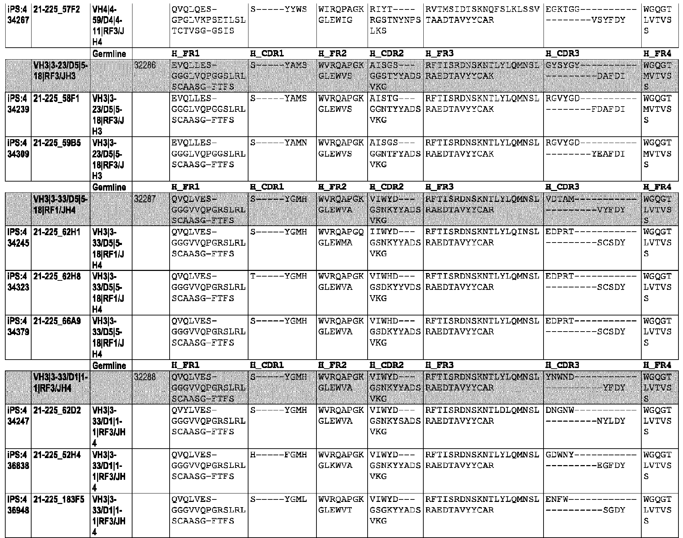

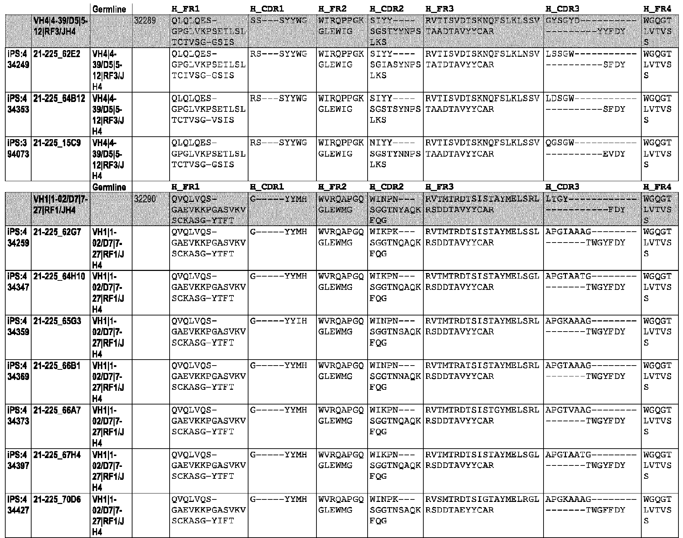

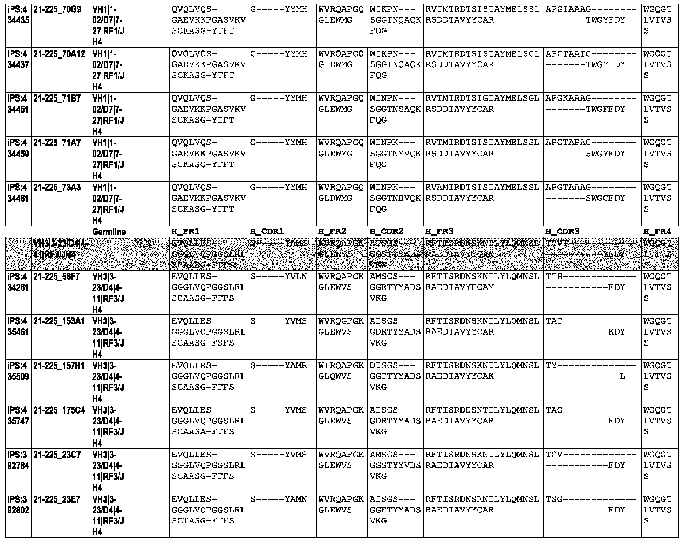

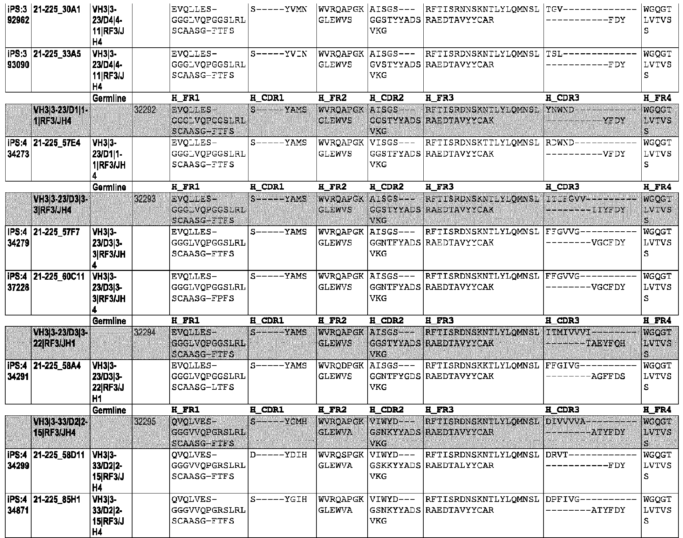

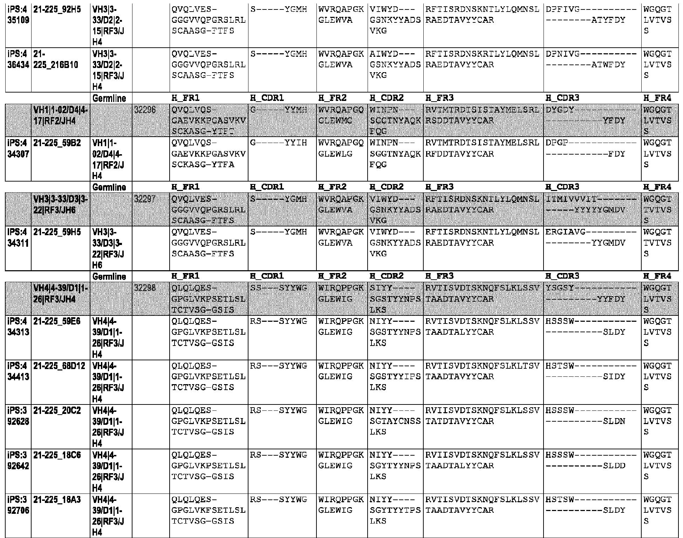

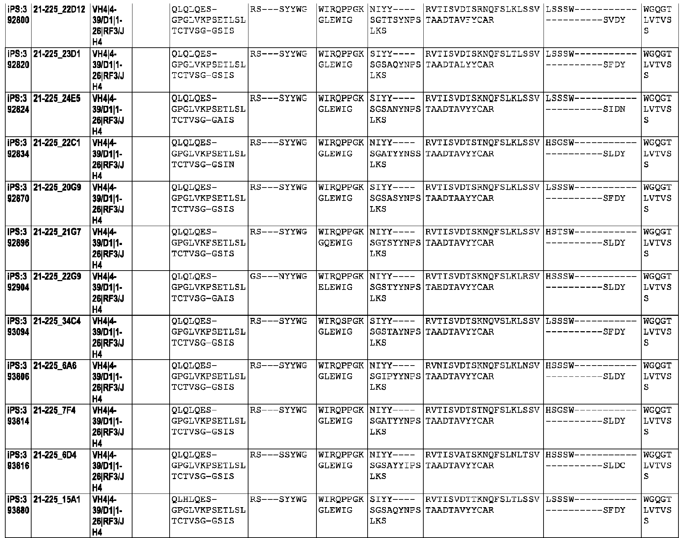

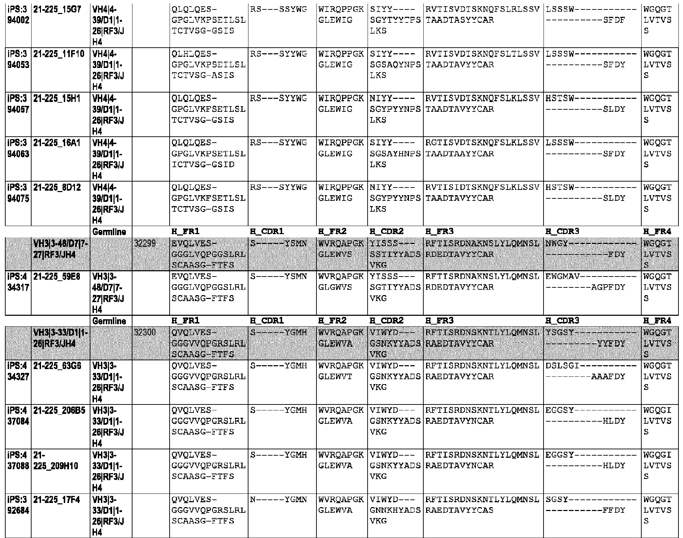

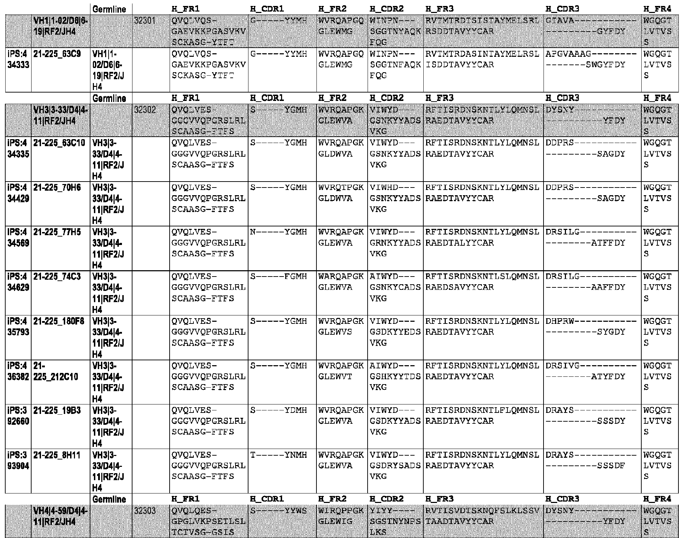

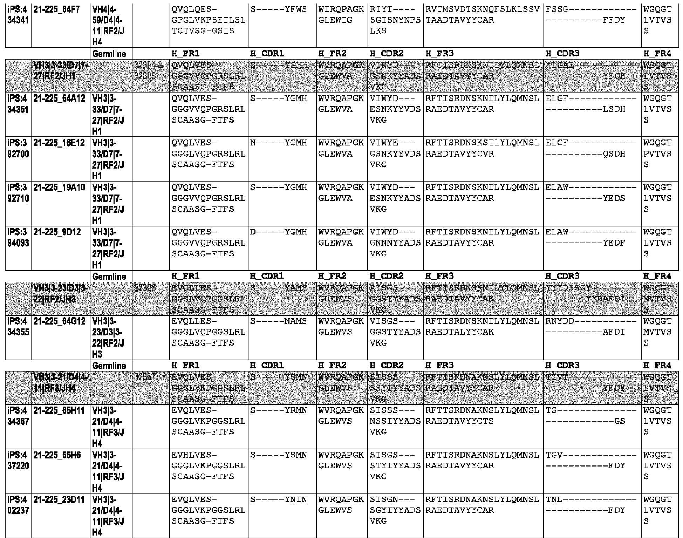

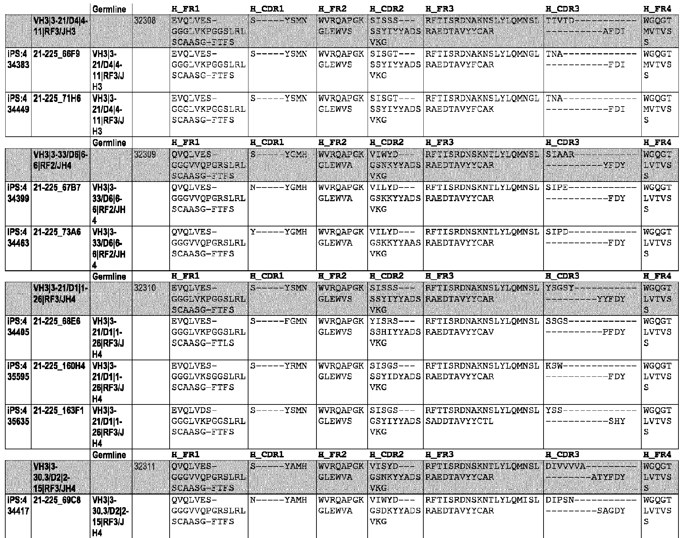

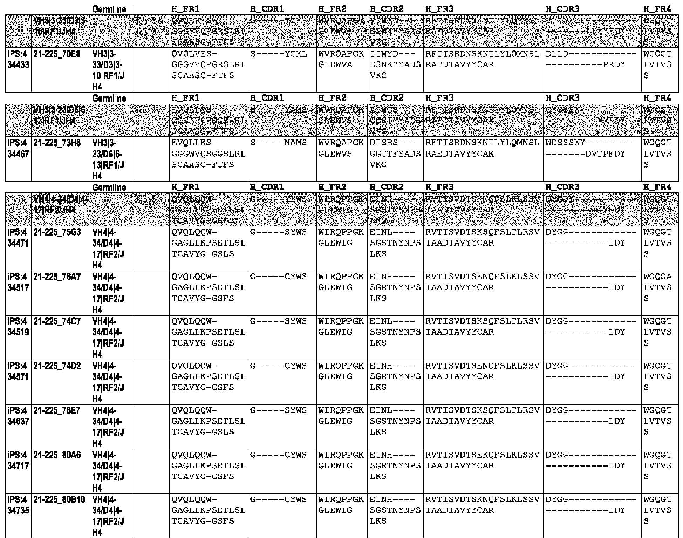

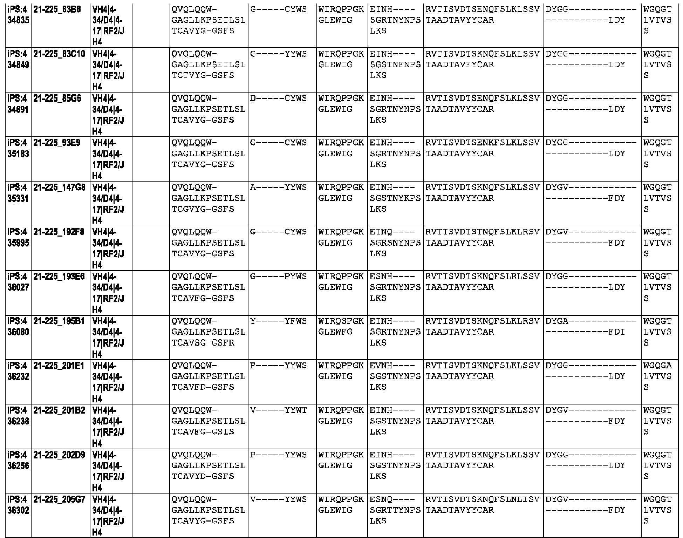

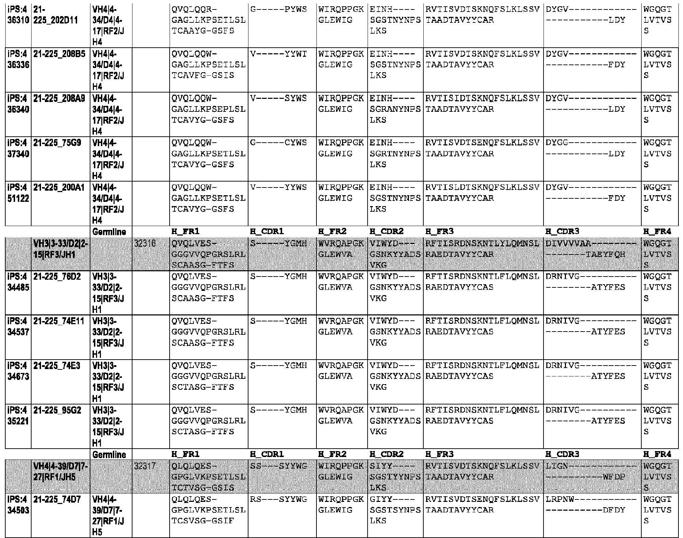

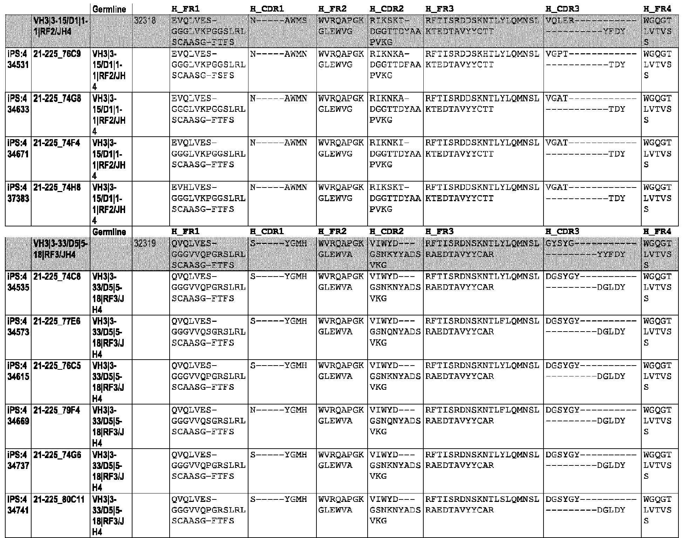

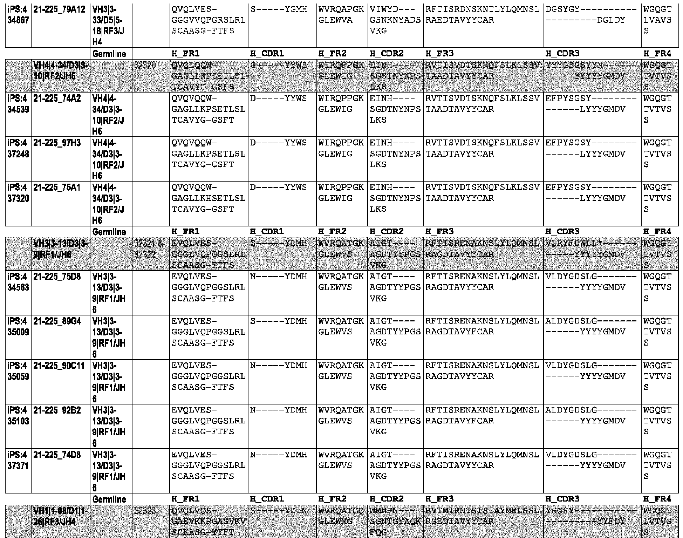

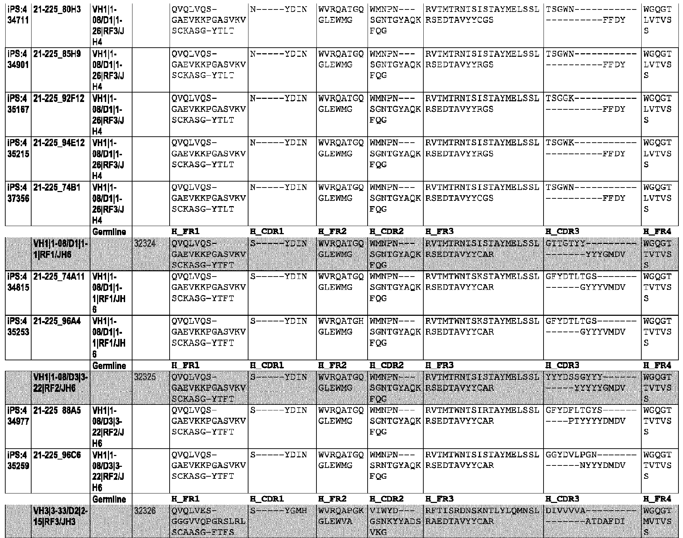

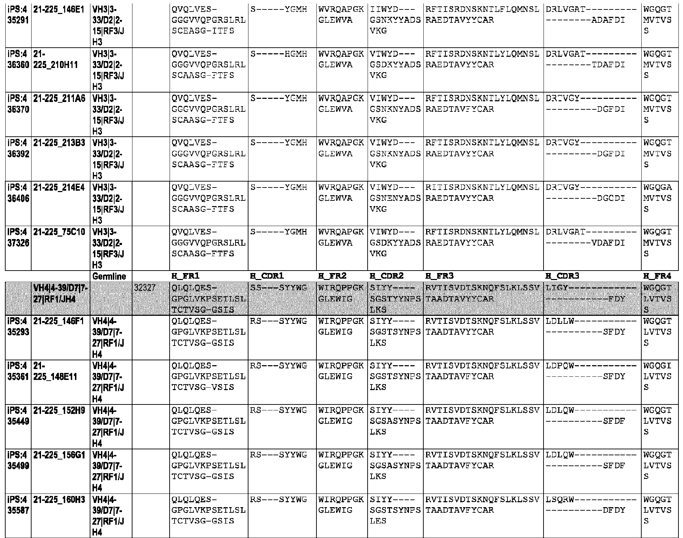

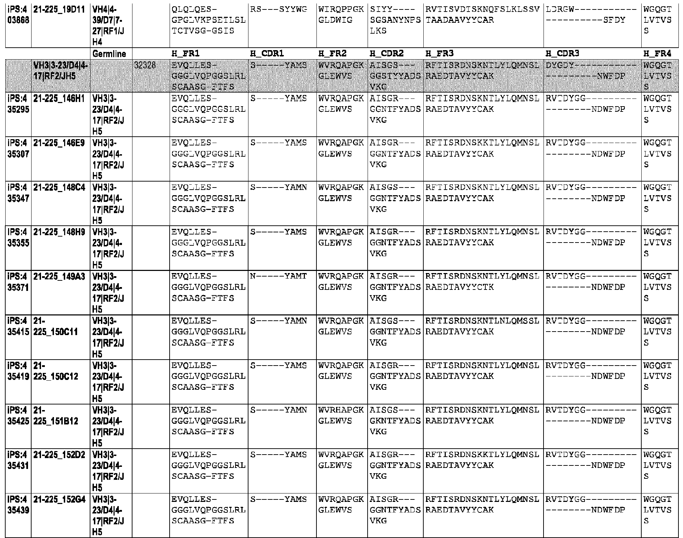

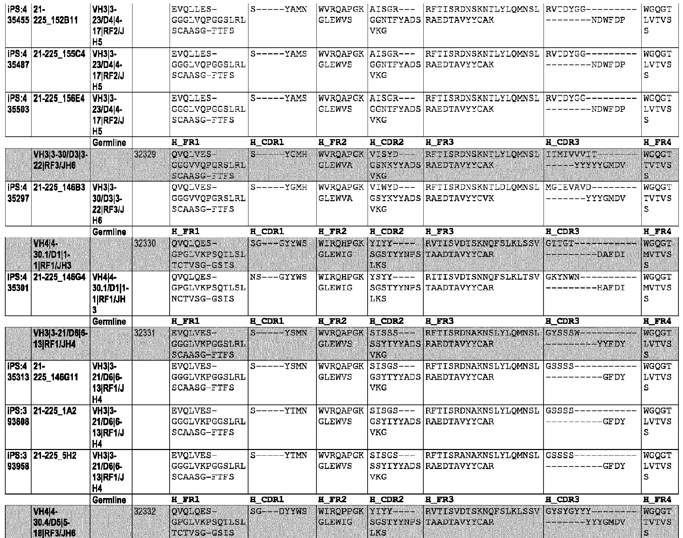

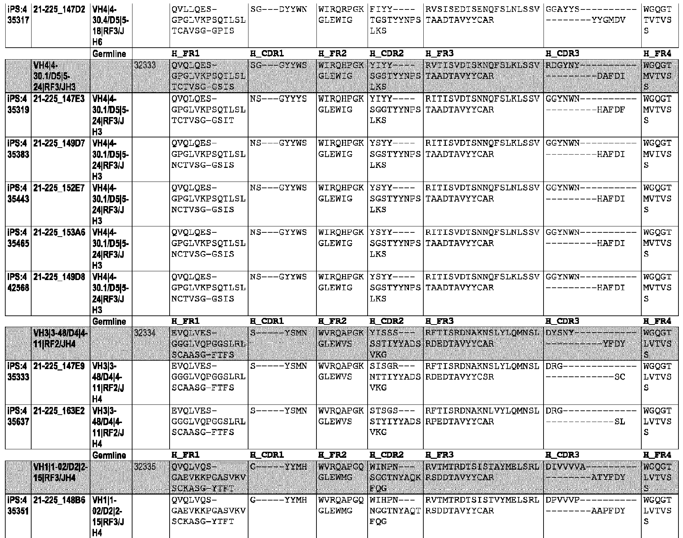

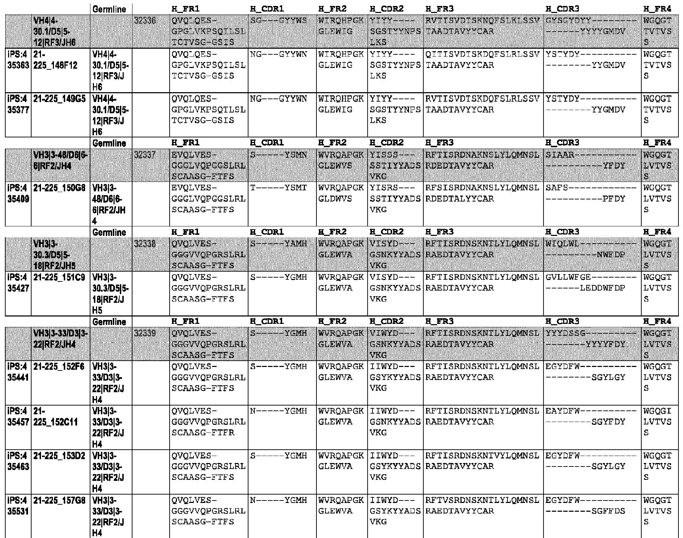

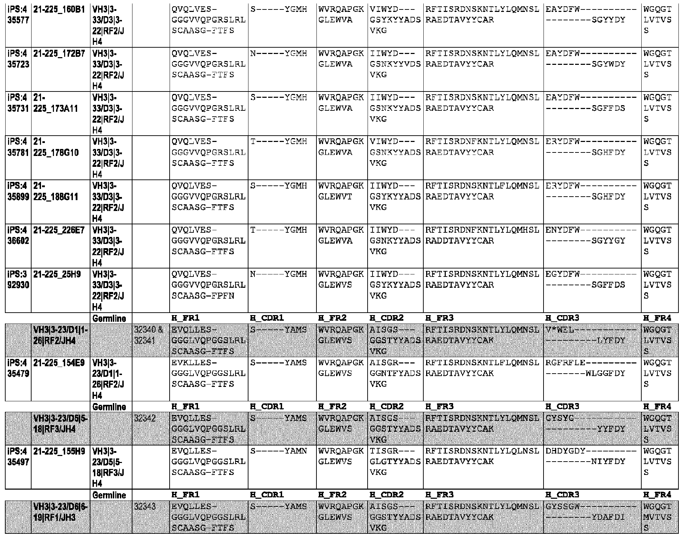

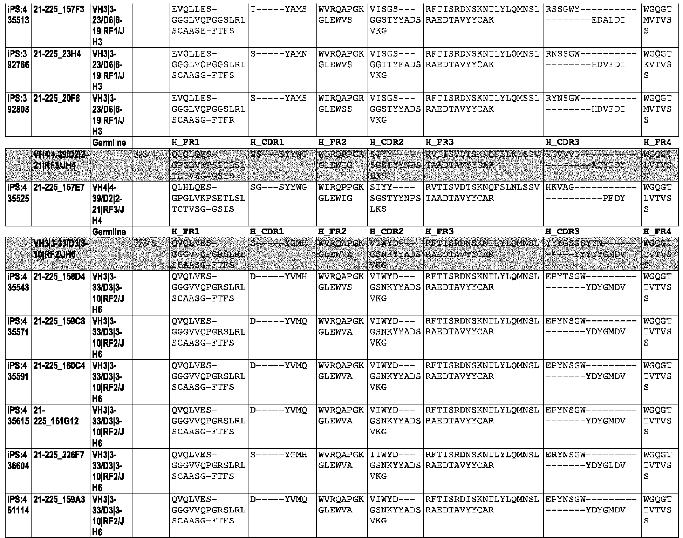

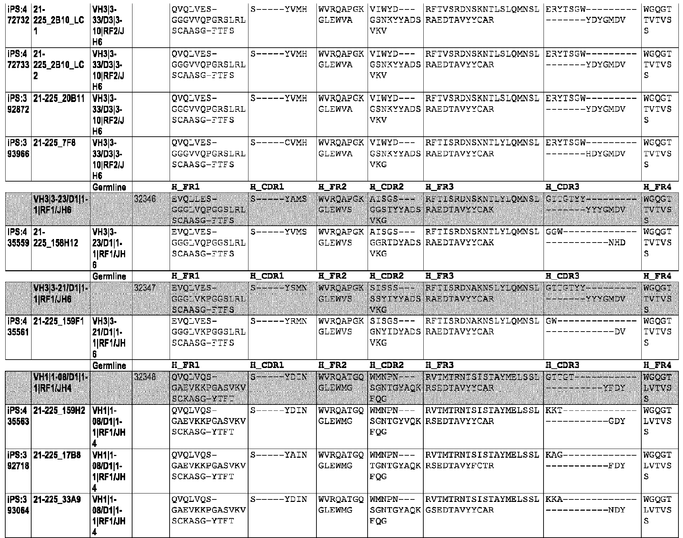

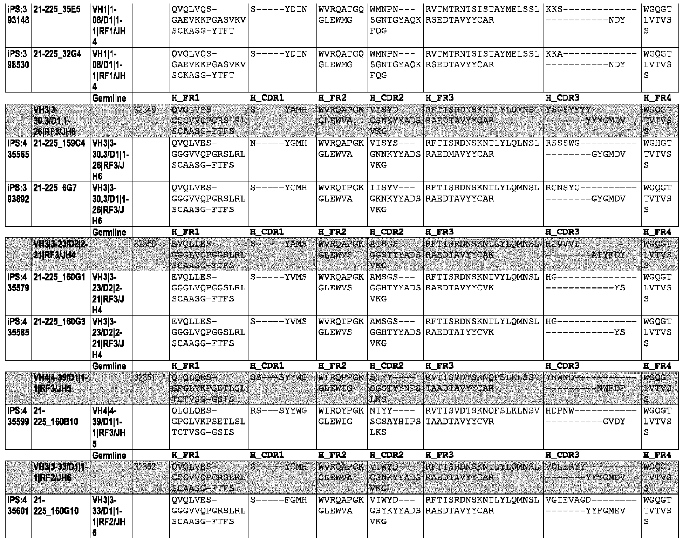

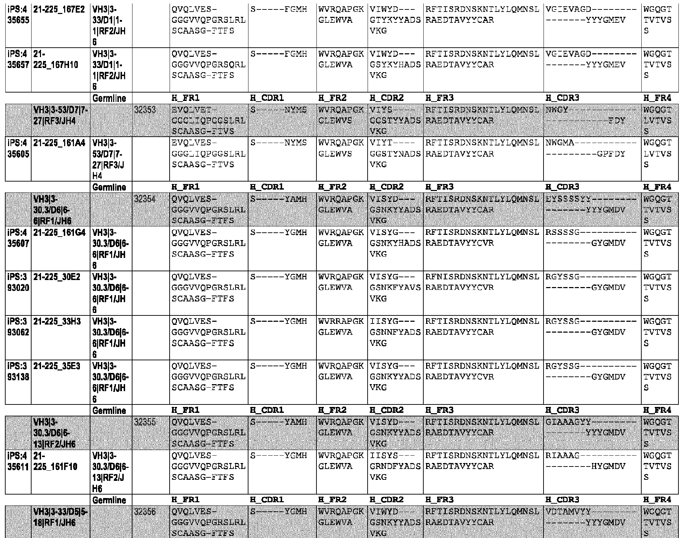

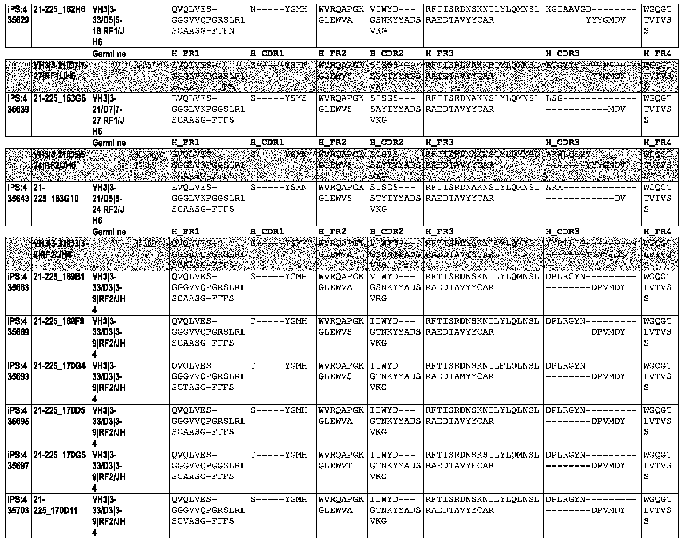

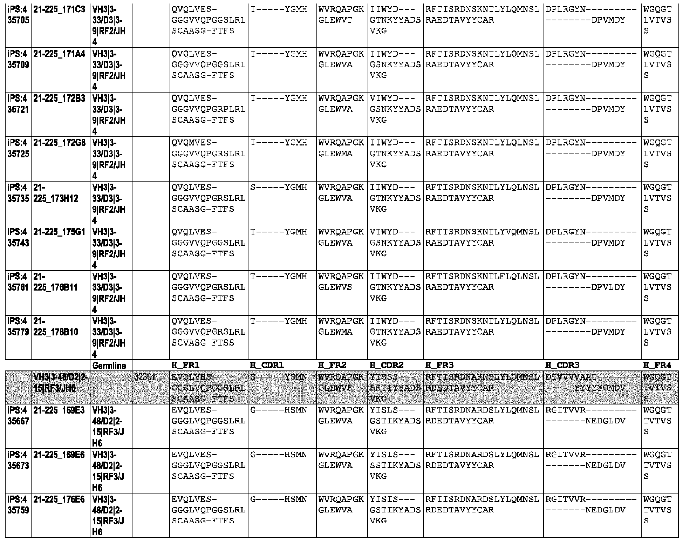

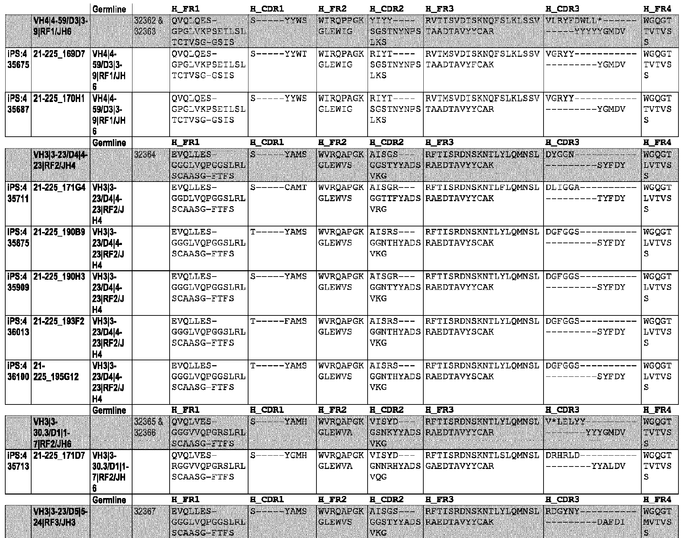

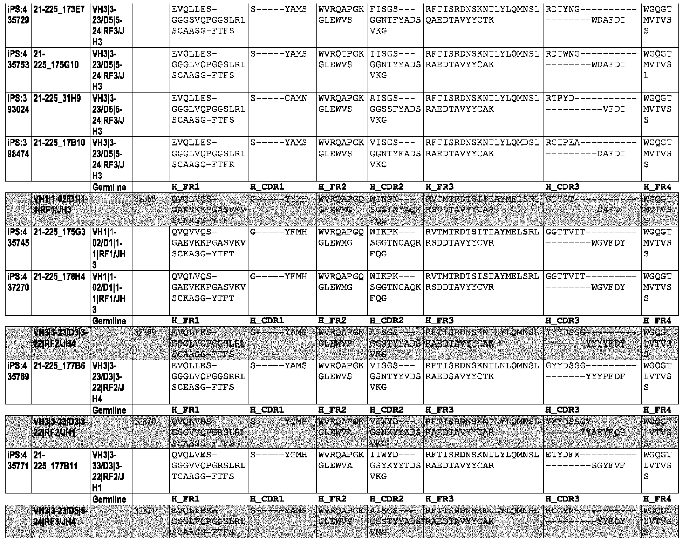

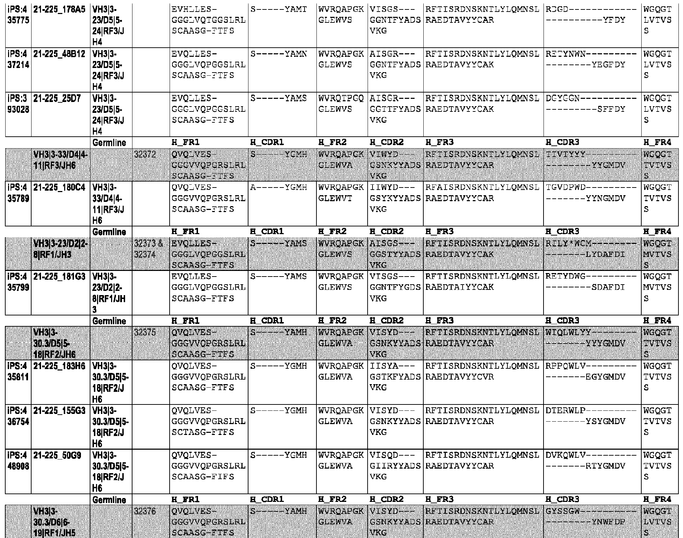

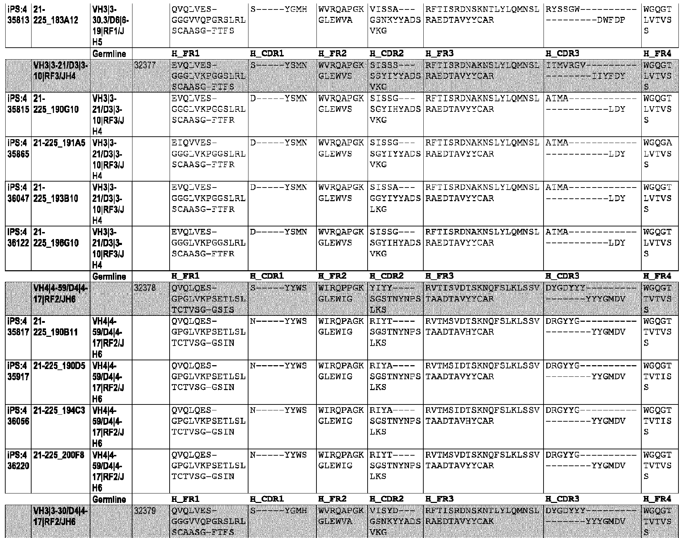

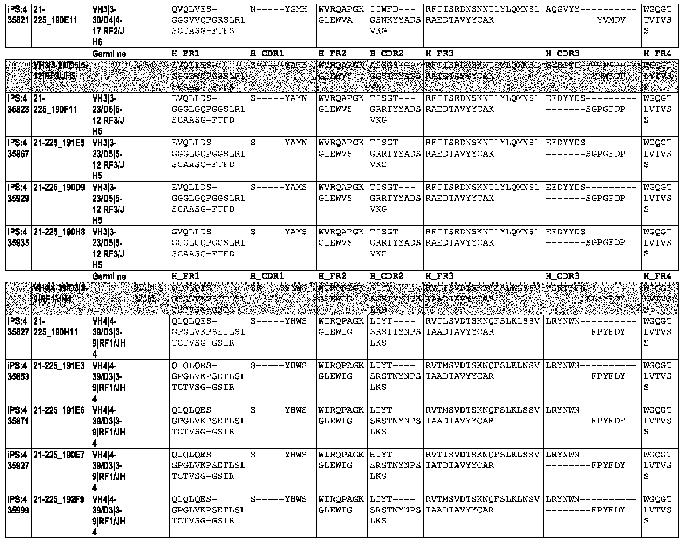

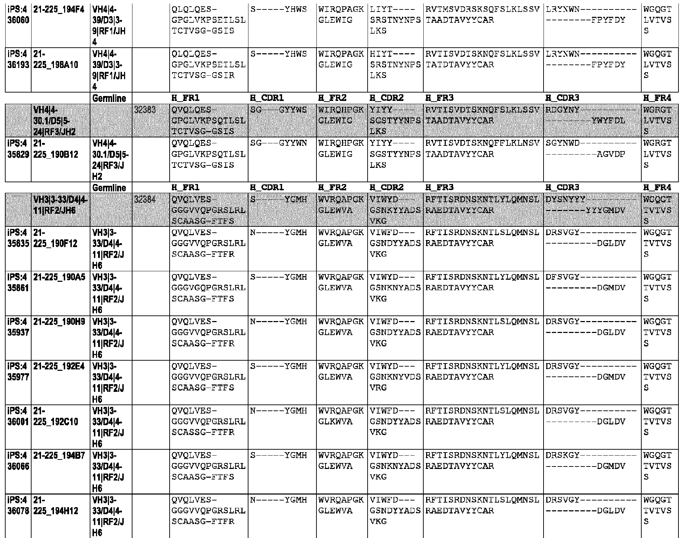

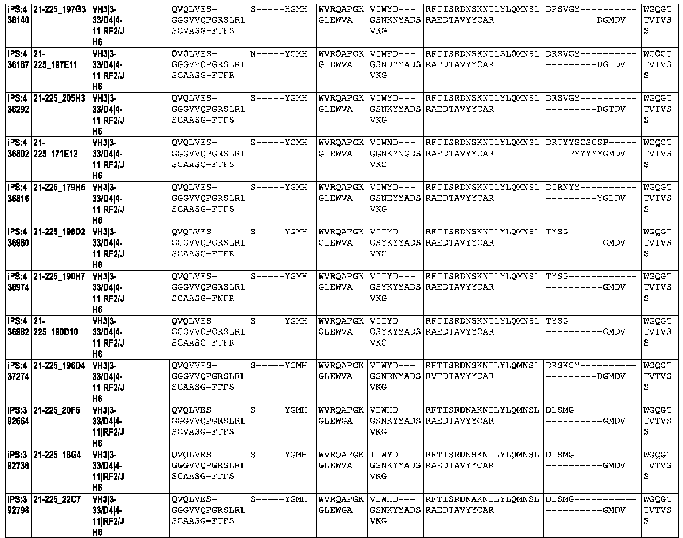

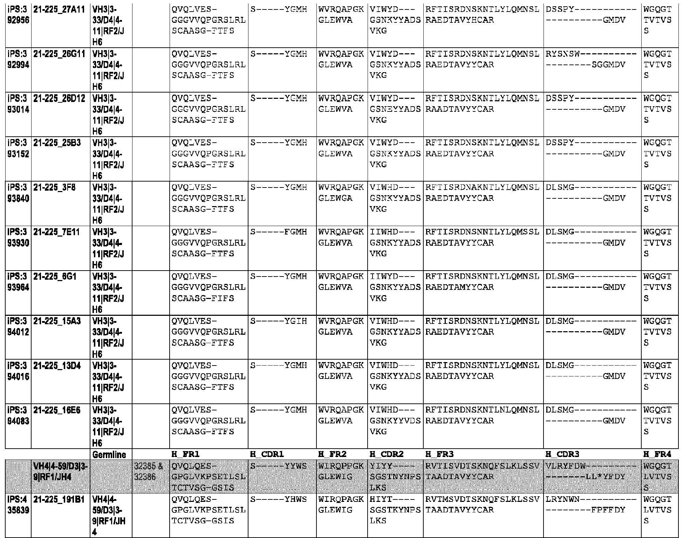

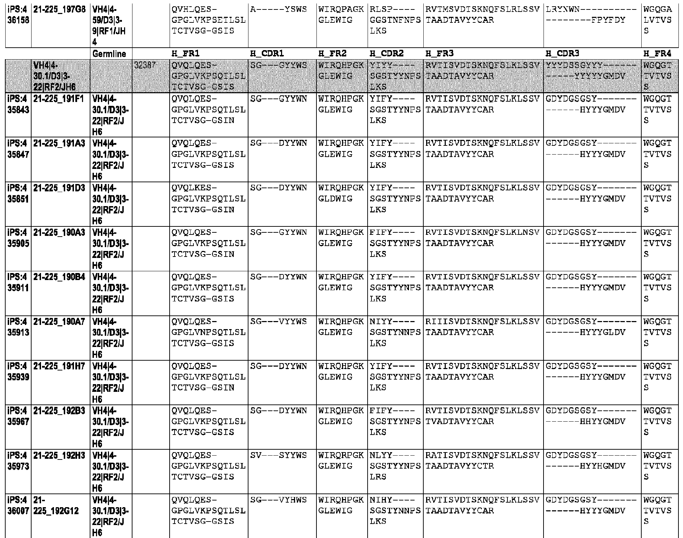

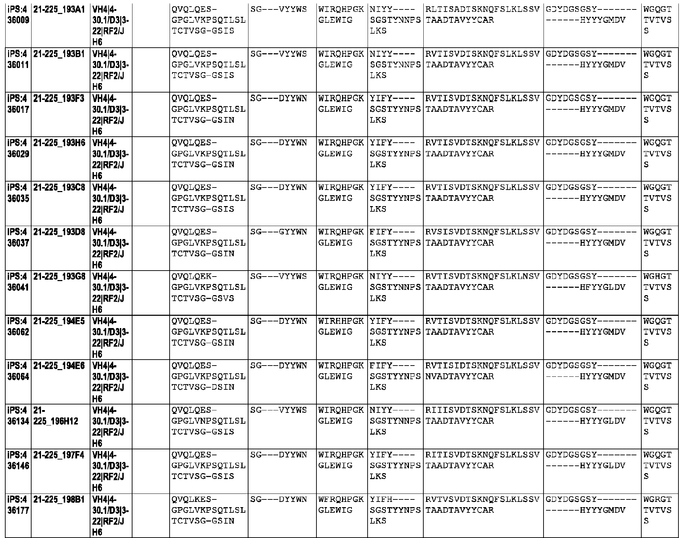

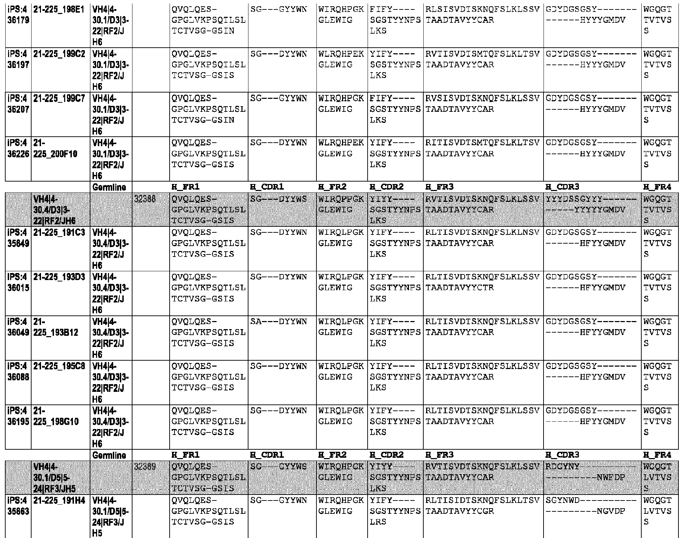

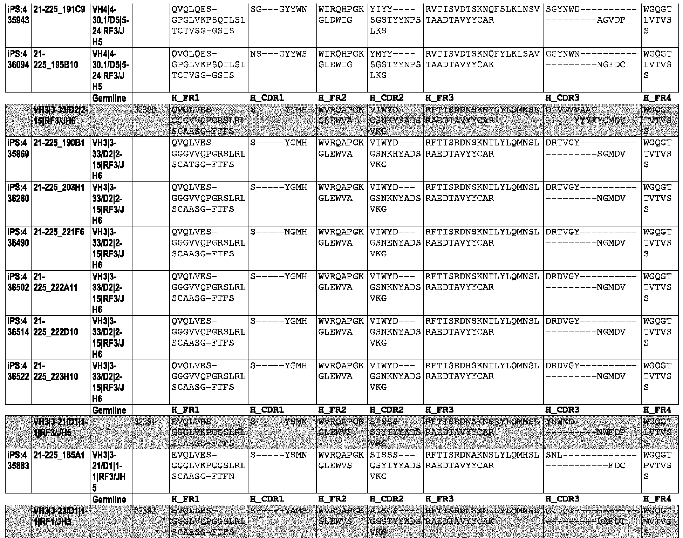

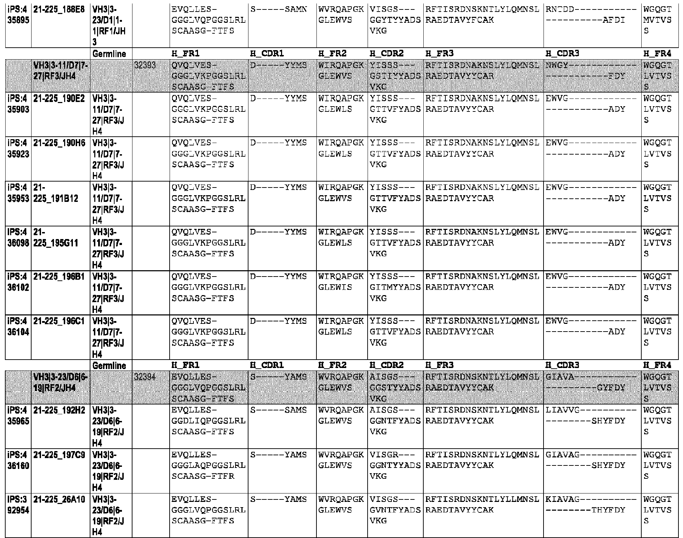

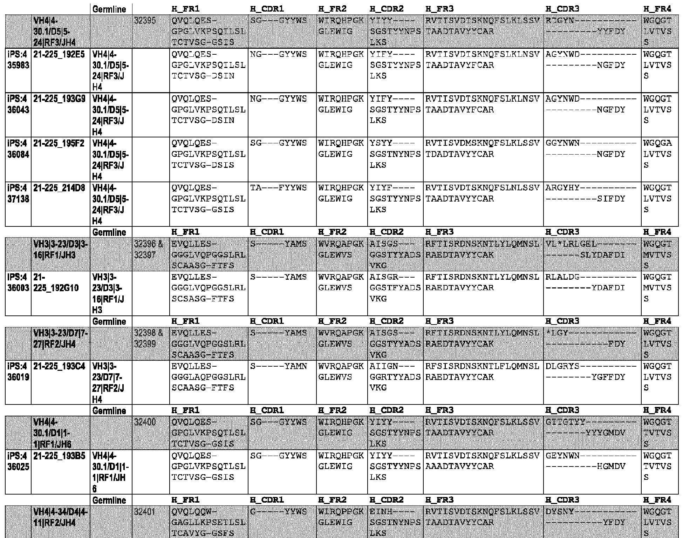

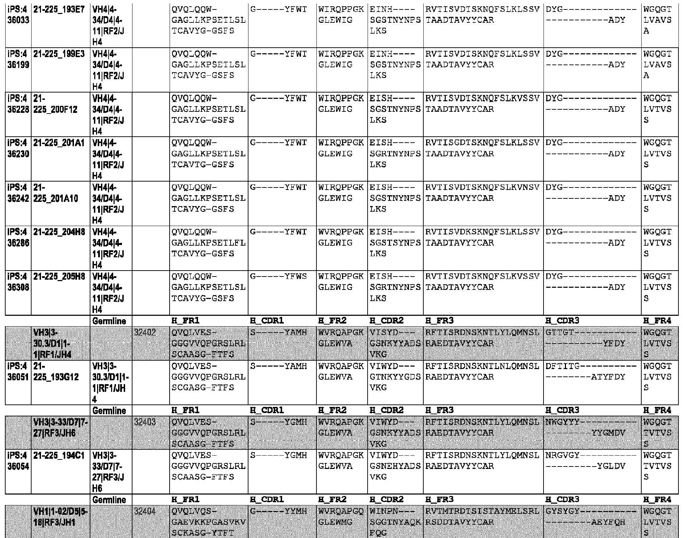

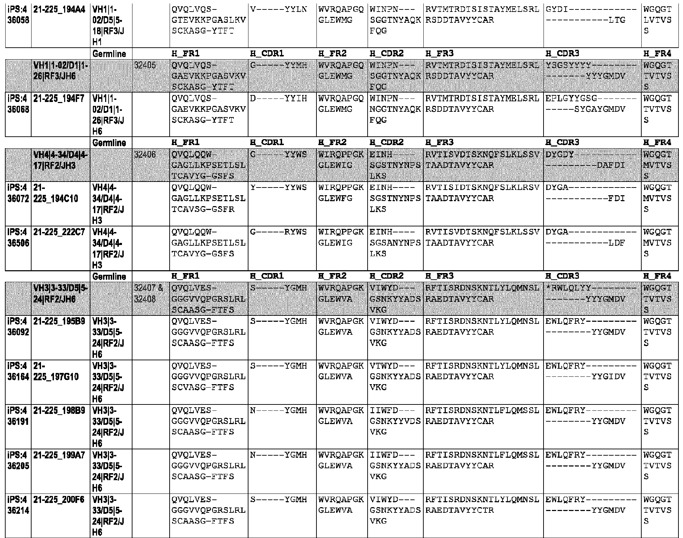

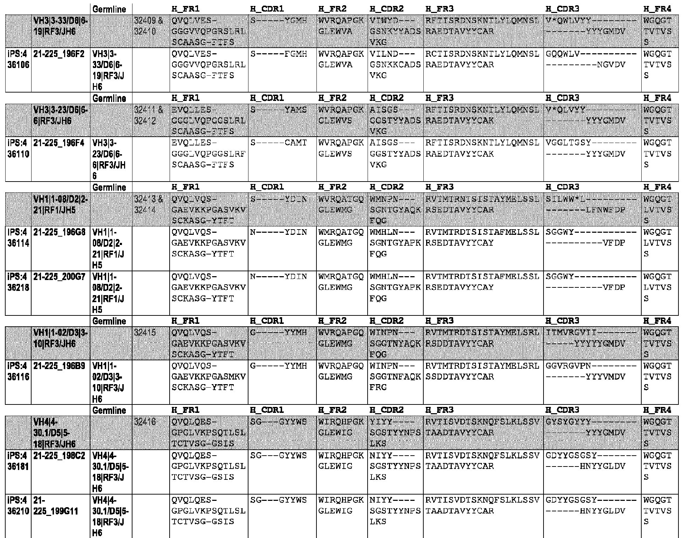

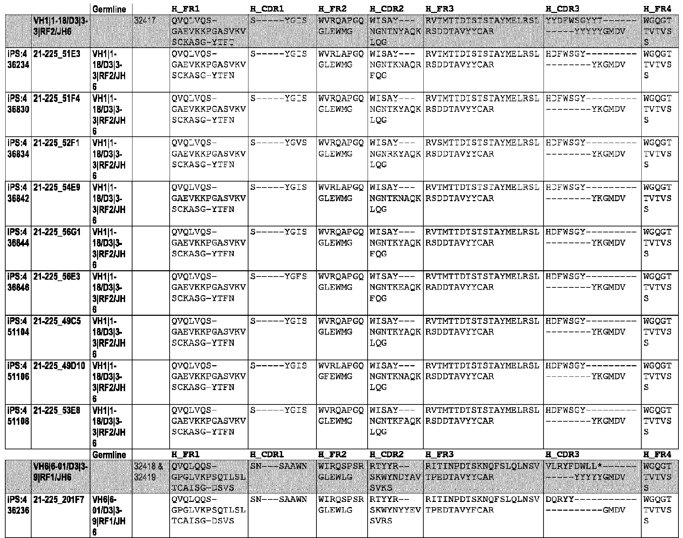

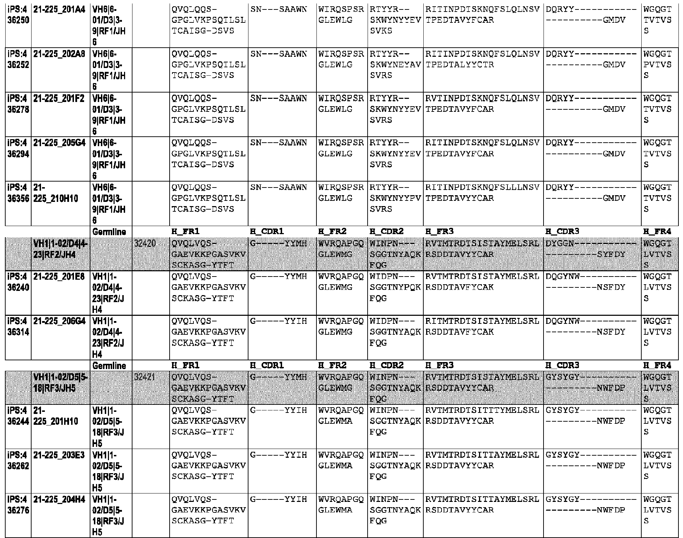

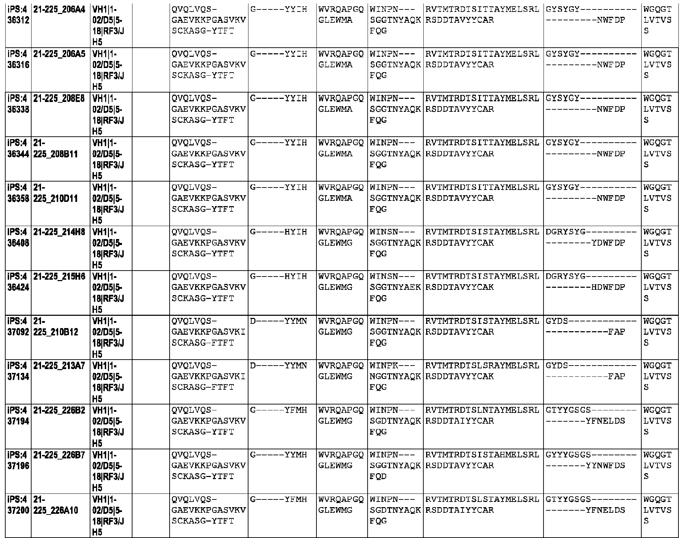

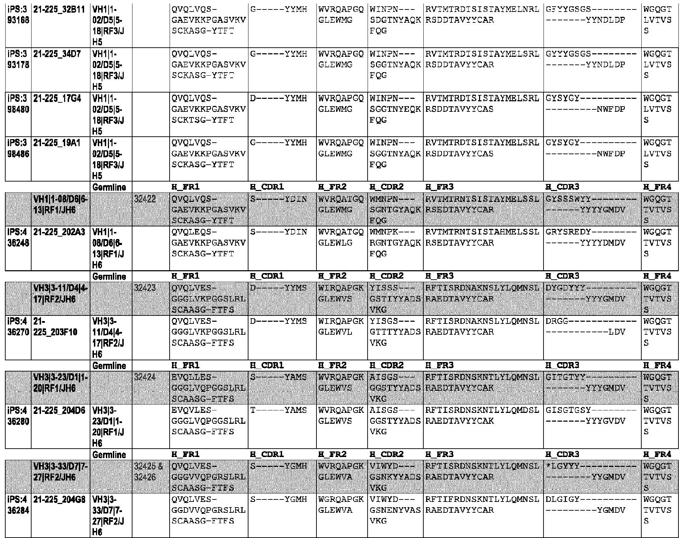

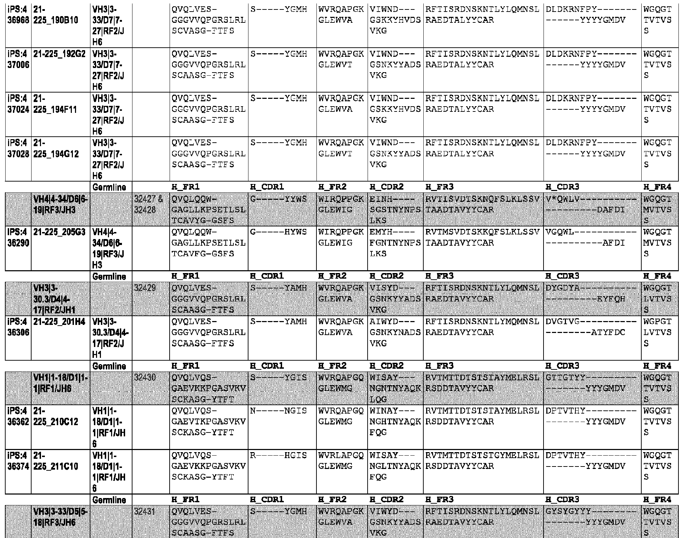

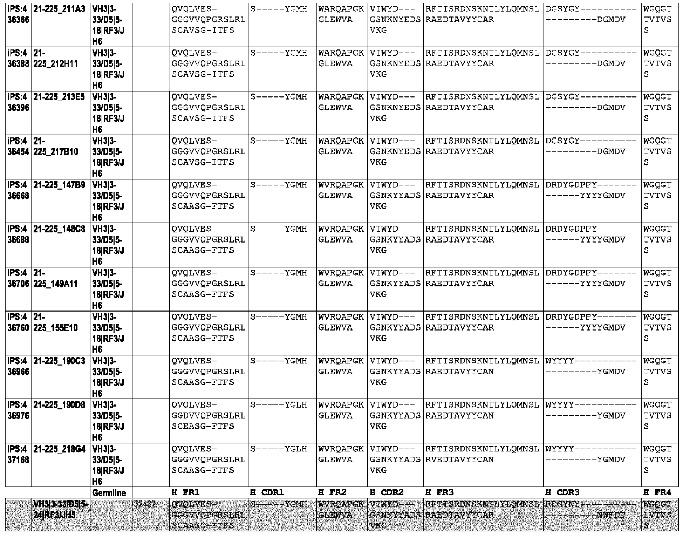

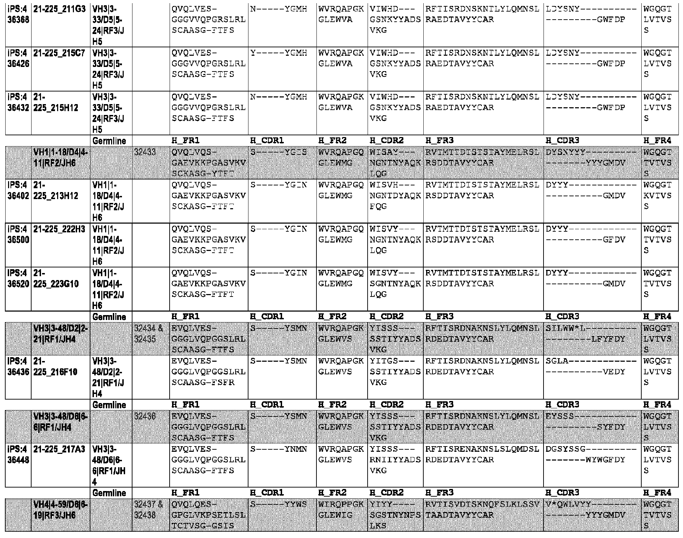

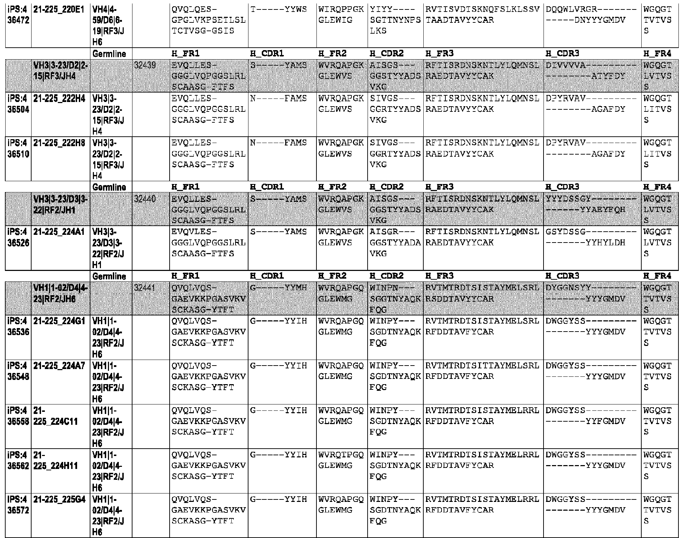

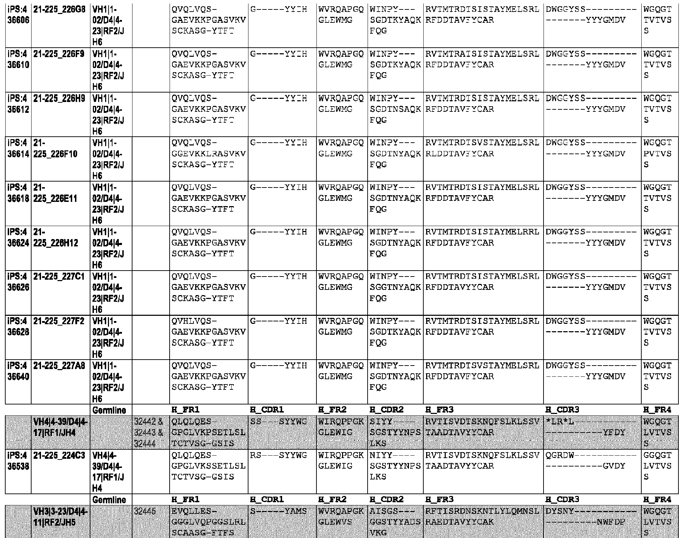

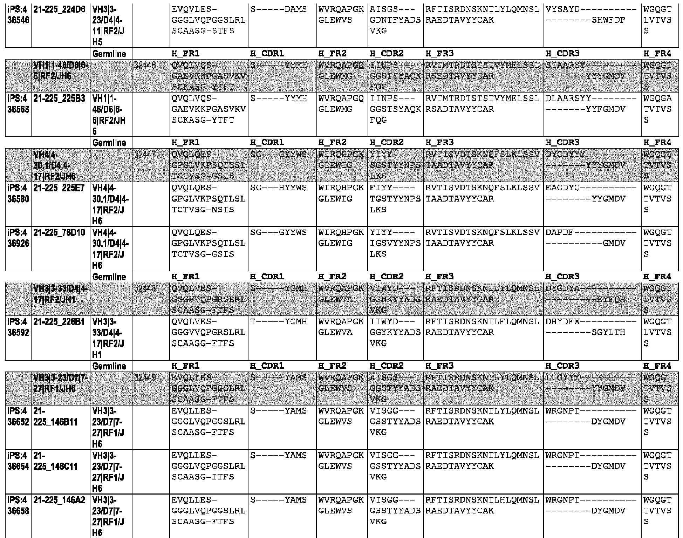

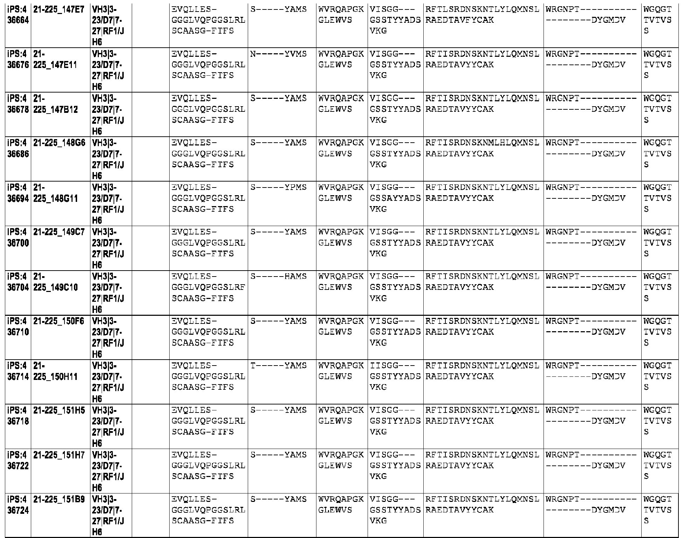

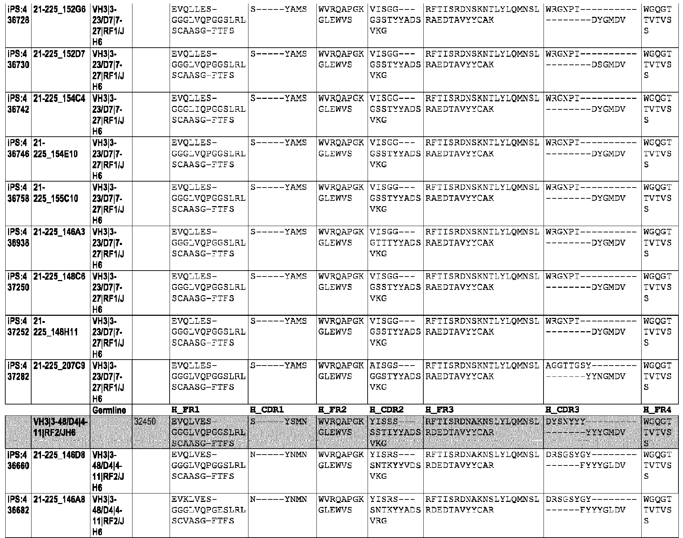

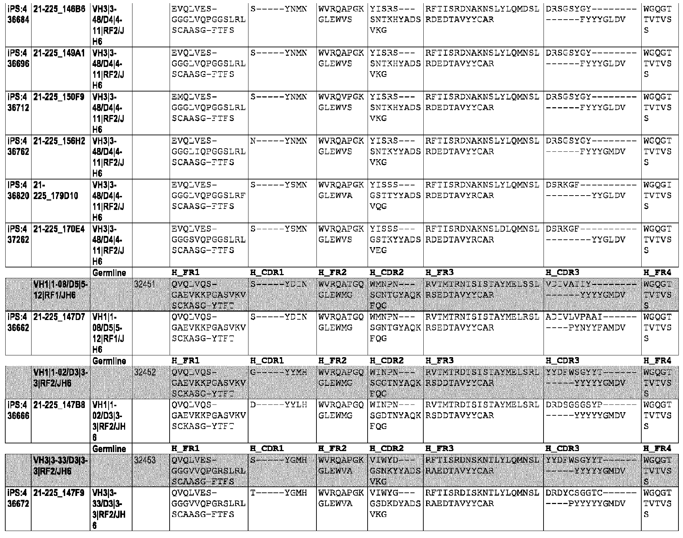

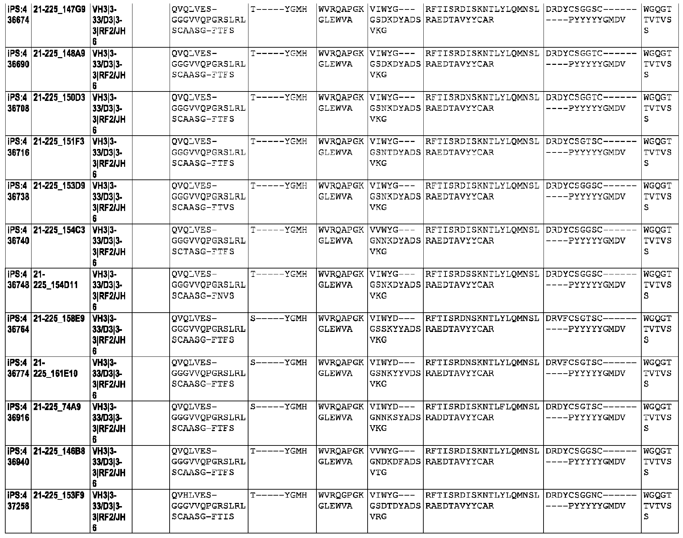

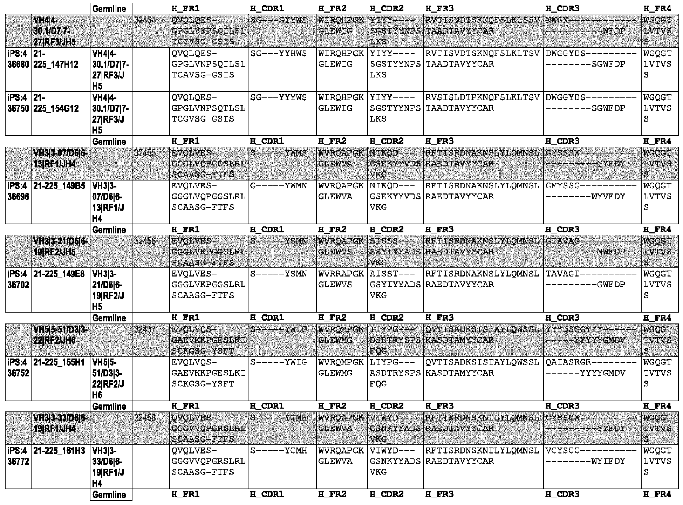

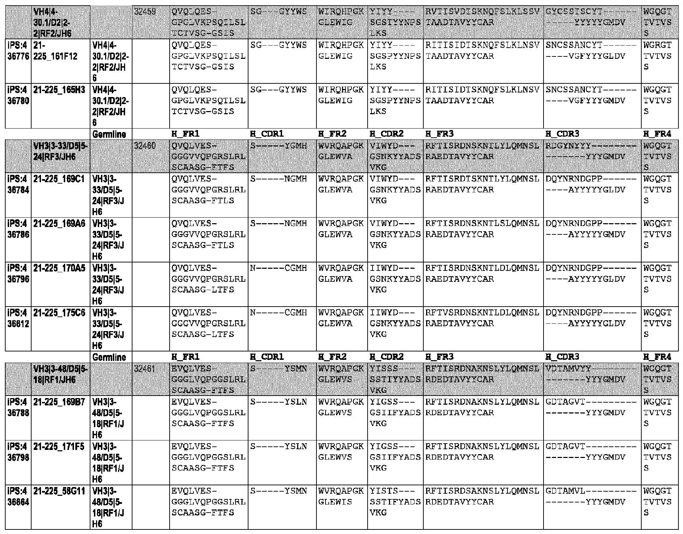

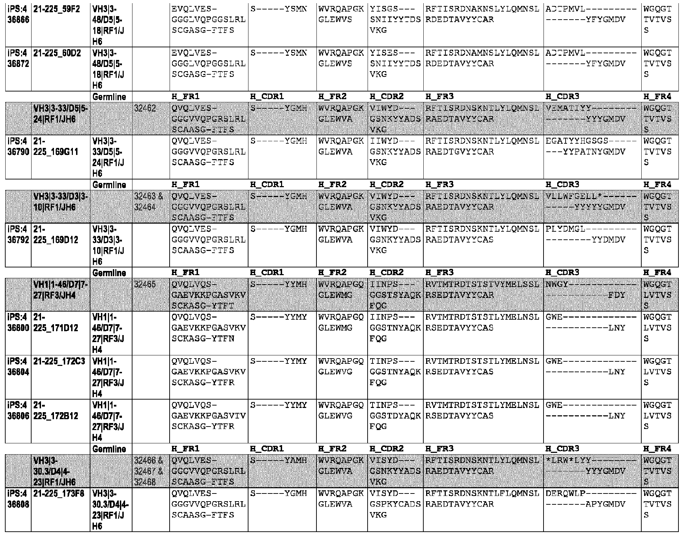

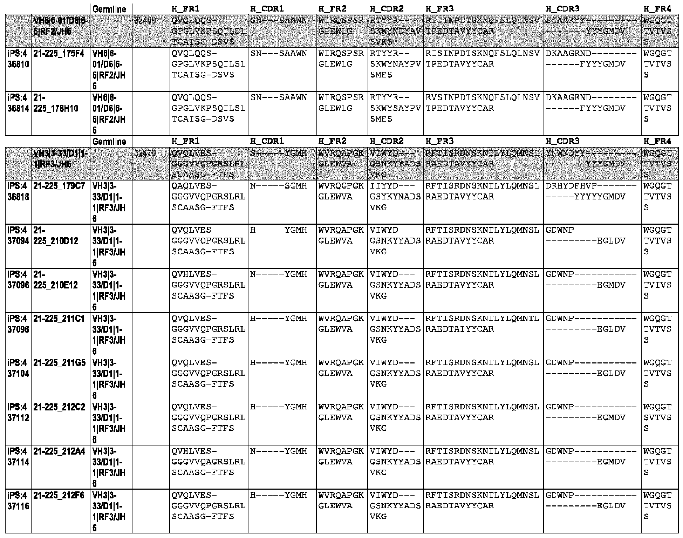

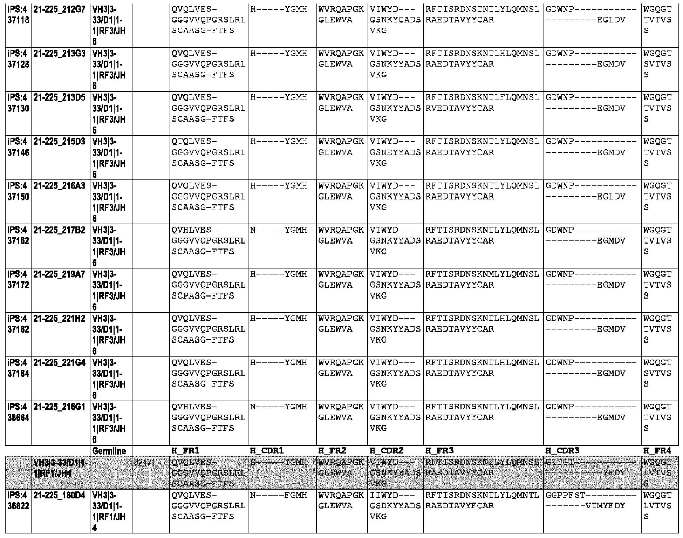

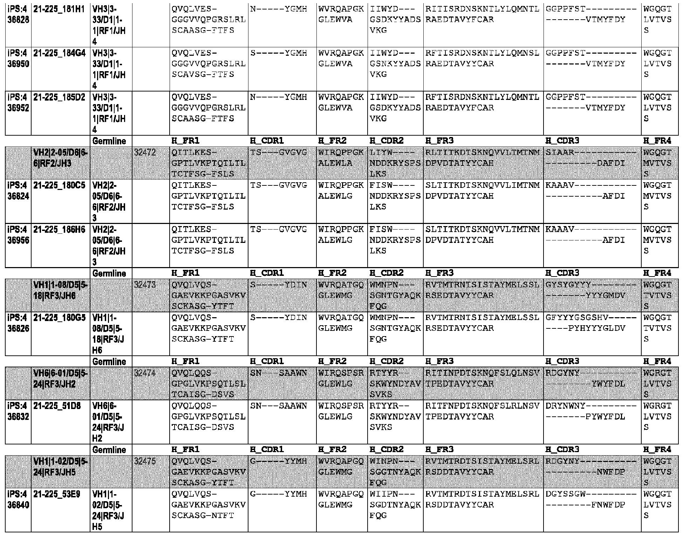

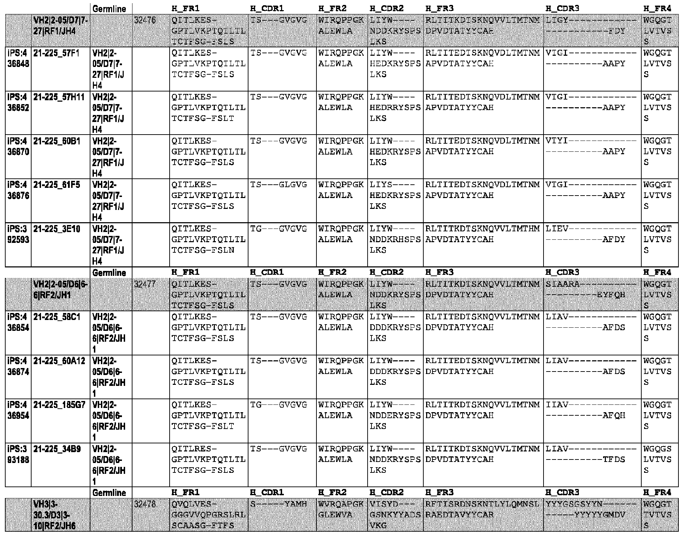

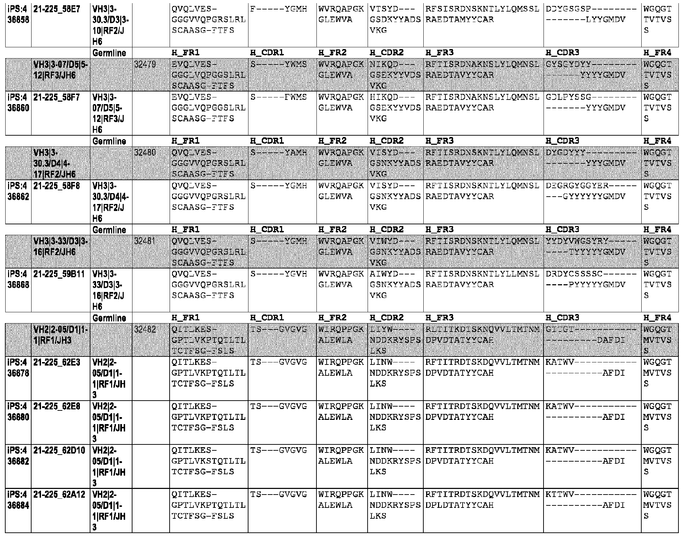

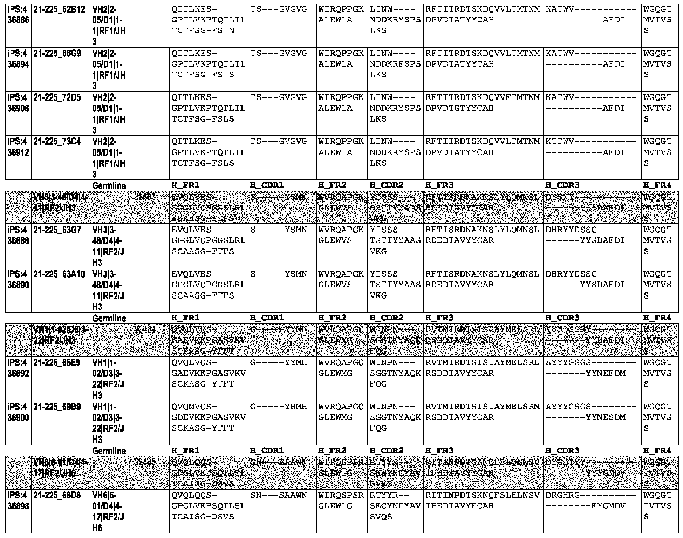

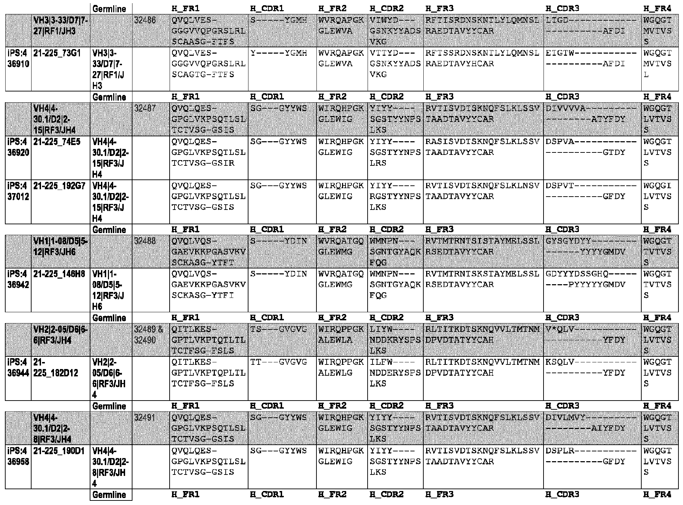

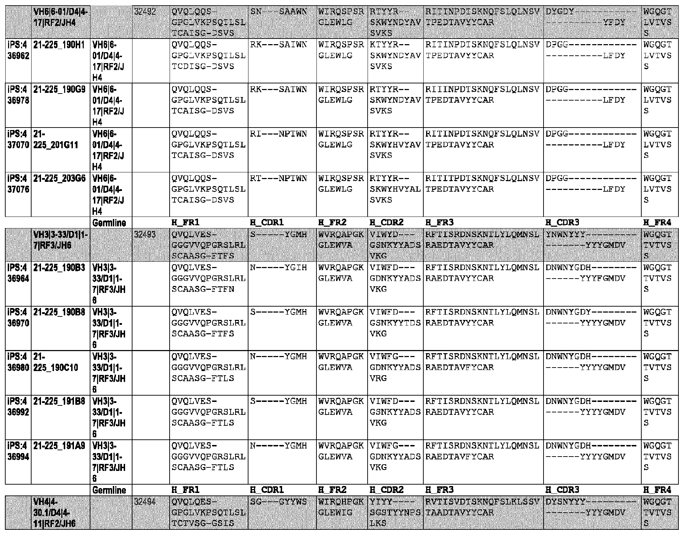

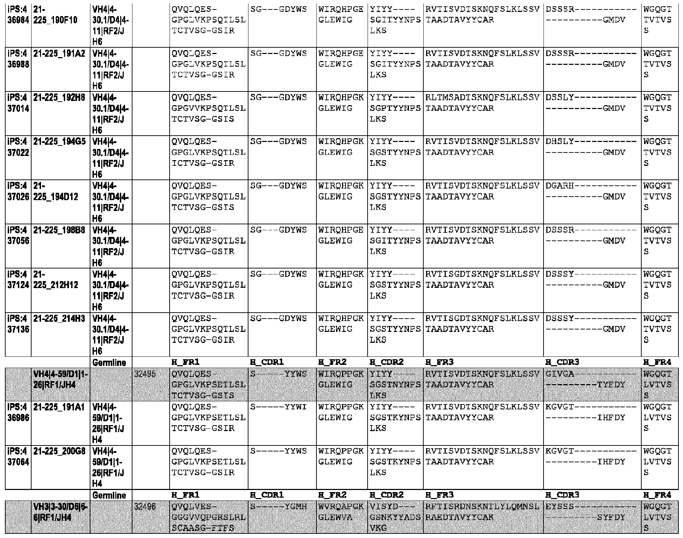

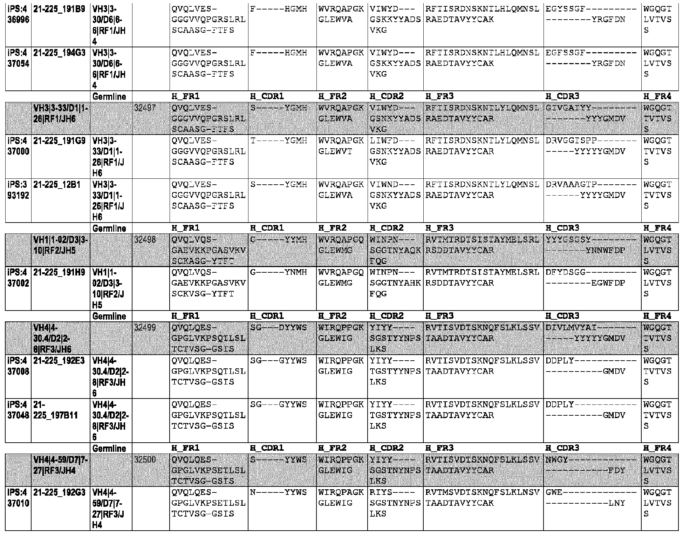

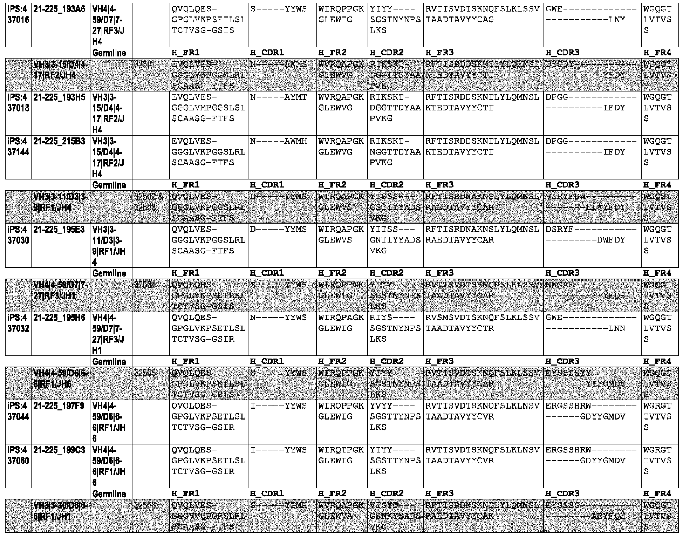

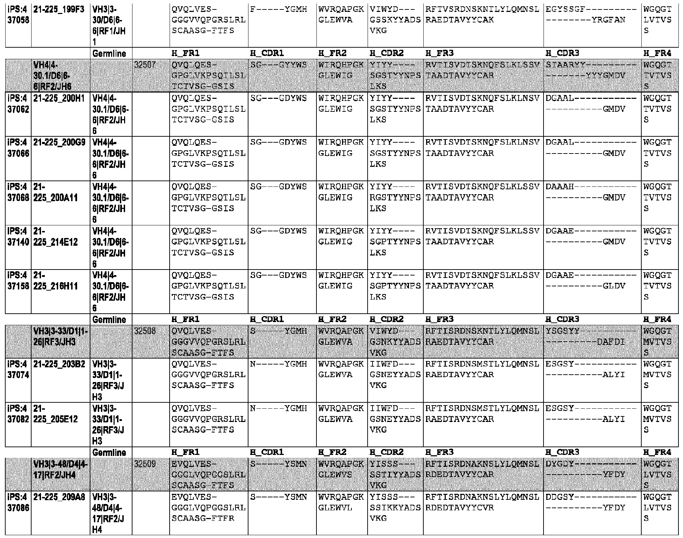

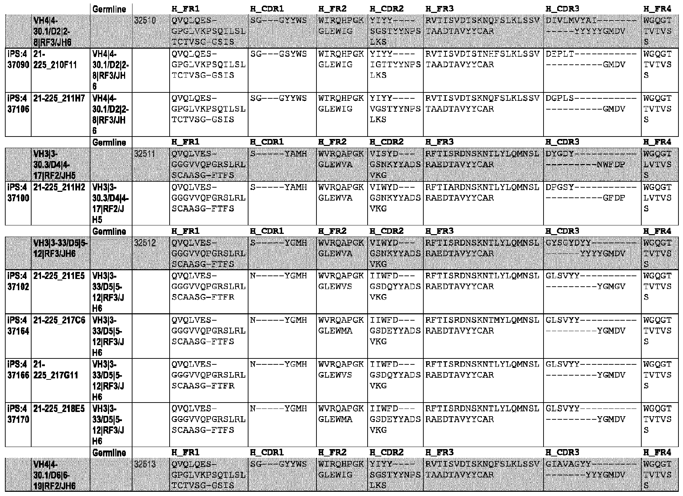

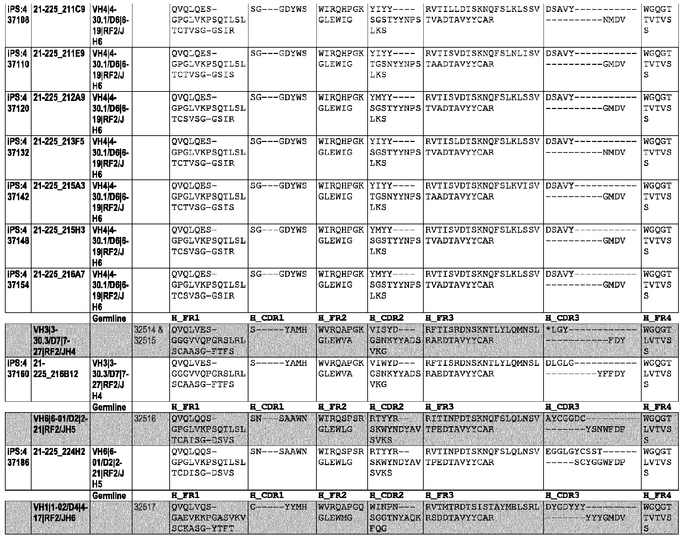

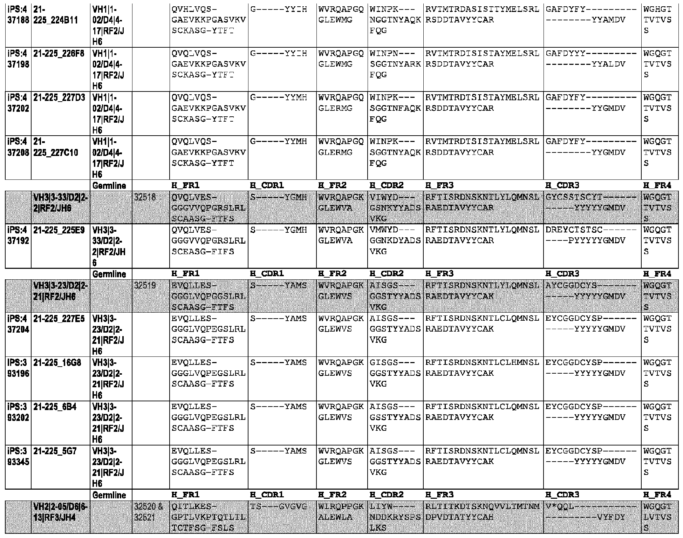

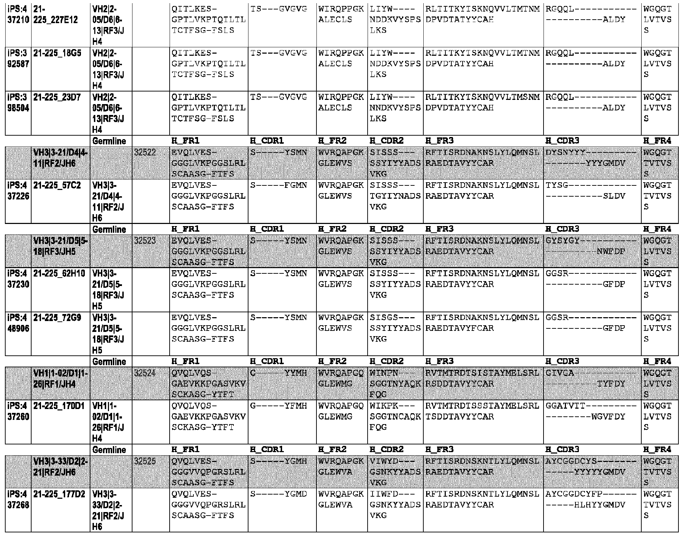

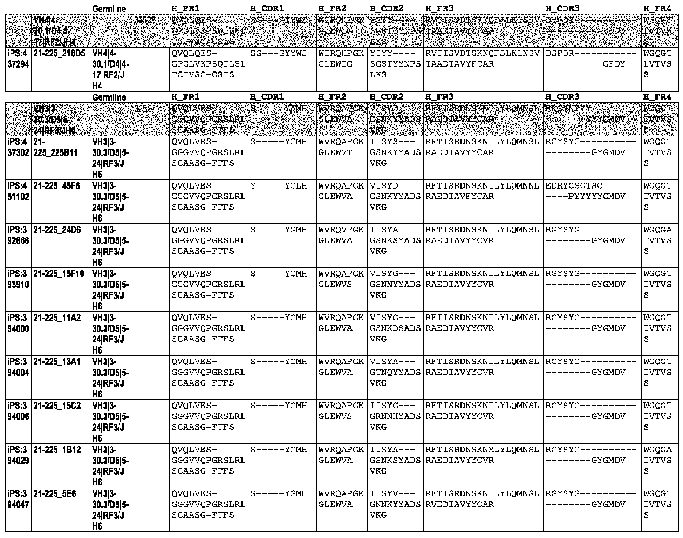

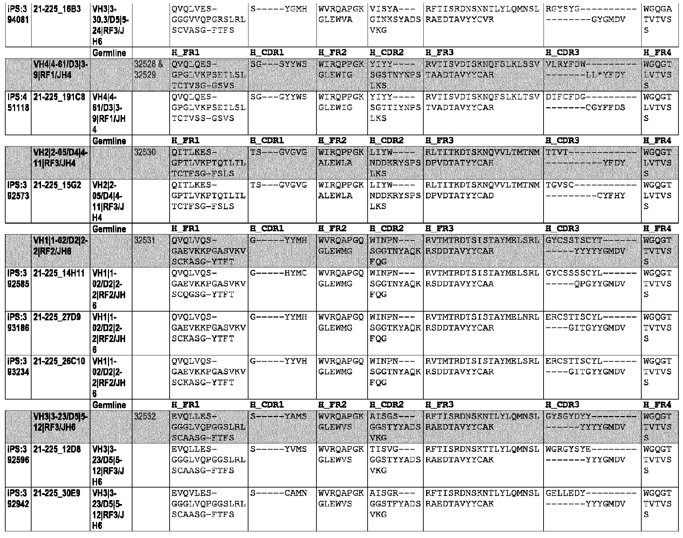

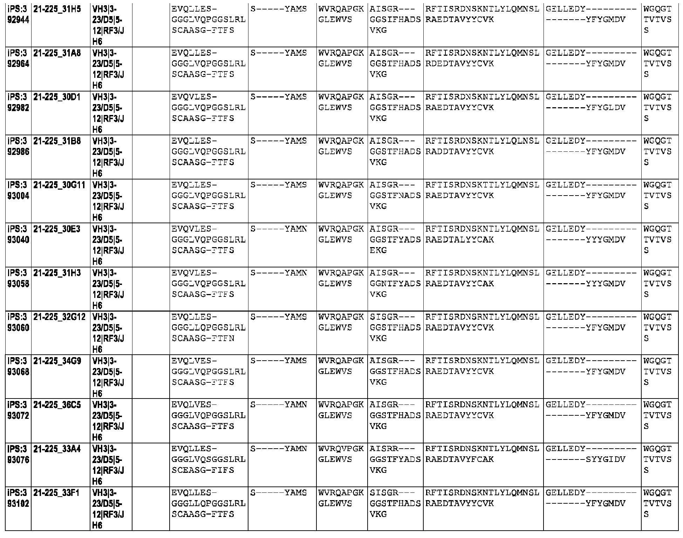

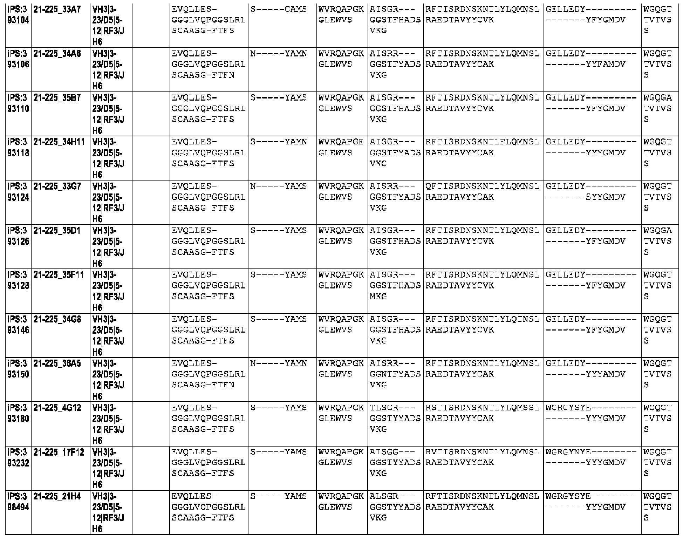

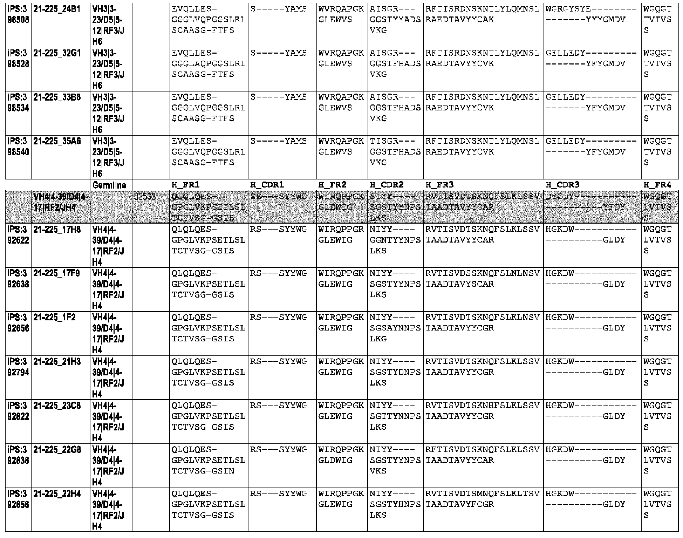

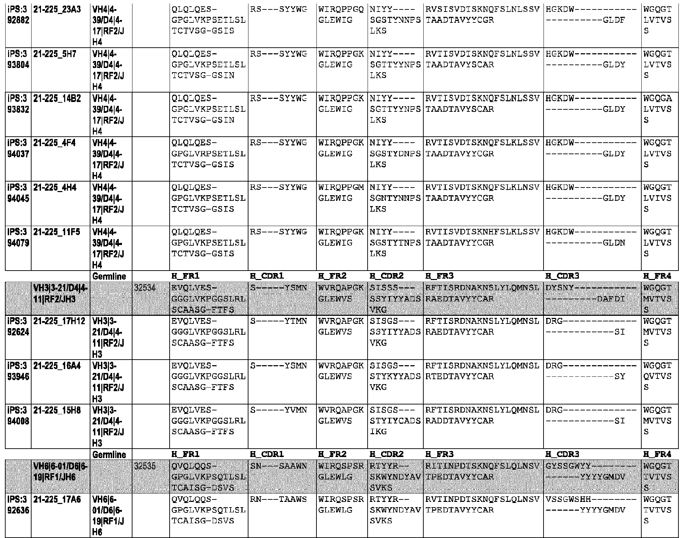

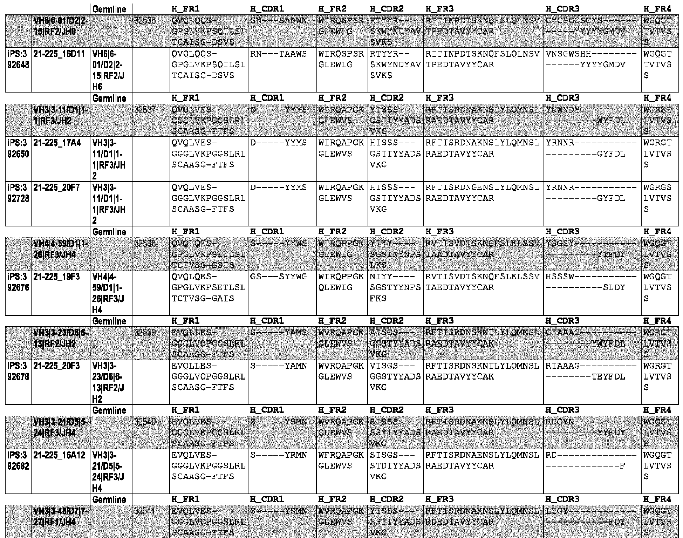

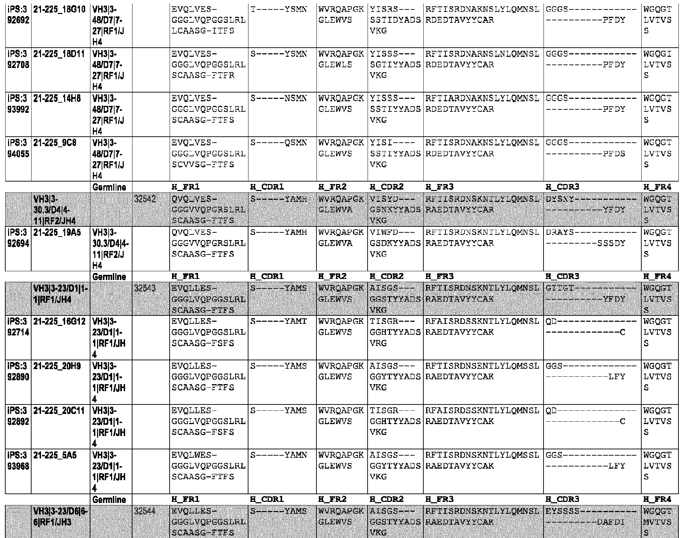

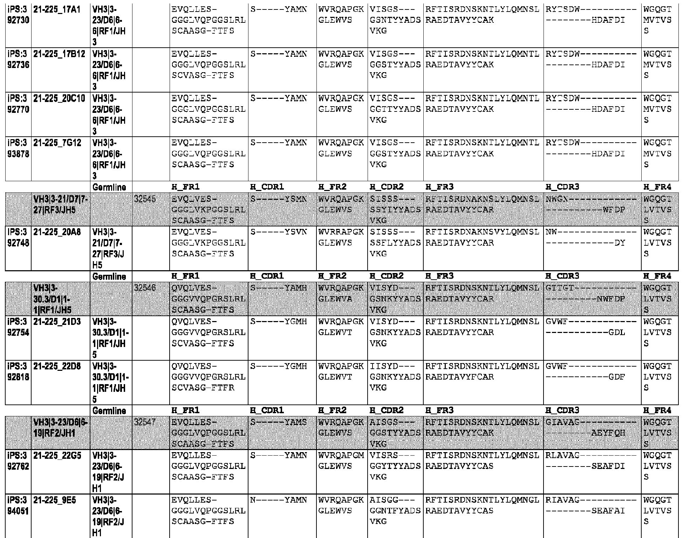

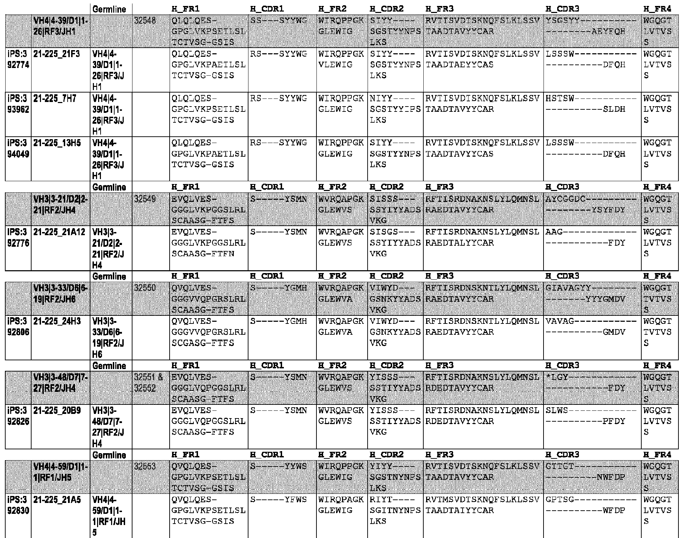

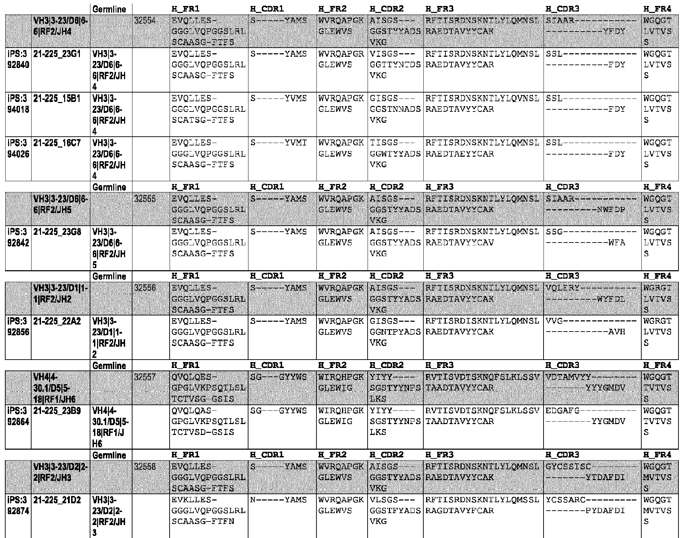

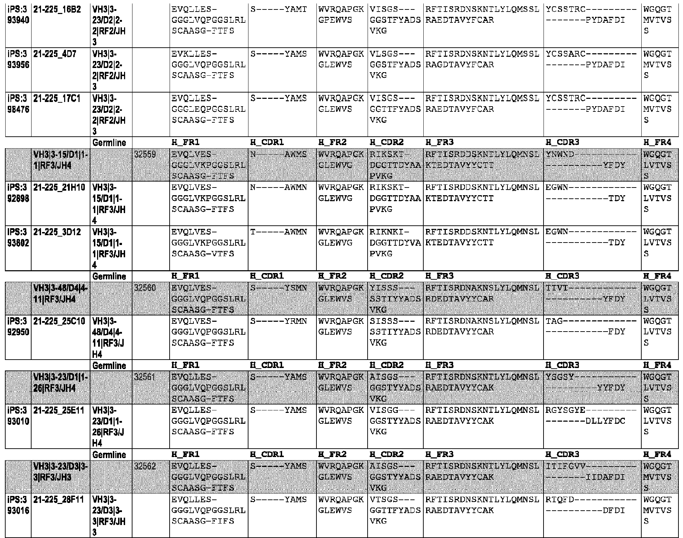

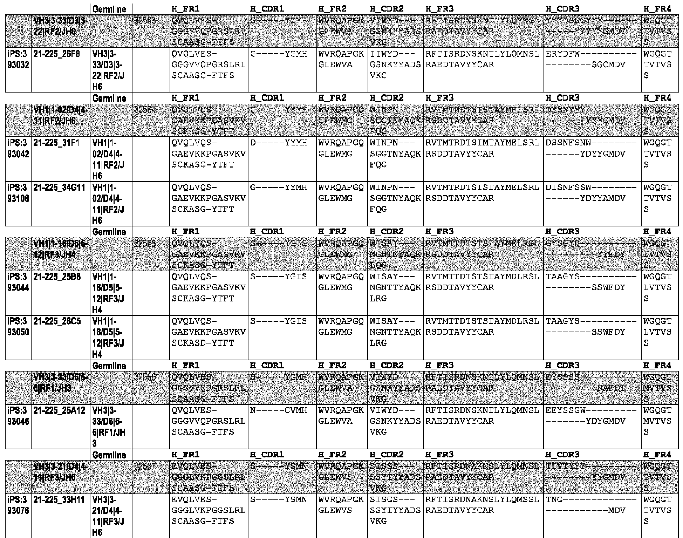

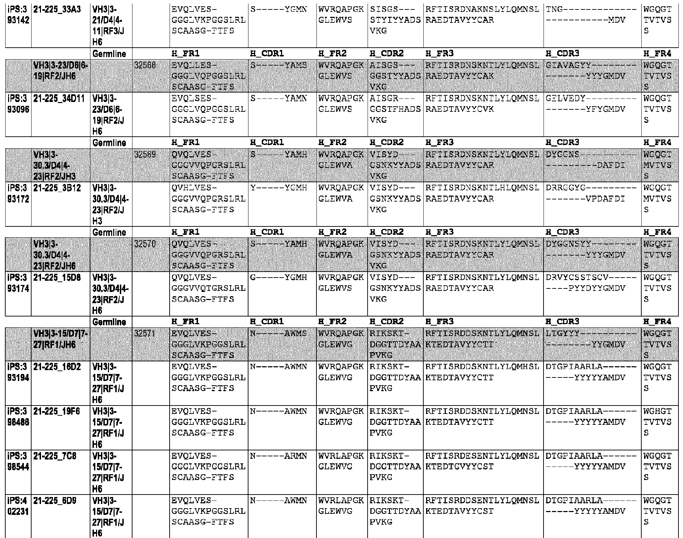

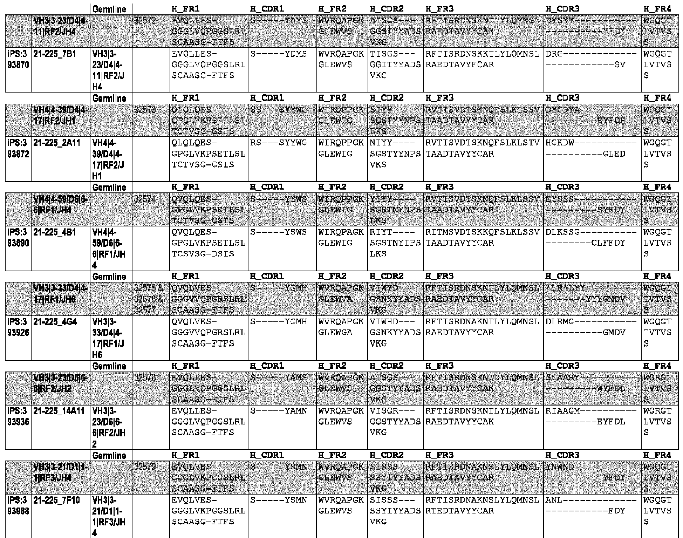

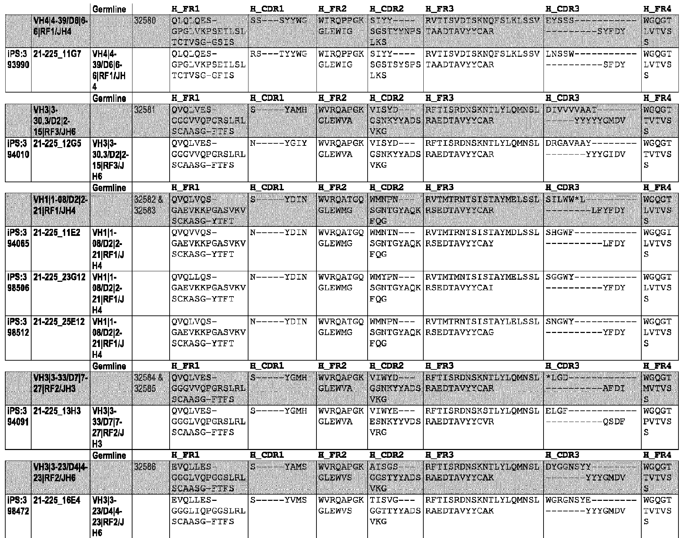

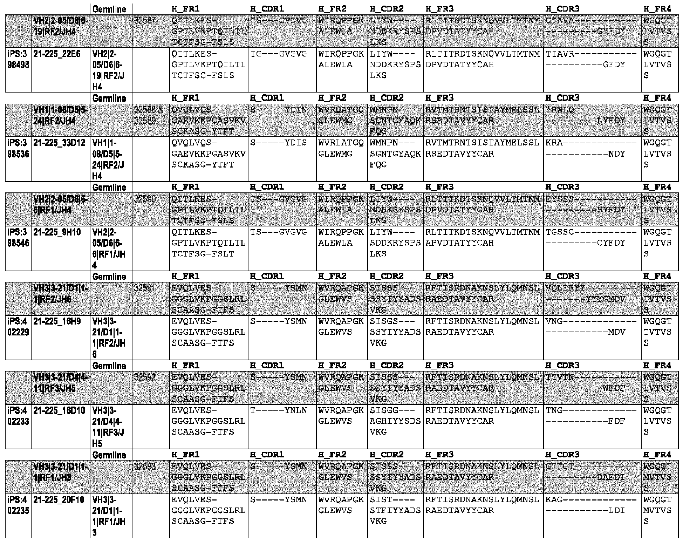

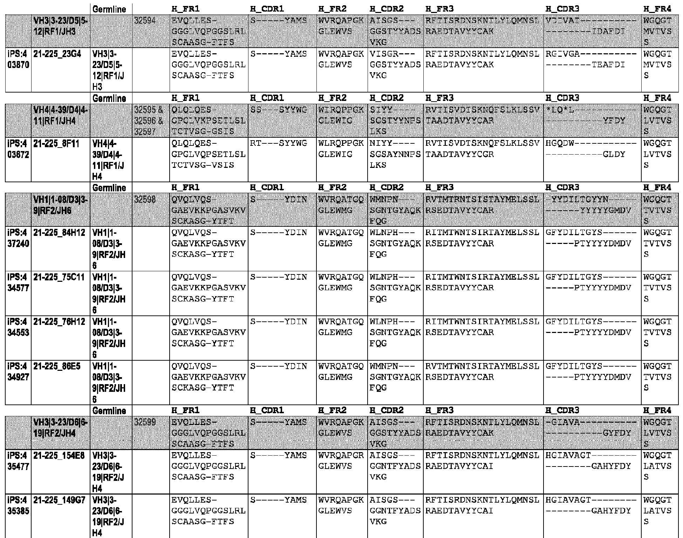

**Figure 51.** A table presenting a protein alignment of light and heavy variable regions for certain antigen binding proteins of the present invention (Table 4). An asterisk "*" denotes a stop codon. Sequences containing a stop codon are represented as distinct sequences in the Sequence Listing, however, these sequences are related. Generally speaking, however, the amino acid sequences of the light and heavy chain variable domains in the protein alignment presented in Table 4 are wrapped due to space issues, and unless stated otherwise, like in the case of sequences with one or more stop codons, should be understood to be single amino acid sequences.

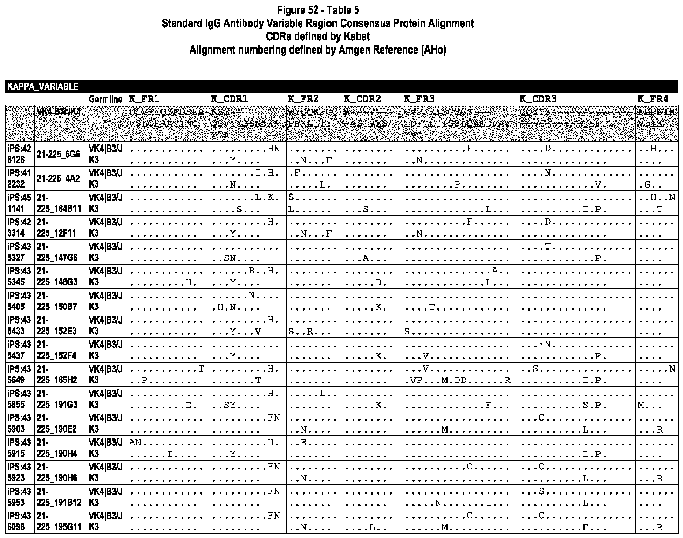

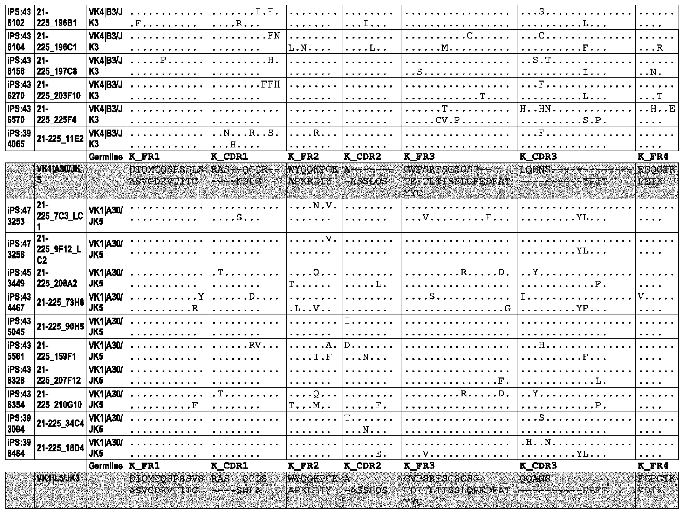

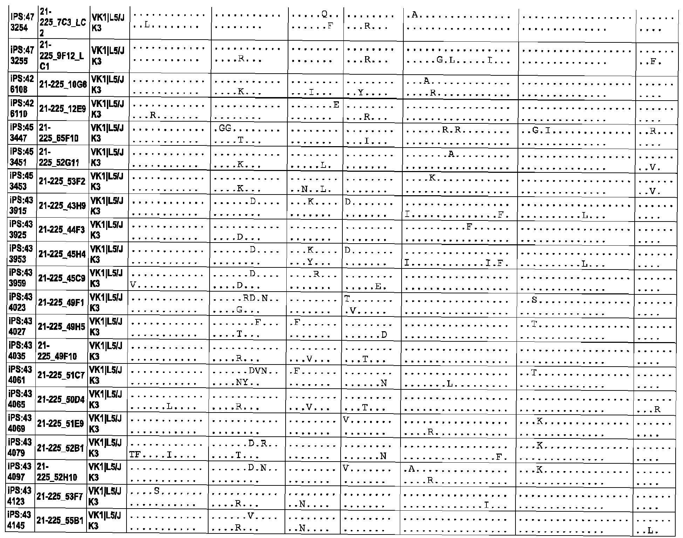

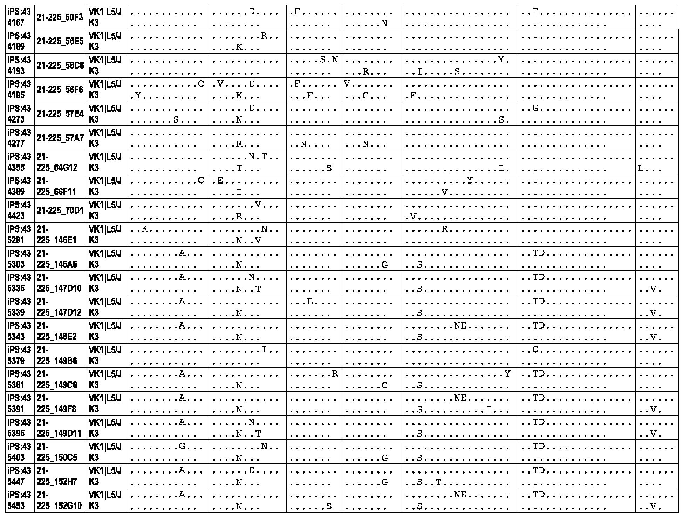

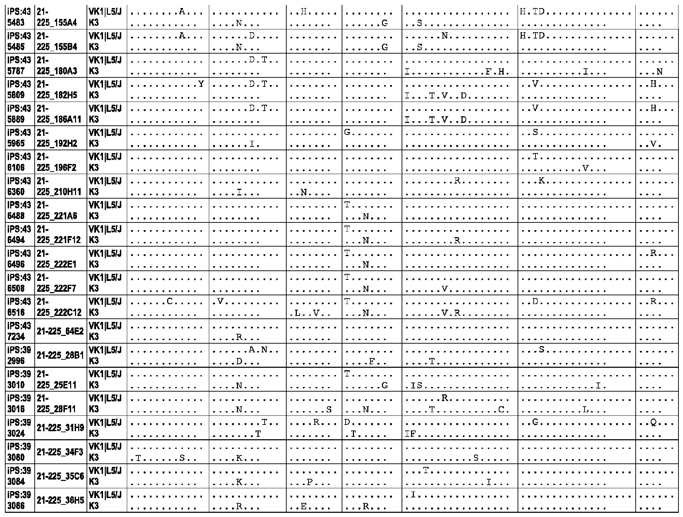

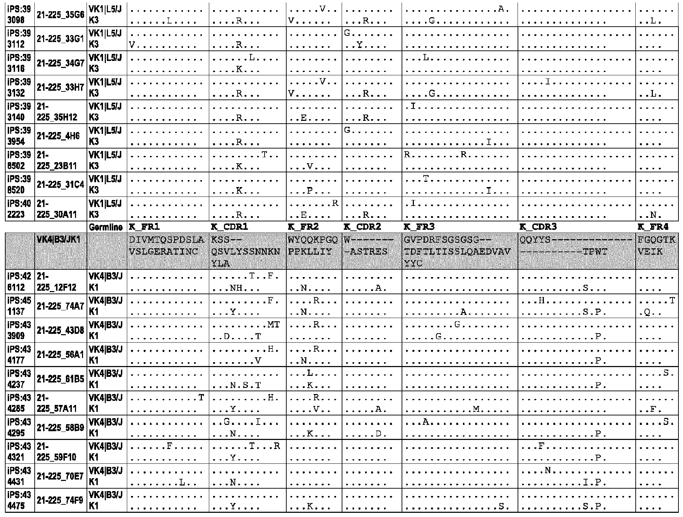

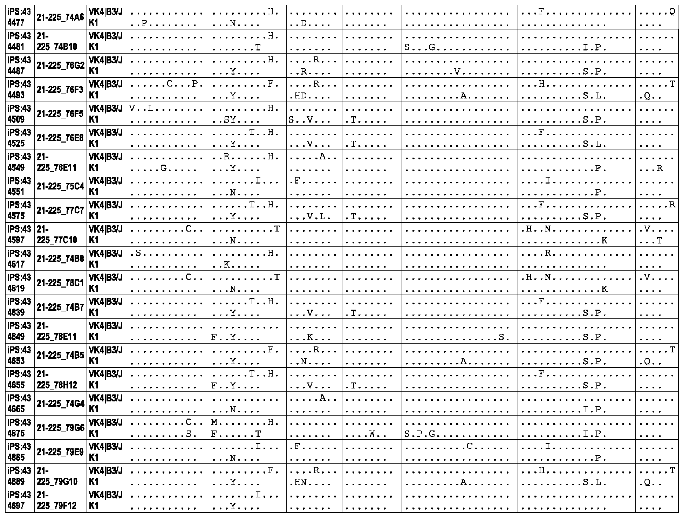

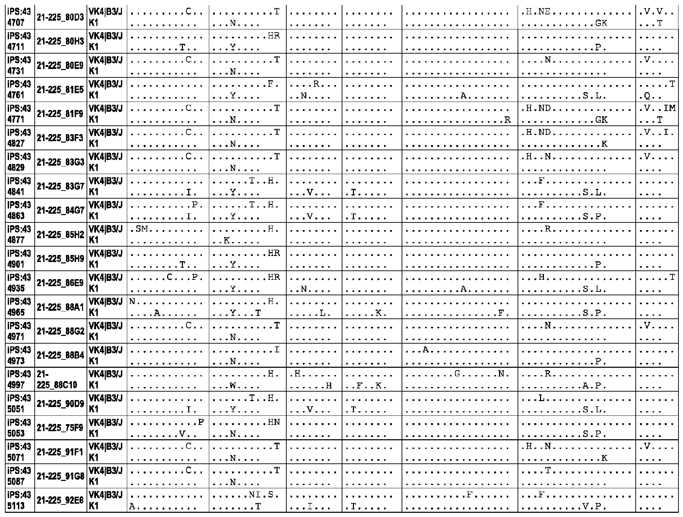

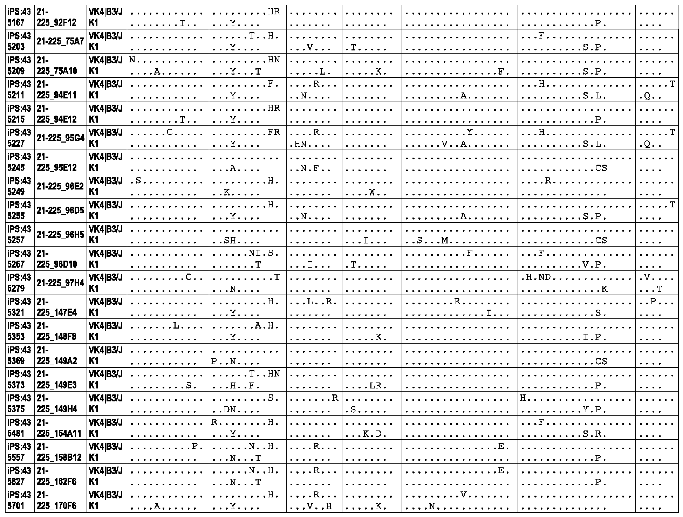

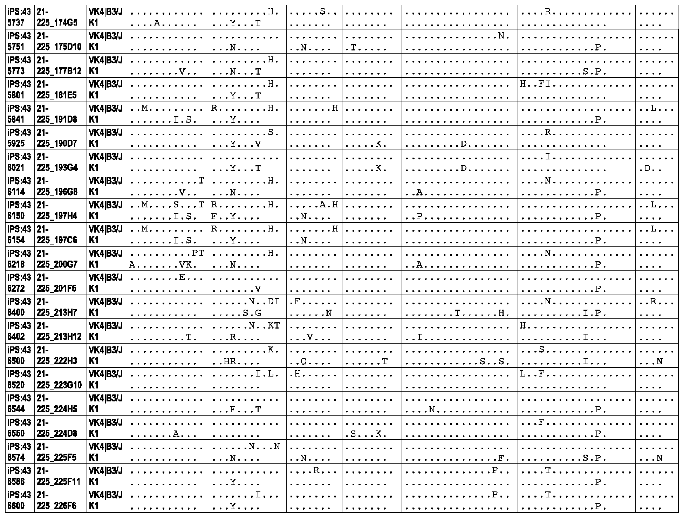

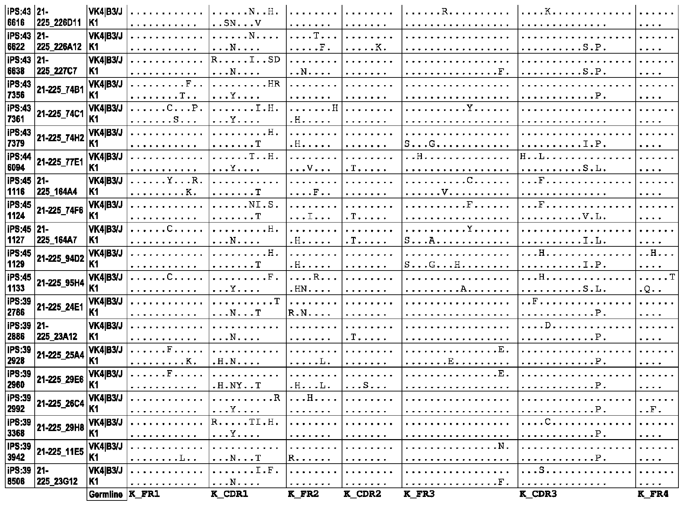

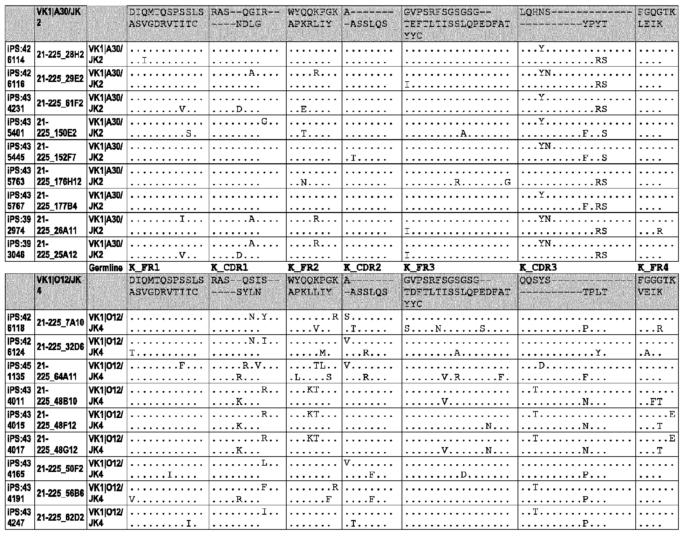

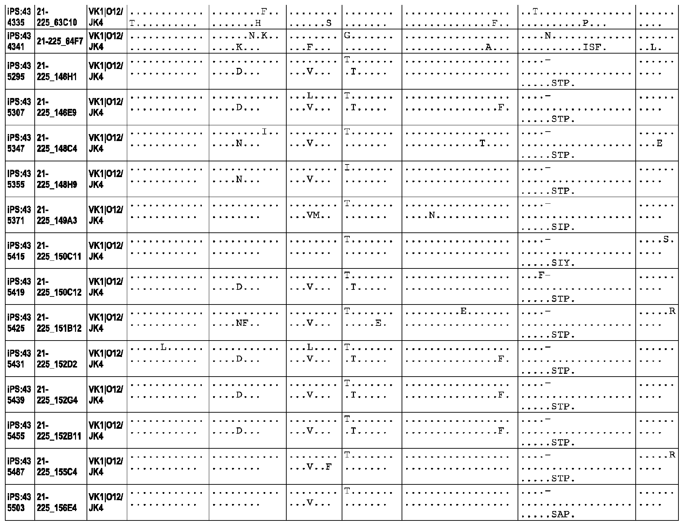

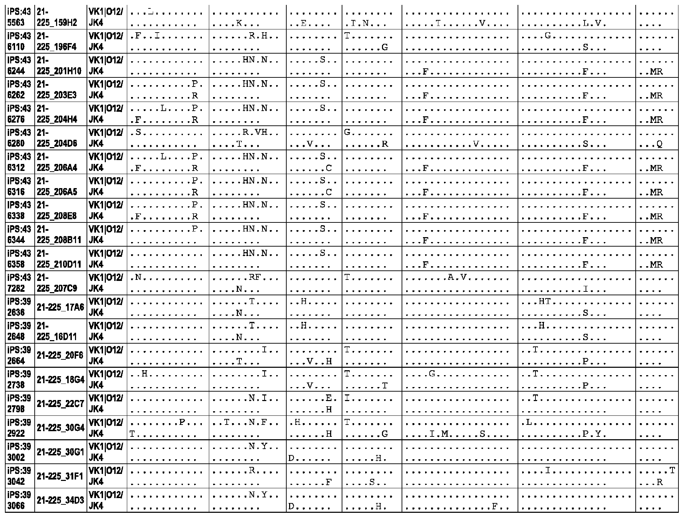

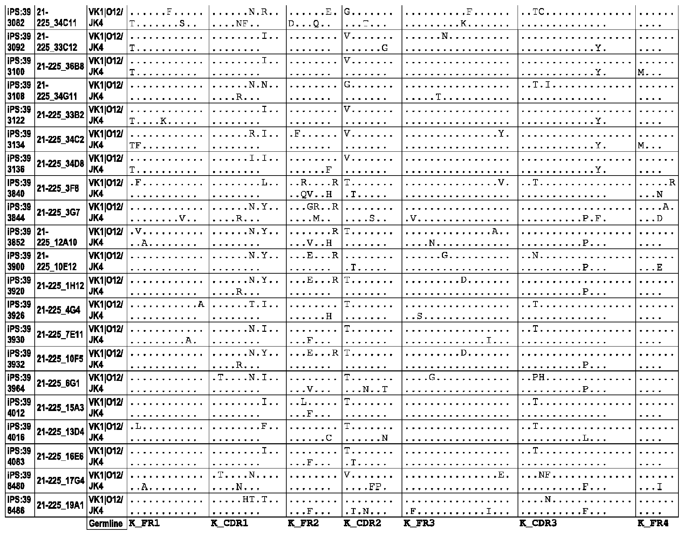

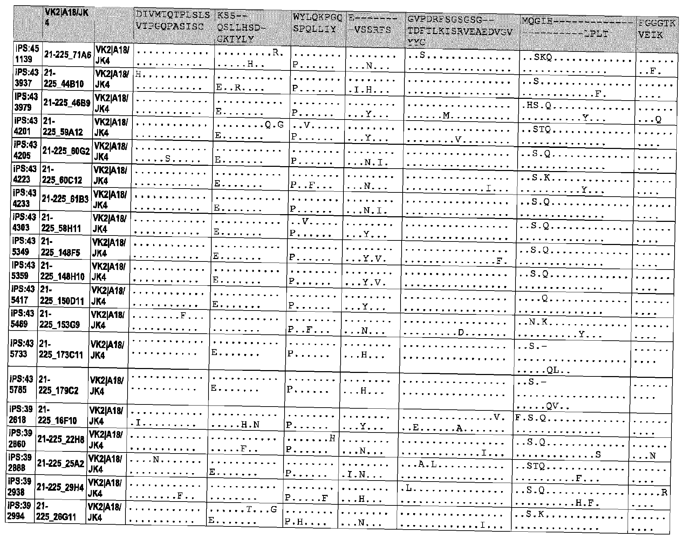

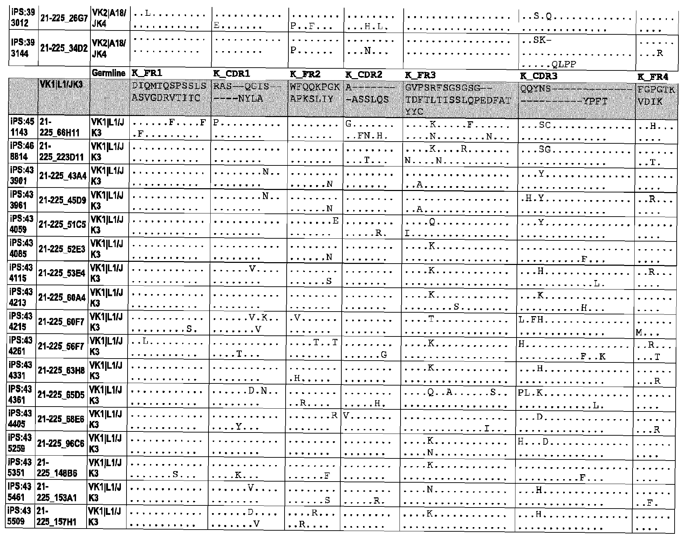

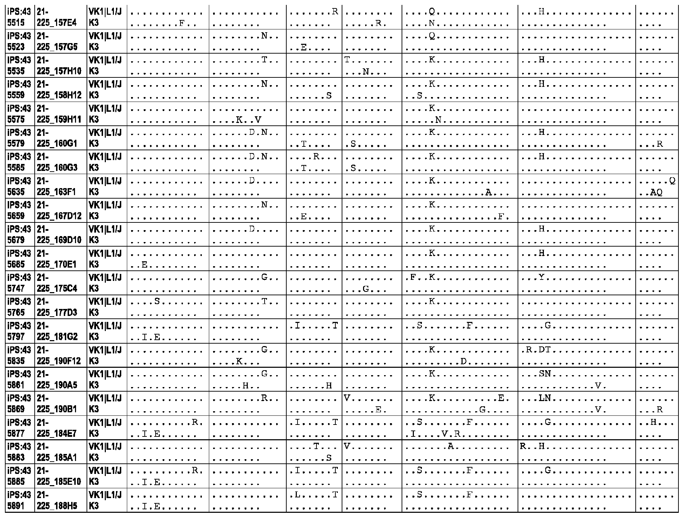

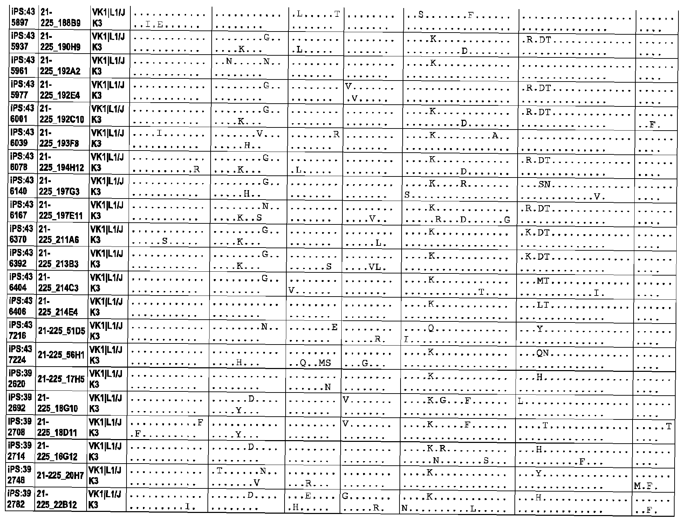

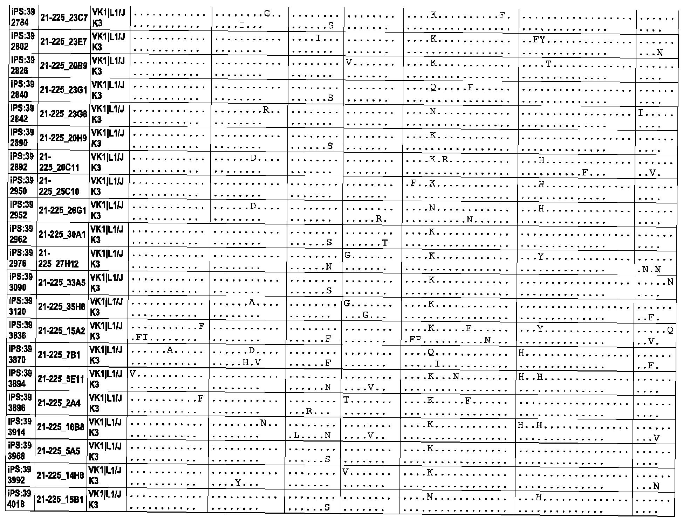

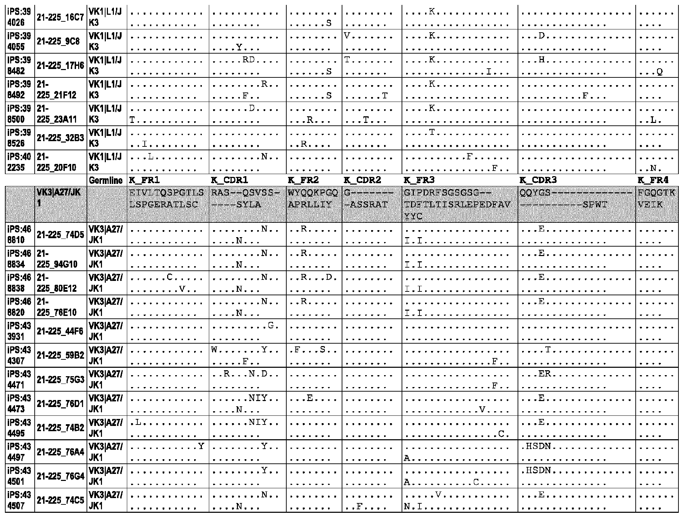

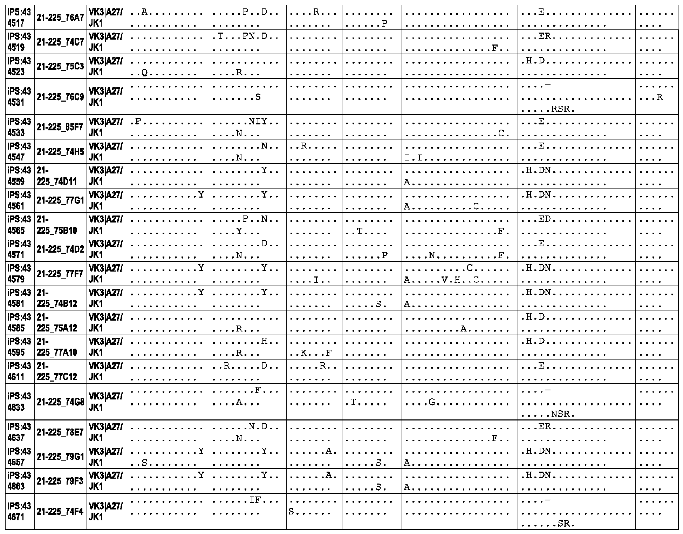

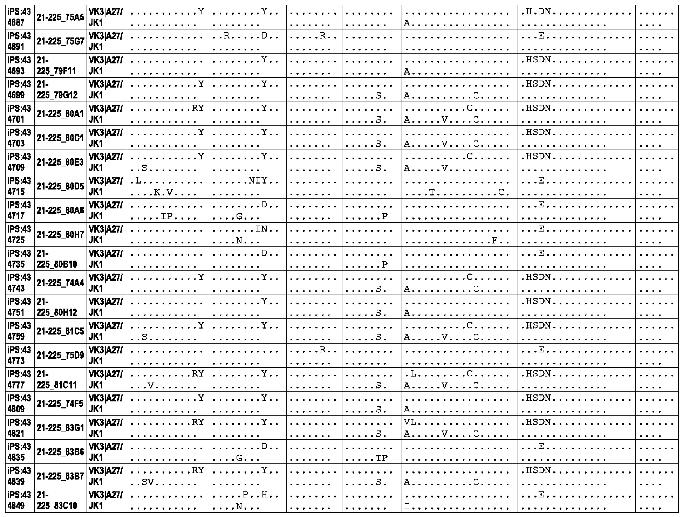

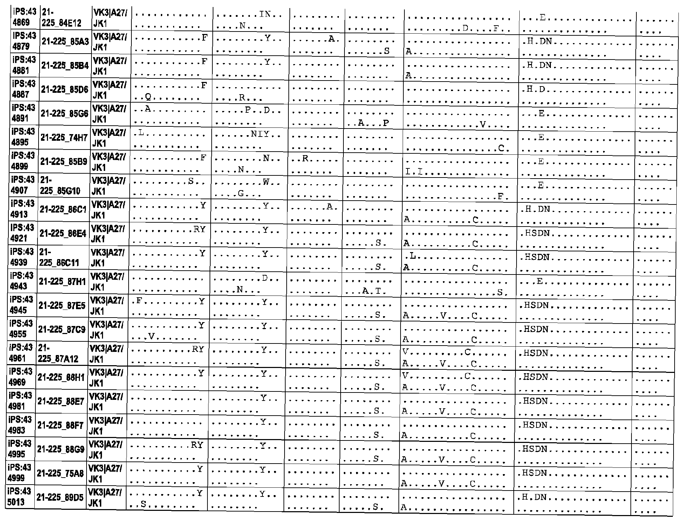

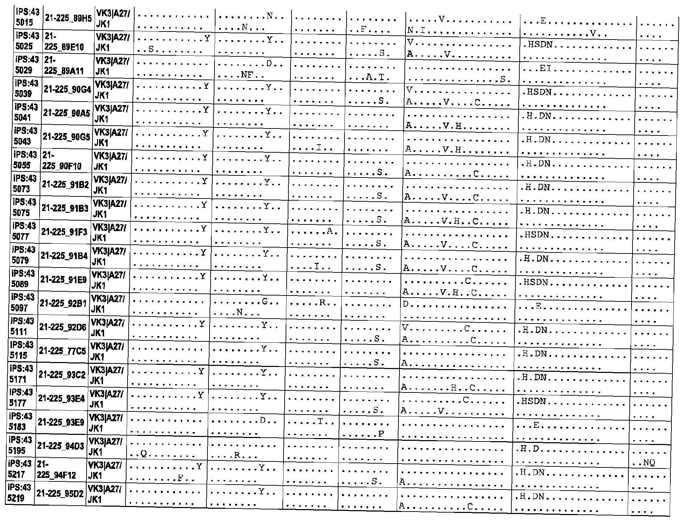

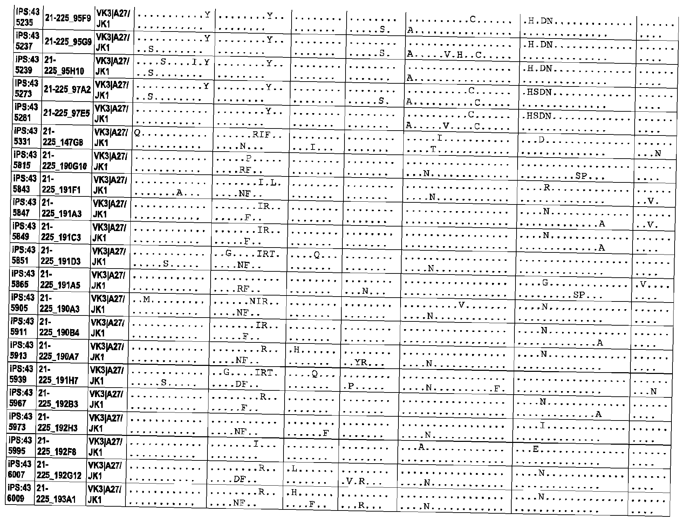

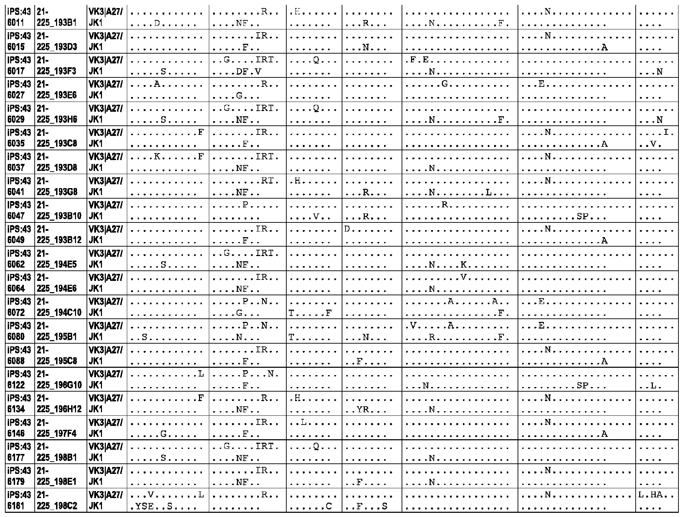

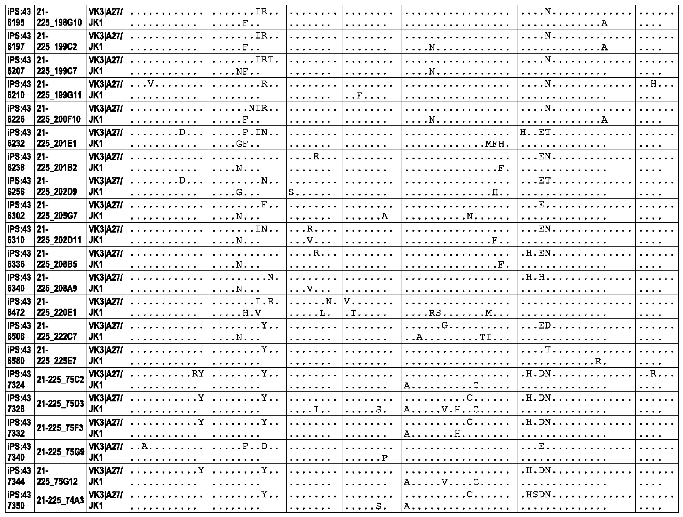

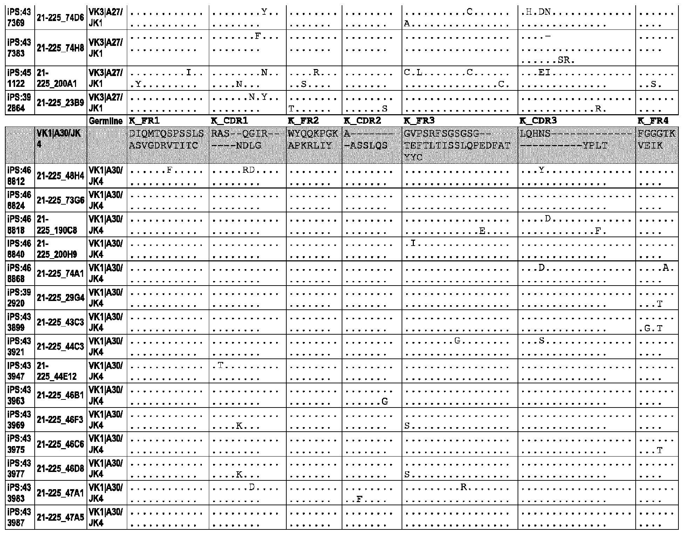

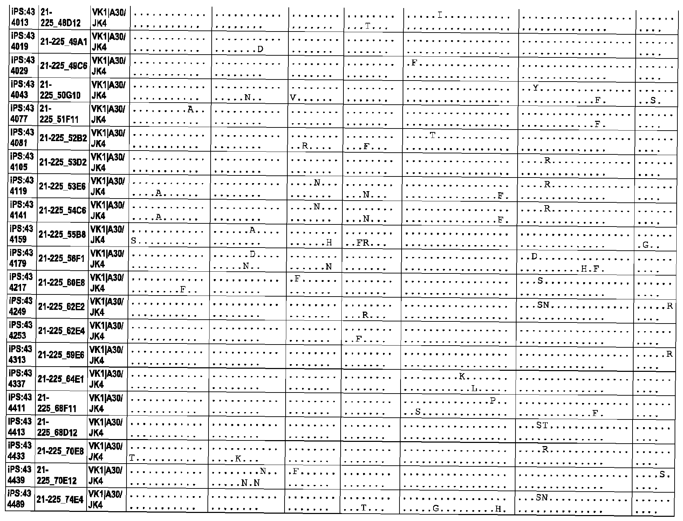

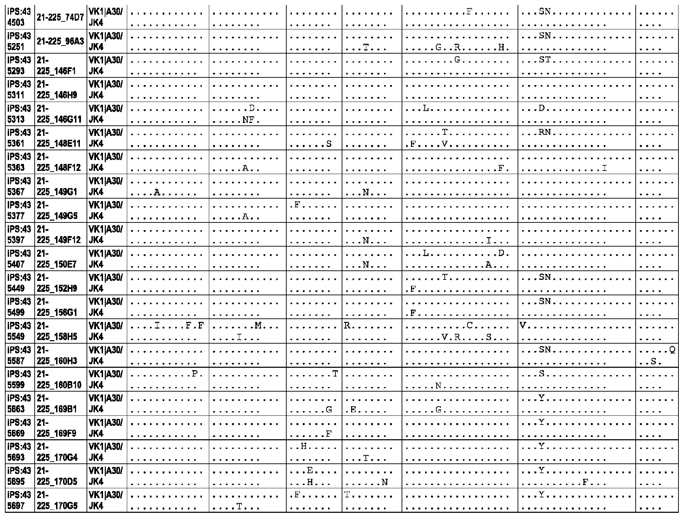

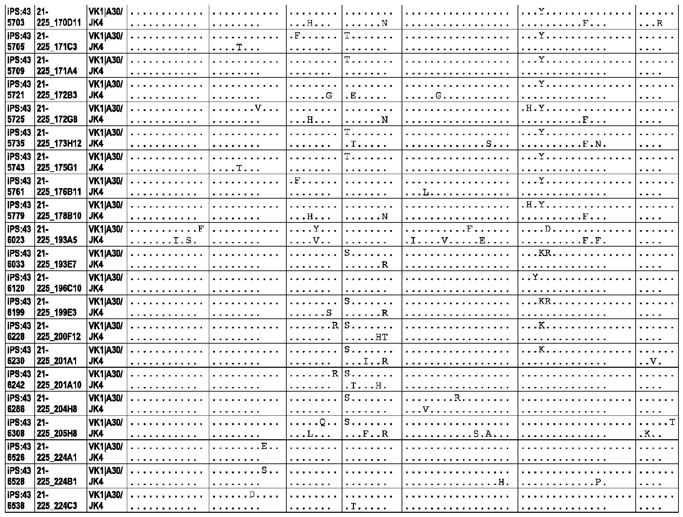

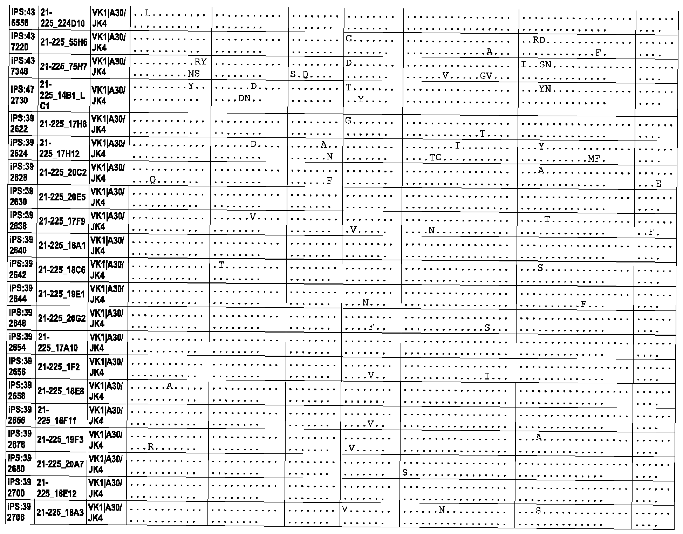

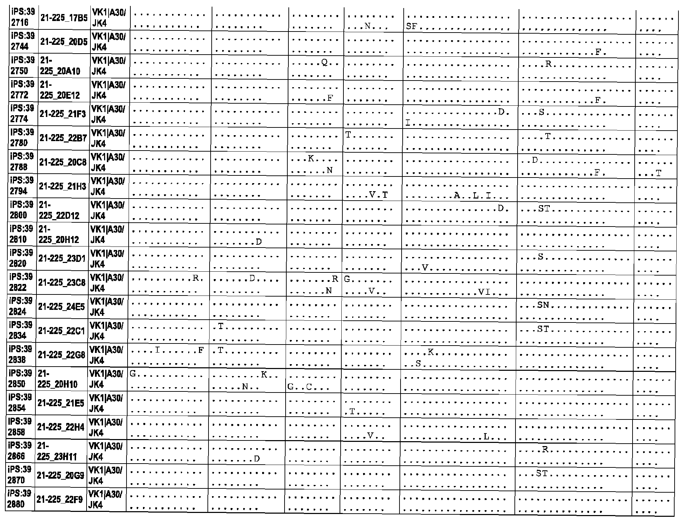

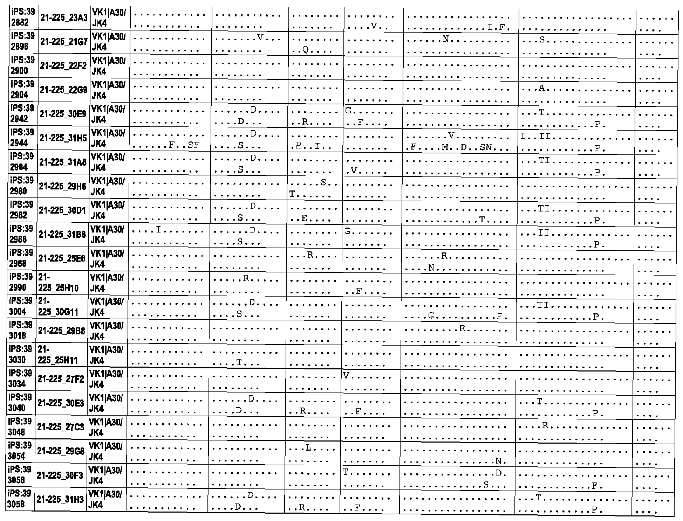

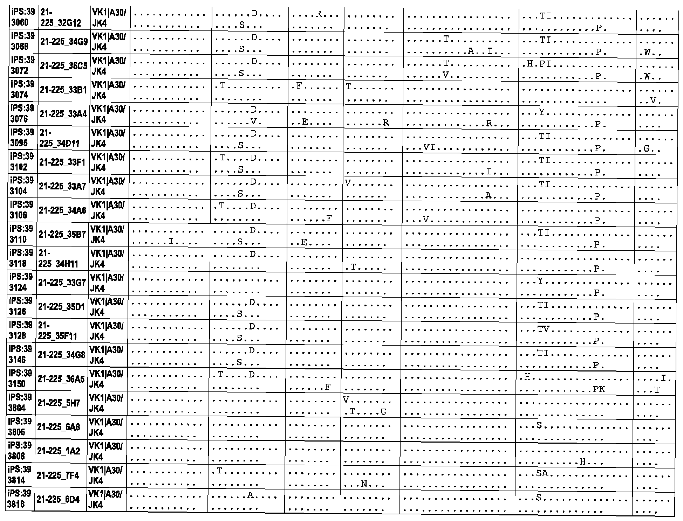

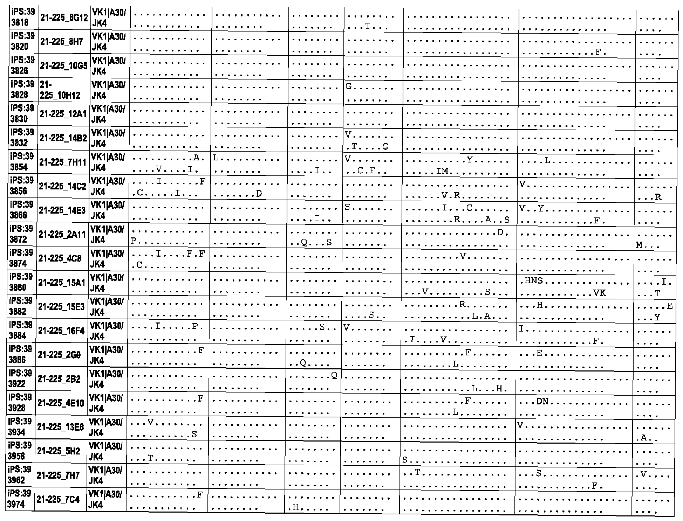

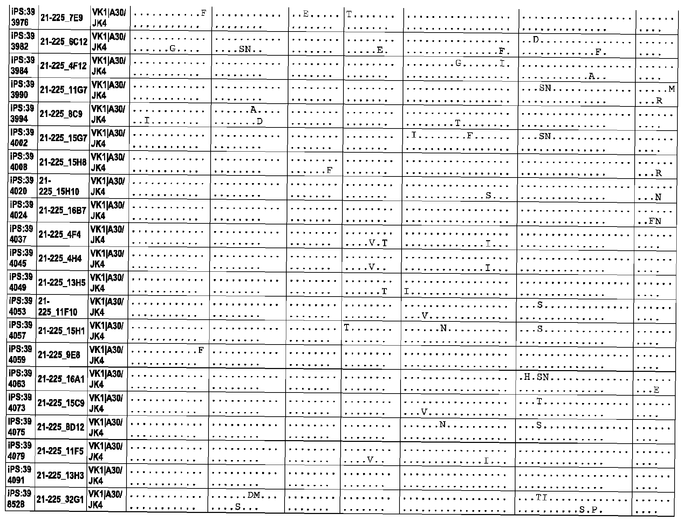

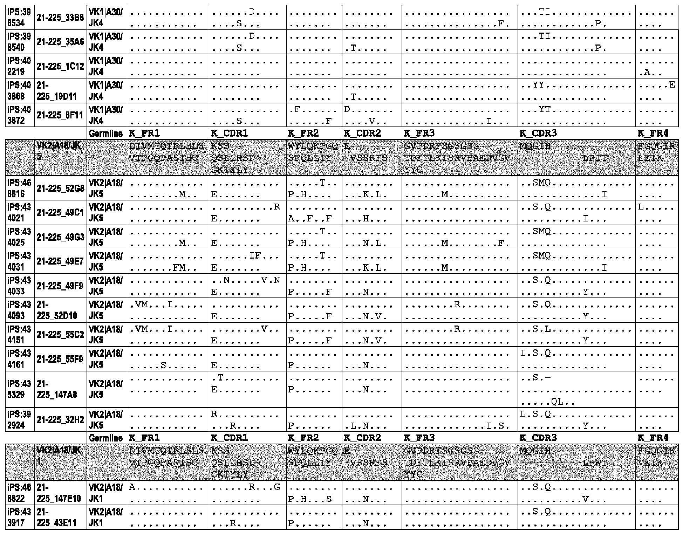

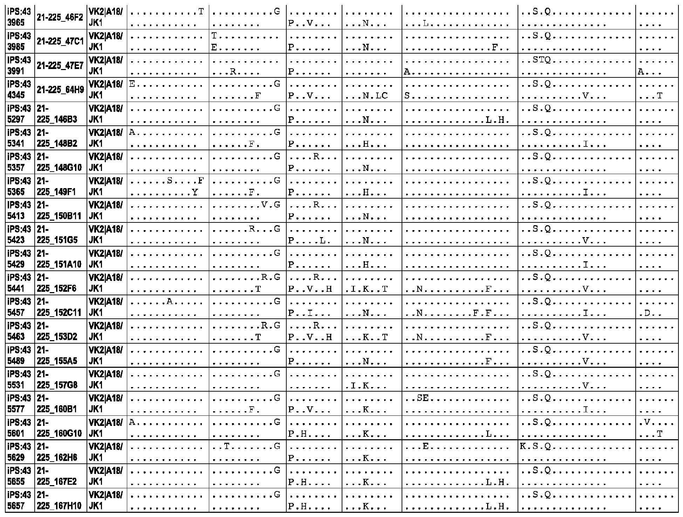

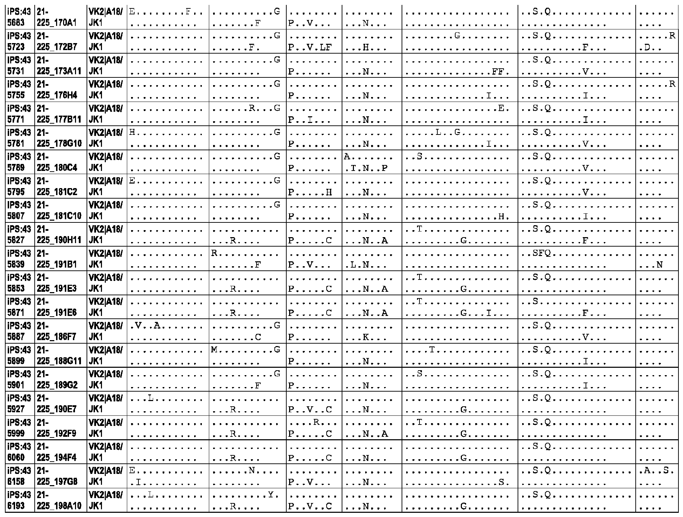

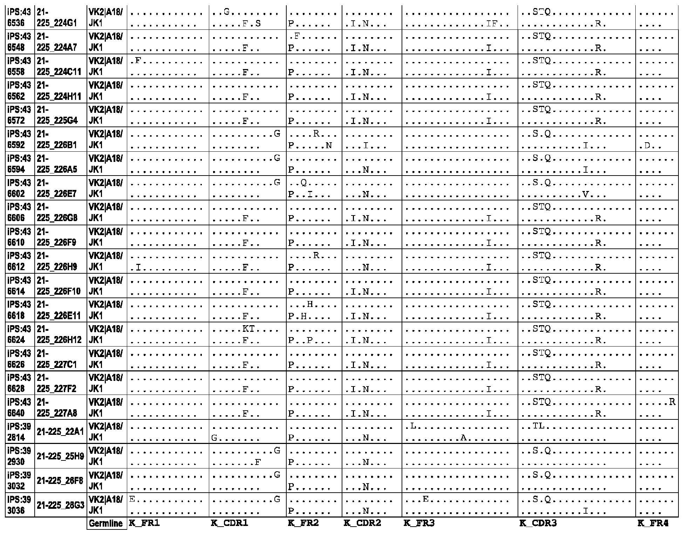

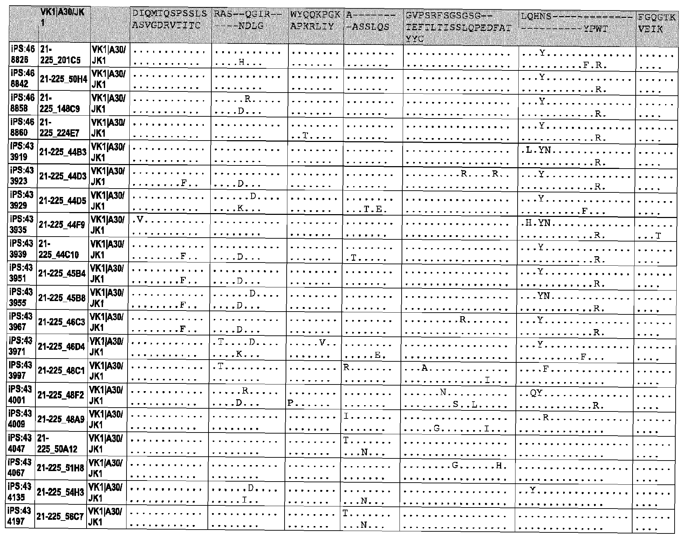

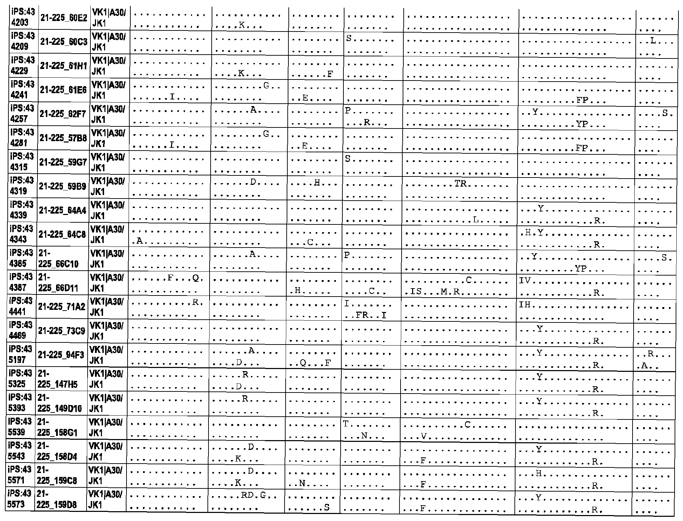

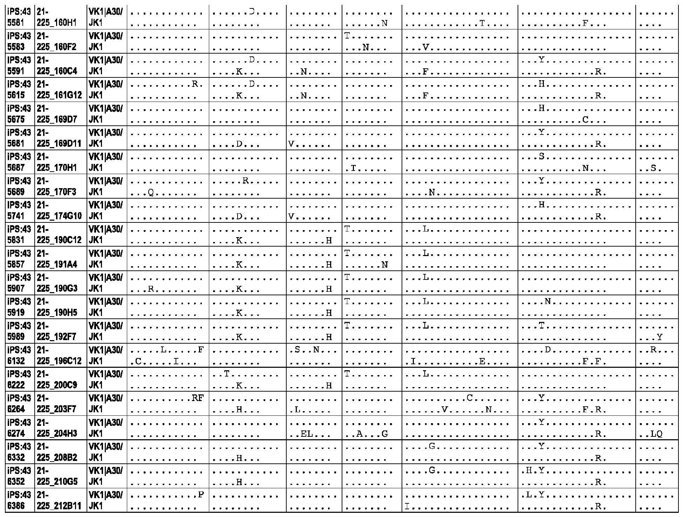

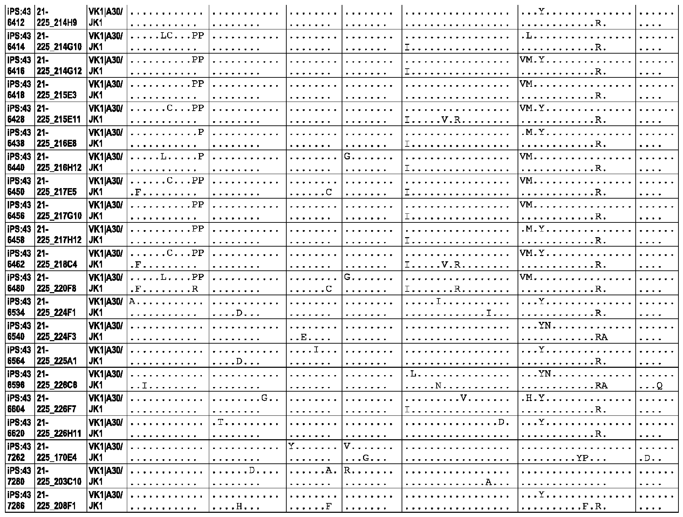

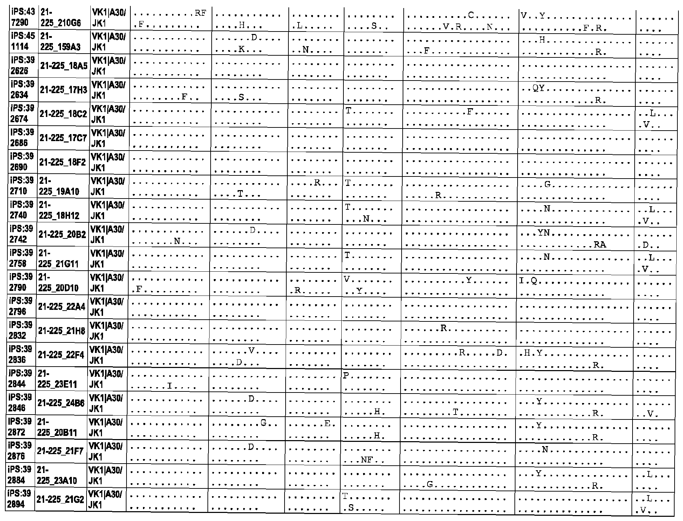

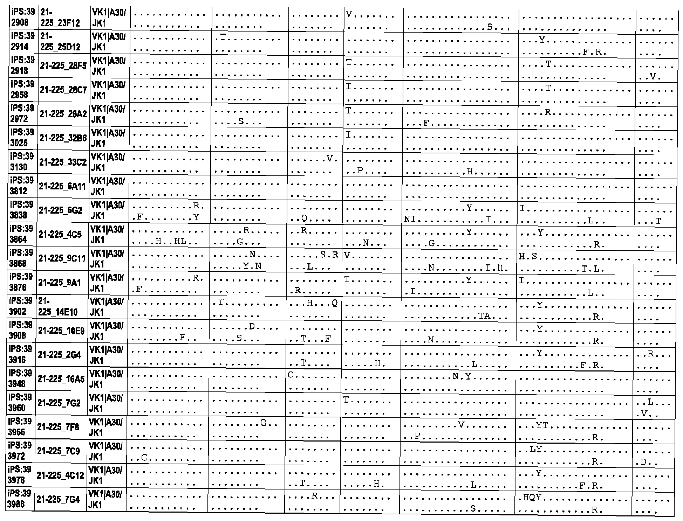

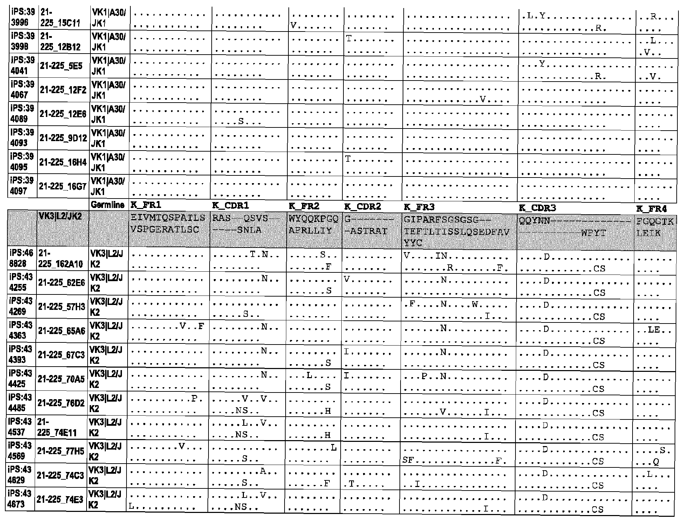

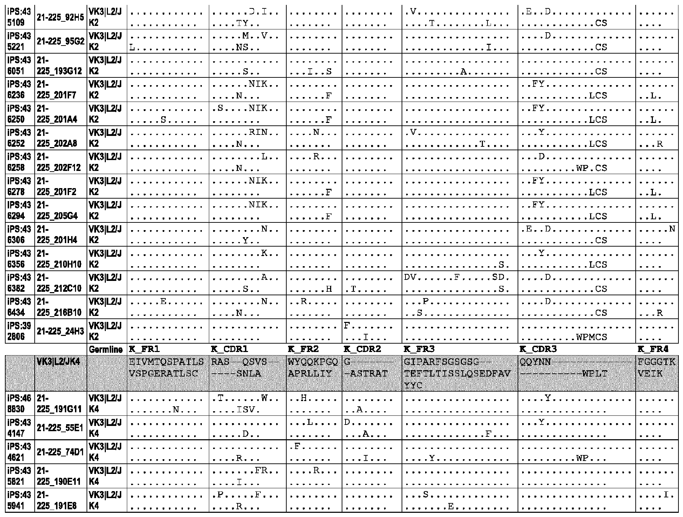

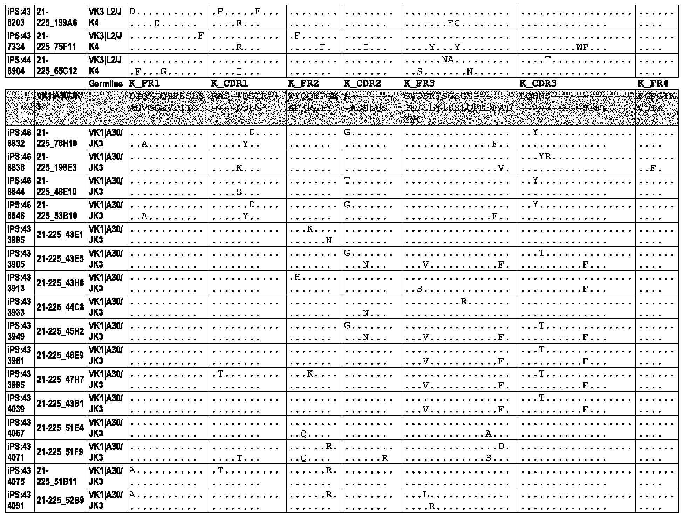

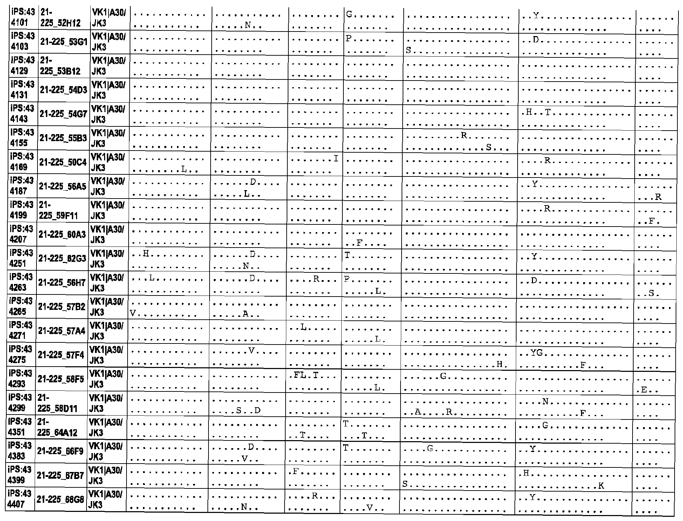

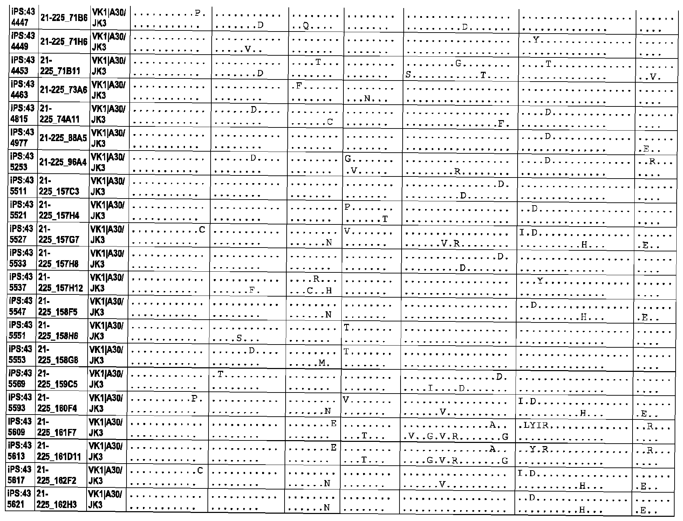

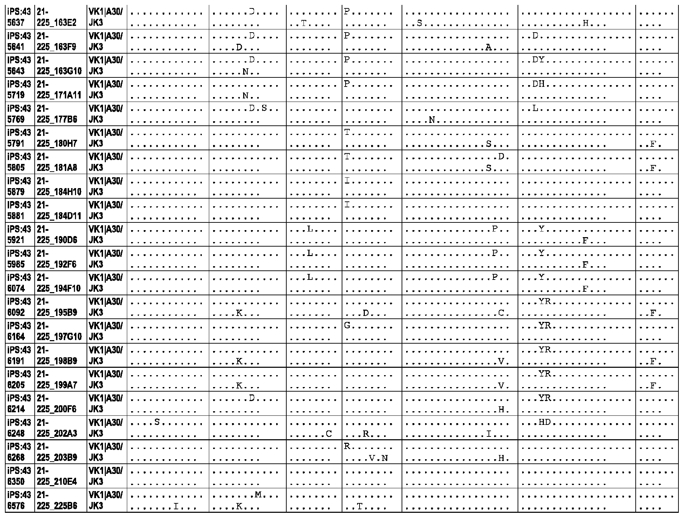

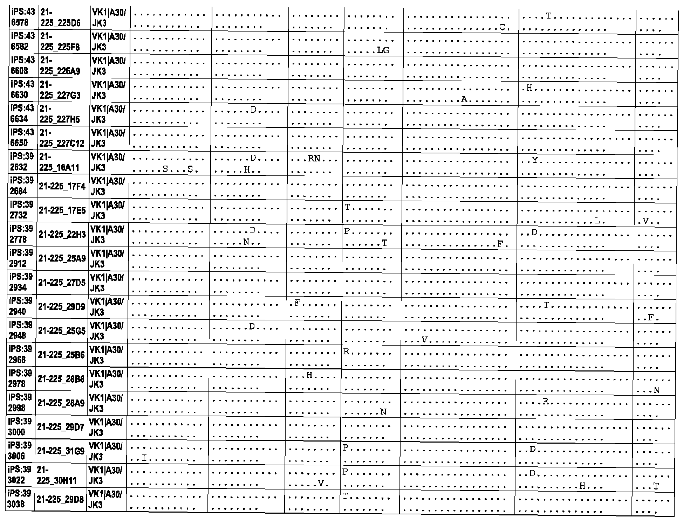

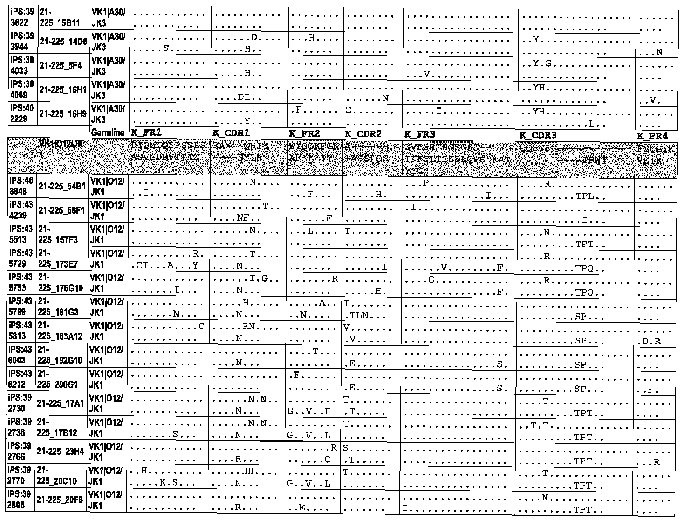

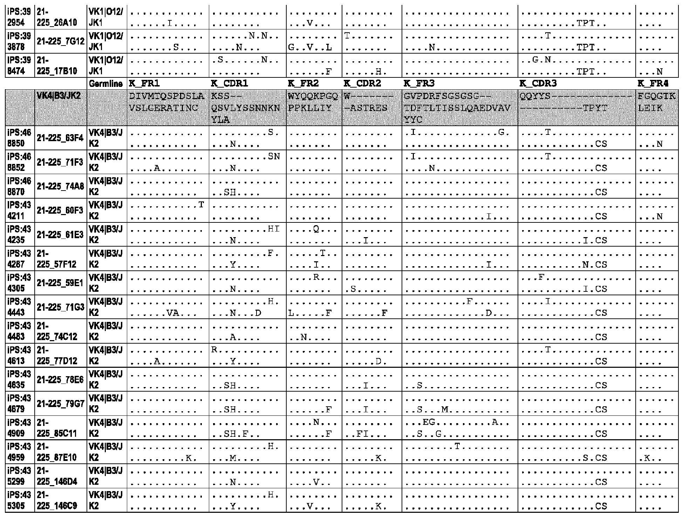

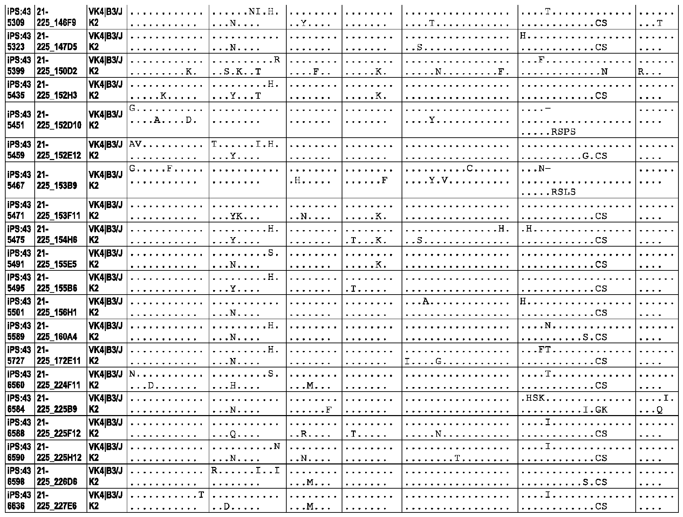

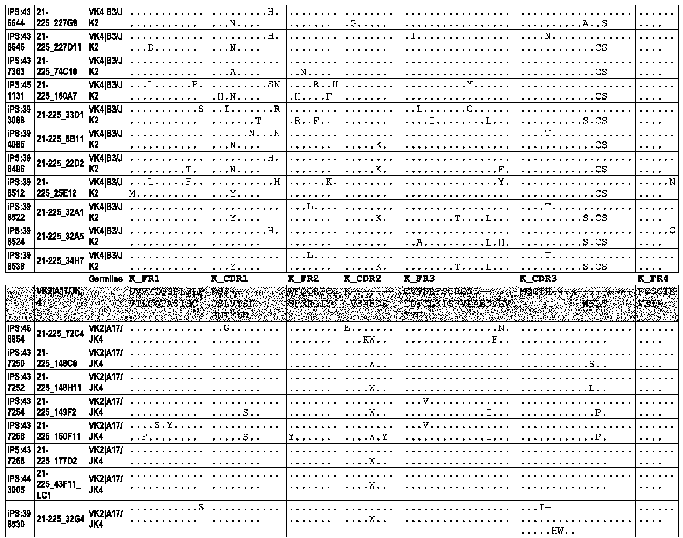

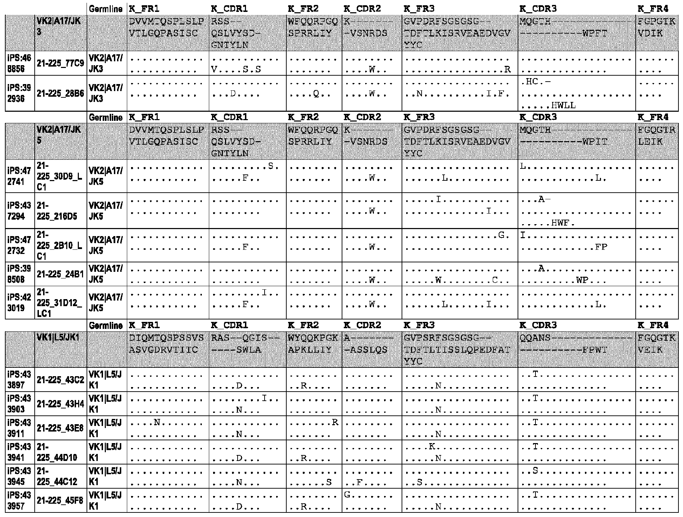

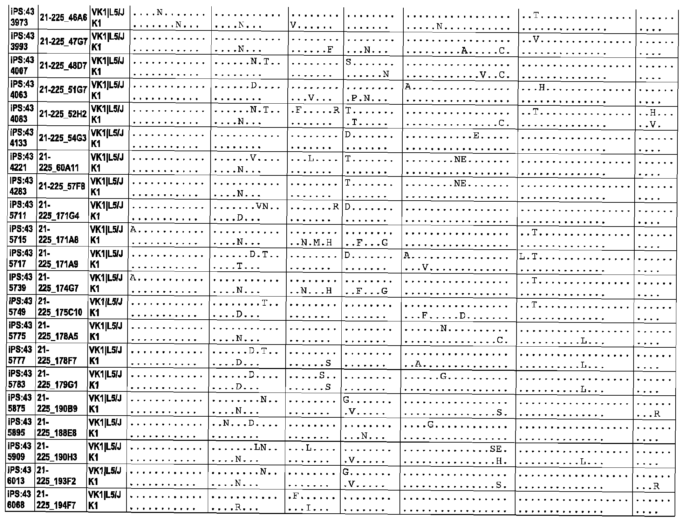

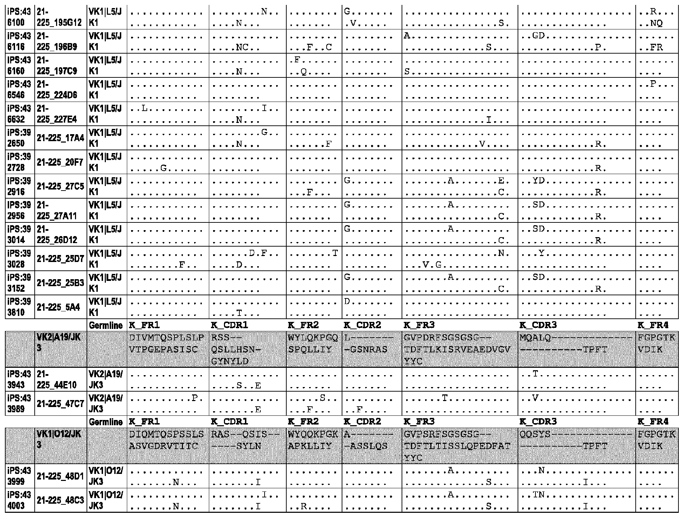

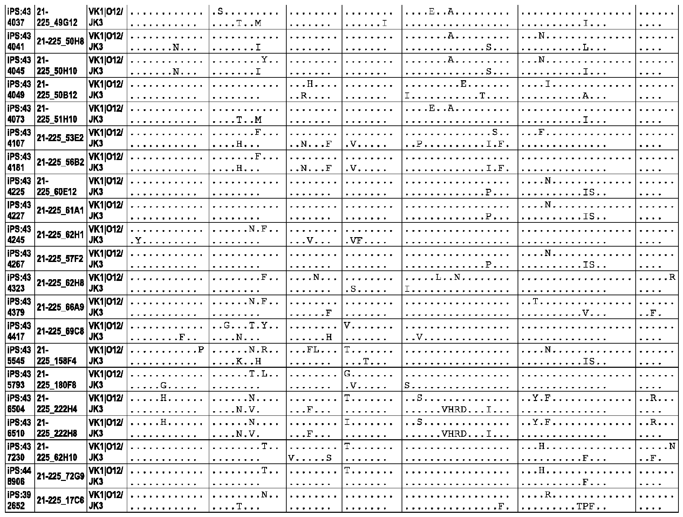

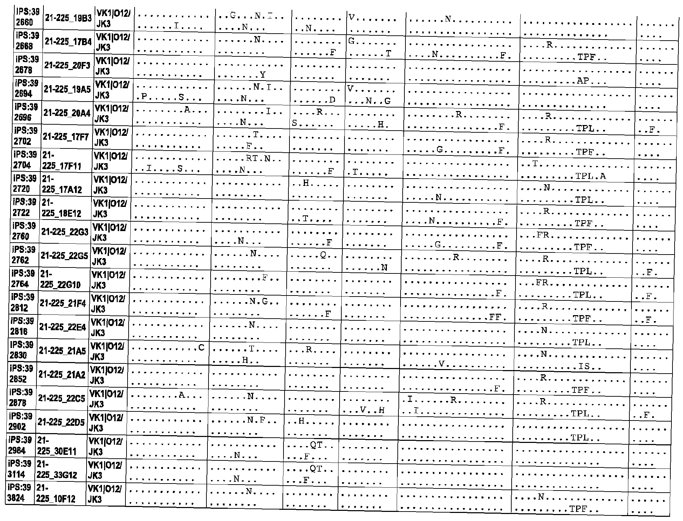

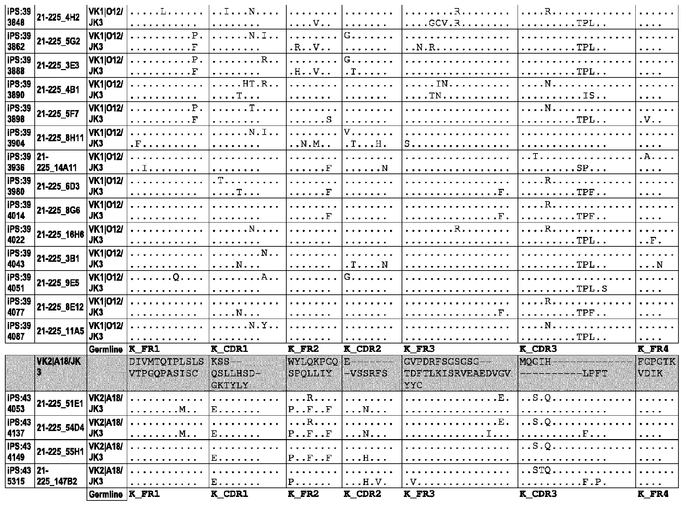

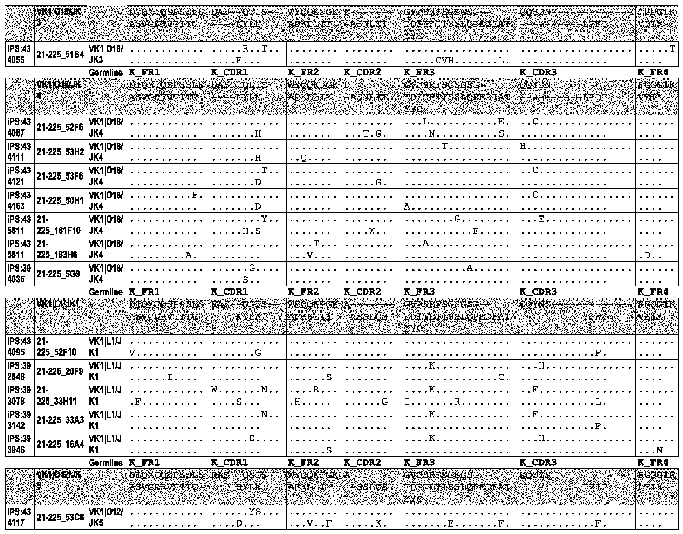

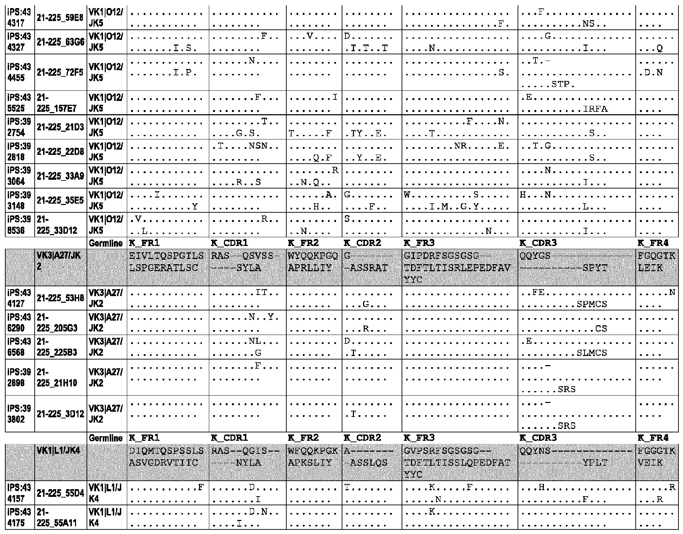

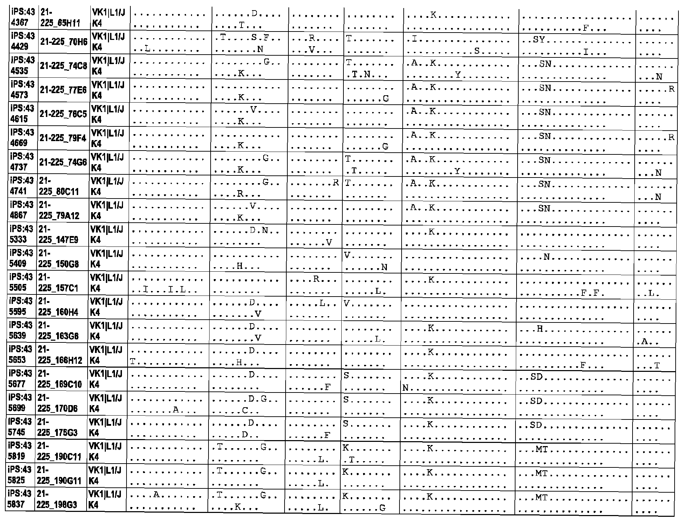

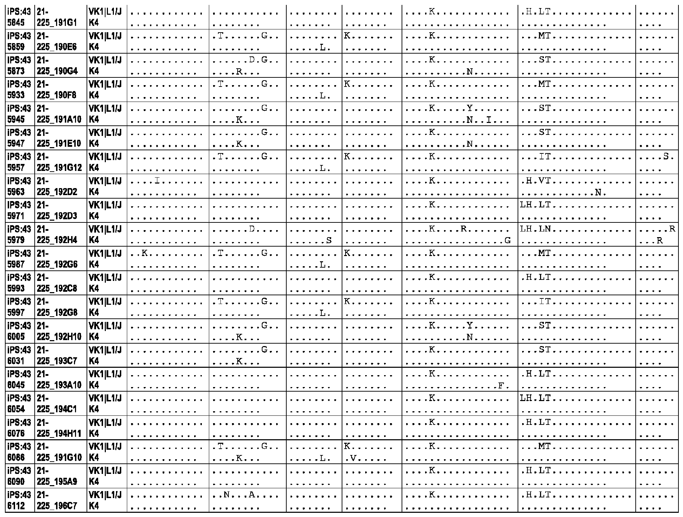

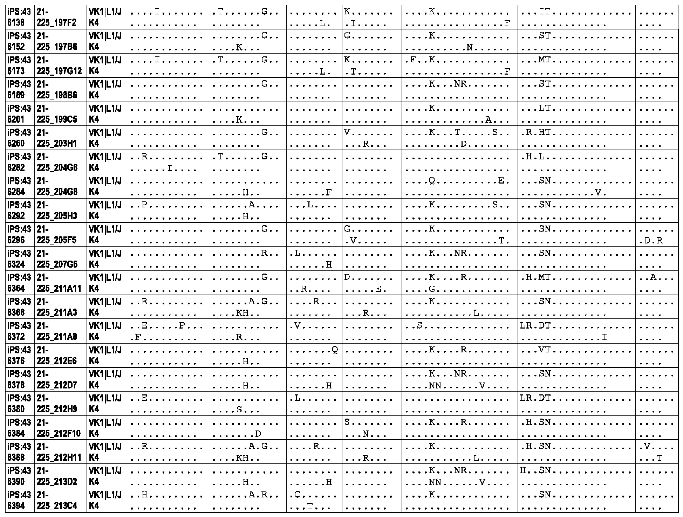

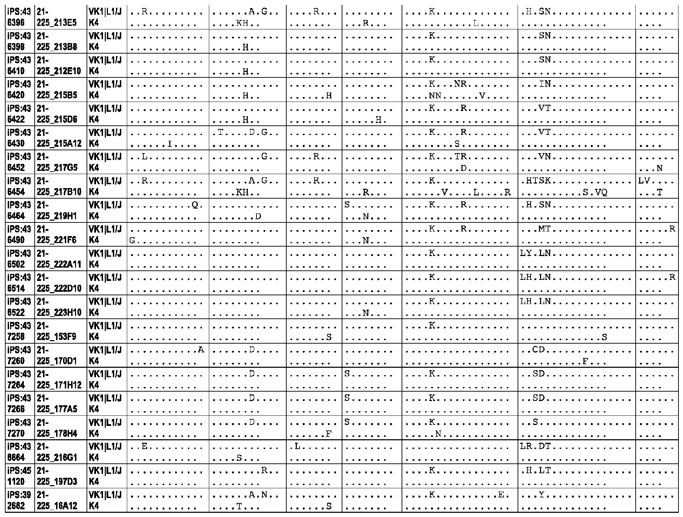

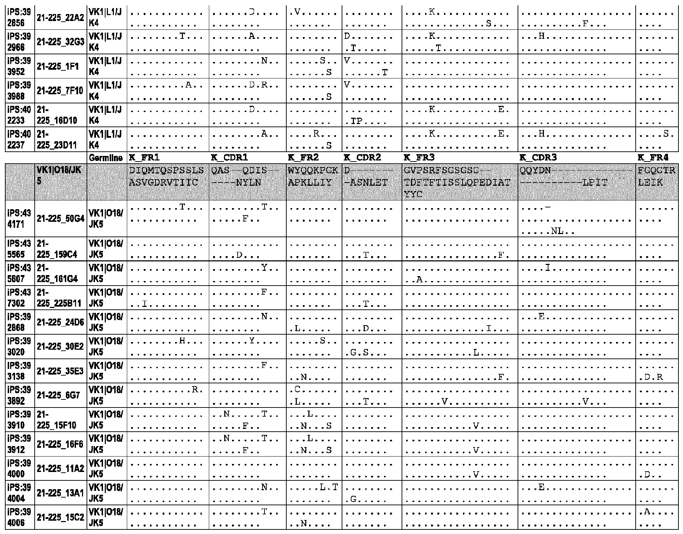

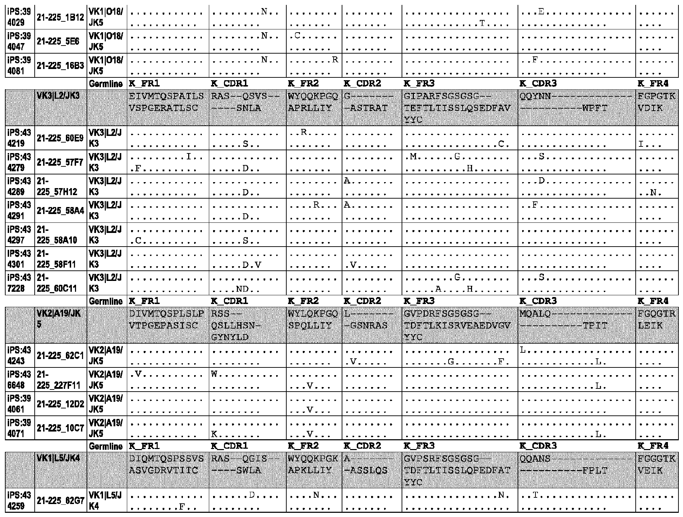

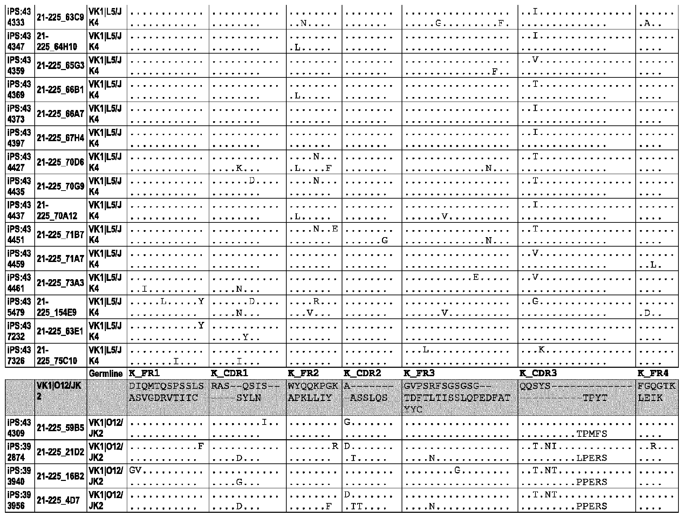

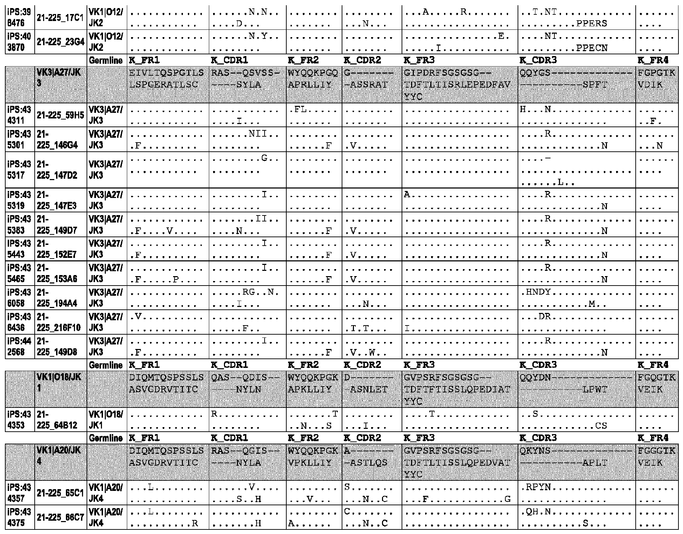

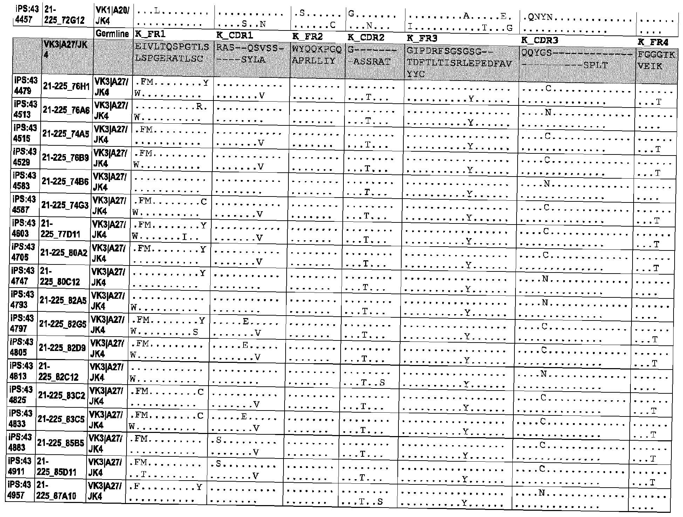

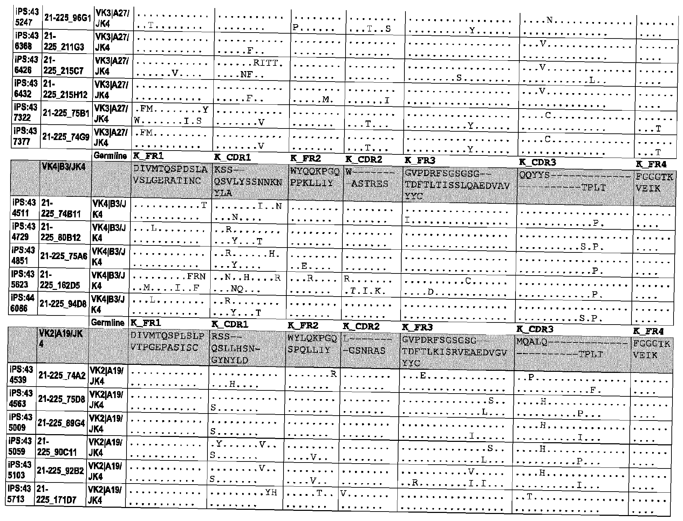

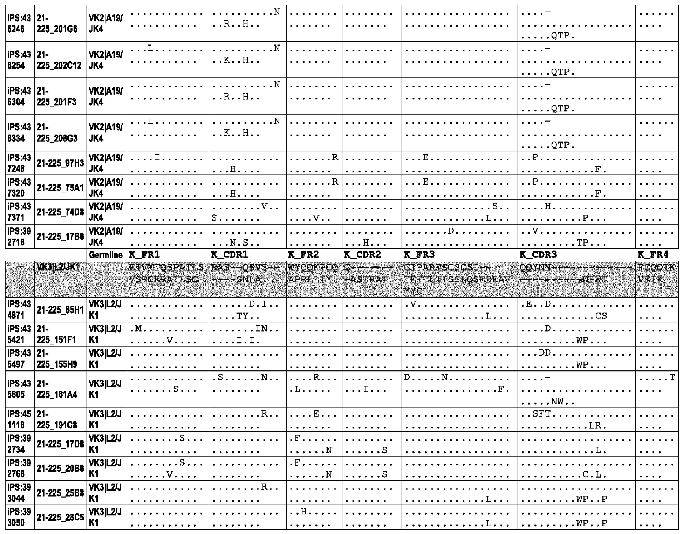

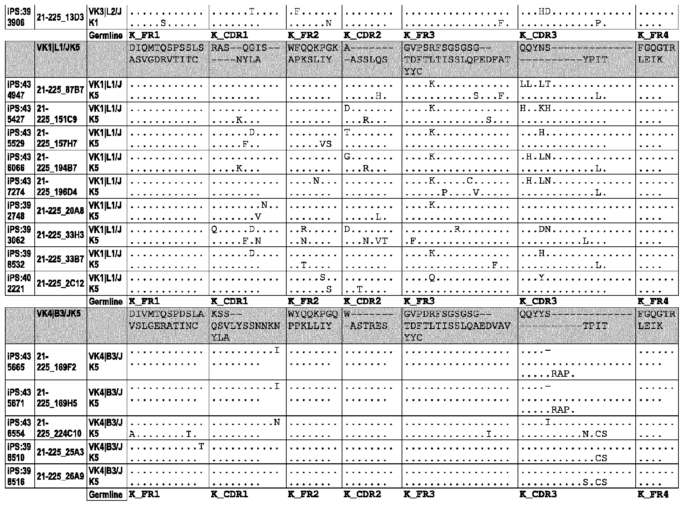

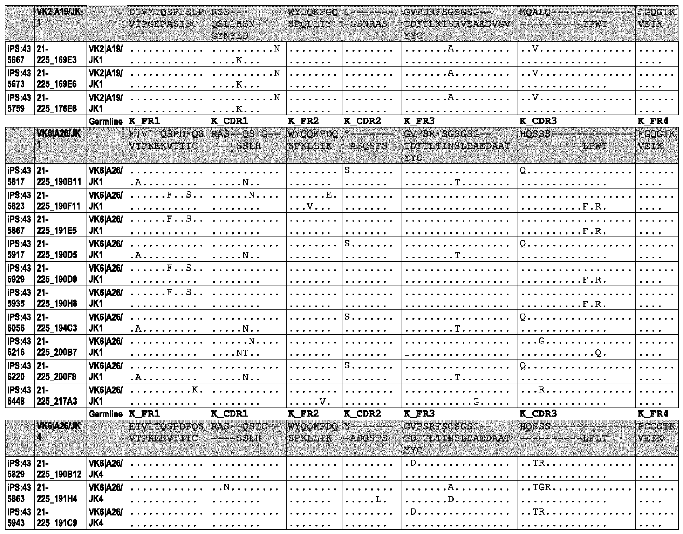

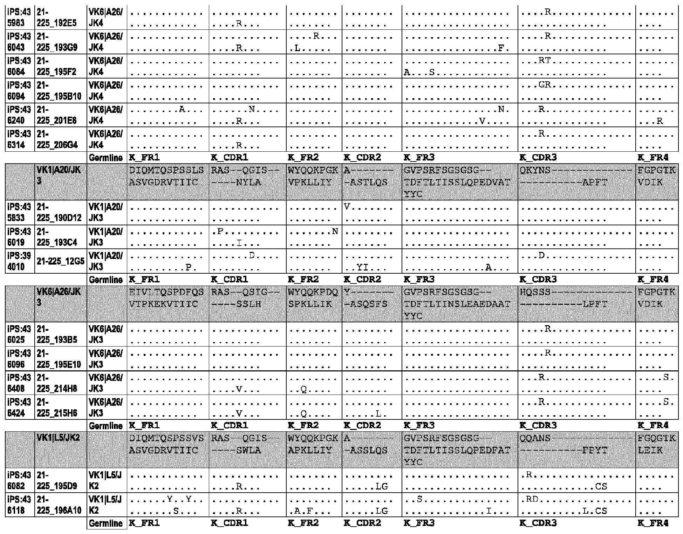

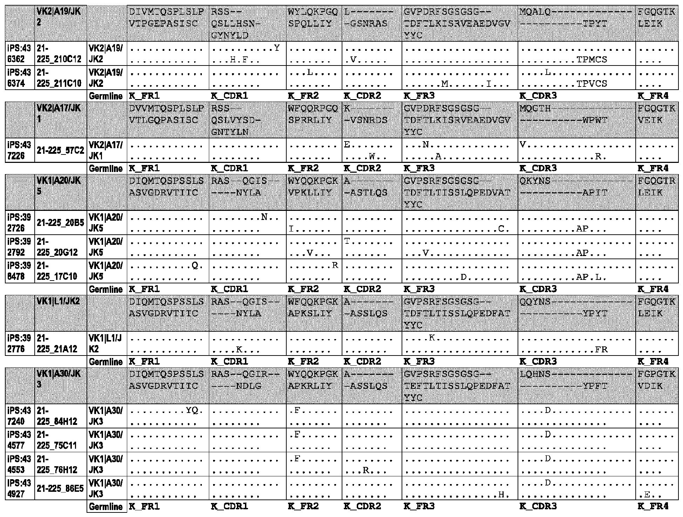

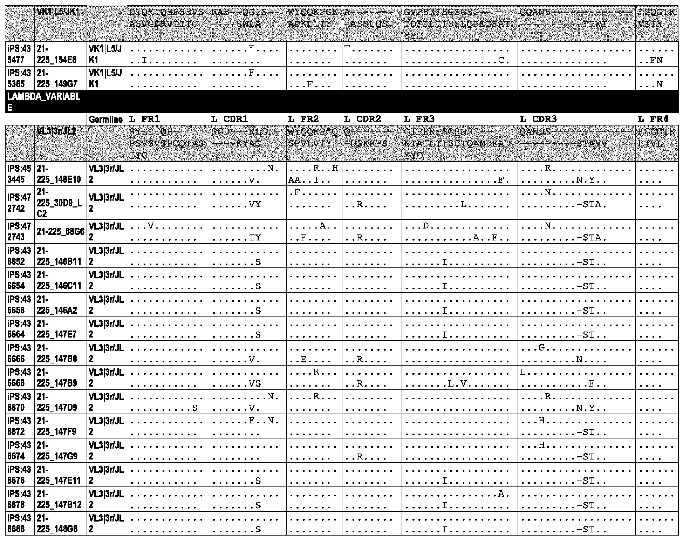

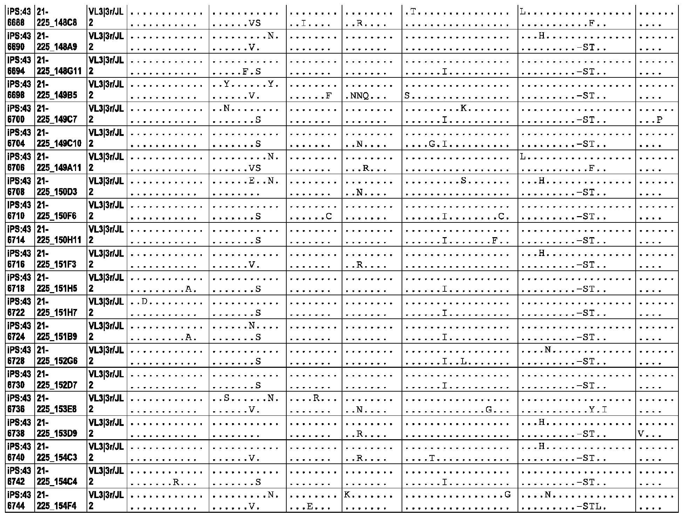

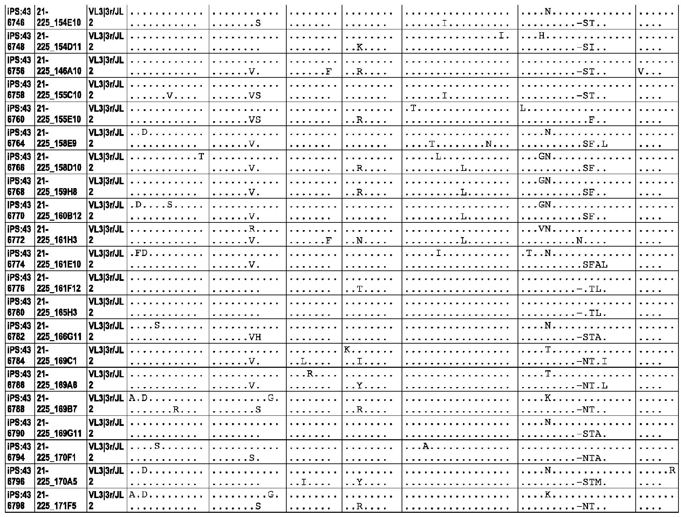

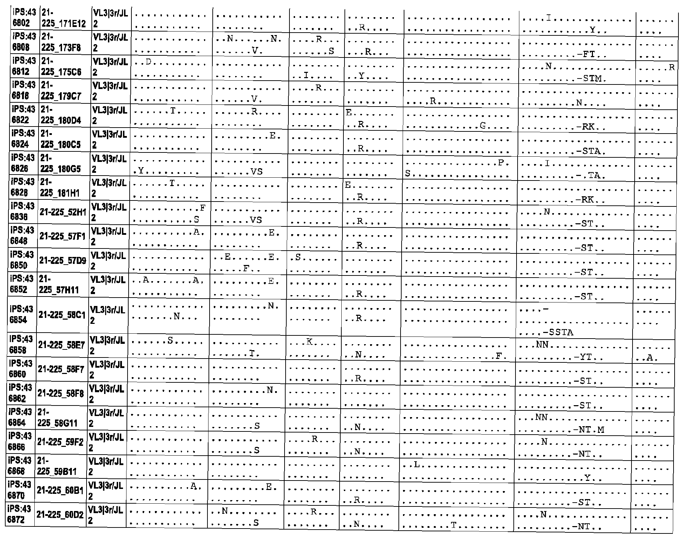

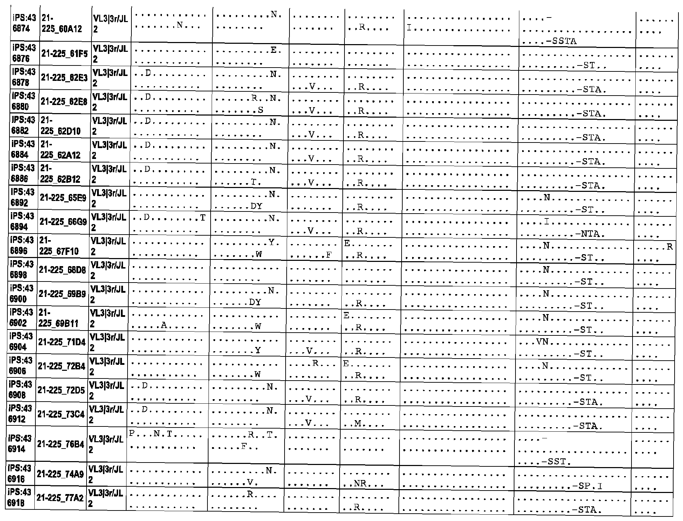

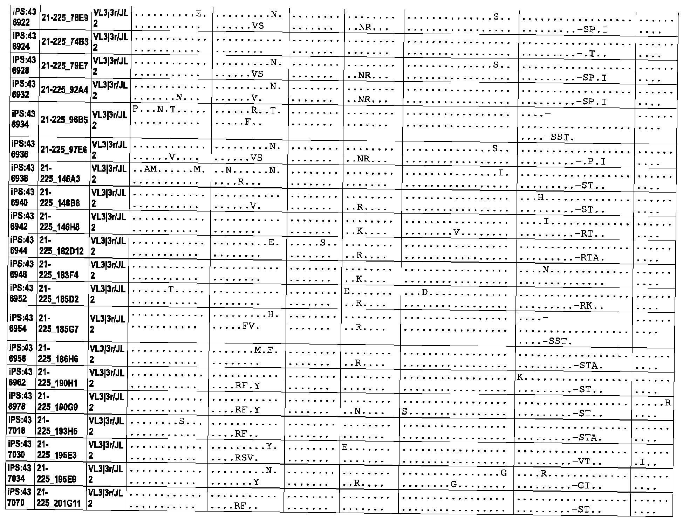

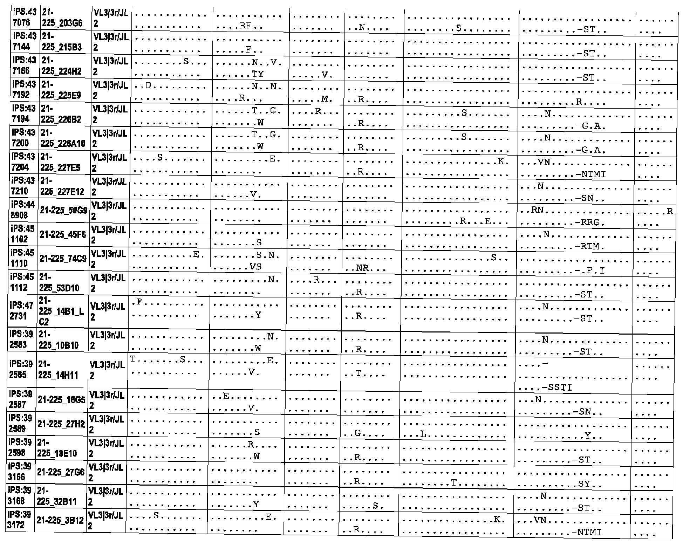

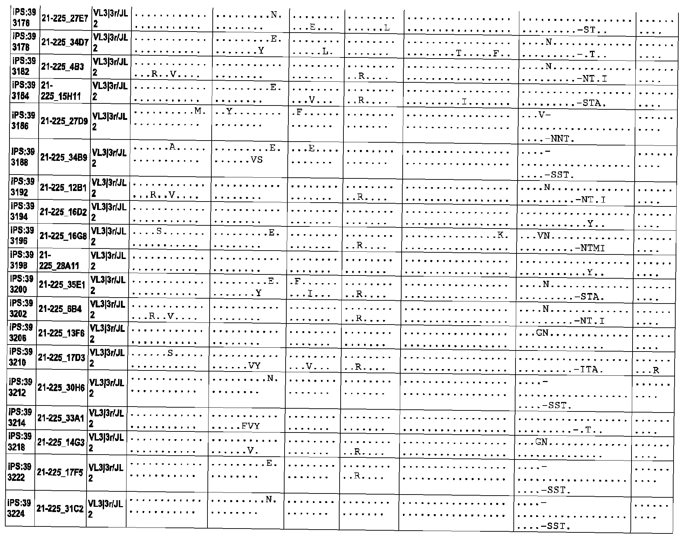

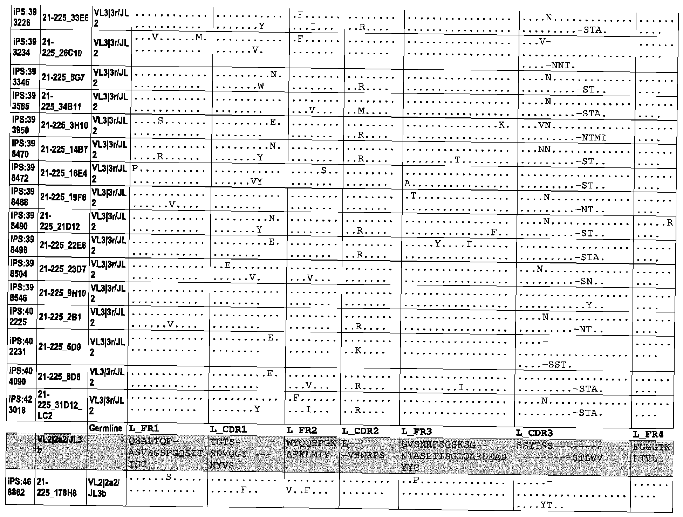

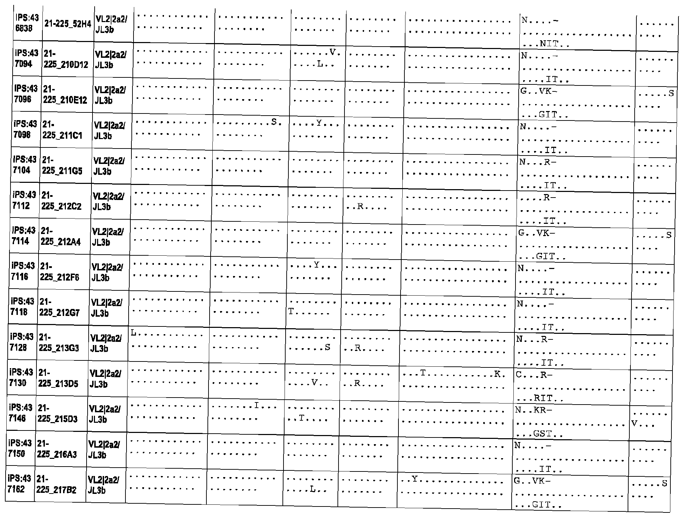

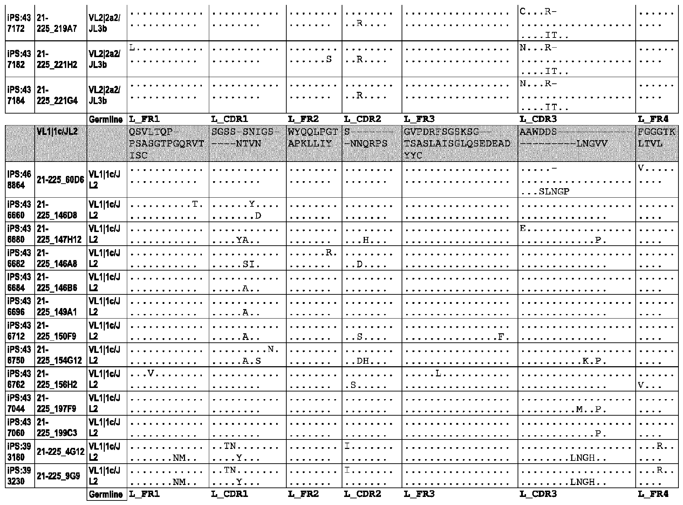

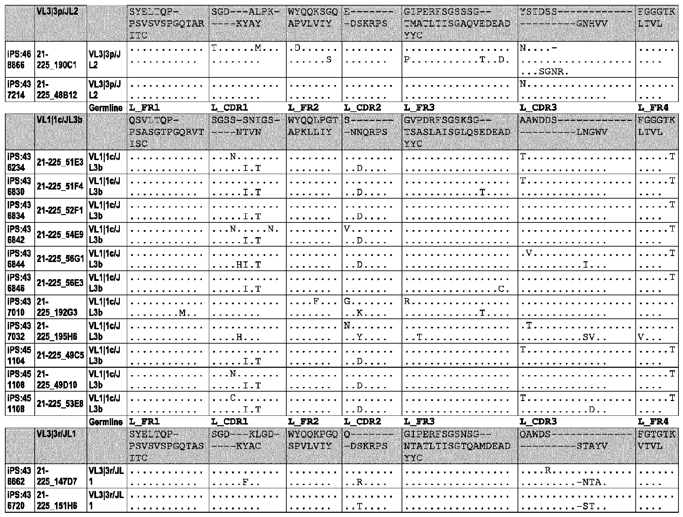

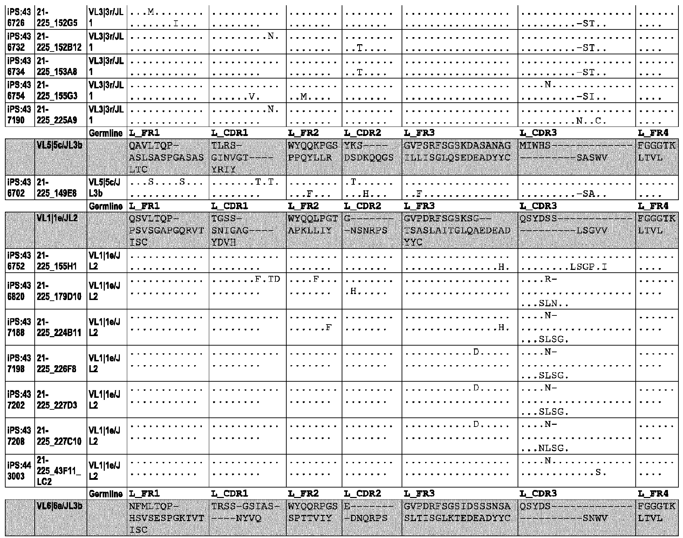

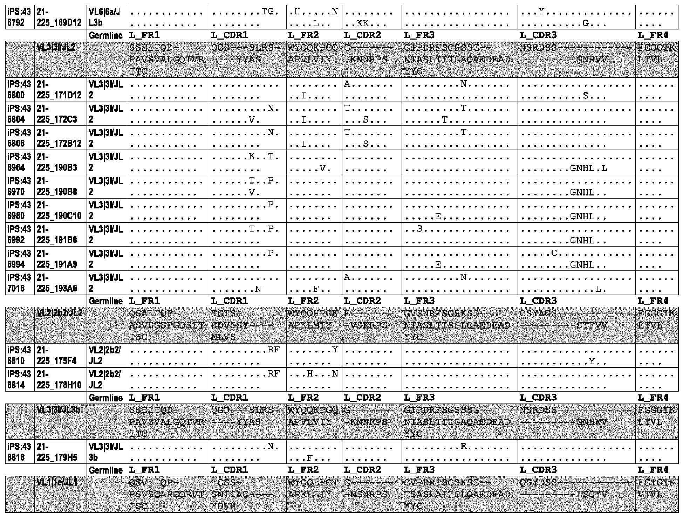

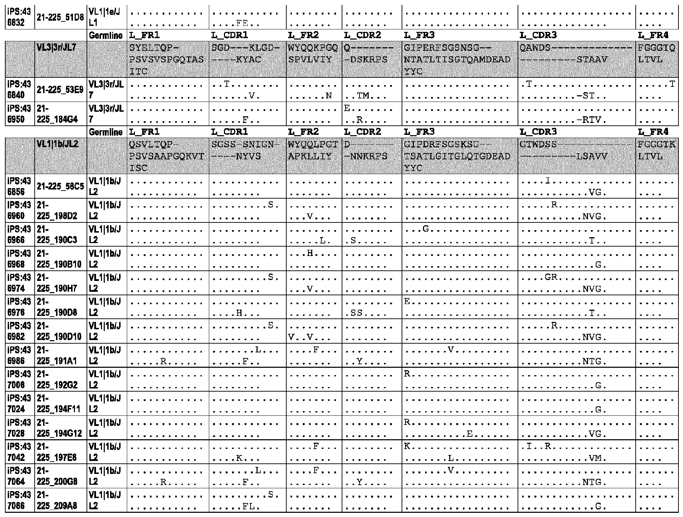

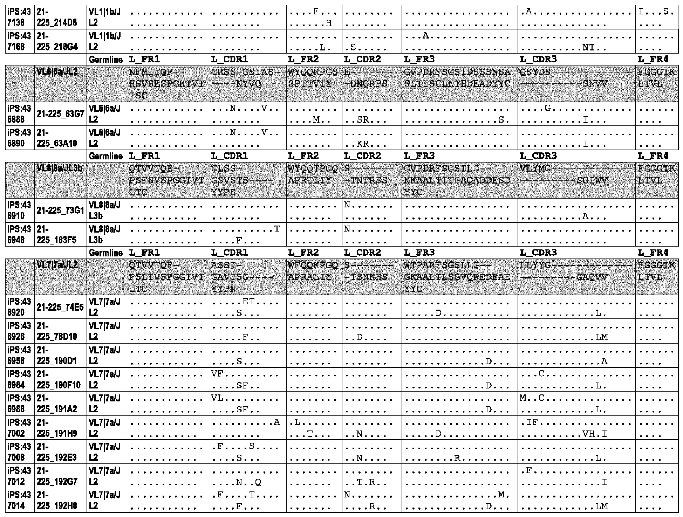

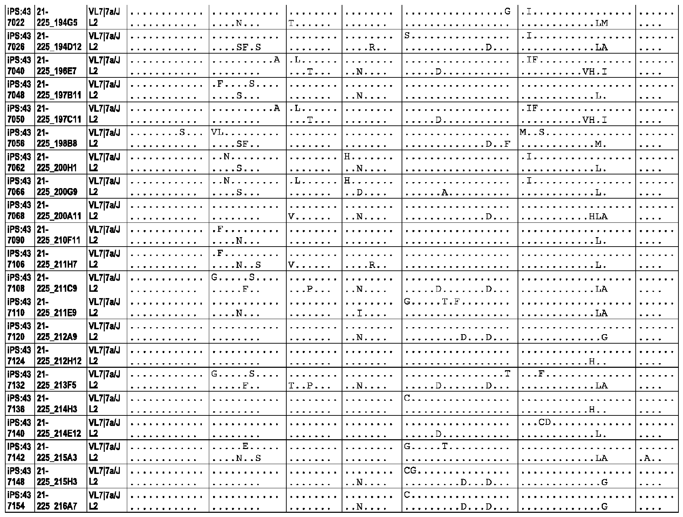

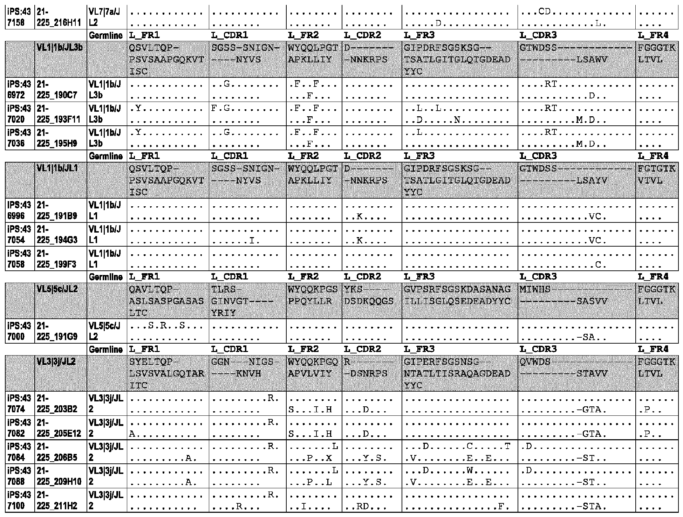

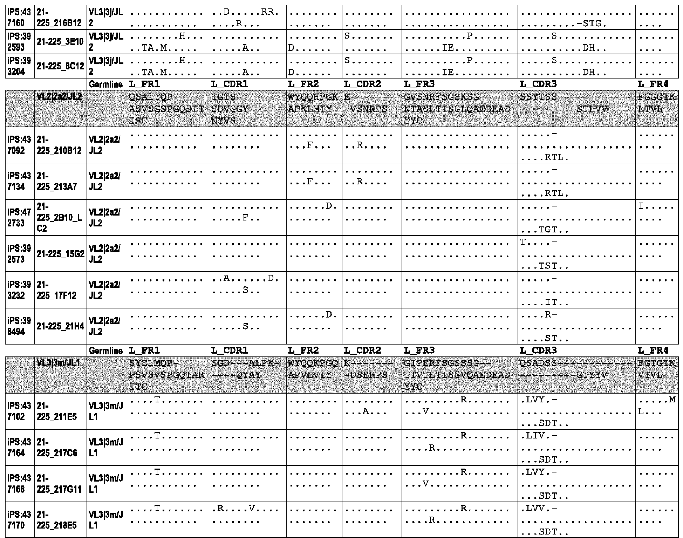

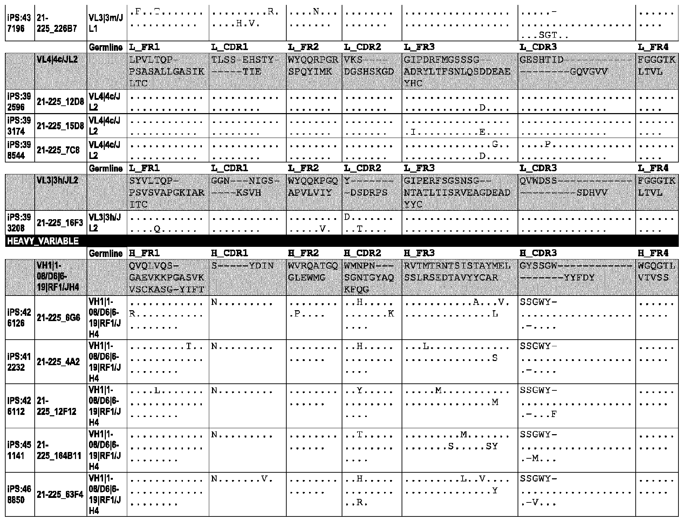

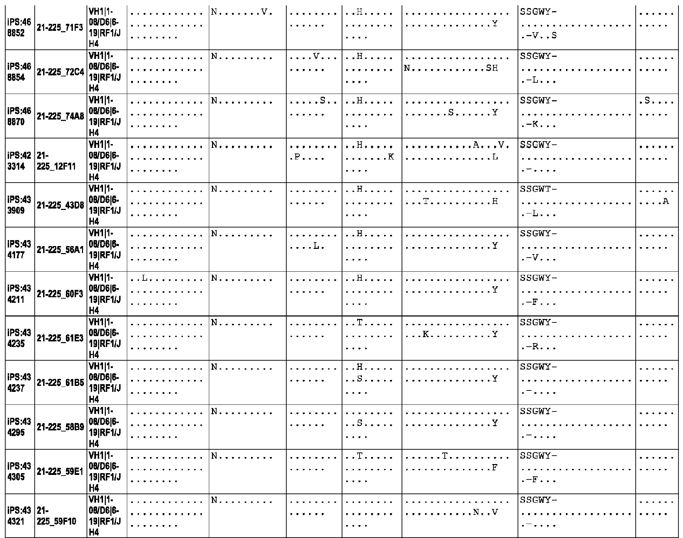

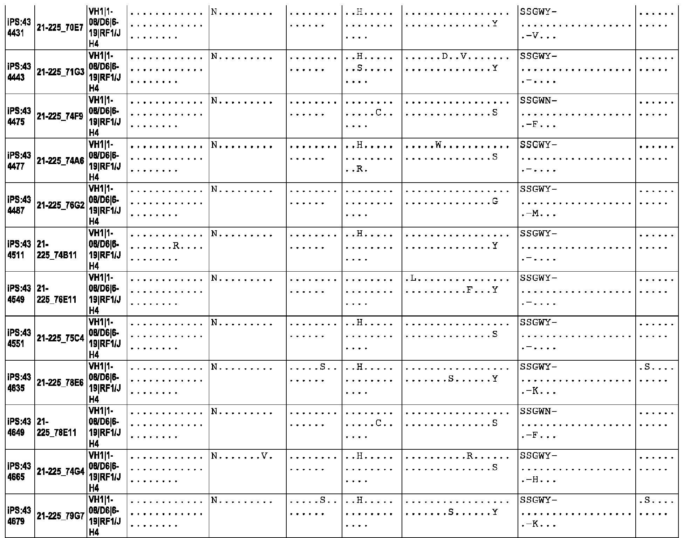

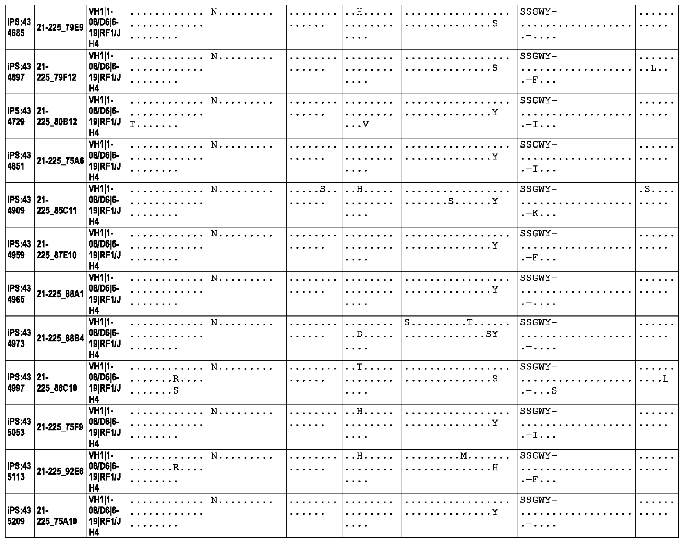

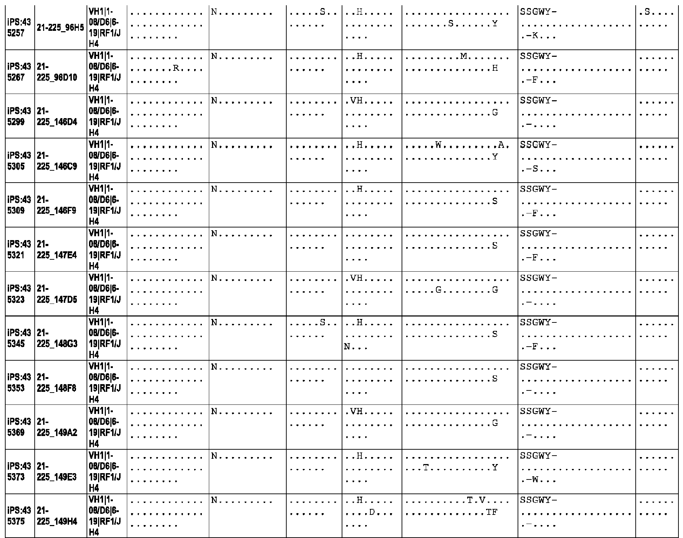

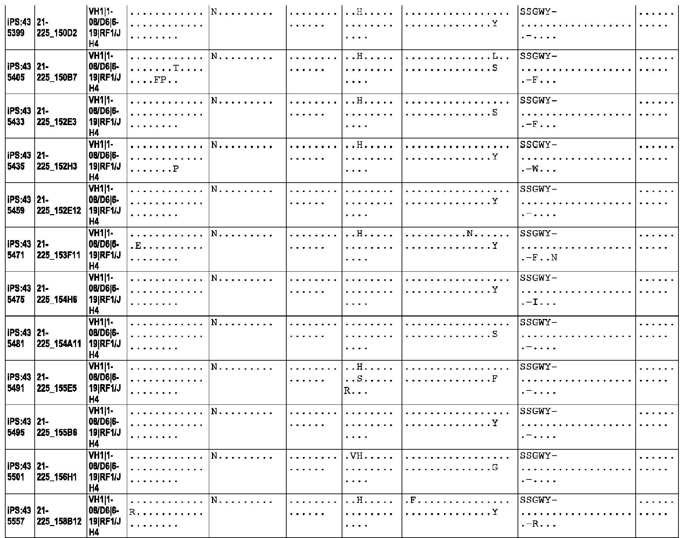

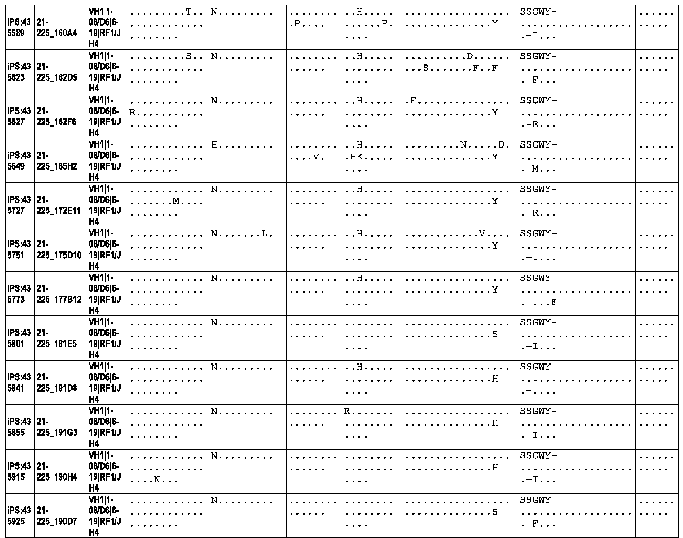

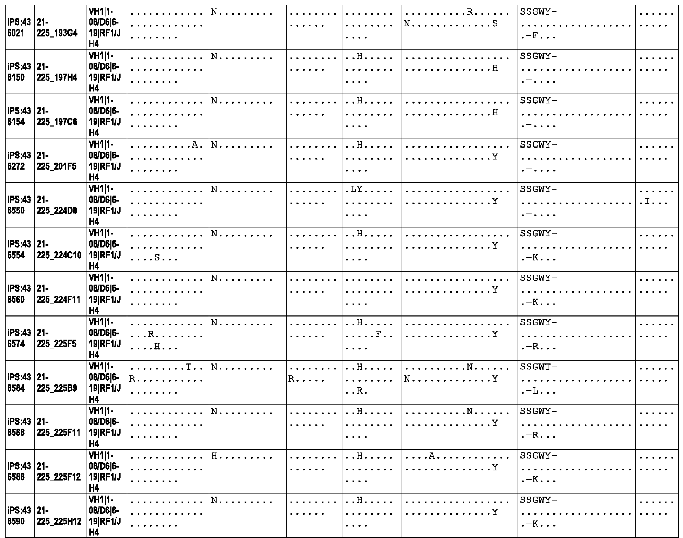

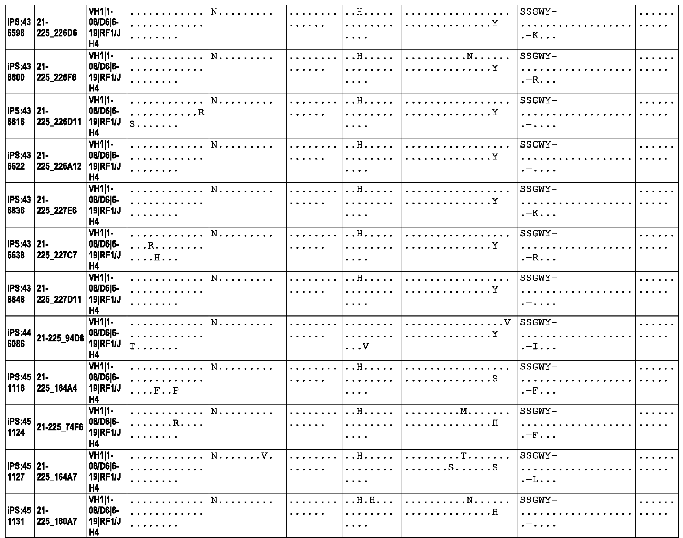

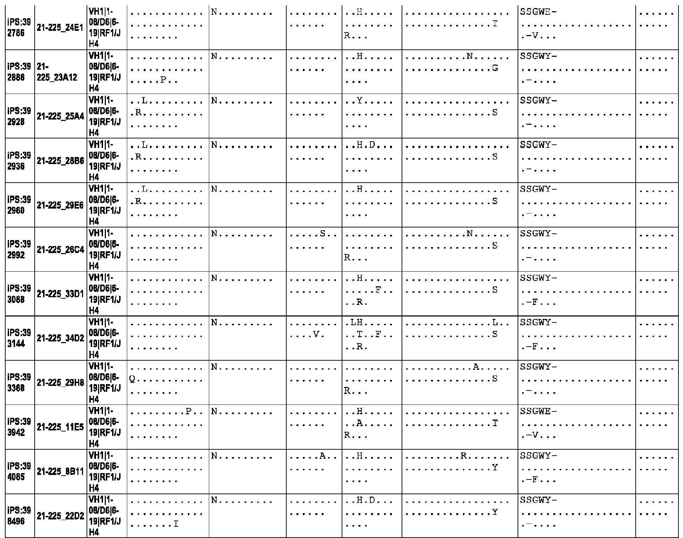

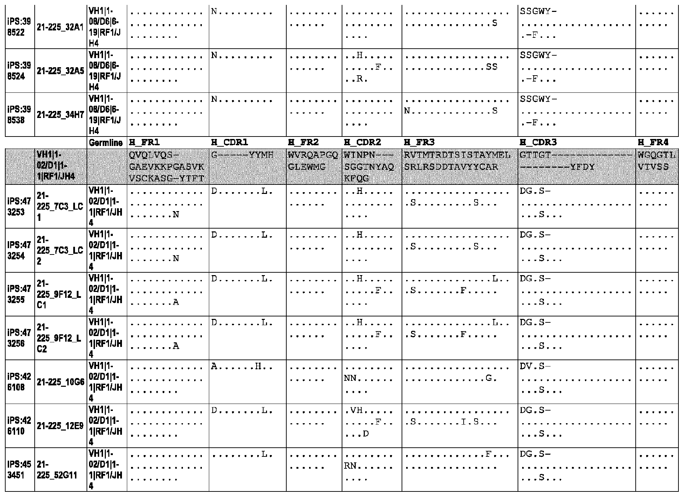

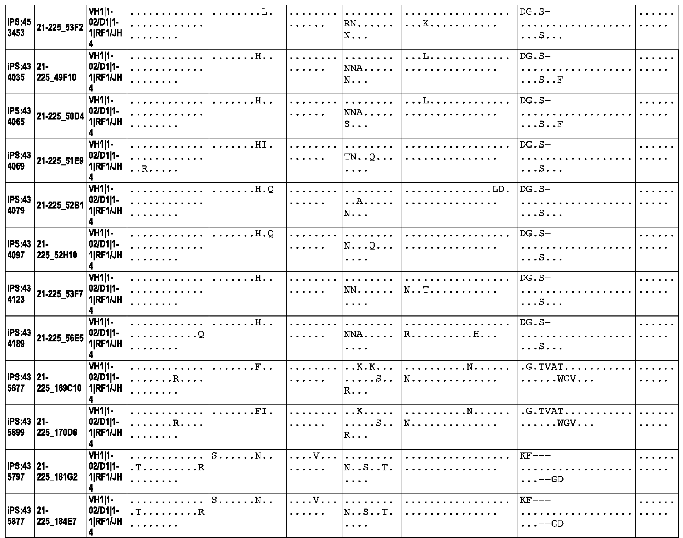

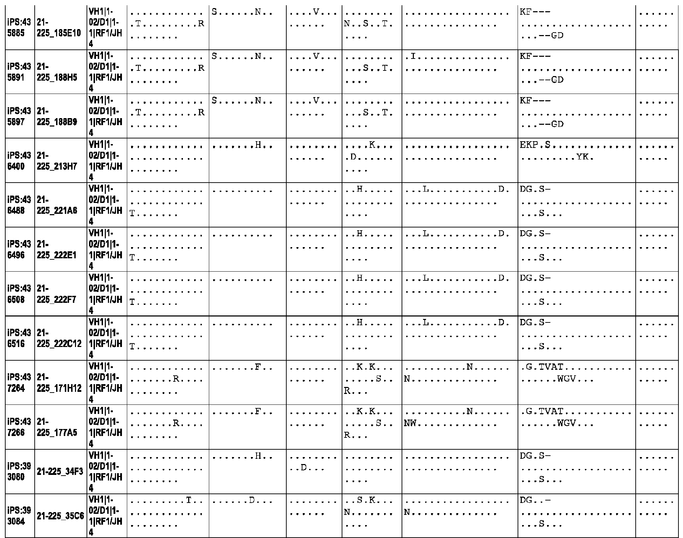

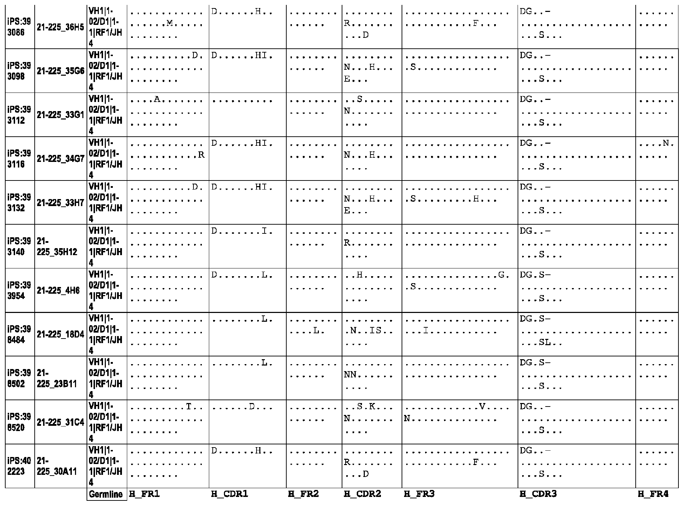

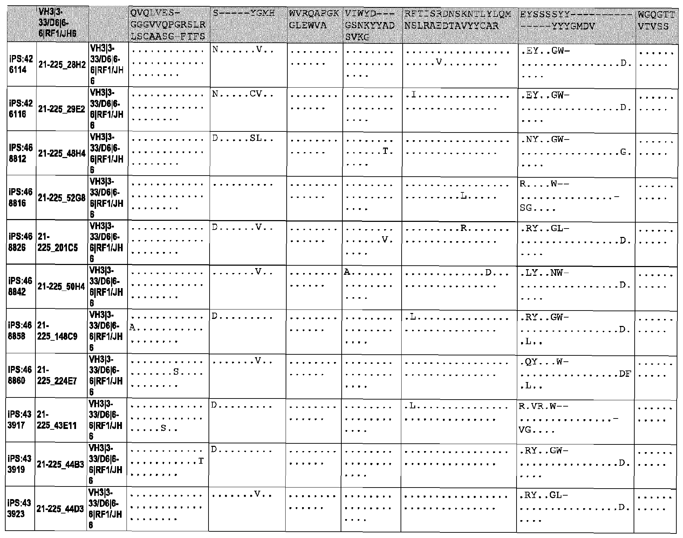

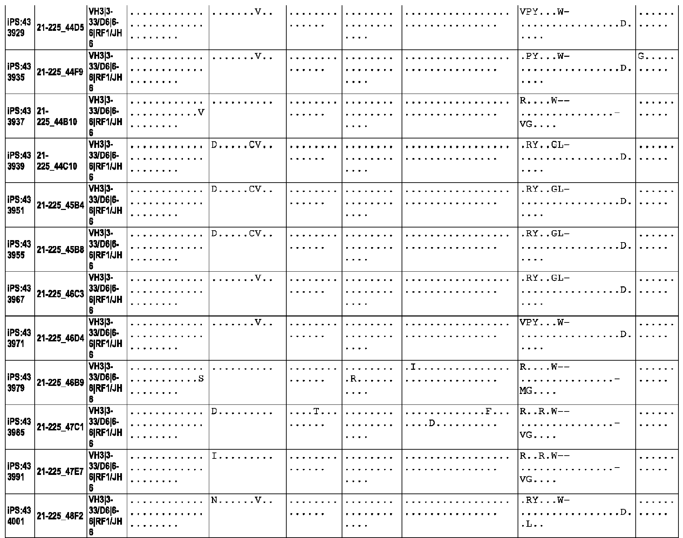

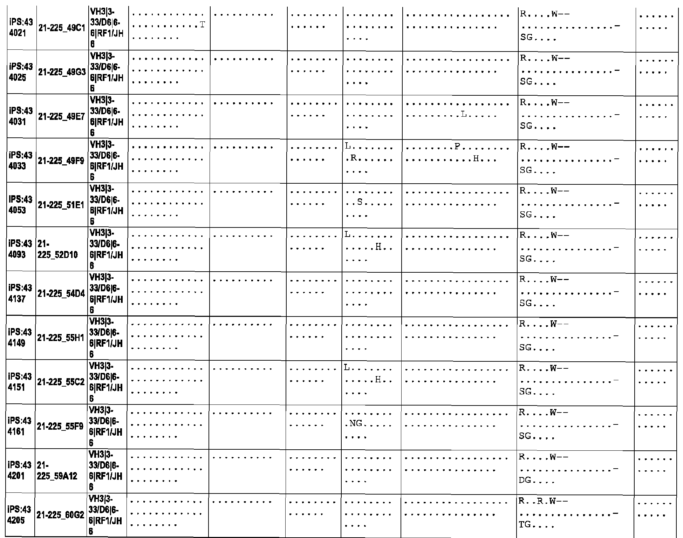

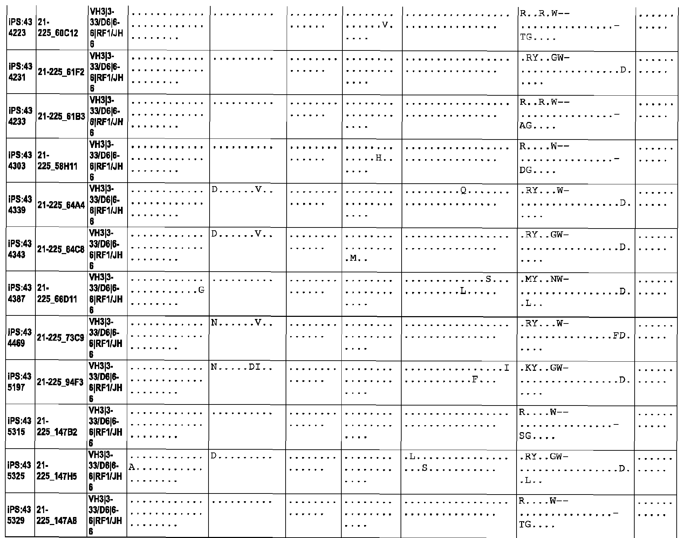

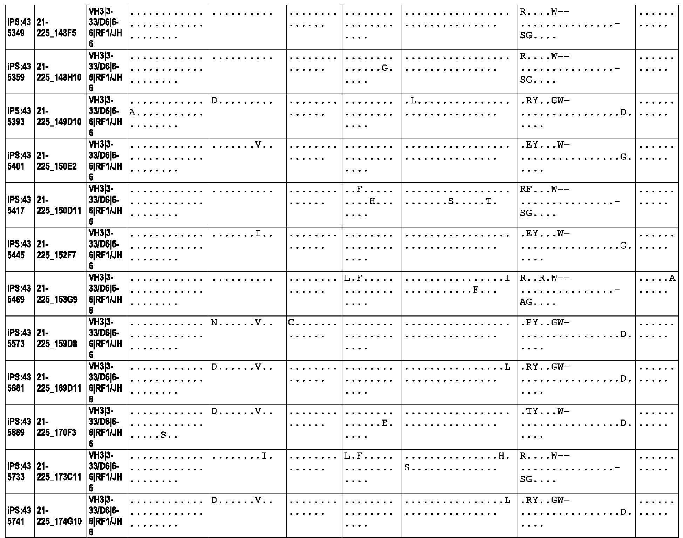

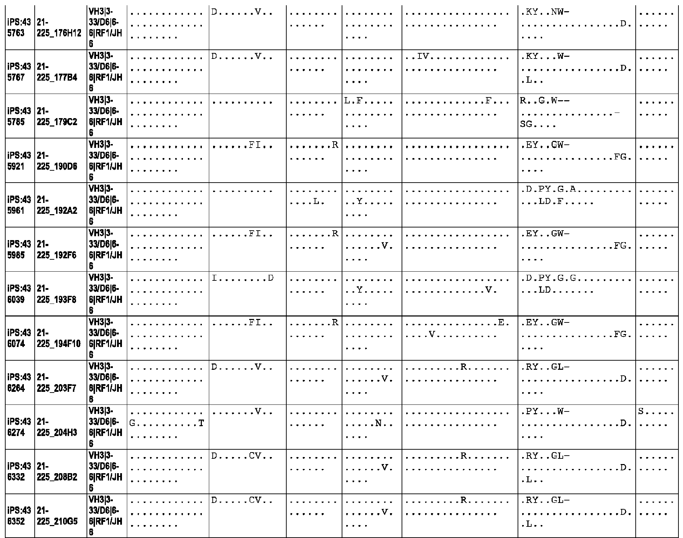

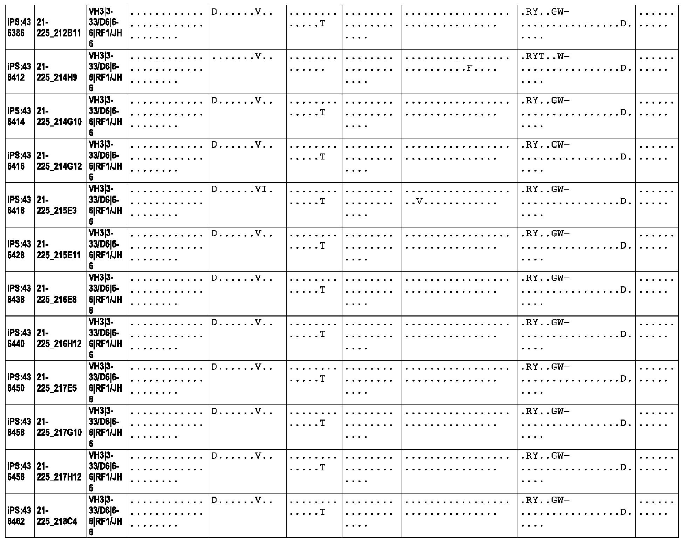

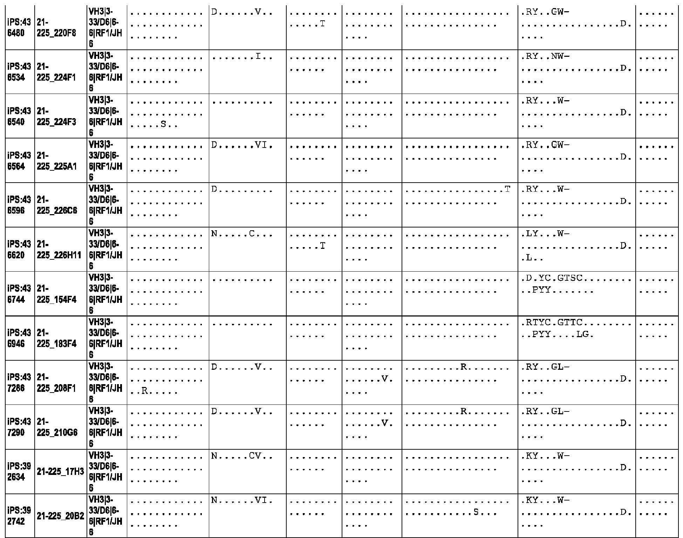

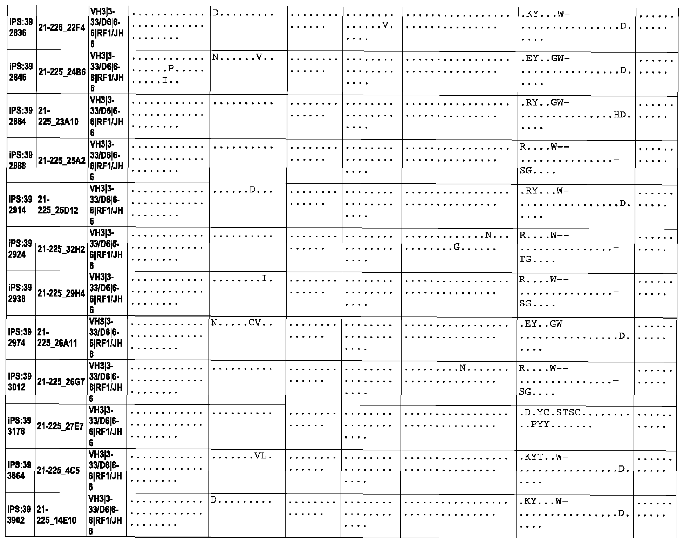

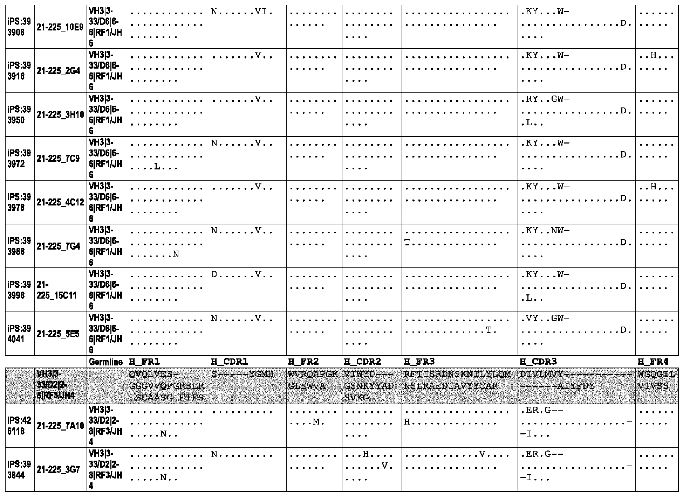

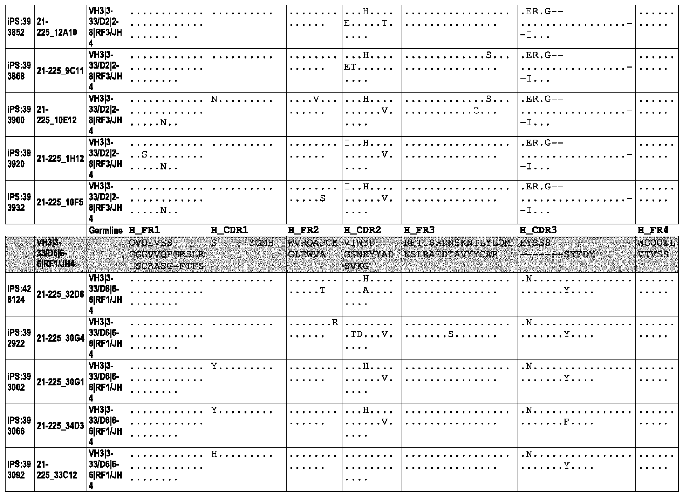

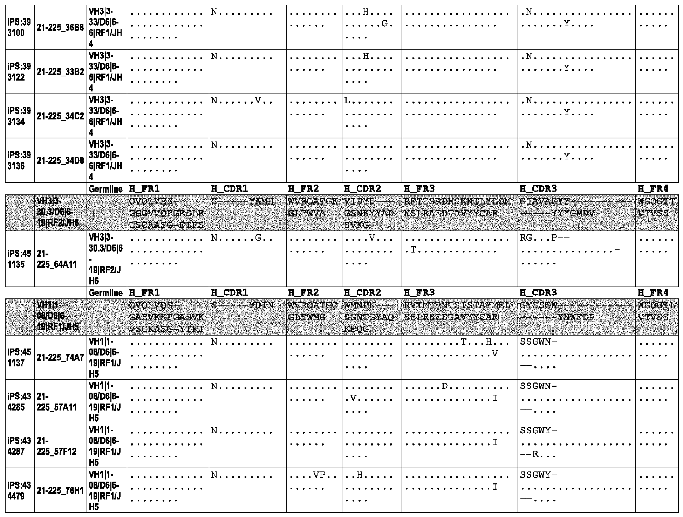

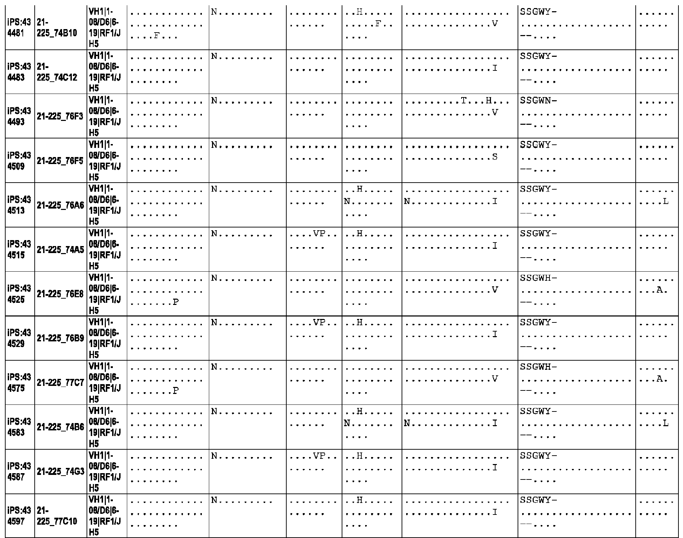

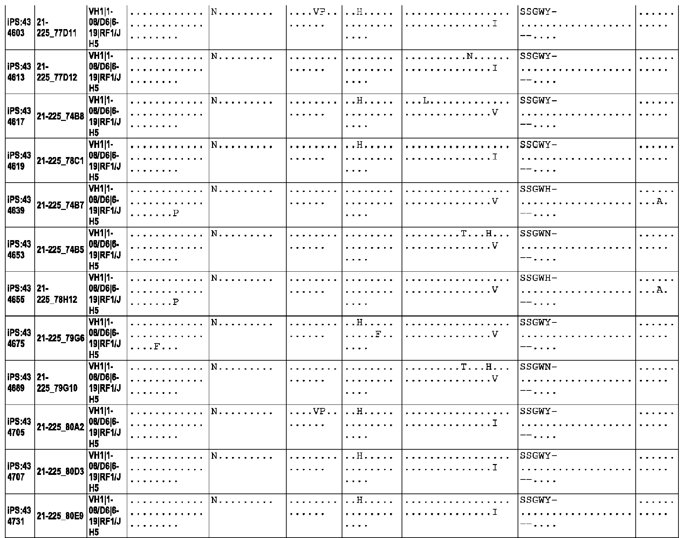

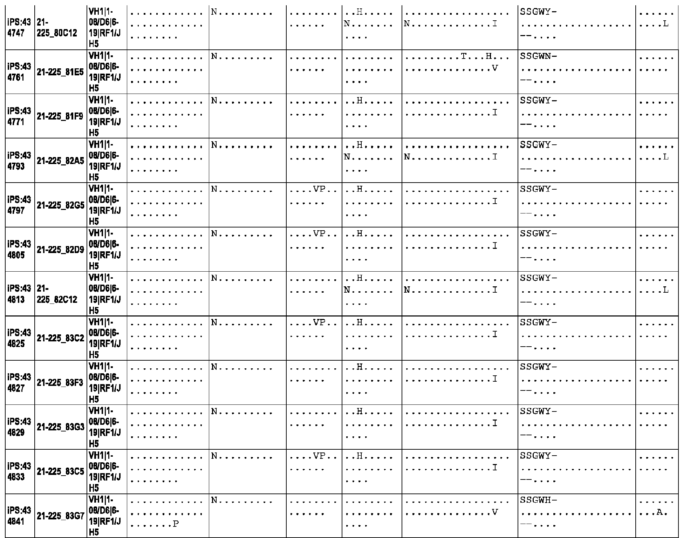

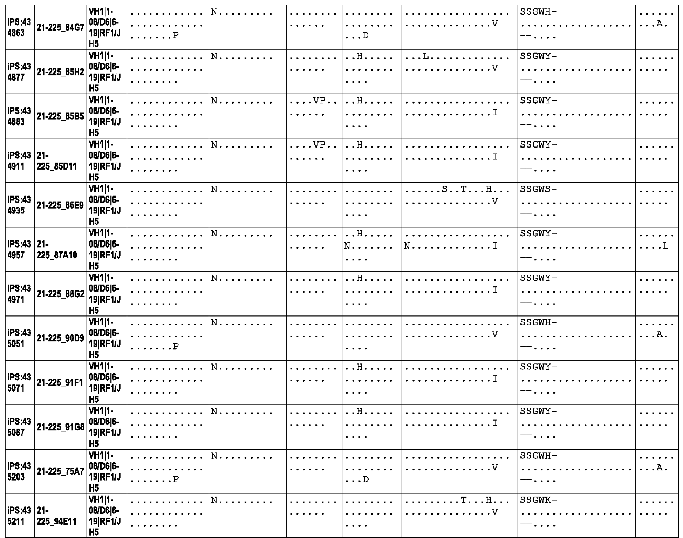

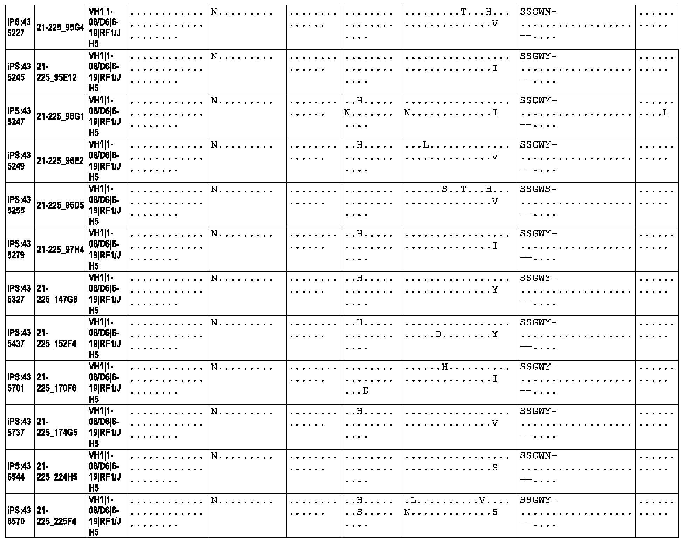

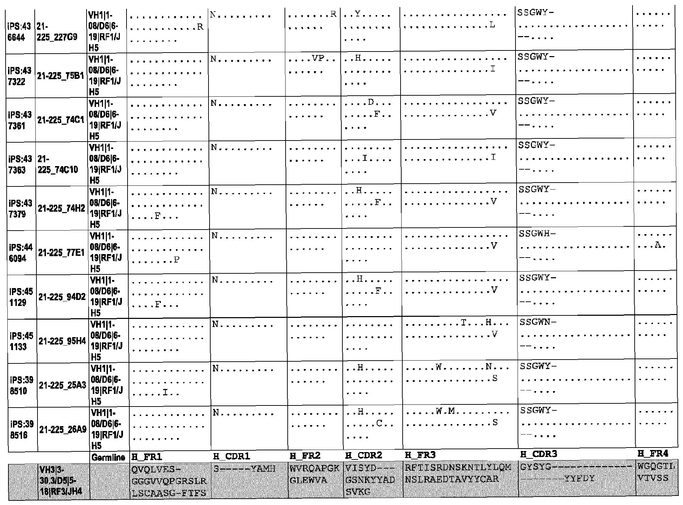

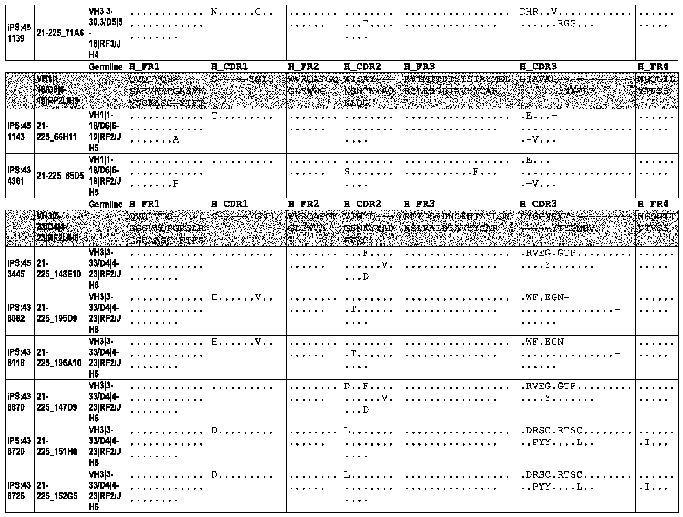

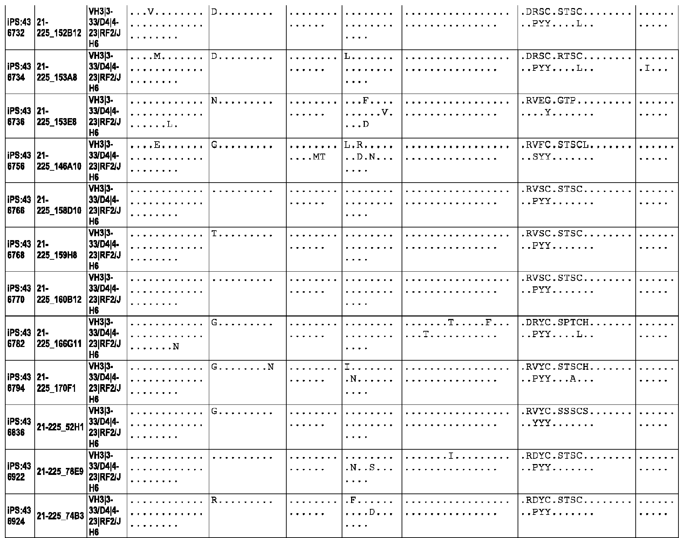

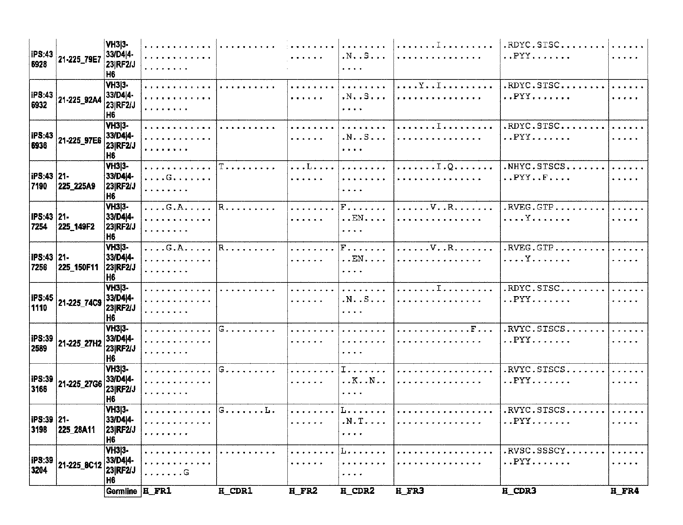

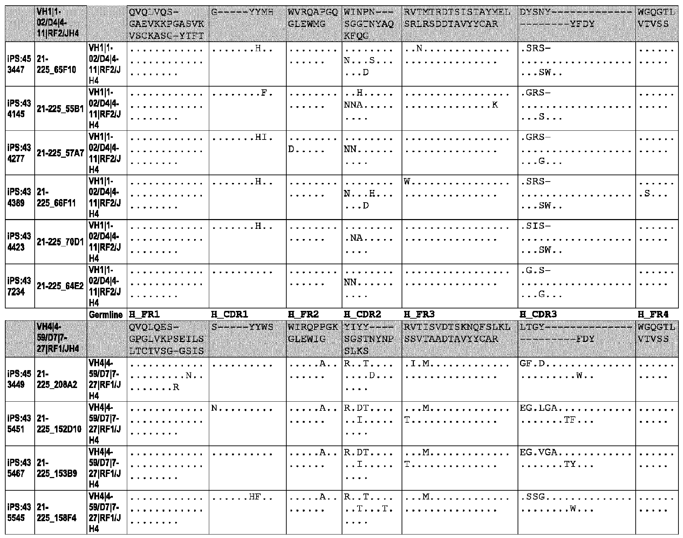

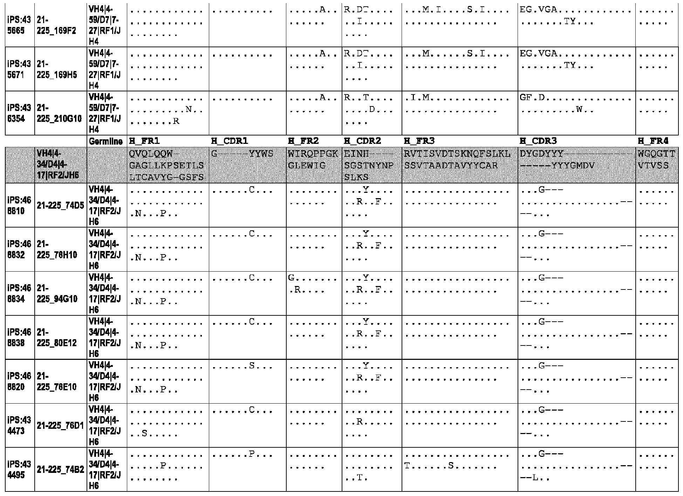

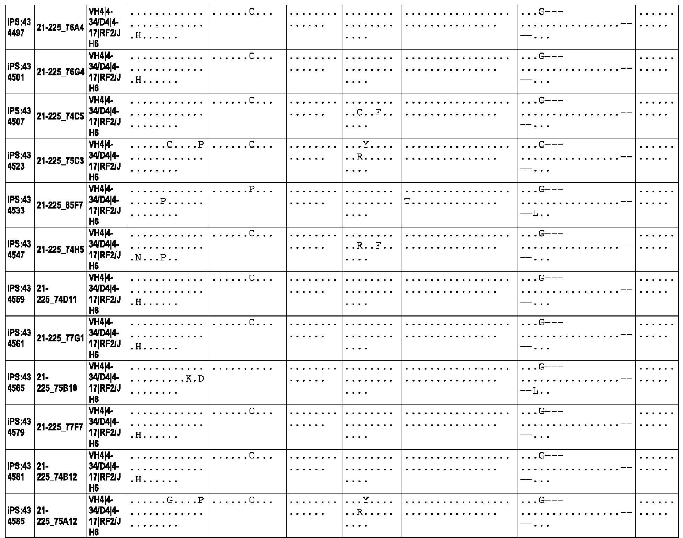

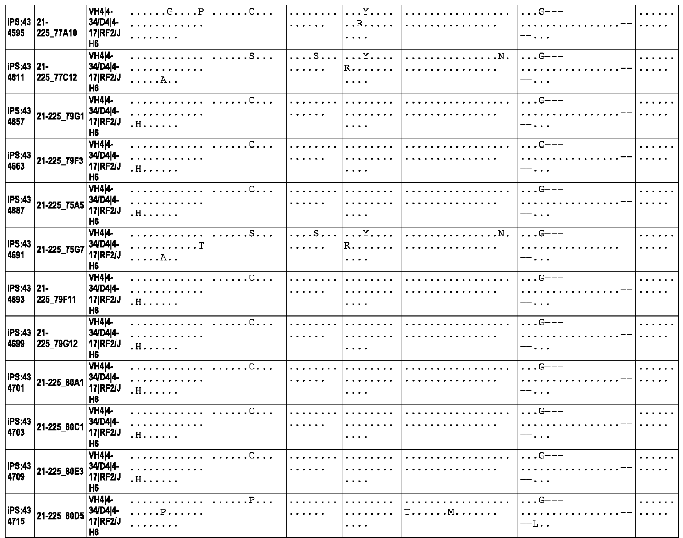

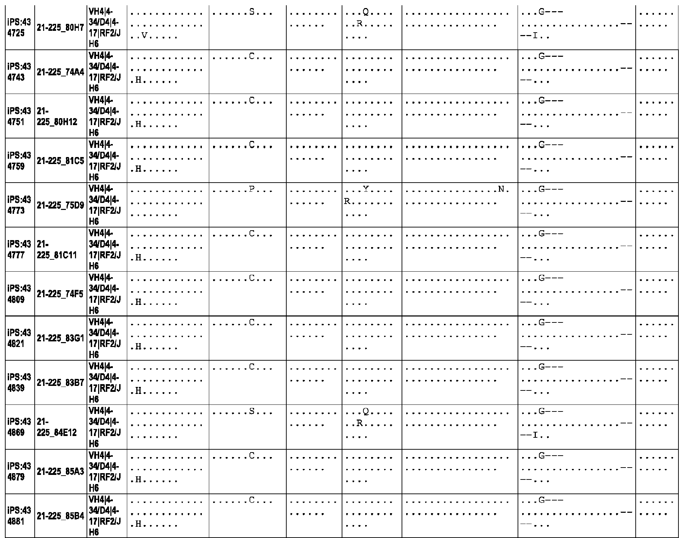

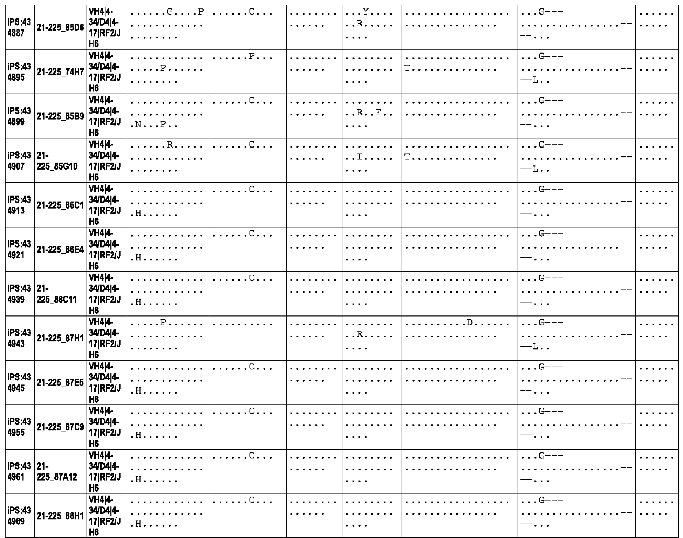

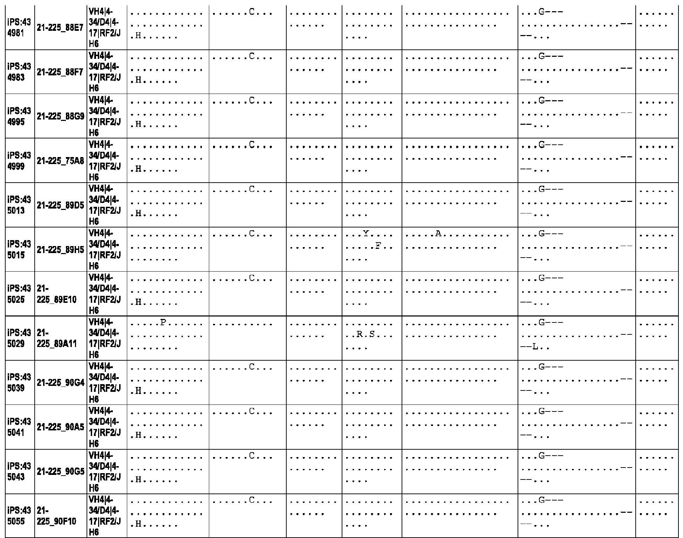

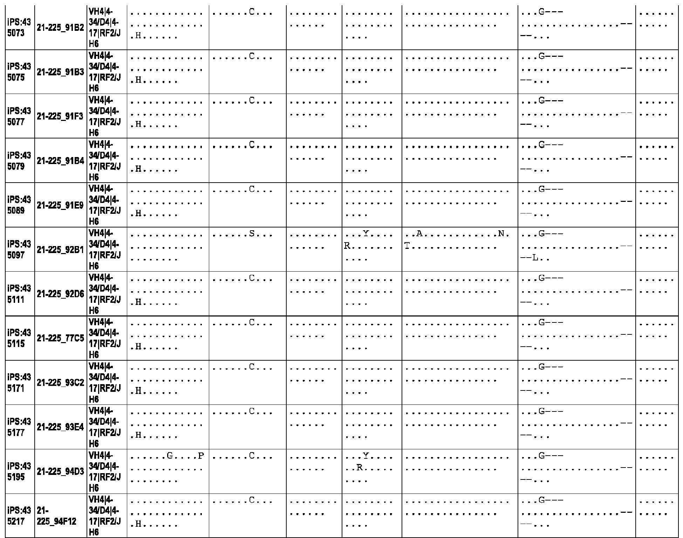

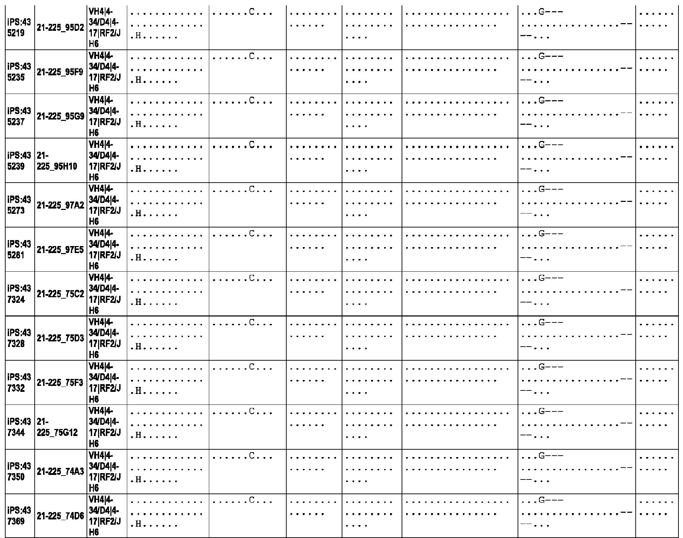

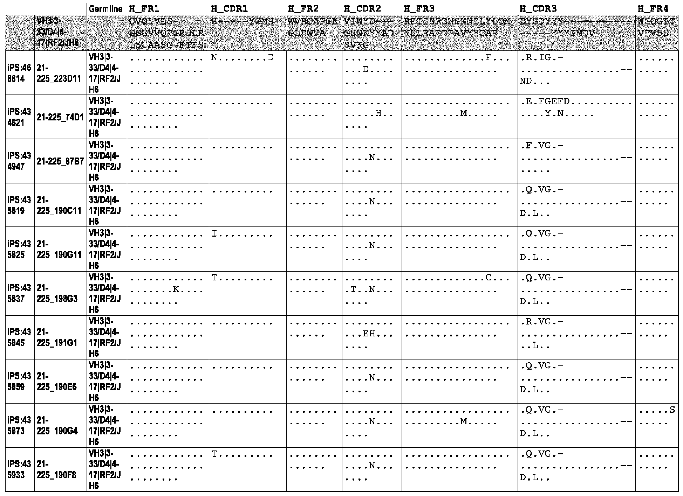

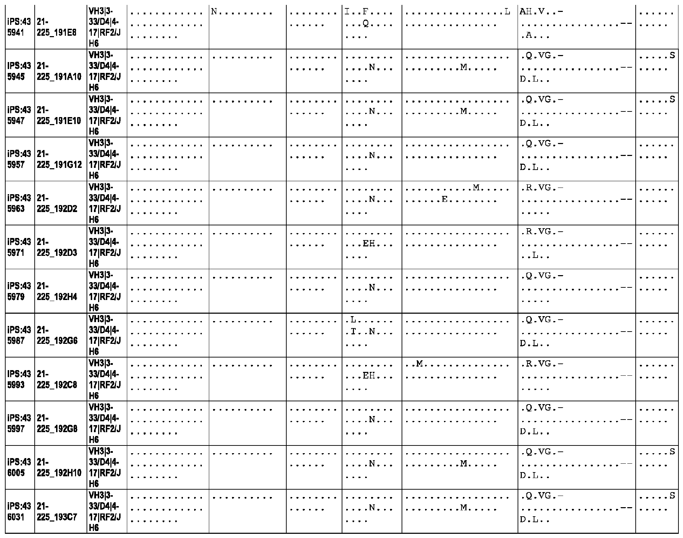

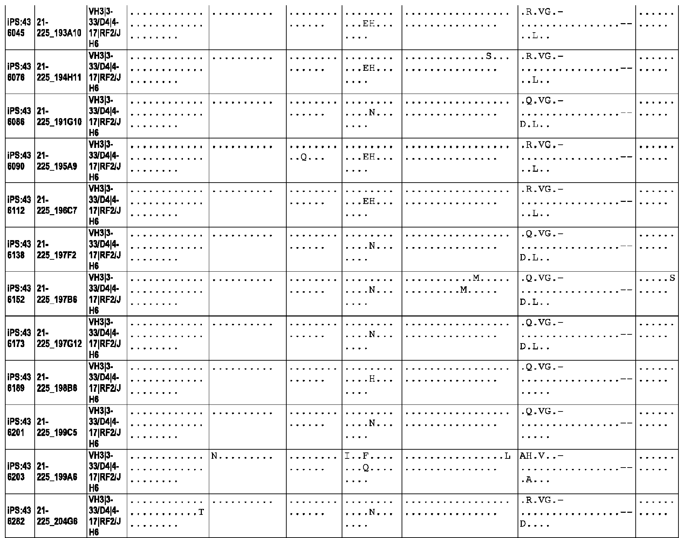

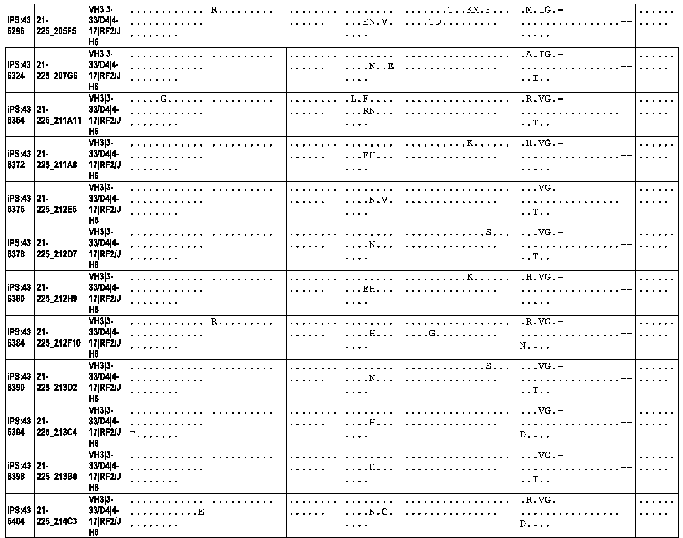

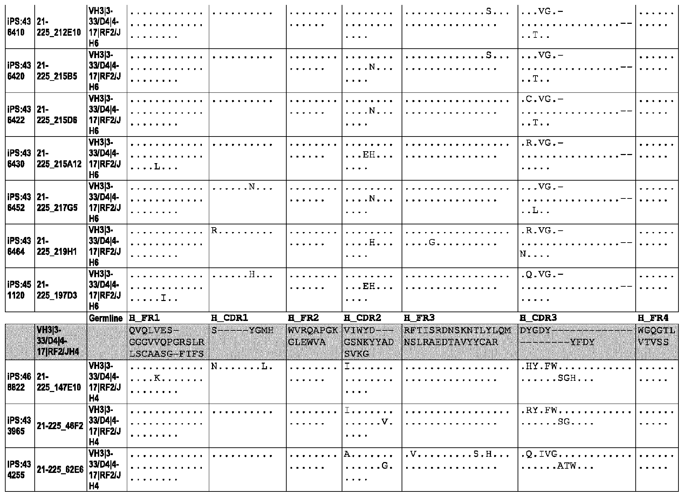

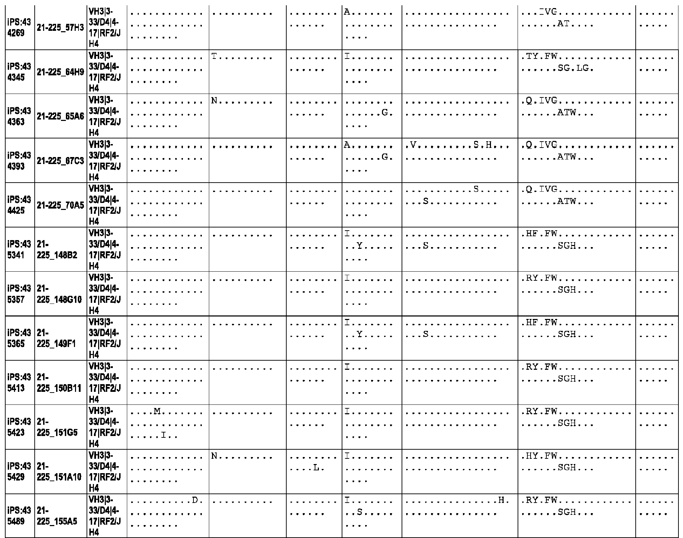

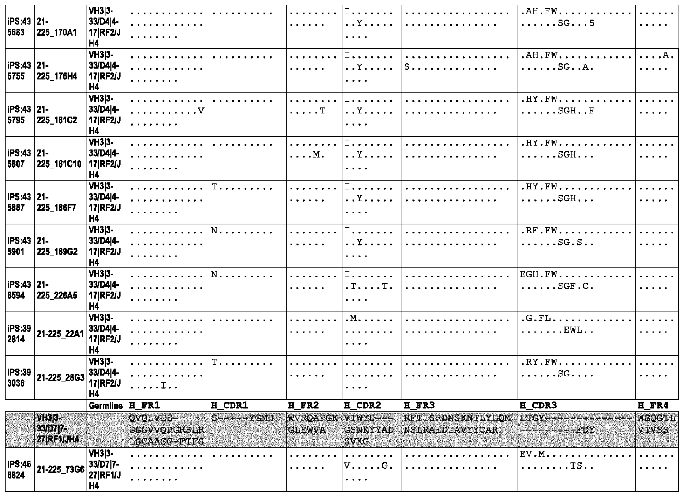

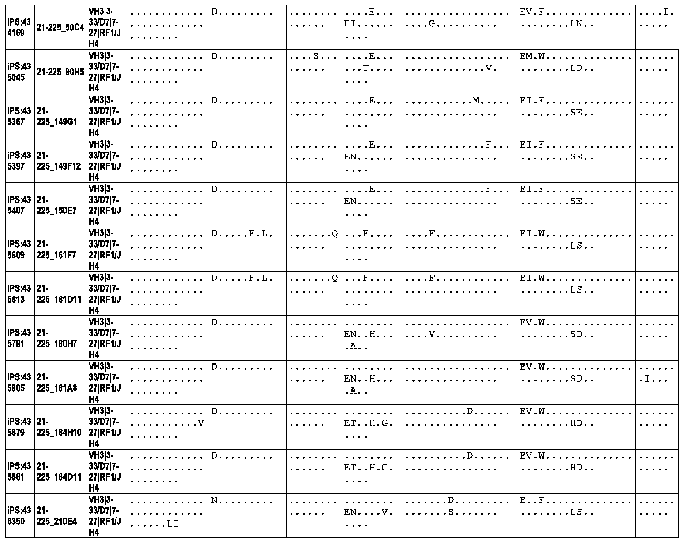

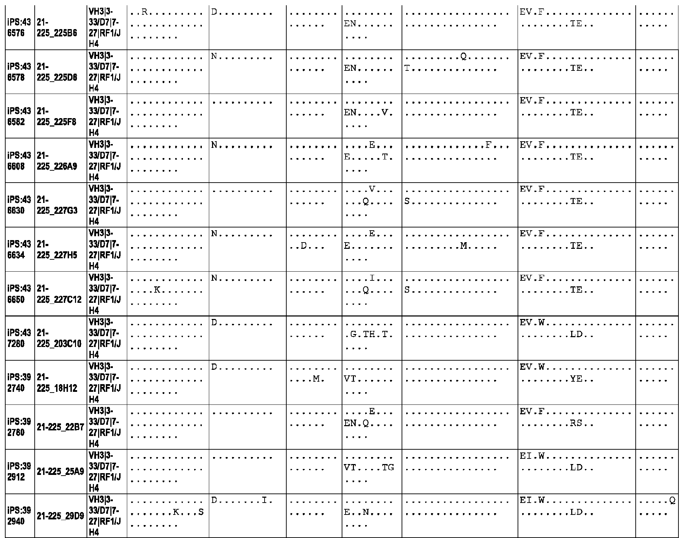

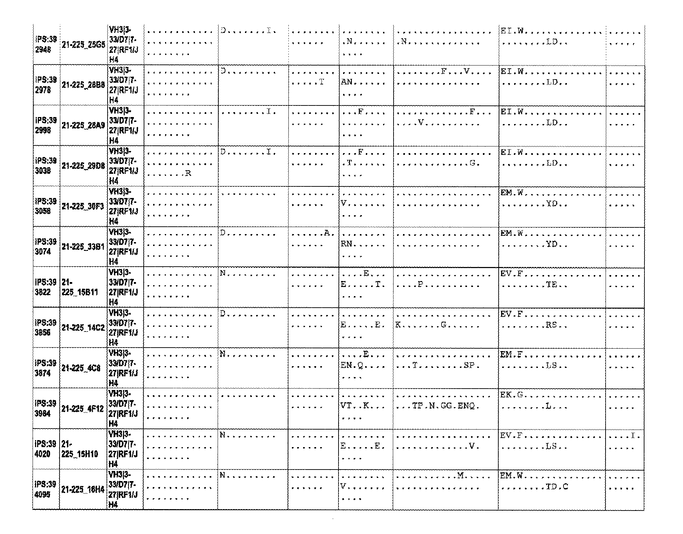

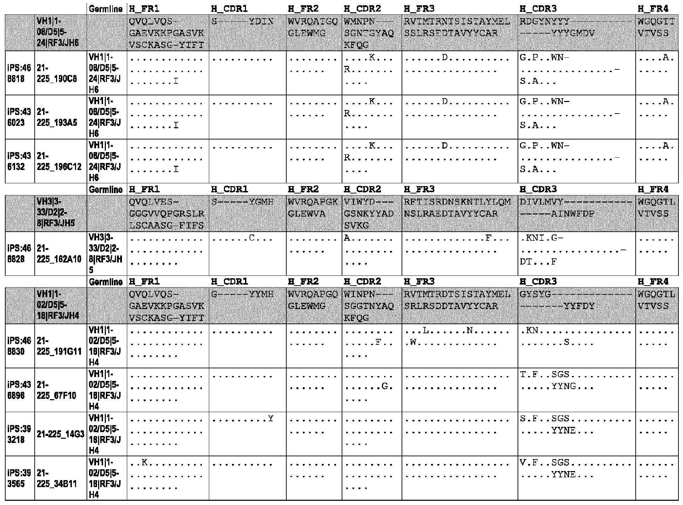

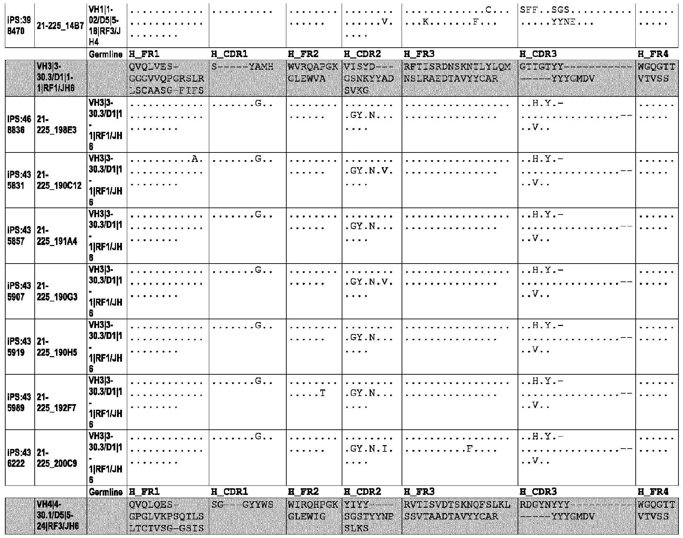

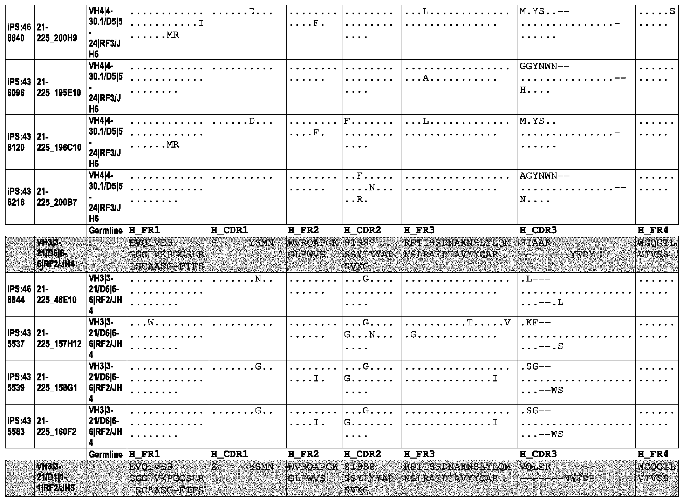

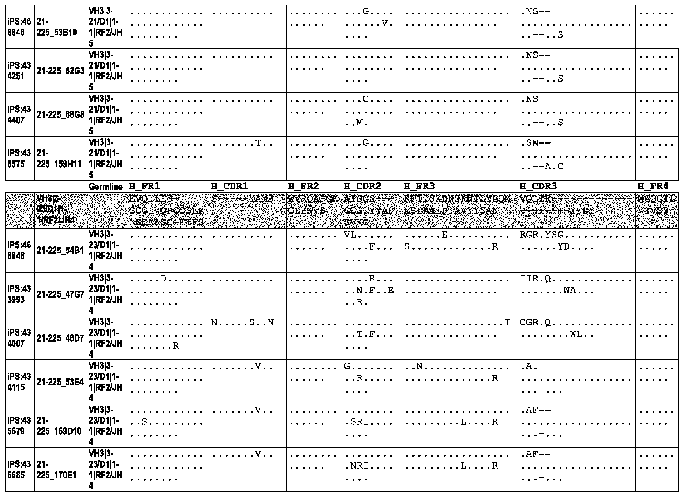

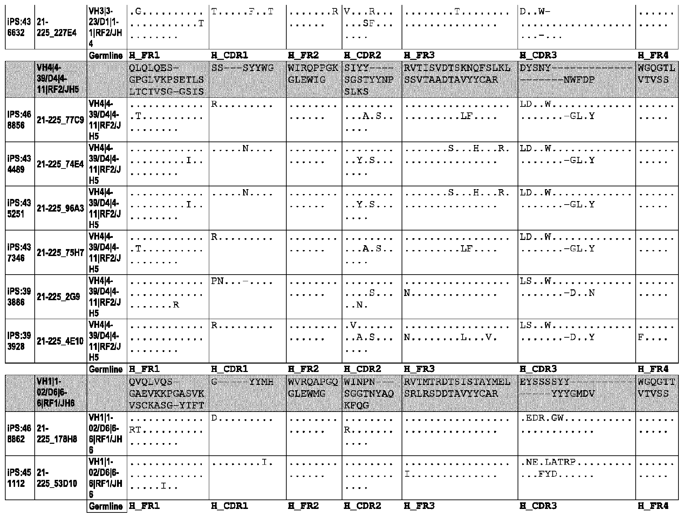

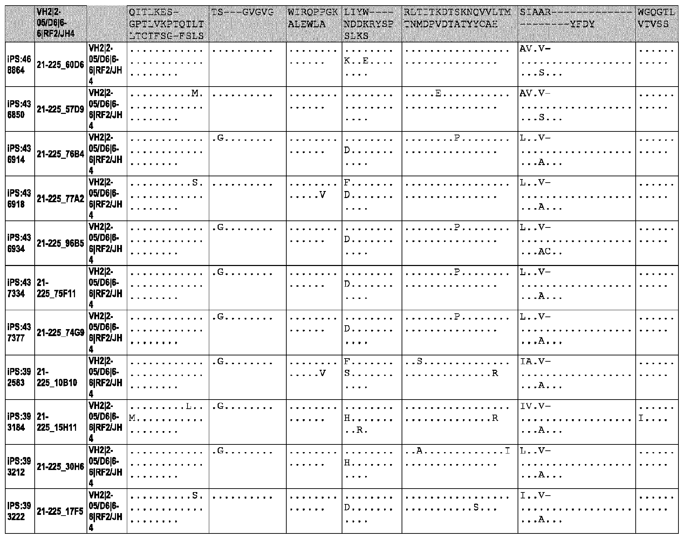

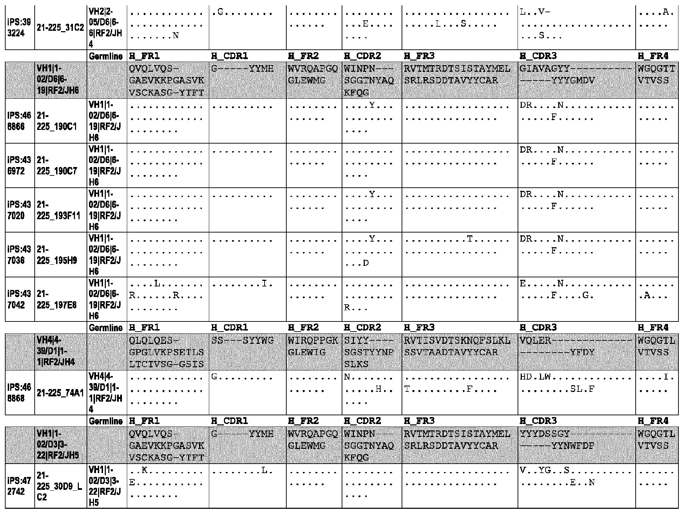

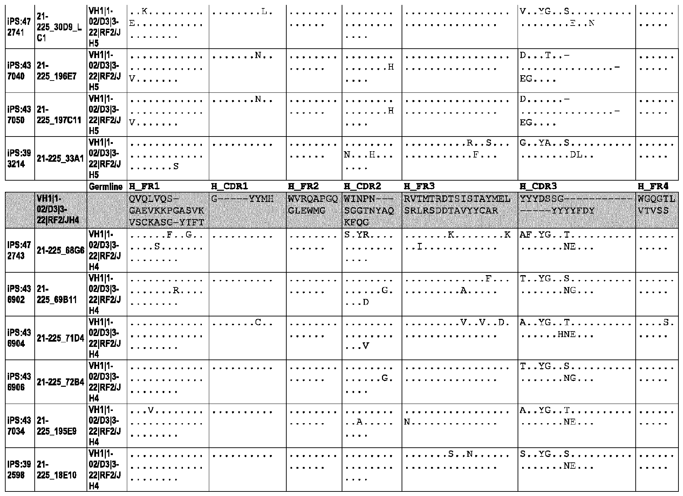

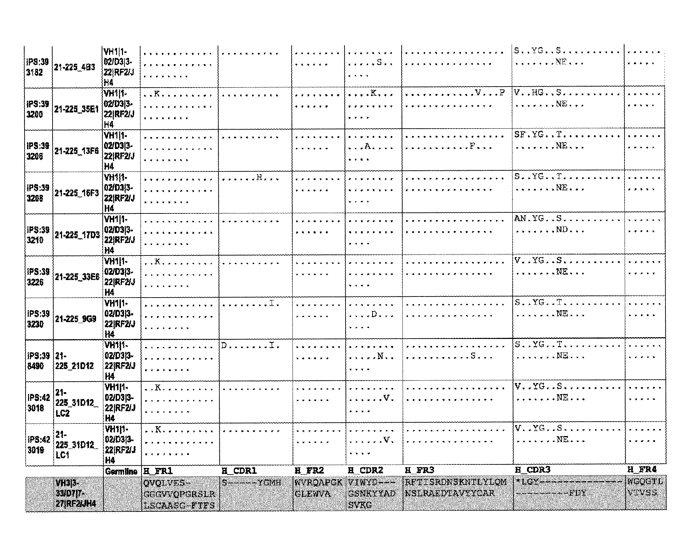

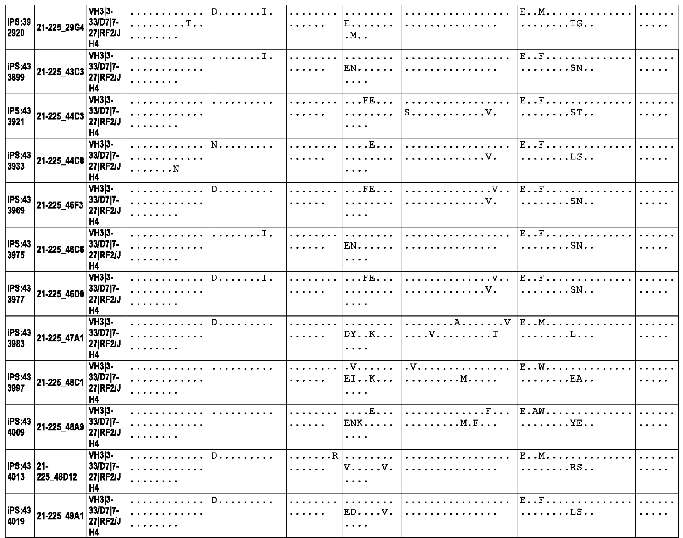

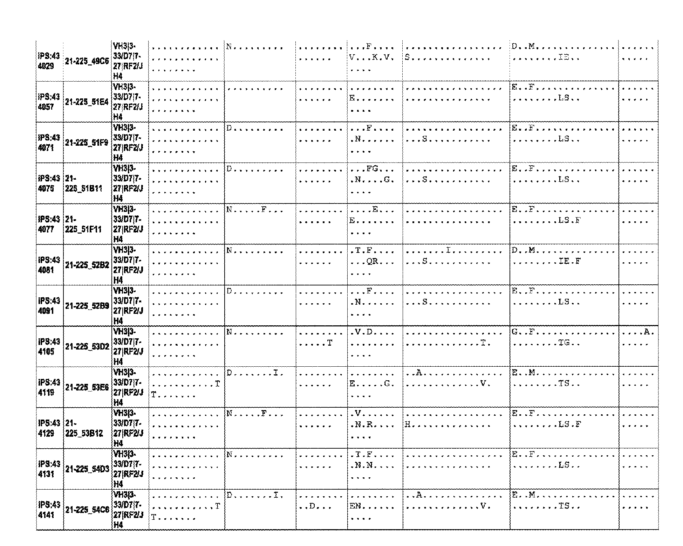

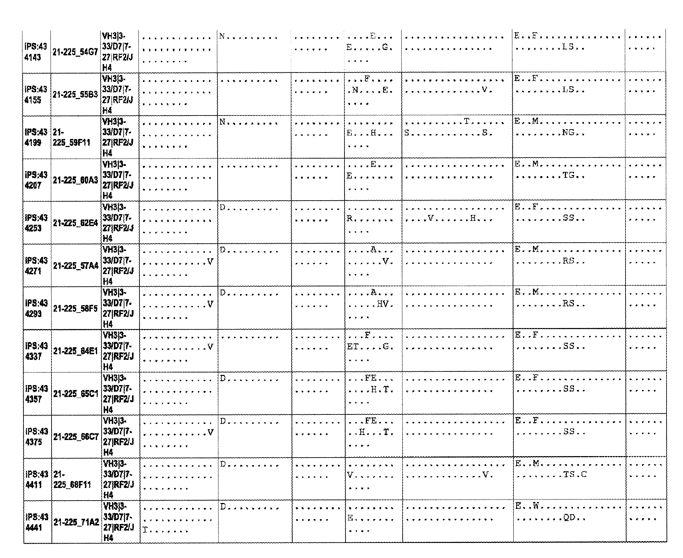

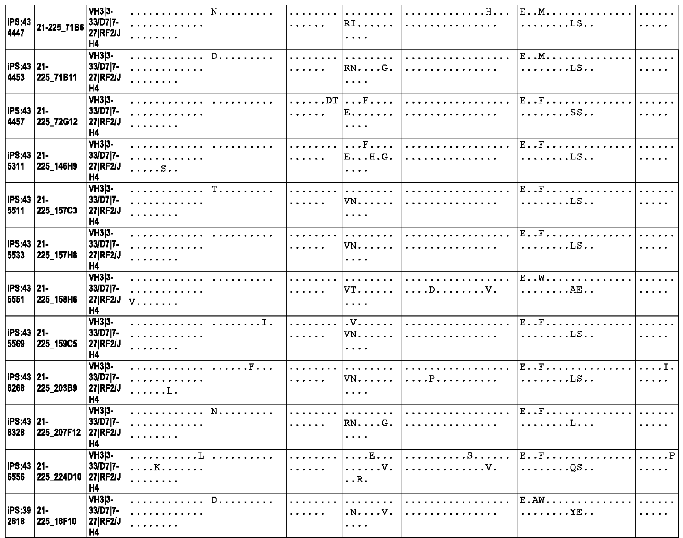

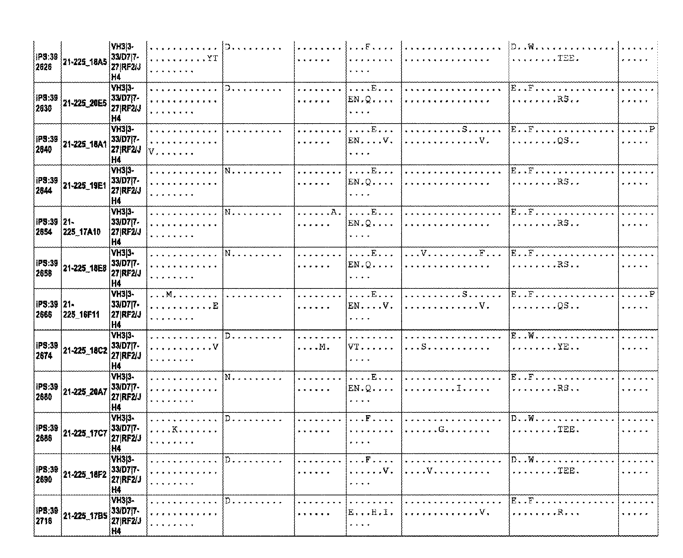

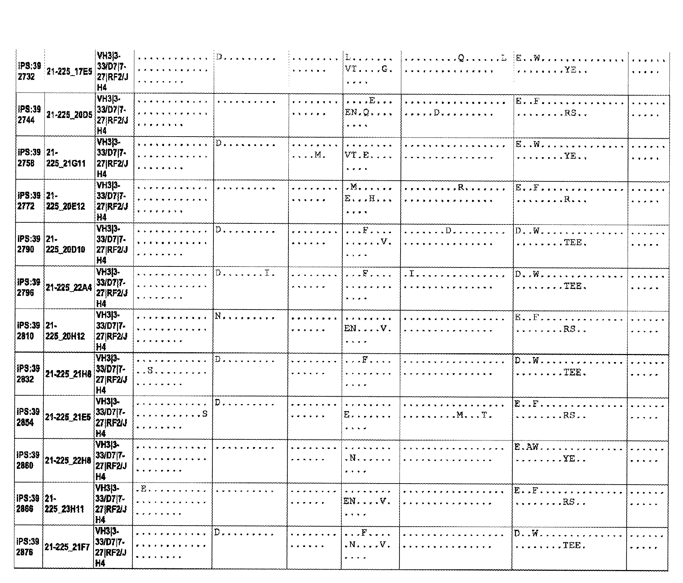

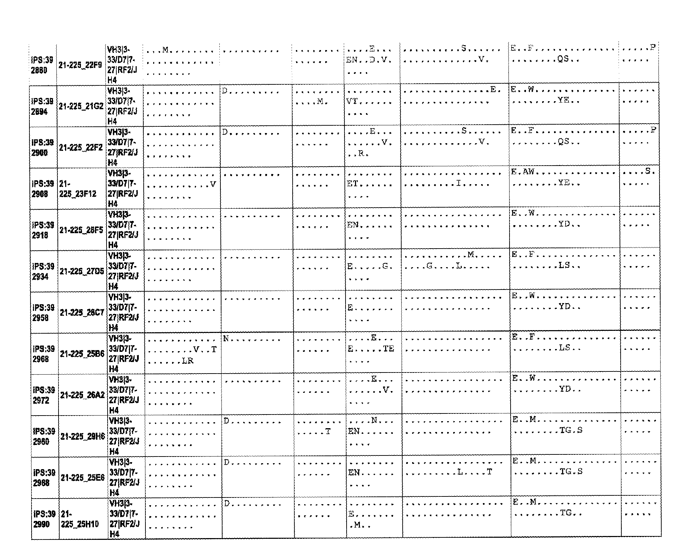

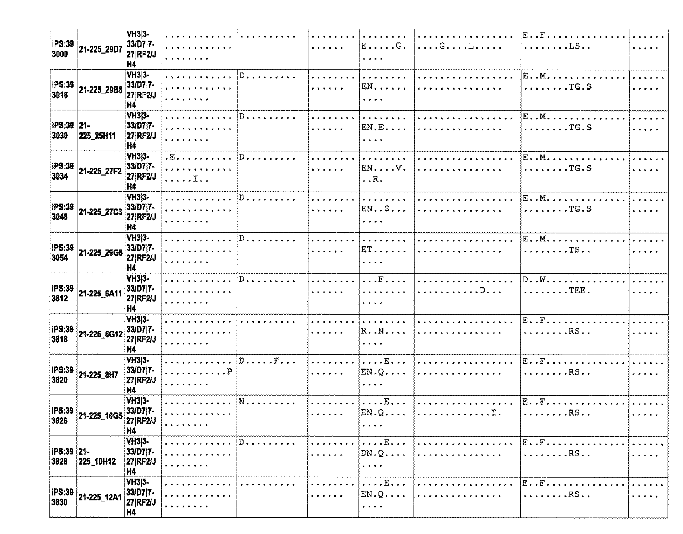

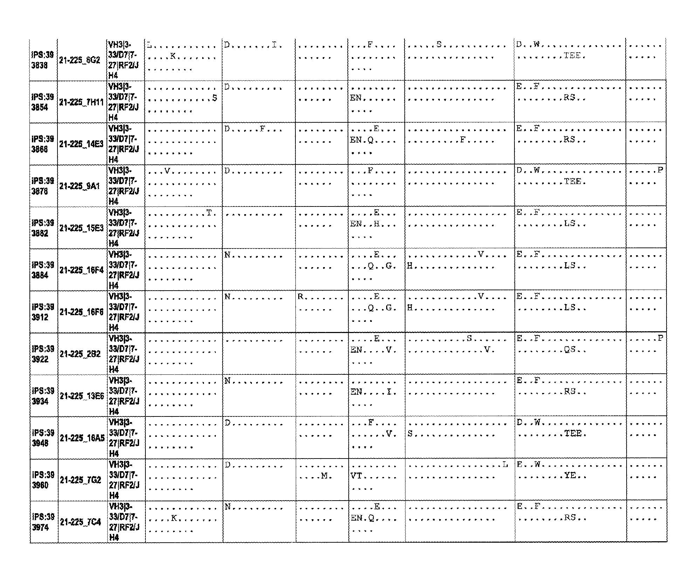

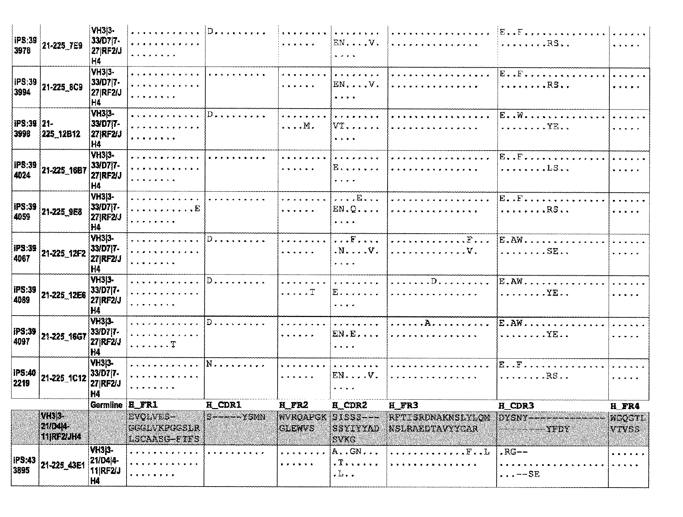

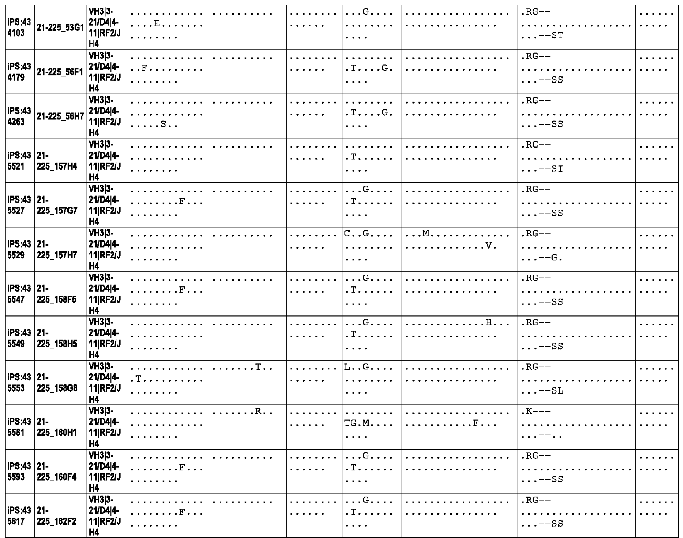

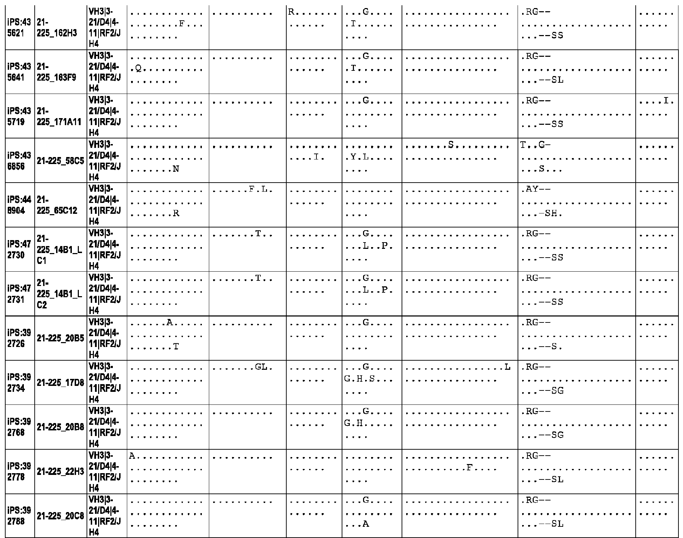

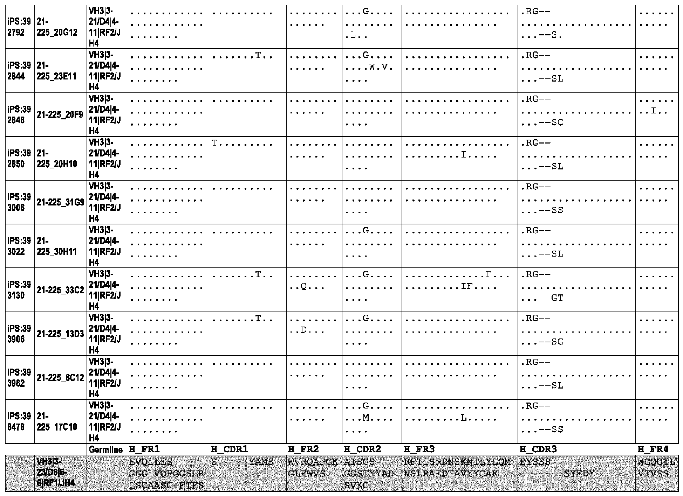

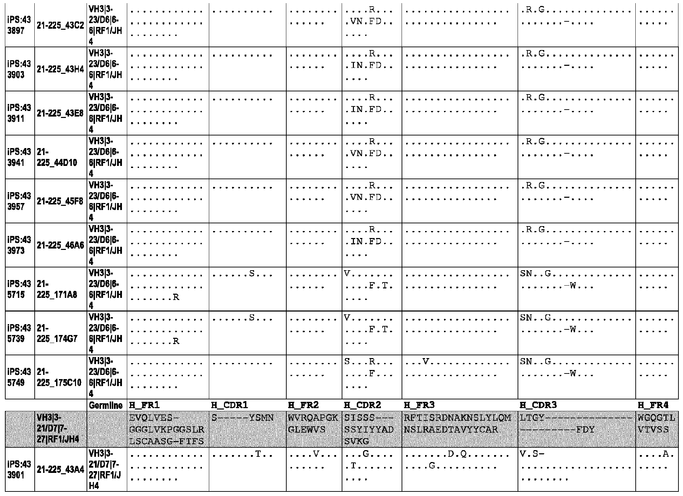

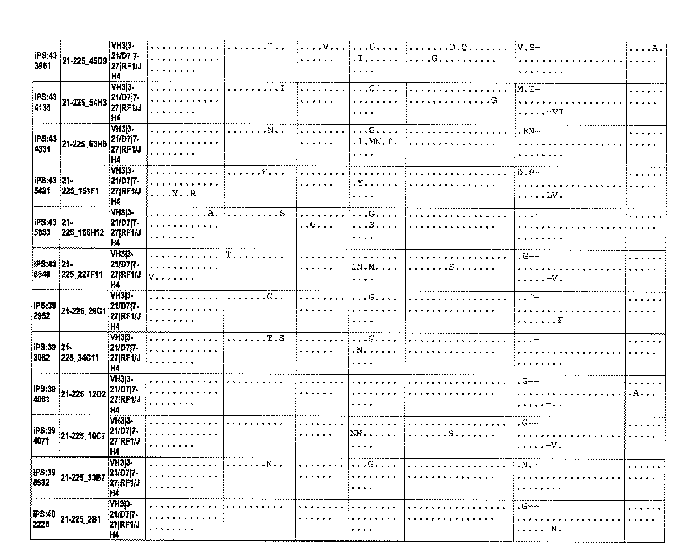

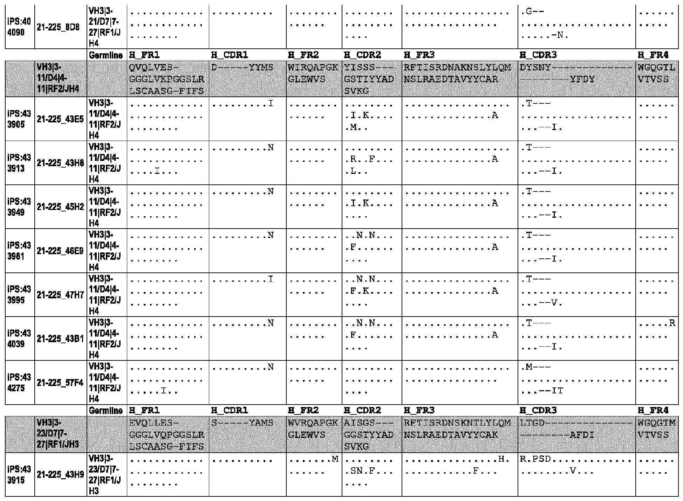

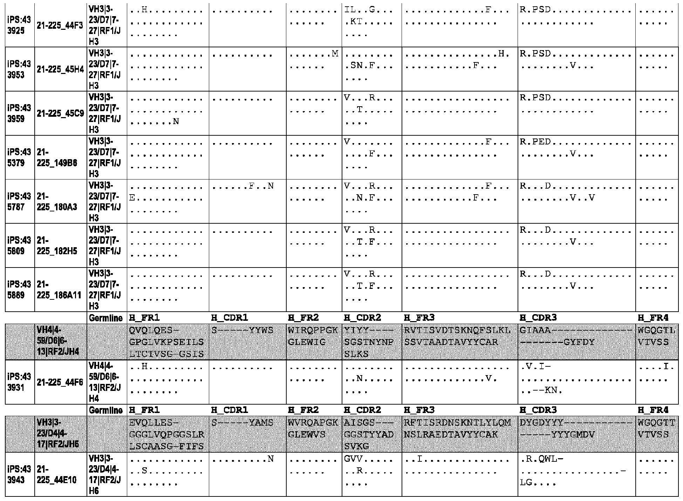

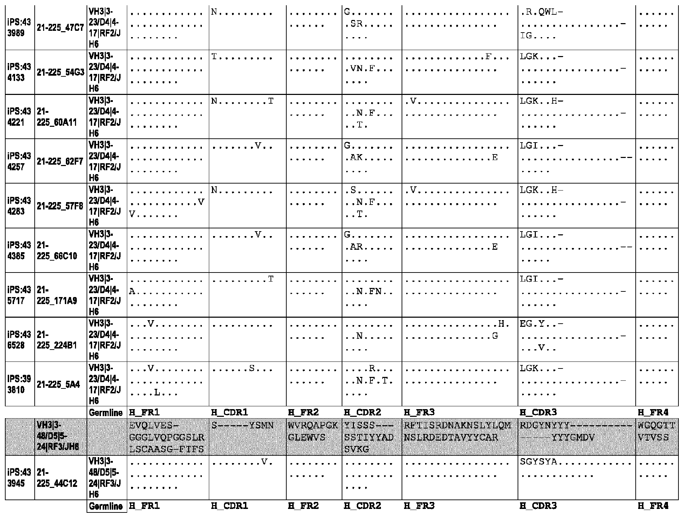

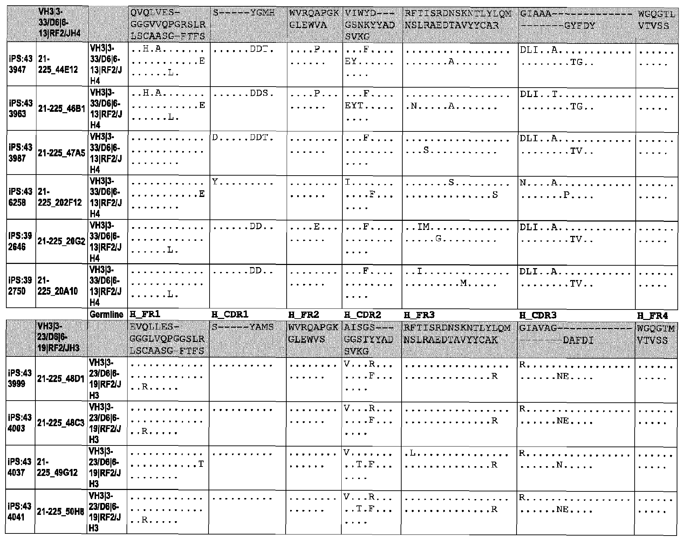

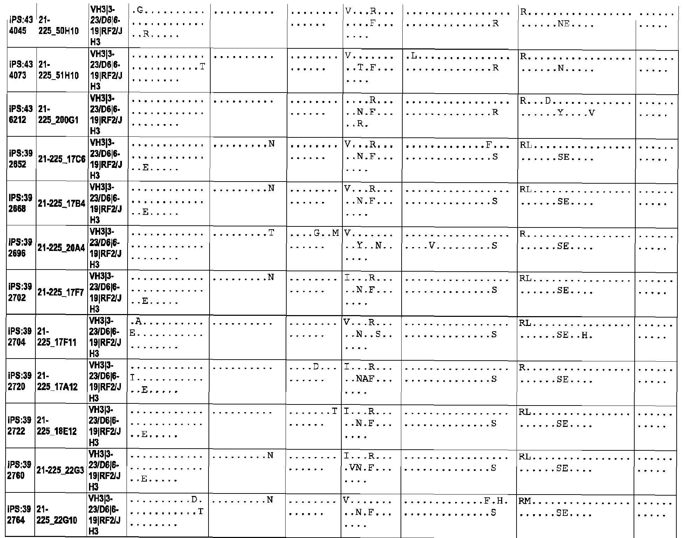

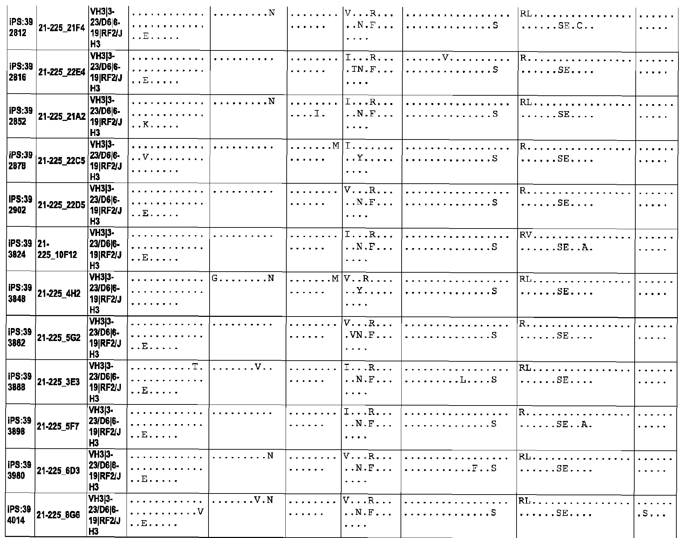

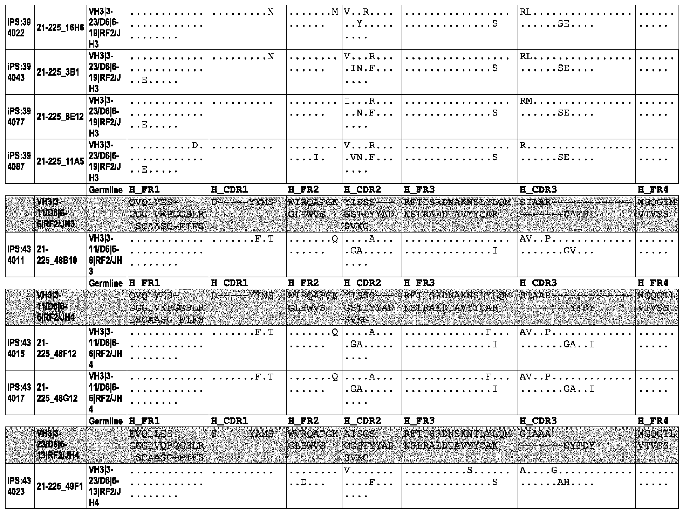

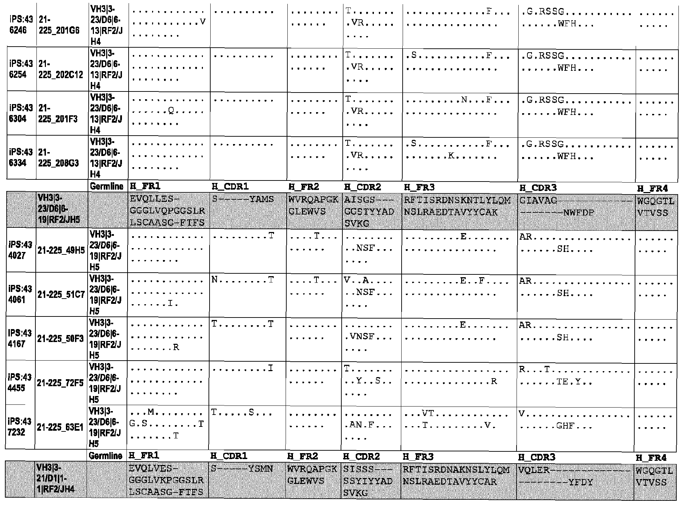

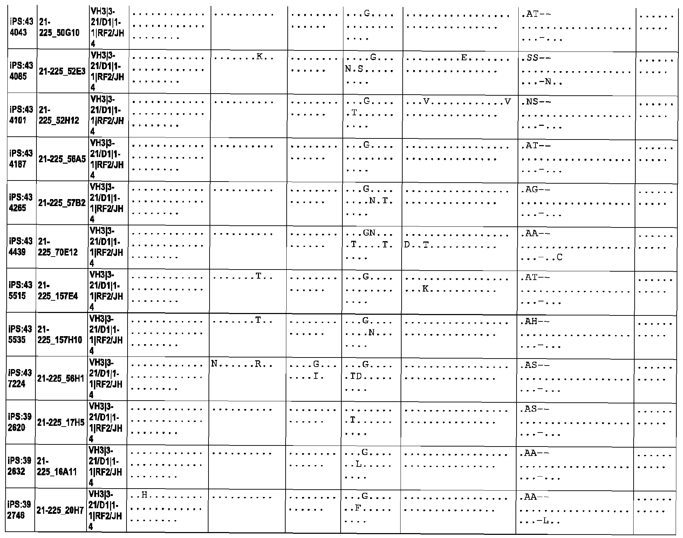

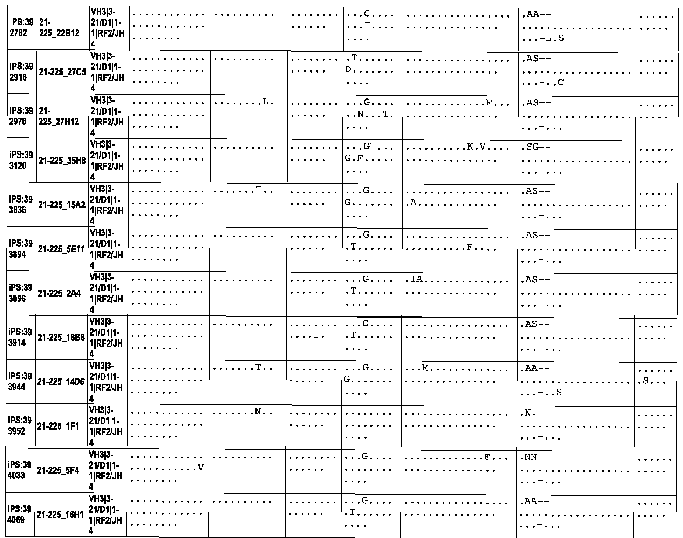

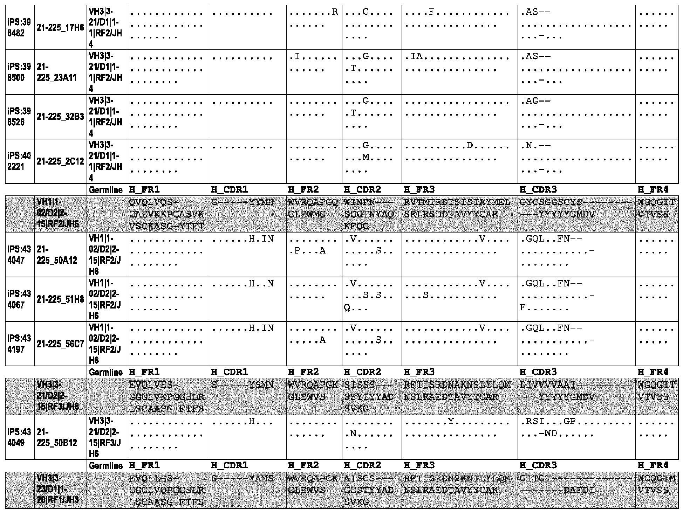

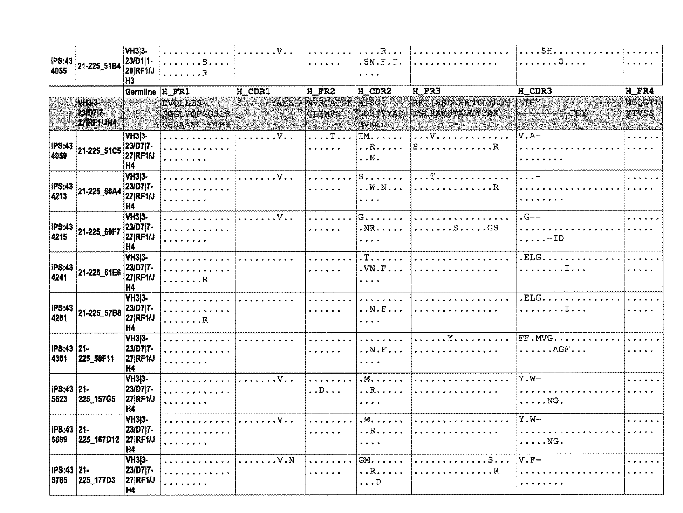

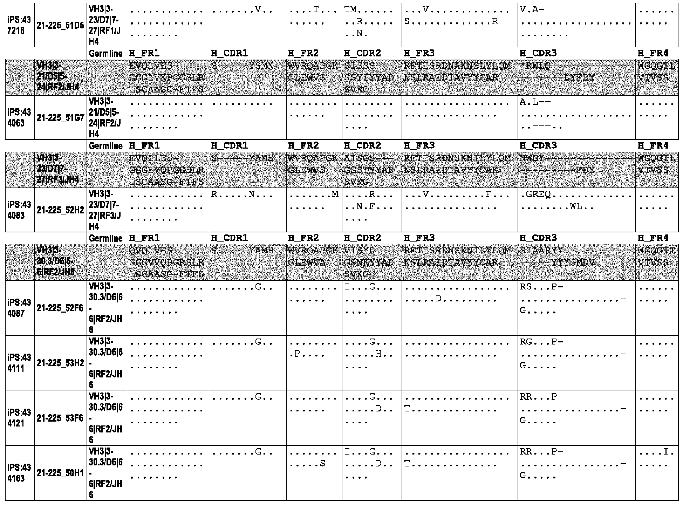

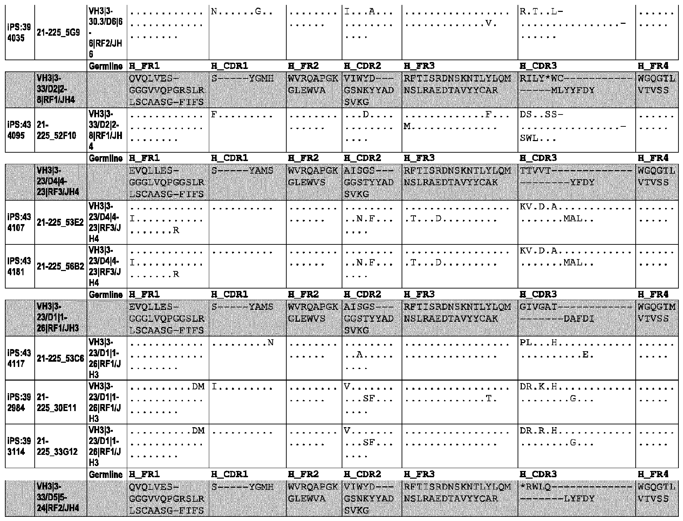

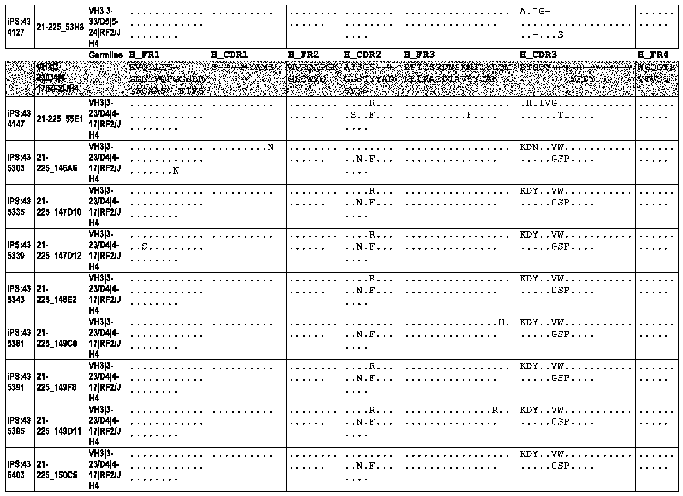

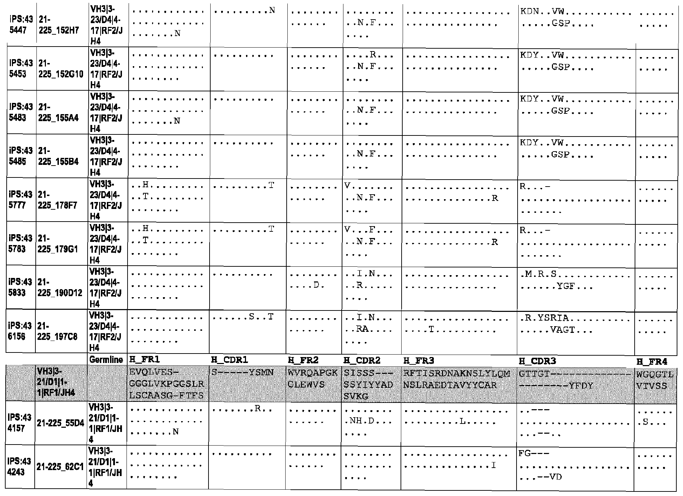

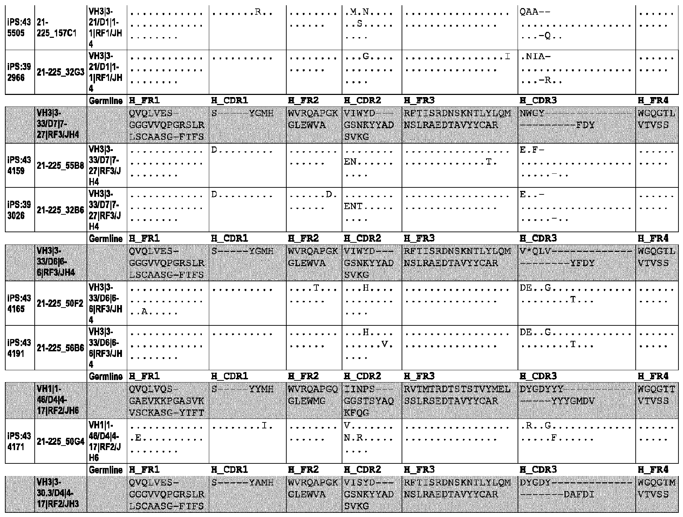

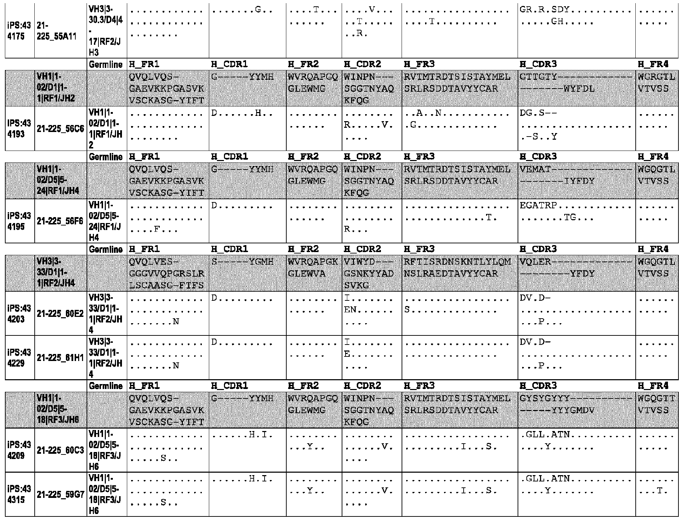

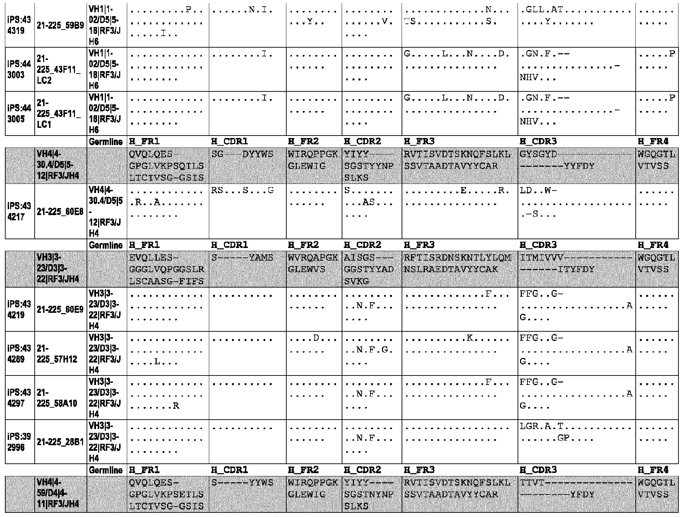

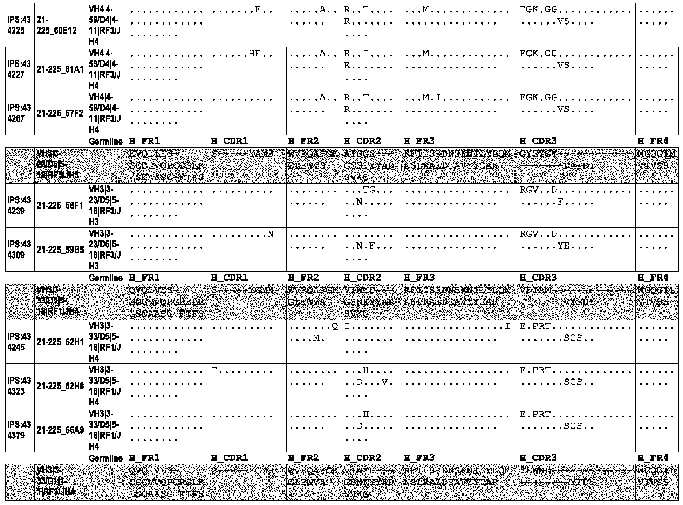

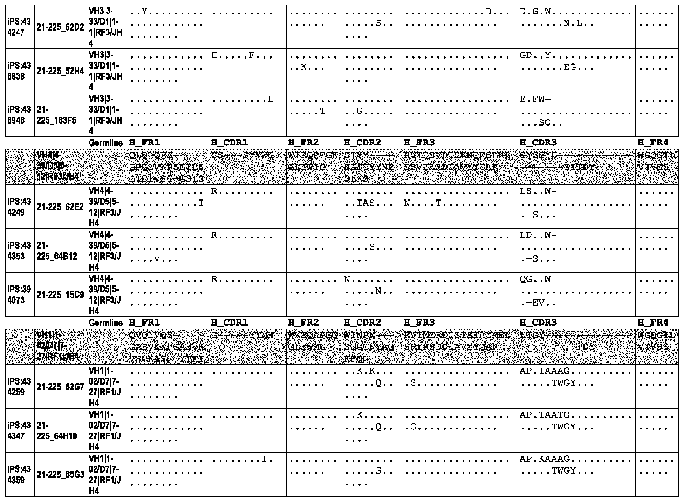

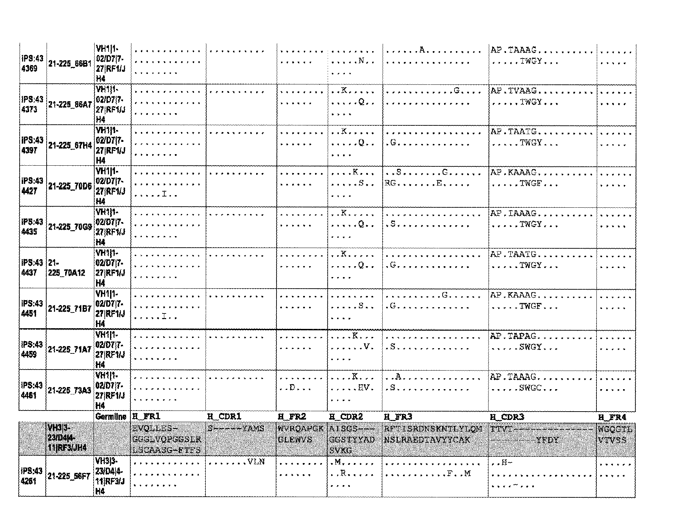

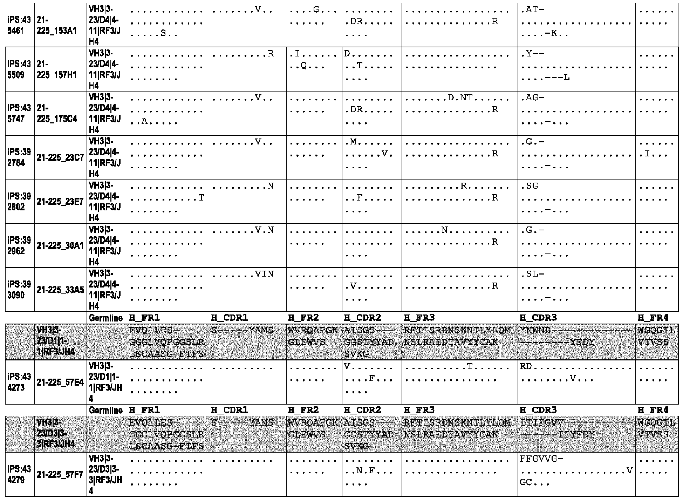

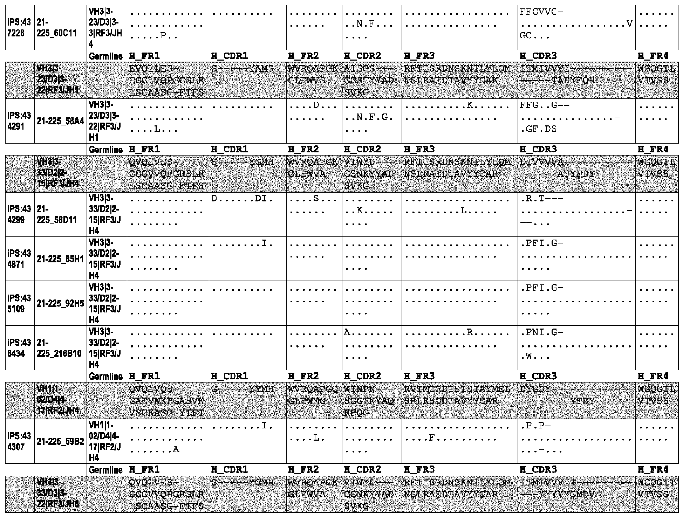

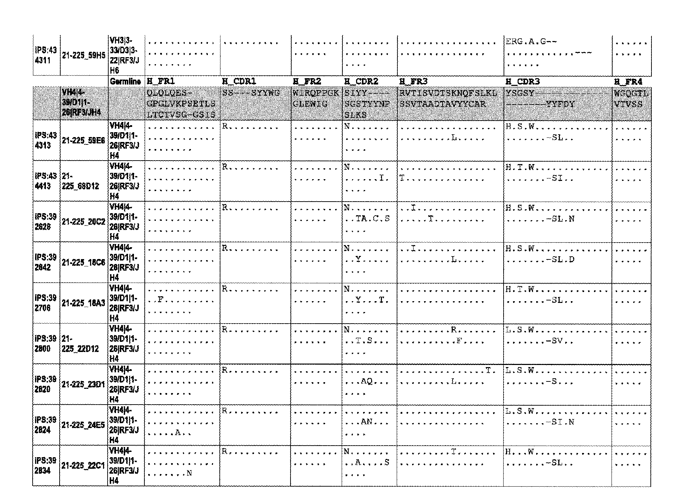

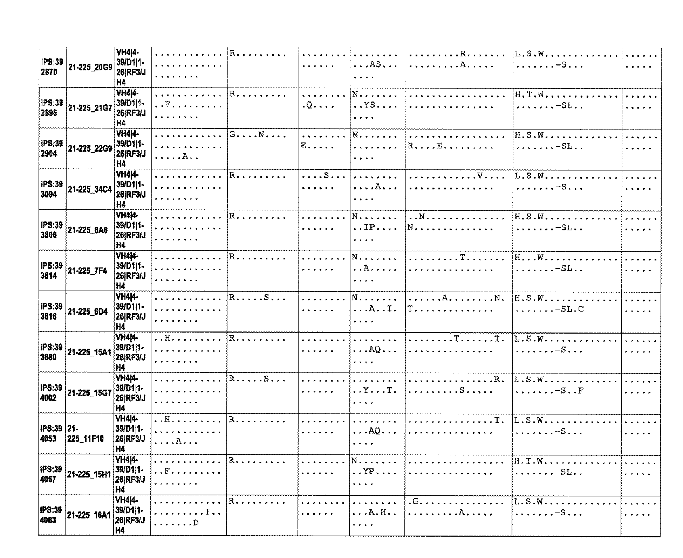

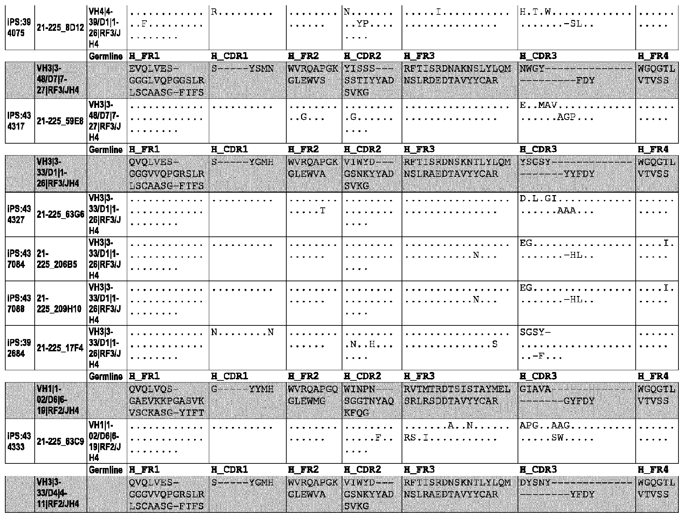

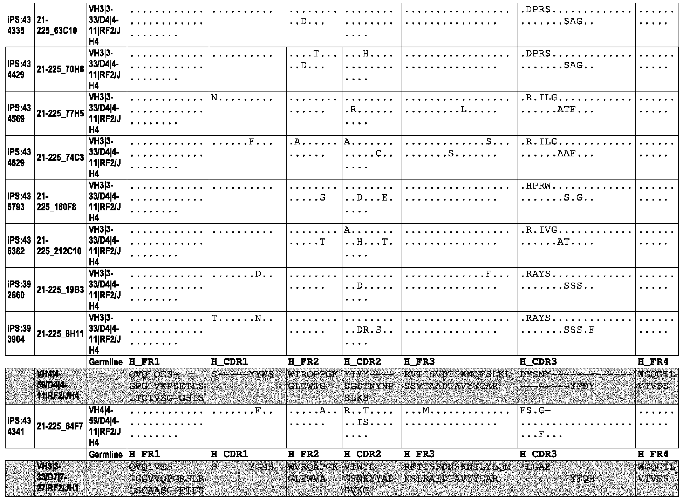

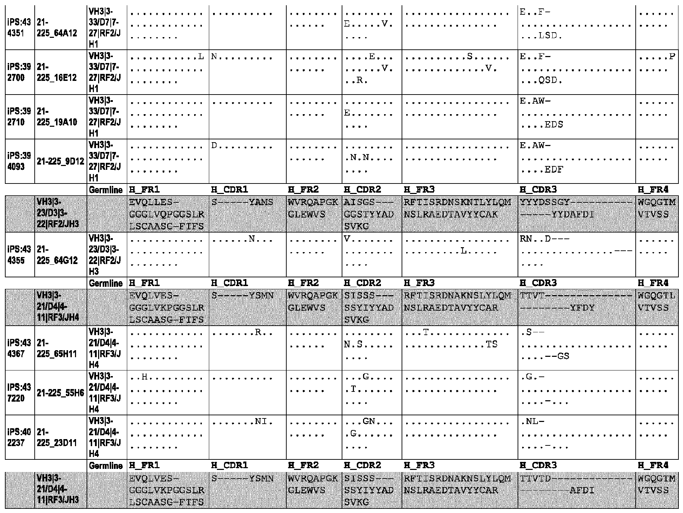

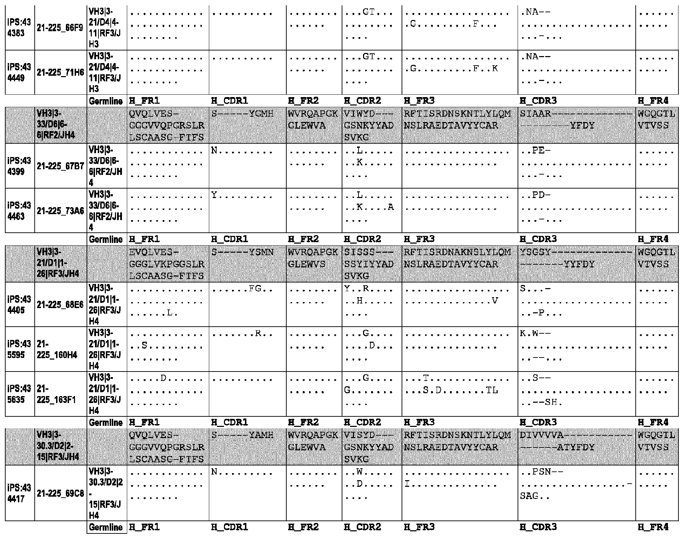

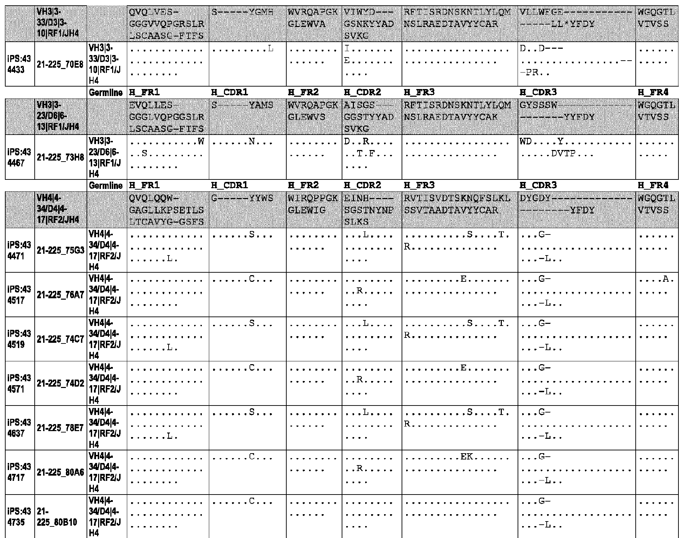

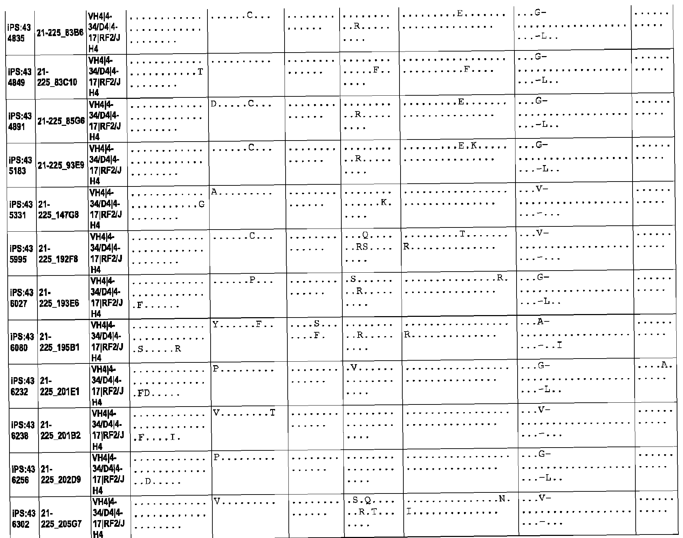

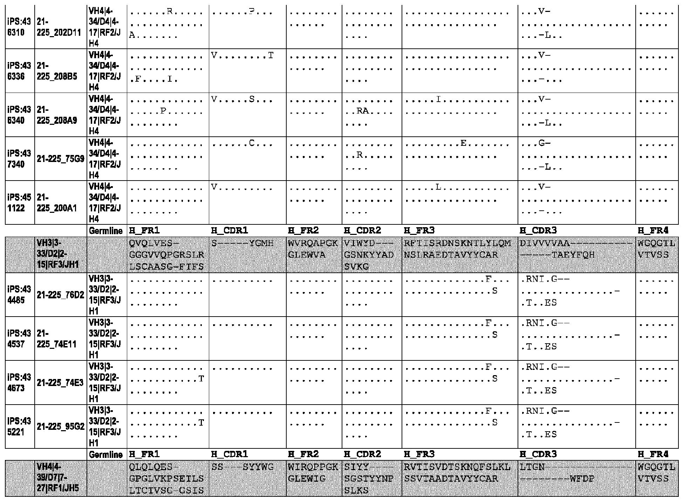

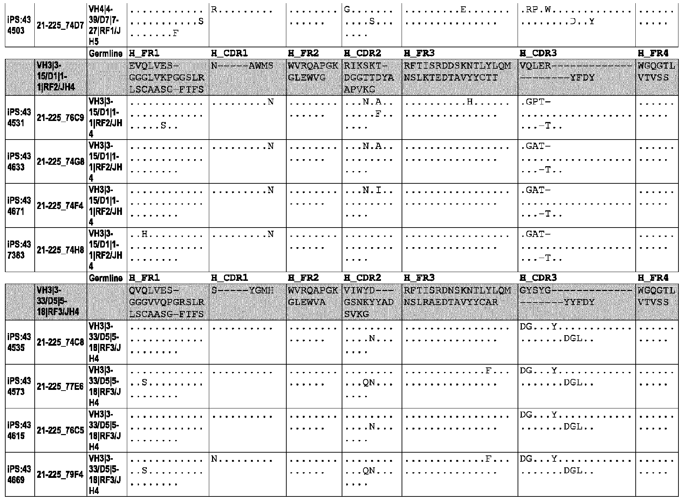

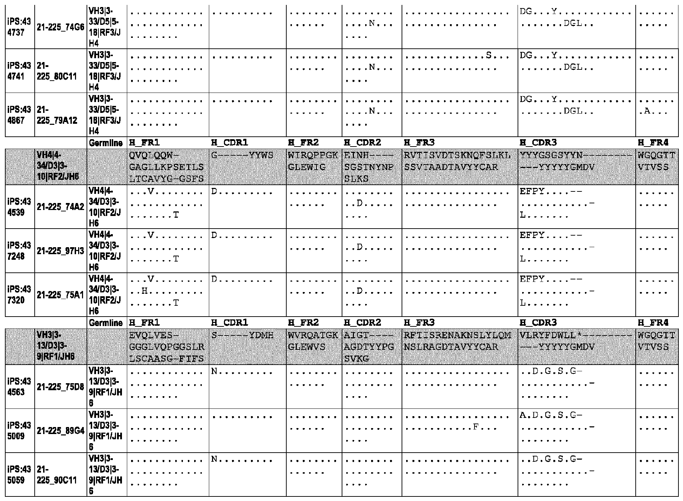

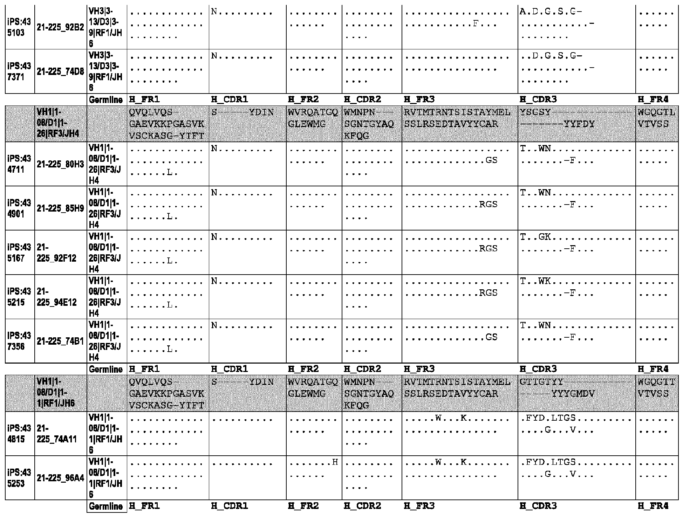

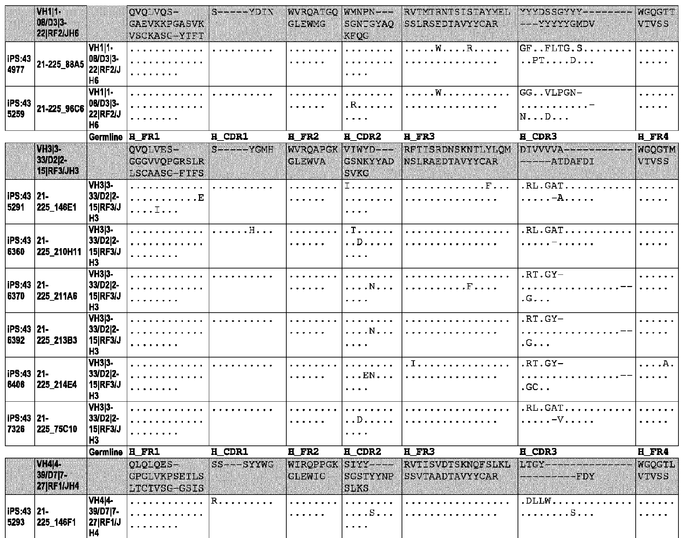

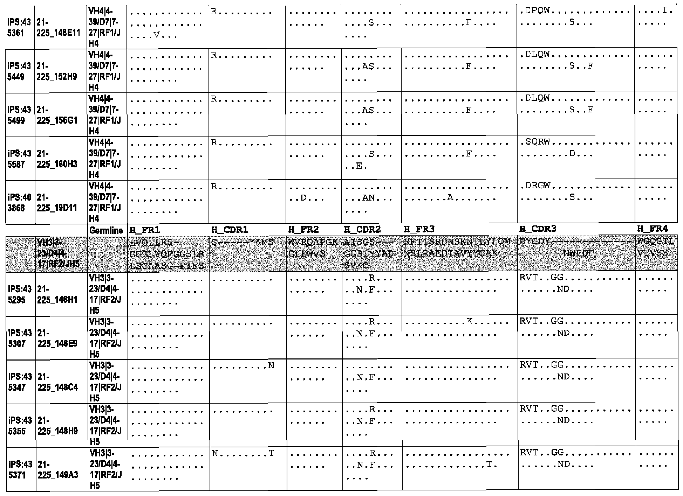

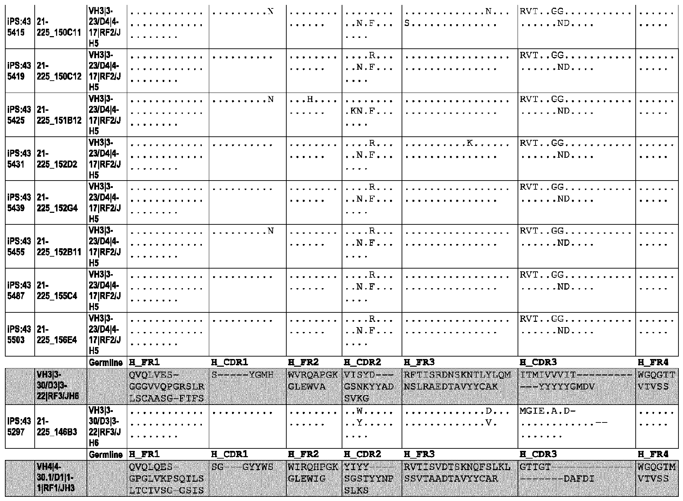

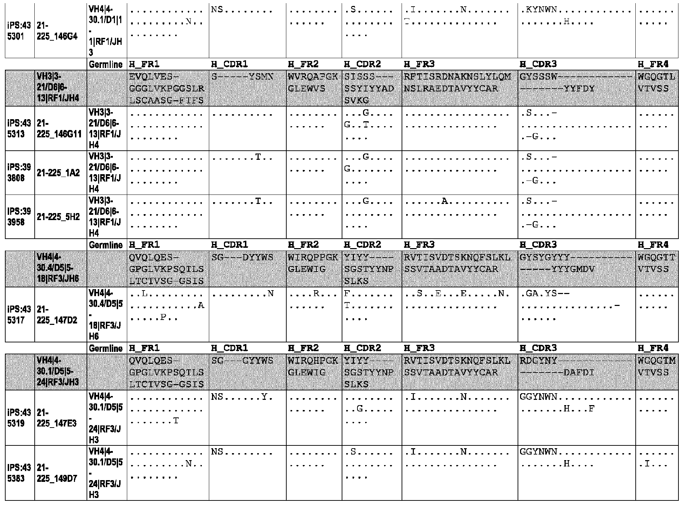

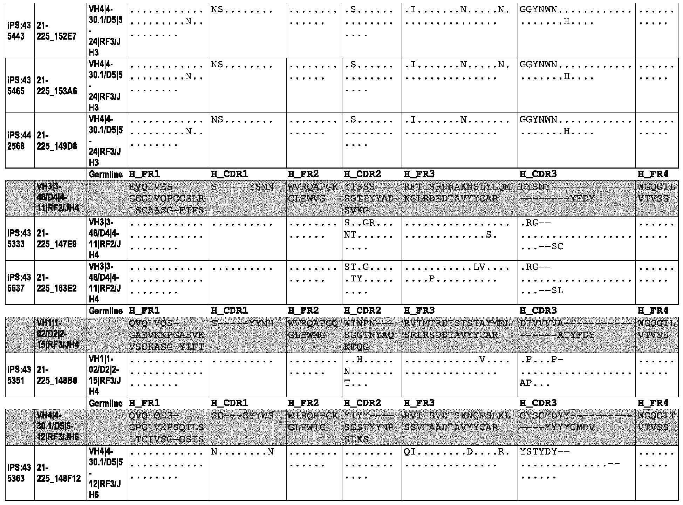

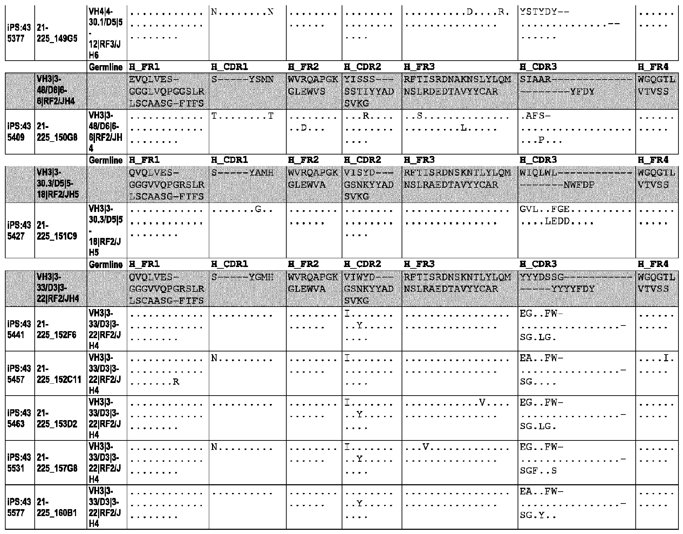

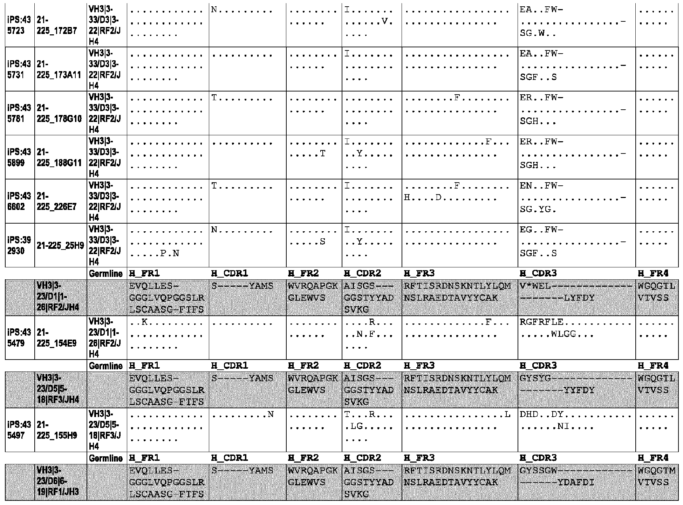

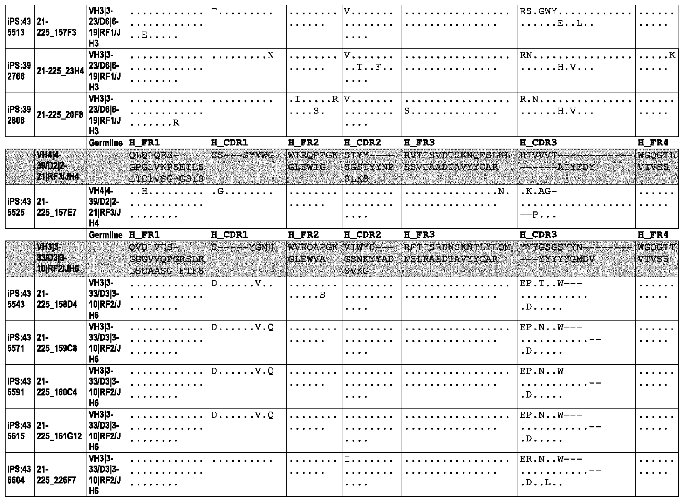

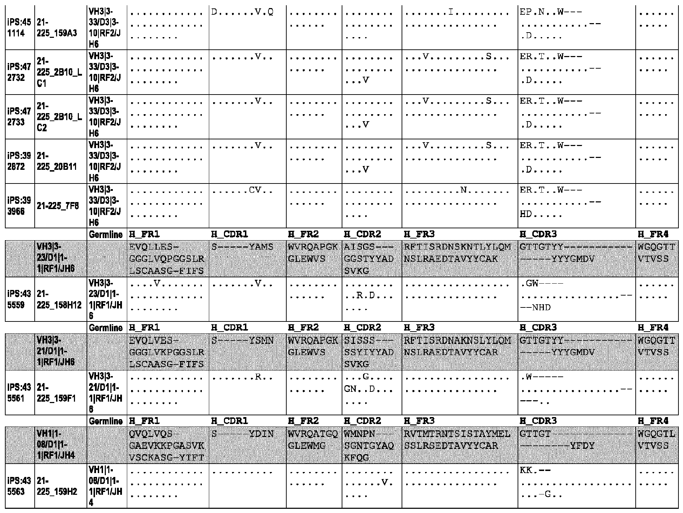

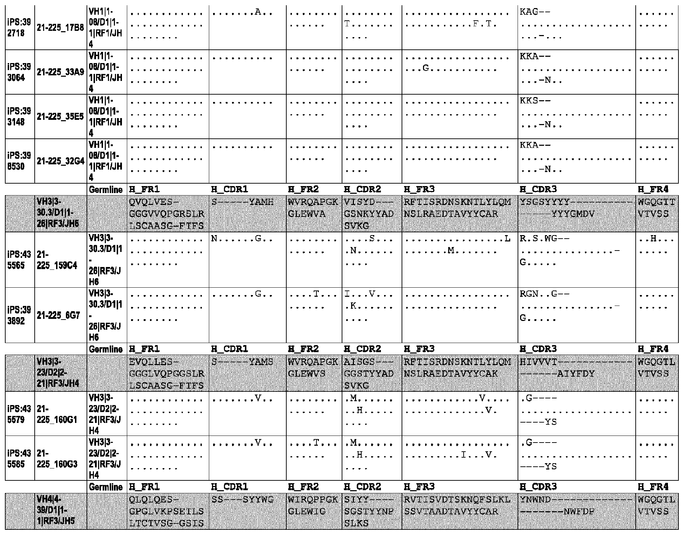

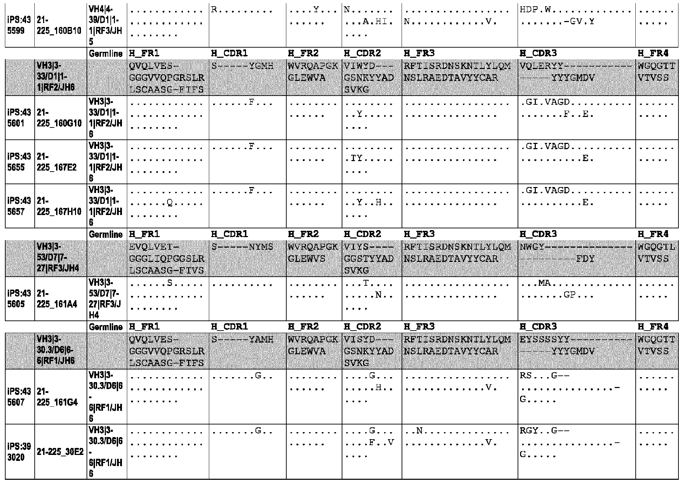

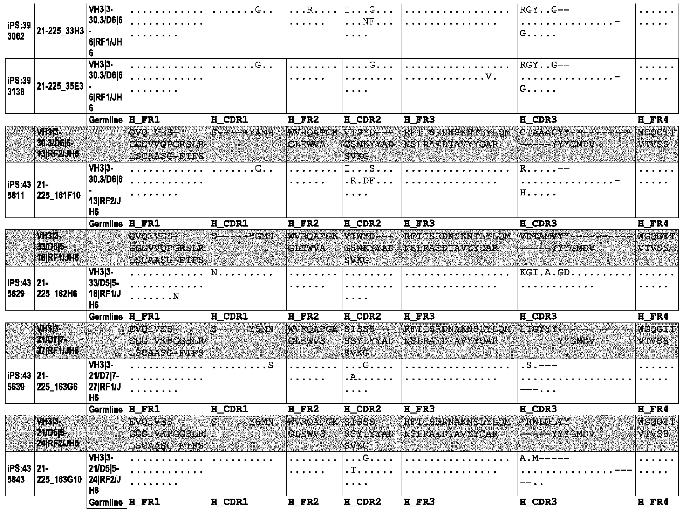

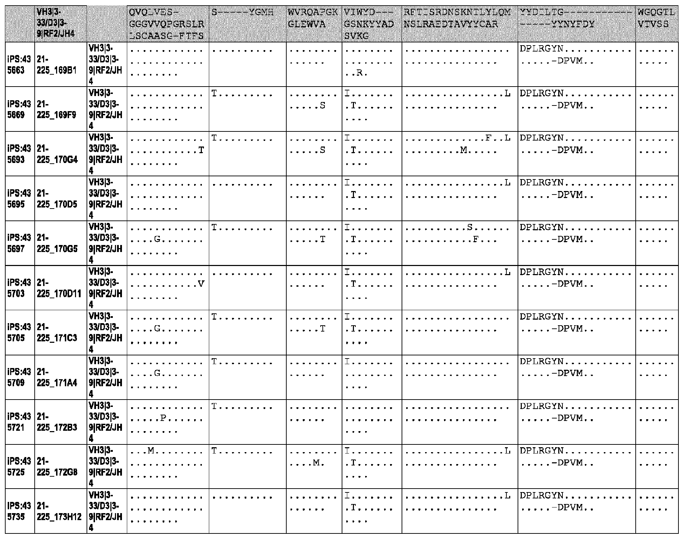

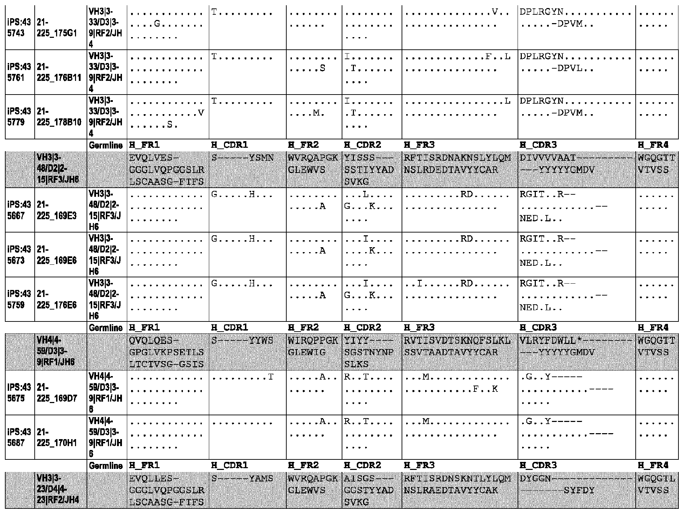

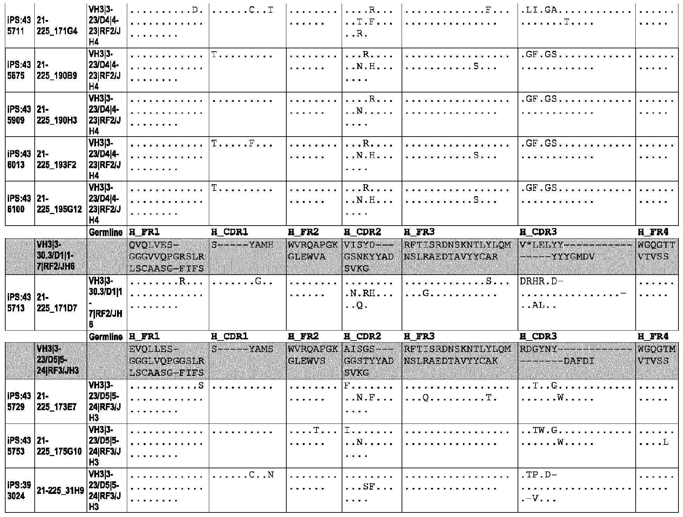

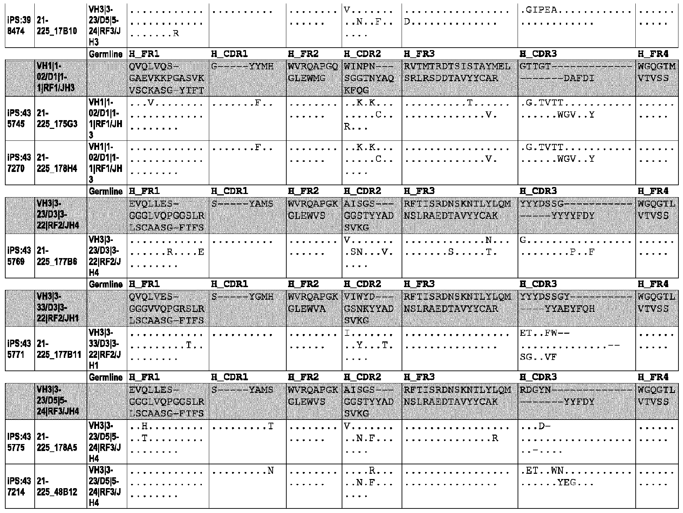

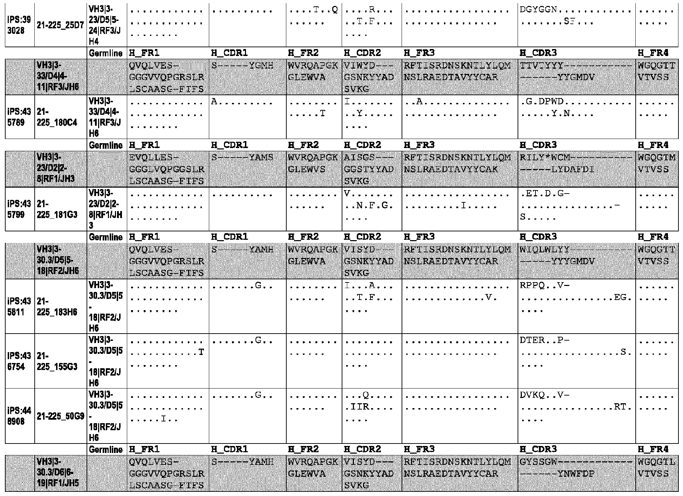

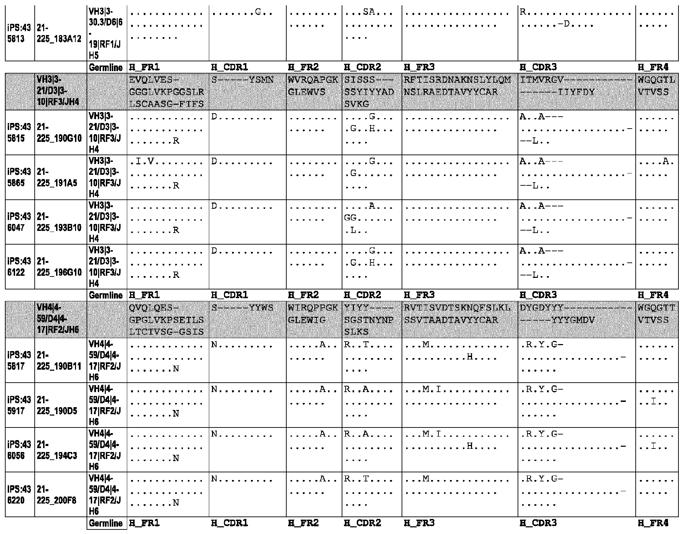

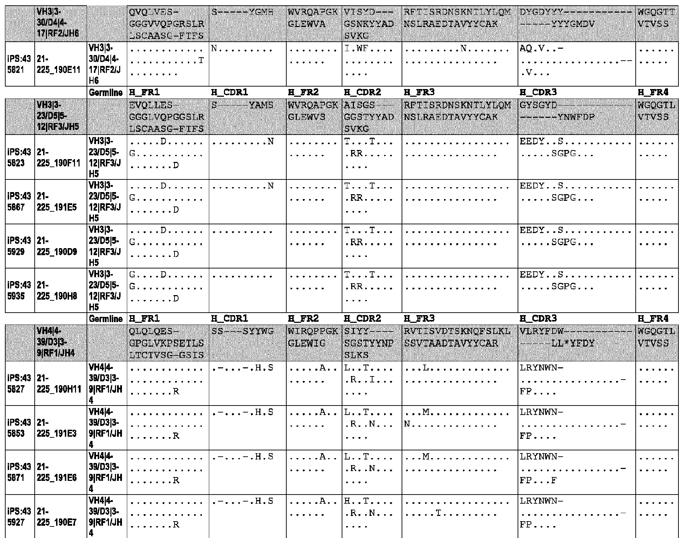

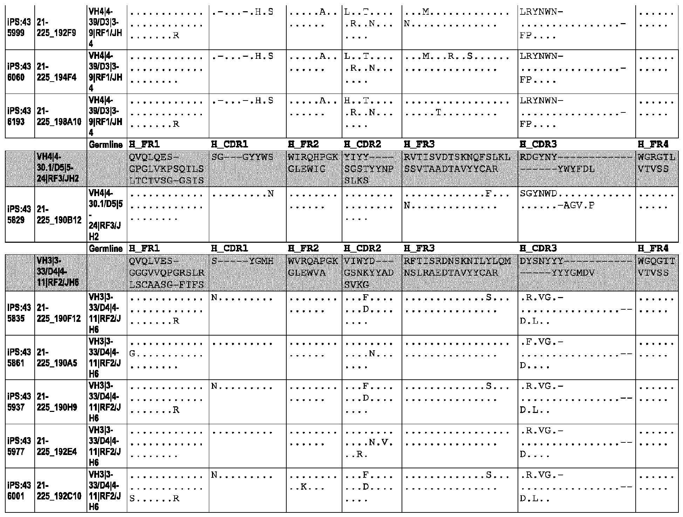

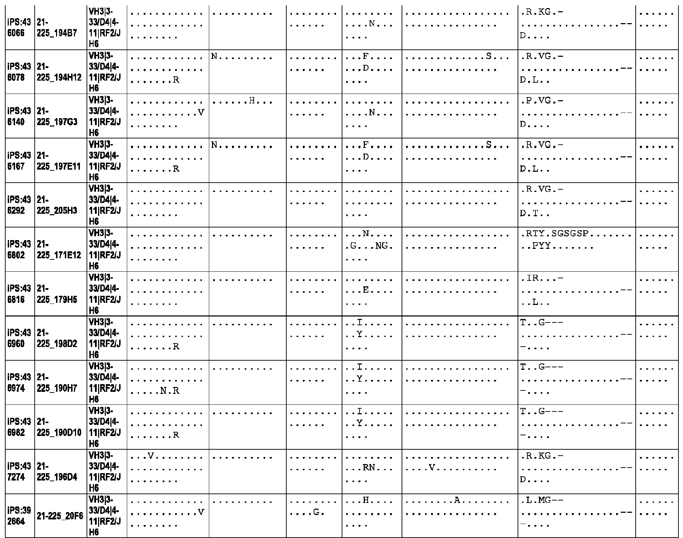

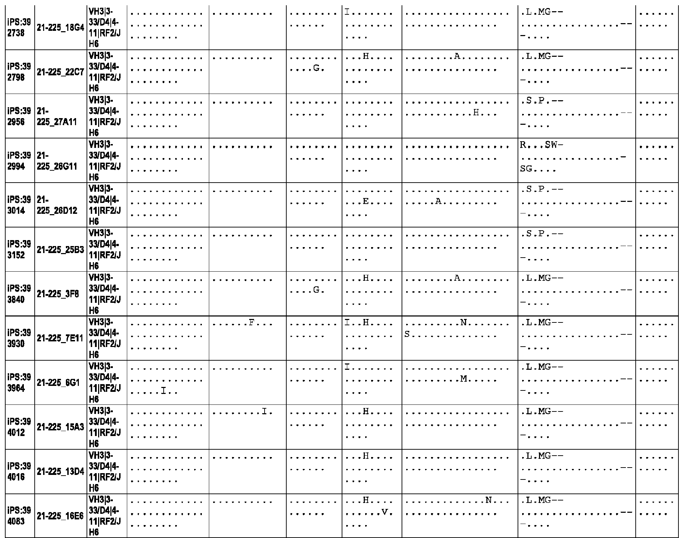

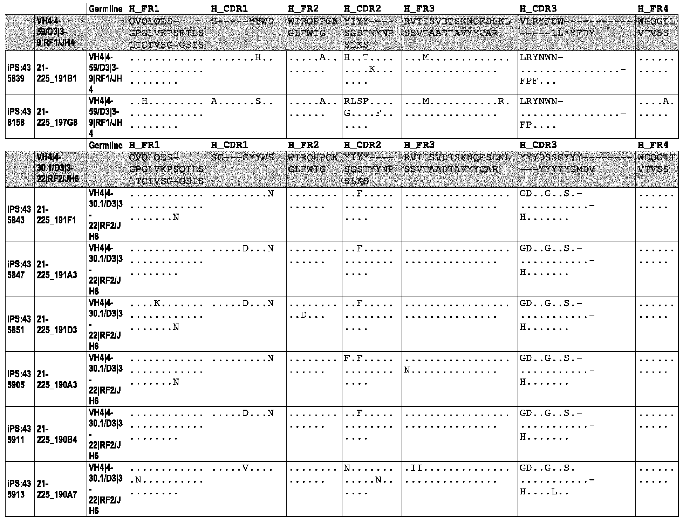

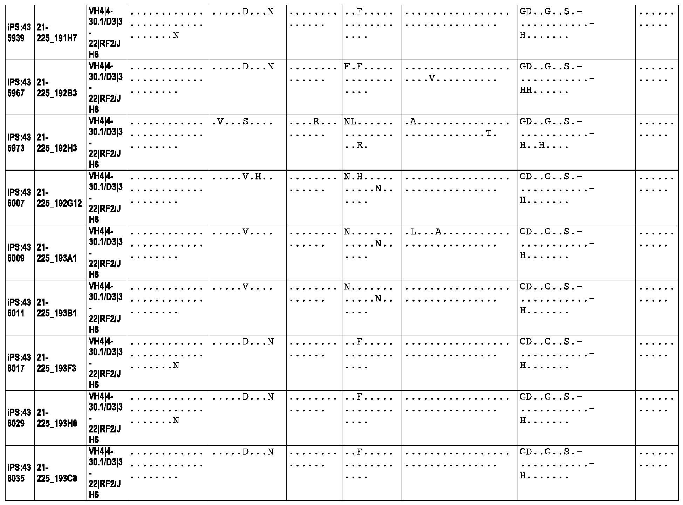

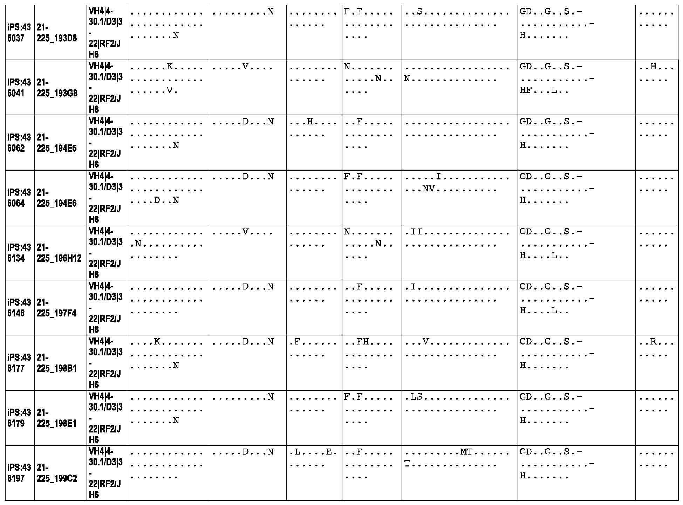

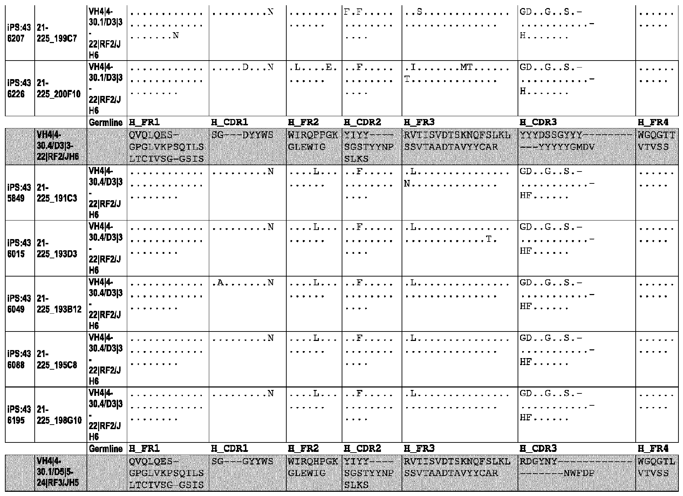

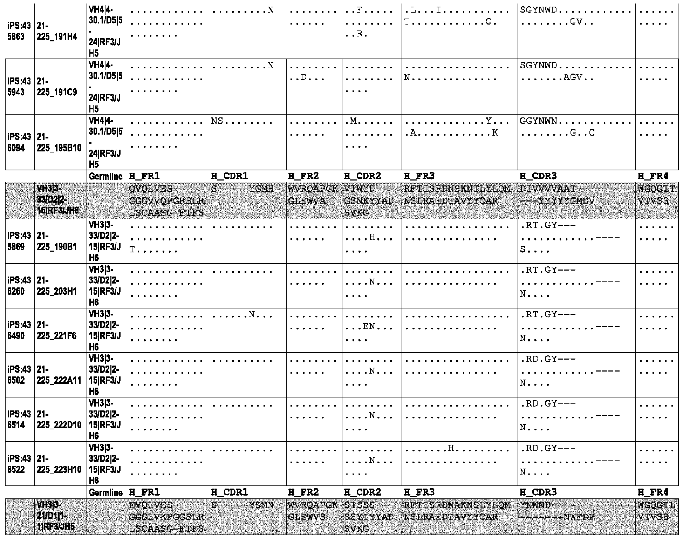

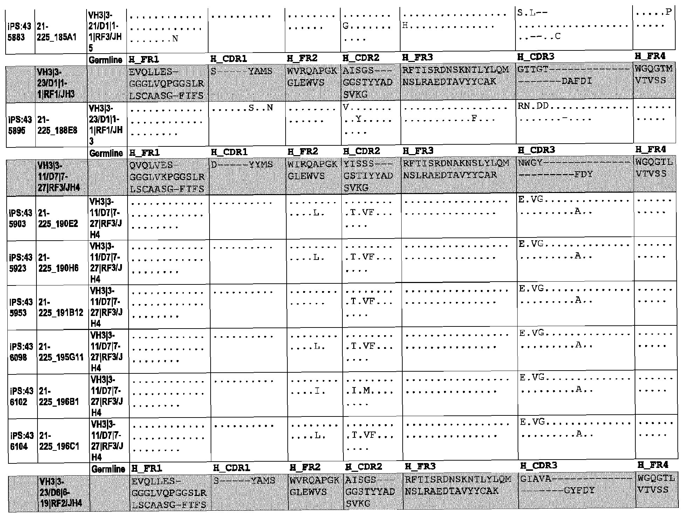

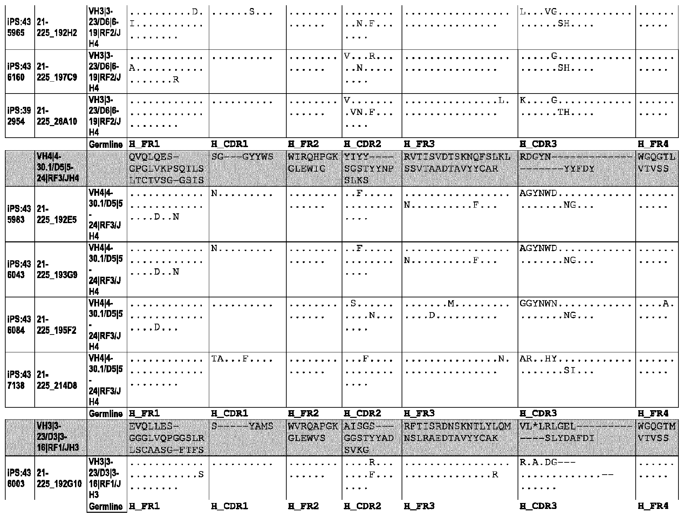

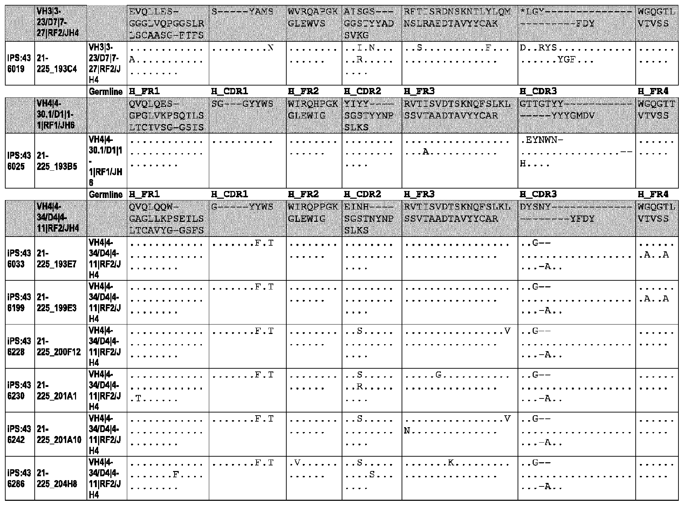

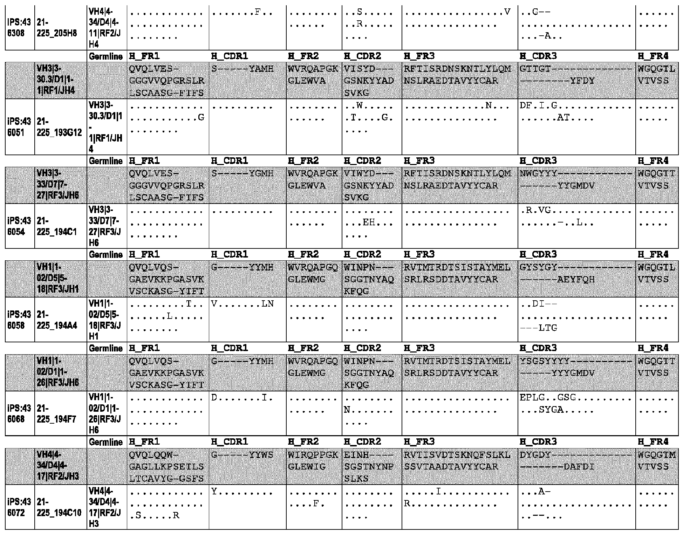

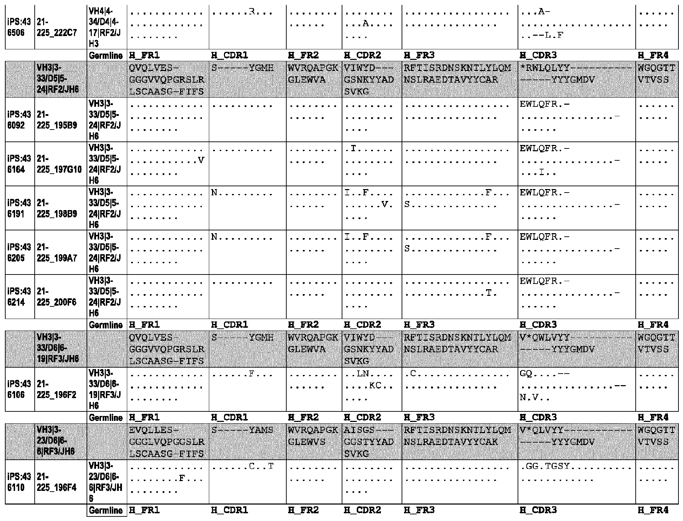

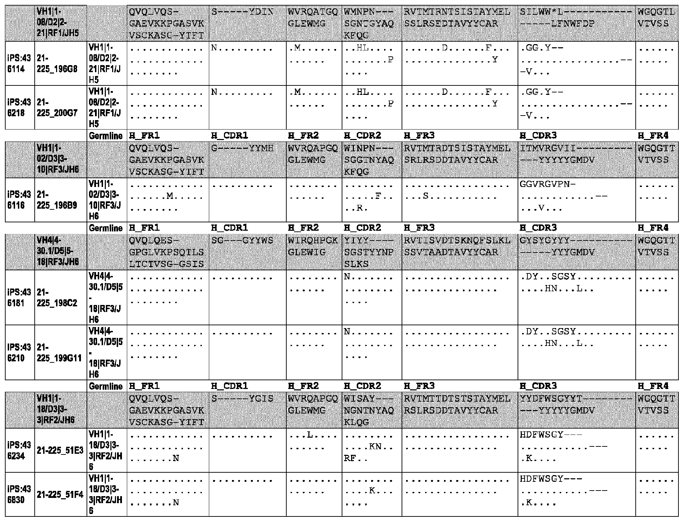

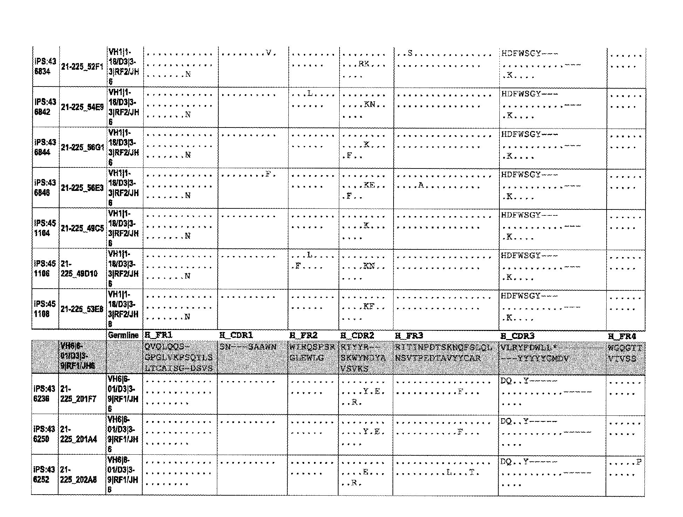

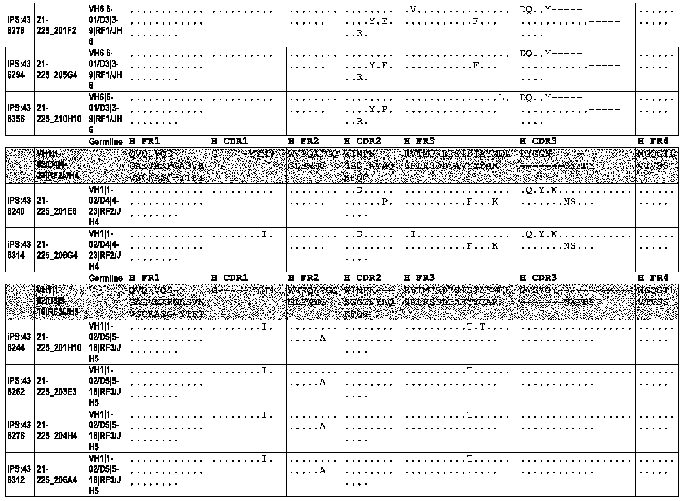

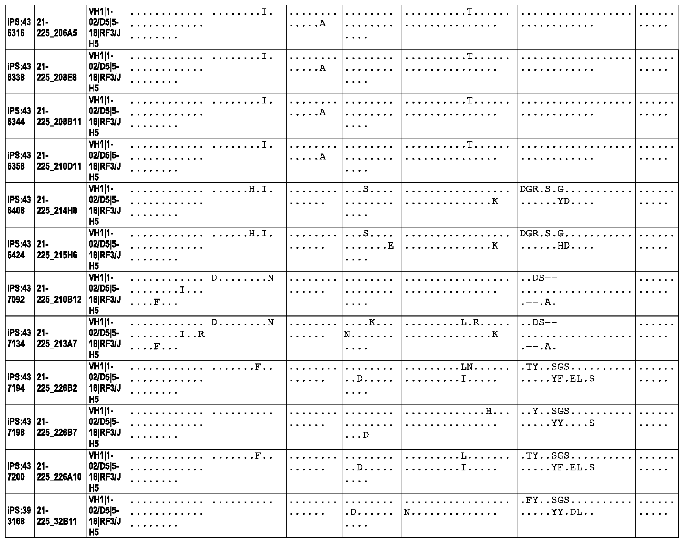

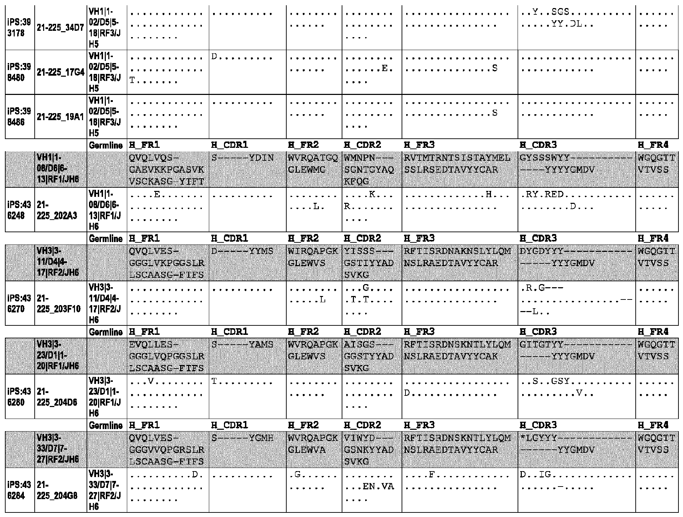

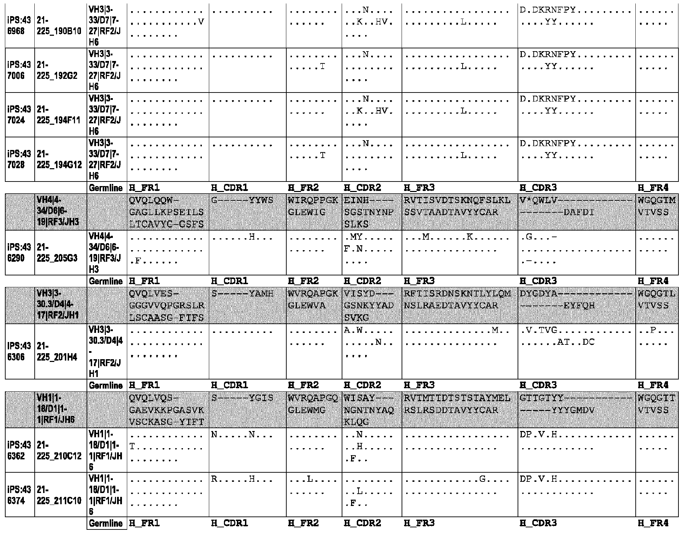

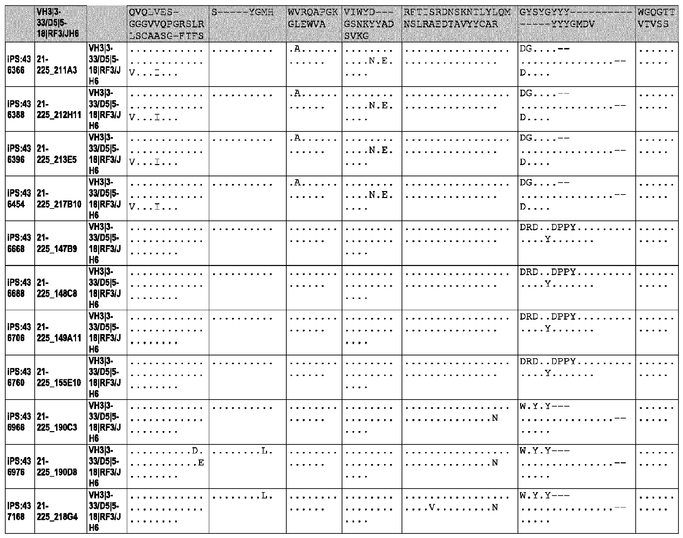

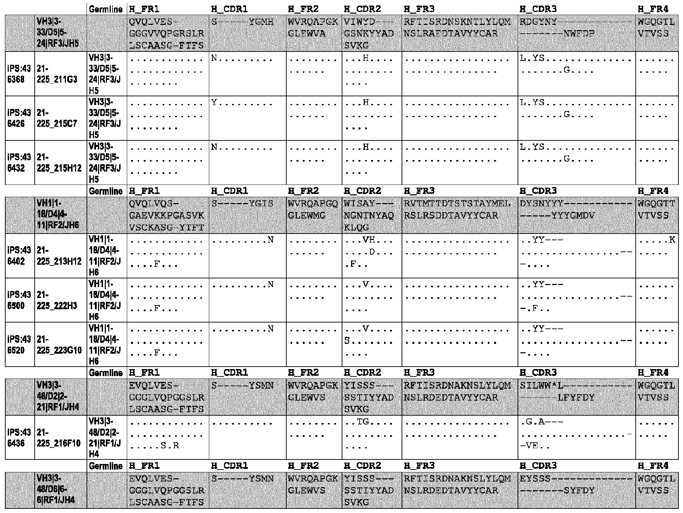

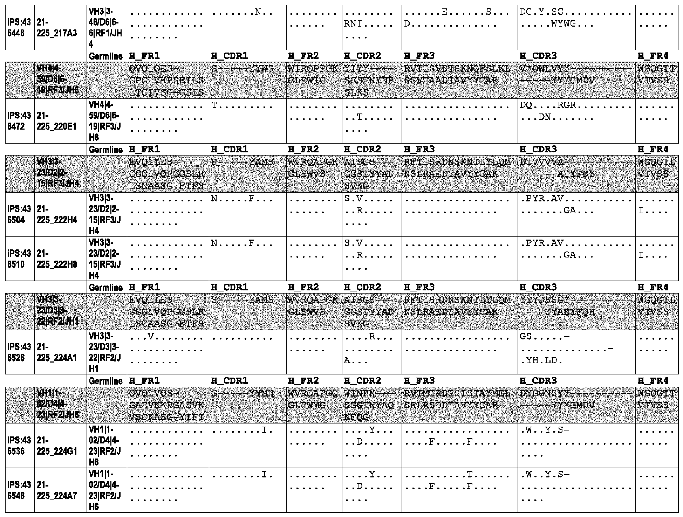

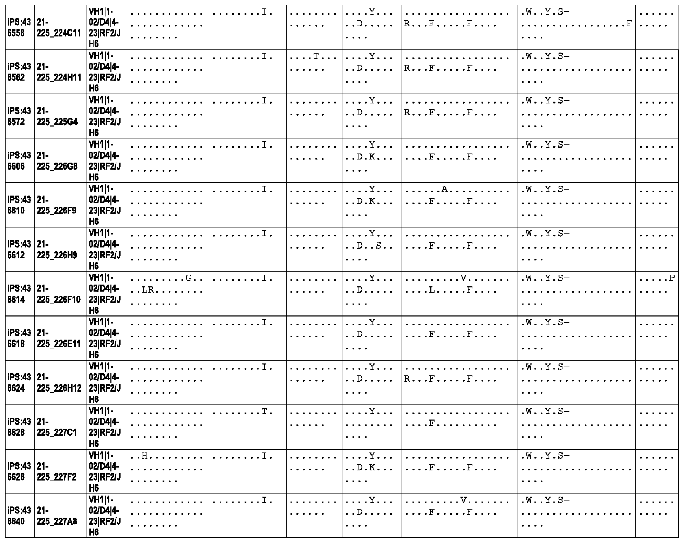

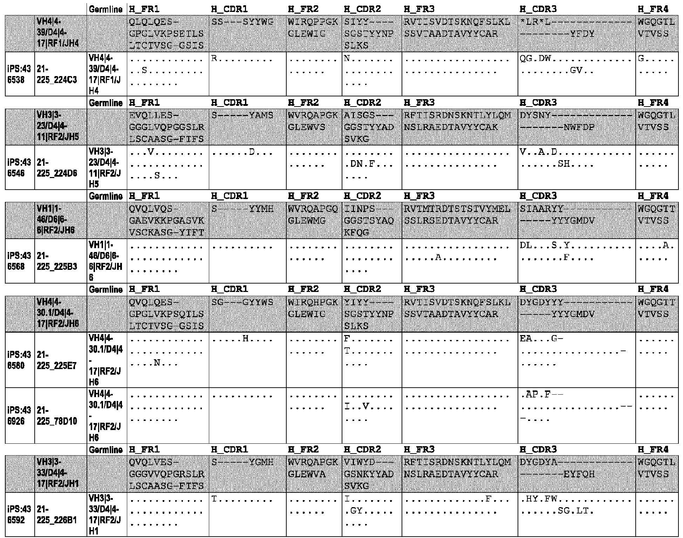

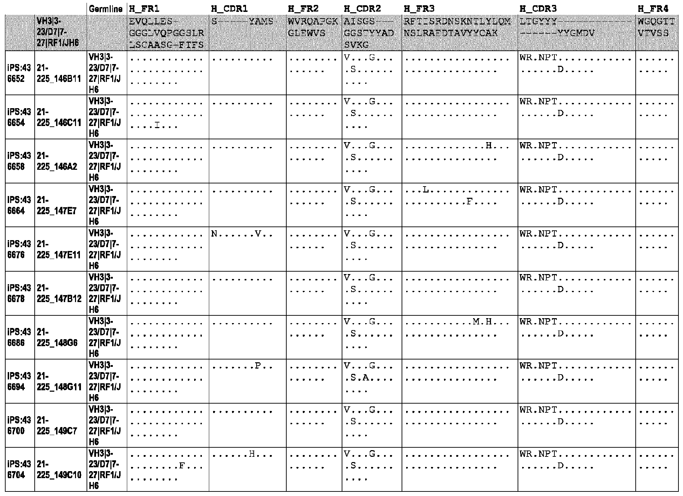

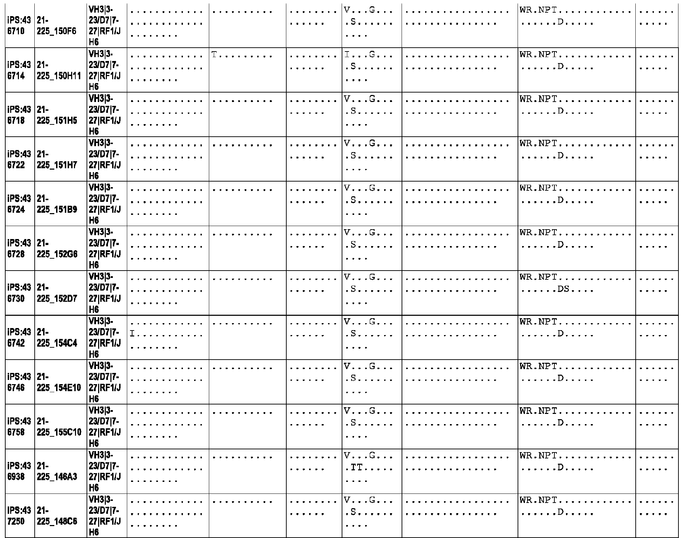

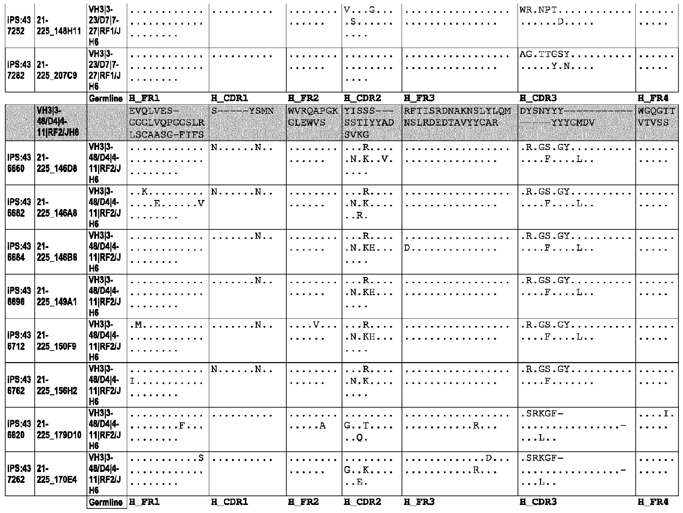

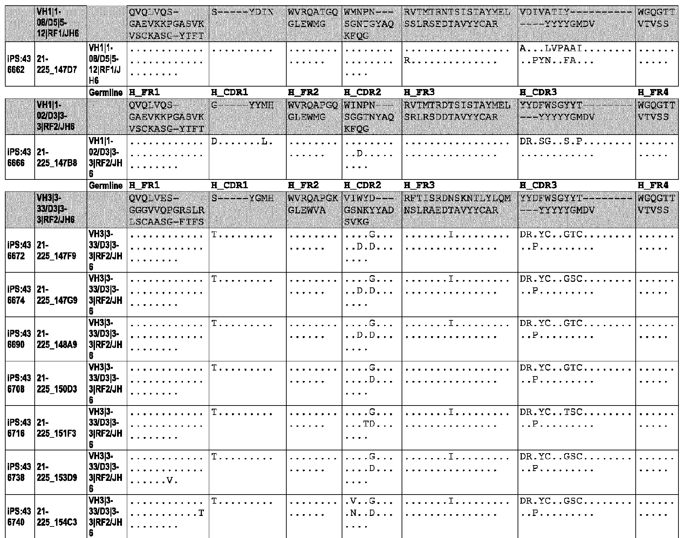

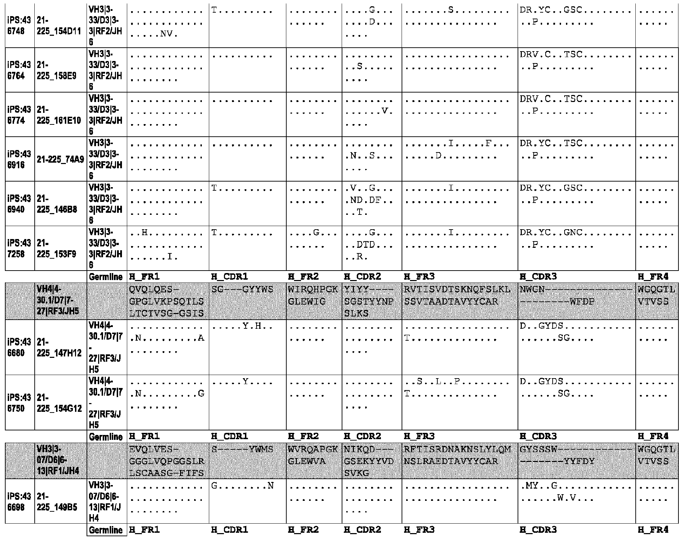

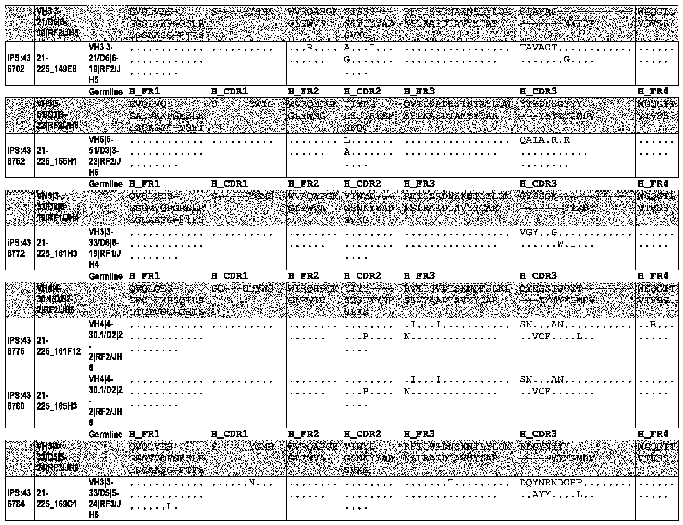

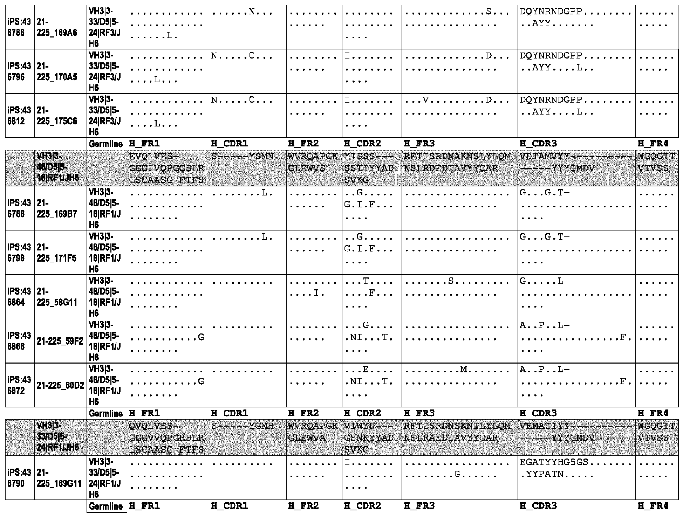

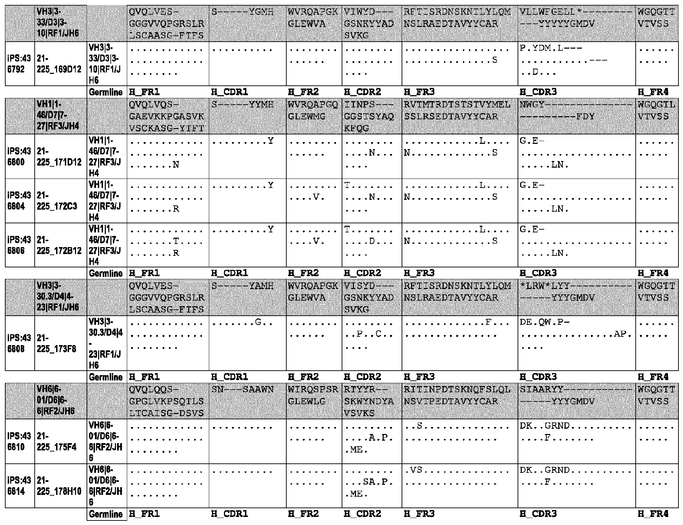

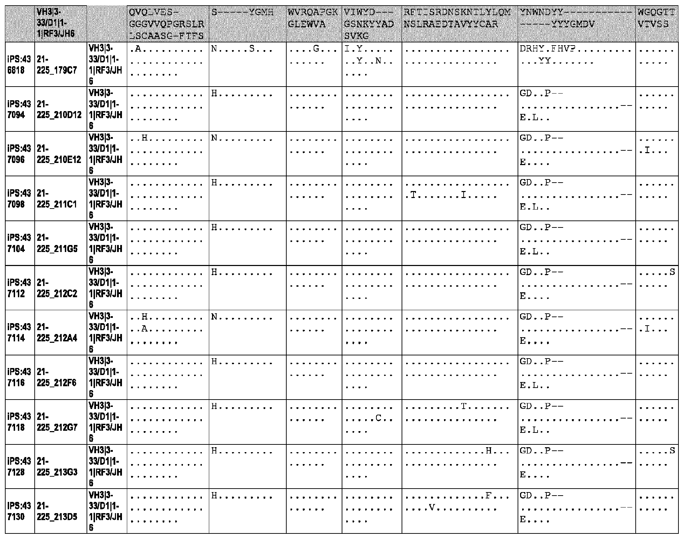

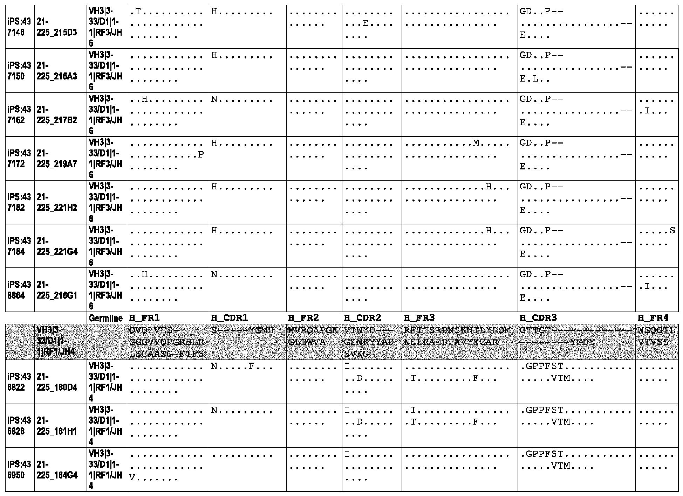

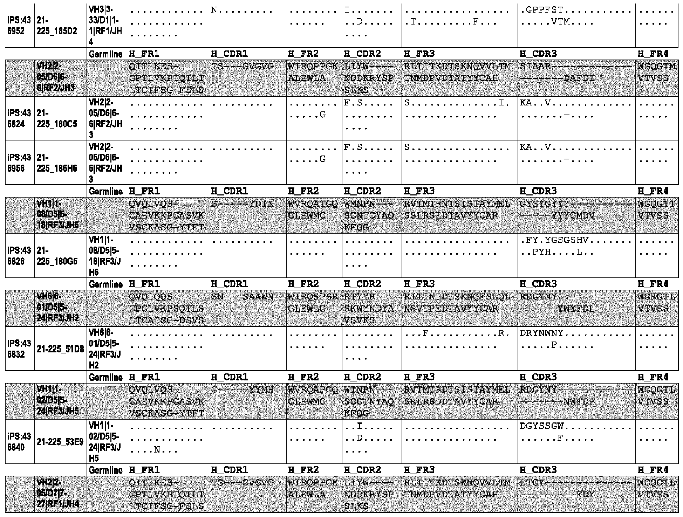

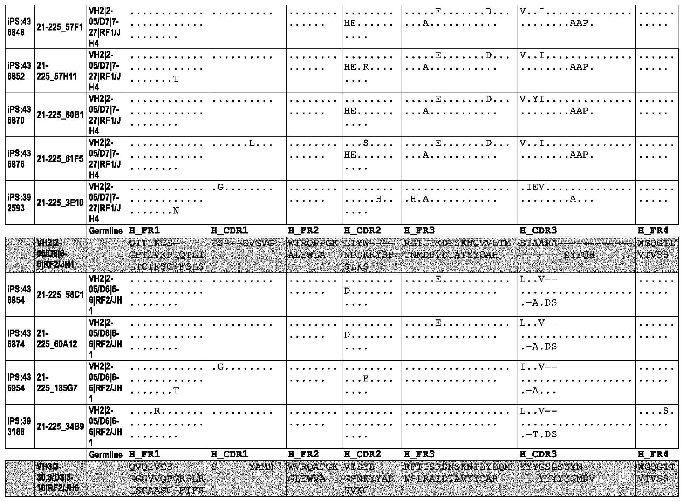

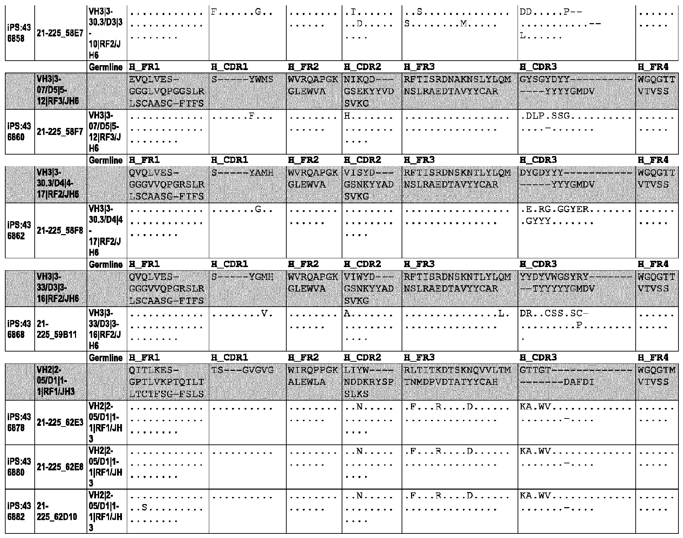

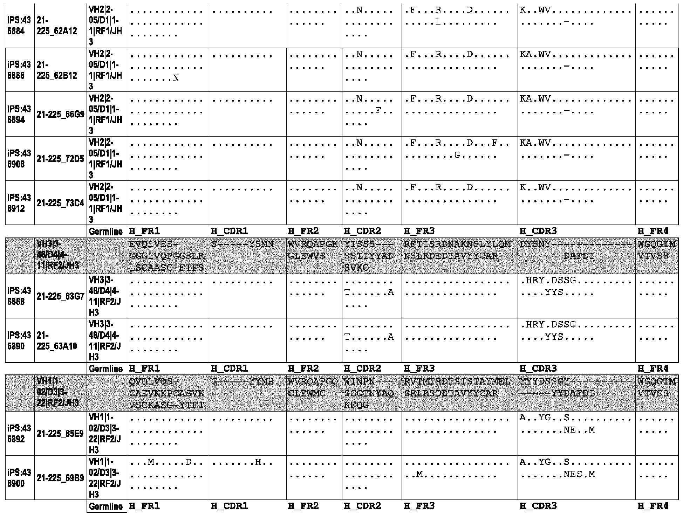

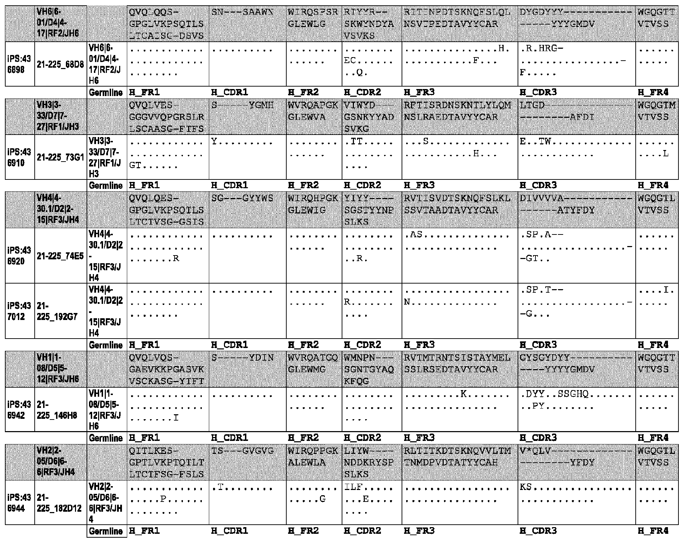

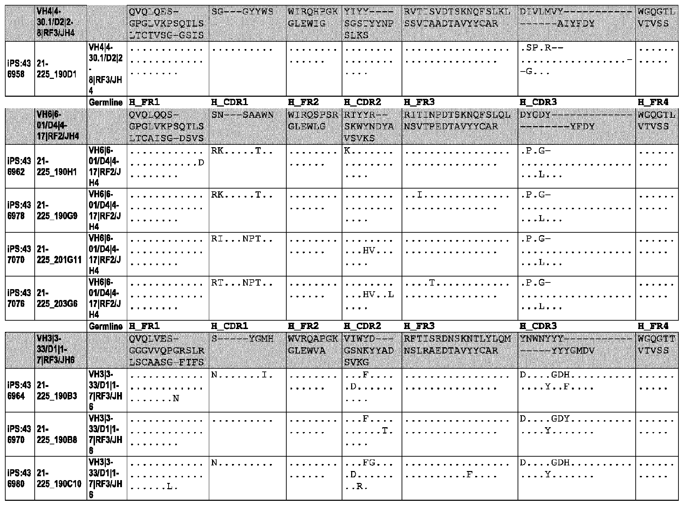

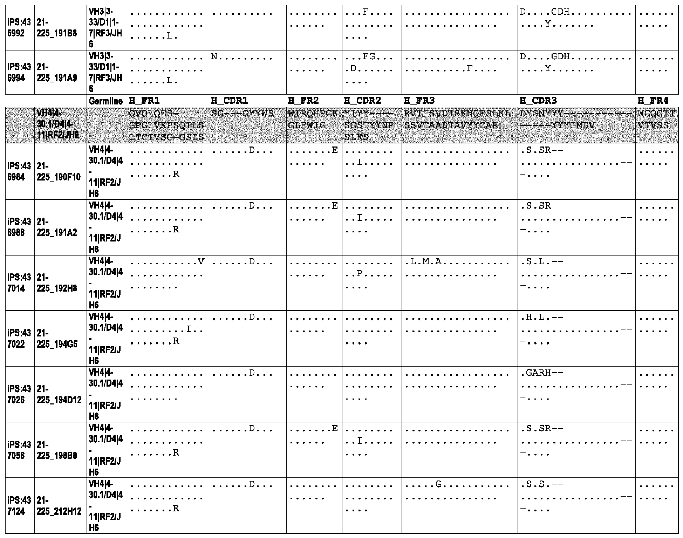

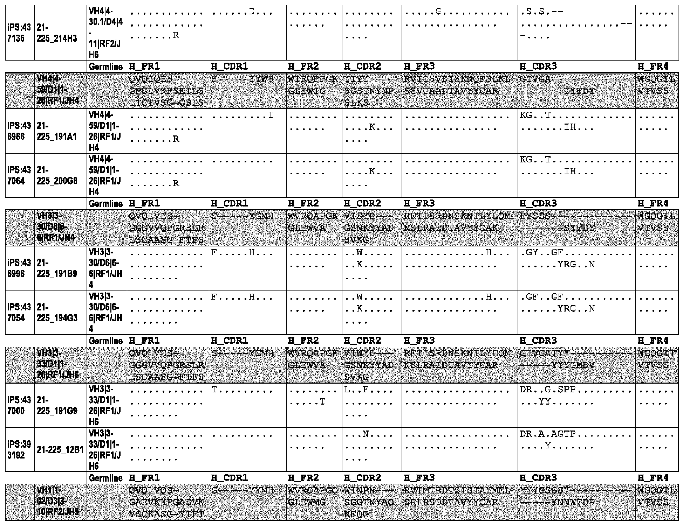

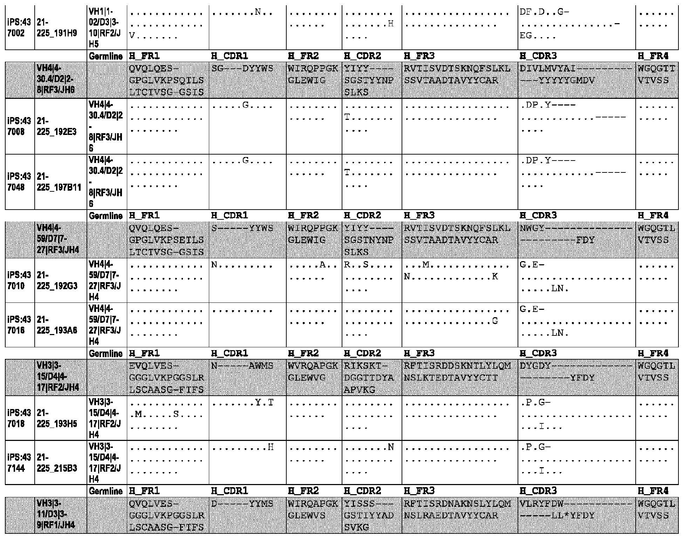

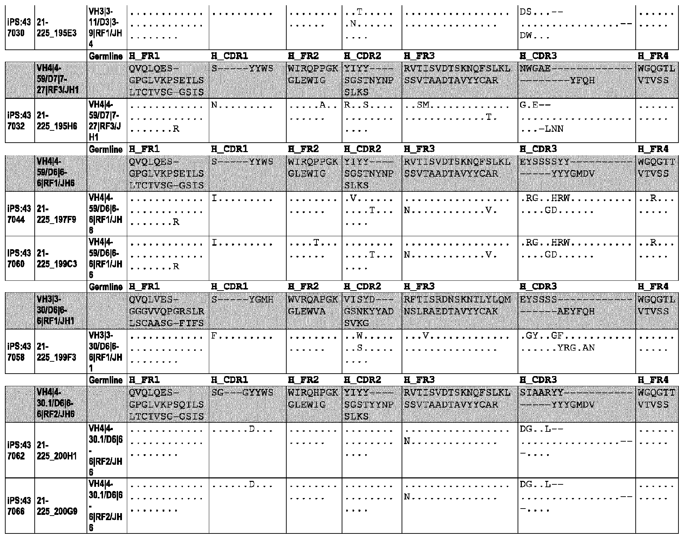

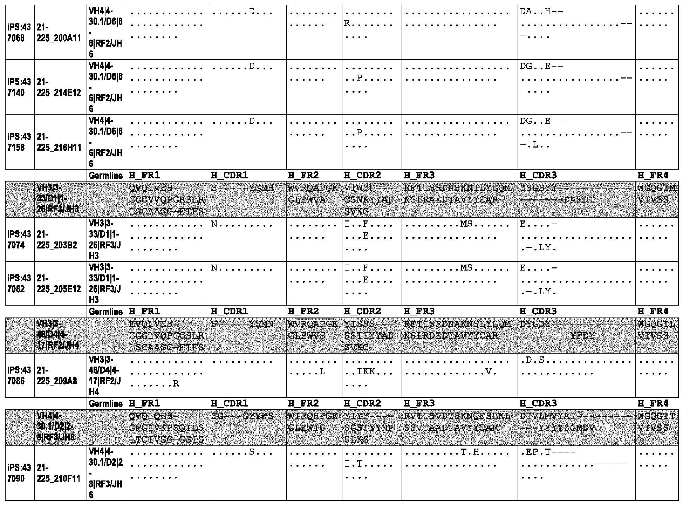

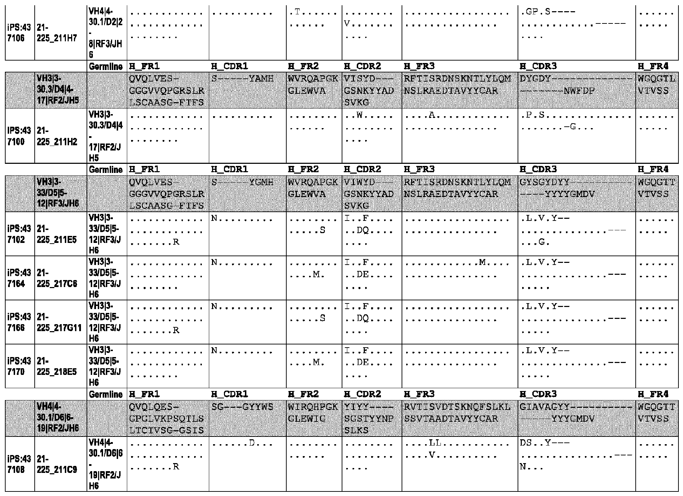

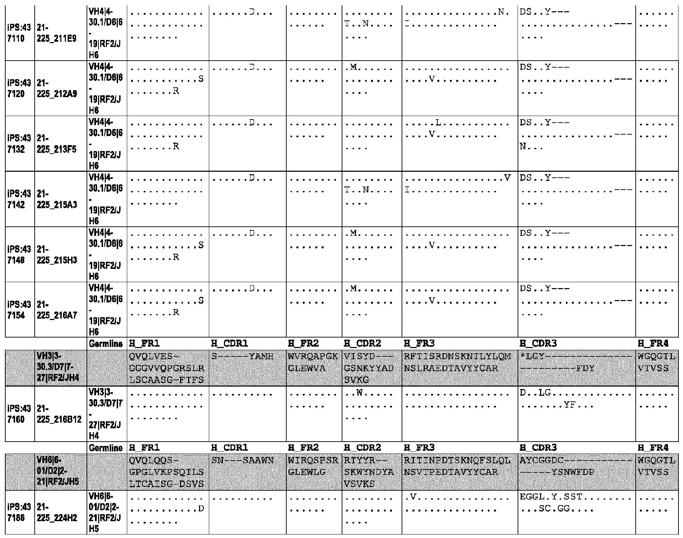

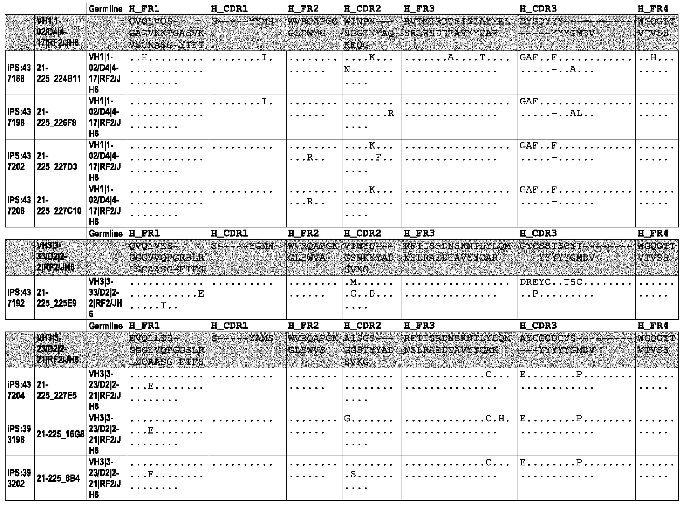

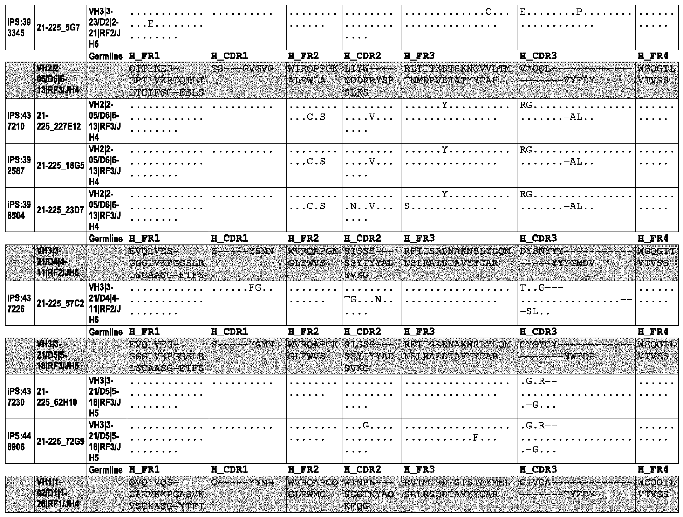

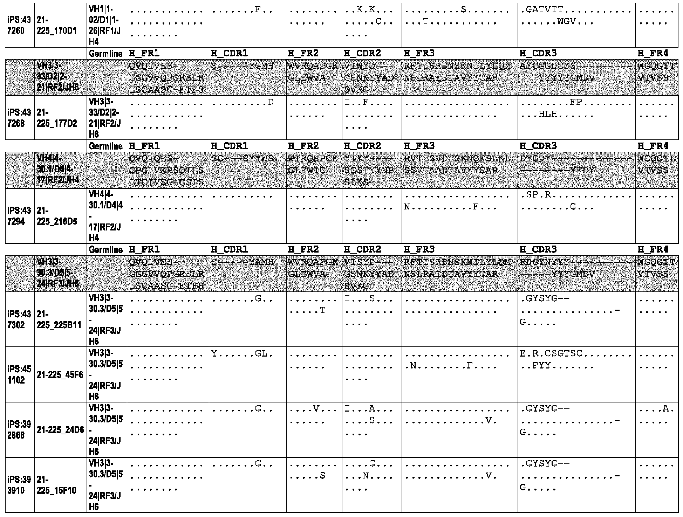

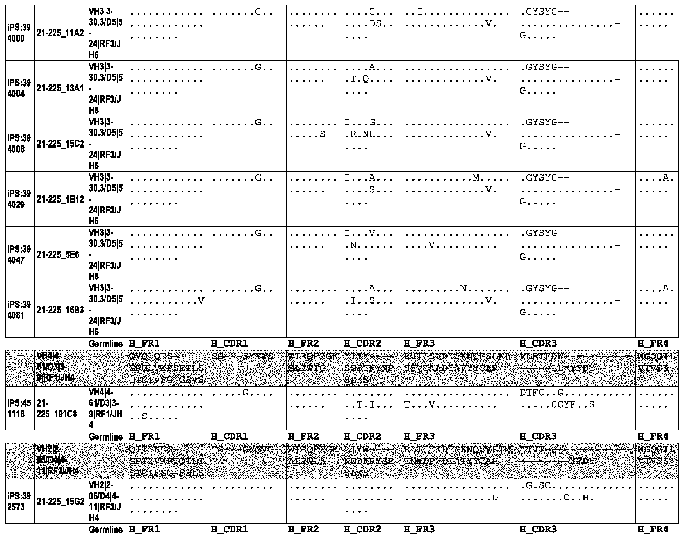

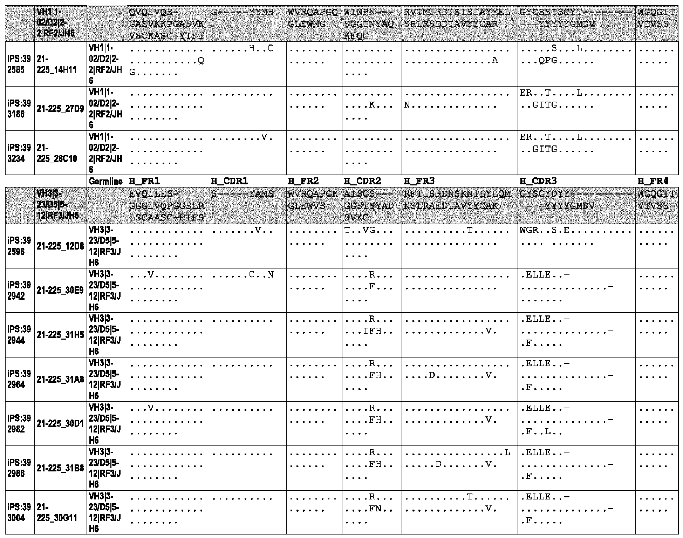

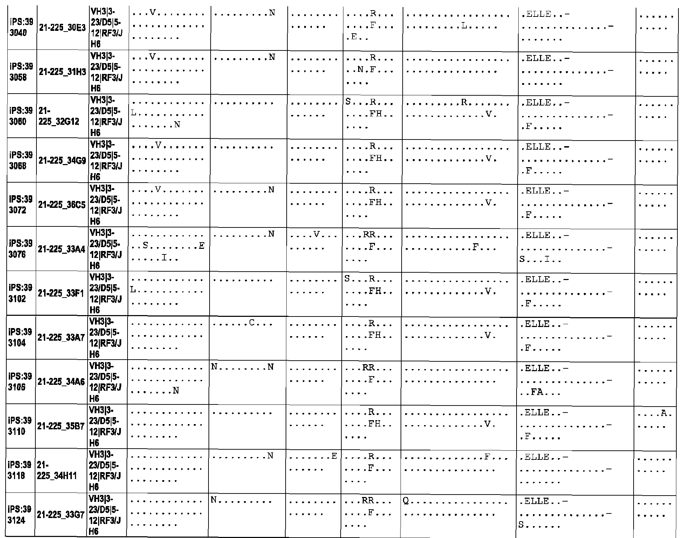

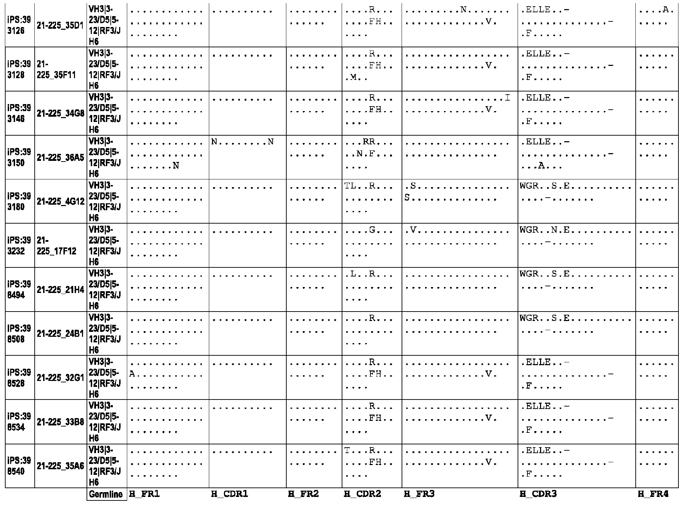

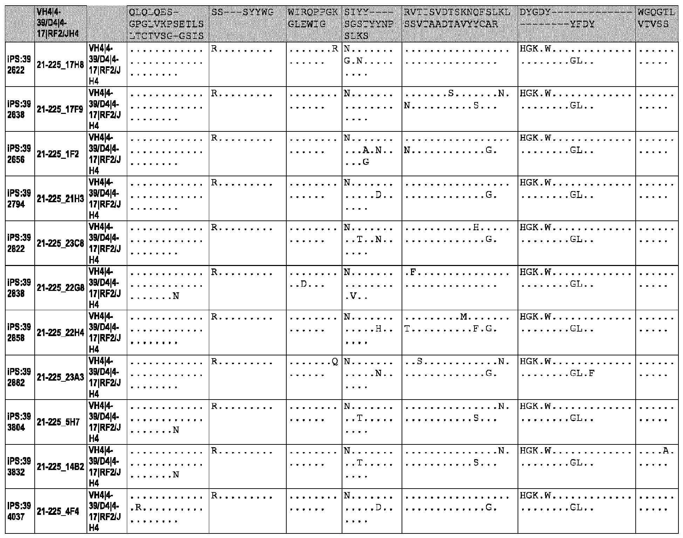

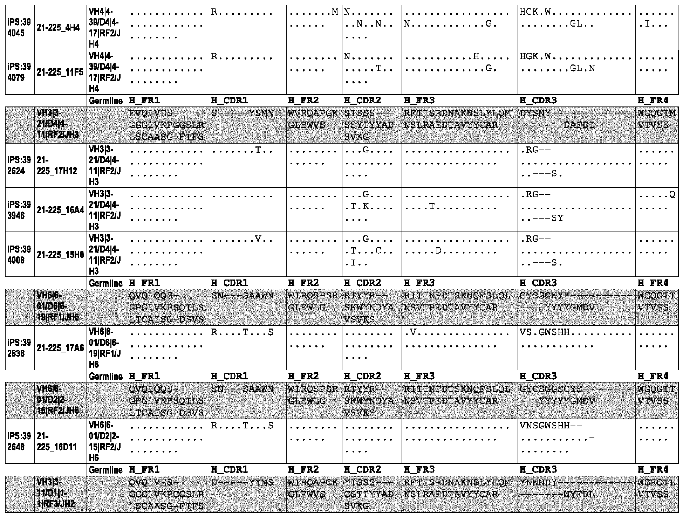

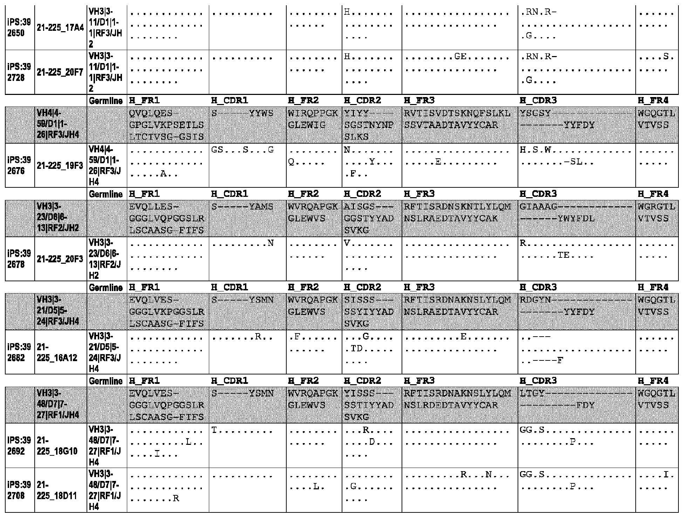

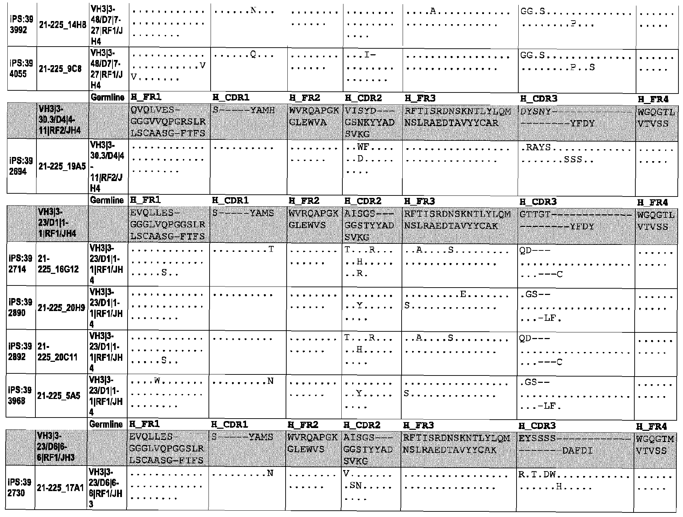

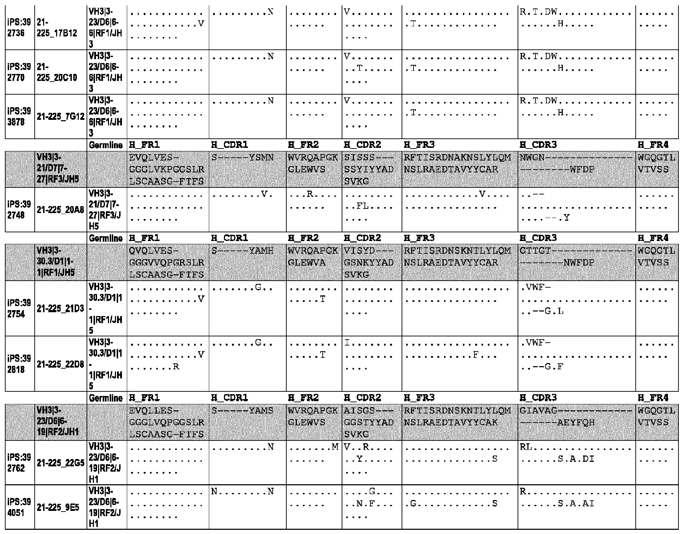

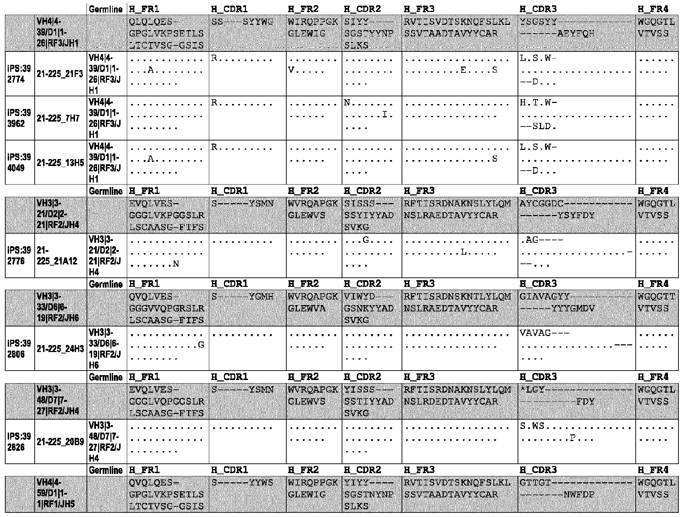

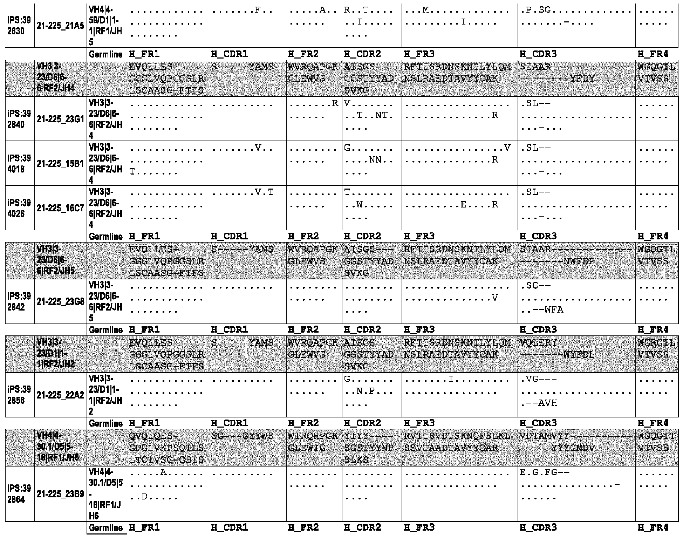

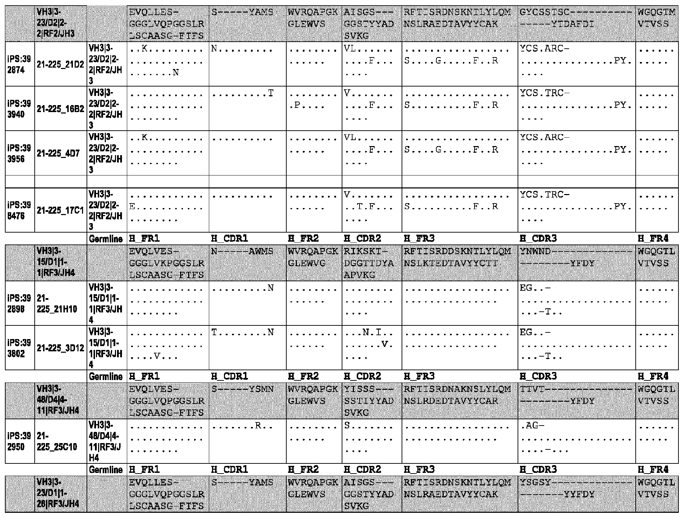

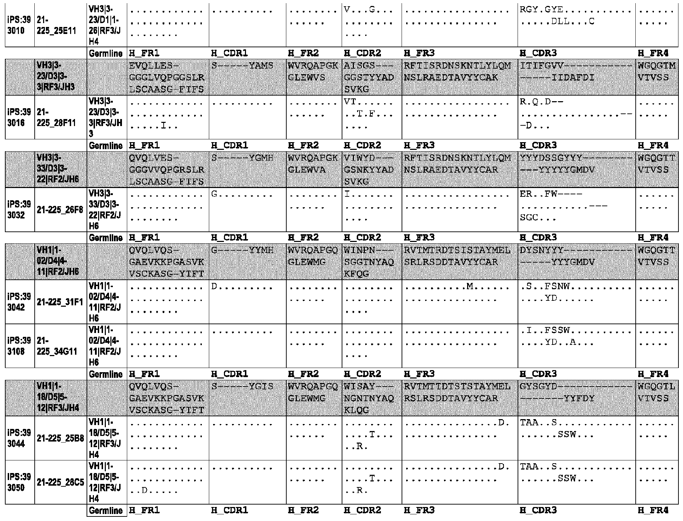

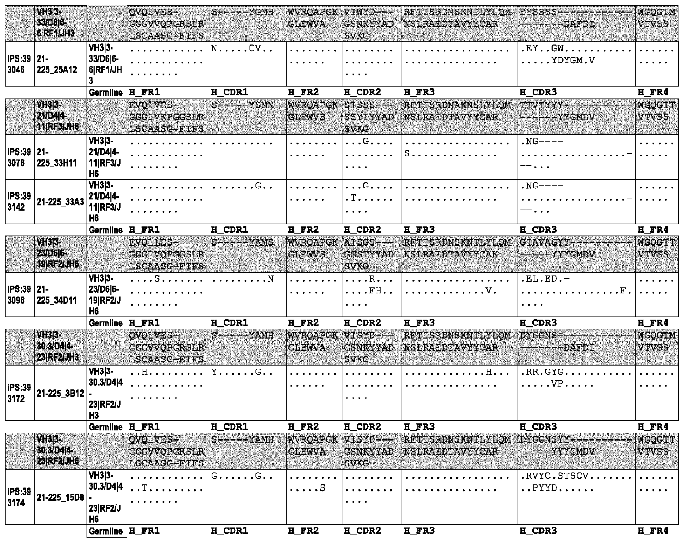

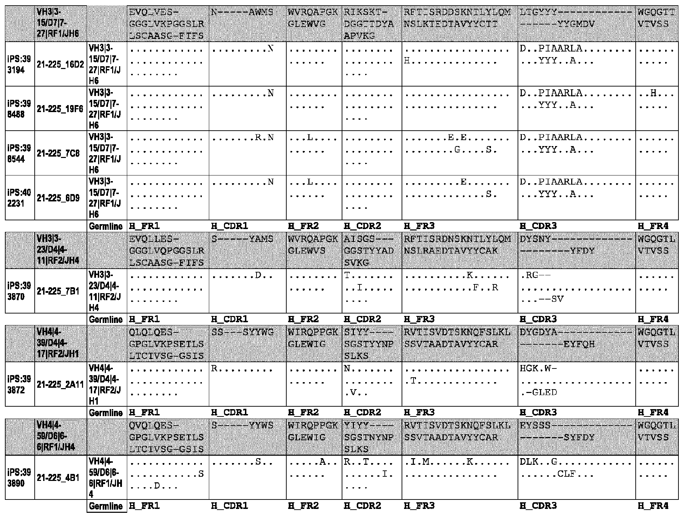

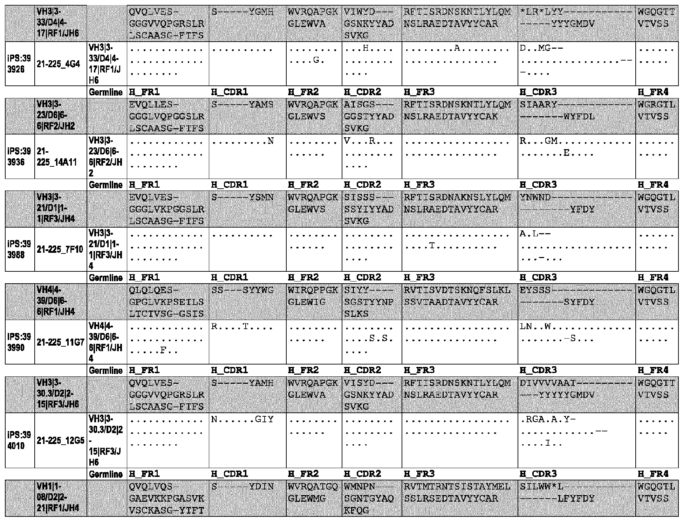

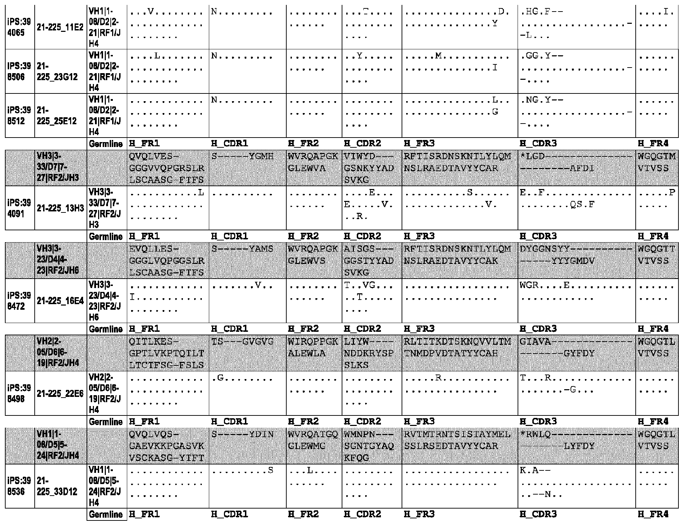

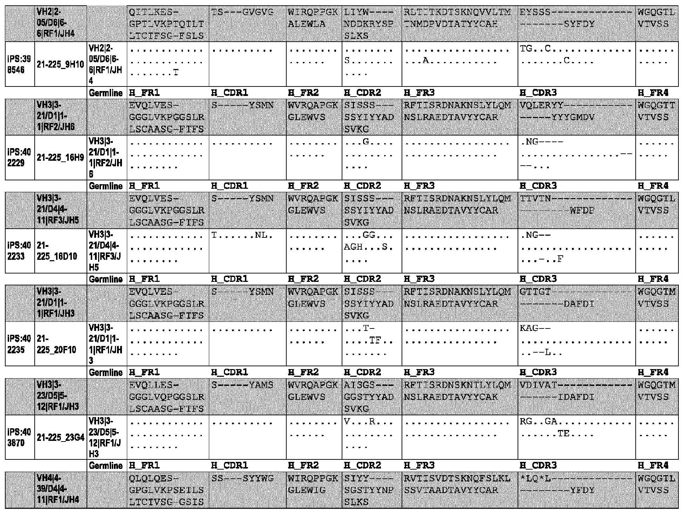

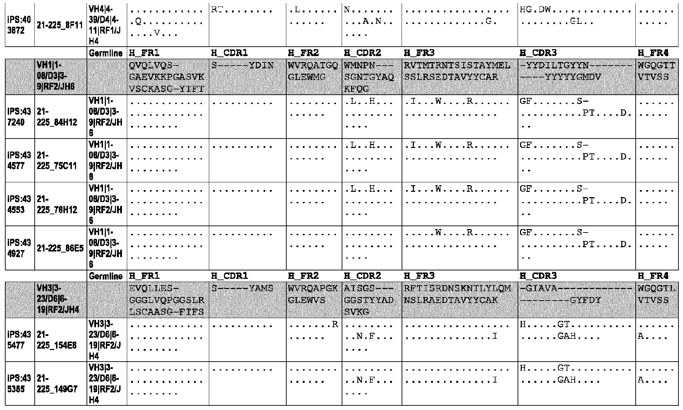

**Figure 52.** A table presenting a consensus protein alignment of light and heavy variable regions for certain antigen binding proteins of the present invention (Table 5). An asterisk "*" denotes a stop codon. Sequences containing a stop codon are respresented as distinct sequences in the Sequence Listing, however, these sequences are related. Generally speaking, however, the amino acid sequences of the light and heavy chain variable domains in the consensus protein alignment presented in Table 5 are wrapped due to space issues, and unless stated otherwise, like in the case of sequences with one or more stop codons, should be understood to be single amino acid sequences.

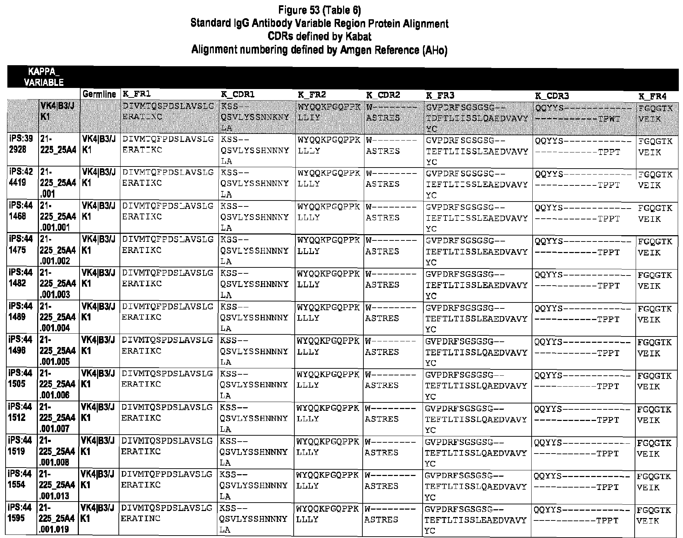

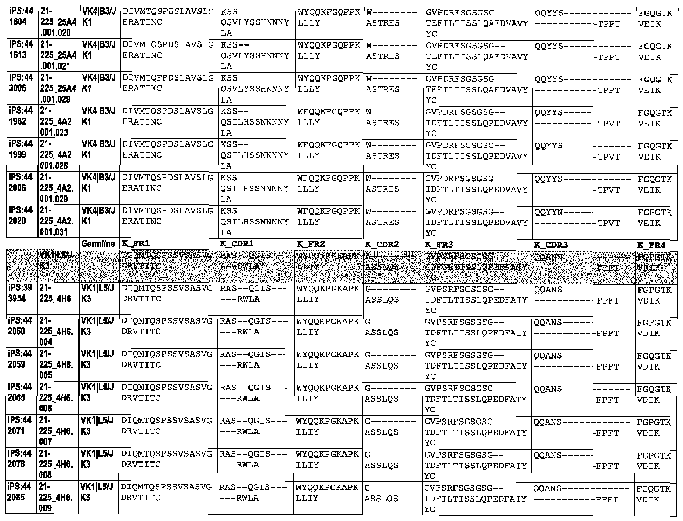

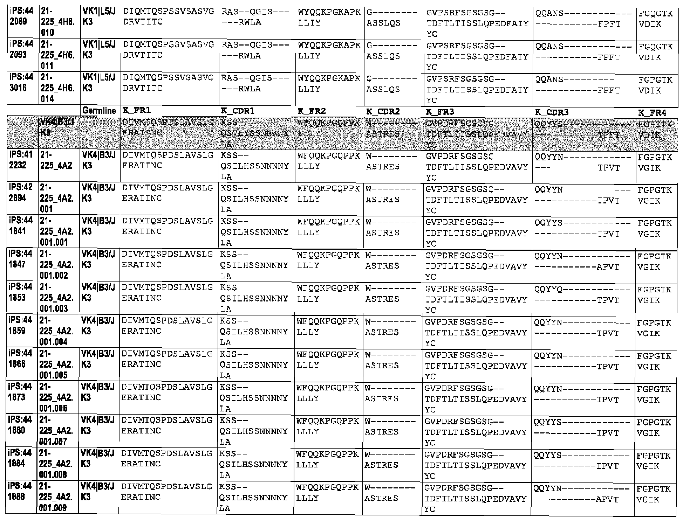

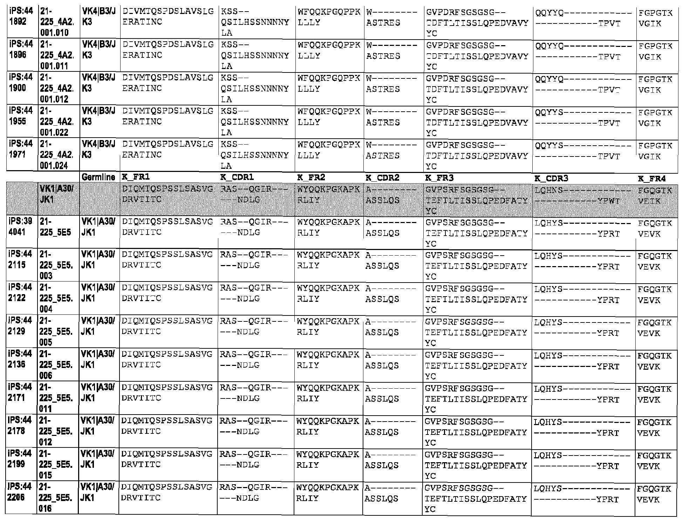

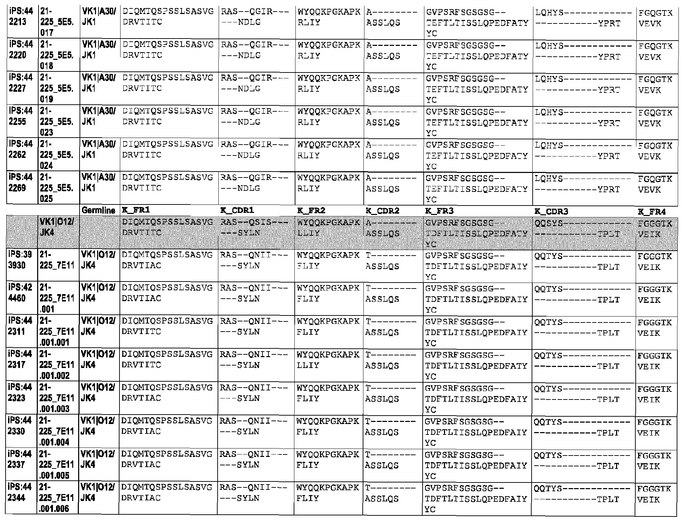

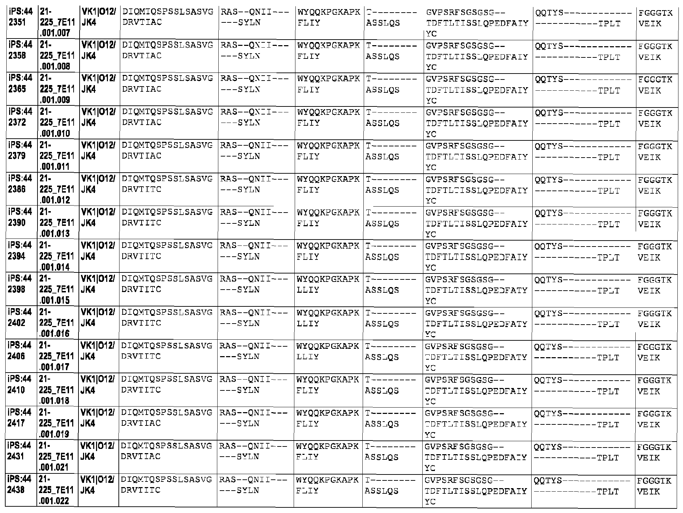

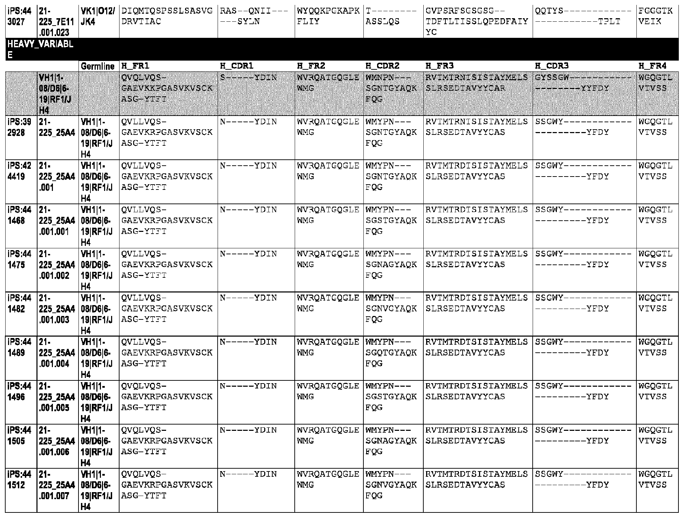

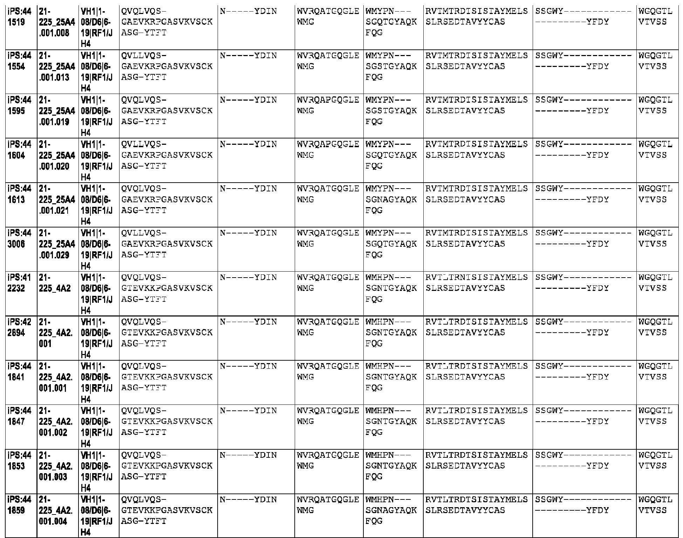

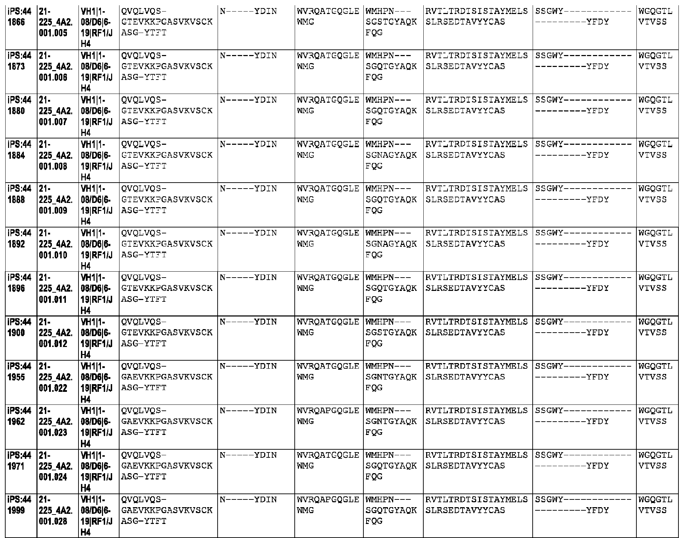

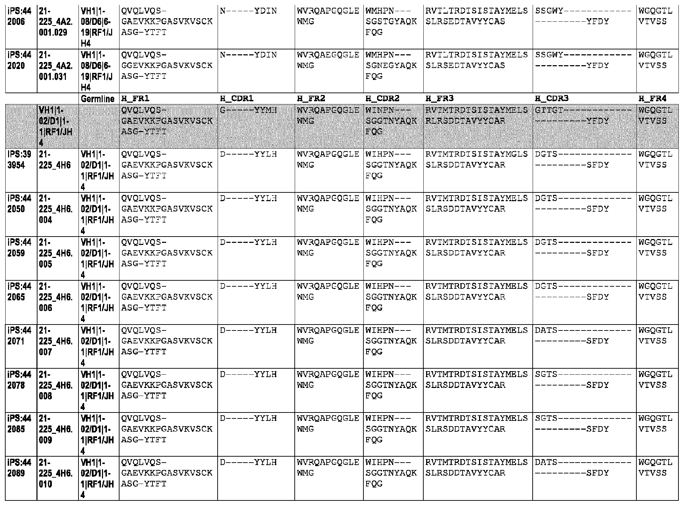

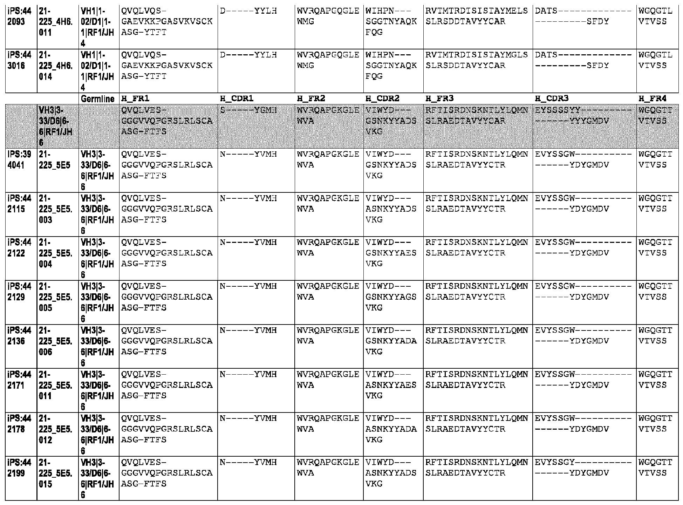

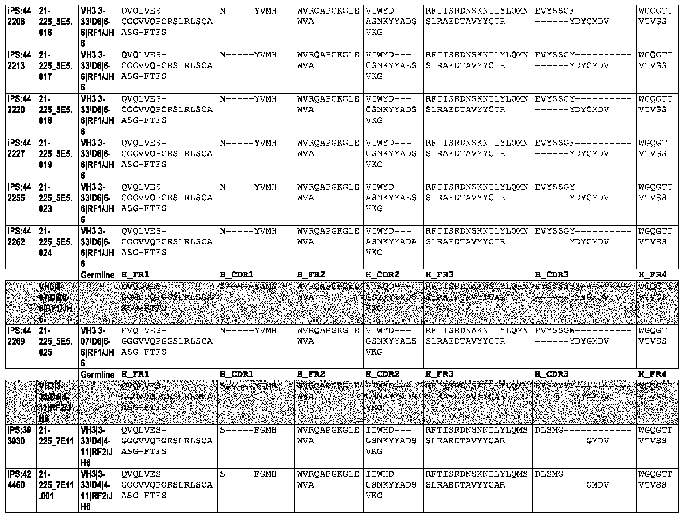

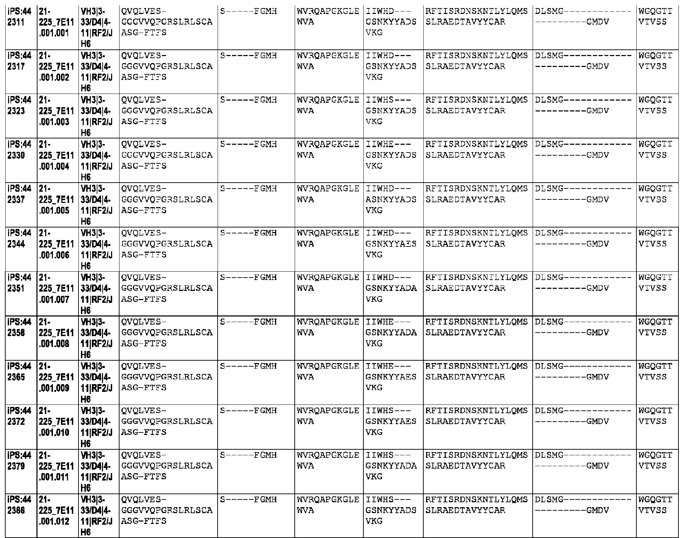

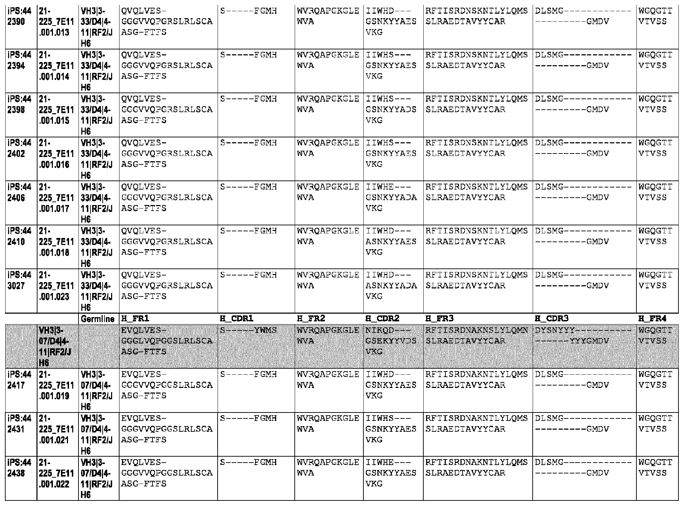

**Figure 53.** A table presenting a protein alignment of light and heavy variable regions for certain optimized antigen binding proteins of the present invention (Table 6). The amino acid sequences of the light and heavy chain variable domains in the protein alignment presented in Table 6 are wrapped due to space issues, and unless stated otherwise, should be understood to be single amino acid sequences.

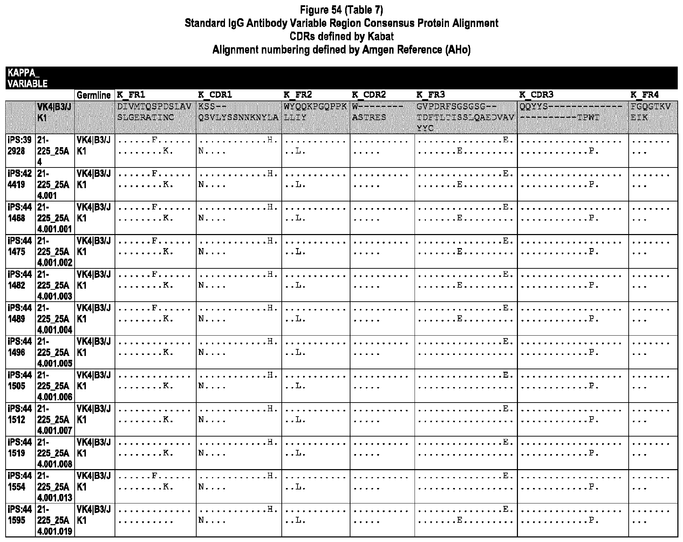

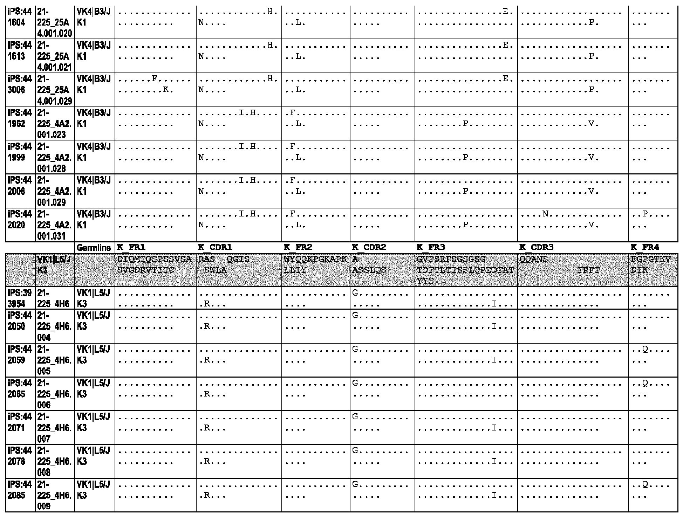

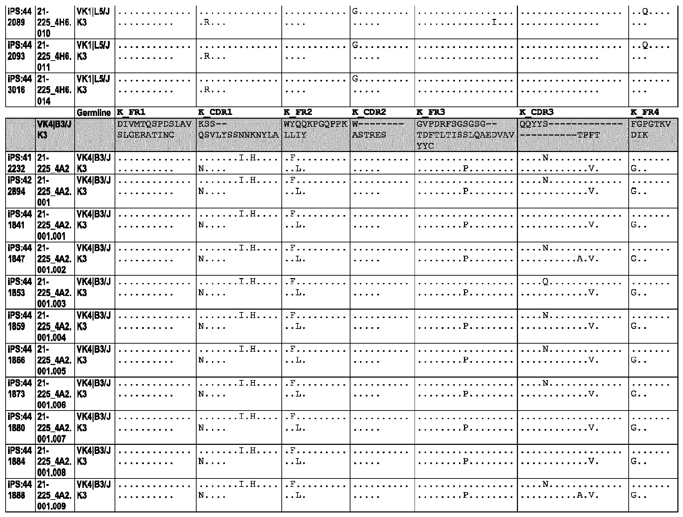

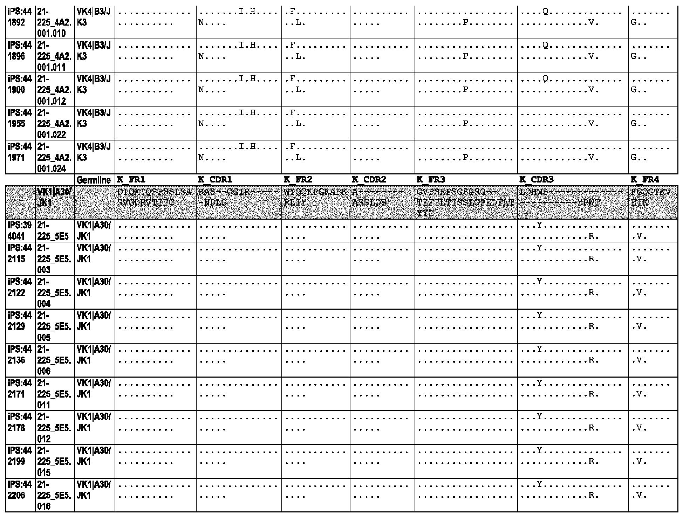

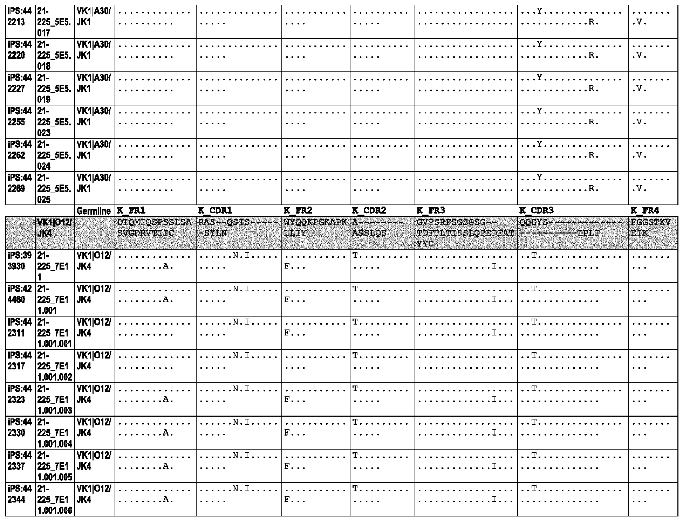

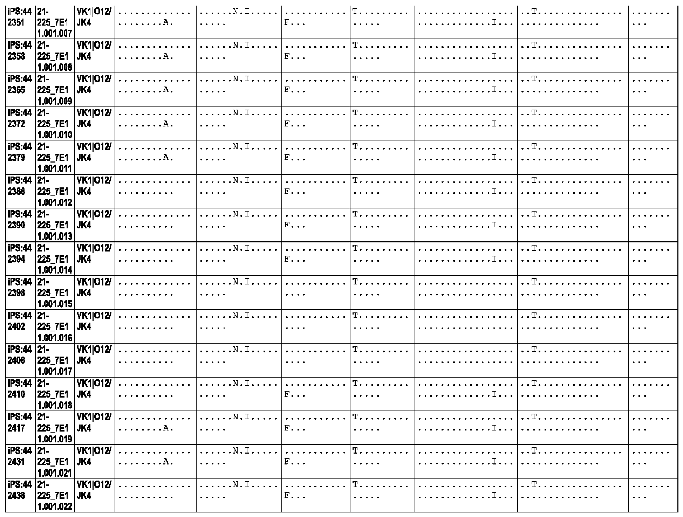

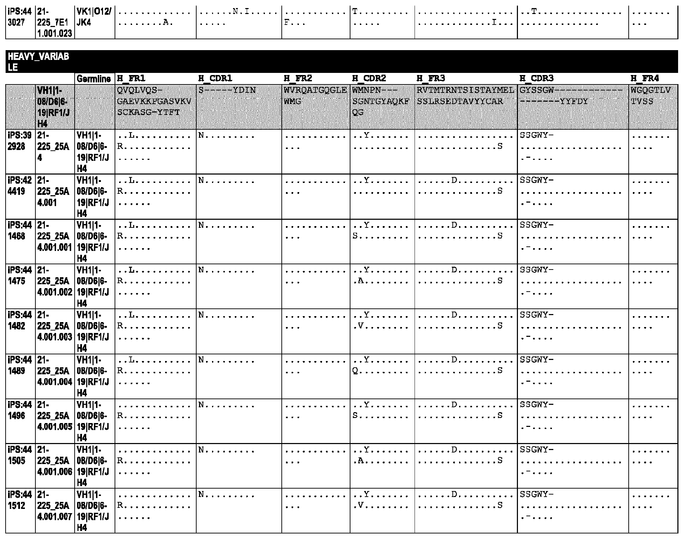

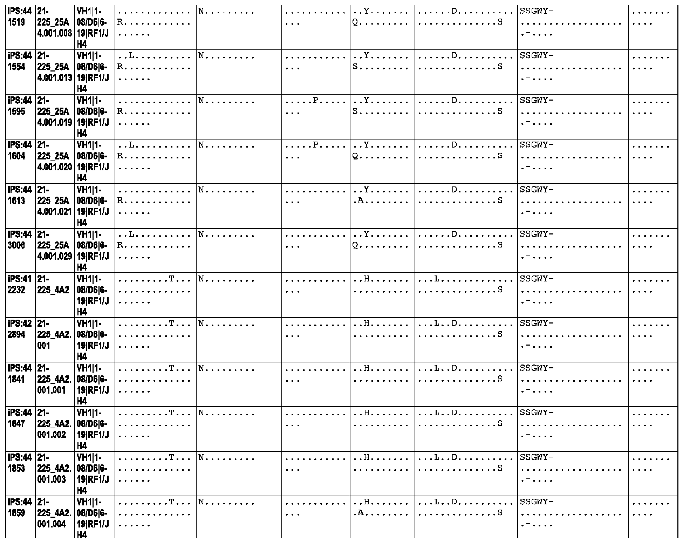

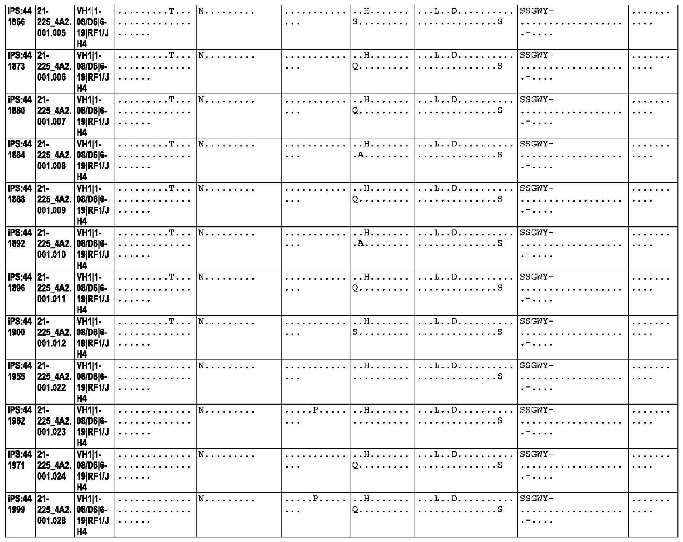

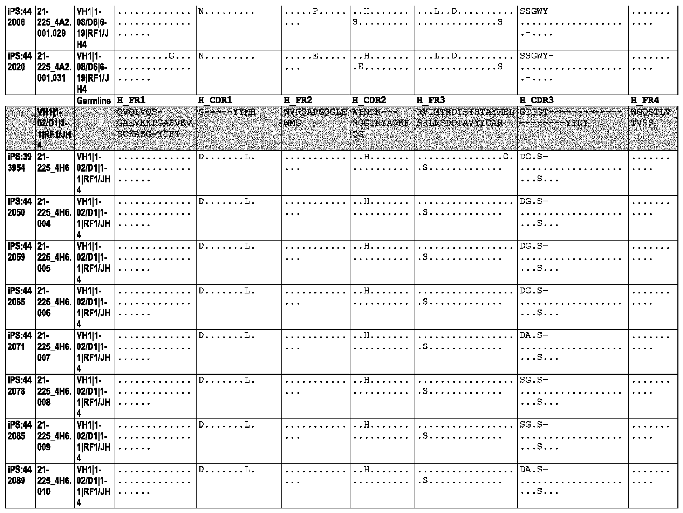

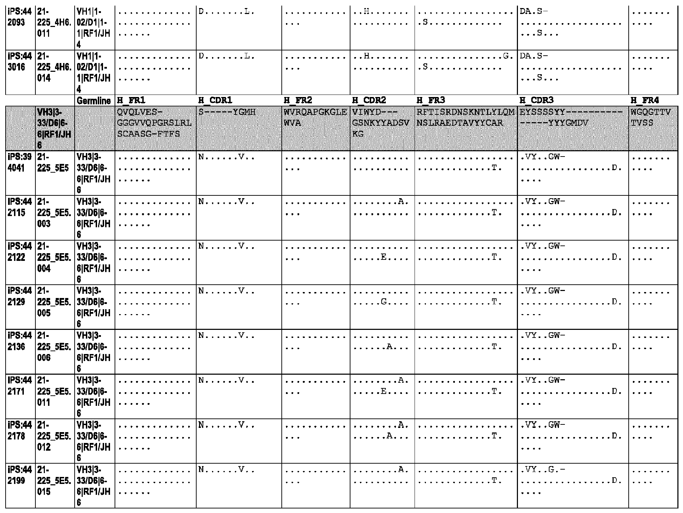

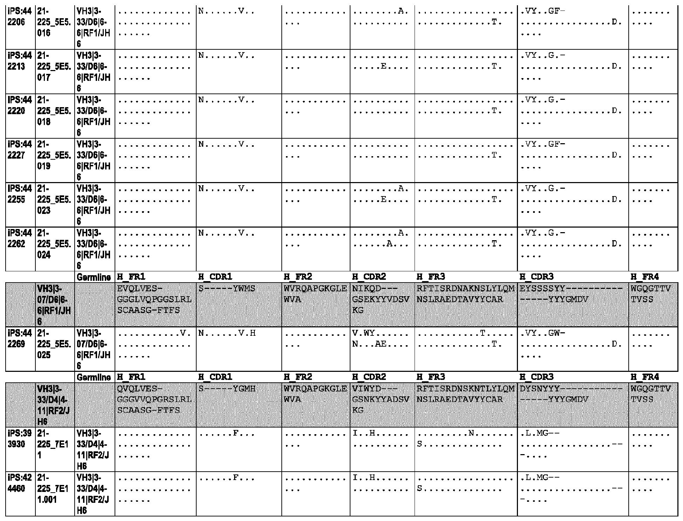

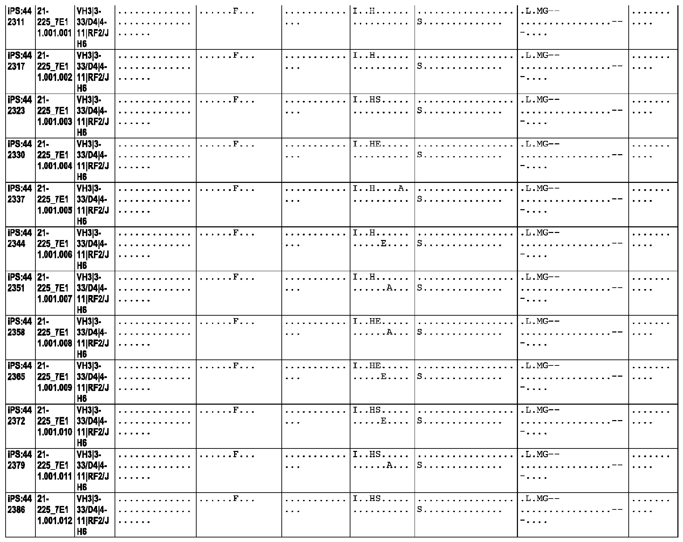

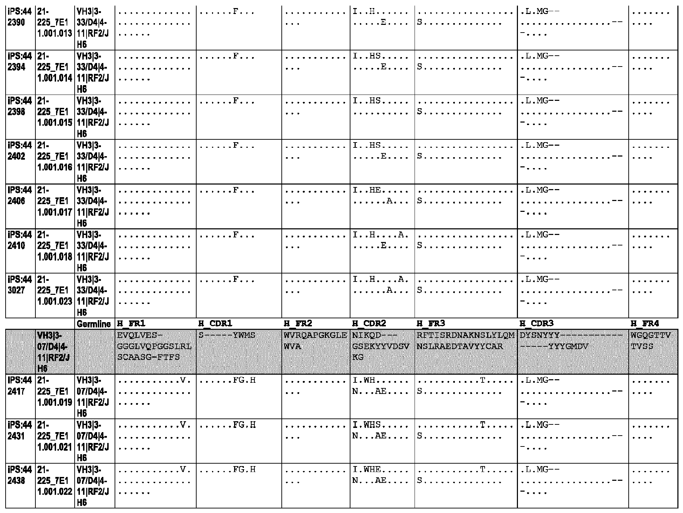

**Figure 54.** A table presenting a consensus protein alignment of light and heavy variable regions for certain optimized antigen binding proteins of the present invention (Table 7). The amino acid sequences of the light and heavy chain variable domains in the consensus protein alignment presented in Table 7 are wrapped due to space issues, and unless stated otherwise, should be understood to be single amino acid sequences.

**Figure 55.** A group of tables presenting the consensus sequences of various heavy and light chain variable regions (Tables 19A and 20A, respectively), as well as the consensus sequences of CDRs of various heavy and light chain variable regions (Tables 19B and C and Tables 20B and 20C, respectively) for certain antigen binding proteins of the present invention.

**Figure 56.** A group of tables presenting the detailed consensus protein alignment of various light and heavy chain variable regions for certain antigen binding proteins of the present invention (Tables 21-48). The shading of amino acid residues in the consensus protein alignment presented in Tables 21-48 denote particular residues that one of ordinary skill in the art may wish to target for engineering.

**Figure 57.** A group of tables presenting the consensus protein alignment of various light and heavy chain variable regions for certain antigen binding proteins of the present invention (Tables 49-134).

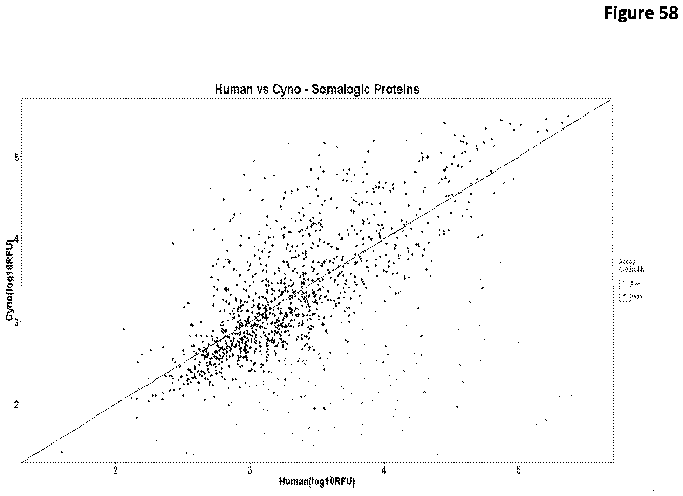

**Figure 58.** A graph depicting the credibility of protein measurements in cynomolgus monkey. Log10 RFU of mean protein levels in the two species are plotted and the ones with low credibility (light dots) and high credibility (darker dots) are marked.

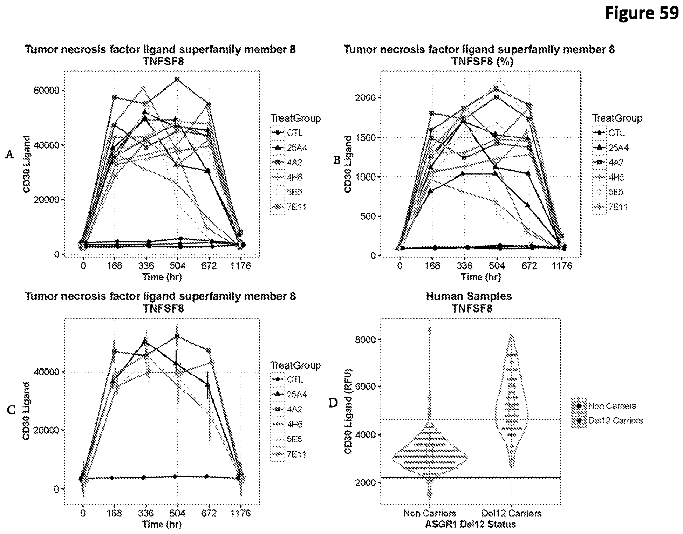

**Figure 59.** Serum protein analysis of cynomolgus monkey treated with anti-ASGR-1 antibodies. Panel A is a graph depicting TNFSF8 protein levels in individual animals of different treatment group across the time points. Panel B is a graph depicting normalized TNFSF8 protein levels (percent of time point 0) in individual animals of different treatment groups across the time points. Panel C is a graph depicting TNFSF8 protein levels in each treatment group (n=3, error bar represents the SEM), and Panel D is a graph depicting the distribution of TNFSF8 protein levels in human ASGR1 del12 carriers and non-carriers.

## DETAILED DESCRIPTION OF THE VARIOUS EMBODIMENTS

As shown in Example 1 below, sequence variants in ASGR-1 (which resulted in either a faster degrading ASGR1 or a loss of function ASGR1 mutation) resulted in a lowering in the level of non-HDL cholesterol in humans. This in turn resulted in a decrease in the risk of coronary artery disease experienced by these people. As loss of function mutations in ASGR-1 resulted in both the lowering of non-HDL cholesterol and the lowering of coronary artery disease, antibodies and inhibitory RNA that effectively block ASGR can be used to lower the risk of coronary artery disease.

The present invention is directed to inhibitors of ASGR, ASGR-1 and/or ASGR-2. The present invention provides antigen binding proteins that specifically bind to human ASGR, ASGR-1 and/or ASGR-2 and inhibit human ASGR, ASGR-1 and/or ASGR-2 binding to a ligand. The present invention also provides antigen binding proteins that specifically bind to other species of ASGR, ASGR-1 and/or ASGR-2. The present invention is further directed to methods of treating or preventing cardiovascular disease in a human subject comprising administering an inhibitor of ASGR, ASGR-1 and/or ASGR-2, wherein the ASGR inhibitor an antigen binding protein and/or an interfering RNA (e.g., siRNA or shRNA).

The present invention further provides compositions, kits, and methods relating to antigen binding proteins that specifically bind to human ASGR, human ASGR-1, and/or human ASGR-2. Also provided are nucleic acid molecules comprising a sequence of polynucleotides that encode all or a portion of a polypeptide that specifically binds to human ASGR, human ASGR-1, and/or human ASGR-2. The present invention further provides vectors and plasmids comprising such nucleic acids, and cells or cell lines comprising such nucleic acids and/or vectors and plasmids. The provided methods further include, for example, methods of making, identifying, or isolating antigen binding proteins that bind to human ASGR, human ASGR-1, and/or human ASGR-2, methods of determining whether an antigen binding protein binds to human ASGR, human ASGR-1, and/or human ASGR-2, methods of making compositions, such as pharmaceutical compositions, comprising an antigen binding protein that binds to human ASGR, human ASGR-1, and/or human ASGR-2, and methods for administering an antigen binding protein that binds human ASGR, human ASGR-1, and/or human ASGR-2 to a human subject.

It is to be understood that both the foregoing general description and the following detailed description are exemplary and explanatory only and are not restrictive of the invention as itemed. In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise. Also, the use of the term "portion" can include part of a moiety or the entire moiety.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well-known and commonly used in the art. The methods and techniques of the present invention are generally performed according to conventional methods well known in the art and as described in various general and more specific references that are cited and discussed throughout the present specification unless otherwise indicated. See, e.g., Sambrook et al. Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual Cold Spring Harbor Laboratory Press, Cold Spring Harbor, N.Y. (1990), which are incorporated herein by reference. Enzymatic reactions and purification techniques are performed according to manufacturer's specifications, as commonly accomplished in the art or as described herein. The terminology used in connection with, and the laboratory procedures and techniques of, analytical chemistry, synthetic organic chemistry, and medicinal and pharmaceutical chemistry described herein are those well-known and commonly used in the art. Standard techniques can be used for chemical syntheses, chemical analyses, pharmaceutical preparation, formulation, and delivery, and treatment of patients.

Polynucleotide and polypeptide sequences are indicated using standard one- or three-letter abbreviations. Unless otherwise indicated, polypeptide sequences have their amino termini at the left and their carboxy termini at the right, and single-stranded nucleic acid sequences, and the top strand of double-stranded nucleic acid sequences, have their 5' termini at the left and their 3' termini at the right. A particular section of a polypeptide can be designated by amino acid residue number such as amino acids 1 to 50, or by the actual residue at that site such as asparagine to proline. A particular polypeptide or polynucleotide sequence also can be described by explaining how it differs from a reference sequence.

The following terms, unless otherwise indicated, shall be understood to have the following meanings:

The term "inhibitor" as used herein, is a compound that decreases the magnitude of at least one activity or function of a molecule compared to the magnitude of the activity or function observed in the absence of the inhibitor. In some instances, an inhibitor will substantially decrease the magnitude of at least one activity or function of a molecule compared to the magnitude of the activity or function observed in the absence of the inhibitor. In some instances, an inhibitor will completely diminish the magnitude of at least one activity or function of a molecule compared to the magnitude of the activity or function observed in the absence of the inhibitor. Certain exemplary inhibitors include, but are not limited to, proteins, peptides, antibodies, peptibodies, aptamers, antisense oligonucleotides, interfering RNA, carbohydrates or small organic molecules.

The term "isolated molecule" (where the molecule is, for example, a polypeptide, a polynucleotide, antigen binding protein or an antibody) is a molecule that by virtue of its origin or source of derivation (1) is not associated with naturally associated components that accompany it in its native state, (2) is substantially free of other molecules from the same species (3) is expressed by a cell from a different species, or (4) does not occur in nature. Thus, a molecule that is chemically synthesized, or expressed in a cellular system different from the cell from which it naturally originates, will be "isolated" from its naturally associated components. A molecule also may be rendered substantially free of naturally associated components by isolation, using purification techniques well known in the art. Molecule purity or homogeneity may be assayed by a number of means well known in the art. For example, the purity of a polypeptide sample may be assayed using polyacrylamide gel electrophoresis and staining of the gel to visualize the polypeptide using techniques well known in the art. For certain purposes, higher resolution may be provided by using HPLC or other means well known in the art for purification.

The terms "polynucleotide," "oligonucleotide" and "nucleic acid" are used interchangeably throughout and include DNA molecules (e.g., cDNA or genomic DNA), RNA molecules (e.g., mRNA), analogs of the DNA or RNA generated using nucleotide analogs (e.g., peptide nucleic acids and non-naturally occurring nucleotide analogs), and hybrids thereof. The nucleic acid molecule can be single-stranded or double-stranded. In one embodiment, the nucleic acid molecules of the invention comprise a contiguous open reading frame encoding an antibody, or a fragment, derivative, mutein, or variant thereof, of the invention.

A "vector" is a nucleic acid that can be used to introduce another nucleic acid linked to it into a cell. One type of vector is a "plasmid," which refers to a linear or circular double stranded DNA molecule into which additional nucleic acid segments can be ligated. Another type of vector is a viral vector (e.g., replication defective retroviruses, adenoviruses and adeno-associated viruses), wherein additional DNA segments can be introduced into the viral genome. Certain vectors are capable of autonomous replication in a host cell into which they are introduced (e.g., bacterial vectors comprising a bacterial origin of replication and episomal mammalian vectors). Other vectors (e.g., non-episomal mammalian vectors) are integrated into the genome of a host cell upon introduction into the host cell, and thereby are replicated along with the host genome. An "expression vector" is a type of vector that can direct the expression of a chosen polynucleotide.

A nucleotide sequence is "operably linked" to a regulatory sequence if the regulatory sequence affects the expression (e.g., the level, timing, or location of expression) of the nucleotide sequence. A "regulatory sequence" is a nucleic acid that affects the expression (e.g., the level, timing, or location of expression) of a nucleic acid to which it is operably linked. The regulatory sequence can, for example, exert its effects directly on the regulated nucleic acid, or through the action of one or more other molecules (e.g., polypeptides that bind to the regulatory sequence and/or the nucleic acid). Examples of regulatory sequences include promoters, enhancers and other expression control elements (e.g., polyadenylation signals). Further examples of regulatory sequences are described in, for example, Goeddel, 1990, Gene Expression Technology: Methods in Enzymology 185, Academic Press, San Diego, CA and Baron et al., 1995, Nucleic Acids Res. 23:3605-06.

A "host cell" is a cell that can be used to express a nucleic acid, e.g., a nucleic acid of the invention. A host cell can be a prokaryote, for example, E. coli, or it can be a eukaryote, for example, a single-celled eukaryote (e.g., a yeast or other fungus), a plant cell (e.g., a tobacco or tomato plant cell), an animal cell (e.g., a human cell, a monkey cell, a hamster cell, a rat cell, a mouse cell, or an insect cell) or a hybridoma. Typically, a host cell is a cultured cell that can be transformed or transfected with a polypeptide-encoding nucleic acid, which can then be expressed in the host cell. The phrase "recombinant host cell" can be used to denote a host cell that has been transformed or transfected with a nucleic acid to be expressed. A host cell also can be a cell that comprises the nucleic acid but does not express it at a desired level unless a regulatory sequence is introduced into the host cell such that it becomes operably linked with the nucleic acid. It is understood that the term host cell refers not only to the particular subject cell but to the progeny or potential progeny of such a cell. Because certain modifications may occur in succeeding generations due to, e.g., mutation or environmental influence, such progeny may not, in fact, be identical to the parent cell, but are still included within the scope of the term as used herein.

ASGR

Genomic database analysis is one manner that allows for the discovery of associations between disease states and particular targets and/or pathways. For example, genetic analysis of patients with familial hypercholesterolemia resulted in the discovery of proprotein convertase subtilisin/kexin type 9 (PCSK9) being involved with regulating serum LDL cholesterol levels and risk of developing coronary artery disease, and ultimately, in the development of the recently approved Repatha®, an anti-hPCSK9 antibody, (see, e.g., Jackson et al., U.S. Patent No. 8,030,457). Advances in DNA sequencing technology provide the means to sequence the genomes of large numbers of individuals allowing for discovery of rare variants. deCODE Genetics (an Amgen company) has previously reported methods to analyze whole genomes of large numbers of Icelanders in order to search for associations between genetic variants and traits of interest. (Gudbjartsson et al., Nature Genetics; Vol. 47; 5; May 2015; p.435-444).

This methodology has now been applied in the search for novel genetic variants that affect cardiovascular disease, including cholesterol levels, and the risk for developing coronary artery disease and myocardial infarction (MI). The groundbreaking analysis performed has identified novel sequence variants of the Ashwell-Morell Receptor that are implicated in cardiovascular disease.

In the present invention, whole-genome sequencing of the Icelandic population discovered a rare, 12 base pair deletion ("del12") in intron 4 of the ASGR-1 gene that is also present in other European ancestry populations. This deletion leads to a frameshift predicted to generate a truncated ASGR-1 receptor subunit that is lacking both the oligomerization and extracellular carbohydrate recognition domains (also known as "CRD," "carbohydrate binding domain" or "CBD") or may generate an unstable and rapidly degraded transcript (and therefore no protein) due to nonsense mediated decay. In the present invention, whole-genome sequencing of the Icelandic population also discovered a second rare loss of function variant in the ASGR-1 gene; namely, a 4 base pair insertion in exon 7 (c.469-472dupAACT or "W158X"). This 4 base pair insertion in exon 7 causes a frameshift and introduces a premature stop codon at amino acid 158 out of the 291 amino acid full length protein (NP_001662.1 :p.W158X). This variant is predicted to encode a protein lacking the carbohydrate recognition domain of the receptor or may generate an unstable and rapidly degraded transcript (and therefore no protein) due to nonsense mediated decay. Furthermore, the W158X variant effects all reported refseq transcripts of ASGR-1 regardless of tissue or cell type of expression. Without wishing to be bound by any particular hypothesis, the analysis indicates that del12 and W158X results in lower non-HDL cholesterol levels, protection against CAD and MI, leading to prolonged life. Additionally, the analysis indicates that del12 and W158X also associates with increased levels of circulating ALP and vitamin B12. Supporting this del12 and W158X association with increased levels of ALP are data from mice having a Y272C variant in ASGR-1, showing that these mice exhibit a phenotype of increased plasma ALP (Sabrautzki et al., Mamm. Genome, 23, 416-430, 2012). The Y272 position in mouse ASGR-1 corresponds to the Y273 position in human ASGR-1 (see Figure 1A).

The Ashwell-Morell Receptor (AMR), originally named the hepatic asialoglycoprotein receptor, was one of the first cellular receptors to be isolated and identified. (Grewal, Methods in Enzymology, Volume 479, Chapter 13, 2010, pp.223-241). This receptor is also known as the Ashwell Receptor, the hepatic galactose/N-acetylgalactosamine (Gal-NAc) receptor, or the hepatic lectin receptor. However, this receptor is now more commonly known as "ASGPR," or simply "ASGR."

ASGR is a C-type lectin that is expressed on the surface of hepatocytes and is made up of 48 kDa major subunit(s) (ASGR-1) and 40 kDa minor subunit(s) (ASGR-2). (Roggenbuck et al., Autoimmune Highlights, 2012, 3:119-125). Functional variants of ASGR are formed by the oligomerization of the ASGR-1 and ASGR-2 subunits. (Grewal). The receptor complexes can comprise homo-oligomers and hetero-oligomers of the ASGR-1 and ASGR-2 subunits, with (ASGR-1)$_2$-(ASGR-2)$_1$ trimer being the most common form and having the highest affinity to substrate. (Grewal). Other identified forms of ASGR include (ASGR-1)$_2$, (ASGR-1)$_3$, (ASGR-1)$_2$-(ASGR-2)$_2$, (ASGR-1)$_3$-(ASGR-2)$_2$. (Grewal).

The polynucleotide and polypeptide sequences for several species of ASGR-1 and ASGR-2 are known. Table 1 presents sequences for human, mouse, rat, pig, dog and cynomolgus. Figures 1A, 1B and 2 present sequence alignments of various species of ASGR-1 and ASGR-2, and Figure 3 presents a sequence alignment between human ASGR-1 and human ASGR-2.

ASGR-1 is a single pass transmembrane protein and is the major subunit of ASGR. The galactose (Gal) or N-acetylgalactosamine (GalNAc) residues of glycoproteins are exposed by removal of sialic acid by sialidases, hence the term asialoglycoprotein for the ligands of ASGR. Although ASGR expression is detected in other tissues, liver is the predominant site of expression. A circulating form of the receptor, generated from ASGR-1 transcripts lacking exon two, has also been reported. (Liu J, Hu B, Yang Y, et al. A new splice variant of the major subunit of human asialoglycoprotein receptor encodes a secreted form in hepatocytes. PloS one 2010;5:e12934). The del12 and W158X variants are predicted to truncate both the membrane bound and the circulating form of the receptor, and as mentioned above, the W158X variant may generate an unstable and rapidly degraded transcript (and therefore no protein) due to nonsense mediated decay.

The primary reported function of ASGR is to bind and internalize glycoproteins in the circulation that contain terminal galactose or N-acetylgalactosamine residues (asialoglycoproteins), resulting in the clearance of these proteins from the circulation. (Roggenbuck). Reported endogenous ligands include components of the blood coagulation system, such as platelets and Von Willebrand Factor. (Grewal).

As used herein, the terms "ASGR, ASGR-1, and/or ASGR-2 function" or "ASGR, ASGR-1, and/or ASGR-2 activity" includes any biological effect of ASGR, ASGR-1 and/or ASGR-2. In certain embodiments, ASGR function or activity includes the ability of ASGR to interact or bind to a ligand. In some embodiments, ASGR function or activity is represented by the ability of ASGR to interact or bind to sugars including but not limited to lactose, galactose, and/or GalNAc or glycoproteins displaying such sugars including but not limited to fetuin, orosomucoid and/or alkaline phosphatase. In some embodiments, ASGR function or activity includes any biological activity resulting from ASGR response. Exemplary activities include, but are not limited to, clearance of asialoglycoproteins from the circulation; clearance of IgA from circulation; removal of apoptotic cells; clearance of low density lipoprotein (LDL) and/or the disposal of cellular fibronectin (Roggenbuck).

Given the location of ASGR on the surface of liver hepatocytes and its implication in hepatocyte entry by certain viruses (Roggenbuck), the receptor has become a target of convenience for therapeutics that require delivery to the liver and internalization into the cells. Examples of these uses include the targeted delivery of doxorubicin to hepatocellular carcinoma (Wei et al., Int J Nanomedicine, 2015, 10:5123-37), gene delivery to hepatocytes (D'Souza et al., J Control Release, 2015, 203:126-39), and targeted delivery of siRNA to hepatocytes (Rajeev et al., Chembiochem, 2015, 16(6):903-8).

Although the ASGR and its ability to mediate endocytosis and degradation of desialated glycoproteins has been known for nearly 4 decades, the endogenous ligands and the physiological function of the receptor have been difficult to establish. (Weigel PH, Yik JH. Glycans as endocytosis signals: the cases of the asialoglycoprotein and hyaluronan/chondroitin sulfate receptors. Biochimica et biophysica acta 2002;1572:341-63). It has been reported that ASGR-1-/- mice (lacking any ASGR activity) thrive normally and do not accumulate desialylated glycoproteins in their circulation although they are unable to clear exogenously added asialoglycoproteins, suggesting that under normal physiological condition ASGR is not essential for homeostasis of circulating asialoglycoproteins. (Tozawa R, Ishibashi S, Osuga J, et al. Asialoglycoprotein receptor deficiency in mice lacking the major receptor subunit. Its obligate requirement for the stable expression of oligomeric receptor. The Journal of Biological Chemistry 2001;276:12624-8).

In contrast to the ASGR-1 knockout mice which lack an apparent phenotype, the present invention has established a clear physiological role for human ASGR-1 in cardiovascular disease, for example, but not limited to, the regulation of non-HDL levels and modulation of CAD and MI risk. The present invention has also demonstrated the association of del12 and W158X with increased levels of circulating ALP and vitamin B 12. Furthermore, the present invention shows that disturbing one allele of ASGR-1 appears to have an overall beneficial effect as heterozygotes carriers of del12 live on average 1.5 years longer than non-carriers.

Surprisingly, the various embodiments provided herein demonstrate that the del12 variant and the W158 variant both have an effect on non-HDL levels that is opposite to their effect on ALP and vitamin B12 levels; decreasing non-HDL and increasing ALP and vitamin B12. While not wishing to be bound by any particular hypothesis, it is important to note that the common variant previously described that associates with ALP and LDL cholesterol also has opposing effects on these serum components; hence ASGR-1 may affect the level of these molecules through different mechanisms. It is unlikely that the ALP increase mediated by de112 or W158X reflects an underlying liver disease since other measures of liver function are not affected. Both ALP and the vitamin B12 transporter in the circulation, haptocorrin, are asialylated glycoproteins known to bind ASGR-1 and be cleared from the circulation by the receptor (Tuin A, Huizinga-Van der Vlag A, van Loenen-Weemaes AM, Meijer DK, Poelstra K. On the role and fate of LPS-dephosphorylating activity in the rat liver. American Journal of Physiology Gastrointestinal and Liver Physiology 2006;290:G377-85; Furger E, Fedosov SN, Lildballe DL, et al. Comparison of recombinant human haptocorrin expressed in human embryonic kidney cells and native haptocorrin. PloS one 2012;7:e37421; Burger RL, Schneider RJ, Mehlman CS, Allen RH. Human plasma R-type vitamin B12-binding proteins. II. The role of transcobalamin I, transcobalamin III, and the normal granulocyte vitamin B12-binding protein in the plasma transport of vitamin B12. The Journal of Biological Chemistry 1975;250:7707-13; Steirer LM, Park EI, Townsend RR, Baenziger JU. The asialoglycoprotein receptor regulates levels of plasma glycoproteins terminating with sialic acid alpha2,6-galactose. The Journal of Biological Chemistry 2009;284:3777-83). While not wishing to be bound by any particular hypothesis, the more likely reason for the increased levels of ALP and vitamin B12 in del12 carriers and in W158X carriers is decreased clearance of desialylated forms of these molecules from the circulation, due to reduced number of functional ASGR receptors in del12 carriers and in W158X carriers, suggesting a role for ASGR-1 in maintaining homeostasis of circulating ALP and vitamin B12.

While not wishing to be bound by any particular hypothesis, the decreased levels of non-HDL in del12 carriers and in W158X carriers in the face of reduced ASGR-1 function suggest that ASGR-1 affects non-HDL levels by mechanisms other than direct binding and endocytosis of cholesterol particles. In mice expressing a hypomorphic form of neuraminidase 1 (Neu1), a sialidase that cleaves the sialic acid residues thereby generating substrates for ASGR-1, the LDL receptor (LDLR) is sialylated and this form of the receptor was more stable and took up LDL cholesterol more avidly (LDL levels were decreased in these mice) than the asialylated form of the wild type LDLR (Yang A, Gyulay G, Mitchell M, White E, Trigatti BL Igdoura SA. Hypomorphic sialidase expression decreases serum cholesterol by downregulation of VLDL production in mice Journal of Lipid Research 2012;53:2573-2585). Both ASGR and LDLR are located in clathrin-coated pits on hepatocytes and ASGR may be capable of interacting with the asialylated form of the LDLR and blocking its activity.

Two novel rare variants in ASGR-1 have been identified herein that play a role in cardiovascular disease, including, but not limited to, lowering non-HDL levels and protecting against CAD and MI. These variants disrupt ASGR-1 protein function. Accordingly, the present invention is further directed to methods of inhibiting ASGR function, methods of inhibiting ASGR-1 function and/or methods of inhibiting ASGR-2 function. The present invention is further directed to molecules (for example, but not limited to, antigen binding proteins or interfering RNA) that inhibit ASGR function, ASGR-1 function and/or ASGR-2 function.

Antigen binding proteins

In some embodiments, the invention comprises antigen binding proteins that bind to ASGR, ASGR-1, and/or ASGR-2 of different species, including, but not limited to, human, cynomolgus, porcine, canine, murine and rat. In some embodiments, the antigen binding proteins specifically bind to ASGR, ASGR-1, and/or ASGR-2 of different species, including, but not limited to, human, cynomolgus, porcine, canine, and murine and rat. Exemplary amino acid sequences of human, cyno, dog, pig, rat and mouse ASGR-1 and ASGR-2 are provided in Figures 1-3. In some embodiments, the antigen binding proteins further inhibit ASGR, ASGR-1 and/or ASGR-2 from binding to a ligand.

An "antigen binding protein" is a protein comprising an antigen binding fragment that binds to an antigen and, optionally, a scaffold or framework portion that allows the antigen binding fragment to adopt a conformation that promotes binding of the antigen binding protein to the antigen. In the instant application, the antigen is ASGR, ASGR-1 and/or ASGR-2 protein or a fragment thereof. In some embodiments, the antigen binding fragment comprises at least one CDR from an antibody that binds to the antigen, and in some embodiments comprises the heavy chain CDR3 from an antibody that binds to the antigen. In some embodiments, the antigen binding fragment comprises all three CDRs from the heavy chain of an antibody that binds to the antigen or from the light chain of an antibody that binds to the antigen. In still some embodiments, the antigen binding fragment comprises all six CDRs from an antibody that binds to the antigen (three from the heavy chain and three from the light chain). The antigen binding fragment in certain embodiments is an antibody fragment.

Nonlimiting examples of antigen binding proteins include antibodies, antibody fragments (e.g., an antigen binding fragment of an antibody), antibody derivatives, and antibody analogs. Further specific examples include, but are not limited to, a single-chain variable fragment (scFv), a nanobody (e.g. VH domain of camelid heavy chain antibodies; VHH fragment, see Cortez-Retamozo et al., Cancer Research, Vol. 64:2853-57, 2004), a Fab fragment, a Fab' fragment, a F(ab')2 fragment, a Fv fragment, a Fd fragment, and a complementarity determining region (CDR) fragment. These molecules can be derived from any mammalian source, such as human, mouse, rat, rabbit, or pig, dog, or camelid. Antibody fragments may compete for binding of a target antigen with an intact antibody and the fragments may be produced by the modification of intact antibodies (e.g. enzymatic or chemical cleavage) or synthesized de novo using recombinant DNA technologies or peptide synthesis. The antigen binding protein can comprise, for example, an alternative protein scaffold or artificial scaffold with grafted CDRs or CDR derivatives. Such scaffolds include, but are not limited to, antibody-derived scaffolds comprising mutations introduced to, for example, stabilize the three-dimensional structure of the antigen binding protein as well as wholly synthetic scaffolds comprising, for example, a biocompatible polymer. See, for example, Korndorfer et al., 2003, Proteins: Structure, Function, and Bioinformatics, Volume 53, Issue 1:121-129 (2003); Roque et al., Biotechnol. Prog. 20:639-654 (2004). In addition, peptide antibody mimetics ("PAMs") can be used, as well as scaffolds based on antibody mimetics utilizing fibronectin components as a scaffold.

An antigen binding protein can also include a protein comprising one or more antibody fragments incorporated into a single polypeptide chain or into multiple polypeptide chains. For instance, antigen binding proteins can include, but are not limited to, a diabody (see, e.g., EP 404,097; WO 93/11161; and Hollinger et al., Proc. Natl. Acad. Sci. USA, Vol. 90:6444-6448, 1993); an intrabody; a domain antibody (single VL or VH domain or two or more VH domains joined by a peptide linker; see Ward et al., Nature, Vol. 341:544-546, 1989); a maxibody (2 scFvs fused to Fc region, see Fredericks et al., Protein Engineering, Design & Selection, Vol. 17:95-106, 2004 and Powers et al., Journal of Immunological Methods, Vol. 251:123-135, 2001); a triabody; a tetrabody; a minibody (scFv fused to CH3 domain; see Olafsen et al., Protein Eng Des Sel. , Vol.17:315-23, 2004); a peptibody (one or more peptides attached to an Fc region, see WO 00/24782); a linear antibody (a pair of tandem Fd segments (VH-CH1-VH-CH1 ) which, together with complementary light chain polypeptides, form a pair of antigen binding regions, see Zapata et al., Protein Eng., Vol. 8:1057-1062, 1995); a small modular immunopharmaceutical (see U.S. Patent Publication No. 20030133939); and immunoglobulin fusion proteins (e.g. IgG-scFv, IgG-Fab, 2scFv-IgG, 4scFv-IgG, VH-IgG, IgG-VH, and Fab-scFv-Fc).

In certain embodiments, an antigen binding protein can have, for example, the structure of an immunoglobulin. An "immunoglobulin" is a tetrameric molecule, with each tetramer comprising two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function.

Within light and heavy chains, the variable (V) and constant regions (C) are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989)) (incorporated by reference in its entirety for all purposes). The variable regions of each light/heavy chain pair form the antibody binding site such that an intact immunoglobulin has two binding sites.

Immunoglobulin chains exhibit the same general structure of relatively conserved framework regions (FR) joined by three hypervariable regions, also called complementarity determining regions or CDRs. From N-terminus to C-terminus, both light and heavy chains comprise the domains FR1, CDR1, FR2, CDR2, FR3, CDR3 and FR4.

Human light chains are classified as kappa and lambda light chains. The term "light chain" refers to a polypeptide comprising, from amino terminus to carboxyl terminus, a single immunoglobulin light chain variable region (VL) and a single immunoglobulin light chain constant domain (CL). Heavy chains are classified as mu ($\mu$), delta ($\Delta$), gamma ($\gamma$), alpha ($\alpha$), and epsilon ($\varepsilon$), and define the antibody's isotype as IgM, IgD, IgG, IgA, and IgE, respectively. The term "heavy chain" refers to a polypeptide comprising, from amino terminus to carboxyl terminus, a single immunoglobulin heavy chain variable region (VH), an immunoglobulin heavy chain constant domain 1 (CH1), an immunoglobulin hinge region, an immunoglobulin heavy chain constant domain 2 (CH2), an immunoglobulin heavy chain constant domain 3 (CH3), and optionally an immunoglobulin heavy chain constant domain 4 (CH4). The IgG-class is further divided into subclasses, namely, IgG1, IgG2, IgG3, and IgG4. The IgA-class is further divided into subclasses, namely IgA1 and IgA2. The IgM has subclasses including, but not limited to, IgM1 and IgM2. The heavy chains in IgG, IgA, and IgD antibodies have three domains (CH1, CH2, and CH3), whereas the heavy chains in IgM and IgE antibodies have four domains (CH1, CH2, CH3, and CH4). The immunoglobulin heavy chain constant domains can be from any immunoglobulin isotype, including subtypes. The antibody chains are linked together via inter-polypeptide disulfide bonds between the CL domain and the CH1 domain (i.e. between the light and heavy chain) and between the hinge regions of the antibody heavy chains.

The term "antibody" refers to an intact immunoglobulin of any isotype, and includes, for instance, chimeric, humanized, human, and bispecific antibodies. An "antibody" is a species of an antigen binding protein. An intact antibody will generally comprise at least two full-length heavy chains and two full-length light chains. Antibody sequences can be derived solely from a single species, or can be "chimeric," that is, different portions of the antibody can be derived from two different species as described further below. Unless otherwise indicated, the term "antibody" also includes antibodies comprising two substantially full-length heavy chains and two substantially full-length light chains provided the antibodies retain the same or similar binding and/or function as the antibody comprised of two full length light and heavy chains. For example, antibodies having 1, 2, 3, 4, or 5 amino acid residue substitutions, insertions or deletions at the N-terminus and/or C-terminus of the heavy and/ or light chains are included in the definition provided that the antibodies retain the same or similar binding and/or function as the antibodies comprising two full length heavy chains and two full length light chains. Furthermore, unless explicitly excluded, antibodies include, for example, monoclonal antibodies, polyclonal antibodies, chimeric antibodies, humanized antibodies, human antibodies, bispecific antibodies, and synthetic antibodies. In some sections of the present disclosure, examples of antigen binding proteins are described herein in terms of the hybridoma line number as "number/letter/number" (*e.g.,* 25A4). In these cases, the exact name denotes a specific monoclonal antibody derived from a specific hybridoma having a specific light chain variable region and heavy chain variable region. In some sections of the present disclosure, examples of antigen binding proteins are described herein in terms of "number/letter/number/"dot"/number" (*e.g.*, 25A4.001) or number/letter/number/"dot"/number/"dot"/number (e.g., 25A4.001.001). In these cases, the name denotes a variant of a specific antibody having a light chain variable region and a heavy chain variable region that is related to, but distinct from the antibody derived from a hybridoma. That is, for example, an antigen binding protein named 25A4 is not the same as an antibody named 25A4.001 or an antibody named 25A4.001.001.

A "polyclonal antibody" refers to a population of antibodies that are typically widely varied in composition and binding specificity. A "monoclonal antibody" ("mAb") as used herein refers to one or more of a population of antibodies having identical sequences. Monoclonal antibodies bind to the antigen at a particular epitope on the antigen.

In some embodiments, the antigen binding protein is a "fragment" or "antigen binding fragment" of an antibody. As used herein and unless otherwise specified, an "antibody fragment" refers to the Fab, Fab', F(ab')2, and Fv fragments that contain at least one CDR of an immunoglobulin that is sufficient to confer specific antigen binding to ASGR, ASGR-1 and/or ASGR-2. Antibody fragments may be produced by recombinant DNA techniques or by enzymatic or chemical cleavage of intact antibodies.

A Fab fragment is a monovalent fragment having the VL, VH, CL and CH1 domains; a F(ab')2 fragment is a bivalent fragment having two Fab fragments linked by a disulfide bridge at the hinge region; a Fd fragment has the VH and CH1 domains; an Fv fragment has the VL and VH domains of a single arm of an antibody; and a dAb fragment has a VH domain, a VL domain, or an antigen-binding fragment of a VH or VL domain (US Pat. No. 6,846,634, 6,696,245, US App. Pub. No. 05/0202512, 04/0202995, 04/0038291, 04/0009507, 03/0039958, Ward et al., Nature 341:544-546 (1989)). In certain embodiments, these antibody fragments can be incorporated into single domain antibodies, single-chain antibodies, maxibodies, minibodies, intrabodies, diabodies, triabodies, tetrabodies, v-NAR and bis-scFv (see e.g., Hollinger and Hudson, 2005, Nature Biotechnology, 23, 9, 1126-1136). Other antigen binding proteins envisioned are antibody polypeptides such as those disclosed in U. S. Patent No. 6,703,199, including fibronectin polypeptide monobodies, the polypeptides as disclosed in U.S. Patent Publication 2005/0238646. In some embodiments, the antibodies comprise at least one CDR set forth in Tables 2 or 6 herein.

A "single-chain variable fragment" ("scFv") is a fusion protein in which a VL and a VH region are joined via a linker (e.g., a synthetic sequence of amino acid residues) to form a continuous protein chain wherein the linker is long enough to allow the protein chain to fold back on itself and form a monovalent antigen binding site (see, e.g., Bird et al., Science 242:423-26 (1988) and Huston et al., 1988, Proc. Natl. Acad. Sci. USA 85:5879-83 (1988)). For the sake of clarity, a "single-chain variable fragment" is not an antibody or an antibody fragment as defined herein. Diabodies are bivalent antibodies comprising two polypeptide chains, wherein each polypeptide chain comprises VH and VL domains joined by a linker that is too short to allow for pairing between two domains on the same chain, thus allowing each domain to pair with a complementary domain on another polypeptide chain (see, e.g., Holliger et al., 1993, Proc. Natl. Acad. Sci. USA 90:6444-48

(1993), and Poljak et al., Structure 2:1121-23 (1994)). If the two polypeptide chains of a diabody are identical, then a diabody resulting from their pairing will have two identical antigen binding sites. Polypeptide chains having different sequences can be used to make a diabody with two different antigen binding sites. Similarly, tribodies and tetrabodies are antibodies comprising three and four polypeptide chains, respectively, and forming three and four antigen binding sites, respectively, which can be the same or different.

The term "CDR" refers to the complementarity determining region (also termed "minimal recognition units" or "hypervariable region") within antibody variable sequences. The CDRs permit the antigen binding protein to specifically bind to a particular antigen of interest. There are three heavy chain variable region CDRs (CDRH1, CDRH2 and CDRH3) and three light chain variable region CDRs (CDRL1, CDRL2 and CDRL3). The CDRs in each of the two chains typically are aligned by the framework regions to form a structure that binds specifically to a specific epitope or domain on the target protein. From N-terminus to C-terminus, naturally-occurring light and heavy chain variable regions both typically conform to the following order of these elements: FR1, CDR1, FR2, CDR2, FR3, CDR3 and FR4. A numbering system has been devised for assigning numbers to amino acids that occupy positions in each of these domains. This numbering system is defined in Kabat Sequences of Proteins of Immunological Interest (1987 and 1991, NIH, Bethesda, MD), or Chothia & Lesk, 1987, J. Mol. Biol. 196:901-917; Chothia et al., 1989, Nature 342:878-883. Complementarity determining regions (CDRs) and framework regions (FR) of a given antibody may be identified using this system. Other numbering systems for the amino acids in immunoglobulin chains include IMGT® (the international ImMunoGeneTics information system; Lefranc et al, Dev. Comp. Immunol. 29:185-203; 2005) and AHo (Honegger and Pluckthun, J. Mol. Biol. 309(3):657-670; 2001). One or more CDRs may be incorporated into a molecule either covalently or noncovalently to make it an antigen binding protein.

In some embodiments, an antigen binding protein of the invention may incorporate the CDR(s) as part of a larger polypeptide chain, may covalently link the CDR(s) to another polypeptide chain, or may incorporate the CDR(s) noncovalently. The antigen binding molecules may comprise at least one of the CDRs described herein incorporated into a biocompatible framework structure. In one example, the biocompatible framework structure comprises a polypeptide or portion thereof that is sufficient to form a conformationally stable structural support, or framework, or scaffold, which is able to display one or more sequences of amino acids that bind to an antigen (e.g., CDRs, a variable region, etc.) in a localized surface region. Such structures can be a naturally occurring polypeptide or polypeptide "fold" (a structural motif), or can have one or more modifications, such as additions, deletions or substitutions of amino acids, relative to a naturally occurring polypeptide or fold. These scaffolds can be derived from a polypeptide of any species (or of more than one species), such as a human, other mammal, other vertebrate, invertebrate, plant, bacteria or virus.

Typically the biocompatible framework structures are based on protein scaffolds or skeletons other than immunoglobulin domains. For example, those based on fibronectin, ankyrin, lipocalin, neocarzinostain, cytochrome b, CP1 zinc finger, PST1, coiled coil, LACI-D1, Z domain and tendamistat domains may be used (See e.g., Nygren and Uhlen, 1997, Current Opinion in Structural Biology, 7, 463-469).

An antigen binding protein may have one or more binding sites. If there is more than one binding site, the binding sites may be identical to one another or may be different. For example, an antibody typically has two identical binding sites, while a "bispecific" or "bifunctional" antibody has two different binding sites. The two binding sites of a bispecific antigen binding protein or antibody will bind to two different epitopes, which can reside on the same or different protein targets.

In some embodiments, the ASGR-1 antigen binding protein is a bispecific antibody. In certain embodiments, a bispecific antibody binds to ASGR, ASGR-1 or ASGR-2 and PCSK9. In some embodiments, a bispecific antibody will bind to the ASGR-1 CBD and will inhibit ASGR-1 function, in addition to binding to PCSK9 and inhibiting the binding of PCSK9 to the LDLR. Methods of making bispecific antibodies are known in the art. One such method of making a "bispecific," or "bifunctional" antigen binding protein or antibody involves the fusion of hybridomas or linking of Fab' fragments. *See, e.g.,* Songsivilai and Lachmann, 1990, Clin. Exp. Immunol. 79:315-321; Kostelny et al., 1992, J. Immunol. 148:1547-1553. Another method involves engineering the Fc portion of the heavy chains such as to create "knobs" and "holes" which facilitate heterodimer formation of the heavy chains when co-expressed in a cell. U.S. 7,695,963. Still another method also involves engineering the Fc portion of the heavy chain but uses electrostatic steering to encourage heterodimer formation while discouraging homodimer formation of the heavy chains when co-expressed in a cell. WO 09/089,004, which is incorporated herein by reference in its entirety.

The term "human antibody" includes antibodies having antibody regions such as variable and constant regions or domains which correspond substantially to human germline immunoglobulin sequences known in the art, including, for example, those described by Kabat *et al.* (1991) (*loc. cit.*). The human antibodies of the invention may include amino acid residues not encoded by human germline immunoglobulin sequences (*e.g.*, mutations introduced by random or site-specific mutagenesis *in vitro* or by somatic mutation *in vivo*), for example in the CDRs, and in particular, in CDR3. The human antibodies can have at least one, two, three, four, five, or more positions replaced with an amino acid residue that is not encoded by the human germline immunoglobulin sequence. The definition of human antibodies as used herein also contemplates fully human antibodies, which include only non-artificially and/or genetically altered human sequences of antibodies as those can be derived by using technologies or systems known in the art, such as for example, phage display technology or transgenic mouse technology, including but not limited to the Xenomouse.

A humanized antibody has a sequence that differs from the sequence of an antibody derived from a non-human species by one or more amino acid substitutions, deletions, and/or additions, such that the humanized antibody is less likely to induce an immune response, and/or induces a less severe immune response, as compared to the non-human species antibody, when it is administered to a human subject. In one embodiment, certain amino acids in the framework and constant domains of the heavy and/or light chains of the non-human species antibody are mutated to produce the humanized antibody. In another embodiment, the constant domain(s) from a human antibody are fused to the variable domain(s) of a non-human species. In another embodiment, one or more amino acid residues in one or more CDR sequences of a non-human antibody are changed to reduce the likely immunogenicity of the non-human antibody when it is administered to a human subject, wherein the changed amino acid residues either are not critical for immunospecific binding of the antibody to its antigen, or the changes to the amino acid sequence that are made are conservative changes, such that the binding of the humanized antibody to the antigen is not significantly worse than the binding of the non-human antibody to the antigen. Examples of how to make humanized antibodies may be found in U.S. Pat. Nos. 6,054,297, 5,886,152 and 5,877,293.

The term "chimeric antibody" refers to an antibody that contains one or more regions from one antibody and one or more regions from one or more other antibodies. In one embodiment, one or more of the CDRs are derived from a human anti-ASGR, ASGR-1 or ASGR-2 antibody. In another embodiment, all of the CDRs are derived from a human anti-ASGR, ASGR-1 or ASGR-2 antibody. In another embodiment, the CDRs from more than one human anti-ASGR, ASGR-1 or ASGR-2 antibodies are mixed and matched in a chimeric antibody. For instance, a chimeric antibody may comprise a CDR1 from the light chain of a first human anti-ASGR, ASGR-1 or ASGR-2 antibody, a CDR2 and a CDR3 from the light chain of a second human anti-ASGR, ASGR-1 or ASGR-2 antibody, and the CDRs from the heavy chain from a third anti-ASGR, ASGR-1 or ASGR-2 antibody. Further, the framework regions may be derived from one of the same anti-ASGR, ASGR-1 or ASGR-2 antibodies, from one or more different antibodies, such as a human antibody, or from a humanized antibody. In one example of a chimeric antibody, a portion of the heavy and/or light chain is identical with, homologous to, or derived from an antibody from a particular species or belonging to a particular antibody class or subclass, while the remainder of the chain(s) is/are identical with, homologous to, or derived from an antibody or antibodies from another species or belonging to another antibody class or subclass. Also included are fragments of such antibodies that exhibit the desired biological activity.

A "neutralizing antigen binding protein" or "inhibitory antigen binding protein" or "antagonizing antigen binding protein" (e.g., "neutralizing antibody" or "inhibitory antibody" or "antagonizing antibody") refers to an antigen binding protein or antibody, respectively, that binds to a target molecule and reduces and/or prevents the biological effect of that target molecule. This can be done, for example, by directly blocking a site on the target molecule through which the target molecule interacts with other molecules (e.g. blocking a ligand binding site of a receptor) or by indirectly blocking a site on the target molecule through which the target molecule interacts with other molecules (such as structural or energetic alterations in the target molecule). In some embodiments, these terms can also denote an antigen binding protein or antibody that prevents the target molecule to which it is bound from performing a biological function. In assessing the binding and/or specificity of an antigen binding protein, *e.g.*, an antibody or immunologically functional fragment thereof, an antibody or fragment can substantially inhibit binding of a target molecule to its binding partner when an excess of antibody reduces the quantity of binding partner bound to the target molecule by at least about 1-20, 20-30%, 30-40%, 40-50%, 50-60%, 60-70%, 70-80%, 80-85%, 85-90%, 90-95%, 95-97%, 97-98%, 98-99%, 99.5%, 99.9% and 100%. In some embodiments, inhibition is complete. The measurement of reduction of binding is done using various assays known to those skilled in the art, (e.g., an *in vitro* competitive binding assay) and performed using relevant control molecules so that actual inhibition is measured. For example, numerous competition assays are well known in the art, with nonlimiting examples being competition ELISA, use of the BiaCore® platform, the Kinexa® platform, or the like. Further examples include: solid phase direct or indirect radioimmunoassay (MA), solid phase direct or indirect enzyme immunoassay (EIA), sandwich competition assay (see, e.g., Stahli et al., 1983, Methods in Enzymology 9:242-253); solid phase direct biotin-avidin EIA (see, e.g., Kirkland et al., 1986, J. Immunol. 137:3614-3619) solid phase direct labeled assay, solid phase direct labeled sandwich assay (see, e.g., Harlow and Lane, 1988, Antibodies, A Laboratory Manual, Cold Spring Harbor Press); solid phase direct label RIA using 1-125 label (see, e.g., Morel et al., 1988, Molec. Immunol. 25:7-15); solid phase direct biotin-avidin EIA (see, e.g., Cheung, et al., 1990, Virology 176:546-552); and direct labeled RIA (Moldenhauer et al., 1990, Scand. J. Immunol. 32:7-82). Typically, such an assay involves the use of purified antigen bound to a solid surface or cells bearing either of these, an unlabelled test antigen binding protein and a labeled reference antigen binding protein. In some embodiments, in the case of ASGR, ASGR-1 and/or ASGR-2, such a neutralizing antigen binding protein or antibody can diminish the ability of ASGR, ASGR-1 and/or ASGR-2 to bind to a ligand. In some embodiments, the neutralizing ability is characterized and/or described via a competition assay. In some embodiments, the neutralizing ability is described in terms of an $IC_{50}$ or $EC_{50}$ value. The antigen binding proteins in at least Table C are strong neutralizers. In some embodiments, the antibodies or antigen binding proteins neutralize by binding to ASGR, ASGR-1 and/or ASGR-2 and preventing ASGR, ASGR-1 and/or ASGR-2 from binding to a ligand, including sugars such as lactose, galactose, and/or GalNAc or glycoproteins displaying such sugars, such as fetuin, orosomucoid and/or alkaline phosphatase (or reducing the ability of ASGR, ASGR-1 and/or ASGR-2 to bind to ligand).

Competitive inhibition can be measured by determining the amount of labelled ligand bound to the solid surface or cells in the presence of the test antigen binding protein. Usually the test antigen binding protein is present in excess. Antigen binding proteins or antibodies identified by competition assay (competing antigen binding proteins or antibodies) include antigen binding proteins binding to the same epitope as the reference antigen binding proteins and antigen binding proteins binding to an adjacent epitope sufficiently proximal to the epitope bound by the reference antigen binding protein for steric hindrance to occur. Usually, when a competing antigen binding protein is present in excess, it will inhibit (e.g., reduce) specific binding of a reference antigen binding protein to a target antigen by at least 40-45%, 45-50%, 50-55%, 55-60%, 60-65%, 65-70%, 70-75% or 75% or more. In some embodiments, binding is inhibited by at least 40%, 45%, 50%, 55%, 60%, 65%, 70%, 75%, 80%, 85%, 90%, or 95%. In some embodiments, binding is inhibited by at least 80-85%, 85-90%, 90-95%, 95-97%, or 97% or more, including up to 100% inhibition.

In some embodiments, a ligand binding assay is used where cells expressing the target protein (e.g., ASGR-1) are mixed with antigen binding proteins and incubated for a time period, then washed. These cells are then incubated with labelled ligand (e.g., β-GalNAc) for a time period and then washed and analyzed for ligand binding, where reduced ligand binding as compared to a relevant control antigen binding protein indicates inhibition of binding due to the antigen binding protein blocking or inhibiting this binding.

Another manner in which the reduction in binding can be measured is the half maximal inhibitory concentration (IC50). The IC50 measures the amount or concentration of antigen binding protein that is needed to inhibit a given attribute (e.g., ligand binding) by half. In certain embodiments, the antigen binding proteins (e.g., human antibodies) have an IC50 value of 90 nM or less, in another embodiment, an IC50 value of 80 nM or less, in another embodiment, 70 nM or less, in another embodiment, 60 nM or less, in another embodiment, 50 nM or less, in another embodiment, 40 nM or less, in another embodiment, 30 nM or less, in another embodiment 25 nM or less.

In certain embodiments, the antigen binding proteins of the invention bind to an ASGR-1 monomer. In some embodiments, the antigen binding proteins of the invention bind to an ASGR-1 oligomer. In further embodiments, the antigen binding proteins of the invention bind to an ASGR-2 monomer. In some embodiments, the antigen binding proteins of the invention bind to an ASGR-2 oligomer. In certain embodiments, the antigen binding proteins of the invention bind to both ASGR-1 monomers and ASGR-2 monomers. In certain embodiments, the antigen binding proteins of the invention bind to an ASGR oligomer comprising an $(ASGR-1)_2$-$(ASGR-2)_1$ trimer. In some embodiments, the antigen binding proteins of the invention bind to an ASGR oligomer comprising an $(ASGR-1)_2$ dimer. In further embodiments, the antigen binding proteins of the invention bind to an ASGR oligomer comprising an $(ASGR-1)_3$ trimer. In yet further embodiments, the antigen binding proteins of the invention bind to an ASGR oligomer comprising an $(ASGR-1)_2$-$(ASGR-2)_2$ tetramer. In further embodiments, the antigen binding proteins of the invention bind to an ASGR oligomer comprising an $(ASGR-1)_3$-$(ASGR-2)_2$ pentamer. In some embodiments, the antigen binding proteins of the invention bind to a multimeric complex comprising at least two subunits of ASGR-1 and/or ASGR-2.

In certain embodiments, the antigen binding proteins (e.g., antibodies, antibody fragments, etc.) bind to ASGR, ASGR-1 and /or ASGR-2 and inhibit ASGR, ASGR-1 and/or ASGR-2 from binding to a ligand, wherein the antigen binding proteins comprise specific amino acid residues at particular positons in the molecule (e.g., in the VH, VL or CDRs). These residues may be involved in the binding properties of desired molecules (e.g., part of the paratope). A "paratope" are used herein is the location in an antibody that binds to the antigen. The paratope can comprise several amino acid residues from the VH and/or VL CDRs, and also can comprise residues from the framework regions. The paratope binds to the antigen's epitope. Paratopes can be determined using methodologies similar to those described determining epitopes. Once the amino acid residues involved in the binding properties of desired molecules, are identified, this information can be used to design antigen binding proteins (e.g., antibodies, antibody fragments, etc.) that can bind to ASGR, ASGR-1 and/or ASGR-2 and inhibit ASGR function (e.g., inhibit ASGR, ASGR-1 and/or ASGR-2 from binding to ligand).

The binding site (or interface) between the representative antibodies and human ASGR-1 can be determined/defined a number of ways. For example, binding of representative antigen binding proteins (e.g., antibodies) to human ASGR-1 was analyzed in Example 10 using X-ray crystallography, and the binding site or interface was determined using distance. The crystal structure of the antibody/huASGR1 complex provides information as to which residues of representative antibodies form the interface with human ASGR-1. As mentioned above, one of ordinary skill in the art may use this information to design antigen binding proteins and antigen binding protein variants, including those that contain variable domains having 90% identity or greater, 95% identity or greater, 97% identity or greater, 99% identity or greater, or those antigen binding protein variants that contain variable domains having 20 or less, 15 or less, or 10 or less, or 5 or less insertions, deletions, and/or substitutions within the light chain and/or heavy chain variable domain of the antigen binding proteins disclosed herein. One may wish to maintain the amino acids within the interface while altering non-interface residues. Thus, in some embodiments, one may design and create antigen binding proteins and antigen binding protein variants of the antigen binding proteins disclosed herein having one or more amino acid additions, substitutions, and/or deletions within one or more CDRs that maintain binding to human ASGR-1 and inhibit ASGR, ASGR-1 and/or ASGR-2 function (e.g., inhibit ASGR, ASGR-1 and or ASGR-2 from binding to ligand).

In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, 12, 13 or all amino acid residues selected from the group consisting of Q27, R30, D32, H91, Y92, S93, Y94, I2, G28, I29, L33, Q90, P95, and R96 of SEQ ID NO:25010 and/or the heavy chain variable region comprises at least 1, 2, 3, 4, 5, 6, 7, 8, 9, 10, 11, 12, 13, 14, 15, 16, 17, 18, 19, 20, 21, 22, 23 or all amino acid residues selected from the group consisting of S30, N31, W52, Y53, D54, S56, N57, Y59, Y101, S102, S103, G104, W105, Y106, D107, Y32, V33, V50, G55, K58, N74, E99, V100, and Y108 of SEQ ID NO:29016. In some embodiments, the light chain variable region comprises at least 1, 2, 3, 4, 5, 6 or all amino acid residues selected from the group consisting of Q27, R30, D32, H91, Y92, S93, andY94 of SEQ ID NO:25010 and/or the heavy chain variable region comprises at least 1, 2, 3, 4, 5, 6, 7, 8, 9, 10, 11, 12, 13, 14, or all amino acid residues selected from the group consisting of S30, N31, W52, Y53, D54, S56, N57, Y59, Y101, S102, S103, G104, W105, Y106, and D107 of SEQ ID NO:29016. In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, 12, 13, 14, 15 or all amino acid residues selected from the group consisting of H31, S33, N34, N36, Y38, W56, Y97, Y98, I29, S32, N35, N37, Y55, T59, Q96, N99, T100 of SEQ ID NO:25164 and/or the heavy chain variable region comprises at least 1, 2, 3, 4, 5, 6, 7, 8, 9, 10, 11, 12, 13, 14, 15, 16, 17, 18, 19, 20, 21, 22, 23, 24, 25, 26 or all amino acid residues selected from the group consisting of T28, F29, T30, N31, Y32, D33, W50, H52, S55, N57, S99, S100, G101, W102, Y103, Y27, I34, N35, W47, M51, P53, N54, G56, T58, G59, Y104, D106 of SEQ ID NO:29170. In some embodiments, the light chain variable region comprises at least 1, 2, 3, 4, 5, 6, 7 or all amino acid residues selected from the group consisting H31, S33, N34, N36, Y38, W56, Y97, Y98 of SEQ ID NO:25164 and/or the heavy chain variable region comprises at least 1, 2, 3, 4, 5, 6, 7, 8, 9, 10, 11, 12, 13, 14, or all amino acid residues selected from the group consisting of T28, F29, T30, N31, Y32, D33, W50, H52, S55, N57, S99, S100, G101, W102, Y103 of SEQ ID NO:29170. In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, 12, 13, 14, 15, 16, 17, 18 or all amino acid residues selected from the group consisting of I30, Y32, T91, Y92, S93, T94, I96, I2, Q27, N28, I29, S31, L33, N34, T50, S67, Q89, Q90, P95 of SEQ ID NO:24908 and/or the heavy chain variable region comprises at least 1, 2, 3, 4, 5, 6, 7, 8, 9, 10, 11, 12, 13, 14, 15, 16, 17, 18, 19, 20, 21, 22 or all amino acid residues selected from the group consisting of S30, S31, I50, W52, H53, S56, N57, Y59, S01, M102, G103, T28, F29, F32, G33, H35, W47, I51, D54, K58, D99, L100, G104 of SEQ ID NO:28914. In some embodiments, the light chain variable region comprises at least 1, 2, 3, 4, 5, 6 or all amino acid residues selected from the group consisting I30, Y32, T91, Y92, S93, T94, I96 of SEQ ID NO:24908 and/or the heavy chain variable region comprises at least 1, 2, 3, 4, 5, 6, 7, 8, 9, 10 or all amino acid residues selected from the group consisting of S30, S31, I50, W52, H53, S56, N57, Y59, S01, M102, G103 of SEQ ID NO:28914. In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, 12, 13, 14, 15 or all amino acid residues selected from the group consisting of Y32, S91, Y92, R93, Thr94, Pro95, F97, Ile2, Q27, N28, NAG100, Ile29, S30, S31, Q90, and L96 of SEQ ID NO:24362 and/or the heavy chain variable region comprises at least 1, 2, 3, 4, 5, 6, 7, 8, 9, 10, 11, 12, 13, 14, 15, 16, 17, 18, 19, 20, 21, 22, 23, 24, 25, 26, 27 or all amino acid residues selected from the group consisting of A33, Val50, Ile51, S52, R53, S54, G55, G56, Y57, Y59, R99, A101, A103, G104, E106, S30, S31, Y32, Met34,N35, W47, S49, Thr58, R72, N74, L100, Val102, and S105 of SEQ ID NO:28368. In some embodiments, the light chain variable region comprises at least 1, 2, 3, 4, 5, 6, or all amino acid residues selected from the group consisting of Y32, S91, Y92, R93, Thr94, Pro95, and F97 of SEQ ID NO:24362, and/or the heavy chain variable region comprises at least 1, 2, 3, 4, 5, 6, 7, 8, 9, 10, 11, 12, 13, 14, or all amino acid residues selected from the group consisting of A33, Val50, Ile51, S52, R53, S54, G55, G56, Y57, Y59, R99, A101, A103, G104, and E106 of SEQ ID NO:28368. In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, 12, 13, 14, 15, 16, 17 or all amino acid residues selected from the group consisting of Q27, W32, A91, N92, S93, F94, F96, D1, I2, G28, I29, S30, R31, Y49, G50, Q89, Q90, and P95 of SEQ ID NO:24930 and/or the heavy chain variable region comprises at least 1, 2, 3, 4, 5, 6, 7, 8, 9, 10, 11, 12, 13, 14, 15, 16, 17, 18, 19, 20, 21, 22 or all amino acid residues selected from the group consisting of Y33, H35, W50, H52, S55, G57, T58, N59, D99, G100, T101, S102, D31, Y32, L34, W47, I51, N54, G56, Y60, Q65, S103, and F104 of SEQ ID NO:28936. In some embodiments, the light chain variable region comprises at least 1, 2, 3, 4, 5, 6 or all amino acid residues selected from the group consisting of Q27, W32, A91, N92, S93, F94, and F96 of SEQ ID NO:24930 and/or the heavy chain variable region comprises at least 1, 2, 3, 4, 5, 6, 7, 8, 9, 10, 11 or all amino acid residues selected from the group consisting of Y33, H35, W50, H52, S55, G57, T58, N59, D99, G100, T101, and S102 of SEQ ID NO:28936. In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, 12, 13, 14, 15, 16, 17, 18, 19 or all amino acid residue selected from the group consisting of Y32, Y49, T50, Q55, S91, H92, S93, F94, F96, S28, I29, T30, N33, L46, S53, L54, S56, Q89, Q90, and P95 of SEQ ID NO:28074 and/or the heavy chain variable region comprises at least 1, 2, 3, 4, 5, 6, 7, 8, 9, 10, 11, 12, 13, 14, 15, 16, 17, 18, 19, 20, 21, 22, 23, 24, 25, 26, 27, 28 or all amino acid residues selected from the group consisting of G26, F27, T28, S30, S31, Y32, S33, S52, G53, S54, S56, Y57, Y59, R98, G100, S101, R102, V2, F29, N35, S50, T51, S55, I58, R72, G99, G103, F104 and D105 of SEQ ID NO:32080. In some embodiments, the light chain variable region comprises at least 1, 2, 3, 4, 5, 6, 7, 8 or all amino acid residues selected from the group consisting of Y32, Y49, T50, Q55, S91, H92, S93, F94, and F96 of SEQ ID NO:28074 and/or heavy chain variable region comprises at least 1, 2, 3, 4, 5, 6, 7, 8, 9, 10, 11, 12, 13, 14, 15, 16 or all amino acid residues selected from the group consisting of G26, F27, T28, S30, S31, Y32, S33, S52, G53, S54, S56, Y57, Y59, R98, G100, S101 and R102 of SEQ ID NO:32080. In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, 12, 13, 14, 15, 16, 17, 18, 19, 20, 21 or all amino acid residues selected from the group consisting of V29, S30, I32, Y33, L47, Y50, R55, A56, T57, Y94, G28, N31, L48, I49, G51, N54, G58, I59, S68, G69, D93, and S95 of SEQ ID NO:26814 and/or the heavy chain variable region comprises at least 1, 2, 3, 4, 5, 6, 7, 8, 9, 10, 11, 12, 13, 14, 15, 16, 17, 18, 19, 20, 21, 22, 23, 24, 25 or all amino acid residues selected from the group consisting of V31, Y32, Y33, W50, N52, S55, G57, R98, G99, Y100, D101, I102, T204, V2, Y27, T30, L34, N35, P53, N54, G56, T58, N59, A97, L103, and G105 of SEQ ID NO:30820. In some embodiments, the light chain variable region comprises at least 1, 2, 3, 4, 5, 6, 7, 8, 9, or all amino acid residues selected from the group consisting of V29, S30, I32, Y33, L47, Y50, R55, A56, T57, and Y94 of SEQ ID NO:26814 and/or heavy chain variable region comprises at least 1, 2, 3, 4, 5, 6, 7, 8, 9, 10, 11, 12 or all amino acid residues selected from the group consisting of V31, Y32, Y33, W50, N52, S55, G57, R98, G99, Y100, D101, I102, and T204 of SEQ ID NO:30820. In some embodiments, the antigen binding protein or the antibody comprises a light chain variable region and/or a heavy chain variable region, wherein the light chain variable region comprises at least 1, 2, 3 or all amino acid residues selected from the group consisting of N31, Y50, V51, Q54 SEQ ID NO:27482; and/or the heavy chain variable region comprises at least 1, 2, 3, 4, 5, 6, 7, 8, 9, 10, 11, 12, 13, 14, 15, 16, 17, 18, 19, 20, 21, 22, 23, 24, 25, 26, 27 or all amino acid residues selected from the group consisting of N30, S31, Y32, S52, Y54, N55, K59, R98, D100, F101, W102, S103, G104, Y105, K107, D110, V2, Y27, T28, F29, G33, W50, A53, G56, N57, H99, Y106, or G108 of SEQ ID NO:31488. In some embodiments, the antigen binding protein or the antibody comprises a light chain variable region and/or a heavy chain variable region, wherein the heavy chain variable region comprises at least 1, 2, 3, 4, 5, 6, 7, 8, 9, 10, 11, 12, 13, 14, 15, or all amino acid residues selected from the group consisting ofN30, S31, Y32, S52, Y54, N55, K59, R98, D100, F101, W102, S103, G104, Y105, K107, and D110 of SEQ ID NO:31488. In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, or all amino acid residues selected from the group consisting of Y33, Y50, D51, N53, K54, S57, V34, S52, R55, P56, G58, and G65 of SEQ ID NO:27780 and/or the heavy chain variable region comprises at least 1, 2, 3, 4, 5, 6, 7, 8, 9, 10, 11, 12, 13, 14, 15, 16, 17, 18, 19, 20, 21, 22, 23, 24, 25 or all amino acid residues selected from the group consisting of Q1, V2, F27, S30, S31, Y32, Y53, D54, W99, Y100, Y101, Y102, G26, T28, F29, G33, W52, G55, R72, N74, N98, Y103, Y104, D107, and V108 of SEQ ID NO:31786. In some embodiments, the light chain variable region comprises at least 1, 2, 3, 4, 5 or all amino acid residues selected from the group consisting of Y33, Y50, D51, N53, K54 and S57 of SEQ ID NO:27780 and/or heavy chain variable region comprises at least 1, 2, 3, 4, 5, 6, 7, 8, 9, 10, 11 or all amino acid residues selected from the group consisting of Q1, V2, F27, S30, S31, Y32, Y53, D54, W99, Y100, Y101, and Y102 of SEQ ID NO:31786. In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, 12, 13, 14, 15, 16, 17, 18, 19 or all amino acid residues selected from the group consisting of H31, G32, D33, G34, K35, Y37, I97, Q98, I99, I2, Q27, S28, L29, L30, T36, E55, Q95, S96, P100, and W101 of SEQ ID NO:26536 and/or the heavy chain variable region comprises at least 1, 2, 3, 4, 5, 6, 7, 8, 9, 10, 11, 12, 13, 14, 15, 16, 17, 18, 19, 20, 21, 22, 23 or all amino acid residues selected from the group consisting of S31, W52, Y53, D54, Y57, Y59, D102, F103, W104, T28, S30, Y32, G33, W47, I50, I51, S56, K58, Y60, K65, D99, H101, S105, and G106 of SEQ ID NO:30542. In some embodiments, the light chain variable region comprises at least 1, 2, 3, 4, 5, 6, 7, 8 or all amino acid residues selected from the group consisting of H31, G32, D33, G34, K35, Y37, 197, Q98, and I99 of SEQ ID NO:26536 and/or heavy chain variable region comprises at least 1, 2, 3, 4, 5, 6, 7, 8 or all amino acid residues selected from the group consisting of S31, W52, Y53, D54, Y57, Y59, D102, F103 and W104 of SEQ ID NO:30542. In some embodiments, the antigen binding protein or the antibody comprises a light chain variable region and/or a heavy chain variable region, wherein the light chain variable region comprises at least 1, 2, 3, 4, 5, 6, 7, 8, 9, 10, 11, 12, 13, 14, 15, 16, 17, 18, 19 or all amino acid residues selected from the group consisting of N30, S31, Y33, F50, S54, S68, Y92, E93, W97, S28, V29, G32, L47, G51, A52, S53, R55, A56, G69, Q90, Q91, S94, and S95 of SEQ ID NO:26826 and/or the heavy chain variable region comprises at least 1, 2, 3, 4, 5, 6, 7, 8, 9, 10, 11, 12, 13, 14, 15, 16, 17, 18, 19, 20, 21, 22, 23, 24 or all amino acid residues selected from the group consisting of R30, Y31, Y33, E50, S54, S56, N58, D98, Y99, G100, S28, Y32, W34, S35, W47, G49, I51, S52, H53, G55, T57, R97, A101, F102 and D103 of SEQ ID NO:30832. In some embodiments, the light chain variable region comprises at least 1, 2, 3, 4, 5, 6, 7, 8 or all amino acid residues selected from the group consisting of N30, S31, Y33, F50, S54, S68, Y92, E93, and W97 of SEQ ID NO:26826 and/or heavy chain variable region comprises at least 1, 2, 3, 4, 5, 6, 7, 8, 9 or all amino acid residues selected from the group consisting of R30, Y31, Y33, E50, S54, S56, N58, D98, Y99 and G100 of SEQ ID NO:30832.

In further embodiments, consensus sequences among the antigen binding proteins of the inventions are envisioned. For example, the variable heavy chain and variable light chain regions (VH and VL) and the CDRs (HCDR1/2/3 and LCDR1/2/3) of the invention include consensus sequences derived from groups of related monoclonal antibodies. In some embodiments, the antigen binding proteins (e.g., antibodies) may be related by both sequence homology and function. As described herein, a "consensus sequence" refers to amino acid sequences having conserved amino acids common among a number of sequences and amino acids that vary within given amino acid sequences at certain positions. In some embodiments, the varied amino acid at a certain position is a substitution. In some embodiments, the varied amino acid at a certain position is a deletion. In some embodiments, the varied amino acid at a certain position is an addition or insertion. These varied amino acids will be apparent to one of skill in the art when analyzing particular antibody VH, VL and/or CDR sequences.

For example, antibody sequences were analyzed using the following methodology. The Smith-Waterman algorithm was used to align amino acid sequences against translated IMGT germline V, D and J genes. The V gene was located first, then the J gene was located in the region downstream from located V gene, and finally the D gene was located in the region between V and J regions. Note, that since D gene is a relatively short sequence that is located in the hypervariable CDR3 region, a spurious match is possible and as such, was taken into consideration.

Sequences from each group were then subjected to sequence similarity alignment interrogation using a program that employs a standard ClustalW algorithm (see, Thompson et al., 1994, Nucleic Acids Res. 22:4673-4680). In some cases, the Biosum cost matrix was used with a gap creation penalty of 50 was employed along with a gap extension penalty of 0.1. The sequence logos were generated by Geneious (v8.1.7, Biomatters) once the alignments were made and then exported as PDF images. The consensus sequences were generated in Geneious (v8.1.7, Biomatters) with a 0% threshold and exported as FASTA files. Amino acids that varied within each group were noted with the notation X within each consensus sequence. See Table 19A VH Consensus 1-14 and Table 20A VL Consensus 1-14 in Figure 55, and Tables 21-48 in Figure 56 for the consensus sequences resulting from this analysis. In other cases, the consensus sequences were generated in Abinitio. See Table 19A VH Consensus-15-60 and Table 20A VL Consensus 15-54 in Figure 55, and Tables 49-134 in Figure 57 for the consensus sequences resulting from this analysis.

Alternatively, different methods of analysis readily available to one of skill in the art can be used. For example, consensus sequences can be determined using standard phylogenic analyses of the CDRs corresponding to the VH (i.e., Variable Heavy, etc.) & VL (i.e., Variable Light, etc.) of antibodies. For example, amino acid sequences corresponding to the entire variable domains of either VH or VL can be converted to FASTA formatting for ease in processing comparative alignments and inferring phylogenies. Next, framework regions of these sequences can be replaced with an artificial linker sequence so that examination of the CDRs alone can be performed without introducing any amino acid position weighting bias due to coincident events (e.g., such as unrelated antibodies that serendipitously share a common germline framework heritage) while still keeping CDRs contiguous within the same sequence corresponding to a VH or VL. VH or VL sequences of this format can then be subjected to sequence similarity alignment interrogation using a program that employs a standard ClustalW-like algorithm (see, Thompson et al., 1994, Nucleic Acids Res. 22:4673-4680). A gap creation penalty of 8.0 can be employed along with a gap extension penalty of 2.0. This program likewise generated phylograms (phylogenic tree illustrations) based on sequence similarity alignments using either UPGMA (unweighted pair group method using arithmetic averages) or Neighbor-Joining methods (see, Saitou and Nei, 1987, Molecular Biology and Evolution 4:406-425) to construct & illustrate similarity and distinction of sequence groups via branch length comparison and grouping. The original sequence alignments generated can be employed to empirically examine and document the occurrence of amino acids tolerated at each position with a consensus group. Consensus sequences for the groups of similar sequences within each CDR can then be prepared.

In another type of approach, CDR consensus sequences can be determined for each separate CDR, independently of their contiguous context within the same sequence corresponding to a VH or VL. In this approach the consensus sequences can be determined by aligning each H-CDR1, H-CDR2, H-CDR3, L-CDR1, L-CDR2, and L-CDR3 in groups, i.e., by aligning the individual H-CDR1 sequences of the antigen binding proteins to determine a H-CDR1 consensus sequence, by aligning the individual H-CDR2 sequences of the antigen binding proteins to determine a H-CDR2 consensus sequence, by aligning the individual H-CDR3 sequences of the antigen binding proteins to determine a H-CDR3 consensus sequence, by aligning the individual L-CDR1 sequences of the antigen binding proteins to determine a L-CDR1 consensus sequence, by aligning the individual L-CDR2 sequences of the antigen binding proteins to determine a L-CDR2 consensus sequence, and by aligning the individual L-CDR3 sequences of the antigen binding proteins to determine a L-CDR3 consensus sequence. Similarities between sequences within each individual CDR sequences can be identified. Consensus sequences for the groups of similar sequences within each CDR can then be prepared.

Various emobodiments of Variable Heavy chain (VH) Consensus amino acid sequences of the present invention are set forth in Table 19A of Figure 55 (CDRs are underlined, with the first being CDR1). Various embodiments of VH CDR Consensus amino acid sequences of the present invention are set forth in Tables 19B and 19C of Figure 55. In some cases, an "X" is present in the amino acid sequences set forth in Tables 19A and 19B which signifies that more than one amino acid (or no amino acid) may be present at this location (see Figures 56 and 57 for details of the consensus protein alignment). In some cases a "-" is present in Table 19A (which is the result of the consensus alignment) and signifies that no amino acid is present at the location (see Figures 56 and 57 for details of the consensus protein alignment). The VH Consensus sequences and the VH CDR Consensus sequences are based on analysis of 8 or more aligned VH/VH CDR antibody sequences, as described above. In some cases, the VH/VH CDR Consensus sequence is based on analysis of 25 or more, 50 or more, 75 or more, or 100 or more aligned VH antibody sequences. In one case, the VH/VH CDR Consensus sequence is based on analysis of 149 aligned VH antibody sequences.

Various emobodiments of Variable Light chain (VL) Consensus amino acid sequences of the present invention are set forth in Table 20A of Figure 55 (CDRs are underlined, with the first being CDR1). Various embodiments of VL CDR Consensus amino acid sequences of the present invention are set forth in Tables 20B and 20C of Figure 55. As mentioned above, in some cases, an "X" is present in the amino acid sequences set forth in Tables 20A and 20B which signifies that more than one amino acid (or no amino acid) may be present at this location (see Figures 56 and 57 for details of the consensus protein alignment). In some cases a "-" is present in Table 20A (which is the result of the consensus alignment) and signifies that no amino acid is present at the location (see Figures 56 and 57 for details of the consensus protein alignment). The VL Consensus sequences and the VL CDR Consensus sequences are based on analysis of 8 or more aligned VL/VL CDR antibody sequences, as described above. In some cases, the VL/VL CDR Consensus sequence is based on analysis of 25 or more, 50 or more, 75 or more, or 100 or more, 125 or more, or 150 or more aligned VL antibody sequences. In one case, the VL/VL CDR Consensus sequence is based on analysis of 209 aligned VL antibody sequences.

As discussed above, the consensus sequences in certain embodiments can comprise substitutions, deletions, or additions/insertions at different positions in the sequence. Specific examples of these substitutions, deletions, or additions/insertions can be found in Tables 19C and 20C of Figure 55, as well as Tables 21-48 of Figure 56 and Tables 49-134 of Figure 57, all of which are included herein. However, in no way should the amino acid substitutions, deletions, or additions/insertions exemplified in Tables 19A-C and 20A-C in Figure 55 or in Tables 21-48 in Figure 56 or in Tables 49-134 in Figure 57 be construed to limit the invention to only those amino acid substitutions, deletions, or additions at any position in the identified consensus sequences (VH, VL and/or CDRs) with any amino acid is contemplated herein.

In certain embodiments, the antigen binding proteins of the invention comprise 3 VH CDRs and 3 VL CDRs, wherein at least one VH CDR is a VH1 CDR selected from Table 19B or Table 19C as depicted in Figure 55. In certain embodiments, the antigen binding proteins of the invention comprise 3 VH CDRs and 3 VL CDRs, wherein at least one VH CDR is a VH2 CDR selected from Table 19B or Table 19C as depicted in Figure 55. In certain embodiments, the antigen binding proteins of the invention comprise 3 VH CDRs and 3 VL CDRs, wherein at least one VH CDR is a VH3 CDR selected from Table 19B or Table 19C as depicted in Figure 55. In certain embodiments, the antigen binding proteins of the invention comprise 3 VH CDRs and 3 VL CDRs, wherein the VH1 CDR, the VH2 CDR and the VH3 CDR is selected from Table 19B or Table 19C as depicted in Figure 55.

In certain embodiments, the antigen binding proteins of the invention comprise 3 VH CDRs and 3 VL CDRs, wherein at least one VL CDR is a VL1 CDR selected from Table 20B or Table 20C as depicted in Figure 55. In certain embodiments, the antigen binding proteins of the invention comprise 3 VH CDRs and 3 VL CDRs, wherein at least one VL CDR is a VL2 CDR selected from Table 20B or Table 20C as depicted in Figure 55. In certain embodiments, the antigen binding proteins of the invention comprise 3 VH CDRs and 3 VL CDRs, wherein at least one VL CDR is a VL3 CDR selected from Table 20B or Table 20C as depicted in Figure 55. In certain embodiments, the antigen binding proteins of the invention comprise 3 VH CDRs and 3 VL CDRs, wherein the VL1 CDR, the VL2 CDR and the VL3 CDR is selected from Table 20B or Table 20C as depicted in Figure 55.

In some embodiments, antigen binding proteins comprise no more than one, two, three, four, five, or six amino acid additions, deletions or substitutions of a VH. In some embodiments, antigen binding proteins comprise no more than one, two, three, four, five, or six amino acid additions, deletions or substitutions of a VL. In further embodiments, antigen binding proteins comprise no more than one, two, three, four, five, or six amino acid additions, deletions or substitutions within a VH1 CDR. In further embodiments, antigen binding proteins comprise no more than one, two, three, four, five, or six amino acid additions, deletions or substitutions within a VH2 CDR. In further embodiments, antigen binding proteins comprise no more than one, two, three, four, five, or six amino acid additions, deletions or substitutions within a VH3 CDR. In further embodiments, antigen binding proteins comprise no more than one, two, three, four, five, or six amino acid additions, deletions or substitutions within a VL1 CDR. In further embodiments, antigen binding proteins comprise no more than one, two, three, four, five, or six amino acid additions, deletions or substitutions within a VL2 CDR. In further embodiments, antigen binding proteins comprise no more than one, two, three, four, five, or six amino acid additions, deletions or substitutions within a VL3 CDR.

In some embodiments, antigen binding proteins comprise no more than one, two, three, four, five, or six amino acid additions, deletions or substitutions of a VH consensus sequence. In some embodiments, antigen binding proteins comprise no more than one, two, three, four, five, or six amino acid additions, deletions or substitutions of a VL consensus sequence. In further embodiments, antigen binding proteins comprise no more than one, two, three, four, five, or six amino acid additions, deletions or substitutions within a VH1 CDR Consensus sequence. In further embodiments, antigen binding proteins comprise no more than one, two, three, four, five, or six amino acid additions, deletions or substitutions within a VH2 CDR Consensus sequence. In further embodiments, antigen binding proteins comprise no more than one, two, three, four, five, or six amino acid additions, deletions or substitutions within a VH3 CDR Consensus sequence. In further embodiments, antigen binding proteins comprise no more than one, two, three, four, five, or six amino acid additions, deletions or substitutions within a VL1 CDR Consensus sequence. In further embodiments, antigen binding proteins comprise no more than one, two, three, four, five, or six amino acid additions, deletions or substitutions within a VL2 CDR Consensus sequence. In further embodiments, antigen binding proteins comprise no more than one, two, three, four, five, or six amino acid additions, deletions or substitutions within a VL3 CDR Consensus sequence.

In some embodiments, framework consensus sequences are encompassed by the present invention. Examples of these framework consensus sequences and additions, deletions or substitutions are shown in Tables 21-48 in Figure 56 and Tables 49-134 in Figure 57 herein.

In a further embodiment, the antigen binding proteins of the invention bind to ASGR of different species, including, but not limited to, human, cynomolgus, porcine, canine, murine and rat. In some embodiments, the antigen binding proteins of the invention bind to human. In some embodiments, the antigen binding proteins of the invention bind to cynomolgus ASGR. In some embodiments, the antigen binding proteins of the invention bind to porcine ASGR. In some embodiments, the antigen binding proteins of the invention bind to canine ASGR. In some embodiments, the antigen binding proteins of the invention bind to murine ASGR. In some embodiments, the antigen binding proteins of the invention bind to rat ASGR. In some embodiments, the antigen binding proteins specifically bind to ASGR of the different species.

In some embodiments, the antigen binding proteins of the invention bind to ASGR-1 of different species, including, but not limited to, human, cynomolgus, porcine, canine, murine and rat. In some embodiments, the antigen binding proteins of the invention bind to human ASGR-1. In some embodiments, the antigen binding proteins of the invention bind to cynomolgus ASGR-1. In some embodiments, the antigen binding proteins of the invention bind to porcine ASGR-1. In some embodiments, the antigen binding proteins of the invention bind to canine ASGR-1. In some embodiments, the antigen binding proteins of the invention bind to murine ASGR-1. In some embodiments, the antigen binding proteins of the invention bind to rat ASGR-1. In some embodiments, the antigen binding proteins specifically bind to ASGR-1 of the different species.

In some embodiments, the antigen binding proteins of the invention binds to ASGR-2 of different species, including, but not limited to, human, cynomolgus, porcine, canine, murine and rat. In some embodiments, the antigen binding proteins of the invention bind to human ASGR-2. In some embodiments, the antigen binding proteins of the invention bind to cynomolgus ASGR-2. In some embodiments, the antigen binding proteins of the invention bind to porcine ASGR-2. In some embodiments, the antigen binding proteins of the invention bind to canine ASGR-2. In some embodiments, the antigen binding proteins of the invention bind to murine ASGR-2. In some embodiments, the antigen binding proteins of the invention bind to rat ASGR-2. In some embodiments, the antigen binding proteins specifically bind to ASGR-2 of the different species.

In some embodiments, the antigen binding proteins of the invention bind to ASGR, ASGR-1 and/or ASGR-2 from two or more different species, and/or bind ASGR, ASGR-1 and/or ASGR-2 from the same species. For example, but not limited to: an antibody that binds human and cynomolgus ASGR-1; an antibody that binds to human, cynomolgus and porcine ASGR-1; an antibody that binds to human, cynomolgus, rat and murine ASGR-2; an antibody that binds human ASGR-1 and human ASGR-2; an antibody that binds human and cynomolgus ASGR-1 and ASGR-2. In some embodiments, the antigen binding proteins specifically bind to ASGR, ASGR-1 and/or ASGR-2 from two or more different species and/or specifically bind ASGR, ASGR-1 and/or ASGR-2 from the same species.

As discussed herein, the ASGR receptor, and ASGR-1 and/or ASGR-2 separately, internalize into the cell upon ligand binding. Accordingly, in certain embodiments, the invention provides antigen binding proteins that inhibit or reduce internalization of ASGR, ASGR-1 and/or ASGR-2. In certain embodiments, the antigen binding proteins of the invention reduce ligand binding and also inhibit internalization of ASGR, ASGR-1 and/or ASGR-2. In some embodiments, the antigen binding proteins of the invention inhibit internalization without necessarily inhibiting ligand binding.

In some embodiments, the antigen binding proteins (e.g., antibodies) of the invention are pH and/or calcium insensitive molecules, as well as binding to ASGR, ASGR-1 and/or ASGR-2 and inhibiting the binding to a ligand. It is envisioned that these properties are desired to reduce or prevent the molecule from disassociating from the receptor during the endocytotic process in order to extend the half-life of the molecule. In some embodiments, the antigen binding proteins (e.g., antibodies) with pH-independent binding to its antigen such that the affinity for the antigen binding at physiological pH (i.e., pH 7.4) is similar to that at endosomal pH (i.e., pH 5.5-6.0). In some embodiments, the antigen binding proteins (e.g., antibodies) with calcium-independent binding to its antigen such that the affinity for the antigen binding at assay conditions (i.e., 1mM calcium) is similar to that in the absence of exogenously added calcium. In some embodiments, the antigen binding proteins with both pH- and calcium-independent binding to its antigen such that the affinity for the antigen binding at physiologic pH and in the presence of calcium is similar to that at endosomal pH (i.e., pH 5.5-6.0) and in the absence of calcium. Any number of methods known to one skilled in the art can be used to measure pH and/or calcium insensitivity, such as the method described in Example 7C below.

ASGR-1, an asialoglycoprotein receptor, contains an N-term cytosolic domain, a transmembrane domain, a stalk region and a carbohydrate recognition domain (CRD) (alternatively known as the carbohydrate binding domain, or "CBD"). The carbohydrate recognition domain ("CRD") structure of ASGR-1 is reported in literature (M. Meier et al, JMB (2000)300, 857-865). The structure of ASGR-1 at a higher resolution than reported, and also when bound to various ligands (e.g., sugars including but not limited to lactose, galactose, and/or GalNAc or glycoproteins displaying such sugars including but not limited to fetuin, orosomucoid and/or alkaline phosphatase) is provided herein (see Example 10 and Figures 18-21 herein). Given the importance of this domain to the function of ASGR-1, in some embodiments, it is desirable to target this domain with the antigen binding proteins of the present invention.

Accordingly, in some embodiments, the antigen binding proteins of the invention bind to the CBD of ASGR-1. In certain embodiments, the antigen binding proteins of the invention bind to the CBD of human ASGR-1. In certain embodiments, the antigen binding proteins of the invention bind to the CBD of SEQ ID NO:5. In some embodiments, the antigen binding proteins of the invention bind to amino acid residues selected from the group consisting of 148-291, 149-291, 150-291, 151-291, 152-291, 153-291, and 154-291 of SEQ ID NO:5. In some embodiments, the invention comprises an isolated antigen binding protein that binds to human ASGR-1 CBD within Helix $\alpha$-1 or Helix $\alpha$-2. In some embodiments, the invention comprises an isolated antigen binding protein that binds to human ASGR-1 CBD within residues 174-186 of SEQ ID NO:5. In some embodiments, the invention comprises an isolated antigen binding protein that binds to human ASGR-1 CBD within residues 194-206 of SEQ ID NO:5. In some embodiments, the invention comprises an isolated antigen binding protein that binds to human ASGR-1 CBD at the same or overlapping binding site as where a ligand binds (e.g., sugars including but not limited to lactose, galactose, and/or GalNAc or glycoproteins displaying such sugars including but not limited to fetuin, orosomucoid and/or alkaline phosphatase or other sugars and glycoproteins capable of binding to ASGR, ASGR-1, and/or ASGR-2). In some embodiments, the invention comprises an isolated antigen binding protein that binds to human ASGR-1 CBD within residues 237-273 or residues 240-267 of SEQ ID NO:5. In some embodiments, the antigen binding proteins of the invention bind to the CBD of cynomolgus ASGR-1. In some embodiments, the antigen binding proteins of the invention bind to the CBD of porcine ASGR-1. In some embodiments, the antigen binding proteins of the invention bind to the CBD of canine ASGR-1. In some embodiments, the antigen binding proteins of the invention bind to the CBD of murine ASGR-1. In yet some embodiments, the antigen binding proteins of the invention bind to the CBD of rat ASGR-1. In yet some embodiments, the antigen binding proteins of the invention bind to the CBD of two or more different ASGR-1 species, for example, but not limited to, human ASGR-1 and cynomolgus ASGR-1, or human ASGR-1, cynomolgus ASGR-1 and canine ASGR-1, or human ASGR-1 and murine ASGR-1.

In further embodiments, the antigen binding proteins of the invention bind to ASGR-1 and inhibit binding of ligand to ASGR-1. In a specific embodiment, the ligands that are inhibited include, but are not limited to, sugars including but not limited to lactose, galactose, and/or GalNAc or glycoproteins displaying such sugars including but not limited to fetuin, orosomucoid and/or alkaline phosphatase or other sugars and glycoproteins capable of binding to ASGR, ASGR-1, and/or ASGR-2.

The tyrosine at position 272 of murine ASGR-1 (position 273 of human ASGR-1 (SEQ ID NO:5)) appears to be important for protein stability, as it displays hydrogen bonding to D266 of murine ASGR-1 and several van der Waals contacts to other residues of murine ASGR-1 (N208, W210, H256, and R270). Additionally, by analogy with other lectins, Y272 of murine ASGR-1 may play a role in carbohydrate binding and function of ASGR-1. Accordingly, in some embodiments, the antigen binding proteins of the invention bind to or interact with Y273 of human ASGR-1. In some embodiments, the antigen binding proteins of the invention bind to ASGR-1 at an epitope that comprises Y273 of human ASGR-1. In some embodiments, the antigen binding proteins of the invention bind to ASGR-1 at an epitope that results in Y273 of human ASGR-1 being unable to take part in binding ligand.

Analysis of the crystal structure of hASGR-1 revealed specific amino acids that are involved in the interaction between hASGR-1 and the ligands (e.g., sugars including but not limited to lactose, galactose, and/or GalNAc or glycoproteins displaying such sugars including but not limited to fetuin, orosomucoid and/or alkaline phosphatase). Accordingly, in further embodiments, the antigen binding proteins of the invention bind to or interact with at least one of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least one of Q240, D242, W244, E253, N265, D266, D267, N209, R237, P238, E239, P241, D243, Y245, G246, H247, G252, C255, H257, T259, D260, V268, R271, Y273, F258, R263, or W264. In further embodiments, the antigen binding proteins of the invention bind to hASGR-1 and block or reduce the binding or interaction of at least one of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., sugars including but not limited to lactose, galactose, and/or GalNAc or glycoproteins displaying such sugars including but not limited to fetuin, orosomucoid and/or alkaline phosphatase).

In further embodiments, the antigen binding proteins of the invention bind to or interact with at least one of Q240, D242, W244, E253, N265, D266, D267, R237, P238, H257, T259, or Y273. In some embodiments, the antigen binding proteins of the invention bind at an epitope comprising at least one of Q240, D242, W244, E253, N265, D266, D267, R237, P238, H257, T259, or Y273. In further embodiments, the antigen binding proteins of the invention bind to hASGR-1 and block or reduce the binding or interaction of at least one of Q240, D242, W244, E253, N265, D266, D267, R237, P238, H257, T259, or Y273 with a ligand (e.g., sugars including but not limited to lactose, galactose, and/or GalNAc or glycoproteins displaying such sugars including but not limited to fetuin, orosomucoid and/or alkaline phosphatase).

In some embodiments, the antigen binding proteins of the invention bind to or interact with at least two of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least three of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least four of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least five of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least six of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least seven of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least eight of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least nine ofQ240, D242, W244, E253, N265, D266, D267, N209, R237, P238, E239, P241, D243, Y245, G246, H247, G252, C255, H257, T259, D260, V268, R271, Y273, F258, R263, or W264 . In some embodiments, the antigen binding proteins of the invention bind to or interact with at least ten of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least eleven of Q240, D242, W244, E253, N265, D266, D267, N209, R237, P238, E239, P241, D243, Y245, G246, H247, G252, C255, H257, T259, D260, V268, R271, Y273, F258, R263, or W264 . In some embodiments, the antigen binding proteins of the invention bind to or interact with at least twelve of Q240, D242, W244, E253, N265, D266, D267, N209, R237, P238, E239, P241, D243, Y245, G246, H247, G252, C255, H257, T259, D260, V268, R271, Y273, F258, R263, or W264 . In some embodiments, the antigen binding proteins of the invention bind to or interact with at least thirteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least fourteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least fifteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least sixteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least seventeen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least eighteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least nineteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind to or interact with at least twenty of Q240, D242, W244, E253, N265, D266, D267, N209, R237, P238, E239, P241, D243, Y245, G246, H247, G252, C255, H257, T259, D260, V268, R271, Y273, F258, R263, or W264.

In some embodiments, the antigen binding proteins of the invention bind to or interact with at least two of Q240, D242, W244, E253, N265, D266, D267, R237, P238, H257, T259, or Y273. In some embodiments, the antigen binding proteins of the invention bind to or interact with at least three of Q240, D242, W244, E253, N265, D266, D267, R237, P238, H257, T259, or Y273. In some embodiments, the antigen binding proteins of the invention bind to or interact with at least four of Q240, D242, W244, E253, N265, D266, D267, R237, P238, H257, T259, or Y273. In some embodiments, the antigen binding proteins of the invention bind to or interact with at least five of Q240, D242, W244, E253, N265, D266, D267, R237, P238, H257, T259, or Y273. In some embodiments, the antigen binding proteins of the invention bind to or interact with at least six of Q240, D242, W244, E253, N265, D266, D267, R237, P238, H257, T259, or Y273. In some embodiments, the antigen binding proteins of the invention bind to or interact with at least seven of Q240, D242, W244, E253, N265, D266, D267, R237, P238, H257, T259, or Y273. In some embodiments, the antigen binding proteins of the invention bind to or interact with at least eight of Q240, D242, W244, E253, N265, D266, D267, R237, P238, H257, T259, or Y273. In some embodiments, the antigen binding proteins of the invention bind to or interact with at least nine of Q240, D242, W244, E253, N265, D266, D267, R237, P238, H257, T259, or Y273. In some embodiments, the antigen binding proteins of the invention bind to or interact with at least ten of Q240, D242, W244, E253, N265, D266, D267, R237, P238, H257, T259, or Y273. In some embodiments, the antigen binding proteins of the invention bind to or interact with at least eleven of Q240, D242, W244, E253, N265, D266, D267, R237, P238, H257, T259, or Y273. In some embodiments, the antigen binding proteins of the invention bind to or interact with at least all of Q240, D242, W244, E253, N265, D266, D267, R237, P238, H257, T259, or Y273.

In some embodiments, the antigen binding proteins of the invention bind at an epitope comprising at least two of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least three of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least four of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least five of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least six of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least seven of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least eight of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least nine of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least ten of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least eleven of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least twelve of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least thirteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least fourteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least fifteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least sixteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least seventeen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least eighteen of Q240, D242, W244, E253, N265, D266, D267, N209, R237, P238, E239, P241, D243, Y245, G246, H247, G252, C255, H257, T259, D260, V268, R271, Y273, F258, R263, or W264 . In some embodiments, the antigen binding proteins of the invention bind at an epitope comprising at least nineteen of Q240, D242, W244, E253, N265, D266, D267, N209, R237, P238, E239, P241, D243, Y245, G246, H247, G252, C255, H257, T259, D260, V268, R271, Y273, F258, R263, or W264. In some embodiments, the antigen binding proteins of the invention bind at an epitope comprising at least twenty of Q240, D242, W244, E253, N265, D266, D267, N209, R237, P238, E239, P241, D243, Y245, G246, H247, G252, C255, H257, T259, D260, V268, R271, Y273, F258, R263, or W264.

In some embodiments, the antigen binding proteins of the invention bind at an epitope comprising at least two of Q240, D242, W244, E253, N265, D266, D267, R237, P238, H257, T259, or Y273. In some embodiments, the antigen binding proteins of the invention bind at an epitope comprising at least three of Q240, D242, W244, E253, N265, D266, D267, R237, P238, H257, T259, or Y273. In some embodiments, the antigen binding proteins of the invention bind at an epitope comprising at least four of Q240, D242, W244, E253, N265, D266, D267, R237, P238, H257, T259, or Y273. In some embodiments, the antigen binding proteins of the invention bind at an epitope comprising at least five of Q240, D242, W244, E253, N265, D266, D267, R237, P238, H257, T259, or Y273. In some embodiments, the antigen binding proteins of the invention bind at an epitope comprising at least six of Q240, D242, W244, E253, N265, D266, D267, R237, P238, H257, T259, or Y273. In some embodiments, the antigen binding proteins of the invention bind at an epitope comprising at least seven of Q240, D242, W244, E253, N265, D266, D267, R237, P238, H257, T259, or Y273. In some embodiments, the antigen binding proteins of the invention bind at an epitope comprising at least eight of Q240, D242, W244, E253, N265, D266, D267, R237, P238, H257, T259, or Y273. In some embodiments, the antigen binding proteins of the invention bind at an epitope comprising at least nine of Q240, D242, W244, E253, N265, D266, D267, R237, P238, H257, T259, or Y273. In some embodiments, the antigen binding proteins of the invention bind at an epitope comprising at least ten of Q240, D242, W244, E253, N265, D266, D267, R237, P238, H257, T259, or Y273. In some embodiments, the antigen binding proteins of the invention bind at an epitope comprising at least eleven of Q240, D242, W244, E253, N265, D266, D267, R237, P238, H257, T259, or Y273. In some embodiments, the antigen binding proteins of the invention bind at an epitope comprising all of Q240, D242, W244, E253, N265, D266, D267, R237, P238, H257, T259, or Y273.

In further embodiments, the antigen binding proteins of the invention bind to hASGR-1 and block or reduce the binding or interaction of at least two of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., sugars including but not limited to lactose, galactose, and/or GalNAc or glycoproteins displaying such sugars including but not limited to fetuin, orosomucoid and/or alkaline phosphatase). In further embodiments, the antigen binding proteins of the invention bind to hASGR-1 and block or reduce the binding or interaction of at least three of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., sugars including but not limited to lactose, galactose, and/or GalNAc or glycoproteins displaying such sugars including but not limited to fetuin, orosomucoid and/or alkaline phosphatase). In further embodiments, the antigen binding proteins of the invention bind to hASGR-1 and block or reduce the binding or interaction of at least four of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g,sugars including but not limited to lactose, galactose, and/or GalNAc or glycoproteins displaying such sugars including but not limited to fetuin, orosomucoid and/or alkaline phosphatase). In further embodiments, the antigen binding proteins of the invention bind to hASGR-1 and block or reduce the binding or interaction of at least five of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g,sugars including but not limited to lactose, galactose, and/or GalNAc or glycoproteins displaying such sugars including but not limited to fetuin, orosomucoid and/or alkaline phosphatase). In further embodiments, the antigen binding proteins of the invention bind to hASGR-1 and block or reduce the binding or interaction of at least six of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g,sugars including but not limited to lactose, galactose, and/or GalNAc or glycoproteins displaying such sugars including but not limited to fetuin, orosomucoid and/or alkaline phosphatase). In further embodiments, the antigen binding proteins of the invention bind to hASGR-1 and block or reduce the binding or interaction of at least seven of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., sugars including but not limited to lactose, galactose, and/or GalNAc or glycoproteins displaying such sugars including but not limited to fetuin, orosomucoid and/or alkaline phosphatase). In further embodiments, the antigen binding proteins of the invention bind to hASGR-1 and block or reduce the binding or interaction of at least eight of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g,sugars including but not limited to lactose, galactose, and/or GalNAc or glycoproteins displaying such sugars including but not limited to fetuin, orosomucoid and/or alkaline phosphatase). In further embodiments, the antigen binding proteins of the invention bind to hASGR-1 and block or reduce the binding or interaction of at least nine of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least ten of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least eleven of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least twelve of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least thirteen of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least fourteen of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least fifteen of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least sixteen of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least seventeen of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least eighteen of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least nineteen of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc). In further embodiments, the antigen binding proteins of the invention bind to hASGR-1 and block or reduce the binding or interaction of at least twenty of Q240, D242, W244, E253, N265, D266, D267, N209, R237, P238, E239, P241, D243, Y245, G246, H247, G252, C255, H257, T259, D260, V268, R271, Y273, F258, R263, or W264 with a ligand (e.g., lactose, galactose and/or GalNAc).

In further embodiments, the antigen binding proteins of the invention bind to hASGR-1 and block or reduce the binding or interaction of at least two of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of at least three of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of at least four of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of at least five of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of at least six of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of at least seven of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of at least eight of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of at least nine of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of at least ten of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of at least eleven of Q240, D242, W244, E253, N265, D266, D267, R237, P238, H257, T259, or Y273 with a ligand (e.g., lactose, galactose and/or GalNAc). In further embodiments, the antigen binding proteins of the invention bind to hASGR-1 and block or reduce the binding or interaction of all of Q240, D242, W244, E253, N265, D266, D267, R237, P238, H257, T259, or Y273 with a ligand (e.g., lactose, galactose and/or GalNAc).

In order to relate unique antigen binding protein sequence features to specific functions or binding characteristics, sequences from antigen binding proteins of the invention from various characterization bins can be analyzed. For example, antigen binding proteins of the invention can be tested for their ability to bind a variety of binning probes (e.g., membrane preps from cells expressing ASGR-1 from different species or soluble huASGR-1). For each unique binding bin, the heavy and light chain sequences from each of the antigen binding proteins can be compared and claded based on, for example: 1. the unique VDJ and VJ rearrangements; 2. divergence from germline (ie. unique somatic hypermutation); and 3. relatedness to other antigen binding proteins of the same bin. Accordingly, in certain embodiments, the antigen binding proteins comprising the same or similar sequence features and patterns, will have substantially the same or similar binding characteristics. In specific embodiments, these antigen binding proteins can bind to the same or similar epitope with varying affinities.

The exemplary antigen binding proteins described herein have properties based on the epitope on ASGR, ASGR-1 and/or ASGR-2 that is bound by the antigen binding protein. The term "epitope" includes any determinant capable of being bound by an antigen binding protein, such as an antibody. An epitope is a region of an antigen that is bound by, or interacts with, an antigen binding protein that targets that antigen, and when the antigen is a protein, includes specific amino acids that directly contact, or interact with, the antigen binding protein. An epitope can be formed both by contiguous amino acids or non-contiguous amino acids juxtaposed by tertiary folding of a protein. A "linear epitope" is an epitope where an amino acid primary sequence comprises the recognized epitope. A linear epitope typically includes at least 3 or at least 4, and more usually, at least 5 or at least 6 or at least 7, for example, about 8 to about 10 amino acids in a unique sequence.

A "conformational epitope", in contrast to a linear epitope, is a group of discontinuous amino acids (e.g., in a polypeptide, amino acid residues that are not contiguous in the polypeptide's primary sequence but that, in the context of the polypeptide's tertiary and quaternary structure, are near enough to each other to be bound by an antigen binding protein). Epitope determinants can include chemically active surface groupings of molecules such as amino acids, sugar side chains, phosphoryl or sulfonyl groups, and can have specific three dimensional structural characteristics, and/or specific charge characteristics. Generally, antigen binding proteins specific for a particular target molecule will preferentially recognize an epitope on the target molecule in a complex mixture of proteins and/or macromolecules.

Methods of characterizing the epitope bound by an antigen binding protein are well known in the art, including, but not limited to, binning (competition and/or cross-competition) (Miller et al "Epitope binning of murine monoclonal antibodies by a multiplexed pairing assay" J Immunol Methods (2011) 365, 118-25), peptide mapping (e.g., PEPSPOT™) (Albert et al "The B-cell Epitope of the Monoclonal Anti-Factor VIII Antibody ESH8 Characterized by Peptide Array Analysis" 2008 Thromb Haemost 99, 634-7), mutagenesis methods such as chimeras (Song et al "Epitope Mapping of Ibalizumab, a Humanized Anti-CD4 Monoclonal Antibody with Anti-HIV-1 Activity in Infected Patients" J. Virol. (2010) 84, 6935-6942) , alanine scanning (Cunningham and Wells "High-resolution epitope mapping of HGH-receptor interactions by alanine-scanning mutagenesis" Science (1989) 244, 1081- 1085), arginine scanning (Lim et al "A diversity of antibody epitopes can induce signaling through the erythropoietin receptor" Biochemistry (2010) 49, 3797-3804), HD exchange methods (Coates et al "Epitope mapping by amide hydrogen/deuterium exchange coupled with immobilization of antibody, on-line proteolysis, liquid chromatography and mass spectrometry" Rapid Commun. Mass Spectrom. (2009) 23 639- 647), NMR cross saturation methods (Morgan et al "Precise epitope mapping of malaria parasite inhibitory antibodies by TROSY NMR cross-saturation" Biochemistry (2005) 44, 518-23), and crystallography (Gerhardt et al "Structure of IL-17A in complex with a potent, fully human neutralizing antibody" J. Mol. Biol (2009) 394, 905-21). The methods vary in the level of detail they provide as to the amino acids comprising the epitope.

Antigen binding proteins of the present invention include those that have an identical or overlapping epitope with an exemplary antigen binding protein described in Tables 2-7. In some embodiments, the antigen binding protein has an identical epitope as to the exemplary antigen binding proteins. In other embodiments, the antigen binding protein binds only a subset of the same amino acids as the exemplary antigen binding protein. In some embodiments, antigen binding proteins that might bind to any of the epitopes that are bound by the antibodies listed in Tables A, B, C or 6 are especially useful.

In certain embodiments, the antigen binding proteins of the present invention have an identical or overlapping epitope to the antigen binding proteins in Table 2-7 and comprise a) a light chain variable domain having at least 90% identity, at least 95% identity, or is identical to the amino acid sequence of the antigen binding proteins described in Tables 2-7; b) a heavy chain variable domain having at least 90% identity, at least 95% identity, or is identical to the amino acid sequence of the antigen binding proteins set forth in Tables 2-7; or c) the light chain variable domain of a) and the heavy chain variable domain of b).

In certain embodiments, the antigen binding protein of the present invention has an identical or overlapping epitope to the antigen binding proteins selected from the group consisting of 25A4, 4H6, 4A2, 5E5, 7E11, 54E9, 22G5, 194A4, 218G4, 176H4 and 194C10 wherein the antigen binding protein comprises a light chain variable domain having at least 90% identity, at least 95% identity, or is identical to the amino acid sequence of 25A4 and a heavy chain variable domain having at least 90% identity, at least 95% identity, or is identical to the amino acid sequence of 25A4; those comprising a light chain variable domain having at least 90% identity, at least 95% identity, or is identical to the amino acid sequence of 4H6 and a heavy chain variable domain having at least 90% identity, at least 95% identity, or is identical to the amino acid sequence of 4H6; those comprising a light chain variable domain having at least 90% identity, at least 95% identity, or is identical to the amino acid sequence of 4A2 and a heavy chain variable domain having at least 90% identity, at least 95% identity, or is identical to the amino acid sequence of 4A2; those comprising a light chain variable domain having at least 90% identity, at least 95% identity, or is identical to the amino acid sequence of 5E5 and a heavy chain variable domain having at least 90% identity, at least 95% identity, or is identical to the amino acid sequence of 5E5; those comprising a light chain variable domain having at least 90% identity, at least 95% identity, or is identical to the amino acid sequence of 7E11 and a heavy chain variable domain having at least 90% identity, at least 95% identity, or is identical to the amino acid sequence of 7E11; those comprising a light chain variable domain having at least 90% identity, at least 95% identity, or is identical to the amino acid sequence of 54E9 and a heavy chain variable domain having at least 90% identity, at least 95% identity, or is identical to the amino acid sequence of 54E9; those comprising a light chain variable domain having at least 90% identity, at least 95% identity, or is identical to the amino acid sequence of 22G5 and a heavy chain variable domain having at least 90% identity, at least 95% identity, or is identical to the amino acid sequence of 22G5;; those comprising a light chain variable domain having at least 90% identity, at least 95% identity, or is identical to the amino acid sequence of 194A4 and a heavy chain variable domain having at least 90% identity, at least 95% identity, or is identical to the amino acid sequence of 194A4; those comprising a light chain variable domain having at least 90% identity, at least 95% identity, or is identical to the amino acid sequence of 218G4G4 and a heavy chain variable domain having at least 90% identity, at least 95% identity, or is identical to the amino acid sequence of 218G4; those comprising a light chain variable domain having at least 90% identity, at least 95% identity, or is identical to the amino acid sequence of 176H4 and a heavy chain variable domain having at least 90% identity, at least 95% identity, or is identical to the amino acid sequence of 176H4; those comprising a light chain variable domain having at least 90% identity, at least 95% identity, or is identical to the amino acid sequence of 194C10 and a heavy chain variable domain having at least 90% identity, at least 95% identity, or is identical to the amino acid sequence of 194C10.

In certain embodiments, the ASGR-1 antigen binding protein of the invention has an identical or overlapping epitope as the antibodies in Tables 2-7, and comprises a light chain variable domain comprising an LCDR1 having no more than three amino acid additions, deletions, or substitutions from the LCDR1 sequence set forth in Table 2; an LCDR2 having no more than three amino acid additions, deletions, or substitutions from the LCDR2 sequence set forth in Table 2; and an LCDR3 having no more than three amino acid additions, deletions, or substitutions from the LCDR3 sequence set forth in Table 2; and a heavy chain variable domain comprising a) an HCDR1 having no more than three amino acid additions, deletions, or substitutions from the HCDR1 sequence set forth in Table 2; an HCDR2 having no more than three amino acid additions, deletions, or substitutions from the HCDR2 sequence set forth in Table 2; and an HCDR3 having no more than three amino acid additions, deletions, or substitutions from the HCDR3 sequence set forth in Table 2.

In certain embodiments, the ASGR-1 antigen binding protein of the invention has an identical or overlapping epitope as the antibodies in Tables A, B, C or 6, and comprises a light chain variable domain comprising an LCDR1 having no more than three amino acid additions, deletions, or substitutions from the LCDR1 sequence set forth in Tables A, B, C or 6; an LCDR2 having no more than three amino acid additions, deletions, or substitutions from the LCDR2 sequence set forth in Tables A, B, C or 6; and an LCDR3 having no more than three amino acid additions, deletions, or substitutions from the LCDR3 sequence set forth in Tables A, B, C or 6; and a heavy chain variable domain comprising a) an HCDR1 having no more than three amino acid additions, deletions, or substitutions from the HCDR1 sequence set forth in Tables A, B, C or 6; an HCDR2 having no more than three amino acid additions, deletions, or substitutions from the HCDR2 sequence set forth in Tables A, B, C or 6; and an HCDR3 having no more than three amino acid additions, deletions, or substitutions from the HCDR3 sequence set forth in Tables A, B, C or 6.

In certain embodiments, the ASGR-1 antigen binding protein of the invention has an identical or overlapping epitope as the antibody, 25A4, and comprises a light chain variable domain comprising an LCDR1 having no more than three amino acid additions, deletions, or substitutions from the LCDR1 sequence set forth in SEQ ID NO:480; an LCDR2 having no more than three amino acid additions, deletions, or substitutions from the LCDR2 sequence set forth in SEQ ID NO:8492; and an LCDR3 having no more than three amino acid additions, deletions, or substitutions from the LCDR3 sequence set forth in SEQ ID NO:16504; and a heavy chain variable domain comprising an HCDR1 having no more than three amino acid additions, deletions, or substitutions from the HCDR1 sequence set forth in SEQ ID NO:4488; an HCDR2 having no more than three amino acid additions, deletions, or substitutions from the HCDR2 sequence set forth in SEQ ID NO:12500; and an HCDR3 having no more than three amino acid additions, deletions, or substitutions from the HCDR3 sequence set forth in SEQ ID NO:20512.

In certain embodiments, the ASGR-1 antigen binding protein of the invention has an identical or overlapping epitope as the antibody, 4H6, and comprises a light chain variable domain comprising an LCDR1 having no more than three amino acid additions, deletions, or substitutions from the LCDR1 sequence set forth in SEQ ID NO:894; an LCDR2 having no more than three amino acid additions, deletions, or substitutions from the LCDR2 sequence set forth in SEQ ID NO:8906; and an LCDR3 having no more than three amino acid additions, deletions, or substitutions from the LCDR3 sequence set forth in SEQ ID NO:16918; and a heavy chain variable domain comprising an HCDR1 having no more than three amino acid additions, deletions, or substitutions from the HCDR1 sequence set forth in SEQ ID NO:4902; an HCDR2 having no more than three amino acid additions, deletions, or substitutions from the HCDR2 sequence set forth in SEQ ID NO:12914; and an HCDR3 having no more than three amino acid additions, deletions, or substitutions from the HCDR3 sequence set forth in SEQ ID NO:20926.

In certain embodiments, the ASGR-1 antigen binding protein of the invention has an identical or overlapping epitope as the antibody, 4A2, and comprises a light chain variable domain comprising an LCDR1 having no more than three amino acid additions, deletions, or substitutions from the LCDR1 sequence set forth in SEQ ID NO:1130; an LCDR2 having no more than three amino acid additions, deletions, or substitutions from the LCDR2 sequence set forth in SEQ ID NO:9142; and an LCDR3 having no more than three amino acid additions, deletions, or substitutions from the LCDR3 sequence set forth in SEQ ID NO:17154; and a heavy chain variable domain comprising an HCDR1 having no more than three amino acid additions, deletions, or substitutions from the HCDR1 sequence set forth in SEQ ID NO:5136; an HCDR2 having no more than three amino acid additions, deletions, or substitutions from the HCDR2 sequence set forth in SEQ ID NO:13148; and an HCDR3 having no more than three amino acid additions, deletions, or substitutions from the HCDR3 sequence set forth in SEQ ID NO:21160.

In certain embodiments, the ASGR-1 antigen binding protein of the invention has an identical or overlapping epitope as the antibody, 5E5, and comprises a light chain variable domain comprising an LCDR1 having no more than three amino acid additions, deletions, or substitutions from the LCDR1 sequence set forth in SEQ ID NO:974; an LCDR2 having no more than three amino acid additions, deletions, or substitutions from the LCDR2 sequence set forth in SEQ ID NO:8986; and an LCDR3 having no more than three amino acid additions, deletions, or substitutions from the LCDR3 sequence set forth in SEQ ID NO:16998; and a heavy chain variable domain comprising an HCDR1 having no more than three amino acid additions, deletions, or substitutions from the HCDR1 sequence set forth in SEQ ID NO:4982; an HCDR2 having no more than three amino acid additions, deletions, or substitutions from the HCDR2 sequence set forth in SEQ ID NO:12994; and an HCDR3 having no more than three amino acid additions, deletions, or substitutions from the HCDR3 sequence set forth in SEQ ID NO:21006.

In certain embodiments, the ASGR-1 antigen binding protein of the invention has an identical or overlapping epitope as the antibody, 7E11, and comprises a light chain variable domain comprising an LCDR1 having no more than three amino acid additions, deletions, or substitutions from the LCDR1 sequence set forth in SEQ ID NO:872; an LCDR2 having no more than three amino acid additions, deletions, or substitutions from the LCDR2 sequence set forth in SEQ ID NO:8884; and an LCDR3 having no more than three amino acid additions, deletions, or substitutions from the LCDR3 sequence set forth in SEQ ID NO:16896; and a heavy chain variable domain comprising an HCDR1 having no more than three amino acid additions, deletions, or substitutions from the HCDR1 sequence set forth in SEQ ID NO:4880; an HCDR2 having no more than three amino acid additions, deletions, or substitutions from the HCDR2 sequence set forth in SEQ ID NO:12892; and an HCDR3 having no more than three amino acid additions, deletions, or substitutions from the HCDR3 sequence set forth in SEQ ID NO:20904.

In certain embodiments, the ASGR-1 antigen binding protein of the invention has an identical or overlapping epitope as the antibody, 54E9, and comprises a light chain variable domain comprising an LCDR1 having no more than three amino acid additions, deletions, or substitutions from the LCDR1 sequence set forth in SEQ ID NO:3448; an LCDR2 having no more than three amino acid additions, deletions, or substitutions from the LCDR2 sequence set forth in SEQ ID NO:11460; and an LCDR3 having no more than three amino acid additions, deletions, or substitutions from the LCDR3 sequence set forth in SEQ ID NO:19472; and a heavy chain variable domain comprising an HCDR1 having no more than three amino acid additions, deletions, or substitutions from the HCDR1 sequence set forth in SEQ ID NO:7452; an HCDR2 having no more than three amino acid additions, deletions, or substitutions from the HCDR2 sequence set forth in SEQ ID NO:15464; and an HCDR3 having no more than three amino acid additions, deletions, or substitutions from the HCDR3 sequence set forth in SEQ ID NO:23476.

In certain embodiments, the ASGR-1 antigen binding protein of the invention has an identical or overlapping epitope as the antibody, 22G5, and comprises a light chain variable domain comprising an LCDR1 having no more than three amino acid additions, deletions, or substitutions from the LCDR1 sequence set forth in SEQ ID NO:326; an LCDR2 having no more than three amino acid additions, deletions, or substitutions from the LCDR2 sequence set forth in SEQ ID NO:8338; and an LCDR3 having no more than three amino acid additions, deletions, or substitutions from the LCDR3 sequence set forth in SEQ ID NO:16350; and a heavy chain variable domain comprising an HCDR1 having no more than three amino acid additions, deletions, or substitutions from the HCDR1 sequence set forth in SEQ ID NO:4334; an HCDR2 having no more than three amino acid additions, deletions, or substitutions from the HCDR2 sequence set forth in SEQ ID NO:12346; and an HCDR3 having no more than three amino acid additions, deletions, or substitutions from the HCDR3 sequence set forth in SEQ ID NO:20358.

In certain embodiments, the ASGR-1 antigen binding protein of the invention has an identical or overlapping epitope as the antibody, 194A4, and comprises a light chain variable domain comprising an LCDR1 having no more than three amino acid additions, deletions, or substitutions from the LCDR1 sequence set forth in SEQ ID NO:2780; an LCDR2 having no more than three amino acid additions, deletions, or substitutions from the LCDR2 sequence set forth in SEQ ID NO:10792; and an LCDR3 having no more than three amino acid additions, deletions, or substitutions from the LCDR3 sequence set forth in SEQ ID NO:18804; and a heavy chain variable domain comprising an HCDR1 having no more than three amino acid additions, deletions, or substitutions from the HCDR1 sequence set forth in SEQ ID NO:6786; an HCDR2 having no more than three amino acid additions, deletions, or substitutions from the HCDR2 sequence set forth in SEQ ID NO:14798; and an HCDR3 having no more than three amino acid additions, deletions, or substitutions from the HCDR3 sequence set forth in SEQ ID NO:22810.

In certain embodiments, the ASGR-1 antigen binding protein of the invention has an identical or overlapping epitope as the antibody, 218G4, and comprises a light chain variable domain comprising an LCDR1 having no more than three amino acid additions, deletions, or substitutions from the LCDR1 sequence set forth in SEQ ID NO:3746; an LCDR2 having no more than three amino acid additions, deletions, or substitutions from the LCDR2 sequence set forth in SEQ ID NO:11758; and an LCDR3 having no more than three amino acid additions, deletions, or substitutions from the LCDR3 sequence set forth in SEQ ID NO:19770; and a heavy chain variable domain comprising an HCDR1 having no more than three amino acid additions, deletions, or substitutions from the HCDR1 sequence set forth in SEQ ID NO:7750; an HCDR2 having no more than three amino acid additions, deletions, or substitutions from the HCDR2 sequence set forth in SEQ ID NO:15762; and an HCDR3 having no more than three amino acid additions, deletions, or substitutions from the HCDR3 sequence set forth in SEQ ID NO:23774.

In certain embodiments, the ASGR-1 antigen binding protein of the invention has an identical or overlapping epitope as the antibody, 176H4, and comprises a light chain variable domain comprising an LCDR1 having no more than three amino acid additions, deletions, or substitutions from the LCDR1 sequence set forth in SEQ ID NO:2502; an LCDR2 having no more than three amino acid additions, deletions, or substitutions from the LCDR2 sequence set forth in SEQ ID NO:10514; and an LCDR3 having no more than three amino acid additions, deletions, or substitutions from the LCDR3 sequence set forth in SEQ ID NO:18526; and a heavy chain variable domain comprising an HCDR1 having no more than three amino acid additions, deletions, or substitutions from the HCDR1 sequence set forth in SEQ ID NO:6508; an HCDR2 having no more than three amino acid additions, deletions, or substitutions from the HCDR2 sequence set forth in SEQ ID NO:14520; and an HCDR3 having no more than three amino acid additions, deletions, or substitutions from the HCDR3 sequence set forth in SEQ ID NO:22532.

In certain embodiments, the ASGR-1 antigen binding protein of the invention has an identical or overlapping epitope as the antibody, 194C10, and comprises a light chain variable domain comprising an LCDR1 having no more than three amino acid additions, deletions, or substitutions from the LCDR1 sequence set forth in SEQ ID NO:2792; an LCDR2 having no more than three amino acid additions, deletions, or substitutions from the LCDR2 sequence set forth in SEQ ID NO:10804; and an LCDR3 having no more than three amino acid additions, deletions, or substitutions from the LCDR3 sequence set forth in SEQ ID NO:18816; and a heavy chain variable domain comprising an HCDR1 having no more than three amino acid additions, deletions, or substitutions from the HCDR1 sequence set forth in SEQ ID NO:6798; an HCDR2 having no more than three amino acid additions, deletions, or substitutions from the HCDR2 sequence set forth in SEQ ID NO:14810; and an HCDR3 having no more than three amino acid additions, deletions, or substitutions from the HCDR3 sequence set forth in SEQ ID NO:22822.

Antigen binding proteins that have an identical or overlapping epitope will often compete for binding to the antigen, ASGR, ASGR1 and/or ASGR2. Thus, in certain embodiments, an antigen binding protein (e.g., antibody or antibody fragment thereof) of the invention competes with the antigen binding proteins described in Tables 2-7. In some embodiments, an antigen binding protein (e.g., antibody or antibody fragment thereof) of the invention competes with the antigen binding proteins described in Tables A, B and C. In some embodiments, an antigen binding protein (e.g., antibody or antibody fragment thereof) of the invention competes with the antigen binding proteins described in Table 6. To "compete" or "competition" means the antigen binding proteins compete for the same epitope or binding site on a target. Such competition can be determined by an assay in which the reference antigen binding protein (e.g., antibody or antibody fragment thereof) prevents or inhibits specific binding of a test antigen binding protein. Numerous types of competitive binding assays can be used to determine if a test molecule competes with a reference molecule for binding. Examples of assays that can be employed include solid phase direct or indirect radioimmunoassay (RIA), solid phase direct or indirect enzyme immunoassay (EIA), sandwich competition assay (see, e.g., Stahli et al. (1983) Methods in Enzymology 9:242-253), solid phase direct biotin-avidin EIA (see, e.g., Kirkland et al., (1986) J. Immunol. 137:3614-3619), solid phase direct labeled assay, solid phase direct labeled sandwich assay, Luminex (Jia et al "A novel method of Multiplexed Competitive Antibody Binning for the characterization of monoclonal antibodies" J. Immunological Methods (2004) 288, 91-98) and surface plasmon resonance ((Song et al "Epitope Mapping of Ibalizumab, a Humanized Anti-CD4 Monoclonal Antibody with Anti-HIV-1 Activity in Infected Patients" J. Virol. (2010) 84, 6935-6942). An exemplary method of determining competition is described in Example 7D. Usually, when a competing antigen binding protein is present in excess, it will inhibit binding of a reference antigen binding protein to a common antigen by at least 50%, 55%, 60%, 65%, 70%, or 75%. In some instances, binding to ASGR-1 is inhibited by at least 80%, 85%, 90%, 95%, 96%, 97%, 98%, 99%, or more.

Besides competition, antigen binding proteins (e.g., antibodies or antibody fragments thereof) with identical, overlapping, or similar epitopes may be affected by mutagenesis of ASGR, ASGR-1 and/or ASGR-2 similarly. In brief, the domain(s)/region(s) containing residues that are in contact with or are buried by an antibody can be identified by mutating specific residues in ASGR, ASGR-1 and/or ASGR-2 (e.g., a wild-type antigen) and determining whether the antigen binding protein can bind the mutated or variant ASGR, ASGR-1 and/or ASGR-2 protein. By making a number of individual mutations, residues that play a direct role in binding or that are in sufficiently close proximity to the antibody such that a mutation can affect binding between the antigen binding protein and antigen can be identified. From the knowledge of these amino acids, the domain(s) or region(s) of the antigen that contain residues in contact with the antigen binding protein or covered by the antibody can be elucidated. Such a domain can include the binding epitope of an antigen binding protein. As mentioned above, one specific example of this general approach utilizes an arginine/glutamic acid scanning protocol (see, e.g., Nanevicz, T., et al., 1995, J. Biol. Chem., 270:37, 21619-21625 and Zupnick, A., et al., 2006, J. Biol. Chem., 281:29, 20464-20473). In general, arginine and glutamic acids are substituted (typically individually) for an amino acid in the wild-type polypeptide because these amino acids are charged and bulky and thus have the potential to disrupt binding between an antigen binding protein and an antigen in the region of the antigen where the mutation is introduced. Arginine residues that exist in the wild-type antigen are replaced with glutamic acid. A variety of such individual mutants are obtained and the collected binding results analyzed to determine what residues affect binding. In Example 7E, scanning arginine/glutamic acid mutagenesis was performed using the human ASGR-1 CBD domain and the effect on exemplary antibodies was determined. Included with the scope of the invention are ASGR, ASGR-1 and/or ASGR-2 antigen binding proteins having characteristics such that they are affected in a similar way as an exemplary antibody to mutagenesis.

Example 7E describes one such arginine/glutamic acid scanning of ASGR-1 for ASGR-1 antigen binding proteins provided herein. A series of mutant ASGR-1 antigens were created, with each mutant antigen having a single mutation. Binding of each mutant ASGR-1 antigen with various ASGR-1 antigen binding proteins was measured and compared to the ability of the selected antigen binding proteins to bind to human ASGR-1 (SEQ ID NO:5). In certain embodiments, binding of an antigen binding protein of the present invention to ASGR-1 is inhibited by a single mutation in ASGR-1, wherein the single mutation is selected from the group consisting of R170, S171, G172, R183, L184, W195, E196, K199, H203, H204, P207, V208, N209, H215, D216, P220, D225, D228, R237, P238, E239, P241, D242, D243, Y245, G246, H247, G248, L249, G251, E253, T259, D260, R263, N265, Q270, R271, P272, R274, and E280 as shown in SEQ ID NO:5. In some embodiments, the ASGR-1 antigen binding proteins share the attributes of antibody 4A2 and their binding to ASGR-1 is inhibited a mutation of any of W195, E196, K199, H204, P207, and R263. In some embodiments, the ASGR-1 antigen binding proteins share the attributes of antibody 4B3 and their binding to ASGR-1 isinhibited by a mutation of any of H203, H204, P220, and G251. In some embodiments, the ASGR-1 antigen binding proteins share the attributes of antibody 5E5 and their binding to ASGR-1 is inhibited by a mutation of any of W195, K199, and R263. In some embodiments, the ASGR-1 antigen binding proteins share the attributes of antibody 6G7 and their binding to ASGR-1 is inhibited by a mutation of any of R183, L184, H215, P220, P238, G246, H247, G248, G251, and N265. In some embodiments, the ASGR-1 antigen binding proteins share the attributes of antibody 149D11 and their binding is inhibited by a mutation of any of R170, S171, and L184. In some embodiments, the ASGR-1 antigen binding proteins share the attributes of antibody 175F4 and their binding is inhibited by a mutation of R183. In some embodiments, the ASGR-1 antigen binding proteins share the attributes of antibody 17H6 and their binding is inhibited by a mutation of any of P241, D242, D243, Y245, G251, and E253. In some embodiments, the ASGR-1 antigen binding proteins share the attributes of antibody 194A4 and their binding is inhibited by a mutation of D260. In some embodiments, the ASGR-1 antigen binding proteins share the attributes of antibody 60C12 and their binding is inhibited by a mutation of any of R170, R237, E239, P241, T259, D260, R263, and N265. In some embodiments, the ASGR-1 antigen binding proteins share the attributes of antibody 65D5 and their binding is inhibited by a mutation of any of R237, T259, D260 and R263. In some embodiments, the ASGR-1 antigen binding proteins share the attributes of antibody 190F8 or 191G1 and their binding is inhibited by a mutation of any of R170, S171, G172, E196, H204, P207, V208, N209, H215, D216, D225, D228, P238, D243, G248, L249, G251, D260, Q270, R271, P272, R274 and E280. In some embodiments, the ASGR-1 antigen binding proteins share the attributes of antibody 199A7 and their binding is inhibited by a mutation of any of R170, R183, H215 and Q270. In some embodiments, the ASGR-1 antigen binding proteins share the attributes of antibody 146B6 and their binding is inhibited by a mutation of any of P241, T259, and N265. In some embodiments, the ASGR-1 antigen binding proteins share the attributes of antibody 193E7 and their binding is inhibited by a mutation of any of P207 and R263. In some embodiments, any of two or more, three or more, four or more, five or more, six or more, seven or more, eight or more nine or more, ten or more, or all of the single mutations of the aforementioned groups individually inhibit binding of the ASGR-1 antigen binding protein to ASGR-1.

Binding of various anti-ASGR-1 antigen binding proteins (e.g., antibodies 5E5, 22G5, 7E11, 4A2, 4H6, 72G9, 194A4, 54E9, 218G4, 176H4 and 194C10) were further analyzed using X-ray crystallography. The results from the X-ray crystallography were highly correlated with the results from the Arginine/Glutamic acid mutagenesis profiling described above and in Example 7E. The interface between an antigen binding protein and the antigen can be determined/defined a number of ways. In Examples 10B-L, the interface was determined by selecting interface residues having at least one atom within a predefined distance to its partner protein. In some embodiments, ASGR-1 residues that are within the interface with antibody, 5E5, as determined by distance of 8Å or less are: H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, or W264 (SEQ ID NO:5). In some embodiments, ASGR-1 residues that are within the interface with antibody, 5E5, as determined by distance of 5Å or less are: H161, E162, W195, E196, Q198, K199, F200, Q202, H203, H204, G232, F233, K234, N235, W236, R237, P238, D261, G262, or R263 (SEQ ID NO:5). In certain embodiments, the ASGR-1 antigen binding protein forms an interface with ASGR-1 that overlaps with that of antibody 5E5, including those wherein any of: H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, or W264 (SEQ ID NO:5) are within the interface. In certain embodiments, the ASGR-1 antigen binding protein forms an interface with ASGR-1 that overlaps with that of antibody 5E5, including those wherein any of H161, E162, W195, E196, Q198, K199, F200, Q202, H203, H204, G232, F233, K234, N235, W236, R237, or P238 (SEQ ID NO:5) are within the interface.

In some embodiments, ASGR-1 residues that are within the interface with antibody, 22G5, as determined by distance of 8Å or less are: W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, or T279 (SEQ ID NO:5). In some embodiments, ASGR-1 residues that are within the interface with antibody, 22G5, as determined by distance of 5Å or less are: W167, S171, G172, K173, A174, A176, D177, N180, Y181, R183, L184, E185, D186, Q270, P272, or W275 (SEQ ID NO:5). In certain embodiments, the ASGR-1 antigen binding protein forms an interface with ASGR-1 that overlaps with that of antibody 5E5, including those wherein any of: W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, or T279 (SEQ ID NO:5) are within the interface. In certain embodiments, the ASGR-1 antigen binding protein forms an interface with ASGR-1 that overlaps with that of antibody 5E5, including those wherein any of W167, S171, G172, K173, A174, A176, D177, N180, Y181, R183, L184, E185, D186, Q270, P272, or W275 (SEQ ID NO:5) are within the interface.

In some embodiments, ASGR-1 residues that are within the interface with antibody, 4A2, as determined by distance of 8Å or less are: R170, W195, E196, K199, Q202, H203, H204, 1205, G206, P207, V208, F233, K234, N235, W236, P238, D260, D261, G262, R263, R274, P155, N157, W158, F168, S169, R170, W175, A178, D179, C182, A187, W211, C269, R271, Y273, R274, C277, or T279 (SEQ ID NO:5). In some embodiments, ASGR-1 residues that are within the interface with antibody, 4A2, as determined by distance of 5Å or less are: R170, W195, E196, K199, Q202, H203, H204, 1205, G206, P207, V208, F233, K234, N235, W236, P238, D260, D261, G262, R263, or R274 (SEQ ID NO:5). In certain embodiments, the ASGR-1 antigen binding protein forms an interface with ASGR-1 that overlaps with that of antibody 4A2, including those wherein any of R170, W195, E196, K199, Q202, H203, H204, I205, G206, P207, V208, F233, K234, N235, W236, P238, D260, D261, G262, R263, R274, P155, N157, W158, F168, S169, R170, W175, A178, D179, C182, A187, W211, C269, R271, Y273, R274, C277, or T279 (SEQ ID NO:5) are within the interface. In certain embodiments, the ASGR-1 antigen binding protein forms an interface with ASGR-1 that overlaps with that of antibody 4A2, including those wherein any of: R170, W195, E196, K199, Q202, H203, H204, 1205, G206, P207, V208, F233, K234, N235, W236, P238, D260, D261, G262, R263, R274 (SEQ ID NO:5) are within the interface.

In some embodiments, ASGR-1 residues that are within the interface with antibody, 7E11, as determined by distance of 8Å or less are: H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, or W264 (SEQ ID NO:5). In some embodiments, ASGR-1 residues that are within the interface with antibody, 7E11, as determined by distance of 5Å or less are: H161, S194, W195, E196, Q198, K199, F200, Q202, H203, F233, K234, N235, W236, R237, P238, or R263 (SEQ ID NO:5). In certain embodiments, the ASGR-1 antigen binding protein forms an interface with ASGR-1 that overlaps with that of antibody 7E11, including those wherein any of : H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, or W264 (SEQ ID NO:5) are within the interface. In certain embodiments, the ASGR-1 antigen binding protein forms an interface with ASGR-1 that overlaps with that of antibody 7E11, including those wherein any of are within the surface: H161, S194, W195, E196, Q198, K199, F200, Q202, H203, F233, K234, N235, W236, R237, P238, or R263 (SEQ ID NO:5) are within the interface.

In some embodiments, ASGR-1 residues that are within the interface with antibody, 4H6, as determined by distance of 8Å or less are: H161, E162, T193, S194, W195, E196, K199, Q202, T231, G232, F233, K234, N235, P238, D261, R263, R163, V192, E197, Q198, H203, P207, D228, E230, W236, R237, D260, G262, or W264 (SEQ ID NO:5). In some embodiments, ASGR-1 residues that are within the interface with antibody, 4H6, as determined by distance of 5Å or less are: H161, E162, T193, S194, W195, E196, K199, Q202, T231, G232, F233, K234, N235, P238, D261, or R263 (SEQ ID NO:5). In certain embodiments, the ASGR-1 antigen binding protein forms an interface with ASGR-1 that overlaps with that of antibody 4H6, including those wherein any of: H161, E162, T193, S194, W195, E196, K199, Q202, T231, G232, F233, K234, N235, P238, D261, R263, R163, V192, E197, Q198, H203, P207, D228, E230, W236, R237, D260, G262, or W264 (SEQ ID NO:5) are within the interface. In certain embodiments, the ASGR-1 antigen binding protein forms an interface with ASGR-1 that overlaps with that of antibody 4H6, including those wherein any of are within the surface: H161, E162, T193, S194, W195, E196, K199, Q202, T231, G232, F233, K234, N235, P238, D261, or R263 (SEQ ID NO:5) are within the interface.

In some embodiments, ASGR-1 residues that are within the interface with antibody, 72G9, as determined by distance of 8Å or less are: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, or C269 (SEQ ID NO:5). In some embodiments, ASGR-1 residues that are within the interface with antibody, 72G9, as determined by distance of 5Å or less are: D216, Q217, N218, G219, P220, W221, Y229, E230, K234, W236, E239, Q240, P241, D242, D243, W244, Y245, G246, L249, G250, G251, G252, D254, or Q270 (SEQ ID NO:5). In certain embodiments, the ASGR-1 antigen binding protein forms an interface with ASGR-1 that overlaps with that of antibody 72G9, including those wherein any of: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269 (SEQ ID NO:5) are within the interface. In certain embodiments, the ASGR-1 antigen binding protein forms an interface with ASGR-1 that overlaps with that of antibody 72G9, including those wherein any of: D216, Q217, N218, G219, P220, W221, Y229, E230, K234, W236, E239, Q240, P241, D242, D243, W244, Y245, G246, L249, G250, G251, G252, D254, or Q270 (SEQ ID NO:5) are within the interface.

In some embodiments, ASGR-1 residues that are within the interface with antibody, 194A4, as determined by distance of 8Å or less are: T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, or W264 (SEQ ID NO:5). In some embodiments, ASGR-1 residues that are within the interface with antibody, 194A4, as determined by distance of 5Å or less are: T193, S194, W195, E196, P220, W221, G226, T227, D228, Y229, E230, T231, G232, F233, K234, N235, W236, R237, P238, E239, or G252 (SEQ ID NO:5). In certain embodiments, the ASGR-1 antigen binding protein forms an interface with ASGR-1 that overlaps with that of antibody 194A4, including those wherein any of: T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, or W264 (SEQ ID NO:5) are within the interface. In certain embodiments, the ASGR-1 antigen binding protein forms an interface with ASGR-1 that overlaps with that of antibody 194A4, including those wherein any of are within the surface: T193, S194, W195, E196, P220, W221, G226, T227, D228, Y229, E230, T231, G232, F233, K234, N235, W236, R237, P238, E239, or G252 (SEQ ID NO:5) within the interface.

In some embodiments, ASGR-1 residues that are within the interface with antibody, 194C10, as determined by distance of 8Å or less are: N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270 or W275 (SEQ ID NO:5). In some embodiments, ASGR-1 residues that are within the interface with antibody, 194C10, as determined by distance of 5Å or less are: N157, R170, S171, G172, Q202, H203, H204, I205, G206, P207, V208, N209, T210, D260, R271, P272, Y273 or R274 (SEQ ID NO:5). In certain embodiments, the ASGR-1 antigen binding protein forms an interface with ASGR-1 that overlaps with that of antibody 194C10, including those wherein any of: N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5) are within the interface. In certain embodiments, the ASGR-1 antigen binding protein forms an interface with ASGR-1 that overlaps with that of antibody 194C10, including those wherein any of N157, R170, S171, G172, Q202, H203, H204, I205, G206, P207, V208, N209, T210, D260, R271, P272, Y273, or R274 (SEQ ID NO:5) are within the interface.

In some embodiments, ASGR-1 residues that are within the interface with antibody, 54E9, as determined by distance of 8Å or less are: W195, N209, N235, R237, P238, E239, Q240, D242, H257, T259, D260, D261, R263, N265, D267, R271, Y273, Q198, Q202, P207, V208, F233, W236, D243, E253, F258, G262, W264, or D266 (SEQ ID NO:5). In some embodiments, ASGR-1 residues that are within the interface with antibody, 54E9, as determined by distance of 5Å or less are: W195, N209, N235, R237, P238, E239, Q240, D242, H257, T259, D260, D261, R263, N265, D267, R271, or Y273 (SEQ ID NO:5). In certain embodiments, the ASGR-1 antigen binding protein forms an interface with ASGR-1 that overlaps with that of antibody 54E9, including those wherein any of W195, N209, N235, R237, P238, E239, Q240, D242, H257, T259, D260, D261, R263, N265, D267, R271, Y273, Q198, Q202, P207, V208, F233, W236, D243, E253, F258, G262, W264, or D266 (SEQ ID NO:5) are within the interface. In certain embodiments, the ASGR-1 antigen binding protein forms an interface with ASGR-1 that overlaps with that of antibody 54E9, including those wherein any of: W195, N209, N235, R237, P238, E239, Q240, D242, H257, T259, D260, D261, R263, N265, D267, R271, or Y273 (SEQ ID NO:5) are within the interface.

In some embodiments, ASGR-1 residues that are within the interface with antibody, 218G4, as determined by distance of 8Å or less are: R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, or W275 (SEQ ID NO:5). In some embodiments, ASGR-1 residues that are within the interface with antibody, 218G4, as determined by distance of 5Å or less are: R170, S171, G172, A174, H204, I205, G206, P207, V208, N209, H257, D260, N265, D267, Q270, R271, P272, Y273, or R274 (SEQ ID NO:5). In certain embodiments, the ASGR-1 antigen binding protein forms an interface with ASGR-1 that overlaps with that of antibody 218G4, including those wherein any of: R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, or W275 (SEQ ID NO:5) are within the interface. In certain embodiments, the ASGR-1 antigen binding protein forms an interface with ASGR-1 that overlaps with that of antibody 218G4, including those wherein any of: R170, S171, G172, K173, A174, D177, P207, V208, N209, R237, Q240, W244, G246, H247, G248, L249, E253, H257, T259, D260, N265, D267, Q270, R271, P272, Y273 or R274 (SEQ ID NO:5) are within the interface.

In some embodiments, ASGR-1 residues that are within the interface with antibody, 176H4, as determined by distance of 8Å or less are: R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments, ASGR-1 residues that are within the interface with antibody, 176H4, as determined by distance of 5Å or less are: R170, S171, G172, K173, A174, D177, P207, V208, N209, R237, Q240, W244, G246, H247, G248, L249, E253, H257, T259, D260, N265, D267, Q270, R271, P272, Y273 or R274 (SEQ ID NO:5). In certain embodiments, the ASGR-1 antigen binding protein forms an interface with ASGR-1 that overlaps with that of antibody 176H4, including those wherein any of R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, W275 (SEQ ID NO:5) are within the interface. In certain embodiments, the ASGR-1 antigen binding protein forms an interface with ASGR-1 that overlaps with that of antibody 176H4, including those wherein any of: R170, S171, G172, K173, A174, D177, P207, V208, N209, R237, Q240, W244, G246, H247, G248, L249, E253, H257, T259, D260, N265, D267, Q270, R271, P272, Y273, R274 (SEQ ID NO:5) are within the interface.

In some embodiments, the ASGR-1 residues that are involved in ligand binding are also in close proximity to the areas where antibodies 72G9, 54E9, 218G4 or 176H4 bind and can be useful for manipulating ASGR-1 binding to ligand. In some embodiments, the ASGR-1 antigen binding protein forms an interface with ASGR-1 that overlaps with that of antibody 72G9 and the ligand (e.g., GalNAc), including those wherein any of Q240, D242, W244, E239, P241, D243, Y245, G246, G252, R237, E253, P238, H247, C255, or V268 (SEQ ID NO:5) are within the interface. In some embodiments, the ASGR-1 antigen binding protein forms an interface with ASGR-1 that overlaps with that of antibody 72G9 and the ligand (e.g., GalNAc), including those wherein any of Q240, D242, or W244 (SEQ ID NO:5) are within the interface. In some embodiments, the ASGR-1 antigen binding protein forms an interface with ASGR-1 that overlaps with that of antibody 72G9 and the ligand (e.g., GalNAc), including those wherein any of Q240, D242, W244, E239, P241, D243, Y245, G246 or G252 (SEQ ID NO:5) are within the interface. In some embodiments, the ASGR-1 antigen binding protein forms an interface with ASGR-1 that overlaps with that of antibody 72G9 and the ligand (e.g., GalNAc), including those wherein any of Q240, D242, W244, R237 or E253 (SEQ ID NO:5) are within the interface. As noted in the examples below, the extent of inhibition resulting from 72G9 is lower than other direct blocking antibodies provided herein. While not intended to be limiting, this is understood to occur due to the nature of the relative orientations of the ASGR-1 protein and the antibody when bound to one another. For example, when the 72G9 antibody is bound to ASGR-1, there is still sufficient space for a ligand to reach the binding site, to some (althought lesser) extent. In some embodiments, the ASGR-1 antigen binding protein forms an interface with ASGR-1 that overlaps with that of antibody 54E9 and the ligand (e.g., GalNAc), including those wherein any of N209, R237, Q240, D242, H257, T259, N265, D267, Y273, P238, E239, D260, R263, R271, E253, D266, D243, F258, or W264 (SEQ ID NO:5) are within the interface. In some embodiments, the ASGR-1 antigen binding protein forms an interface with ASGR-1 that overlaps with that of antibody 54E9 and the ligand (e.g., GalNAc), including those wherein any of N209, R237, Q240, D242, H257, T259, N265, D267, or Y273 (SEQ ID NO:5) are within the interface. In some embodiments, the ASGR-1 antigen binding protein forms an interface with ASGR-1 that overlaps with that of antibody 54E9 and the ligand (e.g., GalNAc), including those wherein any of N209, R237, Q240, D242, H257, T259, N265, D267, Y273, P238, E239, D260, R263, or R271 (SEQ ID NO:5) are within the interface. In some embodiments, the ASGR-1 antigen binding protein forms an interface with ASGR-1 that overlaps with that of antibody 54E9 and the ligand (e.g., GalNAc), including those wherein any of N209, R237, Q240, D242, H257, T259, N265, D267, Y273, E253 or D266 (SEQ ID NO:5) are within the interface. In some embodiments, the ASGR-1 antigen binding protein forms an interface with ASGR-1 that overlaps with that of antibody 218G4 and the ligand (e.g., GalNAc), including those wherein any of N209, H257, N265, D267, Y273, D260, R271, R237, T259, D266, F258 or V268 (SEQ ID NO:5) are within the interface. In some embodiments, the ASGR-1 antigen binding protein forms an interface with ASGR-1 that overlaps with that of antibody 218G4 and the ligand (e.g., GalNAc), including those wherein any of N209, H257, N265, D267, or Y273 (SEQ ID NO:5) are within the interface. In some embodiments, the ASGR-1 antigen binding protein forms an interface with ASGR-1 that overlaps with that of antibody 218G4 and the ligand (e.g., GalNAc), including those wherein any of N209, H257, N265, D267, Y273, D260 or R271 (SEQ ID NO:5) are within the interface. In some embodiments, the ASGR-1 antigen binding protein forms an interface with ASGR-1 that overlaps with that of antibody 218G4 and the ligand (e.g., GalNAc), including those wherein any of N209, H257, N265, D267, Y273. R237, T259 or D266 (SEQ ID NO:5) are within the interface. In some embodiments, the ASGR-1 antigen binding protein forms an interface with ASGR-1 that overlaps with that of antibody 176H4 and the ligand (e.g., GalNAc), including those wherein any of N209, R237, Q240, W244, E253, H257, T259, N265, D267, Y273, G246, H247, D260, R271, D266, P238, E239, Y245, F258, R263, W264, or V268 (SEQ ID NO:5) are within the interface. In some embodiments, the ASGR-1 antigen binding protein forms an interface with ASGR-1 that overlaps with that of antibody 176H4 and the ligand (e.g., GalNAc), including those wherein any of N209, R237, Q240, W244, E253, H257, T259, N265, D267, or Y273 (SEQ ID NO:5) are within the interface. In some embodiments, the ASGR-1 antigen binding protein forms an interface with ASGR-1 that overlaps with that of antibody 176H4 and the ligand (e.g., GalNAc), including those wherein any of N209, R237, Q240, W244, E253, H257, T259, N265, D267, Y273, G246, H247, D260, or R271 (SEQ ID NO:5) are within the interface. In some embodiments, the ASGR-1 antigen binding protein forms an interface with ASGR-1 that overlaps with that of antibody 176H4 and the ligand (e.g., GalNAc), including those wherein any of N209, R237, Q240, W244, E253, H257, T259, N265, D267, Y273, or D266 (SEQ ID NO:5) are within the interface.

As discussed above, the binding interaction between huASGR-1 and ligand (e.g., lactose, galactose, GalNAc), as well as the binding interaction between huASGR-1 and various embodiments of the antigen binding proteins (e.g., antibodies) of the present invention was evaluated using x-ray crystallography as described in Example 10. The binding interaction between huASGR-1 and various embodiments of the antigen binding proteins (e.g., antibodies) of the present invention was also evaluated using methodologies, including epitope binning as described in Example 7D, and arginine/glutamic acid mutational profiling as described in Example 7E. A summary of the data obtained through these methodologies is set forth in Table D below. This summary illustrates the various binding characteristics of representative antigen binding proteins (e.g., antibodies) of the present invention and their ability to directly and/or indirectly inhibit ligand binding to huASGR-1. In some embodiments, antibodies that interact with residues in common across different ligands can result in a similar form of inhibition (direct) across the various ligands. Examples of such residues are underlined and in bold in Table D.

Table D:  Summary of Binding Characteristics of Representative Antigen Binding Proteins Derived from Examples 7 and 10.

| Ligand/mAb Name | mAb Epitope (bin) | Interaction Site (crystal structure <5 angstroms) | Interaction Site (crystal structure 5-8 angstroms) | R/E scan |
|---|---|---|---|---|
| Ligand/ Lactose | ND | **Q240, D242, W244, E253, N265, D266, D267** | N209, R237, **P238, E239,** P241, **D243, Y245, G246, H247, G252, C255,** H257, T259, D260, **V268, R271,** Y273 | ND |
| Ligand/ Galactose | ND | R237, **D240, D242, W244, E253, N265, D266, D267** | N209, **P238, E239, P241, D243, Y245, G246, H247, G252, C255,** H257, T259, **V268, R271,** Y273 | ND |
| Ligand/ GalNAc | ND | N209, R237, **D240, D242, W244, E253,** H257, T259, **N265, D266, D267,** Y273 | **P238, E239, P241, D243, Y245, G246, H247, G252, C255,** F258, D260, R263, W264, **V268, R271** | ND |

| | | | | |
|---|---|---|---|---|
| 5E5 – Interaction is representative of indirect inhibition of ligand binding | A | H161, E162, W195, E196, Q198, K199, F200, Q202, H203, H204, G232, F233, K234, N235, W236, R237, P238, D261, G262, R263 | V159, E160, R163, T193, S194, E197, V201, I205, G206, P207, Y229, E230, T231, E239, F258, T259, D260, W264 | W195, K199 |
| 4A2 - Interaction is representative of indirect inhibition of ligand binding | A | R170, W195, E196, K199, Q202, H203, H204, I205, G206, P207, V208, F233, K234, N235, W236, P238, D260, D261, G262, R263, R274 | N157, V159, F168, S169, S171, S194, Q198, F200, V201, T210, R237, E239, Q240, F258, T259, W264 | W195 |
| 7E11 - Interaction is representative of indirect inhibition of ligand binding | A | H161, S194, W195, E196, Q198, K199, F200, Q202, H203, F233, K234, N235, W236, R237, P238, R263 | E160, E162, V192, T193, E197, V201, H204, Y229, E230, T231, G232, E239, Q240, P241, D261, G262, W264 | W195 |
| 4H6 - Interaction is representative of indirect inhibition of ligand binding | A | H161, E162, T193, S194, W195, E196, K199, Q202, T231, G232, F233, K234, N235, P238, D261, R263 | R163, V192, E197, Q198, H203, P207, D228, E230, W236, R237, D260, G262, W264 | ND |
| 22G5 - Interaction is representative of indirect inhibition of ligand binding | B | W167, S171, G172, K173, A174, A176, D177, N180, Y181, R183, L184, E185, D186, Q270, P272, W275 | P155, N157, W158, F168, S169, R170, W175, A178, D179, C182, A187, W211, C269, R271, Y273, R274, C277, T279 | R183, L184, H215, P220, G246, G248, G251, N265 |
| 194A4 - Interaction is representative of indirect inhibition of ligand binding | C | T193, S194, W195, E196, P220, W221, G226, T227, D228, Y229, E230, T231, G232, F233, K234, N235, W236, R237, P238, E239, G252 | H161, E162, V191, V192, E197, Q198, D216, G219, K222, W223, D225, R263, W264 | D260 |
| 72G9 - Interaction is representative of direct inhibition of | C | D216, Q217, N218, G219, P220, W221, Y229, E230, K234, W236, E239, Q240, | H215, K222, T231, G232, R237, P238, H247, G248, E253, C255, D266, V268, | P241, D242, D243, Y245, G251, E253 |

| | | | | |
|---|---|---|---|---|
| ligand binding | | P241, D242, D243, W244, Y245, G246, L249, G250, G251, G252, D254, Q270 | C269 | |
| 54E9 - Interaction is representative of direct inhibition of ligand binding | E | W195, N209, N235, R237, P238, E239, Q240, D242, H257, T259, D260, D261, R263, N265, D267, R271, Y273 | Q198, Q202, P207, V208, F233, W236, D243, E253, F258, G262, W264, D266 | R237, E239, P241, T259, D260, R263, N265 |
| 218G4 - Interaction is representative of direct inhibition of ligand binding | L/O | R170, S171, G172, A174, H204, I205, G206, P207, V208, N209, H257, D260, N265, D267, Q270, R271, P272, Y273, R274 | W167, F168, S169, K173, W175, D177, Y181, Q202, H203, T210, W211, R237, F258, T259, D261, D266, V268, C269, W275 | R171, G172, P238, R274 |
| 176H4 - Interaction is representative of direct inhibition of ligand binding | L/R | R170, S171, G172, K173, A174, D177, P207, V208, N209, R237, Q240, W244, G246, H247, G248, L249, E253, H257, T259, D260, N265, D267, Q270, R271, P272, Y273, R274 | S169, W175, A176, A178, T210, W211, W236, P238, E239, D242, Y245, G250, G251, F258, D261, G262, R263, W264, D266, V268, C269, W275 | G172, P241, D242, H247, L249, N265, R271, P272 |
| 194C10 – Interaction is representative of direct and/or indirect inhibition of ligand binding | L/T | N157, R170, S171, G172, Q202, H203, H204, I205, G206, P207, V208, N209, T210, D260, R271, P272, Y273, R274 | V156, W158, V159, H161, W167, F168, S169, K173, K199, F200, V201, W211, R237, H257, F258, T259, D261, D267, V268, Q270, W275 | R170, G172, V208, R274 |

In some embodiments, the antibody can directly inhibit ASGR-1 CBD/Ligand binding. While described herein in greater detail, and while not intended to be limiting by theory, such an interaction can denote that the antibody interacts with the section of ASGR-1 CBD that binds to its ligand directly, such that a paratope or other section of an antigen binding protein (e.g., antibody) directly obstructs the ligand's access to the binding site in ASGR1 CBD. An antigen binding protein or antibody can be designated as a direct inhibitor when it has one or more of the characteristics of the direct inhibitors provided herein, including the examples below (such as example 10, or the crystal structures referenced therein). Some examples of direct inhibition are shown by 72G9, 54E9, 218G4 and 176H4 and are indicated in Table D. In some embodiments, a direct inhibitor can bind to one or more of residues 237-273 or residues 240-267 of SEQ ID NO:5 of ASGR-1.

In some embodiments, the antigen binding protein or antibody can indirectly inhibit ASGR-1 CBD/Ligand binding. While described herein in greater detail, and while not intended to be limiting by theory, this denotes that the antigen binding protein or antibody binds to ASGR-1 CBD, but need not directly obstruct the ligand's access to the binding site in ASGR-1 CBD. An antigen binding protein or antibody can be designated as an indirect inhibitor when it has one or more of the characteristics of the indirect inhibitors provided herein, including the examples below (such as example 10 or the crystal structures provided therein). Some examples of indirect inhibition are shown by 5E5, 4A2, 7E11, 4H6, 22G5, 194A4, and are indicated in Table D. While not limiting, it is noted that indirect inhibition can occur from a variety of interactions or rearrangements. For example, indirect inhibition may occur from a conformational rearrangement of the carbohydrate binding loop occurs which could impair the carbohydrate binding loop from binding to/interacting with ligand (i.e., carbohydrates). In some embodiments, an indirect inhibitor can bind to one or more of the residues in ASGR-1 CBD helix alpha 1 and/or helix alpha 2. In some embodiments, the antibody binds to ASGR-1 and results in the disordering of the CBD.

In some embodiments, an antigen binding protein or antibody can have characteristics of both direct and indirect inhibition and/or bind to areas on ASGR-1 CBD that are common to both types of inhibition. Of course, such an embodiment may have sufficient inhibition capability through its direct, indirect, or both direct and indirect interactions.

In some embodiments, the distinction between direct and indirect inhibition need not be made. In some embodiments, denoting that an antigen binding protein or antibody provides direct or indirect inhibition means that it provides at least that form of inhibition (e.g., ASGR-1 CBD/Ligand blocking). In some embodiments, an antigen binding protein or antibody that provides direct inhibition, may also provide indirect aspects as well (such as other conformational changes). In addition, as shown in Table D, as the interation between ASGR-1 CBD and its ligands can vary for each of the noted three ligands, what may be a direct or indirect interaction for one ligand, need not be direct or indirect for another. While the antibodies provided herein that have the properties of direct and/or indirect inhibition will function accordingly, and the guidance provided herein allows for one to screen for and produce additional such antibodies, the fact that an antibody simply binds to ASGR-1 CBD does not necessarily mean that it will bind at the relevant locations on ASGR-1 to allow for direct or indirect inhibition.

In some embodiments, an isolated antigen binding protein that binds to human ASGR and inhibits ASGR function is provided. In one embodiment, the invention comprises an isolated antigen binding protein that binds to human ASGR and inhibits ASGR binding to ligand. In another embodiment, the invention comprises an isolated antigen binding protein that binds to human ASGR-1 and inhibits ASGR-1 binding to ligand and/or ASGR-1 interaction with ASGR-2. In another embodiment, the invention comprises an isolated antigen binding protein that binds to human ASGR-2 and inhibits ASGR-2 binding to ligand and/or ASGR-2 interaction with ASGR-1. In yet another embodiment, the invention comprises an isolated antigen binding protein that binds to human ASGR-1 and human ASGR-2, and inhibits ASGR-1 and/or ASGR-2 binding to ligand. In some embodiments, the isolated binding protein binds specifically to human ASGR, ASGR-1 and/or ASGR-2.

In some embodiments, an isolated antigen binding protein is provided, wherein the isolated antigen binding protein binds to human ASGR-1 and comprises one or more VH CDR1, VH CDR2 or VH CDR3 having an amino acid sequence identical to or comprising 1, 2, or 3 amino acid residue substitutions, deletions or insertions in each CDR relative to the VH of any of the sequences set forth in Tables 3-7. In some embodiments, the invention comprises an isolated antigen binding protein, wherein the isolated antigen binding protein binds to human ASGR-1 and comprises one or more VL CDR1, VL CDR2 or VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises one or more VH CDR1, VH CDR2 or VH CDR3 having an amino acid sequence identical to or comprising 1, 2, or 3 amino acid residue substitutions, deletions or insertions in each CDR relative to the VH of any of the sequences set forth in Tables 3-7, and one or more VL CDR1, VL CDR2 or VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises one VH CDR1, VH CDR2 or VH CDR3 having an amino acid sequence identical to or comprising 1, 2, or 3 amino acid residue substitutions, deletions or insertions in each CDR relative to the VH of any of the sequences set forth in Tables 3-7, and one VL CDR1, VL CDR2 or VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises two VH CDR1, VH CDR2 or VH CDR3 having an amino acid sequence identical to or comprising 1, 2, or 3 amino acid residue substitutions, deletions or insertions in each CDR relative to the VH of any of the sequences set forth in Tables 3-7, and two VL CDR1, VL CDR2 or VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises the VH CDR1, VH CDR2 and VH CDR3 having an amino acid sequence identical to or comprising 1, 2, or 3 amino acid residue substitutions, deletions or insertions in each CDR relative to the VH of any of the sequences set forth in Tables 3-7, and the VL CDR1, VL CDR2 and VL CDR3 having an amino acid sequence identical to or comprising 1, 2, or 3 amino acid residue substitutions, deletions or insertions in each CDR relative to the VL of any of the sequences set forth in Tables 3-7. In some embodiments, the isolated antigen binding protein comprises the VH CDR1, VH CDR2, VH CDR3, VL CDR1, VL CDR2, and VL CDR3 having an amino acid sequence identical to any of the sequences set forth in Tables 3-7. In some embodiments, the isolated antigen binding protein comprises the VH CDR1, VH CDR2 or VH CDR3 having an amino acid sequence identical to or comprising 1, 2, or 3 amino acid residue substitutions, deletions or insertions in each CDR relative to the VH of any of the sequences set forth in TABLE A. In some embodiments, the isolated antigen binding

EP 4 435 105 A2

protein comprises the VL CDR1, VL CDR2 or VL CDR3 having an amino acid sequence identical to or comprising 1, 2, or 3 amino acid residue substitutions, deletions or insertions in each CDR relative to the VL of any of the sequences set forth in TABLE A. In some embodiments, the isolated antigen binding protein comprises the VH CDR1, VH CDR2, and VH CDR3 having an amino acid sequence identical to or comprising 1, 2, or 3 amino acid residue substitutions, deletions or insertions in each CDR relative to the VH of any of the sequences set forth in TABLE A, and the VL CDR1, VL CDR2 andVL CDR3, having an amino acid sequence identical to or comprising 1, 2, or 3 amino acid residue substitutions, deletions or insertions in each CDR relative to the VL of any of the sequences set forth in TABLE A. In some embodiments, the isolated antigen binding protein comprises the VH CDR1, VH CDR2, VH CDR3, VL CDR1, VL CDR2, and VL CDR3 having an amino acid sequence identical to any of the sequences set forth in TABLE A. In some embodiments, the isolated antigen binding protein comprises the VH CDR1, VH CDR2 or VH CDR3 having an amino acid sequence identical to or comprising 1, 2, or 3 amino acid residue substitutions, deletions or insertions in each CDR relative to the VH of any of the sequences set forth in TABLE B. In some embodiments, the isolated antigen binding protein comprises the VL CDR1, VL CDR2 or VL CDR3 having an amino acid sequence identical to or comprising 1, 2, or 3 amino acid residue substitutions, deletions or insertions in each CDR relative to the VL of any of the sequences set forth in TABLE B. In some embodiments, the isolated antigen binding protein comprises the VH CDR1, VH CDR2, and VH CDR3 having an amino acid sequence identical to or comprising 1, 2, or 3 amino acid residue substitutions, deletions or insertions in each CDR relative to the VH of any of the sequences set forth in TABLE B, and the VL CDR1, VL CDR2 and VL CDR3 having an amino acid sequence identical to or comprising 1, 2, or 3 amino acid residue substitutions, deletions or insertions in each CDR relative to the VL of any of the sequences set forth in TABLE B. In some embodiments, the isolated antigen binding protein comprises the VH CDR1, VH CDR2, VH CDR3, VL CDR1, VL CDR2, and VL CDR3 having an amino acid sequence identical to any of the sequences set forth in TABLE B. In still some embodiments, the isolated antigen binding protein comprises the VH CDR1, VH CDR2 or VH CDR3 having an amino acid sequence identical to or comprising 1, 2, or 3 amino acid residue substitutions, deletions or insertions in each CDR relative to the VH of any of the sequences set forth in TABLE C. In some embodiments, the isolated antigen binding protein comprises the VL CDR1, VL CDR2 or VL CDR3 having an amino acid sequence identical to or comprising 1, 2, or 3 amino acid residue substitutions, deletions or insertions in each CDR relative to the VL of any of the sequences set forth in TABLE C. In some embodiments, the isolated antigen binding protein comprises the VH CDR1, VH CDR2, and VH CDR3 having an amino acid sequence identical to or comprising 1, 2, or 3 amino acid residue substitutions, deletions or insertions in each CDR relative to the VH of any of the sequences set forth in TABLE C, and the VL CDR1, VL CDR2 and VL CDR3, having an amino acid sequence identical to or comprising 1, 2, or 3 amino acid residue substitutions, deletions or insertions in each CDR relative to the VL of any of the sequences set forth in TABLE C. In some embodiments, the isolated antigen binding protein comprises the VH CDR1, VH CDR2, VH CDR3, VL CDR1, VL CDR2, and VL CDR3 having an amino acid sequence identical to any of the sequences set forth in TABLE C. In further embodiments, the isolated antigen binding protein comprises the VH CDR1, VH CDR2 or VH CDR3 having an amino acid sequence identical to or comprising 1, 2, or 3 amino acid residue substitutions, deletions or insertions in each CDR relative to the VH of any of the sequences set forth in Table 6. In some embodiments, the isolated antigen binding protein comprises the VL CDR1, VL CDR2 or VL CDR3 having an amino acid sequence identical to or comprising 1, 2, or 3 amino acid residue substitutions, deletions or insertions in each CDR relative to the VL of any of the sequences set forth in Table 6. In some embodiments, the isolated antigen binding protein comprises the VH CDR1, VH CDR2, and VH CDR3 having an amino acid sequence identical to or comprising 1, 2, or 3 amino acid residue substitutions, deletions or insertions in each CDR relative to the VH of any of the sequences set forth in Table 6, and the VL CDR1, VL CDR2 and VL CDR3, having an amino acid sequence identical to or comprising 1, 2, or 3 amino acid residue substitutions, deletions or insertions in each CDR relative to the VL of any of the sequences set forth in Table 6. In some embodiments, the isolated antigen binding protein comprises the VH CDR1, VH CDR2, VH CDR3, VL CDR1, VL CDR2, and VL CDR3 having an amino acid sequence identical to any of the sequences set forth in Table 6.

In some embodiments, an isolated antigen binding protein is provided, wherein the antigen binding protein specifically binds human ASGR-1 and comprises a heavy chain variable domain having at least 90% identity to any of the VH domain amino acid sequences set forth in Tables 3-7. In some aspects, the invention provides an isolated antigen binding protein, wherein the antigen binding protein specifically binds human ASGR-1 and comprises a light chain variable domain having at least 90% identity to any of the VL domain amino acid sequences set forth in Tables 3-7. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Tables 3-7, and a light chain variable domain having at least 90% identity to any of the VL domain amino acid sequences set forth in Tables 3-7. In some embodiments, the invention provides an isolated antigen binding protein, wherein the antigen binding protein specifically binds human ASGR-1 and comprises a heavy chain variable domain having any of the VH domain amino acid sequences set forth in Tables 3-7, and a light chain variable domain having any of the VL domain amino acid sequences set forth in Tables 3-7. In some embodiments, the invention provides an isolated antigen binding protein, wherein the antigen binding protein specifically

53

binds human ASGR-1 and comprises a heavy chain variable domain having at least 90% identity to any of the VH domain amino acid sequences set forth in Table A. In some embodiments, the invention provides an isolated antigen binding protein, wherein the antigen binding protein specifically binds human ASGR-1 and comprises a light chain variable domain having at least 90% identity to any of the VL domain amino acid sequences set forth in Table A. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table A, and a light chain variable domain having at least 90% identity to any of the VL domain amino acid sequences set forth in Table A. In some embodiments, the invention provides an isolated antigen binding protein, wherein the antigen binding protein specifically binds human ASGR-1 and comprises a heavy chain variable domain having any of the VH domain amino acid sequences set forth in Table A, and a light chain variable domain having any of the VL domain amino acid sequences set forth in Table A. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table B. In some embodiments, the invention provides an isolated antigen binding protein, wherein the antigen binding protein specifically binds human ASGR-1 and comprises a light chain variable domain having at least 90% identity to any of the VL domain amino acid sequences set forth in Table B. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table B, and a light chain variable domain having at least 90% identity to any of the VL domain amino acid sequences set forth in Table B. In some embodiments, the invention provides an isolated antigen binding protein, wherein the antigen binding protein specifically binds human ASGR-1 and comprises a heavy chain variable domain having any of the VH domain amino acid sequences set forth in Table B, and a light chain variable domain having any of the VL domain amino acid sequences set forth in Table B. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table C. In some embodiments, the invention provides an isolated antigen binding protein, wherein the antigen binding protein specifically binds human ASGR-1 and comprises a light chain variable domain having at least 90% identity to any of the VL domain amino acid sequences set forth in Table C. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table C, and a light chain variable domain having at least 90% identity to any of the VL domain amino acid sequences set forth in Table C. In some embodiments, the invention provides an isolated antigen binding protein, wherein the antigen binding protein specifically binds human ASGR-1 and comprises a heavy chain variable domain having any of the VH domain amino acid sequences set forth in Table C, and a light chain variable domain having any of the VL domain amino acid sequences set forth in Table C. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table 6. In some embodiments, the invention provides an isolated antigen binding protein, wherein the antigen binding protein specifically binds human ASGR-1 and comprises a light chain variable domain having at least 90% identity to any of the VL domain amino acid sequences set forth in Table 6. In some embodiments, the invention provides an isolated antigen binding protein, wherein the antigen binding protein specifically binds human ASGR-1 and comprises a heavy chain variable domain having at least 90% identity to any of the VH domain amino acid sequences set forth in Table 6, and a light chain variable domain having at least 90% identity to any of the VL domain amino acid sequences set forth in Table 6. In some embodiments, the invention provides an isolated antigen binding protein, wherein the antigen binding protein specifically binds human ASGR-1 and comprises a heavy chain variable domain having any of the VH domain amino acid sequences set forth in Table 6, and a light chain variable domain having any of the VL domain amino acid sequences set forth in Table 6.

In some embodiments, an isolated antigen binding protein is provided, wherein the antigen binding protein binds human ASGR-1 and comprises one or more VH CDR1, VH CDR2 or VH CDR3 having an amino acid sequence identical to or comprising no more than 18 amino acid residue substitutions, insertions or deletions in each CDR relative to the VH of any of the sequences set forth in Table 19A as depicted in Figure 55. In some embodiments, the invention provides an isolated antigen binding protein, wherein the antigen binding protein binds human ASGR-1 and comprises one or more VH CDR1, VH CDR2 or VH CDR3 having an amino acid sequence identical to or comprising a conservative subsitutuion of any of the amino acid sequences set forth in Tables 19B or 19C, as depicted in Figure 55. In some aspects, the invention comprises an isolated antigen binding protein, wherein the isolated antigen binding protein binds to human ASGR-1 and comprises one or more VL CDR1, VL CDR2 or VL CDR3 having an amino acid sequence identical to or comprising no more than 14 amino acid residue substitutions, insertions or deletions in each CDR relative to the VL of any of the sequences set forth in Table 20A as depicted in Figure 55. In some embodiments, the invention provides an isolated antigen binding protein, wherein the antigen binding protein binds human ASGR-1 and comprises one or more VL CDR1, VL CDR2 or VL CDR3 having an amino acid sequence identical to or comprising a conservative subsitutuion of any of the amino acid sequences set forth in Tables 20B or 20C, as depicted in Figure 55. In some embodiments, the isolated antigen binding protein comprises one or more VH CDR1, VH CDR2 or VH CDR3 having an amino acid sequence identical to or comprising no more than 18amino acid residue substitutions, inseriors or deletions in each CDR relative to the VH of any of the sequences set forth in Table 19A, as depicted in Figure 55, and one or more VL CDR1, VL CDR2 or VL CDR3 having an amino acid sequence identical to or comprising no more than 14amino acid residue substitutions, insertions or deletions in each CDR relative to the VL of any of the sequences set forth in Table 20A as depicted in Figure 55. In some embodiments, the invention provides an isolated antigen binding protein, wherein the antigen binding protein binds human ASGR-1 and comprises one or more VH CDR1, VH CDR2 or VH CDR3 having an amino acid sequence identical to or comprising a conservative subsitutuion of any of the amino acid sequences set forth in Tables 19B or 19C, as depicted in Figure 55, and one or more VL CDR1, VL CDR2 or VL CDR3 having an amino acid sequence identical to or comprising a conservative subsitutuion of any of the amino acid sequences set forth in Tables 20B or 20C, as depicted in Figure 55. In some embodiments, the isolated antigen binding protein comprises one VH CDR1, VH CDR2 or VH CDR3 having an amino acid sequence identical no more than 18 amino acid residue substitutions, insertions or deletions in each CDR relative to the VH of any of the sequences set forth in Table19A, as depicted in Figure 55, and one VL CDR1, VL CDR2 or VL CDR3 having an amino acid sequence identical to or comprising no more than 14amino acid residue substitutions, insertions or deletions in each CDR relative to the VL of any of the sequences set forth in Table 20A, as depicted in Figure 55. In some embodiments, the invention provides an isolated antigen binding protein, wherein the antigen binding protein binds human ASGR-1 and comprises one VH CDR1, VH CDR2 or VH CDR3 having an amino acid sequence identical to or comprising a conservative subsitutuion of any of the amino acid sequences set forth in Tables 19B or 19C, as depicted in Figure 55, and one VL CDR1, VL CDR2 or VL CDR3 having an amino acid sequence identical to or comprising a conservative subsitutuion of any of the amino acid sequences set forth in Tables 20B or 20C, as depicted in Figure 55. In some embodiments, the isolated antigen binding protein comprises two VH CDR1, VH CDR2 or VH CDR3 having an amino acid sequence identical to or comprising up to 18amino acid residue substitutions, insertions or deletions in each CDR relative to the VH of any of the sequences set forth in Table 19A, as depicted in Figure 55, and two VL CDR1, VL CDR2 or VL CDR3 having an amino acid sequence identical to or comprising up to 14amino acid residue substitutions, insertions or deletions in each CDR relative to the VL of any of the sequences set forth in Table 20A, as depicted in Figure 55. In some embodiments, the invention provides an isolated antigen binding protein, wherein the antigen binding protein binds human ASGR-1 and comprises two VH CDR1, VH CDR2 or VH CDR3 having an amino acid sequence identical to or comprising a conservative subsitutuion of any of the amino acid sequences set forth in Tables 19B or 19C, as depicted in Figure 55, and two VL CDR1, VL CDR2 or VL CDR3 having an amino acid sequence identical to or comprising a conservative subsitutuion of any of the amino acid sequences set forth in Tables 20B or 20C, as depicted in Figure 55. In some embodiments, the isolated antigen binding protein comprises the VH CDR1, VH CDR2 and VH CDR3 having an amino acid sequence identical to or comprising up to 18amino acid residue substitutions, insertions or deletions in each CDR relative to the VH of any of the sequences set forth in Table 19A, as depicted in Figure 55, and the VL CDR1, VL CDR2 and VL CDR3 having an amino acid sequence identical to or comprising up to 14amino acid residue substitutions, insertions or deletions in each CDR relative to the VL of any of the sequences set forth in Table 20A, as depicted in Figure 55. In some embodiments, the invention provides an isolated antigen binding protein, wherein the antigen binding protein binds human ASGR-1 and comprises the VH CDR1, VH CDR2 or VH CDR3 having an amino acid sequence identical to or comprising a conservative subsitutuion of any of the amino acid sequences set forth in Tables 19B or 19C, as depicted in Figure 55, and the VL CDR1, VL CDR2 or VL CDR3 having an amino acid sequence identical to or comprising a conservative subsitutuion of any of the amino acid sequences set forth in Tables 20B or 20C, as depicted in Figure 55.

In some embodiments, an isolated antigen binding protein is provided, wherein the antigen binding protein specifically binds human ASGR-1 and comprises a heavy chain variable domain having at least 90% identity to any of the VH domain amino acid sequences set forth in Tables 19A, as depicted in Figure 55 or in Tables 21-34 as depicted in Figure 56 or in Tables 49-95 as depicted in Figure 56. In some aspects, the invention provides an isolated antigen binding protein, wherein the antigen binding protein specifically binds human ASGR-1 and comprises a light chain variable domain having at least 90% identity to any of the VL domain amino acid sequences set forth in Table 20A, as depicted in Figure 55, or in Tables 35-48, as depicted in Figure 56 or in Tables 96-134 as depicted in Figure 57. In some embodiments, the antigen binding protein comprises a heavy chain variable domain having at least 90% identity to any of the VH domain amino acid sequences set forth in Tables 19A as depicted in Figure 55, or in Tables 21-34 as depicted in Figure 56 or in Tables 49-95 as depicted in Figure 57, and a light chain variable domain having at least 90% identity to any of the VL domain amino acid sequences set forth in Table 20A as depicted in Figure 55 or in Tables 35-48 as depicted in Figure 56 or in Tables 96-134 as depicted in Figure 57. In some embodiments, the antigen binding protein comprises a heavy chain variable domain having any of the VH domain amino acid sequences set forth in Tables 19A as depicted in Figure 55, or in Tables 21-34 as depicted in Figure 56 or in Tables 49-95 as depicted in Figure 57, and a light chain variable domain having any of the VL domain amino acid sequences set forth in Table 20A as depicted in Figure 55 or in Tables 35-48 as depicted in Figure 56 or inTables 96-134 as depicted in Figure 57.

In some embodiments, an antigen binding protein that specifically binds to human ASGR-1 at an epitope that is bound by any of the antigen binding proteins disclosed herein is provided. In some embodiments, the invention provides an isolated antigen binding protein that specifically binds to human ASGR-1 at an epitope that is bound by at least one of the antigen binding proteins set forth in Tables 2-7. In some embodiments, the invention provides an isolated antigen binding protein that specifically binds to human ASGR-1 at an epitope that is bound by at least one of the antigen binding proteins set forth in Table A. In some embodiments, the invention provides an isolated antigen binding protein that specifically binds to human ASGR-1 at an epitope that is bound by at least one of the antigen binding proteins set forth in Table B. In some embodiments, the invention provides an isolated antigen binding protein that specifically binds to human ASGR-1 at an epitope that is bound by at least one of the antigen binding proteins set forth in Table C. In some embodiments, the invention provides an isolated antigen binding protein that specifically binds to human ASGR-1 at an epitope that is bound by at least one of the antigen binding proteins set forth in Table 6.

In some embodiments, the invention provides an isolated antigen binding protein that competes for binding to human ASGR-1 with any of the antigen binding proteins disclosed herein. In some embodiments, the invention provides an isolated antigen binding protein that competes for binding with any of the antigen binding proteins set forth in Tables 2-7. In some embodiments, the invention provides an isolated antigen binding protein that competes for binding with any of the antigen binding proteins set forth in Table A. In some embodiments, the invention provides an isolated antigen binding protein that competes for binding with any of the antigen binding proteins set forth in Table B. In still some embodiments, the invention provides an isolated antigen binding protein that competes for binding with any of the antigen binding proteins set forth in Table C. In yet another embodiment, the invention provides an isolated antigen binding protein that competes for binding with any of the antigen binding proteins set forth in Table 6.

In some embodiments, an isolated antigen binding protein that binds to human ASGR-1 within the carbohydrate recognition domain ("CRD") (also known as the carbohydrate binding domain or "CBD") and inhibits human ASGR-1 binding to ligand is provided. In some embodiments, the antigen binding protein binds to human ASGR-1 within residues 148-291, or 149-291, or 150-291, or 151-291, or 152-291, or 153-291, or 154-291, or 155-291 of SEQ ID NO:5. In some embodiments, the invention comprises an isolated antigen binding protein that binds to human ASGR-1 CBD within Helix α-1. In some embodiments, the invention comprises an isolated antigen binding protein that binds to human ASGR-1 within residues 174-186 of SEQ ID NO:5. In some embodiments, the invention comprises an isolated antigen binding protein that binds to human ASGR-1 CBD within Helix α-2. In some embodiments, the invention comprises an isolated antigen binding protein that binds to human ASGR-1 CBD within residues 194-206 of SEQ ID NO:5. In some embodiments, the invention comprises an isolated antigen binding protein that binds to human ASGR-1 within residues 237-273 or residues 240-267 of SEQ ID NO:5. In some embodiments, the antigen binding protein binds to ASGR-1 having an amino acid sequence that is at least 90% identical to SEQ ID NO:5. In some embodiments, the antigen binding protein is an antibody.

In some embodiments, an isolated antigen binding protein or an antibody that binds to human ASGR-1 and inhibits human ASGR-1 function is provided. In some embodiments, the isolated antigen binding protein or an antibody binds to human ASGR-1 and inhibits human ASGR-1 from binding to a ligand. In some embodiments, the antigen binding protein or antibody or a paratope in an antibody binds to human ASGR-1 at an epitope comprising at least one of the following amino acid residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, Y273, P238, E239, P241, D243, Y245, G246, H247, G252, C255, F258, D260, R263, W264, V268, R271, D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, W275, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, Y273, D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, R274, N157, R170, S171, G172, Q202, H203, H204, I205, G206, P207, V208, N209, T210, D260, R271, P272, Y273, R274 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, Y273, P238, E239, P241, D243, Y245, G246, H247, G252, C255, F258, D260, R263, W264, V268, R271, D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, Y273, D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, or Y273 (SEQ ID NO:5). In some embodiments, the antigen binding protein or antibody or paratope in an antibody binds to human ASGR-1 at an epitope comprising at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, or Y273 (SEQ ID NO:5). In some embodiments, the antigen binding protein or antibody or paratope in an antibody binds to human ASGR-1 at an epitope comprising at least one of the following residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, : H161, E162, V191, V192, E197, Q198, D216, G219, K222, W223, D225, R263, W264, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, W275, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments, the antigen binding protein or antibody or paratope in an antibody binds to human ASGR-1 at an epitope comprising at least one of the following residues: D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, , R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, R274, N157, R170, S171, G172, Q202, H203, H204, I205, G206, P207, V208, N209, T210, D260, R271, P272, Y273, or R274 (SEQ ID NO:5). In some embodiments, the antigen binding protein or antibody or paratope in an antibody binds to human ASGR-1 at an epitope comprising at least one of the following residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments, the antigen binding protein or antibody or paratope in an antibody binds to human ASGR-1 at an epitope comprising at least one of the following residues: D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5). In some embodiments when the antigen binding protein or antibody or paratope in an antibody binds to human ASGR-1 at an epitope comprising at least one of the following residues:, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments, the antigen binding protein or antibody or paratope in an antibody binds to human ASGR-1 at an epitope comprising at least one of the following residues: H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, N157, R170, S171, G172, Q202, H203, H204, I205, G206, P207, V208, N209, T210, D260, R271, P272, Y273, or R274 (SEQ ID NO:5). In some embodiments, the antigen binding protein or antibody or a paratope in an antibody binds to human ASGR-1 at an epitope comprising at least one of the following amino acid residues: H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, or W264 (SEQ ID NO:5). In some embodiments, the antigen binding protein or antibody or a paratope in an antibody binds to human ASGR-1 at an epitope comprising at least one of the following amino acid residues: H161, E162, W195, E196, Q198, K199, F200, Q202, H203, H204, G232, F233, K234, N235, W236, R237, P238, D261, G262, or R263 (SEQ ID NO:5). In some embodiments, the antigen binding protein or antibody or a paratope in an antibody binds to human ASGR1 at an epitope comprising at least one of the following amino acid residues: W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, or T279 (SEQ ID NO:5). In some embodiments, the antigen binding protein or antibody or a paratope in an antibody binds to human ASGR-1 at an epitope comprising at least one of the following amino acid residues: W167, S171, G172, K173, A174, A176, D177, N180, Y181, R183, L184, E185, D186, Q270, P272, or W275 (SEQ ID NO:5). In some embodiments, the antigen binding protein or antibody or a paratope in an antibody binds to human ASGR-1 at an epitope comprising at least one of the following amino acid residues: R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, or W264 (SEQ ID NO:5). In some embodiments, the antigen binding protein or antibody or a paratope in an antibody binds to human ASGR-1 at an epitope comprising at least one of the following amino acid residues: R170, W195, E196, K199, Q202, H203, H204, I205, G206, P207, V208, F233, K234, N235, W236, P238, D260, D261, G262, R263, or R274 (SEQ ID NO:5). In some embodiments, the antigen binding protein or antibody or a paratope in an antibody binds to human ASGR-1 at an epitope comprising at least one of the following amino acid residues: H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, or W264 (SEQ ID NO:5). In some embodiments, the antigen binding protein or antibody or a paratope in an antibody binds to human ASGR-1 at an epitope comprising at least one of the following amino acid residues: H161, S194, W195, E196, Q198, K199, F200, Q202, H203, F233, K234, N235, W236, R237, P238, or R263 (SEQ ID NO:5). In some embodiments, the antigen binding protein or antibody or a paratope in an antibody binds to human ASGR-1 at an epitope comprising at least one of the following amino acid residues: H161, E162, T193, S194, W195, E196, K199, Q202, T231, G232, F233, K234, N235, P238, D261, R263, R163, V192, E197, Q198, H203, P207, D228, E230, W236, R237, D260, G262, or W264 (SEQ ID NO:5). In some embodiments, the antigen binding protein or antibody or a paratope in an antibody binds to human ASGR-1 at an epitope comprising at least one of the following amino acid residues: H161, E162, T193, S194, W195, E196, K199, Q202, T231, G232, F233, K234, N235, P238, D261, or R263 (SEQ ID NO:5). In some embodiments, the antigen binding protein or antibody or a paratope in an antibody binds to human ASGR-1 at an epitope comprising at least one of the following amino acid residues: T193, S194, W195, E196, P220, W221, G226, T227, D228, Y229, E230, T231, G232, F233, K234, N235, W236, R237, P238, E239, G252, : H161, E162, V191, V192, E197, Q198, D216, G219, K222, W223, D225, R263, or W264 (SEQ ID NO:5). In some embodiments, the antigen binding protein or antibody or a paratope in an antibody binds to human ASGR-1 at an epitope comprising at least one of the following amino acid residues: T193, S194, W195, E196, P220, W221, G226, T227, D228, Y229, E230, T231, G232, F233, K234, N235, W236, R237, P238, E239, or G252 (SEQ ID NO:5). In some embodiments, the antigen binding protein or antibody or a paratope in an antibody binds to human ASGR-1 at an epitope comprising at least one of the following amino acid residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, or C269 (SEQ ID NO:5). In some embodiments, the antigen binding protein or antibody or a paratope in an antibody binds to human ASGR-1 at an epitope comprising at least one of the following amino acid residues: D216, Q217, N218, G219, P220, W221, Y229, E230, K234, W236, E239, Q240, P241, D242, D243, W244, Y245, G246, L249, G250, G251, G252, D254, or Q270 (SEQ ID NO:5). In some embodiments, the antigen binding protein or antibody or a paratope in an antibody binds to human ASGR-1 at an epitope comprising at least one of the following amino acid residues: W195, N209, N235, R237, P238, E239, Q240, D242, H257, T259, D260, D261, R263, N265, D267, R271, Y273, Q198, Q202, P207, V208, F233, W236, D243, E253, F258, G262, W264, or D266 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: W195, N209, N235, R237, P238, E239, Q240, D242, H257, T259, D260, D261, R263, N265, D267, R271, or Y273 (SEQ ID NO:5). In some embodiments, the antigen binding protein or antibody or a paratope in an antibody binds to human ASGR-1 at an epitope comprising at least one of the following amino acid residues: N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments, the antigen binding protein or antibody or a paratope in an antibody binds to human ASGR-1 at an epitope comprising at least one of the following amino acid residues: N157, R170, S171, G172, Q202, H203, H204, I205, G206, P207, V208, N209, T210, D260, R271, P272, Y273, or R274 (SEQ ID NO:5). In some embodiments, the antigen binding protein or antibody or a paratope in an antibody binds to human ASGR-1 at an epitope comprising at least one of the following amino acid residues: R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, or W275 (SEQ ID NO:5). In some embodiments, the antigen binding protein or antibody or a paratope in an antibody binds to human ASGR-1 at an epitope comprising at least one of the following amino acid residues: R170, S171, G172, A174, H204, I205, G206, P207, V208, N209, H257, D260, N265, D267, Q270, R271, P272, Y273, or R274 (SEQ ID NO:5). In some embodiments, the antigen binding protein or antibody or a paratope in an antibody binds to human ASGR-1 at an epitope comprising at least one of the following amino acid residues: R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments, the antigen binding protein or antibody or a paratope in an antibody binds to human ASGR-1 at an epitope comprising at least one of the following amino acid residues: R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5).

In some embodiments, an isolated antigen binding protein or an antibody or a paratope in an antibody that specifically binds to human ASGR-1 and inhibits human ASGR-1 function is provided. In some embodiments, the isolated antigen binding protein or an antibody or a paratope in an antibody specifically binds to human ASGR-1 and inhibits human ASGR-1 from binding to a ligand. In some embodiments, the antigen binding protein or antibody or a paratope in an antibody specifically binds to human ASGR-1 within residues 148-291 of SEQ ID NO:5. In some embodiments when the antigen binding protein or antibody or a paratope in an antibody is bound to human ASGR-1, the antigen binding protein or antibody or a paratope in an antibody is positioned 8 angstroms or less from at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, Y273, P238, E239, P241, D243, Y245, G246, H247, G252, C255, F258, D260, R263, W264, V268, R271, D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, W275, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, Y273, D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, R274, N157, R170, S171, G172, Q202, H203, H204, I205, G206, P207, V208, N209, T210, D260, R271, P272, Y273, or R274 (SEQ ID NO:5).

In some embodiments when the antigen binding protein or antibody or a paratope in an antibody is bound to human ASGR-1, the antigen binding protein or antibody or a paratope in an antibody is positioned 8 angstroms or less from at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, Y273, P238, E239, P241, D243, Y245, G246, H247, G252, C255, F258, D260, R263, W264, V268, R271, D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, Y273, D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: Q240, D242, W244, E253, N265, D266, D267, N209, R237, P238, E239, P241, D243, Y245, G246, H247, G252, C255, H257, T259, D260, V268, R271, Y273, R237, Q240, D242, W244, E253, N265, D266, D267, N209, P238, E239, P241, D243, Y245, G246, H247, G252, C255, H257, T259, V268, R271, Y273, N209, R237, Q240, D242, W244, E253, H257, T259, N265, D266, D267, or Y273 (SEQ ID NO:5). In some embodiments when the antigen binding protein or antibody or a paratope in an antibody is positioned 5 angstroms or less from at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, or Y273 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: Q240, D242, W244, E253, N265, D266, D267, R237, Q240, D242, W244, E253, N265, D266, D267, N209, R237, Q240, D242, W244, E253, H257, T259, N265, D266, D267, or Y273 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: Q240, D242, W244, E253, N265, D266, or D267 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: R237, Q240, D242, W244, E253, N265, D266, D267, N209, P238, E239, P241, D243, Y245, G246, H247, G252, C255, H257, T259, V268, R271, or Y273 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: R237, Q240, D242, W244, E253, N265, D266, or D267 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: N209, R237, Q240, D242, W244, E253, H257, T259, N265, D266, D267, Y273, P238, E239, P241, D243, Y245, G246, H247, G252, C255, F258, D260, R263, W264, V268, or R271 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: N209, R237, Q240, D242, W244, E253, H257, T259, N265, D266, D267, or Y273 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, or C269 (SEQ ID NO:5). In some embodiments when the antigen binding protein or antibody is bound to human ASGR-1, the antigen binding protein or antibody or a paratope in an antibody is positioned 5 angstroms or less from at least one of the following residues: D216, Q217, N218, G219, P220, W221, Y229, E230, K234, W236, E239, Q240, P241, D242, D243, W244, Y245, G246, L249, G250, G251, G252, D254, or Q270 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: W195, N209, N235, R237, P238, E239, Q240, D242, H257, T259, D260, D261, R263, N265, D267, R271, Y273, Q198, Q202, P207, V208, F233, W236, D243, E253, F258, G262, W264, or D266 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: W195, N209, N235, R237, P238, E239, Q240, D242, H257, T259, D260, D261, R263, N265, D267, R271, or Y273 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: R170, S171, G172, A174, H204, I205, G206, P207, V208, N209, H257, D260, N265, D267, Q270, R271, P272, Y273, or R274 (SEQ ID NO:5). In some embodiments when the antigen binding protein or antibody or a paratope in an antibody is positioned 8 angstroms or less from at least one of the following residues: R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: R170, S171, G172, K173, A174, D177, P207, V208, N209, R237, Q240, W244, G246, H247, G248, L249, E253, H257, T259, D260, N265, D267, Q270, R271, P272, Y273, or R274 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: D216, Q217, N218, G219, P220, W221, Y229, E230, K234, W236, E239, Q240, P241, D242, D243, W244, Y245, G246, L249, G250, G251, G252, D254, Q270, H215, K222, T231, G232, R237, P238, H247, G248, E253, C255, D266, V268, C269, W195, N209, N235, R237, P238, E239, Q240, D242, H257, T259, D260, D261, R263, N265, D267, R271, Y273, Q198, Q202, P207, V208, F233, W236, D243, E253, F258, G262, W264, D266, H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, R170, S171, G172, A174, H204, I205, G206, P207, V208, N209, H257, D260, N265, D267, Q270, R271, P272, Y273, R274, W167, F168, S169, K173, W175, D177, Y181, Q202, H203, T210, W211, R237, F258, T259, D261, D266, V268, C269, W275, R170, S171, G172, K173, A174, D177, P207, V208, N209, R237, Q240, W244, G246, H247, G248, L249, E253, H257, T259, D260, N265, D267, Q270, R271, P272, Y273, R274, S169, W175, A176, A178, T210, W211, W236, P238, E239, D242, Y245, G250, G251, F258, D261, G262, R263, W264, D266, V268, C269, W275, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues of human ASGR-1 (SEQ ID NO:5): D216, Q217, N218, G219, P220, W221, Y229, E230, K234, W236, E239, Q240, P241, D242, D243, W244, Y245, G246, L249, G250, G251, G252, D254, Q270, W195, N209, N235, R237, P238, E239, Q240, D242, H257, T259, D260, D261, R263, N265, D267, R271, Y273, H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, R170, S171, G172, A174, H204, I205, G206, P207, V208, N209, H257, D260, N265, D267, Q270, R271, P272, Y273, R274, R170, S171, G172, K173, A174, D177, P207, V208, N209, R237, Q240, W244, G246, H247, G248, L249, E253, H257, T259, D260, N265, D267, Q270, R271, P272, Y273, R274, N157, R170, S171, G172, Q202, H203, H204, I205, G206, P207, V208, N209, T210, D260, R271, P272, Y273, or R274 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, W264, W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, T279, R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, W264, H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, W264, H161, E162, T193, S194, W195, E196, K199, Q202, T231, G232, F233, K234, N235, P238, D261, R263, R163, V192, E197, Q198, H203, P207, D228, E230, W236, R237, D260, G262, or W264, T193, S194, W195, E196, P220, W221, G226, T227, D228, Y229, E230, T231, G232, F233, K234, N235, W236, R237, P238, E239, G252, H161, E162, V191, V192, E197, Q198, D216, G219, K222, W223, D225, R263, W264, N157, R170, S171, G172, Q202, H203, H204, I205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270, or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: H161, E162, W195, E196, Q198, K199, F200, Q202, H203, H204, G232, F233, K234, N235, W236, R237, P238, D261, G262, R263, W167, S171, G172, K173, A174, A176, D177, N180, Y181, R183, L184, E185, D186, Q270, P272, W275, R170, W195, E196, K199, Q202, H203, H204, I205, G206, P207, V208, F233, K234, N235, W236, P238, D260, D261, G262, R263, R274, H161, S194, W195, E196, Q198, K199, F200, Q202, H203, F233, K234, N235, W236, R237, P238, R263, H161, E162, T193, S194, W195, E196, K199, Q202, T231, G232, F233, K234, N235, P238, D261, R263, T193, S194, W195, E196, P220, W221, G226, T227, D228, Y229, E230, T231, G232, F233, K234, N235, W236, R237, P238, E239, G252, N157, R170, S171, G172, Q202, H203, H204, I205, G206, P207, V208, N209, T210, D260, R271, P272, Y273, R274, V156, W158, V159, H161, W167, F168, S169, K173, (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: H161, E162, W195, E196, Q198, K199, F200, Q202, H203, H204, G232, F233, K234, N235, W236, R237, P238, D261, G262, R263, V159, E160, R163, T193, S194, E197, V201, I205, G206, P207, Y229, E230, T231, E239, F258, T259, D260, or W264 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues of human ASGR-1 (SEQ ID NO:5): H161, E162, W195, E196, Q198, K199, F200, Q202, H203, H204, G232, F233, K234, N235, W236, R237, P238, D261, G262, or R263 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: W167, S171, G172, K173, A174, A176, D177, N180, Y181, R183, L184, E185, D186, Q270, P272, W275, P155, N157, W158, F168, S169, R170, W175, A178, D179, C182, A187, W211, C269, R271, Y273, R274, C277, or T279 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: W167, S171, G172, K173, A174, A176, D177, N180, Y181, R183, L184, E185, D186, Q270, P272, or W275 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: R170, W195, E196, K199, Q202, H203, H204, I205, G206, P207, V208, F233, K234, N235, W236, P238, D260, D261, G262, R263, R274, N157, V159, F168, S169, S171, S194, Q198, F200, V201, T210, R237, E239, Q240, F258, T259, or W264 (SEQ ID NO:5). In some embodiments when the antigen binding protein or antibody or a paratope in an antibody is bound to human ASGR-1, the antigen binding protein or or a paratope in an antibody is positioned 5 angstroms or less from at least one of the following residues: R170, W195, E196, K199, Q202, H203, H204, I205, G206, P207, V208, F233, K234, N235, W236, P238, D260, D261, G262, R263, or R274 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: H161, S194, W195, E196, Q198, K199, F200, Q202, H203, F233, K234, N235, W236, R237, P238, R263, E160, E162, V192, T193, E197, V201, H204, Y229, E230, T231, G232, E239, Q240, P241, D261, G262, or W264 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: H161, S194, W195, E196, Q198, K199, F200, Q202, H203, F233, K234, N235, W236, R237, P238, or R263 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: H161, E162, T193, S194, W195, E196, K199, Q202, T231, G232, F233, K234, N235, P238, D261, R263, R163, V192, E197, Q198, H203, P207, D228, E230, W236, R237, D260, G262, or W264 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: H161, E162, T193, S194, W195, E196, K199, Q202, T231, G232, F233, K234, N235, P238, D261, or R263 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: T193, S194, W195, E196, P220, W221, G226, T227, D228, Y229, E230, T231, G232, F233, K234, N235, W236, R237, P238, E239, G252, : H161, E162, V191, V192, E197, Q198, D216, G219, K222, W223, D225, R263, or W264 (SEQ ID NO:5). In some embodiments when the antigen binding protein or antibody or a paratope in an antibody is positioned 5 angstroms or less from at least one of the following residues: T193, S194, W195, E196, P220, W221, G226, T227, D228, Y229, E230, T231, G232, F233, K234, N235, W236, R237, P238, E239, or G252 (SEQ ID NO:5). In some embodiments when the antigen binding protein or antibody or a paratope in an antibody is bound to human ASGR-1, the antigen binding protein or antibody or a paratope in an antibody is positioned 8 angstroms or less from at least one of the following residues: N157, R170, S171, G172, Q202, H203, H204, 1205, G206, P207, V208, N209, T210, D260, R271, P272, Y273, R274, V156, W158, V159, H161, W167, F168, S169, K173, K199, F200, V201, W211, R237, H257, F258, T259, D261, D267, V268, Q270 or W275 (SEQ ID NO:5). In some embodiments when the antigen binding protein or antibody or a paratope in an antibody is bound to human ASGR-1, the antigen binding protein or antibody or a paratope in an antibody is positioned 5 angstroms or less from at least one of the following residues: N157, R170, S171, G172, Q202, H203, H204, I205, G206, P207, V208, N209, T210, D260, R271, P272, Y273 or R274 (SEQ ID NO:5).

In some embodiments, an isolated antigen binding protein or antibody that specifically binds to human ASGR-1 and inhibits human ASGR-1 function is provided. In some embodiments, the isolated antigen binding protein or antibody that specifically binds to human ASGR-1 inhibits binding of human ASGR-1 binding to a ligand. In some embodiments, the antigen binding protein or antibody specifically binds to human ASGR-1 at a location that overlaps with a location where a ligand binds to human ASGR-1. In some embodiments, the location where a ligand binds to ASGR-1 includes at least one amino acid residue selected from the group consisting of N209, R237, Q240, D242, W244, E253, H257, T259, N265, D266, D267, Y273, P238, E239, P241, D243, Y245, G246, H247, G252, C255, F258, D260, R263, W264, V268, or R271 (SEQ ID NO:5). In some embodiments, an isolated antigen binding protein or an antibody specifically binds to human ASGR-1 at a location that overlaps with a location that a ligand binds to ASGR-1. In some embodiments, the location that a ligand binds to human ASGR-1 includes at least one amino acid residue selected from the group consisting of: N209, R237, Q240, D242, W244, E253, H257, T259, N265, D266, D267, and Y273 (SEQ ID NO:5).

In some embodiments, an isolated antigen binding protein that binds to human ASGR-1 and inhibits human ASGR, ASGR-1 and/or ASGR-2 function is provided, wherein the antigen binding protein does not bind to a variant ASGR-1 protein, and wherein said variant ASGR-1 protein comprises a single mutation of a residue selected the group consisting of R170, S171, G172, R183, L184, W195, E196, K199, H203, H204, P207, V208, N209, H215, D216, P220, D225, D228, R237, P238, E239, P241, D242, D243, Y245, G246, H247, G248, L249, G251, E253, T259, D260, R263, N265, Q270, R271, P272, R274, and E280 as shown in SEQ ID NO:5. In some embodiments, an isolated antigen binding protein or an antibody is contemplated. An antigen binding protein "does not bind" to a variant ASGR-1 protein when the measured reduction in antibody binding signal to a variant ASGR-1 protein (compared to that determined for binding to wild type ASGR-1) is statistically significant as measured by any number of methods known to one skilled in the art, such as the method described in Example 7E below. In some embodiments, the variant ASGR-1 protein comprises a single mutation of a residue at a position selected from the group consisting of: W195, E196, K199, H203, H204, P207, P220, G251, and R263 as shown in SEQ ID NO:5. In some embodiments, the single mutation is selected from the group consisting of H203, H204, P220, and G251. In some embodiments, the single mutation is selected from the group consisting of W195, E196 and K199. In some embodiments, the single mutation is selected from the group consisting of W195, E196 and H204. In some embodiments, the single mutation is selected from the group consisting W195, K199, and R263. In some embodiments, the single mutation is selected from the group consisting of W195 and E196. In some embodiments, the single mutation is selected from the group consisting of W195 and K199. In some embodiments, the single mutation is selected from the group consisting of W195 or P207. In some embodiments, the single mutation is selected from the group consisting of W195 and R263. In some embodiments, the single mutation is selected from the group consisting of H203 and H204. In some embodiments, the single mutation is selected from the group consisting of K199 and R263. In some embodiments, the single mutation is a mutation of residue W195. In some embodiments, the variant ASGR-1 protein comprises a single mutation of a residue selected the group consisting of R170, S171, R183, L184, H215, P220, P238, G246, H247, G248, G251, and N265 as shown in SEQ ID NO:5. In some embodiments, the single mutation is selected from the group consisting of R183, L184, H215, P220, G246, G248, G251, and N265. In some embodiments, the single mutation is selected from the group consisting of L184, P220, P238, H247, and G251. In some embodiments, the single mutation is selected from the group consisting of R170, S171, and L184. In some embodiments, the single mutation is a mutation of residue R183. In some embodiments, the single mutation is a mutation of residue L184. In some embodiments, the variant ASGR-1 protein comprises a single mutation of a residue at a position selected from the group consisting of: P241, D242, D243, Y245, G251, E253 and D260 as shown in SEQ ID NO:5. In some embodiments, the single mutation is selected from the group consisting of P241, D243, Y245, G251, E253 and D260. In some embodiments, the single mutation is selected from the group consisting of P241, D243, and E253. In some embodiments, the single mutation is a mutation of residue D260. In some embodiments, the variant ASGR-1 protein comprises a single mutation of a residue at a position selected from the group consisting or comprising:_R170, R237, E239, P241, T259, D260, R263, and N265 as shown in SEQ ID NO:5. In some embodiments, the single mutation is selected from the group consisting of R237, D260 and R263. In some embodiments, the single mutation is selected from the group consisting of R237, T259, D260 and R263. In some embodiments, the single mutation is selected from the group consisting of R170, R237, P241, T259, D260, R263 and N265. In some embodiments, the single mutation is selected from the group consisting of R237, E239, P241, T259, D260, R263 and N265. In some embodiments, the variant ASGR-1 protein comprises a single mutation of a residue at a position selected from the group consisting or comprising: R170, S171, G172, E196, H204, P207, V208, N209, H215, D216, D225, D228, P238, P241, D242, D243, H247, G248, L249, G251, D260, R263, N265, Q270, R271, P272, R274 and E280 as shown in SEQ ID NO:5. In some embodiments, the single mutation is selected from the group consisting of R170, S171, G172, E196, H204, P207, V208, N209, H215, D216, D225, D228, P238, P241, D242, D243, H247, G248, L249, G251, D260, R263, N265, Q270, R271, P272, R274 and E280 as shown in SEQ ID NO:5 . In some embodiments, the single mutation is selected from the group consisting ofR170, S171, G172, E196, H204, P207, H215, D216, D225, D228, D243, G248, L249, G251, D260, Q270, R271, P272, R274 and E280. In some embodiments, the single mutation is selected from the group consisting of G172, V208, R271, P272 and R274. In some embodiments, the single mutation is selected from the group consisting of G172, R271 and R274. In some embodiments, the single mutation is selected from the group consisting of G172, N209, and R271. In some embodiments, the single mutation is selected from the group consisting of R170, G172, V208, R271 and P272. In some embodiments, the single mutation is selected from the group consisting of G172, V208, P238, R271, P272 and R274. In some embodiments, the single mutation is selected from the group consisting of G172, P238, R271, P272 and R274. In some embodiments, the variant ASGR-1 protein comprises a single mutation of a residue at a position selected from the group consisting or comprising:_G172, P238, R271 and R274 as shown in SEQ ID NO:5. In some embodiments, the variant ASGR-1 protein comprises a single mutation of a residue at a position selected from the group consisting or comprising: R170, G172, V208 and R274 as shown in SEQ ID NO:5. In some embodiments, the variant ASGR-1 protein comprises a single mutation of a residue at a position selected from the group consisting or comprising: R170, R183, H215 and Q270 as shown in SEQ ID NO:5. In some embodiments, the variant ASGR-1 protein comprises a single mutation of a residue at a position selected from the group consisting or comprising: P241, T259, and N265 as shown in SEQ ID NO:5. In some embodiments, the variant ASGR-1 protein comprises a single mutation of a residue at a position selected from the group consisting or comprising: P207 and R263 as shown in SEQ ID NO:5. In some embodiments, the variant ASGR-1 protein comprises a single mutation of a residue at a position selected from the group consisting or comprising: G172, P241, D242, H247, L249, N265, R271 and P272 as shown in SEQ ID NO:5. In some embodiments, the antigen binding protein or antibody does not bind to two or more variant ASGR-1 proteins, wherein the variant ASGR-1 proteins comprise the single mutations of the group individually.

A "CDR grafted antibody" is an antibody comprising one or more CDRs derived from an antibody of a particular species or isotype and the framework of another antibody of the same or different species or isotype.

A "multi-specific antibody" is an antibody that recognizes more than one epitope on one or more antigens. A subclass of this type of antibody is a "bi-specific antibody" which recognizes two distinct epitopes on the same or different antigens.

An antigen binding protein including an antibody "specifically binds" to an antigen, such as ASGR, ASGR-1 or ASGR-2, if it binds to the antigen with a tight binding affinity as determined by a equilibrium dissociation constant ($K_D$, or corresponding $K_D$, as defined below) value of $10^{-7}$ M or less. An antigen binding protein that specifically binds to human ASGR, ASGR-1 or ASGR-2 may be able to bind to ASGR, ASGR-1 or ASGR-2 from other species as well with the same or different affinities.

Affinity can be determined using a variety of techniques known in the art, for example but not limited to, equilibrium methods (e.g., enzyme-linked immunoabsorbent assay (ELISA); KinExA, Rathanaswami et al. Analytical Biochemistry, Vol. 373:52-60, 2008; or radioimmunoassay (RIA)), or by a surface plasmon resonance assay or other mechanism of kinetics-based assay (e.g., BIACORE® analysis or Octet® analysis (forteBIO)), and other methods such as indirect binding assays, competitive binding assays fluorescence resonance energy transfer (FRET), gel electrophoresis and chromatography (e.g., gel filtration). These and other methods may utilize a label on one or more of the components being examined and/or employ a variety of detection methods including but not limited to chromogenic, fluorescent, luminescent, or isotopic labels. A detailed description of binding affinities and kinetics can be found in Paul, W. E., ed., Fundamental Immunology, 4th Ed., Lippincott-Raven, Philadelphia (1999), which focuses on antibody-immunogen interactions. One example of a competitive binding assay is a radioimmunoassay comprising the incubation of labeled antigen with the antibody of interest in the presence of increasing amounts of unlabeled antigen, and the detection of the antibody bound to the labeled antigen. The affinity of the antibody of interest for a particular antigen and the binding off-rates can be determined from the data by scatchard plot analysis. Competition with a second antibody can also be determined using radioimmunoassays. In this case, the antigen is incubated with antibody of interest conjugated to a labeled compound in the presence of increasing amounts of an unlabeled second antibody.

Further embodiments of the invention provide antigen binding molecules (e.g., antibodies) that specifically bind ASGR, ASGR-1 and/or ASGR-2 with an equilibrium dissociation constant or $K_D$ ($k_{off}/k_{on}$) of less than $10^{-7}$ M, or of less than $10^{-8}$ M, or of less than $10^{-9}$ M, or of less than $10^{-10}$ M, or of less than $10^{-11}$ M, or of less than $10^{-12}$ M, or of less than $10^{-13}$ M, or of less than $5 \times 10^{-13}$ M (lower values indicating tighter binding affinity). Yet further embodiments of the invention are antigen binding molecules that specifically bind ASGR, ASGR-1 and/or ASGR-2 with an equilibrium dissociation constant or $K_D$ ($k_{off}/k_{on}$) of less than about $10^{-7}$ M, or of less than about $10^{-8}$ M, or of less than about $10^{-9}$ M, or of less than about $10^{-10}$ M, or of less than about $10^{-11}$ M, or of less than about $10^{-12}$ M, or of less than about $10^{-13}$ M, or of less than about $5 \times 10^{-13}$ M.

In still another embodiment, an antigen binding protein of the invention (e.g., an antibody) that specifically bind ASGR, ASGR-1 and/or ASGR-2 has an equilibrium dissociation constant or $K_D$ ($k_{off}/k_{on}$) of between about $10^{-7}$ M and about $10^{-8}$ M, between about $10^{-8}$ M and about $10^{-9}$ M, between about $10^{-9}$ M and about $10^{-10}$ M, between about $10^{-10}$ M and about $10^{-11}$ M, between about $10^{-11}$ M and about $10^{-12}$ M, between about $10^{-12}$ M and about $10^{-13}$ M. In still another embodiment, an antibody of the invention that specifically bind ASGR, ASGR-1 and/or ASGR-2 has an equilibrium dissociation constant or $K_D$ ($k_{off}/k_{on}$) of between $10^{-7}$ M and $10^{-8}$ M, between $10^{-8}$ M and $10^{-9}$ M, between $10^{-9}$ M and $10^{-10}$ M, between $10^{-10}$ M and $10^{-11}$ M, between $10^{-11}$ M and $10^{-12}$ M, between $10^{-12}$ M and $10^{-13}$ M.

It will be appreciated that an antigen binding protein of the present invention (e.g., an antibody or fragments thereof) may have at least one amino acid substitution, providing that the antigen binding protein retains the same or better desired binding specificity (e.g., binding to human ASGR, human ASGR-1, and/or human ASGR-2)(See Example 14). Therefore, modifications to the antigen binding protein structures are encompassed within the scope of the invention. In one embodiment, the antigen binding protein (e.g., but not limited to, an antibody) comprises sequences that each independently differ by 5, 4, 3, 2, 1, or 0 single amino acid additions, substitutions, and/or deletions from a CDR sequence of those set forth in Table 2 herein. As used herein, a CDR sequence that differs by no more than a total of, for example, four amino acid additions, substitutions and/or deletions from a CDR sequence shown in Table 2 below refers to a sequence with 4, 3, 2, 1 or 0 single amino acid additions, substitutions, and/or deletions compared with the sequences shown in Table 2. These may include amino acid substitutions, which may be conservative or non-conservative that do not destroy the desired binding capability of an antibody. Conservative amino acid substitutions may encompass non-naturally occurring amino acid residues, which are typically incorporated by chemical peptide synthesis rather than by synthesis in biological systems. These include peptidomimetics and other reversed or inverted forms of amino acid moieties. A conservative amino acid substitution may also involve a substitution of a native amino acid residue with a normative residue such that there is little or no effect on the polarity or charge of the amino acid residue at that position. In some embodiments, the one or more substitutions to one or more of the antibody sequences can be as follows for each noted section in the noted antibody: 1) VH1|1-08/D6|6-19|RF1/JH4, 25A4 H CDR2 sequence - WMYPN --- SGNTGYAQKFQG, where N at 11 can be S or Q and T at 12 can be A or V, such that the sequence can be Trp Met Tyr Pro Asn Ser Gly X1 X2 Gly Tyr Ala Gin Lys Phe Gln Gly (SEQ ID NO: 50259) wherein X1 = N or S or Q or a conservative substitution thereof, X2 = T or A or V or a conservative substitution thereof. 2) VH1|1-08/D6|6-19|RF1/JH4, 4A2 H CDR2 sequence - WMHPN --- SGNTGYAQKFQG, where N at 11 can be S or Q, and T at 12 can be A or E, such that the sequence can be Trp Met His Pro Asn Ser Gly X1 X2 Gly Tyr Ala Gin Lys Phe Gln Gly (SEQ ID NO: 50260) wherein X1 = N or S or Q or a conservative substitution thereof, X2 = T or A or E or a conservative substitution thereof. 3) VK4|B3/JK3, 4A2 L CDR3 sequence - QQYYN----------------------TPVT, where N at 5 can be Q, and T at 29 can be A, such that the sequence can be Gin Gln Tyr Tyr X1 X2 Pro Val Thr (SEQ ID NO: 50261) wherein X1 = N or Q or a conservative substitution thereof, X2 = T or A or a conservative substitution thereof. 4) VH1|1-02/D1|1-1|RF1/JH4, 4H6 H CDR3 sequence - DGTS--SFDY, where D at 1 can be S, G at 2 can be A, such that the sequence can be X1 X2 Thr Ser Ser Phe Asp Tyr (SEQ ID NO: 50262) wherein X1 = D or S or a conservative substitution thereof, X2 = or A or a conservative substitution thereof. 5) VH313-33/D414-11IRF2/JH6 and VH3|3-07/D4|4-11|RF2/JH6, 7E11 H CDR2 sequence - IIWHD---GSNKYY ADSVKG, where D at 5 can be S or E, G at 9 can be A, D at 16 can be E, and S at 17 can be A, such that the sequence can be Ile Ile Trp His X1 X2 Ser Asn Lys Tyr Tyr Ala X3 X4 Val Lys Gly (SEQ ID NO: 50263) wherein X1 = D or S or E or a conservative substitution thereof, X2 = G or A or a conservative substitution thereof, X3 = D or E or a conservative substitution thereof, X4 = S or A or a conservative substitution thereof. 6) VH3|3-33/D6|6-6|RF1/JH6 and VH3|3-07/D6|6-6|RF1/JH6, 5E5 H CDR2 sequence VIWYD---GSNKYYADSVKG, where G at 9 can be A, D at 16 can be E or G, and S at 17 can be A, such that the sequence can be Val Ile Trp Tyr Asp X1 Ser Asn Lys Tyr Tyr Ala X2 X3 Val Lys Gly (SEQ ID NO: 50264) wherein X1 = G or A or a conservative substitution thereof X2 = D or E or G or a conservative substitution thereof X3 = S or A or a conservative substitution thereof. 7) VH3|3-33/D6|6-6|RF1/JH6 and VH3|3-07/D6|6-6|RF1/JH6, 5E5 H CDR3 sequence EVYSSGW----------------YDYGMDV, where W at 7 can be F, such that the sequence can be Glu Val Tyr Ser Ser Gly X1 Tyr Asp Tyr Gly Met Asp Val (SEQ ID NO: 50265) wherein X1 = W or F or a conservative substitution thereof.

In some embodiments, any one or more of the above CDR sequences can be combined with any one or more of the CDR sequences provided herein (e.g., Table 2 in Figure 49, and Tables 19A-C and Tables 20A-C in Figure 55). In some embodiments, any one or more of the above CDR sequences can be combined with any one or more CDR sequences provided herein for the designated antibody to provide an antibody of 6 CDRs (LCDR1, LCDR2, LCDR3, HCDR1, HCDR2, and HCDR3). For example, any one or more of the above CDRs can be used as one of the CDRs for the antibodies provided in Table 2 in Figure 49 and/or Tables 19A, 19B, 19C, 20A, 20B and/or 20C in Figure 55. In some embodiments, the variant positions provided in the above consensus sequences can be further combined as optional variations with the variations of sequence provided in Table 2 in Figure 49, and Tables 19A-C and Tables 20A-C in Figure 55, such that any demonstrated combination of sequences in one consensus sequence (e.g., for an antibody, such as 4A2 H CDR2 above) can be combined with all permissible options outlined for the relevant antibody in Table 2 in Figure 49, and Tables 19A-C and Tables 20A-C in Figure 55 (e.g., the corresponding 4A2 H CDR2), which can further be combined with any of the other 4A2 sequences in Table 2 in Figure 49, and Tables 19A-C and Tables 20A-C in Figure 55 (e.g., HCDR1, HCDR3, LCDR1, LCDR2, and LCDR3). Of course, 4A2 L CDR3 noted above can similarly be combined, and/or combined with the immediate combination as well. Thus, such sequences are not disclosed herein as needing to be alternative sequences, but are contemplated as additional options for the noted sequences. In some embodiments, variants of such sequences are also contemplated. Such variants can retain or have superior desired activity. Examples of such aspects are provided in Example 14 and tables 6 and 7. In some embodiments, any one or more of the FR regions in tables 6 and 7 can be combined with any one or more of the CDR sequences provided herein. In some embodiments, any one or more of the FR regions provided in Table 6 or 7 can be combined with the corresponding CDR set for the corresponding antibody (as a set of 6 CDRs). Thus, variants of antibody 4A2 are provided that include 6 CDRs (HCDR1, HCDR2, HCDR3, LCDR1, LCDR2, and LCDR3) and 8 FRs HFR1, HFR2, HFR3, HFR4, LFR1, LFR2, LFR3, and LFR4), any particular sequence of which can be from any of the designated sequecnes for antibody 4A2 provided herein (the present paragraph, Tables 2, 6 and/or 7, tables 19A, 19B, and 19C, 20A, 20B and 20C, etc).

Non-conservative substitutions may involve the exchange of a member of one class of amino acids or amino acid mimetics for a member from another class with different physical properties (e.g. size, polarity, hydrophobicity, charge). In certain embodiments, such substituted residues may be introduced into regions of a human antibody that are homologous with non-human antibodies, or into the non-homologous regions of the molecule.

Moreover, one skilled in the art may generate test variants containing a single amino acid substitution at each desired amino acid residue. The variants can then be screened using activity assays known to those skilled in the art. Such variants could be used to gather information about suitable variants. For example, if one discovered that a change to a particular amino acid residue resulted in destroyed, undesirably reduced, or unsuitable activity, variants with such a change may be avoided. In other words, based on information gathered from such routine experiments, one skilled in the art can readily determine the amino acids where further substitutions should be avoided either alone or in combination with other mutations.

A skilled artisan will be able to determine suitable variants of the antigen binding protein as set forth herein using well-known techniques. In certain embodiments, one skilled in the art may identify suitable areas of the molecule that may be changed without destroying activity by targeting regions not believed to be important for activity. In certain embodiments, one can identify residues and portions of the molecules that are conserved among similar polypeptides as has been describe above. In certain embodiments, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in a protein that correspond to amino acid residues which are important for activity or structure in similar proteins. One skilled in the art may opt for chemically similar amino acid substitutions for such predicted important amino acid residues.

In some embodiments, one skilled in the art may identify residues that may be changed that result in enhanced properties as desired. For example, an amino acid substitution (conservative or non-conservative) may result in enhanced binding affinity to human ASGR, human ASGR-1, and/or human ASGR-2, or enhanced binding affinity to other species of ASGR, ASGR-1, and/or ASGR-2.

One skilled in the art can also analyze the three-dimensional structure and amino acid sequence in relation to that structure in similar polypeptides. In view of such information, one skilled in the art may predict the alignment of amino acid residues of an antibody with respect to its three dimensional structure. In certain embodiments, one skilled in the art may choose not to make radical changes to amino acid residues predicted to be on the surface of the protein, since such residues may be involved in important interactions with other molecules. A number of scientific publications have been devoted to the prediction of secondary structure. See Moult J., Curr. Op. in Biotech., 7(4):422-427 (1996), Chou et al., Biochemistry, 13(2):222-245 (1974); Chou et al., Biochemistry, 113(2):211-222 (1974); Chou et al., Adv. Enzymol. Relat. Areas Mol. Biol., 47:45-148 (1978); Chou et al., Ann. Rev. Biochem., 47:251-276 and Chou et al., Biophys. J., 26:367-384

(1979). Moreover, computer programs are currently available to assist with predicting secondary structure. One method of predicting secondary structure is based upon homology modeling. For example, two polypeptides or proteins which have a sequence identity of greater than 30%, or similarity greater than 40% often have similar structural topologies. The growth of the protein structural database (PDB) has provided enhanced predictability of secondary structure, including the potential number of folds within a polypeptide's or protein's structure. See Holm et al., Nucl. Acid. Res., 27(1):244-247 (1999). Additional methods of predicting secondary structure include "threading" (Jones, D., Curr. Opin. Struct. Biol., 7(3):377-87 (1997); Sippl et al., Structure, 4(1):15-19 (1996)), "profile analysis" (Bowie et al., Science, 253:164-170 (1991); Gribskov et al., Meth. Enzym., 183:146-159 (1990); Gribskov et al., Proc. Nat. Acad. Sci., 84(13):4355-4358 (1987)), and "evolutionary linkage" (See Holm, supra (1999), and Brenner, supra (1997)).

In certain embodiments, variants of the antigen binding protein include glycosylation variants wherein the number and/or type of glycosylation site has been altered compared to the amino acid sequences of a parent polypeptide. In certain embodiments, variants comprise a greater or a lesser number of N-linked glycosylation sites than the native protein. Alternatively, substitutions which eliminate this sequence will remove an existing N-linked carbohydrate chain. Also provided is a rearrangement of N-linked carbohydrate chains wherein one or more N-linked glycosylation sites (typically those that are naturally occurring) are eliminated and one or more new N-linked sites are created. Additional antibody variants include cysteine variants wherein one or more cysteine residues are deleted from or substituted for another amino acid (e.g., serine) as compared to the parent amino acid sequence. Cysteine variants may be useful when antibodies must be refolded into a biologically active conformation such as after the isolation of insoluble inclusion bodies. Cysteine variants generally have fewer cysteine residues than the native protein, and typically have an even number to minimize interactions resulting from unpaired cysteines.

Desired amino acid substitutions (whether conservative or non-conservative) can be determined by those skilled in the art at the time such substitutions are desired. In certain embodiments, amino acid substitutions can be used to identify important residues of antibodies to the target of interest, or to increase or decrease the affinity of the antibodies to the target of interest described herein.

According to certain embodiments, desired amino acid substitutions are those which: (1) reduce susceptibility to proteolysis, (2) reduce susceptibility to oxidation, (3) alter binding affinity for forming protein complexes, (4) alter binding affinities, and/or (4) confer or modify other physiochemical or functional properties on such polypeptides. According to certain embodiments, single or multiple amino acid substitutions (in certain embodiments, conservative amino acid substitutions) may be made in the naturally-occurring sequence (in certain embodiments, in the portion of the polypeptide outside the domain(s) forming intermolecular contacts). In certain embodiments, a conservative amino acid substitution typically may not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et al. Nature 354:105 (1991), which are each incorporated herein by reference.

Antigen Binding Protein Sequences

The amino acid sequences of the light chain CDRs of exemplary antigen binding proteins (antibodies) and the heavy chain CDRs of exemplary antigen binding proteins (antibodies) are shown in Tables 2-7, in addition to the exemplary antigen binding proteins described above as consensus light chain CDRs and/or consensus heavy chain CDRs (see Tables 19 B and C and Tables 20 B and C in Figure 55). Also shown are polynucleotide sequences which encode the amino acid sequences of the CDRs (Table 2). Tables 3-7 and Tables A, B and C further provide the amino acid sequences of the VH and VL of exemplary antigen binding proteins (e.g., antibodies), in addition to the exemplary antigen binding proteins described above as consensus variable light chain sequences and/or consensus variable heavy chain sequences (see Table 19A and Table 20A in Figure 55, as well as the Tables in Figures 56 and 57). Table 3 further provides the polynucleotide (DNA) sequences encoding the amino acid sequences of the variable light and variable heavy domains for exemplary antibodies.

Particular embodiments of antigen binding proteins of the present invention comprise one or more amino acid sequences that are identical to the amino acid sequences of one or more of the CDRs and/or FRs (framework regions) illustrated herein in Tables 2-7, and Tables A-C below. In one embodiment, the antigen binding protein comprises a light chain CDR1 sequence illustrated herein in Table 2 in Figure 49 and Table C below. In another embodiment, the antigen binding protein comprises a light chain CDR2 sequence illustrated herein in Table 2 in Figure 49 and Table C below. In another embodiment, the antigen binding protein comprises a light chain CDR3 sequence illustrated in herein in Table 2 in Figure 49 and Table C below. In another embodiment, the antigen binding protein comprises a heavy chain CDR1 sequence illustrated in herein in Table 2 in Figure 49 and Table C below. In another embodiment, the antigen binding protein comprises a heavy chain CDR2 sequence illustrated herein in Table 2 in Figure 49 and Table C below. In another embodiment, the antigen binding protein comprises a heavy chain CDR3 sequence illustrated herein in Table 2 in Figure 49 and Table C below. In another embodiment, the antigen binding protein comprises a light chain FR1 sequence illustrated herein in Tables 3-7 in Figures 50-54, respectively. In another embodiment, the antigen binding protein comprises a light chain FR2 sequence illustrated herein in Tables 3-7 in Figures 50-54, respectively. In another embodiment, the antigen binding protein comprises a light chain FR3 sequence illustrated herein in Tables 3-7 in Figures 50-54, respectively. In another embodiment, the antigen binding protein comprises a light chain FR4 sequence illustrated herein in Table 3-7 in Figures 50-54, respectively. In another embodiment, the antigen binding protein comprises a heavy chain FR1 sequence illustrated herein in Table 3-7 in Figures 50-54, respectively. In another embodiment, the antigen binding protein comprises a heavy chain FR2 sequence illustrated herein in Table 3-7 in Figures 50-54, respectively. In another embodiment, the antigen binding protein comprises a heavy chain FR3 sequence illustrated herein in Table 3-7 in Figures 50-54, respectively. In another embodiment, the antigen binding protein comprises a heavy chain FR4 sequence illustrated herein in Table 3-7 in Figures 50-54, respectively.

In another embodiment, at least one of the antigen binding protein's CDR3 sequences differs by no more than 6, 5, 4, 3, 2, 1 or 0 single amino acid addition, substitution, and/ordeletion from a CDR3 sequence from the sequences as shown in Table 2 in Figure 49 or Table C below. In another embodiment, the antigen binding protein's light chain CDR3 sequence differs by no more than 6, 5, 4, 3, 2, 1 or 0 single amino acid addition, substitution, and/or deletion from a light chain CDR3 sequence from the sequences as shown in Table 2 in Figure 49 or Table C below and the antigen binding protein's heavy chain CDR3 sequence differs by no more than 6, 5, 4, 3, 2, 1 or 0 single amino acid addition, substitution, and/or deletion from a heavy chain CDR3 sequence from the sequences as shown in Table 2 in Figure 49 or Table C below. In another embodiment, the antigen binding protein further comprises 1, 2, 3, 4, or 5 CDR sequences that each independently differs by 6, 5, 4, 3, 2, 1, or 0 single amino acid additions, substitutions, and/or deletions from a CDR sequence of the sequences shown in Table 2 in Figure 49 or Table C below. In another embodiment, the antigen binding protein comprises the CDRs of the light chain variable region and the CDRs of the heavy chain variable region set forth in Table 2 in Figure 49 or Table C below. In a further embodiment, the antigen binding protein comprises the CDRs of any one of the antibodies in Table 2 in Figure 49 or Table C below. In one embodiment, the antigen binding protein is a human antibody. In another embodiment, the antigen binding protein is a humanized antibody. In certain embodiments, the VH CDRs and the VL CDRs are paired in a manner indicated in Tables 2-7 in Figures 49-54, respectively.

In one embodiment, the antigen binding protein (e.g., an antibody) comprises a light chain variable domain comprising a sequence of amino acids that differs from the sequence of a light chain variable domain listed in Table 3-7 in Figures 50-54, respectively at 15, 14, 13, 12, 11, 10, 9, 8, 7, 6, 5, 4, 3, 2, 1 or 0 residues, wherein each such sequence difference is independently either a deletion, insertion, or substitution of one amino acid residue. In another embodiment, the antigen binding protein (e.g., an antibody) comprises a heavy chain variable domain comprising a sequence of amino acids that differs from the sequence of a heavy chain variable domain listed in Table 3-7 in Figures 50-54, respectively at 15, 14, 13, 12, 11, 10, 9, 8, 7, 6, 5, 4, 3, 2, 1 or 0 residues, wherein each such sequence difference is independently either a deletion, insertion, or substitution of one amino acid residue. In certain embodiments, the antigen binding protein comprises a light chain variable domain and a heavy chain variable domain that are paired in a manner indicated in Tables 3-7 in Figures 50-54, respectively. In certain embodiments, the antigen binding protein comprises a light chain variable domain and a heavy chain variable domain that are paired in a manner indicated in Tables A-C below.

In a particular embodiment, the antigen binding protein (e.g., antibody) binds to human ASGR-1 and comprises a heavy chain variable domain containing one or more VH CDR1 (HCDR1), VH CDR2 (HCDR2) and/or VH CDR3 (HCDR3), wherein the VH CDR1 (HCDR1) has an amino acid sequence identical to, or comprising not more than 3 amino acid additions/insertions, deletions or substitutions as compared to, the amino acid sequences selected from the group consisting of SEQ ID NO:5136, SEQ ID NO:50001, SEQ ID NO:50012 and SEQ ID NO:50468; the VH CDR2 (HCDR2) has an amino acid sequence identical to, or comprising not more than 3 amino acid additions/insertions, deletions or substitutions as compared to, the amino acid sequences selected from the group consisting of SEQ ID NO:13148, SEQ ID NO:50002, SEQ ID NO:50014, and SEQ ID NO:50260; and the VH CDR3 (HCDR3) has an amino acid sequence identical to, or comprising not more than 3 amino acid additions/insertions, deletions or substitutions as compared to, the amino acid sequences selected from the group consisting of SEQ ID NO:21160, SEQ ID NO:50003, and SEQ ID NO:50470.

In a particular embodiment, the antigen binding protein (e.g., antibody) binds to human ASGR-1 and comprises a light chain variable domain containing one or more VL CDR1 (LCDR1), VL CDR2 (LCDR2) and/or VL CDR3 (LCDR3), wherein the VL CDR1 (LCDR1) has an amino acid sequence identical to, or comprising not more than 3 amino acid additions/insertions, deletions or substitutions as compared to, the amino acid sequences selected from the group consisting of SEQ ID NO:1130, SEQ ID NO:50133, SEQ ID NO:50156 and SEQ ID NO:50162; the VL CDR2 (LCDR2) has an amino acid sequence identical to, or comprising not more than 3 amino acid additions/insertions, deletions or substitutions as compared to, the amino acid sequences selected from the group consisting of SEQ ID NO:9142, SEQ ID NO:50157, SEQ ID NO:50163, SEQ ID NO:50229, SEQ ID NO:50619, SEQ ID NO:50643 and SEQ ID NO:50649; and the VL CDR3 (LCDR3) has an amino acid sequence identical to, or comprising not more than 3 amino acid additions/insertions, deletions or substitutions as compared to, the amino acid sequences selected from the group consisting of SEQ ID NO:17154, SEQ ID NO:50134, SEQ ID NO:50164, and SEQ ID NO:50261.

In a particular embodiment, the antigen binding protein (e.g., antibody) binds to human ASGR-1 and comprises A) a heavy chain variable domain containing one or more VH CDR1 (HCDR1), VH CDR2 (HCDR2) and/or VH CDR3 (HCDR3), wherein the VH CDR1 (HCDR1) has an amino acid sequence identical to, or comprising not more than 3 amino acid additions/insertions, deletions or substitutions as compared to, the amino acid sequences selected from the group consisting of SEQ ID NO:5136, SEQ ID NO:50001, SEQ ID NO:50012 and SEQ ID NO:50468; the VH CDR2 (HCDR2) has an amino acid sequence identical to, or comprising not more than 3 amino acid additions/insertions, deletions or substitutions as compared to, the amino acid sequences selected from the group consisting of SEQ ID NO:13148, SEQ ID NO:50002, SEQ ID NO:50014, and SEQ ID NO:50260; and the VH CDR3 (HCDR3) has an amino acid sequence identical to, or comprising not more than 3 amino acid additions/insertions, deletions or substitutions as compared to, the amino acid sequences selected from the group consisting of SEQ ID NO:21160, SEQ ID NO:50003, and SEQ ID NO:50470; and B) a light chain variable domain containing one or more VL CDR1 (LCDR1), VL CDR2 (LCDR2) and/or VL CDR3 (LCDR3), wherein the VL CDR1 (LCDR1) has an amino acid sequence identical to, or comprising not more than 3 amino acid additions/insertions, deletions or substitutions as compared to, the amino acid sequences selected from the group consisting of SEQ ID NO:1130, SEQ ID NO:50133, SEQ ID NO:50156 and SEQ ID NO:50162; the VL CDR2 (LCDR2) has an amino acid sequence identical to, or comprising not more than 3 amino acid additions/insertions, deletions or substitutions as compared to, the amino acid sequences selected from the group consisting of SEQ ID NO:9142, SEQ ID NO:50157, SEQ ID NO:50163, SEQ ID NO:50229, SEQ ID NO:50619, SEQ ID NO:50643 and SEQ ID NO:50649; and the VL CDR3 (LCDR3) has an amino acid sequence identical to, or comprising not more than 3 amino acid additions/insertions, deletions or substitutions as compared to, the amino acid sequences selected from the group consisting of SEQ ID NO:17154, SEQ ID NO:50134, SEQ ID NO:50164, and SEQ ID NO:50261. In one embodiment, the antigen binding protein (e.g., antibody) comprises A) a heavy chain variable domain containing a VH CDR1 (HCDR1), a VH CDR2 (HCDR2) and a VH CDR3 (HCDR3), wherein the VH CDR1 (HCDR1) has an amino acid sequence identical to, or comprising not more than 3 amino acid additions/insertions, deletions or substitutions as compared to, the amino acid sequences selected from the group consisting of SEQ ID NO:5136, SEQ ID NO:50001, SEQ ID NO:50012 and SEQ ID NO:50468; the VH CDR2 (HCDR2) has an amino acid sequence identical to, or comprising not more than 3 amino acid additions/insertions, deletions or substitutions as compared to, the amino acid sequences selected from the group consisting of SEQ ID NO:13148, SEQ ID NO:50002, SEQ ID NO:50014, and SEQ ID NO:50260; and the VH CDR3 (HCDR3) has an amino acid sequence identical to, or comprising not more than 3 amino acid additions/insertions, deletions or substitutions as compared to, the amino acid sequences selected from the group consisting of SEQ ID NO:21160, SEQ ID NO:50003, and SEQ ID NO:50470; and B) a light chain variable domain containing a VL CDR1 (LCDR1), a VL CDR2 (LCDR2) and a VL CDR3 (LCDR3), wherein the VL CDR1 (LCDR1) has an amino acid sequence identical to, or comprising not more than 3 amino acid additions/insertions, deletions or substitutions as compared to, the amino acid sequences selected from the group consisting of SEQ ID NO:1130, SEQ ID NO:50133, SEQ ID NO:50156 and SEQ ID NO:50162; the VL CDR2 (LCDR2) has an amino acid sequence identical to, or comprising not more than 3 amino acid additions/insertions, deletions or substitutions as compared to, the amino acid sequences selected from the group consisting of SEQ ID NO:9142, SEQ ID NO:50157, SEQ ID NO:50163, SEQ ID NO:50229, SEQ ID NO:50619, SEQ ID NO:50643 and SEQ ID NO:50649; and the VL CDR3 (LCDR3) has an amino acid sequence identical to, or comprising not more than 3 amino acid additions/insertions, deletions or substitutions as compared to, the amino acid sequences selected from the group consisting of SEQ ID NO:17154, SEQ ID NO:50134, SEQ ID NO:50164, and SEQ ID NO:50261. In one embodiment, the antigen binding protein comprises A) a heavy chain variable domain containing a VH CDR1 (HCDR1), a VH CDR2 (HCDR2) and a VH CDR3 (HCDR3), wherein the VH CDR1 (HCDR1) amino acid sequence is selected from the group consisting of SEQ ID NO:5136, SEQ ID NO:50001, SEQ ID NO:50012 and SEQ ID NO:50468; the VH CDR2 (HCDR2) amino acid sequence is selected from the group consisting of SEQ ID NO:13148, SEQ ID NO:50002, SEQ ID NO:50014, and SEQ ID NO:50260; and the VH CDR3 (HCDR3) amino acid sequence is selected from the group consisting of SEQ ID NO:21160, SEQ ID NO:50003, and SEQ ID NO:50470; and B) a light chain variable domain containing a VL CDR1 (LCDR1), a VL CDR2 (LCDR2) and a VL CDR3 (LCDR3), wherein the VL CDR1 (LCDR1) amino acid sequence is selected from the group consisting of SEQ ID NO:1130, SEQ ID NO:50133, SEQ ID NO:50156 and SEQ ID NO:50162; the VL CDR2 (LCDR2) amino acid sequence is selected from the group consisting of SEQ ID NO:9142, SEQ ID NO:50157, SEQ ID NO:50163, SEQ ID NO:50229, SEQ ID NO:50619, SEQ ID NO:50643 and SEQ ID NO:50649; and the VL CDR3 (LCDR3) amino acid sequence is selected from the group consisting of SEQ ID NO:17154, SEQ ID NO:50134, SEQ ID NO:50164, and SEQ ID NO:50261. In one embodiment, the antigen binding protein comprises a heavy chain variable domain and a light chain variable domain containing a VH CDR1 having the amino acid sequence set forth in SEQ ID NO:5136; a VH CDR2 having the amino acid sequence set forth in SEQ ID NO:13148; a VH CDR3 having the amino acid sequence set forth in SEQ ID NO:21 160; a VL CDR1 having the amino acid sequence set forth in SEQ ID NO:1130; a VL CDR2 having the amino acid sequence set forth in SEQ ID NO:9142; and a VL CDR3 having the amino acid sequence set forth in SEQ ID NO:17154.

In a particular embodiment, the antigen binding protein (e.g., antibody) comprises a) a light chain variable domain having no more than ten or no more than five amino acid additions/insertions, deletions or substitutions from the amino acid sequence set forth in SEQ ID NO:25164 or SEQ ID NO:50326; b) a heavy chain variable domain having no more than ten or no more than five amino acid additions/insertions, deletions or substitutions from the amino acid sequence set forth in SEQ ID NO:29170 or SEQ ID NO:50266; or c) the light chain variable domain of a) and the heavy chain variable domain of b). In one embodiment, the antigen binding protein comprises a light chain varable domain having the amino acid sequence set forth in SEQ ID NO:25164 or SEQ ID NO:50326; and a heavy chain variable domain having the amino acid sequence set forth in SEQ ID NO:29170 or SEQ ID NO:50266. In one embodiment, the antigen binding protein comprises a light chain varable domain having the amino acid sequence set forth in SEQ ID NO:50326; and a heavy chain variable domain having the amino acid sequence set forth in SEQ ID NO:50266. In one embodiment, the antigen binding protein comprises a light chain varable domain having the amino acid sequence set forth in SEQ ID NO:25164; and a heavy chain variable domain having the amino acid sequence set forth in SEQ ID NO:29170.

While specific embodiments relating to the antigen binding protein identified as 4A2 are set forth above with particularity, the embodiments of the present invention are not intended to be limited in scope to this individual embodiment. The embodiments directed to 4A2 are intended merely as single illustrations of individual embodiments. It is fully anticipated that the embodiments of the present invention include antigen binding proteins comprising heavy chain variable domains containing one or more VH CDR1 (HCDR1), VH CDR2 (HCDR2) and/or VH CDR3 (HCDR3) and/or light chain variable domains containing one or more VL CDR1 (LCDR1), VL CDR2 (LCDR2) and/or VL CDR3 (LCDR3) as set forth in Tables 2-7 in Figures 49-57, respectively, as well as Tables 19A-C and Tables 20A-C in Figure 55, Tables 21-134 in Figures 56 and 57, and Tables A, B and C.

## Table A: Exemplary Heavy and Light Chain Variable Regions

| Ab name | SEQ ID NOs: VH/VL | Ab name | SEQ ID NOs: VH/VL | Ab name | SEQ ID NOs: VH/VL | Ab name | SEQ ID NOs: VH/VL |
|---|---|---|---|---|---|---|---|
| 10G6 | 29184/25178 | 59F2 | 31512/27506 | 147E9 | 30172/26166 | 191G10 | 30846/26840 |
| 11E2 | 29040/25034 | 5E5 | 29016/25010 | 184E7 | 30660/26654 | 191G12 | 30730/26724 |
| 11F5 | 29054/25048 | 60D2 | 31518/27512 | 194A4 | 30820/26814 | 192C10 | 30764/26758 |
| 12E9 | 29186/25180 | 60E8 | 29494/25488 | 208A2 | 28136/24130 | 192C8 | 30756/26750 |
| 12F11 | 29178/25172 | 63A10 | 31536/27530 | 210G10 | 31054/27048 | 192E4 | 30744/26738 |
| 12F12 | 29188/25182 | 63G7 | 31534/27528 | 4B1 | 28878/24872 | 192G6 | 30752/26746 |
| 13F6 | 28772/24766 | 64B12 | 29624/25618 | 60E12 | 29502/25496 | 192G8 | 30760/26754 |
| 148E10 | 28132/24126 | 65F10 | 28134/24128 | 61A1 | 29504/25498 | 192H10 | 30768/26762 |
| 154F4 | 31392/27386 | 68G6 | 28224/24218 | 62H10 | 31832/27826 | 193C7 | 30794/26788 |
| 159H8 | 31416/27410 | 6A6 | 28806/24800 | 63H8 | 29604/25598 | 194B7 | 30828/26822 |
| 160B12 | 31418/27412 | 6D4 | 28816/24810 | 72G9 | 32080/28074 | 194C1 | 30816/26810 |
| 175D10 | 30538/26532 | 6D9 | 29154/25148 | 8D8 | 29168/25162 | 196C7 | 30870/26864 |
| 177D2 | 31858/27852 | 6G6 | 29198/25192 | 12D2 | 29036/25030 | 197B6 | 30894/26888 |
| 25A4 | 28522/ 24516 | 70D1 | 29670/25664 | 148H10 | 30196/26190 | 197E11 | 30906/26900 |
| 25D12 | 28510/24504 | 7A10 | 29194/25188 | 173C11 | 30520/26514 | 197F2 | 30886/26880 |
| 26C4 | 28580/24574 | 7E11 | 28914/24908 | 179C2 | 30570/26564 | 197G3 | 30888/26882 |
| 27E7 | 28744/24738 | 7F4 | 28814/24808 | 47C1 | 29286/25280 | 198G3 | 30620/26614 |
| 28H2 | 29190/25184 | 7F8 | 28948/24942 | 49C1 | 29320/25314 | 213B3 | 31092/27086 |
| 29E2 | 29192/25186 | 7G4 | 28966/24960 | 60C12 | 29500/25494 | 219H1 | 31156/27150 |
| 29E6 | 28550/24544 | 8D12 | 29050/25044 | 60G2 | 29482/25476 | 74C8 | 29768/25762 |
| 29H8 | 28798/24792 | 9F12LC1 | 28216/24210 | 65D5 | 29632/25626 | 74G6 | 29894/25888 |
| 32D6 | 29196/25190 | 9F12LC2 | 28217/24211 | 66H11 | 28130/24124 | 75G3 | 29714/25708 |
| 3G7 | 28840/24834 | 9G9 | 28790/24784 | 71A6 | 28128/24122 | 89A11 | 30028/26022 |
| 45B4 | 29252/25246 | 65E9 | 31538/27532 | 73G1 | 31556/27550 | 74B2 | 29736/25730 |
| 49F10 | 29334/25328 | 72B4 | 31552/27546 | 49C5 | 32086/28080 | 74H7 | 29966/25960 |
| 4A2 | 29170/25164 | 7H7 | 28944/24938 | 49D10 | 32088/28082 | 85F7 | 29766/25760 |
| 4B3 | 28750/24744 | 9C11 | 28856/24850 | 51E3 | 30958/26952 | 198B9 | 30918/26912 |
| 4H6 | 28936/24930 | 12B12 | 28978/24972 | 51F4 | 31476/27470 | 199A7 | 30932/26926 |
| 50D4 | 29362/25356 | 147D10 | 30174/26168 | 53E8 | 32090/28084 | 218G4 | 31786/27780 |
| 50G9 | 32082/28076 | 149D11 | 30226/26220 | 54E9 | 31488/27482 | 146A8 | 31332/27326 |
| 51E9 | 29366/25360 | 149F8 | 30222/26216 | 56E3 | 31492/27486 | 146B6 | 31334/27328 |
| 52G11 | 28138/24132 | 151B9 | 31372/27366 | 56G1 | 31490/27484 | 149A1 | 31344/27338 |
| 52H1 | 31482/27476 | 175F4 | 31456/27450 | 190C11 | 30602/26596 | 172B12 | 31452/27446 |
| 53F2 | 28140/24134 | 22G5 | 28368/24362 | 190E6 | 30642/26636 | 172C3 | 31450/27444 |
| 53F7 | 29412/25406 | 48B12 | 31820/27814 | 190F12 | 30618/26612 | 193E7 | 30796/26790 |
| 55B1 | 29430/25424 | 52H2 | 29380/25374 | 190F8 | 30712/26706 | 199E3 | 30926/26920 |
| 56E5 | 29466/25460 | 6G7 | 28880/24874 | 190G11 | 30608/26602 | 226F9 | 31264/27258 |
| 65C12 | 32078/28072 | 7G2 | 28942/24936 | 190H9 | 30716/26710 | 227C1 | 31280/27274 |

Table A continued:

| Ab name | SEQ ID NOs: VH/VL | Ab name | SEQ ID NOs: VH/VL | Ab name | SEQ ID NOs: VH/VL |
|---|---|---|---|---|---|
| 176H4 | 30542/26536 | 72F5 | 29700/25694 | 48D7 | 29306/25300 |
| 194C10 | 30832/26826 | 191A10 | 30724/26718 | 52D10 | 29388/25382 |
| 191E10 | 30726/26720 | 191G1 | 30628/26622 | 59E6 | 29590/25584 |
| 196F4 | 30868/26862 | 227F2 | 31282/27276 | 64E2 | 31836/27830 |
| 198D2 | 31604/27598 | 31D12LC1 | 29176/25170 | 57A7 | 29554/25548 |
| 202A3 | 30972/26966 | 31D12LC2 | 29174/25168 | 58G11 | 31510/27504 |
| 204G6 | 31004/26998 | 7C3LC1 | 28212/24206 | 64G12 | 29626/25620 |
| 224G1 | 31196/27190 | 7C3LC2 | 28214/24208 | | |

Table B:

| Ab name | SEQ ID NOs: VH/VL | Ab name | SEQ ID NOs: VH/VL | Ab name | SEQ ID NOs: VH/VL | Ab name | SEQ ID NOs: VH/VL |
|---|---|---|---|---|---|---|---|
| 175D10 | 30538/26532 | 184E7 | 30660/26654 | 192E4 | 30744/26738 | 74B2 | 29736/25730 |
| 25A4 | 28522/24516 | 194A4 | 30820/26814 | 192G6 | 30752/26746 | 74H7 | 29966/25960 |
| 26C4 | 28580/24574 | 208A2 | 28136/24130 | 192G8 | 30760/26754 | 85F7 | 29766/25760 |
| 29H8 | 28798/24792 | 210G10 | 31054/27048 | 192H10 | 30768/26762 | 218G4 | 31786/27780 |
| 49F10 | 29334/25328 | 4B1 | 28878/24872 | 193C7 | 30794/26788 | 172B12 | 31452/27446 |
| 4A2 | 29170/25164 | 72G9 | 32080/28074 | 194B7 | 30828/26822 | 172C3 | 31450/27444 |
| 4H6 | 28936/24930 | 190C11 | 30602/26596 | 194C1 | 30816/26810 | 193E7 | 30796/26790 |
| 50D4 | 29362/25356 | 190E6 | 30642/26636 | 196C7 | 30870/26864 | 199E3 | 30926/26920 |
| 51E9 | 29366/25360 | 190F12 | 30618/26612 | 197B6 | 30894/26888 | 191E10 | 30726/26720 |
| 52H1 | 31482/27476 | 190F8 | 30712/26706 | 197E11 | 30906/26900 | 196F4 | 30868/26862 |
| 55B1 | 29430/25424 | 190G11 | 30608/26602 | 197F2 | 30886/26880 | 198D2 | 31604/27598 |
| 56E5 | 29466/25460 | 190H9 | 30716/26710 | 197G3 | 30888/26882 | 202A3 | 30972/26966 |
| 64B12 | 29624/25618 | 191A10 | 30724/26718 | 198G3 | 30620/26614 | 204G6 | 31004/26998 |
| 6G6 | 29198/25192 | 191G1 | 30628/26622 | 213B3 | 31092/27086 | 10G6 | 29184/25178 |
| 7F4 | 28814/24808 | 191G10 | 30846/26840 | 219H1 | 31156/27150 | 160B12 | 31418/27412 |
| 7G4 | 28966/24960 | 191G12 | 30730/26724 | 74C8 | 29768/25762 | 177D2 | 31858/27852 |
| 149F8 | 30222/26216 | 192C10 | 30764/26758 | 74G6 | 29894/25888 | 53F7 | 29412/25406 |
| 48B12 | 31820/27814 | 192C8 | 30756/26750 | 75G3 | 29714/25708 | 63A10 | 31536/24530 |
| 7E11 | 28914/24908 | 198B9 | 30918/26912 | 146B6 | 31334/27328 | 22G5 | 28368/24362 |
| 6G7 | 28880/24874 | 199A7 | 30932/26926 | 176H4 | 30542/26536 | 5E5 | 29016/25010 |
| 147E9 | 30172/26166 | 146A8 | 31332/27326 | 149A1 | 31344/27338 | 194C10 | 30832/26826 |
| 54E9 | 31488/27482 | 12D2 | 29036/25030 | | | | |

Table C: Exemplary Heavy and Light Chain Variable Regions and Heavy and Light Chain CDR1/2/3

| Ab name | VH SEQ ID NOs: | VL SEQ ID NOs: | HCDR1 SEQ ID NOs: | HCDR2 SEQ ID NOs: | HCDR3 SEQ ID NOs: | LCDR1 SEQ ID NOs: | LCDR2 SEQ ID NOs: | LCDR3 SEQ ID NOs: |
|---|---|---|---|---|---|---|---|---|
| 25A4 | 28522 or 50266 | 24516 or 50316 | 4488, 50468, 50001 or 50013 | 12500, 50002, 50014 or 50259 | 20512, 50003 or 50470 | 480, 50133 or 50162 | 8492, 50157, 50229, 50619, 50643 or 50649 | 16504, 50134, 50164 or 50620 |
| 26C4 | 28580 or 50266 | 24574 or 50316 | 4546, 50001, 50013 or 50468 | 12588 or 50002 | 20570, 50003 or 50470 | 538, 50133 or 50156 | 8550, 50157, 50163, 50229, 50619, 50643 or 50649 | 16562, 50134, 50164 or 50620 |
| 29H8 | 28798 or 50266 | 24792 or 50316 | 4764, 50001, 50013 or 50468 | 12776 or 50002 | 20788 or 50003 or 50470 | 756 or 50133 | 8768, 50157, 50163 50229, 50619, 50643 or 50649 | 16780 or 50134 |
| 4A2 | 29170 or 50266 | 25164 or 50326 | 5136, 50001, 50013, or 50468 | 13148, 50002, 50014 or 50260 | 21160 , 50003 or 50470 | 1130, 50133, 50156 or 50162 | 9142, 50157, 50163 50229, 50619, 50643 or 50649 | 17154, 50134, 50164 or 50261 |
| 4H6 | 28936 or 50272 | 24930 or 50321 | 4902 or 50019 | 12914 or 50020 | 20926 or 50021 or 50262 | 894, 50147 or 50159 | 8096, 50148 or 50160 | 16918 or 50149 |
| 56E5 | 29466 or 50272 | 25460 or 50321 | 5432, 50019 or 50058 | 13444 or 50020 | 21456 or 50021 | 1426 or 50147 | 9438, 50123, 50131, 50136, 50139, 50142, 50145, 50148, 50154, | 17450 or 50149 |

| | | | | | | | | |
|---|---|---|---|---|---|---|---|---|
| | | | | | | | 50160, 50181, 50184, 50199, 50202, 50213 or 50248 | |
| 7F4 | 28814 or 50284 | 24808 or 50312 | 4780, 50046 or 50075 | 12792 or 50047 | 20804 or 50048 | 772, 50122, 50130, 50135 or 50198 | 8784, 50123, 50131, 50136, 50139, 50142, 50145, 50148, 50154, 50160, 50181, 50199 or 50213 | 16796 or 50124 |
| 7G4 | 28966 or 50267 | 24960 or 50315 | 4932, 50004, 50037 or 50107 | 12944, 50005, 50008, 50017, 50023, 50026, 50038, 50053, 50067, 50073, 50085, 50088, 50100, 50108, 50238 or 50254 | 20956 or 50006 | 924, 50122, 50130, 50135, 50198, or 50247 | 8936, 50123, 50131, 50136, 50139, 50142, 50145, 50148, 50154, 50160, 50181, 50184, 50199, 50202, 50213 or 50248 | 16948 |
| 48B12 | 31820 or 50267 | 27814 | 7784, 50034, 50055, 50093, 50113 or 50116 | 15796, 50032, 50035, 50056, 50070, 50091, | 23808 | 3780 | 11792 or 50126 | 19804 |

|  |  |  |  | 50105 or 50117 |  |  |  |  |
|---|---|---|---|---|---|---|---|---|
| 184E7 | 30660 or 50272 | 26654 or 50320 | 6626, 50019 or 50237 | 14638 or 50020 | 22650 | 2620, 50138, 50144, 50147, 50183 or 50212 | 10632, 50123, 50131, 50136, 50139, 50142, 50145, 50148, 50154, 50160, 50181, 50184, 50199, 50202, 50213 or 50248 | 18644 or 50146 |
| 194A4 | 30820 | 26814 or 50342 | 6786 | 14798, 50020, 50050, 50059 or 50079 | 22810 | 2780 or 50206 | 10792, 50128 or 50207 | 18804 or 50208 |
| 4B1 | 28878 | 24872 or 50323 | 4844 | 12856 | 20868 | 836, 50141 or 50153 | 8848, 50123, 50131, 50136, 50139, 50142, 50145, 50148, 50154, 50160, 50181, 50184, 50199, 50202, 50213 or 50248 | 16860, 50143 or 50203 |
| 190F8 | 30712 or 50271 | 26706 or 50318 | 6678, 50007, 50016, 50037, 50066, 50072 | 14690, 50017, 50023, 50038 or 50088 | 22702 or 50018 | 2672, 50138 or 50144 | 10684 or 50139 | 18696, 50140, or 50146 |

| | | | 50084, 50237 or 50253 | | | | | |
|---|---|---|---|---|---|---|---|---|
| 191G1 | 30628 or 50271 | 26622 or 50318 | 6594, 50004, 50007, 50016, 50022, 50025, 50037, 50066, 50072, 50084, 50087, 50096, 50099, 50107, 50237 or 50252 | 14606, 50008 or 50017 | 22618 or 50018 | 2588, 50138, 50144, 50147, 50183, or 50212 | 10600, 50123, 50131, 50136, 50139, 50142, 50145, 50148, 50154, 50160, 50181, 50184, 50199, 50202, 50213 or 50214 | 18612 or 50140 |
| 191G10 | 30846 or 50271 | 26840 or 50318 | 6812, 50004, 50007, 50016, 50022, 50025, 50037, 50066, 50072, 50084, 50087, 50096, 50099, 50107, 50237 or 50253 | 14824, 50017, 50023, 50038, or 50088 | 22836 or 50018 | 2806, 50138 or 50144 | 10818 or 50139 | 18830 or 50140 |
| 194C1 | 30816 | 26810 | 6782, 50004, 50007, 50016, 50022, 50025, 50037, 50066, 50072, 50084, | 14794, 50008, or 50017 | 22806 | 2776, 50138, 50144, 50147, 50183 or 50212 | 10788, 50123, 50131, 50136, 50139, 50142, 50145, 50148, 50154, 50160, | 18800 or 50140 |

| | | | 50087, 50096, 50099, 50107, 50237 or 50253 | | | | 50181, 50184, 50199, 50202, 50213 or 50248 | |
|---|---|---|---|---|---|---|---|---|
| 197G3 | 30888 or 50273 | 26882 or 50320 | 6854, 50016 or 50022 | 14866, 50017, 50023, 50038, or 50088 | 22878 or 50024 | 2848, 50138 or 50144 | 10860, 50123, 50131, 50136, 50139, 50142, 50145, 50148, 50154, 50160, 50181, 50184, 50199, 50202, 50213 or 50248 | 18872 or 50140 |
| 198G3 | 30620 or 50271 | 26614 or 50318 | 6586, 50007, 50016, 50037, 50066, 50072, 50084, 50237 or 50253 | 14598, 50017, or 50038 | 22610 or 50018 | 2580 or 50138 | 10592 or 50139 | 18604 or 50140 |
| 75G3 | 29714 or 50283 | 25708 or 50314 | 5680, 50010 or 50233 | 13692 | 21704 or 50235 | 1674 or 50127 | 9686 or 50128 | 17698 or 50129 |
| 218G4 | 31786 or 50298 | 27780 or 50335 | 7750, 50004, 50025, 50037, 50087, 50096 or 50253 | 15762, 50005, 50008, 50017, 50023, 50026, 50038, 50053, | 23774 | 3746 or 50189 | 11758 or 50190 | 19770 or 50191 |

| | | | | | | | | |
|---|---|---|---|---|---|---|---|---|
| | | | | 50067, 50073, 50085, 50088, 50100, 50108, 50238 or 50254 | | | | |
| 193E7 | 30796 | 26790 or 50312 | 6762 | 14774, 50011, or 50234 | 22786 | 2756, 50122, 50130, 50135, 50198, or 50247 | 10768, 50123 or 50142 | 18780 or 50124 |
| 198D2 | 31604 or 50273 | 27598 or 50335 | 7568, 50004, 50007, 50016, 50022, 50025, 50037, 50066, 50072, 50084, 50087, 50096, 50099, 50107, 50237 or 50253 | 15580 or 50023 | 23592 | 3564 or 50189 | 11576 or 50190 | 19588 or 50191 |
| 202A3 | 30972 | 26966 or 50317 | 6938 | 14950 | 22962 | 2932, 50122, 50130, 50135, 50198, or 50247 | 10944, 50123, 50131, 50136, 50139, 50142, 50148, or 50213 | 18956 or 50137 |
| 7E11 | 28914 or 50273 | 24908 or 50319 | 4880, 50004, 50007, 50022, 50025 or 50037 | 12892 or 50263 or 50023 | 20904 or 50024 | 872 or 50141 or 50153 | 8884, 50123, 50131, 50136, 50139, 50142, 50145, | 16896, or 50143 |

| | | | | | | | | |
|---|---|---|---|---|---|---|---|---|
| | | | | | | | 50148, 50154, 50160, 50181, 50199 or 50213 | |
| 22G5 | 28368 | 24362 or 50323 | 4334, 50031, 50034, 50055, 50093, 50113 or 50116 | 12346 or 50032 | 20358 or 50033 | 326, 50141, 50153, 50180 or 50201 | 8338, 50123, 50131, 50136, 50139, 50142, 50148, 50154 or 50160 | 16350 |
| 5E5 | 29016 or 50267 | 25010 or 50315 | 4982, 50004, 50037 or 50107 | 12994, 50005, 50008, 50017, 50023, 50026, 50038, 50053, 50067, 50073, 50085, 50088, 50100, 50108, 50238, 50254 or 50264, | 21006, 50006 or 50265 | 974, 50122, 50130, 50135, 50198, or 50247 | 8986, 50123, 50131, 50136, 50139, 50142, 50145, 50148, 50154, 50160, 50181, 50184, 50199, 50202, 50213 or 50248 | 16998 or 50132 |
| 54E9 | 31488 or 50303 | 27482 or 50338 | 7452 or 50102 | 15464 or 50103 | 23476 or 50227 | 3448 or 50195 | 11460 or 50196 | 19472 or 50197 |
| 6G7 | 28880 | 24874 or 50334 | 4846, 50004, 50007, 50016, 50022, 50025, 50037, | 12858 | 20870 or 50098 | 838 or 50186 | 8850 or 50187 | 16862 or 50188 |

| | | | 50066, 50072, 50084, 50087, 50096, 50099, 50107, 50237 or 50253 | | | | | |
|---|---|---|---|---|---|---|---|---|
| 176H4 | 30542 or 50282 | 26536 or 50322 | 6508, 50004, 50007, 50016, 50022, 50025, 50037, 50066, 50072, 50084, 50087, 50096, 50099, 50107, 50237 or 50253 | 14520, 50023, 50053, 50085 or 50254 | 22532, or 50255 | 2502, 50150, or 50174 | 10514, 50151, 50175 or 50205 | 18526 or 50152 |
| 194C10 | 30832 | 26826 or 50314 | 6798 or 50233 | 14810, 50011 or 50234 | 22822 | 2792 or 50146 | 10804 or 50128 | 18816 or 50129 |

In the exemplary embodiments described above, the antigen binding proteins maintain desired binding to the various desired species of ASGR, ASGR-1 and/or ASGR-2.

In another embodiment, the light-chain variable domain comprises a sequence of amino acids that is at least 70%, 75%, 80%, 85%, 90%, 91%, 92%, 93%, 94%, 95%, 96%, 97%, 98%, 99% or 100% identical to the sequence of a light chain variable domain listed above.

In another embodiment, the light chain variable domain comprises a sequence of amino acids that is encoded by a nucleotide sequence that is at least 70%, 75%, 80%, 85%, 90%, 91%, 92%, 93%, 94%, 95%, 96%, 97%, 98%, 99% or 100% identical to the polynucleotide sequence listed above. In another embodiment, the light chain variable domain comprises a sequence of amino acids that is encoded by a polynucleotide that hybridizes under moderately stringent conditions to the complement of a polynucleotide that encodes a light chain variable domain selected from the sequences listed above. In another embodiment, the light chain variable domain comprises a sequence of amino acids that is encoded by a polynucleotide that hybridizes under stringent conditions to the complement of a polynucleotide that encodes a light chain variable domain selected from the group consisting of the sequences listed above.

In another embodiment, the heavy chain variable domain comprises a sequence of amino acids that is at least 70%, 75%, 80%, 85%, 90%, 91%, 92%, 93%, 94%, 95%, 96%, 97%, 98%, 99% or 100% identical to the sequence of a heavy chain variable domain selected from the sequences listed above. In another embodiment, the heavy chain variable domain comprises a sequence of amino acids that is encoded by a nucleotide sequence that is at least 70%, 75%, 80%, 85%, 90%, 91%, 92%, 93%, 94%, 95%, 96%, 97%, 98%, 99% or 100% identical to a nucleotide sequence that encodes a heavy chain variable domain selected from the sequences listed above. In another embodiment, the heavy chain variable domain comprises a sequence of amino acids that is encoded by a polynucleotide that hybridizes under moderately stringent conditions to the complement of a polynucleotide that encodes a heavy chain variable domain selected from the sequences listed above. In another embodiment, the heavy chain variable domain comprises a sequence of amino acids that is encoded by a polynucleotide that hybridizes under stringent conditions to the complement of a polynucleotide that encodes a heavy chain variable domain selected from the sequences listed above.

In the exemplary embodiments described above, the antigen binding proteins maintain desired binding to the various desired species of ASGR, ASGR-1 and/or ASGR-2.

Antigen binding proteins of the invention (e.g., antibodies) can comprise any constant region known in the art. The light chain constant region can be, for example, a kappa- or lambda-type light chain constant region, e.g., a human kappa- or lambda-type light chain constant region. The heavy chain constant region can be, for example, an alpha-, delta-, epsilon-, gamma-, or mu-type heavy chain constant regions, e.g., a human alpha-, delta-, epsilon-, gamma-, or mu-type heavy chain constant region.

Techniques are known for deriving an antibody of a different subclass or isotype from an antibody of interest, i.e., subclass switching. Thus, IgG antibodies may be derived from an IgM antibody, for example, and vice versa. Such techniques allow the preparation of new antibodies that possess the antigen-binding properties of a given antibody (the parent antibody), but also exhibit biological properties associated with an antibody isotype or subclass different from that of the parent antibody. Recombinant DNA techniques may be employed. Cloned DNA encoding particular antibody polypeptides may be employed in such procedures, e.g., DNA encoding the constant domain of an antibody of the desired isotype. See also Lanitto et al., Methods Mol. Biol. 178:303-16 (2002).

In one embodiment, an antigen binding protein of the invention further comprises the constant light chain kappa or lambda domains or a fragment of these. Exemplary sequences of the light chain constant regions and polynucleotides encoding them are provided in Table 15 below, and are generally well known in the art. In another embodiment, an antigen binding protein of the invention further comprises a heavy chain constant domain, or a fragment thereof, such as the IgG1 or IgG2 heavy chain constant region provided in Table 15.

The antigen binding proteins (for example, antibodies) of the present invention include those having a desired isotype (for example, IgA, IgG1, IgG2, IgG3, IgG4, IgM, IgE, and IgD) as well as Fab or F(ab')$_2$ fragments thereof. Moreover, if an IgG4 is desired, it may also be desired to introduce a point mutation in the hinge region as described in Bloom et al., 1997, Protein Science 6:407, (incorporated by reference herein) to alleviate a tendency to form intra-H chain disulfide bonds that can lead to heterogeneity in the IgG4 antibodies.

Generation of antibodies

Antibodies of the invention may be prepared by techniques that are well known to those skilled in the art. For example, by immunizing an animal (e.g., a mouse or rat or rabbit) and then by immortalizing spleen cells harvested from the animal after completion of the immunization schedule. The spleen cells can be immortalized using any technique known in the art, e.g., by fusing them with myeloma cells to produce hybridomas. See, for example, Antibodies; Harlow and Lane, Cold Spring Harbor Laboratory Press, 1st Edition, e.g. from 1988, or 2nd Edition, e.g. from 2014).

In one embodiment, a humanized monoclonal antibody comprises the variable domain of a murine antibody (or all or part of the antigen binding site thereof) and a constant domain derived from a human antibody. Alternatively, a humanized antibody fragment may comprise the antigen binding site of a murine monoclonal antibody and a variable domain fragment (lacking the antigen-binding site) derived from a human antibody. Procedures for the production of engineered monoclonal antibodies include those described in Riechmann et al., 1988, Nature 332:323, Liu et al., 1987, Proc. Nat. Acad. Sci. USA 84:3439, Larrick et al., 1989, Bio/Technology 7:934, and Winter et al., 1993, TIPS 14:139. In one embodiment, the chimeric antibody is a CDR grafted antibody. Techniques for humanizing antibodies are discussed in, e.g., U.S. Pat. No.s 5,869,619; 5,225,539; 5,821,337; 5,859,205; 6,881,557, Padlan et al., 1995, FASEB J. 9:133-39, Tamura et al., 2000, J. Immunol. 164:1432-41, Zhang, W., et al., Molecular Immunology. 42(12):1445-1451, 2005; Hwang W. et al., Methods. 36(1):35-42, 2005; Dall'Acqua WF, et al., Methods 36(1):43-60, 2005; and Clark, M., Immunology Today. 21(8):397-402, 2000.

An antibody of the present invention may also be a fully human monoclonal antibody. Fully human monoclonal antibodies may be generated by any number of techniques with which those having ordinary skill in the art will be familiar. Such methods include, but are not limited to, Epstein Barr Virus (EBV) transformation of human peripheral blood cells (e.g., containing B lymphocytes), in vitro immunization of human B-cells, fusion of spleen cells from immunized transgenic mice carrying inserted human immunoglobulin genes, isolation from human immunoglobulin V region phage libraries, or other procedures as known in the art and based on the disclosure herein.

Procedures have been developed for generating human monoclonal antibodies in non-human animals. For example, mice in which one or more endogenous immunoglobulin genes have been inactivated by various means have been prepared. Human immunoglobulin genes have been introduced into the mice to replace the inactivated mouse genes. In this technique, elements of the human heavy and light chain locus are introduced into strains of mice derived from embryonic stem cell lines that contain targeted disruptions of the endogenous heavy chain and light chain loci (see also Bruggemann et al., Curr. Opin. Biotechnol. 8:455-58 (1997)). For example, human immunoglobulin transgenes may be mini-gene constructs, or transloci on yeast artificial chromosomes, which undergo B-cell-specific DNA rearrangement and hypermutation in the mouse lymphoid tissue.

Antibodies produced in the animal incorporate human immunoglobulin polypeptide chains encoded by the human genetic material introduced into the animal. In one embodiment, a non-human animal, such as a transgenic mouse, is immunized with a suitable immunogen.

Examples of techniques for production and use of transgenic animals for the production of human or partially human antibodies are described in U.S. Patents 5,814,318, 5,569,825, and 5,545,806, Davis et al., Production of human antibodies from transgenic mice in Lo, ed. Antibody Engineering: Methods and Protocols, Humana Press, NJ:191-200 (2003), Kellermann et al., 2002, Curr Opin Biotechnol. 13:593-97, Russel et al., 2000, Infect Immun. 68:1820-26, Gallo et al., 2000, Eur J Immun. 30:534-40, Davis et al., 1999, Cancer Metastasis Rev. 18:421-25, Green, 1999, J Immunol Methods. 231:11-23, Jakobovits, 1998, Advanced Drug Delivery Reviews 31:33-42, Green et al., 1998, J Exp Med. 188:483-95, Jakobovits A, 1998, Exp. Opin. Invest. Drugs. 7:607-14, Tsuda et al., 1997, Genomics. 42:413-21, Mendez et al., 1997, Nat Genet. 15:146-56, Jakobovits, 1994, Curr Biol. 4:761-63, Arbones et al., 1994, Immunity. 1:247-60, Green et al., 1994, Nat Genet. 7:13-21, Jakobovits et al., 1993, Nature. 362:255-58, Jakobovits et al., 1993, Proc Natl Acad Sci U S A. 90:2551-55. Chen, J., M. Trounstine, F. W. Alt, F. Young, C. Kurahara, J. Loring, D. Huszar. "Immunoglobulin gene rearrangement in B-cell deficient mice generated by targeted deletion of the JH locus." International Immunology 5 (1993): 647-656, Choi et al., 1993, Nature Genetics 4: 117-23, Fishwild et al., 1996, Nature Biotechnology 14: 845-51, Harding et al., 1995, Annals of the New York Academy of Sciences, Lonberg et al., 1994, Nature 368: 856-59, Lonberg, 1994, Transgenic Approaches to Human Monoclonal Antibodies in Handbook of Experimental Pharmacology 113: 49-101, Lonberg et al., 1995, Internal Review of Immunology 13: 65-93, Neuberger, 1996, Nature Biotechnology 14: 826, Taylor et al., 1992, Nucleic Acids Research 20: 6287-95, Taylor et al., 1994, International Immunology 6: 579-91, Tomizuka et al., 1997, Nature Genetics 16: 133-43, Tomizuka et al., 2000, Proceedings of the National Academy of Sciences USA 97: 722-27, Tuaillon et al., 1993, Proceedings of the National Academy of Sciences USA 90: 3720-24, and Tuaillon et al., 1994, Journal of Immunology 152: 2912-20.; Lonberg et al., Nature 368:856, 1994; Taylor et al., Int. Immun. 6:579, 1994; U.S. Patent No. 5,877,397; Bruggemann et al., 1997 Curr. Opin. Biotechnol. 8:455-58; Jakobovits et al., 1995 Ann. N. Y. Acad. Sci. 764:525-35. In addition, protocols involving the XenoMouse® (Abgenix, now Amgen, Inc.) are described, for example in U.S. 05/0118643 and WO 05/694879, WO 98/24838, WO 00/76310, and US Patent 7,064,244.

Lymphoid cells from the immunized transgenic mice are fused with myeloma cells for example to produce hybridomas. Myeloma cells for use in hybridoma-producing fusion procedures preferably are non-antibody-producing, have high fusion efficiency, and enzyme deficiencies that render them incapable of growing in certain selective media which support the growth of only the desired fused cells (hybridomas). Examples of suitable cell lines for use in such fusions include Sp-20, P3-X63/Ag8, P3-X63-Ag8.653, NS1/1.Ag 4 1, Sp210-Ag14, FO, NSO/U, MPC-11, MPC11-X45-GTG 1.7 and S194/5XX0 Bul; examples of cell lines used in rat fusions include R210.RCY3, Y3-Ag 1.2.3, IR983F and 4B210. Other cell lines useful for cell fusions are U-266, GM1500-GRG2, LICR-LON-HMy2 and UC729-6.

The lymphoid (e.g., spleen) cells and the myeloma cells may be combined for a few minutes with a membrane fusion-promoting agent, such as polyethylene glycol or a nonionic detergent, and then plated at low density on a selective medium that supports the growth of hybridoma cells but not unfused myeloma cells. One selection media is HAT (hypoxanthine, aminopterin, thymidine). After a sufficient time, usually about one to two weeks, colonies of cells are observed. Single colonies are isolated, and antibodies produced by the cells may be tested for binding activity to, for example, human ASGR-1, using any one of a variety of immunoassays known in the art and described herein. The hybridomas are cloned (e.g., by limited dilution cloning or by soft agar plaque isolation) and positive clones that produce an antibody specific to, for example, human ASGR-1, are selected and cultured. The monoclonal antibodies from the hybridoma cultures may be isolated from the supernatants of hybridoma cultures. Thus the present invention provides hybridomas that comprise polynucleotides encoding the antigen binding proteins of the invention in the chromosomes of the cell. These hybridomas can be cultured according to methods described herein and known in the art.

Another method for generating human antibodies of the invention includes immortalizing human peripheral blood cells by EBV transformation. See, e.g., U.S. Patent No. 4,464,456. Such an immortalized B-cell line (or lymphoblastoid cell line) producing a monoclonal antibody that specifically binds to, for example, human ASGR-1, can be identified by immunodetection methods as provided herein, for example, an ELISA, and then isolated by standard cloning techniques. The stability of the lymphoblastoid cell line producing an antibody may be improved by fusing the transformed cell line with a murine myeloma to produce a mouse-human hybrid cell line according to methods known in the art (see, e.g., Glasky et al., Hybridoma 8:377-89 (1989)). Still another method to generate human monoclonal antibodies is in vitro immunization, which includes priming human splenic B-cells with antigen, followed by fusion of primed B-cells with a heterohybrid fusion partner. See, e.g., Boerner et al., 1991 J. Immunol. 147:86-95.

In certain embodiments, a B-cell that is producing a desired antibody is selected and the light chain and heavy chain variable regions are cloned from the B-cell according to molecular biology techniques known in the art (WO 92/02551; U.S. patent 5,627,052; Babcook et al., Proc. Natl. Acad. Sci. USA 93:7843-48 (1996)) and described herein. B-cells from an immunized animal may be isolated from the spleen, lymph node, or peripheral blood sample by selecting a cell that is producing a desired antibody. B-cells may also be isolated from humans, for example, from a peripheral blood sample. Methods for detecting single B-cells that are producing an antibody with the desired specificity are well known in the art, for example, by plaque formation, fluorescence-activated cell sorting, in vitro stimulation followed by detection of specific antibody, and the like. Methods for selection of specific antibody-producing B-cells include, for example, preparing a single cell suspension of B-cells in soft agar that contains antigen. Binding of the specific antibody produced by the B-cell to the antigen results in the formation of a complex, which may be visible as an immunoprecipitate. After the B-cells producing the desired antibody are selected, the specific antibody genes may be cloned by isolating and amplifying DNA or mRNA according to methods known in the art and described herein.

An additional method for obtaining antibodies of the invention is by phage display. See, e.g., Winter et al., 1994 Annu. Rev. Immunol. 12:433-55; Burton et al., 1994 Adv. Immunol. 57:191-280. Human or murine immunoglobulin variable region gene combinatorial libraries may be created in phage vectors that can be screened to select Ig fragments (Fab, Fv, sFv, or multimers thereof) that bind specifically to TGF-beta binding protein or variant or fragment thereof. See, e.g., U.S. Patent No. 5,223,409; Huse et al., 1989 Science 246:1275-81; Sastry et al., Proc. Natl. Acad. Sci. USA 86:5728-32 (1989); Alting-Mees et al., Strategies in Molecular Biology 3:1-9 (1990); Kang et al., 1991 Proc. Natl. Acad. Sci. USA 88:4363-66; Hoogenboom et al., 1992 J. Molec. Biol. 227:381-388; Schlebusch et al., 1997 Hybridoma 16:47-52 and references cited therein. For example, a library containing a plurality of polynucleotide sequences encoding Ig variable region fragments may be inserted into the genome of a filamentous bacteriophage, such as M13 or a variant thereof, in frame with the sequence encoding a phage coat protein. A fusion protein may be a fusion of the coat protein with the light chain variable region domain and/or with the heavy chain variable region domain. According to certain embodiments, immunoglobulin Fab fragments may also be displayed on a phage particle (see, e.g., U.S. Patent No. 5,698,426).

Heavy and light chain immunoglobulin cDNA expression libraries may also be prepared in lambda phage, for example, using λImmunoZap™(H) and λImmunoZap™(L) vectors (Stratagene, La Jolla, California). Briefly, mRNA is isolated from a B-cell population, and used to create heavy and light chain immunoglobulin cDNA expression libraries in the λImmunoZap(H) and λImmunoZap(L) vectors. These vectors may be screened individually or co-expressed to form Fab fragments or antibodies (see Huse et al., supra; see also Sastry et al., supra). Positive plaques may subsequently be converted to a non-lytic plasmid that allows high level expression of monoclonal antibody fragments from E. coli.

In one embodiment, in a hybridoma the variable regions of a gene expressing a monoclonal antibody of interest are amplified using nucleotide primers. These primers may be synthesized by one of ordinary skill in the art, or may be purchased from commercially available sources. (See, e.g., Stratagene (La Jolla, California), which sells primers for mouse and human variable regions including, among others, primers for $V_{Ha}$, $V_{Hb}$, $V_{Hc}$, $V_{Hd}$, $C_{Hl}$, $V_L$ and $C_L$ regions.) These primers may be used to amplify heavy or light chain variable regions, which may then be inserted into vectors such as ImmunoZAP™H or ImmunoZAP™L (Stratagene), respectively. These vectors may then be introduced into E. coli, yeast, or mammalian-based systems for expression. Large amounts of a single-chain protein containing a fusion of the $V_H$ and $V_L$ domains may be produced using these methods (see Bird et al., Science 242:423-426, 1988).

In certain embodiments, the antigen binding proteins of the invention are obtained from transgenic animals (e.g., mice) that produce "heavy chain only" antibodies or "HCAbs." HCAbs are analogous to naturally occurring camel and llama single-chain VHH antibodies.

See, for example, U.S. Patent Nos. 8,507,748 and 8,502,014, and U.S. Patent Application Publication Nos. US2009/0285805A1, US2009/0169548A1, US2009/0307787A1, US2011/0314563A1, US2012/0151610A1, WO2008/122886A2, and WO2009/013620A2.

Once cells producing antibodies according to the invention have been obtained using any of the above-described immunization and other techniques, the specific antibody genes may be cloned by isolating and amplifying DNA or mRNA therefrom according to standard procedures as described herein. The antibodies produced therefrom may be sequenced and the CDRs identified and the DNA coding for the CDRs may be manipulated as described previously to generate other antibodies according to the invention.

In certain embodiments, antibodies are generated by first identifying antibodies that bind to cells expressing, for example, human ASGR, human ASGR-1 and/or human ASGR-2, and/or compete for binding with the antibodies described in this application.

It will be understood by one skilled in the art that some proteins, such as antibodies, may undergo a variety of posttranslational modifications. The type and extent of these modifications often depends on the host cell line used to express the protein as well as the culture conditions. Such modifications may include variations in glycosylation, methionine oxidation, diketopiperizine formation, aspartate isomerization and asparagine deamidation. A frequent modification is the loss of a carboxy-terminal basic residue (such as lysine or arginine) due to the action of carboxypeptidases (as described in Harris, R.J. Journal of Chromatography 705:129-134, 1995).

An alternative method for production of a murine monoclonal antibody is to inject the hybridoma cells into the peritoneal cavity of a syngeneic mouse, for example, a mouse that has been treated (e.g., pristane-primed) to promote formation of ascites fluid containing the monoclonal antibody. Monoclonal antibodies can be isolated and purified by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)," in Methods in Molecular Biology, Vol. 10, pages 79-104 (The Humana Press, Inc. 1992)). Monoclonal antibodies may be purified by affinity chromatography using an appropriate ligand selected based on particular properties of the antibody (e.g., heavy or light chain isotype, binding specificity, etc.). Examples of a suitable ligand, immobilized on a solid support, include Protein A, Protein G, an anticonstant region (light chain or heavy chain) antibody, an anti-idiotype antibody, and a TGF-beta binding protein, or fragment or variant thereof.

Molecular evolution of the complementarity determining regions (CDRs) in the center of the antibody binding site also has been used to isolate antibodies with increased affinity, for example, those as described by Schier et al., 1996, J. Mol. Biol. 263:551. Accordingly, such techniques are useful in preparing antibodies of the invention.

Although human, partially human, or humanized antibodies will be suitable for many applications, particularly those involving administration of the antibody to a human subject, other types of antigen binding proteins will be suitable for certain applications. The non-human antibodies of the invention can be, for example, derived from any antibody-producing animal, such as mouse, rat, rabbit, goat, donkey, or non-human primate (for example, monkey such as cynomologous or rhesus monkey) or ape (e.g., chimpanzee)). Non-human antibodies of the invention can be used, for example, in in vitro and cell-culture based applications, or any other application where an immune response to the antibody of the invention does not occur, is insignificant, can be prevented, is not a concern, or is desired. An antibody from a particular species can be made by, for example, immunizing an animal of that species with the desired immunogen or using an artificial system for generating antibodies of that species (e.g., a bacterial or phage display-based system for generating antibodies of a particular species), or by converting an antibody from one species into an antibody from another species by replacing, e.g., the constant region of the antibody with a constant region from the other species, or by replacing one or more amino acid residues of the antibody so that it more closely resembles the sequence of an antibody from the other species. In one embodiment, the antibody is a chimeric antibody comprising amino acid sequences derived from antibodies from two or more different species.

Antibodies also may be prepared by any of a number of other conventional techniques. For example, they may be purified from cells that naturally express them (e.g., an antibody can be purified from a hybridoma that produces it), or produced in recombinant expression systems, using any technique known in the art. See, for example, Monoclonal Antibodies, Hybridomas: A New Dimension in Biological Analyses, Kenneth et al. (eds.), Plenum Press, New York (1980); and Antibodies: A Laboratory Manual, Harlow and Land (eds.), Cold Spring Harbor Laboratory Press, Cold Spring Harbor, NY, (1988).

Where it is desired to improve the affinity of antibodies according to the invention containing one or more of the above-mentioned CDRs can be obtained by a number of affinity maturation protocols including maintaining the CDRs (Yang et al., J. Mol. Biol., 254, 392-403, 1995), chain shuffling (Marks et al., Bio/Technology, 10, 779-783, 1992), use of mutation strains of E. coli. (Low et al., J. Mol. Biol., 250, 350-368, 1996), DNA shuffling (Patten et al., Curr. Opin. Biotechnol., 8, 724-733, 1997), phage display (Thompson et al., J. Mol. Biol., 256, 7-88, 1996) and additional PCR techniques (Crameri, et al., Nature, 391, 288-291, 1998). All of these methods of affinity maturation are discussed by Vaughan et al. (Nature Biotechnology, 16, 535-539, 1998).

Single chain antibodies may be formed by linking heavy and light chain variable domain (Fv region) fragments via an amino acid bridge (short peptide linker), resulting in a single polypeptide chain. Such single-chain Fvs (scFvs) have been prepared by fusing DNA encoding a peptide linker between DNAs encoding the two variable domain polypeptides ($V_L$ and $V_H$). The resulting polypeptides can fold back on themselves to form antigen-binding monomers, or they can form multimers (e.g., dimers, trimers, or tetramers), depending on the length of a flexible linker between the two variable domains (Kortt et al., 1997, Prot. Eng. 10:423; Kortt et al., 2001, Biomol. Eng. 18:95-108). By combining different $V_L$ and $V_H$-comprising polypeptides, one can form multimeric scFvs that bind to different epitopes (Kriangkum et al., 2001, Biomol. Eng. 18:31-40). Techniques developed for the production of single chain antibodies include those described in U.S. Patent No. 4,946,778; Bird, 1988, Science 242:423; Huston et al., 1988, Proc. Natl. Acad. Sci. USA 85:5879; Ward et al., 1989, Nature 334:544, de Graaf et al., 2002, Methods Mol Biol. 178:379-87.

Antigen binding fragments derived from an antibody can also be obtained, for example, by proteolytic hydrolysis of the antibody, for example, pepsin or papain digestion of whole antibodies according to conventional methods. By way of example, antibody fragments can be produced by enzymatic cleavage of antibodies with pepsin to provide a 5S fragment termed $F(ab')_2$. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using papain produces two monovalent Fab fragments and an Fc fragment directly. These methods are described, for example, by Goldenberg, U.S. Patent No. 4,331,647, Nisonoff et al., Arch. Biochem. Biophys. 89:230, 1960; Porter, Biochem. J. 73:119, 1959;

Edelman et al., in Methods in Enzymology 1:422 (Academic Press 1967); and by Andrews, S.M. and Titus, J.A. in Current Protocols in Immunology (Coligan J.E., et al., eds), John Wiley & Sons, New York (2003), pages 2.8.1-2.8.10 and 2.10A.1-2.10A.5. Other methods for cleaving antibodies, such as separating heavy chains to form monovalent light-heavy chain fragments (Fd), further cleaving of fragments, or other enzymatic, chemical, or genetic techniques may also be used, so long as the fragments bind to the antigen that is recognized by the intact antibody.

Another exemplary form of an antigen binding protein is a peptide comprising one or more complementarity determining regions (CDRs) of an antibody. CDRs can be obtained by constructing polynucleotides that encode the CDR of interest. Such polynucleotides are prepared, for example, by using the polymerase chain reaction to synthesize the variable region using mRNA of antibody-producing cells as a template (see, for example, Larrick et al., Methods: A Companion to Methods in Enzymology 2:106, 1991; Courtenay-Luck, "Genetic Manipulation of Monoclonal Antibodies," in Monoclonal Antibodies: Production, Engineering and Clinical Application, Ritter et al. (eds.), page 166 (Cambridge University Press 1995); and Ward et al., "Genetic Manipulation and Expression of Antibodies," in Monoclonal Antibodies: Principles and Applications, Birch et al., (eds.), page 137 (Wiley-Liss, Inc. 1995)). The antibody fragment further may comprise at least one variable region domain of an antibody described herein. Thus, for example, the V region domain may be monomeric and be a $V_H$ or $V_L$ domain, which is capable of independently binding a desired target (e.g., human ASGR-1) with an affinity at least equal to $10^{-7}$M or less as described herein.

The variable region may be any naturally occurring variable domain or an engineered version thereof. By engineered version is meant a variable region that has been created using recombinant DNA engineering techniques. Such engineered versions include those created, for example, from a specific antibody variable region by insertions, deletions, or changes in or to the amino acid sequences of the specific antibody. One of ordinary skill in the art can use any known methods for identifying amino acid residues appropriate for engineering, such as the amino acid residues depicted with shading in Tables 21-48 of Figure 56. Additional examples include engineered variable regions containing at least one CDR and optionally one or more framework amino acids from a first antibody and the remainder of the variable region domain from a second antibody. Engineered versions of antibody variable domains may be generated by any number of techniques with which those having ordinary skill in the art will be familiar, including but not limited to the methods outlined in Example 14 below.

The variable region may be covalently attached at a C-terminal amino acid to at least one other antibody domain or a fragment thereof. Thus, for example, a VH that is present in the variable region may be linked to an immunoglobulin CH1 domain. Similarly a $V_L$ domain may be linked to a $C_K$ domain. In this way, for example, the antibody may be a Fab fragment wherein the antigen binding domain contains associated $V_H$ and $V_L$ domains covalently linked at their C-termini to a CH1 and $C_K$ domain, respectively. The CH1 domain may be extended with further amino acids, for example to provide a hinge region or a portion of a hinge region domain as found in a Fab' fragment, or to provide further domains, such as antibody CH2 and CH3 domains.

Derivatives and variants

The nucleotide sequences of the antigen binding proteins of the present invention, encoding the corresponding amino acid sequences of the antibodies of the present invention, can be altered, for example, by random mutagenesis or by site-directed mutagenesis (e.g., oligonucleotide-directed site-specific mutagenesis) to create an altered polynucleotide comprising one or more particular nucleotide substitutions, deletions, or insertions as compared to the non-mutated polynucleotide. Examples of techniques for making such alterations are described in Walder et al., 1986, Gene 42:133; Bauer et al. 1985, Gene 37:73; Craik, BioTechniques, January 1985, 12-19; Smith et al., 1981, Genetic Engineering: Principles and Methods, Plenum Press; and U.S. Patent Nos. 4,518,584 and 4,737,462. These and other methods can be used to make, for example, derivatives of the antigen binding proteins that have a desired property, for example, increased affinity, avidity, or specificity for a desired target, increased activity or stability in vivo or in vitro, or reduced in vivo side-effects as compared to the underivatized antibody.

Other derivatives of the antigen binding proteins within the scope of this invention include covalent or aggregative conjugates of the antigen binding proteins, with other proteins or polypeptides, such as by expression of recombinant fusion proteins comprising heterologous polypeptides fused to the N-terminus or C-terminus of a polypeptide. For example, the conjugated peptide may be a heterologous signal (or leader) polypeptide, e.g., the yeast alpha-factor leader, or a peptide such as an epitope tag. Antigen binding protein-containing fusion proteins can comprise peptides added to facilitate purification or identification of antigen binding protein (e.g., poly-His). An antigen binding protein also can be linked to the FLAG peptide as described in Hopp et al., Bio/Technology 6:1204, 1988, and U.S. Patent 5,011,912. The FLAG peptide is highly antigenic and provides an epitope reversibly bound by a specific monoclonal antibody (mAb), enabling rapid assay and facile purification of expressed recombinant protein. Reagents useful for preparing fusion proteins in which the FLAG peptide is fused to a given polypeptide are commercially available (Sigma, St. Louis, MO).

In another embodiment, the antigen binding proteins within the scope of this invention include antibody conjugates where antibody is conjugated to a non-proteinaceous chemical (drug) to form an antibody drug conjugate (ADC).

Generally the ADC comprises an antibody conjugated to a chemotherapeutic agent, e.g., a cytotoxic agent, a cytostatic agent, a toxin, or a radioactive agent. A linker molecule can be used to conjugate the drug to the antibody. A wide variety of linkers and drugs useful in ADC technology are known in the art and may be used in embodiments of the present invention. (See US20090028856; US2009/0274713; US2007/0031402; WO2005/084390; WO2009/099728; US5208020; US5416064; US5475092; 5585499; 6436931; 6372738; and 6340701, all incorporated herein by reference).

In another embodiment, oligomers that contain one or more antigen binding proteins may be employed in certain embodiments of the present invention. Oligomers may be in the form of covalently-linked or non-covalently-linked dimers, trimers, or higher oligomers. Oligomers comprising two or more antigen binding protein are contemplated for use, with one example being a homodimer. Other oligomers include heterodimers, homotrimers, heterotrimers, homotetramers, heterotetramers, etc.

One embodiment is directed to oligomers comprising multiple antigen binding proteins joined via covalent or non-covalent interactions between peptide moieties fused to the antigen binding proteins. Such peptides may be peptide linkers (spacers), or peptides that have the property of promoting oligomerization. Leucine zippers and certain polypeptides derived from antibodies are among the peptides that can promote oligomerization of antigen binding proteins attached thereto, as described in more detail below.

In particular embodiments, the oligomers comprise from two to four antigen binding proteins. The antigen binding proteins of the oligomer may be in any form, such as any of the forms described above, e.g., variants.

In one embodiment, an oligomer is prepared using polypeptides derived from immunoglobulins. Preparation of fusion proteins comprising certain heterologous polypeptides fused to various portions of antibody-derived polypeptides (including the Fc domain) has been described, e.g., by Ashkenazi et al., 1991, PNAS USA 88:10535; Byrn et al., 1990, Nature 344:677; and Hollenbaugh et al., 1992 "Construction of Immunoglobulin Fusion Proteins", in Current Protocols in Immunology, Suppl. 4, pages 10.19.1 - 10.19.11.

One embodiment of the present invention is directed to a dimer comprising two fusion proteins created by fusing an antigen binding fragment of an anti-ASGR, ASGR-1, and/or ASGR-2 antibody to the Fc region of an antibody. The dimer can be made by, for example, inserting a gene fusion encoding the fusion protein into an appropriate expression vector, expressing the gene fusion in host cells transformed with the recombinant expression vector, and allowing the expressed fusion protein to assemble much like antibody molecules, whereupon interchain disulfide bonds form between the Fc moieties to yield the dimer.

The term "Fc polypeptide" as used herein includes native and mutein forms of polypeptides derived from the Fc region of an antibody. Truncated forms of such polypeptides containing the hinge region that promotes dimerization also are included. Fusion proteins comprising Fc moieties (and oligomers formed therefrom) offer the advantage of facile purification by affinity chromatography over Protein A or Protein G columns.

One suitable Fc polypeptide, described in PCT application WO 93/10151 (hereby incorporated by reference), is a single chain polypeptide extending from the N-terminal hinge region to the native C-terminus of the Fc region of a human IgG1 antibody. Another useful Fc polypeptide is the Fc mutein described in U.S. Patent 5,457,035 and in Baum et al., 1994, EMBO J. 13:3992-4001. The amino acid sequence of this mutein is identical to that of the native Fc sequence presented in WO 93/10151, except that amino acid 19 has been changed from Leu to Ala, amino acid 20 has been changed from Leu to Glu, and amino acid 22 has been changed from Gly to Ala. The mutein exhibits reduced affinity for Fc receptors.

In some embodiments, the variable portion of the heavy and/or light chains of a desired antibody may be substituted for the variable portion of an antibody heavy and/or light chain.

Alternatively, the oligomer is a fusion protein comprising multiple antigen binding proteins, with or without peptide linkers (spacer peptides). Among the suitable peptide linkers are those described in U.S. Patents 4,751,180 and 4,935,233.

Another method for preparing oligomeric antigen binding proteins involves use of a leucine zipper. Leucine zipper domains are peptides that promote oligomerization of the proteins in which they are found. Leucine zippers were originally identified in several DNA-binding proteins (Landschulz et al., 1988, Science 240:1759), and have since been found in a variety of different proteins. Among the known leucine zippers are naturally occurring peptides and derivatives thereof that dimerize or trimerize. Examples of leucine zipper domains suitable for producing soluble oligomeric proteins are described in PCT application WO 94/10308, and the leucine zipper derived from lung surfactant protein D (SPD) described in Hoppe et al., 1994, FEES Letters 344:191, hereby incorporated by reference. The use of a modified leucine zipper that allows for stable trimerization of a heterologous protein fused thereto is described in Fanslow et al., 1994, Semin. Immunol. 6:267-78. In one approach, recombinant fusion proteins comprising a desired antibody fragment or derivative fused to a leucine zipper peptide are expressed in suitable host cells, and the soluble oligomeric antibody fragments or derivatives that form are recovered from the culture supernatant.

In another embodiment, the antigen binding proteins (e.g., antibodies) can be conjugated to a suitable vehicle to enhance the half-life thereof. Suitable vehicles include, but are not limited to Fc, albumin, transferrin, and the like. These and other suitable vehicles are known in the art. Such conjugated CDR peptides may be in monomeric, dimeric, tetrameric, or other form. In one embodiment, one or more water-soluble polymer is bonded at one or more specific position, for example at the amino terminus, of a binding agent. In an example, an antibody derivative comprises one or more water soluble polymer attachments, including, but not limited to, polyethylene glycol, polyoxyethylene glycol, or polypropylene glycol. See, e.g., U.S. Pat. Nos. 4,640,835, 4,496,689, 4,301,144, 4,670,417, 4,791,192 and 4,179,337. In certain embodiments, a derivative comprises one or more of monomethoxy-polyethylene glycol, dextran, cellulose, or other carbohydrate based polymers, poly-(N-vinyl pyrrolidone)-polyethylene glycol, propylene glycol homopolymers, a polypropylene oxide/ethylene oxide co-polymer, polyoxyethylated polyols (e.g., glycerol) and polyvinyl alcohol, as well as mixtures of such polymers. In certain embodiments, one or more water-soluble polymer is randomly attached to one or more side chains. In certain embodiments, PEG can act to improve the therapeutic capacity for a binding agent, such as an antibody. Certain such methods are discussed, for example, in U.S. Pat. No. 6,133,426, which is hereby incorporated by reference for any purpose. In certain embodiments, antibodies of the invention may be chemically bonded with polymers, lipids, or other moieties.

Nucleic acids encoding antigen binding proteins

In another embodiment, the present invention provides isolated nucleic acid molecules that encode the antigen binding proteins of the present invention. In addition, provided are vectors comprising the nucleic acids, cell comprising the nucleic acids, and methods of making the antigen binding proteins of the invention. The nucleic acids comprise, for example, polynucleotides that encode all or part of an antigen binding protein, for example, one or both chains of an antibody of the invention, or a fragment, derivative, mutein, or variant thereof, polynucleotides sufficient for use as hybridization probes, PCR primers or sequencing primers for identifying, analyzing, mutating or amplifying a polynucleotide encoding a polypeptide, anti-sense nucleic acids for inhibiting expression of a polynucleotide, and complementary sequences of the foregoing. The nucleic acids can be any length as appropriate for the desired use or function, and can comprise one or more additional sequences, for example, regulatory sequences, and/or be part of a larger nucleic acid, for example, a vector. The nucleic acids can be single-stranded or double-stranded and can comprise RNA and/or DNA nucleotides, and artificial variants thereof (e.g., peptide nucleic acids).

Nucleic acids encoding antibody polypeptides (e.g., heavy or light chain, variable domain only, or full length) may be isolated from B-cells of mice that have been immunized with antigen. The nucleic acid may be isolated by conventional procedures such as polymerase chain reaction (PCR).

Nucleic acid sequences encoding the variable regions of the heavy and light chain variable regions are included herein. The skilled artisan will appreciate that, due to the degeneracy of the genetic code, each of the polypeptide sequences disclosed herein is encoded by a large number of other nucleic acid sequences. The present invention provides each degenerate nucleotide sequence encoding each antigen binding protein of the invention.

The invention further provides nucleic acids that hybridize to other nucleic acids under particular hybridization conditions. Methods for hybridizing nucleic acids are well-known in the art. See, e.g., Current Protocols in Molecular Biology, John Wiley & Sons, N.Y. (1989), 6.3.1-6.3.6. As defined herein, for example, a moderately stringent hybridization condition uses a prewashing solution containing 5X sodium chloride/sodium citrate (SSC), 0.5% SDS, 1.0 mM EDTA (pH 8.0), hybridization buffer of about 50% formamide, 6X SSC, and a hybridization temperature of 55° C (or other similar hybridization solutions, such as one containing about 50% formamide, with a hybridization temperature of 42° C), and washing conditions of 60° C, in 0.5X SSC, 0.1% SDS. A stringent hybridization condition hybridizes in 6X SSC at 45° C, followed by one or more washes in 0.1X SSC, 0.2% SDS at 68° C. Furthermore, one of skill in the art can manipulate the hybridization and/or washing conditions to increase or decrease the stringency of hybridization such that nucleic acids comprising nucleotide sequences that are at least 65, 70, 75, 80, 85, 90, 95, 98 or 99% identical to each other typically remain hybridized to each other. The basic parameters affecting the choice of hybridization conditions and guidance for devising suitable conditions are set forth by, for example, Sambrook, Fritsch, and Maniatis (1989, Molecular Cloning: A Laboratory Manual, Cold Spring Harbor Laboratory Press, Cold Spring Harbor, N.Y., chapters 9 and 11; and Current Protocols in Molecular Biology, 1995, Ausubel et al., eds., John Wiley & Sons, Inc., sections 2.10 and 6.3-6.4), and can be readily determined by those having ordinary skill in the art based on, for example, the length and/or base composition of the DNA. Changes can be introduced by mutation into a nucleic acid, thereby leading to changes in the amino acid sequence of a polypeptide (e.g., an antigen binding protein) that it encodes. Mutations can be introduced using any technique known in the art. In one embodiment, one or more particular amino acid residues are changed using, for example, a site-directed mutagenesis protocol. In another embodiment, one or more randomly selected residues is changed using, for example, a random mutagenesis protocol. However it is made, a mutant polypeptide can be expressed and screened for a desired property.

Mutations can be introduced into a nucleic acid without significantly altering the biological activity of a polypeptide that it encodes. For example, one can make nucleotide substitutions leading to amino acid substitutions at non-essential amino acid residues. In one embodiment, a nucleotide sequence provided herein for of the antibodies of the present invention, or a desired fragment, variant, or derivative thereof, is mutated such that it encodes an amino acid sequence comprising one or more deletions or substitutions of amino acid residues that are shown herein for the light chains of the antibodies of the present invention or the heavy chains of the antibodies of the present invention to be residues where two or more sequences differ. In another embodiment, the mutagenesis inserts an amino acid adjacent to one or more amino acid residues shown herein for the light chains of the antibodies of the present invention or the heavy chains of the antibodies of the present invention to be residues where two or more sequences differ. Alternatively, one or more mutations can be introduced into a nucleic acid that selectively change the biological activity of a polypeptide that it encodes.

In another embodiment, the present invention provides vectors comprising a nucleic acid encoding a polypeptide of the invention or a portion thereof. Examples of vectors include, but are not limited to, plasmids, viral vectors, non-episomal mammalian vectors and expression vectors, for example, recombinant expression vectors.

The recombinant expression vectors of the invention can comprise a nucleic acid of the invention in a form suitable for expression of the nucleic acid in a host cell. The recombinant expression vectors include one or more regulatory sequences, selected on the basis of the host cells to be used for expression, which is operably linked to the nucleic acid sequence to be expressed. Regulatory sequences include those that direct constitutive expression of a nucleotide sequence in many types of host cells (e.g., SV40 early gene enhancer, Rous sarcoma virus promoter and cytomegalovirus promoter), those that direct expression of the nucleotide sequence only in certain host cells (e.g., tissue-specific regulatory sequences, see Voss et al., 1986, Trends Biochem. Sci. 11:287, Maniatis et al., 1987, Science 236:1237, incorporated by reference herein in their entireties), and those that direct inducible expression of a nucleotide sequence in response to particular treatment or condition (e.g., the metallothionin promoter in mammalian cells and the tet-responsive and/or streptomycin responsive promoter in both prokaryotic and eukaryotic systems (see id.). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein.

In another embodiment, the present invention provides host cells into which a recombinant expression vector of the invention has been introduced. A host cell can be any prokaryotic cell or eukaryotic cell. Prokaryotic host cells include gram negative or gram positive organisms, for example E. coli or bacilli. Higher eukaryotic cells include insect cells, yeast cells, and established cell lines of mammalian origin. Examples of suitable mammalian host cell lines include Chinese hamster ovary (CHO) cells or their derivatives such as Veggie CHO and related cell lines which grow in serum-free media (see Rasmussen et al., 1998, Cytotechnology 28:31) or CHO strain DXB-11, which is deficient in DHFR (see Urlaub et al., 1980, Proc. Natl. Acad. Sci. USA 77:4216-20). Additional CHO cell lines include CHO-K1 (ATCC#CCL-61), EM9 (ATCC# CRL-1861), and UV20 (ATCC# CRL-1862). Additional host cells include the COS-7 line of monkey kidney cells (ATCC CRL 1651) (see Gluzman et al., 1981, Cell 23:175), L cells, C127 cells, 3T3 cells (ATCC CCL 163), AM-1/D cells (described in U.S. Patent No. 6,210,924), HeLa cells, BHK (ATCC CRL 10) cell lines, the CV1/EBNA cell line derived from the African green monkey kidney cell line CV1 (ATCC CCL 70) (see McMahan et al., 1991, EMBO J. 10:2821), human embryonic kidney cells such as 293, 293 EBNA or MSR 293, human epidermal A431 cells, human Colo205 cells, other transformed primate cell lines, normal diploid cells, cell strains derived from in vitro culture of primary tissue, primary explants, HL-60, U937, HaK or Jurkat cells. Appropriate cloning and expression vectors for use with bacterial, fungal, yeast, and mammalian cellular hosts are described by Pouwels et al. (Cloning Vectors: A Laboratory Manual, Elsevier, New York, 1985).

Vector DNA can be introduced into prokaryotic or eukaryotic cells via conventional transformation or transfection techniques. For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., for resistance to antibiotics) is generally introduced into the host cells along with the gene of interest. Additional selectable markers include those which confer resistance to drugs, such as G418, hygromycin and methotrexate. Cells stably transfected with the introduced nucleic acid can be identified by drug selection (e.g., cells that have incorporated the selectable marker gene will survive, while the other cells die), among other methods.

The transformed cells can be cultured under conditions that promote expression of the polypeptide, and the polypeptide recovered by conventional protein purification procedures. Polypeptides contemplated for use herein include substantially homogeneous recombinant mammalian antibody polypeptides substantially free of contaminating endogenous materials.

Cells containing the nucleic acid encoding the antigen binding proteins of the present invention also include hybridomas. The production and culturing of hybridomas are discussed in the antibody section above.

In some emobodiments, a vector comprising a nucleic acid molecule as described herein is provided. In some embodiments, the invention comprises a host cell comprising a nucleic acid molecule as described herein.

In some emobodiments, a nucleic acid molecule encoding the antigen binding protein as described herein is provided.

In some emobodiments, a pharmaceutical composition comprising at least one antigen binding protein described herein is provided.

Antigen binding protein production

The antigen binding proteins of the invention can be produced by any method known in the art for the synthesis of proteins (e.g., antibodies), in particular, by chemical synthesis or preferably, by recombinant expression techniques.

Recombinant expression of the antigen binding proteins requires construction of an expression vector containing a polynucleotide that encodes the the antigen binding proteins. Once a polynucleotide encoding the antigen binding proteins molecule has been obtained, the vector for the production of the antigen binding proteins may be produced by recombinant DNA technology. An expression vector is constructed containing the antigen binding proteins coding sequences and appropriate transcriptional and translational control signals. These methods include, for example, in vitro recombinant DNA techniques, synthetic techniques, and in vivo genetic recombination.

The expression vector is transferred to a host cell by conventional techniques and the transfected cells are then cultured by conventional techniques to produce an antigen binding proteins of the invention. In one embodiment of the invention, vectors encoding both the heavy and light chains of an antibody may be co-expressed in the host cell for expression of the entire immunoglobulin molecule, as detailed below.

A variety of host-expression vector systems may be utilized to express the antigen binding proteins of the invention. Such host-expression systems represent vehicles by which the coding sequences of interest may be produced and subsequently purified, but also represent cells which may, when transformed or transfected with the appropriate nucleotide coding sequences, express an antibody molecule of the invention in situ. Bacterial cells such as E. coli, and eukaryotic cells are commonly used for the expression of a recombinant antibody molecule, especially for the expression of whole recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett et al., Bio/Technology 8:2 (1990)).

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, COS, 293, 3T3, or myeloma cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compounds that interact directly or indirectly with the antibody molecule.

A number of selection systems may be used, including but not limited to the herpes simplex virus thymidine kinase (Wigler et al., Cell 11:223 (1977)), hypoxanthine-guanine phosphoribosyltransferase (Szybalska & Szybalski, Proc. Natl. Acad. Sci. USA 48:202 (1992)), and adenine phosphoribosyltransferase (Lowy et al., Cell 22:817 (1980)) genes can be employed in tk, hgprt or aprt-cells, respectively. Also, antimetabolite resistance can be used as the basis of selection for the following genes: dhfr, which confers resistance to methotrexate (Wigler et al., Proc. Natl. Acad. Sci. USA 77:357 (1980); O'Hare et al., Proc. Natl. Acad. Sci. USA 78:1527 (1981)); gpt, which confers resistance to mycophenolic acid (Mulligan & Berg, Proc. Natl. Acad. Sci. USA 78:2072 (1981)); neo, which confers resistance to the aminoglycoside G-418 (Wu and Wu, Biotherapy 3:87-95 (1991)); and hygro, which confers resistance to hygromycin (Santerre et al., Gene 30:147 (1984)). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., J. Mol. Biol. 150:1 (1981), which are incorporated by reference herein in their entireties.

The expression levels of an antibody molecule can be increased by vector amplification (for a review, see Bebbington and Hentschel, "The use of vectors based on gene amplification for the expression of cloned genes in mammalian cells" (DNA Cloning, Vol. 3. Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., Mol. Cell. Biol. 3:257 (1983)).

The host cell may be co-transfected with two expression vectors of the invention, for example, the first vector encoding an antibody heavy chain derived polypeptide and the second vector encoding an antibody light chain derived polypeptide. The two vectors may contain identical selectable markers which enable equal expression of heavy and light chain polypeptides. Alternatively, a single vector may be used which encodes, and is capable of expressing, for example, both antibody heavy and light chain polypeptides. In such situations, the light chain should be placed before the heavy chain to avoid an excess of toxic free heavy chain (Proudfoot, Nature 322:52 (1986); Kohler, Proc. Natl. Acad. Sci. USA 77:2197 (1980)). The coding sequences for the heavy and light chains may comprise cDNA or genomic DNA.

Once an antibody molecule of the invention has been produced by an animal, chemically synthesized, or recombinantly expressed, it may be purified by any method known in the art for purification of an immunoglobulin molecule, for example, by chromatography (e.g., ion exchange, affinity, particularly by affinity for the specific antigen after Protein A, and size-exclusion chromatography), centrifugation, differential solubility, or by any other standard technique for the purification of proteins. In addition, the antibodies of the present invention or fragments thereof can be fused to heterologous polypeptide sequences described herein or otherwise known in the art, to facilitate purification.

In some embodiments, the present invention encompasses antibodies recombinantly fused or chemically conjugated (including both covalently and non-covalently conjugations) to a polypeptide. Fused or conjugated antibodies of the present invention may be used for ease in purification. See e.g., Harbor et al., supra, and PCT publication WO 93/21232; EP 439,095; Naramura et al., Immunol. Lett. 39:91-99 (1994); U.S. Pat. No. 5,474,981; Gillies et al., Proc. Natl. Acad. Sci. 89:1428-1432 (1992); Fell et al., J. Immunol. 146:2446-2452 (1991).

Moreover, the antibodies or fragments thereof of the present invention can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, Calif., 91311), among others, many of which are commercially available. As described in Gentz et al., Proc. Natl. Acad. Sci. USA 86:821-824 (1989), for instance, hexa-histidine provides for convenient purification of the fusion protein. Other peptide tags useful for purification include, but are not limited to, the "HA" tag, which corresponds to an epitope derived from the influenza hemagglutinin protein (Wilson et al., Cell 37:767 (1984)) and the "flag" tag.

Antibody Effector Function

In some embodiments, the present invention provides antigen binding proteins (e.g., antibodies) with altered effector function (e.g., decreasing or increasing effector function). Nonlimiting examples of methods for increasing effector function can be found in U.S. Pat. Nos. 5,624,821, 6,602,684, 7,029,872, U.S. Patent Application Publication Nos. 2006/0067930A1, 2005/0272128A1, 2005/0079605A1, 2005/0123546A1, 2004/0072290A1, 2006/0257399A1, 2004/0261148A1, 2007/0092521, 2006/0040325A1, and 2006/0039904A1, and International Patent Application Publication Nos. WO 04/029207, WO03011878, WO05044859, WO 06071856, and WO 06071280.

Methods of engineering Fc regions of antibodies so as to alter effector functions are known in the art (e.g., U.S. Patent Publication No. 20040185045 and PCT Publication No. WO 2004/016750, both to Koenig et al., which describe altering the Fc region to enhance the binding affinity for Fc gamma RIIB as compared with the binding affinity for FC gamma RIIA; see, also, PCT Publication Nos. WO 99/58572 to Armour et al., WO 99/51642 to Idusogie et al., and U.S. Pat. No. 6,395,272 to Deo et al.). Methods of modifying the Fc region to decrease binding affinity to Fc gamma RIIB are also known in the art (e.g., U.S. Patent Publication No. 20010036459 and PCT Publication No. WO 01/79299, both to Ravetch et al.). Modified antibodies having variant Fc regions with enhanced binding affinity for Fc gamma RIIIA and/or Fc gamma RIIA as compared with a wildtype Fc region have also been described (e.g., PCT Publication Nos. WO 2004/063351, to Stavenhagen et al., the disclosure of which is incorporated herein in its entirety).

Antibody effector function may also be modified through the generation of antibodies with altered glycosylation patterns. Such altered glycosylation patterns have been demonstrated to increase or decrease the ADCC ability of antibodies, as desired. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation.

Half-life alteration

In some embodiments, the present invention provides for antigen binding proteins (e.g., antibodies) which have an extended half-life in vivo. In particular, the present invention provides antigen binding proteins which have a half-life in a mammal (for example, but not limited to, a human), of greater than 3 days, greater than 7 days, greater than 10 days, greater than 15 days, greater than 25 days, greater than 30 days, greater than 35 days, greater than 40 days, greater than 45 days, greater than 2 months, greater than 3 months, greater than 4 months, or greater than 5 months.

To prolong the serum circulation of antigen binding proteins (for example, monoclonal antibodies) or antibody fragments (for example, Fab fragments) in vivo, for example, inert polymer molecules such as high molecular weight polyethyleneglycol (PEG) can be attached to the antibodies (including antibody fragments thereof) with or without a multifunctional linker either through site-specific conjugation of the PEG to the N- or C-terminus of the antibodies or via epsilon-amino groups present on lysine residues. Linear or branched polymer derivatization that results in minimal loss of biological activity will be used. The degree of conjugation can be closely monitored by SDS-PAGE and mass spectrometry to ensure proper conjugation of PEG molecules to the antigen binding proteins. Unreacted PEG can be separated from antigen binding proteins-PEG conjugates by size-exclusion or by ion-exchange chromatography. PEG-derivatized antigen binding proteins can be tested for binding activity as well as for in vivo efficacy using methods known to those of skill in the art, for example, by immunoassays described herein.

In certain embodiments, antibodies having an increased half-life in vivo can also be generated by introducing one or more amino acid modifications (i.e., substitutions, insertions or deletions) into an IgG constant domain, or FcRn binding fragment thereof (e.g., Fc or hinge Fc domain fragment). See, e.g., International Publication No. WO 98/23289; International Publication No. WO 97/34631; and U.S. Pat. No. 6,277,375, each of which is incorporated herein by reference in its entirety.

Conjugates

In some embodiments, covalent modifications of the antigen binding proteins of the invention are included within the scope of this invention. They may be made by chemical synthesis or by enzymatic or chemical cleavage of the antigen binding proteins, if applicable. Other types of covalent modifications of the antigen binding proteins are introduced into the molecule by reacting targeted amino acid residues of the antibody with an organic derivatizing agent that is capable of reacting with selected side chains or the N- or C-terminal residues.

Cysteinyl residues most commonly are reacted with alpha-haloacetates (and corresponding amines), such as chloroacetic acid or chloroacetamide, to give carboxymethyl or carboxyamidomethyl derivatives. Similarly, iodo-reagents may also be used. Cysteinyl residues also are derivatized by reaction with bromotrifluoroacetone, alpha-bromo-beta-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysyl and amino-terminal residues are reacted with succinic or other carboxylic acid anhydrides. Derivatization with these agents has the effect of reversing the charge of the lysinyl residues. Other suitable reagents for derivatizing .alpha.-amino-containing residues and/or e-amino-containing residues include imidoesters such as methyl picolinimidate, pyridoxal phosphate, pyridoxal, chloroborohydride, trinitrobenzenesulfonic acid, 0-methylisourea, 2,4-pentanedione, and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginyl residues generally requires that the reaction be performed in alkaline conditions because of the high pKa of the guanidine functional group. Furthermore, these reagents may react with the epsilon-amino groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using $I^{125}$ or $I^{131}$ to prepare labeled proteins for use in radioimmunoassay.

Carboxyl side groups (aspartyl or glutamyl) are selectively modified by reaction with carbodiimides (R-N==C==N-R'), where R and R' are different alkyl groups, such as 1-cyclohexyl-3-(2-morpholinyl-4-ethyl)carbodiimide or 1-ethyl-3-(4-azonia-4,4-dimethylpentyl)carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues, respectively. These residues are deamidated under neutral or basic conditions. The deamidated form of these residues falls within the scope of this invention.

Other modifications include hydroxylation of proline and lysine, phosphorylation of hydroxyl groups of seryl or threonyl residues, methylation of the .alpha.-amino groups of lysine, arginine, and histidine side chains (T. E. Creighton, Proteins: Structure and Molecular Properties, W.H. Freeman & Co., San Francisco, pp. 79-86 (1983)), acetylation of the N-terminal amine, and amidation of any C-terminal carboxyl group.

Another type of covalent modification involves chemically or enzymatically coupling glycosides to the antibody. These procedures are advantageous in that they do not require production of the antibody in a host cell that has glycosylation capabilities for N- or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published 11 Sep. 1987, and in Aplin and Wriston, CRC Crit. Rev. Biochem., pp. 259-306 (1981).

Interfering RNA

In some embodiments, the present invention provides polynucleotide compositions that target ASGR-1 and/or ASGR-2 and are useful for methods for treatment, therapy, and prophylaxis in disease related to ASGR, ASGR-1 and/or ASGR-2 expression, where reduction or inhibition of the expression or function of a selected target polynucleotide sequence is desired. Examples of polynucleotides that can be used to target ASGR-1 and/or ASGR-2 sequences and reduce ASGR-1 and/or ASGR-2 expression include, but are not limited to, antisense oligonucleotides, and RNA interference (RNAi) agents, including short or small interfering RNA (siRNA), short hairpin RNA (shRNA), and microRNA (miRNA). See, for example, U.S. Patent Nos. 6,506,559; 8,394,628; 7,056,704; 7,078,196; 6,107,094; 5,898,031; 6,573,099; and European Patent No. 1,144,623. See also, for example, U.S. patent application publication nos. 2015/0259689; 2015/0197746; 2011/0092565; U.S. Patent Nos. 8,877,917; 8,507,455; and 7,579,451.

In certain embodiments, a composition for inhibiting the function or expression of a target polynucleotide sequence (e.g. ASGR-1 mRNA sequence, ASGR-2 mRNA sequence) in a mammalian cell, according to this invention, comprises an agent that provides to a mammalian cell an at least partially double-stranded RNA molecule (e.g., an interfering RNA molecule). A double-stranded RNA molecule may include chemical modifications to ribonucleotides, including modifications to the ribose sugar, base, or backbone components of the ribonucleotides, such as those described herein or known in the art. Any such modifications, as used in a double-stranded RNA molecule (e.g. siRNA, shRNA, or the like), are encompassed by the term "double-stranded RNA" for the purposes of this disclosure. Thus, in general, the term "RNA" may also include RNA-DNA hybrids and polynucleotides comprising one or more modified nucleotides (e.g. nucleotides with modifications at the 2' position of the ribose ring), except where specified otherwise, e.g., where a 2'-OH group of ribose is required for a particular linkage.

In some embodiments at least 10% of a partially double-stranded RNA molecule is double-stranded. Alternatively, the double stranded portion of these RNA molecules can be at least 30% of the length of the molecule. In another embodiment, the double stranded portion of these molecules can be at least 50% of the the length of the molecule. In still another embodiment, the double stranded portion of these molecules can be at least 70% of the length of the molecule. In another embodiment, the double stranded portion of these molecules can be at least 90% of the length of the molecule. In another embodiment, the molecule can be double stranded over its entire length. Alternatively, the double-stranded portion of these molecules can occur at either or both termini, or in some middle portion of the molecule, if the molecule is linear. Similarly, the double-stranded portion can be in any location if the molecule is circular. In certain embodiments of the present invention, the double-stranded portion of the RNA molecule becomes double-stranded only when the molecule is in the mammalian cell. In still other embodiment of this invention, the partially double-stranded molecule is an RNA/DNA hybrid, for example, a single strand containing RNA and DNA, prepared in vitro; or a duplex of two such single strands or portions thereof. In yet another embodiment, the RNA molecule, made in vivo or in vitro, is a duplex comprised of an RNA single strand and a DNA single strand. In some embodiments, the partially double-stranded RNA molecule comprises a polynucleotide sequence that is substantially homologous to the target polynucleotide sequence in order to effectively reduce or inhibit the function or expression thereof. The necessary homology may be suitably defined by use of a computer algorithm. As known in the art and discussed herein, "homology" or "identity" means the degree of sequence relatedness between two polypeptide or two polynucleotide sequences as determined by the identity of the match between two lengths of such sequences. Both identity and homology can be readily calculated by methods in the prior art [See also, e.g., COMPUTATIONAL MOLECULAR BIOLOGY, Lesk, A. M., ed., Oxford University Press, New York, (1988); BIOCOMPUTING: INFORMATICS AND GENOME PROJECTS, Smith, D. W., ed., Academic Press, New York, (1993); COMPUTER ANALYSIS OF SEQUENCE DATA, PART I, Griffin, A. M., and Griffin, H. G., eds., Humana Press, New Jersey, (1994); SEQUENCE ANALYSIS IN MOLECULAR BIOLOGY, von Heinje, G., Academic Press, (1987); and SEQUENCE ANALYSIS PRIMER, Gribskov, M. and Devereux, J., eds., M Stockton Press, New York, (1991)]. While there exist a number of methods to measure identity and homology between two polynucleotide sequences, the terms "identity", "similarity" and homology are well known to skilled artisans [H. Carillo and D. Lipton, SIAM J. Applied Math., 48:1073 (1988)]. Methods commonly employed to determine identity or homology between two sequences include, but are not limited to, those disclosed in Guide to Huge Computers, Martin J. Bishop, ed., Academic Press, San Diego, 1994, and H. Carillo and D. Lipton, SIAM J. Applied Math., 48:1073 (1988). Preferred methods to determine identity or homology are designed to give the largest match between the two sequences tested. Methods to determine identity and similarity are codified in computer programs. Preferred computer program to determine identity and homology between two sequences include, but are not limited to, the algorithm BESTFIT from the GCG program package [J. Devereux et al., Nucl. Acids Res., 12(1):387 (1984)], the related MACVECTOR program (Oxford), and the FASTA (Pearson) programs. For instance, searches for sequence similarities in databases between significant naturally occurring mammalian polynucleotide sequences and target polynucleotide sequences enable the design of suitable RNA molecules desired for use in the invention. The algorithm and/or the degree of homology necessary for any particular RNA molecule may be selected by one of skill in the art, depending on the identity of the target, and/or the closeness of homology of the target sequence to any naturally occurring mammalian sequence, which is desired to be left functioning normally after use of the methods of this invention.

In some embodiments, a polynucleotide composition for reducing the expression or function of ASGR-1 and/or ASGR-2 sequences is an RNAi agent comprising a double-stranded RNA molecule which comprises two antiparallel strands of contiguous nucleotides that are sufficiently complementary to each other to hybridize to form a duplex region. "Hybridize" or "hybridization" refers to the pairing of complementary polynucleotides, typically via hydrogen bonding (e.g. Watson-Crick, Hoogsteen or reversed Hoogsteen hydrogen bonding) between complementary bases in the two polynucleotides. The strand comprising a region having a sequence that is substantially complementary to a target sequence (e.g. target mRNA) is referred to as the "antisense strand." The "sense strand" refers to the strand that includes a region that is substantially complementary to a region of the antisense strand. In some embodiments, the sense strand may comprise a region that has a sequence that is substantially identical to the target sequence.

As used herein, a first sequence is "complementary" to a second sequence if a polynucleotide comprising the first sequence can hybridize to a polynucleotide comprising the second sequence to form a duplex region under certain conditions, such as physiological conditions. Other such conditions can include moderate or stringent hybridization conditions, which are known to those of skill in the art. A first sequence is considered to be fully complementary (100% complementary) to a second sequence if a polynucleotide comprising the first sequence base pairs with a polynucleotide comprising the second sequence over the entire length of one or both nucleotide sequences without any mismatches. A sequence is "substantially complementary" to a target sequence if the sequence is at least about 80%, 85%, 90%, 95%, 96%, 97%, 98%, 99% or 100% complementary to a target sequence. Percent complementarity can be calculated by dividing the number of bases in a first sequence that are complementary to bases at corresponding positions in a second or target sequence by the total length of the first sequence. A sequence may also be said to be substantially complementary to another sequence if there are no more than 5, 4, 3, or 2 mismatches over a 30 base pair duplex region when the two sequences are hybridized. Generally, if any nucleotide overhangs, as defined herein, are present, the sequence of such overhangs is not considered in determining the degree of complementarity between two sequences. By way of example, a sense strand of 21 nucleotides in length and an antisense strand of 21 nucleotides in length that hybridize to form a 19 base pair duplex region with a 2 nucleotide overhang at the 3' end of each strand would be considered to be fully complementary as the term is used herein.

In some embodiments, a region of the antisense strand comprises a sequence that is fully complementary to a region of the target RNA sequence (e.g. ASGR-1 and/or ASGR-2 mRNA). In such embodiments, the sense strand may comprise a sequence that is fully complementary to the sequence of the antisense strand. In other such embodiments, the sense strand may comprise a sequence that is substantially complementary to the sequence of the antisense strand, e.g. having 1, 2, 3, 4, or 5 mismatches in the duplex region formed by the sense and antisense strands. In certain embodiments, it is preferred that any mismatches occur within the terminal regions (e.g. within 6, 5, 4, 3, or 2 nucleotides of the 5' and/or 3' ends of the strands). In one embodiment, any mismatches in the duplex region formed from the sense and antisense strands occur within 6, 5, 4, 3, or 2 nucleotides of the 5' end of the antisense strand.

In certain embodiments, the sense strand and antisense strand of the double-stranded RNA may be two separate molecules that hybridize to form a duplex region, but are otherwise unconnected. Such double-stranded RNA molecules formed from two separate strands are referred to as "small interfering RNAs" or "short interfering RNAs" (siRNAs).

In other embodiments, the sense strand and the antisense strand that hybridize to form a duplex region may be part of a single RNA molecule, i.e. the sense and antisense strands are part of a self-complementary region of a single RNA molecule. In such cases, a single RNA molecule comprises a duplex region (also referred to as a stem region) and a loop region. The 3' end of the sense strand is connected to the 5' end of the antisense strand by a contiguous sequence of unpaired nucleotides, which will form the loop region. The loop region is typically of a sufficient length to allow the RNA molecule to fold back on itself such that the antisense strand can base pair with the sense strand to form the duplex or stem region. The loop region can comprise from about 3 to about 25, from about 5 to about 15, or from about 8 to about 12 unpaired nucleotides. Such RNA molecules with at least partially self-complementary regions are referred to as "short hairpin RNAs" (shRNAs). The length of a single, at least partially self-complementary RNA molecule can be from about 35 nucleotides to about 100 nucleotides, from about 45 nucleotides to about 85 nucleotides, or from about 50 to about 60 nucleotides and comprise a duplex region and loop region each having the lengths recited herein.

In some embodiments, the double-stranded RNA molecule comprises a sense strand and an antisense strand, wherein the antisense strand comprises a region having a sequence that is substantially or fully complementary to an ASGR-1 messenger RNA (mRNA) sequence and/or ASGR-2 mRNA sequence. As used herein, an "ASGR-1 mRNA sequence" or "ASGR-2 mRNA sequence" refers to any messenger RNA sequence, including splice variants, encoding an ASGR-1 protein or ASGR-2 protein, including ASGR-1 or ASGR-2 protein variants or isoforms from any species (e.g. mouse, rat, non-human primate, human).

The sense strand of the double-stranded RNA molecule typically comprises a sequence that is sufficiently complementary to the sequence of the antisense strand such that the two strands hybridize under physiological conditions to form a duplex region. A "duplex region" refers to the region in two complementary or substantially complementary polynucleotides that form base pairs with one another, either by Watson-Crick base pairing or other hydrogen bonding interaction, to create a duplex between the two polynucleotides. The duplex region of the RNA molecule should be of sufficient length to allow the RNA molecule to enter the RNA interference pathway, e.g. by engaging the Dicer enzyme and/or the RISC complex. For instance, in some embodiments, the duplex region is about 15 to about 30 base pairs in length. Other lengths for the duplex region within this range are also suitable, such as about 15 to about 28 base pairs, about 15 to about 26 base pairs, about 15 to about 24 base pairs, about 15 to about 22 base pairs, about 17 to about 28 base pairs, about 17 to about 26 base pairs, about 17 to about 24 base pairs, about 17 to about 23 base pairs, about 17 to about 21 base pairs, about 19 to about 25 base pairs, about 19 to about 23 base pairs, or about 19 to about 21 base pairs. In one embodiment, the duplex region is about 17 to about 24 base pairs in length. In another embodiment, the duplex region is about 19 to about 21 base pairs in length.

For embodiments in which the sense strand and antisense strand are two separate molecules (e.g. RNAi agent is a siRNA), the sense strand and antisense strand need not be the same length as the length of the duplex region. For instance, one or both strands may be longer than the duplex region and have one or more unpaired nucleotides or mismatches flanking the duplex region. Thus, in some embodiments, the double-stranded RNA molecule comprises at least one nucleotide overhang. As used herein, a "nucleotide overhang" refers to the unpaired nucleotide or nucleotides that extend beyond the duplex region at the terminal ends of the strands. Nucleotide overhangs are typically created when the 3' end of one strand extends beyond the 5' end of the other strand or when the 5' end of one strand extends beyond the 3' end of the other strand. The length of a nucleotide overhang is generally between 1 and 6 nucleotides, 1 and 5 nucleotides, 1 and 4 nucleotides, 1 and 3 nucleotides, 2 and 6 nucleotides, 2 and 5 nucleotides, or 2 and 4 nucleotides. In some embodiments, the nucleotide overhang comprises 1, 2, 3, 4, 5, or 6 nucleotides. In one particular embodiment, the nucleotide overhang comprises 1 to 4 nucleotides. In certain embodiments, the nucleotide overhang comprises 2 nucleotides. The nucleotides in the overhang can be ribonucleotides, deoxyribonucleotides, or modified nucleotides as described herein.

The nucleotide overhang can be at the 5' end or 3' end of one or both strands. For example, in one embodiment, the double-stranded RNA molecule comprises a nucleotide overhang at the 5' end and the 3' end of the antisense strand. In another embodiment, the double-stranded RNA molecule comprises a nucleotide overhang at the 5' end and the 3' end of the sense strand. In some embodiments, the double-stranded RNA molecule comprises a nucleotide overhang at the 5' end of the sense strand and the 5' end of the antisense strand. In other embodiments, the double-stranded RNA molecule comprises a nucleotide overhang at the 3' end of the sense strand and the 3' end of the antisense strand.

The double-stranded RNA molecules may comprise a single nucleotide overhang at one end of the molecule and a blunt end at the other. A "blunt end" means that the sense strand and antisense strand are fully base-paired at the end of the molecule and there are no unpaired nucleotides that extend beyond the duplex region. In some embodiments, the double-stranded RNA molecule comprises a nucleotide overhang at the 3' end of the sense strand and a blunt end at the 5' end of the sense strand and 3' end of the antisense strand. In other embodiments, the double-stranded RNA molecule comprises a nucleotide overhang at the 3' end of the antisense strand and a blunt end at the 5' end of the antisense strand and the 3' end of the sense strand. In certain embodiments, the double-stranded RNA molecule comprises a blunt end at both ends of the double-stranded RNA molecule. In such embodiments, the sense strand and antisense strand have the same length and the duplex region is the same length as the sense and antisense strands (i.e. the molecule is double-stranded over its entire length).

The sense strand and antisense strand can each independently be about 15 to about 30 nucleotides in length, about 18 to about 28 nucleotides in length, about 19 to about 27 nucleotides in length, about 19 to about 25 nucleotides in length, about 19 to about 23 nucleotides in length, about 21 to about 25 nucleotides in length, or about 21 to about 23 nucleotides in length. In certain embodiments, the sense strand and antisense strand are each about 18, about 19, about 20, about 21, about 22, about 23, about 24, or about 25 nucleotides in length. In some embodiments, the sense strand and antisense strand have the same length but form a duplex region that is shorter than the strands such that the double-stranded RNA molecule has two nucleotide overhangs. For instance, in one embodiment, the double-stranded RNA molecule comprises (i) a sense strand and an antisense strand that are each 21 nucleotides in length, (ii) a duplex region that is 19 base pairs in length, and (iii) nucleotide overhangs of 2 unpaired nucleotides at both the 3' end of the sense strand and the 3' end of the antisense strand. In another embodiment, the double-stranded RNA molecule comprises (i) a sense strand and an antisense strand that are each 23 nucleotides in length, (ii) a duplex region that is 21 base pairs in length, and (iii) nucleotide overhangs of 2 unpaired nucleotides at both the 3' end of the sense strand and the 3' end of the antisense strand. In other embodiments, the sense strand and antisense strand have the same length and form a duplex region over their entire length such that there are no nucleotide overhangs on either end of the double-stranded molecule. In one such embodiment, the double-stranded RNA molecule is blunt ended and comprises (i) a sense strand and an antisense strand, each of which is 21 nucleotides in length, and (ii) a duplex region that is 21 base pairs in length. In another such embodiment, the double-stranded RNA molecule is blunt ended and comprises (i) a sense strand and an antisense strand, each of which is 23 nucleotides in length, and (ii) a duplex region that is 23 base pairs in length.

In other embodiments, the sense strand or the antisense strand is longer than the other strand and the two strands form a duplex region having a length equal to that of the shorter strand such that the double-stranded RNA molecule comprises at least one nucleotide overhang. For example, in one embodiment, the double-stranded RNA molecule comprises (i) a sense strand that is 19 nucleotides in length, (ii) an antisense strand that is 21 nucleotides in length, (iii) a duplex region of 19 base pairs in length, and (iv) a single nucleotide overhang of 2 unpaired nucleotides at the 3' end of the antisense strand. In another embodiment, the double-stranded RNA molecule comprises (i) a sense strand that is 21 nucleotides in length, (ii) an antisense strand that is 23 nucleotides in length, (iii) a duplex region of 21 base pairs in length, and (iv) a single nucleotide overhang of 2 unpaired nucleotides at the 3' end of the antisense strand.

Off-target toxicity is a constant concern in the development of pharmaceutical products. With interfering RNA agents, the potential exists for homology with certain endogenous polynucleotide sequences that could lead to unintended toxic effects in the patient receiving the interfering RNA. Accordingly, in some embodiments, the RNA molecule comprises a polynucleotide sequence that is also substantially non-homologous to any naturally occurring, normally functioning, and essential mammalian polynucleotide sequence, so that the RNA molecule does not adversely affect the function of any essential naturally occurring mammalian polynucleotide sequence, when used in the methods of this invention. Such naturally occurring functional mammalian polynucleotide sequences include mammalian sequences that encode desired proteins, as well as mammalian sequences that are non-coding, but that provide for essential regulatory sequences in a healthy mammal. Preferably, the RNA molecule useful in the methods of the invention must be sufficiently distinct in sequence from any mammalian polynucleotide sequence expressed in the target cells (e.g. liver cells) for which the function is intended to be undisturbed after any of the methods of this invention are performed. As described for determining the homology to the target sequence above, one of skill in the art may resort to the above-identified computer algorithms to define the essential lack of homology between the RNA molecule polynucleotide sequence and the normal mammalian sequences expressed in the target cells. For example, in a specific embodiment, the homology between the sequence of an RNAi agent and the selected normal sequence expressed in the target cells is less than the homologies of the formulae described above. In some embodiments, there is almost no homology at all between the sequence of an RNAi agent and any normal mammalian sequence.

The double-stranded RNA molecules used in the methods of the invention may comprise one or more modified nucleotides. A "modified nucleotide" refers to a nucleotide that has one or more chemical modifications to the nucleoside, nucleobase, pentose ring, or phosphate group. The double-stranded RNA molecules may comprise combinations of modified nucleotides, ribonucleotides, and deoxyribonucleotides. Incorporation of modified nucleotides into one or both strands of double-stranded RNA molecules can improve the in vivo stability of the RNA molecules, e.g., by reducing the molecules' susceptibility to nucleases and other degradation processes. The potency of double-stranded RNA molecules for reducing expression of the target gene can also be enhanced by incorporation of modified nucleotides.

In certain embodiments, the modified nucleotides have a modification of the ribose sugar. These sugar modifications can include modifications at the 2' and/or 5' position of the pentose ring. A 2'-modified nucleotide refers to a nucleotide having a pentose ring with a substituent at the 2' position other than H or OH. Such 2'-modifications include, but are not limited to, 2'-O-alkyl (e.g. O-$C_1$-$C_{10}$ or O-$C_1$-$C_{10}$ substituted alkyl), 2'-O-allyl (O-$CH_2CH=CH_2$), 2'-C-allyl, 2'-fluoro, 2'-O-methyl ($OCH_3$), 2'-O-methoxyethyl (O-$(CH_2)_2OCH_3$), 2'-$OCF_3$, 2'-O$(CH_2)_2SCH_3$, 2'-O-aminoalkyl, 2'-amino (e.g. $NH_2$), 2'-O-ethylamine, and 2'-azido. Modifications at the 5' position of the pentose ring include, but are not limited to, 5'-methyl (R or S); 5'-vinyl, and 5'-methoxy.

The double-stranded RNA molecules employed in the methods of the invention may also comprise one or more modified internucleotide linkages. As used herein, the term "modified internucleotide linkage" refers to an internucleotide linkage other than the natural 3' to 5' phosphodiester linkage. In some embodiments, the modified internucleotide linkage is a phosphorous-containing internucleotide linkage, such as a phosphotriester, aminoalkylphosphotriester, an alkylphosphonate (e.g. methylphosphonate, 3'-alkylene phosphonate), a phosphinate, a phosphoramidate (e.g. 3'-amino phosphoramidate and aminoalkylphosphoramidate), a phosphorothioate (P=S), a chiral phosphorothioate, a phosphorodithioate, a thionophosphoramidate, a thionoalkylphosphonate, a thionoalkylphosphotriester, and a boranophosphate.

In one embodiment, a modified internucleotide linkage is a 2' to 5' phosphodiester linkage. In other embodiments, the modified internucleotide linkage is a non-phosphorous-containing internucleotide linkage and thus can be referred to as a modified internucleoside linkage. Such non-phosphorous-containing linkages include, but are not limited to, morpholino linkages (formed in part from the sugar portion of a nucleoside); siloxane linkages (-O-Si(H)$_2$-O-); sulfide, sulfoxide and sulfone linkages; formacetyl and thioformacetyl linkages; alkene containing backbones; sulfamate backbones; methyl-enemethylimino (-CH$_2$-N(CH$_3$) -O-CH$_2$-) and methylenehydrazino linkages; sulfonate and sulfonamide linkages; amide linkages; and others having mixed N, O, S and CH$_2$ component parts. In one embodiment, the modified internucleoside linkage is a peptide-based linkage (e.g. aminoethylglycine) to create a peptide nucleic acid or PNA, such as those described in U.S. Patent Nos. 5,539,082; 5,714,331; and 5,719,262. Other suitable modified internucleotide and internucleoside linkages that may be employed in the double-stranded RNA molecules are described in U.S. Patent No. 6,693,187, U.S. Patent No. 9,181,551, U.S. Patent Publication No. 2016/0122761, and Deleavey and Damha, Chemistry and Biology, Vol. 19: 937-954, 2012, all of which are hereby incorporated by reference in their entireties.

## Interfering RNA delivery

The interfering RNA compounds can be administered by any method suitable for administration of nucleic acid agents, such as a DNA vaccine or gene therapy vectors. These methods include gene guns, bio injectors, and skin patches as well as needle-free methods such as the micro-particle DNA vaccine technology disclosed in U.S. Pat. No. 6,194,389, and the mammalian transdermal needle-free vaccination with powder-form vaccine as disclosed in U.S. Pat. No. 6,168,587. Additionally, intranasal delivery is possible, as described in, inter alia, Hamajima et al. (1998), Clin. Immunol. Immunopathol., 88(2), 205-10. Liposomes (e.g., as described in U.S. Pat. No. 6,472,375) and microencapsulation can also be used. Biodegradable targetable microparticle delivery systems can also be used (e.g., as described in U.S. Pat. No. 6,471,996).

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques. The materials can also be obtained commercially from, for example, Alza Corporation and Nova Pharmaceuticals, Inc. Liposomal suspensions can also be used as pharmaceutically acceptable carriers. These can be prepared according to methods known to those skilled in the art, for example, as described in U.S. Pat. No. 4,522,811.

The interfering RNA molecule may be conjugated to one or more carbohydrate moieties to optimize one or more properties of the interfering RNA molecule. In many cases, the carbohydrate moiety will be attached to a modified subunit of the interfering RNA molecule or at the 5' or 3' end of one of strands of the interfering RNA molecule. E.g., the ribose sugar of one or more ribonucleotide subunits of an interfering RNA molecule can be replaced with another moiety, e.g., a non-carbohydrate (preferably cyclic) carrier to which is attached a carbohydrate moiety. A cyclic carrier may be a carbocyclic ring system, i.e., all ring atoms are carbon atoms, or a heterocyclic ring system, i.e., one or more ring atoms may be a heteroatom, e.g., nitrogen, oxygen, sulfur. The cyclic carrier may be a monocyclic ring system, or may contain two or more rings, e.g. fused rings. The cyclic carrier may be a fully saturated ring system, or it may contain one or more double bonds.

The carbohydrate moiety may be attached to the polynucleotide via a carrier. The carriers include (i) at least one "backbone attachment point," preferably two "backbone attachment points" and (ii) at least one "tethering attachment point." A "backbone attachment point" as used herein refers to a functional group, e.g. a hydroxyl group, or generally, a bond available for, and that is suitable for incorporation of the carrier into the backbone, e.g., the phosphate, or modified phosphate, e.g., sulfur containing, backbone, of a ribonucleic acid. A "tethering attachment point" (TAP) in some embodiments refers to a constituent ring atom of the cyclic carrier, e.g., a carbon atom or a heteroatom (distinct from an atom which provides a backbone attachment point), that connects a selected moiety. The moiety can be, e.g., a carbohydrate, e.g. monosaccharide, disaccharide, trisaccharide, tetrasaccharide, oligosaccharide and polysaccharide. Optionally, the selected moiety is connected by an intervening tether to the cyclic carrier. Thus, the cyclic carrier will often include a functional group, e.g., an amino group, or generally, provide a bond, that is suitable for incorporation or tethering of another chemical entity, e.g., a ligand to the constituent ring.

In some embodiments the interfering RNA molecule of the invention is conjugated to a carbohydrate moiety via a carrier, wherein the carrier can be cyclic group or acyclic group; in specific embodiments, the cyclic group is selected from pyrrolidinyl, pyrazolinyl, pyrazolidinyl, imidazolinyl, imidazolidinyl, piperidinyl, piperazinyl, [1,3]dioxolane, oxazolidinyl, isoxazolidinyl, morpholinyl, thiazolidinyl, isothiazolidinyl, quinoxalinyl, pyridazinonyl, tetrahydrofuryl and decalin; preferably, the acyclic group is selected from serinol backbone or diethanolamine backbone.

Targeting the interfering RNA

Given that ASGR, ASGR-1 and/or ASGR-2 is expressed on the surface of liver cells (e.g. hepatocytes), in certain embodiments, it is desirable to deliver the interfering RNA molecules to those liver cells so that the interfering effect can be exerted specifically within liver cells. Accordingly, in certain embodiments, the interfering RNA molecules are specifically targeted to liver cells using various methodologies known in the art and described herein. For example, in certain embodiments, antigen binding proteins (e.g. antibodies) or other targeting moieties disclosed herein below can be used to specifically target the interfering RNA molecules to the hepatocytes using various different receptors expressed on the surface of hepatocytes. In certain embodiments, the interfering RNA molecules are targeted to liver cells using the surface expressed ASGR, ASGR-1 and/or ASGR-2. In these embodiments, it is envisioned that this can result in a self-regulating system that reduces the amount of RNAi agent delivered to the liver cells as expression of ASGR, ASGR-1, and/or ASGR-2 is reduced due to the effect of the targeted interfering RNA.

A wide variety of targeting moieties can be coupled to the oligonucleotides of the present invention. In some embodiments, the targeting moieties are coupled, e.g., covalently, either directly or indirectly via an intervening tether.

In some embodiments, a targeting moiety alters the distribution, targeting or lifetime of the molecule into which it is incorporated. In preferred embodiments a targeting moiety provides an enhanced affinity for a selected target, e.g., molecule, cell or cell type, compartment, receptor e.g., a cellular or organ compartment, tissue, organ or region of the body, as, e.g., compared to a species absent such a targeting moiety. Targeting moieties providing enhanced affinity for a selected target are also termed targeting moieties.

Some targeting moieties can have endosomolytic properties. The endosomolytic targeting moieties promote the lysis of the endosome and/or transport of the composition of the invention, or its components, from the endosome to the cytoplasm of the cell. The endosomolytic targeting moietymay be a polyanionic peptide or peptidomimetic which shows pH-dependent membrane activity and fusogenicity. In one embodiment, the endosomolytic targeting moiety assumes its active conformation at endosomal pH. The "active" conformation is that conformation in which the endosomolytic targeting moietypromotes lysis of the endosome and/or transport of the composition of the invention, or its components, from the endosome to the cytoplasm of the cell. Exemplary endosomolytic targeting moietiesinclude the GALA peptide (Subbarao et al., Biochemistry, 1987, 26: 2964-2972), the EALA peptide (Vogel et al., J. Am. Chem. Soc., 1996, 118: 1581-1586), and their derivatives (Turk et al., Biochem. Biophys. Acta, 2002, 1559: 56-68). In one embodiment, the endosomolytic component may contain a chemical group (e.g., an amino acid) which will undergo a change in charge or protonation in response to a change in pH. The endosomolytic component may be linear or branched.

In certain embodiments, targeting moieties can improve transport, hybridization, and specificity properties and may also improve nuclease resistance of the resultant natural or modified oligoribonucleotide, or a polymeric molecule comprising any combination of monomers described herein and/or natural or modified ribonucleotides.

In some embodiments, targeting moieties in general can include therapeutic modifiers, e.g., for enhancing uptake; diagnostic compounds or reporter groups e.g., for monitoring distribution; cross-linking agents; and nuclease-resistance conferring moieties. General examples include lipids, steroids, vitamins, sugars, proteins, peptides, polyamines, and peptide mimics.

Targeting moieties can include a naturally occurring substance, such as a protein (e.g., human serum albumin (I), low-density lipoprotein (LDL), high-density lipoprotein (HDL), or globulin); a carbohydrate (e.g., a dextran, pullulan, chitin, chitosan, inulin, cyclodextrin or hyaluronic acid); or a lipid. The targeting moiety may also be a recombinant or synthetic molecule, such as a synthetic polymer, e.g., a synthetic polyamino acid, an oligonucleotide (e.g. an aptamer). Examples of polyamino acids include polyamino acid is a polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an alpha helical peptide.

Targeting moieties can also include other targeting groups, e.g., a cell or tissue targeting agent, e.g., a lectin, glycoprotein, lipid or protein, e.g., an antibody, that binds to a specified cell type such as a kidney cell. A targeting group can be a thyrotropin, melanotropin, lectin, glycoprotein, surfactant protein A, Mucin carbohydrate, multivalent lactose, multivalent galactose, N-acetyl-galactosamine, N-acetyl-gulucosamine multivalent mannose, multivalent fucose, glycosylated polyaminoacids, multivalent galactose, transferrin, bisphosphonate, polyglutamate, polyaspartate, a lipid, cholesterol, a steroid, bile acid, folate, vitamin B12, biotin, an RGD peptide, an RGD peptide mimetic or an aptamer.

Other examples of targeting moieties include dyes, intercalating agents (e.g. acridines), cross-linkers (e.g. psoralene, mitomycin C), porphyrins (TPPC4, texaphyrin, Sapphyrin), polycyclic aromatic hydrocarbons (e.g., phenazine, dihydrophenazine), artificial endonucleases or a chelator (e.g. EDTA), lipophilic molecules, e.g, cholesterol, cholic acid, 206ligonucle acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]$_2$, polyamino, alkyl, substituted alkyl, radiolabeled markers, enzymes, haptens (e.g. biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP.

Targeting moieties can be proteins, e.g., glycoproteins, or peptides, e.g., molecules having a specific affinity for a co-moiety, or antigen binding proteins, such as antibodies; e.g., an antibody, that binds to a specified cell type such as a liver hepatocyte. Targeting moieties may also include hormones and hormone receptors. They can also include non-peptidic species, such as lipids, lectins, carbohydrates, vitamins, cofactors, multivalent lactose, multivalent galactose, N-acetyl-galactosamine, N-acetyl-gulucosamine multivalent mannose, multivalent fucose, or aptamers. The targeting moiety can be, for example, a lipopolysaccharide.

The targeting moiety can be a substance, e.g, a drug, which can increase the uptake of the interfering RNA molecule into the cell, for example, by disrupting the cell's cytoskeleton, e.g., by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin.

The targeting moiety can increase the uptake of the interfering RNA molecule into the cell by activating an inflammatory response, for example. Exemplary targeting moieties that would have such an effect include tumor necrosis factor alpha (TNFalpha), interleukin-1 beta, or gamma interferon.

In one embodiment, the targeting moiety is a lipid or lipid-based molecule. Such a lipid or lipid-based molecule preferably binds a serum protein, e.g., human serum albumin (I). A serum protein binding targeting moiety, in certain embodiments, allows for distribution of the conjugate to a target tissue, e.g., a non-kidney target tissue of the body. For example, the target tissue can be the liver, including hepatocytes or parenchymal cells of the liver. Other molecules that can bind serum proteins can also be used as targeting moieties. For example, naproxen or aspirin can be used. A lipid or lipid-based targeting moiety can (a) increase resistance to degradation of the conjugate, (b) increase targeting or transport into a target cell or cell membrane, and/or (c) can be used to adjust binding to a serum protein.

A lipid based targeting moiety can be used to modulate, e.g., control the binding of the conjugate to a target tissue. For example, a lipid or lipid-based targeting moiety that binds to a serum protein more strongly will be less likely to be targeted to the kidney and therefore less likely to be cleared from the body. A lipid or lipid-based targeting moiety that binds to a serum protein less strongly can be used to target the conjugate to the kidney, if so desired.

In one embodiment, the lipid based targeting moiety binds human serum albumin. In a specific embodiment, it binds human serum albumin with a sufficient affinity such that the conjugate will be preferably distributed to a non-kidney tissue. In certain embodiments, it is preferred that the affinity not be so strong that the human serum albumin targeting moiety binding cannot be reversed.

In another preferred embodiment, the lipid based targeting moiety binds human serum albumin weakly or not at all, such that the conjugate will be preferably distributed to the kidney. Other moieties that target to kidney cells can also be used in place of or in addition to the lipid-based targeting moiety.

In another embodiment, the targeting moiety is for example a vitamin, e.g., a vitamin, which is taken up by a target cell, e.g., a proliferating cell. Exemplary vitamins include vitamin A, E, and K. Other exemplary vitamins include B vitamins, e.g., folic acid, B12, riboflavin, biotin, pyridoxal or other vitamins or nutrients taken up by cells. Also included are low density lipoprotein (LDL) and high-density lipoprotein (HDL).

In another embodiment, the targeting moiety is a cell-permeation agent, preferably a helical cell-permeation agent. In some embodiments, the agent is amphipathic. An exemplary agent is a peptide such as tat or antennopedia. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

The targeting moiety can be a peptide or peptidomimetic. A peptidomimetic (also referred to herein as an oligopeptidomimetic) is a molecule capable of folding into a defined three-dimensional structure similar to a natural peptide. The peptide or peptidomimetic moiety can be about 5-50 amino acids long, e.g., about 5, 10, 15, 20, 25, 30, 35, 40, 45, or 50 amino acids long. A peptide or peptidomimetic can be, for example, a cell permeation peptide, cationic peptide, amphipathic peptide, or hydrophobic peptide (e.g., consisting primarily of Tyr, Trp or Phe). The peptide moiety can be a dendrimer peptide, constrained peptide or crosslinked peptide. In another alternative, the peptide moiety can include a hydrophobic membrane translocation sequence (MTS). An exemplary hydrophobic MTS-containing peptide is RFGF having the amino acid sequence AAVALLPAVLLALLAP. An RFGF analogue (e.g., amino acid sequence AALLPVLLAAP) containing a hydrophobic MTS can also be a targeting moiety. The peptide moiety can be a "delivery" peptide, which can carry large polar molecules including peptides, oligonucleotides, and protein across cell membranes. For example, sequences from the HIV Tat protein (GRKKRRQRRRPPQ) and the Drosophila Antennapedia protein (RQIKIWFQNRRMKWKK) have been found to be capable of functioning as delivery peptides. A peptide or peptidomimetic can be encoded by a random sequence of DNA, such as a peptide identified from a phage-display library, or one-bead-one-compound (OBOC) combinatorial library (Lam et al., Nature, 354:82-84, 1991). In some embodiments, the peptide or peptidomimetic tethered to an interfering RNA molecule via an incorporated monomer unit is a cell targeting peptide such as an arginine-glycine-aspartic acid (RGD)-peptide, or RGD mimic. A peptide moiety can range in length from about 5 amino acids to about 40 amino acids. The peptide moieties can have a structural modification, such as to increase stability or direct conformational properties. Any of the structural modifications described below can be utilized. An RGD peptide can facilitate targeting of an interfering RNA molecule to cells of a variety of other tissues, including the lung, kidney, spleen, or liver (Aoki et al., Cancer Gene Therapy 8:783-787, 2001). The RGD peptide can be linear or cyclic, and can be modified, e.g., glycosylated or methylated to facilitate targeting to specific tissues. For example, a glycosylated RGD peptide can deliver an interfering RNA molecule to a cell expressing $\alpha V\beta_3$ (Haubner et al., Jour. Nucl. Med., 42:326-336, 2001). Peptides that target markers enriched in proliferating cells can be used. E.g., RGD containing peptides and peptidomimetics can target cells, in particular cells that exhibit an integrin. Thus, one could use RGD peptides, cyclic peptides containing RGD, RGD peptides that include D-amino acids, as well as synthetic RGD mimics. In addition to RGD, one can use other moieties that target the integrin ligand.

A "cell permeation peptide" is capable of permeating a cell, e.g., a microbial cell, such as a bacterial or fungal cell, or a mammalian cell, such as a human cell. A microbial cell-permeating peptide can be, for example, an $\alpha$-helical linear peptide (e.g., LL-37 or Ceropin P1), a disulfide bond-containing peptide (e.g., $\alpha$-defensin, $\beta$-defensin or bactenecin), or a peptide containing only one or two dominating amino acids (e.g., PR-39 or indolicidin). A cell permeation peptide can also include a nuclear localization signal (NLS). For example, a cell permeation peptide can be a bipartite amphipathic peptide, such as MPG, which is derived from the fusion peptide domain of HIV-1 gp41 and the NLS of SV40 large T antigen (Simeoni et al., Nucl. Acids Res. 31:2717-2724, 2003).

In one embodiment, a targeting peptide can be an amphipathic $\alpha$-helical peptide. Exemplary amphipathic $\alpha$-helical peptides include, but are not limited to, cecropins, lycotoxins, paradaxins, buforin, CPF, bombinin-like peptide (BLP), cathelicidins, ceratotoxins, S. clava peptides, hagfish intestinal antimicrobial peptides (HFIAPs), magainines, brevinins-2, dermaseptins, melittins, pleurocidin, H.sub.2A peptides, Xenopus peptides, esculentinis-1, and caerins.

Peptide and peptidomimetic targeting moietiesinclude those having naturally occurring or modified peptides, e.g., D or L peptides; $\alpha$, $\beta$, or $\gamma$ peptides; N-methyl peptides; azapeptides; peptides having one or more amide, i.e., peptide, linkages replaced with one or more urea, thiourea, carbamate, or sulfonyl urea linkages; or cyclic peptides.

The targeting moiety can be any moiety that is capable of targeting a specific receptor. Examples are: folate, GalNAc, galactose, mannose, mannose-6P, clusters of sugars such as GalNAc cluster, mannose cluster, galactose cluster, or an apatamer. A cluster is a combination of two or more sugar units. The targeting moieties also include integrin receptor moieties, chemokine receptor moieties, transferrin, biotin, serotonin receptor moieties, PSMA, endothelin, GCPII, somatostatin, LDL and HDL moieties. The targeting moieties can also be based on nucleic acid, e.g., an aptamer. The aptamer can be unmodified or have any combination of modifications disclosed herein.

Other exemplary endosomal release agents include imidazoles, poly or oligoimidazoles, PEIs, peptides, fusogenic peptides, polycaboxylates, polyacations, masked oligo or poly cations or anions, acetals, polyacetals, ketals/polyketyals, orthoesters, polymers with masked or unmasked cationic or anionic charges, dendrimers with masked or unmasked cationic or anionic charges.

Pharmacokinetic ("PK") modulators include lipophiles, bile acids, steroids, phospholipid analogues, peptides, protein binding agents, PEG, vitamins etc. Examplary PK modulators include, but are not limited to, cholesterol, fatty acids, cholic acid, lithocholic acid, dialkylglycerides, diacylglyceride, phospholipids, sphingolipids, naproxen, ibuprofen, vitamin E, biotin etc. Oligonucleotides that comprise a number of phosphorothioate linkages are also known to bind to serum protein, thus short oligonucleotides, e.g. oligonucleotides of about 5 bases, 10 bases, 15 bases or 20 bases, comprising multiple of phosphorothioate linkages in the backbaone are also amenable to the present invention as targeting moieties(e.g. as PK modulating moieties). In addition, aptamers that bind serum components (e.g. serum proteins) are also amenable to the present invention as PK modulating moieties.

When two or more targeting moieties are present, the targeting moieties can all have same properties, all have different properties or some targeting moieties have the same properties while others have different properties. For example, a targeting moiety can have targeting properties, have endosomolytic activity and/or have PK modulating properties. In certain embodiments, all the have different properties.

In some embodiments, a targeting moiety can be conjugated to nucleobases, sugar moieties, or internucleosidic linkages of nucleic acid molecules. Conjugation to purine nucleobases or derivatives thereof can occur at any position including, endocyclic and exocyclic atoms. In some embodiments, the 2-, 6-, 7-, or 8-positions of a purine nucleobase are attached to a conjugate moiety. Conjugation to pyrimidine nucleobases or derivatives thereof can also occur at any position. In some embodiments, the 2-, 5-, and 6-positions of a pyrimidine nucleobase can be substituted with a conjugate moiety. Conjugation to sugar moieties of nucleosides can occur at any carbon atom. Example carbon atoms of a sugar moiety that can be attached to a conjugate moiety include the 2', 3', and 5' carbon atoms. The 1' position can also be attached to a conjugate moiety, such as in an abasic residue. Internucleosidic linkages can also bear conjugate moieties.

For phosphorus-containing linkages (e.g., phosphodiester, phosphorothioate, phosphorodithiotate, phosphoroamidate, and the like), the conjugate moiety can be attached directly to the phosphorus atom or to an O, N, or S atom bound to the phosphorus atom. For amine- or amide-containing internucleosidic linkages (e.g., PNA), the conjugate moiety can be attached to the nitrogen atom of the amine or amide or to an adjacent carbon atom.

It is envisioned that any suitable targeting moiety in the field of RNA interference may be used, although the targeting moiety is typically a carbohydrate e.g. monosaccharide (such as GalNAc), disaccharide, trisaccharide, tetrasaccharide, polysaccharide. Linkers that conjugate the targeting moiety to the nucleic acid include those discussed herein. For example, the targeting moiety can be one or more GalNAc derivatives attached through a bivalent or trivalent branched linker.

In certain embodiments, cleavable linking groups are utilized. A cleavable linking group is one which is sufficiently stable outside the cell, but which upon entry into a target cell is cleaved to release the two parts the linker is holding together. In one embodiment, the cleavable linking group is cleaved at least 10 times or more, and in some embodiments, at least 100 times faster in the target cell or under a first reference condition (which can, e.g., be selected to mimic or represent intracellular conditions) than in the blood of a subject, or under a second reference condition (which can, e.g., be selected to mimic or represent conditions found in the blood or serum).

Cleavable linking groups are susceptible to cleavage agents, e.g., pH, redox potential or the presence of degradative molecules. Generally, cleavage agents are more prevalent or found at higher levels or activities inside cells than in serum or blood. Examples of such degradative agents include: redox agents which are selected for particular substrates or which have no substrate specificity, including, e.g., oxidative or reductive enzymes or reductive agents such as mercaptans, present in cells, that can degrade a redox cleavable linking group by reduction; esterases; endosomes or agents that can create an acidic environment, e.g., those that result in a pH of five or lower; enzymes that can hydrolyze or degrade an acid cleavable linking group by acting as a general acid, peptidases (which can be substrate specific), and phosphatases.

A cleavable linkage group, such as a disulfide bond can be susceptible to pH. The pH of human serum is 7.4, while the average intracellular pH is slightly lower, ranging from about 7.1-7.3. Endosomes have a more acidic pH, in the range of 5.5-6.0, and lysosomes have an even more acidic pH at around 5.0. Some linkers will have a cleavable linking group that is cleaved at a preferred pH, thereby releasing the cationic lipid from the moiety inside the cell, or into the desired compartment of the cell.

A linker can include a cleavable linking group that is cleavable by a particular enzyme. The type of cleavable linking group incorporated into a linker can depend on the cell to be targeted. For example, liver targeting targeting moietiescan be linked to the cationic lipids through a linker that includes an ester group. Liver cells are rich in esterases, and therefore the linker will be cleaved more efficiently in liver cells than in cell types that are not esterase-rich. Other cell-types rich in esterases include cells of the lung, renal cortex, and testis. Linkers that contain peptide bonds can be used when targeting cell types rich in peptidases, such as liver cells and synoviocytes.

In general, the suitability of a candidate cleavable linking group can be evaluated by testing the ability of a degradative agent (or condition) to cleave the candidate linking group. It will also be desirable to also test the candidate cleavable linking group for the ability to resist cleavage in the blood or when in contact with other non-target tissue. Thus one can determine the relative susceptibility to cleavage between a first and a second condition, where the first is selected to be indicative of cleavage in a target cell and the second is selected to be indicative of cleavage in other tissues or biological fluids, e.g., blood or serum. The evaluations can be carried out in cell free systems, in cells, in cell culture, in organ or tissue culture, or in whole animals. It may be useful to make initial evaluations in cell-free or culture conditions and to confirm by further evaluations in whole animals. In some embodiments, useful candidate compounds are cleaved at least 2, 4, 10 or 100 times faster in the cell (or under in vitro conditions selected to mimic intracellular conditions) as compared to blood or serum (or under in vitro conditions selected to mimic extracellular conditions).

In some embodiments, redox cleavable linking groups are utilized. Redox cleavable linking groups are cleaved upon reduction or oxidation. An example of reductively cleavable linking group is a disulphide linking group (--S-S--). To determine if a candidate cleavable linking group is a suitable "reductively cleavable linking group," or for example is suitable for use with a particular interfering RNA molecule and particular targeting agent one can look to methods described herein. For example, a candidate can be evaluated by incubation with dithiothreitol (DTT), or other reducing agent using reagents know in the art, which mimic the rate of cleavage which would be observed in a cell, e.g., a target cell. The candidates can also be evaluated under conditions which are selected to mimic blood or serum conditions. In a specific embodiment, candidate compounds are cleaved by at most 10% in the blood. In some embodiments, useful candidate compounds are degraded at least 2, 4, 10 or 100 times faster in the cell (or under in vitro conditions selected to mimic intracellular conditions) as compared to blood (or under in vitro conditions selected to mimic extracellular conditions). The rate of cleavage of candidate compounds can be determined using standard enzyme kinetics assays under conditions chosen to mimic intracellular media and compared to conditions chosen to mimic extracellular media.

In yet some embodiments, phosphate-based cleavable linking groups are cleaved by agents that degrade or hydrolyze the phosphate group. An example of an agent that cleaves phosphate groups in cells are enzymes such as phosphatases in cells. Examples of phosphate-based linking groups are -O-P(O)(Ork)-O--, --O-P(S)(Ork)-O--, --O-P(S)(SRk)-O--, -S-P(O) (Ork)-O--, --O-P(O)(Ork)-S--, --S-P(O)(Ork)-S--, --O-P(S)(Ork)-S--, --S-P(S)(Ork)-O--, --O-P(O)(Rk)-O--,O-P(S)(Rk)-O--, --S-P(O)(Rk)-O--, --S-P(S)(Rk)-O--, --S-P(O)(Rk)-S--, --O-P(S)(Rk)-S--. Specific embodiments include -O-P(O)(OH)-O--, --O-P(S)(OH)-O--, --O-P(S)(SH)-O--, --S-P(O)(OH)-O--, --O-P(O)(OH)-S--, --S-P(O)(OH)-S--, --O-P(S)(OH)-S--, --S-P(S)(OH)-O--, --OP(O)(H)-O--, --O-P(S)(H)-O--, --S-P(O)(H)-O--, --S-P(S)(H)-O--, --S-P(O)(H)-S--, --OP(S)(H)-S--. Another specific embodiment is -O-P(O)(OH)-O--. These candidates can be evaluated using methods analogous to those described above.

In some embodiments, acid cleavable linking groups, which are linking groups that are cleaved under acidic conditions, are envisioned. In some embodiments acid cleavable linking groups are cleaved in an acidic environment with a pH of about 6.5 or lower (e.g., about 6.0, 5.5, 5.0, or lower), or by agents such as enzymes that can act as a general acid. In a cell, specific low pH organelles, such as endosomes and lysosomes can provide a cleaving environment for acid cleavable linking groups. Examples of acid cleavable linking groups include but are not limited to hydrazones, esters, and esters of amino acids. Acid cleavable groups can have the general formula - C==NN--, C(O)O, or -OC(O). A specific embodiment is when the carbon attached to the oxygen of the ester (the alkoxy group) is an aryl group, substituted alkyl group, or tertiary alkyl group such as dimethyl pentyl or t-butyl. These candidates can be evaluated using methods analogous to those described above.

In some embodiments, ester-based cleavable linking groups, which are cleaved by enzymes such as esterases and amidases in cells, are envisioned. Examples of ester-based cleavable linking groups include but are not limited to esters of alkylene, alkenylene and alkynylene groups. Ester cleavable linking groups have the general formula -C(O)O--, or -OC(O)--. These candidates can be evaluated using methods analogous to those described above.

In yet further embodiments, peptide-based cleavable linking groups, which are cleaved by enzymes such as peptidases and proteases in cells, are envisioned. Peptide-based cleavable linking groups are peptide bonds formed between amino acids to yield oligopeptides (e.g., dipeptides, tripeptides etc.) and polypeptides. Peptide-based cleavable groups do not include the amide group (--C(O)NH--). The amide group can be formed between any alkylene, alkenylene or alkynelene. A peptide bond is a special type of amide bond formed between amino acids to yield peptides and proteins. The peptide based cleavage group is generally limited to the peptide bond (i.e., the amide bond) formed between amino acids yielding peptides and proteins and does not include the entire amide functional group. Peptide-based cleavable linking groups have the general formula -NHCHR$^A$C(O)NHCHR$^B$C(O)--, where R$^A$ and R$^B$ are the R groups of the two adjacent amino acids. These candidates can be evaluated using methods analogous to those described above. As used herein, "carbohydrate" refers to a compound which is either a carbohydrate per se made up of one or more monosaccharide units having at least 6 carbon atoms (which may be linear, branched or cyclic) with an oxygen, nitrogen or sulfur atom bonded to each carbon atom; or a compound having as a part thereof a carbohydrate moiety made up of one or more monosaccharide units each having at least six carbon atoms (which may be linear, branched or cyclic), with an oxygen, nitrogen or sulfur atom bonded to each carbon atom. Representative carbohydrates include the sugars (mono-, di-, tri- and oligosaccharides containing from about 4-9 monosaccharide units), and polysaccharides such as starches, glycogen, cellulose and polysaccharide gums.

### Synthesis of interfering RNA

The interfering RNA molecules that can be employed in the methods of the present invention can readily be made using techniques known in the art, for example, using conventional RNA solid phase synthesis. See, for example, U.S. Patent No. 8,877,917. The polynucleotides of the double-stranded RNA molecules can be assembled on a suitable nucleic acid synthesizer utilizing standard nucleotide or nucleoside precursors (e.g. phosphoramidites). Automated nucleic acid synthesizers are sold commercially by several vendors, including DNA/RNA synthesizers from Applied Biosystems (Foster City, CA), MerMade synthesizers from BioAutomation (Irving, TX), and OligoPilot synthesizers from GE Healthcare Life Sciences (Pittsburgh, PA).

The 2' silyl protecting group can be used in conjunction with acid labile dimethoxytrityl (DMT) at the 5' position of ribonucleosides to synthesize oligonucleotides via phosphoramidite chemistry. Final deprotection conditions are known not to significantly degrade RNA products. All syntheses can be conducted in any automated or manual synthesizer on large, medium, or small scale. The syntheses may also be carried out in multiple well plates or glass slides.

The 2'-O-silyl group can be removed via exposure to fluoride ions, which can include any source of fluoride ion, e.g., those salts containing fluoride ion paired with inorganic counterions e.g., cesium fluoride and potassium fluoride or those salts containing fluoride ion paired with an organic counterion, e.g., a tetraalkylammonium fluoride. A crown ether catalyst can be utilized in combination with the inorganic fluoride in the deprotection reaction. Preferred fluoride ion source are tetrabutylammonium fluoride or aminehydrofluorides (e.g., combining aqueous HF with triethylamine in a dipolar aprotic solvent, e.g., dimethylformamide).

The choice of protecting groups for use on the phosphite triesters and phosphotriesters can alter the stability of the triesters towards fluoride. Methyl protection of the phosphotriester or phosphitetriester can stabilize the linkage against fluoride ions and improve process yields.

Since ribonucleosides have a reactive 2' hydroxyl substituent, it can be desirable to protect the reactive 2' position in RNA with a protecting group that is orthogonal to a 5'-O-dimethoxytrityl protecting group, e.g., one stable to treatment with acid. Silyl protecting groups meet this criterion and can be readily removed in a final fluoride deprotection step that can result in minimal RNA degradation.

Tetrazole catalysts can be used in the standard phosphoramidite coupling reaction. Preferred catalysts include e.g., tetrazole, S-ethyl-tetrazole, p-nitrophenyltetrazole.

See also, for example, Trufert et al., Tetrahedron, 52:3005, 1996; and Manoharan, "Oligonucleotide Conjugates in Antisense Technology," in Antisense Drug Technology, ed. S. T. Crooke, Marcel Dekker, Inc., 2001. The protected monomer compounds can be separated from a reaction mixture and further purified by a method such as column chromatography, high pressure liquid chromatography, or recrystallization. As can be appreciated by the skilled artisan, further methods of synthesizing the compounds of the formulae herein will be evident to those of ordinary skill in the art. Additionally, the various synthetic steps may be performed in an alternate sequence or order to give the desired compounds. Other synthetic chemistry transformations, protecting groups (e.g., for hydroxyl, amino, etc. present on the bases) and protecting group methodologies (protection and deprotection) useful in synthesizing the compounds described herein are known in the art and include, for example, those such as described in R. Larock, Comprehensive Organic Transformations, VCH Publishers (1989); T. W. Greene and P. G. M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

Methods of Treatment

In further embodiments of the present invention, a method of treating a human subject, comprising administering a therapeutic dosage of the antigen binding proteins or antibodies or interfering RNA (e.g., siRNA or shRNA) of the present invention is provided. In one embodiment, the antigen binding proteins are monoclonal antibodies. In one embodiment, the antigen binding proteins are human antibodies. In another embodiment, the antigen binding proteins or antibodies are humanized antibodies. In another embodiment, interfering RNA (e.g., siRNA or shRNA) is administered. As used herein the term "subject" refers to a mammal, including humans, and can be used interchangeably with the term "patient".

Given the results of the Icelandic study presented in the examples below, there need not be any particular further manipulation downstream in a host receiving a therapy involving administering the ASGR inhibitor (e.g., antigen binding proteins or antibodies or RNAi) to the host. That is, in some embodiments, the antibody (or RNAi) need simply be one or more of the antibodies (or RNAi) described herein, which binds to (and inhibits) ASGR (such as ASGR1), and be administered in an amount, and at a frequency sufficient to reduce the risk of cardiovascular disease, myocardial infarction, or other disorders provided herein. In some embodiments, the antibody (or RNAi) is administered in an amount sufficient to result in a lowering of non-HDL cholesterol. In some embodiments, the antibody (or RNAi) is administered in an amount sufficient to result in lowering LDL cholesterol. While not intended to be limiting unless expressed otherwise, below is a description of various embodiments through which ASGR can have an impact on various disorders, and thus, how the various antibodies (or RNAi) provided herein (which can inhibit (e.g., reduce) ASGR function) can have an impact on the various disorders provided herein.

In some embodiments, the ASGR inhibitor (e.g., antigen binding proteins or antibodies or RNAi) operates through ASGR's role in platelet clearance. Inhibiting (e.g., reducing) the receptor results in a reduction in clearance of old platelets. Such older platelets do not coagulate as well as new platelets and as a result, the blood is thinner. As a result, plaques can lessen and there can be a positive impact (e.g., stroke is lessened) for the subject.

In some embodiments, the ASGR inhibitor (e.g., antigen binding proteins or antibodies or RNAi) binds to ASGR to alter inflammation. For example, reducing the ASGR-1 receptor results in a modification of the immune response. Normally, there can be an increase in proinflammatory cytokines. These proinflammatory cytokines are circulating in the native state (one where the ASGR1 receptor is not reduced). However, ALP (alkaline phosphatase) can have an antiinflammatory role thereby reducing inflammation and coagulopathy systemically. In some embodiments, the mechanism of action involves reducing ASGR1 which increases ALP and therefore reduces inflammation.

In some embodiments, and without intending to be limited by theory (unless expressed otherwise), the ASGR inhibitor (e.g., antigen binding proteins or antibodies or RNAi) can reduce an activity due to ASGR interacting with one or more other molecules, either directly or indirectly. For example, various embodiments for various proteins are provided herein in Examples 18 and 19. As noted above, this selection of proteins can also be useful for determining the effectiveness of the ASGR inhibitor (e.g., antigen binding proteins or antibodies or RNAi) (and/or the amount of the antibody and/or identification of a subject who can respond to the therapy (or RNAi)) by monitoring one or more of these proteins as a Cardiovascular Disease marker. Thus, these markers are useful as markers and, without intending to be limited by theory, in some embodiments, one or more of the proteins disclosed below is the protein through which (directly or indirectly) ASGR1 modulation achieves its benefit for one or more of the disorders provided herein, including cardiovascular disease.

In addition to the marker proteins described in Examples 18 and 19 herein (which also allow for various mechanisms of action and monitoring the effectiveness of various ASGR inhibitors (e.g., antigen binding proteins or antibodies or RNAi) and dosage regimes), the following proteins of interest are those that interact with ASGR, and ASGR-1 in particular, directly by binding to them. Thus, these are additional interactions that can be inhibited (e.g., reduced) for various embodiments provided herein, by various ASGR inhibitors (e.g., antigen binding proteins or antibodies or RNAi). While not intending to be limited by theory (unless explicitly stated otherwise), ASGR-1's binding to one or more of the following proteins can be inhibited (e.g., reduced) by using an ASGR-1 inhibitor (e.g., antigen binding protein or antibody or RNAi) provided herein that inhibits (e.g., reduces) the noted binding. While in some embodiments, the protein interactions are contemplated as resulting mechanisms of action that occur downstream from when ASGR levels are effectively reduced by an ASGR inhibitor (e.g., antigen binding protein or antibody binding or via RNAi), the following list is a list of proteins that directly bind to ASGR1, and thus whose direct binding to ASGR-1 can be inhibited (e.g., reduced) by one or more of the antigen binding proteins or antibodies provided herein (or RNAi). In some embodiments, the ASGR-1 inhbiitor (e.g., antigen bindng protein or antibody or RNAi) inhibits (e.g., reduces) ASGR-1's binding to one or more of: Alpha-2-HS-glycoprotein (aka Fetuin A) (see Tozawa et al, J Biol Chem (2001) 276:12624-12628); Asialoglycoprotein receptor 1 (see Stockert et al (1977) Science 197:667-668), Orosomucoid (aka alpha-1-acid glycoprotein) (see Tozawa et al, J Biol Chem (2001) 276:12624-12628), Alkaline phosphatase, (see Hardonk MJ, Scholtens HB. Histochemistry. 1980;69(3):289-97 and Scholtens HB, Meijer DK, Hardonk MJ. Liver. 1982 Mar;2(1):14-21), LDL and chylomicrons (Windler et al Biochem J (1991) 276:79-87), Fibronectin (see Rotundo et al Hepatology (1998) 28:475-485), and IgA (see Stockert et al PNAS (1982) 79:6229-6231). In some embodiments, the ASGR inhibitor (e.g., antigen binding protein or antibody or RNAi) antibody binds to ASGR and inhibits (e.g., reduces) ASGR's interaction with a molecule that has a terminal gal or galNAc, including, but not limited to protein ligands, synthetic polysaccharides, solid substrates, etc. In some embodiments, the ASGR inhibitor (e.g., antigen binding proteins or antibodies or RNAi) inhibits (e.g., reduces) ASGR1's ability to bind to an asialylated molecule. In some embodiments, the invention provides a method of treating or preventing a cardiovascular disease comprising administering to a patient in need thereof a therapeutically effective dose of an ASGR inhibitor as described herein. In some embodiments, the cardiovascular disease is coronary artery disease or myocardial infarction. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described hereinn. In some embodiments, the ASGR, ASGR-1 and/or ASGR-2 inhibitor is an interfering RNA (e.g., siRNA or shRNA) as described herein. In some embodiments, the relative risk reduction of a cardiovascular event is at least about 5%, at least about 10%, at least about 15%, at least about 20%, at least about 25%, at least about 30%, at least about 35%, at least about 40%, at least about 45%, at least about 50%, at least about 55%, at least about 60% in the patient. Some non-limiting examples of cardiovascular disease include atherosclerotic diseases, such as, for example, coronary heart disease, coronary artery disease, peripheral arterial disease, stroke (ischaemic and hemorrhagic), angina pectoris, cerebrovascular disease, acute coronary syndrome, and myocardial infarction. In some embodiments, the ASGR, ASGR-1 and/or ASGR-2 inhibitors of the present invention are useful in reducing the risk of: nonfatal heart attacks, fatal and non-fatal strokes, certain types of heart surgery, hospitalization for heart failure, chest pain in patients with heart disease, and/or cardiovascular events because of established heart disease such as prior heart attack, prior heart surgery, and/or chest pain with evidence of clogged arteries. In some embodiments, the ASGR, ASGR-1 and/or ASGR-2 inhibitors of the present invention and methods can be used to reduce the risk of recurrent cardiovascular events.

In some embodiments, the invention provides a method of decreasing the risk of acquiring coronary artery disease or having an MI comprising administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described hereinn. In some embodiments, the ASGR, ASGR-1 and/or ASGR-2 inhibitor is an interfering RNA (e.g., siRNA or shRNA) as described herein. In some embodiments, the relative risk reduction of coronary artery disease or MI is at least about 5%, at least about 10%, at least about 15%, at least about 20%, at least about 25%, at least about 30%, at least about 35%, at least about 40%, at least about 45%, at least about 50%, at least about 55%, at least about 60% in the patient.

In some embodiments, the invention provides a method of reducing blood LDL cholesterol levels in a patient comprising administering to a patient in need thereof a therapeutically effective dose of of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described hereinn. In some embodiments, the ASGR, ASGR-1 and/or ASGR-2 inhibitor is an interfering RNA (e.g., siRNA or shRNA) as described herein. In some embodiments, the blood LDL cholesterol level in the patient is reduced by at least about 15%, as compared to a predose level of blood LDL cholesterol in the patient. In some embodiments of this aspect of the invention, the blood LDL cholesterol level of said patient is lowered by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose level of blood LDL cholesterol in the patient.

In some embodiments, the invention provides a method of reducing non-HDL cholesterol levels in a patient comprising administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described hereinn. In some embodiments, the ASGR, ASGR-1 and/or ASGR-2 inhibitor is an interfering RNA (e.g., siRNA or shRNA) as described herein. In some embodiments, the non-HDL cholesterol level in the patient is reduced by at least about 5%, as compared to a predose level of non-HDL cholesterol in the patient. In some embodiments of this aspect of the invention, the non-HDL cholesterol level of said patient is lowered by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose level of non-HDL cholesterol in the patient.

In some embodiments, the invention provides a method of increasing ALP levels in a patient comprising administering to a patient in need thereof a therapeutically effective dose of of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described hereinn. In some embodiments, the ASGR, ASGR-1 and/or ASGR-2 inhibitor is an interfering RNA (e.g., siRNA or shRNA) as described herein. In some embodiments, the ALP level in the patient is increased by at least about 30%, as compared to a predose level of ALP in the patient. In some embodiments of this aspect of the invention, the ALP level of said patient is increased by at least about at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose ALP level in the patient. In some embodiments, ALP levels are increased at least about 1.25x, 1.5x, 2x, 2.5x, 3x, 3.5x, 4x, 4.5x, and 5x over pretreatment.

In some embodiments, the invention provides a method of antagonizing ASGR, ASGR-1 and/or ASGR-2 in a patient comprising administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described hereinn. In some embodiments, the ASGR, ASGR-1 and/or ASGR-2 inhibitor is an interfering RNA (e.g., siRNA or shRNA) as described herein.

In some embodiments, a method of treating or preventing a cardiovascular disease is provided and comprises administering to a patient in need thereof a therapeutically effective dose of an ASGR inhibitor as described herein. In some embodiments, the ASGR inhibitor is an inhibitor of ASGR-1. In some embodiments, the ASGR inhibitor is an inhibitor of ASGR-2. In some embodiments, the ASGR inhibitor is an inhibitor of ASGR-1 and ASGR-2. In some embodiments, the ASGR, ASGR-1 and/or ASGR-2 inhibitor is one or more of the antigen binding proteins described herein. In some embodiments, the ASGR, ASGR-1 and/or ASGR-2 inhibitor is an interfering RNA (e.g., siRNA or shRNA) as described herein. In some embodiments, the relative risk reduction of a cardiovascular event is at least about 5%, at least about 10%, at least about 15%, at least about 20%, at least about 25%, at least about 30%, at least about 35%, at least about 40%, at least about 45%, at least about 50%, at least about 55%, at least about 60% in the patient.

The term "treatment" encompasses alleviation of at least one symptom or other embodiment of a disorder, or reduction of disease severity, and the like. An antigen binding protein, in particular a human antibody according to the present invention, need not effect a complete cure, or eradicate every symptom or manifestation of a disease, to constitute a viable therapeutic agent. As is recognized in the pertinent field, drugs employed as therapeutic agents may reduce the severity of a given disease state, but need not abolish every manifestation of the disease to be regarded as useful therapeutic agents. Simply reducing the impact of a disease (for example, by reducing the number or severity of its symptoms, or by increasing the effectiveness of another treatment, or by producing another beneficial effect), or reducing the likelihood that the disease will occur or worsen in a subject, is sufficient. One embodiment of the invention is directed to a method comprising administering to a patient an antigen binding protein or interfering RNA in an amount and for a time sufficient to induce a sustained improvement over baseline of an indicator that reflects the severity of the particular disorder.

The term "prevention" encompasses prevention of at least one symptom or other embodiment of a disorder, and the like. A prophylactically administered treatment incorporating an antigen binding protein, in particular a human antibody according to the present invention, need not be completely effective in preventing the onset of a condition in order to constitute a viable prophylactic agent. Simply reducing the likelihood that the disease will occur or worsen in a subject, is sufficient.

The term "non-HDL cholesterol" encompasses all cholesterol-containing proatherogenic lipoproteins, including LDL cholesterol, very-low-density lipoprotein, intermediate-density lipoprotein, lipoprotein(a), and chylomicron. Non-HDL cholesterol levels are calculated by subtracting HDL cholesterol levels from total cholesterol levels.

As is understood in the pertinent field, pharmaceutical compositions comprising the antigen binding proteins and/or interfering RNA are administered to a subject in a manner appropriate to the indication and the composition. In one embodiment, pharmaceutical compositions comprise the human antibodies of the present invention. In another embodiment, pharmaceutical compositions comprise interfering RNA. Pharmaceutical compositions may be administered by any suitable technique, including but not limited to parenterally, topically, or by inhalation. If injected, the pharmaceutical composition can be administered, for example, via intra-articular, intravenous, intramuscular, intralesional, intraperitoneal or subcutaneous routes, by bolus injection, or continuous infusion. Delivery by inhalation includes, for example, nasal or oral inhalation, use of a nebulizer, inhalation of the antigen binding protein in aerosol form, and the like. Other alternatives include oral preparations including pills, syrups, or lozenges.

Advantageously, the antigen binding proteins or interfering RNA are administered in the form of a composition comprising one or more additional components such as a physiologically acceptable carrier, excipient or diluent. Optionally, the composition additionally comprises one or more physiologically active agents. In various particular embodiments, the composition comprises one, two, three, four, five, or six physiologically active agents in addition to one or more antigen binding proteins (e.g, human antibodies) or interfering RNA.

Kits for use by medical practitioners are provided including one or more antigen binding proteins or interfering RNA and a label or other instructions for use in treating any of the conditions discussed herein. In one embodiment, the kit includes a sterile preparation of one or more human antibodies, or one or more interfering RNA which may be in the form of a composition as disclosed herein, and may be in one or more vials.

Dosages and the frequency of administration may vary according to such factors as the route of administration, the particular antigen binding proteins or interfering RNA employed, the nature and severity of the disease to be treated, whether the condition is acute or chronic, and the size and general condition of the subject. Appropriate dosages can be determined by procedures known in the pertinent art, e.g. in clinical trials that may involve dose escalation studies.

An antigen binding protein, e.g., monoclonal antibodies, or interfering RNA may be administered, for example, once or more than once, e.g., at regular intervals over a period of time. In particular embodiments, an antigen binding protein or interfering RNA is administered over a period of at least once a month or more, e.g., for one, two, or three months or even indefinitely. For treating chronic conditions, long-term treatment is generally most effective. However, for treating acute conditions, administration for shorter periods, e.g. from one to six weeks, may be sufficient. In general, the antigen binding protein or interfering RNA is administered until the patient manifests a medically relevant degree of improvement over baseline for the chosen indicator or indicators.

One example of therapeutic regimens provided herein comprise subcutaneous injection of an antigen binding protein or interfering RNA once a week, or once every two weeks, or once every month, once every other month, once every three months, once every six months or longer, at an appropriate dosage, to treat a condition in which it is desired to target cells expressing ASGR, ASGR-1 and/or ASGR-2. Weekly or monthly administration of antigen binding protein could be continued until a desired result is achieved, e.g., the subject's symptoms subside. Treatment may resume as needed, or, alternatively, maintenance doses may be administered.

In some embodiments, one or more of the markers in Tables 18.1, 18.2, 19.3, and 19.4 can be used to determine whether or not the amount of ASGR inhibitor (e.g., antigen binding protein and/or antibody and/or RNAi) administered is sufficient for its intended therapeutic application. In some embodiments, when one or more of the alterations in protein level, for the proteins outlined in one or more of Tables 18.1, 18.2, 19.3, and 19.4 changes in response to administering the antigen binding protein, antibody and/or RNAi, the antigen binding protein , antibody and/or RNAi is having an effect in the host. In some embodiments, the amount is sufficient when it alters the level of non-HDL cholesterol to a desired amount or reduces it by a desired amount. In some embodiments, the markers used can be one or more of those in one or more of Tiers 1, 2, 3, 4, and 5 of Table 19.4. In some embodiments, the markers used can be one or more of those in one or more of Tiers 1 and 5 of Table 19.4.

Combination therapies

Particular embodiments of methods and compositions of the invention involve the use of at least one antigen binding protein and/or interfering RNA and one or more other therapeutics useful for treating or preventing cardiovascular disease, for example. In one embodiment, antigen binding proteins and/or interfering RNA are administered alone or in combination with other agents useful for treating the condition with which the patient is afflicted. Examples of such agents include both proteinaceous and non-proteinaceous drugs. When multiple therapeutics are co-administered, dosages may be adjusted accordingly, as is recognized in the pertinent art. "Co-administration" and combination therapy are not limited to simultaneous administration, but also include treatment regimens in which an antigen binding protein is administered at least once during a course of treatment that involves administering at least one other therapeutic agent to the patient. In certain embodiments, an antigen binding protein or interfering RNA is administered prior to the administration of at least one other therapeutic agent. In certain embodiments, an antigen binding protein or interfering RNA is administered concurrent with the administration of at least one other therapeutic agent. In certain embodiments, an antigen binding protein or interfering RNA is administered subsequent to the administration of at least one other therapeutic agent.

In one embodiment, the at least one antigen binding protein or antibody and/or the interfering RNA is administered to a subject in combination with an anti-PCSK9 antibody (e.g., Repatha®·, Praluent®, bococizumab). In another embodiment, the at least one antigen binding protein or antibody and/or the interfering RNA is administered to a subject in combination with at least one other cholesterol-lowering (serum and/or total body cholesterol) agent. In some embodiments, the agents that increase the expression of LDLR, have been observed to increase serum HDL levels, lower LDL levels or lower triglyceride levels. Exemplary agents include, but are not limited to, statins (e.g., atorvastatin, cerivastatin, fluvastatin, lovastatin, mevastatin, pitavastatin, pravastatin, rosuvastatin, simvastatin), Nicotinic acid (Niacin) (NIACOR, NIASPAN (slow release niacin), SLO-NIACIN (slow release niacin)), Fibric acid (LOPID (Gemfibrozil), TRICOR (fenofibrate), Bile acid sequestrants (QUESTRAN (cholestyramine), colesevelam (WELCHOL), COLESTID (colestipol)), Cholesterol absorption inhibitors (ZETIA (ezetimibe)), combining nicotinic acid with statin (ADVICOR (LOVASTATIN and NIASPAN), combining a statin with an absorption inhibitor (VYTORIN (ZOCOR and ZETIA) and/or lipid modifying agents. In some embodiments, the at least one antigen binding protein and/or interfering RNA is combined with PPAR gamma agonsits, PPAR alpha/gamma agonists, squalene synthase inhibitors, CETP inhibitors, anti-hypertensives, anti-diabetic agents (such as sulphonyl ureas, insulin, GLP-1 analogs, DDPIV inhibitors), ApoB modulators, MTP inhibitoris and/or arteriosclerosis obliterans treatments. In some embodiments, the at least one antigen binding protein and/or interfering RNA is combined with an agent that increases the level of LDLR protein in a subject, such as statins, certain cytokines like oncostatin M, estrogen, and/or certain herbal ingredients such as berberine. In some embodiments, the at least one antigen binding protein and/or interfering RNA is combined with an agent that increases serum cholesterol levels in a subject (such as certain anti-psycotic agents, certain HIV protease inhibitors, dietary factors such as high fructose, sucrose, cholesterol or certain fatty acids and certain nuclear receptor agonists and antagonists for RXR, RAR, LXR, FXR). The combination of the two can allow for the undesirable side-effects of other agents to be mitigated by the antigen binding protein or interfering RNA.

Diagnostic Uses

In one embodiment, antigen binding proteins of the invention are useful for detecting the presence of ASGR, ASGR-1 and/or ASGR-2 in a biological sample. The term "detecting" as used herein encompasses quantitative or qualitative detection. In certain embodiments, a biological sample comprises a cell or tissue. In certain embodiments, such tissues include tissues that express ASGR, ASGR-1 and/or ASGR-2 at higher levels relative to other tissues.

In one embodiment, the invention provides a method of detecting the presence of ASGR, ASGR-1 and/or ASGR-2 in a biological sample. In certain embodiments, the method comprises contacting the biological sample with an antigen binding protein of the invention under conditions permissive for binding of an antigen binding protein to ASGR, ASGR-1 and/or ASGR-2, and detecting whether a complex is formed between the antigen binding protein and ASGR, ASGR-1 and/or AS GR-2.

In one embodiment, the invention provides a method of diagnosing a disorder associated with increased or decreased expression of ASGR, ASGR-1 and/or ASGR-2. In certain embodiments, the method comprises contacting a test cell with an antigen binding protein; determining the level of expression (either quantitatively or qualitatively) of ASGR, ASGR-1 and/or ASGR-2 by the test cell by detecting binding of the antigen binding protein to ASGR, ASGR-1 and/or ASGR-2; and comparing the level of expression of ASGR, ASGR-1 and/or ASGR-2 by the test cell with the level of expression of ASGR, ASGR-1 and/or ASGR-2 by a control cell (e.g., a normal cell of the same tissue origin as the test cell or a cell that expresses ASGR, ASGR-1 and/or ASGR-2 at levels comparable to such a normal cell), wherein a higher or lower level of expression of ASGR, ASGR-1 and/or ASGR-2 by the test cell as compared to the control cell indicates the presence of a disorder associated with increased or decreased expression of ASGR, ASGR-1 and/or ASGR-2. In certain embodiments, the test cell is obtained from an individual suspected of having a disorder associated with increased or decreased expression of ASGR, ASGR-1 and/or ASGR-2.

In certain embodiments, a method of diagnosis or detection, such as those described above, comprises detecting binding of an antigen binding protein of the invention to ASGR, ASGR-1 and/or ASGR-2 expressed on the surface of a cell or in a membrane preparation obtained from a cell expressing ASGR, ASGR-1 and/or ASGR-2 on its surface. In certain embodiments, the method comprises contacting a cell with an antigen binding protein under conditions permissive for binding of an antigen binding protein of the invention to ASGR, ASGR-1 and/or ASGR-2, and detecting whether a complex is formed between the antigen binding protein of the invention and ASGR, ASGR-1 and/or ASGR-2 on the cell surface. An exemplary assay for detecting binding of an antigen binding protein of the invention to ASGR, ASGR-1 and/or ASGR-2 expressed on the surface of a cell is a "FACS" assay.

Certain other methods can be used to detect binding of antigen binding protein of the invention to ASGR, ASGR-1 and/or ASGR-2. Such methods include, but are not limited to, antigen-binding assays that are well known in the art, such as western blots, radioimmunoassays, ELISA (enzyme linked immunosorbent assay), "sandwich" immunoassays, immunoprecipitation assays, fluorescent immunoassays, protein A immunoassays, and immunohistochemistry (IHC).

In certain embodiments, antigen binding proteins of the invention are labeled. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes $^{32}$P, $^{14}$C, $^{125}$I, $^{3}$H, and $^{131}$I, fluorophores such as rare earth chelates or fluorescein and its derivatives, rhodamine and its derivatives, dansyl, umbelliferone, luceriferases, e.g., firefly luciferase and bacterial luciferase (U.S. Pat. No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

In certain embodiments, antigen binding proteins of the invention are immobilized on an insoluble matrix. Immobilization entails separating the antigen binding protein of the invention from any ASGR, ASGR-1 and/or ASGR-2 that remains free in solution. This conventionally is accomplished by either insolubilizing the antigen binding protein of the invention before the assay procedure, as by adsorption to a water-insoluble matrix or surface (see, e.g., Bennich et al., U.S. Pat. No. 3,720,760), or by covalent coupling (for example, using glutaraldehyde cross-linking), or by insolubilizing the antigen binding protein of the invention after formation of a complex between the antigen binding protein of the invention and ASGR, ASGR-1 and/or ASGR-2, e.g., by immunoprecipitation.

The invention having been described, the following examples are offered by way of illustration, and not limitation.

Numerous sequences have been provided herein. Where there is a discrepancy in the sequences, the sequences in the tables presented within the figures control, unless there is an indication otherwise. If there is any unintended difference between the same consensus sequences, the consensus sequences as provided in the figures (from the tables within the figures) will control (unless indicated otherwise). For any further descrepancies (rather than just alternative sequences) the sequences within Tables 1-7 will control, unless designated otherwise. The figures contain multiple sequences, sequence alignments and sequence components of various nucleic and amino acid sequences. The present specification references this information in terms of the designated tables and/or the designated figures. Either reference (via figure or table) can be used and either designation (figure or table) will indicate the alternative designation as well, where appropriate. Thus, FIG. 48 designates Table 1, FIG. 49 designates Table 2, FIG. 50 designates Table 3, FIG. 51 designates Table 4, FIG. 52 designates Table 5, FIG. 53 designates Table 6, FIG. 54 designates Table 7, FIG. 55 designates Tables 19A, 19B, 19C, 20A, 20B, and 20C, FIG. 56 designates Tables 21-48, and FIG. 57 designates Tables 49-134, and vice versa. As such, any discussion herein in regard to the above figures or tables is interchangeable with respect to the "table" or "figure" nomenclature.

## EXAMPLES

## Example 1 - Identification of rare sequence variants that disrupt ASGR-1 function and lower non-HDL cholesterol and protect against coronary artery disease

The level of circulating non-high density lipoprotein (non-HDL) cholesterol is heritable and strongly correlated with the risk of coronary artery disease (CAD) and myocardial infraction (MI). Whole-genome sequencing offers the potential to search for rare sequence variants that have large effects on serum lipid levels and hence the risk of cardiovascular disease, such as CAD and MI.

METHODS

Study Participants:

Details of the population sample sets from Iceland, Denmark and The Netherlands, used to measure the various lipids traits (non-HDL cholesterol, HDL cholesterol, LDL cholesterol and triglycerides), alkaline phosphatase (ALP), ferritin, and vitamin B12, are outlined in Table 1.2. The dataset for ferritin is not shown. The coronary artery disease case-control sample sets that were a part of the study are outlined in Table 1.1.

Icelandic study population

Study participants were enrolled as part of various genetics programs at deCODE. Blood lipid levels (total cholesterol, non-high density lipoprotein cholesterol (non-HDL-C), low density lipoprotein cholesterol (LDL-C), high density lipoprotein cholesterol (HDL-C) and triglycerides), alkaline phosphatase and vitamin B12 levels were obtained from three of the largest laboratories in Iceland: 1) Landspitali - The National University Hospital of Iceland (LUH), Reykjavik (measurements performed between the years 1993 and 2012, hospitalized and ambulatory patients), 2) The Laboratory in Mjódd (RAM), Reykjavik (measurements performed between 2004 and 2012, ambulatory patients) and 3) Akureyri Hospital, The Regional Hospital in North Iceland, Akureyri (performed between 2004 and 2010, hospitalized and ambulatory patients). Information on the participants is outlined in Table 1.2. Lipid levels were adjusted for sex, year of birth and age at measurement, lipid lowering medication and measurement site, using the average of multiple measurements for an individual, and then normalized to a standard normal distribution using quantile normalization. To obtain effect estimates in mmol/L the estimates from the regression analysis were multiplied by the estimated standard deviation of lipid level in the population. Given their approximately log-normal distribution, triglyceride levels were log-transformed before adjustment and the corresponding effect estimates are presented as percentage change instead of units of mmol/L. The total number of individuals with non-HDL cholesterol, LDL cholesterol, HDL cholesterol and triglycerides in Iceland is shown in the Table 1.3 below. For each lipid, the number of chip-typed and directly imputed individuals and those with familial imputations is also shown.

Table 1.3: Lipid levels of Icelandic Study Participants

| | Non-HDL-C | LDL-C | HDL-C | Triglycerides |
|---|---|---|---|---|
| **Total number** | 119,146 | 53,841 | 119,514 | 80,111 |
| Direct imputation | 69,277 | 51,029 | 69,414 | 59,678 |
| Familial imputation | 49,869 | 2,812 | 50,100 | 20,433 |

The total number of Icelandic individuals with lipid values used in the study and the breakdown into those that were chip-typed and directly imputed (Direct imputation) and those that were first and second degree relatives of chip-typed individuals and had their genotypes inferred based on genealogy (Familial imputation).

Non-HDL cholesterol was obtained by subtracting HDL cholesterol from total cholesterol and measures the amount of cholesterol carried within all atherogenic lipoprotein particles (VLDL, IDL, LDL, chylomicrons and Lp(a)). The LDLcholesterol was calculated, using the Friedewald equation (for triglyceride levels < 4.00 mmol/L) (Friedewald, W. T., Levy, R. I. & Fredrickson, D. S. Estimation of the concentration of low-density lipoprotein cholesterol in plasma, without use of the preparative ultracentrifuge. Clin. Chem. 18, 499-502 (1972)). Total cholesterol and HDL-cholesterol values are a mixture of fasting and non-fasting values, whereas triglycerides are fasting values exclusively.

Coronary artery disease (CAD) was defined as a) individuals in the MONICA registry who suffered myocardial infarction (MI) before the age of 75 in Iceland between 1981 and 2002 and satisfied the MONICA criteria (Gudbjartsson, et al., Large-scale whole-genome sequencing of the Icelandic population. Nature genetics 2015), b) subjects with CAD discharge diagnoses (ICD 9 codes 410.*, 411.*, 412.*, 414.* or ICD 10 codes 120.0, I21.*, 122.*. I23.*, 124.*, I25.*) from LUH, c) subjects diagnosed with significant angiographic CAD (see defined below) identified from a nationwide clinical registry of coronary angiography and percutaneous coronary interventions at LUH between the years 1987 and 2012, d) subjects undergoing coronary artery bypass grafting (CABG) procedures at LUH between the years 2002 and 2011 or e) cause of death or contributing cause of death listed as MI or CAD (ICD 9 or 10 codes) on death registries between the years 1996 and 2009. Coronary angiograms in the nationwide registry were evaluated by an interventional cardiologist. Patients were considered to have significant angiographic CAD if one or more of the three major epicardial coronary vessels or the left main coronary artery was found to have at least 50% stenosis by visual estimation.

**Non-Icelandic study populations**

Characteristics of the non-Icelandic sample sets are outlined in Table 1.1 and Table 1.2. All the studies outlined in Tables 1.1 and 1.2 were approved by appropriate bioethics and/or data protection authorities. For samples from the Nijmegen Biomedical Study, Netherlands, the lipid values (namely, total cholesterol, HDL-cholesterol and triglycerides) were all non-fasting values. For samples from the Danish Inter99 and Addition studies, the lipid values were all fasting values. All participating subjects donating biological samples signed informed consents. Personal identities of the phenotypes and biological samples were encrypted by a third party system provided by the Icelandic Data Protection Authority.

**Data Generation and Analysis**

*Wholegenome sequencing, SNP calling, and imputation*

The Icelandic samples were genotyped using Illumina microarrays (Samani NJ et al., Genomewide association analysis of coronary artery disease. The New England journal of medicine 2007;357:443-53). The whole-genomes of 2,636 Icelanders were sequenced using the standard TruSeq methodology (Illumina) to a mean depth of at least 10X (median 20X)(Samani NJ et al., Genomewide association analysis of coronary artery disease. The New England journal of medicine 2007;357:443-53). For improved sequencing coverage of the GC-rich intron 4 in *ASGR-1* gene, the whole-genome sequence data generated for 738 Icelanders was analyzed using TruSeq PCR-free method from Illumina (mean depth of 30X). The del12 variant in intron 4 of *ASGR-1* was detected in this dataset.

*Single-Track assay SNP and microsatellite genotyping:*

We performed single SNP genotyping of rs186021206, using the Centaurus (Nanogen) platform (Gretarsdottir S, et al., Genome-wide association study identifies a sequence variant within the DAB2IP gene conferring susceptibility to abdominal aortic aneurysm. Nature genetics 2010;42:692). The del12 variant was genotyped using a PCR based method with the following primers: forward primer (NED labelled) 5'-TTCATCTTTCTTCCCACATTGC-3' (SEQ ID NO: 32600), reverse primer 5'-GGGCCTGAGAGAGACGTTCA-3' (SEQ ID NO: 32601). An internal size standard was added to the resulting PCR products and the fragments were separated and detected on an Applied Biosystems model 3730 sequencer, using in-house Allele Caller software.

*Statistical analyses:*

Associations between imputed genotypes and serum lipids (non-HDL cholesterol, HDL cholesterol, LDL cholesterol and triglycerides), ALP, ferritin and vitamin B12 levels in the Icelandic dataset were tested using a generalized linear regression, assuming an additive genetic model (Samani NJ et al., Genomewide association analysis of coronary artery disease. The New England journal of medicine 2007;357:443-53; and Olsen MH, et al., N-terminal pro-brain natriuretic peptide, but not high sensitivity C-reactive protein, improves cardiovascular risk prediction in the general population. European heart journal 2007;28:1374-81). For the Icelandic dataset, logistic regression was used to test for association between the del12 variant and coronary artery disease and myocardial infarction, treating the disease status as the response and the number of copies of del12 an individual carries as the explanatory variable. Coronary artery disease case-control association analysis for the non-Icelandic sample sets was done using the NEMO software (Jorgensen AB, et al., Loss-of-function mutations in APOC3 and risk of ischemic vascular disease. The New England journal of medicine 2014;371:32-41) assuming a multiplicative risk model. Results for the Icelandic and the non-Icelandic sample sets were combined using a Mantel-Haenszel fixed effects model. To estimate the effect of the del12 variant on myocardial infarction-free survival, Kaplan-Meier curves were estimated for survival to first myocardial infarction in heterozygous carriers and non-carriers (Hoogendoorn EH, et al., Thyroid function and prevalence of anti-thyroperoxidase antibodies in a population with borderline sufficient iodine intake: influences of age and sex. Clinical chemistry 2006;52:104-11) by dividing the corresponding chi-square statistic by 1.36 for non-HDL cholesterol, 1.57 for HDL cholesterol, 1.40 for triglycerides, 1.53 for ALP, 1.30 for vitamin B12, 1.71 for coronary artery disease and 1.48 for myocardial infarction.

To obtain a reliable imputation of the del12 variant, 3,799 Icelandic individuals were genotyped for the del12 variant and those genotypes were used as a training set for imputation of the del12 variant into the rest of the Icelandic population. The imputation information for del12 was 0.99.

The Icelandic samples were genotyped using Illumina microarrays as described above (Gudbartssoon, DF, et al., Large Scale whole-genome sequencing of the Icelandic population. Nature Genetics 2015). The whole-genomes of 2,636 Icelanders were sequenced using Illumina standard TruSeq methodology to a mean depth of at least 10X (median 20X) (Di Angelantonio E, et al., Major lipids, apolipoproteins, and risk of vascular disease. Jama 2009;302:1993-2000). A total of 35.5 million autosomal SNPs and INDEL's were identified using the Genome Analysis Toolkit version 2.3.9.

Information about haplotype sharing was used to improve variant genotyping, taking advantage of the fact that all sequenced individuals had also been chip-typed and long-range-phased. Variants were annotated using Ensembl release 72 and Variant Effect Predictor (VEP) version 2.8. Of the 35.5 million sequence variants found, 25.3 million variants passed the quality threshold and were imputed into 104,220 Icelanders who had been genotyped using Illumina chips. Additionally, using the Icelandic genealogy, genotype probabilities were calculated for 294,212 untyped individuals who are first and second degree relatives of the chip-typed individuals born after 1880 (Gudbartssoon, DF, et al., Large Scale whole-genome sequencing of the Icelandic population. Nature Genetics 2015). The informativeness of genotype imputation (imputation information) was estimated by the ratio of the variance of imputed expected allele counts and the variance of the actual allele counts:

$$\frac{Var(E(\theta|chip\ data))}{Var(\theta)},$$

where $\theta$ is the allele count. $Var(E(\theta|chip\ data))$ was estimated by the observed variance of the imputed expected counts and $Var(\theta)$ was estimated by $p(1 - p)$, where $p$ is the allele frequency.

For improved sequencing coverage of the GC-rich intron 4 in *ASGR-1* gene, whole-genome sequence ("WGS") data generated for 738 Icelanders was analyzed using TruSeq PCR-free method from Illumina (mean depth of 30X). This PCR-free method gave much better coverage of GC-rich regions including the *ASGR-1* intron 4. The del12 variant in intron 4 of *ASGR*-1 was detected in five individuals in this dataset.

To provide improved coverage of the associated region (1Mb centered on ASGR-1), a new dataset was analyzed that included an additional 5,817 WGS individuals (on top of the 2,636 WGS Icelanders). These additional individuals were sequenced with either Illumina TruSeq PCR free or TrueSeq Nano methods. These Illumina TrueSeq methods give enhanced sequence coverage as compared to the standard Illumina TrueSeq method (median sequencing depth 32X). The identified sequence variants were imputed into 150,656 Icelandic chipped-typed individuals, and with the use of genealogy information, into primary and secondary relatives of chip-typed individuals that were un-typed. In this expanded dataset, we identified another rare (0.027%), novel variant, W158X. The W158X variant is a four bp INDEL in exon 7 of ASGR-1 (NM_001671.4:c.469_472dupAACT) that causes frameshift and introduction of premature stop codon at amino acid 158 out of the 291 amino acid full length protein (NP_001662.1:p.Trp158X). A total of 345 individuals were Sanger-sequenced based on the imputation predicted carriers and non-carriers of c.469_472dupAACT. In this dataset, 79 c.469_472dupAACT carriers and 270 non-carriers were identified. This genotype data was then used to re-impute the variant into the Icelandic dataset. For non-HDL cholesterol, a larger sample set (n=136,261) was used in the association analysis outlined in Tables 1.4A and 1.4B.

Associations between imputed genotypes and serum lipids (non-HDL cholesterol, HDL cholesterol, LDL cholesterol and triglycerides), ALP and vitamin B12 levels in the Icelandic dataset were tested using a generalized linear regression, assuming an additive genetic model (Gudbjartsson DF, et al., Large-scale whole-genome sequencing of the Icelandic population. Nature genetics 2015; and Steinthorsdottir V, et al., Identification of low-frequency and rare sequence variants associated with elevated or reduced risk of type 2 diabetes. Nature genetics 2014;46:294-8). All measurements were adjusted for age, sex and measurement site, and average was taken over the available measurements after adjustment and inverse normal transformation. The lipid measurements were further adjusted for statin use. Removing individual known to take lipid lowering drugs in the Icelandic dataset did not alter the association with non-HDL cholesterol. The effect, in standardized units, changed from -0.29 (95% CI -0.38, -0.20; P = $4.0 \times 10^{-11}$) to -0.30 (-0.39, -0.21; P = $6.7 \times 10^{-11}$). This amounted to excluding 16,295 individuals, out of 119,146 individuals with non-HDL cholesterol information.

The lipid, ALP and vitamin B12 measurements from the Danish Inter99 study, ADDITION Denmark screening cohort, and the Nijmegen biomedical study, were adjusted and transformed in the same way and tested for association with allele count of del12 and rs186021206 using the linear regression implemented in the R software package. Results from the different populations were combined using the inverse variance fixed-effects method with METAL (Willer CJ, et al., METAL: fast and efficient meta-analysis of genomewide association scans. Bioinformatics 2010;26:2190-1). Effect estimates from the regression analysis are expressed in units of standard deviation (SD). To obtain effect estimates in mg/dL for non-HDL cholesterol, LDL cholesterol and HDL cholesterol, the estimates from the regression analysis were multiplied by the estimated SD of the population distributions. Triglyceride, ALP and vitamin B12 levels were log-transformed before adjustment as their distributions are approximately log-normal, and the corresponding effect estimates are presented as percentage change.

For the Icelandic dataset, logistic regression was used to test for association between the del12 variant and coronary artery disease and myocardial infarction, treating the disease status as the response and the number of copies of the deletion an individual carries as the explanatory variable. Other available individual characteristics that correlate with disease status were also included in the model as nuisance variables (Gudbjartsson DF, et al., Large-scale whole-genome sequencing of the Icelandic population. Nature genetics 2015). Coronary artery disease case-control association analysis for the non-Icelandic sample sets was done using the NEMO software (Gretarsdottir S, et al., The gene encoding phosphodiesterase 4D confers risk of ischemic stroke. Nature genetics 2003;35:131-8) assuming a multiplicative risk model. Results for the Icelandic and the non-Icelandic sample sets were combined using a Mantel-Haenszel fixed-effects model. Heterogeneity in the effect estimate was tested assuming that the estimated odds ratios for different groups follows a log-normal distribution using a likelihood ratio test with degrees of freedom equal to the number of groups compared minus one.

To estimate the effect of the del12 variant on myocardial infarction free survival, we estimated the Kaplan-Meier curves for survival to first myocardial infarction in heterozygous carriers and non-carriers stratified by sex and tested the difference in survival between carriers and non-carriers using the Cox proportional model. The analysis was performed using the survival library in the R software package. The survival analysis was based on 87,718 chip genotyped Icelanders and 44,655 Icelandic first and second degree relatives of chip typed individuals after restricting our analysis to those who lived to be at least 40 years old. Death was treated as a censoring event.

**Functional Characterization of the del12 Variant in ASGR-1**

*cDNA preparation, amplification, Sanger sequencing and next generation sequencing:*

RNA was isolated from blood samples from carriers and non-carriers of del12. Following cDNA generation, the region between exon 3 and 5 in *ASGR-1* was PCR amplified and the identified PCR products (two for del12 carriers and one for non-carriers) were Sanger sequenced using standard methodology to determine the sequence difference between the identified cDNA products. To quantify the ratio between the two amplified cDNA PCR products, they were sequenced using Illumina MiSeq instrument coupled with the MiSeq v2 reagent kit.

*Western blot analysis:*

The wild type *ASGR-1* cDNA and *ASGR-1* cDNA with the 22bp deletion were transiently overexpressed in HeLa cells to determine if *ASGR-1* transcripts with the 22 bp deletion generated stable truncated ASGR-1 protein as evaluated by western blot analysis.

RNA was isolated from blood samples using a Qiagen RNA maxi kit. Concentration and quality of the RNA was determined using an Agilent 2100 Bioanalyzer (Agilent Technologies), all samples had RIN values over 7. Following cDNA generation, the region between exon 3 and 5 in *ASGR-1* was PCR amplified using the Advantage® 2 Polymerase kit (Clontech) with the forward primer, CACTCAGGTCCTTCTGCTGTTTC (SEQ ID NO: 32602) and the reverse primer, 5'-ACCTCGCCTCCTCCTGCT-3' (SEQ ID NO: 32603). The resulting products were resolved on agarose gel and the identified PCR products (two for del12 carriers and one for non-carriers) were Sanger sequenced using standard methodology to determine the sequence difference between the identified cDNA products. To quantitate the ratio between the two amplified cDNA PCR products, they were sequenced using Illumina MiSeq instrument coupled with the MiSeq v2 reagent kit.

**Transient overexpression of wild type and mutated ASGR-1 harbouring the 22bp deletion at the end of exon 4 in HeLa cells.**

*Generation and cloning of wild type and mutated ASGR-1 cDNA:*

cDNA of ASGR-1 was obtained by PCR on human liver marathon ready cDNA (BD biosciences Clontech). The primers used were Forward 5'GCCAGCCCTATCATGACCAA'3 (SEQ ID NO: 32604) and Reverse 5'GCAGGTCGAGGCATTGAAGA'3 (SEQ ID NO: 32605). The resulting cDNA contained all exons including the start and stop codons of ASGR-1. PCR product was run on 1.6% Agarose gel and a band of the correct size was excised out and purified using QIAquick gel extraction kit (QIAGEN 28704) following the manufacturer's protocol. For cloning of ASGR-1 cDNA into pcDNA3.1/V5-His TOPO vector (Invitrogen K4800-01), 2μl of the gel extraction product was used and the manufacturer's protocol was followed resulting in pcDNA3.1_ASGR-1_WT. Transformed TOP10 chemically competent cells (Invitrogen C4040-10) were plated on LB plates containing 50μg/ml ampicillin. Colonies were expanded in 3ml LB medium containing 50μg/ml ampicillin. Plasmids were purified using QIAGEN plasmid mini kit (QIAGEN 12125) following the manufacturer's protocol. The plasmid sequence was confirmed by Sanger sequencing using the following sequencing primers: T7: 5'TAATACGACTCACTATAGGG'3 (SEQ ID NO: 32606), BGH: 5'TAGAAGGCACAGTCGAGG'3 (SEQ ID NO: 32607) and ASGR-1: 5'GAGGCAATGTGGGAAGAAAGATG'3 (SEQ ID NO: 32608)

*Introduction of 22bp deletion in ASGR-1:*

In order to generate a cDNA representative of the del12 carrier mRNA, targeted mutagenesis was performed. The Q5 Site-directed mutagenesis kit (New England BioLabs E0554S) and the pcDNA3.1_ASGR-1_WT plasmid was used as a template. In short, a PCR reaction was performed using the following primers 5'GAGGCAATGTGGGAAGAAAGATGAAGTCG'3 (SEQ ID NO: 32609) and 5'CTGGGCCTCCGTGCTCGC'3 (SEQ ID NO: 32610), resulting in a double-stranded DNA fragment representing the entire pcDNA3.1_ASGR-1_WT plasmid lacking the 22bp at the end of exon 4. Following the manufacturers recommendation, 1uL of the PCR reaction was used in the KLD reaction (New England BioLabs E0554S) wherein the PCR fragment is phosphorylated, re-circularized and the non-mutated template plasmid is removed. Mutated plasmids were transformed into NEB 5-alpha Competent cells (New England BioLabs C2987H) and plated on LB plates containing 50$\mu$g/ml ampicillin. Colonies were expanded in 3ml LB medium containing 50$\mu$g/ml ampicillin. Plasmids were purified using QIAGEN plasmid mini kit (QIAGEN 12125) following the manufacturer's protocol. ASGR-1_22bp_del sequence was confirmed by Sanger sequencing.

*Expression of ASGR-1 in Cultured cells:*

Two days prior to transfection, 100,000 HeLa cells (Public Health England 93021013) were seeded into each well of a 6-well plate in 3mL of DMEM medium (11995-065, ThermoFisher) supplemented with 10% fetal calf serum (ThermoFisher 10500-064) and 50 units/mL penicillin and 50 ug/mL streptomycin (ThermoFisher 15070-063). Cells were incubated at 37°C and 5% $CO_2$ in a humidified incubator.

The day before transfection, media was replaced with the without antibiotics. On the day of transfection, for each transfected well, 2.5ug of plasmids containing ASGR-1_WT or ASGR-1_del22 cDNA were diluted in 125uL Opti-Mem medium (ThermoFisher 31985-047) and 5uL of P3000 reagent (ThermoFisher L3000-008). Next, 3.75uL Lipofectamine 3000 (ThermoFisher L3000-008) were mixed with 125uL of Opti-Mem. Subsequently, the diluted plasmid solution was mixed with the Lipofectamine 3000 solution at a 1:1 ratio and incubated at room temperature for 5 minutes before the addition of 250uL of the combined solution to each transfected well.

24 hours post transfection, the spent media was replaced with fresh without antibiotics. Selected wells were supplemented with 10uM MG132 (TOCRIS 1748) for 4.5 hours prior to harvesting of cells. 48 hours post transfection cells were harvested for analysis by washing wells 2x with PBS (ThermoFisher 14190-250) followed by an 8 minute incubation with 1mL of 0.5mM EDTA in PBS (ThermoFisher 15575-020). Next, the EDTA solution was aspirated and cells dislodged by pipetting of 2mL of fresh media. 3x6-wells were pooled for each experimental condition and cells were spun down at 300 x g for 5 minutes. The equivalent of 2x6-wells were lysed in 200uL of RIPA buffer for Western blot analysis. The remainder of cells were split in two and lysed in 300uL RLT buffer (Qiagen 74106) or 900uL Tissue and Cell lysis solution (Epicentre MTC096H) and snap frozen on dry ice for RNA and DNA extraction respectively. Three different transient expression experiments were done and all gave the same results.

*Quantitative PCR analysis:*

RNA was isolated from cells using the RNeasy Mini Kit (Qiagen 74106) according to manufacturer's recommendations, and concentration and quality was determined with Nanodrop 1000 spectrophotometer (Thermo Scientific). cDNA was synthesized using the High capacity cDNA reverse transcriptase kit (ThermoFisher). DNA was isolated from cells using the MasterPure DNA Purification Kit (Epicentre MCD85201) according to manufacturer's recommendation.

Analysis of gene expression and transfection efficiency was performed on total cDNA and DNA respectively, with real-time PCR on an ABI Prism 7900HT Sequence Detection System (ThermoFisher) using forward (AGACCTTCAGCATCTGGACAATG (SEQ ID NO: 32611)) and reverse (CGAGGTCCGGAGCAGAGA (SEQ ID NO: 32612)) primers and fluorescent labelled probe spanning exon junction 2-3 of the ASGR-1 gene (6FAM-CAGAAAAGGGCCACCTC-MGB (SEQ ID NO: 32613) (ThermoFisher). Human betaActin assay (ThermoFisher 4326315E) was run in parallel to verify normalization of input cDNA and DNA.

*Western blot analysis:*

Cells corresponding to two wells of a 6 well plate were lysed using 200$\mu$l of RIPA buffer with 1:1100 Halt protease and phosphatase inhibitor cocktail (Thermo Scientific 78442). Lysates were kept on ice for 10 min with agitation followed by sonication for 20 sec (Branson 2510) and additional agitation on ice for 10 min. Lysates were spun down at 4°C for 15 min at 14,000 x g. Total protein amount of lysates was estimated using the Pierce BCA protein assay kit (Thermo Scientific 23227). Samples were prepared using Novex Bolt LDS sample buffer (4x) (Life technologies B0007) and Novex Bolt sample reducing agent (10x) (Life technologies B0009) and run on Novex Nupage 4-12% Bis-Tris gel (Life technologies NP0335BOX). Total protein amount per lane was 24$\mu$g and PageRuler (Thermo scientific 26616) was used to estimate protein size. The gel was run at a constant of 200V for 50 min. Proteins were transferred to a nitrocellulose membrane (Life technologies IB23002) using iBlot2 (Life technologies). Membranes were allowed to dry and were then hydrated with MQ water before blotting. Membranes were blocked for 1 hour at room temperature using Odyssey blocking buffer PBS (Li-Cor 927-40000). Primary antibodies used were $\alpha$-ASGR-1 (Sigma-Aldrich HPA011954) 1:500 (recognizes amino acid 1-41) and $\alpha$-beta-actin (Abcam ab6276) 1:5000 incubated in blocking buffer with the addition of 0.1 % Tween for 3 hours at room temperature. Secondary antibodies used were $\alpha$-Rabbit 680RD (Li-Cor 926-68073) and $\alpha$-Mouse 800CW (Li-Cor 926-32212) both 1:20,000 in PBST + 0.01% SDS for 1 hour at room temperature. After washing the membrane it was allowed to dry and then scanned using the Odyssey infrared imaging system (Li-Cor Biosciences).

## Other diseases and traits in deCODE database;

The deCODE Genetics phenotype database contains medical information on diseases and traits obtained through collaboration with specialists in each field. This includes information on cardiovascular diseases (e.g., myocardial infarction, coronary arterial disease, peripheral arterial disease, atrial fibrillation, sick sinus syndrome and stroke), metabolic disorders (e.g., obesity, diabetes, and metabolic syndrome), psychiatric disorders (e.g., schizophrenia, bipolar disorder, anxiety and depression), addictions (e.g., nicotine, alcohol), inflammatory diseases (e.g., rheumatoid arthritis, lupus, and asthma), musculoskeletal disorders (e.g., osteoarthritis, osteoporosis), eye diseases (e.g., glaucoma), kidney diseases (e.g., kidney stones, kidney failure) and 29 types of cancer. Anthropometric measures have also been collected through several of these projects. Routinely measured traits from patient workups (e.g., sodium, potassium, bicarbonate, calcium, phosphate, creatinine, blood cell counts, hemoglobin, hematocrit, immunoglobulins, iron, vitamins, lipids, liver function tests and more) were obtained from the Landspitali University Hospital, Reykjavik, and the Icelandic Medical Center Laboratory in Mjodd (Laeknasetrid), Reykjavik. The number of independent and uncorrelated secondary traits tested for association with del12 amounts to 400.

## RESULTS

### ASSOCIATION OF SEQUENCE VARIANTS WITH NON-HDL CHOLESTEROL LEVELS

Sequence variants were first identified through whole-genome sequencing ("WSG") of 2,636 Icelanders to a median depth of 20x. These variants were imputed (assisted by long-range phased haplotypes) into the genomes of 104,220 Icelanders who had been genotyped using Illumina single nucleotide polymorphism (SNP) arrays. In addition, Icelandic genealogical information was used to calculate genotype probabilities for 294,212 close relatives to those genotyped. Using these data we screened for novel rare variants that associated with non-HDL cholesterol levels (n=1 19,146). A set of seven correlated (pairwise r2>0.7) rare non-coding SNPs on chromosome 17p13.1 associated with non-HDL cholesterol level. The seven variants span 80kb, including the asialoglycoprotein receptor 1 and 2 (ASGR-1 and ASGR-2) genes. The strongest association was represented by rs186021206 (minor allele frequency (MAF) = 0.43%) located downstream of ASGR-1 that associates with 8.9±1.5 mg/dl lowering of non-HDL cholesterol (P=1.4×10-9)(Table 1.4B).

The associated region was well covered by the whole-genome sequencing except for intron 4 of ASGR-1. This intron is 79 base pairs (bp) long and very GC rich. To explore this region further 738 individuals were whole genome sequenced with PCR-free sequencing (Illumina), that gave enhanced coverage of the intron and led to the identification of a 12bp deletion within the intron;NM_001671.4:c.284-36_283+33delCTGGGGCTGGGG here after referred to as del12. Following direct genotyping of del12 and imputation into the Icelandic dataset, we observed that del12 (MAF=0.41%) is highly correlated with rs186021206 (r2=0.86) and the six other correlated SNPs and associates even more strongly with lowering of non-HDL cholesterol levels (decrease of 10.2 ± 1.5 mg/dl, P=2.5×10-10) (Table 1.9A). Del12 also increases HDL cholesterol and decreases triglyceride (TG) levels, albeit to a much lesser degree than for non-HDL cholesterol (Tables 1.4A and 1.9B). None of the seven SNPs maintained a significant association with non-HDL cholesterol after adjusting for del12 indicating that del12 is sufficient to explain the non-HDL association.

To validate the del12 association with non-HDL cholesterol levels, del12 in samples from The Netherlands (Nijmegen Biomedical Study18) and Denmark (Danish Inter9919 and Danish Addition study20) were genotyped. Del12 associated with non-HDL cholesterol in each sample set with similar effect size as in Iceland (Table 1.2, Tables 1.4A and 1.4B and Table 1.9B). When all three datasets were combined with the Icelandic discovery data, it was established that del12 lowers non-HDL cholesterol by 11.6±1.5 mg/dl (P=1.0×10-16)(Table 1.9B).

To identify additional additional loss of function variants in ASGR-1, an extended dataset was screened based on sequence variants identified through whole-genome sequencing ("WSG") of an additional group of 5,817 WGS Icelanders on top of the 2,636 described above. In this dataset, a rare four bp insertion mutation was identified; namely, MAF=0.027%; NM_001671.4:c.469_472dupAACT. As mentioned throughout, this frameshift mutation introduces a premature stop codon at amino acid 158 out of the 291 amino acid full length protein (NP_001662.1:p.W158X). Potential carriers and non-carriers were directly genotyped using Sanger sequencing. Those genotypes were then used to re-impute p.W158X into 150,656 Icelandic chipped typed individuals and their first and second degree relatives. In this dataset, c.469_472dupAACT associates significantly with a decrease in non-HDL cholesterol (-21.6 mg/dL, 95% CI-34.2 to -9.6) and an increase in ALP (45.3% increase, 95% CI 20.4 to 68.2, P=$7.9\times10^{-6}$) (Table 1.8). The direction of the effects of c.469_472dupAACT and the effect sizes are similar to that of del12 (Table 1.8). Given that a single test was performed, these results provide a significant replication of the ASGR-1 loss of function effect on non-HDL and ALP. Furthermore, since W158X is not correlated with del12 (i.e. there was no overlap between individuals carrying W158X and del12), the W158X variant provides yet further proof that the loss of function in the ASGR-1 gene is responsible for the observed changes in non-HDL, Triglycerides, Alkaline Phosphatase, Ferritin and Vitamin B12 levels. For coronary artery disease, the odds ratio for W158X (c.469_472dupAACT) was 0.65 (95% CI 0.26 to 1.40; P=0.24). As mentioned above, the W158X (c.469_472dupAACT) variant is independent of del12 and none of the 79 carriers found in Iceland carried del12. The variant also appears to be specific to the Icelandic population as it is not detected in large population databases such as (Exome Aggregation Consortium (ExAC), Exome Variant Server (EVS), Genomes of the Netherlands (GoNL) and dbSNP.

DEL12 WITHIN INTRON 4 OF ASGR-1 CAUSES A SPLICING ERROR RESULTING IN A FRAMESHIFT

Since del12 is located in intron 4 of ASGR-1, we examined its effect on splicing between exons 4 and 5. The region between exon 3 and 5 in cDNA generated from blood samples from 12 non-carriers and 12 heterozygous carriers of del12 was PCR amplified (Figure 4). The PCR products were resolved by gel electrophoresis demonstrating a band of 239bp in non-carrier. In del12 carriers, however, a smaller 217bp band was noted in addition to the expected 239bp PCR product (Figure 4B). Upon Sanger sequencing of the cDNA products we identified in the 217bp cDNA fragment a 22bp deletion at the end of exon 4 (Figure 4C). The deletion of these 22bp from the ASGR-1 transcript appears to be driven by a pseudo 5'-splice site in exon 4 (Figure 4D). It causes a frameshift in carriers such that, if translated, the resulting protein would lack both the oligomerization and carbohydrate recognition domains. To quantify this splicing defect we used the Illumina TruSeq method for direct digital counting of sequencing reads that were generated by sequencing the two cDNA products found in del12 carriers. On average, 32 $\pm$ 13% of the total ASGR-1 transcripts were accounted for by the incorrectly spliced isoform (Figure 4E). This form could not be detected in non-carriers (Figure 4E). Together, these data identify ASGR-1 as the target gene for the non-HDL association at this locus and are consistent with the associated mutation, del12, disrupting the function of the ASGR-1 protein. ASGR-1 is the major subunit of the hepatic asioaloglycoprotein receptor (ASGR) known to recognize and mediate the endocytosis and degradation of a wide variety of desialylated glycoproteins that contain terminal galactose (Gal) or N-acetylgalactosamine (Gal-NAc) residues on their N-linked carbohydrate chains (Morell AG, Gregoriadis G, Scheinberg IH, Hickman J, Ashwell G. The role of sialic acid in determining the survival of glycoproteins in the circulation. The Journal of biological chemistry 1971;246:1461-7; Van Den Hamer CJ, Morell AG, Scheinberg IH, Hickman J, Ashwell G. Physical and chemical studies on ceruloplasmin. IX. The role of galactosyl residues in the clearance of ceruloplasmin from the circulation. The Journal of biological chemistry 1970;245:4397-402; Ashwell G, Harford J. Carbohydrate-specific receptors of the liver. Annual review of biochemistry 1982;51:531-54; Weigel PH. Galactosyl and N-acetylgalactosaminyl homeostasis: a function for mammalian asialoglycoprotein receptors. BioEssays: news and reviews in molecular, cellular and developmental biology 1994;16:519-24).

THE DEL12 VARIANT IN ASGR-1 AND RISK OF CORONARY ARTERY DISEASE

Given the effect of del12 on non-HDL cholesterol levels, its impact on risk of CAD in 33,090 cases and 236,254 controls from Iceland and 8,558 cases and 11,120 controls from the USA, the UK, New Zealand and Denmark was assessed. It was found that carriers of del12 have a lower risk of CAD than non-carriers (odds ratio 0.66; 95% confidence interval [CI] 0.55 to 0.79; P=$6.3\times10^{-6}$) (Figure 5A). There was no evidence of heterogeneity across the eight study populations (Phet=0.96). Del12 also decreases risk of MI in Iceland (hazard ratio 0.64; 95% CI, 0.64 to 0.80; P=$8.5\times10^{-5}$) (Figure 5B). In addition, del12 carriers have a 1.5 years longer lifespan than non-carriers (95% CI, 0.2 to 2.8 years; P=0.020).

There is a strong positive correlation between the effect of sequence variants on non-HDL cholesterol levels and risk of CAD (Haddad L, Day IN, Hunt S, Williams RR, Humphries SE, Hopkins PN. Evidence for a third genetic locus causing familial hypercholesterolemia. A non-LDLR, non-APOB kindred. Journal of lipid research 1999;40:1113-22; Timms KM, Wagner S, Samuels ME, et al. A mutation in PCSK9 causing autosomal-dominant hypercholesterolemia in a Utah pedigree. Human genetics 2004;114:349-53; Varret M, Rabes JP, Saint-Jore B, et al. A third major locus for autosomal dominant hypercholesterolemia maps to 1p34.1-p32. American journal of human genetics 1999;64:1378-87; Hunt SC, Hopkins PN, Bulka K, et al. Genetic localization to chromosome 1p32 of the third locus for familial hypercholesterolemia in a Utah kindred. Arterioscler Thromb Vase Biol 2000;20:1089-93; Do R, Willer CJ, Schmidt EM, et al.

Common variants associated with plasma triglycerides and risk for coronary artery disease. Nature genetics 2013;45:1345-52) (Figure 6, Table 1.5). However, several published variants, deviate from the overall trend. For example, LPA and ANGPTL4 variants have a substantially greater effect on CAD than their non-HDL effects would predict while the effect of the APOE variants is weaker than predicted by the non-HDL effect. Del12 in ASGR-1 is another example of a variant whose effect on CAD is stronger than predicted by the effect non-HDL cholesterol effect (Figure 6, Table 1.5).

ASSOCIATION OF DEL12 WITH SERUM LEVELS OF ALP AND VITAMIN B12

To determine the overall effect of del12 in ASGR-1, its effect on a variety of human diseases and other traits in the Icelandic dataset was screened. A highly significant association of del12 with higher levels of circulating alkaline phosphatase (ALP) ($33.6 \pm 2.8$ U/L increase, $P=3.6 \times 10^{-63}$) and vitamin B12 ($58.4 \pm 8.3$ pmol/L increase, $P=3.1 \times 10^{-12}$) was observed (Tables 8A and 8B and Table 18). An increase in ALP levels may reflect liver disease, however, there was no increase in del 12 carriers in serum gamma glutamyl transferase (GGT), bilirubin, alanine aminotransferase or other measures of liver function that commonly parallel changes in ALP in liver disease (Table 1.6).

The del12 association with higher levels of ALP and vitamin B12 in individuals from the Danish Inter99 study with comparable effect sizes ($P=9.9 \times 10^{-69}$ for ALP and $P=9.9 \times 10^{-14}$ for vitamin B12) was replicated (Table 1.10).

A common variant upstream of ASGR-1 (rs314253; MAF=35.1%) has been reported to associate modestly with both LDL cholesterol and ALP levels (Chambers JC, Zhang W, Sehmi J, et al. Genome-wide association study identifies loci influencing concentrations of liver enzymes in plasma. Nature genetics 2011;43:1131-8; Willer CJ, Schmidt EM, Sengupta S, et al. Discovery and refinement of loci associated with lipid levels. Nature genetics 2013;45:1274-83). This common variant association is replicated in the data of the present invention (strongest association for both ALP and non-HDL with the correlated rs56093546; MAF=21.6%) and that its associations with ALP and non-HDL are independent of the rare signal represented by del12 ($r2<0.001$, Table 1.5) as demonstrated. As for del12, this common variant has opposite effects on ALP and non-HDL; the allele that increases ALP decreases non-HDL (see Chambers; Willer) (Table 1.7).

| TABLE 1.1 | | | | |
|---|---|---|---|---|
| **Study** | **Design** | **Definition of CAD and MI cases** | **Assertainment of controls** | **Reference** |
| Iceland | Case/control | CAD and MI cases were defined by: a) discharge diagnoses (ICD 9 codes 410.*, 411.*, 412.*, 414.* or ICD 10 codes 120.0, 121.*, 122.*. 123.*, 124.*, I25.*) from LUH, b) significant angiographic CAD ($\geq$ 50% stenosis of the major coronary vessels), c) undergone coronary revascularisation (CABG) d) MI or CAD (ICD 9 or 10 codes) listed in death registries, or e) MI before the age of 75 from MONICA registry | Study participants from various deCODE genetics programs without known CVD. | Heigadottir A, Thorleifsson G, Manolescu A, et al. A common variant on chromosom e 9p21 affects the risk of myocardial infarction. Science (New York, NY) 2007;316:1 491-3. |

(continued)

| TABLE 1.1 | | | | |
|---|---|---|---|---|
| **Study** | **Design** | **Definition of CAD and MI cases** | **Assertainment of controls** | **Reference** |
| UK 1 - Leicester MI Study | Case/control | Cases included MI patients admitted to the coronary care units of the Leicester Royal Infirmary, Leicester and the Royal Hallamshire Hospital, Sheffield and satisfied the WHO criteria for acute MI. | Controls included adult visitors of individuals with non-cardiovascular disease from each hospital or individuals from three primary care practices located in the same geographical area. Individuals who reported a history of CAD were excluded. | Heigadottir A, Manolescu A, Thorleifsson G, et al. The gene encoding 5-lipoxygenas e activating protein confers risk of myocardial infarction and stroke. Nature genetics 2004;36:23 3-9. |
| UK2 - BHF Family Heart Study | Case/control | The British Heart Foundation Family Heart Study (BHF-FHS) CAD cases were index cases from families of European ancestry with a strong familial history of defined CAD recruited from throughout the United Kingdom. CAD was defined as a validated history of myocardial infarction or coronary revascularisation (PTCA or CABG) before the 66th birthday. | Controls were blood donors recruited by the United Kingdom Blood Service (UKBS) as part of the Wellcome Trust Case Control Consortium Study. | Genome-wide association study of 14,000 cases of seven common diseases and 3,000 shared controls. Nature 2007;447:6 61-78, and Samani NJ, Erdmann J, Hall AS, et al. Genomewid e association analysis of coronary artery disease. The New England journal of medicine 2007;357: 4 43-53. |
| Emory (Atlanta, Georgi a, USA) | Case/control | Cases were identified from subjects undergoing cardiac catheterization at the Emory University Hospital. CAD cases included those that had at least one significant stenosis ($\geq$50%) in any of the major coronary arteries on angiography, or those without significant stenosis but had history of MI, CABG, or PCI. | Controls included individuals undergoing cardiac catheterization with no or minimal CAD (<20% stenosis) and had no prior history of MI or CAD. Additional controls were recruited from the Grady Memorial Hospitals and Clinical Registry in Neurology (CRIN) and included individuals with non-vascular neurological diseases (mainly Parkinson's and Alzheimer's diseases), their spouses, unrelated friends and community volunteers; excluding those with a known history of CAD. | Heigadottir A, et al. (2007) |

(continued)

| TABLE 1.1 | | | | |
|---|---|---|---|---|
| Study | Design | Definition of CAD and MI cases | Assertainment of controls | Reference |
| Duke (Durham, North Carolina, USA) | Case/control | Participants were enrolled at Duke University Medical Center through the cardiac catheterization laboratories. MI cases included those with self-reported history of MI (corroborated by review of medical records), or those who suffered an MI during the study follow-up period. | Controls included those with no history of MI prior or subsequent to the index cardiac catheterization and no PCI or CABG ejection fraction on left ventriculogram greater than 40%, and stenosis less than 50% on coronary angiography. | Helgadottir A, et al. (2007) |
| UPenn (Philadelphia, Pennsylvania, USA) | Case/control | The study participants were enrolled at the University of Pennsylvania Medical Center and included subjects undergoing cardiac catheterization. CAD cases included those that had at least one significant stenosis ($\geq$ 50%) in any of the major coronary arteries on angiography, or those without significant stenosis but had history of MI, CABG, or PCI. | Controls included individuals without significant luminal stenosis on coronary angiography (luminal stenosis less than 50%). | Heigadottir A, et al. (2007) |
| New Zealand | Case/control | a) Significant angiographic CAD ($\geq$ 50% stenosis of the major coronary vessels), b) CABG-procedures c) MI or CAD (ICD 9 or 10 codes) in a clinical registry. | Study participants without known CAD and ultrasound screened for carotid artery disease and abdominal aortic aneurysm, with ankle brachial index to exclude peripheral artery disease. | Gretarsdotti r S, Baas AF, Thorleifsson G, et al. Genome-wide association study identifies a sequence variant within the DAB2IP gene conferring susceptibilit y to abdominal aortic aneurysm. Nature genetics 2010;42:69 2-7. |

(continued)

| TABLE 1.1 | | | | |
|---|---|---|---|---|
| Study | Design | Definition of CAD and MI cases | Assertainment of controls | Reference |
| Denmark 1 (Gentofte cadlab) | Case/control | Cases were identified from subject investigated by coronary artery angiography because of suspected ischemic heart disease, valvular heart disease or cardiomyopathy. CAD cases included those that had at least one significant stenosis ($\geq$50%) in any of the major coronary arteries on angiography | Individuals in Monica10 and Inter99 studies without CAD diagnosis based on information from the Danish National Patient Registry and the Danish Register of Causes of Death. | |
| Denmark 2 (Monica10) | Case/control | Monica10 is a population based study. Participants were recruited from the Danish Central Personal Register as random samples of the population in the southern part of the former Copenhagen County. Cardiovascular events were defined as first ever non-fatal or fatal CVD (ICD-8: 390-448/ICD-10: 100-179). Assessment of the cardiovascular endpoints was based on data from the Danish National Patient Registry and the Danish Register of Causes of Death. | Individuals in Monica10 and Inter99 studies without CAD diagnosis based on information from the Danish National Patient Registry and the Danish Register of Causes of Death. | Olsen MH, Hansen TW, Christensen MK, et al. N-terminal pro-brain natriuretic peptide, but not high sensitivity C-reactive protein, improves cardiovascu lar risk prediction in the general population. European heart journal 2007;28:13 74-81. |
| Denmark 3 (Inter99) | Case/control | The Inter99 study is a population-based randomized controlled trial (CT00289237, ClinicalTrials.gov) investigating the effects of lifestyle intervention on cardiovascular disease. Cardiovascular events were defined as first ever non-fatal or fatal CVD (ICD-8: 390-448/ICD-10: 100-179). Assessment of the cardiovascular endpoints was based on data from the Danish National Patient Registry and the Danish Register of Causes of Death. | Individuals in Monica10 and Inter99 studies without CAD diagnosis based on information from the Danish National Patient Registry and the Danish Register of Causes of Death. | 14. Jorg ensen AB, Frikke-Schmidt R, Nordestgaa rd BG, Tybjaerg-Hansen A. Loss-of-function mutations in APOC3 and risk of ischemic vascular disease. The New England journal of medicine 2014;371:3 2-41. |

(continued)

| TABLE 1.1 | | | | |
|---|---|---|---|---|
| **Study** | **Design** | **Definition of CAD and MI cases** | **Assertainment of controls** | **Reference** |
| Sweden | Case / Control | Ischemic stroke patients from the clinic at Karolinska Univerity Hospital, Stockholm. The ischemic stroke diagnosis was based on clinical findings and brain imaging (CT or MRI). | Population-based controls, either healthy blood donors or healthy volunteers recruited at the Karolinska Hospital | Gretarsdotti r et al (2008) Traylor et al (2012) |
| South Germany | Case / Control | Ischemic stroke patients recruited at the stroke unit of the Department of Neurology, Klinikum Grosshadern, University of Munich. Diagnoses were based on clinical findings and imaging evidence (either CT or MRI), and were clinically confirmed by neurologists. | Gender and age matched individuals without history of cardiovascular disease selected from the KORA S4 Study | Traylor et al (2008) Gschwendt ner et al (2009) Wichmann et al (2005) |
| West Germany | Case / Control | Ischemic stroke patients recruited through hospitis participating in the regional Westphalian Stroke Register. Diagnoses were based on clinical findings and imaging evidence (either CT or MRI), and were clinically confirmed by neurologists. | Population controls with a self-reported history of stroke from the population based Dortmund Health Study | Traylor et al (2008) Berger et al (2007) |
| United Kingdom | Case / Control | Ischemic stroke patients recruited through a cerebrovascular service clinic. All cases were phenotyped by one experienced stroke neurologist with review of original brain imaging with CT or MRI. | Community controls, age and gender matched and free of symptomatic cerebrovascular disease were recruited from the same geographic area as the patients. | Traylor et al (2008) Gschwendt ner et al (2009) |

**Table 1.2.** Characteristics of Participants in the Discovery and Replication Studies of the association of del12 Variant with Plasma Lipid, Alkaline Phosphatase, and Vitamin B12 levels

| Trait[a] | Iceland | Nijmegen Biomedical Study (Netherlands)[c] | Inter99 study (Denmark)[d] | Addition Study (Denmark)[e] |
|---|---|---|---|---|
| Ancestry | Caucasian | Caucasian | Caucasian | Caucasian |
| N[b] | 194,958 | 5,645 | 7,633 | 9,689 |
| Mean age (SD), yrs | 58.2 (40.6-75.8) | 55.8 (38.0-73.6) | 48.5 (36.1-55.5) | 59.9 (53.1-66.7) |
| Gender, % female | 53.4% | 53.6% | 49.9% | 46.4% |
| Non-HDL cholesterol (SD), mg/dL | 154.7 (109.1-200.3) | 170.7 (129.4-212.0) | 161.6 (117.5-205.7) | 164.7 (124.0-205.4) |
| LDL cholesterol (SD), mg/dL | 133.0 (91.6-174.4) | 138.6 (102.2-175.0) | 137.2 (99.7-174.7) | 139.3 (101.9-176.1) |
| HDL cholesterol (SD), mg/dL | 54.7 (37.7-71.7) | 52.6 (39.2-66.0) | 54.2 (38.4-70.0) | 60.0 (43.6-76.4) |
| Total Cholesterol (SD), mg/dL | 208.0 (162.6-253.4) | 223.4 (180.9-265.9) | 215.8 (173.6-258.0) | 224.7 (183.9-265.5) |
| Triglycerides (SD), mg/dL | 133.6 (67.6-190.5) | 155.8 (94.5-256.8) | 105.8 (60.8-183.9) | 117.4 (73.5-187.3) |
| Alkaline phosphatase (SD), IU/l | 87.1 (53.5-141.7) | na | 41.3 (30.7-55.6) | na |
| Vitamin B12 (SD), pmol/l | 398 (256-618) | na | 398 (286-554) | na |

[a]The average values (where available) for each of the traits listed is shown ($\pm$ one SD). [b]Number of individuals with measurements for at least one of the traits. [c]Wetzels et al (2007)[5], [d]Jörgensen et al (2003)[6],[e]Lauritzen et al (2000)[7].

**Table 1.4A. Association of del12 with Non-HDL Cholesterol, LDL Cholesterol, HDL Cholesterol, Triglyceride, ALP and Vitamin B12 in Iceland, Denmark and The Netherlands**

| | Study population (n) | del12 freq. (%) | Effect (95% CI)[a] | P value | Population mean value[e] ($\pm$ 1SD) |
|---|---|---|---|---|---|
| | **Non-HDL cholesterol** | | mg/dL | | mg/dL |
| Discovery | Iceland (119,146) | 0.41 | -13.6 (-17.7,-9.4) | $2.5 \times 10^{-10}$ | 154.7 (109.1-200.3) |
| Replication | Denmark A[b] (6,182) | 0.22 | -21.3 (-36.8,-5.9) | 0.0069 | 161.6 (117.5-205.7) |
| Replication | Denmark B[c] (9,656) | 0.32 | -22.2 (-32.8,-11.7) | $3.8 \times 10^{-5}$ | 164.7 (124.0-205.4) |
| Replication | The Netherlands[d] (5,537) | 0.50 | -17.0 (-28.3,-5.7) | 0.0032 | 170.7 (129.4-212.0) |
| | Combined | | -15.3 (-18.9,-11.7) | $1.0 \times 10^{-16}$ | |
| | **LDL cholesterol** | | mg/dL | | |
| Discovery | Iceland (53,841) | 0.41 | -9.5 (-14.0,-5.1) | $2.8 \times 10^{-5}$ | 133.0 (91.6-174.4 |
| Replication | Denmark A (6,098) | 0.22 | -22.1 (-35.5,-8.7) | 0.0012 | 137.2 (99.7-174.7) |

(continued)

| | | | | | |
|---|---|---|---|---|---|
| **LDL cholesterol** | | | **mg/dL** | | |
| Replication | Denmark B (8,080) | 0.32 | -19.0 (-29.2,-8.8) | 0.00026 | 139.3 (101.9-176.1) |
| Replication | The Netherlands (5,523) | 0.50 | -16.0 (-26.1,-6.0) | 0.0018 | 138.6 (102.2-175.0) |
| | Combined | | -12.5 (-16.2,-8.8) | $3.9\times10^{-11}$ | |
| **HDL cholesterol** | | | **mg/dL** | | **mg/dL** |
| Discovery | Iceland (119,514) | 0.41 | 2.4 (0.7,4.1) | 0.0058 | 54.7 (37.7-71.7) |
| Replication | Denmark A (6,182) | 0.22 | 4.6 (-0.8,9.9) | 0.096 | 54.2 (38.4-70.0) |
| Replication | Denmark B (9,656) | 0.32 | 2.4 (-1.8,6.7) | 0.26 | 60.0 (43.6-76.4) |
| Replication | The Netherlands (5,537) | 0.50 | 2.4 (-1.3,6.0) | 0.20 | 52.6 (39.2-66.0) |
| | Combined | | 2.5 (1.1,4.0) | 0.00039 | |
| **Triglyceride** | | | **% change** | | **mg/dL** |
| Discovery | Iceland (80,011) | 0.41 | -6.1 (-10.8,-1.5) | 0.012 | 133.6 (67.6-190.5) |
| Replication | Denmark A (6,182) | 0.22 | -6.0 (-25.2,11.4) | 0.53 | 105.8 (60.8-183.9) |
| Replication | Denmark B (8,163) | 0.32 | -8.9 (-21.0,2.3) | 0.15 | 117.4 (73.5-187.3) |
| Replication | The Netherlands (5,537) | 0.50 | -4.4 (-17.9,8.2) | 0.52 | 155.8 (94.5-256.8) |
| | Combined | | -6.3 (-10.3,-2.3) | 0.0032 | |
| **ALP** | | | **% change** | | **U/L** |
| Discovery | Iceland (126,060) | 0.41 | 50.1 (42.9,57.2) | $3.6\times10^{-63}$ | 87.1 (53.5-141.7) |
| Replication | Denmark A[c] (5,829) | 0.22 | 29.1 (14.8,42.5) | $3.1\times10^{-6}$ | 41.3 (30.7-55.6) |
| | Combined | | 46.5 (40.1,52.7) | $5.6\times10^{-69}$ | |
| **Vitamin B12** | | | **% change** | | **pmol/L** |
| Discovery | Iceland (97,910) | 0.41 | 16.6 (11.5,21.5) | $3.1\times10^{-12}$ | 398 (256-618) |
| Replication | Denmark A[c] (5,826) | 0.22 | 18.6 (3.9,32.4) | 0.0053 | 398 (286-554) |
| | Combined | | 16.8 (12.0,21.5) | $8.3\times10^{-14}$ | |

[a]Effect estimates and 95% confidence intervals (95% CI) in mg/dL for the non-HDL cholesterol and HDL cholesterol and as percentage change for triglyceride, ALP and vitamin B12. [b]The Danish Inter99 study (Jorgensen et al. 2003). [c]The Danish Addition study (van den Donk et al. 2011). [d]The Nijmegen Biomedical Study (Hoogendoom et al. 2006). "For triglyceride, ALP and vitamin B12, the population mean and the SD are calculated for log-transformed values and transformed back to original units. To convert the values for non-HDL and HDL cholesterol to millimoles per liter, multiply by 0.02586. To convert triglyceride to mmol/L, multiply by 0.01129.

| Table 1.4B. Association of del12 and rs186021206 with Cholesterols, Triglyceride, Alkaline Phosphatase and Vitamin B12 Measurements in Iceland, Denmark and the Netherlands. | | | | | | | |
|---|---|---|---|---|---|---|---|
| | **rs186021206** | | | | **del12** | | |
| **Trait / Cohort (n)[a]** | **Effect[b]** | **Effect (95% CI)[c]** | ***P*** | ***P*[adj][d]** | **Effect[b]** | **Effect (95% CI)[c]** | ***P*** |
| **Non-HDL cholesterol** | **SD** | **mg/dL** | | | | **mg/dL** | |
| Iceland (119,146) | -0.28 | -12.9 (-17.1,-8.7) | $1.4\times10^{-9}$ | 0.39 | -0.30 | -13.6 (-17.7,-9.4) | $2.5\times10^{-10}$ |

(continued)

| Table 1.4B. Association of del12 and rs186021206 with Cholesterols, Triglyceride, Alkaline Phosphatase and Vitamin B12 Measurements in Iceland, Denmark and the Netherlands. | | | | | | | | |
|---|---|---|---|---|---|---|---|---|
| | | rs186021206 | | | | del12 | | |
| Trait / Cohort (n)[a] | | Effect[b] | Effect (95% CI)[c] | P | $P_{adj}$[d] | Effect[b] | Effect (95% CI)[c] | P |
| **Non-HDL cholesterol** | | SD | mg/dL | | | | mg/dL | |
| Denmark (6,182) | A | -0.38 | -16.7 (-27.9,-5.4) | 0.0038 | 0.64 | -0.48 | -21.3 (-36.8,-5.9) | 0.0069 |
| Denmark (9,656) | B | -0.32 | -13.1 (-21.0,-5.3) | 0.0011 | 0.74 | -0.55 | -22.2 (-32.8,-11.7) | $3.8\times10^{-5}$ |
| The Netherlands (5,537) | | -0.23 | -9.7 (-19.9,0.5) | 0.062 | 0.19 | -0.41 | -17.0 (-28.3,-5.7) | 0.0032 |
| Combined | | -0.29 | -12.9 (-16.3,-9.6) | $2.0\times10^{-14}$ | 0.24 | -0.34 | -15.3 (-18.9,-11.7) | $1.0\times10^{-16}$ |
| **LDL cholesterol** | | mg/dL | | | | | mg/dL | |
| Iceland (53,841) | | -0.22 | -9.2 (-13.6,-4.7) | $5.5\times10^{-5}$ | 0.78 | -0.23 | -9.5 (-14.0,-5.1) | $2.8\times10^{-5}$ |
| Denmark (6,098) | A | -0.43 | -16.1 (-25.8,-6.3) | 0.0012 | 0.56 | -0.59 | -22.1 (-35.5,-8.7) | 0.0012 |
| Denmark (8,080) | B | -0.34 | -12.5 (-20.3,-4.7) | 0.0016 | 0.86 | -0.51 | -19.0 (-29.2,-8.8) | 0.00026 |
| The Netherlands (5,523) | | -0.36 | -13.2 (-22.3,-4.2) | 0.0041 | 0.81 | -0.44 | -16.0 (-26.1,-6.0) | 0.0018 |
| Combined | | -0.28 | -11.1 (-14.5,-7.8) | $1.0\times10^{-10}$ | 0.70 | -0.31 | -12.5 (-16.2,-8.8) | $3.9\times10^{-11}$ |
| **Total cholesterol** | | mg/dL | | | | | mg/dL | |
| Iceland (125,381) | | -0.22 | -9.9 (-14.0,-5.7) | $3.1\times10^{-6}$ | 0.78 | -0.23 | -10.5 (-14.7,-6.4) | $6.5\times10^{-7}$ |
| Denmark (6,182) | A | -0.32 | -13.5 (-24.2,-2.8) | 0.014 | 0.54 | -0.33 | -14.0 (-28.7,0.8) | 0.063 |
| Denmark (9,656) | B | -0.30 | -12.0 (-19.9, 4.2) | 0.0027 | 0.97 | -0.47 | -19.2 (-29.8,-8.6) | 0.00040 |
| The Netherlands (5,537) | | -0.21 | -9.0 (-19.5,1.5) | 0.0927 | 0.48 | -0.33 | -14.1 (-25.7,-2.5) | 0.018 |
| Combined | | -0.24 | -10.5 (-13.8,-7.2) | $5.1\times10^{-10}$ | 0.68 | -0.27 | -12.0 (-15.6,-8.5) | $5.6\times10^{-11}$ |
| **HDL cholesterol** | | mg/dL | | | | | mg/dL | |
| Iceland (119,514) | | 0.13 | 2.2 (0.5,3.9) | 0.011 | 0.0055 | 0.14 | 2.4 (0.7,4.1) | 0.0058 |
| Denmark (6,182) | A | 0.15 | 2.4 (-1.5,6.4) | 0.22 | 0.84 | 0.29 | 4.6 (-0.8,9.9) | 0.096 |
| Denmark (9,656) | B | 0.03 | 0.4 (-2.7,3.6) | 0.79 | 0.32 | 0.15 | 2.4 (-1.8,6.7) | 0.26 |
| The Netherlands (5,537) | | 0.02 | 0.2 (-3.1,3.5) | 0.9 | 0.043 | 0.18 | 2.4 (-1.3,6.0) | 0.20 |
| Combined | | 0.10 | 1.6 (0.4,2.9) | 0.01 | 0.001 | 0.15 | 2.5 (1.1,4.0) | 0.00039 |

(continued)

| Triglyceride | | | % change | | | | % change | |
|---|---|---|---|---|---|---|---|---|
| Iceland (80,011) | | -0.11 | -5.4 (-10.1,-0.8) | 0.027 | 0.13 | -0.12 | -6.1 (-10.8,-1.5) | 0.012 |
| Denmark (6,182) | A | -0.26 | -13.4 (-26.1,-1.6) | 0.046 | 0.11 | -0.11 | -6.0 (-25.2,11.4) | 0.53 |
| Denmark (8,163) | B | -0.03 | -1.3 (-11.2,8.0) | 0.79 | 0.099 | -0.2 | -8.9 (-21.0,2.3) | 0.15 |
| The Netherlands (5,537) | | 0.13 | 6.5 (-7.0,19.1) | 0.32 | 0.0057 | -0.09 | -4.4 (-17.9,8.2) | 0.52 |
| Combined | | -0.09 | -4.2 (-7.9,-0.6) | 0.028 | 0.0066 | -0.13 | -6.3 (-10.3,-2.3) | 0.003 |
| **ALP** | | | % change | | | | % change | |
| Iceland (126,060) | A | 0.82 | 48.9 (41.8,55.8) | $1.2 \times 10^{-61}$ | 0.10 | 0.84 | 50.1 (42.9,57.2) | $3.6 \times 10^{-63}$ |
| Denmark (6,035) | | 0.70 | 23.0 (13.2,32.4) | $2.2 \times 10^{-7}$ | 0.092 | 0.86 | 29.1 (14.8,42.5) | $3.1 \times 10^{-6}$ |
| Combined | | 0.80 | 41.5 (35.9,47.0) | $1.9 \times 10^{-67}$ | 0.026 | 0.84 | 46.5 (40.1,52.7) | $5.6 \times 10^{-69}$ |
| **Vitamin B12** | | | % change | | | | % change | |
| Iceland (97,910) | | 0.33 | 15.8 (10.8,20.7) | $2.0 \times 10^{-11}$ | 0.15 | 0.35 | 16.6 (11.5,21.5) | $3.1 \times 10^{-12}$ |
| Denmark (6,032) | A | 0.49 | 17.6 (7.2,27.7) | 0.00027 | 0.011 | 0.52 | 18.6 (3.9,32.4) | 0.0053 |
| Combined | | 0.35 | 16.1 (11.6,20.6) | $4.3 \times 10^{-14}$ | 0.84 | 0.36 | 16.8 (12.0,21.5) | $8.3 \times 10^{-14}$ |

[a]Number of individuals with trait value and genotypes. [b]Effect estimates from the regression in units of standard deviations (SD) of the distributions of the adjusted values. [c] Effect estimates and 95% confidence intervals (95% CI) in mg/dL for the cholesterol, and as percentage change for triglyceride, ALP and vitamin B12.

[d]P-values adjusted for the effect of del12. "The Netherlands", The Nijmegen Biomedical Study[15]; "Denmark A", The Danish Inter99 study[6]; "Denmark B", The Danish Addition study[16].

| Table 1.5. The association of published lipid variants with non-HDL cholesterol levels and coronary artery disease in Iceland. | | | | | | | | |
|---|---|---|---|---|---|---|---|---|
| Build 36 position | | | | Non-HDL (mg/dL) | | Coronary artery disease | | |
| Chr | Position (hg18) | MAF | Info | Effect | SE | OR | 95% CI | |
| 1 | 25,641,524 | 0.47184 | 0.996 | 0.7 | 0.2 | 0.99 | 0.97 | 1.02 |
| 1 | 55,278,235 | 0.01173 | 0.986 | -17.2 | 1.0 | 0.73 | 0.66 | 0.81 |
| 1 | 62,725,961 | 0.21814 | 0.996 | 1.6 | 0.3 | 1.01 | 0.98 | 1.03 |
| 1 | 62,906,518 | 0.33844 | 0.998 | -2.3 | 0.2 | 0.99 | 0.97 | 1.01 |
| 1 | 92,766,395 | 0.19052 | 0.999 | 0.8 | 0.3 | 0.99 | 0.97 | 1.02 |
| 1 | 109,620,053 | 0.20789 | 0.999 | 4.8 | 0.3 | 1.08 | 1.06 | 1.11 |
| 1 | 110,000,250 | 0.41287 | 0.995 | 1.0 | 0.2 | 1.01 | 0.99 | 1.03 |
| 1 | 149,225,460 | 0.15162 | 0.997 | -0.7 | 0.3 | 1.03 | 1.00 | 1.06 |
| 1 | 154,967,275 | 0.28892 | 0.998 | -0.5 | 0.2 | 0.99 | 0.97 | 1.02 |
| 1 | 219,036,651 | 0.28689 | 0.994 | 0.9 | 0.2 | 1.01 | 0.98 | 1.03 |
| 1 | 228,362,314 | 0.39128 | 0.999 | -1.1 | 0.2 | 0.99 | 0.97 | 1.01 |
| 1 | 232,915,962 | 0.4424 | 0.999 | 1.2 | 0.2 | 1.00 | 0.98 | 1.03 |

(continued)

| Table 1.5. The association of published lipid variants with non-HDL cholesterol levels and coronary artery disease in Iceland. | | | | | | | | |
|---|---|---|---|---|---|---|---|---|
| **Build 36 position** | | | | **Non-HDL (mg/dL)** | | **Coronary artery disease** | | |
| **Chr** | **Position (hg18)** | **MAF** | **Info** | **Effect** | **SE** | **OR** | **95% CI** | |
| 2 | 21,087,477 | 0.04518 | 0.999 | -6.1 | 0.5 | 0.94 | 0.89 | 0.99 |
| 2 | 21,117,405 | 0.3491 | 0.997 | 2.9 | 0.2 | 1.05 | 1.03 | 1.07 |
| 2 | 21,139,562 | 0.1408 | 0.999 | 4.3 | 0.3 | 1.08 | 1.04 | 1.11 |
| 2 | 27,584,444 | 0.34466 | 0.998 | -1.8 | 0.2 | 1.00 | 0.98 | 1.03 |
| 2 | 27,584,716 | 0.20151 | 0.995 | -1.4 | 0.3 | 1.00 | 0.97 | 1.02 |
| 2 | 43,927,385 | 0.27892 | 0.999 | -2.6 | 0.2 | 0.95 | 0.93 | 0.98 |
| 2 | 43,953,086 | 0.19027 | 0.997 | -1.5 | 0.3 | 0.96 | 0.94 | 0.99 |
| 2 | 63,003,061 | 0.32014 | 0.997 | 0.9 | 0.2 | 1.02 | 1.00 | 1.05 |
| 2 | 118,293,189 | 0.07895 | 0.998 | -0.8 | 0.4 | 1.02 | 0.98 | 1.06 |
| 2 | 121,025,958 | 0.41077 | 0.994 | 0.6 | 0.2 | 1.03 | 1.01 | 1.06 |
| 2 | 169,538,401 | 0.37685 | 0.999 | -0.5 | 0.2 | 0.99 | 0.97 | 1.01 |
| 2 | 216,012,629 | 0.32322 | 0.998 | 0.8 | 0.2 | 0.95 | 0.93 | 0.97 |
| 3 | 12,271,469 | 0.3667 | 0.998 | -1.2 | 0.2 | 0.99 | 0.97 | 1.02 |
| 3 | 32,508,014 | 0.07924 | 0.997 | -1.6 | 0.4 | 0.98 | 0.94 | 1.02 |
| 3 | 133,691,893 | 0.11977 | 0.998 | -1.1 | 0.3 | 0.99 | 0.96 | 1.03 |
| 3 | 172,209,912 | 0.07646 | 0.999 | 0.8 | 0.4 | 1.08 | 1.04 | 1.12 |
| 4 | 3,442,937 | 0.40281 | 0.991 | 0.7 | 0.2 | 1.03 | 1.00 | 1.05 |
| 4 | 25,672,088 | 0.14802 | 0.993 | 0.9 | 0.3 | 1.04 | 1.01 | 1.07 |
| 4 | 88,249,285 | 0.40279 | 0.999 | 0.7 | 0.2 | 1.00 | 0.98 | 1.02 |
| 4 | 100,233,828 | 0.42298 | 0.998 | 0.5 | 0.2 | 1.01 | 0.99 | 1.03 |
| 5 | 74,661,243 | 0.35407 | 0.999 | 2.8 | 0.2 | 1.04 | 1.02 | 1.06 |
| 5 | 122,883,315 | 0.47211 | 0.995 | 0.5 | 0.2 | 1.00 | 0.98 | 1.02 |
| 5 | 156,322,875 | 0.35741 | 0.998 | 1.7 | 0.2 | 1.01 | 0.99 | 1.03 |
| 6 | 16,217,142 | 0.46163 | 0.995 | -0.8 | 0.2 | 0.99 | 0.97 | 1.01 |
| 6 | 26,201,120 | 0.06713 | 1.000 | -1.5 | 0.4 | 0.99 | 0.95 | 1.03 |
| 6 | 31,373,469 | 0.29084 | 0.993 | 0.8 | 0.2 | 1.02 | 1.00 | 1.04 |
| 6 | 43,865,874 | 0.47286 | 0.993 | 0.9 | 0.2 | 1.02 | 1.00 | 1.04 |
| 6 | 100,706,818 | 0.19956 | 0.998 | -1.0 | 0.3 | 1.00 | 0.97 | 1.02 |
| 6 | 116,444,196 | 0.40848 | 0.998 | -0.6 | 0.2 | 0.98 | 0.96 | 1.00 |
| 6 | 127,494,332 | 0.47183 | 0.999 | 0.9 | 0.2 | 1.01 | 0.99 | 1.03 |
| 6 | 139,873,450 | 0.42692 | 0.999 | -0.7 | 0.2 | 0.98 | 0.96 | 1.00 |
| 6 | 160,881,127 | 0.01773 | 1.000 | 4.0 | 0.8 | 1.31 | 1.21 | 1.41 |
| 6 | 160,930,108 | 0.06104 | 0.984 | 2.3 | 0.4 | 1.27 | 1.22 | 1.33 |
| 7 | 21,573,877 | 0.22512 | 0.992 | 1.5 | 0.3 | 1.00 | 0.98 | 1.02 |
| 7 | 25,958,351 | 0.14423 | 0.993 | 0.9 | 0.3 | 1.05 | 1.02 | 1.08 |
| 7 | 44,548,856 | 0.2013 | 0.990 | 2.0 | 0.3 | 1.02 | 1.00 | 1.05 |
| 7 | 44,567,220 | 0.42549 | 0.998 | -1.2 | 0.2 | 0.97 | 0.95 | 0.99 |
| 7 | 72,620,810 | 0.11552 | 0.998 | -0.9 | 0.3 | 1.02 | 0.99 | 1.06 |
| 7 | 72,697,942 | 0.46468 | 0.997 | 0.5 | 0.2 | 0.99 | 0.97 | 1.01 |
| 7 | 130,095,474 | 0.44163 | 0.998 | -0.5 | 0.2 | 0.96 | 0.94 | 0.98 |
| 8 | 9,221,641 | 0.07554 | 0.997 | 1.9 | 0.4 | 1.04 | 1.00 | 1.08 |
| 8 | 18,316,718 | 0.18705 | 0.996 | -1.3 | 0.3 | 0.96 | 0.94 | 0.99 |
| 8 | 19,888,502 | 0.08181 | 0.996 | -2.1 | 0.4 | 0.93 | 0.89 | 0.97 |
| 8 | 19,910,123 | 0.45471 | 0.996 | -1.0 | 0.2 | 0.96 | 0.94 | 0.98 |
| 8 | 55,584,167 | 0.24432 | 1.000 | 1.0 | 0.2 | 1.02 | 0.99 | 1.04 |

(continued)

| Table 1.5. The association of published lipid variants with non-HDL cholesterol levels and coronary artery disease in Iceland. | | | | | | | | |
|---|---|---|---|---|---|---|---|---|
| **Build 36 position** | | | | **Non-HDL (mg/dL)** | | **Coronary artery disease** | | |
| **Chr** | **Position (hg18)** | **MAF** | **Info** | **Effect** | **SE** | **OR** | **95% CI** | |
| 8 | 59,548,473 | 0.31037 | 0.998 | -1.4 | 0.2 | 0.99 | 0.97 | 1.01 |
| 8 | 116,733,072 | 0.26318 | 0.999 | -1.1 | 0.2 | 1.00 | 0.97 | 1.02 |
| 8 | 126,543,488 | 0.22755 | 0.997 | -1.9 | 0.3 | 0.96 | 0.94 | 0.99 |
| 8 | 126,551,803 | 0.49199 | 0.999 | -2.3 | 0.2 | 0.95 | 0.93 | 0.97 |
| 8 | 145,094,645 | 0.385 | 0.990 | 0.7 | 0.2 | 0.98 | 0.96 | 1.00 |
| 9 | 2,630,759 | 0.09898 | 0.998 | -1.3 | 0.4 | 0.97 | 0.94 | 1.01 |
| 9 | 16,894,846 | 0.31865 | 0.998 | -0.5 | 0.2 | 0.97 | 0.95 | 0.99 |
| 9 | 106,704,122 | 0.25781 | 0.999 | -1.1 | 0.2 | 0.97 | 0.95 | 0.99 |
| 9 | 106,724,051 | 0.28833 | 0.997 | -0.6 | 0.2 | 0.99 | 0.97 | 1.02 |
| 9 | 135,122,694 | 0.38646 | 0.997 | -0.9 | 0.2 | 0.99 | 0.97 | 1.01 |
| 9 | 135,143,989 | 0.15248 | 0.995 | 1.0 | 0.3 | 1.05 | 1.02 | 1.08 |
| 10 | 94,829,632 | 0.42892 | 0.993 | -0.6 | 0.2 | 0.99 | 0.97 | 1.01 |
| 11 | 18,612,847 | 0.30731 | 0.998 | 0.8 | 0.2 | 1.02 | 1.00 | 1.04 |
| 11 | 61,305,450 | 0.27208 | 0.991 | 0.8 | 0.2 | 1.01 | 0.99 | 1.04 |
| 11 | 61,354,548 | 0.38782 | 0.998 | -1.1 | 0.2 | 1.00 | 0.98 | 1.02 |
| 11 | 116,144,314 | 0.06787 | 0.999 | -5.8 | 0.4 | 0.94 | 0.91 | 0.98 |
| 11 | 116,159,645 | 0.46743 | 0.999 | -0.5 | 0.2 | 0.97 | 0.95 | 0.99 |
| 11 | 116,206,564 | 0.00228 | 0.979 | -15.1 | 2.3 | 0.91 | 0.73 | 1.14 |
| 11 | 122,039,714 | 0.40275 | 0.996 | 0.6 | 0.2 | 1.01 | 0.99 | 1.03 |
| 11 | 125,749,162 | 0.10572 | 0.999 | 0.7 | 0.3 | 1.02 | 0.99 | 1.06 |
| 12 | 110,492,139 | 0.38236 | 0.999 | 0.8 | 0.2 | 0.94 | 0.92 | 0.96 |
| 12 | 110,794,963 | 0.2284 | 0.999 | 0.8 | 0.3 | 0.94 | 0.92 | 0.97 |
| 12 | 119,901,033 | 0.30901 | 0.994 | 0.9 | 0.2 | 1.03 | 1.01 | 1.05 |
| 13 | 31,851,388 | 0.44766 | 0.999 | -0.7 | 0.2 | 0.99 | 0.97 | 1.01 |
| 14 | 23,953,727 | 0.49889 | 0.995 | 0.8 | 0.2 | 0.98 | 0.96 | 1.01 |
| 15 | 56,518,445 | 0.19278 | 0.999 | -0.6 | 0.3 | 0.99 | 0.97 | 1.02 |
| 16 | 55,542,640 | 0.38939 | 0.991 | -1.8 | 0.2 | 0.97 | 0.95 | 0.99 |
| 16 | 55,572,592 | 0.06047 | 0.997 | 2.9 | 0.5 | 1.04 | 1.00 | 1.09 |
| 16 | 66,485,543 | 0.10432 | 1.000 | -0.8 | 0.3 | 0.97 | 0.94 | 1.01 |
| 16 | 70,665,594 | 0.14755 | 0.997 | 1.3 | 0.3 | 1.03 | 1.00 | 1.06 |
| 17 | 7,032,374 | 0.35058 | 0.996 | -1.0 | 0.2 | 0.98 | 0.96 | 1.00 |
| 17 | 8,101,874 | 0.49481 | 0.998 | -0.4 | 0.2 | 0.96 | 0.94 | 0.98 |
| 17 | 39,281,652 | 0.03364 | 0.989 | 1.3 | 0.6 | 1.08 | 1.02 | 1.15 |
| 17 | 42,746,803 | 0.28266 | 0.998 | 0.6 | 0.2 | 1.02 | 1.00 | 1.04 |
| 17 | 64,394,061 | 0.32561 | 0.995 | 0.5 | 0.2 | 1.03 | 1.01 | 1.05 |
| 18 | 45,363,953 | 0.01171 | 0.999 | 4.8 | 1.0 | 1.00 | 0.91 | 1.09 |
| 19 | 8,335,323 | 0.02392 | 0.965 | -4.7 | 0.7 | 0.80 | 0.74 | 0.86 |
| 19 | 11,063,306 | 0.0888 | 0.995 | -6.8 | 0.4 | 0.89 | 0.86 | 0.92 |
| 19 | 11,088,602 | 0.45236 | 0.997 | 1.4 | 0.2 | 1.02 | 1.00 | 1.04 |
| 19 | 19,268,718 | 0.07838 | 0.997 | -3.8 | 0.4 | 0.96 | 0.92 | 1.00 |
| 19 | 50,103,781 | 0.16819 | 0.980 | 8.4 | 0.3 | 1.05 | 1.02 | 1.08 |
| 19 | 50,103,919 | 0.05236 | 0.968 | -16.9 | 0.5 | 0.83 | 0.79 | 0.87 |
| 19 | 53,898,229 | 0.39118 | 0.997 | 1.1 | 0.2 | 1.00 | 0.98 | 1.03 |
| 19 | 57,016,028 | 0.27115 | 0.999 | 0.6 | 0.2 | 1.03 | 1.01 | 1.06 |
| 19 | 59,489,660 | 0.21613 | 0.990 | -0.6 | 0.3 | 0.99 | 0.96 | 1.02 |

(continued)

| Table 1.5. The association of published lipid variants with non-HDL cholesterol levels and coronary artery disease in Iceland. | | | | | | | | |
|---|---|---|---|---|---|---|---|---|
| **Build 36 position** | | | | **Non-HDL (mg/dL)** | | **Coronary artery disease** | | |
| **Chr** | **Position (hg18)** | **MAF** | **Info** | **Effect** | **SE** | **OR** | **95% CI** | |
| 20 | 12,910,718 | 0.45731 | 0.998 | 0.4 | 0.2 | 1.00 | 0.98 | 1.03 |
| 20 | 17,793,921 | 0.15541 | 0.991 | 0.8 | 0.3 | 0.98 | 0.95 | 1.01 |
| 20 | 38,613,850 | 0.34358 | 0.997 | -1.1 | 0.2 | 0.98 | 0.96 | 1.00 |
| 20 | 39,157,752 | 0.45945 | 0.997 | 1.1 | 0.2 | 0.99 | 0.97 | 1.01 |
| 20 | 42,475,778 | 0.04599 | 0.993 | -1.3 | 0.5 | 0.98 | 0.93 | 1.03 |
| 20 | 44,018,827 | 0.21978 | 0.998 | 1.3 | 0.3 | 0.98 | 0.96 | 1.01 |

Shown are the build 36 positions (hg18), minor allele frequency (MAF), imputation information, the non-HDL effect in mg/dL and the standard error of the estimate (SE), and the OR for coronary artery disease and 95% CI for the minor allele.

| Table 1.6. Association of del12 with various measures of liver function in Iceland | | | | | |
|---|---|---|---|---|---|
| **Phenotype** | **n[a]** | **Effect[b]** | **Effect (95% CI)[c]** | **P** | **Mean (± 1 SD)[d]** |
| | | | **% change** | | |
| Alanine Transaminase | 144,402 | 0.087 | 5.8 (-0.4,12.2) | 0.065 | 28.7 (15.0-54.8 ) units/L |
| Alkaline Phosphatase | 126,060 | 0.840 | 50.1 (42.9,57.2) | $3.6 \times 10^{-63}$ | 87.1 (53.5-141.7) units/L |
| Aspartate Transaminase | 144,931 | 0.072 | 4.1 (-2.9,11.4) | 0.095 | 28.1 (14.2-55.6) units/L |
| Bilirubin | 94,805 | 0.054 | 3.7 (-2.6,10.4) | 0.25 | 9.1 (4.6-18.0) $\mu$m/L |
| Gamma Glutamyl Transpeptidase | 138,844 | 0.113 | 10.3 (1.7,19.2) | 0.015 | 30.9 (13.1-72.9 ) units/L |
| | | | **g/L** | | |
| Albumin | 78,555 | -0.109 | -0.72 (-1.37,0.06) | 0.033 | 39.5 (33.0-46.0) g/L |

[a]Number of individuals used in the association analysis for each of the traits. [b]Effect estimate, in units of standard deviation, from regression of adjusted trait values on the expected genotype count of del12. [c]Effect estimates and 95% CI in original units. For traits with log-normal distribution the effects are presented as percentage change with 95% CI. [d]Mean trait values, ± one SD, in the Icelandic population. For traits with log-normal distribution the mean and SD is calculated for log-transformed trait values and transformed back to original units.

Table 1.7. Common Variants at the *ASGR-1* Locus Associated with Non-HDL Cholesterol and Alkaline Phosphatase in Iceland

| | rs314253 | rs56093546 | del12 |
|---|---|---|---|
| **Chromosome position** | 17:7032374 | 17:7004539 | 17:7020979 |
| **MAF (%)** | 35.06 | 21.63 | 0.43 |
| **Effect[a] on non-HDL cholesterol (P value)** | -0.03 ($5.9 \times 10^{-6}$) | -0.04 ($2.0 \times 10^{-6}$) | -0.30 ($2.5 \times 10^{-10}$) |
| **Adjusted for rs314253 (P)** | - | 0,022 | $7.9 \times 10^{-11}$ |
| **Adjusted for rs56093546 (P)** | 0,0068 | - | $7.2 \times 10^{-11}$ |

(continued)

**Table 1.7. Common Variants at the *ASGR-1* Locus Associated with Non-HDL Cholesterol and Alkaline Phosphatase in Iceland**

|  | rs314253 | rs56093546 | del12 |
|---|---|---|---|
| Chromosome position | 17:7032374 | 17:7004539 | 17:7020979 |
| Adjusted for del12 (*P*) | $6.4\times10^{-7}$ | $1.7\times10^{-6}$ | - |
| Effect[a] on ALP (P value) | 0.050 ($3.9\times10^{-21}$) | 0.068 ($7.4\times10^{-28}$) | 0.82 ($3.6\times10^{-63}$) |
| Adjusted for rs314253 (*P*) | - | $5.7\times10^{-12}$ | $4.1\times10^{-66}$ |
| Adjusted for rs56093546 (*P*) | 0,000042 | - | $2.0\times10^{-66}$ |
| Adjusted for del12 (*P*) | $4.2\times10^{-24}$ | $4.0\times10^{-31}$ | - |
| r$^2$, D' (relative to rs314253) | - | 0.29, 0.76 | 0.001, 0.60 |
| r$^2$, D' (relative to rs56093546) | 0.29, 0.76 | - | 0.001, 1.00 |

[a]Effect estimates from the regression in units of standard deviations of the distributions of the adjusted values.

The association of rs314253 with LDL cholesterol was reported in Willer et al 2013 and with ALP in Chambers et al., 2011.

**Table 1.8. Association of p.w158X and del12 with Cholesterols, Triglyceride, Alkaline Phosphatase, Vitamin B12 and CAD in an extended Icelandic dataset**

| Trait / (n)[a] | Effect[b] | p.W158X Effect (95% CI)[c] | *P* | Effect[b] | del12 Effect (95% CI)[c] | *P* |
|---|---|---|---|---|---|---|
| Non-HDL cholesterol | SD | mg/dL | | SD | mg/dL | |
| (136,261) | -0.45 | -21.6 (-34.2,-9.6) | 0.00057 | -0.29 | -13.3 (-17.2,-9.3) | $4.0\times10^{-11}$ |
| LDL cholesterol | | mg/dL | | | mg/dL | |
| (53,932) | -0.38 | -15.9 (-32.7,0.9) | 0.064 | -0.23 | -9.7 (-14.1,-5.1) | $2.8\times10^{-5}$ |
| Total cholesterol | | mg/dL | | | mg/dL | |
| (131,879) | -0.30 | -13.5 (-29.3,2.2) | 0.091 | -0.23 | -10.4 (-14.2,-6.5) | $1.4\times10^{-7}$ |
| HDL cholesterol | | mg/dL | | | mg/dL | |
| (124,437) | 0.14 | 2.4 (-3.9,8.7) | 0.45 | 0.15 | 2.5 (1.0,4.0) | 0.0016 |
| Triglyceride | | % change | | | % change | |
| (82,569) | -0.17 | -8.4 (-25.5,7.2) | 0.33 | -0.12 | -6.0 (-10.4,-1.8) | 0.0075 |
| ALP | | % change | | | % change | |
| (131,966) | 0.77 | 45.3 (20.4,68.2) | $7.9\times10^{-6}$ | 0.80 | 47.7 (2.2,87.1) | $5.6\times10^{-76}$ |
| Vitamin B12 | | % change | | | % change | |
| (102,624) | 0.26 | 15.6 (-4.3,34.0) | 0.084 | 0.33 | 17.5 (3.1,30.9) | $5.6\times10^{-16}$ |
| CAD | | OR | P | | OR | P |
| (35,134/275,567) | | 0.61 (0.26,1.40) | 0.24 | | 0.66 (0.54,0.81) | $4.5\times10^{-5}$ |

(continued)

| CAD | OR | P | OR | P |
|---|---|---|---|---|

[a]Number of individuals with trait value and genotypes. [b]Effect estimates from the regression in units of standard deviations (SD) of the distributions of the adjusted values. [c] Effect estimates and 95% confidence intervals (95% CI) in mg/dL for the cholesterols, and as percentage change for triglyceride, ALP and vitamin B12. [d]P-values adjusted for the effect of del12. This analysis was done on an updated Icelandic dataset that includes 8,453 WGS individuals and imputation into 150,656 Icelandic individuals. For none-HDL cholesterol association analysis an updated sample set was used that contained 136,261 Icelanders.

**Table 1.9A. Correlation and conditional analysis for del12 and the seven other SNPs that show the strongest association at 17p13.1 with non-HDL cholesterol in Iceland**

| Variant | Pos | EA | OA | EA.freq (%) | $r^{2\ d}$ | non-HDL Effect[c] | non-HDL P | $P_{adjdel12}$[a] | $P_{adjSNP}$[b] |
|---|---|---|---|---|---|---|---|---|---|
| chr17: 6930020:5 | 6930020 | T | C | 0.39 | 0.85 | -0.243 | $5.2×10^{-7}$ | 0.10 | 2.8E-05 |
| rs188743906 | 6931736 | T | C | 0.39 | 0.85 | -0.243 | $5.2×10^{-7}$ | 0.18 | 2.9E-05 |
| rs150983647 | 6942021 | T | C | 0.44 | 0.76 | -0.232 | $5.3×10^{-7}$ | 0.39 | 7.6E-05 |
| chr17: 6944653:5 | 6944653 | A | G | 0.39 | 0.85 | -0.242 | $5.9×10^{-7}$ | 0.10 | 2.3E-05 |
| rs146261845 | 6952978 | T | C | 0.40 | 0.75 | -0.259 | $1.1×10^{-7}$ | 0.88 | 0.00053 |
| chr17: 6961021:5 | 6961021 | C | T | 0.39 | 0.85 | -0.250 | $2.2×10^{-7}$ | 0.18 | 0.00010 |
| rs186021206 | 7010136 | A | G | 0.43 | 0.86 | -0.283 | $1.4×10^{-9}$ | 0.39 | 0.067 |
| del12 | **7020979** | del12 | - | 0.41 | | -0.297 | $2.5×10^{-10}$ | - | - |

[a]P-value for correlation between the SNP and the trait, tested conditional on the association of the trait with del12. [b]P-value for the correlation between the trait and del12, tested conditional on the association of the trait with the SNP. [c]Effect estimated in units of standardized trait values. [d]Correlation $r^2$ between del12 and sequencing genotypes of the SNPs in 2,128 Icelandic individuals. Shown are the build 36 positions (hg18).

**Table 1.9B. Association of del12 with Non-HDL Cholesterol, HDL Cholesterol and Triglyceride Measurements, in Iceland, Denmark and the Netherlands**

| | Study population (n) | Change[a] ⊠ SE | P value | Mean value[b] in non-carriers (SD) |
|---|---|---|---|---|
| | **Non-HDL cholesterol** | mg/dl | | mg/dl |
| Discovery | Iceland (119,146) | -10.4 ± 1.5 | $2.5×10^{-10}$ | 156.8 (38.2) |
| Replication | The Netherlandsc (5,156) | -15.4 ± 5.4 | 0.0032 | 170.7 (41.3) |
| Replication | Denmark A[d] (5,968) | -17.4 ± 8.1 | 0.0069 | 158.3 (42.9) |
| Replication | Denmark B[e] (8,822) | -21.6 ± 5.4 | $3.8×10^{-5}$ $1.0×10^{-16}$ | 164.5 (40.5) |
| | Combined | -11.6 ± 1.5 | | |
| | **HDL cholesterol** | mg/dl | | mg/dl |
| Discovery | Iceland (119,514) | 0±0.4 | 0.0058 | 55.2 (15.8) |
| Replication | The Netherlands (5,537) | 2.7±1.5 | 0.20 | 52.2 (13.1) |
| Replication | Denmark A (6,182) | 1.2±2.7 | 0.096 | 55.2 (15.4) |
| Replication | Denmark B (9,656) | 1.5±1.2 | 0.26 | 59.9 (16.2) |

(continued)

| | Study population | HDL cholesterol | mg/dl | | mg/dl |
|---|---|---|---|---|---|
| | | Combined | 0±0.4 | 0.00039 | |
| | | **Triglyceride - mg/dl** | **mg/dl** | | **mg/dl** |
| Discovery | | Iceland (80,011) | -1.2±1.5 | 0.012 | 130.9 (75.2) |
| Replication | | The Netherlands (5,537) | -0.4±5.8 | 0.52 | 176.9 (121.2) |
| Replication | | Denmark A (6,182) | 8.1±6.9 | 0.53 | 116.8 (84.0) |
| Replication | | Denmark B (8,163) | -3.5±2.3 | 0.15 | 131.8 (118.5) |
| | | Combined | -1.5±1.2 | 0.0030 | |

[a]Effect size, ± standard error, represents the difference in mean values between heterozygote carriers and non-carriers of the variants after adjusting for age, sex and, for Iceland, site and statin use. [b]Calculated based on unadjusted values. [c]The Nijmegen Biomedical Study (Wetzels et al. 2007). [d]The Danish Inter99 study (Jørgensen et al. 2003). [e]The Danish Addition study (Lauritzen et al. 2000). To convert the values for non-HDL cholesterol to millimoles per liter, multiply by 0.02586

**Table 1.10. Association of del12 with Alkaline Phosphatase and Vitamin B12 Serum Measurements in Iceland and Denmark**

| | Study population (n) | Change[a] ± SE | *P* value | Mean value[b] in non-carriers (SD) |
|---|---|---|---|---|
| | **ALP** | **U/L** | | **U/L** |
| Discovery | Iceland (126,060) | + 33.6 ± 2.8 | $3.6\times10^{-63}$ | 92.8 (64.0) |
| Replication | Denmark A (5,829) | + 15.8 ± 2.6 | $1.7\times10^{-6}$ | 42.9 (13.5) |
| | Combined | + 24.1 ± 1.9 | $9.9\times10^{-69}$ | |
| | **Vitamin B12** | **pmol/L** | | **pmol/L** |
| Discovery | Iceland (97,910) | +58.4 ± 8.3 | $3.1\times10^{-12}$ | 439.0 (171.0) |
| Replication | Denmark A (5,826) | +75.9 ± 29.2 | 0.0069 | 420.0 (146.0) |
| | Combined | +59.7 ± 7.9 | $9.9\times10^{-14}$ | |

[a]Effect size, ± standard error, represents the difference in mean values between heterozygote carriers and non-carriers of the variants after adjusting for age, sex and, for Iceland, site and statin use. [b]Calculated based on unadjusted values. [c]The Nijmegen Biomedical Study (Wetzels et al. 2007). [d]The Danish Inter99 study (Jørgensen et al. 2003). [e]The Danish Addition study (Lauritzen et al. 2000). To convert the values for non-HDL cholesterol to millimoles per liter, multiply by 0.02586

## Example 2-ALP data from ASGR-1 Knockout mice

ASGR-1 KO mice (strain B6.129S4-*ASGR-1[tm1Sau]*/SaubJxmJ) were obtained from Jackson Labs and maintained on a chow diet. Serum was collected from male and female animals after a 4hr fast and tested in an Olympus AU640 Clinical Chemistry Analyzer. Compared to wild-type mice, serum ALP is elevated in ASGR-1 knockout mice (*, p < 0.05; ****, p < 0.0001, one-way ANOVA with Dunnett test). Levels of alanine transaminase (ALT) and aspartate transaminase (AST) were not significantly different between the groups. These data are summarized in Figure 7 herein. WT=wild-type; HE=heterozygous; HO=homozygous.

## Example 3-RNAi

## Material and Methods

## siRNA Constucts

Table 3.1:

| Vendor | Vendor catalog# | Primary Gene Target | Target Sequence | SEQ ID NO: | matched control | SEQ ID NO: |
|---|---|---|---|---|---|---|
| Fisher/Ambion | S1662 | hASGR-1 | ACUUCACAGC GAGCACGGA | 32614 | ACUUCACACGC AGCACGGA | 32632 |
| GE/Dharmacon | D-011215-01 | hASGR-2 | GCCAAGGACU UUCAAGAUA | 32615 | GCCAAGGAGAA UCAAGAUA | 32633 |
| GE/Dharmacon | D-011215-03 | hASGR-2 | UGACGGAGGU CCAGGCAAU | 32616 | UGACGGAGCAG CAGGCAAU | 32634 |
| GE/Dharmacon | D-011215-04 | hASGR-2 | AGUGAUGGCU CUUGGAAAU | 32617 | AGUGAUGGGAG UUGGAAAU | 32635 |
| Fisher/Ambion | S1665 | hASGR-2 | GACUAUAGGC ACAACUACA | 32618 | GACUAUAGCGU CAACUACA | 32636 |
| Fisher/Ambion | S194296 | hASGR-2 | CUGUGUGACUG GGUCCCAA | 32619 | CUGUGUGAGAC GGUCCCAA | 32637 |
| Fisher/Ambion | S194297 | hASGR-2 | CACCUCUGGCU AACCCAUA | 32620 | CACCUCUGGGAA ACCCAUA | 32638 |
| GE/Dharmacon | D-042958-01 | mASGR-1 | GAGACAGGCUU CCAGAAUU | 32621 | GAGACAGGGAA CCAGAAUU | 32639 |
| GE/Dharmacon | D-042958-04 | mASGR-1 | UGAAGUUAGUG GAGUCGAA | 32622 | UGAAGUUACAC GAGUCGAA | 32640 |

(continued)

| Vendor | Vendor catalog# | Primary Gene Target | Target Sequence | SEQ ID NO: | matched control | SEQ ID NO: |
|---|---|---|---|---|---|---|
| Fisher/Ambion | S62656 | mASGR-1 | AGAUCACUCCA GUUUGCUA | 32623 | AGAUCACUGGU GUUUGCUA | 32641 |
| Qiagen | S10273579 6 | mASGR-1 | CCAUCAUGACA AAGGAUUA | 32624 | CCAUCAUGUGU AAGGAUUA | 32642 |
| GE/Dharmacon | D-061966-01 | mASGR-2 | GGAUGGAACU GAUUAUAGA | 32625 | GGAUGGAAGAC AUUAUAGA | 32643 |
| GE/Dharmacon | D-061966-02 | mASGR-2 | GGAAUUGGGCC UUCACUCA | 32626 | GGAAUUGGCGG UUCACUCA | 32644 |
| GE/Dharmacon | D-061966-03 | mASGR-2 | GACGGAACAUC ACCCACUA | 32627 | GACGGAACUAG ACCCACUA | 32645 |
| GE/Dharmacon | D-061966-04 | mASGR-2 | GGAUAGGUCUU ACCGACAG | 32628 | GGAUAGGUGAA ACCGACAG | 32646 |
| GE/Dharmacon | 562659 | mASGR-2 | GCAGGAUCCU AGGAUAGAA | 32629 | GCAGGAUCGAU GGAUAGAA | 32647 |
| Fisher/Ambion | S62660 | mASGR-2 | ACAUUGCUCU UUCACCUGA | 32630 | ACAUUGCUGAA UCACCUGA | 32648 |
| Fisher/Ambion | S62661 | mASGR-2 | GAAGAGUUUC GGACCCUGA | 32631 | GAAGAGUUAGC GACCCUGA | 32649 |

### Expression analysis

RNA was isolated from the HepG2, CHOs stable cell lines, or liver tissues treated with scrambled siRNA, matched control siRNA or siRNAs against hASGR-1, hASGR-2, mASGR-1 or mASGR-2 using the Qiacube and standard Qiagen RNA isolation protocol. The RNA was DNase treated using the RQ1 DNase kit (Promega). Quantitative PCR was performed according to the manufacturer's protocol on the Quantstudio 7 using the indicated primer probe set (hASGR-1: Hs01005019_m1; hASGR-2: Hs00910102_m1; mASGR-1: Mm01245581_m1, mASGR-2:Mm00431863_m1) from Applied Biosystems. 50ng RNA/well was used and normalized with 18S internal control.

### siRNA transfection

Cells were transfected with 10nM indicated scrambled siRNA, matched control siRNA or siRNAs against hASGR-1, hASGR-2, mASGR-1 or mASGR-2 siRNA for 3-4 days, using Lipofectamine RNAMAX (Thermo Scientific) following manufacturer's RNAi reverse transfection protocol. Transfection was done in 96 well Screenstar microplates (Greiner bio-one) for internalization assay as well as in 96 well clear tissue culture plates (Corning) for QPCR and Western blotting.

### Western Blotting

Cells were lysed in RIPA buffer containing inhibitors 3-4 days after siRNA transfection. Cell lysates were passed through a 21 gauge syringe five times and then centrifuged at 13000rpm at 4C for 15mins. Supernatants were collected and protein concentrations were determined. If needed, 30ug of protein was deglycosylated using the deglycosylation kit (Genzyme). 10ug-30ug of total protein was loaded in each well. The gel was transferred onto a nitrocellulose membrane and the membrane was blocked with 5% blocking buffer for 1hr at RT. Membrane was then probed with anti-mASGR-1 (1 :1000, R &D), hASGR-1 (1:1000, ProteinTech), hASGR-2 (1:1000, Abcam), anti-flag (1:5000, Sigma), anti-his (1:1000, Cell signaling) and mouse anti β-actin (1:5000, Thermo Fisher or Cell signaling) o/n at 4C. The membrane was further probed with anti-mouse and anti-rat secondary antibodies to detection the indicated bands.

### Ligand Internalization assay

CHO stable cell lines were treated with scrambled siRNA, matched control siRNA or siRNAs against hASGR-1, hASGR-2, mASGR-1 or mASGR-2 siRNA for 3-4 days and plated in 96-well plate. Biotin-GalNAc-PAA was incubated and strepavidin-Alexa488 was further added to cells. Draq5 was used to counterstain cells (for both cytoplasm and nuclei). Cells were scanned with Operetta Image System and data analyzed by Columbus.

### Animal Study

All animal housing conditions and research protocols were approved by the Amgen Institutional Animal Care and Use Committee (IACUC). Mice were housed in a specified-pathogen free, AAALAC, Intl-accredited facility in ventilated microisolators. Procedures and housing rooms are positively pressured and regulated on a 12:12 dark:light cycle. All animals received reverse-osmosis purified water ad libitum via an automatic watering system. 10-12 week old C57BL/6J animals (The Jackson Laboratory) were singly housed and were fed standard chow (2020× Teklad global soy protein-free extruded rodent diet; Harlan).

siRNAs modified for in vivo studies were formulated with Invivofectamine 3.0 (Thermo Scientific) following the manufacturer's protocol. In brief, siRNAs were pre-mixed with complex buffer (provided by manufacturer) and Invivofectamine 3.0, and then incubated at 50°C for 30 minute and further diluted by PBS before injection.

Mice were i.v. injected with buffer, indicated siRNA and matched control siRNA at 1-2 mg/kg body weight in 0.25 ml buffer at indicated time. Liver total RNA from harvested animals was processed for qPCR analysis.

Data from these studies is provided in Figures 8-17 herein.

### Example 4- Y272C mutant data

Stable pools of Chinese hamster ovary (CHO) cells expressing C-terminal FLAG epitope-tagged murine wild-type or Y272C ASGR-1 were generated by established methods using puromycin selection. Cell surface expression of ASGR-1 was confirmed by FACS using anti-FLAG antibody both during selection process and at the time of the experiment. Ligand binding was assessed by FACS using β-GalNAc-PAA-biotin (Glycotech Corporation) and streptavidin-phycoerythrin (PE). Briefly, ligand was added to 100ul cells ($1 \times 10^6$ cells) in Dulbeco's Modified Eagle Medium (DMEM) without phenol red plus 2% bovine serum albumin (BSA) and incubated on ice for 60 minutes. Cells were then washed 3x with DMEM without phenol red plus 2% BSA. Streptavidin-PE was then added at 1 μg/ml for 20 minutes on ice followed by 3 more washes in DMEM without phenol red plus 2% BSA, at which point the cells were resuspended in 0.5m1 DMEM without phenol red plus 2% BSA and 5ul of 0.1mM SyTOx Blue viability dye and analyzed on a BD LSR II (BD Biosciences). Data are presented as Median Fluorescence Intensity as shown in Table 4.1, below.

**Table 4.1**

| ASGR-1 Y272C has reduced ligand binding compared to wild-type ASGR-1 | | | | | |
|---|---|---|---|---|---|
| | β-GalNAc-PAA-biotin, ug/ml | | | | Anti-FLAG antibody |
| | 0 | 0.1 | 0.3 | | |
| Parental | 5.23 | 5.52 | 5.57 | | 7.3 |
| WT | 4.87 | 763.51 | 1394.86 | | 3959.65 |
| Y272C | 5.28 | 5.47 | 6.10 | | 973.38 |

## Example 5-generation of antibodies

### Molecular Cloning of ASGR-1 and ASGR-2 Sequences

For production of recombinant ASGR-1 and ASGR-2 vectors, cDNA sequences were synthesized, obtained from a commercial source or compiled from RNA sequencing data (Amgen). Human, mouse and rat ASGR cDNA clones were from obtained commercially (OriGene Technologies, Inc.). All other ASGR cDNAs were synthesized (Integrated DNA Technologies, Inc.). GenBank accession numbers are as follows: human ASGR-1 (NM_001671.4), human ASGR-2 (NM_080913.3), mouse ASGR-1 (BC022106.1), mouse ASGR-2 (BC011197.1), rat ASGR-1 (NM_012503), rat ASGR-2 (NM_017189), pig ASGR-1 (NM_001244458), pig ASGR-2 (XM_005669199), dog ASGR-1 (XM_546579), dog ASGR-2 (XM _003434599), cynomologus monkey ASGR-1 (XP_005582755). Since the NCBI entry for cynomologus ASGR-2 was a partial amino acid sequence (NCBI protein accession # EHH57653), the complete nucleotide sequence was compiled through the analysis of the cyno genome (genome build Macaca_fascicularis_5.0; GenBank accession number GCA_000364345.1; Washington University) and RNA sequencing data (Amgen) from cyno liver, heart and skin tissue. For transient or stable mammalian expression, cDNAs were cloned into pTTS (National Research Council of Canada), pSLX235a (SureTech) or pJiF1 (Boyce Lab, Massachusetts General Hospital, US Patent: 7,192,933). For individual recombinant protein production in mammalian cells, most sequences were tagged at their C-termini with a 6xHis purification tag. For complexes of huASGR-1 and huASGR-2, huASGR-2 was expressed without the 6xHis tag. For recombinant expression in E. coli, sequences were cloned into pET21a (Novagen, EMD Millipore). The amino acid sequences of the resultant ASGR proteins are shown in Table 1.

### Expression and Purification of Recombinant Proteins

### Generation of Stable CHO-S Cell Pools for Recombinant Protein Expression

CHO-S (Invitrogen, Carlsbad, California) cells were transfected with the pSLX235a vector encoding ASGR-1 or ASGR-2 using Lipfectamine LTX according to the manufacturer's recommendations (ThermoFisher Scientific). Stable pools were selected using 10ug/ml puromycin (single selections) or 10ug/ml puromycin and 400ug/ml hygromycin (double selections) and by culturing the cells in fresh media every 2 days. Stable pools were then used for recombinant protein production.

### Recombinant Protein Production and Purification from CHO-S Cell Stable Pools

Cells from the selected stable pools were expanded in growth medium. When sufficient cell numbers had been obtained, cultures were seeded in 2L conical flasks in a volume of 1L of growth medium at a viable cell density of $8 \times 10^5$ cells/ml. Cells were then cultured in suspension at 37°C, in 5% $CO_2$ for three days, after which the temperature was dropped to 31°C for the final 7 days of production. Centrifugation was used to pellet the cells, and the resulting supernatant was filtered to generate conditioned medium.

Individual recombinant proteins were purified via the 6xHis tag using Ni-Excel resin (GE Healthcare). Briefly, 1.4L of conditioned medium was loaded onto 3x 5ml Ni-Excel Hi-trap columns and then washed with 10 column volumes of wash buffer (25mM HEPES, pH7.6, 250mM NaCl, 1mM CaClz, 50mM imidazole). Protein was eluted from the columns with 7 column volumes of elution buffer (25mM HEPES, pH7.6, 250mM NaCl, 1mM $CaCl_2$, 400mM imidazole). The

eluted fractions were loaded onto a HiLoad Superdex 200 column via 2x 10ml injections and eluted with 25mM HEPES, pH 7.6; 150mM NaCl, 1mM $CaCl_2$. The final fractions were collected based on their expected molecular weight. The identity of the proteins in each eluted peak was confirmed by LC-TOF-MS after deglycosylation (with N-glycanase, O-glycanase and sialidase) and reduction. ASGR-1/ASGR-2 complexes were purified by pre-incubating the ASGR-1-6xHis Tag conditioned medium with ASGR-2-no 6xHis Tag conditioned medium. These conditions permitted association of both proteins giving a complex that could be purified via the standard two-step Ni-Excel/SEC method.

## Recombinant Protein Production and Purification from E. coli

E. coli codon optimized sequences were cloned into the pET21a expression plasmid. Plasmids were transformed into E.coli strain BL21(DE3) Star (ThermoFisher Scientific Inc.) and individual clones were selected using carbinicillin. For expression, cells were grown in 1L TB growth medium (supplemented with carbinicillin) in a 4L flask at 37°C with shaking. When an optical density of 2 was achieved, protein expression was induced by the addition of 1mM IPTG (final concentration). After 4 hours of induction at 37°C, the cell paste was harvested by centrifugation (recovering between 7 and 14 g cell paste/ L culture). Protein localization into the insoluble fraction was confirmed by SDS-PAGE.

Inclusion bodies were recovered from the cell paste and solubilized in 6M guanidinium containin 10mM DTT. Successful protein refolding was established by screening a matrix of 32 conditions that included a variety of buffers, pHs, denaturants, stabilizing agents and reducing agents. The refolding procedure was initiated by rapidly diluting the dissolved inclusion bodies at a ratio of 1:15 into the appropriate refold buffer, maintaining approximately 1mg of protein per condition. The samples were then incubated at 4°C for 60 hours. The resulting batches were analysed by SDS-PAGE and Ion Exchange chromatography to identify the optimal refolding conditions. For the ASGR-1 CBD (148-291), the final refold conditions were: pH 9.5, 2.5M urea, 20% glycerol, 4mM cysteine and 4mM cystamine.

## Generation of anti-ASGR Immune Responses

## Mouse Strains

Fully human antibodies to human ASGR were generated by immunizing XENOMOUSE® transgenic mice (U.S. Pat. Nos. 6,114,598; 6,162,963;6,833,268; 7,049,426; 7,064,244, which are incorporated herein by references in their entirety; Green et al., 1994, Nature Genetics 7:13-21; Mendez et al., 1997, Nature Genetics 15:146-156; Green and Jakobovitis, 1998, J. Ex. Med, 188:483-495; Kellerman and Green, Current Opinion in Biotechnology 13, 593-597,2002). Animals from the XMG2-K, XMG2-KL, XMG4-K and XMG4-KL XENOMOUSE® strains were used for all immunizations.

Mouse anti-human ASGR antibodies were generated by immunizing BALB/c, C57BL/6 and CD-1 mice (Charles River Laboratories, San Diego, California) as well as B6.12954-*ASGR-1tm1Sau*/SaubJxmJ (ASGR-1 KO mice) and C57BL6 x129 F1 mice (Jackson Laboratory, Sacramento, CA).

Fully human, heavy chain only antibodies (HCAbs) were generated by immunizing the VH4 and 8V3 strains of transgenic Harbour mice (Janssens et al. 2006, PNAS 103:15130-15135; Harbour Biologies, Rotterdam, Netherlands). Rat anti-mouse ASGR antibodies were generated using Brown Norway Rats (Charles River Laboratories, San Diego, California).

## Immunizations

Multiple immunogens and routes of immunization were used to generate anti-human ASGR immune responses. For genetic immunizations, mice were immunized 12-14 times over 6-8 weeks using the Helios Gene Gun system according to the manufacturer's instructions (BioRad, Hercules, California). Briefly, expression vectors encoding wild type human or mouse ASGR-1 (or both huASGR-1+huASGR-2, muASGR-1+muASGR-2) were coated onto gold beads (BioRad, Hercules, California) and delivered to the epidermis of a shaved mouse or rat abdomen. For cell-based immunizations, mice and rats were immunized with CHO-s cells (Invitrogen, Carlsbad, California) or 293-6E cells (National Resource Council of Canada) transiently transfected with expression vectors encoding human or mouse ASGR-1 (or both huASGR-1+huASGR-2, muASGR-1+muASGR-2). Animals were immunized with cells mixed with Alum prepared from aluminum potassium sulfate (EMD Chemicals Inc., Gibbstown, NJ) and CpG-ODN (Eurofins MWG Operon LLC, Huntsville, AL) 10 times over 6 weeks using a protocol that alternated between sub-cutaneous and intraperitoneal injections. The initial boost was comprised of $4 \times 10^6$ cells while subsequent boosts contained $2 \times 10^6$ cells. For soluble protein immunizations, mice were immunized with a variety of human ASGR recombinant proteins representing the complete extracellular domain (ECD), the carbohydrate binding domain (CBD) or the complex of ASGR-1 and ASGR-2 ECDs (Table 5.1). Animals were immunized with recombinant protein (or recombinant protein conjugated to KLH using standard methods) mixed with Alum and CpG-ODN, Complete Freund's Adjuvant (Sigma), or MPL+ Adjuvant (Sigma) 10 times over 4-6 weeks using sub-cutaneous injections. The initial boost was comprised of 10μg while subsequent

boosts contained 5-10μg. Human ASGR-1-specific serum titers were monitored by live-cell FACS analysis on an Accuri flow cytometer (BD Biosciences). Animals with the highest antigen-specific serum titers were sacrificed and used for hybridoma generation (Kohler and Milstein, 1975).

Table 5.1. Soluble, Recombinant Protein Antigens Used for Immunizations

| Recombinant Protein Immunogen | Source |
| --- | --- |
| huASGR-1 (Cat#:C428) ECD-KLH conjugate | Novoprotein |
| huASGR-1 (64-291) ECD-KLH conjugate | Amgen |
| huASGR-1 (64-291) ECD | Amgen |
| huASGR-1 (154-291) CBD | Amgen |
| huASGR-1(64-291)/huASGR-2 (61-287) ECD Complex | Amgen |
| huASGR-1(64-291)/huASGR-2 (61-287) ECD Complex-KLH conjugate | Amgen |
| muASGR-1 (63-284) | Amgen |

**Preparation of Monoclonal Antibodies**

**Hybridoma Generation**

Animals exhibiting suitable serum titers were identified and lymphocytes were obtained from spleen and/or draining lymphnodes. Pooled lymphocytes (from each immunization cohort) were dissociated from lymphoid tissue by grinding in a suitable medium (for example, Dulbecco's Modified Eagle Medium (DMEM); Invitrogen, Carlsbad, CA). B cells were selected and/or expanded using standard methods, and fused with a suitable fusion partner using techniques that were known in the art.

**Antigen Enrichment of Hybridoma Pools**

Fused hybridoma pools from each immune tissue harvest were used as a source of material for FACS-based enrichments using a variety of probes. To enrich for hybridomas expressing antibodies specific to native (full length, on-cell) human, cyno, mouse, rat, dog, or pig ASGR-1 (and native human ASGR-2) membranes were prepared from 293T cells transiently expressing the relevant ASGR cDNA construct. 24 hours after transfection using 293-fectin (ThermoFisher Scientific Inc.), cells were biotinylated with E-Z link NHS-LC-LC- Biotin according to the manufacturer's recommendation (ThermoFisher Scientific Inc.). After biotinylation, cells were homogenized with a needle and syringe to form membrane fragments and referred to as "membrane preps". The biotinylated membrane preps were then used to detect hybridomas expressing surface antibodies specific to the target of interest via standard biotin-streptavidin chemistry. To enrich for hybridomas capable of binding to the recombinant ASGR-1 ECD or CBD, soluble, 6xHis-tagged ASGR-1 proteins were used (Amgen).

To enrich hybridoma pools for the antigen of interest, they were first incubated with the appropriate membrane prep or soluble probe. For soluble forms of ASGR-1, the recombinant protein probes were added to the hybridomas and allowed to bind. Excess probe was then washed away and the antigen-specific hybridomas were identified by simultaneous detection of surface IgG (with an Alexa 488 conjugated secondary antibody (Jackson ImmunoResearch) (Gt anti-mouse Fc for wild type mouse hybridomas and Gt anti-human Fc for transgenic mouse hybridomas)) and the soluble ASGR-1 probe via its 6xHis tag (using an Amgen-derived anti-6xHis monoclonal antibody conjugated to Alexa 647 via an Alexa 647 labeling kit (ThermoFisher Scientific Inc). Hybridomas expressing surface IgG and binding antigen were detected by FACS analysis on an Accuri flow cytometer. Dual positive events were sorted as single cells into 384-well plates on a FACS Aria cell sorter (BD Biosciences). For native forms of ASGR-1, biotinylated membrane preps were prepared as described from 293T cells transiently expressing the appropriate antigen. After washing away unbound probe, dual positive hybridomas expressing cell surface IgG and binding antigen were detected using an Alexa 488 conjugated secondary antibody (to detect IgG) and streptavidin conjugated to Alexa 647 (Jackson ImmunoResearch) to detect antigen. These events were sorted as single cells into 384-well plates on a FACS Aria cell sorter. After several days of culture, the hybridoma supernatants containing monoclonal antibodies were collected and used in the screening assays described in the examples below.

## Example 6: Identification of ASGR-1 Specific Antibodies

The following Table 6.1 summarizes the approximate numbers of antibodies assayed:

**Table 6.1. Summary of the identification and selection of huASGR-1 binding, ligand blocking antibodies.**

| ASGR-1 Screen | Number of Antibodies |
|---|---|
| huASGR-1 Binders | 15731 |
| huASGR-1-Ligand Blockers (>60%) | 5306 |
| Sequences Unique huASGR-1-Ligand Blockers | 2603 (disclosed in Table 3) |
| huASGR-1-Ligand Blockers (>50%) | 172 (disclosed in Table 3) |

## Example 6-A: Initial Selection of ASGR-1 Specific Binding Antibodies

Hybridoma supernatants (monoclonal antibodies) were screened for binding to human ASGR-1 transiently expressed on Human Embryonic Kidney (HEK) 293 cells using the Cell Insight™ High Content Imaging Platform (ThermoFisher Scientific). Human ASGR-1 was transiently expressed on host HEK 293 cells by transfection using human ASGR-1 DNA, Gibco™ Opti-MEM® media and 293Fectin™ reagents following the protocol set out by the manufacturer. Transfected HEK 293 cells expressing the human ASGR-1, hybridoma supernatant or control samples, Alexa Fluor® 488 IgG Fc fragment-specific detection antibody and Hoechst 33342 stain were mixed and incubated for 3 hours at room temperature. Samples were then washed and analyzed on the CellIsight™ system. Supernatants were counter-screened against HEK 293 cells transfected with empty parental vector (referred to as mock). Analysis was done using irrelevant IgG antibody supernatant sample signal; hybridoma supernatant samples showing two times or greater signal over irrelevant IgG antibody sample were considered to be exhibiting ASGR-1-specific binding profiles and selected for further characterization. See Table 6.1.

## Example 6-B: Identification of ASGR-1 Receptor-Ligand Blocking Antibodies

ASGR-1-binding hybridoma supernatants were tested for their ability to block ASGR-1 from binding ligand. Competitive binding assays were performed on the antigen specific hybridoma supernatant samples using FACS on either HEK 293 cells transiently expressing human ASGR-1 or CHO-S cells stably expressing Human ASGR-1 as follows. HEK 293 cells or CHO-S cells expressing human ASGR-1 were mixed with the antibody sample (hybridoma supernatants specific for ASGR-1) and incubated for 1 hour at 4°C, and then washed twice. Cells with bound sample were then incubated with precomplexed $\beta$-GalNAc-PAA-Biotin (GlycoTech, Gaithersburg, Maryland)/Alexa Fluor® 647- Streptavidin for 45 minutes at 4°C. The concentration of $\beta$-GalNAc-PAA-Biotin was used at the binding EC50 concentration on the specific cell line. The concentration of Alexa Fluor® 647 Streptavidin was used at a 2:1 molar ratio to $\beta$-GalNAc-PAA-Biotin. The 7-AAD cell viability stain was then added and the cells incubated for a further 15 minutes at 4°C, washed twice and resuspended in FACS buffer. Where tolerated by cell viability, FACS buffer supplemented with 1mM Calcium Chloride was used in all steps. Samples were analyzed using a BD Accuri™ Flow Cytometer and an Intellicyt HyperCyt Autosampler. Analysis was done using irrelevant (non-ASGR-1 specific) IgG antibody supernatant control signal on both mock transfected HEK 293 cells and Human ASGR-1 transfected HEK 293 cells to determine maximum and minimum $\beta$-GalNAc-PAA-Biotin binding signal. Using these maximum and minimum binding signals, the % $\beta$-GalNAc-PAA-Biotin binding inhibition was determined. ASGR-1 antibodies having the ability to reduce ligand binding >_60% were identified (Table 6.1), and sequenced using methods available to those skilled in the art. The sequences of unique ASGR-1-specific, ligand blocking antibodies are displayed in Table 2-7 herein.

The unique ASGR-1-specific, ligand blocking antibodies were then tested for their ability to block the GalNAc ligand under more stringent conditions using a single, known antibody concentration (5ug/ml). The receptor-ligand blocking assays were performed using 293T cells transiently expressing ASGR-1 or CHOs cells that had been stably transfected with ASGR-1. ASGR-1 antibodies having the ability to reduce ligand binding ≥50% were identified. See Table 6.1.

**Example 7; Antibody Characterization Assays**

**A. ASGR-1 Species Cross Reactivity, ASGR-2 Selectivity Assays and Hepatoma (HEPG2) Binding Assays**

Human ASGR-1-specific, ligand competing antibody samples were tested for binding to ASGR-1 from other species (cynomologus monkey ASGR-1, mouse ASGR-1, rat ASGR-1, dog ASGR-1, and pig ASGR-1) as well as to human ASGR-2 in FACS binding assays at normalized antibody concentrations. For cell-based assays, HEK 293 cells expressing the appropriate antigen of interest were mixed with antibody sample or controls, incubated for 1 hour at 4°C, and then washed twice. Cells with bound antibody were then incubated with Alexa Fluor® 647 IgG Fc fragment-specific detection antibody and 7-AAD viability stain for 15 minutes at 4°C, washed once and resuspended in FACS buffer. Samples were analyzed using a BD Accuri™ Flow Cytometer and an Intellicyt HyperCyt Autosampler. As a negative control, supernatants and controls were also screened against HEK 293 cells transfected with empty parental vector. Analysis was done using irrelevant (non-ASGR-1 specific) IgG antibody supernatant sample signal; hybridoma supernatant samples showing at least two times the signal over irrelevant IgG antibody sample were considered to be exhibiting ASGR-1- species specific binding profiles. For membrane-prep binding assays, ASGR-1 species specific membrane preps were used to coat LumAvidin® microspheres (beads) and tested for binding to selected hybridoma supernatants or controls. Briefly, ASGR-1 species specific membrane preps were incubated with streptavidin-coated LumAvidin® beads for 45 minutes in the dark at room temperature and washed twice. Beads were resuspeneded in FACS buffer containing Stabilguard®. Antigen-bound beads were then incubated with normalized antibody sample for 1 hour in the dark at room temperature, washed twice, incubated with Alexa Fluor® 488 IgG Fc fragment-specific detection antibody for 15 minutes in the dark at room temperature, washed once and finally resuspended in FACS buffer. Samples were analyzed using an Intellicyt iQue™ Screener Platform. FACS buffer supplemented with 1mM Calcium Chloride was used in all steps. As a negative control, supernatants and controls were also screened against a non-ASGR-1 antigen membrane prep coated on the LumAvidin® beads. Analysis was done using irrelevant (non-ASGR-1 specific) IgG antibody supernatant sample signal; hybridoma supernatant samples showing at least two times the signal over irrelevant IgG antibody sample were considered to be exhibiting specific binding profiles. See Table 7.1.

Human ASGR-1-specific, ligand competing hybridoma supernatant samples were screened for binding to the human hepatocellular carcinoma cell line HepG2 (ATCC HB-8065) at normalized antibody concentrations. For FACS binding assays, HepG2 cells were mixed with normalized antibody samples or controls, incubated for 1 hour at 4°C, and washed twice. Cells with bound antibody were then incubated with Alexa Fluor® 647 IgG Fc fragment-specific detection antibody and 7-AAD viability stain for 15 minutes at 4°C, washed once and resuspended in FACS buffer. Samples were analyzed using a BD Accuri™ Flow Cytometer and an Intellicyt HyperCyt Autosampler. For high content imaging binding assays, HepG2 cells were mixed with normalized antibody samples or controls, incubated for 1 hour at room temperature and washed twice. Cells with bound antibody were then incubated with Alexa Fluor® 488 IgG Fc fragment-specific detection antibody and Hoechst 33342 stain for 30 minutes at room temperature, washed twice and analyzed on the CellInsight™ system. Where tolerated by cell viability, FACS buffer supplemented with 1mM Calcium Chloride was used in all steps. Analysis was done using irrelevant (non-ASGR-1 specific) IgG antibody supernatant sample signal; hybridoma supernatant samples showing two times or greater signal over irrelevant IgG antibody sample were considered to be exhibiting HepG2 ASGR-1 specific binding profiles. See Table 7.1.

Table 7.1. Summary of the binding specificities of the selected human ASGR-1 binding antibodies.

| mAb | Binding Data Summary | | | | | | | |
|---|---|---|---|---|---|---|---|---|
| | Human ASGR-1 | Cyno ASGR-1 | Mouse ASGR-1 | Rat ASGR-1 | Dog ASGR-1 | Pig ASGR-1 | HEPG2 Cells | Human ASGR-2 |
| 25A4 | Y | Y | N | Y | N | Y | Y | N |
| 26C4 | Y | Y | N | Y | N | Y | Y | N |
| 29H8 | Y | Y | N | Y | N | Y | Y | N |
| 4A2 | Y | Y | N | Y | N | Y | Y | N |
| 4H6 | Y | Y | Y | Y | N | Y | Y | N |
| 56E5 | Y | Y | N | N | N | Y | Y | N |
| 7F4 | Y | Y | N | no data | Y | Y | Y | Y |
| 7G4 | Y | Y | N | N | N | Y | Y | N |

(continued)

| mAb | Binding Data Summary | | | | | | | |
|---|---|---|---|---|---|---|---|---|
| | Human ASGR-1 | Cyno ASGR-1 | Mouse ASGR-1 | Rat ASGR-1 | Dog ASGR-1 | Pig ASGR-1 | HEPG2 Cells | Human ASGR-2 |
| 48B12 | Y | Y | N | N | N | Y | Y | N |
| 184E7 | Y | Y | Y | Y | Y | Y | Y | N |
| 194A4 | Y | Y | N | Y | Y | Y | Y | N |
| 4B1 | Y | Y | Y | Y | Y | Y | Y | N |
| 72G9 | Y | Y | Y | Y | Y | Y | Y | N |
| 190F8 | Y | Y | N | N | Y | Y | Y | N |
| 191G1 | Y | Y | N | N | Y | Y | Y | N |
| 191G10 | Y | Y | N | N | Y | Y | Y | N |
| 194C1 | Y | Y | N | N | Y | Y | Y | N |
| 197G3 | Y | Y | N | N | Y | Y | Y | N |
| 198G3 | Y | Y | N | N | Y | Y | Y | N |
| 75G3 | Y | Y | N | N | Y | Y | Y | N |
| 218G4 | Y | Y | N | N | Y | Y | Y | N |
| 193E7 | Y | Y | N | N | Y | N | Y | N |
| 198D2 | Y | Y | N | Y | N | Y | Y | N |
| 202A3 | Y | Y | N | N | Y | Y | Y | N |
| 7E11 | Y | Y | N | N | N | Y | Y | N |
| 22G5 | Y | Y | N | N | N | N | Y | N |
| 5E5 | Y | Y | N | Y | N | N | Y | N |
| 54E9 | Y | Y | N | N | Y | N | Y | N |
| 6G7 | Y | Y | N | Y | N | N | Y | Y |
| 176H4 | Y | Y | N | N | Y | Y | Y | N |
| 194C10 | Y | Y | N | N | Y | Y | Y | N |
| 12D2 | Y | Y | Y | Y | Y | Y | Y | N |

## B. Relative Binding Affinities for ASGR-Specific mAbs

To assess antibody and antigen interaction strength (relative binding affinity), ASGR-1 specific, ligand competing antibody hybridoma supernatants were tested in a limiting antigen binding assay. Titrated amounts of recombinant, soluble ASGR-1 biotinylated protein was incubated with streptavidin-coated LumAvidin Beads® for 45 minutes in the dark at room temperature and washed twice. Beads were resuspeneded in FACS buffer containing Stabilguard® and 0.05% Sodium Azide. Antigen-bound beads were then incubated with normalized hybridoma supernatant sample or controls for 18 hours in the dark at room temperature, washed twice, incubated with Alexa Fluor® 488 IgG fragment-specific detection antibody for 15 minutes in the dark at room temperature, washed once and finally resuspended in FACS buffer. Samples were analyzed using an Intellicyt iQue™ Screener Platform. FACS buffer supplemented with 1mM Calcium Chloride was used in all steps. Analysis was done using irrelevant (non-ASGR-1 specific) IgG antibody supernatant sample signal; hybridoma supernatant samples showing at least two times or greater signal over irrelevant IgG antibody sample were considered to be exhibiting ASGR-1 specific binding profiles. In this assay method, the antibody binding signal correlates with antibody affinity. Antibody binding data for a representative antigen coating concentration that fell in the linear range of the instrument signal detection is shown in Table 7.2. The degree of antibody binding to the target (ASGR-1) correlates with the measured fluorescent intensity and thus allows a relative comparison of affinities across the panel.

Table 7.2 Limited Antigen Binding Assay to Assess Relative Affinities of selected mAbs

| mAb | Soluble ASGR-1 2.5 ng/mL (FACS Geomean) | mAb | Soluble ASGR-1 2.5 ng/mL (FACS Geomean) | mAb | Soluble ASGR-1 2.5 ng/mL (FACS Geomean) | mAb | Soluble ASGR-1 2.5 ng/mL (FACS Geomean) |
|---|---|---|---|---|---|---|---|
| 25A4 | 17952 | 48B12 | 26989 | 194C1 | 16937 | 7E11 | 4662 |
| 26C4 | 12007 | 184E7 | 40198 | 197G3 | 17708 | 22G5 | 1078 |
| 29H8 | 12179 | 194A4 | 38934 | 198G3 | 25969 | 5E5 | 3278 |
| 4A2 | 16604 | 4B1 | 10060 | 75G3 | 35840 | 54E9 | 6487 |
| 4H6 | 2990 | 72G9 | 34014 | 218G4 | 15105 | 6G7 | 2290 |
| 56E5 | 22648 | 190F8 | 13899 | 193E7 | 18315 | 176H4 | 29444 |
| 7F4 | 4910 | 191G1 | 9546 | 198D2 | 1872 | 194C10 | 21854 |
| 7G4 | 6795 | 191G10 | 24154 | 202A3 | 2152 | 12D2 | 105 |

## C. pH and Calcium Sensitivity

This Example characterizes ASGR-1 antibodies based on the effect of pH and/or calcium on their ability to bind the target. For this example, a label-free, kinetic antibody-ASGR-1 binding assay was employed to assess the sensitivity of the antibodies to changes in pH and calcium. Briefly, the ASGR-1-specific, ligand-competing antibodies were first immobilized and then allowed to bind recombinant, soluble huASGR-1 under physiological conditions (ie. pH 7.4, 1mM CaCl2). The amount of binding was determined and set to 100%. In order to determine if the antibody-ASGR-1 interaction was sensitive to changes in pH or Ca, the assay buffer was then changed to conditions lacking calcium, a reduced pH (pH 5.6) or both lacking calcium and reduced pH (pH 5.6), and dissociation of ASGR-1 from the mAbs monitored. The amount of ASGR-1 remaining bound under each condition was assessed and expressed as a percent of the starting signal. If a >10% difference in ASGR-1 binding signal was calculated (when compared to that measured under physiological conditions), a particular antibody was classified as being sensitive to that condition. Using this method, the selected antibodies were classified into 5 categories:

1. affected by the removal of calcium
2. unaffected by the removal of calcium or drop in pH
3. affected when both calcium is removed and pH is dropped
4. affected by calcium removal, pH drop and both combined
5. affected by the drop in pH

The relative dissociation of ASGR-1 from antibodies was measured using a label-free assay on an OctetHTX instrument (Fortebio). Antibody samples were captured on anti-HuFc kinetic biosensors (ForteBio cat # 18-5064) at 5 ug/mL in assay buffer (10 mM Tris, 0.1 % Triton, 150 mM NaCl, 1 mg/mL BSA, 1 mM CaCl2, pH7.4) for three minutes. A one minute baseline stabilization step was performed in assay buffer. Soluble ASGR-1 (Amgen) at 6 ug/ml in assay buffer was added and association to the antibodies was monitored for two minutes. Subsequent dissociation of ASGR-1 from the antibodies was performed by incubating the ASGR-1-mAb complexes for 10 minutes under each of the following conditions:

| pH 7.4 + calcium | 10mM Tris, 0.1%Triton, 150mMNaCl, 1mg/mL BSA, pH7.4, 1mM CaCl2 |
|---|---|
| pH 7.4 - calcium | 10mM Tris, 0.1%Triton, 150mMNaCl, 1mg/mL BSA, pH7.4 |
| pH 5.6 + calcium | 10mM Tris, 0.1%Triton, 150mMNaCl, 1mg/mL BSA, pH5.6, 1mM CaCl2 |
| pH 5.6 - calcium | 10mM Tris, 0.1%Triton, 150mMNaCl, 1mg/mL BSA, pH5.6 |

The binding signal at the end of the 2 minute association phase for each dissociation experiment was set to 100% and used to represent the maximal level of ASGR-1 binding. After 1 minute of dissociation, the percentage of ASGR-1 remaining bound was calculated. The lower the percent remaining at a given time point indicates increased levels of dissociation in response to the test conditions (ie. different pH and/or calcium concentrations). The change in the percentage of ASGR-1 remaining bound in response to each test condition relative to the percent remaining in the control conditions (ie. pH 7.4 + calcium) was determined. Cut-offs for an antibody to be categorized as being sensitive to a particular condition were set to >10% (ie. if >10% of the ASGR-1 dissociates from the antibody under a particular test condition compared to control condition, it was deemed sensitive to that condition). The analysis was done using the 1 minute dissociation time point (except for mAb 149A1 which was binned based on the 4 minute dissociation time point). Using this analysis, the ASGR-1-binding, receptor-ligand blocking antibodies were separated into groups according to their dissociation profiles in response to pH and calcium (Table 7.3). Antibodies belonging to each category were observed.

Table 7.3. pH and Calcium Sensitivity of ASGR-1-mAb Interactions

| pH and Calcium Sensitivity Determination (% Change Compared to pH 7.4 + Calcium) | | | | | | | |
|---|---|---|---|---|---|---|---|
| mAb | pH 7.4 minus Calcium | pH 5.6 plus Calcium | pH 5.6 minus Calcium | Calcium sensitive | pH sensitive | pH and calcium sensitive | pH bin |
| 10G6 | 7% | 4% | 15% | N | N | Y | 3 |
| 148E10 | 7% | 19% | 33% | N | Y | Y | 5 |
| 154F4 | 10% | 41% | 67% | N | Y | Y | 5 |
| 159H8 | 6% | 10% | 26% | N | Y | Y | 5 |
| 160B12 | 6% | 8% | 22% | N | N | Y | 3 |
| 175D10 | 4% | -3% | 2% | N | N | N | 2 |
| 177D2 | 3% | 2% | 10% | N | N | Y | 3 |
| 25A4 | 2% | -3% | -1% | N | N | N | 2 |
| 26C4 | 3% | 2% | 2% | N | N | N | 2 |
| 27E7 | 20% | 35% | 46% | Y | Y | Y | 4 |
| 29E2 | 5% | 25% | 38% | N | Y | Y | 5 |
| 29H8 | 2% | -2% | 2% | N | N | N | 2 |
| 31D12 | 10% | 27% | 34% | Y | Y | Y | 4 |
| 32D6 | 26% | 33% | 55% | Y | Y | Y | 4 |
| 45B4 | 4% | 10% | 23% | N | Y | Y | 5 |
| 49F10 | 4% | -2% | 8% | N | N | N | 2 |
| 4A2 | 1% | -3% | 1% | N | N | N | 2 |
| 4B3 | 12% | 33% | 45% | Y | Y | Y | 4 |
| 4H6 | 5% | -1% | 2% | N | N | N | 2 |
| 50D4 | 6% | 0% | 9% | N | N | N | 2 |
| 50G9 | 37% | 62% | 44% | Y | Y | Y | 4 |
| 51E9 | 3% | -5% | 2% | N | N | N | 2 |
| 52G11 | 15% | 1% | 13% | Y | N | Y | 1 |
| 52H1 | 5% | -1% | 10% | N | N | N | 2 |
| 53F2 | 15% | 1% | 13% | Y | N | Y | 1 |
| 53F7 | 9% | 3% | 13% | N | N | Y | 3 |
| 55B1 | 5% | -2% | 4% | N | N | N | 2 |

(continued)

| pH and Calcium Sensitivity Determination (% Change Compared to pH 7.4 + Calcium) | | | | | | | |
|---|---|---|---|---|---|---|---|
| mAb | pH 7.4 minus Calcium | pH 5.6 plus Calcium | pH 5.6 minus Calcium | Calcium sensitive | pH sensitive | pH and calcium sensitive | pH bin |
| 56E5 | 1% | -6% | -1% | N | N | N | 2 |
| 57A7 | 13% | 13% | 29% | Y | Y | Y | 4 |
| 58G11 | 38% | 12% | 51% | Y | Y | Y | 4 |
| 59F2 | 48% | 52% | 74% | Y | Y | Y | 4 |
| 5E5 | 7% | 18% | 42% | N | Y | Y | 5 |
| 60D2 | 20% | 42% | 49% | Y | Y | Y | 4 |
| 60E8 | 3% | 11% | 18% | N | Y | Y | 5 |
| 63A10 | 8% | 3% | 47% | N | N | Y | 3 |
| 63G7 | 20% | 15% | 59% | Y | Y | Y | 4 |
| 64B12 | 6% | 6% | 7% | N | N | N | 2 |
| 65F10 | 25% | 18% | 37% | Y | Y | Y | 4 |
| 68G6 | 22% | 39% | 47% | Y | Y | Y | 4 |
| 6D9 | 14% | 25% | 42% | Y | Y | Y | 4 |
| 6G6 | 1% | -3% | 0% | N | N | N | 2 |
| 70D1 | 17% | 12% | 29% | Y | Y | Y | 4 |
| 7E11 | 9% | 5% | 14% | N | N | Y | 3 |
| 7F4 | 4% | 6% | 9% | N | N | N | 2 |
| 7G4 | 2% | 1% | 7% | N | N | N | 2 |
| 9G9 | 25% | 38% | 55% | Y | Y | Y | 4 |
| 65E9 | 22% | 30% | 35% | Y | Y | Y | 4 |
| 72B4 | 32% | 26% | 43% | Y | Y | Y | 4 |
| 147D10 | 13% | 4% | 11% | Y | N | Y | 1 |
| 149D11 | 11% | 3% | 11% | Y | N | Y | 1 |
| 149F8 | 1% | -8% | -1% | N | N | N | 2 |
| 22G5 | 40% | 35% | No Data | Y | Y | No Data | 4* |
| 48B12 | 4% | -6% | 0% | N | N | N | 2 |
| 52H2 | 26% | 11% | 32% | Y | Y | Y | 4 |
| 6G7 | 8% | 4% | 16% | N | N | Y | 3 |
| 64G12 | 24% | 10% | 24% | Y | N | Y | 1 |
| 72F5 | 64% | 20% | 30% | Y | Y | Y | 4 |
| 147E9 | 5% | -4% | 20% | N | N | Y | 3 |
| 184E7 | 1% | -9% | -3% | N | N | N | 2 |
| 194A4 | -1% | -7% | -3% | N | N | N | 2 |
| 208A2 | -4% | -10% | -5% | N | N | N | 2 |
| 210G10 | -3% | -10% | -5% | N | N | N | 2 |

(continued)

| pH and Calcium Sensitivity Determination (% Change Compared to pH 7.4 + Calcium) | | | | | | | |
|---|---|---|---|---|---|---|---|
| mAb | pH 7.4 minus Calcium | pH 5.6 plus Calcium | pH 5.6 minus Calcium | Calcium sensitive | pH sensitive | pH and calcium sensitive | pH bin |
| 4B1 | 6% | -5% | -2% | N | N | N | 2 |
| 62H10 | 13% | -2% | 14% | Y | N | Y | 1 |
| 72G9 | 1% | -7% | -1% | N | N | N | 2 |
| 148H10 | 45% | 10% | 47% | Y | N | Y | 1 |
| 173C11 | 17% | 0% | 29% | Y | N | Y | 1 |
| 179C2 | 25% | 0% | 45% | Y | N | Y | 1 |
| 47C1 | 13% | -1% | 10% | Y | N | Y | 1 |
| 49C1 | 72% | 23% | 64% | Y | Y | Y | 4 |
| 60C12 | 14% | -3% | 12% | Y | N | Y | 1 |
| 60G2 | 36% | 7% | 31% | Y | N | Y | 1 |
| 65D5 | 34% | 7% | 61% | Y | N | Y | 1 |
| 66H11 | 81% | 36% | 52% | Y | Y | Y | 4 |
| 73G1 | 100% | 33% | 62% | Y | Y | Y | 4 |
| 51E3 | 65% | 16% | 42% | Y | Y | Y | 4 |
| 53E8 | 68% | 20% | 64% | Y | Y | Y | 4 |
| 54E9 | 79% | 24% | 75% | Y | Y | Y | 4 |
| 56E3 | 75% | 21% | 16% | Y | Y | Y | 4 |
| 190C11 | -1% | -6% | -6% | N | N | N | 2 |
| 190E6 | -1% | -12% | -6% | N | N | N | 2 |
| 190F12 | -1% | -6% | -6% | N | N | N | 2 |
| 190F8 | -1% | -5% | -5% | N | N | N | 2 |
| 190G11 | -2% | -8% | -5% | N | N | N | 2 |
| 190H9 | -1% | -6% | -7% | N | N | N | 2 |
| 191A10 | 0% | -5% | -5% | N | N | N | 2 |
| 191G1 | -10% | -15% | -11% | N | N | N | 2 |
| 191G10 | 0% | -5% | -5% | N | N | N | 2 |
| 191G12 | -2% | -5% | -6% | N | N | N | 2 |
| 192C10 | -1% | -6% | -6% | N | N | N | 2 |
| 192C8 | -9% | -14% | -14% | N | N | N | 2 |
| 192E4 | -2% | -9% | -8% | N | N | N | 2 |
| 192G6 | -1% | -6% | -6% | N | N | N | 2 |
| 192G8 | -1% | -5% | -6% | N | N | N | 2 |
| 192H10 | 0% | -5% | -4% | N | N | N | 2 |
| 193C7 | -1% | -8% | -8% | N | N | N | 2 |
| 194B7 | 1% | -4% | -4% | N | N | N | 2 |

(continued)

| pH and Calcium Sensitivity Determination (% Change Compared to pH 7.4 + Calcium) | | | | | | | |
|---|---|---|---|---|---|---|---|
| mAb | pH 7.4 minus Calcium | pH 5.6 plus Calcium | pH 5.6 minus Calcium | Calcium sensitive | pH sensitive | pH and calcium sensitive | pH bin |
| 194C1 | -7% | -12% | -8% | N | N | N | 2 |
| 196C7 | -8% | -12% | -12% | N | N | N | 2 |
| 197B6 | -1% | -8% | -7% | N | N | N | 2 |
| 197E11 | -1% | -5% | -4% | N | N | N | 2 |
| 197F2 | 0% | -6% | -6% | N | N | N | 2 |
| 197G3 | 2% | -3% | -3% | N | N | N | 2 |
| 198G3 | -1% | -4% | -4% | N | N | N | 2 |
| 213B3 | -1% | -7% | -3% | N | N | N | 2 |
| 219H1 | 2% | -3% | 1% | N | N | N | 2 |
| 74C8 | 1% | -7% | -3% | N | N | N | 2 |
| 74G6 | 1% | -9% | -4% | N | N | N | 2 |
| 75G3 | -1% | -1% | 2% | N | N | N | 2 |
| 74B2 | 8% | -9% | -5% | N | N | N | 2 |
| 74H7 | 1% | -2% | 1% | N | N | N | 2 |
| 85F7 | 2% | -2% | 2% | N | N | N | 2 |
| 198B9 | 3% | 2% | 11% | N | N | Y | 3 |
| 199A7 | 1% | 1% | 10% | N | N | Y | 3 |
| 218G4 | 1% | -4% | 0% | N | N | N | 2 |
| 146A8 | 2% | -9% | 25% | N | N | Y | 3 |
| 146B6 | 2% | -5% | 13% | N | N | Y | 3 |
| 149A1 | 2% | -7% | 9% | N | N | Y | 3* |
| 172B12 | -14% | -27% | -13% | N | N | N | 2 |
| 172C3 | -9% | -26% | 0% | N | N | N | 2 |
| 193E7 | -9% | -9% | -4% | N | N | N | 2 |
| 199E3 | -5% | -4% | -4% | N | N | N | 2 |
| 226F9 | 100% | 51% | 77% | Y | Y | Y | 4 |
| 227C1 | 100% | 54% | 73% | Y | Y | Y | 4 |
| 227F2 | 80% | 50% | 100% | Y | Y | Y | 4 |
| 65C12 | 13% | 0% | 23% | Y | N | Y | 1 |
| 176H4 | 2% | -4% | 26% | N | N | Y | 3 |
| 194C10 | 2% | 10% | 16% | N | Y | Y | 5 |
| 191E10 | -1% | -9% | -9% | N | N | N | 2 |
| 196F4 | -8% | -5% | -6% | N | N | N | 2 |
| 198D2 | -8% | -30% | -28% | N | N | N | 2 |
| 202A3 | -21% | -22% | -23% | N | N | N | 2 |

(continued)

| pH and Calcium Sensitivity Determination (% Change Compared to pH 7.4 + Calcium) | | | | | | | |
|---|---|---|---|---|---|---|---|
| mAb | pH 7.4 minus Calcium | pH 5.6 plus Calcium | pH 5.6 minus Calcium | Calcium sensitive | pH sensitive | pH and calcium sensitive | pH bin |
| 204G6 | -5% | -11% | -10% | N | N | N | 2 |
| 224G1 | 77% | 41% | 65% | Y | Y | Y | 4 |
| 52D10 | 21% | 3% | 45% | Y | N | Y | 1 |
| 64E2 | 48% | 29% | 49% | Y | Y | Y | 4 |
| *No actual data; bin predicted on the totality of information regarding the antibody. | | | | | | | |

## D. Relative Epitope Binning/Profiling

A common way to characterize epitopes is through competition experiments. Antibodies that compete with each other can be thought of as binding the same or overlapping site on the target. This example describes a method of determining competition for binding to hASGR-1 and the results of the method when applied to a number of antibodies described herein.

Binning experiments can be conducted in a number of ways, and the method employed may have an effect on the assay results. Common to these methods is that ASGR-1 is typically bound by one reference antibody and probed by another. If the reference antibody prevents the binding of the probe antibody then the antibodies are said to be in the same bin. The order in which the antibodies are employed is important. If antibody A is employed as the reference antibody and blocks the binding of antibody B the converse is not always true: antibody B used as the reference antibody will not necessarily block antibody A. There are a number of factors in play here: the binding of an antibody can cause conformational changes in the target which prevent the binding of the second antibody, or epitopes which overlap but do not completely occlude each other may allow for the second antibody to still have enough high-affinity interactions with the target to allow binding. In general, if competition is observed in either order the antibodies are said to bin together, and if both antibodies can block each other then it is likely that the epitopes overlap more completely.

For this example, a modified antibody-antibody competition assay was used to determine the relative epitope binning profiles of the ASGR-1 specific, ligand blocking antibodies in a high throughput manner. Briefly, individual antibodies were tested for their ability to compete for binding with a panel of reference antibodies chosen based on their different binding characterstics (eg. species cross reactivity, HEPG2 binding, etc.) and primary sequences. The pattern of competition/binding of each test antibody with the reference antibody panel was then determined and compared to those produced from the other test antibodies. The degree of correlation between the individual test antibody competition/binding profiles was then compared. Antibodies that showed similar competition/binding profiles were binned (grouped) together (eg. Binning Profile A, B, etc.).

Biotinylated recombinant soluble human ASGR-1 protein was coupled to streptavidin coated, uniquely barcoded LumAvidin Beads® (LumAvidin Microspheres, Cat#:L101-LXXX-01; Luminex Corp., Austin, Texas, U.S.A.) for 45 minutes in the dark at room temperature and washed twice. The reference antibody hybridoma supernatant samples were incubated with the antigen-coated beads for 1 hour in the dark at room temperature and washed three times. Beads were resuspended in FACS buffer containing Stabilguard®. The antigen-coated, reference antibody-bound beads were pooled and then divided into individual sample wells containing a normalized (2.5ug/ml) test antibody (hybridoma supernatant) sample (or negative control), incubated for 1 hour in the dark at room temperature and washed twice. The samples were then incubated with Alexa Fluor® 488 IgG fragment-specific detection antibody for 15 minutes in the dark at room temperature, washed once and resuspended in FACS buffer. FACS buffer supplemented with 1mM Calcium Chloride was used in all steps. Samples were analyzed using an Intellicyt iQue™ Screener Platform.

To determine the antibody competition/binding profiles of the individual test antibodies, the reference-only antibody binding signal was subtracted from the reference plus test antibody signal for each competition/binding reaction (ie. across the entire reference antibody set). An individual antibody binding profile was defined as the collection of net binding values for each competition/binding reaction. The degree of similarity between individual profiles was then assessed by calculating the coefficient of determination between each of the test antibody profiles. Test antibodies showing high degrees of similarity ($R^2 \geq 0.8$) to each other were then grouped into common binning profiles. Separate binning profiles were only defined if there were two or more samples with a high degree of correlation. If individual unique antibody binning profiles were observed (ie. they displayed a low degree of similarity to other test antibody binding profiles), the bin was classified as unknown. Using this method, the ASGR-1-binding, receptor-ligand blocking antibodies were sub-divided into 14 unique binning profiles (A, B, C, D, E, L, M, N, O, P, Q, R, T and unknown) (Table 7.4). Antibodies that displayed a unique binning profile (as defined above) but shared a relatively high degree of similarity to another profile ($R^2$ = 0.6-0.8) were categorized as a sub-bin (ie. A.1, A.2, etc.) of that profile.

Table 7.4 Relative Epitope Binning/Profiling of ASGR-1 Specific Receptor-Ligand Blocking mAbs

| mAb | Epitope BIN | mAb | Epitope BIN | mAb | Epitope BIN | mAb | Epitope BIN |
|---|---|---|---|---|---|---|---|
| 10G6 | A | 52H1 | A | 9C11 | A.3 | 60G2 | E |
| 11E2 | A | 53F2 | A | 12B12 | B | 65D5 | E |
| 11F5 | A | 53F7 | A | 147D10 | B | 66H11 | E |
| 12E9 | A | 55B1 | A | 149D11 | B | 71A6 | E |

| 12F11 | A | 56E5 | A | 149F8 | B | 73G1 | E |
|-------|---|------|---|-------|---|------|---|
| 12F12 | A | 57A7 | A | 151B9 | B | 49C5 | E.1 |
| 13F6 | A | 58G11 | A | 175F4 | B | 49D10 | E.1 |
| 148E10 | A | 59F2 | A | 22G5 | B | 51E3 | E.1 |
| 154F4 | A | 5E5 | A | 48B12 | B | 51F4 | E.1 |
| 159H8 | A | 60D2 | A | 52H2 | B | 53E8 | E.1 |
| 160B12 | A | 60E8 | A | 6G7 | B | 54E9 | E.1 |
| 175D10 | A | 63A10 | A | 7G2 | B | 56E3 | E.1 |
| 177D2 | A | 63G7 | A | 64G12 | B.1 | 56G1 | E.1 |
| 25A4 | A | 64B12 | A | 72F5 | B.1 | 190C11 | L |
| 25D12 | A | 65F10 | A | 147E9 | C | 190E6 | L |
| 26C4 | A | 68G6 | A | 184E7 | C | 190F12 | L |
| 27E7 | A | 6A6 | A | 194A4 | C | 190F8 | L |
| 28H2 | A | 6D4 | A | 208A2 | C | 190G11 | L |
| 29E2 | A | 6D9 | A | 210G10 | C | 190H9 | L |
| 29E6 | A | 6G6 | A | 4B1 | C | 191A10 | L |
| 29H8 | A | 70D1 | A | 60E12 | C | 191G1 | L |
| 31D12 | A | 7A10 | A | 61A1 | C | 191G10 | L |
| 32D6 | A | 7C3 | A | 62H10 | C | 191G12 | L |
| 3G7 | A | 7E11 | A | 63H8 | C | 192C10 | L |
| 45B4 | A | 7F4 | A | 72G9 | C | 192C8 | L |
| 49F10 | A | 7F8 | A | 8D8 | D.1 | 192E4 | L |
| 4A2 | A | 7G4 | A | 12D2 | E | 192G6 | L |
| 4B3 | A | 8D12 | A | 148H10 | E | 192G8 | L |
| 4H6 | A | 9F12 | A | 173C11 | E | 192H10 | L |
| 50D4 | A | 9G9 | A | 179C2 | E | 193C7 | L |
| 50G9 | A | 65E9 | A.1 | 47C1 | E | 194B7 | L |
| 51E9 | A | 72B4 | A.1 | 49C1 | E | 194C1 | L |
| 52G11 | A | 7H7 | A.2 | 60C12 | E | 196C7 | L |
| 197B6 | L | 197F2 | L | 198G3 | L | 219H1 | L |
| 197E11 | L | 197G3 | L | 213B3 | L | 74C8 | L |
| 74G6 | L | 74H7 | M.1 | 218G4 | O | 172B12 | Q |
| 75G3 | M | 85F7 | M.1 | 146A8 | P | 172C3 | Q |
| 89A11 | M | 198B9 | N | 146B6 | P | 193E7 | Q |
| 74B2 | M.1 | 199A7 | N | 149A1 | P | 199E3 | Q |
| 226F9 | Q | 227F2 | Q | 176H4 | R | | |
| 227C1 | Q | 65C12 | Q | 194C10 | T | | |

**E. Epitope Mapping - Arginine/Glutamic Acid Mutational Profiling**

This Example characterizes ASGR-1 antibodies based on the effect of mutagenesis of ASGR-1 on their ability to bind the target. Previous data indicated that the ASGR-1 CBD is primarily responsible for antibody binding for the panel of antibodies. As such, only the ASGR-1 CBD was considered structurally in the context of the full length ASGR-1 in the design of mutation sites.

Arginine/Glutamic acid mutational mapping was used to characterize epitopes bound by human ASGR-1-specific, ligand blocking antibodies. Briefly, 144 individual point mutations were made across the CBD domain of human ASGR-1 protein (SEQ ID NO:5) starting at position 148. Ninety-one constructs, representing surface residues (modelled using the ASGR-1 crystal structure in the PyMOL Molecular Graphics System (Version 1.8; Schrödinger, LLC.)) and therefore potentially accessible for antibody binding, were selected for these assays. Mutant hASGR-1 variants were constructed such that non-arginine residues were changed to arginine and where wild type arginine residues were mutated to glutamic acid. Each mutant hASGR-1 sequence was then cloned into a mammalian expression vector and used to transiently transfect CHOs cells. The ability of human ASGR-1-specific, ligand competing antibodies to bind to the mutant hASGR-1 proteins was assessed by FACS as described above.

Antibodies were tested for binding to the individual mutant and wild type ASGR-1 constructs using normalized antibody concentrations (5ug/ml). CHO-S cells transiently expressing the appropriate mutated or non-mutated antigen of interest were mixed with antibody sample or controls, incubated for 1 hour at 4°C, and then washed twice. Cells with bound antibody were then incubated with Alexa Fluor® 647 IgG Fc fragment-specific detection antibody and 7-AAD viability stain for 15 minutes at 4°C, washed once and resuspended in FACS buffer. Samples were analyzed using a BD Accuri™ Flow Cytometer and an Intellicyt HyperCyt Autosampler. As a negative control, supernatants and controls were also screened against CHO-S cells transfected with empty parental vector (referred to as mock). In order to exclude mutants that were poorly expressed or produced mis-folded antigen, only constructs that yeilded a binding data average of at least 25% or greater compared to the average binding observed on wildtype hASGR-1 was used for further analysis. Because mutant hASGR-1 expression levels varied relative to each other, sample binding data for each construct was normalized for expression by dividing the binding data from an antibody not affected by the mutations (e.g., 65C12) by the binding values of each test antibody on a given mutant construct. Also, because the antibody binding affinities varied amongst the samples, the expression corrected data (above) was further normalized by comparing test antibody binding on each mutant construct to wild type hASGR-1. Identification of specific mutations that affected test antibody binding was performed by an interquartile range (IQR) analysis to determine statistical outliers. A mutation was identified as a "hit" if the calculated values were ≥ 3x the IQR (above the 3rd quartile/upper fence) for a given mutant construct. Although IQR analysis was used here to determine signifance and identify hits, one skilled in the art will recognize that a number of methods could be employed in order to normalize the data (eg. using epitope-tagged constructs or other ASGR-1-binding antibodies directed against non-CBD epitopes). Any statistically significant reduction in antibody binding signal to a mutant construct (compared to that determined for binding to wild type ASGR-1) determined by these methods could be used for hit identification.

For illustrative purposes, Table 7.5 shows the IQR analysis with a single mutant construct (i.e., H203).

Table 7.5. IQR analysis (representative data for construct H203)

| mAb | FACS Binding Geomean | | Expression Normalization to mAb 65C12 | | Antibody Binding Normalization to wt ASGR1 Binding | | > Q3 + 3xIQR Gating | |
|---|---|---|---|---|---|---|---|---|
| | wt ASGR1 | H203 | wt ASGR1 | H203 | wt ASGR1 | H203 | wt ASGR1 | H203 |
| 4A2 | 41104 | 18946 | 1.3644 | 1.3597 | 1.0000 | 0.9966 | 1.0000 | 0.9966 |
| 7E11 | 45453 | 14714 | 1.2338 | 1.7509 | 1.0000 | 1.4191 | 1.0000 | 1.4191 |
| 56E5 | 42617 | 20345 | 1.3159 | 1.2662 | 1.0000 | 0.9622 | 1.0000 | 0.9622 |
| 7G4 | 48526 | 18542 | 1.1557 | 1.3893 | 1.0000 | 1.2022 | 1.0000 | 1.2022 |
| 53F7 | 43474 | 18081 | 1.2900 | 1.4248 | 1.0000 | 1.1045 | 1.0000 | 1.1045 |
| 10G6 | 43059 | 18213 | 1.3024 | 1.4145 | 1.0000 | 1.0860 | 1.0000 | 1.0860 |
| 26C4 | 45991 | 13484 | 1.2194 | 1.9105 | 1.0000 | 1.5668 | 1.0000 | 1.5668 |
| 6G6 | 47628 | 20505 | 1.1775 | 1.2564 | 1.0000 | 1.0670 | 1.0000 | 1.0670 |
| 29H8 | 40927 | 13217 | 1.3702 | 1.9491 | 1.0000 | 1.4225 | 1.0000 | 1.4225 |
| 25A4 | 55579 | 20036 | 1.0090 | 1.2858 | 1.0000 | 1.2743 | 1.0000 | 1.2743 |
| 32D6 | 36128 | 13465 | 1.5522 | 1.9132 | 1.0000 | 1.2325 | 1.0000 | 1.2325 |
| 198D2 | 16882 | 7138 | 3.3219 | 3.6090 | 1.0000 | 1.0864 | 1.0000 | 1.0864 |
| 4B3 | 35561 | 1696 | 1.5770 | 15.1900 | 1.0000 | 9.6323 | 1.0000 | 9.6323 |
| 50G9 | 37326 | 1506 | 1.5024 | 17.1095 | 1.0000 | 11.3879 | 1.0000 | 11.3879 |
| 60D2 | 29631 | 1368 | 1.8926 | 18.8256 | 1.0000 | 9.9467 | 1.0000 | 9.9467 |
| 59F2 | 27915 | 1346 | 2.0089 | 19.1372 | 1.0000 | 9.5260 | 1.0000 | 9.5260 |
| 60E8 | 38653 | 1518 | 1.4509 | 16.9692 | 1.0000 | 11.6960 | 1.0000 | 11.6960 |

| | | | | | | | | |
|---|---|---|---|---|---|---|---|---|
| 65E9 | 29613 | 1471 | 1.8938 | 17.5097 | 1.0000 | 9.2460 | 1.0000 | 9.2460 |
| 5E5 | 40651 | 12616 | 1.3796 | 2.0420 | 1.0000 | 1.4802 | 1.0000 | 1.4802 |
| 29E2 | 25781 | 15058 | 2.1752 | 1.7108 | 1.0000 | 0.7865 | 1.0000 | 0.7865 |
| 45B4 | 30350 | 14012 | 1.8478 | 1.8385 | 1.0000 | 0.9950 | 1.0000 | 0.9950 |
| 6G7 | 38643 | 15089 | 1.4512 | 1.7073 | 1.0000 | 1.1764 | 1.0000 | 1.1764 |
| 72F5 | 27993 | 10499 | 2.0034 | 2.4537 | 1.0000 | 1.2248 | 1.0000 | 1.2248 |
| 22G5 | 45048 | 15060 | 1.2449 | 1.7105 | 1.0000 | 1.3740 | 1.0000 | 1.3740 |
| 48B12 | 52493 | 20467 | 1.0683 | 1.2587 | 1.0000 | 1.1782 | 1.0000 | 1.1782 |
| 151B9 | 23527 | 9738 | 2.3837 | 2.6454 | 1.0000 | 1.1098 | 1.0000 | 1.1098 |
| 52H2 | 47957 | 18609 | 1.1694 | 1.3843 | 1.0000 | 1.1838 | 1.0000 | 1.1838 |
| 149D11 | 23601 | 8866 | 2.3761 | 2.9055 | 1.0000 | 1.2228 | 1.0000 | 1.2228 |
| 175F4 | 33619 | 14804 | 1.6681 | 1.7401 | 1.0000 | 1.0432 | 1.0000 | 1.0432 |
| 147E9 | 40166 | 21513 | 1.3962 | 1.1975 | 1.0000 | 0.8577 | 1.0000 | 0.8577 |
| 61A1 | 39965 | 20142 | 1.4032 | 1.2790 | 1.0000 | 0.9115 | 1.0000 | 0.9115 |
| 184E7 | 42704 | 18354 | 1.3132 | 1.4036 | 1.0000 | 1.0688 | 1.0000 | 1.0688 |
| 72G9 | 36507 | 18778 | 1.5361 | 1.3719 | 1.0000 | 0.8931 | 1.0000 | 0.8931 |
| 194A4 | 16291 | 12149 | 3.4424 | 2.1204 | 1.0000 | 0.6160 | 1.0000 | 0.6160 |
| 60C12 | 31286 | 19812 | 1.7925 | 1.3003 | 1.0000 | 0.7254 | 1.0000 | 0.7254 |
| 173C11 | 28526 | 13861 | 1.9659 | 1.8586 | 1.0000 | 0.9454 | 1.0000 | 0.9454 |
| 56E3 | 33876 | 20425 | 1.6555 | 1.2613 | 1.0000 | 0.7619 | 1.0000 | 0.7619 |
| 54E9 | 38589 | 15344 | 1.4533 | 1.6789 | 1.0000 | 1.1552 | 1.0000 | 1.1552 |
| 65D5 | 41007 | 20291 | 1.3676 | 1.2696 | 1.0000 | 0.9283 | 1.0000 | 0.9283 |
| 190F8 | 36503 | 15073 | 1.5363 | 1.7091 | 1.0000 | 1.1125 | 1.0000 | 1.1125 |
| 198G3 | 21467 | 13143 | 2.6124 | 1.9600 | 1.0000 | 0.7503 | 1.0000 | 0.7503 |
| 191G10 | 33829 | 17045 | 1.6578 | 1.5114 | 1.0000 | 0.9117 | 1.0000 | 0.9117 |
| 202A3 | 24848 | 12497 | 2.2570 | 2.0614 | 1.0000 | 0.9134 | 1.0000 | 0.9134 |
| 194C1 | 20860 | 11044 | 2.6884 | 2.3325 | 1.0000 | 0.8676 | 1.0000 | 0.8676 |
| 176H4 | 33506 | 10237 | 1.6737 | 2.5166 | 1.0000 | 1.5036 | 1.0000 | 1.5036 |
| 197G3 | 13308 | 3503 | 4.2141 | 7.3547 | 1.0000 | 1.7453 | 1.0000 | 1.7453 |
| 191G1 | 25298 | 10876 | 2.2168 | 2.3687 | 1.0000 | 1.0685 | 1.0000 | 1.0685 |
| 213B3 | 15070 | 12846 | 3.7212 | 2.0054 | 1.0000 | 0.5389 | 1.0000 | 0.5389 |
| 218G4 | 12212 | 7933 | 4.5923 | 3.2472 | 1.0000 | 0.7071 | 1.0000 | 0.7071 |
| 75G3 | 37223 | 14472 | 1.5066 | 1.7801 | 1.0000 | 1.1815 | 1.0000 | 1.1815 |
| 194C10 | 28138 | 13217 | 1.9930 | 1.9491 | 1.0000 | 0.9780 | 1.0000 | 0.9780 |
| 85F7 | 32968 | 16509 | 1.7010 | 1.5605 | 1.0000 | 0.9174 | 1.0000 | 0.9174 |
| 199A7 | 17005 | 9455 | 3.2978 | 2.7247 | 1.0000 | 0.8262 | 1.0000 | 0.8262 |
| 146B6 | 24138 | 14412 | 2.3233 | 1.7875 | 1.0000 | 0.7694 | 1.0000 | 0.7694 |
| 193E7 | 35508 | 13783 | 1.5794 | 1.8691 | 1.0000 | 1.1835 | 1.0000 | 1.1835 |
| 65C12 | 56080 | 25761 | 1.0000 | 1.0000 | 1.0000 | 1.0000 | 1.0000 | 1.0000 |

The grayed values for antibodies 4B3, 50G9, 60D2, 59F2, 60E8, and 65E9 represent the statistically significant hits (i.e., ≥ 3x the IQR) whose binding was affected by mutations H203.

A summary of the hASGR-1 residues important for binding of the representative antibodies is shown in Table 7.6. In addition, this analysis revealed that the mutation of some ASGR-1 residues had more dramatic effects on a given antibody binding than others. This likely reflects the relative contribution or importance that these residues have in mediating interactions with specific test antibodies. The degree by which each mutation impacted the ability of a test antibody to bind was determined by calculating the magnitude of an individual binding data point above the upper gate determined by each IQR analysis. The relative impact of each mutation on the binding of a given test antibody was then ranked using this method and displayed as a heatmap in Table 7.6. Dark grey coloring indicates the data point deviated dramatically from the upper gate (ie. a large effect on antibody binding), while light grey/white indicates the data point was very close to the cut offs (ie. 3x the IQR)(Table 7.6). When aligned with the relative epitope profiling bin assignments (Example 7D above), this analysis reveals a set of core ASGR-1 amino acid positions that, when mutated, disrupt test antibody binding. As such, these positions are likely part of the ASGR-1 epitopes bound by the selected antibodies. These amino acid residues either directly contact or are involved in the interaction with the antibody, or are in close enough proximity that, when mutated, interfere with antibody binding. Amino acid positions identified as statistically significant hits, but barely made the cut offs, and map to surface ASGR-1 locations distinct from the main epitope bins (Figure 47) may represent residues that, when mutated, disrupt the conformation of ASGR-1 such that an antibody that binds to a distinct epitope is affected (ie. an indirect effect). mAb 197G3 is an example of an antibody displaying a range of binding sensitivities in this assay, yet the most important residues (R274 and R271) can be identified by rank ordering them as described.

# Table 7.6. ASGR-1 residues identified as hits via Arg/Glu scanning mutagenesis

++++        -

| mAb | Relative Epitope Profiling Bin | ASGR1 CBD Mutations That Reduced Antibody Binding Signal (3xIQR Cutoff) | | | | | | | | | | | | | | | | | | | |
|---|---|---|---|---|---|---|---|---|---|---|---|---|---|---|---|---|---|---|---|---|---|
| 4A2 | A | W195 | | | | | | | | | | | | | | | | | | | |
| 7E11 | A | W195 | | | | | | | | | | | | | | | | | | | |
| 56E5 | A | W195 | | | | | | | | | | | | | | | | | | | |
| 7G4 | A | W195 | R263 | | | | | | | | | | | | | | | | | | |
| 53F7 | A | W195 | K199 | E196 | | | | | | | | | | | | | | | | | |
| 10G6 | A | W195 | E196 | | | | | | | | | | | | | | | | | | |
| 26C4 | A | W195 | P207 | | | | | | | | | | | | | | | | | | |
| 6G6 | A | | | | | | | | | | | | | | | | | | | | |
| 29H8 | A | W195 | P207 | | | | | | | | | | | | | | | | | | |
| 25A4 | A | | | | | | | | | | | | | | | | | | | | |
| 32D6 | A | W195 | K199 | | | | | | | | | | | | | | | | | | |
| 198D2 | unknown | W195 | E196 | H204 | | | | | | | | | | | | | | | | | |
| 4B3 | A | H203 | H204 | | | | | | | | | | | | | | | | | | |
| 50G9 | A | H203 | H204 | | | | | | | | | | | | | | | | | | |
| 60D2 | A | H203 | H204 | | | | | | | | | | | | | | | | | | |
| 59F2 | A | H203 | H204 | | | | | | | | | | | | | | | | | | |
| 60E8 | A | H203 | H204 | P220 | G251 | | | | | | | | | | | | | | | | |
| 65E9 | A.1 | H203 | H204 | | | | | | | | | | | | | | | | | | |
| 5E5 | A | K199 | W195 | R263 | | | | | | | | | | | | | | | | | |
| 29E2 | A | K199 | R263 | | | | | | | | | | | | | | | | | | |
| 45B4 | A | K199 | R263 | | | | | | | | | | | | | | | | | | |
| 6G7 | B | L184 | | | | | | | | | | | | | | | | | | | |
| 72F5 | B.1 | L184 | | | | | | | | | | | | | | | | | | | |
| 22G5 | B | L184 | N265 | P220 | H215 | G251 | G248 | R183 | G246 | | | | | | | | | | | | |
| 48B12 | B | L184 | | | | | | | | | | | | | | | | | | | |
| 151B9 | B | L184 | | | | | | | | | | | | | | | | | | | |
| 52H2 | B | L184 | P238 | H247 | G251 | P220 | | | | | | | | | | | | | | | |
| 149D11 | B | R170 | L184 | S171 | | | | | | | | | | | | | | | | | |
| 175F4 | B | R183 | | | | | | | | | | | | | | | | | | | |
| 147E9 | C | D243 | P241 | D242 | G251 | E253 | Y245 | | | | | | | | | | | | | | |
| 61A1 | C | D243 | P241 | Y245 | D242 | E253 | G251 | | | | | | | | | | | | | | |
| 184E7 | C | D243 | E253 | P241 | | | | | | | | | | | | | | | | | |
| 72G9 | C | D243 | Y245 | P241 | D242 | E253 | G251 | | | | | | | | | | | | | | |
| 194A4 | C | D260 | | | | | | | | | | | | | | | | | | | |
| 60C12 | E | R237 | D260 | R263 | | | | | | | | | | | | | | | | | |
| 173C11 | E | R237 | D260 | T259 | R263 | | | | | | | | | | | | | | | | |
| 56E3 | E.1 | R237 | T259 | R263 | N265 | D260 | P241 | R170 | | | | | | | | | | | | | |
| 54E9 | E.1 | R237 | T259 | N265 | R263 | D260 | P241 | E239 | | | | | | | | | | | | | |
| 65D5 | E | D260 | R237 | R263 | T259 | | | | | | | | | | | | | | | | |
| 190F8 | L | R271 | R274 | G172 | P272 | V208 | | | | | | | | | | | | | | | |
| 198G3 | L | R271 | R274 | G172 | | | | | | | | | | | | | | | | | |
| 191G10 | L | R271 | G172 | R274 | | | | | | | | | | | | | | | | | |
| 202A3 | unknown | R271 | G172 | N209 | | | | | | | | | | | | | | | | | |
| 194C1 | L | R274 | R271 | P272 | G172 | V208 | R170 | | | | | | | | | | | | | | |
| 176H4 | R | R271 | P272 | N265 | G172 | P241 | L249 | H247 | D242 | | | | | | | | | | | | |
| 197G3 | L | R274 | R271 | R170 | G172 | D243 | G248 | D216 | P272 | S171 | Q270 | L249 | E196 | D260 | H215 | D225 | D228 | G251 | E280 | P207 | H204 |
| 191G1 | L | R274 | P238 | R271 | P272 | G172 | V208 | | | | | | | | | | | | | | |
| 213B3 | L | P238 | R271 | R274 | P272 | G172 | | | | | | | | | | | | | | | |
| 218G4 | O | R274 | P238 | G172 | R271 | | | | | | | | | | | | | | | | |
| 75G3 | M | R274 | R170 | G172 | V208 | | | | | | | | | | | | | | | | |
| 194C10 | T | R274 | R170 | G172 | V208 | | | | | | | | | | | | | | | | |
| 85F7 | M.1 | R274 | R170 | V208 | G172 | | | | | | | | | | | | | | | | |
| 199A7 | N | H215 | R170 | R183 | Q270 | | | | | | | | | | | | | | | | |
| 146B6 | P | T259 | N265 | P241 | | | | | | | | | | | | | | | | | |
| 193E7 | Q | R263 | P207 | | | | | | | | | | | | | | | | | | |
| 65C12 | Q | | | | | | | | | | | | | | | | | | | | |

In order to compare the mutational hit patterns of the individual test antibodies with each other, the coefficient of determination between the test antibodies was determined. The expression and antibody binding normalized data set was used to generate binding profiles for each test antibody across the mutant panel. The resulting profiles for each individual test antibody were then compared for their degree of similarity to all of the other test antibodies. The coefficient of determination ($R^2$) for each combination was determined and converted into a heat map in order to visualize the resulting patterns (Figure 46). For simplicity, a representative antibody from each unique mutational profile (Reference Antibody) is shown in Figure 46. This analysis revealed 7 predominant hit patterns or mutational clusters.. Test antibodies affected by the 7 predominant mutational clusters correspond to those from competition/binding binning profiles A, B, C, E and L (3 distinct hit/mutational clusters of bin A antibodies and 1 distinct hit/mutational cluster of bin B, bin C, bin E and bin L antibodies). The remaining antibodies, categorized as displaying separate binning profiles (compared to bins A, B, C, E and L), are affected by distinct mutations in ASGR-1, but also include residues that partially overlap with test antibodies belonging to the predominant bins.

This data indicates the selected antibodies bind to epitopes that partially overlap with the 7 predominant epitope regions. The residues important for the binding of antibodies belonging to the 7 predominant epitope regions were then mapped onto a computer representation of the surface of the ASGR-1 structure using the PyMOL Molecular Graphics System (Version 1.8; Schrödinger, LLC.) (Figure 47). A residue on the surface of ASGR-1 was considered part of the same epitope region if at least one antibody from a distinct binning profile (ie. A, B, C, E and L) was identified as being sensitive to mutation. For example, the predominant epitope region for antibodies belonging to binning profile C includes hASGR-1 residues P241, D242, D243, Y245, G251 and E253 (SEQ ID NO:5). The binding of antibody 147E9 is affected by mutation of all of these residues, while antibody 184E7 is only disrupted by mutation of P241, D243 and E253. Thus, the predominant epitope region of ASGR-1 bound by antibodies belonging to binning profile C is defined as including one or more of (but not limited to) P241, D242, D243, Y245, G251 and E253 (SEQ ID NO:5). Also, note that the antibody 194A4 was classified as belonging to Bin C as determined in Example 7D, however, the results of this arginine/glutamic acid mutational profiling (as well as the results from crystal structure analysis of the ASGR-1 CBD/194A4 complex described in Example 10H) suggests that the relative epitope profiling may have been inaccurate.

Antibodies belonging to binning profile A were further sub-divided into 3 distinct mutational clusters. These clusters mapped to ASGR-1 surface positions that overlap with, or are in extremely close physical proximity to, each other consistent with a common binning profile. Antibodies that displayed binning profiles distinct from the 5 major bins (i.e., A, B, C, E and L) also showed distinct patterns of mutations that affected their binding (Figure 46). Some binning profiles (R, O, M, M.1 and T) share significant overlap with antibodies from binning profile L, and can be considered sub-bins of this profile. Taken together, this data indicates that antibodies capable of blocking ASGR-1-ligand interactions bind to 5 major epitope regions. In addition, blocking antibodies were identified that bind to partially overlapping epitopes of these major regions.

**Example 8: ASGR Internalization Assay**

To determine whether the antibodies bind and also prevent internalization of ASGR-1 into cells expressing ASGR-1, an in vitro internalization assay is performed of various antibody samples.

Human ASGR-1 Internalization Cellular Imaging Assay Protocol

Reagents:

U2OS (Human Osteosarcoma) cell line
McCoy's 5A Medium: Gibco, #16600-082
MEM NEAA (100X): Gibco, #11140-050
Penicillin-Streptomycin (10,000U/ml, 100X) Gibco, #15140-122
L-Glutamine (100X): Gibco, #25030-081
Fetal Bovine Serum: Gibco, #16000-044
DPBS (without Ca and Mg): Gibco, #14190-136
DPBS (with Ca and Mg): Gibco, #14040-133
Cell Dissociation Buffer: Gibco, #13151-014
1Liter Filter: Coming, #430517
Hepes Buffer (1M): Gibco, #15630-080
BacMam Virus - huASGR-1: GS: SNAP26f
β-GalNAc-PAA-Biotin: GlycoTech, #01-011
SNAP-Surface Alexa Fluor 546: New England Biolabs, #S9132S
Streptavidin-Alexa Fluor 633: Life Technologies, #S21375
Hoechst 33342: Invitrogen, #H3570
Pitstop2: abcam Biochemical, #ab120687
Pitstop2 - negative control: abcam Biochemical, #ab120688
Paraformaldehyde (8% Aqueous Solution): Electron Microscopy Sciences, #157-8-100
Imaging plate - 96 well Optical Bottom: Thermo Scientific Nunc, #165305

Operetta High Content Imager: Perkin Elmer

U2OS complete growth medium:

McCoy's 5A with 10%FBS, 1XMEM NEAA, 1XL-Glutamine, and 1XPenicillin-Streptomycin Medium was filtered before use on cells

U2OS cell plating and culturing:

U2OS cells were grown to 75-85% confluence in T175 before plating into a 96 well plate.

1. The U2OS culture medium was aspirated off the cells in the T175 flask

2. Cells were washed with 10mls of DPBS and aspirated off

3. 3mls of Cell Dissociation Buffer was added to the cells and incubated for 5 minutes inside a cell incubator (37°C, 5%CO2) to detach the cells from the T175 flask.

4. The detached cells were diluted with 7mls of the growth medium

5. 1ml of cells were used to count the number of cells available to plate

6. The cells were diluted in growth medium to give a final concentration of 28,000 cells/well and BacMam virus (huASGR-1: GS: SNAP26f) was also added to the cells at this time with the desired concentration (MOI).

7. The cells were mixed together with the BacMam virus for 1-2 minutes and then plated on the 96 well imaging plate at a volume of 100ul/well.

8. The plate was placed inside an incubator (37°C, 5%CO2) for 16-20 hours before treatment.

Treatment of Cells (16-20 hours incubation)

1. The next day, the medium on the 96 well plate was dumped out and washed once with DPBS.

2. McCoy's 5A Medium plus 10mM of Hepes buffer (assay buffer) was added to the cells (100ul) for 1 hour inside the incubator.

3. After the 1 hour incubation, the medium was dumped out and washed once with DPBS containing Ca and Mg.

4. Pitstop2 and Pitstop2 negative control were prepared in assay buffer at 20uM.

5. Volume of 100u1 per well of the inhibitors were added to the U2OS cells for 15 minutes inside the incubator.

6. GalNAc-biotin (100nM) and strepavidin-Alexa633 (100nM) were pre-mixed in assay buffer and incubated for 10 minutes at room temperature.

7. SNAP-Surface Alexa Fluor 546 (2.5uM) was prepared in assay buffer.

8. After the 15 minutes incubation, both GalNAc-biotin-strepavidin-Alexa633 and SNAP-Surface Alexa Fluor 546 were directly added (10ul) to the medium containing Pitstop2 inhibitors for 30 minutes inside the incubator.

9. After the 30 minutes incubation, medium was dumped out and the cells were washed once with DPBS.

10. The cells were fixed by adding 50ul of 4% Paraformaldehyde (8% paraformaldehyde was diluted with DPBS) containing Hoechst dye (1:5000 dilution) to the cells for 10 minutes at room temperature.

11. After 10 minutes incubation, the cells were washed twice with DPBS and 100ul of DPBS was added to each well.

12. The plate was imaged on the Operetta instrument with three channels measuring the different fluorescence dyes.

1) Hoechst was measured using filters in the range of excitation: 360-400nm and emission: 410-480nm

2) GalNAc-biotin-strepavidin-Alexa633 was measured using filters in the range of excitation: 600-630nm and emission: 640-680nm

3) SNAP-Surface Alexa Fluor 546 was measured using filters in the range of excitation: 520-550nm and emission: 560-630

13. Harmony 3.5 software (Perkin Elmer) was used to identify and quantify internalized spots for fluorescence dyes added in the assay.

This internalization assay can be performed to assay the antigen binding proteins of the invention to determine how much they reduce or inhibit internalization of ASGR, ASGR-1, and/or ASGR-2.

**Example 9: Additional ligand blocking assays**

Preparation of desialated protein ligands (asialofetuin and orosomucoid)

A. Asialofetuin

Bovine fetuin (AHSG) was obtained commercially (Sigma) and purified using a CaptoQ Impres (GE Healthcare Life Sciences) matrix. Briefly, the material was loaded in 25mM TRIS pH 7.9 at up to 17mg/ml resin, resolved in 20mM BisTRIS (pH6.5) with a gradient of sodium chloride. The main peak was gradient pooled (-0.15M NaCl final) and resolved on a SuperDex200 SEC (GE Healthcare Life Sciences) in Hepes-buffered saline (pH 7.9). The purified AHSG was then concentrated and incubated with Innolink Biotin 3545 (EMD Millipore) according to the manufacturer's instructions. The biotinylated protein was then desalted by gel filtration and concentrated once again.
The purified, biotinylated protein was subsequently desialated by incubation with C. perfringens neuraminidase (Sigma; 1 unit/10mg protein for 12 hours at 37oC in 50mM sodium phosphate, 9mM HEPES, 0.12M NaCl, pH6). The resulting material was harvested and digested for an additional 3 hours with A. ureafaciens neuraminidase (QAbio; 0.5 units/10mg protein at 37oC). The digested sample was diluted 3 fold with 20mM HEPES containing 0.15M NaCl (pH 7.5) (HBS) to neutral pH and applied to a monomeric Avidin agarose (Pierce) HR16/10 column, run at 60cm/hour. The loaded column was held for 15 minutes then washed with four column volumes of HBS. The biotinylated, desialated protein was finally eluted with three column volumes of HBS containing 2mM Biotin plus an additional two column volumes of 0.1M Glycine-HCl (pH 2.8), which was immediately neutralized during collection with 50mM TRIS Base). Protein-containing fractions from both types of elutions were identified, pooled, concentrated, dialyzed extensively against 10mM HEPES, 0.14M NaCl (pH 7.5), re-concentrated and finally filtered sterilized. The purified lots were then analyzed by SDS-PAGE and mass spectrometry prior to use in the described assays.

B. Orosomucoid

Bovine orosomucoid (AGP) was obtained commercially (Sigma) and purified over SuperDex200 resin equilibrated in HBS (pH7.9) by size exclusion chromatography. The front of the main AGP peak was combined from 3 individual runs to generate hyperglycosylated AGP, with the remainder of the main peaks (from the 3 combined runs) to generate hypoglycosylated AGP. For biotinylation, the purified AGP was concentrated to 5mg/ml and incubated with Innolink Biotin 354S as described. The biotinylated protein was then desalted by gel filtration and concentrated.
After biotinylation, the protein was desialated by incubating it for 18 hours at 37oC with one unit of C. perfringens neuraminidase (Sigma) per 10mg protein in 50mM sodium phosphate, 9mM HEPES, 0.12M NaCl (pH6). The resulting material was harvested and digested for an additional 6 hours at 37oC with 0.5 units A. ureafaciens neuraminidase (QAbio) per 10mg protein. The sample was diluted 3 fold with HBS to achieve a neutral pH and applied to a monomeric Avidin agarose (Pierce) HR16/10 column, run at 60cm/hour. The loaded column was held for 15 minutes and then washed with four column volumes of HBS. The biotinylated, desialated protein was subsequently eluted with three column volumes of HBS containing 2mM Biotin, plus two column volumes 0.1M Glycine-HCl (pH 2.8), which was immediately neutralized during collection with 50mM TRIS Base. Protein-containing fractions from both types of elutions were identified, pooled, concentrated, dialyzed extensively against 10mM HEPES, 0.14M NaCl (pH 7.5), re-concentrated and finally filtered sterilized. The purified lots were then analyzed by SDS-PAGE and mass spectrometry prior to use in the described assays.

These ligands can be used in additional ligand binding assays to determine antigen binding protein inhibition of ligand binding to ASGR, ASGR-1 and/or ASGR-2.

**Example 10: Crystal Stucture Analysis of Interaction between Ligands and ASGR-1 and Antibodies and ASGR-1**

**A. Crystal Structures of ASGR-1 Carbohydrate Binding Domain with Ligand Bound**

**Introduction**

The crystal structure of ligand free ASGR-1 CBD (carbohydrate binding domain) has been previously described (1). Protein expression of ASGR-1 CBD (SEQ ID NO:5), purification and crystallization was performed similar to the published method, however the structures described here differ from the published crystal structure. Analysis of these structures shows extra N- and C-terminal amino acids compared to the published structure, how various ligands interact with the ASGR-1 carbohydrate binding domain, and possible selectivity determinants between ASGR-1/ASGR-2 for various saccharides.

**Results**

**Lactose binds in the carbohydrate binding pocket of ASGR-1**

Protein crystals of the ASGR-1/Lactose complex were grown and the crystal structure was determined at 2.05 Å. Although a method similar to that of the published structure was followed, clear electron density is present for the lactose disaccharide in the carbohydrate binding pocket. See Figures 18A and 18B. In this structure, the galactose ring of the lactose disaccharide sits on top of the calcium ion at the carbohydrate binding domain and forms the majority of the contacts with the ASGR-1 protein. Hydrogen bonds are formed between lactose and ASGR-1 amino acids Q240, D242, E253, and N265. Additionally, van der Waals interactions are formed with at least W244 (SEQ ID NO:5). See Figure 18C.

Analysis of the crystal structure identifies specific amino acids involved in the interaction between ASGR-1 and lactose. Interacting with at least these amino acids by an alternate molecule can completely or partially affect the interaction between ASGR-1 and lactose.

ASGR-1 / Lactose analysis (Distances below were calculated with PyMOL):

Amino acids with at least one non-hydrogen atom 4.5 Å or less to the bound lactose molecule were identified and include: Q240, D242, W244, E253, N265, D266, D267 (SEQ ID NO:5).

Amino acids with at least one non-hydrogen atom 5 Å or less to the bound lactose molecule were identified and include: Q240, D242, W244, E253, N265, D266, D267 (SEQ ID NO:5).

Amino acids with at least one non-hydrogen atom 5-8 Å from the bound lactose molecule were identified and include: N209, R237, P238, E239, P241, D243, Y245, G246, H247, G252, C255, H257, T259, D260, V268, R271, Y273 (SEQ ID NO:5).

**Galactose binds in the carbohydrate binding pocket of ASGR-1 similar to lactose**

Protein crystals of the ASGR-1/Galactose complex were grown and the crystal structure was determined at 2.4 Å. Although a method similar to that of the published structure was followed, clear electron density is present for the galactose saccharide in the carbohydrate binding domain. See Figure 19A and 19B.

In this structure, galactose sits on top of the calcium ion at the carbohydrate binding site and forms contacts with the ASGR-1 protein. Hydrogen bonds are formed between galactose and ASGR-1 amino acids Q240, D242, E253, and N265 (SEQ ID NO:5). Additionally, van der Waals interactions are formed with at least W244. See Figure 19C.

Analysis of the crystal structure identifies specific amino acids involved in the interaction between ASGR-1 and galactose. Interacting with at least these amino acids by an alternate molecule may completely or partially affect the interaction between ASGR-1 and galactose. Distances below were calculated with PyMOL.

ASGR-1 / Galactose analysis (Distances below were calculated with PyMOL):

Amino acids with at least one non-hydrogen atom 4.5 Å or less to the bound galactose molecule were identified and include: R237, Q240, D242, W244, E253, N265, D266, D267 (SEQ ID NO:5). Amino acids with at least one non-hydrogen atom 5 Å or less to the bound lactose molecule were identified and include: R237, Q240, D242, W244, E253, N265, D266, D267 (SEQ ID NO:5).

Amino acids with at least one non-hydrogen atom 5-8 Å from the bound lactose molecule were identified and include: N209, P238, E239, P241, D243, Y245, G246, H247, G252, C255, H257, T259, V268, R271, Y273 (SEQ ID NO:5).

When comparing the ASGR-1/Lactose and ASGR-1/Galactose structures, the galactose rings of each saccharide superimpose very well. One difference in the proteins in the two structures is the conformation of R237, an amino acid in close proximity to the carbohydrate binding site. In the superimposition shown in Figure 20, the ASGR-1/Lactose structure is shown in white and the ASGR-1/Galactose structure is shown in black.

**N-acetyl-D-galactosamine (GalNAc) binds in the carbohydrate binding pocket of ASGR-1 similar to galactose, buts forms additional interactions**

Protein crystals of the ASGR-1/GalNAc complex were grown and the crystal structure was determined at 2.2 Å. Although a method similar to that of the published structure was followed, clear electron density is present for the GalNAc saccharide in the carbohydrate binding pocket. See Figure 21A and Figure 21B.

In this structure, GalNAc sits on top of the calcium ion at the carbohydrate binding site and forms contacts with the ASGR-1 protein. Hydrogen bonds are formed between GalNAc and ASGR-1 amino acids Q240, D242, E253, and N265. Additionally, van der Waals interactions are formed with at least W244. In this structure, R237 is in a similar conformation as observed in the galactose complex. However, in this case hydrogen bonds are formed between R237 and the acetyl of GalNAc. These additional interactions with R237 help explain both the observed tighter binding of GalNAc (than galactose) to ASGR-1, and the tighter binding to GalNAc to ASGR-1 (than ASGR-2, in which this amino acid is Ala rather than Arg). See Figure 21C

**ASGR-1 / GalNAc analysis (Distances were calculated with PyMOL):**

Analysis of the crystal structure identifies specific amino acids involved in the interaction between ASGR-1 and GalNAc. Interacting with at least one of these amino acids by an alternate molecule may completely or partially inhibit the interaction between ASGR-1 and GalNAc.

Amino acids with at least one atom 4.5 Å or less to the bound GalNAc molecule were identified and include: N209, R237, Q240, D242, W244W244, E253, H257, T259, N265, D266, D267, Y273 (SEQ ID NO:5). Amino acids with at least one non-hydrogen atom 5 Å or less to the bound lactose molecule were identified and include: N209, R237, Q240, D242, W244, E253, H257, T259, N265, D266, D267, Y273 (SEQ ID NO:5).

Amino acids with at least one non-hydrogen atom 5-8 Å from the bound lactose molecule were identified and include: P238, E239, P241, D243, Y245, G246, H247, G252, C255, F258, D260, R263, W264, V268, R271 (SEQ ID NO:5).

The coordinates for the ASGR-1 CBD/GalNAc crystal structure complex are presented in Table 10.1.

**Methods**

ASGR-1 expression and purification

For all chrystallography experiments in Example 12, Human ASGR-1 CBD protein (SEQ ID NO:5) was expressed in E.coli and refolded and purified.

ASGR-1 crystallization

Purified human ASGR-1 CBD (148-291) protein was concentrated to 8-12 mg/ml. ASGR-1/carbohydrate complex crystals grow in 0.1 M sodium cacodylate pH 6.8, 0.08 M ammonium sulfate, 21-23% PEG 8000 in the presence of 20 mM ligand (lactose, galactose or GalNAc).

Data collection and structure determination

Datasets for ASGR-1 CBD complexes were collected on a Rigaku FR-E X-ray source (ASGR-1/Lactose and ASGR-1/Galactose) or at Berkeley Advanced Light Source beamline 5.0.2 (ASGR-1/GalNAc). All datasets were processed with iMosflm(2) and scaled with AIMLESS(3) from the CCP4 program suite(4).

ASGR-1/Lactose crystals grow in the C2 space group with unit cell dimensions a=113.5, b=32.3, c=40.4 Å, $\beta$=92.3° with one complex molecule per asymmetric unit, and diffract to 2.05 Å resolution. The ASGR-1 structure was solved by molecular replacement with the program PHASER(5) using the published ASGR-1 structure(1) as the starting search model. The structure was improved with multiple rounds of model building with Coot(6) and refinement with PHENIX(7). The refined structure has R=18.9 and $R_{free}$=24.4.

ASGR-1/Galactose crystals grow in the C2 space group with unit cell dimensions a=113.1, b=32.7, c=40.7 Å, β=91.6° with one complex molecule per asymmetric unit, and diffract to 2.4 Å resolution. The ASGR-1/Lactose structure was used as the starting molecule for molecular replacement, and model building and refinement were performed as described for the ASGR-1/Lactose complex to R=15.8 and $R_{free}$=22.9.

ASGR-1/GalNAc crystals grow in the C2 space group with unit cell dimensions a=112.7, b=32.3, c=40.5 Å, β=91.7° with one complex molecule per asymmetric unit, and diffract to 2.2 Å resolution. The ASGR-1/Lactose structure was used as the starting molecule for molecular replacement, and model building and refinement were performed as described for the ASGR-1/Lactose complex to R=16.5 and $R_{free}$=23.0.

Structure analysis and distance calculations were performed with the program PyMOL(8).

**References**

1. Meier, M., Bider, M. D., Malashkevich, V. N., Spiess, M., and Burkhard, P. (2000) Crystal structure of the carbohydrate recognition domain of the H1 subunit of the asialoglycoprotein receptor. Journal of molecular biology 300, 857-865

2. Battye, T. G., Kontogiannis, L., Johnson, O., Powell, H. R., and Leslie, A. G. (2011) iMOSFLM: a new graphical interface for diffraction-image processing with MOSFLM. Acta crystallographica 67, 271-281

3. Evans, P. (2006) Scaling and assessment of data quality. Acta crystallographica 62, 72-82

4. CCP4. (1994) The CCP4 suite: programs for protein crystallography. Acta crystallographic a 50, 760-763

5. McCoy, A. J., Grosse-Kunstleve, R. W., Adams, P. D., Winn, M. D., Storoni, L. C., and Read, R. J. (2007) Phaser crystallographic software. Journal of applied crystallography 40, 658-674

6. Emsley, P., Lohkamp, B., Scott, W. G., and Cowtan, K. (2010) Features and development of Coot. Acta crystallographica 66, 486-501

7. Adams, P. D., Afonine, P. V., Bunkoczi, G., Chen, V. B., Davis, I. W., Echols, N., Headd, J. J., Hung, L. W., Kapral, G. J., Grosse-Kunstleve, R. W., McCoy, A. J., Moriarty, N. W., Oeffner, R., Read, R. J., Richardson, D. C., Richardson, J. S., Terwilliger, T. C., and Zwart, P. H. (2010) PHENIX: a comprehensive Python-based system for macromolecular structure solution. Acta crystallographic a 66, 213-221

8. DeLano, W. L. (2002) The PyMOL Molecular Graphics System. Palo Alto

**B. Crystal Structure of ASGR-1 Carbohydrate Binding Domain (CBD) with 5E5**

The present example presents the crystal structure of the ASGR-1 CBD bound to the Fab fragment of 5E5, determined to 1.95 Å resolution (the conditions for which are described in the below). This structure, depicted in FIG. 22A&B, shows that when 5E5 binds to/interacts with ASGR-1, a conformational rearrangement of the carbohydrate binding loop occurs, impairing the carbohydrate binding loop from binding to/interacting with ligand (i.e., carbohydrates). This demonstrates that the 5E5 Fab indirectly inhibits the ASGR-1 CBD/Ligand binding.

The depicted structure also allows one to identify specific core ASGR amino acid residues for the interaction interface of 5E5 with ASGR-1. This was defined as residues that are within 5 Å of the 5E5 protein. The core residues are as follows: H161, E162, W195, E196, Q198, K199, F200, Q202, H203, H204, G232, F233, K234, N235, W236, R237, P238, D261, G262, R263 (SEQ ID NO:5).

The structures were also used to identify boundary ASGR-1 amino acid residues for the interaction interface with 5E5. These residues were ASGR-1 residues that were from 5-8 Å of the 5E5 protein. The boundary residues are as follows: V159, E160, R163, T193, S194, E197, V201, 1205, G206, P207, Y229, E230, T231, E239, F258, T259, D260, W264 (SEQ ID NO:5).

Specific core 5E5 amino acid residues of the interaction interface with ASGR-1 were defined as 5E5 residues that are within 5 Å of the ASGR-1 protein. The core 5E5 Heavy Chain residues include: S30, N31, W52, Y53, D54, S56, N57, Y59, Y101, S102, S103, G104, W105, Y106, D107; and the core 5E5 Light Chain residues include: 5E5 Light Chain: Q27, R30, D32, H91, Y92, S93, Y94.

Boundary 5E5 amino acid residues of the interaction interface with ASGR-1 were defined as 5E5 residues that are 5-8 Å from the ASGR-1 protein. The boundary 5E5 Heavy Chain residues include: Y32, V33, V50, G55, K58, N74, E99, V100, Y108; and the boundary 5E5 Light Chain residues include: I2, G28, I29, L33, Q90, P95, R96.

**Methods**

**Expression and purification of protein samples**

The 5E5 Fab fragment was generated by cleaving the 5E5 mAb with caspase 3. Post caspase cleavage, the Fab was isolated by purification on a MonoS ion exchange column. Ni Sepharose Excel subtraction was then performed to ensure the Fc domain was removed from the sample.

5E5 mAb Heavy Chain (SEQ ID NO: 32695):

QVQLVESGGGVVQPGRSLRLSCAASGFTFSNYVMHWVRQAPGKGLEWVAVIWYDGSNKY
YADSVKGRFTISRDNSKNTLYLQMNSLRAEDTAVYYCTREVYSSGWYDYGMDVWGQGTT
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCGSDEVDGGDKTHTCPP
CPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPGHHHHHH

5E5 mAb Light Chain (SEQ ID NO:32696):

DIQMTQSPSSLSASVGDRVTITCRASQGIRNDLGWYQQKPGKAPKRLIYAASSLQSGVPSRF
SGSGSGTEFTLTISSLQPEDFATYYCLQHYSYPRTFGQGTKVEVKRTVAAPSVFIFPPSDEQL
KSGTASVVCLLNNFYPREAKVQWKVDNALQSGNSQESVTEQDSKDSTYSLSSTLTLSKADY
EKHKVYACEVTHQGLSSPVTKSFNRGEC

5E5 Fab Heavy Chain (Post Cleavage) (SEQ ID NO:32697):

QVQLVESGGGVVQPGRSLRLSCAASGFTFSNYVMHWVRQAPGKGLEWVAVIWYDGSNKY
YADSVKGRFTISRDNSKNTLYLQMNSLRAEDTAVYYCTREVYSSGWYDYGMDVWGQGTT
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCGSDEVD

5E5 Fab Light Chain (Post Cleavage) (SEQ ID NO:32698):

DIQMTQSPSSLSASVGDRVTITCRASQGIRNDLGWYQQKPGKAPKRLIYAASSLQSGVPSRF
SGSGSGTEFTLTISSLQPEDFATYYCLQHYSYPRTFGQGTKVEVKRTVAAPSVFIFPPSDEQL
KSGTASVVCLLNNFYPREAKVQWKVDNALQSGNSQESVTEQDSKDSTYSLSSTLTLSKADY
EKHKVYACEVTHQGLSSPVTKSFNRGEC

Complex formation and crystallization

The ASGR-1 CBD / 5E5 Fab complex was made by mixing a molar excess of ASGR-1 CBD with 5E5 Fab. The complex was separated from excess ASGR-1 by purification on a size exclusion chromatography column. The ASGR-1 CBD / 5E5 Fab complex was concentrated to 10 mg/ml and crystallizes in 0.1 M Tris pH 8.5, 12% PEG 4000.

Data collection and structure determination

The dataset for the ASGR-1 CBD / 5E5 Fab complex crystal was collected on beamline 5.0.2 at the Berkeley synchrotron and processed with Mosflm[1]/Aimless[2].

ASGR-1 CBD / 5E5 Fab complex crystals grow in the $P2_1$ space group with unit cell dimensions a=62.93, b=41.75, c=118.89 Å and β=97.16 with one complex molecule per asymmetric unit, and diffract to 1.95 Å resolution. The ASGR-1 CBD / 5E5 Fab complex structure was solved by molecular replacement with the program Molrep[2]. The structure was improved with multiple rounds of model building with Coot[3] and refinement with Phenix[4], to a final R=25.9

/ $R_{free}$=30.5. While the electron density for the ASGR-1 CBD and 5E5 Fab variable domain (along with the corresponding interface) is quite good, the electron density for the 5E5 constant domain is poor (most likely due to poor packing within the crystal lattice). This likely explains the higher R/$R_{free}$ observed from this structure refinement.

Core interaction interface amino acids were determined as being all amino acid residues with at least one non-hydrogen atom less than or equal to 5 Å from the partner protein. 5 Å was chosen as the core region cutoff distance to allow for atoms within a van der Waals radius plus a possible water-mediated hydrogen bond. Boundary interaction interface amino acids were determined as all amino acid residues with at least one non-hydrogen atom less than or equal to 8 Å from the partner protein but not included in the core interaction list. Less than or equal to 8 Å was chosen as the boundary region cutoff distance to allow for the length of an extended arginine amino acid. Amino acids that met these distance criteria were calculated with the program PyMOL[5].

## References

1. Battye, T.G., Kontogiannis, L., Johnson, O., Powell, H.R. & Leslie, A.G. iMOSFLM: a new graphical interface for diffraction-image processing with MOSFLM. Acta Crystallogr D Biol Crystallogr 67, 271-81 (2011).
2. CCP4. The CCP4 suite: programs for protein crystallography. Acta Crystallogr D Biol Crystallogr 50, 760-3 (1994).
3. Emsley, P., Lohkamp, B., Scott, W.G. & Cowtan, K. Features and development of Coot. Acta Crystallogr D Biol Crystallogr 66, 486-501 (2010).
4. Adams, P.D. et al. PHENIX: a comprehensive Python-based system for macromolecular structure solution. Acta Crystallogr D Biol Crystallogr 66, 213-21 (2010).
5. DeLano, W.L. The PyMOL Molecular Graphics System. (Palo Alto, 2002).

## C. Crystal Structure of ASGR-1 Carbohydrate Binding Domain (CBD) with 22G5

The present example presents the crystal structure of the ASGR-1 CBD bound to the Fab fragment of 22G5, determined to 2.1 Å resolution (the conditions of which are described above in B). This structure, depicted in FIG. 23A&B, shows that when 22G5 binds to/interacts with ASGR-1, a conformational rearrangement of the carbohydrate binding loop occurs, impairing the carbohydrate binding loop from binding to/interacting with ligand (i.e., carbohydrates). This demonstrates that the 22G5 Fab indirectly inhibits the ASGR-1 CBD/Ligand binding.

The depicted structure also allows one to identify specific core ASGR amino acid residues for the interaction interface of 22G5 with ASGR-1. This was defined as residues that are within 5 Å of the 22G5 protein. The core residues are as follows: W167, S171, G172, K173, A174, A176, D177, N180, Y181, R183, L184, E185, D186, Q270, P272, W275 (SEQ ID NO:5).

The structures were also used to identify boundary ASGR-1 amino acid residues for the interaction interface with 22G5. These residues were ASGR-1 residues that were from 5-8 Å of the 22G5 protein. The boundary residues are as follows: P155, N157, W158, F168, S169, R170, W175, A178, D179, C182, A187, W211, C269, R271, Y273, R274, C277, T279 (SEQ ID NO:5).

Specific core 22G5 amino acid residues of the interaction interface with ASGR-1 were defined as 22G5 residues that are within 5 Å of the ASGR-1 protein. The core 22G5 Heavy Chain residues include: A33, V50, I51, S52, R53, S54, G55, G56, Y57, Y59, R99, A101, A103, G104, E106; and the core 22G5 Light Chain residues include: 22G5 Light Chain: Y32, S91, Y92, R93, Thfl4, Pro95, F97.

Boundary 22G5 amino acid residues of the interaction interface with ASGR-1 were defined as 22G5 residues that are 5-8 Å from the ASGR-1 protein. The boundary 22G5 Heavy Chain residues include: S30, S31, Y32, M34, N35, W47, S49, T58, R72, N74, L100, V102, S105; and the boundary 22G5 Light Chain residues include: I2, Q27, N28, NAG100, I29, S30, S31, Q90, L96.

## Methods:

The same methods were followed as described above in Example 10B except for the following changes:

The 22G5 Fab fragment was generated by cleaving the 22G5-IgG4 mAb with papain;
The ASGR-1 CBD / 22G5 Fab complex was concentrated to 8 mg/ml and crystallized in 0.1 Bis-Tris pH 6.5, 0.2 sodium malonate, 20% PEG 3350;
The dataset was processed with XDS/Aimless;
ASGR-1 CBD / 22G5 Fab complex crystals grow in the P212121 space group with unit cell dimensions a=46.04, b=80.34, c=169.14 Å with one complex molecule per asymmetric unit, and diffract to 2.1 Å resolution; and

The structure was improved with multiple rounds of model building with Coot3 and refinement with Phenix4, to a final R=17.8 / Rfree=22.5.

**D. Crystal Structure of ASGR-1 Carbohydrate Binding Domain (CBD) with 4A2**

The present example presents the crystal structure of the ASGR-1 CBD bound to the Fab fragment of 4A2, determined to 2.15 Å resolution (the conditions of which are described above in section B of this Example). This structure, depicted in Figures 24, 25 and 26, shows that when 4A2 binds to/interacts with ASGR-1, a conformational rearrangement of the carbohydrate binding loop occurs, impairing the carbohydrate binding loop from binding to/interacting with ligand (i.e., carbohydrates). This demonstrates that the 4A2 Fab indirectly inhibits the ASGR-1 CBD/Ligand binding.

The depicted structure also allows one to identify specific core ASGR amino acid residues for the interaction interface of 4A2 with ASGR-1. This was defined as residues that are within 5 Å of the 4A2 protein. The core residues are as follows: R170, W195, E196, K199, Q202, H203, H204, I205, G206, P207, V208, F233, K234, N235, W236, P238, D260, D261, G262, R263, R274 (SEQ ID NO:5).

The structures were also used to identify boundary ASGR-1 amino acid residues for the interaction interface with 4A2. These residues were ASGR-1 residues that were from 5-8 Å of the 4A2 protein. The boundary residues are as follows: N157, V159, F168, S169, S171, S194, Q198, F200, V201, T210, R237, E239, Q240, F258, T259, W264 (SEQ ID NO:5).

Specific core 4A2 amino acid residues of the interaction interface with ASGR-1 were defined as 4A2 residues that are within 5 Å of the ASGR-1 protein. The core 4A2 Heavy Chain residues include: T28, F29, T30, N31, Y32, D33, W50, H52, S55, N57, S99, S100, G101, W102, Y103; and the core 4A2 Light Chain residues include: 4A2 Light Chain: H31, S33, N34, N36, Y38, W56, Y97, Y98.

Boundary 4A2 amino acid residues of the interaction interface with ASGR-1 were defined as 4A2 residues that are 5-8 Å from the ASGR-1 protein. The boundary 4A2 Heavy Chain residues include: Y27, I34, N35, W47, M51, P53, N54, G56, T58, G59, Y104, D106; and the boundary 4A2 Light Chain residues include: I29, S32, N35, N37, Y55, T59, Q96, N99, T100.

The coordinates for the ASGR-1 CBD/4A2 crystal structure complex are presented in Table 10.2.

**Methods:**

The same methods were followed as described above in part B of this Example except for the following changes:

1. For this antibody only, a double stop codon was inserted at the end of CH1 domain that allowed for expression of a 4A2 Fab. The Fab purification was carried out via an affinity and a cation exchanger column. The final sequence of 4A2 Fab is:

Heavy Chain (SEQ ID NO:32650):

QVQLVQSGTEVKKPGASVKVSCKASGYTFTNYDINWVRQATGQGLEWMGWMHPN

SGNTGYAQKFQGRVTLTRDTSISTAYMELSSLRSEDTAVYYCASSSGWYYFDYWGQ

GTLVTVSSASTKGPSVFPLAPSSKSTSGGTAALGCLVKDYFPEPVTVSWNSGALTSG

VHTFPAVLQSSGLYSLSSVVTVPSSSLGTQTYICNVNHKPSNTKVDKKVEPKSCGSD

EVDGGD

Light Chain (SEQ ID NO:32651):

DIVMTQSPDSLAVSLGERATINCKSSQSILHSSNNNNYLAWFQQKPGQPPKLL

LYWASTRESGVPDRFSGSGSGTDFTLTISSLQPEDVAVYYCQQYYNTPVTFGP

GTKVGIKRTVAAPSVFIFPPSDEQLKSGTASVVCLLNNFYPREAKVQWKVDN

ALQSGNSQESVTEQDSKDSTYSLSSTLTLSKADYEKHKVYACEVTHQGLSSP

VTKSFNRGEC

1. The ASGR-1 CBD / 4A2 Fab complex was concentrated to 20 mg/ml and crystallized in 0.2 M Tri-Lithium citrate and 20% PEG3350;

2. The ASGR-1 CBD / 4A2 Fab complex crystals grow in the $P2_12_12_1$ space group with unit cell dimensions a=63.42, b=76.37, c=156.67 Å with one complex molecule per asymmetric unit, and diffract to 2.15 Å resolution; and

3. The structure was improved with multiple rounds of model building with Coot[3] and refinement with Phenix[4], to a final R=17.9 / R$_{free}$=21.8.

**Crystal Structure of ASGR-1 Carbohydrate Binding Domain (CBD) with 7E11**

The present example presents the crystal structure of the ASGR-1 CBD bound to the Fab fragment of 7E11, determined to 2.0 Å resolution (the conditions of which are described above in section B of this Example). This structure, depicted in FIGS. 27 and 28, shows that when 7E11 binds to/interacts with ASGR-1, a conformational rearrangement of the carbohydrate binding loop occurs, impairing the carbohydrate binding loop from binding to/interacting with ligand (i.e., carbohydrates). This demonstrates that the 7E11 Fab indirectly inhibits the ASGR-1 CBD/Ligand binding.

The depicted structure also allows one to identify specific core ASGR amino acid residues for the interaction interface of 7E11 with ASGR-1. This was defined as residues that are within 5 Å of the 7E11 protein. The core residues are as follows: H161, S194, W195, E196, Q198, K199, F200, Q202, H203, F233, K234, N235, W236, R237, P238, R263 (SEQ ID NO:5).

The structures were also used to identify boundary ASGR-1 amino acid residues for the interaction interface with 7E11. These residues were ASGR-1 residues that were from 5-8 Å of the 7E11 protein. The boundary residues are as follows: E160, E162, V192, T193, E197, V201, H204, Y229, E230, T231, G232, E239, Q240, P241, D261, G262, W264 (SEQ ID NO:5).

Specific core 7E11 amino acid residues of the interaction interface with ASGR-1 were defined as 7E11 residues that are within 5 Å of the ASGR-1 protein. The core 7E11 Heavy Chain residues include: S30, S31, I50, W52, H53, S56, N57, Y59, S01, M102, G103; and the core 7E11 Light Chain residues include: I30, Y32, T91, Y92, S93, T94, I96.

Boundary 7E11 amino acid residues of the interaction interface with ASGR-1 were defined as 7E11residues that are 5-8 Å from the ASGR-1 protein. The boundary 7E11 Heavy Chain residues include: T28, F29, F32, G33, H35, W47, I51, D54, K58, D99, L100, G104; and the boundary 7E11 Light Chain residues include: I2, Q27, N28, I29, S31, L33, N34, T50, S67, Q89, Q90, P95.

Methods:

The same methods were followed as described above in part B of this example except for the following changes: The 7E11 Fab fragment was generated by cleaving the 7E11 mAb with caspase 3:

7E11 mAb Heavy Chain (SEQ **ID** NO:32652):

QVQLVESGGGVVQPGRSLRLSCAASGFTFSSFGMHWVRQAPGKGLEWVAII
WHDGSNKYYADSVKGRFTISRDNSNNTLYLQMSSLRAEDTAVYYCARDLS
MGGMDVWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNH
KPSNTKVDKKVEPKSCGSDEVDGGDKTHTCPPCPAPELLGGPSVFLFPPKPKD
TLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGHHHHHH

7E11 mAb Light Chain (SEQ ID NO:32653):

DIQMTQSPSSLSASVGDRVTIACRASQNIISYLNWYQQKPGKAPKFLIYTASSL
QSGVPSRFSGSGSGTDFTLTISSLQPEDFAIYYCQQTYSTPLTFGGGTKVEIKRT
VAAPSVFIFPPSDEQLKSGTASVVCLLNNFYPREAKVQWKVDNALQSGNSQE

SVTEQDSKDSTYSLSSTLTLSKADYEKHKVYACEVTHQGLSSPVTKSFNRGE

C

7E11 Fab Heavy Chain (Post-Cleavage) (SEQ ID NO:32654):

QVQLVESGGGVVQPGRSLRLSCAASGFTFSSFGMHWVRQAPGKGLEWVAII
WHDGSNKYYADSVKGRFTISRDNSNNTLYLQMSSLRAEDTAVYYCARDLS
MGGMDVWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNH
KPSNTKVDKKVEPKSCGSDEVD

7E11 Fab Light Chain (Post-Cleavage) (SEQ ID NO:32655):

DIQMTQSPSSLSASVGDRVTIACRASQNIISYLNWYQQKPGKAPKFLIYTASSL
QSGVPSRFSGSGSGTDFTLTISSLQPEDFAIYYCQQTYSTPLTFGGGTKVEIKRT
VAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGE

C

1. The ASGR-1 CBD / 7E11 Fab complex was concentrated to 20 mg/ml and crystallized in 0.2 M Potassium Phosphate monobasic and 20% PEG3350;

2. The ASGR-1 CBD / 7E11 Fab complex crystals grow in the P6222 space group with unit cell dimensions a=105.75, b=105.75, c=193.75 Å and $\gamma$=120.0 ° with one complex molecule per asymmetric unit, and diffract to 2.0 Å resolution;

3. The dataset was processed with XDS/CCP4;

4. The ASGR-1 CBD / 7E11 Fab complex structure was solved by molecular replacement with the program Phaser; and

5. The structure was improved with multiple rounds of model building with Coot[3] and refinement with Phenix[4], to a final R=21.4 / R$_{free}$=26.9.

**E. Crystal Structure of ASGR-1 Carbohydrate Binding Domain (CBD) with 4H6**

The present example presents the crystal structure of the ASGR-1 CBD bound to the Fab fragment of 4H6, determined to 2.6 Å resolution (the conditions of which are described above in section B of this Example). This structure, depicted in FIGS. 29 and 30, shows that when 4H6 binds to/interacts with ASGR-1, a conformational rearrangement of the carbohydrate binding loop occurs, impairing the carbohydrate binding loop from binding to/interacting with ligand (i.e., carbohydrates). This demonstrates that the 4H6 Fab indirectly inhibits the ASGR-1 CBD/Ligand binding.

The depicted structure also allows one to identify specific core ASGR amino acid residues for the interaction interface of 4H6 with ASGR-1. This was defined as residues that are within 5 Å of the 4H6 protein. The core residues are as follows: H161, E162, T193, S194, W195, E196, K199, Q202, T231, G232, F233, K234, N235, P238, D261, R263 (SEQ ID NO:5).

The structures were also used to identify boundary ASGR-1 amino acid residues for the interaction interface with 4H6. These residues were ASGR-1 residues that were from 5-8 Å of the 4H6 protein. The boundary residues are as follows: R163, V192, E197, Q198, H203, P207, D228, E230, W236, R237, D260, G262, W264 (SEQ ID NO:5).

Specific core 4H6 amino acid residues of the interaction interface with ASGR-1 were defined as 4H6 residues that are within 5 Å of the ASGR-1 protein. The core 4H6 Heavy Chain residues include: Y33, H35, W50, H52, S55, G57, T58, N59, D99, G100, T101, S102; and the core 4H6 Light Chain residues include: Q27, W32, A91, N92, S93, F94, F96.

Boundary 4H6 amino acid residues of the interaction interface with ASGR-1 were defined as 4H6 residues that are 5-8 Å from the ASGR-1 protein. The boundary 4H6 Heavy Chain residues include: D31, Y32, L34, W47, I51, N54, G56, Y60, Q65, S103, F104; and the boundary 4H6 Light Chain residues include: D1, I2, G28, I29, S30, R31, Y49, G50, Q89, Q90, P95.

**Methods:**

The same methods were followed as described above in part B of this example except for the following changes:

1. The 4H6 Fab fragment was generated by cleaving the 4H6 mAb with caspase 3.

**4H6 mAb Heavy Chain (SEQ ID NO:32656):**

QVQLVQSGAEVKKPGASVKVSCKASGYTFTDYYLHWVRQAPGQGLEWMGWIHPN
SGGTNYAQKFQGRVTMTRDTSISTAYMGLSSLRSDDTAVYYCARDGTSSFDYWGQ
GTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCGSD
EVDGGDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSL
SLSPGHHHHHH

**4H6 mAb Light Chain (SEQ ID NO:32657):**

DIQMTQSPSSVSASVGDRVTITCRASQGISRWLAWYQQKPGKAPKLLIYGASSLQSG
VPSRFSGSGSGTDFTLTISSLQPEDFAIYYCQQANSFPFTFGPGTKVDIKGTVAAPSVFI
FPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC

4H6 Fab Heavy Chain (Post-Cleavage) (SEQ ID NO:32658):

QVQLVQSGAEVKKPGASVKVSCKASGYTFTDYYLHWVRQAPGQGLEWMGWIHPN
SGGTNYAQKFQGRVTMTRDTSISTAYMGLSSLRSDDTAVYYCARDGTSSFDYWGQ
GTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCGSD
EVD

4H6 Fab Light Chain (Post-Cleavage) (SEQ ID NO:32659):

DIQMTQSPSSVSASVGDRVTITCRASQGISRWLAWYQQKPGKAPKLLIYGASSLQSG
VPSRFSGSGSGTDFTLTISSLQPEDFAIYYCQQANSFPFTFGPGTKVDIKGTVAAPSVFI
FPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC

2. The ASGR-1 CBD / 4H6 Fab complex was concentrated to 20 mg/ml and crystallized in 0.2M Sodium fluoride, 0.1 M Bis Tris propane pH8.5, 20% PEG3350;

3. The dataset was collected on beamline ID22 at the APS synchrotron and processed with HKL2000/CCP4;

4. The ASGR-1 CBD / 4H6 Fab complex crystals grow in the $P12_11$ space group with unit cell dimensions a=57.20, b=43.58, c=131.65 Å and $\beta$=90.7° with one complex molecule per asymmetric unit, and diffract to 2.6 Å resolution;

5. The ASGR-1 CBD / 4H6 Fab complex structure was solved by molecular replacement with the program Phaser; and

6. The structure was improved with multiple rounds of model building with Coot[3] and refinement with Phenix[4], to a

final R=17.9 / $R_{free}$=22.5.

**F. Crystal Structure of ASGR-1 Carbohydrate Binding Domain (CBD) with 72G9**

The present example presents the crystal structure of the ASGR-1 CBD bound to the Fab fragment of 72G9, determined to 2.55 Å resolution (the conditions of which are described above in section B of this Example). This structure, depicted in FIGS. 31 and 32A and 32B, shows that when 72G9 binds to/interacts **with** ASGR-1, the CDR H2 loop of the Fab fragment appears to directly block the ligand (i.e., carbohydrate) binding/interacting to ASGR-1 CBD. This demonstrates that the 72G9 Fab directly inhibits the ASGR-1 CBD/Ligand binding.

The deicted structure also allows one to identify specific core ASGR amino acid residues for the interaction interface of 72G9 with ASGR-1. This was defined as residues that are within 5 Å of the 72G9 protein. The core residues are as follows: D216, Q217, N218, G219, P220, W221, Y229, E230, K234, W236, E239, Q240, P241, D242, D243, W244, Y245, G246, L249, G250, G251, G252, D254, Q270 ((SEQ ID NO:5).

The structures were also used to identify boundary ASGR-1 amino acid residues for the interaction interface with 72G9. These residues were ASGR-1 residues that were from 5-8 Å of the 72G9 protein. The boundary residues are as follows: H215, K222, T231, G232, R237, P238, H247, G248, E253, C255, D266, V268, C269 (SEQ ID NO:5).

Specific core 72G9 amino acid residues of the interaction interface with ASGR-1 were defined as 72G9 residues that are within 5 Å of the ASGR-1 protein. The core 72G9 Heavy Chain residues include: G26, F27, T28, S30, S31, Y32, S33, S52, G53, S54, S56, Y57, Y59, R98, G100, S101, R102; and the core 72G9 Light Chain residues include: Y32, Y49, T50, Q55, S91, H92, S93, F94, F96.

Boundary 72G9 amino acid residues of the interaction interface with ASGR-1 were defined as 72G9 residues that are 5-8 Å from the ASGR-1 protein. The boundary 72G9 Heavy Chain residues include: V2, F29, N35, S50, T51, S55, I58, R72, G99, G103, F104, D105 ; and the boundary 72G9 Light Chain residues include: S28, I29, T30, N33, L46, S53, L54, S56, Q89, Q90, P95.

**Methods:**

The same methods were followed as described above in part B of this example except for the following changes:

1. The 72G9 Fab fragment was generated by cleaving the 72G9 mAb with caspase 3.

**72G9 mAb Heavy Chain (SEQ ID NO:32660):**

EVQLVESGGGLVKPGGSLRLSCAASGFTFSSYSMNWVRQAPGKGLEWVSSISGSSSY
IYYADSVKGRFTISRDNAKNSLYLQMNSLRAEDTAVYFCARGGSRGFDPWGQGTLV
TVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCGSDEVDGG
DKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPI
EKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GHHHHHH

**72G9 mAb Light Chain (SEQ ID NO:32661):**

DIQMTQSPSSLSASVGDRVTITCRASQSITSYLNWYQQKPGKAPKLLIYTASSLQSGV
PSRFSGSGSGTDFTLTISSLQPEDFATYYCQQSHSFPFTFGPGTKVDIKRTVAAPSVFIF


PPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC

**72G9 Fab Heavy Chain (Post-Cleavage) (SEQ ID NO:32662):**

EVQLVESGGGLVKPGGSLRLSCAASGFTFSSYSMNWVRQAPGKGLEWVSSISGSSSY
IYYADSVKGRFTISRDNAKNSLYLQMNSLRAEDTAVYFCARGGSRGFDPWGQGTLV
TVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCGSDEVD

**72G9 Fab Light Chain (Post-Cleavage) (SEQ ID NO:32663):**

DIQMTQSPSSLSASVGDRVTITCRASQSITSYLNWYQQKPGKAPKLLIYTASSLQSGV
PSRFSGSGSGTDFTLTISSLQPEDFATYYCQQSHSFPFTFGPGTKVDIKRTVAAPSVFIF
PPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC

2. The 72G9 Fab/ASGR-1 CBD complex was concentrated to 0.2 M Magnesium Sulfate heptahydrate, 20% PEG3350;

3. The ASGR-1 CBD / 72G9 Fab complex crystals grew in the $P2_1$ space group with unit cell dimensions a=100.98, b=64.95, c=100.68 Å and $\beta$=96.43° with one complex molecule per asymmetric unit, and diffract to 2.55 Å resolution;

4. The dataset was processed with XDS/CCP4;

5. The ASGR-1 CBD / 72G9 Fab complex structure was solved by molecular replacement with the program Phaser; and

6. The structure was improved with multiple rounds of model building with Coot[3] and refinement with Phenix[4], to a final R=20.4 / $R_{free}$=23.4.

## G. Crystal Structure of ASGR-1 Carbohydrate Binding Domain (CBD) with 194A4

The present example presents the crystal structure of the ASGR-1 CBD bound to the Fab fragment of 194A4, determined to 2.6 Å resolution (the conditions of which are described above in section B of this Example). This structure, depicted in FIGS. 33 and 34, shows that when 194A4 binds to/interacts with ASGR-1, a conformational rearrangement of the carbohydrate binding loop occurs, impairing the carbohydrate binding loop from binding to/interacting with ligand (i.e., carbohydrates). This demonstrates that the 194A4 Fab indirectly inhibits the ASGR-1 CBD/Ligand binding.

The depicted structure also allows one to identify specific core ASGR amino acid residues for the interaction interface of 194A4 with ASGR-1. This was defined as residues that are within 5 Å of the 194A4 protein. The core residues are as follows: T193, S194, W195, E196, P220, W221, G226, T227, D228, Y229, E230, T231, G232, F233, K234, N235, W236, R237, P238, E239, G252 (SEQ ID NO:5).

The structures were also used to identify boundary ASGR-1 amino acid residues for the interaction interface with 194A4. These residues were ASGR-1 residues that were from 5-8 Å of the 194A4 protein. The boundary residues are as follows: H161, E162, V191, V192, E197, Q198, D216, G219, K222, W223, D225, R263, W264 (SEQ ID NO:5).

Specific core 194A4 amino acid residues of the interaction interface with ASGR-1 were defined as 194A4 residues that are within 5 Å of the ASGR-1 protein. The core 194A4 Heavy Chain residues include: V31, Y32, Y33, W50, N52, S55, G57, R98, G99, Y100, D101, 1102, T204; and the core 194A4 Light Chain residues include: V29, S30, I32, Y33, L47, Y50, R55, A56, T57, Y94.

Boundary 194A4 amino acid residues of the interaction interface with ASGR-1 were defined as 194A4 residues that are 5-8 Å from the ASGR-1 protein. The boundary 194A4 Heavy Chain residues include: V2, Y27, T30, L34, N35, P53, N54, G56, T58, N59, A97, L103, G105; and the boundary 194A4 Light Chain residues include: G28, N31, L48, I49, G51, N54, G58, I59, S68, G69, D93, S95.

## Methods:

The same methods were followed as described above in part B of this example except for the following changes:

1. The 194A4 Fab fragment was generated by cleaving the 194A4 mAb with caspase 3.

**194A4 mAb Heavy Chain (SEQ ID NO:32664):**

QVQLVQSGTEVKKPGASLKVSCKASGYTFTVYYLNWVRQAPGQGLEWMGWINPN
SGGTNYAQKFQGRVTMTRDTSISTAYMELSRLRSDDTAVYYCARGYDILTGWGQG
TLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCGSDE
VDGGDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSLSLSPG
HHHHHH

**194A4 mAb Light Chain (SEQ ID NO:32665):**

EIVLTQSPGTLSLSPGERATLSCRASRGVSNIYLAWYQQKPGQAPRLLIYGASNRATG
IPDRFSGSGSGTDFTLTISRLEPEDFAVYYCQHNDYSMFTFGPGTKVDIKRTVAAPSV
FIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

**194A4 Fab Heavy Chain (Post-Cleavage) (SEQ ID NO:32666):**

QVQLVQSGTEVKKPGASLKVSCKASGYTFTVYYLNWVRQAPGQGLEWMGWINPN
SGGTNYAQKFQGRVTMTRDTSISTAYMELSRLRSDDTAVYYCARGYDILTGWGQG
TLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCGSDE
VD

**194A4 Fab Light Chain (Post-Cleavage) (SEQ ID NO:32667):**

EIVLTQSPGTLSLSPGERATLSCRASRGVSNIYLAWYQQKPGQAPRLLIYGASNRATG
IPDRFSGSGSGTDFTLTISRLEPEDFAVYYCQHNDYSMFTFGPGTKVDIKRTVAAPSV
FIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

2. The 194A4 Fab/ASGR-1 CBD complex was concentrated to 13.1 mg/mL and crystallized with 0.2 M Sodium chloride, 0.1M MES pH6.0, 20% PEG2000 MME;

3. The dataset was processed with XDS/CCP4;

4. The 194A4 Fab/ASGR-1 CBD complex crystals grow in the $P2_12_12_1$ space group with unit cell dimensions a=52.23, b=66.40, c=177.75 Å with one complex molecule per asymmetric unit, and diffract to 2.6 Å resolution;

5. The ASGR-1 CBD / 194A4 Fab complex structure was solved by molecular replacement with the program Phaser; and

6. The structure was improved with multiple rounds of model building with Coot[3] and refinement with Phenix[4], to a final R=20.1 / $R_{free}$=24.6.

**H. Crystal Structure of ASGR-1 Carbohydrate Binding Domain (CBD) with 54E9**

The present example presents the crystal structure of the ASGR-1 CBD bound to the Fab fragment of 54E9, determined to 2.6 Å resolution (the conditions of which are described above in section B of this Example). This structure, depicted in FIG. 35 and FIG. 36A and FIG. 36B, shows that when 54E9 binds to/interacts with ASGR-1, the CDR H3 loop of the Fab fragment appears to directly block the ligand (i.e., carbohydrate) from binding/interacting to ASGR-1 CBD. This demonstrates that the 54E9 Fab directly inhibits the ASGR-1 CBD/Ligand binding.

The depicted structure also allows one to identify specific core ASGR amino acid residues for the interaction interface of 54E9 with ASGR-1. This was defined as residues that are within 5 Å of the 54E9 protein. The core residues are as follows: W195, N209, N235, R237, P238, E239, Q240, D242, H257, T259, D260, D261, R263, N265, D267, R271, Y273 (SEQ ID NO:5).

The structures were also used to identify boundary ASGR-1 amino acid residues for the interaction interface with 54E9. These residues were ASGR-1 residues that were from 5-8 Å of the 54E9 protein. The boundary residues are as follows: Q198, Q202, P207, V208, F233, W236, D243, E253, F258, G262, W264, D266 (SEQ ID NO:5).

Specific core 54E9 amino acid residues of the interaction interface with ASGR-1 were defined as 54E9 residues that are within 5 Å of the ASGR-1 protein. The core 54E9 Heavy Chain residues include: N30, S31, Y32, S52, Y54, N55, K59, R98, D100, F101, W102, S103, G104, Y105, K107, D 110; and the core 54E9 Light Chain residues include: none.

Boundary 54E9 amino acid residues of the interaction interface with ASGR-1 were defined as 54E9 residues that are 5-8 Å from the ASGR-1 protein. The boundary 54E9 Heavy Chain residues include: V2, Y27, T28, F29, G33, W50, A53, G56, N57, H99, Y106, G108; and the boundary 54E9 Light Chain residues include: N31, Y50, V51, Q54.

**Methods:**

The same methods were followed as described above in part B of this example except for the following changes:

1. The 54E9 Fab fragment was generated by cleaving the 54E9 mAb with caspase 3.

**54E9 mAb Heavy Chain (SEQ ID NO:32668):**

QVQLVQSGAEVKKPGASVKVSCKASGYTFNSYGISWVRLAPGQGLEWMGWISAYN
GNTKNAQKLQGRVTMTTDTSTSTAYMELRSLRSDDTAVYYCARHDFWSGYYKGM
DVWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEP
KSCGSDEVDGGDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHN
HYTQKSLSLSPGHHHHHH

**54E9 mAb Light Chain (SEQ ID NO: 32669):**

QSVLTQPPSASGTPGQRVTISCSGSNSNIGNNIVTWYQQLPGTAPKLLIYVNDQRPSG
VPDRFSGSKSGTSASLAISGLQSEDEADYYCAAWDDSLNGWVFGGGTTLTVLGQPK
AAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS

**54E9 Fab Heavy Chain (Post-Cleavage) (SEQ ID NO:32670):**

QVQLVQSGAEVKKPGASVKVSCKASGYTFNSYGISWVRLAPGQGLEWMGWISAYN
GNTKNAQKLQGRVTMTTDTSTSTAYMELRSLRSDDTAVYYCARHDFWSGYYKGM
DVWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEP
KSCGSDEVD

**54E9 Fab Light Chain (Post-Cleavage) (SEQ ID NO:32671):**

QSVLTQPPSASGTPGQRVTISCSGSNSNIGNNIVTWYQQLPGTAPKLLIYVNDQRPSG
VPDRFSGSKSGTSASLAISGLQSEDEADYYCAAWDDSLNGWVFGGGTTLTVLGQPK
AAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS

1. The 54E9 Fab/ASGR-1 CBD complex was concentrated to 14.8 mg/mL and crystallized with 0.2 M Magnesium Chloride hexahydrate, 20% PEG3350;

2. The dataset was processed with XDS/CCP4;

3. The 54E9 Fab/ASGR-1 CBD complex crystals grow in the I2 space group with unit cell dimensions a=64.66, b=41.65, c=224.59 Å and β=97.60° with one complex molecule per asymmetric unit, and diffract to 2.6 Å resolution;

4. The 54E9 Fab/ASGR-1 CBD complex structure was solved by molecular replacement with the program Phaser; and

5. The structure was improved with multiple rounds of model building with Coot[3] and refinement with Phenix[4], to a final R=19.1 / $R_{free}$=25.9

### I. Crystal Structure of ASGR-1 Carbohydrate Binding Domain (CBD) with 218G4

The present example presents the crystal structure of the ASGR-1 CBD bound to the Fab fragment of 218G4, determined to 2.4 Å resolution (the conditions of which are described above in section B of this Example). This structure, depicted in Figures 37 and 38, shows that when 218G4 binds to/interacts with ASGR-1, it impairs its ability to bind to ligand (e.g., carbohydrate). This demonstrates that the 218G4 Fab directly inhibits the ASGR-1 CBD/Ligand binding.

The depicted structure also allows one to identify specific core ASGR amino acid residues for the interaction interface of 218G4 with ASGR-1. This was defined as residues that are within 5 Å of the 218G4 protein. The core residues are as follows: R170, S171, G172, A174, H204, I205, G206, P207, V208, N209, H257, D260, N265, D267, Q270, R271, P272, Y273, R274 (SEQ ID NO:5).

The structures were also used to identify boundary ASGR-1 amino acid residues for the interaction interface with 218G4. These residues were ASGR-1 residues that were from 5-8 Å of the 218G4 protein. The boundary residues are as follows: W167, F168, S169, K173, W175, D177, Y181, Q202, H203, T210, W211, R237, F258, T259, D261, D266, V268, C269, W275 (SEQ ID NO:5).

Specific core 218G4 amino acid residues of the interaction interface with ASGR-1 were defined as 218G4 residues that are within 5 Å of the ASGR-1 protein. The core 218G4 Heavy Chain residues include: Q1, V2, F27, S30, S31, Y32, Y53, D54, W99, Y100, Y101, Y102; and the core 218G4 Light Chain residues include: Y33, Y50, D51, N53, K54, S57.

Boundary 218G4 amino acid residues of the interaction interface with ASGR-1 were defined as 218G4 residues that are 5-8 Å from the ASGR-1 protein. The boundary 218G4 Heavy Chain residues include: G26, T28, F29, G33, W52, G55, R72, N74, N98, Y103, Y104, D107, V108; and the boundary 218G4 Light Chain residues include: V34, S52, R55, P56, G58, G65.

The coordinates for the ASGR-1 CBD/GalNAc crystal structure complex are presented in Table 10.3.

#### Methods:

The same methods were followed as described above in part B of this example except for the following changes:

1. The 218G4 Fab fragment was generated by cleaving the 218G4 mAb with caspase 3.

**218G4 mAb Heavy Chain (SEQ ID NO:32672):**

QVQLVESGGGVVQPGRSLRLSCAASGFTFSSYGLHWVRQAPGKGLEWVAVIWYDGSNKY

YADSVKGRFTISRDNSKNTLYLQMNSLRVEDTAVYYCANWYYYYYGMDVWGQGTTVTV

SSASTKGPSVFPLAPSSKSTSGGTAALGCLVKDYFPEPVTVSWNSGALTSGVHTFPAVLQSS

GLYSLSSVVTVPSSSLGTQTYICNVNHKPSNTKVDKKVEPKSCGSDEVDGGDKTHTCPPCPA

PELLGGPSVFLFPPKPKDTLMISRTPEVTCVVVDVSHEDPEVKFNWYVDGVEVHNAKTKPR

EEQYNSTYRVVSVLTVLHQDWLNGKEYKCKVSNKALPAPIEKTISKAKGQPREPQVYTLPP

SREEMTKNQVSLTCLVKGFYPSDIAVEWESNGQPENNYKTTPPVLDSDGSFFLYSKLTVDK

SRWQQGNVFSCSVMHEALHNHYTQKSLSLSPGHHHHHH

**218G4 mAb Light Chain (SEQ ID NO: 32673):**

QSVLTQPPSVSAAPGQKVTISCSGSSSNIGNNYVSWYQQLPGTAPKLLLYDSNKRPSGIPARF

SGSKSGTSATLGITGLQTGDEADYYCGTWDSSLNTVVFGGGTKLTVLSQPKAAPSVTLFPPS

SEELQANKATLVCLISDFYPGAVTVAWKADSSPVKAGVETTTPSKQSNNKYAASSYLSLTP

EQWKSHRSYSCQVTHEGSTVEKTVAPTECS


**218G4 Fab Heavy Chain (Post-Cleavage)(SEQ ID NO: 32674):**


QVQLVESGGGVVQPGRSLRLSCAASGFTFSSYGLHWVRQAPGKGLEWVAVIWYDGSNKY

YADSVKGRFTISRDNSKNTLYLQMNSLRVEDTAVYYCANWYYYYYGMDVWGQGTTVTV

SSASTKGPSVFPLAPSSKSTSGGTAALGCLVKDYFPEPVTVSWNSGALTSGVHTFPAVLQSS

GLYSLSSVVTVPSSSLGTQTYICNVNHKPSNTKVDKKVEPKSCGSDEVD


**218G4 Fab Light Chain (Post-Cleavage):**

Same sequence as 218G4 mAb Light chain


1. The 218G4 Fab/ASGR-1 CBD complex was concentrated to 16.4 mg/mL and crystallized with 0.1M Tris pH8 and 1.6M Lithium Sulfate;

2. The dataset was collected from a single crystal on beamline ID22 at the Argonne National Laboratory and processed with XDS/CCP4;

3. The 218G4 Fab/ASGR-1 CBD complex crystals grow in the C222 space group with unit cell dimensions a=137.24, b=245.26, c=118.91 Å with two complex molecules per asymmetric unit and diffract to 2.6 Å resolution;

4. The 218G4 Fab/ASGR-1 CBD complex structure was solved by molecular replacement with the program Phaser; and

5. The structure was improved with multiple rounds of model building with Coot[3] and refinement with Phenix[4], to a final $R_{factor}$=18.4/ $R_{free}$=21.6


**J. Crystal Structure of ASGR-1 Carbohydrate Binding Domain (CBD) with 176H4**

The present example presents the crystal structure of the ASGR-1 CBD bound to the Fab fragment of 176H5, determined to 2.3 Å resolution (the conditions of which are described above in section B of this Example). This structure, depicted in Figures 39 and 40, show that when 176H4 binds to/interacts with ASGR-1, it appears to block ligand (e.g., carbohydrate) binding by ASGR-1 CBD, with the paratope of the 176H4 antibody located directly on top of the carbohydrate binding pocket. This demonstrates that the 174H4 Fab directly inhibits the ASGR-1 CBD/Ligand binding.

The depicted structure also allows one to identify specific core ASGR amino acid residues for the interaction interface of 176H4 with ASGR-1. This was defined as residues that are within 5 Å of the 176H4 protein. The core residues are as follows: R170, S171, G172, K173, A174, D177, P207, V208, N209, R237, Q240, W244, G246, H247, G248, L249, E253, H257, T259, D260, N265, D267, Q270, R271, P272, Y273, R274 (SEQ ID NO:5).

The structures were also used to identify boundary ASGR-1 amino acid residues for the interaction interface with 176H4. These residues were ASGR-1 residues that were from 5-8 Å of the 176H4 protein. The boundary residues are as follows: S169, W175, A176, A178, T210, W211, W236, P238, E239, D242, Y245, G250, G251, F258, D261, G262, R263, W264, D266, V268, C269, W275 (SEQ ID NO:5).

Specific core 176H4 amino acid residues of the interaction interface with ASGR-1 were defined as 176H4 residues that are within 5 Å of the ASGR-1 protein. The core 176H4 Heavy Chain residues include: S31, W52, Y53, D54, Y57, Y59, D102, F103, W104; and the core 176H4 Light Chain residues include: H31, G32, D33, G34, K35, Y37, I97, Q98, I99.

Boundary 176H4 amino acid residues of the interaction interface with ASGR-1 were defined as 176H4 residues that are 5-8 Å from the ASGR-1 protein. The boundary 176H4 Heavy Chain residues include: T28, S30, Y32, G33, W47, I50, I51, S56, K58, Y60, K65, D99, H101, S105, G106; and the boundary 176H4 Light Chain residues include: I2, Q27, S28, L29, L30, T36, E55, Q95, S96, P100, W101.

**Methods:**

The same methods were followed as described above in part B of this example except for the following changes:

1. The 176H4 Fab fragment was generated by cleaving the 176H4 mAb with caspase 3.

**176H4 mAb Heavy Chain (SEQ ID NO:32675):**

QVQLVESGGGVVQPGRSLRLSCAASGFTFSSYGMHWVRQAPGKGLEWVAIIWYDG
SYKYYADSVKGRFTISRDNSKNTLYLQMSSLRAEDTAVYYCARDAHDFWSGYFAY
WGQGALVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSC
GSDEVDGGDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNHYT
QKSLSLSPGHHHHHH

**176H4 mAb Light Chain (SEQ ID NO: 32676:**

DIVMTQTPLSLSVTPGQPASISCKSSQSLLHGDGKTYLYWYLQKPGQPPQLLIYEVSN
RFSGVPDRFSGSGSGTDFTLKISRVEAEDVGIYYCMQSIQIPWTFGQGTRVEIKRTVA

APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

**176H4 Fab Heavy Chain (Post-Cleavage)(SEQ ID NO:32677):**

QVQLVESGGGVVQPGRSLRLSCAASGFTFSSYGMHWVRQAPGKGLEWVAIIWYDG
SYKYYADSVKGRFTISRDNSKNTLYLQMSSLRAEDTAVYYCARDAHDFWSGYFAY
WGQGALVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSC
GSDEVD

176H4 Fab Light Chain (Post-Cleavage):
Same sequence as 176H4 mAb Light chain

1. The 176H4 Fab/ASGR-1 CBD complex was concentrated to 14.9 mg/mL and crystallized 1with 0.2 M Sodium Nitrate, 20% PEG3350;
2. The dataset was collected from a single crystal on beamline ID22 at the Argonne National Laboratory and processed with XDS/CCP4;
3. The 176H4 Fab/ASGR-1 CBD complex crystals grow in the 1121 space group with unit cell dimensions a=68.31, b=126.31, c=134.13 Å and $\beta$=101.6° with two complex molecules per asymmetric unit, and diffract to 2.3 Å resolution;
4. The 176H4 Fab/ASGR-1 CBD complex structure was solved by molecular replacement with the program Phaser; and
5. The structure was improved with multiple rounds of model building with Coot[3] and refinement with Phenix[4], to a final $R_{factor}$=17.9 / $R_{free}$=23.3

**K. Crystal Structure of ASGR-1 Carbohydrate Binding Domain (CBD) with 194C10**

The present example presents the crystal structure of the ASGR-1 CBD bound to the Fab fragment of 194C10, determined to 2.6 Å resolution (the conditions of which are described above in section B of this Example). This structure, depicted in Figures 41 and 42, shows that when 194C10 binds to/interacts with ASGR-1, it likely induces a conformational rearrangement of the carbohydrate binding loop, impairing ASGR-1 CBD from binding to ligand (e.g., carbohydrate), as well as possibly blocking the ligand (e.g., carbohydrate) binding by ASGR-1 CBD, with the paratope of the 194C10 Fab. These data indicate that the 174H4 Fab may directly and/or indirectly inhibit the ASGR-1 CBD/Ligand binding.

The depicted structure also allows one to identify specific core ASGR amino acid residues for the interaction interface of 194C10 with ASGR-1. This was defined as residues that are within 5 Å of the 194C10 protein. The core residues are as follows: N157, R170, S171, G172, Q202, H203, H204, 1205, G206, P207, V208, N209, T210, D260, R271, P272, Y273, R274 (SEQ ID NO:5).

The structures were also used to identify boundary ASGR-1 amino acid residues for the interaction interface with 194C10. These residues were ASGR-1 residues that were from 5-8 Å of the 194C10 protein. The boundary residues are as follows: V156, W158, V159, H161, W167, F168, S169, K173, K199, F200, V201, W211, R237, H257, F258, T259, D261, D267, V268, Q270, W275 (SEQ ID NO:5).

Specific core 194C10 amino acid residues of the interaction interface with ASGR-1 were defined as 194C10 residues that are within 5 Å of the ASGR-1 protein. The core 194C10 Heavy Chain residues include: R30, Y31, Y33, E50, S54, S56, N58, D98, Y99, G100; and the core 194C10 Light Chain residues include: N30, S31, Y33, F50, S54, S68, Y92, E93, W97.

Boundary 194C10 amino acid residues of the interaction interface with ASGR-1 were defined as 194C10 residues that are 5-8 Å from the ASGR-1 protein. The boundary 194C10 Heavy Chain residues include: S28, Y32, W34, S35, W47, G49, I51, S52, H53, G55, T57, R97, A101, F102, D103; and the boundary 194C10 Light Chain residues include: S28, V29, G32, L47, G51, A52, S53, R55, A56, G69, Q90, Q91, S94, S95.

The coordinates for the ASGR-1 CBD/GalNAc crystal structure complex are presented in Table 10.4.

**Methods:**

The same methods were followed as described above in part B of this example except for the following changes:

1. 194C10 Fab fragment was generated by cleaving the 194C10 mAb with caspase 3.

194C10 mAb Heavy Chain (SEQ ID NO:32678):

QVQLQQWGAGLLKPSETLSLTCAVSGGSFRYYYWSWIRQPPGKGLEWFGEI
NHAGSTNYNPSLKSRVTISIDTSKNQFSLKLRSVTAADTAVYYCARDYGAFDIWGQG
TMVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCG

194C10 mAb Light Chain (SEQ ID NO:32679):

EIVLTQSPGTLSLSPGERATLSCRASPSVNSGYLAWYQQKPGQTPRLLIFGASSRATGI
PDRFSASGSGADFTLTISRLEPEDFAVYFCQQYESSPWTFGQGTKVEIKRTVAAPSVFI
FPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC*

194C10 Fab Heavy Chain (Post-Cleavage)(SEQ ID NO:32680):

QVQLQQWGAGLLKPSETLSLTCAVSGGSFRYYYWSWIRQPPGKGLEWFGEINHAGS
TNYNPSLKSRVTISIDTSKNQFSLKLRSVTAADTAVYYCARDYGAFDIWGQGTMVT
VSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCGSDEVD

194C10 Fab Light Chain (Post-Cleavage):

Same sequence as 194C10 mAb Light chain

1. The 194C10 Fab/ASGR-1 CBD complex was concentrated to 13.6 mg/mL and crystallized with 0.2 M Ammonium Sulfate, 0.1 M Tris pH7.5, 20% PEG5000MME;
2. The dataset was collected from a single crystal on beamline ID22 at the Argonne National Laboratory and processed with XDS/CCP4;

3. The 194C10 Fab/ASGR-1 CBD complex crystals grow in the $P12_11$ space group with unit cell dimensions a=65.62, b=130.44, c=85.93 Å and β=111.6 ° with two complex molecules per asymmetric unit, and diffract to 2.6 Å resolution.;

4. The 194C10 Fab/ASGR-1 CBD complex structure was solved by molecular replacement with the program Phaser; and

5. The structure was improved with multiple rounds of model building with Coot[3] and refinement with Phenix[4], to a final $R_{factor}$=17.1 / $R_{free}$=22.8.

### L. Interaction between GalNAc, ASGR-1 and certain Antibodies

The structure of the 72G9/ASGR-1 complex (Item G above) was overlaid on the ASGR-1/ligand (GalNac) structure (Item A above) and the result of this combination is depicted in Figure 31B. The structure of the 54E9/ASGR-1 complex (Item I above) was also overlaid on the ASGR-1/ligand (GalNac) structure (Item A above) and the result of this combination is depicted in Figure 35B. The structure of the 218G4/ASGR-1 complex (Item J above) was overlaid on the ASGR-1/ligand (GalNac) structure (Item A above) and the result of this combination is depicted in Figure 38. The structure of the 176H4/ASGR-1 complex (Item K above) was overlaid on the ASGR-1/ligand (GalNac) structure (Item A above) and the result of this combination is depicted in Figure 40. These figures demonstrate areas on ASGR-1 which can be usefully targeted to inhibit ASGR-1 interaction with a ligand, e.g., GalNac. These figures show that 72G9, 54E9, 218G4 and 176H4 directly interact with a subset of amino acid residues that are specifically involved in binding to the ligand (e.g., GalNAc).

As noted above, analysis of the crystal structures identified specific amino acids involved in the interaction between ASGR-1 and the partner proteins (the core and boundary regions of the interface on the ASGR-1 surface) and the spatial requirements of these partner proteins to interact with ASGR-1. The structures suggest ways to inhibit the interaction between ASGR-1 and a ligand, GalNAc. First, as noted above, binding an agent to ASGR-1 where it shares residues in common with the binding site of a ligand such as GalNAc would inhibit the interaction between ASGR-1 and the ligand. Second, an agent that binds outside of the residues in common can sterically interfere with the ligand that are either N- or C-terminal to the ligand to prevent the interaction between ASGR-1 and a ligand.

In some embodiments, the residues that are involved in both ligand binding and are close to the areas where the above noted antigen binding proteins bind are especially useful for manipulating ASGR-1binding to ligand. For example, amino acid residues from interfaces in common in both the core region and boundary region for the different binding partners are listed in Table 10.5 below.

Table 10.5

| Parameters | Amino acid position(s) |
|---|---|
| 72G9 /GalNAc both under 5 Å | Q240, D242, W244 |
| 72G9 under 5 Å / GalNAc 5-8 Å | E239, P241, D243, Y245, G246, G252 |
| 72G9 at 5-8 Å / GalNAc under 5 Å | R237, E253 |
| 72G9 / GalNAc both at 5-8 Å | P238, H247, C255, V268 |
| 54E9 / GalNAc both under 5 Å | N209, R237, Q240, D242, H257, T259, N265, D267, Y273 |
| 54E9 under 5 Å / GalNAc 5-8 Å | P238, E239, D260, R263, R271 |
| 54E9 at 5-8 Å / GalNAc under 5 Å | E253, D266 |
| 54E9 / GalNAc both at 5-8 Å | D243, F258, W264 |
| 218G4 / GalNAc both under 5 Å | N209, H257, N265, D267, Y273 |
| 218G4 under 5 Å / GalNAc 5-8 Å | D260, R271 |
| 218G4 at 5-8 Å / GalNAc under 5 Å | R237, T259, D266 |
| 218G4 / GalNAc both at 5-8 Å | F258, V268 |
| 176H4 / GalNAc both under 5 Å | N209, R237, Q240, W244, E253, H257, T259, N265, D267, Y273 |
| 176H4 under 5 Å / GalNAc 5-8 Å | G246, H247, D260, R271 |
| 176H4 at 5-8 Å / GalNAc under 5 Å | D266 |
| 176H4 / GalNAc both at 5-8 Å | P238, E239, Y245, F258, R263, W264, V268 |

As will be appreciated by one of skill in the art, in some embodiments, the antigen binding proteins bind to and/or block at least one of the above noted residues.

Antigen binding proteins and molecules that interact with the relevant areas or residues of the structure of ASGR-1 (including those areas or residues within 15, 15-8, 8, 8-5, 5, or fewer angstroms from where ligands, such as GalNAc, or the antibodies, interact with ASGR-1) depicted in the figures (e.g., Figures 19-42) and/or their corresponding positions on the structures from the coordinates are also contemplated.

**Example 11: Determination of the Binding Affinity of ASGR-1 Specific Antibodies**

To quantitate the binding affinity of specific antibodies for ASGR-1 (either purified from hybridoma supernatants or made recombinantly), association and dissociation rates can be measured using a ForteBio Octet instrument. The antibodies were covalently coupled to AR2G tips to load levels close to 2 nm and then bound to the soluble human ASGR-1 carbohydrate binding domain (CBD; amino acid residues 154-281; N-terminal 6xHis tag) in a 3-fold serial dilution series starting typically at 30 nM with either 3-point or 6-point dilution series. Experimental kinetic results were globally fit to a 1:1 binding model in order to determine the association and dissociation rate constants as well as the equilibrium dissociation constant. Association and dissociation times were chosen to ensure that curvature was present during association curves and measured dissociation levels dropped at least 5% from starting levels. All Octet buffers contained 10 mM Tris (pH7.5), 150 mM NaCl, 1 mM CaClz, 0.10 mg/ml BSA and 0.13% Triton X-100. Octet assays were run at 27°C. Because this assay only measures binding to the ASGR-1 CBD, antibodies that recognize epitopes partially or entirely outside the CBD and/or recognize ASGR-1 in the context of a native ASGR complex, for example, as could occur on cell membranes, may not score as positive in this assay. Data provided for representative antibodies in TABLE 11.1.

## Table 11.1:

| Ab name | Octet binding $K_D$ (nM) | Ab name | Octet binding $K_D$ (nM) |
|---|---|---|---|
| 4H6 | 4.8 | 194A4 | 0.7 |
| 4B1 | >30 | 194C1 | 1.3 |
| 4A2 | 0.06 | 194C10 | 4 |
| 5E5 | 7.6 | 197G3 | 0.8 |
| 6G7 | 2.0 | 198D2 | >30 |
| 7G4 | 0.9 | 198G3 | 0.04 |
| 7F4 | 1.2 | 202A3 | >30 |
| 7E11 | 1.6 | 218G4 | 2.6 |
| 12D2 | >30 | 4A2.001 | 0.06 |
| 22G5 | 1.4 | 4A2.001.003 | 0.04 |
| 25A4 | 0.03 | 4A2.001.004 | 0.03 |
| 26C4 | 0.4 | 4A2.001.005 | 0.02 |
| 29H8 | 1.0 | 4A2.001.010 | 0.04 |
| 48B12 | 0.3 | 4A2.001.012 | 0.04 |
| 54E9 | >30 | 25A4.001 | 0.06 |
| 56E5 | 0.5 | 25A4.001.021 | 0.04 |
| 72G9 | 0.5 | 4H6.009 | 0.28 |
| 75G3 | 1.0 | 7E11.001 | 0.71 |
| 176H4 | 0.8 | 7E11.001.005 | 0.42 |
| 184E7 | 0.3 | 7E11.001.007 | 0.62 |
| 190F8 | 0.6 | 5E5.016 | 1.46 |
| 191G1 | 2.4 | 5E5.019 | 1.80 |
| 191G10 | 0.5 | 5E5.005 | 2.00 |
| 193E7 | 3.5 | | |

## Example 12: CHO-S:huASGR-1 Cell Binding Assay

CHO-S stable high-expressing cell line were developed for both human ASGR-1 as well as mouse ASGR-1. A typical 384 well plate multiplex flow cytometery-based cell binding method is described as followed: Parental CHO-S cells and CHO-S:huASGR-1 cells were respectively labeled using a CellTrace CFSE Cell Proliferation Kit (ThermoFisher Catalog #C34554) and CellTrace Violet Cell Proliferation Kit (ThermoFisher Catalog#C34557) CHO-S:muASGR-1 were not labeled. 20 ul of cells at 4C were added to duplicate wells of the 384 well plate. The cells were equally mixed from all three cell lines (30K cells/well). Then 20 ul of the ASGR-1 antibodies (either purified from hybridoma supernatants or made recombinantly) were added in an 11-point dose response using a 1:2 fold serial dilution starting at 100 nM.. The cells and antibodies were incubated for 30 min at 4C and then spun down and washed twice with FACS buffer containing 1mM CaCl2. 30 ul of anti-huIgG-APC secondary antibodies were then added at a 1:1000 dilution ) for 30 min at 4C and then washed once with the same buffer. 60ul of PI (1:1000) was added and then the cells were read by a core flow cytometry facility. The cells were gated first for live cells, then for single cells and finally for the cell dyes to separate the mixed cells into the three different cell populations. Histograms of signal vs count representing the binding profile of each antibody at each antibody concentration were automatically analyzed for the median of the binding signal and then a binding graph was made with log 10 antibody concentration in nM on the X axis with standard deviation of the median signals from the duplicate wells on the Y-axis. The binding curves were fit with a standard four parameter sigmoidal binding curve and EC50's reported for all graphs with full curves. Data provided for representative antibodies in TABLE12.1.

**Table 12.1:**

| Ab name | Cell binding EC50 (nM) | Ab name | Cell binding EC50 (nM) |
|---|---|---|---|
| 4H6 | 1.70 | 56E5 | 1.1 |
| 4B1 | 4.1 | 72G9 | 0.41 |
| 4A2 | 0.82 | 75G3 | 1 |
| 4A2.001 | 1.8 | 176H4 | 1 |
| 5E5 | 3.80 | 184E7 | 1 |
| 6G7 | 0.6 | 190F8 | 9 |
| 7G4 | 0.69 | 191G1 | 0.16 |
| 7F4 | 5.40 | 191G10 | 0.31 |
| 7E11 | 1.40 | 193E7 | 0.13 |
| 7E11.001 | 3.2 | 194A4 | 25 |
| 12D2 | 3.2 | 194C1 | 0.11 |
| 22G5 | 7.2 | 194C10 | 0.56 |
| 25A4 | 1.6 | 197G3 | 0.25 |
| 25A4.001 | 1.2 | 198D2 | 0.14 |
| 26C4 | 11 | 198G3 | 0.21 |
| 29H8 | 1.9 | 202A3 | 0.8 |
| 48B12 | 38 | 218G4 | 2.2 |
| 54E9 | 5 | | |

For human ASGR-2, CHO-S stable cells expressing C-terminal His-tagged human ASGR-2 were resuspended in cold flow buffer (10mM Tris, pH 7.5, 137mM NaCl, 1mM CaCl2 and 2%fetal bovine serum) and 1.5x10e6 cells per well were added to a 96-well, v-bottom plate in a volume of 80 ul. 80 ul of antibody at 400 nM was then added to each well. After incubation on ice for 30 min, the cells were centrifuged at 1400 rpm for 3 min and then washed twice in cold flow buffer. The cells were then resuspended in 120 ul of anti-human IgG-APC (diluted 1:1000 in flow buffer) and incubated on ice for 30 minutes, centrifuged and washed twice as before, and resuspended in 200ul cold flow buffer, and then analyzed on a BD-LSR II flow cytometer. Data provided for antibody 7F4 in Figure 43.

**Example 13: CHO-S:huASGR-1 Ligand Blocking Assay**

All ASGR-1 antibodies that bound either human or mouse ASGR-1 stable CHO-S cells were then tested for ligand blocking using both a protein ligand and a synthetic sugar ligand. The method in brief is as follows: first, 20 ul of either CHO-Shuman or mouse ASGR-1 cells were added to wells of a 384 well plate (30k cells/well) followed by spin and discarding the supernatant. Second, 10 ul of the antibodies (either purified from hybridoma supernatants or made recombinantly) were added in duplicate to the cells in a dilution series (200 nM top concentration, 1:2 serial dilution, 11 point curve) and were incubated for 30 min at 4C. Third, 10 ul of the minimally biotinylated ligands were added at 2x their binding EC05, so that the wells contained a final 20 ul volume with Ab starting at 100 nM and the ligand at their EC50. After 30 min incubation at 4C, the plate was spun and washed twice with FACS buffer + 1 mM CaCl2 followed by the detection streptavidin-AF647 at 1:1000 dilution. After 30min at 4C, the cells were spun and washed once and then 60 ul PI added at 1:1000 dilution and the plates delivered to a core flow cytometry facility. The plates were read and processed similarly to the cell binding method except the signal now represents an inhibition curve and typically decreases a function of increasing antibody concentration. IC50 nM potency and % Inhibition were reported. The desialylated, biotinylated asialofetuin (see Example 9A) and biotinylated GALNAc-PAA (Fisher #NC9024754) were used as ligands with measured binding EC50s of 10.7 and 5.4 nM. Differences in the ability of antibodies to block these two ligands could occur as a result of differences in, for example, avidity stemming from differences in the number and/or orientation of the ASGR binding terminal sugar residues of each ligand, steric hindrance between antibody and each ligand, and/or changes in the conformation of ASGR induced by antibody binding that selectively alters the binding of each ligand. Data provided for representative antibodies in TABLE 13.1.

**Table 13.1:**

| | Ligand Blocking | | | |
| | bn-GalNAc-PAA | | bn-asialofetuin | |
| Ab name | IC50 (nM) | % Inhibition | IC50 (nM) | % Inhibition |
|---|---|---|---|---|
| 4H6 | 8.1 | 20% | 12 | 85% |
| 4B1 | 42 | 36% | 64 | 75% |
| 4A2 | 54 | 70% | 11 | 99% |
| 4A2.001 | 28 | 75 | 12 | 99 |
| 5E5 | >200 | 0% | 16 | 95% |
| 6G7 | >200 | 0% | 11 | 99% |
| 7G4 | 20 | -30% | 14 | 96% |
| 7F4 | 0.24 | 30% | 2.6 | 99% |
| 7E11 | 40 | 37% | 13 | 99% |
| 7E11.001 | >100 | 50 | 13 | 99 |
| 12D2 | 2.1 | 10% | 10 | 20% |
| 22G5 | 11 | 93% | 3.4 | 99% |
| 25A4 | 40 | 77% | 11 | 99% |
| 25A4.001 | 31 | 68 | 8.1 | 99 |
| 26C4 | 36 | 83% | 6.6 | 99% |
| 29H8 | 17 | 99% | 7 | 99% |
| 48B12 | 86 | 94% | 19 | 99% |
| 54E9 | 100 | 19% | 50 | 75% |
| 56E5 | 45 | 99% | 23 | 99% |
| 72G9 | 24 | 20% | 53 | 20% |
| 75G3 | 115 | 99% | 29 | 99% |
| 176H4 | 73 | 79% | 59 | 99% |
| 184E7 | 10 | 99% | 23 | 99% |
| 190F8 | 44 | 83% | 34 | 98% |
| 191G1 | 62 | 78% | 24 | 99% |
| 191010 | 56 | 99% | 27 | 99% |
| 193E7 | 33 | 60% | 30 | 99% |
| 194A4 | 48 | 60% | 57 | 99% |
| 194C1 | 72 | 89% | 34 | 99% |
| 194C10 | 87 | 99% | 30 | 99% |
| 197G3 | 15 | 74% | 29 | 90% |
| 198D2 | 55 | 99% | 22 | 99% |
| 198G3 | 5 | 81% | 26 | 99% |
| 202A3 | 32 | 96% | 16 | 98% |
| 218G4 | 71 | 99% | 28 | 99% |

**Example 14: ASGR-1** Specific **Antibody Optimization (Chemical Degradation** Site **Engineering:**

Variable domain sequence motifs having a high risk of sidechain degradation were engineered out of ASGR-1 specific antibodies. See for example, ASGR-1 specific antibody sequences in Tables 6 and 7.

Certain high risk motifs included: (1) CDR 'NG' and 'NT' sequences prone to asparagine deamidation, (2) CDR 'DG,' 'DH,' 'DS,' and 'DT' sequences prone to aspartic acid isomerization, (3) and CDR3 tryptohphans prone to oxidation. Bioinformatics and structural analyses were used to identify substitutions likely to retain binding affinity to the ASGR-1 CBD. Typically, substitution identities were derived from germline sequences or from sequence-related ASGR-1 CBD-binding mAbs. These substitutions were then modeled into a homology model of the unbound mAb using the software MOE (CCG)[1] to predict structural fitness. For cases in which the bioinformatics or structural analyses did not provide a clear substitution identity, residue types chemically similar to the parent residue were identified.

Variable domain sequence motifs violating multiple sequence alignment-based pair-wise residue covariance trends[2] were also engineered out of ASGR-1 specific antibodies. Substitution identities for covariance violators were identified using a hybrid bioinformatics/structural approach similar to that used to remediate degradation sites.

1. Molecular Operating Environment (MOE), 2013.08; Chemical Computing Group, Inc., 1010 Sherbooke St. West, Suit #910, Montreal, QC, Canada, H3A 2R7, 2016.
2. Kannan, G. Method of Correlated Mutational Analysis to Improve Therapeutic Antibodies. US Patent Application PCT/US2012/028596 filed March 9, 2012.

**Example 15: Epitope Mapping Using Peptide Arrays**

Custom peptide microarrays were obtained commercially (PEPperPRINT GmbH). For epitope mapping using linear arrays, the antigen (ASGR-1) was translated into 291 different overlapping 15 amino acid (aa) peptides printed in duplicate (582 peptide spots per array copy). For epitope mapping using cyclized arrays, the antigen (ASGR-1) was translated into 888 different overlapping 7 aa, 10 aa and 13 aa peptides printed in duplicate (1,776 peptide spots per array copy). Peptide cyclization was accomplished using N- to C-terminal thioether formation with and without an additional scaffold for varying conformational restriction. Each PEPperCHIP® Peptide array is framed by Flag (DYKDDDDKAS) and HA (YPYDVPDYAG) control peptides. Assay buffer was PBS-T (PBS, pH7.4, 0.05% Tween 20), blocking buffer was Rockland Blocking Buffer (Rockland Immunochemicals), staining buffer was assay buffer + 10% Rockland Blocking buffer. Secondary antibody was goat anti-human IRDye680LT (Li-Cor). Control antibodies were anti-FLAG M2 DyLight800, anti-HA DyLight680. Arrays were scanned on Li-Cor Odyssey with an offset of 0.65mm, 21um resolution.

Array staining and detection was per manufacturer's instructions. Briefly, arrays were pre-stained with secondary antibodies for 30 minutes, washed and scanned to detect background binding. Arrays were then stained with commercially available primary antibodies overnight, followed by washing and 30 minute incubation with labeled secondary antibodies. Arrays were scanned to detect binding of anti-ASGR-1 antibodies. Finally, arrays were stained with control antibodies for 45 minutes prior to washing and scanning to detect control peptides.

Antigen binding proteins with desired binding properties can be identified using this assay.

Example 16: *In vivo* **studies**

RNAi constructs that reduce expression of ASGR-1 and/or ASGR-2 and/or antigen binding proteins, such as monoclonal antibodies, that inhibit ligand binding to ASGR, ASGR-1, and/or ASGR-2 *in vitro* can be administered *in vivo* to a relevant animal model and levels and/or activity of endogenous blood proteins like alkaline phosphatase measured. In addition, the clearance of exogenously administered ASGR ligands (for example asialoglycoproteins, certain non-asialylated proteins, synthetic ligands, etc.) can be inhibited by pre-treatment with RNAi or a co- or pre-administered antibody.

Additionally, physiologic effects of the antigen binding proteins or RNAi can be evaluated in relevant animal models of cardiovascular disease using readouts including blood pressure, primary and secondary hemostasis, heart function and morphology, endothelial function, LDL cholesterol levels, non-HDL cholesterol levels, inflammation, and atherosclerosis.

**Example 17: Effect of ASGR-1 antibody 4A2 on serum LDL cholesterol and alkaline phosphatase in normal and obese cynomolgus monkeys**

The purpose of the study was to evaluate the LDL cholesterol (LDL-C)-lowering activity of anti-ASGR inhibitors. In general, cynomolgus monkeys do not have high levels of total cholesterol, HDL-C or LDL-C. Therefore, both normal and dyslipidemic models were utilized in this example. In the dyslipidemic model, monkeys were selected if their LDL levels were at least 100mg/dL (normal is 40-60mg/dL), and if there body mass index was over 41 kg/m$^2$ (normal is below 35 kg/m$^2$). Animals that met these criterea on standard diet were classified as spontaneously obese dylipidemic. Other animals were fed a high-fat diet (HFD; 4.15 kcal/gm, 32% fat) prior to inclusion in the study and were classified as HFD obese dyslipidemic.

Naive male spontaneous obese dyslipidemic and HFD obese dyslipidemic cynolgous monkeys were given a single subcutaneous injection of anti-ASGR-1 antibody 4A2.001 (IgG1z-SEFL2) (10 mg/kg in 10 mM sodium acetate, 9% sucrose, 0.01% polysorbate-80, pH 5.2). Naive male and female normal cynomolgus monkeys were given a single intravenous injection of anti-ASGR-1 antibody 4A2.001 (IgG1z-SEFL2)(100 mg/kg in 10 mM sodium acetate, 9% sucrose, 0.01% polysorbate-80, pH 5.2). Blood was collected from overnight fasted animals to monitor LDL-C and alkaline phosphatase (ALP) levels post-antibody injection. Blood was collected 70, 118, 190 and 268 hours post-injection (dyslipidemic models) and at 0.05, 0.25, 0.5, 1, 4, 8, 24, 48, 72, 168, 240, 336, 504, 672, 840, 1008, and 1176 hours post-injection (normal). LDL-C decrease (%) and ALP increase (%) were the main endpoints of the study and were measured on Roche C311 and C501 chemistry analyzers. Baseline levels of LDL-C and ALP were established from blood collected 7 days prior to antibody administration.

Dyslipidemic model:

| | |
|---|---|
| Species: | *Macaca fascicularis* |
| Weight Range: | >7.0 kg |
| BMI Range: | >41 kg/m$^2$ |
| Age range: | 12-17 years |
| Time on HFD: | 6 months |
| Source: | KBI monkey colony |
| Number and Sex: | 3 male spontaneous obese monkeys and 3 male HFD induced obese monkeys (BMI>41, LDL>80 mg/dL)). Animals were selected from a larger pool based on similar baseline LDL and ALP levels |

Normal model:

| | |
|---|---|
| Species: | *Macaca fascicularis* |
| Weight Range: | 2.6-4.2 kg |
| Age range: | 2.5-4 years |
| Number and Sex: | 2 male and 1 female fed normal laboratory diet |

Data for this study is provided in Figure 44 (dyslipidemic model) and Figure 45 (normal model).

### Example 18: Proteomic Profiling of Serum Samples from Human ASGR1 Carriers and Controls

Introduction

As described above in Example 1, ASGR1 loss-of-function (LOF) was found to be associated with a beneficial phenotype (protected from coronary artery disease, lower LDL cholesterol and longer life span) in human. [1]. To understand the mechanism of action underlying this association and find potential biomarkers, proteomic measurement of human serum samples were performed and compared to changes in circulating protein levels between the ASGR1 LOF variant carriers and controls.

**Materials and methods**

Sample collection and Proteomic Profiling

A total of 333 human serum samples were acquired from the deCODE Icelandic population study, including 100 ASGR1 del12 heterozygous carriers (cases group) and 233 non-carriers (controls group). The Case/Control Groups are well matched by sex, age and collection time/ freezer storage time. 150ul serum samples were shipped to SomaLogic Inc, where 1310 proteins were measured by the SOMAscan Assay 1.3k. The 1310 proteins were SOMAmer® Reagents Generated to Human Proteins, the complete list of tested proteins are summarized in the SOMAscan Assay 1.3K Content, Rev 1 (Effective: 9/21/2015) which is incorpored by reference herein in its entirety.

The SOMAscan assay measured serum protein concentration using a Slow Off-rate Modified DNA Aptamer (SOMAmer)-based capture array[2]. Each of the 1310 proteins is bond by its respective fluorescently labeled SOMAmer in the assay and their concentrations are reflected by the respective SOMAmer's relative fluorescence units (RFU).

## Data analysis

2 Samples were removed due to low volume that did not meet Somascan requirements and 13 samples were removed because they had been treated with EDTA. The RFU data of each measured protein was log transformed, then centered and scaled to calculate standardized RFU values for this protein. Principle components (PCs) were derived from 1310 standardized RFU values by principle components analysis. An outlier removal based on Hotellings T2 distribution of PC1 and PC2 was applied and excluded another 8 samples from further analysis.

After QC, the remaining 93 ASGR1 Del12 heterozygous Carriers (cases group) and 217 samples without the Del12 allele (controls group) and their standardized RFU values of each protein were analyzed by a linear model adjusting for Age, Sex, FreezerTime and the first 10 PCs,

$$Yi = \beta0 + \beta1Gi + \beta2AGEi + \beta3SEXi + \beta4FTi + \beta5PC1i + \cdots + \beta15PC10i + \varepsilon i$$

where $Yi$ is the standardized RFU value for the *i th* sample for a particular protein, $Gi$ is the Del12 genotype the *i th* sample and $\beta1$ capture the estimates of the mean difference between human samples with Del12 and without Del12. Since 1310 tests were performed for the proteins on Somascan platform, we calculated the significant threshold by Bonferroni method ($0.05/1310 = 3.82 \times 10^{-5}$) assuming these are independent tests. However, the Bonferroni correction is likely too stringent because proteins are often correlated with each other therefore these tests are not independent. Thus a realistic threshold of significance ($5.19 \times 10^{-5}$) was obtained by performing 100,000 permutations using the method by Sham and Purcell 2014[3].

## Results and Discussion

Using the permutation threshold, 41 Proteins were identified to have significant serum levels between human ASGR1 del12 carriers and non-carriers ($P < 5.19 \times 10^{-5}$). Of those, 26 show significant increase in the carriers (Table 18.1) and 15 decrease significantly in the carriers (Table 18.2). These changes are likely to mediate the beneficial effects resulting from ASGR1 loss of function seen in the del12 carriers. The levels of these proteins in blood can serve as biomarkers for ASGR1 loss of function and be used to assess ASGR1-targeted therapy during drug development.

**Table 18.1. Proteins with significant increase in serum of ASGR1 del12 carriers.**

| p value | Estimate (SD) | Gene | Full Name |
|---------|---------------|------|-----------|
| 3.71E-54 | 1.34 | TNFSF8 | Tumor necrosis factor ligand superfamily member 8 |
| 1.33E-52 | 1.45 | CD163 | Scavenger receptor cysteine-rich type 1 protein M130 |
| 2.07E-25 | 1.09 | CSF1R | Macrophage colony-stimulating factor 1 receptor |
| 1.44E-24 | 1.16 | LYVE1 | Lymphatic vessel endothelial hyaluronic acid receptor 1 |
| 1.03E-22 | 0.65 | IL6ST | Interleukin-6 receptor subunit beta |
| 4.56E-15 | 0.67 | IL18BP | Interleukin-18-binding protein |
| 1.16E-12 | 0.74 | CD300C | CMRF35-like molecule 6 |
| 2.47E-12 | 0.59 | TYRO3 | Tyrosine-protein kinase receptor TYRO3 |
| 8.85E-12 | 0.80 | LRP8 | Low-density lipoprotein receptor-related protein 8 |
| 1.76E-09 | 0.66 | IL1RL1 | Interleukin-1 receptor-like 1 |

(continued)

| p value | Estimate (SD) | Gene | Full Name |
|---------|---------------|------|-----------|
| 2.62E-09 | 0.61 | ISLR2 | Immunoglobulin superfamily containing leucine-rich repeat protein 2 |
| 4.01E-09 | 0.55 | SIGLEC7 | Sialic acid-binding Ig-like lectin 7 |
| 4.47E-09 | 0.48 | NRXN3 | Neurexin-3-beta |
| 1.03E-07 | 0.58 | PLAU | Urokinase-type plasminogen activator |
| 2.96E-07 | 0.37 | CD55 | Complement decay-accelerating factor |
| 8.27E-07 | 0.53 | CD48 | CD48 antigen |
| 1.22E-06 | 0.31 | TNFRSF21 | Tumor necrosis factor receptor superfamily member 21 |
| 1.62E-06 | 0.36 | MRC2 | C-type mannose receptor 2 |
| 3.82E-06 | 0.57 | KLK13 | Kallikrein-13 |
| 4.95E-06 | 0.33 | IGF1R | Insulin-like growth factor 1 receptor |
| 1.46E-05 | 0.45 | ANGPT2 | Angiopoietin-2 |
| 2.02E-05 | 0.39 | CNTN4 | Contactin-4 |
| 2.57E-05 | 0.47 | FCGR3B | Low affinity immunoglobulin gamma Fc region receptor III-B |
| 2.93E-05 | 0.38 | C1S | Complement C1s subcomponent |
| 3.92E-05 | 0.40 | LY9 | T-lymphocyte surface antigen Ly-9 |
| 4.48E-05 | 0.46 | CD200R1 | Cell surface glycoprotein CD200 receptor 1 |

**Table 18.2. Proteins with significant decrease in serum of ASGR1 del12 carriers.**

| p value | Estimate (SD) | Gene | Target Full Name |
|---------|---------------|------|------------------|
| 1.08E-09 | -0.52 | CD93 | Complement component C1q receptor |
| 6.32E-09 | -0.50 | IDS | Iduronate 2-sulfatase |
| 1.56E-07 | -0.34 | RGMB | RGM domain family member B |
| 2.91E-07 | -0.44 | TGFBI | Transforming growth factor-beta-induced protein ig-h3 |
| 5.56E-07 | -0.48 | LUM | Lumican |
| 6.67E-07 | -0.46 | MMP2 | 72 kDa type IV collagenase |
| 1.36E-06 | -0.38 | FLRT2 | Leucine-rich repeat transmembrane protein FLRT2 |
| 2.18E-06 | -0.48 | AHSG | Alpha-2-HS-glycoprotein |
| 2.44E-06 | -0.37 | CSH1 CSH2 | Chorionic somatomammotropin hormone |
| 3.16E-06 | -0.54 | ESM1 | Endothelial cell-specific molecule 1 |
| 1.36E-05 | -0.52 | AFM | Afamin |
| 1.67E-05 | -0.48 | TNFRSF17 | Tumor necrosis factor receptor superfamily member 17 |
| 2.68E-05 | -0.46 | OMD | Osteomodulin |
| 4.69E-05 | -0.23 | GDI2 | Rab GDP dissociation inhibitor beta |
| 5.09E-05 | -0.45 | SPOCK2 | Testican-2 |

**References**

1 See also, Nioi, P. et al. Variant ASGR1 Associated with a Reduced Risk of Coronary Artery Disease. The New England journal of medicine 374, 2131-2141, doi:10.1056/NEJMoa1508419 (2016).

2 Gold, L. et al. Aptamer-based multiplexed proteomic technology for biomarker discovery. PLoS One 5, e15004, doi:10.1371/joumal.pone.0015004 (2010).

3 Sham, P. C. & Purcell, S. M. Statistical power and significance testing in large-scale genetic studies. Nature reviews. Genetics 15, 335-346, doi:10.1038/nrg3706 (2014).

**Example 19: Proteomic Profiling of Serum Samples from ASGR1 Cyno PK-PD study**

Introduction

As decribed above in Example 1, ASGR1 loss-of-function (LOF) was found to be associated with a beneficial phenotype (protected from coronary artery disease, lower LDL cholesterol and longer life span) in human[1]. Certain ASGR-1 antigen binding proteins disclosed herein were found to mimic the LOF effects, and can be useful in the treatment of coronary artery disease. In brief, cynomolgus monkeys were treated with certain ASGR-1 specific, ligand blocking antibodies in order to study the PK-PD profile of these antibodies. Moreover, a dose-dependent elevation of alkaline phosphatase (ALP) levels was observed in the Ab-treated cynos, which resembles the ALP elevation seen in human ASGR1 LOF carriers. In addition to ALP, proteomic profiling in human serum identified 41 proteins that potentially underlie the beneficial effects caused by ASGR1 LOF as described above in Example 18. To compare effects of anti-ASGR1 antibody treatment with the human ASGR1 LOF and identify comparable signatures in cynomolgus monkey, proteomic measurement of the serum samples from this study was conducted. The list of proteins with altered levels in the antibody-treated animals is compared to the ones identified in human LOF carriers.

**Materials and methods**

Sample selection and Proteomic Profiling

6 animal groups with 3 animals in each group were selected for proteomic profiling. The 6 groups include 5 antibody-treated groups (mAb1/25A4, mAb2/4A2, mAb3/7E11, mAb4/5E5 and mAb8/4H6) and a vehicle control group (mAb6). The animals were dosed once at 100mg/kg. Serum samples from time points 0, 168, 336, 504, 672 and 1176 hours were collected for each animal (Table 19.1 & 19.2). The only exception is group mAb8/4H6, where time point 1008 hour is used instead of 1176 hour. 120ul serum samples were shipped to SomaLogic Inc, where 1310 proteins (see table 18.0) were measured by the SOMAscan Assay 1.3k.

The SOMAscan assay measures serum protein concentration using a Slow Off-rate Modified DNA Aptamer (SOMAmer)-based capture array[2]. Each of the 1310 proteins is bond by its respective fluorescently labeled SOMAmer in the assay and their concentrations are reflected by the respective SOMAmer's relative fluorescence units (RFU).

## Table 19.1. Serum sample selection.

| Animal group | Animal Number | Time points | | | | | |
|---|---|---|---|---|---|---|---|
| | | D0 | D8 | D15 | D22 | D29 | D50 |
| | | 0 hr | 168 hr | 336 hr | 504 hr | 672 hr | 1176 hr |
| 25A4 | 701, 702, 703 | √ | √ | √ | √ | √ | √ |
| 4A2 | 704, 705, 706 | √ | √ | √ | √ | √ | √ |
| 7E11 | 707, 708, 711 | √ | √ | √ | √ | √ | √ |
| 9E5 | 709, 710, 712 | √ | √ | √ | √ | √ | √ |
| SEFL2 control | 716, 717, 718 | √ | √ | √ | √ | √ | √ |
| 4H6 | 204, 205, 206 | √ | √ | √ | √ | √ | √* |

\* 4H6 was collected at D43 (1008 hr).

### Table 19.2. List of all sample groups by treatment and time points.

| Sample group | Treatment (*e.g.*, drug, vehicle, *etc.*) | Time point | # of Samples in Group | Subject ID |
|---|---|---|---|---|
| 25A4_D0 | mAb1 | 0 Hr | 3 | 701, 702, 703 |
| 25A4_D8 | mAb1 | 168 Hr | 3 | 701, 702, 703 |
| 25A4_D15 | mAb1 | 336 Hr | 3 | 701, 702, 703 |
| 25A4_D22 | mAb1 | 504 Hr | 3 | 701, 702, 703 |
| 25A4_D29 | mAb1 | 672 Hr | 3 | 701, 702, 703 |
| 25A4_D50 | mAb1 | 1176 Hr | 3 | 701, 702, 703 |
| 4A2_D0 | mAb2 | 0 Hr | 3 | 704, 705, 706 |
| 4A2_D8 | mAb2 | 168 Hr | 3 | 704, 705, 706 |
| 4A2_D15 | mAb2 | 336 Hr | 3 | 704, 705, 706 |
| 4A2 D22 | mAb2 | 504 Hr | 3 | 704, 705, 706 |
| 4A2_D29 | mAb2 | 672 Hr | 3 | 704, 705, 706 |
| 4A2_D50 | mAb2 | 1176 Hr | 3 | 704, 705, 706 |
| 7E11_D0 | mAb3 | 0 Hr | 3 | 707, 708, 711 |
| 7E11_D8 | mAb3 | 168 Hr | 3 | 707, 708, 711 |
| 7E11_D15 | mAb3 | 336 Hr | 3 | 707, 708, 711 |
| 7E11_D22 | mAb3 | 504 Hr | 3 | 707, 708, 711 |
| 7E11_D29 | mAb3 | 672 Hr | 3 | 707, 708, 711 |
| 7E11_D50 | mAb3 | 1176 Hr | 3 | 707, 708, 711 |

(continued)

| Sample group | Treatment (*e.g.*, drug, vehicle, *etc.*) | Time point | # of Samples in Group | Subject ID |
|---|---|---|---|---|
| 5E5_D0 | mAb4 | 0 Hr | 3 | 709, 710, 712 |
| 5E5_D8 | mAb4 | 168 Hr | 3 | 709, 710, 712 |
| 5E5_D15 | mAb4 | 336 Hr | 3 | 709, 710, 712 |
| 5E5_D22 | mAb4 | 504 Hr | 3 | 709, 710, 712 |
| 5E5_D29 | mAb4 | 672 Hr | 3 | 709, 710, 712 |
| 5E5_D50 | mAb4 | 1176 Hr | 3 | 709, 710, 712 |
| CTL_D0 | mAb6 | 0 Hr | 3 | 716, 717, 718 |
| CTL_D8 | mAb6 | 168 Hr | 3 | 716, 717, 718 |
| CTL_D15 | mAb6 | 336 Hr | 3 | 716, 717, 718 |
| CTL_D22 | mAb6 | 504 Hr | 3 | 716, 717, 718 |
| CTL_D29 | mAb6 | 672 Hr | 3 | 716, 717, 718 |
| CTL_D50 | mAb6 | 1176 Hr | 3 | 716, 717, 718 |
| 4H6_D0 | mAb8 | 0 Hr | 3 | 204, 205, 206 |
| 4H6_D8 | mAb8 | 168 Hr | 3 | 204, 205, 206 |
| 4H6_D15 | mAb8 | 336 Hr | 3 | 204, 205, 206 |
| 4H6_D22 | mAb8 | 504 Hr | 3 | 204, 205, 206 |
| 4H6_D29 | mAb8 | 672 Hr | 3 | 204, 205, 206 |
| 4H6_D43 | mAb8 | 1008 Hr | 3 | 204, 205, 206 |

Data analysis

As the SOMAscan assay was developed for humans, some proteins in cynomolgus monkey may not be recognized by the SOMAmer reagents. As a result, SOMAscan measurements of these proteins would have low credibility and may not reflect the true protein levels. A simple criterion was defined to determine the credibility of the measurements, assuming the serum levels of a given protein are in relatively close range in human and cynomolgus monkey. The mean and range of each protein level in human are calculated based on the 217 human control samples from the human proteomic study described in Example 18. The mean and range of each protein level in cynomolgus monkey are calculated based on a total of 48 samples including measurements of all time points for the SEFL-2 control group and the pre-treatment (D0) and washout period (D50) measurements of all the other groups. A protein measurement would be assigned low credibility if (1) its range in cynomolgus monkey is not overlapping with human; and (2) there is a 5 fold difference between the mean level of this protein in human and cynomolgus monkey. A total of 162 proteins were determined as low-credibility by these criteria and were excluded (Figure 58, which depicts a summary of the credibility of protein measurements in cynomolgus monkey). In FIG. 58, log10 RFU of mean protein levels in the two species are plotted and the ones with low credibility (light shading) and high credibility (black) are marked.

One sample in the 4H6 group was removed due to low volume that did not meet the requirements for the SOMAscan assay. No outliers were found in the principle components analysis. A linear mixed model adjusting for potential confounding factors was used to test whether the ASGR1 antibody treatment changes each protein level differently from the control group over time points,

$$Y_{ti} = \beta_0 + \beta_1 \text{TREATGROUP}_i + \beta_2 \text{TIME}_{ti} + \beta_3 (\text{TREATGROUP}_i)(\text{TIME}_{ti}) + \beta_4 COV_{ti} + \cdots + \beta_{m+4} COV_{ti} + b_{0i} + \varepsilon_{ti}$$

which is determined by the p-value for $\beta_3$ (i.e., treatment by time interaction; mean difference in slopes between treatment conditions). The random effect $b_{0i}$ captures individual animal heterogeneity. The TREATGROUP is coded as (25A4=4A2=7E11=5E5=4H6=1; SELF-2=0) and TIME is coded as (D8=D15=D22=D29=1; D0=D50=0) to test for the ASGR1 antibodies effect after treatment comparing to pre-treatment and washout period. Since multiple tests were performed for the proteins on SOMAscan platform, a Bonferroni corrected significant threshold ($5\times10^{-5}$) was used.

**Results and discussion**

33 proteins were identified to have significant serum level changes after ASGR1 antibody treatment (Table 19.3; $P<5\times10^{-5}$). Interestingly, all the 33 proteins show increased levels (1.36~10.18 fold) after ASGR1 antibody treatment.

**Table 19.3. Proteins with significant changes after ASGR1 antibody treatment in Cynomolgus monkey.**

| P-value | Estimated Fold Change | Gene | Full Name |
|---|---|---|---|
| 1.87E-13 | 10.18 | TNFSF8 | Tumor necrosis factor ligand superfamily member 8 |
| 1.01E-06 | 8.56 | ASGR1 | Asialoglycoprotein receptor 1 |
| 1.35E-10 | 3.93 | ADGRE2 | Adhesion G protein-coupled receptor E2 |
| 2.74E-11 | 2.86 | CD86 | T-lymphocyte activation antigen CD86 |
| 1.46E-11 | 2.81 | TNFRSF21 | Tumor necrosis factor receptor superfamily member 21 |
| 7.48E-10 | 2.57 | L1CAM | Neural cell adhesion molecule L1 |
| 6.09E-12 | 2.42 | PLXNC1 | Plexin-C1 |
| 1.22E-07 | 2.11 | MRC2 | C-type mannose receptor 2 |
| 1.18E-06 | 2.10 | AMIGO2 | Amphoterin-induced protein 2 |
| 2.28E-11 | 2.02 | ANGPT2 | Angiopoietin-2 |
| 6.68E-09 | 1.99 | INSR | Insulin receptor |
| 1.02E-10 | 1.93 | IL17RA | Interleukin-17 receptor A |
| 7.12E-12 | 1.90 | NRXN3 | Neurexin-3-beta |
| 5.95E-06 | 1.85 | GPNMB | Transmembrane glycoprotein NMB |
| 2.03E-06 | 1.74 | IGF1R | Insulin-like growth factor 1 receptor |
| 3.91E-09 | 1.73 | PLAUR | Urokinase plasminogen activator surface receptor |
| 3.58E-09 | 1.69 | FGFR1 | Fibroblast growth factor receptor 1 |
| 1.26E-06 | 1.60 | LRP8 | Low-density lipoprotein receptor-related protein 8 |
| 3.87E-09 | 1.55 | LYPD3 | Ly6/PLAUR domain-containing protein 3 |
| 3.17E-06 | 1.55 | GRN | Granulins |
| 4.27E-05 | 1.54 | CNTN4 | Contactin-4 |
| 4.59E-07 | 1.54 | KDR | Vascular endothelial growth factor receptor 2 |
| 4.99E-06 | 1.53 | IL12RB2 | Interleukin-12 receptor subunit beta-2 |
| 5.85E-06 | 1.52 | ROBO3 | Roundabout homolog 3 |
| 1.44E-06 | 1.50 | ALCAM | CD166 antigen |
| 3.83E-05 | 1.46 | TYRO3 | Tyrosine-protein kinase receptor TYRO3 |
| 3.09E-05 | 1.45 | CADM1 | Cell adhesion molecule 1 |
| 1.53E-08 | 1.44 | JAG1 | Protein jagged-1 |
| 2.58E-09 | 1.43 | ISLR2 | Immunoglobulin superfamily containing leucine-rich repeat protein 2 |
| 3.11E-05 | 1.39 | SET | Protein SET |

(continued)

| P-value | Estimated Fold Change | Gene | Full Name |
|---|---|---|---|
| 4.64E-05 | 1.38 | IL20RA | Interleukin-20 receptor subunit alpha |
| 2.15E-06 | 1.36 | KLRK1 | NKG2-D type II integral membrane protein |
| 2.39E-05 | 1.36 | GFRA2 | GDNF family receptor alpha-2 |

To compare results from this study with the human proteomic study, a list of proteins made by the 33 proteins in Table 19.3 and the top 41 proteins identified in human was compiled. This results in a list of 64 proteins total. The estimates of protein level change and p-value of the changes in the studies were compared (Table 19.4). Based on concordance of change in the cyno (in response to ASGR1 antibody treatment) and human (in response to ASGR1 LOF) studies, the proteins are classified into 5 tiers. Tier 1 includes 10 proteins that pass the stringent Bonferroni corrected significance level ($p<5\times10^{-5}$) in both studies with the same direction of changes. The number of proteins supported by strong evidence in both studies are much higher than the number one would expect by chance ($p=1.58\times10^{-8}$; Fisher's exact test). It indicates that ASGR1 Ab treatment can induce a serum protein levels change in cyno that is similar to the effect of del12 LOF variant in Human. Therefore, these proteins are the core biomarkers. For example, the strongest biomarker TNFSF8 had more than 10 fold increase after ASGR1 Antibody treatment (Figures 59A-59D, which depict the results of serum protein levels of TNFSF8 in cyno and human studies).

Tier 2 contains 12 proteins with strong evidence ($p<5\times10^{-5}$) in the cyno study and suggestive evidence ($p<0.05$) in human with the same direction of changes. Both Tier 1 and 2 proteins have increased levels in both studies. Tier 3 includes 11 proteins that are found significant only in the cyno study but not human. These proteins are likely to be biomarkers specific for the drug modality or for cynomolgus monkeys. For example, the soluble secreted form of ASGR1 increased more than 10 fold after antibody treatment but no significant difference was observed in human between the ASGR1 del12 carriers and non-carriers. Tier 4 contains 17 proteins with significant evidence ($p<5\times10^{-5}$) in the human study but not supported by the cyno study. Majority of the proteins in Tier 4 has decrease levels in human del12 carriers. This observation may indicate a difference between antibody treatment and constitutive gene LOF. It could also possibly be due to species difference or simply caused by lower statistical power in the cyno study.

Lastly, there are 14 proteins with significant changes in human classified as Tier 5 because they were excluded in the cyno study due to the low credibility of their SOMAmer reagents.

In summary, the two studies show high degree of concordance between the antibody treatment in cynomolgus monkey and ASGR1 LOF in humans, with 10 proteins (Tier 1) showing very significant changes in the same direction in both studies. The ASGR-1 antibody treatment is working well as a way of mimicking the effects of ASGR1 LOF in humans and can be useful in the treatment of coronary artery disease.

**Table 19.4. Five tiers of protein biomarkers and comparison of the estimates of protein level change and p-value between the two studies.**

| Target Full Name | Gene | human | | Cyno | | Tier |
|---|---|---|---|---|---|---|
| | | Estimate (SD) | P-value | Estimate log2FC | P-value | |
| Tumor necrosis factor ligand superfamily member 8 | TNFSF8 | 1.34 | 3.7E-54 | 3.35 | 1.87E-13 | 1 |
| Tumor necrosis factor receptor superfamily member 21 | TNFRSF21 | 0.31 | 1.2E-06 | 1.49 | 1.46E-11 | 1 |
| C-type mannose receptor 2 | MRC2 | 0.36 | 1.6E-06 | 1.08 | 1.22E-07 | 1 |
| Angiopoietin-2 | ANGPT2 | 0.45 | 1.5E-05 | 1.01 | 2.28E-11 | 1 |
| Neurexin-3-beta | NRXN3 | 0.48 | 4.5E-09 | 0.93 | 7.12E-12 | 1 |
| Insulin-like growth factor 1 receptor | IGF1R | 0.33 | 5.0E-06 | 0.80 | 2.03E-06 | 1 |
| Low-density lipoprotein receptor-related protein 8 | LRP8 | 0.80 | 8.9E-12 | 0.68 | 1.26E-06 | 1 |

(continued)

| Target Full Name | Gene | human | | Cyno | | Tier |
|---|---|---|---|---|---|---|
| | | Estimate (SD) | P-value | Estimate log2FC | P-value | |
| Contactin-4 | CNTN4 | 0.39 | 2.0E-05 | 0.63 | 4.27E-05 | 1 |
| Tyrosine-protein kinase receptor TYRO3 | TYRO3 | 0.59 | 2.5E-12 | 0.55 | 3.83E-05 | 1 |
| Immunoglobulin superfamily containing leucine-rich repeat protein 2 | ISLR2 | 0.61 | 2.6E-09 | 0.52 | 2.58E-09 | 1 |
| T-lymphocyte activation antigen CD86 | CD86 | 0.39 | 2.1E-03 | 1.52 | 2.74E-11 | 2 |
| Neural cell adhesion molecule L1 | L1CAM | 0.30 | 5.5E-03 | 1.36 | 7.48E-10 | 2 |
| Plexin-CI | PLXNC1 | 0.40 | 1.0E-04 | 1.28 | 6.09E-12 | 2 |
| Amphoterin-induced protein 2 | AMIGO2 | 0.44 | 1.9E-04 | 1.07 | 1.18E-06 | 2 |
| Interleukin-17 receptor A | IL17RA | 0.29 | 0.03 | 0.95 | 1.02E-10 | 2 |
| Urokinase plasminogen activator surface receptor | PLAUR | 0.35 | 3.3E-04 | 0.79 | 3.91E-09 | 2 |
| Fibroblast growth factor receptor 1 | FGFR1 | 0.30 | 2.3E-03 | 0.75 | 3.58E-09 | 2 |
| Granulins | GRN | 0.27 | 5.7E-03 | 0.63 | 3.17E-06 | 2 |
| CD166 antigen | ALCAM | 0.20 | 9.1E-03 | 0.58 | 1.44E-06 | 2 |
| Protein jagged-1 | JAG1 | 0.17 | 0.01 | 0.53 | 1.53E-08 | 2 |
| Protein SET | SET | 0.28 | 2.1E-03 | 0.47 | 3.11E-05 | 2 |
| GDNF family receptor alpha-2 | GFRA2 | 0.39 | 9.2E-05 | 0.44 | 2.39E-05 | 2 |
| Asialoglycoprotein receptor 1 | ASGR1 | 0.00 | 0.99 | 3.10 | 1.01E-06 | 3 |
| Adhesion G protein-coupled receptor E2 | ADGRE2 | 0.04 | 0.70 | 1.97 | 1.35E-10 | 3 |
| Insulin receptor | INSR | 0.20 | 0.06 | 1.00 | 6.68E-09 | 3 |
| Transmembrane glycoprotein NMB | GPNMB | -0.22 | 0.01 | 0.89 | 5.95E-06 | 3 |
| Ly6/PLAUR domain-containing protein 3 | LYPD3 | -0.06 | 0.26 | 0.63 | 3.87E-09 | 3 |
| Vascular endothelial growth factor receptor 2 | KDR | 0.19 | 0.09 | 0.63 | 4.59E-07 | 3 |
| Interleukin-12 receptor subunit beta-2 | IL12RB2 | 0.11 | 0.38 | 0.61 | 4.99E-06 | 3 |
| Roundabout homolog 3 | ROBO3 | 0.08 | 0.55 | 0.61 | 5.85E-06 | 3 |
| Cell adhesion molecule 1 | CADM1 | -0.17 | 0.02 | 0.53 | 3.09E-05 | 3 |

(continued)

| Target Full Name | Gene | human | | Cyno | | Tier |
|---|---|---|---|---|---|---|
| | | Estimate (SD) | P-value | Estimate log2FC | P-value | |
| Interleukin-20 receptor subunit alpha | IL20RA | 0.05 | 0.69 | 0.47 | 4.64E-05 | 3 |
| NKG2-D type II integral membrane protein | KLRK1 | -0.14 | 0.25 | 0.44 | 2.15E-06 | 3 |
| Lymphatic vessel endothelial hyaluronic acid receptor 1 | LYVE1 | 1.16 | 1.44E-24 | 0.00 | 0.96 | 4 |
| CMRF35-like molecule 6 | CD300C | 0.74 | 1.16E-12 | 0.03 | 0.39 | 4 |
| Interleukin-1 receptor-like 1 | IL1RL1 | 0.66 | 1.76E-09 | 0.75 | 0.10 | 4 |
| Kallikrein-13 | KLK13 | 0.57 | 3.82E-06 | 0.08 | 0.42 | 4 |
| CD48 antigen | CD48 | 0.53 | 8.27E-07 | -0.06 | 0.81 | 4 |
| Rab GDP dissociation inhibitor beta | GDI2 | -0.23 | 4.69E-05 | -0.27 | 0.09 | 4 |
| Chorionic somatomammotropin hormone | CSH1 CSH2 | -0.37 | 2.44E-06 | 0.19 | 9.4E-03 | 4 |
| Leucine-rich repeat transmembrane protein FLRT2 | FLRT2 | -0.38 | 1.36E-06 | 0.30 | 0.03 | 4 |
| Transforming growth factor-beta-induced protein ig-h3 | TGFBI | -0.44 | 2.91E-07 | 0.13 | 0.46 | 4 |
| Testican-2 | SPOCK2 | -0.45 | 5.09E-05 | 0.10 | 0.96 | 4 |
| 72 kDa type IV collagenase | MMP2 | -0.46 | 6.67E-07 | 0.13 | 0.48 | 4 |
| Osteomodulin | OMD | -0.46 | 2.68E-05 | 0.12 | 0.78 | 4 |
| Alpha-2-HS-glycoprotein | AHSG | -0.48 | 2.18E-06 | 0.00 | 0.97 | 4 |
| Iduronate 2-sulfatase | IDS | -0.50 | 6.32E-09 | 0.05 | 0.33 | 4 |
| Complement component Clq receptor | CD93 | -0.52 | 1.08E-09 | 0.19 | 0.15 | 4 |
| Afamin | AFM | -0.52 | 1.36E-05 | 0.02 | 0.94 | 4 |
| Endothelial cell-specific molecule 1 | ESM1 | -0.54 | 3.16E-06 | 0.09 | 0.56 | 4 |
| Scavenger receptor cysteine-rich type 1 protein M130 | CD163 | 1.45 | 1.33E-52 | NA | NA | 5 |
| Macrophage colony-stimulating factor 1 receptor | CSF1R | 1.09 | 2.07E-25 | NA | NA | 5 |
| Interleukin-18-binding protein | IL18BP | 0.67 | 4.56E-15 | NA | NA | 5 |
| Interleukin-6 receptor subunit beta | IL6ST | 0.65 | 1.03E-22 | NA | NA | 5 |
| Urokinase-type plasminogen activator | PLAU | 0.58 | 1.03E-07 | NA | NA | 5 |

(continued)

| Target Full Name | Gene | human | | Cyno | | Tier |
|---|---|---|---|---|---|---|
| | | Estimate (SD) | P-value | Estimate log2FC | P-value | |
| Sialic acid-binding Ig-like lectin 7 | SIGLEC7 | 0.55 | 4.01E-09 | NA | NA | 5 |
| Low affinity immunoglobulin gamma Fc region receptor III-B | FCGR3B | 0.47 | 2.57E-05 | NA | NA | 5 |
| Cell surface glycoprotein CD200 receptor 1 | CD200R1 | 0.46 | 4.48E-05 | NA | NA | 5 |
| T-lymphocyte surface antigen Ly-9 | LY9 | 0.40 | 3.92E-05 | NA | NA | 5 |
| Complement Cl s subcomponent | C1S | 0.38 | 2.93E-05 | NA | NA | 5 |
| Complement decay-accelerating factor | CD55 | 0.37 | 2.96E-07 | NA | NA | 5 |
| RGM domain family member B | RGMB | -0.34 | 1.56E-07 | NA | NA | 5 |
| Lumican | LUM | -0.48 | 5.56E-07 | NA | NA | 5 |
| Tumor necrosis factor receptor superfamily member 17 | TNFRSF17 | -0.48 | 1.67E-05 | NA | NA | 5 |

**References**

1 See also, Nioi, P. et al. Variant ASGR1 Associated with a Reduced Risk of Coronary Artery Disease. The New England journal of medicine 374, 2131-2141, doi:10.1056/NEJMoa1508419 (2016).
2 Gold, L. et al. Aptamer-based multiplexed proteomic technology for biomarker discovery. PLoS One 5, e15004, doi:10.1371/joumal.pone.0015004 (2010).

**Example 20: Method of reducing a risk of cardiovascular disease**

A subject at risk of cardiovascular disease is identified. One or more antibodies as provided herein (see Example 7, as well as Tables A, B and C) and/or RNAi constructs that reduce expression of ASGR-1 and/or ASGR-2 (as outlined in Example 3), are administered to a subject at risk of cardiovascular disease. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., Tier 1) is monitored to make certain that an adequate amount of the antibody and/or RNAi construct is administered and is functioning as desired. The risk that the subject will experience cardio vascular disease is decreased.

Additionally, as a further option, physiologic effects of the antibody and/or RNAi can be evaluated in relevant animal models of cardiovascular disease using readouts including blood pressure, primary and secondary hemostasis, heart function and morphology, endothelial function, LDL cholesterol levels, non-HDL cholesterol levels, inflammation, and/or atherosclerosis.

**Example 21: Method of reducing a risk of myocardial infarction or coronary artery disease**

A subject at risk of a myocardial infarction or coronary artery disease is identified. One or more antibodies as provided herein (see Example 7, as well as Tables A, B and C) and/or RNAi constructs that reduce expression of ASGR-1 and/or ASGR-2 (as outlined in Example 3), are administered to a subject at risk of a myocardial infarction or coronary artery disease. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., Tier 1) is monitored to make certain that an adequate amount of the antibody and/or RNAi construct is administered and is functioning as desired. The risk that the subject will experience a myocardial infarction or coronary artery disease is decreased.

Additionally, as a further option, physiologic effects of the antibody and/or RNAi can be evaluated in relevant animal models of myocardial infarction or coronary artery disease using readouts including blood pressure, primary and secondary hemostasis, heart function and morphology, endothelial function, LDL cholesterol levels, non-HDL cholesterol levels, inflammation, and/or atherosclerosis.

## Example 22: Method of reducing LDL cholesterol

A subject having a LDL cholesterol level to be lowered is identified. One or more antibodies as provided herein (see Example 7, as well as Tables A, B and C) and/or RNAi constructs that reduce expression of ASGR-1 and/or ASGR-2 (as outlined in Example 3), are administered to the subject. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., tier 1) is monitored to make certain that an adequate amount of the antibody and/or RNAi construct is administered and is functioning as desired. The level of LDL cholesterol in the subject is thereby reduced.

## Example 23: Method of reducing non-HDL cholesterol

A subject having a non-HDL cholesterol level to be lowered is identified. One or more antibodies as provided herein (see Example 7, as well as Tables A, B and C) and/or RNAi constructs that reduce expression of ASGR-1 and/or ASGR-2 (as outlined in Example 3), are administered to the subject. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., Tier 1) is monitored to make certain that an adequate amount of the antibody and/or RNAi construct is administered and is functioning as desired. The level of non-HDL cholesterol in the subject is thereby reduced.

## Example 24: Method of increasing ALP levels

One or more antibodies as provided herein (see Example 7, as well as Tables A, B, and C) and/or RNAi constructs that reduce expression of ASGR-1 and/or ASGR-2 (as outlined in Example 3), are administered to the subject. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., Tier 1) is monitored to make certain that an adequate amount of the antibody and/or RNAi construct is administered and is functioning as desired. The level of ALP in the subject is thereby increased.

## Example 25: Method of Monitoring the Effectiveness of an ASGR-1 Therapy

One or more antibodies as provided herein (see Example 7, as well as Tables A, B and C) and/or RNAi constructs that reduce expression of ASGR-1 and/or ASGR-2 (as outlined in Example 3), are administered to the subject. One or more of the markers in Example 19 is monitored to make certain that an adequate amount of the antibody and/or RNAi construct is administered and is functioning as desired. When the marker level changes in a similar manner to those changes noted in Example 19 (e.g., Tier 1), it is evidence that the amount of the one or more antibody and/or RNAi is effective. Additionally, as a further option, the effectiveness of this biochemical change can be observed by its physiologic effects from the antibody and/or RNAi, which can be evaluated using readouts including blood pressure, primary and secondary hemostasis, heart function and morphology, endothelial function, LDL cholesterol levels, non-HDL cholesterol levels, inflammation, and/or atherosclerosis.

## Table 10.1

| | | | | | | | | | |
|---|---|---|---|---|---|---|---|---|---|
| ATOM | 1 | O | THR | A | 151 | -35.000 | -25.802 | 13.973 | 1.00 41.82 | O |
| ATOM | 2 | N | THR | A | 151 | -34.909 | -27.403 | 11.804 | 1.00 40.99 | N |
| ATOM | 3 | CA | THR | A | 151 | -34.274 | -27.888 | 13.020 | 1.00 40.74 | C |
| ATOM | 4 | C | THR | A | 151 | -34.232 | -26.755 | 14.051 | 1.00 40.85 | C |
| ATOM | 5 | CB | THR | A | 151 | -32.835 | -28.381 | 12.758 | 1.00 37.81 | C |
| ATOM | 6 | OG1 | THR | A | 151 | -32.738 | -28.919 | 11.438 | 1.00 46.22 | O |
| ATOM | 7 | CG2 | THR | A | 151 | -32.470 | -29.491 | 13.725 | 1.00 44.52 | C |
| ATOM | 8 | O | CYS | A | 152 | -30.928 | -26.774 | 16.222 | 1.00 27.69 | O |
| ATOM | 9 | N | CYS | A | 152 | -33.315 | -26.861 | 15.005 | 1.00 43.00 | N |
| ATOM | 10 | CA | CYS | A | 152 | -33.119 | -25.852 | 16.027 | 1.00 32.33 | C |
| ATOM | 11 | C | CYS | A | 152 | -31.621 | -25.772 | 16.312 | 1.00 30.92 | C |
| ATOM | 12 | CB | CYS | A | 152 | -33.900 | -26.213 | 17.289 | 1.00 36.97 | C |
| ATOM | 13 | SG | CYS | A | 152 | -34.435 | -24.804 | 18.287 | 1.00 47.04 | S |
| ATOM | 14 | N | CYS | A | 153 | -31.104 | -24.590 | 16.620 | 1.00 26.78 | N |
| ATOM | 15 | CA | CYS | A | 153 | -29.716 | -24.493 | 17.055 | 1.00 24.74 | C |
| ATOM | 16 | C | CYS | A | 153 | -29.577 | -25.058 | 18.464 | 1.00 26.56 | C |
| ATOM | 17 | O | CYS | A | 153 | -30.538 | -25.026 | 19.235 | 1.00 25.88 | O |
| ATOM | 18 | CB | CYS | A | 153 | -29.243 | -23.040 | 17.017 | 1.00 21.46 | C |
| ATOM | 19 | SG | CYS | A | 153 | -29.368 | -22.304 | 15.376 | 1.00 33.20 | S |
| ATOM | 20 | N | PRO | A | 154 | -28.379 | -25.571 | 18.813 | 1.00 26.96 | N |
| ATOM | 21 | CA | PRO | A | 154 | -28.146 | -26.019 | 20.190 | 1.00 20.79 | C |
| ATOM | 22 | C | PRO | A | 154 | -28.236 | -24.848 | 21.163 | 1.00 22.57 | C |
| ATOM | 23 | O | PRO | A | 154 | -28.081 | -23.710 | 20.737 | 1.00 22.50 | O |
| ATOM | 24 | CB | PRO | A | 154 | -26.715 | -26.585 | 20.147 | 1.00 20.99 | C |
| ATOM | 25 | CG | PRO | A | 154 | -26.432 | -26.822 | 18.709 | 1.00 21.42 | C |
| ATOM | 26 | CD | PRO | A | 154 | -27.183 | -25.760 | 17.974 | 1.00 19.94 | C |
| ATOM | 27 | N | VAL | A | 155 | -28.499 | -25.124 | 22.438 | 1.00 21.58 | N |
| ATOM | 28 | CA | VAL | A | 155 | -28.490 | -24.090 | 23.462 | 1.00 20.72 | C |
| ATOM | 29 | C | VAL | A | 155 | -27.187 | -23.287 | 23.412 | 1.00 25.13 | C |
| ATOM | 30 | O | VAL | A | 155 | -26.109 | -23.865 | 23.218 | 1.00 27.20 | O |
| ATOM | 31 | CB | VAL | A | 155 | -28.664 | -24.704 | 24.884 | 1.00 23.97 | C |
| ATOM | 32 | CG1 | VAL | A | 155 | -28.384 | -23.669 | 25.957 | 1.00 27.90 | C |
| ATOM | 33 | CG2 | VAL | A | 155 | -30.062 | -25.267 | 25.061 | 1.00 25.53 | C |
| ATOM | 34 | N | ASN | A | 156 | -27.299 | -21.968 | 23.586 | 1.00 20.81 | N |
| ATOM | 35 | CA | ASN | A | 156 | -26.158 | -21.050 | 23.634 | 1.00 24.68 | C |
| ATOM | 36 | C | ASN | A | 156 | -25.568 | -20.824 | 22.253 | 1.00 25.74 | C |
| ATOM | 37 | O | ASN | A | 156 | -24.518 | -20.206 | 22.106 | 1.00 25.52 | O |
| ATOM | 38 | CB | ASN | A | 156 | -25.070 | -21.552 | 24.603 | 1.00 30.21 | C |
| ATOM | 39 | CG | ASN | A | 156 | -25.565 | -21.628 | 26.041 | 1.00 38.75 | C |
| ATOM | 40 | OD1 | ASN | A | 156 | -26.494 | -20.908 | 26.430 | 1.00 35.88 | O |
| ATOM | 41 | ND2 | ASN | A | 156 | -24.953 | -22.502 | 26.835 | 1.00 33.84 | N |
| ATOM | 42 | N | TRP | A | 157 | -26.247 | -21.340 | 21.237 | 1.00 24.43 | N |
| ATOM | 43 | CA | TRP | A | 157 | -25.928 | -20.981 | 19.866 | 1.00 23.93 | C |
| ATOM | 44 | C | TRP | A | 157 | -26.920 | -19.930 | 19.389 | 1.00 26.16 | C |
| ATOM | 45 | O | TRP | A | 157 | -28.032 | -19.841 | 19.904 | 1.00 28.51 | O |
| ATOM | 46 | CB | TRP | A | 157 | -25.965 | -22.202 | 18.965 | 1.00 22.94 | C |
| ATOM | 47 | CG | TRP | A | 157 | -24.818 | -23.129 | 19.174 | 1.00 24.76 | C |
| ATOM | 48 | CD1 | TRP | A | 157 | -24.459 | -23.752 | 20.336 | 1.00 20.94 | C |
| ATOM | 49 | CD2 | TRP | A | 157 | -23.887 | -23.562 | 18.180 | 1.00 19.03 | C |
| ATOM | 50 | NE1 | TRP | A | 157 | -23.355 | -24.540 | 20.126 | 1.00 23.79 | N |
| ATOM | 51 | CE2 | TRP | A | 157 | -22.983 | -24.440 | 18.809 | 1.00 21.53 | C |
| ATOM | 52 | CE3 | TRP | A | 157 | -23.717 | -23.273 | 16.828 | 1.00 15.83 | C |
| ATOM | 53 | CZ2 | TRP | A | 157 | -21.927 | -25.043 | 18.124 | 1.00 16.56 | C |
| ATOM | 54 | CZ3 | TRP | A | 157 | -22.667 | -23.866 | 16.152 | 1.00 18.56 | C |
| ATOM | 55 | CH2 | TRP | A | 157 | -21.790 | -24.746 | 16.798 | 1.00 13.77 | C |
| ATOM | 56 | N | VAL | A | 158 | -26.520 | -19.120 | 18.420 | 1.00 25.61 | N |
| ATOM | 57 | CA | VAL | A | 158 | -27.394 | -18.058 | 17.951 | 1.00 23.85 | C |
| ATOM | 58 | C | VAL | A | 158 | -27.857 | -18.367 | 16.536 | 1.00 26.75 | C |
| ATOM | 59 | O | VAL | A | 158 | -27.048 | -18.589 | 15.642 | 1.00 24.47 | O |
| ATOM | 60 | CB | VAL | A | 158 | -26.698 | -16.690 | 17.998 | 1.00 26.10 | C |
| ATOM | 61 | CG1 | VAL | A | 158 | -27.691 | -15.587 | 17.690 | 1.00 21.15 | C |
| ATOM | 62 | CG2 | VAL | A | 158 | -26.076 | -16.469 | 19.368 | 1.00 27.52 | C |
| ATOM | 63 | N | GLU | A | 159 | -29.170 | -18.411 | 16.351 | 1.00 30.02 | N |
| ATOM | 64 | CA | GLU | A | 159 | -29.751 | -18.659 | 15.043 | 1.00 28.56 | C |
| ATOM | 65 | C | GLU | A | 159 | -29.824 | -17.359 | 14.272 | 1.00 25.78 | C |
| ATOM | 66 | O | GLU | A | 159 | -30.140 | -16.313 | 14.840 | 1.00 24.18 | O |
| ATOM | 67 | CB | GLU | A | 159 | -31.145 | -19.279 | 15.182 | 1.00 24.88 | C |
| ATOM | 68 | CG | GLU | A | 159 | -31.764 | -19.742 | 13.875 | 1.00 35.35 | C |
| ATOM | 69 | CD | GLU | A | 159 | -33.213 | -20.190 | 14.052 | 1.00 41.67 | C |

| | | | | | | | | | | | |
|---|---|---|---|---|---|---|---|---|---|---|---|
| ATOM | 70 | OE1 | GLU | A | 159 | -34.123 | -19.392 | 13.733 | 1.00 | 44.61 | O |
| ATOM | 71 | OE2 | GLU | A | 159 | -33.441 | -21.333 | 14.516 | 1.00 | 36.73 | O |
| ATOM | 72 | N | HIS | A | 160 | -29.542 | -17.432 | 12.977 | 1.00 | 25.27 | N |
| ATOM | 73 | CA | HIS | A | 160 | -29.577 | -16.257 | 12.118 | 1.00 | 31.03 | C |
| ATOM | 74 | C | HIS | A | 160 | -29.525 | -16.672 | 10.656 | 1.00 | 30.42 | C |
| ATOM | 75 | O | HIS | A | 160 | -28.530 | -17.261 | 10.205 | 1.00 | 26.33 | O |
| ATOM | 76 | CB | HIS | A | 160 | -28.411 | -15.317 | 12.426 | 1.00 | 29.99 | C |
| ATOM | 77 | CG | HIS | A | 160 | -28.320 | -14.150 | 11.493 | 1.00 | 28.06 | C |
| ATOM | 78 | ND1 | HIS | A | 160 | -29.043 | -12.991 | 11.679 | 1.00 | 33.48 | N |
| ATOM | 79 | CD2 | HIS | A | 160 | -27.605 | -13.970 | 10.359 | 1.00 | 29.69 | C |
| ATOM | 80 | CE1 | HIS | A | 160 | -28.770 | -12.143 | 10.703 | 1.00 | 30.20 | C |
| ATOM | 81 | NE2 | HIS | A | 160 | -27.903 | -12.713 | 9.887 | 1.00 | 29.37 | N |
| ATOM | 82 | N | GLU | A | 161 | -30.593 | -16.361 | 9.925 | 1.00 | 29.26 | N |
| ATOM | 83 | CA | GLU | A | 161 | -30.696 | -16.730 | 8.520 | 1.00 | 30.40 | C |
| ATOM | 84 | C | GLU | A | 161 | -30.349 | -18.188 | 8.278 | 1.00 | 30.86 | C |
| ATOM | 85 | O | GLU | A | 161 | -29.548 | -18.493 | 7.393 | 1.00 | 34.60 | O |
| ATOM | 86 | CB | GLU | A | 161 | -29.788 | -15.852 | 7.659 | 1.00 | 35.54 | C |
| ATOM | 87 | CG | GLU | A | 161 | -30.197 | -14.386 | 7.604 | 1.00 | 41.66 | C |
| ATOM | 88 | CD | GLU | A | 161 | -31.526 | -14.165 | 6.901 | 1.00 | 42.46 | C |
| ATOM | 89 | OE1 | GLU | A | 161 | -32.027 | -15.108 | 6.252 | 1.00 | 47.99 | O |
| ATOM | 90 | OE2 | GLU | A | 161 | -32.070 | -13.043 | 7.001 | 1.00 | 40.82 | O |
| ATOM | 91 | N | ARG | A | 162 | -30.928 | -19.068 | 9.092 | 1.00 | 24.36 | N |
| ATOM | 92 | CA | ARG | A | 162 | -30.792 | -20.516 | 8.931 | 1.00 | 31.86 | C |
| ATOM | 93 | C | ARG | A | 162 | -29.374 | -21.045 | 9.202 | 1.00 | 30.42 | C |
| ATOM | 94 | O | ARG | A | 162 | -29.030 | -22.166 | 8.814 | 1.00 | 31.05 | O |
| ATOM | 95 | CB | ARG | A | 162 | -31.250 | -20.930 | 7.528 | 1.00 | 38.14 | C |
| ATOM | 96 | CG | ARG | A | 162 | -32.267 | -22.051 | 7.540 | 1.00 | 48.13 | C |
| ATOM | 97 | CD | ARG | A | 162 | -33.076 | -22.071 | 6.261 | 1.00 | 59.39 | C |
| ATOM | 98 | NE | ARG | A | 162 | -33.517 | -23.423 | 5.921 | 1.00 | 73.66 | N |
| ATOM | 99 | CZ | ARG | A | 162 | -34.365 | -23.702 | 4.937 | 1.00 | 64.55 | C |
| ATOM | 100 | NH1 | ARG | A | 162 | -34.866 | -22.720 | 4.200 | 1.00 | 53.99 | N |
| ATOM | 101 | NH2 | ARG | A | 162 | -34.711 | -24.960 | 4.694 | 1.00 | 63.33 | N |
| ATOM | 102 | N | SER | A | 163 | -28.556 | -20.240 | 9.869 | 1.00 | 24.82 | N |
| ATOM | 103 | CA | SER | A | 163 | -27.287 | -20.728 | 10.379 | 1.00 | 27.06 | C |
| ATOM | 104 | C | SER | A | 163 | -27.270 | -20.611 | 11.897 | 1.00 | 28.98 | C |
| ATOM | 105 | O | SER | A | 163 | -27.914 | -19.730 | 12.474 | 1.00 | 28.00 | O |
| ATOM | 106 | CB | SER | A | 163 | -26.110 | -19.963 | 9.768 | 1.00 | 26.42 | C |
| ATOM | 107 | OG | SER | A | 163 | -25.629 | -20.605 | 8.596 | 1.00 | 27.29 | O |
| ATOM | 108 | N | CYS | A | 164 | -26.548 | -21.522 | 12.537 | 1.00 | 26.62 | N |
| ATOM | 109 | CA | CYS | A | 164 | -26.342 | -21.469 | 13.975 | 1.00 | 23.71 | C |
| ATOM | 110 | C | CYS | A | 164 | -24.920 | -20.996 | 14.249 | 1.00 | 23.70 | C |
| ATOM | 111 | O | CYS | A | 164 | -23.980 | -21.412 | 13.579 | 1.00 | 20.95 | O |
| ATOM | 112 | CB | CYS | A | 164 | -26.591 | -22.838 | 14.601 | 1.00 | 25.60 | C |
| ATOM | 113 | SG | CYS | A | 164 | -28.228 | -23.515 | 14.210 | 1.00 | 29.69 | S |
| ATOM | 114 | N | TYR | A | 165 | -24.766 | -20.116 | 15.227 | 1.00 | 23.47 | N |
| ATOM | 115 | CA | TYR | A | 165 | -23.469 | -19.539 | 15.507 | 1.00 | 21.97 | C |
| ATOM | 116 | C | TYR | A | 165 | -23.139 | -19.708 | 16.970 | 1.00 | 22.33 | C |
| ATOM | 117 | O | TYR | A | 165 | -23.970 | -19.473 | 17.835 | 1.00 | 21.92 | O |
| ATOM | 118 | CB | TYR | A | 165 | -23.434 | -18.055 | 15.141 | 1.00 | 17.23 | C |
| ATOM | 119 | CG | TYR | A | 165 | -23.665 | -17.761 | 13.679 | 1.00 | 18.76 | C |
| ATOM | 120 | CD1 | TYR | A | 165 | -24.949 | -17.615 | 13.174 | 1.00 | 22.13 | C |
| ATOM | 121 | CD2 | TYR | A | 165 | -22.601 | -17.602 | 12.813 | 1.00 | 21.90 | C |
| ATOM | 122 | CE1 | TYR | A | 165 | -25.169 | -17.327 | 11.833 | 1.00 | 25.39 | C |
| ATOM | 123 | CE2 | TYR | A | 165 | -22.808 | -17.317 | 11.470 | 1.00 | 23.30 | C |
| ATOM | 124 | CZ | TYR | A | 165 | -24.096 | -17.177 | 10.990 | 1.00 | 24.28 | C |
| ATOM | 125 | OH | TYR | A | 165 | -24.295 | -16.900 | 9.663 | 1.00 | 25.37 | O |
| ATOM | 126 | N | TRP | A | 166 | -21.913 | -20.111 | 17.243 | 1.00 | 17.81 | N |
| ATOM | 127 | CA | TRP | A | 166 | -21.436 | -20.129 | 18.601 | 1.00 | 20.06 | C |
| ATOM | 128 | C | TRP | A | 166 | -20.213 | -19.213 | 18.680 | 1.00 | 18.56 | C |
| ATOM | 129 | O | TRP | A | 166 | -19.289 | -19.307 | 17.870 | 1.00 | 18.84 | O |
| ATOM | 130 | CB | TRP | A | 166 | -21.117 | -21.554 | 19.040 | 1.00 | 18.95 | C |
| ATOM | 131 | CG | TRP | A | 166 | -20.709 | -21.665 | 20.486 | 1.00 | 22.40 | C |
| ATOM | 132 | CD1 | TRP | A | 166 | -21.533 | -21.745 | 21.563 | 1.00 | 21.92 | C |
| ATOM | 133 | CD2 | TRP | A | 166 | -19.369 | -21.697 | 21.001 | 1.00 | 22.78 | C |
| ATOM | 134 | NE1 | TRP | A | 166 | -20.797 | -21.831 | 22.717 | 1.00 | 26.27 | N |
| ATOM | 135 | CE2 | TRP | A | 166 | -19.464 | -21.805 | 22.400 | 1.00 | 22.51 | C |
| ATOM | 136 | CE3 | TRP | A | 166 | -18.098 | -21.648 | 20.408 | 1.00 | 20.00 | C |
| ATOM | 137 | CZ2 | TRP | A | 166 | -18.343 | -21.869 | 23.221 | 1.00 | 19.21 | C |
| ATOM | 138 | CZ3 | TRP | A | 166 | -16.989 | -21.716 | 21.214 | 1.00 | 19.92 | C |
| ATOM | 139 | CH2 | TRP | A | 166 | -17.116 | -21.820 | 22.616 | 1.00 | 20.77 | C |
| ATOM | 140 | N | PHE | A | 167 | -20.233 | -18.308 | 19.648 | 1.00 | 20.52 | N |

| ATOM | 141 | CA | PHE | A | 167 | -19.189 | -17.304 | 19.803 | 1.00 | 17.05 | C |
|------|-----|------|-----|---|-----|---------|---------|--------|------|-------|---|
| ATOM | 142 | C | PHE | A | 167 | -18.367 | -17.589 | 21.039 | 1.00 | 23.11 | C |
| ATOM | 143 | O | PHE | A | 167 | -18.847 | -17.397 | 22.149 | 1.00 | 20.92 | O |
| ATOM | 144 | CB | PHE | A | 167 | -19.794 | -15.905 | 19.894 | 1.00 | 17.09 | C |
| ATOM | 145 | CG | PHE | A | 167 | -20.649 | -15.533 | 18.712 | 1.00 | 19.67 | C |
| ATOM | 146 | CD1 | PHE | A | 167 | -22.012 | -15.806 | 18.710 | 1.00 | 20.06 | C |
| ATOM | 147 | CD2 | PHE | A | 167 | -20.087 | -14.907 | 17.598 | 1.00 | 18.66 | C |
| ATOM | 148 | CE1 | PHE | A | 167 | -22.807 | -15.457 | 17.615 | 1.00 | 21.81 | C |
| ATOM | 149 | CE2 | PHE | A | 167 | -20.860 | -14.555 | 16.501 | 1.00 | 14.19 | C |
| ATOM | 150 | CZ | PHE | A | 167 | -22.230 | -14.836 | 16.502 | 1.00 | 20.28 | C |
| ATOM | 151 | N | SER | A | 168 | -17.127 | -18.039 | 20.853 | 1.00 | 20.07 | N |
| ATOM | 152 | CA | SER | A | 168 | -16.296 | -18.360 | 22.000 | 1.00 | 19.88 | C |
| ATOM | 153 | C | SER | A | 168 | -16.073 | -17.121 | 22.847 | 1.00 | 23.65 | C |
| ATOM | 154 | O | SER | A | 168 | -16.105 | -15.992 | 22.331 | 1.00 | 22.61 | O |
| ATOM | 155 | CB | SER | A | 168 | -14.950 | -18.952 | 21.570 | 1.00 | 13.34 | C |
| ATOM | 156 | OG | SER | A | 168 | -14.018 | -17.940 | 21.262 | 1.00 | 16.40 | O |
| ATOM | 157 | N | ARG | A | 169 | -15.877 | -17.339 | 24.149 | 1.00 | 18.01 | N |
| ATOM | 158 | CA | ARG | A | 169 | -15.443 | -16.284 | 25.038 | 1.00 | 22.03 | C |
| ATOM | 159 | C | ARG | A | 169 | -14.033 | -16.614 | 25.568 | 1.00 | 21.36 | C |
| ATOM | 160 | O | ARG | A | 169 | -13.617 | -16.124 | 26.615 | 1.00 | 24.92 | O |
| ATOM | 161 | CB | ARG | A | 169 | -16.447 | -16.082 | 26.182 | 1.00 | 26.09 | C |
| ATOM | 162 | CG | ARG | A | 169 | -17.858 | -15.584 | 25.733 | 1.00 | 33.24 | C |
| ATOM | 163 | CD | ARG | A | 169 | -17.799 | -14.586 | 24.532 | 1.00 | 35.48 | C |
| ATOM | 164 | NE | ARG | A | 169 | -19.120 | -14.199 | 24.007 | 1.00 | 41.60 | N |
| ATOM | 165 | CZ | ARG | A | 169 | -19.315 | -13.401 | 22.953 | 1.00 | 38.23 | C |
| ATOM | 166 | NH1 | ARG | A | 169 | -18.279 | -12.905 | 22.281 | 1.00 | 28.72 | N |
| ATOM | 167 | NH2 | ARG | A | 169 | -20.550 | -13.100 | 22.559 | 1.00 | 36.96 | N |
| ATOM | 168 | N | SER | A | 170 | -13.303 | -17.438 | 24.823 | 1.00 | 16.23 | N |
| ATOM | 169 | CA | SER | A | 170 | -11.877 | -17.660 | 25.065 | 1.00 | 16.00 | C |
| ATOM | 170 | C | SER | A | 170 | -11.094 | -17.698 | 23.742 | 1.00 | 17.67 | C |
| ATOM | 171 | O | SER | A | 170 | -11.662 | -17.537 | 22.663 | 1.00 | 16.96 | O |
| ATOM | 172 | CB | SER | A | 170 | -11.652 | -18.958 | 25.851 | 1.00 | 17.57 | C |
| ATOM | 173 | OG | SER | A | 170 | -12.101 | -20.084 | 25.121 | 1.00 | 18.85 | O |
| ATOM | 174 | N | GLY | A | 171 | -9.783 | -17.916 | 23.824 | 1.00 | 19.23 | N |
| ATOM | 175 | CA | GLY | A | 171 | -8.947 | -17.854 | 22.645 | 1.00 | 12.77 | C |
| ATOM | 176 | C | GLY | A | 171 | -8.169 | -19.115 | 22.334 | 1.00 | 16.65 | C |
| ATOM | 177 | O | GLY | A | 171 | -7.701 | -19.820 | 23.236 | 1.00 | 16.99 | O |
| ATOM | 178 | N | LYS | A | 172 | -8.037 | -19.389 | 21.039 | 1.00 | 15.85 | N |
| ATOM | 179 | CA | LYS | A | 172 | -7.313 | -20.542 | 20.533 | 1.00 | 13.77 | C |
| ATOM | 180 | C | LYS | A | 172 | -6.509 | -20.164 | 19.309 | 1.00 | 17.02 | C |
| ATOM | 181 | O | LYS | A | 172 | -6.873 | -19.224 | 18.576 | 1.00 | 17.20 | O |
| ATOM | 182 | CB | LYS | A | 172 | -8.262 | -21.678 | 20.148 | 1.00 | 16.06 | C |
| ATOM | 183 | CG | LYS | A | 172 | -8.818 | -22.506 | 21.281 | 1.00 | 17.04 | C |
| ATOM | 184 | CD | LYS | A | 172 | -9.638 | -23.666 | 20.687 | 1.00 | 17.13 | C |
| ATOM | 185 | CE | LYS | A | 172 | -10.400 | -24.441 | 21.766 | 1.00 | 16.06 | C |
| ATOM | 186 | NZ | LYS | A | 172 | -9.439 | -25.070 | 22.711 | 1.00 | 20.45 | N |
| ATOM | 187 | N | ALA | A | 173 | -5.427 | -20.902 | 19.074 | 1.00 | 13.08 | N |
| ATOM | 188 | CA | ALA | A | 173 | -4.750 | -20.834 | 17.792 | 1.00 | 14.02 | C |
| ATOM | 189 | C | ALA | A | 173 | -5.758 | -21.289 | 16.754 | 1.00 | 16.04 | C |
| ATOM | 190 | O | ALA | A | 173 | -6.675 | -22.056 | 17.064 | 1.00 | 15.29 | O |
| ATOM | 191 | CB | ALA | A | 173 | -3.482 | -21.720 | 17.766 | 1.00 | 14.17 | C |
| ATOM | 192 | N | TRP | A | 174 | -5.591 | -20.827 | 15.522 | 1.00 | 16.89 | N |
| ATOM | 193 | CA | TRP | A | 174 | -6.571 | -21.098 | 14.492 | 1.00 | 15.29 | C |
| ATOM | 194 | C | TRP | A | 174 | -6.839 | -22.598 | 14.329 | 1.00 | 17.24 | C |
| ATOM | 195 | O | TRP | A | 174 | -8.000 | -23.030 | 14.334 | 1.00 | 17.21 | O |
| ATOM | 196 | CB | TRP | A | 174 | -6.111 | -20.486 | 13.185 | 1.00 | 15.83 | C |
| ATOM | 197 | CG | TRP | A | 174 | -7.133 | -20.523 | 12.123 | 1.00 | 17.80 | C |
| ATOM | 198 | CD1 | TRP | A | 174 | -8.015 | -19.532 | 11.795 | 1.00 | 14.69 | C |
| ATOM | 199 | CD2 | TRP | A | 174 | -7.375 | -21.598 | 11.217 | 1.00 | 17.89 | C |
| ATOM | 200 | NE1 | TRP | A | 174 | -8.784 | -19.926 | 10.732 | 1.00 | 13.89 | N |
| ATOM | 201 | CE2 | TRP | A | 174 | -8.416 | -21.193 | 10.361 | 1.00 | 17.17 | C |
| ATOM | 202 | CE3 | TRP | A | 174 | -6.803 | -22.865 | 11.036 | 1.00 | 20.13 | C |
| ATOM | 203 | CZ2 | TRP | A | 174 | -8.911 | -22.013 | 9.353 | 1.00 | 17.46 | C |
| ATOM | 204 | CZ3 | TRP | A | 174 | -7.294 | -23.675 | 10.039 | 1.00 | 18.74 | C |
| ATOM | 205 | CH2 | TRP | A | 174 | -8.338 | -23.246 | 9.208 | 1.00 | 21.31 | C |
| ATOM | 206 | N | ALA | A | 175 | -5.781 | -23.395 | 14.229 | 1.00 | 14.70 | N |
| ATOM | 207 | CA | ALA | A | 175 | -5.950 | -24.829 | 13.999 | 1.00 | 16.88 | C |
| ATOM | 208 | C | ALA | A | 175 | -6.707 | -25.488 | 15.138 | 1.00 | 18.55 | C |
| ATOM | 209 | O | ALA | A | 175 | -7.444 | -26.448 | 14.921 | 1.00 | 18.63 | O |
| ATOM | 210 | CB | ALA | A | 175 | -4.595 | -25.512 | 13.803 | 1.00 | 20.43 | C |
| ATOM | 211 | N | ASP | A | 176 | -6.530 | -24.982 | 16.355 | 1.00 | 15.66 | N |

```
ATOM    212  CA  ASP A 176      -7.267 -25.535  17.479  1.00 14.07           C
ATOM    213  C   ASP A 176      -8.717 -25.047  17.471  1.00 17.62           C
ATOM    214  O   ASP A 176      -9.621 -25.790  17.834  1.00 19.92           O
ATOM    215  CB  ASP A 176      -6.566 -25.191  18.784  1.00 17.82           C
ATOM    216  CG  ASP A 176      -5.169 -25.805  18.865  1.00 32.45           C
ATOM    217  OD2 ASP A 176      -4.231 -25.127  19.349  1.00 29.94           O
ATOM    218  OD1 ASP A 176      -5.003 -26.959  18.405  1.00 34.43           O
ATOM    219  N   ALA A 177      -8.951 -23.811  17.036  1.00 15.88           N
ATOM    220  CA  ALA A 177     -10.324 -23.342  16.867  1.00 15.36           C
ATOM    221  C   ALA A 177     -11.000 -24.134  15.765  1.00 14.26           C
ATOM    222  O   ALA A 177     -12.176 -24.472  15.856  1.00 17.76           O
ATOM    223  CB  ALA A 177     -10.355 -21.866  16.550  1.00 12.43           C
ATOM    224  N   ASP A 178     -10.250 -24.417  14.710  1.00 17.91           N
ATOM    225  CA  ASP A 178     -10.776 -25.168  13.575  1.00 18.45           C
ATOM    226  C   ASP A 178     -11.241 -26.540  14.045  1.00 20.72           C
ATOM    227  O   ASP A 178     -12.390 -26.930  13.819  1.00 17.75           O
ATOM    228  CB  ASP A 178      -9.714 -25.301  12.493  1.00 18.31           C
ATOM    229  CG  ASP A 178     -10.161 -26.169  11.346  1.00 24.63           C
ATOM    230  OD1 ASP A 178     -11.279 -25.949  10.829  1.00 26.01           O
ATOM    231  OD2 ASP A 178      -9.388 -27.067  10.960  1.00 28.35           O
ATOM    232  N   ASN A 179     -10.343 -27.251  14.729  1.00 18.35           N
ATOM    233  CA  ASN A 179     -10.668 -28.541  15.329  1.00 20.73           C
ATOM    234  C   ASN A 179     -11.839 -28.466  16.310  1.00 21.76           C
ATOM    235  O   ASN A 179     -12.689 -29.359  16.343  1.00 22.85           O
ATOM    236  CB  ASN A 179      -9.443 -29.130  16.037  1.00 19.26           C
ATOM    237  CG  ASN A 179      -9.756 -30.435  16.752  1.00 24.98           C
ATOM    238  OD1 ASN A 179      -9.955 -30.455  17.961  1.00 27.04           O
ATOM    239  ND2 ASN A 179      -9.814 -31.528  16.002  1.00 24.23           N
ATOM    240  N   TYR A 180     -11.897 -27.407  17.110  1.00 17.86           N
ATOM    241  CA  TYR A 180     -13.001 -27.265  18.054  1.00 16.79           C
ATOM    242  C   TYR A 180     -14.342 -27.266  17.313  1.00 19.82           C
ATOM    243  O   TYR A 180     -15.287 -27.948  17.709  1.00 19.73           O
ATOM    244  CB  TYR A 180     -12.861 -25.985  18.891  1.00 16.59           C
ATOM    245  CG  TYR A 180     -14.056 -25.719  19.810  1.00 19.48           C
ATOM    246  CD2 TYR A 180     -13.996 -26.024  21.162  1.00 24.26           C
ATOM    247  CD1 TYR A 180     -15.245 -25.156  19.316  1.00 18.40           C
ATOM    248  CE2 TYR A 180     -15.089 -25.789  22.008  1.00 20.67           C
ATOM    249  CE1 TYR A 180     -16.328 -24.922  20.135  1.00 16.82           C
ATOM    250  CZ  TYR A 180     -16.243 -25.239  21.483  1.00 23.60           C
ATOM    251  OH  TYR A 180     -17.312 -25.009  22.311  1.00 27.99           O
ATOM    252  N   CYS A 181     -14.416 -26.480  16.247  1.00 21.68           N
ATOM    253  CA  CYS A 181     -15.634 -26.361  15.468  1.00 21.18           C
ATOM    254  C   CYS A 181     -16.005 -27.691  14.816  1.00 22.39           C
ATOM    255  O   CYS A 181     -17.173 -28.054  14.738  1.00 17.23           O
ATOM    256  CB  CYS A 181     -15.479 -25.268  14.414  1.00 17.00           C
ATOM    257  SG  CYS A 181     -15.347 -23.612  15.108  1.00 21.92           S
ATOM    258  N   ARG A 182     -15.008 -28.435  14.366  1.00 22.70           N
ATOM    259  CA  ARG A 182     -15.309 -29.699  13.720  1.00 25.40           C
ATOM    260  C   ARG A 182     -15.881 -30.687  14.737  1.00 22.32           C
ATOM    261  O   ARG A 182     -16.756 -31.489  14.417  1.00 21.62           O
ATOM    262  CB  ARG A 182     -14.064 -30.255  13.031  1.00 22.43           C
ATOM    263  CG  ARG A 182     -13.757 -29.535  11.727  1.00 27.35           C
ATOM    264  CD  ARG A 182     -12.390 -29.887  11.188  1.00 27.56           C
ATOM    265  NE  ARG A 182     -11.981 -28.956  10.139  1.00 34.68           N
ATOM    266  CZ  ARG A 182     -12.311 -29.080   8.851  1.00 37.24           C
ATOM    267  NH1 ARG A 182     -13.062 -30.100   8.446  1.00 30.09           N
ATOM    268  NH2 ARG A 182     -11.900 -28.176   7.966  1.00 33.00           N
ATOM    269  N   LEU A 183     -15.415 -30.609  15.975  1.00 22.21           N
ATOM    270  CA  LEU A 183     -15.858 -31.565  16.980  1.00 20.10           C
ATOM    271  C   LEU A 183     -17.289 -31.265  17.408  1.00 22.94           C
ATOM    272  O   LEU A 183     -17.907 -32.076  18.096  1.00 23.53           O
ATOM    273  CB  LEU A 183     -14.918 -31.577  18.182  1.00 19.03           C
ATOM    274  CG  LEU A 183     -13.826 -32.651  18.153  1.00 24.47           C
ATOM    275  CD1 LEU A 183     -13.119 -32.696  16.809  1.00 24.47           C
ATOM    276  CD2 LEU A 183     -12.816 -32.407  19.258  1.00 24.08           C
ATOM    277  N   GLU A 184     -17.809 -30.113  16.977  1.00 23.63           N
ATOM    278  CA  GLU A 184     -19.222 -29.762  17.156  1.00 23.88           C
ATOM    279  C   GLU A 184     -20.075 -29.971  15.910  1.00 22.40           C
ATOM    280  O   GLU A 184     -21.110 -29.330  15.791  1.00 22.93           O
ATOM    281  CB  GLU A 184     -19.372 -28.292  17.560  1.00 23.75           C
ATOM    282  CG  GLU A 184     -18.635 -27.890  18.806  1.00 26.06           C
```

```
ATOM    283  CD   GLU A 184     -19.139 -28.631  20.019  1.00 33.11           C
ATOM    284  OE1  GLU A 184     -20.363 -28.913  20.075  1.00 34.32           O
ATOM    285  OE2  GLU A 184     -18.305 -28.946  20.899  1.00 34.75           O
ATOM    286  N    ASP A 185     -19.651 -30.834  14.989  1.00 21.89           N
ATOM    287  CA   ASP A 185     -20.270 -30.908  13.664  1.00 25.50           C
ATOM    288  C    ASP A 185     -20.452 -29.523  13.066  1.00 22.06           C
ATOM    289  O    ASP A 185     -21.511 -29.206  12.547  1.00 21.39           O
ATOM    290  CB   ASP A 185     -21.637 -31.606  13.701  1.00 40.42           C
ATOM    291  CG   ASP A 185     -21.531 -33.104  13.910  1.00 51.64           C
ATOM    292  OD1  ASP A 185     -20.401 -33.647  13.831  1.00 55.89           O
ATOM    293  OD2  ASP A 185     -22.589 -33.745  14.116  1.00 52.07           O
ATOM    294  N    ALA A 186     -19.419 -28.693  13.152  1.00 25.31           N
ATOM    295  CA   ALA A 186     -19.521 -27.315  12.702  1.00 21.25           C
ATOM    296  C    ALA A 186     -18.262 -26.920  11.989  1.00 18.66           C
ATOM    297  O    ALA A 186     -17.353 -27.723  11.805  1.00 20.79           O
ATOM    298  CB   ALA A 186     -19.779 -26.377  13.882  1.00 18.33           C
ATOM    299  N    HIS A 187     -18.199 -25.658  11.603  1.00 18.96           N
ATOM    300  CA   HIS A 187     -17.012 -25.135  10.969  1.00 17.14           C
ATOM    301  C    HIS A 187     -16.810 -23.710  11.439  1.00 17.01           C
ATOM    302  O    HIS A 187     -17.776 -23.033  11.778  1.00 19.70           O
ATOM    303  CB   HIS A 187     -17.156 -25.191   9.450  1.00 15.59           C
ATOM    304  CG   HIS A 187     -18.323 -24.414   8.940  1.00 15.47           C
ATOM    305  ND1  HIS A 187     -18.281 -23.048   8.761  1.00 15.92           N
ATOM    306  CD2  HIS A 187     -19.578 -24.800   8.606  1.00 18.70           C
ATOM    307  CE1  HIS A 187     -19.456 -22.628   8.323  1.00 18.66           C
ATOM    308  NE2  HIS A 187     -20.261 -23.671   8.223  1.00 21.90           N
ATOM    309  N    LEU A 188     -15.560 -23.264  11.480  1.00 16.40           N
ATOM    310  CA   LEU A 188     -15.256 -21.859  11.698  1.00 14.33           C
ATOM    311  C    LEU A 188     -16.058 -20.996  10.738  1.00 16.82           C
ATOM    312  O    LEU A 188     -16.141 -21.289   9.527  1.00 15.26           O
ATOM    313  CB   LEU A 188     -13.761 -21.602  11.512  1.00 13.49           C
ATOM    314  CG   LEU A 188     -12.853 -21.922  12.696  1.00 16.50           C
ATOM    315  CD1  LEU A 188     -11.390 -21.816  12.290  1.00 18.24           C
ATOM    316  CD2  LEU A 188     -13.163 -20.975  13.841  1.00 15.22           C
ATOM    317  N    VAL A 189     -16.613 -19.915  11.271  1.00 15.19           N
ATOM    318  CA   VAL A 189     -17.596 -19.127  10.543  1.00 13.95           C
ATOM    319  C    VAL A 189     -17.130 -18.784   9.129  1.00 14.59           C
ATOM    320  O    VAL A 189     -15.970 -18.432   8.894  1.00 13.02           O
ATOM    321  CB   VAL A 189     -17.957 -17.836  11.316  1.00 15.19           C
ATOM    322  CG1  VAL A 189     -16.788 -16.866  11.361  1.00 13.58           C
ATOM    323  CG2  VAL A 189     -19.191 -17.168  10.700  1.00 19.68           C
ATOM    324  N    VAL A 190     -18.041 -18.966   8.183  1.00 16.77           N
ATOM    325  CA   VAL A 190     -17.814 -18.617   6.786  1.00 18.83           C
ATOM    326  C    VAL A 190     -18.720 -17.429   6.469  1.00 18.58           C
ATOM    327  O    VAL A 190     -19.925 -17.526   6.604  1.00 17.97           O
ATOM    328  CB   VAL A 190     -18.113 -19.808   5.843  1.00 19.02           C
ATOM    329  CG1  VAL A 190     -18.145 -19.357   4.367  1.00 16.14           C
ATOM    330  CG2  VAL A 190     -17.092 -20.917   6.052  1.00 17.67           C
ATOM    331  N    VAL A 191     -18.146 -16.297   6.086  1.00 16.96           N
ATOM    332  CA   VAL A 191     -18.956 -15.094   5.909  1.00 16.61           C
ATOM    333  C    VAL A 191     -19.163 -14.813   4.424  1.00 21.27           C
ATOM    334  O    VAL A 191     -18.196 -14.541   3.705  1.00 19.05           O
ATOM    335  CB   VAL A 191     -18.302 -13.887   6.589  1.00 19.68           C
ATOM    336  CG1  VAL A 191     -19.161 -12.637   6.414  1.00 16.81           C
ATOM    337  CG2  VAL A 191     -18.073 -14.187   8.076  1.00 17.57           C
ATOM    338  N    THR A 192     -20.410 -14.895   3.960  1.00 17.29           N
ATOM    339  CA   THR A 192     -20.686 -14.767   2.523  1.00 21.54           C
ATOM    340  C    THR A 192     -21.558 -13.571   2.143  1.00 21.36           C
ATOM    341  O    THR A 192     -21.854 -13.372   0.976  1.00 21.17           O
ATOM    342  CB   THR A 192     -21.344 -16.046   1.963  1.00 19.51           C
ATOM    343  OG1  THR A 192     -22.486 -16.392   2.755  1.00 23.52           O
ATOM    344  CG2  THR A 192     -20.354 -17.193   2.007  1.00 19.60           C
ATOM    345  N    SER A 193     -21.938 -12.744   3.107  1.00 18.59           N
ATOM    346  CA   SER A 193     -22.678 -11.549   2.755  1.00 21.89           C
ATOM    347  C    SER A 193     -22.432 -10.451   3.763  1.00 26.64           C
ATOM    348  O    SER A 193     -21.913 -10.693   4.856  1.00 19.28           O
ATOM    349  CB   SER A 193     -24.172 -11.847   2.681  1.00 23.78           C
ATOM    350  OG   SER A 193     -24.655 -12.141   3.986  1.00 23.32           O
ATOM    351  N    TRP A 194     -22.825  -9.241   3.378  1.00 27.24           N
ATOM    352  CA   TRP A 194     -22.794  -8.087   4.260  1.00 24.77           C
ATOM    353  C    TRP A 194     -23.633  -8.331   5.504  1.00 25.57           C
```

| ATOM | 354 | O   | TRP A 194 | -23.199 | -8.061  | 6.624  | 1.00 26.59 | O |
| ATOM | 355 | CB  | TRP A 194 | -23.295 | -6.845  | 3.519  | 1.00 28.68 | C |
| ATOM | 356 | CG  | TRP A 194 | -23.125 | -5.572  | 4.283  | 1.00 38.36 | C |
| ATOM | 357 | CD1 | TRP A 194 | -24.116 | -4.747  | 4.741  | 1.00 37.90 | C |
| ATOM | 358 | CD2 | TRP A 194 | -21.884 | -4.976  | 4.696  | 1.00 44.56 | C |
| ATOM | 359 | NE1 | TRP A 194 | -23.569 | -3.668  | 5.400  | 1.00 43.45 | N |
| ATOM | 360 | CE2 | TRP A 194 | -22.202 | -3.785  | 5.388  | 1.00 46.98 | C |
| ATOM | 361 | CE3 | TRP A 194 | -20.538 | -5.334  | 4.546  | 1.00 36.22 | C |
| ATOM | 362 | CZ2 | TRP A 194 | -21.219 | -2.949  | 5.932  | 1.00 52.52 | C |
| ATOM | 363 | CZ3 | TRP A 194 | -19.560 | -4.503  | 5.089  | 1.00 42.44 | C |
| ATOM | 364 | CH2 | TRP A 194 | -19.908 | -3.325  | 5.773  | 1.00 54.05 | C |
| ATOM | 365 | N   | GLU A 195 | -24.848 | -8.824  | 5.293  | 1.00 26.92 | N |
| ATOM | 366 | CA  | GLU A 195 | -25.764 | -9.112  | 6.385  | 1.00 23.47 | C |
| ATOM | 367 | C   | GLU A 195 | -25.105 | -9.996  | 7.433  | 1.00 23.12 | C |
| ATOM | 368 | O   | GLU A 195 | -25.175 | -9.715  | 8.632  | 1.00 28.18 | O |
| ATOM | 369 | CB  | GLU A 195 | -27.037 | -9.781  | 5.846  | 1.00 30.26 | C |
| ATOM | 370 | CG  | GLU A 195 | -27.820 | -10.627 | 6.876  | 1.00 40.78 | C |
| ATOM | 371 | CD  | GLU A 195 | -27.676 | -12.144 | 6.673  | 1.00 40.62 | C |
| ATOM | 372 | OE1 | GLU A 195 | -27.945 | -12.645 | 5.559  | 1.00 43.62 | O |
| ATOM | 373 | OE2 | GLU A 195 | -27.294 | -12.841 | 7.634  | 1.00 42.10 | O |
| ATOM | 374 | N   | GLU A 196 | -24.454 | -11.059 | 6.975  | 1.00 20.16 | N |
| ATOM | 375 | CA  | GLU A 196 | -23.824 | -12.005 | 7.884  | 1.00 25.02 | C |
| ATOM | 376 | C   | GLU A 196 | -22.610 | -11.407 | 8.600  | 1.00 21.89 | C |
| ATOM | 377 | O   | GLU A 196 | -22.377 | -11.689 | 9.775  | 1.00 18.44 | O |
| ATOM | 378 | CB  | GLU A 196 | -23.416 | -13.265 | 7.136  | 1.00 20.96 | C |
| ATOM | 379 | CG  | GLU A 196 | -22.822 | -14.312 | 8.049  | 1.00 22.24 | C |
| ATOM | 380 | CD  | GLU A 196 | -22.589 | -15.613 | 7.337  | 1.00 22.15 | C |
| ATOM | 381 | OE1 | GLU A 196 | -22.492 | -15.604 | 6.093  | 1.00 19.92 | O |
| ATOM | 382 | OE2 | GLU A 196 | -22.506 | -16.645 | 8.028  | 1.00 24.74 | O |
| ATOM | 383 | N   | GLN A 197 | -21.851 | -10.584 | 7.881  | 1.00 18.28 | N |
| ATOM | 384 | CA  | GLN A 197 | -20.751 | -9.817  | 8.456  | 1.00 18.47 | C |
| ATOM | 385 | C   | GLN A 197 | -21.217 | -8.914  | 9.600  | 1.00 24.91 | C |
| ATOM | 386 | O   | GLN A 197 | -20.639 | -8.924  | 10.694 | 1.00 18.25 | O |
| ATOM | 387 | CB  | GLN A 197 | -20.084 | -8.973  | 7.373  | 1.00 18.68 | C |
| ATOM | 388 | CG  | GLN A 197 | -19.167 | -7.888  | 7.890  | 1.00 23.19 | C |
| ATOM | 389 | CD  | GLN A 197 | -17.832 | -8.428  | 8.348  | 1.00 19.88 | C |
| ATOM | 390 | OE1 | GLN A 197 | -17.290 | -9.361  | 7.758  | 1.00 19.94 | O |
| ATOM | 391 | NE2 | GLN A 197 | -17.289 | -7.840  | 9.406  | 1.00 20.73 | N |
| ATOM | 392 | N   | LYS A 198 | -22.253 | -8.118  | 9.334  | 1.00 26.27 | N |
| ATOM | 393 | CA  | LYS A 198 | -22.775 | -7.197  | 10.330 | 1.00 20.44 | C |
| ATOM | 394 | C   | LYS A 198 | -23.308 | -7.995  | 11.495 | 1.00 18.19 | C |
| ATOM | 395 | O   | LYS A 198 | -23.199 | -7.578  | 12.637 | 1.00 20.09 | O |
| ATOM | 396 | CB  | LYS A 198 | -23.886 | -6.312  | 9.757  | 1.00 26.97 | C |
| ATOM | 397 | CG  | LYS A 198 | -23.441 | -5.226  | 8.806  | 1.00 32.65 | C |
| ATOM | 398 | CD  | LYS A 198 | -24.652 | -4.387  | 8.368  | 1.00 47.94 | C |
| ATOM | 399 | CE  | LYS A 198 | -25.404 | -3.782  | 9.568  | 1.00 44.86 | C |
| ATOM | 400 | NZ  | LYS A 198 | -26.475 | -2.822  | 9.154  | 1.00 46.87 | N |
| ATOM | 401 | N   | PHE A 199 | -23.903 | -9.142  | 11.196 | 1.00 16.78 | N |
| ATOM | 402 | CA  | PHE A 199 | -24.438 | -9.981  | 12.248 | 1.00 18.71 | C |
| ATOM | 403 | C   | PHE A 199 | -23.319 | -10.480 | 13.161 | 1.00 19.39 | C |
| ATOM | 404 | O   | PHE A 199 | -23.448 | -10.481 | 14.383 | 1.00 19.46 | O |
| ATOM | 405 | CB  | PHE A 199 | -25.204 | -11.163 | 11.669 | 1.00 15.44 | C |
| ATOM | 406 | CG  | PHE A 199 | -25.498 | -12.218 | 12.675 | 1.00 18.84 | C |
| ATOM | 407 | CD2 | PHE A 199 | -24.739 | -13.374 | 12.732 | 1.00 22.90 | C |
| ATOM | 408 | CD1 | PHE A 199 | -26.510 | -12.045 | 13.596 | 1.00 23.16 | C |
| ATOM | 409 | CE2 | PHE A 199 | -24.998 | -14.339 | 13.686 | 1.00 25.42 | C |
| ATOM | 410 | CE1 | PHE A 199 | -26.775 | -13.011 | 14.557 | 1.00 24.25 | C |
| ATOM | 411 | CZ  | PHE A 199 | -26.023 | -14.155 | 14.603 | 1.00 20.45 | C |
| ATOM | 412 | N   | VAL A 200 | -22.220 | -10.923 | 12.568 | 1.00 20.88 | N |
| ATOM | 413 | CA  | VAL A 200 | -21.127 | -11.461 | 13.366 | 1.00 19.97 | C |
| ATOM | 414 | C   | VAL A 200 | -20.361 | -10.342 | 14.096 | 1.00 19.76 | C |
| ATOM | 415 | O   | VAL A 200 | -20.037 | -10.494 | 15.267 | 1.00 18.57 | O |
| ATOM | 416 | CB  | VAL A 200 | -20.201 | -12.301 | 12.496 | 1.00 20.92 | C |
| ATOM | 417 | CG1 | VAL A 200 | -18.898 | -12.658 | 13.243 | 1.00 18.59 | C |
| ATOM | 418 | CG2 | VAL A 200 | -20.942 | -13.551 | 12.046 | 1.00 16.54 | C |
| ATOM | 419 | N   | GLN A 201 | -20.126 | -9.211  | 13.430 | 1.00 19.23 | N |
| ATOM | 420 | CA  | GLN A 201 | -19.564 | -8.024  | 14.096 | 1.00 22.96 | C |
| ATOM | 421 | C   | GLN A 201 | -20.288 | -7.714  | 15.403 | 1.00 25.56 | C |
| ATOM | 422 | O   | GLN A 201 | -19.649 | -7.493  | 16.437 | 1.00 22.68 | O |
| ATOM | 423 | CB  | GLN A 201 | -19.648 | -6.781  | 13.199 | 1.00 23.94 | C |
| ATOM | 424 | CG  | GLN A 201 | -18.493 | -6.549  | 12.235 | 1.00 24.31 | C |

| ATOM | 425 | CD | GLN A 201 | -18.865 | -5.570 | 11.096 | 1.00 | 31.70 | C |
|------|-----|-----|-----------|---------|--------|--------|------|-------|---|
| ATOM | 426 | OE1 | GLN A 201 | -18.274 | -5.595 | 10.007 | 1.00 | 25.36 | O |
| ATOM | 427 | NE2 | GLN A 201 | -19.856 | -4.718 | 11.349 | 1.00 | 28.05 | N |
| ATOM | 428 | N | HIS A 202 | -21.625 | -7.687 | 15.349 | 1.00 | 19.95 | N |
| ATOM | 429 | CA | HIS A 202 | -22.415 | -7.330 | 16.514 | 1.00 | 20.60 | C |
| ATOM | 430 | C | HIS A 202 | -22.126 | -8.228 | 17.715 | 1.00 | 23.45 | C |
| ATOM | 431 | O | HIS A 202 | -21.915 | -7.742 | 18.822 | 1.00 | 26.55 | O |
| ATOM | 432 | CB | HIS A 202 | -23.906 | -7.382 | 16.207 | 1.00 | 22.12 | C |
| ATOM | 433 | CG | HIS A 202 | -24.758 | -7.236 | 17.425 | 1.00 | 21.72 | C |
| ATOM | 434 | ND1 | HIS A 202 | -25.123 | -6.010 | 17.931 | 1.00 | 25.57 | N |
| ATOM | 435 | CD2 | HIS A 202 | -25.276 | -8.160 | 18.267 | 1.00 | 25.38 | C |
| ATOM | 436 | CE1 | HIS A 202 | -25.846 | -6.183 | 19.022 | 1.00 | 23.46 | C |
| ATOM | 437 | NE2 | HIS A 202 | -25.958 | -7.481 | 19.244 | 1.00 | 23.52 | N |
| ATOM | 438 | N | HIS A 203 | -22.128 | -9.537 | 17.495 | 1.00 | 22.74 | N |
| ATOM | 439 | CA | HIS A 203 | -21.920 | -10.478 | 18.584 | 1.00 | 21.81 | C |
| ATOM | 440 | C | HIS A 203 | -20.469 | -10.633 | 19.043 | 1.00 | 21.12 | C |
| ATOM | 441 | O | HIS A 203 | -20.243 | -11.066 | 20.168 | 1.00 | 31.14 | O |
| ATOM | 442 | CB | HIS A 203 | -22.455 | -11.849 | 18.201 | 1.00 | 19.70 | C |
| ATOM | 443 | CG | HIS A 203 | -23.947 | -11.937 | 18.209 | 1.00 | 27.48 | C |
| ATOM | 444 | ND1 | HIS A 203 | -24.669 | -12.220 | 19.349 | 1.00 | 27.80 | N |
| ATOM | 445 | CD2 | HIS A 203 | -24.855 | -11.767 | 17.220 | 1.00 | 22.28 | C |
| ATOM | 446 | CE1 | HIS A 203 | -25.958 | -12.223 | 19.061 | 1.00 | 21.84 | C |
| ATOM | 447 | NE2 | HIS A 203 | -26.097 | -11.945 | 17.777 | 1.00 | 20.85 | N |
| ATOM | 448 | N | ILE A 204 | -19.487 | -10.319 | 18.206 | 1.00 | 19.01 | N |
| ATOM | 449 | CA | ILE A 204 | -18.102 | -10.491 | 18.664 | 1.00 | 24.65 | C |
| ATOM | 450 | C | ILE A 204 | -17.506 | -9.213 | 19.247 | 1.00 | 23.37 | C |
| ATOM | 451 | O | ILE A 204 | -16.502 | -9.262 | 19.956 | 1.00 | 29.46 | O |
| ATOM | 452 | CB | ILE A 204 | -17.156 | -10.992 | 17.550 | 1.00 | 19.60 | C |
| ATOM | 453 | CG1 | ILE A 204 | -17.032 | -9.970 | 16.421 | 1.00 | 22.94 | C |
| ATOM | 454 | CG2 | ILE A 204 | -17.581 | -12.351 | 17.037 | 1.00 | 18.69 | C |
| ATOM | 455 | CD1 | ILE A 204 | -15.981 | -10.357 | 15.400 | 1.00 | 18.85 | C |
| ATOM | 456 | N | GLY A 205 | -18.118 | -8.075 | 18.954 | 1.00 | 21.26 | N |
| ATOM | 457 | CA | GLY A 205 | -17.663 | -6.811 | 19.504 | 1.00 | 22.73 | C |
| ATOM | 458 | C | GLY A 205 | -16.273 | -6.438 | 19.025 | 1.00 | 24.53 | C |
| ATOM | 459 | O | GLY A 205 | -15.782 | -6.990 | 18.041 | 1.00 | 29.29 | O |
| ATOM | 460 | N | PRO A 206 | -15.614 | -5.508 | 19.729 | 1.00 | 27.53 | N |
| ATOM | 461 | CA | PRO A 206 | -14.313 | -5.000 | 19.264 | 1.00 | 25.17 | C |
| ATOM | 462 | C | PRO A 206 | -13.141 | -5.982 | 19.496 | 1.00 | 23.26 | C |
| ATOM | 463 | O | PRO A 206 | -12.078 | -5.584 | 19.979 | 1.00 | 27.76 | O |
| ATOM | 464 | CB | PRO A 206 | -14.137 | -3.729 | 20.092 | 1.00 | 24.03 | C |
| ATOM | 465 | CG | PRO A 206 | -14.809 | -4.066 | 21.394 | 1.00 | 21.52 | C |
| ATOM | 466 | CD | PRO A 206 | -16.016 | -4.911 | 21.018 | 1.00 | 22.26 | C |
| ATOM | 467 | N | VAL A 207 | -13.325 | -7.240 | 19.114 | 1.00 | 21.78 | N |
| ATOM | 468 | CA | VAL A 207 | -12.381 | -8.298 | 19.464 | 1.00 | 22.06 | C |
| ATOM | 469 | C | VAL A 207 | -11.844 | -9.063 | 18.248 | 1.00 | 21.75 | C |
| ATOM | 470 | O | VAL A 207 | -12.602 | -9.464 | 17.361 | 1.00 | 16.31 | O |
| ATOM | 471 | CB | VAL A 207 | -13.044 | -9.308 | 20.425 | 1.00 | 18.92 | C |
| ATOM | 472 | CG1 | VAL A 207 | -12.057 | -10.360 | 20.865 | 1.00 | 16.73 | C |
| ATOM | 473 | CG2 | VAL A 207 | -13.637 | -8.587 | 21.612 | 1.00 | 15.45 | C |
| ATOM | 474 | N | ASN A 208 | -10.532 | -9.277 | 18.220 | 1.00 | 20.49 | N |
| ATOM | 475 | CA | ASN A 208 | -9.922 | -10.105 | 17.191 | 1.00 | 18.16 | C |
| ATOM | 476 | C | ASN A 208 | -10.499 | -11.507 | 17.256 | 1.00 | 15.84 | C |
| ATOM | 477 | O | ASN A 208 | -10.423 | -12.147 | 18.294 | 1.00 | 16.63 | O |
| ATOM | 478 | CB | ASN A 208 | -8.413 | -10.156 | 17.367 | 1.00 | 16.49 | C |
| ATOM | 479 | CG | ASN A 208 | -7.741 | -8.839 | 17.055 | 1.00 | 18.40 | C |
| ATOM | 480 | OD1 | ASN A 208 | -6.915 | -8.354 | 17.828 | 1.00 | 21.56 | O |
| ATOM | 481 | ND2 | ASN A 208 | -8.067 | -8.269 | 15.912 | 1.00 | 15.53 | N |
| ATOM | 482 | N | THR A 209 | -11.073 | -11.982 | 16.157 | 1.00 | 14.76 | N |
| ATOM | 483 | CA | THR A 209 | -11.804 | -13.251 | 16.157 | 1.00 | 13.30 | C |
| ATOM | 484 | C | THR A 209 | -11.524 | -14.065 | 14.892 | 1.00 | 14.80 | C |
| ATOM | 485 | O | THR A 209 | -11.708 | -13.581 | 13.778 | 1.00 | 13.11 | O |
| ATOM | 486 | CB | THR A 209 | -13.330 | -13.001 | 16.288 | 1.00 | 15.05 | C |
| ATOM | 487 | OG1 | THR A 209 | -13.573 | -12.186 | 17.436 | 1.00 | 17.23 | O |
| ATOM | 488 | CG2 | THR A 209 | -14.102 | -14.310 | 16.450 | 1.00 | 12.52 | C |
| ATOM | 489 | N | TRP A 210 | -11.075 | -15.300 | 15.058 | 1.00 | 12.28 | N |
| ATOM | 490 | CA | TRP A 210 | -10.804 | -16.146 | 13.905 | 1.00 | 13.62 | C |
| ATOM | 491 | C | TRP A 210 | -12.057 | -16.440 | 13.089 | 1.00 | 15.28 | C |
| ATOM | 492 | O | TRP A 210 | -13.124 | -16.679 | 13.649 | 1.00 | 14.02 | O |
| ATOM | 493 | CB | TRP A 210 | -10.197 | -17.482 | 14.330 | 1.00 | 13.45 | C |
| ATOM | 494 | CG | TRP A 210 | -8.783 | -17.472 | 14.852 | 1.00 | 12.79 | C |
| ATOM | 495 | CD1 | TRP A 210 | -8.352 | -18.038 | 16.003 | 1.00 | 14.29 | C |

| ATOM | 496 | CD2 | TRP | A | 210 | -7.619 | -16.907 | 14.223 | 1.00 | 12.57 | C |
|------|-----|-----|-----|---|-----|--------|---------|--------|------|-------|---|
| ATOM | 497 | NE1 | TRP | A | 210 | -6.992 | -17.868 | 16.141 | 1.00 | 14.57 | N |
| ATOM | 498 | CE2 | TRP | A | 210 | -6.521 | -17.177 | 15.057 | 1.00 | 12.81 | C |
| ATOM | 499 | CE3 | TRP | A | 210 | -7.401 | -16.202 | 13.033 | 1.00 | 13.91 | C |
| ATOM | 500 | CZ2 | TRP | A | 210 | -5.225 | -16.755 | 14.754 | 1.00 | 15.48 | C |
| ATOM | 501 | CZ3 | TRP | A | 210 | -6.107 | -15.791 | 12.729 | 1.00 | 15.74 | C |
| ATOM | 502 | CH2 | TRP | A | 210 | -5.038 | -16.076 | 13.582 | 1.00 | 14.80 | C |
| ATOM | 503 | N | MET | A | 211 | -11.917 | -16.443 | 11.763 | 1.00 | 14.46 | N |
| ATOM | 504 | CA | MET | A | 211 | -12.972 | -16.939 | 10.897 | 1.00 | 13.94 | C |
| ATOM | 505 | C | MET | A | 211 | -12.389 | -18.101 | 10.107 | 1.00 | 18.15 | C |
| ATOM | 506 | O | MET | A | 211 | -11.183 | -18.368 | 10.203 | 1.00 | 14.39 | O |
| ATOM | 507 | CB | MET | A | 211 | -13.511 | -15.843 | 9.973 | 1.00 | 14.29 | C |
| ATOM | 508 | CG | MET | A | 211 | -12.639 | -15.552 | 8.758 | 1.00 | 15.21 | C |
| ATOM | 509 | SD | MET | A | 211 | -13.121 | -14.063 | 7.849 | 1.00 | 15.99 | S |
| ATOM | 510 | CE | MET | A | 211 | -12.617 | -12.803 | 9.002 | 1.00 | 13.06 | C |
| ATOM | 511 | N | GLY | A | 212 | -13.234 | -18.800 | 9.348 | 1.00 | 16.19 | N |
| ATOM | 512 | CA | GLY | A | 212 | -12.811 | -20.001 | 8.645 | 1.00 | 14.73 | C |
| ATOM | 513 | C | GLY | A | 212 | -12.179 | -19.746 | 7.292 | 1.00 | 14.61 | C |
| ATOM | 514 | O | GLY | A | 212 | -12.524 | -20.367 | 6.306 | 1.00 | 14.25 | O |
| ATOM | 515 | N | LEU | A | 213 | -11.231 | -18.824 | 7.260 | 1.00 | 15.62 | N |
| ATOM | 516 | CA | LEU | A | 213 | -10.589 | -18.426 | 6.023 | 1.00 | 13.96 | C |
| ATOM | 517 | C | LEU | A | 213 | -9.080 | -18.461 | 6.261 | 1.00 | 16.13 | C |
| ATOM | 518 | O | LEU | A | 213 | -8.604 | -17.950 | 7.283 | 1.00 | 13.92 | O |
| ATOM | 519 | CB | LEU | A | 213 | -11.069 | -17.041 | 5.609 | 1.00 | 12.59 | C |
| ATOM | 520 | CG | LEU | A | 213 | -10.425 | -16.377 | 4.404 | 1.00 | 13.49 | C |
| ATOM | 521 | CD1 | LEU | A | 213 | -10.715 | -17.173 | 3.139 | 1.00 | 12.34 | C |
| ATOM | 522 | CD2 | LEU | A | 213 | -10.899 | -14.942 | 4.283 | 1.00 | 13.03 | C |
| ATOM | 523 | N | HIS | A | 214 | -8.348 | -19.107 | 5.356 | 1.00 | 14.35 | N |
| ATOM | 524 | CA | HIS | A | 214 | -6.894 | -19.291 | 5.509 | 1.00 | 16.41 | C |
| ATOM | 525 | C | HIS | A | 214 | -6.244 | -19.539 | 4.160 | 1.00 | 18.20 | C |
| ATOM | 526 | O | HIS | A | 214 | -6.940 | -19.874 | 3.207 | 1.00 | 16.31 | O |
| ATOM | 527 | CB | HIS | A | 214 | -6.577 | -20.464 | 6.440 | 1.00 | 13.36 | C |
| ATOM | 528 | CG | HIS | A | 214 | -6.949 | -21.797 | 5.874 | 1.00 | 17.43 | C |
| ATOM | 529 | ND1 | HIS | A | 214 | -6.016 | -22.687 | 5.391 | 1.00 | 23.48 | N |
| ATOM | 530 | CD2 | HIS | A | 214 | -8.158 | -22.382 | 5.690 | 1.00 | 19.34 | C |
| ATOM | 531 | CE1 | HIS | A | 214 | -6.630 | -23.772 | 4.949 | 1.00 | 21.50 | C |
| ATOM | 532 | NE2 | HIS | A | 214 | -7.930 | -23.613 | 5.122 | 1.00 | 22.11 | N |
| ATOM | 533 | N | ASP | A | 215 | -4.920 | -19.376 | 4.079 | 1.00 | 18.22 | N |
| ATOM | 534 | CA | ASP | A | 215 | -4.188 | -19.750 | 2.867 | 1.00 | 17.30 | C |
| ATOM | 535 | C | ASP | A | 215 | -2.925 | -20.539 | 3.240 | 1.00 | 19.55 | C |
| ATOM | 536 | O | ASP | A | 215 | -1.834 | -20.316 | 2.712 | 1.00 | 19.31 | O |
| ATOM | 537 | CB | ASP | A | 215 | -3.849 | -18.504 | 2.029 | 1.00 | 14.78 | C |
| ATOM | 538 | CG | ASP | A | 215 | -2.730 | -17.641 | 2.638 | 1.00 | 17.69 | C |
| ATOM | 539 | OD1 | ASP | A | 215 | -2.483 | -17.688 | 3.869 | 1.00 | 15.15 | O |
| ATOM | 540 | OD2 | ASP | A | 215 | -2.095 | -16.899 | 1.866 | 1.00 | 17.44 | O |
| ATOM | 541 | N | GLN | A | 216 | -3.078 | -21.470 | 4.167 | 1.00 | 21.06 | N |
| ATOM | 542 | CA | GLN | A | 216 | -1.934 | -22.227 | 4.657 | 1.00 | 24.33 | C |
| ATOM | 543 | C | GLN | A | 216 | -1.340 | -23.160 | 3.607 | 1.00 | 21.68 | C |
| ATOM | 544 | O | GLN | A | 216 | -0.150 | -23.452 | 3.649 | 1.00 | 24.43 | O |
| ATOM | 545 | CB | GLN | A | 216 | -2.333 | -23.029 | 5.886 | 1.00 | 23.90 | C |
| ATOM | 546 | CG | GLN | A | 216 | -2.684 | -22.189 | 7.079 | 1.00 | 20.99 | C |
| ATOM | 547 | CD | GLN | A | 216 | -3.202 | -23.036 | 8.215 | 1.00 | 30.17 | C |
| ATOM | 548 | OE1 | GLN | A | 216 | -4.266 | -23.648 | 8.110 | 1.00 | 26.71 | O |
| ATOM | 549 | NE2 | GLN | A | 216 | -2.440 | -23.098 | 9.305 | 1.00 | 40.70 | N |
| ATOM | 550 | N | ASN | A | 217 | -2.162 | -23.621 | 2.668 | 1.00 | 23.32 | N |
| ATOM | 551 | CA | ASN | A | 217 | -1.685 | -24.532 | 1.629 | 1.00 | 20.04 | C |
| ATOM | 552 | C | ASN | A | 217 | -1.612 | -23.852 | 0.283 | 1.00 | 24.86 | C |
| ATOM | 553 | O | ASN | A | 217 | -1.540 | -24.510 | -0.756 | 1.00 | 27.21 | O |
| ATOM | 554 | CB | ASN | A | 217 | -2.588 | -25.759 | 1.517 | 1.00 | 28.11 | C |
| ATOM | 555 | CG | ASN | A | 217 | -2.888 | -26.378 | 2.852 | 1.00 | 32.30 | C |
| ATOM | 556 | OD1 | ASN | A | 217 | -4.055 | -26.547 | 3.218 | 1.00 | 38.88 | O |
| ATOM | 557 | ND2 | ASN | A | 217 | -1.841 | -26.715 | 3.600 | 1.00 | 27.42 | N |
| ATOM | 558 | N | GLY | A | 218 | -1.658 | -22.529 | 0.295 | 1.00 | 22.21 | N |
| ATOM | 559 | CA | GLY | A | 218 | -1.460 | -21.777 | -0.918 | 1.00 | 20.14 | C |
| ATOM | 560 | C | GLY | A | 218 | -2.428 | -20.632 | -1.068 | 1.00 | 22.88 | C |
| ATOM | 561 | O | GLY | A | 218 | -2.165 | -19.536 | -0.571 | 1.00 | 23.78 | O |
| ATOM | 562 | N | PRO | A | 219 | -3.534 | -20.871 | -1.791 | 1.00 | 26.59 | N |
| ATOM | 563 | CA | PRO | A | 219 | -4.537 | -19.838 | -2.062 | 1.00 | 23.67 | C |
| ATOM | 564 | C | PRO | A | 219 | -5.541 | -19.712 | -0.933 | 1.00 | 20.80 | C |
| ATOM | 565 | O | PRO | A | 219 | -5.700 | -20.647 | -0.143 | 1.00 | 21.41 | O |
| ATOM | 566 | CB | PRO | A | 219 | -5.216 | -20.342 | -3.341 | 1.00 | 21.68 | C |

| ATOM | 567 | CG | PRO A 219 | -5.134 | -21.823 | -3.228 | 1.00 | 22.20 | C |
|---|---|---|---|---|---|---|---|---|---|
| ATOM | 568 | CD | PRO A 219 | -3.775 | -22.087 | -2.592 | 1.00 | 27.98 | C |
| ATOM | 569 | N | TRP A 220 | -6.210 | -18.564 | -0.867 | 1.00 | 19.23 | N |
| ATOM | 570 | CA | TRP A 220 | -7.281 | -18.357 | 0.093 | 1.00 | 20.89 | C |
| ATOM | 571 | C | TRP A 220 | -8.394 | -19.370 | -0.119 | 1.00 | 18.11 | C |
| ATOM | 572 | O | TRP A 220 | -8.890 | -19.549 | -1.237 | 1.00 | 16.24 | O |
| ATOM | 573 | CB | TRP A 220 | -7.818 | -16.924 | 0.000 | 1.00 | 20.98 | C |
| ATOM | 574 | CG | TRP A 220 | -6.855 | -15.955 | 0.591 | 1.00 | 19.44 | C |
| ATOM | 575 | CD1 | TRP A 220 | -6.071 | -15.058 | -0.079 | 1.00 | 22.05 | C |
| ATOM | 576 | CD2 | TRP A 220 | -6.519 | -15.825 | 1.977 | 1.00 | 16.53 | C |
| ATOM | 577 | NE1 | TRP A 220 | -5.286 | -14.361 | 0.811 | 1.00 | 14.72 | N |
| ATOM | 578 | CE2 | TRP A 220 | -5.539 | -14.815 | 2.077 | 1.00 | 13.97 | C |
| ATOM | 579 | CE3 | TRP A 220 | -6.958 | -16.456 | 3.143 | 1.00 | 12.82 | C |
| ATOM | 580 | CZ2 | TRP A 220 | -4.997 | -14.422 | 3.295 | 1.00 | 16.02 | C |
| ATOM | 581 | CZ3 | TRP A 220 | -6.422 | -16.062 | 4.347 | 1.00 | 15.01 | C |
| ATOM | 582 | CH2 | TRP A 220 | -5.449 | -15.060 | 4.418 | 1.00 | 18.72 | C |
| ATOM | 583 | N | LYS A 221 | -8.744 | -20.038 | 0.979 | 1.00 | 18.43 | N |
| ATOM | 584 | CA | LYS A 221 | -9.784 | -21.059 | 1.030 | 1.00 | 20.12 | C |
| ATOM | 585 | C | LYS A 221 | -10.651 | -20.866 | 2.269 | 1.00 | 17.14 | C |
| ATOM | 586 | O | LYS A 221 | -10.133 | -20.648 | 3.354 | 1.00 | 17.85 | O |
| ATOM | 587 | CB | LYS A 221 | -9.169 | -22.463 | 1.059 | 1.00 | 22.71 | C |
| ATOM | 588 | CG | LYS A 221 | -8.599 | -22.928 | -0.263 | 1.00 | 30.31 | C |
| ATOM | 589 | CD | LYS A 221 | -7.862 | -24.250 | -0.113 | 1.00 | 39.91 | C |
| ATOM | 590 | CE | LYS A 221 | -7.419 | -24.776 | -1.471 | 1.00 | 44.01 | C |
| ATOM | 591 | NZ | LYS A 221 | -6.601 | -26.016 | -1.336 | 1.00 | 58.69 | N |
| ATOM | 592 | N | TRP A 222 | -11.965 | -20.938 | 2.096 | 1.00 | 20.05 | N |
| ATOM | 593 | CA | TRP A 222 | -12.893 | -21.059 | 3.220 | 1.00 | 18.07 | C |
| ATOM | 594 | C | TRP A 222 | -12.973 | -22.520 | 3.658 | 1.00 | 15.25 | C |
| ATOM | 595 | O | TRP A 222 | -12.883 | -23.404 | 2.821 | 1.00 | 22.45 | O |
| ATOM | 596 | CB | TRP A 222 | -14.280 | -20.553 | 2.827 | 1.00 | 18.47 | C |
| ATOM | 597 | CG | TRP A 222 | -14.383 | -19.074 | 2.639 | 1.00 | 15.11 | C |
| ATOM | 598 | CD1 | TRP A 222 | -14.384 | -18.397 | 1.458 | 1.00 | 15.59 | C |
| ATOM | 599 | CD2 | TRP A 222 | -14.536 | -18.090 | 3.667 | 1.00 | 12.30 | C |
| ATOM | 600 | NE1 | TRP A 222 | -14.525 | -17.045 | 1.686 | 1.00 | 17.01 | N |
| ATOM | 601 | CE2 | TRP A 222 | -14.621 | -16.834 | 3.037 | 1.00 | 14.83 | C |
| ATOM | 602 | CE3 | TRP A 222 | -14.607 | -18.148 | 5.058 | 1.00 | 15.63 | C |
| ATOM | 603 | CZ2 | TRP A 222 | -14.774 | -15.651 | 3.749 | 1.00 | 14.19 | C |
| ATOM | 604 | CZ3 | TRP A 222 | -14.750 | -16.975 | 5.761 | 1.00 | 14.82 | C |
| ATOM | 605 | CH2 | TRP A 222 | -14.833 | -15.741 | 5.103 | 1.00 | 16.93 | C |
| ATOM | 606 | N | VAL A 223 | -13.160 | -22.781 | 4.948 | 1.00 | 18.67 | N |
| ATOM | 607 | CA | VAL A 223 | -13.104 | -24.154 | 5.453 | 1.00 | 21.37 | C |
| ATOM | 608 | C | VAL A 223 | -14.214 | -25.092 | 4.955 | 1.00 | 19.54 | C |
| ATOM | 609 | O | VAL A 223 | -14.002 | -26.293 | 4.883 | 1.00 | 26.83 | O |
| ATOM | 610 | CB | VAL A 223 | -13.127 | -24.195 | 7.000 | 1.00 | 20.27 | C |
| ATOM | 611 | CG1 | VAL A 223 | -11.823 | -23.684 | 7.550 | 1.00 | 16.93 | C |
| ATOM | 612 | CG2 | VAL A 223 | -14.323 | -23.424 | 7.546 | 1.00 | 13.22 | C |
| ATOM | 613 | N | ASP A 224 | -15.391 | -24.575 | 4.627 | 1.00 | 20.49 | N |
| ATOM | 614 | CA | ASP A 224 | -16.455 | -25.453 | 4.129 | 1.00 | 28.05 | C |
| ATOM | 615 | C | ASP A 224 | -16.450 | -25.610 | 2.592 | 1.00 | 31.47 | C |
| ATOM | 616 | O | ASP A 224 | -17.344 | -26.231 | 2.017 | 1.00 | 34.41 | O |
| ATOM | 617 | CB | ASP A 224 | -17.832 | -24.956 | 4.606 | 1.00 | 26.17 | C |
| ATOM | 618 | CG | ASP A 224 | -18.313 | -23.720 | 3.861 | 1.00 | 30.40 | C |
| ATOM | 619 | OD1 | ASP A 224 | -17.480 | -22.971 | 3.288 | 1.00 | 27.86 | O |
| ATOM | 620 | OD2 | ASP A 224 | -19.548 | -23.486 | 3.866 | 1.00 | 41.83 | O |
| ATOM | 621 | N | GLY A 225 | -15.442 | -25.060 | 1.930 | 1.00 | 26.37 | N |
| ATOM | 622 | CA | GLY A 225 | -15.325 | -25.234 | 0.495 | 1.00 | 24.35 | C |
| ATOM | 623 | C | GLY A 225 | -15.828 | -24.060 | -0.325 | 1.00 | 26.11 | C |
| ATOM | 624 | O | GLY A 225 | -15.605 | -24.021 | -1.532 | 1.00 | 30.45 | O |
| ATOM | 625 | N | THR A 226 | -16.512 | -23.114 | 0.322 | 1.00 | 28.38 | N |
| ATOM | 626 | CA | THR A 226 | -16.991 | -21.894 | -0.346 | 1.00 | 26.57 | C |
| ATOM | 627 | C | THR A 226 | -15.853 | -21.219 | -1.107 | 1.00 | 27.23 | C |
| ATOM | 628 | O | THR A 226 | -14.732 | -21.105 | -0.602 | 1.00 | 25.68 | O |
| ATOM | 629 | CB | THR A 226 | -17.587 | -20.886 | 0.669 | 1.00 | 26.04 | C |
| ATOM | 630 | OG1 | THR A 226 | -18.644 | -21.511 | 1.408 | 1.00 | 31.78 | O |
| ATOM | 631 | CG2 | THR A 226 | -18.109 | -19.649 | -0.025 | 1.00 | 22.83 | C |
| ATOM | 632 | N | ASP A 227 | -16.131 | -20.791 | -2.331 | 1.00 | 25.74 | N |
| ATOM | 633 | CA | ASP A 227 | -15.101 | -20.198 | -3.157 | 1.00 | 24.58 | C |
| ATOM | 634 | C | ASP A 227 | -14.769 | -18.774 | -2.724 | 1.00 | 27.29 | C |
| ATOM | 635 | O | ASP A 227 | -15.658 | -17.943 | -2.523 | 1.00 | 23.84 | O |
| ATOM | 636 | CB | ASP A 227 | -15.523 | -20.199 | -4.625 | 1.00 | 31.00 | C |
| ATOM | 637 | CG | ASP A 227 | -14.712 | -19.226 | -5.447 | 1.00 | 33.32 | C |

```
ATOM    638  OD1 ASP A 227     -13.509 -19.502  -5.653  1.00 36.87           O
ATOM    639  OD2 ASP A 227     -15.261 -18.175  -5.849  1.00 32.69           O
ATOM    640  N   TYR A 228     -13.480 -18.485  -2.609  1.00 25.74           N
ATOM    641  CA  TYR A 228     -13.055 -17.195  -2.090  1.00 22.35           C
ATOM    642  C   TYR A 228     -13.186 -16.074  -3.109  1.00 22.96           C
ATOM    643  O   TYR A 228     -13.706 -15.006  -2.779  1.00 21.88           O
ATOM    644  CB  TYR A 228     -11.610 -17.272  -1.590  1.00 17.43           C
ATOM    645  CG  TYR A 228     -10.989 -15.928  -1.261  1.00 18.06           C
ATOM    646  CD2 TYR A 228     -10.176 -15.269  -2.181  1.00 19.30           C
ATOM    647  CD1 TYR A 228     -11.208 -15.316  -0.019  1.00 18.53           C
ATOM    648  CE2 TYR A 228      -9.602 -14.021  -1.886  1.00 18.76           C
ATOM    649  CE1 TYR A 228     -10.630 -14.081   0.293  1.00 15.99           C
ATOM    650  CZ  TYR A 228      -9.826 -13.436  -0.643  1.00 20.11           C
ATOM    651  OH  TYR A 228      -9.245 -12.212  -0.342  1.00 16.25           O
ATOM    652  N   GLU A 229     -12.717 -16.293  -4.339  1.00 24.84           N
ATOM    653  CA  GLU A 229     -12.560 -15.153  -5.244  1.00 26.92           C
ATOM    654  C   GLU A 229     -13.890 -14.532  -5.653  1.00 27.92           C
ATOM    655  O   GLU A 229     -13.958 -13.314  -5.816  1.00 23.76           O
ATOM    656  CB  GLU A 229     -11.744 -15.529  -6.482  1.00 31.64           C
ATOM    657  CG  GLU A 229     -12.229 -16.734  -7.265  1.00 42.79           C
ATOM    658  CD  GLU A 229     -11.112 -17.346  -8.115  1.00 43.21           C
ATOM    659  OE1 GLU A 229     -10.142 -16.627  -8.448  1.00 39.47           O
ATOM    660  OE2 GLU A 229     -11.196 -18.552  -8.440  1.00 52.24           O
ATOM    661  N   THR A 230     -14.945 -15.341  -5.782  1.00 26.03           N
ATOM    662  CA  THR A 230     -16.276 -14.802  -6.087  1.00 26.83           C
ATOM    663  C   THR A 230     -17.120 -14.519  -4.847  1.00 30.75           C
ATOM    664  O   THR A 230     -18.233 -14.006  -4.945  1.00 30.18           O
ATOM    665  CB  THR A 230     -17.090 -15.754  -6.977  1.00 27.80           C
ATOM    666  OG1 THR A 230     -17.113 -17.052  -6.378  1.00 25.69           O
ATOM    667  CG2 THR A 230     -16.472 -15.855  -8.362  1.00 25.73           C
ATOM    668  N   GLY A 231     -16.610 -14.872  -3.678  1.00 25.26           N
ATOM    669  CA  GLY A 231     -17.362 -14.653  -2.463  1.00 27.26           C
ATOM    670  C   GLY A 231     -17.154 -13.274  -1.878  1.00 26.63           C
ATOM    671  O   GLY A 231     -16.290 -12.519  -2.321  1.00 25.15           O
ATOM    672  N   PHE A 232     -17.973 -12.959  -0.882  1.00 23.85           N
ATOM    673  CA  PHE A 232     -17.839 -11.763  -0.056  1.00 25.36           C
ATOM    674  C   PHE A 232     -16.433 -11.627   0.530  1.00 22.51           C
ATOM    675  O   PHE A 232     -15.815 -12.623   0.898  1.00 24.64           O
ATOM    676  CB  PHE A 232     -18.875 -11.820   1.062  1.00 23.46           C
ATOM    677  CG  PHE A 232     -18.859 -10.645   1.971  1.00 24.65           C
ATOM    678  CD1 PHE A 232     -19.395  -9.429   1.564  1.00 19.49           C
ATOM    679  CD2 PHE A 232     -18.354 -10.760   3.254  1.00 17.94           C
ATOM    680  CE1 PHE A 232     -19.391  -8.337   2.414  1.00 21.52           C
ATOM    681  CE2 PHE A 232     -18.350  -9.671   4.102  1.00 20.16           C
ATOM    682  CZ  PHE A 232     -18.870  -8.457   3.678  1.00 22.04           C
ATOM    683  N   LYS A 233     -15.923 -10.400   0.576  1.00 24.28           N
ATOM    684  CA  LYS A 233     -14.642 -10.099   1.224  1.00 22.92           C
ATOM    685  C   LYS A 233     -14.782  -8.799   1.978  1.00 22.84           C
ATOM    686  O   LYS A 233     -15.471  -7.891   1.516  1.00 22.56           O
ATOM    687  CB  LYS A 233     -13.495  -9.991   0.212  1.00 17.41           C
ATOM    688  CG  LYS A 233     -13.227 -11.274  -0.560  1.00 20.00           C
ATOM    689  CD  LYS A 233     -12.285 -11.032  -1.710  1.00 21.13           C
ATOM    690  CE  LYS A 233     -12.419 -12.105  -2.767  1.00 26.26           C
ATOM    691  NZ  LYS A 233     -13.753 -12.082  -3.453  1.00 23.06           N
ATOM    692  N   ASN A 234     -14.132  -8.705   3.134  1.00 17.57           N
ATOM    693  CA  ASN A 234     -14.165  -7.467   3.910  1.00 21.38           C
ATOM    694  C   ASN A 234     -12.791  -7.112   4.486  1.00 19.87           C
ATOM    695  O   ASN A 234     -12.683  -6.685   5.629  1.00 23.24           O
ATOM    696  CB  ASN A 234     -15.212  -7.571   5.033  1.00 18.47           C
ATOM    697  CG  ASN A 234     -15.554  -6.222   5.643  1.00 19.07           C
ATOM    698  OD1 ASN A 234     -15.762  -6.106   6.848  1.00 22.84           O
ATOM    699  ND2 ASN A 234     -15.579  -5.191   4.817  1.00 21.10           N
ATOM    700  N   TRP A 235     -11.742  -7.311   3.697  1.00 19.16           N
ATOM    701  CA  TRP A 235     -10.390  -6.975   4.135  1.00 22.32           C
ATOM    702  C   TRP A 235     -10.264  -5.498   4.467  1.00 20.90           C
ATOM    703  O   TRP A 235     -10.730  -4.642   3.719  1.00 27.44           O
ATOM    704  CB  TRP A 235      -9.354  -7.319   3.063  1.00 20.49           C
ATOM    705  CG  TRP A 235      -9.281  -8.753   2.684  1.00 20.76           C
ATOM    706  CD1 TRP A 235      -9.712  -9.314   1.523  1.00 19.71           C
ATOM    707  CD2 TRP A 235      -8.736  -9.816   3.467  1.00 18.48           C
ATOM    708  NE1 TRP A 235      -9.475 -10.663   1.532  1.00 18.33           N
```

| ATOM | 709 | CE2 | TRP | A | 235 | -8.869 | -10.998 | 2.713 | 1.00 | 17.93 | C |
|------|-----|-----|-----|---|-----|--------|---------|-------|------|-------|---|
| ATOM | 710 | CE3 | TRP | A | 235 | -8.135 | -9.883 | 4.730 | 1.00 | 17.04 | C |
| ATOM | 711 | CZ2 | TRP | A | 235 | -8.427 | -12.236 | 3.180 | 1.00 | 18.19 | C |
| ATOM | 712 | CZ3 | TRP | A | 235 | -7.703 | -11.102 | 5.193 | 1.00 | 14.22 | C |
| ATOM | 713 | CH2 | TRP | A | 235 | -7.855 | -12.269 | 4.423 | 1.00 | 16.87 | C |
| ATOM | 714 | N | ARG | A | 236 | -9.630 | -5.198 | 5.585 | 1.00 | 23.85 | N |
| ATOM | 715 | CA | ARG | A | 236 | -9.169 | -3.842 | 5.826 | 1.00 | 25.84 | C |
| ATOM | 716 | C | ARG | A | 236 | -8.260 | -3.466 | 4.669 | 1.00 | 25.73 | C |
| ATOM | 717 | O | ARG | A | 236 | -7.521 | -4.318 | 4.166 | 1.00 | 27.18 | O |
| ATOM | 718 | CB | ARG | A | 236 | -8.428 | -3.743 | 7.159 | 1.00 | 24.94 | C |
| ATOM | 719 | CG | ARG | A | 236 | -8.108 | -2.312 | 7.586 | 1.00 | 32.25 | C |
| ATOM | 720 | CD | ARG | A | 236 | -7.436 | -2.288 | 8.947 | 1.00 | 30.42 | C |
| ATOM | 721 | NE | ARG | A | 236 | -6.217 | -3.084 | 8.934 | 1.00 | 38.54 | N |
| ATOM | 722 | CZ | ARG | A | 236 | -5.711 | -3.700 | 10.000 | 1.00 | 39.09 | C |
| ATOM | 723 | NH1 | ARG | A | 236 | -6.327 | -3.608 | 11.176 | 1.00 | 39.28 | N |
| ATOM | 724 | NH2 | ARG | A | 236 | -4.588 | -4.409 | 9.889 | 1.00 | 27.72 | N |
| ATOM | 725 | N | PRO | A | 237 | -8.333 | -2.210 | 4.212 | 1.00 | 25.65 | N |
| ATOM | 726 | CA | PRO | A | 237 | -7.412 | -1.747 | 3.167 | 1.00 | 31.88 | C |
| ATOM | 727 | C | PRO | A | 237 | -5.951 | -2.048 | 3.497 | 1.00 | 27.32 | C |
| ATOM | 728 | O | PRO | A | 237 | -5.531 | -1.839 | 4.638 | 1.00 | 28.86 | O |
| ATOM | 729 | CB | PRO | A | 237 | -7.678 | -0.242 | 3.122 | 1.00 | 33.60 | C |
| ATOM | 730 | CG | PRO | A | 237 | -9.149 | -0.156 | 3.436 | 1.00 | 32.49 | C |
| ATOM | 731 | CD | PRO | A | 237 | -9.367 | -1.203 | 4.515 | 1.00 | 29.17 | C |
| ATOM | 732 | N | GLU | A | 238 | -5.231 | -2.563 | 2.501 | 1.00 | 21.38 | N |
| ATOM | 733 | CA | GLU | A | 238 | -3.843 | -3.022 | 2.608 | 1.00 | 23.04 | C |
| ATOM | 734 | C | GLU | A | 238 | -3.741 | -4.424 | 3.231 | 1.00 | 19.36 | C |
| ATOM | 735 | O | GLU | A | 238 | -2.648 | -4.901 | 3.526 | 1.00 | 20.85 | O |
| ATOM | 736 | CB | GLU | A | 238 | -2.978 | -2.022 | 3.397 | 1.00 | 26.89 | C |
| ATOM | 737 | CG | GLU | A | 238 | -2.828 | -0.651 | 2.740 | 1.00 | 31.80 | C |
| ATOM | 738 | CD | GLU | A | 238 | -2.337 | 0.424 | 3.713 | 1.00 | 51.15 | C |
| ATOM | 739 | OE1 | GLU | A | 238 | -2.205 | 0.128 | 4.926 | 1.00 | 50.89 | O |
| ATOM | 740 | OE2 | GLU | A | 238 | -2.101 | 1.573 | 3.269 | 1.00 | 56.05 | O |
| ATOM | 741 | N | GLN | A | 239 | -4.872 | -5.089 | 3.427 | 1.00 | 20.11 | N |
| ATOM | 742 | CA | GLN | A | 239 | -4.840 | -6.495 | 3.825 | 1.00 | 21.60 | C |
| ATOM | 743 | C | GLN | A | 239 | -5.501 | -7.317 | 2.736 | 1.00 | 19.53 | C |
| ATOM | 744 | O | GLN | A | 239 | -6.332 | -6.792 | 2.011 | 1.00 | 21.20 | O |
| ATOM | 745 | CB | GLN | A | 239 | -5.543 | -6.726 | 5.166 | 1.00 | 17.31 | C |
| ATOM | 746 | CG | GLN | A | 239 | -5.020 | -5.893 | 6.310 | 1.00 | 21.03 | C |
| ATOM | 747 | CD | GLN | A | 239 | -3.565 | -6.156 | 6.618 | 1.00 | 18.65 | C |
| ATOM | 748 | OE1 | GLN | A | 239 | -3.045 | -7.232 | 6.343 | 1.00 | 17.67 | O |
| ATOM | 749 | NE2 | GLN | A | 239 | -2.897 | -5.165 | 7.184 | 1.00 | 18.18 | N |
| ATOM | 750 | N | PRO | A | 240 | -5.132 | -8.601 | 2.601 | 1.00 | 16.46 | N |
| ATOM | 751 | CA | PRO | A | 240 | -4.108 | -9.345 | 3.337 | 1.00 | 20.08 | C |
| ATOM | 752 | C | PRO | A | 240 | -2.696 | -8.938 | 2.901 | 1.00 | 19.97 | C |
| ATOM | 753 | O | PRO | A | 240 | -2.486 | -8.679 | 1.708 | 1.00 | 19.33 | O |
| ATOM | 754 | CB | PRO | A | 240 | -4.412 | -10.806 | 2.966 | 1.00 | 15.73 | C |
| ATOM | 755 | CG | PRO | A | 240 | -4.999 | -10.714 | 1.622 | 1.00 | 20.40 | C |
| ATOM | 756 | CD | PRO | A | 240 | -5.783 | -9.438 | 1.580 | 1.00 | 18.17 | C |
| ATOM | 757 | N | ASP | A | 241 | -1.744 | -8.882 | 3.830 | 1.00 | 16.04 | N |
| ATOM | 758 | CA | ASP | A | 241 | -0.399 | -8.446 | 3.472 | 1.00 | 12.88 | C |
| ATOM | 759 | C | ASP | A | 241 | 0.663 | -9.491 | 3.732 | 1.00 | 12.48 | C |
| ATOM | 760 | O | ASP | A | 241 | 1.842 | -9.234 | 3.490 | 1.00 | 15.24 | O |
| ATOM | 761 | CB | ASP | A | 241 | -0.035 | -7.164 | 4.212 | 1.00 | 15.52 | C |
| ATOM | 762 | CG | ASP | A | 241 | -0.053 | -7.332 | 5.733 | 1.00 | 19.38 | C |
| ATOM | 763 | OD1 | ASP | A | 241 | -0.451 | -8.416 | 6.231 | 1.00 | 19.33 | O |
| ATOM | 764 | OD2 | ASP | A | 241 | 0.293 | -6.358 | 6.430 | 1.00 | 17.43 | O |
| ATOM | 765 | N | ASP | A | 242 | 0.249 | -10.652 | 4.237 | 1.00 | 17.41 | N |
| ATOM | 766 | CA | ASP | A | 242 | 1.166 | -11.774 | 4.527 | 1.00 | 16.54 | C |
| ATOM | 767 | C | ASP | A | 242 | 2.465 | -11.313 | 5.190 | 1.00 | 16.20 | C |
| ATOM | 768 | O | ASP | A | 242 | 3.572 | -11.552 | 4.700 | 1.00 | 18.85 | O |
| ATOM | 769 | CB | ASP | A | 242 | 1.487 | -12.530 | 3.261 | 1.00 | 16.74 | C |
| ATOM | 770 | CG | ASP | A | 242 | 2.053 | -13.888 | 3.538 | 1.00 | 21.40 | C |
| ATOM | 771 | OD1 | ASP | A | 242 | 1.481 | -14.576 | 4.413 | 1.00 | 17.68 | O |
| ATOM | 772 | OD2 | ASP | A | 242 | 3.066 | -14.253 | 2.887 | 1.00 | 16.85 | O |
| ATOM | 773 | N | TRP | A | 243 | 2.301 | -10.617 | 6.299 | 1.00 | 15.83 | N |
| ATOM | 774 | CA | TRP | A | 243 | 3.382 | -9.929 | 6.960 | 1.00 | 16.80 | C |
| ATOM | 775 | C | TRP | A | 243 | 4.390 | -10.913 | 7.545 | 1.00 | 16.03 | C |
| ATOM | 776 | O | TRP | A | 243 | 4.020 | -11.905 | 8.153 | 1.00 | 15.06 | O |
| ATOM | 777 | CB | TRP | A | 243 | 2.799 | -9.041 | 8.043 | 1.00 | 19.08 | C |
| ATOM | 778 | CG | TRP | A | 243 | 3.727 | -8.029 | 8.570 | 1.00 | 24.75 | C |
| ATOM | 779 | CD1 | TRP | A | 243 | 4.591 | -7.248 | 7.857 | 1.00 | 21.85 | C |

```
ATOM    780  CD2 TRP A 243      3.888  -7.665   9.942  1.00 19.00           C
ATOM    781  NE1 TRP A 243      5.274  -6.417   8.707  1.00 20.11           N
ATOM    782  CE2 TRP A 243      4.858  -6.653   9.993  1.00 17.79           C
ATOM    783  CE3 TRP A 243      3.296  -8.092  11.133  1.00 19.98           C
ATOM    784  CZ2 TRP A 243      5.259  -6.067  11.189  1.00 19.26           C
ATOM    785  CZ3 TRP A 243      3.697  -7.508  12.321  1.00 19.32           C
ATOM    786  CH2 TRP A 243      4.666  -6.508  12.338  1.00 14.64           C
ATOM    787  N   TYR A 244      5.671 -10.643   7.350  1.00 19.42           N
ATOM    788  CA  TYR A 244      6.694 -11.486   7.945  1.00 20.57           C
ATOM    789  C   TYR A 244      7.295 -10.760   9.140  1.00 19.06           C
ATOM    790  O   TYR A 244      8.022 -11.353   9.930  1.00 17.76           O
ATOM    791  CB  TYR A 244      7.784 -11.833   6.932  1.00 18.60           C
ATOM    792  CG  TYR A 244      7.501 -13.035   6.056  1.00 24.25           C
ATOM    793  CD1 TYR A 244      6.568 -12.970   5.020  1.00 17.33           C
ATOM    794  CD2 TYR A 244      8.189 -14.228   6.244  1.00 24.36           C
ATOM    795  CE1 TYR A 244      6.321 -14.070   4.203  1.00 18.22           C
ATOM    796  CE2 TYR A 244      7.945 -15.342   5.436  1.00 21.75           C
ATOM    797  CZ  TYR A 244      7.016 -15.257   4.415  1.00 19.74           C
ATOM    798  OH  TYR A 244      6.776 -16.367   3.620  1.00 14.89           O
ATOM    799  N   GLY A 245      6.958  -9.478   9.265  1.00 19.02           N
ATOM    800  CA  GLY A 245      7.658  -8.568  10.152  1.00 20.36           C
ATOM    801  C   GLY A 245      7.367  -8.768  11.621  1.00 19.87           C
ATOM    802  O   GLY A 245      7.941  -8.086  12.475  1.00 21.29           O
ATOM    803  N   HIS A 246      6.463  -9.696  11.910  1.00 19.33           N
ATOM    804  CA  HIS A 246      6.150 -10.062  13.277  1.00 17.48           C
ATOM    805  C   HIS A 246      7.284 -10.899  13.844  1.00 18.02           C
ATOM    806  O   HIS A 246      7.418 -11.015  15.053  1.00 22.65           O
ATOM    807  CB  HIS A 246      4.825 -10.823  13.340  1.00 15.31           C
ATOM    808  CG  HIS A 246      4.719 -11.907  12.316  1.00 19.52           C
ATOM    809  ND1 HIS A 246      5.326 -13.134  12.467  1.00 16.40           N
ATOM    810  CD2 HIS A 246      4.101 -11.937  11.112  1.00 16.23           C
ATOM    811  CE1 HIS A 246      5.085 -13.875  11.400  1.00 17.63           C
ATOM    812  NE2 HIS A 246      4.343 -13.172  10.564  1.00 16.33           N
ATOM    813  N   GLY A 247      8.098 -11.482  12.968  1.00 18.78           N
ATOM    814  CA  GLY A 247      9.295 -12.193  13.394  1.00 17.32           C
ATOM    815  C   GLY A 247      9.030 -13.544  14.022  1.00 19.13           C
ATOM    816  O   GLY A 247      9.904 -14.116  14.677  1.00 21.71           O
ATOM    817  N   LEU A 248      7.828 -14.074  13.811  1.00 20.58           N
ATOM    818  CA  LEU A 248      7.427 -15.330  14.447  1.00 17.12           C
ATOM    819  C   LEU A 248      7.676 -16.527  13.537  1.00 14.44           C
ATOM    820  O   LEU A 248      7.656 -17.670  13.977  1.00 17.33           O
ATOM    821  CB  LEU A 248      5.951 -15.270  14.852  1.00 19.74           C
ATOM    822  CG  LEU A 248      5.635 -14.119  15.804  1.00 17.23           C
ATOM    823  CD1 LEU A 248      4.193 -14.202  16.254  1.00 17.05           C
ATOM    824  CD2 LEU A 248      6.599 -14.120  17.007  1.00 16.08           C
ATOM    825  N   GLY A 249      7.920 -16.261  12.263  1.00 17.53           N
ATOM    826  CA  GLY A 249      8.159 -17.332  11.315  1.00 16.74           C
ATOM    827  C   GLY A 249      7.032 -17.381  10.316  1.00 15.59           C
ATOM    828  O   GLY A 249      5.874 -17.344  10.708  1.00 17.37           O
ATOM    829  N   GLY A 250      7.370 -17.451   9.029  1.00 14.92           N
ATOM    830  CA  GLY A 250      6.370 -17.435   7.982  1.00 19.03           C
ATOM    831  C   GLY A 250      5.672 -16.090   7.891  1.00 20.35           C
ATOM    832  O   GLY A 250      6.054 -15.119   8.549  1.00 18.82           O
ATOM    833  N   GLY A 251      4.646 -16.022   7.057  1.00 21.60           N
ATOM    834  CA  GLY A 251      3.910 -14.787   6.880  1.00 17.04           C
ATOM    835  C   GLY A 251      2.691 -14.851   7.752  1.00 16.59           C
ATOM    836  O   GLY A 251      2.778 -15.115   8.952  1.00 22.55           O
ATOM    837  N   GLU A 252      1.539 -14.636   7.137  1.00 16.53           N
ATOM    838  CA  GLU A 252      0.272 -14.664   7.846  1.00 15.39           C
ATOM    839  C   GLU A 252     -0.760 -15.400   7.016  1.00 17.17           C
ATOM    840  O   GLU A 252     -1.021 -15.027   5.864  1.00 14.07           O
ATOM    841  CB  GLU A 252     -0.201 -13.253   8.143  1.00 15.30           C
ATOM    842  CG  GLU A 252      0.748 -12.448   8.970  1.00 16.44           C
ATOM    843  CD  GLU A 252      0.242 -11.045   9.189  1.00 18.48           C
ATOM    844  OE1 GLU A 252      0.261 -10.579  10.340  1.00 16.20           O
ATOM    845  OE2 GLU A 252     -0.159 -10.400   8.200  1.00 15.99           O
ATOM    846  N   ASP A 253     -1.355 -16.437   7.590  1.00 15.10           N
ATOM    847  CA  ASP A 253     -2.163 -17.337   6.787  1.00 16.04           C
ATOM    848  C   ASP A 253     -3.600 -17.499   7.243  1.00 15.46           C
ATOM    849  O   ASP A 253     -4.310 -18.320   6.713  1.00 17.45           O
ATOM    850  CB  ASP A 253     -1.519 -18.719   6.745  1.00 15.26           C
```

```
ATOM    851  CG  ASP A 253      -0.090 -18.683    6.258  1.00 18.84          C
ATOM    852  OD1 ASP A 253       0.337 -17.664    5.670  1.00 19.76          O
ATOM    853  OD2 ASP A 253       0.605 -19.703    6.423  1.00 24.71          O
ATOM    854  N   CYS A 254      -4.029 -16.745    8.238  1.00 15.58          N
ATOM    855  CA  CYS A 254      -5.321 -17.012    8.818  1.00 13.38          C
ATOM    856  C   CYS A 254      -6.083 -15.715    9.012  1.00 17.24          C
ATOM    857  O   CYS A 254      -5.577 -14.778    9.643  1.00 17.90          O
ATOM    858  CB  CYS A 254      -5.161 -17.762   10.147  1.00 17.64          C
ATOM    859  SG  CYS A 254      -4.659 -19.487    9.965  1.00 15.82          S
ATOM    860  N   ALA A 255      -7.298 -15.672    8.474  1.00 10.34          N
ATOM    861  CA  ALA A 255      -8.134 -14.474    8.555  1.00 15.36          C
ATOM    862  C   ALA A 255      -8.863 -14.334    9.886  1.00 16.38          C
ATOM    863  O   ALA A 255      -9.420 -15.293   10.424  1.00 15.65          O
ATOM    864  CB  ALA A 255      -9.153 -14.460    7.430  1.00 13.09          C
ATOM    865  N   HIS A 256      -8.886 -13.116   10.401  1.00 15.41          N
ATOM    866  CA  HIS A 256      -9.712 -12.834   11.552  1.00 16.09          C
ATOM    867  C   HIS A 256     -10.427 -11.512   11.367  1.00 14.49          C
ATOM    868  O   HIS A 256      -9.986 -10.664   10.599  1.00 17.03          O
ATOM    869  CB  HIS A 256      -8.874 -12.816   12.841  1.00 11.61          C
ATOM    870  CG  HIS A 256      -7.844 -11.733   12.885  1.00 15.00          C
ATOM    871  ND1 HIS A 256      -7.979 -10.609   13.673  1.00 13.69          N
ATOM    872  CD2 HIS A 256      -6.657 -11.605   12.245  1.00 13.08          C
ATOM    873  CE1 HIS A 256      -6.915  -9.840   13.522  1.00 15.34          C
ATOM    874  NE2 HIS A 256      -6.098 -10.423   12.661  1.00 15.12          N
ATOM    875  N   PHE A 257     -11.543 -11.357   12.065  1.00 16.75          N
ATOM    876  CA  PHE A 257     -12.147 -10.057   12.229  1.00 14.34          C
ATOM    877  C   PHE A 257     -11.246  -9.225   13.119  1.00 16.09          C
ATOM    878  O   PHE A 257     -10.752  -9.703   14.142  1.00 16.99          O
ATOM    879  CB  PHE A 257     -13.541 -10.159   12.844  1.00 13.50          C
ATOM    880  CG  PHE A 257     -14.430 -11.166   12.176  1.00 21.06          C
ATOM    881  CD2 PHE A 257     -14.462 -12.477   12.618  1.00 16.97          C
ATOM    882  CD1 PHE A 257     -15.233 -10.803   11.098  1.00 18.50          C
ATOM    883  CE2 PHE A 257     -15.277 -13.410   12.003  1.00 17.90          C
ATOM    884  CE1 PHE A 257     -16.049 -11.729   10.487  1.00 16.37          C
ATOM    885  CZ  PHE A 257     -16.066 -13.029   10.928  1.00 14.28          C
ATOM    886  N   THR A 258     -11.039  -7.979   12.734  1.00 15.18          N
ATOM    887  CA  THR A 258     -10.337  -7.053   13.597  1.00 19.93          C
ATOM    888  C   THR A 258     -11.351  -6.356   14.465  1.00 22.34          C
ATOM    889  O   THR A 258     -12.557  -6.616   14.356  1.00 28.29          O
ATOM    890  CB  THR A 258      -9.553  -6.014   12.814  1.00 18.33          C
ATOM    891  OG1 THR A 258     -10.468  -5.213   12.058  1.00 17.14          O
ATOM    892  CG2 THR A 258      -8.569  -6.692   11.876  1.00 19.40          C
ATOM    893  N   ASP A 259     -10.869  -5.459   15.316  1.00 20.10          N
ATOM    894  CA  ASP A 259     -11.735  -4.749   16.250  1.00 23.59          C
ATOM    895  C   ASP A 259     -12.909  -4.037   15.572  1.00 23.64          C
ATOM    896  O   ASP A 259     -13.984  -3.947   16.148  1.00 24.14          O
ATOM    897  CB  ASP A 259     -10.911  -3.747   17.079  1.00 22.86          C
ATOM    898  CG  ASP A 259     -10.205  -2.685   16.223  1.00 30.45          C
ATOM    899  OD1 ASP A 259     -10.086  -2.851   14.987  1.00 32.11          O
ATOM    900  OD2 ASP A 259      -9.736  -1.679   16.808  1.00 36.17          O
ATOM    901  N   ASP A 260     -12.710  -3.542   14.351  1.00 27.68          N
ATOM    902  CA  ASP A 260     -13.762  -2.778   13.673  1.00 24.53          C
ATOM    903  C   ASP A 260     -14.544  -3.664   12.716  1.00 23.69          C
ATOM    904  O   ASP A 260     -15.474  -3.208   12.055  1.00 27.59          O
ATOM    905  CB  ASP A 260     -13.177  -1.551   12.934  1.00 23.65          C
ATOM    906  CG  ASP A 260     -12.279  -1.920   11.738  1.00 31.18          C
ATOM    907  OD1 ASP A 260     -12.578  -2.896   11.008  1.00 25.05          O
ATOM    908  OD2 ASP A 260     -11.267  -1.206   11.514  1.00 30.59          O
ATOM    909  N   GLY A 261     -14.138  -4.927   12.617  1.00 24.03          N
ATOM    910  CA  GLY A 261     -14.898  -5.908   11.863  1.00 18.76          C
ATOM    911  C   GLY A 261     -14.236  -6.262   10.550  1.00 17.99          C
ATOM    912  O   GLY A 261     -14.459  -7.331   10.004  1.00 13.89          O
ATOM    913  N   ARG A 262     -13.392  -5.373   10.048  1.00 19.30          N
ATOM    914  CA  ARG A 262     -12.709  -5.649    8.791  1.00 19.10          C
ATOM    915  C   ARG A 262     -11.659  -6.749    8.989  1.00 19.94          C
ATOM    916  O   ARG A 262     -11.216  -7.016   10.120  1.00 12.26          O
ATOM    917  CB  ARG A 262     -12.115  -4.356    8.232  1.00 20.66          C
ATOM    918  CG  ARG A 262     -13.219  -3.351    7.897  1.00 25.30          C
ATOM    919  CD  ARG A 262     -12.667  -2.017    7.453  1.00 28.34          C
ATOM    920  NE  ARG A 262     -11.765  -1.459    8.449  1.00 25.73          N
ATOM    921  CZ  ARG A 262     -11.035  -0.367    8.254  1.00 29.19          C
```

| ATOM | 922 | NH1 | ARG A 262 | -11.114 | 0.278 | 7.100 | 1.00 | 24.96 | N |
|------|-----|-----|-----------|---------|-------|-------|------|-------|---|
| ATOM | 923 | NH2 | ARG A 262 | -10.229 | 0.079 | 9.208 | 1.00 | 29.61 | N |
| ATOM | 924 | N   | TRP A 263 | -11.307 | -7.432 | 7.903 | 1.00 | 14.26 | N |
| ATOM | 925 | CA  | TRP A 263 | -10.517 | -8.648 | 8.041 | 1.00 | 16.50 | C |
| ATOM | 926 | C   | TRP A 263 | -9.043 | -8.337 | 8.000 | 1.00 | 15.53 | C |
| ATOM | 927 | O   | TRP A 263 | -8.627 | -7.337 | 7.443 | 1.00 | 16.65 | O |
| ATOM | 928 | CB  | TRP A 263 | -10.851 | -9.661 | 6.946 | 1.00 | 13.91 | C |
| ATOM | 929 | CG  | TRP A 263 | -12.290 | -10.013 | 6.862 | 1.00 | 16.25 | C |
| ATOM | 930 | CD1 | TRP A 263 | -13.273 | -9.643 | 7.721 | 1.00 | 13.40 | C |
| ATOM | 931 | CD2 | TRP A 263 | -12.918 | -10.804 | 5.846 | 1.00 | 17.03 | C |
| ATOM | 932 | NE1 | TRP A 263 | -14.473 | -10.156 | 7.314 | 1.00 | 15.19 | N |
| ATOM | 933 | CE2 | TRP A 263 | -14.284 | -10.870 | 6.160 | 1.00 | 14.98 | C |
| ATOM | 934 | CE3 | TRP A 263 | -12.456 | -11.455 | 4.697 | 1.00 | 13.09 | C |
| ATOM | 935 | CZ2 | TRP A 263 | -15.196 | -11.577 | 5.378 | 1.00 | 19.78 | C |
| ATOM | 936 | CZ3 | TRP A 263 | -13.360 | -12.154 | 3.922 | 1.00 | 16.68 | C |
| ATOM | 937 | CH2 | TRP A 263 | -14.716 | -12.206 | 4.261 | 1.00 | 16.08 | C |
| ATOM | 938 | N   | ASN A 264 | -8.262 | -9.226 | 8.588 | 1.00 | 15.51 | N |
| ATOM | 939 | CA  | ASN A 264 | -6.816 | -9.162 | 8.521 | 1.00 | 14.14 | C |
| ATOM | 940 | C   | ASN A 264 | -6.313 | -10.573 | 8.398 | 1.00 | 14.75 | C |
| ATOM | 941 | O   | ASN A 264 | -6.994 | -11.503 | 8.817 | 1.00 | 18.31 | O |
| ATOM | 942 | CB  | ASN A 264 | -6.239 | -8.490 | 9.768 | 1.00 | 17.34 | C |
| ATOM | 943 | CG  | ASN A 264 | -4.721 | -8.499 | 9.794 | 1.00 | 18.04 | C |
| ATOM | 944 | OD1 | ASN A 264 | -4.070 | -8.128 | 8.820 | 1.00 | 13.84 | O |
| ATOM | 945 | ND2 | ASN A 264 | -4.151 | -8.917 | 10.923 | 1.00 | 16.66 | N |
| ATOM | 946 | N   | ASP A 265 | -5.146 | -10.757 | 7.795 | 1.00 | 18.48 | N |
| ATOM | 947 | CA  | ASP A 265 | -4.527 | -12.072 | 7.800 | 1.00 | 18.19 | C |
| ATOM | 948 | C   | ASP A 265 | -3.499 | -11.991 | 8.902 | 1.00 | 17.54 | C |
| ATOM | 949 | O   | ASP A 265 | -2.811 | -10.980 | 9.030 | 1.00 | 18.38 | O |
| ATOM | 950 | CB  | ASP A 265 | -3.912 | -12.455 | 6.438 | 1.00 | 15.50 | C |
| ATOM | 951 | CG  | ASP A 265 | -2.875 | -11.458 | 5.943 | 1.00 | 16.55 | C |
| ATOM | 952 | OD1 | ASP A 265 | -2.920 | -10.277 | 6.358 | 1.00 | 15.74 | O |
| ATOM | 953 | OD2 | ASP A 265 | -2.022 | -11.858 | 5.106 | 1.00 | 16.21 | O |
| ATOM | 954 | N   | ASP A 266 | -3.449 | -13.013 | 9.745 | 1.00 | 15.99 | N |
| ATOM | 955 | CA  | ASP A 266 | -2.527 | -12.977 | 10.867 | 1.00 | 16.72 | C |
| ATOM | 956 | C   | ASP A 266 | -1.843 | -14.321 | 11.085 | 1.00 | 19.73 | C |
| ATOM | 957 | O   | ASP A 266 | -2.100 | -15.292 | 10.371 | 1.00 | 18.63 | O |
| ATOM | 958 | CB  | ASP A 266 | -3.244 | -12.542 | 12.139 | 1.00 | 15.67 | C |
| ATOM | 959 | CG  | ASP A 266 | -2.356 | -11.743 | 13.041 | 1.00 | 20.35 | C |
| ATOM | 960 | OD2 | ASP A 266 | -2.877 | -10.827 | 13.717 | 1.00 | 20.40 | O |
| ATOM | 961 | OD1 | ASP A 266 | -1.130 | -12.024 | 13.055 | 1.00 | 18.05 | O |
| ATOM | 962 | N   | VAL A 267 | -0.951 | -14.365 | 12.069 | 1.00 | 21.31 | N |
| ATOM | 963 | CA  | VAL A 267 | -0.236 | -15.588 | 12.357 | 1.00 | 20.03 | C |
| ATOM | 964 | C   | VAL A 267 | -1.206 | -16.604 | 12.957 | 1.00 | 17.18 | C |
| ATOM | 965 | O   | VAL A 267 | -1.904 | -16.301 | 13.923 | 1.00 | 19.78 | O |
| ATOM | 966 | CB  | VAL A 267 | 0.935 | -15.327 | 13.302 | 1.00 | 18.16 | C |
| ATOM | 967 | CG1 | VAL A 267 | 1.670 | -16.609 | 13.576 | 1.00 | 19.68 | C |
| ATOM | 968 | CG2 | VAL A 267 | 1.862 | -14.301 | 12.685 | 1.00 | 15.95 | C |
| ATOM | 969 | N   | CYS A 268 | -1.246 | -17.803 | 12.383 | 1.00 | 12.37 | N |
| ATOM | 970 | CA  | CYS A 268 | -2.213 | -18.818 | 12.785 | 1.00 | 14.68 | C |
| ATOM | 971 | C   | CYS A 268 | -2.031 | -19.284 | 14.238 | 1.00 | 14.79 | C |
| ATOM | 972 | O   | CYS A 268 | -2.960 | -19.800 | 14.855 | 1.00 | 17.64 | O |
| ATOM | 973 | CB  | CYS A 268 | -2.140 | -20.010 | 11.832 | 1.00 | 15.66 | C |
| ATOM | 974 | SG  | CYS A 268 | -2.628 | -19.567 | 10.101 | 1.00 | 27.47 | S |
| ATOM | 975 | N   | GLN A 269 | -0.851 | -19.069 | 14.786 | 1.00 | 13.72 | N |
| ATOM | 976 | CA  | GLN A 269 | -0.559 | -19.493 | 16.149 | 1.00 | 16.48 | C |
| ATOM | 977 | C   | GLN A 269 | -1.136 | -18.565 | 17.190 | 1.00 | 15.79 | C |
| ATOM | 978 | O   | GLN A 269 | -1.204 | -18.920 | 18.376 | 1.00 | 17.70 | O |
| ATOM | 979 | CB  | GLN A 269 | 0.934 | -19.562 | 16.397 | 1.00 | 17.79 | C |
| ATOM | 980 | CG  | GLN A 269 | 1.752 | -20.417 | 15.507 | 1.00 | 20.66 | C |
| ATOM | 981 | CD  | GLN A 269 | 3.240 | -20.205 | 15.825 | 1.00 | 31.48 | C |
| ATOM | 982 | OE1 | GLN A 269 | 3.870 | -19.269 | 15.315 | 1.00 | 28.23 | O |
| ATOM | 983 | NE2 | GLN A 269 | 3.786 | -21.039 | 16.708 | 1.00 | 24.46 | N |
| ATOM | 984 | N   | ARG A 270 | -1.501 | -17.358 | 16.781 | 1.00 | 13.72 | N |
| ATOM | 985 | CA  | ARG A 270 | -2.043 | -16.420 | 17.759 | 1.00 | 14.78 | C |
| ATOM | 986 | C   | ARG A 270 | -3.318 | -16.974 | 18.307 | 1.00 | 13.79 | C |
| ATOM | 987 | O   | ARG A 270 | -4.116 | -17.542 | 17.558 | 1.00 | 16.01 | O |
| ATOM | 988 | CB  | ARG A 270 | -2.301 | -15.045 | 17.162 | 1.00 | 13.63 | C |
| ATOM | 989 | CG  | ARG A 270 | -1.059 | -14.369 | 16.672 | 1.00 | 17.92 | C |
| ATOM | 990 | CD  | ARG A 270 | -1.316 | -12.904 | 16.442 | 1.00 | 15.26 | C |
| ATOM | 991 | NE  | ARG A 270 | -0.240 | -12.295 | 15.678 | 1.00 | 18.21 | N |
| ATOM | 992 | CZ  | ARG A 270 | 0.907 | -11.880 | 16.206 | 1.00 | 17.13 | C |

```
ATOM    993  NH1 ARG A 270      1.137 -12.008  17.513  1.00 15.71           N
ATOM    994  NH2 ARG A 270      1.814 -11.320  15.426  1.00 12.96           N
ATOM    995  N   PRO A 271     -3.503 -16.833  19.623  1.00 14.36           N
ATOM    996  CA  PRO A 271     -4.769 -17.166  20.253  1.00 12.34           C
ATOM    997  C   PRO A 271     -5.760 -15.999  20.077  1.00 13.26           C
ATOM    998  O   PRO A 271     -5.535 -14.874  20.507  1.00 14.69           O
ATOM    999  CB  PRO A 271     -4.381 -17.391  21.727  1.00 15.38           C
ATOM   1000  CG  PRO A 271     -3.151 -16.556  21.928  1.00 15.88           C
ATOM   1001  CD  PRO A 271     -2.502 -16.336  20.593  1.00 15.31           C
ATOM   1002  N   TYR A 272     -6.855 -16.285  19.400  1.00 13.29           N
ATOM   1003  CA  TYR A 272     -7.918 -15.315  19.193  1.00 16.14           C
ATOM   1004  C   TYR A 272     -9.201 -16.027  19.549  1.00 13.34           C
ATOM   1005  O   TYR A 272     -9.224 -17.253  19.601  1.00 12.02           O
ATOM   1006  CB  TYR A 272     -7.982 -14.830  17.736  1.00 11.47           C
ATOM   1007  CG  TYR A 272     -6.967 -13.776  17.310  1.00 13.69           C
ATOM   1008  CD1 TYR A 272     -6.439 -12.872  18.213  1.00  9.93           C
ATOM   1009  CD2 TYR A 272     -6.574 -13.673  15.969  1.00 12.95           C
ATOM   1010  CE1 TYR A 272     -5.538 -11.914  17.807  1.00 11.19           C
ATOM   1011  CE2 TYR A 272     -5.684 -12.723  15.561  1.00  9.94           C
ATOM   1012  CZ  TYR A 272     -5.164 -11.845  16.484  1.00 14.00           C
ATOM   1013  OH  TYR A 272     -4.257 -10.893  16.080  1.00 17.41           O
ATOM   1014  N   ARG A 273    -10.271 -15.272  19.765  1.00 12.95           N
ATOM   1015  CA  ARG A 273    -11.572 -15.885  19.870  1.00 12.35           C
ATOM   1016  C   ARG A 273    -11.927 -16.389  18.493  1.00 14.06           C
ATOM   1017  O   ARG A 273    -11.229 -16.088  17.517  1.00 14.38           O
ATOM   1018  CB  ARG A 273    -12.611 -14.905  20.397  1.00 14.33           C
ATOM   1019  CG  ARG A 273    -12.364 -14.520  21.841  1.00 12.78           C
ATOM   1020  CD  ARG A 273    -13.449 -13.645  22.401  1.00 17.64           C
ATOM   1021  NE  ARG A 273    -13.276 -13.515  23.840  1.00 21.61           N
ATOM   1022  CZ  ARG A 273    -14.015 -12.742  24.625  1.00 24.75           C
ATOM   1023  NH1 ARG A 273    -14.997 -12.010  24.109  1.00 30.00           N
ATOM   1024  NH2 ARG A 273    -13.763 -12.702  25.930  1.00 20.72           N
ATOM   1025  N   TRP A 274    -12.986 -17.183  18.411  1.00 13.15           N
ATOM   1026  CA  TRP A 274    -13.420 -17.706  17.130  1.00 13.83           C
ATOM   1027  C   TRP A 274    -14.926 -17.845  17.166  1.00 15.15           C
ATOM   1028  O   TRP A 274    -15.525 -17.760  18.227  1.00 14.10           O
ATOM   1029  CB  TRP A 274    -12.758 -19.043  16.833  1.00 12.63           C
ATOM   1030  CG  TRP A 274    -13.207 -20.135  17.726  1.00 16.63           C
ATOM   1031  CD1 TRP A 274    -14.106 -21.107  17.428  1.00 18.08           C
ATOM   1032  CD2 TRP A 274    -12.780 -20.377  19.080  1.00 14.16           C
ATOM   1033  NE1 TRP A 274    -14.265 -21.951  18.507  1.00 15.30           N
ATOM   1034  CE2 TRP A 274    -13.463 -21.521  19.533  1.00 15.80           C
ATOM   1035  CE3 TRP A 274    -11.874 -19.744  19.944  1.00 16.39           C
ATOM   1036  CZ2 TRP A 274    -13.282 -22.046  20.822  1.00 16.41           C
ATOM   1037  CZ3 TRP A 274    -11.700 -20.261  21.226  1.00 17.93           C
ATOM   1038  CH2 TRP A 274    -12.407 -21.401  21.651  1.00 14.81           C
ATOM   1039  N   VAL A 275    -15.532 -18.025  16.003  1.00 13.91           N
ATOM   1040  CA  VAL A 275    -16.954 -18.282  15.911  1.00 14.90           C
ATOM   1041  C   VAL A 275    -17.157 -19.604  15.214  1.00 17.68           C
ATOM   1042  O   VAL A 275    -16.570 -19.843  14.160  1.00 16.82           O
ATOM   1043  CB  VAL A 275    -17.690 -17.199  15.126  1.00 15.04           C
ATOM   1044  CG1 VAL A 275    -19.194 -17.473  15.122  1.00 14.67           C
ATOM   1045  CG2 VAL A 275    -17.359 -15.825  15.676  1.00 12.68           C
ATOM   1046  N   CYS A 276    -17.993 -20.463  15.791  1.00 18.92           N
ATOM   1047  CA  CYS A 276    -18.396 -21.676  15.095  1.00 20.03           C
ATOM   1048  C   CYS A 276    -19.725 -21.444  14.397  1.00 19.45           C
ATOM   1049  O   CYS A 276    -20.581 -20.714  14.896  1.00 17.29           O
ATOM   1050  CB  CYS A 276    -18.493 -22.859  16.055  1.00 22.59           C
ATOM   1051  SG  CYS A 276    -16.895 -23.523  16.446  1.00 31.78           S
ATOM   1052  N   GLU A 277    -19.865 -22.050  13.225  1.00 18.94           N
ATOM   1053  CA  GLU A 277    -21.077 -21.941  12.437  1.00 21.40           C
ATOM   1054  C   GLU A 277    -21.492 -23.299  11.919  1.00 19.98           C
ATOM   1055  O   GLU A 277    -20.668 -24.086  11.467  1.00 20.59           O
ATOM   1056  CB  GLU A 277    -20.887 -20.988  11.263  1.00 17.46           C
ATOM   1057  CG  GLU A 277    -22.187 -20.673  10.536  1.00 21.39           C
ATOM   1058  CD  GLU A 277    -21.972 -20.063   9.154  1.00 22.43           C
ATOM   1059  OE1 GLU A 277    -20.817 -20.032   8.682  1.00 21.58           O
ATOM   1060  OE2 GLU A 277    -22.962 -19.607   8.540  1.00 22.39           O
ATOM   1061  N   THR A 278    -22.778 -23.572  11.998  1.00 19.21           N
ATOM   1062  CA  THR A 278    -23.351 -24.710  11.309  1.00 24.97           C
ATOM   1063  C   THR A 278    -24.612 -24.250  10.581  1.00 25.29           C
```

```
ATOM    1064  O    THR A 278     -25.166 -23.196  10.887  1.00 24.46           O
ATOM    1065  CB   THR A 278     -23.688 -25.846  12.267  1.00 25.96           C
ATOM    1066  OG1  THR A 278     -23.799 -27.063  11.527  1.00 36.51           O
ATOM    1067  CG2  THR A 278     -24.996 -25.566  12.973  1.00 23.69           C
ATOM    1068  N    GLU A 279     -25.065 -25.033   9.613  1.00 29.02           N
ATOM    1069  CA   GLU A 279     -26.228 -24.630   8.841  1.00 31.70           C
ATOM    1070  C    GLU A 279     -27.438 -25.498   9.167  1.00 29.56           C
ATOM    1071  O    GLU A 279     -27.299 -26.687   9.406  1.00 29.11           O
ATOM    1072  CB   GLU A 279     -25.911 -24.678   7.347  1.00 28.64           C
ATOM    1073  CG   GLU A 279     -24.969 -23.568   6.885  1.00 27.57           C
ATOM    1074  CD   GLU A 279     -23.497 -23.939   7.011  1.00 31.65           C
ATOM    1075  OE1  GLU A 279     -22.662 -23.026   7.182  1.00 30.86           O
ATOM    1076  OE2  GLU A 279     -23.166 -25.139   6.923  1.00 32.59           O
ATOM    1077  N    LEU A 280     -28.617 -24.885   9.219  1.00 34.32           N
ATOM    1078  CA   LEU A 280     -29.855 -25.649   9.304  1.00 35.91           C
ATOM    1079  C    LEU A 280     -30.265 -26.069   7.893  1.00 40.23           C
ATOM    1080  O    LEU A 280     -30.735 -27.187   7.666  1.00 43.65           O
ATOM    1081  CB   LEU A 280     -30.974 -24.837   9.959  1.00 37.40           C
ATOM    1082  CG   LEU A 280     -30.811 -24.359  11.398  1.00 33.17           C
ATOM    1083  CD1  LEU A 280     -32.064 -23.616  11.839  1.00 39.13           C
ATOM    1084  CD2  LEU A 280     -30.516 -25.518  12.322  1.00 36.44           C
ATOM    1085  OXT  LEU A 280     -30.126 -25.285   6.942  1.00 35.92           O
HETATM  1086  CA   CA  A1001      -0.390 -15.928   3.667  1.00 18.60          Ca
HETATM  1087  CA   CA  A1002      -1.936  -8.931   8.005  1.00 15.61          Ca
HETATM  1088  CA   CA  A1003     -22.072 -18.548   6.675  1.00 20.87          Ca
HETATM  1089  CL   CL  A1004      -0.586 -12.976  20.217  1.00 15.82          Cl
HETATM  1090  CL   CL  A1005     -16.511 -11.093  27.288  1.00 51.09          Cl
TER
HETATM  1091  C1   NGA D   1      -0.855  -5.202  11.893  1.00 29.65           C
HETATM  1092  C2   NGA D   1      -1.637  -6.424  11.455  1.00 25.74           C
HETATM  1093  C3   NGA D   1      -0.719  -7.433  10.877  1.00 19.12           C
HETATM  1094  C4   NGA D   1       0.140  -6.858   9.782  1.00 26.89           C
HETATM  1095  C5   NGA D   1       0.800  -5.564  10.227  1.00 27.00           C
HETATM  1096  C6   NGA D   1       1.564  -4.950   9.107  1.00 23.07           C
HETATM  1097  C7   NGA D   1      -3.674  -6.716  12.908  1.00 23.79           C
HETATM  1098  C8   NGA D   1      -4.345  -7.353  14.124  1.00 17.91           C
HETATM  1099  N2   NGA D   1      -2.306  -7.017  12.612  1.00 24.92           N
HETATM  1100  O1   NGA D   1      -1.657  -4.257  12.478  1.00 40.15           O
HETATM  1101  O3   NGA D   1      -1.485  -8.575  10.399  1.00 17.51           O
HETATM  1102  O4   NGA D   1      -0.627  -6.578   8.599  1.00 18.77           O
HETATM  1103  O5   NGA D   1      -0.199  -4.606  10.704  1.00 21.93           O
HETATM  1104  O6   NGA D   1       2.201  -3.801   9.619  1.00 23.93           O
HETATM  1105  O7   NGA D   1      -4.290  -5.954  12.181  1.00 26.58           O
TER
END
```

## Table 10.2

| ATOM | 1 | N | GLN | A | 1 | 4.287 | -11.302 | 31.298 | 1.00 | 52.13 | N |
|------|---|---|-----|---|---|-------|---------|--------|------|-------|---|
| ATOM | 2 | CA | GLN | A | 1 | 2.840 | -11.140 | 31.183 | 1.00 | 68.91 | C |
| ATOM | 3 | C | GLN | A | 1 | 2.320 | -11.699 | 29.861 | 1.00 | 58.31 | C |
| ATOM | 4 | O | GLN | A | 1 | 3.071 | -11.816 | 28.894 | 1.00 | 49.26 | O |
| ATOM | 5 | CB | GLN | A | 1 | 2.457 | -9.664 | 31.320 | 1.00 | 75.74 | C |
| ATOM | 6 | CG | GLN | A | 1 | 2.710 | -9.088 | 32.707 | 1.00 | 88.73 | C |
| ATOM | 7 | CD | GLN | A | 1 | 1.879 | -9.771 | 33.783 | 1.00 | 100.20 | C |
| ATOM | 8 | NE2 | GLN | A | 1 | 2.448 | -9.903 | 34.978 | 1.00 | 94.67 | N |
| ATOM | 9 | OE1 | GLN | A | 1 | 0.742 | -10.179 | 33.539 | 1.00 | 98.51 | O |
| ATOM | 10 | N | VAL | A | 2 | 1.035 | -12.043 | 29.824 | 1.00 | 56.43 | N |
| ATOM | 11 | CA | VAL | A | 2 | 0.425 | -12.599 | 28.617 | 1.00 | 47.45 | C |
| ATOM | 12 | C | VAL | A | 2 | 0.210 | -11.533 | 27.541 | 1.00 | 49.76 | C |
| ATOM | 13 | O | VAL | A | 2 | -0.422 | -10.506 | 27.790 | 1.00 | 52.22 | O |
| ATOM | 14 | CB | VAL | A | 2 | -0.928 | -13.275 | 28.926 | 1.00 | 51.59 | C |
| ATOM | 15 | CG1 | VAL | A | 2 | -1.627 | -13.682 | 27.636 | 1.00 | 51.80 | C |
| ATOM | 16 | CG2 | VAL | A | 2 | -0.727 | -14.481 | 29.825 | 1.00 | 44.15 | C |
| ATOM | 17 | N | GLN | A | 3 | 0.740 | -11.785 | 26.348 | 1.00 | 39.57 | N |
| ATOM | 18 | CA | GLN | A | 3 | 0.582 | -10.876 | 25.219 | 1.00 | 41.94 | C |
| ATOM | 19 | C | GLN | A | 3 | 0.085 | -11.615 | 23.981 | 1.00 | 38.69 | C |
| ATOM | 20 | O | GLN | A | 3 | 0.586 | -12.691 | 23.649 | 1.00 | 39.97 | O |
| ATOM | 21 | CB | GLN | A | 3 | 1.903 | -10.176 | 24.891 | 1.00 | 40.78 | C |
| ATOM | 22 | CG | GLN | A | 3 | 2.450 | -9.275 | 25.980 | 1.00 | 61.11 | C |
| ATOM | 23 | CD | GLN | A | 3 | 3.756 | -8.612 | 25.570 | 1.00 | 71.25 | C |
| ATOM | 24 | NE2 | GLN | A | 3 | 4.265 | -7.724 | 26.417 | 1.00 | 63.18 | N |
| ATOM | 25 | OE1 | GLN | A | 3 | 4.299 | -8.899 | 24.502 | 1.00 | 74.87 | O |
| ATOM | 26 | N | LEU | A | 4 | -0.896 | -11.035 | 23.298 | 1.00 | 33.31 | N |
| ATOM | 27 | CA | LEU | A | 4 | -1.361 | -11.577 | 22.025 | 1.00 | 32.23 | C |
| ATOM | 28 | C | LEU | A | 4 | -1.077 | -10.581 | 20.903 | 1.00 | 34.08 | C |
| ATOM | 29 | O | LEU | A | 4 | -1.523 | -9.436 | 20.954 | 1.00 | 35.79 | O |
| ATOM | 30 | CB | LEU | A | 4 | -2.854 | -11.904 | 22.076 | 1.00 | 24.96 | C |
| ATOM | 31 | CG | LEU | A | 4 | -3.339 | -12.906 | 23.123 | 1.00 | 35.14 | C |
| ATOM | 32 | CD1 | LEU | A | 4 | -4.849 | -13.045 | 23.036 | 1.00 | 33.63 | C |
| ATOM | 33 | CD2 | LEU | A | 4 | -2.665 | -14.259 | 22.950 | 1.00 | 36.57 | C |
| ATOM | 34 | N | VAL | A | 5 | -0.333 | -11.023 | 19.894 | 1.00 | 34.68 | N |
| ATOM | 35 | CA | VAL | A | 5 | 0.072 | -10.150 | 18.794 | 1.00 | 31.10 | C |
| ATOM | 36 | C | VAL | A | 5 | -0.418 | -10.708 | 17.466 | 1.00 | 27.37 | C |
| ATOM | 37 | O | VAL | A | 5 | -0.029 | -11.804 | 17.061 | 1.00 | 33.78 | O |
| ATOM | 38 | CB | VAL | A | 5 | 1.603 | -9.971 | 18.747 | 1.00 | 33.23 | C |
| ATOM | 39 | CG1 | VAL | A | 5 | 2.000 | -9.098 | 17.564 | 1.00 | 39.33 | C |
| ATOM | 40 | CG2 | VAL | A | 5 | 2.107 | -9.365 | 20.052 | 1.00 | 30.20 | C |
| ATOM | 41 | N | GLN | A | 6 | -1.280 | -9.951 | 16.792 | 1.00 | 27.57 | N |
| ATOM | 42 | CA | GLN | A | 6 | -1.882 | -10.411 | 15.545 | 1.00 | 31.03 | C |
| ATOM | 43 | C | GLN | A | 6 | -1.155 | -9.849 | 14.333 | 1.00 | 32.02 | C |
| ATOM | 44 | O | GLN | A | 6 | -0.447 | -8.847 | 14.431 | 1.00 | 31.18 | O |
| ATOM | 45 | CB | GLN | A | 6 | -3.362 | -10.026 | 15.492 | 1.00 | 23.97 | C |
| ATOM | 46 | CG | GLN | A | 6 | -4.154 | -10.551 | 16.674 | 1.00 | 31.59 | C |
| ATOM | 47 | CD | GLN | A | 6 | -5.613 | -10.158 | 16.634 | 1.00 | 31.89 | C |
| ATOM | 48 | NE2 | GLN | A | 6 | -6.203 | -10.163 | 15.438 | 1.00 | 30.10 | N |
| ATOM | 49 | OE1 | GLN | A | 6 | -6.210 | -9.863 | 17.669 | 1.00 | 28.34 | O |
| ATOM | 50 | N | SER | A | 7 | -1.343 | -10.498 | 13.190 | 1.00 | 28.18 | N |
| ATOM | 51 | CA | SER | A | 7 | -0.769 | -10.018 | 11.941 | 1.00 | 37.96 | C |
| ATOM | 52 | C | SER | A | 7 | -1.529 | -8.784 | 11.456 | 1.00 | 36.31 | C |
| ATOM | 53 | O | SER | A | 7 | -2.504 | -8.356 | 12.088 | 1.00 | 29.94 | O |
| ATOM | 54 | CB | SER | A | 7 | -0.782 | -11.125 | 10.884 | 1.00 | 31.07 | C |
| ATOM | 55 | OG | SER | A | 7 | -2.003 | -11.840 | 10.917 | 1.00 | 34.72 | O |
| ATOM | 56 | N | GLY | A | 8 | -1.084 | -8.220 | 10.336 | 1.00 | 37.68 | N |
| ATOM | 57 | CA | GLY | A | 8 | -1.579 | -6.932 | 9.880 | 1.00 | 34.93 | C |
| ATOM | 58 | C | GLY | A | 8 | -2.778 | -6.994 | 8.957 | 1.00 | 39.24 | C |
| ATOM | 59 | O | GLY | A | 8 | -3.278 | -8.071 | 8.641 | 1.00 | 43.54 | O |
| ATOM | 60 | N | THR | A | 9 | -3.221 | -5.818 | 8.519 | 1.00 | 33.12 | N |
| ATOM | 61 | CA | THR | A | 9 | -4.417 | -5.660 | 7.698 | 1.00 | 34.90 | C |
| ATOM | 62 | C | THR | A | 9 | -4.411 | -6.499 | 6.424 | 1.00 | 34.53 | C |
| ATOM | 63 | O | THR | A | 9 | -3.369 | -6.699 | 5.809 | 1.00 | 36.17 | O |
| ATOM | 64 | CB | THR | A | 9 | -4.612 | -4.186 | 7.290 | 1.00 | 34.99 | C |
| ATOM | 65 | OG1 | THR | A | 9 | -4.305 | -3.333 | 8.400 | 1.00 | 54.03 | O |
| ATOM | 66 | CG2 | THR | A | 9 | -6.032 | -3.948 | 6.877 | 1.00 | 25.09 | C |
| ATOM | 67 | N | GLU | A | 10 | -5.591 | -6.977 | 6.042 | 1.00 | 38.28 | N |
| ATOM | 68 | CA | GLU | A | 10 | -5.770 | -7.781 | 4.840 | 1.00 | 36.11 | C |
| ATOM | 69 | C | GLU | A | 10 | -6.846 | -7.183 | 3.942 | 1.00 | 42.38 | C |

| ATOM | 70 | O | GLU A | 10 | -7.825 | -6.614 | 4.433 | 1.00 | 36.65 | O |
|------|-----|-----|-------|-----|---------|---------|--------|------|-------|---|
| ATOM | 71 | CB | GLU A | 10 | -6.156 | -9.217 | 5.204 | 1.00 | 41.15 | C |
| ATOM | 72 | CG | GLU A | 10 | -5.177 | -9.938 | 6.112 | 1.00 | 45.12 | C |
| ATOM | 73 | CD | GLU A | 10 | -4.128 | -10.719 | 5.342 | 1.00 | 54.57 | C |
| ATOM | 74 | OE1 | GLU A | 10 | -3.998 | -10.501 | 4.118 | 1.00 | 60.79 | O |
| ATOM | 75 | OE2 | GLU A | 10 | -3.438 | -11.556 | 5.963 | 1.00 | 51.03 | O |
| ATOM | 76 | N | VAL A | 11 | -6.659 | -7.314 | 2.630 | 1.00 | 33.01 | N |
| ATOM | 77 | CA | VAL A | 11 | -7.690 | -6.970 | 1.654 | 1.00 | 35.41 | C |
| ATOM | 78 | C | VAL A | 11 | -7.886 | -8.152 | 0.709 | 1.00 | 37.75 | C |
| ATOM | 79 | O | VAL A | 11 | -6.920 | -8.678 | 0.153 | 1.00 | 40.66 | O |
| ATOM | 80 | CB | VAL A | 11 | -7.331 | -5.718 | 0.826 | 1.00 | 41.44 | C |
| ATOM | 81 | CG1 | VAL A | 11 | -8.587 | -5.137 | 0.181 | 1.00 | 34.49 | C |
| ATOM | 82 | CG2 | VAL A | 11 | -6.642 | -4.681 | 1.692 | 1.00 | 43.75 | C |
| ATOM | 83 | N | LYS A | 12 | -9.133 | -8.572 | 0.535 | 1.00 | 35.34 | N |
| ATOM | 84 | CA | LYS A | 12 | -9.430 | -9.745 | -0.276 | 1.00 | 34.88 | C |
| ATOM | 85 | C | LYS A | 12 | -10.619 | -9.497 | -1.196 | 1.00 | 41.54 | C |
| ATOM | 86 | O | LYS A | 12 | -11.503 | -8.701 | -0.881 | 1.00 | 37.51 | O |
| ATOM | 87 | CB | LYS A | 12 | -9.716 | -10.958 | 0.618 | 1.00 | 37.30 | C |
| ATOM | 88 | CG | LYS A | 12 | -8.570 | -11.375 | 1.529 | 1.00 | 31.97 | C |
| ATOM | 89 | CD | LYS A | 12 | -7.396 | -11.926 | 0.737 | 1.00 | 39.11 | C |
| ATOM | 90 | CE | LYS A | 12 | -6.276 | -12.372 | 1.664 | 1.00 | 40.25 | C |
| ATOM | 91 | NZ | LYS A | 12 | -5.084 | -12.848 | 0.910 | 1.00 | 43.21 | N |
| ATOM | 92 | N | LYS A | 13 | -10.641 | -10.186 | -2.332 | 1.00 | 40.85 | N |
| ATOM | 93 | CA | LYS A | 13 | -11.800 | -10.147 | -3.217 | 1.00 | 46.07 | C |
| ATOM | 94 | C | LYS A | 13 | -12.873 | -11.095 | -2.687 | 1.00 | 41.63 | C |
| ATOM | 95 | O | LYS A | 13 | -12.557 | -12.058 | -1.991 | 1.00 | 42.80 | O |
| ATOM | 96 | CB | LYS A | 13 | -11.401 | -10.525 | -4.646 | 1.00 | 44.49 | C |
| ATOM | 97 | CG | LYS A | 13 | -10.368 | -9.597 | -5.262 | 1.00 | 51.17 | C |
| ATOM | 98 | CD | LYS A | 13 | -10.037 | -10.015 | -6.686 | 1.00 | 69.57 | C |
| ATOM | 99 | CE | LYS A | 13 | -9.093 | -9.026 | -7.351 | 1.00 | 76.15 | C |
| ATOM | 100 | NZ | LYS A | 13 | -8.805 | -9.405 | -8.763 | 1.00 | 81.80 | N |
| ATOM | 101 | N | PRO A | 14 | -14.149 | -10.815 | -2.992 | 1.00 | 40.61 | N |
| ATOM | 102 | CA | PRO A | 14 | -15.210 | -11.741 | -2.581 | 1.00 | 42.09 | C |
| ATOM | 103 | C | PRO A | 14 | -14.959 | -13.147 | -3.124 | 1.00 | 43.49 | C |
| ATOM | 104 | O | PRO A | 14 | -14.532 | -13.286 | -4.268 | 1.00 | 43.97 | O |
| ATOM | 105 | CB | PRO A | 14 | -16.474 | -11.125 | -3.191 | 1.00 | 41.78 | C |
| ATOM | 106 | CG | PRO A | 14 | -16.153 | -9.669 | -3.327 | 1.00 | 42.84 | C |
| ATOM | 107 | CD | PRO A | 14 | -14.683 | -9.605 | -3.641 | 1.00 | 40.32 | C |
| ATOM | 108 | N | GLY A | 15 | -15.191 | -14.166 | -2.302 | 1.00 | 44.15 | N |
| ATOM | 109 | CA | GLY A | 15 | -14.989 | -15.542 | -2.716 | 1.00 | 39.13 | C |
| ATOM | 110 | C | GLY A | 15 | -13.611 | -16.090 | -2.391 | 1.00 | 48.08 | C |
| ATOM | 111 | O | GLY A | 15 | -13.385 | -17.297 | -2.469 | 1.00 | 45.50 | O |
| ATOM | 112 | N | ALA A | 16 | -12.683 | -15.208 | -2.028 | 1.00 | 41.49 | N |
| ATOM | 113 | CA | ALA A | 16 | -11.334 | -15.636 | -1.665 | 1.00 | 40.38 | C |
| ATOM | 114 | C | ALA A | 16 | -11.263 | -16.095 | -0.209 | 1.00 | 44.65 | C |
| ATOM | 115 | O | ALA A | 16 | -12.281 | -16.184 | 0.478 | 1.00 | 39.46 | O |
| ATOM | 116 | CB | ALA A | 16 | -10.340 | -14.512 | -1.908 | 1.00 | 42.69 | C |
| ATOM | 117 | N | SER A | 17 | -10.052 | -16.383 | 0.257 | 1.00 | 41.27 | N |
| ATOM | 118 | C | SER A | 17 | -8.822 | -15.906 | 2.328 | 1.00 | 41.94 | C |
| ATOM | 119 | O | SER A | 17 | -7.925 | -15.363 | 1.688 | 1.00 | 46.41 | O |
| ATOM | 120 | CA A | SER A | 17 | -9.841 | -16.800 | 1.638 | 0.70 | 41.50 | C |
| ATOM | 121 | CB A | SER A | 17 | -9.382 | -18.258 | 1.699 | 0.70 | 42.17 | C |
| ATOM | 122 | OG A | SER A | 17 | -10.402 | -19.130 | 1.247 | 0.70 | 48.56 | O |
| ATOM | 123 | CA B | SER A | 17 | -9.844 | -16.800 | 1.638 | 0.30 | 41.51 | C |
| ATOM | 124 | CB B | SER A | 17 | -9.387 | -18.259 | 1.703 | 0.30 | 42.20 | C |
| ATOM | 125 | OG B | SER A | 17 | -8.050 | -18.394 | 1.253 | 0.30 | 38.89 | O |
| ATOM | 126 | N | VAL A | 18 | -8.963 | -15.760 | 3.639 | 1.00 | 40.15 | N |
| ATOM | 127 | CA | VAL A | 18 | -8.023 | -14.966 | 4.414 | 1.00 | 37.97 | C |
| ATOM | 128 | C | VAL A | 18 | -7.539 | -15.771 | 5.615 | 1.00 | 36.78 | C |
| ATOM | 129 | O | VAL A | 18 | -8.313 | -16.499 | 6.237 | 1.00 | 43.11 | O |
| ATOM | 130 | CB | VAL A | 18 | -8.657 | -13.636 | 4.879 | 1.00 | 39.97 | C |
| ATOM | 131 | CG1 | VAL A | 18 | -9.911 | -13.893 | 5.706 | 1.00 | 34.36 | C |
| ATOM | 132 | CG2 | VAL A | 18 | -7.651 | -12.802 | 5.661 | 1.00 | 41.62 | C |
| ATOM | 133 | N | LYS A | 19 | -6.251 | -15.664 | 5.921 | 1.00 | 39.92 | N |
| ATOM | 134 | CA | LYS A | 19 | -5.706 | -16.304 | 7.110 | 1.00 | 40.61 | C |
| ATOM | 135 | C | LYS A | 19 | -5.015 | -15.279 | 8.012 | 1.00 | 41.01 | C |
| ATOM | 136 | O | LYS A | 19 | -4.067 | -14.610 | 7.599 | 1.00 | 42.89 | O |
| ATOM | 137 | CB | LYS A | 19 | -4.737 | -17.424 | 6.731 | 1.00 | 38.22 | C |
| ATOM | 138 | CG | LYS A | 19 | -4.105 | -18.105 | 7.932 | 1.00 | 35.23 | C |
| ATOM | 139 | CD | LYS A | 19 | -3.153 | -19.219 | 7.527 | 1.00 | 37.78 | C |
| ATOM | 140 | CE | LYS A | 19 | -3.892 | -20.532 | 7.324 | 1.00 | 52.29 | C |

```
ATOM    141  NZ   LYS A  19     -2.953 -21.693   7.310  1.00 57.68           N
ATOM    142  N    VAL A  20     -5.511 -15.165   9.241  1.00 38.03           N
ATOM    143  CA   VAL A  20     -4.982 -14.234  10.235  1.00 34.63           C
ATOM    144  C    VAL A  20     -4.240 -15.005  11.332  1.00 39.64           C
ATOM    145  O    VAL A  20     -4.658 -16.099  11.720  1.00 36.81           O
ATOM    146  CB   VAL A  20     -6.115 -13.388  10.867  1.00 33.08           C
ATOM    147  CG1  VAL A  20     -5.553 -12.359  11.839  1.00 39.43           C
ATOM    148  CG2  VAL A  20     -6.941 -12.707   9.784  1.00 38.82           C
ATOM    149  N    SER A  21     -3.144 -14.439  11.832  1.00 30.21           N
ATOM    150  CA   SER A  21     -2.338 -15.124  12.838  1.00 32.22           C
ATOM    151  C    SER A  21     -2.354 -14.384  14.168  1.00 34.12           C
ATOM    152  O    SER A  21     -2.566 -13.176  14.218  1.00 32.94           O
ATOM    153  CB   SER A  21     -0.892 -15.289  12.354  1.00 32.38           C
ATOM    154  OG   SER A  21     -0.205 -14.046  12.353  1.00 35.31           O
ATOM    155  N    CYS A  22     -2.116 -15.123  15.244  1.00 36.35           N
ATOM    156  CA   CYS A  22     -2.109 -14.565  16.587  1.00 33.43           C
ATOM    157  C    CYS A  22     -0.985 -15.204  17.401  1.00 38.78           C
ATOM    158  O    CYS A  22     -1.059 -16.380  17.757  1.00 39.38           O
ATOM    159  CB   CYS A  22     -3.468 -14.791  17.260  1.00 38.36           C
ATOM    160  SG   CYS A  22     -3.592 -14.210  18.969  1.00 47.87           S
ATOM    161  N    LYS A  23      0.064 -14.440  17.681  1.00 37.86           N
ATOM    162  CA   LYS A  23      1.207 -14.980  18.410  1.00 40.02           C
ATOM    163  C    LYS A  23      1.019 -14.817  19.912  1.00 36.84           C
ATOM    164  O    LYS A  23      0.925 -13.697  20.415  1.00 38.22           O
ATOM    165  CB   LYS A  23      2.506 -14.303  17.966  1.00 40.53           C
ATOM    166  CG   LYS A  23      3.763 -15.022  18.446  1.00 49.89           C
ATOM    167  CD   LYS A  23      5.015 -14.206  18.167  1.00 49.82           C
ATOM    168  CE   LYS A  23      6.195 -15.104  17.823  1.00 63.34           C
ATOM    169  NZ   LYS A  23      6.392 -16.183  18.827  1.00 59.88           N
ATOM    170  N    ALA A  24      0.960 -15.940  20.621  1.00 39.67           N
ATOM    171  CA   ALA A  24      0.756 -15.929  22.066  1.00 44.74           C
ATOM    172  C    ALA A  24      2.070 -16.103  22.819  1.00 52.29           C
ATOM    173  O    ALA A  24      2.952 -16.844  22.390  1.00 57.40           O
ATOM    174  CB   ALA A  24     -0.227 -17.014  22.468  1.00 43.19           C
ATOM    175  N    SER A  25      2.193 -15.416  23.949  1.00 49.70           N
ATOM    176  CA   SER A  25      3.390 -15.511  24.771  1.00 51.40           C
ATOM    177  C    SER A  25      3.108 -15.073  26.206  1.00 52.18           C
ATOM    178  O    SER A  25      2.195 -14.282  26.449  1.00 44.33           O
ATOM    179  CB   SER A  25      4.513 -14.665  24.171  1.00 54.47           C
ATOM    180  OG   SER A  25      4.108 -13.314  24.022  1.00 61.15           O
ATOM    181  N    GLY A  26      3.887 -15.597  27.149  1.00 43.62           N
ATOM    182  CA   GLY A  26      3.797 -15.182  28.539  1.00 42.96           C
ATOM    183  C    GLY A  26      2.914 -16.042  29.429  1.00 47.24           C
ATOM    184  O    GLY A  26      2.711 -15.718  30.599  1.00 47.28           O
ATOM    185  N    TYR A  27      2.388 -17.136  28.889  1.00 41.19           N
ATOM    186  CA   TYR A  27      1.504 -18.006  29.657  1.00 39.91           C
ATOM    187  C    TYR A  27      2.259 -18.809  30.704  1.00 53.27           C
ATOM    188  O    TYR A  27      3.307 -19.393  30.423  1.00 48.93           O
ATOM    189  CB   TYR A  27      0.740 -18.956  28.733  1.00 48.01           C
ATOM    190  CG   TYR A  27     -0.376 -18.280  27.978  1.00 52.64           C
ATOM    191  CD2  TYR A  27     -0.151 -17.727  26.726  1.00 45.16           C
ATOM    192  CD1  TYR A  27     -1.655 -18.180  28.526  1.00 47.65           C
ATOM    193  CE2  TYR A  27     -1.163 -17.099  26.032  1.00 50.12           C
ATOM    194  CE1  TYR A  27     -2.678 -17.555  27.837  1.00 41.89           C
ATOM    195  CZ   TYR A  27     -2.422 -17.015  26.590  1.00 49.26           C
ATOM    196  OH   TYR A  27     -3.417 -16.385  25.887  1.00 50.17           O
ATOM    197  N    THR A  28      1.705 -18.844  31.910  1.00 55.54           N
ATOM    198  CA   THR A  28      2.345 -19.511  33.035  1.00 52.97           C
ATOM    199  C    THR A  28      1.695 -20.867  33.330  1.00 58.74           C
ATOM    200  O    THR A  28      2.080 -21.560  34.275  1.00 53.17           O
ATOM    201  CB   THR A  28      2.301 -18.622  34.297  1.00 45.57           C
ATOM    202  CG2  THR A  28      0.870 -18.237  34.629  1.00 50.36           C
ATOM    203  OG1  THR A  28      2.873 -19.326  35.405  1.00 80.42           O
ATOM    204  O    PHE A  29     -0.347 -22.422  30.294  1.00 42.50           O
ATOM    205  N    PHE A  29      0.711 -21.242  32.516  1.00 52.37           N
ATOM    206  CA   PHE A  29      0.048 -22.536  32.661  1.00 40.27           C
ATOM    207  C    PHE A  29     -0.288 -23.133  31.297  1.00 41.00           C
ATOM    208  CB   PHE A  29     -1.216 -22.409  33.519  1.00 44.95           C
ATOM    209  CG   PHE A  29     -2.147 -21.312  33.081  1.00 42.97           C
ATOM    210  CD2  PHE A  29     -2.146 -20.087  33.728  1.00 44.83           C
ATOM    211  CD1  PHE A  29     -3.034 -21.512  32.034  1.00 41.74           C
```

```
ATOM   212  CE2 PHE A  29     -3.005 -19.077  33.334  1.00 49.55           C
ATOM   213  CE1 PHE A  29     -3.893 -20.508  31.635  1.00 43.96           C
ATOM   214  CZ  PHE A  29     -3.879 -19.287  32.286  1.00 50.75           C
ATOM   215  O   THR A  30     -1.919 -25.101  28.021  1.00 42.53           O
ATOM   216  N   THR A  30     -0.530 -24.439  31.272  1.00 40.36           N
ATOM   217  CA  THR A  30     -0.586 -25.179  30.016  1.00 42.39           C
ATOM   218  C   THR A  30     -1.914 -25.074  29.255  1.00 42.49           C
ATOM   219  CB  THR A  30     -0.281 -26.682  30.251  1.00 43.93           C
ATOM   220  OG1 THR A  30     -1.295 -27.254  31.083  1.00 62.78           O
ATOM   221  CG2 THR A  30      1.071 -26.857  30.923  1.00 45.80           C
ATOM   222  O   ASN A  31     -5.415 -23.080  30.210  1.00 32.62           O
ATOM   223  N   ASN A  31     -3.035 -24.959  29.965  1.00 33.58           N
ATOM   224  CA  ASN A  31     -4.334 -25.021  29.288  1.00 29.92           C
ATOM   225  C   ASN A  31     -5.085 -23.699  29.201  1.00 35.69           C
ATOM   226  CB  ASN A  31     -5.230 -26.055  29.963  1.00 36.07           C
ATOM   227  CG  ASN A  31     -4.646 -27.449  29.903  1.00 37.13           C
ATOM   228  OD1 ASN A  31     -4.449 -28.006  28.821  1.00 32.81           O
ATOM   229  ND2 ASN A  31     -4.361 -28.018  31.066  1.00 28.91           N
ATOM   230  N   TYR A  32     -5.354 -23.289  27.968  1.00 31.28           N
ATOM   231  CA  TYR A  32     -6.159 -22.112  27.677  1.00 34.89           C
ATOM   232  C   TYR A  32     -6.695 -22.261  26.258  1.00 37.67           C
ATOM   233  O   TYR A  32     -6.197 -23.085  25.491  1.00 29.11           O
ATOM   234  CB  TYR A  32     -5.339 -20.825  27.826  1.00 30.61           C
ATOM   235  CG  TYR A  32     -4.090 -20.799  26.970  1.00 42.67           C
ATOM   236  CD1 TYR A  32     -2.900 -21.355  27.425  1.00 39.54           C
ATOM   237  CD2 TYR A  32     -4.101 -20.225  25.704  1.00 38.42           C
ATOM   238  CE1 TYR A  32     -1.758 -21.341  26.643  1.00 41.78           C
ATOM   239  CE2 TYR A  32     -2.962 -20.206  24.921  1.00 42.06           C
ATOM   240  CZ  TYR A  32     -1.797 -20.765  25.393  1.00 47.23           C
ATOM   241  OH  TYR A  32     -0.666 -20.741  24.609  1.00 56.16           O
ATOM   242  N   ASP A  33     -7.716 -21.482  25.919  1.00 30.49           N
ATOM   243  CA  ASP A  33     -8.271 -21.484  24.570  1.00 29.65           C
ATOM   244  C   ASP A  33     -7.998 -20.161  23.883  1.00 31.10           C
ATOM   245  O   ASP A  33     -8.062 -19.113  24.513  1.00 28.34           O
ATOM   246  CB  ASP A  33     -9.779 -21.734  24.591  1.00 29.08           C
ATOM   247  CG  ASP A  33    -10.139 -23.124  25.082  1.00 34.81           C
ATOM   248  OD1 ASP A  33     -9.427 -24.090  24.729  1.00 31.12           O
ATOM   249  OD2 ASP A  33    -11.145 -23.247  25.814  1.00 30.55           O
ATOM   250  N   ILE A  34     -7.685 -20.204  22.593  1.00 28.80           N
ATOM   251  CA  ILE A  34     -7.698 -18.989  21.801  1.00 29.45           C
ATOM   252  C   ILE A  34     -9.070 -18.897  21.155  1.00 28.08           C
ATOM   253  O   ILE A  34     -9.518 -19.838  20.500  1.00 32.08           O
ATOM   254  CB  ILE A  34     -6.587 -18.954  20.729  1.00 34.02           C
ATOM   255  CG1 ILE A  34     -5.301 -18.380  21.315  1.00 40.10           C
ATOM   256  CG2 ILE A  34     -6.981 -18.048  19.581  1.00 33.89           C
ATOM   257  CD1 ILE A  34     -4.555 -19.327  22.160  1.00 37.52           C
ATOM   258  N   ASN A  35     -9.743 -17.774  21.381  1.00 23.45           N
ATOM   259  CA  ASN A  35    -11.044 -17.502  20.786  1.00 28.14           C
ATOM   260  C   ASN A  35    -10.911 -16.447  19.693  1.00 30.81           C
ATOM   261  O   ASN A  35    -10.013 -15.607  19.733  1.00 33.76           O
ATOM   262  CB  ASN A  35    -12.045 -17.023  21.849  1.00 30.44           C
ATOM   263  CG  ASN A  35    -12.218 -18.015  22.989  1.00 30.22           C
ATOM   264  OD1 ASN A  35    -13.183 -18.779  23.018  1.00 29.29           O
ATOM   265  ND2 ASN A  35    -11.293 -17.995  23.943  1.00 26.20           N
ATOM   266  N   TRP A  36    -11.802 -16.482  18.715  1.00 28.47           N
ATOM   267  CA  TRP A  36    -11.807 -15.447  17.697  1.00 32.59           C
ATOM   268  C   TRP A  36    -13.139 -14.720  17.718  1.00 32.65           C
ATOM   269  O   TRP A  36    -14.202 -15.337  17.703  1.00 30.35           O
ATOM   270  CB  TRP A  36    -11.509 -16.038  16.318  1.00 30.51           C
ATOM   271  CG  TRP A  36    -10.106 -16.553  16.242  1.00 27.75           C
ATOM   272  CD1 TRP A  36     -9.665 -17.781  16.637  1.00 28.80           C
ATOM   273  CD2 TRP A  36     -8.951 -15.840  15.779  1.00 33.27           C
ATOM   274  CE2 TRP A  36     -7.847 -16.707  15.906  1.00 31.39           C
ATOM   275  CE3 TRP A  36     -8.745 -14.557  15.260  1.00 31.98           C
ATOM   276  NE1 TRP A  36     -8.309 -17.884  16.434  1.00 36.72           N
ATOM   277  CZ2 TRP A  36     -6.554 -16.334  15.535  1.00 38.10           C
ATOM   278  CZ3 TRP A  36     -7.457 -14.186  14.890  1.00 30.43           C
ATOM   279  CH2 TRP A  36     -6.380 -15.071  15.030  1.00 33.42           C
ATOM   280  N   VAL A  37    -13.053 -13.397  17.790  1.00 27.11           N
ATOM   281  CA  VAL A  37    -14.207 -12.528  17.936  1.00 30.57           C
ATOM   282  C   VAL A  37    -14.076 -11.410  16.914  1.00 33.89           C
```

| ATOM | 283 | O | VAL | A | 37 | -13.032 | -10.759 | 16.846 | 1.00 | 33.58 | O |
| ATOM | 284 | CB | VAL | A | 37 | -14.301 | -11.929 | 19.370 | 1.00 | 25.89 | C |
| ATOM | 285 | CG1 | VAL | A | 37 | -15.467 | -10.957 | 19.478 | 1.00 | 27.13 | C |
| ATOM | 286 | CG2 | VAL | A | 37 | -14.423 | -13.031 | 20.417 | 1.00 | 26.86 | C |
| ATOM | 287 | N | ARG | A | 38 | -15.116 | -11.188 | 16.115 | 1.00 | 29.31 | N |
| ATOM | 288 | CA | ARG | A | 38 | -15.054 | -10.150 | 15.088 | 1.00 | 28.87 | C |
| ATOM | 289 | C | ARG | A | 38 | -16.002 | -8.995 | 15.395 | 1.00 | 32.02 | C |
| ATOM | 290 | O | ARG | A | 38 | -17.005 | -9.163 | 16.089 | 1.00 | 30.64 | O |
| ATOM | 291 | CB | ARG | A | 38 | -15.367 | -10.732 | 13.708 | 1.00 | 35.52 | C |
| ATOM | 292 | CG | ARG | A | 38 | -16.652 | -11.529 | 13.656 | 1.00 | 39.93 | C |
| ATOM | 293 | CD | ARG | A | 38 | -17.550 | -11.096 | 12.506 | 1.00 | 43.60 | C |
| ATOM | 294 | NE | ARG | A | 38 | -17.164 | -11.681 | 11.229 | 1.00 | 44.71 | N |
| ATOM | 295 | CZ | ARG | A | 38 | -18.023 | -12.119 | 10.311 | 1.00 | 45.96 | C |
| ATOM | 296 | NH1 | ARG | A | 38 | -19.329 | -12.055 | 10.529 | 1.00 | 39.60 | N |
| ATOM | 297 | NH2 | ARG | A | 38 | -17.572 | -12.626 | 9.170 | 1.00 | 33.94 | N |
| ATOM | 298 | N | GLN | A | 39 | -15.669 | -7.821 | 14.871 | 1.00 | 30.63 | N |
| ATOM | 299 | CA | GLN | A | 39 | -16.452 | -6.618 | 15.103 | 1.00 | 28.53 | C |
| ATOM | 300 | C | GLN | A | 39 | -16.568 | -5.795 | 13.825 | 1.00 | 31.84 | C |
| ATOM | 301 | O | GLN | A | 39 | -15.569 | -5.314 | 13.294 | 1.00 | 29.51 | O |
| ATOM | 302 | CB | GLN | A | 39 | -15.819 | -5.775 | 16.219 | 1.00 | 30.14 | C |
| ATOM | 303 | CG | GLN | A | 39 | -16.516 | -4.444 | 16.478 | 1.00 | 28.25 | C |
| ATOM | 304 | CD | GLN | A | 39 | -16.024 | -3.781 | 17.751 | 1.00 | 39.30 | C |
| ATOM | 305 | NE2 | GLN | A | 39 | -16.896 | -3.689 | 18.751 | 1.00 | 30.37 | N |
| ATOM | 306 | OE1 | GLN | A | 39 | -14.870 | -3.368 | 17.839 | 1.00 | 39.08 | O |
| ATOM | 307 | N | ALA | A | 40 | -17.788 | -5.637 | 13.330 | 1.00 | 37.04 | N |
| ATOM | 308 | CA | ALA | A | 40 | -18.016 | -4.815 | 12.149 | 1.00 | 44.35 | C |
| ATOM | 309 | C | ALA | A | 40 | -18.259 | -3.363 | 12.548 | 1.00 | 49.15 | C |
| ATOM | 310 | O | ALA | A | 40 | -18.880 | -3.094 | 13.579 | 1.00 | 49.51 | O |
| ATOM | 311 | CB | ALA | A | 40 | -19.190 | -5.352 | 11.341 | 1.00 | 36.53 | C |
| ATOM | 312 | N | THR | A | 41 | -17.768 | -2.444 | 11.715 | 1.00 | 55.43 | N |
| ATOM | 313 | CA | THR | A | 41 | -17.923 | -0.987 | 11.865 | 1.00 | 45.13 | C |
| ATOM | 314 | C | THR | A | 41 | -17.917 | -0.449 | 13.304 | 1.00 | 61.05 | C |
| ATOM | 315 | O | THR | A | 41 | -18.779 | 0.345 | 13.688 | 1.00 | 60.70 | O |
| ATOM | 316 | CB | THR | A | 41 | -19.224 | -0.480 | 11.157 | 1.00 | 56.54 | C |
| ATOM | 317 | OG1 | THR | A | 41 | -19.398 | 0.918 | 11.416 | 1.00 | 74.65 | O |
| ATOM | 318 | CG2 | THR | A | 41 | -20.475 | -1.233 | 11.611 | 1.00 | 50.35 | C |
| ATOM | 319 | N | GLY | A | 42 | -16.931 | -0.880 | 14.088 | 1.00 | 60.31 | N |
| ATOM | 320 | CA | GLY | A | 42 | -16.677 | -0.311 | 15.402 | 1.00 | 50.20 | C |
| ATOM | 321 | C | GLY | A | 42 | -17.674 | -0.603 | 16.515 | 1.00 | 58.90 | C |
| ATOM | 322 | O | GLY | A | 42 | -17.496 | -0.125 | 17.638 | 1.00 | 64.22 | O |
| ATOM | 323 | N | GLN | A | 43 | -18.718 | -1.377 | 16.225 | 1.00 | 61.17 | N |
| ATOM | 324 | CA | GLN | A | 43 | -19.700 | -1.716 | 17.255 | 1.00 | 58.73 | C |
| ATOM | 325 | C | GLN | A | 43 | -20.368 | -3.072 | 17.007 | 1.00 | 47.27 | C |
| ATOM | 326 | O | GLN | A | 43 | -20.811 | -3.369 | 15.897 | 1.00 | 54.96 | O |
| ATOM | 327 | CB | GLN | A | 43 | -20.761 | -0.613 | 17.362 | 1.00 | 57.68 | C |
| ATOM | 328 | CG | GLN | A | 43 | -21.613 | -0.690 | 18.627 | 1.00 | 55.99 | C |
| ATOM | 329 | CD | GLN | A | 43 | -22.130 | 0.669 | 19.075 | 1.00 | 60.55 | C |
| ATOM | 330 | NE2 | GLN | A | 43 | -21.668 | 1.727 | 18.416 | 1.00 | 61.26 | N |
| ATOM | 331 | OE1 | GLN | A | 43 | -22.929 | 0.765 | 20.007 | 1.00 | 62.03 | O |
| ATOM | 332 | N | GLY | A | 44 | -20.438 | -3.885 | 18.059 | 1.00 | 52.11 | N |
| ATOM | 333 | CA | GLY | A | 44 | -21.017 | -5.215 | 17.978 | 1.00 | 38.87 | C |
| ATOM | 334 | C | GLY | A | 44 | -19.946 | -6.290 | 17.933 | 1.00 | 40.89 | C |
| ATOM | 335 | O | GLY | A | 44 | -19.033 | -6.225 | 17.115 | 1.00 | 48.89 | O |
| ATOM | 336 | N | LEU | A | 45 | -20.051 | -7.281 | 18.811 | 1.00 | 28.02 | N |
| ATOM | 337 | CA | LEU | A | 45 | -19.065 | -8.355 | 18.862 | 1.00 | 32.48 | C |
| ATOM | 338 | C | LEU | A | 45 | -19.726 | -9.703 | 18.639 | 1.00 | 29.29 | C |
| ATOM | 339 | O | LEU | A | 45 | -20.768 | -9.993 | 19.226 | 1.00 | 35.37 | O |
| ATOM | 340 | CB | LEU | A | 45 | -18.331 | -8.345 | 20.209 | 1.00 | 30.66 | C |
| ATOM | 341 | CG | LEU | A | 45 | -17.553 | -7.072 | 20.561 | 1.00 | 31.07 | C |
| ATOM | 342 | CD1 | LEU | A | 45 | -17.379 | -6.937 | 22.068 | 1.00 | 32.58 | C |
| ATOM | 343 | CD2 | LEU | A | 45 | -16.198 | -7.075 | 19.873 | 1.00 | 29.40 | C |
| ATOM | 344 | N | GLU | A | 46 | -19.140 | -10.533 | 17.785 | 1.00 | 29.17 | N |
| ATOM | 345 | CA | GLU | A | 46 | -19.639 | -11.895 | 17.673 | 1.00 | 35.62 | C |
| ATOM | 346 | C | GLU | A | 46 | -18.513 | -12.920 | 17.699 | 1.00 | 35.22 | C |
| ATOM | 347 | O | GLU | A | 46 | -17.496 | -12.798 | 17.009 | 1.00 | 32.17 | O |
| ATOM | 348 | CB | GLU | A | 46 | -20.511 | -12.072 | 16.421 | 1.00 | 37.90 | C |
| ATOM | 349 | CG | GLU | A | 46 | -19.828 | -11.914 | 15.092 | 1.00 | 47.11 | C |
| ATOM | 350 | CD | GLU | A | 46 | -20.765 | -12.211 | 13.925 | 1.00 | 55.52 | C |
| ATOM | 351 | OE1 | GLU | A | 46 | -21.675 | -13.052 | 14.087 | 1.00 | 54.51 | O |
| ATOM | 352 | OE2 | GLU | A | 46 | -20.601 | -11.595 | 12.850 | 1.00 | 58.10 | O |
| ATOM | 353 | N | TRP | A | 47 | -18.732 | -13.926 | 18.536 | 1.00 | 32.28 | N |

| ATOM | 354 | CA | TRP | A | 47 | -17.800 | -15.012 | 18.788 | 1.00 | 36.07 | C |
|------|-----|-----|-----|---|----|---------|---------|--------|------|-------|---|
| ATOM | 355 | C | TRP | A | 47 | -17.838 | -16.016 | 17.641 | 1.00 | 34.08 | C |
| ATOM | 356 | O | TRP | A | 47 | -18.910 | -16.392 | 17.169 | 1.00 | 29.79 | O |
| ATOM | 357 | CB | TRP | A | 47 | -18.166 | -15.664 | 20.121 | 1.00 | 25.80 | C |
| ATOM | 358 | CG | TRP | A | 47 | -17.311 | -16.791 | 20.594 | 1.00 | 33.81 | C |
| ATOM | 359 | CD1 | TRP | A | 47 | -16.096 | -16.703 | 21.210 | 1.00 | 32.06 | C |
| ATOM | 360 | CD2 | TRP | A | 47 | -17.644 | -18.182 | 20.562 | 1.00 | 33.38 | C |
| ATOM | 361 | CE2 | TRP | A | 47 | -16.572 | -18.882 | 21.149 | 1.00 | 34.18 | C |
| ATOM | 362 | CE3 | TRP | A | 47 | -18.740 | -18.904 | 20.079 | 1.00 | 35.50 | C |
| ATOM | 363 | NE1 | TRP | A | 47 | -15.638 | -17.957 | 21.536 | 1.00 | 29.56 | N |
| ATOM | 364 | CZ2 | TRP | A | 47 | -16.565 | -20.271 | 21.268 | 1.00 | 31.56 | C |
| ATOM | 365 | CZ3 | TRP | A | 47 | -18.729 | -20.282 | 20.196 | 1.00 | 42.35 | C |
| ATOM | 366 | CH2 | TRP | A | 47 | -17.647 | -20.950 | 20.787 | 1.00 | 34.97 | C |
| ATOM | 367 | N | MET | A | 48 | -16.668 | -16.437 | 17.179 | 1.00 | 32.09 | N |
| ATOM | 368 | CA | MET | A | 48 | -16.596 | -17.326 | 16.028 | 1.00 | 30.89 | C |
| ATOM | 369 | C | MET | A | 48 | -16.261 | -18.755 | 16.429 | 1.00 | 29.94 | C |
| ATOM | 370 | O | MET | A | 48 | -16.653 | -19.705 | 15.757 | 1.00 | 33.43 | O |
| ATOM | 371 | CB | MET | A | 48 | -15.558 | -16.823 | 15.028 | 1.00 | 28.48 | C |
| ATOM | 372 | CG | MET | A | 48 | -15.790 | -15.409 | 14.538 | 1.00 | 33.13 | C |
| ATOM | 373 | SD | MET | A | 48 | -14.488 | -14.924 | 13.396 | 1.00 | 37.93 | S |
| ATOM | 374 | CE | MET | A | 48 | -14.665 | -16.165 | 12.119 | 1.00 | 39.94 | C |
| ATOM | 375 | N | GLY | A | 49 | -15.511 | -18.908 | 17.511 | 1.00 | 33.42 | N |
| ATOM | 376 | CA | GLY | A | 49 | -15.128 | -20.234 | 17.955 | 1.00 | 33.05 | C |
| ATOM | 377 | C | GLY | A | 49 | -13.878 | -20.244 | 18.806 | 1.00 | 34.29 | C |
| ATOM | 378 | O | GLY | A | 49 | -13.256 | -19.202 | 19.031 | 1.00 | 34.24 | O |
| ATOM | 379 | N | TRP | A | 50 | -13.509 | -21.429 | 19.279 | 1.00 | 30.14 | N |
| ATOM | 380 | CA | TRP | A | 50 | -12.368 | -21.562 | 20.176 | 1.00 | 27.60 | C |
| ATOM | 381 | C | TRP | A | 50 | -11.393 | -22.619 | 19.679 | 1.00 | 36.00 | C |
| ATOM | 382 | O | TRP | A | 50 | -11.758 | -23.513 | 18.915 | 1.00 | 26.22 | O |
| ATOM | 383 | CB | TRP | A | 50 | -12.833 | -21.906 | 21.595 | 1.00 | 27.32 | C |
| ATOM | 384 | CG | TRP | A | 50 | -13.525 | -23.240 | 21.714 | 1.00 | 31.33 | C |
| ATOM | 385 | CD1 | TRP | A | 50 | -14.867 | -23.479 | 21.627 | 1.00 | 31.09 | C |
| ATOM | 386 | CD2 | TRP | A | 50 | -12.907 | -24.513 | 21.952 | 1.00 | 32.23 | C |
| ATOM | 387 | CE2 | TRP | A | 50 | -13.936 | -25.476 | 21.994 | 1.00 | 36.04 | C |
| ATOM | 388 | CE3 | TRP | A | 50 | -11.583 | -24.932 | 22.133 | 1.00 | 35.42 | C |
| ATOM | 389 | NE1 | TRP | A | 50 | -15.122 | -24.819 | 21.791 | 1.00 | 30.64 | N |
| ATOM | 390 | CZ2 | TRP | A | 50 | -13.684 | -26.834 | 22.208 | 1.00 | 34.17 | C |
| ATOM | 391 | CZ3 | TRP | A | 50 | -11.334 | -26.282 | 22.347 | 1.00 | 33.78 | C |
| ATOM | 392 | CH2 | TRP | A | 50 | -12.379 | -27.215 | 22.381 | 1.00 | 34.68 | C |
| ATOM | 393 | N | MET | A | 51 | -10.147 | -22.507 | 20.120 | 1.00 | 29.16 | N |
| ATOM | 394 | CA | MET | A | 51 | -9.148 | -23.507 | 19.807 | 1.00 | 28.07 | C |
| ATOM | 395 | C | MET | A | 51 | -8.191 | -23.679 | 20.975 | 1.00 | 31.55 | C |
| ATOM | 396 | O | MET | A | 51 | -7.728 | -22.696 | 21.553 | 1.00 | 33.21 | O |
| ATOM | 397 | CB | MET | A | 51 | -8.374 | -23.130 | 18.549 | 1.00 | 31.45 | C |
| ATOM | 398 | CG | MET | A | 51 | -7.420 | -24.221 | 18.093 | 1.00 | 37.56 | C |
| ATOM | 399 | SD | MET | A | 51 | -5.818 | -23.586 | 17.594 | 1.00 | 57.24 | S |
| ATOM | 400 | CE | MET | A | 51 | -5.222 | -22.920 | 19.142 | 1.00 | 46.36 | C |
| ATOM | 401 | N | HIS | A | 52 | -7.902 | -24.935 | 21.305 | 1.00 | 27.88 | N |
| ATOM | 402 | CA | HIS | A | 52 | -6.991 | -25.294 | 22.391 | 1.00 | 35.15 | C |
| ATOM | 403 | C | HIS | A | 52 | -5.615 | -25.621 | 21.816 | 1.00 | 35.88 | C |
| ATOM | 404 | O | HIS | A | 52 | -5.449 | -26.650 | 21.169 | 1.00 | 39.53 | O |
| ATOM | 405 | CB | HIS | A | 52 | -7.557 | -26.491 | 23.173 | 1.00 | 31.19 | C |
| ATOM | 406 | CG | HIS | A | 52 | -6.737 | -26.899 | 24.362 | 1.00 | 32.88 | C |
| ATOM | 407 | CD2 | HIS | A | 52 | -6.353 | -26.213 | 25.462 | 1.00 | 35.83 | C |
| ATOM | 408 | ND1 | HIS | A | 52 | -6.248 | -28.180 | 24.520 | 1.00 | 33.47 | N |
| ATOM | 409 | CE1 | HIS | A | 52 | -5.585 | -28.258 | 25.660 | 1.00 | 32.93 | C |
| ATOM | 410 | NE2 | HIS | A | 52 | -5.631 | -27.080 | 26.252 | 1.00 | 33.96 | N |
| ATOM | 411 | N | PRO | A | 53 | -4.629 | -24.737 | 22.035 | 1.00 | 36.74 | N |
| ATOM | 412 | CA | PRO | A | 53 | -3.284 | -24.901 | 21.465 | 1.00 | 41.48 | C |
| ATOM | 413 | C | PRO | A | 53 | -2.619 | -26.232 | 21.802 | 1.00 | 43.82 | C |
| ATOM | 414 | O | PRO | A | 53 | -1.959 | -26.811 | 20.941 | 1.00 | 42.27 | O |
| ATOM | 415 | CB | PRO | A | 53 | -2.497 | -23.744 | 22.088 | 1.00 | 37.52 | C |
| ATOM | 416 | CG | PRO | A | 53 | -3.521 | -22.710 | 22.372 | 1.00 | 42.21 | C |
| ATOM | 417 | CD | PRO | A | 53 | -4.768 | -23.456 | 22.751 | 1.00 | 37.85 | C |
| ATOM | 418 | N | ASN | A | 54 | -2.795 | -26.709 | 23.029 | 1.00 | 40.74 | N |
| ATOM | 419 | CA | ASN | A | 54 | -2.088 | -27.903 | 23.482 | 1.00 | 40.42 | C |
| ATOM | 420 | C | ASN | A | 54 | -2.575 | -29.192 | 22.814 | 1.00 | 44.96 | C |
| ATOM | 421 | O | ASN | A | 54 | -1.848 | -30.184 | 22.781 | 1.00 | 53.00 | O |
| ATOM | 422 | CB | ASN | A | 54 | -2.201 | -28.035 | 25.003 | 1.00 | 41.59 | C |
| ATOM | 423 | CG | ASN | A | 54 | -0.988 | -28.704 | 25.620 | 1.00 | 48.60 | C |
| ATOM | 424 | ND2 | ASN | A | 54 | -1.220 | -29.565 | 26.607 | 1.00 | 49.53 | N |

```
ATOM    425  OD1 ASN A  54     0.146 -28.456  25.208  1.00 54.90           O
ATOM    426  N   SER A  55    -3.792 -29.179  22.276  1.00 41.51           N
ATOM    427  CA  SER A  55    -4.354 -30.372  21.641  1.00 39.42           C
ATOM    428  C   SER A  55    -4.800 -30.137  20.196  1.00 45.89           C
ATOM    429  O   SER A  55    -4.958 -31.083  19.428  1.00 45.29           O
ATOM    430  CB  SER A  55    -5.544 -30.886  22.444  1.00 40.90           C
ATOM    431  OG  SER A  55    -6.661 -30.030  22.269  1.00 41.20           O
ATOM    432  N   GLY A  56    -5.020 -28.878  19.831  1.00 39.03           N
ATOM    433  CA  GLY A  56    -5.491 -28.550  18.497  1.00 34.31           C
ATOM    434  C   GLY A  56    -6.992 -28.707  18.348  1.00 33.47           C
ATOM    435  O   GLY A  56    -7.551 -28.402  17.298  1.00 35.63           O
ATOM    436  N   ASN A  57    -7.649 -29.185  19.401  1.00 32.07           N
ATOM    437  CA  ASN A  57    -9.099 -29.323  19.384  1.00 35.35           C
ATOM    438  C   ASN A  57    -9.792 -27.972  19.257  1.00 35.99           C
ATOM    439  O   ASN A  57    -9.298 -26.956  19.753  1.00 31.96           O
ATOM    440  CB  ASN A  57    -9.585 -30.042  20.638  1.00 36.70           C
ATOM    441  CG  ASN A  57    -9.149 -31.490  20.679  1.00 51.73           C
ATOM    442  OD1 ASN A  57    -8.193 -31.882  20.005  1.00 51.72           O
ATOM    443  ND2 ASN A  57    -9.851 -32.297  21.467  1.00 40.02           N
ATOM    444  N   THR A  58   -10.938 -27.966  18.585  1.00 32.70           N
ATOM    445  CA  THR A  58   -11.657 -26.728  18.315  1.00 34.35           C
ATOM    446  C   THR A  58   -13.149 -26.874  18.543  1.00 36.07           C
ATOM    447  O   THR A  58   -13.664 -27.985  18.682  1.00 35.59           O
ATOM    448  CB  THR A  58   -11.463 -26.251  16.859  1.00 35.50           C
ATOM    449  CG2 THR A  58    -9.986 -26.209  16.474  1.00 29.82           C
ATOM    450  OG1 THR A  58   -12.167 -27.132  15.976  1.00 34.89           O
ATOM    451  N   GLY A  59   -13.833 -25.735  18.563  1.00 34.36           N
ATOM    452  CA  GLY A  59   -15.283 -25.682  18.542  1.00 31.26           C
ATOM    453  C   GLY A  59   -15.712 -24.404  17.843  1.00 36.02           C
ATOM    454  O   GLY A  59   -15.268 -23.319  18.214  1.00 34.77           O
ATOM    455  N   TYR A  60   -16.561 -24.528  16.826  1.00 38.85           N
ATOM    456  CA  TYR A  60   -17.004 -23.374  16.045  1.00 36.79           C
ATOM    457  C   TYR A  60   -18.467 -23.041  16.310  1.00 43.76           C
ATOM    458  O   TYR A  60   -19.269 -23.933  16.581  1.00 40.31           O
ATOM    459  CB  TYR A  60   -16.818 -23.630  14.546  1.00 40.99           C
ATOM    460  CG  TYR A  60   -15.446 -24.123  14.157  1.00 40.11           C
ATOM    461  CD1 TYR A  60   -14.305 -23.583  14.729  1.00 37.87           C
ATOM    462  CD2 TYR A  60   -15.292 -25.132  13.213  1.00 40.09           C
ATOM    463  CE1 TYR A  60   -13.050 -24.031  14.374  1.00 39.91           C
ATOM    464  CE2 TYR A  60   -14.037 -25.587  12.851  1.00 40.87           C
ATOM    465  CZ  TYR A  60   -12.920 -25.030  13.436  1.00 42.40           C
ATOM    466  OH  TYR A  60   -11.666 -25.467  13.091  1.00 41.60           O
ATOM    467  N   ALA A  61   -18.816 -21.760  16.223  1.00 32.46           N
ATOM    468  CA  ALA A  61   -20.219 -21.370  16.223  1.00 39.25           C
ATOM    469  C   ALA A  61   -20.873 -21.880  14.940  1.00 40.91           C
ATOM    470  O   ALA A  61   -20.228 -21.916  13.891  1.00 43.24           O
ATOM    471  CB  ALA A  61   -20.363 -19.856  16.343  1.00 34.62           C
ATOM    472  N   GLN A  62   -22.140 -22.280  15.030  1.00 45.76           N
ATOM    473  CA  GLN A  62   -22.878 -22.825  13.887  1.00 53.88           C
ATOM    474  C   GLN A  62   -22.795 -21.937  12.646  1.00 47.20           C
ATOM    475  O   GLN A  62   -22.716 -22.431  11.522  1.00 48.71           O
ATOM    476  CB  GLN A  62   -24.349 -23.042  14.258  1.00 52.36           C
ATOM    477  CG  GLN A  62   -24.596 -24.209  15.206  1.00 79.57           C
ATOM    478  CD  GLN A  62   -24.422 -25.562  14.534  1.00 86.89           C
ATOM    479  NE2 GLN A  62   -24.222 -26.603  15.339  1.00 75.39           N
ATOM    480  OE1 GLN A  62   -24.468 -25.670  13.306  1.00 81.01           O
ATOM    481  N   LYS A  63   -22.804 -20.626  12.865  1.00 48.15           N
ATOM    482  CA  LYS A  63   -22.788 -19.644  11.787  1.00 42.61           C
ATOM    483  C   LYS A  63   -21.524 -19.723  10.919  1.00 43.90           C
ATOM    484  O   LYS A  63   -21.540 -19.325   9.756  1.00 41.77           O
ATOM    485  CB  LYS A  63   -22.936 -18.235  12.377  1.00 45.82           C
ATOM    486  CG  LYS A  63   -23.079 -17.120  11.353  1.00 50.01           C
ATOM    487  CD  LYS A  63   -23.154 -15.753  12.024  1.00 52.82           C
ATOM    488  CE  LYS A  63   -23.046 -14.631  10.996  1.00 50.15           C
ATOM    489  NZ  LYS A  63   -23.127 -13.283  11.624  1.00 61.30           N
ATOM    490  N   PHE A  64   -20.435 -20.246  11.478  1.00 41.78           N
ATOM    491  CA  PHE A  64   -19.156 -20.267  10.768  1.00 39.68           C
ATOM    492  C   PHE A  64   -18.656 -21.675  10.456  1.00 44.13           C
ATOM    493  O   PHE A  64   -17.611 -21.836   9.821  1.00 46.68           O
ATOM    494  CB  PHE A  64   -18.094 -19.513  11.574  1.00 35.08           C
ATOM    495  CG  PHE A  64   -18.454 -18.086  11.848  1.00 41.60           C
```

| ATOM | 496 | CD1 | PHE | A | 64 | -18.254 | -17.109 | 10.885 | 1.00 | 42.81 | C |
|------|-----|-----|-----|---|----|---------|---------|--------|------|-------|---|
| ATOM | 497 | CD2 | PHE | A | 64 | -19.008 | -17.720 | 13.062 | 1.00 | 34.34 | C |
| ATOM | 498 | CE1 | PHE | A | 64 | -18.593 | -15.790 | 11.135 | 1.00 | 34.56 | C |
| ATOM | 499 | CE2 | PHE | A | 64 | -19.350 | -16.405 | 13.313 | 1.00 | 40.22 | C |
| ATOM | 500 | CZ | PHE | A | 64 | -19.142 | -15.441 | 12.348 | 1.00 | 32.48 | C |
| ATOM | 501 | N | GLN | A | 65 | -19.389 | -22.689 | 10.907 | 1.00 | 49.73 | N |
| ATOM | 502 | CA | GLN | A | 65 | -19.066 | -24.069 | 10.552 | 1.00 | 46.28 | C |
| ATOM | 503 | C | GLN | A | 65 | -19.116 | -24.231 | 9.040 | 1.00 | 44.61 | C |
| ATOM | 504 | O | GLN | A | 65 | -20.120 | -23.905 | 8.408 | 1.00 | 45.36 | O |
| ATOM | 505 | CB | GLN | A | 65 | -20.024 | -25.051 | 11.229 | 1.00 | 47.60 | C |
| ATOM | 506 | CG | GLN | A | 65 | -19.573 | -25.483 | 12.619 | 1.00 | 59.40 | C |
| ATOM | 507 | CD | GLN | A | 65 | -20.692 | -26.096 | 13.442 | 1.00 | 69.19 | C |
| ATOM | 508 | NE2 | GLN | A | 65 | -20.460 | -26.234 | 14.745 | 1.00 | 55.87 | N |
| ATOM | 509 | OE1 | GLN | A | 65 | -21.754 | -26.434 | 12.916 | 1.00 | 71.81 | O |
| ATOM | 510 | N | GLY | A | 66 | -18.019 | -24.711 | 8.464 | 1.00 | 39.34 | N |
| ATOM | 511 | CA | GLY | A | 66 | -17.919 | -24.856 | 7.026 | 1.00 | 46.10 | C |
| ATOM | 512 | C | GLY | A | 66 | -17.130 | -23.748 | 6.349 | 1.00 | 56.88 | C |
| ATOM | 513 | O | GLY | A | 66 | -16.677 | -23.912 | 5.214 | 1.00 | 58.19 | O |
| ATOM | 514 | N | ARG | A | 67 | -16.966 | -22.617 | 7.031 | 1.00 | 44.44 | N |
| ATOM | 515 | CA | ARG | A | 67 | -16.192 | -21.511 | 6.470 | 1.00 | 43.73 | C |
| ATOM | 516 | C | ARG | A | 67 | -14.935 | -21.213 | 7.280 | 1.00 | 45.38 | C |
| ATOM | 517 | O | ARG | A | 67 | -13.937 | -20.745 | 6.732 | 1.00 | 45.99 | O |
| ATOM | 518 | CB | ARG | A | 67 | -17.045 | -20.244 | 6.368 | 1.00 | 47.99 | C |
| ATOM | 519 | CG | ARG | A | 67 | -18.207 | -20.359 | 5.406 | 1.00 | 47.03 | C |
| ATOM | 520 | CD | ARG | A | 67 | -18.570 | -19.011 | 4.793 | 1.00 | 47.09 | C |
| ATOM | 521 | NE | ARG | A | 67 | -19.006 | -18.032 | 5.783 | 1.00 | 50.09 | N |
| ATOM | 522 | CZ | ARG | A | 67 | -18.526 | -16.794 | 5.866 | 1.00 | 49.71 | C |
| ATOM | 523 | NH1 | ARG | A | 67 | -17.590 | -16.383 | 5.020 | 1.00 | 46.73 | N |
| ATOM | 524 | NH2 | ARG | A | 67 | -18.983 | -15.964 | 6.794 | 1.00 | 47.52 | N |
| ATOM | 525 | N | VAL | A | 68 | -14.979 | -21.480 | 8.582 | 1.00 | 39.50 | N |
| ATOM | 526 | CA | VAL | A | 68 | -13.848 | -21.157 | 9.443 | 1.00 | 35.99 | C |
| ATOM | 527 | C | VAL | A | 68 | -12.994 | -22.390 | 9.737 | 1.00 | 39.26 | C |
| ATOM | 528 | O | VAL | A | 68 | -13.491 | -23.514 | 9.795 | 1.00 | 40.40 | O |
| ATOM | 529 | CB | VAL | A | 68 | -14.314 | -20.511 | 10.777 | 1.00 | 43.31 | C |
| ATOM | 530 | CG1 | VAL | A | 68 | -14.925 | -21.554 | 11.709 | 1.00 | 44.00 | C |
| ATOM | 531 | CG2 | VAL | A | 68 | -13.155 | -19.793 | 11.461 | 1.00 | 36.66 | C |
| ATOM | 532 | N | THR | A | 69 | -11.694 | -22.167 | 9.893 | 1.00 | 34.83 | N |
| ATOM | 533 | CA | THR | A | 69 | -10.772 | -23.220 | 10.282 | 1.00 | 37.94 | C |
| ATOM | 534 | C | THR | A | 69 | -9.749 | -22.651 | 11.247 | 1.00 | 36.48 | C |
| ATOM | 535 | O | THR | A | 69 | -9.020 | -21.722 | 10.905 | 1.00 | 39.14 | O |
| ATOM | 536 | CB | THR | A | 69 | -10.049 | -23.833 | 9.069 | 1.00 | 38.15 | C |
| ATOM | 537 | CG2 | THR | A | 69 | -9.167 | -24.998 | 9.508 | 1.00 | 36.65 | C |
| ATOM | 538 | OG1 | THR | A | 69 | -11.015 | -24.297 | 8.120 | 1.00 | 40.71 | O |
| ATOM | 539 | N | LEU | A | 70 | -9.705 | -23.202 | 12.454 | 1.00 | 32.50 | N |
| ATOM | 540 | CA | LEU | A | 70 | -8.759 | -22.746 | 13.463 | 1.00 | 32.88 | C |
| ATOM | 541 | C | LEU | A | 70 | -7.647 | -23.769 | 13.633 | 1.00 | 41.47 | C |
| ATOM | 542 | O | LEU | A | 70 | -7.911 | -24.942 | 13.900 | 1.00 | 41.36 | O |
| ATOM | 543 | CB | LEU | A | 70 | -9.465 | -22.501 | 14.797 | 1.00 | 32.92 | C |
| ATOM | 544 | CG | LEU | A | 70 | -10.682 | -21.573 | 14.763 | 1.00 | 41.37 | C |
| ATOM | 545 | CD1 | LEU | A | 70 | -11.203 | -21.301 | 16.178 | 1.00 | 36.02 | C |
| ATOM | 546 | CD2 | LEU | A | 70 | -10.360 | -20.271 | 14.047 | 1.00 | 32.82 | C |
| ATOM | 547 | N | THR | A | 71 | -6.405 | -23.324 | 13.465 | 1.00 | 33.46 | N |
| ATOM | 548 | CA | THR | A | 71 | -5.250 | -24.204 | 13.587 | 1.00 | 35.36 | C |
| ATOM | 549 | C | THR | A | 71 | -4.166 | -23.523 | 14.409 | 1.00 | 39.68 | C |
| ATOM | 550 | O | THR | A | 71 | -4.299 | -22.357 | 14.775 | 1.00 | 38.29 | O |
| ATOM | 551 | CB | THR | A | 71 | -4.684 | -24.607 | 12.203 | 1.00 | 40.98 | C |
| ATOM | 552 | CG2 | THR | A | 71 | -5.679 | -25.475 | 11.451 | 1.00 | 37.98 | C |
| ATOM | 553 | OG1 | THR | A | 71 | -4.413 | -23.432 | 11.427 | 1.00 | 44.28 | O |
| ATOM | 554 | N | ARG | A | 72 | -3.097 | -24.250 | 14.713 | 1.00 | 41.13 | N |
| ATOM | 555 | CA | ARG | A | 72 | -1.996 | -23.659 | 15.460 | 1.00 | 41.98 | C |
| ATOM | 556 | C | ARG | A | 72 | -0.644 | -24.252 | 15.077 | 1.00 | 44.70 | C |
| ATOM | 557 | O | ARG | A | 72 | -0.566 | -25.292 | 14.429 | 1.00 | 41.56 | O |
| ATOM | 558 | CB | ARG | A | 72 | -2.231 | -23.811 | 16.970 | 1.00 | 44.74 | C |
| ATOM | 559 | CG | ARG | A | 72 | -2.599 | -25.216 | 17.439 | 1.00 | 46.36 | C |
| ATOM | 560 | CD | ARG | A | 72 | -1.366 | -26.056 | 17.691 | 1.00 | 52.55 | C |
| ATOM | 561 | NE | ARG | A | 72 | -1.653 | -27.299 | 18.401 | 1.00 | 45.44 | N |
| ATOM | 562 | CZ | ARG | A | 72 | -2.105 | -28.406 | 17.824 | 1.00 | 51.24 | C |
| ATOM | 563 | NH1 | ARG | A | 72 | -2.353 | -28.429 | 16.522 | 1.00 | 53.24 | N |
| ATOM | 564 | NH2 | ARG | A | 72 | -2.319 | -29.492 | 18.553 | 1.00 | 55.87 | N |
| ATOM | 565 | N | ASP | A | 73 | 0.413 | -23.563 | 15.488 | 1.00 | 44.00 | N |
| ATOM | 566 | CA | ASP | A | 73 | 1.779 | -24.033 | 15.331 | 1.00 | 45.95 | C |

| ATOM | 567 | C | ASP | A | 73 | 2.510 | -23.807 | 16.652 | 1.00 | 45.15 | C |
|---|---|---|---|---|---|---|---|---|---|---|---|
| ATOM | 568 | O | ASP | A | 73 | 3.010 | -22.710 | 16.916 | 1.00 | 46.41 | O |
| ATOM | 569 | CB | ASP | A | 73 | 2.473 | -23.305 | 14.175 | 1.00 | 52.61 | C |
| ATOM | 570 | CG | ASP | A | 73 | 3.922 | -23.728 | 13.995 | 1.00 | 58.41 | C |
| ATOM | 571 | OD1 | ASP | A | 73 | 4.345 | -24.730 | 14.609 | 1.00 | 57.08 | O |
| ATOM | 572 | OD2 | ASP | A | 73 | 4.639 | -23.059 | 13.220 | 1.00 | 60.43 | O |
| ATOM | 573 | N | THR | A | 74 | 2.560 | -24.847 | 17.480 | 1.00 | 46.17 | N |
| ATOM | 574 | CA | THR | A | 74 | 3.082 | -24.726 | 18.841 | 1.00 | 48.47 | C |
| ATOM | 575 | C | THR | A | 74 | 4.559 | -24.346 | 18.884 | 1.00 | 46.89 | C |
| ATOM | 576 | O | THR | A | 74 | 5.012 | -23.723 | 19.842 | 1.00 | 53.90 | O |
| ATOM | 577 | CB | THR | A | 74 | 2.888 | -26.035 | 19.636 | 1.00 | 54.20 | C |
| ATOM | 578 | CG2 | THR | A | 74 | 1.439 | -26.185 | 20.072 | 1.00 | 48.42 | C |
| ATOM | 579 | OG1 | THR | A | 74 | 3.250 | -27.155 | 18.817 | 1.00 | 60.06 | O |
| ATOM | 580 | N | SER | A | 75 | 5.303 | -24.710 | 17.845 | 1.00 | 51.70 | N |
| ATOM | 581 | CA | SER | A | 75 | 6.737 | -24.436 | 17.801 | 1.00 | 54.91 | C |
| ATOM | 582 | C | SER | A | 75 | 7.036 | -22.937 | 17.815 | 1.00 | 55.26 | C |
| ATOM | 583 | O | SER | A | 75 | 8.114 | -22.515 | 18.237 | 1.00 | 49.86 | O |
| ATOM | 584 | CB | SER | A | 75 | 7.365 | -25.088 | 16.568 | 1.00 | 55.81 | C |
| ATOM | 585 | OG | SER | A | 75 | 6.713 | -24.668 | 15.382 | 1.00 | 60.43 | O |
| ATOM | 586 | N | ILE | A | 76 | 6.077 | -22.136 | 17.359 | 1.00 | 52.63 | N |
| ATOM | 587 | CA | ILE | A | 76 | 6.224 | -20.684 | 17.386 | 1.00 | 47.56 | C |
| ATOM | 588 | C | ILE | A | 76 | 5.090 | -20.021 | 18.170 | 1.00 | 50.56 | C |
| ATOM | 589 | O | ILE | A | 76 | 4.920 | -18.802 | 18.109 | 1.00 | 52.59 | O |
| ATOM | 590 | CB | ILE | A | 76 | 6.271 | -20.093 | 15.962 | 1.00 | 50.46 | C |
| ATOM | 591 | CG1 | ILE | A | 76 | 5.040 | -20.521 | 15.163 | 1.00 | 47.49 | C |
| ATOM | 592 | CG2 | ILE | A | 76 | 7.536 | -20.531 | 15.248 | 1.00 | 46.69 | C |
| ATOM | 593 | CD1 | ILE | A | 76 | 4.922 | -19.840 | 13.817 | 1.00 | 48.75 | C |
| ATOM | 594 | N | SER | A | 77 | 4.324 | -20.834 | 18.896 | 1.00 | 49.81 | N |
| ATOM | 595 | CA | SER | A | 77 | 3.229 | -20.357 | 19.744 | 1.00 | 45.00 | C |
| ATOM | 596 | C | SER | A | 77 | 2.246 | -19.470 | 18.988 | 1.00 | 46.19 | C |
| ATOM | 597 | O | SER | A | 77 | 1.894 | -18.387 | 19.456 | 1.00 | 40.30 | O |
| ATOM | 598 | CB | SER | A | 77 | 3.781 | -19.591 | 20.951 | 1.00 | 43.85 | C |
| ATOM | 599 | OG | SER | A | 77 | 4.716 | -20.370 | 21.673 | 1.00 | 63.87 | O |
| ATOM | 600 | N | THR | A | 78 | 1.800 | -19.925 | 17.822 | 1.00 | 39.04 | N |
| ATOM | 601 | CA | THR | A | 78 | 0.956 | -19.090 | 16.979 | 1.00 | 37.69 | C |
| ATOM | 602 | C | THR | A | 78 | -0.337 | -19.788 | 16.575 | 1.00 | 37.97 | C |
| ATOM | 603 | O | THR | A | 78 | -0.325 | -20.930 | 16.115 | 1.00 | 39.48 | O |
| ATOM | 604 | CB | THR | A | 78 | 1.718 | -18.641 | 15.712 | 1.00 | 38.64 | C |
| ATOM | 605 | CG2 | THR | A | 78 | 0.810 | -17.840 | 14.787 | 1.00 | 38.64 | C |
| ATOM | 606 | OG1 | THR | A | 78 | 2.828 | -17.822 | 16.094 | 1.00 | 35.01 | O |
| ATOM | 607 | N | ALA | A | 79 | -1.454 | -19.093 | 16.765 | 1.00 | 33.81 | N |
| ATOM | 608 | CA | ALA | A | 79 | -2.756 | -19.592 | 16.336 | 1.00 | 34.16 | C |
| ATOM | 609 | C | ALA | A | 79 | -3.165 | -18.934 | 15.023 | 1.00 | 34.47 | C |
| ATOM | 610 | O | ALA | A | 79 | -2.748 | -17.815 | 14.720 | 1.00 | 33.95 | O |
| ATOM | 611 | CB | ALA | A | 79 | -3.805 | -19.340 | 17.403 | 1.00 | 24.34 | C |
| ATOM | 612 | N | TYR | A | 80 | -3.990 | -19.631 | 14.252 | 1.00 | 31.73 | N |
| ATOM | 613 | CA | TYR | A | 80 | -4.432 | -19.135 | 12.958 | 1.00 | 37.57 | C |
| ATOM | 614 | C | TYR | A | 80 | -5.942 | -19.198 | 12.810 | 1.00 | 39.18 | C |
| ATOM | 615 | O | TYR | A | 80 | -6.598 | -20.132 | 13.278 | 1.00 | 37.02 | O |
| ATOM | 616 | CB | TYR | A | 80 | -3.786 | -19.929 | 11.819 | 1.00 | 36.79 | C |
| ATOM | 617 | CG | TYR | A | 80 | -2.281 | -19.862 | 11.797 | 1.00 | 40.36 | C |
| ATOM | 618 | CD1 | TYR | A | 80 | -1.621 | -18.821 | 11.158 | 1.00 | 37.20 | C |
| ATOM | 619 | CD2 | TYR | A | 80 | -1.517 | -20.847 | 12.413 | 1.00 | 38.74 | C |
| ATOM | 620 | CE1 | TYR | A | 80 | -0.238 | -18.761 | 11.135 | 1.00 | 37.53 | C |
| ATOM | 621 | CE2 | TYR | A | 80 | -0.140 | -20.797 | 12.395 | 1.00 | 37.97 | C |
| ATOM | 622 | CZ | TYR | A | 80 | 0.497 | -19.755 | 11.756 | 1.00 | 43.48 | C |
| ATOM | 623 | OH | TYR | A | 80 | 1.872 | -19.708 | 11.742 | 1.00 | 41.36 | O |
| ATOM | 624 | N | MET | A | 81 | -6.486 | -18.195 | 12.141 | 1.00 | 31.97 | N |
| ATOM | 625 | CA | MET | A | 81 | -7.895 | -18.185 | 11.803 | 1.00 | 37.08 | C |
| ATOM | 626 | C | MET | A | 81 | -7.999 | -18.062 | 10.297 | 1.00 | 34.14 | C |
| ATOM | 627 | O | MET | A | 81 | -7.505 | -17.096 | 9.717 | 1.00 | 38.01 | O |
| ATOM | 628 | CB | MET | A | 81 | -8.619 | -17.036 | 12.509 | 1.00 | 39.39 | C |
| ATOM | 629 | CG | MET | A | 81 | -10.137 | -17.099 | 12.445 | 1.00 | 43.17 | C |
| ATOM | 630 | SD | MET | A | 81 | -10.835 | -16.468 | 10.907 | 1.00 | 56.54 | S |
| ATOM | 631 | CE | MET | A | 81 | -10.224 | -14.782 | 10.930 | 1.00 | 40.20 | C |
| ATOM | 632 | N | GLU | A | 82 | -8.606 | -19.054 | 9.659 | 1.00 | 36.27 | N |
| ATOM | 633 | CA | GLU | A | 82 | -8.825 | -18.989 | 8.222 | 1.00 | 37.25 | C |
| ATOM | 634 | C | GLU | A | 82 | -10.310 | -18.963 | 7.908 | 1.00 | 38.89 | C |
| ATOM | 635 | O | GLU | A | 82 | -11.064 | -19.831 | 8.343 | 1.00 | 42.10 | O |
| ATOM | 636 | CB | GLU | A | 82 | -8.159 | -20.161 | 7.504 | 1.00 | 34.97 | C |
| ATOM | 637 | CG | GLU | A | 82 | -8.406 | -20.149 | 6.003 | 1.00 | 48.29 | C |

| ATOM | 638 | CD | GLU | A | 82 | -7.374 | -20.946 | 5.228 | 1.00 | 61.14 | C |
| ATOM | 639 | OE1 | GLU | A | 82 | -6.617 | -21.717 | 5.858 | 1.00 | 70.53 | O |
| ATOM | 640 | OE2 | GLU | A | 82 | -7.313 | -20.793 | 3.989 | 1.00 | 62.98 | O |
| ATOM | 641 | N | LEU | A | 83 | -10.726 | -17.946 | 7.161 | 1.00 | 41.14 | N |
| ATOM | 642 | CA | LEU | A | 83 | -12.118 | -17.807 | 6.766 | 1.00 | 40.75 | C |
| ATOM | 643 | C | LEU | A | 83 | -12.204 | -17.828 | 5.246 | 1.00 | 42.77 | C |
| ATOM | 644 | O | LEU | A | 83 | -11.572 | -17.017 | 4.569 | 1.00 | 46.85 | O |
| ATOM | 645 | CB | LEU | A | 83 | -12.716 | -16.519 | 7.336 | 1.00 | 38.47 | C |
| ATOM | 646 | CG | LEU | A | 83 | -14.233 | -16.359 | 7.216 | 1.00 | 41.96 | C |
| ATOM | 647 | CD1 | LEU | A | 83 | -14.956 | -17.499 | 7.919 | 1.00 | 40.57 | C |
| ATOM | 648 | CD2 | LEU | A | 83 | -14.677 | -15.015 | 7.776 | 1.00 | 38.68 | C |
| ATOM | 649 | N | SER | A | 84 | -12.974 | -18.769 | 4.711 | 1.00 | 41.64 | N |
| ATOM | 650 | CA | SER | A | 84 | -13.079 | -18.936 | 3.267 | 1.00 | 42.87 | C |
| ATOM | 651 | C | SER | A | 84 | -14.426 | -18.442 | 2.740 | 1.00 | 44.92 | C |
| ATOM | 652 | O | SER | A | 84 | -15.304 | -18.067 | 3.522 | 1.00 | 44.37 | O |
| ATOM | 653 | CB | SER | A | 84 | -12.868 | -20.402 | 2.888 | 1.00 | 39.46 | C |
| ATOM | 654 | OG | SER | A | 84 | -13.903 | -21.211 | 3.416 | 1.00 | 44.27 | O |
| ATOM | 655 | N | SER | A | 85 | -14.572 | -18.454 | 1.414 | 1.00 | 47.43 | N |
| ATOM | 656 | CA | SER | A | 85 | -15.769 | -17.960 | 0.732 | 1.00 | 41.86 | C |
| ATOM | 657 | C | SER | A | 85 | -16.189 | -16.591 | 1.245 | 1.00 | 43.45 | C |
| ATOM | 658 | O | SER | A | 85 | -17.337 | -16.392 | 1.652 | 1.00 | 44.70 | O |
| ATOM | 659 | CB | SER | A | 85 | -16.922 | -18.952 | 0.882 | 1.00 | 45.07 | C |
| ATOM | 660 | OG | SER | A | 85 | -16.674 | -20.125 | 0.128 | 1.00 | 51.48 | O |
| ATOM | 661 | N | LEU | A | 86 | -15.249 | -15.650 | 1.223 | 1.00 | 43.44 | N |
| ATOM | 662 | CA | LEU | A | 86 | -15.457 | -14.338 | 1.826 | 1.00 | 41.57 | C |
| ATOM | 663 | C | LEU | A | 86 | -16.526 | -13.526 | 1.104 | 1.00 | 46.59 | C |
| ATOM | 664 | O | LEU | A | 86 | -16.551 | -13.456 | -0.128 | 1.00 | 40.39 | O |
| ATOM | 665 | CB | LEU | A | 86 | -14.142 | -13.557 | 1.858 | 1.00 | 40.33 | C |
| ATOM | 666 | CG | LEU | A | 86 | -13.076 | -14.119 | 2.800 | 1.00 | 41.08 | C |
| ATOM | 667 | CD1 | LEU | A | 86 | -11.732 | -13.444 | 2.573 | 1.00 | 39.92 | C |
| ATOM | 668 | CD2 | LEU | A | 86 | -13.527 | -13.966 | 4.241 | 1.00 | 33.71 | C |
| ATOM | 669 | N | ARG | A | 87 | -17.420 | -12.933 | 1.888 | 1.00 | 37.30 | N |
| ATOM | 670 | CA | ARG | A | 87 | -18.411 | -12.002 | 1.371 | 1.00 | 42.71 | C |
| ATOM | 671 | C | ARG | A | 87 | -18.086 | -10.616 | 1.905 | 1.00 | 44.74 | C |
| ATOM | 672 | O | ARG | A | 87 | -17.304 | -10.483 | 2.846 | 1.00 | 42.11 | O |
| ATOM | 673 | CB | ARG | A | 87 | -19.828 | -12.418 | 1.774 | 1.00 | 47.73 | C |
| ATOM | 674 | CG | ARG | A | 87 | -20.231 | -13.806 | 1.298 | 1.00 | 57.87 | C |
| ATOM | 675 | CD | ARG | A | 87 | -21.702 | -14.088 | 1.577 | 1.00 | 78.50 | C |
| ATOM | 676 | NE | ARG | A | 87 | -22.154 | -15.318 | 0.928 | 1.00 | 98.72 | N |
| ATOM | 677 | CZ | ARG | A | 87 | -23.391 | -15.800 | 1.009 | 1.00 | 102.44 | C |
| ATOM | 678 | NH1 | ARG | A | 87 | -24.311 | -15.158 | 1.716 | 1.00 | 100.96 | N |
| ATOM | 679 | NH2 | ARG | A | 87 | -23.708 | -16.927 | 0.384 | 1.00 | 94.73 | N |
| ATOM | 680 | N | SER | A | 88 | -18.682 | -9.586 | 1.315 | 1.00 | 48.46 | N |
| ATOM | 681 | CA | SER | A | 88 | -18.426 | -8.221 | 1.759 | 1.00 | 47.83 | C |
| ATOM | 682 | C | SER | A | 88 | -18.871 | -8.035 | 3.212 | 1.00 | 43.27 | C |
| ATOM | 683 | O | SER | A | 88 | -18.315 | -7.210 | 3.935 | 1.00 | 45.67 | O |
| ATOM | 684 | CB | SER | A | 88 | -19.126 | -7.209 | 0.843 | 1.00 | 46.62 | C |
| ATOM | 685 | OG | SER | A | 88 | -20.530 | -7.399 | 0.826 | 1.00 | 56.66 | O |
| ATOM | 686 | N | GLU | A | 89 | -19.857 | -8.823 | 3.638 | 1.00 | 43.65 | N |
| ATOM | 687 | CA | GLU | A | 89 | -20.347 | -8.780 | 5.012 | 1.00 | 42.47 | C |
| ATOM | 688 | C | GLU | A | 89 | -19.332 | -9.323 | 6.015 | 1.00 | 42.20 | C |
| ATOM | 689 | O | GLU | A | 89 | -19.508 | -9.163 | 7.224 | 1.00 | 44.31 | O |
| ATOM | 690 | CB | GLU | A | 89 | -21.658 | -9.562 | 5.146 | 1.00 | 48.07 | C |
| ATOM | 691 | CG | GLU | A | 89 | -22.881 | -8.844 | 4.594 | 1.00 | 69.24 | C |
| ATOM | 692 | CD | GLU | A | 89 | -22.970 | -8.904 | 3.079 | 1.00 | 77.53 | C |
| ATOM | 693 | OE1 | GLU | A | 89 | -22.301 | -9.773 | 2.476 | 1.00 | 65.90 | O |
| ATOM | 694 | OE2 | GLU | A | 89 | -23.710 | -8.081 | 2.493 | 1.00 | 76.65 | O |
| ATOM | 695 | N | ASP | A | 90 | -18.279 | -9.969 | 5.519 | 1.00 | 36.36 | N |
| ATOM | 696 | CA | ASP | A | 90 | -17.213 | -10.463 | 6.388 | 1.00 | 34.38 | C |
| ATOM | 697 | C | ASP | A | 90 | -16.197 | -9.364 | 6.709 | 1.00 | 39.43 | C |
| ATOM | 698 | O | ASP | A | 90 | -15.250 | -9.589 | 7.463 | 1.00 | 38.18 | O |
| ATOM | 699 | CB | ASP | A | 90 | -16.494 | -11.658 | 5.751 | 1.00 | 38.88 | C |
| ATOM | 700 | CG | ASP | A | 90 | -17.361 | -12.903 | 5.681 | 1.00 | 42.31 | C |
| ATOM | 701 | OD1 | ASP | A | 90 | -18.157 | -13.146 | 6.616 | 1.00 | 37.56 | O |
| ATOM | 702 | OD2 | ASP | A | 90 | -17.237 | -13.646 | 4.683 | 1.00 | 47.30 | O |
| ATOM | 703 | N | THR | A | 91 | -16.389 | -8.183 | 6.127 | 1.00 | 34.63 | N |
| ATOM | 704 | CA | THR | A | 91 | -15.525 | -7.042 | 6.418 | 1.00 | 35.67 | C |
| ATOM | 705 | C | THR | A | 91 | -15.661 | -6.663 | 7.887 | 1.00 | 34.77 | C |
| ATOM | 706 | O | THR | A | 91 | -16.743 | -6.285 | 8.340 | 1.00 | 34.99 | O |
| ATOM | 707 | CB | THR | A | 91 | -15.858 | -5.818 | 5.532 | 1.00 | 37.50 | C |
| ATOM | 708 | CG2 | THR | A | 91 | -15.075 | -4.591 | 5.994 | 1.00 | 34.40 | C |

| ATOM | 709 | OG1 | THR A | 91 | -15.529 | -6.104 | 4.164 | 1.00 | 40.03 | O |
|------|-----|-----|-------|-----|---------|--------|--------|------|-------|---|
| ATOM | 710 | N | ALA A | 92 | -14.562 | -6.772 | 8.626 | 1.00 | 29.62 | N |
| ATOM | 711 | CA | ALA A | 92 | -14.588 | -6.570 | 10.070 | 1.00 | 32.67 | C |
| ATOM | 712 | C | ALA A | 92 | -13.190 | -6.544 | 10.665 | 1.00 | 27.73 | C |
| ATOM | 713 | O | ALA A | 92 | -12.209 | -6.882 | 10.001 | 1.00 | 29.29 | O |
| ATOM | 714 | CB | ALA A | 92 | -15.416 | -7.671 | 10.746 | 1.00 | 33.43 | C |
| ATOM | 715 | N | VAL A | 93 | -13.112 | -6.143 | 11.929 | 1.00 | 30.17 | N |
| ATOM | 716 | CA | VAL A | 93 | -11.897 | -6.308 | 12.704 | 1.00 | 26.67 | C |
| ATOM | 717 | C | VAL A | 93 | -11.959 | -7.652 | 13.424 | 1.00 | 29.85 | C |
| ATOM | 718 | O | VAL A | 93 | -12.911 | -7.934 | 14.154 | 1.00 | 32.54 | O |
| ATOM | 719 | CB | VAL A | 93 | -11.705 | -5.169 | 13.726 | 1.00 | 31.84 | C |
| ATOM | 720 | CG1 | VAL A | 93 | -10.539 | -5.475 | 14.653 | 1.00 | 24.37 | C |
| ATOM | 721 | CG2 | VAL A | 93 | -11.487 | -3.842 | 13.009 | 1.00 | 25.26 | C |
| ATOM | 722 | N | TYR A | 94 | -10.948 | -8.484 | 13.207 | 1.00 | 26.52 | N |
| ATOM | 723 | CA | TYR A | 94 | -10.911 | -9.806 | 13.816 | 1.00 | 31.35 | C |
| ATOM | 724 | C | TYR A | 94 | -9.961 | -9.826 | 15.005 | 1.00 | 33.59 | C |
| ATOM | 725 | O | TYR A | 94 | -8.769 | -9.546 | 14.867 | 1.00 | 31.79 | O |
| ATOM | 726 | CB | TYR A | 94 | -10.518 | -10.861 | 12.774 | 1.00 | 25.00 | C |
| ATOM | 727 | CG | TYR A | 94 | -11.629 | -11.105 | 11.781 | 1.00 | 27.22 | C |
| ATOM | 728 | CD1 | TYR A | 94 | -11.863 | -10.216 | 10.739 | 1.00 | 30.50 | C |
| ATOM | 729 | CD2 | TYR A | 94 | -12.469 | -12.207 | 11.906 | 1.00 | 23.97 | C |
| ATOM | 730 | CE1 | TYR A | 94 | -12.893 | -10.423 | 9.842 | 1.00 | 27.95 | C |
| ATOM | 731 | CE2 | TYR A | 94 | -13.492 | -12.423 | 11.022 | 1.00 | 26.58 | C |
| ATOM | 732 | CZ | TYR A | 94 | -13.703 | -11.527 | 9.989 | 1.00 | 32.87 | C |
| ATOM | 733 | OH | TYR A | 94 | -14.730 | -11.739 | 9.104 | 1.00 | 30.47 | O |
| ATOM | 734 | N | TYR A | 95 | -10.511 | -10.135 | 16.177 | 1.00 | 24.85 | N |
| ATOM | 735 | CA | TYR A | 95 | -9.731 | -10.215 | 17.406 | 1.00 | 28.45 | C |
| ATOM | 736 | C | TYR A | 95 | -9.474 | -11.661 | 17.805 | 1.00 | 27.62 | C |
| ATOM | 737 | O | TYR A | 95 | -10.356 | -12.508 | 17.688 | 1.00 | 28.27 | O |
| ATOM | 738 | CB | TYR A | 95 | -10.450 | -9.512 | 18.564 | 1.00 | 24.54 | C |
| ATOM | 739 | CG | TYR A | 95 | -10.626 | -8.019 | 18.419 | 1.00 | 26.81 | C |
| ATOM | 740 | CD1 | TYR A | 95 | -9.580 | -7.148 | 18.692 | 1.00 | 27.26 | C |
| ATOM | 741 | CD2 | TYR A | 95 | -11.847 | -7.481 | 18.036 | 1.00 | 28.58 | C |
| ATOM | 742 | CE1 | TYR A | 95 | -9.737 | -5.778 | 18.571 | 1.00 | 27.06 | C |
| ATOM | 743 | CE2 | TYR A | 95 | -12.017 | -6.113 | 17.913 | 1.00 | 27.13 | C |
| ATOM | 744 | CZ | TYR A | 95 | -10.960 | -5.269 | 18.184 | 1.00 | 27.27 | C |
| ATOM | 745 | OH | TYR A | 95 | -11.125 | -3.911 | 18.060 | 1.00 | 31.79 | O |
| ATOM | 746 | N | CYS A | 96 | -8.274 | -11.945 | 18.289 | 1.00 | 23.92 | N |
| ATOM | 747 | CA | CYS A | 96 | -8.073 | -13.182 | 19.025 | 1.00 | 30.30 | C |
| ATOM | 748 | C | CYS A | 96 | -8.115 | -12.829 | 20.505 | 1.00 | 27.04 | C |
| ATOM | 749 | O | CYS A | 96 | -7.708 | -11.736 | 20.899 | 1.00 | 28.05 | O |
| ATOM | 750 | CB | CYS A | 96 | -6.758 | -13.877 | 18.645 | 1.00 | 33.84 | C |
| ATOM | 751 | SG | CYS A | 96 | -5.246 | -13.010 | 19.099 | 1.00 | 48.82 | S |
| ATOM | 752 | N | ALA A | 97 | -8.634 | -13.742 | 21.316 | 1.00 | 28.58 | N |
| ATOM | 753 | CA | ALA A | 97 | -8.783 | -13.497 | 22.743 | 1.00 | 26.57 | C |
| ATOM | 754 | C | ALA A | 97 | -8.768 | -14.816 | 23.507 | 1.00 | 32.11 | C |
| ATOM | 755 | O | ALA A | 97 | -9.409 | -15.780 | 23.091 | 1.00 | 30.87 | O |
| ATOM | 756 | CB | ALA A | 97 | -10.065 | -12.736 | 23.016 | 1.00 | 26.61 | C |
| ATOM | 757 | N | SER A | 98 | -8.042 | -14.864 | 24.621 | 1.00 | 27.25 | N |
| ATOM | 758 | CA | SER A | 98 | -7.888 | -16.121 | 25.348 | 1.00 | 32.20 | C |
| ATOM | 759 | C | SER A | 98 | -8.839 | -16.275 | 26.532 | 1.00 | 29.23 | C |
| ATOM | 760 | O | SER A | 98 | -9.249 | -15.294 | 27.153 | 1.00 | 25.23 | O |
| ATOM | 761 | CB | SER A | 98 | -6.453 | -16.280 | 25.845 | 1.00 | 33.45 | C |
| ATOM | 762 | OG | SER A | 98 | -6.155 | -15.337 | 26.856 | 1.00 | 45.41 | O |
| ATOM | 763 | N | SER A | 99 | -9.169 | -17.528 | 26.833 | 1.00 | 29.87 | N |
| ATOM | 764 | CA | SER A | 99 | -9.971 | -17.885 | 28.001 | 1.00 | 30.05 | C |
| ATOM | 765 | C | SER A | 99 | -9.348 | -19.099 | 28.689 | 1.00 | 35.19 | C |
| ATOM | 766 | O | SER A | 99 | -8.528 | -19.794 | 28.086 | 1.00 | 30.60 | O |
| ATOM | 767 | CB | SER A | 99 | -11.417 | -18.171 | 27.596 | 1.00 | 26.53 | C |
| ATOM | 768 | OG | SER A | 99 | -11.485 | -19.129 | 26.552 | 1.00 | 29.62 | O |
| ATOM | 769 | N | SER A 100 | | -9.738 | -19.355 | 29.938 | 1.00 | 33.90 | N |
| ATOM | 770 | CA | SER A 100 | | -9.099 | -20.396 | 30.749 | 1.00 | 39.84 | C |
| ATOM | 771 | C | SER A 100 | | -10.060 | -21.427 | 31.338 | 1.00 | 33.23 | C |
| ATOM | 772 | O | SER A 100 | | -9.932 | -21.793 | 32.506 | 1.00 | 40.18 | O |
| ATOM | 773 | CB | SER A 100 | | -8.319 | -19.758 | 31.900 | 1.00 | 32.53 | C |
| ATOM | 774 | OG | SER A 100 | | -7.194 | -19.048 | 31.424 | 1.00 | 49.78 | O |
| ATOM | 775 | O | GLY A 101 | | -14.315 | -23.137 | 30.676 | 1.00 | 40.43 | O |
| ATOM | 776 | N | GLY A 101 | | -11.017 | -21.899 | 30.547 | 1.00 | 31.28 | N |
| ATOM | 777 | CA | GLY A 101 | | -11.974 | -22.868 | 31.048 | 1.00 | 28.36 | C |
| ATOM | 778 | C | GLY A 101 | | -13.406 | -22.374 | 30.999 | 1.00 | 32.57 | C |
| ATOM | 779 | O | TRP A 102 | | -13.530 | -18.918 | 29.800 | 1.00 | 32.97 | O |

| | | | | | | | | | |
|---|---|---|---|---|---|---|---|---|---|
| ATOM | 780 | N | TRP A 102 | -13.617 | -21.110 | 31.357 | 1.00 | 23.41 | N |
| ATOM | 781 | CA | TRP A 102 | -14.879 | -20.435 | 31.059 | 1.00 | 26.91 | C |
| ATOM | 782 | C | TRP A 102 | -14.626 | -19.471 | 29.916 | 1.00 | 30.14 | C |
| ATOM | 783 | CB | TRP A 102 | -15.432 | -19.683 | 32.271 | 1.00 | 27.64 | C |
| ATOM | 784 | CG | TRP A 102 | -15.721 | -20.553 | 33.447 | 1.00 | 28.49 | C |
| ATOM | 785 | CD1 | TRP A 102 | -16.761 | -21.428 | 33.587 | 1.00 | 25.22 | C |
| ATOM | 786 | CD2 | TRP A 102 | -14.972 | -20.618 | 34.664 | 1.00 | 25.37 | C |
| ATOM | 787 | NE1 | TRP A 102 | -16.701 | -22.038 | 34.818 | 1.00 | 26.75 | N |
| ATOM | 788 | CE2 | TRP A 102 | -15.611 | -21.559 | 35.498 | 1.00 | 23.51 | C |
| ATOM | 789 | CE3 | TRP A 102 | -13.819 | -19.977 | 35.128 | 1.00 | 23.78 | C |
| ATOM | 790 | CZ2 | TRP A 102 | -15.134 | -21.874 | 36.772 | 1.00 | 27.12 | C |
| ATOM | 791 | CZ3 | TRP A 102 | -13.346 | -20.291 | 36.396 | 1.00 | 31.84 | C |
| ATOM | 792 | CH2 | TRP A 102 | -14.003 | -21.232 | 37.202 | 1.00 | 24.60 | C |
| ATOM | 793 | O | TYR A 103 | -16.560 | -16.374 | 27.434 | 1.00 | 35.87 | O |
| ATOM | 794 | N | TYR A 103 | -15.628 | -19.268 | 29.071 | 1.00 | 23.62 | N |
| ATOM | 795 | CA | TYR A 103 | -15.441 | -18.444 | 27.884 | 1.00 | 33.88 | C |
| ATOM | 796 | C | TYR A 103 | -15.723 | -16.960 | 28.117 | 1.00 | 34.03 | C |
| ATOM | 797 | CB | TYR A 103 | -16.321 | -18.949 | 26.742 | 1.00 | 27.85 | C |
| ATOM | 798 | CG | TYR A 103 | -15.988 | -20.342 | 26.265 | 1.00 | 32.69 | C |
| ATOM | 799 | CD1 | TYR A 103 | -14.804 | -20.601 | 25.588 | 1.00 | 31.32 | C |
| ATOM | 800 | CD2 | TYR A 103 | -16.866 | -21.396 | 26.478 | 1.00 | 31.92 | C |
| ATOM | 801 | CE1 | TYR A 103 | -14.498 | -21.879 | 25.143 | 1.00 | 32.07 | C |
| ATOM | 802 | CE2 | TYR A 103 | -16.571 | -22.673 | 26.036 | 1.00 | 31.82 | C |
| ATOM | 803 | CZ | TYR A 103 | -15.385 | -22.908 | 25.370 | 1.00 | 34.61 | C |
| ATOM | 804 | OH | TYR A 103 | -15.090 | -24.176 | 24.931 | 1.00 | 37.00 | O |
| ATOM | 805 | N | TYR A 104 | -15.035 | -16.359 | 29.085 | 1.00 | 30.47 | N |
| ATOM | 806 | CA | TYR A 104 | -14.929 | -14.904 | 29.133 | 1.00 | 34.52 | C |
| ATOM | 807 | C | TYR A 104 | -13.452 | -14.557 | 28.940 | 1.00 | 37.81 | C |
| ATOM | 808 | O | TYR A 104 | -12.571 | -15.311 | 29.359 | 1.00 | 33.63 | O |
| ATOM | 809 | CB | TYR A 104 | -15.488 | -14.328 | 30.439 | 1.00 | 28.01 | C |
| ATOM | 810 | CG | TYR A 104 | -14.762 | -14.755 | 31.690 | 1.00 | 29.48 | C |
| ATOM | 811 | CD2 | TYR A 104 | -15.209 | -15.837 | 32.442 | 1.00 | 31.05 | C |
| ATOM | 812 | CD1 | TYR A 104 | -13.639 | -14.068 | 32.132 | 1.00 | 33.84 | C |
| ATOM | 813 | CE2 | TYR A 104 | -14.547 | -16.227 | 33.600 | 1.00 | 31.77 | C |
| ATOM | 814 | CE1 | TYR A 104 | -12.968 | -14.452 | 33.282 | 1.00 | 38.93 | C |
| ATOM | 815 | CZ | TYR A 104 | -13.424 | -15.530 | 34.009 | 1.00 | 35.52 | C |
| ATOM | 816 | OH | TYR A 104 | -12.751 | -15.905 | 35.147 | 1.00 | 47.88 | O |
| ATOM | 817 | N | PHE A 105 | -13.180 | -13.423 | 28.308 | 1.00 | 34.39 | N |
| ATOM | 818 | CA | PHE A 105 | -11.862 | -13.201 | 27.718 | 1.00 | 31.85 | C |
| ATOM | 819 | C | PHE A 105 | -11.046 | -12.124 | 28.419 | 1.00 | 31.30 | C |
| ATOM | 820 | O | PHE A 105 | -11.367 | -10.938 | 28.347 | 1.00 | 33.02 | O |
| ATOM | 821 | CB | PHE A 105 | -12.046 | -12.872 | 26.240 | 1.00 | 27.43 | C |
| ATOM | 822 | CG | PHE A 105 | -13.093 | -13.719 | 25.583 | 1.00 | 32.14 | C |
| ATOM | 823 | CD1 | PHE A 105 | -12.894 | -15.083 | 25.423 | 1.00 | 32.34 | C |
| ATOM | 824 | CD2 | PHE A 105 | -14.292 | -13.168 | 25.166 | 1.00 | 33.77 | C |
| ATOM | 825 | CE1 | PHE A 105 | -13.866 | -15.879 | 24.840 | 1.00 | 28.51 | C |
| ATOM | 826 | CE2 | PHE A 105 | -15.267 | -13.959 | 24.577 | 1.00 | 36.93 | C |
| ATOM | 827 | CZ | PHE A 105 | -15.052 | -15.317 | 24.413 | 1.00 | 31.65 | C |
| ATOM | 828 | N | ASP A 106 | -9.981 | -12.543 | 29.097 | 1.00 | 31.31 | N |
| ATOM | 829 | CA | ASP A 106 | -9.205 | -11.614 | 29.914 | 1.00 | 33.51 | C |
| ATOM | 830 | C | ASP A 106 | -8.015 | -11.014 | 29.167 | 1.00 | 32.69 | C |
| ATOM | 831 | O | ASP A 106 | -7.485 | -9.983 | 29.578 | 1.00 | 38.29 | O |
| ATOM | 832 | CB | ASP A 106 | -8.734 | -12.298 | 31.206 | 1.00 | 37.27 | C |
| ATOM | 833 | CG | ASP A 106 | -8.078 | -13.645 | 30.962 | 1.00 | 44.11 | C |
| ATOM | 834 | OD1 | ASP A 106 | -8.298 | -14.243 | 29.886 | 1.00 | 44.95 | O |
| ATOM | 835 | OD2 | ASP A 106 | -7.347 | -14.114 | 31.860 | 1.00 | 39.24 | O |
| ATOM | 836 | N | TYR A 107 | -7.604 | -11.645 | 28.069 | 1.00 | 33.81 | N |
| ATOM | 837 | CA | TYR A 107 | -6.550 | -11.085 | 27.220 | 1.00 | 31.46 | C |
| ATOM | 838 | C | TYR A 107 | -6.967 | -11.061 | 25.752 | 1.00 | 27.21 | C |
| ATOM | 839 | O | TYR A 107 | -7.565 | -12.015 | 25.256 | 1.00 | 27.70 | O |
| ATOM | 840 | CB | TYR A 107 | -5.250 | -11.870 | 27.388 | 1.00 | 34.62 | C |
| ATOM | 841 | CG | TYR A 107 | -4.719 | -11.830 | 28.802 | 1.00 | 39.20 | C |
| ATOM | 842 | CD1 | TYR A 107 | -4.065 | -10.705 | 29.284 | 1.00 | 44.79 | C |
| ATOM | 843 | CD2 | TYR A 107 | -4.882 | -12.912 | 29.659 | 1.00 | 42.77 | C |
| ATOM | 844 | CE1 | TYR A 107 | -3.584 | -10.658 | 30.583 | 1.00 | 57.22 | C |
| ATOM | 845 | CE2 | TYR A 107 | -4.403 | -12.875 | 30.957 | 1.00 | 50.96 | C |
| ATOM | 846 | CZ | TYR A 107 | -3.758 | -11.746 | 31.414 | 1.00 | 48.15 | C |
| ATOM | 847 | OH | TYR A 107 | -3.279 | -11.702 | 32.703 | 1.00 | 71.00 | O |
| ATOM | 848 | N | TRP A 108 | -6.638 | -9.967 | 25.066 | 1.00 | 27.81 | N |
| ATOM | 849 | CA | TRP A 108 | -7.055 | -9.741 | 23.679 | 1.00 | 21.18 | C |
| ATOM | 850 | C | TRP A 108 | -5.892 | -9.324 | 22.782 | 1.00 | 26.32 | C |

```
ATOM   851  O    TRP A 108    -4.984  -8.615  23.218  1.00 29.92       O
ATOM   852  CB   TRP A 108    -8.132  -8.654  23.610  1.00 21.90       C
ATOM   853  CG   TRP A 108    -9.444  -9.007  24.233  1.00 27.98       C
ATOM   854  CD1  TRP A 108    -9.700  -9.207  25.560  1.00 27.08       C
ATOM   855  CD2  TRP A 108   -10.693  -9.171  23.554  1.00 27.01       C
ATOM   856  CE2  TRP A 108   -11.663  -9.484  24.530  1.00 25.58       C
ATOM   857  CE3  TRP A 108   -11.085  -9.093  22.212  1.00 25.64       C
ATOM   858  NE1  TRP A 108   -11.032  -9.500  25.746  1.00 24.70       N
ATOM   859  CZ2  TRP A 108   -12.999  -9.715  24.208  1.00 25.47       C
ATOM   860  CZ3  TRP A 108   -12.414  -9.322  21.893  1.00 32.29       C
ATOM   861  CH2  TRP A 108   -13.355  -9.630  22.889  1.00 32.24       C
ATOM   862  N    GLY A 109    -5.928  -9.748  21.523  1.00 27.02       N
ATOM   863  CA   GLY A 109    -5.002  -9.227  20.531  1.00 28.74       C
ATOM   864  C    GLY A 109    -5.415  -7.812  20.159  1.00 31.88       C
ATOM   865  O    GLY A 109    -6.476  -7.346  20.584  1.00 26.79       O
ATOM   866  N    GLN A 110    -4.595  -7.120  19.373  1.00 28.50       N
ATOM   867  CA   GLN A 110    -4.878  -5.721  19.055  1.00 29.47       C
ATOM   868  C    GLN A 110    -5.904  -5.611  17.928  1.00 30.21       C
ATOM   869  O    GLN A 110    -6.432  -4.532  17.659  1.00 36.00       O
ATOM   870  CB   GLN A 110    -3.591  -4.966  18.683  1.00 23.57       C
ATOM   871  CG   GLN A 110    -3.125  -5.156  17.239  1.00 29.23       C
ATOM   872  CD   GLN A 110    -2.309  -6.419  17.032  1.00 33.10       C
ATOM   873  NE2  GLN A 110    -1.568  -6.467  15.929  1.00 31.73       N
ATOM   874  OE1  GLN A 110    -2.343  -7.342  17.850  1.00 35.23       O
ATOM   875  N    GLY A 111    -6.192  -6.733  17.277  1.00 31.62       N
ATOM   876  CA   GLY A 111    -7.188  -6.760  16.225  1.00 31.32       C
ATOM   877  C    GLY A 111    -6.604  -6.675  14.831  1.00 34.22       C
ATOM   878  O    GLY A 111    -5.561  -6.059  14.612  1.00 31.13       O
ATOM   879  N    THR A 112    -7.291  -7.299  13.882  1.00 29.50       N
ATOM   880  CA   THR A 112    -6.858  -7.300  12.494  1.00 30.89       C
ATOM   881  C    THR A 112    -8.008  -6.901  11.587  1.00 30.69       C
ATOM   882  O    THR A 112    -9.035  -7.577  11.551  1.00 28.68       O
ATOM   883  CB   THR A 112    -6.334  -8.683  12.064  1.00 34.70       C
ATOM   884  CG2  THR A 112    -5.960  -8.683  10.584  1.00 33.17       C
ATOM   885  OG1  THR A 112    -5.182  -9.025  12.846  1.00 39.75       O
ATOM   886  N    LEU A 113    -7.842  -5.804  10.859  1.00 28.77       N
ATOM   887  CA   LEU A 113    -8.868  -5.372   9.917  1.00 26.18       C
ATOM   888  C    LEU A 113    -8.801  -6.203   8.635  1.00 32.80       C
ATOM   889  O    LEU A 113    -7.769  -6.263   7.968  1.00 31.11       O
ATOM   890  CB   LEU A 113    -8.721  -3.883   9.587  1.00 26.67       C
ATOM   891  CG   LEU A 113    -9.680  -3.339   8.518  1.00 32.26       C
ATOM   892  CD1  LEU A 113   -11.139  -3.581   8.899  1.00 29.02       C
ATOM   893  CD2  LEU A 113    -9.437  -1.855   8.262  1.00 32.24       C
ATOM   894  N    VAL A 114    -9.904  -6.859   8.305  1.00 28.44       N
ATOM   895  CA   VAL A 114   -10.009  -7.566   7.036  1.00 30.93       C
ATOM   896  C    VAL A 114   -11.076  -6.899   6.188  1.00 30.62       C
ATOM   897  O    VAL A 114   -12.233  -6.788   6.601  1.00 29.64       O
ATOM   898  CB   VAL A 114   -10.353  -9.058   7.225  1.00 31.72       C
ATOM   899  CG1  VAL A 114   -10.535  -9.740   5.869  1.00 33.36       C
ATOM   900  CG2  VAL A 114    -9.265  -9.750   8.042  1.00 32.90       C
ATOM   901  N    THR A 115   -10.676  -6.436   5.009  1.00 35.29       N
ATOM   902  CA   THR A 115   -11.604  -5.788   4.100  1.00 34.33       C
ATOM   903  C    THR A 115   -11.863  -6.700   2.912  1.00 36.39       C
ATOM   904  O    THR A 115   -10.929  -7.156   2.251  1.00 35.90       O
ATOM   905  CB   THR A 115   -11.068  -4.426   3.610  1.00 36.40       C
ATOM   906  CG2  THR A 115   -12.038  -3.785   2.628  1.00 34.46       C
ATOM   907  OG1  THR A 115   -10.890  -3.552   4.730  1.00 37.23       O
ATOM   908  N    VAL A 116   -13.136  -6.976   2.657  1.00 39.45       N
ATOM   909  CA   VAL A 116   -13.527  -7.775   1.504  1.00 34.84       C
ATOM   910  C    VAL A 116   -14.212  -6.875   0.490  1.00 38.13       C
ATOM   911  O    VAL A 116   -15.289  -6.339   0.755  1.00 35.45       O
ATOM   912  CB   VAL A 116   -14.475  -8.932   1.886  1.00 39.24       C
ATOM   913  CG1  VAL A 116   -14.679  -9.864   0.693  1.00 39.66       C
ATOM   914  CG2  VAL A 116   -13.924  -9.705   3.075  1.00 38.07       C
ATOM   915  N    SER A 117   -13.589  -6.706  -0.671  1.00 34.44       N
ATOM   916  CA   SER A 117   -14.126  -5.795  -1.671  1.00 40.89       C
ATOM   917  C    SER A 117   -13.687  -6.131  -3.091  1.00 40.89       C
ATOM   918  O    SER A 117   -12.598  -6.661  -3.313  1.00 38.45       O
ATOM   919  CB   SER A 117   -13.716  -4.359  -1.345  1.00 34.47       C
ATOM   920  OG   SER A 117   -14.224  -3.460  -2.318  1.00 43.36       O
ATOM   921  O    SER A 118   -12.636  -4.911  -6.922  1.00 43.31       O
```

```
ATOM   922  N   SER A 118   -14.546  -5.795  -4.047  1.00 42.01      N
ATOM   923  CA  SER A 118   -14.232  -5.940  -5.462  1.00 52.20      C
ATOM   924  C   SER A 118   -13.391  -4.770  -5.963  1.00 47.46      C
ATOM   925  CB  SER A 118   -15.516  -6.046  -6.287  1.00 44.49      C
ATOM   926  OG  SER A 118   -16.364  -7.057  -5.772  1.00 64.31      O
ATOM   927  O   ALA A 119   -10.794  -3.378  -5.023  1.00 46.60      O
ATOM   928  N   ALA A 119   -13.530  -3.617  -5.311  1.00 44.58      N
ATOM   929  CA  ALA A 119   -12.862  -2.393  -5.753  1.00 40.93      C
ATOM   930  C   ALA A 119   -11.342  -2.520  -5.714  1.00 45.33      C
ATOM   931  CB  ALA A 119   -13.312  -1.211  -4.908  1.00 36.58      C
ATOM   932  N   SER A 120   -10.666  -1.656  -6.462  1.00 46.47      N
ATOM   933  CA  SER A 120    -9.215  -1.709  -6.568  1.00 45.38      C
ATOM   934  C   SER A 120    -8.519  -1.011  -5.404  1.00 40.94      C
ATOM   935  O   SER A 120    -8.988   0.011  -4.907  1.00 41.57      O
ATOM   936  CB  SER A 120    -8.765  -1.087  -7.888  1.00 48.55      C
ATOM   937  OG  SER A 120    -9.353  -1.760  -8.986  1.00 60.18      O
ATOM   938  N   THR A 121    -7.396  -1.579  -4.977  1.00 34.74      N
ATOM   939  CA  THR A 121    -6.548  -0.974  -3.960  1.00 32.02      C
ATOM   940  C   THR A 121    -5.820   0.246  -4.520  1.00 36.76      C
ATOM   941  O   THR A 121    -5.170   0.163  -5.560  1.00 38.97      O
ATOM   942  CB  THR A 121    -5.515  -1.984  -3.424  1.00 34.58      C
ATOM   943  CG2 THR A 121    -4.467  -1.287  -2.564  1.00 34.08      C
ATOM   944  OG1 THR A 121    -6.183  -2.982  -2.643  1.00 35.30      O
ATOM   945  N   LYS A 122    -5.925   1.374  -3.822  1.00 38.80      N
ATOM   946  CA  LYS A 122    -5.311   2.619  -4.276  1.00 33.51      C
ATOM   947  C   LYS A 122    -4.612   3.348  -3.137  1.00 34.96      C
ATOM   948  O   LYS A 122    -5.217   3.618  -2.103  1.00 36.45      O
ATOM   949  CB  LYS A 122    -6.364   3.533  -4.907  1.00 31.35      C
ATOM   950  CG  LYS A 122    -5.799   4.818  -5.474  1.00 41.47      C
ATOM   951  CD  LYS A 122    -6.894   5.726  -6.013  1.00 44.52      C
ATOM   952  CE  LYS A 122    -6.300   6.975  -6.653  1.00 45.34      C
ATOM   953  NZ  LYS A 122    -5.380   7.695  -5.723  1.00 52.67      N
ATOM   954  N   GLY A 123    -3.336   3.665  -3.328  1.00 34.17      N
ATOM   955  CA  GLY A 123    -2.581   4.418  -2.344  1.00 31.92      C
ATOM   956  C   GLY A 123    -2.957   5.889  -2.359  1.00 38.36      C
ATOM   957  O   GLY A 123    -3.423   6.403  -3.375  1.00 39.10      O
ATOM   958  N   PRO A 124    -2.747   6.578  -1.228  1.00 35.34      N
ATOM   959  CA  PRO A 124    -3.181   7.963  -1.024  1.00 33.85      C
ATOM   960  C   PRO A 124    -2.245   9.021  -1.600  1.00 38.95      C
ATOM   961  O   PRO A 124    -1.036   8.809  -1.689  1.00 33.76      O
ATOM   962  CB  PRO A 124    -3.209   8.084   0.497  1.00 31.61      C
ATOM   963  CG  PRO A 124    -2.100   7.184   0.944  1.00 31.80      C
ATOM   964  CD  PRO A 124    -2.095   6.024  -0.026  1.00 35.65      C
ATOM   965  N   SER A 125    -2.817  10.160  -1.974  1.00 31.51      N
ATOM   966  CA  SER A 125    -2.038  11.366  -2.216  1.00 34.17      C
ATOM   967  C   SER A 125    -2.015  12.176  -0.926  1.00 32.77      C
ATOM   968  O   SER A 125    -3.041  12.315  -0.261  1.00 38.58      O
ATOM   969  CB  SER A 125    -2.631  12.189  -3.362  1.00 40.17      C
ATOM   970  OG  SER A 125    -2.812  11.397  -4.524  1.00 45.30      O
ATOM   971  N   VAL A 126    -0.851  12.695  -0.557  1.00 28.64      N
ATOM   972  CA  VAL A 126    -0.734  13.466   0.674  1.00 28.98      C
ATOM   973  C   VAL A 126    -0.444  14.927   0.363  1.00 33.93      C
ATOM   974  O   VAL A 126     0.556  15.244  -0.279  1.00 37.76      O
ATOM   975  CB  VAL A 126     0.367  12.905   1.593  1.00 28.89      C
ATOM   976  CG1 VAL A 126     0.374  13.641   2.930  1.00 28.32      C
ATOM   977  CG2 VAL A 126     0.169  11.413   1.797  1.00 24.48      C
ATOM   978  N   PHE A 127    -1.325  15.809   0.825  1.00 29.15      N
ATOM   979  CA  PHE A 127    -1.212  17.236   0.557  1.00 31.17      C
ATOM   980  C   PHE A 127    -1.034  18.028   1.850  1.00 38.80      C
ATOM   981  O   PHE A 127    -1.578  17.657   2.892  1.00 31.26      O
ATOM   982  CB  PHE A 127    -2.448  17.739  -0.198  1.00 29.74      C
ATOM   983  CG  PHE A 127    -2.709  17.013  -1.484  1.00 32.93      C
ATOM   984  CD1 PHE A 127    -1.798  17.076  -2.529  1.00 36.91      C
ATOM   985  CD2 PHE A 127    -3.869  16.275  -1.657  1.00 33.66      C
ATOM   986  CE1 PHE A 127    -2.033  16.407  -3.718  1.00 30.94      C
ATOM   987  CE2 PHE A 127    -4.114  15.605  -2.848  1.00 38.29      C
ATOM   988  CZ  PHE A 127    -3.194  15.670  -3.878  1.00 33.93      C
ATOM   989  N   PRO A 128    -0.278  19.134   1.785  1.00 38.69      N
ATOM   990  CA  PRO A 128    -0.029  19.931   2.990  1.00 31.44      C
ATOM   991  C   PRO A 128    -1.230  20.772   3.410  1.00 34.99      C
ATOM   992  O   PRO A 128    -1.930  21.318   2.562  1.00 36.42      O
```

```
ATOM    993  CB  PRO A 128       1.136  20.832   2.575  1.00 35.21           C
ATOM    994  CG  PRO A 128       0.966  20.997   1.104  1.00 31.99           C
ATOM    995  CD  PRO A 128       0.395  19.694   0.598  1.00 31.14           C
ATOM    996  N   LEU A 129      -1.470  20.845   4.714  1.00 31.68           N
ATOM    997  CA  LEU A 129      -2.354  21.853   5.280  1.00 32.81           C
ATOM    998  C   LEU A 129      -1.451  22.903   5.908  1.00 29.54           C
ATOM    999  O   LEU A 129      -1.054  22.777   7.063  1.00 28.79           O
ATOM   1000  CB  LEU A 129      -3.309  21.249   6.314  1.00 28.35           C
ATOM   1001  CG  LEU A 129      -4.272  20.181   5.787  1.00 32.12           C
ATOM   1002  CD1 LEU A 129      -5.104  19.577   6.917  1.00 27.87           C
ATOM   1003  CD2 LEU A 129      -5.170  20.762   4.700  1.00 27.99           C
ATOM   1004  N   ALA A 130      -1.106  23.923   5.130  1.00 30.89           N
ATOM   1005  CA  ALA A 130      -0.044  24.853   5.509  1.00 38.77           C
ATOM   1006  C   ALA A 130      -0.484  25.820   6.595  1.00 37.50           C
ATOM   1007  O   ALA A 130      -1.576  26.378   6.524  1.00 41.37           O
ATOM   1008  CB  ALA A 130       0.445  25.624   4.286  1.00 37.41           C
ATOM   1009  N   PRO A 131       0.381  26.031   7.599  1.00 45.74           N
ATOM   1010  CA  PRO A 131       0.059  26.928   8.714  1.00 46.02           C
ATOM   1011  C   PRO A 131      -0.111  28.370   8.245  1.00 52.71           C
ATOM   1012  O   PRO A 131       0.650  28.845   7.397  1.00 47.91           O
ATOM   1013  CB  PRO A 131       1.264  26.778   9.647  1.00 40.81           C
ATOM   1014  CG  PRO A 131       2.376  26.354   8.764  1.00 45.92           C
ATOM   1015  CD  PRO A 131       1.751  25.499   7.694  1.00 43.34           C
ATOM   1016  N   SER A 132      -1.117  29.043   8.793  1.00 55.56           N
ATOM   1017  CA  SER A 132      -1.473  30.396   8.380  1.00 71.89           C
ATOM   1018  C   SER A 132      -0.309  31.371   8.517  1.00 74.46           C
ATOM   1019  O   SER A 132       0.408  31.364   9.518  1.00 79.24           O
ATOM   1020  CB  SER A 132      -2.670  30.896   9.198  1.00 76.95           C
ATOM   1021  OG  SER A 132      -3.053  32.204   8.811  1.00 77.97           O
ATOM   1022  N   SER A 133      -0.120  32.201   7.496  1.00 80.68           N
ATOM   1023  CA  SER A 133       0.838  33.296   7.575  1.00 89.81           C
ATOM   1024  C   SER A 133       0.309  34.331   8.560  1.00 95.47           C
ATOM   1025  O   SER A 133       1.072  35.087   9.166  1.00 92.23           O
ATOM   1026  CB  SER A 133       1.075  33.922   6.198  1.00 81.20           C
ATOM   1027  OG  SER A 133      -0.144  34.340   5.609  1.00 74.80           O
ATOM   1028  N   LYS A 134      -1.012  34.343   8.717  1.00 93.18           N
ATOM   1029  CA  LYS A 134      -1.678  35.224   9.665  1.00 90.77           C
ATOM   1030  C   LYS A 134      -1.923  34.525  11.001  1.00 94.50           C
ATOM   1031  O   LYS A 134      -2.896  34.825  11.696  1.00 94.00           O
ATOM   1032  CB  LYS A 134      -3.003  35.729   9.089  1.00 86.16           C
ATOM   1033  CG  LYS A 134      -2.852  36.635   7.879  1.00 83.75           C
ATOM   1034  CD  LYS A 134      -4.135  37.409   7.614  1.00 91.56           C
ATOM   1035  CE  LYS A 134      -3.926  38.492   6.567  1.00 95.47           C
ATOM   1036  NZ  LYS A 134      -5.110  39.389   6.446  1.00 88.66           N
ATOM   1037  N   SER A 135      -1.045  33.588  11.353  1.00 96.11           N
ATOM   1038  CA  SER A 135      -1.111  32.936  12.658  1.00 97.73           C
ATOM   1039  C   SER A 135      -0.885  33.973  13.753  1.00102.54           C
ATOM   1040  O   SER A 135      -0.148  34.941  13.555  1.00102.52           O
ATOM   1041  CB  SER A 135      -0.077  31.810  12.768  1.00 83.08           C
ATOM   1042  OG  SER A 135      -0.335  30.775  11.834  1.00 78.24           O
ATOM   1043  N   THR A 136      -1.524  33.775  14.901  1.00102.47           N
ATOM   1044  CA  THR A 136      -1.399  34.717  16.009  1.00105.40           C
ATOM   1045  C   THR A 136       0.030  34.741  16.550  1.00104.11           C
ATOM   1046  O   THR A 136       0.518  33.744  17.085  1.00 99.44           O
ATOM   1047  CB  THR A 136      -2.377  34.379  17.154  1.00 99.90           C
ATOM   1048  CG2 THR A 136      -3.817  34.548  16.692  1.00100.80           C
ATOM   1049  OG1 THR A 136      -2.173  33.027  17.582  1.00 97.52           O
ATOM   1050  N   SER A 137       0.696  35.883  16.395  1.00105.35           N
ATOM   1051  CA  SER A 137       2.071  36.049  16.858  1.00104.45           C
ATOM   1052  C   SER A 137       2.168  35.866  18.370  1.00102.60           C
ATOM   1053  O   SER A 137       1.517  36.580  19.135  1.00 98.58           O
ATOM   1054  CB  SER A 137       2.610  37.424  16.456  1.00105.78           C
ATOM   1055  OG  SER A 137       2.624  37.578  15.047  1.00 96.80           O
ATOM   1056  N   GLY A 138       2.981  34.902  18.790  1.00 97.35           N
ATOM   1057  CA  GLY A 138       3.115  34.571  20.197  1.00 98.58           C
ATOM   1058  C   GLY A 138       1.978  33.692  20.685  1.00 99.72           C
ATOM   1059  O   GLY A 138       1.816  33.480  21.888  1.00 98.83           O
ATOM   1060  N   GLY A 139       1.188  33.180  19.744  1.00 93.81           N
ATOM   1061  CA  GLY A 139       0.044  32.348  20.066  1.00 77.07           C
ATOM   1062  C   GLY A 139       0.181  30.926  19.557  1.00 70.80           C
ATOM   1063  O   GLY A 139       1.247  30.316  19.662  1.00 65.42           O
```

220

```
ATOM   1064  N   THR A 140     -0.904  30.398  18.998  1.00 65.95           N
ATOM   1065  CA  THR A 140     -0.941  29.012  18.541  1.00 60.59           C
ATOM   1066  C   THR A 140     -1.281  28.906  17.053  1.00 54.28           C
ATOM   1067  O   THR A 140     -2.238  29.520  16.580  1.00 54.81           O
ATOM   1068  CB  THR A 140     -1.963  28.189  19.360  1.00 57.65           C
ATOM   1069  CG2 THR A 140     -2.218  26.832  18.719  1.00 45.39           C
ATOM   1070  OG1 THR A 140     -1.463  27.993  20.689  1.00 65.11           O
ATOM   1071  N   ALA A 141     -0.488  28.129  16.321  1.00 41.19           N
ATOM   1072  CA  ALA A 141     -0.767  27.862  14.914  1.00 47.82           C
ATOM   1073  C   ALA A 141     -1.193  26.410  14.708  1.00 39.86           C
ATOM   1074  O   ALA A 141     -0.881  25.541  15.521  1.00 42.63           O
ATOM   1075  CB  ALA A 141      0.447  28.181  14.059  1.00 33.31           C
ATOM   1076  N   ALA A 142     -1.911  26.151  13.621  1.00 35.54           N
ATOM   1077  CA  ALA A 142     -2.279  24.787  13.265  1.00 32.56           C
ATOM   1078  C   ALA A 142     -1.747  24.446  11.885  1.00 30.50           C
ATOM   1079  O   ALA A 142     -1.761  25.276  10.978  1.00 39.38           O
ATOM   1080  CB  ALA A 142     -3.791  24.600  13.317  1.00 33.11           C
ATOM   1081  N   LEU A 143     -1.267  23.220  11.735  1.00 29.03           N
ATOM   1082  CA  LEU A 143     -0.827  22.726  10.447  1.00 27.15           C
ATOM   1083  C   LEU A 143     -1.214  21.263  10.343  1.00 29.85           C
ATOM   1084  O   LEU A 143     -1.577  20.643  11.340  1.00 30.37           O
ATOM   1085  CB  LEU A 143      0.681  22.922  10.267  1.00 31.63           C
ATOM   1086  CG  LEU A 143      1.648  22.236  11.232  1.00 36.90           C
ATOM   1087  CD1 LEU A 143      2.145  20.915  10.665  1.00 35.87           C
ATOM   1088  CD2 LEU A 143      2.817  23.154  11.551  1.00 37.42           C
ATOM   1089  N   GLY A 144     -1.155  20.708   9.140  1.00 25.86           N
ATOM   1090  CA  GLY A 144     -1.542  19.327   8.968  1.00 28.52           C
ATOM   1091  C   GLY A 144     -1.254  18.715   7.615  1.00 24.81           C
ATOM   1092  O   GLY A 144     -0.577  19.300   6.774  1.00 28.21           O
ATOM   1093  N   CYS A 145     -1.779  17.511   7.428  1.00 23.50           N
ATOM   1094  CA  CYS A 145     -1.680  16.777   6.178  1.00 26.22           C
ATOM   1095  C   CYS A 145     -3.059  16.307   5.759  1.00 29.48           C
ATOM   1096  O   CYS A 145     -3.828  15.820   6.587  1.00 28.29           O
ATOM   1097  CB  CYS A 145     -0.745  15.567   6.317  1.00 32.48           C
ATOM   1098  SG  CYS A 145      1.014  15.968   6.295  1.00 53.28           S
ATOM   1099  N   LEU A 146     -3.364  16.447   4.475  1.00 31.34           N
ATOM   1100  CA  LEU A 146     -4.579  15.883   3.911  1.00 25.63           C
ATOM   1101  C   LEU A 146     -4.235  14.603   3.157  1.00 30.29           C
ATOM   1102  O   LEU A 146     -3.490  14.627   2.177  1.00 34.41           O
ATOM   1103  CB  LEU A 146     -5.265  16.895   2.991  1.00 30.65           C
ATOM   1104  CG  LEU A 146     -6.575  16.493   2.311  1.00 36.27           C
ATOM   1105  CD1 LEU A 146     -7.664  16.170   3.329  1.00 29.24           C
ATOM   1106  CD2 LEU A 146     -7.027  17.607   1.378  1.00 36.45           C
ATOM   1107  N   VAL A 147     -4.772  13.485   3.628  1.00 30.39           N
ATOM   1108  CA  VAL A 147     -4.504  12.182   3.033  1.00 27.86           C
ATOM   1109  C   VAL A 147     -5.700  11.750   2.184  1.00 27.94           C
ATOM   1110  O   VAL A 147     -6.718  11.321   2.711  1.00 33.43           O
ATOM   1111  CB  VAL A 147     -4.209  11.143   4.123  1.00 30.05           C
ATOM   1112  CG1 VAL A 147     -3.807   9.811   3.509  1.00 31.17           C
ATOM   1113  CG2 VAL A 147     -3.116  11.661   5.053  1.00 29.02           C
ATOM   1114  N   LYS A 148     -5.579  11.870   0.866  1.00 32.05           N
ATOM   1115  CA  LYS A 148     -6.760  11.820   0.010  1.00 38.00           C
ATOM   1116  C   LYS A 148     -6.740  10.696  -1.026  1.00 35.47           C
ATOM   1117  O   LYS A 148     -5.685  10.328  -1.539  1.00 38.98           O
ATOM   1118  CB  LYS A 148     -6.935  13.172  -0.697  1.00 39.17           C
ATOM   1119  CG  LYS A 148     -8.280  13.343  -1.383  1.00 47.97           C
ATOM   1120  CD  LYS A 148     -8.527  14.785  -1.779  1.00 41.88           C
ATOM   1121  CE  LYS A 148     -9.901  14.951  -2.417  1.00 51.38           C
ATOM   1122  NZ  LYS A 148    -10.035  14.191  -3.688  1.00 52.46           N
ATOM   1123  N   ASP A 149     -7.929  10.160  -1.309  1.00 39.61           N
ATOM   1124  CA  ASP A 149     -8.162   9.206  -2.398  1.00 36.49           C
ATOM   1125  C   ASP A 149     -7.423   7.886  -2.225  1.00 40.38           C
ATOM   1126  O   ASP A 149     -6.620   7.499  -3.076  1.00 38.28           O
ATOM   1127  CB  ASP A 149     -7.779   9.829  -3.748  1.00 35.81           C
ATOM   1128  CG  ASP A 149     -8.690  10.975  -4.139  1.00 44.23           C
ATOM   1129  OD1 ASP A 149     -9.834  11.014  -3.642  1.00 45.39           O
ATOM   1130  OD2 ASP A 149     -8.265  11.835  -4.940  1.00 51.78           O
ATOM   1131  N   TYR A 150     -7.702   7.185  -1.133  1.00 28.61           N
ATOM   1132  CA  TYR A 150     -7.115   5.872  -0.950  1.00 31.03           C
ATOM   1133  C   TYR A 150     -8.197   4.827  -0.728  1.00 38.37           C
ATOM   1134  O   TYR A 150     -9.354   5.159  -0.456  1.00 36.04           O
```

```
ATOM   1135  CB   TYR A 150      -6.113   5.872   0.212  1.00 29.48           C
ATOM   1136  CG   TYR A 150      -6.713   6.143   1.571  1.00 32.63           C
ATOM   1137  CD1  TYR A 150      -6.837   7.439   2.051  1.00 31.16           C
ATOM   1138  CD2  TYR A 150      -7.145   5.100   2.380  1.00 34.56           C
ATOM   1139  CE1  TYR A 150      -7.381   7.689   3.295  1.00 26.37           C
ATOM   1140  CE2  TYR A 150      -7.691   5.340   3.625  1.00 30.49           C
ATOM   1141  CZ   TYR A 150      -7.804   6.633   4.079  1.00 32.92           C
ATOM   1142  OH   TYR A 150      -8.347   6.865   5.322  1.00 33.53           O
ATOM   1143  N    PHE A 151      -7.807   3.565  -0.866  1.00 30.04           N
ATOM   1144  CA   PHE A 151      -8.699   2.437  -0.650  1.00 32.42           C
ATOM   1145  C    PHE A 151      -7.861   1.173  -0.518  1.00 32.34           C
ATOM   1146  O    PHE A 151      -6.895   0.992  -1.258  1.00 38.41           O
ATOM   1147  CB   PHE A 151      -9.708   2.298  -1.799  1.00 32.75           C
ATOM   1148  CG   PHE A 151     -10.788   1.287  -1.535  1.00 35.46           C
ATOM   1149  CD2  PHE A 151     -11.978   1.669  -0.936  1.00 38.75           C
ATOM   1150  CD1  PHE A 151     -10.605  -0.047  -1.863  1.00 36.37           C
ATOM   1151  CE2  PHE A 151     -12.969   0.743  -0.676  1.00 37.03           C
ATOM   1152  CE1  PHE A 151     -11.595  -0.981  -1.605  1.00 36.99           C
ATOM   1153  CZ   PHE A 151     -12.777  -0.584  -1.013  1.00 38.87           C
ATOM   1154  N    PRO A 152      -8.207   0.307   0.446  1.00 36.93           N
ATOM   1155  CA   PRO A 152      -9.241   0.535   1.456  1.00 33.39           C
ATOM   1156  C    PRO A 152      -8.638   1.119   2.726  1.00 36.06           C
ATOM   1157  O    PRO A 152      -7.493   1.579   2.706  1.00 30.72           O
ATOM   1158  CB   PRO A 152      -9.775  -0.869   1.707  1.00 35.39           C
ATOM   1159  CG   PRO A 152      -8.534  -1.711   1.609  1.00 34.66           C
ATOM   1160  CD   PRO A 152      -7.652  -1.056   0.552  1.00 34.24           C
ATOM   1161  N    GLU A 153      -9.400   1.095   3.816  1.00 31.54           N
ATOM   1162  CA   GLU A 153      -8.855   1.413   5.132  1.00 35.20           C
ATOM   1163  C    GLU A 153      -7.853   0.323   5.528  1.00 30.90           C
ATOM   1164  O    GLU A 153      -7.918  -0.790   5.012  1.00 30.88           O
ATOM   1165  CB   GLU A 153      -9.979   1.531   6.170  1.00 34.19           C
ATOM   1166  CG   GLU A 153     -10.884   2.735   5.978  1.00 30.47           C
ATOM   1167  CD   GLU A 153     -10.607   3.838   6.980  1.00 49.19           C
ATOM   1168  OE1  GLU A 153     -11.550   4.226   7.705  1.00 56.79           O
ATOM   1169  OE2  GLU A 153      -9.452   4.323   7.042  1.00 53.78           O
ATOM   1170  N    PRO A 154      -6.923   0.634   6.444  1.00 31.40           N
ATOM   1171  CA   PRO A 154      -6.719   1.915   7.118  1.00 33.58           C
ATOM   1172  C    PRO A 154      -5.436   2.624   6.692  1.00 34.09           C
ATOM   1173  O    PRO A 154      -4.576   2.024   6.047  1.00 33.03           O
ATOM   1174  CB   PRO A 154      -6.614   1.494   8.576  1.00 27.86           C
ATOM   1175  CG   PRO A 154      -5.808   0.210   8.476  1.00 30.94           C
ATOM   1176  CD   PRO A 154      -6.178  -0.435   7.133  1.00 33.22           C
ATOM   1177  N    VAL A 155      -5.306   3.892   7.059  1.00 34.38           N
ATOM   1178  CA   VAL A 155      -4.006   4.540   7.013  1.00 35.85           C
ATOM   1179  C    VAL A 155      -3.648   4.982   8.420  1.00 35.04           C
ATOM   1180  O    VAL A 155      -4.525   5.310   9.219  1.00 34.51           O
ATOM   1181  CB   VAL A 155      -3.966   5.755   6.060  1.00 36.38           C
ATOM   1182  CG1  VAL A 155      -4.367   5.351   4.656  1.00 37.19           C
ATOM   1183  CG2  VAL A 155      -4.850   6.870   6.572  1.00 41.10           C
ATOM   1184  N    THR A 156      -2.358   4.966   8.730  1.00 32.78           N
ATOM   1185  CA   THR A 156      -1.887   5.503   9.994  1.00 29.21           C
ATOM   1186  C    THR A 156      -1.074   6.759   9.733  1.00 35.34           C
ATOM   1187  O    THR A 156      -0.448   6.916   8.675  1.00 29.04           O
ATOM   1188  CB   THR A 156      -1.034   4.487  10.785  1.00 34.00           C
ATOM   1189  CG2  THR A 156      -1.843   3.230  11.088  1.00 33.80           C
ATOM   1190  OG1  THR A 156       0.132   4.140  10.028  1.00 35.62           O
ATOM   1191  N    VAL A 157      -1.099   7.665  10.700  1.00 27.89           N
ATOM   1192  CA   VAL A 157      -0.375   8.916  10.582  1.00 33.47           C
ATOM   1193  C    VAL A 157       0.370   9.197  11.867  1.00 28.46           C
ATOM   1194  O    VAL A 157      -0.216   9.168  12.949  1.00 36.97           O
ATOM   1195  CB   VAL A 157      -1.319  10.100  10.276  1.00 28.34           C
ATOM   1196  CG1  VAL A 157      -0.523  11.395  10.154  1.00 28.04           C
ATOM   1197  CG2  VAL A 157      -2.120   9.833   9.011  1.00 26.05           C
ATOM   1198  N    SER A 158       1.665   9.455  11.752  1.00 30.73           N
ATOM   1199  CA   SER A 158       2.429   9.964  12.880  1.00 36.51           C
ATOM   1200  C    SER A 158       3.087  11.267  12.461  1.00 32.08           C
ATOM   1201  O    SER A 158       3.162  11.571  11.270  1.00 30.98           O
ATOM   1202  CB   SER A 158       3.476   8.951  13.347  1.00 35.17           C
ATOM   1203  OG   SER A 158       4.538   8.848  12.419  1.00 40.81           O
ATOM   1204  N    TRP A 159       3.546  12.040  13.440  1.00 27.85           N
ATOM   1205  CA   TRP A 159       4.263  13.276  13.166  1.00 30.67           C
```

222

```
ATOM   1206  C   TRP A 159      5.684  13.207  13.707  1.00 31.39           C
ATOM   1207  O   TRP A 159      5.900  12.792  14.845  1.00 30.84           O
ATOM   1208  CB  TRP A 159      3.519  14.470  13.761  1.00 28.15           C
ATOM   1209  CG  TRP A 159      2.278  14.795  13.004  1.00 35.31           C
ATOM   1210  CD1 TRP A 159      1.049  14.237  13.170  1.00 31.91           C
ATOM   1211  CD2 TRP A 159      2.149  15.745  11.942  1.00 33.46           C
ATOM   1212  CE2 TRP A 159      0.807  15.716  11.518  1.00 32.34           C
ATOM   1213  CE3 TRP A 159      3.038  16.620  11.310  1.00 33.99           C
ATOM   1214  NE1 TRP A 159      0.157  14.784  12.283  1.00 31.36           N
ATOM   1215  CZ2 TRP A 159      0.331  16.526  10.489  1.00 30.53           C
ATOM   1216  CZ3 TRP A 159      2.565  17.423  10.289  1.00 30.89           C
ATOM   1217  CH2 TRP A 159      1.223  17.372   9.890  1.00 33.28           C
ATOM   1218  N   ASN A 160      6.639  13.617  12.875  1.00 26.79           N
ATOM   1219  CA  ASN A 160      8.061  13.584  13.208  1.00 28.03           C
ATOM   1220  C   ASN A 160      8.491  12.236  13.778  1.00 33.44           C
ATOM   1221  O   ASN A 160      9.200  12.171  14.784  1.00 32.52           O
ATOM   1222  CB  ASN A 160      8.405  14.708  14.185  1.00 27.50           C
ATOM   1223  CG  ASN A 160      8.269  16.078  13.559  1.00 30.24           C
ATOM   1224  ND2 ASN A 160      8.292  17.119  14.385  1.00 34.83           N
ATOM   1225  OD1 ASN A 160      8.153  16.199  12.342  1.00 37.06           O
ATOM   1226  N   SER A 161      8.026  11.168  13.135  1.00 32.54           N
ATOM   1227  CA  SER A 161      8.394   9.801  13.492  1.00 38.63           C
ATOM   1228  C   SER A 161      7.988   9.425  14.916  1.00 37.84           C
ATOM   1229  O   SER A 161      8.601   8.556  15.530  1.00 36.85           O
ATOM   1230  CB  SER A 161      9.901   9.598  13.310  1.00 33.00           C
ATOM   1231  OG  SER A 161     10.292   9.901  11.983  1.00 36.60           O
ATOM   1232  N   GLY A 162      6.951  10.074  15.434  1.00 33.46           N
ATOM   1233  CA  GLY A 162      6.456   9.771  16.764  1.00 33.72           C
ATOM   1234  C   GLY A 162      7.005  10.686  17.843  1.00 35.30           C
ATOM   1235  O   GLY A 162      6.639  10.568  19.011  1.00 42.69           O
ATOM   1236  N   ALA A 163      7.885  11.602  17.457  1.00 37.22           N
ATOM   1237  CA  ALA A 163      8.439  12.561  18.405  1.00 36.88           C
ATOM   1238  C   ALA A 163      7.428  13.661  18.725  1.00 41.59           C
ATOM   1239  O   ALA A 163      7.524  14.318  19.762  1.00 40.49           O
ATOM   1240  CB  ALA A 163      9.725  13.165  17.865  1.00 30.59           C
ATOM   1241  N   LEU A 164      6.460  13.858  17.833  1.00 36.52           N
ATOM   1242  CA  LEU A 164      5.423  14.863  18.052  1.00 35.71           C
ATOM   1243  C   LEU A 164      4.089  14.180  18.333  1.00 36.12           C
ATOM   1244  O   LEU A 164      3.517  13.513  17.468  1.00 35.37           O
ATOM   1245  CB  LEU A 164      5.316  15.802  16.849  1.00 30.79           C
ATOM   1246  CG  LEU A 164      4.328  16.965  16.961  1.00 36.59           C
ATOM   1247  CD1 LEU A 164      4.537  17.756  18.256  1.00 36.35           C
ATOM   1248  CD2 LEU A 164      4.450  17.875  15.752  1.00 33.18           C
ATOM   1249  N   THR A 165      3.601  14.359  19.555  1.00 33.05           N
ATOM   1250  CA  THR A 165      2.442  13.623  20.048  1.00 39.48           C
ATOM   1251  C   THR A 165      1.347  14.555  20.562  1.00 39.43           C
ATOM   1252  O   THR A 165      0.164  14.353  20.285  1.00 39.07           O
ATOM   1253  CB  THR A 165      2.864  12.646  21.174  1.00 34.92           C
ATOM   1254  CG2 THR A 165      1.693  12.309  22.089  1.00 52.65           C
ATOM   1255  OG1 THR A 165      3.367  11.442  20.588  1.00 50.23           O
ATOM   1256  N   SER A 166      1.750  15.577  21.308  1.00 33.38           N
ATOM   1257  CA  SER A 166      0.804  16.495  21.930  1.00 35.48           C
ATOM   1258  C   SER A 166      0.202  17.452  20.915  1.00 34.11           C
ATOM   1259  O   SER A 166      0.920  18.061  20.123  1.00 37.38           O
ATOM   1260  CB  SER A 166      1.487  17.285  23.047  1.00 37.23           C
ATOM   1261  OG  SER A 166      2.094  16.406  23.982  1.00 58.12           O
ATOM   1262  N   GLY A 167     -1.120  17.583  20.945  1.00 36.94           N
ATOM   1263  CA  GLY A 167     -1.809  18.532  20.090  1.00 35.05           C
ATOM   1264  C   GLY A 167     -2.196  17.943  18.750  1.00 38.60           C
ATOM   1265  O   GLY A 167     -2.701  18.650  17.879  1.00 38.31           O
ATOM   1266  N   VAL A 168     -1.965  16.643  18.586  1.00 35.11           N
ATOM   1267  CA  VAL A 168     -2.262  15.968  17.328  1.00 34.52           C
ATOM   1268  C   VAL A 168     -3.690  15.438  17.297  1.00 33.72           C
ATOM   1269  O   VAL A 168     -4.145  14.801  18.242  1.00 33.06           O
ATOM   1270  CB  VAL A 168     -1.295  14.792  17.074  1.00 34.33           C
ATOM   1271  CG1 VAL A 168     -1.662  14.059  15.783  1.00 32.11           C
ATOM   1272  CG2 VAL A 168      0.141  15.285  17.027  1.00 30.55           C
ATOM   1273  N   HIS A 169     -4.392  15.712  16.203  1.00 34.59           N
ATOM   1274  CA  HIS A 169     -5.693  15.108  15.953  1.00 33.15           C
ATOM   1275  C   HIS A 169     -5.701  14.463  14.577  1.00 34.43           C
ATOM   1276  O   HIS A 169     -5.556  15.147  13.561  1.00 32.61           O
```

```
ATOM   1277  CB  HIS A 169      -6.822  16.140  16.039  1.00 33.41           C
ATOM   1278  CG  HIS A 169      -7.036  16.702  17.409  1.00 38.03           C
ATOM   1279  CD2 HIS A 169      -6.891  17.961  17.887  1.00 35.39           C
ATOM   1280  ND1 HIS A 169      -7.482  15.940  18.467  1.00 45.83           N
ATOM   1281  CE1 HIS A 169      -7.592  16.702  19.541  1.00 37.15           C
ATOM   1282  NE2 HIS A 169      -7.240  17.933  19.215  1.00 39.39           N
ATOM   1283  N   THR A 170      -5.857  13.145  14.551  1.00 33.36           N
ATOM   1284  CA  THR A 170      -6.059  12.424  13.304  1.00 31.63           C
ATOM   1285  C   THR A 170      -7.525  12.030  13.217  1.00 32.95           C
ATOM   1286  O   THR A 170      -8.032  11.288  14.056  1.00 37.08           O
ATOM   1287  CB  THR A 170      -5.168  11.178  13.200  1.00 35.35           C
ATOM   1288  CG2 THR A 170      -5.437  10.439  11.895  1.00 29.64           C
ATOM   1289  OG1 THR A 170      -3.792  11.576  13.246  1.00 35.66           O
ATOM   1290  N   PHE A 171      -8.202  12.553  12.204  1.00 30.34           N
ATOM   1291  CA  PHE A 171      -9.641  12.398  12.076  1.00 30.60           C
ATOM   1292  C   PHE A 171     -10.036  11.082  11.422  1.00 29.79           C
ATOM   1293  O   PHE A 171      -9.297  10.551  10.597  1.00 31.35           O
ATOM   1294  CB  PHE A 171     -10.214  13.574  11.286  1.00 23.31           C
ATOM   1295  CG  PHE A 171     -10.141  14.874  12.024  1.00 25.50           C
ATOM   1296  CD2 PHE A 171     -11.197  15.295  12.810  1.00 29.91           C
ATOM   1297  CD1 PHE A 171      -9.004  15.662  11.957  1.00 31.55           C
ATOM   1298  CE2 PHE A 171     -11.133  16.488  13.502  1.00 30.68           C
ATOM   1299  CE1 PHE A 171      -8.931  16.856  12.647  1.00 36.15           C
ATOM   1300  CZ  PHE A 171     -10.001  17.269  13.422  1.00 30.59           C
ATOM   1301  N   PRO A 172     -11.204  10.546  11.805  1.00 35.35           N
ATOM   1302  CA  PRO A 172     -11.781   9.388  11.117  1.00 36.50           C
ATOM   1303  C   PRO A 172     -11.928   9.670   9.627  1.00 36.86           C
ATOM   1304  O   PRO A 172     -12.265  10.794   9.253  1.00 33.48           O
ATOM   1305  CB  PRO A 172     -13.151   9.225  11.784  1.00 32.84           C
ATOM   1306  CG  PRO A 172     -12.979   9.826  13.137  1.00 36.13           C
ATOM   1307  CD  PRO A 172     -12.018  10.968  12.961  1.00 33.84           C
ATOM   1308  N   ALA A 173     -11.661   8.675   8.791  1.00 32.35           N
ATOM   1309  CA  ALA A 173     -11.783   8.850   7.349  1.00 35.00           C
ATOM   1310  C   ALA A 173     -13.237   9.021   6.930  1.00 34.52           C
ATOM   1311  O   ALA A 173     -14.132   8.415   7.516  1.00 37.71           O
ATOM   1312  CB  ALA A 173     -11.168   7.666   6.618  1.00 34.54           C
ATOM   1313  N   VAL A 174     -13.473   9.849   5.920  1.00 33.54           N
ATOM   1314  CA  VAL A 174     -14.792   9.909   5.307  1.00 33.28           C
ATOM   1315  C   VAL A 174     -14.737   9.250   3.939  1.00 35.09           C
ATOM   1316  O   VAL A 174     -13.734   9.348   3.223  1.00 34.87           O
ATOM   1317  CB  VAL A 174     -15.320  11.360   5.171  1.00 32.47           C
ATOM   1318  CG1 VAL A 174     -15.378  12.032   6.534  1.00 40.37           C
ATOM   1319  CG2 VAL A 174     -14.469  12.168   4.196  1.00 37.12           C
ATOM   1320  N   LEU A 175     -15.809   8.550   3.591  1.00 40.76           N
ATOM   1321  CA  LEU A 175     -15.921   7.932   2.280  1.00 38.53           C
ATOM   1322  C   LEU A 175     -16.501   8.941   1.300  1.00 37.30           C
ATOM   1323  O   LEU A 175     -17.605   9.447   1.494  1.00 43.25           O
ATOM   1324  CB  LEU A 175     -16.788   6.669   2.344  1.00 37.54           C
ATOM   1325  CG  LEU A 175     -16.905   5.864   1.047  1.00 40.58           C
ATOM   1326  CD1 LEU A 175     -15.529   5.519   0.504  1.00 37.07           C
ATOM   1327  CD2 LEU A 175     -17.733   4.598   1.258  1.00 43.41           C
ATOM   1328  N   GLN A 176     -15.744   9.239   0.252  1.00 44.15           N
ATOM   1329  CA  GLN A 176     -16.163  10.211  -0.746  1.00 37.45           C
ATOM   1330  C   GLN A 176     -17.095   9.569  -1.769  1.00 44.09           C
ATOM   1331  O   GLN A 176     -17.298   8.356  -1.756  1.00 44.31           O
ATOM   1332  CB  GLN A 176     -14.941  10.815  -1.439  1.00 39.61           C
ATOM   1333  CG  GLN A 176     -13.940  11.448  -0.480  1.00 41.98           C
ATOM   1334  CD  GLN A 176     -12.634  11.818  -1.160  1.00 45.48           C
ATOM   1335  NE2 GLN A 176     -11.917  10.813  -1.655  1.00 33.19           N
ATOM   1336  OE1 GLN A 176     -12.270  12.993  -1.231  1.00 52.08           O
ATOM   1337  N   SER A 177     -17.655  10.390  -2.655  1.00 43.50           N
ATOM   1338  CA  SER A 177     -18.571   9.913  -3.690  1.00 44.39           C
ATOM   1339  C   SER A 177     -17.886   8.978  -4.679  1.00 42.75           C
ATOM   1340  O   SER A 177     -18.534   8.157  -5.323  1.00 48.60           O
ATOM   1341  CB  SER A 177     -19.180  11.096  -4.443  1.00 49.14           C
ATOM   1342  OG  SER A 177     -19.961  11.895  -3.575  1.00 52.77           O
ATOM   1343  N   SER A 178     -16.571   9.116  -4.793  1.00 39.08           N
ATOM   1344  CA  SER A 178     -15.780   8.312  -5.714  1.00 38.13           C
ATOM   1345  C   SER A 178     -15.542   6.890  -5.215  1.00 39.53           C
ATOM   1346  O   SER A 178     -14.972   6.064  -5.930  1.00 46.28           O
ATOM   1347  CB  SER A 178     -14.435   8.991  -5.957  1.00 43.23           C
```

```
ATOM   1348  OG   SER A 178     -13.792    9.255   -4.720  1.00 49.71           O
ATOM   1349  N    GLY A 179     -15.960    6.608   -3.985  1.00 41.09           N
ATOM   1350  CA   GLY A 179     -15.696    5.317   -3.379  1.00 35.99           C
ATOM   1351  C    GLY A 179     -14.316    5.259   -2.746  1.00 41.20           C
ATOM   1352  O    GLY A 179     -13.882    4.210   -2.266  1.00 37.34           O
ATOM   1353  N    LEU A 180     -13.623    6.393   -2.742  1.00 37.81           N
ATOM   1354  CA   LEU A 180     -12.302    6.482   -2.127  1.00 39.78           C
ATOM   1355  C    LEU A 180     -12.370    7.247   -0.808  1.00 38.81           C
ATOM   1356  O    LEU A 180     -13.165    8.179   -0.659  1.00 34.97           O
ATOM   1357  CB   LEU A 180     -11.310    7.152   -3.082  1.00 38.31           C
ATOM   1358  CG   LEU A 180     -11.181    6.507   -4.470  1.00 38.11           C
ATOM   1359  CD1  LEU A 180     -10.351    7.381   -5.408  1.00 33.95           C
ATOM   1360  CD2  LEU A 180     -10.577    5.119   -4.359  1.00 35.65           C
ATOM   1361  N    TYR A 181     -11.534    6.849    0.147  1.00 33.32           N
ATOM   1362  CA   TYR A 181     -11.492    7.494    1.457  1.00 34.27           C
ATOM   1363  C    TYR A 181     -10.628    8.746    1.466  1.00 36.24           C
ATOM   1364  O    TYR A 181      -9.805    8.964    0.573  1.00 31.81           O
ATOM   1365  CB   TYR A 181     -10.969    6.527    2.524  1.00 29.72           C
ATOM   1366  CG   TYR A 181     -11.916    5.396    2.837  1.00 35.89           C
ATOM   1367  CD1  TYR A 181     -12.985    5.582    3.707  1.00 33.01           C
ATOM   1368  CD2  TYR A 181     -11.743    4.140    2.267  1.00 31.28           C
ATOM   1369  CE1  TYR A 181     -13.859    4.547    4.002  1.00 34.04           C
ATOM   1370  CE2  TYR A 181     -12.614    3.095    2.560  1.00 35.99           C
ATOM   1371  CZ   TYR A 181     -13.669    3.306    3.427  1.00 35.87           C
ATOM   1372  OH   TYR A 181     -14.539    2.278    3.721  1.00 42.84           O
ATOM   1373  N    SER A 182     -10.815    9.555    2.503  1.00 33.72           N
ATOM   1374  CA   SER A 182      -9.993   10.731    2.727  1.00 30.09           C
ATOM   1375  C    SER A 182      -9.965   11.071    4.214  1.00 35.24           C
ATOM   1376  O    SER A 182     -10.975   10.941    4.907  1.00 35.29           O
ATOM   1377  CB   SER A 182     -10.521   11.913    1.917  1.00 35.11           C
ATOM   1378  OG   SER A 182      -9.824   13.099    2.239  1.00 44.24           O
ATOM   1379  N    LEU A 183      -8.809   11.488    4.713  1.00 24.60           N
ATOM   1380  CA   LEU A 183      -8.745   11.992    6.077  1.00 33.42           C
ATOM   1381  C    LEU A 183      -7.713   13.095    6.223  1.00 28.32           C
ATOM   1382  O    LEU A 183      -6.855   13.293    5.365  1.00 35.46           O
ATOM   1383  CB   LEU A 183      -8.455   10.858    7.075  1.00 32.60           C
ATOM   1384  CG   LEU A 183      -7.132   10.092    7.186  1.00 38.46           C
ATOM   1385  CD1  LEU A 183      -6.003   10.910    7.815  1.00 32.89           C
ATOM   1386  CD2  LEU A 183      -7.377    8.845    8.013  1.00 35.96           C
ATOM   1387  N    SER A 184      -7.801   13.812    7.329  1.00 28.07           N
ATOM   1388  CA   SER A 184      -6.803   14.806    7.644  1.00 30.87           C
ATOM   1389  C    SER A 184      -6.202   14.512    9.009  1.00 36.19           C
ATOM   1390  O    SER A 184      -6.846   13.908    9.869  1.00 32.31           O
ATOM   1391  CB   SER A 184      -7.411   16.198    7.619  1.00 27.57           C
ATOM   1392  OG   SER A 184      -8.437   16.292    8.586  1.00 37.02           O
ATOM   1393  N    SER A 185      -4.958   14.930    9.190  1.00 29.68           N
ATOM   1394  CA   SER A 185      -4.309   14.883   10.487  1.00 27.58           C
ATOM   1395  C    SER A 185      -3.733   16.254   10.747  1.00 29.16           C
ATOM   1396  O    SER A 185      -3.023   16.799    9.908  1.00 34.94           O
ATOM   1397  CB   SER A 185      -3.211   13.822   10.533  1.00 29.89           C
ATOM   1398  OG   SER A 185      -2.506   13.889   11.762  1.00 33.01           O
ATOM   1399  N    VAL A 186      -4.051   16.825   11.898  1.00 31.07           N
ATOM   1400  CA   VAL A 186      -3.573   18.158   12.209  1.00 32.17           C
ATOM   1401  C    VAL A 186      -2.863   18.166   13.545  1.00 30.53           C
ATOM   1402  O    VAL A 186      -3.002   17.244   14.347  1.00 29.86           O
ATOM   1403  CB   VAL A 186      -4.719   19.179   12.231  1.00 30.64           C
ATOM   1404  CG1  VAL A 186      -5.443   19.180   10.890  1.00 27.44           C
ATOM   1405  CG2  VAL A 186      -5.684   18.868   13.369  1.00 31.93           C
ATOM   1406  N    VAL A 187      -2.082   19.211   13.765  1.00 28.24           N
ATOM   1407  CA   VAL A 187      -1.402   19.397   15.028  1.00 29.57           C
ATOM   1408  C    VAL A 187      -1.341   20.894   15.298  1.00 33.56           C
ATOM   1409  O    VAL A 187      -1.156   21.689   14.377  1.00 33.65           O
ATOM   1410  CB   VAL A 187       0.013   18.764   15.013  1.00 32.42           C
ATOM   1411  CG1  VAL A 187       0.854   19.335   13.875  1.00 35.03           C
ATOM   1412  CG2  VAL A 187       0.712   18.946   16.361  1.00 32.60           C
ATOM   1413  N    THR A 188      -1.553   21.282   16.549  1.00 31.74           N
ATOM   1414  CA   THR A 188      -1.405   22.674   16.943  1.00 36.08           C
ATOM   1415  C    THR A 188      -0.048   22.862   17.608  1.00 40.20           C
ATOM   1416  O    THR A 188       0.332   22.092   18.488  1.00 42.98           O
ATOM   1417  CB   THR A 188      -2.526   23.122   17.898  1.00 35.80           C
ATOM   1418  CG2  THR A 188      -3.810   23.379   17.124  1.00 36.31           C
```

```
ATOM   1419  OG1 THR A 188     -2.765  22.093  18.865  1.00 43.98          O
ATOM   1420  N   VAL A 189      0.685  23.878  17.166  1.00 34.48          N
ATOM   1421  CA  VAL A 189      2.034  24.144  17.655  1.00 36.93          C
ATOM   1422  C   VAL A 189      2.205  25.642  17.918  1.00 45.71          C
ATOM   1423  O   VAL A 189      1.428  26.453  17.409  1.00 48.89          O
ATOM   1424  CB  VAL A 189      3.105  23.673  16.645  1.00 39.16          C
ATOM   1425  CG1 VAL A 189      2.983  22.178  16.378  1.00 36.29          C
ATOM   1426  CG2 VAL A 189      2.999  24.470  15.354  1.00 30.72          C
ATOM   1427  N   PRO A 190      3.216  26.018  18.719  1.00 45.89          N
ATOM   1428  CA  PRO A 190      3.473  27.449  18.936  1.00 50.63          C
ATOM   1429  C   PRO A 190      3.852  28.173  17.644  1.00 48.93          C
ATOM   1430  O   PRO A 190      4.637  27.643  16.858  1.00 44.98          O
ATOM   1431  CB  PRO A 190      4.645  27.450  19.924  1.00 48.82          C
ATOM   1432  CG  PRO A 190      4.553  26.131  20.620  1.00 46.27          C
ATOM   1433  CD  PRO A 190      4.067  25.170  19.574  1.00 42.43          C
ATOM   1434  N   SER A 191      3.297  29.364  17.433  1.00 48.29          N
ATOM   1435  CA  SER A 191      3.581  30.147  16.231  1.00 50.08          C
ATOM   1436  C   SER A 191      5.068  30.440  16.074  1.00 50.39          C
ATOM   1437  O   SER A 191      5.585  30.495  14.959  1.00 44.82          O
ATOM   1438  CB  SER A 191      2.802  31.463  16.249  1.00 57.90          C
ATOM   1439  OG  SER A 191      1.415  31.239  16.078  1.00 74.28          O
ATOM   1440  N   SER A 192      5.753  30.618  17.200  1.00 46.04          N
ATOM   1441  CA  SER A 192      7.169  30.965  17.193  1.00 48.53          C
ATOM   1442  C   SER A 192      8.062  29.808  16.739  1.00 53.82          C
ATOM   1443  O   SER A 192      9.241  30.009  16.445  1.00 58.88          O
ATOM   1444  CB  SER A 192      7.598  31.447  18.582  1.00 52.00          C
ATOM   1445  OG  SER A 192      7.184  30.541  19.592  1.00 49.57          O
ATOM   1446  N   SER A 193      7.507  28.601  16.671  1.00 54.17          N
ATOM   1447  CA  SER A 193      8.299  27.443  16.260  1.00 50.73          C
ATOM   1448  C   SER A 193      8.291  27.260  14.741  1.00 50.16          C
ATOM   1449  O   SER A 193      9.128  26.541  14.197  1.00 53.54          O
ATOM   1450  CB  SER A 193      7.795  26.168  16.944  1.00 44.93          C
ATOM   1451  OG  SER A 193      6.626  25.670  16.317  1.00 46.77          O
ATOM   1452  N   LEU A 194      7.349  27.907  14.060  1.00 45.24          N
ATOM   1453  CA  LEU A 194      7.292  27.842  12.602  1.00 46.01          C
ATOM   1454  C   LEU A 194      8.520  28.519  12.007  1.00 55.53          C
ATOM   1455  O   LEU A 194      8.857  29.643  12.376  1.00 62.86          O
ATOM   1456  CB  LEU A 194      6.021  28.502  12.073  1.00 39.49          C
ATOM   1457  CG  LEU A 194      4.678  28.001  12.599  1.00 46.53          C
ATOM   1458  CD1 LEU A 194      3.550  28.840  12.024  1.00 43.35          C
ATOM   1459  CD2 LEU A 194      4.476  26.535  12.261  1.00 44.15          C
ATOM   1460  N   GLY A 195      9.195  27.832  11.091  1.00 59.38          N
ATOM   1461  CA  GLY A 195     10.422  28.355  10.519  1.00 62.95          C
ATOM   1462  C   GLY A 195     11.653  27.847  11.246  1.00 59.76          C
ATOM   1463  O   GLY A 195     12.729  27.733  10.660  1.00 64.18          O
ATOM   1464  N   THR A 196     11.493  27.540  12.529  1.00 59.45          N
ATOM   1465  CA  THR A 196     12.570  26.958  13.320  1.00 58.51          C
ATOM   1466  C   THR A 196     12.448  25.434  13.387  1.00 60.43          C
ATOM   1467  O   THR A 196     13.414  24.713  13.138  1.00 61.86          O
ATOM   1468  CB  THR A 196     12.583  27.522  14.754  1.00 61.27          C
ATOM   1469  CG2 THR A 196     13.706  26.891  15.568  1.00 57.43          C
ATOM   1470  OG1 THR A 196     12.767  28.942  14.708  1.00 68.83          O
ATOM   1471  N   GLN A 197     11.254  24.954  13.725  1.00 49.07          N
ATOM   1472  CA  GLN A 197     11.009  23.525  13.894  1.00 41.26          C
ATOM   1473  C   GLN A 197     10.473  22.872  12.619  1.00 42.46          C
ATOM   1474  O   GLN A 197      9.577  23.400  11.963  1.00 40.55          O
ATOM   1475  CB  GLN A 197     10.030  23.290  15.049  1.00 44.12          C
ATOM   1476  CG  GLN A 197      9.629  21.833  15.242  1.00 47.27          C
ATOM   1477  CD  GLN A 197     10.769  20.968  15.751  1.00 57.04          C
ATOM   1478  NE2 GLN A 197     10.939  19.790  15.150  1.00 43.31          N
ATOM   1479  OE1 GLN A 197     11.490  21.354  16.673  1.00 51.73          O
ATOM   1480  N   THR A 198     11.034  21.719  12.273  1.00 41.32          N
ATOM   1481  CA  THR A 198     10.577  20.958  11.121  1.00 35.03          C
ATOM   1482  C   THR A 198      9.365  20.113  11.502  1.00 36.05          C
ATOM   1483  O   THR A 198      9.359  19.468  12.550  1.00 35.98          O
ATOM   1484  CB  THR A 198     11.705  20.058  10.565  1.00 33.49          C
ATOM   1485  CG2 THR A 198     11.153  19.010   9.620  1.00 33.33          C
ATOM   1486  OG1 THR A 198     12.644  20.870   9.848  1.00 47.14          O
ATOM   1487  N   TYR A 199      8.334  20.141  10.660  1.00 34.82          N
ATOM   1488  CA  TYR A 199      7.135  19.338  10.882  1.00 33.85          C
ATOM   1489  C   TYR A 199      6.877  18.409   9.704  1.00 31.69          C
```

| ATOM | 1490 | O | TYR A 199 | 6.644 | 18.859 | 8.582 | 1.00 34.22 | O |
| ATOM | 1491 | CB | TYR A 199 | 5.924 | 20.238 | 11.129 | 1.00 28.42 | C |
| ATOM | 1492 | CG | TYR A 199 | 6.043 | 21.030 | 12.407 | 1.00 33.72 | C |
| ATOM | 1493 | CD1 | TYR A 199 | 5.935 | 20.405 | 13.640 | 1.00 27.74 | C |
| ATOM | 1494 | CD2 | TYR A 199 | 6.288 | 22.398 | 12.385 | 1.00 27.12 | C |
| ATOM | 1495 | CE1 | TYR A 199 | 6.058 | 21.120 | 14.822 | 1.00 37.15 | C |
| ATOM | 1496 | CE2 | TYR A 199 | 6.412 | 23.122 | 13.561 | 1.00 35.98 | C |
| ATOM | 1497 | CZ | TYR A 199 | 6.294 | 22.477 | 14.776 | 1.00 33.49 | C |
| ATOM | 1498 | OH | TYR A 199 | 6.414 | 23.184 | 15.948 | 1.00 40.19 | O |
| ATOM | 1499 | N | ILE A 200 | 6.935 | 17.108 | 9.969 | 1.00 30.26 | N |
| ATOM | 1500 | CA | ILE A 200 | 6.768 | 16.097 | 8.933 | 1.00 31.27 | C |
| ATOM | 1501 | C | ILE A 200 | 5.710 | 15.089 | 9.347 | 1.00 33.53 | C |
| ATOM | 1502 | O | ILE A 200 | 5.768 | 14.546 | 10.452 | 1.00 32.98 | O |
| ATOM | 1503 | CB | ILE A 200 | 8.093 | 15.352 | 8.644 | 1.00 32.27 | C |
| ATOM | 1504 | CG1 | ILE A 200 | 9.164 | 16.333 | 8.160 | 1.00 37.70 | C |
| ATOM | 1505 | CG2 | ILE A 200 | 7.880 | 14.236 | 7.619 | 1.00 26.84 | C |
| ATOM | 1506 | CD1 | ILE A 200 | 10.495 | 15.689 | 7.860 | 1.00 36.87 | C |
| ATOM | 1507 | N | CYS A 201 | 4.739 | 14.842 | 8.470 | 1.00 28.73 | N |
| ATOM | 1508 | CA | CYS A 201 | 3.747 | 13.812 | 8.746 | 1.00 31.40 | C |
| ATOM | 1509 | C | CYS A 201 | 4.129 | 12.523 | 8.032 | 1.00 31.76 | C |
| ATOM | 1510 | O | CYS A 201 | 4.442 | 12.518 | 6.837 | 1.00 30.36 | O |
| ATOM | 1511 | CB | CYS A 201 | 2.338 | 14.264 | 8.341 | 1.00 29.40 | C |
| ATOM | 1512 | SG | CYS A 201 | 1.974 | 14.198 | 6.581 | 1.00 46.24 | S |
| ATOM | 1513 | N | ASN A 202 | 4.123 | 11.434 | 8.788 | 1.00 32.10 | N |
| ATOM | 1514 | CA | ASN A 202 | 4.479 | 10.126 | 8.262 | 1.00 32.09 | C |
| ATOM | 1515 | C | ASN A 202 | 3.216 | 9.332 | 7.990 | 1.00 30.31 | C |
| ATOM | 1516 | O | ASN A 202 | 2.476 | 8.990 | 8.908 | 1.00 34.67 | O |
| ATOM | 1517 | CB | ASN A 202 | 5.384 | 9.380 | 9.243 | 1.00 31.62 | C |
| ATOM | 1518 | CG | ASN A 202 | 6.445 | 10.276 | 9.849 | 1.00 34.57 | C |
| ATOM | 1519 | ND2 | ASN A 202 | 7.425 | 10.661 | 9.041 | 1.00 31.78 | N |
| ATOM | 1520 | OD1 | ASN A 202 | 6.382 | 10.621 | 11.030 | 1.00 37.73 | O |
| ATOM | 1521 | N | VAL A 203 | 2.958 | 9.057 | 6.722 | 1.00 31.29 | N |
| ATOM | 1522 | CA | VAL A 203 | 1.730 | 8.384 | 6.341 | 1.00 29.99 | C |
| ATOM | 1523 | C | VAL A 203 | 2.037 | 6.982 | 5.860 | 1.00 29.95 | C |
| ATOM | 1524 | O | VAL A 203 | 2.962 | 6.775 | 5.079 | 1.00 32.94 | O |
| ATOM | 1525 | CB | VAL A 203 | 0.981 | 9.163 | 5.243 | 1.00 35.04 | C |
| ATOM | 1526 | CG1 | VAL A 203 | −0.314 | 8.459 | 4.876 | 1.00 28.65 | C |
| ATOM | 1527 | CG2 | VAL A 203 | 0.710 | 10.592 | 5.707 | 1.00 26.66 | C |
| ATOM | 1528 | N | ASN A 204 | 1.264 | 6.019 | 6.345 | 1.00 29.35 | N |
| ATOM | 1529 | CA | ASN A 204 | 1.449 | 4.631 | 5.961 | 1.00 31.31 | C |
| ATOM | 1530 | C | ASN A 204 | 0.127 | 4.003 | 5.525 | 1.00 33.24 | C |
| ATOM | 1531 | O | ASN A 204 | −0.888 | 4.112 | 6.214 | 1.00 34.37 | O |
| ATOM | 1532 | CB | ASN A 204 | 2.073 | 3.843 | 7.117 | 1.00 37.45 | C |
| ATOM | 1533 | CG | ASN A 204 | 2.259 | 2.373 | 6.789 | 1.00 54.72 | C |
| ATOM | 1534 | ND2 | ASN A 204 | 3.039 | 2.091 | 5.754 | 1.00 50.83 | N |
| ATOM | 1535 | OD1 | ASN A 204 | 1.698 | 1.501 | 7.455 | 1.00 73.44 | O |
| ATOM | 1536 | N | HIS A 205 | 0.141 | 3.373 | 4.358 | 1.00 31.18 | N |
| ATOM | 1537 | CA | HIS A 205 | −1.028 | 2.675 | 3.844 | 1.00 37.56 | C |
| ATOM | 1538 | C | HIS A 205 | −0.605 | 1.286 | 3.413 | 1.00 34.89 | C |
| ATOM | 1539 | O | HIS A 205 | −0.256 | 1.068 | 2.253 | 1.00 36.52 | O |
| ATOM | 1540 | CB | HIS A 205 | −1.658 | 3.433 | 2.675 | 1.00 27.77 | C |
| ATOM | 1541 | CG | HIS A 205 | −2.917 | 2.803 | 2.155 | 1.00 35.56 | C |
| ATOM | 1542 | CD2 | HIS A 205 | −4.104 | 2.562 | 2.753 | 1.00 33.13 | C |
| ATOM | 1543 | ND1 | HIS A 205 | −3.037 | 2.344 | 0.859 | 1.00 35.44 | N |
| ATOM | 1544 | CE1 | HIS A 205 | −4.248 | 1.846 | 0.686 | 1.00 33.94 | C |
| ATOM | 1545 | NE2 | HIS A 205 | −4.919 | 1.967 | 1.815 | 1.00 29.56 | N |
| ATOM | 1546 | N | LYS A 206 | −0.623 | 0.353 | 4.358 | 1.00 34.52 | N |
| ATOM | 1547 | CA | LYS A 206 | −0.155 | −1.008 | 4.104 | 1.00 41.43 | C |
| ATOM | 1548 | C | LYS A 206 | −0.833 | −1.754 | 2.942 | 1.00 35.74 | C |
| ATOM | 1549 | O | LYS A 206 | −0.155 | −2.493 | 2.231 | 1.00 39.67 | O |
| ATOM | 1550 | CB | LYS A 206 | −0.277 | −1.846 | 5.379 | 1.00 47.00 | C |
| ATOM | 1551 | CG | LYS A 206 | 0.830 | −1.575 | 6.386 | 1.00 57.13 | C |
| ATOM | 1552 | CD | LYS A 206 | 0.895 | −2.659 | 7.452 | 1.00 81.12 | C |
| ATOM | 1553 | CE | LYS A 206 | 2.103 | −2.473 | 8.361 | 1.00 81.79 | C |
| ATOM | 1554 | NZ | LYS A 206 | 2.106 | −1.138 | 9.024 | 1.00 63.36 | N |
| ATOM | 1555 | N | PRO A 207 | −2.158 | −1.589 | 2.748 | 1.00 37.38 | N |
| ATOM | 1556 | CA | PRO A 207 | −2.759 | −2.328 | 1.624 | 1.00 32.27 | C |
| ATOM | 1557 | C | PRO A 207 | −2.137 | −2.030 | 0.252 | 1.00 38.84 | C |
| ATOM | 1558 | O | PRO A 207 | −2.064 | −2.930 | −0.578 | 1.00 37.23 | O |
| ATOM | 1559 | CB | PRO A 207 | −4.218 | −1.876 | 1.655 | 1.00 34.91 | C |
| ATOM | 1560 | CG | PRO A 207 | −4.471 | −1.559 | 3.085 | 1.00 40.39 | C |

```
ATOM   1561  CD   PRO A 207      -3.192  -0.955   3.590  1.00 32.89           C
ATOM   1562  N    SER A 208      -1.696  -0.797   0.020  1.00 38.69           N
ATOM   1563  CA   SER A 208      -1.064  -0.446  -1.250  1.00 34.52           C
ATOM   1564  C    SER A 208       0.454  -0.414  -1.115  1.00 40.84           C
ATOM   1565  O    SER A 208       1.157  -0.044  -2.056  1.00 41.36           O
ATOM   1566  CB   SER A 208      -1.565   0.911  -1.751  1.00 37.36           C
ATOM   1567  OG   SER A 208      -1.111   1.961  -0.912  1.00 32.83           O
ATOM   1568  N    ASN A 209       0.942  -0.792   0.065  1.00 38.90           N
ATOM   1569  CA   ASN A 209       2.367  -0.729   0.389  1.00 42.99           C
ATOM   1570  C    ASN A 209       2.941   0.680   0.177  1.00 43.61           C
ATOM   1571  O    ASN A 209       4.051   0.841  -0.327  1.00 47.92           O
ATOM   1572  CB   ASN A 209       3.147  -1.756  -0.441  1.00 48.09           C
ATOM   1573  CG   ASN A 209       4.502  -2.086   0.159  1.00 71.77           C
ATOM   1574  ND2  ASN A 209       5.502  -2.260  -0.700  1.00 76.92           N
ATOM   1575  OD1  ASN A 209       4.649  -2.177   1.379  1.00 76.40           O
ATOM   1576  N    THR A 210       2.174   1.692   0.575  1.00 40.46           N
ATOM   1577  CA   THR A 210       2.546   3.090   0.371  1.00 38.21           C
ATOM   1578  C    THR A 210       3.020   3.766   1.656  1.00 38.99           C
ATOM   1579  O    THR A 210       2.320   3.746   2.670  1.00 38.40           O
ATOM   1580  CB   THR A 210       1.361   3.901  -0.200  1.00 38.96           C
ATOM   1581  CG2  THR A 210       1.720   5.376  -0.316  1.00 37.56           C
ATOM   1582  OG1  THR A 210       1.006   3.395  -1.494  1.00 42.32           O
ATOM   1583  N    LYS A 211       4.214   4.353   1.607  1.00 32.28           N
ATOM   1584  CA   LYS A 211       4.716   5.195   2.693  1.00 35.66           C
ATOM   1585  C    LYS A 211       5.072   6.573   2.155  1.00 33.89           C
ATOM   1586  O    LYS A 211       5.786   6.698   1.158  1.00 32.27           O
ATOM   1587  CB   LYS A 211       5.940   4.569   3.370  1.00 36.77           C
ATOM   1588  CG   LYS A 211       5.624   3.421   4.316  1.00 45.64           C
ATOM   1589  CD   LYS A 211       6.864   2.974   5.086  1.00 52.89           C
ATOM   1590  CE   LYS A 211       7.970   2.513   4.143  1.00 58.66           C
ATOM   1591  NZ   LYS A 211       9.223   2.152   4.870  1.00 61.89           N
ATOM   1592  N    VAL A 212       4.564   7.608   2.811  1.00 27.70           N
ATOM   1593  CA   VAL A 212       4.861   8.978   2.416  1.00 34.14           C
ATOM   1594  C    VAL A 212       5.294   9.800   3.623  1.00 33.04           C
ATOM   1595  O    VAL A 212       4.657   9.748   4.677  1.00 34.67           O
ATOM   1596  CB   VAL A 212       3.640   9.659   1.741  1.00 28.22           C
ATOM   1597  CG1  VAL A 212       3.944  11.111   1.424  1.00 26.60           C
ATOM   1598  CG2  VAL A 212       3.236   8.911   0.475  1.00 33.62           C
ATOM   1599  N    ASP A 213       6.387  10.544   3.469  1.00 31.96           N
ATOM   1600  CA   ASP A 213       6.802  11.535   4.460  1.00 31.89           C
ATOM   1601  C    ASP A 213       6.622  12.935   3.886  1.00 34.24           C
ATOM   1602  O    ASP A 213       7.286  13.306   2.918  1.00 32.65           O
ATOM   1603  CB   ASP A 213       8.258  11.323   4.883  1.00 35.66           C
ATOM   1604  CG   ASP A 213       8.479   9.985   5.572  1.00 47.44           C
ATOM   1605  OD1  ASP A 213       7.659   9.616   6.445  1.00 42.96           O
ATOM   1606  OD2  ASP A 213       9.475   9.302   5.238  1.00 43.88           O
ATOM   1607  N    LYS A 214       5.728  13.708   4.492  1.00 33.17           N
ATOM   1608  CA   LYS A 214       5.361  15.016   3.967  1.00 30.01           C
ATOM   1609  C    LYS A 214       5.791  16.138   4.906  1.00 30.73           C
ATOM   1610  O    LYS A 214       5.254  16.286   6.004  1.00 29.64           O
ATOM   1611  CB   LYS A 214       3.849  15.082   3.727  1.00 28.75           C
ATOM   1612  CG   LYS A 214       3.356  16.407   3.174  1.00 34.59           C
ATOM   1613  CD   LYS A 214       3.879  16.624   1.767  1.00 33.64           C
ATOM   1614  CE   LYS A 214       3.468  17.978   1.222  1.00 44.70           C
ATOM   1615  NZ   LYS A 214       4.102  18.251  -0.100  1.00 46.18           N
ATOM   1616  N    LYS A 215       6.760  16.932   4.469  1.00 31.30           N
ATOM   1617  CA   LYS A 215       7.181  18.086   5.247  1.00 29.47           C
ATOM   1618  C    LYS A 215       6.197  19.230   5.031  1.00 34.25           C
ATOM   1619  O    LYS A 215       5.887  19.587   3.895  1.00 30.95           O
ATOM   1620  CB   LYS A 215       8.597  18.521   4.870  1.00 32.87           C
ATOM   1621  CG   LYS A 215       9.076  19.731   5.657  1.00 32.92           C
ATOM   1622  CD   LYS A 215      10.523  20.071   5.351  1.00 42.60           C
ATOM   1623  CE   LYS A 215      10.950  21.339   6.087  1.00 41.79           C
ATOM   1624  NZ   LYS A 215      12.411  21.587   5.969  1.00 52.69           N
ATOM   1625  N    VAL A 216       5.697  19.798   6.121  1.00 27.91           N
ATOM   1626  CA   VAL A 216       4.736  20.883   6.011  1.00 31.50           C
ATOM   1627  C    VAL A 216       5.346  22.189   6.512  1.00 35.76           C
ATOM   1628  O    VAL A 216       5.803  22.282   7.654  1.00 31.10           O
ATOM   1629  CB   VAL A 216       3.443  20.567   6.782  1.00 32.04           C
ATOM   1630  CG1  VAL A 216       2.447  21.716   6.650  1.00 29.56           C
ATOM   1631  CG2  VAL A 216       2.833  19.265   6.271  1.00 28.39           C
```

```
ATOM   1632  N   GLU A 217      5.357  23.187   5.634  1.00 39.92          N
ATOM   1633  CA  GLU A 217      5.937  24.497   5.924  1.00 46.47          C
ATOM   1634  C   GLU A 217      4.917  25.604   5.714  1.00 44.08          C
ATOM   1635  O   GLU A 217      3.980  25.443   4.933  1.00 41.16          O
ATOM   1636  CB  GLU A 217      7.149  24.764   5.026  1.00 44.32          C
ATOM   1637  CG  GLU A 217      8.396  23.976   5.365  1.00 58.93          C
ATOM   1638  CD  GLU A 217      9.501  24.188   4.344  1.00 64.35          C
ATOM   1639  OE1 GLU A 217     10.678  24.300   4.750  1.00 75.54          O
ATOM   1640  OE2 GLU A 217      9.192  24.239   3.135  1.00 64.80          O
ATOM   1641  N   PRO A 218      5.099  26.740   6.406  1.00 48.25          N
ATOM   1642  CA  PRO A 218      4.315  27.925   6.039  1.00 50.69          C
ATOM   1643  C   PRO A 218      4.667  28.339   4.615  1.00 57.06          C
ATOM   1644  O   PRO A 218      5.822  28.177   4.218  1.00 55.63          O
ATOM   1645  CB  PRO A 218      4.757  28.979   7.060  1.00 50.18          C
ATOM   1646  CG  PRO A 218      6.087  28.500   7.561  1.00 50.76          C
ATOM   1647  CD  PRO A 218      6.027  27.005   7.521  1.00 42.82          C
ATOM   1648  N   LYS A 219      3.703  28.834   3.846  1.00 55.24          N
ATOM   1649  CA  LYS A 219      3.998  29.214   2.468  1.00 72.58          C
ATOM   1650  C   LYS A 219      4.426  30.679   2.392  1.00 77.04          C
ATOM   1651  O   LYS A 219      3.946  31.522   3.156  1.00 71.74          O
ATOM   1652  CB  LYS A 219      2.796  28.952   1.553  1.00 66.67          C
ATOM   1653  CG  LYS A 219      3.147  29.002   0.068  1.00 73.26          C
ATOM   1654  CD  LYS A 219      2.190  28.182  -0.790  1.00 78.60          C
ATOM   1655  CE  LYS A 219      0.862  28.890  -1.001  1.00 76.20          C
ATOM   1656  NZ  LYS A 219      0.010  28.168  -1.992  1.00 70.66          N
ATOM   1657  N   SER A 220      5.346  30.969   1.477  1.00 78.80          N
ATOM   1658  CA  SER A 220      5.870  32.319   1.306  1.00 86.14          C
ATOM   1659  C   SER A 220      5.245  33.004   0.096  1.00 76.66          C
ATOM   1660  O   SER A 220      4.778  32.339  -0.829  1.00 76.17          O
ATOM   1661  CB  SER A 220      7.394  32.284   1.164  1.00 88.61          C
ATOM   1662  OG  SER A 220      7.787  31.375   0.150  1.00 79.58          O
TER
ATOM   1663  N   ASP B   1    -30.071 -19.770  21.044  1.00 55.86          N
ATOM   1664  CA  ASP B   1    -28.959 -18.907  21.429  1.00 51.10          C
ATOM   1665  C   ASP B   1    -29.427 -17.792  22.359  1.00 49.42          C
ATOM   1666  O   ASP B   1    -30.475 -17.185  22.139  1.00 43.10          O
ATOM   1667  CB  ASP B   1    -28.287 -18.316  20.190  1.00 44.13          C
ATOM   1668  CG  ASP B   1    -27.652 -19.377  19.308  1.00 61.47          C
ATOM   1669  OD2 ASP B   1    -26.859 -19.009  18.415  1.00 60.15          O
ATOM   1670  OD1 ASP B   1    -27.951 -20.577  19.503  1.00 63.76          O
ATOM   1671  N   ILE B   2    -28.649 -17.533  23.404  1.00 43.87          N
ATOM   1672  CA  ILE B   2    -29.004 -16.506  24.372  1.00 42.91          C
ATOM   1673  C   ILE B   2    -28.769 -15.115  23.794  1.00 45.05          C
ATOM   1674  O   ILE B   2    -27.663 -14.787  23.359  1.00 46.40          O
ATOM   1675  CB  ILE B   2    -28.207 -16.666  25.675  1.00 39.78          C
ATOM   1676  CG1 ILE B   2    -28.476 -18.041  26.288  1.00 37.36          C
ATOM   1677  CG2 ILE B   2    -28.563 -15.558  26.659  1.00 35.94          C
ATOM   1678  CD1 ILE B   2    -27.665 -18.321  27.537  1.00 32.11          C
ATOM   1679  N   VAL B   3    -29.824 -14.306  23.783  1.00 41.92          N
ATOM   1680  CA  VAL B   3    -29.759 -12.953  23.249  1.00 38.55          C
ATOM   1681  C   VAL B   3    -29.539 -11.943  24.372  1.00 39.90          C
ATOM   1682  O   VAL B   3    -30.258 -11.945  25.372  1.00 40.51          O
ATOM   1683  CB  VAL B   3    -31.042 -12.599  22.476  1.00 39.53          C
ATOM   1684  CG1 VAL B   3    -30.987 -11.169  21.965  1.00 34.86          C
ATOM   1685  CG2 VAL B   3    -31.241 -13.570  21.324  1.00 38.27          C
ATOM   1686  N   MET B   4    -28.528 -11.094  24.214  1.00 35.54          N
ATOM   1687  CA  MET B   4    -28.248 -10.063  25.208  1.00 37.31          C
ATOM   1688  C   MET B   4    -28.677  -8.695  24.687  1.00 34.89          C
ATOM   1689  O   MET B   4    -28.362  -8.327  23.559  1.00 35.92          O
ATOM   1690  CB  MET B   4    -26.760 -10.043  25.570  1.00 29.00          C
ATOM   1691  CG  MET B   4    -26.173 -11.410  25.886  1.00 32.64          C
ATOM   1692  SD  MET B   4    -26.854 -12.116  27.401  1.00 36.18          S
ATOM   1693  CE  MET B   4    -26.247 -10.945  28.614  1.00 29.96          C
ATOM   1694  N   THR B   5    -29.405  -7.953  25.510  1.00 36.75          N
ATOM   1695  CA  THR B   5    -29.788  -6.591  25.170  1.00 36.17          C
ATOM   1696  C   THR B   5    -29.273  -5.640  26.238  1.00 38.85          C
ATOM   1697  O   THR B   5    -29.080  -6.029  27.392  1.00 40.10          O
ATOM   1698  CB  THR B   5    -31.315  -6.436  25.040  1.00 33.68          C
ATOM   1699  CG2 THR B   5    -31.853  -7.309  23.910  1.00 30.80          C
ATOM   1700  OG1 THR B   5    -31.938  -6.816  26.270  1.00 36.62          O
ATOM   1701  N   GLN B   6    -29.038  -4.394  25.848  1.00 33.36          N
```

| ATOM | 1702 | CA | GLN | B | 6 | -28.602 | -3.376 | 26.788 | 1.00 | 33.06 | C |
|------|------|-----|-----|---|----|---------|--------|--------|------|-------|---|
| ATOM | 1703 | C | GLN | B | 6 | -29.438 | -2.119 | 26.620 | 1.00 | 31.88 | C |
| ATOM | 1704 | O | GLN | B | 6 | -30.040 | -1.899 | 25.571 | 1.00 | 35.73 | O |
| ATOM | 1705 | CB | GLN | B | 6 | -27.116 | -3.048 | 26.599 | 1.00 | 33.60 | C |
| ATOM | 1706 | CG | GLN | B | 6 | -26.169 | -4.214 | 26.870 | 1.00 | 36.92 | C |
| ATOM | 1707 | CD | GLN | B | 6 | -24.721 | -3.883 | 26.533 | 1.00 | 38.24 | C |
| ATOM | 1708 | NE2 | GLN | B | 6 | -24.272 | -2.702 | 26.941 | 1.00 | 30.52 | N |
| ATOM | 1709 | OE1 | GLN | B | 6 | -24.017 | -4.682 | 25.915 | 1.00 | 36.39 | O |
| ATOM | 1710 | N | SER | B | 7 | -29.470 | -1.302 | 27.665 | 1.00 | 32.38 | N |
| ATOM | 1711 | CA | SER | B | 7 | -30.103 | 0.006 | 27.606 | 1.00 | 37.23 | C |
| ATOM | 1712 | C | SER | B | 7 | -29.416 | 0.933 | 28.601 | 1.00 | 36.04 | C |
| ATOM | 1713 | O | SER | B | 7 | -28.976 | 0.485 | 29.662 | 1.00 | 39.38 | O |
| ATOM | 1714 | CB | SER | B | 7 | -31.602 | -0.096 | 27.901 | 1.00 | 37.67 | C |
| ATOM | 1715 | OG | SER | B | 7 | -31.826 | -0.591 | 29.208 | 1.00 | 50.24 | O |
| ATOM | 1716 | N | PRO | B | 8 | -29.296 | 2.226 | 28.258 | 1.00 | 38.41 | N |
| ATOM | 1717 | CA | PRO | B | 8 | -29.712 | 2.835 | 26.989 | 1.00 | 38.28 | C |
| ATOM | 1718 | C | PRO | B | 8 | -28.708 | 2.568 | 25.872 | 1.00 | 44.42 | C |
| ATOM | 1719 | O | PRO | B | 8 | -27.687 | 1.924 | 26.117 | 1.00 | 41.31 | O |
| ATOM | 1720 | CB | PRO | B | 8 | -29.766 | 4.326 | 27.325 | 1.00 | 41.32 | C |
| ATOM | 1721 | CG | PRO | B | 8 | -28.725 | 4.492 | 28.383 | 1.00 | 37.24 | C |
| ATOM | 1722 | CD | PRO | B | 8 | -28.754 | 3.226 | 29.197 | 1.00 | 31.36 | C |
| ATOM | 1723 | N | ASP | B | 9 | -28.994 | 3.049 | 24.667 | 1.00 | 42.66 | N |
| ATOM | 1724 | CA | ASP | B | 9 | -28.037 | 2.952 | 23.570 | 1.00 | 44.47 | C |
| ATOM | 1725 | C | ASP | B | 9 | -26.850 | 3.863 | 23.842 | 1.00 | 46.92 | C |
| ATOM | 1726 | O | ASP | B | 9 | -25.699 | 3.503 | 23.588 | 1.00 | 38.54 | O |
| ATOM | 1727 | CB | ASP | B | 9 | -28.687 | 3.322 | 22.236 | 1.00 | 44.64 | C |
| ATOM | 1728 | CG | ASP | B | 9 | -29.773 | 2.349 | 21.827 | 1.00 | 70.41 | C |
| ATOM | 1729 | OD1 | ASP | B | 9 | -29.730 | 1.181 | 22.272 | 1.00 | 74.55 | O |
| ATOM | 1730 | OD2 | ASP | B | 9 | -30.671 | 2.753 | 21.057 | 1.00 | 80.56 | O |
| ATOM | 1731 | N | SER | B | 10 | -27.150 | 5.044 | 24.371 | 1.00 | 42.57 | N |
| ATOM | 1732 | CA | SER | B | 10 | -26.146 | 6.061 | 24.628 | 1.00 | 39.96 | C |
| ATOM | 1733 | C | SER | B | 10 | -26.395 | 6.721 | 25.978 | 1.00 | 42.60 | C |
| ATOM | 1734 | O | SER | B | 10 | -27.522 | 6.742 | 26.475 | 1.00 | 43.39 | O |
| ATOM | 1735 | CB | SER | B | 10 | -26.149 | 7.110 | 23.515 | 1.00 | 29.15 | C |
| ATOM | 1736 | OG | SER | B | 10 | -25.012 | 7.943 | 23.611 | 1.00 | 55.37 | O |
| ATOM | 1737 | N | LEU | B | 11 | -25.342 | 7.286 | 26.553 | 1.00 | 38.86 | N |
| ATOM | 1738 | CA | LEU | B | 11 | -25.384 | 7.756 | 27.927 | 1.00 | 39.26 | C |
| ATOM | 1739 | C | LEU | B | 11 | -24.310 | 8.819 | 28.132 | 1.00 | 33.06 | C |
| ATOM | 1740 | O | LEU | B | 11 | -23.172 | 8.636 | 27.709 | 1.00 | 43.59 | O |
| ATOM | 1741 | CB | LEU | B | 11 | -25.186 | 6.564 | 28.875 | 1.00 | 46.93 | C |
| ATOM | 1742 | CG | LEU | B | 11 | -25.354 | 6.653 | 30.391 | 1.00 | 40.86 | C |
| ATOM | 1743 | CD1 | LEU | B | 11 | -24.122 | 7.245 | 31.054 | 1.00 | 49.46 | C |
| ATOM | 1744 | CD2 | LEU | B | 11 | -26.602 | 7.452 | 30.735 | 1.00 | 58.72 | C |
| ATOM | 1745 | N | ALA | B | 12 | -24.668 | 9.930 | 28.768 | 1.00 | 34.90 | N |
| ATOM | 1746 | CA | ALA | B | 12 | -23.695 | 10.979 | 29.078 | 1.00 | 32.04 | C |
| ATOM | 1747 | C | ALA | B | 12 | -23.942 | 11.565 | 30.460 | 1.00 | 35.38 | C |
| ATOM | 1748 | O | ALA | B | 12 | -25.070 | 11.894 | 30.813 | 1.00 | 40.40 | O |
| ATOM | 1749 | CB | ALA | B | 12 | -23.733 | 12.076 | 28.027 | 1.00 | 39.73 | C |
| ATOM | 1750 | N | VAL | B | 13 | -22.874 | 11.709 | 31.235 | 1.00 | 38.02 | N |
| ATOM | 1751 | CA | VAL | B | 13 | -22.987 | 12.114 | 32.629 | 1.00 | 32.52 | C |
| ATOM | 1752 | C | VAL | B | 13 | -21.794 | 12.994 | 33.009 | 1.00 | 41.29 | C |
| ATOM | 1753 | O | VAL | B | 13 | -20.707 | 12.841 | 32.450 | 1.00 | 40.66 | O |
| ATOM | 1754 | CB | VAL | B | 13 | -23.098 | 10.856 | 33.537 | 1.00 | 47.79 | C |
| ATOM | 1755 | CG1 | VAL | B | 13 | -22.187 | 10.934 | 34.752 | 1.00 | 44.21 | C |
| ATOM | 1756 | CG2 | VAL | B | 13 | -24.548 | 10.619 | 33.939 | 1.00 | 43.75 | C |
| ATOM | 1757 | N | SER | B | 14 | -21.999 | 13.933 | 33.931 | 1.00 | 41.23 | N |
| ATOM | 1758 | CA | SER | B | 14 | -20.938 | 14.857 | 34.333 | 1.00 | 36.91 | C |
| ATOM | 1759 | C | SER | B | 14 | -19.862 | 14.158 | 35.162 | 1.00 | 39.93 | C |
| ATOM | 1760 | O | SER | B | 14 | -20.125 | 13.118 | 35.766 | 1.00 | 42.44 | O |
| ATOM | 1761 | CB | SER | B | 14 | -21.528 | 16.030 | 35.121 | 1.00 | 46.50 | C |
| ATOM | 1762 | OG | SER | B | 14 | -22.570 | 16.660 | 34.393 | 1.00 | 52.97 | O |
| ATOM | 1763 | N | LEU | B | 15 | -18.656 | 14.729 | 35.182 | 1.00 | 35.81 | N |
| ATOM | 1764 | CA | LEU | B | 15 | -17.549 | 14.188 | 35.975 | 1.00 | 41.19 | C |
| ATOM | 1765 | C | LEU | B | 15 | -17.949 | 13.897 | 37.417 | 1.00 | 46.73 | C |
| ATOM | 1766 | O | LEU | B | 15 | -18.711 | 14.651 | 38.022 | 1.00 | 44.31 | O |
| ATOM | 1767 | CB | LEU | B | 15 | -16.356 | 15.148 | 35.990 | 1.00 | 42.78 | C |
| ATOM | 1768 | CG | LEU | B | 15 | -15.368 | 15.192 | 34.825 | 1.00 | 60.55 | C |
| ATOM | 1769 | CD1 | LEU | B | 15 | -15.877 | 16.103 | 33.716 | 1.00 | 58.51 | C |
| ATOM | 1770 | CD2 | LEU | B | 15 | -13.996 | 15.648 | 35.316 | 1.00 | 59.55 | C |
| ATOM | 1771 | N | GLY | B | 16 | -17.431 | 12.800 | 37.960 | 1.00 | 41.51 | N |
| ATOM | 1772 | CA | GLY | B | 16 | -17.639 | 12.466 | 39.355 | 1.00 | 38.32 | C |

| ATOM | 1773 | C | GLY | B | 16 | -19.048 | 12.012 | 39.675 | 1.00 | 37.62 | C |
|------|------|------|-----|---|----|---------|--------|--------|------|-------|---|
| ATOM | 1774 | O | GLY | B | 16 | -19.362 | 11.730 | 40.829 | 1.00 | 45.46 | O |
| ATOM | 1775 | N | GLU | B | 17 | -19.902 | 11.938 | 38.661 | 1.00 | 40.92 | N |
| ATOM | 1776 | CA | GLU | B | 17 | -21.273 | 11.496 | 38.876 | 1.00 | 41.65 | C |
| ATOM | 1777 | C | GLU | B | 17 | -21.458 | 10.032 | 38.491 | 1.00 | 45.71 | C |
| ATOM | 1778 | O | GLU | B | 17 | -20.568 | 9.404 | 37.912 | 1.00 | 38.54 | O |
| ATOM | 1779 | CB | GLU | B | 17 | -22.256 | 12.378 | 38.104 | 1.00 | 42.09 | C |
| ATOM | 1780 | CG | GLU | B | 17 | -22.365 | 13.791 | 38.659 | 1.00 | 48.23 | C |
| ATOM | 1781 | CD | GLU | B | 17 | -23.478 | 14.596 | 38.011 | 1.00 | 66.55 | C |
| ATOM | 1782 | OE1 | GLU | B | 17 | -23.909 | 14.234 | 36.894 | 1.00 | 71.11 | O |
| ATOM | 1783 | OE2 | GLU | B | 17 | -23.923 | 15.591 | 38.624 | 1.00 | 74.03 | O |
| ATOM | 1784 | N | ARG | B | 18 | -22.627 | 9.500 | 38.823 | 1.00 | 45.65 | N |
| ATOM | 1785 | CA | ARG | B | 18 | -22.899 | 8.077 | 38.701 | 1.00 | 45.58 | C |
| ATOM | 1786 | C | ARG | B | 18 | -23.508 | 7.729 | 37.346 | 1.00 | 40.92 | C |
| ATOM | 1787 | O | ARG | B | 18 | -24.379 | 8.436 | 36.843 | 1.00 | 38.35 | O |
| ATOM | 1788 | CB | ARG | B | 18 | -23.826 | 7.638 | 39.838 | 1.00 | 43.55 | C |
| ATOM | 1789 | CG | ARG | B | 18 | -24.162 | 6.165 | 39.879 | 1.00 | 54.20 | C |
| ATOM | 1790 | CD | ARG | B | 18 | -24.969 | 5.853 | 41.132 | 1.00 | 66.41 | C |
| ATOM | 1791 | NE | ARG | B | 18 | -25.394 | 4.459 | 41.181 | 1.00 | 84.38 | N |
| ATOM | 1792 | CZ | ARG | B | 18 | -26.076 | 3.921 | 42.186 | 1.00 | 83.62 | C |
| ATOM | 1793 | NH1 | ARG | B | 18 | -26.412 | 4.662 | 43.234 | 1.00 | 81.35 | N |
| ATOM | 1794 | NH2 | ARG | B | 18 | -26.420 | 2.640 | 42.142 | 1.00 | 85.37 | N |
| ATOM | 1795 | N | ALA | B | 19 | -23.030 | 6.643 | 36.751 | 1.00 | 35.20 | N |
| ATOM | 1796 | CA | ALA | B | 19 | -23.584 | 6.161 | 35.491 | 1.00 | 34.46 | C |
| ATOM | 1797 | C | ALA | B | 19 | -23.980 | 4.696 | 35.617 | 1.00 | 39.57 | C |
| ATOM | 1798 | O | ALA | B | 19 | -23.276 | 3.904 | 36.247 | 1.00 | 38.45 | O |
| ATOM | 1799 | CB | ALA | B | 19 | -22.588 | 6.351 | 34.356 | 1.00 | 36.85 | C |
| ATOM | 1800 | N | THR | B | 20 | -25.105 | 4.344 | 35.004 | 1.00 | 33.06 | N |
| ATOM | 1801 | CA | THR | B | 20 | -25.654 | 3.001 | 35.107 | 1.00 | 35.98 | C |
| ATOM | 1802 | C | THR | B | 20 | -26.066 | 2.452 | 33.742 | 1.00 | 38.75 | C |
| ATOM | 1803 | O | THR | B | 20 | -26.762 | 3.118 | 32.979 | 1.00 | 37.55 | O |
| ATOM | 1804 | CB | THR | B | 20 | -26.866 | 2.987 | 36.062 | 1.00 | 38.02 | C |
| ATOM | 1805 | CG2 | THR | B | 20 | -27.718 | 1.757 | 35.842 | 1.00 | 44.22 | C |
| ATOM | 1806 | OG1 | THR | B | 20 | -26.398 | 3.008 | 37.419 | 1.00 | 45.86 | O |
| ATOM | 1807 | N | ILE | B | 21 | -25.622 | 1.235 | 33.441 | 1.00 | 37.58 | N |
| ATOM | 1808 | CA | ILE | B | 21 | -25.973 | 0.559 | 32.196 | 1.00 | 35.14 | C |
| ATOM | 1809 | C | ILE | B | 21 | -26.661 | -0.765 | 32.507 | 1.00 | 37.66 | C |
| ATOM | 1810 | O | ILE | B | 21 | -26.193 | -1.527 | 33.350 | 1.00 | 32.81 | O |
| ATOM | 1811 | CB | ILE | B | 21 | -24.733 | 0.284 | 31.326 | 1.00 | 38.29 | C |
| ATOM | 1812 | CG1 | ILE | B | 21 | -23.964 | 1.573 | 31.057 | 1.00 | 37.46 | C |
| ATOM | 1813 | CG2 | ILE | B | 21 | -25.127 | -0.395 | 30.018 | 1.00 | 45.60 | C |
| ATOM | 1814 | CD1 | ILE | B | 21 | -22.717 | 1.348 | 30.238 | 1.00 | 41.15 | C |
| ATOM | 1815 | N | ASN | B | 22 | -27.761 | -1.046 | 31.819 | 1.00 | 30.24 | N |
| ATOM | 1816 | CA | ASN | B | 22 | -28.521 | -2.260 | 32.084 | 1.00 | 37.10 | C |
| ATOM | 1817 | C | ASN | B | 22 | -28.270 | -3.358 | 31.050 | 1.00 | 40.28 | C |
| ATOM | 1818 | O | ASN | B | 22 | -28.041 | -3.081 | 29.870 | 1.00 | 35.19 | O |
| ATOM | 1819 | CB | ASN | B | 22 | -30.013 | -1.932 | 32.157 | 1.00 | 36.20 | C |
| ATOM | 1820 | CG | ASN | B | 22 | -30.308 | -0.788 | 33.121 | 1.00 | 60.60 | C |
| ATOM | 1821 | OD1 | ASN | B | 22 | -30.309 | -0.974 | 34.341 | 1.00 | 57.11 | O |
| ATOM | 1822 | ND2 | ASN | B | 22 | -30.547 | 0.407 | 32.576 | 1.00 | 55.17 | N |
| ATOM | 1823 | N | CYS | B | 23 | -28.315 | -4.604 | 31.509 | 1.00 | 36.70 | N |
| ATOM | 1824 | CA | CYS | B | 23 | -28.121 | -5.762 | 30.647 | 1.00 | 34.30 | C |
| ATOM | 1825 | C | CYS | B | 23 | -29.231 | -6.791 | 30.872 | 1.00 | 38.13 | C |
| ATOM | 1826 | O | CYS | B | 23 | -29.570 | -7.107 | 32.012 | 1.00 | 40.38 | O |
| ATOM | 1827 | CB | CYS | B | 23 | -26.748 | -6.391 | 30.913 | 1.00 | 37.80 | C |
| ATOM | 1828 | SG | CYS | B | 23 | -26.222 | -7.657 | 29.735 | 1.00 | 53.67 | S |
| ATOM | 1829 | N | LYS | B | 24 | -29.794 | -7.312 | 29.786 | 1.00 | 33.98 | N |
| ATOM | 1830 | CA | LYS | B | 24 | -30.813 | -8.356 | 29.871 | 1.00 | 36.97 | C |
| ATOM | 1831 | C | LYS | B | 24 | -30.440 | -9.564 | 29.024 | 1.00 | 42.52 | C |
| ATOM | 1832 | O | LYS | B | 24 | -29.979 | -9.419 | 27.890 | 1.00 | 38.82 | O |
| ATOM | 1833 | CB | LYS | B | 24 | -32.181 | -7.831 | 29.425 | 1.00 | 43.72 | C |
| ATOM | 1834 | CG | LYS | B | 24 | -33.084 | -7.356 | 30.550 | 1.00 | 55.35 | C |
| ATOM | 1835 | CD | LYS | B | 24 | -34.508 | -7.143 | 30.045 | 1.00 | 61.25 | C |
| ATOM | 1836 | CE | LYS | B | 24 | -35.433 | -6.673 | 31.155 | 1.00 | 65.56 | C |
| ATOM | 1837 | NZ | LYS | B | 24 | -35.055 | -5.323 | 31.656 | 1.00 | 61.27 | N |
| ATOM | 1838 | N | SER | B | 25 | -30.644 | -10.755 | 29.580 | 1.00 | 37.96 | N |
| ATOM | 1839 | CA | SER | B | 25 | -30.438 | -11.991 | 28.835 | 1.00 | 41.49 | C |
| ATOM | 1840 | C | SER | B | 25 | -31.782 | -12.646 | 28.540 | 1.00 | 40.87 | C |
| ATOM | 1841 | O | SER | B | 25 | -32.677 | -12.640 | 29.379 | 1.00 | 42.24 | O |
| ATOM | 1842 | CB | SER | B | 25 | -29.524 | -12.949 | 29.603 | 1.00 | 33.20 | C |
| ATOM | 1843 | OG | SER | B | 25 | -29.974 | -13.149 | 30.931 | 1.00 | 36.26 | O |

| ATOM | 1844 | N | SER | B | 26 | -31.915 | -13.209 | 27.342 | 1.00 | 38.29 | N |
|------|------|------|------|------|------|------|------|------|------|------|------|
| ATOM | 1845 | CA | SER | B | 26 | -33.176 | -13.790 | 26.895 | 1.00 | 38.35 | C |
| ATOM | 1846 | C | SER | B | 26 | -33.605 | -14.975 | 27.759 | 1.00 | 43.66 | C |
| ATOM | 1847 | O | SER | B | 26 | -34.760 | -15.394 | 27.720 | 1.00 | 40.54 | O |
| ATOM | 1848 | CB | SER | B | 26 | -33.067 | -14.224 | 25.434 | 1.00 | 35.59 | C |
| ATOM | 1849 | OG | SER | B | 26 | -32.037 | -15.182 | 25.272 | 1.00 | 38.41 | O |
| ATOM | 1850 | N | GLN | B | 27 | -32.666 | -15.519 | 28.527 | 1.00 | 42.85 | N |
| ATOM | 1851 | CA | GLN | B | 27 | -32.979 | -16.560 | 29.498 | 1.00 | 39.30 | C |
| ATOM | 1852 | C | GLN | B | 27 | -32.004 | -16.465 | 30.660 | 1.00 | 43.02 | C |
| ATOM | 1853 | O | GLN | B | 27 | -31.011 | -15.745 | 30.581 | 1.00 | 38.87 | O |
| ATOM | 1854 | CB | GLN | B | 27 | -32.926 | -17.946 | 28.858 | 1.00 | 40.88 | C |
| ATOM | 1855 | CG | GLN | B | 27 | -31.538 | -18.387 | 28.447 | 1.00 | 44.20 | C |
| ATOM | 1856 | CD | GLN | B | 27 | -31.549 | -19.707 | 27.705 | 1.00 | 51.33 | C |
| ATOM | 1857 | NE2 | GLN | B | 27 | -31.158 | -20.776 | 28.393 | 1.00 | 42.28 | N |
| ATOM | 1858 | OE1 | GLN | B | 27 | -31.906 | -19.769 | 26.525 | 1.00 | 47.83 | O |
| ATOM | 1859 | N | SER | B | 28 | -32.291 | -17.187 | 31.738 | 1.00 | 40.20 | N |
| ATOM | 1860 | CA | SER | B | 28 | -31.473 | -17.109 | 32.941 | 1.00 | 36.91 | C |
| ATOM | 1861 | C | SER | B | 28 | -30.065 | -17.631 | 32.699 | 1.00 | 41.10 | C |
| ATOM | 1862 | O | SER | B | 28 | -29.871 | -18.659 | 32.048 | 1.00 | 36.62 | O |
| ATOM | 1863 | CB | SER | B | 28 | -32.120 | -17.889 | 34.087 | 1.00 | 41.70 | C |
| ATOM | 1864 | OG | SER | B | 28 | -31.399 | -17.700 | 35.294 | 1.00 | 42.03 | O |
| ATOM | 1865 | N | ILE | B | 29 | -29.084 | -16.906 | 33.227 | 1.00 | 36.07 | N |
| ATOM | 1866 | CA | ILE | B | 29 | -27.692 | -17.320 | 33.149 | 1.00 | 34.98 | C |
| ATOM | 1867 | C | ILE | B | 29 | -27.143 | -17.474 | 34.564 | 1.00 | 36.30 | C |
| ATOM | 1868 | O | ILE | B | 29 | -25.934 | -17.400 | 34.795 | 1.00 | 34.45 | O |
| ATOM | 1869 | CB | ILE | B | 29 | -26.846 | -16.316 | 32.344 | 1.00 | 31.71 | C |
| ATOM | 1870 | CG1 | ILE | B | 29 | -26.947 | -14.916 | 32.949 | 1.00 | 29.95 | C |
| ATOM | 1871 | CG2 | ILE | B | 29 | -27.310 | -16.276 | 30.898 | 1.00 | 29.61 | C |
| ATOM | 1872 | CD1 | ILE | B | 29 | -26.198 | -13.855 | 32.145 | 1.00 | 31.42 | C |
| ATOM | 1873 | O | LEU | B | 30 | -28.655 | -20.230 | 36.655 | 1.00 | 44.52 | O |
| ATOM | 1874 | N | LEU | B | 30 | -28.058 | -17.672 | 35.508 | 1.00 | 33.91 | N |
| ATOM | 1875 | CA | LEU | B | 30 | -27.709 | -18.044 | 36.873 | 1.00 | 39.31 | C |
| ATOM | 1876 | C | LEU | B | 30 | -27.673 | -19.563 | 36.977 | 1.00 | 38.06 | C |
| ATOM | 1877 | CB | LEU | B | 30 | -28.716 | -17.465 | 37.872 | 1.00 | 37.18 | C |
| ATOM | 1878 | CG | LEU | B | 30 | -28.589 | -17.932 | 39.327 | 1.00 | 39.11 | C |
| ATOM | 1879 | CD2 | LEU | B | 30 | -29.827 | -17.558 | 40.128 | 1.00 | 36.35 | C |
| ATOM | 1880 | CD1 | LEU | B | 30 | -27.331 | -17.378 | 39.987 | 1.00 | 36.12 | C |
| ATOM | 1881 | O | HIS | B | 31 | -26.681 | -21.536 | 39.965 | 1.00 | 38.61 | O |
| ATOM | 1882 | N | HIS | B | 31 | -26.546 | -20.117 | 37.411 | 1.00 | 33.48 | N |
| ATOM | 1883 | CA | HIS | B | 31 | -26.464 | -21.562 | 37.580 | 1.00 | 38.93 | C |
| ATOM | 1884 | C | HIS | B | 31 | -27.113 | -21.968 | 38.897 | 1.00 | 39.33 | C |
| ATOM | 1885 | CB | HIS | B | 31 | -25.018 | -22.046 | 37.533 | 1.00 | 38.09 | C |
| ATOM | 1886 | CG | HIS | B | 31 | -24.889 | -23.538 | 37.446 | 1.00 | 39.11 | C |
| ATOM | 1887 | ND1 | HIS | B | 31 | -24.604 | -24.190 | 36.272 | 1.00 | 42.93 | N |
| ATOM | 1888 | CD2 | HIS | B | 31 | -25.028 | -24.496 | 38.394 | 1.00 | 37.40 | C |
| ATOM | 1889 | CE1 | HIS | B | 31 | -24.560 | -25.497 | 36.497 | 1.00 | 40.76 | C |
| ATOM | 1890 | NE2 | HIS | B | 31 | -24.816 | -25.705 | 37.772 | 1.00 | 41.52 | N |
| ATOM | 1891 | O | SER | B | 32 | -28.568 | -23.745 | 42.260 | 1.00 | 57.25 | O |
| ATOM | 1892 | N | SER | B | 32 | -28.140 | -22.809 | 38.812 | 1.00 | 43.81 | N |
| ATOM | 1893 | CA | SER | B | 32 | -28.969 | -23.149 | 39.972 | 1.00 | 49.61 | C |
| ATOM | 1894 | C | SER | B | 32 | -28.209 | -23.866 | 41.089 | 1.00 | 43.17 | C |
| ATOM | 1895 | CB | SER | B | 32 | -30.159 | -24.004 | 39.530 | 1.00 | 47.25 | C |
| ATOM | 1896 | OG | SER | B | 32 | -29.731 | -25.115 | 38.758 | 1.00 | 56.93 | O |
| ATOM | 1897 | O | SER | B | 33 | -25.191 | -24.612 | 43.655 | 1.00 | 47.19 | O |
| ATOM | 1898 | N | SER | B | 33 | -27.155 | -24.596 | 40.737 | 1.00 | 40.34 | N |
| ATOM | 1899 | CA | SER | B | 33 | -26.407 | -25.373 | 41.729 | 1.00 | 50.61 | C |
| ATOM | 1900 | C | SER | B | 33 | -25.300 | -24.578 | 42.428 | 1.00 | 46.45 | C |
| ATOM | 1901 | CB | SER | B | 33 | -25.807 | -26.621 | 41.079 | 1.00 | 46.34 | C |
| ATOM | 1902 | OG | SER | B | 33 | -26.825 | -27.469 | 40.581 | 1.00 | 54.71 | O |
| ATOM | 1903 | O | ASN | B | 34 | -22.973 | -20.977 | 43.235 | 1.00 | 37.27 | O |
| ATOM | 1904 | N | ASN | B | 34 | -24.470 | -23.875 | 41.661 | 1.00 | 42.95 | N |
| ATOM | 1905 | CA | ASN | B | 34 | -23.365 | -23.137 | 42.269 | 1.00 | 42.26 | C |
| ATOM | 1906 | C | ASN | B | 34 | -23.735 | -21.686 | 42.578 | 1.00 | 34.30 | C |
| ATOM | 1907 | CB | ASN | B | 34 | -22.112 | -23.195 | 41.378 | 1.00 | 39.81 | C |
| ATOM | 1908 | CG | ASN | B | 34 | -22.306 | -22.519 | 40.027 | 1.00 | 38.01 | C |
| ATOM | 1909 | OD1 | ASN | B | 34 | -22.552 | -21.313 | 39.949 | 1.00 | 35.29 | O |
| ATOM | 1910 | ND2 | ASN | B | 34 | -22.163 | -23.292 | 38.952 | 1.00 | 32.57 | N |
| ATOM | 1911 | O | ASN | B | 35 | -24.573 | -17.694 | 42.495 | 1.00 | 38.12 | O |
| ATOM | 1912 | N | ASN | B | 35 | -24.900 | -21.256 | 42.095 | 1.00 | 36.64 | N |
| ATOM | 1913 | CA | ASN | B | 35 | -25.455 | -19.936 | 42.414 | 1.00 | 41.38 | C |
| ATOM | 1914 | C | ASN | B | 35 | -24.612 | -18.762 | 41.888 | 1.00 | 38.67 | C |

```
ATOM   1915  CB   ASN B  35   -25.650 -19.805  43.936  1.00 38.07           C
ATOM   1916  CG   ASN B  35   -26.644 -18.719  44.315  1.00 52.73           C
ATOM   1917  OD1  ASN B  35   -27.611 -18.462  43.594  1.00 51.71           O
ATOM   1918  ND2  ASN B  35   -26.403 -18.069  45.452  1.00 46.79           N
ATOM   1919  O    ASN B  36   -24.385 -18.267  38.068  1.00 37.24           O
ATOM   1920  N    ASN B  36   -23.947 -18.955  40.752  1.00 32.67           N
ATOM   1921  CA   ASN B  36   -23.207 -17.863  40.120  1.00 35.30           C
ATOM   1922  C    ASN B  36   -23.838 -17.439  38.801  1.00 30.07           C
ATOM   1923  CB   ASN B  36   -21.743 -18.252  39.888  1.00 30.99           C
ATOM   1924  CG   ASN B  36   -20.922 -18.234  41.165  1.00 39.65           C
ATOM   1925  OD1  ASN B  36   -20.340 -17.210  41.526  1.00 36.59           O
ATOM   1926  ND2  ASN B  36   -20.870 -19.369  41.854  1.00 34.78           N
ATOM   1927  O    ASN B  37   -22.048 -14.928  36.474  1.00 26.67           O
ATOM   1928  N    ASN B  37   -23.761 -16.144  38.510  1.00 28.56           N
ATOM   1929  CA   ASN B  37   -24.222 -15.603  37.237  1.00 32.35           C
ATOM   1930  C    ASN B  37   -23.090 -15.540  36.217  1.00 35.69           C
ATOM   1931  CB   ASN B  37   -24.817 -14.214  37.437  1.00 33.21           C
ATOM   1932  CG   ASN B  37   -25.996 -14.219  38.386  1.00 35.83           C
ATOM   1933  OD1  ASN B  37   -25.854 -13.922  39.573  1.00 39.01           O
ATOM   1934  ND2  ASN B  37   -27.166 -14.566  37.868  1.00 28.90           N
ATOM   1935  N    TYR B  38   -23.306 -16.156  35.059  1.00 26.80           N
ATOM   1936  CA   TYR B  38   -22.251 -16.313  34.063  1.00 31.80           C
ATOM   1937  C    TYR B  38   -22.234 -15.119  33.118  1.00 29.97           C
ATOM   1938  O    TYR B  38   -22.505 -15.241  31.924  1.00 29.53           O
ATOM   1939  CB   TYR B  38   -22.431 -17.628  33.295  1.00 28.25           C
ATOM   1940  CG   TYR B  38   -22.072 -18.859  34.115  1.00 31.67           C
ATOM   1941  CD2  TYR B  38   -21.149 -19.792  33.649  1.00 32.47           C
ATOM   1942  CD1  TYR B  38   -22.656 -19.083  35.358  1.00 26.38           C
ATOM   1943  CE2  TYR B  38   -20.816 -20.912  34.406  1.00 31.96           C
ATOM   1944  CE1  TYR B  38   -22.327 -20.190  36.118  1.00 30.74           C
ATOM   1945  CZ   TYR B  38   -21.414 -21.102  35.639  1.00 32.95           C
ATOM   1946  OH   TYR B  38   -21.096 -22.200  36.403  1.00 33.05           O
ATOM   1947  N    LEU B  39   -21.905 -13.963  33.680  1.00 29.02           N
ATOM   1948  CA   LEU B  39   -21.932 -12.703  32.957  1.00 26.70           C
ATOM   1949  C    LEU B  39   -20.623 -11.945  33.112  1.00 25.80           C
ATOM   1950  O    LEU B  39   -20.043 -11.912  34.202  1.00 29.17           O
ATOM   1951  CB   LEU B  39   -23.082 -11.829  33.457  1.00 30.90           C
ATOM   1952  CG   LEU B  39   -23.219 -10.510  32.698  1.00 33.97           C
ATOM   1953  CD1  LEU B  39   -24.064 -10.714  31.458  1.00 27.79           C
ATOM   1954  CD2  LEU B  39   -23.785  -9.419  33.577  1.00 33.15           C
ATOM   1955  N    ALA B  40   -20.168 -11.330  32.025  1.00 28.13           N
ATOM   1956  CA   ALA B  40   -18.989 -10.470  32.068  1.00 25.92           C
ATOM   1957  C    ALA B  40   -19.287  -9.078  31.501  1.00 26.60           C
ATOM   1958  O    ALA B  40   -20.162  -8.917  30.648  1.00 25.73           O
ATOM   1959  CB   ALA B  40   -17.836 -11.111  31.309  1.00 26.93           C
ATOM   1960  N    TRP B  41   -18.556  -8.079  31.986  1.00 26.55           N
ATOM   1961  CA   TRP B  41   -18.651  -6.720  31.455  1.00 30.35           C
ATOM   1962  C    TRP B  41   -17.325  -6.293  30.832  1.00 29.83           C
ATOM   1963  O    TRP B  41   -16.261  -6.507  31.415  1.00 26.54           O
ATOM   1964  CB   TRP B  41   -19.042  -5.723  32.549  1.00 25.43           C
ATOM   1965  CG   TRP B  41   -20.484  -5.762  32.956  1.00 30.44           C
ATOM   1966  CD1  TRP B  41   -21.014  -6.405  34.039  1.00 30.19           C
ATOM   1967  CD2  TRP B  41   -21.581  -5.116  32.297  1.00 28.45           C
ATOM   1968  CE2  TRP B  41   -22.747  -5.418  33.032  1.00 31.50           C
ATOM   1969  CE3  TRP B  41   -21.692  -4.318  31.154  1.00 33.61           C
ATOM   1970  NE1  TRP B  41   -22.372  -6.204  34.090  1.00 28.08           N
ATOM   1971  CZ2  TRP B  41   -24.008  -4.946  32.664  1.00 32.35           C
ATOM   1972  CZ3  TRP B  41   -22.947  -3.846  30.787  1.00 31.41           C
ATOM   1973  CH2  TRP B  41   -24.087  -4.163  31.541  1.00 36.76           C
ATOM   1974  N    PHE B  42   -17.400  -5.679  29.655  1.00 25.32           N
ATOM   1975  CA   PHE B  42   -16.220  -5.212  28.939  1.00 27.15           C
ATOM   1976  C    PHE B  42   -16.271  -3.715  28.680  1.00 31.54           C
ATOM   1977  O    PHE B  42   -17.338  -3.154  28.431  1.00 31.28           O
ATOM   1978  CB   PHE B  42   -16.073  -5.944  27.601  1.00 23.42           C
ATOM   1979  CG   PHE B  42   -15.772  -7.405  27.739  1.00 26.14           C
ATOM   1980  CD1  PHE B  42   -14.462  -7.847  27.845  1.00 24.13           C
ATOM   1981  CD2  PHE B  42   -16.794  -8.337  27.758  1.00 29.43           C
ATOM   1982  CE1  PHE B  42   -14.181  -9.195  27.976  1.00 28.57           C
ATOM   1983  CE2  PHE B  42   -16.518  -9.690  27.887  1.00 26.26           C
ATOM   1984  CZ   PHE B  42   -15.212 -10.116  27.997  1.00 25.92           C
ATOM   1985  N    GLN B  43   -15.109  -3.076  28.723  1.00 27.61           N
```

233

```
ATOM   1986  CA  GLN B  43   -14.976  -1.691  28.289  1.00 26.82        C
ATOM   1987  C   GLN B  43   -14.148  -1.643  27.004  1.00 32.12        C
ATOM   1988  O   GLN B  43   -13.062  -2.221  26.945  1.00 29.12        O
ATOM   1989  CB  GLN B  43   -14.317  -0.842  29.375  1.00 23.34        C
ATOM   1990  CG  GLN B  43   -14.190   0.629  29.022  1.00 24.31        C
ATOM   1991  CD  GLN B  43   -13.172   1.345  29.891  1.00 29.32        C
ATOM   1992  NE2 GLN B  43   -13.650   2.131  30.851  1.00 31.37        N
ATOM   1993  OE1 GLN B  43   -11.970   1.192  29.701  1.00 31.73        O
ATOM   1994  N   GLN B  44   -14.656  -0.977  25.972  1.00 20.12        N
ATOM   1995  CA  GLN B  44   -13.869  -0.810  24.754  1.00 26.13        C
ATOM   1996  C   GLN B  44   -13.601   0.662  24.464  1.00 26.14        C
ATOM   1997  O   GLN B  44   -14.477   1.377  23.977  1.00 29.23        O
ATOM   1998  CB  GLN B  44   -14.559  -1.470  23.556  1.00 24.46        C
ATOM   1999  CG  GLN B  44   -13.755  -1.359  22.263  1.00 26.38        C
ATOM   2000  CD  GLN B  44   -14.303  -2.228  21.137  1.00 28.28        C
ATOM   2001  NE2 GLN B  44   -13.407  -2.827  20.355  1.00 28.01        N
ATOM   2002  OE1 GLN B  44   -15.512  -2.363  20.977  1.00 28.62        O
ATOM   2003  N   LYS B  45   -12.388   1.103  24.786  1.00 27.16        N
ATOM   2004  CA  LYS B  45   -11.913   2.448  24.466  1.00 35.42        C
ATOM   2005  C   LYS B  45   -11.696   2.581  22.963  1.00 35.66        C
ATOM   2006  O   LYS B  45   -11.493   1.577  22.281  1.00 35.60        O
ATOM   2007  CB  LYS B  45   -10.602   2.752  25.205  1.00 33.99        C
ATOM   2008  CG  LYS B  45   -10.694   2.701  26.707  1.00 42.68        C
ATOM   2009  CD  LYS B  45    -9.353   3.040  27.351  1.00 41.29        C
ATOM   2010  CE  LYS B  45    -8.230   2.156  26.828  1.00 41.00        C
ATOM   2011  NZ  LYS B  45    -7.078   2.115  27.785  1.00 34.96        N
ATOM   2012  N   PRO B  46   -11.726   3.820  22.440  1.00 38.26        N
ATOM   2013  CA  PRO B  46   -11.508   4.031  21.001  1.00 37.25        C
ATOM   2014  C   PRO B  46   -10.173   3.470  20.504  1.00 36.36        C
ATOM   2015  O   PRO B  46    -9.125   3.762  21.087  1.00 35.60        O
ATOM   2016  CB  PRO B  46   -11.537   5.559  20.865  1.00 41.28        C
ATOM   2017  CG  PRO B  46   -12.362   6.021  22.009  1.00 39.45        C
ATOM   2018  CD  PRO B  46   -12.046   5.080  23.138  1.00 35.28        C
ATOM   2019  N   GLY B  47   -10.225   2.664  19.447  1.00 34.42        N
ATOM   2020  CA  GLY B  47    -9.034   2.100  18.835  1.00 31.31        C
ATOM   2021  C   GLY B  47    -8.501   0.849  19.517  1.00 42.85        C
ATOM   2022  O   GLY B  47    -7.507   0.270  19.074  1.00 30.21        O
ATOM   2023  N   GLN B  48    -9.163   0.425  20.589  1.00 29.62        N
ATOM   2024  CA  GLN B  48    -8.662  -0.672  21.411  1.00 33.47        C
ATOM   2025  C   GLN B  48    -9.588  -1.887  21.411  1.00 33.23        C
ATOM   2026  O   GLN B  48   -10.787  -1.757  21.171  1.00 26.94        O
ATOM   2027  CB  GLN B  48    -8.451  -0.187  22.850  1.00 32.23        C
ATOM   2028  CG  GLN B  48    -7.391   0.893  22.990  1.00 33.96        C
ATOM   2029  CD  GLN B  48    -6.011   0.404  22.590  1.00 43.63        C
ATOM   2030  OE1 GLN B  48    -5.276   1.095  21.885  1.00 42.98        O
ATOM   2031  NE2 GLN B  48    -5.652  -0.796  23.042  1.00 39.16        N
ATOM   2032  N   PRO B  49    -9.032  -3.077  21.691  1.00 30.89        N
ATOM   2033  CA  PRO B  49    -9.885  -4.246  21.918  1.00 26.27        C
ATOM   2034  C   PRO B  49   -10.687  -4.069  23.203  1.00 27.88        C
ATOM   2035  O   PRO B  49   -10.290  -3.264  24.045  1.00 27.50        O
ATOM   2036  CB  PRO B  49    -8.883  -5.396  22.049  1.00 26.15        C
ATOM   2037  CG  PRO B  49    -7.629  -4.740  22.538  1.00 28.99        C
ATOM   2038  CD  PRO B  49    -7.602  -3.394  21.865  1.00 32.59        C
ATOM   2039  N   PRO B  50   -11.803  -4.799  23.351  1.00 25.78        N
ATOM   2040  CA  PRO B  50   -12.539  -4.746  24.621  1.00 23.59        C
ATOM   2041  C   PRO B  50   -11.625  -5.113  25.793  1.00 30.73        C
ATOM   2042  O   PRO B  50   -10.661  -5.859  25.608  1.00 26.96        O
ATOM   2043  CB  PRO B  50   -13.643  -5.791  24.438  1.00 24.85        C
ATOM   2044  CG  PRO B  50   -13.817  -5.908  22.949  1.00 25.66        C
ATOM   2045  CD  PRO B  50   -12.441  -5.694  22.370  1.00 25.19        C
ATOM   2046  N   LYS B  51   -11.909  -4.574  26.972  1.00 27.75        N
ATOM   2047  CA  LYS B  51   -11.131  -4.899  28.158  1.00 25.94        C
ATOM   2048  C   LYS B  51   -12.052  -5.420  29.251  1.00 30.23        C
ATOM   2049  O   LYS B  51   -13.083  -4.818  29.547  1.00 25.89        O
ATOM   2050  CB  LYS B  51   -10.351  -3.680  28.654  1.00 29.25        C
ATOM   2051  CG  LYS B  51    -9.163  -4.045  29.535  1.00 32.02        C
ATOM   2052  CD  LYS B  51    -9.469  -3.859  31.001  1.00 37.80        C
ATOM   2053  CE  LYS B  51    -8.409  -4.533  31.869  1.00 37.22        C
ATOM   2054  NZ  LYS B  51    -8.655  -5.999  32.014  1.00 34.08        N
ATOM   2055  N   LEU B  52   -11.680  -6.547  29.845  1.00 24.57        N
ATOM   2056  CA  LEU B  52   -12.514  -7.177  30.860  1.00 28.11        C
```

```
ATOM   2057  C    LEU B  52     -12.555   -6.335  32.130  1.00 24.97           C
ATOM   2058  O    LEU B  52     -11.514   -5.962  32.663  1.00 30.29           O
ATOM   2059  CB   LEU B  52     -11.998   -8.583  31.172  1.00 26.30           C
ATOM   2060  CG   LEU B  52     -12.775   -9.420  32.190  1.00 32.39           C
ATOM   2061  CD1  LEU B  52     -14.222   -9.597  31.751  1.00 25.02           C
ATOM   2062  CD2  LEU B  52     -12.099  -10.772  32.378  1.00 30.83           C
ATOM   2063  N    LEU B  53     -13.760   -6.030  32.600  1.00 27.61           N
ATOM   2064  CA   LEU B  53     -13.938   -5.297  33.854  1.00 30.52           C
ATOM   2065  C    LEU B  53     -14.434   -6.219  34.956  1.00 31.29           C
ATOM   2066  O    LEU B  53     -13.913   -6.226  36.068  1.00 30.02           O
ATOM   2067  CB   LEU B  53     -14.936   -4.153  33.688  1.00 25.25           C
ATOM   2068  CG   LEU B  53     -14.711   -3.080  32.627  1.00 33.70           C
ATOM   2069  CD1  LEU B  53     -15.917   -2.149  32.611  1.00 26.80           C
ATOM   2070  CD2  LEU B  53     -13.432   -2.308  32.907  1.00 32.50           C
ATOM   2071  N    LEU B  54     -15.460   -6.993  34.631  1.00 28.48           N
ATOM   2072  CA   LEU B  54     -16.169   -7.782  35.623  1.00 30.79           C
ATOM   2073  C    LEU B  54     -16.516   -9.152  35.071  1.00 28.54           C
ATOM   2074  O    LEU B  54     -16.791   -9.294  33.883  1.00 26.65           O
ATOM   2075  CB   LEU B  54     -17.448   -7.055  36.059  1.00 31.42           C
ATOM   2076  CG   LEU B  54     -17.289   -5.722  36.798  1.00 33.30           C
ATOM   2077  CD1  LEU B  54     -18.529   -4.870  36.615  1.00 32.48           C
ATOM   2078  CD2  LEU B  54     -17.052   -5.971  38.268  1.00 34.07           C
ATOM   2079  N    TYR B  55     -16.502  -10.158  35.938  1.00 29.20           N
ATOM   2080  CA   TYR B  55     -17.005  -11.475  35.580  1.00 29.74           C
ATOM   2081  C    TYR B  55     -17.755  -12.043  36.791  1.00 32.63           C
ATOM   2082  O    TYR B  55     -17.726  -11.449  37.872  1.00 31.32           O
ATOM   2083  CB   TYR B  55     -15.869  -12.393  35.101  1.00 28.86           C
ATOM   2084  CG   TYR B  55     -14.714  -12.558  36.065  1.00 30.07           C
ATOM   2085  CD1  TYR B  55     -13.746  -11.572  36.204  1.00 31.15           C
ATOM   2086  CD2  TYR B  55     -14.578  -13.717  36.814  1.00 30.26           C
ATOM   2087  CE1  TYR B  55     -12.686  -11.728  37.088  1.00 34.78           C
ATOM   2088  CE2  TYR B  55     -13.525  -13.886  37.695  1.00 33.93           C
ATOM   2089  CZ   TYR B  55     -12.582  -12.889  37.828  1.00 36.44           C
ATOM   2090  OH   TYR B  55     -11.534  -13.059  38.705  1.00 37.42           O
ATOM   2091  N    TRP B  56     -18.448  -13.165  36.599  1.00 28.09           N
ATOM   2092  CA   TRP B  56     -19.439  -13.657  37.566  1.00 26.67           C
ATOM   2093  C    TRP B  56     -20.387  -12.528  37.984  1.00 27.89           C
ATOM   2094  O    TRP B  56     -20.767  -12.420  39.151  1.00 24.63           O
ATOM   2095  CB   TRP B  56     -18.769  -14.276  38.799  1.00 22.01           C
ATOM   2096  CG   TRP B  56     -17.799  -15.367  38.462  1.00 27.09           C
ATOM   2097  CD1  TRP B  56     -16.475  -15.402  38.777  1.00 26.84           C
ATOM   2098  CD2  TRP B  56     -18.068  -16.569  37.720  1.00 23.97           C
ATOM   2099  NE1  TRP B  56     -15.901  -16.551  38.286  1.00 28.86           N
ATOM   2100  CE2  TRP B  56     -16.857  -17.284  37.633  1.00 25.06           C
ATOM   2101  CE3  TRP B  56     -19.214  -17.112  37.131  1.00 23.47           C
ATOM   2102  CZ2  TRP B  56     -16.758  -18.514  36.983  1.00 28.72           C
ATOM   2103  CZ3  TRP B  56     -19.117  -18.334  36.486  1.00 28.66           C
ATOM   2104  CH2  TRP B  56     -17.896  -19.021  36.414  1.00 29.03           C
ATOM   2105  N    ALA B  57     -20.734  -11.686  37.009  1.00 26.41           N
ATOM   2106  CA   ALA B  57     -21.626  -10.531  37.167  1.00 29.78           C
ATOM   2107  C    ALA B  57     -21.083   -9.421  38.079  1.00 31.22           C
ATOM   2108  O    ALA B  57     -21.331   -8.242  37.817  1.00 35.13           O
ATOM   2109  CB   ALA B  57     -23.008  -10.985  37.667  1.00 27.19           C
ATOM   2110  N    SER B  58     -20.344   -9.767  39.132  1.00 32.27           N
ATOM   2111  CA   SER B  58     -19.992   -8.748  40.125  1.00 31.56           C
ATOM   2112  C    SER B  58     -18.563   -8.741  40.672  1.00 32.07           C
ATOM   2113  O    SER B  58     -18.251   -7.901  41.517  1.00 37.30           O
ATOM   2114  CB   SER B  58     -20.955   -8.849  41.315  1.00 36.25           C
ATOM   2115  OG   SER B  58     -20.817  -10.091  41.985  1.00 37.37           O
ATOM   2116  N    THR B  59     -17.687   -9.640  40.229  1.00 31.17           N
ATOM   2117  CA   THR B  59     -16.319   -9.596  40.759  1.00 29.77           C
ATOM   2118  C    THR B  59     -15.365   -8.910  39.770  1.00 30.97           C
ATOM   2119  O    THR B  59     -15.367   -9.201  38.575  1.00 31.74           O
ATOM   2120  CB   THR B  59     -15.786  -11.014  41.142  1.00 36.77           C
ATOM   2121  OG1  THR B  59     -14.721  -11.403  40.269  1.00 41.80           O
ATOM   2122  CG2  THR B  59     -16.890  -12.053  41.102  1.00 28.98           C
ATOM   2123  N    ARG B  60     -14.565   -7.978  40.284  1.00 32.62           N
ATOM   2124  CA   ARG B  60     -13.693   -7.159  39.449  1.00 36.43           C
ATOM   2125  C    ARG B  60     -12.441   -7.902  39.022  1.00 35.37           C
ATOM   2126  O    ARG B  60     -11.819   -8.588  39.825  1.00 33.08           O
ATOM   2127  CB   ARG B  60     -13.278   -5.877  40.182  1.00 33.09           C
```

| ATOM | 2128 | CG | ARG | B | 60 | -14.342 | -4.796 | 40.212 | 1.00 | 39.07 | C |
| ATOM | 2129 | CD | ARG | B | 60 | -13.752 | -3.436 | 40.558 | 1.00 | 37.50 | C |
| ATOM | 2130 | NE | ARG | B | 60 | -12.854 | -3.495 | 41.707 | 1.00 | 43.68 | N |
| ATOM | 2131 | CZ | ARG | B | 60 | -13.259 | -3.551 | 42.971 | 1.00 | 51.03 | C |
| ATOM | 2132 | NH1 | ARG | B | 60 | -14.555 | -3.560 | 43.254 | 1.00 | 53.34 | N |
| ATOM | 2133 | NH2 | ARG | B | 60 | -12.368 | -3.603 | 43.952 | 1.00 | 52.19 | N |
| ATOM | 2134 | N | GLU | B | 61 | -12.061 | -7.747 | 37.759 | 1.00 | 30.40 | N |
| ATOM | 2135 | CA | GLU | B | 61 | -10.764 | -8.235 | 37.310 | 1.00 | 35.70 | C |
| ATOM | 2136 | C | GLU | B | 61 | -9.659 | -7.523 | 38.077 | 1.00 | 35.51 | C |
| ATOM | 2137 | O | GLU | B | 61 | -9.863 | -6.424 | 38.598 | 1.00 | 36.52 | O |
| ATOM | 2138 | CB | GLU | B | 61 | -10.582 | -8.023 | 35.805 | 1.00 | 30.95 | C |
| ATOM | 2139 | CG | GLU | B | 61 | -10.406 | -9.307 | 35.015 | 1.00 | 51.01 | C |
| ATOM | 2140 | CD | GLU | B | 61 | -9.084 | -10.015 | 35.278 | 1.00 | 47.16 | C |
| ATOM | 2141 | OE1 | GLU | B | 61 | -8.884 | -10.535 | 36.397 | 1.00 | 57.21 | O |
| ATOM | 2142 | OE2 | GLU | B | 61 | -8.245 | -10.063 | 34.354 | 1.00 | 55.45 | O |
| ATOM | 2143 | N | SER | B | 62 | -8.491 | -8.148 | 38.143 | 1.00 | 36.53 | N |
| ATOM | 2144 | CA | SER | B | 62 | -7.352 | -7.559 | 38.838 | 1.00 | 37.86 | C |
| ATOM | 2145 | C | SER | B | 62 | -6.959 | -6.222 | 38.211 | 1.00 | 35.62 | C |
| ATOM | 2146 | O | SER | B | 62 | -6.919 | -6.086 | 36.992 | 1.00 | 34.27 | O |
| ATOM | 2147 | CB | SER | B | 62 | -6.166 | -8.527 | 38.822 | 1.00 | 40.55 | C |
| ATOM | 2148 | OG | SER | B | 62 | -5.113 | -8.060 | 39.646 | 1.00 | 56.20 | O |
| ATOM | 2149 | N | GLY | B | 63 | -6.690 | -5.228 | 39.049 | 1.00 | 32.40 | N |
| ATOM | 2150 | CA | GLY | B | 63 | -6.280 | -3.924 | 38.561 | 1.00 | 32.97 | C |
| ATOM | 2151 | C | GLY | B | 63 | -7.413 | -2.971 | 38.213 | 1.00 | 38.33 | C |
| ATOM | 2152 | O | GLY | B | 63 | -7.194 | -1.767 | 38.099 | 1.00 | 40.50 | O |
| ATOM | 2153 | N | VAL | B | 64 | -8.624 | -3.498 | 38.045 | 1.00 | 31.70 | N |
| ATOM | 2154 | CA | VAL | B | 64 | -9.780 | -2.664 | 37.711 | 1.00 | 30.42 | C |
| ATOM | 2155 | C | VAL | B | 64 | -10.188 | -1.782 | 38.894 | 1.00 | 34.52 | C |
| ATOM | 2156 | O | VAL | B | 64 | -10.378 | -2.279 | 40.004 | 1.00 | 36.24 | O |
| ATOM | 2157 | CB | VAL | B | 64 | -10.988 | -3.525 | 37.269 | 1.00 | 33.44 | C |
| ATOM | 2158 | CG1 | VAL | B | 64 | -12.241 | -2.668 | 37.101 | 1.00 | 31.47 | C |
| ATOM | 2159 | CG2 | VAL | B | 64 | -10.668 | -4.271 | 35.985 | 1.00 | 30.34 | C |
| ATOM | 2160 | N | PRO | B | 65 | -10.326 | -0.466 | 38.655 | 1.00 | 35.36 | N |
| ATOM | 2161 | CA | PRO | B | 65 | -10.693 | 0.500 | 39.701 | 1.00 | 33.81 | C |
| ATOM | 2162 | C | PRO | B | 65 | -12.039 | 0.170 | 40.344 | 1.00 | 36.63 | C |
| ATOM | 2163 | O | PRO | B | 65 | -12.940 | -0.301 | 39.649 | 1.00 | 30.63 | O |
| ATOM | 2164 | CB | PRO | B | 65 | -10.765 | 1.831 | 38.946 | 1.00 | 31.15 | C |
| ATOM | 2165 | CG | PRO | B | 65 | -9.947 | 1.623 | 37.710 | 1.00 | 35.40 | C |
| ATOM | 2166 | CD | PRO | B | 65 | -10.131 | 0.182 | 37.347 | 1.00 | 33.17 | C |
| ATOM | 2167 | N | ASP | B | 66 | -12.177 | 0.421 | 41.645 | 1.00 | 33.02 | N |
| ATOM | 2168 | CA | ASP | B | 66 | -13.408 | 0.069 | 42.357 | 1.00 | 39.73 | C |
| ATOM | 2169 | C | ASP | B | 66 | -14.599 | 0.965 | 41.998 | 1.00 | 39.77 | C |
| ATOM | 2170 | O | ASP | B | 66 | -15.703 | 0.752 | 42.499 | 1.00 | 35.02 | O |
| ATOM | 2171 | CB | ASP | B | 66 | -13.180 | 0.093 | 43.874 | 1.00 | 38.87 | C |
| ATOM | 2172 | CG | ASP | B | 66 | -12.527 | 1.374 | 44.354 | 1.00 | 46.60 | C |
| ATOM | 2173 | OD1 | ASP | B | 66 | -12.535 | 2.376 | 43.608 | 1.00 | 57.95 | O |
| ATOM | 2174 | OD2 | ASP | B | 66 | -12.004 | 1.377 | 45.489 | 1.00 | 64.41 | O |
| ATOM | 2175 | N | ARG | B | 67 | -14.376 | 1.953 | 41.129 | 1.00 | 35.35 | N |
| ATOM | 2176 | CA | ARG | B | 67 | -15.470 | 2.752 | 40.569 | 1.00 | 31.85 | C |
| ATOM | 2177 | C | ARG | B | 67 | -16.456 | 1.880 | 39.807 | 1.00 | 34.69 | C |
| ATOM | 2178 | O | ARG | B | 67 | -17.638 | 2.209 | 39.698 | 1.00 | 35.56 | O |
| ATOM | 2179 | CB | ARG | B | 67 | -14.947 | 3.828 | 39.614 | 1.00 | 39.18 | C |
| ATOM | 2180 | CG | ARG | B | 67 | -13.752 | 4.611 | 40.095 | 1.00 | 51.83 | C |
| ATOM | 2181 | CD | ARG | B | 67 | -13.589 | 5.861 | 39.242 | 1.00 | 57.84 | C |
| ATOM | 2182 | NE | ARG | B | 67 | -13.490 | 5.570 | 37.810 | 1.00 | 38.84 | N |
| ATOM | 2183 | CZ | ARG | B | 67 | -12.335 | 5.389 | 37.179 | 1.00 | 42.20 | C |
| ATOM | 2184 | NH1 | ARG | B | 67 | -11.197 | 5.458 | 37.859 | 1.00 | 41.09 | N |
| ATOM | 2185 | NH2 | ARG | B | 67 | -12.314 | 5.140 | 35.878 | 1.00 | 37.06 | N |
| ATOM | 2186 | N | PHE | B | 68 | -15.948 | 0.785 | 39.245 | 1.00 | 28.82 | N |
| ATOM | 2187 | CA | PHE | B | 68 | -16.759 | -0.122 | 38.443 | 1.00 | 36.23 | C |
| ATOM | 2188 | C | PHE | B | 68 | -17.314 | -1.232 | 39.308 | 1.00 | 34.50 | C |
| ATOM | 2189 | O | PHE | B | 68 | -16.564 | -1.931 | 39.995 | 1.00 | 31.34 | O |
| ATOM | 2190 | CB | PHE | B | 68 | -15.941 | -0.727 | 37.297 | 1.00 | 30.38 | C |
| ATOM | 2191 | CG | PHE | B | 68 | -15.401 | 0.287 | 36.337 | 1.00 | 34.41 | C |
| ATOM | 2192 | CD1 | PHE | B | 68 | -16.127 | 0.651 | 35.215 | 1.00 | 31.80 | C |
| ATOM | 2193 | CD2 | PHE | B | 68 | -14.163 | 0.874 | 36.551 | 1.00 | 34.95 | C |
| ATOM | 2194 | CE1 | PHE | B | 68 | -15.629 | 1.586 | 34.328 | 1.00 | 29.96 | C |
| ATOM | 2195 | CE2 | PHE | B | 68 | -13.661 | 1.807 | 35.666 | 1.00 | 35.32 | C |
| ATOM | 2196 | CZ | PHE | B | 68 | -14.396 | 2.163 | 34.554 | 1.00 | 31.86 | C |
| ATOM | 2197 | N | SER | B | 69 | -18.627 | -1.408 | 39.275 | 1.00 | 30.82 | N |
| ATOM | 2198 | CA | SER | B | 69 | -19.228 | -2.482 | 40.044 | 1.00 | 30.92 | C |

```
ATOM   2199  C    SER B  69   -20.364   -3.114  39.268  1.00 34.45           C
ATOM   2200  O    SER B  69   -20.998   -2.468  38.430  1.00 35.02           O
ATOM   2201  CB   SER B  69   -19.724   -1.970  41.397  1.00 32.49           C
ATOM   2202  OG   SER B  69   -20.797   -1.062  41.230  1.00 37.05           O
ATOM   2203  N    GLY B  70   -20.610   -4.387  39.550  1.00 30.93           N
ATOM   2204  CA   GLY B  70   -21.673   -5.116  38.896  1.00 28.03           C
ATOM   2205  C    GLY B  70   -22.689   -5.620  39.898  1.00 38.68           C
ATOM   2206  O    GLY B  70   -22.361   -5.887  41.056  1.00 36.13           O
ATOM   2207  N    SER B  71   -23.932   -5.737  39.449  1.00 35.97           N
ATOM   2208  CA   SER B  71   -24.996   -6.282  40.276  1.00 32.81           C
ATOM   2209  C    SER B  71   -26.038   -6.927  39.380  1.00 36.09           C
ATOM   2210  O    SER B  71   -25.980   -6.798  38.157  1.00 36.12           O
ATOM   2211  CB   SER B  71   -25.632   -5.193  41.136  1.00 38.54           C
ATOM   2212  OG   SER B  71   -26.284   -4.232  40.322  1.00 42.67           O
ATOM   2213  N    GLY B  72   -26.996   -7.612  39.992  1.00 35.53           N
ATOM   2214  CA   GLY B  72   -28.054   -8.256  39.242  1.00 32.26           C
ATOM   2215  C    GLY B  72   -28.025   -9.760  39.411  1.00 33.08           C
ATOM   2216  O    GLY B  72   -27.109  -10.312  40.020  1.00 37.03           O
ATOM   2217  N    SER B  73   -29.038  -10.420  38.869  1.00 35.95           N
ATOM   2218  CA   SER B  73   -29.166  -11.861  38.993  1.00 39.04           C
ATOM   2219  C    SER B  73   -30.132  -12.395  37.946  1.00 40.07           C
ATOM   2220  O    SER B  73   -31.042  -11.691  37.512  1.00 40.54           O
ATOM   2221  CB   SER B  73   -29.643  -12.235  40.398  1.00 44.78           C
ATOM   2222  OG   SER B  73   -29.753  -13.638  40.538  1.00 50.73           O
ATOM   2223  N    GLY B  74   -29.920  -13.638  37.531  1.00 41.84           N
ATOM   2224  CA   GLY B  74   -30.816  -14.291  36.597  1.00 37.63           C
ATOM   2225  C    GLY B  74   -30.776  -13.743  35.182  1.00 41.67           C
ATOM   2226  O    GLY B  74   -29.999  -14.211  34.347  1.00 37.99           O
ATOM   2227  N    THR B  75   -31.628  -12.761  34.907  1.00 34.80           N
ATOM   2228  CA   THR B  75   -31.767  -12.239  33.553  1.00 38.56           C
ATOM   2229  C    THR B  75   -31.515  -10.733  33.475  1.00 38.78           C
ATOM   2230  O    THR B  75   -31.439  -10.173  32.384  1.00 44.01           O
ATOM   2231  CB   THR B  75   -33.176  -12.534  32.980  1.00 38.87           C
ATOM   2232  CG2  THR B  75   -33.477  -14.025  33.021  1.00 37.18           C
ATOM   2233  OG1  THR B  75   -34.163  -11.838  33.749  1.00 46.98           O
ATOM   2234  N    ASP B  76   -31.389  -10.083  34.629  1.00 37.55           N
ATOM   2235  CA   ASP B  76   -31.244   -8.628  34.678  1.00 38.55           C
ATOM   2236  C    ASP B  76   -29.989   -8.211  35.414  1.00 30.72           C
ATOM   2237  O    ASP B  76   -29.767   -8.605  36.557  1.00 39.41           O
ATOM   2238  CB   ASP B  76   -32.463   -7.978  35.340  1.00 39.46           C
ATOM   2239  CG   ASP B  76   -33.567   -7.680  34.352  1.00 54.19           C
ATOM   2240  OD2  ASP B  76   -34.444   -8.548  34.149  1.00 61.43           O
ATOM   2241  OD1  ASP B  76   -33.553   -6.575  33.770  1.00 68.17           O
ATOM   2242  N    PHE B  77   -29.173   -7.397  34.756  1.00 32.93           N
ATOM   2243  CA   PHE B  77   -27.890   -6.999  35.314  1.00 34.16           C
ATOM   2244  C    PHE B  77   -27.608   -5.540  35.021  1.00 28.32           C
ATOM   2245  O    PHE B  77   -28.054   -5.005  34.010  1.00 34.43           O
ATOM   2246  CB   PHE B  77   -26.769   -7.870  34.748  1.00 33.65           C
ATOM   2247  CG   PHE B  77   -27.008   -9.340  34.908  1.00 31.73           C
ATOM   2248  CD1  PHE B  77   -26.584  -10.004  36.050  1.00 34.33           C
ATOM   2249  CD2  PHE B  77   -27.663  -10.061  33.924  1.00 30.60           C
ATOM   2250  CE1  PHE B  77   -26.807  -11.363  36.204  1.00 28.94           C
ATOM   2251  CE2  PHE B  77   -27.892  -11.420  34.078  1.00 32.18           C
ATOM   2252  CZ   PHE B  77   -27.463  -12.067  35.215  1.00 28.31           C
ATOM   2253  N    THR B  78   -26.864   -4.894  35.909  1.00 33.91           N
ATOM   2254  CA   THR B  78   -26.454   -3.520  35.673  1.00 36.80           C
ATOM   2255  C    THR B  78   -24.972   -3.332  35.956  1.00 35.53           C
ATOM   2256  O    THR B  78   -24.429   -3.893  36.909  1.00 33.42           O
ATOM   2257  CB   THR B  78   -27.250   -2.514  36.536  1.00 36.13           C
ATOM   2258  CG2  THR B  78   -28.744   -2.790  36.465  1.00 35.39           C
ATOM   2259  OG1  THR B  78   -26.815   -2.600  37.896  1.00 52.08           O
ATOM   2260  N    LEU B  79   -24.321   -2.546  35.108  1.00 33.37           N
ATOM   2261  CA   LEU B  79   -22.978   -2.072  35.382  1.00 32.04           C
ATOM   2262  C    LEU B  79   -23.082   -0.689  36.000  1.00 35.99           C
ATOM   2263  O    LEU B  79   -23.805    0.166  35.494  1.00 30.23           O
ATOM   2264  CB   LEU B  79   -22.138   -2.030  34.105  1.00 32.75           C
ATOM   2265  CG   LEU B  79   -20.814   -1.269  34.215  1.00 31.00           C
ATOM   2266  CD1  LEU B  79   -19.875   -1.948  35.189  1.00 30.67           C
ATOM   2267  CD2  LEU B  79   -20.151   -1.129  32.853  1.00 29.83           C
ATOM   2268  N    THR B  80   -22.379   -0.467  37.102  1.00 37.73           N
ATOM   2269  CA   THR B  80   -22.390    0.852  37.711  1.00 37.01           C
```

| ATOM | 2270 | C | THR | B | 80 | -20.999 | 1.468 | 37.726 | 1.00 | 37.89 | C |
| ATOM | 2271 | O | THR | B | 80 | -20.044 | 0.876 | 38.229 | 1.00 | 33.52 | O |
| ATOM | 2272 | CB | THR | B | 80 | -22.938 | 0.816 | 39.152 | 1.00 | 47.22 | C |
| ATOM | 2273 | CG2 | THR | B | 80 | -22.815 | 2.194 | 39.802 | 1.00 | 42.48 | C |
| ATOM | 2274 | OG1 | THR | B | 80 | -24.316 | 0.427 | 39.130 | 1.00 | 44.17 | O |
| ATOM | 2275 | N | ILE | B | 81 | -20.893 | 2.659 | 37.152 | 1.00 | 36.66 | N |
| ATOM | 2276 | CA | ILE | B | 81 | -19.699 | 3.464 | 37.321 | 1.00 | 35.94 | C |
| ATOM | 2277 | C | ILE | B | 81 | -20.013 | 4.576 | 38.308 | 1.00 | 41.22 | C |
| ATOM | 2278 | O | ILE | B | 81 | -20.729 | 5.527 | 37.984 | 1.00 | 39.45 | O |
| ATOM | 2279 | CB | ILE | B | 81 | -19.206 | 4.068 | 35.997 | 1.00 | 29.74 | C |
| ATOM | 2280 | CG1 | ILE | B | 81 | -19.077 | 2.986 | 34.927 | 1.00 | 31.37 | C |
| ATOM | 2281 | CG2 | ILE | B | 81 | -17.875 | 4.784 | 36.204 | 1.00 | 37.10 | C |
| ATOM | 2282 | CD1 | ILE | B | 81 | -18.674 | 3.527 | 33.563 | 1.00 | 36.05 | C |
| ATOM | 2283 | N | SER | B | 82 | -19.506 | 4.442 | 39.525 | 1.00 | 36.61 | N |
| ATOM | 2284 | CA | SER | B | 82 | -19.621 | 5.521 | 40.489 | 1.00 | 47.92 | C |
| ATOM | 2285 | C | SER | B | 82 | -18.478 | 6.491 | 40.221 | 1.00 | 54.74 | C |
| ATOM | 2286 | O | SER | B | 82 | -17.328 | 6.069 | 40.079 | 1.00 | 68.40 | O |
| ATOM | 2287 | CB | SER | B | 82 | -19.571 | 4.991 | 41.921 | 1.00 | 38.83 | C |
| ATOM | 2288 | OG | SER | B | 82 | -18.240 | 4.674 | 42.287 | 1.00 | 41.65 | O |
| ATOM | 2289 | N | SER | B | 83 | -18.803 | 7.776 | 40.124 | 1.00 | 52.26 | N |
| ATOM | 2290 | CA | SER | B | 83 | -17.818 | 8.825 | 39.853 | 1.00 | 47.12 | C |
| ATOM | 2291 | C | SER | B | 83 | -17.144 | 8.663 | 38.481 | 1.00 | 47.93 | C |
| ATOM | 2292 | O | SER | B | 83 | -15.999 | 8.217 | 38.381 | 1.00 | 37.39 | O |
| ATOM | 2293 | CB | SER | B | 83 | -16.763 | 8.864 | 40.962 | 1.00 | 42.74 | C |
| ATOM | 2294 | OG | SER | B | 83 | -15.929 | 10.003 | 40.831 | 1.00 | 61.34 | O |
| ATOM | 2295 | N | LEU | B | 84 | -17.862 | 9.054 | 37.432 | 1.00 | 39.91 | N |
| ATOM | 2296 | CA | LEU | B | 84 | -17.373 | 8.932 | 36.061 | 1.00 | 40.35 | C |
| ATOM | 2297 | C | LEU | B | 84 | -16.120 | 9.777 | 35.825 | 1.00 | 43.92 | C |
| ATOM | 2298 | O | LEU | B | 84 | -16.079 | 10.954 | 36.188 | 1.00 | 32.91 | O |
| ATOM | 2299 | CB | LEU | B | 84 | -18.470 | 9.334 | 35.072 | 1.00 | 36.00 | C |
| ATOM | 2300 | CG | LEU | B | 84 | -18.281 | 8.902 | 33.616 | 1.00 | 40.11 | C |
| ATOM | 2301 | CD1 | LEU | B | 84 | -18.455 | 7.396 | 33.472 | 1.00 | 34.46 | C |
| ATOM | 2302 | CD2 | LEU | B | 84 | -19.246 | 9.639 | 32.702 | 1.00 | 39.38 | C |
| ATOM | 2303 | N | GLN | B | 85 | -15.103 | 9.166 | 35.221 | 1.00 | 35.37 | N |
| ATOM | 2304 | CA | GLN | B | 85 | -13.839 | 9.844 | 34.940 | 1.00 | 35.03 | C |
| ATOM | 2305 | C | GLN | B | 85 | -13.682 | 10.088 | 33.432 | 1.00 | 35.85 | C |
| ATOM | 2306 | O | GLN | B | 85 | -14.311 | 9.400 | 32.627 | 1.00 | 30.69 | O |
| ATOM | 2307 | CB | GLN | B | 85 | -12.665 | 9.023 | 35.484 | 1.00 | 33.69 | C |
| ATOM | 2308 | CG | GLN | B | 85 | -12.719 | 8.782 | 36.990 | 1.00 | 39.42 | C |
| ATOM | 2309 | CD | GLN | B | 85 | -12.651 | 10.069 | 37.795 | 1.00 | 43.01 | C |
| ATOM | 2310 | NE2 | GLN | B | 85 | -13.732 | 10.386 | 38.500 | 1.00 | 38.02 | N |
| ATOM | 2311 | OE1 | GLN | B | 85 | -11.640 | 10.772 | 37.778 | 1.00 | 40.65 | O |
| ATOM | 2312 | N | PRO | B | 86 | -12.857 | 11.082 | 33.046 | 1.00 | 33.28 | N |
| ATOM | 2313 | CA | PRO | B | 86 | -12.643 | 11.413 | 31.631 | 1.00 | 31.64 | C |
| ATOM | 2314 | C | PRO | B | 86 | -12.220 | 10.209 | 30.792 | 1.00 | 35.32 | C |
| ATOM | 2315 | O | PRO | B | 86 | -12.635 | 10.093 | 29.641 | 1.00 | 30.64 | O |
| ATOM | 2316 | CB | PRO | B | 86 | -11.520 | 12.456 | 31.681 | 1.00 | 34.31 | C |
| ATOM | 2317 | CG | PRO | B | 86 | -11.690 | 13.108 | 33.001 | 1.00 | 42.02 | C |
| ATOM | 2318 | CD | PRO | B | 86 | -12.146 | 12.020 | 33.936 | 1.00 | 35.55 | C |
| ATOM | 2319 | N | GLU | B | 87 | -11.423 | 9.316 | 31.373 | 1.00 | 32.17 | N |
| ATOM | 2320 | CA | GLU | B | 87 | -10.921 | 8.161 | 30.637 | 1.00 | 31.69 | C |
| ATOM | 2321 | C | GLU | B | 87 | -11.975 | 7.063 | 30.470 | 1.00 | 31.13 | C |
| ATOM | 2322 | O | GLU | B | 87 | -11.744 | 6.086 | 29.761 | 1.00 | 31.02 | O |
| ATOM | 2323 | CB | GLU | B | 87 | -9.681 | 7.583 | 31.327 | 1.00 | 32.83 | C |
| ATOM | 2324 | CG | GLU | B | 87 | -9.941 | 6.983 | 32.703 | 1.00 | 34.70 | C |
| ATOM | 2325 | CD | GLU | B | 87 | -9.628 | 7.950 | 33.834 | 1.00 | 47.47 | C |
| ATOM | 2326 | OE1 | GLU | B | 87 | -9.890 | 9.166 | 33.681 | 1.00 | 39.21 | O |
| ATOM | 2327 | OE2 | GLU | B | 87 | -9.111 | 7.486 | 34.876 | 1.00 | 49.29 | O |
| ATOM | 2328 | N | ASP | B | 88 | -13.131 | 7.227 | 31.108 | 1.00 | 28.67 | N |
| ATOM | 2329 | CA | ASP | B | 88 | -14.175 | 6.210 | 31.053 | 1.00 | 29.26 | C |
| ATOM | 2330 | C | ASP | B | 88 | -15.032 | 6.315 | 29.787 | 1.00 | 31.41 | C |
| ATOM | 2331 | O | ASP | B | 88 | -15.961 | 5.532 | 29.602 | 1.00 | 31.09 | O |
| ATOM | 2332 | CB | ASP | B | 88 | -15.070 | 6.293 | 32.295 | 1.00 | 32.59 | C |
| ATOM | 2333 | CG | ASP | B | 88 | -14.287 | 6.153 | 33.595 | 1.00 | 39.45 | C |
| ATOM | 2334 | OD1 | ASP | B | 88 | -13.166 | 5.597 | 33.565 | 1.00 | 34.75 | O |
| ATOM | 2335 | OD2 | ASP | B | 88 | -14.794 | 6.595 | 34.650 | 1.00 | 40.00 | O |
| ATOM | 2336 | N | VAL | B | 89 | -14.725 | 7.276 | 28.920 | 1.00 | 27.74 | N |
| ATOM | 2337 | CA | VAL | B | 89 | -15.437 | 7.395 | 27.648 | 1.00 | 29.97 | C |
| ATOM | 2338 | C | VAL | B | 89 | -15.162 | 6.169 | 26.783 | 1.00 | 26.75 | C |
| ATOM | 2339 | O | VAL | B | 89 | -14.020 | 5.928 | 26.391 | 1.00 | 32.52 | O |
| ATOM | 2340 | CB | VAL | B | 89 | -15.029 | 8.662 | 26.862 | 1.00 | 36.47 | C |

```
ATOM   2341  CG1 VAL B  89    -15.785    8.732   25.538  1.00 36.88           C
ATOM   2342  CG2 VAL B  89    -15.281    9.908   27.681  1.00 41.88           C
ATOM   2343  N   ALA B  90    -16.209    5.410   26.479  1.00 25.10           N
ATOM   2344  CA  ALA B  90    -16.063    4.149   25.764  1.00 28.51           C
ATOM   2345  C   ALA B  90    -17.410    3.508   25.491  1.00 30.70           C
ATOM   2346  O   ALA B  90    -18.448    3.995   25.943  1.00 27.64           O
ATOM   2347  CB  ALA B  90    -15.185    3.178   26.561  1.00 30.46           C
ATOM   2348  N   VAL B  91    -17.377    2.401   24.755  1.00 29.92           N
ATOM   2349  CA  VAL B  91    -18.539    1.542   24.606  1.00 28.27           C
ATOM   2350  C   VAL B  91    -18.425    0.364   25.573  1.00 33.48           C
ATOM   2351  O   VAL B  91    -17.368   -0.265   25.684  1.00 25.89           O
ATOM   2352  CB  VAL B  91    -18.683    1.024   23.172  1.00 28.97           C
ATOM   2353  CG1 VAL B  91    -19.940    0.178   23.045  1.00 28.55           C
ATOM   2354  CG2 VAL B  91    -18.723    2.194   22.196  1.00 29.32           C
ATOM   2355  N   TYR B  92    -19.511    0.077   26.281  1.00 29.51           N
ATOM   2356  CA  TYR B  92    -19.509   -0.993   27.267  1.00 29.47           C
ATOM   2357  C   TYR B  92    -20.379   -2.148   26.793  1.00 33.18           C
ATOM   2358  O   TYR B  92    -21.477   -1.945   26.267  1.00 29.29           O
ATOM   2359  CB  TYR B  92    -19.978   -0.472   28.630  1.00 23.56           C
ATOM   2360  CG  TYR B  92    -19.010    0.510   29.254  1.00 27.95           C
ATOM   2361  CD2 TYR B  92    -18.114    0.104   30.236  1.00 31.49           C
ATOM   2362  CD1 TYR B  92    -18.978    1.841   28.848  1.00 28.41           C
ATOM   2363  CE2 TYR B  92    -17.222    0.995   30.808  1.00 26.89           C
ATOM   2364  CE1 TYR B  92    -18.086    2.740   29.411  1.00 25.28           C
ATOM   2365  CZ  TYR B  92    -17.213    2.309   30.387  1.00 27.09           C
ATOM   2366  OH  TYR B  92    -16.325    3.190   30.940  1.00 27.92           O
ATOM   2367  N   TYR B  93    -19.863   -3.361   26.962  1.00 27.67           N
ATOM   2368  CA  TYR B  93    -20.548   -4.569   26.524  1.00 26.66           C
ATOM   2369  C   TYR B  93    -20.725   -5.548   27.673  1.00 29.56           C
ATOM   2370  O   TYR B  93    -19.802   -5.759   28.458  1.00 27.75           O
ATOM   2371  CB  TYR B  93    -19.765   -5.270   25.412  1.00 25.28           C
ATOM   2372  CG  TYR B  93    -19.615   -4.503   24.125  1.00 28.54           C
ATOM   2373  CD2 TYR B  93    -20.529   -4.662   23.090  1.00 26.88           C
ATOM   2374  CD1 TYR B  93    -18.540   -3.644   23.928  1.00 27.13           C
ATOM   2375  CE2 TYR B  93    -20.386   -3.973   21.899  1.00 32.89           C
ATOM   2376  CE1 TYR B  93    -18.385   -2.955   22.743  1.00 29.51           C
ATOM   2377  CZ  TYR B  93    -19.309   -3.123   21.731  1.00 32.69           C
ATOM   2378  OH  TYR B  93    -19.161   -2.436   20.550  1.00 33.36           O
ATOM   2379  N   CYS B  94    -21.901   -6.157   27.762  1.00 25.68           N
ATOM   2380  CA  CYS B  94    -22.079   -7.300   28.644  1.00 25.53           C
ATOM   2381  C   CYS B  94    -21.899   -8.557   27.810  1.00 28.07           C
ATOM   2382  O   CYS B  94    -22.013   -8.516   26.585  1.00 28.25           O
ATOM   2383  CB  CYS B  94    -23.450   -7.282   29.329  1.00 30.94           C
ATOM   2384  SG  CYS B  94    -24.873   -7.383   28.210  1.00 35.78           S
ATOM   2385  N   GLN B  95    -21.606   -9.671   28.468  1.00 25.81           N
ATOM   2386  CA  GLN B  95    -21.373  -10.920   27.758  1.00 28.41           C
ATOM   2387  C   GLN B  95    -21.819  -12.093   28.612  1.00 24.10           C
ATOM   2388  O   GLN B  95    -21.476  -12.169   29.789  1.00 33.33           O
ATOM   2389  CB  GLN B  95    -19.891  -11.065   27.391  1.00 24.57           C
ATOM   2390  CG  GLN B  95    -19.574  -12.277   26.523  1.00 27.70           C
ATOM   2391  CD  GLN B  95    -18.497  -13.168   27.121  1.00 30.66           C
ATOM   2392  NE2 GLN B  95    -18.756  -14.468   27.147  1.00 41.67           N
ATOM   2393  OE1 GLN B  95    -17.452  -12.695   27.557  1.00 36.39           O
ATOM   2394  N   GLN B  96    -22.584  -13.008   28.031  1.00 30.99           N
ATOM   2395  CA  GLN B  96    -22.926  -14.232   28.748  1.00 28.28           C
ATOM   2396  C   GLN B  96    -21.986  -15.347   28.313  1.00 31.50           C
ATOM   2397  O   GLN B  96    -21.612  -15.447   27.141  1.00 27.92           O
ATOM   2398  CB  GLN B  96    -24.392  -14.630   28.517  1.00 24.83           C
ATOM   2399  CG  GLN B  96    -24.737  -15.108   27.104  1.00 28.68           C
ATOM   2400  CD  GLN B  96    -24.439  -16.586   26.876  1.00 32.36           C
ATOM   2401  NE2 GLN B  96    -24.396  -16.992   25.611  1.00 32.40           N
ATOM   2402  OE1 GLN B  96    -24.241  -17.348   27.826  1.00 32.56           O
ATOM   2403  N   TYR B  97    -21.592  -16.182   29.261  1.00 26.53           N
ATOM   2404  CA  TYR B  97    -20.838  -17.370   28.918  1.00 30.75           C
ATOM   2405  C   TYR B  97    -21.465  -18.571   29.608  1.00 31.37           C
ATOM   2406  O   TYR B  97    -20.778  -19.519   29.989  1.00 28.22           O
ATOM   2407  CB  TYR B  97    -19.367  -17.209   29.294  1.00 27.16           C
ATOM   2408  CG  TYR B  97    -19.121  -16.745   30.714  1.00 30.11           C
ATOM   2409  CD2 TYR B  97    -18.893  -17.661   31.737  1.00 25.04           C
ATOM   2410  CD1 TYR B  97    -19.093  -15.390   31.026  1.00 26.12           C
ATOM   2411  CE2 TYR B  97    -18.650  -17.239   33.033  1.00 26.05           C
```

```
ATOM   2412  CE1 TYR B  97   -18.856 -14.960  32.316  1.00 30.26        C
ATOM   2413  CZ  TYR B  97   -18.634 -15.887  33.314  1.00 28.89        C
ATOM   2414  OH  TYR B  97   -18.396 -15.458  34.597  1.00 29.84        O
ATOM   2415  N   TYR B  98   -22.784 -18.510  29.762  1.00 37.70        N
ATOM   2416  CA  TYR B  98   -23.553 -19.601  30.349  1.00 32.36        C
ATOM   2417  C   TYR B  98   -23.579 -20.801  29.410  1.00 37.08        C
ATOM   2418  O   TYR B  98   -23.446 -21.942  29.849  1.00 37.43        O
ATOM   2419  CB  TYR B  98   -24.981 -19.153  30.672  1.00 32.63        C
ATOM   2420  CG  TYR B  98   -25.769 -20.191  31.440  1.00 40.80        C
ATOM   2421  CD1 TYR B  98   -25.434 -20.511  32.750  1.00 39.01        C
ATOM   2422  CD2 TYR B  98   -26.844 -20.853  30.857  1.00 44.09        C
ATOM   2423  CE1 TYR B  98   -26.146 -21.462  33.461  1.00 46.24        C
ATOM   2424  CE2 TYR B  98   -27.566 -21.807  31.563  1.00 46.44        C
ATOM   2425  CZ  TYR B  98   -27.209 -22.107  32.864  1.00 48.82        C
ATOM   2426  OH  TYR B  98   -27.913 -23.052  33.574  1.00 61.88        O
ATOM   2427  N   ASN B  99   -23.758 -20.542  28.117  1.00 36.19        N
ATOM   2428  CA  ASN B  99   -23.611 -21.596  27.119  1.00 44.15        C
ATOM   2429  C   ASN B  99   -23.116 -21.074  25.778  1.00 42.06        C
ATOM   2430  O   ASN B  99   -23.118 -19.868  25.521  1.00 43.16        O
ATOM   2431  CB  ASN B  99   -24.927 -22.361  26.924  1.00 49.30        C
ATOM   2432  CG  ASN B  99   -26.102 -21.455  26.600  1.00 51.38        C
ATOM   2433  OD1 ASN B  99   -27.134 -21.508  27.271  1.00 68.12        O
ATOM   2434  ND2 ASN B  99   -25.959 -20.628  25.568  1.00 49.12        N
ATOM   2435  N   THR B 100   -22.700 -22.001  24.924  1.00 38.31        N
ATOM   2436  CA  THR B 100   -22.195 -21.657  23.607  1.00 37.71        C
ATOM   2437  C   THR B 100   -23.339 -21.679  22.601  1.00 36.72        C
ATOM   2438  O   THR B 100   -24.243 -22.506  22.713  1.00 45.21        O
ATOM   2439  CB  THR B 100   -21.076 -22.618  23.173  1.00 40.81        C
ATOM   2440  OG1 THR B 100   -21.540 -23.966  23.286  1.00 41.71        O
ATOM   2441  CG2 THR B 100   -19.855 -22.442  24.069  1.00 34.33        C
ATOM   2442  N   PRO B 101   -23.313 -20.765  21.617  1.00 37.91        N
ATOM   2443  CA  PRO B 101   -22.253 -19.773  21.404  1.00 35.04        C
ATOM   2444  C   PRO B 101   -22.295 -18.613  22.395  1.00 38.94        C
ATOM   2445  O   PRO B 101   -23.368 -18.135  22.774  1.00 33.27        O
ATOM   2446  CB  PRO B 101   -22.526 -19.273  19.983  1.00 36.11        C
ATOM   2447  CG  PRO B 101   -24.004 -19.394  19.839  1.00 36.49        C
ATOM   2448  CD  PRO B 101   -24.388 -20.638  20.615  1.00 42.27        C
ATOM   2449  N   VAL B 102   -21.111 -18.192  22.823  1.00 35.40        N
ATOM   2450  CA  VAL B 102   -20.943 -16.980  23.610  1.00 34.45        C
ATOM   2451  C   VAL B 102   -21.531 -15.794  22.858  1.00 35.11        C
ATOM   2452  O   VAL B 102   -21.300 -15.643  21.659  1.00 30.87        O
ATOM   2453  CB  VAL B 102   -19.454 -16.735  23.911  1.00 38.34        C
ATOM   2454  CG1 VAL B 102   -19.155 -15.250  24.058  1.00 41.39        C
ATOM   2455  CG2 VAL B 102   -19.046 -17.509  25.142  1.00 38.42        C
ATOM   2456  N   THR B 103   -22.307 -14.966  23.549  1.00 28.91        N
ATOM   2457  CA  THR B 103   -22.929 -13.817  22.901  1.00 32.04        C
ATOM   2458  C   THR B 103   -22.740 -12.532  23.711  1.00 33.94        C
ATOM   2459  O   THR B 103   -22.715 -12.554  24.945  1.00 30.64        O
ATOM   2460  CB  THR B 103   -24.437 -14.054  22.659  1.00 33.42        C
ATOM   2461  CG2 THR B 103   -24.658 -15.178  21.636  1.00 30.57        C
ATOM   2462  OG1 THR B 103   -25.068 -14.415  23.890  1.00 35.52        O
ATOM   2463  N   PHE B 104   -22.594 -11.419  22.996  1.00 27.76        N
ATOM   2464  CA  PHE B 104   -22.429 -10.102  23.604  1.00 32.55        C
ATOM   2465  C   PHE B 104   -23.693  -9.274  23.430  1.00 34.05        C
ATOM   2466  O   PHE B 104   -24.481  -9.520  22.519  1.00 32.37        O
ATOM   2467  CB  PHE B 104   -21.257  -9.342  22.971  1.00 30.42        C
ATOM   2468  CG  PHE B 104   -19.915  -9.986  23.175  1.00 30.59        C
ATOM   2469  CD1 PHE B 104   -19.531 -11.079  22.417  1.00 27.18        C
ATOM   2470  CD2 PHE B 104   -19.019  -9.467  24.094  1.00 30.39        C
ATOM   2471  CE1 PHE B 104   -18.289 -11.660  22.590  1.00 31.59        C
ATOM   2472  CE2 PHE B 104   -17.775 -10.044  24.274  1.00 31.20        C
ATOM   2473  CZ  PHE B 104   -17.411 -11.144  23.519  1.00 33.50        C
ATOM   2474  N   GLY B 105   -23.873  -8.278  24.291  1.00 34.23        N
ATOM   2475  CA  GLY B 105   -24.878  -7.258  24.057  1.00 26.27        C
ATOM   2476  C   GLY B 105   -24.415  -6.334  22.941  1.00 34.89        C
ATOM   2477  O   GLY B 105   -23.264  -6.413  22.509  1.00 31.29        O
ATOM   2478  N   PRO B 106   -25.307  -5.447  22.470  1.00 35.38        N
ATOM   2479  CA  PRO B 106   -25.028  -4.559  21.336  1.00 33.90        C
ATOM   2480  C   PRO B 106   -24.100  -3.399  21.689  1.00 32.12        C
ATOM   2481  O   PRO B 106   -23.610  -2.715  20.794  1.00 34.27        O
ATOM   2482  CB  PRO B 106   -26.417  -4.037  20.956  1.00 29.01        C
```

```
ATOM   2483  CG   PRO B 106     -27.173   -4.053   22.248  1.00 37.44           C
ATOM   2484  CD   PRO B 106     -26.676   -5.267   22.989  1.00 33.03           C
ATOM   2485  N    GLY B 107     -23.874   -3.177   22.978  1.00 34.23           N
ATOM   2486  CA   GLY B 107     -22.984   -2.120   23.415  1.00 34.20           C
ATOM   2487  C    GLY B 107     -23.701   -0.843   23.807  1.00 34.29           C
ATOM   2488  O    GLY B 107     -24.725   -0.489   23.230  1.00 37.38           O
ATOM   2489  N    THR B 108     -23.149   -0.148   24.795  1.00 33.98           N
ATOM   2490  CA   THR B 108     -23.695    1.121   25.256  1.00 35.86           C
ATOM   2491  C    THR B 108     -22.617    2.190   25.231  1.00 33.06           C
ATOM   2492  O    THR B 108     -21.586    2.039   25.886  1.00 33.05           O
ATOM   2493  CB   THR B 108     -24.251    1.016   26.690  1.00 31.62           C
ATOM   2494  CG2  THR B 108     -24.619    2.395   27.221  1.00 36.86           C
ATOM   2495  OG1  THR B 108     -25.406    0.174   26.702  1.00 36.12           O
ATOM   2496  N    LYS B 109     -22.847    3.271   24.493  1.00 28.00           N
ATOM   2497  CA   LYS B 109     -21.850    4.337   24.418  1.00 33.62           C
ATOM   2498  C    LYS B 109     -21.956    5.254   25.634  1.00 38.15           C
ATOM   2499  O    LYS B 109     -23.026    5.786   25.931  1.00 41.81           O
ATOM   2500  CB   LYS B 109     -22.008    5.142   23.124  1.00 36.39           C
ATOM   2501  CG   LYS B 109     -22.625    4.343   21.980  1.00 56.96           C
ATOM   2502  CD   LYS B 109     -21.807    4.421   20.696  1.00 56.13           C
ATOM   2503  CE   LYS B 109     -21.884    5.792   20.054  1.00 62.08           C
ATOM   2504  NZ   LYS B 109     -21.138    5.822   18.763  1.00 74.82           N
ATOM   2505  N    VAL B 110     -20.848    5.420   26.348  1.00 32.23           N
ATOM   2506  CA   VAL B 110     -20.804    6.345   27.474  1.00 29.73           C
ATOM   2507  C    VAL B 110     -19.884    7.524   27.185  1.00 32.33           C
ATOM   2508  O    VAL B 110     -18.699    7.340   26.904  1.00 33.08           O
ATOM   2509  CB   VAL B 110     -20.328    5.659   28.765  1.00 31.47           C
ATOM   2510  CG1  VAL B 110     -20.223    6.678   29.886  1.00 29.96           C
ATOM   2511  CG2  VAL B 110     -21.270    4.525   29.142  1.00 34.90           C
ATOM   2512  N    GLY B 111     -20.440    8.730   27.249  1.00 29.76           N
ATOM   2513  CA   GLY B 111     -19.666    9.945   27.076  1.00 29.88           C
ATOM   2514  C    GLY B 111     -19.749   10.757   28.351  1.00 39.68           C
ATOM   2515  O    GLY B 111     -20.459   10.374   29.280  1.00 39.37           O
ATOM   2516  N    ILE B 112     -19.030   11.872   28.416  1.00 31.76           N
ATOM   2517  CA   ILE B 112     -19.082   12.697   29.613  1.00 36.49           C
ATOM   2518  C    ILE B 112     -19.508   14.132   29.295  1.00 41.86           C
ATOM   2519  O    ILE B 112     -19.221   14.665   28.216  1.00 33.11           O
ATOM   2520  CB   ILE B 112     -17.720   12.704   30.366  1.00 36.62           C
ATOM   2521  CG1  ILE B 112     -16.745   13.711   29.766  1.00 42.34           C
ATOM   2522  CG2  ILE B 112     -17.105   11.311   30.403  1.00 53.62           C
ATOM   2523  CD1  ILE B 112     -16.711   15.042   30.519  1.00 54.93           C
ATOM   2524  O    LYS B 113     -19.210   16.754   32.044  1.00 44.16           O
ATOM   2525  N    LYS B 113     -20.201   14.744   30.251  1.00 37.92           N
ATOM   2526  CA   LYS B 113     -20.582   16.147   30.170  1.00 39.34           C
ATOM   2527  C    LYS B 113     -19.560   17.015   30.892  1.00 43.78           C
ATOM   2528  CB   LYS B 113     -21.965   16.381   30.778  1.00 41.17           C
ATOM   2529  CG   LYS B 113     -23.120   15.740   30.040  1.00 40.99           C
ATOM   2530  CD   LYS B 113     -24.434   16.062   30.750  1.00 46.55           C
ATOM   2531  CE   LYS B 113     -25.614   15.365   30.094  1.00 50.82           C
ATOM   2532  NZ   LYS B 113     -26.868   15.545   30.882  1.00 66.34           N
ATOM   2533  O    ARG B 114     -19.706   20.614   29.907  1.00 33.63           O
ATOM   2534  N    ARG B 114     -19.080   18.045   30.208  1.00 37.75           N
ATOM   2535  CA   ARG B 114     -18.179   19.009   30.821  1.00 32.69           C
ATOM   2536  C    ARG B 114     -18.661   20.423   30.526  1.00 32.37           C
ATOM   2537  CB   ARG B 114     -16.747   18.815   30.316  1.00 29.43           C
ATOM   2538  CG   ARG B 114     -16.605   18.850   28.801  1.00 32.96           C
ATOM   2539  CD   ARG B 114     -15.228   19.372   28.400  1.00 29.78           C
ATOM   2540  NE   ARG B 114     -15.053   20.768   28.793  1.00 30.75           N
ATOM   2541  CZ   ARG B 114     -13.893   21.418   28.775  1.00 35.05           C
ATOM   2542  NH1  ARG B 114     -13.841   22.691   29.144  1.00 37.02           N
ATOM   2543  NH2  ARG B 114     -12.784   20.801   28.392  1.00 37.36           N
ATOM   2544  O    THR B 115     -17.390   22.466   28.475  1.00 30.86           O
ATOM   2545  N    THR B 115     -17.898   21.413   30.968  1.00 34.13           N
ATOM   2546  CA   THR B 115     -18.244   22.800   30.701  1.00 37.77           C
ATOM   2547  C    THR B 115     -18.146   23.108   29.207  1.00 38.67           C
ATOM   2548  CB   THR B 115     -17.336   23.763   31.484  1.00 35.59           C
ATOM   2549  OG1  THR B 115     -15.972   23.545   31.106  1.00 34.24           O
ATOM   2550  CG2  THR B 115     -17.485   23.531   32.986  1.00 28.14           C
ATOM   2551  N    VAL B 116     -18.932   24.080   28.762  1.00 39.57           N
ATOM   2552  CA   VAL B 116     -18.884   24.545   27.383  1.00 35.06           C
ATOM   2553  C    VAL B 116     -17.491   25.075   27.047  1.00 35.92           C
```

```
ATOM   2554  O    VAL B 116     -16.872  25.772  27.855  1.00 32.43           O
ATOM   2555  CB   VAL B 116     -19.938  25.649  27.133  1.00 35.82           C
ATOM   2556  CG1  VAL B 116     -19.734  26.295  25.778  1.00 33.41           C
ATOM   2557  CG2  VAL B 116     -21.347  25.082  27.253  1.00 29.35           C
ATOM   2558  N    ALA B 117     -16.991  24.725  25.865  1.00 29.09           N
ATOM   2559  CA   ALA B 117     -15.719  25.259  25.387  1.00 27.87           C
ATOM   2560  C    ALA B 117     -15.829  25.618  23.913  1.00 34.19           C
ATOM   2561  O    ALA B 117     -16.203  24.779  23.088  1.00 29.90           O
ATOM   2562  CB   ALA B 117     -14.589  24.258  25.615  1.00 30.76           C
ATOM   2563  N    ALA B 118     -15.522  26.870  23.588  1.00 32.82           N
ATOM   2564  CA   ALA B 118     -15.615  27.347  22.212  1.00 34.29           C
ATOM   2565  C    ALA B 118     -14.474  26.788  21.370  1.00 29.81           C
ATOM   2566  O    ALA B 118     -13.357  26.625  21.858  1.00 31.85           O
ATOM   2567  CB   ALA B 118     -15.614  28.877  22.173  1.00 29.49           C
ATOM   2568  N    PRO B 119     -14.753  26.481  20.097  1.00 31.49           N
ATOM   2569  CA   PRO B 119     -13.680  25.979  19.237  1.00 32.80           C
ATOM   2570  C    PRO B 119     -12.745  27.089  18.790  1.00 34.11           C
ATOM   2571  O    PRO B 119     -13.190  28.218  18.601  1.00 30.89           O
ATOM   2572  CB   PRO B 119     -14.436  25.404  18.039  1.00 29.59           C
ATOM   2573  CG   PRO B 119     -15.668  26.247  17.957  1.00 33.03           C
ATOM   2574  CD   PRO B 119     -16.043  26.559  19.386  1.00 30.58           C
ATOM   2575  N    SER B 120     -11.467  26.769  18.635  1.00 29.42           N
ATOM   2576  CA   SER B 120     -10.567  27.624  17.878  1.00 36.74           C
ATOM   2577  C    SER B 120     -10.661  27.181  16.418  1.00 34.59           C
ATOM   2578  O    SER B 120     -10.687  25.983  16.131  1.00 33.48           O
ATOM   2579  CB   SER B 120      -9.135  27.531  18.407  1.00 31.92           C
ATOM   2580  OG   SER B 120      -8.627  26.219  18.245  1.00 50.83           O
ATOM   2581  N    VAL B 121     -10.734  28.139  15.499  1.00 34.03           N
ATOM   2582  CA   VAL B 121     -11.015  27.831  14.096  1.00 29.08           C
ATOM   2583  C    VAL B 121      -9.845  28.195  13.179  1.00 31.62           C
ATOM   2584  O    VAL B 121      -9.239  29.257  13.314  1.00 33.72           O
ATOM   2585  CB   VAL B 121     -12.300  28.560  13.616  1.00 30.04           C
ATOM   2586  CG1  VAL B 121     -12.663  28.158  12.193  1.00 30.45           C
ATOM   2587  CG2  VAL B 121     -13.460  28.259  14.551  1.00 31.72           C
ATOM   2588  N    PHE B 122      -9.534  27.295  12.251  1.00 27.86           N
ATOM   2589  CA   PHE B 122      -8.463  27.498  11.283  1.00 29.57           C
ATOM   2590  C    PHE B 122      -8.935  27.084   9.890  1.00 39.92           C
ATOM   2591  O    PHE B 122      -9.622  26.068   9.734  1.00 34.52           O
ATOM   2592  CB   PHE B 122      -7.215  26.689  11.663  1.00 36.36           C
ATOM   2593  CG   PHE B 122      -6.747  26.906  13.078  1.00 32.19           C
ATOM   2594  CD1  PHE B 122      -7.271  26.158  14.117  1.00 36.97           C
ATOM   2595  CD2  PHE B 122      -5.770  27.847  13.362  1.00 34.40           C
ATOM   2596  CE1  PHE B 122      -6.836  26.352  15.419  1.00 41.91           C
ATOM   2597  CE2  PHE B 122      -5.330  28.044  14.661  1.00 36.84           C
ATOM   2598  CZ   PHE B 122      -5.864  27.294  15.689  1.00 37.49           C
ATOM   2599  N    ILE B 123      -8.566  27.862   8.879  1.00 28.07           N
ATOM   2600  CA   ILE B 123      -8.915  27.528   7.505  1.00 32.41           C
ATOM   2601  C    ILE B 123      -7.636  27.277   6.701  1.00 32.00           C
ATOM   2602  O    ILE B 123      -6.622  27.942   6.907  1.00 31.75           O
ATOM   2603  CB   ILE B 123      -9.777  28.646   6.846  1.00 28.87           C
ATOM   2604  CG1  ILE B 123     -10.347  28.183   5.502  1.00 32.47           C
ATOM   2605  CG2  ILE B 123      -8.985  29.938   6.700  1.00 28.48           C
ATOM   2606  CD1  ILE B 123     -11.358  29.154   4.896  1.00 31.95           C
ATOM   2607  N    PHE B 124      -7.679  26.291   5.811  1.00 31.26           N
ATOM   2608  CA   PHE B 124      -6.514  25.928   5.009  1.00 30.58           C
ATOM   2609  C    PHE B 124      -6.854  25.944   3.523  1.00 35.12           C
ATOM   2610  O    PHE B 124      -7.774  25.250   3.089  1.00 33.03           O
ATOM   2611  CB   PHE B 124      -5.992  24.540   5.390  1.00 32.40           C
ATOM   2612  CG   PHE B 124      -5.566  24.410   6.825  1.00 32.10           C
ATOM   2613  CD1  PHE B 124      -4.314  24.842   7.232  1.00 31.26           C
ATOM   2614  CD2  PHE B 124      -6.407  23.820   7.759  1.00 31.84           C
ATOM   2615  CE1  PHE B 124      -3.913  24.711   8.549  1.00 34.33           C
ATOM   2616  CE2  PHE B 124      -6.015  23.684   9.076  1.00 34.28           C
ATOM   2617  CZ   PHE B 124      -4.765  24.133   9.474  1.00 35.30           C
ATOM   2618  N    PRO B 125      -6.107  26.731   2.734  1.00 37.67           N
ATOM   2619  CA   PRO B 125      -6.296  26.753   1.278  1.00 33.34           C
ATOM   2620  C    PRO B 125      -5.816  25.454   0.646  1.00 32.58           C
ATOM   2621  O    PRO B 125      -4.996  24.766   1.247  1.00 29.20           O
ATOM   2622  CB   PRO B 125      -5.422  27.930   0.816  1.00 33.45           C
ATOM   2623  CG   PRO B 125      -5.036  28.671   2.074  1.00 39.04           C
ATOM   2624  CD   PRO B 125      -5.049  27.658   3.171  1.00 32.49           C
```

```
ATOM   2625  N    PRO B 126      -6.307  25.126  -0.557  1.00 31.25          N
ATOM   2626  CA   PRO B 126      -5.736  23.974  -1.263  1.00 36.87          C
ATOM   2627  C    PRO B 126      -4.265  24.213  -1.615  1.00 37.25          C
ATOM   2628  O    PRO B 126      -3.844  25.360  -1.778  1.00 32.38          O
ATOM   2629  CB   PRO B 126      -6.599  23.868  -2.527  1.00 31.74          C
ATOM   2630  CG   PRO B 126      -7.137  25.243  -2.728  1.00 32.69          C
ATOM   2631  CD   PRO B 126      -7.351  25.800  -1.348  1.00 31.78          C
ATOM   2632  N    SER B 127      -3.491  23.139  -1.709  1.00 33.45          N
ATOM   2633  CA   SER B 127      -2.082  23.251  -2.055  1.00 37.63          C
ATOM   2634  C    SER B 127      -1.931  23.368  -3.565  1.00 41.08          C
ATOM   2635  O    SER B 127      -2.812  22.939  -4.317  1.00 38.62          O
ATOM   2636  CB   SER B 127      -1.299  22.043  -1.542  1.00 33.64          C
ATOM   2637  OG   SER B 127      -1.647  20.874  -2.267  1.00 31.28          O
ATOM   2638  N    ASP B 128      -0.814  23.938  -4.008  1.00 40.33          N
ATOM   2639  CA   ASP B 128      -0.544  24.044  -5.437  1.00 42.97          C
ATOM   2640  C    ASP B 128      -0.439  22.664  -6.066  1.00 36.76          C
ATOM   2641  O    ASP B 128      -0.783  22.481  -7.232  1.00 42.27          O
ATOM   2642  CB   ASP B 128       0.737  24.841  -5.695  1.00 46.63          C
ATOM   2643  CG   ASP B 128       0.570  26.322  -5.406  1.00 59.28          C
ATOM   2644  OD1  ASP B 128      -0.562  26.834  -5.546  1.00 59.20          O
ATOM   2645  OD2  ASP B 128       1.570  26.974  -5.040  1.00 62.56          O
ATOM   2646  N    GLU B 129       0.022  21.690  -5.286  1.00 37.90          N
ATOM   2647  CA   GLU B 129       0.240  20.347  -5.809  1.00 41.75          C
ATOM   2648  C    GLU B 129      -1.075  19.666  -6.183  1.00 42.49          C
ATOM   2649  O    GLU B 129      -1.157  18.982  -7.202  1.00 42.15          O
ATOM   2650  CB   GLU B 129       1.008  19.489  -4.800  1.00 38.29          C
ATOM   2651  CG   GLU B 129       1.521  18.181  -5.394  1.00 55.48          C
ATOM   2652  CD   GLU B 129       2.471  17.435  -4.469  1.00 75.53          C
ATOM   2653  OE1  GLU B 129       3.644  17.234  -4.857  1.00 78.69          O
ATOM   2654  OE2  GLU B 129       2.044  17.043  -3.360  1.00 72.25          O
ATOM   2655  N    GLN B 130      -2.104  19.857  -5.365  1.00 37.24          N
ATOM   2656  CA   GLN B 130      -3.403  19.259  -5.654  1.00 34.81          C
ATOM   2657  C    GLN B 130      -4.093  19.947  -6.829  1.00 35.05          C
ATOM   2658  O    GLN B 130      -4.774  19.297  -7.622  1.00 36.82          O
ATOM   2659  CB   GLN B 130      -4.312  19.312  -4.427  1.00 32.75          C
ATOM   2660  CG   GLN B 130      -5.631  18.597  -4.645  1.00 30.49          C
ATOM   2661  CD   GLN B 130      -6.626  18.848  -3.534  1.00 38.30          C
ATOM   2662  NE2  GLN B 130      -7.643  18.000  -3.456  1.00 35.08          N
ATOM   2663  OE1  GLN B 130      -6.485  19.793  -2.754  1.00 31.99          O
ATOM   2664  N    LEU B 131      -3.930  21.265  -6.924  1.00 34.78          N
ATOM   2665  CA   LEU B 131      -4.512  22.036  -8.019  1.00 46.01          C
ATOM   2666  C    LEU B 131      -4.044  21.506  -9.369  1.00 40.30          C
ATOM   2667  O    LEU B 131      -4.800  21.497 -10.337  1.00 49.24          O
ATOM   2668  CB   LEU B 131      -4.160  23.521  -7.884  1.00 39.51          C
ATOM   2669  CG   LEU B 131      -4.884  24.279  -6.773  1.00 41.36          C
ATOM   2670  CD1  LEU B 131      -4.458  25.736  -6.738  1.00 42.24          C
ATOM   2671  CD2  LEU B 131      -6.385  24.168  -6.956  1.00 37.48          C
ATOM   2672  N    LYS B 132      -2.798  21.048  -9.414  1.00 43.36          N
ATOM   2673  CA   LYS B 132      -2.214  20.462 -10.616  1.00 47.83          C
ATOM   2674  C    LYS B 132      -2.933  19.197 -11.086  1.00 46.86          C
ATOM   2675  O    LYS B 132      -2.715  18.740 -12.206  1.00 54.52          O
ATOM   2676  CB   LYS B 132      -0.737  20.146 -10.373  1.00 49.58          C
ATOM   2677  CG   LYS B 132       0.229  21.216 -10.868  1.00 56.15          C
ATOM   2678  CD   LYS B 132       1.614  21.060 -10.241  1.00 64.07          C
ATOM   2679  CE   LYS B 132       2.009  19.593 -10.066  1.00 66.31          C
ATOM   2680  NZ   LYS B 132       2.086  18.843 -11.352  1.00 66.61          N
ATOM   2681  N    SER B 133      -3.776  18.626 -10.232  1.00 47.49          N
ATOM   2682  CA   SER B 133      -4.497  17.403 -10.578  1.00 38.87          C
ATOM   2683  C    SER B 133      -5.963  17.680 -10.922  1.00 45.85          C
ATOM   2684  O    SER B 133      -6.718  16.757 -11.226  1.00 45.58          O
ATOM   2685  CB   SER B 133      -4.415  16.389  -9.433  1.00 48.92          C
ATOM   2686  OG   SER B 133      -5.127  16.848  -8.293  1.00 52.02          O
ATOM   2687  N    GLY B 134      -6.365  18.947 -10.858  1.00 39.38          N
ATOM   2688  CA   GLY B 134      -7.692  19.349 -11.294  1.00 38.72          C
ATOM   2689  C    GLY B 134      -8.737  19.497 -10.202  1.00 40.64          C
ATOM   2690  O    GLY B 134      -9.904  19.767 -10.488  1.00 47.67          O
ATOM   2691  N    THR B 135      -8.320  19.335  -8.951  1.00 44.09          N
ATOM   2692  CA   THR B 135      -9.239  19.382  -7.816  1.00 41.60          C
ATOM   2693  C    THR B 135      -8.700  20.294  -6.716  1.00 38.79          C
ATOM   2694  O    THR B 135      -7.490  20.386  -6.515  1.00 39.11          O
ATOM   2695  CB   THR B 135      -9.485  17.965  -7.246  1.00 42.58          C
```

```
ATOM   2696  CG2 THR B 135     -10.545  17.982  -6.153  1.00 36.34           C
ATOM   2697  OG1 THR B 135      -9.910  17.092  -8.300  1.00 49.29           O
ATOM   2698  N   ALA B 136      -9.599  20.979  -6.016  1.00 39.56           N
ATOM   2699  CA  ALA B 136      -9.218  21.845  -4.907  1.00 31.14           C
ATOM   2700  C   ALA B 136      -9.980  21.476  -3.637  1.00 41.19           C
ATOM   2701  O   ALA B 136     -11.211  21.514  -3.609  1.00 38.90           O
ATOM   2702  CB  ALA B 136      -9.465  23.300  -5.257  1.00 30.89           C
ATOM   2703  N   SER B 137      -9.243  21.121  -2.588  1.00 32.83           N
ATOM   2704  CA  SER B 137      -9.844  20.823  -1.295  1.00 29.16           C
ATOM   2705  C   SER B 137      -9.551  21.947  -0.312  1.00 31.90           C
ATOM   2706  O   SER B 137      -8.391  22.273  -0.060  1.00 32.52           O
ATOM   2707  CB  SER B 137      -9.325  19.493  -0.742  1.00 31.20           C
ATOM   2708  OG  SER B 137      -9.665  18.411  -1.588  1.00 35.83           O
ATOM   2709  N   VAL B 138     -10.605  22.549   0.227  1.00 29.38           N
ATOM   2710  CA  VAL B 138     -10.455  23.578   1.250  1.00 28.61           C
ATOM   2711  C   VAL B 138     -10.856  22.986   2.596  1.00 28.33           C
ATOM   2712  O   VAL B 138     -11.896  22.332   2.708  1.00 28.89           O
ATOM   2713  CB  VAL B 138     -11.304  24.821   0.943  1.00 32.25           C
ATOM   2714  CG1 VAL B 138     -10.880  25.981   1.825  1.00 32.25           C
ATOM   2715  CG2 VAL B 138     -11.171  25.197  -0.524  1.00 34.85           C
ATOM   2716  N   VAL B 139     -10.031  23.208   3.615  1.00 27.05           N
ATOM   2717  CA  VAL B 139     -10.241  22.558   4.906  1.00 30.66           C
ATOM   2718  C   VAL B 139     -10.472  23.556   6.033  1.00 28.68           C
ATOM   2719  O   VAL B 139      -9.741  24.537   6.173  1.00 29.77           O
ATOM   2720  CB  VAL B 139      -9.042  21.646   5.267  1.00 31.42           C
ATOM   2721  CG1 VAL B 139      -9.199  21.067   6.665  1.00 30.03           C
ATOM   2722  CG2 VAL B 139      -8.904  20.529   4.239  1.00 29.56           C
ATOM   2723  N   CYS B 140     -11.507  23.300   6.826  1.00 31.41           N
ATOM   2724  CA  CYS B 140     -11.792  24.088   8.015  1.00 30.57           C
ATOM   2725  C   CYS B 140     -11.634  23.207   9.245  1.00 36.50           C
ATOM   2726  O   CYS B 140     -12.198  22.113   9.306  1.00 29.35           O
ATOM   2727  CB  CYS B 140     -13.205  24.672   7.960  1.00 32.78           C
ATOM   2728  SG  CYS B 140     -13.561  25.937   9.212  1.00 47.42           S
ATOM   2729  N   LEU B 141     -10.871  23.686  10.222  1.00 34.12           N
ATOM   2730  CA  LEU B 141     -10.629  22.931  11.444  1.00 30.81           C
ATOM   2731  C   LEU B 141     -11.230  23.619  12.672  1.00 33.78           C
ATOM   2732  O   LEU B 141     -11.006  24.809  12.904  1.00 32.21           O
ATOM   2733  CB  LEU B 141      -9.126  22.721  11.649  1.00 30.25           C
ATOM   2734  CG  LEU B 141      -8.728  22.199  13.032  1.00 33.72           C
ATOM   2735  CD1 LEU B 141      -9.143  20.737  13.205  1.00 28.38           C
ATOM   2736  CD2 LEU B 141      -7.234  22.383  13.280  1.00 35.34           C
ATOM   2737  N   LEU B 142     -12.006  22.862  13.442  1.00 25.85           N
ATOM   2738  CA  LEU B 142     -12.538  23.331  14.721  1.00 29.63           C
ATOM   2739  C   LEU B 142     -11.865  22.526  15.822  1.00 31.52           C
ATOM   2740  O   LEU B 142     -11.966  21.300  15.848  1.00 32.17           O
ATOM   2741  CB  LEU B 142     -14.062  23.177  14.796  1.00 28.07           C
ATOM   2742  CG  LEU B 142     -14.992  24.140  14.044  1.00 29.90           C
ATOM   2743  CD1 LEU B 142     -14.697  24.189  12.550  1.00 36.79           C
ATOM   2744  CD2 LEU B 142     -16.440  23.731  14.274  1.00 27.06           C
ATOM   2745  N   ASN B 143     -11.174  23.209  16.723  1.00 25.29           N
ATOM   2746  CA  ASN B 143     -10.291  22.521  17.648  1.00 31.88           C
ATOM   2747  C   ASN B 143     -10.754  22.593  19.100  1.00 34.33           C
ATOM   2748  O   ASN B 143     -11.042  23.673  19.618  1.00 32.56           O
ATOM   2749  CB  ASN B 143      -8.876  23.089  17.512  1.00 32.86           C
ATOM   2750  CG  ASN B 143      -7.813  22.134  18.016  1.00 43.50           C
ATOM   2751  ND2 ASN B 143      -7.037  22.574  18.997  1.00 47.72           N
ATOM   2752  OD1 ASN B 143      -7.683  21.018  17.518  1.00 44.96           O
ATOM   2753  N   ASN B 144     -10.840  21.421  19.733  1.00 33.53           N
ATOM   2754  CA  ASN B 144     -11.100  21.284  21.171  1.00 31.24           C
ATOM   2755  C   ASN B 144     -12.330  22.023  21.688  1.00 29.06           C
ATOM   2756  O   ASN B 144     -12.211  22.953  22.482  1.00 33.00           O
ATOM   2757  CB  ASN B 144      -9.873  21.743  21.962  1.00 30.14           C
ATOM   2758  CG  ASN B 144      -8.626  20.965  21.598  1.00 37.05           C
ATOM   2759  ND2 ASN B 144      -7.468  21.575  21.802  1.00 43.00           N
ATOM   2760  OD1 ASN B 144      -8.704  19.827  21.134  1.00 38.36           O
ATOM   2761  N   PHE B 145     -13.513  21.602  21.258  1.00 31.89           N
ATOM   2762  CA  PHE B 145     -14.732  22.260  21.706  1.00 29.94           C
ATOM   2763  C   PHE B 145     -15.672  21.295  22.416  1.00 34.88           C
ATOM   2764  O   PHE B 145     -15.533  20.074  22.317  1.00 26.87           O
ATOM   2765  CB  PHE B 145     -15.455  22.936  20.529  1.00 26.54           C
ATOM   2766  CG  PHE B 145     -15.846  21.994  19.418  1.00 27.20           C
```

| ATOM | 2767 | CD1 | PHE | B | 145 | -14.961 | 21.705 | 18.392 | 1.00 | 25.82 | C |
|------|------|-----|-----|---|-----|---------|--------|--------|------|-------|---|
| ATOM | 2768 | CD2 | PHE | B | 145 | -17.102 | 21.413 | 19.392 | 1.00 | 29.78 | C |
| ATOM | 2769 | CE1 | PHE | B | 145 | -15.316 | 20.839 | 17.368 | 1.00 | 27.58 | C |
| ATOM | 2770 | CE2 | PHE | B | 145 | -17.467 | 20.551 | 18.372 | 1.00 | 29.41 | C |
| ATOM | 2771 | CZ  | PHE | B | 145 | -16.569 | 20.263 | 17.356 | 1.00 | 27.09 | C |
| ATOM | 2772 | N   | TYR | B | 146 | -16.619 | 21.871 | 23.149 | 1.00 | 28.10 | N |
| ATOM | 2773 | CA  | TYR | B | 146 | -17.698 | 21.125 | 23.778 | 1.00 | 30.54 | C |
| ATOM | 2774 | C   | TYR | B | 146 | -18.886 | 22.071 | 23.962 | 1.00 | 29.20 | C |
| ATOM | 2775 | O   | TYR | B | 146 | -18.702 | 23.217 | 24.364 | 1.00 | 28.71 | O |
| ATOM | 2776 | CB  | TYR | B | 146 | -17.262 | 20.527 | 25.128 | 1.00 | 31.96 | C |
| ATOM | 2777 | CG  | TYR | B | 146 | -18.319 | 19.621 | 25.711 | 1.00 | 34.78 | C |
| ATOM | 2778 | CD2 | TYR | B | 146 | -18.334 | 18.262 | 25.418 | 1.00 | 31.97 | C |
| ATOM | 2779 | CD1 | TYR | B | 146 | -19.334 | 20.131 | 26.515 | 1.00 | 31.84 | C |
| ATOM | 2780 | CE2 | TYR | B | 146 | -19.319 | 17.432 | 25.925 | 1.00 | 32.92 | C |
| ATOM | 2781 | CE1 | TYR | B | 146 | -20.324 | 19.311 | 27.022 | 1.00 | 32.17 | C |
| ATOM | 2782 | CZ  | TYR | B | 146 | -20.310 | 17.965 | 26.725 | 1.00 | 34.30 | C |
| ATOM | 2783 | OH  | TYR | B | 146 | -21.291 | 17.149 | 27.228 | 1.00 | 42.60 | O |
| ATOM | 2784 | N   | PRO | B | 147 | -20.112 | 21.593 | 23.695 | 1.00 | 25.21 | N |
| ATOM | 2785 | CA  | PRO | B | 147 | -20.474 | 20.225 | 23.311 | 1.00 | 31.43 | C |
| ATOM | 2786 | C   | PRO | B | 147 | -20.233 | 19.921 | 21.838 | 1.00 | 34.10 | C |
| ATOM | 2787 | O   | PRO | B | 147 | -19.696 | 20.751 | 21.103 | 1.00 | 33.70 | O |
| ATOM | 2788 | CB  | PRO | B | 147 | -21.967 | 20.159 | 23.643 | 1.00 | 32.07 | C |
| ATOM | 2789 | CG  | PRO | B | 147 | -22.446 | 21.552 | 23.429 | 1.00 | 27.75 | C |
| ATOM | 2790 | CD  | PRO | B | 147 | -21.301 | 22.453 | 23.836 | 1.00 | 31.95 | C |
| ATOM | 2791 | N   | ARG | B | 148 | -20.646 | 18.725 | 21.434 | 1.00 | 34.40 | N |
| ATOM | 2792 | CA  | ARG | B | 148 | -20.383 | 18.168 | 20.110 | 1.00 | 32.34 | C |
| ATOM | 2793 | C   | ARG | B | 148 | -21.069 | 18.932 | 18.975 | 1.00 | 32.84 | C |
| ATOM | 2794 | O   | ARG | B | 148 | -20.505 | 19.087 | 17.892 | 1.00 | 29.63 | O |
| ATOM | 2795 | CB  | ARG | B | 148 | -20.823 | 16.700 | 20.101 | 1.00 | 35.79 | C |
| ATOM | 2796 | CG  | ARG | B | 148 | -20.923 | 16.051 | 18.744 | 1.00 | 41.03 | C |
| ATOM | 2797 | CD  | ARG | B | 148 | -19.703 | 15.208 | 18.453 | 1.00 | 47.90 | C |
| ATOM | 2798 | NE  | ARG | B | 148 | -20.044 | 13.980 | 17.738 | 1.00 | 56.46 | N |
| ATOM | 2799 | CZ  | ARG | B | 148 | -20.405 | 13.932 | 16.458 | 1.00 | 56.76 | C |
| ATOM | 2800 | NH1 | ARG | B | 148 | -20.485 | 15.048 | 15.740 | 1.00 | 54.31 | N |
| ATOM | 2801 | NH2 | ARG | B | 148 | -20.688 | 12.767 | 15.893 | 1.00 | 51.59 | N |
| ATOM | 2802 | N   | GLU | B | 149 | -22.284 | 19.404 | 19.230 | 1.00 | 36.40 | N |
| ATOM | 2803 | CA  | GLU | B | 149 | -23.068 | 20.115 | 18.224 | 1.00 | 39.72 | C |
| ATOM | 2804 | C   | GLU | B | 149 | -22.387 | 21.403 | 17.779 | 1.00 | 39.89 | C |
| ATOM | 2805 | O   | GLU | B | 149 | -22.182 | 22.319 | 18.577 | 1.00 | 39.53 | O |
| ATOM | 2806 | CB  | GLU | B | 149 | -24.473 | 20.431 | 18.755 | 1.00 | 34.93 | C |
| ATOM | 2807 | CG  | GLU | B | 149 | -25.369 | 19.209 | 18.956 | 1.00 | 45.72 | C |
| ATOM | 2808 | CD  | GLU | B | 149 | -24.967 | 18.363 | 20.158 | 1.00 | 54.80 | C |
| ATOM | 2809 | OE1 | GLU | B | 149 | -24.402 | 18.920 | 21.125 | 1.00 | 48.46 | O |
| ATOM | 2810 | OE2 | GLU | B | 149 | -25.211 | 17.138 | 20.130 | 1.00 | 62.82 | O |
| ATOM | 2811 | N   | ALA | B | 150 | -22.042 | 21.462 | 16.498 | 1.00 | 35.98 | N |
| ATOM | 2812 | CA  | ALA | B | 150 | -21.432 | 22.649 | 15.914 | 1.00 | 37.14 | C |
| ATOM | 2813 | C   | ALA | B | 150 | -21.867 | 22.779 | 14.465 | 1.00 | 43.97 | C |
| ATOM | 2814 | O   | ALA | B | 150 | -22.094 | 21.778 | 13.786 | 1.00 | 46.01 | O |
| ATOM | 2815 | CB  | ALA | B | 150 | -19.914 | 22.586 | 16.014 | 1.00 | 32.49 | C |
| ATOM | 2816 | N   | LYS | B | 151 | -21.987 | 24.012 | 13.992 | 1.00 | 39.41 | N |
| ATOM | 2817 | CA  | LYS | B | 151 | -22.417 | 24.246 | 12.624 | 1.00 | 43.48 | C |
| ATOM | 2818 | C   | LYS | B | 151 | -21.319 | 24.913 | 11.807 | 1.00 | 39.53 | C |
| ATOM | 2819 | O   | LYS | B | 151 | -20.799 | 25.966 | 12.179 | 1.00 | 36.87 | O |
| ATOM | 2820 | CB  | LYS | B | 151 | -23.687 | 25.099 | 12.596 | 1.00 | 43.88 | C |
| ATOM | 2821 | CG  | LYS | B | 151 | -24.256 | 25.301 | 11.196 | 1.00 | 55.40 | C |
| ATOM | 2822 | CD  | LYS | B | 151 | -25.547 | 26.104 | 11.223 | 1.00 | 53.19 | C |
| ATOM | 2823 | CE  | LYS | B | 151 | -26.147 | 26.222 | 9.830  | 1.00 | 70.97 | C |
| ATOM | 2824 | NZ  | LYS | B | 151 | -27.463 | 26.923 | 9.839  | 1.00 | 84.38 | N |
| ATOM | 2825 | N   | VAL | B | 152 | -20.968 | 24.282 | 10.694 | 1.00 | 36.81 | N |
| ATOM | 2826 | CA  | VAL | B | 152 | -19.990 | 24.837 | 9.774  | 1.00 | 37.97 | C |
| ATOM | 2827 | C   | VAL | B | 152 | -20.652 | 25.168 | 8.446  | 1.00 | 36.38 | C |
| ATOM | 2828 | O   | VAL | B | 152 | -21.221 | 24.292 | 7.790  | 1.00 | 36.25 | O |
| ATOM | 2829 | CB  | VAL | B | 152 | -18.820 | 23.867 | 9.535  | 1.00 | 39.90 | C |
| ATOM | 2830 | CG1 | VAL | B | 152 | -17.884 | 24.420 | 8.470  | 1.00 | 35.36 | C |
| ATOM | 2831 | CG2 | VAL | B | 152 | -18.078 | 23.614 | 10.834 | 1.00 | 39.55 | C |
| ATOM | 2832 | N   | GLN | B | 153 | -20.590 | 26.437 | 8.060  | 1.00 | 32.64 | N |
| ATOM | 2833 | CA  | GLN | B | 153 | -21.118 | 26.864 | 6.771  | 1.00 | 34.66 | C |
| ATOM | 2834 | C   | GLN | B | 153 | -19.999 | 27.378 | 5.884  | 1.00 | 38.08 | C |
| ATOM | 2835 | O   | GLN | B | 153 | -19.290 | 28.319 | 6.244  | 1.00 | 38.02 | O |
| ATOM | 2836 | CB  | GLN | B | 153 | -22.187 | 27.946 | 6.946  | 1.00 | 37.83 | C |
| ATOM | 2837 | CG  | GLN | B | 153 | -23.590 | 27.404 | 7.164  | 1.00 | 60.26 | C |

```
ATOM   2838  CD   GLN B 153     -24.596  28.498   7.470  1.00 67.71           C
ATOM   2839  NE2  GLN B 153     -25.857  28.257   7.123  1.00 56.86           N
ATOM   2840  OE1  GLN B 153     -24.244  29.548   8.009  1.00 70.19           O
ATOM   2841  N    TRP B 154     -19.840  26.747   4.727  1.00 31.58           N
ATOM   2842  CA   TRP B 154     -18.864  27.189   3.747  1.00 27.44           C
ATOM   2843  C    TRP B 154     -19.449  28.300   2.884  1.00 33.80           C
ATOM   2844  O    TRP B 154     -20.592  28.209   2.436  1.00 30.68           O
ATOM   2845  CB   TRP B 154     -18.415  26.022   2.869  1.00 25.14           C
ATOM   2846  CG   TRP B 154     -17.449  25.089   3.532  1.00 34.77           C
ATOM   2847  CD1  TRP B 154     -17.729  23.870   4.080  1.00 29.48           C
ATOM   2848  CD2  TRP B 154     -16.042  25.295   3.709  1.00 33.52           C
ATOM   2849  CE2  TRP B 154     -15.534  24.159   4.369  1.00 36.13           C
ATOM   2850  CE3  TRP B 154     -15.163  26.328   3.370  1.00 28.50           C
ATOM   2851  NE1  TRP B 154     -16.584  23.304   4.582  1.00 32.42           N
ATOM   2852  CZ2  TRP B 154     -14.186  24.028   4.697  1.00 35.02           C
ATOM   2853  CZ3  TRP B 154     -13.827  26.199   3.699  1.00 32.42           C
ATOM   2854  CH2  TRP B 154     -13.350  25.056   4.355  1.00 33.88           C
ATOM   2855  N    LYS B 155     -18.661  29.343   2.652  1.00 26.76           N
ATOM   2856  CA   LYS B 155     -19.089  30.458   1.818  1.00 34.70           C
ATOM   2857  C    LYS B 155     -18.018  30.803   0.795  1.00 35.14           C
ATOM   2858  O    LYS B 155     -16.869  31.063   1.149  1.00 34.32           O
ATOM   2859  CB   LYS B 155     -19.415  31.685   2.671  1.00 31.08           C
ATOM   2860  CG   LYS B 155     -20.658  31.527   3.525  1.00 39.61           C
ATOM   2861  CD   LYS B 155     -20.861  32.722   4.439  1.00 45.78           C
ATOM   2862  CE   LYS B 155     -22.086  32.531   5.322  1.00 52.43           C
ATOM   2863  NZ   LYS B 155     -22.240  33.644   6.300  1.00 64.93           N
ATOM   2864  N    VAL B 156     -18.404  30.787  -0.474  1.00 33.51           N
ATOM   2865  CA   VAL B 156     -17.512  31.163  -1.562  1.00 34.85           C
ATOM   2866  C    VAL B 156     -18.043  32.430  -2.235  1.00 37.05           C
ATOM   2867  O    VAL B 156     -19.085  32.398  -2.889  1.00 36.43           O
ATOM   2868  CB   VAL B 156     -17.378  30.035  -2.590  1.00 37.60           C
ATOM   2869  CG1  VAL B 156     -16.452  30.450  -3.701  1.00 36.02           C
ATOM   2870  CG2  VAL B 156     -16.863  28.772  -1.921  1.00 31.56           C
ATOM   2871  N    ASP B 157     -17.321  33.536  -2.060  1.00 38.87           N
ATOM   2872  CA   ASP B 157     -17.785  34.866  -2.468  1.00 41.38           C
ATOM   2873  C    ASP B 157     -19.196  35.139  -1.951  1.00 44.37           C
ATOM   2874  O    ASP B 157     -20.058  35.627  -2.684  1.00 49.49           O
ATOM   2875  CB   ASP B 157     -17.733  35.023  -3.991  1.00 42.34           C
ATOM   2876  CG   ASP B 157     -16.326  35.296  -4.499  1.00 44.58           C
ATOM   2877  OD1  ASP B 157     -15.517  35.866  -3.734  1.00 49.40           O
ATOM   2878  OD2  ASP B 157     -16.029  34.942  -5.659  1.00 49.33           O
ATOM   2879  N    ASN B 158     -19.405  34.809  -0.678  1.00 38.79           N
ATOM   2880  CA   ASN B 158     -20.688  34.958   0.016  1.00 46.07           C
ATOM   2881  C    ASN B 158     -21.827  34.101  -0.535  1.00 42.73           C
ATOM   2882  O    ASN B 158     -22.992  34.335  -0.218  1.00 49.48           O
ATOM   2883  CB   ASN B 158     -21.115  36.426   0.033  1.00 47.14           C
ATOM   2884  CG   ASN B 158     -20.716  37.122   1.313  1.00 64.92           C
ATOM   2885  ND2  ASN B 158     -20.238  38.356   1.196  1.00 70.04           N
ATOM   2886  OD1  ASN B 158     -20.825  36.549   2.400  1.00 67.22           O
ATOM   2887  N    ALA B 159     -21.497  33.100  -1.341  1.00 37.66           N
ATOM   2888  CA   ALA B 159     -22.492  32.110  -1.731  1.00 37.19           C
ATOM   2889  C    ALA B 159     -22.368  30.887  -0.823  1.00 39.41           C
ATOM   2890  O    ALA B 159     -21.298  30.285  -0.720  1.00 32.50           O
ATOM   2891  CB   ALA B 159     -22.330  31.717  -3.191  1.00 30.09           C
ATOM   2892  N    LEU B 160     -23.464  30.537  -0.159  1.00 34.32           N
ATOM   2893  CA   LEU B 160     -23.496  29.376   0.720  1.00 29.40           C
ATOM   2894  C    LEU B 160     -23.360  28.083  -0.073  1.00 33.09           C
ATOM   2895  O    LEU B 160     -24.083  27.858  -1.044  1.00 36.96           O
ATOM   2896  CB   LEU B 160     -24.790  29.348   1.540  1.00 37.86           C
ATOM   2897  CG   LEU B 160     -24.960  30.336   2.701  1.00 54.68           C
ATOM   2898  CD1  LEU B 160     -25.190  31.767   2.222  1.00 48.85           C
ATOM   2899  CD2  LEU B 160     -26.100  29.883   3.608  1.00 60.01           C
ATOM   2900  N    GLN B 161     -22.427  27.238   0.347  1.00 30.58           N
ATOM   2901  CA   GLN B 161     -22.206  25.943  -0.284  1.00 30.22           C
ATOM   2902  C    GLN B 161     -22.919  24.850   0.499  1.00 35.29           C
ATOM   2903  O    GLN B 161     -22.868  24.821   1.727  1.00 38.99           O
ATOM   2904  CB   GLN B 161     -20.710  25.633  -0.371  1.00 31.23           C
ATOM   2905  CG   GLN B 161     -19.880  26.761  -0.958  1.00 30.86           C
ATOM   2906  CD   GLN B 161     -20.221  27.015  -2.407  1.00 32.74           C
ATOM   2907  NE2  GLN B 161     -20.715  28.210  -2.698  1.00 30.42           N
ATOM   2908  OE1  GLN B 161     -20.062  26.138  -3.252  1.00 34.09           O
```

```
ATOM   2909  N    SER B 162     -23.588  23.955  -0.215  1.00 27.98           N
ATOM   2910  CA   SER B 162     -24.234  22.815   0.416  1.00 38.77           C
ATOM   2911  C    SER B 162     -24.111  21.602  -0.496  1.00 34.56           C
ATOM   2912  O    SER B 162     -24.271  21.717  -1.713  1.00 33.35           O
ATOM   2913  CB   SER B 162     -25.703  23.126   0.723  1.00 31.67           C
ATOM   2914  OG   SER B 162     -26.329  22.040   1.385  1.00 49.66           O
ATOM   2915  N    GLY B 163     -23.800  20.449   0.090  1.00 33.25           N
ATOM   2916  CA   GLY B 163     -23.739  19.206  -0.661  1.00 28.14           C
ATOM   2917  C    GLY B 163     -22.356  18.880  -1.192  1.00 32.51           C
ATOM   2918  O    GLY B 163     -22.101  17.758  -1.620  1.00 33.73           O
ATOM   2919  N    ASN B 164     -21.457  19.859  -1.164  1.00 30.22           N
ATOM   2920  CA   ASN B 164     -20.114  19.664  -1.694  1.00 31.34           C
ATOM   2921  C    ASN B 164     -19.043  19.693  -0.605  1.00 35.48           C
ATOM   2922  O    ASN B 164     -17.881  19.990  -0.876  1.00 31.32           O
ATOM   2923  CB   ASN B 164     -19.809  20.722  -2.767  1.00 30.74           C
ATOM   2924  CG   ASN B 164     -19.951  22.147  -2.248  1.00 33.60           C
ATOM   2925  ND2  ASN B 164     -19.572  23.116  -3.073  1.00 33.79           N
ATOM   2926  OD1  ASN B 164     -20.405  22.374  -1.127  1.00 32.68           O
ATOM   2927  N    SER B 165     -19.435  19.387   0.628  1.00 35.40           N
ATOM   2928  CA   SER B 165     -18.470  19.275   1.714  1.00 35.79           C
ATOM   2929  C    SER B 165     -18.750  18.042   2.561  1.00 32.53           C
ATOM   2930  O    SER B 165     -19.849  17.490   2.521  1.00 29.82           O
ATOM   2931  CB   SER B 165     -18.478  20.530   2.593  1.00 32.78           C
ATOM   2932  OG   SER B 165     -19.639  20.592   3.401  1.00 33.05           O
ATOM   2933  N    GLN B 166     -17.742  17.606   3.311  1.00 30.86           N
ATOM   2934  CA   GLN B 166     -17.896  16.486   4.236  1.00 32.40           C
ATOM   2935  C    GLN B 166     -17.194  16.792   5.553  1.00 32.23           C
ATOM   2936  O    GLN B 166     -16.181  17.493   5.575  1.00 33.90           O
ATOM   2937  CB   GLN B 166     -17.346  15.189   3.634  1.00 26.55           C
ATOM   2938  CG   GLN B 166     -18.110  14.694   2.418  1.00 37.55           C
ATOM   2939  CD   GLN B 166     -17.656  13.318   1.960  1.00 45.88           C
ATOM   2940  NE2  GLN B 166     -16.458  13.247   1.393  1.00 37.75           N
ATOM   2941  OE1  GLN B 166     -18.378  12.332   2.110  1.00 44.50           O
ATOM   2942  N    GLU B 167     -17.741  16.260   6.642  1.00 26.95           N
ATOM   2943  CA   GLU B 167     -17.217  16.491   7.985  1.00 34.35           C
ATOM   2944  C    GLU B 167     -16.789  15.199   8.666  1.00 31.65           C
ATOM   2945  O    GLU B 167     -17.351  14.136   8.416  1.00 31.31           O
ATOM   2946  CB   GLU B 167     -18.263  17.166   8.868  1.00 33.65           C
ATOM   2947  CG   GLU B 167     -18.526  18.617   8.585  1.00 50.67           C
ATOM   2948  CD   GLU B 167     -19.553  19.191   9.544  1.00 59.29           C
ATOM   2949  OE1  GLU B 167     -20.018  18.431  10.425  1.00 49.27           O
ATOM   2950  OE2  GLU B 167     -19.895  20.388   9.418  1.00 59.18           O
ATOM   2951  N    SER B 168     -15.815  15.317   9.556  1.00 28.62           N
ATOM   2952  CA   SER B 168     -15.393  14.216  10.407  1.00 32.03           C
ATOM   2953  C    SER B 168     -15.096  14.753  11.802  1.00 31.07           C
ATOM   2954  O    SER B 168     -14.499  15.819  11.945  1.00 28.49           O
ATOM   2955  CB   SER B 168     -14.165  13.522   9.817  1.00 34.82           C
ATOM   2956  OG   SER B 168     -13.566  12.666  10.762  1.00 40.29           O
ATOM   2957  N    AVAL B 169    -15.514  14.013  12.828  0.89 30.56           N
ATOM   2958  CA   AVAL B 169    -15.342  14.433  14.221  0.89 30.43           C
ATOM   2959  C    AVAL B 169    -14.558  13.389  15.017  0.89 31.75           C
ATOM   2960  O    AVAL B 169    -14.796  12.193  14.870  0.89 33.51           O
ATOM   2961  CB   AVAL B 169    -16.708  14.663  14.910  0.89 32.02           C
ATOM   2962  CG1  AVAL B 169    -16.518  15.215  16.317  0.89 33.65           C
ATOM   2963  CG2  AVAL B 169    -17.578  15.591  14.079  0.89 31.73           C
ATOM   2964  N    BVAL B 169    -15.523  14.018  12.822  0.11 30.45           N
ATOM   2965  CA   BVAL B 169    -15.292  14.424  14.200  0.11 31.12           C
ATOM   2966  C    BVAL B 169    -14.467  13.380  14.937  0.11 31.77           C
ATOM   2967  O    BVAL B 169    -14.578  12.184  14.669  0.11 33.10           O
ATOM   2968  CB   BVAL B 169    -16.616  14.648  14.953  0.11 32.66           C
ATOM   2969  CG1  BVAL B 169    -17.352  15.847  14.385  0.11 32.73           C
ATOM   2970  CG2  BVAL B 169    -17.482  13.403  14.878  0.11 33.85           C
ATOM   2971  N    THR B 170     -13.633  13.837  15.862  1.00 29.38           N
ATOM   2972  CA   THR B 170     -12.847  12.921  16.682  1.00 36.76           C
ATOM   2973  C    THR B 170     -13.703  12.346  17.805  1.00 33.01           C
ATOM   2974  O    THR B 170     -14.762  12.885  18.133  1.00 30.78           O
ATOM   2975  CB   THR B 170     -11.620  13.605  17.308  1.00 30.27           C
ATOM   2976  CG2  THR B 170     -10.719  14.211  16.235  1.00 34.58           C
ATOM   2977  OG1  THR B 170     -12.057  14.633  18.199  1.00 31.73           O
ATOM   2978  N    GLU B 171     -13.243  11.248  18.390  1.00 34.68           N
ATOM   2979  CA   GLU B 171     -13.843  10.746  19.618  1.00 35.38           C
```

| ATOM | 2980 | C   | GLU B 171 | -13.613 | 11.768 | 20.720 | 1.00 37.95 | C |
| ATOM | 2981 | O   | GLU B 171 | -12.689 | 12.584 | 20.639 | 1.00 34.11 | O |
| ATOM | 2982 | CB  | GLU B 171 | -13.247 |  9.391 | 20.011 | 1.00 35.96 | C |
| ATOM | 2983 | CG  | GLU B 171 | -13.457 |  8.290 | 18.984 | 1.00 47.50 | C |
| ATOM | 2984 | CD  | GLU B 171 | -14.850 |  7.683 | 19.038 | 1.00 65.62 | C |
| ATOM | 2985 | OE1 | GLU B 171 | -15.595 |  7.969 | 20.002 | 1.00 65.84 | O |
| ATOM | 2986 | OE2 | GLU B 171 | -15.198 |  6.912 | 18.116 | 1.00 70.74 | O |
| ATOM | 2987 | N   | GLN B 172 | -14.456 | 11.734 | 21.743 | 1.00 34.37 | N |
| ATOM | 2988 | CA  | GLN B 172 | -14.272 | 12.609 | 22.885 | 1.00 27.96 | C |
| ATOM | 2989 | C   | GLN B 172 | -12.897 | 12.347 | 23.509 | 1.00 29.48 | C |
| ATOM | 2990 | O   | GLN B 172 | -12.544 | 11.210 | 23.785 | 1.00 33.27 | O |
| ATOM | 2991 | CB  | GLN B 172 | -15.399 | 12.399 | 23.899 | 1.00 31.65 | C |
| ATOM | 2992 | CG  | GLN B 172 | -15.483 | 13.474 | 24.959 | 1.00 34.27 | C |
| ATOM | 2993 | CD  | GLN B 172 | -16.804 | 13.457 | 25.706 | 1.00 37.13 | C |
| ATOM | 2994 | NE2 | GLN B 172 | -17.166 | 14.594 | 26.286 | 1.00 32.91 | N |
| ATOM | 2995 | OE1 | GLN B 172 | -17.496 | 12.437 | 25.755 | 1.00 34.99 | O |
| ATOM | 2996 | N   | ASP B 173 | -12.120 | 13.408 | 23.700 | 1.00 30.64 | N |
| ATOM | 2997 | CA  | ASP B 173 | -10.766 | 13.296 | 24.231 | 1.00 32.58 | C |
| ATOM | 2998 | C   | ASP B 173 | -10.749 | 12.684 | 25.636 | 1.00 36.32 | C |
| ATOM | 2999 | O   | ASP B 173 | -11.537 | 13.069 | 26.502 | 1.00 30.09 | O |
| ATOM | 3000 | CB  | ASP B 173 | -10.097 | 14.671 | 24.246 | 1.00 32.10 | C |
| ATOM | 3001 | CG  | ASP B 173 |  -8.613 | 14.593 | 24.538 | 1.00 36.21 | C |
| ATOM | 3002 | OD2 | ASP B 173 |  -8.222 | 14.802 | 25.708 | 1.00 36.85 | O |
| ATOM | 3003 | OD1 | ASP B 173 |  -7.836 | 14.324 | 23.595 | 1.00 37.22 | O |
| ATOM | 3004 | N   | SER B 174 |  -9.844 | 11.737 | 25.863 | 1.00 32.20 | N |
| ATOM | 3005 | CA  | SER B 174 |  -9.818 | 11.015 | 27.134 | 1.00 38.98 | C |
| ATOM | 3006 | C   | SER B 174 |  -9.243 | 11.855 | 28.275 | 1.00 32.61 | C |
| ATOM | 3007 | O   | SER B 174 |  -9.301 | 11.447 | 29.432 | 1.00 42.30 | O |
| ATOM | 3008 | CB  | SER B 174 |  -9.017 |  9.717 | 26.998 | 1.00 36.54 | C |
| ATOM | 3009 | OG  | SER B 174 |  -7.630 |  9.986 | 26.883 | 1.00 46.81 | O |
| ATOM | 3010 | N   | LYS B 175 |  -8.691 | 13.020 | 27.954 | 1.00 35.47 | N |
| ATOM | 3011 | CA  | LYS B 175 |  -8.142 | 13.907 | 28.981 | 1.00 33.77 | C |
| ATOM | 3012 | C   | LYS B 175 |  -9.040 | 15.113 | 29.278 | 1.00 36.43 | C |
| ATOM | 3013 | O   | LYS B 175 |  -9.479 | 15.287 | 30.410 | 1.00 30.79 | O |
| ATOM | 3014 | CB  | LYS B 175 |  -6.749 | 14.404 | 28.581 | 1.00 36.54 | C |
| ATOM | 3015 | CG  | LYS B 175 |  -5.717 | 13.303 | 28.387 | 1.00 47.29 | C |
| ATOM | 3016 | CD  | LYS B 175 |  -4.339 | 13.895 | 28.117 | 1.00 54.56 | C |
| ATOM | 3017 | CE  | LYS B 175 |  -3.317 | 12.811 | 27.821 | 1.00 53.35 | C |
| ATOM | 3018 | NZ  | LYS B 175 |  -1.931 | 13.358 | 27.809 | 1.00 53.83 | N |
| ATOM | 3019 | N   | ASP B 176 |  -9.306 | 15.950 | 28.275 | 1.00 35.92 | N |
| ATOM | 3020 | CA  | ASP B 176 | -10.070 | 17.176 | 28.522 | 1.00 33.42 | C |
| ATOM | 3021 | C   | ASP B 176 | -11.526 | 17.072 | 28.069 | 1.00 33.69 | C |
| ATOM | 3022 | O   | ASP B 176 | -12.293 | 18.025 | 28.205 | 1.00 32.10 | O |
| ATOM | 3023 | CB  | ASP B 176 |  -9.391 | 18.387 | 27.857 | 1.00 37.93 | C |
| ATOM | 3024 | CG  | ASP B 176 |  -9.374 | 18.315 | 26.326 | 1.00 44.81 | C |
| ATOM | 3025 | OD1 | ASP B 176 | -10.135 | 17.530 | 25.718 | 1.00 39.09 | O |
| ATOM | 3026 | OD2 | ASP B 176 |  -8.591 | 19.081 | 25.721 | 1.00 49.31 | O |
| ATOM | 3027 | N   | SER B 177 | -11.879 | 15.923 | 27.501 | 1.00 31.41 | N |
| ATOM | 3028 | CA  | SER B 177 | -13.259 | 15.601 | 27.145 | 1.00 32.38 | C |
| ATOM | 3029 | C   | SER B 177 | -13.869 | 16.487 | 26.062 | 1.00 31.53 | C |
| ATOM | 3030 | O   | SER B 177 | -15.087 | 16.651 | 26.017 | 1.00 29.23 | O |
| ATOM | 3031 | CB  | SER B 177 | -14.142 | 15.665 | 28.390 | 1.00 29.13 | C |
| ATOM | 3032 | OG  | SER B 177 | -13.689 | 14.767 | 29.386 | 1.00 32.14 | O |
| ATOM | 3033 | N   | THR B 178 | -13.031 | 17.045 | 25.192 | 1.00 32.85 | N |
| ATOM | 3034 | CA  | THR B 178 | -13.521 | 17.873 | 24.091 | 1.00 31.34 | C |
| ATOM | 3035 | C   | THR B 178 | -13.603 | 17.102 | 22.779 | 1.00 29.53 | C |
| ATOM | 3036 | O   | THR B 178 | -13.145 | 15.960 | 22.680 | 1.00 31.68 | O |
| ATOM | 3037 | CB  | THR B 178 | -12.627 | 19.108 | 23.865 | 1.00 30.04 | C |
| ATOM | 3038 | CG2 | THR B 178 | -12.576 | 19.979 | 25.117 | 1.00 31.87 | C |
| ATOM | 3039 | OG1 | THR B 178 | -11.302 | 18.683 | 23.526 | 1.00 33.96 | O |
| ATOM | 3040 | N   | TYR B 179 | -14.199 | 17.738 | 21.776 | 1.00 26.97 | N |
| ATOM | 3041 | CA  | TYR B 179 | -14.198 | 17.225 | 20.411 | 1.00 27.62 | C |
| ATOM | 3042 | C   | TYR B 179 | -13.395 | 18.139 | 19.503 | 1.00 28.49 | C |
| ATOM | 3043 | O   | TYR B 179 | -13.193 | 19.316 | 19.809 | 1.00 27.60 | O |
| ATOM | 3044 | CB  | TYR B 179 | -15.620 | 17.110 | 19.862 | 1.00 23.93 | C |
| ATOM | 3045 | CG  | TYR B 179 | -16.540 | 16.224 | 20.659 | 1.00 32.03 | C |
| ATOM | 3046 | CD2 | TYR B 179 | -16.701 | 14.884 | 20.326 | 1.00 29.16 | C |
| ATOM | 3047 | CD1 | TYR B 179 | -17.266 | 16.729 | 21.735 | 1.00 27.55 | C |
| ATOM | 3048 | CE2 | TYR B 179 | -17.549 | 14.065 | 21.046 | 1.00 36.83 | C |
| ATOM | 3049 | CE1 | TYR B 179 | -18.118 | 15.915 | 22.467 | 1.00 35.32 | C |
| ATOM | 3050 | CZ  | TYR B 179 | -18.254 | 14.584 | 22.118 | 1.00 36.45 | C |

```
ATOM   3051   OH   TYR B 179    -19.096  13.768  22.837  1.00 41.00          O
ATOM   3052   N    SER B 180    -12.945  17.597  18.379  1.00 27.52          N
ATOM   3053   CA   SER B 180    -12.437  18.418  17.288  1.00 29.79          C
ATOM   3054   C    SER B 180    -13.143  18.002  16.000  1.00 28.83          C
ATOM   3055   O    SER B 180    -13.591  16.862  15.875  1.00 26.78          O
ATOM   3056   CB   SER B 180    -10.921  18.287  17.150  1.00 34.04          C
ATOM   3057   OG   SER B 180    -10.255  18.889  18.246  1.00 36.05          O
ATOM   3058   N    LEU B 181    -13.252  18.928  15.053  1.00 27.50          N
ATOM   3059   CA   LEU B 181    -13.984  18.670  13.819  1.00 29.24          C
ATOM   3060   C    LEU B 181    -13.206  19.150  12.595  1.00 34.49          C
ATOM   3061   O    LEU B 181    -12.583  20.214  12.610  1.00 29.64          O
ATOM   3062   CB   LEU B 181    -15.367  19.337  13.866  1.00 31.76          C
ATOM   3063   CG   LEU B 181    -16.315  19.197  12.662  1.00 33.92          C
ATOM   3064   CD1  LEU B 181    -17.770  19.237  13.115  1.00 33.67          C
ATOM   3065   CD2  LEU B 181    -16.071  20.275  11.620  1.00 27.36          C
ATOM   3066   N    SER B 182    -13.266  18.355  11.534  1.00 30.45          N
ATOM   3067   CA   SER B 182    -12.622  18.683  10.273  1.00 32.39          C
ATOM   3068   C    SER B 182    -13.653  18.706   9.157  1.00 34.50          C
ATOM   3069   O    SER B 182    -14.346  17.715   8.935  1.00 32.11          O
ATOM   3070   CB   SER B 182    -11.521  17.671   9.953  1.00 35.13          C
ATOM   3071   OG   SER B 182    -10.952  17.929   8.684  1.00 35.29          O
ATOM   3072   N    SER B 183    -13.769  19.841   8.473  1.00 32.48          N
ATOM   3073   CA   SER B 183    -14.666  19.946   7.327  1.00 31.00          C
ATOM   3074   C    SER B 183    -13.877  20.240   6.058  1.00 37.54          C
ATOM   3075   O    SER B 183    -13.014  21.119   6.041  1.00 34.24          O
ATOM   3076   CB   SER B 183    -15.720  21.028   7.548  1.00 28.88          C
ATOM   3077   OG   SER B 183    -16.568  21.127   6.415  1.00 34.00          O
ATOM   3078   N    THR B 184    -14.185  19.495   5.002  1.00 29.33          N
ATOM   3079   CA   THR B 184    -13.490  19.618   3.733  1.00 32.43          C
ATOM   3080   C    THR B 184    -14.449  20.002   2.609  1.00 33.53          C
ATOM   3081   O    THR B 184    -15.375  19.258   2.296  1.00 30.24          O
ATOM   3082   CB   THR B 184    -12.778  18.303   3.347  1.00 30.27          C
ATOM   3083   CG2  THR B 184    -12.075  18.448   1.993  1.00 34.53          C
ATOM   3084   OG1  THR B 184    -11.813  17.966   4.348  1.00 43.02          O
ATOM   3085   N    LEU B 185    -14.212  21.164   2.013  1.00 29.43          N
ATOM   3086   CA   LEU B 185    -14.950  21.610   0.838  1.00 26.42          C
ATOM   3087   C    LEU B 185    -14.195  21.216  -0.428  1.00 31.67          C
ATOM   3088   O    LEU B 185    -13.021  21.549  -0.587  1.00 32.58          O
ATOM   3089   CB   LEU B 185    -15.162  23.123   0.882  1.00 26.19          C
ATOM   3090   CG   LEU B 185    -15.801  23.777  -0.346  1.00 33.72          C
ATOM   3091   CD1  LEU B 185    -17.290  23.451  -0.426  1.00 29.32          C
ATOM   3092   CD2  LEU B 185    -15.570  25.285  -0.336  1.00 29.64          C
ATOM   3093   N    THR B 186    -14.862  20.503  -1.330  1.00 27.07          N
ATOM   3094   CA   THR B 186    -14.200  20.043  -2.544  1.00 32.13          C
ATOM   3095   C    THR B 186    -14.797  20.676  -3.794  1.00 34.19          C
ATOM   3096   O    THR B 186    -15.999  20.569  -4.046  1.00 32.51          O
ATOM   3097   CB   THR B 186    -14.265  18.512  -2.672  1.00 35.70          C
ATOM   3098   CG2  THR B 186    -13.532  18.054  -3.919  1.00 33.31          C
ATOM   3099   OG1  THR B 186    -13.652  17.913  -1.522  1.00 40.62          O
ATOM   3100   N    LEU B 187    -13.941  21.346  -4.562  1.00 35.11          N
ATOM   3101   CA   LEU B 187    -14.315  21.945  -5.843  1.00 35.77          C
ATOM   3102   C    LEU B 187    -13.339  21.525  -6.932  1.00 37.88          C
ATOM   3103   O    LEU B 187    -12.206  21.145  -6.642  1.00 36.56          O
ATOM   3104   CB   LEU B 187    -14.333  23.471  -5.755  1.00 30.60          C
ATOM   3105   CG   LEU B 187    -15.286  24.143  -4.775  1.00 36.33          C
ATOM   3106   CD1  LEU B 187    -15.031  25.641  -4.767  1.00 43.76          C
ATOM   3107   CD2  LEU B 187    -16.729  23.835  -5.147  1.00 50.34          C
ATOM   3108   N    SER B 188    -13.767  21.609  -8.187  1.00 38.12          N
ATOM   3109   CA   SER B 188    -12.839  21.434  -9.297  1.00 37.37          C
ATOM   3110   C    SER B 188    -11.888  22.627  -9.343  1.00 37.79          C
ATOM   3111   O    SER B 188    -12.169  23.681  -8.761  1.00 33.69          O
ATOM   3112   CB   SER B 188    -13.584  21.297 -10.625  1.00 36.22          C
ATOM   3113   OG   SER B 188    -14.208  22.519 -10.973  1.00 39.78          O
ATOM   3114   N    LYS B 189    -10.765  22.460 -10.031  1.00 38.51          N
ATOM   3115   CA   LYS B 189     -9.809  23.546 -10.193  1.00 37.38          C
ATOM   3116   C    LYS B 189    -10.460  24.721 -10.915  1.00 37.61          C
ATOM   3117   O    LYS B 189    -10.331  25.869 -10.491  1.00 42.81          O
ATOM   3118   CB   LYS B 189     -8.576  23.064 -10.959  1.00 43.42          C
ATOM   3119   CG   LYS B 189     -7.574  24.158 -11.293  1.00 46.58          C
ATOM   3120   CD   LYS B 189     -6.453  23.615 -12.166  1.00 47.94          C
ATOM   3121   CE   LYS B 189     -5.477  24.704 -12.561  1.00 50.50          C
```

```
ATOM   3122  NZ   LYS B 189    -4.369  24.160 -13.395  1.00 67.88      N
ATOM   3123  N    ALA B 190   -11.175  24.415 -11.995  1.00 37.86      N
ATOM   3124  CA   ALA B 190   -11.862  25.429 -12.791  1.00 38.31      C
ATOM   3125  C    ALA B 190   -12.825  26.252 -11.947  1.00 41.13      C
ATOM   3126  O    ALA B 190   -12.859  27.477 -12.057  1.00 45.90      O
ATOM   3127  CB   ALA B 190   -12.602  24.777 -13.953  1.00 30.47      C
ATOM   3128  N    ASP B 191   -13.602  25.582 -11.100  1.00 38.92      N
ATOM   3129  CA   ASP B 191   -14.536  26.287 -10.226  1.00 37.31      C
ATOM   3130  C    ASP B 191   -13.796  27.103  -9.175  1.00 35.97      C
ATOM   3131  O    ASP B 191   -14.211  28.214  -8.831  1.00 35.16      O
ATOM   3132  CB   ASP B 191   -15.497  25.308  -9.542  1.00 40.65      C
ATOM   3133  CG   ASP B 191   -16.675  24.932 -10.425  1.00 54.48      C
ATOM   3134  OD1  ASP B 191   -16.840  25.554 -11.498  1.00 48.85      O
ATOM   3135  OD2  ASP B 191   -17.445  24.024 -10.039  1.00 50.23      O
ATOM   3136  N    TYR B 192   -12.700  26.549  -8.667  1.00 35.50      N
ATOM   3137  CA   TYR B 192   -11.948  27.193  -7.595  1.00 34.33      C
ATOM   3138  C    TYR B 192   -11.381  28.543  -8.022  1.00 37.59      C
ATOM   3139  O    TYR B 192   -11.395  29.505  -7.256  1.00 35.83      O
ATOM   3140  CB   TYR B 192   -10.814  26.288  -7.115  1.00 34.12      C
ATOM   3141  CG   TYR B 192    -9.932  26.940  -6.076  1.00 36.45      C
ATOM   3142  CD1  TYR B 192   -10.429  27.251  -4.815  1.00 34.80      C
ATOM   3143  CD2  TYR B 192    -8.606  27.243  -6.350  1.00 32.17      C
ATOM   3144  CE1  TYR B 192    -9.632  27.849  -3.857  1.00 32.72      C
ATOM   3145  CE2  TYR B 192    -7.797  27.838  -5.394  1.00 34.13      C
ATOM   3146  CZ   TYR B 192    -8.319  28.142  -4.152  1.00 31.36      C
ATOM   3147  OH   TYR B 192    -7.526  28.732  -3.197  1.00 35.96      O
ATOM   3148  N    GLU B 193   -10.895  28.609  -9.255  1.00 39.80      N
ATOM   3149  CA   GLU B 193   -10.237  29.811  -9.763  1.00 43.18      C
ATOM   3150  C    GLU B 193   -11.213  30.900 -10.210  1.00 39.55      C
ATOM   3151  O    GLU B 193   -10.799  32.022 -10.496  1.00 44.13      O
ATOM   3152  CB   GLU B 193    -9.311  29.440 -10.921  1.00 44.18      C
ATOM   3153  CG   GLU B 193    -8.172  28.528 -10.515  1.00 46.87      C
ATOM   3154  CD   GLU B 193    -7.393  28.010 -11.704  1.00 66.53      C
ATOM   3155  OE1  GLU B 193    -7.965  27.968 -12.815  1.00 70.34      O
ATOM   3156  OE2  GLU B 193    -6.210  27.648 -11.529  1.00 73.19      O
ATOM   3157  N    LYS B 194   -12.502  30.575 -10.261  1.00 41.15      N
ATOM   3158  CA   LYS B 194   -13.522  31.554 -10.634  1.00 39.86      C
ATOM   3159  C    LYS B 194   -13.955  32.429  -9.461  1.00 44.25      C
ATOM   3160  O    LYS B 194   -14.816  33.295  -9.615  1.00 42.88      O
ATOM   3161  CB   LYS B 194   -14.754  30.855 -11.214  1.00 43.14      C
ATOM   3162  CG   LYS B 194   -14.506  30.110 -12.507  1.00 50.46      C
ATOM   3163  CD   LYS B 194   -15.768  29.418 -12.991  1.00 54.94      C
ATOM   3164  CE   LYS B 194   -15.503  28.612 -14.255  1.00 65.50      C
ATOM   3165  NZ   LYS B 194   -14.922  29.454 -15.339  1.00 69.52      N
ATOM   3166  N    HIS B 195   -13.373  32.204  -8.288  1.00 42.19      N
ATOM   3167  CA   HIS B 195   -13.799  32.939  -7.099  1.00 42.19      C
ATOM   3168  C    HIS B 195   -12.625  33.455  -6.285  1.00 36.87      C
ATOM   3169  O    HIS B 195   -11.498  32.976  -6.423  1.00 37.36      O
ATOM   3170  CB   HIS B 195   -14.687  32.061  -6.214  1.00 38.63      C
ATOM   3171  CG   HIS B 195   -15.894  31.518  -6.914  1.00 41.06      C
ATOM   3172  CD2  HIS B 195   -16.180  30.270  -7.356  1.00 44.21      C
ATOM   3173  ND1  HIS B 195   -16.987  32.296  -7.224  1.00 43.75      N
ATOM   3174  CE1  HIS B 195   -17.894  31.554  -7.834  1.00 44.35      C
ATOM   3175  NE2  HIS B 195   -17.430  30.321  -7.926  1.00 44.01      N
ATOM   3176  N    LYS B 196   -12.908  34.424  -5.420  1.00 38.25      N
ATOM   3177  CA   LYS B 196   -11.876  35.084  -4.639  1.00 35.29      C
ATOM   3178  C    LYS B 196   -11.915  34.716  -3.152  1.00 39.07      C
ATOM   3179  O    LYS B 196   -10.942  34.190  -2.615  1.00 39.45      O
ATOM   3180  CB   LYS B 196   -11.998  36.602  -4.793  1.00 41.25      C
ATOM   3181  CG   LYS B 196   -10.931  37.391  -4.047  1.00 43.11      C
ATOM   3182  CD   LYS B 196   -11.215  38.884  -4.099  1.00 57.11      C
ATOM   3183  CE   LYS B 196   -10.103  39.686  -3.447  1.00 60.58      C
ATOM   3184  NZ   LYS B 196   -10.341  41.150  -3.566  1.00 75.28      N
ATOM   3185  N    VAL B 197   -13.032  35.002  -2.488  1.00 33.64      N
ATOM   3186  CA   VAL B 197   -13.108  34.851  -1.037  1.00 41.13      C
ATOM   3187  C    VAL B 197   -13.640  33.478  -0.620  1.00 39.79      C
ATOM   3188  O    VAL B 197   -14.758  33.098  -0.973  1.00 35.93      O
ATOM   3189  CB   VAL B 197   -13.993  35.943  -0.400  1.00 39.79      C
ATOM   3190  CG1  VAL B 197   -13.964  35.829   1.117  1.00 33.80      C
ATOM   3191  CG2  VAL B 197   -13.532  37.330  -0.834  1.00 36.75      C
ATOM   3192  N    TYR B 198   -12.827  32.743   0.134  1.00 36.80      N
```

```
ATOM   3193  CA  TYR B 198    -13.223  31.442   0.662  1.00 31.41           C
ATOM   3194  C   TYR B 198    -13.289  31.518   2.173  1.00 34.47           C
ATOM   3195  O   TYR B 198    -12.324  31.909   2.821  1.00 35.65           O
ATOM   3196  CB  TYR B 198    -12.252  30.347   0.210  1.00 29.88           C
ATOM   3197  CG  TYR B 198    -12.394  30.041  -1.259  1.00 32.49           C
ATOM   3198  CD1 TYR B 198    -11.774  30.838  -2.219  1.00 31.45           C
ATOM   3199  CD2 TYR B 198    -13.179  28.978  -1.694  1.00 30.07           C
ATOM   3200  CE1 TYR B 198    -11.919  30.573  -3.572  1.00 35.86           C
ATOM   3201  CE2 TYR B 198    -13.327  28.706  -3.043  1.00 31.84           C
ATOM   3202  CZ  TYR B 198    -12.695  29.509  -3.975  1.00 31.78           C
ATOM   3203  OH  TYR B 198    -12.842  29.245  -5.313  1.00 37.12           O
ATOM   3204  N   ALA B 199    -14.435  31.148   2.731  1.00 31.33           N
ATOM   3205  CA  ALA B 199    -14.672  31.330   4.155  1.00 35.87           C
ATOM   3206  C   ALA B 199    -15.414  30.150   4.755  1.00 37.75           C
ATOM   3207  O   ALA B 199    -16.236  29.517   4.090  1.00 29.26           O
ATOM   3208  CB  ALA B 199    -15.454  32.614   4.394  1.00 28.89           C
ATOM   3209  N   CYS B 200    -15.114  29.844   6.012  1.00 29.79           N
ATOM   3210  CA  CYS B 200    -15.967  28.940   6.764  1.00 30.62           C
ATOM   3211  C   CYS B 200    -16.481  29.695   7.980  1.00 34.82           C
ATOM   3212  O   CYS B 200    -15.727  30.376   8.673  1.00 35.07           O
ATOM   3213  CB  CYS B 200    -15.232  27.651   7.157  1.00 39.10           C
ATOM   3214  SG  CYS B 200    -13.954  27.783   8.432  1.00 54.20           S
ATOM   3215  N   GLU B 201    -17.785  29.598   8.205  1.00 34.80           N
ATOM   3216  CA  GLU B 201    -18.438  30.312   9.289  1.00 35.26           C
ATOM   3217  C   GLU B 201    -18.905  29.313  10.338  1.00 33.11           C
ATOM   3218  O   GLU B 201    -19.670  28.395  10.036  1.00 30.25           O
ATOM   3219  CB  GLU B 201    -19.619  31.134   8.764  1.00 31.34           C
ATOM   3220  CG  GLU B 201    -20.333  31.938   9.838  1.00 46.76           C
ATOM   3221  CD  GLU B 201    -21.725  32.382   9.423  1.00 57.59           C
ATOM   3222  OE1 GLU B 201    -22.650  31.538   9.431  1.00 59.83           O
ATOM   3223  OE2 GLU B 201    -21.893  33.575   9.093  1.00 62.46           O
ATOM   3224  N   VAL B 202    -18.450  29.490  11.572  1.00 33.14           N
ATOM   3225  CA  VAL B 202    -18.715  28.498  12.604  1.00 34.14           C
ATOM   3226  C   VAL B 202    -19.683  28.996  13.674  1.00 34.64           C
ATOM   3227  O   VAL B 202    -19.512  30.079  14.236  1.00 32.69           O
ATOM   3228  CB  VAL B 202    -17.408  28.046  13.276  1.00 32.83           C
ATOM   3229  CG1 VAL B 202    -17.699  27.136  14.458  1.00 27.65           C
ATOM   3230  CG2 VAL B 202    -16.513  27.355  12.258  1.00 31.56           C
ATOM   3231  N   THR B 203    -20.702  28.185  13.942  1.00 30.80           N
ATOM   3232  CA  THR B 203    -21.670  28.455  14.997  1.00 34.07           C
ATOM   3233  C   THR B 203    -21.556  27.394  16.091  1.00 36.01           C
ATOM   3234  O   THR B 203    -21.518  26.196  15.802  1.00 34.64           O
ATOM   3235  CB  THR B 203    -23.111  28.477  14.444  1.00 36.63           C
ATOM   3236  CG2 THR B 203    -24.102  28.883  15.526  1.00 36.15           C
ATOM   3237  OG1 THR B 203    -23.190  29.403  13.356  1.00 45.86           O
ATOM   3238  N   HIS B 204    -21.499  27.841  17.343  1.00 32.76           N
ATOM   3239  CA  HIS B 204    -21.356  26.946  18.487  1.00 34.42           C
ATOM   3240  C   HIS B 204    -21.850  27.620  19.764  1.00 33.50           C
ATOM   3241  O   HIS B 204    -21.750  28.837  19.909  1.00 34.28           O
ATOM   3242  CB  HIS B 204    -19.895  26.510  18.656  1.00 29.00           C
ATOM   3243  CG  HIS B 204    -19.689  25.483  19.728  1.00 38.33           C
ATOM   3244  CD2 HIS B 204    -19.847  24.138  19.717  1.00 32.84           C
ATOM   3245  ND1 HIS B 204    -19.266  25.810  20.999  1.00 29.49           N
ATOM   3246  CE1 HIS B 204    -19.177  24.710  21.726  1.00 34.17           C
ATOM   3247  NE2 HIS B 204    -19.522  23.681  20.973  1.00 33.34           N
ATOM   3248  N   GLN B 205    -22.367  26.816  20.687  1.00 33.90           N
ATOM   3249  CA  GLN B 205    -22.892  27.307  21.961  1.00 33.84           C
ATOM   3250  C   GLN B 205    -21.905  28.198  22.725  1.00 36.35           C
ATOM   3251  O   GLN B 205    -22.313  29.126  23.425  1.00 40.21           O
ATOM   3252  CB  GLN B 205    -23.309  26.121  22.835  1.00 33.40           C
ATOM   3253  CG  GLN B 205    -23.981  26.507  24.136  1.00 49.24           C
ATOM   3254  CD  GLN B 205    -24.475  25.304  24.914  1.00 50.37           C
ATOM   3255  NE2 GLN B 205    -24.852  25.527  26.168  1.00 57.58           N
ATOM   3256  OE1 GLN B 205    -24.518  24.186  24.396  1.00 53.77           O
ATOM   3257  N   GLY B 206    -20.610  27.931  22.578  1.00 30.24           N
ATOM   3258  CA  GLY B 206    -19.587  28.728  23.237  1.00 32.96           C
ATOM   3259  C   GLY B 206    -19.239  30.036  22.542  1.00 34.80           C
ATOM   3260  O   GLY B 206    -18.466  30.834  23.064  1.00 30.49           O
ATOM   3261  N   LEU B 207    -19.806  30.260  21.363  1.00 38.67           N
ATOM   3262  CA  LEU B 207    -19.553  31.492  20.619  1.00 41.75           C
ATOM   3263  C   LEU B 207    -20.765  32.412  20.674  1.00 40.10           C
```

| ATOM | 3264 | O | LEU | B | 207 | -21.848 | 32.050 | 20.210 | 1.00 | 42.41 | O |
| ATOM | 3265 | CB | LEU | B | 207 | -19.204 | 31.180 | 19.165 | 1.00 | 34.75 | C |
| ATOM | 3266 | CG | LEU | B | 207 | -17.957 | 30.336 | 18.917 | 1.00 | 35.04 | C |
| ATOM | 3267 | CD1 | LEU | B | 207 | -17.871 | 29.946 | 17.444 | 1.00 | 32.97 | C |
| ATOM | 3268 | CD2 | LEU | B | 207 | -16.709 | 31.087 | 19.362 | 1.00 | 34.44 | C |
| ATOM | 3269 | N | SER | B | 208 | -20.582 | 33.600 | 21.239 | 1.00 | 41.65 | N |
| ATOM | 3270 | CA | SER | B | 208 | -21.685 | 34.551 | 21.366 | 1.00 | 50.19 | C |
| ATOM | 3271 | C | SER | B | 208 | -22.189 | 34.991 | 19.989 | 1.00 | 43.51 | C |
| ATOM | 3272 | O | SER | B | 208 | -23.367 | 35.307 | 19.824 | 1.00 | 51.88 | O |
| ATOM | 3273 | CB | SER | B | 208 | -21.260 | 35.758 | 22.207 | 1.00 | 41.21 | C |
| ATOM | 3274 | OG | SER | B | 208 | -19.985 | 36.239 | 21.815 | 1.00 | 63.38 | O |
| ATOM | 3275 | N | SER | B | 209 | -21.296 | 34.995 | 19.002 | 1.00 | 40.06 | N |
| ATOM | 3276 | CA | SER | B | 209 | -21.682 | 35.228 | 17.613 | 1.00 | 44.47 | C |
| ATOM | 3277 | C | SER | B | 209 | -20.812 | 34.364 | 16.695 | 1.00 | 38.33 | C |
| ATOM | 3278 | O | SER | B | 209 | -19.696 | 34.005 | 17.067 | 1.00 | 43.33 | O |
| ATOM | 3279 | CB | SER | B | 209 | -21.557 | 36.715 | 17.253 | 1.00 | 45.07 | C |
| ATOM | 3280 | OG | SER | B | 209 | -20.203 | 37.102 | 17.120 | 1.00 | 49.50 | O |
| ATOM | 3281 | N | PRO | B | 210 | -21.323 | 34.017 | 15.499 | 1.00 | 44.65 | N |
| ATOM | 3282 | CA | PRO | B | 210 | -20.580 | 33.149 | 14.574 | 1.00 | 38.77 | C |
| ATOM | 3283 | C | PRO | B | 210 | -19.176 | 33.661 | 14.264 | 1.00 | 41.23 | C |
| ATOM | 3284 | O | PRO | B | 210 | -18.966 | 34.867 | 14.141 | 1.00 | 45.61 | O |
| ATOM | 3285 | CB | PRO | B | 210 | -21.450 | 33.156 | 13.315 | 1.00 | 39.30 | C |
| ATOM | 3286 | CG | PRO | B | 210 | -22.826 | 33.399 | 13.826 | 1.00 | 43.89 | C |
| ATOM | 3287 | CD | PRO | B | 210 | -22.658 | 34.357 | 14.974 | 1.00 | 40.75 | C |
| ATOM | 3288 | N | VAL | B | 211 | -18.224 | 32.741 | 14.155 | 1.00 | 39.40 | N |
| ATOM | 3289 | CA | VAL | B | 211 | -16.834 | 33.090 | 13.883 | 1.00 | 39.75 | C |
| ATOM | 3290 | C | VAL | B | 211 | -16.475 | 32.703 | 12.452 | 1.00 | 40.69 | C |
| ATOM | 3291 | O | VAL | B | 211 | -16.747 | 31.582 | 12.020 | 1.00 | 36.87 | O |
| ATOM | 3292 | CB | VAL | B | 211 | -15.877 | 32.398 | 14.880 | 1.00 | 38.99 | C |
| ATOM | 3293 | CG1 | VAL | B | 211 | -14.429 | 32.537 | 14.436 | 1.00 | 34.79 | C |
| ATOM | 3294 | CG2 | VAL | B | 211 | -16.071 | 32.973 | 16.272 | 1.00 | 40.49 | C |
| ATOM | 3295 | N | THR | B | 212 | -15.880 | 33.639 | 11.716 | 1.00 | 35.72 | N |
| ATOM | 3296 | CA | THR | B | 212 | -15.550 | 33.409 | 10.314 | 1.00 | 39.63 | C |
| ATOM | 3297 | C | THR | B | 212 | -14.055 | 33.500 | 10.046 | 1.00 | 35.76 | C |
| ATOM | 3298 | O | THR | B | 212 | -13.424 | 34.518 | 10.323 | 1.00 | 42.94 | O |
| ATOM | 3299 | CB | THR | B | 212 | -16.272 | 34.414 | 9.391 | 1.00 | 38.85 | C |
| ATOM | 3300 | CG2 | THR | B | 212 | -15.814 | 34.238 | 7.953 | 1.00 | 37.83 | C |
| ATOM | 3301 | OG1 | THR | B | 212 | -17.687 | 34.196 | 9.461 | 1.00 | 46.70 | O |
| ATOM | 3302 | N | LYS | B | 213 | -13.491 | 32.424 | 9.513 | 1.00 | 33.60 | N |
| ATOM | 3303 | CA | LYS | B | 213 | -12.120 | 32.451 | 9.029 | 1.00 | 36.19 | C |
| ATOM | 3304 | C | LYS | B | 213 | -12.155 | 32.386 | 7.514 | 1.00 | 38.31 | C |
| ATOM | 3305 | O | LYS | B | 213 | -12.964 | 31.659 | 6.938 | 1.00 | 40.76 | O |
| ATOM | 3306 | CB | LYS | B | 213 | -11.298 | 31.295 | 9.602 | 1.00 | 35.13 | C |
| ATOM | 3307 | CG | LYS | B | 213 | -11.186 | 31.301 | 11.120 | 1.00 | 41.13 | C |
| ATOM | 3308 | CD | LYS | B | 213 | -10.462 | 32.537 | 11.635 | 1.00 | 36.48 | C |
| ATOM | 3309 | CE | LYS | B | 213 | -10.432 | 32.550 | 13.161 | 1.00 | 36.86 | C |
| ATOM | 3310 | NZ | LYS | B | 213 | -9.699 | 33.727 | 13.703 | 1.00 | 51.87 | N |
| ATOM | 3311 | N | SER | B | 214 | -11.283 | 33.149 | 6.869 | 1.00 | 36.97 | N |
| ATOM | 3312 | CA | SER | B | 214 | -11.301 | 33.233 | 5.420 | 1.00 | 35.10 | C |
| ATOM | 3313 | C | SER | B | 214 | -9.934 | 33.547 | 4.827 | 1.00 | 37.64 | C |
| ATOM | 3314 | O | SER | B | 214 | -9.021 | 33.986 | 5.524 | 1.00 | 41.48 | O |
| ATOM | 3315 | CB | SER | B | 214 | -12.307 | 34.291 | 4.971 | 1.00 | 35.76 | C |
| ATOM | 3316 | OG | SER | B | 214 | -11.918 | 35.574 | 5.422 | 1.00 | 40.23 | O |
| ATOM | 3317 | N | PHE | B | 215 | -9.804 | 33.297 | 3.530 | 1.00 | 36.45 | N |
| ATOM | 3318 | CA | PHE | B | 215 | -8.651 | 33.746 | 2.767 | 1.00 | 39.24 | C |
| ATOM | 3319 | C | PHE | B | 215 | -9.114 | 34.202 | 1.388 | 1.00 | 45.83 | C |
| ATOM | 3320 | O | PHE | B | 215 | -10.185 | 33.804 | 0.916 | 1.00 | 37.02 | O |
| ATOM | 3321 | CB | PHE | B | 215 | -7.598 | 32.640 | 2.645 | 1.00 | 32.01 | C |
| ATOM | 3322 | CG | PHE | B | 215 | -8.066 | 31.428 | 1.878 | 1.00 | 38.83 | C |
| ATOM | 3323 | CD1 | PHE | B | 215 | -8.729 | 30.397 | 2.524 | 1.00 | 31.21 | C |
| ATOM | 3324 | CD2 | PHE | B | 215 | -7.830 | 31.315 | 0.516 | 1.00 | 35.76 | C |
| ATOM | 3325 | CE1 | PHE | B | 215 | -9.156 | 29.279 | 1.825 | 1.00 | 33.60 | C |
| ATOM | 3326 | CE2 | PHE | B | 215 | -8.251 | 30.201 | -0.190 | 1.00 | 35.19 | C |
| ATOM | 3327 | CZ | PHE | B | 215 | -8.915 | 29.180 | 0.466 | 1.00 | 34.00 | C |
| ATOM | 3328 | N | ASN | B | 216 | -8.316 | 35.057 | 0.761 | 1.00 | 45.06 | N |
| ATOM | 3329 | CA | ASN | B | 216 | -8.533 | 35.429 | -0.629 | 1.00 | 42.67 | C |
| ATOM | 3330 | C | ASN | B | 216 | -7.591 | 34.626 | -1.499 | 1.00 | 39.47 | C |
| ATOM | 3331 | O | ASN | B | 216 | -6.388 | 34.607 | -1.255 | 1.00 | 45.25 | O |
| ATOM | 3332 | CB | ASN | B | 216 | -8.310 | 36.926 | -0.843 | 1.00 | 45.51 | C |
| ATOM | 3333 | CG | ASN | B | 216 | -9.256 | 37.778 | -0.024 | 1.00 | 49.43 | C |
| ATOM | 3334 | ND2 | ASN | B | 216 | -8.785 | 38.943 | 0.402 | 1.00 | 55.43 | N |

```
ATOM   3335  OD1 ASN B 216    -10.396  37.393   0.227  1.00 51.90        O
ATOM   3336  N   ARG B 217     -8.138  33.946  -2.499  1.00 39.98        N
ATOM   3337  CA  ARG B 217     -7.327  33.135  -3.396  1.00 43.37        C
ATOM   3338  C   ARG B 217     -6.257  33.985  -4.069  1.00 49.33        C
ATOM   3339  O   ARG B 217     -6.559  35.019  -4.665  1.00 52.85        O
ATOM   3340  CB  ARG B 217     -8.207  32.457  -4.449  1.00 42.40        C
ATOM   3341  CG  ARG B 217     -7.441  31.578  -5.420  1.00 38.23        C
ATOM   3342  CD  ARG B 217     -8.358  31.012  -6.485  1.00 38.31        C
ATOM   3343  NE  ARG B 217     -9.059  32.068  -7.204  1.00 42.76        N
ATOM   3344  CZ  ARG B 217     -8.588  32.665  -8.292  1.00 51.33        C
ATOM   3345  NH1 ARG B 217     -7.414  32.303  -8.793  1.00 42.73        N
ATOM   3346  NH2 ARG B 217     -9.294  33.622  -8.883  1.00 48.38        N
ATOM   3347  N   GLY B 218     -5.004  33.555  -3.952  1.00 53.01        N
ATOM   3348  CA  GLY B 218     -3.897  34.253  -4.582  1.00 57.34        C
ATOM   3349  C   GLY B 218     -3.338  35.410  -3.771  1.00 63.03        C
ATOM   3350  O   GLY B 218     -2.818  36.373  -4.334  1.00 67.80        O
ATOM   3351  N   GLU B 219     -3.443  35.321  -2.449  1.00 60.01        N
ATOM   3352  CA  GLU B 219     -2.900  36.353  -1.571  1.00 61.79        C
ATOM   3353  C   GLU B 219     -2.130  35.742  -0.405  1.00 61.79        C
ATOM   3354  O   GLU B 219     -1.976  34.525  -0.321  1.00 69.81        O
ATOM   3355  CB  GLU B 219     -4.015  37.259  -1.042  1.00 62.53        C
ATOM   3356  CG  GLU B 219     -4.663  38.151  -2.096  1.00 56.22        C
ATOM   3357  CD  GLU B 219     -5.625  39.161  -1.489  1.00 74.19        C
ATOM   3358  OE1 GLU B 219     -5.593  39.345  -0.252  1.00 78.86        O
ATOM   3359  OE2 GLU B 219     -6.413  39.770  -2.247  1.00 64.28        O
TER
ATOM   3360  N   THR C 152    -29.241 -45.858  26.627  1.00 80.47   D000 N
ATOM   3361  CA  THR C 152    -29.487 -45.616  28.045  1.00 91.58   D000 C
ATOM   3362  C   THR C 152    -29.269 -44.146  28.405  1.00 88.87   D000 C
ATOM   3363  O   THR C 152    -28.934 -43.329  27.545  1.00 88.60   D000 O
ATOM   3364  CB  THR C 152    -28.582 -46.499  28.936  1.00 90.05   D000 C
ATOM   3365  CG2 THR C 152    -29.009 -47.960  28.857  1.00 70.32   D000 C
ATOM   3366  OG1 THR C 152    -27.220 -46.382  28.506  1.00 79.38   D000 O
ATOM   3367  N   CYS C 153    -29.465 -43.813  29.677  1.00 83.40   D000 N
ATOM   3368  CA  CYS C 153    -29.261 -42.446  30.143  1.00 72.69   D000 C
ATOM   3369  C   CYS C 153    -28.836 -42.415  31.611  1.00 63.73   D000 C
ATOM   3370  O   CYS C 153    -28.923 -43.419  32.318  1.00 52.26   D000 O
ATOM   3371  CB  CYS C 153    -30.532 -41.616  29.941  1.00 73.56   D000 C
ATOM   3372  SG  CYS C 153    -30.281 -39.826  30.081  1.00 98.30   D000 S
ATOM   3373  N   CYS C 154    -28.370 -41.254  32.059  1.00 61.15   D000 N
ATOM   3374  CA  CYS C 154    -27.916 -41.085  33.434  1.00 58.46   D000 C
ATOM   3375  C   CYS C 154    -29.071 -41.141  34.432  1.00 51.28   D000 C
ATOM   3376  O   CYS C 154    -30.208 -40.827  34.086  1.00 47.54   D000 O
ATOM   3377  CB  CYS C 154    -27.160 -39.758  33.580  1.00 53.06   D000 C
ATOM   3378  SG  CYS C 154    -25.446 -39.818  33.000  1.00 71.64   D000 S
ATOM   3379  N   PRO C 155    -28.781 -41.556  35.674  1.00 43.23   D000 N
ATOM   3380  CA  PRO C 155    -29.767 -41.494  36.757  1.00 48.50   D000 C
ATOM   3381  C   PRO C 155    -30.193 -40.062  37.062  1.00 49.23   D000 C
ATOM   3382  O   PRO C 155    -29.481 -39.117  36.708  1.00 42.32   D000 O
ATOM   3383  CB  PRO C 155    -29.020 -42.104  37.953  1.00 42.80   D000 C
ATOM   3384  CG  PRO C 155    -27.576 -41.991  37.609  1.00 50.48   D000 C
ATOM   3385  CD  PRO C 155    -27.514 -42.158  36.122  1.00 50.94   D000 C
ATOM   3386  N   VAL C 156    -31.343 -39.913  37.713  1.00 40.74   D000 N
ATOM   3387  CA  VAL C 156    -31.863 -38.598  38.077  1.00 48.19   D000 C
ATOM   3388  C   VAL C 156    -30.837 -37.822  38.897  1.00 42.17   D000 C
ATOM   3389  O   VAL C 156    -30.149 -38.403  39.739  1.00 40.87   D000 O
ATOM   3390  CB  VAL C 156    -33.183 -38.717  38.875  1.00 54.12   D000 C
ATOM   3391  CG1 VAL C 156    -33.775 -37.341  39.149  1.00 44.84   D000 C
ATOM   3392  CG2 VAL C 156    -34.181 -39.590  38.122  1.00 50.34   D000 C
ATOM   3393  N   ASN C 157    -30.727 -36.522  38.610  1.00 41.21   D000 N
ATOM   3394  CA  ASN C 157    -29.820 -35.593  39.292  1.00 41.02   D000 C
ATOM   3395  C   ASN C 157    -28.353 -35.786  38.927  1.00 39.65   D000 C
ATOM   3396  O   ASN C 157    -27.481 -35.121  39.485  1.00 47.91   D000 O
ATOM   3397  CB  ASN C 157    -29.979 -35.689  40.812  1.00 45.93   D000 C
ATOM   3398  CG  ASN C 157    -31.360 -35.289  41.276  1.00 51.89   D000 C
ATOM   3399  ND2 ASN C 157    -31.885 -36.011  42.260  1.00 51.21   D000 N
ATOM   3400  OD1 ASN C 157    -31.953 -34.347  40.752  1.00 55.30   D000 O
ATOM   3401  N   TRP C 158    -28.081 -36.696  37.997  1.00 38.04   D000 N
ATOM   3402  CA  TRP C 158    -26.737 -36.841  37.449  1.00 35.52   D000 C
ATOM   3403  C   TRP C 158    -26.694 -36.212  36.061  1.00 41.15   D000 C
ATOM   3404  O   TRP C 158    -27.720 -36.118  35.387  1.00 32.47   D000 O
```

```
ATOM  3405  CB   TRP C 158   -26.323 -38.313  37.394  1.00 35.29      D000 C
ATOM  3406  CG   TRP C 158   -26.115 -38.930  38.751  1.00 40.11      D000 C
ATOM  3407  CD1  TRP C 158   -27.045 -39.064  39.741  1.00 38.54      D000 C
ATOM  3408  CD2  TRP C 158   -24.903 -39.505  39.262  1.00 36.91      D000 C
ATOM  3409  CE2  TRP C 158   -25.175 -39.961  40.568  1.00 38.54      D000 C
ATOM  3410  CE3  TRP C 158   -23.615 -39.676  38.743  1.00 32.86      D000 C
ATOM  3411  NE1  TRP C 158   -26.488 -39.677  40.837  1.00 42.00      D000 N
ATOM  3412  CZ2  TRP C 158   -24.209 -40.576  41.362  1.00 34.01      D000 C
ATOM  3413  CZ3  TRP C 158   -22.657 -40.286  39.533  1.00 31.10      D000 C
ATOM  3414  CH2  TRP C 158   -22.958 -40.728  40.827  1.00 31.72      D000 C
ATOM  3415  N    VAL C 159   -25.511 -35.775  35.640  1.00 37.12      D000 N
ATOM  3416  CA   VAL C 159   -25.359 -35.091  34.360  1.00 35.19      D000 C
ATOM  3417  C    VAL C 159   -24.434 -35.876  33.422  1.00 42.52      D000 C
ATOM  3418  O    VAL C 159   -23.383 -36.372  33.837  1.00 37.10      D000 O
ATOM  3419  CB   VAL C 159   -24.811 -33.660  34.557  1.00 36.85      D000 C
ATOM  3420  CG1  VAL C 159   -24.794 -32.907  33.240  1.00 35.63      D000 C
ATOM  3421  CG2  VAL C 159   -25.650 -32.909  35.583  1.00 36.56      D000 C
ATOM  3422  N    GLU C 160   -24.827 -35.987  32.157  1.00 36.06      D000 N
ATOM  3423  CA   GLU C 160   -24.070 -36.784  31.197  1.00 43.73      D000 C
ATOM  3424  C    GLU C 160   -23.047 -35.975  30.405  1.00 36.77      D000 C
ATOM  3425  O    GLU C 160   -23.328 -34.874  29.939  1.00 41.07      D000 O
ATOM  3426  CB   GLU C 160   -25.018 -37.481  30.221  1.00 43.94      D000 C
ATOM  3427  CG   GLU C 160   -24.301 -38.291  29.158  1.00 50.04      D000 C
ATOM  3428  CD   GLU C 160   -25.222 -38.743  28.045  1.00 67.78      D000 C
ATOM  3429  OE1  GLU C 160   -26.430 -38.930  28.308  1.00 69.69      D000 O
ATOM  3430  OE2  GLU C 160   -24.735 -38.904  26.905  1.00 67.23      D000 O
ATOM  3431  N    HIS C 161   -21.856 -36.541  30.261  1.00 37.45      D000 N
ATOM  3432  CA   HIS C 161   -20.829 -35.993  29.388  1.00 38.41      D000 C
ATOM  3433  C    HIS C 161   -19.987 -37.135  28.842  1.00 40.31      D000 C
ATOM  3434  O    HIS C 161   -19.301 -37.816  29.604  1.00 36.78      D000 O
ATOM  3435  CB   HIS C 161   -19.951 -34.987  30.134  1.00 35.44      D000 C
ATOM  3436  CG   HIS C 161   -18.785 -34.487  29.334  1.00 39.39      D000 C
ATOM  3437  CD2  HIS C 161   -17.492 -34.888  29.300  1.00 37.49      D000 C
ATOM  3438  ND1  HIS C 161   -18.888 -33.445  28.439  1.00 37.81      D000 N
ATOM  3439  CE1  HIS C 161   -17.707 -33.223  27.887  1.00 36.44      D000 C
ATOM  3440  NE2  HIS C 161   -16.844 -34.085  28.390  1.00 39.12      D000 N
ATOM  3441  N    GLU C 162   -20.066 -37.349  27.529  1.00 45.55      D000 N
ATOM  3442  CA   GLU C 162   -19.271 -38.365  26.834  1.00 40.64      D000 C
ATOM  3443  C    GLU C 162   -19.341 -39.749  27.484  1.00 41.12      D000 C
ATOM  3444  O    GLU C 162   -18.318 -40.305  27.887  1.00 42.56      D000 O
ATOM  3445  CB   GLU C 162   -17.806 -37.919  26.744  1.00 41.10      D000 C
ATOM  3446  CG   GLU C 162   -17.596 -36.576  26.052  1.00 49.56      D000 C
ATOM  3447  CD   GLU C 162   -18.120 -36.554  24.622  1.00 68.97      D000 C
ATOM  3448  OE1  GLU C 162   -18.103 -37.612  23.955  1.00 61.44      D000 O
ATOM  3449  OE2  GLU C 162   -18.551 -35.472  24.163  1.00 82.19      D000 O
ATOM  3450  N    ARG C 163   -20.550 -40.289  27.586  1.00 39.55      D000 N
ATOM  3451  CA   ARG C 163   -20.790 -41.614  28.175  1.00 50.72      D000 C
ATOM  3452  C    ARG C 163   -20.305 -41.757  29.628  1.00 46.47      D000 C
ATOM  3453  O    ARG C 163   -20.132 -42.870  30.127  1.00 44.94      D000 O
ATOM  3454  CB   ARG C 163   -20.156 -42.710  27.306  1.00 49.40      D000 C
ATOM  3455  CG   ARG C 163   -20.768 -42.827  25.907  1.00 51.79      D000 C
ATOM  3456  CD   ARG C 163   -20.685 -44.252  25.348  1.00 44.72      D000 C
ATOM  3457  NE   ARG C 163   -21.723 -45.140  25.879  1.00 51.93      D000 N
ATOM  3458  CZ   ARG C 163   -21.483 -46.234  26.606  1.00 59.01      D000 C
ATOM  3459  NH1  ARG C 163   -22.492 -46.980  27.044  1.00 45.88      D000 N
ATOM  3460  NH2  ARG C 163   -20.236 -46.586  26.897  1.00 49.85      D000 N
ATOM  3461  N    SER C 164   -20.099 -40.633  30.306  1.00 43.03      D000 N
ATOM  3462  CA   SER C 164   -19.865 -40.646  31.746  1.00 37.64      D000 C
ATOM  3463  C    SER C 164   -20.969 -39.876  32.459  1.00 37.23      D000 C
ATOM  3464  O    SER C 164   -21.536 -38.933  31.903  1.00 37.58      D000 O
ATOM  3465  CB   SER C 164   -18.498 -40.052  32.092  1.00 37.20      D000 C
ATOM  3466  OG   SER C 164   -17.509 -41.061  32.172  1.00 40.60      D000 O
ATOM  3467  N    CYS C 165   -21.272 -40.283  33.687  1.00 36.28      D000 N
ATOM  3468  CA   CYS C 165   -22.296 -39.624  34.493  1.00 35.32      D000 C
ATOM  3469  C    CYS C 165   -21.666 -38.921  35.688  1.00 39.53      D000 C
ATOM  3470  O    CYS C 165   -20.843 -39.504  36.398  1.00 33.29      D000 O
ATOM  3471  CB   CYS C 165   -23.344 -40.632  34.974  1.00 42.45      D000 C
ATOM  3472  SG   CYS C 165   -24.315 -41.382  33.657  1.00 55.85      D000 S
ATOM  3473  N    TYR C 166   -22.065 -37.674  35.915  1.00 29.38      D000 N
ATOM  3474  CA   TYR C 166   -21.475 -36.870  36.976  1.00 33.48      D000 C
ATOM  3475  C    TYR C 166   -22.532 -36.391  37.963  1.00 33.01      D000 C
```

```
ATOM  3476  O    TYR C 166   -23.665 -36.110  37.589  1.00 33.24      D000 O
ATOM  3477  CB   TYR C 166   -20.730 -35.667  36.388  1.00 32.05      D000 C
ATOM  3478  CG   TYR C 166   -19.658 -36.030  35.383  1.00 31.06      D000 C
ATOM  3479  CD1  TYR C 166   -19.990 -36.385  34.080  1.00 30.70      D000 C
ATOM  3480  CD2  TYR C 166   -18.313 -36.003  35.732  1.00 30.70      D000 C
ATOM  3481  CE1  TYR C 166   -19.017 -36.713  33.153  1.00 33.69      D000 C
ATOM  3482  CE2  TYR C 166   -17.329 -36.329  34.807  1.00 32.22      D000 C
ATOM  3483  CZ   TYR C 166   -17.690 -36.682  33.519  1.00 32.45      D000 C
ATOM  3484  OH   TYR C 166   -16.723 -37.007  32.593  1.00 35.47      D000 O
ATOM  3485  N    TRP C 167   -22.156 -36.309  39.231  1.00 33.71      D000 N
ATOM  3486  CA   TRP C 167   -23.032 -35.743  40.246  1.00 35.55      D000 C
ATOM  3487  C    TRP C 167   -22.262 -34.685  41.017  1.00 32.66      D000 C
ATOM  3488  O    TRP C 167   -21.140 -34.931  41.470  1.00 31.46      D000 O
ATOM  3489  CB   TRP C 167   -23.559 -36.827  41.186  1.00 35.80      D000 C
ATOM  3490  CG   TRP C 167   -24.458 -36.290  42.265  1.00 43.58      D000 C
ATOM  3491  CD1  TRP C 167   -25.805 -36.079  42.183  1.00 38.70      D000 C
ATOM  3492  CD2  TRP C 167   -24.070 -35.896  43.587  1.00 35.59      D000 C
ATOM  3493  CE2  TRP C 167   -25.235 -35.456  44.251  1.00 41.09      D000 C
ATOM  3494  CE3  TRP C 167   -22.852 -35.869  44.271  1.00 35.37      D000 C
ATOM  3495  NE1  TRP C 167   -26.280 -35.577  43.373  1.00 39.42      D000 N
ATOM  3496  CZ2  TRP C 167   -25.215 -34.997  45.567  1.00 38.44      D000 C
ATOM  3497  CZ3  TRP C 167   -22.835 -35.415  45.579  1.00 37.33      D000 C
ATOM  3498  CH2  TRP C 167   -24.008 -34.985  46.213  1.00 36.75      D000 C
ATOM  3499  N    PHE C 168   -22.858 -33.505  41.155  1.00 33.58      D000 N
ATOM  3500  CA   PHE C 168   -22.172 -32.374  41.778  1.00 33.25      D000 C
ATOM  3501  C    PHE C 168   -22.773 -32.041  43.134  1.00 31.97      D000 C
ATOM  3502  O    PHE C 168   -23.953 -31.721  43.227  1.00 34.21      D000 O
ATOM  3503  CB   PHE C 168   -22.229 -31.148  40.861  1.00 28.44      D000 C
ATOM  3504  CG   PHE C 168   -21.573 -31.363  39.526  1.00 30.16      D000 C
ATOM  3505  CD1  PHE C 168   -22.287 -31.896  38.463  1.00 31.96      D000 C
ATOM  3506  CD2  PHE C 168   -20.239 -31.038  39.337  1.00 29.17      D000 C
ATOM  3507  CE1  PHE C 168   -21.682 -32.099  37.229  1.00 29.38      D000 C
ATOM  3508  CE2  PHE C 168   -19.625 -31.242  38.114  1.00 28.14      D000 C
ATOM  3509  CZ   PHE C 168   -20.348 -31.776  37.057  1.00 28.28      D000 C
ATOM  3510  N    SER C 169   -21.961 -32.110  44.186  1.00 30.11      D000 N
ATOM  3511  CA   SER C 169   -22.462 -31.851  45.528  1.00 31.18      D000 C
ATOM  3512  C    SER C 169   -22.693 -30.360  45.740  1.00 37.87      D000 C
ATOM  3513  O    SER C 169   -22.121 -29.522  45.038  1.00 34.20      D000 O
ATOM  3514  CB   SER C 169   -21.494 -32.389  46.583  1.00 30.35      D000 C
ATOM  3515  OG   SER C 169   -20.471 -31.453  46.859  1.00 30.05      D000 O
ATOM  3516  N    ARG C 170   -23.536 -30.027  46.708  1.00 37.76      D000 N
ATOM  3517  CA   ARG C 170   -23.767 -28.628  47.046  1.00 42.71      D000 C
ATOM  3518  C    ARG C 170   -23.330 -28.383  48.488  1.00 43.83      D000 C
ATOM  3519  O    ARG C 170   -23.437 -27.274  49.010  1.00 47.78      D000 O
ATOM  3520  CB   ARG C 170   -25.237 -28.254  46.829  1.00 44.70      D000 C
ATOM  3521  CG   ARG C 170   -25.717 -28.513  45.395  1.00 57.03      D000 C
ATOM  3522  CD   ARG C 170   -27.063 -27.862  45.082  1.00 63.29      D000 C
ATOM  3523  NE   ARG C 170   -28.144 -28.365  45.925  1.00 84.25      D000 N
ATOM  3524  CZ   ARG C 170   -28.822 -29.485  45.688  1.00 84.39      D000 C
ATOM  3525  NH1  ARG C 170   -29.792 -29.862  46.511  1.00 72.14      D000 N
ATOM  3526  NH2  ARG C 170   -28.528 -30.230  44.631  1.00 91.59      D000 N
ATOM  3527  N    SER C 171   -22.817 -29.439  49.112  1.00 39.23      D000 N
ATOM  3528  CA   SER C 171   -22.298 -29.372  50.470  1.00 41.01      D000 C
ATOM  3529  C    SER C 171   -20.827 -29.773  50.489  1.00 37.33      D000 C
ATOM  3530  O    SER C 171   -20.305 -30.281  49.499  1.00 31.87      D000 O
ATOM  3531  CB   SER C 171   -23.106 -30.277  51.401  1.00 36.62      D000 C
ATOM  3532  OG   SER C 171   -23.000 -31.633  51.004  1.00 43.48      D000 O
ATOM  3533  N    GLY C 172   -20.166 -29.549  51.620  1.00 31.79      D000 N
ATOM  3534  CA   GLY C 172   -18.749 -29.840  51.743  1.00 31.58      D000 C
ATOM  3535  C    GLY C 172   -18.428 -31.069  52.573  1.00 37.61      D000 C
ATOM  3536  O    GLY C 172   -19.161 -31.427  53.495  1.00 34.62      D000 O
ATOM  3537  N    LYS C 173   -17.317 -31.714  52.231  1.00 32.21      D000 N
ATOM  3538  CA   LYS C 173   -16.818 -32.878  52.954  1.00 30.84      D000 C
ATOM  3539  C    LYS C 173   -15.300 -32.903  52.885  1.00 36.15      D000 C
ATOM  3540  O    LYS C 173   -14.715 -32.463  51.894  1.00 35.63      D000 O
ATOM  3541  CB   LYS C 173   -17.365 -34.184  52.367  1.00 35.20      D000 C
ATOM  3542  CG   LYS C 173   -18.746 -34.590  52.824  1.00 33.26      D000 C
ATOM  3543  CD   LYS C 173   -19.092 -35.966  52.277  1.00 36.71      D000 C
ATOM  3544  CE   LYS C 173   -20.518 -36.364  52.617  1.00 39.30      D000 C
ATOM  3545  NZ   LYS C 173   -20.706 -36.532  54.082  1.00 42.46      D000 N
ATOM  3546  N    ALA C 174   -14.665 -33.423  53.930  1.00 34.50      D000 N
```

```
ATOM   3547  CA   ALA C 174   -13.244 -33.733  53.871  1.00 32.78      D000 C
ATOM   3548  C    ALA C 174   -13.011 -34.731  52.737  1.00 30.79      D000 C
ATOM   3549  O    ALA C 174   -13.918 -35.486  52.375  1.00 27.51      D000 O
ATOM   3550  CB   ALA C 174   -12.756 -34.296  55.201  1.00 32.37      D000 C
ATOM   3551  N    TRP C 175   -11.802 -34.736  52.182  1.00 28.74      D000 N
ATOM   3552  CA   TRP C 175   -11.510 -35.556  51.011  1.00 34.63      D000 C
ATOM   3553  C    TRP C 175   -11.853 -37.026  51.230  1.00 31.83      D000 C
ATOM   3554  O    TRP C 175   -12.520 -37.640  50.398  1.00 31.59      D000 O
ATOM   3555  CB   TRP C 175   -10.041 -35.430  50.606  1.00 31.97      D000 C
ATOM   3556  CG   TRP C 175    -9.771 -36.017  49.251  1.00 27.08      D000 C
ATOM   3557  CD1  TRP C 175    -9.812 -35.364  48.051  1.00 33.57      D000 C
ATOM   3558  CD2  TRP C 175    -9.442 -37.378  48.955  1.00 30.56      D000 C
ATOM   3559  CE2  TRP C 175    -9.285 -37.476  47.558  1.00 29.73      D000 C
ATOM   3560  CE3  TRP C 175    -9.257 -38.524  49.737  1.00 31.43      D000 C
ATOM   3561  NE1  TRP C 175    -9.518 -36.234  47.029  1.00 30.16      D000 N
ATOM   3562  CZ2  TRP C 175    -8.958 -38.674  46.926  1.00 31.89      D000 C
ATOM   3563  CZ3  TRP C 175    -8.934 -39.711  49.107  1.00 33.11      D000 C
ATOM   3564  CH2  TRP C 175    -8.787 -39.777  47.715  1.00 31.57      D000 C
ATOM   3565  N    ALA C 176   -11.402 -37.585  52.349  1.00 36.55      D000 N
ATOM   3566  CA   ALA C 176   -11.654 -38.992  52.642  1.00 34.85      D000 C
ATOM   3567  C    ALA C 176   -13.153 -39.287  52.720  1.00 31.81      D000 C
ATOM   3568  O    ALA C 176   -13.611 -40.335  52.264  1.00 32.34      D000 O
ATOM   3569  CB   ALA C 176   -10.961 -39.397  53.940  1.00 39.21      D000 C
ATOM   3570  N    ASP C 177   -13.921 -38.362  53.286  1.00 29.24      D000 N
ATOM   3571  CA   ASP C 177   -15.363 -38.562  53.387  1.00 30.70      D000 C
ATOM   3572  C    ASP C 177   -16.048 -38.465  52.021  1.00 34.41      D000 C
ATOM   3573  O    ASP C 177   -17.005 -39.186  51.749  1.00 30.91      D000 O
ATOM   3574  CB   ASP C 177   -15.976 -37.560  54.363  1.00 36.68      D000 C
ATOM   3575  CG   ASP C 177   -15.641 -37.883  55.810  1.00 47.91      D000 C
ATOM   3576  OD1  ASP C 177   -15.459 -39.080  56.124  1.00 48.52      D000 O
ATOM   3577  OD2  ASP C 177   -15.561 -36.943  56.631  1.00 44.32      D000 O
ATOM   3578  N    ALA C 178   -15.558 -37.575  51.164  1.00 33.94      D000 N
ATOM   3579  CA   ALA C 178   -16.104 -37.450  49.814  1.00 37.48      D000 C
ATOM   3580  C    ALA C 178   -15.776 -38.696  48.994  1.00 30.71      D000 C
ATOM   3581  O    ALA C 178   -16.620 -39.219  48.265  1.00 33.32      D000 O
ATOM   3582  CB   ALA C 178   -15.568 -36.197  49.130  1.00 29.20      D000 C
ATOM   3583  N    ASP C 179   -14.540 -39.161  49.134  1.00 30.70      D000 N
ATOM   3584  CA   ASP C 179   -14.078 -40.389  48.501  1.00 27.81      D000 C
ATOM   3585  C    ASP C 179   -14.986 -41.564  48.868  1.00 36.66      D000 C
ATOM   3586  O    ASP C 179   -15.411 -42.333  48.005  1.00 36.40      D000 O
ATOM   3587  CB   ASP C 179   -12.630 -40.671  48.918  1.00 31.15      D000 C
ATOM   3588  CG   ASP C 179   -12.068 -41.935  48.289  1.00 39.74      D000 C
ATOM   3589  OD1  ASP C 179   -12.435 -42.255  47.139  1.00 49.64      D000 O
ATOM   3590  OD2  ASP C 179   -11.247 -42.607  48.947  1.00 47.11      D000 O
ATOM   3591  N    ASN C 180   -15.299 -41.680  50.153  1.00 32.38      D000 N
ATOM   3592  CA   ASN C 180   -16.155 -42.751  50.642  1.00 33.10      D000 C
ATOM   3593  C    ASN C 180   -17.598 -42.602  50.155  1.00 32.93      D000 C
ATOM   3594  O    ASN C 180   -18.224 -43.582  49.755  1.00 36.93      D000 O
ATOM   3595  CB   ASN C 180   -16.108 -42.799  52.171  1.00 39.79      D000 C
ATOM   3596  CG   ASN C 180   -16.936 -43.931  52.745  1.00 50.05      D000 C
ATOM   3597  ND2  ASN C 180   -16.557 -45.165  52.427  1.00 44.28      D000 N
ATOM   3598  OD1  ASN C 180   -17.911 -43.699  53.461  1.00 53.44      D000 O
ATOM   3599  N    TYR C 181   -18.120 -41.378  50.182  1.00 33.15      D000 N
ATOM   3600  CA   TYR C 181   -19.460 -41.105  49.659  1.00 36.25      D000 C
ATOM   3601  C    TYR C 181   -19.609 -41.606  48.218  1.00 38.76      D000 C
ATOM   3602  O    TYR C 181   -20.608 -42.238  47.875  1.00 36.89      D000 O
ATOM   3603  CB   TYR C 181   -19.775 -39.605  49.728  1.00 29.33      D000 C
ATOM   3604  CG   TYR C 181   -21.139 -39.226  49.184  1.00 38.78      D000 C
ATOM   3605  CD1  TYR C 181   -21.333 -39.016  47.820  1.00 39.24      D000 C
ATOM   3606  CD2  TYR C 181   -22.232 -39.065  50.032  1.00 37.86      D000 C
ATOM   3607  CE1  TYR C 181   -22.574 -38.670  47.316  1.00 41.79      D000 C
ATOM   3608  CE2  TYR C 181   -23.484 -38.716  49.534  1.00 30.18      D000 C
ATOM   3609  CZ   TYR C 181   -23.643 -38.520  48.173  1.00 43.34      D000 C
ATOM   3610  OH   TYR C 181   -24.868 -38.175  47.654  1.00 43.72      D000 O
ATOM   3611  N    CYS C 182   -18.617 -41.321  47.379  1.00 32.27      D000 N
ATOM   3612  CA   CYS C 182   -18.688 -41.720  45.977  1.00 33.21      D000 C
ATOM   3613  C    CYS C 182   -18.666 -43.240  45.819  1.00 36.35      D000 C
ATOM   3614  O    CYS C 182   -19.433 -43.793  45.032  1.00 28.20      D000 O
ATOM   3615  CB   CYS C 182   -17.550 -41.079  45.177  1.00 33.72      D000 C
ATOM   3616  SG   CYS C 182   -17.775 -39.294  44.910  1.00 35.82      D000 S
ATOM   3617  N    ARG C 183   -17.798 -43.911  46.573  1.00 38.71      D000 N
```

```
ATOM   3618  CA  ARG C 183    -17.731 -45.373  46.546  1.00 38.40      D000 C
ATOM   3619  C   ARG C 183    -19.071 -45.997  46.942  1.00 41.03      D000 C
ATOM   3620  O   ARG C 183    -19.466 -47.030  46.409  1.00 42.99      D000 O
ATOM   3621  CB  ARG C 183    -16.621 -45.880  47.473  1.00 39.02      D000 C
ATOM   3622  CG  ARG C 183    -15.217 -45.538  47.002  1.00 46.96      D000 C
ATOM   3623  CD  ARG C 183    -14.169 -45.992  48.007  1.00 60.57      D000 C
ATOM   3624  NE  ARG C 183    -12.816 -45.655  47.570  1.00 80.77      D000 N
ATOM   3625  CZ  ARG C 183    -11.722 -45.845  48.301  1.00 85.00      D000 C
ATOM   3626  NH1 ARG C 183    -11.814 -46.372  49.516  1.00 72.80      D000 N
ATOM   3627  NH2 ARG C 183    -10.532 -45.505  47.818  1.00 88.26      D000 N
ATOM   3628  N   LEU C 184    -19.769 -45.348  47.869  1.00 38.52      D000 N
ATOM   3629  CA  LEU C 184    -21.063 -45.822  48.348  1.00 45.53      D000 C
ATOM   3630  C   LEU C 184    -22.165 -45.627  47.305  1.00 44.46      D000 C
ATOM   3631  O   LEU C 184    -23.192 -46.300  47.344  1.00 49.42      D000 O
ATOM   3632  CB  LEU C 184    -21.436 -45.110  49.656  1.00 38.94      D000 C
ATOM   3633  CG  LEU C 184    -21.111 -45.837  50.967  1.00 47.53      D000 C
ATOM   3634  CD1 LEU C 184    -19.766 -46.545  50.898  1.00 51.14      D000 C
ATOM   3635  CD2 LEU C 184    -21.138 -44.869  52.138  1.00 42.20      D000 C
ATOM   3636  N   GLU C 185    -21.953 -44.701  46.376  1.00 42.20      D000 N
ATOM   3637  CA  GLU C 185    -22.903 -44.483  45.288  1.00 39.20      D000 C
ATOM   3638  C   GLU C 185    -22.504 -45.293  44.056  1.00 40.02      D000 C
ATOM   3639  O   GLU C 185    -22.992 -45.040  42.954  1.00 41.00      D000 O
ATOM   3640  CB  GLU C 185    -22.991 -42.997  44.936  1.00 43.28      D000 C
ATOM   3641  CG  GLU C 185    -23.645 -42.139  46.005  1.00 50.04      D000 C
ATOM   3642  CD  GLU C 185    -25.132 -42.410  46.144  1.00 63.83      D000 C
ATOM   3643  OE1 GLU C 185    -25.853 -42.327  45.125  1.00 64.40      D000 O
ATOM   3644  OE2 GLU C 185    -25.578 -42.711  47.274  1.00 66.65      D000 O
ATOM   3645  N   ASP C 186    -21.618 -46.266  44.266  1.00 39.71      D000 N
ATOM   3646  CA  ASP C 186    -21.073 -47.104  43.197  1.00 46.14      D000 C
ATOM   3647  C   ASP C 186    -20.374 -46.235  42.149  1.00 46.36      D000 C
ATOM   3648  O   ASP C 186    -20.472 -46.469  40.940  1.00 37.33      D000 O
ATOM   3649  CB  ASP C 186    -22.172 -47.957  42.555  1.00 48.03      D000 C
ATOM   3650  CG  ASP C 186    -21.616 -49.072  41.689  1.00 57.58      D000 C
ATOM   3651  OD1 ASP C 186    -20.621 -49.705  42.104  1.00 69.48      D000 O
ATOM   3652  OD2 ASP C 186    -22.167 -49.306  40.592  1.00 63.55      D000 O
ATOM   3653  N   ALA C 187    -19.667 -45.222  42.634  1.00 37.49      D000 N
ATOM   3654  CA  ALA C 187    -18.974 -44.291  41.763  1.00 36.04      D000 C
ATOM   3655  C   ALA C 187    -17.592 -43.992  42.322  1.00 37.13      D000 C
ATOM   3656  O   ALA C 187    -17.086 -44.715  43.183  1.00 35.71      D000 O
ATOM   3657  CB  ALA C 187    -19.784 -43.005  41.603  1.00 29.96      D000 C
ATOM   3658  N   HIS C 188    -16.981 -42.924  41.826  1.00 31.72      D000 N
ATOM   3659  CA  HIS C 188    -15.693 -42.486  42.332  1.00 29.40      D000 C
ATOM   3660  C   HIS C 188    -15.569 -40.982  42.157  1.00 28.01      D000 C
ATOM   3661  O   HIS C 188    -16.263 -40.391  41.329  1.00 26.39      D000 O
ATOM   3662  CB  HIS C 188    -14.556 -43.211  41.618  1.00 23.87      D000 C
ATOM   3663  CG  HIS C 188    -14.541 -43.002  40.137  1.00 35.80      D000 C
ATOM   3664  CD2 HIS C 188    -14.996 -43.776  39.123  1.00 32.29      D000 C
ATOM   3665  ND1 HIS C 188    -14.009 -41.874  39.548  1.00 32.82      D000 N
ATOM   3666  CE1 HIS C 188    -14.134 -41.964  38.236  1.00 33.00      D000 C
ATOM   3667  NE2 HIS C 188    -14.729 -43.109  37.951  1.00 33.93      D000 N
ATOM   3668  N   LEU C 189    -14.708 -40.364  42.958  1.00 26.16      D000 N
ATOM   3669  CA  LEU C 189    -14.400 -38.950  42.784  1.00 28.90      D000 C
ATOM   3670  C   LEU C 189    -13.909 -38.716  41.362  1.00 29.00      D000 C
ATOM   3671  O   LEU C 189    -13.176 -39.541  40.805  1.00 27.16      D000 O
ATOM   3672  CB  LEU C 189    -13.356 -38.488  43.796  1.00 26.67      D000 C
ATOM   3673  CG  LEU C 189    -13.882 -38.242  45.208  1.00 33.12      D000 C
ATOM   3674  CD1 LEU C 189    -12.735 -37.973  46.169  1.00 33.71      D000 C
ATOM   3675  CD2 LEU C 189    -14.857 -37.076  45.186  1.00 27.90      D000 C
ATOM   3676  N   VAL C 190    -14.320 -37.592  40.786  1.00 27.74      D000 N
ATOM   3677  CA  VAL C 190    -14.117 -37.333  39.365  1.00 27.15      D000 C
ATOM   3678  C   VAL C 190    -12.644 -37.428  38.968  1.00 29.08      D000 C
ATOM   3679  O   VAL C 190    -11.743 -36.982  39.690  1.00 26.29      D000 O
ATOM   3680  CB  VAL C 190    -14.699 -35.946  38.957  1.00 29.42      D000 C
ATOM   3681  CG1 VAL C 190    -13.956 -34.796  39.654  1.00 25.30      D000 C
ATOM   3682  CG2 VAL C 190    -14.705 -35.779  37.434  1.00 23.06      D000 C
ATOM   3683  N   VAL C 191    -12.420 -38.079  37.834  1.00 26.83      D000 N
ATOM   3684  CA  VAL C 191    -11.106 -38.192  37.221  1.00 30.57      D000 C
ATOM   3685  C   VAL C 191    -11.136 -37.388  35.927  1.00 28.23      D000 C
ATOM   3686  O   VAL C 191    -12.017 -37.590  35.096  1.00 26.03      D000 O
ATOM   3687  CB  VAL C 191    -10.739 -39.664  36.942  1.00 28.44      D000 C
ATOM   3688  CG1 VAL C 191     -9.433 -39.763  36.170  1.00 25.60      D000 C
```

```
ATOM   3689  CG2 VAL C 191    -10.663 -40.445  38.256  1.00 26.79      D000 C
ATOM   3690  N   VAL C 192    -10.200 -36.457  35.770  1.00 28.93      D000 N
ATOM   3691  CA  VAL C 192    -10.224 -35.550  34.629  1.00 27.42      D000 C
ATOM   3692  C   VAL C 192     -9.181 -35.963  33.602  1.00 31.88      D000 C
ATOM   3693  O   VAL C 192     -7.984 -35.957  33.888  1.00 27.55      D000 O
ATOM   3694  CB  VAL C 192     -9.979 -34.094  35.061  1.00 23.02      D000 C
ATOM   3695  CG1 VAL C 192    -10.008 -33.170  33.851  1.00 23.08      D000 C
ATOM   3696  CG2 VAL C 192    -11.017 -33.670  36.085  1.00 21.27      D000 C
ATOM   3697  N   THR C 193     -9.637 -36.314  32.403  1.00 25.64      D000 N
ATOM   3698  CA  THR C 193     -8.751 -36.918  31.415  1.00 32.64      D000 C
ATOM   3699  C   THR C 193     -8.608 -36.120  30.125  1.00 31.08      D000 C
ATOM   3700  O   THR C 193     -7.920 -36.558  29.206  1.00 30.10      D000 O
ATOM   3701  CB  THR C 193     -9.223 -38.333  31.040  1.00 33.34      D000 C
ATOM   3702  CG2 THR C 193     -9.327 -39.208  32.285  1.00 32.24      D000 C
ATOM   3703  OG1 THR C 193    -10.501 -38.252  30.398  1.00 34.27      D000 O
ATOM   3704  N   SER C 194     -9.247 -34.958  30.049  1.00 29.11      D000 N
ATOM   3705  CA  SER C 194     -9.115 -34.111  28.866  1.00 29.77      D000 C
ATOM   3706  C   SER C 194     -9.525 -32.671  29.159  1.00 29.25      D000 C
ATOM   3707  O   SER C 194    -10.227 -32.400  30.137  1.00 23.94      D000 O
ATOM   3708  CB  SER C 194     -9.956 -34.663  27.708  1.00 25.83      D000 C
ATOM   3709  OG  SER C 194    -11.335 -34.423  27.935  1.00 28.83      D000 O
ATOM   3710  N   TRP C 195     -9.080 -31.753  28.305  1.00 28.44      D000 N
ATOM   3711  CA  TRP C 195     -9.456 -30.343  28.409  1.00 29.60      D000 C
ATOM   3712  C   TRP C 195    -10.972 -30.177  28.304  1.00 25.02      D000 C
ATOM   3713  O   TRP C 195    -11.553 -29.331  28.974  1.00 29.97      D000 O
ATOM   3714  CB  TRP C 195     -8.737 -29.531  27.323  1.00 26.47      D000 C
ATOM   3715  CG  TRP C 195     -9.010 -28.039  27.283  1.00 32.42      D000 C
ATOM   3716  CD1 TRP C 195     -9.356 -27.306  26.181  1.00 34.68      D000 C
ATOM   3717  CD2 TRP C 195     -8.932 -27.101  28.375  1.00 30.83      D000 C
ATOM   3718  CE2 TRP C 195     -9.245 -25.827  27.853  1.00 34.03      D000 C
ATOM   3719  CE3 TRP C 195     -8.631 -27.215  29.736  1.00 30.71      D000 C
ATOM   3720  NE1 TRP C 195     -9.498 -25.981  26.515  1.00 32.39      D000 N
ATOM   3721  CZ2 TRP C 195     -9.271 -24.678  28.645  1.00 31.06      D000 C
ATOM   3722  CZ3 TRP C 195     -8.655 -26.071  30.523  1.00 32.95      D000 C
ATOM   3723  CH2 TRP C 195     -8.969 -24.820  29.974  1.00 30.07      D000 C
ATOM   3724  N   GLU C 196    -11.607 -30.999  27.471  1.00 26.97      D000 N
ATOM   3725  CA  GLU C 196    -13.060 -30.960  27.306  1.00 30.42      D000 C
ATOM   3726  C   GLU C 196    -13.794 -31.365  28.580  1.00 34.02      D000 C
ATOM   3727  O   GLU C 196    -14.802 -30.757  28.943  1.00 28.31      D000 O
ATOM   3728  CB  GLU C 196    -13.505 -31.871  26.158  1.00 30.46      D000 C
ATOM   3729  CG  GLU C 196    -13.221 -31.329  24.769  1.00 41.10      D000 C
ATOM   3730  CD  GLU C 196    -11.772 -31.498  24.353  1.00 46.52      D000 C
ATOM   3731  OE1 GLU C 196    -11.002 -32.155  25.092  1.00 42.32      D000 O
ATOM   3732  OE2 GLU C 196    -11.406 -30.973  23.281  1.00 46.49      D000 O
ATOM   3733  N   GLU C 197    -13.297 -32.404  29.245  1.00 29.50      D000 N
ATOM   3734  CA  GLU C 197    -13.899 -32.854  30.489  1.00 26.78      D000 C
ATOM   3735  C   GLU C 197    -13.708 -31.790  31.574  1.00 26.09      D000 C
ATOM   3736  O   GLU C 197    -14.631 -31.493  32.329  1.00 27.28      D000 O
ATOM   3737  CB  GLU C 197    -13.305 -34.198  30.930  1.00 26.61      D000 C
ATOM   3738  CG  GLU C 197    -14.055 -34.841  32.100  1.00 27.35      D000 C
ATOM   3739  CD  GLU C 197    -13.518 -36.215  32.488  1.00 37.87      D000 C
ATOM   3740  OE1 GLU C 197    -12.434 -36.602  32.003  1.00 33.97      D000 O
ATOM   3741  OE2 GLU C 197    -14.187 -36.910  33.285  1.00 39.22      D000 O
ATOM   3742  N   GLN C 198    -12.514 -31.205  31.629  1.00 25.72      D000 N
ATOM   3743  CA  GLN C 198    -12.214 -30.140  32.588  1.00 25.80      D000 C
ATOM   3744  C   GLN C 198    -13.189 -28.975  32.463  1.00 29.00      D000 C
ATOM   3745  O   GLN C 198    -13.685 -28.458  33.464  1.00 24.52      D000 O
ATOM   3746  CB  GLN C 198    -10.783 -29.632  32.394  1.00 23.78      D000 C
ATOM   3747  CG  GLN C 198    -10.506 -28.288  33.045  1.00 25.75      D000 C
ATOM   3748  CD  GLN C 198    -10.246 -28.397  34.537  1.00 33.71      D000 C
ATOM   3749  NE2 GLN C 198    -10.599 -27.350  35.279  1.00 25.10      D000 N
ATOM   3750  OE1 GLN C 198     -9.734 -29.410  35.017  1.00 28.06      D000 O
ATOM   3751  N   LYS C 199    -13.466 -28.574  31.226  1.00 30.60      D000 N
ATOM   3752  CA  LYS C 199    -14.326 -27.424  30.974  1.00 34.08      D000 C
ATOM   3753  C   LYS C 199    -15.770 -27.743  31.319  1.00 28.60      D000 C
ATOM   3754  O   LYS C 199    -16.489 -26.895  31.848  1.00 25.57      D000 O
ATOM   3755  CB  LYS C 199    -14.212 -26.978  29.517  1.00 28.25      D000 C
ATOM   3756  CG  LYS C 199    -12.855 -26.386  29.183  1.00 34.79      D000 C
ATOM   3757  CD  LYS C 199    -12.532 -26.545  27.711  1.00 43.54      D000 C
ATOM   3758  CE  LYS C 199    -13.150 -25.447  26.892  1.00 38.89      D000 C
ATOM   3759  NZ  LYS C 199    -12.740 -25.539  25.453  1.00 35.02      D000 N
```

```
ATOM   3760  N   PHE C 200   -16.188 -28.968  31.013  1.00 29.54   D000 N
ATOM   3761  CA  PHE C 200   -17.515 -29.436  31.393  1.00 30.79   D000 C
ATOM   3762  C   PHE C 200   -17.682 -29.381  32.909  1.00 25.62   D000 C
ATOM   3763  O   PHE C 200   -18.651 -28.807  33.414  1.00 25.66   D000 O
ATOM   3764  CB  PHE C 200   -17.762 -30.861  30.891  1.00 27.79   D000 C
ATOM   3765  CG  PHE C 200   -18.939 -31.530  31.541  1.00 33.05   D000 C
ATOM   3766  CD1 PHE C 200   -20.230 -31.216  31.154  1.00 29.57   D000 C
ATOM   3767  CD2 PHE C 200   -18.754 -32.467  32.547  1.00 33.93   D000 C
ATOM   3768  CE1 PHE C 200   -21.315 -31.826  31.753  1.00 31.19   D000 C
ATOM   3769  CE2 PHE C 200   -19.837 -33.077  33.154  1.00 30.44   D000 C
ATOM   3770  CZ  PHE C 200   -21.118 -32.756  32.755  1.00 33.80   D000 C
ATOM   3771  N   VAL C 201   -16.730 -29.971  33.627  1.00 27.40   D000 N
ATOM   3772  CA  VAL C 201   -16.756 -29.964  35.088  1.00 26.65   D000 C
ATOM   3773  C   VAL C 201   -16.750 -28.533  35.609  1.00 33.34   D000 C
ATOM   3774  O   VAL C 201   -17.593 -28.149  36.428  1.00 25.82   D000 O
ATOM   3775  CB  VAL C 201   -15.557 -30.727  35.679  1.00 30.52   D000 C
ATOM   3776  CG1 VAL C 201   -15.448 -30.466  37.173  1.00 27.81   D000 C
ATOM   3777  CG2 VAL C 201   -15.677 -32.219  35.392  1.00 24.80   D000 C
ATOM   3778  N   GLN C 202   -15.804 -27.748  35.098  1.00 28.75   D000 N
ATOM   3779  CA  GLN C 202   -15.629 -26.353  35.482  1.00 28.03   D000 C
ATOM   3780  C   GLN C 202   -16.905 -25.544  35.295  1.00 26.50   D000 C
ATOM   3781  O   GLN C 202   -17.221 -24.662  36.097  1.00 30.81   D000 O
ATOM   3782  CB  GLN C 202   -14.495 -25.736  34.663  1.00 28.31   D000 C
ATOM   3783  CG  GLN C 202   -13.779 -24.603  35.333  1.00 34.63   D000 C
ATOM   3784  CD  GLN C 202   -12.592 -24.127  34.524  1.00 36.40   D000 C
ATOM   3785  NE2 GLN C 202   -12.382 -22.815  34.494  1.00 34.64   D000 N
ATOM   3786  OE1 GLN C 202   -11.874 -24.931  33.924  1.00 33.68   D000 O
ATOM   3787  N   HIS C 203   -17.640 -25.848  34.232  1.00 28.19   D000 N
ATOM   3788  CA  HIS C 203   -18.895 -25.158  33.971  1.00 28.76   D000 C
ATOM   3789  C   HIS C 203   -19.921 -25.389  35.081  1.00 37.82   D000 C
ATOM   3790  O   HIS C 203   -20.664 -24.477  35.450  1.00 30.91   D000 O
ATOM   3791  CB  HIS C 203   -19.490 -25.597  32.633  1.00 27.45   D000 C
ATOM   3792  CG  HIS C 203   -20.836 -25.002  32.361  1.00 35.85   D000 C
ATOM   3793  CD2 HIS C 203   -21.189 -23.825  31.791  1.00 40.47   D000 C
ATOM   3794  ND1 HIS C 203   -22.010 -25.619  32.728  1.00 42.36   D000 N
ATOM   3795  CE1 HIS C 203   -23.035 -24.857  32.380  1.00 38.39   D000 C
ATOM   3796  NE2 HIS C 203   -22.561 -23.764  31.810  1.00 42.37   D000 N
ATOM   3797  N   HIS C 204   -19.970 -26.608  35.612  1.00 31.47   D000 N
ATOM   3798  CA  HIS C 204   -20.992 -26.941  36.602  1.00 30.96   D000 C
ATOM   3799  C   HIS C 204   -20.619 -26.543  38.023  1.00 29.48   D000 C
ATOM   3800  O   HIS C 204   -21.489 -26.143  38.796  1.00 28.82   D000 O
ATOM   3801  CB  HIS C 204   -21.317 -28.434  36.545  1.00 30.72   D000 C
ATOM   3802  CG  HIS C 204   -22.172 -28.808  35.376  1.00 31.65   D000 C
ATOM   3803  CD2 HIS C 204   -21.889 -29.495  34.244  1.00 34.41   D000 C
ATOM   3804  ND1 HIS C 204   -23.494 -28.433  35.274  1.00 39.82   D000 N
ATOM   3805  CE1 HIS C 204   -23.993 -28.884  34.138  1.00 35.14   D000 C
ATOM   3806  NE2 HIS C 204   -23.038 -29.531  33.493  1.00 39.82   D000 N
ATOM   3807  N   ILE C 205   -19.340 -26.628  38.375  1.00 26.45   D000 N
ATOM   3808  CA  ILE C 205   -18.942 -26.275  39.735  1.00 32.57   D000 C
ATOM   3809  C   ILE C 205   -18.811 -24.758  39.911  1.00 29.31   D000 C
ATOM   3810  O   ILE C 205   -18.976 -24.244  41.015  1.00 30.32   D000 O
ATOM   3811  CB  ILE C 205   -17.613 -26.955  40.152  1.00 31.86   D000 C
ATOM   3812  CG1 ILE C 205   -16.437 -26.421  39.339  1.00 31.34   D000 C
ATOM   3813  CG2 ILE C 205   -17.710 -28.477  40.014  1.00 29.23   D000 C
ATOM   3814  CD1 ILE C 205   -15.107 -26.968  39.802  1.00 32.36   D000 C
ATOM   3815  N   GLY C 206   -18.540 -24.043  38.825  1.00 24.89   D000 N
ATOM   3816  CA  GLY C 206   -18.307 -22.613  38.912  1.00 26.12   D000 C
ATOM   3817  C   GLY C 206   -17.033 -22.318  39.688  1.00 33.16   D000 C
ATOM   3818  O   GLY C 206   -16.182 -23.197  39.845  1.00 27.62   D000 O
ATOM   3819  N   PRO C 207   -16.895 -21.081  40.189  1.00 27.47   D000 N
ATOM   3820  CA  PRO C 207   -15.679 -20.657  40.895  1.00 28.28   D000 C
ATOM   3821  C   PRO C 207   -15.651 -21.107  42.360  1.00 33.47   D000 C
ATOM   3822  O   PRO C 207   -15.598 -20.266  43.255  1.00 30.13   D000 O
ATOM   3823  CB  PRO C 207   -15.741 -19.130  40.792  1.00 27.65   D000 C
ATOM   3824  CG  PRO C 207   -17.204 -18.830  40.787  1.00 30.21   D000 C
ATOM   3825  CD  PRO C 207   -17.873 -19.984  40.065  1.00 29.69   D000 C
ATOM   3826  N   VAL C 208   -15.674 -22.417  42.594  1.00 36.48   D000 N
ATOM   3827  CA  VAL C 208   -15.803 -22.969  43.944  1.00 29.83   D000 C
ATOM   3828  C   VAL C 208   -14.809 -24.105  44.182  1.00 33.04   D000 C
ATOM   3829  O   VAL C 208   -14.697 -25.001  43.351  1.00 26.89   D000 O
ATOM   3830  CB  VAL C 208   -17.233 -23.504  44.191  1.00 35.14   D000 C
```

```
ATOM   3831  CG1 VAL C 208   -17.349 -24.108  45.585  1.00 33.92      D000 C
ATOM   3832  CG2 VAL C 208   -18.271 -22.402  43.981  1.00 32.86      D000 C
ATOM   3833  N   ASN C 209   -14.092 -24.071  45.306  1.00 30.99      D000 N
ATOM   3834  CA  ASN C 209   -13.164 -25.153  45.649  1.00 31.35      D000 C
ATOM   3835  C   ASN C 209   -13.881 -26.500  45.682  1.00 32.29      D000 C
ATOM   3836  O   ASN C 209   -14.881 -26.666  46.379  1.00 29.08      D000 O
ATOM   3837  CB  ASN C 209   -12.486 -24.895  46.994  1.00 25.98      D000 C
ATOM   3838  CG  ASN C 209   -11.461 -23.781  46.931  1.00 30.97      D000 C
ATOM   3839  ND2 ASN C 209   -11.376 -22.997  48.000  1.00 35.10      D000 N
ATOM   3840  OD1 ASN C 209   -10.750 -23.626  45.938  1.00 30.97      D000 O
ATOM   3841  N   THR C 210   -13.363 -27.457  44.919  1.00 30.16      D000 N
ATOM   3842  CA  THR C 210   -14.049 -28.725  44.705  1.00 26.80      D000 C
ATOM   3843  C   THR C 210   -13.049 -29.865  44.549  1.00 26.68      D000 C
ATOM   3844  O   THR C 210   -12.182 -29.822  43.673  1.00 30.56      D000 O
ATOM   3845  CB  THR C 210   -14.949 -28.655  43.456  1.00 29.42      D000 C
ATOM   3846  CG2 THR C 210   -15.785 -29.916  43.312  1.00 22.22      D000 C
ATOM   3847  OG1 THR C 210   -15.813 -27.515  43.556  1.00 26.16      D000 O
ATOM   3848  N   TRP C 211   -13.165 -30.872  45.410  1.00 25.09      D000 N
ATOM   3849  CA  TRP C 211   -12.294 -32.041  45.357  1.00 30.07      D000 C
ATOM   3850  C   TRP C 211   -12.423 -32.796  44.038  1.00 36.81      D000 C
ATOM   3851  O   TRP C 211   -13.510 -32.874  43.456  1.00 27.60      D000 O
ATOM   3852  CB  TRP C 211   -12.605 -33.013  46.501  1.00 27.04      D000 C
ATOM   3853  CG  TRP C 211   -12.261 -32.549  47.900  1.00 28.12      D000 C
ATOM   3854  CD1 TRP C 211   -13.097 -32.529  48.983  1.00 28.01      D000 C
ATOM   3855  CD2 TRP C 211   -10.991 -32.072  48.366  1.00 26.23      D000 C
ATOM   3856  CE2 TRP C 211   -11.136 -31.771  49.739  1.00 31.66      D000 C
ATOM   3857  CE3 TRP C 211    -9.751 -31.857  47.756  1.00 23.53      D000 C
ATOM   3858  NE1 TRP C 211   -12.428 -32.060  50.091  1.00 29.06      D000 N
ATOM   3859  CZ2 TRP C 211   -10.084 -31.274  50.509  1.00 31.81      D000 C
ATOM   3860  CZ3 TRP C 211    -8.709 -31.361  48.522  1.00 26.50      D000 C
ATOM   3861  CH2 TRP C 211    -8.881 -31.078  49.885  1.00 32.71      D000 C
ATOM   3862  N   MET C 212   -11.310 -33.363  43.585  1.00 29.78      D000 N
ATOM   3863  CA  MET C 212   -11.327 -34.332  42.500  1.00 26.98      D000 C
ATOM   3864  C   MET C 212   -10.681 -35.620  43.009  1.00 30.71      D000 C
ATOM   3865  O   MET C 212   -10.179 -35.655  44.134  1.00 26.90      D000 O
ATOM   3866  CB  MET C 212   -10.598 -33.793  41.266  1.00 28.53      D000 C
ATOM   3867  CG  MET C 212    -9.082 -33.872  41.344  1.00 27.04      D000 C
ATOM   3868  SD  MET C 212    -8.287 -32.882  40.061  1.00 30.51      D000 S
ATOM   3869  CE  MET C 212    -8.761 -31.231  40.597  1.00 22.33      D000 C
ATOM   3870  N   GLY C 213   -10.693 -36.673  42.195  1.00 23.24      D000 N
ATOM   3871  CA  GLY C 213   -10.149 -37.955  42.612  1.00 21.72      D000 C
ATOM   3872  C   GLY C 213    -8.655 -38.108  42.405  1.00 27.75      D000 C
ATOM   3873  O   GLY C 213    -8.199 -39.106  41.851  1.00 28.41      D000 O
ATOM   3874  N   LEU C 214    -7.891 -37.124  42.870  1.00 25.92      D000 N
ATOM   3875  CA  LEU C 214    -6.443 -37.109  42.691  1.00 26.73      D000 C
ATOM   3876  C   LEU C 214    -5.755 -36.799  44.018  1.00 25.75      D000 C
ATOM   3877  O   LEU C 214    -6.073 -35.802  44.659  1.00 25.95      D000 O
ATOM   3878  CB  LEU C 214    -6.049 -36.076  41.629  1.00 24.90      D000 C
ATOM   3879  CG  LEU C 214    -4.566 -35.777  41.383  1.00 25.82      D000 C
ATOM   3880  CD1 LEU C 214    -3.840 -37.006  40.861  1.00 22.24      D000 C
ATOM   3881  CD2 LEU C 214    -4.428 -34.615  40.402  1.00 20.00      D000 C
ATOM   3882  N   HIS C 215    -4.819 -37.653  44.426  1.00 28.82      D000 N
ATOM   3883  CA  HIS C 215    -4.139 -37.488  45.709  1.00 25.47      D000 C
ATOM   3884  C   HIS C 215    -2.797 -38.207  45.727  1.00 27.39      D000 C
ATOM   3885  O   HIS C 215    -2.570 -39.123  44.938  1.00 28.76      D000 O
ATOM   3886  CB  HIS C 215    -5.011 -38.011  46.850  1.00 25.17      D000 C
ATOM   3887  CG  HIS C 215    -5.082 -39.505  46.915  1.00 33.85      D000 C
ATOM   3888  CD2 HIS C 215    -5.484 -40.419  45.999  1.00 36.27      D000 C
ATOM   3889  ND1 HIS C 215    -4.705 -40.221  48.030  1.00 43.52      D000 N
ATOM   3890  CE1 HIS C 215    -4.874 -41.511  47.680  1.00 45.66      D000 C
ATOM   3891  NE2 HIS C 215    -5.347 -41.657  46.575  1.00 38.89      D000 N
ATOM   3892  N   ASP C 216    -1.908 -37.803  46.632  1.00 31.91      D000 N
ATOM   3893  CA  ASP C 216    -0.623 -38.485  46.760  1.00 28.17      D000 C
ATOM   3894  C   ASP C 216    -0.369 -38.944  48.199  1.00 35.16      D000 C
ATOM   3895  O   ASP C 216     0.764 -38.955  48.668  1.00 31.36      D000 O
ATOM   3896  CB  ASP C 216     0.518 -37.585  46.258  1.00 28.42      D000 C
ATOM   3897  CG  ASP C 216     0.795 -36.394  47.171  1.00 35.01      D000 C
ATOM   3898  OD1 ASP C 216     0.013 -36.142  48.110  1.00 28.86      D000 O
ATOM   3899  OD2 ASP C 216     1.805 -35.695  46.936  1.00 31.47      D000 O
ATOM   3900  N   GLN C 217    -1.432 -39.332  48.894  1.00 31.06      D000 N
ATOM   3901  CA  GLN C 217    -1.305 -39.781  50.278  1.00 41.47      D000 C
```

```
ATOM   3902  C    GLN C 217      -0.531 -41.103  50.385  1.00 38.57      D000 C
ATOM   3903  O    GLN C 217       0.157 -41.346  51.374  1.00 44.82      D000 O
ATOM   3904  CB   GLN C 217      -2.688 -39.926  50.918  1.00 37.52      D000 C
ATOM   3905  CG   GLN C 217      -3.504 -38.644  50.919  1.00 33.20      D000 C
ATOM   3906  CD   GLN C 217      -4.837 -38.799  51.626  1.00 39.32      D000 C
ATOM   3907  NE2  GLN C 217      -5.810 -39.389  50.941  1.00 31.27      D000 N
ATOM   3908  OE1  GLN C 217      -4.989 -38.391  52.775  1.00 48.65      D000 O
ATOM   3909  N    ASN C 218      -0.630 -41.939  49.356  1.00 38.48      D000 N
ATOM   3910  CA   ASN C 218       0.018 -43.251  49.363  1.00 53.29      D000 C
ATOM   3911  C    ASN C 218       1.205 -43.357  48.405  1.00 47.43      D000 C
ATOM   3912  O    ASN C 218       1.397 -44.389  47.761  1.00 58.53      D000 O
ATOM   3913  CB   ASN C 218      -1.004 -44.339  49.018  1.00 57.61      D000 C
ATOM   3914  CG   ASN C 218      -2.213 -44.313  49.933  1.00 65.75      D000 C
ATOM   3915  ND2  ASN C 218      -1.966 -44.302  51.241  1.00 59.68      D000 N
ATOM   3916  OD1  ASN C 218      -3.356 -44.293  49.471  1.00 59.70      D000 O
ATOM   3917  N    GLY C 219       2.001 -42.299  48.313  1.00 35.56      D000 N
ATOM   3918  CA   GLY C 219       3.081 -42.255  47.344  1.00 36.85      D000 C
ATOM   3919  C    GLY C 219       2.873 -41.128  46.353  1.00 34.18      D000 C
ATOM   3920  O    GLY C 219       2.347 -40.080  46.716  1.00 32.25      D000 O
ATOM   3921  N    PRO C 220       3.284 -41.332  45.092  1.00 35.79      D000 N
ATOM   3922  CA   PRO C 220       3.144 -40.284  44.072  1.00 33.07      D000 C
ATOM   3923  C    PRO C 220       1.683 -40.001  43.728  1.00 31.10      D000 C
ATOM   3924  O    PRO C 220       0.801 -40.767  44.117  1.00 29.49      D000 O
ATOM   3925  CB   PRO C 220       3.897 -40.859  42.867  1.00 37.25      D000 C
ATOM   3926  CG   PRO C 220       3.868 -42.336  43.073  1.00 41.99      D000 C
ATOM   3927  CD   PRO C 220       3.925 -42.547  44.558  1.00 34.70      D000 C
ATOM   3928  N    TRP C 221       1.433 -38.904  43.019  1.00 28.69      D000 N
ATOM   3929  CA   TRP C 221       0.070 -38.514  42.668  1.00 29.66      D000 C
ATOM   3930  C    TRP C 221      -0.626 -39.607  41.857  1.00 29.27      D000 C
ATOM   3931  O    TRP C 221      -0.081 -40.110  40.879  1.00 26.36      D000 O
ATOM   3932  CB   TRP C 221       0.075 -37.185  41.900  1.00 27.58      D000 C
ATOM   3933  CG   TRP C 221       0.406 -36.020  42.782  1.00 30.86      D000 C
ATOM   3934  CD1  TRP C 221       1.629 -35.437  42.947  1.00 32.27      D000 C
ATOM   3935  CD2  TRP C 221      -0.495 -35.311  43.643  1.00 27.75      D000 C
ATOM   3936  CE2  TRP C 221       0.253 -34.309  44.298  1.00 32.54      D000 C
ATOM   3937  CE3  TRP C 221      -1.861 -35.428  43.926  1.00 25.76      D000 C
ATOM   3938  NE1  TRP C 221       1.545 -34.406  43.853  1.00 32.24      D000 N
ATOM   3939  CZ2  TRP C 221      -0.320 -33.425  45.219  1.00 27.09      D000 C
ATOM   3940  CZ3  TRP C 221      -2.430 -34.549  44.841  1.00 29.33      D000 C
ATOM   3941  CH2  TRP C 221      -1.658 -33.560  45.474  1.00 29.04      D000 C
ATOM   3942  N    LYS C 222      -1.826 -39.981  42.289  1.00 30.12      D000 N
ATOM   3943  CA   LYS C 222      -2.585 -41.046  41.643  1.00 30.34      D000 C
ATOM   3944  C    LYS C 222      -4.058 -40.687  41.502  1.00 27.82      D000 C
ATOM   3945  O    LYS C 222      -4.627 -40.026  42.370  1.00 29.51      D000 O
ATOM   3946  CB   LYS C 222      -2.453 -42.351  42.436  1.00 35.42      D000 C
ATOM   3947  CG   LYS C 222      -1.072 -42.983  42.388  1.00 42.04      D000 C
ATOM   3948  CD   LYS C 222      -0.966 -44.138  43.373  1.00 62.76      D000 C
ATOM   3949  CE   LYS C 222       0.452 -44.687  43.443  1.00 66.16      D000 C
ATOM   3950  NZ   LYS C 222       0.599 -45.699  44.533  1.00 72.02      D000 N
ATOM   3951  N    TRP C 223      -4.670 -41.121  40.406  1.00 29.14      D000 N
ATOM   3952  CA   TRP C 223      -6.119 -41.022  40.249  1.00 31.28      D000 C
ATOM   3953  C    TRP C 223      -6.787 -42.218  40.920  1.00 29.31      D000 C
ATOM   3954  O    TRP C 223      -6.254 -43.321  40.883  1.00 30.17      D000 O
ATOM   3955  CB   TRP C 223      -6.512 -40.959  38.774  1.00 23.94      D000 C
ATOM   3956  CG   TRP C 223      -6.104 -39.694  38.085  1.00 27.95      D000 C
ATOM   3957  CD1  TRP C 223      -5.059 -39.530  37.226  1.00 23.23      D000 C
ATOM   3958  CD2  TRP C 223      -6.742 -38.414  38.191  1.00 24.93      D000 C
ATOM   3959  CE2  TRP C 223      -6.026 -37.523  37.365  1.00 23.92      D000 C
ATOM   3960  CE3  TRP C 223      -7.851 -37.938  38.901  1.00 23.92      D000 C
ATOM   3961  NE1  TRP C 223      -5.002 -38.228  36.790  1.00 25.78      D000 N
ATOM   3962  CZ2  TRP C 223      -6.382 -36.182  37.228  1.00 28.71      D000 C
ATOM   3963  CZ3  TRP C 223      -8.201 -36.603  38.771  1.00 28.69      D000 C
ATOM   3964  CH2  TRP C 223      -7.468 -35.740  37.941  1.00 27.80      D000 C
ATOM   3965  N    VAL C 224      -7.958 -42.001  41.514  1.00 28.47      D000 N
ATOM   3966  CA   VAL C 224      -8.620 -43.038  42.302  1.00 29.16      D000 C
ATOM   3967  C    VAL C 224      -9.055 -44.259  41.486  1.00 31.96      D000 C
ATOM   3968  O    VAL C 224      -9.186 -45.349  42.038  1.00 33.21      D000 O
ATOM   3969  CB   VAL C 224      -9.866 -42.485  43.037  1.00 27.95      D000 C
ATOM   3970  CG1  VAL C 224      -9.463 -41.450  44.071  1.00 29.62      D000 C
ATOM   3971  CG2  VAL C 224     -10.863 -41.900  42.050  1.00 31.35      D000 C
ATOM   3972  N    ASP C 225      -9.272 -44.083  40.184  1.00 30.51      D000 N
```

```
ATOM   3973  CA  ASP C 225     -9.755 -45.182  39.347  1.00 33.42      D000 C
ATOM   3974  C   ASP C 225     -8.622 -45.903  38.615  1.00 35.01      D000 C
ATOM   3975  O   ASP C 225     -8.866 -46.705  37.717  1.00 45.07      D000 O
ATOM   3976  CB  ASP C 225    -10.798 -44.673  38.337  1.00 34.24      D000 C
ATOM   3977  CG  ASP C 225    -10.187 -43.849  37.205  1.00 39.60      D000 C
ATOM   3978  OD1 ASP C 225     -9.032 -43.379  37.334  1.00 35.30      D000 O
ATOM   3979  OD2 ASP C 225    -10.878 -43.658  36.179  1.00 41.15      D000 O
ATOM   3980  N   GLY C 226     -7.383 -45.605  38.989  1.00 34.08      D000 N
ATOM   3981  CA  GLY C 226     -6.241 -46.272  38.389  1.00 29.68      D000 C
ATOM   3982  C   GLY C 226     -5.723 -45.631  37.115  1.00 34.65      D000 C
ATOM   3983  O   GLY C 226     -4.725 -46.086  36.555  1.00 35.05      D000 O
ATOM   3984  N   THR C 227     -6.394 -44.578  36.652  1.00 34.54      D000 N
ATOM   3985  CA  THR C 227     -5.926 -43.829  35.486  1.00 32.36      D000 C
ATOM   3986  C   THR C 227     -4.500 -43.333  35.721  1.00 35.82      D000 C
ATOM   3987  O   THR C 227     -4.196 -42.779  36.779  1.00 36.25      D000 O
ATOM   3988  CB  THR C 227     -6.840 -42.625  35.175  1.00 36.95      D000 C
ATOM   3989  CG2 THR C 227     -6.315 -41.834  33.980  1.00 37.07      D000 C
ATOM   3990  OG1 THR C 227     -8.164 -43.089  34.891  1.00 40.09      D000 O
ATOM   3991  N   ASP C 228     -3.625 -43.540  34.743  1.00 35.21      D000 N
ATOM   3992  CA  ASP C 228     -2.227 -43.165  34.900  1.00 35.66      D000 C
ATOM   3993  C   ASP C 228     -2.063 -41.648  34.951  1.00 40.45      D000 C
ATOM   3994  O   ASP C 228     -2.550 -40.926  34.080  1.00 33.72      D000 O
ATOM   3995  CB  ASP C 228     -1.378 -43.748  33.773  1.00 36.06      D000 C
ATOM   3996  CG  ASP C 228      0.098 -43.451  33.953  1.00 42.86      D000 C
ATOM   3997  OD1 ASP C 228      0.720 -44.052  34.855  1.00 52.83      D000 O
ATOM   3998  OD2 ASP C 228      0.636 -42.613  33.199  1.00 43.62      D000 O
ATOM   3999  N   TYR C 229     -1.372 -41.168  35.978  1.00 34.02      D000 N
ATOM   4000  CA  TYR C 229     -1.267 -39.735  36.202  1.00 35.25      D000 C
ATOM   4001  C   TYR C 229     -0.210 -39.078  35.320  1.00 32.64      D000 C
ATOM   4002  O   TYR C 229     -0.470 -38.045  34.713  1.00 32.76      D000 O
ATOM   4003  CB  TYR C 229     -0.967 -39.448  37.676  1.00 34.94      D000 C
ATOM   4004  CG  TYR C 229     -0.488 -38.039  37.936  1.00 31.42      D000 C
ATOM   4005  CD1 TYR C 229     -1.381 -36.975  37.988  1.00 28.67      D000 C
ATOM   4006  CD2 TYR C 229      0.860 -37.773  38.129  1.00 36.14      D000 C
ATOM   4007  CE1 TYR C 229     -0.941 -35.684  38.220  1.00 24.57      D000 C
ATOM   4008  CE2 TYR C 229      1.309 -36.493  38.366  1.00 33.76      D000 C
ATOM   4009  CZ  TYR C 229      0.407 -35.452  38.412  1.00 34.92      D000 C
ATOM   4010  OH  TYR C 229      0.869 -34.176  38.646  1.00 35.23      D000 O
ATOM   4011  N   GLU C 230      0.973 -39.679  35.244  1.00 32.24      D000 N
ATOM   4012  CA  GLU C 230      2.108 -39.042  34.578  1.00 40.08      D000 C
ATOM   4013  C   GLU C 230      1.866 -38.810  33.086  1.00 34.17      D000 C
ATOM   4014  O   GLU C 230      2.253 -37.777  32.545  1.00 32.51      D000 O
ATOM   4015  CB  GLU C 230      3.381 -39.869  34.774  1.00 40.46      D000 C
ATOM   4016  CG  GLU C 230      4.639 -39.157  34.302  1.00 62.30      D000 C
ATOM   4017  CD  GLU C 230      5.913 -39.799  34.821  1.00100.35      D000 C
ATOM   4018  OE1 GLU C 230      5.824 -40.683  35.701  1.00 96.71      D000 O
ATOM   4019  OE2 GLU C 230      7.006 -39.416  34.349  1.00104.16      D000 O
ATOM   4020  N   THR C 231      1.210 -39.760  32.428  1.00 32.37      D000 N
ATOM   4021  CA  THR C 231      0.942 -39.638  31.001  1.00 35.98      D000 C
ATOM   4022  C   THR C 231     -0.426 -39.018  30.742  1.00 40.00      D000 C
ATOM   4023  O   THR C 231     -0.829 -38.837  29.593  1.00 34.33      D000 O
ATOM   4024  CB  THR C 231      1.015 -41.005  30.291  1.00 45.18      D000 C
ATOM   4025  CG2 THR C 231      2.392 -41.634  30.485  1.00 36.03      D000 C
ATOM   4026  OG1 THR C 231      0.004 -41.878  30.813  1.00 40.41      D000 O
ATOM   4027  N   GLY C 232     -1.135 -38.685  31.815  1.00 31.90      D000 N
ATOM   4028  CA  GLY C 232     -2.478 -38.155  31.689  1.00 35.88      D000 C
ATOM   4029  C   GLY C 232     -2.556 -36.641  31.641  1.00 31.67      D000 C
ATOM   4030  O   GLY C 232     -1.569 -35.940  31.855  1.00 34.20      D000 O
ATOM   4031  N   PHE C 233     -3.754 -36.148  31.351  1.00 34.66      D000 N
ATOM   4032  CA  PHE C 233     -4.057 -34.723  31.357  1.00 30.10      D000 C
ATOM   4033  C   PHE C 233     -3.792 -34.089  32.730  1.00 28.30      D000 C
ATOM   4034  O   PHE C 233     -4.053 -34.702  33.766  1.00 31.59      D000 O
ATOM   4035  CB  PHE C 233     -5.520 -34.520  30.932  1.00 29.82      D000 C
ATOM   4036  CG  PHE C 233     -6.022 -33.115  31.094  1.00 33.74      D000 C
ATOM   4037  CD1 PHE C 233     -5.829 -32.175  30.088  1.00 27.88      D000 C
ATOM   4038  CD2 PHE C 233     -6.715 -32.739  32.240  1.00 24.62      D000 C
ATOM   4039  CE1 PHE C 233     -6.298 -30.878  30.231  1.00 27.56      D000 C
ATOM   4040  CE2 PHE C 233     -7.184 -31.441  32.389  1.00 32.64      D000 C
ATOM   4041  CZ  PHE C 233     -6.975 -30.510  31.381  1.00 27.36      D000 C
ATOM   4042  N   LYS C 234     -3.267 -32.866  32.730  1.00 29.76      D000 N
ATOM   4043  CA  LYS C 234     -3.045 -32.109  33.963  1.00 29.48      D000 C
```

```
ATOM   4044  C    LYS C 234     -3.455 -30.646  33.789  1.00 31.02      D000 C
ATOM   4045  O    LYS C 234     -3.316 -30.077  32.704  1.00 25.86      D000 O
ATOM   4046  CB   LYS C 234     -1.579 -32.189  34.397  1.00 32.09      D000 C
ATOM   4047  CG   LYS C 234     -1.056 -33.603  34.611  1.00 30.92      D000 C
ATOM   4048  CD   LYS C 234      0.422 -33.598  34.955  1.00 34.48      D000 C
ATOM   4049  CE   LYS C 234      1.026 -34.993  34.843  1.00 34.77      D000 C
ATOM   4050  NZ   LYS C 234      1.050 -35.479  33.432  1.00 34.51      D000 N
ATOM   4051  N    ASN C 235     -3.957 -30.040  34.861  1.00 29.35      D000 N
ATOM   4052  CA   ASN C 235     -4.382 -28.644  34.822  1.00 26.46      D000 C
ATOM   4053  C    ASN C 235     -4.012 -27.912  36.116  1.00 26.48      D000 C
ATOM   4054  O    ASN C 235     -4.814 -27.157  36.659  1.00 29.79      D000 O
ATOM   4055  CB   ASN C 235     -5.896 -28.559  34.573  1.00 25.06      D000 C
ATOM   4056  CG   ASN C 235     -6.345 -27.173  34.127  1.00 27.67      D000 C
ATOM   4057  ND2  ASN C 235     -7.464 -26.705  34.673  1.00 26.28      D000 N
ATOM   4058  OD1  ASN C 235     -5.692 -26.533  33.308  1.00 27.02      D000 O
ATOM   4059  N    TRP C 236     -2.795 -28.145  36.607  1.00 27.50      D000 N
ATOM   4060  CA   TRP C 236     -2.325 -27.526  37.850  1.00 27.74      D000 C
ATOM   4061  C    TRP C 236     -2.206 -26.010  37.747  1.00 27.99      D000 C
ATOM   4062  O    TRP C 236     -1.870 -25.474  36.690  1.00 31.33      D000 O
ATOM   4063  CB   TRP C 236     -0.960 -28.092  38.264  1.00 23.47      D000 C
ATOM   4064  CG   TRP C 236     -0.967 -29.533  38.710  1.00 24.40      D000 C
ATOM   4065  CD1  TRP C 236     -0.544 -30.616  37.990  1.00 25.63      D000 C
ATOM   4066  CD2  TRP C 236     -1.407 -30.040  39.979  1.00 27.52      D000 C
ATOM   4067  CE2  TRP C 236     -1.224 -31.442  39.955  1.00 25.85      D000 C
ATOM   4068  CE3  TRP C 236     -1.940 -29.450  41.132  1.00 25.98      D000 C
ATOM   4069  NE1  TRP C 236     -0.696 -31.766  38.732  1.00 26.26      D000 N
ATOM   4070  CZ2  TRP C 236     -1.551 -32.258  41.037  1.00 25.53      D000 C
ATOM   4071  CZ3  TRP C 236     -2.267 -30.263  42.208  1.00 25.29      D000 C
ATOM   4072  CH2  TRP C 236     -2.071 -31.654  42.152  1.00 26.90      D000 C
ATOM   4073  N    ARG C 237     -2.473 -25.323  38.855  1.00 29.23      D000 N
ATOM   4074  CA   ARG C 237     -2.100 -23.918  38.983  1.00 34.44      D000 C
ATOM   4075  C    ARG C 237     -0.586 -23.790  38.820  1.00 34.81      D000 C
ATOM   4076  O    ARG C 237      0.147 -24.755  39.052  1.00 28.62      D000 O
ATOM   4077  CB   ARG C 237     -2.547 -23.349  40.337  1.00 30.99      D000 C
ATOM   4078  CG   ARG C 237     -4.048 -23.189  40.474  1.00 37.60      D000 C
ATOM   4079  CD   ARG C 237     -4.455 -21.737  40.591  1.00 42.14      D000 C
ATOM   4080  NE   ARG C 237     -4.482 -21.299  41.980  1.00 49.94      D000 N
ATOM   4081  CZ   ARG C 237     -5.493 -20.641  42.538  1.00 55.18      D000 C
ATOM   4082  NH1  ARG C 237     -6.568 -20.334  41.822  1.00 41.85      D000 N
ATOM   4083  NH2  ARG C 237     -5.428 -20.287  43.814  1.00 55.93      D000 N
ATOM   4084  N    PRO C 238     -0.113 -22.606  38.401  1.00 36.47      D000 N
ATOM   4085  CA   PRO C 238      1.334 -22.380  38.314  1.00 36.67      D000 C
ATOM   4086  C    PRO C 238      2.051 -22.730  39.619  1.00 31.29      D000 C
ATOM   4087  O    PRO C 238      1.581 -22.352  40.692  1.00 39.20      D000 O
ATOM   4088  CB   PRO C 238      1.435 -20.881  38.015  1.00 41.44      D000 C
ATOM   4089  CG   PRO C 238      0.172 -20.566  37.297  1.00 41.47      D000 C
ATOM   4090  CD   PRO C 238     -0.886 -21.461  37.885  1.00 34.59      D000 C
ATOM   4091  N    GLU C 239      3.149 -23.473  39.502  1.00 34.14      D000 N
ATOM   4092  CA   GLU C 239      4.016 -23.854  40.624  1.00 40.34      D000 C
ATOM   4093  C    GLU C 239      3.405 -24.898  41.566  1.00 40.27      D000 C
ATOM   4094  O    GLU C 239      3.952 -25.164  42.636  1.00 43.88      D000 O
ATOM   4095  CB   GLU C 239      4.428 -22.615  41.426  1.00 43.42      D000 C
ATOM   4096  CG   GLU C 239      5.157 -21.571  40.598  1.00 50.20      D000 C
ATOM   4097  CD   GLU C 239      5.859 -20.539  41.453  1.00 75.90      D000 C
ATOM   4098  OE1  GLU C 239      5.212 -19.540  41.836  1.00 82.09      D000 O
ATOM   4099  OE2  GLU C 239      7.059 -20.732  41.744  1.00 84.44      D000 O
ATOM   4100  N    GLN C 240      2.285 -25.493  41.165  1.00 33.28      D000 N
ATOM   4101  CA   GLN C 240      1.703 -26.611  41.904  1.00 31.36      D000 C
ATOM   4102  C    GLN C 240      1.873 -27.891  41.082  1.00 31.46      D000 C
ATOM   4103  O    GLN C 240      1.926 -27.827  39.856  1.00 30.03      D000 O
ATOM   4104  CB   GLN C 240      0.224 -26.354  42.208  1.00 28.17      D000 C
ATOM   4105  CG   GLN C 240     -0.065 -25.007  42.862  1.00 36.91      D000 C
ATOM   4106  CD   GLN C 240      0.799 -24.737  44.083  1.00 42.84      D000 C
ATOM   4107  NE2  GLN C 240      1.452 -23.580  44.095  1.00 37.20      D000 N
ATOM   4108  OE1  GLN C 240      0.885 -25.559  45.001  1.00 41.85      D000 O
ATOM   4109  N    PRO C 241      1.968 -29.060  41.746  1.00 31.89      D000 N
ATOM   4110  CA   PRO C 241      1.952 -29.276  43.200  1.00 36.63      D000 C
ATOM   4111  C    PRO C 241      3.307 -28.999  43.845  1.00 34.40      D000 C
ATOM   4112  O    PRO C 241      4.332 -29.013  43.162  1.00 31.05      D000 O
ATOM   4113  CB   PRO C 241      1.583 -30.757  43.326  1.00 30.98      D000 C
ATOM   4114  CG   PRO C 241      2.166 -31.380  42.097  1.00 28.05      D000 C
```

| ATOM | 4115 | CD | PRO | C 241 | 2.042 | -30.334 | 41.004 | 1.00 | 28.29 | D000 C |
|------|------|----|-----|-------|-------|---------|--------|------|-------|--------|
| ATOM | 4116 | N | ASP | C 242 | 3.306 | -28.762 | 45.151 | 1.00 | 31.20 | D000 N |
| ATOM | 4117 | CA | ASP | C 242 | 4.534 | -28.429 | 45.869 | 1.00 | 33.88 | D000 C |
| ATOM | 4118 | C | ASP | C 242 | 5.428 | -29.640 | 46.150 | 1.00 | 36.07 | D000 C |
| ATOM | 4119 | O | ASP | C 242 | 6.583 | -29.482 | 46.545 | 1.00 | 35.39 | D000 O |
| ATOM | 4120 | CB | ASP | C 242 | 4.189 | -27.712 | 47.176 | 1.00 | 30.91 | D000 C |
| ATOM | 4121 | CG | ASP | C 242 | 3.796 | -26.257 | 46.953 | 1.00 | 42.15 | D000 C |
| ATOM | 4122 | OD2 | ASP | C 242 | 2.709 | -25.848 | 47.409 | 1.00 | 41.70 | D000 O |
| ATOM | 4123 | OD1 | ASP | C 242 | 4.571 | -25.527 | 46.298 | 1.00 | 38.03 | D000 O |
| ATOM | 4124 | N | ASP | C 243 | 4.894 | -30.841 | 45.935 | 1.00 | 33.28 | D000 N |
| ATOM | 4125 | CA | ASP | C 243 | 5.659 | -32.080 | 46.098 | 1.00 | 33.76 | D000 C |
| ATOM | 4126 | C | ASP | C 243 | 5.140 | -33.135 | 45.123 | 1.00 | 36.43 | D000 C |
| ATOM | 4127 | O | ASP | C 243 | 4.000 | -33.045 | 44.662 | 1.00 | 31.00 | D000 O |
| ATOM | 4128 | CB | ASP | C 243 | 5.562 | -32.592 | 47.539 | 1.00 | 33.56 | D000 C |
| ATOM | 4129 | CG | ASP | C 243 | 6.805 | -33.360 | 47.980 | 1.00 | 45.01 | D000 C |
| ATOM | 4130 | OD1 | ASP | C 243 | 7.510 | -33.935 | 47.118 | 1.00 | 32.68 | D000 O |
| ATOM | 4131 | OD2 | ASP | C 243 | 7.073 | -33.389 | 49.201 | 1.00 | 41.68 | D000 O |
| ATOM | 4132 | O | TRP | C 244 | 4.635 | -37.408 | 43.978 | 1.00 | 29.40 | D000 O |
| ATOM | 4133 | N | TRP | C 244 | 5.959 | -34.135 | 44.811 | 1.00 | 26.76 | D000 N |
| ATOM | 4134 | CA | TRP | C 244 | 5.530 | -35.179 | 43.886 | 1.00 | 32.96 | D000 C |
| ATOM | 4135 | C | TRP | C 244 | 5.096 | -36.460 | 44.610 | 1.00 | 33.98 | D000 C |
| ATOM | 4136 | CB | TRP | C 244 | 6.635 | -35.486 | 42.872 | 1.00 | 22.49 | D000 C |
| ATOM | 4137 | CG | TRP | C 244 | 7.926 | -35.939 | 43.487 | 1.00 | 31.66 | D000 C |
| ATOM | 4138 | CD1 | TRP | C 244 | 8.985 | -35.153 | 43.851 | 1.00 | 35.24 | D000 C |
| ATOM | 4139 | CD2 | TRP | C 244 | 8.301 | -37.286 | 43.797 | 1.00 | 28.87 | D000 C |
| ATOM | 4140 | NE1 | TRP | C 244 | 9.992 | -35.930 | 44.374 | 1.00 | 39.02 | D000 N |
| ATOM | 4141 | CE2 | TRP | C 244 | 9.597 | -37.243 | 44.351 | 1.00 | 34.79 | D000 C |
| ATOM | 4142 | CE3 | TRP | C 244 | 7.666 | -38.526 | 43.661 | 1.00 | 27.95 | D000 C |
| ATOM | 4143 | CZ2 | TRP | C 244 | 10.267 | -38.390 | 44.770 | 1.00 | 32.14 | D000 C |
| ATOM | 4144 | CZ3 | TRP | C 244 | 8.333 | -39.662 | 44.076 | 1.00 | 30.86 | D000 C |
| ATOM | 4145 | CH2 | TRP | C 244 | 9.621 | -39.586 | 44.624 | 1.00 | 31.78 | D000 C |
| ATOM | 4146 | O | TYR | C 245 | 4.758 | -35.925 | 48.510 | 1.00 | 29.36 | D000 O |
| ATOM | 4147 | N | TYR | C 245 | 5.250 | -36.491 | 45.930 | 1.00 | 32.02 | D000 N |
| ATOM | 4148 | CA | TYR | C 245 | 4.614 | -37.534 | 46.735 | 1.00 | 28.48 | D000 C |
| ATOM | 4149 | C | TYR | C 245 | 4.247 | -36.969 | 48.099 | 1.00 | 30.14 | D000 C |
| ATOM | 4150 | CB | TYR | C 245 | 5.502 | -38.778 | 46.871 | 1.00 | 25.38 | D000 C |
| ATOM | 4151 | CG | TYR | C 245 | 6.734 | -38.633 | 47.739 | 1.00 | 32.91 | D000 C |
| ATOM | 4152 | CD2 | TYR | C 245 | 6.785 | -39.200 | 49.009 | 1.00 | 31.25 | D000 C |
| ATOM | 4153 | CD1 | TYR | C 245 | 7.865 | -37.975 | 47.270 | 1.00 | 30.40 | D000 C |
| ATOM | 4154 | CE2 | TYR | C 245 | 7.920 | -39.093 | 49.795 | 1.00 | 28.79 | D000 C |
| ATOM | 4155 | CE1 | TYR | C 245 | 8.999 | -37.860 | 48.049 | 1.00 | 32.06 | D000 C |
| ATOM | 4156 | CZ | TYR | C 245 | 9.025 | -38.420 | 49.307 | 1.00 | 29.81 | D000 C |
| ATOM | 4157 | OH | TYR | C 245 | 10.159 | -38.300 | 50.077 | 1.00 | 27.57 | D000 O |
| ATOM | 4158 | O | GLY | C 246 | 4.628 | -37.473 | 51.323 | 1.00 | 34.84 | D000 O |
| ATOM | 4159 | N | GLY | C 246 | 3.340 | -37.650 | 48.789 | 1.00 | 31.19 | D000 N |
| ATOM | 4160 | CA | GLY | C 246 | 2.703 | -37.067 | 49.955 | 1.00 | 35.76 | D000 C |
| ATOM | 4161 | C | GLY | C 246 | 3.427 | -37.207 | 51.272 | 1.00 | 33.62 | D000 C |
| ATOM | 4162 | O | HIS | C 247 | 2.259 | -37.710 | 55.785 | 1.00 | 43.05 | D000 O |
| ATOM | 4163 | N | HIS | C 247 | 2.664 | -37.031 | 52.345 | 1.00 | 38.43 | D000 N |
| ATOM | 4164 | CA | HIS | C 247 | 3.202 | -36.998 | 53.694 | 1.00 | 39.05 | D000 C |
| ATOM | 4165 | C | HIS | C 247 | 2.411 | -37.945 | 54.588 | 1.00 | 38.77 | D000 C |
| ATOM | 4166 | CB | HIS | C 247 | 3.164 | -35.565 | 54.234 | 1.00 | 39.25 | D000 C |
| ATOM | 4167 | CG | HIS | C 247 | 3.692 | -34.553 | 53.266 | 1.00 | 36.64 | D000 C |
| ATOM | 4168 | ND1 | HIS | C 247 | 4.970 | -34.039 | 53.346 | 1.00 | 46.82 | D000 N |
| ATOM | 4169 | CD2 | HIS | C 247 | 3.126 | -33.978 | 52.179 | 1.00 | 35.16 | D000 C |
| ATOM | 4170 | CE1 | HIS | C 247 | 5.163 | -33.185 | 52.358 | 1.00 | 42.11 | D000 C |
| ATOM | 4171 | NE2 | HIS | C 247 | 4.059 | -33.130 | 51.632 | 1.00 | 47.04 | D000 N |
| ATOM | 4172 | O | GLY | C 248 | -0.747 | -38.509 | 54.445 | 1.00 | 40.24 | D000 O |
| ATOM | 4173 | N | GLY | C 248 | 1.903 | -39.018 | 53.991 | 1.00 | 40.96 | D000 N |
| ATOM | 4174 | CA | GLY | C 248 | 1.118 | -39.995 | 54.720 | 1.00 | 37.66 | D000 C |
| ATOM | 4175 | C | GLY | C 248 | -0.330 | -39.578 | 54.902 | 1.00 | 47.53 | D000 C |
| ATOM | 4176 | O | LEU | C 249 | -3.773 | -38.603 | 57.140 | 1.00 | 54.25 | D000 O |
| ATOM | 4177 | N | LEU | C 249 | -1.097 | -40.433 | 55.574 | 1.00 | 52.35 | D000 N |
| ATOM | 4178 | CA | LEU | C 249 | -2.508 | -40.173 | 55.842 | 1.00 | 52.11 | D000 C |
| ATOM | 4179 | C | LEU | C 249 | -2.689 | -39.160 | 56.968 | 1.00 | 52.40 | D000 C |
| ATOM | 4180 | CB | LEU | C 249 | -3.233 | -41.474 | 56.202 | 1.00 | 42.33 | D000 C |
| ATOM | 4181 | CG | LEU | C 249 | -3.333 | -42.572 | 55.139 | 1.00 | 60.62 | D000 C |
| ATOM | 4182 | CD2 | LEU | C 249 | -4.287 | -42.165 | 54.027 | 1.00 | 56.35 | D000 C |
| ATOM | 4183 | CD1 | LEU | C 249 | -3.789 | -43.874 | 55.773 | 1.00 | 55.71 | D000 C |
| ATOM | 4184 | O | GLY | C 250 | -1.984 | -35.849 | 59.639 | 1.00 | 57.54 | D000 O |
| ATOM | 4185 | N | GLY | C 250 | -1.622 | -38.924 | 57.727 | 1.00 | 52.24 | D000 N |

```
ATOM   4186  CA   GLY C 250      -1.698 -38.112  58.930  1.00 50.41      D000 C
ATOM   4187  C    GLY C 250      -1.663 -36.608  58.725  1.00 57.93      D000 C
ATOM   4188  N    GLY C 251      -1.269 -36.168  57.535  1.00 52.64      D000 N
ATOM   4189  CA   GLY C 251      -1.203 -34.746  57.250  1.00 44.98      D000 C
ATOM   4190  C    GLY C 251      -0.639 -34.433  55.880  1.00 46.45      D000 C
ATOM   4191  O    GLY C 251      -0.459 -35.329  55.051  1.00 43.28      D000 O
ATOM   4192  N    GLY C 252      -0.369 -33.152  55.642  1.00 37.49      D000 N
ATOM   4193  CA   GLY C 252       0.205 -32.710  54.387  1.00 32.69      D000 C
ATOM   4194  C    GLY C 252      -0.831 -32.272  53.366  1.00 34.76      D000 C
ATOM   4195  O    GLY C 252      -2.005 -32.646  53.445  1.00 30.54      D000 O
ATOM   4196  N    GLU C 253      -0.385 -31.472  52.402  1.00 33.99      D000 N
ATOM   4197  CA   GLU C 253      -1.250 -30.989  51.331  1.00 40.45      D000 C
ATOM   4198  C    GLU C 253      -1.285 -32.029  50.222  1.00 29.00      D000 C
ATOM   4199  O    GLU C 253      -0.676 -31.860  49.168  1.00 35.30      D000 O
ATOM   4200  CB   GLU C 253      -0.757 -29.632  50.821  1.00 34.32      D000 C
ATOM   4201  CG   GLU C 253      -0.717 -28.572  51.926  1.00 42.40      D000 C
ATOM   4202  CD   GLU C 253      -0.225 -27.217  51.444  1.00 58.06      D000 C
ATOM   4203  OE1  GLU C 253      -0.620 -26.792  50.337  1.00 59.58      D000 O
ATOM   4204  OE2  GLU C 253       0.557 -26.574  52.177  1.00 57.93      D000 O
ATOM   4205  N    ASP C 254      -2.015 -33.109  50.484  1.00 27.38      D000 N
ATOM   4206  CA   ASP C 254      -1.921 -34.327  49.693  1.00 31.18      D000 C
ATOM   4207  C    ASP C 254      -3.140 -34.599  48.805  1.00 33.58      D000 C
ATOM   4208  O    ASP C 254      -3.216 -35.646  48.158  1.00 27.45      D000 O
ATOM   4209  CB   ASP C 254      -1.700 -35.535  50.623  1.00 29.94      D000 C
ATOM   4210  CG   ASP C 254      -0.346 -35.504  51.328  1.00 36.48      D000 C
ATOM   4211  OD1  ASP C 254       0.494 -34.630  51.013  1.00 28.05      D000 O
ATOM   4212  OD2  ASP C 254      -0.117 -36.374  52.196  1.00 35.72      D000 O
ATOM   4213  N    CYS C 255      -4.090 -33.670  48.768  1.00 26.75      D000 N
ATOM   4214  CA   CYS C 255      -5.294 -33.867  47.963  1.00 26.33      D000 C
ATOM   4215  C    CYS C 255      -5.518 -32.727  46.977  1.00 32.54      D000 C
ATOM   4216  O    CYS C 255      -5.323 -31.559  47.316  1.00 27.66      D000 O
ATOM   4217  CB   CYS C 255      -6.514 -34.024  48.869  1.00 27.73      D000 C
ATOM   4218  SG   CYS C 255      -6.407 -35.447  49.969  1.00 35.91      D000 S
ATOM   4219  N    ALA C 256      -5.930 -33.072  45.758  1.00 28.09      D000 N
ATOM   4220  CA   ALA C 256      -6.116 -32.073  44.710  1.00 28.14      D000 C
ATOM   4221  C    ALA C 256      -7.560 -31.598  44.637  1.00 28.47      D000 C
ATOM   4222  O    ALA C 256      -8.500 -32.389  44.749  1.00 30.35      D000 O
ATOM   4223  CB   ALA C 256      -5.678 -32.627  43.357  1.00 25.70      D000 C
ATOM   4224  N    HIS C 257      -7.732 -30.298  44.444  1.00 28.56      D000 N
ATOM   4225  CA   HIS C 257      -9.054 -29.742  44.223  1.00 25.18      D000 C
ATOM   4226  C    HIS C 257      -9.020 -28.689  43.127  1.00 31.48      D000 C
ATOM   4227  O    HIS C 257      -7.990 -28.054  42.895  1.00 26.66      D000 O
ATOM   4228  CB   HIS C 257      -9.615 -29.139  45.514  1.00 24.94      D000 C
ATOM   4229  CG   HIS C 257      -8.842 -27.958  46.023  1.00 29.72      D000 C
ATOM   4230  CD2  HIS C 257      -7.685 -27.880  46.715  1.00 26.19      D000 C
ATOM   4231  ND1  HIS C 257      -9.275 -26.658  45.852  1.00 31.92      D000 N
ATOM   4232  CE1  HIS C 257      -8.411 -25.834  46.415  1.00 33.33      D000 C
ATOM   4233  NE2  HIS C 257      -7.434 -26.546  46.943  1.00 31.64      D000 N
ATOM   4234  N    PHE C 258     -10.146 -28.528  42.441  1.00 25.00      D000 N
ATOM   4235  CA   PHE C 258     -10.340 -27.385  41.567  1.00 28.97      D000 C
ATOM   4236  C    PHE C 258     -10.347 -26.129  42.421  1.00 30.92      D000 C
ATOM   4237  O    PHE C 258     -10.917 -26.128  43.511  1.00 27.46      D000 O
ATOM   4238  CB   PHE C 258     -11.655 -27.491  40.788  1.00 28.67      D000 C
ATOM   4239  CG   PHE C 258     -11.790 -28.752  39.975  1.00 28.57      D000 C
ATOM   4240  CD1  PHE C 258     -11.253 -28.831  38.699  1.00 24.14      D000 C
ATOM   4241  CD2  PHE C 258     -12.475 -29.845  40.477  1.00 26.98      D000 C
ATOM   4242  CE1  PHE C 258     -11.385 -29.982  37.944  1.00 27.83      D000 C
ATOM   4243  CE2  PHE C 258     -12.610 -31.002  39.727  1.00 29.05      D000 C
ATOM   4244  CZ   PHE C 258     -12.064 -31.067  38.458  1.00 26.68      D000 C
ATOM   4245  N    THR C 259      -9.709 -25.068  41.935  1.00 31.52      D000 N
ATOM   4246  CA   THR C 259      -9.778 -23.766  42.596  1.00 27.35      D000 C
ATOM   4247  C    THR C 259     -10.853 -22.908  41.947  1.00 32.37      D000 C
ATOM   4248  O    THR C 259     -11.547 -23.361  41.038  1.00 28.98      D000 O
ATOM   4249  CB   THR C 259      -8.450 -23.013  42.517  1.00 30.50      D000 C
ATOM   4250  CG2  THR C 259      -7.336 -23.832  43.146  1.00 29.47      D000 C
ATOM   4251  OG1  THR C 259      -8.141 -22.759  41.139  1.00 26.60      D000 O
ATOM   4252  N    ASP C 260     -10.966 -21.657  42.383  1.00 33.47      D000 N
ATOM   4253  CA   ASP C 260     -11.989 -20.772  41.841  1.00 32.04      D000 C
ATOM   4254  C    ASP C 260     -11.660 -20.274  40.432  1.00 28.10      D000 C
ATOM   4255  O    ASP C 260     -12.469 -19.571  39.833  1.00 35.94      D000 O
ATOM   4256  CB   ASP C 260     -12.232 -19.574  42.774  1.00 31.62      D000 C
```

```
ATOM   4257  CG  ASP C 260   -10.952 -18.844  43.150  1.00 39.51      D000 C
ATOM   4258  OD1 ASP C 260    -9.894 -19.114  42.544  1.00 46.04      D000 O
ATOM   4259  OD2 ASP C 260   -11.010 -17.986  44.057  1.00 48.87      D000 O
ATOM   4260  N   ASP C 261   -10.494 -20.631  39.890  1.00 32.99      D000 N
ATOM   4261  CA  ASP C 261   -10.219 -20.308  38.483  1.00 33.63      D000 C
ATOM   4262  C   ASP C 261   -10.169 -21.568  37.618  1.00 34.02      D000 C
ATOM   4263  O   ASP C 261    -9.808 -21.517  36.440  1.00 35.61      D000 O
ATOM   4264  CB  ASP C 261    -8.920 -19.493  38.330  1.00 34.41      D000 C
ATOM   4265  CG  ASP C 261    -7.663 -20.257  38.749  1.00 41.30      D000 C
ATOM   4266  OD1 ASP C 261    -7.662 -21.504  38.780  1.00 38.03      D000 O
ATOM   4267  OD2 ASP C 261    -6.644 -19.591  39.031  1.00 47.65      D000 O
ATOM   4268  N   GLY C 262   -10.525 -22.701  38.214  1.00 34.67      D000 N
ATOM   4269  CA  GLY C 262   -10.608 -23.949  37.477  1.00 29.76      D000 C
ATOM   4270  C   GLY C 262    -9.366 -24.810  37.571  1.00 25.05      D000 C
ATOM   4271  O   GLY C 262    -9.449 -26.038  37.513  1.00 31.23      D000 O
ATOM   4272  N   ARG C 263    -8.207 -24.177  37.710  1.00 26.83      D000 N
ATOM   4273  CA  ARG C 263    -6.952 -24.919  37.778  1.00 26.39      D000 C
ATOM   4274  C   ARG C 263    -6.771 -25.578  39.148  1.00 30.19      D000 C
ATOM   4275  O   ARG C 263    -7.392 -25.172  40.135  1.00 28.43      D000 O
ATOM   4276  CB  ARG C 263    -5.775 -24.000  37.443  1.00 27.07      D000 C
ATOM   4277  CG  ARG C 263    -5.652 -23.711  35.943  1.00 29.33      D000 C
ATOM   4278  CD  ARG C 263    -4.595 -22.643  35.649  1.00 33.17      D000 C
ATOM   4279  NE  ARG C 263    -5.071 -21.305  35.982  1.00 44.51      D000 N
ATOM   4280  CZ  ARG C 263    -5.787 -20.547  35.156  1.00 52.96      D000 C
ATOM   4281  NH1 ARG C 263    -6.105 -21.002  33.951  1.00 49.17      D000 N
ATOM   4282  NH2 ARG C 263    -6.188 -19.340  35.532  1.00 51.57      D000 N
ATOM   4283  N   TRP C 264    -5.916 -26.596  39.202  1.00 26.05      D000 N
ATOM   4284  CA  TRP C 264    -5.847 -27.477  40.365  1.00 28.26      D000 C
ATOM   4285  C   TRP C 264    -4.859 -27.000  41.415  1.00 27.92      D000 C
ATOM   4286  O   TRP C 264    -3.844 -26.385  41.097  1.00 28.53      D000 O
ATOM   4287  CB  TRP C 264    -5.460 -28.897  39.938  1.00 24.67      D000 C
ATOM   4288  CG  TRP C 264    -6.268 -29.456  38.814  1.00 25.08      D000 C
ATOM   4289  CD1 TRP C 264    -7.436 -28.958  38.304  1.00 26.48      D000 C
ATOM   4290  CD2 TRP C 264    -5.963 -30.627  38.048  1.00 26.55      D000 C
ATOM   4291  CE2 TRP C 264    -6.994 -30.785  37.098  1.00 22.22      D000 C
ATOM   4292  CE3 TRP C 264    -4.923 -31.565  38.081  1.00 25.16      D000 C
ATOM   4293  NE1 TRP C 264    -7.879 -29.752  37.276  1.00 26.28      D000 N
ATOM   4294  CZ2 TRP C 264    -7.012 -31.837  36.181  1.00 27.60      D000 C
ATOM   4295  CZ3 TRP C 264    -4.944 -32.616  37.170  1.00 22.92      D000 C
ATOM   4296  CH2 TRP C 264    -5.979 -32.739  36.232  1.00 24.94      D000 C
ATOM   4297  N   ASN C 265    -5.155 -27.313  42.670  1.00 30.65      D000 N
ATOM   4298  CA  ASN C 265    -4.243 -27.018  43.762  1.00 29.62      D000 C
ATOM   4299  C   ASN C 265    -4.178 -28.182  44.735  1.00 27.94      D000 C
ATOM   4300  O   ASN C 265    -5.157 -28.904  44.910  1.00 27.71      D000 O
ATOM   4301  CB  ASN C 265    -4.668 -25.746  44.490  1.00 31.63      D000 C
ATOM   4302  CG  ASN C 265    -3.717 -25.373  45.608  1.00 37.30      D000 C
ATOM   4303  ND2 ASN C 265    -4.220 -25.357  46.839  1.00 35.10      D000 N
ATOM   4304  OD1 ASN C 265    -2.541 -25.102  45.367  1.00 39.83      D000 O
ATOM   4305  N   ASP C 266    -3.021 -28.364  45.358  1.00 24.21      D000 N
ATOM   4306  CA  ASP C 266    -2.850 -29.405  46.366  1.00 28.45      D000 C
ATOM   4307  C   ASP C 266    -3.066 -28.832  47.763  1.00 36.19      D000 C
ATOM   4308  O   ASP C 266    -2.397 -27.880  48.157  1.00 35.56      D000 O
ATOM   4309  CB  ASP C 266    -1.463 -30.046  46.253  1.00 25.57      D000 C
ATOM   4310  CG  ASP C 266    -0.349 -29.020  46.030  1.00 36.15      D000 C
ATOM   4311  OD1 ASP C 266    -0.572 -28.006  45.326  1.00 34.08      D000 O
ATOM   4312  OD2 ASP C 266     0.762 -29.236  46.554  1.00 38.58      D000 O
ATOM   4313  N   ASP C 267    -4.007 -29.412  48.507  1.00 32.84      D000 N
ATOM   4314  CA  ASP C 267    -4.386 -28.883  49.817  1.00 30.12      D000 C
ATOM   4315  C   ASP C 267    -4.571 -30.002  50.845  1.00 33.99      D000 C
ATOM   4316  O   ASP C 267    -4.684 -31.181  50.488  1.00 23.96      D000 O
ATOM   4317  CB  ASP C 267    -5.675 -28.058  49.701  1.00 33.91      D000 C
ATOM   4318  CG  ASP C 267    -5.729 -26.898  50.691  1.00 39.18      D000 C
ATOM   4319  OD1 ASP C 267    -4.872 -26.833  51.601  1.00 39.63      D000 O
ATOM   4320  OD2 ASP C 267    -6.640 -26.051  50.560  1.00 41.15      D000 O
ATOM   4321  N   VAL C 268    -4.610 -29.625  52.121  1.00 30.38      D000 N
ATOM   4322  CA  VAL C 268    -4.808 -30.587  53.202  1.00 34.77      D000 C
ATOM   4323  C   VAL C 268    -6.169 -31.260  53.054  1.00 31.59      D000 C
ATOM   4324  O   VAL C 268    -7.157 -30.630  52.685  1.00 30.27      D000 O
ATOM   4325  CB  VAL C 268    -4.683 -29.923  54.596  1.00 36.31      D000 C
ATOM   4326  CG1 VAL C 268    -3.302 -29.294  54.757  1.00 31.26      D000 C
ATOM   4327  CG2 VAL C 268    -5.770 -28.881  54.801  1.00 33.82      D000 C
```

```
ATOM   4328  N    CYS C 269      -6.209 -32.555  53.334  1.00 32.86      D000 N
ATOM   4329  CA   CYS C 269      -7.369 -33.364  52.991  1.00 31.61      D000 C
ATOM   4330  C    CYS C 269      -8.559 -33.098  53.917  1.00 36.95      D000 C
ATOM   4331  O    CYS C 269      -9.684 -33.504  53.627  1.00 36.14      D000 O
ATOM   4332  CB   CYS C 269      -6.984 -34.839  53.009  1.00 44.62      D000 C
ATOM   4333  SG   CYS C 269      -5.637 -35.218  51.830  1.00 60.76      D000 S
ATOM   4334  N    GLN C 270      -8.305 -32.384  55.009  1.00 31.38      D000 N
ATOM   4335  CA   GLN C 270      -9.324 -32.047  55.992  1.00 34.32      D000 C
ATOM   4336  C    GLN C 270     -10.328 -30.995  55.495  1.00 35.28      D000 C
ATOM   4337  O    GLN C 270     -11.434 -30.895  56.022  1.00 38.13      D000 O
ATOM   4338  CB   GLN C 270      -8.640 -31.552  57.265  1.00 41.74      D000 C
ATOM   4339  CG   GLN C 270      -9.526 -31.531  58.491  1.00 57.53      D000 C
ATOM   4340  CD   GLN C 270      -8.755 -31.148  59.743  1.00 62.03      D000 C
ATOM   4341  NE2  GLN C 270      -9.057 -29.975  60.280  1.00 54.32      D000 N
ATOM   4342  OE1  GLN C 270      -7.898 -31.898  60.222  1.00 60.58      D000 O
ATOM   4343  N    ARG C 271      -9.940 -30.207  54.495  1.00 30.92      D000 N
ATOM   4344  CA   ARG C 271     -10.790 -29.124  53.996  1.00 30.72      D000 C
ATOM   4345  C    ARG C 271     -12.159 -29.633  53.533  1.00 38.04      D000 C
ATOM   4346  O    ARG C 271     -12.246 -30.543  52.709  1.00 31.45      D000 O
ATOM   4347  CB   ARG C 271     -10.096 -28.385  52.849  1.00 34.39      D000 C
ATOM   4348  CG   ARG C 271      -8.799 -27.676  53.234  1.00 35.61      D000 C
ATOM   4349  CD   ARG C 271      -9.066 -26.393  53.999  1.00 43.95      D000 C
ATOM   4350  NE   ARG C 271      -7.837 -25.661  54.292  1.00 42.12      D000 N
ATOM   4351  CZ   ARG C 271      -7.169 -25.750  55.438  1.00 45.47      D000 C
ATOM   4352  NH1  ARG C 271      -7.606 -26.547  56.406  1.00 42.46      D000 N
ATOM   4353  NH2  ARG C 271      -6.059 -25.048  55.614  1.00 47.94      D000 N
ATOM   4354  N    PRO C 272     -13.237 -29.045  54.074  1.00 35.02      D000 N
ATOM   4355  CA   PRO C 272     -14.604 -29.446  53.724  1.00 34.48      D000 C
ATOM   4356  C    PRO C 272     -15.064 -28.856  52.392  1.00 34.36      D000 C
ATOM   4357  O    PRO C 272     -16.036 -28.104  52.355  1.00 37.58      D000 O
ATOM   4358  CB   PRO C 272     -15.433 -28.887  54.881  1.00 36.83      D000 C
ATOM   4359  CG   PRO C 272     -14.678 -27.668  55.306  1.00 32.07      D000 C
ATOM   4360  CD   PRO C 272     -13.217 -28.007  55.121  1.00 35.31      D000 C
ATOM   4361  N    TYR C 273     -14.370 -29.197  51.312  1.00 34.00      D000 N
ATOM   4362  CA   TYR C 273     -14.725 -28.690  49.994  1.00 34.47      D000 C
ATOM   4363  C    TYR C 273     -15.886 -29.467  49.399  1.00 31.88      D000 C
ATOM   4364  O    TYR C 273     -16.205 -30.571  49.848  1.00 34.84      D000 O
ATOM   4365  CB   TYR C 273     -13.523 -28.760  49.048  1.00 33.39      D000 C
ATOM   4366  CG   TYR C 273     -12.391 -27.827  49.412  1.00 29.84      D000 C
ATOM   4367  CD1  TYR C 273     -12.634 -26.642  50.096  1.00 31.84      D000 C
ATOM   4368  CD2  TYR C 273     -11.077 -28.131  49.065  1.00 27.54      D000 C
ATOM   4369  CE1  TYR C 273     -11.595 -25.778  50.431  1.00 30.36      D000 C
ATOM   4370  CE2  TYR C 273     -10.036 -27.280  49.391  1.00 31.31      D000 C
ATOM   4371  CZ   TYR C 273     -10.303 -26.104  50.075  1.00 31.94      D000 C
ATOM   4372  OH   TYR C 273      -9.273 -25.261  50.404  1.00 32.60      D000 O
ATOM   4373  N    ARG C 274     -16.510 -28.891  48.377  1.00 29.76      D000 N
ATOM   4374  CA   ARG C 274     -17.470 -29.627  47.566  1.00 28.81      D000 C
ATOM   4375  C    ARG C 274     -16.750 -30.733  46.802  1.00 30.11      D000 C
ATOM   4376  O    ARG C 274     -15.520 -30.772  46.766  1.00 28.45      D000 O
ATOM   4377  CB   ARG C 274     -18.191 -28.689  46.601  1.00 31.26      D000 C
ATOM   4378  CG   ARG C 274     -19.110 -27.703  47.298  1.00 35.55      D000 C
ATOM   4379  CD   ARG C 274     -19.871 -26.844  46.309  1.00 33.61      D000 C
ATOM   4380  NE   ARG C 274     -20.885 -26.046  46.984  1.00 39.40      D000 N
ATOM   4381  CZ   ARG C 274     -21.630 -25.121  46.389  1.00 48.65      D000 C
ATOM   4382  NH1  ARG C 274     -21.474 -24.867  45.097  1.00 48.98      D000 N
ATOM   4383  NH2  ARG C 274     -22.531 -24.446  47.090  1.00 55.24      D000 N
ATOM   4384  N    TRP C 275     -17.507 -31.639  46.198  1.00 28.06      D000 N
ATOM   4385  CA   TRP C 275     -16.887 -32.709  45.432  1.00 28.84      D000 C
ATOM   4386  C    TRP C 275     -17.754 -33.114  44.252  1.00 29.72      D000 C
ATOM   4387  O    TRP C 275     -18.905 -32.693  44.130  1.00 26.78      D000 O
ATOM   4388  CB   TRP C 275     -16.601 -33.927  46.322  1.00 29.81      D000 C
ATOM   4389  CG   TRP C 275     -17.824 -34.715  46.717  1.00 31.96      D000 C
ATOM   4390  CD1  TRP C 275     -18.293 -35.855  46.125  1.00 31.44      D000 C
ATOM   4391  CD2  TRP C 275     -18.726 -34.423  47.795  1.00 29.40      D000 C
ATOM   4392  CE2  TRP C 275     -19.718 -35.426  47.793  1.00 31.93      D000 C
ATOM   4393  CE3  TRP C 275     -18.791 -33.412  48.759  1.00 32.50      D000 C
ATOM   4394  NE1  TRP C 275     -19.430 -36.288  46.767  1.00 36.68      D000 N
ATOM   4395  CZ2  TRP C 275     -20.761 -35.447  48.718  1.00 33.08      D000 C
ATOM   4396  CZ3  TRP C 275     -19.829 -33.433  49.676  1.00 33.96      D000 C
ATOM   4397  CH2  TRP C 275     -20.801 -34.443  49.648  1.00 35.93      D000 C
ATOM   4398  N    VAL C 276     -17.178 -33.926  43.376  1.00 28.10      D000 N
```

```
ATOM   4399  CA   VAL C 276     -17.870 -34.412  42.195  1.00 29.03      D000 C
ATOM   4400  C    VAL C 276     -17.737 -35.924  42.114  1.00 27.19      D000 C
ATOM   4401  O    VAL C 276     -16.628 -36.454  42.177  1.00 28.31      D000 O
ATOM   4402  CB   VAL C 276     -17.302 -33.780  40.905  1.00 28.00      D000 C
ATOM   4403  CG1  VAL C 276     -18.046 -34.293  39.688  1.00 27.41      D000 C
ATOM   4404  CG2  VAL C 276     -17.364 -32.259  40.982  1.00 28.48      D000 C
ATOM   4405  N    CYS C 277     -18.862 -36.619  41.988  1.00 26.93      D000 N
ATOM   4406  CA   CYS C 277     -18.832 -38.058  41.751  1.00 33.14      D000 C
ATOM   4407  C    CYS C 277     -18.994 -38.338  40.262  1.00 37.44      D000 C
ATOM   4408  O    CYS C 277     -19.699 -37.612  39.564  1.00 33.29      D000 O
ATOM   4409  CB   CYS C 277     -19.922 -38.770  42.562  1.00 32.89      D000 C
ATOM   4410  SG   CYS C 277     -19.660 -38.700  44.364  1.00 47.93      D000 S
ATOM   4411  N    GLU C 278     -18.324 -39.386  39.787  1.00 30.10      D000 N
ATOM   4412  CA   GLU C 278     -18.367 -39.774  38.386  1.00 28.53      D000 C
ATOM   4413  C    GLU C 278     -18.528 -41.288  38.252  1.00 32.73      D000 C
ATOM   4414  O    GLU C 278     -17.931 -42.050  39.008  1.00 29.46      D000 O
ATOM   4415  CB   GLU C 278     -17.095 -39.315  37.655  1.00 32.72      D000 C
ATOM   4416  CG   GLU C 278     -16.913 -39.932  36.260  1.00 32.26      D000 C
ATOM   4417  CD   GLU C 278     -15.636 -39.483  35.555  1.00 39.23      D000 C
ATOM   4418  OE1  GLU C 278     -14.646 -39.151  36.241  1.00 36.80      D000 O
ATOM   4419  OE2  GLU C 278     -15.622 -39.463  34.303  1.00 40.68      D000 O
ATOM   4420  N    THR C 279     -19.346 -41.717  37.297  1.00 27.04      D000 N
ATOM   4421  CA   THR C 279     -19.453 -43.132  36.963  1.00 34.21      D000 C
ATOM   4422  C    THR C 279     -19.688 -43.285  35.460  1.00 36.69      D000 C
ATOM   4423  O    THR C 279     -19.848 -42.296  34.746  1.00 39.25      D000 O
ATOM   4424  CB   THR C 279     -20.586 -43.826  37.758  1.00 39.26      D000 C
ATOM   4425  CG2  THR C 279     -21.950 -43.311  37.326  1.00 34.73      D000 C
ATOM   4426  OG1  THR C 279     -20.522 -45.244  37.554  1.00 42.37      D000 O
ATOM   4427  N    GLU C 280     -19.704 -44.522  34.980  1.00 32.85      D000 N
ATOM   4428  CA   GLU C 280     -19.835 -44.782  33.548  1.00 50.68      D000 C
ATOM   4429  C    GLU C 280     -21.239 -45.225  33.156  1.00 52.22      D000 C
ATOM   4430  O    GLU C 280     -22.086 -45.481  34.011  1.00 55.73      D000 O
ATOM   4431  CB   GLU C 280     -18.829 -45.846  33.118  1.00 52.68      D000 C
ATOM   4432  CG   GLU C 280     -17.416 -45.583  33.593  1.00 64.50      D000 C
ATOM   4433  CD   GLU C 280     -16.653 -46.863  33.862  1.00 89.69      D000 C
ATOM   4434  OE1  GLU C 280     -17.283 -47.943  33.861  1.00 93.00      D000 O
ATOM   4435  OE2  GLU C 280     -15.424 -46.790  34.078  1.00104.15      D000 O
ATOM   4436  O    LEU C 281     -22.641 -47.686  29.761  1.00 76.02      D000 O
ATOM   4437  N    LEU C 281     -21.477 -45.319  31.852  1.00 63.81      D000 N
ATOM   4438  CA   LEU C 281     -22.728 -45.870  31.340  1.00 65.22      D000 C
ATOM   4439  C    LEU C 281     -22.565 -47.338  30.941  1.00 72.08      D000 C
ATOM   4440  CB   LEU C 281     -23.228 -45.044  30.158  1.00 59.22      D000 C
ATOM   4441  CG   LEU C 281     -23.879 -43.727  30.578  1.00 63.11      D000 C
ATOM   4442  CD1  LEU C 281     -24.279 -42.891  29.370  1.00 48.41      D000 C
ATOM   4443  CD2  LEU C 281     -25.083 -44.021  31.458  1.00 67.92      D000 C
ATOM   4444  O    ASP C 282     -20.760 -51.176  30.617  1.00 50.93      D000 O
ATOM   4445  N    ASP C 282     -22.342 -48.183  31.947  1.00 77.43      D000 N
ATOM   4446  CA   ASP C 282     -22.184 -49.629  31.778  1.00 80.26      D000 C
ATOM   4447  C    ASP C 282     -21.144 -50.009  30.726  1.00 71.14      D000 C
ATOM   4448  CB   ASP C 282     -23.529 -50.272  31.433  1.00 81.46      D000 C
ATOM   4449  CG   ASP C 282     -24.448 -50.366  32.632  1.00 80.75      D000 C
ATOM   4450  OD1  ASP C 282     -24.417 -49.445  33.476  1.00 77.18      D000 O
ATOM   4451  OD2  ASP C 282     -25.192 -51.364  32.736  1.00 78.02      D000 O
TER
```

## Table 10.3

| ATOM | 1 | N | GLN C | 1 | 12.778 | 87.875 | 6.343 | 1.00 63.46 | N |
|------|-----|-----|---------|-----|---------|---------|---------|------------|-----|
| ATOM | 2 | CA | GLN C | 1 | 12.935 | 86.437 | 6.168 | 1.00 57.97 | C |
| ATOM | 3 | C | GLN C | 1 | 11.798 | 85.716 | 6.845 | 1.00 55.78 | C |
| ATOM | 4 | O | GLN C | 1 | 11.088 | 86.308 | 7.656 | 1.00 58.48 | O |
| ATOM | 5 | CB | GLN C | 1 | 14.271 | 85.946 | 6.714 | 1.00 51.88 | C |
| ATOM | 6 | CG | GLN C | 1 | 14.814 | 84.739 | 5.974 | 1.00 59.79 | C |
| ATOM | 7 | CD | GLN C | 1 | 14.717 | 84.869 | 4.445 | 1.00 78.61 | C |
| ATOM | 8 | OE1 | GLN C | 1 | 14.676 | 85.980 | 3.896 | 1.00 61.52 | O |
| ATOM | 9 | NE2 | GLN C | 1 | 14.669 | 83.721 | 3.753 | 1.00 84.10 | N |
| ATOM | 10 | N | VAL C | 2 | 11.615 | 84.445 | 6.497 | 1.00 50.14 | N |
| ATOM | 11 | CA | VAL C | 2 | 10.404 | 83.710 | 6.828 | 1.00 46.16 | C |
| ATOM | 12 | C | VAL C | 2 | 10.800 | 82.363 | 7.396 | 1.00 45.45 | C |
| ATOM | 13 | O | VAL C | 2 | 11.642 | 81.667 | 6.822 | 1.00 49.37 | O |
| ATOM | 14 | CB | VAL C | 2 | 9.493 | 83.521 | 5.596 | 1.00 45.22 | C |
| ATOM | 15 | CG1 | VAL C | 2 | 8.322 | 82.567 | 5.911 | 1.00 45.47 | C |
| ATOM | 16 | CG2 | VAL C | 2 | 8.965 | 84.848 | 5.109 | 1.00 40.64 | C |
| ATOM | 17 | N | GLN C | 3 | 10.163 | 81.983 | 8.498 | 1.00 50.52 | N |
| ATOM | 18 | CA | GLN C | 3 | 10.262 | 80.648 | 9.066 | 1.00 49.86 | C |
| ATOM | 19 | C | GLN C | 3 | 8.856 | 80.074 | 9.158 | 1.00 45.53 | C |
| ATOM | 20 | O | GLN C | 3 | 7.924 | 80.766 | 9.596 | 1.00 40.46 | O |
| ATOM | 21 | CB | GLN C | 3 | 10.933 | 80.674 | 10.449 | 1.00 52.58 | C |
| ATOM | 22 | CG | GLN C | 3 | 12.476 | 80.689 | 10.448 | 1.00 67.48 | C |
| ATOM | 23 | CD | GLN C | 3 | 13.103 | 82.051 | 10.135 | 1.00 74.06 | C |
| ATOM | 24 | OE1 | GLN C | 3 | 12.444 | 83.102 | 10.186 | 1.00 75.22 | O |
| ATOM | 25 | NE2 | GLN C | 3 | 14.394 | 82.033 | 9.811 | 1.00 80.56 | N |
| ATOM | 26 | N | LEU C | 4 | 8.702 | 78.828 | 8.714 | 1.00 36.58 | N |
| ATOM | 27 | CA | LEU C | 4 | 7.451 | 78.093 | 8.811 | 1.00 35.24 | C |
| ATOM | 28 | C | LEU C | 4 | 7.674 | 76.836 | 9.634 | 1.00 35.32 | C |
| ATOM | 29 | O | LEU C | 4 | 8.563 | 76.043 | 9.319 | 1.00 34.10 | O |
| ATOM | 30 | CB | LEU C | 4 | 6.925 | 77.728 | 7.417 | 1.00 35.59 | C |
| ATOM | 31 | CG | LEU C | 4 | 6.747 | 78.896 | 6.452 | 1.00 38.97 | C |
| ATOM | 32 | CD1 | LEU C | 4 | 6.422 | 78.423 | 5.045 | 1.00 39.74 | C |
| ATOM | 33 | CD2 | LEU C | 4 | 5.653 | 79.806 | 6.970 | 1.00 42.54 | C |
| ATOM | 34 | N | VAL C | 5 | 6.835 | 76.623 | 10.647 | 1.00 40.31 | N |
| ATOM | 35 | CA | VAL C | 5 | 6.970 | 75.492 | 11.568 | 1.00 38.30 | C |
| ATOM | 36 | C | VAL C | 5 | 5.625 | 74.776 | 11.675 | 1.00 41.55 | C |
| ATOM | 37 | O | VAL C | 5 | 4.663 | 75.325 | 12.232 | 1.00 47.07 | O |
| ATOM | 38 | CB | VAL C | 5 | 7.451 | 75.939 | 12.956 | 1.00 35.88 | C |
| ATOM | 39 | CG1 | VAL C | 5 | 7.568 | 74.743 | 13.871 | 1.00 36.28 | C |
| ATOM | 40 | CG2 | VAL C | 5 | 8.763 | 76.660 | 12.847 | 1.00 26.29 | C |
| ATOM | 41 | N | GLU C | 6 | 5.558 | 73.556 | 11.169 | 1.00 42.37 | N |
| ATOM | 42 | CA | GLU C | 6 | 4.354 | 72.741 | 11.241 | 1.00 43.70 | C |
| ATOM | 43 | C | GLU C | 6 | 4.281 | 71.970 | 12.559 | 1.00 43.98 | C |
| ATOM | 44 | O | GLU C | 6 | 5.290 | 71.677 | 13.196 | 1.00 49.49 | O |
| ATOM | 45 | CB | GLU C | 6 | 4.290 | 71.749 | 10.081 | 1.00 44.93 | C |
| ATOM | 46 | CG | GLU C | 6 | 4.481 | 72.363 | 8.699 | 1.00 44.29 | C |
| ATOM | 47 | CD | GLU C | 6 | 5.949 | 72.419 | 8.267 | 1.00 44.27 | C |
| ATOM | 48 | OE1 | GLU C | 6 | 6.215 | 72.317 | 7.041 | 1.00 42.60 | O |
| ATOM | 49 | OE2 | GLU C | 6 | 6.832 | 72.490 | 9.153 | 1.00 47.85 | O1- |
| ATOM | 50 | N | SER C | 7 | 3.061 | 71.619 | 12.950 | 1.00 48.19 | N |
| ATOM | 51 | CA | SER C | 7 | 2.851 | 70.788 | 14.128 | 1.00 51.74 | C |
| ATOM | 52 | C | SER C | 7 | 1.509 | 70.084 | 13.994 | 1.00 45.51 | C |
| ATOM | 53 | O | SER C | 7 | 0.681 | 70.437 | 13.151 | 1.00 43.94 | O |
| ATOM | 54 | CB | SER C | 7 | 2.904 | 71.620 | 15.410 | 1.00 38.38 | C |
| ATOM | 55 | OG | SER C | 7 | 1.907 | 72.623 | 15.354 | 1.00 49.56 | O |
| ATOM | 56 | N | GLY C | 8 | 1.310 | 69.067 | 14.829 | 1.00 48.93 | N |
| ATOM | 57 | CA | GLY C | 8 | 0.030 | 68.405 | 14.930 | 1.00 34.09 | C |
| ATOM | 58 | C | GLY C | 8 | -0.065 | 67.069 | 14.246 | 1.00 43.11 | C |
| ATOM | 59 | O | GLY C | 8 | -1.148 | 66.466 | 14.255 | 1.00 46.79 | O |
| ATOM | 60 | N | GLY C | 9 | 1.015 | 66.567 | 13.666 | 1.00 37.58 | N |
| ATOM | 61 | CA | GLY C | 9 | 0.947 | 65.229 | 13.116 | 1.00 47.86 | C |
| ATOM | 62 | C | GLY C | 9 | 0.747 | 64.199 | 14.226 | 1.00 51.47 | C |
| ATOM | 63 | O | GLY C | 9 | 0.716 | 64.509 | 15.419 | 1.00 54.50 | O |
| ATOM | 64 | N | GLY C | 10 | 0.731 | 62.943 | 13.833 | 1.00 46.74 | N |
| ATOM | 65 | CA | GLY C | 10 | 0.618 | 61.871 | 14.804 | 1.00 46.77 | C |
| ATOM | 66 | C | GLY C | 10 | -0.035 | 60.648 | 14.193 | 1.00 52.50 | C |
| ATOM | 67 | O | GLY C | 10 | -0.310 | 60.586 | 12.994 | 1.00 47.51 | O |
| ATOM | 68 | N | VAL C | 11 | -0.256 | 59.651 | 15.049 | 1.00 51.48 | N |
| ATOM | 69 | CA | VAL C | 11 | -0.832 | 58.380 | 14.625 | 1.00 53.97 | C |

| | | | | | | | | | | |
|---|---|---|---|---|---|---|---|---|---|---|
| ATOM | 70 | C | VAL | C | 11 | -2.320 | 58.434 | 14.900 | 1.00 52.43 | C |
| ATOM | 71 | O | VAL | C | 11 | -2.739 | 58.881 | 15.975 | 1.00 50.83 | O |
| ATOM | 72 | CB | VAL | C | 11 | -0.183 | 57.184 | 15.345 | 1.00 43.91 | C |
| ATOM | 73 | CG1 | VAL | C | 11 | -0.742 | 55.891 | 14.787 | 1.00 43.96 | C |
| ATOM | 74 | CG2 | VAL | C | 11 | 1.317 | 57.226 | 15.201 | 1.00 43.94 | C |
| ATOM | 75 | N | VAL | C | 12 | -3.117 | 58.015 | 13.916 | 1.00 45.99 | N |
| ATOM | 76 | CA | VAL | C | 12 | -4.570 | 58.072 | 13.996 | 1.00 46.88 | C |
| ATOM | 77 | C | VAL | C | 12 | -5.115 | 56.950 | 13.139 | 1.00 46.73 | C |
| ATOM | 78 | O | VAL | C | 12 | -4.469 | 56.494 | 12.191 | 1.00 47.91 | O |
| ATOM | 79 | CB | VAL | C | 12 | -5.179 | 59.425 | 13.540 | 1.00 47.29 | C |
| ATOM | 80 | CG1 | VAL | C | 12 | -4.763 | 60.547 | 14.468 | 1.00 46.14 | C |
| ATOM | 81 | CG2 | VAL | C | 12 | -4.755 | 59.739 | 12.130 | 1.00 48.50 | C |
| ATOM | 82 | N | GLN | C | 13 | -6.316 | 56.547 | 13.443 | 1.00 51.98 | N |
| ATOM | 83 | CA | GLN | C | 13 | -7.001 | 55.451 | 12.787 | 1.00 48.11 | C |
| ATOM | 84 | C | GLN | C | 13 | -7.748 | 55.950 | 11.559 | 1.00 42.02 | C |
| ATOM | 85 | O | GLN | C | 13 | -8.237 | 57.082 | 11.548 | 1.00 45.22 | O |
| ATOM | 86 | CB | GLN | C | 13 | -8.005 | 54.841 | 13.740 | 1.00 57.37 | C |
| ATOM | 87 | CG | GLN | C | 13 | -7.409 | 54.422 | 15.035 | 1.00 67.65 | C |
| ATOM | 88 | CD | GLN | C | 13 | -8.286 | 53.439 | 15.730 | 1.00 79.97 | C |
| ATOM | 89 | OE1 | GLN | C | 13 | -8.845 | 53.737 | 16.788 | 1.00 93.60 | O |
| ATOM | 90 | NE2 | GLN | C | 13 | -8.446 | 52.258 | 15.130 | 1.00 91.83 | N |
| ATOM | 91 | N | PRO | C | 14 | -7.878 | 55.101 | 10.541 | 1.00 38.64 | N |
| ATOM | 92 | CA | PRO | C | 14 | -8.652 | 55.475 | 9.351 | 1.00 37.32 | C |
| ATOM | 93 | C | PRO | C | 14 | -10.050 | 55.954 | 9.725 | 1.00 48.06 | C |
| ATOM | 94 | O | PRO | C | 14 | -10.688 | 55.419 | 10.634 | 1.00 50.66 | O |
| ATOM | 95 | CB | PRO | C | 14 | -8.711 | 54.174 | 8.539 | 1.00 35.18 | C |
| ATOM | 96 | CG | PRO | C | 14 | -7.556 | 53.370 | 9.005 | 1.00 40.76 | C |
| ATOM | 97 | CD | PRO | C | 14 | -7.351 | 53.727 | 10.456 | 1.00 40.72 | C |
| ATOM | 98 | N | GLY | C | 15 | -10.525 | 56.982 | 9.023 | 1.00 45.47 | N |
| ATOM | 99 | CA | GLY | C | 15 | -11.836 | 57.526 | 9.264 | 1.00 39.13 | C |
| ATOM | 100 | C | GLY | C | 15 | -11.886 | 58.622 | 10.303 | 1.00 40.65 | C |
| ATOM | 101 | O | GLY | C | 15 | -12.887 | 59.331 | 10.374 | 1.00 51.10 | O |
| ATOM | 102 | N | ARG | C | 16 | -10.842 | 58.781 | 11.109 | 1.00 42.12 | N |
| ATOM | 103 | CA | ARG | C | 16 | -10.775 | 59.854 | 12.087 | 1.00 42.11 | C |
| ATOM | 104 | C | ARG | C | 16 | -10.275 | 61.158 | 11.450 | 1.00 49.72 | C |
| ATOM | 105 | O | ARG | C | 16 | -10.040 | 61.255 | 10.237 | 1.00 41.14 | O |
| ATOM | 106 | CB | ARG | C | 16 | -9.865 | 59.456 | 13.243 | 1.00 48.99 | C |
| ATOM | 107 | CG | ARG | C | 16 | -10.287 | 58.203 | 13.961 | 1.00 58.16 | C |
| ATOM | 108 | CD | ARG | C | 16 | -11.621 | 58.360 | 14.645 | 1.00 60.17 | C |
| ATOM | 109 | NE | ARG | C | 16 | -11.592 | 57.775 | 15.983 | 1.00 78.30 | N |
| ATOM | 110 | CZ | ARG | C | 16 | -12.669 | 57.597 | 16.743 | 1.00 90.64 | C |
| ATOM | 111 | NH1 | ARG | C | 16 | -13.866 | 57.952 | 16.295 | 1.00 92.41 | N1+ |
| ATOM | 112 | NH2 | ARG | C | 16 | -12.552 | 57.058 | 17.950 | 1.00 96.43 | N |
| ATOM | 113 | N | SER | C | 17 | -10.093 | 62.174 | 12.299 | 1.00 44.14 | N |
| ATOM | 114 | CA | SER | C | 17 | -9.731 | 63.516 | 11.876 | 1.00 45.83 | C |
| ATOM | 115 | C | SER | C | 17 | -8.490 | 63.998 | 12.604 | 1.00 44.89 | C |
| ATOM | 116 | O | SER | C | 17 | -8.207 | 63.600 | 13.738 | 1.00 51.51 | O |
| ATOM | 117 | CB | SER | C | 17 | -10.853 | 64.535 | 12.132 | 1.00 45.46 | C |
| ATOM | 118 | OG | SER | C | 17 | -11.938 | 64.263 | 11.274 | 1.00 59.74 | O |
| ATOM | 119 | N | LEU | C | 18 | -7.817 | 64.947 | 11.961 | 1.00 41.62 | N |
| ATOM | 120 | CA | LEU | C | 18 | -6.610 | 65.573 | 12.473 | 1.00 44.03 | C |
| ATOM | 121 | C | LEU | C | 18 | -6.536 | 66.971 | 11.886 | 1.00 41.33 | C |
| ATOM | 122 | O | LEU | C | 18 | -6.961 | 67.203 | 10.756 | 1.00 44.87 | O |
| ATOM | 123 | CB | LEU | C | 18 | -5.366 | 64.772 | 12.079 | 1.00 41.97 | C |
| ATOM | 124 | CG | LEU | C | 18 | -4.114 | 64.794 | 12.940 | 1.00 54.29 | C |
| ATOM | 125 | CD1 | LEU | C | 18 | -4.440 | 64.407 | 14.380 | 1.00 50.61 | C |
| ATOM | 126 | CD2 | LEU | C | 18 | -3.075 | 63.836 | 12.334 | 1.00 49.38 | C |
| ATOM | 127 | N | ARG | C | 19 | -6.000 | 67.902 | 12.654 | 1.00 39.81 | N |
| ATOM | 128 | CA | ARG | C | 19 | -5.810 | 69.262 | 12.189 | 1.00 40.64 | C |
| ATOM | 129 | C | ARG | C | 19 | -4.334 | 69.605 | 12.283 | 1.00 48.86 | C |
| ATOM | 130 | O | ARG | C | 19 | -3.778 | 69.652 | 13.386 | 1.00 48.74 | O |
| ATOM | 131 | CB | ARG | C | 19 | -6.650 | 70.222 | 13.021 | 1.00 41.19 | C |
| ATOM | 132 | CG | ARG | C | 19 | -6.806 | 71.632 | 12.452 | 1.00 46.04 | C |
| ATOM | 133 | CD | ARG | C | 19 | -6.323 | 72.509 | 13.554 | 1.00 49.64 | C |
| ATOM | 134 | NE | ARG | C | 19 | -7.182 | 73.633 | 13.872 | 1.00 52.33 | N |
| ATOM | 135 | CZ | ARG | C | 19 | -6.947 | 74.447 | 14.900 | 1.00 60.69 | C |
| ATOM | 136 | NH1 | ARG | C | 19 | -5.914 | 74.205 | 15.702 | 1.00 60.16 | N1+ |
| ATOM | 137 | NH2 | ARG | C | 19 | -7.741 | 75.487 | 15.142 | 1.00 61.67 | N |
| ATOM | 138 | N | LEU | C | 20 | -3.716 | 69.890 | 11.137 | 1.00 44.47 | N |
| ATOM | 139 | CA | LEU | C | 20 | -2.328 | 70.331 | 11.121 | 1.00 42.17 | C |
| ATOM | 140 | C | LEU | C | 20 | -2.270 | 71.840 | 11.202 | 1.00 40.77 | C |

| | | | | | | | | | | | |
|---|---|---|---|---|---|---|---|---|---|---|---|
| ATOM | 141 | O | LEU C | 20 | -3.145 | 72.544 | 10.691 | 1.00 | 39.38 | | O |
| ATOM | 142 | CB | LEU C | 20 | -1.591 | 69.860 | 9.864 | 1.00 | 32.38 | | C |
| ATOM | 143 | CG | LEU C | 20 | -1.658 | 68.360 | 9.649 | 1.00 | 39.39 | | C |
| ATOM | 144 | CD1 | LEU C | 20 | -0.808 | 67.907 | 8.483 | 1.00 | 38.47 | | C |
| ATOM | 145 | CD2 | LEU C | 20 | -1.266 | 67.633 | 10.930 | 1.00 | 41.15 | | C |
| ATOM | 146 | N | SER C | 21 | -1.221 | 72.328 | 11.845 | 1.00 | 39.02 | | N |
| ATOM | 147 | CA | SER C | 21 | -0.951 | 73.748 | 11.950 | 1.00 | 40.69 | | C |
| ATOM | 148 | C | SER C | 21 | 0.423 | 74.053 | 11.372 | 1.00 | 47.84 | | C |
| ATOM | 149 | O | SER C | 21 | 1.339 | 73.221 | 11.416 | 1.00 | 45.59 | | O |
| ATOM | 150 | CB | SER C | 21 | -1.011 | 74.211 | 13.397 | 1.00 | 38.57 | | C |
| ATOM | 151 | OG | SER C | 21 | -2.330 | 74.127 | 13.871 | 1.00 | 56.62 | | O |
| ATOM | 152 | N | CYS C | 22 | 0.546 | 75.263 | 10.834 | 1.00 | 38.99 | | N |
| ATOM | 153 | CA | CYS C | 22 | 1.795 | 75.794 | 10.299 | 1.00 | 44.99 | | C |
| ATOM | 154 | C | CYS C | 22 | 1.951 | 77.220 | 10.827 | 1.00 | 46.04 | | C |
| ATOM | 155 | O | CYS C | 22 | 1.172 | 78.113 | 10.465 | 1.00 | 41.82 | | O |
| ATOM | 156 | CB | CYS C | 22 | 1.788 | 75.761 | 8.768 | 1.00 | 44.73 | | C |
| ATOM | 157 | SG | CYS C | 22 | 3.139 | 76.705 | 7.941 | 1.00 | 52.54 | | S |
| ATOM | 158 | N | ALA C | 23 | 2.921 | 77.417 | 11.717 | 1.00 | 39.89 | | N |
| ATOM | 159 | CA | ALA C | 23 | 3.158 | 78.694 | 12.375 | 1.00 | 39.98 | | C |
| ATOM | 160 | C | ALA C | 23 | 4.271 | 79.454 | 11.659 | 1.00 | 41.32 | | C |
| ATOM | 161 | O | ALA C | 23 | 5.359 | 78.912 | 11.428 | 1.00 | 44.39 | | O |
| ATOM | 162 | CB | ALA C | 23 | 3.522 | 78.488 | 13.850 | 1.00 | 35.18 | | C |
| ATOM | 163 | N | ALA C | 24 | 3.999 | 80.703 | 11.327 | 1.00 | 39.85 | | N |
| ATOM | 164 | CA | ALA C | 24 | 4.895 | 81.524 | 10.537 | 1.00 | 43.27 | | C |
| ATOM | 165 | C | ALA C | 24 | 5.477 | 82.657 | 11.372 | 1.00 | 46.72 | | C |
| ATOM | 166 | O | ALA C | 24 | 4.801 | 83.237 | 12.220 | 1.00 | 48.85 | | O |
| ATOM | 167 | CB | ALA C | 24 | 4.157 | 82.110 | 9.339 | 1.00 | 38.90 | | C |
| ATOM | 168 | N | SER C | 25 | 6.720 | 83.018 | 11.076 | 1.00 | 44.32 | | N |
| ATOM | 169 | CA | SER C | 25 | 7.328 | 84.177 | 11.710 | 1.00 | 46.12 | | C |
| ATOM | 170 | C | SER C | 25 | 8.226 | 84.874 | 10.695 | 1.00 | 44.36 | | C |
| ATOM | 171 | O | SER C | 25 | 8.623 | 84.304 | 9.673 | 1.00 | 43.53 | | O |
| ATOM | 172 | CB | SER C | 25 | 8.122 | 83.783 | 12.966 | 1.00 | 46.62 | | C |
| ATOM | 173 | OG | SER C | 25 | 9.191 | 82.915 | 12.642 | 1.00 | 42.94 | | O |
| ATOM | 174 | N | GLY C | 26 | 8.563 | 86.112 | 11.001 | 1.00 | 46.40 | | N |
| ATOM | 175 | CA | GLY C | 26 | 9.394 | 86.915 | 10.124 | 1.00 | 42.45 | | C |
| ATOM | 176 | C | GLY C | 26 | 8.527 | 87.884 | 9.326 | 1.00 | 52.00 | | C |
| ATOM | 177 | O | GLY C | 26 | 7.673 | 88.572 | 9.891 | 1.00 | 48.03 | | O |
| ATOM | 178 | N | PHE C | 27 | 8.729 | 87.910 | 8.013 | 1.00 | 49.49 | | N |
| ATOM | 179 | CA | PHE C | 27 | 7.986 | 88.820 | 7.151 | 1.00 | 45.04 | | C |
| ATOM | 180 | C | PHE C | 27 | 6.479 | 88.576 | 7.273 | 1.00 | 44.14 | | C |
| ATOM | 181 | O | PHE C | 27 | 6.025 | 87.430 | 7.293 | 1.00 | 47.90 | | O |
| ATOM | 182 | CB | PHE C | 27 | 8.449 | 88.611 | 5.712 | 1.00 | 45.76 | | C |
| ATOM | 183 | CG | PHE C | 27 | 7.704 | 89.421 | 4.711 | 1.00 | 48.04 | | C |
| ATOM | 184 | CD1 | PHE C | 27 | 7.470 | 90.769 | 4.937 | 1.00 | 50.27 | | C |
| ATOM | 185 | CD2 | PHE C | 27 | 7.236 | 88.838 | 3.539 | 1.00 | 49.31 | | C |
| ATOM | 186 | CE1 | PHE C | 27 | 6.784 | 91.537 | 4.011 | 1.00 | 53.69 | | C |
| ATOM | 187 | CE2 | PHE C | 27 | 6.550 | 89.591 | 2.606 | 1.00 | 47.82 | | C |
| ATOM | 188 | CZ | PHE C | 27 | 6.320 | 90.951 | 2.846 | 1.00 | 49.54 | | C |
| ATOM | 189 | N | THR C | 28 | 5.709 | 89.671 | 7.335 | 1.00 | 44.86 | | N |
| ATOM | 190 | CA | THR C | 28 | 4.261 | 89.693 | 7.577 | 1.00 | 38.38 | | C |
| ATOM | 191 | C | THR C | 28 | 3.475 | 88.544 | 6.941 | 1.00 | 42.91 | | C |
| ATOM | 192 | O | THR C | 28 | 3.343 | 88.483 | 5.711 | 1.00 | 36.78 | | O |
| ATOM | 193 | CB | THR C | 28 | 3.679 | 90.998 | 7.038 | 1.00 | 49.68 | | C |
| ATOM | 194 | OG1 | THR C | 28 | 4.434 | 92.106 | 7.536 | 1.00 | 46.23 | | O |
| ATOM | 195 | CG2 | THR C | 28 | 2.208 | 91.133 | 7.410 | 1.00 | 44.54 | | C |
| ATOM | 196 | N | PHE C | 29 | 2.891 | 87.687 | 7.790 | 1.00 | 38.44 | | N |
| ATOM | 197 | CA | PHE C | 29 | 2.154 | 86.502 | 7.350 | 1.00 | 40.01 | | C |
| ATOM | 198 | C | PHE C | 29 | 1.089 | 86.822 | 6.304 | 1.00 | 36.82 | | C |
| ATOM | 199 | O | PHE C | 29 | 0.942 | 86.092 | 5.316 | 1.00 | 36.46 | | O |
| ATOM | 200 | CB | PHE C | 29 | 1.508 | 85.858 | 8.571 | 1.00 | 39.47 | | C |
| ATOM | 201 | CG | PHE C | 29 | 0.772 | 84.586 | 8.296 | 1.00 | 41.07 | | C |
| ATOM | 202 | CD1 | PHE C | 29 | 1.443 | 83.470 | 7.809 | 1.00 | 39.25 | | C |
| ATOM | 203 | CD2 | PHE C | 29 | -0.586 | 84.484 | 8.581 | 1.00 | 37.61 | | C |
| ATOM | 204 | CE1 | PHE C | 29 | 0.782 | 82.271 | 7.600 | 1.00 | 37.53 | | C |
| ATOM | 205 | CE2 | PHE C | 29 | -1.260 | 83.280 | 8.380 | 1.00 | 42.81 | | C |
| ATOM | 206 | CZ | PHE C | 29 | -0.575 | 82.170 | 7.878 | 1.00 | 41.35 | | C |
| ATOM | 207 | N | SER C | 30 | 0.352 | 87.917 | 6.489 | 1.00 | 34.06 | | N |
| ATOM | 208 | CA | SER C | 30 | -0.719 | 88.270 | 5.564 | 1.00 | 37.45 | | C |
| ATOM | 209 | C | SER C | 30 | -0.200 | 88.707 | 4.203 | 1.00 | 39.61 | | C |
| ATOM | 210 | O | SER C | 30 | -1.018 | 88.991 | 3.319 | 1.00 | 35.46 | | O |
| ATOM | 211 | CB | SER C | 30 | -1.587 | 89.392 | 6.141 | 1.00 | 31.23 | | C |

271

| ATOM | 212 | OG | SER C | 30 | -0.804 | 90.566 | 6.341 | 1.00 38.68 | O |
| ATOM | 213 | N | SER C | 31 | 1.115 | 88.752 | 3.998 | 1.00 31.95 | N |
| ATOM | 214 | CA | SER C | 31 | 1.653 | 89.144 | 2.705 | 1.00 42.10 | C |
| ATOM | 215 | C | SER C | 31 | 1.858 | 87.979 | 1.743 | 1.00 36.93 | C |
| ATOM | 216 | O | SER C | 31 | 2.347 | 88.199 | 0.629 | 1.00 40.45 | O |
| ATOM | 217 | CB | SER C | 31 | 2.975 | 89.887 | 2.883 | 1.00 38.00 | C |
| ATOM | 218 | OG | SER C | 31 | 2.725 | 91.165 | 3.425 | 1.00 50.55 | O |
| ATOM | 219 | N | TYR C | 32 | 1.476 | 86.759 | 2.103 | 1.00 32.77 | N |
| ATOM | 220 | CA | TYR C | 32 | 1.671 | 85.678 | 1.145 | 1.00 37.31 | C |
| ATOM | 221 | C | TYR C | 32 | 0.613 | 84.597 | 1.312 | 1.00 35.54 | C |
| ATOM | 222 | O | TYR C | 32 | 0.059 | 84.403 | 2.401 | 1.00 33.81 | O |
| ATOM | 223 | CB | TYR C | 32 | 3.083 | 85.087 | 1.282 | 1.00 33.59 | C |
| ATOM | 224 | CG | TYR C | 32 | 3.516 | 84.779 | 2.701 | 1.00 29.77 | C |
| ATOM | 225 | CD1 | TYR C | 32 | 3.130 | 83.599 | 3.315 | 1.00 33.82 | C |
| ATOM | 226 | CD2 | TYR C | 32 | 4.353 | 85.643 | 3.405 | 1.00 38.18 | C |
| ATOM | 227 | CE1 | TYR C | 32 | 3.550 | 83.271 | 4.603 | 1.00 38.72 | C |
| ATOM | 228 | CE2 | TYR C | 32 | 4.781 | 85.340 | 4.703 | 1.00 39.15 | C |
| ATOM | 229 | CZ | TYR C | 32 | 4.365 | 84.145 | 5.296 | 1.00 45.73 | C |
| ATOM | 230 | OH | TYR C | 32 | 4.741 | 83.816 | 6.579 | 1.00 41.78 | O |
| ATOM | 231 | N | GLY C | 33 | 0.344 | 83.893 | 0.209 | 1.00 34.05 | N |
| ATOM | 232 | CA | GLY C | 33 | -0.428 | 82.670 | 0.260 | 1.00 32.21 | C |
| ATOM | 233 | C | GLY C | 33 | 0.446 | 81.497 | 0.679 | 1.00 39.56 | C |
| ATOM | 234 | O | GLY C | 33 | 1.665 | 81.640 | 0.823 | 1.00 32.33 | O |
| ATOM | 235 | N | LEU C | 34 | -0.196 | 80.340 | 0.907 | 1.00 32.53 | N |
| ATOM | 236 | CA | LEU C | 34 | 0.521 | 79.169 | 1.401 | 1.00 34.46 | C |
| ATOM | 237 | C | LEU C | 34 | -0.060 | 77.886 | 0.824 | 1.00 30.24 | C |
| ATOM | 238 | O | LEU C | 34 | -1.200 | 77.841 | 0.360 | 1.00 33.75 | O |
| ATOM | 239 | CB | LEU C | 34 | 0.515 | 79.085 | 2.933 | 1.00 38.99 | C |
| ATOM | 240 | CG | LEU C | 34 | 1.236 | 80.236 | 3.655 | 1.00 41.08 | C |
| ATOM | 241 | CD1 | LEU C | 34 | 0.223 | 81.245 | 4.217 | 1.00 33.75 | C |
| ATOM | 242 | CD2 | LEU C | 34 | 2.165 | 79.704 | 4.725 | 1.00 35.97 | C |
| ATOM | 243 | N | HIS C | 35 | 0.768 | 76.846 | 0.863 | 1.00 31.72 | N |
| ATOM | 244 | CA | HIS C | 35 | 0.503 | 75.507 | 0.371 | 1.00 29.96 | C |
| ATOM | 245 | C | HIS C | 35 | 0.618 | 74.498 | 1.498 | 1.00 33.37 | C |
| ATOM | 246 | O | HIS C | 35 | 1.323 | 74.717 | 2.484 | 1.00 34.65 | O |
| ATOM | 247 | CB | HIS C | 35 | 1.534 | 75.064 | -0.671 | 1.00 31.82 | C |
| ATOM | 248 | CG | HIS C | 35 | 1.514 | 75.835 | -1.949 | 1.00 38.58 | C |
| ATOM | 249 | ND1 | HIS C | 35 | 0.781 | 75.435 | -3.047 | 1.00 31.99 | N |
| ATOM | 250 | CD2 | HIS C | 35 | 2.200 | 76.940 | -2.333 | 1.00 34.19 | C |
| ATOM | 251 | CE1 | HIS C | 35 | 1.008 | 76.269 | -4.046 | 1.00 28.49 | C |
| ATOM | 252 | NE2 | HIS C | 35 | 1.855 | 77.197 | -3.635 | 1.00 33.84 | N |
| ATOM | 253 | N | TRP C | 36 | -0.010 | 73.347 | 1.298 | 1.00 29.55 | N |
| ATOM | 254 | CA | TRP C | 36 | 0.381 | 72.110 | 1.957 | 1.00 30.04 | C |
| ATOM | 255 | C | TRP C | 36 | 0.870 | 71.151 | 0.887 | 1.00 32.99 | C |
| ATOM | 256 | O | TRP C | 36 | 0.217 | 70.995 | -0.149 | 1.00 33.39 | O |
| ATOM | 257 | CB | TRP C | 36 | -0.771 | 71.490 | 2.735 | 1.00 30.51 | C |
| ATOM | 258 | CG | TRP C | 36 | -1.084 | 72.233 | 3.977 | 1.00 36.65 | C |
| ATOM | 259 | CD1 | TRP C | 36 | -2.063 | 73.191 | 4.152 | 1.00 36.08 | C |
| ATOM | 260 | CD2 | TRP C | 36 | -0.447 | 72.073 | 5.246 | 1.00 33.16 | C |
| ATOM | 261 | NE1 | TRP C | 36 | -2.055 | 73.637 | 5.456 | 1.00 34.80 | N |
| ATOM | 262 | CE2 | TRP C | 36 | -1.073 | 72.972 | 6.146 | 1.00 31.89 | C |
| ATOM | 263 | CE3 | TRP C | 36 | 0.602 | 71.272 | 5.706 | 1.00 33.27 | C |
| ATOM | 264 | CZ2 | TRP C | 36 | -0.690 | 73.079 | 7.475 | 1.00 35.29 | C |
| ATOM | 265 | CZ3 | TRP C | 36 | 0.976 | 71.373 | 7.022 | 1.00 36.31 | C |
| ATOM | 266 | CH2 | TRP C | 36 | 0.334 | 72.276 | 7.898 | 1.00 37.43 | C |
| ATOM | 267 | N | VAL C | 37 | 2.037 | 70.544 | 1.114 | 1.00 31.46 | N |
| ATOM | 268 | CA | VAL C | 37 | 2.588 | 69.540 | 0.215 | 1.00 28.53 | C |
| ATOM | 269 | C | VAL C | 37 | 2.937 | 68.334 | 1.066 | 1.00 37.43 | C |
| ATOM | 270 | O | VAL C | 37 | 3.354 | 68.473 | 2.221 | 1.00 38.22 | O |
| ATOM | 271 | CB | VAL C | 37 | 3.832 | 70.056 | -0.554 | 1.00 32.46 | C |
| ATOM | 272 | CG1 | VAL C | 37 | 4.424 | 68.959 | -1.463 | 1.00 28.26 | C |
| ATOM | 273 | CG2 | VAL C | 37 | 3.501 | 71.313 | -1.364 | 1.00 25.69 | C |
| ATOM | 274 | N | ARG C | 38 | 2.787 | 67.142 | 0.503 | 1.00 30.65 | N |
| ATOM | 275 | CA | ARG C | 38 | 3.030 | 65.960 | 1.303 | 1.00 35.42 | C |
| ATOM | 276 | C | ARG C | 38 | 3.936 | 64.983 | 0.565 | 1.00 33.75 | C |
| ATOM | 277 | O | ARG C | 38 | 4.133 | 65.061 | -0.657 | 1.00 31.48 | O |
| ATOM | 278 | CB | ARG C | 38 | 1.721 | 65.273 | 1.714 | 1.00 31.04 | C |
| ATOM | 279 | CG | ARG C | 38 | 1.037 | 64.459 | 0.654 | 1.00 31.84 | C |
| ATOM | 280 | CD | ARG C | 38 | -0.318 | 63.986 | 1.190 | 1.00 28.76 | C |
| ATOM | 281 | NE | ARG C | 38 | -1.059 | 63.233 | 0.184 | 1.00 33.40 | N |
| ATOM | 282 | CZ | ARG C | 38 | -2.280 | 62.718 | 0.368 | 1.00 33.79 | C |

| ATOM | 283 | NH1 | ARG | C | 38 | -2.940 | 62.867 | 1.523 | 1.00 | 29.44 | N1+ |
|------|-----|-----|-----|---|----|--------|--------|-------|------|-------|-----|
| ATOM | 284 | NH2 | ARG | C | 38 | -2.845 | 62.057 | -0.617 | 1.00 | 27.56 | N |
| ATOM | 285 | N | GLN | C | 39 | 4.517 | 64.077 | 1.344 | 1.00 | 31.44 | N |
| ATOM | 286 | CA | GLN | C | 39 | 5.466 | 63.101 | 0.809 | 1.00 | 33.90 | C |
| ATOM | 287 | C | GLN | C | 39 | 5.299 | 61.833 | 1.621 | 1.00 | 30.60 | C |
| ATOM | 288 | O | GLN | C | 39 | 5.589 | 61.823 | 2.816 | 1.00 | 37.53 | O |
| ATOM | 289 | CB | GLN | C | 39 | 6.908 | 63.606 | 0.880 | 1.00 | 29.62 | C |
| ATOM | 290 | CG | GLN | C | 39 | 7.950 | 62.614 | 0.336 | 1.00 | 33.68 | C |
| ATOM | 291 | CD | GLN | C | 39 | 9.317 | 63.272 | 0.142 | 1.00 | 38.26 | C |
| ATOM | 292 | OE1 | GLN | C | 39 | 9.832 | 63.941 | 1.041 | 1.00 | 36.27 | O |
| ATOM | 293 | NE2 | GLN | C | 39 | 9.884 | 63.121 | -1.051 | 1.00 | 31.46 | N |
| ATOM | 294 | N | ALA | C | 40 | 4.806 | 60.795 | 0.980 | 1.00 | 33.74 | N |
| ATOM | 295 | CA | ALA | C | 40 | 4.687 | 59.501 | 1.621 | 1.00 | 42.24 | C |
| ATOM | 296 | C | ALA | C | 40 | 6.082 | 58.868 | 1.723 | 1.00 | 41.93 | C |
| ATOM | 297 | O | ALA | C | 40 | 6.967 | 59.180 | 0.921 | 1.00 | 39.79 | O |
| ATOM | 298 | CB | ALA | C | 40 | 3.726 | 58.625 | 0.811 | 1.00 | 21.89 | C |
| ATOM | 299 | N | PRO | C | 41 | 6.304 | 57.974 | 2.691 | 1.00 | 47.02 | N |
| ATOM | 300 | CA | PRO | C | 41 | 7.671 | 57.444 | 2.920 | 1.00 | 46.31 | C |
| ATOM | 301 | C | PRO | C | 41 | 8.252 | 56.774 | 1.681 | 1.00 | 40.48 | C |
| ATOM | 302 | O | PRO | C | 41 | 7.677 | 55.834 | 1.127 | 1.00 | 49.95 | O |
| ATOM | 303 | CB | PRO | C | 41 | 7.475 | 56.438 | 4.065 | 1.00 | 38.60 | C |
| ATOM | 304 | CG | PRO | C | 41 | 6.179 | 56.872 | 4.754 | 1.00 | 43.78 | C |
| ATOM | 305 | CD | PRO | C | 41 | 5.319 | 57.433 | 3.651 | 1.00 | 44.84 | C |
| ATOM | 306 | N | GLY | C | 42 | 9.416 | 57.254 | 1.256 | 1.00 | 49.67 | N |
| ATOM | 307 | CA | GLY | C | 42 | 10.049 | 56.738 | 0.054 | 1.00 | 47.19 | C |
| ATOM | 308 | C | GLY | C | 42 | 9.476 | 57.189 | -1.279 | 1.00 | 48.22 | C |
| ATOM | 309 | O | GLY | C | 42 | 9.825 | 56.588 | -2.300 | 1.00 | 45.62 | O |
| ATOM | 310 | N | LYS | C | 43 | 8.627 | 58.223 | -1.325 | 1.00 | 42.77 | N |
| ATOM | 311 | CA | LYS | C | 43 | 7.904 | 58.581 | -2.556 | 1.00 | 40.19 | C |
| ATOM | 312 | C | LYS | C | 43 | 8.149 | 60.040 | -2.956 | 1.00 | 36.26 | C |
| ATOM | 313 | O | LYS | C | 43 | 8.895 | 60.789 | -2.310 | 1.00 | 41.39 | O |
| ATOM | 314 | CB | LYS | C | 43 | 6.402 | 58.338 | -2.390 | 1.00 | 43.74 | C |
| ATOM | 315 | CG | LYS | C | 43 | 6.020 | 56.900 | -2.068 | 1.00 | 44.39 | C |
| ATOM | 316 | CD | LYS | C | 43 | 6.527 | 55.936 | -3.112 | 1.00 | 56.25 | C |
| ATOM | 317 | CE | LYS | C | 43 | 5.920 | 54.540 | -2.918 | 1.00 | 72.49 | C |
| ATOM | 318 | NZ | LYS | C | 43 | 6.709 | 53.467 | -3.617 | 1.00 | 73.82 | N1+ |
| ATOM | 319 | N | GLY | C | 44 | 7.498 | 60.457 | -4.036 | 1.00 | 35.63 | N |
| ATOM | 320 | CA | GLY | C | 44 | 7.709 | 61.788 | -4.551 | 1.00 | 29.55 | C |
| ATOM | 321 | C | GLY | C | 44 | 6.833 | 62.802 | -3.843 | 1.00 | 34.99 | C |
| ATOM | 322 | O | GLY | C | 44 | 6.008 | 62.475 | -2.990 | 1.00 | 36.77 | O |
| ATOM | 323 | N | LEU | C | 45 | 7.026 | 64.067 | -4.205 | 1.00 | 30.55 | N |
| ATOM | 324 | CA | LEU | C | 45 | 6.192 | 65.134 | -3.664 | 1.00 | 32.18 | C |
| ATOM | 325 | C | LEU | C | 45 | 4.783 | 65.057 | -4.257 | 1.00 | 31.76 | C |
| ATOM | 326 | O | LEU | C | 45 | 4.597 | 64.740 | -5.440 | 1.00 | 25.93 | O |
| ATOM | 327 | CB | LEU | C | 45 | 6.820 | 66.508 | -3.939 | 1.00 | 31.66 | C |
| ATOM | 328 | CG | LEU | C | 45 | 8.233 | 66.714 | -3.370 | 1.00 | 32.55 | C |
| ATOM | 329 | CD1 | LEU | C | 45 | 8.776 | 68.091 | -3.682 | 1.00 | 27.48 | C |
| ATOM | 330 | CD2 | LEU | C | 45 | 8.219 | 66.491 | -1.871 | 1.00 | 28.43 | C |
| ATOM | 331 | N | GLU | C | 46 | 3.784 | 65.298 | -3.413 | 1.00 | 27.12 | N |
| ATOM | 332 | CA | GLU | C | 46 | 2.404 | 65.402 | -3.865 | 1.00 | 32.84 | C |
| ATOM | 333 | C | GLU | C | 46 | 1.799 | 66.669 | -3.286 | 1.00 | 32.06 | C |
| ATOM | 334 | O | GLU | C | 46 | 1.754 | 66.837 | -2.062 | 1.00 | 31.76 | O |
| ATOM | 335 | CB | GLU | C | 46 | 1.579 | 64.183 | -3.451 | 1.00 | 36.78 | C |
| ATOM | 336 | CG | GLU | C | 46 | 0.226 | 64.173 | -4.126 | 1.00 | 44.38 | C |
| ATOM | 337 | CD | GLU | C | 46 | -0.758 | 63.166 | -3.539 | 1.00 | 46.89 | C |
| ATOM | 338 | OE1 | GLU | C | 46 | -0.435 | 62.467 | -2.548 | 1.00 | 45.34 | O |
| ATOM | 339 | OE2 | GLU | C | 46 | -1.871 | 63.088 | -4.092 | 1.00 | 50.23 | O1- |
| ATOM | 340 | N | TRP | C | 47 | 1.330 | 67.548 | -4.162 | 1.00 | 27.92 | N |
| ATOM | 341 | CA | TRP | C | 47 | 0.676 | 68.764 | -3.715 | 1.00 | 31.90 | C |
| ATOM | 342 | C | TRP | C | 47 | -0.664 | 68.439 | -3.079 | 1.00 | 31.01 | C |
| ATOM | 343 | O | TRP | C | 47 | -1.386 | 67.555 | -3.546 | 1.00 | 32.31 | O |
| ATOM | 344 | CB | TRP | C | 47 | 0.485 | 69.713 | -4.900 | 1.00 | 35.33 | C |
| ATOM | 345 | CG | TRP | C | 47 | -0.297 | 70.972 | -4.630 | 1.00 | 30.11 | C |
| ATOM | 346 | CD1 | TRP | C | 47 | 0.104 | 72.068 | -3.910 | 1.00 | 29.39 | C |
| ATOM | 347 | CD2 | TRP | C | 47 | -1.616 | 71.270 | -5.118 | 1.00 | 29.40 | C |
| ATOM | 348 | NE1 | TRP | C | 47 | -0.886 | 73.032 | -3.925 | 1.00 | 32.24 | N |
| ATOM | 349 | CE2 | TRP | C | 47 | -1.952 | 72.563 | -4.660 | 1.00 | 34.57 | C |
| ATOM | 350 | CE3 | TRP | C | 47 | -2.534 | 70.575 | -5.913 | 1.00 | 30.62 | C |
| ATOM | 351 | CZ2 | TRP | C | 47 | -3.190 | 73.165 | -4.958 | 1.00 | 29.29 | C |
| ATOM | 352 | CZ3 | TRP | C | 47 | -3.764 | 71.177 | -6.215 | 1.00 | 31.59 | C |
| ATOM | 353 | CH2 | TRP | C | 47 | -4.075 | 72.459 | -5.736 | 1.00 | 32.18 | C |

```
ATOM    354  N    VAL C   48     -1.010  69.189  -2.031  1.00 31.17          N
ATOM    355  CA   VAL C   48     -2.268  69.019  -1.303  1.00 28.29          C
ATOM    356  C    VAL C   48     -3.239  70.192  -1.553  1.00 34.03          C
ATOM    357  O    VAL C   48     -4.371  69.989  -2.002  1.00 29.40          O
ATOM    358  CB   VAL C   48     -2.007  68.824   0.206  1.00 29.92          C
ATOM    359  CG1  VAL C   48     -3.321  68.671   0.944  1.00 32.67          C
ATOM    360  CG2  VAL C   48     -1.117  67.606   0.433  1.00 28.26          C
ATOM    361  N    ALA C   49     -2.825  71.421  -1.237  1.00 30.79          N
ATOM    362  CA   ALA C   49     -3.747  72.546  -1.373  1.00 31.82          C
ATOM    363  C    ALA C   49     -2.974  73.854  -1.325  1.00 32.29          C
ATOM    364  O    ALA C   49     -1.845  73.908  -0.823  1.00 30.87          O
ATOM    365  CB   ALA C   49     -4.821  72.517  -0.277  1.00 30.36          C
ATOM    366  N    AVAL C   50    -3.605  74.914  -1.835  0.50 31.84          N
ATOM    367  CA   AVAL C   50    -3.060  76.269  -1.763  0.50 30.56          C
ATOM    368  C    AVAL C   50    -4.179  77.222  -1.373  0.50 31.68          C
ATOM    369  O    AVAL C   50    -5.333  77.047  -1.784  0.50 29.47          O
ATOM    370  CB   AVAL C   50    -2.408  76.711  -3.093  0.50 30.12          C
ATOM    371  CG1  AVAL C   50    -3.459  76.855  -4.204  0.50 28.96          C
ATOM    372  CG2  AVAL C   50    -1.624  77.987  -2.912  0.50 24.87          C
ATOM    373  N    BVAL C   50    -3.584  74.900  -1.900  0.50 32.14          N
ATOM    374  CA   BVAL C   50    -3.117  76.279  -1.776  0.50 30.77          C
ATOM    375  C    BVAL C   50    -4.231  77.106  -1.160  0.50 31.70          C
ATOM    376  O    BVAL C   50    -5.415  76.765  -1.240  0.50 30.32          O
ATOM    377  CB   BVAL C   50    -2.698  76.932  -3.115  0.50 30.01          C
ATOM    378  CG1  BVAL C   50    -2.103  75.960  -4.000  0.50 36.11          C
ATOM    379  CG2  BVAL C   50    -3.896  77.491  -3.831  0.50 31.66          C
ATOM    380  N    ILE C   51     -3.834  78.232  -0.574  1.00 31.49          N
ATOM    381  CA   ILE C   51     -4.770  79.260  -0.151  1.00 28.89          C
ATOM    382  C    ILE C   51     -4.143  80.593  -0.519  1.00 37.27          C
ATOM    383  O    ILE C   51     -2.913  80.736  -0.534  1.00 32.74          O
ATOM    384  CB   ILE C   51     -5.085  79.203   1.356  1.00 33.18          C
ATOM    385  CG1  ILE C   51     -6.238  80.154   1.691  1.00 31.97          C
ATOM    386  CG2  ILE C   51     -3.837  79.550   2.210  1.00 26.57          C
ATOM    387  CD1  ILE C   51     -6.766  79.971   3.093  1.00 31.63          C
ATOM    388  N    TRP C   52     -5.000  81.561  -0.838  1.00 30.70          N
ATOM    389  CA   TRP C   52     -4.563  82.882  -1.241  1.00 25.99          C
ATOM    390  C    TRP C   52     -4.040  83.652  -0.036  1.00 29.12          C
ATOM    391  O    TRP C   52     -4.302  83.303   1.117  1.00 36.14          O
ATOM    392  CB   TRP C   52     -5.722  83.638  -1.898  1.00 27.78          C
ATOM    393  CG   TRP C   52     -5.405  84.135  -3.270  1.00 33.59          C
ATOM    394  CD1  TRP C   52     -5.333  85.445  -3.676  1.00 32.68          C
ATOM    395  CD2  TRP C   52     -5.102  83.340  -4.426  1.00 30.76          C
ATOM    396  NE1  TRP C   52     -5.004  85.510  -5.010  1.00 32.81          N
ATOM    397  CE2  TRP C   52     -4.853  84.235  -5.495  1.00 33.10          C
ATOM    398  CE3  TRP C   52     -4.989  81.962  -4.657  1.00 33.14          C
ATOM    399  CZ2  TRP C   52     -4.516  83.792  -6.779  1.00 32.67          C
ATOM    400  CZ3  TRP C   52     -4.670  81.524  -5.941  1.00 33.56          C
ATOM    401  CH2  TRP C   52     -4.435  82.436  -6.983  1.00 32.43          C
ATOM    402  N    TYR C   53     -3.304  84.726  -0.315  1.00 29.93          N
ATOM    403  CA   TYR C   53     -2.784  85.558   0.768  1.00 38.06          C
ATOM    404  C    TYR C   53     -3.898  86.148   1.630  1.00 40.02          C
ATOM    405  O    TYR C   53     -3.690  86.389   2.829  1.00 43.14          O
ATOM    406  CB   TYR C   53     -1.915  86.685   0.203  1.00 36.99          C
ATOM    407  CG   TYR C   53     -2.522  87.380  -0.992  1.00 42.26          C
ATOM    408  CD1  TYR C   53     -3.449  88.409  -0.831  1.00 39.71          C
ATOM    409  CD2  TYR C   53     -2.172  86.999  -2.290  1.00 38.52          C
ATOM    410  CE1  TYR C   53     -4.015  89.042  -1.927  1.00 38.70          C
ATOM    411  CE2  TYR C   53     -2.720  87.631  -3.397  1.00 43.88          C
ATOM    412  CZ   TYR C   53     -3.647  88.654  -3.211  1.00 46.93          C
ATOM    413  OH   TYR C   53     -4.205  89.264  -4.318  1.00 49.29          O
ATOM    414  N    ASP C   54     -5.082  86.385   1.058  1.00 35.51          N
ATOM    415  CA   ASP C   54     -6.200  86.943   1.811  1.00 38.58          C
ATOM    416  C    ASP C   54     -7.258  85.899   2.166  1.00 37.13          C
ATOM    417  O    ASP C   54     -8.397  86.262   2.468  1.00 42.80          O
ATOM    418  CB   ASP C   54     -6.839  88.093   1.036  1.00 35.61          C
ATOM    419  CG   ASP C   54     -7.319  87.664  -0.336  1.00 39.47          C
ATOM    420  OD1  ASP C   54     -7.355  86.435  -0.617  1.00 40.37          O
ATOM    421  OD2  ASP C   54     -7.678  88.551  -1.129  1.00 40.04          O1-
ATOM    422  N    GLY C   55     -6.921  84.614   2.110  1.00 38.50          N
ATOM    423  CA   GLY C   55     -7.878  83.579   2.451  1.00 30.61          C
ATOM    424  C    GLY C   55     -9.007  83.374   1.465  1.00 39.39          C
```

```
ATOM    425  O    GLY C  55     -9.946  82.625   1.773  1.00 37.86           O
ATOM    426  N    SER C  56     -8.944  83.988   0.276  1.00 37.78           N
ATOM    427  CA   SER C  56    -10.036  83.866  -0.689  1.00 30.88           C
ATOM    428  C    SER C  56     -9.948  82.566  -1.485  1.00 29.51           C
ATOM    429  O    SER C  56    -10.561  81.560  -1.107  1.00 35.66           O
ATOM    430  CB   SER C  56    -10.050  85.075  -1.634  1.00 31.22           C
ATOM    431  OG   SER C  56     -8.824  85.214  -2.331  1.00 32.22           O
ATOM    432  N    ASN C  57     -9.192  82.555  -2.578  1.00 28.37           N
ATOM    433  CA   ASN C  57     -9.143  81.363  -3.423  1.00 35.02           C
ATOM    434  C    ASN C  57     -8.480  80.196  -2.691  1.00 32.70           C
ATOM    435  O    ASN C  57     -7.544  80.388  -1.909  1.00 29.84           O
ATOM    436  CB   ASN C  57     -8.407  81.655  -4.733  1.00 29.59           C
ATOM    437  CG   ASN C  57     -9.224  82.532  -5.682  1.00 35.30           C
ATOM    438  OD1  ASN C  57    -10.082  83.295  -5.248  1.00 31.05           O
ATOM    439  ND2  ASN C  57     -8.982  82.390  -6.988  1.00 33.63           N
ATOM    440  N    LYS C  58     -9.018  78.992  -2.915  1.00 28.81           N
ATOM    441  CA   LYS C  58     -8.510  77.731  -2.374  1.00 33.21           C
ATOM    442  C    LYS C  58     -8.561  76.699  -3.485  1.00 31.65           C
ATOM    443  O    LYS C  58     -9.609  76.539  -4.111  1.00 31.96           O
ATOM    444  CB   LYS C  58     -9.356  77.210  -1.201  1.00 29.22           C
ATOM    445  CG   LYS C  58     -9.438  78.136  -0.030  1.00 39.00           C
ATOM    446  CD   LYS C  58    -10.443  77.655   1.024  1.00 28.02           C
ATOM    447  CE   LYS C  58    -10.456  78.663   2.179  1.00 29.93           C
ATOM    448  NZ   LYS C  58    -11.039  78.171   3.465  1.00 34.24           N1+
ATOM    449  N    TYR C  59     -7.465  75.969  -3.693  1.00 33.17           N
ATOM    450  CA   TYR C  59     -7.402  74.893  -4.680  1.00 31.69           C
ATOM    451  C    TYR C  59     -6.931  73.623  -3.988  1.00 32.39           C
ATOM    452  O    TYR C  59     -6.165  73.681  -3.025  1.00 32.73           O
ATOM    453  CB   TYR C  59     -6.450  75.203  -5.856  1.00 33.11           C
ATOM    454  CG   TYR C  59     -6.646  76.536  -6.574  1.00 41.69           C
ATOM    455  CD1  TYR C  59     -6.539  77.741  -5.914  1.00 48.94           C
ATOM    456  CD2  TYR C  59     -6.892  76.574  -7.925  1.00 52.57           C
ATOM    457  CE1  TYR C  59     -6.714  78.936  -6.579  1.00 54.05           C
ATOM    458  CE2  TYR C  59     -7.055  77.762  -8.590  1.00 51.75           C
ATOM    459  CZ   TYR C  59     -6.973  78.936  -7.916  1.00 42.38           C
ATOM    460  OH   TYR C  59     -7.155  80.120  -8.583  1.00 40.63           O
ATOM    461  N    TYR C  60     -7.401  72.470  -4.475  1.00 28.26           N
ATOM    462  CA   TYR C  60     -7.080  71.205  -3.835  1.00 26.89           C
ATOM    463  C    TYR C  60     -6.720  70.156  -4.864  1.00 32.21           C
ATOM    464  O    TYR C  60     -7.276  70.128  -5.967  1.00 30.27           O
ATOM    465  CB   TYR C  60     -8.232  70.657  -3.004  1.00 28.01           C
ATOM    466  CG   TYR C  60     -8.665  71.517  -1.854  1.00 34.24           C
ATOM    467  CD1  TYR C  60     -9.607  72.540  -2.036  1.00 31.75           C
ATOM    468  CD2  TYR C  60     -8.169  71.297  -0.570  1.00 31.69           C
ATOM    469  CE1  TYR C  60    -10.020  73.341  -0.963  1.00 28.22           C
ATOM    470  CE2  TYR C  60     -8.595  72.093   0.512  1.00 30.87           C
ATOM    471  CZ   TYR C  60     -9.516  73.111   0.301  1.00 28.55           C
ATOM    472  OH   TYR C  60     -9.924  73.903   1.354  1.00 30.94           O
ATOM    473  N    ALA C  61     -5.807  69.267  -4.468  1.00 27.28           N
ATOM    474  CA   ALA C  61     -5.533  68.075  -5.252  1.00 28.59           C
ATOM    475  C    ALA C  61     -6.745  67.149  -5.238  1.00 34.61           C
ATOM    476  O    ALA C  61     -7.493  67.080  -4.260  1.00 31.50           O
ATOM    477  CB   ALA C  61     -4.315  67.342  -4.706  1.00 23.81           C
ATOM    478  N    ASP C  62     -6.920  66.423  -6.341  1.00 38.58           N
ATOM    479  CA   ASP C  62     -8.069  65.537  -6.487  1.00 40.90           C
ATOM    480  C    ASP C  62     -8.129  64.490  -5.382  1.00 41.29           C
ATOM    481  O    ASP C  62     -9.210  64.175  -4.884  1.00 43.64           O
ATOM    482  CB   ASP C  62     -8.027  64.862  -7.858  1.00 41.48           C
ATOM    483  CG   ASP C  62     -8.775  65.657  -8.908  1.00 52.54           C
ATOM    484  OD1  ASP C  62     -9.074  66.853  -8.639  1.00 50.91           O
ATOM    485  OD2  ASP C  62     -9.074  65.083  -9.984  1.00 54.03           O1-
ATOM    486  N    SER C  63     -6.978  63.960  -4.967  1.00 38.35           N
ATOM    487  CA   SER C  63     -6.931  62.918  -3.948  1.00 40.77           C
ATOM    488  C    SER C  63     -7.429  63.398  -2.589  1.00 41.73           C
ATOM    489  O    SER C  63     -7.557  62.586  -1.666  1.00 37.97           O
ATOM    490  CB   SER C  63     -5.493  62.386  -3.804  1.00 39.37           C
ATOM    491  OG   SER C  63     -4.582  63.445  -3.547  1.00 39.82           O
ATOM    492  N    VAL C  64     -7.730  64.681  -2.445  1.00 37.08           N
ATOM    493  CA   VAL C  64     -7.937  65.265  -1.130  1.00 34.49           C
ATOM    494  C    VAL C  64     -9.234  66.090  -1.105  1.00 35.91           C
ATOM    495  O    VAL C  64     -9.766  66.419  -0.033  1.00 35.61           O
```

275

| ATOM | 496 | CB | VAL | C | 64 | -6.652 | 66.052 | -0.800 | 1.00 | 37.75 | C |
| ATOM | 497 | CG1 | VAL | C | 64 | -6.895 | 67.438 | -0.272 | 1.00 | 34.28 | C |
| ATOM | 498 | CG2 | VAL | C | 64 | -5.734 | 65.214 | 0.078 | 1.00 | 37.52 | C |
| ATOM | 499 | N | LYS | C | 65 | -9.774 | 66.389 | -2.294 | 1.00 | 33.05 | N |
| ATOM | 500 | CA | LYS | C | 65 | -11.007 | 67.169 | -2.424 | 1.00 | 35.12 | C |
| ATOM | 501 | C | LYS | C | 65 | -12.143 | 66.581 | -1.599 | 1.00 | 41.45 | C |
| ATOM | 502 | O | LYS | C | 65 | -12.378 | 65.368 | -1.600 | 1.00 | 37.85 | O |
| ATOM | 503 | CB | LYS | C | 65 | -11.463 | 67.244 | -3.888 | 1.00 | 35.87 | C |
| ATOM | 504 | CG | LYS | C | 65 | -10.771 | 68.290 | -4.714 | 1.00 | 35.54 | C |
| ATOM | 505 | CD | LYS | C | 65 | -11.454 | 68.428 | -6.057 | 1.00 | 42.42 | C |
| ATOM | 506 | CE | LYS | C | 65 | -10.975 | 69.680 | -6.795 | 1.00 | 38.45 | C |
| ATOM | 507 | NZ | LYS | C | 65 | -9.588 | 69.479 | -7.281 | 1.00 | 40.01 | N1+ |
| ATOM | 508 | N | GLY | C | 66 | -12.876 | 67.460 | -0.925 | 1.00 | 38.42 | N |
| ATOM | 509 | CA | GLY | C | 66 | -14.008 | 67.048 | -0.131 | 1.00 | 34.79 | C |
| ATOM | 510 | C | GLY | C | 66 | -13.656 | 66.580 | 1.252 | 1.00 | 43.32 | C |
| ATOM | 511 | O | GLY | C | 66 | -14.504 | 66.628 | 2.139 | 1.00 | 44.56 | O |
| ATOM | 512 | N | ARG | C | 67 | -12.416 | 66.177 | 1.488 | 1.00 | 42.85 | N |
| ATOM | 513 | CA | ARG | C | 67 | -12.040 | 65.681 | 2.798 | 1.00 | 33.59 | C |
| ATOM | 514 | C | ARG | C | 67 | -11.185 | 66.653 | 3.587 | 1.00 | 38.28 | C |
| ATOM | 515 | O | ARG | C | 67 | -11.308 | 66.698 | 4.812 | 1.00 | 35.39 | O |
| ATOM | 516 | CB | ARG | C | 67 | -11.317 | 64.333 | 2.663 | 1.00 | 38.43 | C |
| ATOM | 517 | CG | ARG | C | 67 | -12.241 | 63.220 | 2.171 | 1.00 | 33.48 | C |
| ATOM | 518 | CD | ARG | C | 67 | -11.540 | 61.860 | 2.172 | 1.00 | 39.76 | C |
| ATOM | 519 | NE | ARG | C | 67 | -10.284 | 61.891 | 1.423 | 1.00 | 38.65 | N |
| ATOM | 520 | CZ | ARG | C | 67 | -9.081 | 61.717 | 1.967 | 1.00 | 39.28 | C |
| ATOM | 521 | NH1 | ARG | C | 67 | -8.981 | 61.482 | 3.259 | 1.00 | 31.68 | N1+ |
| ATOM | 522 | NH2 | ARG | C | 67 | -7.977 | 61.785 | 1.224 | 1.00 | 36.70 | N |
| ATOM | 523 | N | PHE | C | 68 | -10.304 | 67.413 | 2.922 | 1.00 | 37.96 | N |
| ATOM | 524 | CA | PHE | C | 68 | -9.395 | 68.344 | 3.576 | 1.00 | 30.65 | C |
| ATOM | 525 | C | PHE | C | 68 | -9.874 | 69.766 | 3.351 | 1.00 | 34.00 | C |
| ATOM | 526 | O | PHE | C | 68 | -10.488 | 70.071 | 2.325 | 1.00 | 36.05 | O |
| ATOM | 527 | CB | PHE | C | 68 | -7.959 | 68.224 | 3.047 | 1.00 | 37.02 | C |
| ATOM | 528 | CG | PHE | C | 68 | -7.264 | 66.900 | 3.360 | 1.00 | 38.71 | C |
| ATOM | 529 | CD1 | PHE | C | 68 | -7.954 | 65.814 | 3.888 | 1.00 | 35.33 | C |
| ATOM | 530 | CD2 | PHE | C | 68 | -5.894 | 66.767 | 3.140 | 1.00 | 37.81 | C |
| ATOM | 531 | CE1 | PHE | C | 68 | -7.299 | 64.615 | 4.164 | 1.00 | 36.61 | C |
| ATOM | 532 | CE2 | PHE | C | 68 | -5.218 | 65.569 | 3.425 | 1.00 | 33.40 | C |
| ATOM | 533 | CZ | PHE | C | 68 | -5.919 | 64.498 | 3.934 | 1.00 | 40.51 | C |
| ATOM | 534 | N | THR | C | 69 | -9.582 | 70.646 | 4.307 | 1.00 | 35.34 | N |
| ATOM | 535 | CA | THR | C | 69 | -9.924 | 72.055 | 4.163 | 1.00 | 29.08 | C |
| ATOM | 536 | C | THR | C | 69 | -8.743 | 72.900 | 4.621 | 1.00 | 31.52 | C |
| ATOM | 537 | O | THR | C | 69 | -8.229 | 72.710 | 5.725 | 1.00 | 33.87 | O |
| ATOM | 538 | CB | THR | C | 69 | -11.198 | 72.407 | 4.954 | 1.00 | 32.76 | C |
| ATOM | 539 | OG1 | THR | C | 69 | -12.294 | 71.663 | 4.427 | 1.00 | 36.96 | O |
| ATOM | 540 | CG2 | THR | C | 69 | -11.545 | 73.923 | 4.828 | 1.00 | 25.39 | C |
| ATOM | 541 | N | ILE | C | 70 | -8.309 | 73.827 | 3.778 | 1.00 | 33.09 | N |
| ATOM | 542 | CA | ILE | C | 70 | -7.201 | 74.720 | 4.113 | 1.00 | 29.02 | C |
| ATOM | 543 | C | ILE | C | 70 | -7.786 | 76.038 | 4.602 | 1.00 | 32.22 | C |
| ATOM | 544 | O | ILE | C | 70 | -8.810 | 76.503 | 4.093 | 1.00 | 33.11 | O |
| ATOM | 545 | CB | ILE | C | 70 | -6.265 | 74.911 | 2.899 | 1.00 | 35.27 | C |
| ATOM | 546 | CG1 | ILE | C | 70 | -4.942 | 75.579 | 3.288 | 1.00 | 31.47 | C |
| ATOM | 547 | CG2 | ILE | C | 70 | -6.939 | 75.726 | 1.800 | 1.00 | 23.76 | C |
| ATOM | 548 | CD1 | ILE | C | 70 | -3.965 | 75.590 | 2.138 | 1.00 | 31.12 | C |
| ATOM | 549 | N | SER | C | 71 | -7.174 | 76.618 | 5.627 | 1.00 | 35.69 | N |
| ATOM | 550 | CA | SER | C | 71 | -7.670 | 77.883 | 6.153 | 1.00 | 38.73 | C |
| ATOM | 551 | C | SER | C | 71 | -6.513 | 78.588 | 6.845 | 1.00 | 37.39 | C |
| ATOM | 552 | O | SER | C | 71 | -5.444 | 78.009 | 7.046 | 1.00 | 41.40 | O |
| ATOM | 553 | CB | SER | C | 71 | -8.849 | 77.655 | 7.100 | 1.00 | 37.80 | C |
| ATOM | 554 | OG | SER | C | 71 | -8.444 | 76.880 | 8.217 | 1.00 | 38.72 | O |
| ATOM | 555 | N | ARG | C | 72 | -6.726 | 79.852 | 7.204 | 1.00 | 35.68 | N |
| ATOM | 556 | CA | ARG | C | 72 | -5.674 | 80.600 | 7.876 | 1.00 | 36.89 | C |
| ATOM | 557 | C | ARG | C | 72 | -6.274 | 81.545 | 8.908 | 1.00 | 40.29 | C |
| ATOM | 558 | O | ARG | C | 72 | -7.421 | 81.973 | 8.791 | 1.00 | 38.24 | O |
| ATOM | 559 | CB | ARG | C | 72 | -4.825 | 81.385 | 6.864 | 1.00 | 35.87 | C |
| ATOM | 560 | CG | ARG | C | 72 | -5.636 | 82.390 | 6.057 | 1.00 | 34.72 | C |
| ATOM | 561 | CD | ARG | C | 72 | -4.828 | 82.983 | 4.903 | 1.00 | 30.54 | C |
| ATOM | 562 | NE | ARG | C | 72 | -3.674 | 83.722 | 5.397 | 1.00 | 39.63 | N |
| ATOM | 563 | CZ | ARG | C | 72 | -2.575 | 83.951 | 4.694 | 1.00 | 41.45 | C |
| ATOM | 564 | NH1 | ARG | C | 72 | -2.475 | 83.499 | 3.449 | 1.00 | 37.50 | N1+ |
| ATOM | 565 | NH2 | ARG | C | 72 | -1.579 | 84.643 | 5.240 | 1.00 | 38.82 | N |
| ATOM | 566 | N | ASP | C | 73 | -5.478 | 81.878 | 9.920 | 1.00 | 38.55 | N |

| ATOM | 567 | CA  | ASP | C | 73 | -5.852 | 82.875 | 10.919 | 1.00 | 40.45 | C   |
|------|-----|-----|-----|---|----|--------|--------|--------|------|-------|-----|
| ATOM | 568 | C   | ASP | C | 73 | -4.704 | 83.871 | 11.015 | 1.00 | 44.31 | C   |
| ATOM | 569 | O   | ASP | C | 73 | -3.699 | 83.601 | 11.676 | 1.00 | 47.19 | O   |
| ATOM | 570 | CB  | ASP | C | 73 | -6.142 | 82.239 | 12.277 | 1.00 | 45.34 | C   |
| ATOM | 571 | CG  | ASP | C | 73 | -6.685 | 83.247 | 13.303 | 1.00 | 48.06 | C   |
| ATOM | 572 | OD1 | ASP | C | 73 | -6.492 | 84.479 | 13.138 | 1.00 | 54.90 | O   |
| ATOM | 573 | OD2 | ASP | C | 73 | -7.277 | 82.797 | 14.305 | 1.00 | 53.87 | O1- |
| ATOM | 574 | N   | ASN | C | 74 | -4.865 | 85.028 | 10.374 | 1.00 | 39.84 | N   |
| ATOM | 575 | CA  | ASN | C | 74 | -3.778 | 85.991 | 10.341 | 1.00 | 43.99 | C   |
| ATOM | 576 | C   | ASN | C | 74 | -3.450 | 86.497 | 11.739 | 1.00 | 48.38 | C   |
| ATOM | 577 | O   | ASN | C | 74 | -2.282 | 86.767 | 12.040 | 1.00 | 55.63 | O   |
| ATOM | 578 | CB  | ASN | C | 74 | -4.126 | 87.136 | 9.385  | 1.00 | 39.05 | C   |
| ATOM | 579 | CG  | ASN | C | 74 | -3.974 | 86.735 | 7.922  | 1.00 | 46.52 | C   |
| ATOM | 580 | OD1 | ASN | C | 74 | -3.620 | 85.591 | 7.606  | 1.00 | 45.49 | O   |
| ATOM | 581 | ND2 | ASN | C | 74 | -4.238 | 87.668 | 7.024  | 1.00 | 41.67 | N   |
| ATOM | 582 | N   | SER | C | 75 | -4.444 | 86.570 | 12.626 | 1.00 | 52.35 | N   |
| ATOM | 583 | CA  | SER | C | 75 | -4.172 | 87.063 | 13.973 | 1.00 | 49.93 | C   |
| ATOM | 584 | C   | SER | C | 75 | -3.251 | 86.138 | 14.759 | 1.00 | 51.44 | C   |
| ATOM | 585 | O   | SER | C | 75 | -2.606 | 86.596 | 15.705 | 1.00 | 53.63 | O   |
| ATOM | 586 | CB  | SER | C | 75 | -5.480 | 87.277 | 14.735 | 1.00 | 45.73 | C   |
| ATOM | 587 | OG  | SER | C | 75 | -6.025 | 86.059 | 15.205 | 1.00 | 53.39 | O   |
| ATOM | 588 | N   | LYS | C | 76 | -3.158 | 84.860 | 14.387 | 1.00 | 49.51 | N   |
| ATOM | 589 | CA  | LYS | C | 76 | -2.235 | 83.925 | 15.018 | 1.00 | 43.14 | C   |
| ATOM | 590 | C   | LYS | C | 76 | -1.069 | 83.563 | 14.114 | 1.00 | 50.55 | C   |
| ATOM | 591 | O   | LYS | C | 76 | -0.325 | 82.632 | 14.440 | 1.00 | 45.43 | O   |
| ATOM | 592 | CB  | LYS | C | 76 | -2.951 | 82.633 | 15.422 | 1.00 | 45.66 | C   |
| ATOM | 593 | CG  | LYS | C | 76 | -4.185 | 82.802 | 16.282 | 1.00 | 49.74 | C   |
| ATOM | 594 | CD  | LYS | C | 76 | -4.789 | 81.447 | 16.612 | 1.00 | 50.31 | C   |
| ATOM | 595 | CE  | LYS | C | 76 | -6.149 | 81.588 | 17.303 | 1.00 | 56.50 | C   |
| ATOM | 596 | NZ  | LYS | C | 76 | -6.769 | 80.256 | 17.607 | 1.00 | 68.70 | N1+ |
| ATOM | 597 | N   | ASN | C | 77 | -0.896 | 84.275 | 12.992 | 1.00 | 47.54 | N   |
| ATOM | 598 | CA  | ASN | C | 77 | 0.093  | 83.953 | 11.963 | 1.00 | 44.06 | C   |
| ATOM | 599 | C   | ASN | C | 77 | 0.174  | 82.458 | 11.667 | 1.00 | 45.22 | C   |
| ATOM | 600 | O   | ASN | C | 77 | 1.278  | 81.913 | 11.537 | 1.00 | 43.31 | O   |
| ATOM | 601 | CB  | ASN | C | 77 | 1.470  | 84.476 | 12.358 | 1.00 | 41.59 | C   |
| ATOM | 602 | CG  | ASN | C | 77 | 1.530  | 85.984 | 12.365 | 1.00 | 44.41 | C   |
| ATOM | 603 | OD1 | ASN | C | 77 | 0.827  | 86.646 | 11.609 | 1.00 | 48.47 | O   |
| ATOM | 604 | ND2 | ASN | C | 77 | 2.354  | 86.535 | 13.232 | 1.00 | 44.19 | N   |
| ATOM | 605 | N   | THR | C | 78 | -0.981 | 81.789 | 11.547 | 1.00 | 36.70 | N   |
| ATOM | 606 | CA  | THR | C | 78 | -1.021 | 80.336 | 11.472 | 1.00 | 40.83 | C   |
| ATOM | 607 | C   | THR | C | 78 | -1.861 | 79.905 | 10.280 | 1.00 | 44.26 | C   |
| ATOM | 608 | O   | THR | C | 78 | -2.909 | 80.500 | 10.003 | 1.00 | 37.17 | O   |
| ATOM | 609 | CB  | THR | C | 78 | -1.599 | 79.724 | 12.774 | 1.00 | 40.36 | C   |
| ATOM | 610 | OG1 | THR | C | 78 | -0.819 | 80.156 | 13.888 | 1.00 | 45.18 | O   |
| ATOM | 611 | CG2 | THR | C | 78 | -1.572 | 78.205 | 12.739 | 1.00 | 38.53 | C   |
| ATOM | 612 | N   | LEU | C | 79 | -1.365 | 78.886 | 9.574  | 1.00 | 39.56 | N   |
| ATOM | 613 | CA  | LEU | C | 79 | -2.067 | 78.174 | 8.513  | 1.00 | 33.74 | C   |
| ATOM | 614 | C   | LEU | C | 79 | -2.557 | 76.821 | 9.033  | 1.00 | 37.68 | C   |
| ATOM | 615 | O   | LEU | C | 79 | -1.875 | 76.162 | 9.822  | 1.00 | 39.25 | O   |
| ATOM | 616 | CB  | LEU | C | 79 | -1.133 | 77.969 | 7.307  | 1.00 | 40.59 | C   |
| ATOM | 617 | CG  | LEU | C | 79 | -1.615 | 77.032 | 6.178  | 1.00 | 40.09 | C   |
| ATOM | 618 | CD1 | LEU | C | 79 | -2.574 | 77.739 | 5.277  | 1.00 | 28.75 | C   |
| ATOM | 619 | CD2 | LEU | C | 79 | -0.476 | 76.386 | 5.372  | 1.00 | 31.11 | C   |
| ATOM | 620 | N   | TYR | C | 80 | -3.738 | 76.399 | 8.599  | 1.00 | 35.37 | N   |
| ATOM | 621 | CA  | TYR | C | 80 | -4.296 | 75.136 | 9.067  | 1.00 | 38.96 | C   |
| ATOM | 622 | C   | TYR | C | 80 | -4.592 | 74.197 | 7.909  | 1.00 | 36.52 | C   |
| ATOM | 623 | O   | TYR | C | 80 | -4.895 | 74.629 | 6.798  | 1.00 | 37.65 | O   |
| ATOM | 624 | CB  | TYR | C | 80 | -5.592 | 75.328 | 9.872  | 1.00 | 36.20 | C   |
| ATOM | 625 | CG  | TYR | C | 80 | -5.421 | 76.193 | 11.073 | 1.00 | 41.77 | C   |
| ATOM | 626 | CD1 | TYR | C | 80 | -4.830 | 75.697 | 12.234 | 1.00 | 41.02 | C   |
| ATOM | 627 | CD2 | TYR | C | 80 | -5.845 | 77.516 | 11.052 | 1.00 | 39.70 | C   |
| ATOM | 628 | CE1 | TYR | C | 80 | -4.655 | 76.511 | 13.345 | 1.00 | 44.22 | C   |
| ATOM | 629 | CE2 | TYR | C | 80 | -5.685 | 78.340 | 12.152 | 1.00 | 40.29 | C   |
| ATOM | 630 | CZ  | TYR | C | 80 | -5.089 | 77.837 | 13.297 | 1.00 | 49.44 | C   |
| ATOM | 631 | OH  | TYR | C | 80 | -4.938 | 78.666 | 14.388 | 1.00 | 54.21 | O   |
| ATOM | 632 | N   | LEU | C | 81 | -4.520 | 72.901 | 8.194  | 1.00 | 36.72 | N   |
| ATOM | 633 | CA  | LEU | C | 81 | -5.055 | 71.869 | 7.317  | 1.00 | 41.79 | C   |
| ATOM | 634 | C   | LEU | C | 81 | -5.970 | 70.979 | 8.151  | 1.00 | 40.31 | C   |
| ATOM | 635 | O   | LEU | C | 81 | -5.497 | 70.223 | 9.006  | 1.00 | 40.72 | O   |
| ATOM | 636 | CB  | LEU | C | 81 | -3.943 | 71.050 | 6.665  | 1.00 | 36.53 | C   |
| ATOM | 637 | CG  | LEU | C | 81 | -4.473 | 70.006 | 5.677  | 1.00 | 29.95 | C   |

| ATOM | 638 | CD1 | LEU C | 81 | -5.070 | 70.688 | 4.452 | 1.00 | 35.27 | C |
|------|-----|-----|-------|----|--------|--------|-------|------|-------|---|
| ATOM | 639 | CD2 | LEU C | 81 | -3.369 | 69.024 | 5.277 | 1.00 | 31.44 | C |
| ATOM | 640 | N | GLN C | 82 | -7.274 | 71.101 | 7.926 | 1.00 | 38.93 | N |
| ATOM | 641 | CA | GLN C | 82 | -8.277 | 70.254 | 8.566 | 1.00 | 35.95 | C |
| ATOM | 642 | C | GLN C | 82 | -8.454 | 68.996 | 7.728 | 1.00 | 36.48 | C |
| ATOM | 643 | O | GLN C | 82 | -8.834 | 69.081 | 6.557 | 1.00 | 41.11 | O |
| ATOM | 644 | CB | GLN C | 82 | -9.606 | 71.009 | 8.718 | 1.00 | 32.87 | C |
| ATOM | 645 | CG | GLN C | 82 | -10.720 | 70.190 | 9.383 | 1.00 | 27.13 | C |
| ATOM | 646 | CD | GLN C | 82 | -10.339 | 69.723 | 10.783 | 1.00 | 41.21 | C |
| ATOM | 647 | OE1 | GLN C | 82 | -9.875 | 70.511 | 11.621 | 1.00 | 41.00 | O |
| ATOM | 648 | NE2 | GLN C | 82 | -10.466 | 68.418 | 11.018 | 1.00 | 37.49 | N |
| ATOM | 649 | N | MET C | 83 | -8.136 | 67.839 | 8.304 | 1.00 | 36.04 | N |
| ATOM | 650 | CA | MET C | 83 | -8.151 | 66.557 | 7.594 | 1.00 | 39.43 | C |
| ATOM | 651 | C | MET C | 83 | -9.242 | 65.670 | 8.174 | 1.00 | 41.49 | C |
| ATOM | 652 | O | MET C | 83 | -9.145 | 65.225 | 9.321 | 1.00 | 46.39 | O |
| ATOM | 653 | CB | MET C | 83 | -6.791 | 65.854 | 7.669 | 1.00 | 42.85 | C |
| ATOM | 654 | CG | MET C | 83 | -5.616 | 66.618 | 7.030 | 1.00 | 44.37 | C |
| ATOM | 655 | SD | MET C | 83 | -4.047 | 65.719 | 7.166 | 1.00 | 45.89 | S |
| ATOM | 656 | CE | MET C | 83 | -3.833 | 65.851 | 8.911 | 1.00 | 41.39 | C |
| ATOM | 657 | N | ASN C | 84 | -10.284 | 65.432 | 7.400 | 1.00 | 37.96 | N |
| ATOM | 658 | CA | ASN C | 84 | -11.368 | 64.560 | 7.811 | 1.00 | 38.76 | C |
| ATOM | 659 | C | ASN C | 84 | -11.332 | 63.268 | 7.002 | 1.00 | 40.86 | C |
| ATOM | 660 | O | ASN C | 84 | -10.744 | 63.205 | 5.918 | 1.00 | 40.04 | O |
| ATOM | 661 | CB | ASN C | 84 | -12.715 | 65.273 | 7.649 | 1.00 | 33.01 | C |
| ATOM | 662 | CG | ASN C | 84 | -12.807 | 66.525 | 8.498 | 1.00 | 37.92 | C |
| ATOM | 663 | OD1 | ASN C | 84 | -12.179 | 66.617 | 9.548 | 1.00 | 46.79 | O |
| ATOM | 664 | ND2 | ASN C | 84 | -13.617 | 67.480 | 8.071 | 1.00 | 40.77 | N |
| ATOM | 665 | N | SER C | 85 | -11.956 | 62.226 | 7.547 | 1.00 | 43.43 | N |
| ATOM | 666 | CA | SER C | 85 | -12.072 | 60.945 | 6.852 | 1.00 | 40.65 | C |
| ATOM | 667 | C | SER C | 85 | -10.714 | 60.421 | 6.406 | 1.00 | 39.67 | C |
| ATOM | 668 | O | SER C | 85 | -10.529 | 60.022 | 5.252 | 1.00 | 36.84 | O |
| ATOM | 669 | CB | SER C | 85 | -13.010 | 61.055 | 5.654 | 1.00 | 38.73 | C |
| ATOM | 670 | OG | SER C | 85 | -14.333 | 61.254 | 6.094 | 1.00 | 48.16 | O |
| ATOM | 671 | N | LEU C | 86 | -9.757 | 60.421 | 7.331 | 1.00 | 38.73 | N |
| ATOM | 672 | CA | LEU C | 86 | -8.390 | 60.056 | 6.977 | 1.00 | 37.86 | C |
| ATOM | 673 | C | LEU C | 86 | -8.328 | 58.625 | 6.457 | 1.00 | 38.76 | C |
| ATOM | 674 | O | LEU C | 86 | -9.063 | 57.749 | 6.909 | 1.00 | 43.94 | O |
| ATOM | 675 | CB | LEU C | 86 | -7.471 | 60.241 | 8.184 | 1.00 | 39.02 | C |
| ATOM | 676 | CG | LEU C | 86 | -7.098 | 61.711 | 8.395 | 1.00 | 38.85 | C |
| ATOM | 677 | CD1 | LEU C | 86 | -6.467 | 61.983 | 9.765 | 1.00 | 37.48 | C |
| ATOM | 678 | CD2 | LEU C | 86 | -6.157 | 62.137 | 7.290 | 1.00 | 41.68 | C |
| ATOM | 679 | N | ARG C | 87 | -7.468 | 58.400 | 5.470 | 1.00 | 41.40 | N |
| ATOM | 680 | CA | ARG C | 87 | -7.251 | 57.088 | 4.882 | 1.00 | 37.22 | C |
| ATOM | 681 | C | ARG C | 87 | -5.782 | 56.695 | 5.005 | 1.00 | 43.89 | C |
| ATOM | 682 | O | ARG C | 87 | -4.900 | 57.546 | 5.190 | 1.00 | 37.28 | O |
| ATOM | 683 | CB | ARG C | 87 | -7.661 | 57.052 | 3.409 | 1.00 | 41.43 | C |
| ATOM | 684 | CG | ARG C | 87 | -8.995 | 57.661 | 3.103 | 1.00 | 47.94 | C |
| ATOM | 685 | CD | ARG C | 87 | -9.556 | 57.025 | 1.858 | 1.00 | 52.28 | C |
| ATOM | 686 | NE | ARG C | 87 | -9.802 | 57.968 | 0.770 | 1.00 | 59.65 | N |
| ATOM | 687 | CZ | ARG C | 87 | -10.945 | 58.629 | 0.592 | 1.00 | 56.84 | C |
| ATOM | 688 | NH1 | ARG C | 87 | -11.947 | 58.483 | 1.459 | 1.00 | 62.30 | N1+ |
| ATOM | 689 | NH2 | ARG C | 87 | -11.079 | 59.444 | -0.449 | 1.00 | 58.47 | N |
| ATOM | 690 | N | VAL C | 88 | -5.536 | 55.384 | 4.900 | 1.00 | 37.13 | N |
| ATOM | 691 | CA | VAL C | 88 | -4.184 | 54.851 | 5.067 | 1.00 | 40.40 | C |
| ATOM | 692 | C | VAL C | 88 | -3.218 | 55.550 | 4.113 | 1.00 | 41.32 | C |
| ATOM | 693 | O | VAL C | 88 | -2.142 | 56.021 | 4.510 | 1.00 | 39.20 | O |
| ATOM | 694 | CB | VAL C | 88 | -4.191 | 53.320 | 4.863 | 1.00 | 38.65 | C |
| ATOM | 695 | CG1 | VAL C | 88 | -2.774 | 52.764 | 4.702 | 1.00 | 37.78 | C |
| ATOM | 696 | CG2 | VAL C | 88 | -4.883 | 52.634 | 6.045 | 1.00 | 35.60 | C |
| ATOM | 697 | N | GLU C | 89 | -3.621 | 55.684 | 2.856 | 1.00 | 36.74 | N |
| ATOM | 698 | CA | GLU C | 89 | -2.794 | 56.334 | 1.849 | 1.00 | 37.93 | C |
| ATOM | 699 | C | GLU C | 89 | -2.629 | 57.845 | 2.082 | 1.00 | 39.72 | C |
| ATOM | 700 | O | GLU C | 89 | -1.953 | 58.480 | 1.274 | 1.00 | 41.02 | O |
| ATOM | 701 | CB | GLU C | 89 | -3.324 | 56.041 | 0.434 | 1.00 | 30.08 | C |
| ATOM | 702 | CG | GLU C | 89 | -4.747 | 56.489 | 0.150 | 1.00 | 44.00 | C |
| ATOM | 703 | CD | GLU C | 89 | -5.802 | 55.528 | 0.720 | 1.00 | 65.19 | C |
| ATOM | 704 | OE1 | GLU C | 89 | -7.001 | 55.726 | 0.398 | 1.00 | 72.89 | O |
| ATOM | 705 | OE2 | GLU C | 89 | -5.434 | 54.580 | 1.479 | 1.00 | 57.08 | O1- |
| ATOM | 706 | N | ASP C | 90 | -3.261 | 58.456 | 3.092 | 1.00 | 34.66 | N |
| ATOM | 707 | CA | ASP C | 90 | -2.892 | 59.824 | 3.461 | 1.00 | 35.63 | C |
| ATOM | 708 | C | ASP C | 90 | -1.648 | 59.885 | 4.344 | 1.00 | 36.64 | C |

| ATOM | 709 | O | ASP | C | 90 | -1.213 | 60.994 | 4.704 | 1.00 | 34.03 | O |
| ATOM | 710 | CB | ASP | C | 90 | -4.032 | 60.545 | 4.200 | 1.00 | 37.08 | C |
| ATOM | 711 | CG | ASP | C | 90 | -5.265 | 60.761 | 3.338 | 1.00 | 41.53 | C |
| ATOM | 712 | OD1 | ASP | C | 90 | -5.126 | 61.135 | 2.135 | 1.00 | 39.64 | O |
| ATOM | 713 | OD2 | ASP | C | 90 | -6.380 | 60.541 | 3.882 | 1.00 | 36.67 | O1- |
| ATOM | 714 | N | THR | C | 91 | -1.085 | 58.733 | 4.713 | 1.00 | 35.16 | N |
| ATOM | 715 | CA | THR | C | 91 | 0.114 | 58.684 | 5.552 | 1.00 | 38.29 | C |
| ATOM | 716 | C | THR | C | 91 | 1.260 | 59.354 | 4.826 | 1.00 | 31.75 | C |
| ATOM | 717 | O | THR | C | 91 | 1.572 | 58.989 | 3.690 | 1.00 | 36.79 | O |
| ATOM | 718 | CB | THR | C | 91 | 0.484 | 57.225 | 5.857 | 1.00 | 49.62 | C |
| ATOM | 719 | OG1 | THR | C | 91 | -0.561 | 56.594 | 6.611 | 1.00 | 40.91 | O |
| ATOM | 720 | CG2 | THR | C | 91 | 1.824 | 57.138 | 6.602 | 1.00 | 35.10 | C |
| ATOM | 721 | N | ALA | C | 92 | 1.876 | 60.340 | 5.462 | 1.00 | 34.05 | N |
| ATOM | 722 | CA | ALA | C | 92 | 2.886 | 61.136 | 4.778 | 1.00 | 32.82 | C |
| ATOM | 723 | C | ALA | C | 92 | 3.495 | 62.111 | 5.760 | 1.00 | 30.84 | C |
| ATOM | 724 | O | ALA | C | 92 | 2.938 | 62.368 | 6.827 | 1.00 | 30.26 | O |
| ATOM | 725 | CB | ALA | C | 92 | 2.304 | 61.923 | 3.596 | 1.00 | 36.15 | C |
| ATOM | 726 | N | VAL | C | 93 | 4.648 | 62.657 | 5.382 | 1.00 | 35.57 | N |
| ATOM | 727 | CA | VAL | C | 93 | 5.111 | 63.910 | 5.967 | 1.00 | 35.12 | C |
| ATOM | 728 | C | VAL | C | 93 | 4.408 | 65.064 | 5.265 | 1.00 | 34.14 | C |
| ATOM | 729 | O | VAL | C | 93 | 4.322 | 65.099 | 4.032 | 1.00 | 35.63 | O |
| ATOM | 730 | CB | VAL | C | 93 | 6.633 | 64.038 | 5.857 | 1.00 | 34.90 | C |
| ATOM | 731 | CG1 | VAL | C | 93 | 7.077 | 65.442 | 6.323 | 1.00 | 28.59 | C |
| ATOM | 732 | CG2 | VAL | C | 93 | 7.297 | 62.925 | 6.680 | 1.00 | 31.28 | C |
| ATOM | 733 | N | TYR | C | 94 | 3.897 | 66.006 | 6.045 | 1.00 | 33.45 | N |
| ATOM | 734 | CA | TYR | C | 94 | 3.184 | 67.159 | 5.518 | 1.00 | 37.19 | C |
| ATOM | 735 | C | TYR | C | 94 | 4.043 | 68.395 | 5.707 | 1.00 | 36.22 | C |
| ATOM | 736 | O | TYR | C | 94 | 4.511 | 68.654 | 6.820 | 1.00 | 37.12 | O |
| ATOM | 737 | CB | TYR | C | 94 | 1.826 | 67.335 | 6.216 | 1.00 | 29.33 | C |
| ATOM | 738 | CG | TYR | C | 94 | 0.818 | 66.349 | 5.708 | 1.00 | 38.48 | C |
| ATOM | 739 | CD1 | TYR | C | 94 | 0.931 | 64.986 | 6.003 | 1.00 | 33.38 | C |
| ATOM | 740 | CD2 | TYR | C | 94 | -0.223 | 66.760 | 4.887 | 1.00 | 34.11 | C |
| ATOM | 741 | CE1 | TYR | C | 94 | 0.012 | 64.069 | 5.508 | 1.00 | 35.06 | C |
| ATOM | 742 | CE2 | TYR | C | 94 | -1.143 | 65.848 | 4.386 | 1.00 | 32.26 | C |
| ATOM | 743 | CZ | TYR | C | 94 | -1.033 | 64.510 | 4.706 | 1.00 | 31.31 | C |
| ATOM | 744 | OH | TYR | C | 94 | -1.955 | 63.614 | 4.184 | 1.00 | 29.76 | O |
| ATOM | 745 | N | TYR | C | 95 | 4.240 | 69.154 | 4.625 | 1.00 | 33.06 | N |
| ATOM | 746 | CA | TYR | C | 95 | 4.988 | 70.408 | 4.660 | 1.00 | 37.25 | C |
| ATOM | 747 | C | TYR | C | 95 | 4.091 | 71.583 | 4.292 | 1.00 | 38.95 | C |
| ATOM | 748 | O | TYR | C | 95 | 3.283 | 71.491 | 3.356 | 1.00 | 34.73 | O |
| ATOM | 749 | CB | TYR | C | 95 | 6.160 | 70.409 | 3.680 | 1.00 | 33.44 | C |
| ATOM | 750 | CG | TYR | C | 95 | 7.187 | 69.311 | 3.833 | 1.00 | 36.03 | C |
| ATOM | 751 | CD1 | TYR | C | 95 | 8.260 | 69.444 | 4.718 | 1.00 | 30.51 | C |
| ATOM | 752 | CD2 | TYR | C | 95 | 7.124 | 68.165 | 3.036 | 1.00 | 34.65 | C |
| ATOM | 753 | CE1 | TYR | C | 95 | 9.231 | 68.431 | 4.825 | 1.00 | 35.04 | C |
| ATOM | 754 | CE2 | TYR | C | 95 | 8.078 | 67.163 | 3.132 | 1.00 | 35.40 | C |
| ATOM | 755 | CZ | TYR | C | 95 | 9.124 | 67.295 | 4.023 | 1.00 | 36.07 | C |
| ATOM | 756 | OH | TYR | C | 95 | 10.054 | 66.281 | 4.097 | 1.00 | 43.55 | O |
| ATOM | 757 | N | CYS | C | 96 | 4.277 | 72.713 | 4.969 | 1.00 | 32.29 | N |
| ATOM | 758 | CA | CYS | C | 96 | 3.722 | 73.935 | 4.419 | 1.00 | 35.55 | C |
| ATOM | 759 | C | CYS | C | 96 | 4.819 | 74.668 | 3.674 | 1.00 | 34.71 | C |
| ATOM | 760 | O | CYS | C | 96 | 6.007 | 74.463 | 3.919 | 1.00 | 37.92 | O |
| ATOM | 761 | CB | CYS | C | 96 | 3.090 | 74.846 | 5.484 | 1.00 | 37.18 | C |
| ATOM | 762 | SG | CYS | C | 96 | 4.149 | 75.311 | 6.821 | 1.00 | 55.09 | S |
| ATOM | 763 | N | ALA | C | 97 | 4.391 | 75.493 | 2.720 | 1.00 | 37.06 | N |
| ATOM | 764 | CA | ALA | C | 97 | 5.252 | 76.388 | 1.965 | 1.00 | 33.86 | C |
| ATOM | 765 | C | ALA | C | 97 | 4.453 | 77.646 | 1.659 | 1.00 | 36.16 | C |
| ATOM | 766 | O | ALA | C | 97 | 3.224 | 77.620 | 1.638 | 1.00 | 37.96 | O |
| ATOM | 767 | CB | ALA | C | 97 | 5.744 | 75.736 | 0.667 | 1.00 | 31.03 | C |
| ATOM | 768 | N | ASN | C | 98 | 5.141 | 78.756 | 1.411 | 1.00 | 36.24 | N |
| ATOM | 769 | CA | ASN | C | 98 | 4.402 | 79.933 | 0.984 | 1.00 | 36.16 | C |
| ATOM | 770 | C | ASN | C | 98 | 4.643 | 80.154 | -0.505 | 1.00 | 33.85 | C |
| ATOM | 771 | O | ASN | C | 98 | 5.379 | 79.405 | -1.150 | 1.00 | 34.25 | O |
| ATOM | 772 | CB | ASN | C | 98 | 4.750 | 81.159 | 1.836 | 1.00 | 33.87 | C |
| ATOM | 773 | CG | ASN | C | 98 | 6.152 | 81.689 | 1.602 | 1.00 | 36.19 | C |
| ATOM | 774 | OD1 | ASN | C | 98 | 6.912 | 81.172 | 0.781 | 1.00 | 35.81 | O |
| ATOM | 775 | ND2 | ASN | C | 98 | 6.490 | 82.759 | 2.312 | 1.00 | 32.46 | N |
| ATOM | 776 | N | TRP | C | 99 | 4.001 | 81.179 | -1.060 | 1.00 | 29.34 | N |
| ATOM | 777 | CA | TRP | C | 99 | 4.253 | 81.534 | -2.451 | 1.00 | 32.42 | C |
| ATOM | 778 | C | TRP | C | 99 | 4.141 | 83.043 | -2.609 | 1.00 | 34.35 | C |
| ATOM | 779 | O | TRP | C | 99 | 3.294 | 83.677 | -1.978 | 1.00 | 36.87 | O |

| | | | | | | | | | | |
|---|---|---|---|---|---|---|---|---|---|---|
| ATOM | 780 | CB | TRP C | 99 | 3.301 | 80.808 | -3.409 | 1.00 | 31.13 | C |
| ATOM | 781 | CG | TRP C | 99 | 1.832 | 81.172 | -3.311 | 1.00 | 38.60 | C |
| ATOM | 782 | CD1 | TRP C | 99 | 0.869 | 80.545 | -2.558 | 1.00 | 36.35 | C |
| ATOM | 783 | CD2 | TRP C | 99 | 1.160 | 82.217 | -4.028 | 1.00 | 33.13 | C |
| ATOM | 784 | NE1 | TRP C | 99 | -0.359 | 81.151 | -2.754 | 1.00 | 32.28 | N |
| ATOM | 785 | CE2 | TRP C | 99 | -0.207 | 82.175 | -3.653 | 1.00 | 36.97 | C |
| ATOM | 786 | CE3 | TRP C | 99 | 1.580 | 83.187 | -4.949 | 1.00 | 33.81 | C |
| ATOM | 787 | CZ2 | TRP C | 99 | -1.155 | 83.078 | -4.160 | 1.00 | 35.91 | C |
| ATOM | 788 | CZ3 | TRP C | 99 | 0.639 | 84.078 | -5.459 | 1.00 | 33.25 | C |
| ATOM | 789 | CH2 | TRP C | 99 | -0.712 | 84.016 | -5.059 | 1.00 | 34.18 | C |
| ATOM | 790 | N | TYR C | 100 | 5.011 | 83.606 | -3.443 | 1.00 | 35.97 | N |
| ATOM | 791 | CA | TYR C | 100 | 5.148 | 85.048 | -3.651 | 1.00 | 34.82 | C |
| ATOM | 792 | C | TYR C | 100 | 4.693 | 85.512 | -5.022 | 1.00 | 35.71 | C |
| ATOM | 793 | O | TYR C | 100 | 4.101 | 86.591 | -5.143 | 1.00 | 33.01 | O |
| ATOM | 794 | CB | TYR C | 100 | 6.615 | 85.472 | -3.493 | 1.00 | 28.22 | C |
| ATOM | 795 | CG | TYR C | 100 | 7.184 | 85.286 | -2.114 | 1.00 | 36.73 | C |
| ATOM | 796 | CD1 | TYR C | 100 | 6.415 | 85.536 | -0.971 | 1.00 | 34.55 | C |
| ATOM | 797 | CD2 | TYR C | 100 | 8.496 | 84.852 | -1.946 | 1.00 | 35.48 | C |
| ATOM | 798 | CE1 | TYR C | 100 | 6.951 | 85.365 | 0.299 | 1.00 | 36.55 | C |
| ATOM | 799 | CE2 | TYR C | 100 | 9.032 | 84.666 | -0.684 | 1.00 | 33.46 | C |
| ATOM | 800 | CZ | TYR C | 100 | 8.263 | 84.929 | 0.430 | 1.00 | 36.36 | C |
| ATOM | 801 | OH | TYR C | 100 | 8.813 | 84.749 | 1.674 | 1.00 | 36.68 | O |
| ATOM | 802 | N | TYR C | 101 | 4.990 | 84.731 | -6.059 | 1.00 | 32.01 | N |
| ATOM | 803 | CA | TYR C | 101 | 4.927 | 85.197 | -7.439 | 1.00 | 35.14 | C |
| ATOM | 804 | C | TYR C | 101 | 3.758 | 84.539 | -8.159 | 1.00 | 31.46 | C |
| ATOM | 805 | O | TYR C | 101 | 2.795 | 85.215 | -8.492 | 1.00 | 32.52 | O |
| ATOM | 806 | CB | TYR C | 101 | 6.249 | 84.921 | -8.157 | 1.00 | 25.12 | C |
| ATOM | 807 | CG | TYR C | 101 | 7.430 | 85.482 | -7.409 | 1.00 | 31.29 | C |
| ATOM | 808 | CD1 | TYR C | 101 | 7.594 | 86.850 | -7.307 | 1.00 | 30.52 | C |
| ATOM | 809 | CD2 | TYR C | 101 | 8.385 | 84.653 | -6.796 | 1.00 | 34.01 | C |
| ATOM | 810 | CE1 | TYR C | 101 | 8.646 | 87.393 | -6.644 | 1.00 | 31.83 | C |
| ATOM | 811 | CE2 | TYR C | 101 | 9.470 | 85.205 | -6.099 | 1.00 | 29.33 | C |
| ATOM | 812 | CZ | TYR C | 101 | 9.586 | 86.589 | -6.042 | 1.00 | 34.04 | C |
| ATOM | 813 | OH | TYR C | 101 | 10.613 | 87.238 | -5.396 | 1.00 | 38.85 | O |
| ATOM | 814 | N | TYR C | 102 | 3.818 | 83.241 | -8.407 | 1.00 | 32.18 | N |
| ATOM | 815 | CA | TYR C | 102 | 2.692 | 82.528 | -8.984 | 1.00 | 26.83 | C |
| ATOM | 816 | C | TYR C | 102 | 2.185 | 81.474 | -8.000 | 1.00 | 34.57 | C |
| ATOM | 817 | O | TYR C | 102 | 2.959 | 80.888 | -7.237 | 1.00 | 30.16 | O |
| ATOM | 818 | CB | TYR C | 102 | 3.069 | 81.916 | -10.328 | 1.00 | 26.41 | C |
| ATOM | 819 | CG | TYR C | 102 | 4.422 | 81.207 | -10.424 | 1.00 | 31.59 | C |
| ATOM | 820 | CD1 | TYR C | 102 | 5.586 | 81.894 | -10.776 | 1.00 | 25.87 | C |
| ATOM | 821 | CD2 | TYR C | 102 | 4.510 | 79.827 | -10.218 | 1.00 | 34.03 | C |
| ATOM | 822 | CE1 | TYR C | 102 | 6.807 | 81.225 | -10.900 | 1.00 | 28.32 | C |
| ATOM | 823 | CE2 | TYR C | 102 | 5.708 | 79.150 | -10.339 | 1.00 | 29.44 | C |
| ATOM | 824 | CZ | TYR C | 102 | 6.856 | 79.842 | -10.677 | 1.00 | 37.60 | C |
| ATOM | 825 | OH | TYR C | 102 | 8.030 | 79.125 | -10.804 | 1.00 | 31.27 | O |
| ATOM | 826 | N | TYR C | 103 | 0.861 | 81.255 | -8.005 | 1.00 | 34.62 | N |
| ATOM | 827 | CA | TYR C | 103 | 0.206 | 80.545 | -6.908 | 1.00 | 30.24 | C |
| ATOM | 828 | C | TYR C | 103 | 0.561 | 79.069 | -6.860 | 1.00 | 33.90 | C |
| ATOM | 829 | O | TYR C | 103 | 0.334 | 78.431 | -5.826 | 1.00 | 33.35 | O |
| ATOM | 830 | CB | TYR C | 103 | -1.330 | 80.708 | -6.981 | 1.00 | 32.59 | C |
| ATOM | 831 | CG | TYR C | 103 | -1.968 | 80.050 | -8.196 | 1.00 | 30.66 | C |
| ATOM | 832 | CD1 | TYR C | 103 | -2.097 | 80.744 | -9.398 | 1.00 | 32.62 | C |
| ATOM | 833 | CD2 | TYR C | 103 | -2.439 | 78.739 | -8.145 | 1.00 | 31.75 | C |
| ATOM | 834 | CE1 | TYR C | 103 | -2.677 | 80.151 | -10.526 | 1.00 | 34.17 | C |
| ATOM | 835 | CE2 | TYR C | 103 | -3.030 | 78.132 | -9.277 | 1.00 | 35.45 | C |
| ATOM | 836 | CZ | TYR C | 103 | -3.140 | 78.851 | -10.460 | 1.00 | 38.01 | C |
| ATOM | 837 | OH | TYR C | 103 | -3.707 | 78.286 | -11.585 | 1.00 | 43.93 | O |
| ATOM | 838 | N | TYR C | 104 | 1.090 | 78.503 | -7.941 | 1.00 | 33.41 | N |
| ATOM | 839 | CA | TYR C | 104 | 1.431 | 77.088 | -7.959 | 1.00 | 31.54 | C |
| ATOM | 840 | C | TYR C | 104 | 2.921 | 76.846 | -7.775 | 1.00 | 35.80 | C |
| ATOM | 841 | O | TYR C | 104 | 3.383 | 75.706 | -7.933 | 1.00 | 34.83 | O |
| ATOM | 842 | CB | TYR C | 104 | 0.942 | 76.437 | -9.254 | 1.00 | 31.39 | C |
| ATOM | 843 | CG | TYR C | 104 | 1.310 | 77.192 | -10.505 | 1.00 | 35.92 | C |
| ATOM | 844 | CD1 | TYR C | 104 | 2.537 | 76.979 | -11.134 | 1.00 | 29.90 | C |
| ATOM | 845 | CD2 | TYR C | 104 | 0.431 | 78.136 | -11.058 | 1.00 | 37.72 | C |
| ATOM | 846 | CE1 | TYR C | 104 | 2.887 | 77.682 | -12.297 | 1.00 | 31.96 | C |
| ATOM | 847 | CE2 | TYR C | 104 | 0.773 | 78.853 | -12.216 | 1.00 | 33.33 | C |
| ATOM | 848 | CZ | TYR C | 104 | 1.997 | 78.611 | -12.832 | 1.00 | 37.08 | C |
| ATOM | 849 | OH | TYR C | 104 | 2.322 | 79.290 | -13.988 | 1.00 | 38.85 | O |
| ATOM | 850 | N | GLY C | 105 | 3.677 | 77.881 | -7.424 | 1.00 | 30.57 | N |

| ATOM | 851 | CA  | GLY | C | 105 | 5.069  | 77.729 | -7.046 | 1.00 | 31.29 | C   |
|------|-----|-----|-----|---|-----|--------|--------|--------|------|-------|-----|
| ATOM | 852 | C   | GLY | C | 105 | 5.189  | 77.804 | -5.536 | 1.00 | 35.97 | C   |
| ATOM | 853 | O   | GLY | C | 105 | 4.381  | 78.439 | -4.880 | 1.00 | 39.78 | O   |
| ATOM | 854 | N   | MET | C | 106 | 6.174  | 77.104 | -4.983 | 1.00 | 35.79 | N   |
| ATOM | 855 | CA  | MET | C | 106 | 6.477  | 77.149 | -3.560 | 1.00 | 29.65 | C   |
| ATOM | 856 | C   | MET | C | 106 | 7.811  | 77.844 | -3.359 | 1.00 | 34.76 | C   |
| ATOM | 857 | O   | MET | C | 106 | 8.814  | 77.447 | -3.964 | 1.00 | 40.34 | O   |
| ATOM | 858 | CB  | MET | C | 106 | 6.531  | 75.749 | -2.967 | 1.00 | 33.58 | C   |
| ATOM | 859 | CG  | MET | C | 106 | 5.200  | 75.079 | -2.997 | 1.00 | 40.63 | C   |
| ATOM | 860 | SD  | MET | C | 106 | 5.175  | 73.827 | -4.259 | 1.00 | 38.99 | S   |
| ATOM | 861 | CE  | MET | C | 106 | 3.449  | 73.894 | -4.753 | 1.00 | 39.97 | C   |
| ATOM | 862 | N   | ASP | C | 107 | 7.833  | 78.872 | -2.510 | 1.00 | 32.14 | N   |
| ATOM | 863 | CA  | ASP | C | 107 | 9.038  | 79.679 | -2.359 | 1.00 | 36.28 | C   |
| ATOM | 864 | C   | ASP | C | 107 | 9.814  | 79.363 | -1.087 | 1.00 | 36.05 | C   |
| ATOM | 865 | O   | ASP | C | 107 | 10.987 | 79.000 | -1.168 | 1.00 | 43.22 | O   |
| ATOM | 866 | CB  | ASP | C | 107 | 8.684  | 81.166 | -2.419 | 1.00 | 35.85 | C   |
| ATOM | 867 | CG  | ASP | C | 107 | 8.243  | 81.582 | -3.791 | 1.00 | 36.58 | C   |
| ATOM | 868 | OD1 | ASP | C | 107 | 9.126  | 81.733 | -4.663 | 1.00 | 39.74 | O1- |
| ATOM | 869 | OD2 | ASP | C | 107 | 7.024  | 81.741 | -4.005 | 1.00 | 38.36 | O   |
| ATOM | 870 | N   | VAL | C | 108 | 9.185  | 79.486 | 0.079  | 1.00 | 34.27 | N   |
| ATOM | 871 | CA  | VAL | C | 108 | 9.795  | 79.131 | 1.354  | 1.00 | 37.63 | C   |
| ATOM | 872 | C   | VAL | C | 108 | 9.081  | 77.906 | 1.893  | 1.00 | 35.71 | C   |
| ATOM | 873 | O   | VAL | C | 108 | 7.853  | 77.818 | 1.820  | 1.00 | 36.94 | O   |
| ATOM | 874 | CB  | VAL | C | 108 | 9.716  | 80.275 | 2.375  | 1.00 | 39.13 | C   |
| ATOM | 875 | CG1 | VAL | C | 108 | 10.395 | 79.854 | 3.662  | 1.00 | 34.94 | C   |
| ATOM | 876 | CG2 | VAL | C | 108 | 10.334 | 81.534 | 1.809  | 1.00 | 37.63 | C   |
| ATOM | 877 | N   | TRP | C | 109 | 9.845  | 76.969 | 2.442  | 1.00 | 35.86 | N   |
| ATOM | 878 | CA  | TRP | C | 109 | 9.305  | 75.713 | 2.950  | 1.00 | 38.43 | C   |
| ATOM | 879 | C   | TRP | C | 109 | 9.523  | 75.578 | 4.452  | 1.00 | 36.07 | C   |
| ATOM | 880 | O   | TRP | C | 109 | 10.470 | 76.127 | 5.011  | 1.00 | 34.83 | O   |
| ATOM | 881 | CB  | TRP | C | 109 | 9.935  | 74.510 | 2.270  | 1.00 | 28.64 | C   |
| ATOM | 882 | CG  | TRP | C | 109 | 9.627  | 74.379 | 0.834  | 1.00 | 32.20 | C   |
| ATOM | 883 | CD1 | TRP | C | 109 | 10.053 | 75.196 | -0.178 | 1.00 | 33.19 | C   |
| ATOM | 884 | CD2 | TRP | C | 109 | 8.857  | 73.338 | 0.215  | 1.00 | 30.88 | C   |
| ATOM | 885 | NE1 | TRP | C | 109 | 9.589  | 74.729 | -1.385 | 1.00 | 32.70 | N   |
| ATOM | 886 | CE2 | TRP | C | 109 | 8.853  | 73.589 | -1.171 | 1.00 | 32.73 | C   |
| ATOM | 887 | CE3 | TRP | C | 109 | 8.175  | 72.219 | 0.696  | 1.00 | 31.39 | C   |
| ATOM | 888 | CZ2 | TRP | C | 109 | 8.183  | 72.759 | -2.079 | 1.00 | 27.14 | C   |
| ATOM | 889 | CZ3 | TRP | C | 109 | 7.510  | 71.395 | -0.205 | 1.00 | 25.68 | C   |
| ATOM | 890 | CH2 | TRP | C | 109 | 7.532  | 71.661 | -1.575 | 1.00 | 25.11 | C   |
| ATOM | 891 | N   | GLY | C | 110 | 8.611  | 74.852 | 5.095  | 1.00 | 38.56 | N   |
| ATOM | 892 | CA  | GLY | C | 110 | 8.732  | 74.483 | 6.489  | 1.00 | 37.65 | C   |
| ATOM | 893 | C   | GLY | C | 110 | 9.578  | 73.243 | 6.689  | 1.00 | 38.90 | C   |
| ATOM | 894 | O   | GLY | C | 110 | 10.348 | 72.825 | 5.821  | 1.00 | 44.35 | O   |
| ATOM | 895 | N   | GLN | C | 111 | 9.417  | 72.638 | 7.864  | 1.00 | 37.08 | N   |
| ATOM | 896 | CA  | GLN | C | 111 | 10.257 | 71.526 | 8.265  | 1.00 | 37.72 | C   |
| ATOM | 897 | C   | GLN | C | 111 | 9.554  | 70.171 | 8.274  | 1.00 | 39.10 | C   |
| ATOM | 898 | O   | GLN | C | 111 | 10.241 | 69.143 | 8.268  | 1.00 | 46.62 | O   |
| ATOM | 899 | CB  | GLN | C | 111 | 10.854 | 71.823 | 9.646  | 1.00 | 41.49 | C   |
| ATOM | 900 | CG  | GLN | C | 111 | 9.971  | 71.407 | 10.795 | 1.00 | 57.57 | C   |
| ATOM | 901 | CD  | GLN | C | 111 | 10.317 | 72.142 | 12.083 | 1.00 | 71.42 | C   |
| ATOM | 902 | OE1 | GLN | C | 111 | 11.162 | 73.046 | 12.079 | 1.00 | 67.32 | O   |
| ATOM | 903 | NE2 | GLN | C | 111 | 9.662  | 71.761 | 13.194 | 1.00 | 62.29 | N   |
| ATOM | 904 | N   | GLY | C | 112 | 8.229  | 70.137 | 8.247  | 1.00 | 38.85 | N   |
| ATOM | 905 | CA  | GLY | C | 112 | 7.483  | 68.892 | 8.185  | 1.00 | 37.13 | C   |
| ATOM | 906 | C   | GLY | C | 112 | 6.892  | 68.477 | 9.528  | 1.00 | 40.61 | C   |
| ATOM | 907 | O   | GLY | C | 112 | 7.389  | 68.837 | 10.601 | 1.00 | 43.71 | O   |
| ATOM | 908 | N   | THR | C | 113 | 5.771  | 67.754 | 9.462  | 1.00 | 38.16 | N   |
| ATOM | 909 | CA  | THR | C | 113 | 5.125  | 67.066 | 10.574 | 1.00 | 37.93 | C   |
| ATOM | 910 | C   | THR | C | 113 | 4.592  | 65.777 | 9.967  | 1.00 | 40.18 | C   |
| ATOM | 911 | O   | THR | C | 113 | 4.106  | 65.791 | 8.834  | 1.00 | 43.97 | O   |
| ATOM | 912 | CB  | THR | C | 113 | 4.008  | 67.924 | 11.231 | 1.00 | 40.04 | C   |
| ATOM | 913 | OG1 | THR | C | 113 | 3.470  | 67.248 | 12.370 | 1.00 | 48.01 | O   |
| ATOM | 914 | CG2 | THR | C | 113 | 2.863  | 68.248 | 10.265 | 1.00 | 39.86 | C   |
| ATOM | 915 | N   | ATHR | C | 114 | 4.716  | 64.665 | 10.689 | 0.50 | 39.81 | N   |
| ATOM | 916 | CA  | ATHR | C | 114 | 4.418  | 63.359 | 10.110 | 0.50 | 39.05 | C   |
| ATOM | 917 | C   | ATHR | C | 114 | 3.038  | 62.873 | 10.545 | 0.50 | 39.83 | C   |
| ATOM | 918 | O   | ATHR | C | 114 | 2.650  | 63.015 | 11.709 | 0.50 | 39.84 | O   |
| ATOM | 919 | CB  | ATHR | C | 114 | 5.484  | 62.320 | 10.487 | 0.50 | 39.38 | C   |
| ATOM | 920 | OG1 | ATHR | C | 114 | 5.310  | 61.937 | 11.853 | 0.50 | 49.44 | O   |
| ATOM | 921 | CG2 | ATHR | C | 114 | 6.901  | 62.878 | 10.295 | 0.50 | 34.14 | C   |

```
ATOM    922  N   BTHR C 114      4.722  64.656  10.669  0.50 39.81           N
ATOM    923  CA  BTHR C 114      4.413  63.376  10.040  0.50 39.04           C
ATOM    924  C   BTHR C 114      3.083  62.819  10.539  0.50 39.82           C
ATOM    925  O   BTHR C 114      2.766  62.880  11.731  0.50 39.90           O
ATOM    926  CB  BTHR C 114      5.554  62.360  10.232  0.50 39.16           C
ATOM    927  OG1 BTHR C 114      5.037  61.021  10.248  0.50 33.47           O
ATOM    928  CG2 BTHR C 114      6.343  62.647  11.490  0.50 42.84           C
ATOM    929  N   VAL C 115       2.298  62.308   9.592  1.00 38.42           N
ATOM    930  CA  VAL C 115       0.962  61.778   9.817  1.00 36.25           C
ATOM    931  C   VAL C 115       0.968  60.298   9.446  1.00 37.55           C
ATOM    932  O   VAL C 115       1.310  59.940   8.313  1.00 39.55           O
ATOM    933  CB  VAL C 115      -0.092  62.536   8.991  1.00 37.32           C
ATOM    934  CG1 VAL C 115      -1.443  61.798   9.049  1.00 33.92           C
ATOM    935  CG2 VAL C 115      -0.223  63.961   9.492  1.00 31.70           C
ATOM    936  N   THR C 116       0.580  59.443  10.388  1.00 35.49           N
ATOM    937  CA  THR C 116       0.471  58.014  10.145  1.00 38.36           C
ATOM    938  C   THR C 116      -0.987  57.603  10.309  1.00 43.76           C
ATOM    939  O   THR C 116      -1.596  57.873  11.350  1.00 43.30           O
ATOM    940  CB  THR C 116       1.379  57.223  11.096  1.00 45.91           C
ATOM    941  OG1 THR C 116       2.734  57.660  10.937  1.00 47.40           O
ATOM    942  CG2 THR C 116       1.342  55.751  10.742  1.00 42.59           C
ATOM    943  N   VAL C 117      -1.553  56.977   9.283  1.00 36.12           N
ATOM    944  CA  VAL C 117      -2.926  56.501   9.346  1.00 41.60           C
ATOM    945  C   VAL C 117      -2.891  54.978   9.276  1.00 45.74           C
ATOM    946  O   VAL C 117      -2.634  54.394   8.211  1.00 44.01           O
ATOM    947  CB  VAL C 117      -3.813  57.107   8.244  1.00 42.69           C
ATOM    948  CG1 VAL C 117      -5.257  56.664   8.437  1.00 34.13           C
ATOM    949  CG2 VAL C 117      -3.720  58.666   8.235  1.00 38.65           C
ATOM    950  N   SER C 118      -3.181  54.330  10.407  1.00 42.18           N
ATOM    951  CA  SER C 118      -3.101  52.882  10.500  1.00 45.65           C
ATOM    952  C   SER C 118      -4.244  52.329  11.345  1.00 46.76           C
ATOM    953  O   SER C 118      -4.596  52.891  12.389  1.00 43.45           O
ATOM    954  CB  SER C 118      -1.764  52.442  11.101  1.00 45.42           C
ATOM    955  OG  SER C 118      -1.694  51.028  11.142  1.00 53.46           O
ATOM    956  N   SER C 119      -4.800  51.204  10.901  1.00 39.96           N
ATOM    957  CA  SER C 119      -5.765  50.456  11.697  1.00 55.76           C
ATOM    958  C   SER C 119      -5.145  49.241  12.384  1.00 58.97           C
ATOM    959  O   SER C 119      -5.879  48.417  12.940  1.00 56.42           O
ATOM    960  CB  SER C 119      -6.956  50.024  10.833  1.00 47.59           C
ATOM    961  OG  SER C 119      -6.537  49.473   9.592  1.00 60.90           O
ATOM    962  N   ALA C 120      -3.821  49.092  12.325  1.00 53.37           N
ATOM    963  CA  ALA C 120      -3.157  47.942  12.923  1.00 49.27           C
ATOM    964  C   ALA C 120      -3.115  48.063  14.439  1.00 53.61           C
ATOM    965  O   ALA C 120      -3.088  49.160  15.007  1.00 51.80           O
ATOM    966  CB  ALA C 120      -1.736  47.799  12.376  1.00 54.97           C
ATOM    967  N   SER C 121      -3.101  46.918  15.104  1.00 50.81      GZ00 N
ATOM    968  CA  SER C 121      -2.922  46.921  16.546  1.00 62.62      GZ00 C
ATOM    969  C   SER C 121      -1.817  45.945  16.925  1.00 55.18      GZ00 C
ATOM    970  O   SER C 121      -1.492  45.015  16.175  1.00 50.22      GZ00 O
ATOM    971  CB  SER C 121      -4.226  46.571  17.271  1.00 60.87      GZ00 C
ATOM    972  OG  SER C 121      -4.902  45.539  16.576  1.00 67.02      GZ00 O
ATOM    973  N   THR C 122      -1.276  46.170  18.123  1.00 43.63      GZ00 N
ATOM    974  CA  THR C 122      -0.125  45.444  18.640  1.00 49.19      GZ00 C
ATOM    975  C   THR C 122      -0.197  43.955  18.342  1.00 48.67      GZ00 C
ATOM    976  O   THR C 122      -1.207  43.300  18.591  1.00 54.63      GZ00 O
ATOM    977  CB  THR C 122      -0.014  45.661  20.147  1.00 51.65      GZ00 C
ATOM    978  OG1 THR C 122       0.017  47.074  20.426  1.00 49.30      GZ00 O
ATOM    979  CG2 THR C 122       1.260  44.988  20.690  1.00 49.88      GZ00 C
ATOM    980  N   LYS C 123       0.890  43.430  17.795  1.00 50.80      GZ00 N
ATOM    981  CA  LYS C 123       0.976  42.015  17.485  1.00 49.10      GZ00 C
ATOM    982  C   LYS C 123       2.446  41.622  17.506  1.00 56.41      GZ00 C
ATOM    983  O   LYS C 123       3.290  42.358  16.985  1.00 49.74      GZ00 O
ATOM    984  CB  LYS C 123       0.338  41.693  16.133  1.00 44.99      GZ00 C
ATOM    985  CG  LYS C 123       0.499  40.238  15.807  1.00 44.28      GZ00 C
ATOM    986  CD  LYS C 123      -0.078  39.820  14.484  1.00 49.15      GZ00 C
ATOM    987  CE  LYS C 123       0.361  38.368  14.217  1.00 62.00      GZ00 C
ATOM    988  NZ  LYS C 123      -0.182  37.795  12.951  1.00 72.41      GZ00 N1+
ATOM    989  N   GLY C 124       2.752  40.507  18.178  1.00 53.97      GZ00 N
ATOM    990  CA  GLY C 124       4.094  39.983  18.217  1.00 39.15      GZ00 C
ATOM    991  C   GLY C 124       4.382  39.194  16.962  1.00 43.80      GZ00 C
ATOM    992  O   GLY C 124       3.474  38.686  16.297  1.00 46.60      GZ00 O
```

282

| ATOM | 993 | N | PRO C 125 | 5.658 | 39.060 | 16.625 | 1.00 47.07 | GZ00 N |
|------|------|-----|-----------|--------|--------|--------|------------|--------|
| ATOM | 994 | CA | PRO C 125 | 6.043 | 38.419 | 15.362 | 1.00 48.03 | GZ00 C |
| ATOM | 995 | C | PRO C 125 | 6.129 | 36.902 | 15.443 | 1.00 43.83 | GZ00 C |
| ATOM | 996 | O | PRO C 125 | 6.341 | 36.326 | 16.503 | 1.00 45.86 | GZ00 O |
| ATOM | 997 | CB | PRO C 125 | 7.440 | 38.999 | 15.095 | 1.00 45.07 | GZ00 C |
| ATOM | 998 | CG | PRO C 125 | 7.989 | 39.254 | 16.464 | 1.00 48.96 | GZ00 C |
| ATOM | 999 | CD | PRO C 125 | 6.800 | 39.689 | 17.314 | 1.00 46.04 | GZ00 C |
| ATOM | 1000 | N | SER C 126 | 5.963 | 36.270 | 14.280 | 1.00 42.83 | GZ00 N |
| ATOM | 1001 | CA | SER C 126 | 6.393 | 34.900 | 14.053 | 1.00 46.91 | GZ00 C |
| ATOM | 1002 | C | SER C 126 | 7.817 | 34.900 | 13.498 | 1.00 49.79 | GZ00 C |
| ATOM | 1003 | O | SER C 126 | 8.157 | 35.713 | 12.635 | 1.00 45.82 | GZ00 O |
| ATOM | 1004 | CB | SER C 126 | 5.461 | 34.192 | 13.071 | 1.00 50.64 | GZ00 C |
| ATOM | 1005 | OG | SER C 126 | 4.145 | 34.158 | 13.572 | 1.00 58.04 | GZ00 O |
| ATOM | 1006 | N | VAL C 127 | 8.645 | 33.980 | 13.986 | 1.00 49.68 | GZ00 N |
| ATOM | 1007 | CA | VAL C 127 | 10.059 | 33.915 | 13.624 | 1.00 45.00 | GZ00 C |
| ATOM | 1008 | C | VAL C 127 | 10.335 | 32.584 | 12.950 | 1.00 42.10 | GZ00 C |
| ATOM | 1009 | O | VAL C 127 | 10.021 | 31.528 | 13.504 | 1.00 57.42 | GZ00 O |
| ATOM | 1010 | CB | VAL C 127 | 10.958 | 34.110 | 14.855 | 1.00 44.31 | GZ00 C |
| ATOM | 1011 | CG1 | VAL C 127 | 12.431 | 34.119 | 14.456 | 1.00 47.62 | GZ00 C |
| ATOM | 1012 | CG2 | VAL C 127 | 10.577 | 35.406 | 15.554 | 1.00 41.45 | GZ00 C |
| ATOM | 1013 | N | PHE C 128 | 10.912 | 32.635 | 11.758 | 1.00 43.32 | GZ00 N |
| ATOM | 1014 | CA | PHE C 128 | 11.244 | 31.451 | 10.983 | 1.00 44.87 | GZ00 C |
| ATOM | 1015 | C | PHE C 128 | 12.729 | 31.447 | 10.629 | 1.00 50.79 | GZ00 C |
| ATOM | 1016 | O | PHE C 128 | 13.327 | 32.511 | 10.435 | 1.00 47.46 | GZ00 O |
| ATOM | 1017 | CB | PHE C 128 | 10.425 | 31.399 | 9.694 | 1.00 48.10 | GZ00 C |
| ATOM | 1018 | CG | PHE C 128 | 8.942 | 31.384 | 9.922 | 1.00 58.19 | GZ00 C |
| ATOM | 1019 | CD1 | PHE C 128 | 8.272 | 30.200 | 10.176 | 1.00 58.46 | GZ00 C |
| ATOM | 1020 | CD2 | PHE C 128 | 8.220 | 32.566 | 9.912 | 1.00 56.25 | GZ00 C |
| ATOM | 1021 | CE1 | PHE C 128 | 6.912 | 30.198 | 10.387 | 1.00 58.84 | GZ00 C |
| ATOM | 1022 | CE2 | PHE C 128 | 6.855 | 32.568 | 10.128 | 1.00 55.91 | GZ00 C |
| ATOM | 1023 | CZ | PHE C 128 | 6.205 | 31.387 | 10.358 | 1.00 55.09 | GZ00 C |
| ATOM | 1024 | N | PRO C 129 | 13.360 | 30.275 | 10.560 | 1.00 52.29 | GZ00 N |
| ATOM | 1025 | CA | PRO C 129 | 14.779 | 30.230 | 10.200 | 1.00 40.71 | GZ00 C |
| ATOM | 1026 | C | PRO C 129 | 14.975 | 30.363 | 8.705 | 1.00 44.60 | GZ00 C |
| ATOM | 1027 | O | PRO C 129 | 14.205 | 29.819 | 7.907 | 1.00 48.43 | GZ00 O |
| ATOM | 1028 | CB | PRO C 129 | 15.218 | 28.840 | 10.681 | 1.00 53.19 | GZ00 C |
| ATOM | 1029 | CG | PRO C 129 | 13.998 | 28.000 | 10.486 | 1.00 46.32 | GZ00 C |
| ATOM | 1030 | CD | PRO C 129 | 12.829 | 28.925 | 10.840 | 1.00 50.96 | GZ00 C |
| ATOM | 1031 | N | LEU C 130 | 16.017 | 31.101 | 8.330 | 1.00 39.68 | GZ00 N |
| ATOM | 1032 | CA | LEU C 130 | 16.463 | 31.190 | 6.947 | 1.00 38.79 | GZ00 C |
| ATOM | 1033 | C | LEU C 130 | 17.725 | 30.330 | 6.838 | 1.00 45.86 | GZ00 C |
| ATOM | 1034 | O | LEU C 130 | 18.834 | 30.778 | 7.150 | 1.00 44.51 | GZ00 O |
| ATOM | 1035 | CB | LEU C 130 | 16.699 | 32.647 | 6.568 | 1.00 41.81 | GZ00 C |
| ATOM | 1036 | CG | LEU C 130 | 15.436 | 33.491 | 6.795 | 1.00 45.70 | GZ00 C |
| ATOM | 1037 | CD1 | LEU C 130 | 15.639 | 34.968 | 6.444 | 1.00 40.24 | GZ00 C |
| ATOM | 1038 | CD2 | LEU C 130 | 14.275 | 32.910 | 6.003 | 1.00 44.72 | GZ00 C |
| ATOM | 1039 | N | ALA C 131 | 17.552 | 29.096 | 6.402 | 1.00 46.13 | GZ00 N |
| ATOM | 1040 | CA | ALA C 131 | 18.618 | 28.113 | 6.528 | 1.00 43.66 | GZ00 C |
| ATOM | 1041 | C | ALA C 131 | 19.672 | 28.301 | 5.441 | 1.00 44.63 | GZ00 C |
| ATOM | 1042 | O | ALA C 131 | 19.339 | 28.524 | 4.278 | 1.00 46.42 | GZ00 O |
| ATOM | 1043 | CB | ALA C 131 | 18.060 | 26.703 | 6.457 | 1.00 36.60 | GZ00 C |
| ATOM | 1044 | N | PRO C 132 | 20.943 | 28.159 | 5.792 | 1.00 50.91 | GZ00 N |
| ATOM | 1045 | CA | PRO C 132 | 22.009 | 28.205 | 4.784 | 1.00 52.92 | GZ00 C |
| ATOM | 1046 | C | PRO C 132 | 22.027 | 26.951 | 3.921 | 1.00 63.10 | GZ00 C |
| ATOM | 1047 | O | PRO C 132 | 21.557 | 25.876 | 4.315 | 1.00 65.83 | GZ00 O |
| ATOM | 1048 | CB | PRO C 132 | 23.279 | 28.301 | 5.631 | 1.00 51.34 | GZ00 C |
| ATOM | 1049 | CG | PRO C 132 | 22.910 | 27.548 | 6.901 | 1.00 47.48 | GZ00 C |
| ATOM | 1050 | CD | PRO C 132 | 21.454 | 27.876 | 7.145 | 1.00 49.36 | GZ00 C |
| ATOM | 1051 | N | SER C 133 | 22.606 | 27.103 | 2.724 | 1.00 65.77 | GZ00 N |
| ATOM | 1052 | CA | SER C 133 | 22.642 | 26.041 | 1.718 | 1.00 75.34 | GZ00 C |
| ATOM | 1053 | C | SER C 133 | 23.713 | 26.317 | 0.668 | 1.00 79.20 | GZ00 C |
| ATOM | 1054 | O | SER C 133 | 24.893 | 26.462 | 0.990 | 1.00 81.66 | GZ00 O |
| ATOM | 1055 | CB | SER C 133 | 21.272 | 25.900 | 1.043 | 1.00 74.63 | GZ00 C |
| ATOM | 1056 | OG | SER C 133 | 20.727 | 27.170 | 0.711 | 1.00 72.62 | GZ00 O |
| ATOM | 1057 | N | SER C 134 | 23.317 | 26.372 | -0.599 | 1.00 91.08 | GZ00 N |
| ATOM | 1058 | CA | SER C 134 | 24.136 | 27.032 | -1.612 | 1.00 84.25 | GZ00 C |
| ATOM | 1059 | C | SER C 134 | 23.720 | 28.503 | -1.692 | 1.00 85.59 | GZ00 C |
| ATOM | 1060 | O | SER C 134 | 23.292 | 29.031 | -2.718 | 1.00 89.34 | GZ00 O |
| ATOM | 1061 | CB | SER C 134 | 24.022 | 26.312 | -2.950 | 1.00 90.76 | GZ00 C |
| ATOM | 1062 | OG | SER C 134 | 24.604 | 25.016 | -2.867 | 1.00 71.01 | GZ00 O |
| ATOM | 1063 | N | SER C 136 | 23.766 | 29.125 | -0.514 | 1.00 87.58 | GZ00 N |

```
ATOM   1064  CA  SER C 136    23.877  30.568  -0.336  1.00 76.32        GZ00 C
ATOM   1065  C   SER C 136    25.274  30.904   0.199  1.00 70.77        GZ00 C
ATOM   1066  O   SER C 136    25.441  31.713   1.121  1.00 56.45        GZ00 O
ATOM   1067  CB  SER C 136    22.777  31.098   0.588  1.00 64.27        GZ00 C
ATOM   1068  OG  SER C 136    22.863  30.567   1.905  1.00 55.15        GZ00 O
ATOM   1069  N   THR C 137    26.294  30.248  -0.363  1.00 68.34        GZ00 N
ATOM   1070  CA  THR C 137    27.686  30.490  -0.008  1.00 69.91        GZ00 C
ATOM   1071  C   THR C 137    28.416  31.190  -1.157  1.00 67.19        GZ00 C
ATOM   1072  O   THR C 137    28.180  30.891  -2.332  1.00 69.81        GZ00 O
ATOM   1073  CB  THR C 137    28.407  29.183   0.387  1.00 64.52        GZ00 C
ATOM   1074  OG1 THR C 137    29.096  28.626  -0.739  1.00 77.04        GZ00 O
ATOM   1075  CG2 THR C 137    27.427  28.167   0.946  1.00 58.14        GZ00 C
ATOM   1076  N   SER C 138    29.289  32.144  -0.810  1.00 68.23        GZ00 N
ATOM   1077  CA  SER C 138    30.030  32.950  -1.784  1.00 66.23        GZ00 C
ATOM   1078  C   SER C 138    31.526  32.891  -1.454  1.00 65.41        GZ00 C
ATOM   1079  O   SER C 138    32.054  33.661  -0.629  1.00 62.93        GZ00 O
ATOM   1080  CB  SER C 138    29.508  34.383  -1.828  1.00 70.26        GZ00 C
ATOM   1081  OG  SER C 138    29.183  34.732  -3.162  1.00 77.29        GZ00 O
ATOM   1082  N   GLY C 139    32.210  31.988  -2.153  1.00 65.74        GZ00 N
ATOM   1083  CA  GLY C 139    33.577  31.673  -1.822  1.00 55.52        GZ00 C
ATOM   1084  C   GLY C 139    33.581  30.952  -0.495  1.00 52.92        GZ00 C
ATOM   1085  O   GLY C 139    32.963  29.889  -0.339  1.00 56.98        GZ00 O
ATOM   1086  N   GLY C 140    34.241  31.550   0.483  1.00 39.11        GZ00 N
ATOM   1087  CA  GLY C 140    34.394  30.917   1.767  1.00 37.84        GZ00 C
ATOM   1088  C   GLY C 140    33.435  31.476   2.782  1.00 35.31        GZ00 C
ATOM   1089  O   GLY C 140    33.686  31.410   3.987  1.00 35.90        GZ00 O
ATOM   1090  N   THR C 141    32.312  31.990   2.314  1.00 30.60        GZ00 N
ATOM   1091  CA  THR C 141    31.371  32.678   3.180  1.00 35.07        GZ00 C
ATOM   1092  C   THR C 141    29.975  32.121   2.959  1.00 45.03        GZ00 C
ATOM   1093  O   THR C 141    29.638  31.698   1.858  1.00 45.95        GZ00 O
ATOM   1094  CB  THR C 141    31.469  34.196   2.898  1.00 34.50        GZ00 C
ATOM   1095  OG1 THR C 141    31.995  34.851   4.055  1.00 45.09        GZ00 O
ATOM   1096  CG2 THR C 141    30.182  34.807   2.446  1.00 40.84        GZ00 C
ATOM   1097  N   ALA C 142    29.186  32.058   4.026  1.00 40.91        GZ00 N
ATOM   1098  CA  ALA C 142    27.833  31.528   3.943  1.00 37.80        GZ00 C
ATOM   1099  C   ALA C 142    26.893  32.457   4.683  1.00 34.33        GZ00 C
ATOM   1100  O   ALA C 142    27.251  33.006   5.730  1.00 37.65        GZ00 O
ATOM   1101  CB  ALA C 142    27.729  30.125   4.539  1.00 37.82        GZ00 C
ATOM   1102  N   ALA C 143    25.685  32.608   4.148  1.00 31.94        GZ00 N
ATOM   1103  CA  ALA C 143    24.649  33.451   4.731  1.00 31.30        GZ00 C
ATOM   1104  C   ALA C 143    23.527  32.602   5.324  1.00 38.60        GZ00 C
ATOM   1105  O   ALA C 143    23.079  31.624   4.715  1.00 36.49        GZ00 O
ATOM   1106  CB  ALA C 143    24.062  34.401   3.689  1.00 33.71        GZ00 C
ATOM   1107  N   LEU C 144    23.099  32.976   6.529  1.00 37.05        GZ00 N
ATOM   1108  CA  LEU C 144    21.975  32.361   7.208  1.00 40.29        GZ00 C
ATOM   1109  C   LEU C 144    21.227  33.458   7.957  1.00 40.87        GZ00 C
ATOM   1110  O   LEU C 144    21.800  34.504   8.281  1.00 41.49        GZ00 O
ATOM   1111  CB  LEU C 144    22.463  31.247   8.144  1.00 42.35        GZ00 C
ATOM   1112  CG  LEU C 144    23.403  31.747   9.239  1.00 47.05        GZ00 C
ATOM   1113  CD1 LEU C 144    22.647  31.926  10.545  1.00 49.65        GZ00 C
ATOM   1114  CD2 LEU C 144    24.583  30.834   9.410  1.00 40.72        GZ00 C
ATOM   1115  N   GLY C 145    19.949  33.219   8.252  1.00 37.19        GZ00 N
ATOM   1116  CA  GLY C 145    19.181  34.289   8.851  1.00 39.70        GZ00 C
ATOM   1117  C   GLY C 145    17.884  33.886   9.522  1.00 40.16        GZ00 C
ATOM   1118  O   GLY C 145    17.574  32.702   9.685  1.00 44.79        GZ00 O
ATOM   1119  N   CYS C 146    17.133  34.916   9.910  1.00 38.48        GZ00 N
ATOM   1120  CA  CYS C 146    15.879  34.810  10.641  1.00 40.61        GZ00 C
ATOM   1121  C   CYS C 146    14.846  35.719   9.998  1.00 48.21        GZ00 C
ATOM   1122  O   CYS C 146    15.099  36.916   9.820  1.00 41.15        GZ00 O
ATOM   1123  CB  CYS C 146    16.058  35.212  12.114  1.00 36.30        GZ00 C
ATOM   1124  SG  CYS C 146    16.447  33.783  13.156  1.00 70.07        GZ00 S
ATOM   1125  N   LEU C 147    13.679  35.163   9.688  1.00 47.77        GZ00 N
ATOM   1126  CA  LEU C 147    12.549  35.928   9.168  1.00 37.79        GZ00 C
ATOM   1127  C   LEU C 147    11.641  36.320  10.333  1.00 45.95        GZ00 C
ATOM   1128  O   LEU C 147    11.217  35.458  11.112  1.00 45.78        GZ00 O
ATOM   1129  CB  LEU C 147    11.786  35.101   8.140  1.00 39.44        GZ00 C
ATOM   1130  CG  LEU C 147    10.457  35.637   7.600  1.00 42.29        GZ00 C
ATOM   1131  CD1 LEU C 147    10.605  37.010   6.924  1.00 35.22        GZ00 C
ATOM   1132  CD2 LEU C 147     9.902  34.623   6.626  1.00 41.37        GZ00 C
ATOM   1133  N   VAL C 148    11.392  37.614  10.493  1.00 36.60        GZ00 N
ATOM   1134  CA  VAL C 148    10.604  38.136  11.607  1.00 39.69        GZ00 C
```

```
ATOM   1135  C    VAL C 148       9.309  38.707  11.027  1.00 47.33        GZ00  C
ATOM   1136  O    VAL C 148       9.266  39.872  10.617  1.00 45.03        GZ00  O
ATOM   1137  CB   VAL C 148      11.375  39.192  12.409  1.00 42.14        GZ00  C
ATOM   1138  CG1  VAL C 148      10.549  39.661  13.557  1.00 34.51        GZ00  C
ATOM   1139  CG2  VAL C 148      12.701  38.631  12.933  1.00 38.14        GZ00  C
ATOM   1140  N    LYS C 149       8.226  37.923  11.045  1.00 47.26        GZ00  N
ATOM   1141  CA   LYS C 149       7.039  38.205  10.244  1.00 49.61        GZ00  C
ATOM   1142  C    LYS C 149       5.887  38.797  11.059  1.00 48.25        GZ00  C
ATOM   1143  O    LYS C 149       5.698  38.455  12.228  1.00 50.60        GZ00  O
ATOM   1144  CB   LYS C 149       6.563  36.933   9.547  1.00 49.65        GZ00  C
ATOM   1145  CG   LYS C 149       6.317  37.131   8.074  1.00 60.63        GZ00  C
ATOM   1146  CD   LYS C 149       5.120  36.330   7.579  1.00 59.80        GZ00  C
ATOM   1147  CE   LYS C 149       5.405  34.849   7.597  1.00 63.78        GZ00  C
ATOM   1148  NZ   LYS C 149       4.321  34.077   6.887  1.00 62.06        GZ00  N1+
ATOM   1149  N    ASP C 150       5.154  39.729  10.430  1.00 52.54        GZ00  N
ATOM   1150  CA   ASP C 150       3.795  40.160  10.809  1.00 43.96        GZ00  C
ATOM   1151  C    ASP C 150       3.707  40.710  12.236  1.00 45.35        GZ00  C
ATOM   1152  O    ASP C 150       2.955  40.200  13.068  1.00 47.95        GZ00  O
ATOM   1153  CB   ASP C 150       2.782  39.021  10.633  1.00 43.67        GZ00  C
ATOM   1154  CG   ASP C 150       2.587  38.624   9.190  1.00 49.50        GZ00  C
ATOM   1155  OD1  ASP C 150       2.874  39.436   8.284  1.00 47.60        GZ00  O
ATOM   1156  OD2  ASP C 150       2.133  37.486   8.957  1.00 63.54        GZ00  O1-
ATOM   1157  N    TYR C 151       4.437  41.792  12.498  1.00 38.78        GZ00  N
ATOM   1158  CA   TYR C 151       4.395  42.435  13.802  1.00 42.10        GZ00  C
ATOM   1159  C    TYR C 151       4.012  43.911  13.689  1.00 41.99        GZ00  C
ATOM   1160  O    TYR C 151       4.115  44.534  12.628  1.00 46.82        GZ00  O
ATOM   1161  CB   TYR C 151       5.751  42.308  14.529  1.00 46.66        GZ00  C
ATOM   1162  CG   TYR C 151       6.892  43.080  13.890  1.00 45.23        GZ00  C
ATOM   1163  CD1  TYR C 151       7.665  42.517  12.871  1.00 43.89        GZ00  C
ATOM   1164  CD2  TYR C 151       7.206  44.366  14.317  1.00 45.36        GZ00  C
ATOM   1165  CE1  TYR C 151       8.726  43.223  12.280  1.00 43.44        GZ00  C
ATOM   1166  CE2  TYR C 151       8.259  45.082  13.739  1.00 52.49        GZ00  C
ATOM   1167  CZ   TYR C 151       9.018  44.504  12.721  1.00 50.42        GZ00  C
ATOM   1168  OH   TYR C 151      10.045  45.224  12.149  1.00 41.96        GZ00  O
ATOM   1169  N    PHE C 152       3.617  44.474  14.824  1.00 40.95        GZ00  N
ATOM   1170  CA   PHE C 152       3.224  45.868  14.926  1.00 45.04        GZ00  C
ATOM   1171  C    PHE C 152       3.209  46.273  16.388  1.00 42.56        GZ00  C
ATOM   1172  O    PHE C 152       2.802  45.496  17.235  1.00 45.01        GZ00  O
ATOM   1173  CB   PHE C 152       1.847  46.113  14.294  1.00 47.75        GZ00  C
ATOM   1174  CG   PHE C 152       1.465  47.558  14.260  1.00 48.38        GZ00  C
ATOM   1175  CD1  PHE C 152       0.824  48.148  15.339  1.00 48.32        GZ00  C
ATOM   1176  CD2  PHE C 152       1.783  48.342  13.167  1.00 51.92        GZ00  C
ATOM   1177  CE1  PHE C 152       0.510  49.493  15.322  1.00 46.78        GZ00  C
ATOM   1178  CE2  PHE C 152       1.451  49.693  13.144  1.00 49.11        GZ00  C
ATOM   1179  CZ   PHE C 152       0.824  50.262  14.228  1.00 41.05        GZ00  C
ATOM   1180  N    PRO C 153       3.698  47.479  16.702  1.00 42.68        GZ00  N
ATOM   1181  CA   PRO C 153       4.419  48.428  15.848  1.00 52.96        GZ00  C
ATOM   1182  C    PRO C 153       5.930  48.163  15.829  1.00 52.60        GZ00  C
ATOM   1183  O    PRO C 153       6.398  47.155  16.353  1.00 54.71        GZ00  O
ATOM   1184  CB   PRO C 153       4.152  49.766  16.532  1.00 43.17        GZ00  C
ATOM   1185  CG   PRO C 153       4.224  49.393  17.978  1.00 39.59        GZ00  C
ATOM   1186  CD   PRO C 153       3.545  48.016  18.065  1.00 40.76        GZ00  C
ATOM   1187  N    GLU C 154       6.682  49.068  15.220  1.00 50.80        GZ00  N
ATOM   1188  CA   GLU C 154       8.130  49.012  15.287  1.00 47.84        GZ00  C
ATOM   1189  C    GLU C 154       8.530  49.382  16.706  1.00 45.65        GZ00  C
ATOM   1190  O    GLU C 154       7.755  49.990  17.417  1.00 48.79        GZ00  O
ATOM   1191  CB   GLU C 154       8.749  49.958  14.264  1.00 45.04        GZ00  C
ATOM   1192  CG   GLU C 154       8.425  49.586  12.831  1.00 49.34        GZ00  C
ATOM   1193  CD   GLU C 154       9.635  49.012  12.119  1.00 59.99        GZ00  C
ATOM   1194  OE1  GLU C 154      10.114  49.663  11.168  1.00 58.68        GZ00  O
ATOM   1195  OE2  GLU C 154      10.120  47.929  12.535  1.00 57.95        GZ00  O1-
ATOM   1196  N    PRO C 155       9.737  49.006  17.137  1.00 50.54        GZ00  N
ATOM   1197  CA   PRO C 155      10.755  48.195  16.472  1.00 44.50        GZ00  C
ATOM   1198  C    PRO C 155      10.803  46.753  16.983  1.00 53.89        GZ00  C
ATOM   1199  O    PRO C 155      10.174  46.436  17.999  1.00 55.35        GZ00  O
ATOM   1200  CB   PRO C 155      12.031  48.921  16.845  1.00 44.74        GZ00  C
ATOM   1201  CG   PRO C 155      11.773  49.333  18.257  1.00 44.99        GZ00  C
ATOM   1202  CD   PRO C 155      10.279  49.641  18.350  1.00 39.64        GZ00  C
ATOM   1203  N    VAL C 156      11.530  45.887  16.278  1.00 45.82        GZ00  N
ATOM   1204  CA   VAL C 156      12.054  44.666  16.870  1.00 55.31        GZ00  C
ATOM   1205  C    VAL C 156      13.562  44.814  16.945  1.00 52.03        GZ00  C
```

```
ATOM   1206  O   VAL C 156      14.183 45.530 16.153  1.00 55.45      GZ00 O
ATOM   1207  CB  VAL C 156      11.698 43.375 16.105  1.00 52.00      GZ00 C
ATOM   1208  CG1 VAL C 156      10.204 43.139 16.092  1.00 51.44      GZ00 C
ATOM   1209  CG2 VAL C 156      12.231 43.446 14.719  1.00 48.82      GZ00 C
ATOM   1210  N   THR C 157      14.153 44.146 17.919  1.00 53.96      GZ00 N
ATOM   1211  CA  THR C 157      15.596 44.007 17.989  1.00 54.98      GZ00 C
ATOM   1212  C   THR C 157      15.949 42.554 17.732  1.00 54.62      GZ00 C
ATOM   1213  O   THR C 157      15.221 41.644 18.142  1.00 54.87      GZ00 O
ATOM   1214  CB  THR C 157      16.140 44.458 19.341  1.00 45.77      GZ00 C
ATOM   1215  OG1 THR C 157      15.663 43.570 20.361  1.00 57.07      GZ00 O
ATOM   1216  CG2 THR C 157      15.691 45.877 19.627  1.00 51.52      GZ00 C
ATOM   1217  N   VAL C 158      17.042 42.345 17.015  1.00 51.30      GZ00 N
ATOM   1218  CA  VAL C 158      17.521 41.010 16.706  1.00 47.26      GZ00 C
ATOM   1219  C   VAL C 158      18.988 40.957 17.085  1.00 50.93      GZ00 C
ATOM   1220  O   VAL C 158      19.767 41.833 16.692  1.00 49.49      GZ00 O
ATOM   1221  CB  VAL C 158      17.343 40.660 15.219  1.00 46.28      GZ00 C
ATOM   1222  CG1 VAL C 158      17.733 39.213 14.986  1.00 46.68      GZ00 C
ATOM   1223  CG2 VAL C 158      15.911 40.951 14.752  1.00 43.16      GZ00 C
ATOM   1224  N   SER C 159      19.361 39.943 17.850  1.00 51.31      GZ00 N
ATOM   1225  CA  SER C 159      20.756 39.667 18.134  1.00 44.21      GZ00 C
ATOM   1226  C   SER C 159      21.001 38.215 17.769  1.00 45.69      GZ00 C
ATOM   1227  O   SER C 159      20.063 37.437 17.578  1.00 44.40      GZ00 O
ATOM   1228  CB  SER C 159      21.126 39.946 19.605  1.00 39.33      GZ00 C
ATOM   1229  OG  SER C 159      20.384 39.132 20.511  1.00 48.22      GZ00 O
ATOM   1230  N   TRP C 160      22.270 37.863 17.648  1.00 44.65      GZ00 N
ATOM   1231  CA  TRP C 160      22.671 36.505 17.332  1.00 38.87      GZ00 C
ATOM   1232  C   TRP C 160      23.533 35.984 18.468  1.00 43.64      GZ00 C
ATOM   1233  O   TRP C 160      24.351 36.729 19.025  1.00 42.33      GZ00 O
ATOM   1234  CB  TRP C 160      23.420 36.436 15.998  1.00 36.78      GZ00 C
ATOM   1235  CG  TRP C 160      22.520 36.662 14.845  1.00 41.44      GZ00 C
ATOM   1236  CD1 TRP C 160      22.178 37.861 14.294  1.00 39.79      GZ00 C
ATOM   1237  CD2 TRP C 160      21.786 35.664 14.123  1.00 40.51      GZ00 C
ATOM   1238  NE1 TRP C 160      21.304 37.671 13.245  1.00 37.10      GZ00 N
ATOM   1239  CE2 TRP C 160      21.038 36.335 13.125  1.00 36.83      GZ00 C
ATOM   1240  CE3 TRP C 160      21.695 34.270 14.216  1.00 38.13      GZ00 C
ATOM   1241  CZ2 TRP C 160      20.209 35.662 12.228  1.00 37.10      GZ00 C
ATOM   1242  CZ3 TRP C 160      20.878 33.596 13.314  1.00 42.58      GZ00 C
ATOM   1243  CH2 TRP C 160      20.142 34.294 12.336  1.00 43.28      GZ00 C
ATOM   1244  N   ASN C 161      23.291 34.718 18.825  1.00 41.18      GZ00 N
ATOM   1245  CA  ASN C 161      23.956 34.018 19.929  1.00 45.75      GZ00 C
ATOM   1246  C   ASN C 161      24.057 34.898 21.169  1.00 45.89      GZ00 C
ATOM   1247  O   ASN C 161      25.123 35.079 21.763  1.00 49.59      GZ00 O
ATOM   1248  CB  ASN C 161      25.315 33.479 19.496  1.00 36.85      GZ00 C
ATOM   1249  CG  ASN C 161      25.181 32.440 18.399  1.00 44.50      GZ00 C
ATOM   1250  OD1 ASN C 161      24.067 32.010 18.062  1.00 46.14      GZ00 O
ATOM   1251  ND2 ASN C 161      26.306 31.997 17.865  1.00 43.54      GZ00 N
ATOM   1252  N   SER C 162      22.918 35.503 21.508  1.00 46.88      GZ00 N
ATOM   1253  CA  SER C 162      22.740 36.290 22.727  1.00 43.45      GZ00 C
ATOM   1254  C   SER C 162      23.687 37.476 22.784  1.00 47.59      GZ00 C
ATOM   1255  O   SER C 162      24.110 37.883 23.866  1.00 49.00      GZ00 O
ATOM   1256  CB  SER C 162      22.879 35.412 23.972  1.00 38.83      GZ00 C
ATOM   1257  OG  SER C 162      22.029 34.280 23.821  1.00 48.75      GZ00 O
ATOM   1258  N   GLY C 163      24.028 38.040 21.622  1.00 48.38      GZ00 N
ATOM   1259  CA  GLY C 163      24.887 39.205 21.551  1.00 40.94      GZ00 C
ATOM   1260  C   GLY C 163      26.359 38.927 21.292  1.00 53.03      GZ00 C
ATOM   1261  O   GLY C 163      27.105 39.878 20.999  1.00 49.49      GZ00 O
ATOM   1262  N   ALA C 164      26.804 37.665 21.374  1.00 39.35      GZ00 N
ATOM   1263  CA  ALA C 164      28.227 37.381 21.179  1.00 50.46      GZ00 C
ATOM   1264  C   ALA C 164      28.658 37.609 19.732  1.00 52.21      GZ00 C
ATOM   1265  O   ALA C 164      29.776 38.077 19.475  1.00 52.82      GZ00 O
ATOM   1266  CB  ALA C 164      28.544 35.947 21.599  1.00 41.14      GZ00 C
ATOM   1267  N   LEU C 165      27.793 37.267 18.780  1.00 42.69      GZ00 N
ATOM   1268  CA  LEU C 165      28.085 37.349 17.355  1.00 43.42      GZ00 C
ATOM   1269  C   LEU C 165      27.513 38.653 16.821  1.00 44.45      GZ00 C
ATOM   1270  O   LEU C 165      26.292 38.824 16.778  1.00 55.89      GZ00 O
ATOM   1271  CB  LEU C 165      27.476 36.152 16.631  1.00 43.81      GZ00 C
ATOM   1272  CG  LEU C 165      27.622 36.030 15.125  1.00 44.99      GZ00 C
ATOM   1273  CD1 LEU C 165      29.090 36.095 14.649  1.00 39.41      GZ00 C
ATOM   1274  CD2 LEU C 165      26.958 34.734 14.708  1.00 42.07      GZ00 C
ATOM   1275  N   THR C 166      28.389 39.563 16.411  1.00 43.67      GZ00 N
ATOM   1276  CA  THR C 166      28.013 40.872 15.890  1.00 50.96      GZ00 C
```

```
ATOM   1277  C    THR C 166      28.621 41.169 14.521  1.00 48.02      GZ00 C
ATOM   1278  O    THR C 166      27.950 41.770 13.676  1.00 46.23      GZ00 O
ATOM   1279  CB   THR C 166      28.415 41.954 16.912  1.00 52.37      GZ00 C
ATOM   1280  OG1  THR C 166      29.835 41.950 17.073  1.00 48.81      GZ00 O
ATOM   1281  CG2  THR C 166      27.770 41.663 18.271  1.00 45.85      GZ00 C
ATOM   1282  N    SER C 167      29.856 40.736 14.268  1.00 44.72      GZ00 N
ATOM   1283  CA   SER C 167      30.445 40.880 12.941  1.00 46.25      GZ00 C
ATOM   1284  C    SER C 167      29.673 40.045 11.937  1.00 42.05      GZ00 C
ATOM   1285  O    SER C 167      29.319 38.894 12.201  1.00 43.05      GZ00 O
ATOM   1286  CB   SER C 167      31.909 40.437 12.933  1.00 39.29      GZ00 C
ATOM   1287  OG   SER C 167      32.675 41.320 13.706  1.00 49.43      GZ00 O
ATOM   1288  N    GLY C 168      29.424 40.629 10.779  1.00 39.63      GZ00 N
ATOM   1289  CA   GLY C 168      28.689 39.955  9.740  1.00 37.44      GZ00 C
ATOM   1290  C    GLY C 168      27.189 39.958  9.919  1.00 39.53      GZ00 C
ATOM   1291  O    GLY C 168      26.500 39.304  9.131  1.00 41.74      GZ00 O
ATOM   1292  N    VAL C 169      26.653 40.702 10.893  1.00 37.21      GZ00 N
ATOM   1293  CA   VAL C 169      25.219 40.711 11.158  1.00 39.62      GZ00 C
ATOM   1294  C    VAL C 169      24.583 41.858 10.391  1.00 34.91      GZ00 C
ATOM   1295  O    VAL C 169      24.991 43.010 10.529  1.00 35.65      GZ00 O
ATOM   1296  CB   VAL C 169      24.921 40.858 12.660  1.00 42.01      GZ00 C
ATOM   1297  CG1  VAL C 169      23.404 41.063 12.867  1.00 36.86      GZ00 C
ATOM   1298  CG2  VAL C 169      25.410 39.673 13.434  1.00 42.36      GZ00 C
ATOM   1299  N    HIS C 170      23.545 41.564  9.630  1.00 37.60      GZ00 N
ATOM   1300  CA   HIS C 170      22.842 42.603  8.889  1.00 37.31      GZ00 C
ATOM   1301  C    HIS C 170      21.345 42.438  9.134  1.00 37.74      GZ00 C
ATOM   1302  O    HIS C 170      20.704 41.559  8.554  1.00 38.41      GZ00 O
ATOM   1303  CB   HIS C 170      23.180 42.538  7.406  1.00 34.76      GZ00 C
ATOM   1304  CG   HIS C 170      22.719 43.735  6.634  1.00 43.07      GZ00 C
ATOM   1305  ND1  HIS C 170      22.966 43.897  5.287  1.00 43.71      GZ00 N
ATOM   1306  CD2  HIS C 170      22.022 44.830  7.023  1.00 40.56      GZ00 C
ATOM   1307  CE1  HIS C 170      22.453 45.045  4.883  1.00 45.93      GZ00 C
ATOM   1308  NE2  HIS C 170      21.866 45.626  5.915  1.00 45.52      GZ00 N
ATOM   1309  N    THR C 171      20.787 43.270 10.006  1.00 40.13      GZ00 N
ATOM   1310  CA   THR C 171      19.342 43.337 10.182  1.00 39.22      GZ00 C
ATOM   1311  C    THR C 171      18.800 44.400  9.230  1.00 39.29      GZ00 C
ATOM   1312  O    THR C 171      19.079 45.581  9.404  1.00 36.69      GZ00 O
ATOM   1313  CB   THR C 171      18.984 43.680 11.629  1.00 39.31      GZ00 C
ATOM   1314  OG1  THR C 171      19.473 42.647 12.494  1.00 41.01      GZ00 O
ATOM   1315  CG2  THR C 171      17.445 43.812 11.801  1.00 33.43      GZ00 C
ATOM   1316  N    PHE C 172      17.989 43.982  8.262  1.00 38.72      GZ00 N
ATOM   1317  CA   PHE C 172      17.457 44.839  7.215  1.00 38.37      GZ00 C
ATOM   1318  C    PHE C 172      16.263 45.638  7.728  1.00 39.77      GZ00 C
ATOM   1319  O    PHE C 172      15.478 45.140  8.537  1.00 41.83      GZ00 O
ATOM   1320  CB   PHE C 172      16.990 44.017  6.013  1.00 35.77      GZ00 C
ATOM   1321  CG   PHE C 172      18.092 43.449  5.195  1.00 34.43      GZ00 C
ATOM   1322  CD1  PHE C 172      18.884 42.416  5.679  1.00 40.25      GZ00 C
ATOM   1323  CD2  PHE C 172      18.354 43.957  3.936  1.00 40.94      GZ00 C
ATOM   1324  CE1  PHE C 172      19.914 41.886  4.906  1.00 37.45      GZ00 C
ATOM   1325  CE2  PHE C 172      19.389 43.434  3.162  1.00 43.89      GZ00 C
ATOM   1326  CZ   PHE C 172      20.164 42.395  3.650  1.00 36.64      GZ00 C
ATOM   1327  N    PRO C 173      16.092 46.871  7.252  1.00 37.95      GZ00 N
ATOM   1328  CA   PRO C 173      14.883 47.639  7.596  1.00 33.97      GZ00 C
ATOM   1329  C    PRO C 173      13.616 46.905  7.173  1.00 35.10      GZ00 C
ATOM   1330  O    PRO C 173      13.585 46.195  6.163  1.00 33.25      GZ00 O
ATOM   1331  CB   PRO C 173      15.064 48.945  6.817  1.00 35.19      GZ00 C
ATOM   1332  CG   PRO C 173      16.573 49.046  6.616  1.00 34.04      GZ00 C
ATOM   1333  CD   PRO C 173      17.049 47.638  6.441  1.00 32.78      GZ00 C
ATOM   1334  N    ALA C 174      12.550 47.109  7.945  1.00 39.15      GZ00 N
ATOM   1335  CA   ALA C 174      11.316 46.339  7.783  1.00 39.81      GZ00 C
ATOM   1336  C    ALA C 174      10.533 46.750  6.532  1.00 35.98      GZ00 C
ATOM   1337  O    ALA C 174      10.677 47.856  6.028  1.00 39.58      GZ00 O
ATOM   1338  CB   ALA C 174      10.432 46.527  9.013  1.00 43.39      GZ00 C
ATOM   1339  N    VAL C 175       9.686 45.824  6.013  1.00 38.79      GZ00 N
ATOM   1340  CA   VAL C 175       8.639 46.209  5.066  1.00 39.33      GZ00 C
ATOM   1341  C    VAL C 175       7.400 46.592  5.844  1.00 44.21      GZ00 C
ATOM   1342  O    VAL C 175       7.056 45.957  6.847  1.00 43.28      GZ00 O
ATOM   1343  CB   VAL C 175       8.256 45.087  4.084  1.00 50.56      GZ00 C
ATOM   1344  CG1  VAL C 175       8.851 45.303  2.713  1.00 51.62      GZ00 C
ATOM   1345  CG2  VAL C 175       8.510 43.718  4.667  1.00 44.82      GZ00 C
ATOM   1346  N    LEU C 176       6.686 47.587  5.342  1.00 46.20      GZ00 N
ATOM   1347  CA   LEU C 176       5.329 47.863  5.785  1.00 41.81      GZ00 C
```

```
ATOM   1348  C   LEU C 176      4.400  47.199   4.775  1.00 42.53      GZ00 C
ATOM   1349  O   LEU C 176      4.405  47.554   3.595  1.00 55.35      GZ00 O
ATOM   1350  CB  LEU C 176      5.082  49.361   5.889  1.00 43.16      GZ00 C
ATOM   1351  CG  LEU C 176      3.653  49.738   6.270  1.00 46.81      GZ00 C
ATOM   1352  CD1 LEU C 176      3.258  48.982   7.523  1.00 39.25      GZ00 C
ATOM   1353  CD2 LEU C 176      3.565  51.241   6.509  1.00 41.80      GZ00 C
ATOM   1354  N   GLN C 177      3.666  46.189   5.219  1.00 44.53      GZ00 N
ATOM   1355  CA  GLN C 177      2.809  45.403   4.345  1.00 50.59      GZ00 C
ATOM   1356  C   GLN C 177      1.442  46.069   4.173  1.00 49.14      GZ00 C
ATOM   1357  O   GLN C 177      0.999  46.856   5.018  1.00 43.05      GZ00 O
ATOM   1358  CB  GLN C 177      2.634  43.990   4.909  1.00 46.96      GZ00 C
ATOM   1359  CG  GLN C 177      3.923  43.188   5.105  1.00 49.24      GZ00 C
ATOM   1360  CD  GLN C 177      3.666  41.894   5.863  1.00 56.86      GZ00 C
ATOM   1361  OE1 GLN C 177      3.214  40.898   5.285  1.00 62.22      GZ00 O
ATOM   1362  NE2 GLN C 177      3.939  41.905   7.170  1.00 48.26      GZ00 N
ATOM   1363  N   SER C 178      0.751  45.698   3.084  1.00 45.38      GZ00 N
ATOM   1364  CA  SER C 178     -0.624  46.166   2.867  1.00 48.86      GZ00 C
ATOM   1365  C   SER C 178     -1.544  45.854   4.039  1.00 46.49      GZ00 C
ATOM   1366  O   SER C 178     -2.528  46.562   4.260  1.00 57.16      GZ00 O
ATOM   1367  CB  SER C 178     -1.216  45.531   1.617  1.00 50.40      GZ00 C
ATOM   1368  OG  SER C 178     -0.204  45.003   0.795  1.00 71.70      GZ00 O
ATOM   1369  N   SER C 179     -1.258  44.793   4.786  1.00 50.74      GZ00 N
ATOM   1370  CA  SER C 179     -1.988  44.477   6.007  1.00 43.18      GZ00 C
ATOM   1371  C   SER C 179     -1.776  45.503   7.110  1.00 49.34      GZ00 C
ATOM   1372  O   SER C 179     -2.482  45.454   8.121  1.00 48.58      GZ00 O
ATOM   1373  CB  SER C 179     -1.546  43.111   6.513  1.00 44.27      GZ00 C
ATOM   1374  OG  SER C 179     -0.258  43.204   7.095  1.00 48.97      GZ00 O
ATOM   1375  N   GLY C 180     -0.798  46.398   6.970  1.00 46.74      GZ00 N
ATOM   1376  CA  GLY C 180     -0.440  47.281   8.059  1.00 45.92      GZ00 C
ATOM   1377  C   GLY C 180      0.566  46.707   9.037  1.00 50.25      GZ00 C
ATOM   1378  O   GLY C 180      0.916  47.389  10.012  1.00 43.25      GZ00 O
ATOM   1379  N   LEU C 181      1.039  45.481   8.813  1.00 44.89      GZ00 N
ATOM   1380  CA  LEU C 181      2.003  44.836   9.695  1.00 49.99      GZ00 C
ATOM   1381  C   LEU C 181      3.408  44.908   9.101  1.00 47.59      GZ00 C
ATOM   1382  O   LEU C 181      3.594  45.023   7.887  1.00 47.03      GZ00 O
ATOM   1383  CB  LEU C 181      1.621  43.373   9.938  1.00 43.97      GZ00 C
ATOM   1384  CG  LEU C 181      0.269  43.159  10.616  1.00 50.80      GZ00 C
ATOM   1385  CD1 LEU C 181     -0.047  41.676  10.719  1.00 43.98      GZ00 C
ATOM   1386  CD2 LEU C 181      0.246  43.830  11.981  1.00 46.56      GZ00 C
ATOM   1387  N   TYR C 182      4.404  44.833   9.973  1.00 45.32      GZ00 N
ATOM   1388  CA  TYR C 182      5.793  44.866   9.536  1.00 45.62      GZ00 C
ATOM   1389  C   TYR C 182      6.381  43.465   9.437  1.00 44.85      GZ00 C
ATOM   1390  O   TYR C 182      5.955  42.536  10.124  1.00 51.18      GZ00 O
ATOM   1391  CB  TYR C 182      6.645  45.701  10.484  1.00 44.25      GZ00 C
ATOM   1392  CG  TYR C 182      6.265  47.162  10.529  1.00 49.19      GZ00 C
ATOM   1393  CD1 TYR C 182      6.805  48.072   9.628  1.00 49.61      GZ00 C
ATOM   1394  CD2 TYR C 182      5.368  47.633  11.474  1.00 51.64      GZ00 C
ATOM   1395  CE1 TYR C 182      6.474  49.408   9.678  1.00 43.85      GZ00 C
ATOM   1396  CE2 TYR C 182      5.029  48.974  11.530  1.00 55.94      GZ00 C
ATOM   1397  CZ  TYR C 182      5.587  49.853  10.626  1.00 50.36      GZ00 C
ATOM   1398  OH  TYR C 182      5.243  51.177  10.679  1.00 43.75      GZ00 O
ATOM   1399  N   SER C 183      7.374  43.326   8.569  1.00 46.44      GZ00 N
ATOM   1400  CA  SER C 183      8.231  42.152   8.534  1.00 42.34      GZ00 C
ATOM   1401  C   SER C 183      9.673  42.600   8.304  1.00 42.61      GZ00 C
ATOM   1402  O   SER C 183      9.927  43.635   7.681  1.00 42.07      GZ00 O
ATOM   1403  CB  SER C 183      7.799  41.173   7.452  1.00 40.89      GZ00 C
ATOM   1404  OG  SER C 183      6.531  40.617   7.751  1.00 50.66      GZ00 O
ATOM   1405  N   LEU C 184     10.622  41.838   8.842  1.00 42.23      GZ00 N
ATOM   1406  CA  LEU C 184     12.018  42.081   8.515  1.00 39.25      GZ00 C
ATOM   1407  C   LEU C 184     12.794  40.772   8.518  1.00 43.48      GZ00 C
ATOM   1408  O   LEU C 184     12.337  39.746   9.029  1.00 40.77      GZ00 O
ATOM   1409  CB  LEU C 184     12.650  43.095   9.473  1.00 35.18      GZ00 C
ATOM   1410  CG  LEU C 184     12.809  42.808  10.965  1.00 45.00      GZ00 C
ATOM   1411  CD1 LEU C 184     13.869  41.700  11.303  1.00 40.29      GZ00 C
ATOM   1412  CD2 LEU C 184     13.218  44.117  11.588  1.00 33.99      GZ00 C
ATOM   1413  N   SER C 185     14.012  40.842   7.986  1.00 44.24      GZ00 N
ATOM   1414  CA  SER C 185     14.963  39.747   8.041  1.00 35.64      GZ00 C
ATOM   1415  C   SER C 185     16.258  40.228   8.669  1.00 41.38      GZ00 C
ATOM   1416  O   SER C 185     16.650  41.392   8.526  1.00 39.54      GZ00 O
ATOM   1417  CB  SER C 185     15.260  39.197   6.661  1.00 36.15      GZ00 C
ATOM   1418  OG  SER C 185     14.075  38.752   6.055  1.00 40.64      GZ00 O
```

```
ATOM   1419  N   SER C 186     16.927  39.308   9.349  1.00 38.32      GZ00 N
ATOM   1420  CA  SER C 186     18.265  39.524   9.871  1.00 36.92      GZ00 C
ATOM   1421  C   SER C 186     19.108  38.382   9.349  1.00 40.04      GZ00 C
ATOM   1422  O   SER C 186     18.687  37.226   9.434  1.00 43.87      GZ00 O
ATOM   1423  CB  SER C 186     18.273  39.558  11.396  1.00 38.94      GZ00 C
ATOM   1424  OG  SER C 186     19.589  39.675  11.902  1.00 39.68      GZ00 O
ATOM   1425  N   VAL C 187     20.267  38.698   8.773  1.00 38.05      GZ00 N
ATOM   1426  CA  VAL C 187     21.164  37.675   8.245  1.00 39.23      GZ00 C
ATOM   1427  C   VAL C 187     22.543  37.869   8.849  1.00 38.02      GZ00 C
ATOM   1428  O   VAL C 187     22.977  38.990   9.130  1.00 40.44      GZ00 O
ATOM   1429  CB  VAL C 187     21.291  37.714   6.708  1.00 42.17      GZ00 C
ATOM   1430  CG1 VAL C 187     19.984  37.334   6.048  1.00 51.50      GZ00 C
ATOM   1431  CG2 VAL C 187     21.733  39.100   6.265  1.00 42.55      GZ00 C
ATOM   1432  N   VAL C 188     23.252  36.770   9.018  1.00 36.36      GZ00 N
ATOM   1433  CA  VAL C 188     24.640  36.836   9.426  1.00 36.04      GZ00 C
ATOM   1434  C   VAL C 188     25.458  36.076   8.397  1.00 29.41      GZ00 C
ATOM   1435  O   VAL C 188     25.037  35.025   7.901  1.00 36.76      GZ00 O
ATOM   1436  CB  VAL C 188     24.848  36.314  10.861  1.00 42.84      GZ00 C
ATOM   1437  CG1 VAL C 188     24.200  34.977  11.043  1.00 38.37      GZ00 C
ATOM   1438  CG2 VAL C 188     26.343  36.272  11.210  1.00 41.22      GZ00 C
ATOM   1439  N   THR C 189     26.558  36.673   8.000  1.00 28.33      GZ00 N
ATOM   1440  CA  THR C 189     27.541  36.056   7.132  1.00 32.84      GZ00 C
ATOM   1441  C   THR C 189     28.628  35.434   8.001  1.00 34.03      GZ00 C
ATOM   1442  O   THR C 189     29.180  36.100   8.883  1.00 35.30      GZ00 O
ATOM   1443  CB  THR C 189     28.159  37.112   6.215  1.00 32.93      GZ00 C
ATOM   1444  OG1 THR C 189     27.144  37.724   5.438  1.00 45.99      GZ00 O
ATOM   1445  CG2 THR C 189     29.182  36.491   5.293  1.00 42.71      GZ00 C
ATOM   1446  N   VAL C 190     28.939  34.171   7.745  1.00 34.62      GZ00 N
ATOM   1447  CA  VAL C 190     29.911  33.429   8.544  1.00 31.43      GZ00 C
ATOM   1448  C   VAL C 190     30.828  32.676   7.590  1.00 33.62      GZ00 C
ATOM   1449  O   VAL C 190     30.489  32.452   6.412  1.00 32.38      GZ00 O
ATOM   1450  CB  VAL C 190     29.217  32.438   9.514  1.00 32.44      GZ00 C
ATOM   1451  CG1 VAL C 190     28.249  33.160  10.466  1.00 30.52      GZ00 C
ATOM   1452  CG2 VAL C 190     28.516  31.364   8.716  1.00 32.19      GZ00 C
ATOM   1453  N   PRO C 191     31.989  32.240   8.078  1.00 30.12      GZ00 N
ATOM   1454  CA  PRO C 191     32.826  31.359   7.271  1.00 32.76      GZ00 C
ATOM   1455  C   PRO C 191     32.058  30.080   6.963  1.00 30.65      GZ00 C
ATOM   1456  O   PRO C 191     31.363  29.531   7.820  1.00 33.10      GZ00 O
ATOM   1457  CB  PRO C 191     34.045  31.120   8.172  1.00 32.26      GZ00 C
ATOM   1458  CG  PRO C 191     34.060  32.297   9.081  1.00 34.77      GZ00 C
ATOM   1459  CD  PRO C 191     32.645  32.579   9.350  1.00 27.68      GZ00 C
ATOM   1460  N   SER C 192     32.149  29.634   5.711  1.00 29.53      GZ00 N
ATOM   1461  CA  SER C 192     31.350  28.487   5.287  1.00 39.33      GZ00 C
ATOM   1462  C   SER C 192     31.675  27.230   6.099  1.00 36.23      GZ00 C
ATOM   1463  O   SER C 192     30.769  26.485   6.472  1.00 42.93      GZ00 O
ATOM   1464  CB  SER C 192     31.541  28.240   3.789  1.00 38.81      GZ00 C
ATOM   1465  OG  SER C 192     32.874  27.909   3.459  1.00 43.23      GZ00 O
ATOM   1466  N   SER C 193     32.938  27.035   6.471  1.00 34.82      GZ00 N
ATOM   1467  CA  SER C 193     33.323  25.884   7.287  1.00 34.53      GZ00 C
ATOM   1468  C   SER C 193     32.696  25.922   8.671  1.00 34.09      GZ00 C
ATOM   1469  O   SER C 193     32.757  24.928   9.386  1.00 39.08      GZ00 O
ATOM   1470  CB  SER C 193     34.853  25.776   7.406  1.00 30.12      GZ00 C
ATOM   1471  OG  SER C 193     35.392  26.892   8.093  1.00 36.36      GZ00 O
ATOM   1472  N   SER C 194     32.163  27.056   9.102  1.00 38.37      GZ00 N
ATOM   1473  CA  SER C 194     31.530  27.087  10.411  1.00 33.39      GZ00 C
ATOM   1474  C   SER C 194     30.113  26.516  10.401  1.00 42.74      GZ00 C
ATOM   1475  O   SER C 194     29.523  26.338  11.474  1.00 39.11      GZ00 O
ATOM   1476  CB  SER C 194     31.499  28.525  10.943  1.00 36.98      GZ00 C
ATOM   1477  OG  SER C 194     32.808  29.033  11.127  1.00 48.78      GZ00 O
ATOM   1478  N   LEU C 195     29.544  26.221   9.235  1.00 39.64      GZ00 N
ATOM   1479  CA  LEU C 195     28.171  25.716   9.219  1.00 49.46      GZ00 C
ATOM   1480  C   LEU C 195     28.073  24.358   9.912  1.00 44.13      GZ00 C
ATOM   1481  O   LEU C 195     27.079  24.064  10.584  1.00 58.41      GZ00 O
ATOM   1482  CB  LEU C 195     27.658  25.619   7.783  1.00 39.54      GZ00 C
ATOM   1483  CG  LEU C 195     27.446  26.952   7.097  1.00 40.14      GZ00 C
ATOM   1484  CD1 LEU C 195     26.943  26.739   5.696  1.00 44.58      GZ00 C
ATOM   1485  CD2 LEU C 195     26.470  27.772   7.904  1.00 40.80      GZ00 C
ATOM   1486  N   GLY C 196     29.081  23.506   9.744  1.00 51.36      GZ00 N
ATOM   1487  CA  GLY C 196     29.046  22.221  10.420  1.00 65.18      GZ00 C
ATOM   1488  C   GLY C 196     29.407  22.268  11.889  1.00 66.41      GZ00 C
ATOM   1489  O   GLY C 196     29.013  21.375  12.644  1.00 61.49      GZ00 O
```

| ATOM | 1490 | N | THR | C | 197 | 30.128 | 23.300 | 12.314 | 1.00 | 62.25 | GZ00 | N |
| ATOM | 1491 | CA | THR | C | 197 | 30.755 | 23.340 | 13.626 | 1.00 | 58.93 | GZ00 | C |
| ATOM | 1492 | C | THR | C | 197 | 29.995 | 24.161 | 14.655 | 1.00 | 58.26 | GZ00 | C |
| ATOM | 1493 | O | THR | C | 197 | 30.047 | 23.842 | 15.845 | 1.00 | 62.50 | GZ00 | O |
| ATOM | 1494 | CB | THR | C | 197 | 32.165 | 23.899 | 13.486 | 1.00 | 60.74 | GZ00 | C |
| ATOM | 1495 | OG1 | THR | C | 197 | 32.802 | 23.224 | 12.405 | 1.00 | 62.30 | GZ00 | O |
| ATOM | 1496 | CG2 | THR | C | 197 | 32.961 | 23.642 | 14.759 | 1.00 | 74.83 | GZ00 | C |
| ATOM | 1497 | N | GLN | C | 198 | 29.373 | 25.261 | 14.243 | 1.00 | 52.75 | GZ00 | N |
| ATOM | 1498 | CA | GLN | C | 198 | 28.836 | 26.250 | 15.166 | 1.00 | 50.37 | GZ00 | C |
| ATOM | 1499 | C | GLN | C | 198 | 27.314 | 26.260 | 15.176 | 1.00 | 47.44 | GZ00 | C |
| ATOM | 1500 | O | GLN | C | 198 | 26.650 | 25.910 | 14.197 | 1.00 | 47.96 | GZ00 | O |
| ATOM | 1501 | CB | GLN | C | 198 | 29.345 | 27.650 | 14.839 | 1.00 | 49.33 | GZ00 | C |
| ATOM | 1502 | CG | GLN | C | 198 | 30.852 | 27.725 | 14.782 | 1.00 | 55.27 | GZ00 | C |
| ATOM | 1503 | CD | GLN | C | 198 | 31.515 | 27.225 | 16.062 | 1.00 | 61.24 | GZ00 | C |
| ATOM | 1504 | OE1 | GLN | C | 198 | 32.566 | 26.585 | 16.007 | 1.00 | 51.30 | GZ00 | O |
| ATOM | 1505 | NE2 | GLN | C | 198 | 30.903 | 27.517 | 17.218 | 1.00 | 56.92 | GZ00 | N |
| ATOM | 1506 | N | THR | C | 199 | 26.773 | 26.623 | 16.322 | 1.00 | 52.47 | GZ00 | N |
| ATOM | 1507 | CA | THR | C | 199 | 25.343 | 26.782 | 16.481 | 1.00 | 55.26 | GZ00 | C |
| ATOM | 1508 | C | THR | C | 199 | 25.008 | 28.268 | 16.402 | 1.00 | 49.37 | GZ00 | C |
| ATOM | 1509 | O | THR | C | 199 | 25.618 | 29.094 | 17.092 | 1.00 | 42.11 | GZ00 | O |
| ATOM | 1510 | CB | THR | C | 199 | 24.875 | 26.156 | 17.795 | 1.00 | 51.51 | GZ00 | C |
| ATOM | 1511 | OG1 | THR | C | 199 | 24.993 | 24.734 | 17.686 | 1.00 | 63.19 | GZ00 | O |
| ATOM | 1512 | CG2 | THR | C | 199 | 23.428 | 26.505 | 18.070 | 1.00 | 53.59 | GZ00 | C |
| ATOM | 1513 | N | TYR | C | 200 | 24.078 | 28.610 | 15.518 | 1.00 | 48.90 | GZ00 | N |
| ATOM | 1514 | CA | TYR | C | 200 | 23.662 | 29.990 | 15.315 | 1.00 | 48.61 | GZ00 | C |
| ATOM | 1515 | C | TYR | C | 200 | 22.209 | 30.127 | 15.721 | 1.00 | 46.13 | GZ00 | C |
| ATOM | 1516 | O | TYR | C | 200 | 21.332 | 29.443 | 15.173 | 1.00 | 44.41 | GZ00 | O |
| ATOM | 1517 | CB | TYR | C | 200 | 23.885 | 30.425 | 13.871 | 1.00 | 40.49 | GZ00 | C |
| ATOM | 1518 | CG | TYR | C | 200 | 25.348 | 30.403 | 13.527 | 1.00 | 43.98 | GZ00 | C |
| ATOM | 1519 | CD1 | TYR | C | 200 | 26.196 | 31.359 | 14.053 | 1.00 | 42.42 | GZ00 | C |
| ATOM | 1520 | CD2 | TYR | C | 200 | 25.891 | 29.415 | 12.718 | 1.00 | 41.47 | GZ00 | C |
| ATOM | 1521 | CE1 | TYR | C | 200 | 27.536 | 31.356 | 13.770 | 1.00 | 39.19 | GZ00 | C |
| ATOM | 1522 | CE2 | TYR | C | 200 | 27.254 | 29.408 | 12.425 | 1.00 | 45.38 | GZ00 | C |
| ATOM | 1523 | CZ | TYR | C | 200 | 28.062 | 30.394 | 12.954 | 1.00 | 39.23 | GZ00 | C |
| ATOM | 1524 | OH | TYR | C | 200 | 29.417 | 30.433 | 12.699 | 1.00 | 45.19 | GZ00 | O |
| ATOM | 1525 | N | ILE | C | 201 | 21.975 | 30.996 | 16.697 | 1.00 | 42.87 | GZ00 | N |
| ATOM | 1526 | CA | ILE | C | 201 | 20.663 | 31.253 | 17.264 | 1.00 | 42.84 | GZ00 | C |
| ATOM | 1527 | C | ILE | C | 201 | 20.414 | 32.743 | 17.188 | 1.00 | 44.10 | GZ00 | C |
| ATOM | 1528 | O | ILE | C | 201 | 21.240 | 33.530 | 17.663 | 1.00 | 46.46 | GZ00 | O |
| ATOM | 1529 | CB | ILE | C | 201 | 20.581 | 30.788 | 18.727 | 1.00 | 50.09 | GZ00 | C |
| ATOM | 1530 | CG1 | ILE | C | 201 | 20.743 | 29.262 | 18.814 | 1.00 | 45.99 | GZ00 | C |
| ATOM | 1531 | CG2 | ILE | C | 201 | 19.298 | 31.312 | 19.375 | 1.00 | 42.60 | GZ00 | C |
| ATOM | 1532 | CD1 | ILE | C | 201 | 20.953 | 28.790 | 20.208 | 1.00 | 32.65 | GZ00 | C |
| ATOM | 1533 | N | CYS | C | 202 | 19.279 | 33.131 | 16.604 | 1.00 | 42.47 | GZ00 | N |
| ATOM | 1534 | CA | CYS | C | 202 | 18.862 | 34.523 | 16.601 | 1.00 | 43.27 | GZ00 | C |
| ATOM | 1535 | C | CYS | C | 202 | 17.860 | 34.776 | 17.723 | 1.00 | 49.37 | GZ00 | C |
| ATOM | 1536 | O | CYS | C | 202 | 17.021 | 33.922 | 18.040 | 1.00 | 48.50 | GZ00 | O |
| ATOM | 1537 | CB | CYS | C | 202 | 18.262 | 34.923 | 15.250 | 1.00 | 47.82 | GZ00 | C |
| ATOM | 1538 | SG | CYS | C | 202 | 16.539 | 34.525 | 15.045 | 1.00 | 59.08 | GZ00 | S |
| ATOM | 1539 | N | ASN | C | 203 | 17.976 | 35.945 | 18.341 | 1.00 | 44.56 | GZ00 | N |
| ATOM | 1540 | CA | ASN | C | 203 | 17.167 | 36.321 | 19.492 | 1.00 | 39.69 | GZ00 | C |
| ATOM | 1541 | C | ASN | C | 203 | 16.329 | 37.519 | 19.079 | 1.00 | 46.86 | GZ00 | C |
| ATOM | 1542 | O | ASN | C | 203 | 16.862 | 38.616 | 18.876 | 1.00 | 43.01 | GZ00 | O |
| ATOM | 1543 | CB | ASN | C | 203 | 18.043 | 36.660 | 20.698 | 1.00 | 40.17 | GZ00 | C |
| ATOM | 1544 | CG | ASN | C | 203 | 19.277 | 35.769 | 20.785 | 1.00 | 50.91 | GZ00 | C |
| ATOM | 1545 | OD1 | ASN | C | 203 | 20.407 | 36.239 | 20.626 | 1.00 | 44.43 | GZ00 | O |
| ATOM | 1546 | ND2 | ASN | C | 203 | 19.057 | 34.467 | 20.968 | 1.00 | 40.98 | GZ00 | N |
| ATOM | 1547 | N | VAL | C | 204 | 15.027 | 37.320 | 18.972 | 1.00 | 44.56 | GZ00 | N |
| ATOM | 1548 | CA | VAL | C | 204 | 14.126 | 38.360 | 18.515 | 1.00 | 46.34 | GZ00 | C |
| ATOM | 1549 | C | VAL | C | 204 | 13.377 | 38.894 | 19.715 | 1.00 | 43.52 | GZ00 | C |
| ATOM | 1550 | O | VAL | C | 204 | 12.839 | 38.122 | 20.516 | 1.00 | 51.43 | GZ00 | O |
| ATOM | 1551 | CB | VAL | C | 204 | 13.166 | 37.826 | 17.447 | 1.00 | 50.83 | GZ00 | C |
| ATOM | 1552 | CG1 | VAL | C | 204 | 12.204 | 38.932 | 17.006 | 1.00 | 40.92 | GZ00 | C |
| ATOM | 1553 | CG2 | VAL | C | 204 | 13.984 | 37.271 | 16.291 | 1.00 | 38.78 | GZ00 | C |
| ATOM | 1554 | N | ASN | C | 205 | 13.361 | 40.208 | 19.858 | 1.00 | 40.86 | GZ00 | N |
| ATOM | 1555 | CA | ASN | C | 205 | 12.687 | 40.856 | 20.970 | 1.00 | 54.57 | GZ00 | C |
| ATOM | 1556 | C | ASN | C | 205 | 11.772 | 41.947 | 20.431 | 1.00 | 58.30 | GZ00 | C |
| ATOM | 1557 | O | ASN | C | 205 | 12.237 | 42.886 | 19.776 | 1.00 | 55.70 | GZ00 | O |
| ATOM | 1558 | CB | ASN | C | 205 | 13.691 | 41.445 | 21.965 | 1.00 | 49.16 | GZ00 | C |
| ATOM | 1559 | CG | ASN | C | 205 | 13.009 | 42.013 | 23.204 | 1.00 | 69.12 | GZ00 | C |
| ATOM | 1560 | OD1 | ASN | C | 205 | 11.837 | 41.702 | 23.489 | 1.00 | 63.20 | GZ00 | O |

| ATOM | 1561 | ND2 | ASN | C | 205 | 13.716 | 42.884 | 23.921 | 1.00 | 72.08 | GZ00 | N |
|---|---|---|---|---|---|---|---|---|---|---|---|---|
| ATOM | 1562 | N | HIS | C | 206 | 10.482 | 41.839 | 20.732 | 1.00 | 55.60 | GZ00 | N |
| ATOM | 1563 | CA | HIS | C | 206 | 9.489 | 42.834 | 20.326 | 1.00 | 56.54 | GZ00 | C |
| ATOM | 1564 | C | HIS | C | 206 | 8.843 | 43.347 | 21.611 | 1.00 | 58.64 | GZ00 | C |
| ATOM | 1565 | O | HIS | C | 206 | 7.857 | 42.788 | 22.098 | 1.00 | 54.70 | GZ00 | O |
| ATOM | 1566 | CB | HIS | C | 206 | 8.472 | 42.249 | 19.356 | 1.00 | 51.09 | GZ00 | C |
| ATOM | 1567 | CG | HIS | C | 206 | 7.482 | 43.251 | 18.851 | 1.00 | 60.00 | GZ00 | C |
| ATOM | 1568 | ND1 | HIS | C | 206 | 6.150 | 42.954 | 18.660 | 1.00 | 56.09 | GZ00 | N |
| ATOM | 1569 | CD2 | HIS | C | 206 | 7.633 | 44.548 | 18.494 | 1.00 | 60.16 | GZ00 | C |
| ATOM | 1570 | CE1 | HIS | C | 206 | 5.525 | 44.026 | 18.209 | 1.00 | 55.91 | GZ00 | C |
| ATOM | 1571 | NE2 | HIS | C | 206 | 6.402 | 45.007 | 18.100 | 1.00 | 50.50 | GZ00 | N |
| ATOM | 1572 | N | LYS | C | 207 | 9.435 | 44.398 | 22.179 | 1.00 | 59.62 | GZ00 | N |
| ATOM | 1573 | CA | LYS | C | 207 | 8.977 | 44.892 | 23.475 | 1.00 | 60.73 | GZ00 | C |
| ATOM | 1574 | C | LYS | C | 207 | 7.519 | 45.338 | 23.493 | 1.00 | 69.23 | GZ00 | C |
| ATOM | 1575 | O | LYS | C | 207 | 6.848 | 45.083 | 24.509 | 1.00 | 66.50 | GZ00 | O |
| ATOM | 1576 | CB | LYS | C | 207 | 9.906 | 46.014 | 23.956 | 1.00 | 58.40 | GZ00 | C |
| ATOM | 1577 | CG | LYS | C | 207 | 11.292 | 45.481 | 24.321 | 1.00 | 69.34 | GZ00 | C |
| ATOM | 1578 | CD | LYS | C | 207 | 12.257 | 46.543 | 24.809 | 1.00 | 77.26 | GZ00 | C |
| ATOM | 1579 | CE | LYS | C | 207 | 13.596 | 45.890 | 25.148 | 1.00 | 81.04 | GZ00 | C |
| ATOM | 1580 | NZ | LYS | C | 207 | 14.641 | 46.873 | 25.530 | 1.00 | 87.24 | GZ00 | N1+ |
| ATOM | 1581 | N | PRO | C | 208 | 6.956 | 45.959 | 22.441 | 1.00 | 69.40 | GZ00 | N |
| ATOM | 1582 | CA | PRO | C | 208 | 5.540 | 46.372 | 22.538 | 1.00 | 59.57 | GZ00 | C |
| ATOM | 1583 | C | PRO | C | 208 | 4.585 | 45.244 | 22.898 | 1.00 | 64.04 | GZ00 | C |
| ATOM | 1584 | O | PRO | C | 208 | 3.640 | 45.466 | 23.666 | 1.00 | 69.66 | GZ00 | O |
| ATOM | 1585 | CB | PRO | C | 208 | 5.257 | 46.942 | 21.145 | 1.00 | 53.38 | GZ00 | C |
| ATOM | 1586 | CG | PRO | C | 208 | 6.582 | 47.479 | 20.704 | 1.00 | 65.93 | GZ00 | C |
| ATOM | 1587 | CD | PRO | C | 208 | 7.608 | 46.524 | 21.241 | 1.00 | 55.91 | GZ00 | C |
| ATOM | 1588 | N | SER | C | 209 | 4.814 | 44.034 | 22.401 | 1.00 | 60.28 | GZ00 | N |
| ATOM | 1589 | CA | SER | C | 209 | 4.019 | 42.880 | 22.786 | 1.00 | 57.00 | GZ00 | C |
| ATOM | 1590 | C | SER | C | 209 | 4.762 | 41.960 | 23.758 | 1.00 | 67.68 | GZ00 | C |
| ATOM | 1591 | O | SER | C | 209 | 4.284 | 40.848 | 24.024 | 1.00 | 64.38 | GZ00 | O |
| ATOM | 1592 | CB | SER | C | 209 | 3.586 | 42.091 | 21.546 | 1.00 | 56.00 | GZ00 | C |
| ATOM | 1593 | OG | SER | C | 209 | 4.691 | 41.469 | 20.915 | 1.00 | 63.30 | GZ00 | O |
| ATOM | 1594 | N | ASN | C | 210 | 5.918 | 42.392 | 24.271 | 1.00 | 68.38 | GZ00 | N |
| ATOM | 1595 | CA | ASN | C | 210 | 6.846 | 41.563 | 25.049 | 1.00 | 66.63 | GZ00 | C |
| ATOM | 1596 | C | ASN | C | 210 | 6.898 | 40.139 | 24.505 | 1.00 | 64.18 | GZ00 | C |
| ATOM | 1597 | O | ASN | C | 210 | 6.538 | 39.164 | 25.164 | 1.00 | 67.42 | GZ00 | O |
| ATOM | 1598 | CB | ASN | C | 210 | 6.528 | 41.574 | 26.543 | 1.00 | 74.01 | GZ00 | C |
| ATOM | 1599 | CG | ASN | C | 210 | 5.055 | 41.657 | 26.828 | 1.00 | 83.26 | GZ00 | C |
| ATOM | 1600 | OD1 | ASN | C | 210 | 4.483 | 42.750 | 26.885 | 1.00 | 89.02 | GZ00 | O |
| ATOM | 1601 | ND2 | ASN | C | 210 | 4.420 | 40.502 | 26.997 | 1.00 | 83.49 | GZ00 | N |
| ATOM | 1602 | N | THR | C | 211 | 7.358 | 40.045 | 23.268 | 1.00 | 61.55 | GZ00 | N |
| ATOM | 1603 | CA | THR | C | 211 | 7.633 | 38.772 | 22.633 | 1.00 | 57.57 | GZ00 | C |
| ATOM | 1604 | C | THR | C | 211 | 9.143 | 38.608 | 22.609 | 1.00 | 60.01 | GZ00 | C |
| ATOM | 1605 | O | THR | C | 211 | 9.862 | 39.532 | 22.214 | 1.00 | 65.19 | GZ00 | O |
| ATOM | 1606 | CB | THR | C | 211 | 7.089 | 38.734 | 21.209 | 1.00 | 54.23 | GZ00 | C |
| ATOM | 1607 | OG1 | THR | C | 211 | 5.691 | 39.013 | 21.227 | 1.00 | 60.96 | GZ00 | O |
| ATOM | 1608 | CG2 | THR | C | 211 | 7.316 | 37.377 | 20.586 | 1.00 | 57.50 | GZ00 | C |
| ATOM | 1609 | N | LYS | C | 212 | 9.620 | 37.460 | 23.084 | 1.00 | 60.21 | GZ00 | N |
| ATOM | 1610 | CA | LYS | C | 212 | 11.018 | 37.076 | 22.951 | 1.00 | 52.01 | GZ00 | C |
| ATOM | 1611 | C | LYS | C | 212 | 11.042 | 35.689 | 22.350 | 1.00 | 52.91 | GZ00 | C |
| ATOM | 1612 | O | LYS | C | 212 | 10.456 | 34.760 | 22.906 | 1.00 | 57.94 | GZ00 | O |
| ATOM | 1613 | CB | LYS | C | 212 | 11.753 | 37.110 | 24.290 | 1.00 | 47.41 | GZ00 | C |
| ATOM | 1614 | CG | LYS | C | 212 | 11.691 | 38.475 | 24.978 | 1.00 | 57.15 | GZ00 | C |
| ATOM | 1615 | CD | LYS | C | 212 | 12.644 | 38.565 | 26.174 | 1.00 | 66.23 | GZ00 | C |
| ATOM | 1616 | CE | LYS | C | 212 | 12.433 | 39.861 | 26.969 | 1.00 | 79.81 | GZ00 | C |
| ATOM | 1617 | NZ | LYS | C | 212 | 13.705 | 40.467 | 27.503 | 1.00 | 78.51 | GZ00 | N1+ |
| ATOM | 1618 | N | VAL | C | 213 | 11.710 | 35.555 | 21.215 | 1.00 | 55.55 | GZ00 | N |
| ATOM | 1619 | CA | VAL | C | 213 | 11.839 | 34.287 | 20.520 | 1.00 | 49.66 | GZ00 | C |
| ATOM | 1620 | C | VAL | C | 213 | 13.319 | 34.021 | 20.272 | 1.00 | 50.74 | GZ00 | C |
| ATOM | 1621 | O | VAL | C | 213 | 14.050 | 34.906 | 19.810 | 1.00 | 48.29 | GZ00 | O |
| ATOM | 1622 | CB | VAL | C | 213 | 11.051 | 34.291 | 19.199 | 1.00 | 51.80 | GZ00 | C |
| ATOM | 1623 | CG1 | VAL | C | 213 | 11.260 | 32.982 | 18.441 | 1.00 | 52.63 | GZ00 | C |
| ATOM | 1624 | CG2 | VAL | C | 213 | 9.583 | 34.508 | 19.477 | 1.00 | 52.72 | GZ00 | C |
| ATOM | 1625 | N | ASP | C | 214 | 13.757 | 32.813 | 20.598 | 1.00 | 51.96 | GZ00 | N |
| ATOM | 1626 | CA | ASP | C | 214 | 15.054 | 32.301 | 20.199 | 1.00 | 43.41 | GZ00 | C |
| ATOM | 1627 | C | ASP | C | 214 | 14.791 | 31.231 | 19.160 | 1.00 | 52.09 | GZ00 | C |
| ATOM | 1628 | O | ASP | C | 214 | 13.923 | 30.374 | 19.356 | 1.00 | 60.39 | GZ00 | O |
| ATOM | 1629 | CB | ASP | C | 214 | 15.820 | 31.717 | 21.384 | 1.00 | 47.17 | GZ00 | C |
| ATOM | 1630 | CG | ASP | C | 214 | 16.093 | 32.742 | 22.473 | 1.00 | 53.34 | GZ00 | C |
| ATOM | 1631 | OD1 | ASP | C | 214 | 16.343 | 33.921 | 22.145 | 1.00 | 53.82 | GZ00 | O |

| ATOM | 1632 | OD2 | ASP | C 214 | 16.071 | 32.367 | 23.665 | 1.00 | 64.40 | GZ00 | O1- |
|------|------|-----|-----|-------|--------|--------|--------|------|-------|------|-----|
| ATOM | 1633 | N | LYS | C 215 | 15.527 | 31.282 | 18.060 | 1.00 | 49.22 | GZ00 | N |
| ATOM | 1634 | CA | LYS | C 215 | 15.322 | 30.324 | 16.993 | 1.00 | 46.64 | GZ00 | C |
| ATOM | 1635 | C | LYS | C 215 | 16.691 | 29.904 | 16.489 | 1.00 | 51.67 | GZ00 | C |
| ATOM | 1636 | O | LYS | C 215 | 17.531 | 30.748 | 16.165 | 1.00 | 50.73 | GZ00 | O |
| ATOM | 1637 | CB | LYS | C 215 | 14.459 | 30.916 | 15.877 | 1.00 | 41.93 | GZ00 | C |
| ATOM | 1638 | CG | LYS | C 215 | 14.213 | 29.983 | 14.699 | 1.00 | 58.37 | GZ00 | C |
| ATOM | 1639 | CD | LYS | C 215 | 13.161 | 28.894 | 14.993 | 1.00 | 57.43 | GZ00 | C |
| ATOM | 1640 | CE | LYS | C 215 | 11.848 | 29.473 | 15.498 | 1.00 | 54.91 | GZ00 | C |
| ATOM | 1641 | NZ | LYS | C 215 | 10.704 | 28.533 | 15.296 | 1.00 | 59.67 | GZ00 | N1+ |
| ATOM | 1642 | N | LYS | C 216 | 16.916 | 28.597 | 16.475 | 1.00 | 48.55 | GZ00 | N |
| ATOM | 1643 | CA | LYS | C 216 | 18.160 | 28.025 | 16.004 | 1.00 | 44.55 | GZ00 | C |
| ATOM | 1644 | C | LYS | C 216 | 18.075 | 27.873 | 14.498 | 1.00 | 51.23 | GZ00 | C |
| ATOM | 1645 | O | LYS | C 216 | 17.073 | 27.377 | 13.973 | 1.00 | 48.07 | GZ00 | O |
| ATOM | 1646 | CB | LYS | C 216 | 18.384 | 26.667 | 16.671 | 1.00 | 53.16 | GZ00 | C |
| ATOM | 1647 | CG | LYS | C 216 | 19.666 | 25.939 | 16.297 | 1.00 | 58.06 | GZ00 | C |
| ATOM | 1648 | CD | LYS | C 216 | 19.698 | 24.568 | 16.972 | 1.00 | 68.72 | GZ00 | C |
| ATOM | 1649 | CE | LYS | C 216 | 21.064 | 23.901 | 16.884 | 1.00 | 76.90 | GZ00 | C |
| ATOM | 1650 | NZ | LYS | C 216 | 21.133 | 22.703 | 17.775 | 1.00 | 81.06 | GZ00 | N1+ |
| ATOM | 1651 | N | VAL | C 217 | 19.122 | 28.296 | 13.802 | 1.00 | 42.74 | GZ00 | N |
| ATOM | 1652 | CA | VAL | C 217 | 19.158 | 28.228 | 12.355 | 1.00 | 45.91 | GZ00 | C |
| ATOM | 1653 | C | VAL | C 217 | 20.199 | 27.179 | 12.003 | 1.00 | 52.09 | GZ00 | C |
| ATOM | 1654 | O | VAL | C 217 | 21.399 | 27.371 | 12.243 | 1.00 | 50.43 | GZ00 | O |
| ATOM | 1655 | CB | VAL | C 217 | 19.472 | 29.594 | 11.727 | 1.00 | 47.85 | GZ00 | C |
| ATOM | 1656 | CG1 | VAL | C 217 | 19.352 | 29.534 | 10.217 | 1.00 | 36.68 | GZ00 | C |
| ATOM | 1657 | CG2 | VAL | C 217 | 18.552 | 30.667 | 12.314 | 1.00 | 38.98 | GZ00 | C |
| ATOM | 1658 | N | GLU | C 218 | 19.742 | 26.069 | 11.431 | 1.00 | 54.61 | GZ00 | N |
| ATOM | 1659 | CA | GLU | C 218 | 20.591 | 24.937 | 11.107 | 1.00 | 54.05 | GZ00 | C |
| ATOM | 1660 | C | GLU | C 218 | 20.622 | 24.755 | 9.603 | 1.00 | 49.13 | GZ00 | C |
| ATOM | 1661 | O | GLU | C 218 | 19.647 | 25.082 | 8.922 | 1.00 | 58.50 | GZ00 | O |
| ATOM | 1662 | CB | GLU | C 218 | 20.080 | 23.637 | 11.742 | 1.00 | 65.26 | GZ00 | C |
| ATOM | 1663 | CG | GLU | C 218 | 19.944 | 23.671 | 13.253 | 1.00 | 70.38 | GZ00 | C |
| ATOM | 1664 | CD | GLU | C 218 | 19.413 | 22.365 | 13.822 | 1.00 | 84.00 | GZ00 | C |
| ATOM | 1665 | OE1 | GLU | C 218 | 18.889 | 21.534 | 13.040 | 1.00 | 82.26 | GZ00 | O |
| ATOM | 1666 | OE2 | GLU | C 218 | 19.515 | 22.180 | 15.056 | 1.00 | 91.02 | GZ00 | O1- |
| ATOM | 1667 | N | PRO | C 219 | 21.716 | 24.232 | 9.056 | 1.00 | 57.01 | GZ00 | N |
| ATOM | 1668 | CA | PRO | C 219 | 21.755 | 23.952 | 7.612 | 1.00 | 53.03 | GZ00 | C |
| ATOM | 1669 | C | PRO | C 219 | 20.705 | 22.941 | 7.170 | 1.00 | 58.97 | GZ00 | C |
| ATOM | 1670 | O | PRO | C 219 | 19.991 | 22.384 | 8.008 | 1.00 | 62.86 | GZ00 | O |
| ATOM | 1671 | CB | PRO | C 219 | 23.185 | 23.443 | 7.393 | 1.00 | 49.20 | GZ00 | C |
| ATOM | 1672 | CG | PRO | C 219 | 23.796 | 23.307 | 8.761 | 1.00 | 47.12 | GZ00 | C |
| ATOM | 1673 | CD | PRO | C 219 | 23.051 | 24.186 | 9.671 | 1.00 | 55.30 | GZ00 | C |
| ATOM | 1674 | N | LYS | C 220 | 20.623 | 22.700 | 5.857 | 1.00 | 71.75 | GZ00 | N |
| ATOM | 1675 | CA | LYS | C 220 | 19.610 | 21.841 | 5.209 | 1.00 | 68.27 | GZ00 | C |
| ATOM | 1676 | C | LYS | C 220 | 18.226 | 22.491 | 5.306 | 1.00 | 73.79 | GZ00 | C |
| ATOM | 1677 | O | LYS | C 220 | 17.355 | 22.046 | 6.058 | 1.00 | 80.81 | GZ00 | O |
| ATOM | 1678 | CB | LYS | C 220 | 19.570 | 20.424 | 5.812 | 1.00 | 60.33 | GZ00 | C |
| ATOM | 1679 | CG | LYS | C 220 | 18.562 | 19.498 | 5.140 | 1.00 | 75.19 | GZ00 | C |
| ATOM | 1680 | CD | LYS | C 220 | 18.194 | 18.314 | 6.009 | 1.00 | 81.62 | GZ00 | C |
| ATOM | 1681 | CE | LYS | C 220 | 17.204 | 17.408 | 5.284 | 1.00 | 83.68 | GZ00 | C |
| ATOM | 1682 | NZ | LYS | C 220 | 15.922 | 18.107 | 4.974 | 1.00 | 81.30 | GZ00 | N1+ |
| TER | | | | | | | | | | | |
| ATOM | 1683 | N | GLN | D 1 | 3.622 | 57.068 | -14.037 | 1.00 | 73.81 | | N |
| ATOM | 1684 | CA | GLN | D 1 | 2.256 | 57.519 | -13.830 | 1.00 | 77.03 | | C |
| ATOM | 1685 | C | GLN | D 1 | 2.220 | 58.975 | -13.340 | 1.00 | 71.19 | | C |
| ATOM | 1686 | O | GLN | D 1 | 1.170 | 59.618 | -13.403 | 1.00 | 70.55 | | O |
| ATOM | 1687 | CB | GLN | D 1 | 1.535 | 56.594 | -12.844 | 1.00 | 76.72 | | C |
| ATOM | 1688 | CG | GLN | D 1 | 0.016 | 56.704 | -12.856 | 1.00 | 83.42 | | C |
| ATOM | 1689 | CD | GLN | D 1 | -0.568 | 56.892 | -11.458 | 1.00 | 94.69 | | C |
| ATOM | 1690 | OE1 | GLN | D 1 | 0.127 | 56.720 | -10.448 | 1.00 | 86.56 | | O |
| ATOM | 1691 | NE2 | GLN | D 1 | -1.851 | 57.255 | -11.395 | 1.00 | 91.59 | | N |
| ATOM | 1692 | N | SER | D 2 | 3.352 | 59.504 | -12.865 | 1.00 | 62.32 | | N |
| ATOM | 1693 | CA | SER | D 2 | 3.404 | 60.928 | -12.539 | 1.00 | 62.06 | | C |
| ATOM | 1694 | C | SER | D 2 | 3.359 | 61.752 | -13.826 | 1.00 | 54.47 | | C |
| ATOM | 1695 | O | SER | D 2 | 3.840 | 61.331 | -14.885 | 1.00 | 54.37 | | O |
| ATOM | 1696 | CB | SER | D 2 | 4.637 | 61.269 | -11.685 | 1.00 | 55.55 | | C |
| ATOM | 1697 | OG | SER | D 2 | 5.815 | 61.429 | -12.445 | 1.00 | 44.68 | | O |
| ATOM | 1698 | N | VAL | D 3 | 2.765 | 62.942 | -13.725 | 1.00 | 51.09 | | N |
| ATOM | 1699 | CA | VAL | D 3 | 2.399 | 63.693 | -14.926 | 1.00 | 44.98 | | C |
| ATOM | 1700 | C | VAL | D 3 | 3.625 | 64.277 | -15.623 | 1.00 | 44.14 | | C |
| ATOM | 1701 | O | VAL | D 3 | 3.682 | 64.317 | -16.858 | 1.00 | 42.24 | | O |

```
ATOM   1702  CB   VAL D   3      1.355  64.762 -14.561  1.00 48.49           C
ATOM   1703  CG1  VAL D   3      1.035  65.656 -15.762  1.00 33.38           C
ATOM   1704  CG2  VAL D   3      0.095  64.072 -13.994  1.00 36.61           C
ATOM   1705  N    LEU D   4      4.610  64.757 -14.867  1.00 40.88           N
ATOM   1706  CA   LEU D   4      5.895  65.115 -15.451  1.00 38.31           C
ATOM   1707  C    LEU D   4      6.842  63.946 -15.255  1.00 42.46           C
ATOM   1708  O    LEU D   4      6.841  63.318 -14.195  1.00 41.68           O
ATOM   1709  CB   LEU D   4      6.484  66.365 -14.808  1.00 36.49           C
ATOM   1710  CG   LEU D   4      5.476  67.494 -14.624  1.00 34.13           C
ATOM   1711  CD1  LEU D   4      6.074  68.656 -13.825  1.00 34.44           C
ATOM   1712  CD2  LEU D   4      4.978  67.953 -15.972  1.00 29.23           C
ATOM   1713  N    THR D   5      7.637  63.640 -16.273  1.00 39.33           N
ATOM   1714  CA   THR D   5      8.541  62.503 -16.203  1.00 34.69           C
ATOM   1715  C    THR D   5      9.987  62.990 -16.144  1.00 41.85           C
ATOM   1716  O    THR D   5     10.420  63.814 -16.959  1.00 32.54           O
ATOM   1717  CB   THR D   5      8.307  61.529 -17.363  1.00 40.41           C
ATOM   1718  OG1  THR D   5      8.542  62.190 -18.596  1.00 58.56           O
ATOM   1719  CG2  THR D   5      6.858  61.060 -17.366  1.00 35.80           C
ATOM   1720  N    GLN D   6     10.703  62.522 -15.132  1.00 33.22           N
ATOM   1721  CA   GLN D   6     12.100  62.739 -14.872  1.00 31.46           C
ATOM   1722  C    GLN D   6     12.802  61.390 -14.902  1.00 35.85           C
ATOM   1723  O    GLN D   6     12.187  60.381 -14.554  1.00 35.36           O
ATOM   1724  CB   GLN D   6     12.302  63.370 -13.490  1.00 31.54           C
ATOM   1725  CG   GLN D   6     11.670  64.731 -13.302  1.00 32.96           C
ATOM   1726  CD   GLN D   6     11.827  65.238 -11.884  1.00 38.63           C
ATOM   1727  OE1  GLN D   6     10.841  65.645 -11.248  1.00 36.32           O
ATOM   1728  NE2  GLN D   6     13.073  65.220 -11.367  1.00 31.40           N
ATOM   1729  N    PRO D   7     14.069  61.323 -15.306  1.00 39.24           N
ATOM   1730  CA   PRO D   7     14.832  60.087 -15.078  1.00 33.07           C
ATOM   1731  C    PRO D   7     14.881  59.791 -13.588  1.00 34.60           C
ATOM   1732  O    PRO D   7     15.015  60.714 -12.764  1.00 32.93           O
ATOM   1733  CB   PRO D   7     16.235  60.402 -15.652  1.00 31.22           C
ATOM   1734  CG   PRO D   7     16.356  61.910 -15.589  1.00 34.93           C
ATOM   1735  CD   PRO D   7     14.921  62.430 -15.806  1.00 35.72           C
ATOM   1736  N    PRO D   8     14.755  58.518 -13.197  1.00 33.41           N
ATOM   1737  CA   PRO D   8     14.682  58.200 -11.756  1.00 33.71           C
ATOM   1738  C    PRO D   8     15.987  58.443 -11.027  1.00 36.29           C
ATOM   1739  O    PRO D   8     15.966  58.847  -9.855  1.00 35.95           O
ATOM   1740  CB   PRO D   8     14.299  56.705 -11.730  1.00 31.63           C
ATOM   1741  CG   PRO D   8     14.777  56.181 -13.032  1.00 33.24           C
ATOM   1742  CD   PRO D   8     14.568  57.333 -14.040  1.00 28.70           C
ATOM   1743  N    SER D   9     17.128  58.252 -11.679  1.00 31.20           N
ATOM   1744  CA   SER D   9     18.367  58.463 -10.945  1.00 41.37           C
ATOM   1745  C    SER D   9     19.485  58.910 -11.869  1.00 41.68           C
ATOM   1746  O    SER D   9     19.492  58.642 -13.075  1.00 39.62           O
ATOM   1747  CB   SER D   9     18.788  57.197 -10.195  1.00 32.55           C
ATOM   1748  OG   SER D   9     18.984  56.168 -11.129  1.00 46.27           O
ATOM   1749  N    VAL D  10     20.465  59.553 -11.259  1.00 35.48           N
ATOM   1750  CA   VAL D  10     21.529  60.199 -12.000  1.00 37.87           C
ATOM   1751  C    VAL D  10     22.736  60.253 -11.082  1.00 34.78           C
ATOM   1752  O    VAL D  10     22.605  60.523  -9.880  1.00 36.71           O
ATOM   1753  CB   VAL D  10     21.041  61.588 -12.472  1.00 40.73           C
ATOM   1754  CG1  VAL D  10     21.926  62.691 -11.971  1.00 40.07           C
ATOM   1755  CG2  VAL D  10     20.845  61.613 -13.973  1.00 38.95           C
ATOM   1756  N    SER D  11     23.915  59.963 -11.625  1.00 37.46           N
ATOM   1757  CA   SER D  11     25.092  59.962 -10.762  1.00 35.14           C
ATOM   1758  C    SER D  11     26.324  60.390 -11.542  1.00 31.82           C
ATOM   1759  O    SER D  11     26.474  60.057 -12.715  1.00 37.48           O
ATOM   1760  CB   SER D  11     25.311  58.580 -10.130  1.00 33.24           C
ATOM   1761  OG   SER D  11     25.640  57.636 -11.125  1.00 37.23           O
ATOM   1762  N    ALA D  12     27.196  61.139 -10.877  1.00 35.26           N
ATOM   1763  CA   ALA D  12     28.433  61.620 -11.469  1.00 34.57           C
ATOM   1764  C    ALA D  12     29.379  61.996 -10.338  1.00 39.68           C
ATOM   1765  O    ALA D  12     28.955  62.218  -9.199  1.00 31.45           O
ATOM   1766  CB   ALA D  12     28.200  62.814 -12.409  1.00 31.29           C
ATOM   1767  N    ALA D  13     30.673  62.071 -10.679  1.00 36.93           N
ATOM   1768  CA   ALA D  13     31.748  62.366  -9.733  1.00 40.49           C
ATOM   1769  C    ALA D  13     31.847  63.860  -9.433  1.00 40.25           C
ATOM   1770  O    ALA D  13     31.411  64.695 -10.236  1.00 39.24           O
ATOM   1771  CB   ALA D  13     33.081  61.875 -10.291  1.00 30.80           C
ATOM   1772  N    PRO D  14     32.434  64.225  -8.288  1.00 33.52           N
```

```
ATOM   1773  CA   PRO D  14      32.647  65.646   -7.994  1.00 38.76           C
ATOM   1774  C    PRO D  14      33.382  66.324   -9.141  1.00 44.78           C
ATOM   1775  O    PRO D  14      34.233  65.718   -9.798  1.00 36.43           O
ATOM   1776  CB   PRO D  14      33.485  65.629   -6.711  1.00 35.31           C
ATOM   1777  CG   PRO D  14      33.152  64.283   -6.060  1.00 30.30           C
ATOM   1778  CD   PRO D  14      32.928  63.345   -7.208  1.00 32.96           C
ATOM   1779  N    GLY D  15      33.008  67.579   -9.410  1.00 45.92           N
ATOM   1780  CA   GLY D  15      33.585  68.342  -10.484  1.00 34.84           C
ATOM   1781  C    GLY D  15      32.932  68.138  -11.829  1.00 41.92           C
ATOM   1782  O    GLY D  15      33.143  68.956  -12.728  1.00 49.13           O
ATOM   1783  N    GLN D  16      32.143  67.083  -12.001  1.00 37.37           N
ATOM   1784  CA   GLN D  16      31.533  66.844  -13.302  1.00 48.89           C
ATOM   1785  C    GLN D  16      30.220  67.627  -13.500  1.00 47.03           C
ATOM   1786  O    GLN D  16      29.744  68.367  -12.628  1.00 39.51           O
ATOM   1787  CB   GLN D  16      31.306  65.355  -13.502  1.00 50.09           C
ATOM   1788  CG   GLN D  16      32.570  64.589  -13.809  1.00 57.03           C
ATOM   1789  CD   GLN D  16      32.260  63.311  -14.566  1.00 75.65           C
ATOM   1790  OE1  GLN D  16      31.830  62.302  -13.979  1.00 62.08           O
ATOM   1791  NE2  GLN D  16      32.445  63.353  -15.886  1.00 86.71           N
ATOM   1792  N    LYS D  17      29.674  67.481  -14.711  1.00 50.37           N
ATOM   1793  CA   LYS D  17      28.424  68.054  -15.189  1.00 47.34           C
ATOM   1794  C    LYS D  17      27.325  67.004  -15.183  1.00 51.01           C
ATOM   1795  O    LYS D  17      27.566  65.822  -15.440  1.00 57.21           O
ATOM   1796  CB   LYS D  17      28.537  68.558  -16.635  1.00 48.76           C
ATOM   1797  CG   LYS D  17      29.325  69.812  -16.889  1.00 66.24           C
ATOM   1798  CD   LYS D  17      29.537  69.967  -18.408  1.00 76.78           C
ATOM   1799  CE   LYS D  17      30.502  71.109  -18.742  1.00 85.45           C
ATOM   1800  NZ   LYS D  17      30.739  71.204  -20.209  1.00 85.79           N
ATOM   1801  N   AVAL D  18      26.098  67.456  -14.947  0.60 46.54           N
ATOM   1802  CA  AVAL D  18      24.936  66.572  -14.935  0.60 45.04           C
ATOM   1803  C   AVAL D  18      23.728  67.369  -15.412  0.60 42.17           C
ATOM   1804  O   AVAL D  18      23.622  68.572  -15.150  0.60 42.21           O
ATOM   1805  CB  AVAL D  18      24.745  65.964  -13.527  0.60 43.33           C
ATOM   1806  CG1 AVAL D  18      23.328  66.101  -13.049  0.60 41.29           C
ATOM   1807  CG2 AVAL D  18      25.187  64.524  -13.516  0.60 41.57           C
ATOM   1808  N   BVAL D  18      26.101  67.445  -14.920  0.40 46.52           N
ATOM   1809  CA  BVAL D  18      24.961  66.551  -15.030  0.40 45.04           C
ATOM   1810  C   BVAL D  18      23.744  67.363  -15.441  0.40 42.19           C
ATOM   1811  O   BVAL D  18      23.643  68.561  -15.158  0.40 42.16           O
ATOM   1812  CB  BVAL D  18      24.729  65.769  -13.718  0.40 43.17           C
ATOM   1813  CG1 BVAL D  18      24.057  66.644  -12.663  0.40 39.96           C
ATOM   1814  CG2 BVAL D  18      23.942  64.530  -14.005  0.40 42.54           C
ATOM   1815  N    THR D  19      22.831  66.698  -16.143  1.00 46.67           N
ATOM   1816  CA   THR D  19      21.594  67.285  -16.650  1.00 42.21           C
ATOM   1817  C    THR D  19      20.412  66.449  -16.178  1.00 42.60           C
ATOM   1818  O    THR D  19      20.469  65.212  -16.217  1.00 40.66           O
ATOM   1819  CB   THR D  19      21.646  67.356  -18.189  1.00 36.82           C
ATOM   1820  OG1  THR D  19      21.854  68.714  -18.572  1.00 49.09           O
ATOM   1821  CG2  THR D  19      20.396  66.796  -18.859  1.00 40.02           C
ATOM   1822  N    ILE D  20      19.343  67.117  -15.733  1.00 35.88           N
ATOM   1823  CA   ILE D  20      18.132  66.439  -15.267  1.00 33.46           C
ATOM   1824  C    ILE D  20      16.954  66.997  -16.042  1.00 38.36           C
ATOM   1825  O    ILE D  20      16.680  68.200  -15.976  1.00 36.03           O
ATOM   1826  CB   ILE D  20      17.907  66.602  -13.753  1.00 38.27           C
ATOM   1827  CG1  ILE D  20      19.043  65.927  -12.976  1.00 32.31           C
ATOM   1828  CG2  ILE D  20      16.555  65.993  -13.341  1.00 34.30           C
ATOM   1829  CD1  ILE D  20      19.010  66.199  -11.490  1.00 28.88           C
ATOM   1830  N    SER D  21      16.253  66.127  -16.764  1.00 31.98           N
ATOM   1831  CA   SER D  21      15.188  66.575  -17.643  1.00 36.18           C
ATOM   1832  C    SER D  21      13.814  66.346  -17.011  1.00 40.44           C
ATOM   1833  O    SER D  21      13.639  65.541  -16.094  1.00 35.10           O
ATOM   1834  CB   SER D  21      15.267  65.862  -18.988  1.00 35.20           C
ATOM   1835  OG   SER D  21      15.097  64.461  -18.840  1.00 43.09           O
ATOM   1836  N    CYS D  22      12.829  67.052  -17.552  1.00 32.32           N
ATOM   1837  CA   CYS D  22      11.476  67.044  -17.012  1.00 36.57           C
ATOM   1838  C    CYS D  22      10.549  67.317  -18.192  1.00 36.72           C
ATOM   1839  O    CYS D  22      10.444  68.454  -18.656  1.00 33.99           O
ATOM   1840  CB   CYS D  22      11.339  68.103  -15.933  1.00 34.58           C
ATOM   1841  SG   CYS D  22       9.650  68.451  -15.371  1.00 54.36           S
ATOM   1842  N    SER D  23       9.915  66.282  -18.703  1.00 34.27           N
ATOM   1843  CA   SER D  23       9.059  66.460  -19.859  1.00 42.72           C
```

```
ATOM   1844  C    SER D  23      7.582  66.345 -19.454  1.00 41.50           C
ATOM   1845  O    SER D  23      7.209  65.545 -18.582  1.00 29.92           O
ATOM   1846  CB   SER D  23      9.450  65.476 -20.954  1.00 34.92           C
ATOM   1847  OG   SER D  23      8.893  64.230 -20.677  1.00 50.39           O
ATOM   1848  N    GLY D  24      6.765  67.218 -20.037  1.00 36.33           N
ATOM   1849  CA   GLY D  24      5.336  67.237 -19.809  1.00 38.73           C
ATOM   1850  C    GLY D  24      4.600  67.510 -21.103  1.00 39.14           C
ATOM   1851  O    GLY D  24      4.942  66.945 -22.139  1.00 42.42           O
ATOM   1852  N    SER D  25      3.620  68.406 -21.078  1.00 36.67           N
ATOM   1853  CA   SER D  25      2.743  68.598 -22.221  1.00 39.25           C
ATOM   1854  C    SER D  25      2.382  70.072 -22.346  1.00 38.01           C
ATOM   1855  O    SER D  25      2.754  70.902 -21.506  1.00 33.82           O
ATOM   1856  CB   SER D  25      1.496  67.732 -22.067  1.00 41.16           C
ATOM   1857  OG   SER D  25      0.709  68.227 -20.994  1.00 50.29           O
ATOM   1858  N    SER D  26      1.657  70.404 -23.420  1.00 31.89           N
ATOM   1859  CA   SER D  26      1.329  71.809 -23.656  1.00 36.67           C
ATOM   1860  C    SER D  26      0.486  72.395 -22.527  1.00 38.14           C
ATOM   1861  O    SER D  26      0.548  73.600 -22.271  1.00 38.11           O
ATOM   1862  CB   SER D  26      0.624  71.976 -25.002  1.00 31.63           C
ATOM   1863  OG   SER D  26     -0.356  70.980 -25.146  1.00 53.99           O
ATOM   1864  N    SER D  27     -0.279  71.575 -21.818  1.00 33.81           N
ATOM   1865  CA   SER D  27     -1.109  72.150 -20.773  1.00 37.80           C
ATOM   1866  C    SER D  27     -0.387  72.281 -19.431  1.00 40.08           C
ATOM   1867  O    SER D  27     -0.954  72.862 -18.498  1.00 36.37           O
ATOM   1868  CB   SER D  27     -2.393  71.336 -20.608  1.00 31.40           C
ATOM   1869  OG   SER D  27     -2.088  70.014 -20.236  1.00 50.96           O
ATOM   1870  N    ASN D  28      0.839  71.765 -19.295  1.00 34.89           N
ATOM   1871  CA   ASN D  28      1.555  72.073 -18.068  1.00 33.57           C
ATOM   1872  C    ASN D  28      2.831  72.854 -18.389  1.00 34.78           C
ATOM   1873  O    ASN D  28      2.800  74.091 -18.405  1.00 36.75           O
ATOM   1874  CB   ASN D  28      1.817  70.808 -17.214  1.00 34.30           C
ATOM   1875  CG   ASN D  28      2.277  69.580 -18.027  1.00 34.72           C
ATOM   1876  OD1  ASN D  28      3.345  69.568 -18.641  1.00 33.73           O
ATOM   1877  ND2  ASN D  28      1.500  68.515 -17.949  1.00 35.11           N
ATOM   1878  N    ILE D  29      3.952  72.171 -18.633  1.00 33.54           N
ATOM   1879  CA   ILE D  29      5.218  72.866 -18.877  1.00 34.11           C
ATOM   1880  C    ILE D  29      5.107  73.810 -20.072  1.00 36.11           C
ATOM   1881  O    ILE D  29      5.691  74.897 -20.082  1.00 33.26           O
ATOM   1882  CB   ILE D  29      6.350  71.840 -19.063  1.00 33.30           C
ATOM   1883  CG1  ILE D  29      6.604  71.107 -17.741  1.00 32.39           C
ATOM   1884  CG2  ILE D  29      7.621  72.520 -19.555  1.00 25.88           C
ATOM   1885  CD1  ILE D  29      7.724  70.138 -17.806  1.00 36.95           C
ATOM   1886  N    GLY D  30      4.368  73.412 -21.103  1.00 38.51           N
ATOM   1887  CA   GLY D  30      4.274  74.237 -22.289  1.00 30.45           C
ATOM   1888  C    GLY D  30      3.608  75.580 -22.062  1.00 38.50           C
ATOM   1889  O    GLY D  30      3.763  76.475 -22.889  1.00 40.64           O
ATOM   1890  N    ASN D  31      2.836  75.744 -20.987  1.00 37.18           N
ATOM   1891  CA   ASN D  31      2.178  77.031 -20.815  1.00 37.16           C
ATOM   1892  C    ASN D  31      2.192  77.595 -19.395  1.00 36.18           C
ATOM   1893  O    ASN D  31      1.488  78.576 -19.137  1.00 42.11           O
ATOM   1894  CB   ASN D  31      0.731  76.942 -21.340  1.00 41.60           C
ATOM   1895  CG   ASN D  31      0.681  77.025 -22.867  1.00 53.09           C
ATOM   1896  OD1  ASN D  31      0.759  78.110 -23.442  1.00 63.01           O
ATOM   1897  ND2  ASN D  31      0.591  75.877 -23.525  1.00 45.20           N
ATOM   1898  N    ASN D  32      2.985  77.059 -18.471  1.00 38.29           N
ATOM   1899  CA   ASN D  32      3.026  77.622 -17.128  1.00 35.50           C
ATOM   1900  C    ASN D  32      4.467  77.757 -16.662  1.00 33.99           C
ATOM   1901  O    ASN D  32      5.394  77.247 -17.291  1.00 37.19           O
ATOM   1902  CB   ASN D  32      2.192  76.787 -16.158  1.00 29.83           C
ATOM   1903  CG   ASN D  32      0.731  76.754 -16.548  1.00 35.04           C
ATOM   1904  OD1  ASN D  32      0.003  77.720 -16.321  1.00 41.43           O
ATOM   1905  ND2  ASN D  32      0.299  75.657 -17.166  1.00 38.66           N
ATOM   1906  N    TYR D  33      4.647  78.497 -15.572  1.00 30.88           N
ATOM   1907  CA   TYR D  33      5.973  78.725 -15.020  1.00 31.61           C
ATOM   1908  C    TYR D  33      6.529  77.443 -14.422  1.00 39.01           C
ATOM   1909  O    TYR D  33      5.815  76.704 -13.751  1.00 35.01           O
ATOM   1910  CB   TYR D  33      5.925  79.804 -13.951  1.00 27.05           C
ATOM   1911  CG   TYR D  33      5.492  81.136 -14.476  1.00 32.55           C
ATOM   1912  CD1  TYR D  33      6.214  81.780 -15.478  1.00 29.14           C
ATOM   1913  CD2  TYR D  33      4.358  81.770 -13.965  1.00 38.90           C
ATOM   1914  CE1  TYR D  33      5.805  83.036 -15.967  1.00 36.47           C
```

| ATOM | 1915 | CE2 | TYR | D | 33 | 3.948 | 83.013 | -14.438 | 1.00 | 33.42 | C |
|------|------|-----|-----|---|----|--------|---------|---------|------|-------|---|
| ATOM | 1916 | CZ | TYR | D | 33 | 4.671 | 83.642 | -15.438 | 1.00 | 37.47 | C |
| ATOM | 1917 | OH | TYR | D | 33 | 4.246 | 84.866 | -15.903 | 1.00 | 36.22 | O |
| ATOM | 1918 | N | VAL | D | 34 | 7.821 | 77.195 | -14.623 | 1.00 | 33.97 | N |
| ATOM | 1919 | CA | VAL | D | 34 | 8.440 | 75.966 | -14.138 | 1.00 | 32.04 | C |
| ATOM | 1920 | C | VAL | D | 34 | 9.272 | 76.250 | -12.896 | 1.00 | 31.06 | C |
| ATOM | 1921 | O | VAL | D | 34 | 9.986 | 77.254 | -12.832 | 1.00 | 32.29 | O |
| ATOM | 1922 | CB | VAL | D | 34 | 9.292 | 75.308 | -15.236 | 1.00 | 33.98 | C |
| ATOM | 1923 | CG1 | VAL | D | 34 | 10.002 | 74.100 | -14.684 | 1.00 | 30.92 | C |
| ATOM | 1924 | CG2 | VAL | D | 34 | 8.407 | 74.884 | -16.397 | 1.00 | 34.56 | C |
| ATOM | 1925 | N | SER | D | 35 | 9.166 | 75.373 | -11.896 | 1.00 | 31.29 | N |
| ATOM | 1926 | CA | SER | D | 35 | 9.991 | 75.425 | -10.697 | 1.00 | 30.60 | C |
| ATOM | 1927 | C | SER | D | 35 | 10.791 | 74.134 | -10.560 | 1.00 | 35.34 | C |
| ATOM | 1928 | O | SER | D | 35 | 10.367 | 73.068 | -11.017 | 1.00 | 31.50 | O |
| ATOM | 1929 | CB | SER | D | 35 | 9.166 | 75.614 | -9.423 | 1.00 | 26.83 | C |
| ATOM | 1930 | OG | SER | D | 35 | 8.548 | 76.889 | -9.407 | 1.00 | 31.37 | O |
| ATOM | 1931 | N | TRP | D | 36 | 11.967 | 74.248 | -9.938 | 1.00 | 31.19 | N |
| ATOM | 1932 | CA | TRP | D | 36 | 12.774 | 73.104 | -9.559 | 1.00 | 30.26 | C |
| ATOM | 1933 | C | TRP | D | 36 | 12.996 | 73.142 | -8.060 | 1.00 | 29.04 | C |
| ATOM | 1934 | O | TRP | D | 36 | 13.286 | 74.196 | -7.491 | 1.00 | 29.53 | O |
| ATOM | 1935 | CB | TRP | D | 36 | 14.112 | 73.084 | -10.274 | 1.00 | 25.90 | C |
| ATOM | 1936 | CG | TRP | D | 36 | 14.009 | 72.705 | -11.713 | 1.00 | 32.87 | C |
| ATOM | 1937 | CD1 | TRP | D | 36 | 13.867 | 73.552 | -12.773 | 1.00 | 33.05 | C |
| ATOM | 1938 | CD2 | TRP | D | 36 | 14.049 | 71.383 | -12.259 | 1.00 | 32.92 | C |
| ATOM | 1939 | NE1 | TRP | D | 36 | 13.812 | 72.835 | -13.947 | 1.00 | 33.23 | N |
| ATOM | 1940 | CE2 | TRP | D | 36 | 13.941 | 71.505 | -13.660 | 1.00 | 29.67 | C |
| ATOM | 1941 | CE3 | TRP | D | 36 | 14.187 | 70.108 | -11.701 | 1.00 | 32.52 | C |
| ATOM | 1942 | CZ2 | TRP | D | 36 | 13.946 | 70.401 | -14.512 | 1.00 | 32.64 | C |
| ATOM | 1943 | CZ3 | TRP | D | 36 | 14.185 | 69.012 | -12.546 | 1.00 | 30.06 | C |
| ATOM | 1944 | CH2 | TRP | D | 36 | 14.058 | 69.166 | -13.936 | 1.00 | 35.04 | C |
| ATOM | 1945 | N | TYR | D | 37 | 12.874 | 71.982 | -7.429 | 1.00 | 31.89 | N |
| ATOM | 1946 | CA | TYR | D | 37 | 13.051 | 71.848 | -5.995 | 1.00 | 29.68 | C |
| ATOM | 1947 | C | TYR | D | 37 | 14.168 | 70.859 | -5.693 | 1.00 | 36.72 | C |
| ATOM | 1948 | O | TYR | D | 37 | 14.324 | 69.839 | -6.383 | 1.00 | 33.31 | O |
| ATOM | 1949 | CB | TYR | D | 37 | 11.735 | 71.417 | -5.343 | 1.00 | 28.40 | C |
| ATOM | 1950 | CG | TYR | D | 37 | 10.598 | 72.378 | -5.659 | 1.00 | 30.46 | C |
| ATOM | 1951 | CD1 | TYR | D | 37 | 10.417 | 73.540 | -4.921 | 1.00 | 27.52 | C |
| ATOM | 1952 | CD2 | TYR | D | 37 | 9.723 | 72.130 | -6.719 | 1.00 | 29.85 | C |
| ATOM | 1953 | CE1 | TYR | D | 37 | 9.368 | 74.423 | -5.219 | 1.00 | 32.37 | C |
| ATOM | 1954 | CE2 | TYR | D | 37 | 8.691 | 72.993 | -7.019 | 1.00 | 27.34 | C |
| ATOM | 1955 | CZ | TYR | D | 37 | 8.517 | 74.137 | -6.271 | 1.00 | 30.55 | C |
| ATOM | 1956 | OH | TYR | D | 37 | 7.495 | 74.995 | -6.595 | 1.00 | 35.11 | O |
| ATOM | 1957 | N | GLN | D | 38 | 14.938 | 71.174 | -4.653 | 1.00 | 32.56 | N |
| ATOM | 1958 | CA | GLN | D | 38 | 16.019 | 70.332 | -4.174 | 1.00 | 30.13 | C |
| ATOM | 1959 | C | GLN | D | 38 | 15.690 | 69.837 | -2.770 | 1.00 | 33.68 | C |
| ATOM | 1960 | O | GLN | D | 38 | 15.362 | 70.635 | -1.880 | 1.00 | 31.93 | O |
| ATOM | 1961 | CB | GLN | D | 38 | 17.349 | 71.102 | -4.170 | 1.00 | 30.01 | C |
| ATOM | 1962 | CG | GLN | D | 38 | 18.527 | 70.323 | -3.564 | 1.00 | 28.04 | C |
| ATOM | 1963 | CD | GLN | D | 38 | 19.729 | 71.214 | -3.280 | 1.00 | 34.96 | C |
| ATOM | 1964 | OE1 | GLN | D | 38 | 19.664 | 72.147 | -2.473 | 1.00 | 32.51 | O |
| ATOM | 1965 | NE2 | GLN | D | 38 | 20.828 | 70.944 | -3.971 | 1.00 | 33.81 | N |
| ATOM | 1966 | N | GLN | D | 39 | 15.790 | 68.528 | -2.572 | 1.00 | 32.31 | N |
| ATOM | 1967 | CA | GLN | D | 39 | 15.552 | 67.918 | -1.266 | 1.00 | 38.47 | C |
| ATOM | 1968 | C | GLN | D | 39 | 16.805 | 67.162 | -0.843 | 1.00 | 33.22 | C |
| ATOM | 1969 | O | GLN | D | 39 | 17.057 | 66.051 | -1.324 | 1.00 | 33.95 | O |
| ATOM | 1970 | CB | GLN | D | 39 | 14.336 | 67.001 | -1.294 | 1.00 | 32.82 | C |
| ATOM | 1971 | CG | GLN | D | 39 | 13.941 | 66.543 | 0.090 | 1.00 | 38.29 | C |
| ATOM | 1972 | CD | GLN | D | 39 | 12.621 | 65.809 | 0.118 | 1.00 | 34.34 | C |
| ATOM | 1973 | OE1 | GLN | D | 39 | 12.221 | 65.165 | -0.862 | 1.00 | 38.36 | O |
| ATOM | 1974 | NE2 | GLN | D | 39 | 11.909 | 65.942 | 1.236 | 1.00 | 30.94 | N |
| ATOM | 1975 | N | LEU | D | 40 | 17.612 | 67.779 | 0.023 | 1.00 | 37.41 | N |
| ATOM | 1976 | CA | LEU | D | 40 | 18.766 | 67.069 | 0.571 | 1.00 | 41.50 | C |
| ATOM | 1977 | C | LEU | D | 40 | 18.281 | 65.913 | 1.450 | 1.00 | 44.75 | C |
| ATOM | 1978 | O | LEU | D | 40 | 17.202 | 65.988 | 2.051 | 1.00 | 39.49 | O |
| ATOM | 1979 | CB | LEU | D | 40 | 19.659 | 68.020 | 1.368 | 1.00 | 41.85 | C |
| ATOM | 1980 | CG | LEU | D | 40 | 20.144 | 69.262 | 0.592 | 1.00 | 42.10 | C |
| ATOM | 1981 | CD1 | LEU | D | 40 | 20.614 | 70.363 | 1.515 | 1.00 | 38.12 | C |
| ATOM | 1982 | CD2 | LEU | D | 40 | 21.236 | 68.903 | -0.380 | 1.00 | 31.04 | C |
| ATOM | 1983 | N | PRO | D | 41 | 19.045 | 64.827 | 1.520 | 1.00 | 47.88 | N |
| ATOM | 1984 | CA | PRO | D | 41 | 18.568 | 63.625 | 2.225 | 1.00 | 42.38 | C |
| ATOM | 1985 | C | PRO | D | 41 | 18.152 | 63.935 | 3.656 | 1.00 | 45.34 | C |

| ATOM | 1986 | O | PRO D | 41 | 18.876 | 64.603 | 4.404 | 1.00 46.53 | O |
|---|---|---|---|---|---|---|---|---|---|
| ATOM | 1987 | CB | PRO D | 41 | 19.780 | 62.691 | 2.182 | 1.00 43.44 | C |
| ATOM | 1988 | CG | PRO D | 41 | 20.639 | 63.217 | 1.062 | 1.00 46.87 | C |
| ATOM | 1989 | CD | PRO D | 41 | 20.427 | 64.690 | 1.030 | 1.00 42.00 | C |
| ATOM | 1990 | N | GLY D | 42 | 16.963 | 63.459 | 4.029 | 1.00 38.47 | N |
| ATOM | 1991 | CA | GLY D | 42 | 16.400 | 63.729 | 5.346 | 1.00 39.47 | C |
| ATOM | 1992 | C | GLY D | 42 | 15.980 | 65.164 | 5.648 | 1.00 51.21 | C |
| ATOM | 1993 | O | GLY D | 42 | 15.801 | 65.501 | 6.822 | 1.00 48.36 | O |
| ATOM | 1994 | N | THR D | 43 | 15.793 | 66.022 | 4.642 | 1.00 44.85 | N |
| ATOM | 1995 | CA | THR D | 43 | 15.393 | 67.415 | 4.867 | 1.00 47.53 | C |
| ATOM | 1996 | C | THR D | 43 | 14.123 | 67.747 | 4.090 | 1.00 39.84 | C |
| ATOM | 1997 | O | THR D | 43 | 13.599 | 66.944 | 3.315 | 1.00 42.92 | O |
| ATOM | 1998 | CB | THR D | 43 | 16.503 | 68.414 | 4.475 | 1.00 45.75 | C |
| ATOM | 1999 | OG1 | THR D | 43 | 16.602 | 68.488 | 3.045 | 1.00 36.10 | O |
| ATOM | 2000 | CG2 | THR D | 43 | 17.857 | 67.992 | 5.056 | 1.00 41.52 | C |
| ATOM | 2001 | N | ALA D | 44 | 13.619 | 68.952 | 4.318 | 1.00 38.36 | N |
| ATOM | 2002 | CA | ALA D | 44 | 12.484 | 69.426 | 3.553 | 1.00 38.31 | C |
| ATOM | 2003 | C | ALA D | 44 | 12.916 | 69.799 | 2.137 | 1.00 39.83 | C |
| ATOM | 2004 | O | ALA D | 44 | 14.100 | 70.032 | 1.878 | 1.00 37.35 | O |
| ATOM | 2005 | CB | ALA D | 44 | 11.849 | 70.632 | 4.238 | 1.00 38.73 | C |
| ATOM | 2006 | N | PRO D | 45 | 11.977 | 69.838 | 1.194 | 1.00 36.33 | N |
| ATOM | 2007 | CA | PRO D | 45 | 12.277 | 70.462 | -0.097 | 1.00 35.97 | C |
| ATOM | 2008 | C | PRO D | 45 | 12.678 | 71.911 | 0.121 | 1.00 33.44 | C |
| ATOM | 2009 | O | PRO D | 45 | 12.384 | 72.519 | 1.155 | 1.00 33.83 | O |
| ATOM | 2010 | CB | PRO D | 45 | 10.948 | 70.365 | -0.872 | 1.00 28.82 | C |
| ATOM | 2011 | CG | PRO D | 45 | 10.265 | 69.198 | -0.245 | 1.00 36.55 | C |
| ATOM | 2012 | CD | PRO D | 45 | 10.615 | 69.281 | 1.225 | 1.00 34.97 | C |
| ATOM | 2013 | N | LYS D | 46 | 13.362 | 72.460 | -0.880 | 1.00 39.06 | N |
| ATOM | 2014 | CA | LYS D | 46 | 13.782 | 73.855 | -0.903 | 1.00 35.89 | C |
| ATOM | 2015 | C | LYS D | 46 | 13.682 | 74.312 | -2.350 | 1.00 36.30 | C |
| ATOM | 2016 | O | LYS D | 46 | 13.953 | 73.521 | -3.262 | 1.00 37.16 | O |
| ATOM | 2017 | CB | LYS D | 46 | 15.212 | 73.986 | -0.348 | 1.00 36.94 | C |
| ATOM | 2018 | CG | LYS D | 46 | 15.919 | 75.292 | -0.569 | 1.00 43.26 | C |
| ATOM | 2019 | CD | LYS D | 46 | 17.430 | 75.058 | -0.548 | 1.00 46.21 | C |
| ATOM | 2020 | CE | LYS D | 46 | 18.178 | 76.121 | 0.262 | 1.00 53.10 | C |
| ATOM | 2021 | NZ | LYS D | 46 | 19.663 | 75.960 | 0.110 | 1.00 59.22 | N1+ |
| ATOM | 2022 | N | LEU D | 47 | 13.270 | 75.565 | -2.569 | 1.00 31.33 | N |
| ATOM | 2023 | CA | LEU D | 47 | 13.200 | 76.086 | -3.931 | 1.00 31.83 | C |
| ATOM | 2024 | C | LEU D | 47 | 14.601 | 76.299 | -4.513 | 1.00 35.29 | C |
| ATOM | 2025 | O | LEU D | 47 | 15.454 | 76.963 | -3.911 | 1.00 33.02 | O |
| ATOM | 2026 | CB | LEU D | 47 | 12.417 | 77.388 | -3.962 | 1.00 26.35 | C |
| ATOM | 2027 | CG | LEU D | 47 | 12.230 | 78.066 | -5.326 | 1.00 35.90 | C |
| ATOM | 2028 | CD1 | LEU D | 47 | 11.484 | 77.183 | -6.337 | 1.00 30.55 | C |
| ATOM | 2029 | CD2 | LEU D | 47 | 11.508 | 79.411 | -5.153 | 1.00 30.96 | C |
| ATOM | 2030 | N | LEU D | 48 | 14.837 | 75.725 | -5.687 | 1.00 34.87 | N |
| ATOM | 2031 | CA | LEU D | 48 | 16.123 | 75.799 | -6.374 | 1.00 37.46 | C |
| ATOM | 2032 | C | LEU D | 48 | 16.107 | 76.811 | -7.513 | 1.00 33.53 | C |
| ATOM | 2033 | O | LEU D | 48 | 17.043 | 77.600 | -7.660 | 1.00 38.96 | O |
| ATOM | 2034 | CB | LEU D | 48 | 16.516 | 74.416 | -6.926 | 1.00 27.18 | C |
| ATOM | 2035 | CG | LEU D | 48 | 17.944 | 74.289 | -7.452 | 1.00 28.56 | C |
| ATOM | 2036 | CD1 | LEU D | 48 | 18.892 | 74.353 | -6.289 | 1.00 32.28 | C |
| ATOM | 2037 | CD2 | LEU D | 48 | 18.153 | 72.987 | -8.196 | 1.00 32.35 | C |
| ATOM | 2038 | N | LEU D | 49 | 15.063 | 76.766 | -8.334 | 1.00 29.89 | N |
| ATOM | 2039 | CA | LEU D | 49 | 14.871 | 77.609 | -9.500 | 1.00 31.38 | C |
| ATOM | 2040 | C | LEU D | 49 | 13.383 | 77.885 | -9.629 | 1.00 35.27 | C |
| ATOM | 2041 | O | LEU D | 49 | 12.563 | 76.964 | -9.504 | 1.00 32.72 | O |
| ATOM | 2042 | CB | LEU D | 49 | 15.360 | 76.932 | -10.789 | 1.00 28.28 | C |
| ATOM | 2043 | CG | LEU D | 49 | 16.848 | 76.621 | -10.937 | 1.00 40.25 | C |
| ATOM | 2044 | CD1 | LEU D | 49 | 17.100 | 75.739 | -12.173 | 1.00 34.47 | C |
| ATOM | 2045 | CD2 | LEU D | 49 | 17.642 | 77.943 | -10.996 | 1.00 32.52 | C |
| ATOM | 2046 | N | TYR D | 50 | 13.031 | 79.136 | -9.909 | 1.00 28.76 | N |
| ATOM | 2047 | CA | TYR D | 50 | 11.656 | 79.423 | -10.287 | 1.00 31.24 | C |
| ATOM | 2048 | C | TYR D | 50 | 11.659 | 80.149 | -11.622 | 1.00 31.13 | C |
| ATOM | 2049 | O | TYR D | 50 | 12.697 | 80.634 | -12.077 | 1.00 32.85 | O |
| ATOM | 2050 | CB | TYR D | 50 | 10.923 | 80.211 | -9.196 | 1.00 30.09 | C |
| ATOM | 2051 | CG | TYR D | 50 | 11.519 | 81.554 | -8.869 | 1.00 27.45 | C |
| ATOM | 2052 | CD1 | TYR D | 50 | 12.726 | 81.672 | -8.163 | 1.00 31.73 | C |
| ATOM | 2053 | CD2 | TYR D | 50 | 10.859 | 82.708 | -9.238 | 1.00 29.79 | C |
| ATOM | 2054 | CE1 | TYR D | 50 | 13.253 | 82.920 | -7.843 | 1.00 34.25 | C |
| ATOM | 2055 | CE2 | TYR D | 50 | 11.366 | 83.964 | -8.921 | 1.00 36.09 | C |
| ATOM | 2056 | CZ | TYR D | 50 | 12.546 | 84.072 | -8.229 | 1.00 36.80 | C |

EP 4 435 105 A2

```
ATOM   2057  OH   TYR D  50      12.995  85.340  -7.954  1.00 34.65      O
ATOM   2058  N    ASP D  51      10.487  80.198 -12.260  1.00 33.46      N
ATOM   2059  CA   ASP D  51      10.371  80.678 -13.638  1.00 34.13      C
ATOM   2060  C    ASP D  51      11.474  80.078 -14.517  1.00 35.70      C
ATOM   2061  O    ASP D  51      12.269  80.788 -15.139  1.00 32.50      O
ATOM   2062  CB   ASP D  51      10.410  82.199 -13.683  1.00 33.19      C
ATOM   2063  CG   ASP D  51      10.091  82.738 -15.053  1.00 36.53      C
ATOM   2064  OD1  ASP D  51       9.413  82.007 -15.813  1.00 37.05      O
ATOM   2065  OD2  ASP D  51      10.507  83.882 -15.361  1.00 36.34      O1-
ATOM   2066  N    SER D  52      11.565  78.745 -14.494  1.00 35.41      N
ATOM   2067  CA   SER D  52      12.525  77.980 -15.295  1.00 35.10      C
ATOM   2068  C    SER D  52      13.986  78.153 -14.884  1.00 34.26      C
ATOM   2069  O    SER D  52      14.730  77.167 -14.836  1.00 34.09      O
ATOM   2070  CB   SER D  52      12.393  78.333 -16.779  1.00 29.40      C
ATOM   2071  OG   SER D  52      11.152  77.892 -17.275  1.00 42.15      O
ATOM   2072  N    ASN D  53      14.437  79.386 -14.628  1.00 32.85      N
ATOM   2073  CA   ASN D  53      15.878  79.558 -14.463  1.00 32.56      C
ATOM   2074  C    ASN D  53      16.290  80.660 -13.495  1.00 34.70      C
ATOM   2075  O    ASN D  53      17.467  81.027 -13.485  1.00 34.38      O
ATOM   2076  CB   ASN D  53      16.536  79.814 -15.804  1.00 29.46      C
ATOM   2077  CG   ASN D  53      16.066  81.113 -16.443  1.00 40.20      C
ATOM   2078  OD1  ASN D  53      15.192  81.817 -15.918  1.00 32.59      O
ATOM   2079  ND2  ASN D  53      16.652  81.440 -17.583  1.00 37.64      N
ATOM   2080  N    LYS D  54      15.391  81.191 -12.677  1.00 36.81      N
ATOM   2081  CA   LYS D  54      15.761  82.209 -11.713  1.00 29.79      C
ATOM   2082  C    LYS D  54      16.163  81.540 -10.408  1.00 35.07      C
ATOM   2083  O    LYS D  54      15.433  80.686  -9.887  1.00 34.40      O
ATOM   2084  CB   LYS D  54      14.601  83.166 -11.469  1.00 34.19      C
ATOM   2085  CG   LYS D  54      14.979  84.334 -10.607  1.00 39.09      C
ATOM   2086  CD   LYS D  54      15.984  85.215 -11.319  1.00 36.81      C
ATOM   2087  CE   LYS D  54      16.480  86.282 -10.370  1.00 39.74      C
ATOM   2088  NZ   LYS D  54      17.279  87.308 -11.098  1.00 49.26      N1+
ATOM   2089  N    ARG D  55      17.318  81.924  -9.890  1.00 38.57      N
ATOM   2090  C    ARG D  55      17.333  82.285  -7.476  1.00 39.73      C
ATOM   2091  O    ARG D  55      17.511  83.503  -7.533  1.00 41.35      O
ATOM   2092  CA  AARG D  55      17.814  81.402  -8.621  0.50 38.68      C
ATOM   2093  CB  AARG D  55      19.341  81.344  -8.605  0.50 37.77      C
ATOM   2094  CG  AARG D  55      19.958  80.202  -9.404  0.50 40.11      C
ATOM   2095  CD  AARG D  55      21.494  80.251  -9.387  0.50 40.35      C
ATOM   2096  NE  AARG D  55      22.017  81.378 -10.159  0.50 39.90      N
ATOM   2097  CZ  AARG D  55      22.738  82.377  -9.659  0.50 39.21      C
ATOM   2098  NH1 AARG D  55      23.141  83.349 -10.459  0.50 39.24      N1+
ATOM   2099  NH2 AARG D  55      23.060  82.406  -8.369  0.50 34.82      N
ATOM   2100  CA  BARG D  55      17.803  81.399  -8.621  0.50 38.68      C
ATOM   2101  CB  BARG D  55      19.324  81.321  -8.607  0.50 37.74      C
ATOM   2102  CG  BARG D  55      19.902  80.435  -9.684  0.50 40.11      C
ATOM   2103  CD  BARG D  55      21.390  80.690  -9.875  0.50 41.27      C
ATOM   2104  NE  BARG D  55      21.881  80.029 -11.080  0.50 44.29      N
ATOM   2105  CZ  BARG D  55      21.979  80.606 -12.275  0.50 41.10      C
ATOM   2106  NH1 BARG D  55      21.632  81.878 -12.448  0.50 43.98      N1+
ATOM   2107  NH2 BARG D  55      22.436  79.903 -13.299  0.50 37.99      N
ATOM   2108  N    PRO D  56      16.709  81.722  -6.444  1.00 42.43      N
ATOM   2109  CA   PRO D  56      16.550  82.476  -5.202  1.00 39.14      C
ATOM   2110  C    PRO D  56      17.937  82.781  -4.673  1.00 39.14      C
ATOM   2111  O    PRO D  56      18.921  82.132  -5.037  1.00 39.24      O
ATOM   2112  CB   PRO D  56      15.800  81.513  -4.264  1.00 40.40      C
ATOM   2113  CG   PRO D  56      15.350  80.384  -5.079  1.00 36.67      C
ATOM   2114  CD   PRO D  56      16.195  80.347  -6.344  1.00 38.20      C
ATOM   2115  N    SER D  57      18.025  83.787  -3.819  1.00 38.52      N
ATOM   2116  CA   SER D  57      19.326  84.087  -3.245  1.00 45.88      C
ATOM   2117  C    SER D  57      19.760  82.911  -2.381  1.00 44.76      C
ATOM   2118  O    SER D  57      18.928  82.206  -1.807  1.00 46.46      O
ATOM   2119  CB   SER D  57      19.282  85.384  -2.441  1.00 45.60      C
ATOM   2120  OG   SER D  57      18.126  85.430  -1.652  1.00 61.35      O
ATOM   2121  N    GLY D  58      21.056  82.630  -2.375  1.00 45.80      N
ATOM   2122  CA   GLY D  58      21.558  81.468  -1.677  1.00 38.95      C
ATOM   2123  C    GLY D  58      21.780  80.253  -2.549  1.00 45.79      C
ATOM   2124  O    GLY D  58      22.406  79.290  -2.093  1.00 50.05      O
ATOM   2125  N    ILE D  59      21.280  80.246  -3.777  1.00 44.59      N
ATOM   2126  CA   ILE D  59      21.479  79.123  -4.684  1.00 37.66      C
ATOM   2127  C    ILE D  59      22.682  79.446  -5.571  1.00 42.15      C
```

298

```
ATOM   2128  O    ILE D  59    22.645  80.454  -6.294  1.00 41.34          O
ATOM   2129  CB   ILE D  59    20.232  78.831  -5.528  1.00 40.49          C
ATOM   2130  CG1  ILE D  59    19.045  78.500  -4.616  1.00 37.44          C
ATOM   2131  CG2  ILE D  59    20.527  77.690  -6.542  1.00 36.08          C
ATOM   2132  CD1  ILE D  59    19.273  77.277  -3.723  1.00 34.90          C
ATOM   2133  N    PRO D  60    23.724  78.612  -5.571  1.00 38.05          N
ATOM   2134  CA   PRO D  60    24.938  78.914  -6.342  1.00 42.90          C
ATOM   2135  C    PRO D  60    24.666  78.936  -7.835  1.00 40.36          C
ATOM   2136  O    PRO D  60    23.778  78.245  -8.333  1.00 40.44          O
ATOM   2137  CB   PRO D  60    25.888  77.754  -5.990  1.00 43.52          C
ATOM   2138  CG   PRO D  60    25.222  76.963  -4.934  1.00 44.09          C
ATOM   2139  CD   PRO D  60    23.756  77.268  -4.984  1.00 41.65          C
ATOM   2140  N    ALA D  61    25.495  79.695  -8.557  1.00 38.15          N
ATOM   2141  CA   ALA D  61    25.351  79.835 -10.003  1.00 46.92          C
ATOM   2142  C    ALA D  61    25.639  78.546 -10.780  1.00 41.95          C
ATOM   2143  O    ALA D  61    25.376  78.501 -11.990  1.00 41.08          O
ATOM   2144  CB   ALA D  61    26.268  80.955 -10.507  1.00 37.90          C
ATOM   2145  N    ARG D  62    26.167  77.501 -10.144  1.00 40.02          N
ATOM   2146  CA   ARG D  62    26.364  76.277 -10.909  1.00 39.12          C
ATOM   2147  C    ARG D  62    25.061  75.511 -11.136  1.00 41.84          C
ATOM   2148  O    ARG D  62    25.060  74.529 -11.894  1.00 38.44          O
ATOM   2149  CB   ARG D  62    27.411  75.384 -10.240  1.00 40.13          C
ATOM   2150  CG   ARG D  62    27.061  74.940  -8.830  1.00 51.16          C
ATOM   2151  CD   ARG D  62    28.110  73.962  -8.276  1.00 54.07          C
ATOM   2152  NE   ARG D  62    27.617  73.327  -7.065  1.00 42.44          N
ATOM   2153  CZ   ARG D  62    27.632  73.937  -5.888  1.00 48.82          C
ATOM   2154  NH1  ARG D  62    28.139  75.165  -5.790  1.00 55.75          N1+
ATOM   2155  NH2  ARG D  62    27.145  73.336  -4.819  1.00 44.87          N
ATOM   2156  N    PHE D  63    23.959  75.946 -10.518  1.00 35.81          N
ATOM   2157  CA   PHE D  63    22.630  75.461 -10.857  1.00 36.89          C
ATOM   2158  C    PHE D  63    22.032  76.381 -11.914  1.00 41.20          C
ATOM   2159  O    PHE D  63    22.021  77.610 -11.749  1.00 39.30          O
ATOM   2160  CB   PHE D  63    21.724  75.420  -9.622  1.00 36.15          C
ATOM   2161  CG   PHE D  63    22.177  74.455  -8.550  1.00 35.88          C
ATOM   2162  CD1  PHE D  63    23.020  74.870  -7.539  1.00 36.00          C
ATOM   2163  CD2  PHE D  63    21.747  73.132  -8.556  1.00 38.64          C
ATOM   2164  CE1  PHE D  63    23.443  73.983  -6.550  1.00 43.00          C
ATOM   2165  CE2  PHE D  63    22.165  72.239  -7.572  1.00 37.52          C
ATOM   2166  CZ   PHE D  63    23.013  72.664  -6.565  1.00 36.44          C
ATOM   2167  N    SER D  64    21.548  75.795 -13.005  1.00 35.81          N
ATOM   2168  CA   SER D  64    20.854  76.595 -14.008  1.00 37.12          C
ATOM   2169  C    SER D  64    19.753  75.760 -14.648  1.00 38.07          C
ATOM   2170  O    SER D  64    19.691  74.529 -14.505  1.00 38.30          O
ATOM   2171  CB   SER D  64    21.812  77.151 -15.076  1.00 31.57          C
ATOM   2172  OG   SER D  64    22.505  76.104 -15.730  1.00 44.67          O
ATOM   2173  N    GLY D  65    18.868  76.454 -15.349  1.00 34.85          N
ATOM   2174  CA   GLY D  65    17.712  75.817 -15.927  1.00 38.26          C
ATOM   2175  C    GLY D  65    17.422  76.371 -17.302  1.00 40.50          C
ATOM   2176  O    GLY D  65    17.829  77.479 -17.661  1.00 39.25          O
ATOM   2177  N    SER D  66    16.706  75.570 -18.071  1.00 33.85          N
ATOM   2178  CA   SER D  66    16.204  76.050 -19.344  1.00 35.96          C
ATOM   2179  C    SER D  66    14.865  75.386 -19.584  1.00 39.56          C
ATOM   2180  O    SER D  66    14.493  74.412 -18.914  1.00 36.55          O
ATOM   2181  CB   SER D  66    17.164  75.765 -20.490  1.00 36.16          C
ATOM   2182  OG   SER D  66    17.463  74.379 -20.517  1.00 43.70          O
ATOM   2183  N    LYS D  67    14.132  75.959 -20.522  1.00 31.53          N
ATOM   2184  CA   LYS D  67    12.820  75.482 -20.896  1.00 33.07          C
ATOM   2185  C    LYS D  67    12.746  75.549 -22.407  1.00 38.33          C
ATOM   2186  O    LYS D  67    13.238  76.494 -23.014  1.00 43.23          O
ATOM   2187  CB   LYS D  67    11.690  76.313 -20.249  1.00 32.47          C
ATOM   2188  CG   LYS D  67    10.269  75.914 -20.705  1.00 28.80          C
ATOM   2189  CD   LYS D  67     9.194  76.736 -20.001  1.00 35.59          C
ATOM   2190  CE   LYS D  67     7.890  76.733 -20.788  1.00 45.34          C
ATOM   2191  NZ   LYS D  67     6.693  77.312 -20.018  1.00 45.74          N1+
ATOM   2192  N    SER D  68    12.175  74.523 -23.008  1.00 40.72          N
ATOM   2193  CA   SER D  68    11.983  74.502 -24.446  1.00 40.77          C
ATOM   2194  C    SER D  68    10.705  73.722 -24.702  1.00 40.33          C
ATOM   2195  O    SER D  68    10.665  72.518 -24.439  1.00 41.36          O
ATOM   2196  CB   SER D  68    13.179  73.866 -25.139  1.00 44.26          C
ATOM   2197  OG   SER D  68    12.935  73.761 -26.526  1.00 55.67          O
ATOM   2198  N    GLY D  69     9.655  74.408 -25.145  1.00 37.35          N
```

| ATOM | 2199 | CA | GLY | D | 69 | 8.413 | 73.715 | -25.466 | 1.00 | 34.14 | C |
|------|------|-----|-----|---|----|--------|--------|---------|------|-------|---|
| ATOM | 2200 | C | GLY | D | 69 | 7.800 | 73.055 | -24.242 | 1.00 | 37.93 | C |
| ATOM | 2201 | O | GLY | D | 69 | 7.543 | 73.700 | -23.220 | 1.00 | 34.02 | O |
| ATOM | 2202 | N | THR | D | 70 | 7.592 | 71.738 | -24.310 | 1.00 | 38.48 | N |
| ATOM | 2203 | CA | THR | D | 70 | 6.956 | 71.011 | -23.219 | 1.00 | 38.13 | C |
| ATOM | 2204 | C | THR | D | 70 | 7.967 | 70.318 | -22.304 | 1.00 | 39.79 | C |
| ATOM | 2205 | O | THR | D | 70 | 7.590 | 69.416 | -21.549 | 1.00 | 40.93 | O |
| ATOM | 2206 | CB | THR | D | 70 | 5.968 | 69.982 | -23.771 | 1.00 | 37.94 | C |
| ATOM | 2207 | OG1 | THR | D | 70 | 6.672 | 69.053 | -24.608 | 1.00 | 38.51 | O |
| ATOM | 2208 | CG2 | THR | D | 70 | 4.899 | 70.665 | -24.571 | 1.00 | 27.17 | C |
| ATOM | 2209 | N | SER | D | 71 | 9.240 | 70.702 | -22.357 | 1.00 | 32.96 | N |
| ATOM | 2210 | CA | SER | D | 71 | 10.202 | 70.129 | -21.431 | 1.00 | 40.52 | C |
| ATOM | 2211 | C | SER | D | 71 | 11.091 | 71.219 | -20.853 | 1.00 | 36.32 | C |
| ATOM | 2212 | O | SER | D | 71 | 11.231 | 72.312 | -21.411 | 1.00 | 44.52 | O |
| ATOM | 2213 | CB | SER | D | 71 | 11.049 | 69.031 | -22.089 | 1.00 | 48.49 | C |
| ATOM | 2214 | OG | SER | D | 71 | 11.991 | 69.599 | -22.976 | 1.00 | 58.32 | O |
| ATOM | 2215 | N | ALA | D | 72 | 11.645 | 70.915 | -19.686 | 1.00 | 31.74 | N |
| ATOM | 2216 | CA | ALA | D | 72 | 12.519 | 71.800 | -18.942 | 1.00 | 32.67 | C |
| ATOM | 2217 | C | ALA | D | 72 | 13.688 | 70.976 | -18.443 | 1.00 | 37.05 | C |
| ATOM | 2218 | O | ALA | D | 72 | 13.541 | 69.784 | -18.161 | 1.00 | 35.94 | O |
| ATOM | 2219 | CB | ALA | D | 72 | 11.796 | 72.461 | -17.758 | 1.00 | 29.67 | C |
| ATOM | 2220 | N | THR | D | 73 | 14.861 | 71.594 | -18.349 | 1.00 | 33.25 | N |
| ATOM | 2221 | CA | THR | D | 73 | 15.969 | 70.834 | -17.809 | 1.00 | 33.69 | C |
| ATOM | 2222 | C | THR | D | 73 | 16.765 | 71.667 | -16.813 | 1.00 | 36.67 | C |
| ATOM | 2223 | O | THR | D | 73 | 16.912 | 72.886 | -16.951 | 1.00 | 37.39 | O |
| ATOM | 2224 | CB | THR | D | 73 | 16.875 | 70.239 | -18.916 | 1.00 | 40.87 | C |
| ATOM | 2225 | OG1 | THR | D | 73 | 18.214 | 70.724 | -18.799 | 1.00 | 48.32 | O |
| ATOM | 2226 | CG2 | THR | D | 73 | 16.335 | 70.483 | -20.289 | 1.00 | 31.17 | C |
| ATOM | 2227 | N | LEU | D | 74 | 17.224 | 70.979 | -15.775 | 1.00 | 36.18 | N |
| ATOM | 2228 | CA | LEU | D | 74 | 18.084 | 71.536 | -14.749 | 1.00 | 40.71 | C |
| ATOM | 2229 | C | LEU | D | 74 | 19.514 | 71.068 | -15.005 | 1.00 | 39.16 | C |
| ATOM | 2230 | O | LEU | D | 74 | 19.747 | 69.887 | -15.269 | 1.00 | 36.86 | O |
| ATOM | 2231 | CB | LEU | D | 74 | 17.597 | 71.088 | -13.375 | 1.00 | 32.52 | C |
| ATOM | 2232 | CG | LEU | D | 74 | 18.632 | 71.088 | -12.262 | 1.00 | 33.76 | C |
| ATOM | 2233 | CD1 | LEU | D | 74 | 18.979 | 72.495 | -11.837 | 1.00 | 34.80 | C |
| ATOM | 2234 | CD2 | LEU | D | 74 | 18.078 | 70.285 | -11.105 | 1.00 | 28.46 | C |
| ATOM | 2235 | N | GLY | D | 75 | 20.456 | 71.992 | -14.964 | 1.00 | 39.23 | N |
| ATOM | 2236 | CA | GLY | D | 75 | 21.861 | 71.676 | -15.155 | 1.00 | 34.28 | C |
| ATOM | 2237 | C | GLY | D | 75 | 22.643 | 71.975 | -13.891 | 1.00 | 35.73 | C |
| ATOM | 2238 | O | GLY | D | 75 | 22.380 | 72.971 | -13.212 | 1.00 | 31.27 | O |
| ATOM | 2239 | N | ILE | D | 76 | 23.572 | 71.083 | -13.555 | 1.00 | 37.91 | N |
| ATOM | 2240 | CA | ILE | D | 76 | 24.460 | 71.270 | -12.416 | 1.00 | 37.96 | C |
| ATOM | 2241 | C | ILE | D | 76 | 25.880 | 71.043 | -12.900 | 1.00 | 44.61 | C |
| ATOM | 2242 | O | ILE | D | 76 | 26.245 | 69.912 | -13.243 | 1.00 | 45.05 | O |
| ATOM | 2243 | CB | ILE | D | 76 | 24.132 | 70.324 | -11.259 | 1.00 | 37.47 | C |
| ATOM | 2244 | CG1 | ILE | D | 76 | 22.612 | 70.258 | -11.056 | 1.00 | 38.58 | C |
| ATOM | 2245 | CG2 | ILE | D | 76 | 24.842 | 70.801 | -10.019 | 1.00 | 34.75 | C |
| ATOM | 2246 | CD1 | ILE | D | 76 | 22.182 | 69.396 | -9.926 | 1.00 | 35.25 | C |
| ATOM | 2247 | N | THR | D | 77 | 26.684 | 72.103 | -12.916 | 1.00 | 41.92 | N |
| ATOM | 2248 | CA | THR | D | 77 | 28.105 | 72.001 | -13.217 | 1.00 | 45.22 | C |
| ATOM | 2249 | C | THR | D | 77 | 28.911 | 71.995 | -11.922 | 1.00 | 49.05 | C |
| ATOM | 2250 | O | THR | D | 77 | 28.433 | 72.413 | -10.863 | 1.00 | 46.09 | O |
| ATOM | 2251 | CB | THR | D | 77 | 28.570 | 73.156 | -14.111 | 1.00 | 45.74 | C |
| ATOM | 2252 | OG1 | THR | D | 77 | 28.494 | 74.389 | -13.383 | 1.00 | 45.10 | O |
| ATOM | 2253 | CG2 | THR | D | 77 | 27.707 | 73.246 | -15.356 | 1.00 | 41.50 | C |
| ATOM | 2254 | N | GLY | D | 78 | 30.136 | 71.474 | -12.015 | 1.00 | 50.10 | N |
| ATOM | 2255 | CA | GLY | D | 78 | 31.054 | 71.450 | -10.893 | 1.00 | 40.32 | C |
| ATOM | 2256 | C | GLY | D | 78 | 30.509 | 70.763 | -9.660 | 1.00 | 47.60 | C |
| ATOM | 2257 | O | GLY | D | 78 | 30.599 | 71.321 | -8.562 | 1.00 | 46.13 | O |
| ATOM | 2258 | N | LEU | D | 79 | 29.977 | 69.545 | -9.822 | 1.00 | 44.28 | N |
| ATOM | 2259 | CA | LEU | D | 79 | 29.291 | 68.851 | -8.735 | 1.00 | 41.62 | C |
| ATOM | 2260 | C | LEU | D | 79 | 30.097 | 68.875 | -7.445 | 1.00 | 41.28 | C |
| ATOM | 2261 | O | LEU | D | 79 | 31.306 | 68.625 | -7.445 | 1.00 | 43.99 | O |
| ATOM | 2262 | CB | LEU | D | 79 | 29.044 | 67.391 | -9.108 | 1.00 | 44.54 | C |
| ATOM | 2263 | CG | LEU | D | 79 | 27.718 | 66.829 | -9.591 | 1.00 | 41.80 | C |
| ATOM | 2264 | CD1 | LEU | D | 79 | 26.548 | 67.743 | -9.317 | 1.00 | 41.01 | C |
| ATOM | 2265 | CD2 | LEU | D | 79 | 27.807 | 66.456 | -11.048 | 1.00 | 48.29 | C |
| ATOM | 2266 | N | GLN | D | 80 | 29.407 | 69.147 | -6.341 | 1.00 | 38.12 | N |
| ATOM | 2267 | CA | GLN | D | 80 | 29.955 | 69.025 | -5.000 | 1.00 | 37.63 | C |
| ATOM | 2268 | C | GLN | D | 80 | 29.166 | 67.992 | -4.207 | 1.00 | 39.83 | C |
| ATOM | 2269 | O | GLN | D | 80 | 27.975 | 67.771 | -4.461 | 1.00 | 44.36 | O |

```
ATOM    2270  CB   GLN D  80      29.925  70.349  -4.277  1.00 35.93           C
ATOM    2271  CG   GLN D  80      30.598  71.424  -5.072  1.00 44.41           C
ATOM    2272  CD   GLN D  80      30.602  72.746  -4.351  1.00 48.07           C
ATOM    2273  OE1  GLN D  80      30.141  72.848  -3.211  1.00 51.38           O
ATOM    2274  NE2  GLN D  80      31.108  73.778  -5.018  1.00 49.68           N
ATOM    2275  N    THR D  81      29.838  67.362  -3.236  1.00 39.23           N
ATOM    2276  CA   THR D  81      29.185  66.313  -2.459  1.00 43.43           C
ATOM    2277  C    THR D  81      27.905  66.827  -1.825  1.00 38.49           C
ATOM    2278  O    THR D  81      26.949  66.066  -1.660  1.00 43.22           O
ATOM    2279  CB   THR D  81      30.122  65.740  -1.386  1.00 33.08           C
ATOM    2280  OG1  THR D  81      30.589  66.796  -0.558  1.00 47.69           O
ATOM    2281  CG2  THR D  81      31.307  65.035  -2.020  1.00 35.64           C
ATOM    2282  N    GLY D  82      27.859  68.112  -1.498  1.00 35.25           N
ATOM    2283  CA   GLY D  82      26.664  68.722  -0.952  1.00 40.04           C
ATOM    2284  C    GLY D  82      25.510  68.895  -1.929  1.00 38.60           C
ATOM    2285  O    GLY D  82      24.437  69.339  -1.510  1.00 35.12           O
ATOM    2286  N    ASP D  83      25.694  68.543  -3.200  1.00 33.43           N
ATOM    2287  CA   ASP D  83      24.619  68.564  -4.179  1.00 37.84           C
ATOM    2288  C    ASP D  83      23.834  67.251  -4.245  1.00 43.45           C
ATOM    2289  O    ASP D  83      22.814  67.191  -4.957  1.00 40.21           O
ATOM    2290  CB   ASP D  83      25.181  68.882  -5.566  1.00 33.25           C
ATOM    2291  CG   ASP D  83      25.971  70.173  -5.593  1.00 42.35           C
ATOM    2292  OD1  ASP D  83      25.680  71.085  -4.788  1.00 36.88           O
ATOM    2293  OD2  ASP D  83      26.882  70.284  -6.440  1.00 44.43           O1-
ATOM    2294  N    GLU D  84      24.310  66.192  -3.577  1.00 37.08           N
ATOM    2295  CA   GLU D  84      23.578  64.931  -3.529  1.00 38.14           C
ATOM    2296  C    GLU D  84      22.192  65.159  -2.929  1.00 37.73           C
ATOM    2297  O    GLU D  84      22.075  65.642  -1.800  1.00 36.95           O
ATOM    2298  CB   GLU D  84      24.359  63.904  -2.708  1.00 33.68           C
ATOM    2299  CG   GLU D  84      23.683  62.544  -2.653  1.00 41.33           C
ATOM    2300  CD   GLU D  84      24.682  61.389  -2.458  1.00 46.09           C
ATOM    2301  OE1  GLU D  84      24.374  60.429  -1.716  1.00 49.48           O
ATOM    2302  OE2  GLU D  84      25.766  61.427  -3.078  1.00 45.22           O1-
ATOM    2303  N    ALA D  85      21.146  64.836  -3.688  1.00 31.07           N
ATOM    2304  CA   ALA D  85      19.786  65.206  -3.298  1.00 29.56           C
ATOM    2305  C    ALA D  85      18.818  64.596  -4.293  1.00 32.77           C
ATOM    2306  O    ALA D  85      19.218  64.022  -5.314  1.00 32.03           O
ATOM    2307  CB   ALA D  85      19.590  66.729  -3.227  1.00 30.06           C
ATOM    2308  N    ASP D  86      17.531  64.697  -3.959  1.00 30.95           N
ATOM    2309  CA   ASP D  86      16.457  64.474  -4.921  1.00 30.44           C
ATOM    2310  C    ASP D  86      16.015  65.817  -5.481  1.00 33.26           C
ATOM    2311  O    ASP D  86      15.958  66.816  -4.752  1.00 29.91           O
ATOM    2312  CB   ASP D  86      15.261  63.780  -4.283  1.00 36.23           C
ATOM    2313  CG   ASP D  86      15.600  62.414  -3.757  1.00 39.79           C
ATOM    2314  OD1  ASP D  86      16.287  61.640  -4.460  1.00 49.35           O
ATOM    2315  OD2  ASP D  86      15.202  62.134  -2.620  1.00 52.11           O1-
ATOM    2316  N    TYR D  87      15.723  65.834  -6.775  1.00 28.24           N
ATOM    2317  CA   TYR D  87      15.349  67.045  -7.481  1.00 30.37           C
ATOM    2318  C    TYR D  87      13.993  66.832  -8.116  1.00 30.53           C
ATOM    2319  O    TYR D  87      13.736  65.781  -8.716  1.00 31.12           O
ATOM    2320  CB   TYR D  87      16.407  67.425  -8.550  1.00 31.35           C
ATOM    2321  CG   TYR D  87      17.707  67.860  -7.930  1.00 28.53           C
ATOM    2322  CD2  TYR D  87      18.711  66.933  -7.628  1.00 29.65           C
ATOM    2323  CD1  TYR D  87      17.919  69.192  -7.596  1.00 30.97           C
ATOM    2324  CE2  TYR D  87      19.913  67.350  -7.040  1.00 36.12           C
ATOM    2325  CE1  TYR D  87      19.110  69.620  -6.999  1.00 32.34           C
ATOM    2326  CZ   TYR D  87      20.101  68.700  -6.728  1.00 36.00           C
ATOM    2327  OH   TYR D  87      21.261  69.124  -6.123  1.00 36.35           O
ATOM    2328  N    TYR D  88      13.130  67.833  -7.986  1.00 31.77           N
ATOM    2329  CA   TYR D  88      11.763  67.756  -8.495  1.00 30.69           C
ATOM    2330  C    TYR D  88      11.442  68.977  -9.350  1.00 33.16           C
ATOM    2331  O    TYR D  88      11.697  70.123  -8.943  1.00 27.49           O
ATOM    2332  CB   TYR D  88      10.755  67.662  -7.330  1.00 28.47           C
ATOM    2333  CG   TYR D  88      10.863  66.418  -6.469  1.00 32.12           C
ATOM    2334  CD1  TYR D  88      10.157  65.254  -6.797  1.00 33.44           C
ATOM    2335  CD2  TYR D  88      11.639  66.407  -5.312  1.00 35.30           C
ATOM    2336  CE1  TYR D  88      10.246  64.107  -6.011  1.00 33.26           C
ATOM    2337  CE2  TYR D  88      11.735  65.256  -4.505  1.00 28.88           C
ATOM    2338  CZ   TYR D  88      11.038  64.121  -4.860  1.00 35.12           C
ATOM    2339  OH   TYR D  88      11.138  63.001  -4.067  1.00 41.20           O
ATOM    2340  N    CYS D  89      10.860  68.743 -10.520  1.00 29.10           N
```

EP 4 435 105 A2

```
ATOM   2341  CA   CYS D  89     10.217  69.855 -11.206  1.00 33.19          C
ATOM   2342  C    CYS D  89      8.723  69.907 -10.850  1.00 37.48          C
ATOM   2343  O    CYS D  89      8.110  68.906 -10.450  1.00 33.89          O
ATOM   2344  CB   CYS D  89     10.397  69.751 -12.722  1.00 35.57          C
ATOM   2345  SG   CYS D  89      9.788  68.160 -13.353  1.00 50.43          S
ATOM   2346  N    GLY D  90      8.153  71.103 -10.985  1.00 35.51          N
ATOM   2347  CA   GLY D  90      6.747  71.329 -10.696  1.00 31.51          C
ATOM   2348  C    GLY D  90      6.184  72.474 -11.510  1.00 32.91          C
ATOM   2349  O    GLY D  90      6.886  73.440 -11.821  1.00 37.81          O
ATOM   2350  N    THR D  91      4.905  72.347 -11.876  1.00 29.44          N
ATOM   2351  CA   THR D  91      4.112  73.391 -12.523  1.00 35.31          C
ATOM   2352  C    THR D  91      2.648  73.192 -12.187  1.00 34.83          C
ATOM   2353  O    THR D  91      2.252  72.229 -11.533  1.00 31.62          O
ATOM   2354  CB   THR D  91      4.039  73.364 -14.063  1.00 36.67          C
ATOM   2355  OG1  THR D  91      4.722  72.256 -14.635  1.00 42.40          O
ATOM   2356  CG2  THR D  91      4.377  74.637 -14.683  1.00 25.98          C
ATOM   2357  N    TRP D  92      1.845  74.047 -12.800  1.00 33.75          N
ATOM   2358  CA   TRP D  92      0.422  73.882 -12.929  1.00 32.47          C
ATOM   2359  C    TRP D  92      0.088  73.103 -14.202  1.00 34.81          C
ATOM   2360  O    TRP D  92      0.757  73.209 -15.235  1.00 30.64          O
ATOM   2361  CB   TRP D  92     -0.234  75.251 -12.956  1.00 33.91          C
ATOM   2362  CG   TRP D  92     -1.719  75.251 -12.940  1.00 35.91          C
ATOM   2363  CD1  TRP D  92     -2.553  75.591 -13.969  1.00 31.74          C
ATOM   2364  CD2  TRP D  92     -2.557  74.949 -11.818  1.00 34.97          C
ATOM   2365  NE1  TRP D  92     -3.860  75.519 -13.554  1.00 37.78          N
ATOM   2366  CE2  TRP D  92     -3.893  75.127 -12.238  1.00 39.89          C
ATOM   2367  CE3  TRP D  92     -2.308  74.567 -10.494  1.00 27.09          C
ATOM   2368  CZ2  TRP D  92     -4.979  74.921 -11.380  1.00 36.03          C
ATOM   2369  CZ3  TRP D  92     -3.384  74.357  -9.643  1.00 35.46          C
ATOM   2370  CH2  TRP D  92     -4.706  74.542 -10.087  1.00 35.32          C
ATOM   2371  N    ASP D  93     -0.943  72.296 -14.109  1.00 32.62          N
ATOM   2372  CA   ASP D  93     -1.540  71.671 -15.277  1.00 38.27          C
ATOM   2373  C    ASP D  93     -2.925  72.275 -15.476  1.00 37.51          C
ATOM   2374  O    ASP D  93     -3.809  72.089 -14.634  1.00 39.63          O
ATOM   2375  CB   ASP D  93     -1.609  70.162 -15.121  1.00 37.98          C
ATOM   2376  CG   ASP D  93     -2.016  69.490 -16.396  1.00 37.56          C
ATOM   2377  OD1  ASP D  93     -3.109  69.827 -16.919  1.00 38.96          O1-
ATOM   2378  OD2  ASP D  93     -1.226  68.659 -16.884  1.00 37.13          O
ATOM   2379  N    SER D  94     -3.094  73.010 -16.583  1.00 40.55          N
ATOM   2380  CA   SER D  94     -4.324  73.756 -16.838  1.00 43.35          C
ATOM   2381  C    SER D  94     -5.502  72.843 -17.132  1.00 41.15          C
ATOM   2382  O    SER D  94     -6.643  73.204 -16.848  1.00 47.84          O
ATOM   2383  CB   SER D  94     -4.124  74.716 -18.009  1.00 37.93          C
ATOM   2384  OG   SER D  94     -3.093  75.653 -17.720  1.00 50.29          O
ATOM   2385  N    SER D  95     -5.261  71.659 -17.656  1.00 39.23          N
ATOM   2386  CA   SER D  95     -6.392  70.814 -17.991  1.00 45.37          C
ATOM   2387  C    SER D  95     -6.795  69.916 -16.843  1.00 44.47          C
ATOM   2388  O    SER D  95     -7.981  69.617 -16.704  1.00 52.00          O
ATOM   2389  CB   SER D  95     -6.084  69.960 -19.223  1.00 44.25          C
ATOM   2390  OG   SER D  95     -5.390  68.785 -18.856  1.00 58.75          O
ATOM   2391  N    LEU D  96     -5.845  69.487 -16.012  1.00 43.88          N
ATOM   2392  CA   LEU D  96     -6.173  68.775 -14.780  1.00 37.73          C
ATOM   2393  C    LEU D  96     -6.491  69.723 -13.621  1.00 36.61          C
ATOM   2394  O    LEU D  96     -6.885  69.255 -12.550  1.00 38.29          O
ATOM   2395  CB   LEU D  96     -5.019  67.839 -14.391  1.00 41.42          C
ATOM   2396  CG   LEU D  96     -4.577  66.750 -15.380  1.00 39.10          C
ATOM   2397  CD1  LEU D  96     -3.261  66.160 -14.963  1.00 34.94          C
ATOM   2398  CD2  LEU D  96     -5.612  65.640 -15.506  1.00 35.48          C
ATOM   2399  N    ASN D  97     -6.353  71.034 -13.811  1.00 37.50          N
ATOM   2400  CA   ASN D  97     -6.524  72.032 -12.750  1.00 39.48          C
ATOM   2401  C    ASN D  97     -5.885  71.606 -11.418  1.00 35.80          C
ATOM   2402  O    ASN D  97     -6.519  71.581 -10.367  1.00 35.42          O
ATOM   2403  CB   ASN D  97     -8.002  72.403 -12.591  1.00 33.46          C
ATOM   2404  CG   ASN D  97     -8.304  73.766 -13.211  1.00 59.81          C
ATOM   2405  OD1  ASN D  97     -8.600  74.731 -12.490  1.00 63.64          O
ATOM   2406  ND2  ASN D  97     -8.104  73.888 -14.535  1.00 48.78          N
ATOM   2407  N    THR D  98     -4.584  71.319 -11.468  1.00 34.96          N
ATOM   2408  CA   THR D  98     -3.880  70.883 -10.267  1.00 40.51          C
ATOM   2409  C    THR D  98     -2.389  71.197 -10.382  1.00 33.13          C
ATOM   2410  O    THR D  98     -1.843  71.349 -11.482  1.00 33.25          O
ATOM   2411  CB   THR D  98     -4.095  69.378 -10.000  1.00 31.21          C
```

| ATOM | 2412 | OG1 | THR | D | 98  | -3.498 | 69.048 | -8.749  | 1.00 | 36.06 | O   |
| ATOM | 2413 | CG2 | THR | D | 98  | -3.428 | 68.531 | -11.061 | 1.00 | 31.76 | C   |
| ATOM | 2414 | N   | VAL | D | 99  | -1.730 | 71.290 | -9.222  | 1.00 | 29.98 | N   |
| ATOM | 2415 | CA  | VAL | D | 99  | -0.267 | 71.342 | -9.211  | 1.00 | 34.24 | C   |
| ATOM | 2416 | C   | VAL | D | 99  | 0.296  | 69.972 | -9.558  | 1.00 | 34.16 | C   |
| ATOM | 2417 | O   | VAL | D | 99  | -0.215 | 68.936 | -9.120  | 1.00 | 34.33 | O   |
| ATOM | 2418 | CB  | VAL | D | 99  | 0.278  | 71.796 | -7.849  | 1.00 | 33.92 | C   |
| ATOM | 2419 | CG1 | VAL | D | 99  | 1.787  | 71.619 | -7.819  | 1.00 | 30.13 | C   |
| ATOM | 2420 | CG2 | VAL | D | 99  | -0.114 | 73.241 | -7.535  | 1.00 | 28.38 | C   |
| ATOM | 2421 | N   | VAL | D | 100 | 1.375  | 69.952 | -10.321 | 1.00 | 29.37 | N   |
| ATOM | 2422 | CA  | VAL | D | 100 | 1.871  | 68.706 | -10.875 | 1.00 | 30.81 | C   |
| ATOM | 2423 | C   | VAL | D | 100 | 3.380  | 68.659 | -10.653 | 1.00 | 33.23 | C   |
| ATOM | 2424 | O   | VAL | D | 100 | 4.072  | 69.662 | -10.890 | 1.00 | 32.58 | O   |
| ATOM | 2425 | CB  | VAL | D | 100 | 1.442  | 68.618 | -12.352 | 1.00 | 32.14 | C   |
| ATOM | 2426 | CG1 | VAL | D | 100 | 2.538  | 68.362 | -13.255 | 1.00 | 38.21 | C   |
| ATOM | 2427 | CG2 | VAL | D | 100 | 0.327  | 67.591 | -12.507 | 1.00 | 35.01 | C   |
| ATOM | 2428 | N   | PHE | D | 101 | 3.869  | 67.537 | -10.090 | 1.00 | 32.44 | N   |
| ATOM | 2429 | CA  | PHE | D | 101 | 5.292  | 67.296 | -9.826  | 1.00 | 28.74 | C   |
| ATOM | 2430 | C   | PHE | D | 101 | 5.824  | 66.191 | -10.728 | 1.00 | 30.41 | C   |
| ATOM | 2431 | O   | PHE | D | 101 | 5.091  | 65.280 | -11.116 | 1.00 | 36.07 | O   |
| ATOM | 2432 | CB  | PHE | D | 101 | 5.571  | 66.879 | -8.367  | 1.00 | 29.14 | C   |
| ATOM | 2433 | CG  | PHE | D | 101 | 5.505  | 68.006 | -7.372  | 1.00 | 29.14 | C   |
| ATOM | 2434 | CD1 | PHE | D | 101 | 6.472  | 68.991 | -7.355  | 1.00 | 29.41 | C   |
| ATOM | 2435 | CD2 | PHE | D | 101 | 4.483  | 68.067 | -6.438  | 1.00 | 29.40 | C   |
| ATOM | 2436 | CE1 | PHE | D | 101 | 6.421  | 70.041 | -6.430  | 1.00 | 30.71 | C   |
| ATOM | 2437 | CE2 | PHE | D | 101 | 4.412  | 69.107 | -5.519  | 1.00 | 31.48 | C   |
| ATOM | 2438 | CZ  | PHE | D | 101 | 5.388  | 70.100 | -5.509  | 1.00 | 28.28 | C   |
| ATOM | 2439 | N   | GLY | D | 102 | 7.112  | 66.269 | -11.069 | 1.00 | 35.21 | N   |
| ATOM | 2440 | CA  | GLY | D | 102 | 7.774  | 65.104 | -11.621 | 1.00 | 30.07 | C   |
| ATOM | 2441 | C   | GLY | D | 102 | 7.949  | 64.054 | -10.539 | 1.00 | 34.24 | C   |
| ATOM | 2442 | O   | GLY | D | 102 | 7.809  | 64.324 | -9.342  | 1.00 | 32.05 | O   |
| ATOM | 2443 | N   | GLY | D | 103 | 8.282  | 62.833 | -10.962 | 1.00 | 33.87 | N   |
| ATOM | 2444 | CA  | GLY | D | 103 | 8.500  | 61.756 | -9.998  | 1.00 | 30.14 | C   |
| ATOM | 2445 | C   | GLY | D | 103 | 9.767  | 61.896 | -9.164  | 1.00 | 33.06 | C   |
| ATOM | 2446 | O   | GLY | D | 103 | 9.929  | 61.153 | -8.195  | 1.00 | 33.57 | O   |
| ATOM | 2447 | N   | GLY | D | 104 | 10.649 | 62.840 | -9.500  | 1.00 | 34.22 | N   |
| ATOM | 2448 | CA  | GLY | D | 104 | 11.866 | 63.034 | -8.746  | 1.00 | 30.60 | C   |
| ATOM | 2449 | C   | GLY | D | 104 | 13.056 | 62.330 | -9.363  | 1.00 | 32.84 | C   |
| ATOM | 2450 | O   | GLY | D | 104 | 12.939 | 61.230 | -9.914  | 1.00 | 37.98 | O   |
| ATOM | 2451 | N   | THR | D | 105 | 14.219 | 62.953 | -9.267  | 1.00 | 33.92 | N   |
| ATOM | 2452 | CA  | THR | D | 105 | 15.460 | 62.372 | -9.749  | 1.00 | 32.14 | C   |
| ATOM | 2453 | C   | THR | D | 105 | 16.422 | 62.308 | -8.581  | 1.00 | 35.31 | C   |
| ATOM | 2454 | O   | THR | D | 105 | 16.683 | 63.335 | -7.945  | 1.00 | 33.86 | O   |
| ATOM | 2455 | CB  | THR | D | 105 | 16.052 | 63.202 | -10.878 | 1.00 | 35.69 | C   |
| ATOM | 2456 | OG1 | THR | D | 105 | 15.118 | 63.236 | -11.966 | 1.00 | 33.00 | O   |
| ATOM | 2457 | CG2 | THR | D | 105 | 17.374 | 62.575 | -11.328 | 1.00 | 32.50 | C   |
| ATOM | 2458 | N   | LYS | D | 106 | 16.895 | 61.095 | -8.260  | 1.00 | 35.10 | N   |
| ATOM | 2459 | CA  | LYS | D | 106 | 17.920 | 60.928 | -7.232  | 1.00 | 39.20 | C   |
| ATOM | 2460 | C   | LYS | D | 106 | 19.287 | 61.212 | -7.857  | 1.00 | 35.77 | C   |
| ATOM | 2461 | O   | LYS | D | 106 | 19.709 | 60.517 | -8.796  | 1.00 | 33.65 | O   |
| ATOM | 2462 | CB  | LYS | D | 106 | 17.875 | 59.523 | -6.612  | 1.00 | 37.13 | C   |
| ATOM | 2463 | CG  | LYS | D | 106 | 18.991 | 59.257 | -5.575  | 1.00 | 41.72 | C   |
| ATOM | 2464 | CD  | LYS | D | 106 | 18.843 | 57.864 | -4.893  | 1.00 | 66.03 | C   |
| ATOM | 2465 | CE  | LYS | D | 106 | 20.060 | 57.472 | -4.002  | 1.00 | 43.48 | C   |
| ATOM | 2466 | NZ  | LYS | D | 106 | 20.399 | 58.527 | -2.969  | 1.00 | 59.67 | N1+ |
| ATOM | 2467 | N   | LEU | D | 107 | 19.955 | 62.253 | -7.355  | 1.00 | 37.32 | N   |
| ATOM | 2468 | CA  | LEU | D | 107 | 21.313 | 62.606 | -7.757  | 1.00 | 39.36 | C   |
| ATOM | 2469 | C   | LEU | D | 107 | 22.264 | 62.033 | -6.720  | 1.00 | 37.65 | C   |
| ATOM | 2470 | O   | LEU | D | 107 | 22.211 | 62.419 | -5.542  | 1.00 | 38.47 | O   |
| ATOM | 2471 | CB  | LEU | D | 107 | 21.518 | 64.129 | -7.855  | 1.00 | 26.67 | C   |
| ATOM | 2472 | CG  | LEU | D | 107 | 22.784 | 64.731 | -8.570  | 1.00 | 37.81 | C   |
| ATOM | 2473 | CD1 | LEU | D | 107 | 23.134 | 66.182 | -8.167  | 1.00 | 38.40 | C   |
| ATOM | 2474 | CD2 | LEU | D | 107 | 24.043 | 63.923 | -8.466  | 1.00 | 34.25 | C   |
| ATOM | 2475 | N   | THR | D | 108 | 23.176 | 61.171 | -7.148  | 1.00 | 30.15 | N   |
| ATOM | 2476 | CA  | THR | D | 108 | 24.270 | 60.823 | -6.255  | 1.00 | 39.12 | C   |
| ATOM | 2477 | C   | THR | D | 108 | 25.591 | 61.351 | -6.810  | 1.00 | 33.08 | C   |
| ATOM | 2478 | O   | THR | D | 108 | 25.888 | 61.234 | -8.004  | 1.00 | 36.87 | O   |
| ATOM | 2479 | CB  | THR | D | 108 | 24.311 | 59.316 | -5.951  | 1.00 | 42.14 | C   |
| ATOM | 2480 | OG1 | THR | D | 108 | 25.514 | 58.733 | -6.459  | 1.00 | 51.43 | O   |
| ATOM | 2481 | CG2 | THR | D | 108 | 23.075 | 58.603 | -6.471  | 1.00 | 39.89 | C   |
| ATOM | 2482 | N   | VAL | D | 109 | 26.338 | 62.011 | -5.950  | 1.00 | 28.89 | N   |

```
ATOM   2483  CA   VAL D 109      27.679  62.461  -6.272  1.00 36.64          C
ATOM   2484  C    VAL D 109      28.632  61.337  -5.873  1.00 36.03          C
ATOM   2485  O    VAL D 109      28.838  61.088  -4.686  1.00 34.38          O
ATOM   2486  CB   VAL D 109      27.999  63.769  -5.547  1.00 39.07          C
ATOM   2487  CG1  VAL D 109      29.402  64.264  -5.930  1.00 39.69          C
ATOM   2488  CG2  VAL D 109      26.914  64.807  -5.871  1.00 39.03          C
ATOM   2489  N    LEU D 110      29.179  60.637  -6.868  1.00 36.80          N
ATOM   2490  CA   LEU D 110      29.935  59.407  -6.646  1.00 35.35          C
ATOM   2491  C    LEU D 110      31.166  59.653  -5.794  1.00 38.39          C
ATOM   2492  O    LEU D 110      32.109  60.294  -6.255  1.00 39.60          O
ATOM   2493  CB   LEU D 110      30.358  58.813  -7.978  1.00 31.68          C
ATOM   2494  CG   LEU D 110      29.190  58.470  -8.886  1.00 42.08          C
ATOM   2495  CD1  LEU D 110      29.720  58.230 -10.264  1.00 33.32          C
ATOM   2496  CD2  LEU D 110      28.489  57.239  -8.343  1.00 40.75          C
ATOM   2497  N    SER D 111      31.182  59.144  -4.564  1.00 38.34          N
ATOM   2498  CA   SER D 111      32.330  59.296  -3.680  1.00 44.10          C
ATOM   2499  C    SER D 111      32.991  57.956  -3.361  1.00 43.27          C
ATOM   2500  O    SER D 111      33.843  57.885  -2.476  1.00 38.62          O
ATOM   2501  CB   SER D 111      31.917  60.001  -2.396  1.00 39.42          C
ATOM   2502  OG   SER D 111      30.920  59.235  -1.763  1.00 48.72          O
ATOM   2503  N    GLN D 112      32.606  56.901  -4.058  1.00 36.34          N
ATOM   2504  CA   GLN D 112      33.300  55.623  -4.010  1.00 36.47          C
ATOM   2505  C    GLN D 112      32.964  54.900  -5.300  1.00 39.73          C
ATOM   2506  O    GLN D 112      32.074  55.336  -6.045  1.00 41.47          O
ATOM   2507  CB   GLN D 112      32.884  54.798  -2.774  1.00 34.11          C
ATOM   2508  CG   GLN D 112      31.427  54.370  -2.745  1.00 40.26          C
ATOM   2509  CD   GLN D 112      31.098  53.480  -1.545  1.00 44.54          C
ATOM   2510  OE1  GLN D 112      31.422  52.289  -1.542  1.00 49.63          O
ATOM   2511  NE2  GLN D 112      30.446  54.054  -0.523  1.00 32.71          N
ATOM   2512  N    PRO D 113      33.662  53.816  -5.614  1.00 35.83     GZ00 N
ATOM   2513  CA   PRO D 113      33.350  53.105  -6.859  1.00 29.36     GZ00 C
ATOM   2514  C    PRO D 113      31.937  52.559  -6.824  1.00 41.40     GZ00 C
ATOM   2515  O    PRO D 113      31.391  52.249  -5.763  1.00 33.72     GZ00 O
ATOM   2516  CB   PRO D 113      34.371  51.969  -6.897  1.00 29.06     GZ00 C
ATOM   2517  CG   PRO D 113      35.545  52.491  -6.029  1.00 36.02     GZ00 C
ATOM   2518  CD   PRO D 113      34.878  53.300  -4.948  1.00 37.13     GZ00 C
ATOM   2519  N    LYS D 114      31.346  52.445  -8.007  1.00 41.30     GZ00 N
ATOM   2520  CA   LYS D 114      30.050  51.806  -8.112  1.00 39.99     GZ00 C
ATOM   2521  C    LYS D 114      30.168  50.332  -7.726  1.00 42.17     GZ00 C
ATOM   2522  O    LYS D 114      31.189  49.691  -7.965  1.00 37.31     GZ00 O
ATOM   2523  CB   LYS D 114      29.510  51.967  -9.527  1.00 38.27     GZ00 C
ATOM   2524  CG   LYS D 114      29.198  53.426  -9.890  1.00 48.75     GZ00 C
ATOM   2525  CD   LYS D 114      28.597  53.502 -11.284  1.00 54.69     GZ00 C
ATOM   2526  CE   LYS D 114      27.812  54.779 -11.536  1.00 43.93     GZ00 C
ATOM   2527  NZ   LYS D 114      26.918  54.529 -12.718  1.00 45.98     GZ00 N1+
ATOM   2528  N    ALA D 115      29.108  49.806  -7.107  1.00 37.03     GZ00 N
ATOM   2529  CA   ALA D 115      29.084  48.455  -6.562  1.00 31.87     GZ00 C
ATOM   2530  C    ALA D 115      27.754  47.802  -6.906  1.00 41.28     GZ00 C
ATOM   2531  O    ALA D 115      26.691  48.304  -6.514  1.00 37.09     GZ00 O
ATOM   2532  CB   ALA D 115      29.280  48.470  -5.040  1.00 29.90     GZ00 C
ATOM   2533  N    ALA D 116      27.816  46.676  -7.610  1.00 34.16     GZ00 N
ATOM   2534  CA   ALA D 116      26.622  45.929  -7.933  1.00 37.15     GZ00 C
ATOM   2535  C    ALA D 116      26.071  45.289  -6.658  1.00 37.53     GZ00 C
ATOM   2536  O    ALA D 116      26.816  45.037  -5.715  1.00 41.85     GZ00 O
ATOM   2537  CB   ALA D 116      26.937  44.870  -8.989  1.00 28.78     GZ00 C
ATOM   2538  N    PRO D 117      24.772  45.028  -6.594  1.00 40.96     GZ00 N
ATOM   2539  CA   PRO D 117      24.207  44.481  -5.355  1.00 39.65     GZ00 C
ATOM   2540  C    PRO D 117      24.590  43.022  -5.180  1.00 40.39     GZ00 C
ATOM   2541  O    PRO D 117      24.686  42.263  -6.149  1.00 43.42     GZ00 O
ATOM   2542  CB   PRO D 117      22.699  44.620  -5.568  1.00 37.24     GZ00 C
ATOM   2543  CG   PRO D 117      22.553  44.435  -7.049  1.00 32.78     GZ00 C
ATOM   2544  CD   PRO D 117      23.761  45.109  -7.664  1.00 38.78     GZ00 C
ATOM   2545  N    SER D 118      24.817  42.626  -3.934  1.00 40.21     GZ00 N
ATOM   2546  CA   SER D 118      24.859  41.203  -3.626  1.00 43.65     GZ00 C
ATOM   2547  C    SER D 118      23.460  40.793  -3.190  1.00 44.22     GZ00 C
ATOM   2548  O    SER D 118      22.822  41.475  -2.371  1.00 39.83     GZ00 O
ATOM   2549  CB   SER D 118      25.901  40.867  -2.559  1.00 37.70     GZ00 C
ATOM   2550  OG   SER D 118      25.559  41.468  -1.335  1.00 51.03     GZ00 O
ATOM   2551  N    VAL D 119      22.982  39.701  -3.774  1.00 39.65     GZ00 N
ATOM   2552  CA   VAL D 119      21.609  39.244  -3.641  1.00 39.06     GZ00 C
ATOM   2553  C    VAL D 119      21.647  37.843  -3.065  1.00 44.09     GZ00 C
```

```
ATOM   2554   O     VAL D 119    22.315  36.966  -3.626  1.00 39.00        GZ00 O
ATOM   2555   CB    VAL D 119    20.894  39.235  -5.007  1.00 40.58        GZ00 C
ATOM   2556   CG1   VAL D 119    19.469  38.765  -4.865  1.00 33.51        GZ00 C
ATOM   2557   CG2   VAL D 119    20.964  40.602  -5.658  1.00 38.29        GZ00 C
ATOM   2558   N     THR D 120    20.928  37.620  -1.963  1.00 38.97        GZ00 N
ATOM   2559   CA    THR D 120    20.719  36.256  -1.507  1.00 40.40        GZ00 C
ATOM   2560   C     THR D 120    19.218  36.005  -1.360  1.00 41.57        GZ00 C
ATOM   2561   O     THR D 120    18.487  36.826  -0.798  1.00 39.96        GZ00 O
ATOM   2562   CB    THR D 120    21.524  35.939  -0.211  1.00 48.39        GZ00 C
ATOM   2563   OG1   THR D 120    20.649  35.655   0.882  1.00 55.57        GZ00 O
ATOM   2564   CG2   THR D 120    22.506  37.053   0.178  1.00 42.25        GZ00 C
ATOM   2565   N     LEU D 121    18.761  34.879  -1.903  1.00 42.65        GZ00 N
ATOM   2566   CA    LEU D 121    17.343  34.562  -2.031  1.00 44.51        GZ00 C
ATOM   2567   C     LEU D 121    17.063  33.332  -1.184  1.00 42.61        GZ00 C
ATOM   2568   O     LEU D 121    17.626  32.269  -1.444  1.00 39.39        GZ00 O
ATOM   2569   CB    LEU D 121    16.993  34.281  -3.495  1.00 42.30        GZ00 C
ATOM   2570   CG    LEU D 121    15.574  34.358  -4.059  1.00 45.15        GZ00 C
ATOM   2571   CD1   LEU D 121    15.333  33.234  -5.034  1.00 45.85        GZ00 C
ATOM   2572   CD2   LEU D 121    14.485  34.413  -2.986  1.00 44.86        GZ00 C
ATOM   2573   N     PHE D 122    16.200  33.467  -0.209  1.00 37.70        GZ00 N
ATOM   2574   CA    PHE D 122    15.865  32.293   0.587  1.00 43.33        GZ00 C
ATOM   2575   C     PHE D 122    14.487  31.766   0.221  1.00 49.36        GZ00 C
ATOM   2576   O     PHE D 122    13.539  32.551   0.069  1.00 41.30        GZ00 O
ATOM   2577   CB    PHE D 122    15.850  32.606   2.079  1.00 39.48        GZ00 C
ATOM   2578   CG    PHE D 122    17.181  32.910   2.660  1.00 38.94        GZ00 C
ATOM   2579   CD1   PHE D 122    18.057  31.887   2.982  1.00 38.46        GZ00 C
ATOM   2580   CD2   PHE D 122    17.533  34.229   2.949  1.00 36.53        GZ00 C
ATOM   2581   CE1   PHE D 122    19.288  32.169   3.550  1.00 45.61        GZ00 C
ATOM   2582   CE2   PHE D 122    18.740  34.526   3.528  1.00 38.72        GZ00 C
ATOM   2583   CZ    PHE D 122    19.636  33.494   3.830  1.00 40.64        GZ00 C
ATOM   2584   N     PRO D 123    14.347  30.448   0.130  1.00 50.41        GZ00 N
ATOM   2585   CA    PRO D 123    13.024  29.850  -0.048  1.00 46.66        GZ00 C
ATOM   2586   C     PRO D 123    12.262  29.853   1.266  1.00 47.32        GZ00 C
ATOM   2587   O     PRO D 123    12.829  30.182   2.317  1.00 41.78        GZ00 O
ATOM   2588   CB    PRO D 123    13.356  28.423  -0.505  1.00 53.34        GZ00 C
ATOM   2589   CG    PRO D 123    14.625  28.122   0.208  1.00 54.44        GZ00 C
ATOM   2590   CD    PRO D 123    15.402  29.428   0.251  1.00 49.34        GZ00 C
ATOM   2591   N     PRO D 124    10.968  29.530   1.259  1.00 54.23        GZ00 N
ATOM   2592   CA    PRO D 124    10.243  29.456   2.533  1.00 49.80        GZ00 C
ATOM   2593   C     PRO D 124    10.766  28.290   3.353  1.00 47.27        GZ00 C
ATOM   2594   O     PRO D 124    11.127  27.246   2.809  1.00 40.84        GZ00 O
ATOM   2595   CB    PRO D 124     8.786  29.239   2.109  1.00 52.85        GZ00 C
ATOM   2596   CG    PRO D 124     8.882  28.604   0.771  1.00 58.62        GZ00 C
ATOM   2597   CD    PRO D 124    10.120  29.144   0.117  1.00 50.64        GZ00 C
ATOM   2598   N     SER D 125    10.848  28.487   4.665  1.00 47.15        GZ00 N
ATOM   2599   CA    SER D 125    11.315  27.415   5.533  1.00 46.19        GZ00 C
ATOM   2600   C     SER D 125    10.226  26.360   5.725  1.00 55.91        GZ00 C
ATOM   2601   O     SER D 125     9.024  26.627   5.602  1.00 48.89        GZ00 O
ATOM   2602   CB    SER D 125    11.725  27.963   6.895  1.00 46.67        GZ00 C
ATOM   2603   OG    SER D 125    10.587  28.428   7.610  1.00 43.78        GZ00 O
ATOM   2604   N     SER D 126    10.661  25.150   6.076  1.00 53.45        GZ00 N
ATOM   2605   CA    SER D 126     9.697  24.081   6.294  1.00 49.33        GZ00 C
ATOM   2606   C     SER D 126     8.769  24.420   7.455  1.00 51.40        GZ00 C
ATOM   2607   O     SER D 126     7.564  24.166   7.392  1.00 57.31        GZ00 O
ATOM   2608   CB    SER D 126    10.432  22.769   6.528  1.00 46.88        GZ00 C
ATOM   2609   OG    SER D 126    11.378  22.938   7.552  1.00 58.67        GZ00 O
ATOM   2610   N     GLU D 127     9.303  25.045   8.500  1.00 46.78        GZ00 N
ATOM   2611   CA    GLU D 127     8.464  25.457   9.621  1.00 50.81        GZ00 C
ATOM   2612   C     GLU D 127     7.347  26.393   9.173  1.00 60.09        GZ00 C
ATOM   2613   O     GLU D 127     6.256  26.382   9.759  1.00 61.69        GZ00 O
ATOM   2614   CB    GLU D 127     9.329  26.118  10.686  1.00 53.57        GZ00 C
ATOM   2615   CG    GLU D 127    10.626  25.340  10.894  1.00 62.62        GZ00 C
ATOM   2616   CD    GLU D 127    11.443  25.816  12.072  1.00 72.05        GZ00 C
ATOM   2617   OE1   GLU D 127    10.899  26.562  12.933  1.00 69.83        GZ00 O
ATOM   2618   OE2   GLU D 127    12.635  25.426  12.129  1.00 72.47        GZ00 O1-
ATOM   2619   N     GLU D 128     7.608  27.240   8.170  1.00 56.85        GZ00 N
ATOM   2620   CA    GLU D 128     6.568  28.148   7.700  1.00 54.23        GZ00 C
ATOM   2621   C     GLU D 128     5.590  27.441   6.774  1.00 52.82        GZ00 C
ATOM   2622   O     GLU D 128     4.390  27.728   6.804  1.00 53.66        GZ00 O
ATOM   2623   CB    GLU D 128     7.174  29.364   6.983  1.00 52.45        GZ00 C
ATOM   2624   CG    GLU D 128     6.132  30.482   6.745  1.00 57.44        GZ00 C
```

| ATOM | 2625 | CD | GLU | D | 128 | 6.545 | 31.555 | 5.722 | 1.00 | 58.50 | GZ00 | C |
| ATOM | 2626 | OE1 | GLU | D | 128 | 5.856 | 32.590 | 5.684 | 1.00 | 54.07 | GZ00 | O |
| ATOM | 2627 | OE2 | GLU | D | 128 | 7.518 | 31.373 | 4.950 | 1.00 | 49.75 | GZ00 | O1- |
| ATOM | 2628 | N | LEU | D | 129 | 6.095 | 26.539 | 5.927 | 1.00 | 52.50 | GZ00 | N |
| ATOM | 2629 | CA | LEU | D | 129 | 5.226 | 25.707 | 5.104 | 1.00 | 51.40 | GZ00 | C |
| ATOM | 2630 | C | LEU | D | 129 | 4.279 | 24.879 | 5.969 | 1.00 | 59.74 | GZ00 | C |
| ATOM | 2631 | O | LEU | D | 129 | 3.106 | 24.702 | 5.623 | 1.00 | 63.72 | GZ00 | O |
| ATOM | 2632 | CB | LEU | D | 129 | 6.078 | 24.807 | 4.214 | 1.00 | 51.65 | GZ00 | C |
| ATOM | 2633 | CG | LEU | D | 129 | 6.868 | 25.538 | 3.131 | 1.00 | 52.87 | GZ00 | C |
| ATOM | 2634 | CD1 | LEU | D | 129 | 7.857 | 24.604 | 2.459 | 1.00 | 44.54 | GZ00 | C |
| ATOM | 2635 | CD2 | LEU | D | 129 | 5.917 | 26.140 | 2.101 | 1.00 | 49.64 | GZ00 | C |
| ATOM | 2636 | N | GLN | D | 130 | 4.767 | 24.388 | 7.115 | 1.00 | 54.99 | GZ00 | N |
| ATOM | 2637 | CA | GLN | D | 130 | 3.913 | 23.640 | 8.032 | 1.00 | 66.06 | GZ00 | C |
| ATOM | 2638 | C | GLN | D | 130 | 2.760 | 24.489 | 8.552 | 1.00 | 64.20 | GZ00 | C |
| ATOM | 2639 | O | GLN | D | 130 | 1.673 | 23.958 | 8.810 | 1.00 | 68.64 | GZ00 | O |
| ATOM | 2640 | CB | GLN | D | 130 | 4.729 | 23.097 | 9.210 | 1.00 | 64.34 | GZ00 | C |
| ATOM | 2641 | CG | GLN | D | 130 | 5.729 | 22.005 | 8.863 | 1.00 | 65.01 | GZ00 | C |
| ATOM | 2642 | CD | GLN | D | 130 | 6.363 | 21.409 | 10.113 | 1.00 | 78.91 | GZ00 | C |
| ATOM | 2643 | OE1 | GLN | D | 130 | 5.663 | 21.103 | 11.083 | 1.00 | 88.40 | GZ00 | O |
| ATOM | 2644 | NE2 | GLN | D | 130 | 7.691 | 21.262 | 10.108 | 1.00 | 62.99 | GZ00 | N |
| ATOM | 2645 | N | ALA | D | 131 | 2.972 | 25.794 | 8.729 | 1.00 | 53.92 | GZ00 | N |
| ATOM | 2646 | CA | ALA | D | 131 | 1.903 | 26.700 | 9.129 | 1.00 | 51.41 | GZ00 | C |
| ATOM | 2647 | C | ALA | D | 131 | 1.100 | 27.196 | 7.943 | 1.00 | 56.66 | GZ00 | C |
| ATOM | 2648 | O | ALA | D | 131 | 0.366 | 28.184 | 8.075 | 1.00 | 63.18 | GZ00 | O |
| ATOM | 2649 | CB | ALA | D | 131 | 2.459 | 27.889 | 9.915 | 1.00 | 45.31 | GZ00 | C |
| ATOM | 2650 | N | ASN | D | 132 | 1.246 | 26.543 | 6.784 | 1.00 | 55.73 | GZ00 | N |
| ATOM | 2651 | CA | ASN | D | 132 | 0.472 | 26.840 | 5.573 | 1.00 | 65.28 | GZ00 | C |
| ATOM | 2652 | C | ASN | D | 132 | 0.645 | 28.283 | 5.100 | 1.00 | 66.61 | GZ00 | C |
| ATOM | 2653 | O | ASN | D | 132 | -0.264 | 28.869 | 4.507 | 1.00 | 68.44 | GZ00 | O |
| ATOM | 2654 | CB | ASN | D | 132 | -1.018 | 26.502 | 5.762 | 1.00 | 67.97 | GZ00 | C |
| ATOM | 2655 | CG | ASN | D | 132 | -1.347 | 25.065 | 5.335 | 1.00 | 88.31 | GZ00 | C |
| ATOM | 2656 | OD1 | ASN | D | 132 | -1.487 | 24.776 | 4.134 | 1.00 | 78.43 | GZ00 | O |
| ATOM | 2657 | ND2 | ASN | D | 132 | -1.448 | 24.155 | 6.318 | 1.00 | 80.50 | GZ00 | N |
| ATOM | 2658 | N | LYS | D | 133 | 1.812 | 28.866 | 5.352 | 1.00 | 68.70 | GZ00 | N |
| ATOM | 2659 | CA | LYS | D | 133 | 2.244 | 30.090 | 4.696 | 1.00 | 59.67 | GZ00 | C |
| ATOM | 2660 | C | LYS | D | 133 | 3.522 | 29.799 | 3.923 | 1.00 | 57.71 | GZ00 | C |
| ATOM | 2661 | O | LYS | D | 133 | 4.135 | 28.737 | 4.069 | 1.00 | 58.17 | GZ00 | O |
| ATOM | 2662 | CB | LYS | D | 133 | 2.468 | 31.225 | 5.701 | 1.00 | 52.27 | GZ00 | C |
| ATOM | 2663 | CG | LYS | D | 133 | 1.236 | 31.650 | 6.454 | 1.00 | 58.15 | GZ00 | C |
| ATOM | 2664 | CD | LYS | D | 133 | 1.590 | 32.644 | 7.547 | 1.00 | 82.46 | GZ00 | C |
| ATOM | 2665 | CE | LYS | D | 133 | 0.389 | 32.977 | 8.428 | 1.00 | 90.01 | GZ00 | C |
| ATOM | 2666 | NZ | LYS | D | 133 | -0.049 | 31.789 | 9.221 | 1.00 | 85.20 | GZ00 | N1+ |
| ATOM | 2667 | N | ALA | D | 134 | 3.923 | 30.754 | 3.087 | 1.00 | 59.30 | GZ00 | N |
| ATOM | 2668 | CA | ALA | D | 134 | 5.167 | 30.615 | 2.328 | 1.00 | 57.19 | GZ00 | C |
| ATOM | 2669 | C | ALA | D | 134 | 5.607 | 31.994 | 1.872 | 1.00 | 56.76 | GZ00 | C |
| ATOM | 2670 | O | ALA | D | 134 | 4.842 | 32.692 | 1.196 | 1.00 | 55.80 | GZ00 | O |
| ATOM | 2671 | CB | ALA | D | 134 | 4.985 | 29.678 | 1.129 | 1.00 | 53.33 | GZ00 | C |
| ATOM | 2672 | N | THR | D | 135 | 6.826 | 32.399 | 2.238 | 1.00 | 51.12 | GZ00 | N |
| ATOM | 2673 | CA | THR | D | 135 | 7.368 | 33.652 | 1.730 | 1.00 | 46.27 | GZ00 | C |
| ATOM | 2674 | C | THR | D | 135 | 8.764 | 33.429 | 1.168 | 1.00 | 50.06 | GZ00 | C |
| ATOM | 2675 | O | THR | D | 135 | 9.604 | 32.783 | 1.803 | 1.00 | 41.99 | GZ00 | O |
| ATOM | 2676 | CB | THR | D | 135 | 7.422 | 34.735 | 2.803 | 1.00 | 43.17 | GZ00 | C |
| ATOM | 2677 | OG1 | THR | D | 135 | 8.619 | 34.572 | 3.555 | 1.00 | 70.40 | GZ00 | O |
| ATOM | 2678 | CG2 | THR | D | 135 | 6.270 | 34.632 | 3.735 | 1.00 | 40.85 | GZ00 | C |
| ATOM | 2679 | N | LEU | D | 136 | 9.002 | 33.964 | -0.025 | 1.00 | 49.86 | GZ00 | N |
| ATOM | 2680 | CA | LEU | D | 136 | 10.344 | 34.047 | -0.574 | 1.00 | 43.03 | GZ00 | C |
| ATOM | 2681 | C | LEU | D | 136 | 10.956 | 35.371 | -0.127 | 1.00 | 44.71 | GZ00 | C |
| ATOM | 2682 | O | LEU | D | 136 | 10.274 | 36.400 | -0.088 | 1.00 | 45.54 | GZ00 | O |
| ATOM | 2683 | CB | LEU | D | 136 | 10.328 | 33.951 | -2.101 | 1.00 | 48.82 | GZ00 | C |
| ATOM | 2684 | CG | LEU | D | 136 | 9.744 | 32.685 | -2.734 | 1.00 | 50.57 | GZ00 | C |
| ATOM | 2685 | CD1 | LEU | D | 136 | 9.722 | 32.777 | -4.241 | 1.00 | 43.90 | GZ00 | C |
| ATOM | 2686 | CD2 | LEU | D | 136 | 10.592 | 31.534 | -2.313 | 1.00 | 54.51 | GZ00 | C |
| ATOM | 2687 | N | VAL | D | 137 | 12.231 | 35.329 | 0.251 | 1.00 | 38.61 | GZ00 | N |
| ATOM | 2688 | CA | VAL | D | 137 | 12.914 | 36.459 | 0.867 | 1.00 | 39.76 | GZ00 | C |
| ATOM | 2689 | C | VAL | D | 137 | 14.144 | 36.781 | 0.037 | 1.00 | 43.30 | GZ00 | C |
| ATOM | 2690 | O | VAL | D | 137 | 15.046 | 35.946 | -0.086 | 1.00 | 43.19 | GZ00 | O |
| ATOM | 2691 | CB | VAL | D | 137 | 13.286 | 36.167 | 2.326 | 1.00 | 40.83 | GZ00 | C |
| ATOM | 2692 | CG1 | VAL | D | 137 | 13.930 | 37.386 | 2.954 | 1.00 | 40.34 | GZ00 | C |
| ATOM | 2693 | CG2 | VAL | D | 137 | 12.032 | 35.756 | 3.116 | 1.00 | 39.27 | GZ00 | C |
| ATOM | 2694 | N | CYS | D | 138 | 14.161 | 37.969 | -0.563 | 1.00 | 39.42 | GZ00 | N |
| ATOM | 2695 | CA | CYS | D | 138 | 15.270 | 38.436 | -1.392 | 1.00 | 38.06 | GZ00 | C |

```
ATOM   2696  C    CYS D 138      15.958  39.590  -0.669  1.00 42.98          GZ00 C
ATOM   2697  O    CYS D 138      15.379  40.679  -0.549  1.00 42.34          GZ00 O
ATOM   2698  CB   CYS D 138      14.764  38.869  -2.767  1.00 46.28          GZ00 C
ATOM   2699  SG   CYS D 138      16.005  39.055  -4.058  1.00 46.59          GZ00 S
ATOM   2700  N    LEU D 139      17.165  39.345  -0.149  1.00 35.86          GZ00 N
ATOM   2701  CA   LEU D 139      17.953  40.386   0.503  1.00 34.94          GZ00 C
ATOM   2702  C    LEU D 139      18.994  40.946  -0.456  1.00 40.09          GZ00 C
ATOM   2703  O    LEU D 139      19.692  40.198  -1.153  1.00 38.00          GZ00 O
ATOM   2704  CB   LEU D 139      18.641  39.914   1.784  1.00 38.60          GZ00 C
ATOM   2705  CG   LEU D 139      17.851  39.429   2.994  1.00 42.05          GZ00 C
ATOM   2706  CD1  LEU D 139      16.412  39.996   2.992  1.00 34.31          GZ00 C
ATOM   2707  CD2  LEU D 139      17.888  37.954   3.110  1.00 41.56          GZ00 C
ATOM   2708  N    ILE D 140      19.072  42.271  -0.498  1.00 36.85          GZ00 N
ATOM   2709  CA   ILE D 140      19.852  42.991  -1.490  1.00 40.84          GZ00 C
ATOM   2710  C    ILE D 140      20.717  43.982  -0.735  1.00 39.30          GZ00 C
ATOM   2711  O    ILE D 140      20.196  44.821   0.012  1.00 36.82          GZ00 O
ATOM   2712  CB   ILE D 140      18.946  43.703  -2.510  1.00 36.07          GZ00 C
ATOM   2713  CG1  ILE D 140      17.852  42.738  -2.998  1.00 41.84          GZ00 C
ATOM   2714  CG2  ILE D 140      19.749  44.191  -3.673  1.00 33.32          GZ00 C
ATOM   2715  CD1  ILE D 140      16.606  43.414  -3.540  1.00 40.09          GZ00 C
ATOM   2716  N    SER D 141      22.028  43.899  -0.928  1.00 33.24          GZ00 N
ATOM   2717  CA   SER D 141      22.918  44.678  -0.083  1.00 40.45          GZ00 C
ATOM   2718  C    SER D 141      24.146  45.120  -0.865  1.00 40.51          GZ00 C
ATOM   2719  O    SER D 141      24.453  44.592  -1.948  1.00 33.29          GZ00 O
ATOM   2720  CB   SER D 141      23.348  43.878   1.155  1.00 39.86          GZ00 C
ATOM   2721  OG   SER D 141      23.964  42.664   0.748  1.00 40.92          GZ00 O
ATOM   2722  N    ASP D 142      24.835  46.117  -0.288  1.00 34.55          GZ00 N
ATOM   2723  CA   ASP D 142      26.137  46.573  -0.776  1.00 45.39          GZ00 C
ATOM   2724  C    ASP D 142      26.059  47.148  -2.192  1.00 42.72          GZ00 C
ATOM   2725  O    ASP D 142      26.985  46.983  -2.988  1.00 37.05          GZ00 O
ATOM   2726  CB   ASP D 142      27.165  45.436  -0.742  1.00 42.27          GZ00 C
ATOM   2727  CG   ASP D 142      27.636  45.114   0.650  1.00 45.03          GZ00 C
ATOM   2728  OD1  ASP D 142      27.734  46.043   1.489  1.00 49.79          GZ00 O
ATOM   2729  OD2  ASP D 142      27.890  43.921   0.902  1.00 52.25          GZ00 O1-
ATOM   2730  N    PHE D 143      24.967  47.826  -2.529  1.00 33.33          GZ00 N
ATOM   2731  CA   PHE D 143      24.908  48.407  -3.858  1.00 35.29          GZ00 C
ATOM   2732  C    PHE D 143      25.067  49.921  -3.775  1.00 33.78          GZ00 C
ATOM   2733  O    PHE D 143      24.717  50.556  -2.773  1.00 36.42          GZ00 O
ATOM   2734  CB   PHE D 143      23.637  48.009  -4.617  1.00 34.97          GZ00 C
ATOM   2735  CG   PHE D 143      22.340  48.333  -3.911  1.00 35.61          GZ00 C
ATOM   2736  CD1  PHE D 143      21.712  49.549  -4.113  1.00 32.64          GZ00 C
ATOM   2737  CD2  PHE D 143      21.709  47.379  -3.112  1.00 38.53          GZ00 C
ATOM   2738  CE1  PHE D 143      20.485  49.834  -3.493  1.00 41.16          GZ00 C
ATOM   2739  CE2  PHE D 143      20.494  47.658  -2.486  1.00 40.15          GZ00 C
ATOM   2740  CZ   PHE D 143      19.880  48.890  -2.672  1.00 32.95          GZ00 C
ATOM   2741  N    TYR D 144      25.665  50.471  -4.824  1.00 35.76          GZ00 N
ATOM   2742  CA   TYR D 144      26.002  51.882  -4.906  1.00 37.17          GZ00 C
ATOM   2743  C    TYR D 144      26.142  52.286  -6.363  1.00 41.07          GZ00 C
ATOM   2744  O    TYR D 144      26.899  51.649  -7.096  1.00 40.05          GZ00 O
ATOM   2745  CB   TYR D 144      27.305  52.176  -4.182  1.00 31.07          GZ00 C
ATOM   2746  CG   TYR D 144      27.652  53.644  -4.148  1.00 33.84          GZ00 C
ATOM   2747  CD1  TYR D 144      27.125  54.478  -3.168  1.00 34.22          GZ00 C
ATOM   2748  CD2  TYR D 144      28.485  54.207  -5.107  1.00 34.49          GZ00 C
ATOM   2749  CE1  TYR D 144      27.433  55.822  -3.129  1.00 36.40          GZ00 C
ATOM   2750  CE2  TYR D 144      28.800  55.559  -5.076  1.00 35.36          GZ00 C
ATOM   2751  CZ   TYR D 144      28.271  56.358  -4.082  1.00 35.95          GZ00 C
ATOM   2752  OH   TYR D 144      28.578  57.699  -4.037  1.00 40.59          GZ00 O
ATOM   2753  N    PRO D 145      25.442  53.357  -6.782  1.00 35.13          GZ00 N
ATOM   2754  CA   PRO D 145      24.556  54.168  -5.930  1.00 41.08          GZ00 C
ATOM   2755  C    PRO D 145      23.258  53.476  -5.465  1.00 39.60          GZ00 C
ATOM   2756  O    PRO D 145      22.944  52.359  -5.874  1.00 35.16          GZ00 O
ATOM   2757  CB   PRO D 145      24.231  55.395  -6.811  1.00 40.78          GZ00 C
ATOM   2758  CG   PRO D 145      24.659  55.048  -8.190  1.00 39.08          GZ00 C
ATOM   2759  CD   PRO D 145      25.708  53.989  -8.088  1.00 36.76          GZ00 C
ATOM   2760  N    GLY D 146      22.514  54.161  -4.600  1.00 34.87          GZ00 N
ATOM   2761  CA   GLY D 146      21.429  53.525  -3.892  1.00 34.83          GZ00 C
ATOM   2762  C    GLY D 146      20.087  53.444  -4.593  1.00 36.93          GZ00 C
ATOM   2763  O    GLY D 146      19.088  53.931  -4.062  1.00 34.26          GZ00 O
ATOM   2764  N    ALA D 147      20.037  52.844  -5.777  1.00 32.90          GZ00 N
ATOM   2765  CA   ALA D 147      18.750  52.609  -6.410  1.00 32.84          GZ00 C
ATOM   2766  C    ALA D 147      18.802  51.314  -7.178  1.00 32.60          GZ00 C
```

```
ATOM    2767  O    ALA D 147     19.792  51.008  -7.841  1.00 39.02          GZ00 O
ATOM    2768  CB   ALA D 147     18.326  53.719  -7.365  1.00 29.65          GZ00 C
ATOM    2769  N    VAL D 148     17.684  50.603  -7.141  1.00 33.58          GZ00 N
ATOM    2770  CA   VAL D 148     17.588  49.253  -7.651  1.00 34.66          GZ00 C
ATOM    2771  C    VAL D 148     16.121  49.045  -7.985  1.00 41.83          GZ00 C
ATOM    2772  O    VAL D 148     15.247  49.684  -7.396  1.00 32.66          GZ00 O
ATOM    2773  CB   VAL D 148     18.126  48.264  -6.582  1.00 38.73          GZ00 C
ATOM    2774  CG1  VAL D 148     17.030  47.484  -5.915  1.00 37.24          GZ00 C
ATOM    2775  CG2  VAL D 148     19.218  47.381  -7.146  1.00 41.31          GZ00 C
ATOM    2776  N    THR D 149     15.845  48.198  -8.967  1.00 38.40          GZ00 N
ATOM    2777  CA   THR D 149     14.472  47.753  -9.182  1.00 44.38          GZ00 C
ATOM    2778  C    THR D 149     14.427  46.229  -9.138  1.00 46.22          GZ00 C
ATOM    2779  O    THR D 149     15.385  45.556  -9.546  1.00 43.23          GZ00 O
ATOM    2780  CB   THR D 149     13.899  48.266 -10.513  1.00 46.92          GZ00 C
ATOM    2781  OG1  THR D 149     14.727  47.815 -11.599  1.00 50.51          GZ00 O
ATOM    2782  CG2  THR D 149     13.810  49.796 -10.512  1.00 38.30          GZ00 C
ATOM    2783  N    VAL D 150     13.311  45.686  -8.642  1.00 36.64          GZ00 N
ATOM    2784  CA   VAL D 150     13.203  44.262  -8.355  1.00 39.12          GZ00 C
ATOM    2785  C    VAL D 150     12.046  43.662  -9.128  1.00 38.75          GZ00 C
ATOM    2786  O    VAL D 150     10.920  44.160  -9.054  1.00 46.61          GZ00 O
ATOM    2787  CB   VAL D 150     13.032  43.987  -6.853  1.00 39.37          GZ00 C
ATOM    2788  CG1  VAL D 150     12.929  42.488  -6.631  1.00 41.26          GZ00 C
ATOM    2789  CG2  VAL D 150     14.213  44.522  -6.121  1.00 34.76          GZ00 C
ATOM    2790  N    ALA D 151     12.333  42.596  -9.865  1.00 39.71          GZ00 N
ATOM    2791  CA   ALA D 151     11.333  41.805 -10.560  1.00 44.18          GZ00 C
ATOM    2792  C    ALA D 151     11.434  40.350 -10.127  1.00 50.02          GZ00 C
ATOM    2793  O    ALA D 151     12.534  39.784 -10.032  1.00 47.26          GZ00 O
ATOM    2794  CB   ALA D 151     11.494  41.895 -12.081  1.00 37.34          GZ00 C
ATOM    2795  N    TRP D 152     10.276  39.748  -9.889  1.00 47.18          GZ00 N
ATOM    2796  CA   TRP D 152     10.164  38.343  -9.544  1.00 47.04          GZ00 C
ATOM    2797  C    TRP D 152      9.629  37.556 -10.731  1.00 49.55          GZ00 C
ATOM    2798  O    TRP D 152      8.800  38.055 -11.498  1.00 59.75          GZ00 O
ATOM    2799  CB   TRP D 152      9.248  38.155  -8.345  1.00 41.47          GZ00 C
ATOM    2800  CG   TRP D 152      9.799  38.641  -7.051  1.00 45.78          GZ00 C
ATOM    2801  CD1  TRP D 152      9.786  39.918  -6.579  1.00 49.07          GZ00 C
ATOM    2802  CD2  TRP D 152     10.400  37.837  -6.026  1.00 43.34          GZ00 C
ATOM    2803  NE1  TRP D 152     10.361  39.967  -5.325  1.00 46.95          GZ00 N
ATOM    2804  CE2  TRP D 152     10.736  38.700  -4.961  1.00 46.03          GZ00 C
ATOM    2805  CE3  TRP D 152     10.699  36.468  -5.914  1.00 44.86          GZ00 C
ATOM    2806  CZ2  TRP D 152     11.364  38.245  -3.792  1.00 48.01          GZ00 C
ATOM    2807  CZ3  TRP D 152     11.318  36.008  -4.748  1.00 46.08          GZ00 C
ATOM    2808  CH2  TRP D 152     11.649  36.905  -3.702  1.00 46.48          GZ00 C
ATOM    2809  N    LYS D 153     10.109  36.330 -10.882  1.00 54.87          GZ00 N
ATOM    2810  CA   LYS D 153      9.649  35.434 -11.929  1.00 57.27          GZ00 C
ATOM    2811  C    LYS D 153      9.247  34.090 -11.331  1.00 66.16          GZ00 C
ATOM    2812  O    LYS D 153      9.884  33.591 -10.396  1.00 64.92          GZ00 O
ATOM    2813  CB   LYS D 153     10.728  35.247 -12.999  1.00 61.27          GZ00 C
ATOM    2814  CG   LYS D 153     10.832  36.442 -13.910  1.00 66.71          GZ00 C
ATOM    2815  CD   LYS D 153     11.805  36.232 -15.044  1.00 71.68          GZ00 C
ATOM    2816  CE   LYS D 153     11.839  37.483 -15.930  1.00 79.74          GZ00 C
ATOM    2817  NZ   LYS D 153     12.960  37.469 -16.930  1.00 89.60          GZ00 N1+
ATOM    2818  N    ALA D 154      8.145  33.543 -11.836  1.00 68.05          GZ00 N
ATOM    2819  CA   ALA D 154      7.729  32.169 -11.568  1.00 66.27          GZ00 C
ATOM    2820  C    ALA D 154      7.933  31.392 -12.864  1.00 70.66          GZ00 C
ATOM    2821  O    ALA D 154      7.229  31.641 -13.848  1.00 74.13          GZ00 O
ATOM    2822  CB   ALA D 154      6.280  32.108 -11.092  1.00 57.49          GZ00 C
ATOM    2823  N    ASP D 155      8.892  30.464 -12.866  1.00 69.01          GZ00 N
ATOM    2824  CA   ASP D 155      9.320  29.751 -14.079  1.00 76.96          GZ00 C
ATOM    2825  C    ASP D 155      9.440  30.697 -15.267  1.00 78.16          GZ00 C
ATOM    2826  O    ASP D 155      8.849  30.482 -16.327  1.00 86.27          GZ00 O
ATOM    2827  CB   ASP D 155      8.342  28.625 -14.445  1.00 79.86          GZ00 C
ATOM    2828  CG   ASP D 155      8.311  27.503 -13.442  1.00 77.78          GZ00 C
ATOM    2829  OD1  ASP D 155      9.379  27.134 -12.919  1.00 80.60          GZ00 O1-
ATOM    2830  OD2  ASP D 155      7.205  26.974 -13.197  1.00 86.69          GZ00 O
ATOM    2831  N    SER D 156     10.188  31.777 -15.083  1.00 72.25          GZ00 N
ATOM    2832  CA   SER D 156     10.451  32.741 -16.147  1.00 79.24          GZ00 C
ATOM    2833  C    SER D 156      9.207  33.506 -16.599  1.00 70.74          GZ00 C
ATOM    2834  O    SER D 156      9.242  34.141 -17.656  1.00 70.59          GZ00 O
ATOM    2835  CB   SER D 156     11.103  32.085 -17.366  1.00 79.59          GZ00 C
ATOM    2836  OG   SER D 156     10.117  31.458 -18.157  1.00 81.93          GZ00 O
ATOM    2837  N    SER D 157      8.086  33.449 -15.840  1.00 66.95          GZ00 N
```

```
ATOM   2838  CA   SER D 157       6.934  34.326 -16.090  1.00 65.14      GZ00 C
ATOM   2839  C    SER D 157       6.878  35.425 -15.047  1.00 67.53      GZ00 C
ATOM   2840  O    SER D 157       7.031  35.145 -13.848  1.00 64.92      GZ00 O
ATOM   2841  CB   SER D 157       5.624  33.544 -16.060  1.00 63.23      GZ00 C
ATOM   2842  OG   SER D 157       5.562  32.633 -17.136  1.00 75.81      GZ00 O
ATOM   2843  N    PRO D 158       6.652  36.670 -15.446  1.00 66.82      GZ00 N
ATOM   2844  CA   PRO D 158       6.585  37.756 -14.462  1.00 56.39      GZ00 C
ATOM   2845  C    PRO D 158       5.538  37.504 -13.392  1.00 59.58      GZ00 C
ATOM   2846  O    PRO D 158       4.450  36.989 -13.660  1.00 66.88      GZ00 O
ATOM   2847  CB   PRO D 158       6.236  38.986 -15.308  1.00 52.59      GZ00 C
ATOM   2848  CG   PRO D 158       5.845  38.451 -16.652  1.00 66.17      GZ00 C
ATOM   2849  CD   PRO D 158       6.585  37.169 -16.824  1.00 63.85      GZ00 C
ATOM   2850  N    VAL D 159       5.900  37.834 -12.160  1.00 59.53      GZ00 N
ATOM   2851  CA   VAL D 159       5.012  37.741 -11.012  1.00 57.33      GZ00 C
ATOM   2852  C    VAL D 159       4.480  39.133 -10.721  1.00 64.87      GZ00 C
ATOM   2853  O    VAL D 159       5.261  40.073 -10.535  1.00 75.66      GZ00 O
ATOM   2854  CB   VAL D 159       5.735  37.169  -9.785  1.00 58.46      GZ00 C
ATOM   2855  CG1  VAL D 159       4.831  37.239  -8.567  1.00 61.48      GZ00 C
ATOM   2856  CG2  VAL D 159       6.174  35.737 -10.050  1.00 55.69      GZ00 C
ATOM   2857  N    LYS D 160       3.157  39.269 -10.654  1.00 74.88      GZ00 N
ATOM   2858  CA   LYS D 160       2.552  40.558 -10.329  1.00 76.06      GZ00 C
ATOM   2859  C    LYS D 160       2.274  40.696  -8.831  1.00 82.77      GZ00 C
ATOM   2860  O    LYS D 160       2.850  41.565  -8.160  1.00 78.66      GZ00 O
ATOM   2861  CB   LYS D 160       1.265  40.755 -11.142  1.00 83.14      GZ00 C
ATOM   2862  CG   LYS D 160       1.460  41.529 -12.445  1.00 91.80      GZ00 C
ATOM   2863  CD   LYS D 160       2.006  42.934 -12.175  1.00100.07      GZ00 C
ATOM   2864  CE   LYS D 160       1.058  43.758 -11.302  1.00 96.51      GZ00 C
ATOM   2865  NZ   LYS D 160       1.729  44.973 -10.750  1.00 94.46      GZ00 N1+
ATOM   2866  N    ALA D 161       1.410  39.836  -8.293  1.00 72.49      GZ00 N
ATOM   2867  CA   ALA D 161       0.885  40.022  -6.947  1.00 69.27      GZ00 C
ATOM   2868  C    ALA D 161       1.739  39.312  -5.903  1.00 63.52      GZ00 C
ATOM   2869  O    ALA D 161       2.490  38.379  -6.201  1.00 56.61      GZ00 O
ATOM   2870  CB   ALA D 161      -0.551  39.514  -6.859  1.00 71.61      GZ00 C
ATOM   2871  N    GLY D 162       1.608  39.774  -4.659  1.00 50.21      GZ00 N
ATOM   2872  CA   GLY D 162       2.313  39.191  -3.536  1.00 58.65      GZ00 C
ATOM   2873  C    GLY D 162       3.711  39.714  -3.291  1.00 57.47      GZ00 C
ATOM   2874  O    GLY D 162       4.417  39.167  -2.426  1.00 54.30      GZ00 O
ATOM   2875  N    VAL D 163       4.135  40.745  -4.023  1.00 51.81      GZ00 N
ATOM   2876  CA   VAL D 163       5.480  41.291  -3.929  1.00 45.48      GZ00 C
ATOM   2877  C    VAL D 163       5.431  42.543  -3.074  1.00 47.42      GZ00 C
ATOM   2878  O    VAL D 163       4.618  43.442  -3.316  1.00 44.17      GZ00 O
ATOM   2879  CB   VAL D 163       6.060  41.604  -5.319  1.00 46.37      GZ00 C
ATOM   2880  CG1  VAL D 163       7.423  42.278  -5.184  1.00 50.48      GZ00 C
ATOM   2881  CG2  VAL D 163       6.183  40.338  -6.140  1.00 47.00      GZ00 C
ATOM   2882  N    GLU D 164       6.301  42.609  -2.076  1.00 45.49      GZ00 N
ATOM   2883  CA   GLU D 164       6.498  43.832  -1.326  1.00 39.71      GZ00 C
ATOM   2884  C    GLU D 164       7.990  44.067  -1.169  1.00 48.75      GZ00 C
ATOM   2885  O    GLU D 164       8.749  43.133  -0.864  1.00 44.83      GZ00 O
ATOM   2886  CB   GLU D 164       5.764  43.793   0.009  1.00 46.37      GZ00 C
ATOM   2887  CG   GLU D 164       4.308  44.218  -0.195  1.00 59.54      GZ00 C
ATOM   2888  CD   GLU D 164       3.415  43.931   0.978  1.00 60.43      GZ00 C
ATOM   2889  OE1  GLU D 164       3.928  43.465   2.015  1.00 70.95      GZ00 O
ATOM   2890  OE2  GLU D 164       2.197  44.189   0.869  1.00 56.32      GZ00 O1-
ATOM   2891  N    THR D 165       8.400  45.308  -1.434  1.00 43.26      GZ00 N
ATOM   2892  CA   THR D 165       9.794  45.691  -1.572  1.00 34.34      GZ00 C
ATOM   2893  C    THR D 165      10.043  46.955  -0.778  1.00 37.84      GZ00 C
ATOM   2894  O    THR D 165       9.267  47.903  -0.883  1.00 43.88      GZ00 O
ATOM   2895  CB   THR D 165      10.122  45.903  -3.040  1.00 37.47      GZ00 C
ATOM   2896  OG1  THR D 165       9.909  44.671  -3.730  1.00 39.61      GZ00 O
ATOM   2897  CG2  THR D 165      11.578  46.354  -3.240  1.00 38.05      GZ00 C
ATOM   2898  N    THR D 166      11.131  46.982  -0.011  1.00 37.74      GZ00 N
ATOM   2899  CA   THR D 166      11.464  48.177   0.757  1.00 47.21      GZ00 C
ATOM   2900  C    THR D 166      12.099  49.231  -0.141  1.00 40.41      GZ00 C
ATOM   2901  O    THR D 166      12.548  48.951  -1.256  1.00 41.42      GZ00 O
ATOM   2902  CB   THR D 166      12.470  47.888   1.882  1.00 40.73      GZ00 C
ATOM   2903  OG1  THR D 166      13.677  47.375   1.311  1.00 36.35      GZ00 O
ATOM   2904  CG2  THR D 166      11.922  46.878   2.882  1.00 40.85      GZ00 C
ATOM   2905  N    THR D 167      12.135  50.451   0.365  1.00 42.55      GZ00 N
ATOM   2906  CA   THR D 167      12.976  51.467  -0.237  1.00 49.39      GZ00 C
ATOM   2907  C    THR D 167      14.429  51.231   0.164  1.00 47.70      GZ00 C
ATOM   2908  O    THR D 167      14.702  50.656   1.218  1.00 50.42      GZ00 O
```

| ATOM | 2909 | CB  | THR | D | 167 | 12.564 | 52.846 | 0.231  | 1.00 | 42.03 | GZ00 C   |
|------|------|-----|-----|---|-----|--------|--------|--------|------|-------|----------|
| ATOM | 2910 | OG1 | THR | D | 167 | 12.784 | 52.916 | 1.640  | 1.00 | 55.83 | GZ00 O   |
| ATOM | 2911 | CG2 | THR | D | 167 | 11.133 | 53.079 | -0.052 | 1.00 | 40.08 | GZ00 C   |
| ATOM | 2912 | N   | PRO | D | 168 | 15.383 | 51.666 | -0.647 | 1.00 | 50.17 | GZ00 N   |
| ATOM | 2913 | CA  | PRO | D | 168 | 16.789 | 51.463 | -0.271 | 1.00 | 46.73 | GZ00 C   |
| ATOM | 2914 | C   | PRO | D | 168 | 17.132 | 52.312 | 0.943  | 1.00 | 48.10 | GZ00 C   |
| ATOM | 2915 | O   | PRO | D | 168 | 16.592 | 53.405 | 1.140  | 1.00 | 47.07 | GZ00 O   |
| ATOM | 2916 | CB  | PRO | D | 168 | 17.572 | 51.906 | -1.515 | 1.00 | 44.24 | GZ00 C   |
| ATOM | 2917 | CG  | PRO | D | 168 | 16.579 | 52.648 | -2.378 | 1.00 | 51.68 | GZ00 C   |
| ATOM | 2918 | CD  | PRO | D | 168 | 15.216 | 52.134 | -2.030 | 1.00 | 42.84 | GZ00 C   |
| ATOM | 2919 | N   | SER | D | 169 | 18.006 | 51.773 | 1.789  | 1.00 | 38.56 | GZ00 N   |
| ATOM | 2920 | CA  | SER | D | 169 | 18.460 | 52.490 | 2.976  | 1.00 | 44.06 | GZ00 C   |
| ATOM | 2921 | C   | SER | D | 169 | 19.974 | 52.364 | 3.091  | 1.00 | 40.12 | GZ00 C   |
| ATOM | 2922 | O   | SER | D | 169 | 20.562 | 51.347 | 2.703  | 1.00 | 42.12 | GZ00 O   |
| ATOM | 2923 | CB  | SER | D | 169 | 17.776 | 51.973 | 4.261  | 1.00 | 37.35 | GZ00 C   |
| ATOM | 2924 | OG  | SER | D | 169 | 18.098 | 50.608 | 4.455  | 1.00 | 51.17 | GZ00 O   |
| ATOM | 2925 | N   | LYS | D | 170 | 20.601 | 53.409 | 3.614  | 1.00 | 38.28 | GZ00 N   |
| ATOM | 2926 | CA  | LYS | D | 170 | 22.053 | 53.472 | 3.624  | 1.00 | 42.03 | GZ00 C   |
| ATOM | 2927 | C   | LYS | D | 170 | 22.610 | 52.541 | 4.691  | 1.00 | 41.78 | GZ00 C   |
| ATOM | 2928 | O   | LYS | D | 170 | 22.160 | 52.564 | 5.839  | 1.00 | 44.51 | GZ00 O   |
| ATOM | 2929 | CB  | LYS | D | 170 | 22.542 | 54.897 | 3.877  | 1.00 | 38.33 | GZ00 C   |
| ATOM | 2930 | CG  | LYS | D | 170 | 24.014 | 55.068 | 3.482  | 1.00 | 45.43 | GZ00 C   |
| ATOM | 2931 | CD  | LYS | D | 170 | 24.575 | 56.405 | 3.934  | 1.00 | 48.05 | GZ00 C   |
| ATOM | 2932 | CE  | LYS | D | 170 | 23.962 | 57.548 | 3.176  | 1.00 | 54.50 | GZ00 C   |
| ATOM | 2933 | NZ  | LYS | D | 170 | 24.635 | 58.806 | 3.569  | 1.00 | 69.11 | GZ00 N1+ |
| ATOM | 2934 | N   | GLN | D | 171 | 23.589 | 51.723 | 4.305  | 1.00 | 41.93 | GZ00 N   |
| ATOM | 2935 | CA  | GLN | D | 171 | 24.346 | 50.889 | 5.228  | 1.00 | 47.20 | GZ00 C   |
| ATOM | 2936 | C   | GLN | D | 171 | 25.483 | 51.692 | 5.858  | 1.00 | 45.26 | GZ00 C   |
| ATOM | 2937 | O   | GLN | D | 171 | 25.816 | 52.797 | 5.422  | 1.00 | 47.74 | GZ00 O   |
| ATOM | 2938 | CB  | GLN | D | 171 | 24.933 | 49.672 | 4.508  | 1.00 | 38.79 | GZ00 C   |
| ATOM | 2939 | CG  | GLN | D | 171 | 23.914 | 48.809 | 3.823  | 1.00 | 38.60 | GZ00 C   |
| ATOM | 2940 | CD  | GLN | D | 171 | 24.560 | 47.787 | 2.932  | 1.00 | 45.79 | GZ00 C   |
| ATOM | 2941 | OE1 | GLN | D | 171 | 23.900 | 46.859 | 2.433  | 1.00 | 43.80 | GZ00 O   |
| ATOM | 2942 | NE2 | GLN | D | 171 | 25.868 | 47.942 | 2.715  | 1.00 | 45.31 | GZ00 N   |
| ATOM | 2943 | N   | SER | D | 172 | 26.118 | 51.089 | 6.865  | 1.00 | 49.48 | GZ00 N   |
| ATOM | 2944 | CA  | SER | D | 172 | 27.258 | 51.724 | 7.529  | 1.00 | 45.76 | GZ00 C   |
| ATOM | 2945 | C   | SER | D | 172 | 28.391 | 52.012 | 6.557  | 1.00 | 43.14 | GZ00 C   |
| ATOM | 2946 | O   | SER | D | 172 | 29.073 | 53.033 | 6.675  | 1.00 | 51.69 | GZ00 O   |
| ATOM | 2947 | CB  | SER | D | 172 | 27.769 | 50.826 | 8.658  | 1.00 | 55.56 | GZ00 C   |
| ATOM | 2948 | OG  | SER | D | 172 | 26.906 | 50.898 | 9.777  | 1.00 | 71.74 | GZ00 O   |
| ATOM | 2949 | N   | ASN | D | 173 | 28.628 | 51.122 | 5.601  | 1.00 | 40.90 | GZ00 N   |
| ATOM | 2950 | CA  | ASN | D | 173 | 29.697 | 51.363 | 4.646  | 1.00 | 37.43 | GZ00 C   |
| ATOM | 2951 | C   | ASN | D | 173 | 29.304 | 52.349 | 3.542  | 1.00 | 44.12 | GZ00 C   |
| ATOM | 2952 | O   | ASN | D | 173 | 30.080 | 52.526 | 2.588  | 1.00 | 41.37 | GZ00 O   |
| ATOM | 2953 | CB  | ASN | D | 173 | 30.171 | 50.030 | 4.038  | 1.00 | 41.28 | GZ00 C   |
| ATOM | 2954 | CG  | ASN | D | 173 | 29.139 | 49.370 | 3.147  | 1.00 | 44.26 | GZ00 C   |
| ATOM | 2955 | OD1 | ASN | D | 173 | 28.038 | 49.887 | 2.944  | 1.00 | 43.64 | GZ00 O   |
| ATOM | 2956 | ND2 | ASN | D | 173 | 29.500 | 48.208 | 2.597  | 1.00 | 50.50 | GZ00 N   |
| ATOM | 2957 | N   | ASN | D | 174 | 28.142 | 52.999 | 3.669  | 1.00 | 41.55 | GZ00 N   |
| ATOM | 2958 | CA  | ASN | D | 174 | 27.608 | 54.005 | 2.759  | 1.00 | 44.88 | GZ00 C   |
| ATOM | 2959 | C   | ASN | D | 174 | 27.151 | 53.429 | 1.426  | 1.00 | 44.23 | GZ00 C   |
| ATOM | 2960 | O   | ASN | D | 174 | 26.811 | 54.200 | 0.525  | 1.00 | 39.27 | GZ00 O   |
| ATOM | 2961 | CB  | ASN | D | 174 | 28.610 | 55.139 | 2.484  | 1.00 | 42.38 | GZ00 C   |
| ATOM | 2962 | CG  | ASN | D | 174 | 28.741 | 56.082 | 3.653  | 1.00 | 45.60 | GZ00 C   |
| ATOM | 2963 | OD1 | ASN | D | 174 | 27.827 | 56.220 | 4.457  | 1.00 | 52.29 | GZ00 O   |
| ATOM | 2964 | ND2 | ASN | D | 174 | 29.895 | 56.706 | 3.775  | 1.00 | 48.22 | GZ00 N   |
| ATOM | 2965 | N   | LYS | D | 175 | 27.187 | 52.114 | 1.242  | 1.00 | 43.55 | GZ00 N   |
| ATOM | 2966 | CA  | LYS | D | 175 | 26.380 | 51.487 | 0.204  | 1.00 | 46.60 | GZ00 C   |
| ATOM | 2967 | C   | LYS | D | 175 | 24.958 | 51.250 | 0.748  | 1.00 | 40.26 | GZ00 C   |
| ATOM | 2968 | O   | LYS | D | 175 | 24.628 | 51.624 | 1.877  | 1.00 | 36.35 | GZ00 O   |
| ATOM | 2969 | CB  | LYS | D | 175 | 27.054 | 50.204 | -0.272 | 1.00 | 43.68 | GZ00 C   |
| ATOM | 2970 | CG  | LYS | D | 175 | 28.501 | 50.379 | -0.772 | 1.00 | 42.53 | GZ00 C   |
| ATOM | 2971 | CD  | LYS | D | 175 | 29.043 | 48.983 | -1.135 | 1.00 | 47.00 | GZ00 C   |
| ATOM | 2972 | CE  | LYS | D | 175 | 30.522 | 48.940 | -1.403 | 1.00 | 54.60 | GZ00 C   |
| ATOM | 2973 | NZ  | LYS | D | 175 | 30.884 | 47.568 | -1.857 | 1.00 | 65.92 | GZ00 N   |
| ATOM | 2974 | N   | TYR | D | 176 | 24.091 | 50.623 | -0.042 | 1.00 | 37.23 | GZ00 N   |
| ATOM | 2975 | CA  | TYR | D | 176 | 22.676 | 50.583 | 0.302  | 1.00 | 36.34 | GZ00 C   |
| ATOM | 2976 | C   | TYR | D | 176 | 22.145 | 49.158 | 0.349  | 1.00 | 42.73 | GZ00 C   |
| ATOM | 2977 | O   | TYR | D | 176 | 22.657 | 48.244 | -0.314 | 1.00 | 36.34 | GZ00 O   |
| ATOM | 2978 | CB  | TYR | D | 176 | 21.844 | 51.409 | -0.693 | 1.00 | 36.80 | GZ00 C   |
| ATOM | 2979 | CG  | TYR | D | 176 | 22.044 | 52.899 | -0.563 | 1.00 | 36.36 | GZ00 C   |

```
ATOM   2980  CD2 TYR D 176      21.057  53.710  -0.021  1.00 37.31      GZ00 C
ATOM   2981  CD1 TYR D 176      23.222  53.497  -0.996  1.00 41.41      GZ00 C
ATOM   2982  CE2 TYR D 176      21.252  55.072   0.098  1.00 40.59      GZ00 C
ATOM   2983  CE1 TYR D 176      23.423  54.857  -0.883  1.00 39.52      GZ00 C
ATOM   2984  CZ  TYR D 176      22.440  55.639  -0.347  1.00 39.72      GZ00 C
ATOM   2985  OH  TYR D 176      22.662  56.981  -0.238  1.00 49.13      GZ00 O
ATOM   2986  N   ALA D 177      21.056  49.003   1.103  1.00 41.16      GZ00 N
ATOM   2987  CA  ALA D 177      20.371  47.728   1.246  1.00 39.60      GZ00 C
ATOM   2988  C   ALA D 177      18.886  47.901   0.957  1.00 37.33      GZ00 C
ATOM   2989  O   ALA D 177      18.321  48.987   1.140  1.00 41.92      GZ00 O
ATOM   2990  CB  ALA D 177      20.552  47.133   2.665  1.00 35.41      GZ00 C
ATOM   2991  N   ALA D 178      18.271  46.809   0.488  1.00 36.97      GZ00 N
ATOM   2992  CA  ALA D 178      16.829  46.711   0.308  1.00 39.14      GZ00 C
ATOM   2993  C   ALA D 178      16.415  45.246   0.381  1.00 41.15      GZ00 C
ATOM   2994  O   ALA D 178      17.230  44.339   0.192  1.00 38.19      GZ00 O
ATOM   2995  CB  ALA D 178      16.380  47.324  -1.023  1.00 31.46      GZ00 C
ATOM   2996  N   SER D 179      15.125  45.012   0.628  1.00 39.23      GZ00 N
ATOM   2997  CA  SER D 179      14.626  43.644   0.607  1.00 42.89      GZ00 C
ATOM   2998  C   SER D 179      13.279  43.569  -0.100  1.00 42.30      GZ00 C
ATOM   2999  O   SER D 179      12.526  44.543  -0.157  1.00 39.32      GZ00 O
ATOM   3000  CB  SER D 179      14.561  43.043   2.026  1.00 37.57      GZ00 C
ATOM   3001  OG  SER D 179      14.059  43.953   2.960  1.00 39.68      GZ00 O
ATOM   3002  N   SER D 180      13.012  42.399  -0.673  1.00 39.99      GZ00 N
ATOM   3003  CA  SER D 180      11.786  42.133  -1.407  1.00 45.51      GZ00 C
ATOM   3004  C   SER D 180      11.212  40.781  -0.972  1.00 51.71      GZ00 C
ATOM   3005  O   SER D 180      11.932  39.774  -0.921  1.00 43.37      GZ00 O
ATOM   3006  CB  SER D 180      12.062  42.153  -2.911  1.00 42.12      GZ00 C
ATOM   3007  OG  SER D 180      10.869  42.076  -3.655  1.00 40.91      GZ00 O
ATOM   3008  N   TYR D 181       9.914  40.756  -0.686  1.00 44.23      GZ00 N
ATOM   3009  CA  TYR D 181       9.237  39.561  -0.206  1.00 45.60      GZ00 C
ATOM   3010  C   TYR D 181       8.159  39.158  -1.203  1.00 48.02      GZ00 C
ATOM   3011  O   TYR D 181       7.369  40.001  -1.637  1.00 44.19      GZ00 O
ATOM   3012  CB  TYR D 181       8.612  39.804   1.164  1.00 39.73      GZ00 C
ATOM   3013  CG  TYR D 181       9.601  40.149   2.246  1.00 42.39      GZ00 C
ATOM   3014  CD2 TYR D 181      10.094  39.167   3.100  1.00 35.39      GZ00 C
ATOM   3015  CD1 TYR D 181      10.050  41.464   2.416  1.00 38.95      GZ00 C
ATOM   3016  CE2 TYR D 181      11.005  39.477   4.098  1.00 38.77      GZ00 C
ATOM   3017  CE1 TYR D 181      10.958  41.786   3.414  1.00 42.49      GZ00 C
ATOM   3018  CZ  TYR D 181      11.437  40.785   4.254  1.00 40.93      GZ00 C
ATOM   3019  OH  TYR D 181      12.335  41.092   5.250  1.00 36.93      GZ00 O
ATOM   3020  N   LEU D 182       8.137  37.878  -1.576  1.00 39.63      GZ00 N
ATOM   3021  CA  LEU D 182       7.075  37.312  -2.399  1.00 48.32      GZ00 C
ATOM   3022  C   LEU D 182       6.258  36.347  -1.533  1.00 49.68      GZ00 C
ATOM   3023  O   LEU D 182       6.774  35.321  -1.072  1.00 44.59      GZ00 O
ATOM   3024  CB  LEU D 182       7.672  36.616  -3.621  1.00 45.93      GZ00 C
ATOM   3025  CG  LEU D 182       6.711  35.859  -4.540  1.00 54.86      GZ00 C
ATOM   3026  CD1 LEU D 182       5.643  36.822  -5.050  1.00 49.90      GZ00 C
ATOM   3027  CD2 LEU D 182       7.467  35.191  -5.703  1.00 43.02      GZ00 C
ATOM   3028  N   SER D 183       4.992  36.680  -1.295  1.00 52.23      GZ00 N
ATOM   3029  CA  SER D 183       4.099  35.806  -0.538  1.00 47.98      GZ00 C
ATOM   3030  C   SER D 183       3.397  34.840  -1.481  1.00 47.04      GZ00 C
ATOM   3031  O   SER D 183       2.956  35.225  -2.564  1.00 52.72      GZ00 O
ATOM   3032  CB  SER D 183       3.072  36.608   0.251  1.00 45.88      GZ00 C
ATOM   3033  OG  SER D 183       3.720  37.533   1.102  1.00 53.76      GZ00 O
ATOM   3034  N   LEU D 184       3.356  33.574  -1.088  1.00 51.61      GZ00 N
ATOM   3035  CA  LEU D 184       2.741  32.523  -1.874  1.00 51.65      GZ00 C
ATOM   3036  C   LEU D 184       1.951  31.621  -0.944  1.00 60.30      GZ00 C
ATOM   3037  O   LEU D 184       2.089  31.679   0.284  1.00 57.40      GZ00 O
ATOM   3038  CB  LEU D 184       3.777  31.687  -2.623  1.00 53.86      GZ00 C
ATOM   3039  CG  LEU D 184       4.672  32.341  -3.654  1.00 51.77      GZ00 C
ATOM   3040  CD1 LEU D 184       5.638  31.317  -4.213  1.00 50.48      GZ00 C
ATOM   3041  CD2 LEU D 184       3.803  32.927  -4.738  1.00 58.87      GZ00 C
ATOM   3042  N   THR D 185       1.097  30.745  -1.569  1.00 63.83      GZ00 N
ATOM   3043  CA  THR D 185       0.577  29.598  -0.843  1.00 63.50      GZ00 C
ATOM   3044  C   THR D 185       1.550  28.438  -0.977  1.00 60.27      GZ00 C
ATOM   3045  O   THR D 185       2.270  28.332  -1.982  1.00 54.12      GZ00 O
ATOM   3046  CB  THR D 185      -0.782  29.160  -1.397  1.00 58.04      GZ00 C
ATOM   3047  OG1 THR D 185      -0.611  28.645  -2.727  1.00 62.01      GZ00 O
ATOM   3048  CG2 THR D 185      -1.765  30.324  -1.417  1.00 49.17      GZ00 C
ATOM   3049  N   PRO D 186       1.567  27.545   0.008  1.00 56.65      GZ00 N
ATOM   3050  CA  PRO D 186       2.337  26.307  -0.160  1.00 61.35      GZ00 C
```

```
ATOM   3051  C    PRO D 186      2.054  25.617  -1.484  1.00 70.92      GZ00 C
ATOM   3052  O    PRO D 186      2.972  25.043  -2.089  1.00 71.62      GZ00 O
ATOM   3053  CB   PRO D 186      1.893  25.474   1.047  1.00 64.94      GZ00 C
ATOM   3054  CG   PRO D 186      1.565  26.502   2.095  1.00 56.23      GZ00 C
ATOM   3055  CD   PRO D 186      0.970  27.657   1.353  1.00 57.72      GZ00 C
ATOM   3056  N    GLU D 187      0.815  25.725  -1.981  1.00 72.13      GZ00 N
ATOM   3057  CA   GLU D 187      0.440  25.117  -3.255  1.00 73.38      GZ00 C
ATOM   3058  C    GLU D 187      1.182  25.782  -4.408  1.00 71.22      GZ00 C
ATOM   3059  O    GLU D 187      1.817  25.105  -5.229  1.00 67.47      GZ00 O
ATOM   3060  CB   GLU D 187     -1.074  25.235  -3.473  1.00 72.72      GZ00 C
ATOM   3061  CG   GLU D 187     -1.909  25.465  -2.206  1.00 73.60      GZ00 C
ATOM   3062  CD   GLU D 187     -1.655  24.438  -1.114  1.00 91.17      GZ00 C
ATOM   3063  OE1  GLU D 187     -1.492  23.239  -1.442  1.00 95.11      GZ00 O
ATOM   3064  OE2  GLU D 187     -1.608  24.842   0.072  1.00 86.41      GZ00 O1-
ATOM   3065  N    GLN D 188      1.080  27.116  -4.499  1.00 63.92      GZ00 N
ATOM   3066  CA   GLN D 188      1.803  27.852  -5.532  1.00 67.27      GZ00 C
ATOM   3067  C    GLN D 188      3.302  27.589  -5.466  1.00 68.44      GZ00 C
ATOM   3068  O    GLN D 188      3.959  27.448  -6.507  1.00 68.93      GZ00 O
ATOM   3069  CB   GLN D 188      1.524  29.347  -5.411  1.00 69.32      GZ00 C
ATOM   3070  CG   GLN D 188      0.092  29.737  -5.677  1.00 64.52      GZ00 C
ATOM   3071  CD   GLN D 188     -0.225  31.124  -5.174  1.00 74.32      GZ00 C
ATOM   3072  OE1  GLN D 188      0.362  31.585  -4.199  1.00 75.61      GZ00 O
ATOM   3073  NE2  GLN D 188     -1.147  31.808  -5.847  1.00 79.67      GZ00 N
ATOM   3074  N    TRP D 189      3.865  27.522  -4.255  1.00 65.05      GZ00 N
ATOM   3075  CA   TRP D 189      5.304  27.301  -4.130  1.00 65.52      GZ00 C
ATOM   3076  C    TRP D 189      5.697  25.960  -4.734  1.00 70.69      GZ00 C
ATOM   3077  O    TRP D 189      6.669  25.871  -5.497  1.00 69.56      GZ00 O
ATOM   3078  CB   TRP D 189      5.730  27.397  -2.659  1.00 64.52      GZ00 C
ATOM   3079  CG   TRP D 189      7.127  26.879  -2.336  1.00 69.67      GZ00 C
ATOM   3080  CD1  TRP D 189      7.448  25.892  -1.442  1.00 67.82      GZ00 C
ATOM   3081  CD2  TRP D 189      8.372  27.335  -2.885  1.00 66.64      GZ00 C
ATOM   3082  NE1  TRP D 189      8.806  25.699  -1.414  1.00 62.01      GZ00 N
ATOM   3083  CE2  TRP D 189      9.399  26.569  -2.287  1.00 64.99      GZ00 C
ATOM   3084  CE3  TRP D 189      8.720  28.305  -3.833  1.00 68.36      GZ00 C
ATOM   3085  CZ2  TRP D 189     10.750  26.753  -2.592  1.00 66.69      GZ00 C
ATOM   3086  CZ3  TRP D 189     10.067  28.481  -4.146  1.00 68.79      GZ00 C
ATOM   3087  CH2  TRP D 189     11.064  27.704  -3.527  1.00 63.38      GZ00 C
ATOM   3088  N    LYS D 190      4.925  24.909  -4.437  1.00 74.93      GZ00 N
ATOM   3089  CA   LYS D 190      5.269  23.562  -4.875  1.00 74.61      GZ00 C
ATOM   3090  C    LYS D 190      4.919  23.317  -6.334  1.00 72.06      GZ00 C
ATOM   3091  O    LYS D 190      5.471  22.395  -6.946  1.00 76.50      GZ00 O
ATOM   3092  CB   LYS D 190      4.556  22.538  -3.988  1.00 72.08      GZ00 C
ATOM   3093  CG   LYS D 190      5.012  22.585  -2.539  1.00 73.58      GZ00 C
ATOM   3094  CD   LYS D 190      4.154  21.723  -1.635  1.00 86.29      GZ00 C
ATOM   3095  CE   LYS D 190      4.625  21.833  -0.191  1.00 92.57      GZ00 C
ATOM   3096  NZ   LYS D 190      3.732  21.118   0.762  1.00 99.72      GZ00 N1+
ATOM   3097  N    SER D 191      4.050  24.142  -6.915  1.00 68.71      GZ00 N
ATOM   3098  CA   SER D 191      3.582  23.882  -8.272  1.00 73.46      GZ00 C
ATOM   3099  C    SER D 191      4.650  24.213  -9.315  1.00 72.57      GZ00 C
ATOM   3100  O    SER D 191      4.878  23.431 -10.244  1.00 79.90      GZ00 O
ATOM   3101  CB   SER D 191      2.289  24.655  -8.529  1.00 70.09      GZ00 C
ATOM   3102  OG   SER D 191      2.531  26.044  -8.539  1.00 81.55      GZ00 O
ATOM   3103  N    HIS D 192      5.313  25.363  -9.190  1.00 72.67      GZ00 N
ATOM   3104  CA   HIS D 192      6.267  25.792 -10.210  1.00 69.13      GZ00 C
ATOM   3105  C    HIS D 192      7.641  25.135 -10.038  1.00 66.99      GZ00 C
ATOM   3106  O    HIS D 192      8.020  24.695  -8.951  1.00 72.31      GZ00 O
ATOM   3107  CB   HIS D 192      6.420  27.313 -10.199  1.00 72.85      GZ00 C
ATOM   3108  CG   HIS D 192      5.178  28.049 -10.597  1.00 71.64      GZ00 C
ATOM   3109  ND1  HIS D 192      4.172  28.355  -9.704  1.00 70.93      GZ00 N
ATOM   3110  CD2  HIS D 192      4.763  28.504 -11.803  1.00 68.93      GZ00 C
ATOM   3111  CE1  HIS D 192      3.202  28.990 -10.338  1.00 65.00      GZ00 C
ATOM   3112  NE2  HIS D 192      3.534  29.089 -11.614  1.00 73.86      GZ00 N
ATOM   3113  N    ARG D 193      8.390  25.070 -11.148  1.00 64.89      GZ00 N
ATOM   3114  CA   ARG D 193      9.731  24.476 -11.135  1.00 69.27      GZ00 C
ATOM   3115  C    ARG D 193     10.705  25.313 -10.313  1.00 67.85      GZ00 C
ATOM   3116  O    ARG D 193     11.542  24.769  -9.586  1.00 68.86      GZ00 O
ATOM   3117  CB   ARG D 193     10.269  24.354 -12.565  1.00 71.73      GZ00 C
ATOM   3118  CG   ARG D 193      9.414  23.557 -13.540  1.00 81.33      GZ00 C
ATOM   3119  CD   ARG D 193      9.846  23.822 -14.993  1.00 88.10      GZ00 C
ATOM   3120  NE   ARG D 193      8.722  24.419 -15.728  1.00 99.94      GZ00 N
ATOM   3121  CZ   ARG D 193      8.814  25.092 -16.875  1.00101.58      GZ00 C
```

```
ATOM   3122  NH1 ARG D 193      7.716  25.589 -17.441  1.00 88.37       GZ00 N1+
ATOM   3123  NH2 ARG D 193      9.997  25.305 -17.440  1.00 99.51       GZ00 N
ATOM   3124  N   SER D 194     10.619  26.637 -10.428  1.00 68.05       GZ00 N
ATOM   3125  CA  SER D 194     11.553  27.534  -9.768  1.00 62.46       GZ00 C
ATOM   3126  C   SER D 194     10.936  28.917  -9.658  1.00 66.77       GZ00 C
ATOM   3127  O   SER D 194      9.957  29.242 -10.334  1.00 71.03       GZ00 O
ATOM   3128  CB  SER D 194     12.876  27.622 -10.529  1.00 66.04       GZ00 C
ATOM   3129  OG  SER D 194     12.739  28.441 -11.681  1.00 66.32       GZ00 O
ATOM   3130  N   TYR D 195     11.520  29.722  -8.782  1.00 64.92       GZ00 N
ATOM   3131  CA  TYR D 195     11.219  31.142  -8.688  1.00 63.56       GZ00 C
ATOM   3132  C   TYR D 195     12.522  31.927  -8.748  1.00 61.50       GZ00 C
ATOM   3133  O   TYR D 195     13.580  31.431  -8.351  1.00 54.93       GZ00 O
ATOM   3134  CB  TYR D 195     10.481  31.482  -7.408  1.00 57.26       GZ00 C
ATOM   3135  CG  TYR D 195      9.037  31.058  -7.392  1.00 64.67       GZ00 C
ATOM   3136  CD1 TYR D 195      8.679  29.742  -7.126  1.00 69.64       GZ00 C
ATOM   3137  CD2 TYR D 195      8.025  31.986  -7.618  1.00 66.11       GZ00 C
ATOM   3138  CE1 TYR D 195      7.350  29.362  -7.094  1.00 72.89       GZ00 C
ATOM   3139  CE2 TYR D 195      6.704  31.621  -7.592  1.00 63.75       GZ00 C
ATOM   3140  CZ  TYR D 195      6.369  30.311  -7.336  1.00 69.62       GZ00 C
ATOM   3141  OH  TYR D 195      5.043  29.958  -7.309  1.00 71.41       GZ00 O
ATOM   3142  N   SER D 196     12.441  33.161  -9.235  1.00 58.30       GZ00 N
ATOM   3143  CA  SER D 196     13.627  33.990  -9.396  1.00 54.75       GZ00 C
ATOM   3144  C   SER D 196     13.387  35.383  -8.852  1.00 52.46       GZ00 C
ATOM   3145  O   SER D 196     12.282  35.924  -8.944  1.00 50.50       GZ00 O
ATOM   3146  CB  SER D 196     14.068  34.102 -10.856  1.00 55.06       GZ00 C
ATOM   3147  OG  SER D 196     14.543  32.862 -11.337  1.00 59.56       GZ00 O
ATOM   3148  N   CYS D 197     14.434  35.941  -8.263  1.00 51.88       GZ00 N
ATOM   3149  CA  CYS D 197     14.485  37.334  -7.862  1.00 48.95       GZ00 C
ATOM   3150  C   CYS D 197     15.499  38.031  -8.771  1.00 49.99       GZ00 C
ATOM   3151  O   CYS D 197     16.667  37.641  -8.809  1.00 46.06       GZ00 O
ATOM   3152  CB  CYS D 197     14.868  37.443  -6.389  1.00 50.65       GZ00 C
ATOM   3153  SG  CYS D 197     14.970  39.121  -5.807  1.00 59.52       GZ00 S
ATOM   3154  N   GLN D 198     15.048  39.021  -9.538  1.00 50.95       GZ00 N
ATOM   3155  CA  GLN D 198     15.912  39.731 -10.476  1.00 45.62       GZ00 C
ATOM   3156  C   GLN D 198     16.058  41.165 -10.006  1.00 45.99       GZ00 C
ATOM   3157  O   GLN D 198     15.064  41.888  -9.860  1.00 43.74       GZ00 O
ATOM   3158  CB  GLN D 198     15.372  39.667 -11.901  1.00 43.17       GZ00 C
ATOM   3159  CG  GLN D 198     15.534  38.283 -12.527  1.00 59.20       GZ00 C
ATOM   3160  CD  GLN D 198     14.770  38.107 -13.837  1.00 67.77       GZ00 C
ATOM   3161  OE1 GLN D 198     14.034  38.996 -14.273  1.00 68.74       GZ00 O
ATOM   3162  NE2 GLN D 198     14.933  36.945 -14.460  1.00 69.92       GZ00 N
ATOM   3163  N   VAL D 199     17.291  41.556  -9.722  1.00 43.86       GZ00 N
ATOM   3164  CA  VAL D 199     17.600  42.869  -9.177  1.00 43.86       GZ00 C
ATOM   3165  C   VAL D 199     18.389  43.626 -10.233  1.00 44.40       GZ00 C
ATOM   3166  O   VAL D 199     19.480  43.197 -10.619  1.00 41.88       GZ00 O
ATOM   3167  CB  VAL D 199     18.389  42.750  -7.865  1.00 40.13       GZ00 C
ATOM   3168  CG1 VAL D 199     18.560  44.088  -7.241  1.00 33.53       GZ00 C
ATOM   3169  CG2 VAL D 199     17.679  41.778  -6.906  1.00 37.93       GZ00 C
ATOM   3170  N   THR D 200     17.838  44.732 -10.719  1.00 43.84       GZ00 N
ATOM   3171  CA  THR D 200     18.513  45.558 -11.712  1.00 42.52       GZ00 C
ATOM   3172  C   THR D 200     19.125  46.783 -11.044  1.00 39.89       GZ00 C
ATOM   3173  O   THR D 200     18.442  47.506 -10.310  1.00 39.87       GZ00 O
ATOM   3174  CB  THR D 200     17.542  45.987 -12.813  1.00 48.84       GZ00 C
ATOM   3175  OG1 THR D 200     16.958  44.805 -13.380  1.00 46.64       GZ00 O
ATOM   3176  CG2 THR D 200     18.290  46.806 -13.918  1.00 33.12       GZ00 C
ATOM   3177  N   HIS D 201     20.406  47.016 -11.313  1.00 40.27       GZ00 N
ATOM   3178  CA  HIS D 201     21.145  48.125 -10.723  1.00 39.47       GZ00 C
ATOM   3179  C   HIS D 201     22.047  48.723 -11.790  1.00 44.85       GZ00 C
ATOM   3180  O   HIS D 201     22.892  48.013 -12.345  1.00 39.41       GZ00 O
ATOM   3181  CB  HIS D 201     21.973  47.660  -9.532  1.00 37.22       GZ00 C
ATOM   3182  CG  HIS D 201     22.797  48.738  -8.905  1.00 34.72       GZ00 C
ATOM   3183  ND1 HIS D 201     24.134  48.915  -9.190  1.00 33.61       GZ00 N
ATOM   3184  CD2 HIS D 201     22.486  49.658  -7.960  1.00 33.33       GZ00 C
ATOM   3185  CE1 HIS D 201     24.608  49.907  -8.455  1.00 40.99       GZ00 C
ATOM   3186  NE2 HIS D 201     23.628  50.377  -7.699  1.00 33.68       GZ00 N
ATOM   3187  N   GLU D 202     21.840  50.010 -12.100  1.00 47.82       GZ00 N
ATOM   3188  CA  GLU D 202     22.672  50.726 -13.064  1.00 45.90       GZ00 C
ATOM   3189  C   GLU D 202     22.753  49.963 -14.385  1.00 44.77       GZ00 C
ATOM   3190  O   GLU D 202     23.828  49.757 -14.947  1.00 45.87       GZ00 O
ATOM   3191  CB  GLU D 202     24.068  50.973 -12.485  1.00 40.73       GZ00 C
ATOM   3192  CG  GLU D 202     24.078  51.833 -11.241  1.00 37.51       GZ00 C
```

| ATOM | 3193 | CD | GLU | D | 202 | 23.780 | 53.301 | -11.543 | 1.00 | 53.62 | GZ00 | C |
| ATOM | 3194 | OE1 | GLU | D | 202 | 24.472 | 53.880 | -12.414 | 1.00 | 54.80 | GZ00 | O |
| ATOM | 3195 | OE2 | GLU | D | 202 | 22.853 | 53.878 | -10.924 | 1.00 | 56.28 | GZ00 | O1- |
| ATOM | 3196 | N | GLY | D | 203 | 21.600 | 49.498 | -14.862 | 1.00 | 40.18 | GZ00 | N |
| ATOM | 3197 | CA | GLY | D | 203 | 21.584 | 48.775 | -16.117 | 1.00 | 41.95 | GZ00 | C |
| ATOM | 3198 | C | GLY | D | 203 | 22.137 | 47.357 | -16.110 | 1.00 | 51.05 | GZ00 | C |
| ATOM | 3199 | O | GLY | D | 203 | 22.259 | 46.760 | -17.186 | 1.00 | 50.18 | GZ00 | O |
| ATOM | 3200 | N | SER | D | 206 | 22.505 | 46.793 | -14.958 | 1.00 | 47.41 | GZ00 | N |
| ATOM | 3201 | CA | SER | D | 206 | 22.921 | 45.390 | -14.906 | 1.00 | 50.13 | GZ00 | C |
| ATOM | 3202 | C | SER | D | 206 | 22.101 | 44.640 | -13.874 | 1.00 | 46.73 | GZ00 | C |
| ATOM | 3203 | O | SER | D | 206 | 21.921 | 45.117 | -12.747 | 1.00 | 45.75 | GZ00 | O |
| ATOM | 3204 | CB | SER | D | 206 | 24.404 | 45.243 | -14.607 | 1.00 | 46.12 | GZ00 | C |
| ATOM | 3205 | OG | SER | D | 206 | 25.155 | 45.750 | -15.688 | 1.00 | 57.53 | GZ00 | O |
| ATOM | 3206 | N | THR | D | 207 | 21.592 | 43.477 | -14.262 | 1.00 | 41.69 | GZ00 | N |
| ATOM | 3207 | CA | THR | D | 207 | 20.697 | 42.720 | -13.400 | 1.00 | 50.12 | GZ00 | C |
| ATOM | 3208 | C | THR | D | 207 | 21.425 | 41.525 | -12.797 | 1.00 | 47.45 | GZ00 | C |
| ATOM | 3209 | O | THR | D | 207 | 22.129 | 40.793 | -13.501 | 1.00 | 43.02 | GZ00 | O |
| ATOM | 3210 | CB | THR | D | 207 | 19.449 | 42.249 | -14.155 | 1.00 | 49.49 | GZ00 | C |
| ATOM | 3211 | OG1 | THR | D | 207 | 19.797 | 41.130 | -14.968 | 1.00 | 59.30 | GZ00 | O |
| ATOM | 3212 | CG2 | THR | D | 207 | 18.885 | 43.358 | -15.031 | 1.00 | 37.86 | GZ00 | C |
| ATOM | 3213 | N | VAL | D | 208 | 21.234 | 41.328 | -11.495 | 1.00 | 45.06 | GZ00 | N |
| ATOM | 3214 | CA | VAL | D | 208 | 21.724 | 40.163 | -10.767 | 1.00 | 49.03 | GZ00 | C |
| ATOM | 3215 | C | VAL | D | 208 | 20.514 | 39.301 | -10.447 | 1.00 | 44.06 | GZ00 | C |
| ATOM | 3216 | O | VAL | D | 208 | 19.493 | 39.815 | -9.974 | 1.00 | 50.95 | GZ00 | O |
| ATOM | 3217 | CB | VAL | D | 208 | 22.467 | 40.579 | -9.484 | 1.00 | 48.97 | GZ00 | C |
| ATOM | 3218 | CG1 | VAL | D | 208 | 22.858 | 39.372 | -8.668 | 1.00 | 41.12 | GZ00 | C |
| ATOM | 3219 | CG2 | VAL | D | 208 | 23.700 | 41.376 | -9.829 | 1.00 | 39.61 | GZ00 | C |
| ATOM | 3220 | N | GLU | D | 209 | 20.614 | 38.005 | -10.709 | 1.00 | 41.82 | GZ00 | N |
| ATOM | 3221 | CA | GLU | D | 209 | 19.483 | 37.103 | -10.538 | 1.00 | 43.40 | GZ00 | C |
| ATOM | 3222 | C | GLU | D | 209 | 19.835 | 35.970 | -9.586 | 1.00 | 50.61 | GZ00 | C |
| ATOM | 3223 | O | GLU | D | 209 | 20.917 | 35.383 | -9.674 | 1.00 | 57.28 | GZ00 | O |
| ATOM | 3224 | CB | GLU | D | 209 | 19.042 | 36.508 | -11.873 | 1.00 | 49.58 | GZ00 | C |
| ATOM | 3225 | CG | GLU | D | 209 | 17.851 | 35.561 | -11.761 | 1.00 | 62.21 | GZ00 | C |
| ATOM | 3226 | CD | GLU | D | 209 | 17.419 | 34.999 | -13.108 | 1.00 | 75.52 | GZ00 | C |
| ATOM | 3227 | OE1 | GLU | D | 209 | 17.297 | 33.759 | -13.240 | 1.00 | 85.24 | GZ00 | O |
| ATOM | 3228 | OE2 | GLU | D | 209 | 17.155 | 35.806 | -14.026 | 1.00 | 77.80 | GZ00 | O1- |
| ATOM | 3229 | N | LYS | D | 210 | 18.902 | 35.636 | -8.705 | 1.00 | 48.46 | GZ00 | N |
| ATOM | 3230 | CA | LYS | D | 210 | 19.006 | 34.443 | -7.885 | 1.00 | 52.58 | GZ00 | C |
| ATOM | 3231 | C | LYS | D | 210 | 17.741 | 33.630 | -8.066 | 1.00 | 49.39 | GZ00 | C |
| ATOM | 3232 | O | LYS | D | 210 | 16.639 | 34.182 | -8.150 | 1.00 | 51.83 | GZ00 | O |
| ATOM | 3233 | CB | LYS | D | 210 | 19.228 | 34.785 | -6.401 | 1.00 | 50.40 | GZ00 | C |
| ATOM | 3234 | CG | LYS | D | 210 | 20.608 | 35.341 | -6.116 | 1.00 | 46.93 | GZ00 | C |
| ATOM | 3235 | CD | LYS | D | 210 | 21.675 | 34.454 | -6.726 | 1.00 | 45.41 | GZ00 | C |
| ATOM | 3236 | CE | LYS | D | 210 | 23.095 | 35.008 | -6.476 | 1.00 | 52.18 | GZ00 | C |
| ATOM | 3237 | NZ | LYS | D | 210 | 23.541 | 34.804 | -5.069 | 1.00 | 57.98 | GZ00 | N1+ |
| ATOM | 3238 | N | THR | D | 211 | 17.900 | 32.324 | -8.168 | 1.00 | 47.49 | GZ00 | N |
| ATOM | 3239 | CA | THR | D | 211 | 16.750 | 31.460 | -8.337 | 1.00 | 56.28 | GZ00 | C |
| ATOM | 3240 | C | THR | D | 211 | 16.772 | 30.374 | -7.276 | 1.00 | 53.77 | GZ00 | C |
| ATOM | 3241 | O | THR | D | 211 | 17.832 | 29.917 | -6.845 | 1.00 | 60.67 | GZ00 | O |
| ATOM | 3242 | CB | THR | D | 211 | 16.708 | 30.815 | -9.722 | 1.00 | 53.88 | GZ00 | C |
| ATOM | 3243 | OG1 | THR | D | 211 | 17.474 | 29.617 | -9.690 | 1.00 | 68.57 | GZ00 | O |
| ATOM | 3244 | CG2 | THR | D | 211 | 17.315 | 31.729 | -10.774 | 1.00 | 54.97 | GZ00 | C |
| ATOM | 3245 | N | VAL | D | 212 | 15.583 | 29.943 | -6.893 | 1.00 | 54.81 | GZ00 | N |
| ATOM | 3246 | CA | VAL | D | 212 | 15.393 | 28.942 | -5.858 | 1.00 | 54.01 | GZ00 | C |
| ATOM | 3247 | C | VAL | D | 212 | 14.264 | 28.011 | -6.313 | 1.00 | 62.68 | GZ00 | C |
| ATOM | 3248 | O | VAL | D | 212 | 13.334 | 28.429 | -7.016 | 1.00 | 62.49 | GZ00 | O |
| ATOM | 3249 | CB | VAL | D | 212 | 15.121 | 29.656 | -4.510 | 1.00 | 51.80 | GZ00 | C |
| ATOM | 3250 | CG1 | VAL | D | 212 | 13.642 | 30.033 | -4.363 | 1.00 | 48.35 | GZ00 | C |
| ATOM | 3251 | CG2 | VAL | D | 212 | 15.626 | 28.849 | -3.351 | 1.00 | 63.22 | GZ00 | C |
| ATOM | 3252 | N | ALA | D | 213 | 14.367 | 26.729 | -5.951 | 1.00 | 62.74 | GZ00 | N |
| ATOM | 3253 | CA | ALA | D | 213 | 13.422 | 25.713 | -6.406 | 1.00 | 61.72 | GZ00 | C |
| ATOM | 3254 | C | ALA | D | 213 | 12.828 | 24.959 | -5.225 | 1.00 | 66.88 | GZ00 | C |
| ATOM | 3255 | O | ALA | D | 213 | 13.502 | 24.746 | -4.213 | 1.00 | 69.81 | GZ00 | O |
| ATOM | 3256 | CB | ALA | D | 213 | 14.074 | 24.712 | -7.370 | 1.00 | 52.61 | GZ00 | C |
| ATOM | 3257 | N | PRO | D | 214 | 11.554 | 24.566 | -5.318 | 1.00 | 71.94 | GZ00 | N |
| ATOM | 3258 | CA | PRO | D | 214 | 10.911 | 23.880 | -4.181 | 1.00 | 73.82 | GZ00 | C |
| ATOM | 3259 | C | PRO | D | 214 | 11.485 | 22.508 | -3.872 | 1.00 | 83.15 | GZ00 | C |
| ATOM | 3260 | O | PRO | D | 214 | 11.296 | 22.024 | -2.748 | 1.00 | 87.02 | GZ00 | O |
| ATOM | 3261 | CB | PRO | D | 214 | 9.437 | 23.798 | -4.599 | 1.00 | 71.13 | GZ00 | C |
| ATOM | 3262 | CG | PRO | D | 214 | 9.430 | 24.016 | -6.077 | 1.00 | 76.39 | GZ00 | C |
| ATOM | 3263 | CD | PRO | D | 214 | 10.598 | 24.897 | -6.388 | 1.00 | 73.27 | GZ00 | C |

```
ATOM   3264  N    THR D 215      12.167  21.863  -4.815  1.00 81.47       GZ00 N
ATOM   3265  CA   THR D 215      12.918  20.647  -4.512  1.00 88.36       GZ00 C
ATOM   3266  C    THR D 215      14.100  21.003  -3.608  1.00 95.90       GZ00 C
ATOM   3267  O    THR D 215      15.126  21.513  -4.071  1.00 92.30       GZ00 O
ATOM   3268  CB   THR D 215      13.383  19.978  -5.802  1.00 93.99       GZ00 C
ATOM   3269  OG1  THR D 215      14.373  20.797  -6.434  1.00 97.00       GZ00 O
ATOM   3270  CG2  THR D 215      12.210  19.791  -6.759  1.00 92.11       GZ00 C
ATOM   3271  N    GLU D 216      13.956  20.757  -2.306  1.00 96.37       GZ00 N
ATOM   3272  CA   GLU D 216      14.997  21.115  -1.340  1.00 94.49       GZ00 C
ATOM   3273  C    GLU D 216      15.586  19.863  -0.689  1.00103.70       GZ00 C
ATOM   3274  O    GLU D 216      16.586  19.934   0.031  1.00103.19       GZ00 O
ATOM   3275  CB   GLU D 216      14.453  22.060  -0.258  1.00 98.63       GZ00 C
ATOM   3276  CG   GLU D 216      14.051  23.474  -0.741  1.00 99.48       GZ00 C
ATOM   3277  CD   GLU D 216      13.551  24.375   0.397  1.00 94.82       GZ00 C
ATOM   3278  OE1  GLU D 216      14.356  24.704   1.304  1.00 90.53       GZ00 O
ATOM   3279  OE2  GLU D 216      12.349  24.740   0.394  1.00 84.44       GZ00 O1-
TER
ATOM   3280  N    GLN A   1     -34.534  69.246 -14.750  1.00 68.93            N
ATOM   3281  CA   GLN A   1     -35.181  70.408 -14.148  1.00 63.15            C
ATOM   3282  C    GLN A   1     -34.574  70.759 -12.783  1.00 64.87            C
ATOM   3283  O    GLN A   1     -34.907  70.176 -11.757  1.00 73.80            O
ATOM   3284  CB   GLN A   1     -36.678  70.168 -14.001  1.00 72.61            C
ATOM   3285  CG   GLN A   1     -37.438  71.350 -13.425  1.00 79.05            C
ATOM   3286  CD   GLN A   1     -38.607  70.901 -12.574  1.00 82.55            C
ATOM   3287  OE1  GLN A   1     -38.585  69.803 -12.013  1.00 76.25            O
ATOM   3288  NE2  GLN A   1     -39.638  71.743 -12.478  1.00 75.07            N
ATOM   3289  N    VAL A   2     -33.670  71.708 -12.792  1.00 52.55            N
ATOM   3290  CA   VAL A   2     -33.090  72.273 -11.587  1.00 39.46            C
ATOM   3291  C    VAL A   2     -33.760  73.615 -11.340  1.00 37.36            C
ATOM   3292  O    VAL A   2     -34.051  74.357 -12.286  1.00 42.98            O
ATOM   3293  CB   VAL A   2     -31.561  72.402 -11.744  1.00 37.95            C
ATOM   3294  CG1  VAL A   2     -30.942  73.173 -10.605  1.00 32.40            C
ATOM   3295  CG2  VAL A   2     -30.938  71.007 -11.812  1.00 38.29            C
ATOM   3296  N    GLN A   3     -34.074  73.905 -10.086  1.00 28.57            N
ATOM   3297  CA   GLN A   3     -34.530  75.234  -9.705  1.00 36.17            C
ATOM   3298  C    GLN A   3     -33.616  75.802  -8.623  1.00 32.07            C
ATOM   3299  O    GLN A   3     -33.291  75.112  -7.650  1.00 33.31            O
ATOM   3300  CB   GLN A   3     -35.981  75.198  -9.230  1.00 33.26            C
ATOM   3301  CG   GLN A   3     -36.980  75.166 -10.396  1.00 48.28            C
ATOM   3302  CD   GLN A   3     -38.422  75.482  -9.975  1.00 66.43            C
ATOM   3303  OE1  GLN A   3     -38.748  75.535  -8.779  1.00 59.16            O
ATOM   3304  NE2  GLN A   3     -39.284  75.714 -10.964  1.00 65.91            N
ATOM   3305  N    LEU A   4     -33.202  77.051  -8.798  1.00 28.83            N
ATOM   3306  CA   LEU A   4     -32.404  77.779  -7.823  1.00 34.07            C
ATOM   3307  C    LEU A   4     -33.199  79.004  -7.385  1.00 31.68            C
ATOM   3308  O    LEU A   4     -33.759  79.699  -8.231  1.00 35.93            O
ATOM   3309  CB   LEU A   4     -31.054  78.181  -8.431  1.00 31.35            C
ATOM   3310  CG   LEU A   4     -30.271  77.022  -9.054  1.00 35.66            C
ATOM   3311  CD1  LEU A   4     -29.061  77.538  -9.848  1.00 33.22            C
ATOM   3312  CD2  LEU A   4     -29.820  76.024  -7.986  1.00 28.58            C
ATOM   3313  N    VAL A   5     -33.296  79.236  -6.074  1.00 31.24            N
ATOM   3314  CA   VAL A   5     -34.075  80.340  -5.501  1.00 29.71            C
ATOM   3315  C    VAL A   5     -33.192  81.106  -4.521  1.00 30.40            C
ATOM   3316  O    VAL A   5     -32.883  80.594  -3.439  1.00 34.07            O
ATOM   3317  CB   VAL A   5     -35.352  79.849  -4.790  1.00 33.16            C
ATOM   3318  CG1  VAL A   5     -36.116  81.023  -4.194  1.00 22.32            C
ATOM   3319  CG2  VAL A   5     -36.244  79.088  -5.746  1.00 27.68            C
ATOM   3320  N    GLU A   6     -32.830  82.344  -4.866  1.00 27.45            N
ATOM   3321  CA   GLU A   6     -32.039  83.183  -3.970  1.00 34.31            C
ATOM   3322  C    GLU A   6     -32.938  83.895  -2.964  1.00 35.66            C
ATOM   3323  O    GLU A   6     -34.094  84.211  -3.250  1.00 35.15            O
ATOM   3324  CB   GLU A   6     -31.242  84.251  -4.738  1.00 35.33            C
ATOM   3325  CG   GLU A   6     -30.370  83.745  -5.877  1.00 36.08            C
ATOM   3326  CD   GLU A   6     -31.115  83.636  -7.209  1.00 39.10            C
ATOM   3327  OE1  GLU A   6     -32.369  83.508  -7.199  1.00 35.08            O
ATOM   3328  OE2  GLU A   6     -30.432  83.628  -8.263  1.00 35.04            O1-
ATOM   3329  N    SER A   7     -32.368  84.204  -1.800  1.00 36.13            N
ATOM   3330  CA   SER A   7     -33.075  84.945  -0.763  1.00 34.15            C
ATOM   3331  C    SER A   7     -32.033  85.588   0.132  1.00 31.67            C
ATOM   3332  O    SER A   7     -30.848  85.263   0.062  1.00 34.14            O
ATOM   3333  CB   SER A   7     -34.004  84.045   0.060  1.00 29.39            C
```

| ATOM | 3334 | OG | SER A | 7 | -33.246 | 83.029 | 0.704 | 1.00 | 39.57 | O |
|------|------|-----|-------|----|---------|--------|-------|------|--------|-----|
| ATOM | 3335 | N | GLY A | 8 | -32.484 | 86.520 | 0.962 | 1.00 | 33.72 | N |
| ATOM | 3336 | CA | GLY A | 8 | -31.621 | 87.148 | 1.934 | 1.00 | 31.16 | C |
| ATOM | 3337 | C | GLY A | 8 | -31.165 | 88.543 | 1.590 | 1.00 | 40.14 | C |
| ATOM | 3338 | O | GLY A | 8 | -30.323 | 89.095 | 2.311 | 1.00 | 41.88 | O |
| ATOM | 3339 | N | GLY A | 9 | -31.679 | 89.131 | 0.518 | 1.00 | 30.46 | N |
| ATOM | 3340 | CA | GLY A | 9 | -31.281 | 90.478 | 0.150 | 1.00 | 42.56 | C |
| ATOM | 3341 | C | GLY A | 9 | -31.699 | 91.554 | 1.150 | 1.00 | 45.12 | C |
| ATOM | 3342 | O | GLY A | 9 | -31.989 | 91.303 | 2.319 | 1.00 | 45.51 | O |
| ATOM | 3343 | N | GLY A | 10 | -31.614 | 92.794 | 0.695 | 1.00 | 40.16 | N |
| ATOM | 3344 | CA | GLY A | 10 | -32.173 | 93.880 | 1.461 | 1.00 | 36.55 | C |
| ATOM | 3345 | C | GLY A | 10 | -31.303 | 95.118 | 1.379 | 1.00 | 41.88 | C |
| ATOM | 3346 | O | GLY A | 10 | -30.334 | 95.184 | 0.617 | 1.00 | 34.74 | O |
| ATOM | 3347 | N | VAL A | 11 | -31.676 | 96.112 | 2.184 | 1.00 | 37.50 | N |
| ATOM | 3348 | CA | VAL A | 11 | -30.953 | 97.369 | 2.303 | 1.00 | 38.58 | C |
| ATOM | 3349 | C | VAL A | 11 | -30.020 | 97.243 | 3.493 | 1.00 | 43.54 | C |
| ATOM | 3350 | O | VAL A | 11 | -30.423 | 96.759 | 4.555 | 1.00 | 47.55 | O |
| ATOM | 3351 | CB | VAL A | 11 | -31.911 | 98.559 | 2.483 | 1.00 | 48.63 | C |
| ATOM | 3352 | CG1 | VAL A | 11 | -31.118 | 99.850 | 2.585 | 1.00 | 43.07 | C |
| ATOM | 3353 | CG2 | VAL A | 11 | -32.901 | 98.626 | 1.323 | 1.00 | 45.11 | C |
| ATOM | 3354 | N | VAL A | 12 | -28.773 | 97.676 | 3.324 | 1.00 | 36.28 | N |
| ATOM | 3355 | CA | VAL A | 12 | -27.767 | 97.511 | 4.364 | 1.00 | 44.38 | C |
| ATOM | 3356 | C | VAL A | 12 | -26.802 | 98.696 | 4.298 | 1.00 | 42.35 | C |
| ATOM | 3357 | O | VAL A | 12 | -26.638 | 99.336 | 3.253 | 1.00 | 42.56 | O |
| ATOM | 3358 | CB | VAL A | 12 | -27.051 | 96.138 | 4.207 | 1.00 | 47.04 | C |
| ATOM | 3359 | CG1 | VAL A | 12 | -26.272 | 96.069 | 2.897 | 1.00 | 44.35 | C |
| ATOM | 3360 | CG2 | VAL A | 12 | -26.125 | 95.864 | 5.359 | 1.00 | 49.20 | C |
| ATOM | 3361 | N | GLN A | 13 | -26.197 | 99.009 | 5.427 | 1.00 | 43.55 | N |
| ATOM | 3362 | CA | GLN A | 13 | -25.312 | 100.156 | 5.405 | 1.00 | 44.17 | C |
| ATOM | 3363 | C | GLN A | 13 | -23.884 | 99.743 | 5.086 | 1.00 | 47.16 | C |
| ATOM | 3364 | O | GLN A | 13 | -23.471 | 98.619 | 5.399 | 1.00 | 45.14 | O |
| ATOM | 3365 | CB | GLN A | 13 | -25.334 | 100.881 | 6.734 | 1.00 | 45.16 | C |
| ATOM | 3366 | CG | GLN A | 13 | -26.698 | 101.357 | 7.135 | 1.00 | 59.94 | C |
| ATOM | 3367 | CD | GLN A | 13 | -26.607 | 102.516 | 8.099 | 1.00 | 69.00 | C |
| ATOM | 3368 | OE1 | GLN A | 13 | -26.037 | 103.566 | 7.771 | 1.00 | 69.47 | O |
| ATOM | 3369 | NE2 | GLN A | 13 | -27.151 | 102.335 | 9.297 | 1.00 | 70.89 | N |
| ATOM | 3370 | N | PRO A | 14 | -23.132 | 100.657 | 4.472 | 1.00 | 41.71 | N |
| ATOM | 3371 | CA | PRO A | 14 | -21.726 | 100.372 | 4.167 | 1.00 | 38.64 | C |
| ATOM | 3372 | C | PRO A | 14 | -21.001 | 99.888 | 5.411 | 1.00 | 41.46 | C |
| ATOM | 3373 | O | PRO A | 14 | -21.225 | 100.390 | 6.512 | 1.00 | 43.96 | O |
| ATOM | 3374 | CB | PRO A | 14 | -21.191 | 101.725 | 3.683 | 1.00 | 36.30 | C |
| ATOM | 3375 | CG | PRO A | 14 | -22.417 | 102.449 | 3.166 | 1.00 | 37.34 | C |
| ATOM | 3376 | CD | PRO A | 14 | -23.553 | 101.995 | 4.015 | 1.00 | 42.17 | C |
| ATOM | 3377 | N | GLY A | 15 | -20.150 | 98.877 | 5.237 | 1.00 | 39.52 | N |
| ATOM | 3378 | CA | GLY A | 15 | -19.389 | 98.318 | 6.335 | 1.00 | 35.94 | C |
| ATOM | 3379 | C | GLY A | 15 | -20.077 | 97.199 | 7.088 | 1.00 | 40.72 | C |
| ATOM | 3380 | O | GLY A | 15 | -19.407 | 96.463 | 7.826 | 1.00 | 44.51 | O |
| ATOM | 3381 | N | ARG A | 16 | -21.388 | 97.045 | 6.924 | 1.00 | 39.84 | N |
| ATOM | 3382 | CA | ARG A | 16 | -22.114 | 95.953 | 7.556 | 1.00 | 53.52 | C |
| ATOM | 3383 | C | ARG A | 16 | -21.962 | 94.660 | 6.746 | 1.00 | 46.84 | C |
| ATOM | 3384 | O | ARG A | 16 | -21.259 | 94.600 | 5.727 | 1.00 | 39.84 | O |
| ATOM | 3385 | CB | ARG A | 16 | -23.592 | 96.303 | 7.706 | 1.00 | 54.09 | C |
| ATOM | 3386 | CG | ARG A | 16 | -23.880 | 97.531 | 8.533 | 1.00 | 61.92 | C |
| ATOM | 3387 | CD | ARG A | 16 | -23.430 | 97.323 | 9.960 | 1.00 | 69.75 | C |
| ATOM | 3388 | NE | ARG A | 16 | -24.436 | 97.796 | 10.906 | 1.00 | 97.69 | N |
| ATOM | 3389 | CZ | ARG A | 16 | -24.245 | 97.901 | 12.218 | 1.00 | 105.26 | C |
| ATOM | 3390 | NH1 | ARG A | 16 | -23.075 | 97.566 | 12.754 | 1.00 | 97.15 | N1+ |
| ATOM | 3391 | NH2 | ARG A | 16 | -25.226 | 98.346 | 12.995 | 1.00 | 108.93 | N |
| ATOM | 3392 | N | SER A | 17 | -22.664 | 93.625 | 7.206 | 1.00 | 37.36 | N |
| ATOM | 3393 | CA | SER A | 17 | -22.586 | 92.271 | 6.692 | 1.00 | 37.19 | C |
| ATOM | 3394 | C | SER A | 17 | -23.962 | 91.796 | 6.274 | 1.00 | 41.75 | C |
| ATOM | 3395 | O | SER A | 17 | -24.980 | 92.251 | 6.795 | 1.00 | 39.73 | O |
| ATOM | 3396 | CB | SER A | 17 | -22.044 | 91.275 | 7.738 | 1.00 | 42.19 | C |
| ATOM | 3397 | OG | SER A | 17 | -20.664 | 91.495 | 7.994 | 1.00 | 55.67 | O |
| ATOM | 3398 | N | LEU A | 18 | -23.976 | 90.833 | 5.361 | 1.00 | 40.66 | N |
| ATOM | 3399 | CA | LEU A | 18 | -25.220 | 90.256 | 4.877 | 1.00 | 42.98 | C |
| ATOM | 3400 | C | LEU A | 18 | -24.901 | 88.861 | 4.361 | 1.00 | 40.59 | C |
| ATOM | 3401 | O | LEU A | 18 | -23.804 | 88.623 | 3.850 | 1.00 | 41.78 | O |
| ATOM | 3402 | CB | LEU A | 18 | -25.806 | 91.135 | 3.773 | 1.00 | 44.15 | C |
| ATOM | 3403 | CG | LEU A | 18 | -27.278 | 91.269 | 3.460 | 1.00 | 54.36 | C |
| ATOM | 3404 | CD1 | LEU A | 18 | -28.038 | 91.644 | 4.712 | 1.00 | 49.30 | C |

```
ATOM   3405  CD2 LEU A  18   -27.392  92.395   2.454  1.00 49.78      C
ATOM   3406  N   ARG A  19   -25.848  87.940   4.497  1.00 30.82      N
ATOM   3407  CA  ARG A  19   -25.670  86.595   3.965  1.00 34.82      C
ATOM   3408  C   ARG A  19   -26.783  86.270   2.981  1.00 34.54      C
ATOM   3409  O   ARG A  19   -27.959  86.364   3.329  1.00 38.10      O
ATOM   3410  CB  ARG A  19   -25.635  85.549   5.073  1.00 35.74      C
ATOM   3411  CG  ARG A  19   -25.012  84.268   4.606  1.00 37.23      C
ATOM   3412  CD  ARG A  19   -25.223  83.128   5.579  1.00 38.05      C
ATOM   3413  NE  ARG A  19   -26.633  82.826   5.730  1.00 39.69      N
ATOM   3414  CZ  ARG A  19   -27.107  81.838   6.477  1.00 47.15      C
ATOM   3415  NH1 ARG A  19   -28.419  81.642   6.559  1.00 43.39      N1+
ATOM   3416  NH2 ARG A  19   -26.271  81.043   7.130  1.00 46.94      N
ATOM   3417  N   LEU A  20   -26.416  85.921   1.750  1.00 33.95      N
ATOM   3418  CA  LEU A  20   -27.386  85.431   0.783  1.00 32.04      C
ATOM   3419  C   LEU A  20   -27.486  83.908   0.841  1.00 35.48      C
ATOM   3420  O   LEU A  20   -26.522  83.206   1.174  1.00 34.13      O
ATOM   3421  CB  LEU A  20   -27.019  85.873  -0.631  1.00 26.84      C
ATOM   3422  CG  LEU A  20   -26.829  87.380  -0.792  1.00 31.60      C
ATOM   3423  CD1 LEU A  20   -26.621  87.704  -2.261  1.00 25.55      C
ATOM   3424  CD2 LEU A  20   -28.028  88.140  -0.220  1.00 24.15      C
ATOM   3425  N   SER A  21   -28.675  83.403   0.528  1.00 33.17      N
ATOM   3426  CA  SER A  21   -28.924  81.976   0.391  1.00 32.39      C
ATOM   3427  C   SER A  21   -29.441  81.658  -1.002  1.00 34.28      C
ATOM   3428  O   SER A  21   -30.123  82.470  -1.633  1.00 35.15      O
ATOM   3429  CB  SER A  21   -29.944  81.457   1.411  1.00 29.91      C
ATOM   3430  OG  SER A  21   -29.401  81.456   2.706  1.00 32.59      O
ATOM   3431  N   CYS A  22   -29.122  80.452  -1.463  1.00 29.55      N
ATOM   3432  CA  CYS A  22   -29.608  79.954  -2.740  1.00 29.82      C
ATOM   3433  C   CYS A  22   -30.059  78.520  -2.506  1.00 34.08      C
ATOM   3434  O   CYS A  22   -29.225  77.635  -2.286  1.00 33.80      O
ATOM   3435  CB  CYS A  22   -28.515  80.040  -3.803  1.00 37.60      C
ATOM   3436  SG  CYS A  22   -28.846  79.175  -5.362  1.00 44.81      S
ATOM   3437  N   ALA A  23   -31.366  78.297  -2.544  1.00 27.47      N
ATOM   3438  CA  ALA A  23   -31.956  77.006  -2.235  1.00 31.62      C
ATOM   3439  C   ALA A  23   -32.152  76.263  -3.540  1.00 32.14      C
ATOM   3440  O   ALA A  23   -32.753  76.799  -4.480  1.00 32.98      O
ATOM   3441  CB  ALA A  23   -33.296  77.155  -1.500  1.00 23.36      C
ATOM   3442  N   ALA A  24   -31.646  75.037  -3.593  1.00 31.79      N
ATOM   3443  CA  ALA A  24   -31.642  74.239  -4.809  1.00 29.92      C
ATOM   3444  C   ALA A  24   -32.595  73.062  -4.685  1.00 29.71      C
ATOM   3445  O   ALA A  24   -32.735  72.476  -3.612  1.00 39.67      O
ATOM   3446  CB  ALA A  24   -30.238  73.724  -5.104  1.00 27.23      C
ATOM   3447  N   SER A  25   -33.239  72.708  -5.790  1.00 32.42      N
ATOM   3448  CA  SER A  25   -34.067  71.515  -5.851  1.00 28.53      C
ATOM   3449  C   SER A  25   -34.108  70.982  -7.273  1.00 34.80      C
ATOM   3450  O   SER A  25   -33.754  71.688  -8.228  1.00 28.65      O
ATOM   3451  CB  SER A  25   -35.484  71.822  -5.400  1.00 36.68      C
ATOM   3452  OG  SER A  25   -36.046  72.798  -6.269  1.00 34.63      O
ATOM   3453  N   GLY A  26   -34.558  69.720  -7.410  1.00 31.26      N
ATOM   3454  CA  GLY A  26   -34.824  69.150  -8.718  1.00 31.69      C
ATOM   3455  C   GLY A  26   -33.759  68.254  -9.323  1.00 50.48      C
ATOM   3456  O   GLY A  26   -33.944  67.786 -10.460  1.00 59.29      O
ATOM   3457  N   PHE A  27   -32.666  68.001  -8.613  1.00 34.76      N
ATOM   3458  CA  PHE A  27   -31.536  67.198  -9.082  1.00 36.25      C
ATOM   3459  C   PHE A  27   -30.495  67.347  -8.004  1.00 35.91      C
ATOM   3460  O   PHE A  27   -30.163  68.473  -7.622  1.00 41.91      O
ATOM   3461  CB  PHE A  27   -30.986  67.647 -10.455  1.00 34.34      C
ATOM   3462  CG  PHE A  27   -29.671  66.974 -10.868  1.00 35.77      C
ATOM   3463  CD1 PHE A  27   -29.467  65.601 -10.706  1.00 38.27      C
ATOM   3464  CD2 PHE A  27   -28.645  67.728 -11.455  1.00 39.96      C
ATOM   3465  CE1 PHE A  27   -28.246  64.992 -11.075  1.00 42.34      C
ATOM   3466  CE2 PHE A  27   -27.422  67.132 -11.848  1.00 39.02      C
ATOM   3467  CZ  PHE A  27   -27.221  65.758 -11.651  1.00 37.64      C
ATOM   3468  N   THR A  28   -29.960  66.224  -7.555  1.00 34.00      N
ATOM   3469  CA  THR A  28   -29.086  66.145  -6.398  1.00 32.16      C
ATOM   3470  C   THR A  28   -28.160  67.347  -6.297  1.00 32.73      C
ATOM   3471  O   THR A  28   -27.324  67.570  -7.174  1.00 34.74      O
ATOM   3472  CB  THR A  28   -28.286  64.858  -6.521  1.00 39.57      C
ATOM   3473  OG1 THR A  28   -29.211  63.801  -6.780  1.00 48.76      O
ATOM   3474  CG2 THR A  28   -27.527  64.570  -5.250  1.00 39.97      C
ATOM   3475  N   PHE A  29   -28.340  68.137  -5.232  1.00 37.84      N
```

```
ATOM   3476  CA   PHE A  29   -27.545  69.348  -5.012  1.00 34.13      C
ATOM   3477  C    PHE A  29   -26.050  69.063  -5.057  1.00 35.71      C
ATOM   3478  O    PHE A  29   -25.277  69.821  -5.666  1.00 31.39      O
ATOM   3479  CB   PHE A  29   -27.924  69.954  -3.661  1.00 33.04      C
ATOM   3480  CG   PHE A  29   -27.217  71.251  -3.309  1.00 29.10      C
ATOM   3481  CD1  PHE A  29   -27.347  72.386  -4.112  1.00 28.49      C
ATOM   3482  CD2  PHE A  29   -26.505  71.364  -2.117  1.00 30.52      C
ATOM   3483  CE1  PHE A  29   -26.748  73.607  -3.754  1.00 29.07      C
ATOM   3484  CE2  PHE A  29   -25.893  72.592  -1.755  1.00 33.69      C
ATOM   3485  CZ   PHE A  29   -26.023  73.707  -2.578  1.00 27.96      C
ATOM   3486  N    SER A  30   -25.628  67.963  -4.434  1.00 33.43      N
ATOM   3487  CA   SER A  30   -24.208  67.634  -4.348  1.00 38.52      C
ATOM   3488  C    SER A  30   -23.621  67.208  -5.682  1.00 33.12      C
ATOM   3489  O    SER A  30   -22.422  66.936  -5.741  1.00 35.08      O
ATOM   3490  CB   SER A  30   -23.988  66.534  -3.303  1.00 28.69      C
ATOM   3491  OG   SER A  30   -24.728  65.385  -3.669  1.00 38.89      O
ATOM   3492  N    SER A  31   -24.419  67.147  -6.740  1.00 27.16      N
ATOM   3493  CA   SER A  31   -23.905  66.778  -8.045  1.00 38.98      C
ATOM   3494  C    SER A  31   -23.443  67.962  -8.896  1.00 39.11      C
ATOM   3495  O    SER A  31   -23.036  67.740 -10.050  1.00 33.54      O
ATOM   3496  CB   SER A  31   -24.944  65.966  -8.806  1.00 31.50      C
ATOM   3497  OG   SER A  31   -25.034  64.689  -8.205  1.00 43.55      O
ATOM   3498  N    TYR A  32   -23.469  69.194  -8.377  1.00 30.49      N
ATOM   3499  CA   TYR A  32   -22.942  70.307  -9.164  1.00 28.71      C
ATOM   3500  C    TYR A  32   -22.356  71.382  -8.268  1.00 29.76      C
ATOM   3501  O    TYR A  32   -22.712  71.503  -7.092  1.00 29.23      O
ATOM   3502  CB   TYR A  32   -24.002  70.910 -10.078  1.00 34.80      C
ATOM   3503  CG   TYR A  32   -25.331  71.155  -9.416  1.00 32.18      C
ATOM   3504  CD1  TYR A  32   -25.549  72.299  -8.660  1.00 33.21      C
ATOM   3505  CD2  TYR A  32   -26.364  70.259  -9.562  1.00 32.73      C
ATOM   3506  CE1  TYR A  32   -26.770  72.536  -8.050  1.00 37.14      C
ATOM   3507  CE2  TYR A  32   -27.589  70.484  -8.953  1.00 36.08      C
ATOM   3508  CZ   TYR A  32   -27.783  71.621  -8.202  1.00 34.54      C
ATOM   3509  OH   TYR A  32   -28.995  71.854  -7.605  1.00 42.41      O
ATOM   3510  N    GLY A  33   -21.391  72.118  -8.828  1.00 29.06      N
ATOM   3511  CA   GLY A  33   -20.917  73.336  -8.213  1.00 26.22      C
ATOM   3512  C    GLY A  33   -21.823  74.508  -8.562  1.00 32.33      C
ATOM   3513  O    GLY A  33   -22.749  74.389  -9.373  1.00 32.28      O
ATOM   3514  N    LEU A  34   -21.549  75.660  -7.944  1.00 28.11      N
ATOM   3515  CA   LEU A  34   -22.397  76.825  -8.149  1.00 28.06      C
ATOM   3516  C    LEU A  34   -21.579  78.107  -8.137  1.00 32.81      C
ATOM   3517  O    LEU A  34   -20.482  78.185  -7.562  1.00 30.26      O
ATOM   3518  CB   LEU A  34   -23.503  76.934  -7.083  1.00 25.69      C
ATOM   3519  CG   LEU A  34   -24.496  75.772  -7.075  1.00 29.24      C
ATOM   3520  CD1  LEU A  34   -24.122  74.810  -5.948  1.00 25.51      C
ATOM   3521  CD2  LEU A  34   -25.937  76.248  -6.951  1.00 28.69      C
ATOM   3522  N    HIS A  35   -22.174  79.124  -8.757  1.00 25.76      N
ATOM   3523  CA   HIS A  35   -21.645  80.469  -8.877  1.00 28.30      C
ATOM   3524  C    HIS A  35   -22.559  81.459  -8.167  1.00 28.45      C
ATOM   3525  O    HIS A  35   -23.771  81.255  -8.047  1.00 26.56      O
ATOM   3526  CB   HIS A  35   -21.583  80.946 -10.334  1.00 27.75      C
ATOM   3527  CG   HIS A  35   -20.599  80.236 -11.204  1.00 34.48      C
ATOM   3528  ND1  HIS A  35   -19.305  80.689 -11.377  1.00 31.96      N
ATOM   3529  CD2  HIS A  35   -20.742  79.174 -12.034  1.00 27.73      C
ATOM   3530  CE1  HIS A  35   -18.693  79.927 -12.263  1.00 30.71      C
ATOM   3531  NE2  HIS A  35   -19.537  78.989 -12.664  1.00 32.82      N
ATOM   3532  N    TRP A  36   -21.971  82.583  -7.793  1.00 24.52      N
ATOM   3533  CA   TRP A  36   -22.702  83.815  -7.578  1.00 24.53      C
ATOM   3534  C    TRP A  36   -22.276  84.804  -8.656  1.00 30.64      C
ATOM   3535  O    TRP A  36   -21.078  84.979  -8.895  1.00 31.45      O
ATOM   3536  CB   TRP A  36   -22.431  84.389  -6.184  1.00 25.38      C
ATOM   3537  CG   TRP A  36   -23.102  83.636  -5.061  1.00 34.21      C
ATOM   3538  CD1  TRP A  36   -22.528  82.700  -4.225  1.00 27.01      C
ATOM   3539  CD2  TRP A  36   -24.483  83.734  -4.662  1.00 29.26      C
ATOM   3540  NE1  TRP A  36   -23.464  82.236  -3.328  1.00 29.61      N
ATOM   3541  CE2  TRP A  36   -24.669  82.850  -3.574  1.00 29.15      C
ATOM   3542  CE3  TRP A  36   -25.569  84.500  -5.104  1.00 31.70      C
ATOM   3543  CZ2  TRP A  36   -25.902  82.707  -2.925  1.00 29.62      C
ATOM   3544  CZ3  TRP A  36   -26.795  84.353  -4.464  1.00 31.58      C
ATOM   3545  CH2  TRP A  36   -26.950  83.447  -3.390  1.00 32.02      C
ATOM   3546  N    VAL A  37   -23.255  85.430  -9.315  1.00 26.17      N
```

```
ATOM   3547  CA  VAL A  37     -23.052  86.487 -10.305  1.00 26.93           C
ATOM   3548  C   VAL A  37     -23.911  87.683  -9.909  1.00 31.18           C
ATOM   3549  O   VAL A  37     -24.979  87.514  -9.311  1.00 32.57           O
ATOM   3550  CB  VAL A  37     -23.436  86.003 -11.725  1.00 30.07           C
ATOM   3551  CG1 VAL A  37     -23.336  87.141 -12.741  1.00 25.17           C
ATOM   3552  CG2 VAL A  37     -22.577  84.797 -12.141  1.00 27.92           C
ATOM   3553  N   ARG A  38     -23.451  88.900 -10.217  1.00 26.83           N
ATOM   3554  CA  ARG A  38     -24.222  90.086  -9.838  1.00 28.59           C
ATOM   3555  C   ARG A  38     -24.327  91.074 -10.987  1.00 32.00           C
ATOM   3556  O   ARG A  38     -23.546  91.042 -11.942  1.00 34.12           O
ATOM   3557  CB  ARG A  38     -23.649  90.790  -8.616  1.00 24.52           C
ATOM   3558  CG  ARG A  38     -22.405  91.581  -8.863  1.00 27.87           C
ATOM   3559  CD  ARG A  38     -21.832  92.016  -7.532  1.00 25.42           C
ATOM   3560  NE  ARG A  38     -20.569  92.691  -7.719  1.00 26.21           N
ATOM   3561  CZ  ARG A  38     -19.825  93.186  -6.735  1.00 33.88           C
ATOM   3562  NH1 ARG A  38     -20.207  93.058  -5.470  1.00 31.46           N1+
ATOM   3563  NH2 ARG A  38     -18.700  93.822  -7.026  1.00 27.28           N
ATOM   3564  N   GLN A  39     -25.317  91.961 -10.874  1.00 31.14           N
ATOM   3565  CA  GLN A  39     -25.594  92.942 -11.923  1.00 26.67           C
ATOM   3566  C   GLN A  39     -26.067  94.237 -11.278  1.00 33.37           C
ATOM   3567  O   GLN A  39     -27.144  94.282 -10.657  1.00 30.44           O
ATOM   3568  CB  GLN A  39     -26.635  92.419 -12.901  1.00 29.71           C
ATOM   3569  CG  GLN A  39     -26.977  93.391 -14.031  1.00 30.87           C
ATOM   3570  CD  GLN A  39     -27.797  92.710 -15.108  1.00 35.35           C
ATOM   3571  OE1 GLN A  39     -28.796  92.048 -14.812  1.00 37.94           O
ATOM   3572  NE2 GLN A  39     -27.370  92.841 -16.358  1.00 26.92           N
ATOM   3573  N   ALA A  40     -25.269  95.279 -11.425  1.00 28.05           N
ATOM   3574  CA  ALA A  40     -25.664  96.588 -10.926  1.00 34.65           C
ATOM   3575  C   ALA A  40     -26.709  97.199 -11.859  1.00 34.83           C
ATOM   3576  O   ALA A  40     -26.757  96.861 -13.039  1.00 32.60           O
ATOM   3577  CB  ALA A  40     -24.443  97.499 -10.812  1.00 29.23           C
ATOM   3578  N   PRO A  41     -27.556  98.095 -11.352  1.00 41.70           N
ATOM   3579  CA  PRO A  41     -28.654  98.645 -12.175  1.00 37.62           C
ATOM   3580  C   PRO A  41     -28.143  99.327 -13.440  1.00 38.87           C
ATOM   3581  O   PRO A  41     -27.259 100.188 -13.392  1.00 37.58           O
ATOM   3582  CB  PRO A  41     -29.330  99.656 -11.236  1.00 34.86           C
ATOM   3583  CG  PRO A  41     -28.936  99.229  -9.859  1.00 40.20           C
ATOM   3584  CD  PRO A  41     -27.557  98.641  -9.983  1.00 35.94           C
ATOM   3585  N   GLY A  42     -28.698  98.918 -14.578  1.00 36.04           N
ATOM   3586  CA  GLY A  42     -28.295  99.408 -15.887  1.00 32.92           C
ATOM   3587  C   GLY A  42     -26.969  98.887 -16.429  1.00 42.89           C
ATOM   3588  O   GLY A  42     -26.492  99.395 -17.446  1.00 48.01           O
ATOM   3589  N   LYS A  43     -26.358  97.882 -15.817  1.00 37.13           N
ATOM   3590  CA  LYS A  43     -25.003  97.473 -16.196  1.00 39.84           C
ATOM   3591  C   LYS A  43     -24.961  95.989 -16.548  1.00 36.76           C
ATOM   3592  O   LYS A  43     -25.989  95.301 -16.591  1.00 33.65           O
ATOM   3593  CB  LYS A  43     -24.004  97.816 -15.094  1.00 35.22           C
ATOM   3594  CG  LYS A  43     -23.960  99.326 -14.833  1.00 42.47           C
ATOM   3595  CD  LYS A  43     -22.804  99.688 -13.941  1.00 57.71           C
ATOM   3596  CE  LYS A  43     -22.589 101.201 -13.864  1.00 72.06           C
ATOM   3597  NZ  LYS A  43     -21.339 101.531 -13.103  1.00 70.64           N1+
ATOM   3598  N   GLY A  44     -23.743  95.520 -16.834  1.00 31.12           N
ATOM   3599  CA  GLY A  44     -23.510  94.165 -17.290  1.00 28.92           C
ATOM   3600  C   GLY A  44     -23.333  93.158 -16.166  1.00 33.92           C
ATOM   3601  O   GLY A  44     -23.295  93.484 -14.979  1.00 35.65           O
ATOM   3602  N   LEU A  45     -23.202  91.898 -16.569  1.00 31.47           N
ATOM   3603  CA  LEU A  45     -22.959  90.839 -15.606  1.00 26.60           C
ATOM   3604  C   LEU A  45     -21.542  90.959 -15.072  1.00 31.15           C
ATOM   3605  O   LEU A  45     -20.601  91.246 -15.820  1.00 31.96           O
ATOM   3606  CB  LEU A  45     -23.173  89.462 -16.256  1.00 28.04           C
ATOM   3607  CG  LEU A  45     -24.575  89.294 -16.881  1.00 33.18           C
ATOM   3608  CD1 LEU A  45     -24.804  87.921 -17.544  1.00 29.82           C
ATOM   3609  CD2 LEU A  45     -25.639  89.585 -15.859  1.00 27.89           C
ATOM   3610  N   GLU A  46     -21.397  90.735 -13.767  1.00 30.38           N
ATOM   3611  CA  GLU A  46     -20.101  90.660 -13.112  1.00 31.96           C
ATOM   3612  C   GLU A  46     -20.049  89.376 -12.306  1.00 28.71           C
ATOM   3613  O   GLU A  46     -20.899  89.156 -11.440  1.00 28.54           O
ATOM   3614  CB  GLU A  46     -19.854  91.859 -12.196  1.00 30.82           C
ATOM   3615  CG  GLU A  46     -18.411  91.886 -11.708  1.00 35.43           C
ATOM   3616  CD  GLU A  46     -18.145  92.863 -10.551  1.00 42.19           C
ATOM   3617  OE1 GLU A  46     -19.076  93.544 -10.038  1.00 34.18           O
```

| ATOM | 3618 | OE2 | GLU | A | 46 | -16.968 | 92.932 | -10.154 | 1.00 | 46.04 | O1- |
| ATOM | 3619 | N | TRP | A | 47 | -19.052 | 88.537 | -12.584 | 1.00 | 28.91 | N |
| ATOM | 3620 | CA | TRP | A | 47 | -18.888 | 87.300 | -11.834 | 1.00 | 28.05 | C |
| ATOM | 3621 | C | TRP | A | 47 | -18.433 | 87.610 | -10.414 | 1.00 | 28.38 | C |
| ATOM | 3622 | O | TRP | A | 47 | -17.652 | 88.534 | -10.199 | 1.00 | 29.03 | O |
| ATOM | 3623 | CB | TRP | A | 47 | -17.882 | 86.396 | -12.547 | 1.00 | 28.79 | C |
| ATOM | 3624 | CG | TRP | A | 47 | -17.570 | 85.150 | -11.808 | 1.00 | 27.80 | C |
| ATOM | 3625 | CD1 | TRP | A | 47 | -18.396 | 84.090 | -11.618 | 1.00 | 26.75 | C |
| ATOM | 3626 | CD2 | TRP | A | 47 | -16.330 | 84.810 | -11.186 | 1.00 | 29.28 | C |
| ATOM | 3627 | NE1 | TRP | A | 47 | -17.759 | 83.119 | -10.900 | 1.00 | 30.04 | N |
| ATOM | 3628 | CE2 | TRP | A | 47 | -16.487 | 83.529 | -10.623 | 1.00 | 26.53 | C |
| ATOM | 3629 | CE3 | TRP | A | 47 | -15.093 | 85.455 | -11.067 | 1.00 | 30.37 | C |
| ATOM | 3630 | CZ2 | TRP | A | 47 | -15.454 | 82.870 | -9.945 | 1.00 | 30.41 | C |
| ATOM | 3631 | CZ3 | TRP | A | 47 | -14.058 | 84.797 | -10.376 | 1.00 | 27.15 | C |
| ATOM | 3632 | CH2 | TRP | A | 47 | -14.251 | 83.527 | -9.832 | 1.00 | 30.81 | C |
| ATOM | 3633 | N | VAL | A | 48 | -18.923 | 86.836 | -9.441 | 1.00 | 26.85 | N |
| ATOM | 3634 | CA | VAL | A | 48 | -18.590 | 87.031 | -8.026 | 1.00 | 26.22 | C |
| ATOM | 3635 | C | VAL | A | 48 | -17.740 | 85.890 | -7.475 | 1.00 | 27.91 | C |
| ATOM | 3636 | O | VAL | A | 48 | -16.641 | 86.118 | -6.962 | 1.00 | 31.20 | O |
| ATOM | 3637 | CB | VAL | A | 48 | -19.858 | 87.245 | -7.171 | 1.00 | 29.89 | C |
| ATOM | 3638 | CG1 | VAL | A | 48 | -19.465 | 87.415 | -5.709 | 1.00 | 27.30 | C |
| ATOM | 3639 | CG2 | VAL | A | 48 | -20.640 | 88.465 | -7.680 | 1.00 | 27.25 | C |
| ATOM | 3640 | N | ALA | A | 49 | -18.234 | 84.656 | -7.552 | 1.00 | 27.92 | N |
| ATOM | 3641 | CA | ALA | A | 49 | -17.498 | 83.540 | -6.964 | 1.00 | 27.21 | C |
| ATOM | 3642 | C | ALA | A | 49 | -18.028 | 82.240 | -7.528 | 1.00 | 29.25 | C |
| ATOM | 3643 | O | ALA | A | 49 | -19.155 | 82.178 | -8.025 | 1.00 | 29.04 | O |
| ATOM | 3644 | CB | ALA | A | 49 | -17.590 | 83.500 | -5.429 | 1.00 | 22.18 | C |
| ATOM | 3645 | N | VAL | A | 50 | -17.187 | 81.190 | -7.435 | 1.00 | 25.65 | N |
| ATOM | 3646 | CA | VAL | A | 50 | -17.592 | 79.835 | -7.777 | 1.00 | 25.46 | C |
| ATOM | 3647 | C | VAL | A | 50 | -17.129 | 78.894 | -6.678 | 1.00 | 28.11 | C |
| ATOM | 3648 | O | VAL | A | 50 | -16.137 | 79.144 | -5.988 | 1.00 | 30.11 | O |
| ATOM | 3649 | CB | VAL | A | 50 | -17.068 | 79.380 | -9.162 | 1.00 | 31.87 | C |
| ATOM | 3650 | CG1 | VAL | A | 50 | -15.561 | 79.238 | -9.175 | 1.00 | 28.23 | C |
| ATOM | 3651 | CG2 | VAL | A | 50 | -17.767 | 78.084 | -9.606 | 1.00 | 28.82 | C |
| ATOM | 3652 | N | ILE | A | 51 | -17.884 | 77.821 | -6.484 | 1.00 | 29.93 | N |
| ATOM | 3653 | CA | ILE | A | 51 | -17.514 | 76.799 | -5.522 | 1.00 | 25.88 | C |
| ATOM | 3654 | C | ILE | A | 51 | -17.657 | 75.447 | -6.203 | 1.00 | 26.86 | C |
| ATOM | 3655 | O | ILE | A | 51 | -18.486 | 75.262 | -7.100 | 1.00 | 26.48 | O |
| ATOM | 3656 | CB | ILE | A | 51 | -18.354 | 76.879 | -4.227 | 1.00 | 28.38 | C |
| ATOM | 3657 | CG1 | ILE | A | 51 | -17.776 | 75.932 | -3.163 | 1.00 | 29.68 | C |
| ATOM | 3658 | CG2 | ILE | A | 51 | -19.831 | 76.565 | -4.498 | 1.00 | 23.38 | C |
| ATOM | 3659 | CD1 | ILE | A | 51 | -18.344 | 76.180 | -1.775 | 1.00 | 28.59 | C |
| ATOM | 3660 | N | TRP | A | 52 | -16.821 | 74.510 | -5.793 | 1.00 | 24.08 | N |
| ATOM | 3661 | CA | TRP | A | 52 | -16.856 | 73.188 | -6.392 | 1.00 | 28.60 | C |
| ATOM | 3662 | C | TRP | A | 52 | -18.074 | 72.397 | -5.899 | 1.00 | 30.88 | C |
| ATOM | 3663 | O | TRP | A | 52 | -18.688 | 72.722 | -4.879 | 1.00 | 26.43 | O |
| ATOM | 3664 | CB | TRP | A | 52 | -15.559 | 72.452 | -6.066 | 1.00 | 30.05 | C |
| ATOM | 3665 | CG | TRP | A | 52 | -14.821 | 71.992 | -7.256 | 1.00 | 31.59 | C |
| ATOM | 3666 | CD1 | TRP | A | 52 | -14.551 | 70.701 | -7.601 | 1.00 | 34.80 | C |
| ATOM | 3667 | CD2 | TRP | A | 52 | -14.265 | 72.811 | -8.293 | 1.00 | 30.63 | C |
| ATOM | 3668 | NE1 | TRP | A | 52 | -13.842 | 70.664 | -8.781 | 1.00 | 33.66 | N |
| ATOM | 3669 | CE2 | TRP | A | 52 | -13.652 | 71.942 | -9.228 | 1.00 | 31.14 | C |
| ATOM | 3670 | CE3 | TRP | A | 52 | -14.220 | 74.183 | -8.522 | 1.00 | 28.98 | C |
| ATOM | 3671 | CZ2 | TRP | A | 52 | -12.999 | 72.404 | -10.368 | 1.00 | 30.12 | C |
| ATOM | 3672 | CZ3 | TRP | A | 52 | -13.564 | 74.646 | -9.663 | 1.00 | 37.05 | C |
| ATOM | 3673 | CH2 | TRP | A | 52 | -12.953 | 73.756 | -10.562 | 1.00 | 36.23 | C |
| ATOM | 3674 | N | TYR | A | 53 | -18.401 | 71.330 | -6.635 | 1.00 | 28.62 | N |
| ATOM | 3675 | CA | TYR | A | 53 | -19.539 | 70.484 | -6.281 | 1.00 | 31.01 | C |
| ATOM | 3676 | C | TYR | A | 53 | -19.373 | 69.846 | -4.909 | 1.00 | 36.12 | C |
| ATOM | 3677 | O | TYR | A | 53 | -20.370 | 69.557 | -4.240 | 1.00 | 36.43 | O |
| ATOM | 3678 | CB | TYR | A | 53 | -19.743 | 69.393 | -7.329 | 1.00 | 31.97 | C |
| ATOM | 3679 | CG | TYR | A | 53 | -18.458 | 68.713 | -7.742 | 1.00 | 35.13 | C |
| ATOM | 3680 | CD1 | TYR | A | 53 | -17.918 | 67.672 | -6.989 | 1.00 | 37.05 | C |
| ATOM | 3681 | CD2 | TYR | A | 53 | -17.791 | 69.104 | -8.896 | 1.00 | 37.33 | C |
| ATOM | 3682 | CE1 | TYR | A | 53 | -16.755 | 67.058 | -7.366 | 1.00 | 36.11 | C |
| ATOM | 3683 | CE2 | TYR | A | 53 | -16.614 | 68.492 | -9.286 | 1.00 | 40.57 | C |
| ATOM | 3684 | CZ | TYR | A | 53 | -16.095 | 67.479 | -8.521 | 1.00 | 40.64 | C |
| ATOM | 3685 | OH | TYR | A | 53 | -14.916 | 66.886 | -8.923 | 1.00 | 51.83 | O |
| ATOM | 3686 | N | ASP | A | 54 | -18.136 | 69.613 | -4.468 | 1.00 | 31.86 | N |
| ATOM | 3687 | CA | ASP | A | 54 | -17.896 | 69.044 | -3.150 | 1.00 | 32.47 | C |
| ATOM | 3688 | C | ASP | A | 54 | -17.433 | 70.090 | -2.141 | 1.00 | 32.55 | C |

```
ATOM   3689  O    ASP A  54   -16.894  69.732  -1.097  1.00 34.26       O
ATOM   3690  CB   ASP A  54   -16.882  67.901  -3.245  1.00 33.69       C
ATOM   3691  CG   ASP A  54   -15.550  68.344  -3.878  1.00 41.63       C
ATOM   3692  OD1  ASP A  54   -15.332  69.574  -4.029  1.00 35.39       O
ATOM   3693  OD2  ASP A  54   -14.721  67.462  -4.224  1.00 41.66       O1-
ATOM   3694  N    GLY A  55   -17.633  71.376  -2.428  1.00 35.34       N
ATOM   3695  CA   GLY A  55   -17.197  72.416  -1.516  1.00 28.60       C
ATOM   3696  C    GLY A  55   -15.696  72.626  -1.397  1.00 30.85       C
ATOM   3697  O    GLY A  55   -15.261  73.340  -0.483  1.00 31.85       O
ATOM   3698  N    SER A  56   -14.880  72.030  -2.271  1.00 31.41       N
ATOM   3699  CA   SER A  56   -13.427  72.159  -2.138  1.00 28.55       C
ATOM   3700  C    SER A  56   -12.921  73.456  -2.773  1.00 29.96       C
ATOM   3701  O    SER A  56   -12.790  74.463  -2.071  1.00 35.87       O
ATOM   3702  CB   SER A  56   -12.716  70.922  -2.715  1.00 29.73       C
ATOM   3703  OG   SER A  56   -13.055  70.688  -4.069  1.00 33.14       O
ATOM   3704  N    ASN A  57   -12.648  73.468  -4.080  1.00 27.89       N
ATOM   3705  CA   ASN A  57   -12.094  74.669  -4.708  1.00 30.78       C
ATOM   3706  C    ASN A  57   -13.090  75.836  -4.663  1.00 34.96       C
ATOM   3707  O    ASN A  57   -14.296  75.655  -4.883  1.00 29.93       O
ATOM   3708  CB   ASN A  57   -11.712  74.392  -6.167  1.00 28.41       C
ATOM   3709  CG   ASN A  57   -10.430  73.551  -6.316  1.00 36.02       C
ATOM   3710  OD1  ASN A  57   -10.034  72.789  -5.427  1.00 31.33       O
ATOM   3711  ND2  ASN A  57    -9.779  73.703  -7.462  1.00 33.91       N
ATOM   3712  N    LYS A  58   -12.562  77.042  -4.418  1.00 30.71       N
ATOM   3713  CA   LYS A  58   -13.307  78.299  -4.384  1.00 31.44       C
ATOM   3714  C    LYS A  58   -12.504  79.356  -5.117  1.00 31.50       C
ATOM   3715  O    LYS A  58   -11.306  79.491  -4.858  1.00 35.00       O
ATOM   3716  CB   LYS A  58   -13.541  78.796  -2.953  1.00 28.26       C
ATOM   3717  CG   LYS A  58   -14.308  77.853  -2.071  1.00 32.31       C
ATOM   3718  CD   LYS A  58   -14.359  78.350  -0.623  1.00 29.79       C
ATOM   3719  CE   LYS A  58   -15.146  77.337   0.247  1.00 31.84       C
ATOM   3720  NZ   LYS A  58   -15.624  77.954   1.523  1.00 28.78       N1+
ATOM   3721  N    TYR A  59   -13.143  80.105  -6.025  1.00 31.81       N
ATOM   3722  CA   TYR A  59   -12.489  81.224  -6.704  1.00 29.74       C
ATOM   3723  C    TYR A  59   -13.349  82.463  -6.543  1.00 31.86       C
ATOM   3724  O    TYR A  59   -14.581  82.379  -6.491  1.00 36.21       O
ATOM   3725  CB   TYR A  59   -12.258  80.990  -8.212  1.00 35.36       C
ATOM   3726  CG   TYR A  59   -11.620  79.662  -8.593  1.00 35.36       C
ATOM   3727  CD1  TYR A  59   -10.873  78.936  -7.693  1.00 49.05       C
ATOM   3728  CD2  TYR A  59   -11.776  79.141  -9.849  1.00 46.01       C
ATOM   3729  CE1  TYR A  59   -10.317  77.708  -8.033  1.00 56.82       C
ATOM   3730  CE2  TYR A  59   -11.212  77.925 -10.198  1.00 50.42       C
ATOM   3731  CZ   TYR A  59   -10.494  77.215  -9.292  1.00 45.11       C
ATOM   3732  OH   TYR A  59    -9.945  76.014  -9.656  1.00 44.41       O
ATOM   3733  N    TYR A  60   -12.706  83.617  -6.491  1.00 28.31       N
ATOM   3734  CA   TYR A  60   -13.418  84.868  -6.277  1.00 31.56       C
ATOM   3735  C    TYR A  60   -12.950  85.928  -7.263  1.00 33.13       C
ATOM   3736  O    TYR A  60   -11.782  85.963  -7.657  1.00 36.90       O
ATOM   3737  CB   TYR A  60   -13.221  85.407  -4.884  1.00 26.95       C
ATOM   3738  CG   TYR A  60   -13.706  84.522  -3.780  1.00 31.53       C
ATOM   3739  CD1  TYR A  60   -12.877  83.520  -3.249  1.00 34.43       C
ATOM   3740  CD2  TYR A  60   -14.958  84.703  -3.225  1.00 29.79       C
ATOM   3741  CE1  TYR A  60   -13.308  82.707  -2.215  1.00 28.05       C
ATOM   3742  CE2  TYR A  60   -15.400  83.896  -2.183  1.00 32.79       C
ATOM   3743  CZ   TYR A  60   -14.567  82.900  -1.679  1.00 33.02       C
ATOM   3744  OH   TYR A  60   -14.995  82.098  -0.641  1.00 31.57       O
ATOM   3745  N    ALA A  61   -13.872  86.804  -7.650  1.00 23.00       N
ATOM   3746  CA   ALA A  61   -13.464  87.976  -8.406  1.00 28.65       C
ATOM   3747  C    ALA A  61   -12.612  88.888  -7.526  1.00 32.06       C
ATOM   3748  O    ALA A  61   -12.759  88.923  -6.301  1.00 30.34       O
ATOM   3749  CB   ALA A  61   -14.679  88.736  -8.937  1.00 25.89       C
ATOM   3750  N    ASP A  62   -11.713  89.633  -8.171  1.00 32.65       N
ATOM   3751  CA   ASP A  62   -10.805  90.522  -7.445  1.00 32.02       C
ATOM   3752  C    ASP A  62   -11.551  91.559  -6.605  1.00 31.30       C
ATOM   3753  O    ASP A  62   -11.125  91.882  -5.492  1.00 33.95       O
ATOM   3754  CB   ASP A  62    -9.856  91.208  -8.428  1.00 30.42       C
ATOM   3755  CG   ASP A  62    -8.574  90.404  -8.671  1.00 42.21       C
ATOM   3756  OD1  ASP A  62    -8.521  89.196  -8.308  1.00 50.07       O
ATOM   3757  OD2  ASP A  62    -7.625  90.972  -9.257  1.00 56.07       O1-
ATOM   3758  N    SER A  63   -12.672  92.082  -7.106  1.00 28.74       N
ATOM   3759  CA   SER A  63   -13.409  93.118  -6.383  1.00 32.42       C
```

```
ATOM   3760   C    SER A   63   -13.980  92.646   -5.048  1.00  39.97        C
ATOM   3761   O    SER A   63   -14.490  93.477   -4.280  1.00  38.00        O
ATOM   3762   CB   SER A   63   -14.542  93.641   -7.263  1.00  35.16        C
ATOM   3763   OG   SER A   63   -15.314  92.561   -7.747  1.00  42.20        O
ATOM   3764   N    VAL A   64   -13.865  91.356   -4.740  1.00  33.69        N
ATOM   3765   CA   VAL A   64   -14.600  90.717   -3.663  1.00  31.03        C
ATOM   3766   C    VAL A   64   -13.681  89.860   -2.780  1.00  35.18        C
ATOM   3767   O    VAL A   64   -14.040  89.490   -1.651  1.00  33.73        O
ATOM   3768   CB   VAL A   64   -15.740  89.918   -4.330  1.00  34.61        C
ATOM   3769   CG1  VAL A   64   -15.732  88.417   -4.001  1.00  22.27        C
ATOM   3770   CG2  VAL A   64   -17.031  90.609   -4.115  1.00  28.14        C
ATOM   3771   N    LYS A   65   -12.476  89.566   -3.273  1.00  30.69        N
ATOM   3772   CA   LYS A   65   -11.498  88.785   -2.511  1.00  32.30        C
ATOM   3773   C    LYS A   65   -11.300  89.377   -1.126  1.00  35.92        C
ATOM   3774   O    LYS A   65   -11.119  90.590   -0.975  1.00  37.34        O
ATOM   3775   CB   LYS A   65   -10.155  88.769   -3.242  1.00  31.37        C
ATOM   3776   CG   LYS A   65   -10.056  87.772   -4.365  1.00  35.93        C
ATOM   3777   CD   LYS A   65    -8.637  87.667   -4.881  1.00  39.14        C
ATOM   3778   CE   LYS A   65    -8.443  86.411   -5.742  1.00  40.69        C
ATOM   3779   NZ   LYS A   65    -9.097  86.500   -7.077  1.00  44.27        N1+
ATOM   3780   N    GLY A   66   -11.300  88.509   -0.118  1.00  36.76        N
ATOM   3781   CA   GLY A   66   -11.109  88.926    1.249  1.00  34.77        C
ATOM   3782   C    GLY A   66   -12.355  89.409    1.957  1.00  37.43        C
ATOM   3783   O    GLY A   66   -12.361  89.462    3.185  1.00  43.41        O
ATOM   3784   N    ARG A   67   -13.407  89.779    1.231  1.00  37.34        N
ATOM   3785   CA   ARG A   67   -14.630  90.281    1.849  1.00  34.16        C
ATOM   3786   C    ARG A   67   -15.789  89.302    1.783  1.00  32.20        C
ATOM   3787   O    ARG A   67   -16.554  89.208    2.745  1.00  35.10        O
ATOM   3788   CB   ARG A   67   -15.047  91.609    1.199  1.00  28.81        C
ATOM   3789   CG   ARG A   67   -14.047  92.745    1.481  1.00  38.29        C
ATOM   3790   CD   ARG A   67   -14.504  94.103    0.942  1.00  39.40        C
ATOM   3791   NE   ARG A   67   -14.857  94.018   -0.470  1.00  34.94        N
ATOM   3792   CZ   ARG A   67   -16.086  94.209   -0.939  1.00  36.27        C
ATOM   3793   NH1  ARG A   67   -17.074  94.532   -0.113  1.00  29.46        N1+
ATOM   3794   NH2  ARG A   67   -16.325  94.083   -2.236  1.00  33.40        N
ATOM   3795   N    PHE A   68   -15.936  88.558    0.688  1.00  31.92        N
ATOM   3796   CA   PHE A   68   -17.046  87.626    0.523  1.00  33.41        C
ATOM   3797   C    PHE A   68   -16.540  86.204    0.697  1.00  34.08        C
ATOM   3798   O    PHE A   68   -15.382  85.903    0.405  1.00  35.01        O
ATOM   3799   CB   PHE A   68   -17.727  87.743   -0.850  1.00  29.41        C
ATOM   3800   CG   PHE A   68   -18.410  89.076   -1.108  1.00  31.18        C
ATOM   3801   CD1  PHE A   68   -18.242  90.156   -0.256  1.00  28.93        C
ATOM   3802   CD2  PHE A   68   -19.236  89.231   -2.206  1.00  29.68        C
ATOM   3803   CE1  PHE A   68   -18.851  91.388   -0.518  1.00  33.26        C
ATOM   3804   CE2  PHE A   68   -19.865  90.444   -2.462  1.00  36.39        C
ATOM   3805   CZ   PHE A   68   -19.660  91.532   -1.618  1.00  35.74        C
ATOM   3806   N    THR A   69   -17.413  85.325    1.177  1.00  33.37        N
ATOM   3807   CA   THR A   69   -17.054  83.917    1.293  1.00  32.05        C
ATOM   3808   C    THR A   69   -18.206  83.085    0.775  1.00  30.76        C
ATOM   3809   O    THR A   69   -19.354  83.292    1.177  1.00  28.56        O
ATOM   3810   CB   THR A   69   -16.720  83.513    2.739  1.00  38.78        C
ATOM   3811   OG1  THR A   69   -15.552  84.216    3.173  1.00  33.71        O
ATOM   3812   CG2  THR A   69   -16.475  81.992    2.856  1.00  30.01        C
ATOM   3813   N    ILE A   70   -17.892  82.169   -0.143  1.00  28.12        N
ATOM   3814   CA   ILE A   70   -18.862  81.246   -0.708  1.00  31.36        C
ATOM   3815   C    ILE A   70   -18.754  79.918    0.040  1.00  32.93        C
ATOM   3816   O    ILE A   70   -17.655  79.472    0.402  1.00  33.29        O
ATOM   3817   CB   ILE A   70   -18.656  81.079   -2.230  1.00  31.45        C
ATOM   3818   CG1  ILE A   70   -19.832  80.302   -2.852  1.00  29.85        C
ATOM   3819   CG2  ILE A   70   -17.257  80.401   -2.554  1.00  29.79        C
ATOM   3820   CD1  ILE A   70   -19.805  80.244   -4.411  1.00  27.30        C
ATOM   3821   N    SER A   71   -19.899  79.300    0.307  1.00  30.39        N
ATOM   3822   CA   SER A   71   -19.935  78.023    1.017  1.00  34.30        C
ATOM   3823   C    SER A   71   -21.233  77.320    0.648  1.00  30.83        C
ATOM   3824   O    SER A   71   -22.137  77.905    0.046  1.00  34.13        O
ATOM   3825   CB   SER A   71   -19.827  78.201    2.544  1.00  28.89        C
ATOM   3826   OG   SER A   71   -20.944  78.930    3.075  1.00  31.85        O
ATOM   3827   N    ARG A   72   -21.333  76.064    1.046  1.00  33.01        N
ATOM   3828   CA   ARG A   72   -22.531  75.307    0.753  1.00  33.51        C
ATOM   3829   C    ARG A   72   -22.820  74.404    1.941  1.00  36.68        C
ATOM   3830   O    ARG A   72   -21.918  74.070    2.713  1.00  32.75        O
```

```
ATOM   3831  CB   ARG A  72    -22.364  74.492  -0.545  1.00  29.96           C
ATOM   3832  CG   ARG A  72    -21.230  73.458  -0.489  1.00  29.86           C
ATOM   3833  CD   ARG A  72    -20.985  72.827  -1.859  1.00  31.26           C
ATOM   3834  NE   ARG A  72    -22.179  72.130  -2.344  1.00  36.14           N
ATOM   3835  CZ   ARG A  72    -22.480  71.942  -3.627  1.00  33.42           C
ATOM   3836  NH1  ARG A  72    -21.683  72.400  -4.575  1.00  31.33           N1+
ATOM   3837  NH2  ARG A  72    -23.599  71.322  -3.972  1.00  33.70           N
ATOM   3838  N    ASP A  73    -24.094  74.046   2.108  1.00  35.23           N
ATOM   3839  CA   ASP A  73    -24.523  73.070   3.119  1.00  36.76           C
ATOM   3840  C    ASP A  73    -25.407  72.032   2.428  1.00  39.51           C
ATOM   3841  O    ASP A  73    -26.597  72.270   2.181  1.00  34.82           O
ATOM   3842  CB   ASP A  73    -25.251  73.761   4.266  1.00  34.22           C
ATOM   3843  CG   ASP A  73    -25.616  72.808   5.397  1.00  42.95           C
ATOM   3844  OD1  ASP A  73    -25.733  71.576   5.175  1.00  42.54           O
ATOM   3845  OD2  ASP A  73    -25.774  73.306   6.532  1.00  48.48           O1-
ATOM   3846  N    ASN A  74    -24.825  70.874   2.117  1.00  37.32           N
ATOM   3847  CA   ASN A  74    -25.569  69.888   1.350  1.00  37.99           C
ATOM   3848  C    ASN A  74    -26.773  69.366   2.126  1.00  38.75           C
ATOM   3849  O    ASN A  74    -27.823  69.104   1.530  1.00  38.15           O
ATOM   3850  CB   ASN A  74    -24.634  68.757   0.923  1.00  38.26           C
ATOM   3851  CG   ASN A  74    -23.691  69.174  -0.228  1.00  43.82           C
ATOM   3852  OD1  ASN A  74    -23.726  70.314  -0.699  1.00  41.08           O
ATOM   3853  ND2  ASN A  74    -22.837  68.252  -0.664  1.00  42.34           N
ATOM   3854  N    SER A  75    -26.678  69.273   3.454  1.00  37.68           N
ATOM   3855  CA   SER A  75    -27.820  68.764   4.205  1.00  40.41           C
ATOM   3856  C    SER A  75    -29.031  69.694   4.108  1.00  39.19           C
ATOM   3857  O    SER A  75    -30.152  69.250   4.331  1.00  38.81           O
ATOM   3858  CB   SER A  75    -27.433  68.533   5.666  1.00  34.54           C
ATOM   3859  OG   SER A  75    -27.418  69.751   6.380  1.00  41.65           O
ATOM   3860  N    LYS A  76    -28.840  70.966   3.765  1.00  39.96           N
ATOM   3861  CA   LYS A  76    -29.947  71.888   3.540  1.00  32.91           C
ATOM   3862  C    LYS A  76    -30.127  72.270   2.076  1.00  33.45           C
ATOM   3863  O    LYS A  76    -30.819  73.255   1.793  1.00  32.73           O
ATOM   3864  CB   LYS A  76    -29.760  73.156   4.371  1.00  34.21           C
ATOM   3865  CG   LYS A  76    -29.536  72.893   5.843  1.00  39.47           C
ATOM   3866  CD   LYS A  76    -29.433  74.203   6.610  1.00  37.51           C
ATOM   3867  CE   LYS A  76    -29.059  73.960   8.060  1.00  36.30           C
ATOM   3868  NZ   LYS A  76    -28.974  75.226   8.812  1.00  53.39           N1+
ATOM   3869  N    ASN A  77    -29.486  71.560   1.142  1.00  34.23           N
ATOM   3870  CA   ASN A  77    -29.510  71.936  -0.275  1.00  34.08           C
ATOM   3871  C    ASN A  77    -29.340  73.439  -0.531  1.00  33.69           C
ATOM   3872  O    ASN A  77    -30.019  73.994  -1.395  1.00  31.88           O
ATOM   3873  CB   ASN A  77    -30.814  71.468  -0.910  1.00  35.11           C
ATOM   3874  CG   ASN A  77    -30.897  69.952  -1.020  1.00  45.26           C
ATOM   3875  OD1  ASN A  77    -29.889  69.267  -1.184  1.00  51.24           O
ATOM   3876  ND2  ASN A  77    -32.095  69.426  -0.909  1.00  53.41           N
ATOM   3877  N    THR A  78    -28.427  74.101   0.184  1.00  28.09           N
ATOM   3878  CA   THR A  78    -28.320  75.550   0.139  1.00  34.16           C
ATOM   3879  C    THR A  78    -26.883  75.999  -0.101  1.00  31.55           C
ATOM   3880  O    THR A  78    -25.941  75.476   0.499  1.00  29.95           O
ATOM   3881  CB   THR A  78    -28.867  76.150   1.432  1.00  32.28           C
ATOM   3882  OG1  THR A  78    -30.230  75.746   1.559  1.00  36.31           O
ATOM   3883  CG2  THR A  78    -28.789  77.662   1.405  1.00  32.86           C
ATOM   3884  N    LEU A  79    -26.747  76.977  -0.989  1.00  33.81           N
ATOM   3885  CA   LEU A  79    -25.515  77.701  -1.273  1.00  30.18           C
ATOM   3886  C    LEU A  79    -25.571  79.060  -0.585  1.00  32.22           C
ATOM   3887  O    LEU A  79    -26.606  79.724  -0.617  1.00  34.07           O
ATOM   3888  CB   LEU A  79    -25.381  77.913  -2.782  1.00  31.42           C
ATOM   3889  CG   LEU A  79    -24.281  78.860  -3.263  1.00  32.10           C
ATOM   3890  CD1  LEU A  79    -22.973  78.101  -3.223  1.00  23.19           C
ATOM   3891  CD2  LEU A  79    -24.601  79.393  -4.668  1.00  27.71           C
ATOM   3892  N    TYR A  80    -24.450  79.498  -0.015  1.00  30.45           N
ATOM   3893  CA   TYR A  80    -24.385  80.758   0.715  1.00  29.65           C
ATOM   3894  C    TYR A  80    -23.352  81.714   0.114  1.00  36.35           C
ATOM   3895  O    TYR A  80    -22.353  81.294  -0.486  1.00  29.45           O
ATOM   3896  CB   TYR A  80    -24.022  80.536   2.194  1.00  29.90           C
ATOM   3897  CG   TYR A  80    -24.972  79.655   2.914  1.00  35.65           C
ATOM   3898  CD1  TYR A  80    -26.209  80.148   3.347  1.00  31.27           C
ATOM   3899  CD2  TYR A  80    -24.656  78.311   3.162  1.00  32.71           C
ATOM   3900  CE1  TYR A  80    -27.109  79.334   4.006  1.00  30.61           C
ATOM   3901  CE2  TYR A  80    -25.548  77.480   3.834  1.00  34.98           C
```

```
ATOM  3902  CZ   TYR A  80  -26.780  77.999   4.253  1.00 43.60      C
ATOM  3903  OH   TYR A  80  -27.691  77.183   4.903  1.00 43.60      O
ATOM  3904  N    LEU A  81  -23.608  83.016   0.286  1.00 33.92      N
ATOM  3905  CA   LEU A  81  -22.616  84.067   0.062  1.00 32.11      C
ATOM  3906  C    LEU A  81  -22.609  84.955   1.294  1.00 35.72      C
ATOM  3907  O    LEU A  81  -23.559  85.712   1.534  1.00 31.37      O
ATOM  3908  CB   LEU A  81  -22.899  84.890  -1.193  1.00 29.72      C
ATOM  3909  CG   LEU A  81  -21.820  85.907  -1.554  1.00 30.90      C
ATOM  3910  CD1  LEU A  81  -20.535  85.193  -2.008  1.00 31.13      C
ATOM  3911  CD2  LEU A  81  -22.311  86.863  -2.620  1.00 29.96      C
ATOM  3912  N    GLN A  82  -21.546  84.836   2.077  1.00 35.14      N
ATOM  3913  CA   GLN A  82  -21.322  85.681   3.236  1.00 35.67      C
ATOM  3914  C    GLN A  82  -20.628  86.948   2.753  1.00 33.89      C
ATOM  3915  O    GLN A  82  -19.504  86.883   2.239  1.00 36.17      O
ATOM  3916  CB   GLN A  82  -20.463  84.940   4.264  1.00 33.97      C
ATOM  3917  CG   GLN A  82  -20.206  85.726   5.526  1.00 35.10      C
ATOM  3918  CD   GLN A  82  -21.509  86.106   6.226  1.00 41.65      C
ATOM  3919  OE1  GLN A  82  -22.419  85.279   6.375  1.00 41.07      O
ATOM  3920  NE2  GLN A  82  -21.598  87.354   6.669  1.00 39.37      N
ATOM  3921  N    MET A  83  -21.290  88.089   2.892  1.00 34.01      N
ATOM  3922  CA   MET A  83  -20.762  89.349   2.360  1.00 40.25      C
ATOM  3923  C    MET A  83  -20.390  90.231   3.540  1.00 34.20      C
ATOM  3924  O    MET A  83  -21.271  90.753   4.231  1.00 43.36      O
ATOM  3925  CB   MET A  83  -21.782  90.054   1.460  1.00 35.88      C
ATOM  3926  CG   MET A  83  -22.247  89.246   0.242  1.00 40.32      C
ATOM  3927  SD   MET A  83  -23.444  90.120  -0.837  1.00 45.75      S
ATOM  3928  CE   MET A  83  -24.869  90.178   0.214  1.00 39.89      C
ATOM  3929  N    ASN A  84  -19.098  90.416   3.767  1.00 34.41      N
ATOM  3930  CA   ASN A  84  -18.649  91.282   4.849  1.00 35.66      C
ATOM  3931  C    ASN A  84  -18.114  92.576   4.266  1.00 35.32      C
ATOM  3932  O    ASN A  84  -17.798  92.660   3.078  1.00 37.11      O
ATOM  3933  CB   ASN A  84  -17.563  90.616   5.694  1.00 33.02      C
ATOM  3934  CG   ASN A  84  -18.033  89.347   6.358  1.00 38.60      C
ATOM  3935  OD1  ASN A  84  -19.196  89.206   6.717  1.00 38.86      O
ATOM  3936  ND2  ASN A  84  -17.127  88.398   6.499  1.00 43.30      N
ATOM  3937  N    SER A  85  -18.051  93.598   5.113  1.00 37.32      N
ATOM  3938  CA   SER A  85  -17.446  94.870   4.742  1.00 40.34      C
ATOM  3939  C    SER A  85  -18.091  95.435   3.483  1.00 36.53      C
ATOM  3940  O    SER A  85  -17.417  95.833   2.536  1.00 42.50      O
ATOM  3941  CB   SER A  85  -15.936  94.707   4.545  1.00 41.25      C
ATOM  3942  OG   SER A  85  -15.303  94.426   5.777  1.00 48.30      O
ATOM  3943  N    LEU A  86  -19.419  95.440   3.469  1.00 36.42      N
ATOM  3944  CA   LEU A  86  -20.121  95.839   2.269  1.00 30.29      C
ATOM  3945  C    LEU A  86  -19.768  97.269   1.898  1.00 37.87      C
ATOM  3946  O    LEU A  86  -19.530  98.126   2.759  1.00 39.88      O
ATOM  3947  CB   LEU A  86  -21.620  95.670   2.458  1.00 37.21      C
ATOM  3948  CG   LEU A  86  -22.026  94.199   2.267  1.00 41.48      C
ATOM  3949  CD1  LEU A  86  -23.420  93.965   2.745  1.00 31.33      C
ATOM  3950  CD2  LEU A  86  -21.912  93.809   0.788  1.00 34.28      C
ATOM  3951  N    ARG A  87  -19.673  97.500   0.601  1.00 31.11      N
ATOM  3952  CA   ARG A  87  -19.397  98.809   0.054  1.00 36.58      C
ATOM  3953  C    ARG A  87  -20.539  99.186  -0.890  1.00 37.14      C
ATOM  3954  O    ARG A  87  -21.233  98.322  -1.439  1.00 30.93      O
ATOM  3955  CB   ARG A  87  -18.047  98.816  -0.688  1.00 36.84      C
ATOM  3956  CG   ARG A  87  -16.843  98.100  -0.017  1.00 39.75      C
ATOM  3957  CD   ARG A  87  -15.620  98.762  -0.560  1.00 50.01      C
ATOM  3958  NE   ARG A  87  -14.715  97.898  -1.313  1.00 57.70      N
ATOM  3959  CZ   ARG A  87  -13.636  97.274  -0.840  1.00 54.84      C
ATOM  3960  NH1  ARG A  87  -13.275  97.378   0.442  1.00 55.39      N1+
ATOM  3961  NH2  ARG A  87  -12.907  96.541  -1.682  1.00 46.24      N
ATOM  3962  N    VAL A  88  -20.706 100.495  -1.088  1.00 33.94      N
ATOM  3963  CA   VAL A  88  -21.786 101.015  -1.927  1.00 35.85      C
ATOM  3964  C    VAL A  88  -21.768 100.348  -3.297  1.00 33.17      C
ATOM  3965  O    VAL A  88  -22.812  99.996  -3.856  1.00 34.21      O
ATOM  3966  CB   VAL A  88  -21.681 102.557  -2.018  1.00 41.36      C
ATOM  3967  CG1  VAL A  88  -22.508 103.125  -3.182  1.00 34.83      C
ATOM  3968  CG2  VAL A  88  -22.191 103.172  -0.697  1.00 32.83      C
ATOM  3969  N    GLU A  89  -20.577 100.142  -3.842  1.00 32.51      N
ATOM  3970  CA   GLU A  89  -20.416  99.535  -5.149  1.00 32.60      C
ATOM  3971  C    GLU A  89  -20.831  98.070  -5.195  1.00 35.92      C
ATOM  3972  O    GLU A  89  -20.914  97.531  -6.303  1.00 32.77      O
```

```
ATOM   3973  CB   GLU A  89   -18.947  99.625  -5.598  1.00 33.36      C
ATOM   3974  CG   GLU A  89   -18.249 100.917  -5.267  1.00 52.01      C
ATOM   3975  CD   GLU A  89   -17.581 100.894  -3.898  1.00 57.81      C
ATOM   3976  OE1  GLU A  89   -18.121 101.544  -2.969  1.00 46.95      O
ATOM   3977  OE2  GLU A  89   -16.513 100.230  -3.762  1.00 68.05      O1-
ATOM   3978  N    ASP A  90   -21.123  97.427  -4.048  1.00 30.72      N
ATOM   3979  CA   ASP A  90   -21.653  96.063  -4.058  1.00 34.90      C
ATOM   3980  C    ASP A  90   -23.142  96.017  -4.360  1.00 35.83      C
ATOM   3981  O    ASP A  90   -23.710  94.912  -4.422  1.00 33.53      O
ATOM   3982  CB   ASP A  90   -21.377  95.351  -2.721  1.00 28.51      C
ATOM   3983  CG   ASP A  90   -19.884  95.173  -2.453  1.00 37.19      C
ATOM   3984  OD1  ASP A  90   -19.142  94.821  -3.411  1.00 36.43      O
ATOM   3985  OD2  ASP A  90   -19.439  95.412  -1.300  1.00 36.43      O1-
ATOM   3986  N    THR A  91   -23.778  97.180  -4.534  1.00 30.48      N
ATOM   3987  CA   THR A  91   -25.207  97.244  -4.843  1.00 34.41      C
ATOM   3988  C    THR A  91   -25.488  96.586  -6.183  1.00 29.60      C
ATOM   3989  O    THR A  91   -24.889  96.958  -7.192  1.00 33.51      O
ATOM   3990  CB   THR A  91   -25.655  98.705  -4.865  1.00 34.98      C
ATOM   3991  OG1  THR A  91   -25.482  99.260  -3.556  1.00 33.04      O
ATOM   3992  CG2  THR A  91   -27.119  98.836  -5.317  1.00 22.08      C
ATOM   3993  N    ALA A  92   -26.384  95.601  -6.196  1.00 26.49      N
ATOM   3994  CA   ALA A  92   -26.628  94.831  -7.418  1.00 33.82      C
ATOM   3995  C    ALA A  92   -27.764  93.858  -7.167  1.00 31.11      C
ATOM   3996  O    ALA A  92   -28.109  93.559  -6.019  1.00 33.45      O
ATOM   3997  CB   ALA A  92   -25.374  94.036  -7.893  1.00 29.95      C
ATOM   3998  N    VAL A  93   -28.318  93.336  -8.255  1.00 27.74      N
ATOM   3999  CA   VAL A  93   -29.052  92.083  -8.161  1.00 27.69      C
ATOM   4000  C    VAL A  93   -28.035  90.949  -8.156  1.00 32.05      C
ATOM   4001  O    VAL A  93   -27.098  90.935  -8.970  1.00 32.98      O
ATOM   4002  CB   VAL A  93   -30.043  91.925  -9.321  1.00 30.90      C
ATOM   4003  CG1  VAL A  93   -30.675  90.530  -9.260  1.00 27.42      C
ATOM   4004  CG2  VAL A  93   -31.106  93.006  -9.272  1.00 22.48      C
ATOM   4005  N    TYR A  94   -28.210  90.005  -7.236  1.00 28.80      N
ATOM   4006  CA   TYR A  94   -27.333  88.851  -7.090  1.00 27.10      C
ATOM   4007  C    TYR A  94   -28.052  87.579  -7.535  1.00 30.77      C
ATOM   4008  O    TYR A  94   -29.164  87.290  -7.070  1.00 32.21      O
ATOM   4009  CB   TYR A  94   -26.849  88.711  -5.643  1.00 25.98      C
ATOM   4010  CG   TYR A  94   -25.744  89.678  -5.299  1.00 35.21      C
ATOM   4011  CD1  TYR A  94   -26.003  91.042  -5.195  1.00 33.48      C
ATOM   4012  CD2  TYR A  94   -24.448  89.235  -5.071  1.00 28.53      C
ATOM   4013  CE1  TYR A  94   -25.011  91.927  -4.887  1.00 31.11      C
ATOM   4014  CE2  TYR A  94   -23.439  90.127  -4.754  1.00 34.57      C
ATOM   4015  CZ   TYR A  94   -23.734  91.482  -4.674  1.00 34.25      C
ATOM   4016  OH   TYR A  94   -22.756  92.410  -4.395  1.00 31.02      O
ATOM   4017  N    TYR A  95   -27.399  86.815  -8.412  1.00 27.50      N
ATOM   4018  CA   TYR A  95   -27.904  85.566  -8.958  1.00 28.28      C
ATOM   4019  C    TYR A  95   -26.990  84.436  -8.531  1.00 30.88      C
ATOM   4020  O    TYR A  95   -25.769  84.610  -8.464  1.00 30.35      O
ATOM   4021  CB   TYR A  95   -27.948  85.576 -10.497  1.00 23.11      C
ATOM   4022  CG   TYR A  95   -28.742  86.673 -11.106  1.00 28.17      C
ATOM   4023  CD1  TYR A  95   -30.120  86.537 -11.313  1.00 35.38      C
ATOM   4024  CD2  TYR A  95   -28.128  87.864 -11.486  1.00 31.26      C
ATOM   4025  CE1  TYR A  95   -30.863  87.559 -11.882  1.00 29.38      C
ATOM   4026  CE2  TYR A  95   -28.854  88.887 -12.059  1.00 27.86      C
ATOM   4027  CZ   TYR A  95   -30.210  88.733 -12.251  1.00 32.29      C
ATOM   4028  OH   TYR A  95   -30.913  89.752 -12.812  1.00 36.01      O
ATOM   4029  N    CYS A  96   -27.576  83.288  -8.237  1.00 28.07      N
ATOM   4030  CA   CYS A  96   -26.795  82.069  -8.201  1.00 31.83      C
ATOM   4031  C    CYS A  96   -27.035  81.314  -9.498  1.00 30.66      C
ATOM   4032  O    CYS A  96   -28.063  81.482 -10.165  1.00 27.69      O
ATOM   4033  CB   CYS A  96   -27.113  81.176  -6.989  1.00 35.63      C
ATOM   4034  SG   CYS A  96   -28.807  80.660  -6.800  1.00 52.39      S
ATOM   4035  N    ALA A  97   -26.055  80.500  -9.860  1.00 25.32      N
ATOM   4036  CA   ALA A  97   -26.171  79.615 -11.005  1.00 29.44      C
ATOM   4037  C    ALA A  97   -25.383  78.346 -10.695  1.00 29.42      C
ATOM   4038  O    ALA A  97   -24.512  78.346  -9.823  1.00 32.47      O
ATOM   4039  CB   ALA A  97   -25.671  80.294 -12.287  1.00 26.70      C
ATOM   4040  N    ASN A  98   -25.693  77.251 -11.396  1.00 25.38      N
ATOM   4041  CA   ASN A  98   -24.856  76.072 -11.236  1.00 31.07      C
ATOM   4042  C    ASN A  98   -23.922  75.893 -12.441  1.00 25.38      C
ATOM   4043  O    ASN A  98   -23.936  76.655 -13.416  1.00 26.89      O
```

```
ATOM   4044  CB  ASN A  98    -25.714  74.832 -10.962  1.00 26.42           C
ATOM   4045  CG  ASN A  98    -26.528  74.385 -12.153  1.00 31.33           C
ATOM   4046  OD1 ASN A  98    -26.444  74.948 -13.257  1.00 31.54           O
ATOM   4047  ND2 ASN A  98    -27.362  73.366 -11.925  1.00 34.14           N
ATOM   4048  N   TRP A  99    -23.088  74.869 -12.372  1.00 27.06           N
ATOM   4049  CA  TRP A  99    -22.251  74.549 -13.521  1.00 30.36           C
ATOM   4050  C   TRP A  99    -22.064  73.046 -13.590  1.00 30.04           C
ATOM   4051  O   TRP A  99    -21.903  72.391 -12.555  1.00 29.39           O
ATOM   4052  CB  TRP A  99    -20.897  75.266 -13.450  1.00 25.45           C
ATOM   4053  CG  TRP A  99    -19.972  74.863 -12.353  1.00 29.81           C
ATOM   4054  CD1 TRP A  99    -19.834  75.449 -11.122  1.00 28.02           C
ATOM   4055  CD2 TRP A  99    -18.997  73.810 -12.408  1.00 26.75           C
ATOM   4056  NE1 TRP A  99    -18.818  74.830 -10.414  1.00 23.44           N
ATOM   4057  CE2 TRP A  99    -18.300  73.816 -11.178  1.00 25.89           C
ATOM   4058  CE3 TRP A  99    -18.634  72.880 -13.385  1.00 28.32           C
ATOM   4059  CZ2 TRP A  99    -17.278  72.904 -10.891  1.00 30.78           C
ATOM   4060  CZ3 TRP A  99    -17.613  71.972 -13.101  1.00 30.19           C
ATOM   4061  CH2 TRP A  99    -16.951  71.992 -11.859  1.00 28.00           C
ATOM   4062  N   TYR A 100    -22.084  72.513 -14.814  1.00 30.79           N
ATOM   4063  CA  TYR A 100    -22.044  71.070 -15.081  1.00 34.23           C
ATOM   4064  C   TYR A 100    -20.741  70.594 -15.703  1.00 36.36           C
ATOM   4065  O   TYR A 100    -20.246  69.521 -15.339  1.00 31.49           O
ATOM   4066  CB  TYR A 100    -23.188  70.657 -16.023  1.00 29.48           C
ATOM   4067  CG  TYR A 100    -24.563  70.844 -15.457  1.00 29.45           C
ATOM   4068  CD1 TYR A 100    -24.812  70.621 -14.106  1.00 32.61           C
ATOM   4069  CD2 TYR A 100    -25.623  71.233 -16.273  1.00 31.46           C
ATOM   4070  CE1 TYR A 100    -26.081  70.796 -13.576  1.00 32.67           C
ATOM   4071  CE2 TYR A 100    -26.904  71.403 -15.756  1.00 30.44           C
ATOM   4072  CZ  TYR A 100    -27.124  71.173 -14.404  1.00 31.95           C
ATOM   4073  OH  TYR A 100    -28.372  71.337 -13.866  1.00 30.17           O
ATOM   4074  N   TYR A 101    -20.183  71.382 -16.626  1.00 29.07           N
ATOM   4075  CA  TYR A 101    -19.125  70.940 -17.527  1.00 29.25           C
ATOM   4076  C   TYR A 101    -17.797  71.608 -17.189  1.00 34.28           C
ATOM   4077  O   TYR A 101    -16.868  70.944 -16.723  1.00 35.11           O
ATOM   4078  CB  TYR A 101    -19.548  71.243 -18.955  1.00 24.67           C
ATOM   4079  CG  TYR A 101    -20.914  70.690 -19.282  1.00 32.07           C
ATOM   4080  CD1 TYR A 101    -21.119  69.317 -19.342  1.00 32.40           C
ATOM   4081  CD2 TYR A 101    -21.996  71.534 -19.549  1.00 27.53           C
ATOM   4082  CE1 TYR A 101    -22.357  68.791 -19.657  1.00 33.92           C
ATOM   4083  CE2 TYR A 101    -23.255  71.019 -19.850  1.00 27.34           C
ATOM   4084  CZ  TYR A 101    -23.419  69.641 -19.902  1.00 34.29           C
ATOM   4085  OH  TYR A 101    -24.622  69.081 -20.215  1.00 35.46           O
ATOM   4086  N   TYR A 102    -17.670  72.911 -17.415  1.00 28.83           N
ATOM   4087  CA  TYR A 102    -16.483  73.631 -16.998  1.00 29.54           C
ATOM   4088  C   TYR A 102    -16.872  74.660 -15.951  1.00 31.00           C
ATOM   4089  O   TYR A 102    -17.957  75.244 -16.004  1.00 33.23           O
ATOM   4090  CB  TYR A 102    -15.761  74.287 -18.187  1.00 27.78           C
ATOM   4091  CG  TYR A 102    -16.664  74.965 -19.187  1.00 30.93           C
ATOM   4092  CD1 TYR A 102    -17.186  74.251 -20.261  1.00 31.23           C
ATOM   4093  CD2 TYR A 102    -16.985  76.326 -19.074  1.00 27.14           C
ATOM   4094  CE1 TYR A 102    -18.004  74.864 -21.204  1.00 29.52           C
ATOM   4095  CE2 TYR A 102    -17.801  76.957 -20.027  1.00 26.32           C
ATOM   4096  CZ  TYR A 102    -18.311  76.217 -21.086  1.00 29.71           C
ATOM   4097  OH  TYR A 102    -19.120  76.803 -22.045  1.00 27.38           O
ATOM   4098  N   TYR A 103    -15.960  74.870 -15.000  1.00 33.17           N
ATOM   4099  CA  TYR A 103    -16.282  75.569 -13.768  1.00 30.18           C
ATOM   4100  C   TYR A 103    -16.585  77.043 -13.983  1.00 30.85           C
ATOM   4101  O   TYR A 103    -17.198  77.659 -13.111  1.00 31.82           O
ATOM   4102  CB  TYR A 103    -15.133  75.420 -12.772  1.00 30.32           C
ATOM   4103  CG  TYR A 103    -13.820  76.094 -13.175  1.00 36.74           C
ATOM   4104  CD1 TYR A 103    -12.864  75.419 -13.922  1.00 34.48           C
ATOM   4105  CD2 TYR A 103    -13.527  77.399 -12.776  1.00 36.39           C
ATOM   4106  CE1 TYR A 103    -11.652  76.031 -14.266  1.00 37.99           C
ATOM   4107  CE2 TYR A 103    -12.332  78.012 -13.120  1.00 34.39           C
ATOM   4108  CZ  TYR A 103    -11.394  77.329 -13.860  1.00 40.90           C
ATOM   4109  OH  TYR A 103    -10.196  77.947 -14.206  1.00 43.10           O
ATOM   4110  N   TYR A 104    -16.189  77.616 -15.112  1.00 27.43           N
ATOM   4111  CA  TYR A 104    -16.416  79.027 -15.373  1.00 29.07           C
ATOM   4112  C   TYR A 104    -17.599  79.242 -16.291  1.00 28.87           C
ATOM   4113  O   TYR A 104    -17.791  80.354 -16.780  1.00 35.12           O
ATOM   4114  CB  TYR A 104    -15.149  79.690 -15.953  1.00 25.68           C
```

```
ATOM   4115  CG   TYR A 104   -14.499  78.931 -17.095  1.00 30.36           C
ATOM   4116  CD1  TYR A 104   -13.573  77.894 -16.846  1.00 29.10           C
ATOM   4117  CD2  TYR A 104   -14.813  79.233 -18.429  1.00 27.13           C
ATOM   4118  CE1  TYR A 104   -12.964  77.181 -17.913  1.00 27.53           C
ATOM   4119  CE2  TYR A 104   -14.225  78.528 -19.494  1.00 27.81           C
ATOM   4120  CZ   TYR A 104   -13.296  77.508 -19.232  1.00 31.37           C
ATOM   4121  OH   TYR A 104   -12.708  76.829 -20.282  1.00 33.25           O
ATOM   4122  N    GLY A 105   -18.385  78.199 -16.546  1.00 33.05           N
ATOM   4123  CA   GLY A 105   -19.642  78.324 -17.262  1.00 28.37           C
ATOM   4124  C    GLY A 105   -20.806  78.259 -16.290  1.00 33.54           C
ATOM   4125  O    GLY A 105   -20.731  77.601 -15.262  1.00 36.54           O
ATOM   4126  N    MET A 106   -21.885  78.955 -16.614  1.00 33.38           N
ATOM   4127  CA   MET A 106   -23.107  78.909 -15.818  1.00 33.92           C
ATOM   4128  C    MET A 106   -24.192  78.210 -16.619  1.00 33.40           C
ATOM   4129  O    MET A 106   -24.516  78.645 -17.727  1.00 41.54           O
ATOM   4130  CB   MET A 106   -23.575  80.314 -15.438  1.00 31.98           C
ATOM   4131  CG   MET A 106   -22.640  81.023 -14.492  1.00 38.84           C
ATOM   4132  SD   MET A 106   -21.690  82.292 -15.342  1.00 39.21           S
ATOM   4133  CE   MET A 106   -20.191  82.280 -14.355  1.00 44.14           C
ATOM   4134  N    ASP A 107   -24.786  77.161 -16.049  1.00 30.51           N
ATOM   4135  CA   ASP A 107   -25.760  76.380 -16.809  1.00 30.12           C
ATOM   4136  C    ASP A 107   -27.213  76.688 -16.421  1.00 31.04           C
ATOM   4137  O    ASP A 107   -28.019  76.977 -17.300  1.00 30.61           O
ATOM   4138  CB   ASP A 107   -25.432  74.882 -16.678  1.00 32.36           C
ATOM   4139  CG   ASP A 107   -24.108  74.509 -17.383  1.00 34.37           C
ATOM   4140  OD1  ASP A 107   -24.105  74.413 -18.627  1.00 32.86           O1-
ATOM   4141  OD2  ASP A 107   -23.060  74.339 -16.705  1.00 36.09           O
ATOM   4142  N    VAL A 108   -27.581  76.603 -15.138  1.00 32.41           N
ATOM   4143  CA   VAL A 108   -28.922  76.954 -14.662  1.00 26.51           C
ATOM   4144  C    VAL A 108   -28.818  78.175 -13.754  1.00 32.76           C
ATOM   4145  O    VAL A 108   -27.911  78.254 -12.920  1.00 31.62           O
ATOM   4146  CB   VAL A 108   -29.590  75.776 -13.915  1.00 28.43           C
ATOM   4147  CG1  VAL A 108   -31.028  76.146 -13.506  1.00 27.65           C
ATOM   4148  CG2  VAL A 108   -29.617  74.516 -14.803  1.00 30.87           C
ATOM   4149  N    TRP A 109   -29.752  79.117 -13.893  1.00 33.08           N
ATOM   4150  CA   TRP A 109   -29.744  80.348 -13.102  1.00 34.02           C
ATOM   4151  C    TRP A 109   -30.981  80.440 -12.208  1.00 33.68           C
ATOM   4152  O    TRP A 109   -32.064  79.966 -12.563  1.00 33.19           O
ATOM   4153  CB   TRP A 109   -29.716  81.605 -13.997  1.00 30.84           C
ATOM   4154  CG   TRP A 109   -28.485  81.762 -14.834  1.00 36.98           C
ATOM   4155  CD1  TRP A 109   -28.097  80.972 -15.893  1.00 30.27           C
ATOM   4156  CD2  TRP A 109   -27.503  82.805 -14.730  1.00 31.30           C
ATOM   4157  NE1  TRP A 109   -26.927  81.459 -16.432  1.00 31.83           N
ATOM   4158  CE2  TRP A 109   -26.534  82.571 -15.731  1.00 29.54           C
ATOM   4159  CE3  TRP A 109   -27.339  83.902 -13.875  1.00 30.44           C
ATOM   4160  CZ2  TRP A 109   -25.409  83.392 -15.896  1.00 31.19           C
ATOM   4161  CZ3  TRP A 109   -26.223  84.723 -14.042  1.00 30.38           C
ATOM   4162  CH2  TRP A 109   -25.271  84.460 -15.043  1.00 29.90           C
ATOM   4163  N    GLY A 110   -30.820  81.113 -11.066  1.00 29.11           N
ATOM   4164  CA   GLY A 110   -31.931  81.457 -10.200  1.00 35.80           C
ATOM   4165  C    GLY A 110   -32.644  82.701 -10.704  1.00 37.01           C
ATOM   4166  O    GLY A 110   -32.532  83.075 -11.871  1.00 37.79           O
ATOM   4167  N    GLN A 111   -33.399  83.358  -9.817  1.00 35.07           N
ATOM   4168  CA   GLN A 111   -34.189  84.495 -10.276  1.00 30.15           C
ATOM   4169  C    GLN A 111   -33.645  85.841  -9.831  1.00 32.38           C
ATOM   4170  O    GLN A 111   -34.080  86.862 -10.372  1.00 33.82           O
ATOM   4171  CB   GLN A 111   -35.644  84.410  -9.787  1.00 37.66           C
ATOM   4172  CG   GLN A 111   -35.874  85.115  -8.454  1.00 36.90           C
ATOM   4173  CD   GLN A 111   -35.642  84.208  -7.281  1.00 42.01           C
ATOM   4174  OE1  GLN A 111   -35.896  82.996  -7.380  1.00 52.02           O
ATOM   4175  NE2  GLN A 111   -35.176  84.777  -6.142  1.00 30.60           N
ATOM   4176  N    GLY A 112   -32.755  85.872  -8.846  1.00 31.52           N
ATOM   4177  CA   GLY A 112   -32.098  87.079  -8.391  1.00 29.27           C
ATOM   4178  C    GLY A 112   -32.707  87.635  -7.114  1.00 30.54           C
ATOM   4179  O    GLY A 112   -33.900  87.487  -6.840  1.00 36.59           O
ATOM   4180  N    THR A 113   -31.873  88.297  -6.326  1.00 32.24           N
ATOM   4181  CA   THR A 113   -32.301  89.057  -5.166  1.00 33.85           C
ATOM   4182  C    THR A 113   -31.459  90.323  -5.122  1.00 32.53           C
ATOM   4183  O    THR A 113   -30.284  90.295  -5.496  1.00 30.25           O
ATOM   4184  CB   THR A 113   -32.150  88.240  -3.878  1.00 31.09           C
ATOM   4185  OG1  THR A 113   -32.645  89.016  -2.780  1.00 36.12           O
```

```
ATOM   4186  CG2 THR A 113    -30.672  87.868  -3.623  1.00 30.38           C
ATOM   4187  N  ATHR A 114    -32.052  91.460  -4.734  0.50 32.93           N
ATOM   4188  CA ATHR A 114    -31.300  92.714  -4.776  0.50 30.39           C
ATOM   4189  C  ATHR A 114    -30.691  93.019  -3.419  0.50 31.37           C
ATOM   4190  O  ATHR A 114    -31.310  92.805  -2.374  0.50 33.91           O
ATOM   4191  CB ATHR A 114    -32.120  93.928  -5.266  0.50 31.38           C
ATOM   4192  OG1ATHR A 114    -31.978  95.061  -4.375  0.50 27.49           O
ATOM   4193  CG2ATHR A 114    -33.533  93.593  -5.476  0.50 26.66           C
ATOM   4194  N  BTHR A 114    -32.061  91.410  -4.642  0.50 32.98           N
ATOM   4195  CA BTHR A 114    -31.413  92.712  -4.641  0.50 30.32           C
ATOM   4196  C  BTHR A 114    -30.650  92.930  -3.345  0.50 31.38           C
ATOM   4197  O  BTHR A 114    -31.141  92.610  -2.260  0.50 34.03           O
ATOM   4198  CB BTHR A 114    -32.434  93.834  -4.810  0.50 30.28           C
ATOM   4199  OG1BTHR A 114    -33.299  93.846  -3.681  0.50 37.62           O
ATOM   4200  CG2BTHR A 114    -33.258  93.627  -6.018  0.50 25.95           C
ATOM   4201  N   VAL A 115    -29.451  93.489  -3.463  1.00 29.38           N
ATOM   4202  CA  VAL A 115    -28.711  93.973  -2.317  1.00 31.80           C
ATOM   4203  C   VAL A 115    -28.460  95.452  -2.583  1.00 32.96           C
ATOM   4204  O   VAL A 115    -27.891  95.811  -3.622  1.00 32.78           O
ATOM   4205  CB  VAL A 115    -27.392  93.209  -2.130  1.00 36.32           C
ATOM   4206  CG1 VAL A 115    -26.569  93.856  -1.020  1.00 35.15           C
ATOM   4207  CG2 VAL A 115    -27.651  91.686  -1.886  1.00 32.11           C
ATOM   4208  N   THR A 116    -28.917  96.307  -1.676  1.00 32.75           N
ATOM   4209  CA  THR A 116    -28.678  97.741  -1.754  1.00 34.57           C
ATOM   4210  C   THR A 116    -27.787  98.139  -0.584  1.00 34.59           C
ATOM   4211  O   THR A 116    -28.121  97.881   0.580  1.00 36.50           O
ATOM   4212  CB  THR A 116    -29.995  98.522  -1.736  1.00 43.36           C
ATOM   4213  OG1 THR A 116    -30.802  98.125  -2.856  1.00 40.71           O
ATOM   4214  CG2 THR A 116    -29.731 100.021  -1.809  1.00 29.94           C
ATOM   4215  N   VAL A 117    -26.649  98.738  -0.890  1.00 34.23           N
ATOM   4216  CA  VAL A 117    -25.719  99.181   0.134  1.00 34.01           C
ATOM   4217  C   VAL A 117    -25.756 100.691   0.076  1.00 33.64           C
ATOM   4218  O   VAL A 117    -25.318 101.293  -0.906  1.00 37.46           O
ATOM   4219  CB  VAL A 117    -24.301  98.631  -0.073  1.00 36.31           C
ATOM   4220  CG1 VAL A 117    -23.392  99.053   1.093  1.00 33.46           C
ATOM   4221  CG2 VAL A 117    -24.317  97.090  -0.232  1.00 29.32           C
ATOM   4222  N   SER A 118    -26.359 101.302   1.081  1.00 39.03           N
ATOM   4223  CA  SER A 118    -26.566 102.738   1.077  1.00 39.96           C
ATOM   4224  C   SER A 118    -26.419 103.243   2.497  1.00 45.19           C
ATOM   4225  O   SER A 118    -26.858 102.579   3.439  1.00 47.46           O
ATOM   4226  CB  SER A 118    -27.961 103.097   0.529  1.00 38.81           C
ATOM   4227  OG  SER A 118    -28.165 104.501   0.460  1.00 48.85           O
ATOM   4228  N   SER A 119    -25.802 104.406   2.653  1.00 46.28           N
ATOM   4229  CA  SER A 119    -25.847 105.090   3.935  1.00 53.97           C
ATOM   4230  C   SER A 119    -26.871 106.217   3.954  1.00 56.65           C
ATOM   4231  O   SER A 119    -26.985 106.913   4.961  1.00 67.20           O
ATOM   4232  CB  SER A 119    -24.468 105.628   4.292  1.00 47.03           C
ATOM   4233  OG  SER A 119    -23.903 106.260   3.168  1.00 54.58           O
ATOM   4234  N   ALA A 120    -27.649 106.385   2.890  1.00 48.46           N
ATOM   4235  CA  ALA A 120    -28.631 107.451   2.863  1.00 46.93           C
ATOM   4236  C   ALA A 120    -29.791 107.119   3.793  1.00 50.99           C
ATOM   4237  O   ALA A 120    -30.104 105.957   4.047  1.00 62.07           O
ATOM   4238  CB  ALA A 120    -29.138 107.672   1.439  1.00 43.79           C
ATOM   4239  N   SER A 121    -30.399 108.159   4.341  1.00 61.71      GZ00 N
ATOM   4240  CA  SER A 121    -31.605 108.063   5.149  1.00 54.23      GZ00 C
ATOM   4241  C   SER A 121    -32.579 109.105   4.648  1.00 47.84      GZ00 C
ATOM   4242  O   SER A 121    -32.202 110.015   3.907  1.00 55.11      GZ00 O
ATOM   4243  CB  SER A 121    -31.338 108.273   6.640  1.00 50.17      GZ00 C
ATOM   4244  OG  SER A 121    -30.386 109.312   6.829  1.00 66.59      GZ00 O
ATOM   4245  N   THR A 122    -33.838 108.938   5.041  1.00 46.14      GZ00 N
ATOM   4246  CA  THR A 122    -34.934 109.776   4.572  1.00 52.42      GZ00 C
ATOM   4247  C   THR A 122    -34.557 111.248   4.510  1.00 56.26      GZ00 C
ATOM   4248  O   THR A 122    -33.924 111.783   5.426  1.00 48.53      GZ00 O
ATOM   4249  CB  THR A 122    -36.129 109.606   5.486  1.00 52.24      GZ00 C
ATOM   4250  OG1 THR A 122    -36.428 108.215   5.593  1.00 57.02      GZ00 O
ATOM   4251  CG2 THR A 122    -37.310 110.342   4.912  1.00 53.18      GZ00 C
ATOM   4252  N   LYS A 123    -34.894 111.876   3.385  1.00 54.75      GZ00 N
ATOM   4253  CA  LYS A 123    -34.659 113.296   3.176  1.00 45.96      GZ00 C
ATOM   4254  C   LYS A 123    -35.645 113.763   2.125  1.00 53.79      GZ00 C
ATOM   4255  O   LYS A 123    -35.799 113.095   1.099  1.00 47.21      GZ00 O
ATOM   4256  CB  LYS A 123    -33.228 113.577   2.727  1.00 48.41      GZ00 C
```

```
ATOM   4257  CG   LYS A 123   -32.969 115.041    2.451  1.00 46.73      GZ00 C
ATOM   4258  CD   LYS A 123   -31.559 115.291    1.972  1.00 52.19      GZ00 C
ATOM   4259  CE   LYS A 123   -31.365 116.750    1.520  1.00 54.71      GZ00 C
ATOM   4260  NZ   LYS A 123   -32.367 117.163    0.481  1.00 49.08      GZ00 N1+
ATOM   4261  N    GLY A 124   -36.311 114.898    2.382  1.00 53.18      GZ00 N
ATOM   4262  CA   GLY A 124   -37.243 115.482    1.440  1.00 35.65      GZ00 C
ATOM   4263  C    GLY A 124   -36.503 116.234    0.347  1.00 38.66      GZ00 C
ATOM   4264  O    GLY A 124   -35.330 116.589    0.499  1.00 39.24      GZ00 O
ATOM   4265  N    PRO A 125   -37.165 116.475   -0.782  1.00 39.30      GZ00 N
ATOM   4266  CA   PRO A 125   -36.474 117.071   -1.933  1.00 45.65      GZ00 C
ATOM   4267  C    PRO A 125   -36.378 118.586   -1.844  1.00 46.64      GZ00 C
ATOM   4268  O    PRO A 125   -37.212 119.253   -1.230  1.00 46.44      GZ00 O
ATOM   4269  CB   PRO A 125   -37.378 116.684   -3.110  1.00 44.97      GZ00 C
ATOM   4270  CG   PRO A 125   -38.764 116.674   -2.504  1.00 34.50      GZ00 C
ATOM   4271  CD   PRO A 125   -38.573 116.145   -1.088  1.00 36.15      GZ00 C
ATOM   4272  N    SER A 126   -35.358 119.126   -2.502  1.00 46.29      GZ00 N
ATOM   4273  CA   SER A 126   -35.329 120.540   -2.858  1.00 46.55      GZ00 C
ATOM   4274  C    SER A 126   -35.926 120.698   -4.247  1.00 51.08      GZ00 C
ATOM   4275  O    SER A 126   -35.646 119.893   -5.139  1.00 52.88      GZ00 O
ATOM   4276  CB   SER A 126   -33.903 121.093   -2.860  1.00 40.26      GZ00 C
ATOM   4277  OG   SER A 126   -33.293 120.973   -1.591  1.00 55.79      GZ00 O
ATOM   4278  N    VAL A 127   -36.744 121.735   -4.438  1.00 47.44      GZ00 N
ATOM   4279  CA   VAL A 127   -37.420 121.956   -5.713  1.00 40.11      GZ00 C
ATOM   4280  C    VAL A 127   -36.980 123.290   -6.314  1.00 43.78      GZ00 C
ATOM   4281  O    VAL A 127   -37.076 124.339   -5.665  1.00 52.62      GZ00 O
ATOM   4282  CB   VAL A 127   -38.948 121.898   -5.553  1.00 45.33      GZ00 C
ATOM   4283  CG1  VAL A 127   -39.618 122.036   -6.908  1.00 44.26      GZ00 C
ATOM   4284  CG2  VAL A 127   -39.359 120.584   -4.873  1.00 41.94      GZ00 C
ATOM   4285  N    PHE A 128   -36.517 123.250   -7.564  1.00 40.88      GZ00 N
ATOM   4286  CA   PHE A 128   -36.074 124.425   -8.285  1.00 43.70      GZ00 C
ATOM   4287  C    PHE A 128   -36.857 124.590   -9.578  1.00 46.15      GZ00 C
ATOM   4288  O    PHE A 128   -37.192 123.598  -10.227  1.00 47.49      GZ00 O
ATOM   4289  CB   PHE A 128   -34.582 124.356   -8.615  1.00 42.40      GZ00 C
ATOM   4290  CG   PHE A 128   -33.714 124.253   -7.406  1.00 49.59      GZ00 C
ATOM   4291  CD1  PHE A 128   -33.404 125.389   -6.666  1.00 47.01      GZ00 C
ATOM   4292  CD2  PHE A 128   -33.188 123.033   -7.012  1.00 45.42      GZ00 C
ATOM   4293  CE1  PHE A 128   -32.604 125.308   -5.538  1.00 45.05      GZ00 C
ATOM   4294  CE2  PHE A 128   -32.379 122.946   -5.890  1.00 55.33      GZ00 C
ATOM   4295  CZ   PHE A 128   -32.084 124.085   -5.152  1.00 51.39      GZ00 C
ATOM   4296  N    PRO A 129   -37.153 125.823   -9.985  1.00 44.34      GZ00 N
ATOM   4297  CA   PRO A 129   -37.894 126.021  -11.233  1.00 46.78      GZ00 C
ATOM   4298  C    PRO A 129   -36.975 125.867  -12.426  1.00 45.16      GZ00 C
ATOM   4299  O    PRO A 129   -35.815 126.280  -12.397  1.00 53.62      GZ00 O
ATOM   4300  CB   PRO A 129   -38.404 127.460  -11.109  1.00 47.94      GZ00 C
ATOM   4301  CG   PRO A 129   -37.334 128.141  -10.336  1.00 40.44      GZ00 C
ATOM   4302  CD   PRO A 129   -36.844 127.103   -9.323  1.00 47.69      GZ00 C
ATOM   4303  N    LEU A 130   -37.486 125.227  -13.465  1.00 44.60      GZ00 N
ATOM   4304  CA   LEU A 130   -36.794 125.147  -14.749  1.00 49.12      GZ00 C
ATOM   4305  C    LEU A 130   -37.528 126.108  -15.677  1.00 54.30      GZ00 C
ATOM   4306  O    LEU A 130   -38.580 125.769  -16.225  1.00 53.88      GZ00 O
ATOM   4307  CB   LEU A 130   -36.764 123.714  -15.283  1.00 52.10      GZ00 C
ATOM   4308  CG   LEU A 130   -36.085 122.707  -14.338  1.00 49.99      GZ00 C
ATOM   4309  CD1  LEU A 130   -36.064 121.284  -14.876  1.00 44.72      GZ00 C
ATOM   4310  CD2  LEU A 130   -34.670 123.167  -14.003  1.00 49.22      GZ00 C
ATOM   4311  N    ALA A 131   -36.979 127.339  -15.828  1.00 51.83      GZ00 N
ATOM   4312  CA   ALA A 131   -37.752 128.405  -16.461  1.00 60.57      GZ00 C
ATOM   4313  C    ALA A 131   -37.777 128.241  -17.979  1.00 58.44      GZ00 C
ATOM   4314  O    ALA A 131   -36.767 127.860  -18.578  1.00 62.01      GZ00 O
ATOM   4315  CB   ALA A 131   -37.182 129.780  -16.118  1.00 52.12      GZ00 C
ATOM   4316  N    PRO A 132   -38.922 128.508  -18.610  1.00 63.89      GZ00 N
ATOM   4317  CA   PRO A 132   -38.974 128.551  -20.076  1.00 60.84      GZ00 C
ATOM   4318  C    PRO A 132   -38.312 129.818  -20.583  1.00 68.50      GZ00 C
ATOM   4319  O    PRO A 132   -38.605 130.917  -20.107  1.00 78.71      GZ00 O
ATOM   4320  CB   PRO A 132   -40.477 128.546  -20.374  1.00 61.98      GZ00 C
ATOM   4321  CG   PRO A 132   -41.076 129.218  -19.186  1.00 59.91      GZ00 C
ATOM   4322  CD   PRO A 132   -40.239 128.787  -18.005  1.00 64.04      GZ00 C
ATOM   4323  N    SER A 133   -37.403 129.660  -21.536  1.00 78.25      GZ00 N
ATOM   4324  CA   SER A 133   -36.712 130.783  -22.151  1.00 90.93      GZ00 C
ATOM   4325  C    SER A 133   -36.986 130.784  -23.647  1.00 95.12      GZ00 C
ATOM   4326  O    SER A 133   -37.432 129.785  -24.219  1.00 92.73      GZ00 O
ATOM   4327  CB   SER A 133   -35.195 130.741  -21.878  1.00 93.56      GZ00 C
```

```
ATOM   4328  OG  SER A 133     -34.721 129.411 -21.727  1.00 92.59      GZ00 O
ATOM   4329  N   SER A 134     -36.724 131.923 -24.279  1.00101.62      GZ00 N
ATOM   4330  CA  SER A 134     -37.027 132.073 -25.696  1.00101.63      GZ00 C
ATOM   4331  C   SER A 134     -35.957 131.408 -26.549  1.00 98.00      GZ00 C
ATOM   4332  O   SER A 134     -36.000 130.197 -26.770  1.00105.90      GZ00 O
ATOM   4333  CB  SER A 134     -37.165 133.548 -26.069  1.00 96.91      GZ00 C
ATOM   4334  OG  SER A 134     -37.994 133.692 -27.209  1.00104.41      GZ00 O
ATOM   4335  N   GLY A 139     -42.971 131.608 -30.259  1.00100.95      GZ00 N
ATOM   4336  CA  GLY A 139     -44.001 130.908 -31.008  1.00110.26      GZ00 C
ATOM   4337  C   GLY A 139     -43.531 129.600 -31.626  1.00116.15      GZ00 C
ATOM   4338  O   GLY A 139     -42.529 129.576 -32.345  1.00129.42      GZ00 O
ATOM   4339  N   GLY A 140     -44.252 128.512 -31.352  1.00102.94      GZ00 N
ATOM   4340  CA  GLY A 140     -45.429 128.577 -30.509  1.00 98.08      GZ00 C
ATOM   4341  C   GLY A 140     -45.549 127.510 -29.435  1.00 93.20      GZ00 C
ATOM   4342  O   GLY A 140     -46.648 127.229 -28.960  1.00 88.91      GZ00 O
ATOM   4343  N   THR A 141     -44.427 126.906 -29.049  1.00 94.52      GZ00 N
ATOM   4344  CA  THR A 141     -44.434 125.891 -28.005  1.00 84.86      GZ00 C
ATOM   4345  C   THR A 141     -43.275 126.163 -27.053  1.00 82.99      GZ00 C
ATOM   4346  O   THR A 141     -42.191 126.566 -27.485  1.00 81.83      GZ00 O
ATOM   4347  CB  THR A 141     -44.353 124.474 -28.595  1.00 90.26      GZ00 C
ATOM   4348  OG1 THR A 141     -44.628 123.513 -27.566  1.00 85.35      GZ00 O
ATOM   4349  CG2 THR A 141     -42.971 124.203 -29.232  1.00 79.54      GZ00 C
ATOM   4350  N   ALA A 142     -43.506 125.953 -25.756  1.00 80.86      GZ00 N
ATOM   4351  CA  ALA A 142     -42.487 126.212 -24.745  1.00 77.11      GZ00 C
ATOM   4352  C   ALA A 142     -42.460 125.082 -23.731  1.00 70.08      GZ00 C
ATOM   4353  O   ALA A 142     -43.508 124.527 -23.382  1.00 69.86      GZ00 O
ATOM   4354  CB  ALA A 142     -42.731 127.539 -24.014  1.00 76.03      GZ00 C
ATOM   4355  N   ALA A 143     -41.262 124.748 -23.262  1.00 59.75      GZ00 N
ATOM   4356  CA  ALA A 143     -41.079 123.717 -22.248  1.00 63.56      GZ00 C
ATOM   4357  C   ALA A 143     -40.608 124.351 -20.947  1.00 58.18      GZ00 C
ATOM   4358  O   ALA A 143     -39.664 125.152 -20.948  1.00 54.35      GZ00 O
ATOM   4359  CB  ALA A 143     -40.072 122.666 -22.710  1.00 62.26      GZ00 C
ATOM   4360  N   LEU A 144     -41.241 123.961 -19.840  1.00 51.02      GZ00 N
ATOM   4361  CA  LEU A 144     -40.864 124.417 -18.507  1.00 54.73      GZ00 C
ATOM   4362  C   LEU A 144     -41.027 123.254 -17.537  1.00 53.32      GZ00 C
ATOM   4363  O   LEU A 144     -41.753 122.293 -17.812  1.00 53.18      GZ00 O
ATOM   4364  CB  LEU A 144     -41.709 125.621 -18.047  1.00 46.73      GZ00 C
ATOM   4365  CG  LEU A 144     -43.215 125.351 -17.951  1.00 56.02      GZ00 C
ATOM   4366  CD1 LEU A 144     -43.665 125.025 -16.521  1.00 52.68      GZ00 C
ATOM   4367  CD2 LEU A 144     -44.014 126.520 -18.513  1.00 58.59      GZ00 C
ATOM   4368  N   GLY A 145     -40.359 123.341 -16.391  1.00 51.88      GZ00 N
ATOM   4369  CA  GLY A 145     -40.399 122.209 -15.497  1.00 48.27      GZ00 C
ATOM   4370  C   GLY A 145     -39.960 122.514 -14.085  1.00 51.16      GZ00 C
ATOM   4371  O   GLY A 145     -39.791 123.673 -13.698  1.00 53.82      GZ00 O
ATOM   4372  N   CYS A 146     -39.798 121.434 -13.315  1.00 44.69      GZ00 N
ATOM   4373  CA  CYS A 146     -39.413 121.470 -11.910  1.00 47.10      GZ00 C
ATOM   4374  C   CYS A 146     -38.297 120.462 -11.680  1.00 45.37      GZ00 C
ATOM   4375  O   CYS A 146     -38.433 119.290 -12.038  1.00 42.62      GZ00 O
ATOM   4376  CB  CYS A 146     -40.597 121.139 -10.990  1.00 47.73      GZ00 C
ATOM   4377  SG  CYS A 146     -41.531 122.590 -10.443  1.00 76.23      GZ00 S
ATOM   4378  N   LEU A 147     -37.207 120.910 -11.080  1.00 47.76      GZ00 N
ATOM   4379  CA  LEU A 147     -36.102 120.037 -10.725  1.00 40.76      GZ00 C
ATOM   4380  C   LEU A 147     -36.281 119.619  -9.279  1.00 43.78      GZ00 C
ATOM   4381  O   LEU A 147     -36.273 120.470  -8.388  1.00 44.48      GZ00 O
ATOM   4382  CB  LEU A 147     -34.767 120.742 -10.938  1.00 37.72      GZ00 C
ATOM   4383  CG  LEU A 147     -33.513 120.047 -10.400  1.00 47.51      GZ00 C
ATOM   4384  CD1 LEU A 147     -33.360 118.653 -10.996  1.00 40.15      GZ00 C
ATOM   4385  CD2 LEU A 147     -32.260 120.918 -10.669  1.00 37.25      GZ00 C
ATOM   4386  N   VAL A 148     -36.396 118.311  -9.046  1.00 48.64      GZ00 N
ATOM   4387  CA  VAL A 148     -36.699 117.743  -7.733  1.00 46.35      GZ00 C
ATOM   4388  C   VAL A 148     -35.441 117.031  -7.252  1.00 49.34      GZ00 C
ATOM   4389  O   VAL A 148     -35.200 115.861  -7.576  1.00 50.72      GZ00 O
ATOM   4390  CB  VAL A 148     -37.896 116.788  -7.790  1.00 38.94      GZ00 C
ATOM   4391  CG1 VAL A 148     -38.234 116.266  -6.410  1.00 40.39      GZ00 C
ATOM   4392  CG2 VAL A 148     -39.087 117.487  -8.380  1.00 40.04      GZ00 C
ATOM   4393  N   LYS A 149     -34.660 117.721  -6.440  1.00 45.78      GZ00 N
ATOM   4394  CA  LYS A 149     -33.291 117.334  -6.155  1.00 45.80      GZ00 C
ATOM   4395  C   LYS A 149     -33.137 116.710  -4.774  1.00 47.10      GZ00 C
ATOM   4396  O   LYS A 149     -33.791 117.126  -3.815  1.00 47.43      GZ00 O
ATOM   4397  CB  LYS A 149     -32.379 118.554  -6.266  1.00 47.24      GZ00 C
ATOM   4398  CG  LYS A 149     -31.174 118.293  -7.097  1.00 56.69      GZ00 C
```

```
ATOM   4399  CD  LYS A 149     -29.975 119.004  -6.564  1.00 59.82      GZ00 C
ATOM   4400  CE  LYS A 149     -30.099 120.481  -6.760  1.00 57.45      GZ00 C
ATOM   4401  NZ  LYS A 149     -28.781 121.117  -6.434  1.00 60.94      GZ00 N1+
ATOM   4402  N   ASP A 150     -32.254 115.710  -4.694  1.00 49.92      GZ00 N
ATOM   4403  CA  ASP A 150     -31.630 115.231  -3.456  1.00 45.59      GZ00 C
ATOM   4404  C   ASP A 150     -32.643 114.736  -2.423  1.00 44.92      GZ00 C
ATOM   4405  O   ASP A 150     -32.679 115.208  -1.289  1.00 51.88      GZ00 O
ATOM   4406  CB  ASP A 150     -30.741 116.314  -2.838  1.00 42.77      GZ00 C
ATOM   4407  CG  ASP A 150     -29.533 116.637  -3.689  1.00 56.92      GZ00 C
ATOM   4408  OD1 ASP A 150     -29.136 115.782  -4.512  1.00 57.01      GZ00 O
ATOM   4409  OD2 ASP A 150     -28.970 117.747  -3.525  1.00 62.51      GZ00 O1-
ATOM   4410  N   TYR A 151     -33.415 113.725  -2.802  1.00 45.00      GZ00 N
ATOM   4411  CA  TYR A 151     -34.367 113.102  -1.894  1.00 49.20      GZ00 C
ATOM   4412  C   TYR A 151     -34.088 111.606  -1.765  1.00 57.02      GZ00 C
ATOM   4413  O   TYR A 151     -33.356 111.009  -2.563  1.00 54.16      GZ00 O
ATOM   4414  CB  TYR A 151     -35.807 113.316  -2.346  1.00 41.84      GZ00 C
ATOM   4415  CG  TYR A 151     -36.146 112.630  -3.642  1.00 53.39      GZ00 C
ATOM   4416  CD1 TYR A 151     -36.579 111.294  -3.672  1.00 58.67      GZ00 C
ATOM   4417  CD2 TYR A 151     -36.045 113.316  -4.845  1.00 47.72      GZ00 C
ATOM   4418  CE1 TYR A 151     -36.898 110.679  -4.879  1.00 55.52      GZ00 C
ATOM   4419  CE2 TYR A 151     -36.365 112.718  -6.041  1.00 47.90      GZ00 C
ATOM   4420  CZ  TYR A 151     -36.782 111.403  -6.063  1.00 54.44      GZ00 C
ATOM   4421  OH  TYR A 151     -37.077 110.834  -7.286  1.00 56.96      GZ00 O
ATOM   4422  N   PHE A 152     -34.688 111.011  -0.737  1.00 52.17      GZ00 N
ATOM   4423  CA  PHE A 152     -34.494 109.613  -0.424  1.00 56.95      GZ00 C
ATOM   4424  C   PHE A 152     -35.543 109.170   0.575  1.00 59.21      GZ00 C
ATOM   4425  O   PHE A 152     -35.877 109.929   1.472  1.00 56.82      GZ00 O
ATOM   4426  CB  PHE A 152     -33.097 109.382   0.144  1.00 56.68      GZ00 C
ATOM   4427  CG  PHE A 152     -32.764 107.946   0.349  1.00 56.75      GZ00 C
ATOM   4428  CD1 PHE A 152     -33.106 107.301   1.528  1.00 57.37      GZ00 C
ATOM   4429  CD2 PHE A 152     -32.097 107.233  -0.640  1.00 61.45      GZ00 C
ATOM   4430  CE1 PHE A 152     -32.796 105.963   1.718  1.00 62.30      GZ00 C
ATOM   4431  CE2 PHE A 152     -31.784 105.899  -0.457  1.00 54.88      GZ00 C
ATOM   4432  CZ  PHE A 152     -32.136 105.258   0.722  1.00 52.68      GZ00 C
ATOM   4433  N   PRO A 153     -36.081 107.947   0.414  1.00 64.15      GZ00 N
ATOM   4434  CA  PRO A 153     -35.890 107.052  -0.728  1.00 60.57      GZ00 C
ATOM   4435  C   PRO A 153     -36.949 107.343  -1.784  1.00 57.01      GZ00 C
ATOM   4436  O   PRO A 153     -37.736 108.261  -1.579  1.00 57.18      GZ00 O
ATOM   4437  CB  PRO A 153     -36.104 105.684  -0.109  1.00 56.69      GZ00 C
ATOM   4438  CG  PRO A 153     -37.250 105.962   0.843  1.00 50.72      GZ00 C
ATOM   4439  CD  PRO A 153     -36.942 107.317   1.434  1.00 59.37      GZ00 C
ATOM   4440  N   GLU A 154     -37.022 106.550  -2.848  1.00 55.69      GZ00 N
ATOM   4441  CA  GLU A 154     -38.086 106.727  -3.833  1.00 52.35      GZ00 C
ATOM   4442  C   GLU A 154     -39.389 106.318  -3.182  1.00 47.39      GZ00 C
ATOM   4443  O   GLU A 154     -39.364 105.619  -2.171  1.00 52.96      GZ00 O
ATOM   4444  CB  GLU A 154     -37.805 105.905  -5.093  1.00 53.56      GZ00 C
ATOM   4445  CG  GLU A 154     -36.530 106.322  -5.818  1.00 58.65      GZ00 C
ATOM   4446  CD  GLU A 154     -36.727 106.415  -7.315  1.00 67.26      GZ00 C
ATOM   4447  OE1 GLU A 154     -36.043 105.662  -8.041  1.00 74.85      GZ00 O
ATOM   4448  OE2 GLU A 154     -37.572 107.227  -7.765  1.00 66.72      GZ00 O1-
ATOM   4449  N   PRO A 155     -40.533 106.779  -3.713  1.00 45.32      GZ00 N
ATOM   4450  CA  PRO A 155     -40.792 107.703  -4.827  1.00 55.68      GZ00 C
ATOM   4451  C   PRO A 155     -41.144 109.144  -4.437  1.00 57.15      GZ00 C
ATOM   4452  O   PRO A 155     -41.413 109.427  -3.272  1.00 61.59      GZ00 O
ATOM   4453  CB  PRO A 155     -42.020 107.086  -5.474  1.00 54.31      GZ00 C
ATOM   4454  CG  PRO A 155     -42.813 106.627  -4.281  1.00 36.78      GZ00 C
ATOM   4455  CD  PRO A 155     -41.792 106.177  -3.235  1.00 36.78      GZ00 C
ATOM   4456  N   VAL A 156     -41.135 110.047  -5.417  1.00 56.20      GZ00 N
ATOM   4457  CA  VAL A 156     -41.884 111.294  -5.339  1.00 56.13      GZ00 C
ATOM   4458  C   VAL A 156     -42.962 111.242  -6.407  1.00 55.59      GZ00 C
ATOM   4459  O   VAL A 156     -42.818 110.570  -7.432  1.00 66.78      GZ00 O
ATOM   4460  CB  VAL A 156     -41.023 112.567  -5.514  1.00 57.46      GZ00 C
ATOM   4461  CG1 VAL A 156     -40.008 112.698  -4.399  1.00 56.24      GZ00 C
ATOM   4462  CG2 VAL A 156     -40.327 112.555  -6.838  1.00 58.46      GZ00 C
ATOM   4463  N   THR A 157     -44.052 111.949  -6.156  1.00 55.63      GZ00 N
ATOM   4464  CA  THR A 157     -45.091 112.189  -7.145  1.00 48.48      GZ00 C
ATOM   4465  C   THR A 157     -45.079 113.673  -7.480  1.00 56.97      GZ00 C
ATOM   4466  O   THR A 157     -44.819 114.506  -6.606  1.00 54.54      GZ00 O
ATOM   4467  CB  THR A 157     -46.464 111.777  -6.608  1.00 58.63      GZ00 C
ATOM   4468  OG1 THR A 157     -46.837 112.659  -5.542  1.00 69.16      GZ00 O
ATOM   4469  CG2 THR A 157     -46.413 110.369  -6.046  1.00 52.02      GZ00 C
```

```
ATOM   4470  N   VAL A 158    -45.317 114.003  -8.747  1.00 52.48      GZ00 N
ATOM   4471  CA  VAL A 158    -45.344 115.385  -9.209  1.00 49.55      GZ00 C
ATOM   4472  C   VAL A 158    -46.619 115.606 -10.001  1.00 53.68      GZ00 C
ATOM   4473  O   VAL A 158    -46.942 114.809 -10.885  1.00 58.24      GZ00 O
ATOM   4474  CB  VAL A 158    -44.131 115.743 -10.087  1.00 53.34      GZ00 C
ATOM   4475  CG1 VAL A 158    -44.181 117.245 -10.447  1.00 47.23      GZ00 C
ATOM   4476  CG2 VAL A 158    -42.819 115.357  -9.407  1.00 50.02      GZ00 C
ATOM   4477  N   SER A 159    -47.337 116.684  -9.693  1.00 53.96      GZ00 N
ATOM   4478  CA  SER A 159    -48.485 117.098 -10.485  1.00 54.02      GZ00 C
ATOM   4479  C   SER A 159    -48.334 118.563 -10.856  1.00 57.99      GZ00 C
ATOM   4480  O   SER A 159    -47.487 119.282 -10.319  1.00 58.35      GZ00 O
ATOM   4481  CB  SER A 159    -49.802 116.870  -9.741  1.00 57.03      GZ00 C
ATOM   4482  OG  SER A 159    -49.841 117.623  -8.548  1.00 64.29      GZ00 O
ATOM   4483  N   TRP A 160    -49.143 119.008 -11.804  1.00 52.93      GZ00 N
ATOM   4484  CA  TRP A 160    -49.127 120.403 -12.198  1.00 53.69      GZ00 C
ATOM   4485  C   TRP A 160    -50.507 120.996 -11.965  1.00 60.22      GZ00 C
ATOM   4486  O   TRP A 160    -51.524 120.353 -12.254  1.00 56.05      GZ00 O
ATOM   4487  CB  TRP A 160    -48.667 120.551 -13.654  1.00 52.01      GZ00 C
ATOM   4488  CG  TRP A 160    -47.199 120.239 -13.791  1.00 61.18      GZ00 C
ATOM   4489  CD1 TRP A 160    -46.630 119.002 -13.982  1.00 51.63      GZ00 C
ATOM   4490  CD2 TRP A 160    -46.108 121.169 -13.703  1.00 51.85      GZ00 C
ATOM   4491  NE1 TRP A 160    -45.260 119.116 -14.035  1.00 51.65      GZ00 N
ATOM   4492  CE2 TRP A 160    -44.914 120.433 -13.871  1.00 55.17      GZ00 C
ATOM   4493  CE3 TRP A 160    -46.025 122.550 -13.513  1.00 49.96      GZ00 C
ATOM   4494  CZ2 TRP A 160    -43.654 121.035 -13.847  1.00 52.75      GZ00 C
ATOM   4495  CZ3 TRP A 160    -44.772 123.148 -13.502  1.00 55.98      GZ00 C
ATOM   4496  CH2 TRP A 160    -43.604 122.389 -13.663  1.00 51.25      GZ00 C
ATOM   4497  N   ASN A 161    -50.521 122.215 -11.417  1.00 59.30      GZ00 N
ATOM   4498  CA  ASN A 161    -51.738 122.946 -11.048  1.00 53.77      GZ00 C
ATOM   4499  C   ASN A 161    -52.737 122.039 -10.336  1.00 58.01      GZ00 C
ATOM   4500  O   ASN A 161    -53.929 122.014 -10.642  1.00 64.68      GZ00 O
ATOM   4501  CB  ASN A 161    -52.353 123.615 -12.267  1.00 42.97      GZ00 C
ATOM   4502  CG  ASN A 161    -51.430 124.645 -12.851  1.00 59.88      GZ00 C
ATOM   4503  OD1 ASN A 161    -50.421 124.988 -12.231  1.00 57.52      GZ00 O
ATOM   4504  ND2 ASN A 161    -51.743 125.141 -14.043  1.00 62.17      GZ00 N
ATOM   4505  N   SER A 162    -52.218 121.280  -9.367  1.00 60.34      GZ00 N
ATOM   4506  CA  SER A 162    -53.022 120.430  -8.483  1.00 66.27      GZ00 C
ATOM   4507  C   SER A 162    -53.790 119.353  -9.244  1.00 71.65      GZ00 C
ATOM   4508  O   SER A 162    -54.873 118.941  -8.821  1.00 79.00      GZ00 O
ATOM   4509  CB  SER A 162    -53.984 121.273  -7.641  1.00 67.29      GZ00 C
ATOM   4510  OG  SER A 162    -53.287 122.322  -6.987  1.00 70.91      GZ00 O
ATOM   4511  N   GLY A 163    -53.224 118.858 -10.349  1.00 70.85      GZ00 N
ATOM   4512  CA  GLY A 163    -53.876 117.868 -11.181  1.00 59.17      GZ00 C
ATOM   4513  C   GLY A 163    -54.598 118.443 -12.385  1.00 68.56      GZ00 C
ATOM   4514  O   GLY A 163    -55.017 117.675 -13.255  1.00 76.49      GZ00 O
ATOM   4515  N   ALA A 164    -54.744 119.772 -12.463  1.00 67.36      GZ00 N
ATOM   4516  CA  ALA A 164    -55.483 120.392 -13.561  1.00 63.96      GZ00 C
ATOM   4517  C   ALA A 164    -54.755 120.239 -14.885  1.00 68.34      GZ00 C
ATOM   4518  O   ALA A 164    -55.391 120.060 -15.927  1.00 75.07      GZ00 O
ATOM   4519  CB  ALA A 164    -55.712 121.879 -13.280  1.00 57.29      GZ00 C
ATOM   4520  N   LEU A 165    -53.431 120.361 -14.874  1.00 70.33      GZ00 N
ATOM   4521  CA  LEU A 165    -52.620 120.265 -16.082  1.00 64.91      GZ00 C
ATOM   4522  C   LEU A 165    -52.055 118.846 -16.163  1.00 69.76      GZ00 C
ATOM   4523  O   LEU A 165    -51.263 118.438 -15.303  1.00 66.93      GZ00 O
ATOM   4524  CB  LEU A 165    -51.521 121.324 -16.062  1.00 64.77      GZ00 C
ATOM   4525  CG  LEU A 165    -50.556 121.397 -17.245  1.00 68.60      GZ00 C
ATOM   4526  CD1 LEU A 165    -51.313 121.483 -18.560  1.00 68.89      GZ00 C
ATOM   4527  CD2 LEU A 165    -49.654 122.612 -17.083  1.00 68.22      GZ00 C
ATOM   4528  N   THR A 166    -52.506 118.076 -17.164  1.00 75.54      GZ00 N
ATOM   4529  CA  THR A 166    -52.042 116.700 -17.363  1.00 72.10      GZ00 C
ATOM   4530  C   THR A 166    -51.554 116.504 -18.792  1.00 68.22      GZ00 C
ATOM   4531  O   THR A 166    -50.624 115.732 -19.042  1.00 71.78      GZ00 O
ATOM   4532  CB  THR A 166    -53.133 115.662 -17.055  1.00 67.33      GZ00 C
ATOM   4533  OG1 THR A 166    -54.242 115.850 -17.938  1.00 72.47      GZ00 O
ATOM   4534  CG2 THR A 166    -53.609 115.751 -15.608  1.00 70.69      GZ00 C
ATOM   4535  N   SER A 167    -52.193 117.175 -19.742  1.00 70.73      GZ00 N
ATOM   4536  CA  SER A 167    -51.742 117.096 -21.121  1.00 77.77      GZ00 C
ATOM   4537  C   SER A 167    -50.357 117.710 -21.242  1.00 66.92      GZ00 C
ATOM   4538  O   SER A 167    -50.104 118.796 -20.717  1.00 69.96      GZ00 O
ATOM   4539  CB  SER A 167    -52.734 117.819 -22.039  1.00 74.39      GZ00 C
ATOM   4540  OG  SER A 167    -52.114 118.270 -23.233  1.00 68.17      GZ00 O
```

```
ATOM   4541  N    GLY A 168   -49.455 117.012 -21.932  1.00 64.19      GZ00 N
ATOM   4542  CA   GLY A 168   -48.125 117.554 -22.141  1.00 62.50      GZ00 C
ATOM   4543  C    GLY A 168   -47.169 117.414 -20.977  1.00 58.51      GZ00 C
ATOM   4544  O    GLY A 168   -46.115 118.053 -20.994  1.00 54.75      GZ00 O
ATOM   4545  N    VAL A 169   -47.512 116.619 -19.963  1.00 59.87      GZ00 N
ATOM   4546  CA   VAL A 169   -46.709 116.442 -18.757  1.00 53.22      GZ00 C
ATOM   4547  C    VAL A 169   -45.837 115.206 -18.906  1.00 57.24      GZ00 C
ATOM   4548  O    VAL A 169   -46.341 114.121 -19.219  1.00 54.95      GZ00 O
ATOM   4549  CB   VAL A 169   -47.605 116.322 -17.514  1.00 52.90      GZ00 C
ATOM   4550  CG1  VAL A 169   -46.789 115.872 -16.311  1.00 50.21      GZ00 C
ATOM   4551  CG2  VAL A 169   -48.279 117.657 -17.237  1.00 56.97      GZ00 C
ATOM   4552  N    HIS A 170   -44.533 115.362 -18.656  1.00 49.30      GZ00 N
ATOM   4553  CA   HIS A 170   -43.571 114.263 -18.742  1.00 46.23      GZ00 C
ATOM   4554  C    HIS A 170   -42.653 114.312 -17.519  1.00 45.18      GZ00 C
ATOM   4555  O    HIS A 170   -41.702 115.095 -17.482  1.00 48.42      GZ00 O
ATOM   4556  CB   HIS A 170   -42.786 114.360 -20.050  1.00 46.88      GZ00 C
ATOM   4557  CG   HIS A 170   -41.995 113.136 -20.400  1.00 50.83      GZ00 C
ATOM   4558  ND1  HIS A 170   -41.756 112.109 -19.507  1.00 51.60      GZ00 N
ATOM   4559  CD2  HIS A 170   -41.374 112.784 -21.551  1.00 45.43      GZ00 C
ATOM   4560  CE1  HIS A 170   -41.020 111.180 -20.092  1.00 44.58      GZ00 C
ATOM   4561  NE2  HIS A 170   -40.776 111.565 -21.332  1.00 49.60      GZ00 N
ATOM   4562  N    THR A 171   -42.927 113.466 -16.529  1.00 43.26      GZ00 N
ATOM   4563  CA   THR A 171   -42.052 113.277 -15.380  1.00 42.92      GZ00 C
ATOM   4564  C    THR A 171   -41.082 112.128 -15.662  1.00 43.51      GZ00 C
ATOM   4565  O    THR A 171   -41.505 111.018 -15.974  1.00 42.46      GZ00 O
ATOM   4566  CB   THR A 171   -42.883 113.022 -14.124  1.00 37.89      GZ00 C
ATOM   4567  OG1  THR A 171   -43.681 114.176 -13.878  1.00 47.19      GZ00 O
ATOM   4568  CG2  THR A 171   -42.006 112.776 -12.906  1.00 32.66      GZ00 C
ATOM   4569  N    PHE A 172   -39.793 112.421 -15.634  1.00 46.02      GZ00 N
ATOM   4570  CA   PHE A 172   -38.722 111.494 -15.965  1.00 45.07      GZ00 C
ATOM   4571  C    PHE A 172   -38.336 110.651 -14.754  1.00 49.93      GZ00 C
ATOM   4572  O    PHE A 172   -38.364 111.141 -13.628  1.00 51.84      GZ00 O
ATOM   4573  CB   PHE A 172   -37.501 112.256 -16.475  1.00 38.72      GZ00 C
ATOM   4574  CG   PHE A 172   -37.680 112.793 -17.855  1.00 43.18      GZ00 C
ATOM   4575  CD1  PHE A 172   -38.599 113.810 -18.104  1.00 42.98      GZ00 C
ATOM   4576  CD2  PHE A 172   -36.942 112.277 -18.917  1.00 40.18      GZ00 C
ATOM   4577  CE1  PHE A 172   -38.782 114.304 -19.389  1.00 43.59      GZ00 C
ATOM   4578  CE2  PHE A 172   -37.108 112.779 -20.216  1.00 44.39      GZ00 C
ATOM   4579  CZ   PHE A 172   -38.030 113.797 -20.449  1.00 45.10      GZ00 C
ATOM   4580  N    PRO A 173   -37.971 109.388 -14.970  1.00 45.23      GZ00 N
ATOM   4581  CA   PRO A 173   -37.427 108.573 -13.877  1.00 46.41      GZ00 C
ATOM   4582  C    PRO A 173   -36.181 109.215 -13.275  1.00 46.24      GZ00 C
ATOM   4583  O    PRO A 173   -35.412 109.890 -13.963  1.00 49.60      GZ00 O
ATOM   4584  CB   PRO A 173   -37.083 107.243 -14.568  1.00 43.84      GZ00 C
ATOM   4585  CG   PRO A 173   -37.966 107.212 -15.777  1.00 42.54      GZ00 C
ATOM   4586  CD   PRO A 173   -38.103 108.633 -16.230  1.00 42.67      GZ00 C
ATOM   4587  N    ALA A 174   -35.971 108.971 -11.983  1.00 43.82      GZ00 N
ATOM   4588  CA   ALA A 174   -34.906 109.615 -11.225  1.00 43.70      GZ00 C
ATOM   4589  C    ALA A 174   -33.520 109.033 -11.547  1.00 43.64      GZ00 C
ATOM   4590  O    ALA A 174   -33.392 107.905 -12.020  1.00 48.77      GZ00 O
ATOM   4591  CB   ALA A 174   -35.195 109.486  -9.733  1.00 46.19      GZ00 C
ATOM   4592  N    VAL A 175   -32.460 109.857 -11.304  1.00 40.72      GZ00 N
ATOM   4593  CA   VAL A 175   -31.066 109.414 -11.216  1.00 40.07      GZ00 C
ATOM   4594  C    VAL A 175   -30.766 109.037  -9.779  1.00 49.10      GZ00 C
ATOM   4595  O    VAL A 175   -31.260 109.680  -8.847  1.00 49.26      GZ00 O
ATOM   4596  CB   VAL A 175   -30.053 110.495 -11.665  1.00 47.05      GZ00 C
ATOM   4597  CG1  VAL A 175   -29.752 110.424 -13.122  1.00 49.41      GZ00 C
ATOM   4598  CG2  VAL A 175   -30.479 111.906 -11.241  1.00 43.16      GZ00 C
ATOM   4599  N    LEU A 176   -29.930 108.011  -9.593  1.00 45.95      GZ00 N
ATOM   4600  CA   LEU A 176   -29.317 107.724  -8.305  1.00 45.89      GZ00 C
ATOM   4601  C    LEU A 176   -27.916 108.315  -8.322  1.00 50.82      GZ00 C
ATOM   4602  O    LEU A 176   -27.048 107.853  -9.066  1.00 64.34      GZ00 O
ATOM   4603  CB   LEU A 176   -29.278 106.231  -8.012  1.00 50.15      GZ00 C
ATOM   4604  CG   LEU A 176   -28.524 105.913  -6.714  1.00 55.35      GZ00 C
ATOM   4605  CD1  LEU A 176   -29.054 106.726  -5.525  1.00 46.25      GZ00 C
ATOM   4606  CD2  LEU A 176   -28.546 104.409  -6.408  1.00 46.32      GZ00 C
ATOM   4607  N    GLN A 177   -27.691 109.322  -7.492  1.00 52.50      GZ00 N
ATOM   4608  CA   GLN A 177   -26.422 110.026  -7.518  1.00 63.27      GZ00 C
ATOM   4609  C    GLN A 177   -25.368 109.285  -6.697  1.00 56.88      GZ00 C
ATOM   4610  O    GLN A 177   -25.678 108.456  -5.831  1.00 51.21      GZ00 O
ATOM   4611  CB   GLN A 177   -26.600 111.448  -6.985  1.00 64.89      GZ00 C
```

```
ATOM   4612  CG   GLN A 177   -27.646 112.247  -7.732  1.00 67.73      GZ00 C
ATOM   4613  CD   GLN A 177   -27.955 113.563  -7.061  1.00 68.75      GZ00 C
ATOM   4614  OE1  GLN A 177   -27.236 114.552  -7.240  1.00 87.07      GZ00 O
ATOM   4615  NE2  GLN A 177   -29.018 113.581  -6.259  1.00 58.03      GZ00 N
ATOM   4616  N    SER A 178   -24.100 109.596  -6.997  1.00 53.78      GZ00 N
ATOM   4617  CA   SER A 178   -22.982 109.070  -6.216  1.00 54.16      GZ00 C
ATOM   4618  C    SER A 178   -23.113 109.421  -4.744  1.00 58.69      GZ00 C
ATOM   4619  O    SER A 178   -22.550 108.730  -3.889  1.00 66.24      GZ00 O
ATOM   4620  CB   SER A 178   -21.648 109.588  -6.769  1.00 54.96      GZ00 C
ATOM   4621  OG   SER A 178   -21.589 111.004  -6.780  1.00 68.48      GZ00 O
ATOM   4622  N    SER A 179   -23.821 110.508  -4.432  1.00 59.99      GZ00 N
ATOM   4623  CA   SER A 179   -24.134 110.871  -3.058  1.00 57.94      GZ00 C
ATOM   4624  C    SER A 179   -25.059 109.870  -2.372  1.00 50.10      GZ00 C
ATOM   4625  O    SER A 179   -25.232 109.952  -1.154  1.00 50.29      GZ00 O
ATOM   4626  CB   SER A 179   -24.787 112.254  -3.030  1.00 52.99      GZ00 C
ATOM   4627  OG   SER A 179   -26.135 112.159  -3.478  1.00 61.58      GZ00 O
ATOM   4628  N    GLY A 180   -25.671 108.949  -3.113  1.00 43.93      GZ00 N
ATOM   4629  CA   GLY A 180   -26.698 108.079  -2.571  1.00 44.88      GZ00 C
ATOM   4630  C    GLY A 180   -28.110 108.631  -2.606  1.00 52.65      GZ00 C
ATOM   4631  O    GLY A 180   -29.037 107.923  -2.196  1.00 57.69      GZ00 O
ATOM   4632  N    LEU A 181   -28.306 109.863  -3.094  1.00 54.40      GZ00 N
ATOM   4633  CA   LEU A 181   -29.609 110.513  -3.171  1.00 55.39      GZ00 C
ATOM   4634  C    LEU A 181   -30.141 110.518  -4.600  1.00 52.55      GZ00 C
ATOM   4635  O    LEU A 181   -29.377 110.492  -5.571  1.00 47.86      GZ00 O
ATOM   4636  CB   LEU A 181   -29.523 111.954  -2.660  1.00 50.22      GZ00 C
ATOM   4637  CG   LEU A 181   -29.108 112.109  -1.207  1.00 54.13      GZ00 C
ATOM   4638  CD1  LEU A 181   -28.919 113.590  -0.837  1.00 45.91      GZ00 C
ATOM   4639  CD2  LEU A 181   -30.175 111.435  -0.357  1.00 50.94      GZ00 C
ATOM   4640  N    TYR A 182   -31.469 110.607  -4.712  1.00 45.18      GZ00 N
ATOM   4641  CA   TYR A 182   -32.159 110.643  -5.991  1.00 44.17      GZ00 C
ATOM   4642  C    TYR A 182   -32.489 112.069  -6.403  1.00 48.41      GZ00 C
ATOM   4643  O    TYR A 182   -32.683 112.946  -5.562  1.00 50.13      GZ00 O
ATOM   4644  CB   TYR A 182   -33.454 109.832  -5.939  1.00 51.31      GZ00 C
ATOM   4645  CG   TYR A 182   -33.222 108.357  -5.719  1.00 51.52      GZ00 C
ATOM   4646  CD1  TYR A 182   -32.971 107.499  -6.796  1.00 53.09      GZ00 C
ATOM   4647  CD2  TYR A 182   -33.255 107.817  -4.441  1.00 49.04      GZ00 C
ATOM   4648  CE1  TYR A 182   -32.746 106.144  -6.595  1.00 57.42      GZ00 C
ATOM   4649  CE2  TYR A 182   -33.037 106.464  -4.231  1.00 58.73      GZ00 C
ATOM   4650  CZ   TYR A 182   -32.784 105.634  -5.305  1.00 56.88      GZ00 C
ATOM   4651  OH   TYR A 182   -32.567 104.299  -5.082  1.00 64.47      GZ00 O
ATOM   4652  N    SER A 183   -32.565 112.285  -7.718  1.00 46.87      GZ00 N
ATOM   4653  CA   SER A 183   -33.102 113.509  -8.298  1.00 44.44      GZ00 C
ATOM   4654  C    SER A 183   -33.911 113.162  -9.541  1.00 46.59      GZ00 C
ATOM   4655  O    SER A 183   -33.582 112.222 -10.262  1.00 44.22      GZ00 O
ATOM   4656  CB   SER A 183   -31.992 114.502  -8.679  1.00 39.93      GZ00 C
ATOM   4657  OG   SER A 183   -31.284 114.978  -7.545  1.00 50.25      GZ00 O
ATOM   4658  N    LEU A 184   -34.945 113.951  -9.821  1.00 43.36      GZ00 N
ATOM   4659  CA   LEU A 184   -35.653 113.810 -11.084  1.00 43.02      GZ00 C
ATOM   4660  C    LEU A 184   -36.113 115.182 -11.552  1.00 47.62      GZ00 C
ATOM   4661  O    LEU A 184   -36.058 116.171 -10.815  1.00 47.17      GZ00 O
ATOM   4662  CB   LEU A 184   -36.840 112.839 -10.975  1.00 38.26      GZ00 C
ATOM   4663  CG   LEU A 184   -38.061 113.103 -10.085  1.00 43.98      GZ00 C
ATOM   4664  CD1  LEU A 184   -38.998 114.216 -10.603  1.00 40.01      GZ00 C
ATOM   4665  CD2  LEU A 184   -38.840 111.795  -9.902  1.00 43.99      GZ00 C
ATOM   4666  N    SER A 185   -36.568 115.229 -12.801  1.00 43.75      GZ00 N
ATOM   4667  CA   SER A 185   -37.198 116.411 -13.358  1.00 38.83      GZ00 C
ATOM   4668  C    SER A 185   -38.565 116.044 -13.909  1.00 43.09      GZ00 C
ATOM   4669  O    SER A 185   -38.803 114.901 -14.312  1.00 45.04      GZ00 O
ATOM   4670  CB   SER A 185   -36.346 117.037 -14.462  1.00 38.03      GZ00 C
ATOM   4671  OG   SER A 185   -35.075 117.365 -13.952  1.00 38.25      GZ00 O
ATOM   4672  N    SER A 186   -39.468 117.023 -13.889  1.00 39.49      GZ00 N
ATOM   4673  CA   SER A 186   -40.767 116.950 -14.544  1.00 41.04      GZ00 C
ATOM   4674  C    SER A 186   -40.899 118.159 -15.449  1.00 42.89      GZ00 C
ATOM   4675  O    SER A 186   -40.584 119.272 -15.032  1.00 44.02      GZ00 O
ATOM   4676  CB   SER A 186   -41.917 116.931 -13.536  1.00 44.24      GZ00 C
ATOM   4677  OG   SER A 186   -43.174 117.016 -14.199  1.00 48.30      GZ00 O
ATOM   4678  N    VAL A 187   -41.298 117.945 -16.698  1.00 47.04      GZ00 N
ATOM   4679  CA   VAL A 187   -41.489 119.062 -17.613  1.00 45.11      GZ00 C
ATOM   4680  C    VAL A 187   -42.849 118.971 -18.275  1.00 51.54      GZ00 C
ATOM   4681  O    VAL A 187   -43.401 117.887 -18.494  1.00 53.22      GZ00 O
ATOM   4682  CB   VAL A 187   -40.385 119.152 -18.666  1.00 43.95      GZ00 C
```

```
ATOM   4683  CG1 VAL A 187     -39.102 119.540 -17.976  1.00 54.28      GZ00 C
ATOM   4684  CG2 VAL A 187     -40.236 117.828 -19.352  1.00 52.33      GZ00 C
ATOM   4685  N   VAL A 188     -43.405 120.132 -18.564  1.00 54.43      GZ00 N
ATOM   4686  CA  VAL A 188     -44.641 120.228 -19.315  1.00 58.17      GZ00 C
ATOM   4687  C   VAL A 188     -44.385 121.161 -20.480  1.00 59.55      GZ00 C
ATOM   4688  O   VAL A 188     -43.710 122.188 -20.330  1.00 58.11      GZ00 O
ATOM   4689  CB  VAL A 188     -45.821 120.710 -18.443  1.00 59.93      GZ00 C
ATOM   4690  CG1 VAL A 188     -45.449 121.979 -17.680  1.00 60.26      GZ00 C
ATOM   4691  CG2 VAL A 188     -47.062 120.912 -19.297  1.00 61.12      GZ00 C
ATOM   4692  N   THR A 189     -44.859 120.768 -21.655  1.00 62.33      GZ00 N
ATOM   4693  CA  THR A 189     -44.814 121.638 -22.815  1.00 66.67      GZ00 C
ATOM   4694  C   THR A 189     -46.153 122.362 -22.930  1.00 69.89      GZ00 C
ATOM   4695  O   THR A 189     -47.220 121.739 -22.815  1.00 62.33      GZ00 O
ATOM   4696  CB  THR A 189     -44.477 120.837 -24.072  1.00 67.75      GZ00 C
ATOM   4697  OG1 THR A 189     -44.384 119.450 -23.731  1.00 69.60      GZ00 O
ATOM   4698  CG2 THR A 189     -43.130 121.278 -24.620  1.00 74.71      GZ00 C
ATOM   4699  N   VAL A 190     -46.084 123.681 -23.102  1.00 66.60      GZ00 N
ATOM   4700  CA  VAL A 190     -47.263 124.547 -23.133  1.00 66.10      GZ00 C
ATOM   4701  C   VAL A 190     -47.136 125.490 -24.322  1.00 73.09      GZ00 C
ATOM   4702  O   VAL A 190     -46.045 125.641 -24.901  1.00 71.87      GZ00 O
ATOM   4703  CB  VAL A 190     -47.425 125.349 -21.818  1.00 64.66      GZ00 C
ATOM   4704  CG1 VAL A 190     -47.569 124.428 -20.610  1.00 58.32      GZ00 C
ATOM   4705  CG2 VAL A 190     -46.261 126.316 -21.640  1.00 59.75      GZ00 C
ATOM   4706  N   PRO A 191     -48.245 126.108 -24.739  1.00 74.14      GZ00 N
ATOM   4707  CA  PRO A 191     -48.160 127.158 -25.763  1.00 74.58      GZ00 C
ATOM   4708  C   PRO A 191     -47.354 128.344 -25.261  1.00 76.75      GZ00 C
ATOM   4709  O   PRO A 191     -47.577 128.839 -24.154  1.00 81.03      GZ00 O
ATOM   4710  CB  PRO A 191     -49.625 127.539 -26.000  1.00 69.86      GZ00 C
ATOM   4711  CG  PRO A 191     -50.402 126.339 -25.563  1.00 73.10      GZ00 C
ATOM   4712  CD  PRO A 191     -49.645 125.802 -24.391  1.00 71.77      GZ00 C
ATOM   4713  N   SER A 192     -46.422 128.813 -26.093  1.00 74.74      GZ00 N
ATOM   4714  CA  SER A 192     -45.569 129.925 -25.684  1.00 84.32      GZ00 C
ATOM   4715  C   SER A 192     -46.375 131.203 -25.449  1.00 87.52      GZ00 C
ATOM   4716  O   SER A 192     -46.000 132.033 -24.611  1.00 90.45      GZ00 O
ATOM   4717  CB  SER A 192     -44.469 130.145 -26.720  1.00 82.62      GZ00 C
ATOM   4718  OG  SER A 192     -45.022 130.471 -27.977  1.00 97.04      GZ00 O
ATOM   4719  N   SER A 193     -47.472 131.392 -26.187  1.00 89.79      GZ00 N
ATOM   4720  CA  SER A 193     -48.307 132.573 -25.998  1.00 89.74      GZ00 C
ATOM   4721  C   SER A 193     -48.972 132.612 -24.626  1.00 93.59      GZ00 C
ATOM   4722  O   SER A 193     -49.320 133.699 -24.148  1.00 95.20      GZ00 O
ATOM   4723  CB  SER A 193     -49.389 132.613 -27.076  1.00 81.94      GZ00 C
ATOM   4724  OG  SER A 193     -50.265 131.503 -26.929  1.00 74.81      GZ00 O
ATOM   4725  N   SER A 194     -49.138 131.462 -23.975  1.00 87.57      GZ00 N
ATOM   4726  CA  SER A 194     -49.771 131.416 -22.662  1.00 89.09      GZ00 C
ATOM   4727  C   SER A 194     -48.823 131.758 -21.516  1.00 91.10      GZ00 C
ATOM   4728  O   SER A 194     -49.278 131.840 -20.368  1.00 89.32      GZ00 O
ATOM   4729  CB  SER A 194     -50.372 130.030 -22.418  1.00 81.55      GZ00 C
ATOM   4730  OG  SER A 194     -49.347 129.061 -22.241  1.00 88.02      GZ00 O
ATOM   4731  N   LEU A 195     -47.528 131.951 -21.791  1.00 87.53      GZ00 N
ATOM   4732  CA  LEU A 195     -46.560 132.107 -20.708  1.00 88.74      GZ00 C
ATOM   4733  C   LEU A 195     -46.827 133.359 -19.882  1.00 89.05      GZ00 C
ATOM   4734  O   LEU A 195     -46.677 133.340 -18.654  1.00 89.42      GZ00 O
ATOM   4735  CB  LEU A 195     -45.137 132.120 -21.267  1.00 85.76      GZ00 C
ATOM   4736  CG  LEU A 195     -44.685 130.772 -21.838  1.00 84.17      GZ00 C
ATOM   4737  CD1 LEU A 195     -43.245 130.846 -22.337  1.00 78.51      GZ00 C
ATOM   4738  CD2 LEU A 195     -44.872 129.643 -20.827  1.00 71.43      GZ00 C
ATOM   4739  N   GLY A 196     -47.240 134.451 -20.529  1.00 92.67      GZ00 N
ATOM   4740  CA  GLY A 196     -47.552 135.661 -19.789  1.00 83.87      GZ00 C
ATOM   4741  C   GLY A 196     -48.867 135.611 -19.045  1.00 82.81      GZ00 C
ATOM   4742  O   GLY A 196     -49.054 136.368 -18.090  1.00 84.91      GZ00 O
ATOM   4743  N   THR A 197     -49.775 134.728 -19.455  1.00 85.57      GZ00 N
ATOM   4744  CA  THR A 197     -51.143 134.692 -18.952  1.00 86.43      GZ00 C
ATOM   4745  C   THR A 197     -51.368 133.631 -17.888  1.00 86.30      GZ00 C
ATOM   4746  O   THR A 197     -52.058 133.892 -16.898  1.00 86.17      GZ00 O
ATOM   4747  CB  THR A 197     -52.121 134.417 -20.097  1.00 86.40      GZ00 C
ATOM   4748  OG1 THR A 197     -51.849 135.303 -21.187  1.00 91.08      GZ00 O
ATOM   4749  CG2 THR A 197     -53.569 134.569 -19.626  1.00 80.19      GZ00 C
ATOM   4750  N   GLN A 198     -50.770 132.454 -18.048  1.00 84.30      GZ00 N
ATOM   4751  CA  GLN A 198     -51.154 131.288 -17.272  1.00 78.44      GZ00 C
ATOM   4752  C   GLN A 198     -50.118 131.004 -16.194  1.00 79.40      GZ00 C
ATOM   4753  O   GLN A 198     -48.910 131.155 -16.412  1.00 78.37      GZ00 O
```

```
ATOM  4754  CB  GLN A 198   -51.315 130.071 -18.184 1.00 76.20      GZ00 C
ATOM  4755  CG  GLN A 198   -51.543 128.762 -17.443 1.00 79.56      GZ00 C
ATOM  4756  CD  GLN A 198   -52.867 128.724 -16.710 1.00 81.18      GZ00 C
ATOM  4757  OE1 GLN A 198   -53.881 129.172 -17.236 1.00 93.21      GZ00 O
ATOM  4758  NE2 GLN A 198   -52.867 128.185 -15.493 1.00 73.89      GZ00 N
ATOM  4759  N   THR A 199   -50.606 130.551 -15.043 1.00 73.01      GZ00 N
ATOM  4760  CA  THR A 199   -49.774 130.248 -13.891 1.00 76.95      GZ00 C
ATOM  4761  C   THR A 199   -49.518 128.747 -13.834 1.00 74.20      GZ00 C
ATOM  4762  O   THR A 199   -50.462 127.944 -13.805 1.00 70.39      GZ00 O
ATOM  4763  CB  THR A 199   -50.445 130.746 -12.610 1.00 71.33      GZ00 C
ATOM  4764  OG1 THR A 199   -50.421 132.179 -12.601 1.00 73.00      GZ00 O
ATOM  4765  CG2 THR A 199   -49.728 130.224 -11.382 1.00 65.14      GZ00 C
ATOM  4766  N   TYR A 200   -48.242 128.376 -13.805 1.00 68.08      GZ00 N
ATOM  4767  CA  TYR A 200   -47.829 126.982 -13.781 1.00 61.58      GZ00 C
ATOM  4768  C   TYR A 200   -47.135 126.697 -12.461 1.00 60.20      GZ00 C
ATOM  4769  O   TYR A 200   -46.093 127.293 -12.153 1.00 57.85      GZ00 O
ATOM  4770  CB  TYR A 200   -46.911 126.674 -14.955 1.00 57.54      GZ00 C
ATOM  4771  CG  TYR A 200   -47.580 126.881 -16.287 1.00 62.50      GZ00 C
ATOM  4772  CD1 TYR A 200   -48.564 126.013 -16.731 1.00 61.12      GZ00 C
ATOM  4773  CD2 TYR A 200   -47.218 127.943 -17.106 1.00 67.26      GZ00 C
ATOM  4774  CE1 TYR A 200   -49.178 126.194 -17.951 1.00 65.39      GZ00 C
ATOM  4775  CE2 TYR A 200   -47.820 128.135 -18.333 1.00 69.26      GZ00 C
ATOM  4776  CZ  TYR A 200   -48.802 127.257 -18.753 1.00 70.05      GZ00 C
ATOM  4777  OH  TYR A 200   -49.409 127.447 -19.979 1.00 69.68      GZ00 O
ATOM  4778  N   ILE A 201   -47.710 125.775 -11.699 1.00 54.42      GZ00 N
ATOM  4779  CA  ILE A 201   -47.224 125.396 -10.384 1.00 55.01      GZ00 C
ATOM  4780  C   ILE A 201   -47.026 123.891 -10.383 1.00 53.67      GZ00 C
ATOM  4781  O   ILE A 201   -47.944 123.147 -10.738 1.00 51.99      GZ00 O
ATOM  4782  CB  ILE A 201   -48.220 125.791  -9.275 1.00 53.52      GZ00 C
ATOM  4783  CG1 ILE A 201   -48.406 127.304  -9.225 1.00 59.92      GZ00 C
ATOM  4784  CG2 ILE A 201   -47.757 125.257  -7.920 1.00 51.50      GZ00 C
ATOM  4785  CD1 ILE A 201   -49.568 127.719  -8.359 1.00 59.25      GZ00 C
ATOM  4786  N   CYS A 202   -45.846 123.434  -9.979 1.00 49.43      GZ00 N
ATOM  4787  CA  CYS A 202   -45.655 122.005  -9.794 1.00 52.57      GZ00 C
ATOM  4788  C   CYS A 202   -45.814 121.642  -8.324 1.00 50.37      GZ00 C
ATOM  4789  O   CYS A 202   -45.364 122.364  -7.433 1.00 52.12      GZ00 O
ATOM  4790  CB  CYS A 202   -44.301 121.549 -10.331 1.00 62.40      GZ00 C
ATOM  4791  SG  CYS A 202   -42.952 121.799  -9.238 1.00 64.20      GZ00 S
ATOM  4792  N   ASN A 203   -46.465 120.516  -8.082 1.00 47.36      GZ00 N
ATOM  4793  CA  ASN A 203   -46.831 120.068  -6.747 1.00 46.99      GZ00 C
ATOM  4794  C   ASN A 203   -46.058 118.791  -6.497 1.00 48.88      GZ00 C
ATOM  4795  O   ASN A 203   -46.287 117.778  -7.163 1.00 57.36      GZ00 O
ATOM  4796  CB  ASN A 203   -48.342 119.850  -6.635 1.00 48.02      GZ00 C
ATOM  4797  CG  ASN A 203   -49.137 120.936  -7.344 1.00 55.33      GZ00 C
ATOM  4798  OD1 ASN A 203   -49.750 120.696  -8.385 1.00 60.59      GZ00 O
ATOM  4799  ND2 ASN A 203   -49.148 122.139  -6.765 1.00 49.46      GZ00 N
ATOM  4800  N   VAL A 204   -45.120 118.858  -5.568 1.00 44.47      GZ00 N
ATOM  4801  CA  VAL A 204   -44.215 117.764  -5.274 1.00 47.87      GZ00 C
ATOM  4802  C   VAL A 204   -44.619 117.151  -3.949 1.00 46.74      GZ00 C
ATOM  4803  O   VAL A 204   -44.770 117.864  -2.952 1.00 50.77      GZ00 O
ATOM  4804  CB  VAL A 204   -42.755 118.248  -5.231 1.00 44.63      GZ00 C
ATOM  4805  CG1 VAL A 204   -41.843 117.090  -4.887 1.00 38.03      GZ00 C
ATOM  4806  CG2 VAL A 204   -42.380 118.903  -6.567 1.00 40.53      GZ00 C
ATOM  4807  N   ASN A 205   -44.767 115.832  -3.928 1.00 48.79      GZ00 N
ATOM  4808  CA  ASN A 205   -45.133 115.123  -2.714 1.00 47.93      GZ00 C
ATOM  4809  C   ASN A 205   -44.153 113.974  -2.534 1.00 49.20      GZ00 C
ATOM  4810  O   ASN A 205   -43.964 113.166  -3.445 1.00 60.40      GZ00 O
ATOM  4811  CB  ASN A 205   -46.588 114.648  -2.781 1.00 53.37      GZ00 C
ATOM  4812  CG  ASN A 205   -47.095 114.117  -1.449 1.00 66.37      GZ00 C
ATOM  4813  OD1 ASN A 205   -46.479 114.336  -0.404 1.00 69.20      GZ00 O
ATOM  4814  ND2 ASN A 205   -48.254 113.468  -1.473 1.00 77.20      GZ00 N
ATOM  4815  N   HIS A 206   -43.476 113.954  -1.396 1.00 45.52      GZ00 N
ATOM  4816  CA  HIS A 206   -42.524 112.919  -1.028 1.00 43.97      GZ00 C
ATOM  4817  C   HIS A 206   -42.971 112.323   0.309 1.00 53.01      GZ00 C
ATOM  4818  O   HIS A 206   -42.520 112.750   1.378 1.00 53.77      GZ00 O
ATOM  4819  CB  HIS A 206   -41.137 113.478  -0.951 1.00 40.28      GZ00 C
ATOM  4820  CG  HIS A 206   -40.104 112.463  -0.585 1.00 48.58      GZ00 C
ATOM  4821  ND1 HIS A 206   -39.422 112.498   0.611 1.00 53.96      GZ00 N
ATOM  4822  CD2 HIS A 206   -39.646 111.374  -1.247 1.00 48.75      GZ00 C
ATOM  4823  CE1 HIS A 206   -38.579 111.482   0.667 1.00 51.43      GZ00 C
ATOM  4824  NE2 HIS A 206   -38.689 110.790  -0.453 1.00 52.90      GZ00 N
```

```
ATOM   4825  N   LYS A 207    -43.829 111.306   0.233  1.00 54.92      GZ00 N
ATOM   4826  CA  LYS A 207    -44.424 110.729   1.434  1.00 48.15      GZ00 C
ATOM   4827  C   LYS A 207    -43.418 110.140   2.423  1.00 52.26      GZ00 C
ATOM   4828  O   LYS A 207    -43.648 110.282   3.635  1.00 59.42      GZ00 O
ATOM   4829  CB  LYS A 207    -45.481 109.710   1.007  1.00 49.63      GZ00 C
ATOM   4830  CG  LYS A 207    -46.665 110.413   0.313  1.00 60.89      GZ00 C
ATOM   4831  CD  LYS A 207    -47.752 109.458  -0.167  1.00 75.35      GZ00 C
ATOM   4832  CE  LYS A 207    -48.892 110.226  -0.848  1.00 81.98      GZ00 C
ATOM   4833  NZ  LYS A 207    -49.961 109.352  -1.445  1.00 90.80      GZ00 N1+
ATOM   4834  N   PRO A 208    -42.313 109.506   2.016  1.00 53.20      GZ00 N
ATOM   4835  CA  PRO A 208    -41.376 108.969   3.026  1.00 54.32      GZ00 C
ATOM   4836  C   PRO A 208    -40.864 109.993   4.034  1.00 57.05      GZ00 C
ATOM   4837  O   PRO A 208    -40.579 109.623   5.178  1.00 57.60      GZ00 O
ATOM   4838  CB  PRO A 208    -40.231 108.409   2.174  1.00 48.60      GZ00 C
ATOM   4839  CG  PRO A 208    -40.871 108.022   0.913  1.00 50.40      GZ00 C
ATOM   4840  CD  PRO A 208    -41.948 109.055   0.660  1.00 50.23      GZ00 C
ATOM   4841  N   SER A 209    -40.691 111.254   3.636  1.00 61.65      GZ00 N
ATOM   4842  CA  SER A 209    -40.273 112.325   4.535  1.00 62.97      GZ00 C
ATOM   4843  C   SER A 209    -41.416 113.260   4.930  1.00 61.87      GZ00 C
ATOM   4844  O   SER A 209    -41.174 114.251   5.630  1.00 60.78      GZ00 O
ATOM   4845  CB  SER A 209    -39.165 113.147   3.879  1.00 59.23      GZ00 C
ATOM   4846  OG  SER A 209    -39.685 113.856   2.755  1.00 55.04      GZ00 O
ATOM   4847  N   ASN A 210    -42.646 112.940   4.539  1.00 55.47      GZ00 N
ATOM   4848  CA  ASN A 210    -43.808 113.822   4.651  1.00 56.64      GZ00 C
ATOM   4849  C   ASN A 210    -43.460 115.261   4.290  1.00 56.98      GZ00 C
ATOM   4850  O   ASN A 210    -43.558 116.181   5.100  1.00 62.92      GZ00 O
ATOM   4851  CB  ASN A 210    -44.398 113.761   6.053  1.00 60.66      GZ00 C
ATOM   4852  CG  ASN A 210    -44.555 112.358   6.550  1.00 60.50      GZ00 C
ATOM   4853  OD1 ASN A 210    -45.556 111.702   6.273  1.00 64.24      GZ00 O
ATOM   4854  ND2 ASN A 210    -43.562 111.880   7.288  1.00 60.19      GZ00 N
ATOM   4855  N   THR A 211    -43.011 115.442   3.057  1.00 54.32      GZ00 N
ATOM   4856  CA  THR A 211    -42.767 116.765   2.504  1.00 47.32      GZ00 C
ATOM   4857  C   THR A 211    -43.723 117.022   1.349  1.00 47.89      GZ00 C
ATOM   4858  O   THR A 211    -43.910 116.159   0.489  1.00 54.45      GZ00 O
ATOM   4859  CB  THR A 211    -41.317 116.913   2.046  1.00 49.88      GZ00 C
ATOM   4860  OG1 THR A 211    -40.444 116.645   3.148  1.00 51.07      GZ00 O
ATOM   4861  CG2 THR A 211    -41.061 118.318   1.560  1.00 42.66      GZ00 C
ATOM   4862  N   LYS A 212    -44.338 118.194   1.339  1.00 50.89      GZ00 N
ATOM   4863  CA  LYS A 212    -45.112 118.659   0.198  1.00 47.65      GZ00 C
ATOM   4864  C   LYS A 212    -44.570 120.029  -0.168  1.00 47.72      GZ00 C
ATOM   4865  O   LYS A 212    -44.386 120.878   0.712  1.00 52.21      GZ00 O
ATOM   4866  CB  LYS A 212    -46.611 118.715   0.514  1.00 52.77      GZ00 C
ATOM   4867  CG  LYS A 212    -47.140 117.383   1.052  1.00 63.53      GZ00 C
ATOM   4868  CD  LYS A 212    -48.662 117.295   1.167  1.00 63.25      GZ00 C
ATOM   4869  CE  LYS A 212    -49.049 116.010   1.920  1.00 71.43      GZ00 C
ATOM   4870  NZ  LYS A 212    -50.290 115.345   1.415  1.00 66.03      GZ00 N1+
ATOM   4871  N   VAL A 213    -44.223 120.212  -1.437  1.00 41.63      GZ00 N
ATOM   4872  CA  VAL A 213    -43.729 121.485  -1.935  1.00 44.71      GZ00 C
ATOM   4873  C   VAL A 213    -44.585 121.894  -3.121  1.00 48.49      GZ00 C
ATOM   4874  O   VAL A 213    -44.882 121.063  -3.983  1.00 53.14      GZ00 O
ATOM   4875  CB  VAL A 213    -42.246 121.419  -2.347  1.00 43.73      GZ00 C
ATOM   4876  CG1 VAL A 213    -41.816 122.764  -2.924  1.00 39.48      GZ00 C
ATOM   4877  CG2 VAL A 213    -41.362 121.029  -1.165  1.00 37.84      GZ00 C
ATOM   4878  N   ASP A 214    -45.015 123.156  -3.139  1.00 47.97      GZ00 N
ATOM   4879  CA  ASP A 214    -45.585 123.795  -4.317  1.00 44.59      GZ00 C
ATOM   4880  C   ASP A 214    -44.598 124.838  -4.810  1.00 46.54      GZ00 C
ATOM   4881  O   ASP A 214    -44.025 125.579  -4.009  1.00 54.01      GZ00 O
ATOM   4882  CB  ASP A 214    -46.929 124.477  -4.017  1.00 51.53      GZ00 C
ATOM   4883  CG  ASP A 214    -47.982 123.512  -3.513  1.00 63.26      GZ00 C
ATOM   4884  OD2 ASP A 214    -48.796 123.914  -2.654  1.00 87.44      GZ00 O1-
ATOM   4885  OD1 ASP A 214    -48.012 122.352  -3.969  1.00 69.85      GZ00 O
ATOM   4886  N   LYS A 215    -44.377 124.888  -6.119  1.00 44.93      GZ00 N
ATOM   4887  CA  LYS A 215    -43.442 125.855  -6.679  1.00 46.63      GZ00 C
ATOM   4888  C   LYS A 215    -43.978 126.399  -7.988  1.00 52.58      GZ00 C
ATOM   4889  O   LYS A 215    -44.348 125.626  -8.878  1.00 52.15      GZ00 O
ATOM   4890  CB  LYS A 215    -42.051 125.248  -6.899  1.00 47.58      GZ00 C
ATOM   4891  CG  LYS A 215    -40.969 125.964  -6.129  1.00 50.19      GZ00 C
ATOM   4892  CD  LYS A 215    -40.152 126.858  -7.027  1.00 48.08      GZ00 C
ATOM   4893  CE  LYS A 215    -39.413 127.923  -6.211  1.00 55.88      GZ00 C
ATOM   4894  NZ  LYS A 215    -38.638 127.415  -5.023  1.00 63.59      GZ00 N1+
ATOM   4895  N   LYS A 216    -44.024 127.727  -8.093  1.00 50.59      GZ00 N
```

```
ATOM   4896  CA  LYS A 216     -44.437 128.396  -9.312  1.00 51.04      GZ00 C
ATOM   4897  C   LYS A 216     -43.241 128.550 -10.244  1.00 53.52      GZ00 C
ATOM   4898  O   LYS A 216     -42.135 128.880  -9.807  1.00 49.36      GZ00 O
ATOM   4899  CB  LYS A 216     -45.052 129.760  -8.996  1.00 56.62      GZ00 C
ATOM   4900  CG  LYS A 216     -45.566 130.514 -10.215  1.00 61.12      GZ00 C
ATOM   4901  CD  LYS A 216     -46.136 131.864  -9.823  1.00 67.36      GZ00 C
ATOM   4902  CE  LYS A 216     -46.919 132.483 -10.962  1.00 66.87      GZ00 C
ATOM   4903  NZ  LYS A 216     -47.732 133.632 -10.483  1.00 75.15      GZ00 N1+
ATOM   4904  N   VAL A 217     -43.462 128.277 -11.526  1.00 51.79      GZ00 N
ATOM   4905  CA  VAL A 217     -42.414 128.342 -12.536  1.00 53.46      GZ00 C
ATOM   4906  C   VAL A 217     -42.741 129.494 -13.465  1.00 54.54      GZ00 C
ATOM   4907  O   VAL A 217     -43.747 129.455 -14.179  1.00 59.89      GZ00 O
ATOM   4908  CB  VAL A 217     -42.292 127.021 -13.308  1.00 55.15      GZ00 C
ATOM   4909  CG1 VAL A 217     -41.110 127.076 -14.255  1.00 49.43      GZ00 C
ATOM   4910  CG2 VAL A 217     -42.184 125.845 -12.330  1.00 40.65      GZ00 C
ATOM   4911  N   GLU A 218     -41.905 130.525 -13.455  1.00 58.16      GZ00 N
ATOM   4912  CA  GLU A 218     -42.169 131.714 -14.244  1.00 61.44      GZ00 C
ATOM   4913  C   GLU A 218     -41.064 131.965 -15.263  1.00 73.28      GZ00 C
ATOM   4914  O   GLU A 218     -39.904 131.589 -15.043  1.00 69.38      GZ00 O
ATOM   4915  CB  GLU A 218     -42.312 132.955 -13.350  1.00 65.18      GZ00 C
ATOM   4916  CG  GLU A 218     -43.436 132.867 -12.323  1.00 71.84      GZ00 C
ATOM   4917  CD  GLU A 218     -43.518 134.104 -11.427  1.00 80.98      GZ00 O
ATOM   4918  OE1 GLU A 218     -42.537 134.884 -11.391  1.00 69.24      GZ00 O
ATOM   4919  OE2 GLU A 218     -44.568 134.295 -10.769  1.00 78.83      GZ00 O1-
ATOM   4920  N   PRO A 219     -41.401 132.592 -16.400  1.00 72.81      GZ00 N
ATOM   4921  CA  PRO A 219     -40.374 133.004 -17.371  1.00 73.28      GZ00 C
ATOM   4922  C   PRO A 219     -39.387 134.017 -16.815  1.00 77.52      GZ00 C
ATOM   4923  O   PRO A 219     -39.500 134.420 -15.653  1.00 81.78      GZ00 O
ATOM   4924  CB  PRO A 219     -41.194 133.614 -18.517  1.00 73.25      GZ00 C
ATOM   4925  CG  PRO A 219     -42.619 133.679 -18.024  1.00 67.58      GZ00 C
ATOM   4926  CD  PRO A 219     -42.759 132.663 -16.958  1.00 67.19      GZ00 C
ATOM   4927  N   LYS A 220     -38.405 134.405 -17.627  1.00 81.29      GZ00 N
ATOM   4928  CA  LYS A 220     -37.376 135.375 -17.234  1.00 82.90      GZ00 C
ATOM   4929  C   LYS A 220     -36.507 134.815 -16.117  1.00 88.96      GZ00 C
ATOM   4930  O   LYS A 220     -36.186 133.626 -16.112  1.00 89.60      GZ00 O
ATOM   4931  CB  LYS A 220     -38.003 136.710 -16.809  1.00 83.44      GZ00 C
ATOM   4932  CG  LYS A 220     -37.004 137.772 -16.372  1.00 79.50      GZ00 C
ATOM   4933  CD  LYS A 220     -36.117 138.217 -17.516  1.00 78.87      GZ00 C
ATOM   4934  CE  LYS A 220     -35.095 139.231 -17.031  1.00 76.44      GZ00 C
ATOM   4935  NZ  LYS A 220     -34.316 139.811 -18.154  1.00 72.11      GZ00 N1+
TER
ATOM   4936  N   GLN X   1     -13.182  99.213 -18.301  1.00 76.62           N
ATOM   4937  CA  GLN X   1     -13.330  98.583 -19.611  1.00 76.63           C
ATOM   4938  C   GLN X   1     -13.561  97.073 -19.505  1.00 74.72           C
ATOM   4939  O   GLN X   1     -12.891  96.390 -18.727  1.00 80.15           O
ATOM   4940  CB  GLN X   1     -12.095  98.863 -20.464  1.00 81.35           C
ATOM   4941  CG  GLN X   1     -11.908 100.324 -20.759  1.00 87.31           C
ATOM   4942  CD  GLN X   1     -13.144 100.930 -21.400  1.00101.98           C
ATOM   4943  OE1 GLN X   1     -13.815 101.779 -20.806  1.00102.82           O
ATOM   4944  NE2 GLN X   1     -13.454 100.491 -22.619  1.00 97.35           N
ATOM   4945  N   SER X   2     -14.506  96.551 -20.287  1.00 67.44           N
ATOM   4946  CA  SER X   2     -14.749  95.113 -20.298  1.00 60.09           C
ATOM   4947  C   SER X   2     -13.572  94.377 -20.927  1.00 57.36           C
ATOM   4948  O   SER X   2     -12.944  94.860 -21.875  1.00 53.67           O
ATOM   4949  CB  SER X   2     -16.026  94.785 -21.086  1.00 46.51           C
ATOM   4950  OG  SER X   2     -17.130  94.586 -20.224  1.00 58.04           O
ATOM   4951  N   VAL X   3     -13.284  93.183 -20.403  1.00 47.89           N
ATOM   4952  CA  VAL X   3     -12.146  92.428 -20.923  1.00 46.54           C
ATOM   4953  C   VAL X   3     -12.475  91.869 -22.301  1.00 47.23           C
ATOM   4954  O   VAL X   3     -11.622  91.855 -23.201  1.00 45.87           O
ATOM   4955  CB  VAL X   3     -11.731  91.309 -19.951  1.00 44.31           C
ATOM   4956  CG1 VAL X   3     -10.685  90.401 -20.593  1.00 34.16           C
ATOM   4957  CG2 VAL X   3     -11.202  91.895 -18.640  1.00 39.33           C
ATOM   4958  N   LEU X   4     -13.716  91.408 -22.490  1.00 42.72           N
ATOM   4959  CA  LEU X   4     -14.234  91.004 -23.790  1.00 40.50           C
ATOM   4960  C   LEU X   4     -15.023  92.164 -24.377  1.00 38.73           C
ATOM   4961  O   LEU X   4     -15.756  92.844 -23.656  1.00 38.68           O
ATOM   4962  CB  LEU X   4     -15.120  89.761 -23.666  1.00 32.58           C
ATOM   4963  CG  LEU X   4     -14.559  88.652 -22.763  1.00 35.07           C
ATOM   4964  CD1 LEU X   4     -15.572  87.502 -22.591  1.00 33.11           C
ATOM   4965  CD2 LEU X   4     -13.193  88.130 -23.260  1.00 26.20           C
```

| ATOM | 4966 | N   | THR X | 5  | -14.874 | 92.394 | -25.683 | 1.00 | 30.86 | N |
| ATOM | 4967 | CA  | THR X | 5  | -15.506 | 93.541 | -26.318 | 1.00 | 31.01 | C |
| ATOM | 4968 | C   | THR X | 5  | -16.635 | 93.087 | -27.228 | 1.00 | 32.92 | C |
| ATOM | 4969 | O   | THR X | 5  | -16.412 | 92.301 | -28.156 | 1.00 | 34.89 | O |
| ATOM | 4970 | CB  | THR X | 5  | -14.488 | 94.361 | -27.114 | 1.00 | 35.46 | C |
| ATOM | 4971 | OG1 | THR X | 5  | -13.373 | 94.678 | -26.280 | 1.00 | 37.79 | O |
| ATOM | 4972 | CG2 | THR X | 5  | -15.130 | 95.668 | -27.583 | 1.00 | 23.38 | C |
| ATOM | 4973 | N   | GLN X | 6  | -17.836 | 93.608 | -26.972 | 1.00 | 31.00 | N |
| ATOM | 4974 | CA  | GLN X | 6  | -19.060 | 93.406 | -27.730 | 1.00 | 33.70 | C |
| ATOM | 4975 | C   | GLN X | 6  | -19.549 | 94.756 | -28.224 | 1.00 | 33.00 | C |
| ATOM | 4976 | O   | GLN X | 6  | -19.399 | 95.759 | -27.519 | 1.00 | 30.42 | O |
| ATOM | 4977 | CB  | GLN X | 6  | -20.188 | 92.765 | -26.892 | 1.00 | 30.43 | C |
| ATOM | 4978 | CG  | GLN X | 6  | -19.910 | 91.392 | -26.335 | 1.00 | 28.80 | C |
| ATOM | 4979 | CD  | GLN X | 6  | -21.049 | 90.852 | -25.456 | 1.00 | 32.96 | C |
| ATOM | 4980 | OE1 | GLN X | 6  | -20.823 | 90.407 | -24.329 | 1.00 | 32.89 | O |
| ATOM | 4981 | NE2 | GLN X | 6  | -22.266 | 90.879 | -25.978 | 1.00 | 26.46 | N |
| ATOM | 4982 | N   | PRO X | 7  | -20.179 | 94.813 | -29.392 | 1.00 | 28.38 | N |
| ATOM | 4983 | CA  | PRO X | 7  | -20.880 | 96.043 | -29.796 | 1.00 | 33.26 | C |
| ATOM | 4984 | C   | PRO X | 7  | -21.977 | 96.356 | -28.791 | 1.00 | 38.21 | C |
| ATOM | 4985 | O   | PRO X | 7  | -22.613 | 95.435 | -28.252 | 1.00 | 35.17 | O |
| ATOM | 4986 | CB  | PRO X | 7  | -21.461 | 95.693 | -31.181 | 1.00 | 33.21 | C |
| ATOM | 4987 | CG  | PRO X | 7  | -21.607 | 94.178 | -31.146 | 1.00 | 34.98 | C |
| ATOM | 4988 | CD  | PRO X | 7  | -20.480 | 93.673 | -30.276 | 1.00 | 33.91 | C |
| ATOM | 4989 | N   | PRO X | 8  | -22.189 | 97.632 | -28.468 | 1.00 | 34.91 | N |
| ATOM | 4990 | CA  | PRO X | 8  | -23.156 | 97.962 | -27.407 | 1.00 | 29.83 | C |
| ATOM | 4991 | C   | PRO X | 8  | -24.604 | 97.731 | -27.792 | 1.00 | 37.56 | C |
| ATOM | 4992 | O   | PRO X | 8  | -25.412 | 97.420 | -26.908 | 1.00 | 36.02 | O |
| ATOM | 4993 | CB  | PRO X | 8  | -22.879 | 99.443 | -27.119 | 1.00 | 28.75 | C |
| ATOM | 4994 | CG  | PRO X | 8  | -22.237 | 99.952 | -28.379 | 1.00 | 41.44 | C |
| ATOM | 4995 | CD  | PRO X | 8  | -21.416 | 98.803 | -28.911 | 1.00 | 31.17 | C |
| ATOM | 4996 | N   | SER X | 9  | -24.980 | 97.891 | -29.057 | 1.00 | 30.64 | N |
| ATOM | 4997 | CA  | SER X | 9  | -26.373 | 97.657 | -29.406 | 1.00 | 33.12 | C |
| ATOM | 4998 | C   | SER X | 9  | -26.487 | 97.243 | -30.864 | 1.00 | 36.96 | C |
| ATOM | 4999 | O   | SER X | 9  | -25.582 | 97.451 | -31.685 | 1.00 | 34.35 | O |
| ATOM | 5000 | CB  | SER X | 9  | -27.257 | 98.873 | -29.146 | 1.00 | 35.68 | C |
| ATOM | 5001 | OG  | SER X | 9  | -26.883 | 99.958 | -29.965 | 1.00 | 42.47 | O |
| ATOM | 5002 | N   | VAL X | 10 | -27.628 | 96.644 | -31.164 | 1.00 | 33.72 | N |
| ATOM | 5003 | CA  | VAL X | 10 | -27.851 | 95.977 | -32.431 | 1.00 | 34.33 | C |
| ATOM | 5004 | C   | VAL X | 10 | -29.358 | 95.932 | -32.642 | 1.00 | 34.97 | C |
| ATOM | 5005 | O   | VAL X | 10 | -30.118 | 95.725 | -31.688 | 1.00 | 36.04 | O |
| ATOM | 5006 | CB  | VAL X | 10 | -27.170 | 94.585 | -32.397 | 1.00 | 36.02 | C |
| ATOM | 5007 | CG1 | VAL X | 10 | -28.071 | 93.486 | -32.875 | 1.00 | 38.30 | C |
| ATOM | 5008 | CG2 | VAL X | 10 | -25.823 | 94.625 | -33.148 | 1.00 | 34.46 | C |
| ATOM | 5009 | N   | SER X | 11 | -29.801 | 96.196 | -33.872 | 1.00 | 35.57 | N |
| ATOM | 5010 | CA  | SER X | 11 | -31.238 | 96.220 | -34.114 | 1.00 | 38.43 | C |
| ATOM | 5011 | C   | SER X | 11 | -31.565 | 95.782 | -35.531 | 1.00 | 38.48 | C |
| ATOM | 5012 | O   | SER X | 11 | -30.839 | 96.101 | -36.473 | 1.00 | 39.31 | O |
| ATOM | 5013 | CB  | SER X | 11 | -31.841 | 97.609 | -33.833 | 1.00 | 38.56 | C |
| ATOM | 5014 | OG  | SER X | 11 | -31.368 | 98.589 | -34.727 | 1.00 | 43.26 | O |
| ATOM | 5015 | N   | ALA X | 12 | -32.670 | 95.054 | -35.669 | 1.00 | 37.39 | N |
| ATOM | 5016 | CA  | ALA X | 12 | -33.132 | 94.581 | -36.965 | 1.00 | 35.97 | C |
| ATOM | 5017 | C   | ALA X | 12 | -34.610 | 94.221 | -36.869 | 1.00 | 39.63 | C |
| ATOM | 5018 | O   | ALA X | 12 | -35.144 | 93.969 | -35.782 | 1.00 | 37.40 | O |
| ATOM | 5019 | CB  | ALA X | 12 | -32.317 | 93.374 | -37.454 | 1.00 | 32.40 | C |
| ATOM | 5020 | N   | ALA X | 13 | -35.245 | 94.137 | -38.035 | 1.00 | 39.79 | N |
| ATOM | 5021 | CA  | ALA X | 13 | -36.664 | 93.835 | -38.153 | 1.00 | 40.54 | C |
| ATOM | 5022 | C   | ALA X | 13 | -36.949 | 92.341 | -37.964 | 1.00 | 39.36 | C |
| ATOM | 5023 | O   | ALA X | 13 | -36.060 | 91.493 | -38.120 | 1.00 | 38.64 | O |
| ATOM | 5024 | CB  | ALA X | 13 | -37.170 | 94.286 | -39.518 | 1.00 | 26.30 | C |
| ATOM | 5025 | N   | PRO X | 14 | -38.182 | 91.992 | -37.606 | 1.00 | 39.35 | N |
| ATOM | 5026 | CA  | PRO X | 14 | -38.543 | 90.572 | -37.547 | 1.00 | 40.01 | C |
| ATOM | 5027 | C   | PRO X | 14 | -38.206 | 89.887 | -38.865 | 1.00 | 43.81 | C |
| ATOM | 5028 | O   | PRO X | 14 | -38.330 | 90.469 | -39.949 | 1.00 | 40.74 | O |
| ATOM | 5029 | CB  | PRO X | 14 | -40.050 | 90.596 | -37.287 | 1.00 | 28.16 | C |
| ATOM | 5030 | CG  | PRO X | 14 | -40.292 | 91.919 | -36.614 | 1.00 | 38.63 | C |
| ATOM | 5031 | CD  | PRO X | 14 | -39.300 | 92.873 | -37.204 | 1.00 | 38.01 | C |
| ATOM | 5032 | N   | GLY X | 15 | -37.742 | 88.649 | -38.754 | 1.00 | 42.53 | N |
| ATOM | 5033 | CA  | GLY X | 15 | -37.352 | 87.856 | -39.886 | 1.00 | 39.20 | C |
| ATOM | 5034 | C   | GLY X | 15 | -35.927 | 88.040 | -40.349 | 1.00 | 44.97 | C |
| ATOM | 5035 | O   | GLY X | 15 | -35.426 | 87.186 | -41.077 | 1.00 | 48.28 | O |
| ATOM | 5036 | N   | GLN X | 16 | -35.257 | 89.115 | -39.944 | 1.00 | 41.58 | N |

| ATOM | 5037 | CA | GLN X | 16 | -33.915 | 89.385 | -40.433 | 1.00 | 43.47 | C |
|------|------|----|-------|----|---------|--------|---------|------|-------|---|
| ATOM | 5038 | C | GLN X | 16 | -32.862 | 88.623 | -39.621 | 1.00 | 47.57 | C |
| ATOM | 5039 | O | GLN X | 16 | -33.167 | 87.903 | -38.664 | 1.00 | 46.60 | O |
| ATOM | 5040 | CB | GLN X | 16 | -33.635 | 90.888 | -40.417 | 1.00 | 52.99 | C |
| ATOM | 5041 | CG | GLN X | 16 | -34.303 | 91.702 | -41.539 | 1.00 | 46.17 | C |
| ATOM | 5042 | CD | GLN X | 16 | -33.566 | 93.024 | -41.807 | 1.00 | 74.76 | C |
| ATOM | 5043 | OE1 | GLN X | 16 | -33.715 | 94.021 | -41.064 | 1.00 | 62.51 | O |
| ATOM | 5044 | NE2 | GLN X | 16 | -32.758 | 93.034 | -42.866 | 1.00 | 79.00 | N |
| ATOM | 5045 | N | LYS X | 17 | -31.607 | 88.762 | -40.053 | 1.00 | 53.20 | N |
| ATOM | 5046 | CA | LYS X | 17 | -30.415 | 88.195 | -39.436 | 1.00 | 49.52 | C |
| ATOM | 5047 | C | LYS X | 17 | -29.695 | 89.254 | -38.627 | 1.00 | 51.74 | C |
| ATOM | 5048 | O | LYS X | 17 | -29.796 | 90.454 | -38.883 | 1.00 | 62.58 | O |
| ATOM | 5049 | CB | LYS X | 17 | -29.417 | 87.662 | -40.469 | 1.00 | 49.86 | C |
| ATOM | 5050 | CG | LYS X | 17 | -29.783 | 86.391 | -41.150 | 1.00 | 61.26 | C |
| ATOM | 5051 | CD | LYS X | 17 | -28.839 | 86.137 | -42.317 | 1.00 | 75.06 | C |
| ATOM | 5052 | CE | LYS X | 17 | -29.295 | 84.939 | -43.148 | 1.00 | 82.27 | C |
| ATOM | 5053 | NZ | LYS X | 17 | -28.391 | 84.723 | -44.304 | 1.00 | 82.49 | N |
| ATOM | 5054 | N | VAL X | 18 | -28.913 | 88.785 | -37.670 | 1.00 | 51.92 | N |
| ATOM | 5055 | CA | VAL X | 18 | -28.083 | 89.670 | -36.876 | 1.00 | 47.06 | C |
| ATOM | 5056 | C | VAL X | 18 | -26.882 | 88.857 | -36.416 | 1.00 | 42.24 | C |
| ATOM | 5057 | O | VAL X | 18 | -26.969 | 87.645 | -36.210 | 1.00 | 39.00 | O |
| ATOM | 5058 | CB | VAL X | 18 | -28.932 | 90.288 | -35.735 | 1.00 | 50.43 | C |
| ATOM | 5059 | CG1 | VAL X | 18 | -28.437 | 89.925 | -34.363 | 1.00 | 44.21 | C |
| ATOM | 5060 | CG2 | VAL X | 18 | -29.054 | 91.783 | -35.919 | 1.00 | 48.19 | C |
| ATOM | 5061 | N | THR X | 19 | -25.745 | 89.520 | -36.323 | 1.00 | 40.09 | N |
| ATOM | 5062 | CA | THR X | 19 | -24.519 | 88.924 | -35.825 | 1.00 | 43.49 | C |
| ATOM | 5063 | C | THR X | 19 | -24.005 | 89.770 | -34.672 | 1.00 | 39.57 | C |
| ATOM | 5064 | O | THR X | 19 | -24.036 | 91.000 | -34.737 | 1.00 | 39.43 | O |
| ATOM | 5065 | CB | THR X | 19 | -23.484 | 88.816 | -36.961 | 1.00 | 40.40 | C |
| ATOM | 5066 | OG1 | THR X | 19 | -23.352 | 87.443 | -37.322 | 1.00 | 51.45 | O |
| ATOM | 5067 | CG2 | THR X | 19 | -22.132 | 89.395 | -36.582 | 1.00 | 45.74 | C |
| ATOM | 5068 | N | ILE X | 20 | -23.557 | 89.116 | -33.607 | 1.00 | 36.67 | N |
| ATOM | 5069 | CA | ILE X | 20 | -23.009 | 89.805 | -32.449 | 1.00 | 34.08 | C |
| ATOM | 5070 | C | ILE X | 20 | -21.638 | 89.219 | -32.166 | 1.00 | 35.80 | C |
| ATOM | 5071 | O | ILE X | 20 | -21.517 | 88.008 | -31.946 | 1.00 | 34.42 | O |
| ATOM | 5072 | CB | ILE X | 20 | -23.917 | 89.679 | -31.210 | 1.00 | 36.76 | C |
| ATOM | 5073 | CG1 | ILE X | 20 | -25.269 | 90.345 | -31.467 | 1.00 | 35.10 | C |
| ATOM | 5074 | CG2 | ILE X | 20 | -23.257 | 90.324 | -29.996 | 1.00 | 27.82 | C |
| ATOM | 5075 | CD1 | ILE X | 20 | -26.276 | 90.127 | -30.364 | 1.00 | 28.66 | C |
| ATOM | 5076 | N | SER X | 21 | -20.614 | 90.068 | -32.153 | 1.00 | 38.15 | N |
| ATOM | 5077 | CA | SER X | 21 | -19.237 | 89.614 | -31.980 | 1.00 | 37.42 | C |
| ATOM | 5078 | C | SER X | 21 | -18.762 | 89.818 | -30.547 | 1.00 | 38.58 | C |
| ATOM | 5079 | O | SER X | 21 | -19.344 | 90.570 | -29.762 | 1.00 | 39.24 | O |
| ATOM | 5080 | CB | SER X | 21 | -18.290 | 90.351 | -32.932 | 1.00 | 30.39 | C |
| ATOM | 5081 | OG | SER X | 21 | -18.312 | 91.741 | -32.656 | 1.00 | 45.43 | O |
| ATOM | 5082 | N | CYS X | 22 | -17.662 | 89.152 | -30.226 | 1.00 | 31.32 | N |
| ATOM | 5083 | CA | CYS X | 22 | -17.099 | 89.156 | -28.883 | 1.00 | 33.00 | C |
| ATOM | 5084 | C | CYS X | 22 | -15.601 | 88.936 | -29.035 | 1.00 | 36.67 | C |
| ATOM | 5085 | O | CYS X | 22 | -15.179 | 87.824 | -29.367 | 1.00 | 33.05 | O |
| ATOM | 5086 | CB | CYS X | 22 | -17.737 | 88.067 | -28.029 | 1.00 | 33.80 | C |
| ATOM | 5087 | SG | CYS X | 22 | -16.950 | 87.740 | -26.422 | 1.00 | 49.12 | S |
| ATOM | 5088 | N | SER X | 23 | -14.806 | 89.986 | -28.830 | 1.00 | 35.27 | N |
| ATOM | 5089 | CA | SER X | 23 | -13.362 | 89.917 | -29.015 | 1.00 | 36.02 | C |
| ATOM | 5090 | C | SER X | 23 | -12.635 | 89.893 | -27.682 | 1.00 | 35.20 | C |
| ATOM | 5091 | O | SER X | 23 | -12.961 | 90.659 | -26.765 | 1.00 | 32.83 | O |
| ATOM | 5092 | CB | SER X | 23 | -12.847 | 91.105 | -29.824 | 1.00 | 34.28 | C |
| ATOM | 5093 | OG | SER X | 23 | -13.487 | 91.109 | -31.082 | 1.00 | 60.42 | O |
| ATOM | 5094 | N | GLY X | 24 | -11.631 | 89.027 | -27.600 | 1.00 | 29.76 | N |
| ATOM | 5095 | CA | GLY X | 24 | -10.801 | 88.942 | -26.426 | 1.00 | 32.74 | C |
| ATOM | 5096 | C | GLY X | 24 | -9.364 | 88.732 | -26.822 | 1.00 | 36.96 | C |
| ATOM | 5097 | O | GLY X | 24 | -8.898 | 89.366 | -27.766 | 1.00 | 35.74 | O |
| ATOM | 5098 | N | SER X | 25 | -8.666 | 87.829 | -26.132 | 1.00 | 34.51 | N |
| ATOM | 5099 | CA | SER X | 25 | -7.238 | 87.632 | -26.326 | 1.00 | 35.15 | C |
| ATOM | 5100 | C | SER X | 25 | -6.925 | 86.161 | -26.114 | 1.00 | 38.46 | C |
| ATOM | 5101 | O | SER X | 25 | -7.793 | 85.366 | -25.733 | 1.00 | 35.89 | O |
| ATOM | 5102 | CB | SER X | 25 | -6.411 | 88.485 | -25.366 | 1.00 | 31.48 | C |
| ATOM | 5103 | OG | SER X | 25 | -6.531 | 87.980 | -24.047 | 1.00 | 41.73 | O |
| ATOM | 5104 | N | SER X | 26 | -5.656 | 85.811 | -26.338 | 1.00 | 32.62 | N |
| ATOM | 5105 | CA | SER X | 26 | -5.255 | 84.410 | -26.269 | 1.00 | 40.22 | C |
| ATOM | 5106 | C | SER X | 26 | -5.478 | 83.825 | -24.886 | 1.00 | 39.50 | C |
| ATOM | 5107 | O | SER X | 26 | -5.701 | 82.616 | -24.755 | 1.00 | 43.16 | O |

```
ATOM   5108  CB   SER X  26     -3.779  84.236 -26.645  1.00 33.65           C
ATOM   5109  OG   SER X  26     -2.978  85.154 -25.933  1.00 50.17           O
ATOM   5110  N    SER X  27     -5.442  84.650 -23.847  1.00 37.12           N
ATOM   5111  CA   SER X  27     -5.564  84.097 -22.504  1.00 35.96           C
ATOM   5112  C    SER X  27     -7.010  83.927 -22.062  1.00 36.99           C
ATOM   5113  O    SER X  27     -7.236  83.380 -20.976  1.00 37.69           O
ATOM   5114  CB   SER X  27     -4.848  84.982 -21.480  1.00 32.77           C
ATOM   5115  OG   SER X  27     -5.484  86.238 -21.459  1.00 46.10           O
ATOM   5116  N    ASN X  28     -7.992  84.407 -22.840  1.00 35.46           N
ATOM   5117  CA   ASN X  28     -9.372  84.080 -22.495  1.00 33.17           C
ATOM   5118  C    ASN X  28    -10.056  83.327 -23.636  1.00 34.99           C
ATOM   5119  O    ASN X  28    -10.028  82.089 -23.659  1.00 30.44           O
ATOM   5120  CB   ASN X  28    -10.161  85.324 -22.072  1.00 29.46           C
ATOM   5121  CG   ASN X  28     -9.945  86.529 -22.981  1.00 33.39           C
ATOM   5122  OD1  ASN X  28    -10.325  86.527 -24.163  1.00 33.37           O
ATOM   5123  ND2  ASN X  28     -9.394  87.594 -22.408  1.00 28.13           N
ATOM   5124  N    ILE X  29    -10.677  84.041 -24.577  1.00 26.63           N
ATOM   5125  CA   ILE X  29    -11.370  83.349 -25.660  1.00 33.95           C
ATOM   5126  C    ILE X  29    -10.423  82.422 -26.417  1.00 37.55           C
ATOM   5127  O    ILE X  29    -10.814  81.335 -26.857  1.00 33.77           O
ATOM   5128  CB   ILE X  29    -12.029  84.355 -26.611  1.00 31.78           C
ATOM   5129  CG1  ILE X  29    -13.165  85.074 -25.894  1.00 37.08           C
ATOM   5130  CG2  ILE X  29    -12.570  83.634 -27.846  1.00 27.80           C
ATOM   5131  CD1  ILE X  29    -13.921  85.975 -26.801  1.00 36.60           C
ATOM   5132  N    GLY X  30     -9.171  82.838 -26.596  1.00 38.13           N
ATOM   5133  CA   GLY X  30     -8.264  82.044 -27.399  1.00 32.55           C
ATOM   5134  C    GLY X  30     -7.961  80.683 -26.819  1.00 33.31           C
ATOM   5135  O    GLY X  30     -7.589  79.781 -27.559  1.00 43.57           O
ATOM   5136  N    ASN X  31     -8.136  80.495 -25.521  1.00 40.07           N
ATOM   5137  CA   ASN X  31     -7.797  79.191 -24.968  1.00 37.30           C
ATOM   5138  C    ASN X  31     -8.808  78.618 -23.980  1.00 36.23           C
ATOM   5139  O    ASN X  31     -8.500  77.622 -23.324  1.00 37.83           O
ATOM   5140  CB   ASN X  31     -6.405  79.245 -24.343  1.00 43.50           C
ATOM   5141  CG   ASN X  31     -5.314  79.117 -25.408  1.00 56.73           C
ATOM   5142  OD1  ASN X  31     -5.006  78.007 -25.858  1.00 58.50           O
ATOM   5143  ND2  ASN X  31     -4.764  80.253 -25.851  1.00 48.77           N
ATOM   5144  N    ASN X  32    -10.015  79.157 -23.886  1.00 33.81           N
ATOM   5145  CA   ASN X  32    -10.979  78.603 -22.954  1.00 35.74           C
ATOM   5146  C    ASN X  32    -12.338  78.460 -23.628  1.00 33.34           C
ATOM   5147  O    ASN X  32    -12.583  79.011 -24.700  1.00 34.66           O
ATOM   5148  CB   ASN X  32    -11.035  79.466 -21.699  1.00 32.31           C
ATOM   5149  CG   ASN X  32     -9.711  79.508 -20.983  1.00 33.00           C
ATOM   5150  OD1  ASN X  32     -9.339  78.559 -20.300  1.00 35.81           O
ATOM   5151  ND2  ASN X  32     -8.979  80.606 -21.141  1.00 40.72           N
ATOM   5152  N    TYR X  33    -13.220  77.691 -22.998  1.00 33.41           N
ATOM   5153  CA   TYR X  33    -14.543  77.451 -23.572  1.00 35.49           C
ATOM   5154  C    TYR X  33    -15.355  78.735 -23.542  1.00 30.39           C
ATOM   5155  O    TYR X  33    -15.333  79.455 -22.555  1.00 29.67           O
ATOM   5156  CB   TYR X  33    -15.281  76.348 -22.793  1.00 28.71           C
ATOM   5157  CG   TYR X  33    -14.627  74.978 -22.878  1.00 33.53           C
ATOM   5158  CD1  TYR X  33    -14.513  74.311 -24.098  1.00 27.38           C
ATOM   5159  CD2  TYR X  33    -14.115  74.356 -21.740  1.00 32.47           C
ATOM   5160  CE1  TYR X  33    -13.915  73.063 -24.178  1.00 30.49           C
ATOM   5161  CE2  TYR X  33    -13.514  73.101 -21.813  1.00 31.14           C
ATOM   5162  CZ   TYR X  33    -13.409  72.468 -23.043  1.00 31.76           C
ATOM   5163  OH   TYR X  33    -12.815  71.235 -23.121  1.00 31.17           O
ATOM   5164  N    VAL X  34    -16.106  79.006 -24.602  1.00 29.15           N
ATOM   5165  CA   VAL X  34    -16.887  80.240 -24.708  1.00 36.08           C
ATOM   5166  C    VAL X  34    -18.366  79.954 -24.434  1.00 32.87           C
ATOM   5167  O    VAL X  34    -18.934  79.011 -24.996  1.00 30.79           O
ATOM   5168  CB   VAL X  34    -16.704  80.865 -26.098  1.00 33.81           C
ATOM   5169  CG1  VAL X  34    -17.582  82.090 -26.255  1.00 27.19           C
ATOM   5170  CG2  VAL X  34    -15.243  81.172 -26.332  1.00 32.31           C
ATOM   5171  N    SER X  35    -19.008  80.798 -23.617  1.00 32.88           N
ATOM   5172  CA   SER X  35    -20.444  80.696 -23.349  1.00 30.32           C
ATOM   5173  C    SER X  35    -21.176  81.975 -23.759  1.00 32.72           C
ATOM   5174  O    SER X  35    -20.616  83.075 -23.709  1.00 28.89           O
ATOM   5175  CB   SER X  35    -20.725  80.419 -21.869  1.00 28.37           C
ATOM   5176  OG   SER X  35    -20.265  79.144 -21.480  1.00 28.41           O
ATOM   5177  N    TRP X  36    -22.446  81.832 -24.154  1.00 28.96           N
ATOM   5178  CA   TRP X  36    -23.308  82.972 -24.451  1.00 27.19           C
```

341

| ATOM | 5179 | C | TRP | X | 36 | -24.541 | 82.934 | -23.566 | 1.00 | 32.55 | C |
|------|------|-----|-----|---|----|---------|--------|---------|------|-------|---|
| ATOM | 5180 | O | TRP | X | 36 | -25.151 | 81.873 | -23.374 | 1.00 | 33.96 | O |
| ATOM | 5181 | CB | TRP | X | 36 | -23.738 | 83.010 | -25.915 | 1.00 | 28.66 | C |
| ATOM | 5182 | CG | TRP | X | 36 | -22.661 | 83.470 | -26.893 | 1.00 | 32.03 | C |
| ATOM | 5183 | CD1 | TRP | X | 36 | -21.775 | 82.676 | -27.576 | 1.00 | 30.86 | C |
| ATOM | 5184 | CD2 | TRP | X | 36 | -22.382 | 84.821 | -27.305 | 1.00 | 29.58 | C |
| ATOM | 5185 | NE1 | TRP | X | 36 | -20.965 | 83.451 | -28.377 | 1.00 | 29.86 | N |
| ATOM | 5186 | CE2 | TRP | X | 36 | -21.320 | 84.767 | -28.231 | 1.00 | 32.46 | C |
| ATOM | 5187 | CE3 | TRP | X | 36 | -22.926 | 86.067 | -26.979 | 1.00 | 30.71 | C |
| ATOM | 5188 | CZ2 | TRP | X | 36 | -20.784 | 85.917 | -28.827 | 1.00 | 34.25 | C |
| ATOM | 5189 | CZ3 | TRP | X | 36 | -22.391 | 87.204 | -27.571 | 1.00 | 32.12 | C |
| ATOM | 5190 | CH2 | TRP | X | 36 | -21.329 | 87.120 | -28.480 | 1.00 | 31.97 | C |
| ATOM | 5191 | N | TYR | X | 37 | -24.909 | 84.105 | -23.040 | 1.00 | 34.18 | N |
| ATOM | 5192 | CA | TYR | X | 37 | -26.054 | 84.256 | -22.152 | 1.00 | 28.55 | C |
| ATOM | 5193 | C | TYR | X | 37 | -27.044 | 85.232 | -22.763 | 1.00 | 30.63 | C |
| ATOM | 5194 | O | TYR | X | 37 | -26.646 | 86.220 | -23.399 | 1.00 | 30.08 | O |
| ATOM | 5195 | CB | TYR | X | 37 | -25.607 | 84.731 | -20.760 | 1.00 | 24.98 | C |
| ATOM | 5196 | CG | TYR | X | 37 | -24.567 | 83.797 | -20.195 | 1.00 | 29.34 | C |
| ATOM | 5197 | CD1 | TYR | X | 37 | -24.941 | 82.601 | -19.573 | 1.00 | 27.60 | C |
| ATOM | 5198 | CD2 | TYR | X | 37 | -23.210 | 84.059 | -20.346 | 1.00 | 27.93 | C |
| ATOM | 5199 | CE1 | TYR | X | 37 | -23.978 | 81.699 | -19.056 | 1.00 | 30.73 | C |
| ATOM | 5200 | CE2 | TYR | X | 37 | -22.234 | 83.172 | -19.828 | 1.00 | 31.38 | C |
| ATOM | 5201 | CZ | TYR | X | 37 | -22.629 | 81.990 | -19.195 | 1.00 | 32.90 | C |
| ATOM | 5202 | OH | TYR | X | 37 | -21.685 | 81.119 | -18.701 | 1.00 | 30.71 | O |
| ATOM | 5203 | N | GLN | X | 38 | -28.331 | 84.926 | -22.592 | 1.00 | 29.50 | N |
| ATOM | 5204 | CA | GLN | X | 38 | -29.430 | 85.780 | -23.026 | 1.00 | 30.90 | C |
| ATOM | 5205 | C | GLN | X | 38 | -30.211 | 86.252 | -21.807 | 1.00 | 31.01 | C |
| ATOM | 5206 | O | GLN | X | 38 | -30.612 | 85.436 | -20.970 | 1.00 | 29.04 | O |
| ATOM | 5207 | CB | GLN | X | 38 | -30.365 | 85.044 | -23.979 | 1.00 | 27.81 | C |
| ATOM | 5208 | CG | GLN | X | 38 | -31.587 | 85.851 | -24.407 | 1.00 | 27.64 | C |
| ATOM | 5209 | CD | GLN | X | 38 | -32.640 | 84.958 | -25.007 | 1.00 | 34.54 | C |
| ATOM | 5210 | OE1 | GLN | X | 38 | -33.141 | 84.043 | -24.329 | 1.00 | 34.50 | O |
| ATOM | 5211 | NE2 | GLN | X | 38 | -32.985 | 85.195 | -26.279 | 1.00 | 26.87 | N |
| ATOM | 5212 | N | GLN | X | 39 | -30.416 | 87.566 | -21.705 | 1.00 | 29.58 | N |
| ATOM | 5213 | CA | GLN | X | 39 | -31.199 | 88.157 | -20.623 | 1.00 | 29.79 | C |
| ATOM | 5214 | C | GLN | X | 39 | -32.378 | 88.907 | -21.252 | 1.00 | 36.36 | C |
| ATOM | 5215 | O | GLN | X | 39 | -32.234 | 90.040 | -21.731 | 1.00 | 37.54 | O |
| ATOM | 5216 | CB | GLN | X | 39 | -30.341 | 89.067 | -19.749 | 1.00 | 26.28 | C |
| ATOM | 5217 | CG | GLN | X | 39 | -31.077 | 89.513 | -18.479 | 1.00 | 31.07 | C |
| ATOM | 5218 | CD | GLN | X | 39 | -30.207 | 90.302 | -17.520 | 1.00 | 33.21 | C |
| ATOM | 5219 | OE1 | GLN | X | 39 | -29.255 | 90.951 | -17.926 | 1.00 | 33.77 | O |
| ATOM | 5220 | NE2 | GLN | X | 39 | -30.542 | 90.260 | -16.241 | 1.00 | 34.37 | N |
| ATOM | 5221 | N | LEU | X | 40 | -33.539 | 88.255 | -21.271 | 1.00 | 32.04 | N |
| ATOM | 5222 | CA | LEU | X | 40 | -34.756 | 88.889 | -21.729 | 1.00 | 34.90 | C |
| ATOM | 5223 | C | LEU | X | 40 | -35.132 | 90.007 | -20.753 | 1.00 | 38.54 | C |
| ATOM | 5224 | O | LEU | X | 40 | -34.759 | 89.948 | -19.575 | 1.00 | 31.68 | O |
| ATOM | 5225 | CB | LEU | X | 40 | -35.872 | 87.851 | -21.832 | 1.00 | 33.07 | C |
| ATOM | 5226 | CG | LEU | X | 40 | -35.490 | 86.643 | -22.697 | 1.00 | 39.00 | C |
| ATOM | 5227 | CD1 | LEU | X | 40 | -36.411 | 85.450 | -22.373 | 1.00 | 38.05 | C |
| ATOM | 5228 | CD2 | LEU | X | 40 | -35.493 | 86.947 | -24.228 | 1.00 | 26.12 | C |
| ATOM | 5229 | N | PRO | X | 41 | -35.826 | 91.047 | -21.227 | 1.00 | 36.64 | N |
| ATOM | 5230 | CA | PRO | X | 41 | -36.146 | 92.208 | -20.371 | 1.00 | 41.13 | C |
| ATOM | 5231 | C | PRO | X | 41 | -36.855 | 91.770 | -19.096 | 1.00 | 38.67 | C |
| ATOM | 5232 | O | PRO | X | 41 | -37.737 | 90.913 | -19.128 | 1.00 | 32.82 | O |
| ATOM | 5233 | CB | PRO | X | 41 | -37.079 | 93.058 | -21.246 | 1.00 | 36.21 | C |
| ATOM | 5234 | CG | PRO | X | 41 | -36.885 | 92.555 | -22.651 | 1.00 | 38.84 | C |
| ATOM | 5235 | CD | PRO | X | 41 | -36.528 | 91.097 | -22.519 | 1.00 | 39.30 | C |
| ATOM | 5236 | N | GLY | X | 42 | -36.410 | 92.308 | -17.961 | 1.00 | 37.25 | N |
| ATOM | 5237 | CA | GLY | X | 42 | -36.981 | 91.946 | -16.678 | 1.00 | 39.06 | C |
| ATOM | 5238 | C | GLY | X | 42 | -36.772 | 90.514 | -16.194 | 1.00 | 47.15 | C |
| ATOM | 5239 | O | GLY | X | 42 | -37.474 | 90.080 | -15.269 | 1.00 | 47.29 | O |
| ATOM | 5240 | N | THR | X | 43 | -35.830 | 89.759 | -16.760 | 1.00 | 42.87 | N |
| ATOM | 5241 | CA | THR | X | 43 | -35.594 | 88.390 | -16.295 | 1.00 | 41.60 | C |
| ATOM | 5242 | C | THR | X | 43 | -34.125 | 88.218 | -15.937 | 1.00 | 34.86 | C |
| ATOM | 5243 | O | THR | X | 43 | -33.283 | 89.070 | -16.225 | 1.00 | 35.66 | O |
| ATOM | 5244 | CB | THR | X | 43 | -35.966 | 87.300 | -17.340 | 1.00 | 38.72 | C |
| ATOM | 5245 | OG1 | THR | X | 43 | -34.965 | 87.232 | -18.372 | 1.00 | 37.04 | O |
| ATOM | 5246 | CG2 | THR | X | 43 | -37.299 | 87.578 | -18.007 | 1.00 | 39.86 | C |
| ATOM | 5247 | N | ALA | X | 44 | -33.838 | 87.056 | -15.351 | 1.00 | 36.79 | N |
| ATOM | 5248 | CA | ALA | X | 44 | -32.495 | 86.614 | -15.069 | 1.00 | 33.26 | C |
| ATOM | 5249 | C | ALA | X | 44 | -31.804 | 86.190 | -16.368 | 1.00 | 34.93 | C |

```
ATOM   5250  O   ALA X  44      -32.466  85.879 -17.358  1.00 32.13          O
ATOM   5251  CB  ALA X  44      -32.537  85.449 -14.085  1.00 29.97          C
ATOM   5252  N   PRO X  45      -30.470  86.187 -16.399  1.00 31.73          N
ATOM   5253  CA  PRO X  45      -29.779  85.591 -17.540  1.00 29.03          C
ATOM   5254  C   PRO X  45      -30.152  84.124 -17.700  1.00 32.97          C
ATOM   5255  O   PRO X  45      -30.631  83.455 -16.779  1.00 27.00          O
ATOM   5256  CB  PRO X  45      -28.294  85.750 -17.187  1.00 29.30          C
ATOM   5257  CG  PRO X  45      -28.267  86.928 -16.262  1.00 32.14          C
ATOM   5258  CD  PRO X  45      -29.524  86.784 -15.442  1.00 28.71          C
ATOM   5259  N   LYS X  46      -29.905  83.624 -18.898  1.00 38.36          N
ATOM   5260  CA  LYS X  46      -30.155  82.237 -19.238  1.00 34.68          C
ATOM   5261  C   LYS X  46      -29.009  81.792 -20.131  1.00 35.47          C
ATOM   5262  O   LYS X  46      -28.503  82.593 -20.930  1.00 27.61          O
ATOM   5263  CB  LYS X  46      -31.500  82.135 -19.949  1.00 31.10          C
ATOM   5264  CG  LYS X  46      -31.750  80.915 -20.700  1.00 41.49          C
ATOM   5265  CD  LYS X  46      -32.725  81.196 -21.858  1.00 47.60          C
ATOM   5266  CE  LYS X  46      -33.943  81.979 -21.420  1.00 48.37          C
ATOM   5267  NZ  LYS X  46      -35.081  81.729 -22.345  1.00 49.13          N1+
ATOM   5268  N   LEU X  47      -28.574  80.539 -19.977  1.00 28.77          N
ATOM   5269  CA  LEU X  47      -27.546  80.027 -20.872  1.00 24.97          C
ATOM   5270  C   LEU X  47      -28.138  79.828 -22.264  1.00 32.77          C
ATOM   5271  O   LEU X  47      -29.190  79.200 -22.426  1.00 29.18          O
ATOM   5272  CB  LEU X  47      -26.955  78.730 -20.347  1.00 26.07          C
ATOM   5273  CG  LEU X  47      -25.872  78.105 -21.231  1.00 29.09          C
ATOM   5274  CD1 LEU X  47      -24.689  79.042 -21.402  1.00 29.03          C
ATOM   5275  CD2 LEU X  47      -25.420  76.789 -20.647  1.00 27.90          C
ATOM   5276  N   LEU X  48      -27.482  80.418 -23.260  1.00 30.28          N
ATOM   5277  CA  LEU X  48      -27.901  80.361 -24.651  1.00 33.70          C
ATOM   5278  C   LEU X  48      -27.075  79.352 -25.441  1.00 38.84          C
ATOM   5279  O   LEU X  48      -27.633  78.536 -26.179  1.00 35.80          O
ATOM   5280  CB  LEU X  48      -27.787  81.757 -25.286  1.00 30.10          C
ATOM   5281  CG  LEU X  48      -28.388  81.949 -26.684  1.00 36.25          C
ATOM   5282  CD1 LEU X  48      -29.916  81.905 -26.686  1.00 29.13          C
ATOM   5283  CD2 LEU X  48      -27.887  83.232 -27.342  1.00 32.77          C
ATOM   5284  N   LEU X  49      -25.749  79.397 -25.279  1.00 38.10          N
ATOM   5285  CA  LEU X  49      -24.799  78.535 -25.968  1.00 29.61          C
ATOM   5286  C   LEU X  49      -23.642  78.253 -25.028  1.00 31.16          C
ATOM   5287  O   LEU X  49      -23.153  79.160 -24.352  1.00 32.51          O
ATOM   5288  CB  LEU X  49      -24.241  79.177 -27.235  1.00 29.50          C
ATOM   5289  CG  LEU X  49      -25.158  79.484 -28.410  1.00 35.20          C
ATOM   5290  CD1 LEU X  49      -24.385  80.328 -29.434  1.00 32.31          C
ATOM   5291  CD2 LEU X  49      -25.624  78.172 -29.024  1.00 29.29          C
ATOM   5292  N   TYR X  50      -23.204  77.003 -24.983  1.00 27.72          N
ATOM   5293  CA  TYR X  50      -21.976  76.679 -24.285  1.00 28.60          C
ATOM   5294  C   TYR X  50      -21.037  75.963 -25.242  1.00 30.23          C
ATOM   5295  O   TYR X  50      -21.429  75.569 -26.346  1.00 31.08          O
ATOM   5296  CB  TYR X  50      -22.250  75.864 -23.018  1.00 28.65          C
ATOM   5297  CG  TYR X  50      -22.900  74.531 -23.227  1.00 33.55          C
ATOM   5298  CD1 TYR X  50      -24.246  74.422 -23.613  1.00 37.48          C
ATOM   5299  CD2 TYR X  50      -22.196  73.384 -22.985  1.00 29.14          C
ATOM   5300  CE1 TYR X  50      -24.833  73.183 -23.799  1.00 34.48          C
ATOM   5301  CE2 TYR X  50      -22.768  72.149 -23.152  1.00 39.17          C
ATOM   5302  CZ  TYR X  50      -24.076  72.041 -23.550  1.00 38.17          C
ATOM   5303  OH  TYR X  50      -24.590  70.771 -23.700  1.00 35.08          O
ATOM   5304  N   ASP X  51      -19.768  75.877 -24.840  1.00 31.56          N
ATOM   5305  CA  ASP X  51      -18.698  75.390 -25.713  1.00 29.21          C
ATOM   5306  C   ASP X  51      -18.816  76.000 -27.122  1.00 34.44          C
ATOM   5307  O   ASP X  51      -18.908  75.308 -28.144  1.00 31.23          O
ATOM   5308  CB  ASP X  51      -18.699  73.865 -25.753  1.00 30.93          C
ATOM   5309  CG  ASP X  51      -17.509  73.308 -26.507  1.00 34.52          C
ATOM   5310  OD1 ASP X  51      -16.467  74.009 -26.589  1.00 38.97          O
ATOM   5311  OD2 ASP X  51      -17.633  72.187 -27.047  1.00 37.63          O1-
ATOM   5312  N   SER X  52      -18.882  77.333 -27.151  1.00 29.36          N
ATOM   5313  CA  SER X  52      -18.957  78.121 -28.379  1.00 32.69          C
ATOM   5314  C   SER X  52      -20.258  77.970 -29.163  1.00 32.50          C
ATOM   5315  O   SER X  52      -20.805  78.977 -29.637  1.00 32.67          O
ATOM   5316  CB  SER X  52      -17.781  77.808 -29.305  1.00 32.99          C
ATOM   5317  OG  SER X  52      -16.569  78.277 -28.744  1.00 42.26          O
ATOM   5318  N   ASN X  53      -20.772  76.746 -29.333  1.00 31.33          N
ATOM   5319  CA  ASN X  53      -21.880  76.604 -30.271  1.00 33.53          C
ATOM   5320  C   ASN X  53      -22.896  75.532 -29.886  1.00 36.41          C
```

```
ATOM   5321  O   ASN X  53   -23.725  75.172 -30.735  1.00 34.64        O
ATOM   5322  CB  ASN X  53   -21.326  76.305 -31.671  1.00 29.24        C
ATOM   5323  CG  ASN X  53   -20.625  74.953 -31.730  1.00 38.39        C
ATOM   5324  OD1 ASN X  53   -20.519  74.254 -30.721  1.00 35.56        O
ATOM   5325  ND2 ASN X  53   -20.163  74.573 -32.906  1.00 35.38        N
ATOM   5326  N   LYS X  54   -22.879  75.013 -28.657  1.00 37.93        N
ATOM   5327  CA  LYS X  54   -23.821  73.978 -28.239  1.00 37.84        C
ATOM   5328  C   LYS X  54   -25.063  74.592 -27.605  1.00 33.93        C
ATOM   5329  O   LYS X  54   -24.957  75.380 -26.656  1.00 33.00        O
ATOM   5330  CB  LYS X  54   -23.165  73.014 -27.250  1.00 34.82        C
ATOM   5331  CG  LYS X  54   -24.025  71.814 -26.907  1.00 36.40        C
ATOM   5332  CD  LYS X  54   -24.191  70.937 -28.131  1.00 37.62        C
ATOM   5333  CE  LYS X  54   -25.209  69.832 -27.894  1.00 33.30        C
ATOM   5334  NZ  LYS X  54   -25.145  68.918 -29.053  1.00 47.57        N1+
ATOM   5335  N  AARG X  55   -26.235  74.212 -28.121  0.50 35.72        N
ATOM   5336  CA AARG X  55   -27.511  74.703 -27.600  0.50 37.84        C
ATOM   5337  C  AARG X  55   -27.955  73.849 -26.418  0.50 37.19        C
ATOM   5338  O  AARG X  55   -28.052  72.622 -26.555  0.50 39.62        O
ATOM   5339  CB AARG X  55   -28.585  74.683 -28.683  0.50 35.53        C
ATOM   5340  CG AARG X  55   -28.487  75.808 -29.722  0.50 38.76        C
ATOM   5341  CD AARG X  55   -29.610  75.753 -30.781  0.50 37.60        C
ATOM   5342  NE AARG X  55   -29.427  74.636 -31.705  0.50 42.24        N
ATOM   5343  CZ AARG X  55   -30.198  73.555 -31.759  0.50 40.39        C
ATOM   5344  NH1AARG X  55   -29.919  72.598 -32.628  0.50 43.66        N1+
ATOM   5345  NH2AARG X  55   -31.248  73.434 -30.961  0.50 39.76        N
ATOM   5346  N  BARG X  55   -26.235  74.213 -28.110  0.50 35.73        N
ATOM   5347  CA BARG X  55   -27.499  74.742 -27.594  0.50 37.86        C
ATOM   5348  C  BARG X  55   -27.999  73.875 -26.441  0.50 37.17        C
ATOM   5349  O  BARG X  55   -28.165  72.661 -26.619  0.50 39.65        O
ATOM   5350  CB BARG X  55   -28.548  74.810 -28.701  0.50 35.58        C
ATOM   5351  CG BARG X  55   -28.335  75.971 -29.695  0.50 38.67        C
ATOM   5352  CD BARG X  55   -29.274  75.940 -30.919  0.50 37.26        C
ATOM   5353  NE BARG X  55   -28.921  74.866 -31.845  0.50 42.86        N
ATOM   5354  CZ BARG X  55   -28.062  74.983 -32.859  0.50 43.30        C
ATOM   5355  NH1BARG X  55   -27.465  76.140 -33.108  0.50 28.23        N1+
ATOM   5356  NH2BARG X  55   -27.803  73.928 -33.632  0.50 47.26        N
ATOM   5357  N   PRO X  56   -28.209  74.432 -25.251  1.00 39.61        N
ATOM   5358  CA  PRO X  56   -28.924  73.683 -24.208  1.00 37.27        C
ATOM   5359  C   PRO X  56   -30.328  73.384 -24.708  1.00 37.17        C
ATOM   5360  O   PRO X  56   -30.836  74.048 -25.614  1.00 35.06        O
ATOM   5361  CB  PRO X  56   -28.960  74.643 -23.020  1.00 32.87        C
ATOM   5362  CG  PRO X  56   -28.041  75.768 -23.374  1.00 39.79        C
ATOM   5363  CD  PRO X  56   -27.841  75.794 -24.846  1.00 34.03        C
ATOM   5364  N   SER X  57   -30.973  72.378 -24.127  1.00 35.37        N
ATOM   5365  CA  SER X  57   -32.324  72.095 -24.603  1.00 45.77        C
ATOM   5366  C   SER X  57   -33.240  73.271 -24.286  1.00 39.53        C
ATOM   5367  O   SER X  57   -33.082  73.960 -23.274  1.00 42.51        O
ATOM   5368  CB  SER X  57   -32.860  70.777 -24.033  1.00 44.76        C
ATOM   5369  OG  SER X  57   -32.486  70.611 -22.686  1.00 57.37        O
ATOM   5370  N   GLY X  58   -34.149  73.551 -25.208  1.00 46.11        N
ATOM   5371  CA  GLY X  58   -35.017  74.704 -25.108  1.00 38.41        C
ATOM   5372  C   GLY X  58   -34.575  75.906 -25.915  1.00 46.36        C
ATOM   5373  O   GLY X  58   -35.360  76.846 -26.056  1.00 47.75        O
ATOM   5374  N   ILE X  59   -33.356  75.916 -26.442  1.00 38.47        N
ATOM   5375  CA  ILE X  59   -32.858  77.042 -27.231  1.00 39.45        C
ATOM   5376  C   ILE X  59   -33.096  76.740 -28.706  1.00 38.36        C
ATOM   5377  O   ILE X  59   -32.567  75.743 -29.218  1.00 41.39        O
ATOM   5378  CB  ILE X  59   -31.372  77.301 -26.950  1.00 41.60        C
ATOM   5379  CG1 ILE X  59   -31.165  77.604 -25.460  1.00 39.46        C
ATOM   5380  CG2 ILE X  59   -30.858  78.449 -27.839  1.00 36.16        C
ATOM   5381  CD1 ILE X  59   -31.957  78.855 -24.970  1.00 33.58        C
ATOM   5382  N   PRO X  60   -33.829  77.583 -29.429  1.00 38.04        N
ATOM   5383  CA  PRO X  60   -34.124  77.311 -30.841  1.00 40.67        C
ATOM   5384  C   PRO X  60   -32.877  77.315 -31.712  1.00 41.79        C
ATOM   5385  O   PRO X  60   -31.899  78.020 -31.439  1.00 39.12        O
ATOM   5386  CB  PRO X  60   -35.047  78.468 -31.240  1.00 37.50        C
ATOM   5387  CG  PRO X  60   -35.340  79.184 -30.024  1.00 40.79        C
ATOM   5388  CD  PRO X  60   -34.325  78.891 -29.004  1.00 41.33        C
ATOM   5389  N   ALA X  61   -32.956  76.574 -32.821  1.00 41.88        N
ATOM   5390  CA  ALA X  61   -31.816  76.464 -33.727  1.00 41.28        C
ATOM   5391  C   ALA X  61   -31.500  77.762 -34.449  1.00 37.80        C
```

```
ATOM   5392  O    ALA X  61    -30.440  77.855 -35.066  1.00 37.27           O
ATOM   5393  CB   ALA X  61    -32.043  75.362 -34.759  1.00 34.30           C
ATOM   5394  N    ARG X  62    -32.356  78.779 -34.366  1.00 38.43           N
ATOM   5395  CA   ARG X  62    -31.978  80.019 -35.031  1.00 42.28           C
ATOM   5396  C    ARG X  62    -30.888  80.776 -34.273  1.00 39.16           C
ATOM   5397  O    ARG X  62    -30.360  81.763 -34.795  1.00 42.60           O
ATOM   5398  CB   ARG X  62    -33.215  80.894 -35.260  1.00 40.18           C
ATOM   5399  CG   ARG X  62    -33.956  81.319 -34.029  1.00 45.22           C
ATOM   5400  CD   ARG X  62    -35.169  82.228 -34.403  1.00 57.00           C
ATOM   5401  NE   ARG X  62    -35.710  82.874 -33.214  1.00 45.98           N
ATOM   5402  CZ   ARG X  62    -36.485  82.241 -32.345  1.00 47.84           C
ATOM   5403  NH1  ARG X  62    -36.807  80.976 -32.560  1.00 50.34           N1+
ATOM   5404  NH2  ARG X  62    -36.923  82.855 -31.261  1.00 51.21           N
ATOM   5405  N    PHE X  63    -30.522  80.330 -33.074  1.00 40.25           N
ATOM   5406  CA   PHE X  63    -29.323  80.816 -32.410  1.00 41.09           C
ATOM   5407  C    PHE X  63    -28.171  79.886 -32.756  1.00 41.66           C
ATOM   5408  O    PHE X  63    -28.299  78.666 -32.622  1.00 35.82           O
ATOM   5409  CB   PHE X  63    -29.510  80.887 -30.891  1.00 33.46           C
ATOM   5410  CG   PHE X  63    -30.586  81.832 -30.473  1.00 36.45           C
ATOM   5411  CD1  PHE X  63    -30.310  83.184 -30.280  1.00 39.83           C
ATOM   5412  CD2  PHE X  63    -31.884  81.377 -30.287  1.00 34.93           C
ATOM   5413  CE1  PHE X  63    -31.325  84.085 -29.906  1.00 36.05           C
ATOM   5414  CE2  PHE X  63    -32.889  82.251 -29.923  1.00 38.93           C
ATOM   5415  CZ   PHE X  63    -32.614  83.614 -29.734  1.00 35.26           C
ATOM   5416  N    SER X  64    -27.053  80.455 -33.207  1.00 33.96           N
ATOM   5417  CA   SER X  64    -25.866  79.646 -33.436  1.00 36.60           C
ATOM   5418  C    SER X  64    -24.626  80.455 -33.101  1.00 33.97           C
ATOM   5419  O    SER X  64    -24.663  81.684 -32.984  1.00 37.04           O
ATOM   5420  CB   SER X  64    -25.792  79.134 -34.878  1.00 33.50           C
ATOM   5421  OG   SER X  64    -25.828  80.219 -35.797  1.00 41.55           O
ATOM   5422  N    GLY X  65    -23.514  79.743 -32.965  1.00 33.67           N
ATOM   5423  CA   GLY X  65    -22.271  80.366 -32.569  1.00 32.37           C
ATOM   5424  C    GLY X  65    -21.099  79.800 -33.342  1.00 37.26           C
ATOM   5425  O    GLY X  65    -21.144  78.694 -33.885  1.00 37.56           O
ATOM   5426  N    SER X  66    -20.044  80.596 -33.386  1.00 36.95           N
ATOM   5427  CA   SER X  66    -18.782  80.154 -33.937  1.00 39.89           C
ATOM   5428  C    SER X  66    -17.675  80.805 -33.137  1.00 38.97           C
ATOM   5429  O    SER X  66    -17.884  81.794 -32.427  1.00 42.94           O
ATOM   5430  CB   SER X  66    -18.645  80.521 -35.416  1.00 43.54           C
ATOM   5431  OG   SER X  66    -18.842  81.914 -35.579  1.00 44.16           O
ATOM   5432  N    LYS X  67    -16.493  80.236 -33.268  1.00 37.88           N
ATOM   5433  CA   LYS X  67    -15.318  80.724 -32.578  1.00 38.71           C
ATOM   5434  C    LYS X  67    -14.182  80.683 -33.584  1.00 41.62           C
ATOM   5435  O    LYS X  67    -14.049  79.712 -34.332  1.00 36.78           O
ATOM   5436  CB   LYS X  67    -14.996  79.866 -31.337  1.00 34.63           C
ATOM   5437  CG   LYS X  67    -13.718  80.278 -30.577  1.00 39.36           C
ATOM   5438  CD   LYS X  67    -13.483  79.408 -29.335  1.00 43.58           C
ATOM   5439  CE   LYS X  67    -12.029  79.470 -28.865  1.00 49.06           C
ATOM   5440  NZ   LYS X  67    -11.798  78.829 -27.509  1.00 45.19           N1+
ATOM   5441  N    SER X  68    -13.372  81.733 -33.603  1.00 32.64           N
ATOM   5442  CA   SER X  68    -12.213  81.766 -34.484  1.00 39.05           C
ATOM   5443  C    SER X  68    -11.107  82.533 -33.774  1.00 44.53           C
ATOM   5444  O    SER X  68    -11.196  83.760 -33.661  1.00 37.82           O
ATOM   5445  CB   SER X  68    -12.578  82.412 -35.816  1.00 40.60           C
ATOM   5446  OG   SER X  68    -11.432  82.493 -36.637  1.00 69.58           O
ATOM   5447  N    GLY X  69    -10.073  81.825 -33.344  1.00 41.45           N
ATOM   5448  CA   GLY X  69     -8.973  82.520 -32.683  1.00 38.45           C
ATOM   5449  C    GLY X  69     -9.424  83.162 -31.382  1.00 38.09           C
ATOM   5450  O    GLY X  69     -9.944  82.493 -30.485  1.00 40.05           O
ATOM   5451  N    THR X  70     -9.248  84.479 -31.260  1.00 38.95           N
ATOM   5452  CA   THR X  70     -9.575  85.191 -30.033  1.00 31.37           C
ATOM   5453  C    THR X  70    -10.914  85.919 -30.101  1.00 36.24           C
ATOM   5454  O    THR X  70    -11.192  86.796 -29.271  1.00 37.77           O
ATOM   5455  CB   THR X  70     -8.458  86.171 -29.692  1.00 36.81           C
ATOM   5456  OG1  THR X  70     -8.349  87.134 -30.744  1.00 30.74           O
ATOM   5457  CG2  THR X  70     -7.134  85.414 -29.544  1.00 25.19           C
ATOM   5458  N    SER X  71    -11.765  85.574 -31.048  1.00 37.57           N
ATOM   5459  CA   SER X  71    -13.081  86.175 -31.072  1.00 38.07           C
ATOM   5460  C    SER X  71    -14.125  85.082 -31.262  1.00 38.54           C
ATOM   5461  O    SER X  71    -13.834  83.992 -31.759  1.00 37.91           O
ATOM   5462  CB   SER X  71    -13.185  87.249 -32.162  1.00 40.23           C
```

```
ATOM   5463  OG   SER X  71    -13.241  86.662 -33.448  1.00 48.33           O
ATOM   5464  N    ALA X  72    -15.344  85.387 -30.821  1.00 30.70           N
ATOM   5465  CA   ALA X  72    -16.491  84.505 -30.944  1.00 32.65           C
ATOM   5466  C    ALA X  72    -17.649  85.344 -31.464  1.00 33.91           C
ATOM   5467  O    ALA X  72    -17.724  86.538 -31.199  1.00 35.37           O
ATOM   5468  CB   ALA X  72    -16.847  83.861 -29.596  1.00 28.85           C
ATOM   5469  N   ATHR X  73    -18.543  84.722 -32.219  0.60 34.81           N
ATOM   5470  CA  ATHR X  73    -19.686  85.428 -32.781  0.60 36.26           C
ATOM   5471  C   ATHR X  73    -20.968  84.644 -32.530  0.60 38.23           C
ATOM   5472  O   ATHR X  73    -21.021  83.423 -32.730  0.60 36.94           O
ATOM   5473  CB  ATHR X  73    -19.528  85.662 -34.285  0.60 37.26           C
ATOM   5474  OG1 ATHR X  73    -19.317  84.403 -34.929  0.60 39.56           O
ATOM   5475  CG2 ATHR X  73    -18.350  86.575 -34.575  0.60 38.40           C
ATOM   5476  N   BTHR X  73    -18.549  84.710 -32.211  0.40 34.91           N
ATOM   5477  CA  BTHR X  73    -19.684  85.400 -32.812  0.40 36.39           C
ATOM   5478  C   BTHR X  73    -20.970  84.636 -32.529  0.40 38.11           C
ATOM   5479  O   BTHR X  73    -21.026  83.413 -32.708  0.40 36.77           O
ATOM   5480  CB  BTHR X  73    -19.509  85.557 -34.327  0.40 37.24           C
ATOM   5481  OG1 BTHR X  73    -18.230  86.138 -34.608  0.40 37.63           O
ATOM   5482  CG2 BTHR X  73    -20.620  86.434 -34.910  0.40 32.90           C
ATOM   5483  N    LEU X  74    -21.997  85.359 -32.097  1.00 35.38           N
ATOM   5484  CA   LEU X  74    -23.326  84.804 -31.943  1.00 34.79           C
ATOM   5485  C    LEU X  74    -24.147  85.205 -33.156  1.00 36.97           C
ATOM   5486  O    LEU X  74    -24.156  86.379 -33.545  1.00 33.92           O
ATOM   5487  CB   LEU X  74    -23.986  85.314 -30.665  1.00 32.61           C
ATOM   5488  CG   LEU X  74    -25.507  85.206 -30.626  1.00 31.72           C
ATOM   5489  CD1  LEU X  74    -25.882  83.725 -30.473  1.00 30.32           C
ATOM   5490  CD2  LEU X  74    -26.036  86.026 -29.454  1.00 27.99           C
ATOM   5491  N    GLY X  75    -24.840  84.247 -33.745  1.00 36.52           N
ATOM   5492  CA   GLY X  75    -25.703  84.512 -34.884  1.00 34.24           C
ATOM   5493  C    GLY X  75    -27.148  84.242 -34.527  1.00 37.87           C
ATOM   5494  O    GLY X  75    -27.451  83.245 -33.871  1.00 37.63           O
ATOM   5495  N    ILE X  76    -28.035  85.141 -34.957  1.00 39.75           N
ATOM   5496  CA   ILE X  76    -29.479  84.985 -34.802  1.00 41.67           C
ATOM   5497  C    ILE X  76    -30.121  85.182 -36.168  1.00 42.37           C
ATOM   5498  O    ILE X  76    -30.094  86.290 -36.715  1.00 44.35           O
ATOM   5499  CB   ILE X  76    -30.078  85.974 -33.790  1.00 40.33           C
ATOM   5500  CG1  ILE X  76    -29.186  86.075 -32.552  1.00 41.66           C
ATOM   5501  CG2  ILE X  76    -31.481  85.526 -33.397  1.00 35.42           C
ATOM   5502  CD1  ILE X  76    -29.726  86.959 -31.465  1.00 37.72           C
ATOM   5503  N    THR X  77    -30.686  84.119 -36.725  1.00 43.91           N
ATOM   5504  CA   THR X  77    -31.472  84.236 -37.948  1.00 48.21           C
ATOM   5505  C    THR X  77    -32.958  84.232 -37.615  1.00 43.41           C
ATOM   5506  O    THR X  77    -33.376  83.754 -36.561  1.00 53.87           O
ATOM   5507  CB   THR X  77    -31.176  83.090 -38.915  1.00 48.60           C
ATOM   5508  OG1  THR X  77    -31.675  81.879 -38.346  1.00 50.81           O
ATOM   5509  CG2  THR X  77    -29.695  82.947 -39.136  1.00 39.95           C
ATOM   5510  N    GLY X  78    -33.757  84.758 -38.536  1.00 46.47           N
ATOM   5511  CA   GLY X  78    -35.202  84.734 -38.389  1.00 39.22           C
ATOM   5512  C    GLY X  78    -35.720  85.391 -37.128  1.00 45.27           C
ATOM   5513  O    GLY X  78    -36.522  84.791 -36.407  1.00 43.17           O
ATOM   5514  N    LEU X  79    -35.270  86.624 -36.870  1.00 46.54           N
ATOM   5515  CA   LEU X  79    -35.555  87.331 -35.624  1.00 38.18           C
ATOM   5516  C    LEU X  79    -37.039  87.325 -35.285  1.00 35.99           C
ATOM   5517  O    LEU X  79    -37.888  87.609 -36.131  1.00 36.59           O
ATOM   5518  CB   LEU X  79    -35.059  88.776 -35.730  1.00 39.04           C
ATOM   5519  CG   LEU X  79    -33.807  89.295 -34.996  1.00 40.52           C
ATOM   5520  CD1  LEU X  79    -33.440  88.520 -33.740  1.00 39.26           C
ATOM   5521  CD2  LEU X  79    -32.618  89.397 -35.910  1.00 54.14           C
ATOM   5522  N    GLN X  80    -37.345  87.044 -34.026  1.00 38.14           N
ATOM   5523  CA   GLN X  80    -38.697  87.144 -33.497  1.00 39.98           C
ATOM   5524  C    GLN X  80    -38.700  88.184 -32.389  1.00 41.62           C
ATOM   5525  O    GLN X  80    -37.668  88.422 -31.753  1.00 36.20           O
ATOM   5526  CB   GLN X  80    -39.186  85.813 -32.942  1.00 33.25           C
ATOM   5527  CG   GLN X  80    -39.044  84.680 -33.904  1.00 43.46           C
ATOM   5528  CD   GLN X  80    -39.580  83.358 -33.355  1.00 50.00           C
ATOM   5529  OE1  GLN X  80    -39.521  82.335 -34.031  1.00 49.67           O
ATOM   5530  NE2  GLN X  80    -40.101  83.378 -32.131  1.00 47.24           N
ATOM   5531  N    THR X  81    -39.865  88.806 -32.161  1.00 42.32           N
ATOM   5532  CA   THR X  81    -39.935  89.865 -31.159  1.00 40.35           C
ATOM   5533  C    THR X  81    -39.450  89.373 -29.794  1.00 36.41           C
```

346

```
ATOM   5534   O    THR X   81   -38.817   90.132  -29.050   1.00  35.09        O
ATOM   5535   CB   THR X   81   -41.369   90.415  -31.067   1.00  40.85        C
ATOM   5536   OG1  THR X   81   -42.261   89.347  -30.772   1.00  57.82        O
ATOM   5537   CG2  THR X   81   -41.798   90.998  -32.377   1.00  43.15        C
ATOM   5538   N    GLY X   82   -39.684   88.094  -29.474   1.00  36.68        N
ATOM   5539   CA   GLY X   82   -39.232   87.486  -28.236   1.00  28.64        C
ATOM   5540   C    GLY X   82   -37.724   87.287  -28.129   1.00  37.77        C
ATOM   5541   O    GLY X   82   -37.248   86.752  -27.124   1.00  34.17        O
ATOM   5542   N    ASP X   83   -36.959   87.652  -29.150   1.00  30.06        N
ATOM   5543   CA   ASP X   83   -35.509   87.630  -29.043   1.00  32.84        C
ATOM   5544   C    ASP X   83   -34.923   88.941  -28.517   1.00  34.01        C
ATOM   5545   O    ASP X   83   -33.729   88.984  -28.208   1.00  31.84        O
ATOM   5546   CB   ASP X   83   -34.907   87.303  -30.404   1.00  31.73        C
ATOM   5547   CG   ASP X   83   -35.427   85.990  -30.952   1.00  41.54        C
ATOM   5548   OD1  ASP X   83   -35.746   85.088  -30.133   1.00  36.80        O
ATOM   5549   OD2  ASP X   83   -35.522   85.868  -32.194   1.00  42.52        O1-
ATOM   5550   N    GLU X   84   -35.724   90.002  -28.424   1.00  31.24        N
ATOM   5551   CA   GLU X   84   -35.262   91.272  -27.871   1.00  37.59        C
ATOM   5552   C    GLU X   84   -34.719   91.031  -26.459   1.00  32.26        C
ATOM   5553   O    GLU X   84   -35.402   90.452  -25.609   1.00  35.56        O
ATOM   5554   CB   GLU X   84   -36.416   92.283  -27.890   1.00  30.20        C
ATOM   5555   CG   GLU X   84   -36.070   93.629  -27.345   1.00  45.30        C
ATOM   5556   CD   GLU X   84   -36.947   94.775  -27.890   1.00  45.92        C
ATOM   5557   OE1  GLU X   84   -37.343   95.625  -27.064   1.00  55.26        O
ATOM   5558   OE2  GLU X   84   -37.238   94.832  -29.112   1.00  39.84        O1-
ATOM   5559   N    ALA X   85   -33.455   91.385  -26.241   1.00  33.55        N
ATOM   5560   CA   ALA X   85   -32.741   90.944  -25.047   1.00  30.96        C
ATOM   5561   C    ALA X   85   -31.361   91.575  -25.034   1.00  28.54        C
ATOM   5562   O    ALA X   85   -30.916   92.168  -26.021   1.00  32.81        O
ATOM   5563   CB   ALA X   85   -32.594   89.409  -24.997   1.00  30.64        C
ATOM   5564   N    ASP X   86   -30.680   91.413  -23.903   1.00  29.41        N
ATOM   5565   CA   ASP X   86   -29.249   91.647  -23.793   1.00  31.96        C
ATOM   5566   C    ASP X   86   -28.504   90.322  -23.884   1.00  32.23        C
ATOM   5567   O    ASP X   86   -28.925   89.318  -23.303   1.00  33.09        O
ATOM   5568   CB   ASP X   86   -28.907   92.357  -22.482   1.00  33.01        C
ATOM   5569   CG   ASP X   86   -29.599   93.708  -22.367   1.00  39.16        C
ATOM   5570   OD1  ASP X   86   -29.651   94.437  -23.368   1.00  40.13        O
ATOM   5571   OD2  ASP X   86   -30.101   94.037  -21.280   1.00  48.35        O1-
ATOM   5572   N    TYR X   87   -27.386   90.329  -24.599   1.00  31.45        N
ATOM   5573   CA   TYR X   87   -26.601   89.129  -24.842   1.00  28.71        C
ATOM   5574   C    TYR X   87   -25.182   89.374  -24.358   1.00  32.26        C
ATOM   5575   O    TYR X   87   -24.609   90.435  -24.628   1.00  31.02        O
ATOM   5576   CB   TYR X   87   -26.621   88.762  -26.326   1.00  26.11        C
ATOM   5577   CG   TYR X   87   -28.002   88.352  -26.816   1.00  30.12        C
ATOM   5578   CD1  TYR X   87   -28.925   89.305  -27.224   1.00  29.60        C
ATOM   5579   CD2  TYR X   87   -28.378   87.014  -26.863   1.00  28.20        C
ATOM   5580   CE1  TYR X   87   -30.188   88.932  -27.678   1.00  34.26        C
ATOM   5581   CE2  TYR X   87   -29.626   86.630  -27.320   1.00  32.08        C
ATOM   5582   CZ   TYR X   87   -30.527   87.596  -27.726   1.00  34.03        C
ATOM   5583   OH   TYR X   87   -31.777   87.223  -28.150   1.00  31.35        O
ATOM   5584   N    TYR X   88   -24.632   88.394  -23.642   1.00  29.11        N
ATOM   5585   CA   TYR X   88   -23.311   88.458  -23.036   1.00  28.65        C
ATOM   5586   C    TYR X   88   -22.534   87.201  -23.398   1.00  32.64        C
ATOM   5587   O    TYR X   88   -23.054   86.084  -23.241   1.00  32.58        O
ATOM   5588   CB   TYR X   88   -23.408   88.547  -21.508   1.00  27.95        C
ATOM   5589   CG   TYR X   88   -24.118   89.757  -20.959   1.00  30.42        C
ATOM   5590   CD1  TYR X   88   -25.501   89.759  -20.792   1.00  28.19        C
ATOM   5591   CD2  TYR X   88   -23.401   90.890  -20.574   1.00  31.06        C
ATOM   5592   CE1  TYR X   88   -26.162   90.867  -20.275   1.00  27.13        C
ATOM   5593   CE2  TYR X   88   -24.041   92.005  -20.067   1.00  32.77        C
ATOM   5594   CZ   TYR X   88   -25.424   91.989  -19.920   1.00  36.54        C
ATOM   5595   OH   TYR X   88   -26.055   93.090  -19.405   1.00  30.95        O
ATOM   5596   N    CYS X   89   -21.287   87.372  -23.833   1.00  28.04        N
ATOM   5597   CA   CYS X   89   -20.334   86.269  -23.902   1.00  28.74        C
ATOM   5598   C    CYS X   89   -19.528   86.212  -22.607   1.00  32.37        C
ATOM   5599   O    CYS X   89   -19.378   87.212  -21.902   1.00  35.41        O
ATOM   5600   CB   CYS X   89   -19.378   86.436  -25.082   1.00  33.37        C
ATOM   5601   SG   CYS X   89   -18.463   88.060  -25.073   1.00  43.75        S
ATOM   5602   N    GLY X   90   -19.005   85.032  -22.303   1.00  32.61        N
ATOM   5603   CA   GLY X   90   -18.237   84.821  -21.083   1.00  31.53        C
ATOM   5604   C    GLY X   90   -17.235   83.695  -21.244   1.00  33.08        C
```

```
ATOM    5605  O    GLY X  90    -17.445  82.767 -22.030  1.00 35.81        O
ATOM    5606  N    THR X  91    -16.107  83.816 -20.531  1.00 25.99        N
ATOM    5607  CA   THR X  91    -15.089  82.778 -20.428  1.00 29.82        C
ATOM    5608  C    THR X  91    -14.309  82.951 -19.143  1.00 31.29        C
ATOM    5609  O    THR X  91    -14.533  83.878 -18.365  1.00 34.63        O
ATOM    5610  CB   THR X  91    -13.968  82.816 -21.479  1.00 38.46        C
ATOM    5611  OG1  THR X  91    -14.245  83.717 -22.547  1.00 45.88        O
ATOM    5612  CG2  THR X  91    -13.680  81.476 -21.973  1.00 27.98        C
ATOM    5613  N    TRP X  92    -13.306  82.097 -19.016  1.00 27.25        N
ATOM    5614  CA   TRP X  92    -12.219  82.230 -18.077  1.00 29.16        C
ATOM    5615  C    TRP X  92    -11.070  83.003 -18.714  1.00 32.78        C
ATOM    5616  O    TRP X  92    -10.800  82.873 -19.912  1.00 30.90        O
ATOM    5617  CB   TRP X  92    -11.757  80.836 -17.670  1.00 27.36        C
ATOM    5618  CG   TRP X  92    -10.715  80.810 -16.612  1.00 35.33        C
ATOM    5619  CD1  TRP X  92     -9.392  80.455 -16.757  1.00 34.95        C
ATOM    5620  CD2  TRP X  92    -10.900  81.116 -15.227  1.00 34.91        C
ATOM    5621  NE1  TRP X  92     -8.751  80.510 -15.535  1.00 33.88        N
ATOM    5622  CE2  TRP X  92     -9.647  80.927 -14.584  1.00 35.68        C
ATOM    5623  CE3  TRP X  92    -11.998  81.527 -14.465  1.00 29.27        C
ATOM    5624  CZ2  TRP X  92     -9.471  81.141 -13.221  1.00 32.90        C
ATOM    5625  CZ3  TRP X  92    -11.821  81.740 -13.107  1.00 30.95        C
ATOM    5626  CH2  TRP X  92    -10.571  81.542 -12.498  1.00 36.18        C
ATOM    5627  N    ASP X  93    -10.389  83.802 -17.915  1.00 34.71        N
ATOM    5628  CA   ASP X  93     -9.143  84.431 -18.340  1.00 35.49        C
ATOM    5629  C    ASP X  93     -8.004  83.809 -17.537  1.00 35.61        C
ATOM    5630  O    ASP X  93     -7.912  84.013 -16.322  1.00 38.60        O
ATOM    5631  CB   ASP X  93     -9.192  85.940 -18.151  1.00 30.03        C
ATOM    5632  CG   ASP X  93     -8.045  86.639 -18.857  1.00 39.44        C
ATOM    5633  OD1  ASP X  93     -6.878  86.282 -18.573  1.00 37.16        O1-
ATOM    5634  OD2  ASP X  93     -8.317  87.531 -19.701  1.00 32.36        O
ATOM    5635  N    SER X  94     -7.137  83.061 -18.223  1.00 33.93        N
ATOM    5636  CA   SER X  94     -6.072  82.302 -17.571  1.00 37.23        C
ATOM    5637  C    SER X  94     -5.010  83.199 -16.958  1.00 43.27        C
ATOM    5638  O    SER X  94     -4.321  82.779 -16.024  1.00 47.17        O
ATOM    5639  CB   SER X  94     -5.415  81.337 -18.557  1.00 35.59        C
ATOM    5640  OG   SER X  94     -6.349  80.369 -19.056  1.00 47.61        O
ATOM    5641  N    SER X  95     -4.851  84.415 -17.456  1.00 36.82        N
ATOM    5642  CA   SER X  95     -3.842  85.284 -16.877  1.00 40.13        C
ATOM    5643  C    SER X  95     -4.390  86.199 -15.784  1.00 43.12        C
ATOM    5644  O    SER X  95     -3.652  86.546 -14.858  1.00 44.13        O
ATOM    5645  CB   SER X  95     -3.177  86.104 -17.979  1.00 38.64        C
ATOM    5646  OG   SER X  95     -3.953  87.241 -18.283  1.00 52.16        O
ATOM    5647  N    LEU X  96     -5.653  86.623 -15.858  1.00 49.30        N
ATOM    5648  CA   LEU X  96     -6.260  87.359 -14.750  1.00 41.53        C
ATOM    5649  C    LEU X  96     -6.849  86.436 -13.685  1.00 36.33        C
ATOM    5650  O    LEU X  96     -7.332  86.925 -12.663  1.00 40.59        O
ATOM    5651  CB   LEU X  96     -7.345  88.299 -15.263  1.00 31.21        C
ATOM    5652  CG   LEU X  96     -6.975  89.345 -16.315  1.00 35.69        C
ATOM    5653  CD1  LEU X  96     -8.257  89.972 -16.878  1.00 32.73        C
ATOM    5654  CD2  LEU X  96     -6.072  90.443 -15.740  1.00 33.32        C
ATOM    5655  N    ASN X  97     -6.819  85.128 -13.908  1.00 33.69        N
ATOM    5656  CA   ASN X  97     -7.457  84.113 -13.054  1.00 40.52        C
ATOM    5657  C    ASN X  97     -8.862  84.515 -12.585  1.00 37.02        C
ATOM    5658  O    ASN X  97     -9.170  84.528 -11.394  1.00 38.37        O
ATOM    5659  CB   ASN X  97     -6.566  83.762 -11.863  1.00 40.15        C
ATOM    5660  CG   ASN X  97     -5.793  82.485 -12.103  1.00 56.68        C
ATOM    5661  OD1  ASN X  97     -6.057  81.431 -11.474  1.00 58.82        O
ATOM    5662  ND2  ASN X  97     -4.866  82.541 -13.069  1.00 45.20        N
ATOM    5663  N    THR X  98     -9.736  84.807 -13.551  1.00 33.40        N
ATOM    5664  CA   THR X  98    -11.086  85.225 -13.198  1.00 36.45        C
ATOM    5665  C    THR X  98    -12.042  84.934 -14.345  1.00 31.02        C
ATOM    5666  O    THR X  98    -11.637  84.809 -15.497  1.00 29.12        O
ATOM    5667  CB   THR X  98    -11.138  86.713 -12.830  1.00 32.72        C
ATOM    5668  OG1  THR X  98    -12.455  87.038 -12.382  1.00 37.61        O
ATOM    5669  CG2  THR X  98    -10.840  87.569 -14.031  1.00 32.00        C
ATOM    5670  N    VAL X  99    -13.326  84.815 -14.005  1.00 30.06        N
ATOM    5671  CA   VAL X  99    -14.358  84.769 -15.027  1.00 24.45        C
ATOM    5672  C    VAL X  99    -14.563  86.160 -15.611  1.00 29.75        C
ATOM    5673  O    VAL X  99    -14.620  87.174 -14.897  1.00 26.93        O
ATOM    5674  CB   VAL X  99    -15.680  84.233 -14.460  1.00 28.00        C
ATOM    5675  CG1  VAL X  99    -16.739  84.291 -15.532  1.00 26.99        C
```

```
ATOM   5676  CG2 VAL X  99     -15.525  82.801 -13.869  1.00 29.57           C
ATOM   5677  N   VAL X 100     -14.775  86.208 -16.906  1.00 29.21           N
ATOM   5678  CA  VAL X 100     -14.748  87.464 -17.623  1.00 28.67           C
ATOM   5679  C   VAL X 100     -15.948  87.512 -18.574  1.00 32.26           C
ATOM   5680  O   VAL X 100     -16.225  86.546 -19.296  1.00 33.30           O
ATOM   5681  CB  VAL X 100     -13.355  87.559 -18.285  1.00 34.89           C
ATOM   5682  CG1 VAL X 100     -13.294  87.188 -19.773  1.00 28.07           C
ATOM   5683  CG2 VAL X 100     -12.657  88.799 -17.889  1.00 30.67           C
ATOM   5684  N   PHE X 101     -16.709  88.599 -18.512  1.00 29.76           N
ATOM   5685  CA  PHE X 101     -17.876  88.803 -19.365  1.00 37.29           C
ATOM   5686  C   PHE X 101     -17.634  89.939 -20.348  1.00 33.64           C
ATOM   5687  O   PHE X 101     -16.940  90.913 -20.039  1.00 36.04           O
ATOM   5688  CB  PHE X 101     -19.138  89.186 -18.567  1.00 33.79           C
ATOM   5689  CG  PHE X 101     -19.780  88.069 -17.808  1.00 32.73           C
ATOM   5690  CD1 PHE X 101     -20.482  87.064 -18.464  1.00 38.68           C
ATOM   5691  CD2 PHE X 101     -19.729  88.057 -16.407  1.00 31.51           C
ATOM   5692  CE1 PHE X 101     -21.114  86.044 -17.743  1.00 34.99           C
ATOM   5693  CE2 PHE X 101     -20.345  87.044 -15.674  1.00 28.97           C
ATOM   5694  CZ  PHE X 101     -21.040  86.028 -16.349  1.00 30.18           C
ATOM   5695  N   GLY X 102     -18.283  89.846 -21.507  1.00 31.53           N
ATOM   5696  CA  GLY X 102     -18.420  91.004 -22.354  1.00 30.93           C
ATOM   5697  C   GLY X 102     -19.343  92.039 -21.708  1.00 38.01           C
ATOM   5698  O   GLY X 102     -20.047  91.780 -20.724  1.00 32.78           O
ATOM   5699  N   GLY X 103     -19.311  93.246 -22.272  1.00 29.12           N
ATOM   5700  CA  GLY X 103     -20.117  94.353 -21.794  1.00 29.37           C
ATOM   5701  C   GLY X 103     -21.583  94.244 -22.120  1.00 33.08           C
ATOM   5702  O   GLY X 103     -22.376  95.015 -21.576  1.00 35.44           O
ATOM   5703  N   GLY X 104     -21.964  93.300 -22.974  1.00 34.27           N
ATOM   5704  CA  GLY X 104     -23.358  93.149 -23.326  1.00 29.50           C
ATOM   5705  C   GLY X 104     -23.731  93.836 -24.619  1.00 29.53           C
ATOM   5706  O   GLY X 104     -23.197  94.891 -24.951  1.00 32.35           O
ATOM   5707  N   THR X 105     -24.636  93.220 -25.369  1.00 34.88           N
ATOM   5708  CA  THR X 105     -25.182  93.780 -26.591  1.00 32.20           C
ATOM   5709  C   THR X 105     -26.689  93.826 -26.424  1.00 34.52           C
ATOM   5710  O   THR X 105     -27.326  92.787 -26.221  1.00 32.97           O
ATOM   5711  CB  THR X 105     -24.791  92.943 -27.812  1.00 30.26           C
ATOM   5712  OG1 THR X 105     -23.367  92.949 -27.935  1.00 36.62           O
ATOM   5713  CG2 THR X 105     -25.397  93.520 -29.084  1.00 27.38           C
ATOM   5714  N   LYS X 106     -27.246  95.027 -26.490  1.00 33.22           N
ATOM   5715  CA  LYS X 106     -28.686  95.209 -26.472  1.00 35.13           C
ATOM   5716  C   LYS X 106     -29.210  94.955 -27.877  1.00 32.47           C
ATOM   5717  O   LYS X 106     -28.824  95.647 -28.822  1.00 37.14           O
ATOM   5718  CB  LYS X 106     -29.035  96.619 -26.001  1.00 33.00           C
ATOM   5719  CG  LYS X 106     -30.515  96.974 -26.093  1.00 40.50           C
ATOM   5720  CD  LYS X 106     -30.760  98.406 -25.536  1.00 50.00           C
ATOM   5721  CE  LYS X 106     -32.202  98.919 -25.776  1.00 54.95           C
ATOM   5722  NZ  LYS X 106     -33.307  98.040 -25.259  1.00 53.87           N1+
ATOM   5723  N   LEU X 107     -30.039  93.941 -28.022  1.00 31.69           N
ATOM   5724  CA  LEU X 107     -30.702  93.641 -29.282  1.00 35.95           C
ATOM   5725  C   LEU X 107     -32.109  94.195 -29.217  1.00 34.06           C
ATOM   5726  O   LEU X 107     -32.890  93.810 -28.335  1.00 36.06           O
ATOM   5727  CB  LEU X 107     -30.764  92.135 -29.542  1.00 29.30           C
ATOM   5728  CG  LEU X 107     -31.101  91.566 -30.925  1.00 36.45           C
ATOM   5729  CD1 LEU X 107     -31.582  90.117 -30.792  1.00 47.08           C
ATOM   5730  CD2 LEU X 107     -32.108  92.310 -31.732  1.00 35.75           C
ATOM   5731  N  ATHR X 108     -32.447  95.068 -30.150  0.50 29.34           N
ATOM   5732  CA ATHR X 108     -33.819  95.519 -30.281  0.50 34.47           C
ATOM   5733  C  ATHR X 108     -34.372  94.993 -31.598  0.50 34.43           C
ATOM   5734  O  ATHR X 108     -33.730  95.114 -32.645  0.50 33.84           O
ATOM   5735  CB ATHR X 108     -33.929  97.050 -30.167  0.50 35.43           C
ATOM   5736  OG1ATHR X 108     -34.721  97.575 -31.244  0.50 39.77           O
ATOM   5737  CG2ATHR X 108     -32.553  97.713 -30.114  0.50 36.64           C
ATOM   5738  N  BTHR X 108     -32.435  95.083 -30.149  0.50 28.73           N
ATOM   5739  CA BTHR X 108     -33.795  95.559 -30.334  0.50 34.59           C
ATOM   5740  C  BTHR X 108     -34.344  94.934 -31.610  0.50 34.22           C
ATOM   5741  O  BTHR X 108     -33.682  94.959 -32.651  0.50 33.77           O
ATOM   5742  CB BTHR X 108     -33.864  97.095 -30.407  0.50 35.40           C
ATOM   5743  OG1BTHR X 108     -33.311  97.671 -29.217  0.50 29.14           O
ATOM   5744  CG2BTHR X 108     -35.332  97.571 -30.561  0.50 36.81           C
ATOM   5745  N   VAL X 109     -35.533  94.354 -31.521  1.00 34.56           N
ATOM   5746  CA  VAL X 109     -36.242  93.847 -32.687  1.00 37.76           C
```

349

```
ATOM   5747  C    VAL X 109    -37.211  94.947 -33.124  1.00 37.56           C
ATOM   5748  O    VAL X 109    -38.195  95.233 -32.435  1.00 39.51           O
ATOM   5749  CB   VAL X 109    -36.957  92.526 -32.370  1.00 36.89           C
ATOM   5750  CG1  VAL X 109    -37.657  91.986 -33.592  1.00 37.00           C
ATOM   5751  CG2  VAL X 109    -35.944  91.498 -31.854  1.00 35.12           C
ATOM   5752  N    LEU X 110    -36.903  95.596 -34.249  1.00 40.19           N
ATOM   5753  CA   LEU X 110    -37.598  96.804 -34.693  1.00 37.97           C
ATOM   5754  C    LEU X 110    -39.089  96.596 -34.915  1.00 42.37           C
ATOM   5755  O    LEU X 110    -39.492  95.862 -35.816  1.00 42.06           O
ATOM   5756  CB   LEU X 110    -36.952  97.330 -35.974  1.00 32.89           C
ATOM   5757  CG   LEU X 110    -35.483  97.738 -35.777  1.00 44.78           C
ATOM   5758  CD1  LEU X 110    -34.740  97.954 -37.095  1.00 36.15           C
ATOM   5759  CD2  LEU X 110    -35.451  98.999 -34.936  1.00 37.61           C
ATOM   5760  N    SER X 111    -39.923  97.222 -34.090  1.00 44.41           N
ATOM   5761  CA   SER X 111    -41.366  97.119 -34.268  1.00 50.21           C
ATOM   5762  C    SER X 111    -42.007  98.460 -34.601  1.00 49.69           C
ATOM   5763  O    SER X 111    -43.235  98.560 -34.605  1.00 52.76           O
ATOM   5764  CB   SER X 111    -42.017  96.500 -33.028  1.00 44.40           C
ATOM   5765  OG   SER X 111    -41.726  97.270 -31.878  1.00 60.88           O
ATOM   5766  N    GLN X 112    -41.211  99.487 -34.878  1.00 47.15           N
ATOM   5767  CA   GLN X 112    -41.707 100.757 -35.390  1.00 48.33           C
ATOM   5768  C    GLN X 112    -40.538 101.473 -36.048  1.00 46.20           C
ATOM   5769  O    GLN X 112    -39.389 101.022 -35.937  1.00 50.86           O
ATOM   5770  CB   GLN X 112    -42.328 101.613 -34.268  1.00 45.44           C
ATOM   5771  CG   GLN X 112    -41.332 102.017 -33.210  1.00 54.29           C
ATOM   5772  CD   GLN X 112    -41.921 102.957 -32.174  1.00 55.17           C
ATOM   5773  OE1  GLN X 112    -42.268 102.535 -31.064  1.00 50.12           O
ATOM   5774  NE2  GLN X 112    -42.016 104.247 -32.523  1.00 51.29           N
ATOM   5775  N    PRO X 113    -40.791 102.573 -36.760  1.00 47.86      GZ00 N
ATOM   5776  CA   PRO X 113    -39.684 103.305 -37.392  1.00 44.33      GZ00 C
ATOM   5777  C    PRO X 113    -38.722 103.861 -36.360  1.00 47.98      GZ00 C
ATOM   5778  O    PRO X 113    -39.099 104.165 -35.229  1.00 51.31      GZ00 O
ATOM   5779  CB   PRO X 113    -40.386 104.439 -38.148  1.00 44.96      GZ00 C
ATOM   5780  CG   PRO X 113    -41.754 103.943 -38.377  1.00 46.92      GZ00 C
ATOM   5781  CD   PRO X 113    -42.098 103.123 -37.162  1.00 47.64      GZ00 C
ATOM   5782  N    LYS X 114    -37.464 104.002 -36.764  1.00 48.55      GZ00 N
ATOM   5783  CA   LYS X 114    -36.492 104.609 -35.876  1.00 42.68      GZ00 C
ATOM   5784  C    LYS X 114    -36.864 106.065 -35.628  1.00 49.83      GZ00 C
ATOM   5785  O    LYS X 114    -37.429 106.739 -36.491  1.00 52.51      GZ00 O
ATOM   5786  CB   LYS X 114    -35.082 104.485 -36.457  1.00 45.63      GZ00 C
ATOM   5787  CG   LYS X 114    -34.596 103.034 -36.553  1.00 50.09      GZ00 C
ATOM   5788  CD   LYS X 114    -33.151 102.950 -37.039  1.00 58.16      GZ00 C
ATOM   5789  CE   LYS X 114    -32.468 101.608 -36.704  1.00 40.47      GZ00 C
ATOM   5790  NZ   LYS X 114    -30.975 101.802 -36.780  1.00 37.72      GZ00 N1+
ATOM   5791  N    ALA X 115    -36.559 106.540 -34.424  1.00 48.82      GZ00 N
ATOM   5792  CA   ALA X 115    -36.951 107.875 -33.987  1.00 42.61      GZ00 C
ATOM   5793  C    ALA X 115    -35.771 108.535 -33.298  1.00 43.87      GZ00 C
ATOM   5794  O    ALA X 115    -35.255 108.014 -32.302  1.00 42.55      GZ00 O
ATOM   5795  CB   ALA X 115    -38.151 107.809 -33.040  1.00 37.00      GZ00 C
ATOM   5796  N    ALA X 116    -35.349 109.676 -33.825  1.00 42.05      GZ00 N
ATOM   5797  CA   ALA X 116    -34.281 110.428 -33.189  1.00 51.37      GZ00 C
ATOM   5798  C    ALA X 116    -34.776 111.034 -31.871  1.00 46.85      GZ00 C
ATOM   5799  O    ALA X 116    -35.976 111.217 -31.674  1.00 42.99      GZ00 O
ATOM   5800  CB   ALA X 116    -33.779 111.525 -34.121  1.00 48.36      GZ00 C
ATOM   5801  N    PRO X 117    -33.881 111.279 -30.922  1.00 47.49      GZ00 N
ATOM   5802  CA   PRO X 117    -34.320 111.846 -29.642  1.00 49.41      GZ00 C
ATOM   5803  C    PRO X 117    -34.616 113.337 -29.751  1.00 53.03      GZ00 C
ATOM   5804  O    PRO X 117    -33.903 114.088 -30.426  1.00 49.88      GZ00 O
ATOM   5805  CB   PRO X 117    -33.120 111.582 -28.721  1.00 48.21      GZ00 C
ATOM   5806  CG   PRO X 117    -31.929 111.617 -29.651  1.00 40.15      GZ00 C
ATOM   5807  CD   PRO X 117    -32.431 110.999 -30.939  1.00 47.80      GZ00 C
ATOM   5808  N    SER X 118    -35.660 113.776 -29.048  1.00 43.61      GZ00 N
ATOM   5809  CA   SER X 118    -35.846 115.206 -28.812  1.00 49.92      GZ00 C
ATOM   5810  C    SER X 118    -35.152 115.555 -27.504  1.00 43.16      GZ00 C
ATOM   5811  O    SER X 118    -35.266 114.824 -26.515  1.00 50.92      GZ00 O
ATOM   5812  CB   SER X 118    -37.325 115.608 -28.770  1.00 47.75      GZ00 C
ATOM   5813  OG   SER X 118    -37.961 115.002 -27.671  1.00 58.79      GZ00 O
ATOM   5814  N    VAL X 119    -34.377 116.627 -27.528  1.00 41.52      GZ00 N
ATOM   5815  CA   VAL X 119    -33.512 117.022 -26.426  1.00 44.89      GZ00 C
ATOM   5816  C    VAL X 119    -33.886 118.439 -26.021  1.00 52.97      GZ00 C
ATOM   5817  O    VAL X 119    -33.914 119.338 -26.869  1.00 55.14      GZ00 O
```

```
ATOM   5818  CB   VAL X 119     -32.030 116.957 -26.837  1.00 42.97      GZ00 C
ATOM   5819  CG1  VAL X 119     -31.122 117.319 -25.668  1.00 42.13      GZ00 C
ATOM   5820  CG2  VAL X 119     -31.694 115.590 -27.416  1.00 46.14      GZ00 C
ATOM   5821  N    THR X 120     -34.183 118.644 -24.741  1.00 53.84      GZ00 N
ATOM   5822  CA   THR X 120     -34.297 119.997 -24.223  1.00 52.90      GZ00 C
ATOM   5823  C    THR X 120     -33.396 120.136 -23.000  1.00 49.02      GZ00 C
ATOM   5824  O    THR X 120     -33.365 119.257 -22.135  1.00 44.38      GZ00 O
ATOM   5825  CB   THR X 120     -35.779 120.402 -23.935  1.00 46.21      GZ00 C
ATOM   5826  OG1  THR X 120     -35.982 120.611 -22.536  1.00 53.27      GZ00 O
ATOM   5827  CG2  THR X 120     -36.767 119.365 -24.468  1.00 51.58      GZ00 C
ATOM   5828  N    LEU X 121     -32.652 121.246 -22.960  1.00 49.59      GZ00 N
ATOM   5829  CA   LEU X 121     -31.580 121.506 -22.006  1.00 46.69      GZ00 C
ATOM   5830  C    LEU X 121     -31.926 122.718 -21.147  1.00 49.22      GZ00 C
ATOM   5831  O    LEU X 121     -32.183 123.802 -21.676  1.00 57.48      GZ00 O
ATOM   5832  CB   LEU X 121     -30.282 121.762 -22.756  1.00 42.85      GZ00 C
ATOM   5833  CG   LEU X 121     -28.925 121.456 -22.159  1.00 49.09      GZ00 C
ATOM   5834  CD1  LEU X 121     -27.908 122.373 -22.827  1.00 42.63      GZ00 C
ATOM   5835  CD2  LEU X 121     -28.888 121.557 -20.653  1.00 50.57      GZ00 C
ATOM   5836  N    PHE X 122     -31.952 122.534 -19.834  1.00 51.49      GZ00 N
ATOM   5837  CA   PHE X 122     -32.197 123.630 -18.917  1.00 48.21      GZ00 C
ATOM   5838  C    PHE X 122     -30.914 124.009 -18.215  1.00 52.42      GZ00 C
ATOM   5839  O    PHE X 122     -30.201 123.122 -17.727  1.00 47.78      GZ00 O
ATOM   5840  CB   PHE X 122     -33.245 123.263 -17.872  1.00 44.54      GZ00 C
ATOM   5841  CG   PHE X 122     -34.637 123.136 -18.419  1.00 55.06      GZ00 C
ATOM   5842  CD1  PHE X 122     -35.407 124.269 -18.664  1.00 49.10      GZ00 C
ATOM   5843  CD2  PHE X 122     -35.197 121.885 -18.648  1.00 51.91      GZ00 C
ATOM   5844  CE1  PHE X 122     -36.697 124.155 -19.151  1.00 52.05      GZ00 C
ATOM   5845  CE2  PHE X 122     -36.487 121.766 -19.133  1.00 44.48      GZ00 C
ATOM   5846  CZ   PHE X 122     -37.238 122.899 -19.385  1.00 49.83      GZ00 C
ATOM   5847  N    PRO X 123     -30.613 125.302 -18.116  1.00 55.21      GZ00 N
ATOM   5848  CA   PRO X 123     -29.470 125.760 -17.316  1.00 49.75      GZ00 C
ATOM   5849  C    PRO X 123     -29.834 125.768 -15.844  1.00 49.06      GZ00 C
ATOM   5850  O    PRO X 123     -31.016 125.607 -15.493  1.00 43.82      GZ00 O
ATOM   5851  CB   PRO X 123     -29.233 127.183 -17.842  1.00 55.50      GZ00 C
ATOM   5852  CG   PRO X 123     -30.612 127.648 -18.178  1.00 51.07      GZ00 C
ATOM   5853  CD   PRO X 123     -31.353 126.426 -18.710  1.00 47.80      GZ00 C
ATOM   5854  N    PRO X 124     -28.866 125.964 -14.950  1.00 48.22      GZ00 N
ATOM   5855  CA   PRO X 124     -29.212 126.031 -13.526  1.00 50.20      GZ00 C
ATOM   5856  C    PRO X 124     -30.040 127.272 -13.245  1.00 49.48      GZ00 C
ATOM   5857  O    PRO X 124     -29.819 128.331 -13.832  1.00 60.15      GZ00 O
ATOM   5858  CB   PRO X 124     -27.850 126.075 -12.822  1.00 45.39      GZ00 C
ATOM   5859  CG   PRO X 124     -26.917 126.606 -13.838  1.00 51.55      GZ00 C
ATOM   5860  CD   PRO X 124     -27.423 126.159 -15.178  1.00 47.41      GZ00 C
ATOM   5861  N    SER X 125     -31.021 127.122 -12.367  1.00 47.01      GZ00 N
ATOM   5862  CA   SER X 125     -31.863 128.243 -11.982  1.00 52.47      GZ00 C
ATOM   5863  C    SER X 125     -31.107 129.183 -11.033  1.00 58.92      GZ00 C
ATOM   5864  O    SER X 125     -30.113 128.806 -10.387  1.00 46.42      GZ00 O
ATOM   5865  CB   SER X 125     -33.156 127.749 -11.322  1.00 46.03      GZ00 C
ATOM   5866  OG   SER X 125     -32.890 127.191 -10.049  1.00 47.81      GZ00 O
ATOM   5867  N    SER X 126     -31.591 130.436 -10.966  1.00 57.48      GZ00 N
ATOM   5868  CA   SER X 126     -30.955 131.426 -10.096  1.00 51.60      GZ00 C
ATOM   5869  C    SER X 126     -31.069 131.029  -8.631  1.00 45.30      GZ00 C
ATOM   5870  O    SER X 126     -30.103 131.177  -7.870  1.00 56.73      GZ00 O
ATOM   5871  CB   SER X 126     -31.552 132.818 -10.327  1.00 46.73      GZ00 C
ATOM   5872  OG   SER X 126     -32.956 132.793 -10.192  1.00 64.48      GZ00 O
ATOM   5873  N    GLU X 127     -32.222 130.484  -8.219  1.00 49.07      GZ00 N
ATOM   5874  CA   GLU X 127     -32.347 130.017  -6.839  1.00 53.87      GZ00 C
ATOM   5875  C    GLU X 127     -31.302 128.962  -6.507  1.00 55.39      GZ00 C
ATOM   5876  O    GLU X 127     -30.761 128.948  -5.394  1.00 53.30      GZ00 O
ATOM   5877  CB   GLU X 127     -33.733 129.444  -6.558  1.00 46.74      GZ00 C
ATOM   5878  CG   GLU X 127     -34.909 130.267  -7.005  1.00 50.20      GZ00 C
ATOM   5879  CD   GLU X 127     -36.221 129.697  -6.458  1.00 67.37      GZ00 C
ATOM   5880  OE1  GLU X 127     -36.163 128.878  -5.501  1.00 58.54      GZ00 O
ATOM   5881  OE2  GLU X 127     -37.303 130.059  -6.988  1.00 72.90      GZ00 O1-
ATOM   5882  N    GLU X 128     -30.978 128.088  -7.466  1.00 52.88      GZ00 N
ATOM   5883  CA   GLU X 128     -30.012 127.035  -7.162  1.00 58.48      GZ00 C
ATOM   5884  C    GLU X 128     -28.600 127.593  -7.114  1.00 54.84      GZ00 C
ATOM   5885  O    GLU X 128     -27.778 127.141  -6.305  1.00 50.17      GZ00 O
ATOM   5886  CB   GLU X 128     -30.100 125.882  -8.171  1.00 51.98      GZ00 C
ATOM   5887  CG   GLU X 128     -29.266 124.683  -7.739  1.00 50.39      GZ00 C
ATOM   5888  CD   GLU X 128     -29.022 123.641  -8.841  1.00 56.66      GZ00 C
```

```
ATOM   5889  OE1 GLU X 128    -28.576 122.533  -8.481  1.00 51.79    GZ00 O
ATOM   5890  OE2 GLU X 128    -29.257 123.912 -10.044  1.00 50.04    GZ00 O1-
ATOM   5891  N   LEU X 129    -28.300 128.569  -7.971  1.00 51.67    GZ00 N
ATOM   5892  CA  LEU X 129    -27.020 129.247  -7.835  1.00 57.61    GZ00 C
ATOM   5893  C   LEU X 129    -26.899 129.856  -6.440  1.00 54.69    GZ00 C
ATOM   5894  O   LEU X 129    -25.876 129.685  -5.768  1.00 56.10    GZ00 O
ATOM   5895  CB  LEU X 129    -26.861 130.296  -8.938  1.00 51.14    GZ00 C
ATOM   5896  CG  LEU X 129    -26.749 129.691 -10.350  1.00 59.65    GZ00 C
ATOM   5897  CD1 LEU X 129    -26.772 130.745 -11.454  1.00 51.42    GZ00 C
ATOM   5898  CD2 LEU X 129    -25.537 128.778 -10.502  1.00 48.62    GZ00 C
ATOM   5899  N   GLN X 130    -27.979 130.476  -5.947  1.00 51.64    GZ00 N
ATOM   5900  CA  GLN X 130    -27.987 131.040  -4.600  1.00 54.55    GZ00 C
ATOM   5901  C   GLN X 130    -27.717 130.007  -3.525  1.00 57.65    GZ00 C
ATOM   5902  O   GLN X 130    -27.308 130.384  -2.425  1.00 62.14    GZ00 O
ATOM   5903  CB  GLN X 130    -29.325 131.707  -4.308  1.00 49.30    GZ00 C
ATOM   5904  CG  GLN X 130    -29.494 133.013  -5.001  1.00 55.98    GZ00 C
ATOM   5905  CD  GLN X 130    -29.020 134.140  -4.135  1.00 68.65    GZ00 C
ATOM   5906  OE1 GLN X 130    -27.848 134.527  -4.169  1.00 64.86    GZ00 O
ATOM   5907  NE2 GLN X 130    -29.925 134.657  -3.313  1.00 63.69    GZ00 N
ATOM   5908  N   ALA X 131    -27.980 128.727  -3.788  1.00 58.30    GZ00 N
ATOM   5909  CA  ALA X 131    -27.635 127.669  -2.849  1.00 53.42    GZ00 C
ATOM   5910  C   ALA X 131    -26.223 127.155  -3.057  1.00 55.16    GZ00 C
ATOM   5911  O   ALA X 131    -25.840 126.154  -2.444  1.00 55.67    GZ00 O
ATOM   5912  CB  ALA X 131    -28.629 126.517  -2.951  1.00 53.39    GZ00 C
ATOM   5913  N   ASN X 132    -25.445 127.820  -3.909  1.00 57.80    GZ00 N
ATOM   5914  CA  ASN X 132    -24.062 127.430  -4.188  1.00 65.38    GZ00 C
ATOM   5915  C   ASN X 132    -23.983 126.038  -4.819  1.00 64.86    GZ00 C
ATOM   5916  O   ASN X 132    -23.125 125.221  -4.474  1.00 58.78    GZ00 O
ATOM   5917  CB  ASN X 132    -23.198 127.502  -2.930  1.00 57.21    GZ00 C
ATOM   5918  CG  ASN X 132    -21.742 127.673  -3.259  1.00 66.70    GZ00 C
ATOM   5919  OD1 ASN X 132    -21.389 128.368  -4.221  1.00 68.31    GZ00 O
ATOM   5920  ND2 ASN X 132    -20.880 127.021  -2.485  1.00 71.71    GZ00 N
ATOM   5921  N   LYS X 133    -24.891 125.766  -5.750  1.00 63.21    GZ00 N
ATOM   5922  CA  LYS X 133    -24.866 124.558  -6.558  1.00 55.69    GZ00 C
ATOM   5923  C   LYS X 133    -25.304 124.950  -7.959  1.00 58.70    GZ00 C
ATOM   5924  O   LYS X 133    -25.800 126.057  -8.195  1.00 60.57    GZ00 O
ATOM   5925  CB  LYS X 133    -25.778 123.468  -5.978  1.00 57.74    GZ00 C
ATOM   5926  CG  LYS X 133    -25.404 122.972  -4.574  1.00 60.29    GZ00 C
ATOM   5927  CD  LYS X 133    -26.528 122.090  -3.989  1.00 77.16    GZ00 C
ATOM   5928  CE  LYS X 133    -26.295 121.712  -2.516  1.00 67.68    GZ00 C
ATOM   5929  NZ  LYS X 133    -27.578 121.494  -1.757  1.00 61.53    GZ00 N1+
ATOM   5930  N   ALA X 134    -25.128 124.033  -8.899  1.00 57.91    GZ00 N
ATOM   5931  CA  ALA X 134    -25.550 124.292 -10.273  1.00 57.46    GZ00 C
ATOM   5932  C   ALA X 134    -25.676 122.951 -10.970  1.00 58.56    GZ00 C
ATOM   5933  O   ALA X 134    -24.715 122.173 -10.980  1.00 56.94    GZ00 O
ATOM   5934  CB  ALA X 134    -24.546 125.194 -10.994  1.00 46.64    GZ00 C
ATOM   5935  N   THR X 135    -26.850 122.650 -11.519  1.00 55.26    GZ00 N
ATOM   5936  CA  THR X 135    -26.986 121.434 -12.308  1.00 47.36    GZ00 C
ATOM   5937  C   THR X 135    -27.631 121.778 -13.640  1.00 45.07    GZ00 C
ATOM   5938  O   THR X 135    -28.602 122.540 -13.693  1.00 43.66    GZ00 O
ATOM   5939  CB  THR X 135    -27.779 120.335 -11.579  1.00 46.59    GZ00 C
ATOM   5940  OG1 THR X 135    -29.174 120.523 -11.787  1.00 60.99    GZ00 O
ATOM   5941  CG2 THR X 135    -27.487 120.331 -10.088  1.00 40.51    GZ00 C
ATOM   5942  N   LEU X 136    -27.050 121.251 -14.711  1.00 43.91    GZ00 N
ATOM   5943  CA  LEU X 136    -27.635 121.315 -16.036  1.00 42.15    GZ00 C
ATOM   5944  C   LEU X 136    -28.501 120.080 -16.231  1.00 50.30    GZ00 C
ATOM   5945  O   LEU X 136    -28.140 118.976 -15.812  1.00 46.46    GZ00 O
ATOM   5946  CB  LEU X 136    -26.544 121.383 -17.101  1.00 46.62    GZ00 C
ATOM   5947  CG  LEU X 136    -25.574 122.574 -17.036  1.00 48.02    GZ00 C
ATOM   5948  CD1 LEU X 136    -24.445 122.431 -18.044  1.00 53.43    GZ00 C
ATOM   5949  CD2 LEU X 136    -26.330 123.797 -17.372  1.00 47.48    GZ00 C
ATOM   5950  N   VAL X 137    -29.654 120.270 -16.849  1.00 48.09    GZ00 N
ATOM   5951  CA  VAL X 137    -30.643 119.214 -16.962  1.00 49.70    GZ00 C
ATOM   5952  C   VAL X 137    -30.865 118.972 -18.443  1.00 47.78    GZ00 C
ATOM   5953  O   VAL X 137    -31.364 119.852 -19.156  1.00 46.56    GZ00 O
ATOM   5954  CB  VAL X 137    -31.951 119.574 -16.249  1.00 41.53    GZ00 C
ATOM   5955  CG1 VAL X 137    -32.927 118.433 -16.354  1.00 38.78    GZ00 C
ATOM   5956  CG2 VAL X 137    -31.677 119.926 -14.782  1.00 37.08    GZ00 C
ATOM   5957  N   CYS X 138    -30.480 117.787 -18.905  1.00 41.44    GZ00 N
ATOM   5958  CA  CYS X 138    -30.653 117.371 -20.292  1.00 44.75    GZ00 C
ATOM   5959  C   CYS X 138    -31.717 116.286 -20.316  1.00 42.51    GZ00 C
```

```
ATOM   5960  O   CYS X 138     -31.459 115.150 -19.904  1.00 45.75          GZ00 O
ATOM   5961  CB  CYS X 138     -29.341 116.850 -20.872  1.00 41.85          GZ00 C
ATOM   5962  SG  CYS X 138     -29.335 116.711 -22.686  1.00 48.77          GZ00 S
ATOM   5963  N   LEU X 139     -32.904 115.627 -20.803  1.00 40.25          GZ00 N
ATOM   5964  CA  LEU X 139     -33.998 115.670 -20.918  1.00 44.13          GZ00 C
ATOM   5965  C   LEU X 139     -34.091 115.164 -22.348  1.00 48.33          GZ00 C
ATOM   5966  O   LEU X 139     -34.096 115.956 -23.296  1.00 49.49          GZ00 O
ATOM   5967  CB  LEU X 139     -35.323 116.286 -20.469  1.00 40.97          GZ00 C
ATOM   5968  CG  LEU X 139     -35.163 116.830 -19.046  1.00 45.88          GZ00 C
ATOM   5969  CD1 LEU X 139     -34.991 118.304 -19.121  1.00 55.62          GZ00 C
ATOM   5970  CD2 LEU X 139     -36.336 116.513 -18.183  1.00 56.00          GZ00 C
ATOM   5971  N   ILE X 140     -34.185 113.843 -22.489  1.00 43.98          GZ00 N
ATOM   5972  CA  ILE X 140     -34.065 113.151 -23.763  1.00 39.00          GZ00 C
ATOM   5973  C   ILE X 140     -35.287 112.261 -23.897  1.00 41.08          GZ00 C
ATOM   5974  O   ILE X 140     -35.562 111.453 -23.003  1.00 45.21          GZ00 O
ATOM   5975  CB  ILE X 140     -32.776 112.314 -23.820  1.00 38.23          GZ00 C
ATOM   5976  CG1 ILE X 140     -31.593 113.141 -23.326  1.00 38.68          GZ00 C
ATOM   5977  CG2 ILE X 140     -32.517 111.813 -25.212  1.00 37.81          GZ00 C
ATOM   5978  CD1 ILE X 140     -30.465 112.309 -22.831  1.00 36.60          GZ00 C
ATOM   5979  N   SER X 141     -36.037 112.410 -24.986  1.00 38.32          GZ00 N
ATOM   5980  CA  SER X 141     -37.296 111.688 -25.062  1.00 41.71          GZ00 C
ATOM   5981  C   SER X 141     -37.616 111.305 -26.496  1.00 42.47          GZ00 C
ATOM   5982  O   SER X 141     -37.050 111.841 -27.451  1.00 41.57          GZ00 O
ATOM   5983  CB  SER X 141     -38.442 112.523 -24.495  1.00 41.65          GZ00 C
ATOM   5984  OG  SER X 141     -38.508 113.735 -25.214  1.00 44.00          GZ00 O
ATOM   5985  N   ASP X 142     -38.557 110.367 -26.620  1.00 46.32          GZ00 N
ATOM   5986  CA  ASP X 142     -39.150 109.987 -27.901  1.00 48.85          GZ00 C
ATOM   5987  C   ASP X 142     -38.129 109.382 -28.860  1.00 51.47          GZ00 C
ATOM   5988  O   ASP X 142     -38.175 109.623 -30.072  1.00 44.48          GZ00 O
ATOM   5989  CB  ASP X 142     -39.846 111.179 -28.546  1.00 47.17          GZ00 C
ATOM   5990  CG  ASP X 142     -41.165 111.502 -27.888  1.00 60.06          GZ00 C
ATOM   5991  OD1 ASP X 142     -41.856 110.559 -27.429  1.00 53.08          GZ00 O
ATOM   5992  OD2 ASP X 142     -41.509 112.706 -27.833  1.00 72.08          GZ00 O1-
ATOM   5993  N   PHE X 143     -37.183 108.607 -28.329  1.00 42.34          GZ00 N
ATOM   5994  CA  PHE X 143     -36.209 107.975 -29.202  1.00 50.27          GZ00 C
ATOM   5995  C   PHE X 143     -36.431 106.471 -29.275  1.00 46.86          GZ00 C
ATOM   5996  O   PHE X 143     -36.969 105.852 -28.352  1.00 43.04          GZ00 O
ATOM   5997  CB  PHE X 143     -34.773 108.296 -28.793  1.00 38.71          GZ00 C
ATOM   5998  CG  PHE X 143     -34.426 107.944 -27.388  1.00 40.75          GZ00 C
ATOM   5999  CD1 PHE X 143     -33.898 106.698 -27.084  1.00 36.49          GZ00 C
ATOM   6000  CD2 PHE X 143     -34.537 108.889 -26.377  1.00 38.84          GZ00 C
ATOM   6001  CE1 PHE X 143     -33.510 106.389 -25.788  1.00 41.99          GZ00 C
ATOM   6002  CE2 PHE X 143     -34.161 108.588 -25.078  1.00 39.59          GZ00 C
ATOM   6003  CZ  PHE X 143     -33.645 107.330 -24.777  1.00 38.14          GZ00 C
ATOM   6004  N   TYR X 144     -36.071 105.909 -30.422  1.00 41.60          GZ00 N
ATOM   6005  CA  TYR X 144     -36.247 104.485 -30.675  1.00 40.98          GZ00 C
ATOM   6006  C   TYR X 144     -35.290 104.053 -31.769  1.00 46.46          GZ00 C
ATOM   6007  O   TYR X 144     -35.237 104.680 -32.843  1.00 42.13          GZ00 O
ATOM   6008  CB  TYR X 144     -37.685 104.146 -31.067  1.00 43.87          GZ00 C
ATOM   6009  CG  TYR X 144     -37.912 102.658 -31.265  1.00 41.93          GZ00 C
ATOM   6010  CD1 TYR X 144     -38.183 101.821 -30.184  1.00 44.51          GZ00 C
ATOM   6011  CD2 TYR X 144     -37.838 102.090 -32.527  1.00 35.71          GZ00 C
ATOM   6012  CE1 TYR X 144     -38.379 100.445 -30.358  1.00 40.63          GZ00 C
ATOM   6013  CE2 TYR X 144     -38.050 100.733 -32.717  1.00 40.34          GZ00 C
ATOM   6014  CZ  TYR X 144     -38.309  99.912 -31.631  1.00 41.12          GZ00 C
ATOM   6015  OH  TYR X 144     -38.511  98.567 -31.831  1.00 40.40          GZ00 O
ATOM   6016  N   PRO X 145     -34.530 102.974 -31.511  1.00 40.55          GZ00 N
ATOM   6017  CA  PRO X 145     -34.563 102.164 -30.284  1.00 43.72          GZ00 C
ATOM   6018  C   PRO X 145     -33.998 102.849 -29.028  1.00 43.96          GZ00 C
ATOM   6019  O   PRO X 145     -33.489 103.965 -29.115  1.00 40.33          GZ00 O
ATOM   6020  CB  PRO X 145     -33.699 100.937 -30.644  1.00 43.12          GZ00 C
ATOM   6021  CG  PRO X 145     -32.956 101.292 -31.849  1.00 42.92          GZ00 C
ATOM   6022  CD  PRO X 145     -33.714 102.356 -32.570  1.00 40.02          GZ00 C
ATOM   6023  N   GLY X 146     -34.102 102.165 -27.884  1.00 38.67          GZ00 N
ATOM   6024  CA  GLY X 146     -33.811 102.737 -26.584  1.00 36.90          GZ00 C
ATOM   6025  C   GLY X 146     -32.366 102.660 -26.134  1.00 38.27          GZ00 C
ATOM   6026  O   GLY X 146     -32.068 102.118 -25.070  1.00 42.45          GZ00 O
ATOM   6027  N   ALA X 147     -31.462 103.201 -26.930  1.00 33.88          GZ00 N
ATOM   6028  CA  ALA X 147     -30.063 103.278 -26.560  1.00 36.10          GZ00 C
ATOM   6029  C   ALA X 147     -29.541 104.597 -27.083  1.00 33.68          GZ00 C
ATOM   6030  O   ALA X 147     -29.858 105.007 -28.203  1.00 39.90          GZ00 O
```

```
ATOM   6031  CB  ALA X 147     -29.225 102.121 -27.118  1.00 29.64      GZ00 C
ATOM   6032  N   VAL X 148     -28.712 105.231 -26.263  1.00 33.29      GZ00 N
ATOM   6033  CA  VAL X 148     -28.309 106.608 -26.467  1.00 34.38      GZ00 C
ATOM   6034  C   VAL X 148     -27.005 106.806 -25.702  1.00 44.52      GZ00 C
ATOM   6035  O   VAL X 148     -26.787 106.191 -24.652  1.00 39.89      GZ00 O
ATOM   6036  CB  VAL X 148     -29.464 107.518 -25.970  1.00 40.00      GZ00 C
ATOM   6037  CG1 VAL X 148     -28.993 108.560 -25.020  1.00 41.53      GZ00 C
ATOM   6038  CG2 VAL X 148     -30.271 108.073 -27.138  1.00 35.87      GZ00 C
ATOM   6039  N   THR X 149     -26.133 107.663 -26.213  1.00 36.77      GZ00 N
ATOM   6040  CA  THR X 149     -24.987 108.051 -25.409  1.00 38.57      GZ00 C
ATOM   6041  C   THR X 149     -24.960 109.563 -25.294  1.00 44.30      GZ00 C
ATOM   6042  O   THR X 149     -25.353 110.287 -26.217  1.00 42.08      GZ00 O
ATOM   6043  CB  THR X 149     -23.650 107.565 -25.975  1.00 48.56      GZ00 C
ATOM   6044  OG1 THR X 149     -23.455 108.130 -27.276  1.00 56.60      GZ00 O
ATOM   6045  CG2 THR X 149     -23.624 106.035 -26.054  1.00 37.17      GZ00 C
ATOM   6046  N   VAL X 150     -24.503 110.036 -24.146  1.00 43.54      GZ00 N
ATOM   6047  CA  VAL X 150     -24.564 111.452 -23.836  1.00 47.15      GZ00 C
ATOM   6048  C   VAL X 150     -23.160 111.946 -23.536  1.00 46.29      GZ00 C
ATOM   6049  O   VAL X 150     -22.484 111.422 -22.642  1.00 44.10      GZ00 O
ATOM   6050  CB  VAL X 150     -25.519 111.726 -22.663  1.00 42.61      GZ00 C
ATOM   6051  CG1 VAL X 150     -25.584 113.212 -22.388  1.00 45.14      GZ00 C
ATOM   6052  CG2 VAL X 150     -26.919 111.152 -22.975  1.00 37.35      GZ00 C
ATOM   6053  N   ALA X 151     -22.741 112.977 -24.260  1.00 43.99      GZ00 N
ATOM   6054  CA  ALA X 151     -21.484 113.665 -24.002  1.00 55.42      GZ00 C
ATOM   6055  C   ALA X 151     -21.769 115.133 -23.706  1.00 56.11      GZ00 C
ATOM   6056  O   ALA X 151     -22.614 115.758 -24.361  1.00 51.73      GZ00 O
ATOM   6057  CB  ALA X 151     -20.528 113.545 -25.190  1.00 47.91      GZ00 C
ATOM   6058  N   TRP X 152     -21.091 115.665 -22.693  1.00 48.76      GZ00 N
ATOM   6059  CA  TRP X 152     -21.207 117.064 -22.312  1.00 52.98      GZ00 C
ATOM   6060  C   TRP X 152     -19.957 117.821 -22.742  1.00 62.60      GZ00 C
ATOM   6061  O   TRP X 152     -18.840 117.307 -22.638  1.00 63.13      GZ00 O
ATOM   6062  CB  TRP X 152     -21.402 117.202 -20.798  1.00 54.47      GZ00 C
ATOM   6063  CG  TRP X 152     -22.732 116.706 -20.310  1.00 55.93      GZ00 C
ATOM   6064  CD1 TRP X 152     -23.070 115.415 -20.021  1.00 50.45      GZ00 C
ATOM   6065  CD2 TRP X 152     -23.908 117.493 -20.059  1.00 54.33      GZ00 C
ATOM   6066  NE1 TRP X 152     -24.380 115.350 -19.603  1.00 45.37      GZ00 N
ATOM   6067  CE2 TRP X 152     -24.919 116.608 -19.624  1.00 49.73      GZ00 C
ATOM   6068  CE3 TRP X 152     -24.205 118.856 -20.162  1.00 49.64      GZ00 C
ATOM   6069  CZ2 TRP X 152     -26.201 117.046 -19.286  1.00 49.17      GZ00 C
ATOM   6070  CZ3 TRP X 152     -25.488 119.290 -19.834  1.00 47.92      GZ00 C
ATOM   6071  CH2 TRP X 152     -26.464 118.390 -19.400  1.00 49.61      GZ00 C
ATOM   6072  N   LYS X 153     -20.141 119.053 -23.204  1.00 65.10      GZ00 N
ATOM   6073  CA  LYS X 153     -19.019 119.883 -23.602  1.00 62.17      GZ00 C
ATOM   6074  C   LYS X 153     -19.122 121.266 -22.971  1.00 70.98      GZ00 C
ATOM   6075  O   LYS X 153     -20.213 121.849 -22.883  1.00 60.73      GZ00 O
ATOM   6076  CB  LYS X 153     -18.918 119.968 -25.133  1.00 62.97      GZ00 C
ATOM   6077  CG  LYS X 153     -18.343 118.679 -25.728  1.00 71.97      GZ00 C
ATOM   6078  CD  LYS X 153     -18.093 118.773 -27.212  1.00 76.62      GZ00 C
ATOM   6079  CE  LYS X 153     -17.488 117.483 -27.757  1.00 78.74      GZ00 C
ATOM   6080  NZ  LYS X 153     -17.470 117.510 -29.257  1.00 82.65      GZ00 N1+
ATOM   6081  N   ALA X 154     -17.966 121.767 -22.517  1.00 68.61      GZ00 N
ATOM   6082  CA  ALA X 154     -17.783 123.138 -22.053  1.00 70.61      GZ00 C
ATOM   6083  C   ALA X 154     -16.988 123.883 -23.122  1.00 76.84      GZ00 C
ATOM   6084  O   ALA X 154     -15.785 123.645 -23.282  1.00 70.93      GZ00 O
ATOM   6085  CB  ALA X 154     -17.057 123.158 -20.709  1.00 58.77      GZ00 C
ATOM   6086  N   ASP X 155     -17.672 124.765 -23.853  1.00 75.44      GZ00 N
ATOM   6087  CA  ASP X 155     -17.137 125.491 -25.013  1.00 86.60      GZ00 C
ATOM   6088  C   ASP X 155     -16.167 124.618 -25.821  1.00 88.28      GZ00 C
ATOM   6089  O   ASP X 155     -14.980 124.919 -25.980  1.00 92.56      GZ00 O
ATOM   6090  CB  ASP X 155     -16.529 126.864 -24.643  1.00 93.75      GZ00 C
ATOM   6091  CG  ASP X 155     -15.592 126.838 -23.429  1.00 92.88      GZ00 C
ATOM   6092  OD2 ASP X 155     -15.842 127.652 -22.504  1.00 84.61      GZ00 O1-
ATOM   6093  OD1 ASP X 155     -14.599 126.064 -23.414  1.00 88.83      GZ00 O
ATOM   6094  N   SER X 156     -16.707 123.493 -26.297  1.00 79.32      GZ00 N
ATOM   6095  CA  SER X 156     -16.051 122.520 -27.174  1.00 78.60      GZ00 C
ATOM   6096  C   SER X 156     -14.971 121.688 -26.498  1.00 74.64      GZ00 C
ATOM   6097  O   SER X 156     -14.249 120.963 -27.189  1.00 84.36      GZ00 O
ATOM   6098  CB  SER X 156     -15.436 123.186 -28.416  1.00 73.87      GZ00 C
ATOM   6099  OG  SER X 156     -16.405 123.895 -29.165  1.00 81.66      GZ00 O
ATOM   6100  N   SER X 157     -14.854 121.728 -25.183  1.00 69.01      GZ00 N
ATOM   6101  CA  SER X 157     -13.979 120.769 -24.541  1.00 69.77      GZ00 C
```

```
ATOM   6102  C    SER X 157      -14.813 119.693 -23.873  1.00 80.18          GZ00 C
ATOM   6103  O    SER X 157      -15.795 120.013 -23.189  1.00 78.55          GZ00 O
ATOM   6104  CB   SER X 157      -13.084 121.447 -23.505  1.00 71.70          GZ00 C
ATOM   6105  OG   SER X 157      -12.182 122.329 -24.136  1.00 83.37          GZ00 O
ATOM   6106  N    PRO X 158      -14.476 118.419 -24.062  1.00 82.78          GZ00 N
ATOM   6107  CA   PRO X 158      -15.228 117.349 -23.395  1.00 76.46          GZ00 C
ATOM   6108  C    PRO X 158      -15.217 117.510 -21.881  1.00 71.70          GZ00 C
ATOM   6109  O    PRO X 158      -14.185 117.812 -21.277  1.00 72.61          GZ00 O
ATOM   6110  CB   PRO X 158      -14.501 116.072 -23.839  1.00 73.01          GZ00 C
ATOM   6111  CG   PRO X 158      -13.220 116.534 -24.487  1.00 79.70          GZ00 C
ATOM   6112  CD   PRO X 158      -13.513 117.891 -25.037  1.00 80.30          GZ00 C
ATOM   6113  N    VAL X 159      -16.375 117.266 -21.266  1.00 66.35          GZ00 N
ATOM   6114  CA   VAL X 159      -16.548 117.386 -19.823  1.00 62.00          GZ00 C
ATOM   6115  C    VAL X 159      -16.401 116.006 -19.204  1.00 69.04          GZ00 C
ATOM   6116  O    VAL X 159      -17.189 115.099 -19.491  1.00 77.71          GZ00 O
ATOM   6117  CB   VAL X 159      -17.910 117.997 -19.467  1.00 64.97          GZ00 C
ATOM   6118  CG1  VAL X 159      -18.084 118.013 -17.959  1.00 62.71          GZ00 C
ATOM   6119  CG2  VAL X 159      -18.059 119.400 -20.075  1.00 59.52          GZ00 C
ATOM   6120  N    LYS X 160      -15.413 115.857 -18.328  1.00 74.85          GZ00 N
ATOM   6121  CA   LYS X 160      -14.967 114.550 -17.867  1.00 79.58          GZ00 C
ATOM   6122  C    LYS X 160      -15.580 114.124 -16.535  1.00 75.16          GZ00 C
ATOM   6123  O    LYS X 160      -15.412 112.964 -16.143  1.00 82.11          GZ00 O
ATOM   6124  CB   LYS X 160      -13.429 114.543 -17.758  1.00 85.50          GZ00 C
ATOM   6125  CG   LYS X 160      -12.723 113.330 -18.377  1.00 89.99          GZ00 C
ATOM   6126  CD   LYS X 160      -12.558 113.438 -19.900  1.00 91.72          GZ00 C
ATOM   6127  CE   LYS X 160      -11.807 112.217 -20.463  1.00101.89          GZ00 C
ATOM   6128  NZ   LYS X 160      -11.786 112.147 -21.960  1.00 92.33          GZ00 N1+
ATOM   6129  N    ALA X 161      -16.284 115.011 -15.833  1.00 64.13          GZ00 N
ATOM   6130  CA   ALA X 161      -16.778 114.677 -14.503  1.00 64.11          GZ00 C
ATOM   6131  C    ALA X 161      -18.053 115.454 -14.206  1.00 60.81          GZ00 C
ATOM   6132  O    ALA X 161      -18.346 116.472 -14.836  1.00 59.19          GZ00 O
ATOM   6133  CB   ALA X 161      -15.728 114.953 -13.424  1.00 61.76          GZ00 C
ATOM   6134  N    GLY X 162      -18.809 114.954 -13.231  1.00 53.42          GZ00 N
ATOM   6135  CA   GLY X 162      -20.048 115.575 -12.823  1.00 55.17          GZ00 C
ATOM   6136  C    GLY X 162      -21.273 115.133 -13.595  1.00 59.06          GZ00 C
ATOM   6137  O    GLY X 162      -22.353 115.706 -13.396  1.00 55.42          GZ00 O
ATOM   6138  N    VAL X 163      -21.147 114.128 -14.458  1.00 52.82          GZ00 N
ATOM   6139  CA   VAL X 163      -22.234 113.680 -15.316  1.00 48.37          GZ00 C
ATOM   6140  C    VAL X 163      -22.874 112.455 -14.689  1.00 49.71          GZ00 C
ATOM   6141  O    VAL X 163      -22.179 111.511 -14.296  1.00 50.43          GZ00 O
ATOM   6142  CB   VAL X 163      -21.738 113.364 -16.737  1.00 45.16          GZ00 C
ATOM   6143  CG1  VAL X 163      -22.863 112.761 -17.564  1.00 41.69          GZ00 C
ATOM   6144  CG2  VAL X 163      -21.220 114.622 -17.401  1.00 46.72          GZ00 C
ATOM   6145  N    GLU X 164      -24.197 112.468 -14.599  1.00 47.13          GZ00 N
ATOM   6146  CA   GLU X 164      -24.964 111.300 -14.201  1.00 53.09          GZ00 C
ATOM   6147  C    GLU X 164      -26.115 111.140 -15.178  1.00 46.61          GZ00 C
ATOM   6148  O    GLU X 164      -26.815 112.113 -15.471  1.00 51.75          GZ00 O
ATOM   6149  CB   GLU X 164      -25.447 111.448 -12.757  1.00 51.29          GZ00 C
ATOM   6150  CG   GLU X 164      -24.369 110.989 -11.771  1.00 66.35          GZ00 C
ATOM   6151  CD   GLU X 164      -24.588 111.481 -10.349  1.00 80.34          GZ00 C
ATOM   6152  OE1  GLU X 164      -25.618 112.153 -10.090  1.00 84.11          GZ00 O
ATOM   6153  OE2  GLU X 164      -23.723 111.189  -9.486  1.00 74.82          GZ00 O1-
ATOM   6154  N    THR X 165      -26.291 109.923 -15.699  1.00 44.17          GZ00 N
ATOM   6155  CA   THR X 165      -27.216 109.646 -16.791  1.00 41.22          GZ00 C
ATOM   6156  C    THR X 165      -28.051 108.419 -16.450  1.00 43.11          GZ00 C
ATOM   6157  O    THR X 165      -27.536 107.449 -15.891  1.00 46.87          GZ00 O
ATOM   6158  CB   THR X 165      -26.455 109.433 -18.098  1.00 41.49          GZ00 C
ATOM   6159  OG1  THR X 165      -25.742 110.625 -18.421  1.00 41.60          GZ00 O
ATOM   6160  CG2  THR X 165      -27.397 109.091 -19.242  1.00 40.37          GZ00 C
ATOM   6161  N    THR X 166      -29.352 108.492 -16.715  1.00 45.39          GZ00 N
ATOM   6162  CA   THR X 166      -30.228 107.356 -16.466  1.00 48.92          GZ00 C
ATOM   6163  C    THR X 166      -30.165 106.353 -17.611  1.00 43.82          GZ00 C
ATOM   6164  O    THR X 166      -29.861 106.689 -18.756  1.00 48.48          GZ00 O
ATOM   6165  CB   THR X 166      -31.684 107.786 -16.285  1.00 42.46          GZ00 C
ATOM   6166  OG1  THR X 166      -32.133 108.501 -17.446  1.00 42.85          GZ00 O
ATOM   6167  CG2  THR X 166      -31.833 108.639 -15.075  1.00 49.93          GZ00 C
ATOM   6168  N    VAL X 167      -30.481 105.109 -17.287  1.00 43.83          GZ00 N
ATOM   6169  CA   VAL X 167      -30.761 104.109 -18.306  1.00 47.99          GZ00 C
ATOM   6170  C    VAL X 167      -32.044 104.535 -19.004  1.00 43.14          GZ00 C
ATOM   6171  O    VAL X 167      -32.921 105.146 -18.381  1.00 51.16          GZ00 O
ATOM   6172  CB   VAL X 167      -30.905 102.716 -17.689  1.00 47.80          GZ00 C
```

```
ATOM   6173  CG1 VAL X 167      -29.736 102.444 -16.786   1.00 37.96         GZ00 C
ATOM   6174  CG2 VAL X 167      -32.215 102.646 -16.913   1.00 51.34         GZ00 C
ATOM   6175  N   PRO X 168      -32.201 104.266 -20.280   1.00 44.56         GZ00 N
ATOM   6176  CA  PRO X 168      -33.464 104.616 -20.934   1.00 41.00         GZ00 C
ATOM   6177  C   PRO X 168      -34.607 103.782 -20.385   1.00 47.02         GZ00 C
ATOM   6178  O   PRO X 168      -34.429 102.648 -19.941   1.00 49.69         GZ00 O
ATOM   6179  CB  PRO X 168      -33.202 104.334 -22.419   1.00 43.99         GZ00 C
ATOM   6180  CG  PRO X 168      -31.908 103.574 -22.452   1.00 46.78         GZ00 C
ATOM   6181  CD  PRO X 168      -31.141 103.904 -21.227   1.00 39.42         GZ00 C
ATOM   6182  N   SER X 169      -35.784 104.384 -20.368   1.00 44.67         GZ00 N
ATOM   6183  CA  SER X 169      -36.987 103.723 -19.907   1.00 50.31         GZ00 C
ATOM   6184  C   SER X 169      -38.095 103.966 -20.920   1.00 49.36         GZ00 C
ATOM   6185  O   SER X 169      -38.168 105.022 -21.563   1.00 43.92         GZ00 O
ATOM   6186  CB  SER X 169      -37.417 104.225 -18.517   1.00 43.96         GZ00 C
ATOM   6187  OG  SER X 169      -37.708 105.604 -18.594   1.00 55.02         GZ00 O
ATOM   6188  N   LYS X 170      -38.955 102.967 -21.057   1.00 52.10         GZ00 N
ATOM   6189  CA  LYS X 170      -39.974 102.986 -22.090   1.00 53.24         GZ00 C
ATOM   6190  C   LYS X 170      -41.088 103.939 -21.683   1.00 52.61         GZ00 C
ATOM   6191  O   LYS X 170      -41.529 103.938 -20.531   1.00 55.96         GZ00 O
ATOM   6192  CB  LYS X 170      -40.491 101.563 -22.339   1.00 57.92         GZ00 C
ATOM   6193  CG  LYS X 170      -41.220 101.352 -23.678   1.00 61.39         GZ00 C
ATOM   6194  CD  LYS X 170      -41.730  99.902 -23.841   1.00 60.71         GZ00 C
ATOM   6195  CE  LYS X 170      -40.545  98.928 -24.020   1.00 68.36         GZ00 C
ATOM   6196  NZ  LYS X 170      -40.911  97.510 -24.330   1.00 76.29         GZ00 N1+
ATOM   6197  N   GLN X 171      -41.485 104.794 -22.619   1.00 49.96         GZ00 N
ATOM   6198  CA  GLN X 171      -42.633 105.668 -22.499   1.00 48.46         GZ00 C
ATOM   6199  C   GLN X 171      -43.897 104.888 -22.851   1.00 55.64         GZ00 C
ATOM   6200  O   GLN X 171      -43.843 103.749 -23.324   1.00 58.51         GZ00 O
ATOM   6201  CB  GLN X 171      -42.474 106.880 -23.422   1.00 47.95         GZ00 C
ATOM   6202  CG  GLN X 171      -41.214 107.699 -23.166   1.00 44.29         GZ00 C
ATOM   6203  CD  GLN X 171      -40.919 108.737 -24.253   1.00 49.84         GZ00 C
ATOM   6204  OE1 GLN X 171      -40.035 109.590 -24.090   1.00 47.77         GZ00 O
ATOM   6205  NE2 GLN X 171      -41.651 108.667 -25.365   1.00 49.40         GZ00 N
ATOM   6206  N   SER X 172      -45.052 105.506 -22.605   1.00 61.57         GZ00 N
ATOM   6207  CA  SER X 172      -46.313 104.871 -22.927   1.00 67.22         GZ00 C
ATOM   6208  C   SER X 172      -46.410 104.535 -24.419   1.00 65.89         GZ00 C
ATOM   6209  O   SER X 172      -46.922 103.477 -24.808   1.00 65.81         GZ00 O
ATOM   6210  CB  SER X 172      -47.491 105.696 -22.460   1.00 55.65         GZ00 C
ATOM   6211  OG  SER X 172      -47.391 106.989 -23.016   1.00 65.82         GZ00 O
ATOM   6212  N   ASN X 173      -45.891 105.434 -25.268   1.00 64.43         GZ00 N
ATOM   6213  CA  ASN X 173      -45.898 105.274 -26.722   1.00 55.79         GZ00 C
ATOM   6214  C   ASN X 173      -44.815 104.319 -27.236   1.00 60.52         GZ00 C
ATOM   6215  O   ASN X 173      -44.548 104.300 -28.446   1.00 58.94         GZ00 O
ATOM   6216  CB  ASN X 173      -45.762 106.634 -27.419   1.00 56.26         GZ00 C
ATOM   6217  CG  ASN X 173      -44.404 107.291 -27.198   1.00 62.34         GZ00 C
ATOM   6218  OD1 ASN X 173      -43.536 106.762 -26.496   1.00 55.11         GZ00 O
ATOM   6219  ND2 ASN X 173      -44.207 108.450 -27.834   1.00 61.15         GZ00 N
ATOM   6220  N   ASN X 174      -44.162 103.573 -26.348   1.00 57.78         GZ00 N
ATOM   6221  CA  ASN X 174      -43.139 102.573 -26.649   1.00 62.92         GZ00 C
ATOM   6222  C   ASN X 174      -41.849 103.159 -27.207   1.00 62.49         GZ00 C
ATOM   6223  O   ASN X 174      -40.923 102.390 -27.512   1.00 56.92         GZ00 O
ATOM   6224  CB  ASN X 174      -43.647 101.485 -27.598   1.00 64.96         GZ00 C
ATOM   6225  CG  ASN X 174      -44.491 100.463 -26.877   1.00 72.73         GZ00 C
ATOM   6226  OD1 ASN X 174      -44.329 100.265 -25.673   1.00 66.54         GZ00 O
ATOM   6227  ND2 ASN X 174      -45.394  99.809 -27.599   1.00 78.95         GZ00 N
ATOM   6228  N   LYS X 175      -41.738 104.481 -27.328   1.00 58.39         GZ00 N
ATOM   6229  CA  LYS X 175      -40.429 105.092 -27.468   1.00 52.42         GZ00 C
ATOM   6230  C   LYS X 175      -39.796 105.219 -26.086   1.00 49.60         GZ00 C
ATOM   6231  O   LYS X 175      -40.366 104.789 -25.077   1.00 48.97         GZ00 O
ATOM   6232  CB  LYS X 175      -40.537 106.429 -28.183   1.00 49.13         GZ00 C
ATOM   6233  CG  LYS X 175      -41.199 106.317 -29.545   1.00 48.64         GZ00 C
ATOM   6234  CD  LYS X 175      -41.226 107.678 -30.217   1.00 55.03         GZ00 C
ATOM   6235  CE  LYS X 175      -42.052 107.683 -31.482   1.00 50.14         GZ00 C
ATOM   6236  NZ  LYS X 175      -41.863 108.996 -32.147   1.00 65.58         GZ00 N
ATOM   6237  N   TYR X 176      -38.595 105.789 -26.028   1.00 45.23         GZ00 N
ATOM   6238  CA  TYR X 176      -37.810 105.757 -24.803   1.00 46.64         GZ00 C
ATOM   6239  C   TYR X 176      -37.405 107.159 -24.375   1.00 46.24         GZ00 C
ATOM   6240  O   TYR X 176      -37.332 108.091 -25.185   1.00 42.79         GZ00 O
ATOM   6241  CB  TYR X 176      -36.559 104.893 -24.956   1.00 40.24         GZ00 C
ATOM   6242  CG  TYR X 176      -36.865 103.419 -25.082   1.00 46.22         GZ00 C
ATOM   6243  CD1 TYR X 176      -37.356 102.882 -26.276   1.00 40.78         GZ00 C
```

```
ATOM   6244  CD2 TYR X 176     -36.683 102.567 -24.006  1.00 47.03      GZ00 C
ATOM   6245  CE1 TYR X 176     -37.628 101.537 -26.389  1.00 43.89      GZ00 C
ATOM   6246  CE2 TYR X 176     -36.965 101.215 -24.107  1.00 51.38      GZ00 C
ATOM   6247  CZ  TYR X 176     -37.439 100.706 -25.294  1.00 52.19      GZ00 C
ATOM   6248  OH  TYR X 176     -37.713  99.359 -25.380  1.00 57.66      GZ00 O
ATOM   6249  N   ALA X 177     -37.152 107.290 -23.072  1.00 42.19      GZ00 N
ATOM   6250  CA  ALA X 177     -36.737 108.543 -22.464  1.00 42.58      GZ00 C
ATOM   6251  C   ALA X 177     -35.553 108.309 -21.543  1.00 39.64      GZ00 C
ATOM   6252  O   ALA X 177     -35.393 107.231 -20.966  1.00 43.45      GZ00 O
ATOM   6253  CB  ALA X 177     -37.873 109.197 -21.673  1.00 36.73      GZ00 C
ATOM   6254  N   ALA X 178     -34.735 109.339 -21.397  1.00 35.05      GZ00 N
ATOM   6255  CA  ALA X 178     -33.635 109.306 -20.451  1.00 41.99      GZ00 C
ATOM   6256  C   ALA X 178     -33.310 110.743 -20.087  1.00 37.98      GZ00 C
ATOM   6257  O   ALA X 178     -33.750 111.681 -20.751  1.00 42.02      GZ00 O
ATOM   6258  CB  ALA X 178     -32.406 108.580 -21.021  1.00 32.61      GZ00 C
ATOM   6259  N   SER X 179     -32.574 110.910 -18.999  1.00 43.75      GZ00 N
ATOM   6260  CA  SER X 179     -32.093 112.230 -18.639  1.00 42.28      GZ00 C
ATOM   6261  C   SER X 179     -30.654 112.109 -18.170  1.00 40.65      GZ00 C
ATOM   6262  O   SER X 179     -30.220 111.051 -17.713  1.00 48.12      GZ00 O
ATOM   6263  CB  SER X 179     -32.956 112.897 -17.572  1.00 35.06      GZ00 C
ATOM   6264  OG  SER X 179     -33.250 112.013 -16.525  1.00 37.63      GZ00 O
ATOM   6265  N   SER X 180     -29.921 113.202 -18.326  1.00 41.05      GZ00 N
ATOM   6266  CA  SER X 180     -28.523 113.321 -17.950  1.00 41.22      GZ00 C
ATOM   6267  C   SER X 180     -28.336 114.625 -17.185  1.00 47.40      GZ00 C
ATOM   6268  O   SER X 180     -28.846 115.667 -17.606  1.00 38.98      GZ00 O
ATOM   6269  CB  SER X 180     -27.628 113.305 -19.187  1.00 42.77      GZ00 C
ATOM   6270  OG  SER X 180     -26.270 113.291 -18.824  1.00 40.90      GZ00 O
ATOM   6271  N   TYR X 181     -27.616 114.556 -16.062  1.00 49.96      GZ00 N
ATOM   6272  CA  TYR X 181     -27.366 115.685 -15.179  1.00 43.04      GZ00 C
ATOM   6273  C   TYR X 181     -25.872 115.961 -15.084  1.00 48.60      GZ00 C
ATOM   6274  O   TYR X 181     -25.089 115.054 -14.778  1.00 50.29      GZ00 O
ATOM   6275  CB  TYR X 181     -27.922 115.419 -13.778  1.00 32.89      GZ00 C
ATOM   6276  CG  TYR X 181     -29.417 115.274 -13.722  1.00 40.29      GZ00 C
ATOM   6277  CD2 TYR X 181     -30.224 116.358 -13.413  1.00 36.67      GZ00 C
ATOM   6278  CD1 TYR X 181     -30.030 114.053 -13.999  1.00 41.31      GZ00 C
ATOM   6279  CE2 TYR X 181     -31.614 116.238 -13.366  1.00 40.95      GZ00 C
ATOM   6280  CE1 TYR X 181     -31.402 113.909 -13.951  1.00 38.36      GZ00 C
ATOM   6281  CZ  TYR X 181     -32.195 115.000 -13.636  1.00 46.60      GZ00 C
ATOM   6282  OH  TYR X 181     -33.561 114.845 -13.591  1.00 40.50      GZ00 O
ATOM   6283  N   LEU X 182     -25.488 117.220 -15.298  1.00 46.12      GZ00 N
ATOM   6284  CA  LEU X 182     -24.119 117.692 -15.092  1.00 47.69      GZ00 C
ATOM   6285  C   LEU X 182     -24.094 118.629 -13.889  1.00 50.67      GZ00 C
ATOM   6286  O   LEU X 182     -24.748 119.679 -13.906  1.00 45.67      GZ00 O
ATOM   6287  CB  LEU X 182     -23.582 118.410 -16.332  1.00 50.05      GZ00 C
ATOM   6288  CG  LEU X 182     -22.207 119.070 -16.145  1.00 51.24      GZ00 C
ATOM   6289  CD1 LEU X 182     -21.143 118.063 -15.726  1.00 46.82      GZ00 C
ATOM   6290  CD2 LEU X 182     -21.779 119.815 -17.399  1.00 48.62      GZ00 C
ATOM   6291  N   SER X 183     -23.382 118.231 -12.839  1.00 44.73      GZ00 N
ATOM   6292  CA  SER X 183     -23.218 119.066 -11.658  1.00 53.81      GZ00 C
ATOM   6293  C   SER X 183     -21.967 119.928 -11.797  1.00 58.46      GZ00 C
ATOM   6294  O   SER X 183     -20.896 119.430 -12.152  1.00 65.34      GZ00 O
ATOM   6295  CB  SER X 183     -23.124 118.218 -10.393  1.00 46.16      GZ00 C
ATOM   6296  OG  SER X 183     -24.237 117.370 -10.277  1.00 53.51      GZ00 O
ATOM   6297  N   LEU X 184     -22.104 121.208 -11.480  1.00 52.70      GZ00 N
ATOM   6298  CA  LEU X 184     -21.025 122.174 -11.566  1.00 59.11      GZ00 C
ATOM   6299  C   LEU X 184     -21.049 123.030 -10.308  1.00 62.24      GZ00 C
ATOM   6300  O   LEU X 184     -22.011 123.007  -9.537  1.00 61.48      GZ00 O
ATOM   6301  CB  LEU X 184     -21.178 123.079 -12.796  1.00 57.83      GZ00 C
ATOM   6302  CG  LEU X 184     -21.196 122.468 -14.189  1.00 57.44      GZ00 C
ATOM   6303  CD1 LEU X 184     -21.426 123.563 -15.208  1.00 52.24      GZ00 C
ATOM   6304  CD2 LEU X 184     -19.901 121.740 -14.475  1.00 62.82      GZ00 C
ATOM   6305  N   THR X 185     -19.978 123.805 -10.106  1.00 64.47      GZ00 N
ATOM   6306  CA  THR X 185     -20.070 124.923  -9.176  1.00 60.49      GZ00 C
ATOM   6307  C   THR X 185     -20.580 126.161  -9.899  1.00 61.60      GZ00 C
ATOM   6308  O   THR X 185     -20.455 126.279 -11.129  1.00 56.95      GZ00 O
ATOM   6309  CB  THR X 185     -18.710 125.225  -8.555  1.00 61.74      GZ00 C
ATOM   6310  OG1 THR X 185     -17.813 125.690  -9.577  1.00 64.33      GZ00 O
ATOM   6311  CG2 THR X 185     -18.149 124.005  -7.840  1.00 49.10      GZ00 C
ATOM   6312  N   PRO X 186     -21.194 127.094  -9.165  1.00 63.67      GZ00 N
ATOM   6313  CA  PRO X 186     -21.536 128.392  -9.774  1.00 66.91      GZ00 C
ATOM   6314  C   PRO X 186     -20.364 129.034 -10.512  1.00 66.84      GZ00 C
```

```
ATOM   6315  O    PRO X 186    -20.570 129.710 -11.529  1.00 66.08      GZ00 O
ATOM   6316  CB   PRO X 186    -21.974 129.227  -8.564  1.00 55.91      GZ00 C
ATOM   6317  CG   PRO X 186    -22.496 128.215  -7.584  1.00 54.51      GZ00 C
ATOM   6318  CD   PRO X 186    -21.625 127.004  -7.757  1.00 56.56      GZ00 C
ATOM   6319  N    GLU X 187    -19.133 128.818 -10.030  1.00 69.10      GZ00 N
ATOM   6320  CA   GLU X 187    -17.938 129.365 -10.676  1.00 76.90      GZ00 C
ATOM   6321  C    GLU X 187    -17.718 128.740 -12.049  1.00 75.85      GZ00 C
ATOM   6322  O    GLU X 187    -17.599 129.450 -13.059  1.00 69.16      GZ00 O
ATOM   6323  CB   GLU X 187    -16.708 129.124  -9.791  1.00 78.07      GZ00 C
ATOM   6324  CG   GLU X 187    -17.004 128.966  -8.297  1.00 81.84      GZ00 C
ATOM   6325  CD   GLU X 187    -17.761 130.158  -7.714  1.00100.11      GZ00 C
ATOM   6326  OE1  GLU X 187    -17.435 131.317  -8.086  1.00 95.34      GZ00 O
ATOM   6327  OE2  GLU X 187    -18.692 129.930  -6.901  1.00102.20      GZ00 O1-
ATOM   6328  N    GLN X 188    -17.652 127.402 -12.095  1.00 68.87      GZ00 N
ATOM   6329  CA   GLN X 188    -17.499 126.699 -13.361  1.00 62.65      GZ00 C
ATOM   6330  C    GLN X 188    -18.597 127.093 -14.333  1.00 65.88      GZ00 C
ATOM   6331  O    GLN X 188    -18.350 127.252 -15.533  1.00 66.09      GZ00 O
ATOM   6332  CB   GLN X 188    -17.540 125.195 -13.127  1.00 65.71      GZ00 C
ATOM   6333  CG   GLN X 188    -16.451 124.635 -12.256  1.00 60.72      GZ00 C
ATOM   6334  CD   GLN X 188    -16.821 123.252 -11.764  1.00 66.14      GZ00 C
ATOM   6335  OE1  GLN X 188    -17.996 122.941 -11.617  1.00 70.69      GZ00 O
ATOM   6336  NE2  GLN X 188    -15.827 122.419 -11.504  1.00 73.28      GZ00 N
ATOM   6337  N    TRP X 189    -19.821 127.251 -13.828  1.00 60.70      GZ00 N
ATOM   6338  CA   TRP X 189    -20.941 127.588 -14.695  1.00 61.89      GZ00 C
ATOM   6339  C    TRP X 189    -20.728 128.932 -15.381  1.00 66.39      GZ00 C
ATOM   6340  O    TRP X 189    -20.889 129.048 -16.601  1.00 69.50      GZ00 O
ATOM   6341  CB   TRP X 189    -22.233 127.581 -13.880  1.00 53.09      GZ00 C
ATOM   6342  CG   TRP X 189    -23.388 128.262 -14.520  1.00 48.26      GZ00 C
ATOM   6343  CD1  TRP X 189    -24.099 129.288 -13.998  1.00 54.93      GZ00 C
ATOM   6344  CD2  TRP X 189    -23.972 127.976 -15.799  1.00 53.12      GZ00 C
ATOM   6345  NE1  TRP X 189    -25.102 129.665 -14.859  1.00 60.99      GZ00 N
ATOM   6346  CE2  TRP X 189    -25.047 128.878 -15.976  1.00 52.94      GZ00 C
ATOM   6347  CE3  TRP X 189    -23.704 127.040 -16.803  1.00 54.03      GZ00 C
ATOM   6348  CZ2  TRP X 189    -25.861 128.873 -17.116  1.00 55.81      GZ00 C
ATOM   6349  CZ3  TRP X 189    -24.512 127.036 -17.944  1.00 57.95      GZ00 C
ATOM   6350  CH2  TRP X 189    -25.577 127.953 -18.090  1.00 55.87      GZ00 C
ATOM   6351  N    LYS X 190    -20.337 129.957 -14.619  1.00 75.20      GZ00 N
ATOM   6352  CA   LYS X 190    -20.232 131.302 -15.184  1.00 80.83      GZ00 C
ATOM   6353  C    LYS X 190    -18.940 131.535 -15.962  1.00 78.13      GZ00 C
ATOM   6354  O    LYS X 190    -18.891 132.458 -16.786  1.00 75.06      GZ00 O
ATOM   6355  CB   LYS X 190    -20.398 132.357 -14.081  1.00 73.65      GZ00 C
ATOM   6356  CG   LYS X 190    -21.825 132.394 -13.491  1.00 81.54      GZ00 C
ATOM   6357  CD   LYS X 190    -21.973 133.326 -12.286  1.00 80.94      GZ00 C
ATOM   6358  CE   LYS X 190    -23.413 133.330 -11.769  1.00 79.11      GZ00 C
ATOM   6359  NZ   LYS X 190    -23.602 134.127 -10.516  1.00 82.23      GZ00 N1+
ATOM   6360  N    SER X 191    -17.917 130.698 -15.765  1.00 70.70      GZ00 N
ATOM   6361  CA   SER X 191    -16.625 130.963 -16.388  1.00 77.12      GZ00 C
ATOM   6362  C    SER X 191    -16.645 130.672 -17.891  1.00 80.54      GZ00 C
ATOM   6363  O    SER X 191    -16.183 131.494 -18.691  1.00 90.60      GZ00 O
ATOM   6364  CB   SER X 191    -15.528 130.163 -15.676  1.00 70.51      GZ00 C
ATOM   6365  OG   SER X 191    -15.673 128.776 -15.878  1.00 73.70      GZ00 O
ATOM   6366  N    HIS X 192    -17.193 129.529 -18.303  1.00 79.21      GZ00 N
ATOM   6367  CA   HIS X 192    -17.144 129.148 -19.709  1.00 68.99      GZ00 C
ATOM   6368  C    HIS X 192    -18.216 129.880 -20.503  1.00 68.52      GZ00 C
ATOM   6369  O    HIS X 192    -19.228 130.326 -19.959  1.00 75.08      GZ00 O
ATOM   6370  CB   HIS X 192    -17.328 127.646 -19.858  1.00 67.33      GZ00 C
ATOM   6371  CG   HIS X 192    -16.211 126.849 -19.270  1.00 72.96      GZ00 C
ATOM   6372  ND1  HIS X 192    -16.133 126.565 -17.924  1.00 69.38      GZ00 N
ATOM   6373  CD2  HIS X 192    -15.134 126.260 -19.844  1.00 69.24      GZ00 C
ATOM   6374  CE1  HIS X 192    -15.051 125.843 -17.690  1.00 77.85      GZ00 C
ATOM   6375  NE2  HIS X 192    -14.427 125.644 -18.839  1.00 78.93      GZ00 N
ATOM   6376  N    ARG X 193    -17.980 130.015 -21.811  1.00 67.36      GZ00 N
ATOM   6377  CA   ARG X 193    -18.945 130.743 -22.627  1.00 70.06      GZ00 C
ATOM   6378  C    ARG X 193    -20.261 129.998 -22.711  1.00 73.81      GZ00 C
ATOM   6379  O    ARG X 193    -21.329 130.620 -22.671  1.00 71.37      GZ00 O
ATOM   6380  CB   ARG X 193    -18.411 131.013 -24.033  1.00 83.17      GZ00 C
ATOM   6381  CG   ARG X 193    -17.125 131.815 -24.098  1.00 91.89      GZ00 C
ATOM   6382  CD   ARG X 193    -16.644 131.932 -25.540  1.00 98.59      GZ00 C
ATOM   6383  NE   ARG X 193    -15.566 132.909 -25.682  1.00110.88      GZ00 N
ATOM   6384  CZ   ARG X 193    -14.801 133.031 -26.763  1.00111.56      GZ00 C
ATOM   6385  NH1  ARG X 193    -13.838 133.947 -26.798  1.00100.02      GZ00 N1+
```

```
ATOM   6386  NH2 ARG X 193     -15.019 132.260 -27.824  1.00112.85        GZ00 N
ATOM   6387  N   SER X 194     -20.207 128.670 -22.831  1.00 80.31        GZ00 N
ATOM   6388  CA  SER X 194     -21.414 127.865 -22.991  1.00 74.11        GZ00 C
ATOM   6389  C   SER X 194     -21.137 126.416 -22.618  1.00 71.90        GZ00 C
ATOM   6390  O   SER X 194     -19.991 125.955 -22.604  1.00 68.02        GZ00 O
ATOM   6391  CB  SER X 194     -21.945 127.911 -24.425  1.00 69.53        GZ00 C
ATOM   6392  OG  SER X 194     -21.159 127.065 -25.246  1.00 67.74        GZ00 O
ATOM   6393  N   TYR X 195     -22.224 125.690 -22.382  1.00 67.26        GZ00 N
ATOM   6394  CA  TYR X 195     -22.180 124.258 -22.168  1.00 63.57        GZ00 C
ATOM   6395  C   TYR X 195     -23.120 123.573 -23.142  1.00 64.49        GZ00 C
ATOM   6396  O   TYR X 195     -24.117 124.154 -23.584  1.00 56.47        GZ00 O
ATOM   6397  CB  TYR X 195     -22.572 123.889 -20.766  1.00 57.00        GZ00 C
ATOM   6398  CG  TYR X 195     -21.519 124.195 -19.752  1.00 60.50        GZ00 C
ATOM   6399  CD1 TYR X 195     -21.344 125.486 -19.268  1.00 63.71        GZ00 C
ATOM   6400  CD2 TYR X 195     -20.712 123.184 -19.254  1.00 60.86        GZ00 C
ATOM   6401  CE1 TYR X 195     -20.383 125.757 -18.319  1.00 66.00        GZ00 C
ATOM   6402  CE2 TYR X 195     -19.756 123.440 -18.310  1.00 67.49        GZ00 C
ATOM   6403  CZ  TYR X 195     -19.588 124.727 -17.850  1.00 65.78        GZ00 C
ATOM   6404  OH  TYR X 195     -18.626 124.963 -16.906  1.00 71.05        GZ00 O
ATOM   6405  N   SER X 196     -22.786 122.326 -23.470  1.00 62.79        GZ00 N
ATOM   6406  CA  SER X 196     -23.538 121.568 -24.453  1.00 61.48        GZ00 C
ATOM   6407  C   SER X 196     -23.826 120.160 -23.952  1.00 61.15        GZ00 C
ATOM   6408  O   SER X 196     -23.001 119.525 -23.279  1.00 54.07        GZ00 O
ATOM   6409  CB  SER X 196     -22.791 121.504 -25.783  1.00 63.75        GZ00 C
ATOM   6410  OG  SER X 196     -22.721 122.789 -26.372  1.00 69.20        GZ00 O
ATOM   6411  N   CYS X 197     -25.014 119.690 -24.294  1.00 52.83        GZ00 N
ATOM   6412  CA  CYS X 197     -25.428 118.315 -24.085  1.00 51.91        GZ00 C
ATOM   6413  C   CYS X 197     -25.524 117.683 -25.463  1.00 55.35        GZ00 C
ATOM   6414  O   CYS X 197     -26.303 118.149 -26.305  1.00 55.75        GZ00 O
ATOM   6415  CB  CYS X 197     -26.772 118.256 -23.354  1.00 51.31        GZ00 C
ATOM   6416  SG  CYS X 197     -27.349 116.579 -23.083  1.00 51.29        GZ00 S
ATOM   6417  N   GLN X 198     -24.716 116.655 -25.704  1.00 49.43        GZ00 N
ATOM   6418  CA  GLN X 198     -24.668 115.979 -26.996  1.00 53.99        GZ00 C
ATOM   6419  C   GLN X 198     -25.193 114.555 -26.839  1.00 53.90        GZ00 C
ATOM   6420  O   GLN X 198     -24.579 113.736 -26.139  1.00 49.13        GZ00 O
ATOM   6421  CB  GLN X 198     -23.245 115.967 -27.554  1.00 51.29        GZ00 C
ATOM   6422  CG  GLN X 198     -22.782 117.315 -28.078  1.00 67.46        GZ00 C
ATOM   6423  CD  GLN X 198     -21.293 117.357 -28.388  1.00 69.14        GZ00 C
ATOM   6424  OE1 GLN X 198     -20.565 116.404 -28.128  1.00 72.78        GZ00 O
ATOM   6425  NE2 GLN X 198     -20.838 118.470 -28.943  1.00 73.70        GZ00 N
ATOM   6426  N   VAL X 199     -26.299 114.256 -27.519  1.00 45.97        GZ00 N
ATOM   6427  CA  VAL X 199     -26.970 112.961 -27.422  1.00 47.62        GZ00 C
ATOM   6428  C   VAL X 199     -26.780 112.222 -28.743  1.00 49.39        GZ00 C
ATOM   6429  O   VAL X 199     -27.294 112.648 -29.786  1.00 43.67        GZ00 O
ATOM   6430  CB  VAL X 199     -28.465 113.117 -27.091  1.00 38.03        GZ00 C
ATOM   6431  CG1 VAL X 199     -29.116 111.753 -26.854  1.00 36.64        GZ00 C
ATOM   6432  CG2 VAL X 199     -28.642 114.009 -25.881  1.00 43.16        GZ00 C
ATOM   6433  N   THR X 200     -26.069 111.100 -28.699  1.00 44.37        GZ00 N
ATOM   6434  CA  THR X 200     -25.870 110.288 -29.888  1.00 47.13        GZ00 C
ATOM   6435  C   THR X 200     -26.837 109.107 -29.884  1.00 46.93        GZ00 C
ATOM   6436  O   THR X 200     -26.959 108.389 -28.886  1.00 37.31        GZ00 O
ATOM   6437  CB  THR X 200     -24.426 109.819 -29.990  1.00 47.07        GZ00 C
ATOM   6438  OG1 THR X 200     -23.583 110.975 -30.046  1.00 49.61        GZ00 O
ATOM   6439  CG2 THR X 200     -24.222 108.955 -31.246  1.00 48.16        GZ00 C
ATOM   6440  N   HIS X 201     -27.533 108.936 -31.002  1.00 40.95        GZ00 N
ATOM   6441  CA  HIS X 201     -28.533 107.899 -31.163  1.00 45.21        GZ00 C
ATOM   6442  C   HIS X 201     -28.414 107.309 -32.559  1.00 45.62        GZ00 C
ATOM   6443  O   HIS X 201     -28.609 108.023 -33.549  1.00 41.52        GZ00 O
ATOM   6444  CB  HIS X 201     -29.928 108.468 -30.953  1.00 37.81        GZ00 C
ATOM   6445  CG  HIS X 201     -31.009 107.472 -31.174  1.00 40.25        GZ00 C
ATOM   6446  ND1 HIS X 201     -31.717 107.395 -32.354  1.00 39.48        GZ00 N
ATOM   6447  CD2 HIS X 201     -31.492 106.492 -30.372  1.00 32.60        GZ00 C
ATOM   6448  CE1 HIS X 201     -32.614 106.425 -32.257  1.00 41.48        GZ00 C
ATOM   6449  NE2 HIS X 201     -32.500 105.865 -31.063  1.00 36.04        GZ00 N
ATOM   6450  N   GLU X 202     -28.100 106.014 -32.636  1.00 40.07        GZ00 N
ATOM   6451  CA  GLU X 202     -27.996 105.307 -33.916  1.00 39.77        GZ00 C
ATOM   6452  C   GLU X 202     -27.068 106.042 -34.889  1.00 44.82        GZ00 C
ATOM   6453  O   GLU X 202     -27.387 106.247 -36.062  1.00 40.88        GZ00 O
ATOM   6454  CB  GLU X 202     -29.376 105.095 -34.535  1.00 36.52        GZ00 C
ATOM   6455  CG  GLU X 202     -30.307 104.272 -33.669  1.00 41.95        GZ00 C
ATOM   6456  CD  GLU X 202     -29.852 102.821 -33.573  1.00 51.67        GZ00 C
```

| ATOM | 6457 | OE1 | GLU | X | 202 | -29.603 | 102.188 | -34.631 | 1.00 | 51.18 | GZ00 | O |
|------|------|-----|-----|---|-----|---------|---------|---------|------|-------|------|---|
| ATOM | 6458 | OE2 | GLU | X | 202 | -29.735 | 102.316 | -32.433 | 1.00 | 49.62 | GZ00 | O1- |
| ATOM | 6459 | N | GLY | X | 203 | -25.911 | 106.464 | -34.381 | 1.00 | 41.22 | GZ00 | N |
| ATOM | 6460 | CA | GLY | X | 203 | -24.926 | 107.146 | -35.193 | 1.00 | 42.73 | GZ00 | C |
| ATOM | 6461 | C | GLY | X | 203 | -25.235 | 108.578 | -35.586 | 1.00 | 47.72 | GZ00 | C |
| ATOM | 6462 | O | GLY | X | 203 | -24.445 | 109.169 | -36.321 | 1.00 | 53.27 | GZ00 | O |
| ATOM | 6463 | N | SER | X | 206 | -26.322 | 109.176 | -35.101 | 1.00 | 52.11 | GZ00 | N |
| ATOM | 6464 | CA | SER | X | 206 | -26.642 | 110.577 | -35.375 | 1.00 | 50.86 | GZ00 | C |
| ATOM | 6465 | C | SER | X | 206 | -26.773 | 111.328 | -34.056 | 1.00 | 52.21 | GZ00 | C |
| ATOM | 6466 | O | SER | X | 206 | -27.448 | 110.853 | -33.136 | 1.00 | 52.48 | GZ00 | O |
| ATOM | 6467 | CB | SER | X | 206 | -27.944 | 110.707 | -36.167 | 1.00 | 47.57 | GZ00 | C |
| ATOM | 6468 | OG | SER | X | 206 | -27.838 | 110.125 | -37.454 | 1.00 | 59.00 | GZ00 | O |
| ATOM | 6469 | N | THR | X | 207 | -26.128 | 112.488 | -33.953 | 1.00 | 50.76 | GZ00 | N |
| ATOM | 6470 | CA | THR | X | 207 | -26.108 | 113.241 | -32.704 | 1.00 | 55.87 | GZ00 | C |
| ATOM | 6471 | C | THR | X | 207 | -27.025 | 114.462 | -32.764 | 1.00 | 57.12 | GZ00 | C |
| ATOM | 6472 | O | THR | X | 207 | -27.074 | 115.180 | -33.767 | 1.00 | 57.64 | GZ00 | O |
| ATOM | 6473 | CB | THR | X | 207 | -24.696 | 113.690 | -32.344 | 1.00 | 48.26 | GZ00 | C |
| ATOM | 6474 | OG1 | THR | X | 207 | -24.359 | 114.813 | -33.157 | 1.00 | 73.99 | GZ00 | O |
| ATOM | 6475 | CG2 | THR | X | 207 | -23.702 | 112.580 | -32.600 | 1.00 | 47.46 | GZ00 | C |
| ATOM | 6476 | N | VAL | X | 208 | -27.777 | 114.659 | -31.690 | 1.00 | 49.79 | GZ00 | N |
| ATOM | 6477 | CA | VAL | X | 208 | -28.598 | 115.842 | -31.472 | 1.00 | 52.71 | GZ00 | C |
| ATOM | 6478 | C | VAL | X | 208 | -27.951 | 116.636 | -30.343 | 1.00 | 55.59 | GZ00 | C |
| ATOM | 6479 | O | VAL | X | 208 | -27.557 | 116.063 | -29.317 | 1.00 | 52.08 | GZ00 | O |
| ATOM | 6480 | CB | VAL | X | 208 | -30.047 | 115.452 | -31.131 | 1.00 | 51.79 | GZ00 | C |
| ATOM | 6481 | CG1 | VAL | X | 208 | -30.880 | 116.672 | -30.802 | 1.00 | 49.77 | GZ00 | C |
| ATOM | 6482 | CG2 | VAL | X | 208 | -30.666 | 114.651 | -32.276 | 1.00 | 48.08 | GZ00 | C |
| ATOM | 6483 | N | GLU | X | 209 | -27.787 | 117.939 | -30.547 | 1.00 | 56.25 | GZ00 | N |
| ATOM | 6484 | CA | GLU | X | 209 | -27.107 | 118.778 | -29.571 | 1.00 | 56.90 | GZ00 | C |
| ATOM | 6485 | C | GLU | X | 209 | -27.976 | 119.957 | -29.174 | 1.00 | 58.39 | GZ00 | C |
| ATOM | 6486 | O | GLU | X | 209 | -28.692 | 120.525 | -30.005 | 1.00 | 55.14 | GZ00 | O |
| ATOM | 6487 | CB | GLU | X | 209 | -25.772 | 119.293 | -30.087 | 1.00 | 57.63 | GZ00 | C |
| ATOM | 6488 | CG | GLU | X | 209 | -25.088 | 120.229 | -29.113 | 1.00 | 64.92 | GZ00 | C |
| ATOM | 6489 | CD | GLU | X | 209 | -23.766 | 120.754 | -29.630 | 1.00 | 76.32 | GZ00 | C |
| ATOM | 6490 | OE1 | GLU | X | 209 | -22.869 | 119.942 | -29.932 | 1.00 | 78.95 | GZ00 | O |
| ATOM | 6491 | OE2 | GLU | X | 209 | -23.636 | 121.986 | -29.766 | 1.00 | 85.57 | GZ00 | O1- |
| ATOM | 6492 | N | LYS | X | 210 | -27.935 | 120.282 | -27.886 | 1.00 | 62.74 | GZ00 | N |
| ATOM | 6493 | CA | LYS | X | 210 | -28.467 | 121.522 | -27.345 | 1.00 | 53.79 | GZ00 | C |
| ATOM | 6494 | C | LYS | X | 210 | -27.379 | 122.171 | -26.505 | 1.00 | 56.56 | GZ00 | C |
| ATOM | 6495 | O | LYS | X | 210 | -26.616 | 121.476 | -25.823 | 1.00 | 56.28 | GZ00 | O |
| ATOM | 6496 | CB | LYS | X | 210 | -29.711 | 121.279 | -26.513 | 1.00 | 53.44 | GZ00 | C |
| ATOM | 6497 | CG | LYS | X | 210 | -30.907 | 120.841 | -27.324 | 1.00 | 54.02 | GZ00 | C |
| ATOM | 6498 | CD | LYS | X | 210 | -31.098 | 121.748 | -28.509 | 1.00 | 54.29 | GZ00 | C |
| ATOM | 6499 | CE | LYS | X | 210 | -32.343 | 121.367 | -29.282 | 1.00 | 55.10 | GZ00 | C |
| ATOM | 6500 | NZ | LYS | X | 210 | -33.545 | 121.832 | -28.528 | 1.00 | 61.72 | GZ00 | N1+ |
| ATOM | 6501 | N | THR | X | 211 | -27.270 | 123.498 | -26.592 | 1.00 | 59.32 | GZ00 | N |
| ATOM | 6502 | CA | THR | X | 211 | -26.267 | 124.229 | -25.828 | 1.00 | 59.32 | GZ00 | C |
| ATOM | 6503 | C | THR | X | 211 | -26.946 | 125.341 | -25.039 | 1.00 | 55.85 | GZ00 | C |
| ATOM | 6504 | O | THR | X | 211 | -27.984 | 125.869 | -25.443 | 1.00 | 52.37 | GZ00 | O |
| ATOM | 6505 | CB | THR | X | 211 | -25.177 | 124.830 | -26.725 | 1.00 | 59.58 | GZ00 | C |
| ATOM | 6506 | OG1 | THR | X | 211 | -25.586 | 126.134 | -27.141 | 1.00 | 68.27 | GZ00 | O |
| ATOM | 6507 | CG2 | THR | X | 211 | -24.975 | 123.970 | -27.975 | 1.00 | 57.88 | GZ00 | C |
| ATOM | 6508 | N | VAL | X | 212 | -26.348 | 125.706 | -23.911 | 1.00 | 52.69 | GZ00 | N |
| ATOM | 6509 | CA | VAL | X | 212 | -26.936 | 126.709 | -23.035 | 1.00 | 63.41 | GZ00 | C |
| ATOM | 6510 | C | VAL | X | 212 | -25.825 | 127.640 | -22.548 | 1.00 | 67.46 | GZ00 | C |
| ATOM | 6511 | O | VAL | X | 212 | -24.665 | 127.233 | -22.408 | 1.00 | 65.23 | GZ00 | O |
| ATOM | 6512 | CB | VAL | X | 212 | -27.702 | 126.029 | -21.866 | 1.00 | 57.88 | GZ00 | C |
| ATOM | 6513 | CG1 | VAL | X | 212 | -26.783 | 125.712 | -20.700 | 1.00 | 44.64 | GZ00 | C |
| ATOM | 6514 | CG2 | VAL | X | 212 | -28.897 | 126.846 | -21.442 | 1.00 | 65.99 | GZ00 | C |
| ATOM | 6515 | N | ALA | X | 213 | -26.174 | 128.916 | -22.332 | 1.00 | 65.82 | GZ00 | N |
| ATOM | 6516 | CA | ALA | X | 213 | -25.166 | 129.919 | -21.988 | 1.00 | 71.28 | GZ00 | C |
| ATOM | 6517 | C | ALA | X | 213 | -25.503 | 130.667 | -20.702 | 1.00 | 73.86 | GZ00 | C |
| ATOM | 6518 | O | ALA | X | 213 | -26.677 | 130.982 | -20.445 | 1.00 | 75.77 | GZ00 | O |
| ATOM | 6519 | CB | ALA | X | 213 | -25.003 | 130.928 | -23.133 | 1.00 | 69.34 | GZ00 | C |
| ATOM | 6520 | N | PRO | X | 214 | -24.486 | 130.994 | -19.889 | 1.00 | 70.92 | GZ00 | N |
| ATOM | 6521 | CA | PRO | X | 214 | -24.746 | 131.692 | -18.614 | 1.00 | 72.76 | GZ00 | C |
| ATOM | 6522 | C | PRO | X | 214 | -25.314 | 133.090 | -18.773 | 1.00 | 82.62 | GZ00 | C |
| ATOM | 6523 | O | PRO | X | 214 | -25.933 | 133.596 | -17.827 | 1.00 | 89.01 | GZ00 | O |
| ATOM | 6524 | CB | PRO | X | 214 | -23.366 | 131.732 | -17.943 | 1.00 | 66.71 | GZ00 | C |
| ATOM | 6525 | CG | PRO | X | 214 | -22.637 | 130.585 | -18.525 | 1.00 | 65.72 | GZ00 | C |
| ATOM | 6526 | CD | PRO | X | 214 | -23.119 | 130.445 | -19.944 | 1.00 | 70.60 | GZ00 | C |
| ATOM | 6527 | N | THR | X | 215 | -25.149 | 133.725 | -19.928 | 1.00 | 84.77 | GZ00 | N |

```
ATOM   6528  CA  THR X 215     -25.832 134.986 -20.202  1.00 96.30     GZ00 C
ATOM   6529  C   THR X 215     -27.332 134.702 -20.252  1.00 95.28     GZ00 C
ATOM   6530  O   THR X 215     -27.859 134.231 -21.265  1.00 91.48     GZ00 O
ATOM   6531  CB  THR X 215     -25.328 135.593 -21.510  1.00 97.14     GZ00 C
ATOM   6532  OG1 THR X 215     -25.743 134.773 -22.612  1.00 87.67     GZ00 O
ATOM   6533  CG2 THR X 215     -23.793 135.691 -21.507  1.00 90.01     GZ00 C
ATOM   6534  N   GLU X 216     -28.027 134.994 -19.152  1.00 96.06     GZ00 N
ATOM   6535  CA  GLU X 216     -29.446 134.662 -19.010  1.00 98.88     GZ00 C
ATOM   6536  C   GLU X 216     -30.325 135.917 -18.923  1.00 99.86     GZ00 C
ATOM   6537  O   GLU X 216     -31.553 135.830 -18.821  1.00 94.58     GZ00 O
ATOM   6538  CB  GLU X 216     -29.662 133.791 -17.762  1.00104.37     GZ00 C
ATOM   6539  CG  GLU X 216     -29.007 132.394 -17.811  1.00103.51     GZ00 C
ATOM   6540  CD  GLU X 216     -29.281 131.561 -16.556  1.00100.31     GZ00 C
ATOM   6541  OE1 GLU X 216     -30.463 131.201 -16.329  1.00 98.05     GZ00 O
ATOM   6542  OE2 GLU X 216     -28.323 131.288 -15.788  1.00 94.94     GZ00 O1-
TER
ATOM   6543  N   THR E 152      -7.503 113.907   0.585  1.00 78.03     B000 N
ATOM   6544  CA  THR E 152      -7.804 113.632   1.990  1.00 91.98     B000 C
ATOM   6545  C   THR E 152      -6.989 112.446   2.537  1.00 87.18     B000 C
ATOM   6546  O   THR E 152      -5.914 112.642   3.113  1.00 87.17     B000 O
ATOM   6547  CB  THR E 152      -9.331 113.377   2.199  1.00 99.46     B000 C
ATOM   6548  OG1 THR E 152      -9.547 112.614   3.396  1.00103.56     B000 O
ATOM   6549  CG2 THR E 152      -9.959 112.661   0.997  1.00 88.04     B000 C
ATOM   6550  N   CYS E 153      -7.511 111.230   2.378  1.00 83.15     B000 N
ATOM   6551  CA  CYS E 153      -6.804 109.997   2.699  1.00 86.88     B000 C
ATOM   6552  C   CYS E 153      -6.722 109.106   1.463  1.00 80.76     B000 C
ATOM   6553  O   CYS E 153      -7.463 109.278   0.486  1.00 71.27     B000 O
ATOM   6554  CB  CYS E 153      -7.471 109.227   3.860  1.00 86.61     B000 C
ATOM   6555  SG  CYS E 153      -6.738 109.471   5.513  1.00 90.81     B000 S
ATOM   6556  N   CYS E 154      -5.817 108.133   1.525  1.00 72.71     B000 N
ATOM   6557  CA  CYS E 154      -5.632 107.218   0.411  1.00 64.86     B000 C
ATOM   6558  C   CYS E 154      -6.850 106.311   0.243  1.00 66.48     B000 C
ATOM   6559  O   CYS E 154      -7.564 106.023   1.210  1.00 66.73     B000 O
ATOM   6560  CB  CYS E 154      -4.385 106.373   0.626  1.00 57.69     B000 C
ATOM   6561  SG  CYS E 154      -2.833 107.296   0.371  1.00 72.69     B000 S
ATOM   6562  N   PRO E 155      -7.115 105.854  -0.978  1.00 56.60     B000 N
ATOM   6563  CA  PRO E 155      -8.220 104.914  -1.192  1.00 57.19     B000 C
ATOM   6564  C   PRO E 155      -8.052 103.661  -0.345  1.00 62.02     B000 C
ATOM   6565  O   PRO E 155      -7.012 103.404   0.267  1.00 60.28     B000 O
ATOM   6566  CB  PRO E 155      -8.143 104.589  -2.687  1.00 52.85     B000 C
ATOM   6567  CG  PRO E 155      -7.332 105.677  -3.277  1.00 56.95     B000 C
ATOM   6568  CD  PRO E 155      -6.392 106.158  -2.220  1.00 54.57     B000 C
ATOM   6569  N   VAL E 156      -9.123 102.871  -0.306  1.00 64.34     B000 N
ATOM   6570  CA  VAL E 156      -9.121 101.644   0.477  1.00 62.48     B000 C
ATOM   6571  C   VAL E 156      -8.013 100.726  -0.021  1.00 57.31     B000 C
ATOM   6572  O   VAL E 156      -7.843 100.536  -1.235  1.00 55.79     B000 O
ATOM   6573  CB  VAL E 156     -10.498 100.968   0.373  1.00 58.69     B000 C
ATOM   6574  CG1 VAL E 156     -10.628  99.853   1.400  1.00 62.99     B000 C
ATOM   6575  CG2 VAL E 156     -11.612 102.013   0.508  1.00 72.16     B000 C
ATOM   6576  N   ASN E 157      -7.244 100.163   0.915  1.00 49.37     B000 N
ATOM   6577  CA  ASN E 157      -6.142  99.218   0.687  1.00 55.22     B000 C
ATOM   6578  C   ASN E 157      -4.871  99.882   0.162  1.00 52.64     B000 C
ATOM   6579  O   ASN E 157      -3.871  99.180  -0.036  1.00 49.46     B000 O
ATOM   6580  CB  ASN E 157      -6.513  98.075  -0.276  1.00 51.75     B000 C
ATOM   6581  CG  ASN E 157      -7.713  97.282   0.202  1.00 58.73     B000 C
ATOM   6582  OD1 ASN E 157      -7.839  97.001   1.392  1.00 60.67     B000 O
ATOM   6583  ND2 ASN E 157      -8.609  96.935  -0.720  1.00 58.30     B000 N
ATOM   6584  N   TRP E 158      -4.868 101.187  -0.082  1.00 49.22     B000 N
ATOM   6585  CA  TRP E 158      -3.644 101.885  -0.433  1.00 45.78     B000 C
ATOM   6586  C   TRP E 158      -2.983 102.464   0.818  1.00 47.63     B000 C
ATOM   6587  O   TRP E 158      -3.618 102.655   1.855  1.00 53.96     B000 O
ATOM   6588  CB  TRP E 158      -3.933 102.979  -1.453  1.00 48.76     B000 C
ATOM   6589  CG  TRP E 158      -4.381 102.473  -2.793  1.00 40.31     B000 C
ATOM   6590  CD1 TRP E 158      -5.498 101.731  -3.072  1.00 46.46     B000 C
ATOM   6591  CD2 TRP E 158      -3.718 102.683  -4.043  1.00 35.79     B000 C
ATOM   6592  NE1 TRP E 158      -5.573 101.465  -4.432  1.00 39.86     B000 N
ATOM   6593  CE2 TRP E 158      -4.495 102.050  -5.047  1.00 45.74     B000 C
ATOM   6594  CE3 TRP E 158      -2.548 103.355  -4.417  1.00 40.22     B000 C
ATOM   6595  CZ2 TRP E 158      -4.132 102.067  -6.395  1.00 41.97     B000 C
ATOM   6596  CZ3 TRP E 158      -2.193 103.378  -5.764  1.00 41.46     B000 C
ATOM   6597  CH2 TRP E 158      -2.980 102.727  -6.732  1.00 40.39     B000 C
```

```
ATOM   6598  N    VAL E 159    -1.689 102.734   0.702  1.00 44.22    B000 N
ATOM   6599  CA   VAL E 159    -0.837 103.172   1.800  1.00 48.43    B000 C
ATOM   6600  C    VAL E 159    -0.297 104.558   1.461  1.00 58.07    B000 C
ATOM   6601  O    VAL E 159     0.103 104.818   0.318  1.00 55.09    B000 O
ATOM   6602  CB   VAL E 159     0.330 102.189   2.039  1.00 52.63    B000 C
ATOM   6603  CG1  VAL E 159     1.251 102.704   3.126  1.00 54.92    B000 C
ATOM   6604  CG2  VAL E 159    -0.184 100.818   2.407  1.00 53.05    B000 C
ATOM   6605  N    GLU E 160    -0.275 105.443   2.456  1.00 57.15    B000 N
ATOM   6606  CA   GLU E 160     0.120 106.833   2.256  1.00 59.51    B000 C
ATOM   6607  C    GLU E 160     1.577 107.054   2.654  1.00 55.30    B000 C
ATOM   6608  O    GLU E 160     2.043 106.531   3.673  1.00 50.50    B000 O
ATOM   6609  CB   GLU E 160    -0.809 107.756   3.046  1.00 51.43    B000 C
ATOM   6610  CG   GLU E 160    -0.440 109.219   3.035  1.00 65.54    B000 C
ATOM   6611  CD   GLU E 160    -1.336 110.058   3.952  1.00 77.96    B000 C
ATOM   6612  OE1  GLU E 160    -2.418 109.568   4.357  1.00 79.30    B000 O
ATOM   6613  OE2  GLU E 160    -0.951 111.203   4.281  1.00 75.66    B000 O1-
ATOM   6614  N    HIS E 161     2.304 107.801   1.822  1.00 53.62    B000 N
ATOM   6615  CA   HIS E 161     3.658 108.222   2.177  1.00 53.50    B000 C
ATOM   6616  C    HIS E 161     4.013 109.474   1.393  1.00 61.66    B000 C
ATOM   6617  O    HIS E 161     3.896 109.472   0.159  1.00 55.60    B000 O
ATOM   6618  CB   HIS E 161     4.656 107.120   1.884  1.00 50.97    B000 C
ATOM   6619  CG   HIS E 161     6.076 107.514   2.135  1.00 58.56    B000 C
ATOM   6620  ND1  HIS E 161     6.845 108.173   1.196  1.00 62.19    B000 N
ATOM   6621  CD2  HIS E 161     6.877 107.322   3.213  1.00 53.33    B000 C
ATOM   6622  CE1  HIS E 161     8.056 108.376   1.687  1.00 63.04    B000 C
ATOM   6623  NE2  HIS E 161     8.102 107.869   2.909  1.00 61.41    B000 N
ATOM   6624  N    GLU E 162     4.410 110.541   2.112  1.00 61.25    B000 N
ATOM   6625  CA   GLU E 162     4.767 111.850   1.561  1.00 55.02    B000 C
ATOM   6626  C    GLU E 162     3.975 112.247   0.322  1.00 62.64    B000 C
ATOM   6627  O    GLU E 162     4.537 112.301  -0.778  1.00 75.86    B000 O
ATOM   6628  CB   GLU E 162     6.267 111.936   1.225  1.00 60.44    B000 C
ATOM   6629  CG   GLU E 162     7.223 111.633   2.366  1.00 61.24    B000 C
ATOM   6630  CD   GLU E 162     8.697 111.610   1.933  1.00 86.98    B000 C
ATOM   6631  OE1  GLU E 162     9.025 111.099   0.822  1.00 83.67    B000 O
ATOM   6632  OE2  GLU E 162     9.538 112.097   2.725  1.00 95.90    B000 O1-
ATOM   6633  N    ARG E 163     2.679 112.496   0.469  1.00 55.87    B000 N
ATOM   6634  CA   ARG E 163     1.819 113.000  -0.602  1.00 76.66    B000 C
ATOM   6635  C    ARG E 163     1.554 112.012  -1.738  1.00 68.88    B000 C
ATOM   6636  O    ARG E 163     0.922 112.388  -2.739  1.00 65.44    B000 O
ATOM   6637  CB   ARG E 163     2.398 114.288  -1.215  1.00 89.70    B000 C
ATOM   6638  CG   ARG E 163     2.928 115.328  -0.208  1.00 98.04    B000 C
ATOM   6639  CD   ARG E 163     3.983 116.257  -0.835  1.00106.40    B000 C
ATOM   6640  NE   ARG E 163     3.452 117.044  -1.950  1.00124.58    B000 N
ATOM   6641  CZ   ARG E 163     3.588 116.723  -3.236  1.00120.88    B000 C
ATOM   6642  NH1  ARG E 163     4.245 115.622  -3.585  1.00111.66    B000 N1+
ATOM   6643  NH2  ARG E 163     3.063 117.503  -4.176  1.00118.93    B000 N
ATOM   6644  N    SER E 164     1.973 110.758  -1.622  1.00 63.49    B000 N
ATOM   6645  CA   SER E 164     1.606 109.768  -2.620  1.00 57.22    B000 C
ATOM   6646  C    SER E 164     0.888 108.601  -1.952  1.00 52.22    B000 C
ATOM   6647  O    SER E 164     1.071 108.329  -0.761  1.00 49.98    B000 O
ATOM   6648  CB   SER E 164     2.823 109.290  -3.411  1.00 56.71    B000 C
ATOM   6649  OG   SER E 164     3.161 110.233  -4.411  1.00 65.04    B000 O
ATOM   6650  N    CYS E 165     0.066 107.913  -2.740  1.00 50.88    B000 N
ATOM   6651  CA   CYS E 165    -0.614 106.691  -2.322  1.00 53.42    B000 C
ATOM   6652  C    CYS E 165    -0.042 105.497  -3.076  1.00 50.12    B000 C
ATOM   6653  O    CYS E 165     0.124 105.555  -4.300  1.00 46.54    B000 O
ATOM   6654  CB   CYS E 165    -2.118 106.793  -2.576  1.00 56.64    B000 C
ATOM   6655  SG   CYS E 165    -2.945 108.028  -1.541  1.00 67.86    B000 S
ATOM   6656  N    TYR E 166     0.226 104.410  -2.350  1.00 52.80    B000 N
ATOM   6657  CA   TYR E 166     0.904 103.231  -2.882  1.00 48.23    B000 C
ATOM   6658  C    TYR E 166     0.064 101.984  -2.650  1.00 48.96    B000 C
ATOM   6659  O    TYR E 166    -0.538 101.821  -1.584  1.00 46.07    B000 O
ATOM   6660  CB   TYR E 166     2.265 103.033  -2.222  1.00 45.49    B000 C
ATOM   6661  CG   TYR E 166     3.196 104.186  -2.406  1.00 51.14    B000 C
ATOM   6662  CD1  TYR E 166     3.000 105.382  -1.709  1.00 50.78    B000 C
ATOM   6663  CD2  TYR E 166     4.296 104.081  -3.249  1.00 49.21    B000 C
ATOM   6664  CE1  TYR E 166     3.859 106.442  -1.867  1.00 52.01    B000 C
ATOM   6665  CE2  TYR E 166     5.175 105.138  -3.408  1.00 52.82    B000 C
ATOM   6666  CZ   TYR E 166     4.954 106.317  -2.714  1.00 56.72    B000 C
ATOM   6667  OH   TYR E 166     5.829 107.368  -2.874  1.00 55.75    B000 O
ATOM   6668  N    TRP E 167     0.064 101.085  -3.634  1.00 43.75    B000 N
```

| ATOM | 6669 | CA | TRP | E | 167 | -0.639 | 99.813 | -3.539 | 1.00 | 41.16 | B000 | C |
| ATOM | 6670 | C | TRP | E | 167 | 0.346 | 98.684 | -3.794 | 1.00 | 42.24 | B000 | C |
| ATOM | 6671 | O | TRP | E | 167 | 1.054 | 98.693 | -4.804 | 1.00 | 37.42 | B000 | O |
| ATOM | 6672 | CB | TRP | E | 167 | -1.804 | 99.743 | -4.530 | 1.00 | 42.53 | B000 | C |
| ATOM | 6673 | CG | TRP | E | 167 | -2.599 | 98.467 | -4.395 | 1.00 | 46.44 | B000 | C |
| ATOM | 6674 | CD1 | TRP | E | 167 | -3.662 | 98.242 | -3.560 | 1.00 | 49.81 | B000 | C |
| ATOM | 6675 | CD2 | TRP | E | 167 | -2.395 | 97.251 | -5.116 | 1.00 | 43.13 | B000 | C |
| ATOM | 6676 | NE1 | TRP | E | 167 | -4.114 | 96.955 | -3.706 | 1.00 | 51.07 | B000 | N |
| ATOM | 6677 | CE2 | TRP | E | 167 | -3.366 | 96.330 | -4.665 | 1.00 | 46.50 | B000 | C |
| ATOM | 6678 | CE3 | TRP | E | 167 | -1.491 | 96.852 | -6.103 | 1.00 | 40.37 | B000 | C |
| ATOM | 6679 | CZ2 | TRP | E | 167 | -3.455 | 95.041 | -5.165 | 1.00 | 36.86 | B000 | C |
| ATOM | 6680 | CZ3 | TRP | E | 167 | -1.587 | 95.579 | -6.604 | 1.00 | 39.69 | B000 | C |
| ATOM | 6681 | CH2 | TRP | E | 167 | -2.554 | 94.682 | -6.133 | 1.00 | 42.15 | B000 | C |
| ATOM | 6682 | N | PHE | E | 168 | 0.377 | 97.709 | -2.891 | 1.00 | 42.39 | B000 | N |
| ATOM | 6683 | CA | PHE | E | 168 | 1.369 | 96.645 | -2.921 | 1.00 | 39.44 | B000 | C |
| ATOM | 6684 | C | PHE | E | 168 | 0.718 | 95.320 | -3.300 | 1.00 | 41.71 | B000 | C |
| ATOM | 6685 | O | PHE | E | 168 | -0.056 | 94.762 | -2.515 | 1.00 | 39.56 | B000 | O |
| ATOM | 6686 | CB | PHE | E | 168 | 2.054 | 96.539 | -1.567 | 1.00 | 33.24 | B000 | C |
| ATOM | 6687 | CG | PHE | E | 168 | 2.834 | 97.745 | -1.209 | 1.00 | 45.15 | B000 | C |
| ATOM | 6688 | CD1 | PHE | E | 168 | 4.165 | 97.858 | -1.603 | 1.00 | 43.49 | B000 | C |
| ATOM | 6689 | CD2 | PHE | E | 168 | 2.251 | 98.776 | -0.472 | 1.00 | 43.18 | B000 | C |
| ATOM | 6690 | CE1 | PHE | E | 168 | 4.917 | 98.991 | -1.279 | 1.00 | 48.63 | B000 | C |
| ATOM | 6691 | CE2 | PHE | E | 168 | 2.986 | 99.913 | -0.135 | 1.00 | 49.01 | B000 | C |
| ATOM | 6692 | CZ | PHE | E | 168 | 4.326 | 100.026 | -0.541 | 1.00 | 48.19 | B000 | C |
| ATOM | 6693 | N | SER | E | 169 | 1.081 | 94.788 | -4.469 | 1.00 | 34.62 | B000 | N |
| ATOM | 6694 | CA | SER | E | 169 | 0.586 | 93.478 | -4.863 | 1.00 | 37.57 | B000 | C |
| ATOM | 6695 | C | SER | E | 169 | 1.143 | 92.402 | -3.936 | 1.00 | 34.54 | B000 | C |
| ATOM | 6696 | O | SER | E | 169 | 2.169 | 92.574 | -3.280 | 1.00 | 36.05 | B000 | O |
| ATOM | 6697 | CB | SER | E | 169 | 0.978 | 93.141 | -6.308 | 1.00 | 33.43 | B000 | C |
| ATOM | 6698 | OG | SER | E | 169 | 2.306 | 92.629 | -6.362 | 1.00 | 34.63 | B000 | O |
| ATOM | 6699 | N | ARG | E | 170 | 0.449 | 91.272 | -3.899 | 1.00 | 35.37 | B000 | N |
| ATOM | 6700 | CA | ARG | E | 170 | 0.918 | 90.093 | -3.184 | 1.00 | 37.90 | B000 | C |
| ATOM | 6701 | C | ARG | E | 170 | 0.997 | 88.896 | -4.131 | 1.00 | 35.62 | B000 | C |
| ATOM | 6702 | O | ARG | E | 170 | 0.924 | 87.742 | -3.715 | 1.00 | 37.12 | B000 | O |
| ATOM | 6703 | CB | ARG | E | 170 | 0.028 | 89.827 | -1.967 | 1.00 | 36.19 | B000 | C |
| ATOM | 6704 | CG | ARG | E | 170 | 0.216 | 90.900 | -0.870 | 1.00 | 40.70 | B000 | C |
| ATOM | 6705 | CD | ARG | E | 170 | -0.749 | 90.780 | 0.304 | 1.00 | 45.71 | B000 | C |
| ATOM | 6706 | NE | ARG | E | 170 | -2.082 | 91.308 | -0.011 | 1.00 | 51.47 | B000 | N |
| ATOM | 6707 | CZ | ARG | E | 170 | -3.156 | 91.221 | 0.782 | 1.00 | 53.18 | B000 | C |
| ATOM | 6708 | NH1 | ARG | E | 170 | -3.089 | 90.606 | 1.963 | 1.00 | 52.87 | B000 | N1+ |
| ATOM | 6709 | NH2 | ARG | E | 170 | -4.315 | 91.737 | 0.384 | 1.00 | 52.00 | B000 | N |
| ATOM | 6710 | N | SER | E | 171 | 1.194 | 89.177 | -5.414 | 1.00 | 34.01 | B000 | N |
| ATOM | 6711 | CA | SER | E | 171 | 1.334 | 88.153 | -6.435 | 1.00 | 37.47 | B000 | C |
| ATOM | 6712 | C | SER | E | 171 | 2.092 | 88.761 | -7.607 | 1.00 | 36.87 | B000 | C |
| ATOM | 6713 | O | SER | E | 171 | 2.269 | 89.978 | -7.692 | 1.00 | 35.85 | B000 | O |
| ATOM | 6714 | CB | SER | E | 171 | -0.030 | 87.625 | -6.873 | 1.00 | 33.20 | B000 | C |
| ATOM | 6715 | OG | SER | E | 171 | -0.796 | 88.688 | -7.418 | 1.00 | 38.78 | B000 | O |
| ATOM | 6716 | N | GLY | E | 172 | 2.519 | 87.895 | -8.521 | 1.00 | 30.60 | B000 | N |
| ATOM | 6717 | CA | GLY | E | 172 | 3.411 | 88.281 | -9.590 | 1.00 | 32.88 | B000 | C |
| ATOM | 6718 | C | GLY | E | 172 | 2.710 | 88.558 | -10.905 | 1.00 | 33.42 | B000 | C |
| ATOM | 6719 | O | GLY | E | 172 | 1.628 | 88.050 | -11.168 | 1.00 | 38.97 | B000 | O |
| ATOM | 6720 | N | LYS | E | 173 | 3.360 | 89.389 | -11.723 | 1.00 | 34.61 | B000 | N |
| ATOM | 6721 | CA | LYS | E | 173 | 2.968 | 89.700 | -13.090 | 1.00 | 31.95 | B000 | C |
| ATOM | 6722 | C | LYS | E | 173 | 4.217 | 90.055 | -13.875 | 1.00 | 32.50 | B000 | C |
| ATOM | 6723 | O | LYS | E | 173 | 5.168 | 90.618 | -13.327 | 1.00 | 29.30 | B000 | O |
| ATOM | 6724 | CB | LYS | E | 173 | 1.999 | 90.887 | -13.185 | 1.00 | 35.19 | B000 | C |
| ATOM | 6725 | CG | LYS | E | 173 | 0.540 | 90.586 | -12.876 | 1.00 | 36.71 | B000 | C |
| ATOM | 6726 | CD | LYS | E | 173 | -0.272 | 91.847 | -13.150 | 1.00 | 36.28 | B000 | C |
| ATOM | 6727 | CE | LYS | E | 173 | -1.752 | 91.726 | -12.775 | 1.00 | 37.38 | B000 | C |
| ATOM | 6728 | NZ | LYS | E | 173 | -2.515 | 90.874 | -13.720 | 1.00 | 40.13 | B000 | N1+ |
| ATOM | 6729 | N | ALA | E | 174 | 4.199 | 89.739 | -15.165 | 1.00 | 34.25 | B000 | N |
| ATOM | 6730 | CA | ALA | E | 174 | 5.174 | 90.319 | -16.074 | 1.00 | 31.99 | B000 | C |
| ATOM | 6731 | C | ALA | E | 174 | 5.058 | 91.834 | -16.026 | 1.00 | 33.17 | B000 | C |
| ATOM | 6732 | O | ALA | E | 174 | 3.962 | 92.387 | -15.834 | 1.00 | 28.18 | B000 | O |
| ATOM | 6733 | CB | ALA | E | 174 | 4.938 | 89.837 | -17.503 | 1.00 | 26.95 | B000 | C |
| ATOM | 6734 | N | TRP | E | 175 | 6.199 | 92.506 | -16.231 | 1.00 | 27.62 | B000 | N |
| ATOM | 6735 | CA | TRP | E | 175 | 6.257 | 93.962 | -16.080 | 1.00 | 28.95 | B000 | C |
| ATOM | 6736 | C | TRP | E | 175 | 5.178 | 94.667 | -16.903 | 1.00 | 31.61 | B000 | C |
| ATOM | 6737 | O | TRP | E | 175 | 4.487 | 95.560 | -16.403 | 1.00 | 35.29 | B000 | O |
| ATOM | 6738 | CB | TRP | E | 175 | 7.641 | 94.464 | -16.472 | 1.00 | 34.77 | B000 | C |
| ATOM | 6739 | CG | TRP | E | 175 | 7.893 | 95.874 | -16.085 | 1.00 | 35.77 | B000 | C |

```
ATOM   6740  CD1 TRP E 175     8.505  96.308 -14.950  1.00 35.08      B000 C
ATOM   6741  CD2 TRP E 175     7.574  97.046 -16.846  1.00 33.62      B000 C
ATOM   6742  NE1 TRP E 175     8.582  97.688 -14.946  1.00 39.22      B000 N
ATOM   6743  CE2 TRP E 175     8.021  98.164 -16.099  1.00 37.24      B000 C
ATOM   6744  CE3 TRP E 175     6.965  97.259 -18.086  1.00 30.92      B000 C
ATOM   6745  CZ2 TRP E 175     7.878  99.471 -16.550  1.00 34.67      B000 C
ATOM   6746  CZ3 TRP E 175     6.812  98.559 -18.531  1.00 41.35      B000 C
ATOM   6747  CH2 TRP E 175     7.273  99.651 -17.769  1.00 41.09      B000 C
ATOM   6748  N   ALA E 176     5.003  94.268 -18.164  1.00 31.68      B000 N
ATOM   6749  CA  ALA E 176     4.017  94.938 -19.011  1.00 34.92      B000 C
ATOM   6750  C   ALA E 176     2.598  94.731 -18.504  1.00 42.84      B000 C
ATOM   6751  O   ALA E 176     1.760  95.625 -18.637  1.00 44.50      B000 O
ATOM   6752  CB  ALA E 176     4.121  94.464 -20.459  1.00 27.15      B000 C
ATOM   6753  N   ASP E 177     2.296  93.569 -17.928  1.00 38.72      B000 N
ATOM   6754  CA  ASP E 177     0.959  93.397 -17.376  1.00 38.20      B000 C
ATOM   6755  C   ASP E 177     0.800  94.189 -16.085  1.00 37.56      B000 C
ATOM   6756  O   ASP E 177    -0.277  94.742 -15.824  1.00 35.06      B000 O
ATOM   6757  CB  ASP E 177     0.658  91.915 -17.144  1.00 38.76      B000 C
ATOM   6758  CG  ASP E 177     0.524  91.144 -18.441  1.00 44.40      B000 C
ATOM   6759  OD1 ASP E 177    -0.060  91.696 -19.397  1.00 46.46      B000 O
ATOM   6760  OD2 ASP E 177     1.027  90.000 -18.513  1.00 46.19      B000 O1-
ATOM   6761  N   ALA E 178     1.851  94.231 -15.255  1.00 34.06      B000 N
ATOM   6762  CA  ALA E 178     1.810  95.046 -14.045  1.00 37.97      B000 C
ATOM   6763  C   ALA E 178     1.710  96.521 -14.403  1.00 38.84      B000 C
ATOM   6764  O   ALA E 178     1.028  97.294 -13.722  1.00 36.52      B000 O
ATOM   6765  CB  ALA E 178     3.040  94.776 -13.174  1.00 36.54      B000 C
ATOM   6766  N   ASP E 179     2.391  96.921 -15.471  1.00 38.85      B000 N
ATOM   6767  CA  ASP E 179     2.284  98.283 -15.969  1.00 39.27      B000 C
ATOM   6768  C   ASP E 179     0.840  98.610 -16.344  1.00 44.49      B000 C
ATOM   6769  O   ASP E 179     0.287  99.630 -15.911  1.00 43.34      B000 O
ATOM   6770  CB  ASP E 179     3.233  98.437 -17.155  1.00 41.76      B000 C
ATOM   6771  CG  ASP E 179     3.180  99.817 -17.795  1.00 53.27      B000 C
ATOM   6772  OD1 ASP E 179     3.353 100.836 -17.081  1.00 46.80      B000 O
ATOM   6773  OD2 ASP E 179     2.980  99.863 -19.031  1.00 46.92      B000 O1-
ATOM   6774  N   ASN E 180     0.193  97.723 -17.106  1.00 42.03      B000 N
ATOM   6775  CA  ASN E 180    -1.199  97.957 -17.482  1.00 44.06      B000 C
ATOM   6776  C   ASN E 180    -2.105  97.976 -16.261  1.00 47.76      B000 C
ATOM   6777  O   ASN E 180    -3.071  98.746 -16.212  1.00 47.34      B000 O
ATOM   6778  CB  ASN E 180    -1.687  96.905 -18.488  1.00 43.06      B000 C
ATOM   6779  CG  ASN E 180    -1.015  97.046 -19.870  1.00 67.38      B000 C
ATOM   6780  OD1 ASN E 180    -0.489  98.113 -20.216  1.00 70.80      B000 O
ATOM   6781  ND2 ASN E 180    -1.053  95.971 -20.670  1.00 65.59      B000 N
ATOM   6782  N   TYR E 181    -1.826  97.126 -15.271  1.00 39.12      B000 N
ATOM   6783  CA  TYR E 181    -2.653  97.125 -14.068  1.00 43.01      B000 C
ATOM   6784  C   TYR E 181    -2.651  98.498 -13.388  1.00 42.74      B000 C
ATOM   6785  O   TYR E 181    -3.710  99.015 -13.016  1.00 42.29      B000 O
ATOM   6786  CB  TYR E 181    -2.191  96.021 -13.109  1.00 37.06      B000 C
ATOM   6787  CG  TYR E 181    -2.917  96.012 -11.794  1.00 38.21      B000 C
ATOM   6788  CD1 TYR E 181    -2.558  96.897 -10.772  1.00 34.22      B000 C
ATOM   6789  CD2 TYR E 181    -3.956  95.118 -11.558  1.00 37.19      B000 C
ATOM   6790  CE1 TYR E 181    -3.234  96.912  -9.562  1.00 38.89      B000 C
ATOM   6791  CE2 TYR E 181    -4.640  95.112 -10.337  1.00 36.01      B000 C
ATOM   6792  CZ  TYR E 181    -4.276  96.010  -9.345  1.00 44.74      B000 C
ATOM   6793  OH  TYR E 181    -4.927  95.998  -8.128  1.00 40.18      B000 O
ATOM   6794  N   CYS E 182    -1.472  99.100 -13.203  1.00 38.84      B000 N
ATOM   6795  CA  CYS E 182    -1.422 100.388 -12.511  1.00 45.63      B000 C
ATOM   6796  C   CYS E 182    -2.149 101.467 -13.312  1.00 47.11      B000 C
ATOM   6797  O   CYS E 182    -2.911 102.262 -12.747  1.00 44.58      B000 O
ATOM   6798  CB  CYS E 182     0.031 100.804 -12.239  1.00 42.50      B000 C
ATOM   6799  SG  CYS E 182     0.953  99.774 -11.010  1.00 49.37      B000 S
ATOM   6800  N   ARG E 183    -1.957 101.483 -14.636  1.00 46.95      B000 N
ATOM   6801  CA  ARG E 183    -2.612 102.478 -15.481  1.00 44.11      B000 C
ATOM   6802  C   ARG E 183    -4.130 102.366 -15.394  1.00 50.00      B000 C
ATOM   6803  O   ARG E 183    -4.833 103.386 -15.359  1.00 44.76      B000 O
ATOM   6804  CB  ARG E 183    -2.113 102.348 -16.926  1.00 40.71      B000 C
ATOM   6805  CG  ARG E 183    -0.821 103.153 -17.173  1.00 63.11      B000 C
ATOM   6806  CD  ARG E 183     0.070 102.668 -18.340  1.00 66.43      B000 C
ATOM   6807  NE  ARG E 183     1.428 103.228 -18.202  1.00 85.06      B000 N
ATOM   6808  CZ  ARG E 183     2.473 102.943 -18.989  1.00 82.14      B000 C
ATOM   6809  NH1 ARG E 183     2.333 102.088 -19.998  1.00 86.34      B000 N1+
ATOM   6810  NH2 ARG E 183     3.667 103.501 -18.759  1.00 56.98      B000 N
```

364

```
ATOM   6811  N    LEU E 184     -4.656 101.136 -15.327  1.00 45.91      B000 N
ATOM   6812  CA   LEU E 184     -6.094 100.958 -15.156  1.00 43.34      B000 C
ATOM   6813  C    LEU E 184     -6.575 101.442 -13.804  1.00 44.25      B000 C
ATOM   6814  O    LEU E 184     -7.769 101.694 -13.646  1.00 51.06      B000 O
ATOM   6815  CB   LEU E 184     -6.497  99.490 -15.302  1.00 47.73      B000 C
ATOM   6816  CG   LEU E 184     -6.471  98.840 -16.680  1.00 54.62      B000 C
ATOM   6817  CD1  LEU E 184     -6.890  97.380 -16.546  1.00 41.74      B000 C
ATOM   6818  CD2  LEU E 184     -7.353  99.606 -17.678  1.00 43.16      B000 C
ATOM   6819  N    GLU E 185     -5.685 101.579 -12.827  1.00 49.85      B000 N
ATOM   6820  CA   GLU E 185     -6.032 102.169 -11.541  1.00 49.61      B000 C
ATOM   6821  C    GLU E 185     -5.766 103.658 -11.502  1.00 48.64      B000 C
ATOM   6822  O    GLU E 185     -5.704 104.233 -10.412  1.00 53.97      B000 O
ATOM   6823  CB   GLU E 185     -5.258 101.484 -10.416  1.00 52.10      B000 C
ATOM   6824  CG   GLU E 185     -5.516 100.002 -10.355  1.00 58.31      B000 C
ATOM   6825  CD   GLU E 185     -6.899  99.698  -9.827  1.00 64.50      B000 C
ATOM   6826  OE1  GLU E 185     -7.284 100.277  -8.780  1.00 62.30      B000 O
ATOM   6827  OE2  GLU E 185     -7.614  98.911 -10.485  1.00 74.36      B000 O1-
ATOM   6828  N    ASP E 186     -5.571 104.286 -12.662  1.00 44.48      B000 N
ATOM   6829  CA   ASP E 186     -5.173 105.692 -12.731  1.00 54.36      B000 C
ATOM   6830  C    ASP E 186     -3.929 105.951 -11.874  1.00 51.31      B000 C
ATOM   6831  O    ASP E 186     -3.859 106.883 -11.069  1.00 57.99      B000 O
ATOM   6832  CB   ASP E 186     -6.337 106.604 -12.332  1.00 56.91      B000 C
ATOM   6833  CG   ASP E 186     -6.105 108.048 -12.733  1.00 74.54      B000 C
ATOM   6834  OD1  ASP E 186     -5.482 108.279 -13.798  1.00 78.01      B000 O
ATOM   6835  OD2  ASP E 186     -6.541 108.948 -11.982  1.00 75.17      B000 O1-
ATOM   6836  N    ALA E 187     -2.940 105.083 -12.033  1.00 50.91      B000 N
ATOM   6837  CA   ALA E 187     -1.713 105.165 -11.265  1.00 42.37      B000 C
ATOM   6838  C    ALA E 187     -0.593 104.718 -12.183  1.00 37.72      B000 C
ATOM   6839  O    ALA E 187     -0.810 104.469 -13.372  1.00 44.89      B000 O
ATOM   6840  CB   ALA E 187     -1.820 104.332  -9.980  1.00 41.53      B000 C
ATOM   6841  N    HIS E 188      0.612 104.603 -11.641  1.00 40.45      B000 N
ATOM   6842  CA   HIS E 188      1.724 104.095 -12.422  1.00 40.26      B000 C
ATOM   6843  C    HIS E 188      2.622 103.249 -11.526  1.00 42.46      B000 C
ATOM   6844  O    HIS E 188      2.526 103.288 -10.296  1.00 40.81      B000 O
ATOM   6845  CB   HIS E 188      2.499 105.237 -13.079  1.00 40.08      B000 C
ATOM   6846  CG   HIS E 188      2.996 106.266 -12.107  1.00 47.93      B000 C
ATOM   6847  ND1  HIS E 188      4.133 106.085 -11.346  1.00 46.18      B000 N
ATOM   6848  CD2  HIS E 188      2.508 107.486 -11.772  1.00 40.80      B000 C
ATOM   6849  CE1  HIS E 188      4.327 107.151 -10.587  1.00 51.18      B000 C
ATOM   6850  NE2  HIS E 188      3.358 108.017 -10.829  1.00 50.42      B000 N
ATOM   6851  N    LEU E 189      3.518 102.490 -12.157  1.00 37.36      B000 N
ATOM   6852  CA   LEU E 189      4.459 101.686 -11.393  1.00 38.98      B000 C
ATOM   6853  C    LEU E 189      5.384 102.583 -10.588  1.00 38.79      B000 C
ATOM   6854  O    LEU E 189      5.766 103.663 -11.040  1.00 44.62      B000 O
ATOM   6855  CB   LEU E 189      5.264 100.782 -12.324  1.00 42.15      B000 C
ATOM   6856  CG   LEU E 189      4.561  99.535 -12.851  1.00 38.45      B000 C
ATOM   6857  CD1  LEU E 189      5.376  98.948 -13.979  1.00 31.59      B000 C
ATOM   6858  CD2  LEU E 189      4.384  98.533 -11.707  1.00 36.53      B000 C
ATOM   6859  N    VAL E 190      5.742 102.130  -9.386  1.00 33.02      B000 N
ATOM   6860  CA   VAL E 190      6.360 103.022  -8.410  1.00 37.67      B000 C
ATOM   6861  C    VAL E 190      7.627 103.653  -8.977  1.00 41.59      B000 C
ATOM   6862  O    VAL E 190      8.477 102.978  -9.571  1.00 39.37      B000 O
ATOM   6863  CB   VAL E 190      6.626 102.274  -7.093  1.00 40.50      B000 C
ATOM   6864  CG1  VAL E 190      7.570 101.068  -7.292  1.00 36.01      B000 C
ATOM   6865  CG2  VAL E 190      7.216 103.229  -6.056  1.00 39.66      B000 C
ATOM   6866  N    VAL E 191      7.751 104.967  -8.792  1.00 42.38      B000 N
ATOM   6867  CA   VAL E 191      8.934 105.734  -9.169  1.00 38.93      B000 C
ATOM   6868  C    VAL E 191      9.618 106.183  -7.886  1.00 41.89      B000 C
ATOM   6869  O    VAL E 191      8.994 106.850  -7.054  1.00 49.00      B000 O
ATOM   6870  CB   VAL E 191      8.569 106.923 -10.062  1.00 39.27      B000 C
ATOM   6871  CG1  VAL E 191      9.802 107.734 -10.381  1.00 41.98      B000 C
ATOM   6872  CG2  VAL E 191      7.920 106.410 -11.351  1.00 37.38      B000 C
ATOM   6873  N    VAL E 192     10.885 105.796  -7.712  1.00 36.78      B000 N
ATOM   6874  CA   VAL E 192     11.606 106.006  -6.456  1.00 43.13      B000 C
ATOM   6875  C    VAL E 192     12.466 107.255  -6.601  1.00 46.90      B000 C
ATOM   6876  O    VAL E 192     13.429 107.274  -7.375  1.00 45.02      B000 O
ATOM   6877  CB   VAL E 192     12.459 104.793  -6.065  1.00 41.45      B000 C
ATOM   6878  CG1  VAL E 192     13.073 105.013  -4.679  1.00 35.87      B000 C
ATOM   6879  CG2  VAL E 192     11.618 103.512  -6.094  1.00 37.77      B000 C
ATOM   6880  N    THR E 193     12.117 108.311  -5.870  1.00 47.21      B000 N
ATOM   6881  CA   THR E 193     12.800 109.589  -6.012  1.00 54.54      B000 C
```

```
ATOM   6882  C    THR E 193    13.689 109.963  -4.828  1.00 53.96    B000 C
ATOM   6883  O    THR E 193    14.342 111.005  -4.886  1.00 57.35    B000 O
ATOM   6884  CB   THR E 193    11.775 110.701  -6.279  1.00 47.81    B000 C
ATOM   6885  OG1  THR E 193    10.889 110.829  -5.158  1.00 54.26    B000 O
ATOM   6886  CG2  THR E 193    10.954 110.359  -7.522  1.00 49.33    B000 C
ATOM   6887  N    SER E 194    13.765 109.142  -3.779  1.00 50.25    B000 N
ATOM   6888  CA   SER E 194    14.493 109.535  -2.577  1.00 47.10    B000 C
ATOM   6889  C    SER E 194    14.737 108.321  -1.701  1.00 54.45    B000 C
ATOM   6890  O    SER E 194    14.097 107.277  -1.857  1.00 55.35    B000 O
ATOM   6891  CB   SER E 194    13.727 110.596  -1.779  1.00 50.58    B000 C
ATOM   6892  OG   SER E 194    12.588 110.027  -1.152  1.00 52.89    B000 O
ATOM   6893  N    TRP E 195    15.656 108.488  -0.749  1.00 54.05    B000 N
ATOM   6894  CA   TRP E 195    15.954 107.418   0.196  1.00 53.90    B000 C
ATOM   6895  C    TRP E 195    14.742 107.048   1.047  1.00 56.80    B000 C
ATOM   6896  O    TRP E 195    14.512 105.862   1.323  1.00 53.39    B000 O
ATOM   6897  CB   TRP E 195    17.124 107.822   1.086  1.00 52.43    B000 C
ATOM   6898  CG   TRP E 195    18.407 107.331   0.552  1.00 62.39    B000 C
ATOM   6899  CD1  TRP E 195    19.444 108.092   0.068  1.00 63.42    B000 C
ATOM   6900  CD2  TRP E 195    18.804 105.965   0.402  1.00 65.61    B000 C
ATOM   6901  NE1  TRP E 195    20.464 107.278  -0.362  1.00 65.18    B000 N
ATOM   6902  CE2  TRP E 195    20.100 105.969  -0.170  1.00 69.72    B000 C
ATOM   6903  CE3  TRP E 195    18.199 104.739   0.703  1.00 59.71    B000 C
ATOM   6904  CZ2  TRP E 195    20.797 104.792  -0.447  1.00 59.79    B000 C
ATOM   6905  CZ3  TRP E 195    18.890 103.571   0.425  1.00 54.31    B000 C
ATOM   6906  CH2  TRP E 195    20.174 103.605  -0.142  1.00 59.74    B000 C
ATOM   6907  N    GLU E 196    13.961 108.038   1.487  1.00 55.10    B000 N
ATOM   6908  CA   GLU E 196    12.781 107.723   2.292  1.00 58.29    B000 C
ATOM   6909  C    GLU E 196    11.786 106.895   1.505  1.00 53.21    B000 C
ATOM   6910  O    GLU E 196    11.299 105.874   1.999  1.00 56.81    B000 O
ATOM   6911  CB   GLU E 196    12.127 108.987   2.849  1.00 67.43    B000 C
ATOM   6912  CG   GLU E 196    12.825 109.494   4.092  1.00 75.36    B000 C
ATOM   6913  CD   GLU E 196    14.139 110.132   3.758  1.00 90.49    B000 C
ATOM   6914  OE1  GLU E 196    14.259 110.606   2.610  1.00 89.70    B000 O
ATOM   6915  OE2  GLU E 196    15.055 110.128   4.613  1.00 94.74    B000 O1-
ATOM   6916  N    GLU E 197    11.460 107.326   0.281  1.00 51.77    B000 N
ATOM   6917  CA   GLU E 197    10.589 106.525  -0.570  1.00 52.50    B000 C
ATOM   6918  C    GLU E 197    11.155 105.116  -0.740  1.00 51.92    B000 C
ATOM   6919  O    GLU E 197    10.436 104.125  -0.567  1.00 48.95    B000 O
ATOM   6920  CB   GLU E 197    10.376 107.217  -1.920  1.00 44.93    B000 C
ATOM   6921  CG   GLU E 197     9.163 106.692  -2.685  1.00 46.53    B000 C
ATOM   6922  CD   GLU E 197     8.827 107.482  -3.949  1.00 48.66    B000 C
ATOM   6923  OE1  GLU E 197     9.651 108.313  -4.402  1.00 48.86    B000 O
ATOM   6924  OE2  GLU E 197     7.727 107.257  -4.507  1.00 50.40    B000 O1-
ATOM   6925  N    GLN E 198    12.462 105.006  -1.006  1.00 45.93    B000 N
ATOM   6926  CA   GLN E 198    13.088 103.692  -1.102  1.00 44.41    B000 C
ATOM   6927  C    GLN E 198    12.894 102.888   0.183  1.00 52.88    B000 C
ATOM   6928  O    GLN E 198    12.542 101.701   0.145  1.00 50.74    B000 O
ATOM   6929  CB   GLN E 198    14.572 103.831  -1.416  1.00 39.60    B000 C
ATOM   6930  CG   GLN E 198    15.367 102.576  -1.148  1.00 36.79    B000 C
ATOM   6931  CD   GLN E 198    15.210 101.541  -2.254  1.00 42.68    B000 C
ATOM   6932  OE1  GLN E 198    14.942 101.881  -3.399  1.00 38.01    B000 O
ATOM   6933  NE2  GLN E 198    15.359 100.273  -1.906  1.00 40.53    B000 N
ATOM   6934  N    LYS E 199    13.135 103.512   1.337  1.00 51.74    B000 N
ATOM   6935  CA   LYS E 199    13.004 102.770   2.587  1.00 50.49    B000 C
ATOM   6936  C    LYS E 199    11.544 102.452   2.890  1.00 51.58    B000 C
ATOM   6937  O    LYS E 199    11.242 101.379   3.427  1.00 48.97    B000 O
ATOM   6938  CB   LYS E 199    13.670 103.539   3.731  1.00 53.77    B000 C
ATOM   6939  CG   LYS E 199    15.157 103.755   3.500  1.00 55.44    B000 C
ATOM   6940  CD   LYS E 199    15.992 103.547   4.755  1.00 75.31    B000 C
ATOM   6941  CE   LYS E 199    17.390 104.165   4.583  1.00 85.03    B000 C
ATOM   6942  NZ   LYS E 199    18.151 104.301   5.862  1.00 83.45    B000 N1+
ATOM   6943  N    PHE E 200    10.631 103.358   2.531  1.00 48.81    B000 N
ATOM   6944  CA   PHE E 200     9.204 103.098   2.691  1.00 48.98    B000 C
ATOM   6945  C    PHE E 200     8.773 101.845   1.931  1.00 55.28    B000 C
ATOM   6946  O    PHE E 200     8.032 101.010   2.465  1.00 54.84    B000 O
ATOM   6947  CB   PHE E 200     8.406 104.312   2.224  1.00 46.91    B000 C
ATOM   6948  CG   PHE E 200     6.965 104.034   2.006  1.00 50.71    B000 C
ATOM   6949  CD1  PHE E 200     6.112 103.847   3.085  1.00 53.71    B000 C
ATOM   6950  CD2  PHE E 200     6.449 103.965   0.722  1.00 51.62    B000 C
ATOM   6951  CE1  PHE E 200     4.756 103.588   2.891  1.00 52.00    B000 C
ATOM   6952  CE2  PHE E 200     5.097 103.710   0.512  1.00 50.54    B000 C
```

```
ATOM   6953  CZ  PHE E 200      4.248 103.518   1.605  1.00 54.40      B000 C
ATOM   6954  N   VAL E 201      9.229 101.697   0.680  1.00 51.49      B000 N
ATOM   6955  CA  VAL E 201      8.826 100.553  -0.137  1.00 48.07      B000 C
ATOM   6956  C   VAL E 201      9.420  99.254   0.413  1.00 49.06      B000 C
ATOM   6957  O   VAL E 201      8.706  98.253   0.569  1.00 46.98      B000 O
ATOM   6958  CB  VAL E 201      9.208 100.788  -1.611  1.00 46.66      B000 C
ATOM   6959  CG1 VAL E 201      9.067  99.495  -2.434  1.00 41.41      B000 C
ATOM   6960  CG2 VAL E 201      8.349 101.902  -2.212  1.00 37.70      B000 C
ATOM   6961  N   GLN E 202     10.728  99.252   0.723  1.00 49.32      B000 N
ATOM   6962  CA  GLN E 202     11.380  98.063   1.284  1.00 50.74      B000 C
ATOM   6963  C   GLN E 202     10.622  97.528   2.481  1.00 51.55      B000 C
ATOM   6964  O   GLN E 202     10.464  96.310   2.640  1.00 56.45      B000 O
ATOM   6965  CB  GLN E 202     12.803  98.371   1.749  1.00 47.47      B000 C
ATOM   6966  CG  GLN E 202     13.739  98.831   0.710  1.00 55.69      B000 C
ATOM   6967  CD  GLN E 202     15.139  98.987   1.254  1.00 56.47      B000 C
ATOM   6968  OE1 GLN E 202     15.907  99.831   0.790  1.00 57.62      B000 O
ATOM   6969  NE2 GLN E 202     15.490  98.154   2.219  1.00 53.11      B000 N
ATOM   6970  N   HIS E 203     10.189  98.433   3.360  1.00 51.67      B000 N
ATOM   6971  CA  HIS E 203      9.452  98.030   4.546  1.00 53.23      B000 C
ATOM   6972  C   HIS E 203      8.230  97.215   4.173  1.00 58.75      B000 C
ATOM   6973  O   HIS E 203      7.901  96.227   4.840  1.00 59.40      B000 O
ATOM   6974  CB  HIS E 203      9.043  99.256   5.346  1.00 52.17      B000 C
ATOM   6975  CG  HIS E 203      8.280  98.924   6.587  1.00 63.88      B000 C
ATOM   6976  ND1 HIS E 203      6.924  99.142   6.710  1.00 66.92      B000 N
ATOM   6977  CD2 HIS E 203      8.681  98.359   7.750  1.00 59.72      B000 C
ATOM   6978  CE1 HIS E 203      6.525  98.741   7.904  1.00 64.58      B000 C
ATOM   6979  NE2 HIS E 203      7.572  98.265   8.555  1.00 67.38      B000 N
ATOM   6980  N   HIS E 204      7.548  97.609   3.105  1.00 51.87      B000 N
ATOM   6981  CA  HIS E 204      6.320  96.930   2.742  1.00 47.42      B000 C
ATOM   6982  C   HIS E 204      6.528  95.712   1.851  1.00 45.87      B000 C
ATOM   6983  O   HIS E 204      5.734  94.781   1.934  1.00 46.13      B000 O
ATOM   6984  CB  HIS E 204      5.375  97.925   2.086  1.00 43.64      B000 C
ATOM   6985  CG  HIS E 204      4.761  98.864   3.071  1.00 55.52      B000 C
ATOM   6986  ND1 HIS E 204      5.277 100.116   3.331  1.00 62.43      B000 N
ATOM   6987  CD2 HIS E 204      3.719  98.703   3.917  1.00 51.63      B000 C
ATOM   6988  CE1 HIS E 204      4.551 100.702   4.265  1.00 56.76      B000 C
ATOM   6989  NE2 HIS E 204      3.598  99.867   4.634  1.00 58.19      B000 N
ATOM   6990  N   ILE E 205      7.554  95.673   0.998  1.00 45.67      B000 N
ATOM   6991  CA  ILE E 205      7.676  94.532   0.097  1.00 43.17      B000 C
ATOM   6992  C   ILE E 205      8.408  93.363   0.749  1.00 43.69      B000 C
ATOM   6993  O   ILE E 205      8.193  92.215   0.356  1.00 42.25      B000 O
ATOM   6994  CB  ILE E 205      8.346  94.913  -1.235  1.00 39.71      B000 C
ATOM   6995  CG1 ILE E 205      9.791  95.341  -1.023  1.00 34.62      B000 C
ATOM   6996  CG2 ILE E 205      7.572  96.011  -1.954  1.00 32.37      B000 C
ATOM   6997  CD1 ILE E 205     10.470  95.665  -2.317  1.00 33.57      B000 C
ATOM   6998  N   GLY E 206      9.273  93.616   1.728  1.00 45.56      B000 N
ATOM   6999  CA  GLY E 206     10.038  92.555   2.352  1.00 38.77      B000 C
ATOM   7000  C   GLY E 206     11.058  91.943   1.409  1.00 45.53      B000 C
ATOM   7001  O   GLY E 206     11.640  92.616   0.551  1.00 53.68      B000 O
ATOM   7002  N   PRO E 207     11.268  90.646   1.525  1.00 41.49      B000 N
ATOM   7003  CA  PRO E 207     12.312  89.995   0.720  1.00 48.79      B000 C
ATOM   7004  C   PRO E 207     11.816  89.452  -0.616  1.00 39.32      B000 C
ATOM   7005  O   PRO E 207     12.233  88.359  -0.999  1.00 49.37      B000 O
ATOM   7006  CB  PRO E 207     12.736  88.837   1.624  1.00 42.37      B000 C
ATOM   7007  CG  PRO E 207     11.386  88.411   2.205  1.00 39.72      B000 C
ATOM   7008  CD  PRO E 207     10.597  89.687   2.422  1.00 38.72      B000 C
ATOM   7009  N   VAL E 208     10.949  90.169  -1.333  1.00 40.46      B000 N
ATOM   7010  CA  VAL E 208     10.315  89.659  -2.546  1.00 37.68      B000 C
ATOM   7011  C   VAL E 208     10.706  90.542  -3.729  1.00 38.16      B000 C
ATOM   7012  O   VAL E 208     10.561  91.767  -3.665  1.00 39.77      B000 O
ATOM   7013  CB  VAL E 208      8.785  89.601  -2.383  1.00 40.03      B000 C
ATOM   7014  CG1 VAL E 208      8.133  88.976  -3.608  1.00 32.03      B000 C
ATOM   7015  CG2 VAL E 208      8.427  88.819  -1.106  1.00 41.62      B000 C
ATOM   7016  N   ASN E 209     11.186  89.917  -4.812  1.00 35.17      B000 N
ATOM   7017  CA  ASN E 209     11.506  90.657  -6.028  1.00 30.25      B000 C
ATOM   7018  C   ASN E 209     10.268  91.389  -6.522  1.00 39.60      B000 C
ATOM   7019  O   ASN E 209      9.189  90.792  -6.646  1.00 35.43      B000 O
ATOM   7020  CB  ASN E 209     12.020  89.710  -7.105  1.00 33.04      B000 C
ATOM   7021  CG  ASN E 209     13.430  89.196  -6.817  1.00 37.45      B000 C
ATOM   7022  OD1 ASN E 209     14.282  89.914  -6.286  1.00 39.88      B000 O
ATOM   7023  ND2 ASN E 209     13.676  87.948  -7.166  1.00 34.14      B000 N
```

```
ATOM   7024  N    THR E 210     10.417  92.686  -6.801  1.00 32.18      B000 N
ATOM   7025  CA   THR E 210      9.258  93.553  -6.998  1.00 34.27      B000 C
ATOM   7026  C    THR E 210      9.571  94.589  -8.068  1.00 34.48      B000 C
ATOM   7027  O    THR E 210     10.533  95.349  -7.932  1.00 31.68      B000 O
ATOM   7028  CB   THR E 210      8.864  94.225  -5.667  1.00 33.37      B000 C
ATOM   7029  OG1  THR E 210      8.661  93.210  -4.675  1.00 35.38      B000 O
ATOM   7030  CG2  THR E 210      7.604  95.081  -5.789  1.00 26.61      B000 C
ATOM   7031  N    TRP E 211      8.743  94.626  -9.112  1.00 29.97      B000 N
ATOM   7032  CA   TRP E 211      8.933  95.562 -10.208  1.00 27.14      B000 C
ATOM   7033  C    TRP E 211      8.801  97.006  -9.731  1.00 35.31      B000 C
ATOM   7034  O    TRP E 211      7.981  97.316  -8.863  1.00 35.67      B000 O
ATOM   7035  CB   TRP E 211      7.887  95.312 -11.297  1.00 30.90      B000 C
ATOM   7036  CG   TRP E 211      8.066  94.071 -12.124  1.00 34.50      B000 C
ATOM   7037  CD1  TRP E 211      7.104  93.138 -12.433  1.00 29.56      B000 C
ATOM   7038  CD2  TRP E 211      9.267  93.640 -12.788  1.00 34.63      B000 C
ATOM   7039  NE1  TRP E 211      7.630  92.160 -13.254  1.00 28.60      B000 N
ATOM   7040  CE2  TRP E 211      8.955  92.439 -13.482  1.00 35.28      B000 C
ATOM   7041  CE3  TRP E 211     10.575  94.146 -12.860  1.00 35.02      B000 C
ATOM   7042  CZ2  TRP E 211      9.904  91.741 -14.231  1.00 29.26      B000 C
ATOM   7043  CZ3  TRP E 211     11.518  93.452 -13.602  1.00 36.35      B000 C
ATOM   7044  CH2  TRP E 211     11.177  92.251 -14.275  1.00 30.95      B000 C
ATOM   7045  N    MET E 212      9.605  97.895 -10.316  1.00 32.55      B000 N
ATOM   7046  CA   MET E 212      9.380  99.331 -10.239  1.00 29.40      B000 C
ATOM   7047  C    MET E 212      9.191  99.869 -11.652  1.00 37.49      B000 C
ATOM   7048  O    MET E 212      9.421  99.163 -12.641  1.00 33.69      B000 O
ATOM   7049  CB   MET E 212     10.532 100.045  -9.538  1.00 32.77      B000 C
ATOM   7050  CG   MET E 212     11.802 100.197 -10.374  1.00 37.02      B000 C
ATOM   7051  SD   MET E 212     13.201 100.683  -9.319  1.00 36.83      B000 S
ATOM   7052  CE   MET E 212     13.620  99.156  -8.467  1.00 32.94      B000 C
ATOM   7053  N    GLY E 213      8.730 101.124 -11.741  1.00 37.19      B000 N
ATOM   7054  CA   GLY E 213      8.440 101.761 -13.019  1.00 32.81      B000 C
ATOM   7055  C    GLY E 213      9.662 102.269 -13.761  1.00 40.32      B000 C
ATOM   7056  O    GLY E 213      9.719 103.432 -14.187  1.00 38.40      B000 O
ATOM   7057  N    LEU E 214     10.634 101.380 -13.941  1.00 34.17      B000 N
ATOM   7058  CA   LEU E 214     11.934 101.714 -14.503  1.00 39.27      B000 C
ATOM   7059  C    LEU E 214     12.321 100.610 -15.473  1.00 39.76      B000 C
ATOM   7060  O    LEU E 214     12.335  99.431 -15.100  1.00 41.29      B000 O
ATOM   7061  CB   LEU E 214     12.988 101.877 -13.395  1.00 36.55      B000 C
ATOM   7062  CG   LEU E 214     14.450 102.064 -13.797  1.00 42.98      B000 C
ATOM   7063  CD1  LEU E 214     14.624 103.269 -14.744  1.00 38.04      B000 C
ATOM   7064  CD2  LEU E 214     15.305 102.224 -12.529  1.00 37.75      B000 C
ATOM   7065  N    HIS E 215     12.601 100.987 -16.718  1.00 36.19      B000 N
ATOM   7066  CA   HIS E 215     12.875 100.024 -17.774  1.00 39.33      B000 C
ATOM   7067  C    HIS E 215     13.746 100.691 -18.829  1.00 41.39      B000 C
ATOM   7068  O    HIS E 215     13.843 101.921 -18.899  1.00 36.50      B000 O
ATOM   7069  CB   HIS E 215     11.588  99.528 -18.420  1.00 37.31      B000 C
ATOM   7070  CG   HIS E 215     10.877 100.605 -19.156  1.00 43.54      B000 C
ATOM   7071  ND1  HIS E 215     10.975 100.759 -20.521  1.00 59.96      B000 N
ATOM   7072  CD2  HIS E 215     10.149 101.654 -18.708  1.00 43.07      B000 C
ATOM   7073  CE1  HIS E 215     10.292 101.830 -20.888  1.00 55.73      B000 C
ATOM   7074  NE2  HIS E 215      9.780 102.387 -19.807  1.00 46.66      B000 N
ATOM   7075  N    ASP E 216     14.341  99.858 -19.680  1.00 35.15      B000 N
ATOM   7076  CA   ASP E 216     15.301 100.290 -20.686  1.00 39.15      B000 C
ATOM   7077  C    ASP E 216     14.788  99.998 -22.085  1.00 42.63      B000 C
ATOM   7078  O    ASP E 216     15.571  99.773 -23.004  1.00 42.03      B000 O
ATOM   7079  CB   ASP E 216     16.648  99.600 -20.467  1.00 41.75      B000 C
ATOM   7080  CG   ASP E 216     16.632  98.097 -20.849  1.00 44.42      B000 C
ATOM   7081  OD1  ASP E 216     15.551  97.505 -21.130  1.00 37.16      B000 O
ATOM   7082  OD2  ASP E 216     17.732  97.502 -20.848  1.00 43.61      B000 O1-
ATOM   7083  N    GLN E 217     13.476  99.947 -22.253  1.00 46.29      B000 N
ATOM   7084  CA   GLN E 217     12.991  99.214 -23.411  1.00 59.57      B000 C
ATOM   7085  C    GLN E 217     13.208  99.957 -24.721  1.00 65.22      B000 C
ATOM   7086  O    GLN E 217     13.083  99.331 -25.778  1.00 73.01      B000 O
ATOM   7087  CB   GLN E 217     11.516  98.820 -23.200  1.00 62.75      B000 C
ATOM   7088  CG   GLN E 217     11.375  97.683 -22.125  1.00 61.06      B000 C
ATOM   7089  CD   GLN E 217      9.984  97.034 -22.070  1.00 67.45      B000 C
ATOM   7090  OE1  GLN E 217      9.134  97.409 -21.253  1.00 58.29      B000 O
ATOM   7091  NE2  GLN E 217      9.759  96.044 -22.936  1.00 73.72      B000 N
ATOM   7092  N    ASN E 218     13.622 101.226 -24.688  1.00 54.85      B000 N
ATOM   7093  CA   ASN E 218     14.065 101.896 -25.900  1.00 56.88      B000 C
ATOM   7094  C    ASN E 218     15.579 101.999 -25.997  1.00 63.85      B000 C
```

```
ATOM   7095  O    ASN E 218      16.099 102.476 -27.010  1.00 65.96      B000 O
ATOM   7096  CB   ASN E 218      13.440 103.283 -25.982  1.00 68.65      B000 C
ATOM   7097  CG   ASN E 218      11.988 103.230 -26.379  1.00 76.18      B000 C
ATOM   7098  OD1  ASN E 218      11.117 103.732 -25.663  1.00 80.28      B000 O
ATOM   7099  ND2  ASN E 218      11.710 102.603 -27.524  1.00 70.77      B000 N
ATOM   7100  N    GLY E 219      16.294 101.546 -24.980  1.00 55.99      B000 N
ATOM   7101  CA   GLY E 219      17.721 101.702 -24.908  1.00 47.24      B000 C
ATOM   7102  C    GLY E 219      18.086 102.395 -23.615  1.00 46.37      B000 C
ATOM   7103  O    GLY E 219      18.657 101.797 -22.697  1.00 45.37      B000 O
ATOM   7104  N    PRO E 220      17.760 103.679 -23.515  1.00 47.23      B000 N
ATOM   7105  CA   PRO E 220      18.027 104.396 -22.266  1.00 48.53      B000 C
ATOM   7106  C    PRO E 220      17.055 103.993 -21.169  1.00 44.15      B000 C
ATOM   7107  O    PRO E 220      15.875 103.736 -21.413  1.00 47.26      B000 O
ATOM   7108  CB   PRO E 220      17.875 105.872 -22.658  1.00 43.34      B000 C
ATOM   7109  CG   PRO E 220      17.055 105.868 -23.864  1.00 50.66      B000 C
ATOM   7110  CD   PRO E 220      17.359 104.593 -24.598  1.00 46.49      B000 C
ATOM   7111  N    TRP E 221      17.592 103.878 -19.961  1.00 41.42      B000 N
ATOM   7112  CA   TRP E 221      16.767 103.695 -18.783  1.00 38.08      B000 C
ATOM   7113  C    TRP E 221      15.871 104.916 -18.561  1.00 43.21      B000 C
ATOM   7114  O    TRP E 221      16.325 106.062 -18.630  1.00 45.08      B000 O
ATOM   7115  CB   TRP E 221      17.672 103.446 -17.577  1.00 37.90      B000 C
ATOM   7116  CG   TRP E 221      18.256 102.057 -17.559  1.00 35.77      B000 C
ATOM   7117  CD1  TRP E 221      19.520 101.683 -17.939  1.00 39.29      B000 C
ATOM   7118  CD2  TRP E 221      17.586 100.853 -17.157  1.00 36.68      B000 C
ATOM   7119  NE1  TRP E 221      19.676 100.323 -17.789  1.00 39.97      B000 N
ATOM   7120  CE2  TRP E 221      18.501  99.790 -17.318  1.00 39.47      B000 C
ATOM   7121  CE3  TRP E 221      16.296 100.571 -16.676  1.00 37.85      B000 C
ATOM   7122  CZ2  TRP E 221      18.176  98.469 -16.997  1.00 34.61      B000 C
ATOM   7123  CZ3  TRP E 221      15.970  99.246 -16.364  1.00 38.91      B000 C
ATOM   7124  CH2  TRP E 221      16.909  98.220 -16.523  1.00 36.77      B000 C
ATOM   7125  N    LYS E 222      14.591 104.666 -18.298  1.00 42.42      B000 N
ATOM   7126  CA   LYS E 222      13.600 105.715 -18.110  1.00 45.91      B000 C
ATOM   7127  C    LYS E 222      12.648 105.317 -16.994  1.00 49.90      B000 C
ATOM   7128  O    LYS E 222      12.383 104.131 -16.772  1.00 45.80      B000 O
ATOM   7129  CB   LYS E 222      12.766 105.968 -19.368  1.00 48.31      B000 C
ATOM   7130  CG   LYS E 222      13.519 106.492 -20.561  1.00 57.72      B000 C
ATOM   7131  CD   LYS E 222      12.640 106.357 -21.800  1.00 65.32      B000 C
ATOM   7132  CE   LYS E 222      12.661 104.903 -22.305  1.00 75.88      B000 C
ATOM   7133  NZ   LYS E 222      12.007 104.696 -23.635  1.00 76.97      B000 N1+
ATOM   7134  N    TRP E 223      12.133 106.321 -16.298  1.00 47.14      B000 N
ATOM   7135  CA   TRP E 223      11.031 106.134 -15.372  1.00 41.03      B000 C
ATOM   7136  C    TRP E 223       9.714 106.314 -16.124  1.00 47.51      B000 C
ATOM   7137  O    TRP E 223       9.617 107.117 -17.056  1.00 50.81      B000 O
ATOM   7138  CB   TRP E 223      11.115 107.123 -14.218  1.00 39.77      B000 C
ATOM   7139  CG   TRP E 223      12.293 106.912 -13.328  1.00 40.93      B000 C
ATOM   7140  CD1  TRP E 223      13.467 107.621 -13.338  1.00 41.70      B000 C
ATOM   7141  CD2  TRP E 223      12.410 105.957 -12.270  1.00 41.60      B000 C
ATOM   7142  NE1  TRP E 223      14.311 107.148 -12.366  1.00 40.09      B000 N
ATOM   7143  CE2  TRP E 223      13.688 106.129 -11.692  1.00 39.35      B000 C
ATOM   7144  CE3  TRP E 223      11.561 104.962 -11.758  1.00 42.49      B000 C
ATOM   7145  CZ2  TRP E 223      14.141 105.342 -10.627  1.00 38.41      B000 C
ATOM   7146  CZ3  TRP E 223      12.010 104.183 -10.698  1.00 39.66      B000 C
ATOM   7147  CH2  TRP E 223      13.291 104.374 -10.147  1.00 37.68      B000 C
ATOM   7148  N    VAL E 224       8.693 105.555 -15.715  1.00 38.47      B000 N
ATOM   7149  CA   VAL E 224       7.441 105.568 -16.467  1.00 44.59      B000 C
ATOM   7150  C    VAL E 224       6.732 106.924 -16.423  1.00 46.85      B000 C
ATOM   7151  O    VAL E 224       5.908 107.197 -17.298  1.00 50.06      B000 O
ATOM   7152  CB   VAL E 224       6.487 104.458 -15.967  1.00 42.40      B000 C
ATOM   7153  CG1  VAL E 224       7.059 103.070 -16.288  1.00 42.00      B000 C
ATOM   7154  CG2  VAL E 224       6.227 104.600 -14.475  1.00 34.29      B000 C
ATOM   7155  N    ASP E 225       7.017 107.790 -15.444  1.00 48.54      B000 N
ATOM   7156  CA   ASP E 225       6.329 109.079 -15.338  1.00 53.13      B000 C
ATOM   7157  C    ASP E 225       7.149 110.250 -15.865  1.00 51.10      B000 C
ATOM   7158  O    ASP E 225       6.709 111.394 -15.749  1.00 47.62      B000 O
ATOM   7159  CB   ASP E 225       5.898 109.358 -13.886  1.00 39.25      B000 C
ATOM   7160  CG   ASP E 225       7.078 109.617 -12.948  1.00 49.56      B000 C
ATOM   7161  OD1  ASP E 225       8.244 109.417 -13.352  1.00 47.55      B000 O
ATOM   7162  OD2  ASP E 225       6.834 109.961 -11.767  1.00 55.28      B000 O1-
ATOM   7163  N    GLY E 226       8.319 110.001 -16.449  1.00 50.70      B000 N
ATOM   7164  CA   GLY E 226       9.118 111.058 -17.029  1.00 50.75      B000 C
ATOM   7165  C    GLY E 226      10.206 111.608 -16.128  1.00 49.85      B000 C
```

```
ATOM   7166  O    GLY E 226     11.083 112.321 -16.614  1.00 55.13      B000 O
ATOM   7167  N    THR E 227     10.149 111.328 -14.832  1.00 46.51      B000 N
ATOM   7168  CA   THR E 227     11.258 111.622 -13.942  1.00 42.53      B000 C
ATOM   7169  C    THR E 227     12.579 111.301 -14.624  1.00 50.97      B000 C
ATOM   7170  O    THR E 227     12.760 110.213 -15.178  1.00 57.64      B000 O
ATOM   7171  CB   THR E 227     11.117 110.795 -12.667  1.00 48.55      B000 C
ATOM   7172  OG1  THR E 227      9.834 111.037 -12.070  1.00 46.24      B000 O
ATOM   7173  CG2  THR E 227     12.226 111.120 -11.690  1.00 41.66      B000 C
ATOM   7174  N    ASP E 228     13.490 112.268 -14.601  1.00 59.03      B000 N
ATOM   7175  CA   ASP E 228     14.770 112.131 -15.280  1.00 45.94      B000 C
ATOM   7176  C    ASP E 228     15.624 111.088 -14.580  1.00 51.75      B000 C
ATOM   7177  O    ASP E 228     15.872 111.182 -13.374  1.00 56.28      B000 O
ATOM   7178  CB   ASP E 228     15.501 113.474 -15.311  1.00 47.30      B000 C
ATOM   7179  CG   ASP E 228     16.845 113.379 -15.998  1.00 56.00      B000 C
ATOM   7180  OD1  ASP E 228     16.859 113.296 -17.250  1.00 54.17      B000 O
ATOM   7181  OD2  ASP E 228     17.881 113.348 -15.290  1.00 56.42      B000 O1-
ATOM   7182  N    TYR E 229     16.112 110.114 -15.350  1.00 52.14      B000 N
ATOM   7183  CA   TYR E 229     16.829 109.000 -14.743  1.00 46.06      B000 C
ATOM   7184  C    TYR E 229     18.227 109.400 -14.287  1.00 47.79      B000 C
ATOM   7185  O    TYR E 229     18.667 108.998 -13.201  1.00 47.55      B000 O
ATOM   7186  CB   TYR E 229     16.901 107.830 -15.729  1.00 43.66      B000 C
ATOM   7187  CG   TYR E 229     17.852 106.745 -15.304  1.00 42.09      B000 C
ATOM   7188  CD1  TYR E 229     17.523 105.882 -14.269  1.00 38.14      B000 C
ATOM   7189  CD2  TYR E 229     19.090 106.594 -15.917  1.00 42.41      B000 C
ATOM   7190  CE1  TYR E 229     18.387 104.882 -13.864  1.00 40.35      B000 C
ATOM   7191  CE2  TYR E 229     19.968 105.589 -15.517  1.00 46.35      B000 C
ATOM   7192  CZ   TYR E 229     19.599 104.734 -14.486  1.00 46.45      B000 C
ATOM   7193  OH   TYR E 229     20.450 103.738 -14.064  1.00 40.57      B000 O
ATOM   7194  N    GLU E 230     18.954 110.170 -15.103  1.00 49.15      B000 N
ATOM   7195  CA   GLU E 230     20.375 110.365 -14.821  1.00 46.97      B000 C
ATOM   7196  C    GLU E 230     20.591 111.198 -13.556  1.00 51.12      B000 C
ATOM   7197  O    GLU E 230     21.457 110.879 -12.738  1.00 53.36      B000 O
ATOM   7198  CB   GLU E 230     21.082 110.992 -16.017  1.00 50.20      B000 C
ATOM   7199  CG   GLU E 230     22.607 110.803 -15.963  1.00 63.82      B000 C
ATOM   7200  CD   GLU E 230     23.021 109.363 -15.585  1.00 76.02      B000 C
ATOM   7201  OE1  GLU E 230     22.553 108.396 -16.253  1.00 66.88      B000 O
ATOM   7202  OE2  GLU E 230     23.821 109.201 -14.625  1.00 79.76      B000 O1-
ATOM   7203  N    THR E 231     19.809 112.248 -13.359  1.00 48.10      B000 N
ATOM   7204  CA   THR E 231     19.981 113.081 -12.176  1.00 56.05      B000 C
ATOM   7205  C    THR E 231     19.161 112.602 -10.988  1.00 60.28      B000 C
ATOM   7206  O    THR E 231     19.280 113.183  -9.900  1.00 51.86      B000 O
ATOM   7207  CB   THR E 231     19.606 114.531 -12.495  1.00 52.22      B000 C
ATOM   7208  OG1  THR E 231     18.217 114.591 -12.844  1.00 53.83      B000 O
ATOM   7209  CG2  THR E 231     20.445 115.043 -13.668  1.00 46.56      B000 C
ATOM   7210  N    GLY E 232     18.363 111.540 -11.160  1.00 55.84      B000 N
ATOM   7211  CA   GLY E 232     17.470 111.077 -10.123  1.00 48.35      B000 C
ATOM   7212  C    GLY E 232     18.105 110.067  -9.184  1.00 48.84      B000 C
ATOM   7213  O    GLY E 232     19.249 109.642  -9.345  1.00 44.82      B000 O
ATOM   7214  N    PHE E 233     17.313 109.679  -8.185  1.00 47.45      B000 N
ATOM   7215  CA   PHE E 233     17.724 108.672  -7.218  1.00 45.48      B000 C
ATOM   7216  C    PHE E 233     17.998 107.339  -7.920  1.00 45.39      B000 C
ATOM   7217  O    PHE E 233     17.309 106.963  -8.870  1.00 43.33      B000 O
ATOM   7218  CB   PHE E 233     16.627 108.531  -6.141  1.00 43.07      B000 C
ATOM   7219  CG   PHE E 233     16.952 107.537  -5.062  1.00 49.50      B000 C
ATOM   7220  CD1  PHE E 233     17.819 107.865  -4.031  1.00 49.13      B000 C
ATOM   7221  CD2  PHE E 233     16.393 106.267  -5.077  1.00 46.36      B000 C
ATOM   7222  CE1  PHE E 233     18.130 106.941  -3.043  1.00 56.48      B000 C
ATOM   7223  CE2  PHE E 233     16.703 105.332  -4.089  1.00 43.16      B000 C
ATOM   7224  CZ   PHE E 233     17.570 105.668  -3.073  1.00 50.08      B000 C
ATOM   7225  N    LYS E 234     19.027 106.631  -7.461  1.00 43.29      B000 N
ATOM   7226  CA   LYS E 234     19.350 105.304  -7.964  1.00 42.55      B000 C
ATOM   7227  C    LYS E 234     19.835 104.464  -6.800  1.00 48.47      B000 C
ATOM   7228  O    LYS E 234     20.501 104.989  -5.904  1.00 44.87      B000 O
ATOM   7229  CB   LYS E 234     20.433 105.329  -9.047  1.00 43.84      B000 C
ATOM   7230  CG   LYS E 234     20.093 106.096 -10.319  1.00 43.53      B000 C
ATOM   7231  CD   LYS E 234     21.305 106.028 -11.250  1.00 42.53      B000 C
ATOM   7232  CE   LYS E 234     21.235 107.016 -12.398  1.00 46.96      B000 C
ATOM   7233  NZ   LYS E 234     21.273 108.424 -11.907  1.00 55.21      B000 N1+
ATOM   7234  N    ASN E 235     19.486 103.171  -6.793  1.00 46.39      B000 N
ATOM   7235  CA   ASN E 235     19.925 102.276  -5.717  1.00 42.44      B000 C
ATOM   7236  C    ASN E 235     20.263 100.885  -6.277  1.00 41.99      B000 C
```

370

```
ATOM   7237  O    ASN E 235      19.881  99.849  -5.728  1.00 41.55      B000 O
ATOM   7238  CB   ASN E 235      18.867 102.211  -4.605  1.00 37.90      B000 C
ATOM   7239  CG   ASN E 235      19.353 101.448  -3.380  1.00 40.64      B000 C
ATOM   7240  OD1  ASN E 235      20.538 101.458  -3.079  1.00 45.11      B000 N
ATOM   7241  ND2  ASN E 235      18.449 100.769  -2.685  1.00 41.74      B000 N
ATOM   7242  N    TRP E 236      21.051 100.853  -7.346  1.00 39.52      B000 N
ATOM   7243  CA   TRP E 236      21.407  99.599  -7.994  1.00 37.91      B000 C
ATOM   7244  C    TRP E 236      22.266  98.720  -7.099  1.00 42.70      B000 C
ATOM   7245  O    TRP E 236      23.054  99.199  -6.281  1.00 46.72      B000 O
ATOM   7246  CB   TRP E 236      22.178  99.848  -9.299  1.00 34.60      B000 C
ATOM   7247  CG   TRP E 236      21.377 100.494 -10.396  1.00 43.16      B000 C
ATOM   7248  CD1  TRP E 236      21.452 101.794 -10.810  1.00 36.82      B000 C
ATOM   7249  CD2  TRP E 236      20.363  99.873 -11.207  1.00 38.15      B000 C
ATOM   7250  NE1  TRP E 236      20.565 102.017 -11.839  1.00 41.26      B000 N
ATOM   7251  CE2  TRP E 236      19.884 100.857 -12.103  1.00 39.75      B000 C
ATOM   7252  CE3  TRP E 236      19.831  98.581 -11.273  1.00 36.10      B000 C
ATOM   7253  CZ2  TRP E 236      18.894 100.591 -13.054  1.00 35.54      B000 C
ATOM   7254  CZ3  TRP E 236      18.850  98.311 -12.219  1.00 35.95      B000 C
ATOM   7255  CH2  TRP E 236      18.389  99.317 -13.096  1.00 41.76      B000 C
ATOM   7256  N    ARG E 237      22.102  97.410  -7.274  1.00 45.40      B000 N
ATOM   7257  CA   ARG E 237      23.050  96.440  -6.763  1.00 47.18      B000 C
ATOM   7258  C    ARG E 237      24.449  96.720  -7.314  1.00 51.81      B000 C
ATOM   7259  O    ARG E 237      24.595  97.350  -8.368  1.00 43.69      B000 O
ATOM   7260  CB   ARG E 237      22.634  95.041  -7.185  1.00 46.24      B000 C
ATOM   7261  CG   ARG E 237      21.530  94.422  -6.393  1.00 51.40      B000 C
ATOM   7262  CD   ARG E 237      22.096  93.585  -5.285  1.00 58.45      B000 C
ATOM   7263  NE   ARG E 237      21.038  92.772  -4.710  1.00 70.66      B000 N
ATOM   7264  CZ   ARG E 237      21.262  91.703  -3.961  1.00 72.34      B000 C
ATOM   7265  NH1  ARG E 237      20.232  90.990  -3.494  1.00 63.81      B000 N1+
ATOM   7266  NH2  ARG E 237      22.525  91.368  -3.681  1.00 60.30      B000 N
ATOM   7267  N    PRO E 238      25.489  96.240  -6.628  1.00 52.18      B000 N
ATOM   7268  CA   PRO E 238      26.853  96.359  -7.163  1.00 56.03      B000 C
ATOM   7269  C    PRO E 238      26.950  95.788  -8.569  1.00 52.87      B000 C
ATOM   7270  O    PRO E 238      26.528  94.660  -8.830  1.00 56.32      B000 O
ATOM   7271  CB   PRO E 238      27.694  95.545  -6.171  1.00 52.68      B000 C
ATOM   7272  CG   PRO E 238      26.934  95.614  -4.891  1.00 56.74      B000 C
ATOM   7273  CD   PRO E 238      25.473  95.617  -5.291  1.00 58.40      B000 C
ATOM   7274  N    GLU E 239      27.508  96.588  -9.477  1.00 54.34      B000 N
ATOM   7275  CA   GLU E 239      27.753  96.254 -10.877  1.00 53.49      B000 C
ATOM   7276  C    GLU E 239      26.481  96.144 -11.711  1.00 55.15      B000 C
ATOM   7277  O    GLU E 239      26.545  95.684 -12.862  1.00 57.25      B000 O
ATOM   7278  CB   GLU E 239      28.578  94.974 -11.029  1.00 50.60      B000 C
ATOM   7279  CG   GLU E 239      30.001  95.104 -10.514  1.00 66.65      B000 C
ATOM   7280  CD   GLU E 239      30.663  93.760 -10.297  1.00 83.88      B000 C
ATOM   7281  OE1  GLU E 239      30.038  92.728 -10.639  1.00 75.71      B000 O
ATOM   7282  OE2  GLU E 239      31.816  93.739  -9.808  1.00 96.45      B000 O1-
ATOM   7283  N    GLN E 240      25.336  96.571 -11.187  1.00 42.77      B000 N
ATOM   7284  CA   GLN E 240      24.126  96.666 -11.990  1.00 44.13      B000 C
ATOM   7285  C    GLN E 240      23.886  98.142 -12.320  1.00 39.19      B000 C
ATOM   7286  O    GLN E 240      24.353  99.013 -11.600  1.00 37.74      B000 O
ATOM   7287  CB   GLN E 240      22.931  96.054 -11.253  1.00 41.92      B000 C
ATOM   7288  CG   GLN E 240      23.174  94.638 -10.737  1.00 41.56      B000 C
ATOM   7289  CD   GLN E 240      23.857  93.758 -11.761  1.00 47.34      B000 C
ATOM   7290  OE1  GLN E 240      23.461  93.713 -12.927  1.00 45.90      B000 O
ATOM   7291  NE2  GLN E 240      24.908  93.071 -11.336  1.00 53.15      B000 N
ATOM   7292  N    PRO E 241      23.176  98.433 -13.423  1.00 39.17      B000 N
ATOM   7293  CA   PRO E 241      22.582  97.492 -14.382  1.00 41.50      B000 C
ATOM   7294  C    PRO E 241      23.654  96.945 -15.327  1.00 39.88      B000 C
ATOM   7295  O    PRO E 241      24.800  97.332 -15.130  1.00 47.11      B000 O
ATOM   7296  CB   PRO E 241      21.539  98.350 -15.114  1.00 41.31      B000 C
ATOM   7297  CG   PRO E 241      22.116  99.770 -15.042  1.00 40.16      B000 C
ATOM   7298  CD   PRO E 241      22.851  99.844 -13.732  1.00 35.10      B000 C
ATOM   7299  N    ASP E 242      23.325  96.074 -16.287  1.00 39.07      B000 N
ATOM   7300  CA   ASP E 242      24.337  95.571 -17.221  1.00 42.10      B000 C
ATOM   7301  C    ASP E 242      25.112  96.732 -17.827  1.00 45.64      B000 C
ATOM   7302  O    ASP E 242      24.512  97.697 -18.317  1.00 42.87      B000 O
ATOM   7303  CB   ASP E 242      23.691  94.761 -18.348  1.00 42.84      B000 C
ATOM   7304  CG   ASP E 242      22.886  93.568 -17.852  1.00 43.37      B000 C
ATOM   7305  OD1  ASP E 242      23.330  92.905 -16.895  1.00 46.50      B000 O
ATOM   7306  OD2  ASP E 242      21.800  93.308 -18.430  1.00 43.11      B000 O1-
ATOM   7307  N    ASP E 243      26.448  96.634 -17.808  1.00 39.31      B000 N
```

```
ATOM   7308  CA  ASP E 243      27.300  97.761 -18.190  1.00 47.79      B000 C
ATOM   7309  C   ASP E 243      27.812  97.693 -19.621  1.00 43.78      B000 C
ATOM   7310  O   ASP E 243      28.698  98.475 -19.976  1.00 46.39      B000 O
ATOM   7311  CB  ASP E 243      28.506  97.880 -17.250  1.00 43.08      B000 C
ATOM   7312  CG  ASP E 243      29.395  96.633 -17.252  1.00 54.01      B000 C
ATOM   7313  OD1 ASP E 243      29.151  95.676 -18.025  1.00 56.31      B000 O
ATOM   7314  OD2 ASP E 243      30.350  96.604 -16.453  1.00 60.30      B000 O1-
ATOM   7315  N   TRP E 244      27.332  96.754 -20.435  1.00 42.79      B000 N
ATOM   7316  CA  TRP E 244      27.941  96.563 -21.742  1.00 44.21      B000 C
ATOM   7317  C   TRP E 244      27.283  97.361 -22.860  1.00 43.63      B000 C
ATOM   7318  O   TRP E 244      27.674  97.194 -24.018  1.00 43.92      B000 O
ATOM   7319  CB  TRP E 244      28.043  95.072 -22.108  1.00 43.09      B000 C
ATOM   7320  CG  TRP E 244      26.891  94.182 -21.886  1.00 44.80      B000 C
ATOM   7321  CD1 TRP E 244      25.879  93.900 -22.770  1.00 47.43      B000 C
ATOM   7322  CD2 TRP E 244      26.668  93.355 -20.746  1.00 43.82      B000 C
ATOM   7323  NE1 TRP E 244      25.016  92.980 -22.226  1.00 44.04      B000 N
ATOM   7324  CE2 TRP E 244      25.482  92.621 -20.987  1.00 46.21      B000 C
ATOM   7325  CE3 TRP E 244      27.346  93.171 -19.540  1.00 42.76      B000 C
ATOM   7326  CZ2 TRP E 244      24.957  91.729 -20.060  1.00 44.01      B000 C
ATOM   7327  CZ3 TRP E 244      26.827  92.292 -18.622  1.00 42.24      B000 C
ATOM   7328  CH2 TRP E 244      25.641  91.579 -18.883  1.00 49.08      B000 C
ATOM   7329  N   TYR E 245      26.330  98.242 -22.550  1.00 43.45      B000 N
ATOM   7330  CA  TYR E 245      25.626  99.013 -23.574  1.00 45.01      B000 C
ATOM   7331  C   TYR E 245      26.012 100.481 -23.628  1.00 42.12      B000 C
ATOM   7332  O   TYR E 245      25.598 101.169 -24.560  1.00 45.10      B000 O
ATOM   7333  CB  TYR E 245      24.099  98.947 -23.366  1.00 42.26      B000 C
ATOM   7334  CG  TYR E 245      23.544  97.553 -23.256  1.00 43.72      B000 C
ATOM   7335  CD1 TYR E 245      23.355  96.770 -24.391  1.00 40.29      B000 C
ATOM   7336  CD2 TYR E 245      23.224  97.006 -22.016  1.00 39.35      B000 C
ATOM   7337  CE1 TYR E 245      22.841  95.500 -24.301  1.00 39.08      B000 C
ATOM   7338  CE2 TYR E 245      22.717  95.722 -21.918  1.00 43.84      B000 C
ATOM   7339  CZ  TYR E 245      22.533  94.976 -23.064  1.00 41.96      B000 C
ATOM   7340  OH  TYR E 245      22.036  93.706 -22.975  1.00 45.97      B000 O
ATOM   7341  N   GLY E 246      26.710 101.001 -22.638  1.00 41.64      B000 N
ATOM   7342  CA  GLY E 246      26.989 102.420 -22.592  1.00 43.53      B000 C
ATOM   7343  C   GLY E 246      26.255 103.119 -21.455  1.00 47.49      B000 C
ATOM   7344  O   GLY E 246      25.273 102.626 -20.898  1.00 44.82      B000 O
ATOM   7345  N   HIS E 247      26.714 104.343 -21.189  1.00 50.58      B000 N
ATOM   7346  CA  HIS E 247      26.284 105.118 -20.030  1.00 42.87      B000 C
ATOM   7347  C   HIS E 247      24.783 105.367 -20.033  1.00 44.35      B000 C
ATOM   7348  O   HIS E 247      24.236 105.933 -20.985  1.00 45.35      B000 O
ATOM   7349  CB  HIS E 247      27.044 106.440 -20.003  1.00 53.73      B000 C
ATOM   7350  CG  HIS E 247      26.584 107.373 -18.933  1.00 56.75      B000 C
ATOM   7351  ND1 HIS E 247      26.920 107.206 -17.607  1.00 59.80      B000 N
ATOM   7352  CD2 HIS E 247      25.799 108.476 -18.991  1.00 55.82      B000 C
ATOM   7353  CE1 HIS E 247      26.367 108.172 -16.894  1.00 66.18      B000 C
ATOM   7354  NE2 HIS E 247      25.682 108.956 -17.710  1.00 63.47      B000 N
ATOM   7355  N   GLY E 248      24.117 104.949 -18.954  1.00 45.02      B000 N
ATOM   7356  CA  GLY E 248      22.680 105.131 -18.847  1.00 45.46      B000 C
ATOM   7357  C   GLY E 248      21.849 104.287 -19.791  1.00 46.59      B000 C
ATOM   7358  O   GLY E 248      20.659 104.568 -19.979  1.00 40.42      B000 O
ATOM   7359  N   LEU E 249      22.431 103.242 -20.380  1.00 42.75      B000 N
ATOM   7360  CA  LEU E 249      21.760 102.493 -21.430  1.00 42.93      B000 C
ATOM   7361  C   LEU E 249      21.579 101.026 -21.050  1.00 42.71      B000 C
ATOM   7362  O   LEU E 249      22.327 100.466 -20.239  1.00 35.38      B000 O
ATOM   7363  CB  LEU E 249      22.536 102.595 -22.750  1.00 39.36      B000 C
ATOM   7364  CG  LEU E 249      22.689 104.006 -23.343  1.00 42.86      B000 C
ATOM   7365  CD1 LEU E 249      23.340 103.916 -24.714  1.00 39.37      B000 C
ATOM   7366  CD2 LEU E 249      21.350 104.700 -23.442  1.00 40.64      B000 C
ATOM   7367  N   GLY E 250      20.569 100.410 -21.653  1.00 40.13      B000 N
ATOM   7368  CA  GLY E 250      20.394  98.976 -21.584  1.00 34.39      B000 C
ATOM   7369  C   GLY E 250      20.162  98.460 -22.986  1.00 42.96      B000 C
ATOM   7370  O   GLY E 250      20.251  99.242 -23.936  1.00 39.19      B000 O
ATOM   7371  N   GLY E 251      19.857  97.167 -23.135  1.00 42.21      B000 N
ATOM   7372  CA  GLY E 251      19.622  96.533 -24.418  1.00 38.08      B000 C
ATOM   7373  C   GLY E 251      18.179  96.437 -24.892  1.00 41.51      B000 C
ATOM   7374  O   GLY E 251      17.932  95.813 -25.932  1.00 47.66      B000 O
ATOM   7375  N   GLY E 252      17.209  97.010 -24.169  1.00 45.08      B000 N
ATOM   7376  CA  GLY E 252      15.832  97.049 -24.628  1.00 44.10      B000 C
ATOM   7377  C   GLY E 252      14.893  95.971 -24.115  1.00 40.91      B000 C
ATOM   7378  O   GLY E 252      13.707  96.009 -24.452  1.00 51.51      B000 O
```

```
ATOM   7379  N    GLU E 253     15.380  94.997 -23.351  1.00 49.13      B000 N
ATOM   7380  CA   GLU E 253     14.569  93.886 -22.849  1.00 44.54      B000 C
ATOM   7381  C    GLU E 253     14.410  93.867 -21.335  1.00 40.80      B000 C
ATOM   7382  O    GLU E 253     13.844  92.902 -20.810  1.00 43.41      B000 O
ATOM   7383  CB   GLU E 253     15.180  92.537 -23.273  1.00 49.30      B000 C
ATOM   7384  CG   GLU E 253     15.553  92.443 -24.748  1.00 58.40      B000 C
ATOM   7385  CD   GLU E 253     14.373  92.111 -25.651  1.00 68.11      B000 C
ATOM   7386  OE1  GLU E 253     13.919  90.934 -25.628  1.00 71.69      B000 O
ATOM   7387  OE2  GLU E 253     13.900  93.027 -26.378  1.00 62.49      B000 O1-
ATOM   7388  N    ASP E 254     14.951  94.848 -20.611  1.00 41.14      B000 N
ATOM   7389  CA   ASP E 254     15.056  94.754 -19.159  1.00 36.67      B000 C
ATOM   7390  C    ASP E 254     14.148  95.742 -18.448  1.00 37.02      B000 C
ATOM   7391  O    ASP E 254     13.733  96.776 -18.990  1.00 37.00      B000 O
ATOM   7392  CB   ASP E 254     16.497  94.967 -18.685  1.00 34.36      B000 C
ATOM   7393  CG   ASP E 254     17.333  93.693 -18.779  1.00 37.96      B000 C
ATOM   7394  OD1  ASP E 254     16.776  92.662 -19.226  1.00 36.64      B000 O
ATOM   7395  OD2  ASP E 254     18.547  93.740 -18.453  1.00 41.46      B000 O1-
ATOM   7396  N    CYS E 255     13.822  95.368 -17.219  1.00 35.51      B000 N
ATOM   7397  CA   CYS E 255     13.086  96.210 -16.300  1.00 32.38      B000 C
ATOM   7398  C    CYS E 255     13.781  96.107 -14.955  1.00 37.45      B000 C
ATOM   7399  O    CYS E 255     14.493  95.137 -14.689  1.00 34.78      B000 O
ATOM   7400  CB   CYS E 255     11.612  95.789 -16.219  1.00 34.76      B000 C
ATOM   7401  SG   CYS E 255     10.671  96.035 -17.761  1.00 37.35      B000 S
ATOM   7402  N    ALA E 256     13.609  97.138 -14.123  1.00 31.93      B000 N
ATOM   7403  CA   ALA E 256     14.263  97.192 -12.823  1.00 34.86      B000 C
ATOM   7404  C    ALA E 256     13.329  96.663 -11.744  1.00 35.59      B000 C
ATOM   7405  O    ALA E 256     12.138  96.994 -11.720  1.00 30.92      B000 O
ATOM   7406  CB   ALA E 256     14.688  98.621 -12.480  1.00 33.92      B000 C
ATOM   7407  N    HIS E 257     13.870  95.833 -10.854  1.00 28.95      B000 N
ATOM   7408  CA   HIS E 257     13.097  95.348  -9.721  1.00 35.16      B000 C
ATOM   7409  C    HIS E 257     13.897  95.485  -8.434  1.00 35.13      B000 C
ATOM   7410  O    HIS E 257     15.130  95.402  -8.425  1.00 34.18      B000 O
ATOM   7411  CB   HIS E 257     12.655  93.884  -9.909  1.00 30.40      B000 C
ATOM   7412  CG   HIS E 257     13.789  92.909 -10.005  1.00 36.00      B000 C
ATOM   7413  ND1  HIS E 257     14.213  92.160  -8.930  1.00 36.61      B000 N
ATOM   7414  CD2  HIS E 257     14.576  92.547 -11.050  1.00 29.26      B000 C
ATOM   7415  CE1  HIS E 257     15.205  91.373  -9.306  1.00 36.08      B000 C
ATOM   7416  NE2  HIS E 257     15.444  91.589 -10.588  1.00 41.23      B000 N
ATOM   7417  N    PHE E 258     13.178  95.722  -7.344  1.00 32.98      B000 N
ATOM   7418  CA   PHE E 258     13.783  95.567  -6.036  1.00 35.47      B000 C
ATOM   7419  C    PHE E 258     14.126  94.102  -5.825  1.00 36.56      B000 C
ATOM   7420  O    PHE E 258     13.342  93.217  -6.182  1.00 37.64      B000 O
ATOM   7421  CB   PHE E 258     12.828  96.024  -4.943  1.00 34.07      B000 C
ATOM   7422  CG   PHE E 258     12.279  97.408  -5.140  1.00 36.26      B000 C
ATOM   7423  CD1  PHE E 258     12.991  98.528  -4.707  1.00 31.80      B000 C
ATOM   7424  CD2  PHE E 258     11.025  97.588  -5.714  1.00 31.31      B000 C
ATOM   7425  CE1  PHE E 258     12.469  99.798  -4.865  1.00 33.16      B000 C
ATOM   7426  CE2  PHE E 258     10.492  98.861  -5.882  1.00 39.41      B000 C
ATOM   7427  CZ   PHE E 258     11.208  99.972  -5.462  1.00 34.38      B000 C
ATOM   7428  N    THR E 259     15.302  93.853  -5.249  1.00 37.86      B000 N
ATOM   7429  CA   THR E 259     15.716  92.540  -4.772  1.00 42.13      B000 C
ATOM   7430  C    THR E 259     15.405  92.419  -3.289  1.00 44.25      B000 C
ATOM   7431  O    THR E 259     14.879  93.339  -2.660  1.00 47.92      B000 O
ATOM   7432  CB   THR E 259     17.209  92.303  -4.996  1.00 42.54      B000 C
ATOM   7433  OG1  THR E 259     17.953  93.161  -4.117  1.00 44.35      B000 O
ATOM   7434  CG2  THR E 259     17.579  92.593  -6.433  1.00 39.50      B000 C
ATOM   7435  N    ASP E 260     15.785  91.280  -2.712  1.00 46.15      B000 N
ATOM   7436  CA   ASP E 260     15.408  91.003  -1.334  1.00 48.77      B000 C
ATOM   7437  C    ASP E 260     16.141  91.866  -0.318  1.00 51.79      B000 C
ATOM   7438  O    ASP E 260     15.772  91.841   0.862  1.00 57.39      B000 O
ATOM   7439  CB   ASP E 260     15.627  89.530  -1.002  1.00 48.59      B000 C
ATOM   7440  CG   ASP E 260     17.039  89.064  -1.282  1.00 52.19      B000 C
ATOM   7441  OD1  ASP E 260     17.934  89.890  -1.563  1.00 54.71      B000 O
ATOM   7442  OD2  ASP E 260     17.255  87.847  -1.199  1.00 57.65      B000 O1-
ATOM   7443  N    ASP E 261     17.175  92.602  -0.719  1.00 46.10      B000 N
ATOM   7444  CA   ASP E 261     17.795  93.567   0.177  1.00 47.68      B000 C
ATOM   7445  C    ASP E 261     17.411  95.007  -0.158  1.00 43.64      B000 C
ATOM   7446  O    ASP E 261     17.959  95.938   0.434  1.00 50.15      B000 O
ATOM   7447  CB   ASP E 261     19.322  93.386   0.195  1.00 43.46      B000 C
ATOM   7448  CG   ASP E 261     20.013  93.851  -1.092  1.00 51.38      B000 C
ATOM   7449  OD1  ASP E 261     19.414  94.549  -1.938  1.00 53.10      B000 O
```

```
ATOM   7450  OD2 ASP E 261      21.201  93.523  -1.249  1.00 61.28      B000 O1-
ATOM   7451  N   GLY E 262      16.490  95.208  -1.098  1.00 41.82      B000 N
ATOM   7452  CA  GLY E 262      16.009  96.524  -1.460  1.00 37.30      B000 C
ATOM   7453  C   GLY E 262      16.761  97.180  -2.597  1.00 36.57      B000 C
ATOM   7454  O   GLY E 262      16.212  98.052  -3.272  1.00 41.75      B000 O
ATOM   7455  N   ARG E 263      18.012  96.804  -2.802  1.00 38.36      B000 N
ATOM   7456  CA  ARG E 263      18.764  97.311  -3.928  1.00 41.20      B000 C
ATOM   7457  C   ARG E 263      18.224  96.727  -5.227  1.00 45.69      B000 C
ATOM   7458  O   ARG E 263      17.553  95.687  -5.250  1.00 42.28      B000 O
ATOM   7459  CB  ARG E 263      20.242  96.991  -3.749  1.00 41.55      B000 C
ATOM   7460  CG  ARG E 263      20.841  97.820  -2.615  1.00 42.80      B000 C
ATOM   7461  CD  ARG E 263      22.274  97.464  -2.375  1.00 42.94      B000 C
ATOM   7462  NE  ARG E 263      22.431  96.060  -2.029  1.00 50.20      B000 N
ATOM   7463  CZ  ARG E 263      23.609  95.464  -1.881  1.00 58.64      B000 C
ATOM   7464  NH1 ARG E 263      24.728  96.164  -2.057  1.00 53.73      B000 N1+
ATOM   7465  NH2 ARG E 263      23.670  94.172  -1.573  1.00 56.41      B000 N
ATOM   7466  N   TRP E 264      18.506  97.428  -6.314  1.00 43.23      B000 N
ATOM   7467  CA  TRP E 264      17.854  97.186  -7.588  1.00 42.16      B000 C
ATOM   7468  C   TRP E 264      18.687  96.288  -8.491  1.00 43.43      B000 C
ATOM   7469  O   TRP E 264      19.921  96.322  -8.465  1.00 41.15      B000 O
ATOM   7470  CB  TRP E 264      17.595  98.493  -8.322  1.00 37.69      B000 C
ATOM   7471  CG  TRP E 264      16.910  99.526  -7.547  1.00 42.39      B000 C
ATOM   7472  CD1 TRP E 264      16.342  99.410  -6.306  1.00 42.33      B000 C
ATOM   7473  CD2 TRP E 264      16.714 100.874  -7.958  1.00 41.30      B000 C
ATOM   7474  NE1 TRP E 264      15.797 100.613  -5.924  1.00 37.50      B000 N
ATOM   7475  CE2 TRP E 264      16.011 101.530  -6.921  1.00 43.31      B000 C
ATOM   7476  CE3 TRP E 264      17.068 101.595  -9.104  1.00 38.49      B000 C
ATOM   7477  CZ2 TRP E 264      15.649 102.882  -6.999  1.00 46.31      B000 C
ATOM   7478  CZ3 TRP E 264      16.716 102.938  -9.179  1.00 44.68      B000 C
ATOM   7479  CH2 TRP E 264      16.005 103.564  -8.135  1.00 47.08      B000 C
ATOM   7480  N   ASN E 265      17.986  95.533  -9.339  1.00 40.37      B000 N
ATOM   7481  CA  ASN E 265      18.601  94.718 -10.374  1.00 38.67      B000 C
ATOM   7482  C   ASN E 265      17.770  94.816 -11.652  1.00 35.88      B000 C
ATOM   7483  O   ASN E 265      16.561  95.055 -11.608  1.00 33.72      B000 O
ATOM   7484  CB  ASN E 265      18.731  93.267  -9.899  1.00 40.88      B000 C
ATOM   7485  CG  ASN E 265      19.363  92.369 -10.940  1.00 49.30      B000 C
ATOM   7486  OD1 ASN E 265      20.484  92.622 -11.373  1.00 46.08      B000 O
ATOM   7487  ND2 ASN E 265      18.667  91.288 -11.315  1.00 49.35      B000 N
ATOM   7488  N   ASP E 266      18.431  94.646 -12.795  1.00 37.44      B000 N
ATOM   7489  CA  ASP E 266      17.769  94.648 -14.092  1.00 34.38      B000 C
ATOM   7490  C   ASP E 266      17.585  93.211 -14.559  1.00 35.40      B000 C
ATOM   7491  O   ASP E 266      18.516  92.408 -14.481  1.00 38.07      B000 O
ATOM   7492  CB  ASP E 266      18.549  95.463 -15.137  1.00 34.61      B000 C
ATOM   7493  CG  ASP E 266      20.046  95.092 -15.230  1.00 42.09      B000 C
ATOM   7494  OD1 ASP E 266      20.690  94.762 -14.202  1.00 40.07      B000 O
ATOM   7495  OD2 ASP E 266      20.593  95.167 -16.358  1.00 40.68      B000 O1-
ATOM   7496  N   ASP E 267      16.372  92.880 -15.001  1.00 37.34      B000 N
ATOM   7497  CA  ASP E 267      16.034  91.524 -15.412  1.00 36.69      B000 C
ATOM   7498  C   ASP E 267      15.052  91.576 -16.581  1.00 35.84      B000 C
ATOM   7499  O   ASP E 267      14.431  92.609 -16.843  1.00 37.16      B000 O
ATOM   7500  CB  ASP E 267      15.448  90.748 -14.230  1.00 33.55      B000 C
ATOM   7501  CG  ASP E 267      15.724  89.244 -14.319  1.00 44.66      B000 C
ATOM   7502  OD1 ASP E 267      16.128  88.792 -15.410  1.00 43.90      B000 O
ATOM   7503  OD2 ASP E 267      15.535  88.518 -13.301  1.00 45.36      B000 O1-
ATOM   7504  N   VAL E 268      14.901  90.455 -17.294  1.00 32.21      B000 N
ATOM   7505  CA  VAL E 268      13.973  90.457 -18.423  1.00 34.18      B000 C
ATOM   7506  C   VAL E 268      12.553  90.719 -17.928  1.00 37.30      B000 C
ATOM   7507  O   VAL E 268      12.123  90.211 -16.883  1.00 38.04      B000 O
ATOM   7508  CB  VAL E 268      14.050  89.154 -19.239  1.00 37.36      B000 C
ATOM   7509  CG1 VAL E 268      15.409  89.057 -19.938  1.00 36.15      B000 C
ATOM   7510  CG2 VAL E 268      13.806  87.936 -18.370  1.00 35.18      B000 C
ATOM   7511  N   CYS E 269      11.809  91.513 -18.697  1.00 36.57      B000 N
ATOM   7512  CA  CYS E 269      10.538  92.045 -18.240  1.00 36.70      B000 C
ATOM   7513  C   CYS E 269       9.449  90.995 -18.103  1.00 34.59      B000 C
ATOM   7514  O   CYS E 269       8.418  91.294 -17.500  1.00 35.91      B000 O
ATOM   7515  CB  CYS E 269      10.088  93.163 -19.185  1.00 42.38      B000 C
ATOM   7516  SG  CYS E 269      11.267  94.591 -19.164  1.00 53.92      B000 S
ATOM   7517  N   GLN E 270       9.643  89.777 -18.595  1.00 33.91      B000 N
ATOM   7518  CA  GLN E 270       8.591  88.778 -18.422  1.00 38.11      B000 C
ATOM   7519  C   GLN E 270       8.652  88.057 -17.086  1.00 33.78      B000 C
ATOM   7520  O   GLN E 270       7.760  87.247 -16.828  1.00 33.42      B000 O
```

```
ATOM   7521  CB   GLN E 270      8.578  87.723 -19.549  1.00 38.43      B000 C
ATOM   7522  CG   GLN E 270      9.815  87.602 -20.390  1.00 44.86      B000 C
ATOM   7523  CD   GLN E 270     10.038  88.821 -21.260  1.00 52.91      B000 C
ATOM   7524  OE1  GLN E 270     11.140  89.358 -21.293  1.00 55.48      B000 O
ATOM   7525  NE2  GLN E 270      8.986  89.280 -21.949  1.00 51.33      B000 N
ATOM   7526  N    ARG E 271      9.649  88.338 -16.229  1.00 31.87      B000 N
ATOM   7527  CA   ARG E 271      9.677  87.758 -14.889  1.00 29.54      B000 C
ATOM   7528  C    ARG E 271      8.356  88.049 -14.171  1.00 36.60      B000 C
ATOM   7529  O    ARG E 271      7.873  89.192 -14.210  1.00 35.38      B000 O
ATOM   7530  CB   ARG E 271     10.829  88.325 -14.050  1.00 28.67      B000 C
ATOM   7531  CG   ARG E 271     12.220  87.973 -14.479  1.00 27.89      B000 C
ATOM   7532  CD   ARG E 271     12.418  86.476 -14.518  1.00 31.48      B000 C
ATOM   7533  NE   ARG E 271     13.822  86.098 -14.676  1.00 33.37      B000 N
ATOM   7534  CZ   ARG E 271     14.203  84.864 -14.987  1.00 37.02      B000 C
ATOM   7535  NH1  ARG E 271     13.268  83.937 -15.180  1.00 30.74      B000 N1+
ATOM   7536  NH2  ARG E 271     15.492  84.540 -15.076  1.00 29.23      B000 N
ATOM   7537  N    PRO E 272      7.778  87.122 -13.565  1.00 34.45      B000 N
ATOM   7538  CA   PRO E 272      6.493  87.373 -12.870  1.00 35.22      B000 C
ATOM   7539  C    PRO E 272      6.670  87.925 -11.455  1.00 35.16      B000 C
ATOM   7540  O    PRO E 272      6.234  87.321 -10.470  1.00 31.89      B000 O
ATOM   7541  CB   PRO E 272      5.847  85.979 -12.867  1.00 31.77      B000 C
ATOM   7542  CG   PRO E 272      7.003  85.036 -12.787  1.00 32.91      B000 C
ATOM   7543  CD   PRO E 272      8.114  85.689 -13.621  1.00 30.82      B000 C
ATOM   7544  N    TYR E 273      7.295  89.101 -11.344  1.00 31.62      B000 N
ATOM   7545  CA   TYR E 273      7.555  89.672 -10.028  1.00 32.44      B000 C
ATOM   7546  C    TYR E 273      6.327  90.398  -9.474  1.00 33.52      B000 C
ATOM   7547  O    TYR E 273      5.355  90.667 -10.183  1.00 33.94      B000 O
ATOM   7548  CB   TYR E 273      8.752  90.615 -10.089  1.00 32.72      B000 C
ATOM   7549  CG   TYR E 273     10.050  89.893 -10.371  1.00 34.81      B000 C
ATOM   7550  CD1  TYR E 273     10.215  88.558 -10.004  1.00 30.34      B000 C
ATOM   7551  CD2  TYR E 273     11.125  90.546 -10.996  1.00 34.47      B000 C
ATOM   7552  CE1  TYR E 273     11.428  87.883 -10.249  1.00 31.47      B000 C
ATOM   7553  CE2  TYR E 273     12.329  89.879 -11.245  1.00 31.66      B000 C
ATOM   7554  CZ   TYR E 273     12.468  88.549 -10.867  1.00 32.58      B000 C
ATOM   7555  OH   TYR E 273     13.639  87.883 -11.117  1.00 37.35      B000 O
ATOM   7556  N    ARG E 274      6.356  90.671  -8.170  1.00 34.37      B000 N
ATOM   7557  CA   ARG E 274      5.350  91.549  -7.579  1.00 32.60      B000 C
ATOM   7558  C    ARG E 274      5.542  92.978  -8.089  1.00 35.51      B000 C
ATOM   7559  O    ARG E 274      6.533  93.300  -8.757  1.00 31.65      B000 O
ATOM   7560  CB   ARG E 274      5.426  91.519  -6.048  1.00 31.74      B000 C
ATOM   7561  CG   ARG E 274      5.044  90.177  -5.455  1.00 35.22      B000 C
ATOM   7562  CD   ARG E 274      4.922  90.200  -3.952  1.00 36.81      B000 C
ATOM   7563  NE   ARG E 274      4.320  88.966  -3.474  1.00 36.66      B000 N
ATOM   7564  CZ   ARG E 274      4.010  88.714  -2.206  1.00 38.82      B000 C
ATOM   7565  NH1  ARG E 274      4.266  89.605  -1.260  1.00 40.20      B000 N1+
ATOM   7566  NH2  ARG E 274      3.432  87.563  -1.886  1.00 37.93      B000 N
ATOM   7567  N    TRP E 275      4.591  93.857  -7.757  1.00 34.71      B000 N
ATOM   7568  CA   TRP E 275      4.709  95.242  -8.207  1.00 35.25      B000 C
ATOM   7569  C    TRP E 275      4.038  96.177  -7.209  1.00 33.77      B000 C
ATOM   7570  O    TRP E 275      3.288  95.762  -6.316  1.00 34.64      B000 O
ATOM   7571  CB   TRP E 275      4.133  95.429  -9.622  1.00 29.33      B000 C
ATOM   7572  CG   TRP E 275      2.633  95.433  -9.666  1.00 41.26      B000 C
ATOM   7573  CD1  TRP E 275      1.824  96.531  -9.687  1.00 32.79      B000 C
ATOM   7574  CD2  TRP E 275      1.762  94.290  -9.659  1.00 35.30      B000 C
ATOM   7575  NE1  TRP E 275      0.516  96.147  -9.707  1.00 34.43      B000 N
ATOM   7576  CE2  TRP E 275      0.441  94.779  -9.687  1.00 41.97      B000 C
ATOM   7577  CE3  TRP E 275      1.971  92.908  -9.650  1.00 34.18      B000 C
ATOM   7578  CZ2  TRP E 275     -0.681  93.928  -9.698  1.00 39.53      B000 C
ATOM   7579  CZ3  TRP E 275      0.861  92.059  -9.654  1.00 40.96      B000 C
ATOM   7580  CH2  TRP E 275     -0.445  92.574  -9.680  1.00 40.67      B000 C
ATOM   7581  N    VAL E 276      4.345  97.453  -7.367  1.00 32.75      B000 N
ATOM   7582  CA   VAL E 276      3.827  98.520  -6.528  1.00 36.54      B000 C
ATOM   7583  C    VAL E 276      3.248  99.578  -7.451  1.00 43.35      B000 C
ATOM   7584  O    VAL E 276      3.916  99.998  -8.404  1.00 39.24      B000 O
ATOM   7585  CB   VAL E 276      4.932  99.125  -5.636  1.00 39.71      B000 C
ATOM   7586  CG1  VAL E 276      4.364 100.226  -4.708  1.00 36.15      B000 C
ATOM   7587  CG2  VAL E 276      5.626  98.033  -4.830  1.00 33.07      B000 C
ATOM   7588  N    CYS E 277      2.015 100.004  -7.176  1.00 41.27      B000 N
ATOM   7589  CA   CYS E 277      1.403 101.127  -7.878  1.00 39.77      B000 C
ATOM   7590  C    CYS E 277      1.513 102.387  -7.022  1.00 46.66      B000 C
ATOM   7591  O    CYS E 277      1.519 102.321  -5.786  1.00 41.72      B000 O
```

```
ATOM   7592  CB  CYS E 277      -0.063 100.847  -8.209  1.00 41.42      B000 C
ATOM   7593  SG  CYS E 277      -0.364  99.509  -9.393  1.00 51.15      B000 S
ATOM   7594  N   GLU E 278       1.622 103.536  -7.698  1.00 43.54      B000 N
ATOM   7595  CA  GLU E 278       1.776 104.841  -7.069  1.00 38.79      B000 C
ATOM   7596  C   GLU E 278       0.862 105.858  -7.751  1.00 45.95      B000 C
ATOM   7597  O   GLU E 278       0.782 105.914  -8.988  1.00 39.44      B000 O
ATOM   7598  CB  GLU E 278       3.243 105.302  -7.137  1.00 47.37      B000 C
ATOM   7599  CG  GLU E 278       3.523 106.665  -6.506  1.00 54.07      B000 C
ATOM   7600  CD  GLU E 278       4.983 107.122  -6.640  1.00 55.74      B000 C
ATOM   7601  OE1 GLU E 278       5.813 106.384  -7.214  1.00 49.09      B000 O
ATOM   7602  OE2 GLU E 278       5.299 108.234  -6.158  1.00 63.05      B000 O1-
ATOM   7603  N   THR E 279       0.194 106.677  -6.933  1.00 44.07      B000 N
ATOM   7604  CA  THR E 279      -0.653 107.764  -7.416  1.00 49.75      B000 C
ATOM   7605  C   THR E 279      -0.567 108.944  -6.440  1.00 52.93      B000 C
ATOM   7606  O   THR E 279       0.043 108.854  -5.367  1.00 51.44      B000 O
ATOM   7607  CB  THR E 279      -2.103 107.288  -7.604  1.00 51.62      B000 C
ATOM   7608  OG1 THR E 279      -2.814 108.206  -8.438  1.00 58.36      B000 O
ATOM   7609  CG2 THR E 279      -2.815 107.204  -6.271  1.00 48.77      B000 C
ATOM   7610  N   GLU E 280      -1.195 110.058  -6.818  1.00 57.24      B000 N
ATOM   7611  CA  GLU E 280      -1.083 111.317  -6.090  1.00 59.43      B000 C
ATOM   7612  C   GLU E 280      -2.071 111.410  -4.922  1.00 60.25      B000 C
ATOM   7613  O   GLU E 280      -2.950 110.567  -4.738  1.00 57.16      B000 O
ATOM   7614  CB  GLU E 280      -1.329 112.479  -7.040  1.00 72.93      B000 C
ATOM   7615  CG  GLU E 280      -1.062 112.139  -8.487  1.00 81.08      B000 C
ATOM   7616  CD  GLU E 280      -0.073 113.097  -9.119  1.00105.55      B000 C
ATOM   7617  OE1 GLU E 280      -0.010 114.263  -8.665  1.00104.59      B000 O
ATOM   7618  OE2 GLU E 280       0.648 112.680 -10.055  1.00113.25      B000 O1-
ATOM   7619  N   LEU E 281      -1.917 112.479  -4.135  1.00 77.88      B000 N
ATOM   7620  CA  LEU E 281      -2.807 112.826  -3.011  1.00 70.25      B000 C
ATOM   7621  C   LEU E 281      -2.681 111.825  -1.876  1.00 67.47      B000 C
ATOM   7622  O   LEU E 281      -2.139 112.153  -0.819  1.00 68.88      B000 O
ATOM   7623  CB  LEU E 281      -4.279 112.937  -3.459  1.00 61.71      B000 C
ATOM   7624  CG  LEU E 281      -4.707 114.296  -4.035  1.00 80.45      B000 C
ATOM   7625  CD1 LEU E 281      -5.181 114.179  -5.490  1.00 71.81      B000 C
ATOM   7626  CD2 LEU E 281      -5.774 114.969  -3.143  1.00 70.21      B000 C
TER
ATOM   7627  O   THR F 152     -18.909  43.540  -2.518  1.00 85.67      B000 O
ATOM   7628  N   THR F 152     -17.763  41.399  -0.880  1.00 85.67      B000 N
ATOM   7629  CA  THR F 152     -16.968  42.333  -1.678  1.00 88.53      B000 C
ATOM   7630  C   THR F 152     -17.777  43.608  -2.017  1.00 91.37      B000 C
ATOM   7631  CB  THR F 152     -16.431  41.637  -2.979  1.00 92.20      B000 C
ATOM   7632  OG1 THR F 152     -15.806  42.597  -3.844  1.00 90.90      B000 O
ATOM   7633  CG2 THR F 152     -17.541  40.894  -3.731  1.00 84.33      B000 C
ATOM   7634  N   CYS F 153     -17.202  44.774  -1.715  1.00 87.63      B000 N
ATOM   7635  CA  CYS F 153     -17.870  46.050  -1.926  1.00 82.80      B000 C
ATOM   7636  C   CYS F 153     -17.103  46.899  -2.940  1.00 77.69      B000 C
ATOM   7637  O   CYS F 153     -15.918  46.673  -3.212  1.00 67.09      B000 O
ATOM   7638  CB  CYS F 153     -18.029  46.817  -0.596  1.00 79.27      B000 C
ATOM   7639  SG  CYS F 153     -19.643  46.575   0.239  1.00104.11      B000 S
ATOM   7640  N   CYS F 154     -17.803  47.886  -3.508  1.00 76.91      B000 N
ATOM   7641  CA  CYS F 154     -17.185  48.793  -4.462  1.00 59.34      B000 C
ATOM   7642  C   CYS F 154     -16.202  49.711  -3.745  1.00 54.16      B000 C
ATOM   7643  O   CYS F 154     -16.398  50.041  -2.575  1.00 63.36      B000 O
ATOM   7644  CB  CYS F 154     -18.244  49.634  -5.172  1.00 55.19      B000 C
ATOM   7645  SG  CYS F 154     -19.186  48.756  -6.443  1.00 69.85      B000 S
ATOM   7646  N   PRO F 155     -15.156  50.163  -4.429  1.00 53.28      B000 N
ATOM   7647  CA  PRO F 155     -14.236  51.130  -3.812  1.00 56.60      B000 C
ATOM   7648  C   PRO F 155     -14.973  52.379  -3.346  1.00 57.95      B000 C
ATOM   7649  O   PRO F 155     -16.130  52.640  -3.696  1.00 55.60      B000 O
ATOM   7650  CB  PRO F 155     -13.243  51.459  -4.932  1.00 48.00      B000 C
ATOM   7651  CG  PRO F 155     -13.374  50.336  -5.913  1.00 55.49      B000 C
ATOM   7652  CD  PRO F 155     -14.777  49.826  -5.811  1.00 51.46      B000 C
ATOM   7653  N   VAL F 156     -14.268  53.174  -2.539  1.00 56.46      B000 N
ATOM   7654  CA  VAL F 156     -14.855  54.394  -1.991  1.00 63.80      B000 C
ATOM   7655  C   VAL F 156     -15.250  55.333  -3.126  1.00 59.48      B000 C
ATOM   7656  O   VAL F 156     -14.488  55.538  -4.081  1.00 58.09      B000 O
ATOM   7657  CB  VAL F 156     -13.873  55.058  -1.014  1.00 62.21      B000 C
ATOM   7658  CG1 VAL F 156     -14.577  56.145  -0.211  1.00 55.24      B000 C
ATOM   7659  CG2 VAL F 156     -13.256  54.002  -0.096  1.00 73.19      B000 C
ATOM   7660  N   ASN F 157     -16.460  55.891  -3.031  1.00 53.19      B000 N
ATOM   7661  CA  ASN F 157     -17.093  56.831  -3.964  1.00 54.02      B000 C
```

```
ATOM   7662  C    ASN F 157   -17.605  56.142  -5.227  1.00 54.32      B000 C
ATOM   7663  O    ASN F 157   -18.209  56.814  -6.072  1.00 53.93      B000 O
ATOM   7664  CB   ASN F 157   -16.170  57.985  -4.387  1.00 49.92      B000 C
ATOM   7665  CG   ASN F 157   -15.632  58.759  -3.202  1.00 59.88      B000 C
ATOM   7666  OD1  ASN F 157   -16.357  59.008  -2.236  1.00 56.12      B000 O
ATOM   7667  ND2  ASN F 157   -14.346  59.136  -3.262  1.00 53.76      B000 N
ATOM   7668  N    TRP F 158   -17.394  54.844  -5.396  1.00 49.14      B000 N
ATOM   7669  CA   TRP F 158   -18.015  54.160  -6.516  1.00 47.06      B000 C
ATOM   7670  C    TRP F 158   -19.353  53.577  -6.080  1.00 46.07      B000 C
ATOM   7671  O    TRP F 158   -19.629  53.429  -4.891  1.00 48.01      B000 O
ATOM   7672  CB   TRP F 158   -17.109  53.069  -7.059  1.00 48.92      B000 C
ATOM   7673  CG   TRP F 158   -15.830  53.586  -7.632  1.00 42.48      B000 C
ATOM   7674  CD1  TRP F 158   -14.854  54.269  -6.967  1.00 46.04      B000 C
ATOM   7675  CD2  TRP F 158   -15.364  53.425  -8.975  1.00 40.30      B000 C
ATOM   7676  NE1  TRP F 158   -13.811  54.549  -7.812  1.00 40.04      B000 N
ATOM   7677  CE2  TRP F 158   -14.098  54.047  -9.053  1.00 40.63      B000 C
ATOM   7678  CE3  TRP F 158   -15.900  52.830 -10.124  1.00 38.93      B000 C
ATOM   7679  CZ2  TRP F 158   -13.358  54.092 -10.234  1.00 36.03      B000 C
ATOM   7680  CZ3  TRP F 158   -15.164  52.864 -11.283  1.00 40.94      B000 C
ATOM   7681  CH2  TRP F 158   -13.908  53.499 -11.336  1.00 38.94      B000 C
ATOM   7682  N    VAL F 159   -20.207  53.303  -7.065  1.00 48.15      B000 N
ATOM   7683  CA   VAL F 159   -21.584  52.872  -6.854  1.00 43.77      B000 C
ATOM   7684  C    VAL F 159   -21.777  51.526  -7.536  1.00 53.64      B000 C
ATOM   7685  O    VAL F 159   -21.317  51.328  -8.666  1.00 50.16      B000 O
ATOM   7686  CB   VAL F 159   -22.571  53.918  -7.409  1.00 47.04      B000 C
ATOM   7687  CG1  VAL F 159   -24.008  53.459  -7.275  1.00 49.61      B000 C
ATOM   7688  CG2  VAL F 159   -22.361  55.239  -6.711  1.00 48.78      B000 C
ATOM   7689  N    GLU F 160   -22.478  50.611  -6.864  1.00 57.06      B000 N
ATOM   7690  CA   GLU F 160   -22.632  49.242  -7.343  1.00 56.04      B000 C
ATOM   7691  C    GLU F 160   -23.958  49.061  -8.070  1.00 50.46      B000 C
ATOM   7692  O    GLU F 160   -25.000  49.538  -7.619  1.00 54.39      B000 O
ATOM   7693  CB   GLU F 160   -22.568  48.223  -6.201  1.00 59.56      B000 C
ATOM   7694  CG   GLU F 160   -22.813  46.795  -6.710  1.00 68.99      B000 C
ATOM   7695  CD   GLU F 160   -23.041  45.748  -5.621  1.00 80.25      B000 C
ATOM   7696  OE1  GLU F 160   -22.777  46.016  -4.427  1.00 79.75      B000 O
ATOM   7697  OE2  GLU F 160   -23.491  44.637  -5.985  1.00 78.26      B000 O1-
ATOM   7698  N    HIS F 161   -23.912  48.328  -9.175  1.00 51.49      B000 N
ATOM   7699  CA   HIS F 161   -25.104  47.913  -9.892  1.00 49.49      B000 C
ATOM   7700  C    HIS F 161   -24.786  46.601 -10.587  1.00 53.28      B000 C
ATOM   7701  O    HIS F 161   -23.801  46.508 -11.332  1.00 47.92      B000 O
ATOM   7702  CB   HIS F 161   -25.561  48.959 -10.901  1.00 47.30      B000 C
ATOM   7703  CG   HIS F 161   -26.699  48.503 -11.759  1.00 52.32      B000 C
ATOM   7704  ND1  HIS F 161   -26.511  47.794 -12.930  1.00 49.15      B000 N
ATOM   7705  CD2  HIS F 161   -28.039  48.641 -11.611  1.00 49.74      B000 C
ATOM   7706  CE1  HIS F 161   -27.687  47.540 -13.478  1.00 52.61      B000 C
ATOM   7707  NE2  HIS F 161   -28.630  48.037 -12.696  1.00 54.26      B000 N
ATOM   7708  N    GLU F 162   -25.666  45.618 -10.386  1.00 61.24      B000 N
ATOM   7709  CA   GLU F 162   -25.426  44.226 -10.751  1.00 57.31      B000 C
ATOM   7710  C    GLU F 162   -24.028  43.809 -10.315  1.00 59.22      B000 C
ATOM   7711  O    GLU F 162   -23.715  43.833  -9.118  1.00 59.32      B000 O
ATOM   7712  CB   GLU F 162   -25.596  44.005 -12.253  1.00 54.87      B000 C
ATOM   7713  CG   GLU F 162   -26.901  44.546 -12.835  1.00 59.80      B000 C
ATOM   7714  CD   GLU F 162   -28.197  43.921 -12.303  1.00 84.54      B000 C
ATOM   7715  OE1  GLU F 162   -28.210  43.290 -11.211  1.00 82.94      B000 O
ATOM   7716  OE2  GLU F 162   -29.231  44.078 -13.007  1.00 83.83      B000 O1-
ATOM   7717  N    ARG F 163   -23.163  43.459 -11.263  1.00 55.56      B000 N
ATOM   7718  CA   ARG F 163   -21.811  43.028 -10.919  1.00 70.50      B000 C
ATOM   7719  C    ARG F 163   -20.730  44.041 -11.292  1.00 65.87      B000 C
ATOM   7720  O    ARG F 163   -19.541  43.699 -11.301  1.00 61.99      B000 O
ATOM   7721  CB   ARG F 163   -21.533  41.645 -11.517  1.00 71.43      B000 C
ATOM   7722  CG   ARG F 163   -22.636  40.621 -11.170  1.00 84.91      B000 C
ATOM   7723  CD   ARG F 163   -23.371  40.027 -12.366  1.00 94.82      B000 C
ATOM   7724  NE   ARG F 163   -23.213  38.571 -12.447  1.00110.28      B000 N
ATOM   7725  CZ   ARG F 163   -22.337  37.951 -13.238  1.00109.36      B000 C
ATOM   7726  NH1  ARG F 163   -21.538  38.656 -14.031  1.00107.55      B000 N1+
ATOM   7727  NH2  ARG F 163   -22.266  36.625 -13.245  1.00108.51      B000 N
ATOM   7728  N    SER F 164   -21.104  45.289 -11.551  1.00 62.99      B000 N
ATOM   7729  CA   SER F 164   -20.137  46.326 -11.872  1.00 54.70      B000 C
ATOM   7730  C    SER F 164   -20.163  47.454 -10.845  1.00 52.59      B000 C
ATOM   7731  O    SER F 164   -21.153  47.666 -10.136  1.00 53.73      B000 O
ATOM   7732  CB   SER F 164   -20.405  46.896 -13.272  1.00 56.24      B000 C
```

```
ATOM   7733  OG  SER F 164    -19.909  46.041 -14.287  1.00 65.36      B000 O
ATOM   7734  N   CYS F 165    -19.036  48.161 -10.771  1.00 51.69      B000 N
ATOM   7735  CA  CYS F 165    -18.866  49.373  -9.981  1.00 51.61      B000 C
ATOM   7736  C   CYS F 165    -18.723  50.574 -10.909  1.00 50.55      B000 C
ATOM   7737  O   CYS F 165    -18.004  50.511 -11.912  1.00 48.71      B000 O
ATOM   7738  CB  CYS F 165    -17.633  49.280  -9.076  1.00 55.01      B000 C
ATOM   7739  SG  CYS F 165    -17.771  48.053  -7.751  1.00 71.45      B000 S
ATOM   7740  N   TYR F 166    -19.410  51.670 -10.577  1.00 48.64      B000 N
ATOM   7741  CA  TYR F 166    -19.481  52.838 -11.449  1.00 45.58      B000 C
ATOM   7742  C   TYR F 166    -19.040  54.104 -10.722  1.00 51.09      B000 C
ATOM   7743  O   TYR F 166    -19.325  54.283  -9.533  1.00 49.78      B000 O
ATOM   7744  CB  TYR F 166    -20.886  53.045 -11.982  1.00 39.51      B000 C
ATOM   7745  CG  TYR F 166    -21.427  51.889 -12.777  1.00 45.13      B000 C
ATOM   7746  CD1 TYR F 166    -21.884  50.732 -12.151  1.00 50.08      B000 C
ATOM   7747  CD2 TYR F 166    -21.471  51.945 -14.159  1.00 46.42      B000 C
ATOM   7748  CE1 TYR F 166    -22.393  49.667 -12.887  1.00 48.67      B000 C
ATOM   7749  CE2 TYR F 166    -21.968  50.892 -14.902  1.00 52.75      B000 C
ATOM   7750  CZ  TYR F 166    -22.429  49.752 -14.264  1.00 51.87      B000 C
ATOM   7751  OH  TYR F 166    -22.922  48.712 -15.024  1.00 47.43      B000 O
ATOM   7752  N   TRP F 167    -18.358  54.989 -11.446  1.00 41.54      B000 N
ATOM   7753  CA  TRP F 167    -17.941  56.279 -10.914  1.00 39.17      B000 C
ATOM   7754  C   TRP F 167    -18.472  57.396 -11.821  1.00 40.61      B000 C
ATOM   7755  O   TRP F 167    -18.300  57.352 -13.048  1.00 39.85      B000 O
ATOM   7756  CB  TRP F 167    -16.408  56.328 -10.776  1.00 39.26      B000 C
ATOM   7757  CG  TRP F 167    -15.921  57.583 -10.131  1.00 47.81      B000 C
ATOM   7758  CD1 TRP F 167    -15.728  57.812  -8.787  1.00 43.33      B000 C
ATOM   7759  CD2 TRP F 167    -15.599  58.806 -10.799  1.00 36.88      B000 C
ATOM   7760  NE1 TRP F 167    -15.292  59.110  -8.588  1.00 37.39      B000 N
ATOM   7761  CE2 TRP F 167    -15.191  59.735  -9.807  1.00 42.60      B000 C
ATOM   7762  CE3 TRP F 167    -15.593  59.202 -12.140  1.00 35.30      B000 C
ATOM   7763  CZ2 TRP F 167    -14.796  61.049 -10.122  1.00 32.52      B000 C
ATOM   7764  CZ3 TRP F 167    -15.177  60.506 -12.455  1.00 41.65      B000 C
ATOM   7765  CH2 TRP F 167    -14.805  61.415 -11.442  1.00 34.25      B000 C
ATOM   7766  N   PHE F 168    -19.128  58.386 -11.215  1.00 32.85      B000 N
ATOM   7767  CA  PHE F 168    -19.816  59.459 -11.933  1.00 36.14      B000 C
ATOM   7768  C   PHE F 168    -19.062  60.768 -11.757  1.00 33.17      B000 C
ATOM   7769  O   PHE F 168    -18.933  61.266 -10.640  1.00 32.62      B000 O
ATOM   7770  CB  PHE F 168    -21.257  59.611 -11.439  1.00 31.85      B000 C
ATOM   7771  CG  PHE F 168    -22.092  58.410 -11.718  1.00 43.90      B000 C
ATOM   7772  CD1 PHE F 168    -22.788  58.303 -12.918  1.00 37.94      B000 C
ATOM   7773  CD2 PHE F 168    -22.121  57.349 -10.824  1.00 37.92      B000 C
ATOM   7774  CE1 PHE F 168    -23.534  57.188 -13.204  1.00 33.97      B000 C
ATOM   7775  CE2 PHE F 168    -22.863  56.231 -11.102  1.00 43.27      B000 C
ATOM   7776  CZ  PHE F 168    -23.576  56.147 -12.299  1.00 39.89      B000 C
ATOM   7777  N   SER F 169    -18.544  61.311 -12.851  1.00 34.64      B000 N
ATOM   7778  CA  SER F 169    -17.896  62.613 -12.753  1.00 37.47      B000 C
ATOM   7779  C   SER F 169    -18.933  63.691 -12.453  1.00 35.48      B000 C
ATOM   7780  O   SER F 169    -20.132  63.531 -12.708  1.00 35.12      B000 O
ATOM   7781  CB  SER F 169    -17.154  62.974 -14.046  1.00 31.74      B000 C
ATOM   7782  OG  SER F 169    -18.061  63.533 -14.999  1.00 33.74      B000 O
ATOM   7783  N   ARG F 170    -18.454  64.810 -11.921  1.00 32.66      B000 N
ATOM   7784  CA  ARG F 170    -19.292  65.983 -11.718  1.00 35.64      B000 C
ATOM   7785  C   ARG F 170    -18.692  67.198 -12.423  1.00 37.46      B000 C
ATOM   7786  O   ARG F 170    -18.918  68.347 -12.037  1.00 32.51      B000 O
ATOM   7787  CB  ARG F 170    -19.527  66.211 -10.228  1.00 30.96      B000 C
ATOM   7788  CG  ARG F 170    -20.457  65.128  -9.645  1.00 33.93      B000 C
ATOM   7789  CD  ARG F 170    -20.628  65.237  -8.142  1.00 36.53      B000 C
ATOM   7790  NE  ARG F 170    -19.466  64.712  -7.440  1.00 46.87      B000 N
ATOM   7791  CZ  ARG F 170    -19.272  64.766  -6.121  1.00 50.98      B000 C
ATOM   7792  NH1 ARG F 170    -18.153  64.253  -5.600  1.00 44.20      B000 N1+
ATOM   7793  NH2 ARG F 170    -20.184  65.325  -5.321  1.00 43.86      B000 N
ATOM   7794  N   SER F 171    -17.935  66.933 -13.484  1.00 32.24      B000 N
ATOM   7795  CA  SER F 171    -17.355  67.964 -14.328  1.00 32.82      B000 C
ATOM   7796  C   SER F 171    -17.068  67.317 -15.673  1.00 33.70      B000 C
ATOM   7797  O   SER F 171    -17.093  66.090 -15.807  1.00 32.42      B000 O
ATOM   7798  CB  SER F 171    -16.078  68.565 -13.724  1.00 32.95      B000 C
ATOM   7799  OG  SER F 171    -15.041  67.584 -13.595  1.00 37.93      B000 O
ATOM   7800  N   GLY F 172    -16.766  68.160 -16.662  1.00 27.87      B000 N
ATOM   7801  CA  GLY F 172    -16.607  67.725 -18.032  1.00 30.72      B000 C
ATOM   7802  C   GLY F 172    -15.165  67.477 -18.471  1.00 37.04      B000 C
ATOM   7803  O   GLY F 172    -14.203  68.006 -17.891  1.00 31.19      B000 O
```

```
ATOM   7804  N   LYS F 173    -15.039  66.642 -19.510  1.00 31.65      B000 N
ATOM   7805  CA  LYS F 173    -13.782  66.382 -20.205  1.00 34.23      B000 C
ATOM   7806  C   LYS F 173    -14.091  66.048 -21.659  1.00 34.10      B000 C
ATOM   7807  O   LYS F 173    -15.137  65.468 -21.967  1.00 27.67      B000 O
ATOM   7808  CB  LYS F 173    -12.986  65.212 -19.599  1.00 36.67      B000 C
ATOM   7809  CG  LYS F 173    -12.212  65.491 -18.319  1.00 36.30      B000 C
ATOM   7810  CD  LYS F 173    -11.406  64.251 -17.943  1.00 31.78      B000 C
ATOM   7811  CE  LYS F 173    -10.707  64.369 -16.569  1.00 33.56      B000 C
ATOM   7812  NZ  LYS F 173     -9.556  65.313 -16.523  1.00 45.73      B000 N1+
ATOM   7813  N   ALA F 174    -13.167  66.400 -22.557  1.00 31.22      B000 N
ATOM   7814  CA  ALA F 174    -13.226  65.821 -23.890  1.00 31.29      B000 C
ATOM   7815  C   ALA F 174    -13.149  64.297 -23.774  1.00 30.96      B000 C
ATOM   7816  O   ALA F 174    -12.525  63.758 -22.851  1.00 33.23      B000 O
ATOM   7817  CB  ALA F 174    -12.099  66.364 -24.763  1.00 29.41      B000 C
ATOM   7818  N   TRP F 175    -13.823  63.602 -24.698  1.00 28.04      B000 N
ATOM   7819  CA  TRP F 175    -13.972  62.145 -24.586  1.00 34.38      B000 C
ATOM   7820  C   TRP F 175    -12.623  61.449 -24.421  1.00 37.26      B000 C
ATOM   7821  O   TRP F 175    -12.450  60.587 -23.549  1.00 35.08      B000 O
ATOM   7822  CB  TRP F 175    -14.700  61.599 -25.815  1.00 28.70      B000 C
ATOM   7823  CG  TRP F 175    -15.152  60.160 -25.730  1.00 37.75      B000 C
ATOM   7824  CD1 TRP F 175    -16.401  59.702 -25.374  1.00 32.61      B000 C
ATOM   7825  CD2 TRP F 175    -14.381  58.992 -26.056  1.00 35.80      B000 C
ATOM   7826  NE1 TRP F 175    -16.440  58.337 -25.446  1.00 36.32      B000 N
ATOM   7827  CE2 TRP F 175    -15.219  57.874 -25.866  1.00 39.10      B000 C
ATOM   7828  CE3 TRP F 175    -13.067  58.786 -26.488  1.00 34.63      B000 C
ATOM   7829  CZ2 TRP F 175    -14.788  56.573 -26.097  1.00 34.59      B000 C
ATOM   7830  CZ3 TRP F 175    -12.637  57.494 -26.720  1.00 37.06      B000 C
ATOM   7831  CH2 TRP F 175    -13.493  56.402 -26.517  1.00 41.92      B000 C
ATOM   7832  N   ALA F 176    -11.637  61.842 -25.230  1.00 31.21      B000 N
ATOM   7833  CA  ALA F 176    -10.356  61.158 -25.149  1.00 35.56      B000 C
ATOM   7834  C   ALA F 176     -9.749  61.306 -23.765  1.00 38.45      B000 C
ATOM   7835  O   ALA F 176     -9.108  60.373 -23.270  1.00 39.51      B000 O
ATOM   7836  CB  ALA F 176     -9.403  61.684 -26.229  1.00 24.62      B000 C
ATOM   7837  N   ASP F 177     -9.964  62.449 -23.107  1.00 34.61      B000 N
ATOM   7838  CA  ASP F 177     -9.426  62.601 -21.759  1.00 35.23      B000 C
ATOM   7839  C   ASP F 177    -10.233  61.808 -20.751  1.00 37.46      B000 C
ATOM   7840  O   ASP F 177     -9.663  61.241 -19.812  1.00 34.54      B000 O
ATOM   7841  CB  ASP F 177     -9.379  64.072 -21.351  1.00 34.09      B000 C
ATOM   7842  CG  ASP F 177     -8.386  64.859 -22.167  1.00 41.05      B000 C
ATOM   7843  OD1 ASP F 177     -7.313  64.292 -22.458  1.00 42.78      B000 O
ATOM   7844  OD2 ASP F 177     -8.681  66.028 -22.531  1.00 43.34      B000 O1-
ATOM   7845  N   ALA F 178    -11.561  61.775 -20.907  1.00 34.72      B000 N
ATOM   7846  CA  ALA F 178    -12.357  60.941 -20.010  1.00 40.52      B000 C
ATOM   7847  C   ALA F 178    -11.978  59.471 -20.186  1.00 36.67      B000 C
ATOM   7848  O   ALA F 178    -11.853  58.724 -19.208  1.00 36.37      B000 O
ATOM   7849  CB  ALA F 178    -13.850  61.165 -20.268  1.00 32.74      B000 C
ATOM   7850  N   ASP F 179    -11.714  59.070 -21.424  1.00 34.82      B000 N
ATOM   7851  CA  ASP F 179    -11.257  57.715 -21.706  1.00 36.26      B000 C
ATOM   7852  C   ASP F 179     -9.957  57.397 -20.955  1.00 42.39      B000 C
ATOM   7853  O   ASP F 179     -9.861  56.368 -20.275  1.00 44.65      B000 O
ATOM   7854  CB  ASP F 179    -11.107  57.584 -23.221  1.00 41.84      B000 C
ATOM   7855  CG  ASP F 179    -10.618  56.220 -23.670  1.00 52.24      B000 C
ATOM   7856  OD1 ASP F 179    -11.237  55.192 -23.304  1.00 48.91      B000 O
ATOM   7857  OD2 ASP F 179     -9.620  56.195 -24.430  1.00 51.25      B000 O1-
ATOM   7858  N   ASN F 180     -8.959  58.292 -21.019  1.00 36.75      B000 N
ATOM   7859  CA  ASN F 180     -7.704  58.044 -20.302  1.00 37.14      B000 C
ATOM   7860  C   ASN F 180     -7.923  58.008 -18.796  1.00 39.01      B000 C
ATOM   7861  O   ASN F 180     -7.306  57.205 -18.089  1.00 45.89      B000 O
ATOM   7862  CB  ASN F 180     -6.654  59.118 -20.615  1.00 46.92      B000 C
ATOM   7863  CG  ASN F 180     -6.153  59.087 -22.053  1.00 58.49      B000 C
ATOM   7864  OD1 ASN F 180     -6.232  58.070 -22.751  1.00 61.49      B000 O
ATOM   7865  ND2 ASN F 180     -5.630  60.232 -22.507  1.00 67.59      B000 N
ATOM   7866  N   TYR F 181     -8.782  58.887 -18.283  1.00 43.70      B000 N
ATOM   7867  CA  TYR F 181     -9.073  58.899 -16.852  1.00 39.91      B000 C
ATOM   7868  C   TYR F 181     -9.597  57.540 -16.394  1.00 37.95      B000 C
ATOM   7869  O   TYR F 181     -9.186  57.026 -15.351  1.00 39.77      B000 O
ATOM   7870  CB  TYR F 181    -10.089  60.009 -16.524  1.00 38.63      B000 C
ATOM   7871  CG  TYR F 181    -10.493  60.066 -15.056  1.00 41.58      B000 C
ATOM   7872  CD1 TYR F 181    -11.438  59.167 -14.527  1.00 31.11      B000 C
ATOM   7873  CD2 TYR F 181     -9.928  61.018 -14.193  1.00 31.82      B000 C
ATOM   7874  CE1 TYR F 181    -11.782  59.205 -13.193  1.00 31.20      B000 C
```

```
ATOM   7875  CE2 TYR F 181     -10.285  61.067 -12.856  1.00 32.07      B000 C
ATOM   7876  CZ  TYR F 181     -11.206  60.153 -12.364  1.00 37.73      B000 C
ATOM   7877  OH  TYR F 181     -11.565  60.192 -11.049  1.00 44.00      B000 O
ATOM   7878  N   CYS F 182     -10.545  56.962 -17.133  1.00 38.33      B000 N
ATOM   7879  CA  CYS F 182     -11.080  55.671 -16.702  1.00 46.48      B000 C
ATOM   7880  C   CYS F 182     -10.008  54.591 -16.772  1.00 43.13      B000 C
ATOM   7881  O   CYS F 182      -9.877  53.782 -15.848  1.00 43.36      B000 O
ATOM   7882  CB  CYS F 182     -12.309  55.282 -17.529  1.00 39.09      B000 C
ATOM   7883  SG  CYS F 182     -13.808  56.319 -17.269  1.00 47.89      B000 S
ATOM   7884  N   ARG F 183      -9.196  54.599 -17.834  1.00 45.20      B000 N
ATOM   7885  CA  ARG F 183      -8.147  53.596 -17.968  1.00 43.53      B000 C
ATOM   7886  C   ARG F 183      -7.166  53.667 -16.806  1.00 46.41      B000 C
ATOM   7887  O   ARG F 183      -6.782  52.629 -16.254  1.00 47.85      B000 O
ATOM   7888  CB  ARG F 183      -7.439  53.764 -19.316  1.00 41.85      B000 C
ATOM   7889  CG  ARG F 183      -8.193  53.051 -20.444  1.00 61.73      B000 C
ATOM   7890  CD  ARG F 183      -7.918  53.573 -21.866  1.00 68.72      B000 C
ATOM   7891  NE  ARG F 183      -8.968  53.099 -22.784  1.00 81.47      B000 N
ATOM   7892  CZ  ARG F 183      -9.062  53.405 -24.082  1.00 84.12      B000 C
ATOM   7893  NH1 ARG F 183      -8.149  54.199 -24.652  1.00 78.81      B000 N1+
ATOM   7894  NH2 ARG F 183     -10.082  52.925 -24.811  1.00 60.08      B000 N
ATOM   7895  N   LEU F 184      -6.805  54.884 -16.367  1.00 42.66      B000 N
ATOM   7896  CA  LEU F 184      -5.910  55.030 -15.220  1.00 41.52      B000 C
ATOM   7897  C   LEU F 184      -6.542  54.558 -13.921  1.00 45.58      B000 C
ATOM   7898  O   LEU F 184      -5.822  54.349 -12.944  1.00 51.35      B000 O
ATOM   7899  CB  LEU F 184      -5.441  56.481 -15.057  1.00 36.16      B000 C
ATOM   7900  CG  LEU F 184      -4.410  57.032 -16.056  1.00 52.39      B000 C
ATOM   7901  CD1 LEU F 184      -4.128  58.507 -15.803  1.00 41.04      B000 C
ATOM   7902  CD2 LEU F 184      -3.105  56.258 -15.999  1.00 43.74      B000 C
ATOM   7903  N   GLU F 185      -7.862  54.403 -13.875  1.00 49.78      B000 N
ATOM   7904  CA  GLU F 185      -8.548  53.829 -12.724  1.00 54.22      B000 C
ATOM   7905  C   GLU F 185      -8.765  52.324 -12.849  1.00 48.30      B000 C
ATOM   7906  O   GLU F 185      -9.579  51.769 -12.104  1.00 50.24      B000 O
ATOM   7907  CB  GLU F 185      -9.905  54.506 -12.523  1.00 53.96      B000 C
ATOM   7908  CG  GLU F 185      -9.835  55.994 -12.368  1.00 48.05      B000 C
ATOM   7909  CD  GLU F 185      -9.246  56.395 -11.042  1.00 60.24      B000 C
ATOM   7910  OE1 GLU F 185      -9.666  55.804 -10.016  1.00 64.06      B000 O
ATOM   7911  OE2 GLU F 185      -8.376  57.300 -11.033  1.00 63.60      B000 O1-
ATOM   7912  N   ASP F 186      -8.062  51.659 -13.765  1.00 48.69      B000 N
ATOM   7913  CA  ASP F 186      -8.311  50.254 -14.090  1.00 49.60      B000 C
ATOM   7914  C   ASP F 186      -9.782  50.054 -14.431  1.00 52.34      B000 C
ATOM   7915  O   ASP F 186     -10.445  49.131 -13.950  1.00 49.55      B000 O
ATOM   7916  CB  ASP F 186      -7.889  49.319 -12.951  1.00 53.42      B000 C
ATOM   7917  CG  ASP F 186      -6.443  49.508 -12.545  1.00 79.16      B000 C
ATOM   7918  OD1 ASP F 186      -5.652  50.034 -13.366  1.00 85.17      B000 O
ATOM   7919  OD2 ASP F 186      -6.093  49.125 -11.403  1.00 86.75      B000 O1-
ATOM   7920  N   ALA F 187     -10.303  50.958 -15.252  1.00 50.66      B000 N
ATOM   7921  CA  ALA F 187     -11.705  50.915 -15.627  1.00 43.40      B000 C
ATOM   7922  C   ALA F 187     -11.822  51.404 -17.061  1.00 39.32      B000 C
ATOM   7923  O   ALA F 187     -10.822  51.623 -17.752  1.00 42.43      B000 O
ATOM   7924  CB  ALA F 187     -12.559  51.716 -14.636  1.00 37.47      B000 C
ATOM   7925  N   HIS F 188     -13.048  51.547 -17.523  1.00 38.43      B000 N
ATOM   7926  CA  HIS F 188     -13.273  52.036 -18.864  1.00 42.96      B000 C
ATOM   7927  C   HIS F 188     -14.539  52.878 -18.839  1.00 39.88      B000 C
ATOM   7928  O   HIS F 188     -15.291  52.871 -17.865  1.00 47.38      B000 O
ATOM   7929  CB  HIS F 188     -13.377  50.872 -19.852  1.00 40.78      B000 C
ATOM   7930  CG  HIS F 188     -14.461  49.901 -19.512  1.00 40.48      B000 C
ATOM   7931  ND1 HIS F 188     -15.783  50.124 -19.830  1.00 46.61      B000 N
ATOM   7932  CD2 HIS F 188     -14.427  48.716 -18.858  1.00 45.33      B000 C
ATOM   7933  CE1 HIS F 188     -16.515  49.109 -19.403  1.00 45.57      B000 C
ATOM   7934  NE2 HIS F 188     -15.716  48.241 -18.811  1.00 46.51      B000 N
ATOM   7935  N   LEU F 189     -14.764  53.613 -19.918  1.00 37.01      B000 N
ATOM   7936  CA  LEU F 189     -15.978  54.400 -20.036  1.00 37.46      B000 C
ATOM   7937  C   LEU F 189     -17.191  53.475 -20.121  1.00 43.07      B000 C
ATOM   7938  O   LEU F 189     -17.135  52.415 -20.754  1.00 37.38      B000 O
ATOM   7939  CB  LEU F 189     -15.891  55.299 -21.263  1.00 31.01      B000 C
ATOM   7940  CG  LEU F 189     -14.995  56.532 -21.160  1.00 35.76      B000 C
ATOM   7941  CD1 LEU F 189     -14.766  57.097 -22.537  1.00 33.40      B000 C
ATOM   7942  CD2 LEU F 189     -15.624  57.607 -20.257  1.00 34.63      B000 C
ATOM   7943  N   VAL F 190     -18.301  53.904 -19.502  1.00 37.96      B000 N
ATOM   7944  CA  VAL F 190     -19.417  53.006 -19.237  1.00 34.67      B000 C
ATOM   7945  C   VAL F 190     -19.928  52.393 -20.536  1.00 42.14      B000 C
```

```
ATOM   7946  O    VAL F 190    -20.044  53.064 -21.573  1.00 40.38     B000 O
ATOM   7947  CB   VAL F 190    -20.551  53.722 -18.477  1.00 36.38     B000 C
ATOM   7948  CG1  VAL F 190    -21.205  54.857 -19.319  1.00 31.50     B000 C
ATOM   7949  CG2  VAL F 190    -21.573  52.718 -18.006  1.00 38.12     B000 C
ATOM   7950  N    VAL F 191    -20.178  51.085 -20.485  1.00 40.92     B000 N
ATOM   7951  CA   VAL F 191    -20.787  50.325 -21.567  1.00 40.00     B000 C
ATOM   7952  C    VAL F 191    -22.162  49.884 -21.082  1.00 41.46     B000 C
ATOM   7953  O    VAL F 191    -22.265  49.187 -20.063  1.00 51.69     B000 O
ATOM   7954  CB   VAL F 191    -19.928  49.113 -21.966  1.00 43.90     B000 C
ATOM   7955  CG1  VAL F 191    -20.596  48.350 -23.106  1.00 38.60     B000 C
ATOM   7956  CG2  VAL F 191    -18.503  49.546 -22.338  1.00 38.08     B000 C
ATOM   7957  N    VAL F 192    -23.209  50.284 -21.805  1.00 39.60     B000 N
ATOM   7958  CA   VAL F 192    -24.603  50.091 -21.402  1.00 40.55     B000 C
ATOM   7959  C    VAL F 192    -25.139  48.872 -22.134  1.00 42.33     B000 C
ATOM   7960  O    VAL F 192    -25.454  48.955 -23.326  1.00 43.63     B000 O
ATOM   7961  CB   VAL F 192    -25.464  51.319 -21.747  1.00 43.28     B000 C
ATOM   7962  CG1  VAL F 192    -26.869  51.182 -21.155  1.00 32.94     B000 C
ATOM   7963  CG2  VAL F 192    -24.772  52.629 -21.333  1.00 35.84     B000 C
ATOM   7964  N    THR F 193    -25.341  47.762 -21.429  1.00 48.94     B000 N
ATOM   7965  CA   THR F 193    -25.745  46.531 -22.105  1.00 47.71     B000 C
ATOM   7966  C    THR F 193    -27.211  46.169 -21.896  1.00 49.57     B000 C
ATOM   7967  O    THR F 193    -27.654  45.172 -22.465  1.00 51.89     B000 O
ATOM   7968  CB   THR F 193    -24.866  45.344 -21.673  1.00 37.74     B000 C
ATOM   7969  OG1  THR F 193    -25.053  45.097 -20.280  1.00 43.14     B000 O
ATOM   7970  CG2  THR F 193    -23.361  45.624 -21.935  1.00 34.77     B000 C
ATOM   7971  N    SER F 194    -27.986  46.957 -21.143  1.00 45.16     B000 N
ATOM   7972  CA   SER F 194    -29.364  46.570 -20.845  1.00 43.88     B000 C
ATOM   7973  C    SER F 194    -30.163  47.775 -20.395  1.00 47.69     B000 C
ATOM   7974  O    SER F 194    -29.609  48.782 -19.938  1.00 48.48     B000 O
ATOM   7975  CB   SER F 194    -29.443  45.506 -19.738  1.00 52.95     B000 C
ATOM   7976  OG   SER F 194    -29.162  46.065 -18.455  1.00 47.85     B000 O
ATOM   7977  N    TRP F 195    -31.488  47.622 -20.464  1.00 48.46     B000 N
ATOM   7978  CA   TRP F 195    -32.386  48.680 -20.012  1.00 44.88     B000 C
ATOM   7979  C    TRP F 195    -32.160  49.001 -18.546  1.00 47.13     B000 C
ATOM   7980  O    TRP F 195    -32.238  50.165 -18.133  1.00 45.99     B000 O
ATOM   7981  CB   TRP F 195    -33.843  48.288 -20.250  1.00 41.25     B000 C
ATOM   7982  CG   TRP F 195    -34.410  48.910 -21.469  1.00 54.27     B000 C
ATOM   7983  CD1  TRP F 195    -34.863  48.275 -22.601  1.00 50.60     B000 C
ATOM   7984  CD2  TRP F 195    -34.541  50.319 -21.716  1.00 55.13     B000 C
ATOM   7985  NE1  TRP F 195    -35.307  49.212 -23.521  1.00 52.12     B000 N
ATOM   7986  CE2  TRP F 195    -35.114  50.470 -23.005  1.00 54.46     B000 C
ATOM   7987  CE3  TRP F 195    -34.246  51.467 -20.964  1.00 50.27     B000 C
ATOM   7988  CZ2  TRP F 195    -35.391  51.727 -23.561  1.00 62.09     B000 C
ATOM   7989  CZ3  TRP F 195    -34.530  52.721 -21.513  1.00 54.11     B000 C
ATOM   7990  CH2  TRP F 195    -35.091  52.837 -22.804  1.00 59.63     B000 C
ATOM   7991  N    GLU F 196    -31.875  47.980 -17.747  1.00 43.73     B000 N
ATOM   7992  CA   GLU F 196    -31.638  48.202 -16.330  1.00 46.25     B000 C
ATOM   7993  C    GLU F 196    -30.421  49.094 -16.133  1.00 48.83     B000 C
ATOM   7994  O    GLU F 196    -30.505  50.141 -15.480  1.00 50.61     B000 O
ATOM   7995  CB   GLU F 196    -31.445  46.853 -15.623  1.00 52.52     B000 C
ATOM   7996  CG   GLU F 196    -32.744  46.023 -15.500  1.00 56.68     B000 C
ATOM   7997  CD   GLU F 196    -33.141  45.323 -16.823  1.00 67.73     B000 C
ATOM   7998  OE1  GLU F 196    -32.250  45.039 -17.660  1.00 66.56     B000 O
ATOM   7999  OE2  GLU F 196    -34.348  45.063 -17.030  1.00 76.24     B000 O1-
ATOM   8000  N    GLU F 197    -29.293  48.717 -16.744  1.00 40.21     B000 N
ATOM   8001  CA   GLU F 197    -28.089  49.533 -16.666  1.00 46.56     B000 C
ATOM   8002  C    GLU F 197    -28.364  50.956 -17.149  1.00 46.02     B000 C
ATOM   8003  O    GLU F 197    -27.964  51.927 -16.494  1.00 39.09     B000 O
ATOM   8004  CB   GLU F 197    -26.965  48.881 -17.475  1.00 41.68     B000 C
ATOM   8005  CG   GLU F 197    -25.566  49.351 -17.097  1.00 46.61     B000 C
ATOM   8006  CD   GLU F 197    -24.465  48.581 -17.830  1.00 49.56     B000 C
ATOM   8007  OE1  GLU F 197    -24.781  47.861 -18.816  1.00 45.94     B000 O
ATOM   8008  OE2  GLU F 197    -23.283  48.692 -17.413  1.00 45.17     B000 O1-
ATOM   8009  N    GLN F 198    -29.087  51.091 -18.271  1.00 37.03     B000 N
ATOM   8010  CA   GLN F 198    -29.451  52.403 -18.794  1.00 36.33     B000 C
ATOM   8011  C    GLN F 198    -30.217  53.231 -17.769  1.00 44.55     B000 C
ATOM   8012  O    GLN F 198    -29.896  54.400 -17.546  1.00 40.80     B000 O
ATOM   8013  CB   GLN F 198    -30.275  52.248 -20.065  1.00 35.40     B000 C
ATOM   8014  CG   GLN F 198    -31.035  53.513 -20.445  1.00 35.73     B000 C
ATOM   8015  CD   GLN F 198    -30.137  54.563 -21.106  1.00 43.38     B000 C
ATOM   8016  OE1  GLN F 198    -29.107  54.240 -21.704  1.00 38.10     B000 O
```

```
ATOM    8017  NE2 GLN F 198     -30.535  55.821 -21.007  1.00 38.22      B000 N
ATOM    8018  N   LYS F 199     -31.229  52.641 -17.123  1.00 47.51      B000 N
ATOM    8019  CA  LYS F 199     -32.018  53.399 -16.154  1.00 44.51      B000 C
ATOM    8020  C   LYS F 199     -31.196  53.714 -14.918  1.00 39.60      B000 O
ATOM    8021  O   LYS F 199     -31.308  54.805 -14.351  1.00 43.02      B000 O
ATOM    8022  CB  LYS F 199     -33.276  52.619 -15.760  1.00 48.82      B000 C
ATOM    8023  CG  LYS F 199     -34.257  52.311 -16.891  1.00 50.78      B000 C
ATOM    8024  CD  LYS F 199     -35.683  52.463 -16.392  1.00 65.99      B000 C
ATOM    8025  CE  LYS F 199     -36.698  51.763 -17.281  1.00 72.04      B000 C
ATOM    8026  NZ  LYS F 199     -38.023  51.636 -16.575  1.00 76.98      B000 N1+
ATOM    8027  N   PHE F 200     -30.351  52.773 -14.502  1.00 38.71      B000 N
ATOM    8028  CA  PHE F 200     -29.447  53.007 -13.388  1.00 38.04      B000 C
ATOM    8029  C   PHE F 200     -28.557  54.224 -13.641  1.00 47.55      B000 C
ATOM    8030  O   PHE F 200     -28.381  55.073 -12.761  1.00 42.70      B000 O
ATOM    8031  CB  PHE F 200     -28.604  51.759 -13.163  1.00 40.51      B000 C
ATOM    8032  CG  PHE F 200     -27.381  51.999 -12.347  1.00 41.69      B000 C
ATOM    8033  CD1 PHE F 200     -27.473  52.215 -10.980  1.00 38.29      B000 C
ATOM    8034  CD2 PHE F 200     -26.130  52.027 -12.953  1.00 37.96      B000 C
ATOM    8035  CE1 PHE F 200     -26.330  52.448 -10.216  1.00 42.63      B000 C
ATOM    8036  CE2 PHE F 200     -24.978  52.252 -12.205  1.00 39.67      B000 C
ATOM    8037  CZ  PHE F 200     -25.076  52.466 -10.830  1.00 44.08      B000 C
ATOM    8038  N   VAL F 201     -27.984  54.328 -14.842  1.00 43.83      B000 N
ATOM    8039  CA  VAL F 201     -27.087  55.445 -15.131  1.00 40.81      B000 C
ATOM    8040  C   VAL F 201     -27.865  56.756 -15.182  1.00 44.68      B000 C
ATOM    8041  O   VAL F 201     -27.442  57.758 -14.596  1.00 42.14      B000 O
ATOM    8042  CB  VAL F 201     -26.313  55.196 -16.438  1.00 40.73      B000 C
ATOM    8043  CG1 VAL F 201     -25.631  56.475 -16.900  1.00 38.56      B000 C
ATOM    8044  CG2 VAL F 201     -25.297  54.053 -16.260  1.00 34.68      B000 C
ATOM    8045  N   GLN F 202     -29.005  56.768 -15.894  1.00 37.85      B000 N
ATOM    8046  CA  GLN F 202     -29.859  57.956 -15.959  1.00 43.72      B000 C
ATOM    8047  C   GLN F 202     -30.185  58.499 -14.582  1.00 46.87      B000 C
ATOM    8048  O   GLN F 202     -30.211  59.720 -14.367  1.00 46.96      B000 O
ATOM    8049  CB  GLN F 202     -31.184  57.645 -16.634  1.00 43.62      B000 C
ATOM    8050  CG  GLN F 202     -31.156  57.250 -18.044  1.00 46.33      B000 C
ATOM    8051  CD  GLN F 202     -32.572  57.120 -18.537  1.00 50.37      B000 C
ATOM    8052  OE1 GLN F 202     -32.871  56.353 -19.451  1.00 47.40      B000 O
ATOM    8053  NE2 GLN F 202     -33.469  57.864 -17.901  1.00 48.25      B000 N
ATOM    8054  N   HIS F 203     -30.503  57.603 -13.654  1.00 41.20      B000 N
ATOM    8055  CA  HIS F 203     -30.825  58.034 -12.307  1.00 44.02      B000 C
ATOM    8056  C   HIS F 203     -29.701  58.866 -11.707  1.00 47.89      B000 C
ATOM    8057  O   HIS F 203     -29.958  59.896 -11.078  1.00 47.99      B000 O
ATOM    8058  CB  HIS F 203     -31.106  56.827 -11.421  1.00 40.71      B000 C
ATOM    8059  CG  HIS F 203     -31.413  57.206 -10.013  1.00 53.47      B000 C
ATOM    8060  ND1 HIS F 203     -30.514  57.028  -8.982  1.00 56.30      B000 N
ATOM    8061  CD2 HIS F 203     -32.499  57.805  -9.470  1.00 46.30      B000 C
ATOM    8062  CE1 HIS F 203     -31.044  57.478  -7.858  1.00 55.89      B000 C
ATOM    8063  NE2 HIS F 203     -32.248  57.953  -8.127  1.00 56.08      B000 N
ATOM    8064  N   HIS F 204     -28.444  58.458 -11.918  1.00 41.43      B000 N
ATOM    8065  CA  HIS F 204     -27.328  59.148 -11.283  1.00 39.37      B000 C
ATOM    8066  C   HIS F 204     -26.814  60.355 -12.055  1.00 40.69      B000 C
ATOM    8067  O   HIS F 204     -26.317  61.291 -11.426  1.00 42.64      B000 O
ATOM    8068  CB  HIS F 204     -26.174  58.188 -11.035  1.00 43.78      B000 C
ATOM    8069  CG  HIS F 204     -26.401  57.295  -9.864  1.00 48.22      B000 C
ATOM    8070  ND1 HIS F 204     -26.903  56.017  -9.983  1.00 46.54      B000 N
ATOM    8071  CD2 HIS F 204     -26.255  57.524  -8.538  1.00 40.29      B000 C
ATOM    8072  CE1 HIS F 204     -27.021  55.484  -8.781  1.00 48.73      B000 C
ATOM    8073  NE2 HIS F 204     -26.638  56.378  -7.887  1.00 42.03      B000 N
ATOM    8074  N   ILE F 205     -26.916  60.374 -13.387  1.00 37.14      B000 N
ATOM    8075  CA  ILE F 205     -26.350  61.499 -14.131  1.00 36.61      B000 C
ATOM    8076  C   ILE F 205     -27.319  62.674 -14.148  1.00 42.45      B000 C
ATOM    8077  O   ILE F 205     -26.895  63.820 -14.292  1.00 40.59      B000 O
ATOM    8078  CB  ILE F 205     -25.950  61.116 -15.576  1.00 34.16      B000 C
ATOM    8079  CG1 ILE F 205     -27.167  60.709 -16.402  1.00 34.84      B000 C
ATOM    8080  CG2 ILE F 205     -24.871  60.040 -15.609  1.00 29.47      B000 C
ATOM    8081  CD1 ILE F 205     -26.829  60.403 -17.814  1.00 37.88      B000 C
ATOM    8082  N   GLY F 206     -28.620  62.416 -14.017  1.00 43.01      B000 N
ATOM    8083  CA  GLY F 206     -29.611  63.456 -14.094  1.00 36.11      B000 C
ATOM    8084  C   GLY F 206     -29.691  64.031 -15.491  1.00 41.59      B000 C
ATOM    8085  O   GLY F 206     -29.511  63.335 -16.493  1.00 41.37      B000 O
ATOM    8086  N   PRO F 207     -29.950  65.320 -15.574  1.00 36.32      B000 N
ATOM    8087  CA  PRO F 207     -30.143  65.969 -16.875  1.00 39.41      B000 C
```

```
ATOM  8088  C   PRO F 207  -28.881  66.583 -17.484  1.00 35.53    B000 C
ATOM  8089  O   PRO F 207  -28.943  67.699 -18.000  1.00 50.85    B000 O
ATOM  8090  CB  PRO F 207  -31.148  67.068 -16.531  1.00 38.74    B000 C
ATOM  8091  CG  PRO F 207  -30.663  67.508 -15.160  1.00 35.34    B000 C
ATOM  8092  CD  PRO F 207  -30.199  66.246 -14.457  1.00 39.53    B000 C
ATOM  8093  N   VAL F 208  -27.741  65.910 -17.422  1.00 33.23    B000 N
ATOM  8094  CA  VAL F 208  -26.468  66.469 -17.858  1.00 39.13    B000 C
ATOM  8095  C   VAL F 208  -25.919  65.604 -18.985  1.00 35.96    B000 C
ATOM  8096  O   VAL F 208  -25.881  64.378 -18.856  1.00 36.23    B000 O
ATOM  8097  CB  VAL F 208  -25.470  66.542 -16.690  1.00 35.73    B000 C
ATOM  8098  CG1 VAL F 208  -24.205  67.239 -17.130  1.00 31.91    B000 C
ATOM  8099  CG2 VAL F 208  -26.108  67.258 -15.495  1.00 37.34    B000 C
ATOM  8100  N   ASN F 209  -25.509  66.232 -20.088  1.00 32.26    B000 N
ATOM  8101  CA  ASN F 209  -24.855  65.476 -21.157  1.00 32.97    B000 C
ATOM  8102  C   ASN F 209  -23.617  64.755 -20.626  1.00 34.25    B000 C
ATOM  8103  O   ASN F 209  -22.796  65.346 -19.917  1.00 33.13    B000 O
ATOM  8104  CB  ASN F 209  -24.481  66.389 -22.315  1.00 30.89    B000 C
ATOM  8105  CG  ASN F 209  -25.679  66.764 -23.167  1.00 37.35    B000 C
ATOM  8106  OD1 ASN F 209  -26.585  65.948 -23.377  1.00 36.11    B000 O
ATOM  8107  ND2 ASN F 209  -25.682  67.989 -23.685  1.00 31.49    B000 N
ATOM  8108  N   THR F 210  -23.508  63.458 -20.934  1.00 30.22    B000 N
ATOM  8109  CA  THR F 210  -22.544  62.592 -20.266  1.00 31.09    B000 C
ATOM  8110  C   THR F 210  -21.985  61.559 -21.244  1.00 34.46    B000 C
ATOM  8111  O   THR F 210  -22.756  60.821 -21.873  1.00 32.16    B000 O
ATOM  8112  CB  THR F 210  -23.210  61.900 -19.071  1.00 32.23    B000 C
ATOM  8113  OG1 THR F 210  -23.838  62.882 -18.229  1.00 31.01    B000 O
ATOM  8114  CG2 THR F 210  -22.187  61.116 -18.256  1.00 27.20    B000 C
ATOM  8115  N   TRP F 211  -20.650  61.510 -21.371  1.00 29.01    B000 N
ATOM  8116  CA  TRP F 211  -19.987  60.567 -22.278  1.00 30.78    B000 C
ATOM  8117  C   TRP F 211  -20.208  59.116 -21.857  1.00 33.57    B000 C
ATOM  8118  O   TRP F 211  -20.215  58.792 -20.667  1.00 33.68    B000 O
ATOM  8119  CB  TRP F 211  -18.473  60.834 -22.329  1.00 28.32    B000 C
ATOM  8120  CG  TRP F 211  -18.041  62.091 -23.074  1.00 29.99    B000 C
ATOM  8121  CD1 TRP F 211  -17.177  63.044 -22.623  1.00 28.61    B000 C
ATOM  8122  CD2 TRP F 211  -18.423  62.498 -24.404  1.00 30.26    B000 C
ATOM  8123  NE1 TRP F 211  -16.989  64.012 -23.580  1.00 32.26    B000 N
ATOM  8124  CE2 TRP F 211  -17.742  63.709 -24.681  1.00 29.27    B000 C
ATOM  8125  CE3 TRP F 211  -19.273  61.958 -25.381  1.00 26.34    B000 C
ATOM  8126  CZ2 TRP F 211  -17.891  64.402 -25.888  1.00 28.81    B000 C
ATOM  8127  CZ3 TRP F 211  -19.431  62.640 -26.575  1.00 28.36    B000 C
ATOM  8128  CH2 TRP F 211  -18.739  63.866 -26.818  1.00 34.89    B000 C
ATOM  8129  N   MET F 212  -20.359  58.232 -22.845  1.00 36.13    B000 N
ATOM  8130  CA  MET F 212  -20.261  56.789 -22.634  1.00 36.28    B000 C
ATOM  8131  C   MET F 212  -19.149  56.198 -23.506  1.00 41.20    B000 C
ATOM  8132  O   MET F 212  -18.610  56.848 -24.412  1.00 38.23    B000 O
ATOM  8133  CB  MET F 212  -21.598  56.096 -22.919  1.00 36.01    B000 C
ATOM  8134  CG  MET F 212  -21.950  55.930 -24.386  1.00 32.65    B000 C
ATOM  8135  SD  MET F 212  -23.698  55.486 -24.615  1.00 40.40    B000 S
ATOM  8136  CE  MET F 212  -24.541  57.090 -24.376  1.00 32.88    B000 C
ATOM  8137  N   GLY F 213  -18.810  54.942 -23.233  1.00 41.45    B000 N
ATOM  8138  CA  GLY F 213  -17.729  54.287 -23.952  1.00 36.96    B000 C
ATOM  8139  C   GLY F 213  -18.090  53.824 -25.345  1.00 39.99    B000 C
ATOM  8140  O   GLY F 213  -17.905  52.645 -25.677  1.00 38.24    B000 O
ATOM  8141  N   LEU F 214  -18.614  54.732 -26.173  1.00 34.09    B000 N
ATOM  8142  CA  LEU F 214  -19.135  54.371 -27.489  1.00 37.28    B000 C
ATOM  8143  C   LEU F 214  -18.700  55.449 -28.470  1.00 36.04    B000 C
ATOM  8144  O   LEU F 214  -18.965  56.632 -28.244  1.00 38.95    B000 O
ATOM  8145  CB  LEU F 214  -20.673  54.216 -27.453  1.00 36.90    B000 C
ATOM  8146  CG  LEU F 214  -21.474  53.990 -28.753  1.00 39.75    B000 C
ATOM  8147  CD1 LEU F 214  -20.997  52.758 -29.501  1.00 40.07    B000 C
ATOM  8148  CD2 LEU F 214  -22.967  53.871 -28.493  1.00 34.46    B000 C
ATOM  8149  N   HIS F 215  -18.023  55.054 -29.545  1.00 41.21    B000 N
ATOM  8150  CA  HIS F 215  -17.454  56.028 -30.471  1.00 43.32    B000 C
ATOM  8151  C   HIS F 215  -17.360  55.421 -31.860  1.00 44.63    B000 C
ATOM  8152  O   HIS F 215  -17.496  54.209 -32.041  1.00 41.89    B000 O
ATOM  8153  CB  HIS F 215  -16.081  56.493 -30.014  1.00 37.84    B000 C
ATOM  8154  CG  HIS F 215  -15.059  55.406 -30.023  1.00 48.13    B000 C
ATOM  8155  ND1 HIS F 215  -14.161  55.238 -31.054  1.00 51.31    B000 N
ATOM  8156  CD2 HIS F 215  -14.806  54.416 -29.137  1.00 50.71    B000 C
ATOM  8157  CE1 HIS F 215  -13.391  54.198 -30.797  1.00 52.71    B000 C
ATOM  8158  NE2 HIS F 215  -13.760  53.683 -29.639  1.00 46.57    B000 N
```

```
ATOM   8159  N   ASP F 216   -17.144  56.288 -32.853  1.00 40.97   B000 N
ATOM   8160  CA  ASP F 216   -17.135  55.874 -34.252  1.00 42.50   B000 C
ATOM   8161  C   ASP F 216   -15.783  56.113 -34.912  1.00 43.44   B000 C
ATOM   8162  O   ASP F 216   -15.701  56.251 -36.131  1.00 42.45   B000 O
ATOM   8163  CB  ASP F 216   -18.241  56.589 -35.029  1.00 40.42   B000 C
ATOM   8164  CG  ASP F 216   -17.950  58.067 -35.268  1.00 43.85   B000 C
ATOM   8165  OD1 ASP F 216   -16.970  58.617 -34.703  1.00 43.41   B000 O
ATOM   8166  OD2 ASP F 216   -18.733  58.690 -36.024  1.00 45.80   B000 O1-
ATOM   8167  N   GLN F 217   -14.710  56.137 -34.128  1.00 51.10   B000 N
ATOM   8168  CA  GLN F 217   -13.512  56.817 -34.600  1.00 58.50   B000 C
ATOM   8169  C   GLN F 217   -12.746  56.030 -35.663  1.00 59.01   B000 C
ATOM   8170  O   GLN F 217   -11.858  56.600 -36.302  1.00 64.06   B000 O
ATOM   8171  CB  GLN F 217   -12.653  57.214 -33.385  1.00 60.37   B000 C
ATOM   8172  CG  GLN F 217   -13.415  58.358 -32.581  1.00 65.68   B000 C
ATOM   8173  CD  GLN F 217   -12.601  59.087 -31.493  1.00 70.67   B000 C
ATOM   8174  OE1 GLN F 217   -12.640  58.714 -30.307  1.00 52.11   B000 O
ATOM   8175  NE2 GLN F 217   -11.925  60.175 -31.884  1.00 67.51   B000 N
ATOM   8176  N   ASN F 218   -13.108  54.777 -35.930  1.00 52.55   B000 N
ATOM   8177  CA  ASN F 218   -12.597  54.084 -37.103  1.00 58.15   B000 C
ATOM   8178  C   ASN F 218   -13.615  53.968 -38.223  1.00 65.45   B000 C
ATOM   8179  O   ASN F 218   -13.292  53.430 -39.289  1.00 62.69   B000 O
ATOM   8180  CB  ASN F 218   -12.114  52.698 -36.724  1.00 71.05   B000 C
ATOM   8181  CG  ASN F 218   -10.817  52.743 -35.976  1.00 81.96   B000 C
ATOM   8182  OD1 ASN F 218   -10.715  52.221 -34.854  1.00 76.60   B000 O
ATOM   8183  ND2 ASN F 218    -9.816  53.410 -36.568  1.00 58.18   B000 N
ATOM   8184  N   GLY F 219   -14.834  54.440 -38.007  1.00 51.95   B000 N
ATOM   8185  CA  GLY F 219   -15.897  54.245 -38.957  1.00 50.90   B000 C
ATOM   8186  C   GLY F 219   -17.074  53.561 -38.292  1.00 41.92   B000 C
ATOM   8187  O   GLY F 219   -18.131  54.156 -38.073  1.00 49.46   B000 O
ATOM   8188  N   PRO F 220   -16.902  52.297 -37.938  1.00 48.10   B000 N
ATOM   8189  CA  PRO F 220   -17.984  51.582 -37.251  1.00 50.76   B000 C
ATOM   8190  C   PRO F 220   -18.125  52.032 -35.803  1.00 45.49   B000 C
ATOM   8191  O   PRO F 220   -17.137  52.233 -35.095  1.00 50.03   B000 O
ATOM   8192  CB  PRO F 220   -17.550  50.113 -37.340  1.00 44.33   B000 C
ATOM   8193  CG  PRO F 220   -16.058  50.172 -37.501  1.00 53.78   B000 C
ATOM   8194  CD  PRO F 220   -15.762  51.421 -38.268  1.00 48.76   B000 C
ATOM   8195  N   TRP F 221   -19.371  52.181 -35.365  1.00 42.06   B000 N
ATOM   8196  CA  TRP F 221   -19.631  52.403 -33.953  1.00 44.69   B000 C
ATOM   8197  C   TRP F 221   -19.168  51.194 -33.147  1.00 38.24   B000 C
ATOM   8198  O   TRP F 221   -19.473  50.053 -33.498  1.00 49.53   B000 O
ATOM   8199  CB  TRP F 221   -21.123  52.668 -33.733  1.00 41.17   B000 C
ATOM   8200  CG  TRP F 221   -21.529  54.055 -34.121  1.00 43.26   B000 C
ATOM   8201  CD1 TRP F 221   -22.156  54.450 -35.268  1.00 36.74   B000 C
ATOM   8202  CD2 TRP F 221   -21.300  55.244 -33.355  1.00 34.96   B000 C
ATOM   8203  NE1 TRP F 221   -22.336  55.817 -35.259  1.00 35.73   B000 N
ATOM   8204  CE2 TRP F 221   -21.824  56.322 -34.089  1.00 37.88   B000 C
ATOM   8205  CE3 TRP F 221   -20.709  55.492 -32.116  1.00 35.21   B000 C
ATOM   8206  CZ2 TRP F 221   -21.781  57.639 -33.617  1.00 42.62   B000 C
ATOM   8207  CZ3 TRP F 221   -20.663  56.791 -31.648  1.00 39.03   B000 C
ATOM   8208  CH2 TRP F 221   -21.194  57.848 -32.395  1.00 38.23   B000 C
ATOM   8209  N   LYS F 222   -18.432  51.450 -32.065  1.00 41.84   B000 N
ATOM   8210  CA  LYS F 222   -17.829  50.409 -31.245  1.00 41.88   B000 C
ATOM   8211  C   LYS F 222   -17.885  50.777 -29.770  1.00 45.01   B000 C
ATOM   8212  O   LYS F 222   -17.788  51.953 -29.409  1.00 38.97   B000 O
ATOM   8213  CB  LYS F 222   -16.367  50.176 -31.639  1.00 47.29   B000 C
ATOM   8214  CG  LYS F 222   -16.184  49.600 -33.033  1.00 60.26   B000 C
ATOM   8215  CD  LYS F 222   -14.729  49.672 -33.464  1.00 61.80   B000 C
ATOM   8216  CE  LYS F 222   -14.371  51.081 -33.924  1.00 69.59   B000 C
ATOM   8217  NZ  LYS F 222   -13.030  51.109 -34.562  1.00 80.69   B000 N1+
ATOM   8218  N   TRP F 223   -18.007  49.751 -28.918  1.00 43.07   B000 N
ATOM   8219  CA  TRP F 223   -17.864  49.911 -27.476  1.00 42.29   B000 C
ATOM   8220  C   TRP F 223   -16.411  49.702 -27.074  1.00 45.99   B000 C
ATOM   8221  O   TRP F 223   -15.708  48.879 -27.661  1.00 52.22   B000 O
ATOM   8222  CB  TRP F 223   -18.762  48.940 -26.696  1.00 41.28   B000 C
ATOM   8223  CG  TRP F 223   -20.258  49.200 -26.825  1.00 43.60   B000 C
ATOM   8224  CD1 TRP F 223   -21.155  48.512 -27.605  1.00 41.95   B000 C
ATOM   8225  CD2 TRP F 223   -21.014  50.224 -26.158  1.00 45.65   B000 C
ATOM   8226  NE1 TRP F 223   -22.422  49.047 -27.462  1.00 42.77   B000 N
ATOM   8227  CE2 TRP F 223   -22.366  50.089 -26.575  1.00 41.46   B000 C
ATOM   8228  CE3 TRP F 223   -20.684  51.237 -25.249  1.00 40.03   B000 C
ATOM   8229  CZ2 TRP F 223   -23.376  50.933 -26.118  1.00 42.16   B000 C
```

```
ATOM   8230  CZ3 TRP F 223    -21.695  52.075 -24.792  1.00 38.55    B000 C
ATOM   8231  CH2 TRP F 223    -23.023  51.918 -25.225  1.00 39.46    B000 C
ATOM   8232  N   VAL F 224    -15.966  50.458 -26.065  1.00 39.89    B000 N
ATOM   8233  CA  VAL F 224    -14.557  50.478 -25.696  1.00 41.43    B000 C
ATOM   8234  C   VAL F 224    -14.084  49.166 -25.090  1.00 43.72    B000 C
ATOM   8235  O   VAL F 224    -12.878  48.924 -25.048  1.00 41.80    B000 O
ATOM   8236  CB  VAL F 224    -14.257  51.635 -24.718  1.00 38.57    B000 C
ATOM   8237  CG1 VAL F 224    -14.434  52.978 -25.419  1.00 35.91    B000 C
ATOM   8238  CG2 VAL F 224    -15.154  51.522 -23.509  1.00 37.24    B000 C
ATOM   8239  N   ASP F 225    -14.978  48.330 -24.572  1.00 44.78    B000 N
ATOM   8240  CA  ASP F 225    -14.559  47.074 -23.971  1.00 40.21    B000 C
ATOM   8241  C   ASP F 225    -14.719  45.908 -24.924  1.00 47.22    B000 C
ATOM   8242  O   ASP F 225    -14.534  44.763 -24.513  1.00 49.29    B000 O
ATOM   8243  CB  ASP F 225    -15.321  46.788 -22.679  1.00 41.32    B000 C
ATOM   8244  CG  ASP F 225    -16.790  46.487 -22.914  1.00 46.80    B000 C
ATOM   8245  OD1 ASP F 225    -17.305  46.755 -24.021  1.00 40.18    B000 O
ATOM   8246  OD2 ASP F 225    -17.426  45.946 -21.982  1.00 55.82    B000 O1-
ATOM   8247  N   GLY F 226    -15.078  46.173 -26.181  1.00 46.15    B000 N
ATOM   8248  CA  GLY F 226    -15.202  45.151 -27.189  1.00 44.93    B000 C
ATOM   8249  C   GLY F 226    -16.606  44.610 -27.394  1.00 49.56    B000 C
ATOM   8250  O   GLY F 226    -16.858  43.993 -28.440  1.00 52.14    B000 O
ATOM   8251  N   THR F 227    -17.522  44.843 -26.443  1.00 45.78    B000 N
ATOM   8252  CA  THR F 227    -18.938  44.496 -26.585  1.00 38.28    B000 C
ATOM   8253  C   THR F 227    -19.437  44.781 -27.996  1.00 49.56    B000 C
ATOM   8254  O   THR F 227    -19.216  45.868 -28.544  1.00 51.44    B000 O
ATOM   8255  CB  THR F 227    -19.779  45.282 -25.571  1.00 46.77    B000 C
ATOM   8256  OG1 THR F 227    -19.284  45.062 -24.244  1.00 49.45    B000 O
ATOM   8257  CG2 THR F 227    -21.244  44.872 -25.628  1.00 32.96    B000 C
ATOM   8258  N   ASP F 228    -20.089  43.791 -28.594  1.00 48.42    B000 N
ATOM   8259  CA  ASP F 228    -20.557  43.942 -29.964  1.00 52.56    B000 C
ATOM   8260  C   ASP F 228    -21.653  44.996 -30.034  1.00 47.16    B000 C
ATOM   8261  O   ASP F 228    -22.638  44.926 -29.297  1.00 54.76    B000 O
ATOM   8262  CB  ASP F 228    -21.080  42.609 -30.513  1.00 52.04    B000 C
ATOM   8263  CG  ASP F 228    -21.620  42.741 -31.938  1.00 51.96    B000 C
ATOM   8264  OD1 ASP F 228    -20.800  42.871 -32.867  1.00 55.04    B000 O
ATOM   8265  OD2 ASP F 228    -22.857  42.731 -32.133  1.00 53.62    B000 O1-
ATOM   8266  N   TYR F 229    -21.480  45.975 -30.926  1.00 49.63    B000 N
ATOM   8267  CA  TYR F 229    -22.438  47.072 -31.032  1.00 51.91    B000 C
ATOM   8268  C   TYR F 229    -23.725  46.641 -31.741  1.00 51.02    B000 C
ATOM   8269  O   TYR F 229    -24.823  47.054 -31.343  1.00 48.95    B000 O
ATOM   8270  CB  TYR F 229    -21.800  48.280 -31.749  1.00 41.08    B000 C
ATOM   8271  CG  TYR F 229    -22.801  49.368 -32.155  1.00 42.20    B000 C
ATOM   8272  CD1 TYR F 229    -23.377  50.216 -31.208  1.00 40.58    B000 C
ATOM   8273  CD2 TYR F 229    -23.178  49.530 -33.482  1.00 40.58    B000 C
ATOM   8274  CE1 TYR F 229    -24.293  51.223 -31.586  1.00 37.62    B000 C
ATOM   8275  CE2 TYR F 229    -24.088  50.511 -33.864  1.00 42.49    B000 C
ATOM   8276  CZ  TYR F 229    -24.634  51.359 -32.910  1.00 40.45    B000 C
ATOM   8277  OH  TYR F 229    -25.527  52.323 -33.311  1.00 45.55    B000 O
ATOM   8278  N   GLU F 230    -23.605  45.833 -32.800  1.00 57.91    B000 N
ATOM   8279  CA  GLU F 230    -24.743  45.560 -33.679  1.00 55.10    B000 C
ATOM   8280  C   GLU F 230    -25.831  44.782 -32.956  1.00 53.04    B000 C
ATOM   8281  O   GLU F 230    -27.014  45.118 -33.055  1.00 52.09    B000 O
ATOM   8282  CB  GLU F 230    -24.273  44.782 -34.909  1.00 58.23    B000 C
ATOM   8283  CG  GLU F 230    -25.270  44.727 -36.048  1.00 61.69    B000 C
ATOM   8284  CD  GLU F 230    -25.912  46.067 -36.325  1.00 76.73    B000 C
ATOM   8285  OE1 GLU F 230    -27.160  46.119 -36.285  1.00 75.40    B000 O
ATOM   8286  OE2 GLU F 230    -25.178  47.067 -36.556  1.00 78.82    B000 O1-
ATOM   8287  N   THR F 231    -25.449  43.767 -32.201  1.00 51.54    B000 N
ATOM   8288  CA  THR F 231    -26.410  42.954 -31.478  1.00 55.16    B000 C
ATOM   8289  C   THR F 231    -26.687  43.461 -30.072  1.00 58.68    B000 C
ATOM   8290  O   THR F 231    -27.516  42.866 -29.371  1.00 54.74    B000 O
ATOM   8291  CB  THR F 231    -25.922  41.510 -31.415  1.00 49.05    B000 C
ATOM   8292  OG1 THR F 231    -24.684  41.463 -30.698  1.00 52.11    B000 O
ATOM   8293  CG2 THR F 231    -25.715  40.964 -32.820  1.00 45.52    B000 C
ATOM   8294  N   GLY F 232    -26.030  44.546 -29.646  1.00 55.48    B000 N
ATOM   8295  CA  GLY F 232    -26.155  45.022 -28.285  1.00 46.35    B000 C
ATOM   8296  C   GLY F 232    -27.303  46.007 -28.088  1.00 42.93    B000 C
ATOM   8297  O   GLY F 232    -27.997  46.417 -29.016  1.00 45.48    B000 O
ATOM   8298  N   PHE F 233    -27.484  46.390 -26.830  1.00 40.91    B000 N
ATOM   8299  CA  PHE F 233    -28.463  47.401 -26.462  1.00 38.08    B000 C
ATOM   8300  C   PHE F 233    -28.162  48.738 -27.146  1.00 45.77    B000 C
```

```
ATOM   8301  O    PHE F 233     -26.997  49.125 -27.305  1.00 39.98      B000 O
ATOM   8302  CB   PHE F 233     -28.440  47.548 -24.939  1.00 42.77      B000 C
ATOM   8303  CG   PHE F 233     -29.411  48.544 -24.406  1.00 46.27      B000 C
ATOM   8304  CD1  PHE F 233     -30.749  48.224 -24.274  1.00 47.88      B000 C
ATOM   8305  CD2  PHE F 233     -28.981  49.804 -24.013  1.00 42.70      B000 C
ATOM   8306  CE1  PHE F 233     -31.650  49.153 -23.777  1.00 51.88      B000 C
ATOM   8307  CE2  PHE F 233     -29.871  50.726 -23.515  1.00 41.09      B000 C
ATOM   8308  CZ   PHE F 233     -31.206  50.404 -23.391  1.00 45.56      B000 C
ATOM   8309  N    LYS F 234     -29.225  49.439 -27.562  1.00 40.43      B000 N
ATOM   8310  CA   LYS F 234     -29.131  50.788 -28.118  1.00 41.63      B000 C
ATOM   8311  C    LYS F 234     -30.309  51.634 -27.654  1.00 46.34      B000 C
ATOM   8312  O    LYS F 234     -31.441  51.156 -27.610  1.00 48.63      B000 O
ATOM   8313  CB   LYS F 234     -29.123  50.778 -29.649  1.00 44.55      B000 C
ATOM   8314  CG   LYS F 234     -27.951  50.067 -30.289  1.00 40.30      B000 C
ATOM   8315  CD   LYS F 234     -28.092  50.103 -31.807  1.00 43.14      B000 C
ATOM   8316  CE   LYS F 234     -27.123  49.127 -32.446  1.00 48.35      B000 C
ATOM   8317  NZ   LYS F 234     -27.471  47.729 -32.045  1.00 53.70      B000 N1+
ATOM   8318  N    ASN F 235     -30.060  52.909 -27.362  1.00 47.83      B000 N
ATOM   8319  CA   ASN F 235     -31.120  53.815 -26.906  1.00 38.15      B000 C
ATOM   8320  C    ASN F 235     -30.936  55.217 -27.509  1.00 39.90      B000 C
ATOM   8321  O    ASN F 235     -30.978  56.235 -26.819  1.00 38.13      B000 O
ATOM   8322  CB   ASN F 235     -31.154  53.827 -25.376  1.00 37.57      B000 C
ATOM   8323  CG   ASN F 235     -32.291  54.652 -24.816  1.00 44.55      B000 C
ATOM   8324  OD1  ASN F 235     -33.337  54.797 -25.445  1.00 47.07      B000 O
ATOM   8325  ND2  ASN F 235     -32.088  55.204 -23.620  1.00 46.24      B000 N
ATOM   8326  N    TRP F 236     -30.749  55.278 -28.826  1.00 36.87      B000 N
ATOM   8327  CA   TRP F 236     -30.517  56.540 -29.518  1.00 38.92      B000 C
ATOM   8328  C    TRP F 236     -31.753  57.439 -29.510  1.00 41.60      B000 C
ATOM   8329  O    TRP F 236     -32.890  56.967 -29.580  1.00 44.93      B000 O
ATOM   8330  CB   TRP F 236     -30.123  56.288 -30.973  1.00 32.28      B000 C
ATOM   8331  CG   TRP F 236     -28.813  55.606 -31.170  1.00 44.09      B000 C
ATOM   8332  CD1  TRP F 236     -28.612  54.296 -31.518  1.00 40.32      B000 C
ATOM   8333  CD2  TRP F 236     -27.511  56.192 -31.037  1.00 41.90      B000 C
ATOM   8334  NE1  TRP F 236     -27.266  54.034 -31.608  1.00 35.68      B000 N
ATOM   8335  CE2  TRP F 236     -26.568  55.182 -31.327  1.00 41.49      B000 C
ATOM   8336  CE3  TRP F 236     -27.051  57.474 -30.700  1.00 37.67      B000 C
ATOM   8337  CZ2  TRP F 236     -25.183  55.420 -31.300  1.00 41.56      B000 C
ATOM   8338  CZ3  TRP F 236     -25.686  57.715 -30.683  1.00 37.32      B000 C
ATOM   8339  CH2  TRP F 236     -24.762  56.689 -30.982  1.00 41.77      B000 C
ATOM   8340  N    ARG F 237     -31.508  58.757 -29.481  1.00 35.83      B000 N
ATOM   8341  CA   ARG F 237     -32.537  59.734 -29.801  1.00 44.59      B000 C
ATOM   8342  C    ARG F 237     -33.103  59.444 -31.181  1.00 48.74      B000 C
ATOM   8343  O    ARG F 237     -32.434  58.828 -32.017  1.00 49.21      B000 O
ATOM   8344  CB   ARG F 237     -31.976  61.164 -29.788  1.00 36.97      B000 C
ATOM   8345  CG   ARG F 237     -31.858  61.766 -28.411  1.00 45.68      B000 C
ATOM   8346  CD   ARG F 237     -33.080  62.584 -28.086  1.00 53.50      B000 C
ATOM   8347  NE   ARG F 237     -32.997  63.225 -26.778  1.00 54.24      B000 N
ATOM   8348  CZ   ARG F 237     -32.798  64.523 -26.580  1.00 66.27      B000 C
ATOM   8349  NH1  ARG F 237     -32.738  65.003 -25.335  1.00 53.38      B000 N1+
ATOM   8350  NH2  ARG F 237     -32.650  65.337 -27.621  1.00 69.85      B000 N
ATOM   8351  N    PRO F 238     -34.326  59.897 -31.447  1.00 53.51      B000 N
ATOM   8352  CA   PRO F 238     -34.896  59.751 -32.790  1.00 53.89      B000 C
ATOM   8353  C    PRO F 238     -33.963  60.284 -33.869  1.00 55.90      B000 C
ATOM   8354  O    PRO F 238     -33.458  61.409 -33.783  1.00 47.93      B000 O
ATOM   8355  CB   PRO F 238     -36.184  60.577 -32.707  1.00 53.03      B000 C
ATOM   8356  CG   PRO F 238     -36.568  60.511 -31.263  1.00 50.27      B000 C
ATOM   8357  CD   PRO F 238     -35.285  60.495 -30.496  1.00 54.51      B000 C
ATOM   8358  N    GLU F 239     -33.727  59.454 -34.887  1.00 48.36      B000 N
ATOM   8359  CA   GLU F 239     -32.951  59.792 -36.076  1.00 55.08      B000 C
ATOM   8360  C    GLU F 239     -31.463  59.925 -35.799  1.00 51.71      B000 C
ATOM   8361  O    GLU F 239     -30.717  60.378 -36.673  1.00 53.39      B000 O
ATOM   8362  CB   GLU F 239     -33.471  61.081 -36.715  1.00 54.02      B000 C
ATOM   8363  CG   GLU F 239     -34.894  60.933 -37.217  1.00 64.17      B000 C
ATOM   8364  CD   GLU F 239     -35.603  62.267 -37.357  1.00 82.12      B000 C
ATOM   8365  OE1  GLU F 239     -34.947  63.318 -37.174  1.00 79.31      B000 O
ATOM   8366  OE2  GLU F 239     -36.828  62.260 -37.609  1.00 90.31      B000 O1-
ATOM   8367  N    GLN F 240     -31.010  59.539 -34.613  1.00 45.35      B000 N
ATOM   8368  CA   GLN F 240     -29.594  59.480 -34.313  1.00 45.02      B000 C
ATOM   8369  C    GLN F 240     -29.207  58.001 -34.305  1.00 40.34      B000 C
ATOM   8370  O    GLN F 240     -30.041  57.164 -33.989  1.00 38.58      B000 O
ATOM   8371  CB   GLN F 240     -29.274  60.163 -32.964  1.00 40.27      B000 C
```

```
ATOM   8372  CG   GLN F 240   -29.798  61.588 -32.801  1.00 35.48      B000 C
ATOM   8373  CD   GLN F 240   -29.552  62.460 -34.038  1.00 52.50      B000 C
ATOM   8374  OE1  GLN F 240   -28.448  62.496 -34.599  1.00 44.08      B000 O
ATOM   8375  NE2  GLN F 240   -30.594  63.166 -34.470  1.00 52.73      B000 N
ATOM   8376  N    PRO F 241   -27.933  57.680 -34.609  1.00 41.65      B000 N
ATOM   8377  CA   PRO F 241   -26.875  58.646 -34.945  1.00 36.96      B000 C
ATOM   8378  C    PRO F 241   -27.011  59.145 -36.366  1.00 42.81      B000 C
ATOM   8379  O    PRO F 241   -27.998  58.769 -37.004  1.00 45.53      B000 O
ATOM   8380  CB   PRO F 241   -25.586  57.853 -34.749  1.00 38.94      B000 C
ATOM   8381  CG   PRO F 241   -25.986  56.428 -34.992  1.00 43.27      B000 C
ATOM   8382  CD   PRO F 241   -27.423  56.298 -34.511  1.00 38.99      B000 C
ATOM   8383  N    ASP F 242   -26.097  60.013 -36.818  1.00 39.75      B000 N
ATOM   8384  CA   ASP F 242   -26.139  60.504 -38.190  1.00 36.75      B000 C
ATOM   8385  C    ASP F 242   -26.229  59.323 -39.143  1.00 43.99      B000 C
ATOM   8386  O    ASP F 242   -25.504  58.332 -38.991  1.00 46.47      B000 O
ATOM   8387  CB   ASP F 242   -24.894  61.335 -38.515  1.00 49.95      B000 C
ATOM   8388  CG   ASP F 242   -24.677  62.473 -37.551  1.00 48.49      B000 C
ATOM   8389  OD1  ASP F 242   -25.683  63.064 -37.104  1.00 52.88      B000 O
ATOM   8390  OD2  ASP F 242   -23.497  62.767 -37.248  1.00 50.34      B000 O
ATOM   8391  N    ASP F 243   -27.147  59.405 -40.097  1.00 38.82      B000 N
ATOM   8392  CA   ASP F 243   -27.412  58.269 -40.967  1.00 51.83      B000 C
ATOM   8393  C    ASP F 243   -26.703  58.364 -42.308  1.00 46.33      B000 C
ATOM   8394  O    ASP F 243   -26.951  57.526 -43.172  1.00 49.46      B000 O
ATOM   8395  CB   ASP F 243   -28.919  58.079 -41.193  1.00 42.32      B000 C
ATOM   8396  CG   ASP F 243   -29.605  59.327 -41.786  1.00 64.08      B000 C
ATOM   8397  OD1  ASP F 243   -28.935  60.346 -42.070  1.00 56.96      B000 O
ATOM   8398  OD2  ASP F 243   -30.842  59.286 -41.971  1.00 77.59      B000 O
ATOM   8399  N    TRP F 244   -25.799  59.322 -42.495  1.00 46.94      B000 N
ATOM   8400  CA   TRP F 244   -25.235  59.541 -43.822  1.00 43.08      B000 C
ATOM   8401  C    TRP F 244   -24.046  58.643 -44.134  1.00 41.86      B000 C
ATOM   8402  O    TRP F 244   -23.528  58.721 -45.250  1.00 43.66      B000 O
ATOM   8403  CB   TRP F 244   -24.765  60.989 -43.994  1.00 49.74      B000 C
ATOM   8404  CG   TRP F 244   -25.371  61.991 -43.064  1.00 50.88      B000 C
ATOM   8405  CD1  TRP F 244   -26.695  62.261 -42.902  1.00 52.58      B000 C
ATOM   8406  CD2  TRP F 244   -24.674  62.891 -42.206  1.00 42.95      B000 C
ATOM   8407  NE1  TRP F 244   -26.876  63.254 -41.976  1.00 50.24      B000 N
ATOM   8408  CE2  TRP F 244   -25.650  63.672 -41.536  1.00 51.72      B000 C
ATOM   8409  CE3  TRP F 244   -23.333  63.117 -41.940  1.00 40.66      B000 C
ATOM   8410  CZ2  TRP F 244   -25.322  64.660 -40.608  1.00 34.70      B000 C
ATOM   8411  CZ3  TRP F 244   -23.008  64.096 -41.014  1.00 51.45      B000 C
ATOM   8412  CH2  TRP F 244   -24.002  64.849 -40.357  1.00 45.14      B000 C
ATOM   8413  N    TYR F 245   -23.649  57.749 -43.225  1.00 38.07      B000 N
ATOM   8414  CA   TYR F 245   -22.447  56.950 -43.423  1.00 34.89      B000 C
ATOM   8415  C    TYR F 245   -22.709  55.483 -43.712  1.00 44.67      B000 C
ATOM   8416  O    TYR F 245   -21.778  54.779 -44.103  1.00 52.17      B000 O
ATOM   8417  CB   TYR F 245   -21.529  57.030 -42.191  1.00 38.23      B000 C
ATOM   8418  CG   TYR F 245   -21.240  58.429 -41.761  1.00 43.82      B000 C
ATOM   8419  CD1  TYR F 245   -20.344  59.219 -42.479  1.00 42.91      B000 C
ATOM   8420  CD2  TYR F 245   -21.868  58.976 -40.638  1.00 38.95      B000 C
ATOM   8421  CE1  TYR F 245   -20.090  60.513 -42.101  1.00 39.31      B000 C
ATOM   8422  CE2  TYR F 245   -21.616  60.266 -40.249  1.00 40.49      B000 C
ATOM   8423  CZ   TYR F 245   -20.719  61.032 -40.978  1.00 43.99      B000 C
ATOM   8424  OH   TYR F 245   -20.449  62.323 -40.589  1.00 46.79      B000 O
ATOM   8425  N    GLY F 246   -23.918  54.993 -43.508  1.00 42.86      B000 N
ATOM   8426  CA   GLY F 246   -24.172  53.575 -43.641  1.00 47.08      B000 C
ATOM   8427  C    GLY F 246   -24.546  52.918 -42.321  1.00 48.63      B000 C
ATOM   8428  O    GLY F 246   -24.464  53.502 -41.238  1.00 46.31      B000 O
ATOM   8429  N    HIS F 247   -25.011  51.682 -42.461  1.00 45.64      B000 N
ATOM   8430  CA   HIS F 247   -25.504  50.908 -41.338  1.00 45.98      B000 C
ATOM   8431  C    HIS F 247   -24.400  50.682 -40.309  1.00 49.00      B000 C
ATOM   8432  O    HIS F 247   -23.329  50.161 -40.640  1.00 47.43      B000 O
ATOM   8433  CB   HIS F 247   -26.052  49.570 -41.836  1.00 48.96      B000 C
ATOM   8434  CG   HIS F 247   -26.470  48.656 -40.731  1.00 52.43      B000 C
ATOM   8435  ND1  HIS F 247   -27.650  48.819 -40.037  1.00 52.47      B000 N
ATOM   8436  CD2  HIS F 247   -25.835  47.606 -40.157  1.00 55.93      B000 C
ATOM   8437  CE1  HIS F 247   -27.737  47.891 -39.102  1.00 55.95      B000 C
ATOM   8438  NE2  HIS F 247   -26.649  47.142 -39.154  1.00 65.30      B000 N
ATOM   8439  N    GLY F 248   -24.656  51.080 -39.063  1.00 45.09      B000 N
ATOM   8440  CA   GLY F 248   -23.694  50.884 -37.992  1.00 43.43      B000 C
ATOM   8441  C    GLY F 248   -22.442  51.723 -38.086  1.00 44.64      B000 C
ATOM   8442  O    GLY F 248   -21.463  51.431 -37.391  1.00 45.17      B000 O
```

```
ATOM   8443  N    LEU F 249   -22.449  52.777 -38.907  1.00 44.92      B000 N
ATOM   8444  CA   LEU F 249   -21.244  53.532 -39.232  1.00 47.53      B000 C
ATOM   8445  C    LEU F 249   -21.355  55.001 -38.833  1.00 45.44      B000 C
ATOM   8446  O    LEU F 249   -22.456  55.561 -38.758  1.00 38.63      B000 O
ATOM   8447  CB   LEU F 249   -20.942  53.464 -40.737  1.00 45.64      B000 C
ATOM   8448  CG   LEU F 249   -20.640  52.090 -41.349  1.00 49.29      B000 C
ATOM   8449  CD1  LEU F 249   -20.212  52.237 -42.809  1.00 41.47      B000 C
ATOM   8450  CD2  LEU F 249   -19.590  51.359 -40.538  1.00 35.93      B000 C
ATOM   8451  N    GLY F 250   -20.186  55.619 -38.598  1.00 43.79      B000 N
ATOM   8452  CA   GLY F 250   -20.033  57.062 -38.423  1.00 40.09      B000 C
ATOM   8453  C    GLY F 250   -18.890  57.625 -39.262  1.00 46.39      B000 C
ATOM   8454  O    GLY F 250   -18.312  56.892 -40.068  1.00 46.10      B000 O
ATOM   8455  N    ALA F 251   -18.553  58.910 -39.081  1.00 38.76      B000 N
ATOM   8456  CA   ALA F 251   -17.474  59.596 -39.795  1.00 44.18      B000 C
ATOM   8457  C    ALA F 251   -16.114  59.542 -39.122  1.00 41.29      B000 C
ATOM   8458  O    ALA F 251   -15.172  60.113 -39.673  1.00 48.88      B000 O
ATOM   8459  CB   ALA F 251   -17.762  61.085 -39.974  1.00 67.76      B000 C
ATOM   8460  N    GLY F 252   -15.989  58.946 -37.942  1.00 43.97      B000 N
ATOM   8461  CA   GLY F 252   -14.714  58.903 -37.255  1.00 44.53      B000 C
ATOM   8462  C    GLY F 252   -14.440  60.030 -36.276  1.00 47.60      B000 C
ATOM   8463  O    GLY F 252   -13.427  59.971 -35.575  1.00 55.35      B000 O
ATOM   8464  N    GLU F 253   -15.306  61.045 -36.183  1.00 45.85      B000 N
ATOM   8465  CA   GLU F 253   -15.090  62.165 -35.268  1.00 42.89      B000 C
ATOM   8466  C    GLU F 253   -16.069  62.200 -34.096  1.00 42.96      B000 C
ATOM   8467  O    GLU F 253   -15.975  63.117 -33.272  1.00 41.71      B000 O
ATOM   8468  CB   GLU F 253   -15.188  63.512 -36.017  1.00 43.68      B000 C
ATOM   8469  CG   GLU F 253   -14.363  63.614 -37.321  1.00 57.15      B000 C
ATOM   8470  CD   GLU F 253   -12.875  63.967 -37.117  1.00 70.88      B000 C
ATOM   8471  OE1  GLU F 253   -12.568  65.139 -36.771  1.00 71.64      B000 O
ATOM   8472  OE2  GLU F 253   -12.008  63.079 -37.331  1.00 63.29      B000 O
ATOM   8473  N    ASP F 254   -17.000  61.242 -33.982  1.00 36.37      B000 N
ATOM   8474  CA   ASP F 254   -18.107  61.378 -33.045  1.00 37.71      B000 C
ATOM   8475  C    ASP F 254   -17.988  60.412 -31.877  1.00 36.53      B000 C
ATOM   8476  O    ASP F 254   -17.374  59.349 -31.973  1.00 36.91      B000 O
ATOM   8477  CB   ASP F 254   -19.473  61.157 -33.717  1.00 35.74      B000 C
ATOM   8478  CG   ASP F 254   -20.006  62.400 -34.396  1.00 38.14      B000 C
ATOM   8479  OD2  ASP F 254   -21.148  62.350 -34.936  1.00 36.55      B000 O
ATOM   8480  OD1  ASP F 254   -19.280  63.427 -34.386  1.00 41.05      B000 O1-
ATOM   8481  N    CYS F 255   -18.603  60.799 -30.769  1.00 38.85      B000 N
ATOM   8482  CA   CYS F 255   -18.733  59.942 -29.606  1.00 28.30      B000 C
ATOM   8483  C    CYS F 255   -20.176  60.039 -29.135  1.00 32.80      B000 C
ATOM   8484  O    CYS F 255   -20.878  61.013 -29.418  1.00 34.41      B000 O
ATOM   8485  CB   CYS F 255   -17.749  60.342 -28.515  1.00 32.19      B000 C
ATOM   8486  SG   CYS F 255   -15.954  60.090 -28.944  1.00 42.14      B000 S
ATOM   8487  N    ALA F 256   -20.630  59.006 -28.447  1.00 31.53      B000 N
ATOM   8488  CA   ALA F 256   -22.004  58.933 -27.991  1.00 30.16      B000 C
ATOM   8489  C    ALA F 256   -22.096  59.438 -26.562  1.00 34.02      B000 C
ATOM   8490  O    ALA F 256   -21.281  59.074 -25.706  1.00 31.73      B000 O
ATOM   8491  CB   ALA F 256   -22.530  57.498 -28.071  1.00 28.94      B000 C
ATOM   8492  N    HIS F 257   -23.103  60.261 -26.301  1.00 28.80      B000 N
ATOM   8493  CA   HIS F 257   -23.347  60.726 -24.947  1.00 33.62      B000 C
ATOM   8494  C    HIS F 257   -24.822  60.589 -24.608  1.00 29.12      B000 C
ATOM   8495  O    HIS F 257   -25.696  60.659 -25.483  1.00 29.71      B000 O
ATOM   8496  CB   HIS F 257   -22.895  62.209 -24.730  1.00 31.05      B000 C
ATOM   8497  CG   HIS F 257   -23.622  63.196 -25.584  1.00 27.54      B000 C
ATOM   8498  ND1  HIS F 257   -24.699  63.926 -25.124  1.00 35.11      B000 N
ATOM   8499  CD2  HIS F 257   -23.433  63.578 -26.873  1.00 31.63      B000 C
ATOM   8500  CE1  HIS F 257   -25.140  64.719 -26.088  1.00 30.09      B000 C
ATOM   8501  NE2  HIS F 257   -24.389  64.529 -27.160  1.00 34.93      B000 N
ATOM   8502  N    PHE F 258   -25.083  60.369 -23.324  1.00 31.39      B000 N
ATOM   8503  CA   PHE F 258   -26.433  60.529 -22.810  1.00 37.72      B000 C
ATOM   8504  C    PHE F 258   -26.830  61.993 -22.915  1.00 35.33      B000 C
ATOM   8505  O    PHE F 258   -26.020  62.879 -22.636  1.00 37.08      B000 O
ATOM   8506  CB   PHE F 258   -26.519  60.088 -21.354  1.00 31.41      B000 C
ATOM   8507  CG   PHE F 258   -26.032  58.691 -21.094  1.00 30.94      B000 C
ATOM   8508  CD1  PHE F 258   -26.884  57.598 -21.263  1.00 34.60      B000 C
ATOM   8509  CD2  PHE F 258   -24.750  58.465 -20.612  1.00 31.04      B000 C
ATOM   8510  CE1  PHE F 258   -26.449  56.297 -20.988  1.00 35.62      B000 C
ATOM   8511  CE2  PHE F 258   -24.295  57.160 -20.350  1.00 37.38      B000 C
ATOM   8512  CZ   PHE F 258   -25.156  56.076 -20.534  1.00 34.44      B000 C
ATOM   8513  N    THR F 259   -28.072  62.242 -23.340  1.00 39.97      B000 N
```

```
ATOM   8514  CA  THR F 259    -28.683  63.565 -23.297  1.00 37.91      B000 C
ATOM   8515  C   THR F 259    -29.508  63.699 -22.023  1.00 38.85      B000 C
ATOM   8516  O   THR F 259    -29.572  62.785 -21.197  1.00 42.20      B000 O
ATOM   8517  CB  THR F 259    -29.566  63.817 -24.514  1.00 38.22      B000 C
ATOM   8518  OG1 THR F 259    -30.726  62.979 -24.427  1.00 40.99      B000 O
ATOM   8519  CG2 THR F 259    -28.812  63.526 -25.789  1.00 35.73      B000 C
ATOM   8520  N   ASP F 260    -30.176  64.845 -21.874  1.00 41.22      B000 N
ATOM   8521  CA  ASP F 260    -30.859  65.107 -20.611  1.00 45.54      B000 C
ATOM   8522  C   ASP F 260    -32.098  64.241 -20.394  1.00 44.40      B000 C
ATOM   8523  O   ASP F 260    -32.576  64.175 -19.260  1.00 47.44      B000 O
ATOM   8524  CB  ASP F 260    -31.211  66.595 -20.486  1.00 42.85      B000 C
ATOM   8525  CG  ASP F 260    -32.026  67.112 -21.654  1.00 50.76      B000 C
ATOM   8526  OD1 ASP F 260    -32.520  66.298 -22.465  1.00 48.72      B000 O
ATOM   8527  OD2 ASP F 260    -32.156  68.351 -21.769  1.00 54.59      B000 O1-
ATOM   8528  N   ASP F 261    -32.598  63.531 -21.406  1.00 42.99      B000 N
ATOM   8529  CA  ASP F 261    -33.649  62.544 -21.167  1.00 41.51      B000 C
ATOM   8530  C   ASP F 261    -33.118  61.115 -21.152  1.00 43.32      B000 C
ATOM   8531  O   ASP F 261    -33.911  60.169 -21.146  1.00 48.34      B000 O
ATOM   8532  CB  ASP F 261    -34.795  62.676 -22.192  1.00 37.72      B000 C
ATOM   8533  CG  ASP F 261    -34.432  62.185 -23.606  1.00 50.07      B000 C
ATOM   8534  OD1 ASP F 261    -33.408  61.504 -23.808  1.00 49.28      B000 O
ATOM   8535  OD2 ASP F 261    -35.212  62.470 -24.543  1.00 57.06      B000 O1-
ATOM   8536  N   GLY F 262    -31.801  60.929 -21.192  1.00 37.84      B000 N
ATOM   8537  CA  GLY F 262    -31.223  59.600 -21.120  1.00 39.30      B000 C
ATOM   8538  C   GLY F 262    -30.982  58.938 -22.466  1.00 41.80      B000 C
ATOM   8539  O   GLY F 262    -30.102  58.076 -22.575  1.00 39.59      B000 O
ATOM   8540  N   ARG F 263    -31.736  59.319 -23.494  1.00 35.33      B000 N
ATOM   8541  CA  ARG F 263    -31.468  58.795 -24.822  1.00 38.94      B000 C
ATOM   8542  C   ARG F 263    -30.159  59.370 -25.357  1.00 41.24      B000 C
ATOM   8543  O   ARG F 263    -29.688  60.429 -24.926  1.00 40.40      B000 O
ATOM   8544  CB  ARG F 263    -32.646  59.085 -25.760  1.00 39.18      B000 C
ATOM   8545  CG  ARG F 263    -33.888  58.222 -25.393  1.00 46.56      B000 C
ATOM   8546  CD  ARG F 263    -35.120  58.506 -26.239  1.00 38.08      B000 C
ATOM   8547  NE  ARG F 263    -35.507  59.908 -26.134  1.00 51.22      B000 N
ATOM   8548  CZ  ARG F 263    -36.495  60.464 -26.827  1.00 58.02      B000 C
ATOM   8549  NH1 ARG F 263    -37.218  59.731 -27.673  1.00 52.64      B000 N1+
ATOM   8550  NH2 ARG F 263    -36.754  61.756 -26.676  1.00 51.34      B000 N
ATOM   8551  N   TRP F 264    -29.579  58.655 -26.311  1.00 35.20      B000 N
ATOM   8552  CA  TRP F 264    -28.211  58.875 -26.746  1.00 33.30      B000 C
ATOM   8553  C   TRP F 264    -28.146  59.805 -27.951  1.00 36.94      B000 C
ATOM   8554  O   TRP F 264    -29.059  59.860 -28.785  1.00 39.27      B000 O
ATOM   8555  CB  TRP F 264    -27.546  57.555 -27.110  1.00 32.18      B000 C
ATOM   8556  CG  TRP F 264    -27.593  56.508 -26.057  1.00 35.42      B000 C
ATOM   8557  CD1 TRP F 264    -28.003  56.652 -24.766  1.00 40.13      B000 C
ATOM   8558  CD2 TRP F 264    -27.196  55.143 -26.203  1.00 36.70      B000 C
ATOM   8559  NE1 TRP F 264    -27.880  55.459 -24.095  1.00 37.06      B000 N
ATOM   8560  CE2 TRP F 264    -27.388  54.516 -24.960  1.00 38.24      B000 C
ATOM   8561  CE3 TRP F 264    -26.688  54.387 -27.270  1.00 40.49      B000 C
ATOM   8562  CZ2 TRP F 264    -27.101  53.169 -24.756  1.00 35.99      B000 C
ATOM   8563  CZ3 TRP F 264    -26.407  53.045 -27.061  1.00 32.82      B000 C
ATOM   8564  CH2 TRP F 264    -26.613  52.457 -25.819  1.00 37.82      B000 C
ATOM   8565  N   ASN F 265    -27.044  60.540 -28.034  1.00 31.28      B000 N
ATOM   8566  CA  ASN F 265    -26.763  61.379 -29.187  1.00 33.60      B000 C
ATOM   8567  C   ASN F 265    -25.275  61.280 -29.513  1.00 33.44      B000 C
ATOM   8568  O   ASN F 265    -24.446  60.973 -28.645  1.00 29.37      B000 O
ATOM   8569  CB  ASN F 265    -27.200  62.832 -28.912  1.00 31.57      B000 C
ATOM   8570  CG  ASN F 265    -26.915  63.768 -30.076  1.00 39.82      B000 C
ATOM   8571  OD1 ASN F 265    -27.466  63.615 -31.166  1.00 41.22      B000 O
ATOM   8572  ND2 ASN F 265    -26.003  64.724 -29.856  1.00 36.27      B000 N
ATOM   8573  N   ASP F 266    -24.950  61.494 -30.786  1.00 31.29      B000 N
ATOM   8574  CA  ASP F 266    -23.572  61.488 -31.255  1.00 31.23      B000 C
ATOM   8575  C   ASP F 266    -23.107  62.919 -31.488  1.00 32.82      B000 C
ATOM   8576  O   ASP F 266    -23.788  63.693 -32.166  1.00 30.42      B000 O
ATOM   8577  CB  ASP F 266    -23.408  60.650 -32.536  1.00 36.08      B000 C
ATOM   8578  CG  ASP F 266    -24.411  61.018 -33.661  1.00 40.88      B000 C
ATOM   8579  OD1 ASP F 266    -25.586  61.367 -33.390  1.00 36.41      B000 O
ATOM   8580  OD2 ASP F 266    -24.013  60.933 -34.844  1.00 40.99      B000 O1-
ATOM   8581  N   ASP F 267    -21.943  63.261 -30.935  1.00 35.94      B000 N
ATOM   8582  CA  ASP F 267    -21.403  64.616 -31.027  1.00 36.54      B000 C
ATOM   8583  C   ASP F 267    -19.880  64.547 -31.126  1.00 34.86      B000 C
ATOM   8584  O   ASP F 267    -19.271  63.507 -30.850  1.00 34.33      B000 O
```

```
ATOM   8585  CB   ASP F 267   -21.835  65.470 -29.834  1.00 30.38      B000 C
ATOM   8586  CG   ASP F 267   -21.905  66.947 -30.180  1.00 43.85      B000 C
ATOM   8587  OD1  ASP F 267   -21.377  67.294 -31.265  1.00 39.52      B000 O
ATOM   8588  OD2  ASP F 267   -22.463  67.744 -29.369  1.00 37.84      B000 O1-
ATOM   8589  N    VAL F 268   -19.263  65.675 -31.519  1.00 30.07      B000 N
ATOM   8590  CA   VAL F 268   -17.811  65.692 -31.674  1.00 34.19      B000 C
ATOM   8591  C    VAL F 268   -17.155  65.408 -30.331  1.00 32.91      B000 C
ATOM   8592  O    VAL F 268   -17.605  65.876 -29.277  1.00 34.33      B000 O
ATOM   8593  CB   VAL F 268   -17.313  67.013 -32.292  1.00 36.17      B000 C
ATOM   8594  CG1  VAL F 268   -17.784  67.114 -33.734  1.00 30.93      B000 C
ATOM   8595  CG2  VAL F 268   -17.786  68.204 -31.515  1.00 29.63      B000 C
ATOM   8596  N    CYS F 269   -16.096  64.605 -30.366  1.00 30.58      B000 N
ATOM   8597  CA   CYS F 269   -15.512  64.059 -29.147  1.00 32.39      B000 C
ATOM   8598  C    CYS F 269   -14.819  65.101 -28.276  1.00 35.95      B000 C
ATOM   8599  O    CYS F 269   -14.508  64.795 -27.110  1.00 34.91      B000 O
ATOM   8600  CB   CYS F 269   -14.527  62.940 -29.502  1.00 40.17      B000 C
ATOM   8601  SG   CYS F 269   -15.360  61.521 -30.340  1.00 50.58      B000 S
ATOM   8602  N    GLN F 270   -14.605  66.326 -28.768  1.00 30.41      B000 N
ATOM   8603  CA   GLN F 270   -13.991  67.316 -27.886  1.00 37.36      B000 C
ATOM   8604  C    GLN F 270   -14.990  68.026 -26.982  1.00 33.95      B000 C
ATOM   8605  O    GLN F 270   -14.541  68.820 -26.158  1.00 37.08      B000 O
ATOM   8606  CB   GLN F 270   -13.179  68.371 -28.659  1.00 34.85      B000 C
ATOM   8607  CG   GLN F 270   -13.560  68.598 -30.097  1.00 52.16      B000 C
ATOM   8608  CD   GLN F 270   -13.134  67.433 -30.977  1.00 59.41      B000 C
ATOM   8609  OE1  GLN F 270   -13.911  66.970 -31.818  1.00 54.59      B000 O
ATOM   8610  NE2  GLN F 270   -11.912  66.916 -30.748  1.00 48.02      B000 N
ATOM   8611  N    ARG F 271   -16.302  67.744 -27.083  1.00 27.57      B000 N
ATOM   8612  CA   ARG F 271   -17.274  68.311 -26.158  1.00 29.87      B000 C
ATOM   8613  C    ARG F 271   -16.853  68.013 -24.720  1.00 34.79      B000 C
ATOM   8614  O    ARG F 271   -16.512  66.861 -24.400  1.00 30.23      B000 O
ATOM   8615  CB   ARG F 271   -18.688  67.750 -26.374  1.00 30.54      B000 C
ATOM   8616  CG   ARG F 271   -19.384  68.131 -27.667  1.00 31.79      B000 C
ATOM   8617  CD   ARG F 271   -19.505  69.613 -27.794  1.00 33.75      B000 C
ATOM   8618  NE   ARG F 271   -20.347  70.002 -28.916  1.00 33.09      B000 N
ATOM   8619  CZ   ARG F 271   -20.385  71.243 -29.388  1.00 38.41      B000 C
ATOM   8620  NH1  ARG F 271   -19.614  72.177 -28.817  1.00 34.05      B000 N1+
ATOM   8621  NH2  ARG F 271   -21.173  71.556 -30.417  1.00 29.00      B000 N
ATOM   8622  N    PRO F 272   -16.862  68.955 -23.879  1.00 32.65      B000 N
ATOM   8623  CA   PRO F 272   -16.460  68.718 -22.481  1.00 27.76      B000 C
ATOM   8624  C    PRO F 272   -17.615  68.190 -21.630  1.00 31.78      B000 C
ATOM   8625  O    PRO F 272   -18.061  68.832 -20.676  1.00 29.70      B000 O
ATOM   8626  CB   PRO F 272   -15.983  70.111 -22.042  1.00 28.08      B000 C
ATOM   8627  CG   PRO F 272   -16.860  71.083 -22.841  1.00 28.41      B000 C
ATOM   8628  CD   PRO F 272   -17.059  70.391 -24.190  1.00 31.14      B000 C
ATOM   8629  N    TYR F 273   -18.129  67.005 -21.987  1.00 32.02      B000 N
ATOM   8630  CA   TYR F 273   -19.251  66.438 -21.248  1.00 31.15      B000 C
ATOM   8631  C    TYR F 273   -18.759  65.726 -19.999  1.00 28.60      B000 C
ATOM   8632  O    TYR F 273   -17.577  65.427 -19.850  1.00 27.10      B000 O
ATOM   8633  CB   TYR F 273   -20.061  65.473 -22.113  1.00 28.19      B000 C
ATOM   8634  CG   TYR F 273   -20.762  66.174 -23.244  1.00 30.22      B000 C
ATOM   8635  CD1  TYR F 273   -21.111  67.519 -23.133  1.00 29.80      B000 C
ATOM   8636  CD2  TYR F 273   -21.057  65.512 -24.440  1.00 28.43      B000 C
ATOM   8637  CE1  TYR F 273   -21.765  68.193 -24.178  1.00 29.36      B000 C
ATOM   8638  CE2  TYR F 273   -21.695  66.178 -25.493  1.00 26.89      B000 C
ATOM   8639  CZ   TYR F 273   -22.039  67.519 -25.355  1.00 30.78      B000 C
ATOM   8640  OH   TYR F 273   -22.668  68.191 -26.375  1.00 33.01      B000 O
ATOM   8641  N    ARG F 274   -19.688  65.469 -19.083  1.00 31.67      B000 N
ATOM   8642  CA   ARG F 274   -19.383  64.573 -17.977  1.00 32.33      B000 C
ATOM   8643  C    ARG F 274   -19.177  63.156 -18.523  1.00 29.48      B000 C
ATOM   8644  O    ARG F 274   -19.403  62.875 -19.703  1.00 29.20      B000 O
ATOM   8645  CB   ARG F 274   -20.501  64.618 -16.931  1.00 33.56      B000 C
ATOM   8646  CG   ARG F 274   -20.639  65.990 -16.246  1.00 34.67      B000 C
ATOM   8647  CD   ARG F 274   -21.615  65.998 -15.050  1.00 38.53      B000 C
ATOM   8648  NE   ARG F 274   -21.480  67.235 -14.265  1.00 37.38      B000 N
ATOM   8649  CZ   ARG F 274   -22.106  67.482 -13.117  1.00 34.94      B000 C
ATOM   8650  NH1  ARG F 274   -22.944  66.591 -12.600  1.00 33.17      B000 N1+
ATOM   8651  NH2  ARG F 274   -21.886  68.623 -12.476  1.00 31.06      B000 N
ATOM   8652  N    TRP F 275   -18.741  62.250 -17.662  1.00 29.84      B000 N
ATOM   8653  CA   TRP F 275   -18.518  60.878 -18.100  1.00 34.86      B000 C
ATOM   8654  C    TRP F 275   -18.726  59.935 -16.926  1.00 36.82      B000 C
ATOM   8655  O    TRP F 275   -18.766  60.351 -15.763  1.00 32.31      B000 O
```

```
ATOM   8656  CB  TRP F 275   -17.112 60.684 -18.679  1.00 28.66      B000 C
ATOM   8657  CG  TRP F 275   -16.078 60.676 -17.628  1.00 32.25      B000 C
ATOM   8658  CD1 TRP F 275   -15.531 59.586 -17.019  1.00 34.72      B000 C
ATOM   8659  CD2 TRP F 275   -15.448 61.826 -17.041  1.00 33.73      B000 C
ATOM   8660  NE1 TRP F 275   -14.587 59.989 -16.085  1.00 33.65      B000 N
ATOM   8661  CE2 TRP F 275   -14.521 61.358 -16.087  1.00 32.03      B000 C
ATOM   8662  CE3 TRP F 275   -15.575 63.203 -17.238  1.00 32.63      B000 C
ATOM   8663  CZ2 TRP F 275   -13.732 62.217 -15.334  1.00 34.54      B000 C
ATOM   8664  CZ3 TRP F 275   -14.798 64.057 -16.476  1.00 32.70      B000 C
ATOM   8665  CH2 TRP F 275   -13.890 63.563 -15.542  1.00 33.77      B000 C
ATOM   8666  N   VAL F 276   -18.840 58.646 -17.247  1.00 33.42      B000 N
ATOM   8667  CA  VAL F 276   -19.034 57.591 -16.256  1.00 37.19      B000 C
ATOM   8668  C   VAL F 276   -17.985 56.504 -16.480  1.00 41.17      B000 C
ATOM   8669  O   VAL F 276   -17.762 56.073 -17.620  1.00 38.26      B000 O
ATOM   8670  CB  VAL F 276   -20.454 56.999 -16.338  1.00 37.30      B000 C
ATOM   8671  CG1 VAL F 276   -20.677 55.983 -15.212  1.00 34.94      B000 C
ATOM   8672  CG2 VAL F 276   -21.488 58.117 -16.312  1.00 29.37      B000 C
ATOM   8673  N   CYS F 277   -17.328 56.085 -15.402  1.00 36.51      B000 N
ATOM   8674  CA  CYS F 277   -16.406 54.958 -15.437  1.00 39.62      B000 C
ATOM   8675  C   CYS F 277   -17.094 53.700 -14.909  1.00 45.76      B000 C
ATOM   8676  O   CYS F 277   -17.945 53.759 -14.015  1.00 44.66      B000 O
ATOM   8677  CB  CYS F 277   -15.146 55.230 -14.605  1.00 40.57      B000 C
ATOM   8678  SG  CYS F 277   -14.058 56.553 -15.196  1.00 50.52      B000 S
ATOM   8679  N   GLU F 278   -16.695 52.557 -15.462  1.00 39.15      B000 N
ATOM   8680  CA  GLU F 278   -17.250 51.260 -15.112  1.00 44.84      B000 C
ATOM   8681  C   GLU F 278   -16.120 50.256 -14.936  1.00 47.20      B000 C
ATOM   8682  O   GLU F 278   -15.173 50.230 -15.729  1.00 47.48      B000 O
ATOM   8683  CB  GLU F 278   -18.203 50.751 -16.194  1.00 38.93      B000 C
ATOM   8684  CG  GLU F 278   -18.836 49.416 -15.857  1.00 46.64      B000 C
ATOM   8685  CD  GLU F 278   -19.732 48.897 -16.963  1.00 49.83      B000 C
ATOM   8686  OE1 GLU F 278   -19.861 49.572 -18.007  1.00 51.92      B000 O
ATOM   8687  OE2 GLU F 278   -20.316 47.813 -16.790  1.00 61.81      B000 O1-
ATOM   8688  N   THR F 279   -16.212 49.447 -13.886  1.00 44.44      B000 N
ATOM   8689  CA  THR F 279   -15.287 48.339 -13.689  1.00 49.01      B000 C
ATOM   8690  C   THR F 279   -16.035 47.205 -13.000  1.00 55.21      B000 C
ATOM   8691  O   THR F 279   -17.179 47.359 -12.564  1.00 50.33      B000 O
ATOM   8692  CB  THR F 279   -14.035 48.775 -12.899  1.00 50.55      B000 C
ATOM   8693  OG1 THR F 279   -13.019 47.768 -13.000  1.00 60.70      B000 O
ATOM   8694  CG2 THR F 279   -14.349 49.063 -11.433  1.00 40.44      B000 C
ATOM   8695  N   GLU F 280   -15.392 46.046 -12.929  1.00 62.33      B000 N
ATOM   8696  CA  GLU F 280   -16.042 44.856 -12.389  1.00 60.95      B000 C
ATOM   8697  C   GLU F 280   -15.846 44.774 -10.881  1.00 59.82      B000 C
ATOM   8698  O   GLU F 280   -14.755 45.070 -10.384  1.00 61.25      B000 O
ATOM   8699  CB  GLU F 280   -15.489 43.597 -13.056  1.00 64.94      B000 C
ATOM   8700  CG  GLU F 280   -13.982 43.637 -13.300  1.00 75.16      B000 C
ATOM   8701  CD  GLU F 280   -13.631 44.136 -14.695  1.00 87.07      B000 C
ATOM   8702  OE1 GLU F 280   -14.294 43.684 -15.660  1.00 92.63      B000 O
ATOM   8703  OE2 GLU F 280   -12.707 44.977 -14.825  1.00 87.02      B000 O1-
ATOM   8704  N   GLU F 281   -16.934 44.447 -10.167  1.00 59.93      B000 N
ATOM   8705  CA  GLU F 281   -16.960 43.756  -8.848  1.00 67.16      B000 C
ATOM   8706  C   GLU F 281   -18.241 44.090  -8.095  1.00 67.27      B000 C
ATOM   8707  O   GLU F 281   -18.257 44.100  -6.862  1.00 68.70      B000 O
ATOM   8708  CB  GLU F 281   -15.757 44.085  -7.951  1.00 63.68      B000 C
ATOM   8709  CG  GLU F 281   -15.131 42.885  -7.230  1.00 68.36      B000 C
ATOM   8710  CD  GLU F 281   -14.442 41.881  -8.172  1.00 83.09      B000 C
ATOM   8711  OE1 GLU F 281   -15.127 41.287  -9.037  1.00 77.83      B000 O
ATOM   8712  OE2 GLU F 281   -13.210 41.679  -8.043  1.00 76.05      B000 O
TER
HETATM 9170  C1  GAL G 401    19.131 84.986 -13.073  1.00 67.87           C
HETATM 9171  O1  GAL G 401    19.230 83.939 -14.075  1.00 65.01           O
HETATM 9172  C2  GAL G 401    19.826 84.527 -11.743  1.00 63.05           C
HETATM 9173  O2  GAL G 401    19.202 83.373 -11.240  1.00 42.62           O
HETATM 9174  C3  GAL G 401    19.822 85.673 -10.639  1.00 71.79           C
HETATM 9175  O3  GAL G 401    20.324 85.299  -9.317  1.00 63.98           O
HETATM 9176  C4  GAL G 401    20.547 86.954 -11.192  1.00 74.66           C
HETATM 9177  O4  GAL G 401    21.936 86.736 -11.495  1.00 79.63           O
HETATM 9178  C5  GAL G 401    19.774 87.370 -12.469  1.00 75.84           C
HETATM 9179  O5  GAL G 401    19.667 86.275 -13.538  1.00 68.13           O
HETATM 9180  C6  GAL G 401    20.270 88.659 -13.125  1.00 68.64           C
HETATM 9181  O6  GAL G 401    19.157 89.413 -13.631  1.00 52.02           O
TER
```

```
HETATM 9182  C1   GAL G 402    -25.502  71.256 -32.056  1.00 74.77          C
HETATM 9183  O1   GAL G 402    -24.812  72.366 -32.713  1.00 60.32          O
HETATM 9184  C2   GAL G 402    -26.779  71.727 -31.263  1.00 62.62          C
HETATM 9185  O2   GAL G 402    -26.494  72.728 -30.323  1.00 41.25          O
HETATM 9186  C3   GAL G 402    -27.470  70.522 -30.515  1.00 66.23          C
HETATM 9187  O3   GAL G 402    -28.624  70.910 -29.737  1.00 63.41          O
HETATM 9188  C4   GAL G 402    -27.905  69.432 -31.526  1.00 75.11          C
HETATM 9189  O4   GAL G 402    -29.064  69.854 -32.279  1.00 80.69          O
HETATM 9190  C5   GAL G 402    -26.702  69.051 -32.464  1.00 82.81          C
HETATM 9191  O5   GAL G 402    -25.897  70.216 -33.013  1.00 81.70          O
HETATM 9192  C6   GAL G 402    -27.102  68.166 -33.664  1.00 85.18          C
HETATM 9193  O6   GAL G 402    -26.406  68.545 -34.844  1.00 94.12          O
TER
HETATM 9194  CA   CA  H   1     21.469  91.701 -14.212  1.00 79.84          Ca
TER
HETATM 9195  CA   CA  H   2    -26.073  64.419 -34.040  1.00 79.26          Ca
TER
HETATM 9196  CA   CA  H   3    -18.333  61.223 -36.994  1.00 75.64          Ca
HETATM 9197  CA   CA  H   4     18.240  94.784 -21.928  1.00 73.55          Ca
TER
```

## Table 10.4

| ATOM | 1 | O | GLN A | 1 | -24.853 | -26.439 | 84.334 | 1.00 | 39.67 | O |
|------|---|-----|-------|---|---------|---------|--------|------|-------|---|
| ATOM | 2 | N | GLN A | 1 | -23.947 | -29.300 | 85.222 | 1.00 | 49.60 | N |
| ATOM | 3 | CA | GLN A | 1 | -24.872 | -28.345 | 85.852 | 1.00 | 56.88 | C |
| ATOM | 4 | C | GLN A | 1 | -25.501 | -27.209 | 85.050 | 1.00 | 51.47 | C |
| ATOM | 5 | CB | GLN A | 1 | -24.208 | -27.745 | 87.074 | 1.00 | 45.63 | C |
| ATOM | 6 | CG | GLN A | 1 | -23.967 | -28.823 | 88.054 | 1.00 | 44.21 | C |
| ATOM | 7 | CD | GLN A | 1 | -25.274 | -29.457 | 88.407 | 1.00 | 54.28 | C |
| ATOM | 8 | OE1 | GLN A | 1 | -26.030 | -28.899 | 89.200 | 1.00 | 62.35 | O |
| ATOM | 9 | NE2 | GLN A | 1 | -25.592 | -30.592 | 87.778 | 1.00 | 57.03 | N |
| ATOM | 10 | N | VAL A | 2 | -26.812 | -27.135 | 85.264 | 1.00 | 51.10 | N |
| ATOM | 11 | CA | VAL A | 2 | -27.671 | -26.124 | 84.683 | 1.00 | 42.93 | C |
| ATOM | 12 | C | VAL A | 2 | -27.754 | -24.968 | 85.669 | 1.00 | 44.75 | C |
| ATOM | 13 | O | VAL A | 2 | -28.236 | -25.134 | 86.793 | 1.00 | 42.04 | O |
| ATOM | 14 | CB | VAL A | 2 | -29.061 | -26.696 | 84.378 | 1.00 | 39.51 | C |
| ATOM | 15 | CG1 | VAL A | 2 | -29.809 | -25.773 | 83.468 | 1.00 | 44.64 | C |
| ATOM | 16 | CG2 | VAL A | 2 | -28.948 | -28.065 | 83.754 | 1.00 | 39.26 | C |
| ATOM | 17 | N | GLN A | 3 | -27.240 | -23.812 | 85.269 | 1.00 | 45.15 | N |
| ATOM | 18 | CA | GLN A | 3 | -27.403 | -22.586 | 86.031 | 1.00 | 44.60 | C |
| ATOM | 19 | C | GLN A | 3 | -28.455 | -21.725 | 85.355 | 1.00 | 34.85 | C |
| ATOM | 20 | O | GLN A | 3 | -28.496 | -21.629 | 84.128 | 1.00 | 39.72 | O |
| ATOM | 21 | CB | GLN A | 3 | -26.091 | -21.792 | 86.128 | 1.00 | 50.01 | C |
| ATOM | 22 | CG | GLN A | 3 | -24.978 | -22.456 | 86.931 | 1.00 | 56.60 | C |
| ATOM | 23 | CD | GLN A | 3 | -23.919 | -23.128 | 86.052 | 1.00 | 59.49 | C |
| ATOM | 24 | OE1 | GLN A | 3 | -24.036 | -23.141 | 84.811 | 1.00 | 56.42 | O |
| ATOM | 25 | NE2 | GLN A | 3 | -22.874 | -23.691 | 86.692 | 1.00 | 48.31 | N |
| ATOM | 26 | N | LEU A | 4 | -29.296 | -21.109 | 86.161 | 1.00 | 34.42 | N |
| ATOM | 27 | CA | LEU A | 4 | -30.179 | -20.024 | 85.752 | 1.00 | 31.59 | C |
| ATOM | 28 | C | LEU A | 4 | -29.640 | -18.812 | 86.491 | 1.00 | 37.33 | C |
| ATOM | 29 | O | LEU A | 4 | -29.854 | -18.673 | 87.696 | 1.00 | 44.24 | O |
| ATOM | 30 | CB | LEU A | 4 | -31.634 | -20.283 | 86.124 | 1.00 | 31.99 | C |
| ATOM | 31 | CG | LEU A | 4 | -32.309 | -21.572 | 85.670 | 1.00 | 34.73 | C |
| ATOM | 32 | CD1 | LEU A | 4 | -33.852 | -21.433 | 85.729 | 1.00 | 32.19 | C |
| ATOM | 33 | CD2 | LEU A | 4 | -31.838 | -21.916 | 84.287 | 1.00 | 30.49 | C |
| ATOM | 34 | N | GLN A | 5 | -28.883 | -17.976 | 85.797 | 1.00 | 40.51 | N |
| ATOM | 35 | CA | GLN A | 5 | -28.269 | -16.810 | 86.412 | 1.00 | 38.81 | C |
| ATOM | 36 | C | GLN A | 5 | -29.203 | -15.614 | 86.259 | 1.00 | 37.64 | C |
| ATOM | 37 | O | GLN A | 5 | -29.714 | -15.358 | 85.170 | 1.00 | 41.01 | O |
| ATOM | 38 | CB | GLN A | 5 | -26.911 | -16.544 | 85.768 | 1.00 | 38.94 | C |
| ATOM | 39 | CG | GLN A | 5 | -26.103 | -17.812 | 85.595 | 1.00 | 46.98 | C |
| ATOM | 40 | CD | GLN A | 5 | -24.690 | -17.565 | 85.045 | 1.00 | 61.68 | C |
| ATOM | 41 | OE1 | GLN A | 5 | -24.523 | -17.011 | 83.956 | 1.00 | 63.24 | O |
| ATOM | 42 | NE2 | GLN A | 5 | -23.671 | -17.982 | 85.800 | 1.00 | 53.29 | N |
| ATOM | 43 | N | GLN A | 6 | -29.465 | -14.912 | 87.348 | 1.00 | 37.71 | N |
| ATOM | 44 | CA | GLN A | 6 | -30.413 | -13.810 | 87.326 | 1.00 | 37.43 | C |
| ATOM | 45 | C | GLN A | 6 | -29.670 | -12.491 | 87.484 | 1.00 | 41.79 | C |
| ATOM | 46 | O | GLN A | 6 | -28.694 | -12.405 | 88.237 | 1.00 | 42.07 | O |
| ATOM | 47 | CB | GLN A | 6 | -31.445 | -13.932 | 88.448 | 1.00 | 34.57 | C |
| ATOM | 48 | CG | GLN A | 6 | -32.083 | -15.276 | 88.561 | 1.00 | 37.33 | C |
| ATOM | 49 | CD | GLN A | 6 | -33.308 | -15.279 | 89.458 | 1.00 | 36.27 | C |
| ATOM | 50 | OE1 | GLN A | 6 | -33.695 | -16.319 | 89.966 | 1.00 | 30.06 | O |
| ATOM | 51 | NE2 | GLN A | 6 | -33.920 | -14.117 | 89.653 | 1.00 | 32.89 | N |
| ATOM | 52 | N | TRP A | 7 | -30.144 | -11.463 | 86.785 | 1.00 | 36.25 | N |
| ATOM | 53 | CA | TRP A | 7 | -29.729 | -10.098 | 87.059 | 1.00 | 33.75 | C |
| ATOM | 54 | C | TRP A | 7 | -30.876 | -9.160 | 86.714 | 1.00 | 37.39 | C |
| ATOM | 55 | O | TRP A | 7 | -31.883 | -9.557 | 86.120 | 1.00 | 39.47 | O |
| ATOM | 56 | CB | TRP A | 7 | -28.452 | -9.723 | 86.303 | 1.00 | 33.51 | C |
| ATOM | 57 | CG | TRP A | 7 | -28.542 | -9.763 | 84.822 | 1.00 | 34.91 | C |
| ATOM | 58 | CD1 | TRP A | 7 | -28.929 | -8.739 | 83.990 | 1.00 | 37.52 | C |
| ATOM | 59 | CD2 | TRP A | 7 | -28.206 | -10.865 | 83.970 | 1.00 | 37.56 | C |
| ATOM | 60 | NE1 | TRP A | 7 | -28.872 | -9.149 | 82.675 | 1.00 | 37.51 | N |
| ATOM | 61 | CE2 | TRP A | 7 | -28.425 | -10.446 | 82.635 | 1.00 | 36.81 | C |
| ATOM | 62 | CE3 | TRP A | 7 | -27.737 | -12.160 | 84.204 | 1.00 | 43.59 | C |
| ATOM | 63 | CZ2 | TRP A | 7 | -28.199 | -11.280 | 81.542 | 1.00 | 35.12 | C |
| ATOM | 64 | CZ3 | TRP A | 7 | -27.517 | -12.993 | 83.112 | 1.00 | 48.14 | C |
| ATOM | 65 | CH2 | TRP A | 7 | -27.754 | -12.547 | 81.797 | 1.00 | 43.93 | C |
| ATOM | 66 | N | GLY A | 8 | -30.711 | -7.909 | 87.092 | 1.00 | 31.94 | N |
| ATOM | 67 | CA | GLY A | 8 | -31.730 | -6.894 | 86.915 | 1.00 | 32.15 | C |
| ATOM | 68 | C | GLY A | 8 | -31.597 | -6.027 | 88.146 | 1.00 | 36.03 | C |
| ATOM | 69 | O | GLY A | 8 | -31.196 | -6.479 | 89.217 | 1.00 | 34.32 | O |

| ATOM | 70 | N | ALA A | 9 | -31.911 | -4.745 | 87.991 | 1.00 39.30 | N |
|------|-----|-----|-------|----|---------|--------|--------|------------|-----|
| ATOM | 71 | CA | ALA A | 9 | -31.830 | -3.844 | 89.129 | 1.00 40.16 | C |
| ATOM | 72 | C | ALA A | 9 | -32.853 | -4.275 | 90.168 | 1.00 41.09 | C |
| ATOM | 73 | O | ALA A | 9 | -34.026 | -4.460 | 89.845 | 1.00 43.60 | O |
| ATOM | 74 | CB | ALA A | 9 | -32.073 | -2.399 | 88.690 | 1.00 33.25 | C |
| ATOM | 75 | N | GLY A | 10 | -32.405 | -4.458 | 91.411 | 1.00 37.64 | N |
| ATOM | 76 | CA | GLY A | 10 | -33.275 | -4.938 | 92.461 | 1.00 28.47 | C |
| ATOM | 77 | C | GLY A | 10 | -33.861 | -3.889 | 93.382 | 1.00 36.57 | C |
| ATOM | 78 | O | GLY A | 10 | -34.710 | -4.214 | 94.213 | 1.00 43.35 | O |
| ATOM | 79 | N | LEU A | 11 | -33.418 | -2.638 | 93.273 | 1.00 36.99 | N |
| ATOM | 80 | CA | LEU A | 11 | -33.946 | -1.533 | 94.067 | 1.00 34.54 | C |
| ATOM | 81 | C | LEU A | 11 | -34.748 | -0.595 | 93.168 | 1.00 35.85 | C |
| ATOM | 82 | O | LEU A | 11 | -34.244 | -0.129 | 92.144 | 1.00 38.05 | O |
| ATOM | 83 | CB | LEU A | 11 | -32.818 | -0.764 | 94.760 | 1.00 36.89 | C |
| ATOM | 84 | CG | LEU A | 11 | -33.040 | -0.447 | 96.238 | 1.00 40.06 | C |
| ATOM | 85 | CD1 | LEU A | 11 | -31.989 | 0.520 | 96.748 | 1.00 38.21 | C |
| ATOM | 86 | CD2 | LEU A | 11 | -34.443 | 0.097 | 96.487 | 1.00 39.59 | C |
| ATOM | 87 | N | LEU A | 12 | -35.997 | -0.333 | 93.537 | 1.00 36.22 | N |
| ATOM | 88 | CA | LEU A | 12 | -36.874 | 0.495 | 92.724 | 1.00 38.68 | C |
| ATOM | 89 | C | LEU A | 12 | -37.710 | 1.388 | 93.624 | 1.00 37.27 | C |
| ATOM | 90 | O | LEU A | 12 | -37.955 | 1.071 | 94.791 | 1.00 40.06 | O |
| ATOM | 91 | CB | LEU A | 12 | -37.815 | -0.342 | 91.843 | 1.00 36.78 | C |
| ATOM | 92 | CG | LEU A | 12 | -37.179 | -1.328 | 90.872 | 1.00 36.77 | C |
| ATOM | 93 | CD1 | LEU A | 12 | -38.285 | -2.117 | 90.244 | 1.00 42.02 | C |
| ATOM | 94 | CD2 | LEU A | 12 | -36.382 | -0.610 | 89.812 | 1.00 40.37 | C |
| ATOM | 95 | N | LYS A | 13 | -38.164 | 2.508 | 93.053 | 1.00 36.92 | N |
| ATOM | 96 | CA | LYS A | 13 | -39.141 | 3.391 | 93.665 | 1.00 36.42 | C |
| ATOM | 97 | C | LYS A | 13 | -40.505 | 3.163 | 93.036 | 1.00 35.60 | C |
| ATOM | 98 | O | LYS A | 13 | -40.605 | 2.637 | 91.921 | 1.00 39.97 | O |
| ATOM | 99 | CB | LYS A | 13 | -38.711 | 4.848 | 93.499 | 1.00 41.56 | C |
| ATOM | 100 | CG | LYS A | 13 | -37.227 | 5.088 | 93.781 | 1.00 52.81 | C |
| ATOM | 101 | CD | LYS A | 13 | -36.541 | 5.678 | 92.538 | 1.00 68.42 | C |
| ATOM | 102 | CE | LYS A | 13 | -35.056 | 5.297 | 92.399 | 1.00 70.40 | C |
| ATOM | 103 | NZ | LYS A | 13 | -34.516 | 5.679 | 91.038 | 1.00 67.86 | N1+ |
| ATOM | 104 | N | PRO A | 14 | -41.587 | 3.512 | 93.736 | 1.00 32.85 | N |
| ATOM | 105 | CA | PRO A | 14 | -42.932 | 3.257 | 93.200 | 1.00 31.49 | C |
| ATOM | 106 | C | PRO A | 14 | -43.114 | 3.852 | 91.806 | 1.00 38.32 | C |
| ATOM | 107 | O | PRO A | 14 | -42.521 | 4.880 | 91.465 | 1.00 35.32 | O |
| ATOM | 108 | CB | PRO A | 14 | -43.852 | 3.929 | 94.218 | 1.00 25.23 | C |
| ATOM | 109 | CG | PRO A | 14 | -43.104 | 3.927 | 95.441 | 1.00 29.72 | C |
| ATOM | 110 | CD | PRO A | 14 | -41.646 | 4.040 | 95.105 | 1.00 33.69 | C |
| ATOM | 111 | N | SER A | 15 | -43.920 | 3.155 | 90.996 | 1.00 36.29 | N |
| ATOM | 112 | CA | SER A | 15 | -44.274 | 3.500 | 89.622 | 1.00 33.19 | C |
| ATOM | 113 | C | SER A | 15 | -43.184 | 3.147 | 88.614 | 1.00 34.56 | C |
| ATOM | 114 | O | SER A | 15 | -43.472 | 3.106 | 87.414 | 1.00 34.31 | O |
| ATOM | 115 | CB | SER A | 15 | -44.597 | 4.994 | 89.468 | 1.00 38.65 | C |
| ATOM | 116 | OG | SER A | 15 | -43.421 | 5.751 | 89.168 | 1.00 34.68 | O |
| ATOM | 117 | N | GLU A | 16 | -41.943 | 2.899 | 89.051 | 1.00 30.66 | N |
| ATOM | 118 | CA | GLU A | 16 | -40.952 | 2.443 | 88.083 | 1.00 28.63 | C |
| ATOM | 119 | C | GLU A | 16 | -41.330 | 1.055 | 87.551 | 1.00 33.32 | C |
| ATOM | 120 | O | GLU A | 16 | -42.281 | 0.410 | 88.010 | 1.00 35.10 | O |
| ATOM | 121 | CB | GLU A | 16 | -39.539 | 2.437 | 88.684 | 1.00 30.89 | C |
| ATOM | 122 | CG | GLU A | 16 | -39.075 | 3.729 | 89.360 | 1.00 31.88 | C |
| ATOM | 123 | CD | GLU A | 16 | -37.549 | 3.780 | 89.616 | 1.00 49.97 | C |
| ATOM | 124 | OE1 | GLU A | 16 | -36.907 | 2.727 | 89.843 | 1.00 54.84 | O |
| ATOM | 125 | OE2 | GLU A | 16 | -36.967 | 4.885 | 89.556 | 1.00 57.53 | O |
| ATOM | 126 | N | THR A | 17 | -40.583 | 0.583 | 86.565 | 1.00 32.70 | N |
| ATOM | 127 | CA | THR A | 17 | -40.829 | -0.744 | 86.034 | 1.00 35.01 | C |
| ATOM | 128 | C | THR A | 17 | -39.637 | -1.655 | 86.318 | 1.00 38.16 | C |
| ATOM | 129 | O | THR A | 17 | -38.473 | -1.238 | 86.259 | 1.00 36.52 | O |
| ATOM | 130 | CB | THR A | 17 | -41.198 | -0.690 | 84.543 | 1.00 36.57 | C |
| ATOM | 131 | OG1 | THR A | 17 | -40.325 | -1.515 | 83.766 | 1.00 34.00 | O |
| ATOM | 132 | CG2 | THR A | 17 | -41.198 | 0.711 | 84.055 | 1.00 37.19 | C |
| ATOM | 133 | N | LEU A | 18 | -39.955 | -2.874 | 86.733 | 1.00 35.90 | N |
| ATOM | 134 | CA | LEU A | 18 | -38.969 | -3.873 | 87.100 | 1.00 33.49 | C |
| ATOM | 135 | C | LEU A | 18 | -38.603 | -4.669 | 85.861 | 1.00 32.72 | C |
| ATOM | 136 | O | LEU A | 18 | -39.482 | -5.056 | 85.082 | 1.00 30.94 | O |
| ATOM | 137 | CB | LEU A | 18 | -39.537 | -4.770 | 88.209 | 1.00 34.34 | C |
| ATOM | 138 | CG | LEU A | 18 | -38.899 | -6.037 | 88.790 | 1.00 34.31 | C |
| ATOM | 139 | CD1 | LEU A | 18 | -39.067 | -7.231 | 87.855 | 1.00 31.61 | C |
| ATOM | 140 | CD2 | LEU A | 18 | -37.433 | -5.823 | 89.128 | 1.00 34.33 | C |

```
ATOM    141  N    SER A  19    -37.309   -4.888   85.660  1.00 31.86        N
ATOM    142  CA   SER A  19    -36.877   -5.700   84.536  1.00 35.19        C
ATOM    143  C    SER A  19    -35.754   -6.613   85.001  1.00 31.16        C
ATOM    144  O    SER A  19    -34.777   -6.145   85.596  1.00 30.06        O
ATOM    145  CB   SER A  19    -36.449   -4.841   83.346  1.00 33.07        C
ATOM    146  OG   SER A  19    -35.218   -4.239   83.612  1.00 47.41        O
ATOM    147  N    LEU A  20    -35.934   -7.916   84.768  1.00 25.55        N
ATOM    148  CA   LEU A  20    -35.022   -8.962   85.214  1.00 28.53        C
ATOM    149  C    LEU A  20    -34.777   -9.941   84.074  1.00 27.96        C
ATOM    150  O    LEU A  20    -35.643  -10.142   83.220  1.00 27.00        O
ATOM    151  CB   LEU A  20    -35.596   -9.712   86.425  1.00 26.52        C
ATOM    152  CG   LEU A  20    -35.939   -8.899   87.678  1.00 29.44        C
ATOM    153  CD1  LEU A  20    -36.684   -9.780   88.650  1.00 34.23        C
ATOM    154  CD2  LEU A  20    -34.683   -8.369   88.365  1.00 31.36        C
ATOM    155  N    THR A  21    -33.589  -10.552   84.061  1.00 28.15        N
ATOM    156  CA   THR A  21    -33.237  -11.526   83.034  1.00 30.18        C
ATOM    157  C    THR A  21    -32.621  -12.772   83.663  1.00 33.03        C
ATOM    158  O    THR A  21    -31.918  -12.678   84.673  1.00 38.27        O
ATOM    159  CB   THR A  21    -32.240  -10.913   82.023  1.00 32.85        C
ATOM    160  OG1  THR A  21    -32.757   -9.670   81.536  1.00 32.16        O
ATOM    161  CG2  THR A  21    -31.977  -11.866   80.841  1.00 28.45        C
ATOM    162  N    CYS A  22    -32.883  -13.939   83.053  1.00 27.52        N
ATOM    163  CA   CYS A  22    -32.197  -15.190   83.374  1.00 32.00        C
ATOM    164  C    CYS A  22    -31.452  -15.675   82.149  1.00 29.74        C
ATOM    165  O    CYS A  22    -31.974  -15.629   81.036  1.00 26.79        O
ATOM    166  CB   CYS A  22    -33.116  -16.343   83.872  1.00 27.00        C
ATOM    167  SG   CYS A  22    -33.354  -16.118   85.624  1.00 68.31        S
ATOM    168  N    ALA A  23    -30.228  -16.135   82.379  1.00 32.24        N
ATOM    169  CA   ALA A  23    -29.397  -16.762   81.370  1.00 32.34        C
ATOM    170  C    ALA A  23    -29.311  -18.234   81.721  1.00 30.10        C
ATOM    171  O    ALA A  23    -29.020  -18.577   82.867  1.00 34.73        O
ATOM    172  CB   ALA A  23    -28.004  -16.125   81.339  1.00 32.68        C
ATOM    173  N    VAL A  24    -29.614  -19.093   80.763  1.00 27.15        N
ATOM    174  CA   VAL A  24    -29.616  -20.533   80.977  1.00 32.40        C
ATOM    175  C    VAL A  24    -28.371  -21.124   80.342  1.00 32.49        C
ATOM    176  O    VAL A  24    -28.115  -20.916   79.155  1.00 31.92        O
ATOM    177  CB   VAL A  24    -30.872  -21.204   80.377  1.00 31.63        C
ATOM    178  CG1  VAL A  24    -30.833  -22.694   80.642  1.00 23.27        C
ATOM    179  CG2  VAL A  24    -32.187  -20.590   80.898  1.00 27.72        C
ATOM    180  N    SER A  25    -27.609  -21.875   81.117  1.00 37.38        N
ATOM    181  CA   SER A  25    -26.471  -22.620   80.602  1.00 34.69        C
ATOM    182  C    SER A  25    -26.544  -24.037   81.136  1.00 37.68        C
ATOM    183  O    SER A  25    -27.107  -24.279   82.206  1.00 45.11        O
ATOM    184  CB   SER A  25    -25.146  -21.979   81.013  1.00 35.05        C
ATOM    185  OG   SER A  25    -25.168  -21.665   82.396  1.00 40.52        O
ATOM    186  N    GLY A  26    -25.978  -24.977   80.405  1.00 31.17        N
ATOM    187  CA   GLY A  26    -25.970  -26.319   80.939  1.00 34.95        C
ATOM    188  C    GLY A  26    -26.773  -27.339   80.181  1.00 44.75        C
ATOM    189  O    GLY A  26    -26.261  -28.422   79.895  1.00 53.93        O
ATOM    190  N    GLY A  27    -28.035  -27.049   79.897  1.00 43.99        N
ATOM    191  CA   GLY A  27    -28.827  -28.022   79.179  1.00 39.13        C
ATOM    192  C    GLY A  27    -29.426  -27.412   77.935  1.00 44.00        C
ATOM    193  O    GLY A  27    -28.940  -26.394   77.426  1.00 44.61        O
ATOM    194  N    SER A  28    -30.525  -27.991   77.475  1.00 48.00        N
ATOM    195  CA   SER A  28    -31.204  -27.507   76.286  1.00 42.16        C
ATOM    196  C    SER A  28    -32.051  -26.285   76.611  1.00 41.49        C
ATOM    197  O    SER A  28    -32.302  -25.961   77.778  1.00 37.48        O
ATOM    198  CB   SER A  28    -32.071  -28.605   75.684  1.00 39.00        C
ATOM    199  OG   SER A  28    -31.253  -29.679   75.263  1.00 45.34        O
ATOM    200  N    PHE A  29    -32.448  -25.569   75.552  1.00 39.22        N
ATOM    201  CA   PHE A  29    -33.327  -24.425   75.695  1.00 34.42        C
ATOM    202  C    PHE A  29    -34.696  -24.653   75.097  1.00 38.24        C
ATOM    203  O    PHE A  29    -35.628  -23.923   75.439  1.00 40.29        O
ATOM    204  CB   PHE A  29    -32.710  -23.181   75.041  1.00 30.96        C
ATOM    205  CG   PHE A  29    -33.191  -21.874   75.633  1.00 31.50        C
ATOM    206  CD1  PHE A  29    -33.148  -21.663   77.004  1.00 33.59        C
ATOM    207  CD2  PHE A  29    -33.660  -20.850   74.823  1.00 28.85        C
ATOM    208  CE1  PHE A  29    -33.578  -20.450   77.564  1.00 31.05        C
ATOM    209  CE2  PHE A  29    -34.073  -19.653   75.359  1.00 29.76        C
ATOM    210  CZ   PHE A  29    -34.042  -19.449   76.740  1.00 30.19        C
ATOM    211  N    ARG A  30    -34.855  -25.653   74.242  1.00 41.39        N
```

```
ATOM   212  CA   ARG A  30     -36.009 -25.653  73.362  1.00 44.20           C
ATOM   213  C    ARG A  30     -37.145 -26.551  73.833  1.00 39.55           C
ATOM   214  O    ARG A  30     -38.265 -26.412  73.331  1.00 42.77           O
ATOM   215  CB   ARG A  30     -35.568 -26.002  71.939  1.00 31.85           C
ATOM   216  CG   ARG A  30     -35.075 -27.382  71.728  1.00 39.91           C
ATOM   217  CD   ARG A  30     -34.814 -27.548  70.245  1.00 43.27           C
ATOM   218  NE   ARG A  30     -33.604 -28.305  69.892  1.00 49.24           N
ATOM   219  CZ   ARG A  30     -32.393 -28.213  70.464  1.00 57.67           C
ATOM   220  NH1  ARG A  30     -31.409 -28.969  69.985  1.00 63.98           N
ATOM   221  NH2  ARG A  30     -32.135 -27.413  71.512  1.00 54.11           N
ATOM   222  N    TYR A  31     -36.902 -27.434  74.789  1.00 36.60           N
ATOM   223  CA   TYR A  31     -37.940 -28.340  75.264  1.00 40.80           C
ATOM   224  C    TYR A  31     -38.685 -27.821  76.489  1.00 37.66           C
ATOM   225  O    TYR A  31     -39.628 -28.470  76.946  1.00 36.93           O
ATOM   226  CB   TYR A  31     -37.334 -29.699  75.619  1.00 37.87           C
ATOM   227  CG   TYR A  31     -36.422 -30.240  74.573  1.00 39.45           C
ATOM   228  CD1  TYR A  31     -36.888 -30.500  73.300  1.00 42.59           C
ATOM   229  CD2  TYR A  31     -35.088 -30.528  74.861  1.00 44.81           C
ATOM   230  CE1  TYR A  31     -36.047 -31.005  72.325  1.00 45.84           C
ATOM   231  CE2  TYR A  31     -34.240 -31.049  73.890  1.00 43.49           C
ATOM   232  CZ   TYR A  31     -34.729 -31.281  72.625  1.00 43.29           C
ATOM   233  OH   TYR A  31     -33.922 -31.803  71.648  1.00 43.72           O
ATOM   234  N    TYR A  32     -38.302 -26.677  77.027  1.00 39.36           N
ATOM   235  CA   TYR A  32     -38.735 -26.280  78.350  1.00 34.43           C
ATOM   236  C    TYR A  32     -39.587 -25.028  78.263  1.00 38.74           C
ATOM   237  O    TYR A  32     -39.501 -24.256  77.305  1.00 39.83           O
ATOM   238  CB   TYR A  32     -37.533 -26.028  79.265  1.00 33.39           C
ATOM   239  CG   TYR A  32     -36.634 -27.226  79.343  1.00 38.75           C
ATOM   240  CD2  TYR A  32     -35.377 -27.236  78.750  1.00 32.24           C
ATOM   241  CD1  TYR A  32     -37.061 -28.368  80.004  1.00 38.64           C
ATOM   242  CE2  TYR A  32     -34.570 -28.364  78.823  1.00 39.82           C
ATOM   243  CE1  TYR A  32     -36.270 -29.495  80.088  1.00 40.18           C
ATOM   244  CZ   TYR A  32     -35.029 -29.505  79.499  1.00 46.72           C
ATOM   245  OH   TYR A  32     -34.278 -30.668  79.604  1.00 41.88           O
ATOM   246  N    TYR A  33     -40.420 -24.842  79.282  1.00 31.10           N
ATOM   247  CA   TYR A  33     -41.042 -23.558  79.529  1.00 30.58           C
ATOM   248  C    TYR A  33     -40.221 -22.800  80.563  1.00 30.77           C
ATOM   249  O    TYR A  33     -39.686 -23.392  81.507  1.00 29.88           O
ATOM   250  CB   TYR A  33     -42.491 -23.726  79.987  1.00 30.38           C
ATOM   251  CG   TYR A  33     -43.440 -23.801  78.834  1.00 33.35           C
ATOM   252  CD2  TYR A  33     -44.211 -22.698  78.485  1.00 33.18           C
ATOM   253  CD1  TYR A  33     -43.550 -24.966  78.059  1.00 33.82           C
ATOM   254  CE2  TYR A  33     -45.080 -22.746  77.407  1.00 36.40           C
ATOM   255  CE1  TYR A  33     -44.430 -25.030  76.976  1.00 33.85           C
ATOM   256  CZ   TYR A  33     -45.186 -23.909  76.655  1.00 39.33           C
ATOM   257  OH   TYR A  33     -46.051 -23.924  75.590  1.00 39.05           O
ATOM   258  N    TRP A  34     -40.133 -21.490  80.381  1.00 25.37           N
ATOM   259  CA   TRP A  34     -39.285 -20.639  81.189  1.00 25.30           C
ATOM   260  C    TRP A  34     -40.172 -19.666  81.940  1.00 30.32           C
ATOM   261  O    TRP A  34     -41.013 -18.993  81.328  1.00 28.04           O
ATOM   262  CB   TRP A  34     -38.242 -19.932  80.310  1.00 27.04           C
ATOM   263  CG   TRP A  34     -37.375 -20.952  79.641  1.00 28.16           C
ATOM   264  CD1  TRP A  34     -37.436 -21.371  78.333  1.00 31.12           C
ATOM   265  CD2  TRP A  34     -36.410 -21.787  80.279  1.00 28.13           C
ATOM   266  NE1  TRP A  34     -36.525 -22.375  78.110  1.00 27.45           N
ATOM   267  CE2  TRP A  34     -35.881 -22.653  79.287  1.00 31.81           C
ATOM   268  CE3  TRP A  34     -35.920 -21.875  81.587  1.00 28.15           C
ATOM   269  CZ2  TRP A  34     -34.880 -23.591  79.567  1.00 31.29           C
ATOM   270  CZ3  TRP A  34     -34.911 -22.807  81.864  1.00 29.29           C
ATOM   271  CH2  TRP A  34     -34.411 -23.654  80.858  1.00 25.81           C
ATOM   272  N    SER A  35     -39.993 -19.608  83.268  1.00 28.16           N
ATOM   273  CA   SER A  35     -41.009 -19.048  84.150  1.00 29.83           C
ATOM   274  C    SER A  35     -40.463 -17.999  85.106  1.00 27.98           C
ATOM   275  O    SER A  35     -39.266 -17.907  85.361  1.00 25.65           O
ATOM   276  CB   SER A  35     -41.677 -20.145  84.957  1.00 25.41           C
ATOM   277  OG   SER A  35     -42.298 -21.049  84.068  1.00 33.06           O
ATOM   278  N    TRP A  36     -41.388 -17.224  85.655  1.00 26.15           N
ATOM   279  CA   TRP A  36     -41.091 -16.278  86.712  1.00 23.95           C
ATOM   280  C    TRP A  36     -42.037 -16.535  87.877  1.00 26.92           C
ATOM   281  O    TRP A  36     -43.249 -16.680  87.693  1.00 24.36           O
ATOM   282  CB   TRP A  36     -41.200 -14.847  86.204  1.00 21.96           C
```

```
ATOM    283  CG   TRP A  36     -40.123 -14.545  85.243  1.00 27.15           C
ATOM    284  CD1  TRP A  36     -40.208 -14.586  83.880  1.00 28.66           C
ATOM    285  CD2  TRP A  36     -38.764 -14.186  85.550  1.00 30.43           C
ATOM    286  NE1  TRP A  36     -38.998 -14.263  83.319  1.00 28.45           N
ATOM    287  CE2  TRP A  36     -38.092 -14.011  84.318  1.00 31.09           C
ATOM    288  CE3  TRP A  36     -38.051 -13.988  86.744  1.00 26.18           C
ATOM    289  CZ2  TRP A  36     -36.736 -13.635  84.245  1.00 30.83           C
ATOM    290  CZ3  TRP A  36     -36.710 -13.624  86.669  1.00 31.94           C
ATOM    291  CH2  TRP A  36     -36.065 -13.444  85.426  1.00 27.34           C
ATOM    292  N    ILE A  37     -41.470 -16.623  89.072  1.00 28.61           N
ATOM    293  CA   ILE A  37     -42.220 -16.887  90.289  1.00 25.71           C
ATOM    294  C    ILE A  37     -41.703 -15.924  91.344  1.00 26.51           C
ATOM    295  O    ILE A  37     -40.485 -15.758  91.473  1.00 28.46           O
ATOM    296  CB   ILE A  37     -42.043 -18.359  90.726  1.00 28.49           C
ATOM    297  CG1  ILE A  37     -42.496 -19.298  89.602  1.00 20.22           C
ATOM    298  CG2  ILE A  37     -42.842 -18.657  91.998  1.00 30.40           C
ATOM    299  CD1  ILE A  37     -41.935 -20.641  89.672  1.00 19.85           C
ATOM    300  N    ARG A  38     -42.608 -15.271  92.089  1.00 27.82           N
ATOM    301  CA   ARG A  38     -42.178 -14.383  93.172  1.00 31.79           C
ATOM    302  C    ARG A  38     -42.654 -14.877  94.535  1.00 29.28           C
ATOM    303  O    ARG A  38     -43.711 -15.493  94.666  1.00 30.31           O
ATOM    304  CB   ARG A  38     -42.642 -12.902  92.983  1.00 25.98           C
ATOM    305  CG   ARG A  38     -44.132 -12.704  92.827  1.00 30.50           C
ATOM    306  CD   ARG A  38     -44.718 -11.710  93.796  1.00 31.27           C
ATOM    307  NE   ARG A  38     -44.660 -10.332  93.330  1.00 38.33           N
ATOM    308  CZ   ARG A  38     -45.723  -9.550  93.118  1.00 40.05           C
ATOM    309  NH1  ARG A  38     -46.967  -9.987  93.311  1.00 29.85           N1+
ATOM    310  NH2  ARG A  38     -45.529  -8.307  92.711  1.00 35.67           N
ATOM    311  N    GLN A  39     -41.874 -14.544  95.561  1.00 29.83           N
ATOM    312  CA   GLN A  39     -42.189 -14.894  96.940  1.00 29.44           C
ATOM    313  C    GLN A  39     -42.070 -13.629  97.776  1.00 30.99           C
ATOM    314  O    GLN A  39     -40.945 -13.189  98.072  1.00 30.65           O
ATOM    315  CB   GLN A  39     -41.278 -15.997  97.467  1.00 26.80           C
ATOM    316  CG   GLN A  39     -41.737 -16.561  98.799  1.00 29.10           C
ATOM    317  CD   GLN A  39     -41.020 -17.825  99.195  1.00 32.69           C
ATOM    318  OE1  GLN A  39     -39.808 -17.945  99.051  1.00 34.15           O
ATOM    319  NE2  GLN A  39     -41.773 -18.787  99.702  1.00 36.84           N
ATOM    320  N    PRO A  40     -43.199 -13.006  98.142  1.00 32.88           N
ATOM    321  CA   PRO A  40     -43.142 -11.823  99.012  1.00 32.74           C
ATOM    322  C    PRO A  40     -42.699 -12.202 100.414  1.00 37.01           C
ATOM    323  O    PRO A  40     -42.970 -13.320 100.883  1.00 37.15           O
ATOM    324  CB   PRO A  40     -44.590 -11.305  99.005  1.00 28.52           C
ATOM    325  CG   PRO A  40     -45.230 -11.963  97.786  1.00 33.90           C
ATOM    326  CD   PRO A  40     -44.566 -13.290  97.668  1.00 32.61           C
ATOM    327  N    PRO A  41     -42.011 -11.304 101.120  1.00 41.34           N
ATOM    328  CA   PRO A  41     -41.405 -11.689 102.405  1.00 35.97           C
ATOM    329  C    PRO A  41     -42.486 -12.089 103.398  1.00 39.15           C
ATOM    330  O    PRO A  41     -43.499 -11.400 103.564  1.00 39.19           O
ATOM    331  CB   PRO A  41     -40.643 -10.430 102.834  1.00 35.82           C
ATOM    332  CG   PRO A  41     -41.378  -9.304 102.151  1.00 36.54           C
ATOM    333  CD   PRO A  41     -41.862  -9.861 100.839  1.00 32.74           C
ATOM    334  N    GLY A  42     -42.264 -13.220 104.057  1.00 39.49           N
ATOM    335  CA   GLY A  42     -43.319 -13.884 104.786  1.00 43.62           C
ATOM    336  C    GLY A  42     -44.017 -14.964 103.996  1.00 43.06           C
ATOM    337  O    GLY A  42     -45.137 -15.343 104.347  1.00 44.55           O
ATOM    338  N    LYS A  43     -43.392 -15.449 102.924  1.00 49.76           N
ATOM    339  CA   LYS A  43     -43.711 -16.719 102.276  1.00 46.50           C
ATOM    340  C    LYS A  43     -44.975 -16.606 101.432  1.00 41.59           C
ATOM    341  O    LYS A  43     -45.732 -15.629 101.536  1.00 39.86           O
ATOM    342  CB   LYS A  43     -43.844 -17.858 103.320  1.00 55.91           C
ATOM    343  CG   LYS A  43     -42.513 -18.447 103.914  1.00 45.31           C
ATOM    344  CD   LYS A  43     -41.860 -19.485 102.968  1.00 46.63           C
ATOM    345  CE   LYS A  43     -41.212 -20.692 103.684  1.00 40.96           C
ATOM    346  NZ   LYS A  43     -39.846 -20.507 104.327  1.00 38.05           N
ATOM    347  N    GLY A  44     -45.192 -17.625 100.601  1.00 39.58           N
ATOM    348  CA   GLY A  44     -46.241 -17.682  99.609  1.00 36.71           C
ATOM    349  C    GLY A  44     -45.620 -17.601  98.231  1.00 37.64           C
ATOM    350  O    GLY A  44     -44.862 -16.666  97.961  1.00 40.33           O
ATOM    351  N    LEU A  45     -45.870 -18.568  97.359  1.00 32.84           N
ATOM    352  CA   LEU A  45     -45.302 -18.533  96.019  1.00 31.46           C
ATOM    353  C    LEU A  45     -46.409 -18.152  95.047  1.00 32.57           C
```

| ATOM | 354 | O | LEU | A | 45 | -47.476 | -18.773 | 95.054 | 1.00 | 37.03 | O |
| ATOM | 355 | CB | LEU | A | 45 | -44.665 | -19.877 | 95.649 | 1.00 | 29.61 | C |
| ATOM | 356 | CG | LEU | A | 45 | -43.412 | -20.297 | 96.434 | 1.00 | 28.20 | C |
| ATOM | 357 | CD1 | LEU | A | 45 | -43.088 | -21.788 | 96.284 | 1.00 | 22.57 | C |
| ATOM | 358 | CD2 | LEU | A | 45 | -42.235 | -19.474 | 95.976 | 1.00 | 25.94 | C |
| ATOM | 359 | N | GLU | A | 46 | -46.150 | -17.152 | 94.201 | 1.00 | 27.39 | N |
| ATOM | 360 | CA | GLU | A | 46 | -47.092 | -16.747 | 93.168 | 1.00 | 31.51 | C |
| ATOM | 361 | C | GLU | A | 46 | -46.444 | -16.851 | 91.798 | 1.00 | 35.34 | C |
| ATOM | 362 | O | GLU | A | 46 | -45.360 | -16.303 | 91.565 | 1.00 | 32.92 | O |
| ATOM | 363 | CB | GLU | A | 46 | -47.602 | -15.323 | 93.347 | 1.00 | 29.82 | C |
| ATOM | 364 | CG | GLU | A | 46 | -47.130 | -14.572 | 94.552 | 1.00 | 39.23 | C |
| ATOM | 365 | CD | GLU | A | 46 | -47.805 | -13.180 | 94.638 | 1.00 | 55.69 | C |
| ATOM | 366 | OE1 | GLU | A | 46 | -48.162 | -12.613 | 93.554 | 1.00 | 51.35 | O |
| ATOM | 367 | OE2 | GLU | A | 46 | -48.001 | -12.680 | 95.784 | 1.00 | 52.13 | O1- |
| ATOM | 368 | N | TRP | A | 47 | -47.137 | -17.524 | 90.893 | 1.00 | 29.44 | N |
| ATOM | 369 | CA | TRP | A | 47 | -46.647 | -17.778 | 89.551 | 1.00 | 30.35 | C |
| ATOM | 370 | C | TRP | A | 47 | -46.982 | -16.605 | 88.644 | 1.00 | 32.75 | C |
| ATOM | 371 | O | TRP | A | 47 | -48.143 | -16.202 | 88.550 | 1.00 | 36.68 | O |
| ATOM | 372 | CB | TRP | A | 47 | -47.265 | -19.068 | 89.037 | 1.00 | 24.36 | C |
| ATOM | 373 | CG | TRP | A | 47 | -46.961 | -19.432 | 87.637 | 1.00 | 29.61 | C |
| ATOM | 374 | CD1 | TRP | A | 47 | -45.883 | -20.133 | 87.188 | 1.00 | 25.71 | C |
| ATOM | 375 | CD2 | TRP | A | 47 | -47.784 | -19.186 | 86.495 | 1.00 | 28.45 | C |
| ATOM | 376 | NE1 | TRP | A | 47 | -45.979 | -20.319 | 85.840 | 1.00 | 26.95 | N |
| ATOM | 377 | CE2 | TRP | A | 47 | -47.136 | -19.748 | 85.388 | 1.00 | 25.32 | C |
| ATOM | 378 | CE3 | TRP | A | 47 | -49.001 | -18.540 | 86.303 | 1.00 | 25.64 | C |
| ATOM | 379 | CZ2 | TRP | A | 47 | -47.662 | -19.687 | 84.112 | 1.00 | 26.63 | C |
| ATOM | 380 | CZ3 | TRP | A | 47 | -49.517 | -18.479 | 85.032 | 1.00 | 29.15 | C |
| ATOM | 381 | CH2 | TRP | A | 47 | -48.853 | -19.054 | 83.761 | 1.00 | 27.51 | C |
| ATOM | 382 | N | PHE | A | 48 | -45.966 | -16.059 | 87.975 | 1.00 | 33.76 | N |
| ATOM | 383 | CA | PHE | A | 48 | -46.182 | -14.896 | 87.119 | 1.00 | 32.37 | C |
| ATOM | 384 | C | PHE | A | 48 | -46.601 | -15.253 | 85.708 | 1.00 | 32.57 | C |
| ATOM | 385 | O | PHE | A | 48 | -47.502 | -14.615 | 85.160 | 1.00 | 39.41 | O |
| ATOM | 386 | CB | PHE | A | 48 | -44.925 | -14.027 | 87.073 | 1.00 | 31.35 | C |
| ATOM | 387 | CG | PHE | A | 48 | -44.998 | -12.843 | 87.979 | 1.00 | 30.88 | C |
| ATOM | 388 | CD2 | PHE | A | 48 | -44.484 | -11.622 | 87.593 | 1.00 | 32.09 | C |
| ATOM | 389 | CD1 | PHE | A | 48 | -45.606 | -12.952 | 89.221 | 1.00 | 31.12 | C |
| ATOM | 390 | CE2 | PHE | A | 48 | -44.562 | -10.519 | 88.440 | 1.00 | 34.85 | C |
| ATOM | 391 | CE1 | PHE | A | 48 | -45.695 | -11.855 | 90.073 | 1.00 | 34.60 | C |
| ATOM | 392 | CZ | PHE | A | 48 | -45.165 | -10.639 | 89.683 | 1.00 | 36.89 | C |
| ATOM | 393 | N | GLY | A | 49 | -45.982 | -16.255 | 85.111 | 1.00 | 31.20 | N |
| ATOM | 394 | CA | GLY | A | 49 | -46.195 | -16.531 | 83.707 | 1.00 | 30.09 | C |
| ATOM | 395 | C | GLY | A | 49 | -45.089 | -17.418 | 83.178 | 1.00 | 31.55 | C |
| ATOM | 396 | O | GLY | A | 49 | -44.201 | -17.849 | 83.913 | 1.00 | 31.30 | O |
| ATOM | 397 | N | GLU | A | 50 | -45.176 | -17.704 | 81.881 | 1.00 | 28.58 | N |
| ATOM | 398 | CA | GLU | A | 50 | -44.233 | -18.608 | 81.247 | 1.00 | 27.28 | C |
| ATOM | 399 | C | GLU | A | 50 | -44.118 | -18.247 | 79.773 | 1.00 | 31.68 | C |
| ATOM | 400 | O | GLU | A | 50 | -45.015 | -17.630 | 79.199 | 1.00 | 32.98 | O |
| ATOM | 401 | CB | GLU | A | 50 | -44.669 | -20.060 | 81.434 | 1.00 | 26.85 | C |
| ATOM | 402 | CG | GLU | A | 50 | -46.040 | -20.348 | 80.874 | 1.00 | 29.78 | C |
| ATOM | 403 | CD | GLU | A | 50 | -46.438 | -21.812 | 81.020 | 1.00 | 35.37 | C |
| ATOM | 404 | OE1 | GLU | A | 50 | -47.350 | -22.259 | 80.271 | 1.00 | 37.88 | O |
| ATOM | 405 | OE2 | GLU | A | 50 | -45.834 | -22.514 | 81.875 | 1.00 | 28.88 | O |
| ATOM | 406 | N | ILE | A | 51 | -42.989 | -18.610 | 79.165 | 1.00 | 32.55 | N |
| ATOM | 407 | CA | ILE | A | 51 | -42.790 | -18.425 | 77.735 | 1.00 | 28.12 | C |
| ATOM | 408 | C | ILE | A | 51 | -42.187 | -19.702 | 77.167 | 1.00 | 34.07 | C |
| ATOM | 409 | O | ILE | A | 51 | -41.470 | -20.426 | 77.867 | 1.00 | 29.86 | O |
| ATOM | 410 | CB | ILE | A | 51 | -41.917 | -17.191 | 77.464 | 1.00 | 29.20 | C |
| ATOM | 411 | CG1 | ILE | A | 51 | -41.860 | -16.866 | 75.954 | 1.00 | 32.21 | C |
| ATOM | 412 | CG2 | ILE | A | 51 | -40.574 | -17.350 | 78.147 | 1.00 | 26.55 | C |
| ATOM | 413 | CD1 | ILE | A | 51 | -41.585 | -15.399 | 75.639 | 1.00 | 26.20 | C |
| ATOM | 414 | N | SER | A | 52 | -42.453 | -19.969 | 75.876 | 1.00 | 37.40 | N |
| ATOM | 415 | CA | SER | A | 52 | -42.398 | -21.343 | 75.378 | 1.00 | 41.14 | C |
| ATOM | 416 | C | SER | A | 52 | -41.162 | -21.701 | 74.563 | 1.00 | 43.60 | C |
| ATOM | 417 | O | SER | A | 52 | -40.851 | -22.904 | 74.473 | 1.00 | 56.16 | O |
| ATOM | 418 | CB | SER | A | 52 | -43.624 | -21.655 | 74.508 | 1.00 | 44.90 | C |
| ATOM | 419 | OG | SER | A | 52 | -43.499 | -21.091 | 73.211 | 1.00 | 42.47 | O |
| ATOM | 420 | N | HIS | A | 53 | -40.465 | -20.709 | 73.992 | 1.00 | 38.93 | N |
| ATOM | 421 | CA | HIS | A | 53 | -39.361 | -20.843 | 73.025 | 1.00 | 46.69 | C |
| ATOM | 422 | C | HIS | A | 53 | -39.838 | -20.322 | 71.682 | 1.00 | 40.68 | C |
| ATOM | 423 | O | HIS | A | 53 | -39.045 | -19.789 | 70.908 | 1.00 | 48.98 | O |
| ATOM | 424 | CB | HIS | A | 53 | -38.827 | -22.278 | 72.833 | 1.00 | 47.29 | C |

```
ATOM    425  CG   HIS A  53   -37.623 -22.370  71.938  1.00 50.45          C
ATOM    426  ND1  HIS A  53   -36.332 -22.400  72.429  1.00 48.68          N
ATOM    427  CD2  HIS A  53   -37.510 -22.432  70.589  1.00 50.73          C
ATOM    428  CE1  HIS A  53   -35.477 -22.464  71.421  1.00 44.63          C
ATOM    429  NE2  HIS A  53   -36.166 -22.483  70.294  1.00 45.11          N
ATOM    430  N    SER A  54   -41.136 -20.418  71.427  1.00 43.19          N
ATOM    431  CA   SER A  54   -41.723 -19.986  70.163  1.00 37.71          C
ATOM    432  C    SER A  54   -41.978 -18.486  69.934  1.00 41.91          C
ATOM    433  O    SER A  54   -42.167 -18.132  68.783  1.00 58.83          O
ATOM    434  CB   SER A  54   -43.072 -20.685  69.930  1.00 41.65          C
ATOM    435  OG   SER A  54   -43.284 -21.786  70.799  1.00 58.13          O
ATOM    436  N    GLY A  55   -42.077 -17.595  70.928  1.00 41.19          N
ATOM    437  CA   GLY A  55   -42.199 -17.864  72.342  1.00 37.12          C
ATOM    438  C    GLY A  55   -43.565 -17.363  72.782  1.00 38.09          C
ATOM    439  O    GLY A  55   -43.782 -16.170  73.025  1.00 35.63          O
ATOM    440  N    SER A  56   -44.514 -18.290  72.843  1.00 35.38          N
ATOM    441  CA   SER A  56   -45.839 -17.960  73.328  1.00 36.92          C
ATOM    442  C    SER A  56   -45.838 -17.864  74.851  1.00 39.87          C
ATOM    443  O    SER A  56   -45.057 -18.536  75.536  1.00 38.19          O
ATOM    444  CB   SER A  56   -46.855 -19.002  72.851  1.00 43.51          C
ATOM    445  OG   SER A  56   -46.477 -20.320  73.214  1.00 52.12          O
ATOM    446  N    THR A  57   -46.745 -17.035  75.381  1.00 36.78          N
ATOM    447  CA   THR A  57   -46.758 -16.688  76.792  1.00 35.49          C
ATOM    448  C    THR A  57   -48.106 -17.037  77.412  1.00 36.54          C
ATOM    449  O    THR A  57   -49.129 -17.068  76.738  1.00 36.43          O
ATOM    450  CB   THR A  57   -46.464 -15.195  77.048  1.00 34.28          C
ATOM    451  OG1  THR A  57   -47.488 -14.390  76.463  1.00 34.38          O
ATOM    452  CG2  THR A  57   -45.110 -14.791  76.511  1.00 32.77          C
ATOM    453  N    ASN A  58   -48.081 -17.324  78.707  1.00 34.99          N
ATOM    454  CA   ASN A  58   -49.282 -17.540  79.497  1.00 35.60          C
ATOM    455  C    ASN A  58   -49.013 -16.847  80.812  1.00 38.61          C
ATOM    456  O    ASN A  58   -48.117 -17.271  81.548  1.00 38.39          O
ATOM    457  CB   ASN A  58   -49.562 -19.013  79.731  1.00 33.14          C
ATOM    458  CG   ASN A  58   -49.588 -19.785  78.459  1.00 38.06          C
ATOM    459  OD1  ASN A  58   -50.518 -19.669  77.673  1.00 42.44          O
ATOM    460  ND2  ASN A  58   -48.555 -20.583  78.234  1.00 37.72          N
ATOM    461  N    TYR A  59   -49.778 -15.800  81.101  1.00 35.54          N
ATOM    462  CA   TYR A  59   -49.557 -14.988  82.281  1.00 36.21          C
ATOM    463  C    TYR A  59   -50.617 -15.291  83.325  1.00 34.45          C
ATOM    464  O    TYR A  59   -51.674 -15.857  83.041  1.00 36.30          O
ATOM    465  CB   TYR A  59   -49.603 -13.498  81.943  1.00 35.28          C
ATOM    466  CG   TYR A  59   -48.633 -13.043  80.880  1.00 35.74          C
ATOM    467  CD1  TYR A  59   -47.259 -13.085  81.082  1.00 34.19          C
ATOM    468  CD2  TYR A  59   -49.101 -12.544  79.678  1.00 35.17          C
ATOM    469  CE1  TYR A  59   -46.383 -12.644  80.107  1.00 33.92          C
ATOM    470  CE2  TYR A  59   -48.241 -12.098  78.700  1.00 31.35          C
ATOM    471  CZ   TYR A  59   -46.888 -12.143  78.912  1.00 37.17          C
ATOM    472  OH   TYR A  59   -46.055 -11.690  77.915  1.00 33.15          O
ATOM    473  N    ASN A  60   -50.335 -14.882  84.531  1.00 37.70          N
ATOM    474  CA   ASN A  60   -51.356 -14.909  85.549  1.00 38.74          C
ATOM    475  C    ASN A  60   -52.356 -13.791  85.274  1.00 42.68          C
ATOM    476  O    ASN A  60   -51.962 -12.615  85.220  1.00 41.91          O
ATOM    477  CB   ASN A  60   -50.706 -14.771  86.911  1.00 35.36          C
ATOM    478  CG   ASN A  60   -51.663 -15.019  88.031  1.00 37.89          C
ATOM    479  OD1  ASN A  60   -52.874 -14.862  87.873  1.00 38.66          O
ATOM    480  ND2  ASN A  60   -51.142 -15.541  89.137  1.00 36.71          N
ATOM    481  N    PRO A  61   -53.642 -14.102  85.084  1.00 43.65          N
ATOM    482  CA   PRO A  61   -54.609 -13.045  84.741  1.00 37.57          C
ATOM    483  C    PRO A  61   -54.670 -11.924  85.762  1.00 40.30          C
ATOM    484  O    PRO A  61   -54.997 -10.786  85.405  1.00 42.33          O
ATOM    485  CB   PRO A  61   -55.931 -13.820  84.677  1.00 32.38          C
ATOM    486  CG   PRO A  61   -55.502 -15.207  84.249  1.00 39.32          C
ATOM    487  CD   PRO A  61   -54.273 -15.439  85.092  1.00 39.25          C
ATOM    488  N    SER A  62   -54.321 -12.218  87.016  1.00 40.22          N
ATOM    489  CA   SER A  62   -54.367 -11.233  88.092  1.00 39.59          C
ATOM    490  C    SER A  62   -53.386 -10.087  87.868  1.00 45.31          C
ATOM    491  O    SER A  62   -53.617  -8.957  88.319  1.00 43.39          O
ATOM    492  CB   SER A  62   -54.058 -11.920  89.419  1.00 40.43          C
ATOM    493  OG   SER A  62   -53.155 -11.129  90.177  1.00 48.57          O
ATOM    494  N    LEU A  63   -52.237 -10.383  87.272  1.00 45.35          N
ATOM    495  CA   LEU A  63   -51.297  -9.329  86.930  1.00 44.69          C
```

```
ATOM    496  C   LEU A  63   -51.894   -8.416  85.869  1.00 53.96          C
ATOM    497  O   LEU A  63   -51.658   -7.201  85.861  1.00 54.71          O
ATOM    498  CB  LEU A  63   -50.001   -9.971  86.440  1.00 43.57          C
ATOM    499  CG  LEU A  63   -49.158  -10.633  87.535  1.00 43.51          C
ATOM    500  CD1 LEU A  63   -47.844  -11.134  86.978  1.00 40.31          C
ATOM    501  CD2 LEU A  63   -48.888   -9.663  88.691  1.00 43.97          C
ATOM    502  N   LYS A  64   -52.663   -9.003  84.955  1.00 58.46          N
ATOM    503  CA  LYS A  64   -53.324   -8.320  83.854  1.00 58.94          C
ATOM    504  C   LYS A  64   -52.277   -7.745  82.912  1.00 51.96          C
ATOM    505  O   LYS A  64   -51.377   -8.460  82.452  1.00 48.34          O
ATOM    506  CB  LYS A  64   -54.274   -7.204  84.304  1.00 52.16          C
ATOM    507  CG  LYS A  64   -55.571   -7.258  83.486  1.00 62.77          C
ATOM    508  CD  LYS A  64   -56.400   -5.965  83.488  1.00 81.85          C
ATOM    509  CE  LYS A  64   -57.048   -5.593  84.810  1.00 81.71          C
ATOM    510  NZ  LYS A  64   -57.659   -4.226  84.694  1.00 73.63          N
ATOM    511  N   ALA A  65   -52.386   -6.444  82.659  1.00 41.71          N
ATOM    512  CA  ALA A  65   -51.539   -5.768  81.693  1.00 43.20          C
ATOM    513  C   ALA A  65   -50.168   -5.367  82.231  1.00 38.62          C
ATOM    514  O   ALA A  65   -49.389   -4.808  81.463  1.00 42.39          O
ATOM    515  CB  ALA A  65   -52.249   -4.540  81.128  1.00 46.41          C
ATOM    516  N   ARG A  66   -49.867   -5.542  83.523  1.00 38.64          N
ATOM    517  CA  ARG A  66   -48.595   -5.016  84.025  1.00 34.40          C
ATOM    518  C   ARG A  66   -47.377   -5.847  83.627  1.00 36.18          C
ATOM    519  O   ARG A  66   -46.249   -5.349  83.752  1.00 31.12          O
ATOM    520  CB  ARG A  66   -48.586   -4.863  85.546  1.00 33.68          C
ATOM    521  CG  ARG A  66   -49.764   -4.132  86.091  1.00 40.09          C
ATOM    522  CD  ARG A  66   -49.537   -3.649  87.519  1.00 40.22          C
ATOM    523  NE  ARG A  66   -49.265   -4.663  88.539  1.00 35.47          N
ATOM    524  CZ  ARG A  66   -48.148   -4.713  89.268  1.00 34.74          C
ATOM    525  NH1 ARG A  66   -47.168   -3.842  89.065  1.00 33.41          N1+
ATOM    526  NH2 ARG A  66   -48.004   -5.640  90.205  1.00 40.56          N
ATOM    527  N   VAL A  67   -47.559   -7.095  83.188  1.00 34.62          N
ATOM    528  CA  VAL A  67   -46.463   -8.049  83.084  1.00 36.24          C
ATOM    529  C   VAL A  67   -46.186   -8.411  81.626  1.00 36.09          C
ATOM    530  O   VAL A  67   -47.113   -8.575  80.828  1.00 36.24          O
ATOM    531  CB  VAL A  67   -46.782   -9.292  83.935  1.00 34.93          C
ATOM    532  CG1 VAL A  67   -47.992   -9.994  83.388  1.00 41.56          C
ATOM    533  CG2 VAL A  67   -45.596  -10.222  83.981  1.00 36.82          C
ATOM    534  N   THR A  68   -44.894   -8.531  81.289  1.00 35.40          N
ATOM    535  CA  THR A  68   -44.405   -8.998  79.998  1.00 33.94          C
ATOM    536  C   THR A  68   -43.267   -9.973  80.231  1.00 33.46          C
ATOM    537  O   THR A  68   -42.412   -9.739  81.087  1.00 34.05          O
ATOM    538  CB  THR A  68   -43.854   -7.882  79.090  1.00 37.93          C
ATOM    539  OG1 THR A  68   -44.745   -6.763  79.052  1.00 38.69          O
ATOM    540  CG2 THR A  68   -43.621   -8.423  77.672  1.00 27.13          C
ATOM    541  N   ILE A  69   -43.262  -11.064  79.471  1.00 35.17          N
ATOM    542  CA  ILE A  69   -42.163  -12.021  79.455  1.00 31.25          C
ATOM    543  C   ILE A  69   -41.710  -12.169  78.008  1.00 37.24          C
ATOM    544  O   ILE A  69   -42.541  -12.313  77.103  1.00 33.89          O
ATOM    545  CB  ILE A  69   -42.574  -13.382  80.051  1.00 27.84          C
ATOM    546  CG1 ILE A  69   -42.912  -13.212  81.523  1.00 31.71          C
ATOM    547  CG2 ILE A  69   -41.468  -14.407  79.896  1.00 25.74          C
ATOM    548  CD1 ILE A  69   -43.605  -14.385  82.110  1.00 30.41          C
ATOM    549  N   SER A  70   -40.401  -12.101  77.789  1.00 32.26          N
ATOM    550  CA  SER A  70   -39.826  -12.197  76.460  1.00 29.37          C
ATOM    551  C   SER A  70   -38.678  -13.195  76.500  1.00 33.52          C
ATOM    552  O   SER A  70   -38.103  -13.470  77.557  1.00 32.58          O
ATOM    553  CB  SER A  70   -39.322  -10.841  75.963  1.00 30.41          C
ATOM    554  OG  SER A  70   -38.395  -10.282  76.888  1.00 41.93          O
ATOM    555  N   ILE A  71   -38.333  -13.724  75.331  1.00 30.14          N
ATOM    556  CA  ILE A  71   -37.327  -14.761  75.222  1.00 29.53          C
ATOM    557  C   ILE A  71   -36.358  -14.414  74.097  1.00 34.98          C
ATOM    558  O   ILE A  71   -36.762  -13.926  73.035  1.00 33.60          O
ATOM    559  CB  ILE A  71   -37.992  -16.132  75.000  1.00 29.32          C
ATOM    560  CG1 ILE A  71   -37.001  -17.267  75.279  1.00 29.60          C
ATOM    561  CG2 ILE A  71   -38.575  -16.224  73.606  1.00 30.62          C
ATOM    562  CD1 ILE A  71   -37.644  -18.637  75.331  1.00 29.78          C
ATOM    563  N   ASP A  72   -35.076  -14.658  74.339  1.00 33.23          N
ATOM    564  CA  ASP A  72   -34.016  -14.453  73.355  1.00 32.69          C
ATOM    565  C   ASP A  72   -33.394  -15.828  73.138  1.00 36.12          C
ATOM    566  O   ASP A  72   -32.525  -16.242  73.910  1.00 37.95          O
```

```
ATOM    567  CB  ASP A  72     -33.008 -13.415  73.862  1.00 31.36           C
ATOM    568  CG  ASP A  72     -31.876 -13.113  72.861  1.00 47.78           C
ATOM    569  OD2 ASP A  72     -31.214 -12.051  73.040  1.00 43.89           O
ATOM    570  OD1 ASP A  72     -31.629 -13.924  71.921  1.00 48.56           O
ATOM    571  N   THR A  73     -33.847 -16.547  72.100  1.00 39.39           N
ATOM    572  CA  THR A  73     -33.375 -17.916  71.888  1.00 36.77           C
ATOM    573  C   THR A  73     -31.919 -17.989  71.429  1.00 43.07           C
ATOM    574  O   THR A  73     -31.301 -19.055  71.535  1.00 46.66           O
ATOM    575  CB  THR A  73     -34.251 -18.621  70.868  1.00 36.60           C
ATOM    576  OG1 THR A  73     -34.346 -17.803  69.699  1.00 47.78           O
ATOM    577  CG2 THR A  73     -35.625 -18.860  71.428  1.00 33.19           C
ATOM    578  N   SER A  74     -31.349 -16.886  70.949  1.00 40.76           N
ATOM    579  CA  SER A  74     -29.949 -16.891  70.549  1.00 43.57           C
ATOM    580  C   SER A  74     -29.032 -16.974  71.758  1.00 50.46           C
ATOM    581  O   SER A  74     -28.083 -17.779  71.784  1.00 52.51           O
ATOM    582  CB  SER A  74     -29.654 -15.639  69.744  1.00 48.36           C
ATOM    583  OG  SER A  74     -30.524 -15.613  68.635  1.00 60.90           O
ATOM    584  O   LYS A  75     -28.334 -16.929  76.160  1.00 39.85           O
ATOM    585  N   LYS A  75     -29.291 -16.123  72.754  1.00 40.62           N
ATOM    586  CA  LYS A  75     -28.494 -15.995  73.967  1.00 44.56           C
ATOM    587  C   LYS A  75     -28.929 -16.951  75.076  1.00 39.19           C
ATOM    588  CB  LYS A  75     -28.574 -14.552  74.490  1.00 42.65           C
ATOM    589  CG  LYS A  75     -28.087 -13.508  73.502  1.00 48.05           C
ATOM    590  CD  LYS A  75     -28.250 -12.099  74.045  1.00 49.13           C
ATOM    591  CE  LYS A  75     -28.043 -11.079  72.924  1.00 55.25           C
ATOM    592  NZ  LYS A  75     -27.500  -9.765  73.364  1.00 55.09           N
ATOM    593  N   ASN A  76     -29.936 -17.793  74.823  1.00 43.17           N
ATOM    594  CA  ASN A  76     -30.578 -18.625  75.845  1.00 36.87           C
ATOM    595  C   ASN A  76     -30.949 -17.780  77.073  1.00 35.94           C
ATOM    596  O   ASN A  76     -30.668 -18.130  78.221  1.00 33.33           O
ATOM    597  CB  ASN A  76     -29.696 -19.823  76.213  1.00 32.65           C
ATOM    598  CG  ASN A  76     -29.729 -20.940  75.144  1.00 41.58           C
ATOM    599  OD1 ASN A  76     -30.207 -20.738  74.016  1.00 42.66           O
ATOM    600  ND2 ASN A  76     -29.215 -22.117  75.500  1.00 36.55           N
ATOM    601  N   GLN A  77     -31.635 -16.668  76.813  1.00 29.24           N
ATOM    602  CA  GLN A  77     -32.064 -15.729  77.828  1.00 28.20           C
ATOM    603  C   GLN A  77     -33.578 -15.515  77.767  1.00 32.38           C
ATOM    604  O   GLN A  77     -34.233 -15.760  76.747  1.00 32.97           O
ATOM    605  CB  GLN A  77     -31.365 -14.391  77.668  1.00 31.15           C
ATOM    606  CG  GLN A  77     -29.894 -14.376  78.032  1.00 35.97           C
ATOM    607  CD  GLN A  77     -29.313 -12.956  77.951  1.00 39.65           C
ATOM    608  OE1 GLN A  77     -29.979 -12.017  77.502  1.00 40.22           O
ATOM    609  NE2 GLN A  77     -28.096 -12.792  78.433  1.00 41.23           N
ATOM    610  N   PHE A  78     -34.148 -15.096  78.891  1.00 24.83           N
ATOM    611  CA  PHE A  78     -35.544 -14.682  78.904  1.00 30.44           C
ATOM    612  C   PHE A  78     -35.711 -13.657  80.014  1.00 28.14           C
ATOM    613  O   PHE A  78     -34.863 -13.544  80.899  1.00 29.63           O
ATOM    614  CB  PHE A  78     -36.519 -15.877  79.038  1.00 27.85           C
ATOM    615  CG  PHE A  78     -36.399 -16.648  80.334  1.00 29.82           C
ATOM    616  CD1 PHE A  78     -35.434 -17.641  80.482  1.00 28.03           C
ATOM    617  CD2 PHE A  78     -37.290 -16.414  81.385  1.00 27.75           C
ATOM    618  CE1 PHE A  78     -35.334 -18.366  81.658  1.00 28.84           C
ATOM    619  CE2 PHE A  78     -37.201 -17.127  82.571  1.00 29.25           C
ATOM    620  CZ  PHE A  78     -36.224 -18.108  82.713  1.00 31.58           C
ATOM    621  N   SER A  79     -36.779 -12.866  79.933  1.00 27.98           N
ATOM    622  CA  SER A  79     -36.845 -11.640  80.717  1.00 29.89           C
ATOM    623  C   SER A  79     -38.230 -11.432  81.299  1.00 27.88           C
ATOM    624  O   SER A  79     -39.225 -11.992  80.827  1.00 27.43           O
ATOM    625  CB  SER A  79     -36.437 -10.410  79.881  1.00 27.31           C
ATOM    626  OG  SER A  79     -35.076 -10.523  79.495  1.00 33.81           O
ATOM    627  N   LEU A  80     -38.270 -10.585  82.327  1.00 24.79           N
ATOM    628  CA  LEU A  80     -39.503 -10.224  82.998  1.00 25.66           C
ATOM    629  C   LEU A  80     -39.605  -8.711  83.069  1.00 30.62           C
ATOM    630  O   LEU A  80     -38.669  -8.045  83.525  1.00 32.93           O
ATOM    631  CB  LEU A  80     -39.563 -10.806  84.409  1.00 30.64           C
ATOM    632  CG  LEU A  80     -40.803 -10.348  85.179  1.00 29.58           C
ATOM    633  CD2 LEU A  80     -40.702 -10.668  86.648  1.00 29.00           C
ATOM    634  CD1 LEU A  80     -42.002 -11.007  84.558  1.00 27.41           C
ATOM    635  N   LYS A  81     -40.750  -8.174  82.655  1.00 30.10           N
ATOM    636  CA  LYS A  81     -41.086  -6.769  82.845  1.00 30.96           C
ATOM    637  C   LYS A  81     -42.340  -6.677  83.705  1.00 30.22           C
```

```
ATOM    638  O    LYS A  81    -43.320   -7.382   83.453  1.00 32.80        O
ATOM    639  CB   LYS A  81    -41.299   -6.065   81.499  1.00 30.39        C
ATOM    640  CG   LYS A  81    -40.033   -5.832   80.708  1.00 30.35        C
ATOM    641  CD   LYS A  81    -39.381   -4.518   81.077  1.00 41.11        C
ATOM    642  CE   LYS A  81    -38.053   -4.352   80.368  1.00 43.35        C
ATOM    643  NZ   LYS A  81    -38.187   -4.617   78.920  1.00 51.59        N
ATOM    644  N    LEU A  82    -42.298   -5.837   84.739  1.00 30.39        N
ATOM    645  CA   LEU A  82    -43.450   -5.573   85.601  1.00 29.80        C
ATOM    646  C    LEU A  82    -43.573   -4.071   85.755  1.00 32.85        C
ATOM    647  O    LEU A  82    -42.697   -3.440   86.352  1.00 31.95        O
ATOM    648  CB   LEU A  82    -43.304   -6.239   86.968  1.00 32.00        C
ATOM    649  CG   LEU A  82    -44.442   -6.002   87.959  1.00 32.12        C
ATOM    650  CD1  LEU A  82    -45.711   -6.714   87.526  1.00 26.83        C
ATOM    651  CD2  LEU A  82    -44.016   -6.435   89.349  1.00 28.77        C
ATOM    652  N    ARG A  83    -44.645   -3.505   85.209  1.00 35.72        N
ATOM    653  CA   ARG A  83    -44.790   -2.062   85.113  1.00 35.41        C
ATOM    654  C    ARG A  83    -45.506   -1.514   86.344  1.00 34.77        C
ATOM    655  O    ARG A  83    -46.221   -2.234   87.051  1.00 37.08        O
ATOM    656  CB   ARG A  83    -45.556   -1.686   83.838  1.00 30.94        C
ATOM    657  CG   ARG A  83    -44.709   -1.827   82.572  1.00 31.53        C
ATOM    658  CD   ARG A  83    -45.526   -1.799   81.301  1.00 34.78        C
ATOM    659  NE   ARG A  83    -46.273   -3.036   81.049  1.00 36.35        N
ATOM    660  CZ   ARG A  83    -45.806   -4.149   80.484  1.00 32.43        C
ATOM    661  NH1  ARG A  83    -46.625   -5.178   80.327  1.00 35.09        N
ATOM    662  NH2  ARG A  83    -44.550   -4.257   80.084  1.00 29.31        N
ATOM    663  N    SER A  84    -45.335   -0.214   86.571  1.00 37.19        N
ATOM    664  CA   SER A  84    -46.105    0.534   87.573  1.00 35.36        C
ATOM    665  C    SER A  84    -46.068   -0.152   88.936  1.00 39.37        C
ATOM    666  O    SER A  84    -47.092   -0.470   89.546  1.00 39.10        O
ATOM    667  CB   SER A  84    -47.548    0.713   87.111  1.00 33.54        C
ATOM    668  OG   SER A  84    -47.598    1.374   85.864  1.00 48.40        O
ATOM    669  N    VAL A  85    -44.868   -0.296   89.432  1.00 35.87        N
ATOM    670  CA   VAL A  85    -44.588   -1.159   90.568  1.00 36.45        C
ATOM    671  C    VAL A  85    -44.889   -0.417   91.872  1.00 34.18        C
ATOM    672  O    VAL A  85    -44.767    0.807   91.943  1.00 36.97        O
ATOM    673  CB   VAL A  85    -43.117   -1.606   90.424  1.00 35.38        C
ATOM    674  CG1  VAL A  85    -42.214   -0.855   91.349  1.00 36.25        C
ATOM    675  CG2  VAL A  85    -42.981   -3.082   90.543  1.00 34.41        C
ATOM    676  N    THR A  86    -45.346   -1.130   92.907  1.00 36.49        N
ATOM    677  CA   THR A  86    -45.612   -0.502   94.212  1.00 32.44        C
ATOM    678  C    THR A  86    -44.908   -1.279   95.326  1.00 33.48        C
ATOM    679  O    THR A  86    -44.209   -2.271   95.087  1.00 34.30        O
ATOM    680  CB   THR A  86    -47.107   -0.413   94.556  1.00 32.14        C
ATOM    681  OG1  THR A  86    -47.526   -1.616   95.204  1.00 36.59        O
ATOM    682  CG2  THR A  86    -47.956   -0.200   93.321  1.00 27.41        C
ATOM    683  N    ALA A  87    -45.082   -0.810   96.565  1.00 35.31        N
ATOM    684  CA   ALA A  87    -44.442   -1.484   97.698  1.00 33.77        C
ATOM    685  C    ALA A  87    -44.927   -2.921   97.822  1.00 31.88        C
ATOM    686  O    ALA A  87    -44.184   -3.798   98.273  1.00 31.04        O
ATOM    687  CB   ALA A  87    -44.701   -0.719   98.996  1.00 25.59        C
ATOM    688  N    ALA A  88    -46.161   -3.186   97.408  1.00 30.13        N
ATOM    689  CA   ALA A  88    -46.689   -4.537   97.438  1.00 27.33        C
ATOM    690  C    ALA A  88    -45.962   -5.482   96.487  1.00 29.67        C
ATOM    691  O    ALA A  88    -46.226   -6.681   96.532  1.00 32.42        O
ATOM    692  CB   ALA A  88    -48.185   -4.510   97.113  1.00 20.61        C
ATOM    693  N    ASP A  89    -45.073   -4.987   95.628  1.00 29.86        N
ATOM    694  CA   ASP A  89    -44.275   -5.840   94.756  1.00 27.31        C
ATOM    695  C    ASP A  89    -42.892   -6.166   95.332  1.00 29.16        C
ATOM    696  O    ASP A  89    -42.057   -6.737   94.618  1.00 28.53        O
ATOM    697  CB   ASP A  89    -44.141   -5.200   93.359  1.00 31.57        C
ATOM    698  CG   ASP A  89    -45.504   -4.988   92.660  1.00 35.37        C
ATOM    699  OD1  ASP A  89    -46.204   -5.990   92.421  1.00 36.17        O
ATOM    700  OD2  ASP A  89    -45.880   -3.830   92.332  1.00 34.69        O1-
ATOM    701  N    THR A  90    -42.616   -5.798   96.590  1.00 31.96        N
ATOM    702  CA   THR A  90    -41.400   -6.246   97.270  1.00 27.24        C
ATOM    703  C    THR A  90    -41.440   -7.754   97.476  1.00 25.30        C
ATOM    704  O    THR A  90    -42.379   -8.277   98.074  1.00 30.54        O
ATOM    705  CB   THR A  90    -41.237   -5.541   98.604  1.00 25.80        C
ATOM    706  OG1  THR A  90    -40.966   -4.159   98.369  1.00 34.42        O
ATOM    707  CG2  THR A  90    -40.111   -6.162   99.391  1.00 23.83        C
ATOM    708  N    ALA A  91    -40.460   -8.455   96.928  1.00 26.62        N
```

```
ATOM    709  CA  ALA A  91     -40.455  -9.906  96.947  1.00 26.31           C
ATOM    710  C   ALA A  91     -39.136 -10.402  96.385  1.00 31.03           C
ATOM    711  O   ALA A  91     -38.386  -9.658  95.738  1.00 27.14           O
ATOM    712  CB  ALA A  91     -41.605 -10.497  96.134  1.00 25.43           C
ATOM    713  N   VAL A  92     -38.876 -11.680  96.632  1.00 31.34           N
ATOM    714  CA  VAL A  92     -37.860 -12.385  95.875  1.00 31.22           C
ATOM    715  C   VAL A  92     -38.510 -12.880  94.591  1.00 32.03           C
ATOM    716  O   VAL A  92     -39.615 -13.429  94.616  1.00 32.99           O
ATOM    717  CB  VAL A  92     -37.243 -13.525  96.695  1.00 25.83           C
ATOM    718  CG1 VAL A  92     -36.306 -14.333  95.817  1.00 27.86           C
ATOM    719  CG2 VAL A  92     -36.479 -12.962  97.892  1.00 20.30           C
ATOM    720  N   TYR A  93     -37.871 -12.596  93.460  1.00 31.17           N
ATOM    721  CA  TYR A  93     -38.321 -13.013  92.141  1.00 25.61           C
ATOM    722  C   TYR A  93     -37.385 -14.112  91.680  1.00 27.44           C
ATOM    723  O   TYR A  93     -36.167 -13.932  91.702  1.00 29.84           O
ATOM    724  CB  TYR A  93     -38.327 -11.832  91.166  1.00 24.64           C
ATOM    725  CG  TYR A  93     -39.424 -10.852  91.484  1.00 26.39           C
ATOM    726  CD1 TYR A  93     -39.346 -10.047  92.606  1.00 24.17           C
ATOM    727  CD2 TYR A  93     -40.556 -10.756  90.675  1.00 29.39           C
ATOM    728  CE1 TYR A  93     -40.360  -9.180  92.927  1.00 30.45           C
ATOM    729  CE2 TYR A  93     -41.573  -9.886  90.974  1.00 28.95           C
ATOM    730  CZ  TYR A  93     -41.470  -9.096  92.108  1.00 30.87           C
ATOM    731  OH  TYR A  93     -42.478  -8.228  92.442  1.00 29.44           O
ATOM    732  N   TYR A  94     -37.946 -15.271  91.356  1.00 27.38           N
ATOM    733  CA  TYR A  94     -37.195 -16.387  90.803  1.00 27.27           C
ATOM    734  C   TYR A  94     -37.582 -16.602  89.347  1.00 27.79           C
ATOM    735  O   TYR A  94     -38.728 -16.360  88.943  1.00 25.82           O
ATOM    736  CB  TYR A  94     -37.453 -17.695  91.544  1.00 24.75           C
ATOM    737  CG  TYR A  94     -37.170 -17.737  92.995  1.00 23.43           C
ATOM    738  CD1 TYR A  94     -35.885 -17.935  93.462  1.00 28.16           C
ATOM    739  CD2 TYR A  94     -38.199 -17.643  93.910  1.00 25.00           C
ATOM    740  CE1 TYR A  94     -35.621 -18.000  94.820  1.00 32.06           C
ATOM    741  CE2 TYR A  94     -37.955 -17.702  95.271  1.00 29.99           C
ATOM    742  CZ  TYR A  94     -36.666 -17.881  95.720  1.00 33.84           C
ATOM    743  OH  TYR A  94     -36.428 -17.954  97.069  1.00 39.80           O
ATOM    744  N   CYS A  95     -36.618 -17.046  88.560  1.00 23.05           N
ATOM    745  CA  CYS A  95     -36.940 -17.717  87.319  1.00 26.62           C
ATOM    746  C   CYS A  95     -36.809 -19.212  87.547  1.00 27.72           C
ATOM    747  O   CYS A  95     -36.050 -19.661  88.416  1.00 27.65           O
ATOM    748  CB  CYS A  95     -36.029 -17.289  86.177  1.00 33.36           C
ATOM    749  SG  CYS A  95     -34.315 -17.693  86.436  1.00 42.59           S
ATOM    750  N   ALA A  96     -37.549 -19.988  86.766  1.00 25.63           N
ATOM    751  CA  ALA A  96     -37.488 -21.432  86.941  1.00 24.92           C
ATOM    752  C   ALA A  96     -37.693 -22.118  85.603  1.00 23.67           C
ATOM    753  O   ALA A  96     -38.234 -21.545  84.650  1.00 22.72           O
ATOM    754  CB  ALA A  96     -38.514 -21.933  87.971  1.00 20.77           C
ATOM    755  N   ARG A  97     -37.232 -23.359  85.549  1.00 29.65           N
ATOM    756  CA  ARG A  97     -37.442 -24.222  84.397  1.00 27.16           C
ATOM    757  C   ARG A  97     -38.707 -25.049  84.601  1.00 25.26           C
ATOM    758  O   ARG A  97     -38.796 -25.837  85.543  1.00 26.21           O
ATOM    759  CB  ARG A  97     -36.241 -25.141  84.199  1.00 28.02           C
ATOM    760  CG  ARG A  97     -36.333 -25.943  82.927  1.00 30.37           C
ATOM    761  CD  ARG A  97     -35.057 -26.647  82.678  1.00 30.79           C
ATOM    762  NE  ARG A  97     -35.199 -28.067  82.916  1.00 37.06           N
ATOM    763  CZ  ARG A  97     -34.181 -28.869  83.182  1.00 41.04           C
ATOM    764  NH1 ARG A  97     -32.962 -28.358  83.257  1.00 44.36           N
ATOM    765  NH2 ARG A  97     -34.378 -30.172  83.387  1.00 43.81           N
ATOM    766  N   ASP A  98     -39.663 -24.894  83.701  1.00 28.03           N
ATOM    767  CA  ASP A  98     -40.928 -25.608  83.765  1.00 28.42           C
ATOM    768  C   ASP A  98     -40.781 -26.830  82.871  1.00 29.02           C
ATOM    769  O   ASP A  98     -40.568 -26.690  81.661  1.00 30.24           O
ATOM    770  CB  ASP A  98     -42.074 -24.693  83.315  1.00 28.02           C
ATOM    771  CG  ASP A  98     -43.460 -25.253  83.615  1.00 32.08           C
ATOM    772  OD2 ASP A  98     -44.210 -24.615  84.387  1.00 34.95           O
ATOM    773  OD1 ASP A  98     -43.837 -26.292  83.050  1.00 33.28           O
ATOM    774  N   TYR A  99     -40.940 -28.023  83.458  1.00 30.78           N
ATOM    775  CA  TYR A  99     -40.738 -29.329  82.815  1.00 30.04           C
ATOM    776  C   TYR A  99     -41.625 -30.324  83.561  1.00 33.93           C
ATOM    777  O   TYR A  99     -41.159 -31.204  84.293  1.00 38.31           O
ATOM    778  CB  TYR A  99     -39.272 -29.767  82.827  1.00 29.21           C
ATOM    779  CG  TYR A  99     -38.929 -30.840  81.794  1.00 34.85           C
```

```
ATOM    780  CD1 TYR A  99    -39.322 -30.698  80.458  1.00 35.04       C
ATOM    781  CD2 TYR A  99    -38.133 -31.940  82.127  1.00 32.30       C
ATOM    782  CE1 TYR A  99    -38.982 -31.651  79.496  1.00 36.24       C
ATOM    783  CE2 TYR A  99    -37.783 -32.896  81.172  1.00 28.54       C
ATOM    784  CZ  TYR A  99    -38.209 -32.749  79.865  1.00 35.98       C
ATOM    785  OH  TYR A  99    -37.874 -33.692  78.917  1.00 34.12       O
ATOM    786  N   GLY A 100    -42.933 -30.172  83.358  1.00 28.51       N
ATOM    787  CA  GLY A 100    -43.910 -30.735  84.262  1.00 24.10       C
ATOM    788  C   GLY A 100    -44.030 -29.821  85.465  1.00 27.81       C
ATOM    789  O   GLY A 100    -44.871 -28.922  85.491  1.00 27.49       O
ATOM    790  N   ALA A 101    -43.179 -30.038  86.462  1.00 27.49       N
ATOM    791  CA  ALA A 101    -43.007 -29.155  87.602  1.00 27.47       C
ATOM    792  C   ALA A 101    -41.783 -28.256  87.421  1.00 30.09       C
ATOM    793  O   ALA A 101    -41.094 -28.287  86.395  1.00 27.64       O
ATOM    794  CB  ALA A 101    -42.895 -29.967  88.884  1.00 25.99       C
ATOM    795  N   PHE A 102    -41.526 -27.427  88.434  1.00 23.58       N
ATOM    796  CA  PHE A 102    -40.378 -26.521  88.429  1.00 29.82       C
ATOM    797  C   PHE A 102    -39.181 -27.242  89.042  1.00 28.21       C
ATOM    798  O   PHE A 102    -39.007 -27.255  90.268  1.00 26.65       O
ATOM    799  CB  PHE A 102    -40.704 -25.241  89.185  1.00 24.30       C
ATOM    800  CG  PHE A 102    -41.831 -24.469  88.585  1.00 29.71       C
ATOM    801  CD1 PHE A 102    -41.705 -23.921  87.306  1.00 27.01       C
ATOM    802  CD2 PHE A 102    -43.012 -24.265  89.297  1.00 28.29       C
ATOM    803  CE1 PHE A 102    -42.719 -23.201  86.752  1.00 23.76       C
ATOM    804  CE2 PHE A 102    -44.047 -23.538  88.738  1.00 27.29       C
ATOM    805  CZ  PHE A 102    -43.897 -23.010  87.461  1.00 27.69       C
ATOM    806  N   ASP A 103    -38.333 -27.818  88.179  1.00 24.44       N
ATOM    807  CA  ASP A 103    -37.235 -28.648  88.657  1.00 28.27       C
ATOM    808  C   ASP A 103    -35.943 -27.887  88.873  1.00 27.03       C
ATOM    809  O   ASP A 103    -35.131 -28.306  89.693  1.00 37.49       O
ATOM    810  CB  ASP A 103    -36.989 -29.838  87.721  1.00 31.73       C
ATOM    811  CG  ASP A 103    -36.673 -29.436  86.302  1.00 39.51       C
ATOM    812  OD1 ASP A 103    -36.346 -28.251  86.038  1.00 36.08       O
ATOM    813  OD2 ASP A 103    -36.735 -30.340  85.438  1.00 44.53       O1-
ATOM    814  N   ILE A 104    -35.734 -26.768  88.205  1.00 32.07       N
ATOM    815  CA  ILE A 104    -34.562 -25.951  88.461  1.00 32.48       C
ATOM    816  C   ILE A 104    -35.014 -24.530  88.674  1.00 30.55       C
ATOM    817  O   ILE A 104    -35.832 -24.011  87.910  1.00 32.04       O
ATOM    818  CB  ILE A 104    -33.537 -25.999  87.318  1.00 33.95       C
ATOM    819  CG1 ILE A 104    -33.052 -27.421  87.124  1.00 31.05       C
ATOM    820  CG2 ILE A 104    -32.357 -25.103  87.642  1.00 29.90       C
ATOM    821  CD1 ILE A 104    -32.035 -27.491  86.097  1.00 37.82       C
ATOM    822  N   TRP A 105    -34.437 -23.891  89.678  1.00 30.90       N
ATOM    823  CA  TRP A 105    -34.761 -22.534  90.062  1.00 29.84       C
ATOM    824  C   TRP A 105    -33.493 -21.703  90.002  1.00 35.42       C
ATOM    825  O   TRP A 105    -32.384 -22.226  90.145  1.00 32.10       O
ATOM    826  CB  TRP A 105    -35.340 -22.469  91.479  1.00 30.72       C
ATOM    827  CG  TRP A 105    -36.660 -23.174  91.650  1.00 28.36       C
ATOM    828  CD1 TRP A 105    -36.900 -24.508  91.535  1.00 26.86       C
ATOM    829  CD2 TRP A 105    -37.906 -22.570  92.029  1.00 27.83       C
ATOM    830  NE1 TRP A 105    -38.221 -24.775  91.787  1.00 26.76       N
ATOM    831  CE2 TRP A 105    -38.862 -23.601  92.093  1.00 27.46       C
ATOM    832  CE3 TRP A 105    -38.306 -21.246  92.305  1.00 24.64       C
ATOM    833  CZ2 TRP A 105    -40.203 -23.359  92.425  1.00 26.14       C
ATOM    834  CZ3 TRP A 105    -39.632 -21.002  92.628  1.00 26.09       C
ATOM    835  CH2 TRP A 105    -40.569 -22.052  92.682  1.00 27.22       C
ATOM    836  N   GLY A 106    -33.675 -20.409  89.739  1.00 36.95       N
ATOM    837  CA  GLY A 106    -32.621 -19.444  89.932  1.00 30.80       C
ATOM    838  C   GLY A 106    -32.355 -19.129  91.400  1.00 35.16       C
ATOM    839  O   GLY A 106    -33.108 -19.455  92.312  1.00 36.82       O
ATOM    840  N   GLN A 107    -31.218 -18.464  91.603  1.00 47.39       N
ATOM    841  CA  GLN A 107    -30.810 -17.934  92.899  1.00 36.74       C
ATOM    842  C   GLN A 107    -31.901 -17.089  93.533  1.00 39.96       C
ATOM    843  O   GLN A 107    -32.042 -17.063  94.766  1.00 42.16       O
ATOM    844  CB  GLN A 107    -29.557 -17.093  92.676  1.00 42.92       C
ATOM    845  CG  GLN A 107    -29.167 -17.061  91.154  1.00 49.03       C
ATOM    846  CD  GLN A 107    -28.508 -15.762  90.696  1.00 54.29       C
ATOM    847  OE1 GLN A 107    -28.954 -14.661  91.047  1.00 58.14       O
ATOM    848  NE2 GLN A 107    -27.442 -15.886  89.898  1.00 50.79       N
ATOM    849  N   GLY A 108    -32.681 -16.410  92.707  1.00 31.86       N
ATOM    850  CA  GLY A 108    -33.598 -15.378  93.128  1.00 27.03       C
```

```
ATOM    851  C   GLY A 108     -32.949 -14.010  93.074  1.00 30.24           C
ATOM    852  O   GLY A 108     -31.729 -13.864  93.142  1.00 37.43           O
ATOM    853  N   THR A 109     -33.797 -12.994  92.950  1.00 25.96           N
ATOM    854  CA  THR A 109     -33.387 -11.598  92.963  1.00 27.32           C
ATOM    855  C   THR A 109     -34.249 -10.910  94.000  1.00 31.41           C
ATOM    856  O   THR A 109     -35.475 -10.935  93.890  1.00 32.07           O
ATOM    857  CB  THR A 109     -33.582 -10.910  91.604  1.00 34.49           C
ATOM    858  OG1 THR A 109     -32.815 -11.575  90.587  1.00 40.25           O
ATOM    859  CG2 THR A 109     -33.151  -9.449  91.696  1.00 26.29           C
ATOM    860  N   MET A 110     -33.618 -10.317  95.010  1.00 34.98           N
ATOM    861  CA  MET A 110     -34.341  -9.610  96.058  1.00 32.57           C
ATOM    862  C   MET A 110     -34.744  -8.234  95.546  1.00 36.42           C
ATOM    863  O   MET A 110     -33.881  -7.407  95.236  1.00 38.21           O
ATOM    864  CB  MET A 110     -33.471  -9.495  97.301  1.00 32.17           C
ATOM    865  CG  MET A 110     -34.222  -9.231  98.593  1.00 40.32           C
ATOM    866  SD  MET A 110     -33.067  -9.017  99.988  1.00 67.95           S
ATOM    867  CE  MET A 110     -31.758 -10.224  99.627  1.00 33.01           C
ATOM    868  N   VAL A 111     -36.046  -7.977  95.455  1.00 33.03           N
ATOM    869  CA  VAL A 111     -36.549  -6.745  94.860  1.00 33.46           C
ATOM    870  C   VAL A 111     -37.261  -5.962  95.940  1.00 32.80           C
ATOM    871  O   VAL A 111     -38.250  -6.441  96.502  1.00 34.50           O
ATOM    872  CB  VAL A 111     -37.500  -7.004  93.679  1.00 34.14           C
ATOM    873  CG1 VAL A 111     -38.216  -5.736  93.323  1.00 25.66           C
ATOM    874  CG2 VAL A 111     -36.748  -7.559  92.470  1.00 30.59           C
ATOM    875  N   THR A 112     -36.777  -4.756  96.211  1.00 33.30           N
ATOM    876  CA  THR A 112     -37.383  -3.856  97.176  1.00 31.95           C
ATOM    877  C   THR A 112     -37.950  -2.664  96.425  1.00 36.12           C
ATOM    878  O   THR A 112     -37.249  -2.038  95.621  1.00 37.45           O
ATOM    879  CB  THR A 112     -36.362  -3.387  98.207  1.00 31.08           C
ATOM    880  OG1 THR A 112     -35.660  -4.523  98.708  1.00 33.39           O
ATOM    881  CG2 THR A 112     -37.042  -2.661  99.366  1.00 29.77           C
ATOM    882  N   VAL A 113     -39.210  -2.344  96.684  1.00 31.43           N
ATOM    883  CA  VAL A 113     -39.827  -1.173  96.089  1.00 36.35           C
ATOM    884  C   VAL A 113     -40.215  -0.226  97.216  1.00 36.75           C
ATOM    885  O   VAL A 113     -41.039  -0.569  98.072  1.00 39.18           O
ATOM    886  CB  VAL A 113     -41.043  -1.550  95.234  1.00 34.86           C
ATOM    887  CG1 VAL A 113     -41.651  -0.305  94.602  1.00 34.99           C
ATOM    888  CG2 VAL A 113     -40.631  -2.533  94.188  1.00 34.01           C
ATOM    889  N   SER A 114     -39.653   0.974  97.192  1.00 36.38           N
ATOM    890  CA  SER A 114     -39.797   1.848  98.340  1.00 36.28           C
ATOM    891  C   SER A 114     -39.395   3.273  97.977  1.00 40.12           C
ATOM    892  O   SER A 114     -38.525   3.507  97.123  1.00 40.70           O
ATOM    893  CB  SER A 114     -38.954   1.341  99.507  1.00 31.28           C
ATOM    894  OG  SER A 114     -38.863   2.336 100.505  1.00 39.09           O
ATOM    895  N   SER A 115     -40.017   4.217  98.672  1.00 38.69           N
ATOM    896  CA  SER A 115     -39.621   5.613  98.576  1.00 42.10           C
ATOM    897  C   SER A 115     -38.296   5.892  99.267  1.00 46.74           C
ATOM    898  O   SER A 115     -37.675   6.920  98.973  1.00 42.92           O
ATOM    899  CB  SER A 115     -40.717   6.490  99.162  1.00 39.28           C
ATOM    900  OG  SER A 115     -41.885   6.392  98.349  1.00 47.59           O
ATOM    901  N   ALA A 116     -37.853   5.008 100.168  1.00 39.54           N
ATOM    902  CA  ALA A 116     -36.656   5.257 100.954  1.00 35.81           C
ATOM    903  C   ALA A 116     -35.417   5.270 100.067  1.00 37.57           C
ATOM    904  O   ALA A 116     -35.411   4.730  98.963  1.00 38.65           O
ATOM    905  CB  ALA A 116     -36.507   4.199 102.035  1.00 35.27           C
ATOM    906  N   SER A 117     -34.347   5.880 100.571  1.00 38.00           N
ATOM    907  CA  SER A 117     -33.079   5.924  99.855  1.00 40.96           C
ATOM    908  C   SER A 117     -32.040   5.082 100.572  1.00 39.82           C
ATOM    909  O   SER A 117     -32.108   4.893 101.790  1.00 40.84           O
ATOM    910  CB  SER A 117     -32.561   7.352  99.715  1.00 38.33           C
ATOM    911  OG  SER A 117     -33.448   8.084  98.896  1.00 52.15           O
ATOM    912  N   THR A 118     -31.078   4.576  99.803  1.00 34.77           N
ATOM    913  CA  THR A 118     -30.059   3.709 100.378  1.00 35.77           C
ATOM    914  C   THR A 118     -29.387   4.392 101.560  1.00 40.75           C
ATOM    915  O   THR A 118     -29.127   5.595 101.535  1.00 45.66           O
ATOM    916  CB  THR A 118     -29.030   3.346  99.320  1.00 33.58           C
ATOM    917  OG1 THR A 118     -29.662   2.534  98.320  1.00 42.35           O
ATOM    918  CG2 THR A 118     -27.854   2.607  99.941  1.00 35.40           C
ATOM    919  N   LYS A 119     -29.152   3.629 102.621  1.00 37.56           N
ATOM    920  CA  LYS A 119     -28.635   4.215 103.848  1.00 36.23           C
ATOM    921  C   LYS A 119     -28.000   3.126 104.697  1.00 36.78           C
```

```
ATOM    922  O    LYS A 119     -28.629   2.100 104.959  1.00 31.04           O
ATOM    923  CB   LYS A 119     -29.742   4.904 104.631  1.00 36.08           C
ATOM    924  CG   LYS A 119     -29.224   5.571 105.867  1.00 32.45           C
ATOM    925  CD   LYS A 119     -30.331   6.005 106.761  1.00 36.11           C
ATOM    926  CE   LYS A 119     -29.719   6.656 107.950  1.00 41.55           C
ATOM    927  NZ   LYS A 119     -28.719   5.693 108.485  1.00 40.27           N1+
ATOM    928  N    GLY A 120     -26.761   3.351 105.115  1.00 32.34           N
ATOM    929  CA   GLY A 120     -26.058   2.407 105.937  1.00 32.74           C
ATOM    930  C    GLY A 120     -26.563   2.417 107.364  1.00 33.41           C
ATOM    931  O    GLY A 120     -27.264   3.331 107.801  1.00 34.70           O
ATOM    932  N    PRO A 121     -26.210   1.393 108.120  1.00 31.38           N
ATOM    933  CA   PRO A 121     -26.705   1.283 109.493  1.00 36.27           C
ATOM    934  C    PRO A 121     -25.863   2.075 110.478  1.00 29.36           C
ATOM    935  O    PRO A 121     -24.723   2.442 110.213  1.00 28.70           O
ATOM    936  CB   PRO A 121     -26.592  -0.217 109.784  1.00 32.75           C
ATOM    937  CG   PRO A 121     -25.450  -0.653 108.950  1.00 31.60           C
ATOM    938  CD   PRO A 121     -25.485   0.190 107.692  1.00 31.22           C
ATOM    939  N    SER A 122     -26.481   2.365 111.614  1.00 28.35           N
ATOM    940  CA   SER A 122     -25.790   2.748 112.834  1.00 27.65           C
ATOM    941  C    SER A 122     -25.766   1.529 113.743  1.00 28.89           C
ATOM    942  O    SER A 122     -26.780   0.843 113.874  1.00 33.72           O
ATOM    943  CB   SER A 122     -26.490   3.915 113.528  1.00 27.60           C
ATOM    944  OG   SER A 122     -26.537   5.035 112.665  1.00 32.84           O
ATOM    945  N    VAL A 123     -24.615   1.239 114.341  1.00 30.13           N
ATOM    946  CA   VAL A 123     -24.439   0.062 115.188  1.00 29.26           C
ATOM    947  C    VAL A 123     -24.266   0.513 116.638  1.00 28.50           C
ATOM    948  O    VAL A 123     -23.290   1.188 116.982  1.00 36.71           O
ATOM    949  CB   VAL A 123     -23.257  -0.800 114.713  1.00 29.30           C
ATOM    950  CG1  VAL A 123     -23.161  -2.079 115.535  1.00 32.16           C
ATOM    951  CG2  VAL A 123     -23.421  -1.143 113.243  1.00 29.73           C
ATOM    952  N    PHE A 124     -25.204   0.154 117.477  1.00 29.11           N
ATOM    953  CA   PHE A 124     -25.117   0.453 118.892  1.00 30.43           C
ATOM    954  C    PHE A 124     -24.914  -0.818 119.710  1.00 30.05           C
ATOM    955  O    PHE A 124     -25.432  -1.871 119.341  1.00 28.55           O
ATOM    956  CB   PHE A 124     -26.387   1.149 119.375  1.00 29.02           C
ATOM    957  CG   PHE A 124     -26.765   2.328 118.559  1.00 31.89           C
ATOM    958  CD1  PHE A 124     -25.952   3.448 118.525  1.00 37.36           C
ATOM    959  CD2  PHE A 124     -27.948   2.345 117.848  1.00 33.36           C
ATOM    960  CE1  PHE A 124     -26.309   4.550 117.784  1.00 33.03           C
ATOM    961  CE2  PHE A 124     -28.307   3.451 117.112  1.00 32.67           C
ATOM    962  CZ   PHE A 124     -27.486   4.548 117.085  1.00 31.27           C
ATOM    963  N    PRO A 125     -24.212  -0.744 120.845  1.00 34.75           N
ATOM    964  CA   PRO A 125     -23.997  -1.942 121.663  1.00 33.50           C
ATOM    965  C    PRO A 125     -25.209  -2.306 122.514  1.00 33.10           C
ATOM    966  O    PRO A 125     -25.956  -1.452 122.991  1.00 31.28           O
ATOM    967  CB   PRO A 125     -22.808  -1.549 122.546  1.00 27.55           C
ATOM    968  CG   PRO A 125     -22.913  -0.105 122.656  1.00 24.38           C
ATOM    969  CD   PRO A 125     -23.459   0.407 121.378  1.00 30.67           C
ATOM    970  N    LEU A 126     -25.395  -3.609 122.688  1.00 29.05           N
ATOM    971  CA   LEU A 126     -26.264  -4.145 123.724  1.00 31.66           C
ATOM    972  C    LEU A 126     -25.315  -4.627 124.801  1.00 33.65           C
ATOM    973  O    LEU A 126     -24.728  -5.701 124.688  1.00 37.65           O
ATOM    974  CB   LEU A 126     -27.167  -5.249 123.195  1.00 30.81           C
ATOM    975  CG   LEU A 126     -27.997  -4.672 122.052  1.00 29.82           C
ATOM    976  CD1  LEU A 126     -28.827  -5.730 121.357  1.00 26.48           C
ATOM    977  CD2  LEU A 126     -28.860  -3.574 122.603  1.00 30.33           C
ATOM    978  N    ALA A 127     -25.143  -3.805 125.835  1.00 38.61           N
ATOM    979  CA   ALA A 127     -24.053  -4.011 126.768  1.00 36.09           C
ATOM    980  C    ALA A 127     -24.399  -5.128 127.738  1.00 40.57           C
ATOM    981  O    ALA A 127     -25.510  -5.137 128.289  1.00 36.21           O
ATOM    982  CB   ALA A 127     -23.763  -2.733 127.529  1.00 35.02           C
ATOM    983  N    PRO A 128     -23.478  -6.062 127.986  1.00 38.34           N
ATOM    984  CA   PRO A 128     -23.759  -7.137 128.941  1.00 45.51           C
ATOM    985  C    PRO A 128     -24.091  -6.569 130.309  1.00 56.56           C
ATOM    986  O    PRO A 128     -23.360  -5.736 130.865  1.00 52.82           O
ATOM    987  CB   PRO A 128     -22.463  -7.953 128.968  1.00 43.58           C
ATOM    988  CG   PRO A 128     -21.433  -7.043 128.440  1.00 45.25           C
ATOM    989  CD   PRO A 128     -22.124  -6.162 127.441  1.00 39.79           C
ATOM    990  N    SER A 129     -25.212  -7.049 130.837  1.00 66.36           N
ATOM    991  CA   SER A 129     -25.759  -6.677 132.128  1.00 72.65           C
ATOM    992  C    SER A 129     -24.673  -6.810 133.195  1.00 76.84           C
```

| ATOM | 993 | O | SER | A | 129 | -24.175 | -7.914 | 133.453 | 1.00 | 78.41 | O |
|------|------|-----|-----|---|-----|---------|---------|---------|------|-------|-----|
| ATOM | 994 | CB | SER | A | 129 | -26.983 | -7.568 | 132.406 | 1.00 | 69.58 | C |
| ATOM | 995 | OG | SER | A | 129 | -27.910 | -7.001 | 133.309 | 1.00 | 70.69 | O |
| ATOM | 996 | N | SER | A | 130 | -24.252 | -5.681 | 133.776 | 1.00 | 77.98 | N |
| ATOM | 997 | CA | SER | A | 130 | -23.377 | -5.731 | 134.943 | 1.00 | 85.61 | C |
| ATOM | 998 | C | SER | A | 130 | -23.994 | -6.604 | 136.037 | 1.00 | 90.91 | C |
| ATOM | 999 | O | SER | A | 130 | -23.273 | -7.223 | 136.835 | 1.00 | 87.54 | O |
| ATOM | 1000 | CB | SER | A | 130 | -23.089 | -4.307 | 135.444 | 1.00 | 90.00 | C |
| ATOM | 1001 | OG | SER | A | 130 | -24.274 | -3.529 | 135.594 | 1.00 | 85.47 | O |
| ATOM | 1002 | N | LYS | A | 131 | -25.331 | -6.708 | 136.043 | 1.00 | 95.09 | N |
| ATOM | 1003 | CA | LYS | A | 131 | -26.101 | -7.524 | 136.974 | 1.00 | 92.61 | C |
| ATOM | 1004 | C | LYS | A | 131 | -26.337 | -8.936 | 136.436 | 1.00 | 92.99 | C |
| ATOM | 1005 | O | LYS | A | 131 | -27.391 | -9.540 | 136.695 | 1.00 | 88.38 | O |
| ATOM | 1006 | CB | LYS | A | 131 | -27.436 | -6.839 | 137.284 | 1.00 | 84.94 | C |
| ATOM | 1007 | CG | LYS | A | 131 | -27.308 | -5.523 | 138.053 | 1.00 | 86.39 | C |
| ATOM | 1008 | CD | LYS | A | 131 | -28.673 | -4.977 | 138.437 | 1.00 | 85.07 | C |
| ATOM | 1009 | CE | LYS | A | 131 | -28.583 | -4.070 | 139.646 | 1.00 | 74.74 | C |
| ATOM | 1010 | NZ | LYS | A | 131 | -29.939 | -3.594 | 140.022 | 1.00 | 70.33 | N1+ |
| ATOM | 1011 | N | SER | A | 132 | -25.386 | -9.453 | 135.655 | 1.00 | 91.67 | N |
| ATOM | 1012 | CA | SER | A | 132 | -25.423 | -10.837 | 135.210 | 1.00 | 89.98 | C |
| ATOM | 1013 | C | SER | A | 132 | -25.152 | -11.744 | 136.398 | 1.00 | 95.97 | C |
| ATOM | 1014 | O | SER | A | 132 | -24.296 | -11.439 | 137.237 | 1.00 | 97.89 | O |
| ATOM | 1015 | CB | SER | A | 132 | -24.366 | -11.076 | 134.130 | 1.00 | 80.08 | C |
| ATOM | 1016 | OG | SER | A | 132 | -24.779 | -10.567 | 132.876 | 1.00 | 75.09 | O |
| ATOM | 1017 | N | THR | A | 133 | -25.903 | -12.846 | 136.495 | 1.00 | 96.88 | N |
| ATOM | 1018 | CA | THR | A | 133 | -25.589 | -13.828 | 137.529 | 1.00 | 94.98 | C |
| ATOM | 1019 | C | THR | A | 133 | -24.133 | -14.276 | 137.346 | 1.00 | 90.31 | C |
| ATOM | 1020 | O | THR | A | 133 | -23.836 | -15.107 | 136.480 | 1.00 | 81.64 | O |
| ATOM | 1021 | CB | THR | A | 133 | -26.595 | -15.001 | 137.481 | 1.00 | 90.13 | C |
| ATOM | 1022 | OG1 | THR | A | 133 | -26.943 | -15.313 | 136.117 | 1.00 | 86.99 | O |
| ATOM | 1023 | CG2 | THR | A | 133 | -27.872 | -14.664 | 138.271 | 1.00 | 77.20 | C |
| ATOM | 1024 | N | SER | A | 134 | -23.219 | -13.722 | 138.160 | 1.00 | 93.80 | N |
| ATOM | 1025 | CA | SER | A | 134 | -21.784 | -13.900 | 137.935 | 1.00 | 90.34 | C |
| ATOM | 1026 | C | SER | A | 134 | -21.364 | -15.323 | 138.275 | 1.00 | 87.03 | C |
| ATOM | 1027 | O | SER | A | 134 | -21.597 | -15.802 | 139.389 | 1.00 | 88.65 | O |
| ATOM | 1028 | CB | SER | A | 134 | -20.971 | -12.892 | 138.748 | 1.00 | 81.79 | C |
| ATOM | 1029 | OG | SER | A | 134 | -19.872 | -12.426 | 137.986 | 1.00 | 69.79 | O |
| ATOM | 1030 | N | GLY | A | 135 | -20.701 | -15.981 | 137.329 | 1.00 | 83.14 | N |
| ATOM | 1031 | CA | GLY | A | 135 | -20.552 | -17.418 | 137.366 | 1.00 | 79.97 | C |
| ATOM | 1032 | C | GLY | A | 135 | -21.634 | -18.150 | 136.607 | 1.00 | 75.17 | C |
| ATOM | 1033 | O | GLY | A | 135 | -21.672 | -19.387 | 136.643 | 1.00 | 70.13 | O |
| ATOM | 1034 | N | GLY | A | 136 | -22.531 | -17.411 | 135.954 | 1.00 | 73.84 | N |
| ATOM | 1035 | CA | GLY | A | 136 | -23.577 | -17.924 | 135.095 | 1.00 | 61.25 | C |
| ATOM | 1036 | C | GLY | A | 136 | -23.388 | -17.420 | 133.676 | 1.00 | 63.22 | C |
| ATOM | 1037 | O | GLY | A | 136 | -22.265 | -17.397 | 133.152 | 1.00 | 56.06 | O |
| ATOM | 1038 | N | THR | A | 137 | -24.476 | -16.996 | 133.040 | 1.00 | 59.59 | N |
| ATOM | 1039 | CA | THR | A | 137 | -24.446 | -16.674 | 131.626 | 1.00 | 48.48 | C |
| ATOM | 1040 | C | THR | A | 137 | -24.845 | -15.227 | 131.387 | 1.00 | 51.06 | C |
| ATOM | 1041 | O | THR | A | 137 | -25.789 | -14.712 | 132.002 | 1.00 | 56.26 | O |
| ATOM | 1042 | CB | THR | A | 137 | -25.342 | -17.624 | 130.849 | 1.00 | 47.89 | C |
| ATOM | 1043 | OG1 | THR | A | 137 | -24.756 | -18.935 | 130.893 | 1.00 | 52.57 | O |
| ATOM | 1044 | CG2 | THR | A | 137 | -25.461 | -17.182 | 129.398 | 1.00 | 39.41 | C |
| ATOM | 1045 | N | ALA | A | 138 | -24.088 | -14.574 | 130.510 | 1.00 | 44.71 | N |
| ATOM | 1046 | CA | ALA | A | 138 | -24.313 | -13.195 | 130.117 | 1.00 | 41.78 | C |
| ATOM | 1047 | C | ALA | A | 138 | -24.782 | -13.142 | 128.670 | 1.00 | 43.49 | C |
| ATOM | 1048 | O | ALA | A | 138 | -24.337 | -13.933 | 127.833 | 1.00 | 41.75 | O |
| ATOM | 1049 | CB | ALA | A | 138 | -23.036 | -12.369 | 130.273 | 1.00 | 44.03 | C |
| ATOM | 1050 | N | ALA | A | 139 | -25.689 | -12.215 | 128.384 | 1.00 | 43.46 | N |
| ATOM | 1051 | CA | ALA | A | 139 | -26.067 | -11.872 | 127.022 | 1.00 | 39.95 | C |
| ATOM | 1052 | C | ALA | A | 139 | -25.455 | -10.522 | 126.665 | 1.00 | 35.35 | C |
| ATOM | 1053 | O | ALA | A | 139 | -25.393 | -9.618 | 127.504 | 1.00 | 31.47 | O |
| ATOM | 1054 | CB | ALA | A | 139 | -27.593 | -11.827 | 126.865 | 1.00 | 27.83 | C |
| ATOM | 1055 | N | LEU | A | 140 | -25.005 | -10.399 | 125.419 | 1.00 | 30.62 | N |
| ATOM | 1056 | CA | LEU | A | 140 | -24.563 | -9.129 | 124.863 | 1.00 | 32.63 | C |
| ATOM | 1057 | C | LEU | A | 140 | -24.915 | -9.129 | 123.382 | 1.00 | 32.74 | C |
| ATOM | 1058 | O | LEU | A | 140 | -25.288 | -10.161 | 122.824 | 1.00 | 33.31 | O |
| ATOM | 1059 | CB | LEU | A | 140 | -23.067 | -8.925 | 125.098 | 1.00 | 36.31 | C |
| ATOM | 1060 | CG | LEU | A | 140 | -22.182 | -10.012 | 124.493 | 1.00 | 33.45 | C |
| ATOM | 1061 | CD1 | LEU | A | 140 | -21.538 | -9.500 | 123.223 | 1.00 | 31.90 | C |
| ATOM | 1062 | CD2 | LEU | A | 140 | -21.146 | -10.511 | 125.497 | 1.00 | 33.92 | C |
| ATOM | 1063 | N | GLY | A | 141 | -24.839 | -7.966 | 122.745 | 1.00 | 31.54 | N |

```
ATOM   1064  CA  GLY A 141     -25.151  -7.940 121.332  1.00 28.33           C
ATOM   1065  C   GLY A 141     -24.948  -6.589 120.690  1.00 27.25           C
ATOM   1066  O   GLY A 141     -24.461  -5.640 121.304  1.00 31.88           O
ATOM   1067  N   CYS A 142     -25.353  -6.529 119.428  1.00 25.30           N
ATOM   1068  CA  CYS A 142     -25.336  -5.319 118.623  1.00 28.40           C
ATOM   1069  C   CYS A 142     -26.729  -5.018 118.105  1.00 27.36           C
ATOM   1070  O   CYS A 142     -27.484  -5.930 117.757  1.00 27.52           O
ATOM   1071  CB  CYS A 142     -24.402  -5.447 117.435  1.00 30.69           C
ATOM   1072  SG  CYS A 142     -22.691  -5.228 117.879  1.00 51.18           S
ATOM   1073  N   LEU A 143     -27.056  -3.735 118.049  1.00 27.56           N
ATOM   1074  CA  LEU A 143     -28.293  -3.248 117.460  1.00 27.81           C
ATOM   1075  C   LEU A 143     -27.931  -2.565 116.145  1.00 27.63           C
ATOM   1076  O   LEU A 143     -27.155  -1.613 116.136  1.00 31.79           O
ATOM   1077  CB  LEU A 143     -28.985  -2.274 118.406  1.00 31.36           C
ATOM   1078  CG  LEU A 143     -30.346  -1.766 117.963  1.00 31.23           C
ATOM   1079  CD1 LEU A 143     -31.250  -2.954 117.862  1.00 28.10           C
ATOM   1080  CD2 LEU A 143     -30.899  -0.706 118.886  1.00 32.28           C
ATOM   1081  N   VAL A 144     -28.436  -3.083 115.036  1.00 30.74           N
ATOM   1082  CA  VAL A 144     -28.101  -2.590 113.702  1.00 27.82           C
ATOM   1083  C   VAL A 144     -29.311  -1.800 113.216  1.00 30.66           C
ATOM   1084  O   VAL A 144     -30.289  -2.381 112.744  1.00 30.67           O
ATOM   1085  CB  VAL A 144     -27.732  -3.744 112.769  1.00 28.14           C
ATOM   1086  CG1 VAL A 144     -27.347  -3.252 111.375  1.00 23.30           C
ATOM   1087  CG2 VAL A 144     -26.591  -4.541 113.411  1.00 25.34           C
ATOM   1088  N   LYS A 145     -29.269  -0.470 113.374  1.00 33.19           N
ATOM   1089  CA  LYS A 145     -30.446   0.383 113.245  1.00 31.94           C
ATOM   1090  C   LYS A 145     -30.392   1.292 112.020  1.00 34.55           C
ATOM   1091  O   LYS A 145     -29.322   1.758 111.612  1.00 32.70           O
ATOM   1092  CB  LYS A 145     -30.637   1.229 114.504  1.00 34.83           C
ATOM   1093  CG  LYS A 145     -32.055   1.752 114.654  1.00 39.24           C
ATOM   1094  CD  LYS A 145     -32.309   2.302 116.035  1.00 37.76           C
ATOM   1095  CE  LYS A 145     -33.802   2.550 116.282  1.00 47.55           C
ATOM   1096  NZ  LYS A 145     -34.483   3.453 115.296  1.00 48.57           N1+
ATOM   1097  N   ASP A 146     -31.564   1.494 111.414  1.00 35.65           N
ATOM   1098  CA  ASP A 146     -31.815   2.517 110.399  1.00 31.21           C
ATOM   1099  C   ASP A 146     -30.985   2.308 109.134  1.00 34.08           C
ATOM   1100  O   ASP A 146     -30.204   3.170 108.721  1.00 35.04           O
ATOM   1101  CB  ASP A 146     -31.568   3.919 110.960  1.00 31.93           C
ATOM   1102  CG  ASP A 146     -32.501   4.276 112.097  1.00 39.35           C
ATOM   1103  OD1 ASP A 146     -33.621   3.731 112.154  1.00 37.78           O
ATOM   1104  OD2 ASP A 146     -32.150   5.184 112.878  1.00 48.93           O1-
ATOM   1105  N   TYR A 147     -31.184   1.169 108.490  1.00 35.70           N
ATOM   1106  CA  TYR A 147     -30.527   0.965 107.205  1.00 32.13           C
ATOM   1107  C   TYR A 147     -31.548   0.662 106.127  1.00 30.25           C
ATOM   1108  O   TYR A 147     -32.711   0.385 106.401  1.00 34.62           O
ATOM   1109  CB  TYR A 147     -29.480  -0.146 107.270  1.00 29.22           C
ATOM   1110  CG  TYR A 147     -30.026  -1.497 107.584  1.00 31.09           C
ATOM   1111  CD1 TYR A 147     -30.476  -2.344 106.572  1.00 34.27           C
ATOM   1112  CD2 TYR A 147     -30.068  -1.953 108.896  1.00 31.93           C
ATOM   1113  CE1 TYR A 147     -30.965  -3.615 106.863  1.00 33.60           C
ATOM   1114  CE2 TYR A 147     -30.547  -3.216 109.201  1.00 34.30           C
ATOM   1115  CZ  TYR A 147     -30.998  -4.039 108.186  1.00 35.75           C
ATOM   1116  OH  TYR A 147     -31.474  -5.283 108.511  1.00 33.90           O
ATOM   1117  N   PHE A 148     -31.087   0.720 104.889  1.00 36.14           N
ATOM   1118  CA  PHE A 148     -31.931   0.444 103.740  1.00 36.37           C
ATOM   1119  C   PHE A 148     -31.063   0.353 102.506  1.00 35.17           C
ATOM   1120  O   PHE A 148     -30.171   1.168 102.324  1.00 40.37           O
ATOM   1121  CB  PHE A 148     -32.994   1.533 103.573  1.00 35.29           C
ATOM   1122  CG  PHE A 148     -33.914   1.300 102.429  1.00 38.94           C
ATOM   1123  CD2 PHE A 148     -33.582   1.733 101.154  1.00 37.28           C
ATOM   1124  CD1 PHE A 148     -35.116   0.633 102.619  1.00 43.66           C
ATOM   1125  CE2 PHE A 148     -34.431   1.509 100.090  1.00 40.33           C
ATOM   1126  CE1 PHE A 148     -35.980   0.405 101.558  1.00 40.99           C
ATOM   1127  CZ  PHE A 148     -35.632   0.843 100.288  1.00 41.29           C
ATOM   1128  N   PRO A 149     -31.317  -0.641 101.646  1.00 38.61           N
ATOM   1129  CA  PRO A 149     -32.320  -1.693 101.818  1.00 36.40           C
ATOM   1130  C   PRO A 149     -31.736  -2.913 102.534  1.00 32.84           C
ATOM   1131  O   PRO A 149     -30.607  -2.867 103.010  1.00 32.70           O
ATOM   1132  CB  PRO A 149     -32.679  -2.036 100.378  1.00 36.43           C
ATOM   1133  CG  PRO A 149     -31.331  -1.921  99.683  1.00 33.06           C
ATOM   1134  CD  PRO A 149     -30.649  -0.743 100.332  1.00 34.54           C
```

```
ATOM   1135  N    GLU A 150     -32.499  -3.995 102.601  1.00 33.19           N
ATOM   1136  CA   GLU A 150     -31.952  -5.302 102.970  1.00 32.31           C
ATOM   1137  C    GLU A 150     -30.980  -5.788 101.891  1.00 29.80           C
ATOM   1138  O    GLU A 150     -31.160  -5.456 100.713  1.00 29.74           O
ATOM   1139  CB   GLU A 150     -33.087  -6.301 103.166  1.00 28.58           C
ATOM   1140  CG   GLU A 150     -33.977  -6.000 104.360  1.00 29.38           C
ATOM   1141  CD   GLU A 150     -33.563  -6.767 105.602  1.00 39.48           C
ATOM   1142  OE1  GLU A 150     -34.378  -7.590 106.099  1.00 41.15           O
ATOM   1143  OE2  GLU A 150     -32.413  -6.566 106.065  1.00 41.49           O1-
ATOM   1144  N    PRO A 151     -29.992  -6.630 102.263  1.00 31.91           N
ATOM   1145  CA   PRO A 151     -29.735  -7.230 103.581  1.00 31.76           C
ATOM   1146  C    PRO A 151     -28.502  -6.701 104.317  1.00 35.19           C
ATOM   1147  O    PRO A 151     -27.684  -5.998 103.713  1.00 34.57           O
ATOM   1148  CB   PRO A 151     -29.510  -8.690 103.223  1.00 23.34           C
ATOM   1149  CG   PRO A 151     -28.788  -8.605 101.939  1.00 20.46           C
ATOM   1150  CD   PRO A 151     -29.218  -7.334 101.225  1.00 25.12           C
ATOM   1151  N    VAL A 152     -28.379  -7.046 105.602  1.00 30.30           N
ATOM   1152  CA   VAL A 152     -27.128  -6.911 106.335  1.00 29.66           C
ATOM   1153  C    VAL A 152     -26.726  -8.297 106.815  1.00 33.28           C
ATOM   1154  O    VAL A 152     -27.580  -9.147 107.078  1.00 31.82           O
ATOM   1155  CB   VAL A 152     -27.221  -5.959 107.550  1.00 31.08           C
ATOM   1156  CG1  VAL A 152     -27.344  -4.523 107.116  1.00 36.88           C
ATOM   1157  CG2  VAL A 152     -28.382  -6.349 108.403  1.00 32.42           C
ATOM   1158  N    THR A 153     -25.419  -8.509 106.967  1.00 32.49           N
ATOM   1159  CA   THR A 153     -24.873  -9.711 107.585  1.00 31.87           C
ATOM   1160  C    THR A 153     -24.123  -9.336 108.858  1.00 33.40           C
ATOM   1161  O    THR A 153     -23.517  -8.264 108.952  1.00 35.81           O
ATOM   1162  CB   THR A 153     -23.924 -10.442 106.644  1.00 27.89           C
ATOM   1163  OG1  THR A 153     -22.904  -9.528 106.239  1.00 36.53           O
ATOM   1164  CG2  THR A 153     -24.676 -10.910 105.408  1.00 29.45           C
ATOM   1165  N    VAL A 154     -24.189 -10.213 109.852  1.00 24.86           N
ATOM   1166  CA   VAL A 154     -23.508 -10.013 111.116  1.00 27.57           C
ATOM   1167  C    VAL A 154     -22.745 -11.281 111.472  1.00 29.28           C
ATOM   1168  O    VAL A 154     -23.316 -12.374 111.480  1.00 35.29           O
ATOM   1169  CB   VAL A 154     -24.499  -9.646 112.240  1.00 30.65           C
ATOM   1170  CG1  VAL A 154     -23.770  -9.393 113.542  1.00 30.74           C
ATOM   1171  CG2  VAL A 154     -25.312  -8.454 111.849  1.00 29.70           C
ATOM   1172  N    SER A 155     -21.463 -11.137 111.766  1.00 29.75           N
ATOM   1173  CA   SER A 155     -20.720 -12.183 112.446  1.00 32.79           C
ATOM   1174  C    SER A 155     -20.100 -11.604 113.711  1.00 36.14           C
ATOM   1175  O    SER A 155     -20.134 -10.393 113.961  1.00 34.64           O
ATOM   1176  CB   SER A 155     -19.648 -12.800 111.550  1.00 28.09           C
ATOM   1177  OG   SER A 155     -18.682 -11.839 111.164  1.00 32.25           O
ATOM   1178  N    TRP A 156     -19.522 -12.495 114.504  1.00 28.20           N
ATOM   1179  CA   TRP A 156     -18.940 -12.149 115.781  1.00 30.93           C
ATOM   1180  C    TRP A 156     -17.495 -12.610 115.807  1.00 35.40           C
ATOM   1181  O    TRP A 156     -17.202 -13.750 115.427  1.00 34.28           O
ATOM   1182  CB   TRP A 156     -19.731 -12.786 116.913  1.00 30.86           C
ATOM   1183  CG   TRP A 156     -20.988 -12.046 117.160  1.00 32.29           C
ATOM   1184  CD1  TRP A 156     -22.184 -12.252 116.553  1.00 32.31           C
ATOM   1185  CD2  TRP A 156     -21.168 -10.939 118.048  1.00 31.77           C
ATOM   1186  NE1  TRP A 156     -23.106 -11.348 117.012  1.00 33.15           N
ATOM   1187  CE2  TRP A 156     -22.508 -10.529 117.933  1.00 31.90           C
ATOM   1188  CE3  TRP A 156     -20.325 -10.253 118.929  1.00 33.09           C
ATOM   1189  CZ2  TRP A 156     -23.037  -9.477 118.679  1.00 29.54           C
ATOM   1190  CZ3  TRP A 156     -20.852  -9.197 119.665  1.00 34.64           C
ATOM   1191  CH2  TRP A 156     -22.199  -8.828 119.539  1.00 31.80           C
ATOM   1192  N    ASN A 157     -16.603 -11.723 116.262  1.00 28.12           N
ATOM   1193  CA   ASN A 157     -15.185 -12.020 116.346  1.00 27.84           C
ATOM   1194  C    ASN A 157     -14.675 -12.543 115.010  1.00 31.80           C
ATOM   1195  O    ASN A 157     -13.925 -13.515 114.956  1.00 33.84           O
ATOM   1196  CB   ASN A 157     -14.888 -13.031 117.461  1.00 29.77           C
ATOM   1197  CG   ASN A 157     -15.039 -12.460 118.845  1.00 26.46           C
ATOM   1198  OD1  ASN A 157     -15.346 -11.294 119.024  1.00 30.29           O
ATOM   1199  ND2  ASN A 157     -14.823 -13.294 119.842  1.00 33.06           N
ATOM   1200  N    SER A 158     -15.102 -11.898 113.922  1.00 31.97           N
ATOM   1201  CA   SER A 158     -14.661 -12.248 112.570  1.00 33.51           C
ATOM   1202  C    SER A 158     -14.956 -13.699 112.215  1.00 36.13           C
ATOM   1203  O    SER A 158     -14.224 -14.307 111.438  1.00 42.62           O
ATOM   1204  CB   SER A 158     -13.170 -11.972 112.375  1.00 28.57           C
ATOM   1205  OG   SER A 158     -12.839 -10.678 112.818  1.00 41.72           O
```

```
ATOM   1206  N    GLY A 159     -16.007 -14.278 112.791  1.00 36.17           N
ATOM   1207  CA   GLY A 159     -16.371 -15.654 112.532  1.00 30.18           C
ATOM   1208  C    GLY A 159     -15.840 -16.648 113.540  1.00 32.92           C
ATOM   1209  O    GLY A 159     -16.226 -17.823 113.497  1.00 29.97           O
ATOM   1210  N    ALA A 160     -14.960 -16.217 114.437  1.00 37.02           N
ATOM   1211  CA   ALA A 160     -14.420 -17.132 115.431  1.00 32.79           C
ATOM   1212  C    ALA A 160     -15.442 -17.476 116.505  1.00 34.92           C
ATOM   1213  O    ALA A 160     -15.345 -18.540 117.121  1.00 48.32           O
ATOM   1214  CB   ALA A 160     -13.164 -16.532 116.061  1.00 28.82           C
ATOM   1215  N    LEU A 161     -16.416 -16.617 116.751  1.00 35.16           N
ATOM   1216  CA   LEU A 161     -17.432 -16.878 117.759  1.00 33.95           C
ATOM   1217  C    LEU A 161     -18.713 -17.278 117.047  1.00 32.88           C
ATOM   1218  O    LEU A 161     -19.239 -16.519 116.234  1.00 35.85           O
ATOM   1219  CB   LEU A 161     -17.653 -15.658 118.648  1.00 30.18           C
ATOM   1220  CG   LEU A 161     -18.778 -15.719 119.683  1.00 35.02           C
ATOM   1221  CD1  LEU A 161     -18.826 -17.042 120.432  1.00 28.23           C
ATOM   1222  CD2  LEU A 161     -18.673 -14.517 120.661  1.00 33.30           C
ATOM   1223  N    THR A 162     -19.233 -18.450 117.394  1.00 39.79           N
ATOM   1224  CA   THR A 162     -20.298 -19.078 116.630  1.00 33.54           C
ATOM   1225  C    THR A 162     -21.336 -19.691 117.569  1.00 32.35           C
ATOM   1226  O    THR A 162     -22.541 -19.506 117.391  1.00 30.46           O
ATOM   1227  CB   THR A 162     -19.662 -20.099 115.669  1.00 33.49           C
ATOM   1228  OG1  THR A 162     -19.956 -19.724 114.318  1.00 27.42           O
ATOM   1229  CG2  THR A 162     -20.098 -21.543 115.959  1.00 34.98           C
ATOM   1230  N    SER A 163     -20.885 -20.391 118.598  1.00 34.58           N
ATOM   1231  CA   SER A 163     -21.813 -20.925 119.583  1.00 35.61           C
ATOM   1232  C    SER A 163     -22.538 -19.785 120.301  1.00 35.40           C
ATOM   1233  O    SER A 163     -21.925 -18.781 120.681  1.00 37.87           O
ATOM   1234  CB   SER A 163     -21.044 -21.799 120.578  1.00 35.71           C
ATOM   1235  OG   SER A 163     -21.864 -22.247 121.633  1.00 43.49           O
ATOM   1236  N    GLY A 164     -23.856 -19.911 120.442  1.00 28.98           N
ATOM   1237  CA   GLY A 164     -24.625 -18.958 121.206  1.00 25.01           C
ATOM   1238  C    GLY A 164     -25.128 -17.744 120.446  1.00 33.29           C
ATOM   1239  O    GLY A 164     -25.869 -16.946 121.031  1.00 33.20           O
ATOM   1240  N    VAL A 165     -24.754 -17.576 119.160  1.00 30.78           N
ATOM   1241  CA   VAL A 165     -25.156 -16.414 118.374  1.00 25.26           C
ATOM   1242  C    VAL A 165     -26.588 -16.586 117.885  1.00 31.13           C
ATOM   1243  O    VAL A 165     -26.998 -17.679 117.480  1.00 36.05           O
ATOM   1244  CB   VAL A 165     -24.189 -16.187 117.195  1.00 24.24           C
ATOM   1245  CG1  VAL A 165     -24.660 -15.030 116.320  1.00 26.94           C
ATOM   1246  CG2  VAL A 165     -22.806 -15.872 117.701  1.00 26.51           C
ATOM   1247  N    HIS A 166     -27.368 -15.507 117.975  1.00 30.95           N
ATOM   1248  CA   HIS A 166     -28.639 -15.358 117.275  1.00 27.16           C
ATOM   1249  C    HIS A 166     -28.634 -14.011 116.567  1.00 29.61           C
ATOM   1250  O    HIS A 166     -28.486 -12.968 117.218  1.00 28.82           O
ATOM   1251  CB   HIS A 166     -29.841 -15.434 118.225  1.00 26.50           C
ATOM   1252  CG   HIS A 166     -29.913 -16.697 119.017  1.00 29.14           C
ATOM   1253  ND1  HIS A 166     -30.069 -17.932 118.430  1.00 31.57           N
ATOM   1254  CD2  HIS A 166     -29.894 -16.914 120.349  1.00 29.50           C
ATOM   1255  CE1  HIS A 166     -30.123 -18.859 119.368  1.00 30.30           C
ATOM   1256  NE2  HIS A 166     -30.025 -18.267 120.541  1.00 29.10           N
ATOM   1257  N    THR A 167     -28.765 -14.030 115.242  1.00 32.27           N
ATOM   1258  CA   THR A 167     -29.023 -12.828 114.457  1.00 30.55           C
ATOM   1259  C    THR A 167     -30.473 -12.820 113.997  1.00 29.36           C
ATOM   1260  O    THR A 167     -30.914 -13.738 113.306  1.00 34.07           O
ATOM   1261  CB   THR A 167     -28.089 -12.737 113.264  1.00 24.74           C
ATOM   1262  OG1  THR A 167     -26.740 -12.697 113.745  1.00 35.43           O
ATOM   1263  CG2  THR A 167     -28.377 -11.481 112.509  1.00 23.22           C
ATOM   1264  N    PHE A 168     -31.182 -11.777 114.351  1.00 28.41           N
ATOM   1265  CA   PHE A 168     -32.626 -11.776 114.226  1.00 28.58           C
ATOM   1266  C    PHE A 168     -33.064 -11.245 112.869  1.00 30.77           C
ATOM   1267  O    PHE A 168     -32.304 -10.564 112.176  1.00 31.27           O
ATOM   1268  CB   PHE A 168     -33.222 -10.953 115.358  1.00 22.95           C
ATOM   1269  CG   PHE A 168     -33.164 -11.654 116.672  1.00 28.82           C
ATOM   1270  CD1  PHE A 168     -34.177 -12.526 117.052  1.00 30.01           C
ATOM   1271  CD2  PHE A 168     -32.089 -11.484 117.521  1.00 26.59           C
ATOM   1272  CE1  PHE A 168     -34.120 -13.192 118.269  1.00 26.13           C
ATOM   1273  CE2  PHE A 168     -32.034 -12.153 118.738  1.00 24.89           C
ATOM   1274  CZ   PHE A 168     -33.047 -12.999 119.109  1.00 25.58           C
ATOM   1275  N    PRO A 169     -34.268 -11.613 112.428  1.00 30.91           N
ATOM   1276  CA   PRO A 169     -34.825 -10.991 111.221  1.00 28.45           C
```

```
ATOM   1277  C   PRO A 169     -35.017  -9.500 111.445  1.00 30.28           C
ATOM   1278  O   PRO A 169     -35.392  -9.063 112.534  1.00 26.91           O
ATOM   1279  CB  PRO A 169     -36.166 -11.705 111.036  1.00 21.98           C
ATOM   1280  CG  PRO A 169     -36.064 -12.951 111.870  1.00 25.73           C
ATOM   1281  CD  PRO A 169     -35.157 -12.642 112.999  1.00 24.24           C
ATOM   1282  N   ALA A 170     -34.718  -8.713 110.419  1.00 29.94           N
ATOM   1283  CA  ALA A 170     -34.909  -7.277 110.530  1.00 28.66           C
ATOM   1284  C   ALA A 170     -36.397  -6.962 110.631  1.00 24.51           C
ATOM   1285  O   ALA A 170     -37.231  -7.715 110.147  1.00 20.86           O
ATOM   1286  CB  ALA A 170     -34.289  -6.564 109.326  1.00 28.82           C
ATOM   1287  N   VAL A 171     -36.725  -5.833 111.264  1.00 25.89           N
ATOM   1288  CA  VAL A 171     -38.070  -5.279 111.200  1.00 24.76           C
ATOM   1289  C   VAL A 171     -38.012  -4.012 110.358  1.00 28.48           C
ATOM   1290  O   VAL A 171     -36.997  -3.307 110.326  1.00 27.98           O
ATOM   1291  CB  VAL A 171     -38.700  -4.967 112.577  1.00 27.45           C
ATOM   1292  CG1 VAL A 171     -38.882  -6.245 113.387  1.00 27.65           C
ATOM   1293  CG2 VAL A 171     -37.913  -3.890 113.334  1.00 27.22           C
ATOM   1294  N   LEU A 172     -39.111  -3.745 109.653  1.00 25.17           N
ATOM   1295  CA  LEU A 172     -39.301  -2.534 108.867  1.00 27.51           C
ATOM   1296  C   LEU A 172     -40.092  -1.517 109.692  1.00 31.05           C
ATOM   1297  O   LEU A 172     -41.270  -1.730 109.995  1.00 33.68           O
ATOM   1298  CB  LEU A 172     -40.015  -2.862 107.565  1.00 27.53           C
ATOM   1299  CG  LEU A 172     -40.360  -1.652 106.713  1.00 31.73           C
ATOM   1300  CD1 LEU A 172     -39.092  -0.851 106.423  1.00 31.62           C
ATOM   1301  CD2 LEU A 172     -41.011  -2.118 105.405  1.00 28.68           C
ATOM   1302  N   GLN A 173     -39.439  -0.422 110.052  1.00 25.80           N
ATOM   1303  CA  GLN A 173     -40.029   0.612 110.877  1.00 31.71           C
ATOM   1304  C   GLN A 173     -40.841   1.595 110.033  1.00 34.79           C
ATOM   1305  O   GLN A 173     -40.706   1.669 108.805  1.00 31.87           O
ATOM   1306  CB  GLN A 173     -38.945   1.372 111.634  1.00 33.05           C
ATOM   1307  CG  GLN A 173     -37.969   0.495 112.392  1.00 33.17           C
ATOM   1308  CD  GLN A 173     -36.669   1.222 112.686  1.00 35.50           C
ATOM   1309  OE1 GLN A 173     -36.309   1.424 113.850  1.00 39.44           O
ATOM   1310  NE2 GLN A 173     -35.960   1.626 111.629  1.00 33.32           N
ATOM   1311  N   SER A 174     -41.681   2.378 110.726  1.00 34.71           N
ATOM   1312  CA  SER A 174     -42.563   3.323 110.049  1.00 30.21           C
ATOM   1313  C   SER A 174     -41.773   4.343 109.245  1.00 31.66           C
ATOM   1314  O   SER A 174     -42.322   4.937 108.314  1.00 34.94           O
ATOM   1315  CB  SER A 174     -43.461   4.017 111.063  1.00 31.49           C
ATOM   1316  OG  SER A 174     -42.708   4.905 111.882  1.00 40.96           O
ATOM   1317  N   SER A 175     -40.480   4.495 109.533  1.00 29.45           N
ATOM   1318  CA  SER A 175     -39.594   5.363 108.769  1.00 29.62           C
ATOM   1319  C   SER A 175     -39.270   4.831 107.374  1.00 37.39           C
ATOM   1320  O   SER A 175     -38.665   5.564 106.581  1.00 35.03           O
ATOM   1321  CB  SER A 175     -38.288   5.558 109.531  1.00 26.45           C
ATOM   1322  OG  SER A 175     -37.522   4.366 109.537  1.00 33.35           O
ATOM   1323  N   GLY A 176     -39.610   3.574 107.068  1.00 33.52           N
ATOM   1324  CA  GLY A 176     -39.139   2.922 105.865  1.00 26.71           C
ATOM   1325  C   GLY A 176     -37.767   2.308 105.988  1.00 28.94           C
ATOM   1326  O   GLY A 176     -37.323   1.632 105.061  1.00 33.48           O
ATOM   1327  N   LEU A 177     -37.105   2.482 107.118  1.00 31.53           N
ATOM   1328  CA  LEU A 177     -35.796   1.912 107.374  1.00 32.69           C
ATOM   1329  C   LEU A 177     -35.924   0.601 108.155  1.00 33.78           C
ATOM   1330  O   LEU A 177     -36.839   0.412 108.965  1.00 27.20           O
ATOM   1331  CB  LEU A 177     -34.937   2.932 108.138  1.00 30.37           C
ATOM   1332  CG  LEU A 177     -34.691   4.252 107.381  1.00 31.43           C
ATOM   1333  CD1 LEU A 177     -33.897   5.220 108.180  1.00 29.09           C
ATOM   1334  CD2 LEU A 177     -33.948   4.024 106.072  1.00 32.81           C
ATOM   1335  N   TYR A 178     -34.979  -0.299 107.915  1.00 32.33           N
ATOM   1336  CA  TYR A 178     -34.938  -1.580 108.601  1.00 31.28           C
ATOM   1337  C   TYR A 178     -34.061  -1.480 109.848  1.00 31.26           C
ATOM   1338  O   TYR A 178     -33.221  -0.589 109.975  1.00 30.90           O
ATOM   1339  CB  TYR A 178     -34.399  -2.679 107.681  1.00 30.14           C
ATOM   1340  CG  TYR A 178     -35.311  -3.040 106.535  1.00 29.91           C
ATOM   1341  CD2 TYR A 178     -35.140  -2.470 105.275  1.00 30.71           C
ATOM   1342  CD1 TYR A 178     -36.324  -3.960 106.703  1.00 28.37           C
ATOM   1343  CE2 TYR A 178     -35.966  -2.793 104.226  1.00 32.21           C
ATOM   1344  CE1 TYR A 178     -37.160  -4.287 105.667  1.00 29.97           C
ATOM   1345  CZ  TYR A 178     -36.984  -3.704 104.426  1.00 34.62           C
ATOM   1346  OH  TYR A 178     -37.822  -4.043 103.385  1.00 35.36           O
ATOM   1347  N   SER A 179     -34.265  -2.425 110.764  1.00 31.86           N
```

```
ATOM   1348  CA   SER A 179     -33.513  -2.503 112.007  1.00 29.05           C
ATOM   1349  C    SER A 179     -33.405  -3.960 112.453  1.00 30.76           C
ATOM   1350  O    SER A 179     -34.418  -4.660 112.534  1.00 31.95           O
ATOM   1351  CB   SER A 179     -34.185  -1.669 113.086  1.00 29.95           C
ATOM   1352  OG   SER A 179     -33.208  -1.108 113.936  1.00 40.38           O
ATOM   1353  N    LEU A 180     -32.192  -4.427 112.737  1.00 28.38           N
ATOM   1354  CA   LEU A 180     -32.044  -5.768 113.282  1.00 31.63           C
ATOM   1355  C    LEU A 180     -31.118  -5.768 114.492  1.00 29.92           C
ATOM   1356  O    LEU A 180     -30.388  -4.810 114.763  1.00 25.79           O
ATOM   1357  CB   LEU A 180     -31.555  -6.783 112.224  1.00 30.26           C
ATOM   1358  CG   LEU A 180     -30.143  -7.049 111.701  1.00 31.57           C
ATOM   1359  CD1  LEU A 180     -29.103  -7.464 112.770  1.00 28.74           C
ATOM   1360  CD2  LEU A 180     -30.291  -8.158 110.658  1.00 28.38           C
ATOM   1361  N    SER A 181     -31.159  -6.872 115.228  1.00 25.89           N
ATOM   1362  CA   SER A 181     -30.261  -7.060 116.348  1.00 29.69           C
ATOM   1363  C    SER A 181     -29.651  -8.454 116.283  1.00 34.25           C
ATOM   1364  O    SER A 181     -30.228  -9.383 115.710  1.00 28.49           O
ATOM   1365  CB   SER A 181     -30.972  -6.840 117.680  1.00 32.18           C
ATOM   1366  OG   SER A 181     -31.900  -7.868 117.927  1.00 34.51           O
ATOM   1367  N    SER A 182     -28.451  -8.572 116.852  1.00 34.33           N
ATOM   1368  CA   SER A 182     -27.720  -9.826 116.939  1.00 31.79           C
ATOM   1369  C    SER A 182     -27.166  -9.956 118.351  1.00 29.53           C
ATOM   1370  O    SER A 182     -26.634  -8.992 118.899  1.00 29.22           O
ATOM   1371  CB   SER A 182     -26.589  -9.875 115.889  1.00 28.22           C
ATOM   1372  OG   SER A 182     -25.793 -11.040 116.033  1.00 32.19           O
ATOM   1373  N    VAL A 183     -27.302 -11.140 118.946  1.00 31.33           N
ATOM   1374  CA   VAL A 183     -26.891 -11.353 120.329  1.00 32.43           C
ATOM   1375  C    VAL A 183     -26.090 -12.648 120.461  1.00 34.27           C
ATOM   1376  O    VAL A 183     -26.118 -13.530 119.599  1.00 34.59           O
ATOM   1377  CB   VAL A 183     -28.100 -11.386 121.290  1.00 34.07           C
ATOM   1378  CG1  VAL A 183     -28.915 -10.138 121.139  1.00 30.31           C
ATOM   1379  CG2  VAL A 183     -28.962 -12.619 121.027  1.00 34.20           C
ATOM   1380  N    VAL A 184     -25.370 -12.750 121.567  1.00 30.31           N
ATOM   1381  CA   VAL A 184     -24.615 -13.950 121.895  1.00 34.88           C
ATOM   1382  C    VAL A 184     -24.629 -14.101 123.409  1.00 36.21           C
ATOM   1383  O    VAL A 184     -24.549 -13.112 124.145  1.00 36.42           O
ATOM   1384  CB   VAL A 184     -23.172 -13.893 121.322  1.00 36.52           C
ATOM   1385  CG1  VAL A 184     -22.405 -12.622 121.770  1.00 28.53           C
ATOM   1386  CG2  VAL A 184     -22.413 -15.146 121.668  1.00 28.44           C
ATOM   1387  N    THR A 185     -24.800 -15.335 123.876  1.00 37.42           N
ATOM   1388  CA   THR A 185     -24.681 -15.649 125.290  1.00 33.86           C
ATOM   1389  C    THR A 185     -23.296 -16.235 125.522  1.00 36.91           C
ATOM   1390  O    THR A 185     -22.829 -17.074 124.744  1.00 36.01           O
ATOM   1391  CB   THR A 185     -25.772 -16.614 125.758  1.00 36.45           C
ATOM   1392  OG1  THR A 185     -25.864 -17.719 124.850  1.00 42.60           O
ATOM   1393  CG2  THR A 185     -27.110 -15.912 125.804  1.00 34.96           C
ATOM   1394  N    VAL A 186     -22.632 -15.755 126.567  1.00 40.17           N
ATOM   1395  CA   VAL A 186     -21.268 -16.144 126.917  1.00 38.85           C
ATOM   1396  C    VAL A 186     -21.196 -16.288 128.432  1.00 47.21           C
ATOM   1397  O    VAL A 186     -22.080 -15.806 129.155  1.00 45.09           O
ATOM   1398  CB   VAL A 186     -20.244 -15.102 126.425  1.00 36.70           C
ATOM   1399  CG1  VAL A 186     -20.351 -14.919 124.902  1.00 33.73           C
ATOM   1400  CG2  VAL A 186     -20.485 -13.768 127.107  1.00 35.85           C
ATOM   1401  N    PRO A 187     -20.161 -16.964 128.944  1.00 47.74           N
ATOM   1402  CA   PRO A 187     -20.031 -17.090 130.404  1.00 46.98           C
ATOM   1403  C    PRO A 187     -19.776 -15.738 131.058  1.00 48.85           C
ATOM   1404  O    PRO A 187     -19.016 -14.922 130.538  1.00 46.36           O
ATOM   1405  CB   PRO A 187     -18.830 -18.026 130.571  1.00 43.33           C
ATOM   1406  CG   PRO A 187     -18.751 -18.759 129.303  1.00 39.13           C
ATOM   1407  CD   PRO A 187     -19.170 -17.794 128.244  1.00 43.58           C
ATOM   1408  N    SER A 188     -20.423 -15.509 132.208  1.00 50.33           N
ATOM   1409  CA   SER A 188     -20.194 -14.283 132.972  1.00 47.34           C
ATOM   1410  C    SER A 188     -18.715 -14.066 133.275  1.00 55.46           C
ATOM   1411  O    SER A 188     -18.208 -12.942 133.158  1.00 49.43           O
ATOM   1412  CB   SER A 188     -21.001 -14.310 134.262  1.00 52.91           C
ATOM   1413  OG   SER A 188     -22.363 -14.112 133.973  1.00 59.47           O
ATOM   1414  N    SER A 189     -18.010 -15.127 133.685  1.00 55.39           N
ATOM   1415  CA   SER A 189     -16.590 -15.005 134.003  1.00 55.76           C
ATOM   1416  C    SER A 189     -15.769 -14.513 132.811  1.00 58.34           C
ATOM   1417  O    SER A 189     -14.681 -13.951 133.002  1.00 59.39           O
ATOM   1418  CB   SER A 189     -16.042 -16.351 134.478  1.00 56.17           C
```

```
ATOM   1419  OG  SER A 189     -16.101 -17.316 133.435  1.00 52.74           O
ATOM   1420  N   SER A 190     -16.276 -14.687 131.590  1.00 53.51           N
ATOM   1421  CA  SER A 190     -15.528 -14.312 130.396  1.00 56.22           C
ATOM   1422  C   SER A 190     -15.594 -12.816 130.086  1.00 54.08           C
ATOM   1423  O   SER A 190     -14.802 -12.330 129.265  1.00 51.28           O
ATOM   1424  CB  SER A 190     -16.020 -15.138 129.197  1.00 51.36           C
ATOM   1425  OG  SER A 190     -17.368 -14.852 128.863  1.00 46.74           O
ATOM   1426  N   LEU A 191     -16.523 -12.081 130.693  1.00 53.01           N
ATOM   1427  CA  LEU A 191     -16.577 -10.644 130.464  1.00 53.09           C
ATOM   1428  C   LEU A 191     -15.339  -9.981 131.058  1.00 54.27           C
ATOM   1429  O   LEU A 191     -14.790 -10.428 132.071  1.00 58.30           O
ATOM   1430  CB  LEU A 191     -17.839 -10.054 131.081  1.00 38.62           C
ATOM   1431  CG  LEU A 191     -19.093 -10.795 130.646  1.00 45.96           C
ATOM   1432  CD1 LEU A 191     -20.297 -10.313 131.431  1.00 42.60           C
ATOM   1433  CD2 LEU A 191     -19.308 -10.675 129.131  1.00 40.49           C
ATOM   1434  N   GLY A 192     -14.896  -8.905 130.421  1.00 50.04           N
ATOM   1435  CA  GLY A 192     -13.722  -8.196 130.886  1.00 59.39           C
ATOM   1436  C   GLY A 192     -12.384  -8.841 130.570  1.00 59.35           C
ATOM   1437  O   GLY A 192     -11.382  -8.120 130.468  1.00 65.09           O
ATOM   1438  N   THR A 193     -12.332 -10.163 130.376  1.00 50.42           N
ATOM   1439  CA  THR A 193     -11.152 -10.830 129.835  1.00 51.34           C
ATOM   1440  C   THR A 193     -11.269 -11.178 128.350  1.00 53.60           C
ATOM   1441  O   THR A 193     -10.257 -11.147 127.651  1.00 57.09           O
ATOM   1442  CB  THR A 193     -10.822 -12.109 130.622  1.00 56.32           C
ATOM   1443  OG1 THR A 193     -11.943 -12.512 131.418  1.00 57.77           O
ATOM   1444  CG2 THR A 193      -9.595 -11.892 131.523  1.00 48.53           C
ATOM   1445  N   GLN A 194     -12.451 -11.519 127.838  1.00 53.81           N
ATOM   1446  CA  GLN A 194     -12.619 -11.879 126.431  1.00 47.52           C
ATOM   1447  C   GLN A 194     -13.238 -10.716 125.660  1.00 45.35           C
ATOM   1448  O   GLN A 194     -14.169 -10.066 126.142  1.00 47.02           O
ATOM   1449  CB  GLN A 194     -13.512 -13.111 126.292  1.00 46.52           C
ATOM   1450  CG  GLN A 194     -13.761 -13.550 124.857  1.00 46.74           C
ATOM   1451  CD  GLN A 194     -12.496 -13.985 124.141  1.00 53.73           C
ATOM   1452  OE1 GLN A 194     -12.011 -13.301 123.239  1.00 52.18           O
ATOM   1453  NE2 GLN A 194     -11.967 -15.148 124.528  1.00 50.74           N
ATOM   1454  N   THR A 195     -12.745 -10.462 124.453  1.00 44.88           N
ATOM   1455  CA  THR A 195     -13.244  -9.345 123.657  1.00 46.31           C
ATOM   1456  C   THR A 195     -14.325  -9.802 122.680  1.00 43.67           C
ATOM   1457  O   THR A 195     -14.211 -10.862 122.057  1.00 41.58           O
ATOM   1458  CB  THR A 195     -12.104  -8.651 122.909  1.00 53.06           C
ATOM   1459  OG1 THR A 195     -11.441  -7.742 123.803  1.00 57.28           O
ATOM   1460  CG2 THR A 195     -12.631  -7.884 121.694  1.00 42.53           C
ATOM   1461  N   TYR A 196     -15.393  -9.007 122.579  1.00 43.78           N
ATOM   1462  CA  TYR A 196     -16.551  -9.321 121.750  1.00 36.48           C
ATOM   1463  C   TYR A 196     -16.798  -8.201 120.755  1.00 34.63           C
ATOM   1464  O   TYR A 196     -17.090  -7.066 121.139  1.00 34.38           O
ATOM   1465  CB  TYR A 196     -17.769  -9.564 122.621  1.00 32.34           C
ATOM   1466  CG  TYR A 196     -17.567 -10.773 123.470  1.00 38.36           C
ATOM   1467  CD1 TYR A 196     -17.517 -12.036 122.900  1.00 36.90           C
ATOM   1468  CD2 TYR A 196     -17.359 -10.655 124.830  1.00 39.09           C
ATOM   1469  CE1 TYR A 196     -17.316 -13.151 123.676  1.00 37.95           C
ATOM   1470  CE2 TYR A 196     -17.147 -11.755 125.605  1.00 39.61           C
ATOM   1471  CZ  TYR A 196     -17.129 -13.001 125.028  1.00 39.92           C
ATOM   1472  OH  TYR A 196     -16.921 -14.098 125.825  1.00 47.25           O
ATOM   1473  N   ILE A 197     -16.653  -8.516 119.478  1.00 35.82           N
ATOM   1474  CA  ILE A 197     -16.831  -7.548 118.405  1.00 36.37           C
ATOM   1475  C   ILE A 197     -17.841  -8.108 117.422  1.00 30.22           C
ATOM   1476  O   ILE A 197     -17.783  -9.287 117.065  1.00 32.67           O
ATOM   1477  CB  ILE A 197     -15.496  -7.222 117.702  1.00 32.39           C
ATOM   1478  CG1 ILE A 197     -14.528  -6.609 118.709  1.00 32.03           C
ATOM   1479  CG2 ILE A 197     -15.722  -6.302 116.521  1.00 28.21           C
ATOM   1480  CD1 ILE A 197     -13.138  -6.617 118.246  1.00 30.83           C
ATOM   1481  N   CYS A 198     -18.784  -7.287 117.016  1.00 34.51           N
ATOM   1482  CA  CYS A 198     -19.717  -7.687 115.984  1.00 35.67           C
ATOM   1483  C   CYS A 198     -19.346  -7.002 114.676  1.00 33.53           C
ATOM   1484  O   CYS A 198     -19.039  -5.805 114.645  1.00 32.08           O
ATOM   1485  CB  CYS A 198     -21.157  -7.379 116.404  1.00 36.62           C
ATOM   1486  SG  CYS A 198     -21.662  -5.699 116.191  1.00 46.42           S
ATOM   1487  N   ASN A 199     -19.373  -7.777 113.598  1.00 31.00           N
ATOM   1488  CA  ASN A 199     -18.952  -7.325 112.279  1.00 34.62           C
ATOM   1489  C   ASN A 199     -20.212  -7.179 111.438  1.00 30.03           C
```

```
ATOM   1490  O    ASN A 199     -20.926  -8.157 111.205  1.00 33.61           O
ATOM   1491  CB   ASN A 199     -17.969  -8.322 111.669  1.00 30.80           C
ATOM   1492  CG   ASN A 199     -16.998  -8.861 112.691  1.00 33.45           C
ATOM   1493  OD1  ASN A 199     -17.071 -10.028 113.087  1.00 30.25           O
ATOM   1494  ND2  ASN A 199     -16.090  -8.004 113.146  1.00 31.39           N
ATOM   1495  N    VAL A 200     -20.530  -5.961 111.036  1.00 25.18           N
ATOM   1496  CA   VAL A 200     -21.753  -5.699 110.295  1.00 31.02           C
ATOM   1497  C    VAL A 200     -21.346  -5.394 108.868  1.00 31.80           C
ATOM   1498  O    VAL A 200     -20.402  -4.632 108.642  1.00 32.16           O
ATOM   1499  CB   VAL A 200     -22.581  -4.546 110.896  1.00 26.09           C
ATOM   1500  CG1  VAL A 200     -23.862  -4.409 110.130  1.00 31.91           C
ATOM   1501  CG2  VAL A 200     -22.900  -4.792 112.348  1.00 27.12           C
ATOM   1502  N    ASN A 201     -22.022  -6.016 107.910  1.00 31.12           N
ATOM   1503  CA   ASN A 201     -21.800  -5.693 106.511  1.00 34.13           C
ATOM   1504  C    ASN A 201     -23.127  -5.341 105.866  1.00 32.42           C
ATOM   1505  O    ASN A 201     -24.065  -6.141 105.883  1.00 35.02           O
ATOM   1506  CB   ASN A 201     -21.122  -6.839 105.767  1.00 36.09           C
ATOM   1507  CG   ASN A 201     -20.557  -6.390 104.457  1.00 39.19           C
ATOM   1508  OD1  ASN A 201     -20.860  -5.296 104.000  1.00 43.01           O
ATOM   1509  ND2  ASN A 201     -19.730  -7.218 103.844  1.00 44.41           N
ATOM   1510  N    HIS A 202     -23.222  -4.123 105.361  1.00 34.15           N
ATOM   1511  CA   HIS A 202     -24.349  -3.682 104.561  1.00 33.64           C
ATOM   1512  C    HIS A 202     -23.801  -3.403 103.168  1.00 39.81           C
ATOM   1513  O    HIS A 202     -23.463  -2.264 102.836  1.00 37.99           O
ATOM   1514  CB   HIS A 202     -25.001  -2.473 105.147  1.00 31.71           C
ATOM   1515  CG   HIS A 202     -26.244  -2.052 104.425  1.00 37.75           C
ATOM   1516  ND1  HIS A 202     -26.419  -0.780 103.917  1.00 38.12           N
ATOM   1517  CD2  HIS A 202     -27.377  -2.735 104.129  1.00 32.94           C
ATOM   1518  CE1  HIS A 202     -27.607  -0.699 103.344  1.00 37.74           C
ATOM   1519  NE2  HIS A 202     -28.210  -1.871 103.463  1.00 34.91           N
ATOM   1520  N    LYS A 203     -23.690  -4.461 102.359  1.00 34.79           N
ATOM   1521  CA   LYS A 203     -23.249  -4.285 100.978  1.00 35.75           C
ATOM   1522  C    LYS A 203     -24.071  -3.261 100.195  1.00 39.45           C
ATOM   1523  O    LYS A 203     -23.482  -2.573  99.346  1.00 38.28           O
ATOM   1524  CB   LYS A 203     -23.166  -5.647 100.266  1.00 29.77           C
ATOM   1525  CG   LYS A 203     -21.879  -6.425 100.649  1.00 40.20           C
ATOM   1526  CD   LYS A 203     -20.652  -5.726  99.988  1.00 63.25           C
ATOM   1527  CE   LYS A 203     -19.279  -6.000 100.660  1.00 68.58           C
ATOM   1528  NZ   LYS A 203     -18.938  -7.439 100.898  1.00 69.85           N1+
ATOM   1529  N    PRO A 204     -25.392  -3.108 100.390  1.00 41.00           N
ATOM   1530  CA   PRO A 204     -26.120  -2.121  99.566  1.00 38.96           C
ATOM   1531  C    PRO A 204     -25.629  -0.681  99.705  1.00 40.45           C
ATOM   1532  O    PRO A 204     -25.839   0.114  98.782  1.00 44.07           O
ATOM   1533  CB   PRO A 204     -27.568  -2.272 100.043  1.00 36.97           C
ATOM   1534  CG   PRO A 204     -27.647  -3.652 100.563  1.00 35.26           C
ATOM   1535  CD   PRO A 204     -26.327  -3.952 101.168  1.00 33.30           C
ATOM   1536  N    SER A 205     -25.061  -0.287 100.845  1.00 42.01           N
ATOM   1537  CA   SER A 205     -24.455   1.034 100.993  1.00 40.43           C
ATOM   1538  C    SER A 205     -22.931   0.956 101.094  1.00 39.05           C
ATOM   1539  O    SER A 205     -22.279   1.963 101.369  1.00 37.64           O
ATOM   1540  CB   SER A 205     -25.052   1.778 102.195  1.00 35.25           C
ATOM   1541  OG   SER A 205     -24.659   1.213 103.424  1.00 36.65           O
ATOM   1542  N    ASN A 206     -22.354  -0.219 100.868  1.00 42.36           N
ATOM   1543  CA   ASN A 206     -20.919  -0.449 101.017  1.00 44.31           C
ATOM   1544  C    ASN A 206     -20.414   0.021 102.382  1.00 46.67           C
ATOM   1545  O    ASN A 206     -19.385   0.680 102.506  1.00 50.37           O
ATOM   1546  CB   ASN A 206     -20.146   0.208  99.880  1.00 49.52           C
ATOM   1547  CG   ASN A 206     -19.229  -0.766  99.187  1.00 61.70           C
ATOM   1548  OD1  ASN A 206     -19.323  -1.979  99.401  1.00 67.47           O
ATOM   1549  ND2  ASN A 206     -18.336  -0.253  98.352  1.00 70.26           N
ATOM   1550  N    THR A 207     -21.150  -0.352 103.423  1.00 45.98           N
ATOM   1551  CA   THR A 207     -20.861   0.041 104.792  1.00 36.87           C
ATOM   1552  C    THR A 207     -20.417  -1.180 105.574  1.00 37.03           C
ATOM   1553  O    THR A 207     -21.118  -2.192 105.590  1.00 41.13           O
ATOM   1554  CB   THR A 207     -22.100   0.626 105.457  1.00 42.00           C
ATOM   1555  OG1  THR A 207     -22.616   1.689 104.649  1.00 45.30           O
ATOM   1556  CG2  THR A 207     -21.767   1.132 106.876  1.00 42.01           C
ATOM   1557  N    LYS A 208     -19.263  -1.088 106.210  1.00 36.32           N
ATOM   1558  CA   LYS A 208     -18.793  -2.110 107.127  1.00 32.74           C
ATOM   1559  C    LYS A 208     -18.511  -1.465 108.478  1.00 34.61           C
ATOM   1560  O    LYS A 208     -18.088  -0.307 108.545  1.00 39.61           O
```

```
ATOM   1561  CB  LYS A 208     -17.552  -2.827 106.585  1.00 40.21           C
ATOM   1562  CG  LYS A 208     -17.865  -3.863 105.502  1.00 39.11           C
ATOM   1563  CD  LYS A 208     -16.707  -4.821 105.239  1.00 34.67           C
ATOM   1564  CE  LYS A 208     -15.518  -4.128 104.606  1.00 49.09           C
ATOM   1565  NZ  LYS A 208     -14.349  -5.076 104.456  1.00 57.65           N1+
ATOM   1566  N   VAL A 209     -18.846  -2.174 109.552  1.00 32.84           N
ATOM   1567  CA  VAL A 209     -18.671  -1.684 110.917  1.00 32.20           C
ATOM   1568  C   VAL A 209     -18.247  -2.844 111.797  1.00 34.40           C
ATOM   1569  O   VAL A 209     -18.906  -3.889 111.817  1.00 38.11           O
ATOM   1570  CB  VAL A 209     -19.960  -1.054 111.491  1.00 31.14           C
ATOM   1571  CG1 VAL A 209     -19.816  -0.826 112.974  1.00 27.96           C
ATOM   1572  CG2 VAL A 209     -20.276   0.252 110.810  1.00 26.07           C
ATOM   1573  N   ASP A 210     -17.195  -2.642 112.574  1.00 36.64           N
ATOM   1574  CA  ASP A 210     -16.821  -3.545 113.659  1.00 33.62           C
ATOM   1575  C   ASP A 210     -17.053  -2.791 114.962  1.00 30.39           C
ATOM   1576  O   ASP A 210     -16.490  -1.718 115.159  1.00 32.48           O
ATOM   1577  CB  ASP A 210     -15.360  -3.980 113.548  1.00 32.01           C
ATOM   1578  CG  ASP A 210     -15.084  -4.843 112.325  1.00 42.55           C
ATOM   1579  OD1 ASP A 210     -15.829  -5.839 112.124  1.00 46.60           O
ATOM   1580  OD2 ASP A 210     -14.119  -4.527 111.570  1.00 34.51           O1-
ATOM   1581  N   LYS A 211     -17.904  -3.319 115.827  1.00 34.41           N
ATOM   1582  CA  LYS A 211     -18.270  -2.654 117.073  1.00 28.87           C
ATOM   1583  C   LYS A 211     -17.832  -3.519 118.243  1.00 33.13           C
ATOM   1584  O   LYS A 211     -18.283  -4.661 118.374  1.00 31.45           O
ATOM   1585  CB  LYS A 211     -19.775  -2.409 117.155  1.00 30.56           C
ATOM   1586  CG  LYS A 211     -20.246  -1.799 118.460  1.00 33.07           C
ATOM   1587  CD  LYS A 211     -19.646  -0.430 118.618  1.00 36.20           C
ATOM   1588  CE  LYS A 211     -20.539   0.467 119.407  1.00 33.18           C
ATOM   1589  NZ  LYS A 211     -20.169   1.877 119.156  1.00 35.41           N1+
ATOM   1590  N   LYS A 212     -16.962  -2.972 119.094  1.00 35.68           N
ATOM   1591  CA  LYS A 212     -16.654  -3.617 120.354  1.00 28.26           C
ATOM   1592  C   LYS A 212     -17.806  -3.349 121.296  1.00 30.86           C
ATOM   1593  O   LYS A 212     -18.409  -2.271 121.278  1.00 33.51           O
ATOM   1594  CB  LYS A 212     -15.341  -3.112 120.955  1.00 31.23           C
ATOM   1595  CG  LYS A 212     -14.740  -4.080 121.984  1.00 38.53           C
ATOM   1596  CD  LYS A 212     -13.567  -3.503 122.778  1.00 42.63           C
ATOM   1597  CE  LYS A 212     -13.088  -4.492 123.847  1.00 44.88           C
ATOM   1598  NZ  LYS A 212     -12.001  -3.960 124.723  1.00 52.61           N1+
ATOM   1599  N   VAL A 213     -18.175  -4.376 122.039  1.00 30.30           N
ATOM   1600  CA  VAL A 213     -19.246  -4.322 123.012  1.00 34.60           C
ATOM   1601  C   VAL A 213     -18.589  -4.663 124.336  1.00 35.78           C
ATOM   1602  O   VAL A 213     -18.141  -5.795 124.530  1.00 39.34           O
ATOM   1603  CB  VAL A 213     -20.380  -5.300 122.667  1.00 36.86           C
ATOM   1604  CG1 VAL A 213     -21.521  -5.226 123.700  1.00 37.67           C
ATOM   1605  CG2 VAL A 213     -20.903  -5.028 121.278  1.00 27.28           C
ATOM   1606  N   GLU A 214     -18.489  -3.687 125.228  1.00 43.48           N
ATOM   1607  CA  GLU A 214     -17.839  -3.878 126.521  1.00 42.79           C
ATOM   1608  C   GLU A 214     -18.866  -3.868 127.636  1.00 39.79           C
ATOM   1609  O   GLU A 214     -19.925  -3.250 127.508  1.00 39.59           O
ATOM   1610  CB  GLU A 214     -16.795  -2.787 126.788  1.00 42.03           C
ATOM   1611  CG  GLU A 214     -15.715  -2.770 125.730  1.00 53.61           C
ATOM   1612  CD  GLU A 214     -14.732  -1.636 125.888  1.00 60.49           C
ATOM   1613  OE1 GLU A 214     -13.614  -1.878 126.391  1.00 64.12           O
ATOM   1614  OE2 GLU A 214     -15.085  -0.500 125.505  1.00 65.45           O1-
ATOM   1615  N   PRO A 215     -18.599  -4.573 128.724  1.00 42.19           N
ATOM   1616  CA  PRO A 215     -19.495  -4.496 129.882  1.00 47.10           C
ATOM   1617  C   PRO A 215     -19.546  -3.089 130.452  1.00 54.06           C
ATOM   1618  O   PRO A 215     -18.521  -2.414 130.566  1.00 56.58           O
ATOM   1619  CB  PRO A 215     -18.885  -5.492 130.874  1.00 49.68           C
ATOM   1620  CG  PRO A 215     -17.567  -5.950 130.264  1.00 48.26           C
ATOM   1621  CD  PRO A 215     -17.651  -5.695 128.809  1.00 45.54           C
ATOM   1622  N   LYS A 216     -20.760  -2.636 130.768  1.00 59.07           N
ATOM   1623  CA  LYS A 216     -21.008  -1.289 131.272  1.00 66.58           C
ATOM   1624  C   LYS A 216     -21.516  -1.355 132.710  1.00 80.80           C
ATOM   1625  O   LYS A 216     -22.468  -2.092 133.005  1.00 75.99           O
ATOM   1626  CB  LYS A 216     -22.036  -0.555 130.408  1.00 66.80           C
ATOM   1627  CG  LYS A 216     -22.360   0.859 130.899  1.00 73.54           C
ATOM   1628  CD  LYS A 216     -23.096   1.653 129.834  1.00 77.62           C
ATOM   1629  CE  LYS A 216     -23.325   3.092 130.255  1.00 75.50           C
ATOM   1630  NZ  LYS A 216     -23.769   3.921 129.103  1.00 65.88           N1+
ATOM   1631  N   SER A 217     -20.927  -0.528 133.580  1.00 90.64           N
```

```
ATOM   1632  CA  SER A 217     -21.323  -0.451 134.994  1.00 92.77           C
ATOM   1633  C   SER A 217     -22.109   0.829 135.282  1.00 85.80           C
ATOM   1634  O   SER A 217     -23.332   0.797 135.455  1.00 82.11           O
ATOM   1635  CB  SER A 217     -20.096  -0.528 135.915  1.00 84.71           C
ATOM   1636  OG  SER A 217     -19.239   0.585 135.722  1.00 86.75           O
TER
ATOM   1637  N   GLU B   1     -59.401 -19.548  88.941  1.00 32.61           N
ATOM   1638  CA  GLU B   1     -58.164 -20.286  89.135  1.00 32.73           C
ATOM   1639  C   GLU B   1     -58.344 -21.415  90.159  1.00 39.88           C
ATOM   1640  O   GLU B   1     -59.196 -21.331  91.040  1.00 39.29           O
ATOM   1641  CB  GLU B   1     -57.054 -19.366  89.613  1.00 32.87           C
ATOM   1642  CG  GLU B   1     -57.037 -19.224  91.116  1.00 33.91           C
ATOM   1643  CD  GLU B   1     -56.083 -18.160  91.599  1.00 44.88           C
ATOM   1644  OE1 GLU B   1     -56.022 -17.970  92.832  1.00 55.17           O
ATOM   1645  OE2 GLU B   1     -55.378 -17.536  90.763  1.00 44.77           O1-
ATOM   1646  N   ILE B   2     -57.536 -22.470  90.052  1.00 36.20           N
ATOM   1647  CA  ILE B   2     -57.589 -23.542  91.037  1.00 31.00           C
ATOM   1648  C   ILE B   2     -56.950 -23.074  92.339  1.00 27.84           C
ATOM   1649  O   ILE B   2     -55.774 -22.694  92.370  1.00 27.28           O
ATOM   1650  CB  ILE B   2     -56.893 -24.797  90.521  1.00 30.83           C
ATOM   1651  CG1 ILE B   2     -57.571 -25.303  89.259  1.00 24.03           C
ATOM   1652  CG2 ILE B   2     -56.832 -25.856  91.649  1.00 24.77           C
ATOM   1653  CD1 ILE B   2     -56.866 -26.508  88.716  1.00 24.00           C
ATOM   1654  N   VAL B   3     -57.718 -23.116  93.424  1.00 28.01           N
ATOM   1655  CA  VAL B   3     -57.227 -22.750  94.748  1.00 31.60           C
ATOM   1656  C   VAL B   3     -56.810 -24.011  95.498  1.00 30.30           C
ATOM   1657  O   VAL B   3     -57.580 -24.976  95.602  1.00 30.30           O
ATOM   1658  CB  VAL B   3     -58.287 -21.965  95.535  1.00 30.01           C
ATOM   1659  CG1 VAL B   3     -57.782 -21.724  96.916  1.00 23.45           C
ATOM   1660  CG2 VAL B   3     -58.637 -20.645  94.815  1.00 24.87           C
ATOM   1661  N   LEU B   4     -55.591 -24.017  96.006  1.00 27.66           N
ATOM   1662  CA  LEU B   4     -55.047 -25.168  96.709  1.00 27.72           C
ATOM   1663  C   LEU B   4     -54.941 -24.793  98.170  1.00 30.07           C
ATOM   1664  O   LEU B   4     -54.281 -23.805  98.509  1.00 34.25           O
ATOM   1665  CB  LEU B   4     -53.677 -25.570  96.165  1.00 31.34           C
ATOM   1666  CG  LEU B   4     -53.596 -26.025  94.711  1.00 30.78           C
ATOM   1667  CD1 LEU B   4     -52.155 -26.475  94.361  1.00 29.60           C
ATOM   1668  CD2 LEU B   4     -54.571 -27.161  94.511  1.00 23.79           C
ATOM   1669  N   THR B   5     -55.591 -25.568  99.026  1.00 26.39           N
ATOM   1670  CA  THR B   5     -55.586 -25.330 100.458  1.00 23.07           C
ATOM   1671  C   THR B   5     -54.765 -26.407 101.130  1.00 26.07           C
ATOM   1672  O   THR B   5     -55.089 -27.591 101.034  1.00 29.54           O
ATOM   1673  CB  THR B   5     -57.007 -25.330 101.019  1.00 23.58           C
ATOM   1674  OG1 THR B   5     -57.813 -24.424 100.256  1.00 25.83           O
ATOM   1675  CG2 THR B   5     -56.992 -24.892 102.458  1.00 18.31           C
ATOM   1676  N   GLN B   6     -53.731 -26.003 101.830  1.00 23.61           N
ATOM   1677  CA  GLN B   6     -52.920 -26.943 102.569  1.00 25.57           C
ATOM   1678  C   GLN B   6     -53.328 -26.929 104.024  1.00 24.28           C
ATOM   1679  O   GLN B   6     -53.585 -25.871 104.592  1.00 28.38           O
ATOM   1680  CB  GLN B   6     -51.432 -26.623 102.442  1.00 21.43           C
ATOM   1681  CG  GLN B   6     -50.866 -27.125 101.172  1.00 25.09           C
ATOM   1682  CD  GLN B   6     -49.406 -26.785 101.003  1.00 29.92           C
ATOM   1683  OE1 GLN B   6     -49.065 -25.856 100.258  1.00 30.10           O
ATOM   1684  NE2 GLN B   6     -48.529 -27.542 101.671  1.00 23.00           N
ATOM   1685  N   SER B   7     -53.388 -28.113 104.609  1.00 28.46           N
ATOM   1686  CA  SER B   7     -53.526 -28.273 106.046  1.00 32.14           C
ATOM   1687  C   SER B   7     -52.626 -29.410 106.541  1.00 32.52           C
ATOM   1688  O   SER B   7     -52.238 -30.294 105.774  1.00 31.45           O
ATOM   1689  CB  SER B   7     -54.960 -28.558 106.404  1.00 26.70           C
ATOM   1690  OG  SER B   7     -55.208 -29.883 106.045  1.00 39.01           O
ATOM   1691  N   PRO B   8     -52.242 -29.367 107.816  1.00 35.79           N
ATOM   1692  CA  PRO B   8     -52.444 -28.237 108.728  1.00 32.17           C
ATOM   1693  C   PRO B   8     -51.456 -27.142 108.349  1.00 33.18           C
ATOM   1694  O   PRO B   8     -50.550 -27.465 107.590  1.00 35.18           O
ATOM   1695  CB  PRO B   8     -52.119 -28.837 110.103  1.00 26.39           C
ATOM   1696  CG  PRO B   8     -51.061 -29.854 109.786  1.00 33.66           C
ATOM   1697  CD  PRO B   8     -51.426 -30.438 108.422  1.00 30.11           C
ATOM   1698  N   GLY B   9     -51.584 -25.920 108.877  1.00 35.12           N
ATOM   1699  CA  GLY B   9     -50.583 -24.899 108.603  1.00 25.91           C
ATOM   1700  C   GLY B   9     -49.249 -25.202 109.264  1.00 27.68           C
ATOM   1701  O   GLY B   9     -48.196 -25.015 108.661  1.00 27.97           O
```

```
ATOM   1702  N    THR B  10    -49.274 -25.726 110.487  1.00 26.27           N
ATOM   1703  CA   THR B  10    -48.056 -26.118 111.179  1.00 27.02           C
ATOM   1704  C    THR B  10    -48.197 -27.524 111.746  1.00 26.07           C
ATOM   1705  O    THR B  10    -49.251 -27.890 112.264  1.00 30.58           O
ATOM   1706  CB   THR B  10    -47.725 -25.143 112.310  1.00 26.29           C
ATOM   1707  OG1  THR B  10    -47.699 -23.812 111.789  1.00 30.87           O
ATOM   1708  CG2  THR B  10    -46.368 -25.480 112.929  1.00 18.58           C
ATOM   1709  N    LEU B  11    -47.117 -28.296 111.669  1.00 28.05           N
ATOM   1710  CA   LEU B  11    -47.057 -29.670 112.156  1.00 27.74           C
ATOM   1711  C    LEU B  11    -45.839 -29.819 113.059  1.00 29.19           C
ATOM   1712  O    LEU B  11    -44.706 -29.647 112.600  1.00 31.16           O
ATOM   1713  CB   LEU B  11    -46.954 -30.632 110.978  1.00 33.49           C
ATOM   1714  CG   LEU B  11    -47.842 -31.847 110.756  1.00 40.86           C
ATOM   1715  CD1  LEU B  11    -47.188 -32.642 109.625  1.00 33.69           C
ATOM   1716  CD2  LEU B  11    -47.976 -32.687 112.003  1.00 34.11           C
ATOM   1717  N    SER B  12    -46.055 -30.135 114.336  1.00 32.77           N
ATOM   1718  CA   SER B  12    -44.956 -30.381 115.271  1.00 30.27           C
ATOM   1719  C    SER B  12    -44.851 -31.873 115.562  1.00 30.86           C
ATOM   1720  O    SER B  12    -45.793 -32.475 116.083  1.00 35.59           O
ATOM   1721  CB   SER B  12    -45.145 -29.592 116.560  1.00 27.94           C
ATOM   1722  OG   SER B  12    -45.372 -28.227 116.236  1.00 38.87           O
ATOM   1723  N    LEU B  13    -43.694 -32.453 115.245  1.00 31.96           N
ATOM   1724  CA   LEU B  13    -43.453 -33.887 115.299  1.00 32.10           C
ATOM   1725  C    LEU B  13    -42.010 -34.098 115.707  1.00 30.66           C
ATOM   1726  O    LEU B  13    -41.159 -33.231 115.489  1.00 31.40           O
ATOM   1727  CB   LEU B  13    -43.690 -34.572 113.949  1.00 32.81           C
ATOM   1728  CG   LEU B  13    -45.087 -34.460 113.332  1.00 34.52           C
ATOM   1729  CD1  LEU B  13    -45.085 -35.018 111.924  1.00 32.83           C
ATOM   1730  CD2  LEU B  13    -46.110 -35.164 114.189  1.00 26.42           C
ATOM   1731  N    SER B  14    -41.745 -35.232 116.316  1.00 28.88           N
ATOM   1732  CA   SER B  14    -40.359 -35.534 116.639  1.00 32.75           C
ATOM   1733  C    SER B  14    -39.649 -36.161 115.442  1.00 28.56           C
ATOM   1734  O    SER B  14    -40.281 -36.766 114.580  1.00 31.22           O
ATOM   1735  CB   SER B  14    -40.292 -36.469 117.838  1.00 36.49           C
ATOM   1736  OG   SER B  14    -40.626 -35.749 119.019  1.00 44.98           O
ATOM   1737  N    PRO B  15    -38.341 -36.015 115.345  1.00 29.37           N
ATOM   1738  CA   PRO B  15    -37.609 -36.799 114.341  1.00 28.36           C
ATOM   1739  C    PRO B  15    -37.846 -38.290 114.566  1.00 33.54           C
ATOM   1740  O    PRO B  15    -37.877 -38.770 115.702  1.00 39.74           O
ATOM   1741  CB   PRO B  15    -36.149 -36.404 114.575  1.00 31.85           C
ATOM   1742  CG   PRO B  15    -36.230 -35.042 115.265  1.00 27.05           C
ATOM   1743  CD   PRO B  15    -37.481 -35.086 116.097  1.00 29.92           C
ATOM   1744  N    GLY B  16    -37.994 -39.032 113.467  1.00 34.12           N
ATOM   1745  CA   GLY B  16    -38.362 -40.428 113.500  1.00 30.27           C
ATOM   1746  C    GLY B  16    -39.837 -40.693 113.284  1.00 32.66           C
ATOM   1747  O    GLY B  16    -40.204 -41.796 112.862  1.00 38.35           O
ATOM   1748  N    GLU B  17    -40.690 -39.722 113.549  1.00 28.58           N
ATOM   1749  CA   GLU B  17    -42.105 -39.947 113.346  1.00 27.09           C
ATOM   1750  C    GLU B  17    -42.502 -39.762 111.878  1.00 32.66           C
ATOM   1751  O    GLU B  17    -41.728 -39.318 111.016  1.00 27.01           O
ATOM   1752  CB   GLU B  17    -42.923 -39.013 114.220  1.00 26.44           C
ATOM   1753  CG   GLU B  17    -42.896 -39.366 115.689  1.00 35.87           C
ATOM   1754  CD   GLU B  17    -43.872 -38.506 116.494  1.00 50.33           C
ATOM   1755  OE1  GLU B  17    -43.659 -37.269 116.602  1.00 47.95           O
ATOM   1756  OE2  GLU B  17    -44.862 -39.066 117.011  1.00 67.19           O1-
ATOM   1757  N    ARG B  18    -43.757 -40.098 111.626  1.00 28.24           N
ATOM   1758  CA   ARG B  18    -44.377 -40.060 110.321  1.00 29.25           C
ATOM   1759  C    ARG B  18    -45.174 -38.768 110.187  1.00 31.06           C
ATOM   1760  O    ARG B  18    -45.801 -38.322 111.151  1.00 28.90           O
ATOM   1761  CB   ARG B  18    -45.273 -41.278 110.169  1.00 28.93           C
ATOM   1762  CG   ARG B  18    -46.000 -41.420 108.875  1.00 35.84           C
ATOM   1763  CD   ARG B  18    -46.955 -42.591 109.004  1.00 33.74           C
ATOM   1764  NE   ARG B  18    -47.724 -42.806 107.792  1.00 42.81           N
ATOM   1765  CZ   ARG B  18    -47.249 -43.450 106.731  1.00 49.16           C
ATOM   1766  NH1  ARG B  18    -45.993 -43.927 106.747  1.00 44.51           N1+
ATOM   1767  NH2  ARG B  18    -48.021 -43.606 105.656  1.00 37.48           N
ATOM   1768  N    ALA B  19    -45.104 -38.148 109.001  1.00 30.81           N
ATOM   1769  CA   ALA B  19    -45.764 -36.880 108.699  1.00 27.46           C
ATOM   1770  C    ALA B  19    -46.674 -37.020 107.488  1.00 27.67           C
ATOM   1771  O    ALA B  19    -46.284 -37.594 106.466  1.00 29.51           O
ATOM   1772  CB   ALA B  19    -44.745 -35.775 108.431  1.00 26.69           C
```

```
ATOM   1773  N    THR B  20    -47.863 -36.437 107.576  1.00 23.65          N
ATOM   1774  CA   THR B  20    -48.833 -36.478 106.489  1.00 28.01          C
ATOM   1775  C    THR B  20    -49.374 -35.076 106.258  1.00 29.74          C
ATOM   1776  O    THR B  20    -49.974 -34.477 107.155  1.00 31.62          O
ATOM   1777  CB   THR B  20    -49.966 -37.477 106.791  1.00 31.91          C
ATOM   1778  OG1  THR B  20    -49.516 -38.802 106.486  1.00 38.10          O
ATOM   1779  CG2  THR B  20    -51.212 -37.192 105.973  1.00 31.11          C
ATOM   1780  N    LEU B  21    -49.163 -34.561 105.054  1.00 28.91          N
ATOM   1781  CA   LEU B  21    -49.541 -33.209 104.693  1.00 27.86          C
ATOM   1782  C    LEU B  21    -50.689 -33.322 103.711  1.00 27.21          C
ATOM   1783  O    LEU B  21    -50.742 -34.261 102.921  1.00 27.15          O
ATOM   1784  CB   LEU B  21    -48.377 -32.459 104.031  1.00 24.41          C
ATOM   1785  CG   LEU B  21    -46.975 -32.345 104.643  1.00 26.45          C
ATOM   1786  CD1  LEU B  21    -46.405 -30.957 104.460  1.00 27.98          C
ATOM   1787  CD2  LEU B  21    -46.871 -32.762 106.081  1.00 28.98          C
ATOM   1788  N    SER B  22    -51.620 -32.390 103.764  1.00 27.44          N
ATOM   1789  CA   SER B  22    -52.739 -32.439 102.843  1.00 27.56          C
ATOM   1790  C    SER B  22    -52.718 -31.231 101.926  1.00 29.41          C
ATOM   1791  O    SER B  22    -52.281 -30.148 102.321  1.00 23.55          O
ATOM   1792  CB   SER B  22    -54.067 -32.506 103.571  1.00 22.71          C
ATOM   1793  OG   SER B  22    -54.374 -33.869 103.733  1.00 40.86          O
ATOM   1794  N    CYS B  23    -53.188 -31.445 100.693  1.00 24.46          N
ATOM   1795  CA   CYS B  23    -53.456 -30.379  99.735  1.00 26.64          C
ATOM   1796  C    CYS B  23    -54.824 -30.660  99.146  1.00 26.09          C
ATOM   1797  O    CYS B  23    -55.034 -31.718  98.547  1.00 30.05          O
ATOM   1798  CB   CYS B  23    -52.392 -30.330  98.623  1.00 29.42          C
ATOM   1799  SG   CYS B  23    -52.509 -28.931  97.438  1.00 45.36          S
ATOM   1800  N    ARG B  24    -55.756 -29.740  99.326  1.00 26.38          N
ATOM   1801  CA   ARG B  24    -57.084 -29.874  98.762  1.00 25.14          C
ATOM   1802  C    ARG B  24    -57.249 -28.870  97.633  1.00 27.66          C
ATOM   1803  O    ARG B  24    -56.984 -27.677  97.815  1.00 27.74          O
ATOM   1804  CB   ARG B  24    -58.151 -29.702  99.834  1.00 25.05          C
ATOM   1805  CG   ARG B  24    -58.148 -30.888 100.781  1.00 39.63          C
ATOM   1806  CD   ARG B  24    -58.919 -30.636 102.071  1.00 56.87          C
ATOM   1807  NE   ARG B  24    -60.337 -30.971 101.921  1.00 70.81          N
ATOM   1808  CZ   ARG B  24    -60.899 -32.091 102.373  1.00 75.66          C
ATOM   1809  NH1  ARG B  24    -60.156 -32.994 103.017  1.00 74.34          N1+
ATOM   1810  NH2  ARG B  24    -62.204 -32.303 102.187  1.00 69.31          N
ATOM   1811  N    ALA B  25    -57.657 -29.371  96.465  1.00 25.97          N
ATOM   1812  CA   ALA B  25    -57.809 -28.586  95.252  1.00 24.73          C
ATOM   1813  C    ALA B  25    -59.271 -28.236  95.024  1.00 25.92          C
ATOM   1814  O    ALA B  25    -60.147 -29.102  95.093  1.00 27.92          O
ATOM   1815  CB   ALA B  25    -57.263 -29.344  94.042  1.00 25.78          C
ATOM   1816  N    SER B  26    -59.524 -26.969  94.744  1.00 28.48          N
ATOM   1817  CA   SER B  26    -60.840 -26.517  94.342  1.00 26.13          C
ATOM   1818  C    SER B  26    -60.758 -25.481  93.190  1.00 29.42          C
ATOM   1819  O    SER B  26    -60.242 -24.370  93.373  1.00 30.69          O
ATOM   1820  CB   SER B  26    -61.562 -25.932  95.549  1.00 24.33          C
ATOM   1821  OG   SER B  26    -62.823 -25.420  95.178  1.00 39.40          O
ATOM   1822  N    PRO B  27    -61.248 -25.837  91.994  1.00 27.29          N
ATOM   1823  CA   PRO B  27    -61.824 -27.118  91.541  1.00 25.37          C
ATOM   1824  C    PRO B  27    -60.819 -28.258  91.496  1.00 27.72          C
ATOM   1825  O    PRO B  27    -59.639 -28.025  91.702  1.00 28.26          O
ATOM   1826  CB   PRO B  27    -62.331 -26.806  90.123  1.00 22.36          C
ATOM   1827  CG   PRO B  27    -61.831 -25.420  89.788  1.00 21.74          C
ATOM   1828  CD   PRO B  27    -61.584 -24.724  91.085  1.00 22.47          C
ATOM   1829  N    SER B  28    -61.284 -29.468  91.201  1.00 29.06          N
ATOM   1830  CA   SER B  28    -60.424 -30.643  91.235  1.00 29.98          C
ATOM   1831  C    SER B  28    -59.291 -30.567  90.206  1.00 31.92          C
ATOM   1832  O    SER B  28    -59.390 -29.898  89.174  1.00 33.40          O
ATOM   1833  CB   SER B  28    -61.256 -31.892  90.993  1.00 30.66          C
ATOM   1834  OG   SER B  28    -62.051 -32.132  92.124  1.00 36.68          O
ATOM   1835  N    VAL B  29    -58.219 -31.314  90.488  1.00 29.10          N
ATOM   1836  CA   VAL B  29    -57.081 -31.501  89.588  1.00 27.86          C
ATOM   1837  C    VAL B  29    -57.110 -32.960  89.140  1.00 34.14          C
ATOM   1838  O    VAL B  29    -56.339 -33.796  89.630  1.00 31.49          O
ATOM   1839  CB   VAL B  29    -55.739 -31.126  90.270  1.00 26.93          C
ATOM   1840  CG1  VAL B  29    -54.536 -31.343  89.352  1.00 31.84          C
ATOM   1841  CG2  VAL B  29    -55.741 -29.679  90.699  1.00 27.83          C
ATOM   1842  N    ASN B  30    -58.040 -33.287  88.234  1.00 37.48          N
ATOM   1843  CA   ASN B  30    -58.225 -34.670  87.784  1.00 33.93          C
```

| ATOM | 1844 | C   | ASN | B | 30 | -57.128 | -35.163 | 86.860  | 1.00 | 28.81 | C |
| ---- | ---- | --- | --- | - | -- | ------- | ------- | ------- | ---- | ----- | - |
| ATOM | 1845 | O   | ASN | B | 30 | -57.130 | -36.351 | 86.526  | 1.00 | 35.16 | O |
| ATOM | 1846 | CB  | ASN | B | 30 | -59.573 | -34.847 | 87.088  | 1.00 | 30.66 | C |
| ATOM | 1847 | CG  | ASN | B | 30 | -60.750 | -34.648 | 88.034  | 1.00 | 32.10 | C |
| ATOM | 1848 | OD1 | ASN | B | 30 | -60.735 | -35.110 | 89.179  | 1.00 | 36.69 | O |
| ATOM | 1849 | ND2 | ASN | B | 30 | -61.763 | -33.952 | 87.564  | 1.00 | 29.33 | N |
| ATOM | 1850 | N   | SER | B | 31 | -56.223 | -34.293 | 86.406  | 1.00 | 30.18 | N |
| ATOM | 1851 | CA  | SER | B | 31 | -55.082 | -34.759 | 85.625  | 1.00 | 28.46 | C |
| ATOM | 1852 | C   | SER | B | 31 | -54.004 | -35.394 | 86.484  | 1.00 | 30.09 | C |
| ATOM | 1853 | O   | SER | B | 31 | -53.192 | -36.163 | 85.956  | 1.00 | 25.87 | O |
| ATOM | 1854 | CB  | SER | B | 31 | -54.464 | -33.608 | 84.839  | 1.00 | 28.04 | C |
| ATOM | 1855 | OG  | SER | B | 31 | -54.110 | -32.544 | 85.699  | 1.00 | 28.97 | O |
| ATOM | 1856 | N   | GLY | B | 32 | -54.026 | -35.129 | 87.797  | 1.00 | 30.79 | N |
| ATOM | 1857 | CA  | GLY | B | 32 | -52.930 | -35.453 | 88.685  | 1.00 | 23.28 | C |
| ATOM | 1858 | C   | GLY | B | 32 | -51.708 | -34.581 | 88.501  | 1.00 | 25.51 | C |
| ATOM | 1859 | O   | GLY | B | 32 | -50.636 | -34.931 | 88.982  | 1.00 | 24.92 | O |
| ATOM | 1860 | N   | TYR | B | 33 | -51.838 | -33.436 | 87.830  | 1.00 | 25.51 | N |
| ATOM | 1861 | CA  | TYR | B | 33 | -50.697 | -32.549 | 87.607  | 1.00 | 22.85 | C |
| ATOM | 1862 | C   | TYR | B | 33 | -50.468 | -31.718 | 88.875  | 1.00 | 28.41 | C |
| ATOM | 1863 | O   | TYR | B | 33 | -50.739 | -30.514 | 88.938  | 1.00 | 24.46 | O |
| ATOM | 1864 | CB  | TYR | B | 33 | -50.934 | -31.657 | 86.394  | 1.00 | 25.72 | C |
| ATOM | 1865 | CG  | TYR | B | 33 | -50.986 | -32.351 | 85.031  | 1.00 | 27.40 | C |
| ATOM | 1866 | CD1 | TYR | B | 33 | -50.607 | -33.684 | 84.869  | 1.00 | 25.12 | C |
| ATOM | 1867 | CD2 | TYR | B | 33 | -51.398 | -31.651 | 83.901  | 1.00 | 25.37 | C |
| ATOM | 1868 | CE1 | TYR | B | 33 | -50.659 | -34.306 | 83.618  | 1.00 | 27.61 | C |
| ATOM | 1869 | CE2 | TYR | B | 33 | -51.443 | -32.256 | 82.647  | 1.00 | 28.75 | C |
| ATOM | 1870 | CZ  | TYR | B | 33 | -51.073 | -33.582 | 82.505  | 1.00 | 31.43 | C |
| ATOM | 1871 | OH  | TYR | B | 33 | -51.108 | -34.164 | 81.251  | 1.00 | 25.68 | O |
| ATOM | 1872 | N   | LEU | B | 34 | -49.964 | -32.400 | 89.908  | 1.00 | 26.49 | N |
| ATOM | 1873 | CA  | LEU | B | 34 | -49.740 | -31.801 | 91.217  | 1.00 | 25.62 | C |
| ATOM | 1874 | C   | LEU | B | 34 | -48.316 | -32.075 | 91.697  | 1.00 | 25.75 | C |
| ATOM | 1875 | O   | LEU | B | 34 | -47.893 | -33.228 | 91.789  | 1.00 | 27.99 | O |
| ATOM | 1876 | CB  | LEU | B | 34 | -50.742 | -32.328 | 92.239  | 1.00 | 25.15 | C |
| ATOM | 1877 | CG  | LEU | B | 34 | -50.732 | -31.388 | 93.436  | 1.00 | 22.33 | C |
| ATOM | 1878 | CD1 | LEU | B | 34 | -52.055 | -30.748 | 93.532  | 1.00 | 24.71 | C |
| ATOM | 1879 | CD2 | LEU | B | 34 | -50.401 | -32.127 | 94.682  | 1.00 | 22.19 | C |
| ATOM | 1880 | N   | ALA | B | 35 | -47.595 | -31.021 | 92.035  | 1.00 | 26.54 | N |
| ATOM | 1881 | CA  | ALA | B | 35 | -46.221 | -31.114 | 92.497  | 1.00 | 27.18 | C |
| ATOM | 1882 | C   | ALA | B | 35 | -46.154 | -30.759 | 93.973  | 1.00 | 25.51 | C |
| ATOM | 1883 | O   | ALA | B | 35 | -47.050 | -30.117 | 94.516  | 1.00 | 24.44 | O |
| ATOM | 1884 | CB  | ALA | B | 35 | -45.293 | -30.186 | 91.706  | 1.00 | 20.44 | C |
| ATOM | 1885 | N   | TRP | B | 36 | -45.088 | -31.214 | 94.621  | 1.00 | 23.22 | N |
| ATOM | 1886 | CA  | TRP | B | 36 | -44.790 | -30.852 | 95.995  | 1.00 | 26.05 | C |
| ATOM | 1887 | C   | TRP | B | 36 | -43.368 | -30.316 | 96.037  | 1.00 | 25.42 | C |
| ATOM | 1888 | O   | TRP | B | 36 | -42.462 | -30.896 | 95.429  | 1.00 | 26.45 | O |
| ATOM | 1889 | CB  | TRP | B | 36 | -44.935 | -32.050 | 96.966  | 1.00 | 23.50 | C |
| ATOM | 1890 | CG  | TRP | B | 36 | -46.328 | -32.470 | 97.286  | 1.00 | 23.73 | C |
| ATOM | 1891 | CD1 | TRP | B | 36 | -47.071 | -33.413 | 96.633  | 1.00 | 26.66 | C |
| ATOM | 1892 | CD2 | TRP | B | 36 | -47.144 | -32.006 | 98.375  | 1.00 | 28.12 | C |
| ATOM | 1893 | NE1 | TRP | B | 36 | -48.304 | -33.549 | 97.229  | 1.00 | 24.58 | N |
| ATOM | 1894 | CE2 | TRP | B | 36 | -48.374 | -32.709 | 98.306  | 1.00 | 26.57 | C |
| ATOM | 1895 | CE3 | TRP | B | 36 | -46.951 | -31.079 | 99.407  | 1.00 | 20.21 | C |
| ATOM | 1896 | CZ2 | TRP | B | 36 | -49.407 | -32.506 | 99.222  | 1.00 | 22.88 | C |
| ATOM | 1897 | CZ3 | TRP | B | 36 | -47.970 | -30.872 | 100.294 | 1.00 | 23.52 | C |
| ATOM | 1898 | CH2 | TRP | B | 36 | -49.190 | -31.584 | 100.203 | 1.00 | 24.77 | C |
| ATOM | 1899 | N   | TYR | B | 37 | -43.173 | -29.219 | 96.764  | 1.00 | 25.12 | N |
| ATOM | 1900 | CA  | TYR | B | 37 | -41.858 | -28.628 | 96.947  | 1.00 | 23.85 | C |
| ATOM | 1901 | C   | TYR | B | 37 | -41.551 | -28.525 | 98.427  | 1.00 | 27.32 | C |
| ATOM | 1902 | O   | TYR | B | 37 | -42.446 | -28.279 | 99.247  | 1.00 | 28.76 | O |
| ATOM | 1903 | CB  | TYR | B | 37 | -41.764 | -27.229 | 96.341  | 1.00 | 25.99 | C |
| ATOM | 1904 | CG  | TYR | B | 37 | -42.052 | -27.217 | 94.879  | 1.00 | 25.68 | C |
| ATOM | 1905 | CD2 | TYR | B | 37 | -41.025 | -27.346 | 93.944  | 1.00 | 27.12 | C |
| ATOM | 1906 | CD1 | TYR | B | 37 | -43.346 | -27.106 | 94.426  | 1.00 | 20.71 | C |
| ATOM | 1907 | CE2 | TYR | B | 37 | -41.296 | -27.349 | 92.584  | 1.00 | 23.06 | C |
| ATOM | 1908 | CE1 | TYR | B | 37 | -43.622 | -27.112 | 93.088  | 1.00 | 25.67 | C |
| ATOM | 1909 | CZ  | TYR | B | 37 | -42.598 | -27.241 | 92.170  | 1.00 | 22.19 | C |
| ATOM | 1910 | OH  | TYR | B | 37 | -42.911 | -27.244 | 90.831  | 1.00 | 28.45 | O |
| ATOM | 1911 | N   | GLN | B | 38 | -40.267 | -28.679 | 98.742  | 1.00 | 24.78 | N |
| ATOM | 1912 | CA  | GLN | B | 38 | -39.708 | -28.428 | 100.061 | 1.00 | 26.12 | C |
| ATOM | 1913 | C   | GLN | B | 38 | -38.871 | -27.147 | 100.017 | 1.00 | 23.99 | C |
| ATOM | 1914 | O   | GLN | B | 38 | -38.168 | -26.890 | 99.034  | 1.00 | 22.68 | O |

```
ATOM   1915  CB   GLN B  38     -38.852 -29.619 100.509  1.00 24.24           C
ATOM   1916  CG   GLN B  38     -38.144 -29.399 101.826  1.00 22.10           C
ATOM   1917  CD   GLN B  38     -37.108 -30.460 102.113  1.00 26.91           C
ATOM   1918  OE1  GLN B  38     -36.023 -30.439 101.532  1.00 33.75           O
ATOM   1919  NE2  GLN B  38     -37.418 -31.376 103.032  1.00 24.91           N
ATOM   1920  N    GLN B  39     -38.944 -26.339 101.072  1.00 19.06           N
ATOM   1921  CA   GLN B  39     -38.193 -25.085 101.113  1.00 24.00           C
ATOM   1922  C    GLN B  39     -37.632 -24.846 102.498  1.00 23.43           C
ATOM   1923  O    GLN B  39     -38.389 -24.722 103.462  1.00 21.25           O
ATOM   1924  CB   GLN B  39     -39.043 -23.881 100.719  1.00 25.47           C
ATOM   1925  CG   GLN B  39     -38.226 -22.610 100.620  1.00 25.34           C
ATOM   1926  CD   GLN B  39     -39.027 -21.423 100.161  1.00 31.02           C
ATOM   1927  OE1  GLN B  39     -40.183 -21.244 100.538  1.00 34.04           O
ATOM   1928  NE2  GLN B  39     -38.418 -20.601  99.334  1.00 33.16           N
ATOM   1929  N    LYS B  40     -36.319 -24.712 102.575  1.00 25.20           N
ATOM   1930  CA   LYS B  40     -35.694 -24.322 103.822  1.00 25.84           C
ATOM   1931  C    LYS B  40     -35.497 -22.813 103.870  1.00 27.20           C
ATOM   1932  O    LYS B  40     -35.478 -22.145 102.827  1.00 26.37           O
ATOM   1933  CB   LYS B  40     -34.382 -25.077 103.991  1.00 27.92           C
ATOM   1934  CG   LYS B  40     -34.624 -26.487 104.544  1.00 30.32           C
ATOM   1935  CD   LYS B  40     -33.459 -27.416 104.259  1.00 42.40           C
ATOM   1936  CE   LYS B  40     -33.761 -28.834 104.704  1.00 39.36           C
ATOM   1937  NZ   LYS B  40     -33.984 -28.911 106.185  1.00 42.39           N1+
ATOM   1938  N    PRO B  41     -35.419 -22.231 105.072  1.00 28.86           N
ATOM   1939  CA   PRO B  41     -35.467 -20.757 105.192  1.00 27.65           C
ATOM   1940  C    PRO B  41     -34.345 -20.077 104.416  1.00 27.79           C
ATOM   1941  O    PRO B  41     -33.168 -20.420 104.558  1.00 30.55           O
ATOM   1942  CB   PRO B  41     -35.331 -20.523 106.701  1.00 25.62           C
ATOM   1943  CG   PRO B  41     -35.861 -21.785 107.321  1.00 23.53           C
ATOM   1944  CD   PRO B  41     -35.415 -22.885 106.393  1.00 22.03           C
ATOM   1945  N    GLY B  42     -34.724 -19.113 103.577  1.00 30.24           N
ATOM   1946  CA   GLY B  42     -33.743 -18.444 102.750  1.00 26.94           C
ATOM   1947  C    GLY B  42     -33.131 -19.294 101.657  1.00 34.34           C
ATOM   1948  O    GLY B  42     -32.033 -18.976 101.191  1.00 29.82           O
ATOM   1949  N    GLN B  43     -33.790 -20.388 101.254  1.00 29.49           N
ATOM   1950  CA   GLN B  43     -33.330 -21.236 100.164  1.00 29.08           C
ATOM   1951  C    GLN B  43     -34.400 -21.299  99.086  1.00 26.57           C
ATOM   1952  O    GLN B  43     -35.578 -21.019  99.329  1.00 28.43           O
ATOM   1953  CB   GLN B  43     -32.994 -22.665 100.622  1.00 33.38           C
ATOM   1954  CG   GLN B  43     -32.182 -22.796 101.894  1.00 31.30           C
ATOM   1955  CD   GLN B  43     -30.769 -22.262 101.737  1.00 45.21           C
ATOM   1956  OE1  GLN B  43     -30.173 -22.361 100.665  1.00 48.17           O
ATOM   1957  NE2  GLN B  43     -30.215 -21.713 102.818  1.00 50.07           N
ATOM   1958  N    THR B  44     -33.988 -21.698  97.895  1.00 28.06           N
ATOM   1959  CA   THR B  44     -34.976 -21.828  96.832  1.00 32.50           C
ATOM   1960  C    THR B  44     -35.755 -23.142  97.000  1.00 29.43           C
ATOM   1961  O    THR B  44     -35.282 -24.077  97.652  1.00 28.21           O
ATOM   1962  CB   THR B  44     -34.303 -21.769  95.453  1.00 28.68           C
ATOM   1963  OG1  THR B  44     -33.844 -23.064  95.087  1.00 33.54           O
ATOM   1964  CG2  THR B  44     -33.095 -20.847  95.473  1.00 35.23           C
ATOM   1965  N    PRO B  45     -36.982 -23.217  96.495  1.00 29.94           N
ATOM   1966  CA   PRO B  45     -37.745 -24.456  96.658  1.00 27.80           C
ATOM   1967  C    PRO B  45     -37.021 -25.614  95.982  1.00 24.29           C
ATOM   1968  O    PRO B  45     -36.256 -25.428  95.042  1.00 28.45           O
ATOM   1969  CB   PRO B  45     -39.079 -24.140  95.967  1.00 26.59           C
ATOM   1970  CG   PRO B  45     -39.175 -22.670  95.983  1.00 23.14           C
ATOM   1971  CD   PRO B  45     -37.774 -22.177  95.813  1.00 27.43           C
ATOM   1972  N    ARG B  46     -37.249 -26.817  96.485  1.00 24.84           N
ATOM   1973  CA   ARG B  46     -36.741 -28.036  95.863  1.00 27.05           C
ATOM   1974  C    ARG B  46     -37.920 -28.935  95.516  1.00 28.13           C
ATOM   1975  O    ARG B  46     -38.743 -29.258  96.385  1.00 27.30           O
ATOM   1976  CB   ARG B  46     -35.754 -28.763  96.782  1.00 27.57           C
ATOM   1977  CG   ARG B  46     -35.323 -30.144  96.305  1.00 35.27           C
ATOM   1978  CD   ARG B  46     -34.354 -30.799  97.302  1.00 42.68           C
ATOM   1979  NE   ARG B  46     -33.035 -30.146  97.316  1.00 59.78           N
ATOM   1980  CZ   ARG B  46     -32.623 -29.218  98.197  1.00 64.38           C
ATOM   1981  NH1  ARG B  46     -33.408 -28.780  99.199  1.00 56.48           N1+
ATOM   1982  NH2  ARG B  46     -31.399 -28.715  98.076  1.00 61.89           N
ATOM   1983  N    LEU B  47     -38.007 -29.318  94.244  1.00 28.50           N
ATOM   1984  CA   LEU B  47     -39.058 -30.216  93.781  1.00 25.28           C
ATOM   1985  C    LEU B  47     -38.930 -31.576  94.455  1.00 25.95           C
```

```
ATOM   1986  O    LEU B  47    -37.851 -32.175  94.457  1.00 30.65           O
ATOM   1987  CB   LEU B  47    -38.970 -30.365  92.262  1.00 27.11           C
ATOM   1988  CG   LEU B  47    -39.935 -31.368  91.643  1.00 27.81           C
ATOM   1989  CD1  LEU B  47    -41.357 -30.870  91.856  1.00 21.28           C
ATOM   1990  CD2  LEU B  47    -39.622 -31.533  90.175  1.00 24.15           C
ATOM   1991  N    LEU B  48    -40.027 -32.055  95.040  1.00 22.73           N
ATOM   1992  CA   LEU B  48    -40.056 -33.353  95.715  1.00 28.05           C
ATOM   1993  C    LEU B  48    -40.844 -34.397  94.953  1.00 30.46           C
ATOM   1994  O    LEU B  48    -40.406 -35.548  94.862  1.00 28.89           O
ATOM   1995  CB   LEU B  48    -40.685 -33.237  97.112  1.00 29.74           C
ATOM   1996  CG   LEU B  48    -40.032 -32.529  98.287  1.00 28.54           C
ATOM   1997  CD1  LEU B  48    -40.954 -32.632  99.462  1.00 25.93           C
ATOM   1998  CD2  LEU B  48    -38.712 -33.198  98.616  1.00 30.43           C
ATOM   1999  N    ILE B  49    -42.012 -33.999  94.435  1.00 26.40           N
ATOM   2000  CA   ILE B  49    -42.988 -34.879  93.804  1.00 26.75           C
ATOM   2001  C    ILE B  49    -43.560 -34.161  92.594  1.00 24.70           C
ATOM   2002  O    ILE B  49    -43.809 -32.958  92.648  1.00 22.99           O
ATOM   2003  CB   ILE B  49    -44.129 -35.264  94.780  1.00 26.52           C
ATOM   2004  CG1  ILE B  49    -43.583 -35.980  96.018  1.00 21.26           C
ATOM   2005  CG2  ILE B  49    -45.215 -36.049  94.058  1.00 20.52           C
ATOM   2006  CD1  ILE B  49    -43.212 -37.429  95.770  1.00 22.03           C
ATOM   2007  N    PHE B  50    -43.749 -34.888  91.491  1.00 26.33           N
ATOM   2008  CA   PHE B  50    -44.548 -34.395  90.375  1.00 26.42           C
ATOM   2009  C    PHE B  50    -45.564 -35.460  89.959  1.00 30.44           C
ATOM   2010  O    PHE B  50    -45.467 -36.634  90.342  1.00 27.91           O
ATOM   2011  CB   PHE B  50    -43.678 -33.982  89.184  1.00 21.93           C
ATOM   2012  CG   PHE B  50    -42.877 -35.114  88.610  1.00 30.11           C
ATOM   2013  CD1  PHE B  50    -43.407 -35.919  87.609  1.00 27.24           C
ATOM   2014  CD2  PHE B  50    -41.599 -35.383  89.079  1.00 28.83           C
ATOM   2015  CE1  PHE B  50    -42.682 -36.965  87.085  1.00 34.14           C
ATOM   2016  CE2  PHE B  50    -40.865 -36.430  88.547  1.00 34.66           C
ATOM   2017  CZ   PHE B  50    -41.409 -37.230  87.553  1.00 31.45           C
ATOM   2018  N    GLY B  51    -46.579 -35.022  89.216  1.00 29.06           N
ATOM   2019  CA   GLY B  51    -47.640 -35.920  88.790  1.00 23.55           C
ATOM   2020  C    GLY B  51    -48.357 -36.601  89.926  1.00 28.78           C
ATOM   2021  O    GLY B  51    -48.771 -37.758  89.789  1.00 31.77           O
ATOM   2022  N    ALA B  52    -48.497 -35.913  91.057  1.00 27.54           N
ATOM   2023  CA   ALA B  52    -49.180 -36.389  92.253  1.00 27.65           C
ATOM   2024  C    ALA B  52    -48.403 -37.468  92.995  1.00 27.89           C
ATOM   2025  O    ALA B  52    -48.422 -37.465  94.231  1.00 25.68           O
ATOM   2026  CB   ALA B  52    -50.589 -36.907  91.927  1.00 24.96           C
ATOM   2027  N    SER B  53    -47.679 -38.358  92.289  1.00 24.53           N
ATOM   2028  CA   SER B  53    -47.036 -39.468  93.002  1.00 27.35           C
ATOM   2029  C    SER B  53    -45.606 -39.792  92.588  1.00 30.40           C
ATOM   2030  O    SER B  53    -44.970 -40.616  93.257  1.00 31.18           O
ATOM   2031  CB   SER B  53    -47.855 -40.745  92.844  1.00 27.15           C
ATOM   2032  OG   SER B  53    -48.178 -40.933  91.485  1.00 33.66           O
ATOM   2033  N    SER B  54    -45.082 -39.207  91.526  1.00 27.47           N
ATOM   2034  CA   SER B  54    -43.758 -39.568  91.050  1.00 28.53           C
ATOM   2035  C    SER B  54    -42.717 -38.787  91.833  1.00 28.97           C
ATOM   2036  O    SER B  54    -42.754 -37.552  91.877  1.00 29.74           O
ATOM   2037  CB   SER B  54    -43.621 -39.289  89.556  1.00 27.68           C
ATOM   2038  OG   SER B  54    -44.449 -40.156  88.815  1.00 33.67           O
ATOM   2039  N    ARG B  55    -41.795 -39.519  92.432  1.00 27.26           N
ATOM   2040  CA   ARG B  55    -40.657 -38.955  93.138  1.00 32.14           C
ATOM   2041  C    ARG B  55    -39.710 -38.275  92.152  1.00 30.42           C
ATOM   2042  O    ARG B  55    -39.438 -38.817  91.084  1.00 34.35           O
ATOM   2043  CB   ARG B  55    -39.955 -40.099  93.864  1.00 28.20           C
ATOM   2044  CG   ARG B  55    -39.168 -39.786  95.061  1.00 33.52           C
ATOM   2045  CD   ARG B  55    -38.583 -41.090  95.651  1.00 37.14           C
ATOM   2046  NE   ARG B  55    -39.581 -41.791  96.442  1.00 41.03           N
ATOM   2047  CZ   ARG B  55    -40.134 -42.948  96.118  1.00 41.47           C
ATOM   2048  NH1  ARG B  55    -41.056 -43.474  96.923  1.00 39.94           N1+
ATOM   2049  NH2  ARG B  55    -39.756 -43.579  95.009  1.00 39.66           N
ATOM   2050  N    ALA B  56    -39.246 -37.068  92.483  1.00 32.10           N
ATOM   2051  CA   ALA B  56    -38.216 -36.416  91.686  1.00 34.96           C
ATOM   2052  C    ALA B  56    -36.843 -37.013  91.987  1.00 37.35           C
ATOM   2053  O    ALA B  56    -36.647 -37.718  92.975  1.00 43.05           O
ATOM   2054  CB   ALA B  56    -38.183 -34.911  91.952  1.00 30.18           C
ATOM   2055  N    THR B  57    -35.892 -36.740  91.111  1.00 37.37           N
ATOM   2056  CA   THR B  57    -34.521 -37.183  91.324  1.00 38.34           C
```

EP 4 435 105 A2

```
ATOM    2057  C    THR B  57     -33.836 -36.270  92.313  1.00 39.91           C
ATOM    2058  O    THR B  57     -34.179 -35.087  92.386  1.00 46.64           O
ATOM    2059  CB   THR B  57     -33.716 -37.185  90.020  1.00 45.59           C
ATOM    2060  OG1  THR B  57     -34.516 -36.612  88.969  1.00 40.75           O
ATOM    2061  CG2  THR B  57     -33.271 -38.620  89.674  1.00 44.70           C
ATOM    2062  N    GLY B  58     -32.937 -36.780  93.143  1.00 39.76           N
ATOM    2063  CA   GLY B  58     -32.957 -38.118  93.653  1.00 35.82           C
ATOM    2064  C    GLY B  58     -33.485 -37.854  95.061  1.00 40.59           C
ATOM    2065  O    GLY B  58     -32.728 -37.724  96.021  1.00 37.19           O
ATOM    2066  N    ILE B  59     -34.798 -37.704  95.160  1.00 33.85           N
ATOM    2067  CA   ILE B  59     -35.455 -37.513  96.450  1.00 31.14           C
ATOM    2068  C    ILE B  59     -35.518 -38.865  97.158  1.00 38.31           C
ATOM    2069  O    ILE B  59     -35.890 -39.859  96.524  1.00 35.35           O
ATOM    2070  CB   ILE B  59     -36.855 -36.904  96.264  1.00 29.49           C
ATOM    2071  CG1  ILE B  59     -36.817 -35.375  96.109  1.00 29.46           C
ATOM    2072  CG2  ILE B  59     -37.756 -37.240  97.420  1.00 30.78           C
ATOM    2073  CD1  ILE B  59     -35.937 -34.826  95.062  1.00 37.31           C
ATOM    2074  N    PRO B  60     -35.097 -38.960  98.459  1.00 38.88           N
ATOM    2075  CA   PRO B  60     -35.157 -40.238  99.191  1.00 33.20           C
ATOM    2076  C    PRO B  60     -36.530 -40.890  99.128  1.00 36.41           C
ATOM    2077  O    PRO B  60     -37.527 -40.200  98.889  1.00 37.79           O
ATOM    2078  CB   PRO B  60     -34.831 -39.817 100.629  1.00 34.92           C
ATOM    2079  CG   PRO B  60     -34.076 -38.600 100.501  1.00 30.53           C
ATOM    2080  CD   PRO B  60     -34.609 -37.876  99.320  1.00 31.01           C
ATOM    2081  N    ASP B  61     -36.626 -42.202  99.320  1.00 33.86           N
ATOM    2082  CA   ASP B  61     -37.968 -42.778  99.248  1.00 38.89           C
ATOM    2083  C    ASP B  61     -38.762 -42.694 100.558  1.00 33.94           C
ATOM    2084  O    ASP B  61     -39.868 -43.244 100.610  1.00 36.31           O
ATOM    2085  CB   ASP B  61     -37.897 -44.220  98.751  1.00 43.25           C
ATOM    2086  CG   ASP B  61     -36.955 -45.052  99.542  1.00 47.58           C
ATOM    2087  OD1  ASP B  61     -36.741 -44.735 100.731  1.00 49.56           O
ATOM    2088  OD2  ASP B  61     -36.427 -46.029  98.972  1.00 65.96           O1-
ATOM    2089  N    ARG B  62     -38.222 -42.061 101.609  1.00 32.55           N
ATOM    2090  CA   ARG B  62     -39.015 -41.532 102.717  1.00 32.36           C
ATOM    2091  C    ARG B  62     -40.241 -40.805 102.198  1.00 29.63           C
ATOM    2092  O    ARG B  62     -41.318 -40.850 102.794  1.00 30.70           O
ATOM    2093  CB   ARG B  62     -38.243 -40.501 103.536  1.00 35.05           C
ATOM    2094  CG   ARG B  62     -37.041 -40.958 104.180  1.00 36.82           C
ATOM    2095  CD   ARG B  62     -36.754 -40.043 105.334  1.00 38.78           C
ATOM    2096  NE   ARG B  62     -36.191 -38.739 105.001  1.00 39.12           N
ATOM    2097  CZ   ARG B  62     -35.001 -38.571 104.422  1.00 39.01           C
ATOM    2098  NH1  ARG B  62     -34.289 -39.630 104.056  1.00 36.63           N1+
ATOM    2099  NH2  ARG B  62     -34.530 -37.352 104.195  1.00 33.11           N
ATOM    2100  N    PHE B  63     -40.042 -40.074 101.115  1.00 31.12           N
ATOM    2101  CA   PHE B  63     -41.075 -39.242 100.534  1.00 35.11           C
ATOM    2102  C    PHE B  63     -41.916 -40.080  99.586  1.00 34.68           C
ATOM    2103  O    PHE B  63     -41.373 -40.790  98.729  1.00 30.34           O
ATOM    2104  CB   PHE B  63     -40.444 -38.054  99.803  1.00 27.94           C
ATOM    2105  CG   PHE B  63     -39.701 -37.135 100.713  1.00 28.72           C
ATOM    2106  CD1  PHE B  63     -38.408 -37.425 101.099  1.00 31.44           C
ATOM    2107  CD2  PHE B  63     -40.308 -36.008 101.227  1.00 30.94           C
ATOM    2108  CE1  PHE B  63     -37.731 -36.592 101.968  1.00 33.00           C
ATOM    2109  CE2  PHE B  63     -39.630 -35.178 102.097  1.00 29.25           C
ATOM    2110  CZ   PHE B  63     -38.344 -35.471 102.464  1.00 27.63           C
ATOM    2111  N    SER B  64     -43.235 -40.023  99.775  1.00 30.03           N
ATOM    2112  CA   SER B  64     -44.181 -40.581  98.819  1.00 30.73           C
ATOM    2113  C    SER B  64     -45.432 -39.715  98.844  1.00 27.36           C
ATOM    2114  O    SER B  64     -45.684 -38.974  99.796  1.00 25.57           O
ATOM    2115  CB   SER B  64     -44.501 -42.064  99.109  1.00 28.95           C
ATOM    2116  OG   SER B  64     -45.445 -42.217 100.156  1.00 29.24           O
ATOM    2117  N    ALA B  65     -46.221 -39.806  97.784  1.00 26.92           N
ATOM    2118  CA   ALA B  65     -47.407 -38.974  97.723  1.00 30.37           C
ATOM    2119  C    ALA B  65     -48.448 -39.670  96.870  1.00 30.47           C
ATOM    2120  O    ALA B  65     -48.134 -40.578  96.096  1.00 31.92           O
ATOM    2121  CB   ALA B  65     -47.087 -37.583  97.157  1.00 26.67           C
ATOM    2122  N    SER B  66     -49.692 -39.216  97.007  1.00 27.32           N
ATOM    2123  CA   SER B  66     -50.792 -39.781  96.242  1.00 30.42           C
ATOM    2124  C    SER B  66     -51.978 -38.825  96.278  1.00 27.86           C
ATOM    2125  O    SER B  66     -51.955 -37.788  96.938  1.00 30.99           O
ATOM    2126  CB   SER B  66     -51.165 -41.164  96.778  1.00 32.05           C
ATOM    2127  OG   SER B  66     -51.732 -41.026  98.065  1.00 42.20           O
```

422

```
ATOM   2128  N    GLY B  67    -53.025 -39.190  95.559  1.00 32.79           N
ATOM   2129  CA   GLY B  67    -54.256 -38.435  95.552  1.00 28.74           C
ATOM   2130  C    GLY B  67    -54.669 -38.125  94.138  1.00 34.13           C
ATOM   2131  O    GLY B  67    -53.835 -38.175  93.223  1.00 38.32           O
ATOM   2132  N    SER B  68    -55.943 -37.795  93.936  1.00 35.14           N
ATOM   2133  CA   SER B  68    -56.352 -37.221  92.663  1.00 35.12           C
ATOM   2134  C    SER B  68    -57.678 -36.522  92.863  1.00 35.78           C
ATOM   2135  O    SER B  68    -58.409 -36.804  93.810  1.00 40.40           O
ATOM   2136  CB   SER B  68    -56.473 -38.268  91.551  1.00 42.92           C
ATOM   2137  OG   SER B  68    -56.634 -37.637  90.276  1.00 47.38           O
ATOM   2138  N    GLY B  69    -57.990 -35.635  91.924  1.00 38.09           N
ATOM   2139  CA   GLY B  69    -59.167 -34.812  91.999  1.00 27.17           C
ATOM   2140  C    GLY B  69    -59.015 -33.747  93.048  1.00 30.94           C
ATOM   2141  O    GLY B  69    -58.281 -32.775  92.867  1.00 32.99           O
ATOM   2142  N    ALA B  70    -59.712 -33.901  94.161  1.00 36.50           N
ATOM   2143  CA   ALA B  70    -59.738 -32.812  95.113  1.00 33.81           C
ATOM   2144  C    ALA B  70    -58.726 -32.974  96.231  1.00 32.23           C
ATOM   2145  O    ALA B  70    -58.401 -31.980  96.877  1.00 32.47           O
ATOM   2146  CB   ALA B  70    -61.137 -32.661  95.704  1.00 27.91           C
ATOM   2147  N    ASP B  71    -58.216 -34.184  96.471  1.00 28.53           N
ATOM   2148  CA   ASP B  71    -57.431 -34.467  97.669  1.00 32.89           C
ATOM   2149  C    ASP B  71    -56.068 -35.055  97.343  1.00 29.82           C
ATOM   2150  O    ASP B  71    -55.966 -36.028  96.596  1.00 32.00           O
ATOM   2151  CB   ASP B  71    -58.176 -35.394  98.631  1.00 29.01           C
ATOM   2152  CG   ASP B  71    -59.067 -34.621  99.579  1.00 49.76           C
ATOM   2153  OD1  ASP B  71    -58.492 -34.037 100.529  1.00 51.82           O
ATOM   2154  OD2  ASP B  71    -60.313 -34.586  99.395  1.00 55.52           O1-
ATOM   2155  N    PHE B  72    -55.023 -34.452  97.915  1.00 28.01           N
ATOM   2156  CA   PHE B  72    -53.651 -34.911  97.756  1.00 26.76           C
ATOM   2157  C    PHE B  72    -52.964 -34.963  99.110  1.00 24.59           C
ATOM   2158  O    PHE B  72    -53.217 -34.129  99.987  1.00 27.45           O
ATOM   2159  CB   PHE B  72    -52.889 -34.002  96.778  1.00 29.56           C
ATOM   2160  CG   PHE B  72    -53.477 -34.000  95.391  1.00 29.36           C
ATOM   2161  CD1  PHE B  72    -54.491 -33.128  95.058  1.00 29.76           C
ATOM   2162  CD2  PHE B  72    -53.050 -34.917  94.441  1.00 28.24           C
ATOM   2163  CE1  PHE B  72    -55.056 -33.152  93.790  1.00 33.06           C
ATOM   2164  CE2  PHE B  72    -53.606 -34.935  93.163  1.00 29.21           C
ATOM   2165  CZ   PHE B  72    -54.619 -34.058  92.846  1.00 27.62           C
ATOM   2166  N    THR B  73    -52.117 -35.955  99.300  1.00 20.91           N
ATOM   2167  CA   THR B  73    -51.335 -36.002 100.516  1.00 24.42           C
ATOM   2168  C    THR B  73    -49.884 -36.323 100.180  1.00 25.69           C
ATOM   2169  O    THR B  73    -49.583 -37.044  99.216  1.00 24.83           O
ATOM   2170  CB   THR B  73    -51.917 -37.002 101.553  1.00 23.96           C
ATOM   2171  OG1  THR B  73    -51.922 -38.307 101.005  1.00 31.75           O
ATOM   2172  CG2  THR B  73    -53.357 -36.634 101.919  1.00 29.11           C
ATOM   2173  N    LEU B  74    -48.989 -35.720 100.963  1.00 25.80           N
ATOM   2174  CA   LEU B  74    -47.565 -36.023 100.955  1.00 26.02           C
ATOM   2175  C    LEU B  74    -47.252 -36.738 102.262  1.00 25.39           C
ATOM   2176  O    LEU B  74    -47.701 -36.303 103.322  1.00 25.96           O
ATOM   2177  CB   LEU B  74    -46.730 -34.747 100.811  1.00 22.71           C
ATOM   2178  CG   LEU B  74    -45.217 -34.943 100.997  1.00 27.97           C
ATOM   2179  CD1  LEU B  74    -44.602 -35.660  99.806  1.00 23.58           C
ATOM   2180  CD2  LEU B  74    -44.486 -33.635 101.264  1.00 25.42           C
ATOM   2181  N    THR B  75    -46.551 -37.866 102.182  1.00 25.21           N
ATOM   2182  CA   THR B  75    -46.187 -38.649 103.358  1.00 24.77           C
ATOM   2183  C    THR B  75    -44.676 -38.773 103.458  1.00 29.94           C
ATOM   2184  O    THR B  75    -44.012 -39.180 102.496  1.00 30.23           O
ATOM   2185  CB   THR B  75    -46.830 -40.040 103.345  1.00 27.93           C
ATOM   2186  OG1  THR B  75    -48.185 -39.936 103.785  1.00 33.93           O
ATOM   2187  CG2  THR B  75    -46.105 -40.998 104.261  1.00 32.07           C
ATOM   2188  N    ILE B  76    -44.145 -38.402 104.621  1.00 29.36           N
ATOM   2189  CA   ILE B  76    -42.760 -38.649 105.003  1.00 29.13           C
ATOM   2190  C    ILE B  76    -42.807 -39.727 106.074  1.00 29.72           C
ATOM   2191  O    ILE B  76    -43.436 -39.537 107.122  1.00 30.62           O
ATOM   2192  CB   ILE B  76    -42.070 -37.369 105.508  1.00 26.60           C
ATOM   2193  CG1  ILE B  76    -42.336 -36.210 104.551  1.00 32.41           C
ATOM   2194  CG2  ILE B  76    -40.581 -37.563 105.617  1.00 24.68           C
ATOM   2195  CD1  ILE B  76    -41.949 -34.861 105.118  1.00 27.44           C
ATOM   2196  N    SER B  77    -42.195 -40.878 105.798  1.00 31.29           N
ATOM   2197  CA   SER B  77    -42.453 -42.041 106.645  1.00 34.52           C
ATOM   2198  C    SER B  77    -41.647 -42.001 107.937  1.00 36.39           C
```

```
ATOM    2199   O    SER B  77    -42.148 -42.404 108.994  1.00 42.84        O
ATOM    2200   CB   SER B  77    -42.183 -43.327 105.871  1.00 28.00        C
ATOM    2201   OG   SER B  77    -40.838 -43.394 105.441  1.00 30.78        O
ATOM    2202   N    ARG B  78    -40.419 -41.502 107.873  1.00 29.86        N
ATOM    2203   CA   ARG B  78    -39.567 -41.274 109.032  1.00 29.69        C
ATOM    2204   C    ARG B  78    -38.973 -39.882 108.882  1.00 32.92        C
ATOM    2205   O    ARG B  78    -38.317 -39.601 107.879  1.00 33.41        O
ATOM    2206   CB   ARG B  78    -38.467 -42.342 109.133  1.00 39.68        C
ATOM    2207   CG   ARG B  78    -37.255 -41.895 109.982  1.00 45.90        C
ATOM    2208   CD   ARG B  78    -36.191 -42.997 110.245  1.00 50.49        C
ATOM    2209   NE   ARG B  78    -35.401 -42.741 111.473  1.00 56.24        N
ATOM    2210   CZ   ARG B  78    -35.796 -43.031 112.717  1.00 51.76        C
ATOM    2211   NH1  ARG B  78    -35.013 -42.754 113.768  1.00 42.94        N1+
ATOM    2212   NH2  ARG B  78    -36.989 -43.591 112.915  1.00 53.21        N
ATOM    2213   N    LEU B  79    -39.284 -38.987 109.813  1.00 33.51        N
ATOM    2214   CA   LEU B  79    -38.785 -37.612 109.757  1.00 30.48        C
ATOM    2215   C    LEU B  79    -37.289 -37.545 110.092  1.00 31.79        C
ATOM    2216   O    LEU B  79    -36.893 -37.791 111.233  1.00 34.10        O
ATOM    2217   CB   LEU B  79    -39.590 -36.756 110.722  1.00 29.33        C
ATOM    2218   CG   LEU B  79    -40.492 -35.683 110.136  1.00 29.23        C
ATOM    2219   CD1  LEU B  79    -40.779 -35.916 108.706  1.00 23.33        C
ATOM    2220   CD2  LEU B  79    -41.769 -35.681 110.920  1.00 27.74        C
ATOM    2221   N    GLU B  80    -36.439 -37.169 109.109  1.00 32.68        N
ATOM    2222   CA   GLU B  80    -35.028 -36.892 109.360  1.00 33.86        C
ATOM    2223   C    GLU B  80    -34.836 -35.408 109.665  1.00 35.88        C
ATOM    2224   O    GLU B  80    -35.701 -34.593 109.339  1.00 34.51        O
ATOM    2225   CB   GLU B  80    -34.186 -37.294 108.144  1.00 36.87        C
ATOM    2226   CG   GLU B  80    -34.116 -38.792 107.876  1.00 36.11        C
ATOM    2227   CD   GLU B  80    -33.480 -39.579 109.018  1.00 45.93        C
ATOM    2228   OE1  GLU B  80    -33.946 -40.719 109.264  1.00 47.66        O
ATOM    2229   OE2  GLU B  80    -32.553 -39.050 109.696  1.00 47.84        O1-
ATOM    2230   N    PRO B  81    -33.719 -35.014 110.302  1.00 38.88        N
ATOM    2231   CA   PRO B  81    -33.544 -33.588 110.659  1.00 34.21        C
ATOM    2232   C    PRO B  81    -33.719 -32.635 109.488  1.00 36.66        C
ATOM    2233   O    PRO B  81    -34.340 -31.574 109.634  1.00 35.85        O
ATOM    2234   CB   PRO B  81    -32.112 -33.540 111.209  1.00 41.16        C
ATOM    2235   CG   PRO B  81    -31.881 -34.922 111.747  1.00 43.24        C
ATOM    2236   CD   PRO B  81    -32.600 -35.843 110.790  1.00 43.50        C
ATOM    2237   N    GLU B  82    -33.190 -32.994 108.325  1.00 33.34        N
ATOM    2238   CA   GLU B  82    -33.310 -32.225 107.093  1.00 34.68        C
ATOM    2239   C    GLU B  82    -34.704 -32.235 106.492  1.00 36.53        C
ATOM    2240   O    GLU B  82    -34.861 -31.767 105.360  1.00 32.72        O
ATOM    2241   CB   GLU B  82    -32.363 -32.799 106.059  1.00 37.11        C
ATOM    2242   CG   GLU B  82    -32.783 -34.160 105.573  1.00 41.97        C
ATOM    2243   CD   GLU B  82    -31.777 -35.240 105.930  1.00 51.14        C
ATOM    2244   OE1  GLU B  82    -31.259 -35.252 107.093  1.00 45.81        O
ATOM    2245   OE2  GLU B  82    -31.522 -36.080 105.032  1.00 60.52        O1-
ATOM    2246   N    ASP B  83    -35.691 -32.835 107.151  1.00 36.25        N
ATOM    2247   CA   ASP B  83    -37.045 -32.856 106.622  1.00 30.47        C
ATOM    2248   C    ASP B  83    -37.924 -31.782 107.227  1.00 26.07        C
ATOM    2249   O    ASP B  83    -39.029 -31.554 106.726  1.00 22.59        O
ATOM    2250   CB   ASP B  83    -37.673 -34.235 106.815  1.00 28.20        C
ATOM    2251   CG   ASP B  83    -36.966 -35.300 105.987  1.00 37.30        C
ATOM    2252   OD1  ASP B  83    -36.200 -34.897 105.068  1.00 38.14        O
ATOM    2253   OD2  ASP B  83    -37.177 -36.519 106.234  1.00 37.91        O1-
ATOM    2254   N    PHE B  84    -37.420 -31.069 108.221  1.00 22.98        N
ATOM    2255   CA   PHE B  84    -38.174 -30.025 108.897  1.00 27.33        C
ATOM    2256   C    PHE B  84    -37.971 -28.727 108.122  1.00 28.99        C
ATOM    2257   O    PHE B  84    -36.861 -28.182 108.083  1.00 25.03        O
ATOM    2258   CB   PHE B  84    -37.721 -29.926 110.346  1.00 27.37        C
ATOM    2259   CG   PHE B  84    -37.992 -31.172 111.114  1.00 29.16        C
ATOM    2260   CD2  PHE B  84    -39.193 -31.337 111.794  1.00 27.59        C
ATOM    2261   CD1  PHE B  84    -37.076 -32.213 111.100  1.00 25.70        C
ATOM    2262   CE2  PHE B  84    -39.456 -32.507 112.497  1.00 31.00        C
ATOM    2263   CE1  PHE B  84    -37.334 -33.386 111.793  1.00 31.57        C
ATOM    2264   CZ   PHE B  84    -38.527 -33.529 112.505  1.00 32.40        C
ATOM    2265   N    ALA B  85    -39.048 -28.257 107.494  1.00 26.46        N
ATOM    2266   CA   ALA B  85    -39.022 -27.288 106.408  1.00 23.46        C
ATOM    2267   C    ALA B  85    -40.457 -26.866 106.126  1.00 23.53        C
ATOM    2268   O    ALA B  85    -41.404 -27.329 106.771  1.00 22.25        O
ATOM    2269   CB   ALA B  85    -38.376 -27.893 105.154  1.00 21.86        C
```

| ATOM | 2270 | N   | VAL | B | 86 | -40.617 | -26.039 | 105.105 | 1.00 | 23.47 | N |
| ATOM | 2271 | CA  | VAL | B | 86 | -41.936 | -25.680 | 104.600 | 1.00 | 23.19 | C |
| ATOM | 2272 | C   | VAL | B | 86 | -42.201 | -26.477 | 103.330 | 1.00 | 22.81 | C |
| ATOM | 2273 | O   | VAL | B | 86 | -41.314 | -26.672 | 102.500 | 1.00 | 22.61 | O |
| ATOM | 2274 | CB  | VAL | B | 86 | -42.053 | -24.162 | 104.366 | 1.00 | 25.35 | C |
| ATOM | 2275 | CG1 | VAL | B | 86 | -43.330 | -23.837 | 103.664 | 1.00 | 26.56 | C |
| ATOM | 2276 | CG2 | VAL | B | 86 | -42.014 | -23.424 | 105.700 | 1.00 | 19.36 | C |
| ATOM | 2277 | N   | TYR | B | 87 | -43.417 | -26.967 | 103.188 | 1.00 | 23.96 | N |
| ATOM | 2278 | CA  | TYR | B | 87 | -43.798 | -27.738 | 102.021 | 1.00 | 25.43 | C |
| ATOM | 2279 | C   | TYR | B | 87 | -44.915 | -27.002 | 101.304 | 1.00 | 27.51 | C |
| ATOM | 2280 | O   | TYR | B | 87 | -45.806 | -26.452 | 101.949 | 1.00 | 24.76 | O |
| ATOM | 2281 | CB  | TYR | B | 87 | -44.216 | -29.157 | 102.418 | 1.00 | 22.13 | C |
| ATOM | 2282 | CG  | TYR | B | 87 | -43.036 | -29.940 | 102.943 | 1.00 | 26.73 | C |
| ATOM | 2283 | CD1 | TYR | B | 87 | -42.575 | -29.754 | 104.253 | 1.00 | 22.18 | C |
| ATOM | 2284 | CD2 | TYR | B | 87 | -42.355 | -30.833 | 102.129 | 1.00 | 22.28 | C |
| ATOM | 2285 | CE1 | TYR | B | 87 | -41.484 | -30.435 | 104.729 | 1.00 | 21.84 | C |
| ATOM | 2286 | CE2 | TYR | B | 87 | -41.256 | -31.533 | 102.612 | 1.00 | 25.81 | C |
| ATOM | 2287 | CZ  | TYR | B | 87 | -40.816 | -31.334 | 103.906 | 1.00 | 22.45 | C |
| ATOM | 2288 | OH  | TYR | B | 87 | -39.716 | -32.037 | 104.374 | 1.00 | 18.88 | O |
| ATOM | 2289 | N   | PHE | B | 88 | -44.823 | -26.949 | 99.973  | 1.00 | 25.00 | N |
| ATOM | 2290 | CA  | PHE | B | 88 | -45.780 | -26.258 | 99.124  | 1.00 | 26.03 | C |
| ATOM | 2291 | C   | PHE | B | 88 | -46.299 | -27.240 | 98.077  | 1.00 | 30.78 | C |
| ATOM | 2292 | O   | PHE | B | 88 | -45.519 | -28.026 | 97.519  | 1.00 | 30.24 | O |
| ATOM | 2293 | CB  | PHE | B | 88 | -45.131 | -25.063 | 98.406  | 1.00 | 27.78 | C |
| ATOM | 2294 | CG  | PHE | B | 88 | -44.809 | -23.887 | 99.307  | 1.00 | 31.23 | C |
| ATOM | 2295 | CD1 | PHE | B | 88 | -45.802 | -23.008 | 99.730  | 1.00 | 30.25 | C |
| ATOM | 2296 | CD2 | PHE | B | 88 | -43.494 | -23.634 | 99.690  | 1.00 | 30.31 | C |
| ATOM | 2297 | CE1 | PHE | B | 88 | -45.493 | -21.904 | 100.539 | 1.00 | 30.57 | C |
| ATOM | 2298 | CE2 | PHE | B | 88 | -43.185 | -22.549 | 100.489 | 1.00 | 34.11 | C |
| ATOM | 2299 | CZ  | PHE | B | 88 | -44.197 | -21.678 | 100.917 | 1.00 | 34.33 | C |
| ATOM | 2300 | N   | CYS | B | 89 | -47.603 | -27.210 | 97.808  | 1.00 | 25.66 | N |
| ATOM | 2301 | CA  | CYS | B | 89 | -48.130 | -27.930 | 96.660  | 1.00 | 24.07 | C |
| ATOM | 2302 | C   | CYS | B | 89 | -48.355 | -26.953 | 95.513  | 1.00 | 23.52 | C |
| ATOM | 2303 | O   | CYS | B | 89 | -48.402 | -25.739 | 95.708  | 1.00 | 21.13 | O |
| ATOM | 2304 | CB  | CYS | B | 89 | -49.416 | -28.709 | 96.998  | 1.00 | 21.36 | C |
| ATOM | 2305 | SG  | CYS | B | 89 | -50.748 | -27.800 | 97.719  | 1.00 | 30.20 | S |
| ATOM | 2306 | N   | GLN | B | 90 | -48.451 | -27.502 | 94.302  | 1.00 | 22.98 | N |
| ATOM | 2307 | CA  | GLN | B | 90 | -48.547 | -26.702 | 93.085  | 1.00 | 23.61 | C |
| ATOM | 2308 | C   | GLN | B | 90 | -49.265 | -27.514 | 92.017  | 1.00 | 24.59 | C |
| ATOM | 2309 | O   | GLN | B | 90 | -48.979 | -28.703 | 91.859  | 1.00 | 24.95 | O |
| ATOM | 2310 | CB  | GLN | B | 90 | -47.153 | -26.272 | 92.588  | 1.00 | 23.03 | C |
| ATOM | 2311 | CG  | GLN | B | 90 | -47.199 | -25.382 | 91.346  | 1.00 | 24.92 | C |
| ATOM | 2312 | CD  | GLN | B | 90 | -46.481 | -25.971 | 90.137  | 1.00 | 26.13 | C |
| ATOM | 2313 | OE1 | GLN | B | 90 | -45.402 | -26.556 | 90.261  | 1.00 | 32.43 | O |
| ATOM | 2314 | NE2 | GLN | B | 90 | -47.093 | -25.847 | 88.969  | 1.00 | 23.23 | N |
| ATOM | 2315 | N   | GLN | B | 91 | -50.221 | -26.897 | 91.319  | 1.00 | 22.59 | N |
| ATOM | 2316 | CA  | GLN | B | 91 | -50.911 | -27.567 | 90.224  | 1.00 | 24.25 | C |
| ATOM | 2317 | C   | GLN | B | 91 | -50.510 | -26.926 | 88.899  | 1.00 | 25.92 | C |
| ATOM | 2318 | O   | GLN | B | 91 | -50.206 | -25.731 | 88.830  | 1.00 | 24.83 | O |
| ATOM | 2319 | CB  | GLN | B | 91 | -52.449 | -27.564 | 90.405  | 1.00 | 19.74 | C |
| ATOM | 2320 | CG  | GLN | B | 91 | -53.125 | -26.219 | 90.299  | 1.00 | 21.60 | C |
| ATOM | 2321 | CD  | GLN | B | 91 | -53.417 | -25.811 | 88.871  | 1.00 | 24.32 | C |
| ATOM | 2322 | OE1 | GLN | B | 91 | -53.515 | -26.657 | 87.975  | 1.00 | 23.50 | O |
| ATOM | 2323 | NE2 | GLN | B | 91 | -53.561 | -24.499 | 88.648  | 1.00 | 23.44 | N |
| ATOM | 2324 | N   | TYR | B | 92 | -50.462 | -27.737 | 87.850  | 1.00 | 24.33 | N |
| ATOM | 2325 | CA  | TYR | B | 92 | -50.099 | -27.241 | 86.524  | 1.00 | 26.73 | C |
| ATOM | 2326 | C   | TYR | B | 92 | -51.027 | -27.842 | 85.493  | 1.00 | 27.55 | C |
| ATOM | 2327 | O   | TYR | B | 92 | -50.626 | -28.137 | 84.362  | 1.00 | 29.60 | O |
| ATOM | 2328 | CB  | TYR | B | 92 | -48.610 | -27.501 | 86.194  | 1.00 | 23.03 | C |
| ATOM | 2329 | CG  | TYR | B | 92 | -48.133 | -28.903 | 86.494  | 1.00 | 22.43 | C |
| ATOM | 2330 | CD1 | TYR | B | 92 | -47.776 | -29.257 | 87.789  | 1.00 | 22.77 | C |
| ATOM | 2331 | CD2 | TYR | B | 92 | -48.036 | -29.874 | 85.496  | 1.00 | 22.32 | C |
| ATOM | 2332 | CE1 | TYR | B | 92 | -47.349 | -30.533 | 88.095  | 1.00 | 22.69 | C |
| ATOM | 2333 | CE2 | TYR | B | 92 | -47.589 | -31.154 | 85.788  | 1.00 | 21.88 | C |
| ATOM | 2334 | CZ  | TYR | B | 92 | -47.254 | -31.478 | 87.096  | 1.00 | 23.71 | C |
| ATOM | 2335 | OH  | TYR | B | 92 | -46.820 | -32.729 | 87.444  | 1.00 | 20.44 | O |
| ATOM | 2336 | N   | GLU | B | 93 | -52.289 | -28.031 | 85.880  | 1.00 | 27.99 | N |
| ATOM | 2337 | CA  | GLU | B | 93 | -53.312 | -28.512 | 84.958  | 1.00 | 30.82 | C |
| ATOM | 2338 | C   | GLU | B | 93 | -53.999 | -27.364 | 84.244  | 1.00 | 31.11 | C |
| ATOM | 2339 | O   | GLU | B | 93 | -54.182 | -27.424 | 83.026  | 1.00 | 30.30 | O |
| ATOM | 2340 | CB  | GLU | B | 93 | -54.360 | -29.364 | 85.691  | 1.00 | 30.98 | C |

```
ATOM   2341  CG  GLU B  93    -55.690 -29.496  84.939  1.00 25.68          C
ATOM   2342  CD  GLU B  93    -56.678 -30.425  85.629  1.00 33.12          C
ATOM   2343  OE1 GLU B  93    -56.247 -31.498  86.123  1.00 31.54          O
ATOM   2344  OE2 GLU B  93    -57.889 -30.090  85.680  1.00 32.74          O1-
ATOM   2345  N   SER B  94    -54.366 -26.310  84.970  1.00 30.35          N
ATOM   2346  CA  SER B  94    -55.097 -25.199  84.378  1.00 32.23          C
ATOM   2347  C   SER B  94    -54.388 -23.888  84.703  1.00 28.38          C
ATOM   2348  O   SER B  94    -54.044 -23.617  85.861  1.00 23.42          O
ATOM   2349  CB  SER B  94    -56.556 -25.171  84.862  1.00 32.62          C
ATOM   2350  OG  SER B  94    -57.144 -23.891  84.656  1.00 44.26          O
ATOM   2351  N   SER B  95    -54.180 -23.077  83.678  1.00 29.26          N
ATOM   2352  CA  SER B  95    -53.498 -21.814  83.854  1.00 28.94          C
ATOM   2353  C   SER B  95    -54.464 -20.862  84.524  1.00 28.67          C
ATOM   2354  O   SER B  95    -55.645 -20.897  84.219  1.00 29.79          O
ATOM   2355  CB  SER B  95    -53.015 -21.271  82.514  1.00 23.10          C
ATOM   2356  OG  SER B  95    -52.370 -20.022  82.690  1.00 38.38          O
ATOM   2357  N   PRO B  96    -53.976 -20.029  85.458  1.00 27.09          N
ATOM   2358  CA  PRO B  96    -52.599 -19.892  85.956  1.00 26.26          C
ATOM   2359  C   PRO B  96    -52.131 -21.034  86.857  1.00 27.92          C
ATOM   2360  O   PRO B  96    -52.933 -21.550  87.627  1.00 31.29          O
ATOM   2361  CB  PRO B  96    -52.658 -18.601  86.768  1.00 25.28          C
ATOM   2362  CG  PRO B  96    -54.047 -18.557  87.258  1.00 27.55          C
ATOM   2363  CD  PRO B  96    -54.892 -19.111  86.148  1.00 23.84          C
ATOM   2364  N   TRP B  97    -50.858 -21.419  86.786  1.00 26.30          N
ATOM   2365  CA  TRP B  97    -50.330 -22.305  87.811  1.00 26.57          C
ATOM   2366  C   TRP B  97    -50.524 -21.661  89.178  1.00 25.80          C
ATOM   2367  O   TRP B  97    -50.307 -20.460  89.358  1.00 25.76          O
ATOM   2368  CB  TRP B  97    -48.847 -22.617  87.594  1.00 25.63          C
ATOM   2369  CG  TRP B  97    -48.411 -23.337  86.309  1.00 28.94          C
ATOM   2370  CD1 TRP B  97    -47.124 -23.541  85.926  1.00 29.48          C
ATOM   2371  CD2 TRP B  97    -49.229 -23.928  85.264  1.00 29.78          C
ATOM   2372  NE1 TRP B  97    -47.073 -24.216  84.733  1.00 32.61          N
ATOM   2373  CE2 TRP B  97    -48.344 -24.459  84.296  1.00 28.86          C
ATOM   2374  CE3 TRP B  97    -50.608 -24.072  85.061  1.00 29.91          C
ATOM   2375  CZ2 TRP B  97    -48.789 -25.116  83.134  1.00 31.43          C
ATOM   2376  CZ3 TRP B  97    -51.054 -24.724  83.902  1.00 28.75          C
ATOM   2377  CH2 TRP B  97    -50.140 -25.232  82.951  1.00 31.01          C
ATOM   2378  N   THR B  98    -50.931 -22.460  90.148  1.00 21.99          N
ATOM   2379  CA  THR B  98    -51.167 -21.932  91.472  1.00 23.81          C
ATOM   2380  C   THR B  98    -50.474 -22.819  92.488  1.00 23.94          C
ATOM   2381  O   THR B  98    -50.148 -23.973  92.215  1.00 25.95          O
ATOM   2382  CB  THR B  98    -52.674 -21.787  91.782  1.00 26.18          C
ATOM   2383  OG1 THR B  98    -53.374 -23.006  91.498  1.00 23.15          O
ATOM   2384  CG2 THR B  98    -53.270 -20.618  90.969  1.00 24.44          C
ATOM   2385  N   PHE B  99    -50.184 -22.209  93.633  1.00 28.63          N
ATOM   2386  CA  PHE B  99    -49.511 -22.803  94.776  1.00 26.82          C
ATOM   2387  C   PHE B  99    -50.436 -22.773  95.988  1.00 31.15          C
ATOM   2388  O   PHE B  99    -51.311 -21.912  96.089  1.00 31.15          O
ATOM   2389  CB  PHE B  99    -48.236 -22.020  95.121  1.00 27.58          C
ATOM   2390  CG  PHE B  99    -47.123 -22.166  94.115  1.00 26.17          C
ATOM   2391  CD1 PHE B  99    -46.212 -23.210  94.213  1.00 21.23          C
ATOM   2392  CD2 PHE B  99    -46.970 -21.236  93.096  1.00 26.56          C
ATOM   2393  CE1 PHE B  99    -45.179 -23.346  93.313  1.00 23.45          C
ATOM   2394  CE2 PHE B  99    -45.935 -21.362  92.177  1.00 28.21          C
ATOM   2395  CZ  PHE B  99    -45.028 -22.420  92.291  1.00 25.30          C
ATOM   2396  N   GLY B 100    -50.252 -23.722  96.909  1.00 29.01          N
ATOM   2397  CA  GLY B 100    -50.849 -23.604  98.224  1.00 26.65          C
ATOM   2398  C   GLY B 100    -50.062 -22.660  99.136  1.00 32.15          C
ATOM   2399  O   GLY B 100    -48.953 -22.224  98.831  1.00 30.70          O
ATOM   2400  N   GLN B 101    -50.651 -22.339 100.291  1.00 32.09          N
ATOM   2401  CA  GLN B 101    -49.953 -21.455 101.219  1.00 27.63          C
ATOM   2402  C   GLN B 101    -48.788 -22.134 101.926  1.00 31.28          C
ATOM   2403  O   GLN B 101    -47.993 -21.430 102.555  1.00 31.67          O
ATOM   2404  CB  GLN B 101    -50.888 -20.871 102.296  1.00 31.00          C
ATOM   2405  CG  GLN B 101    -52.371 -21.297 102.285  1.00 41.04          C
ATOM   2406  CD  GLN B 101    -52.581 -22.771 102.623  1.00 41.89          C
ATOM   2407  OE1 GLN B 101    -52.927 -23.567 101.736  1.00 36.09          O
ATOM   2408  NE2 GLN B 101    -52.365 -23.147 103.900  1.00 37.62          N
ATOM   2409  N   GLY B 102    -48.665 -23.453 101.858  1.00 26.43          N
ATOM   2410  CA  GLY B 102    -47.548 -24.085 102.535  1.00 24.50          C
ATOM   2411  C   GLY B 102    -47.895 -24.678 103.898  1.00 27.19          C
```

```
ATOM   2412  O   GLY B 102    -48.800 -24.215 104.600  1.00 27.42           O
ATOM   2413  N   THR B 103    -47.158 -25.724 104.283  1.00 24.40           N
ATOM   2414  CA  THR B 103    -47.207 -26.276 105.634  1.00 27.22           C
ATOM   2415  C   THR B 103    -45.806 -26.259 106.239  1.00 25.76           C
ATOM   2416  O   THR B 103    -44.857 -26.746 105.621  1.00 27.96           O
ATOM   2417  CB  THR B 103    -47.752 -27.718 105.647  1.00 28.05           C
ATOM   2418  OG1 THR B 103    -49.120 -27.729 105.232  1.00 31.71           O
ATOM   2419  CG2 THR B 103    -47.654 -28.348 107.061  1.00 25.08           C
ATOM   2420  N   LYS B 104    -45.673 -25.709 107.442  1.00 22.08           N
ATOM   2421  CA  LYS B 104    -44.409 -25.758 108.159  1.00 26.19           C
ATOM   2422  C   LYS B 104    -44.360 -27.021 109.014  1.00 27.07           C
ATOM   2423  O   LYS B 104    -45.205 -27.212 109.889  1.00 28.44           O
ATOM   2424  CB  LYS B 104    -44.212 -24.523 109.032  1.00 25.21           C
ATOM   2425  CG  LYS B 104    -42.846 -24.510 109.709  1.00 26.78           C
ATOM   2426  CD  LYS B 104    -42.677 -23.365 110.699  1.00 30.43           C
ATOM   2427  CE  LYS B 104    -41.301 -23.463 111.391  1.00 45.22           C
ATOM   2428  NZ  LYS B 104    -40.967 -22.316 112.301  1.00 44.27           N1+
ATOM   2429  N   VAL B 105    -43.366 -27.869 108.779  1.00 25.76           N
ATOM   2430  CA  VAL B 105    -43.119 -29.033 109.624  1.00 29.32           C
ATOM   2431  C   VAL B 105    -42.005 -28.664 110.597  1.00 27.51           C
ATOM   2432  O   VAL B 105    -40.841 -28.516 110.211  1.00 25.98           O
ATOM   2433  CB  VAL B 105    -42.757 -30.283 108.813  1.00 24.39           C
ATOM   2434  CG1 VAL B 105    -42.488 -31.445 109.776  1.00 26.12           C
ATOM   2435  CG2 VAL B 105    -43.868 -30.636 107.890  1.00 21.86           C
ATOM   2436  N   GLU B 106    -42.367 -28.533 111.863  1.00 31.16           N
ATOM   2437  CA  GLU B 106    -41.473 -28.092 112.919  1.00 28.93           C
ATOM   2438  C   GLU B 106    -41.144 -29.242 113.875  1.00 33.11           C
ATOM   2439  O   GLU B 106    -41.922 -30.197 114.030  1.00 30.26           O
ATOM   2440  CB  GLU B 106    -42.130 -26.943 113.669  1.00 31.52           C
ATOM   2441  CG  GLU B 106    -41.324 -26.435 114.830  1.00 41.69           C
ATOM   2442  CD  GLU B 106    -42.170 -26.121 116.028  1.00 37.31           C
ATOM   2443  OE1 GLU B 106    -42.903 -27.027 116.485  1.00 37.32           O
ATOM   2444  OE2 GLU B 106    -42.102 -24.963 116.492  1.00 39.39           O1-
ATOM   2445  N   ILE B 107    -39.971 -29.149 114.495  1.00 26.80           N
ATOM   2446  CA  ILE B 107    -39.486 -30.168 115.421  1.00 28.39           C
ATOM   2447  C   ILE B 107    -40.205 -30.038 116.755  1.00 32.03           C
ATOM   2448  O   ILE B 107    -40.118 -29.003 117.420  1.00 27.69           O
ATOM   2449  CB  ILE B 107    -37.974 -30.050 115.622  1.00 26.88           C
ATOM   2450  CG1 ILE B 107    -37.240 -30.423 114.344  1.00 27.26           C
ATOM   2451  CG2 ILE B 107    -37.552 -30.898 116.793  1.00 26.83           C
ATOM   2452  CD1 ILE B 107    -35.799 -30.043 114.347  1.00 30.15           C
ATOM   2453  N   LYS B 108    -40.866 -31.106 117.182  1.00 31.78           N
ATOM   2454  CA  LYS B 108    -41.455 -31.110 118.512  1.00 32.40           C
ATOM   2455  C   LYS B 108    -40.382 -31.497 119.529  1.00 32.97           C
ATOM   2456  O   LYS B 108    -39.654 -32.475 119.336  1.00 33.78           O
ATOM   2457  CB  LYS B 108    -42.649 -32.069 118.567  1.00 34.38           C
ATOM   2458  CG  LYS B 108    -43.460 -32.006 119.864  1.00 35.60           C
ATOM   2459  CD  LYS B 108    -44.379 -33.223 120.000  1.00 34.87           C
ATOM   2460  CE  LYS B 108    -45.671 -32.857 120.690  1.00 39.16           C
ATOM   2461  NZ  LYS B 108    -45.560 -31.457 121.228  1.00 41.36           N1+
ATOM   2462  N   ARG B 109    -40.270 -30.720 120.604  1.00 30.43           N
ATOM   2463  CA  ARG B 109    -39.298 -30.996 121.651  1.00 27.95           C
ATOM   2464  C   ARG B 109    -39.942 -30.659 122.985  1.00 29.25           C
ATOM   2465  O   ARG B 109    -41.117 -30.284 123.040  1.00 33.46           O
ATOM   2466  CB  ARG B 109    -37.993 -30.224 121.445  1.00 27.54           C
ATOM   2467  CG  ARG B 109    -38.128 -28.705 121.378  1.00 29.91           C
ATOM   2468  CD  ARG B 109    -36.873 -28.023 121.928  1.00 26.11           C
ATOM   2469  NE  ARG B 109    -36.853 -28.173 123.386  1.00 32.71           N
ATOM   2470  CZ  ARG B 109    -35.751 -28.189 124.126  1.00 27.84           C
ATOM   2471  NH1 ARG B 109    -35.827 -28.351 125.449  1.00 24.67           N1+
ATOM   2472  NH2 ARG B 109    -34.577 -28.039 123.536  1.00 23.42           N
ATOM   2473  N   THR B 110    -39.191 -30.838 124.073  1.00 27.37           N
ATOM   2474  CA  THR B 110    -39.762 -30.554 125.385  1.00 28.62           C
ATOM   2475  C   THR B 110    -39.775 -29.056 125.631  1.00 29.53           C
ATOM   2476  O   THR B 110    -38.878 -28.311 125.203  1.00 26.75           O
ATOM   2477  CB  THR B 110    -39.006 -31.227 126.546  1.00 27.19           C
ATOM   2478  OG1 THR B 110    -37.641 -30.803 126.570  1.00 31.87           O
ATOM   2479  CG2 THR B 110    -39.052 -32.727 126.445  1.00 25.84           C
ATOM   2480  N   VAL B 111    -40.796 -28.630 126.367  1.00 31.89           N
ATOM   2481  CA  VAL B 111    -40.905 -27.236 126.752  1.00 33.07           C
ATOM   2482  C   VAL B 111    -39.618 -26.751 127.407  1.00 31.48           C
```

```
ATOM   2483  O    VAL B 111     -38.982 -27.463 128.195  1.00 31.62           O
ATOM   2484  CB   VAL B 111     -42.112 -27.056 127.683  1.00 30.76           C
ATOM   2485  CG1  VAL B 111     -42.176 -25.612 128.149  1.00 27.34           C
ATOM   2486  CG2  VAL B 111     -43.381 -27.478 126.949  1.00 24.38           C
ATOM   2487  N    ALA B 112     -39.223 -25.532 127.044  1.00 29.57           N
ATOM   2488  CA   ALA B 112     -38.047 -24.863 127.582  1.00 25.09           C
ATOM   2489  C    ALA B 112     -38.425 -23.405 127.788  1.00 30.16           C
ATOM   2490  O    ALA B 112     -38.831 -22.734 126.835  1.00 27.80           O
ATOM   2491  CB   ALA B 112     -36.844 -24.984 126.639  1.00 23.34           C
ATOM   2492  N    ALA B 113     -38.318 -22.928 129.029  1.00 32.01           N
ATOM   2493  CA   ALA B 113     -38.663 -21.549 129.326  1.00 28.03           C
ATOM   2494  C    ALA B 113     -37.579 -20.640 128.768  1.00 29.40           C
ATOM   2495  O    ALA B 113     -36.412 -21.035 128.710  1.00 31.93           O
ATOM   2496  CB   ALA B 113     -38.799 -21.325 130.830  1.00 23.97           C
ATOM   2497  N    PRO B 114     -37.934 -19.422 128.353  1.00 27.52           N
ATOM   2498  CA   PRO B 114     -36.911 -18.489 127.860  1.00 27.42           C
ATOM   2499  C    PRO B 114     -36.095 -17.920 129.004  1.00 27.03           C
ATOM   2500  O    PRO B 114     -36.590 -17.719 130.108  1.00 29.23           O
ATOM   2501  CB   PRO B 114     -37.731 -17.386 127.186  1.00 27.64           C
ATOM   2502  CG   PRO B 114     -39.012 -17.376 127.998  1.00 27.54           C
ATOM   2503  CD   PRO B 114     -39.274 -18.806 128.403  1.00 24.48           C
ATOM   2504  N    SER B 115     -34.835 -17.635 128.730  1.00 27.91           N
ATOM   2505  CA   SER B 115     -34.092 -16.739 129.592  1.00 25.63           C
ATOM   2506  C    SER B 115     -34.236 -15.331 129.028  1.00 25.72           C
ATOM   2507  O    SER B 115     -34.238 -15.130 127.814  1.00 27.20           O
ATOM   2508  CB   SER B 115     -32.628 -17.157 129.726  1.00 24.15           C
ATOM   2509  OG   SER B 115     -32.139 -17.720 128.535  1.00 40.34           O
ATOM   2510  N    VAL B 116     -34.430 -14.371 129.922  1.00 26.54           N
ATOM   2511  CA   VAL B 116     -34.857 -13.023 129.579  1.00 28.89           C
ATOM   2512  C    VAL B 116     -33.762 -12.037 129.979  1.00 26.93           C
ATOM   2513  O    VAL B 116     -33.243 -12.093 131.094  1.00 31.62           O
ATOM   2514  CB   VAL B 116     -36.203 -12.695 130.259  1.00 28.35           C
ATOM   2515  CG1  VAL B 116     -36.709 -11.313 129.860  1.00 28.35           C
ATOM   2516  CG2  VAL B 116     -37.212 -13.740 129.882  1.00 18.89           C
ATOM   2517  N    PHE B 117     -33.406 -11.151 129.063  1.00 25.01           N
ATOM   2518  CA   PHE B 117     -32.424 -10.118 129.311  1.00 25.46           C
ATOM   2519  C    PHE B 117     -32.963  -8.826 128.726  1.00 28.62           C
ATOM   2520  O    PHE B 117     -33.533  -8.832 127.632  1.00 31.30           O
ATOM   2521  CB   PHE B 117     -31.081 -10.413 128.650  1.00 25.62           C
ATOM   2522  CG   PHE B 117     -30.536 -11.799 128.892  1.00 29.84           C
ATOM   2523  CD1  PHE B 117     -30.935 -12.876 128.093  1.00 24.22           C
ATOM   2524  CD2  PHE B 117     -29.542 -12.015 129.835  1.00 31.65           C
ATOM   2525  CE1  PHE B 117     -30.405 -14.136 128.283  1.00 22.90           C
ATOM   2526  CE2  PHE B 117     -28.998 -13.290 130.013  1.00 29.63           C
ATOM   2527  CZ   PHE B 117     -29.430 -14.339 129.234  1.00 24.06           C
ATOM   2528  N    ILE B 118     -32.755  -7.717 129.428  1.00 28.33           N
ATOM   2529  CA   ILE B 118     -33.211  -6.409 128.973  1.00 27.52           C
ATOM   2530  C    ILE B 118     -31.997  -5.501 128.878  1.00 27.02           C
ATOM   2531  O    ILE B 118     -31.130  -5.541 129.750  1.00 28.87           O
ATOM   2532  CB   ILE B 118     -34.280  -5.811 129.911  1.00 29.46           C
ATOM   2533  CG1  ILE B 118     -34.774  -4.480 129.347  1.00 30.76           C
ATOM   2534  CG2  ILE B 118     -33.752  -5.639 131.343  1.00 25.68           C
ATOM   2535  CD1  ILE B 118     -35.736  -3.775 130.229  1.00 29.17           C
ATOM   2536  N    PHE B 119     -31.925  -4.700 127.811  1.00 28.09           N
ATOM   2537  CA   PHE B 119     -30.765  -3.875 127.503  1.00 24.90           C
ATOM   2538  C    PHE B 119     -31.149  -2.406 127.478  1.00 29.17           C
ATOM   2539  O    PHE B 119     -32.037  -2.018 126.706  1.00 31.62           O
ATOM   2540  CB   PHE B 119     -30.163  -4.224 126.148  1.00 26.03           C
ATOM   2541  CG   PHE B 119     -29.643  -5.603 126.045  1.00 29.01           C
ATOM   2542  CD1  PHE B 119     -28.383  -5.913 126.505  1.00 26.67           C
ATOM   2543  CD2  PHE B 119     -30.400  -6.594 125.430  1.00 30.15           C
ATOM   2544  CE1  PHE B 119     -27.897  -7.200 126.403  1.00 29.34           C
ATOM   2545  CE2  PHE B 119     -29.927  -7.882 125.314  1.00 25.71           C
ATOM   2546  CZ   PHE B 119     -28.670  -8.191 125.806  1.00 29.15           C
ATOM   2547  N    PRO B 120     -30.468  -1.550 128.231  1.00 34.18           N
ATOM   2548  CA   PRO B 120     -30.747  -0.106 128.152  1.00 34.68           C
ATOM   2549  C    PRO B 120     -30.251   0.460 126.834  1.00 34.50           C
ATOM   2550  O    PRO B 120     -29.378  -0.140 126.188  1.00 36.34           O
ATOM   2551  CB   PRO B 120     -29.963   0.474 129.339  1.00 33.20           C
ATOM   2552  CG   PRO B 120     -29.568  -0.746 130.190  1.00 37.48           C
ATOM   2553  CD   PRO B 120     -29.417  -1.862 129.212  1.00 32.46           C
```

```
ATOM   2554  N   PRO B 121   -30.761   1.616 126.402  1.00 35.15        N
ATOM   2555  CA  PRO B 121   -30.200   2.248 125.203  1.00 34.06        C
ATOM   2556  C   PRO B 121   -28.766   2.697 125.452  1.00 32.75        C
ATOM   2557  O   PRO B 121   -28.410   3.117 126.551  1.00 34.53        O
ATOM   2558  CB  PRO B 121   -31.138   3.436 124.958  1.00 30.93        C
ATOM   2559  CG  PRO B 121   -31.733   3.718 126.279  1.00 29.12        C
ATOM   2560  CD  PRO B 121   -31.870   2.404 126.965  1.00 30.02        C
ATOM   2561  N   SER B 122   -27.930   2.583 124.431  1.00 35.60        N
ATOM   2562  CA  SER B 122   -26.558   3.043 124.576  1.00 40.70        C
ATOM   2563  C   SER B 122   -26.517   4.570 124.585  1.00 42.19        C
ATOM   2564  O   SER B 122   -27.311   5.239 123.913  1.00 40.94        O
ATOM   2565  CB  SER B 122   -25.677   2.503 123.437  1.00 38.69        C
ATOM   2566  OG  SER B 122   -25.921   3.200 122.223  1.00 33.97        O
ATOM   2567  N   ASP B 123   -25.550   5.120 125.328  1.00 41.84        N
ATOM   2568  CA  ASP B 123   -25.391   6.569 125.357  1.00 44.12        C
ATOM   2569  C   ASP B 123   -25.119   7.126 123.965  1.00 48.91        C
ATOM   2570  O   ASP B 123   -25.453   8.285 123.682  1.00 47.81        O
ATOM   2571  CB  ASP B 123   -24.267   6.968 126.311  1.00 47.34        C
ATOM   2572  CG  ASP B 123   -24.693   6.950 127.779  1.00 59.18        C
ATOM   2573  OD1 ASP B 123   -25.864   7.272 128.086  1.00 59.74        O
ATOM   2574  OD2 ASP B 123   -23.839   6.640 128.638  1.00 65.74        O1-
ATOM   2575  N   GLU B 124   -24.542   6.312 123.079  1.00 44.91        N
ATOM   2576  CA  GLU B 124   -24.273   6.776 121.722  1.00 48.45        C
ATOM   2577  C   GLU B 124   -25.559   7.001 120.919  1.00 48.55        C
ATOM   2578  O   GLU B 124   -25.649   7.962 120.145  1.00 48.61        O
ATOM   2579  CB  GLU B 124   -23.360   5.788 121.014  1.00 46.13        C
ATOM   2580  CG  GLU B 124   -22.949   6.279 119.661  1.00 52.72        C
ATOM   2581  CD  GLU B 124   -22.264   5.209 118.846  1.00 59.55        C
ATOM   2582  OE1 GLU B 124   -22.167   5.408 117.608  1.00 50.04        O
ATOM   2583  OE2 GLU B 124   -21.850   4.175 119.443  1.00 55.47        O1-
ATOM   2584  N   GLN B 125   -26.574   6.143 121.093  1.00 46.17        N
ATOM   2585  CA  GLN B 125   -27.839   6.369 120.391  1.00 41.17        C
ATOM   2586  C   GLN B 125   -28.632   7.506 121.018  1.00 44.73        C
ATOM   2587  O   GLN B 125   -29.391   8.187 120.315  1.00 41.05        O
ATOM   2588  CB  GLN B 125   -28.687   5.095 120.366  1.00 36.73        C
ATOM   2589  CG  GLN B 125   -30.012   5.222 119.593  1.00 33.48        C
ATOM   2590  CD  GLN B 125   -31.023   4.118 119.918  1.00 37.11        C
ATOM   2591  OE1 GLN B 125   -30.936   3.430 120.947  1.00 35.61        O
ATOM   2592  NE2 GLN B 125   -31.976   3.925 119.016  1.00 41.82        N
ATOM   2593  N   LEU B 126   -28.493   7.704 122.337  1.00 43.68        N
ATOM   2594  CA  LEU B 126   -29.168   8.813 123.006  1.00 45.10        C
ATOM   2595  C   LEU B 126   -28.719  10.152 122.434  1.00 49.62        C
ATOM   2596  O   LEU B 126   -29.526  11.085 122.313  1.00 49.20        O
ATOM   2597  CB  LEU B 126   -28.906   8.751 124.510  1.00 46.51        C
ATOM   2598  CG  LEU B 126   -29.691   7.699 125.300  1.00 44.00        C
ATOM   2599  CD1 LEU B 126   -29.263   7.692 126.754  1.00 37.07        C
ATOM   2600  CD2 LEU B 126   -31.208   7.898 125.167  1.00 36.79        C
ATOM   2601  N   LYS B 127   -27.428  10.263 122.083  1.00 47.40        N
ATOM   2602  CA  LYS B 127   -26.902  11.463 121.438  1.00 44.91        C
ATOM   2603  C   LYS B 127   -27.707  11.875 120.208  1.00 50.97        C
ATOM   2604  O   LYS B 127   -27.782  13.065 119.883  1.00 56.06        O
ATOM   2605  CB  LYS B 127   -25.441  11.244 121.060  1.00 53.95        C
ATOM   2606  CG  LYS B 127   -24.474  11.931 122.009  1.00 65.16        C
ATOM   2607  CD  LYS B 127   -24.039  11.038 123.160  1.00 60.17        C
ATOM   2608  CE  LYS B 127   -22.846  10.198 122.738  1.00 65.63        C
ATOM   2609  NZ  LYS B 127   -21.833  11.021 122.005  1.00 65.70        N1+
ATOM   2610  N   SER B 128   -28.302  10.918 119.506  1.00 48.42        N
ATOM   2611  CA  SER B 128   -29.026  11.194 118.274  1.00 44.86        C
ATOM   2612  C   SER B 128   -30.527  11.393 118.485  1.00 48.97        C
ATOM   2613  O   SER B 128   -31.271  11.432 117.498  1.00 56.04        O
ATOM   2614  CB  SER B 128   -28.814  10.057 117.266  1.00 51.64        C
ATOM   2615  OG  SER B 128   -29.460   8.856 117.686  1.00 48.78        O
ATOM   2616  N   GLY B 129   -30.999  11.500 119.728  1.00 42.11        N
ATOM   2617  CA  GLY B 129   -32.383  11.859 119.953  1.00 39.56        C
ATOM   2618  C   GLY B 129   -33.356  10.703 120.071  1.00 47.15        C
ATOM   2619  O   GLY B 129   -34.553  10.945 120.312  1.00 45.83        O
ATOM   2620  N   THR B 130   -32.894   9.459 119.903  1.00 44.00        N
ATOM   2621  CA  THR B 130   -33.745   8.278 119.983  1.00 37.83        C
ATOM   2622  C   THR B 130   -33.231   7.272 121.008  1.00 36.90        C
ATOM   2623  O   THR B 130   -32.023   7.117 121.190  1.00 41.23        O
ATOM   2624  CB  THR B 130   -33.871   7.637 118.606  1.00 40.93        C
```

```
ATOM   2625  OG1 THR B 130     -34.572    8.543 117.740  1.00 34.84           O
ATOM   2626  CG2 THR B 130     -34.617    6.297 118.679  1.00 44.09           C
ATOM   2627  N   ALA B 131     -34.155    6.598 121.686  1.00 35.77           N
ATOM   2628  CA  ALA B 131     -33.841    5.535 122.636  1.00 35.93           C
ATOM   2629  C   ALA B 131     -34.469    4.216 122.186  1.00 35.42           C
ATOM   2630  O   ALA B 131     -35.693    4.128 121.999  1.00 37.68           O
ATOM   2631  CB  ALA B 131     -34.346    5.896 124.039  1.00 33.77           C
ATOM   2632  N   SER B 132     -33.645    3.183 122.055  1.00 29.61           N
ATOM   2633  CA  SER B 132     -34.127    1.837 121.791  1.00 29.37           C
ATOM   2634  C   SER B 132     -33.845    0.960 122.999  1.00 24.73           C
ATOM   2635  O   SER B 132     -32.689    0.774 123.367  1.00 30.61           O
ATOM   2636  CB  SER B 132     -33.466    1.265 120.545  1.00 28.99           C
ATOM   2637  OG  SER B 132     -33.838    2.013 119.420  1.00 32.09           O
ATOM   2638  N   VAL B 133     -34.890    0.411 123.599  1.00 29.26           N
ATOM   2639  CA  VAL B 133     -34.753   -0.593 124.654  1.00 30.39           C
ATOM   2640  C   VAL B 133     -35.021   -1.960 124.044  1.00 28.40           C
ATOM   2641  O   VAL B 133     -35.963   -2.123 123.264  1.00 30.58           O
ATOM   2642  CB  VAL B 133     -35.717   -0.303 125.816  1.00 29.07           C
ATOM   2643  CG1 VAL B 133     -35.309   -1.095 127.049  1.00 27.89           C
ATOM   2644  CG2 VAL B 133     -35.751    1.186 126.090  1.00 26.20           C
ATOM   2645  N   VAL B 134     -34.203   -2.948 124.392  1.00 26.36           N
ATOM   2646  CA  VAL B 134     -34.262   -4.261 123.762  1.00 28.94           C
ATOM   2647  C   VAL B 134     -34.505   -5.315 124.830  1.00 29.71           C
ATOM   2648  O   VAL B 134     -33.851   -5.305 125.880  1.00 30.17           O
ATOM   2649  CB  VAL B 134     -32.973   -4.576 122.980  1.00 30.23           C
ATOM   2650  CG1 VAL B 134     -33.115   -5.912 122.281  1.00 22.43           C
ATOM   2651  CG2 VAL B 134     -32.648   -3.460 121.991  1.00 24.76           C
ATOM   2652  N   CYS B 135     -35.438   -6.226 124.560  1.00 27.09           N
ATOM   2653  CA  CYS B 135     -35.688   -7.373 125.423  1.00 30.29           C
ATOM   2654  C   CYS B 135     -35.351   -8.641 124.645  1.00 30.83           C
ATOM   2655  O   CYS B 135     -35.841   -8.828 123.528  1.00 31.86           O
ATOM   2656  CB  CYS B 135     -37.149   -7.402 125.895  1.00 28.77           C
ATOM   2657  SG  CYS B 135     -37.549   -8.569 127.279  1.00 36.90           S
ATOM   2658  N   LEU B 136     -34.537   -9.514 125.236  1.00 28.65           N
ATOM   2659  CA  LEU B 136     -34.113  -10.767 124.610  1.00 26.89           C
ATOM   2660  C   LEU B 136     -34.712  -11.963 125.340  1.00 27.49           C
ATOM   2661  O   LEU B 136     -34.462  -12.158 126.536  1.00 26.81           O
ATOM   2662  CB  LEU B 136     -32.587  -10.874 124.591  1.00 23.26           C
ATOM   2663  CG  LEU B 136     -32.021  -12.242 124.215  1.00 26.55           C
ATOM   2664  CD1 LEU B 136     -32.394  -12.677 122.784  1.00 29.37           C
ATOM   2665  CD2 LEU B 136     -30.539  -12.206 124.383  1.00 26.86           C
ATOM   2666  N   LEU B 137     -35.471  -12.776 124.612  1.00 27.17           N
ATOM   2667  CA  LEU B 137     -36.003  -14.046 125.109  1.00 24.46           C
ATOM   2668  C   LEU B 137     -35.244  -15.151 124.412  1.00 25.24           C
ATOM   2669  O   LEU B 137     -35.476  -15.416 123.232  1.00 27.19           O
ATOM   2670  CB  LEU B 137     -37.498  -14.197 124.847  1.00 24.41           C
ATOM   2671  CG  LEU B 137     -38.513  -13.411 125.671  1.00 26.56           C
ATOM   2672  CD1 LEU B 137     -38.301  -11.899 125.512  1.00 22.36           C
ATOM   2673  CD2 LEU B 137     -39.922  -13.845 125.275  1.00 24.34           C
ATOM   2674  N   ASN B 138     -34.374  -15.820 125.147  1.00 27.07           N
ATOM   2675  CA  ASN B 138     -33.403  -16.731 124.575  1.00 27.89           C
ATOM   2676  C   ASN B 138     -33.800  -18.186 124.822  1.00 28.77           C
ATOM   2677  O   ASN B 138     -34.161  -18.546 125.945  1.00 33.61           O
ATOM   2678  CB  ASN B 138     -32.033  -16.411 125.163  1.00 31.08           C
ATOM   2679  CG  ASN B 138     -30.904  -16.806 124.256  1.00 37.01           C
ATOM   2680  OD1 ASN B 138     -30.870  -16.438 123.076  1.00 34.03           O
ATOM   2681  ND2 ASN B 138     -29.933  -17.509 124.815  1.00 40.66           N
ATOM   2682  N   ASN B 139     -33.800  -18.987 123.747  1.00 26.63           N
ATOM   2683  CA  ASN B 139     -33.861  -20.463 123.722  1.00 26.50           C
ATOM   2684  C   ASN B 139     -35.075  -21.037 124.462  1.00 25.84           C
ATOM   2685  O   ASN B 139     -34.958  -21.778 125.436  1.00 30.08           O
ATOM   2686  CB  ASN B 139     -32.559  -21.104 124.220  1.00 27.61           C
ATOM   2687  CG  ASN B 139     -31.415  -20.897 123.232  1.00 35.63           C
ATOM   2688  OD1 ASN B 139     -31.150  -19.770 122.822  1.00 31.84           O
ATOM   2689  ND2 ASN B 139     -30.785  -21.990 122.787  1.00 33.37           N
ATOM   2690  N   PHE B 140     -36.249  -20.756 123.903  1.00 27.05           N
ATOM   2691  CA  PHE B 140     -37.494  -21.275 124.449  1.00 28.39           C
ATOM   2692  C   PHE B 140     -38.206  -22.147 123.413  1.00 30.25           C
ATOM   2693  O   PHE B 140     -37.990  -22.032 122.203  1.00 30.54           O
ATOM   2694  CB  PHE B 140     -38.430  -20.144 124.905  1.00 25.56           C
ATOM   2695  CG  PHE B 140     -38.791  -19.192 123.813  1.00 27.91           C
```

```
ATOM   2696  CD1 PHE B 140     -37.989 -18.086 123.541  1.00 25.72           C
ATOM   2697  CD2 PHE B 140     -39.922 -19.410 123.030  1.00 27.50           C
ATOM   2698  CE1 PHE B 140     -38.309 -17.202 122.515  1.00 24.13           C
ATOM   2699  CE2 PHE B 140     -40.257 -18.524 122.002  1.00 29.76           C
ATOM   2700  CZ  PHE B 140     -39.438 -17.415 121.744  1.00 27.82           C
ATOM   2701  N   TYR B 141     -39.054 -23.030 123.914  1.00 27.15           N
ATOM   2702  CA  TYR B 141     -39.947 -23.833 123.101  1.00 27.13           C
ATOM   2703  C   TYR B 141     -41.183 -24.086 123.982  1.00 29.40           C
ATOM   2704  O   TYR B 141     -41.048 -24.404 125.162  1.00 28.49           O
ATOM   2705  CB  TYR B 141     -39.278 -25.150 122.634  1.00 27.47           C
ATOM   2706  CG  TYR B 141     -40.171 -25.919 121.693  1.00 28.08           C
ATOM   2707  CD1 TYR B 141     -41.133 -26.780 122.196  1.00 30.33           C
ATOM   2708  CD2 TYR B 141     -40.098 -25.746 120.308  1.00 24.75           C
ATOM   2709  CE1 TYR B 141     -42.002 -27.456 121.359  1.00 30.51           C
ATOM   2710  CE2 TYR B 141     -40.969 -26.402 119.464  1.00 25.25           C
ATOM   2711  CZ  TYR B 141     -41.930 -27.270 120.002  1.00 30.79           C
ATOM   2712  OH  TYR B 141     -42.841 -27.974 119.229  1.00 28.57           O
ATOM   2713  N   PRO B 142     -42.394 -23.975 123.414  1.00 29.92           N
ATOM   2714  CA  PRO B 142     -42.663 -23.735 121.996  1.00 28.29           C
ATOM   2715  C   PRO B 142     -42.638 -22.263 121.586  1.00 29.24           C
ATOM   2716  O   PRO B 142     -42.345 -21.399 122.404  1.00 30.25           O
ATOM   2717  CB  PRO B 142     -44.067 -24.327 121.825  1.00 28.67           C
ATOM   2718  CG  PRO B 142     -44.726 -24.029 123.121  1.00 25.40           C
ATOM   2719  CD  PRO B 142     -43.635 -24.261 124.160  1.00 27.51           C
ATOM   2720  N   ARG B 143     -43.042 -22.022 120.332  1.00 33.09           N
ATOM   2721  CA  ARG B 143     -42.828 -20.754 119.634  1.00 32.76           C
ATOM   2722  C   ARG B 143     -43.567 -19.587 120.289  1.00 33.19           C
ATOM   2723  O   ARG B 143     -43.061 -18.461 120.295  1.00 34.09           O
ATOM   2724  CB  ARG B 143     -43.256 -20.953 118.174  1.00 30.54           C
ATOM   2725  CG  ARG B 143     -43.343 -19.750 117.269  1.00 30.54           C
ATOM   2726  CD  ARG B 143     -42.071 -19.508 116.495  1.00 35.91           C
ATOM   2727  NE  ARG B 143     -42.253 -18.751 115.239  1.00 36.61           N
ATOM   2728  CZ  ARG B 143     -42.878 -17.569 115.145  1.00 37.84           C
ATOM   2729  NH1 ARG B 143     -43.464 -17.009 116.203  1.00 40.08           N1+
ATOM   2730  NH2 ARG B 143     -42.943 -16.947 113.987  1.00 35.58           N
ATOM   2731  N   GLU B 144     -44.743 -19.837 120.856  1.00 31.25           N
ATOM   2732  CA  GLU B 144     -45.607 -18.768 121.346  1.00 35.56           C
ATOM   2733  C   GLU B 144     -45.024 -18.121 122.602  1.00 34.95           C
ATOM   2734  O   GLU B 144     -44.602 -18.808 123.537  1.00 34.88           O
ATOM   2735  CB  GLU B 144     -47.021 -19.299 121.646  1.00 30.65           C
ATOM   2736  CG  GLU B 144     -47.821 -19.817 120.448  1.00 34.84           C
ATOM   2737  CD  GLU B 144     -47.385 -21.206 119.951  1.00 44.49           C
ATOM   2738  OE1 GLU B 144     -47.117 -22.107 120.783  1.00 41.20           O
ATOM   2739  OE2 GLU B 144     -47.333 -21.404 118.716  1.00 47.18           O1-
ATOM   2740  N   ALA B 145     -45.002 -16.795 122.623  1.00 29.26           N
ATOM   2741  CA  ALA B 145     -44.486 -16.077 123.774  1.00 34.47           C
ATOM   2742  C   ALA B 145     -45.102 -14.691 123.756  1.00 33.83           C
ATOM   2743  O   ALA B 145     -45.457 -14.194 122.684  1.00 35.59           O
ATOM   2744  CB  ALA B 145     -42.952 -16.007 123.737  1.00 31.26           C
ATOM   2745  N   LYS B 146     -45.250 -14.077 124.937  1.00 26.49           N
ATOM   2746  CA  LYS B 146     -45.754 -12.707 125.026  1.00 28.76           C
ATOM   2747  C   LYS B 146     -44.766 -11.790 125.756  1.00 32.11           C
ATOM   2748  O   LYS B 146     -44.336 -12.085 126.880  1.00 25.51           O
ATOM   2749  CB  LYS B 146     -47.130 -12.683 125.687  1.00 34.49           C
ATOM   2750  CG  LYS B 146     -47.879 -11.354 125.547  1.00 44.62           C
ATOM   2751  CD  LYS B 146     -49.339 -11.502 126.002  1.00 51.17           C
ATOM   2752  CE  LYS B 146     -50.157 -10.231 125.771  1.00 55.61           C
ATOM   2753  NZ  LYS B 146     -51.627 -10.552 125.678  1.00 51.90           N1+
ATOM   2754  N   VAL B 147     -44.417 -10.672 125.113  1.00 32.22           N
ATOM   2755  CA  VAL B 147     -43.616  -9.609 125.712  1.00 28.21           C
ATOM   2756  C   VAL B 147     -44.534  -8.434 126.015  1.00 34.36           C
ATOM   2757  O   VAL B 147     -45.332  -8.029 125.165  1.00 33.36           O
ATOM   2758  CB  VAL B 147     -42.464  -9.160 124.794  1.00 27.98           C
ATOM   2759  CG1 VAL B 147     -41.768  -7.931 125.372  1.00 29.12           C
ATOM   2760  CG2 VAL B 147     -41.472 -10.275 124.560  1.00 32.19           C
ATOM   2761  N   GLN B 148     -44.444  -7.904 127.230  1.00 35.67           N
ATOM   2762  CA  GLN B 148     -45.128  -6.673 127.593  1.00 36.29           C
ATOM   2763  C   GLN B 148     -44.100  -5.665 128.073  1.00 33.05           C
ATOM   2764  O   GLN B 148     -43.303  -5.968 128.966  1.00 29.76           O
ATOM   2765  CB  GLN B 148     -46.192  -6.913 128.676  1.00 36.98           C
ATOM   2766  CG  GLN B 148     -47.614  -6.800 128.154  1.00 47.87           C
```

```
ATOM   2767  CD   GLN B 148     -48.651  -7.188 129.192  1.00 64.59          C
ATOM   2768  OE1  GLN B 148     -48.420  -7.050 130.398  1.00 64.90          O
ATOM   2769  NE2  GLN B 148     -49.806  -7.674 128.728  1.00 58.09          N
ATOM   2770  N    TRP B 149     -44.099  -4.485 127.465  1.00 34.92          N
ATOM   2771  CA   TRP B 149     -43.234  -3.400 127.918  1.00 35.07          C
ATOM   2772  C    TRP B 149     -43.941  -2.522 128.952  1.00 31.98          C
ATOM   2773  O    TRP B 149     -45.105  -2.154 128.778  1.00 33.16          O
ATOM   2774  CB   TRP B 149     -42.797  -2.549 126.730  1.00 28.25          C
ATOM   2775  CG   TRP B 149     -41.704  -3.158 125.876  1.00 31.59          C
ATOM   2776  CD1  TRP B 149     -41.851  -3.717 124.640  1.00 28.61          C
ATOM   2777  CD2  TRP B 149     -40.305  -3.236 126.186  1.00 28.06          C
ATOM   2778  NE1  TRP B 149     -40.640  -4.134 124.164  1.00 31.98          N
ATOM   2779  CE2  TRP B 149     -39.671  -3.852 125.091  1.00 31.31          C
ATOM   2780  CE3  TRP B 149     -39.529  -2.842 127.280  1.00 32.39          C
ATOM   2781  CZ2  TRP B 149     -38.289  -4.080 125.048  1.00 29.16          C
ATOM   2782  CZ3  TRP B 149     -38.159  -3.066 127.242  1.00 36.30          C
ATOM   2783  CH2  TRP B 149     -37.550  -3.676 126.125  1.00 32.50          C
ATOM   2784  N    LYS B 150     -43.228  -2.174 130.023  1.00 34.33          N
ATOM   2785  CA   LYS B 150     -43.736  -1.240 131.030  1.00 35.21          C
ATOM   2786  C    LYS B 150     -42.711  -0.143 131.257  1.00 36.04          C
ATOM   2787  O    LYS B 150     -41.534  -0.433 131.489  1.00 40.23          O
ATOM   2788  CB   LYS B 150     -44.050  -1.918 132.374  1.00 29.19          C
ATOM   2789  CG   LYS B 150     -45.326  -2.755 132.421  1.00 32.86          C
ATOM   2790  CD   LYS B 150     -45.296  -3.677 133.643  1.00 42.43          C
ATOM   2791  CE   LYS B 150     -46.327  -4.800 133.536  1.00 54.13          C
ATOM   2792  NZ   LYS B 150     -46.598  -5.502 134.839  1.00 48.84          N1+
ATOM   2793  N    VAL B 151     -43.158   1.107 131.189  1.00 34.12          N
ATOM   2794  CA   VAL B 151     -42.355   2.270 131.559  1.00 34.61          C
ATOM   2795  C    VAL B 151     -43.003   2.921 132.784  1.00 36.08          C
ATOM   2796  O    VAL B 151     -44.118   3.448 132.699  1.00 35.70          O
ATOM   2797  CB   VAL B 151     -42.241   3.255 130.392  1.00 31.07          C
ATOM   2798  CG1  VAL B 151     -41.307   4.377 130.732  1.00 31.60          C
ATOM   2799  CG2  VAL B 151     -41.752   2.528 129.173  1.00 37.80          C
ATOM   2800  N    ASP B 152     -42.299   2.902 133.921  1.00 39.23          N
ATOM   2801  CA   ASP B 152     -42.854   3.340 135.217  1.00 38.88          C
ATOM   2802  C    ASP B 152     -44.243   2.743 135.428  1.00 37.74          C
ATOM   2803  O    ASP B 152     -45.194   3.412 135.836  1.00 35.76          O
ATOM   2804  CB   ASP B 152     -42.848   4.863 135.343  1.00 36.31          C
ATOM   2805  CG   ASP B 152     -41.451   5.404 135.628  1.00 44.31          C
ATOM   2806  OD1  ASP B 152     -40.688   4.719 136.357  1.00 39.45          O
ATOM   2807  OD2  ASP B 152     -41.093   6.479 135.094  1.00 46.68          O1-
ATOM   2808  N    ASN B 153     -44.333   1.457 135.097  1.00 38.94          N
ATOM   2809  CA   ASN B 153     -45.491   0.579 135.212  1.00 39.28          C
ATOM   2810  C    ASN B 153     -46.660   0.990 134.321  1.00 37.08          C
ATOM   2811  O    ASN B 153     -47.783   0.508 134.502  1.00 39.74          O
ATOM   2812  CB   ASN B 153     -45.933   0.433 136.666  1.00 37.94          C
ATOM   2813  CG   ASN B 153     -46.502  -0.927 136.937  1.00 41.53          C
ATOM   2814  OD1  ASN B 153     -47.693  -1.071 137.197  1.00 48.49          O
ATOM   2815  ND2  ASN B 153     -45.658  -1.953 136.827  1.00 47.40          N
ATOM   2816  N    ALA B 154     -46.418   1.824 133.322  1.00 27.73          N
ATOM   2817  CA   ALA B 154     -47.403   2.060 132.281  1.00 31.59          C
ATOM   2818  C    ALA B 154     -47.152   1.064 131.145  1.00 35.73          C
ATOM   2819  O    ALA B 154     -46.034   1.006 130.611  1.00 34.76          O
ATOM   2820  CB   ALA B 154     -47.314   3.503 131.785  1.00 30.19          C
ATOM   2821  N    LEU B 155     -48.168   0.246 130.817  1.00 31.02          N
ATOM   2822  CA   LEU B 155     -48.092  -0.680 129.680  1.00 29.47          C
ATOM   2823  C    LEU B 155     -47.940   0.045 128.364  1.00 29.26          C
ATOM   2824  O    LEU B 155     -48.816   0.809 127.967  1.00 32.19          O
ATOM   2825  CB   LEU B 155     -49.321  -1.578 129.581  1.00 26.14          C
ATOM   2826  CG   LEU B 155     -49.137  -2.963 130.188  1.00 41.59          C
ATOM   2827  CD1  LEU B 155     -49.358  -2.997 131.716  1.00 37.40          C
ATOM   2828  CD2  LEU B 155     -49.966  -3.985 129.430  1.00 49.03          C
ATOM   2829  N    GLN B 156     -46.891  -0.293 127.632  1.00 32.37          N
ATOM   2830  CA   GLN B 156     -46.687   0.225 126.289  1.00 31.54          C
ATOM   2831  C    GLN B 156     -47.433  -0.647 125.293  1.00 27.02          C
ATOM   2832  O    GLN B 156     -47.493  -1.864 125.444  1.00 36.94          O
ATOM   2833  CB   GLN B 156     -45.193   0.270 125.966  1.00 31.74          C
ATOM   2834  CG   GLN B 156     -44.395   0.948 127.069  1.00 31.12          C
ATOM   2835  CD   GLN B 156     -44.877   2.358 127.299  1.00 33.29          C
ATOM   2836  OE1  GLN B 156     -44.789   3.213 126.412  1.00 33.92          O
ATOM   2837  NE2  GLN B 156     -45.445   2.599 128.470  1.00 34.06          N
```

```
ATOM   2838  N    SER B 157   -48.069  -0.017 124.322  1.00 25.15        N
ATOM   2839  CA   SER B 157   -48.818  -0.745 123.309  1.00 24.13        C
ATOM   2840  C    SER B 157   -48.684   0.008 121.998  1.00 28.91        C
ATOM   2841  O    SER B 157   -49.132   1.153 121.903  1.00 33.39        O
ATOM   2842  CB   SER B 157   -50.284  -0.869 123.724  1.00 27.59        C
ATOM   2843  OG   SER B 157   -51.056  -1.511 122.738  1.00 34.07        O
ATOM   2844  N    GLY B 158   -48.052  -0.597 121.005  1.00 23.39        N
ATOM   2845  CA   GLY B 158   -47.959   0.017 119.698  1.00 22.69        C
ATOM   2846  C    GLY B 158   -46.630   0.665 119.370  1.00 26.97        C
ATOM   2847  O    GLY B 158   -46.467   1.166 118.256  1.00 30.74        O
ATOM   2848  N    ASN B 159   -45.695   0.721 120.312  1.00 26.14        N
ATOM   2849  CA   ASN B 159   -44.412   1.373 120.086  1.00 25.51        C
ATOM   2850  C    ASN B 159   -43.236   0.401 120.206  1.00 30.63        C
ATOM   2851  O    ASN B 159   -42.149   0.779 120.660  1.00 25.81        O
ATOM   2852  CB   ASN B 159   -44.236   2.542 121.043  1.00 22.42        C
ATOM   2853  CG   ASN B 159   -44.537   2.165 122.463  1.00 29.01        C
ATOM   2854  OD1  ASN B 159   -44.940   1.021 122.757  1.00 30.10        O
ATOM   2855  ND2  ASN B 159   -44.335   3.114 123.370  1.00 32.61        N
ATOM   2856  N    SER B 160   -43.439  -0.859 119.819  1.00 27.65        N
ATOM   2857  CA   SER B 160   -42.360  -1.827 119.814  1.00 29.05        C
ATOM   2858  C    SER B 160   -42.510  -2.753 118.615  1.00 28.89        C
ATOM   2859  O    SER B 160   -43.602  -2.939 118.089  1.00 29.48        O
ATOM   2860  CB   SER B 160   -42.328  -2.626 121.112  1.00 26.29        C
ATOM   2861  OG   SER B 160   -43.447  -3.475 121.181  1.00 31.67        O
ATOM   2862  N    GLN B 161   -41.400  -3.357 118.197  1.00 28.03        N
ATOM   2863  CA   GLN B 161   -41.425  -4.362 117.147  1.00 26.96        C
ATOM   2864  C    GLN B 161   -40.634  -5.596 117.581  1.00 31.51        C
ATOM   2865  O    GLN B 161   -39.759  -5.537 118.451  1.00 30.01        O
ATOM   2866  CB   GLN B 161   -40.871  -3.794 115.850  1.00 27.24        C
ATOM   2867  CG   GLN B 161   -41.747  -2.764 115.183  1.00 27.43        C
ATOM   2868  CD   GLN B 161   -41.064  -2.142 113.976  1.00 35.97        C
ATOM   2869  OE1  GLN B 161   -40.203  -1.267 114.121  1.00 35.92        O
ATOM   2870  NE2  GLN B 161   -41.418  -2.607 112.782  1.00 30.28        N
ATOM   2871  N    GLU B 162   -40.938  -6.720 116.938  1.00 31.79        N
ATOM   2872  CA   GLU B 162   -40.438  -8.034 117.331  1.00 29.63        C
ATOM   2873  C    GLU B 162   -40.040  -8.837 116.104  1.00 30.95        C
ATOM   2874  O    GLU B 162   -40.654  -8.718 115.042  1.00 26.14        O
ATOM   2875  CB   GLU B 162   -41.503  -8.877 117.989  1.00 23.86        C
ATOM   2876  CG   GLU B 162   -41.567  -8.969 119.420  1.00 34.85        C
ATOM   2877  CD   GLU B 162   -42.807  -9.765 119.781  1.00 41.65        C
ATOM   2878  OE1  GLU B 162   -43.430 -10.284 118.835  1.00 38.04        O
ATOM   2879  OE2  GLU B 162   -43.177  -9.858 120.973  1.00 45.93        O1-
ATOM   2880  N    SER B 163   -39.057  -9.712 116.280  1.00 31.61        N
ATOM   2881  CA   SER B 163   -38.794 -10.763 115.312  1.00 25.04        C
ATOM   2882  C    SER B 163   -38.348 -11.995 116.088  1.00 28.10        C
ATOM   2883  O    SER B 163   -37.857 -11.890 117.218  1.00 25.85        O
ATOM   2884  CB   SER B 163   -37.781 -10.326 114.254  1.00 26.93        C
ATOM   2885  OG   SER B 163   -36.461 -10.447 114.714  1.00 29.93        O
ATOM   2886  N    VAL B 164   -38.587 -13.169 115.504  1.00 29.93        N
ATOM   2887  CA   VAL B 164   -38.200 -14.441 116.104  1.00 26.45        C
ATOM   2888  C    VAL B 164   -37.323 -15.214 115.130  1.00 23.48        C
ATOM   2889  O    VAL B 164   -37.520 -15.156 113.918  1.00 28.17        O
ATOM   2890  CB   VAL B 164   -39.430 -15.281 116.521  1.00 30.77        C
ATOM   2891  CG1  VAL B 164   -40.375 -14.468 117.392  1.00 26.66        C
ATOM   2892  CG2  VAL B 164   -40.156 -15.786 115.308  1.00 40.64        C
ATOM   2893  N    THR B 165   -36.328 -15.908 115.663  1.00 28.51        N
ATOM   2894  CA   THR B 165   -35.442 -16.716 114.848  1.00 27.61        C
ATOM   2895  C    THR B 165   -36.154 -17.967 114.344  1.00 29.58        C
ATOM   2896  O    THR B 165   -37.279 -18.300 114.751  1.00 28.80        O
ATOM   2897  CB   THR B 165   -34.195 -17.113 115.641  1.00 28.32        C
ATOM   2898  OG1  THR B 165   -34.575 -17.596 116.939  1.00 33.79        O
ATOM   2899  CG2  THR B 165   -33.275 -15.942 115.795  1.00 25.22        C
ATOM   2900  N    GLU B 166   -35.489 -18.634 113.402  1.00 28.57        N
ATOM   2901  CA   GLU B 166   -35.851 -19.992 113.026  1.00 29.18        C
ATOM   2902  C    GLU B 166   -35.474 -20.953 114.149  1.00 25.81        C
ATOM   2903  O    GLU B 166   -34.705 -20.622 115.046  1.00 28.21        O
ATOM   2904  CB   GLU B 166   -35.158 -20.398 111.722  1.00 25.69        C
ATOM   2905  CG   GLU B 166   -35.615 -19.673 110.472  1.00 23.14        C
ATOM   2906  CD   GLU B 166   -37.112 -19.776 110.263  1.00 35.69        C
ATOM   2907  OE1  GLU B 166   -37.691 -20.801 110.689  1.00 45.01        O
ATOM   2908  OE2  GLU B 166   -37.715 -18.853 109.661  1.00 37.00        O1-
```

433

```
ATOM   2909  N   GLN B 167     -36.061 -22.138 114.120  1.00 29.35           N
ATOM   2910  CA  GLN B 167     -35.749 -23.146 115.126  1.00 27.70           C
ATOM   2911  C   GLN B 167     -34.250 -23.426 115.143  1.00 26.23           C
ATOM   2912  O   GLN B 167     -33.622 -23.530 114.093  1.00 25.94           O
ATOM   2913  CB  GLN B 167     -36.538 -24.406 114.823  1.00 25.14           C
ATOM   2914  CG  GLN B 167     -36.995 -25.171 116.012  1.00 27.67           C
ATOM   2915  CD  GLN B 167     -38.037 -26.211 115.661  1.00 25.13           C
ATOM   2916  OE1 GLN B 167     -38.324 -26.473 114.489  1.00 27.17           O
ATOM   2917  NE2 GLN B 167     -38.591 -26.819 116.672  1.00 23.92           N
ATOM   2918  N   ASP B 168     -33.660 -23.496 116.338  1.00 30.80           N
ATOM   2919  CA  ASP B 168     -32.202 -23.493 116.448  1.00 28.93           C
ATOM   2920  C   ASP B 168     -31.602 -24.806 115.962  1.00 31.78           C
ATOM   2921  O   ASP B 168     -31.981 -25.882 116.433  1.00 30.59           O
ATOM   2922  CB  ASP B 168     -31.771 -23.268 117.895  1.00 31.58           C
ATOM   2923  CG  ASP B 168     -30.269 -23.104 118.028  1.00 34.74           C
ATOM   2924  OD1 ASP B 168     -29.610 -24.086 118.410  1.00 36.21           O1-
ATOM   2925  OD2 ASP B 168     -29.737 -22.026 117.677  1.00 38.15           O
ATOM   2926  N   SER B 169     -30.583 -24.714 115.101  1.00 37.85           N
ATOM   2927  CA  SER B 169     -30.001 -25.906 114.488  1.00 32.86           C
ATOM   2928  C   SER B 169     -29.342 -26.845 115.487  1.00 33.78           C
ATOM   2929  O   SER B 169     -29.068 -27.992 115.126  1.00 37.40           O
ATOM   2930  CB  SER B 169     -28.999 -25.515 113.412  1.00 35.71           C
ATOM   2931  OG  SER B 169     -27.919 -24.817 113.973  1.00 45.05           O
ATOM   2932  N   LYS B 170     -29.105 -26.415 116.725  1.00 35.30           N
ATOM   2933  CA  LYS B 170     -28.484 -27.260 117.742  1.00 32.69           C
ATOM   2934  C   LYS B 170     -29.445 -27.779 118.816  1.00 33.51           C
ATOM   2935  O   LYS B 170     -29.365 -28.957 119.161  1.00 33.92           O
ATOM   2936  CB  LYS B 170     -27.311 -26.510 118.389  1.00 32.22           C
ATOM   2937  CG  LYS B 170     -26.523 -27.313 119.398  1.00 35.45           C
ATOM   2938  CD  LYS B 170     -25.696 -26.399 120.284  1.00 39.33           C
ATOM   2939  CE  LYS B 170     -24.687 -27.150 121.125  1.00 41.85           C
ATOM   2940  NZ  LYS B 170     -23.466 -26.291 121.260  1.00 49.14           N1+
ATOM   2941  N   ASP B 171     -30.386 -26.985 119.360  1.00 29.53           N
ATOM   2942  CA  ASP B 171     -31.298 -27.535 120.366  1.00 24.54           C
ATOM   2943  C   ASP B 171     -32.772 -27.335 120.036  1.00 25.46           C
ATOM   2944  O   ASP B 171     -33.615 -27.519 120.926  1.00 22.30           O
ATOM   2945  CB  ASP B 171     -31.001 -27.011 121.795  1.00 25.26           C
ATOM   2946  CG  ASP B 171     -31.151 -25.471 121.965  1.00 35.81           C
ATOM   2947  OD1 ASP B 171     -31.871 -24.789 121.205  1.00 38.80           O
ATOM   2948  OD2 ASP B 171     -30.555 -24.925 122.927  1.00 42.26           O1-
ATOM   2949  N   SER B 172     -33.096 -26.958 118.791  1.00 24.40           N
ATOM   2950  CA  SER B 172     -34.458 -26.783 118.271  1.00 22.81           C
ATOM   2951  C   SER B 172     -35.296 -25.735 119.023  1.00 29.46           C
ATOM   2952  O   SER B 172     -36.540 -25.777 118.980  1.00 30.46           O
ATOM   2953  CB  SER B 172     -35.204 -28.122 118.240  1.00 27.26           C
ATOM   2954  OG  SER B 172     -34.476 -29.076 117.480  1.00 29.50           O
ATOM   2955  N   THR B 173     -34.685 -24.758 118.688  1.00 26.53           N
ATOM   2956  CA  THR B 173     -35.479 -23.750 120.371  1.00 30.13           C
ATOM   2957  C   THR B 173     -35.667 -22.512 119.487  1.00 28.94           C
ATOM   2958  O   THR B 173     -35.137 -22.397 118.377  1.00 28.80           O
ATOM   2959  CB  THR B 173     -34.869 -23.380 121.742  1.00 29.06           C
ATOM   2960  OG1 THR B 173     -33.536 -22.886 121.592  1.00 27.56           O
ATOM   2961  CG2 THR B 173     -34.849 -24.577 122.671  1.00 23.15           C
ATOM   2962  N   TYR B 174     -36.479 -21.598 119.989  1.00 27.86           N
ATOM   2963  CA  TYR B 174     -36.710 -20.306 119.375  1.00 27.59           C
ATOM   2964  C   TYR B 174     -36.103 -19.210 120.245  1.00 28.01           C
ATOM   2965  O   TYR B 174     -35.906 -19.388 121.452  1.00 28.60           O
ATOM   2966  CB  TYR B 174     -38.210 -20.069 119.181  1.00 27.30           C
ATOM   2967  CG  TYR B 174     -38.818 -20.983 118.147  1.00 30.86           C
ATOM   2968  CD1 TYR B 174     -38.743 -20.672 116.791  1.00 26.78           C
ATOM   2969  CD2 TYR B 174     -39.452 -22.171 118.521  1.00 31.18           C
ATOM   2970  CE1 TYR B 174     -39.283 -21.496 115.842  1.00 27.76           C
ATOM   2971  CE2 TYR B 174     -39.994 -23.016 117.567  1.00 30.49           C
ATOM   2972  CZ  TYR B 174     -39.906 -22.666 116.224  1.00 31.46           C
ATOM   2973  OH  TYR B 174     -40.448 -23.480 115.262  1.00 32.46           O
ATOM   2974  N   SER B 175     -35.773 -18.087 119.621  1.00 26.84           N
ATOM   2975  CA  SER B 175     -35.396 -16.886 120.355  1.00 28.52           C
ATOM   2976  C   SER B 175     -36.172 -15.696 119.814  1.00 28.84           C
ATOM   2977  O   SER B 175     -36.595 -15.687 118.656  1.00 24.93           O
ATOM   2978  CB  SER B 175     -33.904 -16.608 120.274  1.00 31.06           C
ATOM   2979  OG  SER B 175     -33.199 -17.479 121.133  1.00 35.19           O
```

```
ATOM   2980  N    LEU B 176   -36.367 -14.694 120.671  1.00 29.11          N
ATOM   2981  CA   LEU B 176   -37.165 -13.529 120.323  1.00 27.58          C
ATOM   2982  C    LEU B 176   -36.445 -12.262 120.739  1.00 24.37          C
ATOM   2983  O    LEU B 176   -35.796 -12.218 121.779  1.00 27.04          O
ATOM   2984  CB   LEU B 176   -38.553 -13.589 120.973  1.00 29.13          C
ATOM   2985  CG   LEU B 176   -39.558 -12.517 120.560  1.00 30.07          C
ATOM   2986  CD1  LEU B 176   -40.920 -13.129 120.631  1.00 30.39          C
ATOM   2987  CD2  LEU B 176   -39.485 -11.303 121.483  1.00 24.93          C
ATOM   2988  N    SER B 177   -36.556 -11.235 119.905  1.00 29.33          N
ATOM   2989  CA   SER B 177   -35.972  -9.932 120.178  1.00 26.82          C
ATOM   2990  C    SER B 177   -37.041  -8.863 119.992  1.00 27.17          C
ATOM   2991  O    SER B 177   -37.669  -8.809 118.934  1.00 25.25          O
ATOM   2992  CB   SER B 177   -34.782  -9.694 119.255  1.00 26.28          C
ATOM   2993  OG   SER B 177   -34.307  -8.377 119.379  1.00 27.89          O
ATOM   2994  N    SER B 178   -37.232  -8.006 121.013  1.00 25.92          N
ATOM   2995  CA   SER B 178   -38.255  -6.966 121.020  1.00 23.84          C
ATOM   2996  C    SER B 178   -37.624  -5.603 121.261  1.00 27.80          C
ATOM   2997  O    SER B 178   -36.822  -5.443 122.189  1.00 29.43          O
ATOM   2998  CB   SER B 178   -39.308  -7.226 122.096  1.00 28.27          C
ATOM   2999  OG   SER B 178   -40.307  -6.215 122.090  1.00 26.54          O
ATOM   3000  N    THR B 179   -38.007  -4.617 120.444  1.00 26.02          N
ATOM   3001  CA   THR B 179   -37.450  -3.266 120.535  1.00 30.99          C
ATOM   3002  C    THR B 179   -38.536  -2.247 120.809  1.00 25.23          C
ATOM   3003  O    THR B 179   -39.357  -1.967 119.938  1.00 24.43          O
ATOM   3004  CB   THR B 179   -36.714  -2.839 119.274  1.00 31.29          C
ATOM   3005  OG1  THR B 179   -35.681  -3.781 118.962  1.00 35.76          O
ATOM   3006  CG2  THR B 179   -36.121  -1.472 119.512  1.00 23.65          C
ATOM   3007  N    LEU B 180   -38.489  -1.662 121.997  1.00 26.49          N
ATOM   3008  CA   LEU B 180   -39.263  -0.482 122.349  1.00 28.48          C
ATOM   3009  C    LEU B 180   -38.502   0.769 121.939  1.00 24.67          C
ATOM   3010  O    LEU B 180   -37.356   0.949 122.350  1.00 26.90          O
ATOM   3011  CB   LEU B 180   -39.510  -0.482 123.855  1.00 30.84          C
ATOM   3012  CG   LEU B 180   -40.291   0.643 124.498  1.00 34.83          C
ATOM   3013  CD1  LEU B 180   -41.724   0.580 123.976  1.00 34.95          C
ATOM   3014  CD2  LEU B 180   -40.215   0.471 126.015  1.00 31.30          C
ATOM   3015  N    THR B 181   -39.142   1.642 121.161  1.00 25.09          N
ATOM   3016  CA   THR B 181   -38.529   2.892 120.712  1.00 34.09          C
ATOM   3017  C    THR B 181   -39.245   4.090 121.341  1.00 40.80          C
ATOM   3018  O    THR B 181   -40.484   4.142 121.354  1.00 36.26          O
ATOM   3019  CB   THR B 181   -38.548   3.005 119.181  1.00 33.92          C
ATOM   3020  OG1  THR B 181   -37.870   1.881 118.610  1.00 36.57          O
ATOM   3021  CG2  THR B 181   -37.793   4.230 118.744  1.00 36.41          C
ATOM   3022  N    LEU B 182   -38.452   5.031 121.882  1.00 36.61          N
ATOM   3023  CA   LEU B 182   -38.910   6.286 122.483  1.00 33.83          C
ATOM   3024  C    LEU B 182   -38.034   7.434 121.997  1.00 37.62          C
ATOM   3025  O    LEU B 182   -36.923   7.235 121.506  1.00 36.88          O
ATOM   3026  CB   LEU B 182   -38.837   6.287 124.017  1.00 39.93          C
ATOM   3027  CG   LEU B 182   -39.550   5.271 124.901  1.00 43.75          C
ATOM   3028  CD1  LEU B 182   -39.188   5.503 126.364  1.00 42.80          C
ATOM   3029  CD2  LEU B 182   -41.051   5.401 124.698  1.00 48.91          C
ATOM   3030  N    SER B 183   -38.515   8.653 122.183  1.00 40.17          N
ATOM   3031  CA   SER B 183   -37.616   9.784 122.023  1.00 39.39          C
ATOM   3032  C    SER B 183   -36.639   9.844 123.195  1.00 39.08          C
ATOM   3033  O    SER B 183   -36.882   9.278 124.259  1.00 39.26          O
ATOM   3034  CB   SER B 183   -38.401  11.088 121.963  1.00 38.95          C
ATOM   3035  OG   SER B 183   -39.031  11.322 123.210  1.00 35.92          O
ATOM   3036  N    LYS B 184   -35.510  10.530 122.986  1.00 42.26          N
ATOM   3037  CA   LYS B 184   -34.617  10.803 124.109  1.00 40.17          C
ATOM   3038  C    LYS B 184   -35.348  11.566 125.208  1.00 41.28          C
ATOM   3039  O    LYS B 184   -35.138  11.310 126.401  1.00 38.53          O
ATOM   3040  CB   LYS B 184   -33.405  11.605 123.646  1.00 42.87          C
ATOM   3041  CG   LYS B 184   -32.462  11.986 124.806  1.00 52.77          C
ATOM   3042  CD   LYS B 184   -31.228  12.771 124.339  1.00 51.17          C
ATOM   3043  CE   LYS B 184   -30.325  13.153 125.500  1.00 56.17          C
ATOM   3044  NZ   LYS B 184   -30.191  14.632 125.670  1.00 63.96          N1+
ATOM   3045  N    ALA B 185   -36.219  12.500 124.816  1.00 39.51          N
ATOM   3046  CA   ALA B 185   -36.995  13.275 125.780  1.00 38.41          C
ATOM   3047  C    ALA B 185   -37.914  12.389 126.613  1.00 39.82          C
ATOM   3048  O    ALA B 185   -37.897  12.456 127.844  1.00 44.20          O
ATOM   3049  CB   ALA B 185   -37.806  14.346 125.057  1.00 33.82          C
ATOM   3050  N    ASP B 186   -38.764  11.591 125.959  1.00 42.91          N
```

```
ATOM   3051  CA   ASP B 186   -39.646  10.690 126.697  1.00 37.37       C
ATOM   3052  C    ASP B 186   -38.844   9.750 127.573  1.00 37.99       C
ATOM   3053  O    ASP B 186   -39.192   9.506 128.736  1.00 37.79       O
ATOM   3054  CB   ASP B 186   -40.512   9.876 125.739  1.00 42.26       C
ATOM   3055  CG   ASP B 186   -41.589  10.703 125.065  1.00 48.82       C
ATOM   3056  OD1  ASP B 186   -42.264  11.496 125.752  1.00 49.68       O
ATOM   3057  OD2  ASP B 186   -41.778  10.531 123.839  1.00 61.13       O1-
ATOM   3058  N    TYR B 187   -37.781   9.181 127.006  1.00 41.31       N
ATOM   3059  CA   TYR B 187   -36.932   8.262 127.745  1.00 37.24       C
ATOM   3060  C    TYR B 187   -36.398   8.907 129.016  1.00 35.05       C
ATOM   3061  O    TYR B 187   -36.349   8.272 130.075  1.00 36.76       O
ATOM   3062  CB   TYR B 187   -35.786   7.785 126.852  1.00 37.33       C
ATOM   3063  CG   TYR B 187   -34.823   6.915 127.604  1.00 37.18       C
ATOM   3064  CD1  TYR B 187   -35.223   5.663 128.068  1.00 34.09       C
ATOM   3065  CD2  TYR B 187   -33.539   7.359 127.900  1.00 27.91       C
ATOM   3066  CE1  TYR B 187   -34.363   4.870 128.780  1.00 33.76       C
ATOM   3067  CE2  TYR B 187   -32.678   6.577 128.618  1.00 30.33       C
ATOM   3068  CZ   TYR B 187   -33.090   5.328 129.058  1.00 31.48       C
ATOM   3069  OH   TYR B 187   -32.233   4.528 129.785  1.00 31.99       O
ATOM   3070  N    GLU B 188   -35.991  10.166 128.935  1.00 39.77       N
ATOM   3071  CA   GLU B 188   -35.344  10.782 130.083  1.00 44.77       C
ATOM   3072  C    GLU B 188   -36.325  11.334 131.097  1.00 40.36       C
ATOM   3073  O    GLU B 188   -35.879  11.832 132.131  1.00 41.81       O
ATOM   3074  CB   GLU B 188   -34.358  11.849 129.625  1.00 39.20       C
ATOM   3075  CG   GLU B 188   -32.978  11.224 129.620  1.00 45.15       C
ATOM   3076  CD   GLU B 188   -31.974  11.914 128.723  1.00 56.05       C
ATOM   3077  OE1  GLU B 188   -32.317  12.924 128.049  1.00 48.75       O
ATOM   3078  OE2  GLU B 188   -30.831  11.394 128.688  1.00 59.27       O1-
ATOM   3079  N    LYS B 189   -37.630  11.197 130.852  1.00 37.43       N
ATOM   3080  CA   LYS B 189   -38.687  11.560 131.788  1.00 38.07       C
ATOM   3081  C    LYS B 189   -39.236  10.363 132.579  1.00 40.62       C
ATOM   3082  O    LYS B 189   -40.315  10.465 133.167  1.00 46.60       O
ATOM   3083  CB   LYS B 189   -39.813  12.270 131.030  1.00 37.89       C
ATOM   3084  CG   LYS B 189   -39.327  13.568 130.380  1.00 51.23       C
ATOM   3085  CD   LYS B 189   -40.333  14.215 129.404  1.00 62.14       C
ATOM   3086  CE   LYS B 189   -39.717  15.470 128.720  1.00 60.71       C
ATOM   3087  NZ   LYS B 189   -40.587  16.123 127.684  1.00 51.31       N1+
ATOM   3088  N    HIS B 190   -38.541   9.223 132.588  1.00 41.83       N
ATOM   3089  CA   HIS B 190   -39.025   8.026 133.271  1.00 35.94       C
ATOM   3090  C    HIS B 190   -37.865   7.259 133.879  1.00 35.72       C
ATOM   3091  O    HIS B 190   -36.701   7.484 133.543  1.00 36.41       O
ATOM   3092  CB   HIS B 190   -39.810   7.120 132.337  1.00 37.87       C
ATOM   3093  CG   HIS B 190   -41.069   7.745 131.840  1.00 37.33       C
ATOM   3094  ND1  HIS B 190   -41.173   8.323 130.596  1.00 40.44       N
ATOM   3095  CD2  HIS B 190   -42.264   7.926 132.442  1.00 37.28       C
ATOM   3096  CE1  HIS B 190   -42.386   8.813 130.442  1.00 36.52       C
ATOM   3097  NE2  HIS B 190   -43.068   8.583 131.548  1.00 42.61       N
ATOM   3098  N    LYS B 191   -38.188   6.365 134.806  1.00 32.95       N
ATOM   3099  CA   LYS B 191   -37.139   5.707 135.580  1.00 44.01       C
ATOM   3100  C    LYS B 191   -37.065   4.204 135.352  1.00 37.37       C
ATOM   3101  O    LYS B 191   -35.988   3.687 135.032  1.00 37.81       O
ATOM   3102  CB   LYS B 191   -37.316   6.017 137.084  1.00 41.79       C
ATOM   3103  CG   LYS B 191   -36.346   5.312 138.042  1.00 43.26       C
ATOM   3104  CD   LYS B 191   -36.731   5.623 139.511  1.00 54.07       C
ATOM   3105  CE   LYS B 191   -35.668   5.177 140.515  1.00 56.60       C
ATOM   3106  NZ   LYS B 191   -36.031   5.546 141.905  1.00 51.73       N1+
ATOM   3107  N    VAL B 192   -38.164   3.482 135.530  1.00 31.57       N
ATOM   3108  CA   VAL B 192   -38.156   2.027 135.503  1.00 34.89       C
ATOM   3109  C    VAL B 192   -38.596   1.564 134.126  1.00 35.04       C
ATOM   3110  O    VAL B 192   -39.687   1.918 133.661  1.00 35.31       O
ATOM   3111  CB   VAL B 192   -39.054   1.439 136.599  1.00 34.98       C
ATOM   3112  CG1  VAL B 192   -39.122  -0.073 136.464  1.00 30.15       C
ATOM   3113  CG2  VAL B 192   -38.522   1.842 137.965  1.00 36.73       C
ATOM   3114  N    TYR B 193   -37.737   0.785 133.473  1.00 36.15       N
ATOM   3115  CA   TYR B 193   -38.019   0.172 132.181  1.00 35.85       C
ATOM   3116  C    TYR B 193   -38.078  -1.327 132.381  1.00 29.50       C
ATOM   3117  O    TYR B 193   -37.178  -1.898 132.995  1.00 31.86       O
ATOM   3118  CB   TYR B 193   -36.958   0.576 131.150  1.00 28.31       C
ATOM   3119  CG   TYR B 193   -37.137   2.020 130.781  1.00 32.21       C
ATOM   3120  CD1  TYR B 193   -36.656   3.034 131.613  1.00 34.12       C
ATOM   3121  CD2  TYR B 193   -37.831   2.384 129.628  1.00 32.20       C
```

```
ATOM   3122  CE1 TYR B 193     -36.859    4.366 131.313  1.00 31.90           C
ATOM   3123  CE2 TYR B 193     -38.023    3.724 129.299  1.00 38.14           C
ATOM   3124  CZ  TYR B 193     -37.536    4.707 130.163  1.00 37.14           C
ATOM   3125  OH  TYR B 193     -37.717    6.025 129.872  1.00 39.52           O
ATOM   3126  N   ALA B 194     -39.149   -1.954 131.908  1.00 28.74           N
ATOM   3127  CA  ALA B 194     -39.300   -3.384 132.148  1.00 34.71           C
ATOM   3128  C   ALA B 194     -39.970   -4.081 130.963  1.00 33.72           C
ATOM   3129  O   ALA B 194     -40.748   -3.479 130.211  1.00 34.66           O
ATOM   3130  CB  ALA B 194     -40.083   -3.634 133.442  1.00 29.91           C
ATOM   3131  N   CYS B 195     -39.623   -5.349 130.776  1.00 32.36           N
ATOM   3132  CA  CYS B 195     -40.382   -6.251 129.919  1.00 37.46           C
ATOM   3133  C   CYS B 195     -40.814   -7.461 130.732  1.00 40.08           C
ATOM   3134  O   CYS B 195     -40.045   -8.000 131.544  1.00 39.11           O
ATOM   3135  CB  CYS B 195     -39.615   -6.703 128.639  1.00 33.36           C
ATOM   3136  SG  CYS B 195     -37.984   -7.394 128.903  1.00 49.70           S
ATOM   3137  N   GLU B 196     -42.077   -7.822 130.550  1.00 36.65           N
ATOM   3138  CA  GLU B 196     -42.702   -8.939 131.228  1.00 37.99           C
ATOM   3139  C   GLU B 196     -42.961  -10.009 130.181  1.00 33.89           C
ATOM   3140  O   GLU B 196     -43.502   -9.711 129.111  1.00 30.27           O
ATOM   3141  CB  GLU B 196     -43.986   -8.481 131.926  1.00 37.34           C
ATOM   3142  CG  GLU B 196     -44.748   -9.548 132.707  1.00 42.18           C
ATOM   3143  CD  GLU B 196     -46.068   -9.008 133.278  1.00 51.19           C
ATOM   3144  OE1 GLU B 196     -46.538   -7.957 132.796  1.00 55.09           O
ATOM   3145  OE2 GLU B 196     -46.618   -9.605 134.234  1.00 54.09           O1-
ATOM   3146  N   VAL B 197     -42.549  -11.236 130.491  1.00 33.59           N
ATOM   3147  CA  VAL B 197     -42.499  -12.350 129.550  1.00 28.73           C
ATOM   3148  C   VAL B 197     -43.396  -13.467 130.057  1.00 31.19           C
ATOM   3149  O   VAL B 197     -43.137  -14.053 131.117  1.00 32.63           O
ATOM   3150  CB  VAL B 197     -41.057  -12.851 129.355  1.00 29.79           C
ATOM   3151  CG1 VAL B 197     -41.040  -14.182 128.614  1.00 27.12           C
ATOM   3152  CG2 VAL B 197     -40.238  -11.808 128.589  1.00 28.85           C
ATOM   3153  N   THR B 198     -44.436  -13.778 129.293  1.00 32.91           N
ATOM   3154  CA  THR B 198     -45.297  -14.926 129.549  1.00 29.91           C
ATOM   3155  C   THR B 198     -45.009  -16.037 128.547  1.00 32.14           C
ATOM   3156  O   THR B 198     -45.017  -15.808 127.328  1.00 29.74           O
ATOM   3157  CB  THR B 198     -46.763  -14.516 129.502  1.00 29.14           C
ATOM   3158  OG1 THR B 198     -46.977  -13.539 130.518  1.00 36.91           O
ATOM   3159  CG2 THR B 198     -47.664  -15.701 129.771  1.00 31.29           C
ATOM   3160  N   HIS B 199     -44.758  -17.237 129.066  1.00 29.27           N
ATOM   3161  CA  HIS B 199     -44.464  -18.393 128.233  1.00 32.48           C
ATOM   3162  C   HIS B 199     -44.929  -19.638 128.964  1.00 34.28           C
ATOM   3163  O   HIS B 199     -44.998  -19.645 130.194  1.00 35.68           O
ATOM   3164  CB  HIS B 199     -42.975  -18.520 127.932  1.00 28.76           C
ATOM   3165  CG  HIS B 199     -42.659  -19.599 126.950  1.00 31.19           C
ATOM   3166  ND1 HIS B 199     -42.284  -20.867 127.331  1.00 29.79           N
ATOM   3167  CD2 HIS B 199     -42.657  -19.594 125.595  1.00 31.07           C
ATOM   3168  CE1 HIS B 199     -42.066  -21.596 126.252  1.00 29.75           C
ATOM   3169  NE2 HIS B 199     -42.280  -20.845 125.185  1.00 26.47           N
ATOM   3170  N   GLN B 200     -45.239  -20.693 128.204  1.00 28.40           N
ATOM   3171  CA  GLN B 200     -45.833  -21.862 128.839  1.00 28.53           C
ATOM   3172  C   GLN B 200     -44.841  -22.620 129.706  1.00 34.07           C
ATOM   3173  O   GLN B 200     -45.270  -23.425 130.535  1.00 36.20           O
ATOM   3174  CB  GLN B 200     -46.471  -22.791 127.805  1.00 34.90           C
ATOM   3175  CG  GLN B 200     -45.638  -23.987 127.362  1.00 36.70           C
ATOM   3176  CD  GLN B 200     -46.492  -25.092 126.712  1.00 43.78           C
ATOM   3177  OE1 GLN B 200     -46.443  -26.257 127.124  1.00 43.09           O
ATOM   3178  NE2 GLN B 200     -47.268  -24.723 125.692  1.00 41.04           N
ATOM   3179  N   GLY B 201     -43.538  -22.378 129.558  1.00 35.51           N
ATOM   3180  CA  GLY B 201     -42.576  -22.968 130.464  1.00 29.44           C
ATOM   3181  C   GLY B 201     -42.338  -22.163 131.720  1.00 33.22           C
ATOM   3182  O   GLY B 201     -41.524  -22.553 132.563  1.00 33.63           O
ATOM   3183  N   LEU B 202     -43.062  -21.058 131.884  1.00 32.15           N
ATOM   3184  CA  LEU B 202     -42.986  -20.209 133.061  1.00 36.14           C
ATOM   3185  C   LEU B 202     -44.298  -20.336 133.808  1.00 37.68           C
ATOM   3186  O   LEU B 202     -45.352  -20.003 133.256  1.00 37.45           O
ATOM   3187  CB  LEU B 202     -42.765  -18.743 132.669  1.00 36.59           C
ATOM   3188  CG  LEU B 202     -41.529  -18.334 131.867  1.00 27.10           C
ATOM   3189  CD1 LEU B 202     -41.560  -16.866 131.516  1.00 29.06           C
ATOM   3190  CD2 LEU B 202     -40.313  -18.626 132.669  1.00 31.65           C
ATOM   3191  N   SER B 203     -44.227  -20.741 135.082  1.00 42.21           N
ATOM   3192  CA  SER B 203     -45.444  -20.866 135.880  1.00 41.93           C
```

```
ATOM   3193  C    SER B 203    -46.098 -19.513 136.127  1.00 42.43           C
ATOM   3194  O    SER B 203    -47.312 -19.458 136.334  1.00 41.82           O
ATOM   3195  CB   SER B 203    -45.145 -21.561 137.214  1.00 44.30           C
ATOM   3196  OG   SER B 203    -44.114 -20.886 137.919  1.00 57.08           O
ATOM   3197  N    SER B 204    -45.333 -18.425 136.065  1.00 41.83           N
ATOM   3198  CA   SER B 204    -45.886 -17.077 136.094  1.00 43.80           C
ATOM   3199  C    SER B 204    -44.928 -16.136 135.369  1.00 41.32           C
ATOM   3200  O    SER B 204    -43.777 -16.501 135.094  1.00 39.18           O
ATOM   3201  CB   SER B 204    -46.175 -16.616 137.537  1.00 37.72           C
ATOM   3202  OG   SER B 204    -45.011 -16.540 138.333  1.00 37.17           O
ATOM   3203  N    PRO B 205    -45.400 -14.953 134.971  1.00 34.11           N
ATOM   3204  CA   PRO B 205    -44.574 -14.046 134.171  1.00 33.10           C
ATOM   3205  C    PRO B 205    -43.256 -13.665 134.836  1.00 39.09           C
ATOM   3206  O    PRO B 205    -43.148 -13.550 136.053  1.00 45.40           O
ATOM   3207  CB   PRO B 205    -45.475 -12.821 133.999  1.00 37.31           C
ATOM   3208  CG   PRO B 205    -46.835 -13.372 134.001  1.00 39.03           C
ATOM   3209  CD   PRO B 205    -46.817 -14.553 134.944  1.00 38.32           C
ATOM   3210  N    VAL B 206    -42.232 -13.502 134.016  1.00 34.71           N
ATOM   3211  CA   VAL B 206    -40.922 -13.075 134.467  1.00 35.91           C
ATOM   3212  C    VAL B 206    -40.692 -11.645 133.984  1.00 38.85           C
ATOM   3213  O    VAL B 206    -40.938 -11.328 132.811  1.00 36.12           O
ATOM   3214  CB   VAL B 206    -39.836 -14.036 133.951  1.00 34.83           C
ATOM   3215  CG1  VAL B 206    -38.475 -13.474 134.183  1.00 32.65           C
ATOM   3216  CG2  VAL B 206    -39.970 -15.355 134.650  1.00 35.57           C
ATOM   3217  N    THR B 207    -40.236 -10.774 134.883  1.00 38.08           N
ATOM   3218  CA   THR B 207    -39.870  -9.416 134.504  1.00 40.84           C
ATOM   3219  C    THR B 207    -38.378  -9.204 134.736  1.00 39.54           C
ATOM   3220  O    THR B 207    -37.836  -9.618 135.761  1.00 42.64           O
ATOM   3221  CB   THR B 207    -40.678  -8.361 135.276  1.00 37.84           C
ATOM   3222  OG1  THR B 207    -42.077  -8.538 135.017  1.00 43.59           O
ATOM   3223  CG2  THR B 207    -40.291  -6.972 134.837  1.00 32.49           C
ATOM   3224  N    LYS B 208    -37.715  -8.590 133.762  1.00 38.06           N
ATOM   3225  CA   LYS B 208    -36.376  -8.048 133.928  1.00 36.13           C
ATOM   3226  C    LYS B 208    -36.503  -6.553 133.717  1.00 30.77           C
ATOM   3227  O    LYS B 208    -37.237  -6.108 132.840  1.00 34.40           O
ATOM   3228  CB   LYS B 208    -35.349  -8.617 132.922  1.00 32.55           C
ATOM   3229  CG   LYS B 208    -35.155 -10.137 132.891  1.00 33.52           C
ATOM   3230  CD   LYS B 208    -34.907 -10.808 134.236  1.00 35.71           C
ATOM   3231  CE   LYS B 208    -34.562 -12.283 133.994  1.00 39.12           C
ATOM   3232  NZ   LYS B 208    -34.393 -13.134 135.206  1.00 41.39           N1+
ATOM   3233  N    SER B 209    -35.841  -5.768 134.543  1.00 37.37           N
ATOM   3234  CA   SER B 209    -36.013  -4.330 134.455  1.00 33.94           C
ATOM   3235  C    SER B 209    -34.700  -3.653 134.776  1.00 29.43           C
ATOM   3236  O    SER B 209    -33.780  -4.271 135.305  1.00 31.80           O
ATOM   3237  CB   SER B 209    -37.096  -3.848 135.420  1.00 33.21           C
ATOM   3238  OG   SER B 209    -36.754  -4.262 136.724  1.00 35.51           O
ATOM   3239  N    PHE B 210    -34.653  -2.351 134.517  1.00 34.31           N
ATOM   3240  CA   PHE B 210    -33.559  -1.507 134.978  1.00 37.85           C
ATOM   3241  C    PHE B 210    -34.108  -0.121 135.302  1.00 39.37           C
ATOM   3242  O    PHE B 210    -35.098   0.319 134.705  1.00 33.58           O
ATOM   3243  CB   PHE B 210    -32.434  -1.395 133.935  1.00 26.33           C
ATOM   3244  CG   PHE B 210    -32.827  -0.647 132.698  1.00 33.39           C
ATOM   3245  CD1  PHE B 210    -32.688   0.736 132.632  1.00 31.43           C
ATOM   3246  CD2  PHE B 210    -33.342  -1.321 131.597  1.00 32.75           C
ATOM   3247  CE1  PHE B 210    -33.052   1.438 131.490  1.00 35.66           C
ATOM   3248  CE2  PHE B 210    -33.702  -0.630 130.436  1.00 33.47           C
ATOM   3249  CZ   PHE B 210    -33.559   0.752 130.380  1.00 34.55           C
ATOM   3250  N    ASN B 211    -33.475   0.547 136.284  1.00 42.75           N
ATOM   3251  CA   ASN B 211    -33.738   1.955 136.583  1.00 38.70           C
ATOM   3252  C    ASN B 211    -32.818   2.832 135.752  1.00 43.04           C
ATOM   3253  O    ASN B 211    -31.602   2.610 135.717  1.00 39.58           O
ATOM   3254  CB   ASN B 211    -33.515   2.295 138.054  1.00 48.54           C
ATOM   3255  CG   ASN B 211    -34.216   1.354 138.988  1.00 51.99           C
ATOM   3256  OD1  ASN B 211    -35.237   0.765 138.642  1.00 48.81           O
ATOM   3257  ND2  ASN B 211    -33.672   1.208 140.193  1.00 53.46           N
ATOM   3258  N    ARG B 212    -33.396   3.826 135.088  1.00 42.30           N
ATOM   3259  CA   ARG B 212    -32.585   4.725 134.279  1.00 44.00           C
ATOM   3260  C    ARG B 212    -31.547   5.416 135.166  1.00 53.42           C
ATOM   3261  O    ARG B 212    -31.838   5.807 136.304  1.00 49.38           O
ATOM   3262  CB   ARG B 212    -33.478   5.748 133.579  1.00 35.08           C
ATOM   3263  CG   ARG B 212    -32.768   6.567 132.543  1.00 35.57           C
```

```
ATOM   3264  CD   ARG B 212    -33.745   7.458 131.855  1.00 39.63           C
ATOM   3265  NE   ARG B 212    -34.383   8.298 132.850  1.00 48.56           N
ATOM   3266  CZ   ARG B 212    -33.927   9.489 133.210  1.00 47.89           C
ATOM   3267  NH1  ARG B 212    -32.853   9.988 132.617  1.00 49.63           N1+
ATOM   3268  NH2  ARG B 212    -34.554  10.182 134.150  1.00 49.80           N
ATOM   3269  N    GLY B 213    -30.313   5.513 134.660  1.00 57.27           N
ATOM   3270  CA   GLY B 213    -29.233   6.174 135.372  1.00 56.62           C
ATOM   3271  C    GLY B 213    -28.594   5.389 136.502  1.00 64.15           C
ATOM   3272  O    GLY B 213    -27.852   5.979 137.297  1.00 72.50           O
ATOM   3273  N    GLU B 214    -28.872   4.093 136.624  1.00 62.54           N
ATOM   3274  CA   GLU B 214    -28.300   3.285 137.703  1.00 62.11           C
ATOM   3275  C    GLU B 214    -27.713   1.996 137.157  1.00 59.03           C
ATOM   3276  O    GLU B 214    -26.518   1.940 136.863  1.00 63.86           O
ATOM   3277  CB   GLU B 214    -29.353   3.001 138.781  1.00 51.33           C
ATOM   3278  CG   GLU B 214    -29.912   4.302 139.342  1.00 61.22           C
ATOM   3279  CD   GLU B 214    -31.228   4.170 140.105  1.00 66.11           C
ATOM   3280  OE1  GLU B 214    -31.496   3.123 140.751  1.00 61.27           O
ATOM   3281  OE2  GLU B 214    -32.004   5.152 140.044  1.00 64.67           O1-
TER
ATOM   3282  N    GLN C   1    -37.672 -36.358  87.095  1.00 48.43           N
ATOM   3283  CA   GLN C   1    -37.796 -35.842  85.733  1.00 53.92           C
ATOM   3284  C    GLN C   1    -36.907 -36.762  84.921  1.00 48.53           C
ATOM   3285  O    GLN C   1    -37.375 -37.531  84.085  1.00 49.71           O
ATOM   3286  CB   GLN C   1    -37.335 -34.383  85.628  1.00 47.24           C
ATOM   3287  CG   GLN C   1    -37.925 -33.494  86.693  1.00 41.90           C
ATOM   3288  CD   GLN C   1    -37.270 -33.771  88.072  1.00 54.37           C
ATOM   3289  OE1  GLN C   1    -37.245 -34.914  88.549  1.00 44.69           O
ATOM   3290  NE2  GLN C   1    -36.710 -32.731  88.690  1.00 59.79           N
ATOM   3291  N    VAL C   2    -35.614 -36.686  85.222  1.00 51.69           N
ATOM   3292  CA   VAL C   2    -34.607 -37.545  84.617  1.00 47.55           C
ATOM   3293  C    VAL C   2    -34.423 -38.731  85.545  1.00 47.63           C
ATOM   3294  O    VAL C   2    -33.898 -38.581  86.647  1.00 46.25           O
ATOM   3295  CB   VAL C   2    -33.299 -36.782  84.412  1.00 42.21           C
ATOM   3296  CG1  VAL C   2    -32.374 -37.549  83.497  1.00 38.77           C
ATOM   3297  CG2  VAL C   2    -33.625 -35.409  83.861  1.00 38.63           C
ATOM   3298  N    GLN C   3    -34.870 -39.908  85.122  1.00 44.76           N
ATOM   3299  CA   GLN C   3    -34.623 -41.114  85.894  1.00 46.20           C
ATOM   3300  C    GLN C   3    -33.571 -41.955  85.194  1.00 38.10           C
ATOM   3301  O    GLN C   3    -33.475 -41.955  83.965  1.00 39.47           O
ATOM   3302  CB   GLN C   3    -35.902 -41.931  86.152  1.00 46.17           C
ATOM   3303  CG   GLN C   3    -36.857 -42.057  84.981  1.00 54.72           C
ATOM   3304  CD   GLN C   3    -37.939 -40.974  84.954  1.00 59.64           C
ATOM   3305  OE1  GLN C   3    -38.022 -40.180  83.996  1.00 55.09           O
ATOM   3306  NE2  GLN C   3    -38.794 -40.952  85.996  1.00 49.26           N
ATOM   3307  N    LEU C   4    -32.728 -42.584  85.993  1.00 36.10           N
ATOM   3308  CA   LEU C   4    -31.834 -43.639  85.548  1.00 36.24           C
ATOM   3309  C    LEU C   4    -32.349 -44.892  86.234  1.00 38.70           C
ATOM   3310  O    LEU C   4    -32.006 -45.155  87.383  1.00 41.03           O
ATOM   3311  CB   LEU C   4    -30.377 -43.351  85.916  1.00 31.18           C
ATOM   3312  CG   LEU C   4    -29.695 -42.051  85.479  1.00 32.11           C
ATOM   3313  CD1  LEU C   4    -28.199 -42.201  85.552  1.00 34.48           C
ATOM   3314  CD2  LEU C   4    -30.085 -41.672  84.084  1.00 36.90           C
ATOM   3315  N    GLN C   5    -33.149 -45.677  85.525  1.00 44.15           N
ATOM   3316  CA   GLN C   5    -33.773 -46.856  86.108  1.00 45.12           C
ATOM   3317  C    GLN C   5    -32.880 -48.063  85.887  1.00 41.78           C
ATOM   3318  O    GLN C   5    -32.475 -48.340  84.755  1.00 41.54           O
ATOM   3319  CB   GLN C   5    -35.152 -47.084  85.489  1.00 49.54           C
ATOM   3320  CG   GLN C   5    -35.943 -48.256  86.071  1.00 56.47           C
ATOM   3321  CD   GLN C   5    -37.440 -48.116  85.797  1.00 74.65           C
ATOM   3322  OE1  GLN C   5    -37.935 -47.009  85.533  1.00 74.08           O
ATOM   3323  NE2  GLN C   5    -38.166 -49.236  85.850  1.00 70.76           N
ATOM   3324  N    GLN C   6    -32.592 -48.786  86.963  1.00 36.00           N
ATOM   3325  CA   GLN C   6    -31.668 -49.905  86.917  1.00 37.25           C
ATOM   3326  C    GLN C   6    -32.432 -51.207  87.049  1.00 40.30           C
ATOM   3327  O    GLN C   6    -33.416 -51.281  87.787  1.00 48.42           O
ATOM   3328  CB   GLN C   6    -30.617 -49.841  88.032  1.00 37.59           C
ATOM   3329  CG   GLN C   6    -29.875 -48.541  88.142  1.00 40.04           C
ATOM   3330  CD   GLN C   6    -28.646 -48.621  89.041  1.00 38.65           C
ATOM   3331  OE1  GLN C   6    -28.173 -47.599  89.535  1.00 36.68           O
ATOM   3332  NE2  GLN C   6    -28.111 -49.834  89.239  1.00 31.43           N
ATOM   3333  N    TRP C   7    -31.969 -52.228  86.334  1.00 36.46           N
```

439

```
ATOM   3334  CA   TRP C   7      -32.424 -53.585  86.573  1.00 39.72           C
ATOM   3335  C    TRP C   7      -31.284 -54.527  86.258  1.00 37.13           C
ATOM   3336  O    TRP C   7      -30.252 -54.125  85.709  1.00 36.52           O
ATOM   3337  CB   TRP C   7      -33.669 -53.937  85.756  1.00 36.24           C
ATOM   3338  CG   TRP C   7      -33.515 -53.880  84.294  1.00 38.80           C
ATOM   3339  CD1  TRP C   7      -33.103 -54.893  83.464  1.00 37.72           C
ATOM   3340  CD2  TRP C   7      -33.812 -52.760  83.446  1.00 41.40           C
ATOM   3341  NE1  TRP C   7      -33.111 -54.462  82.153  1.00 39.38           N
ATOM   3342  CE2  TRP C   7      -33.544 -53.159  82.113  1.00 39.28           C
ATOM   3343  CE3  TRP C   7      -34.270 -51.460  83.683  1.00 41.24           C
ATOM   3344  CZ2  TRP C   7      -33.713 -52.298  81.022  1.00 37.17           C
ATOM   3345  CZ3  TRP C   7      -34.443 -50.603  82.589  1.00 42.48           C
ATOM   3346  CH2  TRP C   7      -34.164 -51.032  81.278  1.00 38.07           C
ATOM   3347  N    GLY C   8      -31.479 -55.779  86.640  1.00 30.53           N
ATOM   3348  CA   GLY C   8      -30.480 -56.819  86.491  1.00 29.00           C
ATOM   3349  C    GLY C   8      -30.542 -57.704  87.715  1.00 28.80           C
ATOM   3350  O    GLY C   8      -30.936 -57.284  88.802  1.00 35.52           O
ATOM   3351  N    ALA C   9      -30.175 -58.964  87.536  1.00 31.95           N
ATOM   3352  CA   ALA C   9      -30.163 -59.902  88.650  1.00 36.64           C
ATOM   3353  C    ALA C   9      -29.108 -59.490  89.672  1.00 40.72           C
ATOM   3354  O    ALA C   9      -27.936 -59.312  89.329  1.00 44.07           O
ATOM   3355  CB   ALA C   9      -29.893 -61.324  88.152  1.00 32.88           C
ATOM   3356  N    GLY C   10     -29.528 -59.350  90.927  1.00 40.53           N
ATOM   3357  CA   GLY C   10     -28.651 -58.938  92.001  1.00 34.92           C
ATOM   3358  C    GLY C   10     -28.135 -60.045  92.894  1.00 32.59           C
ATOM   3359  O    GLY C   10     -27.259 -59.787  93.722  1.00 37.26           O
ATOM   3360  N    LEU C   11     -28.643 -61.269  92.760  1.00 33.32           N
ATOM   3361  CA   LEU C   11     -28.155 -62.401  93.550  1.00 35.63           C
ATOM   3362  C    LEU C   11     -27.388 -63.330  92.625  1.00 37.18           C
ATOM   3363  O    LEU C   11     -27.945 -63.832  91.641  1.00 40.37           O
ATOM   3364  CB   LEU C   11     -29.292 -63.162  94.238  1.00 33.21           C
ATOM   3365  CG   LEU C   11     -29.045 -63.745  95.639  1.00 35.68           C
ATOM   3366  CD1  LEU C   11     -29.828 -65.026  95.836  1.00 37.14           C
ATOM   3367  CD2  LEU C   11     -27.589 -63.991  95.974  1.00 34.37           C
ATOM   3368  N    LEU C   12     -26.125 -63.573  92.957  1.00 33.96           N
ATOM   3369  CA   LEU C   12     -25.238 -64.365  92.126  1.00 37.97           C
ATOM   3370  C    LEU C   12     -24.363 -65.240  93.002  1.00 35.65           C
ATOM   3371  O    LEU C   12     -24.068 -64.894  94.147  1.00 36.30           O
ATOM   3372  CB   LEU C   12     -24.331 -63.494  91.246  1.00 34.37           C
ATOM   3373  CG   LEU C   12     -24.960 -62.530  90.262  1.00 37.66           C
ATOM   3374  CD1  LEU C   12     -23.856 -61.733  89.638  1.00 43.23           C
ATOM   3375  CD2  LEU C   12     -25.713 -63.294  89.190  1.00 44.30           C
ATOM   3376  N    LYS C   13     -23.932 -66.390  92.428  1.00 36.66           N
ATOM   3377  CA   LYS C   13     -22.957 -67.321  92.963  1.00 38.43           C
ATOM   3378  C    LYS C   13     -21.606 -67.082  92.306  1.00 39.27           C
ATOM   3379  O    LYS C   13     -21.543 -66.620  91.160  1.00 39.31           O
ATOM   3380  CB   LYS C   13     -23.391 -68.766  92.715  1.00 36.21           C
ATOM   3381  CG   LYS C   13     -24.872 -68.896  92.517  1.00 42.22           C
ATOM   3382  CD   LYS C   13     -25.398 -70.074  93.270  1.00 45.83           C
ATOM   3383  CE   LYS C   13     -25.107 -69.921  94.743  1.00 45.08           C
ATOM   3384  NZ   LYS C   13     -25.676 -71.073  95.499  1.00 49.42           N1+
ATOM   3385  N    PRO C   14     -20.517 -67.383  93.016  1.00 33.06           N
ATOM   3386  CA   PRO C   14     -19.180 -67.135  92.464  1.00 31.17           C
ATOM   3387  C    PRO C   14     -19.019 -67.705  91.063  1.00 35.18           C
ATOM   3388  O    PRO C   14     -19.621 -68.717  90.712  1.00 37.79           O
ATOM   3389  CB   PRO C   14     -18.258 -67.830  93.463  1.00 24.90           C
ATOM   3390  CG   PRO C   14     -19.016 -67.795  94.737  1.00 29.59           C
ATOM   3391  CD   PRO C   14     -20.461 -67.876  94.405  1.00 27.79           C
ATOM   3392  N    SER C   15     -18.235 -66.995  90.251  1.00 38.99           N
ATOM   3393  CA   SER C   15     -17.811 -67.278  88.882  1.00 33.18           C
ATOM   3394  C    SER C   15     -18.911 -66.976  87.855  1.00 35.06           C
ATOM   3395  O    SER C   15     -18.601 -66.892  86.661  1.00 34.68           O
ATOM   3396  CB   SER C   15     -17.276 -68.717  88.689  1.00 35.00           C
ATOM   3397  OG   SER C   15     -18.279 -69.665  88.363  1.00 36.60           O
ATOM   3398  N    GLU C   16     -20.172 -66.781  88.266  1.00 33.98           N
ATOM   3399  CA   GLU C   16     -21.187 -66.305  87.322  1.00 35.39           C
ATOM   3400  C    GLU C   16     -20.811 -64.890  86.852  1.00 35.84           C
ATOM   3401  O    GLU C   16     -19.841 -64.279  87.324  1.00 35.76           O
ATOM   3402  CB   GLU C   16     -22.590 -66.308  87.953  1.00 33.60           C
ATOM   3403  CG   GLU C   16     -22.982 -67.595  88.701  1.00 38.40           C
ATOM   3404  CD   GLU C   16     -24.487 -67.720  89.061  1.00 49.48           C
```

```
ATOM   3405  OE1 GLU C  16    -25.199 -66.698  89.225  1.00 50.75           O
ATOM   3406  OE2 GLU C  16    -24.950 -68.866  89.261  1.00 51.03           O1-
ATOM   3407  N   THR C  17    -21.570 -64.359  85.905  1.00 32.01           N
ATOM   3408  CA  THR C  17    -21.301 -63.010  85.451  1.00 32.01           C
ATOM   3409  C   THR C  17    -22.463 -62.094  85.818  1.00 39.55           C
ATOM   3410  O   THR C  17    -23.637 -62.460  85.685  1.00 36.43           O
ATOM   3411  CB  THR C  17    -20.978 -62.950  83.939  1.00 34.07           C
ATOM   3412  OG1 THR C  17    -21.956 -62.180  83.230  1.00 39.64           O
ATOM   3413  CG2 THR C  17    -20.841 -64.327  83.325  1.00 35.94           C
ATOM   3414  N   LEU C  18    -22.109 -60.901  86.295  1.00 37.34           N
ATOM   3415  CA  LEU C  18    -23.065 -59.889  86.703  1.00 30.52           C
ATOM   3416  C   LEU C  18    -23.481 -59.095  85.476  1.00 34.01           C
ATOM   3417  O   LEU C  18    -22.639 -58.717  84.651  1.00 35.55           O
ATOM   3418  CB  LEU C  18    -22.437 -59.006  87.782  1.00 29.47           C
ATOM   3419  CG  LEU C  18    -22.993 -57.708  88.384  1.00 35.82           C
ATOM   3420  CD1 LEU C  18    -22.732 -56.524  87.491  1.00 32.88           C
ATOM   3421  CD2 LEU C  18    -24.488 -57.846  88.679  1.00 31.62           C
ATOM   3422  N   SER C  19    -24.783 -58.870  85.340  1.00 31.00           N
ATOM   3423  CA  SER C  19    -25.301 -58.146  84.187  1.00 36.91           C
ATOM   3424  C   SER C  19    -26.378 -57.173  84.657  1.00 32.12           C
ATOM   3425  O   SER C  19    -27.371 -57.593  85.262  1.00 30.22           O
ATOM   3426  CB  SER C  19    -25.835 -59.143  83.141  1.00 33.82           C
ATOM   3427  OG  SER C  19    -26.265 -58.506  81.963  1.00 42.48           O
ATOM   3428  N   LEU C  20    -26.175 -55.877  84.364  1.00 30.42           N
ATOM   3429  CA  LEU C  20    -27.041 -54.785  84.804  1.00 30.56           C
ATOM   3430  C   LEU C  20    -27.300 -53.840  83.643  1.00 29.54           C
ATOM   3431  O   LEU C  20    -26.430 -53.618  82.801  1.00 31.80           O
ATOM   3432  CB  LEU C  20    -26.416 -53.973  85.970  1.00 25.88           C
ATOM   3433  CG  LEU C  20    -26.042 -54.745  87.243  1.00 27.95           C
ATOM   3434  CD1 LEU C  20    -25.312 -53.866  88.232  1.00 25.17           C
ATOM   3435  CD2 LEU C  20    -27.288 -55.391  87.897  1.00 28.86           C
ATOM   3436  N   THR C  21    -28.479 -53.236  83.636  1.00 29.19           N
ATOM   3437  CA  THR C  21    -28.844 -52.262  82.616  1.00 32.34           C
ATOM   3438  C   THR C  21    -29.444 -51.043  83.297  1.00 33.93           C
ATOM   3439  O   THR C  21    -30.059 -51.155  84.360  1.00 41.09           O
ATOM   3440  CB  THR C  21    -29.833 -52.839  81.576  1.00 32.67           C
ATOM   3441  OG1 THR C  21    -29.282 -54.034  81.016  1.00 29.98           O
ATOM   3442  CG2 THR C  21    -30.116 -51.832  80.457  1.00 28.76           C
ATOM   3443  N   CYS C  22    -29.221 -49.880  82.686  1.00 28.26           N
ATOM   3444  CA  CYS C  22    -29.766 -48.596  83.090  1.00 26.22           C
ATOM   3445  C   CYS C  22    -30.582 -48.024  81.939  1.00 35.11           C
ATOM   3446  O   CYS C  22    -30.119 -48.022  80.794  1.00 35.88           O
ATOM   3447  CB  CYS C  22    -28.601 -47.671  83.441  1.00 34.45           C
ATOM   3448  SG  CYS C  22    -28.863 -46.199  84.443  1.00 53.91           S
ATOM   3449  N   ALA C  23    -31.786 -47.526  82.223  1.00 41.07           N
ATOM   3450  CA  ALA C  23    -32.593 -46.849  81.211  1.00 32.31           C
ATOM   3451  C   ALA C  23    -32.715 -45.377  81.565  1.00 38.18           C
ATOM   3452  O   ALA C  23    -33.077 -45.034  82.693  1.00 40.40           O
ATOM   3453  CB  ALA C  23    -33.971 -47.489  81.072  1.00 31.29           C
ATOM   3454  N   VAL C  24    -32.419 -44.515  80.601  1.00 37.14           N
ATOM   3455  CA  VAL C  24    -32.433 -43.067  80.777  1.00 34.17           C
ATOM   3456  C   VAL C  24    -33.726 -42.531  80.183  1.00 39.79           C
ATOM   3457  O   VAL C  24    -34.063 -42.841  79.033  1.00 44.85           O
ATOM   3458  CB  VAL C  24    -31.202 -42.429  80.111  1.00 35.68           C
ATOM   3459  CG1 VAL C  24    -31.194 -40.927  80.274  1.00 33.16           C
ATOM   3460  CG2 VAL C  24    -29.937 -43.034  80.689  1.00 35.47           C
ATOM   3461  N   SER C  25    -34.433 -41.694  80.936  1.00 40.19           N
ATOM   3462  CA  SER C  25    -35.722 -41.217  80.453  1.00 37.50           C
ATOM   3463  C   SER C  25    -35.843 -39.707  80.355  1.00 46.83           C
ATOM   3464  O   SER C  25    -36.323 -39.206  79.339  1.00 56.63           O
ATOM   3465  CB  SER C  25    -36.842 -41.735  81.372  1.00 39.57           C
ATOM   3466  OG  SER C  25    -36.448 -42.914  82.077  1.00 44.46           O
ATOM   3467  N   GLY C  26    -35.362 -38.958  81.342  1.00 46.38           N
ATOM   3468  CA  GLY C  26    -35.703 -37.542  81.413  1.00 45.90           C
ATOM   3469  C   GLY C  26    -35.175 -36.666  80.290  1.00 45.91           C
ATOM   3470  O   GLY C  26    -35.926 -35.873  79.720  1.00 50.30           O
ATOM   3471  N   GLY C  27    -33.881 -36.752  79.983  1.00 41.90           N
ATOM   3472  CA  GLY C  27    -33.299 -35.764  79.082  1.00 33.59           C
ATOM   3473  C   GLY C  27    -32.626 -36.313  77.847  1.00 35.39           C
ATOM   3474  O   GLY C  27    -33.065 -37.324  77.298  1.00 41.91           O
ATOM   3475  N   SER C  28    -31.574 -35.646  77.382  1.00 41.18           N
```

```
ATOM  3476  CA   SER C  28   -30.856 -36.113  76.204  1.00 38.29        C
ATOM  3477  C    SER C  28   -29.975 -37.312  76.538  1.00 36.07        C
ATOM  3478  O    SER C  28   -29.631 -37.575  77.693  1.00 37.41        O
ATOM  3479  CB   SER C  28   -29.983 -35.007  75.609  1.00 38.36        C
ATOM  3480  OG   SER C  28   -30.761 -33.920  75.126  1.00 40.67        O
ATOM  3481  N    PHE C  29   -29.605 -38.042  75.494  1.00 36.30        N
ATOM  3482  CA   PHE C  29   -28.717 -39.178  75.630  1.00 36.41        C
ATOM  3483  C    PHE C  29   -27.350 -38.932  75.015  1.00 41.79        C
ATOM  3484  O    PHE C  29   -26.408 -39.669  75.329  1.00 42.57        O
ATOM  3485  CB   PHE C  29   -29.357 -40.414  74.999  1.00 29.89        C
ATOM  3486  CG   PHE C  29   -28.843 -41.712  75.541  1.00 31.96        C
ATOM  3487  CD1  PHE C  29   -28.786 -41.939  76.912  1.00 37.24        C
ATOM  3488  CD2  PHE C  29   -28.466 -42.735  74.682  1.00 32.71        C
ATOM  3489  CE1  PHE C  29   -28.325 -43.161  77.428  1.00 31.78        C
ATOM  3490  CE2  PHE C  29   -28.010 -43.953  75.182  1.00 32.65        C
ATOM  3491  CZ   PHE C  29   -27.946 -44.171  76.558  1.00 31.78        C
ATOM  3492  N    ARG C  30   -27.207 -37.893  74.190  1.00 36.62        N
ATOM  3493  CA   ARG C  30   -26.097 -37.790  73.261  1.00 40.24        C
ATOM  3494  C    ARG C  30   -24.969 -36.896  73.757  1.00 41.65        C
ATOM  3495  O    ARG C  30   -23.861 -36.972  73.223  1.00 37.36        O
ATOM  3496  CB   ARG C  30   -26.601 -37.249  71.922  1.00 37.58        C
ATOM  3497  CG   ARG C  30   -27.046 -35.805  72.068  1.00 51.10        C
ATOM  3498  CD   ARG C  30   -27.335 -35.130  70.748  1.00 52.17        C
ATOM  3499  NE   ARG C  30   -28.733 -35.284  70.381  1.00 58.44        N
ATOM  3500  CZ   ARG C  30   -29.297 -34.695  69.334  1.00 65.83        C
ATOM  3501  NH1  ARG C  30   -30.583 -34.897  69.073  1.00 66.12        N1+
ATOM  3502  NH2  ARG C  30   -28.575 -33.907  68.548  1.00 74.46        N
ATOM  3503  N    TYR C  31   -25.210 -36.062  74.758  1.00 44.38        N
ATOM  3504  CA   TYR C  31   -24.179 -35.148  75.227  1.00 38.68        C
ATOM  3505  C    TYR C  31   -23.376 -35.702  76.382  1.00 38.31        C
ATOM  3506  O    TYR C  31   -22.476 -35.022  76.876  1.00 37.88        O
ATOM  3507  CB   TYR C  31   -24.791 -33.816  75.660  1.00 39.97        C
ATOM  3508  CG   TYR C  31   -25.693 -33.157  74.646  1.00 38.66        C
ATOM  3509  CD1  TYR C  31   -25.199 -32.718  73.428  1.00 39.15        C
ATOM  3510  CD2  TYR C  31   -27.032 -32.967  74.913  1.00 40.52        C
ATOM  3511  CE1  TYR C  31   -26.008 -32.113  72.518  1.00 41.25        C
ATOM  3512  CE2  TYR C  31   -27.852 -32.352  74.011  1.00 45.11        C
ATOM  3513  CZ   TYR C  31   -27.342 -31.928  72.814  1.00 48.11        C
ATOM  3514  OH   TYR C  31   -28.178 -31.317  71.914  1.00 50.76        O
ATOM  3515  N    TYR C  32   -23.699 -36.894  76.851  1.00 41.37        N
ATOM  3516  CA   TYR C  32   -23.187 -37.357  78.121  1.00 33.57        C
ATOM  3517  C    TYR C  32   -22.357 -38.615  77.953  1.00 35.33        C
ATOM  3518  O    TYR C  32   -22.466 -39.346  76.967  1.00 39.05        O
ATOM  3519  CB   TYR C  32   -24.325 -37.630  79.090  1.00 34.60        C
ATOM  3520  CG   TYR C  32   -25.238 -36.454  79.258  1.00 38.37        C
ATOM  3521  CD1  TYR C  32   -24.873 -35.386  80.057  1.00 38.78        C
ATOM  3522  CD2  TYR C  32   -26.472 -36.408  78.610  1.00 37.80        C
ATOM  3523  CE1  TYR C  32   -25.711 -34.288  80.207  1.00 42.05        C
ATOM  3524  CE2  TYR C  32   -27.310 -35.329  78.749  1.00 41.43        C
ATOM  3525  CZ   TYR C  32   -26.927 -34.266  79.551  1.00 45.45        C
ATOM  3526  OH   TYR C  32   -27.758 -33.180  79.704  1.00 53.26        O
ATOM  3527  N    TYR C  33   -21.511 -38.840  78.942  1.00 36.12        N
ATOM  3528  CA   TYR C  33   -20.902 -40.133  79.185  1.00 33.30        C
ATOM  3529  C    TYR C  33   -21.731 -40.841  80.236  1.00 29.90        C
ATOM  3530  O    TYR C  33   -22.282 -40.201  81.135  1.00 34.09        O
ATOM  3531  CB   TYR C  33   -19.453 -39.988  79.655  1.00 32.91        C
ATOM  3532  CG   TYR C  33   -18.490 -39.876  78.500  1.00 32.64        C
ATOM  3533  CD1  TYR C  33   -18.235 -38.637  77.891  1.00 29.43        C
ATOM  3534  CD2  TYR C  33   -17.843 -41.004  78.005  1.00 32.78        C
ATOM  3535  CE1  TYR C  33   -17.352 -38.526  76.834  1.00 31.81        C
ATOM  3536  CE2  TYR C  33   -16.947 -40.905  76.954  1.00 37.66        C
ATOM  3537  CZ   TYR C  33   -16.714 -39.671  76.363  1.00 36.45        C
ATOM  3538  OH   TYR C  33   -15.840 -39.599  75.307  1.00 33.16        O
ATOM  3539  N    TRP C  34   -21.845 -42.154  80.099  1.00 31.46        N
ATOM  3540  CA   TRP C  34   -22.687 -42.974  80.957  1.00 28.61        C
ATOM  3541  C    TRP C  34   -21.790 -43.981  81.658  1.00 31.34        C
ATOM  3542  O    TRP C  34   -20.997 -44.666  80.994  1.00 26.44        O
ATOM  3543  CB   TRP C  34   -23.790 -43.628  80.118  1.00 25.06        C
ATOM  3544  CG   TRP C  34   -24.610 -42.557  79.485  1.00 31.01        C
ATOM  3545  CD1  TRP C  34   -24.506 -42.091  78.205  1.00 32.39        C
ATOM  3546  CD2  TRP C  34   -25.632 -41.770  80.113  1.00 33.17        C
```

| ATOM | 3547 | NE1 | TRP | C | 34 | -25.403 | -41.072 | 77.993 | 1.00 | 33.65 | N |
|------|------|-----|-----|---|----|---------|---------|--------|------|-------|-----|
| ATOM | 3548 | CE2 | TRP | C | 34 | -26.108 | -40.853 | 79.145 | 1.00 | 34.22 | C |
| ATOM | 3549 | CE3 | TRP | C | 34 | -26.205 | -41.761 | 81.389 | 1.00 | 28.29 | C |
| ATOM | 3550 | CZ2 | TRP | C | 34 | -27.131 | -39.940 | 79.416 | 1.00 | 33.64 | C |
| ATOM | 3551 | CZ3 | TRP | C | 34 | -27.211 | -40.834 | 81.659 | 1.00 | 30.29 | C |
| ATOM | 3552 | CH2 | TRP | C | 34 | -27.660 | -39.944 | 80.682 | 1.00 | 29.13 | C |
| ATOM | 3553 | N   | SER | C | 35 | -21.903 | -44.049 | 82.998 | 1.00 | 28.84 | N |
| ATOM | 3554 | CA  | SER | C | 35 | -20.898 | -44.663 | 83.856 | 1.00 | 26.34 | C |
| ATOM | 3555 | C   | SER | C | 35 | -21.495 | -45.683 | 84.815 | 1.00 | 25.62 | C |
| ATOM | 3556 | O   | SER | C | 35 | -22.691 | -45.688 | 85.096 | 1.00 | 28.59 | O |
| ATOM | 3557 | CB  | SER | C | 35 | -20.153 | -43.603 | 84.674 | 1.00 | 25.36 | C |
| ATOM | 3558 | OG  | SER | C | 35 | -19.476 | -42.699 | 83.828 | 1.00 | 36.45 | O |
| ATOM | 3559 | N   | TRP | C | 36 | -20.611 | -46.515 | 85.359 | 1.00 | 24.98 | N |
| ATOM | 3560 | CA  | TRP | C | 36 | -20.914 | -47.468 | 86.419 | 1.00 | 22.69 | C |
| ATOM | 3561 | C   | TRP | C | 36 | -19.937 | -47.262 | 87.567 | 1.00 | 26.01 | C |
| ATOM | 3562 | O   | TRP | C | 36 | -18.723 | -47.189 | 87.354 | 1.00 | 30.88 | O |
| ATOM | 3563 | CB  | TRP | C | 36 | -20.829 | -48.911 | 85.911 | 1.00 | 27.21 | C |
| ATOM | 3564 | CG  | TRP | C | 36 | -21.922 | -49.263 | 84.951 | 1.00 | 31.29 | C |
| ATOM | 3565 | CD1 | TRP | C | 36 | -21.856 | -49.288 | 83.581 | 1.00 | 27.11 | C |
| ATOM | 3566 | CD2 | TRP | C | 36 | -23.263 | -49.599 | 85.293 | 1.00 | 28.25 | C |
| ATOM | 3567 | NE1 | TRP | C | 36 | -23.082 | -49.636 | 83.050 | 1.00 | 25.91 | N |
| ATOM | 3568 | CE2 | TRP | C | 36 | -23.959 | -49.843 | 84.083 | 1.00 | 31.98 | C |
| ATOM | 3569 | CE3 | TRP | C | 36 | -23.942 | -49.732 | 86.504 | 1.00 | 23.12 | C |
| ATOM | 3570 | CZ2 | TRP | C | 36 | -25.310 | -50.203 | 84.059 | 1.00 | 30.86 | C |
| ATOM | 3571 | CZ3 | TRP | C | 36 | -25.280 | -50.087 | 86.479 | 1.00 | 26.83 | C |
| ATOM | 3572 | CH2 | TRP | C | 36 | -25.954 | -50.315 | 85.266 | 1.00 | 26.13 | C |
| ATOM | 3573 | N   | ILE | C | 37 | -20.469 | -47.181 | 88.779 | 1.00 | 27.24 | N |
| ATOM | 3574 | CA  | ILE | C | 37 | -19.713 | -46.954 | 90.007 | 1.00 | 25.13 | C |
| ATOM | 3575 | C   | ILE | C | 37 | -20.228 | -47.955 | 91.036 | 1.00 | 26.55 | C |
| ATOM | 3576 | O   | ILE | C | 37 | -21.442 | -48.138 | 91.155 | 1.00 | 29.45 | O |
| ATOM | 3577 | CB  | ILE | C | 37 | -19.904 | -45.502 | 90.503 | 1.00 | 24.76 | C |
| ATOM | 3578 | CG1 | ILE | C | 37 | -19.417 | -44.509 | 89.444 | 1.00 | 26.03 | C |
| ATOM | 3579 | CG2 | ILE | C | 37 | -19.245 | -45.259 | 91.853 | 1.00 | 28.36 | C |
| ATOM | 3580 | CD1 | ILE | C | 37 | -20.031 | -43.142 | 89.552 | 1.00 | 27.83 | C |
| ATOM | 3581 | N   | ARG | C | 38 | -19.328 | -48.630 | 91.755 | 1.00 | 21.22 | N |
| ATOM | 3582 | CA  | ARG | C | 38 | -19.764 | -49.538 | 92.810 | 1.00 | 25.95 | C |
| ATOM | 3583 | C   | ARG | C | 38 | -19.274 | -49.069 | 94.183 | 1.00 | 31.35 | C |
| ATOM | 3584 | O   | ARG | C | 38 | -18.283 | -48.340 | 94.308 | 1.00 | 26.88 | O |
| ATOM | 3585 | CB  | ARG | C | 38 | -19.314 | -50.993 | 92.559 | 1.00 | 23.96 | C |
| ATOM | 3586 | CG  | ARG | C | 38 | -17.834 | -51.151 | 92.450 | 1.00 | 33.62 | C |
| ATOM | 3587 | CD  | ARG | C | 38 | -17.312 | -52.176 | 93.382 | 1.00 | 31.96 | C |
| ATOM | 3588 | NE  | ARG | C | 38 | -17.339 | -53.503 | 92.796 | 1.00 | 36.58 | N |
| ATOM | 3589 | CZ  | ARG | C | 38 | -16.278 | -54.294 | 92.657 | 1.00 | 33.65 | C |
| ATOM | 3590 | NH1 | ARG | C | 38 | -15.072 | -53.910 | 93.057 | 1.00 | 31.68 | N1+ |
| ATOM | 3591 | NH2 | ARG | C | 38 | -16.436 | -55.483 | 92.123 | 1.00 | 28.23 | N |
| ATOM | 3592 | N   | GLN | C | 39 | -20.015 | -49.467 | 95.215 | 1.00 | 27.22 | N |
| ATOM | 3593 | CA  | GLN | C | 39 | -19.688 | -49.135 | 96.599 | 1.00 | 29.87 | C |
| ATOM | 3594 | C   | GLN | C | 39 | -19.795 | -50.406 | 97.437 | 1.00 | 28.44 | C |
| ATOM | 3595 | O   | GLN | C | 39 | -20.911 | -50.833 | 97.779 | 1.00 | 29.08 | O |
| ATOM | 3596 | CB  | GLN | C | 39 | -20.621 | -48.042 | 97.105 | 1.00 | 24.56 | C |
| ATOM | 3597 | CG  | GLN | C | 39 | -20.252 | -47.429 | 98.435 | 1.00 | 28.85 | C |
| ATOM | 3598 | CD  | GLN | C | 39 | -21.101 | -46.198 | 98.718 | 1.00 | 36.48 | C |
| ATOM | 3599 | OE1 | GLN | C | 39 | -22.318 | -46.187 | 98.497 | 1.00 | 42.72 | O |
| ATOM | 3600 | NE2 | GLN | C | 39 | -20.459 | -45.144 | 99.169 | 1.00 | 38.36 | N |
| ATOM | 3601 | N   | PRO | C | 40 | -18.674 | -51.040 | 97.791 | 1.00 | 30.67 | N |
| ATOM | 3602 | CA  | PRO | C | 40 | -18.757 | -52.243 | 98.625 | 1.00 | 27.55 | C |
| ATOM | 3603 | C   | PRO | C | 40 | -19.218 | -51.877 | 100.021 | 1.00 | 33.77 | C |
| ATOM | 3604 | O   | PRO | C | 40 | -18.964 | -50.757 | 100.496 | 1.00 | 34.43 | O |
| ATOM | 3605 | CB  | PRO | C | 40 | -17.315 | -52.776 | 98.621 | 1.00 | 27.60 | C |
| ATOM | 3606 | CG  | PRO | C | 40 | -16.645 | -52.058 | 97.420 | 1.00 | 26.34 | C |
| ATOM | 3607 | CD  | PRO | C | 40 | -17.294 | -50.736 | 97.371 | 1.00 | 28.79 | C |
| ATOM | 3608 | N   | PRO | C | 41 | -19.933 | -52.772 | 100.711 | 1.00 | 38.11 | N |
| ATOM | 3609 | CA  | PRO | C | 41 | -20.568 | -52.374 | 101.976 | 1.00 | 33.02 | C |
| ATOM | 3610 | C   | PRO | C | 41 | -19.528 | -51.959 | 103.001 | 1.00 | 40.48 | C |
| ATOM | 3611 | O   | PRO | C | 41 | -18.509 | -52.636 | 103.187 | 1.00 | 40.42 | O |
| ATOM | 3612 | CB  | PRO | C | 41 | -21.327 | -53.631 | 102.408 | 1.00 | 37.98 | C |
| ATOM | 3613 | CG  | PRO | C | 41 | -20.611 | -54.752 | 101.751 | 1.00 | 37.85 | C |
| ATOM | 3614 | CD  | PRO | C | 41 | -20.123 | -54.207 | 100.431 | 1.00 | 37.87 | C |
| ATOM | 3615 | N   | GLY | C | 42 | -19.761 | -50.786 | 103.604 | 1.00 | 40.56 | N |
| ATOM | 3616 | CA  | GLY | C | 42 | -18.805 | -50.159 | 104.482 | 1.00 | 38.30 | C |
| ATOM | 3617 | C   | GLY | C | 42 | -17.794 | -49.262 | 103.801 | 1.00 | 45.76 | C |

```
ATOM   3618  O    GLY C  42    -17.212 -48.400 104.462  1.00 47.20        O
ATOM   3619  N    LYS C  43    -17.557 -49.445 102.505  1.00 44.96        N
ATOM   3620  CA   LYS C  43    -16.391 -48.889 101.836  1.00 42.23        C
ATOM   3621  C    LYS C  43    -16.754 -47.617 101.060  1.00 40.08        C
ATOM   3622  O    LYS C  43    -17.835 -47.040 101.222  1.00 39.81        O
ATOM   3623  CB   LYS C  43    -15.764 -49.967 100.939  1.00 39.38        C
ATOM   3624  CG   LYS C  43    -15.348 -51.222 101.710  1.00 43.20        C
ATOM   3625  CD   LYS C  43    -14.763 -50.832 103.075  1.00 44.43        C
ATOM   3626  CE   LYS C  43    -14.563 -52.031 103.993  1.00 51.32        C
ATOM   3627  NZ   LYS C  43    -13.263 -52.735 103.784  1.00 55.64        N1+
ATOM   3628  N    GLY C  44    -15.827 -47.168 100.217  1.00 38.92        N
ATOM   3629  CA   GLY C  44    -16.003 -45.991  99.404  1.00 38.32        C
ATOM   3630  C    GLY C  44    -16.454 -46.330  98.000  1.00 39.62        C
ATOM   3631  O    GLY C  44    -17.012 -47.402  97.737  1.00 42.04        O
ATOM   3632  N    LEU C  45    -16.159 -45.425  97.075  1.00 32.83        N
ATOM   3633  CA   LEU C  45    -16.642 -45.491  95.709  1.00 27.76        C
ATOM   3634  C    LEU C  45    -15.528 -45.909  94.759  1.00 30.80        C
ATOM   3635  O    LEU C  45    -14.385 -45.473  94.905  1.00 37.23        O
ATOM   3636  CB   LEU C  45    -17.213 -44.134  95.309  1.00 28.31        C
ATOM   3637  CG   LEU C  45    -18.413 -43.760  96.186  1.00 29.60        C
ATOM   3638  CD1  LEU C  45    -18.761 -42.284  96.033  1.00 25.47        C
ATOM   3639  CD2  LEU C  45    -19.619 -44.621  95.903  1.00 26.22        C
ATOM   3640  N    GLU C  46    -15.834 -46.819  93.839  1.00 25.91        N
ATOM   3641  CA   GLU C  46    -14.895 -47.185  92.791  1.00 30.55        C
ATOM   3642  C    GLU C  46    -15.566 -47.029  91.433  1.00 35.38        C
ATOM   3643  O    GLU C  46    -16.677 -47.523  91.215  1.00 35.98        O
ATOM   3644  CB   GLU C  46    -14.340 -48.609  92.936  1.00 34.86        C
ATOM   3645  CG   GLU C  46    -14.868 -49.426  94.118  1.00 42.88        C
ATOM   3646  CD   GLU C  46    -14.142 -50.792  94.274  1.00 55.29        C
ATOM   3647  OE1  GLU C  46    -13.692 -51.365  93.231  1.00 52.24        O
ATOM   3648  OE2  GLU C  46    -14.010 -51.261  95.444  1.00 42.79        O1-
ATOM   3649  N    TRP C  47    -14.880 -46.345  90.524  1.00 35.22        N
ATOM   3650  CA   TRP C  47    -15.389 -46.093  89.189  1.00 31.80        C
ATOM   3651  C    TRP C  47    -15.070 -47.293  88.313  1.00 32.40        C
ATOM   3652  O    TRP C  47    -13.919 -47.720  88.238  1.00 34.10        O
ATOM   3653  CB   TRP C  47    -14.750 -44.817  88.647  1.00 27.85        C
ATOM   3654  CG   TRP C  47    -15.028 -44.369  87.213  1.00 29.38        C
ATOM   3655  CD1  TRP C  47    -16.066 -43.591  86.769  1.00 28.35        C
ATOM   3656  CD2  TRP C  47    -14.194 -44.605  86.072  1.00 31.89        C
ATOM   3657  NE1  TRP C  47    -15.932 -43.340  85.418  1.00 29.56        N
ATOM   3658  CE2  TRP C  47    -14.792 -43.954  84.970  1.00 29.80        C
ATOM   3659  CE3  TRP C  47    -12.996 -45.312  85.873  1.00 29.70        C
ATOM   3660  CZ2  TRP C  47    -14.234 -43.990  83.699  1.00 30.22        C
ATOM   3661  CZ3  TRP C  47    -12.455 -45.356  84.615  1.00 26.84        C
ATOM   3662  CH2  TRP C  47    -13.068 -44.696  83.541  1.00 31.06        C
ATOM   3663  N    PHE C  48    -16.096 -47.849  87.666  1.00 29.92        N
ATOM   3664  CA   PHE C  48    -15.868 -48.990  86.792  1.00 29.20        C
ATOM   3665  C    PHE C  48    -15.444 -48.547  85.397  1.00 29.36        C
ATOM   3666  O    PHE C  48    -14.536 -49.142  84.808  1.00 30.45        O
ATOM   3667  CB   PHE C  48    -17.123 -49.875  86.744  1.00 27.65        C
ATOM   3668  CG   PHE C  48    -17.029 -51.079  87.638  1.00 30.68        C
ATOM   3669  CD1  PHE C  48    -16.381 -50.992  88.876  1.00 34.82        C
ATOM   3670  CD2  PHE C  48    -17.539 -52.300  87.246  1.00 31.72        C
ATOM   3671  CE1  PHE C  48    -16.247 -52.101  89.705  1.00 32.25        C
ATOM   3672  CE2  PHE C  48    -17.423 -53.420  88.075  1.00 30.94        C
ATOM   3673  CZ   PHE C  48    -16.775 -53.319  89.298  1.00 33.98        C
ATOM   3674  N    GLY C  49    -16.078 -47.519  84.858  1.00 28.11        N
ATOM   3675  CA   GLY C  49    -15.826 -47.128  83.488  1.00 29.63        C
ATOM   3676  C    GLY C  49    -16.967 -46.291  82.950  1.00 32.96        C
ATOM   3677  O    GLY C  49    -17.956 -46.032  83.640  1.00 32.51        O
ATOM   3678  N    GLU C  50    -16.831 -45.916  81.674  1.00 31.40        N
ATOM   3679  CA   GLU C  50    -17.768 -45.012  81.011  1.00 31.61        C
ATOM   3680  C    GLU C  50    -17.854 -45.334  79.519  1.00 30.45        C
ATOM   3681  O    GLU C  50    -16.891 -45.819  78.920  1.00 31.68        O
ATOM   3682  CB   GLU C  50    -17.341 -43.542  81.204  1.00 28.42        C
ATOM   3683  CG   GLU C  50    -15.895 -43.237  80.715  1.00 27.97        C
ATOM   3684  CD   GLU C  50    -15.501 -41.745  80.792  1.00 33.91        C
ATOM   3685  OE1  GLU C  50    -14.488 -41.342  80.144  1.00 32.01        O
ATOM   3686  OE2  GLU C  50    -16.189 -40.969  81.511  1.00 33.87        O1-
ATOM   3687  N    ILE C  51    -19.006 -45.009  78.914  1.00 31.49        N
ATOM   3688  CA   ILE C  51    -19.254 -45.162  77.481  1.00 29.58        C
```

| ATOM | 3689 | C | ILE C | 51 | -19.904 | -43.883 | 76.949 | 1.00 | 36.19 | C |
|------|------|------|-------|----|---------|---------|--------|------|-------|---|
| ATOM | 3690 | O | ILE C | 51 | -20.636 | -43.193 | 77.666 | 1.00 | 32.17 | O |
| ATOM | 3691 | CB | ILE C | 51 | -20.128 | -46.407 | 77.173 | 1.00 | 30.54 | C |
| ATOM | 3692 | CG1 | ILE C | 51 | -20.175 | -46.709 | 75.657 | 1.00 | 30.91 | C |
| ATOM | 3693 | CG2 | ILE C | 51 | -21.541 | -46.263 | 77.756 | 1.00 | 22.08 | C |
| ATOM | 3694 | CD1 | ILE C | 51 | -20.472 | -48.177 | 75.340 | 1.00 | 21.13 | C |
| ATOM | 3695 | N | SER C | 52 | -19.687 | -43.604 | 75.652 | 1.00 | 44.18 | N |
| ATOM | 3696 | CA | SER C | 52 | -19.747 | -42.237 | 75.128 | 1.00 | 42.64 | C |
| ATOM | 3697 | C | SER C | 52 | -21.001 | -41.880 | 74.342 | 1.00 | 41.94 | C |
| ATOM | 3698 | O | SER C | 52 | -21.272 | -40.681 | 74.190 | 1.00 | 50.21 | O |
| ATOM | 3699 | CB | SER C | 52 | -18.545 | -41.962 | 74.210 | 1.00 | 46.91 | C |
| ATOM | 3700 | OG | SER C | 52 | -18.720 | -42.561 | 72.930 | 1.00 | 51.82 | O |
| ATOM | 3701 | N | HIS C | 53 | -21.739 | -42.865 | 73.831 | 1.00 | 36.80 | N |
| ATOM | 3702 | CA | HIS C | 53 | -22.860 | -42.688 | 72.900 | 1.00 | 46.33 | C |
| ATOM | 3703 | C | HIS C | 53 | -22.439 | -43.129 | 71.515 | 1.00 | 45.86 | C |
| ATOM | 3704 | O | HIS C | 53 | -23.241 | -43.704 | 70.781 | 1.00 | 49.43 | O |
| ATOM | 3705 | CB | HIS C | 53 | -23.410 | -41.251 | 72.789 | 1.00 | 46.91 | C |
| ATOM | 3706 | CG | HIS C | 53 | -24.569 | -41.127 | 71.846 | 1.00 | 52.45 | C |
| ATOM | 3707 | ND1 | HIS C | 53 | -24.405 | -40.953 | 70.488 | 1.00 | 47.45 | N |
| ATOM | 3708 | CD2 | HIS C | 53 | -25.907 | -41.185 | 72.059 | 1.00 | 50.31 | C |
| ATOM | 3709 | CE1 | HIS C | 53 | -25.590 | -40.915 | 69.905 | 1.00 | 43.08 | C |
| ATOM | 3710 | NE2 | HIS C | 53 | -26.519 | -41.054 | 70.835 | 1.00 | 45.78 | N |
| ATOM | 3711 | N | SER C | 54 | -21.183 | -42.881 | 71.170 | 1.00 | 47.96 | N |
| ATOM | 3712 | CA | SER C | 54 | -20.637 | -43.213 | 69.863 | 1.00 | 43.93 | C |
| ATOM | 3713 | C | SER C | 54 | -20.189 | -44.668 | 69.568 | 1.00 | 48.52 | C |
| ATOM | 3714 | O | SER C | 54 | -19.920 | -44.942 | 68.399 | 1.00 | 63.14 | O |
| ATOM | 3715 | CB | SER C | 54 | -19.464 | -42.299 | 69.569 | 1.00 | 40.27 | C |
| ATOM | 3716 | OG | SER C | 54 | -18.399 | -42.599 | 70.436 | 1.00 | 55.03 | O |
| ATOM | 3717 | N | GLY C | 55 | -19.968 | -45.572 | 70.529 | 1.00 | 41.03 | N |
| ATOM | 3718 | CA | GLY C | 55 | -19.813 | -45.352 | 71.953 | 1.00 | 41.92 | C |
| ATOM | 3719 | C | GLY C | 55 | -18.437 | -45.851 | 72.360 | 1.00 | 42.37 | C |
| ATOM | 3720 | O | GLY C | 55 | -18.224 | -47.051 | 72.578 | 1.00 | 27.61 | O |
| ATOM | 3721 | N | SER C | 56 | -17.483 | -44.927 | 72.441 | 1.00 | 44.03 | N |
| ATOM | 3722 | CA | SER C | 56 | -16.156 | -45.301 | 72.891 | 1.00 | 40.77 | C |
| ATOM | 3723 | C | SER C | 56 | -16.173 | -45.524 | 74.394 | 1.00 | 39.74 | C |
| ATOM | 3724 | O | SER C | 56 | -17.034 | -45.019 | 75.114 | 1.00 | 41.06 | O |
| ATOM | 3725 | CB | SER C | 56 | -15.122 | -44.243 | 72.520 | 1.00 | 39.37 | C |
| ATOM | 3726 | OG | SER C | 56 | -15.509 | -42.986 | 73.018 | 1.00 | 48.88 | O |
| ATOM | 3727 | N | THR C | 57 | -15.206 | -46.299 | 74.857 | 1.00 | 38.23 | N |
| ATOM | 3728 | CA | THR C | 57 | -15.193 | -46.845 | 76.196 | 1.00 | 29.07 | C |
| ATOM | 3729 | C | THR C | 57 | -13.912 | -46.459 | 76.932 | 1.00 | 32.82 | C |
| ATOM | 3730 | O | THR C | 57 | -12.865 | -46.266 | 76.312 | 1.00 | 34.18 | O |
| ATOM | 3731 | CB | THR C | 57 | -15.325 | -48.352 | 76.054 | 1.00 | 31.23 | C |
| ATOM | 3732 | OG1 | THR C | 57 | -16.527 | -48.801 | 76.681 | 1.00 | 34.61 | O |
| ATOM | 3733 | CG2 | THR C | 57 | -14.109 | -49.052 | 76.571 | 1.00 | 31.55 | C |
| ATOM | 3734 | N | ASN C | 58 | -14.002 | -46.318 | 78.257 | 1.00 | 31.01 | N |
| ATOM | 3735 | CA | ASN C | 58 | -12.838 | -46.118 | 79.121 | 1.00 | 27.41 | C |
| ATOM | 3736 | C | ASN C | 58 | -13.078 | -46.909 | 80.389 | 1.00 | 30.68 | C |
| ATOM | 3737 | O | ASN C | 58 | -14.007 | -46.592 | 81.136 | 1.00 | 31.50 | O |
| ATOM | 3738 | CB | ASN C | 58 | -12.590 | -44.649 | 79.491 | 1.00 | 24.84 | C |
| ATOM | 3739 | CG | ASN C | 58 | -12.449 | -43.760 | 78.297 | 1.00 | 28.91 | C |
| ATOM | 3740 | OD1 | ASN C | 58 | -11.439 | -43.792 | 77.598 | 1.00 | 34.90 | O |
| ATOM | 3741 | ND2 | ASN C | 58 | -13.458 | -42.937 | 78.055 | 1.00 | 33.30 | N |
| ATOM | 3742 | N | TYR C | 59 | -12.249 | -47.915 | 80.640 | 1.00 | 29.82 | N |
| ATOM | 3743 | CA | TYR C | 59 | -12.450 | -48.796 | 81.775 | 1.00 | 33.27 | C |
| ATOM | 3744 | C | TYR C | 59 | -11.447 | -48.512 | 82.871 | 1.00 | 30.48 | C |
| ATOM | 3745 | O | TYR C | 59 | -10.402 | -47.903 | 82.644 | 1.00 | 32.69 | O |
| ATOM | 3746 | CB | TYR C | 59 | -12.326 | -50.263 | 81.379 | 1.00 | 31.17 | C |
| ATOM | 3747 | CG | TYR C | 59 | -13.321 | -50.726 | 80.367 | 1.00 | 34.24 | C |
| ATOM | 3748 | CD1 | TYR C | 59 | -14.679 | -50.757 | 80.670 | 1.00 | 35.71 | C |
| ATOM | 3749 | CD2 | TYR C | 59 | -12.913 | -51.192 | 79.125 | 1.00 | 34.28 | C |
| ATOM | 3750 | CE1 | TYR C | 59 | -15.611 | -51.214 | 79.741 | 1.00 | 37.87 | C |
| ATOM | 3751 | CE2 | TYR C | 59 | -13.837 | -51.665 | 78.194 | 1.00 | 33.51 | C |
| ATOM | 3752 | CZ | TYR C | 59 | -15.183 | -51.659 | 78.498 | 1.00 | 33.62 | C |
| ATOM | 3753 | OH | TYR C | 59 | -16.095 | -52.115 | 77.572 | 1.00 | 34.03 | O |
| ATOM | 3754 | N | ASN C | 60 | -11.765 | -48.993 | 84.060 | 1.00 | 29.37 | N |
| ATOM | 3755 | CA | ASN C | 60 | -10.778 | -48.990 | 85.128 | 1.00 | 38.84 | C |
| ATOM | 3756 | C | ASN C | 60 | -9.747 | -50.095 | 84.877 | 1.00 | 40.23 | C |
| ATOM | 3757 | O | ASN C | 60 | -10.119 | -51.280 | 84.829 | 1.00 | 38.66 | O |
| ATOM | 3758 | CB | ASN C | 60 | -11.437 | -49.191 | 86.480 | 1.00 | 32.03 | C |
| ATOM | 3759 | CG | ASN C | 60 | -10.484 | -48.942 | 87.617 | 1.00 | 34.48 | C |

```
ATOM   3760  OD1 ASN C   60      -9.270 -48.906  87.422  1.00 34.69           O
ATOM   3761  ND2 ASN C   60     -11.018 -48.808  88.819  1.00 30.93           N
ATOM   3762  N   PRO C   61      -8.458 -49.763  84.741  1.00 38.19           N
ATOM   3763  CA  PRO C   61      -7.459 -50.799  84.419  1.00 38.23           C
ATOM   3764  C   PRO C   61      -7.385 -51.943  85.412  1.00 36.76           C
ATOM   3765  O   PRO C   61      -6.981 -53.047  85.035  1.00 42.26           O
ATOM   3766  CB  PRO C   61      -6.156 -49.991  84.368  1.00 37.21           C
ATOM   3767  CG  PRO C   61      -6.609 -48.658  83.864  1.00 35.33           C
ATOM   3768  CD  PRO C   61      -7.870 -48.412  84.660  1.00 38.00           C
ATOM   3769  N   SER C   62      -7.766 -51.718  86.663  1.00 37.94           N
ATOM   3770  CA  SER C   62      -7.755 -52.781  87.665  1.00 41.69           C
ATOM   3771  C   SER C   62      -8.781 -53.878  87.376  1.00 45.04           C
ATOM   3772  O   SER C   62      -8.587 -55.025  87.790  1.00 52.57           O
ATOM   3773  CB  SER C   62      -8.016 -52.197  89.039  1.00 36.72           C
ATOM   3774  OG  SER C   62      -9.370 -51.807  89.106  1.00 43.47           O
ATOM   3775  N   LEU C   63      -9.927 -53.530  86.792  1.00 39.84           N
ATOM   3776  CA  LEU C   63     -10.881 -54.557  86.394  1.00 42.71           C
ATOM   3777  C   LEU C   63     -10.311 -55.431  85.298  1.00 47.05           C
ATOM   3778  O   LEU C   63     -10.608 -56.635  85.235  1.00 44.87           O
ATOM   3779  CB  LEU C   63     -12.177 -53.929  85.914  1.00 39.14           C
ATOM   3780  CG  LEU C   63     -12.942 -53.409  87.106  1.00 41.14           C
ATOM   3781  CD1 LEU C   63     -14.324 -52.965  86.651  1.00 39.64           C
ATOM   3782  CD2 LEU C   63     -13.003 -54.481  88.194  1.00 37.86           C
ATOM   3783  N   LYS C   64      -9.555 -54.816  84.389  1.00 50.10           N
ATOM   3784  CA  LYS C   64      -8.831 -55.520  83.346  1.00 45.99           C
ATOM   3785  C   LYS C   64      -9.797 -56.175  82.376  1.00 46.14           C
ATOM   3786  O   LYS C   64     -10.663 -55.500  81.805  1.00 47.95           O
ATOM   3787  CB  LYS C   64      -7.875 -56.515  84.000  1.00 45.38           C
ATOM   3788  CG  LYS C   64      -6.494 -56.522  83.397  1.00 56.60           C
ATOM   3789  CD  LYS C   64      -5.651 -57.503  84.141  1.00 57.39           C
ATOM   3790  CE  LYS C   64      -5.450 -56.943  85.551  1.00 64.49           C
ATOM   3791  NZ  LYS C   64      -4.775 -57.891  86.483  1.00 75.39           N1+
ATOM   3792  N   ALA C   65      -9.676 -57.484  82.203  1.00 43.80           N
ATOM   3793  CA  ALA C   65     -10.484 -58.168  81.210  1.00 43.71           C
ATOM   3794  C   ALA C   65     -11.908 -58.474  81.670  1.00 40.71           C
ATOM   3795  O   ALA C   65     -12.730 -58.856  80.830  1.00 46.79           O
ATOM   3796  CB  ALA C   65      -9.789 -59.462  80.808  1.00 46.98           C
ATOM   3797  N   ARG C   66     -12.232 -58.303  82.958  1.00 34.69           N
ATOM   3798  CA  ARG C   66     -13.508 -58.800  83.490  1.00 35.91           C
ATOM   3799  C   ARG C   66     -14.721 -57.969  83.088  1.00 34.48           C
ATOM   3800  O   ARG C   66     -15.841 -58.467  83.207  1.00 34.86           O
ATOM   3801  CB  ARG C   66     -13.467 -58.840  85.013  1.00 37.25           C
ATOM   3802  CG  ARG C   66     -12.241 -59.497  85.584  1.00 41.56           C
ATOM   3803  CD  ARG C   66     -12.258 -59.442  87.090  1.00 33.48           C
ATOM   3804  NE  ARG C   66     -13.537 -59.870  87.586  1.00 36.42           N
ATOM   3805  CZ  ARG C   66     -14.096 -59.444  88.710  1.00 31.00           C
ATOM   3806  NH1 ARG C   66     -15.275 -59.921  89.045  1.00 32.67           N
ATOM   3807  NH2 ARG C   66     -13.499 -58.556  89.483  1.00 29.13           N
ATOM   3808  N   VAL C   67     -14.545 -56.720  82.650  1.00 36.81           N
ATOM   3809  CA  VAL C   67     -15.642 -55.760  82.552  1.00 33.37           C
ATOM   3810  C   VAL C   67     -15.923 -55.381  81.104  1.00 38.37           C
ATOM   3811  O   VAL C   67     -15.005 -55.251  80.288  1.00 41.21           O
ATOM   3812  CB  VAL C   67     -15.353 -54.501  83.391  1.00 36.36           C
ATOM   3813  CG1 VAL C   67     -14.134 -53.780  82.865  1.00 34.65           C
ATOM   3814  CG2 VAL C   67     -16.552 -53.582  83.346  1.00 36.14           C
ATOM   3815  N   THR C   68     -17.203 -55.212  80.792  1.00 35.08           N
ATOM   3816  CA  THR C   68     -17.654 -54.712  79.510  1.00 32.85           C
ATOM   3817  C   THR C   68     -18.769 -53.715  79.758  1.00 29.58           C
ATOM   3818  O   THR C   68     -19.686 -53.981  80.536  1.00 30.01           O
ATOM   3819  CB  THR C   68     -18.196 -55.829  78.605  1.00 36.95           C
ATOM   3820  OG1 THR C   68     -17.324 -56.957  78.657  1.00 39.01           O
ATOM   3821  CG2 THR C   68     -18.339 -55.320  77.154  1.00 28.49           C
ATOM   3822  N   ILE C   69     -18.719 -52.595  79.060  1.00 33.88           N
ATOM   3823  CA  ILE C   69     -19.792 -51.617  79.088  1.00 33.76           C
ATOM   3824  C   ILE C   69     -20.257 -51.398  77.658  1.00 34.31           C
ATOM   3825  O   ILE C   69     -19.439 -51.192  76.757  1.00 34.33           O
ATOM   3826  CB  ILE C   69     -19.349 -50.307  79.752  1.00 25.14           C
ATOM   3827  CG1 ILE C   69     -19.047 -50.590  81.218  1.00 24.64           C
ATOM   3828  CG2 ILE C   69     -20.418 -49.246  79.567  1.00 21.90           C
ATOM   3829  CD1 ILE C   69     -18.299 -49.486  81.965  1.00 29.90           C
ATOM   3830  N   SER C   70     -21.567 -51.459  77.455  1.00 35.47           N
```

```
ATOM   3831  CA  SER C  70    -22.170 -51.319  76.141  1.00 33.35           C
ATOM   3832  C   SER C  70    -23.321 -50.323  76.226  1.00 31.43           C
ATOM   3833  O   SER C  70    -23.862 -50.073  77.305  1.00 30.39           O
ATOM   3834  CB  SER C  70    -22.654 -52.668  75.638  1.00 23.50           C
ATOM   3835  OG  SER C  70    -23.508 -53.222  76.609  1.00 35.38           O
ATOM   3836  N   ILE C  71    -23.683 -49.755  75.074  1.00 30.99           N
ATOM   3837  CA  ILE C  71    -24.705 -48.722  74.981  1.00 30.48           C
ATOM   3838  C   ILE C  71    -25.660 -49.058  73.838  1.00 34.95           C
ATOM   3839  O   ILE C  71    -25.246 -49.587  72.806  1.00 34.86           O
ATOM   3840  CB  ILE C  71    -24.064 -47.335  74.790  1.00 32.81           C
ATOM   3841  CG1 ILE C  71    -25.061 -46.237  75.120  1.00 29.16           C
ATOM   3842  CG2 ILE C  71    -23.480 -47.167  73.376  1.00 30.88           C
ATOM   3843  CD1 ILE C  71    -24.442 -44.876  75.149  1.00 30.80           C
ATOM   3844  N   ASP C  72    -26.949 -48.798  74.042  1.00 39.76           N
ATOM   3845  CA  ASP C  72    -27.988 -48.981  73.025  1.00 35.04           C
ATOM   3846  C   ASP C  72    -28.651 -47.630  72.771  1.00 39.02           C
ATOM   3847  O   ASP C  72    -29.552 -47.241  73.518  1.00 43.96           O
ATOM   3848  CB  ASP C  72    -29.012 -50.011  73.491  1.00 37.61           C
ATOM   3849  CG  ASP C  72    -30.119 -50.283  72.465  1.00 45.97           C
ATOM   3850  OD1 ASP C  72    -30.612 -49.345  71.798  1.00 48.11           O
ATOM   3851  OD2 ASP C  72    -30.518 -51.461  72.346  1.00 49.16           O1-
ATOM   3852  N   THR C  73    -28.231 -46.910  71.726  1.00 38.09           N
ATOM   3853  CA  THR C  73    -28.807 -45.586  71.501  1.00 40.83           C
ATOM   3854  C   THR C  73    -30.255 -45.626  71.029  1.00 44.67           C
ATOM   3855  O   THR C  73    -30.892 -44.568  70.997  1.00 48.41           O
ATOM   3856  CB  THR C  73    -27.996 -44.759  70.502  1.00 39.06           C
ATOM   3857  OG1 THR C  73    -27.774 -45.521  69.308  1.00 46.00           O
ATOM   3858  CG2 THR C  73    -26.679 -44.327  71.114  1.00 40.64           C
ATOM   3859  O   SER C  74    -33.984 -45.804  71.487  1.00 55.05           O
ATOM   3860  N   SER C  74    -30.781 -46.782  70.622  1.00 41.41           N
ATOM   3861  CA  SER C  74    -32.194 -46.829  70.244  1.00 49.65           C
ATOM   3862  C   SER C  74    -33.087 -46.665  71.466  1.00 55.28           C
ATOM   3863  CB  SER C  74    -32.524 -48.149  69.539  1.00 50.83           C
ATOM   3864  OG  SER C  74    -31.745 -48.338  68.375  1.00 60.96           O
ATOM   3865  O   LYS C  75    -33.672 -46.626  75.929  1.00 49.05           O
ATOM   3866  N   LYS C  75    -32.833 -47.481  72.498  1.00 48.96           N
ATOM   3867  CA  LYS C  75    -33.603 -47.551  73.730  1.00 48.48           C
ATOM   3868  C   LYS C  75    -33.120 -46.598  74.819  1.00 45.98           C
ATOM   3869  CB  LYS C  75    -33.594 -48.986  74.274  1.00 47.37           C
ATOM   3870  CG  LYS C  75    -34.202 -49.988  73.320  1.00 54.32           C
ATOM   3871  CD  LYS C  75    -34.219 -51.397  73.886  1.00 53.30           C
ATOM   3872  CE  LYS C  75    -34.656 -52.383  72.819  1.00 62.19           C
ATOM   3873  NZ  LYS C  75    -35.421 -53.518  73.409  1.00 63.45           N
ATOM   3874  O   ASN C  76    -31.622 -45.589  77.918  1.00 39.83           O
ATOM   3875  N   ASN C  76    -32.109 -45.772  74.550  1.00 41.83           N
ATOM   3876  CA  ASN C  76    -31.496 -44.951  75.593  1.00 43.47           C
ATOM   3877  C   ASN C  76    -31.146 -45.811  76.807  1.00 36.05           C
ATOM   3878  CB  ASN C  76    -32.402 -43.771  75.976  1.00 36.10           C
ATOM   3879  CG  ASN C  76    -32.342 -42.656  74.963  1.00 38.85           C
ATOM   3880  OD1 ASN C  76    -31.821 -42.833  73.857  1.00 46.14           O
ATOM   3881  ND2 ASN C  76    -32.863 -41.498  75.326  1.00 45.02           N
ATOM   3882  N   GLN C  77    -30.384 -46.868  76.554  1.00 35.70           N
ATOM   3883  CA  GLN C  77    -29.976 -47.812  77.580  1.00 35.90           C
ATOM   3884  C   GLN C  77    -28.472 -48.013  77.506  1.00 36.92           C
ATOM   3885  O   GLN C  77    -27.858 -47.827  76.452  1.00 34.91           O
ATOM   3886  CB  GLN C  77    -30.697 -49.147  77.409  1.00 33.22           C
ATOM   3887  CG  GLN C  77    -32.184 -49.061  77.707  1.00 37.44           C
ATOM   3888  CD  GLN C  77    -32.889 -50.386  77.542  1.00 35.36           C
ATOM   3889  OE1 GLN C  77    -32.301 -51.358  77.088  1.00 30.09           O
ATOM   3890  NE2 GLN C  77    -34.154 -50.431  77.919  1.00 37.97           N
ATOM   3891  N   PHE C  78    -27.882 -48.404  78.638  1.00 30.45           N
ATOM   3892  CA  PHE C  78    -26.495 -48.839  78.650  1.00 28.00           C
ATOM   3893  C   PHE C  78    -26.342 -49.884  79.739  1.00 32.46           C
ATOM   3894  O   PHE C  78    -27.128 -49.933  80.686  1.00 33.14           O
ATOM   3895  CB  PHE C  78    -25.501 -47.668  78.812  1.00 29.59           C
ATOM   3896  CG  PHE C  78    -25.617 -46.911  80.109  1.00 29.92           C
ATOM   3897  CD1 PHE C  78    -26.541 -45.898  80.252  1.00 28.69           C
ATOM   3898  CD2 PHE C  78    -24.767 -47.182  81.160  1.00 29.77           C
ATOM   3899  CE1 PHE C  78    -26.635 -45.191  81.422  1.00 31.87           C
ATOM   3900  CE2 PHE C  78    -24.865 -46.484  82.343  1.00 32.84           C
ATOM   3901  CZ  PHE C  78    -25.800 -45.481  82.475  1.00 32.57           C
```

| ATOM | 3902 | N | SER C | 79 | -25.321 | -50.732 | 79.595 | 1.00 | 30.53 | N |
|------|------|------|-------|-----|---------|---------|--------|------|-------|-----|
| ATOM | 3903 | CA | SER C | 79 | -25.288 | -51.973 | 80.344 | 1.00 | 27.00 | C |
| ATOM | 3904 | C | SER C | 79 | -23.887 | -52.250 | 80.869 | 1.00 | 29.56 | C |
| ATOM | 3905 | O | SER C | 79 | -22.890 | -51.729 | 80.365 | 1.00 | 29.66 | O |
| ATOM | 3906 | CB | SER C | 79 | -25.778 | -53.134 | 79.474 | 1.00 | 30.28 | C |
| ATOM | 3907 | OG | SER C | 79 | -27.128 | -52.938 | 79.104 | 1.00 | 31.71 | O |
| ATOM | 3908 | N | LEU C | 80 | -23.833 | -53.114 | 81.879 | 1.00 | 24.67 | N |
| ATOM | 3909 | CA | LEU C | 80 | -22.595 | -53.502 | 82.522 | 1.00 | 26.25 | C |
| ATOM | 3910 | C | LEU C | 80 | -22.509 | -55.012 | 82.551 | 1.00 | 31.30 | C |
| ATOM | 3911 | O | LEU C | 80 | -23.454 | -55.677 | 82.982 | 1.00 | 32.63 | O |
| ATOM | 3912 | CB | LEU C | 80 | -22.519 | -52.968 | 83.957 | 1.00 | 30.49 | C |
| ATOM | 3913 | CG | LEU C | 80 | -21.310 | -53.471 | 84.764 | 1.00 | 31.84 | C |
| ATOM | 3914 | CD1 | LEU C | 80 | -19.986 | -52.935 | 84.206 | 1.00 | 24.42 | C |
| ATOM | 3915 | CD2 | LEU C | 80 | -21.459 | -53.149 | 86.240 | 1.00 | 29.26 | C |
| ATOM | 3916 | N | LYS C | 81 | -21.377 | -55.549 | 82.111 | 1.00 | 29.00 | N |
| ATOM | 3917 | CA | LYS C | 81 | -21.065 | -56.958 | 82.299 | 1.00 | 31.81 | C |
| ATOM | 3918 | C | LYS C | 81 | -19.803 | -57.045 | 83.139 | 1.00 | 31.17 | C |
| ATOM | 3919 | O | LYS C | 81 | -18.822 | -56.361 | 82.845 | 1.00 | 34.99 | O |
| ATOM | 3920 | CB | LYS C | 81 | -20.864 | -57.684 | 80.964 | 1.00 | 28.39 | C |
| ATOM | 3921 | CG | LYS C | 81 | -22.140 | -57.880 | 80.163 | 1.00 | 33.95 | C |
| ATOM | 3922 | CD | LYS C | 81 | -22.873 | -59.151 | 80.545 | 1.00 | 40.33 | C |
| ATOM | 3923 | CE | LYS C | 81 | -24.202 | -59.282 | 79.817 | 1.00 | 42.26 | C |
| ATOM | 3924 | NZ | LYS C | 81 | -24.025 | -59.096 | 78.349 | 1.00 | 53.78 | N1+ |
| ATOM | 3925 | N | LEU C | 82 | -19.835 | -57.857 | 84.193 | 1.00 | 30.70 | N |
| ATOM | 3926 | CA | LEU C | 82 | -18.652 | -58.141 | 85.004 | 1.00 | 28.52 | C |
| ATOM | 3927 | C | LEU C | 82 | -18.571 | -59.645 | 85.180 | 1.00 | 31.87 | C |
| ATOM | 3928 | O | LEU C | 82 | -19.434 | -60.247 | 85.824 | 1.00 | 37.82 | O |
| ATOM | 3929 | CB | LEU C | 82 | -18.701 | -57.439 | 86.356 | 1.00 | 33.35 | C |
| ATOM | 3930 | CG | LEU C | 82 | -17.543 | -57.699 | 87.330 | 1.00 | 35.28 | C |
| ATOM | 3931 | CD1 | LEU C | 82 | -16.244 | -57.034 | 86.881 | 1.00 | 26.92 | C |
| ATOM | 3932 | CD2 | LEU C | 82 | -17.950 | -57.297 | 88.741 | 1.00 | 25.50 | C |
| ATOM | 3933 | N | ARG C | 83 | -17.554 | -60.251 | 84.600 | 1.00 | 36.83 | N |
| ATOM | 3934 | CA | ARG C | 83 | -17.451 | -61.703 | 84.527 | 1.00 | 39.83 | C |
| ATOM | 3935 | C | ARG C | 83 | -16.737 | -62.289 | 85.745 | 1.00 | 38.25 | C |
| ATOM | 3936 | O | ARG C | 83 | -16.042 | -61.586 | 86.490 | 1.00 | 34.22 | O |
| ATOM | 3937 | CB | ARG C | 83 | -16.745 | -62.101 | 83.221 | 1.00 | 31.30 | C |
| ATOM | 3938 | CG | ARG C | 83 | -17.726 | -62.259 | 82.070 | 1.00 | 37.56 | C |
| ATOM | 3939 | CD | ARG C | 83 | -17.165 | -62.116 | 80.661 | 1.00 | 34.40 | C |
| ATOM | 3940 | NE | ARG C | 83 | -17.224 | -60.730 | 80.213 | 1.00 | 37.07 | N |
| ATOM | 3941 | CZ | ARG C | 83 | -16.174 | -59.946 | 80.029 | 1.00 | 39.95 | C |
| ATOM | 3942 | NH1 | ARG C | 83 | -16.359 | -58.701 | 79.618 | 1.00 | 31.66 | N1+ |
| ATOM | 3943 | NH2 | ARG C | 83 | -14.947 | -60.412 | 80.249 | 1.00 | 46.34 | N |
| ATOM | 3944 | N | SER C | 84 | -16.975 | -63.586 | 85.964 | 1.00 | 35.95 | N |
| ATOM | 3945 | CA | SER C | 84 | -16.215 | -64.416 | 86.908 | 1.00 | 36.75 | C |
| ATOM | 3946 | C | SER C | 84 | -16.089 | -63.725 | 88.271 | 1.00 | 37.32 | C |
| ATOM | 3947 | O | SER C | 84 | -15.020 | -63.344 | 88.754 | 1.00 | 41.30 | O |
| ATOM | 3948 | CB | SER C | 84 | -14.857 | -64.809 | 86.316 | 1.00 | 31.04 | C |
| ATOM | 3949 | OG | SER C | 84 | -14.036 | -63.687 | 86.179 | 1.00 | 38.56 | O |
| ATOM | 3950 | N | VAL C | 85 | -17.235 | -63.589 | 88.856 | 1.00 | 33.08 | N |
| ATOM | 3951 | CA | VAL C | 85 | -17.473 | -62.751 | 90.014 | 1.00 | 32.08 | C |
| ATOM | 3952 | C | VAL C | 85 | -17.088 | -63.499 | 91.292 | 1.00 | 32.80 | C |
| ATOM | 3953 | O | VAL C | 85 | -17.190 | -64.723 | 91.359 | 1.00 | 32.85 | O |
| ATOM | 3954 | CB | VAL C | 85 | -18.965 | -62.372 | 89.921 | 1.00 | 32.31 | C |
| ATOM | 3955 | CG1 | VAL C | 85 | -19.791 | -63.006 | 90.981 | 1.00 | 35.43 | C |
| ATOM | 3956 | CG2 | VAL C | 85 | -19.159 | -60.893 | 89.771 | 1.00 | 33.02 | C |
| ATOM | 3957 | N | THR C | 86 | -16.596 | -62.785 | 92.310 | 1.00 | 36.06 | N |
| ATOM | 3958 | CA | THR C | 86 | -16.263 | -63.389 | 93.614 | 1.00 | 32.42 | C |
| ATOM | 3959 | C | THR C | 86 | -16.976 | -62.624 | 94.727 | 1.00 | 32.82 | C |
| ATOM | 3960 | O | THR C | 86 | -17.644 | -61.619 | 94.485 | 1.00 | 32.02 | O |
| ATOM | 3961 | CB | THR C | 86 | -14.760 | -63.395 | 93.919 | 1.00 | 32.77 | C |
| ATOM | 3962 | OG1 | THR C | 86 | -14.390 | -62.115 | 94.438 | 1.00 | 35.84 | O |
| ATOM | 3963 | CG2 | THR C | 86 | -13.914 | -63.727 | 92.683 | 1.00 | 31.07 | C |
| ATOM | 3964 | N | ALA C | 87 | -16.813 | -63.084 | 95.972 | 1.00 | 34.97 | N |
| ATOM | 3965 | CA | ALA C | 87 | -17.533 | -62.446 | 97.079 | 1.00 | 35.92 | C |
| ATOM | 3966 | C | ALA C | 87 | -17.170 | -60.978 | 97.205 | 1.00 | 36.06 | C |
| ATOM | 3967 | O | ALA C | 87 | -18.015 | -60.163 | 97.593 | 1.00 | 34.31 | O |
| ATOM | 3968 | CB | ALA C | 87 | -17.241 | -63.146 | 98.407 | 1.00 | 24.03 | C |
| ATOM | 3969 | N | ALA C | 88 | -15.927 | -60.626 | 96.853 | 1.00 | 32.27 | N |
| ATOM | 3970 | CA | ALA C | 88 | -15.448 | -59.255 | 96.922 | 1.00 | 32.44 | C |
| ATOM | 3971 | C | ALA C | 88 | -16.179 | -58.331 | 95.964 | 1.00 | 35.33 | C |
| ATOM | 3972 | O | ALA C | 88 | -15.973 | -57.119 | 96.027 | 1.00 | 41.10 | O |

```
ATOM   3973  CB   ALA C  88    -13.953 -59.211  96.632  1.00 26.07       C
ATOM   3974  N    ASP C  89    -17.024 -58.861  95.090  1.00 31.77       N
ATOM   3975  CA   ASP C  89    -17.818 -58.037  94.203  1.00 29.68       C
ATOM   3976  C    ASP C  89    -19.198 -57.735  94.785  1.00 28.71       C
ATOM   3977  O    ASP C  89    -20.017 -57.099  94.115  1.00 29.06       O
ATOM   3978  CB   ASP C  89    -17.943 -58.712  92.825  1.00 36.29       C
ATOM   3979  CG   ASP C  89    -16.584 -58.922  92.122  1.00 33.46       C
ATOM   3980  OD1  ASP C  89    -15.893 -57.927  91.817  1.00 37.02       O
ATOM   3981  OD2  ASP C  89    -16.209 -60.092  91.867  1.00 34.16       O1-
ATOM   3982  N    THR C  90    -19.454 -58.137  96.028  1.00 27.76       N
ATOM   3983  CA   THR C  90    -20.653 -57.712  96.747  1.00 34.87       C
ATOM   3984  C    THR C  90    -20.606 -56.194  96.997  1.00 27.98       C
ATOM   3985  O    THR C  90    -19.694 -55.699  97.659  1.00 27.17       O
ATOM   3986  CB   THR C  90    -20.747 -58.492  98.058  1.00 32.97       C
ATOM   3987  OG1  THR C  90    -20.912 -59.884  97.753  1.00 34.09       O
ATOM   3988  CG2  THR C  90    -21.914 -58.010  98.923  1.00 24.31       C
ATOM   3989  N    ALA C  91    -21.575 -55.456  96.461  1.00 24.84       N
ATOM   3990  CA   ALA C  91    -21.553 -53.998  96.516  1.00 25.95       C
ATOM   3991  C    ALA C  91    -22.876 -53.467  95.992  1.00 29.10       C
ATOM   3992  O    ALA C  91    -23.667 -54.198  95.393  1.00 30.38       O
ATOM   3993  CB   ALA C  91    -20.408 -53.408  95.698  1.00 27.41       C
ATOM   3994  N    VAL C  92    -23.121 -52.183  96.249  1.00 28.41       N
ATOM   3995  CA   VAL C  92    -24.154 -51.471  95.517  1.00 25.07       C
ATOM   3996  C    VAL C  92    -23.533 -50.973  94.227  1.00 26.45       C
ATOM   3997  O    VAL C  92    -22.473 -50.348  94.242  1.00 29.34       O
ATOM   3998  CB   VAL C  92    -24.746 -50.313  96.338  1.00 25.04       C
ATOM   3999  CG1  VAL C  92    -25.682 -49.486  95.466  1.00 22.80       C
ATOM   4000  CG2  VAL C  92    -25.542 -50.843  97.503  1.00 26.44       C
ATOM   4001  N    TYR C  93    -24.177 -51.270  93.107  1.00 29.41       N
ATOM   4002  CA   TYR C  93    -23.720 -50.835  91.800  1.00 25.88       C
ATOM   4003  C    TYR C  93    -24.646 -49.722  91.336  1.00 24.87       C
ATOM   4004  O    TYR C  93    -25.854 -49.927  91.241  1.00 28.12       O
ATOM   4005  CB   TYR C  93    -23.703 -52.015  90.826  1.00 23.05       C
ATOM   4006  CG   TYR C  93    -22.607 -53.020  91.156  1.00 28.89       C
ATOM   4007  CD1  TYR C  93    -22.704 -53.859  92.280  1.00 24.24       C
ATOM   4008  CD2  TYR C  93    -21.462 -53.115  90.359  1.00 26.34       C
ATOM   4009  CE1  TYR C  93    -21.702 -54.752  92.593  1.00 23.55       C
ATOM   4010  CE2  TYR C  93    -20.449 -53.996  90.670  1.00 26.02       C
ATOM   4011  CZ   TYR C  93    -20.566 -54.818  91.783  1.00 26.56       C
ATOM   4012  OH   TYR C  93    -19.535 -55.695  92.078  1.00 23.40       O
ATOM   4013  N    TYR C  94    -24.085 -48.550  91.070  1.00 23.22       N
ATOM   4014  CA   TYR C  94    -24.829 -47.415  90.541  1.00 28.65       C
ATOM   4015  C    TYR C  94    -24.425 -47.179  89.097  1.00 25.80       C
ATOM   4016  O    TYR C  94    -23.241 -47.248  88.766  1.00 24.60       O
ATOM   4017  CB   TYR C  94    -24.547 -46.097  91.309  1.00 25.22       C
ATOM   4018  CG   TYR C  94    -24.796 -46.109  92.775  1.00 22.71       C
ATOM   4019  CD1  TYR C  94    -26.082 -45.978  93.280  1.00 24.72       C
ATOM   4020  CD2  TYR C  94    -23.738 -46.219  93.672  1.00 20.09       C
ATOM   4021  CE1  TYR C  94    -26.311 -45.997  94.640  1.00 22.52       C
ATOM   4022  CE2  TYR C  94    -23.950 -46.237  95.020  1.00 17.42       C
ATOM   4023  CZ   TYR C  94    -25.240 -46.119  95.508  1.00 27.12       C
ATOM   4024  OH   TYR C  94    -25.463 -46.130  96.877  1.00 35.58       O
ATOM   4025  N    CYS C  95    -25.391 -46.795  88.272  1.00 25.05       N
ATOM   4026  CA   CYS C  95    -25.096 -46.099  87.030  1.00 26.50       C
ATOM   4027  C    CYS C  95    -25.220 -44.602  87.287  1.00 28.56       C
ATOM   4028  O    CYS C  95    -25.943 -44.161  88.192  1.00 25.46       O
ATOM   4029  CB   CYS C  95    -26.022 -46.563  85.883  1.00 27.62       C
ATOM   4030  SG   CYS C  95    -27.838 -46.477  86.215  1.00 41.12       S
ATOM   4031  N    ALA C  96    -24.469 -43.816  86.520  1.00 26.44       N
ATOM   4032  CA   ALA C  96    -24.522 -42.377  86.723  1.00 27.54       C
ATOM   4033  C    ALA C  96    -24.302 -41.656  85.408  1.00 32.97       C
ATOM   4034  O    ALA C  96    -23.727 -42.202  84.464  1.00 34.61       O
ATOM   4035  CB   ALA C  96    -23.493 -41.909  87.746  1.00 24.37       C
ATOM   4036  N    ARG C  97    -24.772 -40.412  85.364  1.00 35.37       N
ATOM   4037  CA   ARG C  97    -24.529 -39.520  84.244  1.00 26.30       C
ATOM   4038  C    ARG C  97    -23.290 -38.679  84.524  1.00 27.36       C
ATOM   4039  O    ARG C  97    -23.208 -38.000  85.551  1.00 27.36       O
ATOM   4040  CB   ARG C  97    -25.732 -38.630  83.997  1.00 29.64       C
ATOM   4041  CG   ARG C  97    -25.581 -37.755  82.773  1.00 32.76       C
ATOM   4042  CD   ARG C  97    -26.887 -37.112  82.453  1.00 32.80       C
ATOM   4043  NE   ARG C  97    -26.856 -35.720  82.854  1.00 39.43       N
```

| ATOM | 4044 | CZ | ARG C | 97 | -27.927 | -34.974 | 83.090 | 1.00 | 37.66 | C |
|------|------|-----|-------|-----|---------|---------|--------|------|-------|-----|
| ATOM | 4045 | NH1 | ARG C | 97 | -27.763 | -33.707 | 83.447 | 1.00 | 44.97 | N1+ |
| ATOM | 4046 | NH2 | ARG C | 97 | -29.146 | -35.483 | 82.978 | 1.00 | 39.25 | N |
| ATOM | 4047 | N | ASP C | 98 | -22.322 | -38.755 | 83.621 | 1.00 | 30.15 | N |
| ATOM | 4048 | CA | ASP C | 98 | -21.054 | -38.053 | 83.724 | 1.00 | 29.73 | C |
| ATOM | 4049 | C | ASP C | 98 | -21.145 | -36.751 | 82.929 | 1.00 | 33.62 | C |
| ATOM | 4050 | O | ASP C | 98 | -21.487 | -36.781 | 81.742 | 1.00 | 31.03 | O |
| ATOM | 4051 | CB | ASP C | 98 | -19.936 | -38.949 | 83.190 | 1.00 | 30.30 | C |
| ATOM | 4052 | CG | ASP C | 98 | -18.555 | -38.414 | 83.490 | 1.00 | 31.40 | C |
| ATOM | 4053 | OD1 | ASP C | 98 | -18.240 | -37.292 | 83.033 | 1.00 | 31.40 | O1- |
| ATOM | 4054 | OD2 | ASP C | 98 | -17.779 | -39.139 | 84.160 | 1.00 | 32.47 | O |
| ATOM | 4055 | N | TYR C | 99 | -20.901 | -35.610 | 83.601 | 1.00 | 32.07 | N |
| ATOM | 4056 | CA | TYR C | 99 | -20.981 | -34.283 | 82.971 | 1.00 | 29.21 | C |
| ATOM | 4057 | C | TYR C | 99 | -20.323 | -33.081 | 83.723 | 1.00 | 28.12 | C |
| ATOM | 4058 | O | TYR C | 99 | -21.081 | -32.255 | 84.196 | 1.00 | 36.95 | O |
| ATOM | 4059 | CB | TYR C | 99 | -22.484 | -33.958 | 82.761 | 1.00 | 35.21 | C |
| ATOM | 4060 | CG | TYR C | 99 | -22.884 | -32.899 | 81.729 | 1.00 | 37.19 | C |
| ATOM | 4061 | CD1 | TYR C | 99 | -22.518 | -33.004 | 80.378 | 1.00 | 37.05 | C |
| ATOM | 4062 | CD2 | TYR C | 99 | -23.629 | -31.784 | 82.114 | 1.00 | 38.73 | C |
| ATOM | 4063 | CE1 | TYR C | 99 | -22.905 | -32.034 | 79.447 | 1.00 | 36.99 | C |
| ATOM | 4064 | CE2 | TYR C | 99 | -24.008 | -30.801 | 81.195 | 1.00 | 32.76 | C |
| ATOM | 4065 | CZ | TYR C | 99 | -23.652 | -30.924 | 79.871 | 1.00 | 40.53 | C |
| ATOM | 4066 | OH | TYR C | 99 | -24.045 | -29.934 | 78.971 | 1.00 | 41.60 | O |
| ATOM | 4067 | N | GLY C | 100 | -19.003 | -32.924 | 83.921 | 1.00 | 28.67 | N |
| ATOM | 4068 | CA | GLY C | 100 | -17.960 | -33.926 | 83.970 | 1.00 | 29.97 | C |
| ATOM | 4069 | C | GLY C | 100 | -17.825 | -34.458 | 85.387 | 1.00 | 28.43 | C |
| ATOM | 4070 | O | GLY C | 100 | -17.023 | -35.343 | 85.668 | 1.00 | 35.72 | O |
| ATOM | 4071 | N | ALA C | 101 | -18.638 | -33.941 | 86.294 | 1.00 | 28.13 | N |
| ATOM | 4072 | CA | ALA C | 101 | -18.846 | -34.644 | 87.546 | 1.00 | 23.31 | C |
| ATOM | 4073 | C | ALA C | 101 | -20.080 | -35.531 | 87.385 | 1.00 | 29.38 | C |
| ATOM | 4074 | O | ALA C | 101 | -20.763 | -35.484 | 86.363 | 1.00 | 31.34 | O |
| ATOM | 4075 | CB | ALA C | 101 | -19.026 | -33.659 | 88.689 | 1.00 | 26.55 | C |
| ATOM | 4076 | N | PHE C | 102 | -20.379 | -36.347 | 88.395 | 1.00 | 25.69 | N |
| ATOM | 4077 | CA | PHE C | 102 | -21.562 | -37.213 | 88.337 | 1.00 | 30.27 | C |
| ATOM | 4078 | C | PHE C | 102 | -22.754 | -36.480 | 88.948 | 1.00 | 27.72 | C |
| ATOM | 4079 | O | PHE C | 102 | -22.912 | -36.444 | 90.170 | 1.00 | 25.66 | O |
| ATOM | 4080 | CB | PHE C | 102 | -21.314 | -38.547 | 89.035 | 1.00 | 28.00 | C |
| ATOM | 4081 | CG | PHE C | 102 | -20.216 | -39.368 | 88.406 | 1.00 | 28.82 | C |
| ATOM | 4082 | CD1 | PHE C | 102 | -20.377 | -39.915 | 87.135 | 1.00 | 26.74 | C |
| ATOM | 4083 | CD2 | PHE C | 102 | -19.002 | -39.556 | 89.069 | 1.00 | 25.45 | C |
| ATOM | 4084 | CE1 | PHE C | 102 | -19.376 | -40.658 | 86.548 | 1.00 | 23.07 | C |
| ATOM | 4085 | CE2 | PHE C | 102 | -17.987 | -40.307 | 88.488 | 1.00 | 27.62 | C |
| ATOM | 4086 | CZ | PHE C | 102 | -18.173 | -40.859 | 87.220 | 1.00 | 24.95 | C |
| ATOM | 4087 | N | ASP C | 103 | -23.611 | -35.906 | 88.097 | 1.00 | 25.30 | N |
| ATOM | 4088 | CA | ASP C | 103 | -24.746 | -35.131 | 88.591 | 1.00 | 29.00 | C |
| ATOM | 4089 | C | ASP C | 103 | -26.021 | -35.943 | 88.801 | 1.00 | 32.32 | C |
| ATOM | 4090 | O | ASP C | 103 | -26.862 | -35.523 | 89.596 | 1.00 | 36.85 | O |
| ATOM | 4091 | CB | ASP C | 103 | -25.067 | -33.937 | 87.671 | 1.00 | 26.81 | C |
| ATOM | 4092 | CG | ASP C | 103 | -25.328 | -34.341 | 86.198 | 1.00 | 41.22 | C |
| ATOM | 4093 | OD1 | ASP C | 103 | -25.539 | -35.538 | 85.880 | 1.00 | 33.40 | O1- |
| ATOM | 4094 | OD2 | ASP C | 103 | -25.333 | -33.419 | 85.338 | 1.00 | 51.21 | O |
| ATOM | 4095 | N | ILE C | 104 | -26.213 | -37.077 | 88.127 | 1.00 | 29.34 | N |
| ATOM | 4096 | CA | ILE C | 104 | -27.410 | -37.884 | 88.337 | 1.00 | 28.45 | C |
| ATOM | 4097 | C | ILE C | 104 | -27.007 | -39.332 | 88.536 | 1.00 | 30.33 | C |
| ATOM | 4098 | O | ILE C | 104 | -26.120 | -39.840 | 87.842 | 1.00 | 31.30 | O |
| ATOM | 4099 | CB | ILE C | 104 | -28.406 | -37.758 | 87.170 | 1.00 | 31.09 | C |
| ATOM | 4100 | CG1 | ILE C | 104 | -28.861 | -36.309 | 87.017 | 1.00 | 29.48 | C |
| ATOM | 4101 | CG2 | ILE C | 104 | -29.583 | -38.689 | 87.387 | 1.00 | 25.98 | C |
| ATOM | 4102 | CD1 | ILE C | 104 | -29.838 | -36.138 | 85.943 | 1.00 | 31.56 | C |
| ATOM | 4103 | N | TRP C | 105 | -27.666 | -39.992 | 89.480 | 1.00 | 27.28 | N |
| ATOM | 4104 | CA | TRP C | 105 | -27.361 | -41.357 | 89.855 | 1.00 | 27.66 | C |
| ATOM | 4105 | C | TRP C | 105 | -28.599 | -42.236 | 89.754 | 1.00 | 29.36 | C |
| ATOM | 4106 | O | TRP C | 105 | -29.720 | -41.789 | 90.005 | 1.00 | 31.41 | O |
| ATOM | 4107 | CB | TRP C | 105 | -26.830 | -41.398 | 91.288 | 1.00 | 28.75 | C |
| ATOM | 4108 | CG | TRP C | 105 | -25.559 | -40.624 | 91.483 | 1.00 | 27.49 | C |
| ATOM | 4109 | CD1 | TRP C | 105 | -25.394 | -39.267 | 91.400 | 1.00 | 27.23 | C |
| ATOM | 4110 | CD2 | TRP C | 105 | -24.276 | -41.167 | 91.802 | 1.00 | 25.31 | C |
| ATOM | 4111 | NE1 | TRP C | 105 | -24.078 | -38.935 | 91.650 | 1.00 | 25.97 | N |
| ATOM | 4112 | CE2 | TRP C | 105 | -23.373 | -40.083 | 91.900 | 1.00 | 25.23 | C |
| ATOM | 4113 | CE3 | TRP C | 105 | -23.801 | -42.466 | 92.016 | 1.00 | 25.04 | C |
| ATOM | 4114 | CZ2 | TRP C | 105 | -22.026 | -40.259 | 92.188 | 1.00 | 22.01 | C |

```
ATOM   4115  CZ3 TRP C 105     -22.461 -42.639  92.307  1.00 27.62           C
ATOM   4116  CH2 TRP C 105     -21.586 -41.532  92.389  1.00 24.95           C
ATOM   4117  N   GLY C 106     -28.381 -43.504  89.436  1.00 29.88           N
ATOM   4118  CA  GLY C 106     -29.424 -44.496  89.587  1.00 34.79           C
ATOM   4119  C   GLY C 106     -29.697 -44.833  91.048  1.00 34.11           C
ATOM   4120  O   GLY C 106     -28.952 -44.456  91.951  1.00 31.75           O
ATOM   4121  N   GLN C 107     -30.828 -45.525  91.277  1.00 37.10           N
ATOM   4122  CA  GLN C 107     -31.137 -46.052  92.607  1.00 33.95           C
ATOM   4123  C   GLN C 107     -30.005 -46.904  93.124  1.00 30.12           C
ATOM   4124  O   GLN C 107     -29.773 -46.956  94.331  1.00 31.44           O
ATOM   4125  CB  GLN C 107     -32.411 -46.921  92.621  1.00 38.95           C
ATOM   4126  CG  GLN C 107     -33.442 -46.732  91.485  1.00 53.38           C
ATOM   4127  CD  GLN C 107     -33.137 -47.557  90.207  1.00 55.26           C
ATOM   4128  OE1 GLN C 107     -33.665 -48.666  89.992  1.00 51.28           O
ATOM   4129  NE2 GLN C 107     -32.299 -46.993  89.348  1.00 50.94           N
ATOM   4130  N   GLY C 108     -29.311 -47.597  92.233  1.00 30.68           N
ATOM   4131  CA  GLY C 108     -28.376 -48.619  92.628  1.00 25.20           C
ATOM   4132  C   GLY C 108     -29.039 -49.981  92.689  1.00 28.53           C
ATOM   4133  O   GLY C 108     -30.254 -50.110  92.792  1.00 32.61           O
ATOM   4134  N   THR C 109     -28.203 -51.008  92.614  1.00 26.94           N
ATOM   4135  CA  THR C 109     -28.607 -52.403  92.680  1.00 22.92           C
ATOM   4136  C   THR C 109     -27.757 -53.086  93.740  1.00 30.47           C
ATOM   4137  O   THR C 109     -26.523 -53.049  93.674  1.00 32.40           O
ATOM   4138  CB  THR C 109     -28.418 -53.094  91.321  1.00 29.24           C
ATOM   4139  OG1 THR C 109     -29.226 -52.453  90.327  1.00 29.50           O
ATOM   4140  CG2 THR C 109     -28.754 -54.591  91.395  1.00 25.49           C
ATOM   4141  N   MET C 110     -28.402 -53.685  94.726  1.00 30.19           N
ATOM   4142  CA  MET C 110     -27.674 -54.400  95.765  1.00 31.87           C
ATOM   4143  C   MET C 110     -27.271 -55.749  95.187  1.00 30.25           C
ATOM   4144  O   MET C 110     -28.133 -56.548  94.818  1.00 31.68           O
ATOM   4145  CB  MET C 110     -28.571 -54.546  96.991  1.00 38.09           C
ATOM   4146  CG  MET C 110     -27.925 -54.739  98.362  1.00 38.26           C
ATOM   4147  SD  MET C 110     -29.296 -54.935  99.598  1.00 60.00           S
ATOM   4148  CE  MET C 110     -30.587 -53.829  98.963  1.00 28.36           C
ATOM   4149  N   VAL C 111     -25.971 -55.986  95.049  1.00 30.68           N
ATOM   4150  CA  VAL C 111     -25.467 -57.191  94.399  1.00 30.46           C
ATOM   4151  C   VAL C 111     -24.685 -57.989  95.422  1.00 30.94           C
ATOM   4152  O   VAL C 111     -23.698 -57.495  95.978  1.00 34.81           O
ATOM   4153  CB  VAL C 111     -24.601 -56.875  93.164  1.00 34.32           C
ATOM   4154  CG1 VAL C 111     -23.821 -58.100  92.745  1.00 28.86           C
ATOM   4155  CG2 VAL C 111     -25.462 -56.397  91.995  1.00 24.78           C
ATOM   4156  N   THR C 112     -25.136 -59.214  95.676  1.00 33.49           N
ATOM   4157  CA  THR C 112     -24.495 -60.147  96.593  1.00 29.04           C
ATOM   4158  C   THR C 112     -23.993 -61.341  95.798  1.00 31.99           C
ATOM   4159  O   THR C 112     -24.749 -61.926  95.013  1.00 28.19           O
ATOM   4160  CB  THR C 112     -25.483 -60.629  97.670  1.00 27.70           C
ATOM   4161  OG1 THR C 112     -26.126 -59.513  98.275  1.00 27.51           O
ATOM   4162  CG2 THR C 112     -24.800 -61.447  98.736  1.00 30.55           C
ATOM   4163  N   VAL C 113     -22.726 -61.696  95.985  1.00 29.97           N
ATOM   4164  CA  VAL C 113     -22.185 -62.918  95.405  1.00 36.12           C
ATOM   4165  C   VAL C 113     -21.678 -63.780  96.550  1.00 33.41           C
ATOM   4166  O   VAL C 113     -20.902 -63.312  97.392  1.00 31.40           O
ATOM   4167  CB  VAL C 113     -21.119 -62.648  94.319  1.00 37.87           C
ATOM   4168  CG1 VAL C 113     -20.582 -61.246  94.423  1.00 37.11           C
ATOM   4169  CG2 VAL C 113     -20.016 -63.719  94.288  1.00 28.90           C
ATOM   4170  N   SER C 114     -22.171 -65.019  96.610  1.00 33.34           N
ATOM   4171  CA  SER C 114     -21.991 -65.863  97.781  1.00 37.36           C
ATOM   4172  C   SER C 114     -22.309 -67.316  97.449  1.00 34.25           C
ATOM   4173  O   SER C 114     -23.113 -67.606  96.563  1.00 32.33           O
ATOM   4174  CB  SER C 114     -22.886 -65.382  98.932  1.00 34.37           C
ATOM   4175  OG  SER C 114     -22.928 -66.326  99.983  1.00 36.36           O
ATOM   4176  N   SER C 115     -21.682 -68.224  98.195  1.00 38.98           N
ATOM   4177  CA  SER C 115     -22.065 -69.629  98.129  1.00 38.55           C
ATOM   4178  C   SER C 115     -23.430 -69.890  98.753  1.00 44.22           C
ATOM   4179  O   SER C 115     -24.069 -70.889  98.410  1.00 48.62           O
ATOM   4180  CB  SER C 115     -21.032 -70.491  98.847  1.00 40.98           C
ATOM   4181  OG  SER C 115     -19.794 -70.465  98.158  1.00 57.58           O
ATOM   4182  N   ALA C 116     -23.912 -69.003  99.620  1.00 34.12           N
ATOM   4183  CA  ALA C 116     -25.140 -69.269 100.348  1.00 37.60           C
ATOM   4184  C   ALA C 116     -26.347 -69.330  99.415  1.00 39.26           C
ATOM   4185  O   ALA C 116     -26.329 -68.827  98.282  1.00 36.17           O
```

| ATOM | 4186 | CB | ALA C 116 | -25.366 -68.194 101.413 | 1.00 33.12 | C |
| ATOM | 4187 | N | SER C 117 | -27.414 -69.957 99.915 | 1.00 36.37 | N |
| ATOM | 4188 | CA | SER C 117 | -28.684 -70.042 99.206 | 1.00 39.66 | C |
| ATOM | 4189 | C | SER C 117 | -29.745 -69.252 99.960 | 1.00 36.99 | C |
| ATOM | 4190 | O | SER C 117 | -29.660 -69.069 101.179 | 1.00 40.67 | O |
| ATOM | 4191 | CB | SER C 117 | -29.137 -71.504 99.021 | 1.00 40.84 | C |
| ATOM | 4192 | OG | SER C 117 | -28.239 -72.218 98.172 | 1.00 42.49 | O |
| ATOM | 4193 | N | THR C 118 | -30.735 -68.770 99.215 | 1.00 35.22 | N |
| ATOM | 4194 | CA | THR C 118 | -31.796 -67.963 99.796 | 1.00 34.00 | C |
| ATOM | 4195 | C | THR C 118 | -32.453 -68.705 100.953 | 1.00 37.86 | C |
| ATOM | 4196 | O | THR C 118 | -32.680 -69.912 100.889 | 1.00 37.72 | O |
| ATOM | 4197 | CB | THR C 118 | -32.829 -67.600 98.724 | 1.00 27.84 | C |
| ATOM | 4198 | OG1 | THR C 118 | -32.225 -66.729 97.771 | 1.00 31.08 | O |
| ATOM | 4199 | CG2 | THR C 118 | -34.051 -66.887 99.332 | 1.00 36.20 | C |
| ATOM | 4200 | N | LYS C 119 | -32.740 -67.973 102.024 | 1.00 38.37 | N |
| ATOM | 4201 | CA | LYS C 119 | -33.239 -68.569 103.256 | 1.00 37.26 | C |
| ATOM | 4202 | C | LYS C 119 | -33.922 -67.486 104.079 | 1.00 34.75 | C |
| ATOM | 4203 | O | LYS C 119 | -33.332 -66.432 104.331 | 1.00 32.89 | O |
| ATOM | 4204 | CB | LYS C 119 | -32.093 -69.201 104.040 | 1.00 32.21 | C |
| ATOM | 4205 | CG | LYS C 119 | -32.520 -69.868 105.295 | 1.00 32.82 | C |
| ATOM | 4206 | CD | LYS C 119 | -31.299 -70.193 106.108 | 1.00 34.06 | C |
| ATOM | 4207 | CE | LYS C 119 | -31.675 -70.872 107.411 | 1.00 37.24 | C |
| ATOM | 4208 | NZ | LYS C 119 | -32.670 -70.086 108.181 | 1.00 41.53 | N1+ |
| ATOM | 4209 | N | GLY C 120 | -35.166 -67.738 104.474 | 1.00 34.46 | N |
| ATOM | 4210 | CA | GLY C 120 | -35.906 -66.796 105.275 | 1.00 30.62 | C |
| ATOM | 4211 | C | GLY C 120 | -35.395 -66.829 106.696 | 1.00 30.67 | C |
| ATOM | 4212 | O | GLY C 120 | -34.703 -67.767 107.102 | 1.00 29.23 | O |
| ATOM | 4213 | N | PRO C 121 | -35.705 -65.792 107.467 | 1.00 28.30 | N |
| ATOM | 4214 | CA | PRO C 121 | -35.198 -65.683 108.838 | 1.00 30.58 | C |
| ATOM | 4215 | C | PRO C 121 | -36.084 -66.380 109.863 | 1.00 30.35 | C |
| ATOM | 4216 | O | PRO C 121 | -37.286 -66.554 109.668 | 1.00 31.40 | O |
| ATOM | 4217 | CB | PRO C 121 | -35.223 -64.172 109.082 | 1.00 29.17 | C |
| ATOM | 4218 | CG | PRO C 121 | -36.377 -63.701 108.245 | 1.00 27.47 | C |
| ATOM | 4219 | CD | PRO C 121 | -36.411 -64.578 107.029 | 1.00 27.41 | C |
| ATOM | 4220 | N | SER C 122 | -35.465 -66.726 110.991 | 1.00 27.52 | N |
| ATOM | 4221 | CA | SER C 122 | -36.176 -67.066 112.219 | 1.00 26.31 | C |
| ATOM | 4222 | C | SER C 122 | -36.157 -65.874 113.165 | 1.00 30.19 | C |
| ATOM | 4223 | O | SER C 122 | -35.108 -65.263 113.389 | 1.00 33.03 | O |
| ATOM | 4224 | CB | SER C 122 | -35.553 -68.272 112.920 | 1.00 33.10 | C |
| ATOM | 4225 | OG | SER C 122 | -35.607 -69.425 112.115 | 1.00 42.58 | O |
| ATOM | 4226 | N | VAL C 123 | -37.305 -65.555 113.736 | 1.00 30.40 | N |
| ATOM | 4227 | CA | VAL C 123 | -37.439 -64.392 114.599 | 1.00 29.30 | C |
| ATOM | 4228 | C | VAL C 123 | -37.651 -64.870 116.038 | 1.00 28.97 | C |
| ATOM | 4229 | O | VAL C 123 | -38.681 -65.469 116.368 | 1.00 37.16 | O |
| ATOM | 4230 | CB | VAL C 123 | -38.576 -63.481 114.123 | 1.00 27.97 | C |
| ATOM | 4231 | CG1 | VAL C 123 | -38.600 -62.206 114.971 | 1.00 29.32 | C |
| ATOM | 4232 | CG2 | VAL C 123 | -38.448 -63.196 112.593 | 1.00 23.94 | C |
| ATOM | 4233 | N | PHE C 124 | -36.703 -64.598 116.890 | 1.00 27.59 | N |
| ATOM | 4234 | CA | PHE C 124 | -36.791 -64.961 118.289 | 1.00 30.52 | C |
| ATOM | 4235 | C | PHE C 124 | -36.968 -63.708 119.143 | 1.00 32.03 | C |
| ATOM | 4236 | O | PHE C 124 | -36.508 -62.623 118.771 | 1.00 29.31 | O |
| ATOM | 4237 | CB | PHE C 124 | -35.539 -65.723 118.760 | 1.00 34.19 | C |
| ATOM | 4238 | CG | PHE C 124 | -35.145 -66.879 117.868 | 1.00 33.33 | C |
| ATOM | 4239 | CD1 | PHE C 124 | -35.968 -67.985 117.730 | 1.00 37.57 | C |
| ATOM | 4240 | CD2 | PHE C 124 | -33.956 -66.851 117.163 | 1.00 31.04 | C |
| ATOM | 4241 | CE1 | PHE C 124 | -35.618 -69.033 116.892 | 1.00 34.49 | C |
| ATOM | 4242 | CE2 | PHE C 124 | -33.596 -67.901 116.333 | 1.00 34.61 | C |
| ATOM | 4243 | CZ | PHE C 124 | -34.430 -68.991 116.196 | 1.00 33.73 | C |
| ATOM | 4244 | N | PRO C 125 | -37.636 -63.815 120.285 | 1.00 34.51 | N |
| ATOM | 4245 | CA | PRO C 125 | -37.839 -62.633 121.124 | 1.00 30.32 | C |
| ATOM | 4246 | C | PRO C 125 | -36.613 -62.280 121.951 | 1.00 32.51 | C |
| ATOM | 4247 | O | PRO C 125 | -35.832 -63.136 122.369 | 1.00 36.65 | O |
| ATOM | 4248 | CB | PRO C 125 | -39.010 -63.039 122.025 | 1.00 29.69 | C |
| ATOM | 4249 | CG | PRO C 125 | -38.894 -64.526 122.107 | 1.00 32.87 | C |
| ATOM | 4250 | CD | PRO C 125 | -38.363 -64.991 120.794 | 1.00 34.01 | C |
| ATOM | 4251 | N | LEU C 126 | -36.434 -60.985 122.134 | 1.00 26.85 | N |
| ATOM | 4252 | CA | LEU C 126 | -35.595 -60.422 123.175 | 1.00 26.86 | C |
| ATOM | 4253 | C | LEU C 126 | -36.568 -59.902 124.234 | 1.00 31.33 | C |
| ATOM | 4254 | O | LEU C 126 | -37.153 -58.825 124.081 | 1.00 27.81 | O |
| ATOM | 4255 | CB | LEU C 126 | -34.714 -59.324 122.596 | 1.00 31.15 | C |
| ATOM | 4256 | CG | LEU C 126 | -33.839 -59.786 121.435 | 1.00 29.11 | C |

| ATOM | 4257 | CD1 | LEU | C | 126 | -33.179 | -58.601 | 120.808 | 1.00 | 26.98 | C |
|------|------|-----|-----|---|-----|---------|---------|---------|------|-------|---|
| ATOM | 4258 | CD2 | LEU | C | 126 | -32.786 | -60.749 | 121.950 | 1.00 | 28.83 | C |
| ATOM | 4259 | N | ALA | C | 127 | -36.784 | -60.706 | 125.302 | 1.00 | 34.13 | N |
| ATOM | 4260 | CA | ALA | C | 127 | -37.874 | -60.477 | 126.253 | 1.00 | 34.68 | C |
| ATOM | 4261 | C | ALA | C | 127 | -37.515 | -59.383 | 127.258 | 1.00 | 38.36 | C |
| ATOM | 4262 | O | ALA | C | 127 | -36.400 | -59.366 | 127.792 | 1.00 | 34.45 | O |
| ATOM | 4263 | CB | ALA | C | 127 | -38.229 | -61.756 | 126.999 | 1.00 | 34.71 | C |
| ATOM | 4264 | N | PRO | C | 128 | -38.453 | -58.480 | 127.552 | 1.00 | 43.79 | N |
| ATOM | 4265 | CA | PRO | C | 128 | -38.163 | -57.410 | 128.513 | 1.00 | 43.68 | C |
| ATOM | 4266 | C | PRO | C | 128 | -37.732 | -57.972 | 129.856 | 1.00 | 54.93 | C |
| ATOM | 4267 | O | PRO | C | 128 | -38.285 | -58.961 | 130.350 | 1.00 | 50.70 | O |
| ATOM | 4268 | CB | PRO | C | 128 | -39.481 | -56.625 | 128.600 | 1.00 | 40.46 | C |
| ATOM | 4269 | CG | PRO | C | 128 | -40.513 | -57.526 | 128.054 | 1.00 | 44.62 | C |
| ATOM | 4270 | CD | PRO | C | 128 | -39.823 | -58.379 | 127.020 | 1.00 | 37.96 | C |
| ATOM | 4271 | N | SER | C | 129 | -36.668 | -57.356 | 130.389 | 1.00 | 69.23 | N |
| ATOM | 4272 | CA | SER | C | 129 | -36.026 | -57.680 | 131.657 | 1.00 | 73.37 | C |
| ATOM | 4273 | C | SER | C | 129 | -37.065 | -57.798 | 132.767 | 1.00 | 80.23 | C |
| ATOM | 4274 | O | SER | C | 129 | -37.700 | -56.804 | 133.139 | 1.00 | 81.02 | O |
| ATOM | 4275 | CB | SER | C | 129 | -34.983 | -56.595 | 131.987 | 1.00 | 71.73 | C |
| ATOM | 4276 | OG | SER | C | 129 | -34.000 | -57.018 | 132.909 | 1.00 | 63.19 | O |
| ATOM | 4277 | N | SER | C | 130 | -37.246 | -59.018 | 133.289 | 1.00 | 83.03 | N |
| ATOM | 4278 | CA | SER | C | 130 | -38.109 | -59.226 | 134.446 | 1.00 | 85.79 | C |
| ATOM | 4279 | C | SER | C | 130 | -37.714 | -58.318 | 135.603 | 1.00 | 95.78 | C |
| ATOM | 4280 | O | SER | C | 130 | -38.566 | -57.941 | 136.424 | 1.00 | 93.56 | O |
| ATOM | 4281 | CB | SER | C | 130 | -38.040 | -60.696 | 134.882 | 1.00 | 88.42 | C |
| ATOM | 4282 | OG | SER | C | 130 | -36.688 | -61.134 | 135.021 | 1.00 | 84.37 | O |
| ATOM | 4283 | N | LYS | C | 131 | -36.428 | -57.953 | 135.673 | 1.00 | 96.01 | N |
| ATOM | 4284 | CA | LYS | C | 131 | -35.860 | -57.111 | 136.721 | 1.00 | 92.69 | C |
| ATOM | 4285 | C | LYS | C | 131 | -35.737 | -55.631 | 136.334 | 1.00 | 88.64 | C |
| ATOM | 4286 | O | LYS | C | 131 | -34.824 | -54.957 | 136.820 | 1.00 | 93.47 | O |
| ATOM | 4287 | CB | LYS | C | 131 | -34.490 | -57.660 | 137.135 | 1.00 | 85.98 | C |
| ATOM | 4288 | CG | LYS | C | 131 | -34.512 | -59.018 | 137.841 | 1.00 | 83.85 | C |
| ATOM | 4289 | CD | LYS | C | 131 | -33.113 | -59.398 | 138.328 | 1.00 | 86.44 | C |
| ATOM | 4290 | CE | LYS | C | 131 | -33.162 | -60.359 | 139.509 | 1.00 | 81.44 | C |
| ATOM | 4291 | NZ | LYS | C | 131 | -31.802 | -60.691 | 140.021 | 1.00 | 67.72 | N1+ |
| ATOM | 4292 | N | SER | C | 132 | -36.625 | -55.088 | 135.498 | 1.00 | 91.44 | N |
| ATOM | 4293 | CA | SER | C | 132 | -36.567 | -53.651 | 135.224 | 1.00 | 88.79 | C |
| ATOM | 4294 | C | SER | C | 132 | -36.967 | -52.857 | 136.461 | 1.00 | 93.64 | C |
| ATOM | 4295 | O | SER | C | 132 | -37.915 | -53.218 | 137.168 | 1.00 | 97.31 | O |
| ATOM | 4296 | CB | SER | C | 132 | -37.472 | -53.256 | 134.048 | 1.00 | 75.72 | C |
| ATOM | 4297 | OG | SER | C | 132 | -36.862 | -53.513 | 132.797 | 1.00 | 71.83 | O |
| ATOM | 4298 | N | THR | C | 133 | -36.229 | -51.769 | 136.723 | 1.00 | 99.77 | N |
| ATOM | 4299 | CA | THR | C | 133 | -36.569 | -50.845 | 137.806 | 1.00 | 97.90 | C |
| ATOM | 4300 | C | THR | C | 133 | -38.009 | -50.364 | 137.611 | 1.00 | 93.15 | C |
| ATOM | 4301 | O | THR | C | 133 | -38.291 | -49.593 | 136.684 | 1.00 | 89.44 | O |
| ATOM | 4302 | CB | THR | C | 133 | -35.563 | -49.669 | 137.882 | 1.00 | 87.55 | C |
| ATOM | 4303 | OG1 | THR | C | 133 | -35.179 | -49.234 | 136.565 | 1.00 | 83.45 | O |
| ATOM | 4304 | CG2 | THR | C | 133 | -34.303 | -50.077 | 138.653 | 1.00 | 84.85 | C |
| ATOM | 4305 | N | SER | C | 134 | -38.929 | -50.829 | 138.466 | 1.00 | 93.26 | N |
| ATOM | 4306 | CA | SER | C | 134 | -40.352 | -50.635 | 138.210 | 1.00 | 87.74 | C |
| ATOM | 4307 | C | SER | C | 134 | -40.679 | -49.153 | 138.274 | 1.00 | 85.43 | C |
| ATOM | 4308 | O | SER | C | 134 | -40.329 | -48.472 | 139.242 | 1.00 | 87.73 | O |
| ATOM | 4309 | CB | SER | C | 134 | -41.195 | -51.410 | 139.224 | 1.00 | 74.42 | C |
| ATOM | 4310 | OG | SER | C | 134 | -42.211 | -52.150 | 138.567 | 1.00 | 81.41 | O |
| ATOM | 4311 | N | GLY | C | 135 | -41.314 | -48.643 | 137.223 | 1.00 | 80.04 | N |
| ATOM | 4312 | CA | GLY | C | 135 | -41.395 | -47.211 | 137.051 | 1.00 | 78.15 | C |
| ATOM | 4313 | C | GLY | C | 135 | -40.279 | -46.614 | 136.227 | 1.00 | 80.05 | C |
| ATOM | 4314 | O | GLY | C | 135 | -40.186 | -45.381 | 136.144 | 1.00 | 77.05 | O |
| ATOM | 4315 | N | GLY | C | 136 | -39.414 | -47.444 | 135.639 | 1.00 | 72.87 | N |
| ATOM | 4316 | CA | GLY | C | 136 | -38.350 | -46.962 | 134.783 | 1.00 | 61.44 | C |
| ATOM | 4317 | C | GLY | C | 136 | -38.489 | -47.391 | 133.333 | 1.00 | 56.58 | C |
| ATOM | 4318 | O | GLY | C | 136 | -39.592 | -47.407 | 132.771 | 1.00 | 51.45 | O |
| ATOM | 4319 | N | THR | C | 137 | -37.382 | -47.773 | 132.713 | 1.00 | 53.14 | N |
| ATOM | 4320 | CA | THR | C | 137 | -37.361 | -48.017 | 131.279 | 1.00 | 47.91 | C |
| ATOM | 4321 | C | THR | C | 137 | -37.015 | -49.473 | 131.008 | 1.00 | 48.48 | C |
| ATOM | 4322 | O | THR | C | 137 | -36.090 | -50.028 | 131.617 | 1.00 | 47.97 | O |
| ATOM | 4323 | CB | THR | C | 137 | -36.370 | -47.081 | 130.592 | 1.00 | 48.09 | C |
| ATOM | 4324 | OG1 | THR | C | 137 | -36.886 | -45.745 | 130.650 | 1.00 | 54.06 | O |
| ATOM | 4325 | CG2 | THR | C | 137 | -36.140 | -47.485 | 129.123 | 1.00 | 43.01 | C |
| ATOM | 4326 | N | ALA | C | 138 | -37.781 | -50.087 | 130.110 | 1.00 | 38.89 | N |
| ATOM | 4327 | CA | ALA | C | 138 | -37.598 | -51.469 | 129.714 | 1.00 | 38.16 | C |

```
ATOM   4328  C    ALA C 138    -37.117 -51.516 128.268  1.00 36.59          C
ATOM   4329  O    ALA C 138    -37.541 -50.708 127.437  1.00 36.87          O
ATOM   4330  CB   ALA C 138    -38.908 -52.249 129.871  1.00 31.25          C
ATOM   4331  N    ALA C 139    -36.232 -52.454 127.964  1.00 33.10          N
ATOM   4332  CA   ALA C 139    -35.886 -52.755 126.582  1.00 30.90          C
ATOM   4333  C    ALA C 139    -36.522 -54.080 126.199  1.00 30.77          C
ATOM   4334  O    ALA C 139    -36.619 -54.986 127.028  1.00 31.77          O
ATOM   4335  CB   ALA C 139    -34.371 -52.823 126.376  1.00 25.95          C
ATOM   4336  N    LEU C 140    -36.978 -54.176 124.952  1.00 29.69          N
ATOM   4337  CA   LEU C 140    -37.426 -55.439 124.376  1.00 30.12          C
ATOM   4338  C    LEU C 140    -37.081 -55.420 122.891  1.00 30.17          C
ATOM   4339  O    LEU C 140    -36.795 -54.369 122.319  1.00 29.86          O
ATOM   4340  CB   LEU C 140    -38.930 -55.666 124.605  1.00 29.80          C
ATOM   4341  CG   LEU C 140    -39.844 -54.591 124.028  1.00 30.15          C
ATOM   4342  CD1  LEU C 140    -40.427 -55.049 122.720  1.00 29.13          C
ATOM   4343  CD2  LEU C 140    -40.945 -54.256 125.007  1.00 30.43          C
ATOM   4344  N    GLY C 141    -37.135 -56.581 122.250  1.00 27.97          N
ATOM   4345  CA   GLY C 141    -36.809 -56.584 120.844  1.00 27.01          C
ATOM   4346  C    GLY C 141    -37.043 -57.919 120.174  1.00 30.67          C
ATOM   4347  O    GLY C 141    -37.628 -58.835 120.754  1.00 31.90          O
ATOM   4348  N    CYS C 142    -36.604 -57.978 118.908  1.00 28.16          N
ATOM   4349  CA   CYS C 142    -36.633 -59.156 118.051  1.00 31.74          C
ATOM   4350  C    CYS C 142    -35.238 -59.467 117.527  1.00 28.43          C
ATOM   4351  O    CYS C 142    -34.478 -58.563 117.193  1.00 24.09          O
ATOM   4352  CB   CYS C 142    -37.563 -58.947 116.858  1.00 32.06          C
ATOM   4353  SG   CYS C 142    -39.293 -59.136 117.287  1.00 49.90          S
ATOM   4354  N    LEU C 143    -34.903 -60.747 117.447  1.00 28.84          N
ATOM   4355  CA   LEU C 143    -33.651 -61.187 116.845  1.00 26.55          C
ATOM   4356  C    LEU C 143    -33.989 -61.904 115.550  1.00 27.72          C
ATOM   4357  O    LEU C 143    -34.719 -62.892 115.562  1.00 29.16          O
ATOM   4358  CB   LEU C 143    -32.849 -62.069 117.805  1.00 29.85          C
ATOM   4359  CG   LEU C 143    -31.504 -62.623 117.312  1.00 31.81          C
ATOM   4360  CD1  LEU C 143    -30.634 -61.539 116.821  1.00 29.50          C
ATOM   4361  CD2  LEU C 143    -30.809 -63.273 118.476  1.00 34.71          C
ATOM   4362  N    VAL C 144    -33.506 -61.374 114.434  1.00 24.21          N
ATOM   4363  CA   VAL C 144    -33.850 -61.888 113.121  1.00 25.88          C
ATOM   4364  C    VAL C 144    -32.632 -62.683 112.650  1.00 31.73          C
ATOM   4365  O    VAL C 144    -31.676 -62.122 112.104  1.00 27.35          O
ATOM   4366  CB   VAL C 144    -34.237 -60.756 112.162  1.00 29.39          C
ATOM   4367  CG1  VAL C 144    -34.620 -61.297 110.801  1.00 29.56          C
ATOM   4368  CG2  VAL C 144    -35.395 -59.939 112.721  1.00 21.42          C
ATOM   4369  N    LYS C 145    -32.658 -64.003 112.849  1.00 26.44          N
ATOM   4370  CA   LYS C 145    -31.455 -64.811 112.726  1.00 29.06          C
ATOM   4371  C    LYS C 145    -31.472 -65.687 111.476  1.00 30.53          C
ATOM   4372  O    LYS C 145    -32.513 -66.244 111.098  1.00 29.40          O
ATOM   4373  CB   LYS C 145    -31.253 -65.686 113.974  1.00 29.44          C
ATOM   4374  CG   LYS C 145    -29.832 -66.217 114.070  1.00 30.84          C
ATOM   4375  CD   LYS C 145    -29.511 -66.818 115.405  1.00 37.83          C
ATOM   4376  CE   LYS C 145    -28.009 -67.086 115.543  1.00 43.25          C
ATOM   4377  NZ   LYS C 145    -27.433 -67.955 114.471  1.00 39.52          N1+
ATOM   4378  N    ASP C 146    -30.298 -65.782 110.837  1.00 28.14          N
ATOM   4379  CA   ASP C 146    -30.003 -66.758 109.784  1.00 33.06          C
ATOM   4380  C    ASP C 146    -30.862 -66.564 108.531  1.00 33.58          C
ATOM   4381  O    ASP C 146    -31.584 -67.465 108.102  1.00 36.23          O
ATOM   4382  CB   ASP C 146    -30.185 -68.177 110.330  1.00 36.19          C
ATOM   4383  CG   ASP C 146    -29.226 -68.502 111.441  1.00 38.83          C
ATOM   4384  OD1  ASP C 146    -28.041 -68.058 111.404  1.00 37.01          O
ATOM   4385  OD2  ASP C 146    -29.716 -69.135 112.405  1.00 41.05          O1-
ATOM   4386  N    TYR C 147    -30.735 -65.405 107.905  1.00 29.04          N
ATOM   4387  CA   TYR C 147    -31.400 -65.202 106.626  1.00 29.40          C
ATOM   4388  C    TYR C 147    -30.361 -64.899 105.547  1.00 29.62          C
ATOM   4389  O    TYR C 147    -29.186 -64.645 105.826  1.00 30.41          O
ATOM   4390  CB   TYR C 147    -32.443 -64.080 106.711  1.00 26.97          C
ATOM   4391  CG   TYR C 147    -31.863 -62.715 106.999  1.00 28.14          C
ATOM   4392  CD1  TYR C 147    -31.628 -62.304 108.303  1.00 28.44          C
ATOM   4393  CD2  TYR C 147    -31.529 -61.840 105.963  1.00 29.70          C
ATOM   4394  CE1  TYR C 147    -31.098 -61.071 108.575  1.00 30.16          C
ATOM   4395  CE2  TYR C 147    -30.994 -60.586 106.226  1.00 29.35          C
ATOM   4396  CZ   TYR C 147    -30.788 -60.204 107.538  1.00 32.07          C
ATOM   4397  OH   TYR C 147    -30.269 -58.954 107.832  1.00 32.73          O
ATOM   4398  N    PHE C 148    -30.808 -64.941 104.298  1.00 31.56          N
```

```
ATOM   4399  CA  PHE C 148     -29.950 -64.658 103.161  1.00 31.89           C
ATOM   4400  C   PHE C 148     -30.781 -64.535 101.900  1.00 31.76           C
ATOM   4401  O   PHE C 148     -31.634 -65.367 101.661  1.00 30.72           O
ATOM   4402  CB  PHE C 148     -28.910 -65.765 102.977  1.00 30.49           C
ATOM   4403  CG  PHE C 148     -27.980 -65.524 101.839  1.00 32.65           C
ATOM   4404  CD1 PHE C 148     -28.316 -65.923 100.550  1.00 30.37           C
ATOM   4405  CD2 PHE C 148     -26.766 -64.876 102.053  1.00 31.85           C
ATOM   4406  CE1 PHE C 148     -27.460 -65.681  99.502  1.00 33.92           C
ATOM   4407  CE2 PHE C 148     -25.903 -64.629 101.010  1.00 31.05           C
ATOM   4408  CZ  PHE C 148     -26.244 -65.032  99.734  1.00 36.04           C
ATOM   4409  N   PRO C 149     -30.503 -63.514 101.066  1.00 37.13           N
ATOM   4410  CA  PRO C 149     -29.514 -62.453 101.301  1.00 34.36           C
ATOM   4411  C   PRO C 149     -30.136 -61.270 102.025  1.00 31.13           C
ATOM   4412  O   PRO C 149     -31.294 -61.362 102.414  1.00 33.18           O
ATOM   4413  CB  PRO C 149     -29.116 -62.050  99.885  1.00 28.91           C
ATOM   4414  CG  PRO C 149     -30.384 -62.167  99.146  1.00 26.99           C
ATOM   4415  CD  PRO C 149     -31.093 -63.390  99.717  1.00 27.60           C
ATOM   4416  N   GLU C 150     -29.394 -60.176 102.157  1.00 27.31           N
ATOM   4417  CA  GLU C 150     -29.961 -58.888 102.552  1.00 30.44           C
ATOM   4418  C   GLU C 150     -30.938 -58.398 101.479  1.00 29.76           C
ATOM   4419  O   GLU C 150     -30.806 -58.770 100.312  1.00 30.40           O
ATOM   4420  CB  GLU C 150     -28.839 -57.872 102.761  1.00 29.87           C
ATOM   4421  CG  GLU C 150     -27.928 -58.160 103.958  1.00 29.34           C
ATOM   4422  CD  GLU C 150     -28.365 -57.400 105.210  1.00 38.78           C
ATOM   4423  OE1 GLU C 150     -27.573 -56.543 105.699  1.00 37.11           O
ATOM   4424  OE2 GLU C 150     -29.512 -57.629 105.680  1.00 39.42           O1-
ATOM   4425  N   PRO C 151     -31.930 -57.571 101.855  1.00 28.20           N
ATOM   4426  CA  PRO C 151     -32.224 -57.019 103.180  1.00 28.76           C
ATOM   4427  C   PRO C 151     -33.460 -57.594 103.883  1.00 32.34           C
ATOM   4428  O   PRO C 151     -34.284 -58.288 103.291  1.00 27.74           O
ATOM   4429  CB  PRO C 151     -32.488 -55.554 102.867  1.00 21.65           C
ATOM   4430  CG  PRO C 151     -33.145 -55.605 101.554  1.00 15.86           C
ATOM   4431  CD  PRO C 151     -32.640 -56.807 100.810  1.00 20.18           C
ATOM   4432  N   VAL C 152     -33.572 -57.269 105.165  1.00 31.09           N
ATOM   4433  CA  VAL C 152     -34.798 -57.431 105.925  1.00 31.79           C
ATOM   4434  C   VAL C 152     -35.215 -56.053 106.399  1.00 32.70           C
ATOM   4435  O   VAL C 152     -34.378 -55.174 106.625  1.00 38.99           O
ATOM   4436  CB  VAL C 152     -34.616 -58.366 107.130  1.00 32.39           C
ATOM   4437  CG1 VAL C 152     -34.480 -59.798 106.688  1.00 34.75           C
ATOM   4438  CG2 VAL C 152     -33.386 -57.962 107.854  1.00 32.91           C
ATOM   4439  N   THR C 153     -36.513 -55.863 106.552  1.00 30.59           N
ATOM   4440  CA  THR C 153     -37.032 -54.660 107.178  1.00 33.61           C
ATOM   4441  C   THR C 153     -37.799 -55.060 108.426  1.00 32.20           C
ATOM   4442  O   THR C 153     -38.455 -56.108 108.450  1.00 30.80           O
ATOM   4443  CB  THR C 153     -37.918 -53.848 106.212  1.00 32.33           C
ATOM   4444  OG1 THR C 153     -38.950 -54.679 105.683  1.00 37.08           O
ATOM   4445  CG2 THR C 153     -37.089 -53.315 105.061  1.00 33.42           C
ATOM   4446  N   VAL C 154     -37.709 -54.221 109.459  1.00 27.86           N
ATOM   4447  CA  VAL C 154     -38.401 -54.438 110.727  1.00 29.82           C
ATOM   4448  C   VAL C 154     -39.162 -53.168 111.090  1.00 26.12           C
ATOM   4449  O   VAL C 154     -38.574 -52.085 111.121  1.00 30.95           O
ATOM   4450  CB  VAL C 154     -37.414 -54.808 111.861  1.00 29.46           C
ATOM   4451  CG1 VAL C 154     -38.155 -55.064 113.142  1.00 29.26           C
ATOM   4452  CG2 VAL C 154     -36.547 -56.023 111.485  1.00 24.20           C
ATOM   4453  N   SER C 155     -40.459 -53.293 111.373  1.00 28.07           N
ATOM   4454  CA  SER C 155     -41.207 -52.221 112.033  1.00 29.61           C
ATOM   4455  C   SER C 155     -41.832 -52.750 113.315  1.00 35.01           C
ATOM   4456  O   SER C 155     -41.790 -53.949 113.616  1.00 36.17           O
ATOM   4457  CB  SER C 155     -42.310 -51.632 111.151  1.00 25.87           C
ATOM   4458  OG  SER C 155     -43.302 -52.591 110.859  1.00 28.75           O
ATOM   4459  N   TRP C 156     -42.424 -51.846 114.080  1.00 29.64           N
ATOM   4460  CA  TRP C 156     -43.029 -52.229 115.344  1.00 33.60           C
ATOM   4461  C   TRP C 156     -44.490 -51.793 115.369  1.00 34.15           C
ATOM   4462  O   TRP C 156     -44.813 -50.652 115.017  1.00 33.36           O
ATOM   4463  CB  TRP C 156     -42.226 -51.655 116.514  1.00 31.35           C
ATOM   4464  CG  TRP C 156     -40.969 -52.463 116.781  1.00 31.98           C
ATOM   4465  CD1 TRP C 156     -39.734 -52.302 116.196  1.00 33.14           C
ATOM   4466  CD2 TRP C 156     -40.836 -53.564 117.686  1.00 30.45           C
ATOM   4467  NE1 TRP C 156     -38.849 -53.230 116.690  1.00 25.96           N
ATOM   4468  CE2 TRP C 156     -39.498 -54.008 117.616  1.00 30.62           C
ATOM   4469  CE3 TRP C 156     -41.717 -54.213 118.566  1.00 32.88           C
```

```
ATOM   4470  CZ2 TRP C 156     -39.025 -55.076 118.391  1.00 31.18          C
ATOM   4471  CZ3 TRP C 156     -41.243 -55.274 119.341  1.00 27.61          C
ATOM   4472  CH2 TRP C 156     -39.914 -55.694 119.242  1.00 30.88          C
ATOM   4473  N   ASN C 157     -45.370 -52.719 115.757  1.00 34.57          N
ATOM   4474  CA  ASN C 157     -46.805 -52.459 115.826  1.00 28.72          C
ATOM   4475  C   ASN C 157     -47.299 -51.895 114.495  1.00 36.26          C
ATOM   4476  O   ASN C 157     -47.997 -50.880 114.432  1.00 35.07          O
ATOM   4477  CB  ASN C 157     -47.133 -51.515 116.989  1.00 31.99          C
ATOM   4478  CG  ASN C 157     -46.897 -52.156 118.368  1.00 33.31          C
ATOM   4479  OD1 ASN C 157     -46.496 -53.318 118.479  1.00 34.80          O
ATOM   4480  ND2 ASN C 157     -47.136 -51.387 119.418  1.00 32.04          N
ATOM   4481  N   SER C 158     -46.870 -52.540 113.410  1.00 39.26          N
ATOM   4482  CA  SER C 158     -47.310 -52.190 112.062  1.00 36.22          C
ATOM   4483  C   SER C 158     -46.991 -50.739 111.719  1.00 35.47          C
ATOM   4484  O   SER C 158     -47.673 -50.119 110.908  1.00 39.31          O
ATOM   4485  CB  SER C 158     -48.807 -52.459 111.895  1.00 33.86          C
ATOM   4486  OG  SER C 158     -49.145 -53.743 112.399  1.00 43.34          O
ATOM   4487  N   GLY C 159     -45.938 -50.192 112.306  1.00 35.45          N
ATOM   4488  CA  GLY C 159     -45.566 -48.813 112.078  1.00 33.17          C
ATOM   4489  C   GLY C 159     -46.053 -47.833 113.124  1.00 36.82          C
ATOM   4490  O   GLY C 159     -45.608 -46.684 113.114  1.00 41.61          O
ATOM   4491  N   ALA C 160     -46.919 -48.260 114.048  1.00 38.45          N
ATOM   4492  CA  ALA C 160     -47.469 -47.347 115.047  1.00 32.99          C
ATOM   4493  C   ALA C 160     -46.449 -46.967 116.108  1.00 41.51          C
ATOM   4494  O   ALA C 160     -46.566 -45.903 116.718  1.00 49.03          O
ATOM   4495  CB  ALA C 160     -48.695 -47.977 115.719  1.00 29.64          C
ATOM   4496  N   LEU C 161     -45.463 -47.818 116.362  1.00 40.25          N
ATOM   4497  CA  LEU C 161     -44.450 -47.562 117.372  1.00 30.64          C
ATOM   4498  C   LEU C 161     -43.161 -47.174 116.659  1.00 33.32          C
ATOM   4499  O   LEU C 161     -42.702 -47.877 115.757  1.00 31.89          O
ATOM   4500  CB  LEU C 161     -44.269 -48.786 118.270  1.00 31.29          C
ATOM   4501  CG  LEU C 161     -43.229 -48.759 119.395  1.00 38.45          C
ATOM   4502  CD1 LEU C 161     -43.249 -47.451 120.164  1.00 23.88          C
ATOM   4503  CD2 LEU C 161     -43.494 -49.922 120.328  1.00 32.82          C
ATOM   4504  N   THR C 162     -42.606 -46.032 117.032  1.00 37.42          N
ATOM   4505  CA  THR C 162     -41.535 -45.418 116.262  1.00 33.06          C
ATOM   4506  C   THR C 162     -40.497 -44.859 117.219  1.00 29.92          C
ATOM   4507  O   THR C 162     -39.296 -45.096 117.071  1.00 28.52          O
ATOM   4508  CB  THR C 162     -42.139 -44.328 115.362  1.00 36.32          C
ATOM   4509  OG1 THR C 162     -42.104 -44.761 113.998  1.00 34.07          O
ATOM   4510  CG2 THR C 162     -41.443 -42.968 115.527  1.00 36.07          C
ATOM   4511  N   SER C 163     -40.981 -44.153 118.234  1.00 30.45          N
ATOM   4512  CA  SER C 163     -40.121 -43.634 119.272  1.00 26.52          C
ATOM   4513  C   SER C 163     -39.452 -44.780 120.014  1.00 30.83          C
ATOM   4514  O   SER C 163     -40.117 -45.724 120.461  1.00 32.58          O
ATOM   4515  CB  SER C 163     -40.954 -42.786 120.226  1.00 28.23          C
ATOM   4516  OG  SER C 163     -40.230 -42.404 121.384  1.00 40.32          O
ATOM   4517  N   GLY C 164     -38.137 -44.673 120.178  1.00 30.58          N
ATOM   4518  CA  GLY C 164     -37.364 -45.625 120.941  1.00 29.23          C
ATOM   4519  C   GLY C 164     -36.839 -46.804 120.153  1.00 31.45          C
ATOM   4520  O   GLY C 164     -36.059 -47.589 120.704  1.00 33.69          O
ATOM   4521  N   VAL C 165     -37.207 -46.930 118.878  1.00 27.51          N
ATOM   4522  CA  VAL C 165     -36.793 -48.069 118.070  1.00 28.93          C
ATOM   4523  C   VAL C 165     -35.357 -47.887 117.587  1.00 28.12          C
ATOM   4524  O   VAL C 165     -34.979 -46.831 117.081  1.00 37.17          O
ATOM   4525  CB  VAL C 165     -37.748 -48.269 116.884  1.00 26.69          C
ATOM   4526  CG1 VAL C 165     -37.278 -49.432 116.023  1.00 24.04          C
ATOM   4527  CG2 VAL C 165     -39.163 -48.502 117.367  1.00 24.09          C
ATOM   4528  N   HIS C 166     -34.566 -48.942 117.693  1.00 30.28          N
ATOM   4529  CA  HIS C 166     -33.290 -49.052 116.999  1.00 27.00          C
ATOM   4530  C   HIS C 166     -33.270 -50.379 116.259  1.00 23.79          C
ATOM   4531  O   HIS C 166     -33.286 -51.436 116.895  1.00 25.39          O
ATOM   4532  CB  HIS C 166     -32.105 -49.007 117.975  1.00 27.83          C
ATOM   4533  CG  HIS C 166     -32.009 -47.759 118.809  1.00 31.08          C
ATOM   4534  ND1 HIS C 166     -31.757 -46.516 118.271  1.00 32.94          N
ATOM   4535  CD2 HIS C 166     -32.070 -47.578 120.153  1.00 30.56          C
ATOM   4536  CE1 HIS C 166     -31.681 -45.622 119.242  1.00 30.93          C
ATOM   4537  NE2 HIS C 166     -31.861 -46.242 120.395  1.00 27.04          N
ATOM   4538  N   THR C 167     -33.209 -50.338 114.928  1.00 24.71          N
ATOM   4539  CA  THR C 167     -32.896 -51.526 114.139  1.00 22.25          C
ATOM   4540  C   THR C 167     -31.455 -51.439 113.637  1.00 24.53          C
```

```
ATOM   4541  O    THR C 167    -31.093 -50.498 112.927  1.00 26.79           O
ATOM   4542  CB   THR C 167    -33.857 -51.694 112.980  1.00 20.41           C
ATOM   4543  OG1  THR C 167    -35.176 -51.829 113.501  1.00 28.97           O
ATOM   4544  CG2  THR C 167    -33.529 -52.967 112.219  1.00 21.90           C
ATOM   4545  N    PHE C 168    -30.658 -52.417 113.986  1.00 25.58           N
ATOM   4546  CA   PHE C 168    -29.210 -52.471 113.865  1.00 26.05           C
ATOM   4547  C    PHE C 168    -28.807 -53.018 112.505  1.00 21.09           C
ATOM   4548  O    PHE C 168    -29.560 -53.760 111.890  1.00 25.00           O
ATOM   4549  CB   PHE C 168    -28.623 -53.352 114.969  1.00 23.55           C
ATOM   4550  CG   PHE C 168    -28.681 -52.722 116.316  1.00 22.06           C
ATOM   4551  CD1  PHE C 168    -29.812 -52.855 117.113  1.00 23.76           C
ATOM   4552  CD2  PHE C 168    -27.617 -51.958 116.788  1.00 24.14           C
ATOM   4553  CE1  PHE C 168    -29.892 -52.232 118.390  1.00 24.66           C
ATOM   4554  CE2  PHE C 168    -27.683 -51.341 118.064  1.00 28.11           C
ATOM   4555  CZ   PHE C 168    -28.828 -51.482 118.866  1.00 22.06           C
ATOM   4556  N    PRO C 169    -27.626 -52.662 112.009  1.00 26.60           N
ATOM   4557  CA   PRO C 169    -27.121 -53.295 110.778  1.00 25.79           C
ATOM   4558  C    PRO C 169    -26.907 -54.793 110.988  1.00 26.33           C
ATOM   4559  O    PRO C 169    -26.457 -55.228 112.050  1.00 23.48           O
ATOM   4560  CB   PRO C 169    -25.790 -52.569 110.525  1.00 17.06           C
ATOM   4561  CG   PRO C 169    -25.829 -51.348 111.350  1.00 17.09           C
ATOM   4562  CD   PRO C 169    -26.686 -51.660 112.546  1.00 22.12           C
ATOM   4563  N    ALA C 170    -27.222 -55.586 109.966  1.00 23.48           N
ATOM   4564  CA   ALA C 170    -27.009 -57.027 110.064  1.00 26.56           C
ATOM   4565  C    ALA C 170    -25.523 -57.369 110.178  1.00 25.54           C
ATOM   4566  O    ALA C 170    -24.650 -56.604 109.782  1.00 26.69           O
ATOM   4567  CB   ALA C 170    -27.591 -57.744 108.847  1.00 29.48           C
ATOM   4568  N    VAL C 171    -25.237 -58.531 110.747  1.00 26.07           N
ATOM   4569  CA   VAL C 171    -23.899 -59.104 110.683  1.00 24.19           C
ATOM   4570  C    VAL C 171    -23.934 -60.333 109.789  1.00 26.45           C
ATOM   4571  O    VAL C 171    -24.935 -61.047 109.711  1.00 31.14           O
ATOM   4572  CB   VAL C 171    -23.315 -59.455 112.072  1.00 29.65           C
ATOM   4573  CG1  VAL C 171    -23.042 -58.193 112.858  1.00 27.11           C
ATOM   4574  CG2  VAL C 171    -24.254 -60.404 112.853  1.00 25.85           C
ATOM   4575  N    LEU C 172    -22.832 -60.571 109.101  1.00 31.64           N
ATOM   4576  CA   LEU C 172    -22.658 -61.767 108.288  1.00 28.57           C
ATOM   4577  C    LEU C 172    -21.868 -62.750 109.143  1.00 26.67           C
ATOM   4578  O    LEU C 172    -20.718 -62.492 109.489  1.00 30.87           O
ATOM   4579  CB   LEU C 172    -21.937 -61.442 106.982  1.00 27.30           C
ATOM   4580  CG   LEU C 172    -21.662 -62.583 105.992  1.00 32.63           C
ATOM   4581  CD1  LEU C 172    -22.971 -63.245 105.549  1.00 26.83           C
ATOM   4582  CD2  LEU C 172    -20.839 -62.101 104.772  1.00 28.21           C
ATOM   4583  N    GLN C 173    -22.503 -63.845 109.522  1.00 27.90           N
ATOM   4584  CA   GLN C 173    -21.916 -64.862 110.372  1.00 32.35           C
ATOM   4585  C    GLN C 173    -21.087 -65.824 109.528  1.00 36.44           C
ATOM   4586  O    GLN C 173    -21.204 -65.870 108.294  1.00 34.30           O
ATOM   4587  CB   GLN C 173    -23.010 -65.632 111.111  1.00 33.56           C
ATOM   4588  CG   GLN C 173    -23.996 -64.769 111.851  1.00 29.57           C
ATOM   4589  CD   GLN C 173    -25.319 -65.478 112.079  1.00 34.07           C
ATOM   4590  OE1  GLN C 173    -25.724 -65.696 113.215  1.00 34.19           O
ATOM   4591  NE2  GLN C 173    -26.002 -65.836 110.991  1.00 32.53           N
ATOM   4592  N    SER C 174    -20.254 -66.622 110.208  1.00 36.04           N
ATOM   4593  CA   SER C 174    -19.363 -67.525 109.481  1.00 33.82           C
ATOM   4594  C    SER C 174    -20.138 -68.509 108.622  1.00 32.65           C
ATOM   4595  O    SER C 174    -19.561 -69.087 107.697  1.00 35.26           O
ATOM   4596  CB   SER C 174    -18.437 -68.272 110.441  1.00 31.17           C
ATOM   4597  OG   SER C 174    -18.868 -68.133 111.788  1.00 51.22           O
ATOM   4598  N    SER C 175    -21.438 -68.678 108.879  1.00 30.00           N
ATOM   4599  CA   SER C 175    -22.267 -69.541 108.055  1.00 23.99           C
ATOM   4600  C    SER C 175    -22.566 -68.950 106.691  1.00 33.95           C
ATOM   4601  O    SER C 175    -23.018 -69.689 105.810  1.00 36.33           O
ATOM   4602  CB   SER C 175    -23.588 -69.851 108.767  1.00 31.25           C
ATOM   4603  OG   SER C 175    -24.482 -68.743 108.809  1.00 33.60           O
ATOM   4604  N    GLY C 176    -22.299 -67.659 106.473  1.00 34.11           N
ATOM   4605  CA   GLY C 176    -22.780 -67.006 105.275  1.00 26.57           C
ATOM   4606  C    GLY C 176    -24.191 -66.479 105.380  1.00 33.77           C
ATOM   4607  O    GLY C 176    -24.690 -65.897 104.409  1.00 34.08           O
ATOM   4608  N    LEU C 177    -24.857 -66.682 106.515  1.00 32.19           N
ATOM   4609  CA   LEU C 177    -26.185 -66.145 106.765  1.00 30.75           C
ATOM   4610  C    LEU C 177    -26.072 -64.870 107.584  1.00 33.31           C
ATOM   4611  O    LEU C 177    -25.122 -64.683 108.347  1.00 33.18           O
```

| ATOM | 4612 | CB  | LEU | C | 177 | -27.062 | -67.141 | 107.512 | 1.00 | 35.99 | C |
| ATOM | 4613 | CG  | LEU | C | 177 | -27.316 | -68.487 | 106.843 | 1.00 | 34.64 | C |
| ATOM | 4614 | CD1 | LEU | C | 177 | -28.208 | -69.291 | 107.732 | 1.00 | 30.71 | C |
| ATOM | 4615 | CD2 | LEU | C | 177 | -27.932 | -68.291 | 105.469 | 1.00 | 29.23 | C |
| ATOM | 4616 | N   | TYR | C | 178 | -27.048 | -63.992 | 107.413 | 1.00 | 32.29 | N |
| ATOM | 4617 | CA  | TYR | C | 178 | -27.076 | -62.736 | 108.138 | 1.00 | 29.56 | C |
| ATOM | 4618 | C   | TYR | C | 178 | -27.923 | -62.834 | 109.408 | 1.00 | 32.53 | C |
| ATOM | 4619 | O   | TYR | C | 178 | -28.819 | -63.665 | 109.535 | 1.00 | 33.61 | O |
| ATOM | 4620 | CB  | TYR | C | 178 | -27.616 | -61.627 | 107.249 | 1.00 | 26.03 | C |
| ATOM | 4621 | CG  | TYR | C | 178 | -26.708 | -61.260 | 106.096 | 1.00 | 32.82 | C |
| ATOM | 4622 | CD1 | TYR | C | 178 | -25.696 | -60.309 | 106.248 | 1.00 | 25.25 | C |
| ATOM | 4623 | CD2 | TYR | C | 178 | -26.874 | -61.855 | 104.843 | 1.00 | 28.59 | C |
| ATOM | 4624 | CE1 | TYR | C | 178 | -24.890 | -59.978 | 105.188 | 1.00 | 27.51 | C |
| ATOM | 4625 | CE2 | TYR | C | 178 | -26.075 | -61.526 | 103.785 | 1.00 | 25.14 | C |
| ATOM | 4626 | CZ  | TYR | C | 178 | -25.085 | -60.589 | 103.952 | 1.00 | 33.10 | C |
| ATOM | 4627 | OH  | TYR | C | 178 | -24.299 | -60.260 | 102.873 | 1.00 | 31.54 | O |
| ATOM | 4628 | N   | SER | C | 179 | -27.674 | -61.906 | 110.316 | 1.00 | 30.12 | N |
| ATOM | 4629 | CA  | SER | C | 179 | -28.377 | -61.836 | 111.580 | 1.00 | 26.37 | C |
| ATOM | 4630 | C   | SER | C | 179 | -28.484 | -60.373 | 111.974 | 1.00 | 28.91 | C |
| ATOM | 4631 | O   | SER | C | 179 | -27.473 | -59.669 | 112.014 | 1.00 | 33.49 | O |
| ATOM | 4632 | CB  | SER | C | 179 | -27.616 | -62.615 | 112.653 | 1.00 | 31.79 | C |
| ATOM | 4633 | OG  | SER | C | 179 | -28.434 | -63.550 | 113.310 | 1.00 | 36.87 | O |
| ATOM | 4634 | N   | LEU | C | 180 | -29.693 | -59.908 | 112.244 | 1.00 | 24.41 | N |
| ATOM | 4635 | CA  | LEU | C | 180 | -29.847 | -58.581 | 112.796 | 1.00 | 26.13 | C |
| ATOM | 4636 | C   | LEU | C | 180 | -30.787 | -58.623 | 113.991 | 1.00 | 33.77 | C |
| ATOM | 4637 | O   | LEU | C | 180 | -31.507 | -59.597 | 114.238 | 1.00 | 33.18 | O |
| ATOM | 4638 | CB  | LEU | C | 180 | -30.340 | -57.570 | 111.757 | 1.00 | 32.63 | C |
| ATOM | 4639 | CG  | LEU | C | 180 | -31.742 | -57.362 | 111.177 | 1.00 | 31.41 | C |
| ATOM | 4640 | CD1 | LEU | C | 180 | -32.886 | -57.085 | 112.196 | 1.00 | 23.59 | C |
| ATOM | 4641 | CD2 | LEU | C | 180 | -31.574 | -56.181 | 110.212 | 1.00 | 23.09 | C |
| ATOM | 4642 | N   | SER | C | 181 | -30.773 | -57.531 | 114.735 | 1.00 | 33.23 | N |
| ATOM | 4643 | CA  | SER | C | 181 | -31.662 | -57.356 | 115.859 | 1.00 | 28.03 | C |
| ATOM | 4644 | C   | SER | C | 181 | -32.311 | -55.982 | 115.766 | 1.00 | 26.07 | C |
| ATOM | 4645 | O   | SER | C | 181 | -31.777 | -55.063 | 115.154 | 1.00 | 28.63 | O |
| ATOM | 4646 | CB  | SER | C | 181 | -30.906 | -57.540 | 117.184 | 1.00 | 30.44 | C |
| ATOM | 4647 | OG  | SER | C | 181 | -29.984 | -56.493 | 117.391 | 1.00 | 31.22 | O |
| ATOM | 4648 | N   | SER | C | 182 | -33.506 | -55.881 | 116.326 | 1.00 | 27.13 | N |
| ATOM | 4649 | CA  | SER | C | 182 | -34.271 | -54.649 | 116.409 | 1.00 | 27.94 | C |
| ATOM | 4650 | C   | SER | C | 182 | -34.805 | -54.551 | 117.827 | 1.00 | 28.64 | C |
| ATOM | 4651 | O   | SER | C | 182 | -35.302 | -55.543 | 118.363 | 1.00 | 26.17 | O |
| ATOM | 4652 | CB  | SER | C | 182 | -35.424 | -54.637 | 115.408 | 1.00 | 25.46 | C |
| ATOM | 4653 | OG  | SER | C | 182 | -36.221 | -53.487 | 115.599 | 1.00 | 27.32 | O |
| ATOM | 4654 | N   | VAL | C | 183 | -34.683 | -53.373 | 118.446 | 1.00 | 27.27 | N |
| ATOM | 4655 | CA  | VAL | C | 183 | -35.089 | -53.201 | 119.837 | 1.00 | 30.94 | C |
| ATOM | 4656 | C   | VAL | C | 183 | -35.878 | -51.909 | 120.005 | 1.00 | 30.82 | C |
| ATOM | 4657 | O   | VAL | C | 183 | -35.875 | -51.032 | 119.142 | 1.00 | 33.72 | O |
| ATOM | 4658 | CB  | VAL | C | 183 | -33.886 | -53.209 | 120.800 | 1.00 | 27.96 | C |
| ATOM | 4659 | CG1 | VAL | C | 183 | -33.091 | -54.486 | 120.621 | 1.00 | 17.87 | C |
| ATOM | 4660 | CG2 | VAL | C | 183 | -33.031 | -51.953 | 120.585 | 1.00 | 29.36 | C |
| ATOM | 4661 | N   | VAL | C | 184 | -36.585 | -51.813 | 121.122 | 1.00 | 25.36 | N |
| ATOM | 4662 | CA  | VAL | C | 184 | -37.315 | -50.601 | 121.452 | 1.00 | 29.08 | C |
| ATOM | 4663 | C   | VAL | C | 184 | -37.320 | -50.459 | 122.968 | 1.00 | 33.22 | C |
| ATOM | 4664 | O   | VAL | C | 184 | -37.476 | -51.444 | 123.700 | 1.00 | 34.38 | O |
| ATOM | 4665 | CB  | VAL | C | 184 | -38.734 | -50.602 | 120.812 | 1.00 | 30.67 | C |
| ATOM | 4666 | CG1 | VAL | C | 184 | -39.523 | -51.866 | 121.135 | 1.00 | 28.94 | C |
| ATOM | 4667 | CG2 | VAL | C | 184 | -39.531 | -49.352 | 121.221 | 1.00 | 30.72 | C |
| ATOM | 4668 | N   | THR | C | 185 | -37.102 | -49.234 | 123.440 | 1.00 | 31.38 | N |
| ATOM | 4669 | CA  | THR | C | 185 | -37.213 | -48.922 | 124.854 | 1.00 | 29.00 | C |
| ATOM | 4670 | C   | THR | C | 185 | -38.591 | -48.319 | 125.074 | 1.00 | 30.90 | C |
| ATOM | 4671 | O   | THR | C | 185 | -39.048 | -47.490 | 124.284 | 1.00 | 37.29 | O |
| ATOM | 4672 | CB  | THR | C | 185 | -36.102 | -47.977 | 125.330 | 1.00 | 24.92 | C |
| ATOM | 4673 | OG1 | THR | C | 185 | -35.966 | -46.874 | 124.424 | 1.00 | 32.54 | O |
| ATOM | 4674 | CG2 | THR | C | 185 | -34.779 | -48.706 | 125.402 | 1.00 | 26.78 | C |
| ATOM | 4675 | N   | VAL | C | 186 | -39.273 | -48.800 | 126.103 | 1.00 | 29.32 | N |
| ATOM | 4676 | CA  | VAL | C | 186 | -40.625 | -48.377 | 126.458 | 1.00 | 34.69 | C |
| ATOM | 4677 | C   | VAL | C | 186 | -40.698 | -48.292 | 127.970 | 1.00 | 36.57 | C |
| ATOM | 4678 | O   | VAL | C | 186 | -39.835 | -48.836 | 128.675 | 1.00 | 34.20 | O |
| ATOM | 4679 | CB  | VAL | C | 186 | -41.699 | -49.358 | 125.944 | 1.00 | 36.77 | C |
| ATOM | 4680 | CG1 | VAL | C | 186 | -41.645 | -49.516 | 124.421 | 1.00 | 27.93 | C |
| ATOM | 4681 | CG2 | VAL | C | 186 | -41.571 | -50.715 | 126.679 | 1.00 | 32.35 | C |
| ATOM | 4682 | N   | PRO | C | 187 | -41.717 | -47.608 | 128.499 | 1.00 | 38.80 | N |

```
ATOM   4683  CA  PRO C 187     -41.851 -47.521 129.958  1.00 40.40           C
ATOM   4684  C   PRO C 187     -42.128 -48.886 130.574  1.00 43.67           C
ATOM   4685  O   PRO C 187     -42.872 -49.703 130.024  1.00 42.12           O
ATOM   4686  CB  PRO C 187     -43.033 -46.566 130.145  1.00 36.03           C
ATOM   4687  CG  PRO C 187     -43.095 -45.798 128.889  1.00 29.12           C
ATOM   4688  CD  PRO C 187     -42.697 -46.739 127.820  1.00 34.04           C
ATOM   4689  N   SER C 188     -41.501 -49.128 131.728  1.00 47.08           N
ATOM   4690  CA  SER C 188     -41.753 -50.348 132.489  1.00 48.24           C
ATOM   4691  C   SER C 188     -43.244 -50.561 132.733  1.00 47.93           C
ATOM   4692  O   SER C 188     -43.776 -51.660 132.523  1.00 45.02           O
ATOM   4693  CB  SER C 188     -41.011 -50.260 133.813  1.00 50.96           C
ATOM   4694  OG  SER C 188     -39.631 -50.377 133.576  1.00 59.84           O
ATOM   4695  N   SER C 189     -43.941 -49.495 133.134  1.00 46.10           N
ATOM   4696  CA  SER C 189     -45.363 -49.574 133.444  1.00 51.64           C
ATOM   4697  C   SER C 189     -46.222 -50.037 132.263  1.00 51.86           C
ATOM   4698  O   SER C 189     -47.344 -50.512 132.481  1.00 58.15           O
ATOM   4699  CB  SER C 189     -45.834 -48.218 133.976  1.00 51.35           C
ATOM   4700  OG  SER C 189     -45.680 -47.208 133.001  1.00 55.98           O
ATOM   4701  N   SER C 190     -45.740 -49.918 131.022  1.00 51.05           N
ATOM   4702  CA  SER C 190     -46.558 -50.310 129.870  1.00 50.46           C
ATOM   4703  C   SER C 190     -46.527 -51.807 129.570  1.00 49.06           C
ATOM   4704  O   SER C 190     -47.396 -52.279 128.830  1.00 45.72           O
ATOM   4705  CB  SER C 190     -46.113 -49.566 128.610  1.00 40.88           C
ATOM   4706  OG  SER C 190     -44.817 -49.995 128.218  1.00 41.80           O
ATOM   4707  N   LEU C 191     -45.553 -52.559 130.096  1.00 47.92           N
ATOM   4708  CA  LEU C 191     -45.508 -53.993 129.838  1.00 42.80           C
ATOM   4709  C   LEU C 191     -46.706 -54.670 130.475  1.00 48.54           C
ATOM   4710  O   LEU C 191     -47.206 -54.244 131.517  1.00 56.38           O
ATOM   4711  CB  LEU C 191     -44.230 -54.612 130.387  1.00 39.21           C
ATOM   4712  CG  LEU C 191     -42.884 -54.023 129.982  1.00 44.80           C
ATOM   4713  CD1 LEU C 191     -41.792 -54.654 130.804  1.00 34.81           C
ATOM   4714  CD2 LEU C 191     -42.634 -54.240 128.501  1.00 37.00           C
ATOM   4715  N   GLY C 192     -47.170 -55.743 129.856  1.00 48.62           N
ATOM   4716  CA  GLY C 192     -48.335 -56.412 130.386  1.00 56.13           C
ATOM   4717  C   GLY C 192     -49.643 -55.692 130.132  1.00 55.78           C
ATOM   4718  O   GLY C 192     -50.704 -56.327 130.177  1.00 56.01           O
ATOM   4719  N   THR C 193     -49.601 -54.388 129.869  1.00 48.97           N
ATOM   4720  CA  THR C 193     -50.727 -53.607 129.378  1.00 49.79           C
ATOM   4721  C   THR C 193     -50.644 -53.332 127.883  1.00 49.11           C
ATOM   4722  O   THR C 193     -51.669 -53.324 127.197  1.00 53.12           O
ATOM   4723  CB  THR C 193     -50.780 -52.266 130.114  1.00 58.23           C
ATOM   4724  OG1 THR C 193     -50.126 -52.405 131.385  1.00 58.05           O
ATOM   4725  CG2 THR C 193     -52.227 -51.790 130.293  1.00 53.04           C
ATOM   4726  N   GLN C 194     -49.443 -53.103 127.361  1.00 48.44           N
ATOM   4727  CA  GLN C 194     -49.241 -52.741 125.967  1.00 42.09           C
ATOM   4728  C   GLN C 194     -48.676 -53.911 125.177  1.00 37.51           C
ATOM   4729  O   GLN C 194     -47.808 -54.636 125.660  1.00 40.92           O
ATOM   4730  CB  GLN C 194     -48.324 -51.517 125.873  1.00 43.18           C
ATOM   4731  CG  GLN C 194     -48.013 -51.097 124.469  1.00 46.41           C
ATOM   4732  CD  GLN C 194     -49.252 -50.772 123.679  1.00 49.54           C
ATOM   4733  OE1 GLN C 194     -49.610 -51.489 122.735  1.00 42.75           O
ATOM   4734  NE2 GLN C 194     -49.939 -49.707 124.080  1.00 49.73           N
ATOM   4735  N   THR C 195     -49.176 -54.093 123.963  1.00 36.34           N
ATOM   4736  CA  THR C 195     -48.744 -55.181 123.101  1.00 39.27           C
ATOM   4737  C   THR C 195     -47.657 -54.704 122.151  1.00 39.62           C
ATOM   4738  O   THR C 195     -47.782 -53.650 121.523  1.00 38.44           O
ATOM   4739  CB  THR C 195     -49.921 -55.761 122.312  1.00 43.59           C
ATOM   4740  OG1 THR C 195     -50.568 -56.759 123.110  1.00 45.10           O
ATOM   4741  CG2 THR C 195     -49.470 -56.364 120.984  1.00 36.94           C
ATOM   4742  N   TYR C 196     -46.604 -55.500 122.044  1.00 39.64           N
ATOM   4743  CA  TYR C 196     -45.452 -55.182 121.224  1.00 31.97           C
ATOM   4744  C   TYR C 196     -45.224 -56.327 120.252  1.00 32.44           C
ATOM   4745  O   TYR C 196     -44.912 -57.445 120.668  1.00 28.81           O
ATOM   4746  CB  TYR C 196     -44.231 -54.930 122.098  1.00 33.73           C
ATOM   4747  CG  TYR C 196     -44.426 -53.737 123.002  1.00 38.32           C
ATOM   4748  CD1 TYR C 196     -44.552 -52.458 122.474  1.00 33.41           C
ATOM   4749  CD2 TYR C 196     -44.483 -53.884 124.380  1.00 37.95           C
ATOM   4750  CE1 TYR C 196     -44.728 -51.370 123.289  1.00 33.25           C
ATOM   4751  CE2 TYR C 196     -44.660 -52.788 125.201  1.00 36.63           C
ATOM   4752  CZ  TYR C 196     -44.781 -51.541 124.647  1.00 33.58           C
ATOM   4753  OH  TYR C 196     -44.966 -50.453 125.459  1.00 38.73           O
```

```
ATOM   4754  N   ILE C 197    -45.395 -56.038 118.964  1.00 31.69           N
ATOM   4755  CA  ILE C 197    -45.176 -56.988 117.885  1.00 31.54           C
ATOM   4756  C   ILE C 197    -44.163 -56.386 116.919  1.00 33.20           C
ATOM   4757  O   ILE C 197    -44.321 -55.239 116.484  1.00 33.69           O
ATOM   4758  CB  ILE C 197    -46.476 -57.288 117.131  1.00 31.06           C
ATOM   4759  CG1 ILE C 197    -47.516 -57.886 118.042  1.00 33.48           C
ATOM   4760  CG2 ILE C 197    -46.201 -58.193 115.952  1.00 33.02           C
ATOM   4761  CD1 ILE C 197    -48.862 -57.783 117.425  1.00 24.92           C
ATOM   4762  N   CYS C 198    -43.163 -57.165 116.533  1.00 28.72           N
ATOM   4763  CA  CYS C 198    -42.254 -56.721 115.489  1.00 35.10           C
ATOM   4764  C   CYS C 198    -42.613 -57.390 114.159  1.00 33.06           C
ATOM   4765  O   CYS C 198    -42.986 -58.566 114.107  1.00 34.76           O
ATOM   4766  CB  CYS C 198    -40.798 -56.986 115.869  1.00 35.03           C
ATOM   4767  SG  CYS C 198    -40.302 -58.637 115.586  1.00 44.99           S
ATOM   4768  N   ASN C 199    -42.585 -56.609 113.098  1.00 28.45           N
ATOM   4769  CA  ASN C 199    -43.018 -57.057 111.786  1.00 29.27           C
ATOM   4770  C   ASN C 199    -41.775 -57.159 110.921  1.00 28.88           C
ATOM   4771  O   ASN C 199    -41.126 -56.148 110.645  1.00 34.72           O
ATOM   4772  CB  ASN C 199    -44.058 -56.094 111.214  1.00 29.40           C
ATOM   4773  CG  ASN C 199    -45.068 -55.631 112.274  1.00 35.22           C
ATOM   4774  OD1 ASN C 199    -45.044 -54.468 112.719  1.00 33.27           O
ATOM   4775  ND2 ASN C 199    -45.951 -56.546 112.693  1.00 28.65           N
ATOM   4776  N   VAL C 200    -41.435 -58.381 110.521  1.00 26.15           N
ATOM   4777  CA  VAL C 200    -40.230 -58.679 109.758  1.00 28.67           C
ATOM   4778  C   VAL C 200    -40.617 -59.030 108.329  1.00 30.60           C
ATOM   4779  O   VAL C 200    -41.478 -59.890 108.106  1.00 28.53           O
ATOM   4780  CB  VAL C 200    -39.450 -59.841 110.388  1.00 21.99           C
ATOM   4781  CG1 VAL C 200    -38.183 -60.065 109.624  1.00 27.77           C
ATOM   4782  CG2 VAL C 200    -39.182 -59.577 111.849  1.00 26.33           C
ATOM   4783  N   ASN C 201    -39.960 -58.406 107.359  1.00 31.19           N
ATOM   4784  CA  ASN C 201    -40.153 -58.800 105.970  1.00 33.45           C
ATOM   4785  C   ASN C 201    -38.805 -59.085 105.341  1.00 35.94           C
ATOM   4786  O   ASN C 201    -37.952 -58.195 105.238  1.00 38.25           O
ATOM   4787  CB  ASN C 201    -40.899 -57.739 105.160  1.00 34.65           C
ATOM   4788  CG  ASN C 201    -41.430 -58.284 103.832  1.00 38.40           C
ATOM   4789  OD1 ASN C 201    -42.045 -57.555 103.074  1.00 47.99           O
ATOM   4790  ND2 ASN C 201    -41.203 -59.568 103.557  1.00 39.32           N
ATOM   4791  N   HIS C 202    -38.630 -60.310 104.893  1.00 35.27           N
ATOM   4792  CA  HIS C 202    -37.474 -60.692 104.107  1.00 32.39           C
ATOM   4793  C   HIS C 202    -38.047 -60.925 102.719  1.00 34.80           C
ATOM   4794  O   HIS C 202    -38.424 -62.043 102.375  1.00 35.23           O
ATOM   4795  CB  HIS C 202    -36.777 -61.922 104.669  1.00 29.35           C
ATOM   4796  CG  HIS C 202    -35.529 -62.276 103.935  1.00 32.95           C
ATOM   4797  ND1 HIS C 202    -35.318 -63.520 103.385  1.00 37.12           N
ATOM   4798  CD2 HIS C 202    -34.440 -61.537 103.623  1.00 31.23           C
ATOM   4799  CE1 HIS C 202    -34.145 -63.538 102.778  1.00 33.20           C
ATOM   4800  NE2 HIS C 202    -33.593 -62.346 102.695  1.00 32.51           N
ATOM   4801  N   LYS C 203    -38.159 -59.837 101.948  1.00 37.66           N
ATOM   4802  CA  LYS C 203    -38.635 -59.928 100.569  1.00 34.00           C
ATOM   4803  C   LYS C 203    -37.907 -60.983  99.750  1.00 36.61           C
ATOM   4804  O   LYS C 203    -38.585 -61.707  99.002  1.00 36.77           O
ATOM   4805  CB  LYS C 203    -38.464 -58.583  99.854  1.00 31.21           C
ATOM   4806  CG  LYS C 203    -39.438 -57.458 100.162  1.00 39.44           C
ATOM   4807  CD  LYS C 203    -40.808 -57.621  99.534  1.00 41.59           C
ATOM   4808  CE  LYS C 203    -41.815 -56.681 100.199  1.00 47.06           C
ATOM   4809  NZ  LYS C 203    -41.248 -55.297 100.259  1.00 45.84           N1+
ATOM   4810  N   PRO C 204    -36.574 -61.155  99.854  1.00 38.41           N
ATOM   4811  CA  PRO C 204    -35.907 -62.124  98.962  1.00 33.23           C
ATOM   4812  C   PRO C 204    -36.392 -63.552  99.111  1.00 37.59           C
ATOM   4813  O   PRO C 204    -36.358 -64.297  98.129  1.00 44.57           O
ATOM   4814  CB  PRO C 204    -34.425 -61.994  99.342  1.00 28.63           C
ATOM   4815  CG  PRO C 204    -34.306 -60.634  99.898  1.00 32.37           C
ATOM   4816  CD  PRO C 204    -35.587 -60.346 100.603  1.00 28.15           C
ATOM   4817  N   SER C 205    -36.857 -63.964 100.287  1.00 37.25           N
ATOM   4818  CA  SER C 205    -37.381 -65.312 100.469  1.00 37.14           C
ATOM   4819  C   SER C 205    -38.895 -65.320 100.574  1.00 38.83           C
ATOM   4820  O   SER C 205    -39.480 -66.367 100.869  1.00 37.58           O
ATOM   4821  CB  SER C 205    -36.771 -65.963 101.709  1.00 34.91           C
ATOM   4822  OG  SER C 205    -37.253 -65.348 102.893  1.00 33.36           O
ATOM   4823  N   ASN C 206    -39.532 -64.173 100.334  1.00 40.37           N
ATOM   4824  CA  ASN C 206    -40.974 -63.989 100.466  1.00 40.89           C
```

| ATOM | 4825 | C | ASN C 206 | -41.484 -64.469 101.827 | 1.00 44.23 | C |
|------|------|------|-----------|-------------------------|------------|------|
| ATOM | 4826 | O | ASN C 206 | -42.478 -65.192 101.926 | 1.00 46.80 | O |
| ATOM | 4827 | CB | ASN C 206 | -41.703 -64.684 99.332 | 1.00 49.25 | C |
| ATOM | 4828 | CG | ASN C 206 | -42.643 -63.766 98.626 | 1.00 60.95 | C |
| ATOM | 4829 | OD1 | ASN C 206 | -42.272 -63.130 97.632 | 1.00 64.04 | O |
| ATOM | 4830 | ND2 | ASN C 206 | -43.871 -63.660 99.141 | 1.00 62.21 | N |
| ATOM | 4831 | N | THR C 207 | -40.773 -64.069 102.880 | 1.00 37.63 | N |
| ATOM | 4832 | CA | THR C 207 | -41.070 -64.446 104.256 | 1.00 33.70 | C |
| ATOM | 4833 | C | THR C 207 | -41.518 -63.223 105.044 | 1.00 35.76 | C |
| ATOM | 4834 | O | THR C 207 | -40.806 -62.218 105.096 | 1.00 34.16 | O |
| ATOM | 4835 | CB | THR C 207 | -39.844 -65.055 104.920 | 1.00 30.61 | C |
| ATOM | 4836 | OG1 | THR C 207 | -39.390 -66.163 104.140 | 1.00 32.93 | O |
| ATOM | 4837 | CG2 | THR C 207 | -40.164 -65.490 106.323 | 1.00 30.98 | C |
| ATOM | 4838 | N | LYS C 208 | -42.686 -63.311 105.663 | 1.00 35.22 | N |
| ATOM | 4839 | CA | LYS C 208 | -43.154 -62.277 106.565 | 1.00 31.98 | C |
| ATOM | 4840 | C | LYS C 208 | -43.461 -62.906 107.915 | 1.00 33.42 | C |
| ATOM | 4841 | O | LYS C 208 | -44.079 -63.966 107.977 | 1.00 34.80 | O |
| ATOM | 4842 | CB | LYS C 208 | -44.375 -61.587 105.990 | 1.00 29.96 | C |
| ATOM | 4843 | CG | LYS C 208 | -44.038 -60.590 104.902 | 1.00 34.70 | C |
| ATOM | 4844 | CD | LYS C 208 | -45.215 -59.721 104.524 | 1.00 37.48 | C |
| ATOM | 4845 | CE | LYS C 208 | -46.304 -60.544 103.838 | 1.00 44.32 | C |
| ATOM | 4846 | NZ | LYS C 208 | -47.529 -59.739 103.513 | 1.00 56.22 | N1+ |
| ATOM | 4847 | N | VAL C 209 | -43.064 -62.232 108.994 | 1.00 29.95 | N |
| ATOM | 4848 | CA | VAL C 209 | -43.234 -62.745 110.348 | 1.00 29.28 | C |
| ATOM | 4849 | C | VAL C 209 | -43.711 -61.614 111.255 | 1.00 31.89 | C |
| ATOM | 4850 | O | VAL C 209 | -43.177 -60.501 111.209 | 1.00 33.86 | O |
| ATOM | 4851 | CB | VAL C 209 | -41.930 -63.382 110.883 | 1.00 29.99 | C |
| ATOM | 4852 | CG1 | VAL C 209 | -42.024 -63.631 112.364 | 1.00 29.51 | C |
| ATOM | 4853 | CG2 | VAL C 209 | -41.659 -64.720 110.193 | 1.00 28.13 | C |
| ATOM | 4854 | N | ASP C 210 | -44.744 -61.888 112.051 | 1.00 32.06 | N |
| ATOM | 4855 | CA | ASP C 210 | -45.187 -61.015 113.137 | 1.00 31.18 | C |
| ATOM | 4856 | C | ASP C 210 | -44.963 -61.720 114.466 | 1.00 32.54 | C |
| ATOM | 4857 | O | ASP C 210 | -45.627 -62.717 114.749 | 1.00 37.02 | O |
| ATOM | 4858 | CB | ASP C 210 | -46.667 -60.664 112.988 | 1.00 32.07 | C |
| ATOM | 4859 | CG | ASP C 210 | -46.950 -59.774 111.779 | 1.00 38.99 | C |
| ATOM | 4860 | OD1 | ASP C 210 | -46.283 -58.727 111.607 | 1.00 38.00 | O |
| ATOM | 4861 | OD2 | ASP C 210 | -47.851 -60.125 110.993 | 1.00 43.24 | O1- |
| ATOM | 4862 | N | LYS C 211 | -44.090 -61.171 115.307 | 1.00 28.18 | N |
| ATOM | 4863 | CA | LYS C 211 | -43.714 -61.808 116.561 | 1.00 27.79 | C |
| ATOM | 4864 | C | LYS C 211 | -44.107 -60.919 117.735 | 1.00 35.35 | C |
| ATOM | 4865 | O | LYS C 211 | -43.646 -59.778 117.840 | 1.00 31.15 | O |
| ATOM | 4866 | CB | LYS C 211 | -42.214 -62.112 116.591 | 1.00 30.51 | C |
| ATOM | 4867 | CG | LYS C 211 | -41.695 -62.682 117.906 | 1.00 29.62 | C |
| ATOM | 4868 | CD | LYS C 211 | -42.354 -64.008 118.183 | 1.00 35.84 | C |
| ATOM | 4869 | CE | LYS C 211 | -41.473 -64.913 119.014 | 1.00 40.39 | C |
| ATOM | 4870 | NZ | LYS C 211 | -41.983 -66.295 118.890 | 1.00 39.30 | N1+ |
| ATOM | 4871 | N | LYS C 212 | -44.971 -61.439 118.608 | 1.00 33.29 | N |
| ATOM | 4872 | CA | LYS C 212 | -45.256 -60.779 119.867 | 1.00 29.95 | C |
| ATOM | 4873 | C | LYS C 212 | -44.162 -61.099 120.869 | 1.00 33.39 | C |
| ATOM | 4874 | O | LYS C 212 | -43.713 -62.248 120.985 | 1.00 41.06 | O |
| ATOM | 4875 | CB | LYS C 212 | -46.621 -61.213 120.394 | 1.00 33.65 | C |
| ATOM | 4876 | CG | LYS C 212 | -47.235 -60.285 121.413 | 1.00 32.68 | C |
| ATOM | 4877 | CD | LYS C 212 | -48.491 -60.893 122.040 | 1.00 34.32 | C |
| ATOM | 4878 | CE | LYS C 212 | -49.020 -59.999 123.150 | 1.00 34.90 | C |
| ATOM | 4879 | NZ | LYS C 212 | -50.184 -60.548 123.882 | 1.00 52.78 | N1+ |
| ATOM | 4880 | N | VAL C 213 | -43.742 -60.078 121.602 | 1.00 29.81 | N |
| ATOM | 4881 | CA | VAL C 213 | -42.705 -60.195 122.612 | 1.00 28.67 | C |
| ATOM | 4882 | C | VAL C 213 | -43.332 -59.803 123.948 | 1.00 35.48 | C |
| ATOM | 4883 | O | VAL C 213 | -43.668 -58.634 124.170 | 1.00 34.53 | O |
| ATOM | 4884 | CB | VAL C 213 | -41.493 -59.313 122.281 | 1.00 34.29 | C |
| ATOM | 4885 | CG1 | VAL C 213 | -40.355 -59.481 123.311 | 1.00 29.32 | C |
| ATOM | 4886 | CG2 | VAL C 213 | -41.022 -59.563 120.861 | 1.00 28.07 | C |
| ATOM | 4887 | N | GLU C 214 | -43.503 -60.781 124.833 | 1.00 43.40 | N |
| ATOM | 4888 | CA | GLU C 214 | -44.067 -60.573 126.149 | 1.00 43.27 | C |
| ATOM | 4889 | C | GLU C 214 | -42.990 -60.735 127.209 | 1.00 49.50 | C |
| ATOM | 4890 | O | GLU C 214 | -42.011 -61.461 126.997 | 1.00 50.46 | O |
| ATOM | 4891 | CB | GLU C 214 | -45.206 -61.558 126.434 | 1.00 48.02 | C |
| ATOM | 4892 | CG | GLU C 214 | -46.365 -61.449 125.439 | 1.00 53.85 | C |
| ATOM | 4893 | CD | GLU C 214 | -47.489 -62.450 125.686 | 1.00 57.28 | C |
| ATOM | 4894 | OE1 | GLU C 214 | -48.525 -62.026 126.249 | 1.00 58.32 | O |
| ATOM | 4895 | OE2 | GLU C 214 | -47.343 -63.644 125.318 | 1.00 60.40 | O1- |

```
ATOM   4896  N    PRO C 215    -43.109 -60.039 128.338  1.00 50.15        N
ATOM   4897  CA   PRO C 215    -42.182 -60.293 129.452  1.00 50.10        C
ATOM   4898  C    PRO C 215    -42.336 -61.727 129.928  1.00 54.89        C
ATOM   4899  O    PRO C 215    -43.450 -62.225 130.077  1.00 62.80        O
ATOM   4900  CB   PRO C 215    -42.606 -59.277 130.515  1.00 49.74        C
ATOM   4901  CG   PRO C 215    -43.879 -58.612 129.977  1.00 51.15        C
ATOM   4902  CD   PRO C 215    -43.892 -58.805 128.515  1.00 47.22        C
ATOM   4903  N    LYS C 216    -41.208 -62.402 130.137  1.00 59.54        N
ATOM   4904  CA   LYS C 216    -41.205 -63.833 130.410  1.00 68.05        C
ATOM   4905  C    LYS C 216    -40.699 -64.127 131.820  1.00 79.82        C
ATOM   4906  O    LYS C 216    -39.755 -63.482 132.304  1.00 68.19        O
ATOM   4907  CB   LYS C 216    -40.371 -64.581 129.359  1.00 67.81        C
ATOM   4908  CG   LYS C 216    -40.384 -66.093 129.519  1.00 71.95        C
ATOM   4909  CD   LYS C 216    -39.957 -66.793 128.233  1.00 73.44        C
ATOM   4910  CE   LYS C 216    -40.151 -68.301 128.345  1.00 66.41        C
ATOM   4911  NZ   LYS C 216    -40.055 -68.966 127.016  1.00 77.32        N1+
ATOM   4912  N    SER C 217    -41.360 -65.090 132.479  1.00 86.12        N
ATOM   4913  CA   SER C 217    -41.015 -65.548 133.838  1.00 92.36        C
ATOM   4914  C    SER C 217    -40.297 -66.901 133.836  1.00 83.50        C
ATOM   4915  O    SER C 217    -40.341 -67.644 132.851  1.00 85.79        O
ATOM   4916  CB   SER C 217    -42.273 -65.660 134.707  1.00 84.83        C
ATOM   4917  OG   SER C 217    -43.144 -66.656 134.191  1.00 75.85        O
TER
ATOM   4918  N    GLU D   1     -2.709 -44.362  88.539  1.00 46.38        N
ATOM   4919  CA   GLU D   1     -3.927 -43.604  88.796  1.00 40.18        C
ATOM   4920  C    GLU D   1     -3.692 -42.543  89.882  1.00 40.43        C
ATOM   4921  O    GLU D   1     -2.818 -42.704  90.730  1.00 41.89        O
ATOM   4922  CB   GLU D   1     -5.056 -44.548  89.205  1.00 33.14        C
ATOM   4923  CG   GLU D   1     -5.071 -44.860  90.693  1.00 34.96        C
ATOM   4924  CD   GLU D   1     -6.026 -45.981  91.081  1.00 47.71        C
ATOM   4925  OE1  GLU D   1     -6.745 -46.523  90.207  1.00 56.37        O
ATOM   4926  OE2  GLU D   1     -6.060 -46.324  92.281  1.00 57.65        O1-
ATOM   4927  N    ILE D   2     -4.482 -41.470  89.840  1.00 35.77        N
ATOM   4928  CA   ILE D   2     -4.431 -40.417  90.844  1.00 30.01        C
ATOM   4929  C    ILE D   2     -5.055 -40.916  92.141  1.00 34.99        C
ATOM   4930  O    ILE D   2     -6.246 -41.247  92.184  1.00 35.20        O
ATOM   4931  CB   ILE D   2     -5.147 -39.166  90.336  1.00 32.58        C
ATOM   4932  CG1  ILE D   2     -4.425 -38.645  89.088  1.00 29.66        C
ATOM   4933  CG2  ILE D   2     -5.297 -38.133  91.458  1.00 29.46        C
ATOM   4934  CD1  ILE D   2     -5.026 -37.400  88.488  1.00 28.66        C
ATOM   4935  N    VAL D   3     -4.263 -40.933  93.219  1.00 34.76        N
ATOM   4936  CA   VAL D   3     -4.726 -41.369  94.533  1.00 30.38        C
ATOM   4937  C    VAL D   3     -5.153 -40.143  95.325  1.00 31.44        C
ATOM   4938  O    VAL D   3     -4.409 -39.155  95.403  1.00 31.03        O
ATOM   4939  CB   VAL D   3     -3.641 -42.166  95.278  1.00 29.88        C
ATOM   4940  CG1  VAL D   3     -4.089 -42.450  96.695  1.00 25.14        C
ATOM   4941  CG2  VAL D   3     -3.355 -43.479  94.565  1.00 22.24        C
ATOM   4942  N    LEU D   4     -6.379 -40.174  95.851  1.00 30.04        N
ATOM   4943  CA   LEU D   4     -6.942 -39.055  96.604  1.00 28.75        C
ATOM   4944  C    LEU D   4     -7.087 -39.490  98.050  1.00 29.11        C
ATOM   4945  O    LEU D   4     -7.809 -40.450  98.337  1.00 37.17        O
ATOM   4946  CB   LEU D   4     -8.303 -38.607  96.057  1.00 27.56        C
ATOM   4947  CG   LEU D   4     -8.478 -38.095  94.619  1.00 27.56        C
ATOM   4948  CD1  LEU D   4     -9.887 -37.577  94.388  1.00 23.55        C
ATOM   4949  CD2  LEU D   4     -7.477 -37.038  94.270  1.00 27.03        C
ATOM   4950  N    THR D   5     -6.414 -38.795  98.956  1.00 27.89        N
ATOM   4951  CA   THR D   5     -6.535 -39.071 100.382  1.00 27.95        C
ATOM   4952  C    THR D   5     -7.291 -37.915 101.020  1.00 27.80        C
ATOM   4953  O    THR D   5     -6.899 -36.752 100.880  1.00 29.84        O
ATOM   4954  CB   THR D   5     -5.180 -39.257 101.067  1.00 25.13        C
ATOM   4955  OG1  THR D   5     -4.610 -37.975 101.281  1.00 44.55        O
ATOM   4956  CG2  THR D   5     -4.229 -40.091 100.229  1.00 22.72        C
ATOM   4957  N    GLN D   6     -8.379 -38.229 101.693  1.00 26.25        N
ATOM   4958  CA   GLN D   6     -9.154 -37.231 102.401  1.00 29.68        C
ATOM   4959  C    GLN D   6     -8.770 -37.227 103.870  1.00 29.42        C
ATOM   4960  O    GLN D   6     -8.596 -38.287 104.475  1.00 30.59        O
ATOM   4961  CB   GLN D   6    -10.645 -37.515 102.260  1.00 28.59        C
ATOM   4962  CG   GLN D   6    -11.199 -37.068 100.946  1.00 26.38        C
ATOM   4963  CD   GLN D   6    -12.674 -37.347 100.825  1.00 28.44        C
ATOM   4964  OE1  GLN D   6    -13.063 -38.310 100.182  1.00 30.52        O
ATOM   4965  NE2  GLN D   6    -13.507 -36.497 101.430  1.00 23.50        N
```

```
ATOM   4966  N    SER D   7     -8.646 -36.032 104.442  1.00 32.95          N
ATOM   4967  CA   SER D   7     -8.474 -35.923 105.893  1.00 34.15          C
ATOM   4968  C    SER D   7     -9.305 -34.779 106.507  1.00 36.23          C
ATOM   4969  O    SER D   7     -9.692 -33.818 105.822  1.00 34.31          O
ATOM   4970  CB   SER D   7     -7.007 -35.732 106.234  1.00 32.05          C
ATOM   4971  OG   SER D   7     -6.622 -34.406 105.965  1.00 36.70          O
ATOM   4972  N    PRO D   8     -9.657 -34.917 107.791  1.00 35.26          N
ATOM   4973  CA   PRO D   8     -9.493 -36.114 108.627  1.00 31.68          C
ATOM   4974  C    PRO D   8    -10.479 -37.209 108.218  1.00 35.24          C
ATOM   4975  O    PRO D   8    -11.338 -36.963 107.380  1.00 34.44          O
ATOM   4976  CB   PRO D   8     -9.812 -35.599 110.022  1.00 31.95          C
ATOM   4977  CG   PRO D   8    -10.849 -34.538 109.772  1.00 33.10          C
ATOM   4978  CD   PRO D   8    -10.446 -33.874 108.471  1.00 32.84          C
ATOM   4979  N    GLY D   9    -10.345 -38.409 108.776  1.00 36.77          N
ATOM   4980  CA   GLY D   9    -11.322 -39.448 108.496  1.00 26.26          C
ATOM   4981  C    GLY D   9    -12.671 -39.170 109.129  1.00 26.81          C
ATOM   4982  O    GLY D   9    -13.706 -39.472 108.541  1.00 29.93          O
ATOM   4983  N    THR D  10    -12.681 -38.619 110.349  1.00 29.94          N
ATOM   4984  CA   THR D  10    -13.909 -38.207 111.025  1.00 29.91          C
ATOM   4985  C    THR D  10    -13.748 -36.800 111.602  1.00 35.08          C
ATOM   4986  O    THR D  10    -12.672 -36.421 112.072  1.00 37.12          O
ATOM   4987  CB   THR D  10    -14.324 -39.175 112.138  1.00 28.55          C
ATOM   4988  OG1  THR D  10    -14.423 -40.503 111.612  1.00 29.83          O
ATOM   4989  CG2  THR D  10    -15.682 -38.775 112.712  1.00 26.70          C
ATOM   4990  N    LEU D  11    -14.823 -36.022 111.527  1.00 33.72          N
ATOM   4991  CA   LEU D  11    -14.874 -34.646 111.997  1.00 30.12          C
ATOM   4992  C    LEU D  11    -16.092 -34.516 112.895  1.00 32.24          C
ATOM   4993  O    LEU D  11    -17.220 -34.659 112.407  1.00 35.42          O
ATOM   4994  CB   LEU D  11    -15.010 -33.702 110.811  1.00 31.05          C
ATOM   4995  CG   LEU D  11    -14.043 -32.578 110.566  1.00 35.03          C
ATOM   4996  CD1  LEU D  11    -14.736 -31.626 109.611  1.00 32.51          C
ATOM   4997  CD2  LEU D  11    -13.741 -31.926 111.890  1.00 32.63          C
ATOM   4998  N    SER D  12    -15.880 -34.220 114.183  1.00 27.22          N
ATOM   4999  CA   SER D  12    -16.969 -34.041 115.149  1.00 31.54          C
ATOM   5000  C    SER D  12    -17.136 -32.563 115.466  1.00 26.28          C
ATOM   5001  O    SER D  12    -16.200 -31.925 115.940  1.00 31.22          O
ATOM   5002  CB   SER D  12    -16.711 -34.815 116.443  1.00 30.19          C
ATOM   5003  OG   SER D  12    -16.413 -36.172 116.169  1.00 30.39          O
ATOM   5004  N    LEU D  13    -18.320 -32.023 115.209  1.00 28.43          N
ATOM   5005  CA   LEU D  13    -18.535 -30.588 115.335  1.00 32.40          C
ATOM   5006  C    LEU D  13    -19.965 -30.311 115.779  1.00 32.08          C
ATOM   5007  O    LEU D  13    -20.889 -31.062 115.463  1.00 33.15          O
ATOM   5008  CB   LEU D  13    -18.229 -29.860 114.013  1.00 29.60          C
ATOM   5009  CG   LEU D  13    -16.792 -29.909 113.449  1.00 31.08          C
ATOM   5010  CD1  LEU D  13    -16.740 -29.357 112.045  1.00 34.57          C
ATOM   5011  CD2  LEU D  13    -15.818 -29.128 114.313  1.00 19.13          C
ATOM   5012  N    SER D  14    -20.148 -29.201 116.468  1.00 33.43          N
ATOM   5013  CA   SER D  14    -21.515 -28.841 116.784  1.00 34.69          C
ATOM   5014  C    SER D  14    -22.184 -28.181 115.589  1.00 30.63          C
ATOM   5015  O    SER D  14    -21.528 -27.507 114.789  1.00 26.53          O
ATOM   5016  CB   SER D  14    -21.563 -27.869 117.963  1.00 40.13          C
ATOM   5017  OG   SER D  14    -21.315 -28.526 119.189  1.00 49.17          O
ATOM   5018  N    PRO D  15    -23.496 -28.324 115.471  1.00 30.71          N
ATOM   5019  CA   PRO D  15    -24.231 -27.513 114.495  1.00 30.71          C
ATOM   5020  C    PRO D  15    -23.981 -26.030 114.753  1.00 33.52          C
ATOM   5021  O    PRO D  15    -23.778 -25.598 115.892  1.00 30.44          O
ATOM   5022  CB   PRO D  15    -25.694 -27.907 114.732  1.00 29.82          C
ATOM   5023  CG   PRO D  15    -25.633 -29.250 115.357  1.00 27.19          C
ATOM   5024  CD   PRO D  15    -24.368 -29.263 116.188  1.00 28.99          C
ATOM   5025  N    GLY D  16    -23.917 -25.264 113.673  1.00 34.76          N
ATOM   5026  CA   GLY D  16    -23.561 -23.872 113.725  1.00 30.06          C
ATOM   5027  C    GLY D  16    -22.093 -23.615 113.489  1.00 32.57          C
ATOM   5028  O    GLY D  16    -21.728 -22.532 113.024  1.00 34.09          O
ATOM   5029  N    GLU D  17    -21.243 -24.599 113.748  1.00 30.04          N
ATOM   5030  CA   GLU D  17    -19.828 -24.365 113.578  1.00 29.50          C
ATOM   5031  C    GLU D  17    -19.442 -24.466 112.111  1.00 33.26          C
ATOM   5032  O    GLU D  17    -20.236 -24.848 111.242  1.00 28.54          O
ATOM   5033  CB   GLU D  17    -18.997 -25.365 114.370  1.00 30.61          C
ATOM   5034  CG   GLU D  17    -18.967 -25.187 115.863  1.00 38.28          C
ATOM   5035  CD   GLU D  17    -17.955 -26.152 116.491  1.00 47.48          C
ATOM   5036  OE1  GLU D  17    -16.740 -25.933 116.281  1.00 51.97          O
```

| ATOM | 5037 | OE2 | GLU | D | 17 | -18.365 | -27.154 | 117.143 | 1.00 | 47.45 | O1- |
|------|------|-----|-----|---|----|---------|---------|---------|------|-------|-----|
| ATOM | 5038 | N | ARG | D | 18 | -18.183 | -24.132 | 111.861 | 1.00 | 33.39 | N |
| ATOM | 5039 | CA | ARG | D | 18 | -17.585 | -24.128 | 110.544 | 1.00 | 30.46 | C |
| ATOM | 5040 | C | ARG | D | 18 | -16.823 | -25.439 | 110.354 | 1.00 | 35.01 | C |
| ATOM | 5041 | O | ARG | D | 18 | -16.148 | -25.911 | 111.273 | 1.00 | 36.75 | O |
| ATOM | 5042 | CB | ARG | D | 18 | -16.673 | -22.903 | 110.427 | 1.00 | 30.90 | C |
| ATOM | 5043 | CG | ARG | D | 18 | -15.935 | -22.722 | 109.136 | 1.00 | 33.87 | C |
| ATOM | 5044 | CD | ARG | D | 18 | -14.967 | -21.575 | 109.278 | 1.00 | 28.90 | C |
| ATOM | 5045 | NE | ARG | D | 18 | -14.168 | -21.350 | 108.073 | 1.00 | 42.50 | N |
| ATOM | 5046 | CZ | ARG | D | 18 | -14.603 | -20.689 | 106.995 | 1.00 | 46.47 | C |
| ATOM | 5047 | NH1 | ARG | D | 18 | -15.851 | -20.199 | 106.968 | 1.00 | 39.59 | N1+ |
| ATOM | 5048 | NH2 | ARG | D | 18 | -13.799 | -20.529 | 105.939 | 1.00 | 36.96 | N |
| ATOM | 5049 | N | ALA | D | 19 | -16.955 | -26.046 | 109.172 | 1.00 | 33.54 | N |
| ATOM | 5050 | CA | ALA | D | 19 | -16.302 | -27.317 | 108.864 | 1.00 | 31.96 | C |
| ATOM | 5051 | C | ALA | D | 19 | -15.394 | -27.131 | 107.661 | 1.00 | 27.02 | C |
| ATOM | 5052 | O | ALA | D | 19 | -15.760 | -26.443 | 106.707 | 1.00 | 28.38 | O |
| ATOM | 5053 | CB | ALA | D | 19 | -17.319 | -28.428 | 108.564 | 1.00 | 30.29 | C |
| ATOM | 5054 | N | THR | D | 20 | -14.203 | -27.715 | 107.720 | 1.00 | 24.79 | N |
| ATOM | 5055 | CA | THR | D | 20 | -13.250 | -27.647 | 106.619 | 1.00 | 25.95 | C |
| ATOM | 5056 | C | THR | D | 20 | -12.657 | -29.031 | 106.370 | 1.00 | 28.87 | C |
| ATOM | 5057 | O | THR | D | 20 | -12.056 | -29.619 | 107.272 | 1.00 | 27.37 | O |
| ATOM | 5058 | CB | THR | D | 20 | -12.160 | -26.618 | 106.920 | 1.00 | 33.57 | C |
| ATOM | 5059 | OG1 | THR | D | 20 | -12.678 | -25.302 | 106.669 | 1.00 | 35.59 | O |
| ATOM | 5060 | CG2 | THR | D | 20 | -10.942 | -26.842 | 106.026 | 1.00 | 35.30 | C |
| ATOM | 5061 | N | LEU | D | 21 | -12.845 | -29.553 | 105.153 | 1.00 | 27.92 | N |
| ATOM | 5062 | CA | LEU | D | 21 | -12.408 | -30.885 | 104.753 | 1.00 | 21.26 | C |
| ATOM | 5063 | C | LEU | D | 21 | -11.291 | -30.788 | 103.721 | 1.00 | 27.12 | C |
| ATOM | 5064 | O | LEU | D | 21 | -11.276 | -29.891 | 102.879 | 1.00 | 25.65 | O |
| ATOM | 5065 | CB | LEU | D | 21 | -13.567 | -31.688 | 104.171 | 1.00 | 24.71 | C |
| ATOM | 5066 | CG | LEU | D | 21 | -14.800 | -31.873 | 105.044 | 1.00 | 24.88 | C |
| ATOM | 5067 | CD1 | LEU | D | 21 | -15.745 | -32.804 | 104.393 | 1.00 | 25.75 | C |
| ATOM | 5068 | CD2 | LEU | D | 21 | -14.358 | -32.453 | 106.324 | 1.00 | 36.95 | C |
| ATOM | 5069 | N | SER | D | 22 | -10.364 | -31.732 | 103.777 | 1.00 | 25.30 | N |
| ATOM | 5070 | CA | SER | D | 22 | -9.207 | -31.718 | 102.906 | 1.00 | 29.86 | C |
| ATOM | 5071 | C | SER | D | 22 | -9.228 | -32.907 | 101.960 | 1.00 | 31.55 | C |
| ATOM | 5072 | O | SER | D | 22 | -9.637 | -34.010 | 102.338 | 1.00 | 32.22 | O |
| ATOM | 5073 | CB | SER | D | 22 | -7.908 | -31.743 | 103.711 | 1.00 | 29.48 | C |
| ATOM | 5074 | OG | SER | D | 22 | -7.484 | -30.430 | 103.983 | 1.00 | 48.01 | O |
| ATOM | 5075 | N | CYS | D | 23 | -8.748 | -32.668 | 100.743 | 1.00 | 23.08 | N |
| ATOM | 5076 | CA | CYS | D | 23 | -8.493 | -33.696 | 99.746 | 1.00 | 29.55 | C |
| ATOM | 5077 | C | CYS | D | 23 | -7.132 | -33.425 | 99.133 | 1.00 | 28.60 | C |
| ATOM | 5078 | O | CYS | D | 23 | -6.927 | -32.375 | 98.510 | 1.00 | 28.22 | O |
| ATOM | 5079 | CB | CYS | D | 23 | -9.577 | -33.692 | 98.661 | 1.00 | 32.45 | C |
| ATOM | 5080 | SG | CYS | D | 23 | -9.448 | -34.937 | 97.349 | 1.00 | 34.53 | S |
| ATOM | 5081 | N | ARG | D | 24 | -6.215 | -34.370 | 99.282 | 1.00 | 27.16 | N |
| ATOM | 5082 | CA | ARG | D | 24 | -4.896 | -34.261 | 98.682 | 1.00 | 30.26 | C |
| ATOM | 5083 | C | ARG | D | 24 | -4.755 | -35.243 | 97.517 | 1.00 | 29.93 | C |
| ATOM | 5084 | O | ARG | D | 24 | -5.021 | -36.442 | 97.663 | 1.00 | 32.04 | O |
| ATOM | 5085 | CB | ARG | D | 24 | -3.830 | -34.452 | 99.759 | 1.00 | 30.70 | C |
| ATOM | 5086 | CG | ARG | D | 24 | -3.797 | -33.212 | 100.656 | 1.00 | 40.46 | C |
| ATOM | 5087 | CD | ARG | D | 24 | -3.107 | -33.392 | 101.998 | 1.00 | 47.57 | C |
| ATOM | 5088 | NE | ARG | D | 24 | -2.760 | -32.094 | 102.596 | 1.00 | 64.41 | N |
| ATOM | 5089 | CZ | ARG | D | 24 | -3.482 | -31.436 | 103.512 | 1.00 | 65.33 | C |
| ATOM | 5090 | NH1 | ARG | D | 24 | -4.626 | -31.944 | 103.967 | 1.00 | 59.49 | N1+ |
| ATOM | 5091 | NH2 | ARG | D | 24 | -3.054 | -30.259 | 103.979 | 1.00 | 59.83 | N |
| ATOM | 5092 | N | ALA | D | 25 | -4.379 | -34.715 | 96.351 | 1.00 | 25.59 | N |
| ATOM | 5093 | CA | ALA | D | 25 | -4.224 | -35.487 | 95.126 | 1.00 | 27.28 | C |
| ATOM | 5094 | C | ALA | D | 25 | -2.745 | -35.776 | 94.882 | 1.00 | 29.22 | C |
| ATOM | 5095 | O | ALA | D | 25 | -1.914 | -34.868 | 94.943 | 1.00 | 32.95 | O |
| ATOM | 5096 | CB | ALA | D | 25 | -4.810 | -34.736 | 93.927 | 1.00 | 21.36 | C |
| ATOM | 5097 | N | SER | D | 26 | -2.428 | -37.029 | 94.593 | 1.00 | 26.23 | N |
| ATOM | 5098 | CA | SER | D | 26 | -1.100 | -37.425 | 94.171 | 1.00 | 26.45 | C |
| ATOM | 5099 | C | SER | D | 26 | -1.179 | -38.425 | 93.014 | 1.00 | 36.59 | C |
| ATOM | 5100 | O | SER | D | 26 | -1.636 | -39.568 | 93.195 | 1.00 | 38.09 | O |
| ATOM | 5101 | CB | SER | D | 26 | -0.330 | -38.048 | 95.323 | 1.00 | 31.96 | C |
| ATOM | 5102 | OG | SER | D | 26 | 0.947 | -38.468 | 94.888 | 1.00 | 40.33 | O |
| ATOM | 5103 | N | PRO | D | 27 | -0.729 | -38.019 | 91.817 | 1.00 | 33.93 | N |
| ATOM | 5104 | CA | PRO | D | 27 | -0.176 | -36.725 | 91.413 | 1.00 | 32.59 | C |
| ATOM | 5105 | C | PRO | D | 27 | -1.180 | -35.587 | 91.464 | 1.00 | 31.97 | C |
| ATOM | 5106 | O | PRO | D | 27 | -2.361 | -35.800 | 91.744 | 1.00 | 29.04 | O |
| ATOM | 5107 | CB | PRO | D | 27 | 0.255 | -36.957 | 89.967 | 1.00 | 25.77 | C |

```
ATOM   5108  CG   PRO D  27      0.295 -38.395  89.799  1.00 30.50           C
ATOM   5109  CD   PRO D  27     -0.695 -38.986  90.712  1.00 33.60           C
ATOM   5110  N    SER D  28     -0.691 -34.384  91.176  1.00 29.51           N
ATOM   5111  CA   SER D  28     -1.531 -33.204  91.247  1.00 33.06           C
ATOM   5112  C    SER D  28     -2.689 -33.306  90.258  1.00 31.21           C
ATOM   5113  O    SER D  28     -2.658 -34.064  89.292  1.00 32.08           O
ATOM   5114  CB   SER D  28     -0.708 -31.948  90.968  1.00 30.39           C
ATOM   5115  OG   SER D  28      0.122 -31.650  92.075  1.00 39.06           O
ATOM   5116  N    VAL D  29     -3.741 -32.552  90.537  1.00 31.26           N
ATOM   5117  CA   VAL D  29     -4.834 -32.398  89.589  1.00 31.02           C
ATOM   5118  C    VAL D  29     -4.828 -30.937  89.154  1.00 30.74           C
ATOM   5119  O    VAL D  29     -5.671 -30.137  89.581  1.00 32.15           O
ATOM   5120  CB   VAL D  29     -6.180 -32.849  90.199  1.00 28.79           C
ATOM   5121  CG1  VAL D  29     -7.331 -32.660  89.220  1.00 29.57           C
ATOM   5122  CG2  VAL D  29     -6.092 -34.296  90.584  1.00 28.20           C
ATOM   5123  N    ASN D  30     -3.867 -30.579  88.300  1.00 29.06           N
ATOM   5124  CA   ASN D  30     -3.706 -29.191  87.881  1.00 27.78           C
ATOM   5125  C    ASN D  30     -4.829 -28.695  86.974  1.00 28.98           C
ATOM   5126  O    ASN D  30     -4.903 -27.494  86.715  1.00 34.22           O
ATOM   5127  CB   ASN D  30     -2.371 -29.012  87.165  1.00 25.95           C
ATOM   5128  CG   ASN D  30     -1.177 -29.247  88.072  1.00 29.67           C
ATOM   5129  OD1  ASN D  30     -1.142 -28.823  89.232  1.00 38.01           O
ATOM   5130  ND2  ASN D  30     -0.188 -29.923  87.546  1.00 31.76           N
ATOM   5131  N    SER D  31     -5.715 -29.565  86.496  1.00 32.77           N
ATOM   5132  CA   SER D  31     -6.854 -29.076  85.733  1.00 29.44           C
ATOM   5133  C    SER D  31     -7.919 -28.456  86.623  1.00 29.07           C
ATOM   5134  O    SER D  31     -8.807 -27.763  86.115  1.00 30.00           O
ATOM   5135  CB   SER D  31     -7.480 -30.207  84.911  1.00 30.70           C
ATOM   5136  OG   SER D  31     -7.863 -31.300  85.728  1.00 28.68           O
ATOM   5137  N    GLY D  32     -7.865 -28.708  87.928  1.00 26.73           N
ATOM   5138  CA   GLY D  32     -8.956 -28.326  88.788  1.00 24.35           C
ATOM   5139  C    GLY D  32    -10.205 -29.141  88.582  1.00 28.63           C
ATOM   5140  O    GLY D  32    -11.275 -28.720  89.003  1.00 23.12           O
ATOM   5141  N    TYR D  33    -10.109 -30.294  87.917  1.00 28.39           N
ATOM   5142  CA   TYR D  33    -11.280 -31.144  87.695  1.00 27.10           C
ATOM   5143  C    TYR D  33    -11.513 -31.989  88.947  1.00 25.88           C
ATOM   5144  O    TYR D  33    -11.254 -33.194  88.991  1.00 26.06           O
ATOM   5145  CB   TYR D  33    -11.095 -32.028  86.468  1.00 27.24           C
ATOM   5146  CG   TYR D  33    -11.026 -31.318  85.121  1.00 30.30           C
ATOM   5147  CD1  TYR D  33    -11.355 -29.972  84.977  1.00 24.29           C
ATOM   5148  CD2  TYR D  33    -10.625 -32.014  83.988  1.00 29.73           C
ATOM   5149  CE1  TYR D  33    -11.272 -29.351  83.739  1.00 29.00           C
ATOM   5150  CE2  TYR D  33    -10.535 -31.403  82.757  1.00 27.95           C
ATOM   5151  CZ   TYR D  33    -10.851 -30.083  82.626  1.00 29.77           C
ATOM   5152  OH   TYR D  33    -10.753 -29.528  81.366  1.00 27.33           O
ATOM   5153  N    LEU D  34    -11.990 -31.331  89.993  1.00 24.59           N
ATOM   5154  CA   LEU D  34    -12.197 -32.008  91.264  1.00 22.23           C
ATOM   5155  C    LEU D  34    -13.626 -31.783  91.730  1.00 22.58           C
ATOM   5156  O    LEU D  34    -14.082 -30.644  91.811  1.00 30.13           O
ATOM   5157  CB   LEU D  34    -11.211 -31.517  92.326  1.00 22.34           C
ATOM   5158  CG   LEU D  34    -11.174 -32.540  93.472  1.00 25.73           C
ATOM   5159  CD1  LEU D  34     -9.862 -33.273  93.510  1.00 24.20           C
ATOM   5160  CD2  LEU D  34    -11.517 -31.936  94.803  1.00 22.15           C
ATOM   5161  N    ALA D  35    -14.331 -32.856  92.019  1.00 19.87           N
ATOM   5162  CA   ALA D  35    -15.711 -32.778  92.454  1.00 21.92           C
ATOM   5163  C    ALA D  35    -15.824 -33.191  93.917  1.00 23.07           C
ATOM   5164  O    ALA D  35    -14.962 -33.884  94.461  1.00 19.99           O
ATOM   5165  CB   ALA D  35    -16.614 -33.657  91.593  1.00 18.13           C
ATOM   5166  N    TRP D  36    -16.908 -32.743  94.544  1.00 22.34           N
ATOM   5167  CA   TRP D  36    -17.251 -33.112  95.911  1.00 25.99           C
ATOM   5168  C    TRP D  36    -18.673 -33.656  95.941  1.00 24.08           C
ATOM   5169  O    TRP D  36    -19.573 -33.115  95.291  1.00 23.80           O
ATOM   5170  CB   TRP D  36    -17.138 -31.920  96.890  1.00 24.75           C
ATOM   5171  CG   TRP D  36    -15.751 -31.504  97.216  1.00 23.08           C
ATOM   5172  CD1  TRP D  36    -15.013 -30.555  96.564  1.00 27.42           C
ATOM   5173  CD2  TRP D  36    -14.917 -32.009  98.268  1.00 23.07           C
ATOM   5174  NE1  TRP D  36    -13.774 -30.439  97.142  1.00 26.35           N
ATOM   5175  CE2  TRP D  36    -13.685 -31.316  98.193  1.00 26.49           C
ATOM   5176  CE3  TRP D  36    -15.096 -32.959  99.275  1.00 23.59           C
ATOM   5177  CZ2  TRP D  36    -12.630 -31.546  99.092  1.00 24.68           C
ATOM   5178  CZ3  TRP D  36    -14.049 -33.189 100.167  1.00 27.30           C
```

```
ATOM   5179  CH2 TRP D  36     -12.829 -32.484 100.064  1.00 27.81           C
ATOM   5180  N   TYR D  37     -18.861 -34.737  96.689  1.00 22.56           N
ATOM   5181  CA  TYR D  37     -20.166 -35.347  96.873  1.00 23.04           C
ATOM   5182  C   TYR D  37     -20.507 -35.457  98.354  1.00 24.32           C
ATOM   5183  O   TYR D  37     -19.638 -35.621  99.213  1.00 21.76           O
ATOM   5184  CB  TYR D  37     -20.222 -36.732  96.232  1.00 22.03           C
ATOM   5185  CG  TYR D  37     -19.937 -36.707  94.768  1.00 22.72           C
ATOM   5186  CD1 TYR D  37     -18.634 -36.802  94.302  1.00 19.01           C
ATOM   5187  CD2 TYR D  37     -20.965 -36.569  93.840  1.00 22.13           C
ATOM   5188  CE1 TYR D  37     -18.355 -36.772  92.962  1.00 20.66           C
ATOM   5189  CE2 TYR D  37     -20.690 -36.541  92.487  1.00 21.78           C
ATOM   5190  CZ  TYR D  37     -19.378 -36.642  92.056  1.00 23.03           C
ATOM   5191  OH  TYR D  37     -19.071 -36.606  90.716  1.00 26.68           O
ATOM   5192  N   GLN D  38     -21.790 -35.368  98.640  1.00 24.80           N
ATOM   5193  CA  GLN D  38     -22.316 -35.653  99.956  1.00 24.70           C
ATOM   5194  C   GLN D  38     -23.084 -36.960  99.888  1.00 25.31           C
ATOM   5195  O   GLN D  38     -23.766 -37.233  98.901  1.00 29.60           O
ATOM   5196  CB  GLN D  38     -23.231 -34.524 100.429  1.00 26.48           C
ATOM   5197  CG  GLN D  38     -23.861 -34.780 101.757  1.00 29.22           C
ATOM   5198  CD  GLN D  38     -24.925 -33.785 102.068  1.00 32.98           C
ATOM   5199  OE1 GLN D  38     -26.040 -33.894 101.560  1.00 39.02           O
ATOM   5200  NE2 GLN D  38     -24.591 -32.780 102.888  1.00 28.25           N
ATOM   5201  N   GLN D  39     -22.956 -37.781 100.916  1.00 22.53           N
ATOM   5202  CA  GLN D  39     -23.703 -39.024 100.962  1.00 27.58           C
ATOM   5203  C   GLN D  39     -24.189 -39.277 102.379  1.00 28.58           C
ATOM   5204  O   GLN D  39     -23.379 -39.372 103.304  1.00 23.07           O
ATOM   5205  CB  GLN D  39     -22.875 -40.207 100.468  1.00 26.29           C
ATOM   5206  CG  GLN D  39     -23.719 -41.455 100.412  1.00 24.76           C
ATOM   5207  CD  GLN D  39     -23.000 -42.610  99.847  1.00 27.83           C
ATOM   5208  OE1 GLN D  39     -21.818 -42.804 100.103  1.00 28.43           O
ATOM   5209  NE2 GLN D  39     -23.697 -43.390  99.042  1.00 31.88           N
ATOM   5210  N   LYS D  40     -25.520 -39.390 102.538  1.00 28.80           N
ATOM   5211  CA  LYS D  40     -26.175 -39.790 103.771  1.00 29.70           C
ATOM   5212  C   LYS D  40     -26.436 -41.289 103.763  1.00 34.10           C
ATOM   5213  O   LYS D  40     -26.510 -41.910 102.691  1.00 30.33           O
ATOM   5214  CB  LYS D  40     -27.478 -39.021 103.946  1.00 31.38           C
ATOM   5215  CG  LYS D  40     -27.272 -37.656 104.529  1.00 32.07           C
ATOM   5216  CD  LYS D  40     -28.432 -36.768 104.221  1.00 42.17           C
ATOM   5217  CE  LYS D  40     -28.195 -35.363 104.744  1.00 44.38           C
ATOM   5218  NZ  LYS D  40     -28.086 -35.382 106.237  1.00 45.69           N1+
ATOM   5219  N   PRO D  41     -26.551 -41.899 104.951  1.00 33.47           N
ATOM   5220  CA  PRO D  41     -26.573 -43.373 105.051  1.00 33.11           C
ATOM   5221  C   PRO D  41     -27.702 -44.029 104.267  1.00 30.28           C
ATOM   5222  O   PRO D  41     -28.872 -43.660 104.394  1.00 32.79           O
ATOM   5223  CB  PRO D  41     -26.726 -43.612 106.558  1.00 34.12           C
ATOM   5224  CG  PRO D  41     -26.096 -42.407 107.186  1.00 25.92           C
ATOM   5225  CD  PRO D  41     -26.471 -41.265 106.280  1.00 26.33           C
ATOM   5226  N   GLY D  42     -27.342 -45.026 103.460  1.00 29.14           N
ATOM   5227  CA  GLY D  42     -28.337 -45.688 102.630  1.00 29.58           C
ATOM   5228  C   GLY D  42     -28.914 -44.852 101.497  1.00 36.52           C
ATOM   5229  O   GLY D  42     -29.956 -45.213 100.952  1.00 36.30           O
ATOM   5230  N   GLN D  43     -28.242 -43.771 101.092  1.00 32.91           N
ATOM   5231  CA  GLN D  43     -28.685 -42.898 100.016  1.00 27.04           C
ATOM   5232  C   GLN D  43     -27.619 -42.825  98.930  1.00 27.93           C
ATOM   5233  O   GLN D  43     -26.452 -43.162  99.153  1.00 27.99           O
ATOM   5234  CB  GLN D  43     -28.952 -41.481 100.523  1.00 30.13           C
ATOM   5235  CG  GLN D  43     -29.659 -41.422 101.838  1.00 33.40           C
ATOM   5236  CD  GLN D  43     -31.073 -41.941 101.754  1.00 45.27           C
ATOM   5237  OE1 GLN D  43     -31.760 -41.731 100.750  1.00 42.82           O
ATOM   5238  NE2 GLN D  43     -31.511 -42.665 102.800  1.00 44.71           N
ATOM   5239  N   THR D  44     -28.032 -42.367  97.742  1.00 25.68           N
ATOM   5240  CA  THR D  44     -27.045 -42.187  96.692  1.00 30.33           C
ATOM   5241  C   THR D  44     -26.306 -40.848  96.866  1.00 30.71           C
ATOM   5242  O   THR D  44     -26.834 -39.904  97.464  1.00 26.05           O
ATOM   5243  CB  THR D  44     -27.702 -42.278  95.303  1.00 28.63           C
ATOM   5244  OG1 THR D  44     -28.240 -41.013  94.924  1.00 32.11           O
ATOM   5245  CG2 THR D  44     -28.834 -43.319  95.292  1.00 29.12           C
ATOM   5246  N   PRO D  45     -25.070 -40.756  96.375  1.00 25.93           N
ATOM   5247  CA  PRO D  45     -24.312 -39.512  96.515  1.00 25.74           C
ATOM   5248  C   PRO D  45     -24.977 -38.343  95.801  1.00 24.98           C
ATOM   5249  O   PRO D  45     -25.616 -38.493  94.761  1.00 23.19           O
```

466

```
ATOM   5250  CB  PRO D  45     -22.962 -39.857  95.878  1.00 28.44           C
ATOM   5251  CG  PRO D  45     -22.886 -41.342  95.963  1.00 23.47           C
ATOM   5252  CD  PRO D  45     -24.265 -41.820  95.764  1.00 23.77           N
ATOM   5253  N   ARG D  46     -24.812 -37.163  96.382  1.00 28.06           N
ATOM   5254  CA  ARG D  46     -25.306 -35.910  95.824  1.00 26.21           C
ATOM   5255  C   ARG D  46     -24.120 -35.028  95.449  1.00 27.60           C
ATOM   5256  O   ARG D  46     -23.212 -34.814  96.265  1.00 24.95           O
ATOM   5257  CB  ARG D  46     -26.203 -35.199  96.836  1.00 28.93           C
ATOM   5258  CG  ARG D  46     -26.747 -33.885  96.372  1.00 35.19           C
ATOM   5259  CD  ARG D  46     -28.129 -33.660  96.926  1.00 42.94           C
ATOM   5260  NE  ARG D  46     -28.813 -32.616  96.172  1.00 54.78           N
ATOM   5261  CZ  ARG D  46     -28.994 -31.379  96.619  1.00 62.11           C
ATOM   5262  NH1 ARG D  46     -29.621 -30.481  95.858  1.00 53.78           N1+
ATOM   5263  NH2 ARG D  46     -28.553 -31.045  97.837  1.00 63.79           N
ATOM   5264  N   LEU D  47     -24.116 -34.535  94.216  1.00 26.63           N
ATOM   5265  CA  LEU D  47     -23.029 -33.680  93.772  1.00 24.56           C
ATOM   5266  C   LEU D  47     -23.125 -32.333  94.474  1.00 23.50           C
ATOM   5267  O   LEU D  47     -24.201 -31.746  94.529  1.00 28.69           O
ATOM   5268  CB  LEU D  47     -23.083 -33.508  92.264  1.00 21.59           C
ATOM   5269  CG  LEU D  47     -22.042 -32.542  91.689  1.00 28.11           C
ATOM   5270  CD1 LEU D  47     -20.587 -33.047  91.831  1.00 23.37           C
ATOM   5271  CD2 LEU D  47     -22.396 -32.293  90.251  1.00 26.58           C
ATOM   5272  N   LEU D  48     -22.020 -31.872  95.064  1.00 25.20           N
ATOM   5273  CA  LEU D  48     -21.967 -30.576  95.756  1.00 29.85           C
ATOM   5274  C   LEU D  48     -21.153 -29.530  95.021  1.00 28.28           C
ATOM   5275  O   LEU D  48     -21.553 -28.371  94.962  1.00 26.16           O
ATOM   5276  CB  LEU D  48     -21.354 -30.705  97.156  1.00 24.38           C
ATOM   5277  CG  LEU D  48     -22.032 -31.429  98.300  1.00 29.64           C
ATOM   5278  CD1 LEU D  48     -21.124 -31.235  99.476  1.00 28.92           C
ATOM   5279  CD2 LEU D  48     -23.421 -30.868  98.604  1.00 30.54           C
ATOM   5280  N   ILE D  49     -19.998 -29.933  94.505  1.00 24.89           N
ATOM   5281  CA  ILE D  49     -19.008 -29.059  93.903  1.00 25.06           C
ATOM   5282  C   ILE D  49     -18.484 -29.781  92.672  1.00 28.83           C
ATOM   5283  O   ILE D  49     -18.212 -30.986  92.726  1.00 25.60           O
ATOM   5284  CB  ILE D  49     -17.833 -28.787  94.879  1.00 27.73           C
ATOM   5285  CG1 ILE D  49     -18.307 -28.181  96.214  1.00 22.96           C
ATOM   5286  CG2 ILE D  49     -16.678 -28.055  94.176  1.00 21.77           C
ATOM   5287  CD1 ILE D  49     -18.656 -26.704  96.180  1.00 28.66           C
ATOM   5288  N   PHE D  50     -18.308 -29.049  91.573  1.00 25.66           N
ATOM   5289  CA  PHE D  50     -17.533 -29.563  90.460  1.00 23.52           C
ATOM   5290  C   PHE D  50     -16.420 -28.576  90.149  1.00 30.77           C
ATOM   5291  O   PHE D  50     -16.505 -27.377  90.451  1.00 26.94           O
ATOM   5292  CB  PHE D  50     -18.381 -29.861  89.202  1.00 25.10           C
ATOM   5293  CG  PHE D  50     -19.084 -28.669  88.645  1.00 29.89           C
ATOM   5294  CD2 PHE D  50     -20.394 -28.380  89.034  1.00 28.85           C
ATOM   5295  CD1 PHE D  50     -18.460 -27.848  87.720  1.00 26.32           C
ATOM   5296  CE2 PHE D  50     -21.038 -27.263  88.538  1.00 33.45           C
ATOM   5297  CE1 PHE D  50     -19.111 -26.747  87.212  1.00 31.34           C
ATOM   5298  CZ  PHE D  50     -20.412 -26.457  87.616  1.00 33.07           C
ATOM   5299  N   GLY D  51     -15.381 -29.125  89.536  1.00 29.64           N
ATOM   5300  CA  GLY D  51     -14.110 -28.473  89.344  1.00 31.07           C
ATOM   5301  C   GLY D  51     -13.912 -27.013  89.635  1.00 29.90           C
ATOM   5302  O   GLY D  51     -14.299 -26.190  88.823  1.00 41.98           O
ATOM   5303  N   ALA D  52     -13.331 -26.633  90.761  1.00 29.04           N
ATOM   5304  CA  ALA D  52     -13.045 -27.404  91.963  1.00 27.42           C
ATOM   5305  C   ALA D  52     -13.634 -26.483  93.033  1.00 31.29           C
ATOM   5306  O   ALA D  52     -13.389 -26.630  94.242  1.00 26.21           O
ATOM   5307  CB  ALA D  52     -11.545 -27.613  92.180  1.00 24.46           C
ATOM   5308  N   SER D  53     -14.356 -25.473  92.528  1.00 25.45           N
ATOM   5309  CA  SER D  53     -14.951 -24.408  93.321  1.00 29.64           C
ATOM   5310  C   SER D  53     -16.366 -24.061  92.909  1.00 29.31           C
ATOM   5311  O   SER D  53     -17.001 -23.251  93.595  1.00 29.52           O
ATOM   5312  CB  SER D  53     -14.102 -23.144  93.229  1.00 24.22           C
ATOM   5313  OG  SER D  53     -13.751 -22.931  91.878  1.00 30.08           O
ATOM   5314  N   SER D  54     -16.872 -24.618  91.819  1.00 24.59           N
ATOM   5315  CA  SER D  54     -18.183 -24.249  91.320  1.00 28.33           C
ATOM   5316  C   SER D  54     -19.261 -25.017  92.073  1.00 29.42           C
ATOM   5317  O   SER D  54     -19.261 -26.252  92.107  1.00 28.83           O
ATOM   5318  CB  SER D  54     -18.294 -24.510  89.818  1.00 30.14           C
ATOM   5319  OG  SER D  54     -17.503 -23.611  89.086  1.00 31.63           O
ATOM   5320  N   ARG D  55     -20.185 -24.274  92.653  1.00 26.56           N
```

```
ATOM   5321  CA   ARG D  55      -21.342 -24.858  93.293  1.00 26.99           C
ATOM   5322  C    ARG D  55      -22.293 -25.465  92.261  1.00 31.42           C
ATOM   5323  O    ARG D  55      -22.594 -24.854  91.230  1.00 34.25           O
ATOM   5324  CB   ARG D  55      -22.033 -23.773  94.100  1.00 31.93           C
ATOM   5325  CG   ARG D  55      -22.782 -24.249  95.296  1.00 39.96           C
ATOM   5326  CD   ARG D  55      -23.378 -23.054  96.043  1.00 42.71           C
ATOM   5327  NE   ARG D  55      -22.393 -22.357  96.853  1.00 39.62           N
ATOM   5328  CZ   ARG D  55      -21.943 -21.142  96.574  1.00 42.81           C
ATOM   5329  NH1  ARG D  55      -21.042 -20.564  97.367  1.00 46.41           N1+
ATOM   5330  NH2  ARG D  55      -22.396 -20.516  95.497  1.00 41.95           N
ATOM   5331  N    ALA D  56      -22.783 -26.668  92.562  1.00 29.25           N
ATOM   5332  CA   ALA D  56      -23.814 -27.344  91.783  1.00 34.27           C
ATOM   5333  C    ALA D  56      -25.176 -26.678  91.997  1.00 36.12           C
ATOM   5334  O    ALA D  56      -25.355 -25.830  92.877  1.00 36.11           O
ATOM   5335  CB   ALA D  56      -23.891 -28.823  92.166  1.00 31.23           C
ATOM   5336  N    THR D  57      -26.154 -27.081  91.184  1.00 36.05           N
ATOM   5337  CA   THR D  57      -27.475 -26.471  91.274  1.00 46.10           C
ATOM   5338  C    THR D  57      -28.178 -26.905  92.546  1.00 41.98           C
ATOM   5339  O    THR D  57      -28.091 -28.063  92.960  1.00 44.69           O
ATOM   5340  CB   THR D  57      -28.345 -26.861  90.081  1.00 48.23           C
ATOM   5341  OG1  THR D  57      -27.504 -27.188  88.968  1.00 51.51           O
ATOM   5342  CG2  THR D  57      -29.281 -25.707  89.713  1.00 39.36           C
ATOM   5343  N    GLY D  58      -28.898 -25.966  93.148  1.00 36.11           N
ATOM   5344  CA   GLY D  58      -29.589 -26.198  94.396  1.00 36.25           C
ATOM   5345  C    GLY D  58      -28.722 -26.367  95.629  1.00 35.95           C
ATOM   5346  O    GLY D  58      -29.266 -26.615  96.706  1.00 46.21           O
ATOM   5347  N    ILE D  59      -27.406 -26.270  95.526  1.00 35.23           N
ATOM   5348  CA   ILE D  59      -26.552 -26.443  96.707  1.00 37.35           C
ATOM   5349  C    ILE D  59      -26.499 -25.124  97.480  1.00 32.79           C
ATOM   5350  O    ILE D  59      -26.110 -24.102  96.902  1.00 34.26           O
ATOM   5351  CB   ILE D  59      -25.147 -26.884  96.291  1.00 34.60           C
ATOM   5352  CG1  ILE D  59      -25.150 -28.289  95.658  1.00 33.46           C
ATOM   5353  CG2  ILE D  59      -24.257 -26.892  97.511  1.00 28.92           C
ATOM   5354  CD1  ILE D  59      -25.990 -29.321  96.371  1.00 33.20           C
ATOM   5355  N    PRO D  60      -26.822 -25.098  98.776  1.00 35.94           N
ATOM   5356  CA   PRO D  60      -26.753 -23.831  99.528  1.00 36.87           C
ATOM   5357  C    PRO D  60      -25.384 -23.165  99.443  1.00 39.65           C
ATOM   5358  O    PRO D  60      -24.357 -23.825  99.258  1.00 40.06           O
ATOM   5359  CB   PRO D  60      -27.068 -24.261 100.967  1.00 39.32           C
ATOM   5360  CG   PRO D  60      -27.875 -25.486 100.816  1.00 40.27           C
ATOM   5361  CD   PRO D  60      -27.346 -26.199  99.601  1.00 37.18           C
ATOM   5362  N    ASP D  61      -25.355 -21.841  99.615  1.00 37.82           N
ATOM   5363  CA   ASP D  61      -24.047 -21.197  99.529  1.00 38.85           C
ATOM   5364  C    ASP D  61      -23.271 -21.286 100.830  1.00 38.37           C
ATOM   5365  O    ASP D  61      -22.161 -20.757 100.895  1.00 43.54           O
ATOM   5366  CB   ASP D  61      -24.153 -19.736  99.070  1.00 39.37           C
ATOM   5367  CG   ASP D  61      -25.124 -18.914  99.898  1.00 54.57           C
ATOM   5368  OD1  ASP D  61      -25.360 -19.253 101.082  1.00 61.72           O
ATOM   5369  OD2  ASP D  61      -25.635 -17.901  99.363  1.00 51.55           O1-
ATOM   5370  N    ARG D  62      -23.833 -21.947 101.850  1.00 39.40           N
ATOM   5371  CA   ARG D  62      -23.032 -22.496 102.939  1.00 36.54           C
ATOM   5372  C    ARG D  62      -21.805 -23.229 102.414  1.00 34.81           C
ATOM   5373  O    ARG D  62      -20.738 -23.199 103.037  1.00 31.24           O
ATOM   5374  CB   ARG D  62      -23.837 -23.511 103.745  1.00 37.64           C
ATOM   5375  CG   ARG D  62      -25.054 -23.076 104.366  1.00 36.64           C
ATOM   5376  CD   ARG D  62      -25.298 -24.006 105.536  1.00 40.91           C
ATOM   5377  NE   ARG D  62      -25.716 -25.370 105.214  1.00 38.63           N
ATOM   5378  CZ   ARG D  62      -26.848 -25.692 104.593  1.00 43.76           C
ATOM   5379  NH1  ARG D  62      -27.662 -24.743 104.160  1.00 45.94           N1+
ATOM   5380  NH2  ARG D  62      -27.159 -26.964 104.383  1.00 41.39           N
ATOM   5381  N    PHE D  63      -21.980 -23.979 101.323  1.00 34.78           N
ATOM   5382  CA   PHE D  63      -20.936 -24.809 100.738  1.00 34.76           C
ATOM   5383  C    PHE D  63      -20.101 -23.966  99.797  1.00 32.41           C
ATOM   5384  O    PHE D  63      -20.646 -23.271  98.933  1.00 35.79           O
ATOM   5385  CB   PHE D  63      -21.536 -26.013  99.992  1.00 29.42           C
ATOM   5386  CG   PHE D  63      -22.210 -27.001 100.898  1.00 28.36           C
ATOM   5387  CD1  PHE D  63      -23.504 -26.790 101.344  1.00 31.54           C
ATOM   5388  CD2  PHE D  63      -21.537 -28.120 101.336  1.00 28.75           C
ATOM   5389  CE1  PHE D  63      -24.117 -27.686 102.190  1.00 29.90           C
ATOM   5390  CE2  PHE D  63      -22.149 -29.017 102.180  1.00 31.42           C
ATOM   5391  CZ   PHE D  63      -23.441 -28.796 102.611  1.00 27.00           C
```

| ATOM | 5392 | N | SER | D | 64 | -18.783 | -24.021 | 99.986 | 1.00 | 32.03 | N |
|------|------|------|------|---|----|---------|---------|--------|------|-------|------|
| ATOM | 5393 | CA | SER | D | 64 | -17.815 | -23.408 | 99.081 | 1.00 | 33.91 | C |
| ATOM | 5394 | C | SER | D | 64 | -16.566 | -24.279 | 99.051 | 1.00 | 26.96 | C |
| ATOM | 5395 | O | SER | D | 64 | -16.345 | -25.111 | 99.934 | 1.00 | 29.99 | O |
| ATOM | 5396 | CB | SER | D | 64 | -17.482 | -21.975 | 99.511 | 1.00 | 31.16 | C |
| ATOM | 5397 | OG | SER | D | 64 | -16.576 | -21.995 | 100.592 | 1.00 | 35.76 | O |
| ATOM | 5398 | N | ALA | D | 65 | -15.766 | -24.128 | 98.007 | 1.00 | 21.94 | N |
| ATOM | 5399 | CA | ALA | D | 65 | -14.588 | -24.982 | 97.928 | 1.00 | 27.79 | C |
| ATOM | 5400 | C | ALA | D | 65 | -13.547 | -24.302 | 97.063 | 1.00 | 28.20 | C |
| ATOM | 5401 | O | ALA | D | 65 | -13.859 | -23.392 | 96.297 | 1.00 | 28.35 | O |
| ATOM | 5402 | CB | ALA | D | 65 | -14.930 | -26.370 | 97.382 | 1.00 | 26.70 | C |
| ATOM | 5403 | N | SER | D | 66 | -12.305 | -24.767 | 97.185 | 1.00 | 31.90 | N |
| ATOM | 5404 | CA | SER | D | 66 | -11.196 | -24.189 | 96.432 | 1.00 | 32.71 | C |
| ATOM | 5405 | C | SER | D | 66 | -10.001 | -25.141 | 96.468 | 1.00 | 32.60 | C |
| ATOM | 5406 | O | SER | D | 66 | -10.005 | -26.170 | 97.149 | 1.00 | 34.80 | O |
| ATOM | 5407 | CB | SER | D | 66 | -10.787 | -22.831 | 97.003 | 1.00 | 27.36 | C |
| ATOM | 5408 | OG | SER | D | 66 | -10.156 | -23.031 | 98.253 | 1.00 | 32.54 | O |
| ATOM | 5409 | N | GLY | D | 67 | -8.948 | -24.745 | 95.780 | 1.00 | 31.68 | N |
| ATOM | 5410 | CA | GLY | D | 67 | -7.719 | -25.498 | 95.795 | 1.00 | 28.89 | C |
| ATOM | 5411 | C | GLY | D | 67 | -7.316 | -25.711 | 94.364 | 1.00 | 38.37 | C |
| ATOM | 5412 | O | GLY | D | 67 | -8.135 | -25.485 | 93.466 | 1.00 | 39.57 | O |
| ATOM | 5413 | N | SER | D | 68 | -6.057 | -26.066 | 94.125 | 1.00 | 36.68 | N |
| ATOM | 5414 | CA | SER | D | 68 | -5.677 | -26.559 | 92.813 | 1.00 | 36.53 | C |
| ATOM | 5415 | C | SER | D | 68 | -4.366 | -27.296 | 92.959 | 1.00 | 30.07 | C |
| ATOM | 5416 | O | SER | D | 68 | -3.660 | -27.148 | 93.952 | 1.00 | 38.18 | O |
| ATOM | 5417 | CB | SER | D | 68 | -5.559 | -25.449 | 91.757 | 1.00 | 44.96 | C |
| ATOM | 5418 | OG | SER | D | 68 | -5.439 | -26.014 | 90.444 | 1.00 | 48.06 | O |
| ATOM | 5419 | N | GLY | D | 69 | -4.062 | -28.097 | 91.954 | 1.00 | 29.21 | N |
| ATOM | 5420 | CA | GLY | D | 69 | -2.896 | -28.929 | 91.987 | 1.00 | 29.40 | C |
| ATOM | 5421 | C | GLY | D | 69 | -3.069 | -30.067 | 92.949 | 1.00 | 31.99 | C |
| ATOM | 5422 | O | GLY | D | 69 | -3.815 | -31.018 | 92.687 | 1.00 | 33.63 | O |
| ATOM | 5423 | N | ALA | D | 70 | -2.379 | -29.966 | 94.080 | 1.00 | 31.64 | N |
| ATOM | 5424 | CA | ALA | D | 70 | -2.287 | -31.065 | 95.022 | 1.00 | 31.80 | C |
| ATOM | 5425 | C | ALA | D | 70 | -3.275 | -30.980 | 96.171 | 1.00 | 31.37 | C |
| ATOM | 5426 | O | ALA | D | 70 | -3.550 | -32.008 | 96.792 | 1.00 | 36.24 | O |
| ATOM | 5427 | CB | ALA | D | 70 | -0.868 | -31.140 | 95.594 | 1.00 | 28.13 | C |
| ATOM | 5428 | N | ASP | D | 71 | -3.817 | -29.803 | 96.471 | 1.00 | 30.57 | N |
| ATOM | 5429 | CA | ASP | D | 71 | -4.565 | -29.587 | 97.706 | 1.00 | 33.18 | C |
| ATOM | 5430 | C | ASP | D | 71 | -5.945 | -29.009 | 97.415 | 1.00 | 32.16 | C |
| ATOM | 5431 | O | ASP | D | 71 | -6.063 | -27.965 | 96.768 | 1.00 | 31.72 | O |
| ATOM | 5432 | CB | ASP | D | 71 | -3.800 | -28.638 | 98.622 | 1.00 | 31.72 | C |
| ATOM | 5433 | CG | ASP | D | 71 | -2.722 | -29.331 | 99.396 | 1.00 | 47.35 | C |
| ATOM | 5434 | OD1 | ASP | D | 71 | -3.025 | -30.000 | 100.408 | 1.00 | 54.93 | O |
| ATOM | 5435 | OD2 | ASP | D | 71 | -1.553 | -29.211 | 98.980 | 1.00 | 57.78 | O1- |
| ATOM | 5436 | N | PHE | D | 72 | -6.981 | -29.643 | 97.939 | 1.00 | 28.95 | N |
| ATOM | 5437 | CA | PHE | D | 72 | -8.335 | -29.145 | 97.771 | 1.00 | 25.33 | C |
| ATOM | 5438 | C | PHE | D | 72 | -8.994 | -29.122 | 99.135 | 1.00 | 24.96 | C |
| ATOM | 5439 | O | PHE | D | 72 | -8.687 | -29.939 | 100.005 | 1.00 | 21.64 | O |
| ATOM | 5440 | CB | PHE | D | 72 | -9.121 | -30.008 | 96.781 | 1.00 | 26.11 | C |
| ATOM | 5441 | CG | PHE | D | 72 | -8.492 | -30.051 | 95.425 | 1.00 | 30.35 | C |
| ATOM | 5442 | CD1 | PHE | D | 72 | -7.479 | -30.982 | 95.144 | 1.00 | 29.20 | C |
| ATOM | 5443 | CD2 | PHE | D | 72 | -8.862 | -29.139 | 94.444 | 1.00 | 27.20 | C |
| ATOM | 5444 | CE1 | PHE | D | 72 | -6.862 | -31.014 | 93.898 | 1.00 | 31.22 | C |
| ATOM | 5445 | CE2 | PHE | D | 72 | -8.250 | -29.161 | 93.189 | 1.00 | 30.03 | C |
| ATOM | 5446 | CZ | PHE | D | 72 | -7.251 | -30.105 | 92.913 | 1.00 | 29.83 | C |
| ATOM | 5447 | N | THR | D | 73 | -9.842 | -28.133 | 99.354 | 1.00 | 23.98 | N |
| ATOM | 5448 | CA | THR | D | 73 | -10.593 | -28.118 | 100.589 | 1.00 | 27.63 | C |
| ATOM | 5449 | C | THR | D | 73 | -12.045 | -27.791 | 100.275 | 1.00 | 28.02 | C |
| ATOM | 5450 | O | THR | D | 73 | -12.363 | -27.129 | 99.275 | 1.00 | 25.48 | O |
| ATOM | 5451 | CB | THR | D | 73 | -9.993 | -27.156 | 101.661 | 1.00 | 32.96 | C |
| ATOM | 5452 | OG1 | THR | D | 73 | -10.611 | -25.872 | 101.585 | 1.00 | 38.47 | O |
| ATOM | 5453 | CG2 | THR | D | 73 | -8.473 | -26.987 | 101.511 | 1.00 | 24.84 | C |
| ATOM | 5454 | N | LEU | D | 74 | -12.926 | -28.368 | 101.090 | 1.00 | 30.82 | N |
| ATOM | 5455 | CA | LEU | D | 74 | -14.351 | -28.062 | 101.114 | 1.00 | 26.16 | C |
| ATOM | 5456 | C | LEU | D | 74 | -14.662 | -27.367 | 102.433 | 1.00 | 27.40 | C |
| ATOM | 5457 | O | LEU | D | 74 | -14.247 | -27.843 | 103.499 | 1.00 | 27.12 | O |
| ATOM | 5458 | CB | LEU | D | 74 | -15.184 | -29.336 | 100.953 | 1.00 | 21.42 | C |
| ATOM | 5459 | CG | LEU | D | 74 | -16.696 | -29.239 | 101.152 | 1.00 | 21.05 | C |
| ATOM | 5460 | CD1 | LEU | D | 74 | -17.360 | -28.535 | 100.013 | 1.00 | 24.58 | C |
| ATOM | 5461 | CD2 | LEU | D | 74 | -17.329 | -30.584 | 101.345 | 1.00 | 20.48 | C |
| ATOM | 5462 | N | THR | D | 75 | -15.378 | -26.243 | 102.365 | 1.00 | 24.12 | N |

```
ATOM   5463  CA  THR D  75     -15.762 -25.486 103.548  1.00 24.98          C
ATOM   5464  C   THR D  75     -17.286 -25.435 103.651  1.00 28.91          C
ATOM   5465  O   THR D  75     -17.971 -25.077 102.687  1.00 33.13          O
ATOM   5466  CB  THR D  75     -15.167 -24.066 103.513  1.00 29.48          C
ATOM   5467  OG1 THR D  75     -13.776 -24.123 103.842  1.00 31.85          O
ATOM   5468  CG2 THR D  75     -15.840 -23.161 104.544  1.00 30.01          C
ATOM   5469  N   ILE D  76     -17.820 -25.840 104.799  1.00 25.85          N
ATOM   5470  CA  ILE D  76     -19.217 -25.616 105.141  1.00 25.76          C
ATOM   5471  C   ILE D  76     -19.211 -24.568 106.232  1.00 30.23          C
ATOM   5472  O   ILE D  76     -18.718 -24.829 107.337  1.00 34.43          O
ATOM   5473  CB  ILE D  76     -19.922 -26.893 105.619  1.00 28.28          C
ATOM   5474  CG1 ILE D  76     -19.619 -28.063 104.696  1.00 28.54          C
ATOM   5475  CG2 ILE D  76     -21.443 -26.672 105.686  1.00 28.62          C
ATOM   5476  CD1 ILE D  76     -20.092 -29.368 105.257  1.00 27.59          C
ATOM   5477  N   SER D  77     -19.775 -23.395 105.945  1.00 30.77          N
ATOM   5478  CA  SER D  77     -19.475 -22.251 106.803  1.00 33.53          C
ATOM   5479  C   SER D  77     -20.286 -22.267 108.097  1.00 32.03          C
ATOM   5480  O   SER D  77     -19.781 -21.840 109.141  1.00 35.78          O
ATOM   5481  CB  SER D  77     -19.681 -20.943 106.038  1.00 25.30          C
ATOM   5482  OG  SER D  77     -21.029 -20.795 105.672  1.00 30.61          O
ATOM   5483  N   ARG D  78     -21.539 -22.727 108.051  1.00 34.30          N
ATOM   5484  CA  ARG D  78     -22.346 -22.937 109.254  1.00 33.67          C
ATOM   5485  C   ARG D  78     -23.026 -24.294 109.107  1.00 33.98          C
ATOM   5486  O   ARG D  78     -23.982 -24.422 108.341  1.00 30.96          O
ATOM   5487  CB  ARG D  78     -23.387 -21.830 109.423  1.00 34.14          C
ATOM   5488  CG  ARG D  78     -24.425 -22.137 110.499  1.00 46.20          C
ATOM   5489  CD  ARG D  78     -25.375 -20.962 110.761  1.00 46.68          C
ATOM   5490  NE  ARG D  78     -25.737 -20.874 112.185  1.00 49.36          N
ATOM   5491  CZ  ARG D  78     -26.801 -21.439 112.763  1.00 57.02          C
ATOM   5492  NH1 ARG D  78     -27.008 -21.283 114.074  1.00 54.06          N1+
ATOM   5493  NH2 ARG D  78     -27.666 -22.153 112.047  1.00 59.27          N
ATOM   5494  N   LEU D  79     -22.616 -25.273 109.904  1.00 31.63          N
ATOM   5495  CA  LEU D  79     -23.146 -26.627 109.772  1.00 29.40          C
ATOM   5496  C   LEU D  79     -24.623 -26.674 110.172  1.00 34.83          C
ATOM   5497  O   LEU D  79     -24.969 -26.417 111.328  1.00 36.97          O
ATOM   5498  CB  LEU D  79     -22.333 -27.580 110.640  1.00 26.40          C
ATOM   5499  CG  LEU D  79     -21.373 -28.630 110.107  1.00 26.42          C
ATOM   5500  CD1 LEU D  79     -21.113 -28.496 108.643  1.00 29.85          C
ATOM   5501  CD2 LEU D  79     -20.083 -28.535 110.868  1.00 29.71          C
ATOM   5502  N   GLU D  80     -25.507 -26.990 109.211  1.00 35.29          N
ATOM   5503  CA  GLU D  80     -26.911 -27.234 109.503  1.00 33.25          C
ATOM   5504  C   GLU D  80     -27.130 -28.711 109.784  1.00 37.31          C
ATOM   5505  O   GLU D  80     -26.269 -29.536 109.472  1.00 34.23          O
ATOM   5506  CB  GLU D  80     -27.771 -26.790 108.334  1.00 37.10          C
ATOM   5507  CG  GLU D  80     -27.822 -25.300 108.123  1.00 37.33          C
ATOM   5508  CD  GLU D  80     -28.430 -24.559 109.292  1.00 45.25          C
ATOM   5509  OE1 GLU D  80     -29.311 -25.110 109.996  1.00 48.39          O
ATOM   5510  OE2 GLU D  80     -28.039 -23.395 109.496  1.00 54.67          O1-
ATOM   5511  N   PRO D  81     -28.261 -29.088 110.403  1.00 43.56          N
ATOM   5512  CA  PRO D  81     -28.458 -30.514 110.743  1.00 45.48          C
ATOM   5513  C   PRO D  81     -28.252 -31.464 109.571  1.00 42.65          C
ATOM   5514  O   PRO D  81     -27.600 -32.508 109.728  1.00 39.13          O
ATOM   5515  CB  PRO D  81     -29.909 -30.547 111.254  1.00 44.07          C
ATOM   5516  CG  PRO D  81     -30.130 -29.185 111.794  1.00 40.26          C
ATOM   5517  CD  PRO D  81     -29.382 -28.259 110.884  1.00 41.88          C
ATOM   5518  N   GLU D  82     -28.761 -31.107 108.389  1.00 40.42          N
ATOM   5519  CA  GLU D  82     -28.620 -31.911 107.178  1.00 37.02          C
ATOM   5520  C   GLU D  82     -27.222 -31.924 106.602  1.00 38.11          C
ATOM   5521  O   GLU D  82     -27.053 -32.461 105.498  1.00 37.79          O
ATOM   5522  CB  GLU D  82     -29.528 -31.396 106.079  1.00 34.91          C
ATOM   5523  CG  GLU D  82     -29.092 -30.088 105.479  1.00 42.26          C
ATOM   5524  CD  GLU D  82     -30.094 -28.977 105.612  1.00 49.69          C
ATOM   5525  OE1 GLU D  82     -30.683 -28.801 106.716  1.00 59.37          O
ATOM   5526  OE2 GLU D  82     -30.297 -28.286 104.582  1.00 55.07          O1-
ATOM   5527  N   ASP D  83     -26.234 -31.314 107.246  1.00 31.73          N
ATOM   5528  CA  ASP D  83     -24.893 -31.308 106.690  1.00 31.81          C
ATOM   5529  C   ASP D  83     -24.033 -32.399 107.278  1.00 27.67          C
ATOM   5530  O   ASP D  83     -22.905 -32.577 106.833  1.00 28.90          O
ATOM   5531  CB  ASP D  83     -24.205 -29.949 106.911  1.00 35.00          C
ATOM   5532  CG  ASP D  83     -24.923 -28.802 106.203  1.00 36.30          C
ATOM   5533  OD1 ASP D  83     -25.767 -29.091 105.334  1.00 39.93          O
```

```
ATOM   5534   OD2  ASP  D   83      -24.663 -27.615 106.524   1.00 33.84          O1-
ATOM   5535   N    PHE  D   84      -24.531 -33.133 108.262   1.00 28.52          N
ATOM   5536   CA   PHE  D   84      -23.736 -34.160 108.921   1.00 27.69          C
ATOM   5537   C    PHE  D   84      -23.892 -35.469 108.164   1.00 27.68          C
ATOM   5538   O    PHE  D   84      -24.963 -36.083 108.176   1.00 27.12          O
ATOM   5539   CB   PHE  D   84      -24.127 -34.276 110.391   1.00 26.49          C
ATOM   5540   CG   PHE  D   84      -23.758 -33.065 111.194   1.00 27.03          C
ATOM   5541   CD1  PHE  D   84      -22.520 -32.992 111.822   1.00 27.26          C
ATOM   5542   CD2  PHE  D   84      -24.628 -31.981 111.289   1.00 30.67          C
ATOM   5543   CE1  PHE  D   84      -22.156 -31.865 112.562   1.00 31.27          C
ATOM   5544   CE2  PHE  D   84      -24.281 -30.849 112.016   1.00 29.02          C
ATOM   5545   CZ   PHE  D   84      -23.039 -30.794 112.667   1.00 32.36          C
ATOM   5546   N    ALA  D   85      -22.815 -35.890 107.516   1.00 25.77          N
ATOM   5547   CA   ALA  D   85      -22.859 -36.899 106.469   1.00 21.15          C
ATOM   5548   C    ALA  D   85      -21.420 -37.274 106.118   1.00 22.96          C
ATOM   5549   O    ALA  D   85      -20.472 -36.875 106.800   1.00 22.25          O
ATOM   5550   CB   ALA  D   85      -23.660 -36.381 105.275   1.00 25.38          C
ATOM   5551   N    VAL  D   86      -21.251 -38.069 105.059   1.00 25.06          N
ATOM   5552   CA   VAL  D   86      -19.939 -38.399 104.521   1.00 20.70          C
ATOM   5553   C    VAL  D   86      -19.708 -37.553 103.278   1.00 22.14          C
ATOM   5554   O    VAL  D   86      -20.628 -37.341 102.488   1.00 27.68          O
ATOM   5555   CB   VAL  D   86      -19.841 -39.901 104.217   1.00 19.64          C
ATOM   5556   CG1  VAL  D   86      -18.533 -40.230 103.523   1.00 22.34          C
ATOM   5557   CG2  VAL  D   86      -19.953 -40.660 105.485   1.00 16.64          C
ATOM   5558   N    TYR  D   87      -18.497 -37.033 103.120   1.00 21.19          N
ATOM   5559   CA   TYR  D   87      -18.151 -36.206 101.975   1.00 24.17          C
ATOM   5560   C    TYR  D   87      -17.024 -36.879 101.219   1.00 25.06          C
ATOM   5561   O    TYR  D   87      -16.084 -37.361 101.837   1.00 25.52          O
ATOM   5562   CB   TYR  D   87      -17.765 -34.766 102.410   1.00 25.82          C
ATOM   5563   CG   TYR  D   87      -18.963 -34.036 102.978   1.00 24.53          C
ATOM   5564   CD1  TYR  D   87      -19.368 -34.249 104.286   1.00 22.98          C
ATOM   5565   CD2  TYR  D   87      -19.735 -33.198 102.178   1.00 22.74          C
ATOM   5566   CE1  TYR  D   87      -20.502 -33.616 104.791   1.00 27.82          C
ATOM   5567   CE2  TYR  D   87      -20.857 -32.573 102.662   1.00 22.11          C
ATOM   5568   CZ   TYR  D   87      -21.244 -32.777 103.966   1.00 28.03          C
ATOM   5569   OH   TYR  D   87      -22.379 -32.154 104.449   1.00 29.25          O
ATOM   5570   N    PHE  D   88      -17.140 -36.897  99.885   1.00 22.99          N
ATOM   5571   CA   PHE  D   88      -16.222 -37.590  98.990   1.00 25.06          C
ATOM   5572   C    PHE  D   88      -15.653 -36.649  97.952   1.00 27.58          C
ATOM   5573   O    PHE  D   88      -16.403 -35.890  97.327   1.00 28.74          O
ATOM   5574   CB   PHE  D   88      -16.920 -38.715  98.216   1.00 29.50          C
ATOM   5575   CG   PHE  D   88      -17.173 -39.937  99.013   1.00 30.12          C
ATOM   5576   CD1  PHE  D   88      -16.133 -40.820  99.283   1.00 27.68          C
ATOM   5577   CD2  PHE  D   88      -18.442 -40.231  99.470   1.00 26.34          C
ATOM   5578   CE1  PHE  D   88      -16.354 -41.984 100.003   1.00 31.19          C
ATOM   5579   CE2  PHE  D   88      -18.664 -41.389 100.198   1.00 31.81          C
ATOM   5580   CZ   PHE  D   88      -17.614 -42.268 100.470   1.00 27.93          C
ATOM   5581   N    CYS  D   89      -14.350 -36.745  97.704   1.00 26.11          N
ATOM   5582   CA   CYS  D   89      -13.809 -36.023  96.567   1.00 24.60          C
ATOM   5583   C    CYS  D   89      -13.561 -36.988  95.406   1.00 25.26          C
ATOM   5584   O    CYS  D   89      -13.464 -38.206  95.575   1.00 23.50          O
ATOM   5585   CB   CYS  D   89      -12.537 -35.249  96.941   1.00 23.65          C
ATOM   5586   SG   CYS  D   89      -11.143 -36.170  97.584   1.00 38.72          S
ATOM   5587   N    GLN  D   90      -13.489 -36.424  94.208   1.00 21.35          N
ATOM   5588   CA   GLN  D   90      -13.364 -37.237  93.006   1.00 24.00          C
ATOM   5589   C    GLN  D   90      -12.697 -36.404  91.921   1.00 24.00          C
ATOM   5590   O    GLN  D   90      -13.073 -35.249  91.715   1.00 23.20          O
ATOM   5591   CB   GLN  D   90      -14.732 -37.733  92.536   1.00 21.84          C
ATOM   5592   CG   GLN  D   90      -14.703 -38.606  91.307   1.00 21.39          C
ATOM   5593   CD   GLN  D   90      -15.439 -37.976  90.144   1.00 26.00          C
ATOM   5594   OE1  GLN  D   90      -16.481 -37.331  90.333   1.00 29.02          O
ATOM   5595   NE2  GLN  D   90      -14.915 -38.158  88.933   1.00 21.45          N
ATOM   5596   N    GLN  D   91      -11.714 -36.984  91.236   1.00 22.32          N
ATOM   5597   CA   GLN  D   91      -11.051 -36.316  90.130   1.00 24.41          C
ATOM   5598   C    GLN  D   91      -11.459 -36.980  88.818   1.00 26.03          C
ATOM   5599   O    GLN  D   91      -11.628 -38.205  88.750   1.00 23.79          O
ATOM   5600   CB   GLN  D   91       -9.520 -36.329  90.308   1.00 21.11          C
ATOM   5601   CG   GLN  D   91       -8.866 -37.697  90.214   1.00 21.28          C
ATOM   5602   CD   GLN  D   91       -8.585 -38.091  88.785   1.00 26.27          C
ATOM   5603   OE1  GLN  D   91       -8.432 -37.231  87.917   1.00 28.92          O
ATOM   5604   NE2  GLN  D   91       -8.567 -39.384  88.514   1.00 26.42          N
```

| ATOM | 5605 | N | TYR D | 92 | -11.588 | -36.162 | 87.776 | 1.00 | 22.58 | N |
|------|------|------|-------|-----|---------|---------|--------|------|-------|-----|
| ATOM | 5606 | CA | TYR D | 92 | -11.946 | -36.594 | 86.432 | 1.00 | 26.01 | C |
| ATOM | 5607 | C | TYR D | 92 | -11.013 | -35.927 | 85.432 | 1.00 | 27.20 | C |
| ATOM | 5608 | O | TYR D | 92 | -11.409 | -35.517 | 84.334 | 1.00 | 25.89 | O |
| ATOM | 5609 | CB | TYR D | 92 | -13.413 | -36.300 | 86.093 | 1.00 | 22.29 | C |
| ATOM | 5610 | CG | TYR D | 92 | -13.889 | -34.864 | 86.367 | 1.00 | 26.32 | C |
| ATOM | 5611 | CD1 | TYR D | 92 | -14.320 | -34.475 | 87.646 | 1.00 | 21.74 | C |
| ATOM | 5612 | CD2 | TYR D | 92 | -13.903 | -33.893 | 85.344 | 1.00 | 26.96 | C |
| ATOM | 5613 | CE1 | TYR D | 92 | -14.755 | -33.186 | 87.894 | 1.00 | 22.81 | C |
| ATOM | 5614 | CE2 | TYR D | 92 | -14.347 | -32.585 | 85.587 | 1.00 | 24.44 | C |
| ATOM | 5615 | CZ | TYR D | 92 | -14.764 | -32.247 | 86.867 | 1.00 | 27.75 | C |
| ATOM | 5616 | OH | TYR D | 92 | -15.189 | -30.972 | 87.141 | 1.00 | 29.35 | O |
| ATOM | 5617 | N | GLU D | 93 | -9.755 | -35.782 | 85.826 | 1.00 | 27.70 | N |
| ATOM | 5618 | CA | GLU D | 93 | -8.729 | -35.263 | 84.936 | 1.00 | 29.92 | C |
| ATOM | 5619 | C | GLU D | 93 | -8.055 | -36.384 | 84.141 | 1.00 | 31.61 | C |
| ATOM | 5620 | O | GLU D | 93 | -7.883 | -36.278 | 82.926 | 1.00 | 33.23 | O |
| ATOM | 5621 | CB | GLU D | 93 | -7.687 | -34.482 | 85.744 | 1.00 | 25.29 | C |
| ATOM | 5622 | CG | GLU D | 93 | -6.386 | -34.312 | 84.993 | 1.00 | 28.20 | C |
| ATOM | 5623 | CD | GLU D | 93 | -5.369 | -33.447 | 85.700 | 1.00 | 32.11 | C |
| ATOM | 5624 | OE1 | GLU D | 93 | -5.735 | -32.389 | 86.285 | 1.00 | 26.85 | O |
| ATOM | 5625 | OE2 | GLU D | 93 | -4.183 | -33.828 | 85.637 | 1.00 | 36.40 | O1- |
| ATOM | 5626 | N | SER D | 94 | -7.694 | -37.471 | 84.810 | 1.00 | 28.99 | N |
| ATOM | 5627 | CA | SER D | 94 | -6.939 | -38.555 | 84.213 | 1.00 | 28.53 | C |
| ATOM | 5628 | C | SER D | 94 | -7.651 | -39.859 | 84.514 | 1.00 | 28.50 | C |
| ATOM | 5629 | O | SER D | 94 | -8.040 | -40.110 | 85.661 | 1.00 | 26.38 | O |
| ATOM | 5630 | CB | SER D | 94 | -5.499 | -38.586 | 84.754 | 1.00 | 27.94 | C |
| ATOM | 5631 | OG | SER D | 94 | -4.912 | -39.865 | 84.570 | 1.00 | 33.48 | O |
| ATOM | 5632 | N | SER D | 95 | -7.844 | -40.669 | 83.487 | 1.00 | 25.22 | N |
| ATOM | 5633 | CA | SER D | 95 | -8.488 | -41.943 | 83.683 | 1.00 | 29.13 | C |
| ATOM | 5634 | C | SER D | 95 | -7.512 | -42.941 | 84.345 | 1.00 | 28.02 | C |
| ATOM | 5635 | O | SER D | 95 | -6.324 | -42.923 | 84.083 | 1.00 | 28.38 | O |
| ATOM | 5636 | CB | SER D | 95 | -9.012 | -42.479 | 82.363 | 1.00 | 30.42 | C |
| ATOM | 5637 | OG | SER D | 95 | -9.598 | -43.754 | 82.570 | 1.00 | 42.60 | O |
| ATOM | 5638 | N | PRO D | 96 | -8.018 | -43.796 | 85.228 | 1.00 | 25.83 | N |
| ATOM | 5639 | CA | PRO D | 96 | -9.421 | -43.894 | 85.652 | 1.00 | 27.86 | C |
| ATOM | 5640 | C | PRO D | 96 | -9.883 | -42.764 | 86.572 | 1.00 | 29.69 | C |
| ATOM | 5641 | O | PRO D | 96 | -9.078 | -42.248 | 87.363 | 1.00 | 29.09 | O |
| ATOM | 5642 | CB | PRO D | 96 | -9.454 | -45.229 | 86.397 | 1.00 | 32.44 | C |
| ATOM | 5643 | CG | PRO D | 96 | -8.081 | -45.372 | 86.953 | 1.00 | 29.69 | C |
| ATOM | 5644 | CD | PRO D | 96 | -7.169 | -44.788 | 85.901 | 1.00 | 25.85 | C |
| ATOM | 5645 | N | TRP D | 97 | -11.153 | -42.369 | 86.470 | 1.00 | 27.79 | N |
| ATOM | 5646 | CA | TRP D | 97 | -11.727 | -41.539 | 87.513 | 1.00 | 23.51 | C |
| ATOM | 5647 | C | TRP D | 97 | -11.541 | -42.239 | 88.855 | 1.00 | 27.99 | C |
| ATOM | 5648 | O | TRP D | 97 | -11.697 | -43.459 | 88.964 | 1.00 | 26.14 | O |
| ATOM | 5649 | CB | TRP D | 97 | -13.205 | -41.292 | 87.265 | 1.00 | 26.18 | C |
| ATOM | 5650 | CG | TRP D | 97 | -13.628 | -40.489 | 86.072 | 1.00 | 26.55 | C |
| ATOM | 5651 | CD1 | TRP D | 97 | -14.907 | -40.229 | 85.726 | 1.00 | 23.15 | C |
| ATOM | 5652 | CD2 | TRP D | 97 | -12.804 | -39.864 | 85.056 | 1.00 | 32.73 | C |
| ATOM | 5653 | NE1 | TRP D | 97 | -14.953 | -39.474 | 84.583 | 1.00 | 28.40 | N |
| ATOM | 5654 | CE2 | TRP D | 97 | -13.683 | -39.228 | 84.151 | 1.00 | 27.44 | C |
| ATOM | 5655 | CE3 | TRP D | 97 | -11.419 | -39.760 | 84.835 | 1.00 | 25.48 | C |
| ATOM | 5656 | CZ2 | TRP D | 97 | -13.232 | -38.510 | 83.046 | 1.00 | 28.49 | C |
| ATOM | 5657 | CZ3 | TRP D | 97 | -10.975 | -39.045 | 83.734 | 1.00 | 23.95 | C |
| ATOM | 5658 | CH2 | TRP D | 97 | -11.876 | -38.438 | 82.849 | 1.00 | 28.01 | C |
| ATOM | 5659 | N | THR D | 98 | -11.174 | -41.469 | 89.877 | 1.00 | 28.12 | N |
| ATOM | 5660 | CA | THR D | 98 | -10.911 | -42.018 | 91.196 | 1.00 | 28.04 | C |
| ATOM | 5661 | C | THR D | 98 | -11.606 | -41.178 | 92.255 | 1.00 | 27.40 | C |
| ATOM | 5662 | O | THR D | 98 | -11.797 | -39.972 | 92.089 | 1.00 | 24.69 | O |
| ATOM | 5663 | CB | THR D | 98 | -9.390 | -42.067 | 91.493 | 1.00 | 30.50 | C |
| ATOM | 5664 | OG1 | THR D | 98 | -8.813 | -40.781 | 91.230 | 1.00 | 29.88 | O |
| ATOM | 5665 | CG2 | THR D | 98 | -8.687 | -43.122 | 90.648 | 1.00 | 24.32 | C |
| ATOM | 5666 | N | PHE D | 99 | -11.928 | -41.821 | 93.372 | 1.00 | 28.87 | N |
| ATOM | 5667 | CA | PHE D | 99 | -12.603 | -41.187 | 94.492 | 1.00 | 28.08 | C |
| ATOM | 5668 | C | PHE D | 99 | -11.686 | -41.223 | 95.704 | 1.00 | 31.16 | C |
| ATOM | 5669 | O | PHE D | 99 | -10.854 | -42.128 | 95.835 | 1.00 | 28.56 | O |
| ATOM | 5670 | CB | PHE D | 99 | -13.931 | -41.891 | 94.859 | 1.00 | 22.78 | C |
| ATOM | 5671 | CG | PHE D | 99 | -15.028 | -41.690 | 93.863 | 1.00 | 25.65 | C |
| ATOM | 5672 | CD1 | PHE D | 99 | -15.156 | -42.534 | 92.778 | 1.00 | 25.60 | C |
| ATOM | 5673 | CD2 | PHE D | 99 | -15.945 | -40.658 | 94.015 | 1.00 | 25.03 | C |
| ATOM | 5674 | CE1 | PHE D | 99 | -16.171 | -42.362 | 91.864 | 1.00 | 23.95 | C |
| ATOM | 5675 | CE2 | PHE D | 99 | -16.957 | -40.474 | 93.100 | 1.00 | 25.16 | C |

```
ATOM   5676  CZ   PHE D  99     -17.066 -41.336  92.011  1.00 23.49           C
ATOM   5677  N    GLY D 100     -11.852 -40.218  96.597  1.00 26.03           N
ATOM   5678  CA   GLY D 100     -11.280 -40.302  97.918  1.00 25.35           C
ATOM   5679  C    GLY D 100     -12.110 -41.227  98.769  1.00 29.26           C
ATOM   5680  O    GLY D 100     -13.227 -41.602  98.411  1.00 31.89           O
ATOM   5681  N    GLN D 101     -11.553 -41.623  99.901  1.00 26.91           N
ATOM   5682  CA   GLN D 101     -12.213 -42.607 100.740  1.00 29.14           C
ATOM   5683  C    GLN D 101     -13.218 -42.004 101.718  1.00 33.18           C
ATOM   5684  O    GLN D 101     -13.871 -42.757 102.446  1.00 31.79           O
ATOM   5685  CB   GLN D 101     -11.163 -43.425 101.480  1.00 37.07           C
ATOM   5686  CG   GLN D 101     -10.254 -44.203 100.497  1.00 50.48           C
ATOM   5687  CD   GLN D 101     -10.653 -45.667 100.316  1.00 57.12           C
ATOM   5688  OE1  GLN D 101     -10.139 -46.549 101.022  1.00 66.11           O
ATOM   5689  NE2  GLN D 101     -11.564 -45.935  99.371  1.00 48.92           N
ATOM   5690  N    GLY D 102     -13.381 -40.691 101.740  1.00 27.41           N
ATOM   5691  CA   GLY D 102     -14.489 -40.090 102.452  1.00 24.59           C
ATOM   5692  C    GLY D 102     -14.084 -39.542 103.806  1.00 24.13           C
ATOM   5693  O    GLY D 102     -13.158 -40.032 104.454  1.00 30.96           O
ATOM   5694  N    THR D 103     -14.767 -38.478 104.226  1.00 26.72           N
ATOM   5695  CA   THR D 103     -14.681 -37.929 105.577  1.00 25.89           C
ATOM   5696  C    THR D 103     -16.080 -37.902 106.164  1.00 25.39           C
ATOM   5697  O    THR D 103     -16.997 -37.334 105.560  1.00 19.85           O
ATOM   5698  CB   THR D 103     -14.116 -36.500 105.626  1.00 28.14           C
ATOM   5699  OG1  THR D 103     -12.740 -36.480 105.221  1.00 28.24           O
ATOM   5700  CG2  THR D 103     -14.269 -35.913 107.044  1.00 26.41           C
ATOM   5701  N    LYS D 104     -16.230 -38.501 107.344  1.00 27.38           N
ATOM   5702  CA   LYS D 104     -17.489 -38.491 108.073  1.00 27.31           C
ATOM   5703  C    LYS D 104     -17.515 -37.253 108.966  1.00 28.48           C
ATOM   5704  O    LYS D 104     -16.604 -37.032 109.773  1.00 28.70           O
ATOM   5705  CB   LYS D 104     -17.661 -39.787 108.875  1.00 25.02           C
ATOM   5706  CG   LYS D 104     -19.009 -39.923 109.600  1.00 29.05           C
ATOM   5707  CD   LYS D 104     -19.050 -41.169 110.488  1.00 32.39           C
ATOM   5708  CE   LYS D 104     -20.386 -41.319 111.265  1.00 49.50           C
ATOM   5709  NZ   LYS D 104     -20.557 -42.613 112.082  1.00 40.68           N1+
ATOM   5710  N    VAL D 105     -18.520 -36.416 108.767  1.00 26.83           N
ATOM   5711  CA   VAL D 105     -18.791 -35.267 109.621  1.00 27.60           C
ATOM   5712  C    VAL D 105     -19.905 -35.666 110.574  1.00 25.51           C
ATOM   5713  O    VAL D 105     -21.054 -35.808 110.157  1.00 24.45           O
ATOM   5714  CB   VAL D 105     -19.176 -34.041 108.785  1.00 27.65           C
ATOM   5715  CG1  VAL D 105     -19.528 -32.864 109.682  1.00 29.48           C
ATOM   5716  CG2  VAL D 105     -18.026 -33.694 107.825  1.00 22.38           C
ATOM   5717  N    GLU D 106     -19.579 -35.866 111.851  1.00 31.50           N
ATOM   5718  CA   GLU D 106     -20.568 -36.309 112.826  1.00 28.01           C
ATOM   5719  C    GLU D 106     -20.867 -35.198 113.826  1.00 31.26           C
ATOM   5720  O    GLU D 106     -20.043 -34.311 114.068  1.00 29.59           O
ATOM   5721  CB   GLU D 106     -20.137 -37.601 113.551  1.00 29.60           C
ATOM   5722  CG   GLU D 106     -18.949 -37.531 114.521  1.00 33.75           C
ATOM   5723  CD   GLU D 106     -19.264 -38.095 115.928  1.00 34.42           C
ATOM   5724  OE1  GLU D 106     -19.053 -37.378 116.921  1.00 40.25           O
ATOM   5725  OE2  GLU D 106     -19.688 -39.257 116.061  1.00 33.48           O1-
ATOM   5726  N    ILE D 107     -22.075 -35.258 114.387  1.00 29.99           N
ATOM   5727  CA   ILE D 107     -22.541 -34.269 115.347  1.00 27.29           C
ATOM   5728  C    ILE D 107     -21.815 -34.467 116.668  1.00 32.55           C
ATOM   5729  O    ILE D 107     -21.916 -35.529 117.291  1.00 29.14           O
ATOM   5730  CB   ILE D 107     -24.048 -34.396 115.581  1.00 29.36           C
ATOM   5731  CG1  ILE D 107     -24.859 -33.985 114.363  1.00 29.05           C
ATOM   5732  CG2  ILE D 107     -24.428 -33.602 116.816  1.00 30.42           C
ATOM   5733  CD1  ILE D 107     -26.308 -34.346 114.489  1.00 29.83           C
ATOM   5734  N    LYS D 108     -21.120 -33.428 117.121  1.00 35.45           N
ATOM   5735  CA   LYS D 108     -20.531 -33.410 118.452  1.00 32.78           C
ATOM   5736  C    LYS D 108     -21.581 -32.996 119.483  1.00 36.07           C
ATOM   5737  O    LYS D 108     -22.303 -32.011 119.282  1.00 36.02           O
ATOM   5738  CB   LYS D 108     -19.355 -32.445 118.484  1.00 34.28           C
ATOM   5739  CG   LYS D 108     -18.623 -32.454 119.791  1.00 36.31           C
ATOM   5740  CD   LYS D 108     -17.705 -31.273 119.907  1.00 36.98           C
ATOM   5741  CE   LYS D 108     -16.504 -31.728 120.659  1.00 44.81           C
ATOM   5742  NZ   LYS D 108     -16.855 -33.094 121.165  1.00 40.45           N1+
ATOM   5743  N    ARG D 109     -21.682 -33.760 120.572  1.00 31.47           N
ATOM   5744  CA   ARG D 109     -22.636 -33.469 121.642  1.00 32.33           C
ATOM   5745  C    ARG D 109     -22.004 -33.853 122.962  1.00 30.94           C
ATOM   5746  O    ARG D 109     -20.848 -34.273 123.008  1.00 35.21           O
```

```
ATOM   5747  CB  ARG D 109    -23.957 -34.203 121.464  1.00 26.67           C
ATOM   5748  CG  ARG D 109    -23.826 -35.711 121.380  1.00 32.22           C
ATOM   5749  CD  ARG D 109    -25.081 -36.397 121.890  1.00 28.47           C
ATOM   5750  NE  ARG D 109    -25.123 -36.331 123.335  1.00 32.52           N
ATOM   5751  CZ  ARG D 109    -26.235 -36.307 124.056  1.00 28.79           C
ATOM   5752  NH1 ARG D 109    -26.159 -36.242 125.377  1.00 28.77           N1+
ATOM   5753  NH2 ARG D 109    -27.406 -36.312 123.460  1.00 25.19           N
ATOM   5754  N   THR D 110    -22.765 -33.713 124.040  1.00 27.95           N
ATOM   5755  CA  THR D 110    -22.224 -34.032 125.356  1.00 33.38           C
ATOM   5756  C   THR D 110    -22.147 -35.538 125.572  1.00 32.27           C
ATOM   5757  O   THR D 110    -22.956 -36.306 125.044  1.00 32.94           O
ATOM   5758  CB  THR D 110    -23.068 -33.429 126.468  1.00 32.63           C
ATOM   5759  OG1 THR D 110    -24.415 -33.917 126.356  1.00 28.33           O
ATOM   5760  CG2 THR D 110    -23.037 -31.936 126.370  1.00 31.15           C
ATOM   5761  N   VAL D 111    -21.145 -35.946 126.354  1.00 29.24           N
ATOM   5762  CA  VAL D 111    -20.999 -37.339 126.750  1.00 29.80           C
ATOM   5763  C   VAL D 111    -22.298 -37.861 127.374  1.00 31.10           C
ATOM   5764  O   VAL D 111    -22.978 -37.165 128.137  1.00 36.10           O
ATOM   5765  CB  VAL D 111    -19.794 -37.464 127.699  1.00 29.10           C
ATOM   5766  CG1 VAL D 111    -19.627 -38.899 128.181  1.00 32.56           C
ATOM   5767  CG2 VAL D 111    -18.522 -36.988 126.978  1.00 24.86           C
ATOM   5768  N   ALA D 112    -22.665 -39.086 127.004  1.00 30.83           N
ATOM   5769  CA  ALA D 112    -23.886 -39.735 127.460  1.00 27.58           C
ATOM   5770  C   ALA D 112    -23.597 -41.211 127.684  1.00 30.58           C
ATOM   5771  O   ALA D 112    -23.169 -41.899 126.759  1.00 35.39           O
ATOM   5772  CB  ALA D 112    -25.003 -39.561 126.435  1.00 26.61           C
ATOM   5773  N   ALA D 113    -23.828 -41.703 128.896  1.00 32.52           N
ATOM   5774  CA  ALA D 113    -23.548 -43.102 129.185  1.00 31.32           C
ATOM   5775  C   ALA D 113    -24.588 -44.017 128.544  1.00 31.91           C
ATOM   5776  O   ALA D 113    -25.757 -43.648 128.414  1.00 30.66           O
ATOM   5777  CB  ALA D 113    -23.530 -43.351 130.685  1.00 26.64           C
ATOM   5778  N   PRO D 114    -24.193 -45.222 128.150  1.00 29.42           N
ATOM   5779  CA  PRO D 114    -25.182 -46.176 127.649  1.00 29.26           C
ATOM   5780  C   PRO D 114    -26.021 -46.730 128.781  1.00 29.73           C
ATOM   5781  O   PRO D 114    -25.589 -46.804 129.925  1.00 28.84           O
ATOM   5782  CB  PRO D 114    -24.334 -47.277 127.005  1.00 27.37           C
ATOM   5783  CG  PRO D 114    -23.042 -47.236 127.766  1.00 30.02           C
ATOM   5784  CD  PRO D 114    -22.823 -45.773 128.131  1.00 29.01           C
ATOM   5785  N   SER D 115    -27.255 -47.068 128.454  1.00 28.42           N
ATOM   5786  CA  SER D 115    -28.020 -47.994 129.254  1.00 24.77           C
ATOM   5787  C   SER D 115    -27.788 -49.394 128.706  1.00 27.46           C
ATOM   5788  O   SER D 115    -27.741 -49.606 127.489  1.00 25.81           O
ATOM   5789  CB  SER D 115    -29.502 -47.638 129.221  1.00 27.83           C
ATOM   5790  OG  SER D 115    -29.644 -46.253 129.452  1.00 30.47           O
ATOM   5791  N   VAL D 116    -27.612 -50.342 129.612  1.00 26.91           N
ATOM   5792  CA  VAL D 116    -27.156 -51.679 129.275  1.00 27.26           C
ATOM   5793  C   VAL D 116    -28.258 -52.657 129.636  1.00 26.73           C
ATOM   5794  O   VAL D 116    -28.800 -52.605 130.742  1.00 33.45           O
ATOM   5795  CB  VAL D 116    -25.850 -52.032 130.010  1.00 29.29           C
ATOM   5796  CG1 VAL D 116    -25.339 -53.387 129.535  1.00 30.85           C
ATOM   5797  CG2 VAL D 116    -24.814 -50.930 129.803  1.00 24.64           C
ATOM   5798  N   PHE D 117    -28.591 -53.535 128.703  1.00 25.90           N
ATOM   5799  CA  PHE D 117    -29.585 -54.573 128.907  1.00 25.96           C
ATOM   5800  C   PHE D 117    -29.037 -55.838 128.293  1.00 27.24           C
ATOM   5801  O   PHE D 117    -28.399 -55.788 127.240  1.00 29.01           O
ATOM   5802  CB  PHE D 117    -30.930 -54.282 128.244  1.00 26.04           C
ATOM   5803  CG  PHE D 117    -31.510 -52.943 128.560  1.00 29.45           C
ATOM   5804  CD1 PHE D 117    -31.119 -51.810 127.851  1.00 28.96           C
ATOM   5805  CD2 PHE D 117    -32.523 -52.826 129.497  1.00 27.92           C
ATOM   5806  CE1 PHE D 117    -31.698 -50.590 128.119  1.00 31.67           C
ATOM   5807  CE2 PHE D 117    -33.108 -51.607 129.767  1.00 28.75           C
ATOM   5808  CZ  PHE D 117    -32.705 -50.490 129.086  1.00 31.59           C
ATOM   5809  N   ILE D 118    -29.288 -56.966 128.938  1.00 28.76           N
ATOM   5810  CA  ILE D 118    -28.835 -58.252 128.429  1.00 30.93           C
ATOM   5811  C   ILE D 118    -30.053 -59.149 128.291  1.00 27.51           C
ATOM   5812  O   ILE D 118    -30.949 -59.115 129.134  1.00 28.60           O
ATOM   5813  CB  ILE D 118    -27.764 -58.872 129.348  1.00 29.79           C
ATOM   5814  CG1 ILE D 118    -27.230 -60.167 128.757  1.00 33.20           C
ATOM   5815  CG2 ILE D 118    -28.321 -59.122 130.738  1.00 28.76           C
ATOM   5816  CD1 ILE D 118    -26.243 -60.873 129.679  1.00 35.26           C
ATOM   5817  N   PHE D 119    -30.108 -59.909 127.204  1.00 28.10           N
```

```
ATOM   5818  CA  PHE D 119     -31.241 -60.753 126.853  1.00 27.79          C
ATOM   5819  C   PHE D 119     -30.812 -62.209 126.778  1.00 32.91          C
ATOM   5820  O   PHE D 119     -29.869 -62.544 126.044  1.00 31.43          O
ATOM   5821  CB  PHE D 119     -31.858 -60.343 125.507  1.00 29.66          C
ATOM   5822  CG  PHE D 119     -32.413 -58.970 125.513  1.00 31.25          C
ATOM   5823  CD1 PHE D 119     -33.683 -58.729 126.018  1.00 26.70          C
ATOM   5824  CD2 PHE D 119     -31.659 -57.909 125.051  1.00 27.96          C
ATOM   5825  CE1 PHE D 119     -34.198 -57.450 126.055  1.00 28.84          C
ATOM   5826  CE2 PHE D 119     -32.165 -56.635 125.086  1.00 30.39          C
ATOM   5827  CZ  PHE D 119     -33.445 -56.399 125.584  1.00 27.28          C
ATOM   5828  N   PRO D 120     -31.482 -63.097 127.497  1.00 35.56          N
ATOM   5829  CA  PRO D 120     -31.187 -64.530 127.394  1.00 36.54          C
ATOM   5830  C   PRO D 120     -31.661 -65.088 126.065  1.00 34.90          C
ATOM   5831  O   PRO D 120     -32.495 -64.470 125.387  1.00 33.38          O
ATOM   5832  CB  PRO D 120     -31.987 -65.138 128.557  1.00 36.73          C
ATOM   5833  CG  PRO D 120     -32.403 -63.956 129.408  1.00 42.07          C
ATOM   5834  CD  PRO D 120     -32.546 -62.814 128.466  1.00 32.56          C
ATOM   5835  N   PRO D 121     -31.155 -66.248 125.655  1.00 33.49          N
ATOM   5836  CA  PRO D 121     -31.711 -66.900 124.463  1.00 35.36          C
ATOM   5837  C   PRO D 121     -33.137 -67.359 124.718  1.00 32.32          C
ATOM   5838  O   PRO D 121     -33.510 -67.738 125.829  1.00 33.72          O
ATOM   5839  CB  PRO D 121     -30.772 -68.091 124.225  1.00 34.18          C
ATOM   5840  CG  PRO D 121     -30.187 -68.357 125.588  1.00 34.16          C
ATOM   5841  CD  PRO D 121     -30.062 -67.026 126.262  1.00 28.95          C
ATOM   5842  N   SER D 122     -33.949 -67.282 123.672  1.00 37.30          N
ATOM   5843  CA  SER D 122     -35.326 -67.733 123.761  1.00 35.72          C
ATOM   5844  C   SER D 122     -35.386 -69.261 123.796  1.00 40.60          C
ATOM   5845  O   SER D 122     -34.510 -69.961 123.264  1.00 37.13          O
ATOM   5846  CB  SER D 122     -36.141 -67.176 122.589  1.00 30.63          C
ATOM   5847  OG  SER D 122     -35.821 -67.837 121.384  1.00 34.59          O
ATOM   5848  N   ASP D 123     -36.413 -69.776 124.481  1.00 44.14          N
ATOM   5849  CA  ASP D 123     -36.631 -71.221 124.525  1.00 47.46          C
ATOM   5850  C   ASP D 123     -36.913 -71.793 123.142  1.00 46.92          C
ATOM   5851  O   ASP D 123     -36.606 -72.964 122.884  1.00 49.21          O
ATOM   5852  CB  ASP D 123     -37.776 -71.541 125.478  1.00 54.79          C
ATOM   5853  CG  ASP D 123     -37.352 -71.471 126.921  1.00 61.77          C
ATOM   5854  OD1 ASP D 123     -36.195 -71.849 127.207  1.00 56.99          O
ATOM   5855  OD2 ASP D 123     -38.161 -71.015 127.763  1.00 71.23          O1-
ATOM   5856  N   GLU D 124     -37.480 -70.978 122.241  1.00 45.60          N
ATOM   5857  CA  GLU D 124     -37.753 -71.411 120.869  1.00 49.64          C
ATOM   5858  C   GLU D 124     -36.463 -71.644 120.084  1.00 50.62          C
ATOM   5859  O   GLU D 124     -36.383 -72.565 119.259  1.00 49.45          O
ATOM   5860  CB  GLU D 124     -38.613 -70.365 120.168  1.00 45.37          C
ATOM   5861  CG  GLU D 124     -39.053 -70.740 118.759  1.00 60.55          C
ATOM   5862  CD  GLU D 124     -39.635 -69.542 117.988  1.00 74.03          C
ATOM   5863  OE1 GLU D 124     -39.948 -68.502 118.626  1.00 67.91          O
ATOM   5864  OE2 GLU D 124     -39.760 -69.633 116.740  1.00 80.45          O1-
ATOM   5865  N   GLN D 125     -35.452 -70.799 120.309  1.00 44.68          N
ATOM   5866  CA  GLN D 125     -34.161 -70.979 119.660  1.00 44.38          C
ATOM   5867  C   GLN D 125     -33.365 -72.110 120.296  1.00 44.17          C
ATOM   5868  O   GLN D 125     -32.617 -72.803 119.596  1.00 44.30          O
ATOM   5869  CB  GLN D 125     -33.353 -69.677 119.726  1.00 38.62          C
ATOM   5870  CG  GLN D 125     -32.017 -69.721 118.987  1.00 34.83          C
ATOM   5871  CD  GLN D 125     -31.067 -68.656 119.471  1.00 36.06          C
ATOM   5872  OE1 GLN D 125     -31.232 -68.116 120.570  1.00 37.90          O
ATOM   5873  NE2 GLN D 125     -30.080 -68.328 118.652  1.00 40.51          N
ATOM   5874  N   LEU D 126     -33.515 -72.316 121.606  1.00 39.87          N
ATOM   5875  CA  LEU D 126     -32.798 -73.405 122.254  1.00 43.24          C
ATOM   5876  C   LEU D 126     -33.200 -74.756 121.683  1.00 49.92          C
ATOM   5877  O   LEU D 126     -32.343 -75.623 121.467  1.00 50.55          O
ATOM   5878  CB  LEU D 126     -33.035 -73.372 123.755  1.00 37.51          C
ATOM   5879  CG  LEU D 126     -32.229 -72.275 124.411  1.00 38.73          C
ATOM   5880  CD1 LEU D 126     -32.542 -72.240 125.881  1.00 34.97          C
ATOM   5881  CD2 LEU D 126     -30.723 -72.461 124.145  1.00 38.73          C
ATOM   5882  N   LYS D 127     -34.486 -74.933 121.365  1.00 50.02          N
ATOM   5883  CA  LYS D 127     -34.931 -76.193 120.777  1.00 50.42          C
ATOM   5884  C   LYS D 127     -34.041 -76.636 119.621  1.00 50.52          C
ATOM   5885  O   LYS D 127     -33.827 -77.838 119.434  1.00 61.34          O
ATOM   5886  CB  LYS D 127     -36.378 -76.066 120.287  1.00 54.82          C
ATOM   5887  CG  LYS D 127     -37.364 -75.491 121.309  1.00 56.44          C
ATOM   5888  CD  LYS D 127     -38.834 -75.811 120.959  1.00 64.32          C
```

| ATOM | 5889 | CE | LYS D 127 | -39.297 | -75.170 | 119.628 | 1.00 | 72.71 | C |
| ATOM | 5890 | NZ | LYS D 127 | -39.699 | -76.161 | 118.571 | 1.00 | 64.97 | N1+ |
| ATOM | 5891 | N | SER D 128 | -33.470 | -75.687 | 118.880 | 1.00 | 50.62 | N |
| ATOM | 5892 | CA | SER D 128 | -32.691 | -75.949 | 117.674 | 1.00 | 48.62 | C |
| ATOM | 5893 | C | SER D 128 | -31.181 | -76.031 | 117.919 | 1.00 | 52.68 | C |
| ATOM | 5894 | O | SER D 128 | -30.403 | -76.019 | 116.957 | 1.00 | 53.36 | O |
| ATOM | 5895 | CB | SER D 128 | -32.998 | -74.877 | 116.626 | 1.00 | 51.45 | C |
| ATOM | 5896 | OG | SER D 128 | -32.539 | -73.605 | 117.065 | 1.00 | 54.76 | O |
| ATOM | 5897 | N | GLY D 129 | -30.742 | -76.074 | 119.169 | 1.00 | 49.90 | N |
| ATOM | 5898 | CA | GLY D 129 | -29.355 | -76.368 | 119.452 | 1.00 | 48.29 | C |
| ATOM | 5899 | C | GLY D 129 | -28.416 | -75.187 | 119.533 | 1.00 | 52.26 | C |
| ATOM | 5900 | O | GLY D 129 | -27.243 | -75.382 | 119.882 | 1.00 | 50.58 | O |
| ATOM | 5901 | N | THR D 130 | -28.886 | -73.969 | 119.259 | 1.00 | 49.43 | N |
| ATOM | 5902 | CA | THR D 130 | -28.043 | -72.784 | 119.302 | 1.00 | 44.36 | C |
| ATOM | 5903 | C | THR D 130 | -28.614 | -71.774 | 120.285 | 1.00 | 44.57 | C |
| ATOM | 5904 | O | THR D 130 | -29.830 | -71.596 | 120.369 | 1.00 | 45.91 | O |
| ATOM | 5905 | CB | THR D 130 | -27.942 | -72.142 | 117.926 | 1.00 | 49.85 | C |
| ATOM | 5906 | OG1 | THR D 130 | -27.337 | -73.073 | 117.030 | 1.00 | 49.94 | O |
| ATOM | 5907 | CG2 | THR D 130 | -27.087 | -70.871 | 117.975 | 1.00 | 47.05 | C |
| ATOM | 5908 | N | ALA D 131 | -27.729 | -71.098 | 121.008 | 1.00 | 40.05 | N |
| ATOM | 5909 | CA | ALA D 131 | -28.104 | -70.036 | 121.924 | 1.00 | 38.97 | C |
| ATOM | 5910 | C | ALA D 131 | -27.432 | -68.727 | 121.512 | 1.00 | 37.81 | C |
| ATOM | 5911 | O | ALA D 131 | -26.209 | -68.683 | 121.329 | 1.00 | 38.34 | O |
| ATOM | 5912 | CB | ALA D 131 | -27.721 | -70.421 | 123.347 | 1.00 | 38.52 | C |
| ATOM | 5913 | N | SER D 132 | -28.224 | -67.667 | 121.359 | 1.00 | 33.37 | N |
| ATOM | 5914 | CA | SER D 132 | -27.707 | -66.314 | 121.152 | 1.00 | 30.63 | C |
| ATOM | 5915 | C | SER D 132 | -28.011 | -65.482 | 122.392 | 1.00 | 31.31 | C |
| ATOM | 5916 | O | SER D 132 | -29.166 | -65.369 | 122.800 | 1.00 | 39.28 | O |
| ATOM | 5917 | CB | SER D 132 | -28.321 | -65.659 | 119.913 | 1.00 | 31.88 | C |
| ATOM | 5918 | OG | SER D 132 | -27.968 | -66.338 | 118.727 | 1.00 | 32.64 | O |
| ATOM | 5919 | N | VAL D 133 | -26.999 | -64.942 | 123.008 | 1.00 | 32.01 | N |
| ATOM | 5920 | CA | VAL D 133 | -27.179 | -63.987 | 124.097 | 1.00 | 34.18 | C |
| ATOM | 5921 | C | VAL D 133 | -26.944 | -62.601 | 123.519 | 1.00 | 32.77 | C |
| ATOM | 5922 | O | VAL D 133 | -26.025 | -62.421 | 122.706 | 1.00 | 31.33 | O |
| ATOM | 5923 | CB | VAL D 133 | -26.213 | -64.294 | 125.261 | 1.00 | 34.59 | C |
| ATOM | 5924 | CG1 | VAL D 133 | -26.610 | -63.544 | 126.532 | 1.00 | 32.22 | C |
| ATOM | 5925 | CG2 | VAL D 133 | -26.180 | -65.792 | 125.517 | 1.00 | 30.53 | C |
| ATOM | 5926 | N | VAL D 134 | -27.781 | -61.625 | 123.893 | 1.00 | 27.21 | N |
| ATOM | 5927 | CA | VAL D 134 | -27.711 | -60.290 | 123.295 | 1.00 | 28.33 | C |
| ATOM | 5928 | C | VAL D 134 | -27.496 | -59.226 | 124.365 | 1.00 | 28.83 | C |
| ATOM | 5929 | O | VAL D 134 | -28.174 | -59.214 | 125.397 | 1.00 | 27.78 | O |
| ATOM | 5930 | CB | VAL D 134 | -28.950 | -59.955 | 122.450 | 1.00 | 27.75 | C |
| ATOM | 5931 | CG1 | VAL D 134 | -28.794 | -58.585 | 121.858 | 1.00 | 26.08 | C |
| ATOM | 5932 | CG2 | VAL D 134 | -29.096 | -60.957 | 121.319 | 1.00 | 30.78 | C |
| ATOM | 5933 | N | CYS D 135 | -26.565 | -58.313 | 124.092 | 1.00 | 27.54 | N |
| ATOM | 5934 | CA | CYS D 135 | -26.299 | -57.155 | 124.928 | 1.00 | 31.96 | C |
| ATOM | 5935 | C | CYS D 135 | -26.651 | -55.895 | 124.141 | 1.00 | 32.81 | C |
| ATOM | 5936 | O | CYS D 135 | -26.250 | -55.760 | 122.979 | 1.00 | 27.82 | O |
| ATOM | 5937 | CB | CYS D 135 | -24.827 | -57.154 | 125.365 | 1.00 | 32.75 | C |
| ATOM | 5938 | SG | CYS D 135 | -24.350 | -56.013 | 126.697 | 1.00 | 40.27 | S |
| ATOM | 5939 | N | LEU D 136 | -27.422 | -54.996 | 124.762 | 1.00 | 26.44 | N |
| ATOM | 5940 | CA | LEU D 136 | -27.826 | -53.726 | 124.161 | 1.00 | 24.84 | C |
| ATOM | 5941 | C | LEU D 136 | -27.168 | -52.559 | 124.898 | 1.00 | 28.43 | C |
| ATOM | 5942 | O | LEU D 136 | -27.375 | -52.380 | 126.107 | 1.00 | 27.53 | O |
| ATOM | 5943 | CB | LEU D 136 | -29.345 | -53.566 | 124.191 | 1.00 | 26.69 | C |
| ATOM | 5944 | CG | LEU D 136 | -29.908 | -52.191 | 123.787 | 1.00 | 30.11 | C |
| ATOM | 5945 | CD1 | LEU D 136 | -29.570 | -51.875 | 122.330 | 1.00 | 22.63 | C |
| ATOM | 5946 | CD2 | LEU D 136 | -31.439 | -52.087 | 124.042 | 1.00 | 23.90 | C |
| ATOM | 5947 | N | LEU D 137 | -26.403 | -51.746 | 124.169 | 1.00 | 27.96 | N |
| ATOM | 5948 | CA | LEU D 137 | -25.860 | -50.492 | 124.687 | 1.00 | 24.59 | C |
| ATOM | 5949 | C | LEU D 137 | -26.657 | -49.367 | 124.027 | 1.00 | 28.90 | C |
| ATOM | 5950 | O | LEU D 137 | -26.501 | -49.096 | 122.830 | 1.00 | 28.52 | O |
| ATOM | 5951 | CB | LEU D 137 | -24.364 | -50.380 | 124.419 | 1.00 | 20.03 | C |
| ATOM | 5952 | CG | LEU D 137 | -23.377 | -51.186 | 125.271 | 1.00 | 24.15 | C |
| ATOM | 5953 | CD1 | LEU D 137 | -23.602 | -52.697 | 125.252 | 1.00 | 20.47 | C |
| ATOM | 5954 | CD2 | LEU D 137 | -21.943 | -50.875 | 124.827 | 1.00 | 21.46 | C |
| ATOM | 5955 | N | ASN D 138 | -27.531 | -48.732 | 124.806 | 1.00 | 30.48 | N |
| ATOM | 5956 | CA | ASN D 138 | -28.551 | -47.836 | 124.285 | 1.00 | 28.96 | C |
| ATOM | 5957 | C | ASN D 138 | -28.215 | -46.368 | 124.560 | 1.00 | 31.63 | C |
| ATOM | 5958 | O | ASN D 138 | -27.933 | -45.994 | 125.705 | 1.00 | 33.79 | O |
| ATOM | 5959 | CB | ASN D 138 | -29.904 | -48.179 | 124.900 | 1.00 | 27.28 | C |

```
ATOM   5960  CG   ASN D 138    -31.052 -47.802 124.004  1.00 31.25           C
ATOM   5961  OD1  ASN D 138    -31.153 -48.280 122.875  1.00 36.62           O
ATOM   5962  ND2  ASN D 138    -31.880 -46.874 124.463  1.00 36.73           N
ATOM   5963  N    ASN D 139    -28.258 -45.549 123.495  1.00 33.24           N
ATOM   5964  CA   ASN D 139    -28.250 -44.074 123.524  1.00 28.78           C
ATOM   5965  C    ASN D 139    -27.036 -43.490 124.238  1.00 27.30           C
ATOM   5966  O    ASN D 139    -27.168 -42.799 125.238  1.00 35.75           O
ATOM   5967  CB   ASN D 139    -29.520 -43.529 124.171  1.00 27.41           C
ATOM   5968  CG   ASN D 139    -30.755 -43.820 123.364  1.00 34.79           C
ATOM   5969  OD1  ASN D 139    -30.693 -44.255 122.215  1.00 31.67           O
ATOM   5970  ND2  ASN D 139    -31.903 -43.606 123.985  1.00 49.31           N
ATOM   5971  N    PHE D 140    -25.853 -43.720 123.683  1.00 28.66           N
ATOM   5972  CA   PHE D 140    -24.631 -43.200 124.279  1.00 30.52           C
ATOM   5973  C    PHE D 140    -23.895 -42.295 123.301  1.00 30.45           C
ATOM   5974  O    PHE D 140    -24.127 -42.323 122.089  1.00 31.04           O
ATOM   5975  CB   PHE D 140    -23.703 -44.333 124.760  1.00 27.13           C
ATOM   5976  CG   PHE D 140    -23.324 -45.299 123.685  1.00 30.02           C
ATOM   5977  CD1  PHE D 140    -24.068 -46.450 123.482  1.00 29.03           C
ATOM   5978  CD2  PHE D 140    -22.224 -45.059 122.868  1.00 27.79           C
ATOM   5979  CE1  PHE D 140    -23.724 -47.344 122.477  1.00 28.63           C
ATOM   5980  CE2  PHE D 140    -21.869 -45.950 121.866  1.00 25.04           C
ATOM   5981  CZ   PHE D 140    -22.619 -47.090 121.666  1.00 25.93           C
ATOM   5982  N    TYR D 141    -23.033 -41.452 123.855  1.00 27.31           N
ATOM   5983  CA   TYR D 141    -22.138 -40.640 123.059  1.00 29.62           C
ATOM   5984  C    TYR D 141    -20.895 -40.416 123.882  1.00 30.14           C
ATOM   5985  O    TYR D 141    -21.002 -40.131 125.060  1.00 31.98           O
ATOM   5986  CB   TYR D 141    -22.777 -39.292 122.657  1.00 30.01           C
ATOM   5987  CG   TYR D 141    -21.868 -38.536 121.719  1.00 28.91           C
ATOM   5988  CD1  TYR D 141    -21.906 -38.764 120.356  1.00 24.75           C
ATOM   5989  CD2  TYR D 141    -20.915 -37.652 122.207  1.00 33.08           C
ATOM   5990  CE1  TYR D 141    -21.055 -38.124 119.505  1.00 27.43           C
ATOM   5991  CE2  TYR D 141    -20.049 -36.991 121.353  1.00 32.86           C
ATOM   5992  CZ   TYR D 141    -20.123 -37.233 119.999  1.00 31.49           C
ATOM   5993  OH   TYR D 141    -19.254 -36.584 119.149  1.00 29.36           O
ATOM   5994  N    PRO D 142    -19.704 -40.530 123.272  1.00 33.14           N
ATOM   5995  CA   PRO D 142    -19.424 -40.777 121.853  1.00 31.33           C
ATOM   5996  C    PRO D 142    -19.435 -42.241 121.404  1.00 31.01           C
ATOM   5997  O    PRO D 142    -19.806 -43.143 122.140  1.00 30.96           O
ATOM   5998  CB   PRO D 142    -18.028 -40.201 121.691  1.00 27.46           C
ATOM   5999  CG   PRO D 142    -17.393 -40.495 123.007  1.00 28.61           C
ATOM   6000  CD   PRO D 142    -18.464 -40.243 124.019  1.00 27.72           C
ATOM   6001  N    ARG D 143    -18.974 -42.430 120.167  1.00 35.41           N
ATOM   6002  CA   ARG D 143    -19.142 -43.686 119.441  1.00 32.43           C
ATOM   6003  C    ARG D 143    -18.410 -44.842 120.111  1.00 35.51           C
ATOM   6004  O    ARG D 143    -18.897 -45.974 120.120  1.00 39.91           O
ATOM   6005  CB   ARG D 143    -18.633 -43.477 118.018  1.00 36.16           C
ATOM   6006  CG   ARG D 143    -18.522 -44.686 117.126  1.00 44.24           C
ATOM   6007  CD   ARG D 143    -19.818 -44.835 116.397  1.00 40.17           C
ATOM   6008  NE   ARG D 143    -19.792 -45.523 115.101  1.00 38.57           N
ATOM   6009  CZ   ARG D 143    -19.299 -46.736 114.883  1.00 38.79           C
ATOM   6010  NH1  ARG D 143    -18.689 -47.407 115.852  1.00 42.03           N1+
ATOM   6011  NH2  ARG D 143    -19.395 -47.265 113.675  1.00 43.44           N
ATOM   6012  N    GLU D 144    -17.256 -44.580 120.697  1.00 31.31           N
ATOM   6013  CA   GLU D 144    -16.406 -45.662 121.160  1.00 30.73           C
ATOM   6014  C    GLU D 144    -16.975 -46.318 122.418  1.00 29.58           C
ATOM   6015  O    GLU D 144    -17.308 -45.649 123.397  1.00 32.43           O
ATOM   6016  CB   GLU D 144    -14.994 -45.126 121.391  1.00 28.89           C
ATOM   6017  CG   GLU D 144    -14.361 -44.583 120.090  1.00 45.09           C
ATOM   6018  CD   GLU D 144    -14.920 -43.198 119.638  1.00 55.20           C
ATOM   6019  OE1  GLU D 144    -15.083 -42.301 120.513  1.00 49.33           O
ATOM   6020  OE2  GLU D 144    -15.215 -43.020 118.417  1.00 49.10           O1-
ATOM   6021  N    ALA D 145    -17.065 -47.640 122.396  1.00 25.69           N
ATOM   6022  CA   ALA D 145    -17.552 -48.390 123.535  1.00 29.57           C
ATOM   6023  C    ALA D 145    -16.972 -49.792 123.452  1.00 31.44           C
ATOM   6024  O    ALA D 145    -16.702 -50.304 122.364  1.00 37.00           O
ATOM   6025  CB   ALA D 145    -19.095 -48.415 123.584  1.00 25.18           C
ATOM   6026  N    LYS D 146    -16.792 -50.416 124.607  1.00 31.28           N
ATOM   6027  CA   LYS D 146    -16.278 -51.775 124.678  1.00 35.67           C
ATOM   6028  C    LYS D 146    -17.308 -52.687 125.339  1.00 38.62           C
ATOM   6029  O    LYS D 146    -17.790 -52.390 126.436  1.00 36.49           O
ATOM   6030  CB   LYS D 146    -14.972 -51.810 125.466  1.00 33.50           C
```

```
ATOM   6031  CG   LYS D 146     -14.242 -53.136 125.440  1.00 40.82          C
ATOM   6032  CD   LYS D 146     -12.883 -53.049 126.167  1.00 52.07          C
ATOM   6033  CE   LYS D 146     -12.130 -54.385 126.127  1.00 60.61          C
ATOM   6034  NZ   LYS D 146     -10.663 -54.265 126.375  1.00 71.34          N1+
ATOM   6035  N    VAL D 147     -17.649 -53.786 124.663  1.00 35.29          N
ATOM   6036  CA   VAL D 147     -18.415 -54.883 125.242  1.00 37.00          C
ATOM   6037  C    VAL D 147     -17.443 -56.024 125.497  1.00 36.93          C
ATOM   6038  O    VAL D 147     -16.711 -56.436 124.593  1.00 43.76          O
ATOM   6039  CB   VAL D 147     -19.568 -55.348 124.331  1.00 32.86          C
ATOM   6040  CG1  VAL D 147     -20.166 -56.646 124.849  1.00 30.69          C
ATOM   6041  CG2  VAL D 147     -20.638 -54.322 124.288  1.00 31.55          C
ATOM   6042  N    GLN D 148     -17.443 -56.529 126.720  1.00 35.29          N
ATOM   6043  CA   GLN D 148     -16.699 -57.714 127.106  1.00 36.02          C
ATOM   6044  C    GLN D 148     -17.719 -58.707 127.647  1.00 37.18          C
ATOM   6045  O    GLN D 148     -18.459 -58.386 128.579  1.00 35.76          O
ATOM   6046  CB   GLN D 148     -15.637 -57.350 128.153  1.00 38.02          C
ATOM   6047  CG   GLN D 148     -14.870 -58.512 128.744  1.00 45.95          C
ATOM   6048  CD   GLN D 148     -13.798 -59.062 127.806  1.00 52.43          C
ATOM   6049  OE1  GLN D 148     -13.806 -60.255 127.467  1.00 49.60          O
ATOM   6050  NE2  GLN D 148     -12.857 -58.196 127.398  1.00 47.07          N
ATOM   6051  N    TRP D 149     -17.787 -59.886 127.037  1.00 36.10          N
ATOM   6052  CA   TRP D 149     -18.680 -60.955 127.473  1.00 37.96          C
ATOM   6053  C    TRP D 149     -17.977 -61.861 128.481  1.00 35.95          C
ATOM   6054  O    TRP D 149     -16.799 -62.191 128.318  1.00 37.64          O
ATOM   6055  CB   TRP D 149     -19.142 -61.800 126.281  1.00 33.14          C
ATOM   6056  CG   TRP D 149     -20.226 -61.240 125.409  1.00 34.49          C
ATOM   6057  CD1  TRP D 149     -20.083 -60.721 124.147  1.00 33.46          C
ATOM   6058  CD2  TRP D 149     -21.630 -61.200 125.697  1.00 36.39          C
ATOM   6059  NE1  TRP D 149     -21.301 -60.343 123.643  1.00 29.56          N
ATOM   6060  CE2  TRP D 149     -22.272 -60.620 124.571  1.00 33.79          C
ATOM   6061  CE3  TRP D 149     -22.406 -61.584 126.795  1.00 31.81          C
ATOM   6062  CZ2  TRP D 149     -23.653 -60.408 124.518  1.00 28.35          C
ATOM   6063  CZ3  TRP D 149     -23.777 -61.367 126.741  1.00 37.34          C
ATOM   6064  CH2  TRP D 149     -24.385 -60.787 125.607  1.00 31.64          C
ATOM   6065  N    LYS D 150     -18.700 -62.265 129.526  1.00 35.84          N
ATOM   6066  CA   LYS D 150     -18.195 -63.242 130.494  1.00 39.20          C
ATOM   6067  C    LYS D 150     -19.214 -64.355 130.710  1.00 34.68          C
ATOM   6068  O    LYS D 150     -20.391 -64.086 130.979  1.00 33.70          O
ATOM   6069  CB   LYS D 150     -17.856 -62.579 131.831  1.00 29.07          C
ATOM   6070  CG   LYS D 150     -16.601 -61.761 131.779  1.00 33.89          C
ATOM   6071  CD   LYS D 150     -16.602 -60.781 132.911  1.00 41.07          C
ATOM   6072  CE   LYS D 150     -15.607 -59.667 132.710  1.00 43.52          C
ATOM   6073  NZ   LYS D 150     -15.402 -58.953 134.001  1.00 45.35          N1+
ATOM   6074  N    VAL D 151     -18.756 -65.597 130.594  1.00 30.40          N
ATOM   6075  CA   VAL D 151     -19.524 -66.777 130.977  1.00 36.11          C
ATOM   6076  C    VAL D 151     -18.820 -67.400 132.176  1.00 36.89          C
ATOM   6077  O    VAL D 151     -17.662 -67.816 132.061  1.00 34.20          O
ATOM   6078  CB   VAL D 151     -19.637 -67.786 129.825  1.00 37.15          C
ATOM   6079  CG1  VAL D 151     -20.568 -68.901 130.211  1.00 34.22          C
ATOM   6080  CG2  VAL D 151     -20.111 -67.106 128.549  1.00 38.38          C
ATOM   6081  N    ASP D 152     -19.508 -67.435 133.333  1.00 40.45          N
ATOM   6082  CA   ASP D 152     -18.928 -67.863 134.626  1.00 35.46          C
ATOM   6083  C    ASP D 152     -17.564 -67.218 134.854  1.00 37.61          C
ATOM   6084  O    ASP D 152     -16.602 -67.868 135.265  1.00 40.92          O
ATOM   6085  CB   ASP D 152     -18.833 -69.391 134.739  1.00 34.39          C
ATOM   6086  CG   ASP D 152     -20.200 -70.051 134.992  1.00 43.58          C
ATOM   6087  OD1  ASP D 152     -21.057 -69.424 135.659  1.00 44.13          O
ATOM   6088  OD2  ASP D 152     -20.411 -71.206 134.549  1.00 44.21          O1-
ATOM   6089  N    ASN D 153     -17.493 -65.920 134.560  1.00 36.04          N
ATOM   6090  CA   ASN D 153     -16.305 -65.088 134.682  1.00 34.97          C
ATOM   6091  C    ASN D 153     -15.188 -65.521 133.737  1.00 37.78          C
ATOM   6092  O    ASN D 153     -14.015 -65.140 133.922  1.00 36.32          O
ATOM   6093  CB   ASN D 153     -15.802 -65.009 136.126  1.00 33.76          C
ATOM   6094  CG   ASN D 153     -15.114 -63.678 136.417  1.00 42.55          C
ATOM   6095  OD1  ASN D 153     -15.704 -62.598 136.248  1.00 42.84          O
ATOM   6096  ND2  ASN D 153     -13.853 -63.747 136.831  1.00 46.83          N
ATOM   6097  N    ALA D 154     -15.521 -66.293 132.706  1.00 34.52          N
ATOM   6098  CA   ALA D 154     -14.577 -66.555 131.629  1.00 35.50          C
ATOM   6099  C    ALA D 154     -14.784 -65.526 130.521  1.00 38.64          C
ATOM   6100  O    ALA D 154     -15.880 -65.417 129.954  1.00 35.76          O
ATOM   6101  CB   ALA D 154     -14.727 -67.973 131.090  1.00 31.21          C
```

```
ATOM   6102  N    LEU D 155     -13.725 -64.775 130.223  1.00 39.22           N
ATOM   6103  CA   LEU D 155     -13.728 -63.839 129.108  1.00 34.49           C
ATOM   6104  C    LEU D 155     -13.970 -64.578 127.815  1.00 31.95           C
ATOM   6105  O    LEU D 155     -13.276 -65.550 127.518  1.00 33.49           O
ATOM   6106  CB   LEU D 155     -12.391 -63.125 129.038  1.00 36.99           C
ATOM   6107  CG   LEU D 155     -12.350 -61.800 129.779  1.00 48.06           C
ATOM   6108  CD1  LEU D 155     -12.181 -62.015 131.301  1.00 43.48           C
ATOM   6109  CD2  LEU D 155     -11.219 -60.978 129.190  1.00 51.82           C
ATOM   6110  N    GLN D 156     -14.996 -64.166 127.080  1.00 36.40           N
ATOM   6111  CA   GLN D 156     -15.212 -64.660 125.725  1.00 35.33           C
ATOM   6112  C    GLN D 156     -14.505 -63.750 124.725  1.00 31.08           C
ATOM   6113  O    GLN D 156     -14.665 -62.526 124.763  1.00 36.72           O
ATOM   6114  CB   GLN D 156     -16.703 -64.738 125.398  1.00 32.10           C
ATOM   6115  CG   GLN D 156     -17.523 -65.447 126.450  1.00 35.66           C
ATOM   6116  CD   GLN D 156     -17.059 -66.878 126.701  1.00 36.63           C
ATOM   6117  OE1  GLN D 156     -17.090 -67.722 125.804  1.00 31.49           O
ATOM   6118  NE2  GLN D 156     -16.638 -67.153 127.929  1.00 35.80           N
ATOM   6119  N    SER D 157     -13.790 -64.354 123.795  1.00 24.35           N
ATOM   6120  CA   SER D 157     -13.068 -63.629 122.758  1.00 27.71           C
ATOM   6121  C    SER D 157     -13.201 -64.434 121.474  1.00 29.57           C
ATOM   6122  O    SER D 157     -12.715 -65.562 121.405  1.00 31.81           O
ATOM   6123  CB   SER D 157     -11.607 -63.449 123.142  1.00 27.38           C
ATOM   6124  OG   SER D 157     -10.897 -62.800 122.115  1.00 32.95           O
ATOM   6125  N    GLY D 158     -13.880 -63.886 120.474  1.00 25.60           N
ATOM   6126  CA   GLY D 158     -13.995 -64.518 119.171  1.00 23.02           C
ATOM   6127  C    GLY D 158     -15.329 -65.156 118.862  1.00 31.71           C
ATOM   6128  O    GLY D 158     -15.544 -65.567 117.710  1.00 30.83           O
ATOM   6129  N    ASN D 159     -16.253 -65.223 119.824  1.00 28.19           N
ATOM   6130  CA   ASN D 159     -17.529 -65.872 119.572  1.00 22.12           C
ATOM   6131  C    ASN D 159     -18.686 -64.886 119.660  1.00 25.52           C
ATOM   6132  O    ASN D 159     -19.803 -65.268 120.002  1.00 26.86           O
ATOM   6133  CB   ASN D 159     -17.749 -67.054 120.516  1.00 25.99           C
ATOM   6134  CG   ASN D 159     -17.495 -66.712 121.997  1.00 31.94           C
ATOM   6135  OD1  ASN D 159     -17.103 -65.589 122.345  1.00 28.21           O
ATOM   6136  ND2  ASN D 159     -17.712 -67.706 122.875  1.00 27.51           N
ATOM   6137  N    SER D 160     -18.445 -63.625 119.295  1.00 27.33           N
ATOM   6138  CA   SER D 160     -19.484 -62.606 119.297  1.00 26.68           C
ATOM   6139  C    SER D 160     -19.310 -61.677 118.099  1.00 24.99           C
ATOM   6140  O    SER D 160     -18.224 -61.571 117.525  1.00 29.23           O
ATOM   6141  CB   SER D 160     -19.475 -61.812 120.614  1.00 27.08           C
ATOM   6142  OG   SER D 160     -18.327 -61.009 120.707  1.00 26.36           O
ATOM   6143  N    GLN D 161     -20.407 -61.024 117.708  1.00 24.70           N
ATOM   6144  CA   GLN D 161     -20.404 -59.998 116.667  1.00 24.60           C
ATOM   6145  C    GLN D 161     -21.192 -58.766 117.128  1.00 29.81           C
ATOM   6146  O    GLN D 161     -21.972 -58.818 118.082  1.00 31.67           O
ATOM   6147  CB   GLN D 161     -20.954 -60.560 115.380  1.00 22.23           C
ATOM   6148  CG   GLN D 161     -20.025 -61.563 114.752  1.00 24.29           C
ATOM   6149  CD   GLN D 161     -20.631 -62.200 113.532  1.00 27.64           C
ATOM   6150  OE1  GLN D 161     -21.466 -63.098 113.629  1.00 32.76           O
ATOM   6151  NE2  GLN D 161     -20.246 -61.717 112.372  1.00 23.06           N
ATOM   6152  N    GLU D 162     -20.965 -57.641 116.458  1.00 30.47           N
ATOM   6153  CA   GLU D 162     -21.462 -56.341 116.919  1.00 34.77           C
ATOM   6154  C    GLU D 162     -21.983 -55.478 115.772  1.00 30.23           C
ATOM   6155  O    GLU D 162     -21.425 -55.485 114.678  1.00 26.52           O
ATOM   6156  CB   GLU D 162     -20.360 -55.528 117.568  1.00 32.41           C
ATOM   6157  CG   GLU D 162     -20.266 -55.519 119.031  1.00 32.77           C
ATOM   6158  CD   GLU D 162     -19.053 -54.695 119.437  1.00 41.06           C
ATOM   6159  OE1  GLU D 162     -18.443 -54.075 118.530  1.00 38.20           O
ATOM   6160  OE2  GLU D 162     -18.704 -54.667 120.637  1.00 50.77           O1-
ATOM   6161  N    SER D 163     -22.953 -54.617 116.084  1.00 27.60           N
ATOM   6162  CA   SER D 163     -23.426 -53.583 115.170  1.00 27.13           C
ATOM   6163  C    SER D 163     -23.656 -52.284 115.923  1.00 25.97           C
ATOM   6164  O    SER D 163     -24.105 -52.299 117.068  1.00 26.91           O
ATOM   6165  CB   SER D 163     -24.781 -53.931 114.531  1.00 29.47           C
ATOM   6166  OG   SER D 163     -24.662 -54.692 113.366  1.00 32.02           O
ATOM   6167  N    VAL D 164     -23.422 -51.161 115.243  1.00 20.54           N
ATOM   6168  CA   VAL D 164     -23.727 -49.843 115.781  1.00 23.87           C
ATOM   6169  C    VAL D 164     -24.632 -49.109 114.794  1.00 25.79           C
ATOM   6170  O    VAL D 164     -24.411 -49.146 113.580  1.00 28.96           O
ATOM   6171  CB   VAL D 164     -22.451 -49.022 116.079  1.00 27.40           C
ATOM   6172  CG1  VAL D 164     -22.804 -47.685 116.731  1.00 26.22           C
```

```
ATOM   6173  CG2 VAL D 164    -21.543 -49.782 116.997  1.00 25.86          C
ATOM   6174  N   THR D 165    -25.645 -48.432 115.318  1.00 22.06          N
ATOM   6175  CA  THR D 165    -26.530 -47.661 114.471  1.00 26.12          C
ATOM   6176  C   THR D 165    -25.845 -46.376 113.991  1.00 27.66          C
ATOM   6177  O   THR D 165    -24.781 -45.984 114.468  1.00 24.77          O
ATOM   6178  CB  THR D 165    -27.813 -47.311 115.216  1.00 27.08          C
ATOM   6179  OG1 THR D 165    -27.482 -46.765 116.504  1.00 27.47          O
ATOM   6180  CG2 THR D 165    -28.688 -48.544 115.355  1.00 26.44          C
ATOM   6181  N   GLU D 166    -26.479 -45.719 113.023  1.00 27.00          N
ATOM   6182  CA  GLU D 166    -26.079 -44.370 112.686  1.00 27.71          C
ATOM   6183  C   GLU D 166    -26.495 -43.432 113.817  1.00 33.03          C
ATOM   6184  O   GLU D 166    -27.359 -43.761 114.640  1.00 33.28          O
ATOM   6185  CB  GLU D 166    -26.689 -43.951 111.339  1.00 30.38          C
ATOM   6186  CG  GLU D 166    -26.119 -44.711 110.120  1.00 25.40          C
ATOM   6187  CD  GLU D 166    -24.599 -44.606 110.040  1.00 38.65          C
ATOM   6188  OE1 GLU D 166    -24.038 -43.552 110.423  1.00 46.73          O
ATOM   6189  OE2 GLU D 166    -23.946 -45.597 109.652  1.00 40.23          O1-
ATOM   6190  N   GLN D 167    -25.866 -42.254 113.856  1.00 29.35          N
ATOM   6191  CA  GLN D 167    -26.181 -41.280 114.885  1.00 27.68          C
ATOM   6192  C   GLN D 167    -27.675 -40.977 114.888  1.00 30.18          C
ATOM   6193  O   GLN D 167    -28.273 -40.743 113.841  1.00 28.88          O
ATOM   6194  CB  GLN D 167    -25.371 -40.014 114.663  1.00 26.86          C
ATOM   6195  CG  GLN D 167    -25.050 -39.318 115.952  1.00 30.75          C
ATOM   6196  CD  GLN D 167    -24.012 -38.230 115.810  1.00 30.88          C
ATOM   6197  OE1 GLN D 167    -23.630 -37.851 114.707  1.00 26.28          O
ATOM   6198  NE2 GLN D 167    -23.526 -37.741 116.942  1.00 31.17          N
ATOM   6199  N   ASP D 168    -28.284 -40.993 116.073  1.00 32.93          N
ATOM   6200  CA  ASP D 168    -29.740 -40.995 116.145  1.00 30.89          C
ATOM   6201  C   ASP D 168    -30.322 -39.647 115.730  1.00 32.78          C
ATOM   6202  O   ASP D 168    -29.864 -38.588 116.175  1.00 32.90          O
ATOM   6203  CB  ASP D 168    -30.198 -41.372 117.553  1.00 31.28          C
ATOM   6204  CG  ASP D 168    -31.716 -41.521 117.659  1.00 38.67          C
ATOM   6205  OD2 ASP D 168    -32.352 -40.708 118.357  1.00 38.36          O
ATOM   6206  OD1 ASP D 168    -32.272 -42.488 117.088  1.00 46.18          O1-
ATOM   6207  N   SER D 169    -31.354 -39.697 114.883  1.00 39.01          N
ATOM   6208  CA  SER D 169    -31.955 -38.482 114.346  1.00 32.75          C
ATOM   6209  C   SER D 169    -32.582 -37.588 115.413  1.00 37.05          C
ATOM   6210  O   SER D 169    -32.812 -36.404 115.145  1.00 38.37          O
ATOM   6211  CB  SER D 169    -33.001 -38.845 113.284  1.00 39.20          C
ATOM   6212  OG  SER D 169    -34.079 -39.588 113.824  1.00 50.23          O
ATOM   6213  N   LYS D 170    -32.814 -38.077 116.629  1.00 34.51          N
ATOM   6214  CA  LYS D 170    -33.421 -37.203 117.625  1.00 34.07          C
ATOM   6215  C   LYS D 170    -32.468 -36.701 118.701  1.00 33.50          C
ATOM   6216  O   LYS D 170    -32.589 -35.552 119.108  1.00 37.46          O
ATOM   6217  CB  LYS D 170    -34.629 -37.888 118.275  1.00 37.63          C
ATOM   6218  CG  LYS D 170    -35.389 -37.006 119.243  1.00 37.03          C
ATOM   6219  CD  LYS D 170    -36.331 -37.786 120.178  1.00 38.46          C
ATOM   6220  CE  LYS D 170    -37.575 -38.283 119.424  1.00 45.87          C
ATOM   6221  NZ  LYS D 170    -38.639 -38.916 120.302  1.00 39.82          N1+
ATOM   6222  N   ASP D 171    -31.531 -37.506 119.210  1.00 36.72          N
ATOM   6223  CA  ASP D 171    -30.652 -37.034 120.285  1.00 30.44          C
ATOM   6224  C   ASP D 171    -29.163 -37.175 119.956  1.00 31.83          C
ATOM   6225  O   ASP D 171    -28.327 -37.027 120.864  1.00 24.40          O
ATOM   6226  CB  ASP D 171    -30.977 -37.745 121.621  1.00 24.58          C
ATOM   6227  CG  ASP D 171    -30.724 -39.290 121.598  1.00 37.38          C
ATOM   6228  OD1 ASP D 171    -29.900 -39.800 120.803  1.00 38.28          O
ATOM   6229  OD2 ASP D 171    -31.345 -40.026 122.402  1.00 44.17          O1-
ATOM   6230  N   SER D 172    -28.814 -37.467 118.691  1.00 27.49          N
ATOM   6231  CA  SER D 172    -27.433 -37.542 118.210  1.00 29.50          C
ATOM   6232  C   SER D 172    -26.575 -38.575 118.954  1.00 33.88          C
ATOM   6233  O   SER D 172    -25.339 -38.428 119.009  1.00 32.88          O
ATOM   6234  CB  SER D 172    -26.756 -36.167 118.283  1.00 28.79          C
ATOM   6235  OG  SER D 172    -27.503 -35.209 117.557  1.00 32.16          O
ATOM   6236  N   THR D 173    -27.185 -39.602 119.550  1.00 25.27          N
ATOM   6237  CA  THR D 173    -26.443 -40.663 120.214  1.00 26.81          C
ATOM   6238  C   THR D 173    -26.265 -41.868 119.287  1.00 28.18          C
ATOM   6239  O   THR D 173    -26.791 -41.932 118.163  1.00 26.72          O
ATOM   6240  CB  THR D 173    -27.127 -41.101 121.529  1.00 29.20          C
ATOM   6241  OG1 THR D 173    -28.454 -41.578 121.279  1.00 26.42          O
ATOM   6242  CG2 THR D 173    -27.181 -39.948 122.547  1.00 27.06          C
ATOM   6243  N   TYR D 174    -25.492 -42.824 119.787  1.00 24.11          N
```

```
ATOM   6244  CA   TYR D 174    -25.252 -44.113 119.162  1.00 25.18           C
ATOM   6245  C    TYR D 174    -25.858 -45.225 120.001  1.00 27.09           C
ATOM   6246  O    TYR D 174    -25.925 -45.130 121.232  1.00 26.92           O
ATOM   6247  CB   TYR D 174    -23.740 -44.371 118.993  1.00 24.71           C
ATOM   6248  CG   TYR D 174    -23.145 -43.461 117.980  1.00 27.06           C
ATOM   6249  CD1  TYR D 174    -23.212 -43.778 116.625  1.00 23.86           C
ATOM   6250  CD2  TYR D 174    -22.587 -42.234 118.355  1.00 29.38           C
ATOM   6251  CE1  TYR D 174    -22.697 -42.931 115.671  1.00 28.55           C
ATOM   6252  CE2  TYR D 174    -22.060 -41.366 117.397  1.00 28.07           C
ATOM   6253  CZ   TYR D 174    -22.125 -41.719 116.053  1.00 30.70           C
ATOM   6254  OH   TYR D 174    -21.626 -40.882 115.081  1.00 29.66           O
ATOM   6255  N    SER D 175    -26.220 -46.318 119.329  1.00 29.12           N
ATOM   6256  CA   SER D 175    -26.623 -47.546 120.005  1.00 29.70           C
ATOM   6257  C    SER D 175    -25.851 -48.723 119.437  1.00 27.77           C
ATOM   6258  O    SER D 175    -25.515 -48.748 118.252  1.00 26.37           O
ATOM   6259  CB   SER D 175    -28.127 -47.796 119.868  1.00 28.32           C
ATOM   6260  OG   SER D 175    -28.822 -46.950 120.755  1.00 29.89           O
ATOM   6261  N    LEU D 176    -25.615 -49.725 120.284  1.00 29.44           N
ATOM   6262  CA   LEU D 176    -24.808 -50.884 119.918  1.00 26.61           C
ATOM   6263  C    LEU D 176    -25.499 -52.174 120.346  1.00 25.01           C
ATOM   6264  O    LEU D 176    -26.055 -52.255 121.444  1.00 25.06           O
ATOM   6265  CB   LEU D 176    -23.412 -50.791 120.557  1.00 23.61           C
ATOM   6266  CG   LEU D 176    -22.361 -51.831 120.176  1.00 26.86           C
ATOM   6267  CD1  LEU D 176    -20.995 -51.225 120.278  1.00 28.36           C
ATOM   6268  CD2  LEU D 176    -22.428 -53.052 121.099  1.00 25.35           C
ATOM   6269  N    SER D 177    -25.405 -53.202 119.500  1.00 26.18           N
ATOM   6270  CA   SER D 177    -25.932 -54.536 119.789  1.00 25.92           C
ATOM   6271  C    SER D 177    -24.840 -55.587 119.589  1.00 25.55           C
ATOM   6272  O    SER D 177    -24.136 -55.566 118.577  1.00 30.42           O
ATOM   6273  CB   SER D 177    -27.142 -54.840 118.895  1.00 30.52           C
ATOM   6274  OG   SER D 177    -27.570 -56.176 119.035  1.00 31.20           O
ATOM   6275  N    SER D 178    -24.668 -56.474 120.574  1.00 28.18           N
ATOM   6276  CA   SER D 178    -23.659 -57.527 120.542  1.00 25.27           C
ATOM   6277  C    SER D 178    -24.288 -58.885 120.794  1.00 26.75           C
ATOM   6278  O    SER D 178    -25.102 -59.040 121.705  1.00 31.18           O
ATOM   6279  CB   SER D 178    -22.560 -57.312 121.568  1.00 30.71           C
ATOM   6280  OG   SER D 178    -21.592 -58.348 121.447  1.00 32.24           O
ATOM   6281  N    THR D 179    -23.888 -59.880 120.010  1.00 27.93           N
ATOM   6282  CA   THR D 179    -24.458 -61.220 120.122  1.00 29.75           C
ATOM   6283  C    THR D 179    -23.379 -62.248 120.444  1.00 31.38           C
ATOM   6284  O    THR D 179    -22.529 -62.546 119.604  1.00 25.41           O
ATOM   6285  CB   THR D 179    -25.160 -61.608 118.837  1.00 32.19           C
ATOM   6286  OG1  THR D 179    -26.110 -60.586 118.488  1.00 42.39           O
ATOM   6287  CG2  THR D 179    -25.841 -62.933 119.026  1.00 33.94           C
ATOM   6288  N    LEU D 180    -23.448 -62.822 121.641  1.00 33.64           N
ATOM   6289  CA   LEU D 180    -22.651 -63.988 121.989  1.00 26.84           C
ATOM   6290  C    LEU D 180    -23.377 -65.233 121.509  1.00 28.96           C
ATOM   6291  O    LEU D 180    -24.576 -65.383 121.759  1.00 33.24           O
ATOM   6292  CB   LEU D 180    -22.446 -64.042 123.496  1.00 26.91           C
ATOM   6293  CG   LEU D 180    -21.584 -65.169 124.035  1.00 32.83           C
ATOM   6294  CD1  LEU D 180    -20.179 -64.954 123.541  1.00 32.39           C
ATOM   6295  CD2  LEU D 180    -21.624 -65.196 125.566  1.00 35.83           C
ATOM   6296  N    THR D 181    -22.683 -66.101 120.784  1.00 24.60           N
ATOM   6297  CA   THR D 181    -23.289 -67.321 120.267  1.00 29.40           C
ATOM   6298  C    THR D 181    -22.656 -68.546 120.928  1.00 35.38           C
ATOM   6299  O    THR D 181    -21.430 -68.622 121.063  1.00 32.82           O
ATOM   6300  CB   THR D 181    -23.162 -67.393 118.744  1.00 29.01           C
ATOM   6301  OG1  THR D 181    -23.762 -66.235 118.169  1.00 36.58           O
ATOM   6302  CG2  THR D 181    -23.943 -68.571 118.214  1.00 35.08           C
ATOM   6303  N    LEU D 182    -23.499 -69.481 121.375  1.00 33.54           N
ATOM   6304  CA   LEU D 182    -23.061 -70.743 121.957  1.00 31.73           C
ATOM   6305  C    LEU D 182    -23.908 -71.860 121.379  1.00 38.06           C
ATOM   6306  O    LEU D 182    -25.010 -71.638 120.866  1.00 39.76           O
ATOM   6307  CB   LEU D 182    -23.242 -70.791 123.473  1.00 34.20           C
ATOM   6308  CG   LEU D 182    -22.667 -69.731 124.395  1.00 37.93           C
ATOM   6309  CD1  LEU D 182    -23.089 -70.021 125.827  1.00 39.29           C
ATOM   6310  CD2  LEU D 182    -21.154 -69.692 124.276  1.00 41.23           C
ATOM   6311  N    SER D 183    -23.401 -73.075 121.491  1.00 41.78           N
ATOM   6312  CA   SER D 183    -24.276 -74.218 121.302  1.00 41.97           C
ATOM   6313  C    SER D 183    -25.171 -74.354 122.533  1.00 40.93           C
ATOM   6314  O    SER D 183    -24.832 -73.874 123.619  1.00 39.70           O
```

```
ATOM   6315  CB  SER D 183     -23.449 -75.481 121.071  1.00 42.62          C
ATOM   6316  OG  SER D 183     -22.677 -75.783 122.224  1.00 45.39          O
ATOM   6317  N   LYS D 184     -26.323 -75.017 122.368  1.00 38.98          N
ATOM   6318  CA  LYS D 184     -27.172 -75.267 123.536  1.00 46.77          C
ATOM   6319  C   LYS D 184     -26.428 -76.067 124.602  1.00 45.69          C
ATOM   6320  O   LYS D 184     -26.586 -75.820 125.806  1.00 44.13          O
ATOM   6321  CB  LYS D 184     -28.465 -75.982 123.135  1.00 52.78          C
ATOM   6322  CG  LYS D 184     -29.383 -76.279 124.334  1.00 45.82          C
ATOM   6323  CD  LYS D 184     -30.721 -76.916 123.925  1.00 49.11          C
ATOM   6324  CE  LYS D 184     -30.567 -78.342 123.414  1.00 56.53          C
ATOM   6325  NZ  LYS D 184     -31.811 -78.834 122.761  1.00 58.33          N1+
ATOM   6326  N   ALA D 185     -25.599 -77.019 124.172  1.00 44.49          N
ATOM   6327  CA  ALA D 185     -24.835 -77.822 125.116  1.00 44.16          C
ATOM   6328  C   ALA D 185     -23.903 -76.953 125.958  1.00 46.66          C
ATOM   6329  O   ALA D 185     -23.932 -77.018 127.193  1.00 48.01          O
ATOM   6330  CB  ALA D 185     -24.067 -78.902 124.363  1.00 43.48          C
ATOM   6331  N   ASP D 186     -23.057 -76.142 125.307  1.00 47.56          N
ATOM   6332  CA  ASP D 186     -22.203 -75.208 126.042  1.00 43.67          C
ATOM   6333  C   ASP D 186     -23.040 -74.263 126.887  1.00 41.82          C
ATOM   6334  O   ASP D 186     -22.744 -74.029 128.065  1.00 43.44          O
ATOM   6335  CB  ASP D 186     -21.334 -74.400 125.079  1.00 47.25          C
ATOM   6336  CG  ASP D 186     -20.218 -75.210 124.472  1.00 49.80          C
ATOM   6337  OD1 ASP D 186     -19.524 -75.925 125.218  1.00 51.32          O
ATOM   6338  OD2 ASP D 186     -20.038 -75.131 123.241  1.00 59.81          O1-
ATOM   6339  N   TYR D 187     -24.100 -73.713 126.296  1.00 42.10          N
ATOM   6340  CA  TYR D 187     -24.958 -72.799 127.030  1.00 42.00          C
ATOM   6341  C   TYR D 187     -25.446 -73.439 128.322  1.00 40.74          C
ATOM   6342  O   TYR D 187     -25.462 -72.797 129.381  1.00 36.12          O
ATOM   6343  CB  TYR D 187     -26.148 -72.356 126.154  1.00 35.24          C
ATOM   6344  CG  TYR D 187     -27.113 -71.482 126.924  1.00 36.73          C
ATOM   6345  CD1 TYR D 187     -26.712 -70.236 127.403  1.00 33.83          C
ATOM   6346  CD2 TYR D 187     -28.407 -71.905 127.202  1.00 37.83          C
ATOM   6347  CE1 TYR D 187     -27.570 -69.443 128.137  1.00 35.37          C
ATOM   6348  CE2 TYR D 187     -29.278 -71.112 127.932  1.00 36.87          C
ATOM   6349  CZ  TYR D 187     -28.848 -69.883 128.404  1.00 34.37          C
ATOM   6350  OH  TYR D 187     -29.704 -69.082 129.125  1.00 33.53          O
ATOM   6351  N   GLU D 188     -25.818 -74.715 128.266  1.00 44.28          N
ATOM   6352  CA  GLU D 188     -26.463 -75.311 129.426  1.00 48.57          C
ATOM   6353  C   GLU D 188     -25.494 -75.827 130.473  1.00 44.50          C
ATOM   6354  O   GLU D 188     -25.954 -76.294 131.513  1.00 43.86          O
ATOM   6355  CB  GLU D 188     -27.384 -76.431 128.984  1.00 46.87          C
ATOM   6356  CG  GLU D 188     -28.803 -75.966 128.881  1.00 52.89          C
ATOM   6357  CD  GLU D 188     -29.586 -76.862 127.993  1.00 60.48          C
ATOM   6358  OE1 GLU D 188     -28.963 -77.811 127.472  1.00 63.90          O
ATOM   6359  OE2 GLU D 188     -30.800 -76.623 127.817  1.00 70.92          O1-
ATOM   6360  N   LYS D 189     -24.184 -75.709 130.249  1.00 42.21          N
ATOM   6361  CA  LYS D 189     -23.166 -76.082 131.214  1.00 34.10          C
ATOM   6362  C   LYS D 189     -22.659 -74.905 132.027  1.00 41.19          C
ATOM   6363  O   LYS D 189     -21.617 -75.025 132.675  1.00 43.76          O
ATOM   6364  CB  LYS D 189     -21.981 -76.727 130.501  1.00 42.21          C
ATOM   6365  CG  LYS D 189     -22.277 -78.025 129.749  1.00 49.04          C
ATOM   6366  CD  LYS D 189     -21.065 -78.418 128.889  1.00 50.62          C
ATOM   6367  CE  LYS D 189     -21.332 -79.669 128.071  1.00 61.29          C
ATOM   6368  NZ  LYS D 189     -20.165 -79.960 127.197  1.00 67.56          N1+
ATOM   6369  N   HIS D 190     -23.349 -73.768 132.011  1.00 40.38          N
ATOM   6370  CA  HIS D 190     -22.850 -72.595 132.712  1.00 37.11          C
ATOM   6371  C   HIS D 190     -24.018 -71.833 133.309  1.00 37.04          C
ATOM   6372  O   HIS D 190     -25.167 -72.014 132.913  1.00 39.95          O
ATOM   6373  CB  HIS D 190     -22.044 -71.702 131.782  1.00 41.03          C
ATOM   6374  CG  HIS D 190     -20.840 -72.376 131.212  1.00 41.24          C
ATOM   6375  ND1 HIS D 190     -19.759 -72.747 131.983  1.00 41.48          N
ATOM   6376  CD2 HIS D 190     -20.560 -72.778 129.951  1.00 39.46          C
ATOM   6377  CE1 HIS D 190     -18.856 -73.333 131.218  1.00 36.30          C
ATOM   6378  NE2 HIS D 190     -19.320 -73.368 129.981  1.00 39.82          N
ATOM   6379  N   LYS D 191     -23.719 -70.969 134.270  1.00 34.63          N
ATOM   6380  CA  LYS D 191     -24.775 -70.340 135.046  1.00 45.76          C
ATOM   6381  C   LYS D 191     -24.874 -68.842 134.790  1.00 46.36          C
ATOM   6382  O   LYS D 191     -25.944 -68.357 134.407  1.00 46.07          O
ATOM   6383  CB  LYS D 191     -24.566 -70.607 136.548  1.00 42.22          C
ATOM   6384  CG  LYS D 191     -25.568 -69.875 137.451  1.00 49.74          C
ATOM   6385  CD  LYS D 191     -25.227 -70.029 138.954  1.00 59.90          C
```

```
ATOM   6386  CE   LYS D 191     -26.350 -69.510 139.853  1.00 60.67           C
ATOM   6387  NZ   LYS D 191     -26.031 -69.671 141.293  1.00 66.07           N1+
ATOM   6388  N    VAL D 192     -23.782 -68.100 134.968  1.00 39.06           N
ATOM   6389  CA   VAL D 192     -23.809 -66.644 134.941  1.00 38.75           C
ATOM   6390  C    VAL D 192     -23.378 -66.173 133.553  1.00 39.96           C
ATOM   6391  O    VAL D 192     -22.277 -66.490 133.091  1.00 41.97           O
ATOM   6392  CB   VAL D 192     -22.906 -66.063 136.039  1.00 36.31           C
ATOM   6393  CG1  VAL D 192     -22.876 -64.544 135.989  1.00 34.38           C
ATOM   6394  CG2  VAL D 192     -23.360 -66.548 137.380  1.00 41.02           C
ATOM   6395  N    TYR D 193     -24.241 -65.414 132.886  1.00 38.30           N
ATOM   6396  CA   TYR D 193     -23.924 -64.789 131.608  1.00 35.90           C
ATOM   6397  C    TYR D 193     -23.862 -63.290 131.845  1.00 33.90           C
ATOM   6398  O    TYR D 193     -24.747 -62.734 132.487  1.00 36.52           O
ATOM   6399  CB   TYR D 193     -24.957 -65.165 130.534  1.00 31.33           C
ATOM   6400  CG   TYR D 193     -24.828 -66.629 130.183  1.00 35.91           C
ATOM   6401  CD1  TYR D 193     -25.458 -67.608 130.950  1.00 38.83           C
ATOM   6402  CD2  TYR D 193     -23.971 -67.049 129.171  1.00 38.88           C
ATOM   6403  CE1  TYR D 193     -25.297 -68.968 130.671  1.00 36.63           C
ATOM   6404  CE2  TYR D 193     -23.799 -68.410 128.885  1.00 39.70           C
ATOM   6405  CZ   TYR D 193     -24.461 -69.359 129.646  1.00 36.21           C
ATOM   6406  OH   TYR D 193     -24.302 -70.691 129.360  1.00 37.29           O
ATOM   6407  N    ALA D 194     -22.791 -62.646 131.400  1.00 33.30           N
ATOM   6408  CA   ALA D 194     -22.654 -61.226 131.670  1.00 34.22           C
ATOM   6409  C    ALA D 194     -22.000 -60.510 130.488  1.00 36.04           C
ATOM   6410  O    ALA D 194     -21.164 -61.083 129.782  1.00 35.02           O
ATOM   6411  CB   ALA D 194     -21.857 -61.026 132.952  1.00 29.25           C
ATOM   6412  N    CYS D 195     -22.396 -59.254 130.263  1.00 32.88           N
ATOM   6413  CA   CYS D 195     -21.638 -58.361 129.389  1.00 39.71           C
ATOM   6414  C    CYS D 195     -21.246 -57.121 130.170  1.00 37.39           C
ATOM   6415  O    CYS D 195     -22.084 -56.498 130.839  1.00 33.62           O
ATOM   6416  CB   CYS D 195     -22.382 -57.939 128.108  1.00 39.73           C
ATOM   6417  SG   CYS D 195     -23.983 -57.183 128.349  1.00 55.69           S
ATOM   6418  N    GLU D 196     -19.981 -56.753 130.035  1.00 33.07           N
ATOM   6419  CA   GLU D 196     -19.377 -55.641 130.742  1.00 38.70           C
ATOM   6420  C    GLU D 196     -19.127 -54.523 129.734  1.00 35.02           C
ATOM   6421  O    GLU D 196     -18.610 -54.777 128.639  1.00 28.28           O
ATOM   6422  CB   GLU D 196     -18.078 -56.104 131.410  1.00 35.85           C
ATOM   6423  CG   GLU D 196     -17.366 -55.070 132.220  1.00 39.77           C
ATOM   6424  CD   GLU D 196     -15.962 -55.516 132.610  1.00 46.03           C
ATOM   6425  OE1  GLU D 196     -15.435 -56.465 131.988  1.00 40.61           O
ATOM   6426  OE2  GLU D 196     -15.373 -54.880 133.511  1.00 57.77           O1-
ATOM   6427  N    VAL D 197     -19.512 -53.297 130.097  1.00 34.66           N
ATOM   6428  CA   VAL D 197     -19.563 -52.169 129.169  1.00 35.82           C
ATOM   6429  C    VAL D 197     -18.595 -51.112 129.680  1.00 34.18           C
ATOM   6430  O    VAL D 197     -18.751 -50.610 130.797  1.00 35.27           O
ATOM   6431  CB   VAL D 197     -20.993 -51.601 129.024  1.00 31.64           C
ATOM   6432  CG1  VAL D 197     -21.014 -50.336 128.199  1.00 28.99           C
ATOM   6433  CG2  VAL D 197     -21.944 -52.640 128.462  1.00 28.78           C
ATOM   6434  N    THR D 198     -17.577 -50.805 128.883  1.00 33.99           N
ATOM   6435  CA   THR D 198     -16.654 -49.718 129.164  1.00 32.70           C
ATOM   6436  C    THR D 198     -16.983 -48.563 128.229  1.00 40.52           C
ATOM   6437  O    THR D 198     -17.023 -48.743 127.005  1.00 37.25           O
ATOM   6438  CB   THR D 198     -15.203 -50.146 128.950  1.00 33.81           C
ATOM   6439  OG1  THR D 198     -14.907 -51.282 129.766  1.00 43.84           O
ATOM   6440  CG2  THR D 198     -14.282 -49.029 129.347  1.00 29.60           C
ATOM   6441  N    HIS D 199     -17.211 -47.384 128.805  1.00 37.58           N
ATOM   6442  CA   HIS D 199     -17.528 -46.198 128.030  1.00 31.22           C
ATOM   6443  C    HIS D 199     -17.075 -44.973 128.805  1.00 34.53           C
ATOM   6444  O    HIS D 199     -17.002 -44.999 130.033  1.00 35.60           O
ATOM   6445  CB   HIS D 199     -19.020 -46.115 127.723  1.00 33.40           C
ATOM   6446  CG   HIS D 199     -19.383 -44.969 126.828  1.00 34.76           C
ATOM   6447  ND1  HIS D 199     -19.824 -43.756 127.313  1.00 30.33           N
ATOM   6448  CD2  HIS D 199     -19.360 -44.849 125.479  1.00 32.19           C
ATOM   6449  CE1  HIS D 199     -20.061 -42.940 126.301  1.00 31.06           C
ATOM   6450  NE2  HIS D 199     -19.778 -43.575 125.178  1.00 31.67           N
ATOM   6451  N    GLN D 200     -16.786 -43.888 128.085  1.00 29.72           N
ATOM   6452  CA   GLN D 200     -16.199 -42.738 128.754  1.00 31.37           C
ATOM   6453  C    GLN D 200     -17.177 -42.022 129.684  1.00 35.36           C
ATOM   6454  O    GLN D 200     -16.735 -41.248 130.541  1.00 42.18           O
ATOM   6455  CB   GLN D 200     -15.629 -41.779 127.723  1.00 33.74           C
ATOM   6456  CG   GLN D 200     -16.370 -40.476 127.548  1.00 35.08           C
```

```
ATOM   6457  CD   GLN D 200     -15.538 -39.467 126.778  1.00 39.26           C
ATOM   6458  OE1  GLN D 200     -15.357 -38.324 127.205  1.00 44.14           O
ATOM   6459  NE2  GLN D 200     -15.015 -39.893 125.639  1.00 40.13           N
ATOM   6460  N    GLY D 201     -18.482 -42.261 129.550  1.00 34.51           N
ATOM   6461  CA   GLY D 201     -19.496 -41.749 130.451  1.00 32.88           C
ATOM   6462  C    GLY D 201     -19.793 -42.624 131.658  1.00 34.93           C
ATOM   6463  O    GLY D 201     -20.740 -42.333 132.397  1.00 30.85           O
ATOM   6464  N    LEU D 202     -19.050 -43.719 131.838  1.00 30.30           N
ATOM   6465  CA   LEU D 202     -19.137 -44.588 133.007  1.00 35.37           C
ATOM   6466  C    LEU D 202     -17.845 -44.491 133.805  1.00 41.52           C
ATOM   6467  O    LEU D 202     -16.751 -44.633 133.240  1.00 41.27           O
ATOM   6468  CB   LEU D 202     -19.405 -46.046 132.624  1.00 36.42           C
ATOM   6469  CG   LEU D 202     -20.723 -46.250 131.880  1.00 35.17           C
ATOM   6470  CD1  LEU D 202     -20.916 -47.704 131.503  1.00 29.43           C
ATOM   6471  CD2  LEU D 202     -21.865 -45.735 132.713  1.00 29.90           C
ATOM   6472  N    SER D 203     -17.973 -44.219 135.113  1.00 44.01           N
ATOM   6473  CA   SER D 203     -16.802 -44.095 135.978  1.00 39.30           C
ATOM   6474  C    SER D 203     -16.068 -45.416 136.165  1.00 41.65           C
ATOM   6475  O    SER D 203     -14.867 -45.408 136.460  1.00 49.60           O
ATOM   6476  CB   SER D 203     -17.213 -43.537 137.323  1.00 34.65           C
ATOM   6477  OG   SER D 203     -18.227 -44.369 137.839  1.00 55.55           O
ATOM   6478  N    SER D 204     -16.749 -46.545 136.003  1.00 42.27           N
ATOM   6479  CA   SER D 204     -16.084 -47.841 135.927  1.00 45.69           C
ATOM   6480  C    SER D 204     -16.989 -48.788 135.149  1.00 36.06           C
ATOM   6481  O    SER D 204     -18.181 -48.508 134.979  1.00 38.46           O
ATOM   6482  CB   SER D 204     -15.755 -48.401 137.332  1.00 48.88           C
ATOM   6483  OG   SER D 204     -16.914 -48.719 138.093  1.00 47.01           O
ATOM   6484  N    PRO D 205     -16.453 -49.888 134.637  1.00 32.30           N
ATOM   6485  CA   PRO D 205     -17.271 -50.768 133.791  1.00 36.16           C
ATOM   6486  C    PRO D 205     -18.534 -51.253 134.498  1.00 37.49           C
ATOM   6487  O    PRO D 205     -18.503 -51.695 135.642  1.00 46.01           O
ATOM   6488  CB   PRO D 205     -16.313 -51.925 133.468  1.00 34.46           C
ATOM   6489  CG   PRO D 205     -14.973 -51.268 133.442  1.00 33.52           C
ATOM   6490  CD   PRO D 205     -15.013 -50.158 134.481  1.00 32.12           C
ATOM   6491  N    VAL D 206     -19.629 -51.261 133.750  1.00 37.13           N
ATOM   6492  CA   VAL D 206     -20.952 -51.648 134.217  1.00 32.96           C
ATOM   6493  C    VAL D 206     -21.270 -53.027 133.650  1.00 35.67           C
ATOM   6494  O    VAL D 206     -21.071 -53.272 132.456  1.00 33.93           O
ATOM   6495  CB   VAL D 206     -21.984 -50.601 133.769  1.00 30.63           C
ATOM   6496  CG1  VAL D 206     -23.390 -51.059 134.005  1.00 30.69           C
ATOM   6497  CG2  VAL D 206     -21.703 -49.316 134.479  1.00 38.52           C
ATOM   6498  N    THR D 207     -21.748 -53.932 134.503  1.00 40.16           N
ATOM   6499  CA   THR D 207     -22.093 -55.285 134.087  1.00 33.00           C
ATOM   6500  C    THR D 207     -23.587 -55.538 134.269  1.00 33.37           C
ATOM   6501  O    THR D 207     -24.188 -55.112 135.255  1.00 34.36           O
ATOM   6502  CB   THR D 207     -21.271 -56.317 134.871  1.00 32.59           C
ATOM   6503  OG1  THR D 207     -19.872 -56.064 134.677  1.00 35.95           O
ATOM   6504  CG2  THR D 207     -21.585 -57.744 134.412  1.00 31.54           C
ATOM   6505  N    LYS D 208     -24.194 -56.172 133.277  1.00 38.08           N
ATOM   6506  CA   LYS D 208     -25.529 -56.734 133.393  1.00 33.58           C
ATOM   6507  C    LYS D 208     -25.435 -58.233 133.155  1.00 31.51           C
ATOM   6508  O    LYS D 208     -24.797 -58.679 132.202  1.00 36.26           O
ATOM   6509  CB   LYS D 208     -26.510 -56.072 132.395  1.00 32.49           C
ATOM   6510  CG   LYS D 208     -26.633 -54.559 132.573  1.00 34.11           C
ATOM   6511  CD   LYS D 208     -26.879 -54.180 134.030  1.00 32.10           C
ATOM   6512  CE   LYS D 208     -27.135 -52.694 134.212  1.00 31.43           C
ATOM   6513  NZ   LYS D 208     -28.595 -52.396 134.289  1.00 30.11           N1+
ATOM   6514  N    SER D 209     -26.099 -59.013 133.995  1.00 40.89           N
ATOM   6515  CA   SER D 209     -25.955 -60.457 133.950  1.00 34.12           C
ATOM   6516  C    SER D 209     -27.289 -61.121 134.250  1.00 35.96           C
ATOM   6517  O    SER D 209     -28.265 -60.469 134.620  1.00 35.44           O
ATOM   6518  CB   SER D 209     -24.856 -60.923 134.926  1.00 34.50           C
ATOM   6519  OG   SER D 209     -25.082 -60.438 136.238  1.00 29.12           O
ATOM   6520  N    PHE D 210     -27.333 -62.431 134.025  1.00 39.78           N
ATOM   6521  CA   PHE D 210     -28.424 -63.272 134.488  1.00 37.92           C
ATOM   6522  C    PHE D 210     -27.883 -64.670 134.767  1.00 42.87           C
ATOM   6523  O    PHE D 210     -26.994 -65.149 134.055  1.00 42.97           O
ATOM   6524  CB   PHE D 210     -29.555 -63.296 133.464  1.00 34.40           C
ATOM   6525  CG   PHE D 210     -29.193 -63.943 132.160  1.00 40.31           C
ATOM   6526  CD1  PHE D 210     -29.365 -65.304 131.973  1.00 38.92           C
ATOM   6527  CD2  PHE D 210     -28.702 -63.182 131.109  1.00 37.04           C
```

```
ATOM   6528  CE1 PHE D 210     -29.055 -65.893 130.777  1.00 36.51           C
ATOM   6529  CE2 PHE D 210     -28.393 -63.765 129.913  1.00 35.57           C
ATOM   6530  CZ  PHE D 210     -28.568 -65.125 129.745  1.00 36.28           C
ATOM   6531  N   ASN D 211     -28.433 -65.331 135.796  1.00 41.98           N
ATOM   6532  CA  ASN D 211     -28.093 -66.720 136.076  1.00 44.07           C
ATOM   6533  C   ASN D 211     -29.047 -67.583 135.273  1.00 45.82           C
ATOM   6534  O   ASN D 211     -30.260 -67.386 135.349  1.00 49.01           O
ATOM   6535  CB  ASN D 211     -28.194 -67.060 137.568  1.00 42.88           C
ATOM   6536  CG  ASN D 211     -27.474 -66.058 138.456  1.00 48.18           C
ATOM   6537  OD1 ASN D 211     -26.529 -65.407 138.034  1.00 55.66           O
ATOM   6538  ND2 ASN D 211     -27.920 -65.934 139.701  1.00 53.20           N
ATOM   6539  N   ARG D 212     -28.501 -68.538 134.512  1.00 42.56           N
ATOM   6540  CA  ARG D 212     -29.339 -69.369 133.655  1.00 44.17           C
ATOM   6541  C   ARG D 212     -30.389 -70.120 134.460  1.00 52.65           C
ATOM   6542  O   ARG D 212     -30.117 -70.632 135.550  1.00 57.95           O
ATOM   6543  CB  ARG D 212     -28.497 -70.365 132.870  1.00 44.08           C
ATOM   6544  CG  ARG D 212     -29.313 -71.160 131.848  1.00 39.96           C
ATOM   6545  CD  ARG D 212     -28.430 -72.092 131.021  1.00 41.55           C
ATOM   6546  NE  ARG D 212     -27.681 -72.968 131.906  1.00 52.14           N
ATOM   6547  CZ  ARG D 212     -28.121 -74.138 132.360  1.00 52.75           C
ATOM   6548  NH1 ARG D 212     -29.310 -74.604 131.997  1.00 55.42           N1+
ATOM   6549  NH2 ARG D 212     -27.365 -74.845 133.177  1.00 50.00           N
ATOM   6550  N   GLY D 213     -31.606 -70.150 133.923  1.00 55.21           N
ATOM   6551  CA  GLY D 213     -32.710 -70.874 134.521  1.00 61.80           C
ATOM   6552  C   GLY D 213     -33.365 -70.219 135.716  1.00 69.36           C
ATOM   6553  O   GLY D 213     -34.221 -70.844 136.350  1.00 78.60           O
ATOM   6554  N   GLU D 214     -33.041 -68.971 136.017  1.00 64.22           N
ATOM   6555  CA  GLU D 214     -33.596 -68.288 137.169  1.00 66.25           C
ATOM   6556  C   GLU D 214     -34.131 -66.936 136.710  1.00 74.30           C
ATOM   6557  O   GLU D 214     -34.125 -66.617 135.517  1.00 72.76           O
ATOM   6558  CB  GLU D 214     -32.534 -68.153 138.268  1.00 68.20           C
ATOM   6559  CG  GLU D 214     -31.925 -69.500 138.684  1.00 65.48           C
ATOM   6560  CD  GLU D 214     -30.610 -69.356 139.461  1.00 70.80           C
ATOM   6561  OE1 GLU D 214     -30.431 -68.335 140.180  1.00 56.20           O
ATOM   6562  OE2 GLU D 214     -29.760 -70.276 139.354  1.00 68.01           O1-
ATOM   6563  N   CYS D 215     -34.599 -66.138 137.668  1.00 83.83           N
ATOM   6564  CA  CYS D 215     -35.238 -64.844 137.389  1.00 81.44           C
ATOM   6565  C   CYS D 215     -34.229 -63.723 137.129  1.00 91.66           C
ATOM   6566  O   CYS D 215     -33.764 -63.051 138.055  1.00 89.67           O
ATOM   6567  CB  CYS D 215     -36.153 -64.449 138.554  1.00 87.78           C
ATOM   6568  SG  CYS D 215     -35.402 -64.679 140.202  1.00106.03           S
TER
ATOM   6569  N   THR I 152     -42.630 -26.640  53.407  1.00 78.60     D000  N
ATOM   6570  CA  THR I 152     -41.299 -26.066  53.631  1.00 90.61     D000  C
ATOM   6571  C   THR I 152     -41.155 -25.639  55.096  1.00 90.48     D000  C
ATOM   6572  O   THR I 152     -40.054 -25.653  55.660  1.00 85.79     D000  O
ATOM   6573  CB  THR I 152     -41.001 -24.845  52.694  1.00 84.39     D000  C
ATOM   6574  OG1 THR I 152     -42.169 -24.027  52.567  1.00 87.95     D000  O
ATOM   6575  CG2 THR I 152     -40.569 -25.297  51.311  1.00 74.94     D000  C
ATOM   6576  N   CYS I 153     -42.275 -25.254  55.707  1.00 89.21     D000  N
ATOM   6577  CA  CYS I 153     -42.264 -24.807  57.092  1.00 81.53     D000  C
ATOM   6578  C   CYS I 153     -43.556 -25.234  57.776  1.00 76.72     D000  C
ATOM   6579  O   CYS I 153     -44.575 -25.496  57.128  1.00 77.13     D000  O
ATOM   6580  CB  CYS I 153     -42.087 -23.284  57.178  1.00 79.48     D000  C
ATOM   6581  SG  CYS I 153     -40.357 -22.736  57.167  1.00 91.46     D000  S
ATOM   6582  N   CYS I 154     -43.494 -25.316  59.112  1.00 68.58     D000  N
ATOM   6583  CA  CYS I 154     -44.632 -25.662  59.947  1.00 56.00     D000  C
ATOM   6584  C   CYS I 154     -45.352 -24.407  60.411  1.00 54.60     D000  C
ATOM   6585  O   CYS I 154     -44.726 -23.355  60.580  1.00 53.80     D000  O
ATOM   6586  CB  CYS I 154     -44.181 -26.474  61.153  1.00 48.63     D000  C
ATOM   6587  SG  CYS I 154     -43.645 -28.112  60.680  1.00 63.81     D000  S
ATOM   6588  N   PRO I 155     -46.667 -24.480  60.621  1.00 47.88     D000  N
ATOM   6589  CA  PRO I 155     -47.397 -23.284  61.039  1.00 41.43     D000  C
ATOM   6590  C   PRO I 155     -46.878 -22.765  62.371  1.00 47.89     D000  C
ATOM   6591  O   PRO I 155     -46.206 -23.471  63.130  1.00 43.34     D000  O
ATOM   6592  CB  PRO I 155     -48.855 -23.757  61.137  1.00 48.53     D000  C
ATOM   6593  CG  PRO I 155     -48.818 -25.245  61.066  1.00 40.42     D000  C
ATOM   6594  CD  PRO I 155     -47.558 -25.631  60.385  1.00 47.80     D000  C
ATOM   6595  N   VAL I 156     -47.223 -21.503  62.648  1.00 50.69     D000  N
ATOM   6596  CA  VAL I 156     -46.805 -20.823  63.868  1.00 49.74     D000  C
ATOM   6597  C   VAL I 156     -47.161 -21.667  65.084  1.00 50.63     D000  C
```

```
ATOM    6598  O    VAL I 156    -48.294 -22.142  65.215  1.00 49.76    D000 O
ATOM    6599  CB   VAL I 156    -47.471 -19.435  63.931  1.00 56.06    D000 C
ATOM    6600  CG1  VAL I 156    -46.956 -18.628  65.112  1.00 48.56    D000 C
ATOM    6601  CG2  VAL I 156    -47.275 -18.685  62.605  1.00 59.05    D000 C
ATOM    6602  N    ASN I 157    -46.178 -21.872  65.968  1.00 51.11    D000 N
ATOM    6603  CA   ASN I 157    -46.292 -22.645  67.205  1.00 44.19    D000 C
ATOM    6604  C    ASN I 157    -46.372 -24.144  66.981  1.00 40.81    D000 C
ATOM    6605  O    ASN I 157    -46.737 -24.864  67.902  1.00 42.83    D000 O
ATOM    6606  CB   ASN I 157    -47.464 -22.193  68.081  1.00 45.04    D000 C
ATOM    6607  CG   ASN I 157    -47.278 -20.791  68.601  1.00 50.02    D000 C
ATOM    6608  OD1  ASN I 157    -46.197 -20.437  69.063  1.00 54.69    D000 O
ATOM    6609  ND2  ASN I 157    -48.315 -19.979  68.513  1.00 59.66    D000 N
ATOM    6610  N    TRP I 158    -46.085 -24.633  65.778  1.00 40.56    D000 N
ATOM    6611  CA   TRP I 158    -45.839 -26.051  65.566  1.00 38.45    D000 C
ATOM    6612  C    TRP I 158    -44.345 -26.293  65.371  1.00 40.31    D000 C
ATOM    6613  O    TRP I 158    -43.584 -25.397  65.007  1.00 39.61    D000 O
ATOM    6614  CB   TRP I 158    -46.629 -26.590  64.371  1.00 40.79    D000 C
ATOM    6615  CG   TRP I 158    -48.118 -26.566  64.574  1.00 40.96    D000 C
ATOM    6616  CD1  TRP I 158    -48.892 -25.473  64.883  1.00 39.11    D000 C
ATOM    6617  CD2  TRP I 158    -49.025 -27.667  64.427  1.00 36.12    D000 C
ATOM    6618  NE1  TRP I 158    -50.213 -25.840  64.971  1.00 35.50    D000 N
ATOM    6619  CE2  TRP I 158    -50.325 -27.175  64.688  1.00 32.39    D000 C
ATOM    6620  CE3  TRP I 158    -48.867 -29.017  64.101  1.00 36.76    D000 C
ATOM    6621  CZ2  TRP I 158    -51.453 -27.985  64.636  1.00 33.44    D000 C
ATOM    6622  CZ3  TRP I 158    -49.993 -29.826  64.048  1.00 36.91    D000 C
ATOM    6623  CH2  TRP I 158    -51.271 -29.308  64.321  1.00 34.08    D000 C
ATOM    6624  N    VAL I 159    -43.939 -27.531  65.615  1.00 42.08    D000 N
ATOM    6625  CA   VAL I 159    -42.544 -27.931  65.687  1.00 36.72    D000 C
ATOM    6626  C    VAL I 159    -42.284 -28.942  64.582  1.00 40.42    D000 C
ATOM    6627  O    VAL I 159    -42.934 -29.992  64.529  1.00 34.21    D000 O
ATOM    6628  CB   VAL I 159    -42.222 -28.551  67.060  1.00 40.12    D000 C
ATOM    6629  CG1  VAL I 159    -40.793 -29.037  67.110  1.00 39.21    D000 C
ATOM    6630  CG2  VAL I 159    -42.486 -27.563  68.171  1.00 43.22    D000 C
ATOM    6631  N    GLU I 160    -41.300 -28.660  63.739  1.00 45.25    D000 N
ATOM    6632  CA   GLU I 160    -40.975 -29.588  62.671  1.00 43.30    D000 C
ATOM    6633  C    GLU I 160    -40.058 -30.686  63.184  1.00 41.09    D000 C
ATOM    6634  O    GLU I 160    -39.201 -30.447  64.036  1.00 44.54    D000 O
ATOM    6635  CB   GLU I 160    -40.309 -28.861  61.510  1.00 49.23    D000 C
ATOM    6636  CG   GLU I 160    -40.396 -29.626  60.191  1.00 61.45    D000 C
ATOM    6637  CD   GLU I 160    -39.556 -28.996  59.095  1.00 74.24    D000 C
ATOM    6638  OE1  GLU I 160    -40.073 -28.128  58.350  1.00 75.44    D000 O
ATOM    6639  OE2  GLU I 160    -38.365 -29.367  58.996  1.00 82.31    D000 O1-
ATOM    6640  N    HIS I 161    -40.262 -31.899  62.675  1.00 34.25    D000 N
ATOM    6641  CA   HIS I 161    -39.358 -33.005  62.927  1.00 35.38    D000 C
ATOM    6642  C    HIS I 161    -39.634 -34.146  61.950  1.00 44.04    D000 C
ATOM    6643  O    HIS I 161    -40.655 -34.832  62.063  1.00 44.75    D000 O
ATOM    6644  CB   HIS I 161    -39.491 -33.493  64.372  1.00 34.41    D000 C
ATOM    6645  CG   HIS I 161    -38.662 -34.709  64.679  1.00 42.31    D000 C
ATOM    6646  ND1  HIS I 161    -37.356 -34.632  65.127  1.00 39.49    D000 N
ATOM    6647  CD2  HIS I 161    -38.947 -36.031  64.578  1.00 37.68    D000 C
ATOM    6648  CE1  HIS I 161    -36.884 -35.854  65.308  1.00 36.84    D000 C
ATOM    6649  NE2  HIS I 161    -37.823 -36.720  64.970  1.00 37.93    D000 N
ATOM    6650  N    GLU I 162    -38.726 -34.358  60.992  1.00 48.09    D000 N
ATOM    6651  CA   GLU I 162    -38.750 -35.513  60.090  1.00 42.19    D000 C
ATOM    6652  C    GLU I 162    -39.999 -35.567  59.225  1.00 46.13    D000 C
ATOM    6653  O    GLU I 162    -40.676 -36.597  59.149  1.00 46.71    D000 O
ATOM    6654  CB   GLU I 162    -38.576 -36.822  60.852  1.00 38.57    D000 C
ATOM    6655  CG   GLU I 162    -37.232 -36.891  61.474  1.00 49.45    D000 C
ATOM    6656  CD   GLU I 162    -36.178 -37.369  60.479  1.00 67.10    D000 C
ATOM    6657  OE1  GLU I 162    -36.210 -38.565  60.098  1.00 74.81    D000 O
ATOM    6658  OE2  GLU I 162    -35.341 -36.533  60.050  1.00 59.37    D000 O1-
ATOM    6659  N    ARG I 163    -40.289 -34.453  58.547  1.00 48.14    D000 N
ATOM    6660  CA   ARG I 163    -41.446 -34.362  57.648  1.00 57.33    D000 C
ATOM    6661  C    ARG I 163    -42.768 -34.545  58.387  1.00 53.34    D000 C
ATOM    6662  O    ARG I 163    -43.712 -35.129  57.854  1.00 56.97    D000 O
ATOM    6663  CB   ARG I 163    -41.335 -35.388  56.509  1.00 65.00    D000 C
ATOM    6664  CG   ARG I 163    -40.188 -35.176  55.528  1.00 68.66    D000 C
ATOM    6665  CD   ARG I 163    -40.271 -36.202  54.398  1.00 80.19    D000 C
ATOM    6666  NE   ARG I 163    -41.472 -36.074  53.577  1.00 89.56    D000 N
ATOM    6667  CZ   ARG I 163    -41.971 -37.066  52.841  1.00 92.42    D000 C
ATOM    6668  NH1  ARG I 163    -43.077 -36.881  52.123  1.00 78.10    D000 N1+
```

```
ATOM   6669  NH2 ARG I 163      -41.362 -38.249  52.832  1.00 94.56      D000 N
ATOM   6670  N   SER I 164      -42.822 -34.104  59.641  1.00 53.57      D000 N
ATOM   6671  CA  SER I 164      -44.059 -33.988  60.400  1.00 46.49      D000 C
ATOM   6672  C   SER I 164      -44.026 -32.697  61.203  1.00 51.04      D000 C
ATOM   6673  O   SER I 164      -42.965 -32.279  61.679  1.00 50.06      D000 O
ATOM   6674  CB  SER I 164      -44.270 -35.170  61.343  1.00 43.89      D000 C
ATOM   6675  OG  SER I 164      -44.907 -36.249  60.692  1.00 50.01      D000 O
ATOM   6676  N   CYS I 165      -45.194 -32.075  61.357  1.00 50.05      D000 N
ATOM   6677  CA  CYS I 165      -45.370 -30.919  62.233  1.00 47.87      D000 C
ATOM   6678  C   CYS I 165      -46.135 -31.340  63.489  1.00 43.11      D000 C
ATOM   6679  O   CYS I 165      -47.190 -31.975  63.399  1.00 42.93      D000 O
ATOM   6680  CB  CYS I 165      -46.116 -29.778  61.524  1.00 50.43      D000 C
ATOM   6681  SG  CYS I 165      -45.326 -29.058  60.031  1.00 58.62      D000 S
ATOM   6682  N   TYR I 166      -45.622 -30.957  64.656  1.00 43.41      D000 N
ATOM   6683  CA  TYR I 166      -46.198 -31.350  65.934  1.00 35.17      D000 C
ATOM   6684  C   TYR I 166      -46.617 -30.116  66.719  1.00 36.72      D000 C
ATOM   6685  O   TYR I 166      -45.937 -29.084  66.683  1.00 35.81      D000 O
ATOM   6686  CB  TYR I 166      -45.214 -32.158  66.761  1.00 28.42      D000 C
ATOM   6687  CG  TYR I 166      -44.766 -33.439  66.120  1.00 35.36      D000 C
ATOM   6688  CD1 TYR I 166      -43.703 -33.461  65.194  1.00 40.74      D000 C
ATOM   6689  CD2 TYR I 166      -45.379 -34.629  66.435  1.00 28.15      D000 C
ATOM   6690  CE1 TYR I 166      -43.280 -34.646  64.614  1.00 31.55      D000 C
ATOM   6691  CE2 TYR I 166      -44.966 -35.813  65.864  1.00 35.33      D000 C
ATOM   6692  CZ  TYR I 166      -43.926 -35.818  64.952  1.00 36.07      D000 C
ATOM   6693  OH  TYR I 166      -43.557 -37.022  64.406  1.00 37.06      D000 O
ATOM   6694  N   TRP I 167      -47.735 -30.235  67.438  1.00 33.78      D000 N
ATOM   6695  CA  TRP I 167      -48.213 -29.191  68.335  1.00 33.91      D000 C
ATOM   6696  C   TRP I 167      -48.485 -29.823  69.692  1.00 33.58      D000 C
ATOM   6697  O   TRP I 167      -49.169 -30.848  69.776  1.00 37.11      D000 O
ATOM   6698  CB  TRP I 167      -49.478 -28.509  67.776  1.00 37.58      D000 C
ATOM   6699  CG  TRP I 167      -50.029 -27.432  68.678  1.00 36.19      D000 C
ATOM   6700  CD1 TRP I 167      -49.648 -26.122  68.725  1.00 37.64      D000 C
ATOM   6701  CD2 TRP I 167      -51.064 -27.580  69.657  1.00 33.12      D000 C
ATOM   6702  NE1 TRP I 167      -50.365 -25.452  69.692  1.00 32.45      D000 N
ATOM   6703  CE2 TRP I 167      -51.246 -26.325  70.269  1.00 31.52      D000 C
ATOM   6704  CE3 TRP I 167      -51.846 -28.659  70.083  1.00 35.72      D000 C
ATOM   6705  CZ2 TRP I 167      -52.183 -26.116  71.277  1.00 34.14      D000 C
ATOM   6706  CZ3 TRP I 167      -52.782 -28.447  71.087  1.00 32.27      D000 C
ATOM   6707  CH2 TRP I 167      -52.941 -27.185  71.669  1.00 31.51      D000 C
ATOM   6708  N   PHE I 168      -47.934 -29.223  70.748  1.00 35.27      D000 N
ATOM   6709  CA  PHE I 168      -47.963 -29.784  72.101  1.00 31.67      D000 C
ATOM   6710  C   PHE I 168      -48.837 -28.898  72.975  1.00 31.00      D000 C
ATOM   6711  O   PHE I 168      -48.468 -27.757  73.263  1.00 28.23      D000 O
ATOM   6712  CB  PHE I 168      -46.555 -29.881  72.685  1.00 28.79      D000 C
ATOM   6713  CG  PHE I 168      -45.609 -30.707  71.860  1.00 29.81      D000 C
ATOM   6714  CD1 PHE I 168      -45.461 -32.067  72.099  1.00 30.76      D000 C
ATOM   6715  CD2 PHE I 168      -44.881 -30.129  70.836  1.00 27.05      D000 C
ATOM   6716  CE1 PHE I 168      -44.587 -32.824  71.329  1.00 29.28      D000 C
ATOM   6717  CE2 PHE I 168      -44.009 -30.878  70.073  1.00 27.44      D000 C
ATOM   6718  CZ  PHE I 168      -43.869 -32.224  70.310  1.00 26.73      D000 C
ATOM   6719  N   SER I 169      -49.963 -29.437  73.440  1.00 31.45      D000 N
ATOM   6720  CA  SER I 169      -50.883 -28.643  74.245  1.00 31.26      D000 C
ATOM   6721  C   SER I 169      -50.266 -28.259  75.591  1.00 25.88      D000 C
ATOM   6722  O   SER I 169      -49.376 -28.932  76.115  1.00 23.51      D000 O
ATOM   6723  CB  SER I 169      -52.201 -29.404  74.471  1.00 30.30      D000 C
ATOM   6724  OG  SER I 169      -52.074 -30.442  75.438  1.00 28.82      D000 O
ATOM   6725  N   ARG I 170      -50.734 -27.135  76.129  1.00 27.95      D000 N
ATOM   6726  CA  ARG I 170      -50.447 -26.722  77.495  1.00 34.22      D000 C
ATOM   6727  C   ARG I 170      -51.671 -26.846  78.392  1.00 33.58      D000 C
ATOM   6728  O   ARG I 170      -51.627 -26.414  79.546  1.00 31.86      D000 O
ATOM   6729  CB  ARG I 170      -49.944 -25.282  77.529  1.00 25.51      D000 C
ATOM   6730  CG  ARG I 170      -48.455 -25.122  77.693  1.00 33.77      D000 C
ATOM   6731  CD  ARG I 170      -48.071 -25.130  79.130  1.00 32.63      D000 C
ATOM   6732  NE  ARG I 170      -47.020 -26.113  79.328  1.00 38.28      D000 N
ATOM   6733  CZ  ARG I 170      -46.005 -25.992  80.166  1.00 33.99      D000 C
ATOM   6734  NH1 ARG I 170      -45.904 -24.932  80.960  1.00 29.65      D000 N1+
ATOM   6735  NH2 ARG I 170      -45.123 -26.986  80.241  1.00 33.21      D000 N
ATOM   6736  N   SER I 171      -52.760 -27.420  77.888  1.00 27.31      D000 N
ATOM   6737  CA  SER I 171      -53.943 -27.684  78.682  1.00 24.95      D000 C
ATOM   6738  C   SER I 171      -54.303 -29.138  78.489  1.00 29.34      D000 C
ATOM   6739  O   SER I 171      -53.693 -29.847  77.682  1.00 31.49      D000 O
```

```
ATOM   6740  CB   SER I 171     -55.119 -26.815  78.277  1.00 21.97      D000 C
ATOM   6741  OG   SER I 171     -55.427 -27.085  76.932  1.00 35.69      D000 O
ATOM   6742  N    GLY I 172     -55.301 -29.577  79.252  1.00 29.63      D000 N
ATOM   6743  CA   GLY I 172     -55.700 -30.970  79.280  1.00 24.44      D000 C
ATOM   6744  C    GLY I 172     -57.075 -31.181  78.703  1.00 25.61      D000 C
ATOM   6745  O    GLY I 172     -57.864 -30.245  78.637  1.00 27.53      D000 O
ATOM   6746  N    LYS I 173     -57.363 -32.400  78.273  1.00 25.19      D000 N
ATOM   6747  CA   LYS I 173     -58.629 -32.726  77.650  1.00 27.09      D000 C
ATOM   6748  C    LYS I 173     -58.823 -34.231  77.752  1.00 33.35      D000 C
ATOM   6749  O    LYS I 173     -57.854 -34.995  77.672  1.00 29.52      D000 O
ATOM   6750  CB   LYS I 173     -58.693 -32.295  76.172  1.00 27.91      D000 C
ATOM   6751  CG   LYS I 173     -58.975 -30.805  75.865  1.00 25.52      D000 C
ATOM   6752  CD   LYS I 173     -59.510 -30.671  74.410  1.00 26.32      D000 C
ATOM   6753  CE   LYS I 173     -59.494 -29.257  73.861  1.00 19.84      D000 C
ATOM   6754  NZ   LYS I 173     -60.449 -28.327  74.536  1.00 27.70      D000 N1+
ATOM   6755  N    ALA I 174     -60.076 -34.643  77.956  1.00 29.60      D000 N
ATOM   6756  CA   ALA I 174     -60.433 -36.038  77.791  1.00 32.67      D000 C
ATOM   6757  C    ALA I 174     -60.003 -36.509  76.401  1.00 33.60      D000 C
ATOM   6758  O    ALA I 174     -59.996 -35.730  75.441  1.00 31.75      D000 O
ATOM   6759  CB   ALA I 174     -61.943 -36.202  77.997  1.00 28.75      D000 C
ATOM   6760  N    TRP I 175     -59.641 -37.796  76.289  1.00 30.30      D000 N
ATOM   6761  CA   TRP I 175     -59.069 -38.288  75.033  1.00 32.92      D000 C
ATOM   6762  C    TRP I 175     -59.946 -37.956  73.824  1.00 35.10      D000 C
ATOM   6763  O    TRP I 175     -59.441 -37.507  72.790  1.00 38.08      D000 O
ATOM   6764  CB   TRP I 175     -58.822 -39.795  75.102  1.00 33.10      D000 C
ATOM   6765  CG   TRP I 175     -58.056 -40.337  73.893  1.00 38.61      D000 C
ATOM   6766  CD1  TRP I 175     -56.729 -40.615  73.835  1.00 38.20      D000 C
ATOM   6767  CD2  TRP I 175     -58.584 -40.653  72.579  1.00 43.49      D000 C
ATOM   6768  NE1  TRP I 175     -56.388 -41.079  72.580  1.00 35.04      D000 N
ATOM   6769  CE2  TRP I 175     -57.505 -41.113  71.793  1.00 38.92      D000 C
ATOM   6770  CE3  TRP I 175     -59.863 -40.593  71.996  1.00 43.77      D000 C
ATOM   6771  CZ2  TRP I 175     -57.662 -41.517  70.456  1.00 44.27      D000 C
ATOM   6772  CZ3  TRP I 175     -60.016 -40.989  70.650  1.00 43.79      D000 C
ATOM   6773  CH2  TRP I 175     -58.925 -41.448  69.906  1.00 44.29      D000 C
ATOM   6774  N    ALA I 176     -61.260 -38.156  73.936  1.00 33.82      D000 N
ATOM   6775  CA   ALA I 176     -62.154 -37.894  72.815  1.00 28.11      D000 C
ATOM   6776  C    ALA I 176     -62.108 -36.432  72.394  1.00 37.58      D000 C
ATOM   6777  O    ALA I 176     -62.150 -36.111  71.196  1.00 36.74      D000 O
ATOM   6778  CB   ALA I 176     -63.578 -38.275  73.199  1.00 27.93      D000 C
ATOM   6779  N    ASP I 177     -62.043 -35.528  73.370  1.00 35.00      D000 N
ATOM   6780  CA   ASP I 177     -62.036 -34.117  73.044  1.00 32.37      D000 C
ATOM   6781  C    ASP I 177     -60.712 -33.703  72.435  1.00 34.46      D000 C
ATOM   6782  O    ASP I 177     -60.672 -32.790  71.591  1.00 33.36      D000 O
ATOM   6783  CB   ASP I 177     -62.355 -33.298  74.290  1.00 36.16      D000 C
ATOM   6784  CG   ASP I 177     -63.729 -33.623  74.876  1.00 40.76      D000 C
ATOM   6785  OD1  ASP I 177     -64.648 -33.982  74.097  1.00 35.33      D000 O
ATOM   6786  OD2  ASP I 177     -63.888 -33.508  76.122  1.00 45.92      D000 O1-
ATOM   6787  N    ALA I 178     -59.622 -34.340  72.869  1.00 33.13      D000 N
ATOM   6788  CA   ALA I 178     -58.325 -34.082  72.255  1.00 34.80      D000 C
ATOM   6789  C    ALA I 178     -58.313 -34.585  70.812  1.00 35.86      D000 C
ATOM   6790  O    ALA I 178     -57.887 -33.873  69.898  1.00 30.16      D000 O
ATOM   6791  CB   ALA I 178     -57.224 -34.734  73.085  1.00 29.11      D000 C
ATOM   6792  N    ASP I 179     -58.825 -35.803  70.601  1.00 38.39      D000 N
ATOM   6793  CA   ASP I 179     -59.085 -36.343  69.268  1.00 35.65      D000 C
ATOM   6794  C    ASP I 179     -59.826 -35.347  68.401  1.00 37.02      D000 C
ATOM   6795  O    ASP I 179     -59.397 -35.030  67.286  1.00 39.99      D000 O
ATOM   6796  CB   ASP I 179     -59.886 -37.642  69.413  1.00 41.79      D000 C
ATOM   6797  CG   ASP I 179     -60.149 -38.338  68.094  1.00 42.90      D000 C
ATOM   6798  OD1  ASP I 179     -59.371 -38.120  67.149  1.00 43.83      D000 O
ATOM   6799  OD2  ASP I 179     -61.096 -39.163  68.035  1.00 43.02      D000 O1-
ATOM   6800  N    ASN I 180     -60.934 -34.820  68.911  1.00 39.12      D000 N
ATOM   6801  CA   ASN I 180     -61.715 -33.882  68.117  1.00 39.76      D000 C
ATOM   6802  C    ASN I 180     -60.945 -32.601  67.851  1.00 32.43      D000 C
ATOM   6803  O    ASN I 180     -61.062 -32.026  66.768  1.00 38.63      D000 O
ATOM   6804  CB   ASN I 180     -63.053 -33.580  68.801  1.00 34.44      D000 C
ATOM   6805  CG   ASN I 180     -63.983 -34.773  68.793  1.00 38.43      D000 C
ATOM   6806  OD1  ASN I 180     -63.692 -35.795  68.162  1.00 43.96      D000 O
ATOM   6807  ND2  ASN I 180     -65.126 -34.642  69.458  1.00 38.70      D000 N
ATOM   6808  N    TYR I 181     -60.142 -32.144  68.814  1.00 37.95      D000 N
ATOM   6809  CA   TYR I 181     -59.388 -30.900  68.623  1.00 36.10      D000 C
ATOM   6810  C    TYR I 181     -58.390 -31.012  67.470  1.00 36.90      D000 C
```

```
ATOM   6811  O    TYR I 181    -58.240 -30.077  66.667  1.00 35.28      D000 O
ATOM   6812  CB   TYR I 181    -58.660 -30.519  69.908  1.00 25.56      D000 C
ATOM   6813  CG   TYR I 181    -57.826 -29.264  69.780  1.00 28.66      D000 C
ATOM   6814  CD1  TYR I 181    -56.520 -29.301  69.277  1.00 31.67      D000 C
ATOM   6815  CD2  TYR I 181    -58.336 -28.043  70.162  1.00 25.48      D000 C
ATOM   6816  CE1  TYR I 181    -55.768 -28.154  69.148  1.00 29.47      D000 C
ATOM   6817  CE2  TYR I 181    -57.579 -26.897  70.054  1.00 29.61      D000 C
ATOM   6818  CZ   TYR I 181    -56.299 -26.952  69.546  1.00 33.58      D000 C
ATOM   6819  OH   TYR I 181    -55.570 -25.786  69.431  1.00 36.52      D000 O
ATOM   6820  N    CYS I 182    -57.668 -32.125  67.395  1.00 30.45      D000 N
ATOM   6821  CA   CYS I 182    -56.669 -32.250  66.346  1.00 39.92      D000 C
ATOM   6822  C    CYS I 182    -57.323 -32.304  64.964  1.00 42.48      D000 C
ATOM   6823  O    CYS I 182    -56.879 -31.607  64.034  1.00 37.23      D000 O
ATOM   6824  CB   CYS I 182    -55.793 -33.477  66.614  1.00 37.71      D000 C
ATOM   6825  SG   CYS I 182    -54.685 -33.283  68.064  1.00 42.44      D000 S
ATOM   6826  N    ARG I 183    -58.413 -33.073  64.832  1.00 33.83      D000 N
ATOM   6827  CA   ARG I 183    -59.071 -33.213  63.538  1.00 34.20      D000 C
ATOM   6828  C    ARG I 183    -59.478 -31.860  62.965  1.00 40.37      D000 C
ATOM   6829  O    ARG I 183    -59.310 -31.617  61.764  1.00 43.30      D000 O
ATOM   6830  CB   ARG I 183    -60.275 -34.128  63.670  1.00 36.10      D000 C
ATOM   6831  CG   ARG I 183    -59.902 -35.562  63.991  1.00 34.06      D000 C
ATOM   6832  CD   ARG I 183    -61.081 -36.236  64.587  1.00 39.52      D000 C
ATOM   6833  NE   ARG I 183    -61.350 -37.531  63.997  1.00 57.45      D000 N
ATOM   6834  CZ   ARG I 183    -62.574 -37.960  63.698  1.00 68.60      D000 C
ATOM   6835  NH1  ARG I 183    -63.619 -37.172  63.920  1.00 55.03      D000 N1+
ATOM   6836  NH2  ARG I 183    -62.754 -39.165  63.159  1.00 76.79      D000 N
ATOM   6837  N    LEU I 184    -59.980 -30.952  63.805  1.00 34.08      D000 N
ATOM   6838  CA   LEU I 184    -60.347 -29.625  63.319  1.00 36.10      D000 C
ATOM   6839  C    LEU I 184    -59.159 -28.729  62.998  1.00 40.84      D000 C
ATOM   6840  O    LEU I 184    -59.369 -27.647  62.436  1.00 43.24      D000 O
ATOM   6841  CB   LEU I 184    -61.207 -28.901  64.344  1.00 42.44      D000 C
ATOM   6842  CG   LEU I 184    -62.705 -29.142  64.369  1.00 44.30      D000 C
ATOM   6843  CD1  LEU I 184    -62.982 -30.550  64.684  1.00 40.27      D000 C
ATOM   6844  CD2  LEU I 184    -63.246 -28.314  65.458  1.00 44.72      D000 C
ATOM   6845  N    GLU I 185    -57.938 -29.126  63.359  1.00 44.61      D000 N
ATOM   6846  CA   GLU I 185    -56.714 -28.453  62.938  1.00 42.87      D000 C
ATOM   6847  C    GLU I 185    -56.132 -29.068  61.664  1.00 45.96      D000 C
ATOM   6848  O    GLU I 185    -55.000 -28.744  61.284  1.00 41.44      D000 O
ATOM   6849  CB   GLU I 185    -55.678 -28.517  64.061  1.00 38.65      D000 C
ATOM   6850  CG   GLU I 185    -56.035 -27.729  65.294  1.00 42.71      D000 C
ATOM   6851  CD   GLU I 185    -55.905 -26.233  65.117  1.00 46.41      D000 C
ATOM   6852  OE1  GLU I 185    -54.995 -25.799  64.379  1.00 49.20      D000 O
ATOM   6853  OE2  GLU I 185    -56.701 -25.489  65.737  1.00 47.94      D000 O1-
ATOM   6854  N    ASP I 186    -56.902 -29.923  60.992  1.00 46.15      D000 N
ATOM   6855  CA   ASP I 186    -56.421 -30.765  59.904  1.00 46.32      D000 C
ATOM   6856  C    ASP I 186    -55.202 -31.570  60.344  1.00 45.43      D000 C
ATOM   6857  O    ASP I 186    -54.175 -31.625  59.670  1.00 47.14      D000 O
ATOM   6858  CB   ASP I 186    -56.136 -29.951  58.642  1.00 54.37      D000 C
ATOM   6859  CG   ASP I 186    -56.259 -30.795  57.356  1.00 66.46      D000 C
ATOM   6860  OD1  ASP I 186    -56.408 -32.045  57.464  1.00 61.88      D000 O
ATOM   6861  OD2  ASP I 186    -56.193 -30.206  56.242  1.00 69.65      D000 O1-
ATOM   6862  N    ALA I 187    -55.318 -32.197  61.505  1.00 42.36      D000 N
ATOM   6863  CA   ALA I 187    -54.205 -32.950  62.052  1.00 35.32      D000 C
ATOM   6864  C    ALA I 187    -54.783 -34.150  62.787  1.00 33.26      D000 C
ATOM   6865  O    ALA I 187    -55.966 -34.465  62.668  1.00 37.96      D000 O
ATOM   6866  CB   ALA I 187    -53.337 -32.044  62.933  1.00 32.84      D000 C
ATOM   6867  N    HIS I 188    -53.962 -34.816  63.567  1.00 30.88      D000 N
ATOM   6868  CA   HIS I 188    -54.456 -35.987  64.253  1.00 32.32      D000 C
ATOM   6869  C    HIS I 188    -53.593 -36.192  65.488  1.00 38.94      D000 C
ATOM   6870  O    HIS I 188    -52.452 -35.723  65.547  1.00 41.15      D000 O
ATOM   6871  CB   HIS I 188    -54.419 -37.199  63.331  1.00 28.57      D000 C
ATOM   6872  CG   HIS I 188    -53.039 -37.551  62.871  1.00 39.86      D000 C
ATOM   6873  ND1  HIS I 188    -52.317 -38.593  63.414  1.00 43.77      D000 N
ATOM   6874  CD2  HIS I 188    -52.238 -36.986  61.935  1.00 40.63      D000 C
ATOM   6875  CE1  HIS I 188    -51.136 -38.662  62.824  1.00 43.40      D000 C
ATOM   6876  NE2  HIS I 188    -51.063 -37.697  61.925  1.00 44.73      D000 N
ATOM   6877  N    LEU I 189    -54.155 -36.887  66.480  1.00 34.91      D000 N
ATOM   6878  CA   LEU I 189    -53.389 -37.240  67.667  1.00 36.57      D000 C
ATOM   6879  C    LEU I 189    -52.134 -38.003  67.269  1.00 36.22      D000 C
ATOM   6880  O    LEU I 189    -52.181 -38.892  66.414  1.00 36.87      D000 O
ATOM   6881  CB   LEU I 189    -54.227 -38.079  68.626  1.00 33.58      D000 C
```

```
ATOM   6882  CG   LEU I 189    -55.332 -37.326  69.361  1.00 32.53    D000 C
ATOM   6883  CD1  LEU I 189    -56.060 -38.310  70.251  1.00 31.98    D000 C
ATOM   6884  CD2  LEU I 189    -54.785 -36.156  70.127  1.00 32.11    D000 C
ATOM   6885  N    VAL I 190    -51.006 -37.639  67.889  1.00 34.36    D000 N
ATOM   6886  CA   VAL I 190    -49.706 -38.113  67.426  1.00 36.90    D000 C
ATOM   6887  C    VAL I 190    -49.705 -39.621  67.302  1.00 34.99    D000 C
ATOM   6888  O    VAL I 190    -50.277 -40.340  68.126  1.00 38.49    D000 O
ATOM   6889  CB   VAL I 190    -48.565 -37.658  68.355  1.00 40.36    D000 C
ATOM   6890  CG1  VAL I 190    -48.720 -38.264  69.755  1.00 36.50    D000 C
ATOM   6891  CG2  VAL I 190    -47.214 -38.026  67.743  1.00 36.89    D000 C
ATOM   6892  N    VAL I 191    -49.097 -40.091  66.224  1.00 38.67    D000 N
ATOM   6893  CA   VAL I 191    -48.933 -41.503  65.926  1.00 40.35    D000 C
ATOM   6894  C    VAL I 191    -47.436 -41.758  65.901  1.00 37.87    D000 C
ATOM   6895  O    VAL I 191    -46.719 -41.196  65.064  1.00 39.61    D000 O
ATOM   6896  CB   VAL I 191    -49.589 -41.882  64.586  1.00 42.11    D000 C
ATOM   6897  CG1  VAL I 191    -49.208 -43.319  64.164  1.00 36.05    D000 C
ATOM   6898  CG2  VAL I 191    -51.109 -41.663  64.648  1.00 38.64    D000 C
ATOM   6899  N    VAL I 192    -46.969 -42.599  66.805  1.00 36.01    D000 N
ATOM   6900  CA   VAL I 192    -45.545 -42.767  67.055  1.00 41.63    D000 C
ATOM   6901  C    VAL I 192    -45.088 -44.039  66.361  1.00 37.03    D000 C
ATOM   6902  O    VAL I 192    -45.528 -45.138  66.718  1.00 40.05    D000 O
ATOM   6903  CB   VAL I 192    -45.264 -42.808  68.565  1.00 40.06    D000 C
ATOM   6904  CG1  VAL I 192    -43.805 -43.021  68.828  1.00 43.89    D000 C
ATOM   6905  CG2  VAL I 192    -45.723 -41.516  69.199  1.00 30.23    D000 C
ATOM   6906  N    THR I 193    -44.188 -43.897  65.377  1.00 40.22    D000 N
ATOM   6907  CA   THR I 193    -43.828 -45.009  64.496  1.00 46.20    D000 C
ATOM   6908  C    THR I 193    -42.371 -45.451  64.595  1.00 48.77    D000 C
ATOM   6909  O    THR I 193    -42.009 -46.439  63.948  1.00 50.27    D000 O
ATOM   6910  CB   THR I 193    -44.081 -44.649  63.021  1.00 37.88    D000 C
ATOM   6911  OG1  THR I 193    -43.196 -43.588  62.655  1.00 46.69    D000 O
ATOM   6912  CG2  THR I 193    -45.516 -44.167  62.808  1.00 35.86    D000 C
ATOM   6913  N    SER I 194    -41.529 -44.758  65.364  1.00 47.04    D000 N
ATOM   6914  CA   SER I 194    -40.107 -45.072  65.443  1.00 47.61    D000 C
ATOM   6915  C    SER I 194    -39.578 -44.648  66.802  1.00 47.34    D000 C
ATOM   6916  O    SER I 194    -40.228 -43.898  67.531  1.00 48.44    D000 O
ATOM   6917  CB   SER I 194    -39.292 -44.369  64.357  1.00 44.10    D000 C
ATOM   6918  OG   SER I 194    -39.457 -42.964  64.442  1.00 48.66    D000 O
ATOM   6919  N    TRP I 195    -38.388 -45.147  67.143  1.00 44.89    D000 N
ATOM   6920  CA   TRP I 195    -37.764 -44.738  68.392  1.00 43.95    D000 C
ATOM   6921  C    TRP I 195    -37.376 -43.274  68.361  1.00 46.62    D000 C
ATOM   6922  O    TRP I 195    -37.488 -42.577  69.379  1.00 43.24    D000 O
ATOM   6923  CB   TRP I 195    -36.552 -45.605  68.717  1.00 48.92    D000 C
ATOM   6924  CG   TRP I 195    -36.802 -46.281  70.004  1.00 55.85    D000 C
ATOM   6925  CD1  TRP I 195    -37.056 -45.676  71.212  1.00 56.28    D000 C
ATOM   6926  CD2  TRP I 195    -36.901 -47.690  70.230  1.00 56.55    D000 C
ATOM   6927  NE1  TRP I 195    -37.292 -46.630  72.179  1.00 58.55    D000 N
ATOM   6928  CE2  TRP I 195    -37.199 -47.874  71.605  1.00 65.39    D000 C
ATOM   6929  CE3  TRP I 195    -36.755 -48.815  69.413  1.00 48.79    D000 C
ATOM   6930  CZ2  TRP I 195    -37.353 -49.140  72.174  1.00 63.33    D000 C
ATOM   6931  CZ3  TRP I 195    -36.904 -50.070  69.980  1.00 61.76    D000 C
ATOM   6932  CH2  TRP I 195    -37.198 -50.223  71.349  1.00 69.03    D000 C
ATOM   6933  N    GLU I 196    -36.914 -42.786  67.210  1.00 43.07    D000 N
ATOM   6934  CA   GLU I 196    -36.579 -41.374  67.119  1.00 38.64    D000 C
ATOM   6935  C    GLU I 196    -37.818 -40.526  67.331  1.00 38.66    D000 C
ATOM   6936  O    GLU I 196    -37.782 -39.540  68.074  1.00 41.67    D000 O
ATOM   6937  CB   GLU I 196    -35.919 -41.061  65.783  1.00 37.74    D000 C
ATOM   6938  CG   GLU I 196    -36.000 -42.187  64.776  1.00 52.87    D000 C
ATOM   6939  CD   GLU I 196    -35.141 -43.397  65.156  1.00 66.98    D000 C
ATOM   6940  OE1  GLU I 196    -35.717 -44.504  65.378  1.00 59.55    D000 O
ATOM   6941  OE2  GLU I 196    -33.899 -43.224  65.260  1.00 74.05    D000 O1-
ATOM   6942  N    GLU I 197    -38.945 -40.923  66.737  1.00 39.39    D000 N
ATOM   6943  CA   GLU I 197    -40.168 -40.155  66.948  1.00 42.64    D000 C
ATOM   6944  C    GLU I 197    -40.599 -40.215  68.410  1.00 37.64    D000 C
ATOM   6945  O    GLU I 197    -41.014 -39.205  68.981  1.00 32.92    D000 O
ATOM   6946  CB   GLU I 197    -41.283 -40.664  66.038  1.00 40.56    D000 C
ATOM   6947  CG   GLU I 197    -42.482 -39.748  66.042  1.00 37.27    D000 C
ATOM   6948  CD   GLU I 197    -43.515 -40.096  64.990  1.00 42.36    D000 C
ATOM   6949  OE1  GLU I 197    -43.544 -41.263  64.528  1.00 39.06    D000 O
ATOM   6950  OE2  GLU I 197    -44.277 -39.173  64.601  1.00 44.17    D000 O1-
ATOM   6951  N    GLN I 198    -40.485 -41.396  69.026  1.00 37.19    D000 N
ATOM   6952  CA   GLN I 198    -40.810 -41.562  70.435  1.00 33.73    D000 C
```

```
ATOM   6953  C    GLN I 198     -39.920 -40.689  71.301  1.00 39.61      D000 C
ATOM   6954  O    GLN I 198     -40.401 -39.995  72.206  1.00 35.04      D000 O
ATOM   6955  CB   GLN I 198     -40.659 -43.026  70.832  1.00 32.87      D000 C
ATOM   6956  CG   GLN I 198     -40.502 -43.235  72.323  1.00 37.28      D000 C
ATOM   6957  CD   GLN I 198     -41.830 -43.123  73.085  1.00 35.70      D000 C
ATOM   6958  OE1  GLN I 198     -42.902 -43.371  72.539  1.00 30.32      D000 O
ATOM   6959  NE2  GLN I 198     -41.749 -42.729  74.345  1.00 35.65      D000 N
ATOM   6960  N    LYS I 199     -38.610 -40.700  71.014  1.00 44.77      D000 N
ATOM   6961  CA   LYS I 199     -37.666 -39.882  71.765  1.00 39.06      D000 C
ATOM   6962  C    LYS I 199     -37.934 -38.405  71.551  1.00 32.39      D000 C
ATOM   6963  O    LYS I 199     -37.949 -37.629  72.509  1.00 31.60      D000 O
ATOM   6964  CB   LYS I 199     -36.232 -40.241  71.380  1.00 36.82      D000 C
ATOM   6965  CG   LYS I 199     -35.661 -41.318  72.265  1.00 38.84      D000 C
ATOM   6966  CD   LYS I 199     -34.660 -42.196  71.578  1.00 46.27      D000 C
ATOM   6967  CE   LYS I 199     -33.601 -41.398  70.901  1.00 41.73      D000 C
ATOM   6968  NZ   LYS I 199     -32.530 -42.355  70.568  1.00 49.72      D000 N1+
ATOM   6969  N    PHE I 200     -38.200 -38.011  70.309  1.00 34.30      D000 N
ATOM   6970  CA   PHE I 200     -38.496 -36.613  70.030  1.00 32.36      D000 C
ATOM   6971  C    PHE I 200     -39.712 -36.127  70.813  1.00 34.19      D000 C
ATOM   6972  O    PHE I 200     -39.724 -34.995  71.313  1.00 32.22      D000 O
ATOM   6973  CB   PHE I 200     -38.719 -36.408  68.539  1.00 28.30      D000 C
ATOM   6974  CG   PHE I 200     -39.389 -35.123  68.221  1.00 29.58      D000 C
ATOM   6975  CD1  PHE I 200     -38.661 -33.950  68.167  1.00 34.42      D000 C
ATOM   6976  CD2  PHE I 200     -40.750 -35.070  67.988  1.00 32.89      D000 C
ATOM   6977  CE1  PHE I 200     -39.278 -32.727  67.878  1.00 36.14      D000 C
ATOM   6978  CE2  PHE I 200     -41.373 -33.852  67.708  1.00 32.23      D000 C
ATOM   6979  CZ   PHE I 200     -40.628 -32.679  67.656  1.00 32.52      D000 C
ATOM   6980  N    VAL I 201     -40.766 -36.949  70.896  1.00 34.21      D000 N
ATOM   6981  CA   VAL I 201     -41.963 -36.512  71.604  1.00 32.09      D000 C
ATOM   6982  C    VAL I 201     -41.691 -36.409  73.104  1.00 28.47      D000 C
ATOM   6983  O    VAL I 201     -42.112 -35.445  73.745  1.00 29.67      D000 O
ATOM   6984  CB   VAL I 201     -43.166 -37.423  71.280  1.00 33.84      D000 C
ATOM   6985  CG1  VAL I 201     -44.371 -37.069  72.171  1.00 26.65      D000 C
ATOM   6986  CG2  VAL I 201     -43.564 -37.259  69.826  1.00 29.93      D000 C
ATOM   6987  N    GLN I 202     -40.948 -37.368  73.675  1.00 29.76      D000 N
ATOM   6988  CA   GLN I 202     -40.625 -37.333  75.103  1.00 27.89      D000 C
ATOM   6989  C    GLN I 202     -39.957 -36.027  75.500  1.00 30.88      D000 C
ATOM   6990  O    GLN I 202     -40.371 -35.375  76.462  1.00 33.51      D000 O
ATOM   6991  CB   GLN I 202     -39.692 -38.471  75.456  1.00 36.89      D000 C
ATOM   6992  CG   GLN I 202     -40.291 -39.826  75.569  1.00 42.55      D000 C
ATOM   6993  CD   GLN I 202     -39.269 -40.793  76.129  1.00 48.57      D000 C
ATOM   6994  OE1  GLN I 202     -39.196 -41.954  75.707  1.00 49.10      D000 O
ATOM   6995  NE2  GLN I 202     -38.440 -40.305  77.064  1.00 45.91      D000 N
ATOM   6996  N    HIS I 203     -38.934 -35.620  74.748  1.00 28.33      D000 N
ATOM   6997  CA   HIS I 203     -38.244 -34.367  74.998  1.00 28.20      D000 C
ATOM   6998  C    HIS I 203     -39.221 -33.223  75.160  1.00 32.96      D000 C
ATOM   6999  O    HIS I 203     -39.085 -32.395  76.066  1.00 38.66      D000 O
ATOM   7000  CB   HIS I 203     -37.308 -34.053  73.837  1.00 38.51      D000 C
ATOM   7001  CG   HIS I 203     -35.942 -34.622  73.991  1.00 45.00      D000 C
ATOM   7002  ND1  HIS I 203     -35.086 -34.222  74.994  1.00 43.86      D000 N
ATOM   7003  CD2  HIS I 203     -35.274 -35.544  73.260  1.00 44.53      D000 C
ATOM   7004  CE1  HIS I 203     -33.951 -34.887  74.882  1.00 48.63      D000 C
ATOM   7005  NE2  HIS I 203     -34.043 -35.701  73.843  1.00 52.83      D000 N
ATOM   7006  N    HIS I 204     -40.192 -33.140  74.253  1.00 33.22      D000 N
ATOM   7007  CA   HIS I 204     -41.092 -32.000  74.229  1.00 33.76      D000 C
ATOM   7008  C    HIS I 204     -42.207 -32.107  75.257  1.00 30.31      D000 C
ATOM   7009  O    HIS I 204     -42.651 -31.076  75.766  1.00 32.42      D000 O
ATOM   7010  CB   HIS I 204     -41.680 -31.825  72.836  1.00 34.09      D000 C
ATOM   7011  CG   HIS I 204     -40.730 -31.209  71.872  1.00 33.98      D000 C
ATOM   7012  ND1  HIS I 204     -40.541 -29.847  71.793  1.00 36.56      D000 N
ATOM   7013  CD2  HIS I 204     -39.894 -31.764  70.964  1.00 37.43      D000 C
ATOM   7014  CE1  HIS I 204     -39.636 -29.587  70.865  1.00 42.50      D000 C
ATOM   7015  NE2  HIS I 204     -39.224 -30.732  70.349  1.00 42.23      D000 N
ATOM   7016  N    ILE I 205     -42.658 -33.313  75.609  1.00 27.51      D000 N
ATOM   7017  CA   ILE I 205     -43.735 -33.386  76.587  1.00 30.86      D000 C
ATOM   7018  C    ILE I 205     -43.220 -33.433  78.017  1.00 29.89      D000 C
ATOM   7019  O    ILE I 205     -43.958 -33.056  78.934  1.00 35.65      D000 O
ATOM   7020  CB   ILE I 205     -44.686 -34.591  76.395  1.00 31.24      D000 C
ATOM   7021  CG1  ILE I 205     -43.981 -35.940  76.592  1.00 26.46      D000 C
ATOM   7022  CG2  ILE I 205     -45.357 -34.529  75.064  1.00 28.37      D000 C
ATOM   7023  CD1  ILE I 205     -44.943 -37.086  76.633  1.00 25.80      D000 C
```

```
ATOM   7024  N   GLY I 206   -41.978 -33.852  78.238  1.00 28.95   D000 N
ATOM   7025  CA  GLY I 206   -41.465 -33.985  79.584  1.00 29.61   D000 C
ATOM   7026  C   GLY I 206   -42.122 -35.118  80.345  1.00 27.57   D000 C
ATOM   7027  O   GLY I 206   -42.811 -35.956  79.766  1.00 29.14   D000 O
ATOM   7028  N   PRO I 207   -41.941 -35.153  81.711  1.00 29.38   D000 N
ATOM   7029  CA  PRO I 207   -42.433 -36.295  82.509  1.00 32.33   D000 C
ATOM   7030  C   PRO I 207   -43.904 -36.143  82.896  1.00 35.00   D000 C
ATOM   7031  O   PRO I 207   -44.262 -36.029  84.077  1.00 27.86   D000 O
ATOM   7032  CB  PRO I 207   -41.492 -36.271  83.719  1.00 29.81   D000 C
ATOM   7033  CG  PRO I 207   -41.194 -34.812  83.899  1.00 30.51   D000 C
ATOM   7034  CD  PRO I 207   -41.260 -34.150  82.549  1.00 24.29   D000 C
ATOM   7035  N   VAL I 208   -44.771 -36.126  81.879  1.00 31.78   D000 N
ATOM   7036  CA  VAL I 208   -46.167 -35.726  81.999  1.00 26.40   D000 C
ATOM   7037  C   VAL I 208   -47.040 -36.722  81.258  1.00 27.82   D000 C
ATOM   7038  O   VAL I 208   -46.764 -37.034  80.098  1.00 30.68   D000 O
ATOM   7039  CB  VAL I 208   -46.388 -34.323  81.425  1.00 25.92   D000 C
ATOM   7040  CG1 VAL I 208   -47.832 -33.981  81.495  1.00 28.17   D000 C
ATOM   7041  CG2 VAL I 208   -45.544 -33.320  82.172  1.00 26.96   D000 C
ATOM   7042  N   ASN I 209   -48.080 -37.232  81.927  1.00 27.70   D000 N
ATOM   7043  CA  ASN I 209   -49.054 -38.093  81.264  1.00 25.54   D000 C
ATOM   7044  C   ASN I 209   -49.688 -37.350  80.096  1.00 29.53   D000 C
ATOM   7045  O   ASN I 209   -50.263 -36.271  80.277  1.00 30.75   D000 O
ATOM   7046  CB  ASN I 209   -50.119 -38.560  82.251  1.00 24.84   D000 C
ATOM   7047  CG  ASN I 209   -49.588 -39.606  83.223  1.00 31.55   D000 C
ATOM   7048  OD1 ASN I 209   -48.636 -40.343  82.922  1.00 29.18   D000 O
ATOM   7049  ND2 ASN I 209   -50.193 -39.667  84.407  1.00 32.11   D000 N
ATOM   7050  N   THR I 210   -49.543 -37.905  78.889  1.00 28.06   D000 N
ATOM   7051  CA  THR I 210   -49.900 -37.194  77.666  1.00 29.54   D000 C
ATOM   7052  C   THR I 210   -50.593 -38.164  76.716  1.00 30.18   D000 C
ATOM   7053  O   THR I 210   -50.080 -39.257  76.474  1.00 31.78   D000 O
ATOM   7054  CB  THR I 210   -48.635 -36.568  77.017  1.00 32.46   D000 C
ATOM   7055  OG1 THR I 210   -48.068 -35.576  77.891  1.00 28.52   D000 O
ATOM   7056  CG2 THR I 210   -48.955 -35.895  75.680  1.00 32.03   D000 C
ATOM   7057  N   TRP I 211   -51.748 -37.771  76.178  1.00 28.21   D000 N
ATOM   7058  CA  TRP I 211   -52.484 -38.637  75.257  1.00 30.26   D000 C
ATOM   7059  C   TRP I 211   -51.777 -38.768  73.915  1.00 34.53   D000 C
ATOM   7060  O   TRP I 211   -51.243 -37.784  73.388  1.00 36.53   D000 O
ATOM   7061  CB  TRP I 211   -53.870 -38.078  74.986  1.00 27.00   D000 C
ATOM   7062  CG  TRP I 211   -54.837 -38.105  76.098  1.00 29.16   D000 C
ATOM   7063  CD1 TRP I 211   -55.575 -37.051  76.552  1.00 29.17   D000 C
ATOM   7064  CD2 TRP I 211   -55.232 -39.235  76.871  1.00 27.40   D000 C
ATOM   7065  NE1 TRP I 211   -56.401 -37.453  77.547  1.00 29.33   D000 N
ATOM   7066  CE2 TRP I 211   -56.208 -38.791  77.777  1.00 31.18   D000 C
ATOM   7067  CE3 TRP I 211   -54.849 -40.575  76.896  1.00 30.57   D000 C
ATOM   7068  CZ2 TRP I 211   -56.804 -39.643  78.721  1.00 33.48   D000 C
ATOM   7069  CZ3 TRP I 211   -55.442 -41.420  77.828  1.00 32.75   D000 C
ATOM   7070  CH2 TRP I 211   -56.414 -40.952  78.721  1.00 31.65   D000 C
ATOM   7071  N   MET I 212   -51.828 -39.969  73.332  1.00 30.71   D000 N
ATOM   7072  CA  MET I 212   -51.425 -40.202  71.945  1.00 35.06   D000 C
ATOM   7073  C   MET I 212   -52.588 -40.816  71.166  1.00 34.50   D000 C
ATOM   7074  O   MET I 212   -53.536 -41.339  71.746  1.00 29.90   D000 O
ATOM   7075  CB  MET I 212   -50.197 -41.126  71.870  1.00 33.98   D000 C
ATOM   7076  CG  MET I 212   -50.532 -42.612  72.009  1.00 34.65   D000 C
ATOM   7077  SD  MET I 212   -49.112 -43.701  72.320  1.00 36.95   D000 S
ATOM   7078  CE  MET I 212   -48.665 -43.185  73.977  1.00 32.76   D000 C
ATOM   7079  N   GLY I 213   -52.482 -40.824  69.836  1.00 31.69   D000 N
ATOM   7080  CA  GLY I 213   -53.575 -41.340  69.027  1.00 34.66   D000 C
ATOM   7081  C   GLY I 213   -53.764 -42.849  68.974  1.00 32.70   D000 C
ATOM   7082  O   GLY I 213   -53.862 -43.424  67.900  1.00 36.23   D000 O
ATOM   7083  N   LEU I 214   -53.871 -43.509  70.116  1.00 34.81   D000 N
ATOM   7084  CA  LEU I 214   -53.951 -44.961  70.150  1.00 39.32   D000 C
ATOM   7085  C   LEU I 214   -55.046 -45.365  71.132  1.00 45.03   D000 C
ATOM   7086  O   LEU I 214   -54.950 -45.073  72.327  1.00 45.18   D000 O
ATOM   7087  CB  LEU I 214   -52.589 -45.544  70.557  1.00 39.33   D000 C
ATOM   7088  CG  LEU I 214   -52.388 -47.049  70.722  1.00 44.57   D000 C
ATOM   7089  CD1 LEU I 214   -52.448 -47.717  69.369  1.00 42.58   D000 C
ATOM   7090  CD2 LEU I 214   -51.059 -47.335  71.406  1.00 38.76   D000 C
ATOM   7091  N   HIS I 215   -56.076 -46.053  70.639  1.00 50.99   D000 N
ATOM   7092  CA  HIS I 215   -57.202 -46.449  71.479  1.00 54.24   D000 C
ATOM   7093  C   HIS I 215   -57.809 -47.741  70.957  1.00 53.49   D000 C
ATOM   7094  O   HIS I 215   -57.726 -48.039  69.766  1.00 59.77   D000 O
```

492

```
ATOM   7095  CB  HIS I 215     -58.297 -45.381  71.525  1.00 51.69          D000 C
ATOM   7096  CG  HIS I 215     -58.991 -45.169  70.220  1.00 53.16          D000 C
ATOM   7097  ND1 HIS I 215     -60.319 -44.814  70.139  1.00 61.14          D000 N
ATOM   7098  CD2 HIS I 215     -58.542 -45.241  68.946  1.00 52.42          D000 C
ATOM   7099  CE1 HIS I 215     -60.664 -44.692  68.868  1.00 60.68          D000 C
ATOM   7100  NE2 HIS I 215     -59.602 -44.938  68.124  1.00 56.57          D000 N
ATOM   7101  N   ASP I 216     -58.446 -48.489  71.850  1.00 51.53          D000 N
ATOM   7102  CA  ASP I 216     -59.163 -49.708  71.488  1.00 63.11          D000 C
ATOM   7103  C   ASP I 216     -60.629 -49.629  71.897  1.00 70.32          D000 C
ATOM   7104  O   ASP I 216     -61.218 -50.615  72.345  1.00 73.44          D000 O
ATOM   7105  CB  ASP I 216     -58.504 -50.956  72.083  1.00 62.05          D000 C
ATOM   7106  CG  ASP I 216     -58.816 -51.157  73.565  1.00 63.11          D000 C
ATOM   7107  OD1 ASP I 216     -59.134 -50.177  74.265  1.00 66.02          D000 O
ATOM   7108  OD2 ASP I 216     -58.783 -52.313  74.025  1.00 71.37          D000 O1-
ATOM   7109  N   GLN I 217     -61.243 -48.448  71.761  1.00 68.81          D000 N
ATOM   7110  CA  GLN I 217     -62.674 -48.330  72.036  1.00 79.13          D000 C
ATOM   7111  C   GLN I 217     -63.497 -49.323  71.214  1.00 82.76          D000 C
ATOM   7112  O   GLN I 217     -64.637 -49.629  71.584  1.00 81.10          D000 O
ATOM   7113  CB  GLN I 217     -63.136 -46.885  71.804  1.00 78.37          D000 C
ATOM   7114  CG  GLN I 217     -63.047 -46.020  73.089  1.00 75.77          D000 C
ATOM   7115  CD  GLN I 217     -62.642 -44.570  72.825  1.00 67.80          D000 C
ATOM   7116  OE1 GLN I 217     -61.796 -44.296  71.969  1.00 62.96          D000 O
ATOM   7117  NE2 GLN I 217     -63.220 -43.637  73.591  1.00 56.15          D000 N
ATOM   7118  N   ASN I 218     -62.934 -49.838  70.118  1.00 81.51          D000 N
ATOM   7119  CA  ASN I 218     -63.546 -50.918  69.346  1.00 86.93          D000 C
ATOM   7120  C   ASN I 218     -63.369 -52.279  70.037  1.00 84.87          D000 C
ATOM   7121  O   ASN I 218     -64.349 -52.951  70.386  1.00 79.46          D000 O
ATOM   7122  CB  ASN I 218     -62.928 -50.932  67.942  1.00 92.18          D000 C
ATOM   7123  CG  ASN I 218     -63.905 -51.382  66.867  1.00 99.76          D000 C
ATOM   7124  OD1 ASN I 218     -63.626 -51.249  65.669  1.00 98.33          D000 O
ATOM   7125  ND2 ASN I 218     -65.059 -51.902  67.285  1.00 97.53          D000 N
ATOM   7126  N   GLY I 219     -62.117 -52.679  70.279  1.00 81.16          D000 N
ATOM   7127  CA  GLY I 219     -61.786 -54.004  70.763  1.00 72.74          D000 C
ATOM   7128  C   GLY I 219     -60.286 -54.248  70.697  1.00 71.27          D000 C
ATOM   7129  O   GLY I 219     -59.631 -54.504  71.715  1.00 64.68          D000 O
ATOM   7130  N   PRO I 220     -59.712 -54.165  69.493  1.00 71.97          D000 N
ATOM   7131  CA  PRO I 220     -58.254 -54.149  69.354  1.00 66.99          D000 C
ATOM   7132  C   PRO I 220     -57.718 -52.723  69.288  1.00 65.66          D000 C
ATOM   7133  O   PRO I 220     -58.435 -51.765  68.980  1.00 63.20          D000 O
ATOM   7134  CB  PRO I 220     -58.025 -54.861  68.014  1.00 60.23          D000 C
ATOM   7135  CG  PRO I 220     -59.311 -54.670  67.242  1.00 61.26          D000 C
ATOM   7136  CD  PRO I 220     -60.369 -54.114  68.172  1.00 66.08          D000 C
ATOM   7137  N   TRP I 221     -56.429 -52.596  69.586  1.00 55.14          D000 N
ATOM   7138  CA  TRP I 221     -55.793 -51.292  69.545  1.00 47.35          D000 C
ATOM   7139  C   TRP I 221     -55.622 -50.832  68.103  1.00 49.66          D000 C
ATOM   7140  O   TRP I 221     -55.267 -51.614  67.218  1.00 47.87          D000 O
ATOM   7141  CB  TRP I 221     -54.450 -51.345  70.262  1.00 52.11          D000 C
ATOM   7142  CG  TRP I 221     -54.577 -51.541  71.768  1.00 58.06          D000 C
ATOM   7143  CD1 TRP I 221     -54.489 -52.721  72.456  1.00 56.76          D000 C
ATOM   7144  CD2 TRP I 221     -54.817 -50.524  72.754  1.00 56.97          D000 C
ATOM   7145  NE1 TRP I 221     -54.650 -52.500  73.800  1.00 58.53          D000 N
ATOM   7146  CE2 TRP I 221     -54.860 -51.163  74.012  1.00 57.57          D000 C
ATOM   7147  CE3 TRP I 221     -54.999 -49.136  72.695  1.00 53.29          D000 C
ATOM   7148  CZ2 TRP I 221     -55.078 -50.464  75.201  1.00 53.89          D000 C
ATOM   7149  CZ3 TRP I 221     -55.211 -48.447  73.874  1.00 50.92          D000 C
ATOM   7150  CH2 TRP I 221     -55.251 -49.113  75.110  1.00 52.06          D000 C
ATOM   7151  N   LYS I 222     -55.888 -49.550  67.870  1.00 47.59          D000 N
ATOM   7152  CA  LYS I 222     -55.833 -48.951  66.546  1.00 47.00          D000 C
ATOM   7153  C   LYS I 222     -55.218 -47.565  66.654  1.00 47.01          D000 C
ATOM   7154  O   LYS I 222     -55.421 -46.869  67.650  1.00 48.52          D000 O
ATOM   7155  CB  LYS I 222     -57.240 -48.830  65.933  1.00 47.34          D000 C
ATOM   7156  CG  LYS I 222     -58.076 -50.090  66.018  1.00 46.82          D000 C
ATOM   7157  CD  LYS I 222     -59.483 -49.822  65.523  1.00 66.09          D000 C
ATOM   7158  CE  LYS I 222     -60.297 -51.108  65.437  1.00 78.89          D000 C
ATOM   7159  NZ  LYS I 222     -61.575 -50.950  64.682  1.00 75.65          D000 N1+
ATOM   7160  N   TRP I 223     -54.475 -47.155  65.630  1.00 40.12          D000 N
ATOM   7161  CA  TRP I 223     -54.033 -45.768  65.562  1.00 42.18          D000 C
ATOM   7162  C   TRP I 223     -55.057 -44.910  64.815  1.00 40.82          D000 C
ATOM   7163  O   TRP I 223     -55.674 -45.348  63.843  1.00 42.00          D000 O
ATOM   7164  CB  TRP I 223     -52.654 -45.651  64.892  1.00 40.05          D000 C
ATOM   7165  CG  TRP I 223     -51.497 -46.261  65.689  1.00 43.88          D000 C
```

```
ATOM   7166  CD1 TRP I 223     -50.994 -47.529  65.568  1.00 43.26      D000 C
ATOM   7167  CD2 TRP I 223     -50.711 -45.620  66.718  1.00 45.79      D000 C
ATOM   7168  NE1 TRP I 223     -49.959 -47.722  66.455  1.00 40.92      D000 N
ATOM   7169  CE2 TRP I 223     -49.761 -46.570  67.171  1.00 42.07      D000 C
ATOM   7170  CE3 TRP I 223     -50.730 -44.341  67.310  1.00 40.91      D000 C
ATOM   7171  CZ2 TRP I 223     -48.837 -46.284  68.182  1.00 38.74      D000 C
ATOM   7172  CZ3 TRP I 223     -49.809 -44.054  68.305  1.00 37.29      D000 C
ATOM   7173  CH2 TRP I 223     -48.872 -45.027  68.730  1.00 42.40      D000 C
ATOM   7174  N   VAL I 224     -55.158 -43.642  65.220  1.00 35.57      D000 N
ATOM   7175  CA  VAL I 224     -56.214 -42.782  64.710  1.00 38.56      D000 C
ATOM   7176  C   VAL I 224     -56.046 -42.420  63.229  1.00 44.84      D000 C
ATOM   7177  O   VAL I 224     -57.010 -41.965  62.593  1.00 41.50      D000 O
ATOM   7178  CB  VAL I 224     -56.317 -41.511  65.574  1.00 38.88      D000 C
ATOM   7179  CG1 VAL I 224     -56.643 -41.880  66.984  1.00 34.96      D000 C
ATOM   7180  CG2 VAL I 224     -55.044 -40.664  65.498  1.00 36.68      D000 C
ATOM   7181  N   ASP I 225     -54.854 -42.575  62.657  1.00 47.42      D000 N
ATOM   7182  CA  ASP I 225     -54.634 -42.202  61.264  1.00 45.07      D000 C
ATOM   7183  C   ASP I 225     -54.667 -43.405  60.323  1.00 47.81      D000 C
ATOM   7184  O   ASP I 225     -54.433 -43.248  59.117  1.00 47.18      D000 O
ATOM   7185  CB  ASP I 225     -53.316 -41.416  61.120  1.00 43.11      D000 C
ATOM   7186  CG  ASP I 225     -52.062 -42.297  61.130  1.00 45.43      D000 C
ATOM   7187  OD1 ASP I 225     -52.115 -43.489  61.519  1.00 41.39      D000 O
ATOM   7188  OD2 ASP I 225     -50.996 -41.765  60.728  1.00 46.94      D000 O1-
ATOM   7189  N   GLY I 226     -54.980 -44.593  60.847  1.00 47.45      D000 N
ATOM   7190  CA  GLY I 226     -55.065 -45.811  60.081  1.00 41.79      D000 C
ATOM   7191  C   GLY I 226     -53.833 -46.683  60.170  1.00 52.00      D000 C
ATOM   7192  O   GLY I 226     -53.912 -47.876  59.850  1.00 56.10      D000 O
ATOM   7193  N   THR I 227     -52.713 -46.128  60.647  1.00 51.85      D000 N
ATOM   7194  CA  THR I 227     -51.473 -46.886  60.769  1.00 46.63      D000 C
ATOM   7195  C   THR I 227     -51.717 -48.209  61.476  1.00 39.30      D000 C
ATOM   7196  O   THR I 227     -52.498 -48.293  62.422  1.00 45.35      D000 O
ATOM   7197  CB  THR I 227     -50.425 -46.078  61.535  1.00 41.84      D000 C
ATOM   7198  OG1 THR I 227     -50.287 -44.782  60.931  1.00 39.58      D000 O
ATOM   7199  CG2 THR I 227     -49.093 -46.792  61.502  1.00 32.58      D000 C
ATOM   7200  N   ASP I 228     -51.097 -49.259  60.965  1.00 42.37      D000 N
ATOM   7201  CA  ASP I 228     -51.312 -50.564  61.553  1.00 43.67      D000 C
ATOM   7202  C   ASP I 228     -50.643 -50.637  62.911  1.00 47.41      D000 C
ATOM   7203  O   ASP I 228     -49.510 -50.182  63.090  1.00 48.24      D000 O
ATOM   7204  CB  ASP I 228     -50.780 -51.670  60.657  1.00 44.10      D000 C
ATOM   7205  CG  ASP I 228     -50.886 -53.024  61.317  1.00 47.63      D000 C
ATOM   7206  OD1 ASP I 228     -52.011 -53.436  61.652  1.00 48.15      D000 O
ATOM   7207  OD2 ASP I 228     -49.842 -53.674  61.519  1.00 56.59      D000 O1-
ATOM   7208  N   TYR I 229     -51.362 -51.196  63.878  1.00 48.84      D000 N
ATOM   7209  CA  TYR I 229     -50.808 -51.333  65.220  1.00 52.99      D000 C
ATOM   7210  C   TYR I 229     -49.895 -52.557  65.345  1.00 52.23      D000 C
ATOM   7211  O   TYR I 229     -48.800 -52.471  65.919  1.00 51.49      D000 O
ATOM   7212  CB  TYR I 229     -51.949 -51.395  66.243  1.00 46.59      D000 C
ATOM   7213  CG  TYR I 229     -51.529 -51.806  67.625  1.00 44.81      D000 C
ATOM   7214  CD2 TYR I 229     -51.533 -53.141  67.992  1.00 47.32      D000 C
ATOM   7215  CD1 TYR I 229     -51.096 -50.875  68.549  1.00 47.52      D000 C
ATOM   7216  CE2 TYR I 229     -51.138 -53.545  69.242  1.00 50.06      D000 C
ATOM   7217  CE1 TYR I 229     -50.700 -51.268  69.830  1.00 49.62      D000 C
ATOM   7218  CZ  TYR I 229     -50.723 -52.613  70.161  1.00 51.78      D000 C
ATOM   7219  OH  TYR I 229     -50.341 -53.044  71.407  1.00 41.13      D000 O
ATOM   7220  N   GLU I 230     -50.333 -53.705  64.828  1.00 53.05      D000 N
ATOM   7221  CA  GLU I 230     -49.710 -54.967  65.221  1.00 54.20      D000 C
ATOM   7222  C   GLU I 230     -48.276 -55.102  64.708  1.00 52.15      D000 C
ATOM   7223  O   GLU I 230     -47.392 -55.578  65.430  1.00 54.02      D000 O
ATOM   7224  CB  GLU I 230     -50.577 -56.134  64.766  1.00 60.03      D000 C
ATOM   7225  CG  GLU I 230     -50.249 -57.386  65.539  1.00 67.01      D000 C
ATOM   7226  CD  GLU I 230     -50.036 -57.099  67.016  1.00 66.61      D000 C
ATOM   7227  OE1 GLU I 230     -51.027 -56.765  67.706  1.00 68.50      D000 O
ATOM   7228  OE2 GLU I 230     -48.871 -57.189  67.479  1.00 67.23      D000 O1-
ATOM   7229  N   THR I 231     -48.019 -54.721  63.470  1.00 52.10      D000 N
ATOM   7230  CA  THR I 231     -46.651 -54.762  62.968  1.00 54.05      D000 C
ATOM   7231  C   THR I 231     -45.864 -53.483  63.276  1.00 50.36      D000 C
ATOM   7232  O   THR I 231     -44.664 -53.426  62.977  1.00 52.95      D000 O
ATOM   7233  CB  THR I 231     -46.649 -55.055  61.450  1.00 52.18      D000 C
ATOM   7234  OG1 THR I 231     -47.303 -53.996  60.732  1.00 44.52      D000 O
ATOM   7235  CG2 THR I 231     -47.325 -56.388  61.140  1.00 44.56      D000 C
ATOM   7236  N   GLY I 232     -46.494 -52.478  63.907  1.00 46.91      D000 N
```

```
ATOM   7237  CA  GLY I 232    -45.878 -51.180  64.126  1.00 47.37      D000 C
ATOM   7238  C   GLY I 232    -45.126 -51.024  65.460  1.00 47.60      D000 C
ATOM   7239  O   GLY I 232    -45.082 -51.911  66.317  1.00 49.36      D000 O
ATOM   7240  N   PHE I 233    -44.496 -49.860  65.599  1.00 41.36      D000 N
ATOM   7241  CA  PHE I 233    -43.718 -49.535  66.789  1.00 43.90      D000 C
ATOM   7242  C   PHE I 233    -44.583 -49.581  68.043  1.00 44.53      D000 C
ATOM   7243  O   PHE I 233    -45.750 -49.180  68.019  1.00 48.88      D000 O
ATOM   7244  CB  PHE I 233    -43.083 -48.144  66.644  1.00 46.48      D000 C
ATOM   7245  CG  PHE I 233    -42.286 -47.729  67.846  1.00 53.61      D000 C
ATOM   7246  CD1 PHE I 233    -40.995 -48.137  68.026  1.00 46.82      D000 C
ATOM   7247  CD2 PHE I 233    -42.838 -46.911  68.820  1.00 52.29      D000 C
ATOM   7248  CE1 PHE I 233    -40.276 -47.855  69.139  1.00 48.20      D000 C
ATOM   7249  CE2 PHE I 233    -42.114 -46.558  69.940  1.00 45.86      D000 C
ATOM   7250  CZ  PHE I 233    -40.831 -47.029  70.096  1.00 50.22      D000 C
ATOM   7251  N   LYS I 234    -44.011 -50.083  69.143  1.00 41.34      D000 N
ATOM   7252  CA  LYS I 234    -44.704 -50.159  70.428  1.00 44.31      D000 C
ATOM   7253  C   LYS I 234    -43.739 -49.876  71.575  1.00 45.74      D000 C
ATOM   7254  O   LYS I 234    -42.561 -50.223  71.499  1.00 52.26      D000 O
ATOM   7255  CB  LYS I 234    -45.349 -51.532  70.647  1.00 50.12      D000 C
ATOM   7256  CG  LYS I 234    -46.423 -51.935  69.649  1.00 46.91      D000 C
ATOM   7257  CD  LYS I 234    -46.920 -53.333  69.980  1.00 41.34      D000 C
ATOM   7258  CE  LYS I 234    -47.495 -53.986  68.756  1.00 52.95      D000 C
ATOM   7259  NZ  LYS I 234    -46.449 -54.101  67.706  1.00 54.61      D000 N1+
ATOM   7260  N   ASN I 235    -44.239 -49.255  72.646  1.00 46.61      D000 N
ATOM   7261  CA  ASN I 235    -43.386 -48.905  73.785  1.00 40.27      D000 C
ATOM   7262  C   ASN I 235    -44.180 -48.890  75.098  1.00 44.39      D000 C
ATOM   7263  O   ASN I 235    -44.138 -47.925  75.865  1.00 45.23      D000 O
ATOM   7264  CB  ASN I 235    -42.691 -47.564  73.545  1.00 38.53      D000 C
ATOM   7265  CG  ASN I 235    -41.620 -47.270  74.577  1.00 40.11      D000 C
ATOM   7266  OD1 ASN I 235    -40.990 -48.178  75.105  1.00 44.27      D000 O
ATOM   7267  ND2 ASN I 235    -41.420 -46.000  74.879  1.00 41.29      D000 N
ATOM   7268  N   TRP I 236    -44.905 -49.971  75.387  1.00 43.99      D000 N
ATOM   7269  CA  TRP I 236    -45.720 -50.052  76.600  1.00 45.07      D000 C
ATOM   7270  C   TRP I 236    -44.866 -50.125  77.866  1.00 46.24      D000 C
ATOM   7271  O   TRP I 236    -43.739 -50.626  77.846  1.00 47.40      D000 O
ATOM   7272  CB  TRP I 236    -46.614 -51.289  76.550  1.00 44.75      D000 C
ATOM   7273  CG  TRP I 236    -47.647 -51.240  75.491  1.00 48.81      D000 C
ATOM   7274  CD1 TRP I 236    -47.616 -51.876  74.288  1.00 46.27      D000 C
ATOM   7275  CD2 TRP I 236    -48.878 -50.513  75.531  1.00 50.08      D000 C
ATOM   7276  NE1 TRP I 236    -48.749 -51.591  73.573  1.00 46.50      D000 N
ATOM   7277  CE2 TRP I 236    -49.543 -50.756  74.314  1.00 52.31      D000 C
ATOM   7278  CE3 TRP I 236    -49.482 -49.680  76.480  1.00 48.48      D000 C
ATOM   7279  CZ2 TRP I 236    -50.787 -50.195  74.018  1.00 53.44      D000 C
ATOM   7280  CZ3 TRP I 236    -50.708 -49.126  76.192  1.00 49.54      D000 C
ATOM   7281  CH2 TRP I 236    -51.354 -49.388  74.969  1.00 55.96      D000 C
ATOM   7282  N   ARG I 237    -45.410 -49.600  78.981  1.00 46.86      D000 N
ATOM   7283  CA  ARG I 237    -44.869 -49.924  80.304  1.00 52.81      D000 C
ATOM   7284  C   ARG I 237    -45.128 -51.400  80.593  1.00 57.44      D000 C
ATOM   7285  O   ARG I 237    -46.167 -51.926  80.193  1.00 65.84      D000 O
ATOM   7286  CB  ARG I 237    -45.507 -49.082  81.421  1.00 48.92      D000 C
ATOM   7287  CG  ARG I 237    -45.148 -47.579  81.479  1.00 52.62      D000 C
ATOM   7288  CD  ARG I 237    -43.916 -47.207  82.346  1.00 51.01      D000 C
ATOM   7289  NE  ARG I 237    -44.221 -47.118  83.786  1.00 55.72      D000 N
ATOM   7290  CZ  ARG I 237    -44.309 -45.995  84.513  1.00 52.55      D000 C
ATOM   7291  NH1 ARG I 237    -44.119 -44.796  83.979  1.00 44.43      D000 N1+
ATOM   7292  NH2 ARG I 237    -44.588 -46.073  85.808  1.00 64.23      D000 N
ATOM   7293  N   PRO I 238    -44.206 -52.094  81.259  1.00 64.01      D000 N
ATOM   7294  CA  PRO I 238    -44.450 -53.500  81.615  1.00 65.03      D000 C
ATOM   7295  C   PRO I 238    -45.771 -53.685  82.356  1.00 74.35      D000 C
ATOM   7296  O   PRO I 238    -46.138 -52.874  83.210  1.00 79.19      D000 O
ATOM   7297  CB  PRO I 238    -43.244 -53.846  82.493  1.00 66.34      D000 C
ATOM   7298  CG  PRO I 238    -42.143 -53.019  81.911  1.00 66.55      D000 C
ATOM   7299  CD  PRO I 238    -42.800 -51.710  81.488  1.00 66.32      D000 C
ATOM   7300  N   GLU I 239    -46.483 -54.772  82.011  1.00 76.56      D000 N
ATOM   7301  CA  GLU I 239    -47.850 -55.186  82.415  1.00 81.96      D000 C
ATOM   7302  C   GLU I 239    -48.960 -54.622  81.521  1.00 85.73      D000 C
ATOM   7303  O   GLU I 239    -50.134 -54.970  81.747  1.00 90.20      D000 O
ATOM   7304  CB  GLU I 239    -48.223 -54.802  83.865  1.00 84.76      D000 C
ATOM   7305  CG  GLU I 239    -48.026 -55.831  84.975  1.00 89.72      D000 C
ATOM   7306  CD  GLU I 239    -49.063 -55.648  86.104  1.00 97.46      D000 C
ATOM   7307  OE1 GLU I 239    -50.264 -55.495  85.787  1.00 95.00      D000 O
```

```
ATOM   7308  OE2 GLU I 239     -48.689 -55.645  87.301  1.00 99.60      D000 O1-
ATOM   7309  N   GLN I 240     -48.655 -53.809  80.513  1.00 78.77      D000 N
ATOM   7310  CA  GLN I 240     -49.716 -53.126  79.762  1.00 74.65      D000 C
ATOM   7311  C   GLN I 240     -49.744 -53.472  78.266  1.00 75.43      D000 C
ATOM   7312  O   GLN I 240     -48.690 -53.736  77.678  1.00 70.27      D000 O
ATOM   7313  CB  GLN I 240     -49.570 -51.610  79.956  1.00 69.99      D000 C
ATOM   7314  CG  GLN I 240     -49.627 -51.179  81.421  1.00 73.64      D000 C
ATOM   7315  CD  GLN I 240     -51.003 -51.423  82.047  1.00 79.48      D000 C
ATOM   7316  OE1 GLN I 240     -51.278 -52.503  82.587  1.00 78.83      D000 O
ATOM   7317  NE2 GLN I 240     -51.877 -50.413  81.968  1.00 68.96      D000 N
ATOM   7318  N   PRO I 241     -50.945 -53.443  77.634  1.00 74.07      D000 N
ATOM   7319  CA  PRO I 241     -52.250 -53.045  78.188  1.00 70.37      D000 C
ATOM   7320  C   PRO I 241     -52.840 -54.061  79.178  1.00 75.66      D000 C
ATOM   7321  O   PRO I 241     -53.904 -54.638  78.940  1.00 82.05      D000 O
ATOM   7322  CB  PRO I 241     -53.129 -52.936  76.941  1.00 70.97      D000 C
ATOM   7323  CG  PRO I 241     -52.543 -53.932  75.998  1.00 67.57      D000 C
ATOM   7324  CD  PRO I 241     -51.061 -53.841  76.215  1.00 64.21      D000 C
ATOM   7325  N   GLY I 252     -62.815 -49.790  79.716  1.00 70.62      D000 N
ATOM   7326  CA  GLY I 252     -62.424 -49.267  81.017  1.00 74.95      D000 C
ATOM   7327  C   GLY I 252     -61.035 -48.648  81.048  1.00 70.30      D000 C
ATOM   7328  O   GLY I 252     -60.698 -47.871  81.940  1.00 66.65      D000 O
ATOM   7329  N   GLU I 253     -60.214 -49.020  80.065  1.00 73.32      D000 N
ATOM   7330  CA  GLU I 253     -58.880 -48.472  79.814  1.00 64.01      D000 C
ATOM   7331  C   GLU I 253     -58.695 -48.525  78.298  1.00 60.63      D000 C
ATOM   7332  O   GLU I 253     -57.927 -49.303  77.737  1.00 61.66      D000 O
ATOM   7333  CB  GLU I 253     -57.775 -49.234  80.548  1.00 65.40      D000 C
ATOM   7334  CG  GLU I 253     -57.837 -49.139  82.057  1.00 69.16      D000 C
ATOM   7335  CD  GLU I 253     -57.007 -50.206  82.724  1.00 72.49      D000 C
ATOM   7336  OE1 GLU I 253     -55.760 -50.161  82.597  1.00 66.87      D000 O
ATOM   7337  OE2 GLU I 253     -57.610 -51.095  83.367  1.00 87.80      D000 O1-
ATOM   7338  N   ASP I 254     -59.422 -47.662  77.607  1.00 59.36      D000 N
ATOM   7339  CA  ASP I 254     -59.495 -47.720  76.163  1.00 59.89      D000 C
ATOM   7340  C   ASP I 254     -58.603 -46.688  75.471  1.00 54.78      D000 C
ATOM   7341  O   ASP I 254     -58.661 -46.575  74.247  1.00 56.31      D000 O
ATOM   7342  CB  ASP I 254     -60.957 -47.546  75.721  1.00 67.74      D000 C
ATOM   7343  CG  ASP I 254     -61.915 -48.563  76.378  1.00 69.29      D000 C
ATOM   7344  OD1 ASP I 254     -61.449 -49.599  76.909  1.00 61.14      D000 O
ATOM   7345  OD2 ASP I 254     -63.146 -48.308  76.361  1.00 69.38      D000 O1-
ATOM   7346  N   CYS I 255     -57.789 -45.925  76.210  1.00 47.78      D000 N
ATOM   7347  CA  CYS I 255     -56.967 -44.877  75.608  1.00 41.04      D000 C
ATOM   7348  C   CYS I 255     -55.530 -44.924  76.117  1.00 39.20      D000 C
ATOM   7349  O   CYS I 255     -55.295 -45.211  77.290  1.00 41.84      D000 O
ATOM   7350  CB  CYS I 255     -57.559 -43.510  75.866  1.00 41.88      D000 C
ATOM   7351  SG  CYS I 255     -59.123 -43.260  75.028  1.00 54.50      D000 S
ATOM   7352  N   ALA I 256     -54.577 -44.614  75.230  1.00 35.83      D000 N
ATOM   7353  CA  ALA I 256     -53.144 -44.757  75.487  1.00 36.99      D000 C
ATOM   7354  C   ALA I 256     -52.466 -43.414  75.756  1.00 31.83      D000 C
ATOM   7355  O   ALA I 256     -52.700 -42.427  75.047  1.00 30.75      D000 O
ATOM   7356  CB  ALA I 256     -52.445 -45.428  74.300  1.00 37.73      D000 C
ATOM   7357  N   HIS I 257     -51.584 -43.386  76.754  1.00 31.20      D000 N
ATOM   7358  CA  HIS I 257     -50.871 -42.153  77.060  1.00 31.95      D000 C
ATOM   7359  C   HIS I 257     -49.411 -42.453  77.347  1.00 31.16      D000 C
ATOM   7360  O   HIS I 257     -49.056 -43.554  77.782  1.00 30.03      D000 O
ATOM   7361  CB  HIS I 257     -51.523 -41.373  78.240  1.00 28.18      D000 C
ATOM   7362  CG  HIS I 257     -51.545 -42.119  79.546  1.00 35.85      D000 C
ATOM   7363  ND1 HIS I 257     -50.561 -41.985  80.501  1.00 32.60      D000 N
ATOM   7364  CD2 HIS I 257     -52.433 -43.010  80.051  1.00 37.79      D000 C
ATOM   7365  CE1 HIS I 257     -50.842 -42.756  81.535  1.00 33.70      D000 C
ATOM   7366  NE2 HIS I 257     -51.975 -43.385  81.290  1.00 38.87      D000 N
ATOM   7367  N   PHE I 258     -48.566 -41.458  77.069  1.00 32.31      D000 N
ATOM   7368  CA  PHE I 258     -47.195 -41.470  77.550  1.00 29.99      D000 C
ATOM   7369  C   PHE I 258     -47.186 -41.294  79.066  1.00 32.50      D000 C
ATOM   7370  O   PHE I 258     -48.064 -40.659  79.648  1.00 28.89      D000 O
ATOM   7371  CB  PHE I 258     -46.375 -40.346  76.926  1.00 31.38      D000 C
ATOM   7372  CG  PHE I 258     -46.400 -40.303  75.434  1.00 30.17      D000 C
ATOM   7373  CD1 PHE I 258     -45.501 -41.033  74.692  1.00 31.17      D000 C
ATOM   7374  CD2 PHE I 258     -47.305 -39.487  74.769  1.00 35.10      D000 C
ATOM   7375  CE1 PHE I 258     -45.522 -40.966  73.303  1.00 36.51      D000 C
ATOM   7376  CE2 PHE I 258     -47.330 -39.415  73.380  1.00 33.32      D000 C
ATOM   7377  CZ  PHE I 258     -46.440 -40.152  72.648  1.00 29.52      D000 C
ATOM   7378  N   THR I 259     -46.212 -41.900  79.709  1.00 33.00      D000 N
```

```
ATOM   7379  CA  THR I 259     -46.015 -41.713  81.132  1.00 34.18      D000 C
ATOM   7380  C   THR I 259     -44.809 -40.809  81.341  1.00 36.61      D000 C
ATOM   7381  O   THR I 259     -44.172 -40.339  80.389  1.00 38.23      D000 O
ATOM   7382  CB  THR I 259     -45.834 -43.049  81.856  1.00 36.60      D000 C
ATOM   7383  OG1 THR I 259     -44.598 -43.658  81.448  1.00 38.29      D000 O
ATOM   7384  CG2 THR I 259     -47.006 -43.966  81.583  1.00 32.69      D000 C
ATOM   7385  N   ASP I 260     -44.535 -40.524  82.610  1.00 36.88      D000 N
ATOM   7386  CA  ASP I 260     -43.424 -39.664  82.983  1.00 36.02      D000 C
ATOM   7387  C   ASP I 260     -42.065 -40.201  82.520  1.00 37.71      D000 C
ATOM   7388  O   ASP I 260     -41.091 -39.443  82.545  1.00 42.78      D000 O
ATOM   7389  CB  ASP I 260     -43.475 -39.440  84.501  1.00 38.98      D000 C
ATOM   7390  CG  ASP I 260     -43.369 -40.730  85.292  1.00 40.08      D000 C
ATOM   7391  OD1 ASP I 260     -43.567 -41.816  84.709  1.00 45.52      D000 O
ATOM   7392  OD2 ASP I 260     -43.171 -40.658  86.518  1.00 38.74      D000 O1-
ATOM   7393  N   ASP I 261     -41.964 -41.467  82.099  1.00 33.98      D000 N
ATOM   7394  CA  ASP I 261     -40.713 -42.008  81.579  1.00 35.49      D000 C
ATOM   7395  C   ASP I 261     -40.771 -42.320  80.090  1.00 41.58      D000 C
ATOM   7396  O   ASP I 261     -39.833 -42.910  79.550  1.00 41.61      D000 O
ATOM   7397  CB  ASP I 261     -40.289 -43.240  82.374  1.00 31.38      D000 C
ATOM   7398  CG  ASP I 261     -41.194 -44.433  82.150  1.00 44.50      D000 C
ATOM   7399  OD1 ASP I 261     -42.145 -44.348  81.338  1.00 49.19      D000 O
ATOM   7400  OD2 ASP I 261     -40.992 -45.454  82.843  1.00 43.56      D000 O1-
ATOM   7401  N   GLY I 262     -41.853 -41.954  79.414  1.00 43.44      D000 N
ATOM   7402  CA  GLY I 262     -41.983 -42.127  77.991  1.00 39.17      D000 C
ATOM   7403  C   GLY I 262     -42.711 -43.389  77.570  1.00 36.69      D000 C
ATOM   7404  O   GLY I 262     -43.387 -43.377  76.537  1.00 31.77      D000 O
ATOM   7405  N   ARG I 263     -42.625 -44.459  78.358  1.00 32.80      D000 N
ATOM   7406  CA  ARG I 263     -43.300 -45.692  77.987  1.00 40.54      D000 C
ATOM   7407  C   ARG I 263     -44.820 -45.549  78.138  1.00 42.34      D000 C
ATOM   7408  O   ARG I 263     -45.332 -44.731  78.905  1.00 38.87      D000 O
ATOM   7409  CB  ARG I 263     -42.773 -46.854  78.822  1.00 43.57      D000 C
ATOM   7410  CG  ARG I 263     -41.309 -47.117  78.559  1.00 42.00      D000 C
ATOM   7411  CD  ARG I 263     -40.789 -48.259  79.373  1.00 43.37      D000 C
ATOM   7412  NE  ARG I 263     -41.000 -48.089  80.802  1.00 47.00      D000 N
ATOM   7413  CZ  ARG I 263     -40.718 -49.024  81.706  1.00 54.61      D000 C
ATOM   7414  NH1 ARG I 263     -40.206 -50.187  81.325  1.00 56.14      D000 N1+
ATOM   7415  NH2 ARG I 263     -40.954 -48.806  82.995  1.00 57.34      D000 N
ATOM   7416  N   TRP I 264     -45.545 -46.362  77.390  1.00 41.85      D000 N
ATOM   7417  CA  TRP I 264     -46.964 -46.115  77.212  1.00 40.32      D000 C
ATOM   7418  C   TRP I 264     -47.801 -46.797  78.282  1.00 39.94      D000 C
ATOM   7419  O   TRP I 264     -47.377 -47.759  78.922  1.00 45.09      D000 O
ATOM   7420  CB  TRP I 264     -47.427 -46.610  75.851  1.00 41.81      D000 C
ATOM   7421  CG  TRP I 264     -46.709 -46.051  74.695  1.00 36.80      D000 C
ATOM   7422  CD1 TRP I 264     -45.834 -45.016  74.688  1.00 37.17      D000 C
ATOM   7423  CD2 TRP I 264     -46.807 -46.512  73.349  1.00 41.41      D000 C
ATOM   7424  NE1 TRP I 264     -45.377 -44.795  73.417  1.00 37.55      D000 N
ATOM   7425  CE2 TRP I 264     -45.957 -45.707  72.574  1.00 42.77      D000 C
ATOM   7426  CE3 TRP I 264     -47.530 -47.534  72.724  1.00 37.74      D000 C
ATOM   7427  CZ2 TRP I 264     -45.810 -45.888  71.205  1.00 40.50      D000 C
ATOM   7428  CZ3 TRP I 264     -47.378 -47.716  71.383  1.00 38.97      D000 C
ATOM   7429  CH2 TRP I 264     -46.536 -46.894  70.631  1.00 44.66      D000 C
ATOM   7430  N   ASN I 265     -49.029 -46.309  78.432  1.00 37.04      D000 N
ATOM   7431  CA  ASN I 265     -49.963 -46.923  79.356  1.00 37.46      D000 C
ATOM   7432  C   ASN I 265     -51.387 -46.554  78.963  1.00 37.95      D000 C
ATOM   7433  O   ASN I 265     -51.631 -45.512  78.353  1.00 36.28      D000 O
ATOM   7434  CB  ASN I 265     -49.655 -46.518  80.791  1.00 40.53      D000 C
ATOM   7435  CG  ASN I 265     -50.611 -47.123  81.765  1.00 49.10      D000 C
ATOM   7436  OD1 ASN I 265     -50.635 -48.337  81.939  1.00 51.66      D000 O
ATOM   7437  ND2 ASN I 265     -51.389 -46.281  82.440  1.00 49.67      D000 N
ATOM   7438  N   ASP I 266     -52.314 -47.446  79.291  1.00 45.60      D000 N
ATOM   7439  CA  ASP I 266     -53.738 -47.257  79.058  1.00 45.74      D000 C
ATOM   7440  C   ASP I 266     -54.403 -46.761  80.331  1.00 47.12      D000 C
ATOM   7441  O   ASP I 266     -54.070 -47.212  81.429  1.00 52.08      D000 O
ATOM   7442  CB  ASP I 266     -54.413 -48.561  78.615  1.00 52.43      D000 C
ATOM   7443  CG  ASP I 266     -54.095 -49.732  79.538  1.00 60.22      D000 C
ATOM   7444  OD1 ASP I 266     -52.905 -49.973  79.827  1.00 64.14      D000 O
ATOM   7445  OD2 ASP I 266     -55.035 -50.421  79.977  1.00 67.69      D000 O1-
ATOM   7446  N   ASP I 267     -55.347 -45.838  80.176  1.00 44.83      D000 N
ATOM   7447  CA  ASP I 267     -56.072 -45.253  81.295  1.00 49.89      D000 C
ATOM   7448  C   ASP I 267     -57.475 -44.917  80.805  1.00 46.26      D000 C
ATOM   7449  O   ASP I 267     -57.782 -45.069  79.623  1.00 47.12      D000 O
```

```
ATOM   7450  CB  ASP I 267     -55.339 -44.020  81.860  1.00 46.24      D000 C
ATOM   7451  CG  ASP I 267     -55.846 -43.631  83.228  1.00 48.24      D000 C
ATOM   7452  OD1 ASP I 267     -56.671 -44.391  83.773  1.00 56.95      D000 O
ATOM   7453  OD2 ASP I 267     -55.448 -42.573  83.754  1.00 49.05      D000 O1-
ATOM   7454  N   VAL I 268     -58.347 -44.502  81.727  1.00 41.94      D000 N
ATOM   7455  CA  VAL I 268     -59.707 -44.168  81.328  1.00 44.09      D000 C
ATOM   7456  C   VAL I 268     -59.676 -42.946  80.419  1.00 44.94      D000 C
ATOM   7457  O   VAL I 268     -58.896 -42.007  80.629  1.00 44.57      D000 O
ATOM   7458  CB  VAL I 268     -60.606 -43.939  82.554  1.00 40.01      D000 C
ATOM   7459  CG1 VAL I 268     -60.463 -45.079  83.506  1.00 42.61      D000 C
ATOM   7460  CG2 VAL I 268     -60.274 -42.634  83.242  1.00 40.82      D000 C
ATOM   7461  N   CYS I 269     -60.506 -42.962  79.379  1.00 38.74      D000 N
ATOM   7462  CA  CYS I 269     -60.422 -41.901  78.385  1.00 41.24      D000 C
ATOM   7463  C   CYS I 269     -60.908 -40.569  78.913  1.00 35.89      D000 C
ATOM   7464  O   CYS I 269     -60.800 -39.554  78.219  1.00 40.53      D000 O
ATOM   7465  CB  CYS I 269     -61.204 -42.298  77.127  1.00 43.34      D000 C
ATOM   7466  SG  CYS I 269     -60.582 -43.835  76.370  1.00 57.52      D000 S
ATOM   7467  N   GLN I 270     -61.422 -40.534  80.127  1.00 37.09      D000 N
ATOM   7468  CA  GLN I 270     -61.959 -39.299  80.666  1.00 35.74      D000 C
ATOM   7469  C   GLN I 270     -60.892 -38.406  81.284  1.00 33.91      D000 C
ATOM   7470  O   GLN I 270     -61.156 -37.213  81.465  1.00 36.79      D000 O
ATOM   7471  CB  GLN I 270     -63.029 -39.618  81.709  1.00 43.76      D000 C
ATOM   7472  CG  GLN I 270     -64.394 -39.929  81.095  1.00 52.98      D000 C
ATOM   7473  CD  GLN I 270     -64.451 -41.352  80.534  1.00 53.45      D000 C
ATOM   7474  OE1 GLN I 270     -63.849 -42.272  81.106  1.00 52.07      D000 O
ATOM   7475  NE2 GLN I 270     -65.156 -41.535  79.407  1.00 46.18      D000 N
ATOM   7476  N   ARG I 271     -59.698 -38.940  81.589  1.00 33.55      D000 N
ATOM   7477  CA  ARG I 271     -58.656 -38.139  82.228  1.00 34.17      D000 C
ATOM   7478  C   ARG I 271     -58.333 -36.927  81.364  1.00 35.86      D000 C
ATOM   7479  O   ARG I 271     -58.224 -37.058  80.136  1.00 34.42      D000 O
ATOM   7480  CB  ARG I 271     -57.380 -38.947  82.464  1.00 36.48      D000 C
ATOM   7481  CG  ARG I 271     -57.518 -40.084  83.447  1.00 39.60      D000 C
ATOM   7482  CD  ARG I 271     -57.065 -39.699  84.838  1.00 45.14      D000 C
ATOM   7483  NE  ARG I 271     -57.534 -40.662  85.844  1.00 59.73      D000 N
ATOM   7484  CZ  ARG I 271     -58.422 -40.401  86.810  1.00 58.63      D000 C
ATOM   7485  NH1 ARG I 271     -58.962 -39.185  86.947  1.00 54.22      D000 N1+
ATOM   7486  NH2 ARG I 271     -58.758 -41.360  87.660  1.00 55.45      D000 N
ATOM   7487  N   PRO I 272     -58.230 -35.749  81.936  1.00 32.00      D000 N
ATOM   7488  CA  PRO I 272     -57.855 -34.553  81.148  1.00 29.00      D000 C
ATOM   7489  C   PRO I 272     -56.342 -34.344  81.068  1.00 32.23      D000 C
ATOM   7490  O   PRO I 272     -55.758 -33.416  81.633  1.00 31.22      D000 O
ATOM   7491  CB  PRO I 272     -58.583 -33.433  81.892  1.00 26.05      D000 C
ATOM   7492  CG  PRO I 272     -58.775 -33.959  83.324  1.00 28.86      D000 C
ATOM   7493  CD  PRO I 272     -58.499 -35.435  83.347  1.00 27.78      D000 C
ATOM   7494  N   TYR I 273     -55.680 -35.225  80.325  1.00 32.52      D000 N
ATOM   7495  CA  TYR I 273     -54.251 -35.145  80.086  1.00 29.17      D000 C
ATOM   7496  C   TYR I 273     -53.931 -34.170  78.946  1.00 28.33      D000 C
ATOM   7497  O   TYR I 273     -54.790 -33.766  78.166  1.00 26.77      D000 O
ATOM   7498  CB  TYR I 273     -53.689 -36.524  79.748  1.00 26.56      D000 C
ATOM   7499  CG  TYR I 273     -53.687 -37.527  80.867  1.00 26.99      D000 C
ATOM   7500  CD1 TYR I 273     -53.588 -37.129  82.194  1.00 29.63      D000 C
ATOM   7501  CD2 TYR I 273     -53.757 -38.883  80.594  1.00 28.80      D000 C
ATOM   7502  CE1 TYR I 273     -53.582 -38.057  83.212  1.00 28.90      D000 C
ATOM   7503  CE2 TYR I 273     -53.737 -39.825  81.609  1.00 32.53      D000 C
ATOM   7504  CZ  TYR I 273     -53.656 -39.404  82.911  1.00 29.59      D000 C
ATOM   7505  OH  TYR I 273     -53.634 -40.338  83.910  1.00 29.82      D000 O
ATOM   7506  N   ARG I 274     -52.672 -33.768  78.882  1.00 28.32      D000 N
ATOM   7507  CA  ARG I 274     -52.212 -33.033  77.729  1.00 27.19      D000 C
ATOM   7508  C   ARG I 274     -52.169 -33.971  76.536  1.00 26.48      D000 C
ATOM   7509  O   ARG I 274     -52.280 -35.187  76.673  1.00 28.05      D000 O
ATOM   7510  CB  ARG I 274     -50.857 -32.403  78.011  1.00 24.95      D000 C
ATOM   7511  CG  ARG I 274     -50.926 -31.334  79.066  1.00 25.95      D000 C
ATOM   7512  CD  ARG I 274     -49.541 -30.802  79.382  1.00 28.18      D000 C
ATOM   7513  NE  ARG I 274     -49.548 -29.767  80.411  1.00 27.97      D000 N
ATOM   7514  CZ  ARG I 274     -48.464 -29.347  81.063  1.00 29.49      D000 C
ATOM   7515  NH1 ARG I 274     -47.274 -29.877  80.802  1.00 24.44      D000 N1+
ATOM   7516  NH2 ARG I 274     -48.567 -28.400  81.992  1.00 28.37      D000 N
ATOM   7517  N   TRP I 275     -52.038 -33.395  75.344  1.00 27.91      D000 N
ATOM   7518  CA  TRP I 275     -52.039 -34.203  74.138  1.00 28.85      D000 C
ATOM   7519  C   TRP I 275     -51.106 -33.568  73.105  1.00 30.78      D000 C
ATOM   7520  O   TRP I 275     -50.671 -32.410  73.241  1.00 27.47      D000 O
```

```
ATOM   7521  CB   TRP I 275     -53.473 -34.368  73.600  1.00 23.59      D000 C
ATOM   7522  CG   TRP I 275     -54.009 -33.123  72.979  1.00 26.26      D000 C
ATOM   7523  CD1  TRP I 275     -53.991 -32.808  71.648  1.00 26.24      D000 C
ATOM   7524  CD2  TRP I 275     -54.629 -32.005  73.643  1.00 27.84      D000 C
ATOM   7525  NE1  TRP I 275     -54.549 -31.571  71.442  1.00 27.63      D000 N
ATOM   7526  CE2  TRP I 275     -54.959 -31.059  72.646  1.00 29.00      D000 C
ATOM   7527  CE3  TRP I 275     -54.949 -31.718  74.971  1.00 27.22      D000 C
ATOM   7528  CZ2  TRP I 275     -55.589 -29.850  72.941  1.00 23.11      D000 C
ATOM   7529  CZ3  TRP I 275     -55.572 -30.511  75.258  1.00 26.94      D000 C
ATOM   7530  CH2  TRP I 275     -55.876 -29.595  74.248  1.00 27.13      D000 C
ATOM   7531  N    VAL I 276     -50.827 -34.338  72.051  1.00 26.77      D000 N
ATOM   7532  CA   VAL I 276     -49.950 -33.930  70.959  1.00 29.58      D000 C
ATOM   7533  C    VAL I 276     -50.723 -34.063  69.650  1.00 29.99      D000 C
ATOM   7534  O    VAL I 276     -51.233 -35.146  69.335  1.00 32.34      D000 O
ATOM   7535  CB   VAL I 276     -48.666 -34.790  70.916  1.00 34.01      D000 C
ATOM   7536  CG1  VAL I 276     -47.726 -34.358  69.768  1.00 25.34      D000 C
ATOM   7537  CG2  VAL I 276     -47.957 -34.821  72.259  1.00 22.27      D000 C
ATOM   7538  N    CYS I 277     -50.815 -32.973  68.892  1.00 30.05      D000 N
ATOM   7539  CA   CYS I 277     -51.289 -33.032  67.507  1.00 36.56      D000 C
ATOM   7540  C    CYS I 277     -50.113 -33.216  66.544  1.00 39.90      D000 C
ATOM   7541  O    CYS I 277     -49.037 -32.638  66.739  1.00 34.93      D000 O
ATOM   7542  CB   CYS I 277     -52.054 -31.764  67.111  1.00 33.98      D000 C
ATOM   7543  SG   CYS I 277     -53.711 -31.466  67.812  1.00 42.15      D000 S
ATOM   7544  N    GLU I 278     -50.336 -34.004  65.486  1.00 37.84      D000 N
ATOM   7545  CA   GLU I 278     -49.361 -34.207  64.419  1.00 38.01      D000 C
ATOM   7546  C    GLU I 278     -49.980 -33.930  63.054  1.00 40.54      D000 C
ATOM   7547  O    GLU I 278     -51.105 -34.354  62.766  1.00 41.44      D000 O
ATOM   7548  CB   GLU I 278     -48.801 -35.646  64.430  1.00 36.62      D000 C
ATOM   7549  CG   GLU I 278     -47.685 -35.926  63.414  1.00 36.43      D000 C
ATOM   7550  CD   GLU I 278     -47.249 -37.379  63.472  1.00 44.33      D000 C
ATOM   7551  OE1  GLU I 278     -47.852 -38.105  64.290  1.00 43.28      D000 O
ATOM   7552  OE2  GLU I 278     -46.341 -37.806  62.708  1.00 44.12      D000 O1-
ATOM   7553  N    THR I 279     -49.215 -33.280  62.187  1.00 40.86      D000 N
ATOM   7554  CA   THR I 279     -49.596 -33.153  60.789  1.00 45.51      D000 C
ATOM   7555  C    THR I 279     -48.349 -33.290  59.921  1.00 50.70      D000 C
ATOM   7556  O    THR I 279     -47.274 -32.791  60.269  1.00 52.15      D000 O
ATOM   7557  CB   THR I 279     -50.344 -31.830  60.515  1.00 40.68      D000 C
ATOM   7558  OG1  THR I 279     -50.995 -31.916  59.247  1.00 46.37      D000 O
ATOM   7559  CG2  THR I 279     -49.413 -30.622  60.530  1.00 40.76      D000 C
ATOM   7560  N    GLU I 280     -48.502 -33.993  58.801  1.00 51.69      D000 N
ATOM   7561  CA   GLU I 280     -47.419 -34.287  57.872  1.00 49.36      D000 C
ATOM   7562  C    GLU I 280     -47.271 -33.158  56.860  1.00 56.06      D000 C
ATOM   7563  O    GLU I 280     -48.253 -32.530  56.466  1.00 60.37      D000 O
ATOM   7564  CB   GLU I 280     -47.687 -35.615  57.154  1.00 46.67      D000 C
ATOM   7565  CG   GLU I 280     -47.603 -36.893  58.036  1.00 52.72      D000 C
ATOM   7566  CD   GLU I 280     -48.756 -37.083  59.067  1.00 61.50      D000 C
ATOM   7567  OE1  GLU I 280     -49.701 -36.259  59.124  1.00 55.57      D000 O
ATOM   7568  OE2  GLU I 280     -48.724 -38.095  59.816  1.00 65.48      D000 O1-
ATOM   7569  N    LEU I 281     -46.024 -32.894  56.450  1.00 68.28      D000 N
ATOM   7570  CA   LEU I 281     -45.668 -31.688  55.705  1.00 66.92      D000 C
ATOM   7571  C    LEU I 281     -45.742 -31.880  54.192  1.00 77.57      D000 C
ATOM   7572  O    LEU I 281     -45.234 -31.029  53.454  1.00 82.51      D000 O
ATOM   7573  CB   LEU I 281     -44.264 -31.217  56.095  1.00 62.30      D000 C
ATOM   7574  CG   LEU I 281     -44.155 -29.845  56.772  1.00 72.01      D000 C
ATOM   7575  CD1  LEU I 281     -42.720 -29.512  57.120  1.00 70.45      D000 C
ATOM   7576  CD2  LEU I 281     -44.746 -28.748  55.893  1.00 76.75      D000 C
ATOM   7577  N    ASP I 282     -46.399 -32.958  53.737  1.00 85.50      D000 N
ATOM   7578  CA   ASP I 282     -46.623 -33.356  52.326  1.00 86.01      D000 C
ATOM   7579  C    ASP I 282     -45.384 -34.002  51.715  1.00 79.75      D000 C
ATOM   7580  O    ASP I 282     -45.258 -35.228  51.711  1.00 78.04      D000 O
ATOM   7581  CB   ASP I 282     -47.092 -32.180  51.440  1.00 96.47      D000 C
ATOM   7582  CG   ASP I 282     -48.461 -31.617  51.857  1.00 96.65      D000 C
ATOM   7583  OD1  ASP I 282     -48.811 -31.701  53.055  1.00 90.88      D000 O
ATOM   7584  OD2  ASP I 282     -49.190 -31.087  50.977  1.00 93.64      D000 O1-
TER
ATOM   7585  N    THR P 152     -18.008 -35.866  53.223  1.00 83.74      D000 N
ATOM   7586  CA   THR P 152     -19.403 -36.301  53.203  1.00 93.67      D000 C
ATOM   7587  C    THR P 152     -19.882 -36.656  54.610  1.00 97.43      D000 C
ATOM   7588  O    THR P 152     -21.055 -36.465  54.949  1.00 99.74      D000 O
ATOM   7589  CB   THR P 152     -19.616 -37.526  52.274  1.00 95.00      D000 C
ATOM   7590  OG1  THR P 152     -18.515 -38.434  52.412  1.00 96.24      D000 O
```

```
ATOM   7591  CG2 THR P 152      -19.745 -37.100  50.820  1.00 89.07      D000 C
ATOM   7592  N   CYS P 153      -18.962 -37.176  55.422  1.00 93.53      D000 N
ATOM   7593  CA  CYS P 153      -19.248 -37.564  56.798  1.00 86.06      D000 C
ATOM   7594  C   CYS P 153      -18.009 -37.320  57.642  1.00 73.09      D000 C
ATOM   7595  O   CYS P 153      -16.902 -37.129  57.131  1.00 68.83      D000 O
ATOM   7596  CB  CYS P 153      -19.718 -39.025  56.941  1.00 85.43      D000 C
ATOM   7597  SG  CYS P 153      -21.535 -39.281  56.843  1.00 98.27      D000 S
ATOM   7598  N   CYS P 154      -18.209 -37.292  58.847  1.00 71.85      D000 N
ATOM   7599  CA  CYS P 154      -17.147 -37.059  59.803  1.00 58.19      D000 C
ATOM   7600  C   CYS P 154      -16.521 -38.377  60.251  1.00 53.75      D000 C
ATOM   7601  O   CYS P 154      -17.198 -39.409  60.291  1.00 57.74      D000 O
ATOM   7602  CB  CYS P 154      -17.697 -36.308  61.010  1.00 51.76      D000 C
ATOM   7603  SG  CYS P 154      -18.273 -34.615  60.600  1.00 60.05      D000 S
ATOM   7604  N   PRO P 155      -15.231 -38.360  60.584  1.00 46.56      D000 N
ATOM   7605  CA  PRO P 155      -14.547 -39.591  61.004  1.00 44.27      D000 C
ATOM   7606  C   PRO P 155      -15.153 -40.210  62.258  1.00 48.95      D000 C
ATOM   7607  O   PRO P 155      -15.934 -39.588  62.987  1.00 49.26      D000 O
ATOM   7608  CB  PRO P 155      -13.109 -39.126  61.268  1.00 42.58      D000 C
ATOM   7609  CG  PRO P 155      -12.971 -37.842  60.535  1.00 40.45      D000 C
ATOM   7610  CD  PRO P 155      -14.317 -37.209  60.522  1.00 44.50      D000 C
ATOM   7611  N   VAL P 156      -14.786 -41.475  62.492  1.00 50.73      D000 N
ATOM   7612  CA  VAL P 156      -15.268 -42.191  63.670  1.00 48.55      D000 C
ATOM   7613  C   VAL P 156      -14.926 -41.390  64.914  1.00 49.57      D000 C
ATOM   7614  O   VAL P 156      -13.791 -40.923  65.071  1.00 49.11      D000 O
ATOM   7615  CB  VAL P 156      -14.658 -43.599  63.733  1.00 49.04      D000 C
ATOM   7616  CG1 VAL P 156      -15.733 -44.639  63.993  1.00 37.71      D000 C
ATOM   7617  CG2 VAL P 156      -13.879 -43.914  62.449  1.00 56.31      D000 C
ATOM   7618  N   ASN P 157      -15.919 -41.205  65.791  1.00 49.29      D000 N
ATOM   7619  CA  ASN P 157      -15.831 -40.490  67.074  1.00 45.69      D000 C
ATOM   7620  C   ASN P 157      -15.791 -38.976  66.917  1.00 42.84      D000 C
ATOM   7621  O   ASN P 157      -15.571 -38.265  67.913  1.00 42.59      D000 O
ATOM   7622  CB  ASN P 157      -14.592 -40.896  67.878  1.00 46.95      D000 C
ATOM   7623  CG  ASN P 157      -14.548 -42.372  68.177  1.00 48.62      D000 C
ATOM   7624  OD1 ASN P 157      -15.544 -42.976  68.582  1.00 51.67      D000 O
ATOM   7625  ND2 ASN P 157      -13.396 -42.973  67.937  1.00 38.36      D000 N
ATOM   7626  N   TRP P 158      -16.009 -38.453  65.719  1.00 42.42      D000 N
ATOM   7627  CA  TRP P 158      -16.226 -37.030  65.523  1.00 41.39      D000 C
ATOM   7628  C   TRP P 158      -17.714 -36.789  65.274  1.00 42.54      D000 C
ATOM   7629  O   TRP P 158      -18.454 -37.697  64.895  1.00 47.35      D000 O
ATOM   7630  CB  TRP P 158      -15.395 -36.490  64.348  1.00 38.79      D000 C
ATOM   7631  CG  TRP P 158      -13.877 -36.545  64.488  1.00 37.39      D000 C
ATOM   7632  CD1 TRP P 158      -13.107 -37.655  64.704  1.00 39.44      D000 C
ATOM   7633  CD2 TRP P 158      -12.955 -35.444  64.366  1.00 35.99      D000 C
ATOM   7634  NE1 TRP P 158      -11.763 -37.316  64.728  1.00 31.69      D000 N
ATOM   7635  CE2 TRP P 158      -11.644 -35.969  64.524  1.00 34.45      D000 C
ATOM   7636  CE3 TRP P 158      -13.107 -34.070  64.140  1.00 35.66      D000 C
ATOM   7637  CZ2 TRP P 158      -10.501 -35.165  64.471  1.00 30.75      D000 C
ATOM   7638  CZ3 TRP P 158      -11.970 -33.273  64.083  1.00 35.96      D000 C
ATOM   7639  CH2 TRP P 158      -10.681 -33.827  64.247  1.00 31.46      D000 C
ATOM   7640  N   VAL P 159      -18.149 -35.554  65.509  1.00 37.16      D000 N
ATOM   7641  CA  VAL P 159      -19.556 -35.188  65.499  1.00 34.88      D000 C
ATOM   7642  C   VAL P 159      -19.788 -34.115  64.443  1.00 38.07      D000 C
ATOM   7643  O   VAL P 159      -19.116 -33.083  64.447  1.00 39.78      D000 O
ATOM   7644  CB  VAL P 159      -19.996 -34.693  66.885  1.00 37.29      D000 C
ATOM   7645  CG1 VAL P 159      -21.462 -34.286  66.872  1.00 33.76      D000 C
ATOM   7646  CG2 VAL P 159      -19.717 -35.762  67.920  1.00 35.15      D000 C
ATOM   7647  N   GLU P 160      -20.747 -34.346  63.548  1.00 44.59      D000 N
ATOM   7648  CA  GLU P 160      -21.060 -33.351  62.529  1.00 41.41      D000 C
ATOM   7649  C   GLU P 160      -22.016 -32.294  63.071  1.00 42.80      D000 C
ATOM   7650  O   GLU P 160      -22.890 -32.577  63.894  1.00 40.17      D000 O
ATOM   7651  CB  GLU P 160      -21.661 -34.005  61.284  1.00 46.52      D000 C
ATOM   7652  CG  GLU P 160      -21.512 -33.122  60.047  1.00 56.68      D000 C
ATOM   7653  CD  GLU P 160      -22.207 -33.644  58.800  1.00 66.43      D000 C
ATOM   7654  OE1 GLU P 160      -22.148 -34.876  58.533  1.00 67.75      D000 O
ATOM   7655  OE2 GLU P 160      -22.785 -32.796  58.074  1.00 67.82      D000 O1-
ATOM   7656  N   HIS P 161      -21.824 -31.060  62.622  1.00 42.59      D000 N
ATOM   7657  CA  HIS P 161      -22.738 -29.980  62.952  1.00 40.24      D000 C
ATOM   7658  C   HIS P 161      -22.456 -28.824  62.011  1.00 43.60      D000 C
ATOM   7659  O   HIS P 161      -21.382 -28.220  62.096  1.00 45.56      D000 O
ATOM   7660  CB  HIS P 161      -22.588 -29.557  64.413  1.00 35.89      D000 C
ATOM   7661  CG  HIS P 161      -23.438 -28.383  64.790  1.00 44.75      D000 C
```

```
ATOM   7662  ND1 HIS P 161    -24.736 -28.521  65.238  1.00 41.80      D000 N
ATOM   7663  CD2 HIS P 161    -23.178 -27.050  64.788  1.00 44.31      D000 C
ATOM   7664  CE1 HIS P 161    -25.239 -27.324  65.496  1.00 44.21      D000 C
ATOM   7665  NE2 HIS P 161    -24.315 -26.415  65.231  1.00 47.45      D000 N
ATOM   7666  N   GLU P 162    -23.391 -28.535  61.098  1.00 46.24      D000 N
ATOM   7667  CA  GLU P 162    -23.294 -27.382  60.201  1.00 47.89      D000 C
ATOM   7668  C   GLU P 162    -22.059 -27.512  59.301  1.00 50.82      D000 C
ATOM   7669  O   GLU P 162    -21.272 -26.573  59.127  1.00 44.74      D000 O
ATOM   7670  CB  GLU P 162    -23.249 -26.082  61.009  1.00 53.01      D000 C
ATOM   7671  CG  GLU P 162    -24.485 -25.787  61.849  1.00 58.08      D000 C
ATOM   7672  CD  GLU P 162    -25.529 -24.958  61.127  1.00 70.25      D000 C
ATOM   7673  OE1 GLU P 162    -25.288 -23.740  60.921  1.00 71.49      D000 O
ATOM   7674  OE2 GLU P 162    -26.575 -25.529  60.746  1.00 76.35      D000 O1-
ATOM   7675  N   ARG P 163    -21.884 -28.714  58.744  1.00 48.09      D000 N
ATOM   7676  CA  ARG P 163    -20.780 -29.023  57.836  1.00 57.37      D000 C
ATOM   7677  C   ARG P 163    -19.428 -28.733  58.490  1.00 57.98      D000 C
ATOM   7678  O   ARG P 163    -18.464 -28.324  57.834  1.00 57.75      D000 O
ATOM   7679  CB  ARG P 163    -20.918 -28.307  56.486  1.00 60.45      D000 C
ATOM   7680  CG  ARG P 163    -22.191 -28.694  55.747  1.00 58.49      D000 C
ATOM   7681  CD  ARG P 163    -22.201 -28.270  54.296  1.00 71.93      D000 C
ATOM   7682  NE  ARG P 163    -21.240 -29.050  53.518  1.00 71.00      D000 N
ATOM   7683  CZ  ARG P 163    -20.116 -28.576  52.983  1.00 75.19      D000 C
ATOM   7684  NH1 ARG P 163    -19.331 -29.398  52.298  1.00 77.55      D000 N1+
ATOM   7685  NH2 ARG P 163    -19.773 -27.296  53.117  1.00 67.90      D000 N
ATOM   7686  N   SER P 164    -19.350 -28.961  59.794  1.00 55.73      D000 N
ATOM   7687  CA  SER P 164    -18.096 -28.955  60.520  1.00 45.84      D000 C
ATOM   7688  C   SER P 164    -18.058 -30.235  61.329  1.00 47.92      D000 C
ATOM   7689  O   SER P 164    -19.103 -30.761  61.727  1.00 45.64      D000 O
ATOM   7690  CB  SER P 164    -17.950 -27.732  61.415  1.00 45.00      D000 C
ATOM   7691  OG  SER P 164    -17.414 -26.645  60.673  1.00 53.08      D000 O
ATOM   7692  N   CYS P 165    -16.854 -30.786  61.464  1.00 44.78      D000 N
ATOM   7693  CA  CYS P 165    -16.607 -31.976  62.262  1.00 41.69      D000 C
ATOM   7694  C   CYS P 165    -15.855 -31.593  63.523  1.00 41.12      D000 C
ATOM   7695  O   CYS P 165    -14.843 -30.886  63.464  1.00 42.93      D000 O
ATOM   7696  CB  CYS P 165    -15.813 -33.009  61.472  1.00 49.43      D000 C
ATOM   7697  SG  CYS P 165    -16.607 -33.584  59.964  1.00 54.63      D000 S
ATOM   7698  N   TYR P 166    -16.342 -32.073  64.651  1.00 38.95      D000 N
ATOM   7699  CA  TYR P 166    -15.815 -31.709  65.949  1.00 35.52      D000 C
ATOM   7700  C   TYR P 166    -15.385 -32.965  66.674  1.00 36.03      D000 C
ATOM   7701  O   TYR P 166    -16.004 -34.021  66.524  1.00 35.78      D000 O
ATOM   7702  CB  TYR P 166    -16.857 -30.955  66.776  1.00 34.96      D000 C
ATOM   7703  CG  TYR P 166    -17.320 -29.668  66.153  1.00 36.68      D000 C
ATOM   7704  CD1 TYR P 166    -18.334 -29.650  65.195  1.00 32.91      D000 C
ATOM   7705  CD2 TYR P 166    -16.732 -28.461  66.513  1.00 33.68      D000 C
ATOM   7706  CE1 TYR P 166    -18.753 -28.447  64.630  1.00 36.09      D000 C
ATOM   7707  CE2 TYR P 166    -17.141 -27.259  65.953  1.00 30.23      D000 C
ATOM   7708  CZ  TYR P 166    -18.149 -27.251  65.019  1.00 35.59      D000 C
ATOM   7709  OH  TYR P 166    -18.541 -26.044  64.482  1.00 35.19      D000 O
ATOM   7710  N   TRP P 167    -14.307 -32.851  67.435  1.00 34.48      D000 N
ATOM   7711  CA  TRP P 167    -13.853 -33.934  68.284  1.00 32.86      D000 C
ATOM   7712  C   TRP P 167    -13.586 -33.377  69.678  1.00 34.10      D000 C
ATOM   7713  O   TRP P 167    -12.924 -32.339  69.820  1.00 34.23      D000 O
ATOM   7714  CB  TRP P 167    -12.613 -34.593  67.695  1.00 31.80      D000 C
ATOM   7715  CG  TRP P 167    -12.104 -35.731  68.511  1.00 33.09      D000 C
ATOM   7716  CD1 TRP P 167    -12.489 -37.025  68.430  1.00 33.37      D000 C
ATOM   7717  CD2 TRP P 167    -11.128 -35.664  69.554  1.00 28.44      D000 C
ATOM   7718  NE1 TRP P 167    -11.793 -37.780  69.336  1.00 29.92      D000 N
ATOM   7719  CE2 TRP P 167    -10.948 -36.966  70.036  1.00 29.30      D000 C
ATOM   7720  CE3 TRP P 167    -10.351 -34.629  70.091  1.00 31.68      D000 C
ATOM   7721  CZ2 TRP P 167    -10.034 -37.270  71.048  1.00 34.20      D000 C
ATOM   7722  CZ3 TRP P 167     -9.442 -34.929  71.100  1.00 32.44      D000 C
ATOM   7723  CH2 TRP P 167     -9.294 -36.236  71.570  1.00 30.38      D000 C
ATOM   7724  N   PHE P 168    -14.102 -34.066  70.699  1.00 30.71      D000 N
ATOM   7725  CA  PHE P 168    -14.055 -33.612  72.088  1.00 32.96      D000 C
ATOM   7726  C   PHE P 168    -13.115 -34.503  72.884  1.00 29.97      D000 C
ATOM   7727  O   PHE P 168    -13.354 -35.706  73.001  1.00 33.55      D000 O
ATOM   7728  CB  PHE P 168    -15.460 -33.603  72.691  1.00 26.93      D000 C
ATOM   7729  CG  PHE P 168    -16.381 -32.711  71.950  1.00 26.93      D000 C
ATOM   7730  CD1 PHE P 168    -16.509 -31.381  72.314  1.00 25.66      D000 C
ATOM   7731  CD2 PHE P 168    -17.057 -33.170  70.831  1.00 28.28      D000 C
ATOM   7732  CE1 PHE P 168    -17.335 -30.515  71.594  1.00 29.79      D000 C
```

```
ATOM   7733  CE2 PHE P 168    -17.877 -32.328  70.108  1.00 30.00      D000 C
ATOM   7734  CZ  PHE P 168    -18.025 -30.988  70.490  1.00 26.93      D000 C
ATOM   7735  N   SER P 169    -12.026 -33.922  73.385  1.00 30.64      D000 N
ATOM   7736  CA  SER P 169    -11.061 -34.707  74.145  1.00 34.97      D000 C
ATOM   7737  C   SER P 169    -11.675 -35.155  75.471  1.00 27.83      D000 C
ATOM   7738  O   SER P 169    -12.547 -34.495  76.036  1.00 28.09      D000 O
ATOM   7739  CB  SER P 169     -9.761 -33.908  74.389  1.00 29.33      D000 C
ATOM   7740  OG  SER P 169     -9.906 -32.926  75.407  1.00 28.03      D000 O
ATOM   7741  N   ARG P 170    -11.219 -36.298  75.960  1.00 26.00      D000 N
ATOM   7742  CA  ARG P 170    -11.564 -36.758  77.295  1.00 31.89      D000 C
ATOM   7743  C   ARG P 170    -10.359 -36.735  78.233  1.00 33.73      D000 C
ATOM   7744  O   ARG P 170    -10.416 -37.279  79.337  1.00 35.93      D000 O
ATOM   7745  CB  ARG P 170    -12.183 -38.148  77.207  1.00 26.68      D000 C
ATOM   7746  CG  ARG P 170    -12.874 -38.607  78.430  1.00 33.68      D000 C
ATOM   7747  CD  ARG P 170    -14.298 -38.263  78.356  1.00 32.40      D000 C
ATOM   7748  NE  ARG P 170    -15.029 -38.782  79.492  1.00 28.94      D000 N
ATOM   7749  CZ  ARG P 170    -15.923 -38.060  80.155  1.00 29.78      D000 C
ATOM   7750  NH1 ARG P 170    -16.586 -38.582  81.182  1.00 24.35      D000 N1+
ATOM   7751  NH2 ARG P 170    -16.137 -36.805  79.779  1.00 23.51      D000 N
ATOM   7752  N   SER P 171     -9.256 -36.149  77.801  1.00 29.68      D000 N
ATOM   7753  CA  SER P 171     -8.088 -35.943  78.633  1.00 26.30      D000 C
ATOM   7754  C   SER P 171     -7.684 -34.481  78.520  1.00 31.62      D000 C
ATOM   7755  O   SER P 171     -8.299 -33.707  77.774  1.00 33.43      D000 O
ATOM   7756  CB  SER P 171     -6.959 -36.886  78.220  1.00 26.16      D000 C
ATOM   7757  OG  SER P 171     -6.679 -36.700  76.857  1.00 32.31      D000 O
ATOM   7758  N   GLY P 172     -6.662 -34.095  79.290  1.00 28.87      D000 N
ATOM   7759  CA  GLY P 172     -6.215 -32.713  79.329  1.00 27.34      D000 C
ATOM   7760  C   GLY P 172     -4.841 -32.482  78.725  1.00 28.11      D000 C
ATOM   7761  O   GLY P 172     -4.034 -33.407  78.627  1.00 29.36      D000 O
ATOM   7762  N   LYS P 173     -4.567 -31.249  78.312  1.00 26.28      D000 N
ATOM   7763  CA  LYS P 173     -3.311 -30.891  77.669  1.00 28.99      D000 C
ATOM   7764  C   LYS P 173     -3.133 -29.390  77.801  1.00 31.16      D000 C
ATOM   7765  O   LYS P 173     -4.101 -28.640  77.686  1.00 26.89      D000 O
ATOM   7766  CB  LYS P 173     -3.274 -31.249  76.168  1.00 29.64      D000 C
ATOM   7767  CG  LYS P 173     -3.023 -32.712  75.792  1.00 25.52      D000 C
ATOM   7768  CD  LYS P 173     -2.577 -32.794  74.349  1.00 26.55      D000 C
ATOM   7769  CE  LYS P 173     -2.684 -34.206  73.811  1.00 33.01      D000 C
ATOM   7770  NZ  LYS P 173     -1.755 -35.154  74.459  1.00 35.05      D000 N1+
ATOM   7771  N   ALA P 174     -1.890 -28.962  78.015  1.00 32.75      D000 N
ATOM   7772  CA  ALA P 174     -1.556 -27.556  77.874  1.00 30.91      D000 C
ATOM   7773  C   ALA P 174     -1.960 -27.069  76.483  1.00 32.66      D000 C
ATOM   7774  O   ALA P 174     -1.967 -27.838  75.511  1.00 28.87      D000 O
ATOM   7775  CB  ALA P 174     -0.062 -27.349  78.113  1.00 32.92      D000 C
ATOM   7776  N   TRP P 175     -2.308 -25.777  76.396  1.00 31.13      D000 N
ATOM   7777  CA  TRP P 175     -2.873 -25.232  75.157  1.00 35.20      D000 C
ATOM   7778  C   TRP P 175     -1.991 -25.526  73.942  1.00 35.94      D000 C
ATOM   7779  O   TRP P 175     -2.491 -25.919  72.884  1.00 31.80      D000 O
ATOM   7780  CB  TRP P 175     -3.105 -23.719  75.294  1.00 35.59      D000 C
ATOM   7781  CG  TRP P 175     -3.896 -23.089  74.131  1.00 42.77      D000 C
ATOM   7782  CD1 TRP P 175     -5.235 -22.812  74.113  1.00 41.04      D000 C
ATOM   7783  CD2 TRP P 175     -3.387 -22.650  72.846  1.00 48.23      D000 C
ATOM   7784  NE1 TRP P 175     -5.594 -22.244  72.908  1.00 42.82      D000 N
ATOM   7785  CE2 TRP P 175     -4.483 -22.130  72.115  1.00 46.08      D000 C
ATOM   7786  CE3 TRP P 175     -2.117 -22.651  72.244  1.00 45.34      D000 C
ATOM   7787  CZ2 TRP P 175     -4.348 -21.616  70.816  1.00 42.61      D000 C
ATOM   7788  CZ3 TRP P 175     -1.988 -22.133  70.948  1.00 42.70      D000 C
ATOM   7789  CH2 TRP P 175     -3.095 -21.626  70.256  1.00 41.85      D000 C
ATOM   7790  N   ALA P 176     -0.677 -25.331  74.076  1.00 34.88      D000 N
ATOM   7791  CA  ALA P 176      0.226 -25.561  72.957  1.00 30.60      D000 C
ATOM   7792  C   ALA P 176      0.226 -27.022  72.528  1.00 36.67      D000 C
ATOM   7793  O   ALA P 176      0.291 -27.325  71.328  1.00 33.09      D000 O
ATOM   7794  CB  ALA P 176      1.632 -25.111  73.326  1.00 30.07      D000 C
ATOM   7795  N   ASP P 177      0.176 -27.949  73.490  1.00 34.27      D000 N
ATOM   7796  CA  ASP P 177      0.168 -29.353  73.103  1.00 30.80      D000 C
ATOM   7797  C   ASP P 177     -1.161 -29.738  72.466  1.00 29.53      D000 C
ATOM   7798  O   ASP P 177     -1.190 -30.546  71.540  1.00 28.69      D000 O
ATOM   7799  CB  ASP P 177      0.466 -30.238  74.315  1.00 33.82      D000 C
ATOM   7800  CG  ASP P 177      1.804 -29.908  74.967  1.00 40.40      D000 C
ATOM   7801  OD1 ASP P 177      2.724 -29.448  74.243  1.00 40.31      D000 O
ATOM   7802  OD2 ASP P 177      1.934 -30.111  76.206  1.00 43.49      D000 O1-
ATOM   7803  N   ALA P 178     -2.267 -29.156  72.931  1.00 33.12      D000 N
```

502

```
ATOM   7804  CA   ALA P 178     -3.558 -29.421  72.307  1.00 31.89       D000 C
ATOM   7805  C    ALA P 178     -3.617 -28.832  70.899  1.00 35.82       D000 C
ATOM   7806  O    ALA P 178     -4.104 -29.487  69.969  1.00 35.46       D000 O
ATOM   7807  CB   ALA P 178     -4.690 -28.874  73.178  1.00 25.57       D000 C
ATOM   7808  N    ASP P 179     -3.149 -27.588  70.741  1.00 33.89       D000 N
ATOM   7809  CA   ASP P 179     -2.957 -26.971  69.429  1.00 35.32       D000 C
ATOM   7810  C    ASP P 179     -2.204 -27.905  68.481  1.00 38.70       D000 C
ATOM   7811  O    ASP P 179     -2.660 -28.182  67.365  1.00 37.15       D000 O
ATOM   7812  CB   ASP P 179     -2.188 -25.656  69.607  1.00 36.39       D000 C
ATOM   7813  CG   ASP P 179     -2.089 -24.840  68.331  1.00 45.78       D000 C
ATOM   7814  OD1  ASP P 179     -2.995 -24.936  67.468  1.00 47.95       D000 O
ATOM   7815  OD2  ASP P 179     -1.093 -24.096  68.191  1.00 50.66       D000 O1-
ATOM   7816  N    ASN P 180     -1.049 -28.410  68.918  1.00 32.54       D000 N
ATOM   7817  CA   ASN P 180     -0.271 -29.285  68.052  1.00 37.32       D000 C
ATOM   7818  C    ASN P 180     -1.019 -30.582  67.794  1.00 38.98       D000 C
ATOM   7819  O    ASN P 180     -0.911 -31.163  66.707  1.00 43.91       D000 O
ATOM   7820  CB   ASN P 180      1.118 -29.563  68.660  1.00 33.88       D000 C
ATOM   7821  CG   ASN P 180      2.023 -28.309  68.713  1.00 36.32       D000 C
ATOM   7822  OD1  ASN P 180      1.696 -27.243  68.174  1.00 45.19       D000 O
ATOM   7823  ND2  ASN P 180      3.160 -28.445  69.372  1.00 35.02       D000 N
ATOM   7824  N    TYR P 181     -1.771 -31.060  68.782  1.00 39.97       D000 N
ATOM   7825  CA   TYR P 181     -2.544 -32.280  68.589  1.00 39.12       D000 C
ATOM   7826  C    TYR P 181     -3.578 -32.097  67.471  1.00 38.18       D000 C
ATOM   7827  O    TYR P 181     -3.740 -32.963  66.601  1.00 35.54       D000 O
ATOM   7828  CB   TYR P 181     -3.216 -32.680  69.908  1.00 26.15       D000 C
ATOM   7829  CG   TYR P 181     -4.103 -33.884  69.760  1.00 30.23       D000 C
ATOM   7830  CD1  TYR P 181     -5.415 -33.752  69.303  1.00 32.63       D000 C
ATOM   7831  CD2  TYR P 181     -3.642 -35.158  70.052  1.00 31.11       D000 C
ATOM   7832  CE1  TYR P 181     -6.223 -34.846  69.131  1.00 29.23       D000 C
ATOM   7833  CE2  TYR P 181     -4.463 -36.267  69.898  1.00 26.83       D000 C
ATOM   7834  CZ   TYR P 181     -5.750 -36.093  69.433  1.00 27.61       D000 C
ATOM   7835  OH   TYR P 181     -6.587 -37.157  69.283  1.00 34.40       D000 O
ATOM   7836  N    CYS P 182     -4.291 -30.973  67.476  1.00 34.18       D000 N
ATOM   7837  CA   CYS P 182     -5.297 -30.784  66.443  1.00 40.39       D000 C
ATOM   7838  C    CYS P 182     -4.643 -30.653  65.076  1.00 39.40       D000 C
ATOM   7839  O    CYS P 182     -5.119 -31.245  64.098  1.00 36.09       D000 O
ATOM   7840  CB   CYS P 182     -6.178 -29.570  66.756  1.00 38.38       D000 C
ATOM   7841  SG   CYS P 182     -7.302 -29.774  68.185  1.00 41.03       D000 S
ATOM   7842  N    ARG P 183     -3.541 -29.899  64.997  1.00 38.60       D000 N
ATOM   7843  CA   ARG P 183     -2.855 -29.714  63.724  1.00 36.47       D000 C
ATOM   7844  C    ARG P 183     -2.442 -31.048  63.106  1.00 35.85       D000 C
ATOM   7845  O    ARG P 183     -2.647 -31.265  61.908  1.00 36.71       D000 O
ATOM   7846  CB   ARG P 183     -1.648 -28.802  63.900  1.00 36.20       D000 C
ATOM   7847  CG   ARG P 183     -1.953 -27.334  64.191  1.00 35.03       D000 C
ATOM   7848  CD   ARG P 183     -0.707 -26.729  64.791  1.00 45.48       D000 C
ATOM   7849  NE   ARG P 183     -0.297 -25.461  64.189  1.00 63.54       D000 N
ATOM   7850  CZ   ARG P 183      0.971 -25.139  63.900  1.00 64.81       D000 C
ATOM   7851  NH1  ARG P 183      1.957 -25.996  64.123  1.00 42.00       D000 N1+
ATOM   7852  NH2  ARG P 183      1.259 -23.956  63.368  1.00 73.94       D000 N
ATOM   7853  N    LEU P 184     -1.906 -31.972  63.906  1.00 34.56       D000 N
ATOM   7854  CA   LEU P 184     -1.585 -33.295  63.377  1.00 34.47       D000 C
ATOM   7855  C    LEU P 184     -2.812 -34.158  63.104  1.00 43.39       D000 C
ATOM   7856  O    LEU P 184     -2.648 -35.287  62.629  1.00 48.31       D000 O
ATOM   7857  CB   LEU P 184     -0.669 -34.073  64.311  1.00 40.32       D000 C
ATOM   7858  CG   LEU P 184      0.849 -33.962  64.235  1.00 47.86       D000 C
ATOM   7859  CD1  LEU P 184      1.306 -33.127  63.057  1.00 34.57       D000 C
ATOM   7860  CD2  LEU P 184      1.398 -33.477  65.565  1.00 40.91       D000 C
ATOM   7861  N    GLU P 185     -4.016 -33.714  63.463  1.00 43.99       D000 N
ATOM   7862  CA   GLU P 185     -5.220 -34.401  63.011  1.00 42.68       D000 C
ATOM   7863  C    GLU P 185     -5.746 -33.822  61.714  1.00 42.10       D000 C
ATOM   7864  O    GLU P 185     -6.877 -34.117  61.325  1.00 41.36       D000 O
ATOM   7865  CB   GLU P 185     -6.314 -34.365  64.076  1.00 40.55       D000 C
ATOM   7866  CG   GLU P 185     -5.941 -35.182  65.257  1.00 41.11       D000 C
ATOM   7867  CD   GLU P 185     -5.903 -36.635  64.898  1.00 46.25       D000 C
ATOM   7868  OE1  GLU P 185     -6.722 -37.051  64.048  1.00 49.14       D000 O
ATOM   7869  OE2  GLU P 185     -5.032 -37.350  65.434  1.00 47.23       D000 O1-
ATOM   7870  N    ASP P 186     -4.940 -33.010  61.039  1.00 47.67       D000 N
ATOM   7871  CA   ASP P 186     -5.390 -32.232  59.896  1.00 45.07       D000 C
ATOM   7872  C    ASP P 186     -6.621 -31.416  60.279  1.00 43.94       D000 C
ATOM   7873  O    ASP P 186     -7.601 -31.332  59.539  1.00 49.84       D000 O
ATOM   7874  CB   ASP P 186     -5.656 -33.149  58.700  1.00 47.50       D000 C
```

```
ATOM   7875  CG  ASP P 186      -5.429 -32.459  57.365  1.00 55.72      D000 C
ATOM   7876  OD1 ASP P 186      -5.209 -31.227  57.346  1.00 54.34      D000 O
ATOM   7877  OD2 ASP P 186      -5.476 -33.160  56.329  1.00 65.88      D000 O1-
ATOM   7878  N   ALA P 187      -6.558 -30.797  61.453  1.00 43.57      D000 N
ATOM   7879  CA  ALA P 187      -7.683 -30.054  62.009  1.00 40.06      D000 C
ATOM   7880  C   ALA P 187      -7.136 -28.872  62.799  1.00 39.06      D000 C
ATOM   7881  O   ALA P 187      -5.965 -28.502  62.673  1.00 41.54      D000 O
ATOM   7882  CB  ALA P 187      -8.559 -30.971  62.871  1.00 37.26      D000 C
ATOM   7883  N   HIS P 188      -7.984 -28.270  63.620  1.00 38.84      D000 N
ATOM   7884  CA  HIS P 188      -7.523 -27.141  64.411  1.00 42.15      D000 C
ATOM   7885  C   HIS P 188      -8.399 -26.992  65.655  1.00 44.84      D000 C
ATOM   7886  O   HIS P 188      -9.536 -27.477  65.704  1.00 37.93      D000 O
ATOM   7887  CB  HIS P 188      -7.528 -25.863  63.577  1.00 38.38      D000 C
ATOM   7888  CG  HIS P 188      -8.885 -25.488  63.084  1.00 39.56      D000 C
ATOM   7889  ND1 HIS P 188      -9.637 -24.489  63.663  1.00 42.56      D000 N
ATOM   7890  CD2 HIS P 188      -9.644 -26.008  62.092  1.00 38.88      D000 C
ATOM   7891  CE1 HIS P 188     -10.793 -24.394  63.034  1.00 45.45      D000 C
ATOM   7892  NE2 HIS P 188     -10.822 -25.305  62.075  1.00 47.23      D000 N
ATOM   7893  N   LEU P 189      -7.834 -26.338  66.673  1.00 39.67      D000 N
ATOM   7894  CA  LEU P 189      -8.599 -26.011  67.860  1.00 36.17      D000 C
ATOM   7895  C   LEU P 189      -9.829 -25.215  67.464  1.00 35.83      D000 C
ATOM   7896  O   LEU P 189      -9.731 -24.279  66.669  1.00 34.94      D000 O
ATOM   7897  CB  LEU P 189      -7.733 -25.219  68.832  1.00 37.51      D000 C
ATOM   7898  CG  LEU P 189      -6.682 -25.973  69.637  1.00 36.68      D000 C
ATOM   7899  CD1 LEU P 189      -5.830 -25.001  70.439  1.00 36.06      D000 C
ATOM   7900  CD2 LEU P 189      -7.377 -26.952  70.569  1.00 34.22      D000 C
ATOM   7901  N   VAL P 190     -10.979 -25.590  68.040  1.00 34.05      D000 N
ATOM   7902  CA  VAL P 190     -12.274 -25.094  67.585  1.00 34.93      D000 C
ATOM   7903  C   VAL P 190     -12.304 -23.565  67.518  1.00 35.33      D000 C
ATOM   7904  O   VAL P 190     -11.823 -22.852  68.410  1.00 34.50      D000 O
ATOM   7905  CB  VAL P 190     -13.400 -25.641  68.486  1.00 34.45      D000 C
ATOM   7906  CG1 VAL P 190     -13.221 -25.185  69.924  1.00 32.88      D000 C
ATOM   7907  CG2 VAL P 190     -14.740 -25.157  67.990  1.00 33.98      D000 C
ATOM   7908  N   VAL P 191     -12.889 -23.066  66.438  1.00 34.01      D000 N
ATOM   7909  CA  VAL P 191     -13.051 -21.643  66.182  1.00 35.07      D000 C
ATOM   7910  C   VAL P 191     -14.544 -21.382  66.161  1.00 36.61      D000 C
ATOM   7911  O   VAL P 191     -15.256 -21.923  65.306  1.00 41.56      D000 O
ATOM   7912  CB  VAL P 191     -12.385 -21.225  64.856  1.00 35.46      D000 C
ATOM   7913  CG1 VAL P 191     -12.817 -19.830  64.431  1.00 35.96      D000 C
ATOM   7914  CG2 VAL P 191     -10.875 -21.295  64.981  1.00 36.37      D000 C
ATOM   7915  N   VAL P 192     -15.021 -20.581  67.111  1.00 35.79      D000 N
ATOM   7916  CA  VAL P 192     -16.450 -20.417  67.355  1.00 37.67      D000 C
ATOM   7917  C   VAL P 192     -16.893 -19.094  66.754  1.00 37.75      D000 C
ATOM   7918  O   VAL P 192     -16.442 -18.024  67.186  1.00 38.75      D000 O
ATOM   7919  CB  VAL P 192     -16.776 -20.473  68.854  1.00 38.25      D000 C
ATOM   7920  CG1 VAL P 192     -18.267 -20.314  69.053  1.00 35.90      D000 C
ATOM   7921  CG2 VAL P 192     -16.282 -21.774  69.455  1.00 37.22      D000 C
ATOM   7922  N   THR P 193     -17.795 -19.159  65.778  1.00 36.90      D000 N
ATOM   7923  CA  THR P 193     -18.151 -17.982  64.995  1.00 42.26      D000 C
ATOM   7924  C   THR P 193     -19.600 -17.530  65.148  1.00 41.06      D000 C
ATOM   7925  O   THR P 193     -19.950 -16.480  64.605  1.00 46.91      D000 O
ATOM   7926  CB  THR P 193     -17.813 -18.204  63.503  1.00 40.10      D000 C
ATOM   7927  OG1 THR P 193     -18.471 -19.382  63.012  1.00 45.59      D000 O
ATOM   7928  CG2 THR P 193     -16.302 -18.323  63.286  1.00 36.33      D000 C
ATOM   7929  N   SER P 194     -20.440 -18.252  65.892  1.00 39.77      D000 N
ATOM   7930  CA  SER P 194     -21.849 -17.898  66.016  1.00 36.24      D000 C
ATOM   7931  C   SER P 194     -22.416 -18.395  67.342  1.00 40.36      D000 C
ATOM   7932  O   SER P 194     -21.829 -19.244  68.013  1.00 39.43      D000 O
ATOM   7933  CB  SER P 194     -22.666 -18.470  64.855  1.00 32.78      D000 C
ATOM   7934  OG  SER P 194     -22.508 -19.875  64.777  1.00 40.86      D000 O
ATOM   7935  N   TRP P 195     -23.589 -17.862  67.714  1.00 44.41      D000 N
ATOM   7936  CA  TRP P 195     -24.275 -18.381  68.894  1.00 41.04      D000 C
ATOM   7937  C   TRP P 195     -24.665 -19.830  68.684  1.00 37.41      D000 C
ATOM   7938  O   TRP P 195     -24.633 -20.639  69.618  1.00 38.72      D000 O
ATOM   7939  CB  TRP P 195     -25.519 -17.557  69.216  1.00 44.82      D000 C
ATOM   7940  CG  TRP P 195     -25.243 -16.309  70.020  1.00 51.32      D000 C
ATOM   7941  CD1 TRP P 195     -25.307 -15.008  69.586  1.00 46.03      D000 C
ATOM   7942  CD2 TRP P 195     -24.854 -16.251  71.395  1.00 47.53      D000 C
ATOM   7943  NE1 TRP P 195     -24.987 -14.148  70.610  1.00 47.20      D000 N
ATOM   7944  CE2 TRP P 195     -24.706 -14.885  71.733  1.00 51.18      D000 C
ATOM   7945  CE3 TRP P 195     -24.614 -17.221  72.375  1.00 46.44      D000 C
```

```
ATOM   7946  CZ2 TRP P 195    -24.331 -14.464  73.016  1.00 55.85     D000 C
ATOM   7947  CZ3 TRP P 195    -24.241 -16.801  73.654  1.00 53.40     D000 C
ATOM   7948  CH2 TRP P 195    -24.105 -15.435  73.961  1.00 52.97     D000 C
ATOM   7949  N   GLU P 196    -25.024 -20.181  67.458  1.00 34.05     D000 N
ATOM   7950  CA  GLU P 196    -25.384 -21.557  67.174  1.00 36.55     D000 C
ATOM   7951  C   GLU P 196    -24.200 -22.484  67.430  1.00 37.31     D000 C
ATOM   7952  O   GLU P 196    -24.343 -23.528  68.078  1.00 36.90     D000 O
ATOM   7953  CB  GLU P 196    -25.899 -21.650  65.740  1.00 37.33     D000 C
ATOM   7954  CG  GLU P 196    -25.908 -23.036  65.156  1.00 54.30     D000 C
ATOM   7955  CD  GLU P 196    -27.254 -23.709  65.306  1.00 67.53     D000 O
ATOM   7956  OE1 GLU P 196    -28.111 -23.139  66.025  1.00 74.43     D000 O
ATOM   7957  OE2 GLU P 196    -27.453 -24.799  64.704  1.00 66.20     D000 O1-
ATOM   7958  N   GLU P 197    -23.008 -22.101  66.961  1.00 37.44     D000 N
ATOM   7959  CA  GLU P 197    -21.837 -22.949  67.173  1.00 37.15     D000 C
ATOM   7960  C   GLU P 197    -21.448 -23.002  68.652  1.00 36.59     D000 C
ATOM   7961  O   GLU P 197    -21.077 -24.064  69.166  1.00 34.20     D000 O
ATOM   7962  CB  GLU P 197    -20.674 -22.458  66.315  1.00 32.94     D000 C
ATOM   7963  CG  GLU P 197    -19.417 -23.347  66.343  1.00 35.50     D000 C
ATOM   7964  CD  GLU P 197    -18.383 -22.948  65.292  1.00 41.89     D000 C
ATOM   7965  OE1 GLU P 197    -18.466 -21.807  64.772  1.00 45.12     D000 O
ATOM   7966  OE2 GLU P 197    -17.486 -23.771  64.985  1.00 41.03     D000 O1-
ATOM   7967  N   GLN P 198    -21.538 -21.865  69.345  1.00 32.12     D000 N
ATOM   7968  CA  GLN P 198    -21.193 -21.792  70.759  1.00 31.52     D000 C
ATOM   7969  C   GLN P 198    -22.083 -22.703  71.598  1.00 35.74     D000 C
ATOM   7970  O   GLN P 198    -21.600 -23.403  72.501  1.00 30.28     D000 O
ATOM   7971  CB  GLN P 198    -21.298 -20.334  71.226  1.00 33.50     D000 C
ATOM   7972  CG  GLN P 198    -21.391 -20.126  72.734  1.00 33.84     D000 C
ATOM   7973  CD  GLN P 198    -20.055 -20.173  73.450  1.00 33.31     D000 C
ATOM   7974  OE1 GLN P 198    -19.009 -19.859  72.878  1.00 30.27     D000 O
ATOM   7975  NE2 GLN P 198    -20.085 -20.563  74.713  1.00 33.30     D000 N
ATOM   7976  N   LYS P 199    -23.393 -22.694  71.320  1.00 35.11     D000 N
ATOM   7977  CA  LYS P 199    -24.328 -23.523  72.070  1.00 32.09     D000 C
ATOM   7978  C   LYS P 199    -24.070 -25.009  71.828  1.00 32.10     D000 C
ATOM   7979  O   LYS P 199    -24.103 -25.811  72.767  1.00 31.39     D000 O
ATOM   7980  CB  LYS P 199    -25.753 -23.142  71.692  1.00 31.90     D000 C
ATOM   7981  CG  LYS P 199    -26.283 -21.993  72.497  1.00 34.02     D000 C
ATOM   7982  CD  LYS P 199    -27.260 -21.182  71.709  1.00 36.65     D000 C
ATOM   7983  CE  LYS P 199    -28.314 -22.071  71.096  1.00 40.59     D000 C
ATOM   7984  NZ  LYS P 199    -29.444 -21.249  70.594  1.00 48.36     D000 N1+
ATOM   7985  N   PHE P 200    -23.806 -25.392  70.581  1.00 28.05     D000 N
ATOM   7986  CA  PHE P 200    -23.515 -26.788  70.283  1.00 28.75     D000 C
ATOM   7987  C   PHE P 200    -22.286 -27.284  71.036  1.00 32.43     D000 C
ATOM   7988  O   PHE P 200    -22.287 -28.399  71.568  1.00 30.79     D000 O
ATOM   7989  CB  PHE P 200    -23.318 -26.956  68.787  1.00 27.58     D000 C
ATOM   7990  CG  PHE P 200    -22.644 -28.222  68.417  1.00 29.26     D000 C
ATOM   7991  CD1 PHE P 200    -23.363 -29.387  68.303  1.00 28.09     D000 C
ATOM   7992  CD2 PHE P 200    -21.279 -28.247  68.172  1.00 31.84     D000 C
ATOM   7993  CE1 PHE P 200    -22.747 -30.561  67.943  1.00 30.20     D000 C
ATOM   7994  CE2 PHE P 200    -20.647 -29.415  67.799  1.00 31.13     D000 C
ATOM   7995  CZ  PHE P 200    -21.385 -30.577  67.682  1.00 33.34     D000 C
ATOM   7996  N   VAL P 201    -21.220 -26.478  71.083  1.00 30.80     D000 N
ATOM   7997  CA  VAL P 201    -20.015 -26.900  71.787  1.00 31.26     D000 C
ATOM   7998  C   VAL P 201    -20.277 -26.989  73.295  1.00 31.79     D000 C
ATOM   7999  O   VAL P 201    -19.849 -27.947  73.948  1.00 29.59     D000 O
ATOM   8000  CB  VAL P 201    -18.836 -25.959  71.458  1.00 30.91     D000 C
ATOM   8001  CG1 VAL P 201    -17.611 -26.260  72.345  1.00 32.04     D000 C
ATOM   8002  CG2 VAL P 201    -18.433 -26.104  70.020  1.00 33.28     D000 C
ATOM   8003  N   GLN P 202    -21.001 -26.012  73.862  1.00 30.50     D000 N
ATOM   8004  CA  GLN P 202    -21.323 -26.044  75.290  1.00 30.37     D000 C
ATOM   8005  C   GLN P 202    -22.040 -27.327  75.670  1.00 25.89     D000 C
ATOM   8006  O   GLN P 202    -21.755 -27.903  76.719  1.00 33.97     D000 O
ATOM   8007  CB  GLN P 202    -22.203 -24.865  75.701  1.00 35.09     D000 C
ATOM   8008  CG  GLN P 202    -21.566 -23.520  75.783  1.00 35.69     D000 C
ATOM   8009  CD  GLN P 202    -22.490 -22.528  76.480  1.00 47.70     D000 C
ATOM   8010  OE1 GLN P 202    -22.615 -21.351  76.078  1.00 41.49     D000 O
ATOM   8011  NE2 GLN P 202    -23.110 -22.990  77.575  1.00 49.12     D000 N
ATOM   8012  N   HIS P 203    -23.025 -27.742  74.871  1.00 25.10     D000 N
ATOM   8013  CA  HIS P 203    -23.731 -29.000  75.117  1.00 27.41     D000 C
ATOM   8014  C   HIS P 203    -22.780 -30.154  75.316  1.00 32.74     D000 C
ATOM   8015  O   HIS P 203    -22.919 -30.940  76.262  1.00 37.16     D000 O
ATOM   8016  CB  HIS P 203    -24.638 -29.354  73.947  1.00 31.40     D000 C
```

```
ATOM    8017  CG  HIS P 203     -26.051 -28.936  74.117  1.00 37.58      D000 C
ATOM    8018  ND1 HIS P 203     -26.725 -28.198  73.167  1.00 46.93      D000 N
ATOM    8019  CD2 HIS P 203     -26.942 -29.204  75.098  1.00 43.64      D000 C
ATOM    8020  CE1 HIS P 203     -27.963 -27.992  73.576  1.00 52.72      D000 C
ATOM    8021  NE2 HIS P 203     -28.122 -28.598  74.742  1.00 57.54      D000 N
ATOM    8022  N   HIS P 204     -21.809 -30.280  74.406  1.00 33.87      D000 N
ATOM    8023  CA  HIS P 204     -20.952 -31.453  74.375  1.00 32.62      D000 C
ATOM    8024  C   HIS P 204     -19.857 -31.394  75.428  1.00 30.65      D000 C
ATOM    8025  O   HIS P 204     -19.399 -32.445  75.880  1.00 27.67      D000 O
ATOM    8026  CB  HIS P 204     -20.373 -31.635  72.969  1.00 25.37      D000 C
ATOM    8027  CG  HIS P 204     -21.341 -32.234  71.995  1.00 29.38      D000 C
ATOM    8028  ND1 HIS P 204     -21.525 -33.593  71.869  1.00 30.92      D000 N
ATOM    8029  CD2 HIS P 204     -22.217 -31.661  71.137  1.00 32.57      D000 C
ATOM    8030  CE1 HIS P 204     -22.445 -33.835  70.953  1.00 28.23      D000 C
ATOM    8031  NE2 HIS P 204     -22.883 -32.678  70.494  1.00 34.46      D000 N
ATOM    8032  N   ILE P 205     -19.424 -30.197  75.830  1.00 28.24      D000 N
ATOM    8033  CA  ILE P 205     -18.324 -30.104  76.778  1.00 27.46      D000 C
ATOM    8034  C   ILE P 205     -18.778 -30.056  78.220  1.00 33.06      D000 C
ATOM    8035  O   ILE P 205     -17.952 -30.295  79.111  1.00 35.59      D000 O
ATOM    8036  CB  ILE P 205     -17.392 -28.907  76.513  1.00 33.12      D000 C
ATOM    8037  CG1 ILE P 205     -18.123 -27.564  76.648  1.00 26.96      D000 C
ATOM    8038  CG2 ILE P 205     -16.702 -29.080  75.173  1.00 25.98      D000 C
ATOM    8039  CD1 ILE P 205     -17.174 -26.412  76.624  1.00 23.08      D000 C
ATOM    8040  N   GLY P 206     -20.043 -29.713  78.482  1.00 35.40      D000 N
ATOM    8041  CA  GLY P 206     -20.536 -29.649  79.837  1.00 29.75      D000 C
ATOM    8042  C   GLY P 206     -19.906 -28.523  80.620  1.00 32.65      D000 C
ATOM    8043  O   GLY P 206     -19.313 -27.601  80.053  1.00 35.11      D000 O
ATOM    8044  N   PRO P 207     -20.032 -28.568  81.948  1.00 33.48      D000 N
ATOM    8045  CA  PRO P 207     -19.544 -27.464  82.797  1.00 31.43      D000 C
ATOM    8046  C   PRO P 207     -18.059 -27.610  83.123  1.00 36.17      D000 C
ATOM    8047  O   PRO P 207     -17.637 -27.650  84.288  1.00 38.20      D000 O
ATOM    8048  CB  PRO P 207     -20.443 -27.590  84.032  1.00 35.30      D000 C
ATOM    8049  CG  PRO P 207     -20.723 -29.073  84.132  1.00 31.37      D000 C
ATOM    8050  CD  PRO P 207     -20.694 -29.628  82.727  1.00 30.51      D000 C
ATOM    8051  N   VAL P 208     -17.242 -27.622  82.071  1.00 35.28      D000 N
ATOM    8052  CA  VAL P 208     -15.854 -28.057  82.127  1.00 31.19      D000 C
ATOM    8053  C   VAL P 208     -14.990 -27.043  81.389  1.00 32.34      D000 C
ATOM    8054  O   VAL P 208     -15.252 -26.754  80.217  1.00 30.13      D000 O
ATOM    8055  CB  VAL P 208     -15.733 -29.454  81.493  1.00 24.39      D000 C
ATOM    8056  CG1 VAL P 208     -14.304 -29.927  81.433  1.00 19.72      D000 C
ATOM    8057  CG2 VAL P 208     -16.623 -30.417  82.259  1.00 26.85      D000 C
ATOM    8058  N   ASN P 209     -13.955 -26.514  82.065  1.00 33.01      D000 N
ATOM    8059  CA  ASN P 209     -12.987 -25.628  81.404  1.00 28.33      D000 C
ATOM    8060  C   ASN P 209     -12.325 -26.339  80.224  1.00 28.70      D000 C
ATOM    8061  O   ASN P 209     -11.666 -27.369  80.409  1.00 26.30      D000 O
ATOM    8062  CB  ASN P 209     -11.911 -25.165  82.388  1.00 26.60      D000 C
ATOM    8063  CG  ASN P 209     -12.401 -24.116  83.370  1.00 27.81      D000 C
ATOM    8064  OD1 ASN P 209     -13.324 -23.343  83.094  1.00 31.03      D000 O
ATOM    8065  ND2 ASN P 209     -11.774 -24.083  84.528  1.00 22.70      D000 N
ATOM    8066  N   THR P 210     -12.487 -25.786  79.017  1.00 27.48      D000 N
ATOM    8067  CA  THR P 210     -12.049 -26.451  77.790  1.00 27.53      D000 C
ATOM    8068  C   THR P 210     -11.385 -25.441  76.860  1.00 29.97      D000 C
ATOM    8069  O   THR P 210     -11.918 -24.350  76.656  1.00 28.86      D000 O
ATOM    8070  CB  THR P 210     -13.223 -27.156  77.082  1.00 28.24      D000 C
ATOM    8071  OG1 THR P 210     -13.710 -28.226  77.908  1.00 30.12      D000 O
ATOM    8072  CG2 THR P 210     -12.801 -27.746  75.755  1.00 23.19      D000 C
ATOM    8073  N   TRP P 211     -10.219 -25.797  76.313  1.00 29.85      D000 N
ATOM    8074  CA  TRP P 211      -9.514 -24.902  75.399  1.00 31.37      D000 C
ATOM    8075  C   TRP P 211     -10.280 -24.746  74.099  1.00 34.18      D000 C
ATOM    8076  O   TRP P 211     -10.884 -25.707  73.604  1.00 32.26      D000 O
ATOM    8077  CB  TRP P 211      -8.122 -25.430  75.046  1.00 26.53      D000 C
ATOM    8078  CG  TRP P 211      -7.115 -25.421  76.131  1.00 30.84      D000 C
ATOM    8079  CD1 TRP P 211      -6.341 -26.470  76.523  1.00 29.48      D000 C
ATOM    8080  CD2 TRP P 211      -6.740 -24.312  76.959  1.00 29.42      D000 C
ATOM    8081  NE1 TRP P 211      -5.507 -26.084  77.538  1.00 29.42      D000 N
ATOM    8082  CE2 TRP P 211      -5.740 -24.768  77.832  1.00 29.17      D000 C
ATOM    8083  CE3 TRP P 211      -7.150 -22.979  77.041  1.00 31.34      D000 C
ATOM    8084  CZ2 TRP P 211      -5.145 -23.942  78.778  1.00 31.63      D000 C
ATOM    8085  CZ3 TRP P 211      -6.563 -22.163  77.977  1.00 28.33      D000 C
ATOM    8086  CH2 TRP P 211      -5.568 -22.642  78.830  1.00 30.22      D000 C
ATOM    8087  N   MET P 212     -10.227 -23.530  73.540  1.00 33.06      D000 N
```

```
ATOM   8088  CA   MET P 212    -10.647 -23.222  72.175  1.00 33.50      D000 C
ATOM   8089  C    MET P 212     -9.461 -22.623  71.415  1.00 35.69      D000 C
ATOM   8090  O    MET P 212     -8.401 -22.362  71.985  1.00 33.28      D000 O
ATOM   8091  CB   MET P 212    -11.841 -22.265  72.153  1.00 27.35      D000 C
ATOM   8092  CG   MET P 212    -11.442 -20.835  72.335  1.00 34.07      D000 C
ATOM   8093  SD   MET P 212    -12.807 -19.717  72.680  1.00 33.50      D000 S
ATOM   8094  CE   MET P 212    -13.264 -20.227  74.323  1.00 34.96      D000 C
ATOM   8095  N    GLY P 213     -9.615 -22.465  70.099  1.00 38.96      D000 N
ATOM   8096  CA   GLY P 213     -8.538 -21.941  69.266  1.00 36.71      D000 C
ATOM   8097  C    GLY P 213     -8.297 -20.437  69.293  1.00 36.61      D000 C
ATOM   8098  O    GLY P 213     -8.265 -19.807  68.237  1.00 34.15      D000 O
ATOM   8099  N    LEU P 214     -8.113 -19.847  70.472  1.00 37.22      D000 N
ATOM   8100  CA   LEU P 214     -7.994 -18.399  70.609  1.00 40.19      D000 C
ATOM   8101  C    LEU P 214     -6.897 -18.065  71.618  1.00 44.61      D000 C
ATOM   8102  O    LEU P 214     -6.928 -18.534  72.761  1.00 42.53      D000 O
ATOM   8103  CB   LEU P 214     -9.340 -17.783  71.019  1.00 36.46      D000 C
ATOM   8104  CG   LEU P 214     -9.420 -16.273  71.253  1.00 39.49      D000 C
ATOM   8105  CD1  LEU P 214     -9.308 -15.528  69.943  1.00 39.79      D000 C
ATOM   8106  CD2  LEU P 214    -10.692 -15.890  71.983  1.00 37.37      D000 C
ATOM   8107  N    HIS P 215     -5.914 -17.274  71.191  1.00 47.99      D000 N
ATOM   8108  CA   HIS P 215     -4.787 -16.902  72.042  1.00 53.52      D000 C
ATOM   8109  C    HIS P 215     -4.270 -15.525  71.632  1.00 50.41      D000 C
ATOM   8110  O    HIS P 215     -4.496 -15.076  70.506  1.00 50.05      D000 O
ATOM   8111  CB   HIS P 215     -3.668 -17.963  71.988  1.00 48.40      D000 C
ATOM   8112  CG   HIS P 215     -3.026 -18.112  70.645  1.00 51.27      D000 C
ATOM   8113  ND1  HIS P 215     -1.709 -18.488  70.490  1.00 52.99      D000 N
ATOM   8114  CD2  HIS P 215     -3.517 -17.937  69.394  1.00 48.48      D000 C
ATOM   8115  CE1  HIS P 215     -1.417 -18.539  69.203  1.00 55.12      D000 C
ATOM   8116  NE2  HIS P 215     -2.496 -18.205  68.517  1.00 52.31      D000 N
ATOM   8117  N    ASP P 216     -3.639 -14.826  72.579  1.00 50.32      D000 N
ATOM   8118  CA   ASP P 216     -2.999 -13.547  72.283  1.00 62.90      D000 C
ATOM   8119  C    ASP P 216     -1.529 -13.584  72.680  1.00 66.62      D000 C
ATOM   8120  O    ASP P 216     -0.957 -12.579  73.111  1.00 70.47      D000 O
ATOM   8121  CB   ASP P 216     -3.730 -12.376  72.949  1.00 58.20      D000 C
ATOM   8122  CG   ASP P 216     -3.387 -12.201  74.428  1.00 62.56      D000 C
ATOM   8123  OD1  ASP P 216     -2.915 -13.156  75.088  1.00 67.95      D000 O
ATOM   8124  OD2  ASP P 216     -3.613 -11.088  74.942  1.00 66.89      D000 O1-
ATOM   8125  N    GLN P 217     -0.907 -14.751  72.520  1.00 64.97      D000 N
ATOM   8126  CA   GLN P 217      0.521 -14.866  72.771  1.00 76.33      D000 C
ATOM   8127  C    GLN P 217      1.312 -13.867  71.944  1.00 79.09      D000 C
ATOM   8128  O    GLN P 217      2.439 -13.521  72.314  1.00 84.36      D000 O
ATOM   8129  CB   GLN P 217      0.981 -16.309  72.509  1.00 76.19      D000 C
ATOM   8130  CG   GLN P 217      0.950 -17.206  73.776  1.00 69.70      D000 C
ATOM   8131  CD   GLN P 217      0.539 -18.645  73.498  1.00 61.34      D000 C
ATOM   8132  OE1  GLN P 217     -0.353 -18.897  72.690  1.00 62.21      D000 O
ATOM   8133  NE2  GLN P 217      1.177 -19.597  74.185  1.00 61.50      D000 N
ATOM   8134  N    ASN P 218      0.722 -13.361  70.863  1.00 80.19      D000 N
ATOM   8135  CA   ASN P 218      1.363 -12.319  70.073  1.00 87.85      D000 C
ATOM   8136  C    ASN P 218      1.275 -10.964  70.775  1.00 79.48      D000 C
ATOM   8137  O    ASN P 218      2.294 -10.367  71.140  1.00 80.45      D000 O
ATOM   8138  CB   ASN P 218      0.705 -12.268  68.689  1.00 84.56      D000 C
ATOM   8139  CG   ASN P 218      1.684 -11.916  67.594  1.00 89.88      D000 C
ATOM   8140  OD1  ASN P 218      1.373 -12.042  66.404  1.00 90.71      D000 O
ATOM   8141  ND2  ASN P 218      2.891 -11.507  67.986  1.00 84.53      D000 N
ATOM   8142  N    GLY P 219      0.059 -10.514  71.040  1.00 74.18      D000 N
ATOM   8143  CA   GLY P 219     -0.212  -9.187  71.530  1.00 68.61      D000 C
ATOM   8144  C    GLY P 219     -1.710  -8.949  71.462  1.00 73.97      D000 C
ATOM   8145  O    GLY P 219     -2.366  -8.740  72.489  1.00 71.33      D000 O
ATOM   8146  N    PRO P 220     -2.285  -9.017  70.255  1.00 69.70      D000 N
ATOM   8147  CA   PRO P 220     -3.745  -9.029  70.112  1.00 69.83      D000 C
ATOM   8148  C    PRO P 220     -4.304 -10.446  70.009  1.00 67.70      D000 C
ATOM   8149  O    PRO P 220     -3.608 -11.402  69.652  1.00 66.31      D000 O
ATOM   8150  CB   PRO P 220     -3.967  -8.274  68.792  1.00 64.94      D000 C
ATOM   8151  CG   PRO P 220     -2.674  -8.444  68.009  1.00 60.23      D000 C
ATOM   8152  CD   PRO P 220     -1.625  -9.035  68.933  1.00 70.78      D000 C
ATOM   8153  N    TRP P 221     -5.595 -10.568  70.309  1.00 58.78      D000 N
ATOM   8154  CA   TRP P 221     -6.244 -11.868  70.228  1.00 52.55      D000 C
ATOM   8155  C    TRP P 221     -6.415 -12.290  68.778  1.00 51.19      D000 C
ATOM   8156  O    TRP P 221     -6.795 -11.486  67.920  1.00 53.28      D000 O
ATOM   8157  CB   TRP P 221     -7.601 -11.831  70.940  1.00 52.62      D000 C
ATOM   8158  CG   TRP P 221     -7.438 -11.729  72.425  1.00 50.70      D000 C
```

```
ATOM   8159  CD1 TRP P 221      -7.477 -10.597  73.179  1.00 52.92      D000 C
ATOM   8160  CD2 TRP P 221      -7.164 -12.807  73.332  1.00 48.63      D000 C
ATOM   8161  NE1 TRP P 221      -7.256 -10.901  74.505  1.00 54.57      D000 N
ATOM   8162  CE2 TRP P 221      -7.058 -12.251  74.624  1.00 49.93      D000 C
ATOM   8163  CE3 TRP P 221      -7.001 -14.189  73.177  1.00 46.93      D000 C
ATOM   8164  CZ2 TRP P 221      -6.796 -13.029  75.757  1.00 50.65      D000 C
ATOM   8165  CZ3 TRP P 221      -6.739 -14.960  74.303  1.00 46.92      D000 C
ATOM   8166  CH2 TRP P 221      -6.643 -14.377  75.575  1.00 48.14      D000 C
ATOM   8167  N   LYS P 222      -6.132 -13.568  68.513  1.00 50.38      D000 N
ATOM   8168  CA  LYS P 222      -6.213 -14.155  67.178  1.00 52.36      D000 C
ATOM   8169  C   LYS P 222      -6.785 -15.564  67.274  1.00 48.11      D000 C
ATOM   8170  O   LYS P 222      -6.551 -16.277  68.253  1.00 43.41      D000 O
ATOM   8171  CB  LYS P 222      -4.842 -14.247  66.473  1.00 48.15      D000 C
ATOM   8172  CG  LYS P 222      -3.989 -13.006  66.480  1.00 49.51      D000 C
ATOM   8173  CD  LYS P 222      -2.623 -13.343  65.879  1.00 63.49      D000 C
ATOM   8174  CE  LYS P 222      -1.744 -12.107  65.682  1.00 74.29      D000 C
ATOM   8175  NZ  LYS P 222      -0.559 -12.385  64.812  1.00 71.05      D000 N1+
ATOM   8176  N   TRP P 223      -7.518 -15.967  66.239  1.00 46.69      D000 N
ATOM   8177  CA  TRP P 223      -7.966 -17.345  66.081  1.00 40.92      D000 C
ATOM   8178  C   TRP P 223      -6.901 -18.155  65.334  1.00 42.46      D000 C
ATOM   8179  O   TRP P 223      -6.100 -17.605  64.578  1.00 42.65      D000 O
ATOM   8180  CB  TRP P 223      -9.314 -17.398  65.346  1.00 38.25      D000 C
ATOM   8181  CG  TRP P 223     -10.464 -16.803  66.160  1.00 46.60      D000 C
ATOM   8182  CD1 TRP P 223     -10.918 -15.508  66.115  1.00 44.78      D000 C
ATOM   8183  CD2 TRP P 223     -11.284 -17.476  67.141  1.00 41.63      D000 C
ATOM   8184  NE1 TRP P 223     -11.954 -15.337  67.003  1.00 45.05      D000 N
ATOM   8185  CE2 TRP P 223     -12.203 -16.526  67.641  1.00 43.02      D000 C
ATOM   8186  CE3 TRP P 223     -11.328 -18.786  67.644  1.00 40.53      D000 C
ATOM   8187  CZ2 TRP P 223     -13.152 -16.843  68.628  1.00 38.24      D000 C
ATOM   8188  CZ3 TRP P 223     -12.271 -19.097  68.619  1.00 38.15      D000 C
ATOM   8189  CH2 TRP P 223     -13.165 -18.125  69.103  1.00 37.47      D000 C
ATOM   8190  N   VAL P 224      -6.863 -19.466  65.601  1.00 41.18      D000 N
ATOM   8191  CA  VAL P 224      -5.813 -20.316  65.049  1.00 40.27      D000 C
ATOM   8192  C   VAL P 224      -5.952 -20.568  63.553  1.00 43.70      D000 C
ATOM   8193  O   VAL P 224      -4.977 -20.993  62.918  1.00 45.37      D000 O
ATOM   8194  CB  VAL P 224      -5.738 -21.679  65.760  1.00 35.45      D000 C
ATOM   8195  CG1 VAL P 224      -5.402 -21.490  67.200  1.00 39.13      D000 C
ATOM   8196  CG2 VAL P 224      -7.025 -22.441  65.581  1.00 35.78      D000 C
ATOM   8197  N   ASP P 225      -7.132 -20.379  62.966  1.00 45.80      D000 N
ATOM   8198  CA  ASP P 225      -7.297 -20.628  61.535  1.00 47.35      D000 C
ATOM   8199  C   ASP P 225      -7.260 -19.343  60.721  1.00 44.18      D000 C
ATOM   8200  O   ASP P 225      -7.418 -19.380  59.496  1.00 41.75      D000 O
ATOM   8201  CB  ASP P 225      -8.590 -21.431  61.261  1.00 42.90      D000 C
ATOM   8202  CG  ASP P 225      -9.864 -20.593  61.367  1.00 45.62      D000 C
ATOM   8203  OD1 ASP P 225      -9.832 -19.473  61.933  1.00 42.32      D000 O
ATOM   8204  OD2 ASP P 225     -10.912 -21.079  60.878  1.00 49.08      D000 O1-
ATOM   8205  N   GLY P 226      -7.023 -18.215  61.373  1.00 42.84      D000 N
ATOM   8206  CA  GLY P 226      -6.919 -16.957  60.693  1.00 37.63      D000 C
ATOM   8207  C   GLY P 226      -8.168 -16.131  60.725  1.00 40.53      D000 C
ATOM   8208  O   GLY P 226      -8.089 -14.938  60.408  1.00 43.36      D000 O
ATOM   8209  N   THR P 227      -9.315 -16.725  61.089  1.00 42.48      D000 N
ATOM   8210  CA  THR P 227     -10.562 -15.973  61.156  1.00 39.26      D000 C
ATOM   8211  C   THR P 227     -10.348 -14.706  61.974  1.00 43.02      D000 C
ATOM   8212  O   THR P 227      -9.666 -14.722  63.005  1.00 41.15      D000 O
ATOM   8213  CB  THR P 227     -11.684 -16.819  61.760  1.00 38.82      D000 C
ATOM   8214  OG1 THR P 227     -11.803 -18.060  61.044  1.00 45.33      D000 O
ATOM   8215  CG2 THR P 227     -13.015 -16.065  61.694  1.00 30.94      D000 C
ATOM   8216  N   ASP P 228     -10.899 -13.600  61.485  1.00 35.97      D000 N
ATOM   8217  CA  ASP P 228     -10.647 -12.323  62.128  1.00 43.82      D000 C
ATOM   8218  C   ASP P 228     -11.342 -12.271  63.487  1.00 45.21      D000 C
ATOM   8219  O   ASP P 228     -12.534 -12.577  63.606  1.00 44.16      D000 O
ATOM   8220  CB  ASP P 228     -11.127 -11.167  61.243  1.00 38.78      D000 C
ATOM   8221  CG  ASP P 228     -10.940  -9.817  61.909  1.00 49.28      D000 C
ATOM   8222  OD1 ASP P 228      -9.775  -9.419  62.162  1.00 54.97      D000 O
ATOM   8223  OD2 ASP P 228     -11.958  -9.175  62.242  1.00 50.80      D000 O1-
ATOM   8224  N   TYR P 229     -10.601 -11.828  64.501  1.00 48.02      D000 N
ATOM   8225  CA  TYR P 229     -11.132 -11.730  65.857  1.00 49.96      D000 C
ATOM   8226  C   TYR P 229     -11.979 -10.481  66.041  1.00 49.04      D000 C
ATOM   8227  O   TYR P 229     -13.064 -10.540  66.628  1.00 53.87      D000 O
ATOM   8228  CB  TYR P 229      -9.995 -11.743  66.883  1.00 44.05      D000 C
ATOM   8229  CG  TYR P 229     -10.439 -11.358  68.269  1.00 45.49      D000 C
```

```
ATOM   8230  CD2 TYR P 229     -10.286 -10.061  68.725  1.00 53.43      D000 C
ATOM   8231  CD1 TYR P 229     -10.994 -12.296  69.132  1.00 44.65      D000 C
ATOM   8232  CE2 TYR P 229     -10.684  -9.695  69.997  1.00 56.58      D000 C
ATOM   8233  CE1 TYR P 229     -11.403 -11.944  70.406  1.00 46.74      D000 C
ATOM   8234  CZ  TYR P 229     -11.243 -10.638  70.838  1.00 52.93      D000 C
ATOM   8235  OH  TYR P 229     -11.634 -10.250  72.104  1.00 43.44      D000 O
ATOM   8236  N   GLU P 230     -11.503  -9.341  65.555  1.00 51.91      D000 N
ATOM   8237  CA  GLU P 230     -12.119  -8.087  65.968  1.00 56.16      D000 C
ATOM   8238  C   GLU P 230     -13.560  -7.995  65.476  1.00 54.64      D000 C
ATOM   8239  O   GLU P 230     -14.468  -7.629  66.236  1.00 52.29      D000 O
ATOM   8240  CB  GLU P 230     -11.292  -6.908  65.456  1.00 50.78      D000 C
ATOM   8241  CG  GLU P 230     -11.547  -5.640  66.240  1.00 59.32      D000 C
ATOM   8242  CD  GLU P 230     -11.610  -5.897  67.751  1.00 65.21      D000 C
ATOM   8243  OE1 GLU P 230     -10.556  -6.220  68.353  1.00 63.19      D000 O
ATOM   8244  OE2 GLU P 230     -12.718  -5.790  68.330  1.00 66.54      D000 O1-
ATOM   8245  N   THR P 231     -13.797  -8.382  64.231  1.00 49.30      D000 N
ATOM   8246  CA  THR P 231     -15.125  -8.335  63.651  1.00 49.06      D000 C
ATOM   8247  C   THR P 231     -15.943  -9.595  63.913  1.00 48.66      D000 C
ATOM   8248  O   THR P 231     -17.084  -9.685  63.450  1.00 46.72      D000 O
ATOM   8249  CB  THR P 231     -14.999  -8.096  62.145  1.00 51.91      D000 C
ATOM   8250  OG1 THR P 231     -14.418  -9.247  61.519  1.00 49.55      D000 O
ATOM   8251  CG2 THR P 231     -14.092  -6.893  61.895  1.00 44.25      D000 C
ATOM   8252  N   GLY P 232     -15.374 -10.596  64.593  1.00 53.28      D000 N
ATOM   8253  CA  GLY P 232     -16.057 -11.861  64.770  1.00 51.71      D000 C
ATOM   8254  C   GLY P 232     -16.832 -11.982  66.082  1.00 49.39      D000 C
ATOM   8255  O   GLY P 232     -16.750 -11.136  66.974  1.00 45.77      D000 O
ATOM   8256  N   PHE P 233     -17.569 -13.087  66.178  1.00 46.98      D000 N
ATOM   8257  CA  PHE P 233     -18.353 -13.412  67.362  1.00 45.41      D000 C
ATOM   8258  C   PHE P 233     -17.477 -13.478  68.610  1.00 43.09      D000 C
ATOM   8259  O   PHE P 233     -16.350 -13.978  68.572  1.00 40.14      D000 O
ATOM   8260  CB  PHE P 233     -19.050 -14.755  67.133  1.00 43.80      D000 C
ATOM   8261  CG  PHE P 233     -19.795 -15.261  68.318  1.00 41.20      D000 C
ATOM   8262  CD1 PHE P 233     -21.056 -14.778  68.620  1.00 42.87      D000 C
ATOM   8263  CD2 PHE P 233     -19.235 -16.234  69.130  1.00 41.22      D000 C
ATOM   8264  CE1 PHE P 233     -21.746 -15.250  69.718  1.00 43.07      D000 C
ATOM   8265  CE2 PHE P 233     -19.917 -16.712  70.232  1.00 42.28      D000 C
ATOM   8266  CZ  PHE P 233     -21.179 -16.221  70.526  1.00 42.72      D000 C
ATOM   8267  N   LYS P 234     -18.005 -12.976  69.728  1.00 42.82      D000 N
ATOM   8268  CA  LYS P 234     -17.292 -12.988  71.000  1.00 37.90      D000 C
ATOM   8269  C   LYS P 234     -18.268 -13.294  72.133  1.00 41.60      D000 C
ATOM   8270  O   LYS P 234     -19.440 -12.901  72.093  1.00 44.48      D000 O
ATOM   8271  CB  LYS P 234     -16.596 -11.656  71.279  1.00 35.56      D000 C
ATOM   8272  CG  LYS P 234     -15.570 -11.240  70.265  1.00 40.67      D000 C
ATOM   8273  CD  LYS P 234     -14.974  -9.900  70.655  1.00 45.48      D000 C
ATOM   8274  CE  LYS P 234     -14.396  -9.152  69.453  1.00 51.18      D000 C
ATOM   8275  NZ  LYS P 234     -15.432  -8.815  68.436  1.00 50.83      D000 N1+
ATOM   8276  N   ASN P 235     -17.770 -13.978  73.161  1.00 40.00      D000 N
ATOM   8277  CA  ASN P 235     -18.619 -14.402  74.273  1.00 37.71      D000 C
ATOM   8278  C   ASN P 235     -17.822 -14.433  75.583  1.00 37.90      D000 C
ATOM   8279  O   ASN P 235     -17.903 -15.379  76.370  1.00 37.72      D000 O
ATOM   8280  CB  ASN P 235     -19.249 -15.757  73.947  1.00 35.17      D000 C
ATOM   8281  CG  ASN P 235     -20.286 -16.187  74.963  1.00 40.71      D000 C
ATOM   8282  OD1 ASN P 235     -20.393 -17.373  75.280  1.00 40.09      D000 O
ATOM   8283  ND2 ASN P 235     -21.034 -15.228  75.504  1.00 41.85      D000 N
ATOM   8284  N   TRP P 236     -17.096 -13.353  75.867  1.00 39.79      D000 N
ATOM   8285  CA  TRP P 236     -16.261 -13.296  77.062  1.00 46.37      D000 C
ATOM   8286  C   TRP P 236     -17.096 -13.235  78.337  1.00 41.70      D000 C
ATOM   8287  O   TRP P 236     -18.198 -12.687  78.359  1.00 49.28      D000 O
ATOM   8288  CB  TRP P 236     -15.335 -12.082  77.009  1.00 45.14      D000 C
ATOM   8289  CG  TRP P 236     -14.278 -12.168  75.954  1.00 47.98      D000 C
ATOM   8290  CD1 TRP P 236     -14.263 -11.524  74.742  1.00 45.52      D000 C
ATOM   8291  CD2 TRP P 236     -13.076 -12.948  76.010  1.00 46.16      D000 C
ATOM   8292  NE1 TRP P 236     -13.122 -11.854  74.047  1.00 47.86      D000 N
ATOM   8293  CE2 TRP P 236     -12.377 -12.728  74.800  1.00 48.95      D000 C
ATOM   8294  CE3 TRP P 236     -12.524 -13.813  76.961  1.00 44.69      D000 C
ATOM   8295  CZ2 TRP P 236     -11.155 -13.347  74.517  1.00 41.16      D000 C
ATOM   8296  CZ3 TRP P 236     -11.303 -14.427  76.679  1.00 46.91      D000 C
ATOM   8297  CH2 TRP P 236     -10.630 -14.180  75.469  1.00 44.61      D000 C
ATOM   8298  N   ARG P 237     -16.554 -13.787  79.415  1.00 41.38      D000 N
ATOM   8299  CA  ARG P 237     -17.130 -13.493  80.718  1.00 53.87      D000 C
ATOM   8300  C   ARG P 237     -16.928 -12.007  80.992  1.00 61.28      D000 C
```

| ATOM | 8301 | O   | ARG | P | 237 | -15.886 | -11.452 | 80.630 | 1.00 | 66.27 | D000 | O   |
|------|------|-----|-----|---|-----|---------|---------|--------|------|-------|------|-----|
| ATOM | 8302 | CB  | ARG | P | 237 | -16.478 | -14.322 | 81.833 | 1.00 | 53.42 | D000 | C   |
| ATOM | 8303 | CG  | ARG | P | 237 | -16.800 | -15.823 | 81.816 | 1.00 | 47.57 | D000 | C   |
| ATOM | 8304 | CD  | ARG | P | 237 | -18.062 | -16.175 | 82.614 | 1.00 | 51.09 | D000 | C   |
| ATOM | 8305 | NE  | ARG | P | 237 | -17.828 | -16.314 | 84.057 | 1.00 | 58.37 | D000 | N   |
| ATOM | 8306 | CZ  | ARG | P | 237 | -17.838 | -17.466 | 84.735 | 1.00 | 56.45 | D000 | C   |
| ATOM | 8307 | NH1 | ARG | P | 237 | -18.083 | -18.621 | 84.123 | 1.00 | 48.82 | D000 | N1+ |
| ATOM | 8308 | NH2 | ARG | P | 237 | -17.612 | -17.462 | 86.046 | 1.00 | 63.83 | D000 | N   |
| ATOM | 8309 | N   | PRO | P | 238 | -17.893 | -11.331 | 81.605 | 1.00 | 62.76 | D000 | N   |
| ATOM | 8310 | CA  | PRO | P | 238 | -17.707 | -9.908  | 81.907 | 1.00 | 69.38 | D000 | C   |
| ATOM | 8311 | C   | PRO | P | 238 | -16.409 | -9.654  | 82.665 | 1.00 | 74.57 | D000 | C   |
| ATOM | 8312 | O   | PRO | P | 238 | -16.040 | -10.408 | 83.571 | 1.00 | 71.66 | D000 | O   |
| ATOM | 8313 | CB  | PRO | P | 238 | -18.942 | -9.573  | 82.746 | 1.00 | 64.98 | D000 | C   |
| ATOM | 8314 | CG  | PRO | P | 238 | -19.987 | -10.474 | 82.171 | 1.00 | 64.63 | D000 | C   |
| ATOM | 8315 | CD  | PRO | P | 238 | -19.285 | -11.760 | 81.814 | 1.00 | 57.83 | D000 | C   |
| ATOM | 8316 | N   | GLU | P | 239 | -15.705 | -8.593  | 82.248 | 1.00 | 76.88 | D000 | N   |
| ATOM | 8317 | CA  | GLU | P | 239 | -14.381 | -8.126  | 82.688 | 1.00 | 83.12 | D000 | C   |
| ATOM | 8318 | C   | GLU | P | 239 | -13.208 | -8.804  | 81.960 | 1.00 | 80.80 | D000 | C   |
| ATOM | 8319 | O   | GLU | P | 239 | -12.049 | -8.510  | 82.297 | 1.00 | 84.63 | D000 | O   |
| ATOM | 8320 | CB  | GLU | P | 239 | -14.170 | -8.253  | 84.207 | 1.00 | 83.81 | D000 | C   |
| ATOM | 8321 | CG  | GLU | P | 239 | -14.580 | -6.991  | 84.959 | 1.00 | 91.55 | D000 | C   |
| ATOM | 8322 | CD  | GLU | P | 239 | -13.794 | -6.763  | 86.239 | 1.00 | 97.32 | D000 | C   |
| ATOM | 8323 | OE1 | GLU | P | 239 | -14.226 | -7.250  | 87.308 | 1.00 | 93.15 | D000 | O   |
| ATOM | 8324 | OE2 | GLU | P | 239 | -12.745 | -6.086  | 86.172 | 1.00 | 96.55 | D000 | O1- |
| ATOM | 8325 | N   | GLN | P | 240 | -13.452 | -9.674  | 80.980 | 1.00 | 74.55 | D000 | N   |
| ATOM | 8326 | CA  | GLN | P | 240 | -12.388 | -10.436 | 80.304 | 1.00 | 70.87 | D000 | C   |
| ATOM | 8327 | C   | GLN | P | 240 | -12.327 | -10.058 | 78.811 | 1.00 | 70.85 | D000 | C   |
| ATOM | 8328 | O   | GLN | P | 240 | -13.346 | -9.637  | 78.253 | 1.00 | 75.89 | D000 | O   |
| ATOM | 8329 | CB  | GLN | P | 240 | -12.619 | -11.949 | 80.462 | 1.00 | 68.58 | D000 | C   |
| ATOM | 8330 | CG  | GLN | P | 240 | -12.717 | -12.433 | 81.899 | 1.00 | 68.41 | D000 | C   |
| ATOM | 8331 | CD  | GLN | P | 240 | -11.414 | -12.251 | 82.660 | 1.00 | 75.96 | D000 | C   |
| ATOM | 8332 | OE1 | GLN | P | 240 | -10.466 | -13.018 | 82.480 | 1.00 | 70.60 | D000 | O   |
| ATOM | 8333 | NE2 | GLN | P | 240 | -11.359 | -11.226 | 83.518 | 1.00 | 77.27 | D000 | N   |
| ATOM | 8334 | N   | PRO | P | 241 | -11.147 | -10.193 | 78.150 | 1.00 | 69.18 | D000 | N   |
| ATOM | 8335 | CA  | PRO | P | 241 | -9.849  | -10.736 | 78.592 | 1.00 | 66.74 | D000 | C   |
| ATOM | 8336 | C   | PRO | P | 241 | -9.056  | -9.841  | 79.559 | 1.00 | 73.69 | D000 | C   |
| ATOM | 8337 | O   | PRO | P | 241 | -9.074  | -8.611  | 79.454 | 1.00 | 81.53 | D000 | O   |
| ATOM | 8338 | CB  | PRO | P | 241 | -9.088  | -10.907 | 77.273 | 1.00 | 58.34 | D000 | C   |
| ATOM | 8339 | CG  | PRO | P | 241 | -9.646  | -9.842  | 76.388 | 1.00 | 54.80 | D000 | C   |
| ATOM | 8340 | CD  | PRO | P | 241 | -11.106 | -9.787  | 76.727 | 1.00 | 57.51 | D000 | C   |
| ATOM | 8341 | N   | GLU | P | 253 | -1.712  | -15.376 | 81.188 | 1.00 | 57.88 | D000 | N   |
| ATOM | 8342 | CA  | GLU | P | 253 | -3.063  | -15.516 | 80.648 | 1.00 | 58.93 | D000 | C   |
| ATOM | 8343 | C   | GLU | P | 253 | -3.184  | -15.191 | 79.148 | 1.00 | 57.34 | D000 | C   |
| ATOM | 8344 | O   | GLU | P | 253 | -3.809  | -14.204 | 78.771 | 1.00 | 60.57 | D000 | O   |
| ATOM | 8345 | CB  | GLU | P | 253 | -4.028  | -14.606 | 81.410 | 1.00 | 67.05 | D000 | C   |
| ATOM | 8346 | CG  | GLU | P | 253 | -4.239  | -14.925 | 82.886 | 1.00 | 65.01 | D000 | C   |
| ATOM | 8347 | CD  | GLU | P | 253 | -4.871  | -13.746 | 83.619 | 1.00 | 78.11 | D000 | C   |
| ATOM | 8348 | OE1 | GLU | P | 253 | -6.039  | -13.406 | 83.290 | 1.00 | 74.19 | D000 | O   |
| ATOM | 8349 | OE2 | GLU | P | 253 | -4.210  | -13.174 | 84.525 | 1.00 | 80.72 | D000 | O1- |
| ATOM | 8350 | N   | ASP | P | 254 | -2.582  | -16.015 | 78.297 | 1.00 | 59.04 | D000 | N   |
| ATOM | 8351 | CA  | ASP | P | 254 | -2.582  | -15.774 | 76.863 | 1.00 | 57.72 | D000 | C   |
| ATOM | 8352 | C   | ASP | P | 254 | -3.509  | -16.705 | 76.087 | 1.00 | 51.09 | D000 | C   |
| ATOM | 8353 | O   | ASP | P | 254 | -3.528  | -16.640 | 74.857 | 1.00 | 48.82 | D000 | O   |
| ATOM | 8354 | CB  | ASP | P | 254 | -1.159  | -15.911 | 76.296 | 1.00 | 61.92 | D000 | C   |
| ATOM | 8355 | CG  | ASP | P | 254 | -0.161  | -14.973 | 76.935 | 1.00 | 62.66 | D000 | C   |
| ATOM | 8356 | OD1 | ASP | P | 254 | -0.567  | -13.960 | 77.547 | 1.00 | 61.96 | D000 | O   |
| ATOM | 8357 | OD2 | ASP | P | 254 | 1.048   | -15.281 | 76.834 | 1.00 | 65.49 | D000 | O1- |
| ATOM | 8358 | N   | CYS | P | 255 | -4.265  | -17.574 | 76.753 | 1.00 | 47.05 | D000 | N   |
| ATOM | 8359 | CA  | CYS | P | 255 | -5.063  | -18.571 | 76.051 | 1.00 | 41.26 | D000 | C   |
| ATOM | 8360 | C   | CYS | P | 255 | -6.488  | -18.590 | 76.580 | 1.00 | 38.11 | D000 | C   |
| ATOM | 8361 | O   | CYS | P | 255 | -6.716  | -18.454 | 77.783 | 1.00 | 39.54 | D000 | O   |
| ATOM | 8362 | CB  | CYS | P | 255 | -4.450  | -19.956 | 76.186 | 1.00 | 44.58 | D000 | C   |
| ATOM | 8363 | SG  | CYS | P | 255 | -2.846  | -20.164 | 75.353 | 1.00 | 52.24 | D000 | S   |
| ATOM | 8364 | N   | ALA | P | 256 | -7.440  | -18.776 | 75.674 | 1.00 | 36.50 | D000 | N   |
| ATOM | 8365 | CA  | ALA | P | 256 | -8.857  | -18.645 | 75.980 | 1.00 | 35.39 | D000 | C   |
| ATOM | 8366 | C   | ALA | P | 256 | -9.527  | -20.010 | 76.077 | 1.00 | 31.26 | D000 | C   |
| ATOM | 8367 | O   | ALA | P | 256 | -9.240  | -20.914 | 75.281 | 1.00 | 30.62 | D000 | O   |
| ATOM | 8368 | CB  | ALA | P | 256 | -9.555  | -17.793 | 74.921 | 1.00 | 38.76 | D000 | C   |
| ATOM | 8369 | N   | HIS | P | 257 | -10.412 | -20.157 | 77.066 | 1.00 | 30.17 | D000 | N   |
| ATOM | 8370 | CA  | HIS | P | 257 | -11.139 | -21.406 | 77.272 | 1.00 | 32.66 | D000 | C   |
| ATOM | 8371 | C   | HIS | P | 257 | -12.600 | -21.131 | 77.611 | 1.00 | 33.59 | D000 | C   |

```
ATOM   8372  O    HIS P 257     -12.962 -20.057  78.108  1.00 31.39      D000 O
ATOM   8373  CB   HIS P 257     -10.517 -22.263  78.384  1.00 28.03      D000 C
ATOM   8374  CG   HIS P 257     -10.526 -21.604  79.727  1.00 34.24      D000 C
ATOM   8375  ND1  HIS P 257     -11.562 -21.751  80.621  1.00 34.37      D000 N
ATOM   8376  CD2  HIS P 257      -9.620 -20.802  80.335  1.00 38.40      D000 C
ATOM   8377  CE1  HIS P 257     -11.298 -21.068  81.718  1.00 34.64      D000 C
ATOM   8378  NE2  HIS P 257     -10.128 -20.479  81.569  1.00 37.12      D000 N
ATOM   8379  N    PHE P 258     -13.444 -22.124  77.309  1.00 33.23      D000 N
ATOM   8380  CA   PHE P 258     -14.808 -22.141  77.823  1.00 32.25      D000 C
ATOM   8381  C    PHE P 258     -14.773 -22.392  79.319  1.00 32.26      D000 C
ATOM   8382  O    PHE P 258     -13.956 -23.171  79.811  1.00 29.18      D000 O
ATOM   8383  CB   PHE P 258     -15.652 -23.232  77.153  1.00 30.58      D000 C
ATOM   8384  CG   PHE P 258     -15.651 -23.174  75.659  1.00 27.36      D000 C
ATOM   8385  CD1  PHE P 258     -16.550 -22.394  74.987  1.00 28.97      D000 C
ATOM   8386  CD2  PHE P 258     -14.738 -23.912  74.931  1.00 28.24      D000 C
ATOM   8387  CE1  PHE P 258     -16.541 -22.343  73.614  1.00 34.71      D000 C
ATOM   8388  CE2  PHE P 258     -14.723 -23.869  73.566  1.00 30.59      D000 C
ATOM   8389  CZ   PHE P 258     -15.623 -23.079  72.902  1.00 33.75      D000 C
ATOM   8390  N    THR P 259     -15.684 -21.742  80.033  1.00 36.07      D000 N
ATOM   8391  CA   THR P 259     -15.852 -21.887  81.471  1.00 37.54      D000 C
ATOM   8392  C    THR P 259     -17.106 -22.709  81.741  1.00 38.73      D000 C
ATOM   8393  O    THR P 259     -17.882 -23.018  80.828  1.00 40.70      D000 O
ATOM   8394  CB   THR P 259     -15.949 -20.510  82.148  1.00 41.98      D000 C
ATOM   8395  OG1  THR P 259     -17.200 -19.888  81.813  1.00 41.80      D000 O
ATOM   8396  CG2  THR P 259     -14.799 -19.598  81.696  1.00 30.57      D000 C
ATOM   8397  N    ASP P 260     -17.342 -23.006  83.020  1.00 37.73      D000 N
ATOM   8398  CA   ASP P 260     -18.446 -23.894  83.380  1.00 37.79      D000 C
ATOM   8399  C    ASP P 260     -19.814 -23.404  82.896  1.00 38.90      D000 C
ATOM   8400  O    ASP P 260     -20.758 -24.201  82.869  1.00 41.70      D000 O
ATOM   8401  CB   ASP P 260     -18.483 -24.128  84.901  1.00 35.64      D000 C
ATOM   8402  CG   ASP P 260     -18.619 -22.834  85.712  1.00 42.82      D000 C
ATOM   8403  OD1  ASP P 260     -18.241 -21.740  85.225  1.00 48.13      D000 O
ATOM   8404  OD2  ASP P 260     -19.161 -22.907  86.836  1.00 44.48      D000 O1-
ATOM   8405  N    ASP P 261     -19.955 -22.136  82.499  1.00 41.54      D000 N
ATOM   8406  CA   ASP P 261     -21.226 -21.648  81.973  1.00 42.82      D000 C
ATOM   8407  C    ASP P 261     -21.155 -21.336  80.487  1.00 41.84      D000 C
ATOM   8408  O    ASP P 261     -22.099 -20.769  79.939  1.00 43.69      D000 O
ATOM   8409  CB   ASP P 261     -21.727 -20.412  82.738  1.00 43.58      D000 C
ATOM   8410  CG   ASP P 261     -20.950 -19.119  82.416  1.00 49.58      D000 C
ATOM   8411  OD1  ASP P 261     -19.993 -19.120  81.605  1.00 52.04      D000 O
ATOM   8412  OD2  ASP P 261     -21.333 -18.065  82.974  1.00 48.73      D000 O1-
ATOM   8413  N    GLY P 262     -20.046 -21.659  79.826  1.00 41.79      D000 N
ATOM   8414  CA   GLY P 262     -19.923 -21.469  78.406  1.00 35.80      D000 C
ATOM   8415  C    GLY P 262     -19.239 -20.181  78.005  1.00 34.51      D000 C
ATOM   8416  O    GLY P 262     -18.649 -20.125  76.926  1.00 35.57      D000 O
ATOM   8417  N    ARG P 263     -19.315 -19.141  78.829  1.00 35.18      D000 N
ATOM   8418  CA   ARG P 263     -18.644 -17.901  78.475  1.00 39.45      D000 C
ATOM   8419  C    ARG P 263     -17.120 -18.052  78.581  1.00 36.67      D000 C
ATOM   8420  O    ARG P 263     -16.588 -18.949  79.235  1.00 34.59      D000 O
ATOM   8421  CB   ARG P 263     -19.177 -16.755  79.332  1.00 39.46      D000 C
ATOM   8422  CG   ARG P 263     -20.633 -16.427  79.008  1.00 41.29      D000 C
ATOM   8423  CD   ARG P 263     -21.171 -15.300  79.852  1.00 41.58      D000 C
ATOM   8424  NE   ARG P 263     -21.060 -15.629  81.269  1.00 57.11      D000 N
ATOM   8425  CZ   ARG P 263     -21.316 -14.794  82.274  1.00 54.20      D000 C
ATOM   8426  NH1  ARG P 263     -21.702 -13.543  82.039  1.00 46.67      D000 N1+
ATOM   8427  NH2  ARG P 263     -21.168 -15.216  83.524  1.00 49.65      D000 N
ATOM   8428  N    TRP P 264     -16.414 -17.184  77.879  1.00 39.86      D000 N
ATOM   8429  CA   TRP P 264     -14.984 -17.349  77.652  1.00 38.07      D000 C
ATOM   8430  C    TRP P 264     -14.144 -16.707  78.750  1.00 36.92      D000 C
ATOM   8431  O    TRP P 264     -14.565 -15.769  79.434  1.00 38.51      D000 O
ATOM   8432  CB   TRP P 264     -14.596 -16.743  76.311  1.00 36.59      D000 C
ATOM   8433  CG   TRP P 264     -15.334 -17.316  75.157  1.00 33.59      D000 C
ATOM   8434  CD1  TRP P 264     -16.124 -18.422  75.156  1.00 28.12      D000 C
ATOM   8435  CD2  TRP P 264     -15.343 -16.808  73.823  1.00 37.35      D000 C
ATOM   8436  NE1  TRP P 264     -16.627 -18.635  73.909  1.00 31.66      D000 N
ATOM   8437  CE2  TRP P 264     -16.158 -17.661  73.064  1.00 38.06      D000 C
ATOM   8438  CE3  TRP P 264     -14.736 -15.714  73.194  1.00 34.20      D000 C
ATOM   8439  CZ2  TRP P 264     -16.384 -17.455  71.697  1.00 36.24      D000 C
ATOM   8440  CZ3  TRP P 264     -14.966 -15.510  71.854  1.00 34.62      D000 C
ATOM   8441  CH2  TRP P 264     -15.783 -16.375  71.118  1.00 35.86      D000 C
ATOM   8442  N    ASN P 265     -12.908 -17.179  78.854  1.00 32.60      D000 N
```

```
ATOM   8443  CA   ASN P 265    -11.991 -16.649  79.844  1.00 35.02      D000 C
ATOM   8444  C    ASN P 265    -10.562 -16.955  79.416  1.00 35.40      D000 C
ATOM   8445  O    ASN P 265    -10.303 -17.966  78.762  1.00 34.37      D000 O
ATOM   8446  CB   ASN P 265    -12.302 -17.268  81.206  1.00 41.49      D000 C
ATOM   8447  CG   ASN P 265    -11.391 -16.785  82.295  1.00 45.67      D000 C
ATOM   8448  OD1  ASN P 265    -11.470 -15.634  82.725  1.00 46.99      D000 O
ATOM   8449  ND2  ASN P 265    -10.486 -17.660  82.730  1.00 42.25      D000 N
ATOM   8450  N    ASP P 266     -9.635 -16.081  79.804  1.00 41.02      D000 N
ATOM   8451  CA   ASP P 266     -8.210 -16.284  79.555  1.00 44.53      D000 C
ATOM   8452  C    ASP P 266     -7.533 -16.874  80.790  1.00 44.46      D000 C
ATOM   8453  O    ASP P 266     -7.836 -16.493  81.922  1.00 42.76      D000 O
ATOM   8454  CB   ASP P 266     -7.513 -14.977  79.143  1.00 48.13      D000 C
ATOM   8455  CG   ASP P 266     -7.889 -13.788  80.036  1.00 60.01      D000 C
ATOM   8456  OD1  ASP P 266     -9.096 -13.510  80.179  1.00 60.58      D000 O
ATOM   8457  OD2  ASP P 266     -6.988 -13.158  80.633  1.00 64.47      D000 O1-
ATOM   8458  N    ASP P 267     -6.622 -17.815  80.563  1.00 44.24      D000 N
ATOM   8459  CA   ASP P 267     -5.909 -18.469  81.647  1.00 42.61      D000 C
ATOM   8460  C    ASP P 267     -4.508 -18.821  81.152  1.00 44.14      D000 C
ATOM   8461  O    ASP P 267     -4.233 -18.787  79.952  1.00 41.89      D000 O
ATOM   8462  CB   ASP P 267     -6.688 -19.697  82.122  1.00 38.52      D000 C
ATOM   8463  CG   ASP P 267     -6.223 -20.188  83.464  1.00 45.95      D000 C
ATOM   8464  OD1  ASP P 267     -5.365 -19.510  84.068  1.00 48.74      D000 O
ATOM   8465  OD2  ASP P 267     -6.710 -21.244  83.919  1.00 43.95      D000 O1-
ATOM   8466  N    VAL P 268     -3.612 -19.180  82.076  1.00 47.41      D000 N
ATOM   8467  CA   VAL P 268     -2.258 -19.507  81.643  1.00 46.28      D000 C
ATOM   8468  C    VAL P 268     -2.306 -20.765  80.793  1.00 45.81      D000 C
ATOM   8469  O    VAL P 268     -3.051 -21.717  81.079  1.00 42.24      D000 O
ATOM   8470  CB   VAL P 268     -1.274 -19.643  82.822  1.00 45.28      D000 C
ATOM   8471  CG1  VAL P 268     -1.359 -18.423  83.707  1.00 40.04      D000 C
ATOM   8472  CG2  VAL P 268     -1.489 -20.937  83.609  1.00 40.72      D000 C
ATOM   8473  N    CYS P 269     -1.525 -20.756  79.717  1.00 45.30      D000 N
ATOM   8474  CA   CYS P 269     -1.620 -21.777  78.695  1.00 34.95      D000 C
ATOM   8475  C    CYS P 269     -1.113 -23.121  79.167  1.00 39.62      D000 C
ATOM   8476  O    CYS P 269     -1.267 -24.107  78.438  1.00 44.44      D000 O
ATOM   8477  CB   CYS P 269     -0.849 -21.317  77.456  1.00 47.03      D000 C
ATOM   8478  SG   CYS P 269     -1.435 -19.698  76.766  1.00 60.08      D000 S
ATOM   8479  N    GLN P 270     -0.551 -23.199  80.371  1.00 40.97      D000 N
ATOM   8480  CA   GLN P 270      0.007 -24.461  80.837  1.00 39.29      D000 C
ATOM   8481  C    GLN P 270     -1.038 -25.378  81.448  1.00 33.15      D000 C
ATOM   8482  O    GLN P 270     -0.769 -26.577  81.579  1.00 30.99      D000 O
ATOM   8483  CB   GLN P 270      1.128 -24.207  81.857  1.00 52.36      D000 C
ATOM   8484  CG   GLN P 270      2.531 -23.978  81.256  1.00 68.27      D000 C
ATOM   8485  CD   GLN P 270      3.672 -24.458  82.188  1.00 90.18      D000 C
ATOM   8486  OE1  GLN P 270      3.426 -25.009  83.272  1.00 96.23      D000 O
ATOM   8487  NE2  GLN P 270      4.921 -24.250  81.760  1.00 83.33      D000 N
ATOM   8488  N    ARG P 271     -2.213 -24.847  81.803  1.00 35.97      D000 N
ATOM   8489  CA   ARG P 271     -3.258 -25.641  82.453  1.00 35.38      D000 C
ATOM   8490  C    ARG P 271     -3.614 -26.831  81.577  1.00 34.50      D000 C
ATOM   8491  O    ARG P 271     -3.835 -26.657  80.369  1.00 35.09      D000 O
ATOM   8492  CB   ARG P 271     -4.533 -24.825  82.699  1.00 32.78      D000 C
ATOM   8493  CG   ARG P 271     -4.404 -23.700  83.663  1.00 35.79      D000 C
ATOM   8494  CD   ARG P 271     -4.885 -24.052  85.062  1.00 38.85      D000 C
ATOM   8495  NE   ARG P 271     -4.351 -23.079  86.026  1.00 53.17      D000 N
ATOM   8496  CZ   ARG P 271     -3.487 -23.363  87.009  1.00 57.60      D000 C
ATOM   8497  NH1  ARG P 271     -3.061 -24.616  87.211  1.00 48.15      D000 N1+
ATOM   8498  NH2  ARG P 271     -3.054 -22.387  87.805  1.00 54.98      D000 N
ATOM   8499  N    PRO P 272     -3.709 -27.959  82.102  1.00 34.18      D000 N
ATOM   8500  CA   PRO P 272     -4.104 -29.152  81.304  1.00 30.39      D000 C
ATOM   8501  C    PRO P 272     -5.617 -29.326  81.191  1.00 30.82      D000 C
ATOM   8502  O    PRO P 272     -6.222 -30.227  81.774  1.00 30.88      D000 O
ATOM   8503  CB   PRO P 272     -3.446 -30.296  82.079  1.00 29.98      D000 C
ATOM   8504  CG   PRO P 272     -3.279 -29.776  83.527  1.00 33.19      D000 C
ATOM   8505  CD   PRO P 272     -3.502 -28.269  83.527  1.00 30.33      D000 C
ATOM   8506  N    TYR P 273     -6.260 -28.438  80.435  1.00 29.69      D000 N
ATOM   8507  CA   TYR P 273     -7.685 -28.556  80.183  1.00 26.06      D000 C
ATOM   8508  C    TYR P 273     -7.957 -29.510  79.021  1.00 31.32      D000 C
ATOM   8509  O    TYR P 273     -7.075 -29.834  78.220  1.00 34.09      D000 O
ATOM   8510  CB   TYR P 273     -8.289 -27.199  79.848  1.00 29.36      D000 C
ATOM   8511  CG   TYR P 273     -8.313 -26.189  80.968  1.00 31.45      D000 C
ATOM   8512  CD1  TYR P 273     -8.404 -26.586  82.310  1.00 29.10      D000 C
ATOM   8513  CD2  TYR P 273     -8.243 -24.832  80.685  1.00 26.14      D000 C
```

```
ATOM   8514  CE1 TYR P 273     -8.422 -25.650  83.325  1.00 23.99     D000 C
ATOM   8515  CE2 TYR P 273     -8.275 -23.900  81.688  1.00 30.08     D000 C
ATOM   8516  CZ  TYR P 273     -8.364 -24.312  83.001  1.00 27.73     D000 C
ATOM   8517  OH  TYR P 273     -8.380 -23.357  83.973  1.00 30.77     D000 O
ATOM   8518  N   ARG P 274     -9.208 -29.949  78.931  1.00 28.38     D000 N
ATOM   8519  CA  ARG P 274     -9.699 -30.608  77.733  1.00 26.38     D000 C
ATOM   8520  C   ARG P 274     -9.755 -29.604  76.570  1.00 28.97     D000 C
ATOM   8521  O   ARG P 274     -9.693 -28.383  76.762  1.00 29.05     D000 O
ATOM   8522  CB  ARG P 274    -11.090 -31.183  78.003  1.00 26.93     D000 C
ATOM   8523  CG  ARG P 274    -11.108 -32.298  78.990  1.00 27.16     D000 C
ATOM   8524  CD  ARG P 274    -12.512 -32.728  79.284  1.00 29.23     D000 C
ATOM   8525  NE  ARG P 274    -12.531 -33.815  80.262  1.00 31.79     D000 N
ATOM   8526  CZ  ARG P 274    -13.610 -34.234  80.917  1.00 28.61     D000 C
ATOM   8527  NH1 ARG P 274    -14.801 -33.662  80.721  1.00 27.83     D000 N1+
ATOM   8528  NH2 ARG P 274    -13.495 -35.241  81.767  1.00 30.68     D000 N
ATOM   8529  N   TRP P 275     -9.894 -30.113  75.345  1.00 23.49     D000 N
ATOM   8530  CA  TRP P 275     -9.938 -29.226  74.188  1.00 25.18     D000 C
ATOM   8531  C   TRP P 275    -10.855 -29.837  73.136  1.00 28.35     D000 C
ATOM   8532  O   TRP P 275    -11.240 -31.009  73.214  1.00 27.63     D000 O
ATOM   8533  CB  TRP P 275     -8.524 -28.953  73.614  1.00 26.97     D000 C
ATOM   8534  CG  TRP P 275     -7.949 -30.164  72.960  1.00 26.87     D000 C
ATOM   8535  CD1 TRP P 275     -7.973 -30.473  71.628  1.00 25.57     D000 C
ATOM   8536  CD2 TRP P 275     -7.346 -31.284  73.625  1.00 26.80     D000 C
ATOM   8537  NE1 TRP P 275     -7.403 -31.713  71.419  1.00 26.56     D000 N
ATOM   8538  CE2 TRP P 275     -7.008 -32.230  72.626  1.00 28.11     D000 C
ATOM   8539  CE3 TRP P 275     -7.044 -31.573  74.966  1.00 24.47     D000 C
ATOM   8540  CZ2 TRP P 275     -6.388 -33.439  72.926  1.00 26.32     D000 C
ATOM   8541  CZ3 TRP P 275     -6.439 -32.772  75.265  1.00 25.01     D000 C
ATOM   8542  CH2 TRP P 275     -6.114 -33.692  74.250  1.00 29.39     D000 C
ATOM   8543  N   VAL P 276    -11.186 -29.023  72.135  1.00 27.49     D000 N
ATOM   8544  CA  VAL P 276    -12.064 -29.405  71.039  1.00 29.28     D000 C
ATOM   8545  C   VAL P 276    -11.347 -29.157  69.720  1.00 32.15     D000 C
ATOM   8546  O   VAL P 276    -11.010 -28.009  69.399  1.00 30.70     D000 O
ATOM   8547  CB  VAL P 276    -13.381 -28.615  71.076  1.00 32.24     D000 C
ATOM   8548  CG1 VAL P 276    -14.255 -28.997  69.881  1.00 28.45     D000 C
ATOM   8549  CG2 VAL P 276    -14.099 -28.838  72.418  1.00 27.44     D000 C
ATOM   8550  N   CYS P 277    -11.188 -30.212  68.926  1.00 31.02     D000 N
ATOM   8551  CA  CYS P 277    -10.688 -30.089  67.560  1.00 33.70     D000 C
ATOM   8552  C   CYS P 277    -11.856 -29.883  66.594  1.00 36.60     D000 C
ATOM   8553  O   CYS P 277    -12.924 -30.476  66.769  1.00 35.91     D000 O
ATOM   8554  CB  CYS P 277     -9.893 -31.339  67.158  1.00 33.14     D000 C
ATOM   8555  SG  CYS P 277     -8.261 -31.627  67.947  1.00 35.59     D000 S
ATOM   8556  N   GLU P 278    -11.644 -29.037  65.572  1.00 36.89     D000 N
ATOM   8557  CA  GLU P 278    -12.627 -28.753  64.525  1.00 39.14     D000 C
ATOM   8558  C   GLU P 278    -11.998 -28.940  63.144  1.00 44.48     D000 C
ATOM   8559  O   GLU P 278    -10.812 -28.657  62.944  1.00 39.34     D000 O
ATOM   8560  CB  GLU P 278    -13.201 -27.309  64.639  1.00 33.16     D000 C
ATOM   8561  CG  GLU P 278    -14.353 -26.998  63.675  1.00 36.59     D000 C
ATOM   8562  CD  GLU P 278    -14.820 -25.530  63.719  1.00 44.66     D000 C
ATOM   8563  OE1 GLU P 278    -14.202 -24.714  64.445  1.00 39.06     D000 O
ATOM   8564  OE2 GLU P 278    -15.829 -25.200  63.049  1.00 41.28     D000 O1-
ATOM   8565  N   THR P 279    -12.790 -29.443  62.197  1.00 50.44     D000 N
ATOM   8566  CA  THR P 279    -12.402 -29.459  60.790  1.00 53.12     D000 C
ATOM   8567  C   THR P 279    -13.617 -29.195  59.898  1.00 49.51     D000 C
ATOM   8568  O   THR P 279    -14.703 -29.717  60.154  1.00 53.00     D000 O
ATOM   8569  CB  THR P 279    -11.717 -30.798  60.436  1.00 45.79     D000 C
ATOM   8570  OG1 THR P 279    -11.049 -30.673  59.179  1.00 51.68     D000 O
ATOM   8571  CG2 THR P 279    -12.709 -31.937  60.373  1.00 41.88     D000 C
ATOM   8572  N   GLU P 280    -13.431 -28.398  58.839  1.00 54.11     D000 N
ATOM   8573  CA  GLU P 280    -14.518 -28.026  57.929  1.00 55.44     D000 C
ATOM   8574  C   GLU P 280    -14.664 -29.061  56.808  1.00 60.78     D000 C
ATOM   8575  O   GLU P 280    -13.673 -29.610  56.323  1.00 66.29     D000 O
ATOM   8576  CB  GLU P 280    -14.275 -26.632  57.350  1.00 58.37     D000 C
ATOM   8577  CG  GLU P 280    -14.341 -25.476  58.376  1.00 56.42     D000 C
ATOM   8578  CD  GLU P 280    -13.124 -25.396  59.315  1.00 59.60     D000 C
ATOM   8579  OE1 GLU P 280    -12.166 -26.179  59.134  1.00 63.77     D000 O
ATOM   8580  OE2 GLU P 280    -13.129 -24.553  60.247  1.00 61.91     D000 O1-
ATOM   8581  N   LEU P 281    -15.911 -29.308  56.389  1.00 64.56     D000 N
ATOM   8582  CA  LEU P 281    -16.292 -30.535  55.682  1.00 69.60     D000 C
ATOM   8583  C   LEU P 281    -16.310 -30.448  54.149  1.00 73.85     D000 C
ATOM   8584  O   LEU P 281    -16.945 -31.306  53.521  1.00 77.45     D000 O
```

```
ATOM   8585  CB   LEU P 281      -17.662 -31.015  56.164  1.00 60.03           D000 C
ATOM   8586  CG   LEU P 281      -17.690 -32.374  56.863  1.00 56.08           D000 C
ATOM   8587  CD1  LEU P 281      -19.116 -32.776  57.171  1.00 66.41           D000 C
ATOM   8588  CD2  LEU P 281      -17.006 -33.448  56.066  1.00 58.00           D000 C
ATOM   8589  N    ASP P 282      -15.668 -29.445  53.538  1.00 74.94           D000 N
ATOM   8590  CA   ASP P 282      -15.590 -29.289  52.056  1.00 74.61           D000 C
ATOM   8591  C    ASP P 282      -16.879 -28.715  51.457  1.00 69.05           D000 C
ATOM   8592  O    ASP P 282      -17.285 -27.589  51.750  1.00 68.03           D000 O
ATOM   8593  CB   ASP P 282      -15.291 -30.618  51.327  1.00 75.72           D000 C
ATOM   8594  CG   ASP P 282      -13.973 -31.262  51.748  1.00 83.45           D000 C
ATOM   8595  OD2  ASP P 282      -14.017 -32.153  52.639  1.00 81.11           D000 O
ATOM   8596  OD1  ASP P 282      -12.912 -30.910  51.169  1.00 76.47           D000 O1-
TER
HETATM 8597  CA        CA  E   1  -46.046 -40.018  63.255  1.00 40.77               Ca
HETATM 8598  CA        CA  E   2  -15.968 -22.911  63.354  1.00 43.19               Ca
TER
```

**TABLE 15: Antibody Constant Regions**

| Designation | SEQ ID NO: | CL Domain Amino Acid Sequence |
|---|---|---|
| CL-1 | 32681 | GQPKANPTVTLFPPSSEELQANKATLVCLISDFYPGAVTVAWKADGSP VKAGVETTKPSKQSNNKYAASSYLSLTPEQWKSHRSYSCQVTHEGSTV EKTVAPTECS |
| CL-2 | 32682 | GQPKAAPSVTLFPPSSEELQANKATLVCLISDFYPGAVTVAWKADSSPV KAGVETTTPSKQSNNKYAASSYLSLTPEQWKSHRSYSCQVTHEGSTVEK TVAPTECS |
| CL-2.1 | 32683 | QPKAAPSVTLFPPSSEELQANKATLVCLISDFYPGAVTVAWKADSSPVK AGVETTTPSKQSNNKYAASSYLSLTPEQWKSHRSYSCQVTHEGSTVEKT VAPTECS |
| CL-3 | 32684 | GQPKAAPSVTLFPPSSEELQANKATLVCLISDFYPGAVTVAWKADSSPV KAGVETTTPSKQSNNKYAASSYLSLTPEQWKSHKSYSCQVTHEGSTVEK TVAPTECS |
| CL-7 | 32685 | GQPKAAPSVTLFPPSSEELQANKATLVCLVSDFYPGAVTVAWKADGSP VKVGVETTKPSKQSNNKYAASSYLSLTPEQWKSHRSYSCRVTHEGSTVE KTVAPAECS |
| Human kappa v1 | 32686 | TVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVT KSFNRGEC |
| Human kappa v2 | 32687 | RTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSPV TKSFNRGEC |

**TABLE 15: Antibody Constant Regions**

| Ig isotype | SEQ ID NO: | Heavy Chain Constant Region Amino Acid Sequence |
|---|---|---|
| Human IgG1z | 32688 | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWL NGKEYKCKVSNKALPAPIEKTISKAKGQPREPQVYTLPPSREEMTKNQVS LTCLVKGFYPSDIAVEWESNGQPENNYKTTPPVLDSDGSFFLYSKLTVDK SRWQQGNVFSCSVMHEALHNHYTQKSLSLSPGK |
| Human IgG1za | 32689 | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWL NGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKS RWQQGNVFSCSVMHEALHNHYTQKSLSLSPGK |
| Human IgG1f | 32690 | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKRVEP KSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWL NGKEYKCKVSNKALPAPIEKTISKAKGQPREPQVYTLPPSREEMTKNQVS LTCLVKGFYPSDIAVEWESNGQPENNYKTTPPVLDSDGSFFLYSKLTVDK SRWQQGNVFSCSVMHEALHNHYTQKSLSLSPGK |
| Human IgG1fa | 32691 | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKRVEP KSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWL NGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKS RWQQGNVFSCSVMHEALHNHYTQKSLSLSPGK |
| Human IgG2 | 32692 | ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KCCVECPPCPAPPVAGPSVFLFPPKPKDTLMISRTPEVTCVVVDVSHEDP EVQFNWYVDGVEVHNAKTKPREEQFNSTFRVVSVLTVVHQDWLNGKE YKCKVSNKGLPAPIEKTISKTKGQPREPQVYTLPPSREEMTKNQVSLTCLV KGFYPSDIAVEWESNGQPENNYKTTPPMLDSDGSFFLYSKLTVDKSRW QQGNVFSCSVMHEALHNHYTQKSLSLSPGK |
| Human IgG4 | 32693 | ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPSCPAPEFLGGPSVFLFPPKPKDTLMISRTPEVTCVVVDVSQED PEVQFNWYVDGVEVHNAKTKPREEQFNSTYRVVSVLTVLHQDWLNGK EYKCKVSNKGLPSSIEKTISKAKGQPREPQVYTLPPSQEEMTKNQVSLTCL VKGFYPSDIAVEWESNGQPENNYKTTPPVLDSDGSFFLYSRLTVDKSRW QEGNVFSCSVMHEALHNHYTQKSLSLSLGK |

(continued)

| Ig isotype | SEQ ID NO: | Heavy Chain Constant Region Amino Acid Sequence |
|---|---|---|
| Human IgG1-SEFL2 | 32694 | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVS HEDPEVKFNWYVDGVEVHNAKTKPCEEQYGSTYRCVSVLTVLHQDWL NGKEYKCKVSNKALPAPIEKTISKAKGQPREPQVYTLPPSREEMTKNQVS LTCLVKGFYPSDIAVEWESNGQPENNYKTTPPVLDSDGSFFLYSKLTVDK SRWQQGNVFSCSVMHEALHNHYTQKSLSLSPGK |

Each reference cited herein is hereby incorporated by reference in its entirety for all that it teaches and for all purposes.

The present invention is not to be limited in scope by the specific embodiments described herein, which are intended as single illustrations of individual embodiments of the invention, and functionally equivalent methods and components are invention. Indeed, various modifications of the invention, in addition to those shown and described herein will become apparent to those skilled in the art from the foregoing description and accompanying drawings. Such modifications are intended to fall within the scope of the appended items.

In view of the above, it will be appreciated that the invention also encompasses the following items:

1. An isolated antigen binding protein that binds to human ASGR-1 comprising the amino acid sequence of SEQ ID NO:5, wherein the antigen binding protein inhibits ASGR-1 binding to ligand.

2. The isolated antigen binding protein of item 1, wherein the isolated antigen binding protein binds to a carbohydrate recognition domain of human ASGR-1.

3. The isolated antigen binding protein of item 1 or 2, wherein the isolated antigen binding protein inhibits the internalization of ASGR.

4. The isolated antigen binding protein of any one of items 1-3, wherein the isolated antigen binding protein further binds ASGR-2.

5. The isolated antigen binding protein of any one of items 1-4, wherein the isolated antigen binding protein is a monoclonal antibody.

6. An isolated antigen binding protein that binds to human ASGR-2, wherein the isolated antigen binding protein inhibits ASGR-2 binding to ligand.

7. The isolated antigen binding protein of item 6, wherein the isolated antigen binding protein inhibits the internalization of ASGR.

8. The isolated antigen binding protein of any one of items 6 or 7, wherein the isolated antigen binding protein is a monoclonal antibody.

9. An isolated antigen binding protein that binds to human ASGR and inhibits human ASGR binding to ligand.

10. The isolated antigen binding protein of item 9, wherein the isolated antigen binding protein inhibits internalization of ASGR.

11. The isolated antigen binding protein of any one of items 9 or 10, wherein the isolated antigen binding protein is a monoclonal antibody.

12. An isolated antigen binding protein that binds to human ASGR-1 and human ASGR-2, and inhibits human ASGR-1 and/or human ASGR-2 binding to ligand.

13. The isolated antigen binding protein of item 12, wherein the isolated antigen binding protein inhibits internalization of human ASGR-1 or human ASGR-2.

14. The isolated antigen binding protein of any one of items 13 or 12, wherein the isolated antigen binding protein is a monoclonal antibody.

15. An isolated monoclonal antibody, wherein the isolated monoclonal antibody specifically binds to human ASGR-1 and comprises a VH CDR1, VH CDR2 and VH CDR3 having an amino acid sequence identical to or comprising 1, 2, or 3 amino acid residue substitutions in each CDR relative to the antibody VH of any of the sequences set forth in Tables 3-7.

16. The isolated monoclonal antibody of item 15, wherein said isolated monoclonal antibody further comprises a VL CDR1, VL CDR2 and VL CDR3 having an amino acid sequence identical to or comprising 1, 2, or 3 amino acid residue substitutions in each CDR relative to the antibody VL of any of the sequences set forth in Tables 3-7.

17. The isolated monoclonal antibody of item 16, wherein said VH CDRs and said VL CDRs are the corresponding paired VH and VL CDRs as set forth in Table 2.

18. An isolated monoclonal antibody, wherein the isolated monoclonal antibody specifically binds human ASGR-1 and comprises a heavy chain variable domain having at least 90% identity to any of the VH domain amino acid sequences set forth in Table 3-7.

19. The isolated monoclonal antibody of item 18, further comprising a light chain variable domain having at least 90% identity to any of the VL domain amino acid sequences set forth in Table 3-7.

20. The isolated monoclonal antibody of item 19, wherein the light chain variable domain and the heavy chain variable domain are the corresponding paired VL and VH as set forth in Table 3-7.

21. An isolated monoclonal antibody that competes for binding with the isolated monoclonal antibody of any one of items 1-20.

22. An isolated neutralizing monoclonal antibody that binds to human ASGR-1 comprising the amino acid sequence of SEQ ID NO:5, wherein the isolated neutralizing monoclonal antibody binds to human ASGR-1 at an epitope comprising at least one of the following amino acid residues: D216, Q217, N218, G219, P220, W221, Y229, E230, K234, W236, E239, Q240, P241, D242, D243, W244, Y245, G246, L249, G250, G251, G252, D254, Q270, W195, N209, R237, P238, H257, T259, D260, D261, R263, N265, D267, R271, Y273, H161, E162, E196, Q198, K199, F200, Q202, H203, H204, G232, F233, N235, G262, W167, S171, G172, K173, A174, A176, D177, N180, Y181, R183, L184, E185, D186, P272, W275, R170, 1205, G206, P207, V208, R274, S194, T193, T231, G226, T227 or D228 (SEQ ID NO:5).

23. An isolated neutralizing monoclonal antibody that binds to human ASGR-1 comprising the amino acid sequence of SEQ ID NO:5, wherein the isolated neutralizing monoclonal antibody binds to human ASGR-1 at an epitope comprising at least one of the following amino acid residues: Q240, D242, W244, E253, N265, D266, D267, R237, N209, H257, T259 or Y273 (SEQ ID NO:5).

24. An isolated neutralizing monoclonal antibody that binds to human ASGR-1 comprising the amino acid sequence of SEQ ID NO:5 but does not bind to a variant human ASGR-1, wherein the variant human ASGR-1 comprises a single mutation of a residue selected the group consisting of: R170, S171, G172, R183, L184, W195, E196, K199, H203, H204, P207, V208, N209, H215, D216, P220, D225, D228, R237, P238, E239, P241, D242, D243, Y245, G246, H247, G248, L249, G251, E253, T259, D260, R263, N265, Q270, R271, P272, R274 and E280 as shown in SEQ ID NO:5.

25. The isolated neutralizing monoclonal antibody of item 24, wherein the single mutation is selected from the group consisting of W195, E196, K199, H203, H204, P207, P220, G251, and R263 as shown in SEQ ID NO:5.

26. The isolated antigen binding protein or antibody of any one of items 1-25, wherein the antigen binding protein or antibody is a chimeric antibody, a humanized antibody, or a human antibody.

27. A pharmaceutical composition comprising the isolated antigen binding protein or antibody of any one of items 1-26, and a pharmaceutically acceptable excipient.

28. An isolated nucleic acid encoding the isolated antigen binding protein or antibody of any one of items 1-26.

29. A vector comprising the nucleic acid of item 28.

30. A host cell comprising the vector of item 29 or nucleic acid of item 28.

31. A method for producing an antigen binding protein or an antibody comprising culturing the host cell of item 30 under suitable conditions and recovering said antigen binding protein or antibody.

32. A method of treating or preventing a cardiovascular disease comprising administering to a patient in need thereof a therapeutically effective dose of an inhibitor of ASGR, ASGR-1, and/or ASGR-2.

33. The method of item 32, wherein said cardiovascular disease is coronary artery disease or myocardial infarction.

34. A method of reducing LDL cholesterol levels in a patient comprising administering to a patient in need thereof a therapeutically effective dose of an inhibitor of ASGR, ASGR-1, and/or ASGR-2.

35. A method of reducing non-HDL cholesterol levels in a patient comprising administering to a patient in need thereof a therapeutically effective dose of an inhibitor of ASGR, ASGR-1, and/or ASGR-2.

36. A method of increasing ALP levels in a patient comprising administering to a patient in need thereof a therapeutically effective dose of an inhibitor of ASGR, ASGR-1, and/or ASGR-2.

37. A method of antagonizing ASGR-1 in a patient comprising administering to a patient in need thereof a therapeutically effective dose of an inhibitor of ASGR, ASGR-1, and/or ASGR-2.

38. A method of antagonizing ASGR-2 in a patient comprising administering to a patient in need thereof a therapeutically effective dose of an inhibitor of ASGR, ASGR-1, and/or ASGR-2.

39. The methods of any one of items 32-38, wherein the inhibitor is an interfering RNA (e.g., siRNA or shRNA) that reduces expression of ASGR, ASGR-1 and/or ASGR-2.

40. The methods of any one of items 32-38, wherein the inhibitor is an isolated neutralizing antigen binding protein that binds to human ASGR, ASGR-1 and/or ASGR-2.

41. The methods of item 40, wherein the inhibitor is administered simultaneously or sequentially with at least one agent that lowers cholesterol.

42. The methods of item 41, wherein the at least one agent is a statin, an anti-PCSK9 inhibitor or a combination thereof.

43. The methods of item 42, wherein the at least one agent is selected from the group consisting of evolocumab, alirocumab, bococizumab, ALN-PCS, atorvastatin, cerivastatin, fluvastatin, lovastatin, mevastatin, pitavastatin, pravastatin, rosuvastatin, simvastatin, and some combination thereof.

**Claims**

1. An inhibitor of ASGR, ASGR-1, and/or ASGR-2 that is an isolated neutralizing antigen binding protein that binds to human ASGR, ASGR-1 and/or ASGR-2 for use for:

   (a) treating or preventing a cardiovascular disease in a patient in need thereof, optionally wherein said cardiovascular disease is coronary artery disease or myocardial infarction,
   (b) reducing LDL (low density lipoprotein) cholesterol levels in a patient in need thereof,
   (c) reducing non-HDL (non-high density lipoprotein) cholesterol levels in a patient in need thereof, or
   (d) increasing ALP (alkaline phosphatase) levels in a patient in need thereof.

2. An isolated antigen binding protein that binds to human ASGR-1 comprising the amino acid sequence of SEQ ID NO:5, wherein the antigen binding protein inhibits ASGR-1 binding to ligand, wherein the isolated antigen binding protein is a monoclonal antibody.

3. The isolated antigen binding protein of claim 2, wherein

(a) the isolated antigen binding protein binds to a carbohydrate recognition domain of human ASGR-1;
(b) the isolated antigen binding protein inhibits the internalization of ASGR,
(c) the isolated antigen binding protein further binds ASGR-2, and/or
(d) the isolated antigen binding protein is a chimeric antibody, a humanized antibody, or a human antibody.

4. The isolated antigen binding protein of claim 2 or 3, that is an isolated monoclonal antibody, wherein

(a) the isolated monoclonal antibody specifically binds to human ASGR-1 and comprises:

a VH CDR1, VH CDR2 and VH CDR3 having an amino acid sequence identical to or comprising 1, 2, or 3 amino acid residue substitutions in each CDR relative to the antibody VH of any of the sequences set forth in Tables 3-7; and
a VL CDR1, VL CDR2 and VL CDR3 having an amino acid sequence identical to or comprising 1, 2, or 3 amino acid residue substitutions in each CDR relative to the antibody VL of any of the sequences set forth in Tables 3-7, and/or

(b) the isolated monoclonal antibody specifically binds human ASGR-1 and comprises:

a heavy chain variable domain having at least 90% identity to any of the VH domain amino acid sequences set forth in Table 3-7; and
a light chain variable domain having at least 90% identity to any of the VL domain amino acid sequences set forth in Table 3-7.

5. The isolated antigen binding protein of claim 4, wherein

(a) said VH CDRs and said VL CDRs are the corresponding paired VH and VL CDRs as set forth in Table 2, or
(b) the light chain variable domain and the heavy chain variable domain are the corresponding paired VL and VH as set forth in Table 3-7.

6. The isolated antibody of any one of claims 2-5, that is a neutralizing monoclonal antibody that

(a) binds to human ASGR-1 comprising the amino acid sequence of SEQ ID NO:5, wherein the isolated neutralizing monoclonal antibody binds to human ASGR-1 at an epitope comprising at least one of the following amino acid residues: D216, Q217, N218, G219, P220, W221, Y229, E230, K234, W236, E239, Q240, P241, D242, D243, W244, Y245, G246, L249, G250, G251, G252, D254, Q270, W195, N209, R237, P238, H257, T259, D260, D261, R263, N265, D267, R271, Y273, H161, E162, E196, Q198, K199, F200, Q202, H203, H204, G232, F233, N235, G262, W167, S171, G172, K173, A174, A176, D177, N180, Y181, R183, L184, E185, D186, P272, W275, R170, I205, G206, P207, V208, R274, S194, T193, T231, G226, T227 or D228 (SEQ ID NO:5),
(b) binds to human ASGR-1 comprising the amino acid sequence of SEQ ID NO:5, wherein the isolated neutralizing monoclonal antibody binds to human ASGR-1 at an epitope comprising at least one of the following amino acid residues: Q240, D242, W244, E253, N265, D266, D267, R237, N209, H257, T259 or Y273 (SEQ ID NO:5), or
(c) binds to human ASGR-1 comprising the amino acid sequence of SEQ ID NO:5 but does not bind to a variant human ASGR-1, wherein the variant human ASGR-1 comprises a single mutation of a residue selected the group consisting of: R170, S171, G172, R183, L184, W195, E196, K199, H203, H204, P207, V208, N209, H215, D216, P220, D225, D228, R237, P238, E239, P241, D242, D243, Y245, G246, H247, G248, L249, G251, E253, T259, D260, R263, N265, Q270, R271, P272, R274 and E280 as shown in SEQ ID NO:5, optionally wherein the single mutation is selected from the group consisting of: W195, E196, K199, H203, H204, P207, P220, G251, and R263 as shown in SEQ ID NO:5.

7. A pharmaceutical composition comprising the isolated antibody of any one of claims 2-6, and a pharmaceutically acceptable excipient.

8. An isolated nucleic acid encoding the isolated antibody of any one of claims 2-6.

9. A vector comprising the nucleic acid of claim 8.

10. A host cell comprising the vector of claim 9 or nucleic acid of claim 8.

11. A method for producing an antibody comprising culturing the host cell of claim 10 under suitable conditions and recovering said antibody.

12. The inhibitor of ASGR, ASGR-1, and/or ASGR-2 for the use of claim 1, wherein the inhibitor comprises the isolated antibody of any one of claims 2-6.

13. The inhibitor of ASGR, ASGR-1, and/or ASGR-2 for the use of claim 1 or 12, wherein the inhibitor is for administering simultaneously or sequentially with at least one agent that lowers cholesterol.

14. The inhibitor of ASGR, ASGR-1, and/or ASGR-2 for the use of claim 13, wherein the at least one agent is a statin, an anti-PCSK9 inhibitor or a combination thereof, optionally wherein the at least one agent is selected from the group consisting of evolocumab, alirocumab, bococizumab, ALN-PCS, atorvastatin, cerivastatin, fluvastatin, lovastatin, mevastatin, pitavastatin, pravastatin, rosuvastatin, simvastatin, and some combination thereof.

**ASGR1 Full Seq Multiple Sequence Alignment**   Figure 1A

Figure 1B

# Human ASGR1 Sequence Alignment

# ASGR2 Full Seq Multiple Sequence Alignment

**Figure 2**

Cytoplasmic (56 AA)    Transmembrane (23 AA)

Stalk ~90 AA

Carbohydrate Binding Domain (CBD)

# Human ASGR1 vs Human ASGR2v2 Alignment        Figure 3

**Figure 4**

**Figure 5**

A.

| Study | NA/NC | Frq (%) | | OR (95% CI) | P value |
|---|---|---|---|---|---|
| Iceland | 33090/236254 | 0.41 | | 0.64 (0.51–0.80) | 5.8×10⁻⁵ |
| Denmark | 2258/7522 | 0.27 | | 0.81 (0.41–1.61) | 0.55 |
| Emory | 1804/1823 | 0.74 | | 0.67 (0.37–1.22) | 0.19 |
| Duke | 1268/712 | 0.35 | | 0.34 (0.08–1.38) | 0.13 |
| UPenn | 728/488 | 0.31 | | 0.67 (0.14–3.26) | 0.62 |
| UK–I | 653/664 | 0.83 | | 0.55 (0.21–1.46) | 0.23 |
| UK–II | 2175/1494 | 0.77 | | 0.78 (0.44–1.37) | 0.38 |
| New Zealand | 548/457 | 0.44 | | 0.42 (0.08–2.18) | 0.30 |
| All | | | | 0.66 (0.55–0.79) | 4.0×10⁻⁸ |

Odds Ratio

B.

**Figure 6**

**Figure 7**

A.

Male Mice

B.

Female Mice

**Figure 8**

A

Flag

hASGR1

β-actin

B

C

**Figure 9**

**Figure 10**

A.

B.

**Figure 11**

**Figure 12**

A.

B.

Mouse ASGR1 Expression

C.

Mouse ASGR2 Expression

**Figure 13**

A.

B.

**Figure 14**

**Figure 15**

**Figure 16**

**Figure 17**

**Figure 18**

A

B

C

**Figure 19**

**Figure 20**

A

**Figure 21**

**Figure 22**

A

B

**Figure 23**

A

B

22G5 Light Chain

ASGR1 CBD

22G5 Heavy Chain

**Figure 24**

**Figure 25**

Figure 26

'Carbohydrate
binding loop'

Helix α-1

ASGR1-CBD without 4A2 Ab
ASGR1-CBD with 4A2 Ab

5Å distance

**Figure 27**

Figure 28

**Figure 29**

**Figure 30**

5Å distance

**Figure 31**

Figure 32

A

ASGR1 CBD

Helix α-2

CDR H1

CDR H3

CDR L2

CDR H2

CDR L1

72G9 Heavy
Chain

72G9 Light
Chain

CDR L3

B

ASGR1 CBD

Helix α-1

Helix α-2

Ca2+

GalNAc

Ca2+

72G9
Heavy
Chain

72G9
Light
Chain

**Figure 33**

Figure34

**Figure 35**

**Figure 36**

Figure 37

A

B

**Figure 38**

A

B

218G4 Light
Chain

218G4 Heavy
Chain

GalNAc

ASGR1 CBD with 218G4
ASGR1 CBD without 218G4

W244

Helix α-2

**Figure 39**

**Figure 40**

**Figure 41**

Figure 42

Figure 43

**Binding of antibody 7F4 to parental and ASGR-expressing CHO-S cells**

A

B

C

**Figure 44**

A

B

Serum LDL Cholesterol

Serum Alkaline Phosphatase

**Data expressed as % change from baseline**

Figure 45

A

## Serum LDL Cholesterol

B

## Serum Alkaline Phosphatase

Data expressed as % change from baseline

**Figure 46**

**Figure 47**

**Figure 58**

Human vs Cyno - Somalogic Proteins

**Figure 59**

FIGURE 48

Table 1

**Immunogens**

| Name | Sequence Range | Tags | Amino Acid Sequence | Vector | SEQ ID NO: |
|---|---|---|---|---|---|
| muASGR1 | 1-284 | TCE | MTKDYQDFQHLDNDNDHHQLRRGPPPTPRLLQRLCSGSRLLLLSSSLSILLLVVVCVITSQNSQLREDLLALRQNF SNLTVSTEDQVKALSTQGSSVGRKMKLVESKLEKQQKDLTEDHSSLLLHVKQLVSDVRSLSCQMAAFRGNGSER TCCPINWVEYEGSCYWFSSSVRPWTEADKYCQLENAHLVVVTSRDEQNFLQRHMGPLNTWIGLTDQNGPWKW VDGTDYETGFQNWRPEQPDNWYGHGLGGGEDCAHFTTDGRWNDDVCRRPYRWVCETKLDKAN | pTT5 | 1 |
| muAsgr2 | 1-301 | TCE | VEKDCQDIQQLDSEENDHQLSGDDEHGSHVQDPRIENPHWKGQPLSRPFPQRLCSTFRLSLLALAFNILLLVVIC VVSSQSIQLQEEFRTLKETFSNFSSSTLMEFGALDTLGGSTNAILTSWLAQLEEKQQQLKADHSTLLFHLKHFPMD LRTLTCQLAYFQSNGTECCPVNWVEFGGSCYWFSRDGLTWAEADQYCQLENAHLLVINSREEQDFVVKHRSQF HIWIGLTDRDGSWKWVDGTDYRSNYRNWAFTQPDNWQGHEQGGGEDCAEILSDGHWNDNFCQQVNRWVCE KRRNITH | pTT5 | 2 |
| muASGR1(ECD) | 63-284 | TCE | SQLREDLLALRQNFSNLTVSTEDQVKALSTQGSSVGRKMKLVESKLEKQQKDLTEDHSSLLLHVKQLVSDVRSLS CQMAAFRGNGSERTCCPINWVEYEGSCYWFSSSVRPWTEADKYCQLENAHLVVVTSRDEQNFLQRHMGPLNT WIGLTDQNGPWKWVDGTDYETGFQNWRPEQPDNWYGHGLGGGEDCAHFTTDGRWNDDVCRRPYRWVCET KLDKAN | pSLX235a | 3 |
| muASGR1(CBD) | 153-284 | TCE | CPINWVEYEGSCYWFSSSVRPWTEADKYCQLENAHLVVVTSRDEQNFLQRHMGPLNTWIGLTDQNGPWKWVD GTDYETGFQNWRPEQPDNWYGHGLGGGEDCAHFTTDGRWNDDVCRRPYRWVCETKLDKAN | pSLX235a | 4 |
| huASGR1 | 1-291 | TCE | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLL | pTT5 | 5 |
| huASGR2 | 1-287 | TCE | MAKDFQDIQQLSSEENDHPFHQGPPPAQPLAQRLCSMVCFSLLALSFNILLLVVICVTGSQSAQLQAELRSLKEAF SNFSSSTLTEVQAISTHGGSVGDKITSLGAKLEKQQQDLKADHDALLFHLKHFPVDLRFVACQMELLHSNGSQRT CCPVNWVEHQGSCYWFSHSGKAWAEAEKYCQLENAHLVVINSWEEQKFIVQHTNPFNTWIGLTDSDGSWKWV DGTDYRHNYKNWAVTQPDNWHGHELGGSEDCVEVQPDGRWNDDFCLQVYRWVCEKRRNATGEVA | pTT5 | 6 |
| huASGR1(ECD) | 64-291 | TCE | SQLQEELRGLRETFSNFTASTEAQVKGLSTQGGNVGRKMKSLESQLEKQQKDLSEDHSSLLLHVKQFVSDLRSL SCQMAALQGNGSERTCCPVNWVEHERSCYWFSRSGKAWADADNYCRLEDAHLVVVTSWEEQKFVQHHIGPV NTWMGLHDQNGPWKWVDGTDYETGFKNWRPEQPDDWYGHGLGGGEDCAHFTDDGRWNDDVCQRPYRWV CETELDKASQEPPLL | pSLX235a | 7 |
| huASGR1(CBD) | 154-291 | TCE | CPVNWVEHERSCYWFSRSGKAWADADNYCRLEDAHLVVVTSWEEQKFVQHHIGPVNTWMGLHDQNGPWKW VDGTDYETGFKNWRPEQPDDWYGHGLGGGEDCAHFTDDGRWNDDVCQRPYRWVCETELDKASQEPPLL | pSLX235a | 8 |

FIGURE 48

| huASGR2(ECD) | 61-287 | TCE | QSAQLQAELRSLKEAFSNFSSSTLTEVQAISTHGGSVGDKITSLGAKLEKQQQDLKADHDALLFHLKHFPVDLRFV ACQMELLHSNGSQRTCCPVNWVEHQGSCYWFSHSGKAWAEAEKYCQLENAHLVVINSWEEQKFIVQHTNPFN TWIGLTDSDGSWKWVDGTDYRHNYKNWAVTQPDNWHGHELGGSEDCVEVQPDGRWNDDFCLQVYRWVCEK RRNATGEVA | pSLX235a | 9 |
|---|---|---|---|---|---|
| huASGR2(CBD) | 153-287 | TCE | CPVNWVEHQGSCYWFSHSGKAWAEAEKYCQLENAHLVVINSWEEQKFIVQHTNPFNTWIGLTDSDGSWKWVD GTDYRHNYKNWAVTQPDNWHGHELGGSEDCVEVQPDGRWNDDFCLQVYRWVCEKRRNATGEVA | pSLX235a | 10 |
| **Structural Work** | | | | | |
| **Name** | **Sequence Range** | **Tags** | **Amino Acid Sequence** | **Vector** | |
| M::huASGR1(154-281) | 154-281 | None | MCPVNWVEHERSCYWFSRSGKAWADADNYCRLEDAHLVVVTSWEEQKFVQHHIGPVNTWMGLHDQNGPWK WVDGTDYETGFKNWRPEQPDDWYGHGLGGGEDCAHFTDDGRWNDDVCQRPYRWVCETEL | pET21a | 11 |
| M::huASGR1(148-291) | 148-291 | None | MGSERTCCPVNWVEHERSCYWFSRSCKAWADADNYCRLEDAHLVVVTSWEEQKFVQHHIGPVNTWMGLHDQ NGPWKWVDGTDYETGFKNWRPEQPDDWYGHGLGGGEDCAHFTDDGRWNDDVCQRPYRWVCETELDKASQ EPPLL | pET21a | 12 |
| MA::huASGR1(148-291) | 148-291 | None | MAGSERTCCPVNWVEHERSCYWFSRSGKAWADADNYCRLEDAHLVVVTSWEEQKFVQHHIGPVNTWMGLHD QNGPWKWVDGTDYETGFKNWRPEQPDDWYGHGLGGGEDCAHFTDDGRWNDDVCQRPYRWVCETELDKAS QEPPLL | pET21a | 13 |
| MA::huASGR1(154-281) | 154-281 | None | MACPVNWVEHERSCYWFSRSGKAWADADNYCRLEDAHLVVVTSWEEQKFVQHHIGPVNTWMGLHDQNGPW KWVDGTDYETGFKNWRPEQPDDWYGHGLGGGEDCAHFTDDGRWNDDVCQRPYRWVCETEL | pET21a | 14 |
| MA::huASGR1(60-153) | 60-153 | None | MAGSQNSQLQEELRGLRETFSNFTASTEAQVKGLSTQGGNVGRKMKSLESQLEKQQKDLSEDHSSLLLHVKQF VSDLRSLSCQMAALQGNGSERTC | pET21a | 15 |
| MA::huASGR1(60-291) | 60-291 | None | MAGSQNSQLQEELRGLRETFSNFTASTEAQVKGLSTQGGNVGRKMKSLESQLEKQQKDLSEDHSSLLLHVKQF VSDLRSLSCQMAALQGNGSERTCCPVNWVEHERSCYWFSRSGKAWADADNYCRLEDAHLVVVTSWEEQKFV QHHIGPVNTWMGLHDQNGPWKWVDGTDYETGFKNWRPEQPDDWYGHGLGGGEDCAHFTDDGRWNDDVCQ RPYRWVCETELDKASQEPPLL | pET21a | 16 |
| MA::huASGR1(62-153) | 62-153 | None | MAQNSQLQEELRGLRETFSNFTASTEAQVKGLSTQGGNVGRKMKSLESQLEKQQKDLSEDHSSLLLHVKQFVS DLRSLSCQMAALQGNGSERTC | pET21a | 17 |
| MA::huASGR1(58-153) | 58-153 | None | MAVIGSQNSQLQEELRGLRETFSNFTASTEAQVKGLSTQGGNVGRKMKSLESQLEKQQKDLSEDHSSLLLHVKQ FVSDLRSLSCQMAALQGNGSERTC | pET21a | 18 |
| MA::huASGR1(58-143) | 58-143 | None | MAVIGSQNSQLQEELRGLRETFSNFTASTEAQVKGLSTQGGNVGRKMKSLESQLEKQQKDLSEDHSSLLLHVKQ FVSDLRSLSCQMAA | pET21a | 19 |
| MA::huASGR1(62-143) | 62-143 | None | MAQNSQLQEELRGLRETFSNFTASTEAQVKGLSTQGGNVGRKMKSLESQLEKQQKDLSEDHSSLLLHVKQFVS DLRSLSCQMAA | pET21a | 20 |
| MA::huASGR1(60-143) | 60-143 | None | MAGSQNSQLQEELRGLRETFSNFTASTEAQVKGLSTQGGNVGRKMKSLESQLEKQQKDLSEDHSSLLLHVKQF VSDLRSLSCQMAA | pET21a | 21 |

FIGURE 48

| MA::huASGR1(60-282) | 60-282 | None | MAGSQNSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELD | pET21a | 22 |
|---|---|---|---|---|---|
| MA::huASGR1(62-291) | 62-291 | None | MAQNSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 23 |
| MA::huASGR1(61-291) | 62-291 | None | MASQNSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 24 |
| MA::huASGR1(58-291) | 58-291 | None | MAVIGSQNSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 25 |
| MA::huASGR1(63-291) | 63-291 | None | MANSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 26 |
| MA::huASGR2(59-287) | 59-287 | None | MAGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 27 |
| MA::huASGR2(57-287) | 57-287 | None | MAVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 28 |
| MA::huASGR2(60-287) | 60-287 | None | MASQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 29 |
| MA::huASGR2(61-287) | 61-287 | None | MAQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 30 |
| MA::huASGR2(62-287) | 62-287 | None | MASAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 31 |

FIGURE 48

| Complex Formation Assays | | | | | |
|---|---|---|---|---|---|
| Name | Sequence Range | Tags | Amino Acid Sequence | Vector | |
| huASGR1::GS::SNAP26f | 1-291 | SNAP26f | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLLGSMDKDCEMKRTTLDSPLGKLELSGCEQGLHRIIFLGKGTSAADAVEVPAPAAVLGGPEPLMQATAWLNAYF HQPEAIEEFPVPALHHPVFQQESFTRQVLWKLLKVVKFGEVISYSHLAALAGNPAATAAVKTALSGNPVPILIPCHR VVQGDLDVGGYEGGLAVKEWLLAHEGHRLGKPGLG | pIRES_puro3.1 | 32 |
| huASGR1::GS::(G4S)3::SNAP26f | 1-291 | SNAP26f | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLLGSGGGGSGGGGSGGGGSMDKDCEMKRTTLDSPLGKLELSGCEQGLHRIIFLGKGTSAADAVEVPAPAAVL GGPEPLMQATAWLNAYFHQPEAIEEFPVPALHHPVFQQESFTRQVLWKLLKVVKFGEVISYSHLAALAGNPAATA AVKTALSGNPVPILIPCHRVVQGDLDVGGYEGGLAVKEWLLAHEGHRLGKPGLG | pIRES_puro3.1 , pJif1 | 33 |
| huASGR1::GS::CLIP | 1-291 | CLIP | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLLGSMDKDCEMKRTTLDSPLGKLELSGCEQGLHRIIFLGKGTSAADAVEVPAPAAVLGGPEPLIQATAWLNAYF HQPEAIEEFPVPALHHPVFQQESFTRQVLWKLLKVVKFGEVISESHLAALVGNPAATAAVNTALDGNPVPILIPCH RVVQGDSDVGPYLGGLAVKEWLLAHEGHRLGKPGLG | pIRES_puro3.1 , pJif1 | 34 |
| huASGR1::GS::(G4S)3::CLIP | 1-291 | CLIP | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLLGSGGGGSGGGGSGGGGSMDKDCEMKRTTLDSPLGKLELSGCEQGLHRIIFLGKGTSAADAVEVPAPAAVL GGPEPLIQATAWLNAYFHQPEAIEEFPVPALHHPVFQQESFTRQVLWKLLKVVKFGEVISESHLAALVGNPAATAA VNTALDGNPVPILIPCHRVVQGDSDVGPYLGGLAVKEWLLAHEGHRLGKPGLG | pIRES_puro3.1 | 35 |
| huASGR2::GS::SNAP26f | 1-287 | SNAP26f | MAKDFQDIQQLSSEENDHPFHQGPPPAQPLAQRLCSMVCFSLLALSFNILLLVVICVTGSQSAQLQAELRSLKEAF SNFSSSTLTEVQAISTHGGSVGDKITSLGAKLEKQQQDLKADHDALLFHLKHFPVDLRFVACQMELLHSNGSQRT CCPVNWVEHQGSCYWFSHSGKAWAEAEKYCQLENAHLVVINSWEEQKFIVQHTNPFNTWIGLTDSDGSWKWV DGTDYRHNYKNWAVTQPDNWHGHELGGSEDCVEVQPDGRWNDDFCLQVYRWVCEKRRNATGEVAGSMDKD CEMKRTTLDSPLGKLELSGCEQGLHRIIFLGKGTSAADAVEVPAPAAVLGGPEPLMQATAWLNAYFHQPEAIEEF PVPALHHPVFQQESFTRQVLWKLLKVVKFGEVISYSHLAALAGNPAATAAVKTALSGNPVPILIPCHRVVQGDLDV GGYEGGLAVKEWLLAHEGHRLGKPGLG | pIRES_puro3.1 , pJif1 | 36 |

FIGURE 48

| | | | | | |
|---|---|---|---|---|---|
| huASGR2::GS::(G4S)3::SNAP26f | 1-287 | SNAP26f | MAKDFQDIQQLSSEENDHPFHQGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSGGGGSGGGGSGGGGSMDKDCEMKRTTLDSPLGKLELSGCEQGLHRIIFLGKGTSAADAVEVPAPAAVLGGPEPLMQATAWLNAYFHQPEAIEEFPVPALHHPVFQQESFTRQVLWKLLKVVKFGEVISYSHLAALAGNPAATAAVKTALSGNPVPILIPCHRVVQGDLDVGGYEGGLAVKEWLLAHEGHRLGKPGLG | pIRES_puro3.1 | 37 |
| huASGR2::GS::CLIP | 1-287 | CLIP | MAKDFQDIQQLSSEENDHPFHQGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSMDKDCEMKRTTLDSPLGKLELSGCEQGLHRIIFLGKGTSAADAVEVPAPAAVLGGPEPLIQATAWLNAYFHQPEAIEEFPVPALHHPVFQQESFTRQVLWKLLKVVKFGEVISESHLAALVGNPAATAAVNTALDGNPVPILIPCHRVVQGDSDVGPYLGGLAVKEWLLAHEGHRLGKPGLG | pIRES_puro3.1, pJlf1 | 38 |
| huASGR2::GS::(G4S)3::CLIP | 1-287 | CLIP | MAKDFQDIQQLSSEENDHPFHQGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSGGGGSGGGGSGGGGSMDKDCEMKRTTLDSPLGKLELSGCEQGLHRIIFLGKGTSAADAVEVPAPAAVLGGPEPLIQATAWLNAYFHQPEAIEEFPVPALHHPVFQQESFTRQVLWKLLKVVKFGEVISESHLAALVGNPAATAAVNTALDGNPVPILIPCHRVVQGDSDVGPYLGGLAVKEWLLAHEGHRLGKPGLG | pIRES_puro3.1 | 39 |
| huASGR2(v4)::GS::SNAP26f | 1-306 | SNAP26f | MAKDFQDIQQLSSEENDHPFHQGEGPGTRRLNPRRGNPFLKGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSMDKDCEMKRTTLDSPLGKLELSGCEQGLHRIIFLGKGTSAADAVEVPAPAAVLGGPEPLMQATAWLNAYFHQPEAIEEFPVPALHHPVFQQESFTRQVLWKLLKVVKFGEVISYSHLAALAGNPAATAAVKTALSGNPVPILIPCHRVVQGDLDVGGYEGGLAVKEWLLAHEGHRLGKPGLG | pIRES_puro3.1, pJlf1 | 40 |
| huASGR2(v4)::GS::(G4S)3::SNAP26f | 1-306 | SNAP26f | MAKDFQDIQQLSSEENDHPFHQGEGPGTRRLNPRRGNPFLKGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSGGGGSGGGGSGGGGSMDKDCEMKRTTLDSPLGKLELSGCEQGLHRIIFLGKGTSAADAVEVPAPAAVLGGPEPLMQATAWLNAYFHQPEAIEEFPVPALHHPVFQQESFTRQVLWKLLKVVKFGEVISYSHLAALAGNPAATAAVKTALSGNPVPILIPCHRVVQGDLDVGGYEGGLAVKEWLLAHEGHRLGKPGLG | pIRES_puro3.1 | 41 |
| huASGR2(v4)::GS::CLIP | 1-306 | CLIP | MAKDFQDIQQLSSEENDHPFHQGEGPGTRRLNPRRGNPFLKGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSMDKDCEMKRTTLDSPLGKLELSGCEQGLHRIIFLGKGTSAADAVEVPAPAAVLGGPEPLIQATAWLNAYFHQPEAIEEFPVPALHHPVFQQESFTRQVLWKLLKVVKFGEVISESHLAALVGNPAATAAVNTALDGNPVPILIPCHRVVQGDSDVGPYLGGLAVKEWLLAHEGHRLGKPGLG | pIRES_puro3.1 | 42 |

FIGURE 48

| | | | | | |
|---|---|---|---|---|---|
| huASGR2(v4)::GS::(G4S)3::CLIP | 1-306 | CLIP | MAKDFQDIQQLSSEENDHPFHQGEGPGTRRLNPRRGNPFLKGPPPAQPLAQRLCSMVCFSLLALSFNILLLVVIC VTGSQSAQLQAELRSLKEAFSNFSSSTLTEVQAISTHGGSVGDKITSLGAKLEKQQQDLKADHDALLFHLKHFPVD LRFVACQMELLHSNGSQRTCCPVNWVEHQGSCYWFSHSGKAWAEAEKYCQLENAHLVVINSWEEQKFIVQHT NPFNTWIGLTDSDGSWKWVDGTDYRHNYKNWAVTQPDNWHGHELGGSEDCVEVQPDGRWNDDFCLQVYRW VCEKRRNATGEVAGSGGGGSGGGGSGGGGSMDKDCEMKRTTLDSPLGKLELSGCEQGLHRIIFLGKGTSAADA VEVPAPAAVLGGPEPLIQATAWLNAYFHQPEAIEEFPVPALHHPVFQQESFTRQVLWKLLKVVKFGEVISESHLAA LVGNPAATAAVNTALDGNPVPILIPCHRVVQGDSDVGPYLGGLAVKEWLLAHEGHRLGKPGLG | pIRES_puro3.1 | 43 |
| **Mammalian Expression** | | | | | |
| huASGR1 | 1-291 | None | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLL | pTT5, pSLX235a, pJIF1 | 44 |
| huASGR1(Y273C) | 1-291 | None | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPCRWVCETELDKASQEP PLL | pTT5, pSLX235a, pJIF1 | 45 |
| huASGR1::Flag | 1-291 | Flag | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLLDYKDDDDK | pTT5, pSLX235a | 46 |
| huASGR1(Y273C)::Flag | 1-291 | Flag | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPCRWVCETELDKASQEP PLLDYKDDDDK | pTT5, pSLX235a | 47 |
| cyASGR1 | 1-291 | None | MTKEYQDLQHLDNEESDHHQLGKGPPPPQSLLRRLCSGPRLLLLSLGLSLLLLVVVCVIGSQNAQLQRELRGLRE TLSNFTASTEAQVKGLSTQGGNVGRKMKSLESQLEKQQKDLSEDHSSLLLHVKQFVSDLRSLSCQMAALQGNG SERACCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLL | pTT5, pSLX235a | 48 |
| cyASGR1(Y273C) | 1-291 | None | MTKEYQDLQHLDNEESDHHQLGKGPPPPQSLLRRLCSGPRLLLLSLGLSLLLLVVVCVIGSQNAQLQRELRGLRE TLSNFTASTEAQVKGLSTQGGNVGRKMKSLESQLEKQQKDLSEDHSSLLLHVKQFVSDLRSLSCQMAALQGNG SERACCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPCRWVCETELDKASQEP PLL | pTT5, pSLX235a | 49 |
| cyASGR1::Flag | 1-291 | Flag | MTKEYQDLQHLDNEESDHHQLGKGPPPPQSLLRRLCSGPRLLLLSLGLSLLLLVVVCVIGSQNAQLQRELRGLRE TLSNFTASTEAQVKGLSTQGGNVGRKMKSLESQLEKQQKDLSEDHSSLLLHVKQFVSDLRSLSCQMAALQGNG SERACCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLLDYKDDDDK | pTT5, pSLX235a | 50 |

FIGURE 48

| cyASGR1(Y273C)::Flag | 1-291 | Flag | MTKEYQDLQHLDNEESDHHQLGKGPPPPQSLLRRLCSGPRLLLLSLGLSLLLLVVVCVIGSQNAQLQRELRGLRE TLSNFTASTEAQVKGLSTQGGNVGRKMKSLESQLEKQQKDLSEDHSSLLLHVKQFVSDLRSLSCQMAALQGNG SERACCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPCRWVCETELDKASQEP PLLDYKDDDDK | pTT5, pSLX235a | 51 |
|---|---|---|---|---|---|
| muASGR1 | 1-284 | None | MTKDYQDFQHLDNDNDHHQLRRGPPPTPRLLQRLCSGSRLLLLSSSLSILLLVVVCVITSQNSQLREDLLALRQNF SNLTVSTEDQVKALSTQGSSVGRKMKLVESKLEKQQKDLTEDHSSLLLHVKQLVSDVRSLSCQMAAFRGNGSER TCCPINWVEYEGSCYWFSSSVRPWTEADKYCQLENAHLVVVTSRDEQNFLQRHMGPLNTWIGLTDQNGPWKW VDGTDYETGFQNWRPEQPDNWYGHGLGGGEDCAHFTTDGRWNDDVCRRPYRWVCETKLDKAN | pTT5, pJIF1 | 52 |
| muASGR1(Y272C) | 1-284 | None | MTKDYQDFQHLDNDNDHHQLRRGPPPTPRLLQRLCSGSRLLLLSSSLSILLLVVVCVITSQNSQLREDLLALRQNF SNLTVSTEDQVKALSTQGSSVGRKMKLVESKLEKQQKDLTEDHSSLLLHVKQLVSDVRSLSCQMAAFRGNGSER TCCPINWVEYEGSCYWFSSSVRPWTEADKYCQLENAHLVVVTSRDEQNFLQRHMGPLNTWIGLTDQNGPWKW VDGTDYETGFQNWRPEQPDNWYGHGLGGGEDCAHFTTDGRWNDDVCRRPCRWVCETKLDKAN | pTT5, pSLX235a, pJIF1 | 53 |
| ratASGR1 | 1-284 | None | MTKDYQDFQHLDNENDHHQLQRGPPPAPRLLQRLCSGFRLFLLSLGLSILLLVVVCVITSQNSQLREDLRVLRQNF SNFTVSTEDQVKALTTQGERVGRKMKLVESQLEKHQEDLREDHSRLLLHVKQLVSDVRSLSCQMAALRGNGSE RICCPINWVEYEGSCYWFSSSVKPWTEADKYCQLENAHLVVVTSWEEQRFVQQHMGPLNTWIGLTDQNGPWK WVDGTDYETGFKNWRPGQPDDWYGHGLGGGEDCAHFTTDGHWNDDVCRRPYRWVCETELGKAN | pTT5, pSLX235a | 54 |
| ratASGR1(Y272C) | 1-284 | None | MTKDYQDFQHLDNENDHHQLQRGPPPAPRLLQRLCSGFRLFLLSLGLSILLLVVVCVITSQNSQLREDLRVLRQNF SNFTVSTEDQVKALTTQGERVGRKMKLVESQLEKHQEDLREDHSRLLLHVKQLVSDVRSLSCQMAALRGNGSE RICCPINWVEYEGSCYWFSSSVKPWTEADKYCQLENAHLVVVTSWEEQRFVQQHMGPLNTWIGLTDQNGPWK WVDGTDYETGFKNWRPGQPDDWYGHGLGGGEDCAHFTTDGHWNDDVCRRPCRWVCETELGKAN | pTT5, pSLX235a | 55 |
| muASGR1::Flag | 1-284 | Flag | MTKDYQDFQHLDNDNDHHQLRRGPPPTPRLLQRLCSGSRLLLLSSSLSILLLVVVCVITSQNSQLREDLLALRQNF SNLTVSTEDQVKALSTQGSSVGRKMKLVESKLEKQQKDLTEDHSSLLLHVKQLVSDVRSLSCQMAAFRGNGSER TCCPINWVEYEGSCYWFSSSVRPWTEADKYCQLENAHLVVVTSRDEQNFLQRHMGPLNTWIGLTDQNGPWKW VDGTDYETGFQNWRPEQPDNWYGHGLGGGEDCAHFTTDGRWNDDVCRRPYRWVCETKLDKANDYKDDDDK | pTT5, pSLX235a | 56 |
| muASGR1(Y272C)::Flag | 1-284 | Flag | MTKDYQDFQHLDNDNDHHQLRRGPPPTPRLLQRLCSGSRLLLLSSSLSILLLVVVCVITSQNSQLREDLLALRQNF SNLTVSTEDQVKALSTQGSSVGRKMKLVESKLEKQQKDLTEDHSSLLLHVKQLVSDVRSLSCQMAAFRGNGSER TCCPINWVEYEGSCYWFSSSVRPWTEADKYCQLENAHLVVVTSRDEQNFLQRHMGPLNTWIGLTDQNGPWKW VDGTDYETGFQNWRPEQPDNWYGHGLGGGEDCAHFTTDGRWNDDVCRRPCRWVCETKLDKANDYKDDDDK | pTT5, pSLX235a | 57 |
| ratASGR1::Flag | 1-284 | Flag | MTKDYQDFQHLDNENDHHQLQRGPPPAPRLLQRLCSGFRLFLLSLGLSILLLVVVCVITSQNSQLREDLRVLRQNF SNFTVSTEDQVKALTTQGERVGRKMKLVESQLEKHQEDLREDHSRLLLHVKQLVSDVRSLSCQMAALRGNGSE RICCPINWVEYEGSCYWFSSSVKPWTEADKYCQLENAHLVVVTSWEEQRFVQQHMGPLNTWIGLTDQNGPWK WVDGTDYETGFKNWRPGQPDDWYGHGLGGGEDCAHFTTDGHWNDDVCRRPYRWVCETELGKANDYKDDDD K | pTT5, pSLX235a | 58 |

FIGURE 48

| ratASGR1(Y272C)::Flag | 1-284 | Flag | MTKDYQDFQHLDNENDHHQLQRGPPPAPRLLQRLCSGFRLFLLSLGLSILLLVVVCVITSQNSQLREDLRVLRQNF SNFTVSTEDQVKALTTQGERVGRKMKLVESQLEKHQEDLREDHSRLLLHVKQLVSDVRSLSCQMAALRGNGSE RICCPINWVEYEGSCYWFSSSVKPWTEADKYCQLENAHLVVVTSWEEQRFVQQHMGPLNTWIGLTDQNGPWK WVDGTDYETGFKNWRPGQPDDWYGHGLGGGEDCAHFTTDGHWNDDVCRRPCRWVCETELGKANDYKDDDD K | pTT5, pSLX235a | 59 |
| huASGR2 | 1-306 | None | MAKDFQDIQQLSSEENDHPFHQGPPPAQPLAQRLCSMVCFSLLALSFNILLLVVICVTGSQSAQLQAELRSLKEAF SNFSSSTLTEVQAISTHGGSVGDKITSLGAKLEKQQQDLKADHDALLFHLKHFPVDLRFVACQMELLHSNGSQRT CCPVNWVEHQGSCYWFSHSGKAWAEAEKYCQLENAHLVVINSWEEQKFIVQHTNPFNTWIGLTDSDGSWKWV DGTDYRHNYKNWAVTQPDNWHGHELGGSEDCVEVQPDGRWNDDFCLQVYRWVCEKRRNATGEVA | pTT5, pSLX235a, pJIF1 | 60 |
| muASGR2 | 1-301 | None | MEKDCQDIQQLDSEENDHQLSGDDEHGSHVQDPRIENPHWKGQPLSRPFPQRLCSTFRLSLLALAFNILLLVVIC VVSSQSIQLQEEFRTLKETFSNFSSSTLMEFGALDTLGGSTNAILTSWLAQLEEKQQQLKADHSTLLFHLKHFPMD LRTLTCQLAYFQSNGTECCPVNWVEFGGSCYWFSRDGLTWAEADQYCQLENAHLLVINSREEQDFVVKHRSQF HIWIGLTDRDGSWKWVDGTDYRSNYRNWAFTQPDNWQGHEQGGGEDCAEILSDGHWNDNFCQQVNRWVCE KRRNITH | pTT5, pJIF1 | 61 |
| ratASGR2 | 1-301 | None | MEKDFQDIQQLDSEENDHQLIGDEEQGSHVQNLRTENPRWGGQPPSRPFPQRLCSKFRLSLLALAFNILLLVVIC VVSSQSMQLQKEFWTLKETLSNFSTTTLMEFKALDSHGGSRNDNLTSWETILEKKQKDIKADHSTLLFHLKHFPL DLRTLTCQLAFFLSNGTECCPVNWVEFGGSCYWFSRDGLTWAEADQYCQMENAHLLVINSREEQEFVVKHRGA FHIWIGLTDKDGSWKWVDGTEYRSNFKNWAFTQPDNWQGHEEGGSEDCAEILSDGLWNDNFCQQVNRWACE RKRDITY | pTT5, pSLX235a | 62 |
| huASGR2::6xHis | 1-306 | 6xHis | MAKDFQDIQQLSSEENDHPFHQGPPPAQPLAQRLCSMVCFSLLALSFNILLLVVICVTGSQSAQLQAELRSLKEAF SNFSSSTLTEVQAISTHGGSVGDKITSLGAKLEKQQQDLKADHDALLFHLKHFPVDLRFVACQMELLHSNGSQRT CCPVNWVEHQGSCYWFSHSGKAWAEAEKYCQLENAHLVVINSWEEQKFIVQHTNPFNTWIGLTDSDGSWKWV DGTDYRHNYKNWAVTQPDNWHGHELGGSEDCVEVQPDGRWNDDFCLQVYRWVCEKRRNATGEVAHHHHHH | pTT5, pSLX235a | 63 |
| muASGR2::6xHis | 1-301 | 6xHis | MEKDCQDIQQLDSEENDHQLSGDDEHGSHVQDPRIENPHWKGQPLSRPFPQRLCSTFRLSLLALAFNILLLVVIC VVSSQSIQLQEEFRTLKETFSNFSSSTLMEFGALDTLGGSTNAILTSWLAQLEEKQQQLKADHSTLLFHLKHFPMD LRTLTCQLAYFQSNGTECCPVNWVEFGGSCYWFSRDGLTWAEADQYCQLENAHLLVINSREEQDFVVKHRSQF HIWIGLTDRDGSWKWVDGTDYRSNYRNWAFTQPDNWQGHEQGGGEDCAEILSDGHWNDNFCQQVNRWVCE KRRNITHHHHHHH | pTT5, pSLX235a | 64 |
| ratA::6xHis | 1-301 | 6xHis | MEKDFQDIQQLDSEENDHQLIGDEEQGSHVQNLRTENPRWGGQPPSRPFPQRLCSKFRLSLLALAFNILLLVVIC VVSSQSMQLQKEFWTLKETLSNFSTTTLMEFKALDSHGGSRNDNLTSWETILEKKQKDIKADHSTLLFHLKHFPL DLRTLTCQLAFFLSNGTECCPVNWVEFGGSCYWFSRDGLTWAEADQYCQMENAHLLVINSREEQEFVVKHRGA FHIWIGLTDKDGSWKWVDGTEYRSNFKNWAFTQPDNWQGHEEGGSEDCAEILSDGLWNDNFCQQVNRWACE RKRDITYHHHHHH | pTT5, pSLX235a | 65 |
| dogASGR2::6xHis | 1-302 | 6xHis | MAKDCQDIQQLDSEDSDQQLGRGEGPGPRGHGPRREDRFCRRLPPHQPLLLQRLCSGYRLNLLVLGFNVLMLV AICVIGSQRAQLEEELRILKENFSHFSSGVLMELGVLLSDGGGASSQLTSLEAKLEKQQRDVKADHATLLLHLKHF PSDLRILTCQVAFFQSNGTDCCPVNWLEYEGSCYWFSRSGKTWEEAEKYCQLESAHLVVVNSREEQKFILQHTN PFDTWIGLTDSDGSWRWVDGTDYQQSYKNWAATQPDDWQGHEVGGGEDCAEVRANGRWNDNFCKQVQRW VCEMRRNVTGHHHHHH | pTT5, pSLX235a | 66 |

EP 4 435 105 A2

FIGURE 48

| dogASGR2 | 1-302 | None | MAKDCQDIQQLDSEDSDQQLGRGEGPGPRGHGPRREDRFCRRLPPHQPLLLQRLCSGYRLNLLVLGFNVLMLV AICVIGSQRAQLEEELRILKENFSHFSSGVLMELGVLLSDGGGASSQLTSLEAKLEKQQRDVKADHATLLLHLKHF PSDLRILTCQVAFFQSNGTDCCPVNWLEYEGSCYWFSRSGKTWEEAEKYCQLESAHLVVVNSREEQKFILQHTN PFDTWIGLTDSDGSWRWVDGTDYQQSYKNWAATQPDDWQGHEVGGGEDCAEVRANGRWNDNFCKQVQRW VCEMRRNVTG | pTT5, pSLX235a | 67 |
| cyASGR2 | 1-306 | None | MAKDFQDIQQLSSEENDHPFHRGEGPGPRGLNLRRGNPSLKGPPPAQPLAQRLCSMVRFSLLALSFNILLLVAIC VIGSQSAQLQAELWSLKEAFSNFSSSTLMEVHALGTHGGSVGDKITSLGDKLEKQQQDLKADHDILLLHLKHFPV DLRFVACQMELLHSNGSQRTCCPVNWVEHQGSCYWFSRSGKAWAEAEKYCQLENSHLVVINSWEEQKFIVQH TNLFNTWIGLTDSDGSWKWVDGTDYRHNYKNWAVTQPDDWHGHELGGSEDCVEVRPDGRWNDDFCLQVHR WVCEKRRNATGEAA | pTT5, pSLX235a | 68 |
| cyASGR2(L225P)::6xHis | 1-306 | 6xHis | MAKDFQDIQQLSSEENDHPFHRGEGPGPRGLNLRRGNPSLKGPPPAQPLAQRLCSMVRFSLLALSFNILLLVAIC VIGSQSAQLQAELWSLKEAFSNFSSSTLMEVHALGTHGGSVGDKITSLGDKLEKQQQDLKADHDILLLHLKHFPV DLRFVACQMELLHSNGSQRTCCPVNWVEHQGSCYWFSRSGKAWAEAEKYCQLENSHLVVINSWEEQKFIVQH TNPFNTWIGLTDSDGSWKWVDGTDYRHNYKNWAVTQPDDWHGHELGGSEDCVEVRPDGRWNDDFCLQVHR WVCEKRRNATGEAAHHHHHH | pTT5, pSLX235a | 69 |
| pigASGR2::6xHis | 1-283 | 6xHis | MARDFQDIQQLDSEENDHQLGRGTPLPQPLVLQRLCSKLRLSLLVLGFNVLMLVAVCVVGSQRTQLQMELQTLK ETFSNFSSSLLMEMLTLSTRGGSAGDKVTSLEAKMEKQQQDLKADHATLRLHLKHFPRDVRTLTCRLVFLQSNG TECCPVNWVDYEGSCYWFSRSGKAWAEAEKYCQLENAHLVVINSREEQKFIAQRTNPFQTWIGLTDSDGSWK WVDGTDYGNGYKNWGALQPDDWQGHELGGSEDCVEIQGDGRWNDDFCQQVKRWVCEMKQNITMHHHHHH | pTT5, pSLX235a | 70 |
| cyASGR2(L225P) | 1-306 | None | MAKDFQDIQQLSSEENDHPFHRGEGPGPRGLNLRRGNPSLKGPPPAQPLAQRLCSMVRFSLLALSFNILLLVAIC VIGSQSAQLQAELWSLKEAFSNFSSSTLMEVHALGTHGGSVGDKITSLGDKLEKQQQDLKADHDILLLHLKHFPV DLRFVACQMELLHSNGSQRTCCPVNWVEHQGSCYWFSRSGKAWAEAEKYCQLENSHLVVINSWEEQKFIVQH TNPFNTWIGLTDSDGSWKWVDGTDYRHNYKNWAVTQPDDWHGHELGGSEDCVEVRPDGRWNDDFCLQVHR WVCEKRRNATGEAA | pTT5, pSLX235a | 71 |
| pigASGR2 | 1-283 | None | MARDFQDIQQLDSEENDHQLGRGTPLPQPLVLQRLCSKLRLSLLVLGFNVLMLVAVCVVGSQRTQLQMELQTLK ETFSNFSSSLLMEMLTLSTRGGSAGDKVTSLEAKMEKQQQDLKADHATLRLHLKHFPRDVRTLTCRLVFLQSNG TECCPVNWVDYEGSCYWFSRSGKAWAEAEKYCQLENAHLVVINSREEQKFIAQRTNPFQTWIGLTDSDGSWK WVDGTDYGNGYKNWGALQPDDWQGHELGGSEDCVEIQGDGRWNDDFCQQVKRWVCEMKQNITM | pTT5, pSLX235a | 72 |
| dogASGR1 | 1-284 | None | MTNDYQDLQHLDNEDNDHHLRQVPPAPQPLLRRLCSGPCLLLLSLGLSVLLLVVVCVIGSQNSKLRGELQALRET FSNFTASTEVEVKALSSQGGNVGRKMKSLESQLEKQQKDLSEDHSDLLLHVKQFVSDLRSLSCQIAALHGNGSTL TCCPVNWLEYEGSCYWFSRSGKSWPEADKYCQLESAHLVVVNSREEQKFIQHHMGPVNTWMGLTDQSGPWK WVDGTDYETGFKNWRPEQPDDWYGHGLGGGEDCAHFTDDGRWNDDVCQRPYRWVCEAARDPAT | pTT5, pSLX235a | 73 |
| pigASGR1 | 1-286 | None | MTKEYQDLQHLDNEENDQQHRKGPPPQPSLLRRLCSGPCLLLISMGLSLLLLVVVCVIGSQNSKLQEELQALRET FSNLTASTDAKVKTLSMQGGNVGRKMKSLESQLEKQQQDLSEDHSSLLLHVKQFVSDLRSLSCQMAVLQGNGS ERTCCPVNWVGYEGSCYWFSRSGKPWPEAEKYCQLENAHLVVVGSWEEQKFIQHHVGPVNSWIGLTDQSGP WKWVDGTDYESGFKNWRPEQPDDWYGHGLGGGEDCAHFTEDGGWNDDICQRPYRWVCETQRDRDSGS | pTT5, pSLX235a | 74 |
| dogASGR1::Flag | 1-284 | Flag | MTNDYQDLQHLDNEDNDHHLRQVPPAPQPLLRRLCSGPCLLLLSLGLSVLLLVVVCVIGSQNSKLRGELQALRET FSNFTASTEVEVKALSSQGGNVGRKMKSLESQLEKQQKDLSEDHSDLLLHVKQFVSDLRSLSCQIAALHGNGSTL TCCPVNWLEYEGSCYWFSRSGKSWPEADKYCQLESAHLVVVNSREEQKFIQHHMGPVNTWMGLTDQSGPWK WVDGTDYETGFKNWRPEQPDDWYGHGLGGGEDCAHFTDDGRWNDDVCQRPYRWVCEAARDPATDYKDDD DK | pTT5, pSLX235a | 75 |

579

FIGURE 48

| pigASGR1::Flag | 1-286 | Flag | MTKEYQDLQHLDNEENDQQHRKGPPPQPSLLRRLCSGPCLLLISMGLSLLLLVVVCVIGSQNSKLQEELQALRET FSNLTASTDAKVKTLSMQGGNVGRKMKSLESQLEKQQQDLSEDHSSLLLHVKQFVSDLRSLSCQMAVLQGNGS ERTCCPVNWVGYEGSCYWFSRSGKPWPEAEKYCQLENAHLVVVGSWEEQKFIQHHVGPVNSWIGLTDQSGP WKWVDGTDYESGFKNWRPEQPDDWYGHGLGGGEDCAHFTEDGGWNDDICQRPYRWVCETQRDRDSGSDY KDDDDK | pTT5, pSLX235a | 76 |
| --- | --- | --- | --- | --- | --- |
| huASGR1(deCODE) | 1-94. | None | MTKEYQDLQHLDNEESDHHQLRKGPPPPQPLLQRLCSGPRLLLLSLGLSLLLLVVVCVIGSQNSQLQEELRGLRE TFSNFTASTEAQEAMWEER | pJIF1 | 77 |
| huASGR1(deCODE)::Myc | 1-94. | Myc | MTKEYQDLQHLDNEESDHHQLRKGPPPPQPLLQRLCSGPRLLLLSLGLSLLLLVVVCVIGSQNSQLQEELRGLRE TFSNFTASTEAQEAMWEERGQQKLISEEDL | pTT5 | 0 |

EP 4 435 105 A2

FIGURE 49

Table 2A
Standard
IgG Antibody
VL CDRs

| IPS# | Ab | Type | CDR1 | CDR2 | CDR3 |
|---|---|---|---|---|---|
| iPS:451135 | | NA | CGGGCAAGTCGGAGCGTTAGCAGATATTTAAAT | GTTGCATCCCGTTTGCAAAGT | CAACAGAGTGACAGTTTCCCTCTCACT |
| | 21-225_64A11 | | SEQ ID NO:79 | SEQ ID NO:8091 | SEQ ID NO:16103 |
| | | AA | RASRSVSRYLN | VASRLQS | QQSDSFPLT |
| | | | SEQ ID NO:80 | SEQ ID NO:8092 | SEQ ID NO:16104 |
| iPS:451141 | | NA | AAGTCCAGCCAGAGTCTTTTAAAGAGCTCCAACAATAAGAGCTACTTAGCT | TGGGCATCTTCCCGGGAATCC | CAGCAATATTATAGTATTCCTCCCACT |
| | 21-225_164B11 | | SEQ ID NO:81 | SEQ ID NO:8093 | SEQ ID NO:16105 |
| | | AA | KSSQSLLKSSNNKSYLA | WASSRES | QQYYSIPPT |
| | | | SEQ ID NO:82 | SEQ ID NO:8094 | SEQ ID NO:16106 |
| iPS:451137 | | NA | AAGTCCAGCCAGAGTGTTTTATTCAGCTCCAACAATTATAACTACTTAGCT | TGGGCATCTACCCGGGAATCC | CAGCAATATCATAGTTCTCCTCCGACG |
| | 21-225_74A7 | | SEQ ID NO:83 | SEQ ID NO:8095 | SEQ ID NO:16107 |
| | | AA | KSSQSVLFSSNNYNYLA | WASTRES | QQYHSSPPT |
| | | | SEQ ID NO:84 | SEQ ID NO:8096 | SEQ ID NO:16108 |
| iPS:451139 | | NA | AAGTCTAGTCAGAGCCTCCTGCGTAGTGATGGAAAGACCCATTTGTAT | GAAGTTTCCAACCGGTTCTCT | ATGCAAAGTAAACAGCTTCCTCTCACT |
| | 21-225_71A6 | | SEQ ID NO:85 | SEQ ID NO:8097 | SEQ ID NO:16109 |
| | | AA | KSSQSLLRSDGKTHLY | EVSNRFS | MQSKQLPLT |
| | | | SEQ ID NO:86 | SEQ ID NO:8098 | SEQ ID NO:16110 |

581

FIGURE 49

| iPS:451143 | | NA | CCGGCGAGTCAGGGCATTAG CAATTATTTAGCT | GGTGCATTCAATTTGCAC AGT | CAACAGTATAGTTGTTACCC ATTCACT |
|---|---|---|---|---|---|
| | 21-225_66H11 | | SEQ ID NO:87 | SEQ ID NO:8099 | SEQ ID NO:16111 |
| | | AA | PASQGISNYLA | GAFNLHS | QQYSCYPFT |
| | | | SEQ ID NO:88 | SEQ ID NO:8100 | SEQ ID NO:16112 |
| iPS:453445 | | NA | TCTGGAGATAAATTGGGTAA TAAATATGTTTGT | CAAGATAGCAAGCGGCC CTCA | CAGGCGTGGGACAGGAACAC TTATGTGGTG |
| | 21-225_148E10 | | SEQ ID NO:89 | SEQ ID NO:8101 | SEQ ID NO:16113 |
| | | AA | SGDKLGNKYVC | QDSKRPS | QAWDRNTYVV |
| | | | SEQ ID NO:90 | SEQ ID NO:8102 | SEQ ID NO:16114 |
| iPS:453447 | | NA | CGGGGGGGTCAGGGTATTAG CACATGGTTAGCA | GCTGCATCCATTTGCAA AGT | CAACAGGGTAACATTTTCCC ATTCACT |
| | 21-225_65F10 | | SEQ ID NO:91 | SEQ ID NO:8103 | SEQ ID NO:16115 |
| | | AA | RGGQGISTWLA | AASILQS | QQGNIFPFT |
| | | | SEQ ID NO:92 | SEQ ID NO:8104 | SEQ ID NO:16116 |
| iPS:453449 | | NA | CGGACAAGTCAGGGCATTAG AAATGATTTAGGC | GCTGCATCCAGTTTGCTT AGT | CTACAGTATAATAGTTACCC TCCCACC |
| | 21-225_208A2 | | SEQ ID NO:93 | SEQ ID NO:8105 | SEQ ID NO:16117 |
| | | AA | RTSQGIRNDLG | AASSLLS | LQYNSYPPT |
| | | | SEQ ID NO:94 | SEQ ID NO:8106 | SEQ ID NO:16118 |
| iPS:453451 | | NA | CGGGCGAGTCAGGGTATTAG CAAATGGTTAGCC | GCTGCATCCAGTTTGCAA AGT | CAACAGGCTAACAGTTTCCC ATTCACT |
| | 21-225_52G11 | | SEQ ID NO:95 | SEQ ID NO:8107 | SEQ ID NO:16119 |
| | | AA | RASQGISKWLA | AASSLQS | QQANSFPFT |
| | | | SEQ ID NO:96 | SEQ ID NO:8108 | SEQ ID NO:16120 |
| iPS:453453 | | NA | CGGGCGAGTCAGGGTATTAG CAAGTGGTTAGCC | GCTGCATCCAGTTTGCAA AGT | CAACAGGCTAACAGTTTCCC ATTCACT |
| | 21-225_53F2 | | SEQ ID NO:97 | SEQ ID NO:8109 | SEQ ID NO:16121 |
| | | AA | RASQGISKWLA | AASSLQS | QQANSFPFT |
| | | | SEQ ID NO:98 | SEQ ID NO:8110 | SEQ ID NO:16122 |

FIGURE 49

| iPS:468810 | | NA | AGGGCCAGTCAGAGTGTTAA CAGCAACTACTTAGCC | GGTGCATCCAGCAGGGC CACT | CAGCAGTATGAAAGCTCGCC GTGGACG |
|---|---|---|---|---|---|
| | 21-225_74D5 | | SEQ ID NO:99 | SEQ ID NO:8111 | SEQ ID NO:16123 |
| | | AA | RASQSVNSNYLA | GASSRAT | QQYESSPWT |
| | | | SEQ ID NO:100 | SEQ ID NO:8112 | SEQ ID NO:16124 |
| iPS:468812 | | NA | CGGGCAAGTCGAGACATTAG AAATGATTTAGGC | GCTGCATCCAGTTTGCAA AGT | CTACAACATTATAGTTACCC GCTCACT |
| | 21-225_48H4 | | SEQ ID NO:101 | SEQ ID NO:8113 | SEQ ID NO:16125 |
| | | AA | RASRDIRNDLG | AASSLQS | LQHYSYPLT |
| | | | SEQ ID NO:102 | SEQ ID NO:8114 | SEQ ID NO:16126 |
| iPS:468816 | | NA | AAGTCTAGTCAGAGCCTCCT GCATAGTGAAGGAAAGACC TATTTGTAT | GAAGTTTCCAAGCGGCT CTCT | ATGCAAAGTATGCAGCTTCC GATTATC |
| | 21-225_52G8 | | SEQ ID NO:103 | SEQ ID NO:8115 | SEQ ID NO:16127 |
| | | AA | KSSQSLLHSEGKTYLY | EVSKRLS | MQSMQLPII |
| | | | SEQ ID NO:104 | SEQ ID NO:8116 | SEQ ID NO:16128 |
| iPS:468814 | | NA | CGGGCGAGTCAGGGCATTAG CAATTATTTAGCC | GCTGCATCCACTTTGCAA AGT | CAACAGTATAGTGGTTACCC ATTCACT |
| | 21-225_223D11 | | SEQ ID NO:105 | SEQ ID NO:8117 | SEQ ID NO:16129 |
| | | AA | RASQGISNYLA | AASTLQS | QQYSGYPFT |
| | | | SEQ ID NO:106 | SEQ ID NO:8118 | SEQ ID NO:16130 |
| iPS:468822 | | NA | AAGTCTAGTCAGCGCCTCCT GCATGGTGATGGAAAGACCT ATTTGTAT | GAAGTTTCCAACCGGTTC TCT | ATGCAAAGTATACAGGTTCC GTGGACG |
| | 21-225_147E10 | | SEQ ID NO:107 | SEQ ID NO:8119 | SEQ ID NO:16131 |
| | | AA | KSSQRLLHGDGKTYLY | EVSNRFS | MQSIQVPWT |
| | | | SEQ ID NO:108 | SEQ ID NO:8120 | SEQ ID NO:16132 |
| iPS:468824 | | NA | CGGGCAAGTCAGGGCATTAG AAATGATTTAGGC | GCTGCATCCAGTTTGCAA AGT | CTACAGCATAATAGTTACCC GCTCACT |
| | 21-225_73G6 | | SEQ ID NO:109 | SEQ ID NO:8121 | SEQ ID NO:16133 |

FIGURE 49

| | | | | | |
|---|---|---|---|---|---|
| | | AA | RASQGIRNDLG | AASSLQS | LQHNSYPLT |
| | | | SEQ ID NO:110 | SEQ ID NO:8122 | SEQ ID NO:16134 |
| iPS:468818 | | NA | CGGGCAAGTCAGGGCATTAG AAATGATTTAGGC | GCTGCATCCAGTTTGCAA AGT | CTACAGCATAATGATTACCC GTTCACT |
| | 21-225_190C8 | | SEQ ID NO:111 | SEQ ID NO:8123 | SEQ ID NO:16135 |
| | | AA | RASQGIRNDLG | AASSLQS | LQHNDYPFT |
| | | | SEQ ID NO:112 | SEQ ID NO:8124 | SEQ ID NO:16136 |
| iPS:468826 | | NA | CGGGCAAGTCAGGGCATTAG ACATGATTTAGGC | GCTGCATCCAGTTTGCAA AGT | CTACAGCATTATAGTTTCCCT CGGACG |
| | 21-225_201C5 | | SEQ ID NO:113 | SEQ ID NO:8125 | SEQ ID NO:16137 |
| | | AA | RASQGIRHDLG | AASSLQS | LQHYSFPRT |
| | | | SEQ ID NO:114 | SEQ ID NO:8126 | SEQ ID NO:16138 |
| iPS:468828 | | NA | AGGGCCAGTCAGACTGTTAA CAGCAACTTAGCC | GGTGCATCCACCAGGGC CACT | CAGCAGTATAATGACTGGCC GTGCAGT |
| | 21-225_162A10 | | SEQ ID NO:115 | SEQ ID NO:8127 | SEQ ID NO:16139 |
| | | AA | RASQTVNSNLA | GASTRAT | QQYNDWPCS |
| | | | SEQ ID NO:116 | SEQ ID NO:8128 | SEQ ID NO:16140 |
| iPS:468830 | | NA | AGGACCAGTCAGAGTGTTTG GATTAGCGTAGCC | GGTGCAGCCACCAGGGC CACT | CAGCAGTATAATTACTGGCC GCTCACT |
| | 21-225_191G11 | | SEQ ID NO:117 | SEQ ID NO:8129 | SEQ ID NO:16141 |
| | | AA | RTSQSVWISVA | GAATRAT | QQYNYWPLT |
| | | | SEQ ID NO:118 | SEQ ID NO:8130 | SEQ ID NO:16142 |
| iPS:468832 | | NA | CGGGCAAGTCAGGACATTAG AAATTATTTAGGC | GGTGCATCCAGTTTGCA AAGT | CTACAGTATAATAGTTACCC ATTCACT |
| | 21-225_76H10 | | SEQ ID NO:119 | SEQ ID NO:8131 | SEQ ID NO:16143 |
| | | AA | RASQDIRNYLG | GASSLQS | LQYNSYPFT |
| | | | SEQ ID NO:120 | SEQ ID NO:8132 | SEQ ID NO:16144 |
| iPS:468834 | | NA | AGGGCCAGTCAGAGTGTTAA CAGCAACTACTTAGCC | GGTGCATCCAGCAGGGC CACT | CAGCAGTATGAAAGCTCGCC GTGGACG |
| | 21-225_94G10 | | SEQ ID NO:121 | SEQ ID NO:8133 | SEQ ID NO:16145 |

FIGURE 49

| | | | | | |
|---|---|---|---|---|---|
| | | AA | RASQSVNSNYLA | GASSRAT | QQYESSPWT |
| | | | SEQ ID NO:122 | SEQ ID NO:8134 | SEQ ID NO:16146 |
| iPS:468836 | | NA | CGGGCAAGTCAGGGCATAAGAAAAGATTTAGGC | GCTGCATCCAGTTTGCAAAGT | CTACAACATTATCGTTACCCTTTCACT |
| | 21-225_198E3 | | SEQ ID NO:123 | SEQ ID NO:8135 | SEQ ID NO:16147 |
| | | AA | RASQGIRKDLG | AASSLQS | LQHYRYPFT |
| | | | SEQ ID NO:124 | SEQ ID NO:8136 | SEQ ID NO:16148 |
| iPS:468838 | | NA | AGGGCCAGTCAGAGCGTTAACAGCAACTACTTAGCC | GGTGCATCCAGCAGGGCCACT | CAGCAGTATGAAAGCTCGCCGTGGACG |
| | 21-225_80E12 | | SEQ ID NO:125 | SEQ ID NO:8137 | SEQ ID NO:16149 |
| | | AA | RASQSVNSNYLA | GASSRAT | QQYESSPWT |
| | | | SEQ ID NO:126 | SEQ ID NO:8138 | SEQ ID NO:16150 |
| iPS:468840 | | NA | CGGGCAAGTCAGGGCATTAGAAATGATTTAGGC | GCTGCATCCAGTTTGCAAAGT | CTACAGCATAATAGTTACCCTCTCACT |
| | 21-225_200H9 | | SEQ ID NO:127 | SEQ ID NO:8139 | SEQ ID NO:16151 |
| | | AA | RASQGIRNDLG | AASSLQS | LQHNSYPLT |
| | | | SEQ ID NO:128 | SEQ ID NO:8140 | SEQ ID NO:16152 |
| iPS:468820 | | NA | AGGGCCAGTCAGAGTGTTAACAGCAACTACTTAGCC | GGTGCATCCAGCAGGGCCACT | CAGCAGTATGAAAGCTCGCCGTGGACG |
| | 21-225_76E10 | | SEQ ID NO:129 | SEQ ID NO:8141 | SEQ ID NO:16153 |
| | | AA | RASQSVNSNYLA | GASSRAT | QQYESSPWT |
| | | | SEQ ID NO:130 | SEQ ID NO:8142 | SEQ ID NO:16154 |
| iPS:468842 | | NA | CGGGCAAGTCAGGGCATTAGAAATGATTTAGGC | GCTGCATCCAGTTTGCAAAGT | CTACAACATTATAGTTACCCTCGGACG |
| | 21-225_50H4 | | SEQ ID NO:131 | SEQ ID NO:8143 | SEQ ID NO:16155 |
| | | AA | RASQGIRNDLG | AASSLQS | LQHYSYPRT |
| | | | SEQ ID NO:132 | SEQ ID NO:8144 | SEQ ID NO:16156 |
| iPS:468844 | | NA | CGGGCAAGTCAGGGCATTAGAAGTGATTTAGGC | ACTGCATCCAGTTTGCAAAGT | CTACAGTATAATAGTTACCCATTCACT |
| | 21-225_48E10 | | SEQ ID NO:133 | SEQ ID NO:8145 | SEQ ID NO:16157 |

585

FIGURE 49

| | | | | | |
|---|---|---|---|---|---|
| | | AA | RASQGIRSDLG | TASSLQS | LQYNSYPFT |
| | | | SEQ ID NO:134 | SEQ ID NO:8146 | SEQ ID NO:16158 |
| iPS:468846 | 21-225_53B10 | NA | CGGGCAAGTCAGGACATTAG AAATTATTTAGGC | GGTGCATCCAGTTTGCA AAGT | CTACAGTATAATAGTTACCC ATTCACT |
| | | | SEQ ID NO:135 | SEQ ID NO:8147 | SEQ ID NO:16159 |
| | | AA | RASQDIRNYLG | GASSLQS | LQYNSYPFT |
| | | | SEQ ID NO:136 | SEQ ID NO:8148 | SEQ ID NO:16160 |
| iPS:468848 | 21-225_54B1 | NA | CGGGCAAGTCAGAACATTAG CAGCTATTTAAAT | GCTGCATCCAGTTTGCAT AGT | CAACAGAGTTACAGAACCCC TCTGTGGACG |
| | | | SEQ ID NO:137 | SEQ ID NO:8149 | SEQ ID NO:16161 |
| | | AA | RASQNISSYLN | AASSLHS | QQSYRTPLWT |
| | | | SEQ ID NO:138 | SEQ ID NO:8150 | SEQ ID NO:16162 |
| iPS:468850 | 21-225_63F4 | NA | AAGTCCAGCCAGAGTGTTTT ATCCAGCTCCAACAATAACA ACTACTTAGCT | TGGGCATCTACCCGGGA ATCC | CAGCAATATTATACTACTCC GTGCAGT |
| | | | SEQ ID NO:139 | SEQ ID NO:8151 | SEQ ID NO:16163 |
| | | AA | KSSQSVLSSSNNNNYLA | WASTRES | QQYYTTPCS |
| | | | SEQ ID NO:140 | SEQ ID NO:8152 | SEQ ID NO:16164 |
| iPS:468852 | 21-225_71F3 | NA | AAGTCCAGCCAGAGTGTTTT ATCCAACTCCAACAATAACA ACTACTTAGCT | TGGGCATCTACCCGGGA ATCC | CAGCAATATTATACTACTCC GTGCAGT |
| | | | SEQ ID NO:141 | SEQ ID NO:8153 | SEQ ID NO:16165 |
| | | AA | KSSQSVLSNSNNNNYLA | WASTRES | QQYYTTPCS |
| | | | SEQ ID NO:142 | SEQ ID NO:8154 | SEQ ID NO:16166 |
| iPS:468854 | 21-225_72C4 | NA | AGGTCTGGTCAAAGCCTCGT ATACAGTGATGGAAACACCT ACTTGAAT | GAGGTTTCTAAGTGGGA CTCT | ATGCAAGGTACACACTGGCC GCTCACT |
| | | | SEQ ID NO:143 | SEQ ID NO:8155 | SEQ ID NO:16167 |
| | | AA | RSGQSLVYSDGNTYLN | EVSKWDS | MQGTHWPLT |
| | | | SEQ ID NO:144 | SEQ ID NO:8156 | SEQ ID NO:16168 |

FIGURE 49

| iPS:468856 | | NA | AGGTCTAGTCAAAGCCTCGT TTACAGTGTTGGAAACACCT CCTTGAGT | AAGGTTTCTAACTGGGA CTCT | ATGCAAGGTACACACTGGCC ATTCACT |
|---|---|---|---|---|---|
| | 21-225_77C9 | | SEQ ID NO:145 | SEQ ID NO:8157 | SEQ ID NO:16169 |
| | | AA | RSSQSLVYSVGNTSLS | KVSNWDS | MQGTHWPFT |
| | | | SEQ ID NO:146 | SEQ ID NO:8158 | SEQ ID NO:16170 |
| iPS:468858 | | NA | CGGGCAAGTCGGGGCATTAG AGATGATTTAGGC | GCTGCATCCAGTTTGCAG AGT | CTACAGCATTATAGTTATCC TCGGACG |
| | 21-225_148C9 | | SEQ ID NO:147 | SEQ ID NO:8159 | SEQ ID NO:16171 |
| | | AA | RASRGIRDDLG | AASSLQS | LQHYSYPRT |
| | | | SEQ ID NO:148 | SEQ ID NO:8160 | SEQ ID NO:16172 |
| iPS:468860 | | NA | CGGGCAAGTCAGGGCATTAG AAATGATTTAGGC | GCTGCATCCAGTTTGCAA AGT | CTACAACATTATAGTTACCC TCGGACG |
| | 21-225_224E7 | | SEQ ID NO:149 | SEQ ID NO:8161 | SEQ ID NO:16173 |
| | | AA | RASQGIRNDLG | AASSLQS | LQHYSYPRT |
| | | | SEQ ID NO:150 | SEQ ID NO:8162 | SEQ ID NO:16174 |
| iPS:468862 | | NA | ACTGGAACCAGCAGTGACGT TGGTGGTTATAATTTTGTCT CC | GAGGTCAGTAATCGGCC CTCA | AGCTCATATACAAGCAGCTA CACTTGGGTG |
| | 21-225_178H8 | | SEQ ID NO:151 | SEQ ID NO:8163 | SEQ ID NO:16175 |
| | | AA | TGTSSDVGGYNFVS | EVSNRPS | SSYTSSYTWV |
| | | | SEQ ID NO:152 | SEQ ID NO:8164 | SEQ ID NO:16176 |
| iPS:468864 | | NA | TCTGGAAGCAGCTCCAACAT CGGAAGTAATACTGTAAAC | AGTAATAATCAGCGGCC CTCA | GCAGCATGGGATGACAGCCT GAATGGTCCG |
| | 21-225_60D6 | | SEQ ID NO:153 | SEQ ID NO:8165 | SEQ ID NO:16177 |
| | | AA | SGSSSNIGSNTVN | SNNQRPS | AAWDDSLNGP |
| | | | SEQ ID NO:154 | SEQ ID NO:8166 | SEQ ID NO:16178 |
| iPS:468866 | | NA | ACTGGAGATGCAATGCCGAA AAAATATGCTTAT | GAGGACAGCAAGCGACC CTCC | AACTCAACAGACAGCAGTGG TAATCGGGTG |

FIGURE 49

| | 21-225_190C1 | | SEQ ID NO:155 | SEQ ID NO:8167 | SEQ ID NO:16179 |
|---|---|---|---|---|---|
| | | AA | TGDAMPKKYAY | EDSKRPS | NSTDSSGNRV |
| | | | SEQ ID NO:156 | SEQ ID NO:8168 | SEQ ID NO:16180 |
| iPS:468868 | 21-225_74A1 | NA | CGGGCAAGTCAGGGCATTAG AAATGATTTAGGC | GCTGCATCCAGTTTGCAA AGT | CTACAGCATGATAGTTACCC TCTCACT |
| | | | SEQ ID NO:157 | SEQ ID NO:8169 | SEQ ID NO:16181 |
| | | AA | RASQGIRNDLG | AASSLQS | LQHDSYPLT |
| | | | SEQ ID NO:158 | SEQ ID NO:8170 | SEQ ID NO:16182 |
| iPS:468870 | 21-225_74A8 | NA | AAGTCCAGCCAGAGTGTTTT GTACAGCTCCAACAGTCACA ACTACTTAGCT | TGGGCATCTACCCGGGA ATCC | CAGCAATATTATAGTACTCC GTGCAGT |
| | | | SEQ ID NO:159 | SEQ ID NO:8171 | SEQ ID NO:16183 |
| | | AA | KSSQSVLYSSNSHNYLA | WASTRES | QQYYSTPCS |
| | | | SEQ ID NO:160 | SEQ ID NO:8172 | SEQ ID NO:16184 |
| iPS:472730 | 21-225_14B1_LC1 | NA | CGGGCAAGTCAGGACATTAG AGATAATTTAGGC | ACTGCATACAGTTTGCA AAGT | CTACAACATTATAATTACCC GCTCACT |
| | | | SEQ ID NO:161 | SEQ ID NO:8173 | SEQ ID NO:16185 |
| | | AA | RASQDIRDNLG | TAYSLQS | LQHYNYPLT |
| | | | SEQ ID NO:162 | SEQ ID NO:8174 | SEQ ID NO:16186 |
| iPS:472731 | 21-225_14B1_LC2 | NA | TCTGGAGATAAATTGGGGGA TAAATATGCTTAC | CAAGATAGGAAGCGCCC CTCA | CAGGCGTGGGACAACAGCAC TGTGGTG |
| | | | SEQ ID NO:163 | SEQ ID NO:8175 | SEQ ID NO:16187 |
| | | AA | SGDKLGDKYAY | QDRKRPS | QAWDNSTVV |
| | | | SEQ ID NO:164 | SEQ ID NO:8176 | SEQ ID NO:16188 |
| iPS:472732 | 21-225_2B10_LC1 | NA | AGGTCTAGTCAAAGCCTCGT ATACAGTGATGGAAACACCT TCTTGAAT | AAGGTTTCTAACTGGGA CTCT | ATACAAGGTACGCACTGGCC TTTCCCC |
| | | | SEQ ID NO:165 | SEQ ID NO:8177 | SEQ ID NO:16189 |
| | | AA | RSSQSLVYSDGNTFLN | KVSNWDS | IQGTHWPFP |
| | | | SEQ ID NO:166 | SEQ ID NO:8178 | SEQ ID NO:16190 |

FIGURE 49

| iPS:472733 | 21-225_2B10_LC2 | NA | ACTGGAACCAGCAGTGACGT TGGTGGTTATAATTTTGTCT CC | GAGGTCAGTAATCGGCC CTCA | AGCTCATATACAAGCACCGG CACTGTGGTA |
|---|---|---|---|---|---|
| | | | SEQ ID NO:167 | SEQ ID NO:8179 | SEQ ID NO:16191 |
| | | AA | TGTSSDVGGYNFVS | EVSNRPS | SSYTSTGTVV |
| | | | SEQ ID NO:168 | SEQ ID NO:8180 | SEQ ID NO:16192 |
| iPS:473253 | 21-225_7C3_LC1 | NA | CGGGCAAGTCAGGGCATTAG AAGTGATTTAGGC | GCTGCATCCAGTTTGCAA AGT | CTACAGCATAATAGTTACCT CCCCATCACC |
| | | | SEQ ID NO:169 | SEQ ID NO:8181 | SEQ ID NO:16193 |
| | | AA | RASQGIRSDLG | AASSLQS | LQHNSYLPIT |
| | | | SEQ ID NO:170 | SEQ ID NO:8182 | SEQ ID NO:16194 |
| iPS:473254 | 21-225_7C3_LC2 | NA | CGGGCGAGTCAGGGTATTAG CAGCTGGTTAGCC | GCTGCATCCAGGTTGCA AAGT | CAACAGGCTAACAGTTTCCC ATTCACT |
| | | | SEQ ID NO:171 | SEQ ID NO:8183 | SEQ ID NO:16195 |
| | | AA | RASQGISSWLA | AASRLQS | QQANSFPFT |
| | | | SEQ ID NO:172 | SEQ ID NO:8184 | SEQ ID NO:16196 |
| iPS:473255 | 21-225_9F12_LC1 | NA | CGGGCGAGTCAGGGTATTAG CAGGTGGTTAGCC | GCTGCATCCAGATTGCA AAGT | CAACAGGCTAACAGTTTCCC ATTCACT |
| | | | SEQ ID NO:173 | SEQ ID NO:8185 | SEQ ID NO:16197 |
| | | AA | RASQGISRWLA | AASRLQS | QQANSFPFT |
| | | | SEQ ID NO:174 | SEQ ID NO:8186 | SEQ ID NO:16198 |
| iPS:473256 | 21-225_9F12_LC2 | NA | CGGGCAAGTCAGGGCATTAG AAATGATTTAGGC | GCTGCATCCAGTTTGCAA AGT | CTACAGCATAATAGTTACCT CCCCATCACC |
| | | | SEQ ID NO:175 | SEQ ID NO:8187 | SEQ ID NO:16199 |
| | | AA | RASQGIRNDLG | AASSLQS | LQHNSYLPIT |
| | | | SEQ ID NO:176 | SEQ ID NO:8188 | SEQ ID NO:16200 |
| iPS:472742 | 21-225_30D9_LC2 | NA | TCTGGAGATAAATTGGGGGA TAAATATGTTTAC | CAAGATAGGAAGCGGCC CTCA | CAGGCGTGGGACAACAGCAC TGCGGTA |
| | | | SEQ ID NO:177 | SEQ ID NO:8189 | SEQ ID NO:16201 |
| | | AA | SGDKLGDKYVY | QDRKRPS | QAWDNSTAV |

FIGURE 49

| | | | SEQ ID NO:178 | SEQ ID NO:8190 | SEQ ID NO:16202 |
|---|---|---|---|---|---|
| iPS:472741 | 21-225_30D9_LC1 | NA | AGGTCTAGTCAAAGCCTCGTATCCAGTGATGGAAACACCTTCTTGAAT | AAGGTTTCTAACTGGGACTCT | TTGCAAGGTACACACTGGCCTCTCACC |
| | | | SEQ ID NO:179 | SEQ ID NO:8191 | SEQ ID NO:16203 |
| | | AA | RSSQSLVSSDGNTFLN | KVSNWDS | LQGTHWPLT |
| | | | SEQ ID NO:180 | SEQ ID NO:8192 | SEQ ID NO:16204 |
| iPS:472743 | 21-225_68G6 | NA | TCTGGAGATAAATTGGGGGATAAATATACTTAC | CAAGATAGGAAGCGGCCCTCA | CAGGCGTGGGACAATAGTACTGCGGTA |
| | | | SEQ ID NO:181 | SEQ ID NO:8193 | SEQ ID NO:16205 |
| | | AA | SGDKLGDKYTY | QDRKRPS | QAWDNSTAV |
| | | | SEQ ID NO:182 | SEQ ID NO:8194 | SEQ ID NO:16206 |
| iPS:392573 | 21-225_15G2 | NA | ACTGGAACCAGCAGTGACGTTGGTGGTTATAACTATGTCTCC | GAGGTCAGTAATCGGCCCTCA | ACCTCATATACAAGCACCAGCACTGTGGTC |
| | | | SEQ ID NO:183 | SEQ ID NO:8195 | SEQ ID NO:16207 |
| | | AA | TGTSSDVGGYNYVS | EVSNRPS | TSYTSTSTVV |
| | | | SEQ ID NO:184 | SEQ ID NO:8196 | SEQ ID NO:16208 |
| iPS:392583 | 21-225_10B10 | NA | TCTGGAGATAAATTGGGGAATAAATATGCTTGG | CAAGATAGGAAGCGGCCCTCA | CAGGCGTGGGACAACAGCACTGTGGTT |
| | | | SEQ ID NO:185 | SEQ ID NO:8197 | SEQ ID NO:16209 |
| | | AA | SGDKLGNKYAW | QDRKRPS | QAWDNSTVV |
| | | | SEQ ID NO:186 | SEQ ID NO:8198 | SEQ ID NO:16210 |
| iPS:392585 | 21-225_14H11 | NA | TCTGGAGATAAATTGGGGGAAAAATATGTTTGC | CAAGATACCAAGCGGCCCTCA | CAGGCGTGGGACAGCAGCACTATA |
| | | | SEQ ID NO:187 | SEQ ID NO:8199 | SEQ ID NO:16211 |
| | | AA | SGDKLGEKYVC | QDTKRPS | QAWDSSTI |
| | | | SEQ ID NO:188 | SEQ ID NO:8200 | SEQ ID NO:16212 |
| iPS:392587 | | NA | TCTGGAGAGAAATTGGGGGATAAATATGTTTGT | CAAGATAGCAAGCGGCCCTCA | CAGGCGTGGAACAGCAGCAATGTGGTA |

FIGURE 49

| | 21-225_18G5 | | SEQ ID NO:189 | SEQ ID NO:8201 | SEQ ID NO:16213 |
|---|---|---|---|---|---|
| | | AA | SGEKLGDKYVC | QDSKRPS | QAWNSSNVV |
| iPS:392589 | | NA | SEQ ID NO:190<br>TCTGGAGATAAATTGGGGGA<br>TAAATATGCTTCC | SEQ ID NO:8202<br>CAAGATGGCAAGCGGCC<br>CTCA | SEQ ID NO:16214<br>CAGGCGTGGGACAGCAGCAC<br>TTATGTGGTA |
| | 21-225_27H2 | | SEQ ID NO:191 | SEQ ID NO:8203 | SEQ ID NO:16215 |
| | | AA | SGDKLGDKYAS | QDGKRPS | QAWDSSTYVV |
| iPS:392593 | | NA | SEQ ID NO:192<br>GGGGGAAACAACATTGGAA<br>GTAAAGCTGTGCAC | SEQ ID NO:8204<br>AGCGATAGCAACCGGCC<br>CTCA | SEQ ID NO:16216<br>CAGGTGTGGGACAGTAGTAG<br>TGATCATGTGGTA |
| | 21-225_3E10 | | SEQ ID NO:193 | SEQ ID NO:8205 | SEQ ID NO:16217 |
| | | AA | GGNNIGSKAVH | SDSNRPS | QVWDSSSDHVV |
| iPS:392596 | | NA | SEQ ID NO:194<br>ACCCTAAGCAGTGAGCACAG<br>CACCTACACCATCGAA | SEQ ID NO:8206<br>GTTAAGAGTGATGGCAG<br>CCACAGCAAGGGGGAC | SEQ ID NO:16218<br>GGAGAGAGCCACACGATTGA<br>TGGCCAAGTCGGTGTGGTA |
| | 21-225_12D8 | | SEQ ID NO:195 | SEQ ID NO:8207 | SEQ ID NO:16219 |
| | | AA | TLSSEHSTYTIE | VKSDGSHSKGD | GESHTIDGQVGVV |
| iPS:392598 | | NA | SEQ ID NO:196<br>TCTGGAGATAGATTGGGGGA<br>TAAATATGCTTGG | SEQ ID NO:8208<br>CAAGATCGCAAGCGGCC<br>CTCA | SEQ ID NO:16220<br>CAGGCGTGGGACAGCAGCAC<br>AGTGGTA |
| | 21-225_18E10 | | SEQ ID NO:197 | SEQ ID NO:8209 | SEQ ID NO:16221 |
| | | AA | SGDRLGDKYAW | QDRKRPS | QAWDSSTVV |
| iPS:392618 | | NA | SEQ ID NO:198<br>AAGTCTAGTCAGAGCCTCCT<br>GCATAGTGATGGAAAGACCC<br>ATTTGAAT | SEQ ID NO:8210<br>GAAGTTTCCTACCGGTTC<br>TCT | SEQ ID NO:16222<br>TTTCAAAGTATACAGCTTCC<br>GCTCACT |
| | 21-225_16F10 | | SEQ ID NO:199 | SEQ ID NO:8211 | SEQ ID NO:16223 |
| | | AA | KSSQSLLHSDGKTHLN | EVSYRFS | FQSIQLPLT |
| | | | SEQ ID NO:200 | SEQ ID NO:8212 | SEQ ID NO:16224 |

FIGURE 49

| iPS:392620 | | NA | CGGGCGAGTCAGGGCATTAG CAATTATTTAGCC | GCTGCATCCAGTTTACAA AGT | CAACAGTATCATAGTTACCC ATTCACT |
|---|---|---|---|---|---|
| | 21-225_17H5 | | SEQ ID NO:201 | SEQ ID NO:8213 | SEQ ID NO:16225 |
| | | AA | RASQGISNYLA | AASSLQS | QQYHSYPFT |
| | | | SEQ ID NO:202 | SEQ ID NO:8214 | SEQ ID NO:16226 |
| iPS:392622 | | NA | CGGGCAAGTCAGGGCATTAG AAATGATTTAGGC | GGTGCATCCAGTTTGCA AAGT | CTACAGCATAATAGTTACCC ACTCACT |
| | 21-225_17H8 | | SEQ ID NO:203 | SEQ ID NO:8215 | SEQ ID NO:16227 |
| | | AA | RASQGIRNDLG | GASSLQS | LQHNSYPLT |
| | | | SEQ ID NO:204 | SEQ ID NO:8216 | SEQ ID NO:16228 |
| iPS:392624 | | NA | CGGGCAAGTCAGGACATTAG AAATGATTTAGGC | GCTGCATCCAGTTTGCAA AGT | CTACAGCATTATAGTTATAT GTTCACT |
| | 21-225_17H12 | | SEQ ID NO:205 | SEQ ID NO:8217 | SEQ ID NO:16229 |
| | | AA | RASQDIRNDLG | AASSLQS | LQHYSYMFT |
| | | | SEQ ID NO:206 | SEQ ID NO:8218 | SEQ ID NO:16230 |
| iPS:392626 | | NA | CGGGCAAGTCAGGGCATTAG AAATGATTTAGGC | GCTGCATCCAGTTTGCAA AGT | CTACAGCATAATAGTTACCC GTGGACG |
| | 21-225_18A5 | | SEQ ID NO:207 | SEQ ID NO:8219 | SEQ ID NO:16231 |
| | | AA | RASQGIRNDLG | AASSLQS | LQHNSYPWT |
| | | | SEQ ID NO:208 | SEQ ID NO:8220 | SEQ ID NO:16232 |
| iPS:392628 | | NA | CGGGCAAGTCAGGGCATTAG AAATGATTTAGGC | GCTGCATCCAGTTTGCAA AGT | CTTCAACATGCTAGTTACCC GCTCACT |
| | 21-225_20C2 | | SEQ ID NO:209 | SEQ ID NO:8221 | SEQ ID NO:16233 |
| | | AA | RASQGIRNDLG | AASSLQS | LQHASYPLT |
| | | | SEQ ID NO:210 | SEQ ID NO:8222 | SEQ ID NO:16234 |
| iPS:392630 | | NA | CGGGCAAGTCAGGGCATTAG AAATGATTTAGGC | GCTGCATCCAGTTTGCAA AGT | CTACAGCATAATAGTTACCC GCTCACT |
| | 21-225_20E5 | | SEQ ID NO:211 | SEQ ID NO:8223 | SEQ ID NO:16235 |
| | | AA | RASQGIRNDLG | AASSLQS | LQHNSYPLT |
| | | | SEQ ID NO:212 | SEQ ID NO:8224 | SEQ ID NO:16236 |

FIGURE 49

| iPS:392632 | | NA | CGGGCAAGTCAGGACATTAG AAATCATTTAGGC | GCTGCATCCAGTTTGCAA AGT | CTACAGTATAATAGTTATCC ATTCACT |
|---|---|---|---|---|---|
| | 21-225_16A11 | | SEQ ID NO:213 | SEQ ID NO:8225 | SEQ ID NO:16237 |
| | | AA | RASQDIRNHLG | AASSLQS | LQYNSYPFT |
| | | | SEQ ID NO:214 | SEQ ID NO:8226 | SEQ ID NO:16238 |
| iPS:392634 | | NA | CGGGCAAGTCAGGGCATTAG AAGTGATTTAGGC | GCTGCATCCAGTTTGCAA AGT | CTACAGCAATATAGTTACCC TCGGACG |
| | 21-225_17H3 | | SEQ ID NO:215 | SEQ ID NO:8227 | SEQ ID NO:16239 |
| | | AA | RASQGIRSDLG | AASSLQS | LQQYSYPRT |
| | | | SEQ ID NO:216 | SEQ ID NO:8228 | SEQ ID NO:16240 |
| iPS:392636 | | NA | CGGGCAAGTCAGACCATTAG CAACTATTTAAAT | GCTGCTTCCAGTTTGCAA AGT | CAACAGAGTCACACTTCCCC GCTCACT |
| | 21-225_17A6 | | SEQ ID NO:217 | SEQ ID NO:8229 | SEQ ID NO:16241 |
| | | AA | RASQTISNYLN | AASSLQS | QQSHTSPLT |
| | | | SEQ ID NO:218 | SEQ ID NO:8230 | SEQ ID NO:16242 |
| iPS:392638 | | NA | CGGGCAAGTCAGGTCATTAG AAATGATTTAGGC | GCTGTATCCAGTTTGCAA AGT | CTACAACATAATACTTATCC GCTCACT |
| | 21-225_17F9 | | SEQ ID NO:219 | SEQ ID NO:8231 | SEQ ID NO:16243 |
| | | AA | RASQVIRNDLG | AVSSLQS | LQHNTYPLT |
| | | | SEQ ID NO:220 | SEQ ID NO:8232 | SEQ ID NO:16244 |
| iPS:392640 | | NA | CGGGCAAGTCAGGGCATTAG AAATGATTTAGGC | GCTGCATCCAGTTTACAA AGT | CTACAGCATAATAGTTACCC GCTCACT |
| | 21-225_18A1 | | SEQ ID NO:221 | SEQ ID NO:8233 | SEQ ID NO:16245 |
| | | AA | RASQGIRNDLG | AASSLQS | LQHNSYPLT |
| | | | SEQ ID NO:222 | SEQ ID NO:8234 | SEQ ID NO:16246 |
| iPS:392642 | | NA | CGGACAAGTCAGGGCATTAG AAATGATTTAGGC | GCTGCATCCAGTTTGCAA AGT | CTACAGCATAGTTCTTACCC GCTCACT |
| | 21-225_18C6 | | SEQ ID NO:223 | SEQ ID NO:8235 | SEQ ID NO:16247 |
| | | AA | RTSQGIRNDLG | AASSLQS | LQHSSYPLT |
| | | | SEQ ID NO:224 | SEQ ID NO:8236 | SEQ ID NO:16248 |

FIGURE 49

| iPS:392644 | | NA | CGGGCAAGTCAGGGCATTAG AAATGATTTAGGC | GCTGCATCCAATTTACAA AGT | CTACAGCATAATAGTTTCCC GCTCACT |
|---|---|---|---|---|---|
| | 21-225_19E1 | | SEQ ID NO:225 | SEQ ID NO:8237 | SEQ ID NO:16249 |
| | | AA | RASQGIRNDLG | AASNLQS | LQHNSFPLT |
| | | | SEQ ID NO:226 | SEQ ID NO:8238 | SEQ ID NO:16250 |
| iPS:392646 | | NA | CGGGCAAGTCAGGGCATTAG AAATGATTTAGGC | GCTGCATCCAGTTTCCAA AGT | CTACAGCATAATAGTTACCC GCTCACT |
| | 21-225_20G2 | | SEQ ID NO:227 | SEQ ID NO:8239 | SEQ ID NO:16251 |
| | | AA | RASQGIRNDLG | AASSFQS | LQHNSYPLT |
| | | | SEQ ID NO:228 | SEQ ID NO:8240 | SEQ ID NO:16252 |
| iPS:392648 | | NA | CGGGCAAGTCAGACCATTAG CAACTATTTAAAT | GCTGCATCCAGTTTGCAA AGT | CAACAGAGTCACAGTTCCCC GCTCACT |
| | 21-225_16D11 | | SEQ ID NO:229 | SEQ ID NO:8241 | SEQ ID NO:16253 |
| | | AA | RASQTISNYLN | AASSLQS | QQSHSSPLT |
| | | | SEQ ID NO:230 | SEQ ID NO:8242 | SEQ ID NO:16254 |
| iPS:392650 | | NA | CGGGCGAGTCAGGGTATTGG CAACTGGTTAGCC | GCTGCATCCAGTTTGCAA AGT | CAACAGGCTAACAGTTTCCC TCGGACG |
| | 21-225_17A4 | | SEQ ID NO:231 | SEQ ID NO:8243 | SEQ ID NO:16255 |
| | | AA | RASQGIGNWLA | AASSLQS | QQANSFPRT |
| | | | SEQ ID NO:232 | SEQ ID NO:8244 | SEQ ID NO:16256 |
| iPS:392652 | | NA | CGGGCAAGTCAGAGCATTAA TACTTATTTAAAT | GCTGCATCCAGTTTGCAA AGT | CAACAGAGTTACAGAACCCC CTTTTTCACT |
| | 21-225_17C6 | | SEQ ID NO:233 | SEQ ID NO:8245 | SEQ ID NO:16257 |
| | | AA | RASQSINTYLN | AASSLQS | QQSYRTPFFT |
| | | | SEQ ID NO:234 | SEQ ID NO:8246 | SEQ ID NO:16258 |
| iPS:392654 | | NA | CGGGCAAGTCAGGGCATTAG AAATGATTTAGGC | GCTGCATCCAGTTTACAA AGT | CTACAGCATAATAGTTACCC GCTCACT |
| | 21-225_17A10 | | SEQ ID NO:235 | SEQ ID NO:8247 | SEQ ID NO:16259 |
| | | AA | RASQGIRNDLG | AASSLQS | LQHNSYPLT |
| | | | SEQ ID NO:236 | SEQ ID NO:8248 | SEQ ID NO:16260 |

FIGURE 49

| iPS:392656 | | NA | CGGGCAAGTCAGGGCATTAG AAATGATTTAGGC | GCTGCATCCAGTGTGCA AAGT | CTACAGCATAATAGTTACCC GCTCACT |
|---|---|---|---|---|---|
| | 21-225_1F2 | | SEQ ID NO:237 | SEQ ID NO:8249 | SEQ ID NO:16261 |
| | | AA | RASQGIRNDLG | AASSVQS | LQHNSYPLT |
| | | | SEQ ID NO:238 | SEQ ID NO:8250 | SEQ ID NO:16262 |
| iPS:392658 | | NA | CGGGCAAGTCAGGGCATTAG AAATGATTTAGGC | GCTGCATCCAGTTTGCAA AGT | CTACAGCATAATAGTTACCC GCTCACT |
| | 21-225_18E8 | | SEQ ID NO:239 | SEQ ID NO:8251 | SEQ ID NO:16263 |
| | | AA | RASQGIRNDLG | AASSLQS | LQHNSYPLT |
| | | | SEQ ID NO:240 | SEQ ID NO:8252 | SEQ ID NO:16264 |
| iPS:392660 | | NA | CGGGCAGGTCAGAACATTAT CAACTATTTAAAT | GTTGCATCCAGTTTGCAA AGT | CAACAGAGTTACAGTACCCC TTTCACT |
| | 21-225_19B3 | | SEQ ID NO:241 | SEQ ID NO:8253 | SEQ ID NO:16265 |
| | | AA | RAGQNIINYLN | VASSLQS | QQSYSTPFT |
| | | | SEQ ID NO:242 | SEQ ID NO:8254 | SEQ ID NO:16266 |
| iPS:392664 | | NA | CGGGCAAGTCAGAGCATTAT CACCTATTTAAAT | ACTGCATCCAGTTTGCAA AGT | CAACAGACTTACAGTCCCCC GCTCACT |
| | 21-225_20F6 | | SEQ ID NO:243 | SEQ ID NO:8255 | SEQ ID NO:16267 |
| | | AA | RASQSIITYLN | TASSLQS | QQTYSPPLT |
| | | | SEQ ID NO:244 | SEQ ID NO:8256 | SEQ ID NO:16268 |
| iPS:392666 | | NA | CGGGCAAGTCAGGGCATTAG AAATGATTTAGGC | GCTGCATCCAGTGTACA AAGT | CTACAGCATAATAGTTACCC GCTCACT |
| | 21-225_16F11 | | SEQ ID NO:245 | SEQ ID NO:8257 | SEQ ID NO:16269 |
| | | AA | RASQGIRNDLG | AASSVQS | LQHNSYPLT |
| | | | SEQ ID NO:246 | SEQ ID NO:8258 | SEQ ID NO:16270 |
| iPS:392668 | | NA | CGGGCAAGTCAGAACATTAG TAGTTATTTAAAT | GGTGCATCCAGTTTGCA AACT | CAACAGAGTTACAGAACCCC CTTTTTCACT |
| | 21-225_17B4 | | SEQ ID NO:247 | SEQ ID NO:8259 | SEQ ID NO:16271 |
| | | AA | RASQNISSYLN | GASSLQT | QQSYRTPFFT |
| | | | SEQ ID NO:248 | SEQ ID NO:8260 | SEQ ID NO:16272 |

FIGURE 49

| iPS:392674 | | NA | CGGGCAAGTCAGGGCATTAG AAATGATTTAGGC | ACTGCATCCAGTTTGCAA AGT | CTACAACATAATAGTTACCC GTGGACG |
|---|---|---|---|---|---|
| | 21-225_18C2 | | SEQ ID NO:249 | SEQ ID NO:8261 | SEQ ID NO:16273 |
| | | AA | RASQGIRNDLG | TASSLQS | LQHNSYPWT |
| | | | SEQ ID NO:250 | SEQ ID NO:8262 | SEQ ID NO:16274 |
| iPS:392676 | | NA | CGGGCAAGTCAGGGCATAA GAAATGATTTAGGC | GCTGTTTCCAGTTTGCAA AGT | CTACAGCATGCCAGTTACCC GCTCACT |
| | 21-225_19F3 | | SEQ ID NO:251 | SEQ ID NO:8263 | SEQ ID NO:16275 |
| | | AA | RASQGIRNDLG | AVSSLQS | LQHASYPLT |
| | | | SEQ ID NO:252 | SEQ ID NO:8264 | SEQ ID NO:16276 |
| iPS:392678 | | NA | CGGGCAAGTCAGAGCATTAG TAGTTATTTATAT | GCTGCATCCAGTTTGCAA AGT | CAACAGAGTTACAGTGCCCC TCCATTCACT |
| | 21-225_20F3 | | SEQ ID NO:253 | SEQ ID NO:8265 | SEQ ID NO:16277 |
| | | AA | RASQSISSYLY | AASSLQS | QQSYSAPPFT |
| | | | SEQ ID NO:254 | SEQ ID NO:8266 | SEQ ID NO:16278 |
| iPS:392680 | | NA | CGGGCAAGTCAGGGCATTAG AAATGATTTAGGC | GCTGCATCCAGTTTGCAA AGT | CTACAGCATAATAGTTACCC GCTCACT |
| | 21-225_20A7 | | SEQ ID NO:255 | SEQ ID NO:8267 | SEQ ID NO:16279 |
| | | AA | RASQGIRNDLG | AASSLQS | LQHNSYPLT |
| | | | SEQ ID NO:256 | SEQ ID NO:8268 | SEQ ID NO:16280 |
| iPS:392682 | | NA | CGGGCGAGTCAGGCCATTAA CACTTATTTAGCC | GCTGCATCCAGTTTGCAA AGT | CAACAGTATTATAGTTATCC GCTCACT |
| | 21-225_16A12 | | SEQ ID NO:257 | SEQ ID NO:8269 | SEQ ID NO:16281 |
| | | AA | RASQAINTYLA | AASSLQS | QQYYSYPLT |
| | | | SEQ ID NO:258 | SEQ ID NO:8270 | SEQ ID NO:16282 |
| iPS:392684 | | NA | CGGGCAAGTCAGGGCATTAG AAATGATTTAGGC | GCTGCATCCAGTTTGCAA AGT | CTACAGCATAATAGTTACCC ATTCACT |
| | 21-225_17F4 | | SEQ ID NO:259 | SEQ ID NO:8271 | SEQ ID NO:16283 |
| | | AA | RASQGIRNDLG | AASSLQS | LQHNSYPFT |
| | | | SEQ ID NO:260 | SEQ ID NO:8272 | SEQ ID NO:16284 |

FIGURE 49

| iPS:392686 | | NA | CGGGCAAGTCAGGGCATTAG AAATGATTTAGGC | GCTGCATCCAGTTTGCAA AGT | CTACAGCATAATAGTTACCC GTGGACG |
|---|---|---|---|---|---|
| | 21-225_17C7 | | SEQ ID NO:261 | SEQ ID NO:8273 | SEQ ID NO:16285 |
| | | AA | RASQGIRNDLG | AASSLQS | LQHNSYPWT |
| | | | SEQ ID NO:262 | SEQ ID NO:8274 | SEQ ID NO:16286 |
| iPS:392690 | | NA | CGGGCAAGTCAGGGCATTAG AAATGATTTAGGC | GCTGCATCCAGTTTGCAA AGT | CTACAGCATAATAGTTACCC GTGGACG |
| | 21-225_18F2 | | SEQ ID NO:263 | SEQ ID NO:8275 | SEQ ID NO:16287 |
| | | AA | RASQGIRNDLG | AASSLQS | LQHNSYPWT |
| | | | SEQ ID NO:264 | SEQ ID NO:8276 | SEQ ID NO:16288 |
| iPS:392692 | | NA | CGGGCGAGTCAGGACATTAG CTATTATTTAGCC | GTTGCATCCAGTTTGCAA AGT | TTACAGTATAATAGTTACCC ATTCACT |
| | 21-225_18G10 | | SEQ ID NO:265 | SEQ ID NO:8277 | SEQ ID NO:16289 |
| | | AA | RASQDISYYLA | VASSLQS | LQYNSYPFT |
| | | | SEQ ID NO:266 | SEQ ID NO:8278 | SEQ ID NO:16290 |
| iPS:392694 | | NA | CGGGCAAGTCAGAACATTAT CAACTATTTAAAT | GTTGCATCCAATTTACAA GGT | CAACAGAGTTACAGTACCCC TTTCACT |
| | 21-225_19A5 | | SEQ ID NO:267 | SEQ ID NO:8279 | SEQ ID NO:16291 |
| | | AA | RASQNIINYLN | VASNLQG | QQSYSTPFT |
| | | | SEQ ID NO:268 | SEQ ID NO:8280 | SEQ ID NO:16292 |
| iPS:392696 | | NA | CGGGCAAGTCAGAGCATTAT CAACTATTTAAAT | GCTGCATCCAGTTTGCAC AGT | CAACAGAGTTACAGAACCCC CTTATTCACT |
| | 21-225_20A4 | | SEQ ID NO:269 | SEQ ID NO:8281 | SEQ ID NO:16293 |
| | | AA | RASQSIINYLN | AASSLHS | QQSYRTPLFT |
| | | | SEQ ID NO:270 | SEQ ID NO:8282 | SEQ ID NO:16294 |
| iPS:392700 | | NA | CGGGCAAGTCAGGGCATTAG AAATGATTTAGGC | GCTGCATCCAGTTTACAA AGT | CTACAGCATAATAGTTACCC GCTCACT |
| | 21-225_16E12 | | SEQ ID NO:271 | SEQ ID NO:8283 | SEQ ID NO:16295 |
| | | AA | RASQGIRNDLG | AASSLQS | LQHNSYPLT |
| | | | SEQ ID NO:272 | SEQ ID NO:8284 | SEQ ID NO:16296 |

FIGURE 49

| iPS:392702 | | NA | CGGGCAAGTCAGACCATTAG TAGTTTTTTAAAT | GCTGCTTCCAGTTTGCAA AGT | CAACAGAGTTACAGAACCCC CTTTTTCACT |
|---|---|---|---|---|---|
| | 21-225_17F7 | | SEQ ID NO:273 | SEQ ID NO:8285 | SEQ ID NO:16297 |
| | | AA | RASQTISSFLN | AASSLQS | QQSYRTPFFT |
| | | | SEQ ID NO:274 | SEQ ID NO:8286 | SEQ ID NO:16298 |
| iPS:392704 | | NA | CGGGCAAGTCGGACCATTAA CAACTATTTAAAT | GCTACATCCAGTTTACAA AGT | CAACAGACTTACAGTACCCC CTTATTCGCT |
| | 21-225_17F11 | | SEQ ID NO:275 | SEQ ID NO:8287 | SEQ ID NO:16299 |
| | | AA | RASRTINNYLN | ATSSLQS | QQTYSTPLFA |
| | | | SEQ ID NO:276 | SEQ ID NO:8288 | SEQ ID NO:16300 |
| iPS:392706 | | NA | CGGGCAAGTCAGGGCATTAG AAATGATTTAGGC | GTTGCATCCAGTTTGCAA AGT | CTACAGCATAGTAGTTACCC GCTCACT |
| | 21-225_18A3 | | SEQ ID NO:277 | SEQ ID NO:8289 | SEQ ID NO:16301 |
| | | AA | RASQGIRNDLG | VASSLQS | LQHSSYPLT |
| | | | SEQ ID NO:278 | SEQ ID NO:8290 | SEQ ID NO:16302 |
| iPS:392708 | | NA | CGGGCGAGTCAGGGCATTAG CTATTATTTAGCC | GTTGCATCCAGTTTGCAA AGT | CAACAGTATAATACTTACCC ATTCACT |
| | 21-225_18D11 | | SEQ ID NO:279 | SEQ ID NO:8291 | SEQ ID NO:16303 |
| | | AA | RASQGISYYLA | VASSLQS | QQYNTYPFT |
| | | | SEQ ID NO:280 | SEQ ID NO:8292 | SEQ ID NO:16304 |
| iPS:392710 | | NA | CGGGCAAGTCAGGGCATTAG AACTGATTTAGGC | ACTGCATCCAGTTTGCAA AGT | CTACAGCATAATGGTTACCC GTGGACG |
| | 21-225_19A10 | | SEQ ID NO:281 | SEQ ID NO:8293 | SEQ ID NO:16305 |
| | | AA | RASQGIRTDLG | TASSLQS | LQHNGYPWT |
| | | | SEQ ID NO:282 | SEQ ID NO:8294 | SEQ ID NO:16306 |
| iPS:392714 | | NA | CGGGCGAGTCAGGACATTAG CAATTATTTAGCC | GCTGCATCCAGTTTGCAA AGT | CAACAGTATCATAGTTTCCC ATTCACT |
| | 21-225_16G12 | | SEQ ID NO:283 | SEQ ID NO:8295 | SEQ ID NO:16307 |
| | | AA | RASQDISNYLA | AASSLQS | QQYHSFPFT |
| | | | SEQ ID NO:284 | SEQ ID NO:8296 | SEQ ID NO:16308 |

FIGURE 49

| iPS:392716 | | NA | CGGGCAAGTCAGGGCATTAG AAATGATTTAGGC | GCTGCATCCAATTTGCAA AGT | CTACAGCATAATAGTTACCC GCTCACT |
|---|---|---|---|---|---|
| | 21-225_17B5 | | SEQ ID NO:285 | SEQ ID NO:8297 | SEQ ID NO:16309 |
| | | AA | RASQGIRNDLG | AASNLQS | LQHNSYPLT |
| | | | SEQ ID NO:286 | SEQ ID NO:8298 | SEQ ID NO:16310 |
| iPS:392718 | | NA | AGGTCTAGTCAGAGCCTCCT GCATAGTAATGGGAACAACT CTTTGGAT | TTGGGTTCTCATCGGGCC TCC | ATGCAAGTTCTACAAACTCC TCCCCTCACT |
| | 21-225_17B8 | | SEQ ID NO:287 | SEQ ID NO:8299 | SEQ ID NO:16311 |
| | | AA | RSSQSLLHSNGNNSLD | LGSHRAS | MQVLQTPPLT |
| | | | SEQ ID NO:288 | SEQ ID NO:8300 | SEQ ID NO:16312 |
| iPS:392720 | | NA | CGGGCAAGTCAGAGCATTAG TAGTTATTTAAAT | GCTGCATCCAGTTTGCAA AGT | CAACAGAGTTACAATACCCC CTTATTCACT |
| | 21-225_17A12 | | SEQ ID NO:289 | SEQ ID NO:8301 | SEQ ID NO:16313 |
| | | AA | RASQSISSYLN | AASSLQS | QQSYNTPLFT |
| | | | SEQ ID NO:290 | SEQ ID NO:8302 | SEQ ID NO:16314 |
| iPS:392722 | | NA | CGGGCAAGTCAGAGCATTAG TAGTTATTTAAAT | GCTGCATCCAGTTTACAA AGT | CAACAGAGTTACAGAACCCC CTTTTTCACT |
| | 21-225_18E12 | | SEQ ID NO:291 | SEQ ID NO:8303 | SEQ ID NO:16315 |
| | | AA | RASQSISSYLN | AASSLQS | QQSYRTPFFT |
| | | | SEQ ID NO:292 | SEQ ID NO:8304 | SEQ ID NO:16316 |
| iPS:392726 | | NA | CGGGCGAGTCAGGGCATTAA CAATTATTTAGCC | GCTGCATCCACTTTGCAA TCA | CAAAAGTATAACAGTGCCCC TCCGATCACC |
| | 21-225_20B5 | | SEQ ID NO:293 | SEQ ID NO:8305 | SEQ ID NO:16317 |
| | | AA | RASQGINNYLA | AASTLQS | QKYNSAPPIT |
| | | | SEQ ID NO:294 | SEQ ID NO:8306 | SEQ ID NO:16318 |
| iPS:392728 | | NA | CGGGCGAGTCAGGGTATTAG CAGCTGGTTAGCC | GCTGCATCCAGTTTGCAA AGT | CAACAGGCTAACAGTTTCCC TCGGACG |
| | 21-225_20F7 | | SEQ ID NO:295 | SEQ ID NO:8307 | SEQ ID NO:16319 |
| | | AA | RASQGISSWLA | AASSLQS | QQANSFPRT |

FIGURE 49

| | | | SEQ ID NO:296 | SEQ ID NO:8308 | SEQ ID NO:16320 |
|---|---|---|---|---|---|
| iPS:392730 | | NA | CGGGCAAGTCAGAACATTAACAATTATTTAAAT | ACTACATCTAGTTTACAAAGT | CAACAGAGTTACACTACCCCCACGTGGACG |
| | 21-225_17A1 | | SEQ ID NO:297 | SEQ ID NO:8309 | SEQ ID NO:16321 |
| | | AA | RASQNINNYLN | TTSSLQS | QQSYTTPTWT |
| | | | SEQ ID NO:298 | SEQ ID NO:8310 | SEQ ID NO:16322 |
| iPS:392732 | | NA | CGGGCAAGTCAGGGCATTAGAAATGATTTAGGC | ACTGCATCCAGTTTGCAAAGT | CTACAACATAATAGTTACCCGTTGACG |
| | 21-225_17E5 | | SEQ ID NO:299 | SEQ ID NO:8311 | SEQ ID NO:16323 |
| | | AA | RASQGIRNDLG | TASSLQS | LQHNSYPLT |
| | | | SEQ ID NO:300 | SEQ ID NO:8312 | SEQ ID NO:16324 |
| iPS:392734 | | NA | AGGGCCAGTCAGAGTGTTAGCAGCAACTTAGCC | GGTGCATCCACCAGGGCCAGT | CAGCAGTATAATAACTGGCCTCTGACG |
| | 21-225_17D8 | | SEQ ID NO:301 | SEQ ID NO:8313 | SEQ ID NO:16325 |
| | | AA | RASQSVSSNLA | GASTRAS | QQYNNWPLT |
| | | | SEQ ID NO:302 | SEQ ID NO:8314 | SEQ ID NO:16326 |
| iPS:392736 | | NA | CGGGCAAGTCAGAATATTAACAACTATTTAAAT | ACTGCATCCAGTTTGCAAAGT | CAACAGACTTACACTACCCCCACGTGGACG |
| | 21-225_17B12 | | SEQ ID NO:303 | SEQ ID NO:8315 | SEQ ID NO:16327 |
| | | AA | RASQNINNYLN | TASSLQS | QQTYTTPTWT |
| | | | SEQ ID NO:304 | SEQ ID NO:8316 | SEQ ID NO:16328 |
| iPS:392738 | | NA | CGGGCAAGTCAGAGCATTATCAGCTATTTAAAT | ACTGCATCCAGTTTGCAAACT | CAACAGACTTACAGTCCCCCGCTCACT |
| | 21-225_18G4 | | SEQ ID NO:305 | SEQ ID NO:8317 | SEQ ID NO:16329 |
| | | AA | RASQSIISYLN | TASSLQT | QQTYSPPLT |
| | | | SEQ ID NO:306 | SEQ ID NO:8318 | SEQ ID NO:16330 |
| iPS:392740 | | NA | CGGGCAAGTCAGGGCATTAGAAATGATTTAGGC | ACTGCATCCAATTTGCAAAGT | CTACAACATAATAATTACCCGTGGACG |
| | 21-225_18H12 | | SEQ ID NO:307 | SEQ ID NO:8319 | SEQ ID NO:16331 |
| | | AA | RASQGIRNDLG | TASNLQS | LQHNNYPWT |

FIGURE 49

| | | | | SEQ ID NO:308 | SEQ ID NO:8320 | SEQ ID NO:16332 |
|---|---|---|---|---|---|---|
| iPS:392742 | | NA | | CGGGCAAGTCAGGACATTAG AAATGATTTAGGC | GCTGCATCCAGTTTGCAA AGT | CTACAGCATTATAATTACCC TCGGGCG |
| | 21-225_20B2 | | | SEQ ID NO:309 | SEQ ID NO:8321 | SEQ ID NO:16333 |
| | | AA | | RASQDIRNDLG | AASSLQS | LQHYNYPRA |
| | | | | SEQ ID NO:310 | SEQ ID NO:8322 | SEQ ID NO:16334 |
| iPS:392744 | | NA | | CGGGCAAGTCAGGGCATTAG AAATGATTTAGGC | GCTGCATCCAGTTTGCAA AGT | CTACAGCATAATAGTTACCC GTTCACT |
| | 21-225_20D5 | | | SEQ ID NO:311 | SEQ ID NO:8323 | SEQ ID NO:16335 |
| | | AA | | RASQGIRNDLG | AASSLQS | LQHNSYPFT |
| | | | | SEQ ID NO:312 | SEQ ID NO:8324 | SEQ ID NO:16336 |
| iPS:392746 | | NA | | CGGACGAGTCAGGGCATTAA CAATTATTTAGTC | GCTGCATCCAGTTTGCAA AGT | CAACAGTATTATAGTTACCC ATTCACT |
| | 21-225_20H7 | | | SEQ ID NO:313 | SEQ ID NO:8325 | SEQ ID NO:16337 |
| | | AA | | RTSQGINNYLV | AASSLQS | QQYYSYPFT |
| | | | | SEQ ID NO:314 | SEQ ID NO:8326 | SEQ ID NO:16338 |
| iPS:392748 | | NA | | CGGGCGAGTCAGGGCATTAA TAATTATTTAGTC | GCTGCATCCAGTTTGCTG AGT | CAACAGTATAATAGTTACCC GATCACC |
| | 21-225_20A8 | | | SEQ ID NO:315 | SEQ ID NO:8327 | SEQ ID NO:16339 |
| | | AA | | RASQGINNYLV | AASSLLS | QQYNSYPIT |
| | | | | SEQ ID NO:316 | SEQ ID NO:8328 | SEQ ID NO:16340 |
| iPS:392750 | | NA | | CGGGCAAGTCAGGGCATTAG AAATGATTTAGGC | GCTGCATCCAGTTTGCAA AGT | CTACAGCATAATAGATACCC GCTCACT |
| | 21-225_20A10 | | | SEQ ID NO:317 | SEQ ID NO:8329 | SEQ ID NO:16341 |
| | | AA | | RASQGIRNDLG | AASSLQS | LQHNRYPLT |
| | | | | SEQ ID NO:318 | SEQ ID NO:8330 | SEQ ID NO:16342 |
| iPS:392754 | | NA | | CGGGCAAGTCAGAGCATTAC TGGTTATTCAAAT | GCTACATACAGTTTGGA AAGT | CAACAGAGTTACAGTACCTC GATCACC |
| | 21-225_21D3 | | | SEQ ID NO:319 | SEQ ID NO:8331 | SEQ ID NO:16343 |
| | | AA | | RASQSITGYSN | ATYSLES | QQSYSTSIT |

FIGURE 49

| | | | SEQ ID NO:320 | SEQ ID NO:8332 | SEQ ID NO:16344 |
|---|---|---|---|---|---|
| iPS:392758 | | NA | CGGGCAAGTCAGGGCATTAGAAATGATTTAGGC | ACTGCATCCAGTTTGCAAAGT | CTACAACATAATAATTACCCGTGGACG |
| | 21-225_21G11 | | SEQ ID NO:321 | SEQ ID NO:8333 | SEQ ID NO:16345 |
| | | AA | RASQGIRNDLG | TASSLQS | LQHNNYPWT |
| | | | SEQ ID NO:322 | SEQ ID NO:8334 | SEQ ID NO:16346 |
| iPS:392760 | | NA | CGGGCAAGTCAGAGCATTAGTAATTATTTAAAT | GCTGCTTCCAGTTTGCAAAGT | CAACAGAGTTTCAGAACCCCCTTTTTCACT |
| | 21-225_22G3 | | SEQ ID NO:323 | SEQ ID NO:8335 | SEQ ID NO:16347 |
| | | AA | RASQSISNYLN | AASSLQS | QQSFRTPFFT |
| | | | SEQ ID NO:324 | SEQ ID NO:8336 | SEQ ID NO:16348 |
| iPS:392762 | | NA | CGGGCAAGTCAGAACATTAGCAGCTATTTAAAT | GCTGCATCCAGTTTGCAAAAT | CAACAGAGTTACAGAACCCCCTTATTCACT |
| | 21-225_22G5 | | SEQ ID NO:325 | SEQ ID NO:8337 | SEQ ID NO:16349 |
| | | AA | RASQNISSYLN | AASSLQN | QQSYRTPLFT |
| | | | SEQ ID NO:326 | SEQ ID NO:8338 | SEQ ID NO:16350 |
| iPS:392764 | | NA | CGGGCAAGTCAGAGCATTTTCAGCTATTTAAAT | GCTGCATCCAGTTTACAAAGT | CAACAGAGTTTCAGAACCCCCTTATTCACT |
| | 21-225_22G10 | | SEQ ID NO:327 | SEQ ID NO:8339 | SEQ ID NO:16351 |
| | | AA | RASQSIFSYLN | AASSLQS | QQSFRTPLFT |
| | | | SEQ ID NO:328 | SEQ ID NO:8340 | SEQ ID NO:16352 |
| iPS:392766 | | NA | CGGGCAAGTCAGAGCATTAGCAGGTATTTAAAT | TCTACATCCAGTTTGCAAAGT | CAACAGAGTTACAGTACCCCCACGTGGACG |
| | 21-225_23H4 | | SEQ ID NO:329 | SEQ ID NO:8341 | SEQ ID NO:16353 |
| | | AA | RASQSISRYLN | STSSLQS | QQSYSTPTWT |
| | | | SEQ ID NO:330 | SEQ ID NO:8342 | SEQ ID NO:16354 |
| iPS:392768 | | NA | AGGGCCAGTCAGAGTGTTAGCAGCAACTTAGCC | GGTGCATCCACCAGGGCCAGT | CAGCAGTATAATAACTGTCCTCTGACG |
| | 21-225_20B8 | | SEQ ID NO:331 | SEQ ID NO:8343 | SEQ ID NO:16355 |
| | | AA | RASQSVSSNLA | GASTRAS | QQYNNCPLT |

FIGURE 49

| | | | SEQ ID NO:332 | SEQ ID NO:8344 | SEQ ID NO:16356 |
|---|---|---|---|---|---|
| iPS:392770 | | NA | CGGGCAAGTCACCATATTAG CAACTATTTAAAT | ACTGCATCCAGTTTGCAA AGT | CAACAGAGTTACACTACCCC CACGTGGACG |
| | 21-225_20C10 | | SEQ ID NO:333 | SEQ ID NO:8345 | SEQ ID NO:16357 |
| | | AA | RASHHISNYLN | TASSLQS | QQSYTTPTWT |
| | | | SEQ ID NO:334 | SEQ ID NO:8346 | SEQ ID NO:16358 |
| iPS:392772 | | NA | CGGGCAAGTCAGGGCATTAG AAATGATTTAGGC | GCTGCATCCAGTTTACAA AGT | CTACAGCATAATAGTTACCC GTTCACT |
| | 21-225_20E12 | | SEQ ID NO:335 | SEQ ID NO:8347 | SEQ ID NO:16359 |
| | | AA | RASQGIRNDLG | AASSLQS | LQHNSYPFT |
| | | | SEQ ID NO:336 | SEQ ID NO:8348 | SEQ ID NO:16360 |
| iPS:392774 | | NA | CGGGCAAGTCAGGGCATTAG AAATGATTTAGGC | GCTGCATCCAGTTTGCAA AGT | CTACAGCATAGTAGTTACCC CCTCACT |
| | 21-225_21F3 | | SEQ ID NO:337 | SEQ ID NO:8349 | SEQ ID NO:16361 |
| | | AA | RASQGIRNDLG | AASSLQS | LQHSSYPLT |
| | | | SEQ ID NO:338 | SEQ ID NO:8350 | SEQ ID NO:16362 |
| iPS:392776 | | NA | CGGGCGAGTCAAGGCATTAG CAAATATTTAGCC | GCTGCATCCAGTTTGCAA AGT | CAACAGTATAATAGTTACCC GTTCAGG |
| | 21-225_21A12 | | SEQ ID NO:339 | SEQ ID NO:8351 | SEQ ID NO:16363 |
| | | AA | RASQGISKYLA | AASSLQS | QQYNSYPFR |
| | | | SEQ ID NO:340 | SEQ ID NO:8352 | SEQ ID NO:16364 |
| iPS:392778 | | NA | CGGGCAAGTCAGGACATTAG AAATAATTTAGGC | CCTGCATCCAGTTTGCAA ACT | CTACAGGATAATAGTTACCC ATTCACT |
| | 21-225_22H3 | | SEQ ID NO:341 | SEQ ID NO:8353 | SEQ ID NO:16365 |
| | | AA | RASQDIRNNLG | PASSLQT | LQDNSYPFT |
| | | | SEQ ID NO:342 | SEQ ID NO:8354 | SEQ ID NO:16366 |
| iPS:392780 | | NA | CGGGCAAGTCAGGGCATTAG AAATGATTTAGGC | ACTGCATCCAGTTTGCAA AGT | CTACAGCATAATACTTACCC GCTCACT |
| | 21-225_22B7 | | SEQ ID NO:343 | SEQ ID NO:8355 | SEQ ID NO:16367 |
| | | AA | RASQGIRNDLG | TASSLQS | LQHNTYPLT |

FIGURE 49

| | | | SEQ ID NO:344 | SEQ ID NO:8356 | SEQ ID NO:16368 |
|---|---|---|---|---|---|
| iPS:392782 | | NA | CGGGCGAGTCAGGACATTAGCAATTATTTAGCC | GGTGCATCCAGTTTGCGGAGT | CAACAGTATCATAGTTACCCATTCACT |
| | 21-225_22B12 | | SEQ ID NO:345 | SEQ ID NO:8357 | SEQ ID NO:16369 |
| | | AA | RASQDISNYLA | GASSLRS | QQYHSYPFT |
| | | | SEQ ID NO:346 | SEQ ID NO:8358 | SEQ ID NO:16370 |
| iPS:392784 | | NA | CGGGCGAGTCAGGGCATTGGCATTTATTTAGCC | GCTGCATCCAGTTTGCAAAGT | CAACAGTATAATAGTTACCCATTCACT |
| | 21-225_23C7 | | SEQ ID NO:347 | SEQ ID NO:8359 | SEQ ID NO:16371 |
| | | AA | RASQGIGIYLA | AASSLQS | QQYNSYPFT |
| | | | SEQ ID NO:348 | SEQ ID NO:8360 | SEQ ID NO:16372 |
| iPS:392786 | | NA | AAGTCCAGCCAGAGTGTTTTATACACCTCCAACAATAACAACTACTTAACT | TGGGCATCTACCCGGGAATCC | CAGCAATTTTATAGTACTCCTCCGACG |
| | 21-225_24E1 | | SEQ ID NO:349 | SEQ ID NO:8361 | SEQ ID NO:16373 |
| | | AA | KSSQSVLYTSNNNYLT | WASTRES | QQFYSTPPT |
| | | | SEQ ID NO:350 | SEQ ID NO:8362 | SEQ ID NO:16374 |
| iPS:392788 | | NA | CGGGCAAGTCAGGGCATTAGAAATGATTTAGGC | GCTGCATCCAGTTTGCAAAGT | CTACAAGATAATAGTTACCCGTTCACT |
| | 21-225_20C8 | | SEQ ID NO:351 | SEQ ID NO:8363 | SEQ ID NO:16375 |
| | | AA | RASQGIRNDLG | AASSLQS | LQDNSYPFT |
| | | | SEQ ID NO:352 | SEQ ID NO:8364 | SEQ ID NO:16376 |
| iPS:392790 | | NA | CGGGCAAGTCAGGGCATTAGAAATGATTTAGGC | GTTGCATACAGTTTGCAAAGT | ATACAGCAAAATAGTTACCCGTGGACG |
| | 21-225_20D10 | | SEQ ID NO:353 | SEQ ID NO:8365 | SEQ ID NO:16377 |
| | | AA | RASQGIRNDLG | VAYSLQS | IQQNSYPWT |
| | | | SEQ ID NO:354 | SEQ ID NO:8366 | SEQ ID NO:16378 |
| iPS:392792 | | NA | CGGGCGAGTCAGGGCATTAGCAATTATTTAGCC | ACTGCATCCACTTTGCAATCA | CAAAAGTATAACAGTGCCCCTCCGATCACC |
| | 21-225_20G12 | | SEQ ID NO:355 | SEQ ID NO:8367 | SEQ ID NO:16379 |

FIGURE 49

| | | | | | |
|---|---|---|---|---|---|
| | | AA | RASQGISNYLA | TASTLQS | QKYNSAPPIT |
| | | | SEQ ID NO:356 | SEQ ID NO:8368 | SEQ ID NO:16380 |
| iPS:392794 | | NA | CGGGCAAGTCAGGGCATTAGAAATGATTTAGGC | GCTGCGTCCAGTGTGCAAACT | CTACAGCATAATAGTTATCCGCTCACT |
| | 21-225_21H3 | | SEQ ID NO:357 | SEQ ID NO:8369 | SEQ ID NO:16381 |
| | | AA | RASQGIRNDLG | AASSVQT | LQHNSYPLT |
| | | | SEQ ID NO:358 | SEQ ID NO:8370 | SEQ ID NO:16382 |
| iPS:392796 | | NA | CGGGCAAGTCAGGGCATTAGAAATGATTTAGGC | GCTGCATCCAGTTTGCAAAGT | CTTCAGCATAATAGTTACCCGTGGACG |
| | 21-225_22A4 | | SEQ ID NO:359 | SEQ ID NO:8371 | SEQ ID NO:16383 |
| | | AA | RASQGIRNDLG | AASSLQS | LQHNSYPWT |
| | | | SEQ ID NO:360 | SEQ ID NO:8372 | SEQ ID NO:16384 |
| iPS:392798 | | NA | CGGGCAAGTCAGAACATTATCAGCTATTTAAAT | ATTGCATCCAGTTTGCAAAGT | CAACAGACTTACAGTACCCCGCTCACT |
| | 21-225_22C7 | | SEQ ID NO:361 | SEQ ID NO:8373 | SEQ ID NO:16385 |
| | | AA | RASQNIISYLN | IASSLQS | QQTYSTPLT |
| | | | SEQ ID NO:362 | SEQ ID NO:8374 | SEQ ID NO:16386 |
| iPS:392800 | | NA | CGGGCAAGTCAGGGCATTAGAAATGATTTAGGC | GCTGCATCCAGTTTGCAGAGT | CTACAGCATAGTACTTACCCTCTCACT |
| | 21-225_22D12 | | SEQ ID NO:363 | SEQ ID NO:8375 | SEQ ID NO:16387 |
| | | AA | RASQGIRNDLG | AASSLQS | LQHSTYPLT |
| | | | SEQ ID NO:364 | SEQ ID NO:8376 | SEQ ID NO:16388 |
| iPS:392802 | | NA | CGGGCGAGTCAGGGCATTAGCAATTATTTAGCC | GCTGCATCCAGTTTGCAAAGT | CAACAGTTTTATAGTTACCCATTCACT |
| | 21-225_23E7 | | SEQ ID NO:365 | SEQ ID NO:8377 | SEQ ID NO:16389 |
| | | AA | RASQGISNYLA | AASSLQS | QQFYSYPFT |
| | | | SEQ ID NO:366 | SEQ ID NO:8378 | SEQ ID NO:16390 |
| iPS:392806 | | NA | AGGGCCAGTCAGAGTGTTAGCAGCAACTTAGCC | TTTGCATCCATCAGGGCCACT | CAGCAGTATAATAACTGGCCCATGTGCAGT |
| | 21-225_24H3 | | SEQ ID NO:367 | SEQ ID NO:8379 | SEQ ID NO:16391 |

FIGURE 49

| | | | | | |
|---|---|---|---|---|---|
| | | AA | RASQSVSSNLA | FASIRAT | QQYNNWPMCS |
| | | | SEQ ID NO:368 | SEQ ID NO:8380 | SEQ ID NO:16392 |
| iPS:392808 | | NA | CGGGCAAGTCAGAGCATTAGCAGGTATTTAAAT | GCTGCTTCCAGTTTGCAAAGT | CAACAGAGTTACAATACCCCCACGTGGACG |
| | 21-225_20F8 | | SEQ ID NO:369 | SEQ ID NO:8381 | SEQ ID NO:16393 |
| | | AA | RASQSISRYLN | AASSLQS | QQSYNTPTWT |
| | | | SEQ ID NO:370 | SEQ ID NO:8382 | SEQ ID NO:16394 |
| iPS:392810 | | NA | CGGGCAAGTCAGGGCATTAGAAATGATTTAGAC | GCTGCATCCAGTTTGCAAAGT | CTACAGCATAATAGTTACCCGCTCACT |
| | 21-225_20H12 | | SEQ ID NO:371 | SEQ ID NO:8383 | SEQ ID NO:16395 |
| | | AA | RASQGIRNDLD | AASSLQS | LQHNSYPLT |
| | | | SEQ ID NO:372 | SEQ ID NO:8384 | SEQ ID NO:16396 |
| iPS:392812 | | NA | CGGGCAAGTCAGAACATTGGTAGTTATTTAAAT | GCTGCATCCAGTTTGCAAAGT | CAACAGAGTTACAGAACCCCCTTTTTCACT |
| | 21-225_21F4 | | SEQ ID NO:373 | SEQ ID NO:8385 | SEQ ID NO:16397 |
| | | AA | RASQNIGSYLN | AASSLQS | QQSYRTPFFT |
| | | | SEQ ID NO:374 | SEQ ID NO:8386 | SEQ ID NO:16398 |
| iPS:392814 | | NA | AAGTCTAGTCAGAGCCTCCTGCATAGTGGTGGAAAGACCTATTTATAT | GAAGTTTCCAACCGGTTCTCT | ATGCAAACTTTACACCTTCCGTGGACG |
| | 21-225_22A1 | | SEQ ID NO:375 | SEQ ID NO:8387 | SEQ ID NO:16399 |
| | | AA | KSSQSLLHSGGKTYLY | EVSNRFS | MQTLHLPWT |
| | | | SEQ ID NO:376 | SEQ ID NO:8388 | SEQ ID NO:16400 |
| iPS:392816 | | NA | CGGGCAAGTCAGAACATTAGTAGTTATTTAAAT | GCTGCATCCAGTTTGCAAAGT | CAACAGAGTTACAATACCCCCTTATTCACT |
| | 21-225_22E4 | | SEQ ID NO:377 | SEQ ID NO:8389 | SEQ ID NO:16401 |
| | | AA | RASQNISSYLN | AASSLQS | QQSYNTPLFT |
| | | | SEQ ID NO:378 | SEQ ID NO:8390 | SEQ ID NO:16402 |
| iPS:392818 | | NA | CGGACAAGTCAGAACAGTAACAGTTATTTAAAT | GCTGCATACAGTTTGGAAAGT | CAACAGACTTACGGTACCTCGATCACC |

FIGURE 49

| | 21-225_22D8 | | SEQ ID NO:379 | SEQ ID NO:8391 | SEQ ID NO:16403 |
|---|---|---|---|---|---|
| | | AA | RTSQNSNSYLN | AAYSLES | QQTYGTSIT |
| | | | SEQ ID NO:380 | SEQ ID NO:8392 | SEQ ID NO:16404 |
| iPS:392820 | | NA | CGGGCAAGTCAGGGCATCA GAAATGATTTAGGC | GCTGCATCCAGTTTACAA AGT | CTACAGCATAGTAGTTACCC TCTCACT |
| | 21-225_23D1 | | SEQ ID NO:381 | SEQ ID NO:8393 | SEQ ID NO:16405 |
| | | AA | RASQGIRNDLG | AASSLQS | LQHSSYPLT |
| | | | SEQ ID NO:382 | SEQ ID NO:8394 | SEQ ID NO:16406 |
| iPS:392822 | | NA | CGGGCAAGTCAGGACATTAG AAATGATTTAGGC | GGTGCATCCAGTGTGCA AAGT | CTACAGCATAATAGTTACCC GCTCACT |
| | 21-225_23C8 | | SEQ ID NO:383 | SEQ ID NO:8395 | SEQ ID NO:16407 |
| | | AA | RASQDIRNDLG | GASSVQS | LQHNSYPLT |
| | | | SEQ ID NO:384 | SEQ ID NO:8396 | SEQ ID NO:16408 |
| iPS:392824 | | NA | CGGGCAAGTCAGGGCATTAG AAATGATTTAGGC | GCTGCATCCAGTTTGCAA AGT | CTACAGCATAGTAATTACCC GCTCACT |
| | 21-225_24E5 | | SEQ ID NO:385 | SEQ ID NO:8397 | SEQ ID NO:16409 |
| | | AA | RASQGIRNDLG | AASSLQS | LQHSNYPLT |
| | | | SEQ ID NO:386 | SEQ ID NO:8398 | SEQ ID NO:16410 |
| iPS:392826 | | NA | CGGGCGAGTCAGGGCATTAG CAATTATTTAGCC | GTTGCATCCAGTTTGCAA AGT | CAACAGTATAATACTTATCC ATTCACT |
| | 21-225_20B9 | | SEQ ID NO:387 | SEQ ID NO:8399 | SEQ ID NO:16411 |
| | | AA | RASQGISNYLA | VASSLQS | QQYNTYPFT |
| | | | SEQ ID NO:388 | SEQ ID NO:8400 | SEQ ID NO:16412 |
| iPS:392830 | | NA | CGGGCAAGTCAGACCATTAG CAGCCATTTAAAT | GCTGCATCCAGTTTGCAA AGT | CAACAGAGTTACAATATCTC ATTCACT |
| | 21-225_21A5 | | SEQ ID NO:389 | SEQ ID NO:8401 | SEQ ID NO:16413 |
| | | AA | RASQTISSHLN | AASSLQS | QQSYNISFT |
| | | | SEQ ID NO:390 | SEQ ID NO:8402 | SEQ ID NO:16414 |
| iPS:392832 | | NA | CGGGCAAGTCAGGGCATTAG AAATGATTTAGGC | GCTGCATCCAGTTTACAA AGT | CTACAGCATAATAGTTACCC GTGGACG |

FIGURE 49

| | | | | | |
|---|---|---|---|---|---|
| | **21-225_21H8** | | SEQ ID NO:391 | SEQ ID NO:8403 | SEQ ID NO:16415 |
| | | AA | RASQGIRNDLG | AASSLQS | LQHNSYPWT |
| | | | SEQ ID NO:392 | SEQ ID NO:8404 | SEQ ID NO:16416 |
| iPS:392834 | | NA | CGGACAAGTCAGGGCATTAG AAATGATTTAGGC | GCTGCATCCAGTTTGCAA AGT | CTACAGCATAGTACTTACCC GCTCACT |
| | **21-225_22C1** | | SEQ ID NO:393 | SEQ ID NO:8405 | SEQ ID NO:16417 |
| | | AA | RTSQGIRNDLG | AASSLQS | LQHSTYPLT |
| | | | SEQ ID NO:394 | SEQ ID NO:8406 | SEQ ID NO:16418 |
| iPS:392836 | | NA | CGGGCAAGTCAGGTCATTAG AGATGATTTAGGC | GCTGCATCCAGTTTGCAA AGT | CTACACCACTATAGTTATCC TCGGACG |
| | **21-225_22F4** | | SEQ ID NO:395 | SEQ ID NO:8407 | SEQ ID NO:16419 |
| | | AA | RASQVIRDDLG | AASSLQS | LHHYSYPRT |
| | | | SEQ ID NO:396 | SEQ ID NO:8408 | SEQ ID NO:16420 |
| iPS:392838 | | NA | CGGACAAGTCAGGGCATTAG AAATGATTTAGGC | GCTGCATCCAGTTTGCAA AGT | CTACAGCATAATAGTTACCC GCTCACT |
| | **21-225_22G8** | | SEQ ID NO:397 | SEQ ID NO:8409 | SEQ ID NO:16421 |
| | | AA | RTSQGIRNDLG | AASSLQS | LQHNSYPLT |
| | | | SEQ ID NO:398 | SEQ ID NO:8410 | SEQ ID NO:16422 |
| iPS:392840 | | NA | CGGGCGAGTCAGGGCATTAG CAATTATTTAGCC | GCTGCATCCAGTTTACAG AGT | CAACAGTATAATAGTTACCC ATTCACT |
| | **21-225_23G1** | | SEQ ID NO:399 | SEQ ID NO:8411 | SEQ ID NO:16423 |
| | | AA | RASQGISNYLA | AASSLQS | QQYNSYPFT |
| | | | SEQ ID NO:400 | SEQ ID NO:8412 | SEQ ID NO:16424 |
| iPS:392842 | | NA | CGGGCGAGTCAGGGCATTAG AAATTATTTAGCC | GCTGCATCCAGTTTGCAA AGT | CAACAGTATAATAGTTACCC TTTCACT |
| | **21-225_23G8** | | SEQ ID NO:401 | SEQ ID NO:8413 | SEQ ID NO:16425 |
| | | AA | RASQGIRNYLA | AASSLQS | QQYNSYPFT |
| | | | SEQ ID NO:402 | SEQ ID NO:8414 | SEQ ID NO:16426 |
| iPS:392844 | | NA | CGGGCAAGTCAGGGCATTAG AAATGATTTAGGC | CCTGCATCCAGTTTGCAA AGT | CTACAGCATAATAGTTACCC GTGGACG |

FIGURE 49

| | 21-225_23E11 | | SEQ ID NO:403 | SEQ ID NO:8415 | SEQ ID NO:16427 |
|---|---|---|---|---|---|
| | | AA | RASQGIRNDLG | PASSLQS | LQHNSYPWT |
| | | | SEQ ID NO:404 | SEQ ID NO:8416 | SEQ ID NO:16428 |
| iPS:392846 | | NA | CGGGCAAGTCAGGACATTAG AAATGATTTAGGC | GCTGCATCCAGTTTGCAC AGT | CTACAGCATTATAGTTACCC TCGGACG |
| | 21-225_24B6 | | SEQ ID NO:405 | SEQ ID NO:8417 | SEQ ID NO:16429 |
| | | AA | RASQDIRNDLG | AASSLHS | LQHYSYPRT |
| | | | SEQ ID NO:406 | SEQ ID NO:8418 | SEQ ID NO:16430 |
| iPS:392848 | | NA | CGGGCGAGTCAGGGCATTAG CAATTATTTAGCC | GCTGCATCCAGTTTGCAA AGT | CAACAGTATCATAGTTACCC GTGGACG |
| | 21-225_20F9 | | SEQ ID NO:407 | SEQ ID NO:8419 | SEQ ID NO:16431 |
| | | AA | RASQGISNYLA | AASSLQS | QQYHSYPWT |
| | | | SEQ ID NO:408 | SEQ ID NO:8420 | SEQ ID NO:16432 |
| iPS:392850 | | NA | CGGGCAAGTCAGGGCATTAA AAATAATTTAGGC | GCTGCATCCAGTTTGCAA AGT | CTACAGCATAATAGTTACCC GCTCACT |
| | 21-225_20H10 | | SEQ ID NO:409 | SEQ ID NO:8421 | SEQ ID NO:16433 |
| | | AA | RASQGIKNNLG | AASSLQS | LQHNSYPLT |
| | | | SEQ ID NO:410 | SEQ ID NO:8422 | SEQ ID NO:16434 |
| iPS:392852 | | NA | CGGGCAAGTCAGAGTATTAG TAGTTATTTAAAT | GCTGCATCCAGTTTGCAA AGT | CAACAGAGTTACAGAACCCC CTTTTTCACT |
| | 21-225_21A2 | | SEQ ID NO:411 | SEQ ID NO:8423 | SEQ ID NO:16435 |
| | | AA | RASQSISSYLN | AASSLQS | QQSYRTPFFT |
| | | | SEQ ID NO:412 | SEQ ID NO:8424 | SEQ ID NO:16436 |
| iPS:392854 | | NA | CGGGCAAGTCAGGGCATTAG AAATGATTTAGGC | GCTACATCCAGTTTGCAA AGT | CTACAGCATAATAGTTACCC CCTCACT |
| | 21-225_21E5 | | SEQ ID NO:413 | SEQ ID NO:8425 | SEQ ID NO:16437 |
| | | AA | RASQGIRNDLG | ATSSLQS | LQHNSYPLT |
| | | | SEQ ID NO:414 | SEQ ID NO:8426 | SEQ ID NO:16438 |
| iPS:392856 | | NA | CGGGCGAGTCAGGACATTAG CAATTATTTAGCC | GCTGCATCCAGTTTGCAA AGT | CAACAGTATAATAGTTTCCC GCTCACT |

FIGURE 49

| | 21-225_22A2 | | SEQ ID NO:415 | SEQ ID NO:8427 | SEQ ID NO:16439 |
|---|---|---|---|---|---|
| | | AA | RASQDISNYLA | AASSLQS | QQYNSFPLT |
| | | | SEQ ID NO:416 | SEQ ID NO:8428 | SEQ ID NO:16440 |
| iPS:392858 | | NA | CGGGCAAGTCAGGGCATTAG AAATGATTTAGGC | GCTGCATCCAGTGTGCA AAGT | CTACAGCATAATAGTTACCC GCTCACT |
| | 21-225_22H4 | | SEQ ID NO:417 | SEQ ID NO:8429 | SEQ ID NO:16441 |
| | | AA | RASQGIRNDLG | AASSVQS | LQHNSYPLT |
| | | | SEQ ID NO:418 | SEQ ID NO:8430 | SEQ ID NO:16442 |
| iPS:392860 | | NA | AAGTCTAGTCAGAGCCTCCT GCATAGTGATGGAAAGACCT TTTTGTAT | GAAGTTTCCAACCGGTTC TCT | ATGCAAAGTATACAGCTTCC GCTCTCA |
| | 21-225_22H8 | | SEQ ID NO:419 | SEQ ID NO:8431 | SEQ ID NO:16443 |
| | | AA | KSSQSLLHSDGKTFLY | EVSNRFS | MQSIQLPLS |
| | | | SEQ ID NO:420 | SEQ ID NO:8432 | SEQ ID NO:16444 |
| iPS:392864 | | NA | AGGGCCAGTCAGAATGTTTA CAGCAGCTACTTAGCC | GGTGCATCCAGCAGGGC CAGT | CAGCAGTATGGTAGCTCACC TCGGACG |
| | 21-225_23B9 | | SEQ ID NO:421 | SEQ ID NO:8433 | SEQ ID NO:16445 |
| | | AA | RASQNVYSSYLA | GASSRAS | QQYGSSPRT |
| | | | SEQ ID NO:422 | SEQ ID NO:8434 | SEQ ID NO:16446 |
| iPS:392866 | | NA | CGGGCAAGTCAGGGCATTAG AAATGATTTAGAC | GCTGCATCCAGTTTGCAA AGT | CTACAGCATAATCGTTACCC GCTCACT |
| | 21-225_23H11 | | SEQ ID NO:423 | SEQ ID NO:8435 | SEQ ID NO:16447 |
| | | AA | RASQGIRNDLD | AASSLQS | LQHNRYPLT |
| | | | SEQ ID NO:424 | SEQ ID NO:8436 | SEQ ID NO:16448 |
| iPS:392868 | | NA | CAGGCGAGTCAGGACATTAA CAACTATTTAAAT | GATGCATCCGATTTGGA AACA | CAACAGTATGAAAATCTCCC GATCACC |
| | 21-225_24D6 | | SEQ ID NO:425 | SEQ ID NO:8437 | SEQ ID NO:16449 |
| | | AA | QASQDINNYLN | DASDLET | QQYENLPIT |
| | | | SEQ ID NO:426 | SEQ ID NO:8438 | SEQ ID NO:16450 |

FIGURE 49

| iPS:392870 | | NA | CGGGCAAGTCAGGGCATTAG AAATGATTTAGGC | GCTGCATCCAGTTTGCAA AGT | CTACAGCATAGTACTTACCC TCTCACT |
|---|---|---|---|---|---|
| | 21-225_20G9 | | SEQ ID NO:427 | SEQ ID NO:8439 | SEQ ID NO:16451 |
| | | AA | RASQGIRNDLG | AASSLQS | LQHSTYPLT |
| | | | SEQ ID NO:428 | SEQ ID NO:8440 | SEQ ID NO:16452 |
| iPS:392872 | | NA | CGGGCAAGTCAGGGCATTGG AAATGATTTAGGC | GCTGCATCCAGTTTGCAT AGT | CTACAGCATTATAGTTACCC TCGGACG |
| | 21-225_20B11 | | SEQ ID NO:429 | SEQ ID NO:8441 | SEQ ID NO:16453 |
| | | AA | RASQGIGNDLG | AASSLHS | LQHYSYPRT |
| | | | SEQ ID NO:430 | SEQ ID NO:8442 | SEQ ID NO:16454 |
| iPS:392874 | | NA | CGGGCAAGTCAGAGCATTAG CGACTATTTAAAT | GATACATCCAGTTTGCA AAGT | CAACAGACTTACAATATTCT TCCGGAGCGCAGT |
| | 21-225_21D2 | | SEQ ID NO:431 | SEQ ID NO:8443 | SEQ ID NO:16455 |
| | | AA | RASQSISDYLN | DTSSLQS | QQTYNILPERS |
| | | | SEQ ID NO:432 | SEQ ID NO:8444 | SEQ ID NO:16456 |
| iPS:392876 | | NA | CGGGCAAGTCAGGACATTAG AAATGATTTAGGC | GCTGCATCCAATTTTCAA AGT | CTACAGCATAATAATTACCC GTGGACG |
| | 21-225_21F7 | | SEQ ID NO:433 | SEQ ID NO:8445 | SEQ ID NO:16457 |
| | | AA | RASQDIRNDLG | AASNFQS | LQHNNYPWT |
| | | | SEQ ID NO:434 | SEQ ID NO:8446 | SEQ ID NO:16458 |
| iPS:392878 | | NA | CGGGCAAGTCAGAACATTAG CAGCTATTTAAAT | GCTGCATCCGTTTTGCAA CAT | CAACAGAGTTACAGAACCCC CTTATTCACT |
| | 21-225_22C5 | | SEQ ID NO:435 | SEQ ID NO:8447 | SEQ ID NO:16459 |
| | | AA | RASQNISSYLN | AASVLQH | QQSYRTPLFT |
| | | | SEQ ID NO:436 | SEQ ID NO:8448 | SEQ ID NO:16460 |
| iPS:392880 | | NA | CGGGCAAGTCAGGGCATTAG AAATGATTTAGGC | GCTGCATCCAGTTTACAA AGT | CTACAGCATAATAGTTACCC GCTCACT |
| | 21-225_22F9 | | SEQ ID NO:437 | SEQ ID NO:8449 | SEQ ID NO:16461 |
| | | AA | RASQGIRNDLG | AASSLQS | LQHNSYPLT |
| | | | SEQ ID NO:438 | SEQ ID NO:8450 | SEQ ID NO:16462 |

FIGURE 49

| iPS:392882 | | NA | CGGGCAAGTCAGGGCATTAG AAATGATTTAGGC | GCTGCATCCAGTGTGCA AAGT | CTACAGCATAATAGTTACCC GCTCACT |
|---|---|---|---|---|---|
| | 21-225_23A3 | | SEQ ID NO:439 | SEQ ID NO:8451 | SEQ ID NO:16463 |
| | | AA | RASQGIRNDLG | AASSVQS | LQHNSYPLT |
| | | | SEQ ID NO:440 | SEQ ID NO:8452 | SEQ ID NO:16464 |
| iPS:392884 | | NA | CGGGCAAGTCAGGGCATTAG AAATGATTTAGGC | GCTGCATCCAGTTTGCAA AGT | CTGCAACATTATAGTTACCC TCGGACG |
| | 21-225_23A10 | | SEQ ID NO:441 | SEQ ID NO:8453 | SEQ ID NO:16465 |
| | | AA | RASQGIRNDLG | AASSLQS | LQHYSYPRT |
| | | | SEQ ID NO:442 | SEQ ID NO:8454 | SEQ ID NO:16466 |
| iPS:392886 | | NA | AAGTCCAGCCAGAGTGTTTT ATACAGCTCCAACAATAACA ATTATTTAGCT | TGGACATCTACCCGGGA ATCC | CAGCAGTATTATGATACTCC TCCGACG |
| | 21-225_23A12 | | SEQ ID NO:443 | SEQ ID NO:8455 | SEQ ID NO:16467 |
| | | AA | KSSQSVLYSSNNNNYLA | WTSTRES | QQYYDTPPT |
| | | | SEQ ID NO:444 | SEQ ID NO:8456 | SEQ ID NO:16468 |
| iPS:392888 | | NA | AAGTCTAGTCAGAGCCTCCT ACATAGTGAAGGAAAGACC TATTTGTAT | GAAATTTCCAACCGGTTC TCT | ATGCAAAGTACACAGTTTCC GCTCACT |
| | 21-225_25A2 | | SEQ ID NO:445 | SEQ ID NO:8457 | SEQ ID NO:16469 |
| | | AA | KSSQSLLHSEGKTYLY | EISNRFS | MQSTQFPLT |
| | | | SEQ ID NO:446 | SEQ ID NO:8458 | SEQ ID NO:16470 |
| iPS:392890 | | NA | CGGGCGAGTCAGGGCATTAG CAATTATTTAGCC | GCTGCATCCAGTTTGCAA AGT | CAACAGTATAATAGTTACCC ATTCACT |
| | 21-225_20H9 | | SEQ ID NO:447 | SEQ ID NO:8459 | SEQ ID NO:16471 |
| | | AA | RASQGISNYLA | AASSLQS | QQYNSYPFT |
| | | | SEQ ID NO:448 | SEQ ID NO:8460 | SEQ ID NO:16472 |
| iPS:392892 | | NA | CGGGCGAGTCAGGACATTAG CAATTATTTAGCC | GCTGCATCCAGTTTGCAA AGT | CAACAGTATCATAGTTTCCC ATTCACT |
| | 21-225_20C11 | | SEQ ID NO:449 | SEQ ID NO:8461 | SEQ ID NO:16473 |

612

FIGURE 49

| | | | | | |
|---|---|---|---|---|---|
| | | AA | RASQDISNYLA | AASSLQS | QQYHSFPFT |
| | | | SEQ ID NO:450 | SEQ ID NO:8462 | SEQ ID NO:16474 |
| iPS:392894 | | NA | CGGGCAAGTCAGGGCATTAG AAATGATTTAGGC | ACTTCATCCAGTTTGCAA AGT | CTACAACATAATAGTTACCC GTGGACG |
| | 21-225_21G2 | | SEQ ID NO:451 | SEQ ID NO:8463 | SEQ ID NO:16475 |
| | | AA | RASQGIRNDLG | TSSSLQS | LQHNSYPWT |
| | | | SEQ ID NO:452 | SEQ ID NO:8464 | SEQ ID NO:16476 |
| iPS:392896 | | NA | CGGGCAAGTCAGGGCGTTAG AAATGATTTAGGC | GCTGCATCCAGTTTGCAA AGT | CTACAGCATAGTAGTTACCC GCTCACT |
| | 21-225_21G7 | | SEQ ID NO:453 | SEQ ID NO:8465 | SEQ ID NO:16477 |
| | | AA | RASQGVRNDLG | AASSLQS | LQHSSYPLT |
| | | | SEQ ID NO:454 | SEQ ID NO:8466 | SEQ ID NO:16478 |
| iPS:392898 | | NA | AGGGCCAGTCAGAGTTTTAG CAGCAGCTACTTAGCC | GGTGCATCCAGCAGGGC CACT | CAGCAGTATGGTAGCTCACG CAGT |
| | 21-225_21H10 | | SEQ ID NO:455 | SEQ ID NO:8467 | SEQ ID NO:16479 |
| | | AA | RASQSFSSSYLA | GASSRAT | QQYGSSRS |
| | | | SEQ ID NO:456 | SEQ ID NO:8468 | SEQ ID NO:16480 |
| iPS:392900 | | NA | CGGGCAAGTCAGGGCATTAG AAATGATTTAGGC | GCTGCATCCAGTTTACAA AGT | CTACAGCATAATAGTTACCC GCTCACT |
| | 21-225_22F2 | | SEQ ID NO:457 | SEQ ID NO:8469 | SEQ ID NO:16481 |
| | | AA | RASQGIRNDLG | AASSLQS | LQHNSYPLT |
| | | | SEQ ID NO:458 | SEQ ID NO:8470 | SEQ ID NO:16482 |
| iPS:392902 | | NA | CGGGCAAGTCAGAACATTTT TAGTTATTTAAAT | GCTGCATCCAGTTTGCAA AGT | CAACAGAGTTACAGTACCCC CTTATTCACT |
| | 21-225_22D5 | | SEQ ID NO:459 | SEQ ID NO:8471 | SEQ ID NO:16483 |
| | | AA | RASQNIFSYLN | AASSLQS | QQSYSTPLFT |
| | | | SEQ ID NO:460 | SEQ ID NO:8472 | SEQ ID NO:16484 |
| iPS:392904 | | NA | CGGGCAAGTCAGGGCATTAG AAATGATTTAGGC | GCTGCTTCCAGTTTGCAA AGT | CTACAGCATGCCAGTTACCC GCTCACT |
| | 21-225_22G9 | | SEQ ID NO:461 | SEQ ID NO:8473 | SEQ ID NO:16485 |

FIGURE 49

| | | | | | |
|---|---|---|---|---|---|
| | | AA | RASQGIRNDLG | AASSLQS | LQHASYPLT |
| | | | SEQ ID NO:462 | SEQ ID NO:8474 | SEQ ID NO:16486 |
| iPS:392908 | | NA | CGGGCAAGTCAGGGCATTAG AAATGATTTAGGC | GTTGCATCCAGTTTGCAA AGT | CTACAGCATAATAGTTACCC GTGGACG |
| | 21-225_23F12 | | SEQ ID NO:463 | SEQ ID NO:8475 | SEQ ID NO:16487 |
| | | AA | RASQGIRNDLG | VASSLQS | LQHNSYPWT |
| | | | SEQ ID NO:464 | SEQ ID NO:8476 | SEQ ID NO:16488 |
| iPS:392912 | | NA | CGGGCAAGTCAGGGCATTAG AAATGATTTAGGC | GCTGCATCCAGTTTGCAA AGT | CTACAGCATAATAGTTACCC ATTCACT |
| | 21-225_25A9 | | SEQ ID NO:465 | SEQ ID NO:8477 | SEQ ID NO:16489 |
| | | AA | RASQGIRNDLG | AASSLQS | LQHNSYPFT |
| | | | SEQ ID NO:466 | SEQ ID NO:8478 | SEQ ID NO:16490 |
| iPS:392914 | | NA | CGGACAAGTCAGGGCATTAG AAATGATTTAGGC | GCTGCATCCAGTTTACAA AGT | CTACAGCATTATAGTTTCCCT CGGACG |
| | 21-225_25D12 | | SEQ ID NO:467 | SEQ ID NO:8479 | SEQ ID NO:16491 |
| | | AA | RTSQGIRNDLG | AASSLQS | LQHYSFPRT |
| | | | SEQ ID NO:468 | SEQ ID NO:8480 | SEQ ID NO:16492 |
| iPS:392916 | | NA | CGGGCGAGTCAGGGTATTAG CAGCTGGTTAGCC | GGTGCATCCAGTTTGCA AAGT | CAACAGTATGACAGTTTCCC TCGGACG |
| | 21-225_27C5 | | SEQ ID NO:469 | SEQ ID NO:8481 | SEQ ID NO:16493 |
| | | AA | RASQGISSWLA | GASSLQS | QQYDSFPRT |
| | | | SEQ ID NO:470 | SEQ ID NO:8482 | SEQ ID NO:16494 |
| iPS:392918 | | NA | CGGGCAAGTCAGGGCATTAG AAATGATTTAGGC | ACTGCATCCAGTTTGCAA AGT | CTACAGCATAATACTTACCC GTGGACG |
| | 21-225_28F5 | | SEQ ID NO:471 | SEQ ID NO:8483 | SEQ ID NO:16495 |
| | | AA | RASQGIRNDLG | TASSLQS | LQHNTYPWT |
| | | | SEQ ID NO:472 | SEQ ID NO:8484 | SEQ ID NO:16496 |
| iPS:392920 | | NA | CGGGCAAGTCAGGGCATTAG AAATGATTTAGGC | GCTGCATCCAGTTTGCAA AGT | CTACAGCATAATAGTTACCC GCTCACT |
| | 21-225_29G4 | | SEQ ID NO:473 | SEQ ID NO:8485 | SEQ ID NO:16497 |

FIGURE 49

|  |  | AA | RASQGIRNDLG | AASSLQS | LQHNSYPLT |
|---|---|---|---|---|---|
|  |  |  | SEQ ID NO:474 | SEQ ID NO:8486 | SEQ ID NO:16498 |
| iPS:392922 |  | NA | CGGGCAACTCAGAACATTTTCAGCTATTTAAAT | ACTGCATCCAGTTTGCAAGGT | CAACTCAGCTACAGTCCCCCGTACACT |
|  | 21-225_30G4 |  | SEQ ID NO:475 | SEQ ID NO:8487 | SEQ ID NO:16499 |
|  |  | AA | RATQNIFSYLN | TASSLQG | QLSYSPPYT |
|  |  |  | SEQ ID NO:476 | SEQ ID NO:8488 | SEQ ID NO:16500 |
| iPS:392924 |  | NA | AGGTCTAGTCAGAGCCTCCTCCATAGTGATGGAAGGACCTATTTGTAT | GAACTTTCCAACCGGTTCTCT | TTGCAAAGTATACAATATCCCATCACC |
|  | 21-225_32H2 |  | SEQ ID NO:477 | SEQ ID NO:8489 | SEQ ID NO:16501 |
|  |  | AA | RSSQSLLHSDGRTYLY | ELSNRFS | LQSIQYPIT |
|  |  |  | SEQ ID NO:478 | SEQ ID NO:8490 | SEQ ID NO:16502 |
| iPS:392928 |  | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
|  | 21-225_25A4 |  | SEQ ID NO:479 | SEQ ID NO:8491 | SEQ ID NO:16503 |
|  |  | AA | KSSQSVLYSSHNNNYLA | WASTRES | QQYYSTPPT |
|  |  |  | SEQ ID NO:480 | SEQ ID NO:8492 | SEQ ID NO:16504 |
| iPS:392930 |  | NA | AAGTCTAGTCAGAGCCTCCTGCATGGTGATGGAAAGACCTATTTGTTT | GAAGTTTCCAATCGGTTCTCT | ATGCAAAGTATACAGCTTCCGTGGACG |
|  | 21-225_25H9 |  | SEQ ID NO:481 | SEQ ID NO:8493 | SEQ ID NO:16505 |
|  |  | AA | KSSQSLLHGDGKTYLF | EVSNRFS | MQSIQLPWT |
|  |  |  | SEQ ID NO:482 | SEQ ID NO:8494 | SEQ ID NO:16506 |
| iPS:392934 |  | NA | CGGGCAAGTCAGGGCATTAGAAATGATTTAGGC | GCTGCATCCAGTTTGCAAAGT | CTACAGCATAATAGTTACCCATTCACT |
|  | 21-225_27D5 |  | SEQ ID NO:483 | SEQ ID NO:8495 | SEQ ID NO:16507 |
|  |  | AA | RASQGIRNDLG | AASSLQS | LQHNSYPFT |
|  |  |  | SEQ ID NO:484 | SEQ ID NO:8496 | SEQ ID NO:16508 |

FIGURE 49

| iPS:392936 | | NA | CGGTCTAGTCAAAGCCTCGT ATATAGTGATGGAGACACCT ACTTGAAT | AAGGTTTCTAACTGGGA CTCT | ATGCATTGTACACACTGGCT CCTT |
|---|---|---|---|---|---|
| | 21-225_28B6 | | SEQ ID NO:485 | SEQ ID NO:8497 | SEQ ID NO:16509 |
| | | AA | RSSQSLVYSDGDTYLN | KVSNWDS | MHCTHWLL |
| | | | SEQ ID NO:486 | SEQ ID NO:8498 | SEQ ID NO:16510 |
| iPS:392938 | | NA | AAGTCTAGTCAGAGCCTCCT GCATAGTGATGGAAAGACCT ATTTGTAT | GAGGTTTCCCACCGGTTC TCT | ATGCAAAGTATACAGCATCC GTTCACT |
| | 21-225_29H4 | | SEQ ID NO:487 | SEQ ID NO:8499 | SEQ ID NO:16511 |
| | | AA | KSSQSLLHSDGKTYLY | EVSHRFS | MQSIQHPFT |
| | | | SEQ ID NO:488 | SEQ ID NO:8500 | SEQ ID NO:16512 |
| iPS:392940 | | NA | CGGGCAAGTCAGGGCATTAG AAATGATTTAGGC | GCTGCATCCAGTTTGCAA AGT | CTACAGCATAATACTTACCC ATTCACT |
| | 21-225_29D9 | | SEQ ID NO:489 | SEQ ID NO:8501 | SEQ ID NO:16513 |
| | | AA | RASQGIRNDLG | AASSLQS | LQHNTYPFT |
| | | | SEQ ID NO:490 | SEQ ID NO:8502 | SEQ ID NO:16514 |
| iPS:392942 | | NA | CGGGCAAGTCAGGACATTAG AGATGATTTAGGC | GGTGCATTCAGCTTGCA AAGT | CTACAGCATACTAGTTACCC TCCTACT |
| | 21-225_30E9 | | SEQ ID NO:491 | SEQ ID NO:8503 | SEQ ID NO:16515 |
| | | AA | RASQDIRDDLG | GAFSLQS | LQHTSYPPT |
| | | | SEQ ID NO:492 | SEQ ID NO:8504 | SEQ ID NO:16516 |
| iPS:392944 | | NA | CGGGCAAGTCAGGACATTAG AAGTGATTTAGGC | GCTGCATCCAGTTTGCAA AGT | ATACAACATATTATTTACCC TCCTACT |
| | 21-225_31H5 | | SEQ ID NO:493 | SEQ ID NO:8505 | SEQ ID NO:16517 |
| | | AA | RASQDIRSDLG | AASSLQS | IQHIIYPPT |
| | | | SEQ ID NO:494 | SEQ ID NO:8506 | SEQ ID NO:16518 |
| iPS:392948 | | NA | CGGGCAAGTCAGGACATTAG AAATGATTTAGGC | GCTGCATCCAGTTTGCAA AGT | CTACAGCATAATAGTTACCC CTTCACT |
| | 21-225_25G5 | | SEQ ID NO:495 | SEQ ID NO:8507 | SEQ ID NO:16519 |

FIGURE 49

| | | | | | |
|---|---|---|---|---|---|
| | | AA | RASQDIRNDLG | AASSLQS | LQHNSYPFT |
| | | | SEQ ID NO:496 | SEQ ID NO:8508 | SEQ ID NO:16520 |
| iPS:392950 | | NA | CGGGCGAGTCAGGGCATTAGCAATTATTTAGCC | GCTGCATCCAGTTTGCAAAGT | CAACAGTATCATAGTTACCCATTCACT |
| | 21-225_25C10 | | SEQ ID NO:497 | SEQ ID NO:8509 | SEQ ID NO:16521 |
| | | AA | RASQGISNYLA | AASSLQS | QQYHSYPFT |
| | | | SEQ ID NO:498 | SEQ ID NO:8510 | SEQ ID NO:16522 |
| iPS:392952 | | NA | CGGGCGAGTCAGGACATTAGCAATTATTTAGCC | GCTGCATCCAGTTTGCGAAGT | CAACAGTATCATAGTTACCCATTCACT |
| | 21-225_26G1 | | SEQ ID NO:499 | SEQ ID NO:8511 | SEQ ID NO:16523 |
| | | AA | RASQDISNYLA | AASSLRS | QQYHSYPFT |
| | | | SEQ ID NO:500 | SEQ ID NO:8512 | SEQ ID NO:16524 |
| iPS:392954 | | NA | CGGGCAAGTCAGAGCATTAGCAGCTATTTAAAT | GCTGCATCCAGCTTGCAAAGT | CAACAGAGTTACAGTACCCCTACGTGGACG |
| | 21-225_26A10 | | SEQ ID NO:501 | SEQ ID NO:8513 | SEQ ID NO:16525 |
| | | AA | RASQSISSYLN | AASSLQS | QQSYSTPTWT |
| | | | SEQ ID NO:502 | SEQ ID NO:8514 | SEQ ID NO:16526 |
| iPS:392956 | | NA | CGGGCGAGTCAGGGTATTAGTAGTTGGTTAGCC | GGTGCATCCAGTTTGCAAAGT | CAACAGTCTGACAGTTTCCCTCGGACG |
| | 21-225_27A11 | | SEQ ID NO:503 | SEQ ID NO:8515 | SEQ ID NO:16527 |
| | | AA | RASQGISSWLA | GASSLQS | QQSDSFPRT |
| | | | SEQ ID NO:504 | SEQ ID NO:8516 | SEQ ID NO:16528 |
| iPS:392958 | | NA | CGGGCAAGTCAGGGCATTAGAAATGATTTAGGC | ATTGCATCCAGTTTGCAAAGT | CTACAGCATAATACTTACCCGTGGACG |
| | 21-225_28C7 | | SEQ ID NO:505 | SEQ ID NO:8517 | SEQ ID NO:16529 |
| | | AA | RASQGIRNDLG | IASSLQS | LQHNTYPWT |
| | | | SEQ ID NO:506 | SEQ ID NO:8518 | SEQ ID NO:16530 |
| iPS:392960 | 21 225 29E6 | NA | AAGTCCAGCCAGAGTGTTTTATACAGCTCCCACAATAACTACTACTTAACT | TGGGCATCTTCCCGGGAATCC | CAGCAGTATTATAGTACTCCTCCGACG |

617

FIGURE 49

| | 21-225_29E6 | | SEQ ID NO:507 | SEQ ID NO:8519 | SEQ ID NO:16531 |
|---|---|---|---|---|---|
| | | AA | KSSQSVLYSSHNNYYLT | WASSRES | QQYYSTPPT |
| | | | SEQ ID NO:508 | SEQ ID NO:8520 | SEQ ID NO:16532 |
| iPS:392962 | 21-225_30A1 | NA | CGGGCGAGTCAGGGCATTAGCAATTATTTAGCC | GCTGCATCCAGTTTGCAAACT | CAACAGTATAATAGTTACCCATTCACT |
| | | | SEQ ID NO:509 | SEQ ID NO:8521 | SEQ ID NO:16533 |
| | | AA | RASQGISNYLA | AASSLQT | QQYNSYPFT |
| | | | SEQ ID NO:510 | SEQ ID NO:8522 | SEQ ID NO:16534 |
| iPS:392964 | 21-225_31A8 | NA | CGGGCAAGTCAGGACATTAGAAGTGATTTAGGC | GCTGTATCCAGTTTGCAAAGT | CTACAGCATACTATTTACCCTCCTACT |
| | | | SEQ ID NO:511 | SEQ ID NO:8523 | SEQ ID NO:16535 |
| | | AA | RASQDIRSDLG | AVSSLQS | LQHTIYPPT |
| | | | SEQ ID NO:512 | SEQ ID NO:8524 | SEQ ID NO:16536 |
| iPS:392966 | 21-225_32G3 | NA | CGGGCGAGTCAGGCCATTAGCAATTATTTAGCC | GATACATCCAGTTTGCAAAGT | CAACAGTATCATAGTTACCCGCTCACT |
| | | | SEQ ID NO:513 | SEQ ID NO:8525 | SEQ ID NO:16537 |
| | | AA | RASQAISNYLA | DTSSLQS | QQYHSYPLT |
| | | | SEQ ID NO:514 | SEQ ID NO:8526 | SEQ ID NO:16538 |
| iPS:392968 | 21-225_25B6 | NA | CGGGCAAGTCAGGGCATTAGAAATGATTTAGGC | CGTGCATCCAGTTTGCAAAGT | CTACAGCATAATAGTTACCCATTCACT |
| | | | SEQ ID NO:515 | SEQ ID NO:8527 | SEQ ID NO:16539 |
| | | AA | RASQGIRNDLG | RASSLQS | LQHNSYPFT |
| | | | SEQ ID NO:516 | SEQ ID NO:8528 | SEQ ID NO:16540 |
| iPS:392972 | 21-225_26A2 | NA | CGGGCAAGTCAGGGCATTAGAAGTGATTTAGGC | ACTGCATCCAGTTTGCAGAGT | CTACAGCATAATCGTTACCCGTGGACG |
| | | | SEQ ID NO:517 | SEQ ID NO:8529 | SEQ ID NO:16541 |
| | | AA | RASQGIRSDLG | TASSLQS | LQHNRYPWT |
| | | | SEQ ID NO:518 | SEQ ID NO:8530 | SEQ ID NO:16542 |
| iPS:392974 | | NA | CGGGCAAGTCAGGCCATTAGAAATGATTTAGGC | GCTGCATCCAGTTTGCAAAGT | CTACAGCATTATAATTACCCTCGCAGT |

FIGURE 49

| | 21-225_26A11 | | SEQ ID NO:519 | SEQ ID NO:8531 | SEQ ID NO:16543 |
|---|---|---|---|---|---|
| | | AA | RASQAIRNDLG | AASSLQS | LQHYNYPRS |
| | | | SEQ ID NO:520 | SEQ ID NO:8532 | SEQ ID NO:16544 |
| iPS:392976 | | NA | CGGGCGAGTCAGGGCATTAG CAATTATTTAGCC | GGTGCATCCAGTTTGCA AAGT | CAACAGTATTATAGTTACCC ATTCACT |
| | 21-225_27H12 | | SEQ ID NO:521 | SEQ ID NO:8533 | SEQ ID NO:16545 |
| | | AA | RASQGISNYLA | GASSLQS | QQYYSYPFT |
| | | | SEQ ID NO:522 | SEQ ID NO:8534 | SEQ ID NO:16546 |
| iPS:392978 | | NA | CGGGCAAGTCAGGGCATTAG AAATGATTTAGGC | GCTGCATCCAGTTTGCAA AGT | CTACAGCATAATAGTTACCC ATTCACT |
| | 21-225_28B8 | | SEQ ID NO:523 | SEQ ID NO:8535 | SEQ ID NO:16547 |
| | | AA | RASQGIRNDLG | AASSLQS | LQHNSYPFT |
| | | | SEQ ID NO:524 | SEQ ID NO:8536 | SEQ ID NO:16548 |
| iPS:392980 | | NA | CGGGCAAGTCAGGGCATTAG AAATGATTTAGGC | GCTGCATCCAGTTTGCAA AGT | CTACAGCATAATAGTTACCC GCTCACT |
| | 21-225_29H6 | | SEQ ID NO:525 | SEQ ID NO:8537 | SEQ ID NO:16549 |
| | | AA | RASQGIRNDLG | AASSLQS | LQHNSYPLT |
| | | | SEQ ID NO:526 | SEQ ID NO:8538 | SEQ ID NO:16550 |
| iPS:392982 | | NA | CGGGCAAGTCAGGACATTAG AAGTGATTTAGGC | GCTGCATCCAGTTTGCAA AGT | CTACAGCATACTATTTACCC TCCTACT |
| | 21-225_30D1 | | SEQ ID NO:527 | SEQ ID NO:8539 | SEQ ID NO:16551 |
| | | AA | RASQDIRSDLG | AASSLQS | LQHTIYPPT |
| | | | SEQ ID NO:528 | SEQ ID NO:8540 | SEQ ID NO:16552 |
| iPS:392984 | | NA | CGGGCAAGTCAGAGCATTAG CAACTATTTAAAT | GCTGCATCCAGTTTGCAA AGT | CAACAGAGTTACAGTACCCC ATTCACT |
| | 21-225_30E11 | | SEQ ID NO:529 | SEQ ID NO:8541 | SEQ ID NO:16553 |
| | | AA | RASQSISNYLN | AASSLQS | QQSYSTPFT |
| | | | SEQ ID NO:530 | SEQ ID NO:8542 | SEQ ID NO:16554 |
| iPS:392986 | | NA | CGGGCAAGTCAGGACATTAG AAGTGATTTAGGC | GGTGCATCCAGTTTGCA AAGT | CTACAGCATATTATTTACCCT CCTACT |

FIGURE 49

| | 21-225_31B8 | | SEQ ID NO:531 | SEQ ID NO:8543 | SEQ ID NO:16555 |
|---|---|---|---|---|---|
| | | AA | RASQDIRSDLG | GASSLQS | LQHIIYPPT |
| | | | SEQ ID NO:532 | SEQ ID NO:8544 | SEQ ID NO:16556 |
| iPS:392988 | 21-225_25E6 | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:533 | SEQ ID NO:8545 | SEQ ID NO:16557 |
| | | AA | RASQGIRNDLG | AASSLQS | LQHNSYPLT |
| | | | SEQ ID NO:534 | SEQ ID NO:8546 | SEQ ID NO:16558 |
| iPS:392990 | 21-225_25H10 | NA | CGGGCAAGTCGGGGCATTAG AAATGATTTAGGC | GCTGCATTCAGTTTGCAA AGT | CTACAGCATAATAGTTACCC GCTCACT |
| | | | SEQ ID NO:535 | SEQ ID NO:8547 | SEQ ID NO:16559 |
| | | AA | RASRGIRNDLG | AAFSLQS | LQHNSYPLT |
| | | | SEQ ID NO:536 | SEQ ID NO:8548 | SEQ ID NO:16560 |
| iPS:392992 | 21-225_26C4 | NA | AAGTCCAGCCAGAGTGTTTT ATACCGCTCCAACAATTACA ACTACTTAGCT | TGGGCATCTACCCGGGA ATCC | CAGCAATATTATAGTACTCC TCCGACG |
| | | | SEQ ID NO:537 | SEQ ID NO:8549 | SEQ ID NO:16561 |
| | | AA | KSSQSVLYRSNNYNYLA | WASTRES | QQYYSTPPT |
| | | | SEQ ID NO:538 | SEQ ID NO:8550 | SEQ ID NO:16562 |
| iPS:392994 | 21-225_26G11 | NA | AAGTCTAGTCAGACCCTCCT GCATGGTGAAGGAAAGACC TATTTGTAT | GAAGTTTCCAACCGGTTC TCT | ATGCAAAGTATAAAGCTTCC GCTCACT |
| | | | SEQ ID NO:539 | SEQ ID NO:8551 | SEQ ID NO:16563 |
| | | AA | KSSQTLLHGEGKTYLY | EVSNRFS | MQSIKLPLT |
| | | | SEQ ID NO:540 | SEQ ID NO:8552 | SEQ ID NO:16564 |
| iPS:392996 | 21-225_28B1 | NA | CGGGCGAGTCAGGCTATCAA TGACTGGTTAGCC | GCTGCATCCAGTTTCCAA AGT | CAACAGGCTAGCAGTTTCCC ATTCACT |
| | | | SEQ ID NO:541 | SEQ ID NO:8553 | SEQ ID NO:16565 |
| | | AA | RASQAINDWLA | AASSFQS | QQASSFPFT |
| | | | SEQ ID NO:542 | SEQ ID NO:8554 | SEQ ID NO:16566 |

FIGURE 49

| iPS:392998 | | NA | CGGGCAAGTCAGGGCATTAG AAATGATTTAGGC | GCTGCTTCTAGTTTGCAA AAT | CTACAGCATAATCGTTACCC ATTCACT |
|---|---|---|---|---|---|
| | 21-225_28A9 | | SEQ ID NO:543 | SEQ ID NO:8555 | SEQ ID NO:16567 |
| | | AA | RASQGIRNDLG | AASSLQN | LQHNRYPFT |
| | | | SEQ ID NO:544 | SEQ ID NO:8556 | SEQ ID NO:16568 |
| iPS:393000 | | NA | CGGGCAAGTCAGGGCATTAG AAATGATTTAGGC | GCTGCATCCAGTTTACAA AGT | CTACAGCATAATAGTTACCC ATTCACT |
| | 21-225_29D7 | | SEQ ID NO:545 | SEQ ID NO:8557 | SEQ ID NO:16569 |
| | | AA | RASQGIRNDLG | AASSLQS | LQHNSYPFT |
| | | | SEQ ID NO:546 | SEQ ID NO:8558 | SEQ ID NO:16570 |
| iPS:393002 | | NA | CGGGCAAGTCAGAACATTTA CAGCTATTTAAAT | GCTGCATCCAGTTTGCAT AGT | CAACAGAGTTACAGTACTCC GCTCACT |
| | 21-225_30G1 | | SEQ ID NO:547 | SEQ ID NO:8559 | SEQ ID NO:16571 |
| | | AA | RASQNIYSYLN | AASSLHS | QQSYSTPLT |
| | | | SEQ ID NO:548 | SEQ ID NO:8560 | SEQ ID NO:16572 |
| iPS:393004 | | NA | CGGGCAAGTCAGGACATTAG AAGTGATTTAGGC | GCTGCATCCAGTTTGCAA AGT | CTACAGCATACTATTTACCC TCCTACT |
| | 21-225_30G11 | | SEQ ID NO:549 | SEQ ID NO:8561 | SEQ ID NO:16573 |
| | | AA | RASQDIRSDLG | AASSLQS | LQHTIYPPT |
| | | | SEQ ID NO:550 | SEQ ID NO:8562 | SEQ ID NO:16574 |
| iPS:393006 | | NA | CGGGCAAGTCAGGGCATTAG AAATGATTTAGGC | CCTGCATCCAGTTTGCAA AGT | CTACAGGATAATAGTTACCC TTTCACT |
| | 21-225_31G9 | | SEQ ID NO:551 | SEQ ID NO:8563 | SEQ ID NO:16575 |
| | | AA | RASQGIRNDLG | PASSLQS | LQDNSYPFT |
| | | | SEQ ID NO:552 | SEQ ID NO:8564 | SEQ ID NO:16576 |
| iPS:393010 | | NA | CGGGCGAGTCAGGGTATTAG CAACTGGTTAGCC | ACTGCATCCAGTTTGCAA GGT | CAACAGGCTAACAGTTTCCC AATCACT |
| | 21-225_25E11 | | SEQ ID NO:553 | SEQ ID NO:8565 | SEQ ID NO:16577 |
| | | AA | RASQGISNWLA | TASSLQG | QQANSFPIT |
| | | | SEQ ID NO:554 | SEQ ID NO:8566 | SEQ ID NO:16578 |

EP 4 435 105 A2

FIGURE 49

| iPS:393012 | 21-225_26G7 | NA | AAGTCTAGTCAGAGCCTCCTGCATAGTGAGGGAAAGACCTATTTGTAT | GAAGTTTCCCACCGGCTCTCT | ATGCAAAGTATACAGCTTCCGCTCACT |
| | | | SEQ ID NO:555 | SEQ ID NO:8567 | SEQ ID NO:16579 |
| | | AA | KSSQSLLHSEGKTYLY | EVSHRLS | MQSIQLPLT |
| | | | SEQ ID NO:556 | SEQ ID NO:8568 | SEQ ID NO:16580 |
| iPS:393014 | 21-225_26D12 | NA | CGGGCGAGTCAGGGTATTAGTAGTTGGTTAGCC | GGTGCATCCAGTTTGCAAAGT | CAACAGTCTGACAGTTTCCCTCGGACG |
| | | | SEQ ID NO:557 | SEQ ID NO:8569 | SEQ ID NO:16581 |
| | | AA | RASQGISSWLA | GASSLQS | QQSDSFPRT |
| | | | SEQ ID NO:558 | SEQ ID NO:8570 | SEQ ID NO:16582 |
| iPS:393016 | 21-225_28F11 | NA | CGGGCGAGTCAGGGTATTAGCAACTGGTTAGCC | GCTGCATCCAATTTGCAAAGT | CAACAGGCTAACAGTCTCCCATTCACT |
| | | | SEQ ID NO:559 | SEQ ID NO:8571 | SEQ ID NO:16583 |
| | | AA | RASQGISNWLA | AASNLQS | QQANSLPFT |
| | | | SEQ ID NO:560 | SEQ ID NO:8572 | SEQ ID NO:16584 |
| iPS:393018 | 21-225_29B8 | NA | CGGGCAAGTCAGGGCATTAGAAATGATTTAGGC | GCTGCATCCAGTTTGCAAAGT | CTACAGCATAATAGTTACCCGCTCACT |
| | | | SEQ ID NO:561 | SEQ ID NO:8573 | SEQ ID NO:16585 |
| | | AA | RASQGIRNDLG | AASSLQS | LQHNSYPLT |
| | | | SEQ ID NO:562 | SEQ ID NO:8574 | SEQ ID NO:16586 |
| iPS:393020 | 21-225_30E2 | NA | CAGGCGAGTCAGTACATTAGCAACTATTTAAAT | GATGGATCCAGTTTGGAAACA | CAACAGTATGATAATCTCCCGATCACC |
| | | | SEQ ID NO:563 | SEQ ID NO:8575 | SEQ ID NO:16587 |
| | | AA | QASQYISNYLN | DGSSLET | QQYDNLPIT |
| | | | SEQ ID NO:564 | SEQ ID NO:8576 | SEQ ID NO:16588 |
| iPS:393022 | 21-225_30H11 | NA | CGGGCAAGTCAGGGCATTAGAAATGATTTAGGC | CCTGCATCCAGTTTGCAAAGT | CTACAGGATAATAGTCATCCATTCACT |
| | | | SEQ ID NO:565 | SEQ ID NO:8577 | SEQ ID NO:16589 |
| | | AA | RASQGIRNDLG | PASSLQS | LQDNSHPFT |

622

FIGURE 49

| | | | SEQ ID NO:566 | SEQ ID NO:8578 | SEQ ID NO:16590 |
|---|---|---|---|---|---|
| iPS:393024 | | NA | CGGGCGAGTCAGGGTATTAC CAGCTGGTTAACT | GATACATCCAGTTTGCA AAGT | CAACAGGGTAACAGTTTCCC ATTCACT |
| | 21-225_31H9 | | SEQ ID NO:567 | SEQ ID NO:8579 | SEQ ID NO:16591 |
| | | AA | RASQGITSWLT | DTSSLQS | QQGNSFPFT |
| | | | SEQ ID NO:568 | SEQ ID NO:8580 | SEQ ID NO:16592 |
| iPS:393026 | | NA | CGGGCAAGTCAGGGCATTAG AAATGATTTAGGC | ATTGCATCCAGTTTGCAA AGT | CTACAGCATAATAGTTACCC GTGGACG |
| | 21-225_32B6 | | SEQ ID NO:569 | SEQ ID NO:8581 | SEQ ID NO:16593 |
| | | AA | RASQGIRNDLG | IASSLQS | LQHNSYPWT |
| | | | SEQ ID NO:570 | SEQ ID NO:8582 | SEQ ID NO:16594 |
| iPS:393028 | | NA | CGGGCGAGTCAGGATATTTT CGACTGGTTAGCC | GCTGCATCCAGTTTGCAA AGT | CAACAGGCTTACAGTTTCCC GTGGACG |
| | 21-225_25D7 | | SEQ ID NO:571 | SEQ ID NO:8583 | SEQ ID NO:16595 |
| | | AA | RASQDIFDWLA | AASSLQS | QQAYSFPWT |
| | | | SEQ ID NO:572 | SEQ ID NO:8584 | SEQ ID NO:16596 |
| iPS:393030 | | NA | CGGGCAAGTCAGGGCATTAG AACTGATTTAGGC | GCTGCATCCAGTTTACAA AGT | CTACAGCATAATAGTTACCC GCTCACT |
| | 21-225_25H11 | | SEQ ID NO:573 | SEQ ID NO:8585 | SEQ ID NO:16597 |
| | | AA | RASQGIRTDLG | AASSLQS | LQHNSYPLT |
| | | | SEQ ID NO:574 | SEQ ID NO:8586 | SEQ ID NO:16598 |
| iPS:393032 | | NA | AAGTCTAGTCAGAGCCTCCT GCATGGTGATGGAAAGACCT ATTTGTAT | GAAGTTTCCAACCGGTTC TCT | ATGCAAAGTATACAGCTTCC GTGGACG |
| | 21-225_26F8 | | SEQ ID NO:575 | SEQ ID NO:8587 | SEQ ID NO:16599 |
| | | AA | KSSQSLLHGDGKTYLY | EVSNRFS | MQSIQLPWT |
| | | | SEQ ID NO:576 | SEQ ID NO:8588 | SEQ ID NO:16600 |
| iPS:393034 | | NA | CGGGCAAGTCAGGGCATTAG AAATGATTTAGGC | GTTGCATCCAGTTTGCAA AGT | CTACAGCATAATAGTTACCC GCTCACT |
| | 21-225_27F2 | | SEQ ID NO:577 | SEQ ID NO:8589 | SEQ ID NO:16601 |

FIGURE 49

| | | | | | |
|---|---|---|---|---|---|
| | | AA | RASQGIRNDLG | VASSLQS | LQHNSYPLT |
| | | | SEQ ID NO:578 | SEQ ID NO:8590 | SEQ ID NO:16602 |
| iPS:393036 | 21-225_28G3 | NA | AAGTCTAGTCAGAGCCTCCTACATGGTGATGGAAAGACCTATTTGTAT | GAAGTTTCCAACCGGTTCTCT | ATGCAAAGTATACAGATTCCGTGGACG |
| | | | SEQ ID NO:579 | SEQ ID NO:8591 | SEQ ID NO:16603 |
| | | AA | KSSQSLLHGDGKTYLY | EVSNRFS | MQSIQIPWT |
| | | | SEQ ID NO:580 | SEQ ID NO:8592 | SEQ ID NO:16604 |
| iPS:393038 | 21-225_29D8 | NA | CGGGCAAGTCAGGGCATTAGAAATGATTTAGGC | ACTGCATCCAGTTTGCAAAGT | CTACAGCATAATAGTTACCCATTCACT |
| | | | SEQ ID NO:581 | SEQ ID NO:8593 | SEQ ID NO:16605 |
| | | AA | RASQGIRNDLG | TASSLQS | LQHNSYPFT |
| | | | SEQ ID NO:582 | SEQ ID NO:8594 | SEQ ID NO:16606 |
| iPS:393040 | 21-225_30E3 | NA | CGGGCAAGTCAGGACATTAGAGATGATTTAGGC | GCTGCATTCAGCTTGCAAAGT | CTACAGCATACTAGTTACCCTCCTACT |
| | | | SEQ ID NO:583 | SEQ ID NO:8595 | SEQ ID NO:16607 |
| | | AA | RASQDIRDDLG | AAFSLQS | LQHTSYPPT |
| | | | SEQ ID NO:584 | SEQ ID NO:8596 | SEQ ID NO:16608 |
| iPS:393042 | 21-225_31F1 | NA | CGGGCAAGTCAGAGGATTAGCAGCTATTTAAAT | GCTGCATCCAGTTCGCAAAGT | CAACAGAGTTACATTACCCCGCTCACT |
| | | | SEQ ID NO:585 | SEQ ID NO:8597 | SEQ ID NO:16609 |
| | | AA | RASQRISSYLN | AASSSQS | QQSYITPLT |
| | | | SEQ ID NO:586 | SEQ ID NO:8598 | SEQ ID NO:16610 |
| iPS:393044 | 21-225_25B8 | NA | AGGGCCAGTCAGAGTGTTAGAAGTAATTTAGCC | GGTGCATCCACCAGGGCCACT | CAGCAGTATAATAATTGGCCTCCGTGGCCG |
| | | | SEQ ID NO:587 | SEQ ID NO:8599 | SEQ ID NO:16611 |
| | | AA | RASQSVRSNLA | GASTRAT | QQYNNWPPWP |
| | | | SEQ ID NO:588 | SEQ ID NO:8600 | SEQ ID NO:16612 |
| iPS:393046 | | NA | CGGGCAAGTCAGGCCATTAGAGATGATTTAGGC | GCTGCATCCAGTTTGCAAAGT | CTACAGCATTATAATTACCCTCGCAGT |

FIGURE 49

| | 21-225_25A12 | | SEQ ID NO:589 | SEQ ID NO:8601 | SEQ ID NO:16613 |
|---|---|---|---|---|---|
| | | AA | RASQAIRDDLG | AASSLQS | LQHYNYPRS |
| iPS:393048 | | NA | SEQ ID NO:590<br>CGGGCAAGTCAGGGCATTAG<br>AAATGATTTAGGC | SEQ ID NO:8602<br>GCTGCATCCAGTTTGCAA<br>AGT | SEQ ID NO:16614<br>CTACAGCATAATCGTTACCC<br>GCTCACT |
| | 21-225_27C3 | | SEQ ID NO:591 | SEQ ID NO:8603 | SEQ ID NO:16615 |
| | | AA | RASQGIRNDLG | AASSLQS | LQHNRYPLT |
| iPS:393050 | | NA | SEQ ID NO:592<br>AGGGCCAGTCAGAGTGTTAG<br>CAGCAACTTAGCC | SEQ ID NO:8604<br>GGTGCATCCACCAGGGC<br>CACT | SEQ ID NO:16616<br>CAGCAGTATAATAATTGGCC<br>TCCGTGGCCG |
| | 21-225_28C5 | | SEQ ID NO:593 | SEQ ID NO:8605 | SEQ ID NO:16617 |
| | | AA | RASQSVSSNLA | GASTRAT | QQYNNWPPWP |
| iPS:393054 | | NA | SEQ ID NO:594<br>CGGGCAAGTCAGGGCATTAG<br>AAATGATTTAGGC | SEQ ID NO:8606<br>GCTGCATCCAGTTTGCAA<br>AGT | SEQ ID NO:16618<br>CTACAGCATAATAGTTATCC<br>GCTCACT |
| | 21-225_29G8 | | SEQ ID NO:595 | SEQ ID NO:8607 | SEQ ID NO:16619 |
| | | AA | RASQGIRNDLG | AASSLQS | LQHNSYPLT |
| iPS:393056 | | NA | SEQ ID NO:596<br>CGGGCAAGTCAGGGCATTAG<br>AAATGATTTAGGC | SEQ ID NO:8608<br>ACTGCATCCAGTTTACAA<br>AGT | SEQ ID NO:16620<br>CTACAGCATAATAGTTACCC<br>GTTCACT |
| | 21-225_30F3 | | SEQ ID NO:597 | SEQ ID NO:8609 | SEQ ID NO:16621 |
| | | AA | RASQGIRNDLG | TASSLQS | LQHNSYPFT |
| iPS:393058 | | NA | SEQ ID NO:598<br>CGGGCAAGTCAGGACATTAG<br>AGATGATTTAGGC | SEQ ID NO:8610<br>GCTGCATTCAGCTTGCAA<br>AGT | SEQ ID NO:16622<br>CTACAGCATACTAGTTACCC<br>TCCTACT |
| | 21-225_31H3 | | SEQ ID NO:599 | SEQ ID NO:8611 | SEQ ID NO:16623 |
| | | AA | RASQDIRDDLG | AAFSLQS | LQHTSYPPT |
| iPS:393060 | | NA | SEQ ID NO:600<br>CGGGCAAGTCAGGACATTAG<br>AAGTGATTTAGGC | SEQ ID NO:8612<br>GCTGCATCCAGTTTGCAA<br>AGT | SEQ ID NO:16624<br>CTGCAGCATACTATTTACCC<br>TCCTACT |

FIGURE 49

| | 21-225_32G12 | | SEQ ID NO:601 | SEQ ID NO:8613 | SEQ ID NO:16625 |
|---|---|---|---|---|---|
| | | AA | RASQDIRSDLG | AASSLQS | LQHTIYPPT |
| | | | SEQ ID NO:602 | SEQ ID NO:8614 | SEQ ID NO:16626 |
| iPS:393062 | | NA | CAGGCGAGTCAGGACATTTCCAACTTTTTAAAT | GATGCATCCAATTTGGTAACA | CAACAGTATGATAATCTCCCGATCACC |
| | 21-225_33H3 | | SEQ ID NO:603 | SEQ ID NO:8615 | SEQ ID NO:16627 |
| | | AA | QASQDISNFLN | DASNLVT | QQYDNLPIT |
| | | | SEQ ID NO:604 | SEQ ID NO:8616 | SEQ ID NO:16628 |
| iPS:393064 | | NA | CGGGCAAGTCAGAGCATTAGCAGGTATTTAAGT | GCTGCATCCAGTTTGCAAAGT | CAACAGAGTTACAATATCCCGATCACC |
| | 21-225_33A9 | | SEQ ID NO:605 | SEQ ID NO:8617 | SEQ ID NO:16629 |
| | | AA | RASQSISRYLS | AASSLQS | QQSYNIPIT |
| | | | SEQ ID NO:606 | SEQ ID NO:8618 | SEQ ID NO:16630 |
| iPS:393066 | | NA | CGGGCAAGTCAGAACATTTACAGCTATTTAAAT | GCTGCATCCAGTTTGCATAGT | CAACAGAGTTACAGTACTCCGCTCACT |
| | 21-225_34D3 | | SEQ ID NO:607 | SEQ ID NO:8619 | SEQ ID NO:16631 |
| | | AA | RASQNIYSYLN | AASSLHS | QQSYSTPLT |
| | | | SEQ ID NO:608 | SEQ ID NO:8620 | SEQ ID NO:16632 |
| iPS:393068 | | NA | CGGGCAAGTCAGGACATTAGAAGTGATTTAGGC | GCTGCATCCAGTTTGCAAAGT | CTCCAGCATACTATTTACCCTCCTACT |
| | 21-225_34G9 | | SEQ ID NO:609 | SEQ ID NO:8621 | SEQ ID NO:16633 |
| | | AA | RASQDIRSDLG | AASSLQS | LQHTIYPPT |
| | | | SEQ ID NO:610 | SEQ ID NO:8622 | SEQ ID NO:16634 |
| iPS:393072 | | NA | CGGGCAAGTCAGGACATTAGAAGTGATTTAGGC | GCTGCATCCAGTTTGCAAAGT | CTCCATCATCCTATTTACCCTCCTACT |
| | 21-225_36C5 | | SEQ ID NO:611 | SEQ ID NO:8623 | SEQ ID NO:16635 |
| | | AA | RASQDIRSDLG | AASSLQS | LHHPIYPPT |
| | | | SEQ ID NO:612 | SEQ ID NO:8624 | SEQ ID NO:16636 |
| iPS:393074 | | NA | CGGACAAGTCAGGGCATTAGAAATGATTTAGGC | ACTGCATCCAGTTTGCAAAGT | CTACAGCATAATAGTTACCCGCTCACT |

FIGURE 49

| | | | | | |
|---|---|---|---|---|---|
| | **21-225_33B1** | | | SEQ ID NO:613 | SEQ ID NO:8625 | SEQ ID NO:16637 |
| | | AA | RTSQGIRNDLG | TASSLQS | LQHNSYPLT |
| | | | SEQ ID NO:614 | SEQ ID NO:8626 | SEQ ID NO:16638 |
| iPS:393076 | | NA | CGGGCAAGTCAGGACATTAGAAATGATGTAGGC | GCTGCATCCAGTTTGCAACGT | CTACAGCATTATAGTTACCCTCCTACT |
| | **21-225_33A4** | | SEQ ID NO:615 | SEQ ID NO:8627 | SEQ ID NO:16639 |
| | | AA | RASQDIRNDVG | AASSLQR | LQHYSYPPT |
| | | | SEQ ID NO:616 | SEQ ID NO:8628 | SEQ ID NO:16640 |
| iPS:393078 | | NA | TGGGCGAGTCAGGGCATTAACAGTTATTTAGCC | GCTGCATCCAGTTTGCAAGGT | CAACAGTTTAATAGTTACCCTCTGACG |
| | **21-225_33H11** | | SEQ ID NO:617 | SEQ ID NO:8629 | SEQ ID NO:16641 |
| | | AA | WASQGINSYLA | AASSLQG | QQFNSYPLT |
| | | | SEQ ID NO:618 | SEQ ID NO:8630 | SEQ ID NO:16642 |
| iPS:393080 | | NA | CGGGCGAGTCAGGGTATTAGTAAGTGGTTAGCC | GCTGCATCCAGTTTGCAAAGT | CAACAGGCTAACAGTTTCCCTTTCACT |
| | **21-225_34F3** | | SEQ ID NO:619 | SEQ ID NO:8631 | SEQ ID NO:16643 |
| | | AA | RASQGISKWLA | AASSLQS | QQANSFPFT |
| | | | SEQ ID NO:620 | SEQ ID NO:8632 | SEQ ID NO:16644 |
| iPS:393082 | | NA | CGGGCAAGTCAGAACATTAGGAACTTTTTAAAT | GGTGCATCCACTTTGCAAAGT | CAACAGACTTGCAGTACCCCGCTCACT |
| | **21-225_34C11** | | SEQ ID NO:621 | SEQ ID NO:8633 | SEQ ID NO:16645 |
| | | AA | RASQNIRNFLN | GASTLQS | QQTCSTPLT |
| | | | SEQ ID NO:622 | SEQ ID NO:8634 | SEQ ID NO:16646 |
| iPS:393084 | | NA | CGGGCGAGTCAGGGTATTAGCAAATGGTTAGCC | GCTGCATCCAGTTTGCAGAGT | CAACAGGCTAACAGTTTCCCATTCACT |
| | **21-225_35C6** | | SEQ ID NO:623 | SEQ ID NO:8635 | SEQ ID NO:16647 |
| | | AA | RASQGISKWLA | AASSLQS | QQANSFPFT |
| | | | SEQ ID NO:624 | SEQ ID NO:8636 | SEQ ID NO:16648 |
| iPS:393086 | | NA | CGGGCGAGTCAGGGTATTAGCAGATGGTTAGCC | GCTGCATCCCGTTTGCAAAGT | CAACAGGCTAACAGTTTCCCTTTCACT |

FIGURE 49

| | 21-225_36H5 | | SEQ ID NO:625 | SEQ ID NO:8637 | SEQ ID NO:16649 |
|---|---|---|---|---|---|
| | | AA | RASQGISRWLA | AASRLQS | QQANSFPFT |
| | | | SEQ ID NO:626 | SEQ ID NO:8638 | SEQ ID NO:16650 |
| iPS:393088 | 21-225_33D1 | NA | AAGTCCATCCAGAGTGTTTT ATACAGATCCAACAATAAGA ACTACTTAACT | TGGGCATCTACCCGGGA ATCC | CAGCAATATTATAGTTCTCC GTGCAGT |
| | | | SEQ ID NO:627 | SEQ ID NO:8639 | SEQ ID NO:16651 |
| | | AA | KSIQSVLYRSNNKNYLT | WASTRES | QQYYSSPCS |
| | | | SEQ ID NO:628 | SEQ ID NO:8640 | SEQ ID NO:16652 |
| iPS:393090 | 21-225_33A5 | NA | CGGGCGAGTCAGGGCATTAG CAATTACTTAGCC | GCTGCATCCAGTTTGCAA AGT | CAACAGTATAATAGTTACCC ATTCACT |
| | | | SEQ ID NO:629 | SEQ ID NO:8641 | SEQ ID NO:16653 |
| | | AA | RASQGISNYLA | AASSLQS | QQYNSYPFT |
| | | | SEQ ID NO:630 | SEQ ID NO:8642 | SEQ ID NO:16654 |
| iPS:393092 | 21-225_33C12 | NA | CGGGCAAGTCAGAGCATTAT CAGCTATTTAAAT | GTTGCATCCAGTTTGCAA GGT | CAACAGAGTTACAGTACCCC GTACACT |
| | | | SEQ ID NO:631 | SEQ ID NO:8643 | SEQ ID NO:16655 |
| | | AA | RASQSIISYLN | VASSLQG | QQSYSTPYT |
| | | | SEQ ID NO:632 | SEQ ID NO:8644 | SEQ ID NO:16656 |
| iPS:393094 | 21-225_34C4 | NA | CGGGCAAGTCAGGGCATTAG AAATGATTTAGGC | ACTGCATCCAATTTGCAA AGT | CTACAACATAGTTCTTACCC CATCACC |
| | | | SEQ ID NO:633 | SEQ ID NO:8645 | SEQ ID NO:16657 |
| | | AA | RASQGIRNDLG | TASNLQS | LQHSSYPIT |
| | | | SEQ ID NO:634 | SEQ ID NO:8646 | SEQ ID NO:16658 |
| iPS:393096 | 21-225_34D11 | NA | CGGGCAAGTCAGGACATTAG AAGTGATTTAGGC | GCTGCATCCAGTTTGCAA AGT | CTACAGCATACTATTTACCC TCCTACT |
| | | | SEQ ID NO:635 | SEQ ID NO:8647 | SEQ ID NO:16659 |
| | | AA | RASQDIRSDLG | AASSLQS | LQHTYPPT |
| | | | SEQ ID NO:636 | SEQ ID NO:8648 | SEQ ID NO:16660 |

FIGURE 49

| iPS:393098 | | NA | CGGGCGAGTCAGGGTATTAG CCGGTGGTTAGCC | GCTGCATCCAGGTTGCA AAGT | CAACAGGCTAACAGTTTCCC GTTCACT |
|---|---|---|---|---|---|
| | 21-225_35G6 | | SEQ ID NO:637 | SEQ ID NO:8649 | SEQ ID NO:16661 |
| | | AA | RASQGISRWLA | AASRLQS | QQANSFPFT |
| | | | SEQ ID NO:638 | SEQ ID NO:8650 | SEQ ID NO:16662 |
| iPS:393100 | | NA | CGGGCAAGTCAGAGCATTAT CAGCTATTTAAAT | GTTGCATCCAGTTTGCAA AGT | CAACAGAGTTACAGTACCCC GTACACT |
| | 21-225_36B8 | | SEQ ID NO:639 | SEQ ID NO:8651 | SEQ ID NO:16663 |
| | | AA | RASQSIISYLN | VASSLQS | QQSYSTPYT |
| | | | SEQ ID NO:640 | SEQ ID NO:8652 | SEQ ID NO:16664 |
| iPS:393102 | | NA | CGGACAAGTCAGGACATTAG AAGTGATTTAGGC | GCTGCATCCAGTTTGCAA AGT | CTACAGCATACTATTTACCC TCCTACT |
| | 21-225_33F1 | | SEQ ID NO:641 | SEQ ID NO:8653 | SEQ ID NO:16665 |
| | | AA | RTSQDIRSDLG | AASSLQS | LQHTIYPPT |
| | | | SEQ ID NO:642 | SEQ ID NO:8654 | SEQ ID NO:16666 |
| iPS:393104 | | NA | CGGGCAAGTCAGGACATTAG AAGTGATTTAGGC | GTTGCATCCAGTTTGCAA AGT | CTACAGCATACTATTTACCC TCCTACT |
| | 21-225_33A7 | | SEQ ID NO:643 | SEQ ID NO:8655 | SEQ ID NO:16667 |
| | | AA | RASQDIRSDLG | VASSLQS | LQHTIYPPT |
| | | | SEQ ID NO:644 | SEQ ID NO:8656 | SEQ ID NO:16668 |
| iPS:393106 | | NA | CGGACAAGTCAGGACATCA GAAATGATTTAGGC | GCTGCATCCAGTTTGCAA AGT | CTACAGCATAATAGTTACCC TCCTACT |
| | 21-225_34A6 | | SEQ ID NO:645 | SEQ ID NO:8657 | SEQ ID NO:16669 |
| | | AA | RTSQDIRNDLG | AASSLQS | LQHNSYPPT |
| | | | SEQ ID NO:646 | SEQ ID NO:8658 | SEQ ID NO:16670 |
| iPS:393108 | | NA | CGGGCAAGTCAGAACATTAA CAGGTATTTAAAT | GGTGCATCCAGTTTGCA AAGT | CAACAGACTTACATTACCCC GCTCACT |
| | 21-225_34G11 | | SEQ ID NO:647 | SEQ ID NO:8659 | SEQ ID NO:16671 |
| | | AA | RASQNINRYLN | GASSLQS | QQTYITPLT |
| | | | SEQ ID NO:648 | SEQ ID NO:8660 | SEQ ID NO:16672 |

FIGURE 49

| iPS:393110 | | NA | CGGGCAAGTCAGGACATTAG AAGTGATTTAGGC | GCTGCATCCAGTTTGCAA AGT | CTACAGCATACTATTTACCC TCCTACT |
|---|---|---|---|---|---|
| | 21-225_35B7 | | SEQ ID NO:649 | SEQ ID NO:8661 | SEQ ID NO:16673 |
| | | AA | RASQDIRSDLG | AASSLQS | LQHTIYPPT |
| | | | SEQ ID NO:650 | SEQ ID NO:8662 | SEQ ID NO:16674 |
| iPS:393112 | | NA | CGGGCGAGTCAGGGTATTAG CAGGTGGTTAGCC | GGTGCATACAGTCTGCA AAGT | CAACAGGCTAACAGTTTCCC ATTCACT |
| | 21-225_33G1 | | SEQ ID NO:651 | SEQ ID NO:8663 | SEQ ID NO:16675 |
| | | AA | RASQGISRWLA | GAYSLQS | QQANSFPFT |
| | | | SEQ ID NO:652 | SEQ ID NO:8664 | SEQ ID NO:16676 |
| iPS:393114 | | NA | CGGGCAAGTCAGAGCATTAG CAACTATTTAAAT | GCTGCATCCAGTTTGCAA AGT | CAACAGAGTTACAGTACCCC ATTCACT |
| | 21-225_33G12 | | SEQ ID NO:653 | SEQ ID NO:8665 | SEQ ID NO:16677 |
| | | AA | RASQSISNYLN | AASSLQS | QQSYSTPFT |
| | | | SEQ ID NO:654 | SEQ ID NO:8666 | SEQ ID NO:16678 |
| iPS:393116 | | NA | CGGGCGAGTCAGCTTATTAG CAAGTGGTTAGCC | GCTGCATCCAGTTTGCAA AGT | CAACAGGCTAACAGTTTCCC ATTCACT |
| | 21-225_34G7 | | SEQ ID NO:655 | SEQ ID NO:8667 | SEQ ID NO:16679 |
| | | AA | RASQLISKWLA | AASSLQS | QQANSFPFT |
| | | | SEQ ID NO:656 | SEQ ID NO:8668 | SEQ ID NO:16680 |
| iPS:393118 | | NA | CGGGCAAGTCAGGACATTAG AAATGATTTAGGC | GCTACATCCAGTTTGCAA AGT | CTACAGCATAATAGTTACCC TCCTACT |
| | 21-225_34H11 | | SEQ ID NO:657 | SEQ ID NO:8669 | SEQ ID NO:16681 |
| | | AA | RASQDIRNDLG | ATSSLQS | LQHNSYPPT |
| | | | SEQ ID NO:658 | SEQ ID NO:8670 | SEQ ID NO:16682 |
| iPS:393120 | | NA | CGGGCGAGTCAGGCCATTAG TAATTATTTAGCC | GGTGCGTCCGGTTTGCA AAGT | CAACAGTATAATAGTTACCC ATTCACT |
| | 21-225_35H8 | | SEQ ID NO:659 | SEQ ID NO:8671 | SEQ ID NO:16683 |
| | | AA | RASQAISNYLA | GASGLQS | QQYNSYPFT |
| | | | SEQ ID NO:660 | SEQ ID NO:8672 | SEQ ID NO:16684 |

FIGURE 49

| iPS:393122 | | NA | CGGGCAAGTCAGAGCATTAT CAGCTATTTAAAT | GTTGCATCCAGTTTGCAA AGT | CAACAGAGTTACAGTACCCC GTACACT |
|---|---|---|---|---|---|
| | 21-225_33B2 | | SEQ ID NO:661 | SEQ ID NO:8673 | SEQ ID NO:16685 |
| | | AA | RASQSIISYLN | VASSLQS | QQSYSTPYT |
| | | | SEQ ID NO:662 | SEQ ID NO:8674 | SEQ ID NO:16686 |
| iPS:393124 | | NA | CGGGCAAGTCAGGGCATTAG AAATGATTTAGGC | GCTGCATCCAGTTTGCAA AGT | CTACAGCATTATAGTTACCC TCCTACT |
| | 21-225_33G7 | | SEQ ID NO:663 | SEQ ID NO:8675 | SEQ ID NO:16687 |
| | | AA | RASQGIRNDLG | AASSLQS | LQHYSYPPT |
| | | | SEQ ID NO:664 | SEQ ID NO:8676 | SEQ ID NO:16688 |
| iPS:393126 | | NA | CGGGCAAGTCAGGACATTAG AAGTGATTTAGGC | GCTGCATCCAGTTTGCAA AGT | CTACAGCATACTATTTACCC TCCCACT |
| | 21-225_35D1 | | SEQ ID NO:665 | SEQ ID NO:8677 | SEQ ID NO:16689 |
| | | AA | RASQDIRSDLG | AASSLQS | LQHTIYPPT |
| | | | SEQ ID NO:666 | SEQ ID NO:8678 | SEQ ID NO:16690 |
| iPS:393128 | | NA | CGGGCAAGTCAGGACATTAG AAGTGATTTAGGC | GCTGCATCCAGTTTGCAA AGT | CTACAGCATACTGTTTACCC TCCTACT |
| | 21-225_35F11 | | SEQ ID NO:667 | SEQ ID NO:8679 | SEQ ID NO:16691 |
| | | AA | RASQDIRSDLG | AASSLQS | LQHTVYPPT |
| | | | SEQ ID NO:668 | SEQ ID NO:8680 | SEQ ID NO:16692 |
| iPS:393130 | | NA | CGGGCAAGTCAGGGCATTAG AAATGATTTAGGC | GCTGCACCCAGTTTGCA AAGT | CTACAGCATAATAGTTACCC GTGGACG |
| | 21-225_33C2 | | SEQ ID NO:669 | SEQ ID NO:8681 | SEQ ID NO:16693 |
| | | AA | RASQGIRNDLG | AAPSLQS | LQHNSYPWT |
| | | | SEQ ID NO:670 | SEQ ID NO:8682 | SEQ ID NO:16694 |
| iPS:393132 | | NA | CGGGCGAGTCAGGGTATTAG CCGGTGGTTAGCC | GCTGCATCCAGGTTGCA AAGT | CAACAGGCTAACATTTTCCC GTTCACT |
| | 21-225_33H7 | | SEQ ID NO:671 | SEQ ID NO:8683 | SEQ ID NO:16695 |
| | | AA | RASQGISRWLA | AASRLQS | QQANIFPFT |
| | | | SEQ ID NO:672 | SEQ ID NO:8684 | SEQ ID NO:16696 |

FIGURE 49

| iPS:393134 | | NA | CGGGCAAGTCAGAGAATTAT CAGCTATTTAAAT | GTTGCATCCAGTTTGCAA AGT | CAACAGAGTTACAGTACCCC GTACACT |
|---|---|---|---|---|---|
| | 21-225_34C2 | | SEQ ID NO:673 | SEQ ID NO:8685 | SEQ ID NO:16697 |
| | | AA | RASQRIISYLN | VASSLQS | QQSYSTPYT |
| | | | SEQ ID NO:674 | SEQ ID NO:8686 | SEQ ID NO:16698 |
| iPS:393136 | | NA | CGGGCAAGTCAGATCATTAT CAGCTATTTAAAT | GTTGCATCCAGTTTGCAA AGT | CAACAGAGTTACAGTACCCC GTACACT |
| | 21-225_34D8 | | SEQ ID NO:675 | SEQ ID NO:8687 | SEQ ID NO:16699 |
| | | AA | RASQIIISYLN | VASSLQS | QQSYSTPYT |
| | | | SEQ ID NO:676 | SEQ ID NO:8688 | SEQ ID NO:16700 |
| iPS:393138 | | NA | CAGGCGAGTCAGGACATTTT CAACTATTTAAAT | GATGCCTCCAATTTGGA AACA | CAACAGTATGATAATCTCCC GATCACC |
| | 21-225_35E3 | | SEQ ID NO:677 | SEQ ID NO:8689 | SEQ ID NO:16701 |
| | | AA | QASQDIFNYLN | DASNLET | QQYDNLPIT |
| | | | SEQ ID NO:678 | SEQ ID NO:8690 | SEQ ID NO:16702 |
| iPS:393140 | | NA | CGGGCGAGTCAGGGTATTAG CAGATGGTTAGCC | GCTGCATCCCGTTTGCAA AGT | CAACAGGCTAACAGTTTCCC ATTCACT |
| | 21-225_35H12 | | SEQ ID NO:679 | SEQ ID NO:8691 | SEQ ID NO:16703 |
| | | AA | RASQGISRWLA | AASRLQS | QQANSFPFT |
| | | | SEQ ID NO:680 | SEQ ID NO:8692 | SEQ ID NO:16704 |
| iPS:393142 | | NA | CGGGCGAGTCAGGGCATTAA CAATTATTTAGCC | GCTGCATCCAGTTTGCAA AGT | CAACAGTTTAATAGTTACCC TCCGACG |
| | 21-225_33A3 | | SEQ ID NO:681 | SEQ ID NO:8693 | SEQ ID NO:16705 |
| | | AA | RASQGINNYLA | AASSLQS | QQFNSYPPT |
| | | | SEQ ID NO:682 | SEQ ID NO:8694 | SEQ ID NO:16706 |
| iPS:393144 | | NA | AAGTCTAGTCAGAGCCTCCT GCATAGTGATGGAAAGACCT ATTTGTAT | GAAGTTTCCAACCGGTTC TCT | ATGCAAAGTAAACAGCTTCC TCCT |
| | 21-225_34D2 | | SEQ ID NO:683 | SEQ ID NO:8695 | SEQ ID NO:16707 |
| | | AA | KSSQSLLHSDGKTYLY | EVSNRFS | MQSKQLPP |

FIGURE 49

| | | | SEQ ID NO:684 | SEQ ID NO:8696 | SEQ ID NO:16708 |
|---|---|---|---|---|---|
| iPS:393146 | | NA | CGGGCAAGTCAGGACATTAG AAGTGATTTAGGC | GCTGCATCCAGTTTGCAA AGT | CTACAGCATACTATTTACCC TCCTACT |
| | 21-225_34G8 | | SEQ ID NO:685 | SEQ ID NO:8697 | SEQ ID NO:16709 |
| | | AA | RASQDIRSDLG | AASSLQS | LQHTIYPPT |
| | | | SEQ ID NO:686 | SEQ ID NO:8698 | SEQ ID NO:16710 |
| iPS:393148 | | NA | CGGGCAAGTCAGAGCATTAG CAGCTATTTAAAT | GGTGCATCCAGTTTCCAA AGT | CACCAGAGTTACAATCTCCC GATCACC |
| | 21-225_35E5 | | SEQ ID NO:687 | SEQ ID NO:8699 | SEQ ID NO:16711 |
| | | AA | RASQSISSYLN | GASSFQS | HQSYNLPIT |
| | | | SEQ ID NO:688 | SEQ ID NO:8700 | SEQ ID NO:16712 |
| iPS:393150 | | NA | CGGACAAGTCAGGACATTAG AAATGATTTAGGC | GCTGCATCCAGTTTGCAA AGT | CTACACCACAATAGTTACCC TCCTAAG |
| | 21-225_36A5 | | SEQ ID NO:689 | SEQ ID NO:8701 | SEQ ID NO:16713 |
| | | AA | RTSQDIRNDLG | AASSLQS | LHHNSYPPK |
| | | | SEQ ID NO:690 | SEQ ID NO:8702 | SEQ ID NO:16714 |
| iPS:393152 | | NA | CGGGCGAGTCAGGGTATTAG CAGCTGGTTAGCC | GGTGCATCCAGTTTGCA AAGT | CAACAGTCTGACAGTTTCCC TCGGACG |
| | 21-225_25B3 | | SEQ ID NO:691 | SEQ ID NO:8703 | SEQ ID NO:16715 |
| | | AA | RASQGISSWLA | GASSLQS | QQSDSFPRT |
| | | | SEQ ID NO:692 | SEQ ID NO:8704 | SEQ ID NO:16716 |
| iPS:393166 | | NA | TCTGGAGATAAATTGGGGGA TAAATATGCTTGC | CAAGATAGGAAGCGGCC CTCA | CAGGCGTGGGACAGCAGCTC TTATGTGGTA |
| | 21-225_27G6 | | SEQ ID NO:693 | SEQ ID NO:8705 | SEQ ID NO:16717 |
| | | AA | SGDKLGDKYAC | QDRKRPS | QAWDSSSYVV |
| | | | SEQ ID NO:694 | SEQ ID NO:8706 | SEQ ID NO:16718 |
| iPS:393168 | | NA | TCTGGAGATAAATTGGGGGA TAAATATGCTTAC | CAAGATAGTAAGCGGTC CTCA | CAGGCGTGGGACAACAGCAC TGTGGTA |
| | 21-225_32B11 | | SEQ ID NO:695 | SEQ ID NO:8707 | SEQ ID NO:16719 |
| | | AA | SGDKLGDKYAY | QDSKRSS | QAWDNSTVV |

FIGURE 49

| | | | SEQ ID NO:696 | SEQ ID NO:8708 | SEQ ID NO:16720 |
|---|---|---|---|---|---|
| iPS:393172 | | NA | TCTGGAGATAAATTGGGGGA AAAATATGCTTGC | CAAGATAGGAAGCGGCC CTCA | CAGGCGTGGGTCAACAACAC TATGATA |
| | 21-225_3B12 | | SEQ ID NO:697 | SEQ ID NO:8709 | SEQ ID NO:16721 |
| | | AA | SGDKLGEKYAC | QDRKRPS | QAWVNNTMI |
| | | | SEQ ID NO:698 | SEQ ID NO:8710 | SEQ ID NO:16722 |
| iPS:393174 | | NA | ACCCTAAGCAGTGAGCACAG CACCTACACCATCGAA | GTTAAGAGTGATGGCAG CCACAGCAAGGGGGAC | GGAGAGAGCCACACGATCGA TGGCCAAGTCGGTGTGGTA |
| | 21-225_15D8 | | SEQ ID NO:699 | SEQ ID NO:8711 | SEQ ID NO:16723 |
| | | AA | TLSSEHSTYTIE | VKSDGSHSKGD | GESHTIDGQVGVV |
| | | | SEQ ID NO:700 | SEQ ID NO:8712 | SEQ ID NO:16724 |
| iPS:393176 | | NA | TCTGGAGATAAATTGGGGAA TAAATATGCTTGC | CAAGATAGCAAGCGGCC CTTA | CAGGCGTGGGACAGTAGTAC TGTGGTA |
| | 21-225_27E7 | | SEQ ID NO:701 | SEQ ID NO:8713 | SEQ ID NO:16725 |
| | | AA | SGDKLGNKYAC | QDSKRPL | QAWDSSTVV |
| | | | SEQ ID NO:702 | SEQ ID NO:8714 | SEQ ID NO:16726 |
| iPS:393178 | | NA | TCTGGAGATAAATTGGGGGA GAAATATGCTTAC | CAAGATAGCAAGCGGCC CTCA | CAGGCGTGGGACAACACCAC TGTGGTA |
| | 21-225_34D7 | | SEQ ID NO:703 | SEQ ID NO:8715 | SEQ ID NO:16727 |
| | | AA | SGDKLGEKYAY | QDSKRPS | QAWDNTTVV |
| | | | SEQ ID NO:704 | SEQ ID NO:8716 | SEQ ID NO:16728 |
| iPS:393180 | | NA | TCTGGAACCAACTCCAACAT CGGAAGTTATACTGTAAAC | ATTAATAATCAGCGGCC CTCA | GCAGCATGGGATGACAGCCT GAATGGTCATGTGGTA |
| | 21-225_4G12 | | SEQ ID NO:705 | SEQ ID NO:8717 | SEQ ID NO:16729 |
| | | AA | SGTNSNIGSYTVN | INNQRPS | AAWDDSLNGHVV |
| | | | SEQ ID NO:706 | SEQ ID NO:8718 | SEQ ID NO:16730 |
| iPS:393182 | | NA | TCTGGAGATAAATTGGGGGA TAAATATGCTTGC | CAAGATCGCAAGCGGCC CTCA | CAGGCGTGGGACAACAACAC TGTGATA |

FIGURE 49

| | 21-225_4B3 | | SEQ ID NO:707 | SEQ ID NO:8719 | SEQ ID NO:16731 |
|---|---|---|---|---|---|
| | | AA | SGDKLGDKYAC | QDRKRPS | QAWDNNTVI |
| iPS:393184 | | NA | SEQ ID NO:708 | SEQ ID NO:8720 | SEQ ID NO:16732 |
| | | NA | TCTGGAGATAAATTGGGGGA GAAATATGCTTGC | CAAGATAGGAAGCGGCC CTCA | CAGGCGTGGGACAGCAGCAC TGCGGTA |
| | 21-225_15H11 | | SEQ ID NO:709 | SEQ ID NO:8721 | SEQ ID NO:16733 |
| | | AA | SGDKLGEKYAC | QDRKRPS | QAWDSSTAV |
| iPS:393186 | | NA | SEQ ID NO:710 | SEQ ID NO:8722 | SEQ ID NO:16734 |
| | | NA | TCTGGATATAAATTGGGGGA TAAATATGCTTGC | CAAGATAGCAAGCGGCC CTCA | CAGGCGTGGGTCAACAACAC TGTA |
| | 21-225_27D9 | | SEQ ID NO:711 | SEQ ID NO:8723 | SEQ ID NO:16735 |
| | | AA | SGYKLGDKYAC | QDSKRPS | QAWVNNTV |
| iPS:393188 | | NA | SEQ ID NO:712 | SEQ ID NO:8724 | SEQ ID NO:16736 |
| | | NA | TCTGGAGATAAATTGGGGGA GAAATATGTTTCC | CAAGATAGTAAGCGGCC CTCA | CAGGCGTGGGACAGCAGCAC TGTA |
| | 21-225_34B9 | | SEQ ID NO:713 | SEQ ID NO:8725 | SEQ ID NO:16737 |
| | | AA | SGDKLGEKYVS | QDSKRPS | QAWDSSTV |
| iPS:393192 | | NA | SEQ ID NO:714 | SEQ ID NO:8726 | SEQ ID NO:16738 |
| | | NA | TCTGGAGATAAATTGGGGGA TAAATATGCTTGC | CAAGATCGCAAGCGGCC CTCA | CAGGCGTGGGACAACAACAC TGTGATA |
| | 21-225_12B1 | | SEQ ID NO:715 | SEQ ID NO:8727 | SEQ ID NO:16739 |
| | | AA | SGDKLGDKYAC | QDRKRPS | QAWDNNTVI |
| iPS:393194 | | NA | SEQ ID NO:716 | SEQ ID NO:8728 | SEQ ID NO:16740 |
| | | NA | TCTGGAGATAAATTGGGGGA TAAATATGCTTGC | CAAGATAGCAAGCGGCC CTCA | CAGGCGTGGGACAGCAGCAC TTATGTGGTA |
| | 21-225_16D2 | | SEQ ID NO:717 | SEQ ID NO:8729 | SEQ ID NO:16741 |
| | | AA | SGDKLGDKYAC | QDSKRPS | QAWDSSTYVV |
| iPS:393196 | | NA | SEQ ID NO:718 | SEQ ID NO:8730 | SEQ ID NO:16742 |
| | | NA | TCTGGAGATAAATTGGGGGA AAAATATGCTTGC | CAAGATAGGAAGCGGCC CTCA | CAGGCGTGGGTCAATAACAC TATGATA |

FIGURE 49

| | 21-225_16G8 | | SEQ ID NO:719 | SEQ ID NO:8731 | SEQ ID NO:16743 |
|---|---|---|---|---|---|
| | | AA | SGDKLGEKYAC | QDRKRPS | QAWVNNTMI |
| | | | SEQ ID NO:720 | SEQ ID NO:8732 | SEQ ID NO:16744 |
| iPS:393198 | | NA | TCTGGAGATAAATTGGGGGA TAAATATGCTTGC | CAAGATAGCAAGCGGCC CTCA | CAGGCGTGGGACAGTAGCAC TTATGTGGTA |
| | 21-225_28A11 | | SEQ ID NO:721 | SEQ ID NO:8733 | SEQ ID NO:16745 |
| | | AA | SGDKLGDKYAC | QDSKRPS | QAWDSSTYVV |
| | | | SEQ ID NO:722 | SEQ ID NO:8734 | SEQ ID NO:16746 |
| iPS:393200 | | NA | TCTGGAGATAAATTGGGGGA AAAATATGCTTAC | CAAGATAGGAAGCGGCC CTCA | CAGGCGTGGGACAACAGCAC TGCGGTA |
| | 21-225_35E1 | | SEQ ID NO:723 | SEQ ID NO:8735 | SEQ ID NO:16747 |
| | | AA | SGDKLGEKYAY | QDRKRPS | QAWDNSTAV |
| | | | SEQ ID NO:724 | SEQ ID NO:8736 | SEQ ID NO:16748 |
| iPS:393202 | | NA | TCTGGAGATAAATTGGGGGA TAAATATGCTTGC | CAAGATCGCAAGCGGCC CTCA | CAGGCGTGGGACAACAACAC TGTGATA |
| | 21-225_6B4 | | SEQ ID NO:725 | SEQ ID NO:8737 | SEQ ID NO:16749 |
| | | AA | SGDKLGDKYAC | QDRKRPS | QAWDNNTVI |
| | | | SEQ ID NO:726 | SEQ ID NO:8738 | SEQ ID NO:16750 |
| iPS:393204 | | NA | GGGGGAAACAACATTGGAA GTAAAGCTGTGCAC | AGCGATAGCAACCGGCC CTCA | CAGGTGTGGGACAGTAGTAG TGATCATGTGGTA |
| | 21-225_8C12 | | SEQ ID NO:727 | SEQ ID NO:8739 | SEQ ID NO:16751 |
| | | AA | GGNNIGSKAVH | SDSNRPS | QVWDSSSDHVV |
| | | | SEQ ID NO:728 | SEQ ID NO:8740 | SEQ ID NO:16752 |
| iPS:393206 | | NA | TCTGGAGATAAATTGGGGGA TAAATATGCTTGC | CAAGATAGCAAGCGGCC CTCA | CAGGCGTGGGGGCAACAGCAC TGCTGTGGTA |
| | 21-225_13F6 | | SEQ ID NO:729 | SEQ ID NO:8741 | SEQ ID NO:16753 |
| | | AA | SGDKLGDKYAC | QDSKRPS | QAWGNSTAVV |
| | | | SEQ ID NO:730 | SEQ ID NO:8742 | SEQ ID NO:16754 |
| iPS:393208 | | NA | GGGGGAAACAACATTGGAA GTAAAAGTGTGCAC | GATGATACCGACCGGCC CTCA | CAGGTGTGGGATAGTAGCAG TGATCATGTGGTA |

FIGURE 49

| | 21-225_16F3 | | SEQ ID NO:731 | SEQ ID NO:8743 | SEQ ID NO:16755 |
|---|---|---|---|---|---|
| | | AA | GGNNIGSKSVH | DDTDRPS | QVWDSSSDHVV |
| iPS:393210 | | NA | SEQ ID NO:732 | SEQ ID NO:8744 | SEQ ID NO:16756 |
| | | | TCTGGAGATAAATTGGGGGATAAATATGTTTAC | CAAGATAGGAAGCGGCCCTCA | CAGGCGTGGGACAGCATCACTGCAGTA |
| | 21-225_17D3 | | SEQ ID NO:733 | SEQ ID NO:8745 | SEQ ID NO:16757 |
| | | AA | SGDKLGDKYVY | QDRKRPS | QAWDSITAV |
| iPS:393212 | | NA | SEQ ID NO:734 | SEQ ID NO:8746 | SEQ ID NO:16758 |
| | | | TCTGGAGATAAATTGGGTAATAAATATGCTTGC | CAAGATAGCAAGCGGCCCTCA | CAGGCGTGGGACAGCAGCACTGTT |
| | 21-225_30H6 | | SEQ ID NO:735 | SEQ ID NO:8747 | SEQ ID NO:16759 |
| | | AA | SGDKLGNKYAC | QDSKRPS | QAWDSSTV |
| iPS:393214 | | NA | SEQ ID NO:736 | SEQ ID NO:8748 | SEQ ID NO:16760 |
| | | | TCTGGAGATAAATTGGGGGATAAATTTGTTTAT | CAAGATAGCAAGCGGCCCTCA | CAGGCGTGGGACAGCACCACCGTGGTA |
| | 21-225_33A1 | | SEQ ID NO:737 | SEQ ID NO:8749 | SEQ ID NO:16761 |
| | | AA | SGDKLGDKFVY | QDSKRPS | QAWDSTTVV |
| iPS:393218 | | NA | SEQ ID NO:738 | SEQ ID NO:8750 | SEQ ID NO:16762 |
| | | | TCTGGAGATAAATTGGGGGATAAATATGTTTGC | CAAGATCGCAAGCGGCCCTCA | CAGGCGTGGGGCAACAGCACTGCTGTGGTA |
| | 21-225_14G3 | | SEQ ID NO:739 | SEQ ID NO:8751 | SEQ ID NO:16763 |
| | | AA | SGDKLGDKYVC | QDRKRPS | QAWGNSTAVV |
| iPS:393222 | | NA | SEQ ID NO:740 | SEQ ID NO:8752 | SEQ ID NO:16764 |
| | | | TCTGGAGATAAATTGGGGGAAAAATATGCTTGC | CAAGATAGAAAGCGGCCCTCA | CAGGCGTGGGACAGCAGCACGGTA |
| | 21-225_17F5 | | SEQ ID NO:741 | SEQ ID NO:8753 | SEQ ID NO:16765 |
| | | AA | SGDKLGEKYAC | QDRKRPS | QAWDSSTV |
| iPS:393224 | | NA | SEQ ID NO:742 | SEQ ID NO:8754 | SEQ ID NO:16766 |
| | | | TCTGGAGATAAATTGGGAAATAAATATGCTTGC | CAAGATTCCAAGCGGCCCTCA | CAGGCGTGGGACAGCAGCACTGTA |

FIGURE 49

| | 21-225_31C2 | | SEQ ID NO:743 | SEQ ID NO:8755 | SEQ ID NO:16767 |
|---|---|---|---|---|---|
| | | AA | SGDKLGNKYAC | QDSKRPS | QAWDSSTV |
| | | | SEQ ID NO:744 | SEQ ID NO:8756 | SEQ ID NO:16768 |
| iPS:393226 | | NA | TCTGGAGATAAATTGGGGGATAAATATGCTTAC | CAAGATAGGAAGCGGCCCTCA | CAGGCGTGGGACAACAGCACTGCGGTA |
| | 21-225_33E6 | | SEQ ID NO:745 | SEQ ID NO:8757 | SEQ ID NO:16769 |
| | | AA | SGDKLGDKYAY | QDRKRPS | QAWDNSTAV |
| | | | SEQ ID NO:746 | SEQ ID NO:8758 | SEQ ID NO:16770 |
| iPS:393230 | | NA | TCTGGAACCAACTCCAACATCGGAAGTTATACTGTAAAC | ATTAATAATCAGCGGCCCTCA | GCAGCATGGGATGACAGCCTGAATGGTCATGTGGTA |
| | 21-225_9G9 | | SEQ ID NO:747 | SEQ ID NO:8759 | SEQ ID NO:16771 |
| | | AA | SGTNSNIGSYTVN | INNQRPS | AAWDDSLNGHVV |
| | | | SEQ ID NO:748 | SEQ ID NO:8760 | SEQ ID NO:16772 |
| iPS:393232 | | NA | ACTGGAGCCAGCAGTGACGTTGGTGATTATAACTCTGTCTCC | GAGGTCAGTAATCGGCCCTCA | AGCTCATATACAAGCAGCATCACTGTGGTA |
| | 21-225_17F12 | | SEQ ID NO:749 | SEQ ID NO:8761 | SEQ ID NO:16773 |
| | | AA | TGASSDVGDYNSVS | EVSNRPS | SSYTSSITVV |
| | | | SEQ ID NO:750 | SEQ ID NO:8762 | SEQ ID NO:16774 |
| iPS:393234 | | NA | TCTGGAGATAAATTGGGGGATAAATATGTTTGC | CAAGATAGCAAGCGGCCCTCA | CAGGCGTGGGTCAACAACACTGTA |
| | 21-225_26C10 | | SEQ ID NO:751 | SEQ ID NO:8763 | SEQ ID NO:16775 |
| | | AA | SGDKLGDKYVC | QDSKRPS | QAWVNNTV |
| | | | SEQ ID NO:752 | SEQ ID NO:8764 | SEQ ID NO:16776 |
| iPS:393345 | | NA | TCTGGAGATAAATTGGGGAATAAATATGCTTGG | CAAGATAGGAAGCGGCCCTCA | CAGGCGTGGGACAACAGCACTGTGGTT |
| | 21-225_5G7 | | SEQ ID NO:753 | SEQ ID NO:8765 | SEQ ID NO:16777 |
| | | AA | SGDKLGNKYAW | QDRKRPS | QAWDNSTVV |
| | | | SEQ ID NO:754 | SEQ ID NO:8766 | SEQ ID NO:16778 |

FIGURE 49

| iPS:393368 | 21-225_29H8 | NA | AGGTCCAGCCAGACTATTTT ACACAGCTCCAACAATTACA ACTACTTAGCT | TGGGCATCTACCCGGGA ATCC | CAGCAATATTATTGTACTCC TCCGACG |
|---|---|---|---|---|---|
| | | | SEQ ID NO:755 | SEQ ID NO:8767 | SEQ ID NO:16779 |
| | | AA | RSSQTILHSSNNYNYLA | WASTRES | QQYYCTPPT |
| | | | SEQ ID NO:756 | SEQ ID NO:8768 | SEQ ID NO:16780 |
| iPS:393565 | 21-225_34B11 | NA | TCTGGAGATAAATTGGGGGA TAAATATGCTTGC | CAAGATATGAAGCGGCC CTCA | CAGGCGTGGGACAACAGCAC TGCGGTA |
| | | | SEQ ID NO:757 | SEQ ID NO:8769 | SEQ ID NO:16781 |
| | | AA | SGDKLGDKYAC | QDMKRPS | QAWDNSTAV |
| | | | SEQ ID NO:758 | SEQ ID NO:8770 | SEQ ID NO:16782 |
| iPS:393802 | 21-225_3D12 | NA | AGGGCCAGTCAGAGTGTTAG CAGCAGCTACTTAGCC | GGTACATCCAGCAGGGC CACT | CAGCAGTATGGTAGTTCACG CAGT |
| | | | SEQ ID NO:759 | SEQ ID NO:8771 | SEQ ID NO:16783 |
| | | AA | RASQSVSSSYLA | GTSSRAT | QQYGSSRS |
| | | | SEQ ID NO:760 | SEQ ID NO:8772 | SEQ ID NO:16784 |
| iPS:393804 | 21-225_5H7 | NA | CGGGCAAGTCAGGGCATTAG AAATGATTTAGGC | GTTACATCCAGTTTGCAA GGT | CTACAACATAATAGTTACCC GCTCACT |
| | | | SEQ ID NO:761 | SEQ ID NO:8773 | SEQ ID NO:16785 |
| | | AA | RASQGIRNDLG | VTSSLQG | LQHNSYPLT |
| | | | SEQ ID NO:762 | SEQ ID NO:8774 | SEQ ID NO:16786 |
| iPS:393806 | 21-225_6A6 | NA | CGGGCAAGTCAGGGCATTAG AAATGATTTAGGC | GCTGCATCCAGTTTGCAA AGT | CTACAGCATAGTAGTTACCC GCTCACT |
| | | | SEQ ID NO:763 | SEQ ID NO:8775 | SEQ ID NO:16787 |
| | | AA | RASQGIRNDLG | AASSLQS | LQHSSYPLT |
| | | | SEQ ID NO:764 | SEQ ID NO:8776 | SEQ ID NO:16788 |
| iPS:393808 | 21-225_1A2 | NA | CGGGCAAGTCAGGGCATTAG AAATGATTTAGGC | GCTGCATCCAGTTTGCAA AGT | CTACAGCATAATAGTCACCC TCTCACT |
| | | | SEQ ID NO:765 | SEQ ID NO:8777 | SEQ ID NO:16789 |
| | | AA | RASQGIRNDLG | AASSLQS | LQHNSHPLT |

FIGURE 49

| | | | SEQ ID NO:766 | SEQ ID NO:8778 | SEQ ID NO:16790 |
|---|---|---|---|---|---|
| iPS:393810 | | NA | CGGGCGAGTCAGGGTATTAG CACCTGGTTAGCC | GATGCATCCAGTTTGCA AAGT | CAACAGGCTAACAGTTTCCC GTGGACG |
| | 21-225_5A4 | | SEQ ID NO:767 | SEQ ID NO:8779 | SEQ ID NO:16791 |
| | | AA | RASQGISTWLA | DASSLQS | QQANSFPWT |
| | | | SEQ ID NO:768 | SEQ ID NO:8780 | SEQ ID NO:16792 |
| iPS:393812 | | NA | CGGGCAAGTCAGGGCATTAG AAATGATTTAGGC | GCTGCATCCAGTTTGCAA AGT | CTACAGCATAATAGTTACCC GTGGACG |
| | 21-225_6A11 | | SEQ ID NO:769 | SEQ ID NO:8781 | SEQ ID NO:16793 |
| | | AA | RASQGIRNDLG | AASSLQS | LQHNSYPWT |
| | | | SEQ ID NO:770 | SEQ ID NO:8782 | SEQ ID NO:16794 |
| iPS:393814 | | NA | CGGACAAGTCAGGGCATTAG AAATGATTTAGGC | GCTGCATCCAATTTGCAA AGT | CTACAGCATAGTGCTTACCC GCTCACT |
| | 21-225_7F4 | | SEQ ID NO:771 | SEQ ID NO:8783 | SEQ ID NO:16795 |
| | | AA | RTSQGIRNDLG | AASNLQS | LQHSAYPLT |
| | | | SEQ ID NO:772 | SEQ ID NO:8784 | SEQ ID NO:16796 |
| iPS:393816 | | NA | CGGGCAAGTCAGGCCATTAG AAATGATTTAGGC | GCTGCATCCAGTTTGCAA AGT | CTACAGCATAGTAGTTACCC GCTCACT |
| | 21-225_6D4 | | SEQ ID NO:773 | SEQ ID NO:8785 | SEQ ID NO:16797 |
| | | AA | RASQAIRNDLG | AASSLQS | LQHSSYPLT |
| | | | SEQ ID NO:774 | SEQ ID NO:8786 | SEQ ID NO:16798 |
| iPS:393818 | | NA | CGGGCAAGTCAGGGCATTAG AAATGATTTAGGC | GCTGCATCCACTTTGCAA AGT | CTACAGCATAATAGTTACCC GCTCACT |
| | 21-225_6G12 | | SEQ ID NO:775 | SEQ ID NO:8787 | SEQ ID NO:16799 |
| | | AA | RASQGIRNDLG | AASTLQS | LQHNSYPLT |
| | | | SEQ ID NO:776 | SEQ ID NO:8788 | SEQ ID NO:16800 |
| iPS:393820 | | NA | CGGGCAAGTCAGGGCATTAG AAATGATTTAGGC | GCTGCATCCAGTTTGCAA AGT | CTACAGCATAATAGTTACCC GTTCACT |
| | 21-225_8H7 | | SEQ ID NO:777 | SEQ ID NO:8789 | SEQ ID NO:16801 |
| | | AA | RASQGIRNDLG | AASSLQS | LQHNSYPFT |

FIGURE 49

| | | | SEQ ID NO:778 | SEQ ID NO:8790 | SEQ ID NO:16802 |
|---|---|---|---|---|---|
| iPS:393822 | | NA | CGGGCAAGTCAGGGCATTAG AAATGATTTAGGC | GCTGCATCCAGTTTGCAA AGT | CTACAGCATAATAGTTACCC ATTCACT |
| | 21-225_15B11 | | SEQ ID NO:779 | SEQ ID NO:8791 | SEQ ID NO:16803 |
| | | AA | RASQGIRNDLG | AASSLQS | LQHNSYPFT |
| | | | SEQ ID NO:780 | SEQ ID NO:8792 | SEQ ID NO:16804 |
| iPS:393824 | | NA | CGGGCAAGTCAGAACATTAG TAGTTATTTAAAT | GCTGCATCCAGTTTGCAA AGT | CAACAGAGTTACAATACCCC CTTCTTCACT |
| | 21-225_10F12 | | SEQ ID NO:781 | SEQ ID NO:8793 | SEQ ID NO:16805 |
| | | AA | RASQNISSYLN | AASSLQS | QQSYNTPFFT |
| | | | SEQ ID NO:782 | SEQ ID NO:8794 | SEQ ID NO:16806 |
| iPS:393826 | | NA | CGGGCAAGTCAGGGCATTAG AAATGATTTAGGC | GCTGCATCCAGTTTGCAA AGT | CTACAGCATAATAGTTACCC GCTCACT |
| | 21-225_10G5 | | SEQ ID NO:783 | SEQ ID NO:8795 | SEQ ID NO:16807 |
| | | AA | RASQGIRNDLG | AASSLQS | LQHNSYPLT |
| | | | SEQ ID NO:784 | SEQ ID NO:8796 | SEQ ID NO:16808 |
| iPS:393828 | | NA | CGGGCAAGTCAGGGCATTAG AAATGATTTAGGC | GGTGCATCCAGTTTGCA AAGT | CTACAGCATAATAGTTACCC GCTCACT |
| | 21-225_10H12 | | SEQ ID NO:785 | SEQ ID NO:8797 | SEQ ID NO:16809 |
| | | AA | RASQGIRNDLG | GASSLQS | LQHNSYPLT |
| | | | SEQ ID NO:786 | SEQ ID NO:8798 | SEQ ID NO:16810 |
| iPS:393830 | | NA | CGGGCAAGTCAGGGCATTAG AAATGATTTAGGC | GCTGCATCCAGTTTGCAA AGT | CTACAGCATAATAGTTACCC GCTCACT |
| | 21-225_12A1 | | SEQ ID NO:787 | SEQ ID NO:8799 | SEQ ID NO:16811 |
| | | AA | RASQGIRNDLG | AASSLQS | LQHNSYPLT |
| | | | SEQ ID NO:788 | SEQ ID NO:8800 | SEQ ID NO:16812 |
| iPS:393832 | | NA | CGGGCAAGTCAGGGCATTAG AAATGATTTAGGC | GTTACATCCAGTTTGCAA GGT | CTACAACATAATAGTTACCC GCTCACT |
| | 21-225_14B2 | | SEQ ID NO:789 | SEQ ID NO:8801 | SEQ ID NO:16813 |
| | | AA | RASQGIRNDLG | VTSSLQG | LQHNSYPLT |

FIGURE 49

| | | | SEQ ID NO:790 | SEQ ID NO:8802 | SEQ ID NO:16814 |
|---|---|---|---|---|---|
| iPS:393836 | | NA | CGGGCGAGTCAGGGCATTAGCAATTATTTAGCC | GCTGCATCCAGTTTGCAAAGT | CAACAGTATTATAGTTACCCATTCACT |
| | 21-225_15A2 | | SEQ ID NO:791 | SEQ ID NO:8803 | SEQ ID NO:16815 |
| | | AA | RASQGISNYLA | AASSLQS | QQYYSYPFT |
| | | | SEQ ID NO:792 | SEQ ID NO:8804 | SEQ ID NO:16816 |
| iPS:393838 | | NA | CGGGCAAGTCAGGGCATTAGAAATGATTTAGGC | GCTGCATCCAGTCTGCAAAGT | ATACAGCATAATAGTTACCTGTGGACG |
| | 21-225_6G2 | | SEQ ID NO:793 | SEQ ID NO:8805 | SEQ ID NO:16817 |
| | | AA | RASQGIRNDLG | AASSLQS | IQHNSYLWT |
| | | | SEQ ID NO:794 | SEQ ID NO:8806 | SEQ ID NO:16818 |
| iPS:393840 | | NA | CGGGCAAGTCAGAGTATTCTCAGCTATTTAAAT | ACTACATCCAGTTTGCAAAGT | CAACAGACTTACAGTACCCCGCTCACT |
| | 21-225_3F8 | | SEQ ID NO:795 | SEQ ID NO:8807 | SEQ ID NO:16819 |
| | | AA | RASQSILSYLN | TTSSLQS | QQTYSTPLT |
| | | | SEQ ID NO:796 | SEQ ID NO:8808 | SEQ ID NO:16820 |
| iPS:393844 | | NA | CGGGCAAGTCAGAACATTTACAGGTATTTAAAT | GCTGCATCCAGTTCGCAAAGT | CAACAGAGTTACAGTCCCCCTTTCACT |
| | 21-225_3G7 | | SEQ ID NO:797 | SEQ ID NO:8809 | SEQ ID NO:16821 |
| | | AA | RASQNIYRYLN | AASSSQS | QQSYSPPFT |
| | | | SEQ ID NO:798 | SEQ ID NO:8810 | SEQ ID NO:16822 |
| iPS:393848 | | NA | CGGGCAATTCAGAACATTAGCAGCTATTTAAAT | GCTGCATCCAGCTTGCAAAGT | CAACAGAGTTACAGAACCCCCTTATTCACT |
| | 21-225_4H2 | | SEQ ID NO:799 | SEQ ID NO:8811 | SEQ ID NO:16823 |
| | | AA | RAIQNISSYLN | AASSLQS | QQSYRTPLFT |
| | | | SEQ ID NO:800 | SEQ ID NO:8812 | SEQ ID NO:16824 |
| iPS:393852 | | NA | CGGGCAAGTCAGAACATTTACAGCTATTTAAAT | ACTGCATCCAGTTTGCAAAGT | CAGCAGAGTTACAGTCCCCCTCTCACT |
| | 21-225_12A10 | | SEQ ID NO:801 | SEQ ID NO:8813 | SEQ ID NO:16825 |
| | | AA | RASQNIYSYLN | TASSLQS | QQSYSPPLT |

FIGURE 49

| | | | | SEQ ID NO:802 | SEQ ID NO:8814 | SEQ ID NO:16826 |
|---|---|---|---|---|---|---|
| iPS:393854 | | NA | | CTGGCAAGTCAGGGCATTAG AAATGATTTAGGC | GTTGCATGTAGTTTCCAA AGT | CTACAACATAATCTTTACCC GCTCACT |
| | 21-225_7H11 | | | SEQ ID NO:803 | SEQ ID NO:8815 | SEQ ID NO:16827 |
| | | AA | | LASQGIRNDLG | VACSFQS | LQHNLYPLT |
| | | | | SEQ ID NO:804 | SEQ ID NO:8816 | SEQ ID NO:16828 |
| iPS:393856 | | NA | | CGGGCAAGTCAGGGCATTAG AAATGATTTAGAC | GCTGCATCCAGTTTGCAA AGT | GTACAGCATAATAGTTACCC GCTCACT |
| | 21-225_14C2 | | | SEQ ID NO:805 | SEQ ID NO:8817 | SEQ ID NO:16829 |
| | | AA | | RASQGIRNDLD | AASSLQS | VQHNSYPLT |
| | | | | SEQ ID NO:806 | SEQ ID NO:8818 | SEQ ID NO:16830 |
| iPS:393862 | | NA | | CGGGCAAGTCAGAACATTAT TAGTTATTTAAAT | GGTGCATCCAGTTTGCA AAGT | CAACAGAGTTACAGTACTCC CTTATTCACT |
| | 21-225_5G2 | | | SEQ ID NO:807 | SEQ ID NO:8819 | SEQ ID NO:16831 |
| | | AA | | RASQNIISYLN | GASSLQS | QQSYSTPLFT |
| | | | | SEQ ID NO:808 | SEQ ID NO:8820 | SEQ ID NO:16832 |
| iPS:393864 | | NA | | CGGGCAAGTCGGGGCATCA GAGGTGATTTAGGT | GCTGCATCCAATTTGCAA AGT | CTACAGCATTATAGTTACCC TCGGACG |
| | 21-225_4C5 | | | SEQ ID NO:809 | SEQ ID NO:8821 | SEQ ID NO:16833 |
| | | AA | | RASRGIRGDLG | AASNLQS | LQHYSYPRT |
| | | | | SEQ ID NO:810 | SEQ ID NO:8822 | SEQ ID NO:16834 |
| iPS:393866 | | NA | | CGGGCAAGTCAGGGCATTAG AAATGATTTAGGC | TCTGCATCCAGTTTGCAA AGT | GTACAGCATTATAGTTACCC GTTCACT |
| | 21-225_14E3 | | | SEQ ID NO:811 | SEQ ID NO:8823 | SEQ ID NO:16835 |
| | | AA | | RASQGIRNDLG | SASSLQS | VQHYSYPFT |
| | | | | SEQ ID NO:812 | SEQ ID NO:8824 | SEQ ID NO:16836 |
| iPS:393868 | | NA | | CGGGCAAGTCAGAACATTAG AAATTATTTAAAT | GTTGCATCCAGTTTGCAA AGT | CATCAGAGTAACAGTACTCC TCTCACG |
| | 21-225_9C11 | | | SEQ ID NO:813 | SEQ ID NO:8825 | SEQ ID NO:16837 |
| | | AA | | RASQNIRNYLN | VASSLQS | HQSNSTPLT |

FIGURE 49

| | | | SEQ ID NO:814 | SEQ ID NO:8826 | SEQ ID NO:16838 |
|---|---|---|---|---|---|
| iPS:393870 | | NA | CGGGCGAGTCAGGACATTAGCAATCATTTAGTC | GCTGCATCCAGTTTACAAAGT | CACCAGTATAATAGTTACCCCTTCACT |
| | 21-225_7B1 | | SEQ ID NO:815 | SEQ ID NO:8827 | SEQ ID NO:16839 |
| | | AA | RASQDISNHLV | AASSLQS | HQYNSYPFT |
| | | | SEQ ID NO:816 | SEQ ID NO:8828 | SEQ ID NO:16840 |
| iPS:393872 | | NA | CGGGCAAGTCAGGGCATTAGAAATGATTTAGGC | GCTGCATCCAGTTTGCAAAGT | CTACAGCATAATAGTTACCCGCTCACT |
| | 21-225_2A11 | | SEQ ID NO:817 | SEQ ID NO:8829 | SEQ ID NO:16841 |
| | | AA | RASQGIRNDLG | AASSLQS | LQHNSYPLT |
| | | | SEQ ID NO:818 | SEQ ID NO:8830 | SEQ ID NO:16842 |
| iPS:393874 | | NA | CGGGCAAGTCAGGGCATTAGAAATGATTTAGGC | GCTGCATCCAGTTTGCAAAGT | CTACAGCATAATAGTTACCCGCTCACT |
| | 21-225_4C8 | | SEQ ID NO:819 | SEQ ID NO:8831 | SEQ ID NO:16843 |
| | | AA | RASQGIRNDLG | AASSLQS | LQHNSYPLT |
| | | | SEQ ID NO:820 | SEQ ID NO:8832 | SEQ ID NO:16844 |
| iPS:393876 | | NA | CGGGCAAGTCAGGGCATTAGAAATGATTTAGGG | ACTGCATCCAGTTTGCAAAGT | ATACAGCATAATAGTTACCTGTGGACG |
| | 21-225_9A1 | | SEQ ID NO:821 | SEQ ID NO:8833 | SEQ ID NO:16845 |
| | | AA | RASQGIRNDLG | TASSLQS | IQHNSYLWT |
| | | | SEQ ID NO:822 | SEQ ID NO:8834 | SEQ ID NO:16846 |
| iPS:393878 | | NA | CGGGCAAGTCAGAATATTAACAACTATTTAAAT | ACTGCATCCAGTTTGCAAAGT | CAACAGAGTTACACTACCCCCACGTGGACG |
| | 21-225_7G12 | | SEQ ID NO:823 | SEQ ID NO:8835 | SEQ ID NO:16847 |
| | | AA | RASQNINNYLN | TASSLQS | QQSYTTPTWT |
| | | | SEQ ID NO:824 | SEQ ID NO:8836 | SEQ ID NO:16848 |
| iPS:393880 | | NA | CGGGCAAGTCAGGGCATTAGAAATGATTTAGGC | GCTGCATCCAGTTTACAAAGT | CTACACAACAGTAGTTACCCTGTTAAG |
| | 21-225_15A1 | | SEQ ID NO:825 | SEQ ID NO:8837 | SEQ ID NO:16849 |
| | | AA | RASQGIRNDLG | AASSLQS | LHNSSYPVK |

FIGURE 49

| | | | SEQ ID NO:826 | SEQ ID NO:8838 | SEQ ID NO:16850 |
|---|---|---|---|---|---|
| iPS:393882 | | NA | CGGGCAAGTCAGGGCATTAG AAATGATTTAGGC | GCTGCATCCAGTTCGCA AAGT | CTACAGCATCATAGTTACCC GCTCACT |
| | 21-225_15E3 | | SEQ ID NO:827 | SEQ ID NO:8839 | SEQ ID NO:16851 |
| | | AA | RASQGIRNDLG | AASSSQS | LQHHSYPLT |
| | | | SEQ ID NO:828 | SEQ ID NO:8840 | SEQ ID NO:16852 |
| iPS:393884 | | NA | CGGGCAAGTCAGGGCATTAG AAATGATTTAGGC | GTTGCATCCAGTTTGCAA AGT | ATACAGCATAATAGTTATCC GTTCACT |
| | 21-225_16F4 | | SEQ ID NO:829 | SEQ ID NO:8841 | SEQ ID NO:16853 |
| | | AA | RASQGIRNDLG | VASSLQS | IQHNSYPFT |
| | | | SEQ ID NO:830 | SEQ ID NO:8842 | SEQ ID NO:16854 |
| iPS:393886 | | NA | CGGGCAAGTCAGGGCATTAG AAATGATTTAGGT | GCTGCATCCAGTTTGCAA AGT | TTACAGCATGAAAGTTACCC TCTCACT |
| | 21-225_2G9 | | SEQ ID NO:831 | SEQ ID NO:8843 | SEQ ID NO:16855 |
| | | AA | RASQGIRNDLG | AASSLQS | LQHESYPLT |
| | | | SEQ ID NO:832 | SEQ ID NO:8844 | SEQ ID NO:16856 |
| iPS:393888 | | NA | CGGGCAAGTCAGAGCATTAG AAGTTATTTAAAT | GGTACATCCAGTTTGCA AAGT | CAACAGAGTTACAGTACCCC CTTGTTCACT |
| | 21-225_3E3 | | SEQ ID NO:833 | SEQ ID NO:8845 | SEQ ID NO:16857 |
| | | AA | RASQSIRSYLN | GTSSLQS | QQSYSTPLFT |
| | | | SEQ ID NO:834 | SEQ ID NO:8846 | SEQ ID NO:16858 |
| iPS:393890 | | NA | CGGGCAAGTCATACCATTAG AACCTATTTAAAC | GCTGCATCCAGTTTGCAA AGT | CAACAGAGTTACAATATCTC ATTCACT |
| | 21-225_4B1 | | SEQ ID NO:835 | SEQ ID NO:8847 | SEQ ID NO:16859 |
| | | AA | RASHTIRTYLN | AASSLQS | QQSYNISFT |
| | | | SEQ ID NO:836 | SEQ ID NO:8848 | SEQ ID NO:16860 |
| iPS:393892 | | NA | CAGGCGAGTCAGGACATTAG CAACTATTTAAAT | GATGCATCCACTTTGGA AACA | CAACAGTATGATAATGTCCC GATCACC |
| | 21-225_6G7 | | SEQ ID NO:837 | SEQ ID NO:8849 | SEQ ID NO:16861 |
| | | AA | QASQDISNYLN | DASTLET | QQYDNVPIT |

FIGURE 49

| | | | SEQ ID NO:838 | SEQ ID NO:8850 | SEQ ID NO:16862 |
|---|---|---|---|---|---|
| iPS:393894 | | NA | CGGGCGAGTCAGGGCATTAG CAATTATTTAGCC | GCTGCATCCAGTGTGCA GAGT | CACCAGTATCACAGTTACCC ATTCACT |
| | 21-225_5E11 | | SEQ ID NO:839 | SEQ ID NO:8851 | SEQ ID NO:16863 |
| | | AA | RASQGISNYLA | AASSVQS | HQYHSYPFT |
| | | | SEQ ID NO:840 | SEQ ID NO:8852 | SEQ ID NO:16864 |
| iPS:393896 | | NA | CGGGCGAGTCAGGGCATTAG CAATTATTTAGCC | ACTGCATCCAGTTTGCAA AGT | CAACAGTATAATAGTTACCC ATTCACT |
| | 21-225_2A4 | | SEQ ID NO:841 | SEQ ID NO:8853 | SEQ ID NO:16865 |
| | | AA | RASQGISNYLA | TASSLQS | QQYNSYPFT |
| | | | SEQ ID NO:842 | SEQ ID NO:8854 | SEQ ID NO:16866 |
| iPS:393898 | | NA | CGGGCAAGTCAGACCATTAG TAGTTATTTAAAT | GCTGCATCCAGTTTGCAA AGT | CAACAGAGTTACAATACCCC CTTATTCACT |
| | 21-225_5F7 | | SEQ ID NO:843 | SEQ ID NO:8855 | SEQ ID NO:16867 |
| | | AA | RASQTISSYLN | AASSLQS | QQSYNTPLFT |
| | | | SEQ ID NO:844 | SEQ ID NO:8856 | SEQ ID NO:16868 |
| iPS:393900 | | NA | CGGGCAAGTCAGAACATTTA CAGTTATTTAAAT | GCTACATCCAGTTTGCAA AGT | CAACAGAATTACAGTCCCCC TCTCACT |
| | 21-225_10E12 | | SEQ ID NO:845 | SEQ ID NO:8857 | SEQ ID NO:16869 |
| | | AA | RASQNIYSYLN | ATSSLQS | QQNYSPPLT |
| | | | SEQ ID NO:846 | SEQ ID NO:8858 | SEQ ID NO:16870 |
| iPS:393902 | | NA | CGGACAAGTCAGGGCATTAG AAATGATTTAGGC | GCTGCATCCAGTTTACAA AGT | CTGCAGCATTATAGTTACCC TCGGACG |
| | 21-225_14E10 | | SEQ ID NO:847 | SEQ ID NO:8859 | SEQ ID NO:16871 |
| | | AA | RTSQGIRNDLG | AASSLQS | LQHYSYPRT |
| | | | SEQ ID NO:848 | SEQ ID NO:8860 | SEQ ID NO:16872 |
| iPS:393904 | | NA | CGGGCAAGTCAGAACATTAT CAGCTATTTAAAT | GTTACATCCAGTTTGCAC AGT | CAACAGAGTTACAGTACCCC TTTCACT |
| | 21-225_8H11 | | SEQ ID NO:849 | SEQ ID NO:8861 | SEQ ID NO:16873 |
| | | AA | RASQNIISYLN | VTSSLHS | QQSYSTPFT |

FIGURE 49

| | | | SEQ ID NO:850 | SEQ ID NO:8862 | SEQ ID NO:16874 |
|---|---|---|---|---|---|
| iPS:393906 | | NA | AGGGCCAGTCAGACTGTTAGCAGCAACTTAGCC | GGTGCATCCACCAGGGCCACT | CAGCAGTATCATGACTGGCCTCCGACG |
| | 21-225_13D3 | | SEQ ID NO:851 | SEQ ID NO:8863 | SEQ ID NO:16875 |
| | | AA | RASQTVSSNLA | GASTRAT | QQYHDWPPT |
| iPS:393908 | | NA | SEQ ID NO:852 | SEQ ID NO:8864 | SEQ ID NO:16876 |
| | | NA | CGGGCAAGTCAGGACATTAGAAGTGATTTAGGC | GCTGCATCCAGTTTGCAAAGT | CTACAGCATTATAGTTACCCTCGGACG |
| | 21-225_10E9 | | SEQ ID NO:853 | SEQ ID NO:8865 | SEQ ID NO:16877 |
| | | AA | RASQDIRSDLG | AASSLQS | LQHYSYPRT |
| iPS:393910 | | NA | SEQ ID NO:854 | SEQ ID NO:8866 | SEQ ID NO:16878 |
| | | NA | CAGGCGAATCAGGACATTACCAACTTTTTAAAT | GATGCATCCAATTTGGAAACA | CAACAGTATGATAATCTCCCGATCACC |
| | 21-225_15F10 | | SEQ ID NO:855 | SEQ ID NO:8867 | SEQ ID NO:16879 |
| | | AA | QANQDITNFLN | DASNLET | QQYDNLPIT |
| iPS:393912 | | NA | SEQ ID NO:856 | SEQ ID NO:8868 | SEQ ID NO:16880 |
| | | NA | CAGGCGAATCAGGACATTACCAACTTTTTAAAT | GATGCATCCAATTTGGAAACA | CAACAGTATGATAATCTCCCGATCACC |
| | 21-225_16F6 | | SEQ ID NO:857 | SEQ ID NO:8869 | SEQ ID NO:16881 |
| | | AA | QANQDITNFLN | DASNLET | QQYDNLPIT |
| iPS:393914 | | NA | SEQ ID NO:858 | SEQ ID NO:8870 | SEQ ID NO:16882 |
| | | NA | CGGGCGAGTCAGGGCATTAACAATTATTTAGCC | GCTGCATCCAGTGTGCAGAGT | CACCAGTATCACAGTTACCCATTCACT |
| | 21-225_16B8 | | SEQ ID NO:859 | SEQ ID NO:8871 | SEQ ID NO:16883 |
| | | AA | RASQGINNYLA | AASSVQS | HQYHSYPFT |
| iPS:393916 | | NA | SEQ ID NO:860 | SEQ ID NO:8872 | SEQ ID NO:16884 |
| | | NA | CGGGCAAGTCAGGGCATTAGAAATGATTTAGGC | GCTGCATCCAGTTTGCACAGT | CTACAACATTATAGTTTCCCTCGGACG |
| | 21-225_2G4 | | SEQ ID NO:861 | SEQ ID NO:8873 | SEQ ID NO:16885 |
| | | AA | RASQGIRNDLG | AASSLHS | LQHYSFPRT |

FIGURE 49

| | | | SEQ ID NO:862 | SEQ ID NO:8874 | SEQ ID NO:16886 |
|---|---|---|---|---|---|
| iPS:393920 | | NA | CGGGCAAGTCAGAACATTTACAGGTATTTAAAT | ACTGCATCCAGTTTACAAAGT | CAACAGAGTTACAGTCCCCCTCTCACT |
| | 21-225_1H12 | | SEQ ID NO:863 | SEQ ID NO:8875 | SEQ ID NO:16887 |
| | | AA | RASQNIYRYLN | TASSLQS | QQSYSPPLT |
| | | | SEQ ID NO:864 | SEQ ID NO:8876 | SEQ ID NO:16888 |
| iPS:393922 | | NA | CGGGCAAGTCAGGGCATTAGAAATGATTTAGGC | GCTGCATCCAGTTTACAAAGT | CTACAGCATAATAGTTACCCGCTCACT |
| | 21-225_2B2 | | SEQ ID NO:865 | SEQ ID NO:8877 | SEQ ID NO:16889 |
| | | AA | RASQGIRNDLG | AASSLQS | LQHNSYPLT |
| | | | SEQ ID NO:866 | SEQ ID NO:8878 | SEQ ID NO:16890 |
| iPS:393926 | | NA | CGGGCAAGTCAGACCATTATCAGCTATTTAAAT | ACTGCATCCAGTTTGCAAAGT | CAACAGACTTACAGTACTCCGCTCACT |
| | 21-225_4G4 | | SEQ ID NO:867 | SEQ ID NO:8879 | SEQ ID NO:16891 |
| | | AA | RASQTIISYLN | TASSLQS | QQTYSTPLT |
| | | | SEQ ID NO:868 | SEQ ID NO:8880 | SEQ ID NO:16892 |
| iPS:393928 | | NA | CGGGCAAGTCAGGGCATTAGAAATGATTTAGGC | GCTGCATCCAGTTTGCAAAGT | TTACAGCATGATAATTACCCTCTCACT |
| | 21-225_4E10 | | SEQ ID NO:869 | SEQ ID NO:8881 | SEQ ID NO:16893 |
| | | AA | RASQGIRNDLG | AASSLQS | LQHDNYPLT |
| | | | SEQ ID NO:870 | SEQ ID NO:8882 | SEQ ID NO:16894 |
| iPS:393930 | | NA | CGGGCAAGTCAAAACATTATCAGCTATTTAAAT | ACTGCATCCAGTTTGCAAAGT | CAACAGACTTACAGTACCCCGCTCACT |
| | 21-225_7E11 | | SEQ ID NO:871 | SEQ ID NO:8883 | SEQ ID NO:16895 |
| | | AA | RASQNIISYLN | TASSLQS | QQTYSTPLT |
| | | | SEQ ID NO:872 | SEQ ID NO:8884 | SEQ ID NO:16896 |
| iPS:393932 | | NA | CGGGCAAGTCAGAACATTTACAGGTATTTAAAT | ACTGCATCCAGTTTGCAAAGT | CAACAGAGTTACAGTCCCCCTCTCACT |
| | 21-225_10F5 | | SEQ ID NO:873 | SEQ ID NO:8885 | SEQ ID NO:16897 |
| | | AA | RASQNIYRYLN | TASSLQS | QQSYSPPLT |

FIGURE 49

| | | | SEQ ID NO:874 | SEQ ID NO:8886 | SEQ ID NO:16898 |
|---|---|---|---|---|---|
| iPS:393934 | | NA | CGGGCAAGTCAGGGCATTAG AAATGATTTAGGC | GCTGCATCCAGTTTGCAA AGT | GTACAGCATAATAGTTACCC GCTCACT |
| | 21-225_13E6 | | SEQ ID NO:875 | SEQ ID NO:8887 | SEQ ID NO:16899 |
| | | AA | RASQGIRNDLG | AASSLQS | VQHNSYPLT |
| | | | SEQ ID NO:876 | SEQ ID NO:8888 | SEQ ID NO:16900 |
| iPS:393936 | | NA | CGGGCAAGTCAGAGCATTAG CAGTTATTTAAAT | GCTGCATCCAGTTTGCAA AAT | CAACAGACTTACAGTAGCCC TCCATTCACT |
| | 21-225_14A11 | | SEQ ID NO:877 | SEQ ID NO:8889 | SEQ ID NO:16901 |
| | | AA | RASQSISSYLN | AASSLQN | QQTYSSPPFT |
| | | | SEQ ID NO:878 | SEQ ID NO:8890 | SEQ ID NO:16902 |
| iPS:393940 | | NA | CGGGCAAGTCAGAGCATTAG CGGCTATTTAAAT | GCTGCATCCAGTTTGCAA AGT | CAACAGACTTACAATACCCC TCCGGAGCGCAGT |
| | 21-225_16B2 | | SEQ ID NO:879 | SEQ ID NO:8891 | SEQ ID NO:16903 |
| | | AA | RASQSISGYLN | AASSLQS | QQTYNTPPERS |
| | | | SEQ ID NO:880 | SEQ ID NO:8892 | SEQ ID NO:16904 |
| iPS:393942 | | NA | AAGTCCAGCCAGAGTGTTTT ATACAGCTCCAACAATAACA ACTACTTAACT | TGGGCATCTACCCGGGA ATCC | CAGCAATATTATAGTACTCC TCCGACG |
| | 21-225_11E5 | | SEQ ID NO:881 | SEQ ID NO:8893 | SEQ ID NO:16905 |
| | | AA | KSSQSVLYSSNNNNYLT | WASTRES | QQYYSTPPT |
| | | | SEQ ID NO:882 | SEQ ID NO:8894 | SEQ ID NO:16906 |
| iPS:393944 | | NA | CGGGCAAGTCAGGACATTAG AAATCATTTAGGC | GCTGCATCCAGTTTGCAA AGT | CTACAGTATAATAGTTACCC ATTCACT |
| | 21-225_14D6 | | SEQ ID NO:883 | SEQ ID NO:8895 | SEQ ID NO:16907 |
| | | AA | RASQDIRNHLG | AASSLQS | LQYNSYPFT |
| | | | SEQ ID NO:884 | SEQ ID NO:8896 | SEQ ID NO:16908 |
| iPS:393946 | | NA | CGGGCGAGTCAGGACATTAG TAATTATTTAGCC | GCTGCATCCAGTTTGCAA AGT | CAACAGTATCATAGTTACCC GTGGACG |
| | 21-225_16A4 | | SEQ ID NO:885 | SEQ ID NO:8897 | SEQ ID NO:16909 |

FIGURE 49

| | | | | | |
|---|---|---|---|---|---|
| | | AA | RASQDISNYLA | AASSLQS | QQYHSYPWT |
| | | | SEQ ID NO:886 | SEQ ID NO:8898 | SEQ ID NO:16910 |
| iPS:393948 | | NA | CGGGCAAGTCAGGGCATTAGAAATGATTTAGGC | GCTGCATCCAGTTTGCAAAGT | CTACAGCATAATAGTTACCCGTGGACG |
| | 21-225_16A5 | | SEQ ID NO:887 | SEQ ID NO:8899 | SEQ ID NO:16911 |
| | | AA | RASQGIRNDLG | AASSLQS | LQHNSYPWT |
| | | | SEQ ID NO:888 | SEQ ID NO:8900 | SEQ ID NO:16912 |
| iPS:393950 | | NA | TCTGGAGATAAATTGGGGGAAAAATATGCTTGC | CAAGATAGGAAGCGGCCCTCA | CAGGCGTGGGTCAACAACACTATGATA |
| | 21-225_3H10 | | SEQ ID NO:889 | SEQ ID NO:8901 | SEQ ID NO:16913 |
| | | AA | SGDKLGEKYAC | QDRKRPS | QAWVNNTMI |
| | | | SEQ ID NO:890 | SEQ ID NO:8902 | SEQ ID NO:16914 |
| iPS:393952 | | NA | CGGGCGAGTCAGGGCATTAACAATTATTTAGCC | GTTGCATCCAGTTTGCAAACT | CAACAGTATAATAGTTACCCTCTCACT |
| | 21-225_1F1 | | SEQ ID NO:891 | SEQ ID NO:8903 | SEQ ID NO:16915 |
| | | AA | RASQGINNYLA | VASSLQT | QQYNSYPLT |
| | | | SEQ ID NO:892 | SEQ ID NO:8904 | SEQ ID NO:16916 |
| iPS:393954 | | NA | CGGGCGAGTCAGGGTATTAGCAGGTGGTTAGCC | GGTGCATCCAGTTTGCAAAGT | CAACAGGCTAACAGTTTCCCATTCACT |
| | 21-225_4H6 | | SEQ ID NO:893 | SEQ ID NO:8905 | SEQ ID NO:16917 |
| | | AA | RASQGISRWLA | GASSLQS | QQANSFPFT |
| | | | SEQ ID NO:894 | SEQ ID NO:8906 | SEQ ID NO:16918 |
| iPS:393956 | | NA | CGGGCAAGTCAGAGCATTAGCGACTATTTAAAT | GATACAACCAGTTTGCAAAGT | CAACAGACTTACAATACCCCTCCGGAGCGCAGT |
| | 21-225_4D7 | | SEQ ID NO:895 | SEQ ID NO:8907 | SEQ ID NO:16919 |
| | | AA | RASQSISDYLN | DTTSLQS | QQTYNTPPERS |
| | | | SEQ ID NO:896 | SEQ ID NO:8908 | SEQ ID NO:16920 |
| iPS:393958 | | NA | CGGGCAAGTCAGGGCATTAGAAATGATTTAGGC | GCTGCATCCAGTTTGCAAAGT | CTACAGCATAATAGTTACCCTCTCACT |
| | 21-225_5H2 | | SEQ ID NO:897 | SEQ ID NO:8909 | SEQ ID NO:16921 |

FIGURE 49

| | | | | | |
|---|---|---|---|---|---|
| | | AA | RASQGIRNDLG | AASSLQS | LQHNSYPLT |
| | | | SEQ ID NO:898 | SEQ ID NO:8910 | SEQ ID NO:16922 |
| iPS:393960 | | NA | CGGGCAAGTCAGGGCATTAG AAATGATTTAGGC | ACTGCATCCAGTTTGCAA AGT | CTACAACATAATAGTTACCC GTGGACG |
| | 21-225_7G2 | | SEQ ID NO:899 | SEQ ID NO:8911 | SEQ ID NO:16923 |
| | | AA | RASQGIRNDLG | TASSLQS | LQHNSYPWT |
| | | | SEQ ID NO:900 | SEQ ID NO:8912 | SEQ ID NO:16924 |
| iPS:393962 | | NA | CGGGCAAGTCAGGGCATTAG AAATGATTTAGGC | GCTGCATCCAGTTTGCAA AGT | CTACAGCATAGTAGTTACCC GTTCACT |
| | 21-225_7H7 | | SEQ ID NO:901 | SEQ ID NO:8913 | SEQ ID NO:16925 |
| | | AA | RASQGIRNDLG | AASSLQS | LQHSSYPFT |
| | | | SEQ ID NO:902 | SEQ ID NO:8914 | SEQ ID NO:16926 |
| iPS:393964 | | NA | CGGACAAGTCAGAACATTAT CAGCTATTTAAAT | ACTGCATCCAATTTGCAA ACT | CAACAGCCTCACAGTCCCCC GCTCACT |
| | 21-225_6G1 | | SEQ ID NO:903 | SEQ ID NO:8915 | SEQ ID NO:16927 |
| | | AA | RTSQNIISYLN | TASNLQT | QQPHSPPLT |
| | | | SEQ ID NO:904 | SEQ ID NO:8916 | SEQ ID NO:16928 |
| iPS:393966 | | NA | CGGGCAAGTCAGGGCATTGG AAATGATTTAGGC | GCTGCATCCAGTTTGCAA AGT | CTACAACATTATACTTACCC TCGGACG |
| | 21-225_7F8 | | SEQ ID NO:905 | SEQ ID NO:8917 | SEQ ID NO:16929 |
| | | AA | RASQGIGNDLG | AASSLQS | LQHYTYPRT |
| | | | SEQ ID NO:906 | SEQ ID NO:8918 | SEQ ID NO:16930 |
| iPS:393968 | | NA | CGGGCGAGTCAGGGCATTAG CAATTATTTAGCC | GCTGCATCCAGTTTGCAA AGT | CAACAGTATAATAGTTACCC ATTCACT |
| | 21-225_5A5 | | SEQ ID NO:907 | SEQ ID NO:8919 | SEQ ID NO:16931 |
| | | AA | RASQGISNYLA | AASSLQS | QQYNSYPFT |
| | | | SEQ ID NO:908 | SEQ ID NO:8920 | SEQ ID NO:16932 |
| iPS:393972 | | NA | CGGGCAAGTCAGGGCATTAG AAACGATTTAGGC | GCTGCATCCAGTTTGCAA AGT | CTACAGCTTTATAGTTACCCT CGGACG |
| | 21-225_7C9 | | SEQ ID NO:909 | SEQ ID NO:8921 | SEQ ID NO:16933 |

FIGURE 49

| | | | | | |
|---|---|---|---|---|---|
| | | AA | RASQGIRNDLG | AASSLQS | LQLYSYPRT |
| | | | SEQ ID NO:910 | SEQ ID NO:8922 | SEQ ID NO:16934 |
| iPS:393974 | | NA | CGGGCAAGTCAGGGCATTAGAAATGATTTAGGC | GCTGCATCCAGTTTGCAAAGT | CTACAGCATAATAGTTACCCGCTCACT |
| | 21-225_7C4 | | SEQ ID NO:911 | SEQ ID NO:8923 | SEQ ID NO:16935 |
| | | AA | RASQGIRNDLG | AASSLQS | LQHNSYPLT |
| | | | SEQ ID NO:912 | SEQ ID NO:8924 | SEQ ID NO:16936 |
| iPS:393976 | | NA | CGGGCAAGTCAGGGCATTAGAAATGATTTAGGC | ACTGCATCCAGTTTGCAAAGT | CTACAGCATAATAGTTACCCGCTCACT |
| | 21-225_7E9 | | SEQ ID NO:913 | SEQ ID NO:8925 | SEQ ID NO:16937 |
| | | AA | RASQGIRNDLG | TASSLQS | LQHNSYPLT |
| | | | SEQ ID NO:914 | SEQ ID NO:8926 | SEQ ID NO:16938 |
| iPS:393978 | | NA | CGGGCAAGTCAGGGCATTAGAAATGATTTAGGC | GCTGCATCCAGTTTGCACAGT | CTACAACATTATAGTTTCCCTCGGACG |
| | 21-225_4C12 | | SEQ ID NO:915 | SEQ ID NO:8927 | SEQ ID NO:16939 |
| | | AA | RASQGIRNDLG | AASSLHS | LQHYSFPRT |
| | | | SEQ ID NO:916 | SEQ ID NO:8928 | SEQ ID NO:16940 |
| iPS:393980 | | NA | CGGACAAGTCAGAGCATTAGTACTTATTTAAAT | GCTGCATCCAGTTTGCAAAGT | CAACAGAGTTACAGAACCCCCTTTTTCACT |
| | 21-225_6D3 | | SEQ ID NO:917 | SEQ ID NO:8929 | SEQ ID NO:16941 |
| | | AA | RTSQSISTYLN | AASSLQS | QQSYRTPFFT |
| | | | SEQ ID NO:918 | SEQ ID NO:8930 | SEQ ID NO:16942 |
| iPS:393982 | | NA | CGGGCAAGTCAGGGCATTAGAAGTAATTTAGGC | GCTGCATCCAGTTTGGAAAGT | CTACAGGATAATAGTTATCCGTTCACT |
| | 21-225_6C12 | | SEQ ID NO:919 | SEQ ID NO:8931 | SEQ ID NO:16943 |
| | | AA | RASQGIRSNLG | AASSLES | LQDNSYPFT |
| | | | SEQ ID NO:920 | SEQ ID NO:8932 | SEQ ID NO:16944 |
| iPS:393984 | | NA | CGGGCAAGTCAGGGCATTAGAAATGATTTAGGC | GCTGCATCCAGTTTGCAAAGT | CTACAGCATAATAGTTACGCGCTCACT |
| | 21-225_4F12 | | SEQ ID NO:921 | SEQ ID NO:8933 | SEQ ID NO:16945 |

EP 4 435 105 A2

FIGURE 49

| | | | AA | RASQGIRNDLG | AASSLQS | LQHNSYALT |
|---|---|---|---|---|---|---|
| | | | | SEQ ID NO:922 | SEQ ID NO:8934 | SEQ ID NO:16946 |
| iPS:393986 | | | NA | CGGGCAAGTCAGGGCATTAGAAATGATTTAGGC | GCTGCATCCAGTTTGCAAAGT | CTACATCAATATAGTTACCCTCGGACG |
| | 21-225_7G4 | | | SEQ ID NO:923 | SEQ ID NO:8935 | SEQ ID NO:16947 |
| | | | AA | RASQGIRNDLG | AASSLQS | LHQYSYPRT |
| | | | | SEQ ID NO:924 | SEQ ID NO:8936 | SEQ ID NO:16948 |
| iPS:393988 | | | NA | CGGGCGAGTCAGGACATTAGGAATTATTTAGCC | GTTGCATCCAGTTTGCAAAGT | CAACAGTATAATAGTTACCCTCTCACT |
| | 21-225_7F10 | | | SEQ ID NO:925 | SEQ ID NO:8937 | SEQ ID NO:16949 |
| | | | AA | RASQDIRNYLA | VASSLQS | QQYNSYPLT |
| | | | | SEQ ID NO:926 | SEQ ID NO:8938 | SEQ ID NO:16950 |
| iPS:393990 | | | NA | CGGGCAAGTCAGGGCATTAGAAATGATTTAGGC | GCTGCATCCAGTTTGCAAAGT | CTACAGCATAGTAATTACCCTCTCACT |
| | 21-225_11G7 | | | SEQ ID NO:927 | SEQ ID NO:8939 | SEQ ID NO:16951 |
| | | | AA | RASQGIRNDLG | AASSLQS | LQHSNYPLT |
| | | | | SEQ ID NO:928 | SEQ ID NO:8940 | SEQ ID NO:16952 |
| iPS:393992 | | | NA | CGGGCGAGTCAGGGCATTAGCTATTATTTAGCC | GTTGCATCCAGTTTGCAAAGT | CAACAGTATAATAGTTACCCATTCACT |
| | 21-225_14H8 | | | SEQ ID NO:929 | SEQ ID NO:8941 | SEQ ID NO:16953 |
| | | | AA | RASQGISYYLA | VASSLQS | QQYNSYPFT |
| | | | | SEQ ID NO:930 | SEQ ID NO:8942 | SEQ ID NO:16954 |
| iPS:393994 | | | NA | CGGGCAAGTCAGGCCATTAGAAATGATTTAGAC | GCTGCATCCAGTTTGCAAAGT | CTACAGCATAATAGTTATCCGCTCACT |
| | 21-225_8C9 | | | SEQ ID NO:931 | SEQ ID NO:8943 | SEQ ID NO:16955 |
| | | | AA | RASQAIRNDLD | AASSLQS | LQHNSYPLT |
| | | | | SEQ ID NO:932 | SEQ ID NO:8944 | SEQ ID NO:16956 |
| iPS:393996 | | | NA | CGGGCAAGTCAGGGCATTAGAAATGATTTAGGC | GCTGCATCCAGTTTGCAAAGT | CTACTGCATTATAGTTACCCTCGGACG |
| | 21-225_15C11 | | | SEQ ID NO:933 | SEQ ID NO:8945 | SEQ ID NO:16957 |

653

FIGURE 49

| | | AA | RASQGIRNDLG | AASSLQS | LLHYSYPRT |
|---|---|---|---|---|---|
| | | | SEQ ID NO:934 | SEQ ID NO:8946 | SEQ ID NO:16958 |
| iPS:393998 | | NA | CGGGCAAGTCAGGGCATTAG AAATGATTTAGGC | ACTGCATCCAGTTTGCAA AGT | CTACAACATAATAGTTACCC GTGGACG |
| | 21-225_12B12 | | SEQ ID NO:935 | SEQ ID NO:8947 | SEQ ID NO:16959 |
| | | AA | RASQGIRNDLG | TASSLQS | LQHNSYPWT |
| | | | SEQ ID NO:936 | SEQ ID NO:8948 | SEQ ID NO:16960 |
| iPS:394000 | | NA | CAGGCGAGTCAGGACATTAG CAACTATTTAAAT | GATGCATCCAATTTGGA AACA | CAACAGTATGATAATCTCCC GATCACC |
| | 21-225_11A2 | | SEQ ID NO:937 | SEQ ID NO:8949 | SEQ ID NO:16961 |
| | | AA | QASQDISNYLN | DASNLET | QQYDNLPIT |
| | | | SEQ ID NO:938 | SEQ ID NO:8950 | SEQ ID NO:16962 |
| iPS:394002 | | NA | CGGGCAAGTCAGGGCATTAG AAATGATTTAGGC | GCTGCATCCAGTTTGCAA AGT | CTACAGCATAGTAATTACCC TCTCACT |
| | 21-225_15G7 | | SEQ ID NO:939 | SEQ ID NO:8951 | SEQ ID NO:16963 |
| | | AA | RASQGIRNDLG | AASSLQS | LQHSNYPLT |
| | | | SEQ ID NO:940 | SEQ ID NO:8952 | SEQ ID NO:16964 |
| iPS:394004 | | NA | CAGGCGAGTCAGGACATTAA CAACTATTTAAAT | GATGGATCCAATTTGGA AACA | CAACAGTATGAAAATCTCCC GATCACT |
| | 21-225_13A1 | | SEQ ID NO:941 | SEQ ID NO:8953 | SEQ ID NO:16965 |
| | | AA | QASQDINNYLN | DGSNLET | QQYENLPIT |
| | | | SEQ ID NO:942 | SEQ ID NO:8954 | SEQ ID NO:16966 |
| iPS:394006 | | NA | CAGGCGAGTCAGGACATTAC CAACTATTTAAAT | GATGCATCCAATTTGGA AACA | CAACAGTATGATAATCTCCC GATCACC |
| | 21-225_15C2 | | SEQ ID NO:943 | SEQ ID NO:8955 | SEQ ID NO:16967 |
| | | AA | QASQDITNYLN | DASNLET | QQYDNLPIT |
| | | | SEQ ID NO:944 | SEQ ID NO:8956 | SEQ ID NO:16968 |
| iPS:394008 | | NA | CGGGCAAGTCAGGGCATTAG AAATGATTTAGGC | GCTGCATCCAGTTTGCAA AGT | CTACAGCATAATAGTTACCC GCTCACT |
| | 21-225_15H8 | | SEQ ID NO:945 | SEQ ID NO:8957 | SEQ ID NO:16969 |

FIGURE 49

| | | AA | RASQGIRNDLG | AASSLQS | LQHNSYPLT |
|---|---|---|---|---|---|
| | | | SEQ ID NO:946 | SEQ ID NO:8958 | SEQ ID NO:16970 |
| iPS:394010 | | NA | CGGGCGAGTCAGGACATTAGCAATTATTTAGCC | GCTGCATACATTTTGCAATCA | CAAAAGTATGACAGTGCCCCATTCACT |
| | 21-225_12G5 | | SEQ ID NO:947 | SEQ ID NO:8959 | SEQ ID NO:16971 |
| | | AA | RASQDISNYLA | AAYILQS | QKYDSAPFT |
| | | | SEQ ID NO:948 | SEQ ID NO:8960 | SEQ ID NO:16972 |
| iPS:394012 | | NA | CGGGCAAGTCAGAGCATTATCAGCTATTTAAAT | ACTGCATCCAGTTTGCAAAGT | CAACAGACTTACAGTACCCCGCTCACT |
| | 21-225_15A3 | | SEQ ID NO:949 | SEQ ID NO:8961 | SEQ ID NO:16973 |
| | | AA | RASQSIISYLN | TASSLQS | QQTYSTPLT |
| | | | SEQ ID NO:950 | SEQ ID NO:8962 | SEQ ID NO:16974 |
| iPS:394014 | | NA | CGGGCAAGTCAGAGCATTAGTAGTTATTTAAAT | GCTGCATCCAGTTTGCAAAGT | CAACAGAGTTACAGAACCCCCTTTTTCACT |
| | 21-225_8G6 | | SEQ ID NO:951 | SEQ ID NO:8963 | SEQ ID NO:16975 |
| | | AA | RASQSISSYLN | AASSLQS | QQSYRTPFFT |
| | | | SEQ ID NO:952 | SEQ ID NO:8964 | SEQ ID NO:16976 |
| iPS:394016 | | NA | CGGGCAAGTCAGAGCATTTTCAGCTACTTAAAT | ACTGCATCCAGTTTGCAAAAT | CAACAGACTTACAGTCTTCCGCTCACT |
| | 21-225_13D4 | | SEQ ID NO:953 | SEQ ID NO:8965 | SEQ ID NO:16977 |
| | | AA | RASQSIFSYLN | TASSLQN | QQTYSLPLT |
| | | | SEQ ID NO:954 | SEQ ID NO:8966 | SEQ ID NO:16978 |
| iPS:394018 | | NA | CGGGCGAGTCAGGGCATTAGCAATTATTTAGCC | GCTGCATCCAGTTTGCAAAGT | CAACAGTATCATAGTTACCCATTCACT |
| | 21-225_15B1 | | SEQ ID NO:955 | SEQ ID NO:8967 | SEQ ID NO:16979 |
| | | AA | RASQGISNYLA | AASSLQS | QQYHSYPFT |
| | | | SEQ ID NO:956 | SEQ ID NO:8968 | SEQ ID NO:16980 |
| iPS:394020 | | NA | CGGGCAAGTCAGGGCATTAGAAATGATTTAGGC | GCTGCATCCAGTTTGCAAAGT | CTACAGCATAATAGTTACCCGCTCACT |
| | 21-225_15H10 | | SEQ ID NO:957 | SEQ ID NO:8969 | SEQ ID NO:16981 |

655

FIGURE 49

| | | | | | |
|---|---|---|---|---|---|
| | | AA | RASQGIRNDLG | AASSLQS | LQHNSYPLT |
| | | | SEQ ID NO:958 | SEQ ID NO:8970 | SEQ ID NO:16982 |
| iPS:394022 | | NA | CGGGCAAGTCAGAACATTAGCAGCTATTTAAAT | GCTGCATCCAGTTTGCAAAGT | CAACAGAGTTACAGAACCCCCTTATTCACT |
| | 21-225_16H6 | | SEQ ID NO:959 | SEQ ID NO:8971 | SEQ ID NO:16983 |
| | | AA | RASQNISSYLN | AASSLQS | QQSYRTPLFT |
| | | | SEQ ID NO:960 | SEQ ID NO:8972 | SEQ ID NO:16984 |
| iPS:394024 | | NA | CGGGCAAGTCAGGGCATTAGAAATGATTTAGGC | GCTGCATCCAGTTTGCAAAGT | CTACAGCATAATAGTTACCCGCTCACT |
| | 21-225_16B7 | | SEQ ID NO:961 | SEQ ID NO:8973 | SEQ ID NO:16985 |
| | | AA | RASQGIRNDLG | AASSLQS | LQHNSYPLT |
| | | | SEQ ID NO:962 | SEQ ID NO:8974 | SEQ ID NO:16986 |
| iPS:394026 | | NA | CGGGCGAGTCAGGGCATTAGCAATTATTTAGCC | GCTGCATCCAGTTTGCAAAGT | CAACAGTATAATAGTTACCCATTCACT |
| | 21-225_16C7 | | SEQ ID NO:963 | SEQ ID NO:8975 | SEQ ID NO:16987 |
| | | AA | RASQGISNYLA | AASSLQS | QQYNSYPFT |
| | | | SEQ ID NO:964 | SEQ ID NO:8976 | SEQ ID NO:16988 |
| iPS:394029 | | NA | CAGGCGAGTCAGGACATTAACAACTATTTAAAT | GATGCATCCAATTTGGAAACA | CAACAGTATGAAAATCTCCCGATCACC |
| | 21-225_1B12 | | SEQ ID NO:965 | SEQ ID NO:8977 | SEQ ID NO:16989 |
| | | AA | QASQDINNYLN | DASNLET | QQYENLPIT |
| | | | SEQ ID NO:966 | SEQ ID NO:8978 | SEQ ID NO:16990 |
| iPS:394033 | | NA | CGGGCAAGTCAGGGCATTCGAAATCATTTAGGC | GCTGCCTCCAGTTTGCAAAGT | CTACAGTATAATGGTTACCCATTCACT |
| | 21-225_5F4 | | SEQ ID NO:967 | SEQ ID NO:8979 | SEQ ID NO:16991 |
| | | AA | RASQGIRNHLG | AASSLQS | LQYNGYPFT |
| | | | SEQ ID NO:968 | SEQ ID NO:8980 | SEQ ID NO:16992 |
| iPS:394035 | | NA | CAGGCGAGTCAGGGCATTAGCAACTCTTTAAAT | GATGCATCCAATTTGGAAACA | CAACAATATGATAATCTCCCGCTCACT |
| | 21-225_5G9 | | SEQ ID NO:969 | SEQ ID NO:8981 | SEQ ID NO:16993 |

FIGURE 49

| | | | | | |
|---|---|---|---|---|---|
| | | AA | QASQGISNSLN | DASNLET | QQYDNLPLT |
| | | | SEQ ID NO:970 | SEQ ID NO:8982 | SEQ ID NO:16994 |
| iPS:394037 | | NA | CGGGCAAGTCAGGGCATTAGAAATGATTTAGGC | GCTGCGTCCAGTGTGCAAACT | CTACAGCATAATAGTTACCCGCTCACT |
| | 21-225_4F4 | | SEQ ID NO:971 | SEQ ID NO:8983 | SEQ ID NO:16995 |
| | | AA | RASQGIRNDLG | AASSVQT | LQHNSYPLT |
| | | | SEQ ID NO:972 | SEQ ID NO:8984 | SEQ ID NO:16996 |
| iPS:394041 | | NA | CGGGCAAGTCAGGGCATTAGAAATGATTTAGGC | GCTGCATCCAGTTTGCAAAGT | CTACAGCATTATAGTTACCCTCGGACG |
| | 21-225_5E5 | | SEQ ID NO:973 | SEQ ID NO:8985 | SEQ ID NO:16997 |
| | | AA | RASQGIRNDLG | AASSLQS | LQHYSYPRT |
| | | | SEQ ID NO:974 | SEQ ID NO:8986 | SEQ ID NO:16998 |
| iPS:394043 | | NA | CGGGCAAGTCAGAGTATTAATAATTATTTAAAT | GCTACATCCAGTTTGCAAAAT | CAACAGAGTTACAGTACCCCCTTATTCACT |
| | 21-225_3B1 | | SEQ ID NO:975 | SEQ ID NO:8987 | SEQ ID NO:16999 |
| | | AA | RASQSINNYLN | ATSSLQN | QQSYSTPLFT |
| | | | SEQ ID NO:976 | SEQ ID NO:8988 | SEQ ID NO:17000 |
| iPS:394045 | | NA | CGGGCAAGTCAGGGCATTAGAAATGATTTAGGC | GCTGCATCCAGTGTGCAAAGT | CTACAGCATAATAGTTACCCGCTCACT |
| | 21-225_4H4 | | SEQ ID NO:977 | SEQ ID NO:8989 | SEQ ID NO:17001 |
| | | AA | RASQGIRNDLG | AASSVQS | LQHNSYPLT |
| | | | SEQ ID NO:978 | SEQ ID NO:8990 | SEQ ID NO:17002 |
| iPS:394047 | | NA | CAGGCGAGTCAGGACATTAACAACTATTTAAAT | GATGCATCCAATTTGGAAACA | CAACAGTATGATAATCTCCCGATCACC |
| | 21-225_5E6 | | SEQ ID NO:979 | SEQ ID NO:8991 | SEQ ID NO:17003 |
| | | AA | QASQDINNYLN | DASNLET | QQYDNLPIT |
| | | | SEQ ID NO:980 | SEQ ID NO:8992 | SEQ ID NO:17004 |
| iPS:394049 | | NA | CGGGCAAGTCAGGGCATTAGAAATGATTTAGGC | GCTGCATCCAGTTTGCAAACT | CTACAGCATAATAGTTACCCTCTCACT |
| | 21-225_13H5 | | SEQ ID NO:981 | SEQ ID NO:8993 | SEQ ID NO:17005 |

657

FIGURE 49

| | | | | | |
|---|---|---|---|---|---|
| | | AA | RASQGIRNDLG | AASSLQT | LQHNSYPLT |
| | | | SEQ ID NO:982 | SEQ ID NO:8994 | SEQ ID NO:17006 |
| iPS:394051 | | NA | CGGGCAAGTCAGAGCATTGC CAGTTATTTAAAT | GGTGCATCCAGTTTGCA AAGT | CAACAGAGTTACAGTACCCC CTTATTCAGT |
| | 21-225_9E5 | | SEQ ID NO:983 | SEQ ID NO:8995 | SEQ ID NO:17007 |
| | | AA | RASQSIASYLN | GASSLQS | QQSYSTPLFS |
| | | | SEQ ID NO:984 | SEQ ID NO:8996 | SEQ ID NO:17008 |
| iPS:394053 | | NA | CGGGCAAGTCAGGGCATTAG AAATGATTTAGGC | GCTGCATCCAGTTTACAA AGT | CTACAGCATAGTAGTTACCC TCTCACT |
| | 21-225_11F10 | | SEQ ID NO:985 | SEQ ID NO:8997 | SEQ ID NO:17009 |
| | | AA | RASQGIRNDLG | AASSLQS | LQHSSYPLT |
| | | | SEQ ID NO:986 | SEQ ID NO:8998 | SEQ ID NO:17010 |
| iPS:394055 | | NA | CGGGCGAGTCAGGGCATTAG CTATTATTTAGCC | GTTGCATCCAGTTTGCAA AGT | CAACAGTATGATAGTTACCC ATTCACT |
| | 21-225_9C8 | | SEQ ID NO:987 | SEQ ID NO:8999 | SEQ ID NO:17011 |
| | | AA | RASQGISYYLA | VASSLQS | QQYDSYPFT |
| | | | SEQ ID NO:988 | SEQ ID NO:9000 | SEQ ID NO:17012 |
| iPS:394057 | | NA | CGGGCAAGTCAGGGCATTAG AAATGATTTAGGC | ACTGCATCCAGTTTGCAA AGT | CTACAGCATAGTAGTTACCC GCTCACT |
| | 21-225_15H1 | | SEQ ID NO:989 | SEQ ID NO:9001 | SEQ ID NO:17013 |
| | | AA | RASQGIRNDLG | TASSLQS | LQHSSYPLT |
| | | | SEQ ID NO:990 | SEQ ID NO:9002 | SEQ ID NO:17014 |
| iPS:394059 | | NA | CGGGCAAGTCAGGGCATTAG AAATGATTTAGGC | GCTGCATCCAGTTTGCAA AGT | CTACAGCATAATAGTTACCC GCTCACT |
| | 21-225_9E8 | | SEQ ID NO:991 | SEQ ID NO:9003 | SEQ ID NO:17015 |
| | | AA | RASQGIRNDLG | AASSLQS | LQHNSYPLT |
| | | | SEQ ID NO:992 | SEQ ID NO:9004 | SEQ ID NO:17016 |
| iPS:394061 | 21 225 12D2 | NA | AGGTCTAGTCAGAGCCTCCT CCATAGTAATGGATACAACT ATTTGGAT | TTGGGTTCTAATCGGGCC TCC | ATGCAAGCTCTACAAACTCC TATCACC |

FIGURE 49

| | | | SEQ ID NO:993 | SEQ ID NO:9005 | SEQ ID NO:17017 |
|---|---|---|---|---|---|
| | 21-225_12D2 | AA | RSSQSLLHSNGYNYLD | LGSNRAS | MQALQTPIT |
| | | | SEQ ID NO:994 | SEQ ID NO:9006 | SEQ ID NO:17018 |
| iPS:394063 | | NA | CGGGCAAGTCAGGGCATTAG AAATGATTTAGGC | GCTGCGTCCAGTTTGCAA AGT | CTACACCATAGTAATTACCC TCTCACT |
| | 21-225_16A1 | | SEQ ID NO:995 | SEQ ID NO:9007 | SEQ ID NO:17019 |
| | | AA | RASQGIRNDLG | AASSLQS | LHHSNYPLT |
| | | | SEQ ID NO:996 | SEQ ID NO:9008 | SEQ ID NO:17020 |
| iPS:394065 | | NA | AAGTCCAACCAGAGAGTTTT ATCCAGCTCCAACAATCACA ACTACTTAGCT | TGGGCATCTACCCGGGA ATCC | CAGCAATATTTTAGTACTCC ATTCACT |
| | 21-225_11E2 | | SEQ ID NO:997 | SEQ ID NO:9009 | SEQ ID NO:17021 |
| | | AA | KSNQRVLSSSNNHNYLA | WASTRES | QQYFSTPFT |
| | | | SEQ ID NO:998 | SEQ ID NO:9010 | SEQ ID NO:17022 |
| iPS:394067 | | NA | CGGGCAAGTCAGGGCATTAG AAATGATTTAGGC | GCTGCATCCAGTTTGCAA AGT | CTACAGCATAATAGTTATCC GTGGACG |
| | 21-225_12F2 | | SEQ ID NO:999 | SEQ ID NO:9011 | SEQ ID NO:17023 |
| | | AA | RASQGIRNDLG | AASSLQS | LQHNSYPWT |
| | | | SEQ ID NO:1000 | SEQ ID NO:9012 | SEQ ID NO:17024 |
| iPS:394069 | | NA | CGGGCAAGTCAGGGCATTAG AGATATTTTAGGC | GCTGCATCCAGTTTGCAA AAT | CTACAGTATCATAGTTACCC ATTCACT |
| | 21-225_16H1 | | SEQ ID NO:1001 | SEQ ID NO:9013 | SEQ ID NO:17025 |
| | | AA | RASQGIRDILG | AASSLQN | LQYHSYPFT |
| | | | SEQ ID NO:1002 | SEQ ID NO:9014 | SEQ ID NO:17026 |
| iPS:394071 | | NA | AGGTCTAGTCAGAGCCTCCT GCATAGTAAGGGATACAACT ATTTGGAT | TTGGGTTCTAATCGGGCC TCC | ATGCAAGCTCTACAAACTCC TCTCACC |
| | 21-225_10C7 | | SEQ ID NO:1003 | SEQ ID NO:9015 | SEQ ID NO:17027 |
| | | AA | RSSQSLLHSKGYNYLD | LGSNRAS | MQALQTPLT |
| | | | SEQ ID NO:1004 | SEQ ID NO:9016 | SEQ ID NO:17028 |

FIGURE 49

| iPS:394073 | | NA | CGGGCAAGTCAGGGCATTAG AAATGATTTAGGC | GCTGCATCCAGTTTGCAA AGT | CTACAACATACTAGTTACCC GCTCACT |
|---|---|---|---|---|---|
| | 21-225_15C9 | | SEQ ID NO:1005 | SEQ ID NO:9017 | SEQ ID NO:17029 |
| | | AA | RASQGIRNDLG | AASSLQS | LQHTSYPLT |
| | | | SEQ ID NO:1006 | SEQ ID NO:9018 | SEQ ID NO:17030 |
| iPS:394075 | | NA | CGGGCAAGTCAGGGCATTAG AAATGATTTAGGC | GCTGCATCCAGTTTGCAA AGT | CTACAGCATAGTAGTTACCC GCTCACT |
| | 21-225_8D12 | | SEQ ID NO:1007 | SEQ ID NO:9019 | SEQ ID NO:17031 |
| | | AA | RASQGIRNDLG | AASSLQS | LQHSSYPLT |
| | | | SEQ ID NO:1008 | SEQ ID NO:9020 | SEQ ID NO:17032 |
| iPS:394077 | | NA | CGGGCAAGTCAGAGCATTAG TAATTATTTAAAT | GCTGCATCCAGTTTACAA AGT | CAACAGAGTTACAGAACCCC CTTTTTCACT |
| | 21-225_8E12 | | SEQ ID NO:1009 | SEQ ID NO:9021 | SEQ ID NO:17033 |
| | | AA | RASQSISNYLN | AASSLQS | QQSYRTPFFT |
| | | | SEQ ID NO:1010 | SEQ ID NO:9022 | SEQ ID NO:17034 |
| iPS:394079 | | NA | CGGGCAAGTCAGGGCATTAG AAATGATTTAGGC | GCTGCATCCAGTGTGCA AAGT | CTACAGCATAATAGTTACCC GCTCACT |
| | 21-225_11F5 | | SEQ ID NO:1011 | SEQ ID NO:9023 | SEQ ID NO:17035 |
| | | AA | RASQGIRNDLG | AASSVQS | LQHNSYPLT |
| | | | SEQ ID NO:1012 | SEQ ID NO:9024 | SEQ ID NO:17036 |
| iPS:394081 | | NA | CAGGCGAGTCAGGACATTAA CAACTATTTAAAT | GATGCATCCAATTGGA AACA | CAACAGTTTGATAATCTCCC GATCACC |
| | 21-225_16B3 | | SEQ ID NO:1013 | SEQ ID NO:9025 | SEQ ID NO:17037 |
| | | AA | QASQDINNYLN | DASNLET | QQFDNLPIT |
| | | | SEQ ID NO:1014 | SEQ ID NO:9026 | SEQ ID NO:17038 |
| iPS:394083 | | NA | CGGGCAAGTCAGAGCATTAT CAGCTATTTAAAT | ACTACATCCAGTTTGCAA AGT | CAGCAGACTTACAGTACCCC GCTCACT |
| | 21-225_16E6 | | SEQ ID NO:1015 | SEQ ID NO:9027 | SEQ ID NO:17039 |
| | | AA | RASQSIISYLN | TTSSLQS | QQTYSTPLT |
| | | | SEQ ID NO:1016 | SEQ ID NO:9028 | SEQ ID NO:17040 |

FIGURE 49

| iPS:394085 | | NA | AAGTCCAGCCAGAATGTTTT ATACAACTCCAACAATAACA ACTACTTAGCT | TGGGCATCTACCCGGAA ATCC | CAGCAATATTATACTACTCC GTGCAGT |
|---|---|---|---|---|---|
| | 21-225_8B11 | | SEQ ID NO:1017 | SEQ ID NO:9029 | SEQ ID NO:17041 |
| | | AA | KSSQNVLYNSNNNNYLA | WASTRKS | QQYYTTPCS |
| | | | SEQ ID NO:1018 | SEQ ID NO:9030 | SEQ ID NO:17042 |
| iPS:394087 | | NA | CGGGCAAGTCAGAACATTTA TAGTTATTTAAAT | GCTGCATCCAGTTTGCAA AGT | CAACAGAGTTACAATACCCC CTTATTCACT |
| | 21-225_11A5 | | SEQ ID NO:1019 | SEQ ID NO:9031 | SEQ ID NO:17043 |
| | | AA | RASQNIYSYLN | AASSLQS | QQSYNTPLFT |
| | | | SEQ ID NO:1020 | SEQ ID NO:9032 | SEQ ID NO:17044 |
| iPS:394089 | | NA | CGGGCAAGTCAGGGCATTAG AAGTGATTTAGGC | GCTGCATCCAGTTTGCAA AGT | CTACAGCATAATAGTTACCC GTGGACG |
| | 21-225_12E6 | | SEQ ID NO:1021 | SEQ ID NO:9033 | SEQ ID NO:17045 |
| | | AA | RASQGIRSDLG | AASSLQS | LQHNSYPWT |
| | | | SEQ ID NO:1022 | SEQ ID NO:9034 | SEQ ID NO:17046 |
| iPS:394091 | | NA | CGGGCAAGTCAGGGCATTAG AAATGATTTAGGC | GCTGCATCCAGTTTACAA AGT | CTACAGCATAATAGTTACCC GCTCACT |
| | 21-225_13H3 | | SEQ ID NO:1023 | SEQ ID NO:9035 | SEQ ID NO:17047 |
| | | AA | RASQGIRNDLG | AASSLQS | LQHNSYPLT |
| | | | SEQ ID NO:1024 | SEQ ID NO:9036 | SEQ ID NO:17048 |
| iPS:394093 | | NA | CGGGCAAGTCAGGGCATTAG AAATGATTTAGGC | GCTGCATCCAGTTTGCAA AGT | CTACAGCATAATAGTTACCC GTGGACG |
| | 21-225_9D12 | | SEQ ID NO:1025 | SEQ ID NO:9037 | SEQ ID NO:17049 |
| | | AA | RASQGIRNDLG | AASSLQS | LQHNSYPWT |
| | | | SEQ ID NO:1026 | SEQ ID NO:9038 | SEQ ID NO:17050 |
| iPS:394095 | | NA | CGGGCAAGTCAGGGCATTAG AAATGATTTAGGC | ACTGCATCCAGTTTGCAA AGT | CTACAGCATAATAGTTACCC GTGGACG |
| | 21-225_16H4 | | SEQ ID NO:1027 | SEQ ID NO:9039 | SEQ ID NO:17051 |
| | | AA | RASQGIRNDLG | TASSLQS | LQHNSYPWT |

FIGURE 49

| | | | SEQ ID NO:1028 | SEQ ID NO:9040 | SEQ ID NO:17052 |
|---|---|---|---|---|---|
| iPS:394097 | | NA | CGGGCAAGTCAGGGCATTAG AAATGATTTAGGC | GCTGCATCCAGTTTGCAA AGT | CTACAACATAATAGTTACCC GTGGACG |
| | 21-225_16G7 | | SEQ ID NO:1029 | SEQ ID NO:9041 | SEQ ID NO:17053 |
| | | AA | RASQGIRNDLG | AASSLQS | LQHNSYPWT |
| | | | SEQ ID NO:1030 | SEQ ID NO:9042 | SEQ ID NO:17054 |
| iPS:398470 | | NA | TCTGGAGATAAATTGGGGAA TAAATATGCTTAC | CAAGATAGAAAGCGGCC CTCA | CAGGCGTGGAACAACAGCAC TGTGGTA |
| | 21-225_14B7 | | SEQ ID NO:1031 | SEQ ID NO:9043 | SEQ ID NO:17055 |
| | | AA | SGDKLGNKYAY | QDRKRPS | QAWNNSTVV |
| | | | SEQ ID NO:1032 | SEQ ID NO:9044 | SEQ ID NO:17056 |
| iPS:398472 | | NA | TCTGGAGATAAATTGGGGGA TAAATATGTTTAC | CAAGATAGCAAGCGGCC CTCA | CAGGCGTGGGACAGCAGCAC TGTGGTT |
| | 21-225_16E4 | | SEQ ID NO:1033 | SEQ ID NO:9045 | SEQ ID NO:17057 |
| | | AA | SGDKLGDKYVY | QDSKRPS | QAWDSSTVV |
| | | | SEQ ID NO:1034 | SEQ ID NO:9046 | SEQ ID NO:17058 |
| iPS:398474 | | NA | CGGTCAAGTCAGAGCATTAA CAGCTATTTAAAT | GCTGCATCCAGTTTGCAC AGT | CAACAGGGTTACAATACCCC CACGTGGACG |
| | 21-225_17B10 | | SEQ ID NO:1035 | SEQ ID NO:9047 | SEQ ID NO:17059 |
| | | AA | RSSQSINSYLN | AASSLHS | QQGYNTPTWT |
| | | | SEQ ID NO:1036 | SEQ ID NO:9048 | SEQ ID NO:17060 |
| iPS:398476 | | NA | CGGGCAAGTCAGAACATTAA CGACTATTTAAAT | GCTGCATCCAATTTGCAA AGT | CAACAGACTTACAATACCCC TCCGGAGCGCAGT |
| | 21-225_17C1 | | SEQ ID NO:1037 | SEQ ID NO:9049 | SEQ ID NO:17061 |
| | | AA | RASQNINDYLN | AASNLQS | QQTYNTPPERS |
| | | | SEQ ID NO:1038 | SEQ ID NO:9050 | SEQ ID NO:17062 |
| iPS:398478 | | NA | CGGGCGAGTCAGGGCATTAG CAATTATTTAGCC | GCTGCATCCACTTTGCAA TCA | CAAAAGTATAACAGTGCCCC TCCGCTCACC |
| | 21-225_17C10 | | SEQ ID NO:1039 | SEQ ID NO:9051 | SEQ ID NO:17063 |
| | | AA | RASQGISNYLA | AASTLQS | QKYNSAPPLT |

662

FIGURE 49

| | | | SEQ ID NO:1040 | SEQ ID NO:9052 | SEQ ID NO:17064 |
|---|---|---|---|---|---|
| iPS:398480 | | NA | CGGACAAGTCAGAATATTAG CAACTATTTAAAT | GTTGCGTCCAGTTTCCCA AGT | CAACAGAGTAACTTTTTCCC GCTCACT |
| | 21-225_17G4 | | SEQ ID NO:1041 | SEQ ID NO:9053 | SEQ ID NO:17065 |
| | | AA | RTSQNISNYLN | VASSFPS | QQSNFFPLT |
| | | | SEQ ID NO:1042 | SEQ ID NO:9054 | SEQ ID NO:17066 |
| iPS:398482 | | NA | CGGGCGAGTCGGGACATTAG CAATTATTTAGCC | ACTGCATCCAGTTTGCAA AGT | CAACAGTATCATAGTTACCC ATTCACT |
| | 21-225_17H6 | | SEQ ID NO:1043 | SEQ ID NO:9055 | SEQ ID NO:17067 |
| | | AA | RASRDISNYLA | TASSLQS | QQYHSYPFT |
| | | | SEQ ID NO:1044 | SEQ ID NO:9056 | SEQ ID NO:17068 |
| iPS:398484 | | NA | CGGGCAAGTCAGGGCATTAG AAATGATTTAGGC | GCTGCATCCAGTTTGGA AAGT | CTACATCATAATAATTACCT CCCCATCACC |
| | 21-225_18D4 | | SEQ ID NO:1045 | SEQ ID NO:9057 | SEQ ID NO:17069 |
| | | AA | RASQGIRNDLG | AASSLES | LHHNNYLPIT |
| | | | SEQ ID NO:1046 | SEQ ID NO:9058 | SEQ ID NO:17070 |
| iPS:398486 | | NA | CGGGCAAGTCATACCATTAC CAGCTATTTAAAT | GCTACATCCAATCTCCAA AGT | CAACAGAGTTACAACTTCCC GCTCACT |
| | 21-225_19A1 | | SEQ ID NO:1047 | SEQ ID NO:9059 | SEQ ID NO:17071 |
| | | AA | RASHTITSYLN | ATSNLQS | QQSYNFPLT |
| | | | SEQ ID NO:1048 | SEQ ID NO:9060 | SEQ ID NO:17072 |
| iPS:398488 | | NA | TCTGGAGATAAATTGGGGGA TAAATATGCTTGC | CAAGATAGCAAGCGGCC CTCA | CAGGCGTGGGACAACAACAC TGTGGTA |
| | 21-225_19F6 | | SEQ ID NO:1049 | SEQ ID NO:9061 | SEQ ID NO:17073 |
| | | AA | SGDKLGDKYAC | QDSKRPS | QAWDNNTVV |
| | | | SEQ ID NO:1050 | SEQ ID NO:9062 | SEQ ID NO:17074 |
| iPS:398490 | | NA | TCTGGAGATAAATTGGGGAA TAAATATGCTTAC | CAAGATAGAAAGAGGCC CTCA | CAGGCGTGGGACAACAGCAC TGTGGTA |
| | 21-225_21D12 | | SEQ ID NO:1051 | SEQ ID NO:9063 | SEQ ID NO:17075 |
| | | AA | SGDKLGNKYAY | QDRKRPS | QAWDNSTVV |

663

FIGURE 49

|  |  |  | SEQ ID NO:1052 | SEQ ID NO:9064 | SEQ ID NO:17076 |
|---|---|---|---|---|---|
| iPS:398492 |  | NA | CGGGCGAGTCAGGGCATTAG GAATTTTTTAGCC | GCTGCATCCAGTTTGCAA ACT | CAACAGTATAATAGTTTCCC ATTCACT |
|  | 21-225_21F12 |  | SEQ ID NO:1053 | SEQ ID NO:9065 | SEQ ID NO:17077 |
|  |  | AA | RASQGIRNFLA | AASSLQT | QQYNSFPFT |
|  |  |  | SEQ ID NO:1054 | SEQ ID NO:9066 | SEQ ID NO:17078 |
| iPS:398494 |  | NA | ACTGGAACCAGCAGTGACGT TGGTGGTTATAACTCTGTCT CC | GAGGTCAGTAATCGGCC CTCA | AGCTCATATACAAGGAGCAG CACTGTGGTA |
|  | 21-225_21H4 |  | SEQ ID NO:1055 | SEQ ID NO:9067 | SEQ ID NO:17079 |
|  |  | AA | TGTSSDVGGYNSVS | EVSNRPS | SSYTRSSTVV |
|  |  |  | SEQ ID NO:1056 | SEQ ID NO:9068 | SEQ ID NO:17080 |
| iPS:398496 |  | NA | AAGTCCAGCCAGAGTGTTTT ACACAGCTCCAACAATAACA ACTACTTAGCT | TGGGCATCTACCCGGAA ATCC | CAGCAATATTATAGTACTCC GTGCAGT |
|  | 21-225_22D2 |  | SEQ ID NO:1057 | SEQ ID NO:9069 | SEQ ID NO:17081 |
|  |  | AA | KSSQSVLHSSNNNNYLA | WASTRKS | QQYYSTPCS |
|  |  |  | SEQ ID NO:1058 | SEQ ID NO:9070 | SEQ ID NO:17082 |
| iPS:398498 |  | NA | TCTGGAGATAAATTGGGGGA GAAATATGCTTGC | CAAGATAGAAAGCGGCC CTCA | CAGGCGTGGGACAGCAGCAC TGCGGTA |
|  | 21-225_22E6 |  | SEQ ID NO:1059 | SEQ ID NO:9071 | SEQ ID NO:17083 |
|  |  | AA | SGDKLGEKYAC | QDRKRPS | QAWDSSTAV |
|  |  |  | SEQ ID NO:1060 | SEQ ID NO:9072 | SEQ ID NO:17084 |
| iPS:398500 |  | NA | CGGGCGAGTCAGGACATTAG CAATTATTTAGCC | GCTGCATCCACTTTGCAA AGT | CAACAGTATAATAGTTACCC ATTCACT |
|  | 21-225_23A11 |  | SEQ ID NO:1061 | SEQ ID NO:9073 | SEQ ID NO:17085 |
|  |  | AA | RASQDISNYLA | AASTLQS | QQYNSYPFT |
|  |  |  | SEQ ID NO:1062 | SEQ ID NO:9074 | SEQ ID NO:17086 |
| iPS:398502 |  | NA | CGGGCGAGTCAGGGTATTAC CAAGTGGTTAGCC | GCTGCATCCAGTTTGCAA AGT | CAACAGGCTAACAGTTTCCC ATTCACT |

FIGURE 49

| | 21-225_23B11 | | SEQ ID NO:1063 | SEQ ID NO:9075 | SEQ ID NO:17087 |
|---|---|---|---|---|---|
| | | AA | RASQGITKWLA | AASSLQS | QQANSFPFT |
| | | | SEQ ID NO:1064 | SEQ ID NO:9076 | SEQ ID NO:17088 |
| iPS:398504 | 21-225_23D7 | NA | TCTGGAGAAAAATTGGGGGATAAATATGTTTGT | CAAGATAGCAAGCGGCCCTCA | CAGGCGTGGAACAGCAGCAATGTGGTA |
| | | | SEQ ID NO:1065 | SEQ ID NO:9077 | SEQ ID NO:17089 |
| | | AA | SGEKLGDKYVC | QDSKRPS | QAWNSSNVV |
| | | | SEQ ID NO:1066 | SEQ ID NO:9078 | SEQ ID NO:17090 |
| iPS:398506 | 21-225_23G12 | NA | AAGTCCAGCCAGAGTATTTTATTCAGCTCCAACAATAACAACTACTTAGCT | TGGGCATCTACCCGGGAATCC | CAGCAATATTCTAGTACTCCGTGGACG |
| | | | SEQ ID NO:1067 | SEQ ID NO:9079 | SEQ ID NO:17091 |
| | | AA | KSSQSILFSSNNNNYLA | WASTRES | QQYSSTPWT |
| | | | SEQ ID NO:1068 | SEQ ID NO:9080 | SEQ ID NO:17092 |
| iPS:398508 | 21-225_24B1 | NA | AGGTCTAGTCAAAGCCTCGTATACAGTGATGGAAACACCTACTTGAAT | AAGGTTTCTAACTGGGACTCT | ATGCAAGGTGCACACTGGCCTCCGATCACC |
| | | | SEQ ID NO:1069 | SEQ ID NO:9081 | SEQ ID NO:17093 |
| | | AA | RSSQSLVYSDGNTYLN | KVSNWDS | MQGAHWPPIT |
| | | | SEQ ID NO:1070 | SEQ ID NO:9082 | SEQ ID NO:17094 |
| iPS:398510 | 21-225_25A3 | NA | AAGTCCAGCCAGAGTGTTTTATACAGCTCCAACAATAAGAACTACTTAGCT | TGGGCATCTACCCGGGAATCC | CAGCAATATTATAGTACTCCGTGCAGT |
| | | | SEQ ID NO:1071 | SEQ ID NO:9083 | SEQ ID NO:17095 |
| | | AA | KSSQSVLYSSNNKNYLA | WASTRES | QQYYSTPCS |
| | | | SEQ ID NO:1072 | SEQ ID NO:9084 | SEQ ID NO:17096 |
| iPS:398512 | 21-225_25E12 | NA | AAGTCCAGCCAGAGTGTTTTATACCACTCCAACAATTACAACTACTTAGCT | TGGGCATCTACCCGGGAGTCC | CAGCAATATTACAGTACTCCGTGCAGT |
| | | | SEQ ID NO:1073 | SEQ ID NO:9085 | SEQ ID NO:17097 |

FIGURE 49

| | | | | | |
|---|---|---|---|---|---|
| | | AA | KSSQSVLYHSNNYNYLA | WASTRES | QQYYSTPCS |
| | | | SEQ ID NO:1074 | SEQ ID NO:9086 | SEQ ID NO:17098 |
| iPS:398516 | 21-225_26A9 | NA | AAGTCCAGCCAGAGTGTTTT ATACAGCTCCAACAATAAGA ACTACTTAGCT | TGGGCATCTACCCGGGA ATCC | CAGCAATATTATAGTAGTCC GTGCAGT |
| | | | SEQ ID NO:1075 | SEQ ID NO:9087 | SEQ ID NO:17099 |
| | | AA | KSSQSVLYSSNNKNYLA | WASTRES | QQYYSSPCS |
| | | | SEQ ID NO:1076 | SEQ ID NO:9088 | SEQ ID NO:17100 |
| iPS:398520 | 21-225_31C4 | NA | CGGGCGAGTCAGGGTATTAG CAAATGGTTAGCC | GCTGCATCCAGTTTGCAG AGT | CAACAGGCTAACAGTTTCCC ATTCACT |
| | | | SEQ ID NO:1077 | SEQ ID NO:9089 | SEQ ID NO:17101 |
| | | AA | RASQGISKWLA | AASSLQS | QQANSFPFT |
| | | | SEQ ID NO:1078 | SEQ ID NO:9090 | SEQ ID NO:17102 |
| iPS:398522 | 21-225_32A1 | NA | AAGTCCAGCCAGAGTGTTTT ATACAGCTCCAACAATTACA ACTACTTAGCT | TGGGCATCTACCCGGAA ATCC | CAACAATATTATACTTCTCC GTGCAGT |
| | | | SEQ ID NO:1079 | SEQ ID NO:9091 | SEQ ID NO:17103 |
| | | AA | KSSQSVLYSSNNYNYLA | WASTRKS | QQYYTSPCS |
| | | | SEQ ID NO:1080 | SEQ ID NO:9092 | SEQ ID NO:17104 |
| iPS:398524 | 21-225_32A5 | NA | AAGTCCAGCCAGAGTGTTTT ACACAGCTCCAACAATAAGA ACTACTTAGCT | TGGGCATCTACCCGGGA ATCC | CAGCAATATTATAGTTCTCC GTGCAGT |
| | | | SEQ ID NO:1081 | SEQ ID NO:9093 | SEQ ID NO:17105 |
| | | AA | KSSQSVLHSSNNKNYLA | WASTRES | QQYYSSPCS |
| | | | SEQ ID NO:1082 | SEQ ID NO:9094 | SEQ ID NO:17106 |
| iPS:398526 | 21-225_32B3 | NA | CGGGCGAGTCAGGGCATTAG CAATTATTTAGCC | GCTGCATCCAGTTTACAA AGT | CAACAGTATAATAGTTATCC ATTCACT |
| | | | SEQ ID NO:1083 | SEQ ID NO:9095 | SEQ ID NO:17107 |
| | | AA | RASQGISNYLA | AASSLQS | QQYNSYPFT |
| | | | SEQ ID NO:1084 | SEQ ID NO:9096 | SEQ ID NO:17108 |

FIGURE 49

| iPS:398528 | | NA | CGGGCAAGTCAGGACATGA GAAGTGATTTAGGC | GCTGCATCCAGTTTGCAA AGT | CTACAGCATACTATTTCCCCT CCTACT |
|---|---|---|---|---|---|
| | 21-225_32G1 | | SEQ ID NO:1085 | SEQ ID NO:9097 | SEQ ID NO:17109 |
| | | AA | RASQDMRSDLG | AASSLQS | LQHTISPPT |
| | | | SEQ ID NO:1086 | SEQ ID NO:9098 | SEQ ID NO:17110 |
| iPS:398530 | | NA | AGGTCAAGTCAAAGCCTCGT ATACAGTGATGGAAACACCT ACTTGAAT | AAGGTTTCTAACTGGGA CTCT | ATGCAAGGTATACACTGGCT CACT |
| | 21-225_32G4 | | SEQ ID NO:1087 | SEQ ID NO:9099 | SEQ ID NO:17111 |
| | | AA | RSSQSLVYSDGNTYLN | KVSNWDS | MQGIHWLT |
| | | | SEQ ID NO:1088 | SEQ ID NO:9100 | SEQ ID NO:17112 |
| iPS:398532 | | NA | CGGGCGAGTCAGGACATTAG CAATTATTTGGCC | GCTGCATCTAGTTTGCAA AGT | CAACAGTATCATAGTTACCC GCTCACC |
| | 21-225_33B7 | | SEQ ID NO:1089 | SEQ ID NO:9101 | SEQ ID NO:17113 |
| | | AA | RASQDISNYLA | AASSLQS | QQYHSYPLT |
| | | | SEQ ID NO:1090 | SEQ ID NO:9102 | SEQ ID NO:17114 |
| iPS:398534 | | NA | CGGGCAAGTCAGGACATTAG AAGTGATTTAGGC | GCTGCATCCAGTTTGCAA AGT | CTACAGCATACTATTTACCC TCCTACT |
| | 21-225_33B8 | | SEQ ID NO:1091 | SEQ ID NO:9103 | SEQ ID NO:17115 |
| | | AA | RASQDIRSDLG | AASSLQS | LQHTIYPPT |
| | | | SEQ ID NO:1092 | SEQ ID NO:9104 | SEQ ID NO:17116 |
| iPS:398536 | | NA | CGGGCAAGTCAGAGCATTAG AAGCTATTTAAAT | AGTGCATCCAGTTTGCA AAGT | CAACAGAGTTACAGTATCCC GATCACC |
| | 21-225_33D12 | | SEQ ID NO:1093 | SEQ ID NO:9105 | SEQ ID NO:17117 |
| | | AA | RASQSIRSYLN | SASSLQS | QQSYSIPIT |
| | | | SEQ ID NO:1094 | SEQ ID NO:9106 | SEQ ID NO:17118 |
| iPS:398538 | | NA | AAGTCCAGCCAGAGTGTTTT ATACAGCTCCAACAATTACA ACTACTTAGCT | TGGGCATCTACCCGGAA ATCC | CAGCAATATTATACTTCTCC GTGCAGT |
| | 21-225_34H7 | | SEQ ID NO:1095 | SEQ ID NO:9107 | SEQ ID NO:17119 |

FIGURE 49

| | | | | | |
|---|---|---|---|---|---|
| | | AA | KSSQSVLYSSNNYNYLA | WASTRKS | QQYYTSPCS |
| | | | SEQ ID NO:1096 | SEQ ID NO:9108 | SEQ ID NO:17120 |
| iPS:398540 | | NA | CGGGCAAGTCAGGACATTAGAAGTGATTTAGGC | GCTACATCCAGTTTGCAAAGT | CTACAGCATACTATTTACCCTCCTACT |
| | 21-225_35A6 | | SEQ ID NO:1097 | SEQ ID NO:9109 | SEQ ID NO:17121 |
| | | AA | RASQDIRSDLG | ATSSLQS | LQHTIYPPT |
| | | | SEQ ID NO:1098 | SEQ ID NO:9110 | SEQ ID NO:17122 |
| iPS:398544 | | NA | ACCCTAAGCAGTGAGCACAGCACCTACACCATCGAA | GTTAAGAGTGATGGCAGCCACAGCAAGGGGGAC | GGAGAGAGCCACCCGATTGATGGCCAAGTCGGTGTGGTA |
| | 21-225_7C8 | | SEQ ID NO:1099 | SEQ ID NO:9111 | SEQ ID NO:17123 |
| | | AA | TLSSEHSTYTIE | VKSDGSHSKGD | GESHPIDGQVGVV |
| | | | SEQ ID NO:1100 | SEQ ID NO:9112 | SEQ ID NO:17124 |
| iPS:398546 | | NA | TCTGGAGATAAATTGGGGGATAAATATGCTTGC | CAAGATAGCAAGCGGCCCTCA | CAGGCGTGGGACAGCAGCACTTATGTGGTA |
| | 21-225_9H10 | | SEQ ID NO:1101 | SEQ ID NO:9113 | SEQ ID NO:17125 |
| | | AA | SGDKLGDKYAC | QDSKRPS | QAWDSSTYVV |
| | | | SEQ ID NO:1102 | SEQ ID NO:9114 | SEQ ID NO:17126 |
| iPS:402219 | | NA | CGGGCAAGTCAGGGCATTAGAAATGATTTAGGC | GCTGCATCCAGTTTGCAAAGT | CTACAACATAATAGTTACCCGCTCACT |
| | 21-225_1C12 | | SEQ ID NO:1103 | SEQ ID NO:9115 | SEQ ID NO:17127 |
| | | AA | RASQGIRNDLG | AASSLQS | LQHNSYPLT |
| | | | SEQ ID NO:1104 | SEQ ID NO:9116 | SEQ ID NO:17128 |
| iPS:402221 | | NA | CGGGCGAGTCAGGGCATTAGCAATTATTTAGCC | GCTGCAACCAGTTTGCAAAGT | CAACAGTATTATAGTTACCCGATCACC |
| | 21-225_2C12 | | SEQ ID NO:1105 | SEQ ID NO:9117 | SEQ ID NO:17129 |
| | | AA | RASQGISNYLA | AATSLQS | QQYYSYPIT |
| | | | SEQ ID NO:1106 | SEQ ID NO:9118 | SEQ ID NO:17130 |
| iPS:402223 | | NA | CGGGCGAGTCAGGGTATTAGCAGATGGTTAGCC | GCTGCATCCCGTTTGCAAAGT | CAACAGGCTAACAGTTTCCCATTCACT |

FIGURE 49

| | | | | | |
|---|---|---|---|---|---|
| | **21-225_30A11** | | SEQ ID NO:1107 | SEQ ID NO:9119 | SEQ ID NO:17131 |
| | | AA | RASQGISRWLA | AASRLQS | QQANSFPFT |
| | | | SEQ ID NO:1108 | SEQ ID NO:9120 | SEQ ID NO:17132 |
| **iPS:402225** | | NA | TCTGGAGATAAATTGGGGGATAAATATGCTTGC | CAAGATCGCAAGCGGCCCTCA | CAGGCGTGGGACAACAACACTGTGGTA |
| | **21-225_2B1** | | SEQ ID NO:1109 | SEQ ID NO:9121 | SEQ ID NO:17133 |
| | | AA | SGDKLGDKYAC | QDRKRPS | QAWDNNTVV |
| | | | SEQ ID NO:1110 | SEQ ID NO:9122 | SEQ ID NO:17134 |
| **iPS:402229** | | NA | CGGGCAAGTCAGGGCATTAGAAATTATTTAGGC | GGTGCATCCAGTTTGCAAAGT | CTACAGTATCATAGTTATCTATTCACT |
| | **21-225_16H9** | | SEQ ID NO:1111 | SEQ ID NO:9123 | SEQ ID NO:17135 |
| | | AA | RASQGIRNYLG | GASSLQS | LQYHSYLFT |
| | | | SEQ ID NO:1112 | SEQ ID NO:9124 | SEQ ID NO:17136 |
| **iPS:402231** | | NA | TCTGGAGATAAATTGGGGGAAAAATATGCTTGC | CAAGATAAGAAGCGGCCCTCA | CAGGCGTGGGACAGCAGCACTGTA |
| | **21-225_6D9** | | SEQ ID NO:1113 | SEQ ID NO:9125 | SEQ ID NO:17137 |
| | | AA | SGDKLGEKYAC | QDKKRPS | QAWDSSTV |
| | | | SEQ ID NO:1114 | SEQ ID NO:9126 | SEQ ID NO:17138 |
| **iPS:402233** | | NA | CGGGCGAGTCAGGACATAAGTAATTATTTAGCC | GCTACACCCAGTTTGCAGAGT | CAACAGTATAATAGTTACCCGCTCACT |
| | **21-225_16D10** | | SEQ ID NO:1115 | SEQ ID NO:9127 | SEQ ID NO:17139 |
| | | AA | RASQDISNYLA | ATPSLQS | QQYNSYPLT |
| | | | SEQ ID NO:1116 | SEQ ID NO:9128 | SEQ ID NO:17140 |
| **iPS:402235** | | NA | CGGGCGAGTCAGGGCATTAACAATTATTTAGCC | GCTGCATCCAGTTTGCAAAGT | CAGCAATATAATAGTTACCCATTCACT |
| | **21-225_20F10** | | SEQ ID NO:1117 | SEQ ID NO:9129 | SEQ ID NO:17141 |
| | | AA | RASQGINNYLA | AASSLQS | QQYNSYPFT |
| | | | SEQ ID NO:1118 | SEQ ID NO:9130 | SEQ ID NO:17142 |
| **iPS:402237** | | NA | CGGGCGAGTCAGGGCATTGCCAATTATTTAGCC | GCTGCATCCAGTTTGCAAAGT | CAACAGTATCATAGTTACCCGCTCACT |

FIGURE 49

| | | | | | |
|---|---|---|---|---|---|
| | 21-225_23D11 | | SEQ ID NO:1119 | SEQ ID NO:9131 | SEQ ID NO:17143 |
| | | AA | RASQGIANYLA | AASSLQS | QQYHSYPLT |
| | | | SEQ ID NO:1120 | SEQ ID NO:9132 | SEQ ID NO:17144 |
| iPS:403868 | | NA | CGGGCAAGTCAGGGCATTAG AAATGATTTAGGC | GCTACATCCAGTTTGCAA AGT | CTACAGTATTATAGTTACCC GCTCACT |
| | 21-225_19D11 | | SEQ ID NO:1121 | SEQ ID NO:9133 | SEQ ID NO:17145 |
| | | AA | RASQGIRNDLG | ATSSLQS | LQYYSYPLT |
| | | | SEQ ID NO:1122 | SEQ ID NO:9134 | SEQ ID NO:17146 |
| iPS:403870 | | NA | CGGGCAAGTCAGAACATTTA CAGCTATTTAAAT | GCTGCATCCAGTTTGCAA AGT | CAACAGAGTTACAATACCCC TCCGGAGTGCAAT |
| | 21-225_23G4 | | SEQ ID NO:1123 | SEQ ID NO:9135 | SEQ ID NO:17147 |
| | | AA | RASQNIYSYLN | AASSLQS | QQSYNTPPECN |
| | | | SEQ ID NO:1124 | SEQ ID NO:9136 | SEQ ID NO:17148 |
| iPS:403872 | | NA | CGGGCAAGTCAGGGCATTAG GAGTGATTTAGGC | GATGCATCCAGTGTGCA AAGT | CTACAACATTATACTTACCC GCTCACT |
| | 21-225_8F11 | | SEQ ID NO:1125 | SEQ ID NO:9137 | SEQ ID NO:17149 |
| | | AA | RASQGIRSDLG | DASSVQS | LQHYTYPLT |
| | | | SEQ ID NO:1126 | SEQ ID NO:9138 | SEQ ID NO:17150 |
| iPS:404090 | | NA | TCTGGAGATAAATTGGGGGA GAAATATGCTTGC | CAAGATAGGAAGCGGCC CTCA | CAGGCGTGGGACAGCAGCAC TGCGGTA |
| | 21-225_8D8 | | SEQ ID NO:1127 | SEQ ID NO:9139 | SEQ ID NO:17151 |
| | | AA | SGDKLGEKYAC | QDRKRPS | QAWDSSTAV |
| | | | SEQ ID NO:1128 | SEQ ID NO:9140 | SEQ ID NO:17152 |
| iPS:412232 | | NA | AAGTCCAGCCAGAGTATTTT ACACAGCTCCAACAATAACA ACTACTTAGCT | TGGGCATCTACCCGGGA ATCC | CAGCAATATTATAATACTCC AGTCACT |
| | 21-225_4A2 | | SEQ ID NO:1129 | SEQ ID NO:9141 | SEQ ID NO:17153 |
| | | AA | KSSQSILHSSNNNNYLA | WASTRES | QQYYNTPVT |
| | | | SEQ ID NO:1130 | SEQ ID NO:9142 | SEQ ID NO:17154 |

670

FIGURE 49

| iPS:422894 | 21-225_4A2.001 | NA | AAGTCCAGCCAGAGTATTTT ACACAGCTCCAACAATAACA ACTACTTAGCT | TGGGCATCTACCCGGGA ATCC | CAGCAATATTATAATACTCC AGTCACT |
|---|---|---|---|---|---|
| | | | SEQ ID NO:1131 | SEQ ID NO:9143 | SEQ ID NO:17155 |
| | | AA | KSSQSILHSSNNNNYLA | WASTRES | QQYYNTPVT |
| | | | SEQ ID NO:1132 | SEQ ID NO:9144 | SEQ ID NO:17156 |
| iPS:423018 | 21-225_31D12_LC2 | NA | TCTGGAGATAAATTGGGGGA TAAATATGCTTAC | CAAGATAGGAAGCGGCC CTCA | CAGGCGTGGGACAACAGCAC TGCGGTA |
| | | | SEQ ID NO:1133 | SEQ ID NO:9145 | SEQ ID NO:17157 |
| | | AA | SGDKLGDKYAY | QDRKRPS | QAWDNSTAV |
| | | | SEQ ID NO:1134 | SEQ ID NO:9146 | SEQ ID NO:17158 |
| iPS:423019 | 21-225_31D12_LC1 | NA | AGGTCTAGTCAAAGCCTCAT ATACAGTGATGGAAACACCT TCTTGAAT | AAGGTTTCTAATTGGGA CTCT | ATGCAAGGTACACACTGGCC TCTCACC |
| | | | SEQ ID NO:1135 | SEQ ID NO:9147 | SEQ ID NO:17159 |
| | | AA | RSSQSLIYSDGNTFLN | KVSNWDS | MQGTHWPLT |
| | | | SEQ ID NO:1136 | SEQ ID NO:9148 | SEQ ID NO:17160 |
| iPS:423314 | 21-225_12F11 | NA | AAGTCCAGCCAGAGTGTTTT ACACAGCTCCAACAATTATA ACTACTTAGCT | TGGGCATCTACCCGGGA ATCC | CAGCAATATTATGATACTCC ATTCACT |
| | | | SEQ ID NO:1137 | SEQ ID NO:9149 | SEQ ID NO:17161 |
| | | AA | KSSQSVLHSSNNYNYLA | WASTRES | QQYYDTPFT |
| | | | SEQ ID NO:1138 | SEQ ID NO:9150 | SEQ ID NO:17162 |
| iPS:424419 | 21-225_25A4.001 | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | | | SEQ ID NO:1139 | SEQ ID NO:9151 | SEQ ID NO:17163 |
| | | AA | KSSQSVLYSSHNNNYLA | WASTRES | QQYYSTPPT |
| | | | SEQ ID NO:1140 | SEQ ID NO:9152 | SEQ ID NO:17164 |

FIGURE 49

| iPS:424460 | | NA | CGGGCAAGTCAAAACATTAT CAGCTATTTAAAT | ACTGCATCCAGTTTGCAA AGT | CAACAGACTTACAGTACCCC GCTCACT |
|---|---|---|---|---|---|
| | 21-225_7E11.001 | | SEQ ID NO:1141 | SEQ ID NO:9153 | SEQ ID NO:17165 |
| | | AA | RASQNIISYLN | TASSLQS | QQTYSTPLT |
| | | | SEQ ID NO:1142 | SEQ ID NO:9154 | SEQ ID NO:17166 |
| iPS:426108 | | NA | CGGGCGAGTCAGGGTATTAG CAAATGGTTAGCC | GCTGCATATAGTTTACAA AGT | CAACAGGCTAACAGTTTCCC ATTCACT |
| | 21-225_10G6 | | SEQ ID NO:1143 | SEQ ID NO:9155 | SEQ ID NO:17167 |
| | | AA | RASQGISKWLA | AAYSLQS | QQANSFPFT |
| | | | SEQ ID NO:1144 | SEQ ID NO:9156 | SEQ ID NO:17168 |
| iPS:426110 | | NA | CGGGCGAGTCAGGGTATTAG CAGCTGGTTAGCC | GCTGCATCCAGGTTGCA AAGT | CAACAGGCTAACAGTTTCCC ATTCACT |
| | 21-225_12E9 | | SEQ ID NO:1145 | SEQ ID NO:9157 | SEQ ID NO:17169 |
| | | AA | RASQGISSWLA | AASRLQS | QQANSFPFT |
| | | | SEQ ID NO:1146 | SEQ ID NO:9158 | SEQ ID NO:17170 |
| iPS:426112 | | NA | AAGTCCAGCCAGACTGTTTT ATTCAGCTCCAACAATAACC ACTACTTAGCA | TGGGCATCTACCCGGGC ATCC | CAGCAATATTATAGTAGTCC GTGGACG |
| | 21-225_12F12 | | SEQ ID NO:1147 | SEQ ID NO:9159 | SEQ ID NO:17171 |
| | | AA | KSSQTVLFSSNNNHYLA | WASTRAS | QQYYSSPWT |
| | | | SEQ ID NO:1148 | SEQ ID NO:9160 | SEQ ID NO:17172 |
| iPS:426114 | | NA | CGGGCAAGTCAGGGCATTAG AAATGATTTAGGC | GCTGCATCCAGTTTGCAA AGT | CTACAACATTATAGTTACCC TCGCAGT |
| | 21-225_28H2 | | SEQ ID NO:1149 | SEQ ID NO:9161 | SEQ ID NO:17173 |
| | | AA | RASQGIRNDLG | AASSLQS | LQHYSYPRS |
| | | | SEQ ID NO:1150 | SEQ ID NO:9162 | SEQ ID NO:17174 |
| iPS:426116 | | NA | CGGGCAAGTCAGGCCATTAG AAATGATTTAGGC | GCTGCATCCAGTTTGCAA AGT | TTACAGCATTATAATTACCC TCGCAGT |
| | 21-225_29E2 | | SEQ ID NO:1151 | SEQ ID NO:9163 | SEQ ID NO:17175 |
| | | AA | RASQAIRNDLG | AASSLQS | LQHYNYPRS |

FIGURE 49

| | | | SEQ ID NO:1152 | SEQ ID NO:9164 | SEQ ID NO:17176 |
|---|---|---|---|---|---|
| iPS:426118 | | NA | CGGGCAAGTCAGAACATTTACAGCTATTTAAAT | TCTACATCCAGTTTGCAAAGT | CAACAGAGTTACAGTCCCCCTCTCACT |
| | 21-225_7A10 | | SEQ ID NO:1153 | SEQ ID NO:9165 | SEQ ID NO:17177 |
| | | AA | RASQNIYSYLN | STSSLQS | QQSYSPPLT |
| | | | SEQ ID NO:1154 | SEQ ID NO:9166 | SEQ ID NO:17178 |
| iPS:426124 | | NA | CGGGCAAGTCAGAACATTATCAGCTATTTAAAT | GTTGCATCCCGTTTGCAAAGT | CAACAGAGTTACAGTACCCCGTACACT |
| | 21-225_32D6 | | SEQ ID NO:1155 | SEQ ID NO:9167 | SEQ ID NO:17179 |
| | | AA | RASQNIISYLN | VASRLQS | QQSYSTPYT |
| | | | SEQ ID NO:1156 | SEQ ID NO:9168 | SEQ ID NO:17180 |
| iPS:426126 | | NA | AAGTCCAGCCAGAGTGTTTTACACAACTCCAACAATTATAACTATTTAGCT | TGGGCATCTACCCGGGAATCC | CAGCAATATTATGATACTCCATTCACT |
| | 21-225_6G6 | | SEQ ID NO:1157 | SEQ ID NO:9169 | SEQ ID NO:17181 |
| | | AA | KSSQSVLHNSNNYNYLA | WASTRES | QQYYDTPFT |
| | | | SEQ ID NO:1158 | SEQ ID NO:9170 | SEQ ID NO:17182 |
| iPS:433895 | | NA | CGGGCAAGTCAGGGCATTAGAAATGATTTAGGC | GCTGCATCCAGTTTGCAAAGT | CTACAGCATAATAGTTACCCATTCACT |
| | 21-225_43E1 | | SEQ ID NO:1159 | SEQ ID NO:9171 | SEQ ID NO:17183 |
| | | AA | RASQGIRNDLG | AASSLQS | LQHNSYPFT |
| | | | SEQ ID NO:1160 | SEQ ID NO:9172 | SEQ ID NO:17184 |
| iPS:433897 | | NA | CGGGCGAGTCAGGGTATTAGCGACTGGTTAGCC | GCTGCATCCAGTTTGCAAAGT | CAACAGACTAACAGTTTCCCGTGGACG |
| | 21-225_43C2 | | SEQ ID NO:1161 | SEQ ID NO:9173 | SEQ ID NO:17185 |
| | | AA | RASQGISDWLA | AASSLQS | QQTNSFPWT |
| | | | SEQ ID NO:1162 | SEQ ID NO:9174 | SEQ ID NO:17186 |
| iPS:433899 | | NA | CGGGCAAGTCAGGGCATTAGAAATGATTTAGGC | GCTGCATCCAGTTTGCAAAGT | CTACAGCATAATAGTTACCCTCTCACT |
| | 21-225_43C3 | | SEQ ID NO:1163 | SEQ ID NO:9175 | SEQ ID NO:17187 |

FIGURE 49

| | | AA | RASQGIRNDLG | AASSLQS | LQHNSYPLT |
|---|---|---|---|---|---|
| | | | SEQ ID NO:1164 | SEQ ID NO:9176 | SEQ ID NO:17188 |
| iPS:433901 | | NA | CGGGCGAGTCAGGGCATTAACAATTATTTAGCC | GCTGCATCCAGTTTGCAAAGT | CAACAGTATTATAGTTACCCATTCACT |
| | 21-225_43A4 | | SEQ ID NO:1165 | SEQ ID NO:9177 | SEQ ID NO:17189 |
| | | AA | RASQGINNYLA | AASSLQS | QQYYSYPFT |
| | | | SEQ ID NO:1166 | SEQ ID NO:9178 | SEQ ID NO:17190 |
| iPS:433903 | | NA | CGGGCGAGTCAGGGTATTATCAACTGGTTAGCC | GCTGCATCCAGTTTGCAAAGT | CAACAGACTAACAGTTTCCCGTGGACG |
| | 21-225_43H4 | | SEQ ID NO:1167 | SEQ ID NO:9179 | SEQ ID NO:17191 |
| | | AA | RASQGIINWLA | AASSLQS | QQTNSFPWT |
| | | | SEQ ID NO:1168 | SEQ ID NO:9180 | SEQ ID NO:17192 |
| iPS:433905 | | NA | CGGGCAAGTCAGGGCATTAGAAATGATTTAGGC | GGTGCATCCAATTTGCAAAGT | CTACAGCATACTAGTTTCCCATTCACT |
| | 21-225_43E5 | | SEQ ID NO:1169 | SEQ ID NO:9181 | SEQ ID NO:17193 |
| | | AA | RASQGIRNDLG | GASNLQS | LQHTSFPFT |
| | | | SEQ ID NO:1170 | SEQ ID NO:9182 | SEQ ID NO:17194 |
| iPS:433909 | | NA | AAGTCCAGCCAGAGTGTTTTAATGACCTCCAACGATAAGAACTACTTAACT | TGGGCTTCTACCCGGGAATCC | CAGCAATATTATAGTACTCCTCCGACG |
| | 21-225_43D8 | | SEQ ID NO:1171 | SEQ ID NO:9183 | SEQ ID NO:17195 |
| | | AA | KSSQSVLMTSNDKNYLT | WASTRES | QQYYSTPPT |
| | | | SEQ ID NO:1172 | SEQ ID NO:9184 | SEQ ID NO:17196 |
| iPS:433911 | | NA | CGGGCGAGTCAGGGTATTAGCAACTGGTTAGCC | GCTGCATCCAGTTTGCAAAGT | CAACAGACTAACAGTTTCCCGTGGACG |
| | 21-225_43E8 | | SEQ ID NO:1173 | SEQ ID NO:9185 | SEQ ID NO:17197 |
| | | AA | RASQGISNWLA | AASSLQS | QQTNSFPWT |
| | | | SEQ ID NO:1174 | SEQ ID NO:9186 | SEQ ID NO:17198 |
| iPS:433913 | | NA | CGGGCAAGTCAGGGCATTAGAAATGATTTAGGC | GCTGCATCCAGTTTGCAAAGT | CTACAGCATAATAGTTTCCCATTCACT |

FIGURE 49

| | 21-225_43H8 | | SEQ ID NO:1175 | SEQ ID NO:9187 | SEQ ID NO:17199 |
|---|---|---|---|---|---|
| | | AA | RASQGIRNDLG | AASSLQS | LQHNSFPFT |
| | | | SEQ ID NO:1176 | SEQ ID NO:9188 | SEQ ID NO:17200 |
| iPS:433915 | | NA | CGGGCGAGTCAGGATATTAG CAGCTGGTTAGCC | GATGCATCCAGTTTGCA AAGT | CAACAGGCTAACAGTCTCCC ATTCACT |
| | 21-225_43H9 | | SEQ ID NO:1177 | SEQ ID NO:9189 | SEQ ID NO:17201 |
| | | AA | RASQDISSWLA | DASSLQS | QQANSLPFT |
| | | | SEQ ID NO:1178 | SEQ ID NO:9190 | SEQ ID NO:17202 |
| iPS:433917 | | NA | AAGTCTAGTCAGAGCCTCCT GCACAGTGATGGAAGGACCT ATTTGTAT | GAAGTTTCCAACCGGTTC TCT | ATGCAAAGTATACAGCTTCC GTGGACG |
| | 21-225_43E11 | | SEQ ID NO:1179 | SEQ ID NO:9191 | SEQ ID NO:17203 |
| | | AA | KSSQSLLHSDGRTYLY | EVSNRFS | MQSIQLPWT |
| | | | SEQ ID NO:1180 | SEQ ID NO:9192 | SEQ ID NO:17204 |
| iPS:433919 | | NA | CGGGCAAGTCAGGGCATTAG AAATGATTTAGGC | GCTGCATCCAGTTTGCAA AGT | CTACTGCATTATAATTACCCT CGGACG |
| | 21-225_44B3 | | SEQ ID NO:1181 | SEQ ID NO:9193 | SEQ ID NO:17205 |
| | | AA | RASQGIRNDLG | AASSLQS | LLHYNYPRT |
| | | | SEQ ID NO:1182 | SEQ ID NO:9194 | SEQ ID NO:17206 |
| iPS:433921 | | NA | CGGGCAAGTCAGGGCATTAG AAATGATTTAGGC | GCTGCATCCAGTTTGCAA AGT | CTACAGCATAGTAGTTACCC TCTCACT |
| | 21-225_44C3 | | SEQ ID NO:1183 | SEQ ID NO:9195 | SEQ ID NO:17207 |
| | | AA | RASQGIRNDLG | AASSLQS | LQHSSYPLT |
| | | | SEQ ID NO:1184 | SEQ ID NO:9196 | SEQ ID NO:17208 |
| iPS:433923 | | NA | CGGGCAAGTCAGGGCATTAG AGATGATTTAGGC | GCTGCATCCAGTTTGCAA AGT | CTACAGCATTATAGTTACCC TCGGACG |
| | 21-225_44D3 | | SEQ ID NO:1185 | SEQ ID NO:9197 | SEQ ID NO:17209 |
| | | AA | RASQGIRDDLG | AASSLQS | LQHYSYPRT |
| | | | SEQ ID NO:1186 | SEQ ID NO:9198 | SEQ ID NO:17210 |

FIGURE 49

| iPS:433925 | | NA | CGGGCGAGTCAGGGTATTAG CGACTGGTTAGCC | GCTGCATCCAGTTTACAA AGT | CAACAGGCTAACAGTTTCCC ATTCACT |
|---|---|---|---|---|---|
| | 21-225_44F3 | | SEQ ID NO:1187 | SEQ ID NO:9199 | SEQ ID NO:17211 |
| | | AA | RASQGISDWLA | AASSLQS | QQANSFPFT |
| | | | SEQ ID NO:1188 | SEQ ID NO:9200 | SEQ ID NO:17212 |
| iPS:433929 | | NA | CGGGCAAGTCAGGACATTAG AAAAGATTTAGGC | GCTGCATCCACTTTGGAA AGT | CTACAGCATTATAGTTTCCC GTGGACG |
| | 21-225_44D5 | | SEQ ID NO:1189 | SEQ ID NO:9201 | SEQ ID NO:17213 |
| | | AA | RASQDIRKDLG | AASTLES | LQHYSFPWT |
| | | | SEQ ID NO:1190 | SEQ ID NO:9202 | SEQ ID NO:17214 |
| iPS:433931 | | NA | AGGGCCAGTCAGAGTGTTAG TGGAAGCTACTTAGCC | GGTGCATCCAGCAGGGC CACT | CAGCAGTATGGTAGTTCACC GTGGACG |
| | 21-225_44F6 | | SEQ ID NO:1191 | SEQ ID NO:9203 | SEQ ID NO:17215 |
| | | AA | RASQSVSGSYLA | GASSRAT | QQYGSSPWT |
| | | | SEQ ID NO:1192 | SEQ ID NO:9204 | SEQ ID NO:17216 |
| iPS:433933 | | NA | CGGGCAAGTCAGGGCATTAG AAATGATTTAGGC | GCTGCATCCAATTTGCAA AGT | CTACAGCATAATAGTTACCC ATTCACT |
| | 21-225_44C8 | | SEQ ID NO:1193 | SEQ ID NO:9205 | SEQ ID NO:17217 |
| | | AA | RASQGIRNDLG | AASNLQS | LQHNSYPFT |
| | | | SEQ ID NO:1194 | SEQ ID NO:9206 | SEQ ID NO:17218 |
| iPS:433935 | | NA | CGGGCAAGTCAGGGCATTAG AAATGATTTAGGC | GCTGCATCCAGTTTGCAA AGT | CTCCACCATTATAATTACCCT CGGACG |
| | 21-225_44F9 | | SEQ ID NO:1195 | SEQ ID NO:9207 | SEQ ID NO:17219 |
| | | AA | RASQGIRNDLG | AASSLQS | LHHYNYPRT |
| | | | SEQ ID NO:1196 | SEQ ID NO:9208 | SEQ ID NO:17220 |
| iPS:433937 | | NA | AAGTCTAGTCAGAGCCTCCT GCATAGTGAGGGAAGGACC TATTTGTAT | GAAATTTCCCACCGGTTC TCT | ATGCAAAGTATCCACCTTCC GTTCACT |
| | 21-225_44B10 | | SEQ ID NO:1197 | SEQ ID NO:9209 | SEQ ID NO:17221 |
| | | AA | KSSQSLLHSEGRTYLY | EISHRFS | MQSIHLPFT |

676

FIGURE 49

| | | | SEQ ID NO:1198 | SEQ ID NO:9210 | SEQ ID NO:17222 |
|---|---|---|---|---|---|
| iPS:433939 | | NA | CGGGCAAGTCAGGGCATTAG AGATGATTTAGGC | GCTACATCCAGTTTGCAA AGT | CTACAGCATTATAGTTACCC TCGGACG |
| | 21-225_44C10 | | SEQ ID NO:1199 | SEQ ID NO:9211 | SEQ ID NO:17223 |
| | | AA | RASQGIRDDLG | ATSSLQS | LQHYSYPRT |
| | | | SEQ ID NO:1200 | SEQ ID NO:9212 | SEQ ID NO:17224 |
| iPS:433941 | | NA | CGGGCGAGTCAGGGTATTAG CGACTGGTTAGCC | GCTGCATCCAGTTTGCAA AGT | CAACAGACTAACAGTTTCCC GTGGACG |
| | 21-225_44D10 | | SEQ ID NO:1201 | SEQ ID NO:9213 | SEQ ID NO:17225 |
| | | AA | RASQGISDWLA | AASSLQS | QQTNSFPWT |
| | | | SEQ ID NO:1202 | SEQ ID NO:9214 | SEQ ID NO:17226 |
| iPS:433943 | | NA | AGGTCTAGTCAGAGCCTCCT GCATAGTAATGGATACAGCT ATTTGGAG | TTGGGTTCTAATCGGGCC TCC | ATGCAAACTCTACAAACTCC ATTCACT |
| | 21-225_44E10 | | SEQ ID NO:1203 | SEQ ID NO:9215 | SEQ ID NO:17227 |
| | | AA | RSSQSLLHSNGYSYLE | LGSNRAS | MQTLQTPFT |
| | | | SEQ ID NO:1204 | SEQ ID NO:9216 | SEQ ID NO:17228 |
| iPS:433945 | | NA | CGGGCGAGTCAGGGTATTAG CAACTGGTTAGCC | GCTGCATTCAGTTTGCAA AGT | CAACAGTCTAACAGTTTCCC GTGGACG |
| | 21-225_44C12 | | SEQ ID NO:1205 | SEQ ID NO:9217 | SEQ ID NO:17229 |
| | | AA | RASQGISNWLA | AAFSLQS | QQSNSFPWT |
| | | | SEQ ID NO:1206 | SEQ ID NO:9218 | SEQ ID NO:17230 |
| iPS:433947 | | NA | CGGACAAGTCAGGGCATTAG AAATGATTTAGGC | GCTGCATCCAGTTTGCAA AGT | CTACAACATAATAGTTACCC GCTCACT |
| | 21-225_44E12 | | SEQ ID NO:1207 | SEQ ID NO:9219 | SEQ ID NO:17231 |
| | | AA | RTSQGIRNDLG | AASSLQS | LQHNSYPLT |
| | | | SEQ ID NO:1208 | SEQ ID NO:9220 | SEQ ID NO:17232 |
| iPS:433949 | | NA | CGGGCAAGTCAGGGCATTAG AAATGATTTAGGC | GGTGCATCCAATTTGCA AAGT | CTACAGCATACTAGTTTCCC ATTCACT |
| | 21-225_45H2 | | SEQ ID NO:1209 | SEQ ID NO:9221 | SEQ ID NO:17233 |

677

FIGURE 49

| | | | | | |
|---|---|---|---|---|---|
| | | AA | RASQGIRNDLG | GASNLQS | LQHTSFPFT |
| | | | SEQ ID NO:1210 | SEQ ID NO:9222 | SEQ ID NO:17234 |
| iPS:433951 | | NA | CGGGCAAGTCAGGGCATTAGAGATGATTTAGGC | GCTGCATCCAGTTTGCAAAGT | CTACAGCATTATAGTTACCCTCGGACG |
| | 21-225_45B4 | | SEQ ID NO:1211 | SEQ ID NO:9223 | SEQ ID NO:17235 |
| | | AA | RASQGIRDDLG | AASSLQS | LQHYSYPRT |
| | | | SEQ ID NO:1212 | SEQ ID NO:9224 | SEQ ID NO:17236 |
| iPS:433953 | | NA | CGGGCGAGTCAGGATATTAGCAGCTGGTTAGCC | GATGCATCCAGTTTGCAAAGT | CAACAGGCTAACAGTCTCCCTTTCACT |
| | 21-225_45H4 | | SEQ ID NO:1213 | SEQ ID NO:9225 | SEQ ID NO:17237 |
| | | AA | RASQDISSWLA | DASSLQS | QQANSLPFT |
| | | | SEQ ID NO:1214 | SEQ ID NO:9226 | SEQ ID NO:17238 |
| iPS:433955 | | NA | CGGGCAAGTCAGGACATTAGAGATGATTTAGGC | GCTGCATCCAGTTTGCAAAGT | CTACAGCATTATAATTACCCTCGGACG |
| | 21-225_45B8 | | SEQ ID NO:1215 | SEQ ID NO:9227 | SEQ ID NO:17239 |
| | | AA | RASQDIRDDLG | AASSLQS | LQHYNYPRT |
| | | | SEQ ID NO:1216 | SEQ ID NO:9228 | SEQ ID NO:17240 |
| iPS:433957 | | NA | CGGGCGAGTCAGGGTATTAGCGACTGGTTAGCC | GGTGCATCCAGTTTGCAAAGT | CAACAGACTAACAGTTTCCCGTGGACG |
| | 21-225_45F8 | | SEQ ID NO:1217 | SEQ ID NO:9229 | SEQ ID NO:17241 |
| | | AA | RASQGISDWLA | GASSLQS | QQTNSFPWT |
| | | | SEQ ID NO:1218 | SEQ ID NO:9230 | SEQ ID NO:17242 |
| iPS:433959 | | NA | CGGGCGAGTCAGGATATTAGCGACTGGTTAGCT | GCTGCATCCAGTTTGGAAAGT | CAACAGGCTAACAGTTTCCCATTCACT |
| | 21-225_45C9 | | SEQ ID NO:1219 | SEQ ID NO:9231 | SEQ ID NO:17243 |
| | | AA | RASQDISDWLA | AASSLES | QQANSFPFT |
| | | | SEQ ID NO:1220 | SEQ ID NO:9232 | SEQ ID NO:17244 |
| iPS:433961 | | NA | CGGGCGAGTCAGGGCATTAACAATTATTTAGCC | GCTGCATCCAGTTTGCAAAGT | CAACACTATTATAGTTACCCATTCACT |
| | 21-225_45D9 | | SEQ ID NO:1221 | SEQ ID NO:9233 | SEQ ID NO:17245 |

FIGURE 49

| | | AA | RASQGINNYLA | AASSLQS | QHYYSYPFT |
|---|---|---|---|---|---|
| | | | SEQ ID NO:1222 | SEQ ID NO:9234 | SEQ ID NO:17246 |
| iPS:433963 | | NA | CGGGCAAGTCAGGGCATTAG AAATGATTTAGGC | GCTGCATCCAGTTTGCAA GGT | CTACAGCATAATAGTTACCC GCTCACT |
| | 21-225_46B1 | | SEQ ID NO:1223 | SEQ ID NO:9235 | SEQ ID NO:17247 |
| | | AA | RASQGIRNDLG | AASSLQG | LQHNSYPLT |
| | | | SEQ ID NO:1224 | SEQ ID NO:9236 | SEQ ID NO:17248 |
| iPS:433965 | | NA | AAGTCTAGTCAGAGCCTCCT GCATGGTGATGGAAAGACAT ATTTGTAT | GAAGTTTCCAATCGGTTC TCT | ATGCAAAGTATACAGCTTCC GTGGACG |
| | 21-225_46F2 | | SEQ ID NO:1225 | SEQ ID NO:9237 | SEQ ID NO:17249 |
| | | AA | KSSQSLLHGDGKTYLY | EVSNRFS | MQSIQLPWT |
| | | | SEQ ID NO:1226 | SEQ ID NO:9238 | SEQ ID NO:17250 |
| iPS:433967 | | NA | CGGGCAAGTCAGGGCATTAG AGATGATTTAGGC | GCTGCATCCAGTTTGCAA AGT | CTACAGCATTATAGTTACCC TCGGACG |
| | 21-225_46C3 | | SEQ ID NO:1227 | SEQ ID NO:9239 | SEQ ID NO:17251 |
| | | AA | RASQGIRDDLG | AASSLQS | LQHYSYPRT |
| | | | SEQ ID NO:1228 | SEQ ID NO:9240 | SEQ ID NO:17252 |
| iPS:433969 | | NA | CGGGCAAGTCAGGGCATTAG AAAAGATTTAGGC | GCTGCATCCAGTTTGCAA AGT | CTACAGCATAATAGTTACCC TCTCACT |
| | 21-225_46F3 | | SEQ ID NO:1229 | SEQ ID NO:9241 | SEQ ID NO:17253 |
| | | AA | RASQGIRKDLG | AASSLQS | LQHNSYPLT |
| | | | SEQ ID NO:1230 | SEQ ID NO:9242 | SEQ ID NO:17254 |
| iPS:433971 | | NA | CGGACAAGTCAGGACATTAG AAAAGATTTAGGC | GCTGCATCCAGTTTGGA AAGT | CTACAGCATTATAGTTTCCC GTGGACG |
| | 21-225_46D4 | | SEQ ID NO:1231 | SEQ ID NO:9243 | SEQ ID NO:17255 |
| | | AA | RTSQDIRKDLG | AASSLES | LQHYSFPWT |
| | | | SEQ ID NO:1232 | SEQ ID NO:9244 | SEQ ID NO:17256 |
| iPS:433973 | | NA | CGGGCGAGTCAGGGTATTAG CAACTGGTTAGCC | GCTGCATCCAGTTTGCAA AGT | CAACAGACTAACAGTTTCCC GTGGACG |

FIGURE 49

| | 21-225_46A6 | | SEQ ID NO:1233 | SEQ ID NO:9245 | SEQ ID NO:17257 |
|---|---|---|---|---|---|
| | | AA | RASQGISNWLA | AASSLQS | QQTNSFPWT |
| | | | SEQ ID NO:1234 | SEQ ID NO:9246 | SEQ ID NO:17258 |
| iPS:433975 | | NA | CGGGCAAGTCAGGGCATTAG AAATGATTTAGGC | GCTGCATCCAGTTTGCAA AGT | CTACAGCATAATAGTTACCC TCTCACT |
| | 21-225_46C6 | | SEQ ID NO:1235 | SEQ ID NO:9247 | SEQ ID NO:17259 |
| | | AA | RASQGIRNDLG | AASSLQS | LQHNSYPLT |
| | | | SEQ ID NO:1236 | SEQ ID NO:9248 | SEQ ID NO:17260 |
| iPS:433977 | | NA | CGGGCAAGTCAGGGCATTAG AAAAGATTTAGGC | GCTGCATCCAGTTTGCAA AGT | CTACAGCATAATAGTTACCC TCTCACT |
| | 21-225_46D8 | | SEQ ID NO:1237 | SEQ ID NO:9249 | SEQ ID NO:17261 |
| | | AA | RASQGIRKDLG | AASSLQS | LQHNSYPLT |
| | | | SEQ ID NO:1238 | SEQ ID NO:9250 | SEQ ID NO:17262 |
| iPS:433979 | | NA | AAGTCTAGTCAGAGCCTCCT GCATAGTGAGGGAAAGACC TATTTGTAT | GAAGTTTCTTACCGGTTC TCT | ATGCACAGTATACAGTATCC GCTCACG |
| | 21-225_46B9 | | SEQ ID NO:1239 | SEQ ID NO:9251 | SEQ ID NO:17263 |
| | | AA | KSSQSLLHSEGKTYLY | EVSYRFS | MHSIQYPLT |
| | | | SEQ ID NO:1240 | SEQ ID NO:9252 | SEQ ID NO:17264 |
| iPS:433981 | | NA | CGGGCAAGTCAGGGCATTAG AAATGATTTAGGC | GCTGCATCCAGTTTGCAA AGT | CTTCAGCATACTAGTTTCCC ATTCACT |
| | 21-225_46E9 | | SEQ ID NO:1241 | SEQ ID NO:9253 | SEQ ID NO:17265 |
| | | AA | RASQGIRNDLG | AASSLQS | LQHTSFPFT |
| | | | SEQ ID NO:1242 | SEQ ID NO:9254 | SEQ ID NO:17266 |
| iPS:433983 | | NA | CGGGCAAGTCAGGACATTAG AAATGATTTAGGC | GCTGCATTCAGTTTGCAA AGT | CTGCAACATAATAGTTACCC GCTCACT |
| | 21-225_47A1 | | SEQ ID NO:1243 | SEQ ID NO:9255 | SEQ ID NO:17267 |
| | | AA | RASQDIRNDLG | AAFSLQS | LQHNSYPLT |
| | | | SEQ ID NO:1244 | SEQ ID NO:9256 | SEQ ID NO:17268 |

FIGURE 49

| iPS:433985 | | NA | ACGTCTAGTCAGAGCCTCCT GCATAGTGAAGGAAAGACC TATTTGTAT | GAAGTTTCCAACCGGTTC TCT | ATGCAAAGTATACAGCTTCC GTGGACG |
|---|---|---|---|---|---|
| | 21-225_47C1 | | SEQ ID NO:1245 | SEQ ID NO:9257 | SEQ ID NO:17269 |
| | | AA | TSSQSLLHSEGKTYLY | EVSNRFS | MQSIQLPWT |
| | | | SEQ ID NO:1246 | SEQ ID NO:9258 | SEQ ID NO:17270 |
| iPS:433987 | | NA | CGGGCAAGTCAGGGCATTAG AAATGATTTAGGC | GCTGCATCCAGTTTGCAA AGT | CTACAGCATAATAGTTACCC GCTCACT |
| | 21-225_47A5 | | SEQ ID NO:1247 | SEQ ID NO:9259 | SEQ ID NO:17271 |
| | | AA | RASQGIRNDLG | AASSLQS | LQHNSYPLT |
| | | | SEQ ID NO:1248 | SEQ ID NO:9260 | SEQ ID NO:17272 |
| iPS:433989 | | NA | AGGTCTAGTCAGAGCCTCCT GCATAGTAATGGATACAACT ATTTGGAA | TTGGGTTTTAATCGGGCC TCC | ATGCAAGTTCTACAAACTCC ATTCACT |
| | 21-225_47C7 | | SEQ ID NO:1249 | SEQ ID NO:9261 | SEQ ID NO:17273 |
| | | AA | RSSQSLLHSNGYNYLE | LGFNRAS | MQVLQTPFT |
| | | | SEQ ID NO:1250 | SEQ ID NO:9262 | SEQ ID NO:17274 |
| iPS:433991 | | NA | AAGTCTAGTCAGAGCCTCCT GCATAGTGATGGAAGGACCT ATTTGTAT | GAAGTTTCCAGCCGGTTC TCT | ATGCAAAGTACACAACTTCC GTGGACG |
| | 21-225_47E7 | | SEQ ID NO:1251 | SEQ ID NO:9263 | SEQ ID NO:17275 |
| | | AA | KSSQSLLHSDGRTYLY | EVSSRFS | MQSTQLPWT |
| | | | SEQ ID NO:1252 | SEQ ID NO:9264 | SEQ ID NO:17276 |
| iPS:433993 | | NA | CGGGCGAGTCAGGGTATTAG CAACTGGTTAGCC | GCTGCCTCCAATTTGCAA AGT | CAACAGGTTAACAGTTTCCC GTGGACG |
| | 21-225_47G7 | | SEQ ID NO:1253 | SEQ ID NO:9265 | SEQ ID NO:17277 |
| | | AA | RASQGISNWLA | AASNLQS | QQVNSFPWT |
| | | | SEQ ID NO:1254 | SEQ ID NO:9266 | SEQ ID NO:17278 |
| iPS:433995 | | NA | CGGACAAGTCAGGGCATTAG AAATGATTTAGGC | GCTGCATCCAGTTTGCAA AGT | CTACAACATACTAGTTTCCC ATTCACT |

FIGURE 49

| | 21-225_47H7 | | SEQ ID NO:1255 | SEQ ID NO:9267 | SEQ ID NO:17279 |
|---|---|---|---|---|---|
| | | AA | RTSQGIRNDLG | AASSLQS | LQHTSFPFT |
| | | | SEQ ID NO:1256 | SEQ ID NO:9268 | SEQ ID NO:17280 |
| iPS:433997 | | NA | CGGACAAGTCAGGGCATTAG AAATGATTTAGGC | AGAGCATCCAGTTTGCA AAGT | CTACAGCATAATTTTTACCC GTGGACG |
| | 21-225_48C1 | | SEQ ID NO:1257 | SEQ ID NO:9269 | SEQ ID NO:17281 |
| | | AA | RTSQGIRNDLG | RASSLQS | LQHNFYPWT |
| | | | SEQ ID NO:1258 | SEQ ID NO:9270 | SEQ ID NO:17282 |
| iPS:433999 | | NA | CGGGCAAGTCAGAGCATTAG CAGCTATTTAATT | GCTGCATCCAGTTTGCAA AGT | CAACAGAGTAACAGTATTCC ATTCACT |
| | 21-225_48D1 | | SEQ ID NO:1259 | SEQ ID NO:9271 | SEQ ID NO:17283 |
| | | AA | RASQSISSYLI | AASSLQS | QQSNSIPFT |
| | | | SEQ ID NO:1260 | SEQ ID NO:9272 | SEQ ID NO:17284 |
| iPS:434001 | | NA | CGGGCAAGTCGGGGCATTAG AGATGATTTAGGC | GCTGCATCCAGTTTGCAA AGT | CTACAGCAATATAGTTATCC TCGGACG |
| | 21-225_48F2 | | SEQ ID NO:1261 | SEQ ID NO:9273 | SEQ ID NO:17285 |
| | | AA | RASRGIRDDLG | AASSLQS | LQQYSYPRT |
| | | | SEQ ID NO:1262 | SEQ ID NO:9274 | SEQ ID NO:17286 |
| iPS:434003 | | NA | CGGGCAAGTCAGAGCATTAT CAGCTATTTAATT | GCTGCATCCAGTTTGCAA AGT | CAACAGACTAACAGTATTCC ATTCACT |
| | 21-225_48C3 | | SEQ ID NO:1263 | SEQ ID NO:9275 | SEQ ID NO:17287 |
| | | AA | RASQSIISYLI | AASSLQS | QQTNSIPFT |
| | | | SEQ ID NO:1264 | SEQ ID NO:9276 | SEQ ID NO:17288 |
| iPS:434007 | | NA | CGGGCGAGTCAAAATATTAC CAGCTGGTTAGCC | AGTGCATCCAGTTTGCA AAAT | CAACAGGCTAACAGTTTCCC GTGGACG |
| | 21-225_48D7 | | SEQ ID NO:1265 | SEQ ID NO:9277 | SEQ ID NO:17289 |
| | | AA | RASQNITSWLA | SASSLQN | QQANSFPWT |
| | | | SEQ ID NO:1266 | SEQ ID NO:9278 | SEQ ID NO:17290 |
| iPS:434009 | | NA | CGGGCAAGTCAGGGCATTAG AAATGATTTAGGC | ATTGCATCCAGTTTGCAA AGT | CTACAGCATAATCGTTACCC GTGGACG |

FIGURE 49

| | 21-225_48A9 | | SEQ ID NO:1267 | SEQ ID NO:9279 | SEQ ID NO:17291 |
|---|---|---|---|---|---|
| | | AA | RASQGIRNDLG | IASSLQS | LQHNRYPWT |
| | | | SEQ ID NO:1268 | SEQ ID NO:9280 | SEQ ID NO:17292 |
| iPS:434011 | 21-225_48B10 | NA | CGGGCAAGTCAGAGCATTAGGAAGTATTTAAAT | GCTGCTTCCAGTTTGCAAAGT | CAACAGACTTACAGTAACCCACTCACT |
| | | | SEQ ID NO:1269 | SEQ ID NO:9281 | SEQ ID NO:17293 |
| | | AA | RASQSIRKYLN | AASSLQS | QQTYSNPLT |
| | | | SEQ ID NO:1270 | SEQ ID NO:9282 | SEQ ID NO:17294 |
| iPS:434013 | 21-225_48D12 | NA | CGGGCAAGTCAGGGCATTAGAAATGATTTAGGC | GCTGCATCCACTTTGCAAAGT | CTACAGCATAATAGTTACCCGCTCACT |
| | | | SEQ ID NO:1271 | SEQ ID NO:9283 | SEQ ID NO:17295 |
| | | AA | RASQGIRNDLG | AASTLQS | LQHNSYPLT |
| | | | SEQ ID NO:1272 | SEQ ID NO:9284 | SEQ ID NO:17296 |
| iPS:434015 | 21-225_48F12 | NA | CGGGCAAGTCAGAGCATTAGGAAGTATTTAAAT | GCTGCTTCCAGTTTGCAAAGT | CAACAGACTTACAGTAACCCGCTCACT |
| | | | SEQ ID NO:1273 | SEQ ID NO:9285 | SEQ ID NO:17297 |
| | | AA | RASQSIRKYLN | AASSLQS | QQTYSNPLT |
| | | | SEQ ID NO:1274 | SEQ ID NO:9286 | SEQ ID NO:17298 |
| iPS:434017 | 21-225_48G12 | NA | CGGGCAAGTCAGAGCATTAGGAAGTATTTAAAT | GCTGCTTCCAGTTTGCAAAGT | CAACAGACTTACAGTAACCCGCTCACT |
| | | | SEQ ID NO:1275 | SEQ ID NO:9287 | SEQ ID NO:17299 |
| | | AA | RASQSIRKYLN | AASSLQS | QQTYSNPLT |
| | | | SEQ ID NO:1276 | SEQ ID NO:9288 | SEQ ID NO:17300 |
| iPS:434019 | 21-225_49A1 | NA | CGGGCAAGTCAGGGCATTAGAAATGATTTAGAC | GCTGCATCCAGTTTGCAAAGT | CTACAGCATAATAGTTACCCGCTCACT |
| | | | SEQ ID NO:1277 | SEQ ID NO:9289 | SEQ ID NO:17301 |
| | | AA | RASQGIRNDLD | AASSLQS | LQHNSYPLT |
| | | | SEQ ID NO:1278 | SEQ ID NO:9290 | SEQ ID NO:17302 |

FIGURE 49

| iPS:434021 | 21-225_49C1 | NA | AAGTCTAGTCAGAGCCTCCTGCATAGGGAAGGAAAGACCTATTTGTAC | GAAGTTTCCCACCGGTTCTCT | ATGCAAAGTATACAGATTCCGATCACC |
| | | | SEQ ID NO:1279 | SEQ ID NO:9291 | SEQ ID NO:17303 |
| | | AA | KSSQSLLHREGKTYLY | EVSHRFS | MQSIQIPIT |
| | | | SEQ ID NO:1280 | SEQ ID NO:9292 | SEQ ID NO:17304 |
| iPS:434023 | 21-225_49F1 | NA | CGGGCGAGTCGGGATATTAACGGCTGGTTAGCC | ACTGTCTCCAGTTTGCAAAGT | CAACAGTCTAACAGTTTCCCATTCACT |
| | | | SEQ ID NO:1281 | SEQ ID NO:9293 | SEQ ID NO:17305 |
| | | AA | RASRDINGWLA | TVSSLQS | QQSNSFPFT |
| | | | SEQ ID NO:1282 | SEQ ID NO:9294 | SEQ ID NO:17306 |
| iPS:434025 | 21-225_49G3 | NA | AAGTCTAGTCAGAGCCTCCTGCATAGTGAAGGAAAGACCTATTTGTAT | GAAGTTTCCAACCGGCTCTCT | ATGCAAAGTATGCAGCTTCCGATCACC |
| | | | SEQ ID NO:1283 | SEQ ID NO:9295 | SEQ ID NO:17307 |
| | | AA | KSSQSLLHSEGKTYLY | EVSNRLS | MQSMQLPIT |
| | | | SEQ ID NO:1284 | SEQ ID NO:9296 | SEQ ID NO:17308 |
| iPS:434027 | 21-225_49H5 | NA | CGGGCGAGTCAGGGTTTTAGCACCTGGTTAGCC | GCTGCATCCAGTTTGCAAGAT | CAACAGACTAACAGTTTCCCGTTCACT |
| | | | SEQ ID NO:1285 | SEQ ID NO:9297 | SEQ ID NO:17309 |
| | | AA | RASQGFSTWLA | AASSLQD | QQTNSFPFT |
| | | | SEQ ID NO:1286 | SEQ ID NO:9298 | SEQ ID NO:17310 |
| iPS:434029 | 21-225_49C6 | NA | CGGGCAAGTCAGGGCATTAGAAATGATTTAGGC | GCTGCATCCAGTTTGCAAAGT | CTACAGCATAATAGTTACCCTCTCACT |
| | | | SEQ ID NO:1287 | SEQ ID NO:9299 | SEQ ID NO:17311 |
| | | AA | RASQGIRNDLG | AASSLQS | LQHNSYPLT |
| | | | SEQ ID NO:1288 | SEQ ID NO:9300 | SEQ ID NO:17312 |
| iPS:434031 | 21 225 49E7 | NA | AAGTCTAGTCAGATCTTCTTGCATAGTGAAGGAAAGACCTATTTGTAT | GAAGTTTCCAAGCGGCTCTCT | ATGCAAAGTATGCAGCTTCCGATTATC |

FIGURE 49

| | | | SEQ ID NO:1289 | SEQ ID NO:9301 | SEQ ID NO:17313 |
|---|---|---|---|---|---|
| | 21-225_49E7 | AA | KSSQIFLHSEGKTYLY | EVSKRLS | MQSMQLPII |
| | | | SEQ ID NO:1290 | SEQ ID NO:9302 | SEQ ID NO:17314 |
| iPS:434033 | 21-225_49F9 | NA | AAGTCTAATCAGAGCCTCGT GCATAATGAAGGAAAGACC TATTTGTAT | GAAGTTTCCAACCGGTTC TCT | ATGCAAAGTATACAGTATCC GATCACC |
| | | | SEQ ID NO:1291 | SEQ ID NO:9303 | SEQ ID NO:17315 |
| | | AA | KSNQSLVHNEGKTYLY | EVSNRFS | MQSIQYPIT |
| | | | SEQ ID NO:1292 | SEQ ID NO:9304 | SEQ ID NO:17316 |
| iPS:434035 | 21-225_49F10 | NA | CGGGCGAGTCAGGGTATTAG CAGGTGGTTAGCC | GCTGCATCCACTTTGCAA AGT | CAACAGGCTAACAGTTTCCC ATTCACT |
| | | | SEQ ID NO:1293 | SEQ ID NO:9305 | SEQ ID NO:17317 |
| | | AA | RASQGISRWLA | AASTLQS | QQANSFPFT |
| | | | SEQ ID NO:1294 | SEQ ID NO:9306 | SEQ ID NO:17318 |
| iPS:434037 | 21-225_49G12 | NA | CGGTCAAGTCAGAGCATTAG TACCTATTTAATG | GCTGCATCCAGTTTGCAA ATT | CAACAGAGTTACAGTATCCC ATTCACT |
| | | | SEQ ID NO:1295 | SEQ ID NO:9307 | SEQ ID NO:17319 |
| | | AA | RSSQSISTYLM | AASSLQI | QQSYSIPFT |
| | | | SEQ ID NO:1296 | SEQ ID NO:9308 | SEQ ID NO:17320 |
| iPS:434039 | 21-225_43B1 | NA | CGGGCAAGTCAGGGCATTAG AAATGATTTAGGC | GCTGCATCCAGTTTGCAA AGT | CTACAGCATACTAGTTTCCC ATTCACT |
| | | | SEQ ID NO:1297 | SEQ ID NO:9309 | SEQ ID NO:17321 |
| | | AA | RASQGIRNDLG | AASSLQS | LQHTSFPFT |
| | | | SEQ ID NO:1298 | SEQ ID NO:9310 | SEQ ID NO:17322 |
| iPS:434041 | 21-225_50H8 | NA | CGGGCAAGTCAGAGCATTAG CAGCTATTTAATT | GCTGCATCCAGTCTGCA AAGT | CAACAGAGTAACAGTCTTCC ATTCACT |
| | | | SEQ ID NO:1299 | SEQ ID NO:9311 | SEQ ID NO:17323 |
| | | AA | RASQSISSYLI | AASSLQS | QQSNSLPFT |
| | | | SEQ ID NO:1300 | SEQ ID NO:9312 | SEQ ID NO:17324 |

FIGURE 49

| iPS:434043 | | NA | CGGGCAAGTCAGGGCATTAG AAATAATTTAGGC | GCTGCATCCAGTTTGCAA AGT | CTACAGTATAATAGTTACCC GTTCACT |
| | 21-225_50G10 | | SEQ ID NO:1301 | SEQ ID NO:9313 | SEQ ID NO:17325 |
| | | AA | RASQGIRNNLG | AASSLQS | LQYNSYPFT |
| | | | SEQ ID NO:1302 | SEQ ID NO:9314 | SEQ ID NO:17326 |
| iPS:434045 | | NA | CGGGCAAGTCAGAGCATTTA CAGCTATTTAATT | GCTGCATCCAGTTTGCAA AGT | CAACAGAGTAACAGTATTCC ATTCACT |
| | 21-225_50H10 | | SEQ ID NO:1303 | SEQ ID NO:9315 | SEQ ID NO:17327 |
| | | AA | RASQSIYSYLI | AASSLQS | QQSNSIPFT |
| | | | SEQ ID NO:1304 | SEQ ID NO:9316 | SEQ ID NO:17328 |
| iPS:434047 | | NA | CGGGCAAGTCAGGGCATTAG AAATGATTTAGGC | ACTGCATCCAATTTACAA AGT | CTACAGCATAATAGTTACCC GTGGACG |
| | 21-225_50A12 | | SEQ ID NO:1305 | SEQ ID NO:9317 | SEQ ID NO:17329 |
| | | AA | RASQGIRNDLG | TASNLQS | LQHNSYPWT |
| | | | SEQ ID NO:1306 | SEQ ID NO:9318 | SEQ ID NO:17330 |
| iPS:434049 | | NA | CGGGCAAGTCAGAGCATTAG TAGTTATTTAAAT | GCTGCATCCAGTTTGCAA AGT | CAACAGAGTTACATTGCCCC ATTCACT |
| | 21-225_50B12 | | SEQ ID NO:1307 | SEQ ID NO:9319 | SEQ ID NO:17331 |
| | | AA | RASQSISSYLN | AASSLQS | QQSYIAPFT |
| | | | SEQ ID NO:1308 | SEQ ID NO:9320 | SEQ ID NO:17332 |
| iPS:434053 | | NA | AAGTCTAGTCAGAGCCTCCT GCATAGTGAGGGAAAGACC TATTTGTAT | GAAGTTTCCAACCGGTTC TCT | ATGCAAAGTATACAGCTTCC ATTCACT |
| | 21-225_51E1 | | SEQ ID NO:1309 | SEQ ID NO:9321 | SEQ ID NO:17333 |
| | | AA | KSSQSLLHSEGKTYLY | EVSNRFS | MQSIQLPFT |
| | | | SEQ ID NO:1310 | SEQ ID NO:9322 | SEQ ID NO:17334 |
| iPS:434055 | | NA | CAGGCGAGTCGGGACATTAC CTTCTATTTAAAT | GATGCATCCAATTTGGA AACA | CAACAGTATGATAATCTTCC ATTCACT |
| | 21-225_51B4 | | SEQ ID NO:1311 | SEQ ID NO:9323 | SEQ ID NO:17335 |
| | | AA | QASRDITFYLN | DASNLET | QQYDNLPFT |

FIGURE 49

| | | | SEQ ID NO:1312 | SEQ ID NO:9324 | SEQ ID NO:17336 |
|---|---|---|---|---|---|
| iPS:434057 | | NA | CGGGCAAGTCAGGGCATTAGAAATGATTTAGGC | GCTGCATCCAGTTTGCAAAGT | CTACAGCATAATAGTTACCCATTCACT |
| | 21-225_51E4 | | SEQ ID NO:1313 | SEQ ID NO:9325 | SEQ ID NO:17337 |
| | | AA | RASQGIRNDLG | AASSLQS | LQHNSYPFT |
| | | | SEQ ID NO:1314 | SEQ ID NO:9326 | SEQ ID NO:17338 |
| iPS:434059 | | NA | CGGGCGAGTCAGGGCATTAGCAATTATTTAGCC | GCTGCATCCAGTTTGCGAAGT | CAACAGTATTATAGTTACCCATTCACT |
| | 21-225_51C5 | | SEQ ID NO:1315 | SEQ ID NO:9327 | SEQ ID NO:17339 |
| | | AA | RASQGISNYLA | AASSLRS | QQYYSYPFT |
| | | | SEQ ID NO:1316 | SEQ ID NO:9328 | SEQ ID NO:17340 |
| iPS:434061 | | NA | CGGGCGAGTCAGGATGTTAACAACTACTTAGCC | GCTGCATCCAGTTTGCAAAAT | CAACAAACTAACAGTTTCCCATTCACT |
| | 21-225_51C7 | | SEQ ID NO:1317 | SEQ ID NO:9329 | SEQ ID NO:17341 |
| | | AA | RASQDVNNYLA | AASSLQN | QQTNSFPFT |
| | | | SEQ ID NO:1318 | SEQ ID NO:9330 | SEQ ID NO:17342 |
| iPS:434063 | | NA | CGGGCGAGTCAGGATATTAGCAGTTGGTTAGCC | GCTCCATCCAATTGCAAAGT | CAACAGGCTCACAGTTTCCCGTGGACG |
| | 21-225_51G7 | | SEQ ID NO:1319 | SEQ ID NO:9331 | SEQ ID NO:17343 |
| | | AA | RASQDISSWLA | APSNLQS | QQAHSFPWT |
| | | | SEQ ID NO:1320 | SEQ ID NO:9332 | SEQ ID NO:17344 |
| iPS:434065 | | NA | CGGGCGAGTCAGGGTATTAGCAGGTGGTTAGCC | GCTGCATCCACTTTGCAAAGT | CAACAGGCTAACAGTTTCCCATTCACT |
| | 21-225_50D4 | | SEQ ID NO:1321 | SEQ ID NO:9333 | SEQ ID NO:17345 |
| | | AA | RASQGISRWLA | AASTLQS | QQANSFPFT |
| | | | SEQ ID NO:1322 | SEQ ID NO:9334 | SEQ ID NO:17346 |
| iPS:434067 | | NA | CGGGCAAGTCAGGGCATTAGAAATGATTTGGGC | GCTGCTTCCAGTTTGCAAAGT | CTACAGCATAATAGTTACCCGTGGACG |
| | 21-225_51H8 | | SEQ ID NO:1323 | SEQ ID NO:9335 | SEQ ID NO:17347 |
| | | AA | RASQGIRNDLG | AASSLQS | LQHNSYPWT |

687

FIGURE 49

| | | | SEQ ID NO:1324 | SEQ ID NO:9336 | SEQ ID NO:17348 |
|---|---|---|---|---|---|
| iPS:434069 | | NA | CGGGCGAGTCAGGGTATTAGCAGTTGGTTAGCC | GTTGCATCCAGTTTGCAAAGT | CAACAGGCTAAAAGTTTCCCATTCACT |
| | 21-225_51E9 | | SEQ ID NO:1325 | SEQ ID NO:9337 | SEQ ID NO:17349 |
| | | AA | RASQGISSWLA | VASSLQS | QQAKSFPFT |
| | | | SEQ ID NO:1326 | SEQ ID NO:9338 | SEQ ID NO:17350 |
| iPS:434071 | | NA | CGGGCAAGTCAGGGCATTAGGACTGATTTAGGC | GCTGCATCCAGTTTGCAACGT | CTACAGCATAATAGTTACCCATTCACT |
| | 21-225_51F9 | | SEQ ID NO:1327 | SEQ ID NO:9339 | SEQ ID NO:17351 |
| | | AA | RASQGIRTDLG | AASSLQR | LQHNSYPFT |
| | | | SEQ ID NO:1328 | SEQ ID NO:9340 | SEQ ID NO:17352 |
| iPS:434073 | | NA | CGGGCAAGTCAGAGCATTAGTACCTATTTAATG | GCTGCATCCAGTTTGCAAAGT | CAACAGAGTTACAGTATCCCATTCACT |
| | 21-225_51H10 | | SEQ ID NO:1329 | SEQ ID NO:9341 | SEQ ID NO:17353 |
| | | AA | RASQSISTYLM | AASSLQS | QQSYSIPFT |
| | | | SEQ ID NO:1330 | SEQ ID NO:9342 | SEQ ID NO:17354 |
| iPS:434075 | | NA | CGGACAAGTCAGGGCATTAGAAATGATTTAGGC | GCTGCATCCAGTTTGCAAAGT | CTACAGCATAATAGTTACCCATTCACT |
| | 21-225_51B11 | | SEQ ID NO:1331 | SEQ ID NO:9343 | SEQ ID NO:17355 |
| | | AA | RTSQGIRNDLG | AASSLQS | LQHNSYPFT |
| | | | SEQ ID NO:1332 | SEQ ID NO:9344 | SEQ ID NO:17356 |
| iPS:434077 | | NA | CGGGCAAGTCAGGGCATTAGAAATGATTTAGGC | GCTGCATCCAGTTTGCAAAGT | CTACAGCATAATAGTTACCCGTTCACT |
| | 21-225_51F11 | | SEQ ID NO:1333 | SEQ ID NO:9345 | SEQ ID NO:17357 |
| | | AA | RASQGIRNDLG | AASSLQS | LQHNSYPFT |
| | | | SEQ ID NO:1334 | SEQ ID NO:9346 | SEQ ID NO:17358 |
| iPS:434079 | | NA | CGGGCGAGTCAGGATATTCGCACCTGGTTAGCC | GCTGCATCCAGTTTGCAAAAT | CAACAGGCTAAAAGTTTCCCATTCACT |
| | 21-225_52B1 | | SEQ ID NO:1335 | SEQ ID NO:9347 | SEQ ID NO:17359 |
| | | AA | RASQDIRTWLA | AASSLQN | QQAKSFPFT |

FIGURE 49

| | | | SEQ ID NO:1336 | SEQ ID NO:9348 | SEQ ID NO:17360 |
|---|---|---|---|---|---|
| iPS:434081 | | NA | CGGGCAAGTCAGGGCATTAG AAATGATTTAGGC | GCTGCATCCTTTTTGCAA AGT | CTGCAGCATAATAGCTACCC TCTCACT |
| | 21-225_52B2 | | SEQ ID NO:1337 | SEQ ID NO:9349 | SEQ ID NO:17361 |
| | | AA | RASQGIRNDLG | AASFLQS | LQHNSYPLT |
| | | | SEQ ID NO:1338 | SEQ ID NO:9350 | SEQ ID NO:17362 |
| iPS:434083 | | NA | CGGGCGAGTCAGAATATTAC CAACTGGTTAGCC | ACTACATCCAGTTTGCAA AGT | CAACAGACTAACAGTTTCCC GTGGACG |
| | 21-225_52H2 | | SEQ ID NO:1339 | SEQ ID NO:9351 | SEQ ID NO:17363 |
| | | AA | RASQNITNWLA | TTSSLQS | QQTNSFPWT |
| | | | SEQ ID NO:1340 | SEQ ID NO:9352 | SEQ ID NO:17364 |
| iPS:434085 | | NA | CGGGCGAGTCAGGGCATTAG CAATTATTTAGCC | GCTGCATCCAGTTTACAA AGT | CAACAGTATAATAGTTTCCC TTTCACT |
| | 21-225_52E3 | | SEQ ID NO:1341 | SEQ ID NO:9353 | SEQ ID NO:17365 |
| | | AA | RASQGISNYLA | AASSLQS | QQYNSFPFT |
| | | | SEQ ID NO:1342 | SEQ ID NO:9354 | SEQ ID NO:17366 |
| iPS:434087 | | NA | CAGGCGAGTCAGGACATTAG TAACTATTTACAT | GATGCATCCACTTTGGG AACA | CAACAGTGTGATAATCTCCC GCTCACT |
| | 21-225_52F6 | | SEQ ID NO:1343 | SEQ ID NO:9355 | SEQ ID NO:17367 |
| | | AA | QASQDISNYLH | DASTLGT | QQCDNLPLT |
| | | | SEQ ID NO:1344 | SEQ ID NO:9356 | SEQ ID NO:17368 |
| iPS:434091 | | NA | CGGGCAAGTCAGGGCATTAG AAATGATTTAGGC | GCTGCATCCAGTTTGCAA AGT | CTACAGCATAATAGTTACCC ATTCACT |
| | 21-225_52B9 | | SEQ ID NO:1345 | SEQ ID NO:9357 | SEQ ID NO:17369 |
| | | AA | RASQGIRNDLG | AASSLQS | LQHNSYPFT |
| | | | SEQ ID NO:1346 | SEQ ID NO:9358 | SEQ ID NO:17370 |
| iPS:434093 | | NA | AAGTCTAGTCAGAGCCTCCT GCATAGTGAAGGAAAGACC TATTTGTAT | GAAGTTTCCAACCGGGT CTCT | ATGCAAAGTATACAGTATCC GATCACC |
| | 21-225_52D10 | | SEQ ID NO:1347 | SEQ ID NO:9359 | SEQ ID NO:17371 |

FIGURE 49

| | | | | | |
|---|---|---|---|---|---|
| | | AA | KSSQSLLHSEGKTYLY | EVSNRVS | MQSIQYPIT |
| | | | SEQ ID NO:1348 | SEQ ID NO:9360 | SEQ ID NO:17372 |
| iPS:434095 | | NA | CGGGCGAGTCAGGGTATTAG TAATTATTTAGGC | GCTGCATCTAGTTTGCAA AGT | CAACAGTATAATAGTTATCC TCCGACG |
| | 21-225_52F10 | | SEQ ID NO:1349 | SEQ ID NO:9361 | SEQ ID NO:17373 |
| | | AA | RASQGISNYLG | AASSLQS | QQYNSYPPT |
| | | | SEQ ID NO:1350 | SEQ ID NO:9362 | SEQ ID NO:17374 |
| iPS:434097 | | NA | CGGGCGAGTCAGGATATTAA CAGTTGGTTAGCC | GTTGCATCCAGTTTGCAA AGT | CAACAGGCTAAAAGTTTCCC ATTCACT |
| | 21-225_52H10 | | SEQ ID NO:1351 | SEQ ID NO:9363 | SEQ ID NO:17375 |
| | | AA | RASQDINSWLA | VASSLQS | QQAKSFPFT |
| | | | SEQ ID NO:1352 | SEQ ID NO:9364 | SEQ ID NO:17376 |
| iPS:434101 | | NA | CGGGCAAGTCAGGGCATAA GAAATAATTTAGGC | GGTGCATCCAGTTTGCA AAGT | CTACAGTATAATAGTTATCC ATTCACT |
| | 21-225_52H12 | | SEQ ID NO:1353 | SEQ ID NO:9365 | SEQ ID NO:17377 |
| | | AA | RASQGIRNNLG | GASSLQS | LQYNSYPFT |
| | | | SEQ ID NO:1354 | SEQ ID NO:9366 | SEQ ID NO:17378 |
| iPS:434103 | | NA | CGGGCAAGTCAGGGCATTAG AAATGATTTAGGC | CCTGCATCCAGTTTACAA AGT | CTACAGGATAATAGTTACCC ATTCACT |
| | 21-225_53G1 | | SEQ ID NO:1355 | SEQ ID NO:9367 | SEQ ID NO:17379 |
| | | AA | RASQGIRNDLG | PASSLQS | LQDNSYPFT |
| | | | SEQ ID NO:1356 | SEQ ID NO:9368 | SEQ ID NO:17380 |
| iPS:434105 | | NA | CGGGCAAGTCAGGGCATTAG AAATGATTTAGGC | GCTGCATCCAGTTTGCAA AGT | CTACAACATAATAGATACCC GCTCACT |
| | 21-225_53D2 | | SEQ ID NO:1357 | SEQ ID NO:9369 | SEQ ID NO:17381 |
| | | AA | RASQGIRNDLG | AASSLQS | LQHNRYPLT |
| | | | SEQ ID NO:1358 | SEQ ID NO:9370 | SEQ ID NO:17382 |
| iPS:434107 | | NA | CGGGCAAGTCAGAGTTTTAG CCACTATTTAAAT | GCTGTATCCAGTTTGCAA AGT | CAACAGAGTTTCAGTACCCC ATTCACT |
| | 21-225_53E2 | | SEQ ID NO:1359 | SEQ ID NO:9371 | SEQ ID NO:17383 |

FIGURE 49

| | | AA | RASQSFSHYLN | AVSSLQS | QQSFSTPFT |
|---|---|---|---|---|---|
| | | | SEQ ID NO:1360 | SEQ ID NO:9372 | SEQ ID NO:17384 |
| iPS:434111 | | NA | CAGGCGAGTCAGGACATTAG CAACTATTTACAT | GATGCATCCAATTTGGA AACA | CATCAGTATGATAATCTCCC GCTCACT |
| | 21-225_53H2 | | SEQ ID NO:1361 | SEQ ID NO:9373 | SEQ ID NO:17385 |
| | | AA | QASQDISNYLH | DASNLET | HQYDNLPLT |
| | | | SEQ ID NO:1362 | SEQ ID NO:9374 | SEQ ID NO:17386 |
| iPS:434115 | | NA | CGGGCGAGTCAGGTCATTAG CAATTATTTAGCC | GCTGCATCCAGTTTGCAA AGT | CAACAGTATCATAGTTACCC ACTCACT |
| | 21-225_53E4 | | SEQ ID NO:1363 | SEQ ID NO:9375 | SEQ ID NO:17387 |
| | | AA | RASQVISNYLA | AASSLQS | QQYHSYPLT |
| | | | SEQ ID NO:1364 | SEQ ID NO:9376 | SEQ ID NO:17388 |
| iPS:434117 | | NA | CGGGCAAGTCAGTACAGTAG CGACTATTTAAAT | GCTGCATCCAGTTTGAA AAGT | CAACAGAGTTACAGTACCCC GTTCACC |
| | 21-225_53C6 | | SEQ ID NO:1365 | SEQ ID NO:9377 | SEQ ID NO:17389 |
| | | AA | RASQYSSDYLN | AASSLKS | QQSYSTPFT |
| | | | SEQ ID NO:1366 | SEQ ID NO:9378 | SEQ ID NO:17390 |
| iPS:434119 | | NA | CGGGCAAGTCAGGGCATTAG AAATGATTTAGGC | GCTGCATCCAATTTGCAA AGT | CTACAACATAATCGTTACCC GCTCACT |
| | 21-225_53E6 | | SEQ ID NO:1367 | SEQ ID NO:9379 | SEQ ID NO:17391 |
| | | AA | RASQGIRNDLG | AASNLQS | LQHNRYPLT |
| | | | SEQ ID NO:1368 | SEQ ID NO:9380 | SEQ ID NO:17392 |
| iPS:434121 | | NA | CAGGCGAGTCAAGACATTAC CAACTATTTAGAT | GATGCATCCAATTTGGG AACA | CAACAGTGTGATAATCTCCC GCTCACT |
| | 21-225_53F6 | | SEQ ID NO:1369 | SEQ ID NO:9381 | SEQ ID NO:17393 |
| | | AA | QASQDITNYLD | DASNLGT | QQCDNLPLT |
| | | | SEQ ID NO:1370 | SEQ ID NO:9382 | SEQ ID NO:17394 |
| iPS:434123 | | NA | CGGGCGAGTCAGGGTATTAG CAGGTGGTTAGCC | GCTGCATCCAGTTTGCAA AGT | CAGCAGGCTAACAGTTTCCC ATTCACT |
| | 21-225_53F7 | | SEQ ID NO:1371 | SEQ ID NO:9383 | SEQ ID NO:17395 |

FIGURE 49

| | | | | | |
|---|---|---|---|---|---|
| | | AA | RASQGISRWLA | AASSLQS | QQANSFPFT |
| | | | SEQ ID NO:1372 | SEQ ID NO:9384 | SEQ ID NO:17396 |
| iPS:434127 | | NA | AGGGCCAGTCAGAGTATTAC AAGCAGCTACTTAGCC | GGTGCGTCCGGCAGGGC CACT | CAGCAGTTTGAAAGCTCACC CATGTGCAGT |
| | 21-225_53H8 | | SEQ ID NO:1373 | SEQ ID NO:9385 | SEQ ID NO:17397 |
| | | AA | RASQSITSSYLA | GASGRAT | QQFESSPMCS |
| | | | SEQ ID NO:1374 | SEQ ID NO:9386 | SEQ ID NO:17398 |
| iPS:434129 | | NA | CGGGCAAGTCAGGGCATTAG AAATGATTTAGGC | GCTGCATCCAGTTTGCAA AGT | CTACAGCATAATAGTTACCC ATTCACT |
| | 21-225_53B12 | | SEQ ID NO:1375 | SEQ ID NO:9387 | SEQ ID NO:17399 |
| | | AA | RASQGIRNDLG | AASSLQS | LQHNSYPFT |
| | | | SEQ ID NO:1376 | SEQ ID NO:9388 | SEQ ID NO:17400 |
| iPS:434131 | | NA | CGGGCAAGTCAGGGCATTAG AAATGATTTAGGC | GCTGCATCCAGTTTGCAA AGT | CTACAACATAATAGTTACCC ATTCACT |
| | 21-225_54D3 | | SEQ ID NO:1377 | SEQ ID NO:9389 | SEQ ID NO:17401 |
| | | AA | RASQGIRNDLG | AASSLQS | LQHNSYPFT |
| | | | SEQ ID NO:1378 | SEQ ID NO:9390 | SEQ ID NO:17402 |
| iPS:434133 | | NA | CGGGCGAGTCAGGGTATTAG CAGCTGGTTAGCC | GATGCATCCAGTTTGCA AAGT | CAACAGGCTAACAGTTTCCC GTGGACG |
| | 21-225_54G3 | | SEQ ID NO:1379 | SEQ ID NO:9391 | SEQ ID NO:17403 |
| | | AA | RASQGISSWLA | DASSLQS | QQANSFPWT |
| | | | SEQ ID NO:1380 | SEQ ID NO:9392 | SEQ ID NO:17404 |
| iPS:434135 | | NA | CGGGCAAGTCAGGACATTAG AAATATTTTAGGC | GCTGCATCCAATTTGCAA AGT | CTACAGTATAATAGTTACCC GTGGACG |
| | 21-225_54H3 | | SEQ ID NO:1381 | SEQ ID NO:9393 | SEQ ID NO:17405 |
| | | AA | RASQDIRNILG | AASNLQS | LQYNSYPWT |
| | | | SEQ ID NO:1382 | SEQ ID NO:9394 | SEQ ID NO:17406 |
| iPS:434137 | 21 225 54D4 | NA | AAGTCTAGTCAGAGCCTCCT GCATAGTGAGGGAAAGACC TATTTGTAT | GAAGTTTCCAACCGGTTC TCT | ATGCAAAGTATACAGTTTCC ATTCACT |

692

FIGURE 49

| | 21-225_54D4 | | SEQ ID NO:1383 | SEQ ID NO:9395 | SEQ ID NO:17407 |
|---|---|---|---|---|---|
| | | AA | KSSQSLLHSEGKTYLY | EVSNRFS | MQSIQFPFT |
| | | | SEQ ID NO:1384 | SEQ ID NO:9396 | SEQ ID NO:17408 |
| iPS:434141 | | NA | CGGGCAAGTCAGGGCATTAG AAATGATTTAGGC | GCTGCATCCAATTTGCAA AGT | CTACAGCATAATCGTTACCC GCTCACT |
| | 21-225_54C6 | | SEQ ID NO:1385 | SEQ ID NO:9397 | SEQ ID NO:17409 |
| | | AA | RASQGIRNDLG | AASNLQS | LQHNRYPLT |
| | | | SEQ ID NO:1386 | SEQ ID NO:9398 | SEQ ID NO:17410 |
| iPS:434143 | | NA | CGGGCAAGTCAGGGCATTAG AAATGATTTAGGC | GCTGCATCCAGTTTGCAA AGT | CTACATCATAATACTTACCC ATTCACT |
| | 21-225_54G7 | | SEQ ID NO:1387 | SEQ ID NO:9399 | SEQ ID NO:17411 |
| | | AA | RASQGIRNDLG | AASSLQS | LHHNTYPFT |
| | | | SEQ ID NO:1388 | SEQ ID NO:9400 | SEQ ID NO:17412 |
| iPS:434145 | | NA | CGGGCGAGTCAGGTTATTAG CCGCTGGTTAGCC | GCTGCATCCAGTTTGCAA AGT | CAACAGGCTAACAGTTTCCC ATTCACT |
| | 21-225_55B1 | | SEQ ID NO:1389 | SEQ ID NO:9401 | SEQ ID NO:17413 |
| | | AA | RASQVISRWLA | AASSLQS | QQANSFPFT |
| | | | SEQ ID NO:1390 | SEQ ID NO:9402 | SEQ ID NO:17414 |
| iPS:434147 | | NA | AGGGCCAGTCAGAGTGTTAG CAGCGACTTAGCC | GATGCATCCGCCAGGGC CACT | CAGCAGTATTATAACTGGCC TCTCACT |
| | 21-225_55E1 | | SEQ ID NO:1391 | SEQ ID NO:9403 | SEQ ID NO:17415 |
| | | AA | RASQSVSSDLA | DASARAT | QQYYNWPLT |
| | | | SEQ ID NO:1392 | SEQ ID NO:9404 | SEQ ID NO:17416 |
| iPS:434149 | | NA | AAATCTAGTCAGAGCCTCCT GCATAGTGAAGGAAAGACC TATTTGTAT | GAAGTTTCCCACCGGTTC TCT | ATGCAAAGTATACAGCTTCC ATTCACT |
| | 21-225_55H1 | | SEQ ID NO:1393 | SEQ ID NO:9405 | SEQ ID NO:17417 |
| | | AA | KSSQSLLHSEGKTYLY | EVSHRFS | MQSIQLPFT |
| | | | SEQ ID NO:1394 | SEQ ID NO:9406 | SEQ ID NO:17418 |

FIGURE 49

| iPS:434151 | | NA | AAGTCTAGTCAGAGCCTCGT GCATAGTGAAGGAAAGACC TATTTGTAT | GAAGTTTCCAACCGGGT CTCT | ATGCAAAGTATACTGTATCC GATCACC |
|---|---|---|---|---|---|
| | 21-225_55C2 | | SEQ ID NO:1395 | SEQ ID NO:9407 | SEQ ID NO:17419 |
| | | AA | KSSQSLVHSEGKTYLY | EVSNRVS | MQSILYPIT |
| | | | SEQ ID NO:1396 | SEQ ID NO:9408 | SEQ ID NO:17420 |
| iPS:434155 | | NA | CGGGCAAGTCAGGGCATTAG AAATGATTTAGGC | GCTGCATCCAGTTTGCAA AGT | CTACAACATAATAGTTACCC ATTCACT |
| | 21-225_55B3 | | SEQ ID NO:1397 | SEQ ID NO:9409 | SEQ ID NO:17421 |
| | | AA | RASQGIRNDLG | AASSLQS | LQHNSYPFT |
| | | | SEQ ID NO:1398 | SEQ ID NO:9410 | SEQ ID NO:17422 |
| iPS:434157 | | NA | CGGGCGAGTCAGGACATTAG CAATTATTTAATC | ACTGCATCCAGTTTGCAA AGT | CAACAGTATCATAGTTTCCC TCTCACT |
| | 21-225_55D4 | | SEQ ID NO:1399 | SEQ ID NO:9411 | SEQ ID NO:17423 |
| | | AA | RASQDISNYLI | TASSLQS | QQYHSFPLT |
| | | | SEQ ID NO:1400 | SEQ ID NO:9412 | SEQ ID NO:17424 |
| iPS:434159 | | NA | CGGGCAAGTCAGGCCATTAG AAATGATTTAGGC | GCTGCATTCAGGTTGCA AAGT | CTACAGCATAATAGTTACCC TCTCACT |
| | 21-225_55B8 | | SEQ ID NO:1401 | SEQ ID NO:9413 | SEQ ID NO:17425 |
| | | AA | RASQAIRNDLG | AAFRLQS | LQHNSYPLT |
| | | | SEQ ID NO:1402 | SEQ ID NO:9414 | SEQ ID NO:17426 |
| iPS:434161 | | NA | AAGTCTAGTCAAAGCCTCCT GCATAGTGAAGGAAAGACC TATTTGTAT | GAAGTTTCCAACCGGTTC TCT | ATACAAAGTATACAACTTCC GATCACC |
| | 21-225_55F9 | | SEQ ID NO:1403 | SEQ ID NO:9415 | SEQ ID NO:17427 |
| | | AA | KSSQSLLHSEGKTYLY | EVSNRFS | IQSIQLPIT |
| | | | SEQ ID NO:1404 | SEQ ID NO:9416 | SEQ ID NO:17428 |
| iPS:434163 | | NA | CAGGCGAGTCAAGACATTAG CAACTATTTAGAT | GATGCATCCAATTTGGA AACA | CAACAGTGTGATAATCTCCC GCTCACT |
| | 21-225_50H1 | | SEQ ID NO:1405 | SEQ ID NO:9417 | SEQ ID NO:17429 |

694

FIGURE 49

| | | | | | |
|---|---|---|---|---|---|
| | | AA | QASQDISNYLD | DASNLET | QQCDNLPLT |
| | | | SEQ ID NO:1406 | SEQ ID NO:9418 | SEQ ID NO:17430 |
| iPS:434165 | | NA | CGGGCAAGTCAGAGCATTCTCAGCTATTTGAAT | GTTGCATCCAGTTTCCAAAGT | CAACAGAGTTACAGTCCCCCTCTCACT |
| | 21-225_50F2 | | SEQ ID NO:1407 | SEQ ID NO:9419 | SEQ ID NO:17431 |
| | | AA | RASQSILSYLN | VASSFQS | QQSYSPPLT |
| | | | SEQ ID NO:1408 | SEQ ID NO:9420 | SEQ ID NO:17432 |
| iPS:434167 | | NA | CGGGCGAGTCAGGATATTAGCAGCTGGTTGGCC | GCTGCATCCAGTTTGCAAAAT | CAACAGACTAACAGTTTCCCATTCACT |
| | 21-225_50F3 | | SEQ ID NO:1409 | SEQ ID NO:9421 | SEQ ID NO:17433 |
| | | AA | RASQDISSWLA | AASSLQN | QQTNSFPFT |
| | | | SEQ ID NO:1410 | SEQ ID NO:9422 | SEQ ID NO:17434 |
| iPS:434169 | | NA | CGGGCAAGTCAGGGCATTAGAAATGATTTAGGC | GCTGCATCCAGTTTGCAAAGT | CTACAGCATAATCGTTACCCATTCACT |
| | 21-225_50C4 | | SEQ ID NO:1411 | SEQ ID NO:9423 | SEQ ID NO:17435 |
| | | AA | RASQGIRNDLG | AASSLQS | LQHNRYPFT |
| | | | SEQ ID NO:1412 | SEQ ID NO:9424 | SEQ ID NO:17436 |
| iPS:434171 | | NA | CAGGCGAGTCAGGACATTACCAACTTTTTAAAT | GATGCCTCCAATTTGGAAACA | CAACAGTATGATAATCTGATCACC |
| | 21-225_50G4 | | SEQ ID NO:1413 | SEQ ID NO:9425 | SEQ ID NO:17437 |
| | | AA | QASQDITNFLN | DASNLET | QQYDNLIT |
| | | | SEQ ID NO:1414 | SEQ ID NO:9426 | SEQ ID NO:17438 |
| iPS:434175 | | NA | CGGGCGAGTCAGGACATTAACATTTATTTAGCC | GCTGCATCCAGTTTGCAAAGT | CAACAGTATAATAGTTATCCTCTCACT |
| | 21-225_55A11 | | SEQ ID NO:1415 | SEQ ID NO:9427 | SEQ ID NO:17439 |
| | | AA | RASQDINIYLA | AASSLQS | QQYNSYPLT |
| | | | SEQ ID NO:1416 | SEQ ID NO:9428 | SEQ ID NO:17440 |
| iPS:434177 | 21 225 56A1 | NA | AAGTCCAGCCAGAGTGTTTTACATAGTTCCAACAATAAGAACTACTTAGTT | TGGGCATCTACCCGGGAATCC | CAGCAATATTATAGTACTCCTCCGACG |

FIGURE 49

| | | | SEQ ID NO:1417 | SEQ ID NO:9429 | SEQ ID NO:17441 |
|---|---|---|---|---|---|
| | 21-225_56A1 | AA | KSSQSVLHSSNNKNYLV | WASTRES | QQYYSTPPT |
| iPS:434179 | | NA | SEQ ID NO:1418<br>CGGGCAAGTCAGGACATTAG<br>AAATAATTTAGGC | SEQ ID NO:9430<br>GCTGCATCCAGTTTACAA<br>AGT | SEQ ID NO:17442<br>CTACAGGATAATAGTCACCC<br>GTTCACT |
| | 21-225_56F1 | AA | SEQ ID NO:1419<br>RASQDIRNNLG | SEQ ID NO:9431<br>AASSLQS | SEQ ID NO:17443<br>LQDNSHPFT |
| iPS:434181 | | NA | SEQ ID NO:1420<br>CGGGCAAGTCAGAGTTTTAG<br>CCACTATTTAAAT | SEQ ID NO:9432<br>GCTGTATCCAGTTTGCAA<br>AGT | SEQ ID NO:17444<br>CAACAGAGTTACAGTACCCC<br>ATTCACT |
| | 21-225_56B2 | AA | SEQ ID NO:1421<br>RASQSFSHYLN | SEQ ID NO:9433<br>AVSSLQS | SEQ ID NO:17445<br>QQSYSTPFT |
| iPS:434187 | | NA | SEQ ID NO:1422<br>CGGGCAAGTCAGGACATTAG<br>AAATCTTTTAGGC | SEQ ID NO:9434<br>GCTGCATCCAGTTTGCAA<br>AGT | SEQ ID NO:17446<br>CTACAGTATAATAGTTACCC<br>ATTCACT |
| | 21-225_56A5 | AA | SEQ ID NO:1423<br>RASQDIRNLLG | SEQ ID NO:9435<br>AASSLQS | SEQ ID NO:17447<br>LQYNSYPFT |
| iPS:434189 | | NA | SEQ ID NO:1424<br>CGGGCGAGTCAGGGTATTAG<br>GAAGTGGTTAGCC | SEQ ID NO:9436<br>GCTGCATCCAGTTTGCAA<br>AGT | SEQ ID NO:17448<br>CAACAGGCTAACAGTTTCCC<br>ATTCACT |
| | 21-225_56E5 | AA | SEQ ID NO:1425<br>RASQGIRKWLA | SEQ ID NO:9437<br>AASSLQS | SEQ ID NO:17449<br>QQANSFPFT |
| iPS:434191 | | NA | SEQ ID NO:1426<br>CGGGCAAGTCAGAGCATTTT<br>CAGATATTTAAAT | SEQ ID NO:9438<br>GCTGCATCCAGTTCCAA<br>AGT | SEQ ID NO:17450<br>CAACAGACTTACAGTCCCCC<br>TCTCACT |
| | 21-225_56B6 | AA | SEQ ID NO:1427<br>RASQSIFRYLN | SEQ ID NO:9439<br>AASSFQS | SEQ ID NO:17451<br>QQTYSPPLT |
| iPS:434193 | | NA | SEQ ID NO:1428<br>CGGGCGAGTCAGGGTATTAG<br>CAGCTGGTTAGCC | SEQ ID NO:9440<br>GCTGCATCCAGATTGCA<br>AAGT | SEQ ID NO:17452<br>CAACAGGCTAACAGTTTCCC<br>ATTCACT |

FIGURE 49

| | 21-225_56C6 | | SEQ ID NO:1429 | SEQ ID NO:9441 | SEQ ID NO:17453 |
|---|---|---|---|---|---|
| | | AA | RASQGISSWLA | AASRLQS | QQANSFPFT |
| | | | SEQ ID NO:1430 | SEQ ID NO:9442 | SEQ ID NO:17454 |
| iPS:434195 | 21-225_56F6 | NA | CGGGTGAGTCAGGATATTAGCAAATGGTTAGCC | GTTGCATCCGGTTTGCAAAGT | CAACAGGCTAACAGTTTCCCATTCACT |
| | | | SEQ ID NO:1431 | SEQ ID NO:9443 | SEQ ID NO:17455 |
| | | AA | RVSQDISKWLA | VASGLQS | QQANSFPFT |
| | | | SEQ ID NO:1432 | SEQ ID NO:9444 | SEQ ID NO:17456 |
| iPS:434197 | 21-225_56C7 | NA | CGGGCAAGTCAGGGCATTAGAAATGATTTAGGC | ACTGCATCCAATTTACAAAGT | CTACAGCATAATAGTTATCCGTGGACG |
| | | | SEQ ID NO:1433 | SEQ ID NO:9445 | SEQ ID NO:17457 |
| | | AA | RASQGIRNDLG | TASNLQS | LQHNSYPWT |
| | | | SEQ ID NO:1434 | SEQ ID NO:9446 | SEQ ID NO:17458 |
| iPS:434199 | 21-225_59F11 | NA | CGGGCAAGTCAGGGCATTAGAAATGATTTAGGC | GCTGCATCCAGTTTGCAAAGT | CTACAGCATAATCGTTACCCTTTCACT |
| | | | SEQ ID NO:1435 | SEQ ID NO:9447 | SEQ ID NO:17459 |
| | | AA | RASQGIRNDLG | AASSLQS | LQHNRYPFT |
| | | | SEQ ID NO:1436 | SEQ ID NO:9448 | SEQ ID NO:17460 |
| iPS:434201 | 21-225_59A12 | NA | AAGTCTAGTCAGAGCCTCCAGCATGGTGAAGGAAAGACCTATTTGTAT | GAAGTTTCCTATCGGTTTTCT | ATGCAAAGTACACAGCTTCCGCTCACC |
| | | | SEQ ID NO:1437 | SEQ ID NO:9449 | SEQ ID NO:17461 |
| | | AA | KSSQSLQHGEGKTYLY | EVSYRFS | MQSTQLPLT |
| | | | SEQ ID NO:1438 | SEQ ID NO:9450 | SEQ ID NO:17462 |
| iPS:434203 | 21-225_60E2 | NA | CGGGCAAGTCAGGGCATTAGAAAAGATTTAGGC | GCTGCATCCAGTTTGCAAAGT | CTACAGCATAATAGTTACCCGTGGACG |
| | | | SEQ ID NO:1439 | SEQ ID NO:9451 | SEQ ID NO:17463 |
| | | AA | RASQGIRKDLG | AASSLQS | LQHNSYPWT |
| | | | SEQ ID NO:1440 | SEQ ID NO:9452 | SEQ ID NO:17464 |

FIGURE 49

| iPS:434205 | | NA | AAGTCTAGTCAGAGCCTCCT GCATAGTGAAGGAAAGACC TATTTGTAT | GAAGTTTCCAACCGGAT CTCT | ATGCAAAGTATACAGCTTCC GCTCACT |
|---|---|---|---|---|---|
| | 21-225_60G2 | | SEQ ID NO:1441 | SEQ ID NO:9453 | SEQ ID NO:17465 |
| | | AA | KSSQSLLHSEGKTYLY | EVSNRIS | MQSIQLPLT |
| | | | SEQ ID NO:1442 | SEQ ID NO:9454 | SEQ ID NO:17466 |
| iPS:434207 | | NA | CGGGCAAGTCAGGGCATTAG AAATGATTTAGGC | GCTGCATTTAGTTTGCAA AGT | CTACAGCATAATAGTTACCC TTTCACT |
| | 21-225_60A3 | | SEQ ID NO:1443 | SEQ ID NO:9455 | SEQ ID NO:17467 |
| | | AA | RASQGIRNDLG | AAFSLQS | LQHNSYPFT |
| | | | SEQ ID NO:1444 | SEQ ID NO:9456 | SEQ ID NO:17468 |
| iPS:434209 | | NA | CGGGCAAGTCAGGGCATTAG AAATGATTTAGGC | TCTGCATCCAGTTTACAA AGT | CTACAGCATAATAGTTACCC GTGGACG |
| | 21-225_60C3 | | SEQ ID NO:1445 | SEQ ID NO:9457 | SEQ ID NO:17469 |
| | | AA | RASQGIRNDLG | SASSLQS | LQHNSYPWT |
| | | | SEQ ID NO:1446 | SEQ ID NO:9458 | SEQ ID NO:17470 |
| iPS:434211 | | NA | AAGTCCAGCCAGAGTGTTTT ATACAGCTCCAACAATAAGA ACTACTTAGCT | TGGGCATCTACCCGGGA ATCC | CAGCAATATTATAGTACTCC GTGCAGT |
| | 21-225_60F3 | | SEQ ID NO:1447 | SEQ ID NO:9459 | SEQ ID NO:17471 |
| | | AA | KSSQSVLYSSNNKNYLA | WASTRES | QQYYSTPCS |
| | | | SEQ ID NO:1448 | SEQ ID NO:9460 | SEQ ID NO:17472 |
| iPS:434213 | | NA | CGGGCGAGTCAGGGCATTAG CAATTACTTAGCC | GCTGCATCCAGTTTGCAA AGT | CAACAATATAAAAGTCACCC ATTCACT |
| | 21-225_60A4 | | SEQ ID NO:1449 | SEQ ID NO:9461 | SEQ ID NO:17473 |
| | | AA | RASQGISNYLA | AASSLQS | QQYKSHPFT |
| | | | SEQ ID NO:1450 | SEQ ID NO:9462 | SEQ ID NO:17474 |
| iPS:434215 | | NA | CGGGCGAGTCAGGTCATTAA GAATTATTTAGTC | GCTGCGTCCAGTTTGCAA AGT | CTACAGTTTCATAGTTACCC ATTCACT |
| | 21-225_60F7 | | SEQ ID NO:1451 | SEQ ID NO:9463 | SEQ ID NO:17475 |

698

FIGURE 49

| | | AA | RASQVIKNYLV | AASSLQS | LQFHSYPFT |
|---|---|---|---|---|---|
| | | | SEQ ID NO:1452 | SEQ ID NO:9464 | SEQ ID NO:17476 |
| iPS:434217 | | NA | CGGGCAAGTCAGGGCATTAG AAATGATTTAGGC | GCTGCATCCAGTTTGCAA AGT | CTACAGCATAGTAGTTACCC GCTCACT |
| | 21-225_60E8 | | SEQ ID NO:1453 | SEQ ID NO:9465 | SEQ ID NO:17477 |
| | | AA | RASQGIRNDLG | AASSLQS | LQHSSYPLT |
| | | | SEQ ID NO:1454 | SEQ ID NO:9466 | SEQ ID NO:17478 |
| iPS:434219 | | NA | AGGGCCAGTCAGAGTGTTAG CAGTTCCTTAGCC | GGTGCATCCACCAGGGC CACT | CAGCAGTATAATAACTGGCC ATTCACT |
| | 21-225_60E9 | | SEQ ID NO:1455 | SEQ ID NO:9467 | SEQ ID NO:17479 |
| | | AA | RASQSVSSSLA | GASTRAT | QQYNNWPFT |
| | | | SEQ ID NO:1456 | SEQ ID NO:9468 | SEQ ID NO:17480 |
| iPS:434221 | | NA | CGGGCGAGTCAGGTTATTAG CAACTGGTTAGCC | ACTGCATCCAGTTTGCAA AGT | CAACAGGCTAACAGTTTCCC GTGGACG |
| | 21-225_60A11 | | SEQ ID NO:1457 | SEQ ID NO:9469 | SEQ ID NO:17481 |
| | | AA | RASQVISNWLA | TASSLQS | QQANSFPWT |
| | | | SEQ ID NO:1458 | SEQ ID NO:9470 | SEQ ID NO:17482 |
| iPS:434223 | | NA | AAGTCTAGTCAGAGCCTCCT GCATAGTGAGGGAAAGACC TATTTGTAT | GAAGTTTCCAACCGGTTC TCT | ATGCAAAGTATAAAGTATCC GCTCACT |
| | 21-225_60C12 | | SEQ ID NO:1459 | SEQ ID NO:9471 | SEQ ID NO:17483 |
| | | AA | KSSQSLLHSEGKTYLY | EVSNRFS | MQSIKYPLT |
| | | | SEQ ID NO:1460 | SEQ ID NO:9472 | SEQ ID NO:17484 |
| iPS:434225 | | NA | CGGGCAAGTCAGAGCATTAG CAGCTATTTAAAT | GCTGCATCCAGTTTGCAA AGT | CAACAGAGTTACAATATTTC ATTCACT |
| | 21-225_60E12 | | SEQ ID NO:1461 | SEQ ID NO:9473 | SEQ ID NO:17485 |
| | | AA | RASQSISSYLN | AASSLQS | QQSYNISFT |
| | | | SEQ ID NO:1462 | SEQ ID NO:9474 | SEQ ID NO:17486 |
| iPS:434227 | | NA | CGGGCAAGTCAGAGCATTAG CAGCTATTTAAAT | GCTGCATCCAGTTTGCAA AGT | CAACAGAGTTACAATATTTC ATTCACT |

FIGURE 49

| | 21-225_61A1 | | SEQ ID NO:1463 | SEQ ID NO:9475 | SEQ ID NO:17487 |
|---|---|---|---|---|---|
| | | AA | RASQSISSYLN | AASSLQS | QQSYNISFT |
| iPS:434229 | 21-225_61H1 | NA | SEQ ID NO:1464 CGGGCAAGTCAGGGCATTAG AAAAGATTTAGGC | SEQ ID NO:9476 GCTGCATCCAGTTTGCAA AGT | SEQ ID NO:17488 CTACAGCATAATAGTTACCC GTGGACG |
| | | AA | SEQ ID NO:1465 RASQGIRKDLG | SEQ ID NO:9477 AASSLQS | SEQ ID NO:17489 LQHNSYPWT |
| iPS:434231 | 21-225_61F2 | NA | SEQ ID NO:1466 CGGGCAAGTCAGGGCATTAG AGATGATTTAGGC | SEQ ID NO:9478 GCTGCATCCAGTTTGCAA AGT | SEQ ID NO:17490 CTACAGCATTATAGTTACCC TCGCAGT |
| | | AA | SEQ ID NO:1467 RASQGIRDDLG | SEQ ID NO:9479 AASSLQS | SEQ ID NO:17491 LQHYSYPRS |
| iPS:434233 | 21-225_61B3 | NA | SEQ ID NO:1468 AAGTCTAGTCAGAGCCTCCT GCATAGTGAAGGAAAGACC TATTTGTAT | SEQ ID NO:9480 GAAGTTTCCAACCGGAT CTCT | SEQ ID NO:17492 ATGCAAAGTATACAGCTTCC GCTCACT |
| | | AA | SEQ ID NO:1469 KSSQSLLHSEGKTYLY | SEQ ID NO:9481 EVSNRIS | SEQ ID NO:17493 MQSIQLPLT |
| iPS:434235 | 21-225_61E3 | NA | SEQ ID NO:1470 AAGTCCAGCCAGAGTGTTTT ACACATCTCCAACAATAACA ATTACTTAGCT | SEQ ID NO:9482 TGGGCATCTATCCGGGA ATCC | SEQ ID NO:17494 CAGCAATATTATAGTATTCC GTGCAGT |
| | | AA | SEQ ID NO:1471 KSSQSVLHISNNNYLA | SEQ ID NO:9483 WASIRES | SEQ ID NO:17495 QQYYSIPCS |
| iPS:434237 | 21-225_61B5 | NA | SEQ ID NO:1472 AAGTCCAGCCAGAGTGTTTT ATACAGCTCCAACAATAACA ACTCCTTAACT | SEQ ID NO:9484 TGGGCATCTACCCGGGA ATCC | SEQ ID NO:17496 CAGCAATATTATAGTACTCC TCCGACG |
| | | AA | SEQ ID NO:1473 KSSQSVLYSSNNNNSLT | SEQ ID NO:9485 WASTRES | SEQ ID NO:17497 QQYYSTPPT |

FIGURE 49

| | | | SEQ ID NO:1474 | SEQ ID NO:9486 | SEQ ID NO:17498 |
|---|---|---|---|---|---|
| iPS:434239 | | NA | CGGGCAAGTCAGAGCATTAC CAACTTTTTAAAT | GCTGCATCCAGTTTGCAA AGT | CAACAGAGTTACAGTATCCC GTGGACG |
| | 21-225_58F1 | | SEQ ID NO:1475 | SEQ ID NO:9487 | SEQ ID NO:17499 |
| | | AA | RASQSITNFLN | AASSLQS | QQSYSIPWT |
| | | | SEQ ID NO:1476 | SEQ ID NO:9488 | SEQ ID NO:17500 |
| iPS:434241 | | NA | CGGGCAAGTCAGGGCATTGG AAATGATTTAGGC | GCTGCATCCAGTTTGCAA AGT | CTACAACATAATAGTTTCCC TCCGTGGACG |
| | 21-225_61E6 | | SEQ ID NO:1477 | SEQ ID NO:9489 | SEQ ID NO:17501 |
| | | AA | RASQGIGNDLG | AASSLQS | LQHNSFPPWT |
| | | | SEQ ID NO:1478 | SEQ ID NO:9490 | SEQ ID NO:17502 |
| iPS:434243 | | NA | AGGTCTAGTCAGAGCCTCCT ACATAGTAATGGATACAACT ATTTGGAT | TTGGTTTCTAATCGGGCC TCC | CTGCAAGCTCTACAAACTCC TCTCACC |
| | 21-225_62C1 | | SEQ ID NO:1479 | SEQ ID NO:9491 | SEQ ID NO:17503 |
| | | AA | RSSQSLLHSNGYNYLD | LVSNRAS | LQALQTPLT |
| | | | SEQ ID NO:1480 | SEQ ID NO:9492 | SEQ ID NO:17504 |
| iPS:434245 | | NA | CGGGCAAGTCAGAACATTTT CAGCTATTTAAAT | GCTGTATTTAGTTTGCAA AGT | CAACAGAGTTACAGTACCCC ATTCACT |
| | 21-225_62H1 | | SEQ ID NO:1481 | SEQ ID NO:9493 | SEQ ID NO:17505 |
| | | AA | RASQNIFSYLN | AVFSLQS | QQSYSTPFT |
| | | | SEQ ID NO:1482 | SEQ ID NO:9494 | SEQ ID NO:17506 |
| iPS:434247 | | NA | CGGGCAAGTCAGAGCATTAT CAGTTATTTAAAT | GCTACATCCAGTTTGCAA AGT | CAACAGACTTACAGTCCCCC GCTCACT |
| | 21-225_62D2 | | SEQ ID NO:1483 | SEQ ID NO:9495 | SEQ ID NO:17507 |
| | | AA | RASQSIISYLN | ATSSLQS | QQTYSPPLT |
| | | | SEQ ID NO:1484 | SEQ ID NO:9496 | SEQ ID NO:17508 |
| iPS:434249 | | NA | CGGGCAAGTCAGGGCATTAG AAATGATTTAGGC | GCTGCATCCCGTTTGCAA AGT | CTACAGCATAGTAATTACCC TCTCACT |
| | 21-225_62E2 | | SEQ ID NO:1485 | SEQ ID NO:9497 | SEQ ID NO:17509 |

FIGURE 49

| | | AA | RASQGIRNDLG | AASRLQS | LQHSNYPLT |
|---|---|---|---|---|---|
| | | | SEQ ID NO:1486 | SEQ ID NO:9498 | SEQ ID NO:17510 |
| iPS:434251 | | NA | CGGGCAAGTCAGGACATTAG AAATAATTTAGGC | ACTGCATCCAGTTTGCAA AGT | CTACAGTATAATAGTTACCC ATTCACT |
| | 21-225_62G3 | | SEQ ID NO:1487 | SEQ ID NO:9499 | SEQ ID NO:17511 |
| | | AA | RASQDIRNNLG | TASSLQS | LQYNSYPFT |
| | | | SEQ ID NO:1488 | SEQ ID NO:9500 | SEQ ID NO:17512 |
| iPS:434253 | | NA | CGGGCAAGTCAGGGCATTAG AAATGATTTAGGC | GCTGCATTCAGTTTGCAA AGT | CTACAGCATAATAGTTATCC GCTCACT |
| | 21-225_62E4 | | SEQ ID NO:1489 | SEQ ID NO:9501 | SEQ ID NO:17513 |
| | | AA | RASQGIRNDLG | AAFSLQS | LQHNSYPLT |
| | | | SEQ ID NO:1490 | SEQ ID NO:9502 | SEQ ID NO:17514 |
| iPS:434255 | | NA | AGGGCCAGTCAGAGTGTTAA CAGCAACTTAGCC | GTTGCATCCACCAGGGC CACT | CAGCAGTATAATGACTGGCC GTGTAGT |
| | 21-225_62E6 | | SEQ ID NO:1491 | SEQ ID NO:9503 | SEQ ID NO:17515 |
| | | AA | RASQSVNSNLA | VASTRAT | QQYNDWPCS |
| | | | SEQ ID NO:1492 | SEQ ID NO:9504 | SEQ ID NO:17516 |
| iPS:434257 | | NA | CGGGCAAGTCAGGCCATTAG AAATGATTTAGGC | CCTGCATCCCGTTTGCAA AGT | CTACAGTATAATAGTTACCC TCCGTGGACG |
| | 21-225_62F7 | | SEQ ID NO:1493 | SEQ ID NO:9505 | SEQ ID NO:17517 |
| | | AA | RASQAIRNDLG | PASRLQS | LQYNSYPPWT |
| | | | SEQ ID NO:1494 | SEQ ID NO:9506 | SEQ ID NO:17518 |
| iPS:434259 | | NA | CGGGCGAGTCAGGATATTAG CAGCTGGTTAGCC | GCTGCATCCAGTTTGCAA AGT | CAACAGACTAACAGTTTCCC TCTCACT |
| | 21-225_62G7 | | SEQ ID NO:1495 | SEQ ID NO:9507 | SEQ ID NO:17519 |
| | | AA | RASQDISSWLA | AASSLQS | QQTNSFPLT |
| | | | SEQ ID NO:1496 | SEQ ID NO:9508 | SEQ ID NO:17520 |
| iPS:434261 | | NA | CGGGCGAGTCAGGGCATTAG CACTTATTTAGGC | GCTGCATCCAGTTTGCAA GGT | CATCAGTATAATAGTTTCCC ATTTAAG |
| | 21-225_56F7 | | SEQ ID NO:1497 | SEQ ID NO:9509 | SEQ ID NO:17521 |

FIGURE 49

| | | | | | |
|---|---|---|---|---|---|
| | | AA | RASQGISTYLA | AASSLQG | HQYNSFPFK |
| | | | SEQ ID NO:1498 | SEQ ID NO:9510 | SEQ ID NO:17522 |
| iPS:434263 | | NA | CGGGCAAGTCAGGACATTAGAAATGATTTAGGC | CCTGCATCCAGTTTGCTAAGT | CTACAGGATAATAGTTACCCATTCACT |
| | 21-225_56H7 | | SEQ ID NO:1499 | SEQ ID NO:9511 | SEQ ID NO:17523 |
| | | AA | RASQDIRNDLG | PASSLLS | LQDNSYPFT |
| | | | SEQ ID NO:1500 | SEQ ID NO:9512 | SEQ ID NO:17524 |
| iPS:434265 | | NA | CGGGCAAGTCAGGGCATTAGAAATGCTTTAGGC | GCTGCATCCAGTTTGCAAAGT | CTACAGCATAATAGTTACCCATTCACT |
| | 21-225_57B2 | | SEQ ID NO:1501 | SEQ ID NO:9513 | SEQ ID NO:17525 |
| | | AA | RASQGIRNALG | AASSLQS | LQHNSYPFT |
| | | | SEQ ID NO:1502 | SEQ ID NO:9514 | SEQ ID NO:17526 |
| iPS:434267 | | NA | CGGGCAAGTCAGAGCATTAGCAGCTATTTAAAT | GCTGCATCCAGTTTGCAAAGT | CAACAGAGTTACAATATTTCATTCACT |
| | 21-225_57F2 | | SEQ ID NO:1503 | SEQ ID NO:9515 | SEQ ID NO:17527 |
| | | AA | RASQSISSYLN | AASSLQS | QQSYNISFT |
| | | | SEQ ID NO:1504 | SEQ ID NO:9516 | SEQ ID NO:17528 |
| iPS:434269 | | NA | AGGGCCAGTCAGAGTGTTAGCAGCAGCTTAGCC | GGTGCATCCACCAGGGCCACT | CAGCAGTATAATGACTGGCCGTGCAGT |
| | 21-225_57H3 | | SEQ ID NO:1505 | SEQ ID NO:9517 | SEQ ID NO:17529 |
| | | AA | RASQSVSSSLA | GASTRAT | QQYNDWPCS |
| | | | SEQ ID NO:1506 | SEQ ID NO:9518 | SEQ ID NO:17530 |
| iPS:434271 | | NA | CGGGCAAGTCAGGGCATTAGAAATGATTTAGGC | GCTGCATCCAGTTTGCTAAGT | CTACAGCATAATAGTTACCCATTCACT |
| | 21-225_57A4 | | SEQ ID NO:1507 | SEQ ID NO:9519 | SEQ ID NO:17531 |
| | | AA | RASQGIRNDLG | AASSLLS | LQHNSYPFT |
| | | | SEQ ID NO:1508 | SEQ ID NO:9520 | SEQ ID NO:17532 |
| iPS:434273 | | NA | CGGGCGAGTCAGGATATTAGCAACTGGTTAGCC | GCTGCATCCAGTTTGCAAAGT | CAACAGGGTAACAGTTTCCCATTCACT |
| | 21-225_57E4 | | SEQ ID NO:1509 | SEQ ID NO:9521 | SEQ ID NO:17533 |

FIGURE 49

| | | | | | |
|---|---|---|---|---|---|
| | | AA | RASQDISNWLA | AASSLQS | QQGNSFPFT |
| | | | SEQ ID NO:1510 | SEQ ID NO:9522 | SEQ ID NO:17534 |
| iPS:434275 | | NA | CGGGCAAGTCAGGTCATTAG AAATGATTTAGGC | GCTGCATCCAGTTTGCAA AGT | CTACAGTATGGTAGTTTCCC ATTCACT |
| | 21-225_57F4 | | SEQ ID NO:1511 | SEQ ID NO:9523 | SEQ ID NO:17535 |
| | | AA | RASQVIRNDLG | AASSLQS | LQYGSFPFT |
| | | | SEQ ID NO:1512 | SEQ ID NO:9524 | SEQ ID NO:17536 |
| iPS:434277 | | NA | CGGGCGAGTCAGGGTATTAG CAGGTGGTTAGCC | GCTGCATCCAATTTGCAA AGT | CAACAGGCTAACAGTTTCCC ATTCACT |
| | 21-225_57A7 | | SEQ ID NO:1513 | SEQ ID NO:9525 | SEQ ID NO:17537 |
| | | AA | RASQGISRWLA | AASNLQS | QQANSFPFT |
| | | | SEQ ID NO:1514 | SEQ ID NO:9526 | SEQ ID NO:17538 |
| iPS:434279 | | NA | AGGGCCAGTCAGAGTGTTAG CAGCGACTTAGCC | GGTGCATCCACCAGGGC CACT | CAGCAGTATAGTAACTGGCC ATTCACT |
| | 21-225_57F7 | | SEQ ID NO:1515 | SEQ ID NO:9527 | SEQ ID NO:17539 |
| | | AA | RASQSVSSDLA | GASTRAT | QQYSNWPFT |
| | | | SEQ ID NO:1516 | SEQ ID NO:9528 | SEQ ID NO:17540 |
| iPS:434281 | | NA | CGGGCAAGTCAGGGCATTGG AAATGATTTAGGC | GCTGCATCCAGTTTGCAA AGT | CTACAACATAATAGTTTCCC TCCGTGGACG |
| | 21-225_57B8 | | SEQ ID NO:1517 | SEQ ID NO:9529 | SEQ ID NO:17541 |
| | | AA | RASQGIGNDLG | AASSLQS | LQHNSFPPWT |
| | | | SEQ ID NO:1518 | SEQ ID NO:9530 | SEQ ID NO:17542 |
| iPS:434283 | | NA | CGGGCGAGTCAGGGTATTAG CAACTGGTTAGCC | ACTGCATCCAGTTGCAA AGT | CAACAGGCTAACAGTTTCCC GTGGACG |
| | 21-225_57F8 | | SEQ ID NO:1519 | SEQ ID NO:9531 | SEQ ID NO:17543 |
| | | AA | RASQGISNWLA | TASSLQS | QQANSFPWT |
| | | | SEQ ID NO:1520 | SEQ ID NO:9532 | SEQ ID NO:17544 |
| iPS:434285 | 21 225 57A11 | NA | AAGTCCAGCCAAAGTGTTTT ACACAGCTCCAACAATTATA ACTACTTAGCT | TGGGCATCTACCCGGGC ATCC | CAGCAATATTATAGTACTCC GTGGACG |

FIGURE 49

| | | | | | |
|---|---|---|---|---|---|
| | 21-225_57A11 | | | SEQ ID NO:1521 | SEQ ID NO:9533 | SEQ ID NO:17545 |
| | | AA | KSSQSVLHSSNNYNYLA | WASTRAS | QQYYSTPWT |
| iPS:434287 | 21-225_57F12 | NA | SEQ ID NO:1522 AAGTCCAGCCAGAGTGTTTT ATTCAGCTCCAACAATTACA ATTACTTAGCT | SEQ ID NO:9534 TGGGCATCTACCCGGGA ATCC | SEQ ID NO:17546 CAGCAATATTATAGTAATCC GTGTAGT |
| | | AA | SEQ ID NO:1523 KSSQSVLFSSNNYNYLA | SEQ ID NO:9535 WASTRES | SEQ ID NO:17547 QQYYSNPCS |
| iPS:434289 | 21-225_57H12 | NA | SEQ ID NO:1524 AGGGCCAGTCAGAGTGTTAG CAGCGACTTAGCC | SEQ ID NO:9536 GCTGCATCTACCAGGGC CACT | SEQ ID NO:17548 CAGCAGTATGATAACTGGCC ATTCACT |
| | | AA | SEQ ID NO:1525 RASQSVSSDLA | SEQ ID NO:9537 AASTRAT | SEQ ID NO:17549 QQYDNWPFT |
| iPS:434291 | 21-225_58A4 | NA | SEQ ID NO:1526 AGGGCCAGTCAGAGTGTTAG CAGCGACTTAGCC | SEQ ID NO:9538 GCTGCATCTACCAGGGC CACT | SEQ ID NO:17550 CAGCAGTTTAATAACTGGCC ATTCACT |
| | | AA | SEQ ID NO:1527 RASQSVSSDLA | SEQ ID NO:9539 AASTRAT | SEQ ID NO:17551 QQFNNWPFT |
| iPS:434293 | 21-225_58F5 | NA | SEQ ID NO:1528 CGGGCAAGTCAGGGCATTAG AAATGATTTAGGC | SEQ ID NO:9540 GCTGCATCCAGTTTGCTA AGT | SEQ ID NO:17552 CTACAGCATAATAGTTACCC ATTCACT |
| | | AA | SEQ ID NO:1529 RASQGIRNDLG | SEQ ID NO:9541 AASSLLS | SEQ ID NO:17553 LQHNSYPFT |
| iPS:434295 | 21-225_58B9 | NA | SEQ ID NO:1530 AAGTCCGGCCAGAGTATTTT ATACAGCTCCAACAATAACA ACTACTTAGCT | SEQ ID NO:9542 TGGGCATCTACCCGGGA TTCC | SEQ ID NO:17554 CAGCAATATTATAGTACTCC TCCGACG |
| | | AA | SEQ ID NO:1531 KSGQSILYSSNNNNYLA | SEQ ID NO:9543 WASTRDS | SEQ ID NO:17555 QQYYSTPPT |
| | | | SEQ ID NO:1532 | SEQ ID NO:9544 | SEQ ID NO:17556 |

FIGURE 49

| iPS:434297 | | NA | AGGGCCAGTCAGAGTGTTAG CAGCTCCTTAGCC | GGTGCATCCACCAGGGC CACT | CAGCAGTATAATAACTGGCC ATTCACT |
|---|---|---|---|---|---|
| | 21-225_58A10 | | SEQ ID NO:1533 | SEQ ID NO:9545 | SEQ ID NO:17557 |
| | | AA | RASQSVSSSLA | GASTRAT | QQYNNWPFT |
| | | | SEQ ID NO:1534 | SEQ ID NO:9546 | SEQ ID NO:17558 |
| iPS:434299 | | NA | CGGGCAAGTCAGGGCATTAG AAGTGATTTAGAC | GCTGCATCCAGTTTGCAA AGT | CTCCAGCATAATAATTTCCC ATTCACT |
| | 21-225_58D11 | | SEQ ID NO:1535 | SEQ ID NO:9547 | SEQ ID NO:17559 |
| | | AA | RASQGIRSDLD | AASSLQS | LQHNNFPFT |
| | | | SEQ ID NO:1536 | SEQ ID NO:9548 | SEQ ID NO:17560 |
| iPS:434301 | | NA | AGGGCCAGTCAGAGTGTTAG CAGCGACTTAGTC | GGTGTATCCACCAGGGC CACT | CAGCAGTATAATAACTGGCC ATTCACT |
| | 21-225_58F11 | | SEQ ID NO:1537 | SEQ ID NO:9549 | SEQ ID NO:17561 |
| | | AA | RASQSVSSDLV | GVSTRAT | QQYNNWPFT |
| | | | SEQ ID NO:1538 | SEQ ID NO:9550 | SEQ ID NO:17562 |
| iPS:434303 | | NA | AAGTCTAGTCAGAGCCTCCT GCATAGTGAGGGAAAGACC TATTTGTAT | GAAGTTTCCTATCGGTTT TCT | ATGCAAAGTATACAGCTTCC GCTCACT |
| | 21-225_58H11 | | SEQ ID NO:1539 | SEQ ID NO:9551 | SEQ ID NO:17563 |
| | | AA | KSSQSLLHSEGKTYLY | EVSYRFS | MQSIQLPLT |
| | | | SEQ ID NO:1540 | SEQ ID NO:9552 | SEQ ID NO:17564 |
| iPS:434305 | | NA | AAGTCCAGCCAGAGTGTTTT ATACAGCTCCAACAATAACA ACTACTTAGCT | TGGTCATCTACCCGGGA ATCC | CAGCAATATTTTAGTATTCC GTGCAGT |
| | 21-225_59E1 | | SEQ ID NO:1541 | SEQ ID NO:9553 | SEQ ID NO:17565 |
| | | AA | KSSQSVLYSSNNNNYLA | WSSTRES | QQYFSIPCS |
| | | | SEQ ID NO:1542 | SEQ ID NO:9554 | SEQ ID NO:17566 |
| iPS:434307 | | NA | TGGGCCAGTCAGAGTGTTTA CAGCAGCTTCTTAGCC | GGTGCATCCAGCAGGGC CACT | CAGCAATATGGTACCTCACC GTGGACG |
| | 21-225_59B2 | | SEQ ID NO:1543 | SEQ ID NO:9555 | SEQ ID NO:17567 |

706

FIGURE 49

| | | | | | |
|---|---|---|---|---|---|
| | | AA | WASQSVYSSFLA | GASSRAT | QQYGTSPWT |
| | | | SEQ ID NO:1544 | SEQ ID NO:9556 | SEQ ID NO:17568 |
| iPS:434309 | | NA | CGGGCAAGTCAGAGCATTAT CAGCTATTTAAAT | GGTGCATCCAGTTTGCA GAGT | CAACAGAGTTACAGTACCCC TATGTTCAGT |
| | 21-225_59B5 | | SEQ ID NO:1545 | SEQ ID NO:9557 | SEQ ID NO:17569 |
| | | AA | RASQSIISYLN | GASSLQS | QQSYSTPMFS |
| | | | SEQ ID NO:1546 | SEQ ID NO:9558 | SEQ ID NO:17570 |
| iPS:434311 | | NA | AGGGCCAGTCAGAGTGTTAG CAGCATCTACTTAGCC | GGTGCATCCAGCAGGGC CACT | CACCAGTATGGTAACTCACC ATTCACT |
| | 21-225_59H5 | | SEQ ID NO:1547 | SEQ ID NO:9559 | SEQ ID NO:17571 |
| | | AA | RASQSVSSIYLA | GASSRAT | HQYGNSPFT |
| | | | SEQ ID NO:1548 | SEQ ID NO:9560 | SEQ ID NO:17572 |
| iPS:434313 | | NA | CGGGCAAGTCAGGGCATTAG AAATGATTTAGGC | GCTGCATCCAGTTTGCAA AGT | CTACAGCATAATAGTTACCC GCTCACT |
| | 21-225_59E6 | | SEQ ID NO:1549 | SEQ ID NO:9561 | SEQ ID NO:17573 |
| | | AA | RASQGIRNDLG | AASSLQS | LQHNSYPLT |
| | | | SEQ ID NO:1550 | SEQ ID NO:9562 | SEQ ID NO:17574 |
| iPS:434315 | | NA | CGGGCAAGTCAGGGCATTAG AAATGATTTAGGC | TCTGCATCCAGTTTACAA AGT | CTACAGCATAATAGTTACCC GTGGACG |
| | 21-225_59G7 | | SEQ ID NO:1551 | SEQ ID NO:9563 | SEQ ID NO:17575 |
| | | AA | RASQGIRNDLG | SASSLQS | LQHNSYPWT |
| | | | SEQ ID NO:1552 | SEQ ID NO:9564 | SEQ ID NO:17576 |
| iPS:434317 | | NA | CGGGCAAGTCAGAGCATTAG CAGCTATTTAAAT | GCTGCATCCAGTTTGCAA AGT | CAACAGAGTTTCAGTAATTC GATCACC |
| | 21-225_59E8 | | SEQ ID NO:1553 | SEQ ID NO:9565 | SEQ ID NO:17577 |
| | | AA | RASQSISSYLN | AASSLQS | QQSFSNSIT |
| | | | SEQ ID NO:1554 | SEQ ID NO:9566 | SEQ ID NO:17578 |
| iPS:434319 | | NA | CGGGCAAGTCAGGACATTAG AAATGATTTAGGC | GCTGCATCCAGTTTACAA AGT | CTACAGCATAATAGTTACCC GTGGACG |
| | 21-225_59B9 | | SEQ ID NO:1555 | SEQ ID NO:9567 | SEQ ID NO:17579 |

FIGURE 49

| | | AA | RASQDIRNDLG | AASSLQS | LQHNSYPWT |
|---|---|---|---|---|---|
| | | | SEQ ID NO:1556 | SEQ ID NO:9568 | SEQ ID NO:17580 |
| iPS:434321 | 21-225_59F10 | NA | AAGTCCAGCCAGACTGTTTTATACAGGTCCAACAATTACAACTACTTAGCT | TGGGCATCTACCCGGGAATCC | CAGCAATATTTTAGTACTCCTCCGACG |
| | | | SEQ ID NO:1557 | SEQ ID NO:9569 | SEQ ID NO:17581 |
| | | AA | KSSQTVLYRSNNYNYLA | WASTRES | QQYFSTPPT |
| | | | SEQ ID NO:1558 | SEQ ID NO:9570 | SEQ ID NO:17582 |
| iPS:434323 | 21-225_62H8 | NA | CGGGCAAGTCAGAGCATTTTCAGCTATTTAAAT | GCGTCATCCAGTTTGCAAAGT | CAACAGAGTTACAGTACCCCATTCACT |
| | | | SEQ ID NO:1559 | SEQ ID NO:9571 | SEQ ID NO:17583 |
| | | AA | RASQSIFSYLN | ASSSLQS | QQSYSTPFT |
| | | | SEQ ID NO:1560 | SEQ ID NO:9572 | SEQ ID NO:17584 |
| iPS:434327 | 21-225_63G6 | NA | CGGGCAAGTCAGAGCATTTTCAGCTATTTAAAT | GATACATCCACTTTGCAAACT | CAACAGAGTTACGGTATCCCCATCACC |
| | | | SEQ ID NO:1561 | SEQ ID NO:9573 | SEQ ID NO:17585 |
| | | AA | RASQSIFSYLN | DTSTLQT | QQSYGIPIT |
| | | | SEQ ID NO:1562 | SEQ ID NO:9574 | SEQ ID NO:17586 |
| iPS:434331 | 21-225_63H8 | NA | CGGGCGAGTCAGGGCATTAGCAATTATTTAGCC | GCTGCATCCAGTTTGCAAAGT | CAACAATATCATAGTTACCCATTCACT |
| | | | SEQ ID NO:1563 | SEQ ID NO:9575 | SEQ ID NO:17587 |
| | | AA | RASQGISNYLA | AASSLQS | QQYHSYPFT |
| | | | SEQ ID NO:1564 | SEQ ID NO:9576 | SEQ ID NO:17588 |
| iPS:434333 | 21-225_63C9 | NA | CGGGCGAGTCAGGGTATTAGCAGCTGGTTAGCC | GCTGCATCCAGTTTGCAAAGT | CAACAGATTAACAGTTTCCCTCTCACT |
| | | | SEQ ID NO:1565 | SEQ ID NO:9577 | SEQ ID NO:17589 |
| | | AA | RASQGISSWLA | AASSLQS | QQINSFPLT |
| | | | SEQ ID NO:1566 | SEQ ID NO:9578 | SEQ ID NO:17590 |
| iPS:434335 | | NA | CGGGCAAGTCAGAGCATTTTCAGCTATTTACAT | GCTGCATCCAGTTTACAAAGT | CAACAGACTTACAGTCCCCCGCTCACT |

FIGURE 49

| | 21-225_63C10 | | SEQ ID NO:1567 | SEQ ID NO:9579 | SEQ ID NO:17591 |
|---|---|---|---|---|---|
| | | AA | RASQSIFSYLH | AASSLQS | QQTYSPPLT |
| | | | SEQ ID NO:1568 | SEQ ID NO:9580 | SEQ ID NO:17592 |
| iPS:434337 | | NA | CGGGCAAGTCAGGGCATTAGAAATGATTTAGGC | GCTGCATCCAGTTTGCAAAGT | CTACAGCATAATAGTTACCCGCTCACT |
| | 21-225_64E1 | | SEQ ID NO:1569 | SEQ ID NO:9581 | SEQ ID NO:17593 |
| | | AA | RASQGIRNDLG | AASSLQS | LQHNSYPLT |
| | | | SEQ ID NO:1570 | SEQ ID NO:9582 | SEQ ID NO:17594 |
| iPS:434339 | | NA | CGGGCAAGTCAGGGCATTAGAAATGATTTAGGC | GCTGCATCCAGTTTGCAAAGT | CTACAGCATTATAGTTACCCTCGGACG |
| | 21-225_64A4 | | SEQ ID NO:1571 | SEQ ID NO:9583 | SEQ ID NO:17595 |
| | | AA | RASQGIRNDLG | AASSLQS | LQHYSYPRT |
| | | | SEQ ID NO:1572 | SEQ ID NO:9584 | SEQ ID NO:17596 |
| iPS:434341 | | NA | CGGGCAAGTCAGAACATTAAGAAATATTTAAAT | GGTGCATCCAGTTTGCAAAGT | CAACAGAGTTACAATATTTCGTTCACT |
| | 21-225_64F7 | | SEQ ID NO:1573 | SEQ ID NO:9585 | SEQ ID NO:17597 |
| | | AA | RASQNIKKYLN | GASSLQS | QQSYNISFT |
| | | | SEQ ID NO:1574 | SEQ ID NO:9586 | SEQ ID NO:17598 |
| iPS:434343 | | NA | CGGGCAAGTCAGGGCATTAGAAATGATTTAGGC | GCTGCATCCAGTTTGCAAAGT | CTACACCATTATAGTTACCCTCGGACG |
| | 21-225_64C8 | | SEQ ID NO:1575 | SEQ ID NO:9587 | SEQ ID NO:17599 |
| | | AA | RASQGIRNDLG | AASSLQS | LHHYSYPRT |
| | | | SEQ ID NO:1576 | SEQ ID NO:9588 | SEQ ID NO:17600 |
| iPS:434345 | | NA | AAGTCTAGTCAGAGCCTCCTTCATGGTGATGGAAAGACCTATTTGTTT | GAAGTTTCCAACCGGTTGTGT | ATGCAAAGTATACAGGTTCCGTGGACG |
| | 21-225_64H9 | | SEQ ID NO:1577 | SEQ ID NO:9589 | SEQ ID NO:17601 |
| | | AA | KSSQSLLHGDGKTYLF | EVSNRLC | MQSIQVPWT |
| | | | SEQ ID NO:1578 | SEQ ID NO:9590 | SEQ ID NO:17602 |

FIGURE 49

| iPS:434347 | | NA | CGGGCGAGTCAGGGTATTAGCAGCTGGTTAGCC | GCTGCATCCAGTTTGCAAAGT | CAACAGATTAACAGTTTCCCTCTCACT |
|---|---|---|---|---|---|
| | 21-225_64H10 | | SEQ ID NO:1579 | SEQ ID NO:9591 | SEQ ID NO:17603 |
| | | AA | RASQGISSWLA | AASSLQS | QQINSFPLT |
| | | | SEQ ID NO:1580 | SEQ ID NO:9592 | SEQ ID NO:17604 |
| iPS:434351 | | NA | CGGGCAAGTCAGGGCATTAGAAATGATTTAGGC | ACTGCATCCACTTTGCAAAGT | CTACAGCATAATGGTTACCCATTCACT |
| | 21-225_64A12 | | SEQ ID NO:1581 | SEQ ID NO:9593 | SEQ ID NO:17605 |
| | | AA | RASQGIRNDLG | TASTLQS | LQHNGYPFT |
| | | | SEQ ID NO:1582 | SEQ ID NO:9594 | SEQ ID NO:17606 |
| iPS:434353 | | NA | CGGGCGAGTCAGGACATTAGCAATTATTTAAAT | GATGCATCCATTTTGGAAACA | CAACAGAGTGATAATCTCCCGTGCAGT |
| | 21-225_64B12 | | SEQ ID NO:1583 | SEQ ID NO:9595 | SEQ ID NO:17607 |
| | | AA | RASQDISNYLN | DASILET | QQSDNLPCS |
| | | | SEQ ID NO:1584 | SEQ ID NO:9596 | SEQ ID NO:17608 |
| iPS:434355 | | NA | CGGGCGAGTCAGAATATTACCACCTGGTTAGCC | GCTGCATCCAGTTTGCAAAGT | CAACAGGCTAACAGTTTTCCATTCACT |
| | 21-225_64G12 | | SEQ ID NO:1585 | SEQ ID NO:9597 | SEQ ID NO:17609 |
| | | AA | RASQNITTWLA | AASSLQS | QQANSFPFT |
| | | | SEQ ID NO:1586 | SEQ ID NO:9598 | SEQ ID NO:17610 |
| iPS:434357 | | NA | CGGGCGAGTCAGGTCATTAGCAGTTATTTACAT | AGTGCATCCAATTTGCAATGT | CAACGGCCTTACAATGCCCCGCTCACT |
| | 21-225_65C1 | | SEQ ID NO:1587 | SEQ ID NO:9599 | SEQ ID NO:17611 |
| | | AA | RASQVISSYLH | SASNLQC | QRPYNAPLT |
| | | | SEQ ID NO:1588 | SEQ ID NO:9600 | SEQ ID NO:17612 |
| iPS:434359 | | NA | CGGGCGAGTCAGGGTATTAGCAGCTGGTTAGCC | GCTGCATCCAGTTTGCAAAGT | CAACAGGTTAACAGTTTCCCTCTCACT |
| | 21-225_65G3 | | SEQ ID NO:1589 | SEQ ID NO:9601 | SEQ ID NO:17613 |
| | | AA | RASQGISSWLA | AASSLQS | QQVNSFPLT |
| | | | SEQ ID NO:1590 | SEQ ID NO:9602 | SEQ ID NO:17614 |

FIGURE 49

| iPS:434361 | | NA | CGGGCGAGTCAGGACATTAA CAATTATTTAGCC | GCTGCATCCAGTTTGCAC AGT | CCACTGTATAAAAGTTATCC ACTCACT |
|---|---|---|---|---|---|
| | 21-225_65D5 | | SEQ ID NO:1591 | SEQ ID NO:9603 | SEQ ID NO:17615 |
| | | AA | RASQDINNYLA | AASSLHS | PLYKSYPLT |
| | | | SEQ ID NO:1592 | SEQ ID NO:9604 | SEQ ID NO:17616 |
| iPS:434363 | | NA | AGGGCCAGTCAGAGTGTTAA CAGCAACTTAGCC | GGTGCATCCACCAGGGC CACT | CAGCAGTATAATGACTGGCC GTGCAGT |
| | 21-225_65A6 | | SEQ ID NO:1593 | SEQ ID NO:9605 | SEQ ID NO:17617 |
| | | AA | RASQSVNSNLA | GASTRAT | QQYNDWPCS |
| | | | SEQ ID NO:1594 | SEQ ID NO:9606 | SEQ ID NO:17618 |
| iPS:434367 | | NA | CGGGCGAGTCAGGACATTAG CACTTATTTAGCC | GCTGCATCCAGTTTGCAA AGT | CAACAGTATAATAGTTTCCC TCTCACT |
| | 21-225_65H11 | | SEQ ID NO:1595 | SEQ ID NO:9607 | SEQ ID NO:17619 |
| | | AA | RASQDISTYLA | AASSLQS | QQYNSFPLT |
| | | | SEQ ID NO:1596 | SEQ ID NO:9608 | SEQ ID NO:17620 |
| iPS:434369 | | NA | CGGGCGAGTCAGGGTATTAG CAGCTGGTTAGCC | GCTGCATCCAGTTTGCAA AGT | CAACAGACTAACAGTTTCCC TCTCACT |
| | 21-225_66B1 | | SEQ ID NO:1597 | SEQ ID NO:9609 | SEQ ID NO:17621 |
| | | AA | RASQGISSWLA | AASSLQS | QQTNSFPLT |
| | | | SEQ ID NO:1598 | SEQ ID NO:9610 | SEQ ID NO:17622 |
| iPS:434373 | | NA | CGGGCGAGTCAGGGTATTAG CAGCTGGTTAGCC | GCTGCATCCAGTTTGCAA AGT | CAACAGATTAATAGTTTCCC TCTCACT |
| | 21-225_66A7 | | SEQ ID NO:1599 | SEQ ID NO:9611 | SEQ ID NO:17623 |
| | | AA | RASQGISSWLA | AASSLQS | QQINSFPLT |
| | | | SEQ ID NO:1600 | SEQ ID NO:9612 | SEQ ID NO:17624 |
| iPS:434375 | | NA | CGGGCGAGTCAGGGCATTAG CAATTATTTACAT | TGTGCATCCAATTTACAA TGT | CAACAGCATAATAATTCCCC GCTCACT |
| | 21-225_66C7 | | SEQ ID NO:1601 | SEQ ID NO:9613 | SEQ ID NO:17625 |
| | | AA | RASQGISNYLH | CASNLQC | QQHNNSPLT |
| | | | SEQ ID NO:1602 | SEQ ID NO:9614 | SEQ ID NO:17626 |

FIGURE 49

| iPS:434379 | | NA | CGGGCAAGTCAGAACATTTT CAGCTATTTAAAT | GCTGCATCCAGTTTACAA AGT | CAACAGACTTACAGTGTCCC TTTCACT |
|---|---|---|---|---|---|
| | 21-225_66A9 | | SEQ ID NO:1603 | SEQ ID NO:9615 | SEQ ID NO:17627 |
| | | AA | RASQNIFSYLN | AASSLQS | QQTYSVPFT |
| | | | SEQ ID NO:1604 | SEQ ID NO:9616 | SEQ ID NO:17628 |
| iPS:434383 | | NA | CGGGCAAGTCAGGACATTAG AAATGTTTTAGGC | ACTGCATCCAGTTTACAA AGT | CTACAGTATAATAGTTACCC ATTCACT |
| | 21-225_66F9 | | SEQ ID NO:1605 | SEQ ID NO:9617 | SEQ ID NO:17629 |
| | | AA | RASQDIRNVLG | TASSLQS | LQYNSYPFT |
| | | | SEQ ID NO:1606 | SEQ ID NO:9618 | SEQ ID NO:17630 |
| iPS:434385 | | NA | CGGGCAAGTCAGGCCATTAG AAATGATTTAGGC | CCTGCATCCAGTTTGCAA AGT | CTACAGTATAATAGTTACCC TCCGTGGACG |
| | 21-225_66C10 | | SEQ ID NO:1607 | SEQ ID NO:9619 | SEQ ID NO:17631 |
| | | AA | RASQAIRNDLG | PASSLQS | LQYNSYPPWT |
| | | | SEQ ID NO:1608 | SEQ ID NO:9620 | SEQ ID NO:17632 |
| iPS:434387 | | NA | CGGGCAAGTCAGGGCATTAG AAATGATTTAGGT | GCTGCATCCAGTTGTCAA AGT | ATAGTGCATAATAGTTACCC TCGGACG |
| | 21-225_66D11 | | SEQ ID NO:1609 | SEQ ID NO:9621 | SEQ ID NO:17633 |
| | | AA | RASQGIRNDLG | AASSCQS | IVHNSYPRT |
| | | | SEQ ID NO:1610 | SEQ ID NO:9622 | SEQ ID NO:17634 |
| iPS:434389 | | NA | CGGGAGAGTCAGGGTATTAG CATCTGGTTAGCC | GCTGCATCCAGTTGTCAA AGT | CAACAGGCTAACAGTTTCCC ATTCACT |
| | 21-225_66F11 | | SEQ ID NO:1611 | SEQ ID NO:9623 | SEQ ID NO:17635 |
| | | AA | RESQGISIWLA | AASSLQS | QQANSFPFT |
| | | | SEQ ID NO:1612 | SEQ ID NO:9624 | SEQ ID NO:17636 |
| iPS:434393 | | NA | AGGGCCAGTCAGAGTGTTAA CAGCAACTTAGCC | ATTGCATCCACCAGGGC CACT | CAGCAGTATAATGACTGGCC GTGTAGT |
| | 21-225_67C3 | | SEQ ID NO:1613 | SEQ ID NO:9625 | SEQ ID NO:17637 |
| | | AA | RASQSVNSNLA | IASTRAT | QQYNDWPCS |
| | | | SEQ ID NO:1614 | SEQ ID NO:9626 | SEQ ID NO:17638 |

FIGURE 49

| iPS:434397 | | NA | CGGGCGAGTCAGGGTATTAG CAGCTGGTTAGCC | GCTGCATCCAGTTTGCAA AGT | CAACAGATTAACAGTTTCCC TCTCACT |
|---|---|---|---|---|---|
| | 21-225_67H4 | | SEQ ID NO:1615 | SEQ ID NO:9627 | SEQ ID NO:17639 |
| | | AA | RASQGISSWLA | AASSLQS | QQINSFPLT |
| | | | SEQ ID NO:1616 | SEQ ID NO:9628 | SEQ ID NO:17640 |
| iPS:434399 | | NA | CGGGCAAGTCAGGGCATTAG AAATGATTTAGGC | GCTGCATCCAGTTTGCAA AGT | CTACACCATAATAGTTATCC ATTCAAA |
| | 21-225_67B7 | | SEQ ID NO:1617 | SEQ ID NO:9629 | SEQ ID NO:17641 |
| | | AA | RASQGIRNDLG | AASSLQS | LHHNSYPFK |
| | | | SEQ ID NO:1618 | SEQ ID NO:9630 | SEQ ID NO:17642 |
| iPS:434405 | | NA | CGGGCGAGTCAGGGCATTAG CTATTATTTAGCC | GTTGCATCCAGTTTGCAA AGT | CAACAGTATGATAGTTACCC ATTCACT |
| | 21-225_68E6 | | SEQ ID NO:1619 | SEQ ID NO:9631 | SEQ ID NO:17643 |
| | | AA | RASQGISYYLA | VASSLQS | QQYDSYPFT |
| | | | SEQ ID NO:1620 | SEQ ID NO:9632 | SEQ ID NO:17644 |
| iPS:434407 | | NA | CGGGCAAGTCAGGGCATTAG AAATAATTTAGGC | GCTGCATCCAGTGTGCA AAGT | CTACAGTATAATAGTTACCC ATTCACT |
| | 21-225_68G8 | | SEQ ID NO:1621 | SEQ ID NO:9633 | SEQ ID NO:17645 |
| | | AA | RASQGIRNNLG | AASSVQS | LQYNSYPFT |
| | | | SEQ ID NO:1622 | SEQ ID NO:9634 | SEQ ID NO:17646 |
| iPS:434411 | | NA | CGGGCAAGTCAGGGCATTAG AAATGATTTAGGC | GCTGCATCCAGTTTGCAA AGT | CTACAGCATAATAGTTATCC GTTCACT |
| | 21-225_68F11 | | SEQ ID NO:1623 | SEQ ID NO:9635 | SEQ ID NO:17647 |
| | | AA | RASQGIRNDLG | AASSLQS | LQHNSYPFT |
| | | | SEQ ID NO:1624 | SEQ ID NO:9636 | SEQ ID NO:17648 |
| iPS:434413 | | NA | CGGGCAAGTCAGGGCATTAG AAATGATTTAGGC | GCTGCATCCAGTTTGCAA AGT | CTACAGCATAGTACTTACCC GCTCACT |
| | 21-225_68D12 | | SEQ ID NO:1625 | SEQ ID NO:9637 | SEQ ID NO:17649 |
| | | AA | RASQGIRNDLG | AASSLQS | LQHSTYPLT |
| | | | SEQ ID NO:1626 | SEQ ID NO:9638 | SEQ ID NO:17650 |

FIGURE 49

| iPS:434417 | | NA | CGGGCAGGTCAGACCATTTACAATTATTTAAAT | GTTGCGTCCAGTTTGCAAAGT | CAACAGAGTTACAGTACCCCATTCACT |
|---|---|---|---|---|---|
| | 21-225_69C8 | | SEQ ID NO:1627 | SEQ ID NO:9639 | SEQ ID NO:17651 |
| | | AA | RAGQTIYNYLN | VASSLQS | QQSYSTPFT |
| | | | SEQ ID NO:1628 | SEQ ID NO:9640 | SEQ ID NO:17652 |
| iPS:434423 | | NA | CGGGCGAGTCAGGGTGTTAGCAGGTGGTTAGCC | GCTGCATCCAGTTTGCAAAGT | CAACAGGCTAACAGTTTCCCATTCACT |
| | 21-225_70D1 | | SEQ ID NO:1629 | SEQ ID NO:9641 | SEQ ID NO:17653 |
| | | AA | RASQGVSRWLA | AASSLQS | QQANSFPFT |
| | | | SEQ ID NO:1630 | SEQ ID NO:9642 | SEQ ID NO:17654 |
| iPS:434425 | | NA | AGGGCCAGTCAGAGTGTTAACAGCAACTTAGCC | ATTGCATCCACCAGGGCCACT | CAGCAGTATAATGACTGGCCGTGTAGT |
| | 21-225_70A5 | | SEQ ID NO:1631 | SEQ ID NO:9643 | SEQ ID NO:17655 |
| | | AA | RASQSVNSNLA | IASTRAT | QQYNDWPCS |
| | | | SEQ ID NO:1632 | SEQ ID NO:9644 | SEQ ID NO:17656 |
| iPS:434427 | | NA | CGGGCGAGTCAGGGTATTAGCAAATGGTTAGCC | GCTGCATCCAGTTTGCAAAGT | CAACAGACTAACAGTTTCCCTCTCACT |
| | 21-225_70D6 | | SEQ ID NO:1633 | SEQ ID NO:9645 | SEQ ID NO:17657 |
| | | AA | RASQGISKWLA | AASSLQS | QQTNSFPLT |
| | | | SEQ ID NO:1634 | SEQ ID NO:9646 | SEQ ID NO:17658 |
| iPS:434429 | | NA | CGGACAAGTCAGAGCATTTTCAACTATTTAAAT | ACTGCATCCAGTTTGCAAAGT | CAACAGAGTTACAGTATCCCGCTCACT |
| | 21-225_70H6 | | SEQ ID NO:1635 | SEQ ID NO:9647 | SEQ ID NO:17659 |
| | | AA | RTSQSIFNYLN | TASSLQS | QQSYSIPLT |
| | | | SEQ ID NO:1636 | SEQ ID NO:9648 | SEQ ID NO:17660 |
| iPS:434431 | | NA | AAGTCCAGCCAGAGTGTTTTATACAGCTCCAACAATAACAACTACTTGGCT | TGGGCATCTACCCGGGAATCC | CAGCAATATTATAATATTCCTCCGACG |
| | 21-225_70E7 | | SEQ ID NO:1637 | SEQ ID NO:9649 | SEQ ID NO:17661 |
| | | AA | KSSQSVLYSSNNNNYLA | WASTRES | QQYYNIPPT |

FIGURE 49

| | | | SEQ ID NO:1638 | SEQ ID NO:9650 | SEQ ID NO:17662 |
|---|---|---|---|---|---|
| iPS:434433 | | NA | CGGGCAAGTCAGGGCATTAG AAAGGATTTAGGC | GCTGCATCCAGTTTGCAA AGT | CTACAGCATAATCGTTACCC GCTCACT |
| | 21-225_70E8 | | SEQ ID NO:1639 | SEQ ID NO:9651 | SEQ ID NO:17663 |
| | | AA | RASQGIRKDLG | AASSLQS | LQHNRYPLT |
| | | | SEQ ID NO:1640 | SEQ ID NO:9652 | SEQ ID NO:17664 |
| iPS:434435 | | NA | CGGGCGAGTCAGGATATTAG CAGCTGGTTAGCC | GCTGCATCCAGTTTGCAA AGT | CAACAGACTAACAGTTTCCC TCTCACT |
| | 21-225_70G9 | | SEQ ID NO:1641 | SEQ ID NO:9653 | SEQ ID NO:17665 |
| | | AA | RASQDISSWLA | AASSLQS | QQTNSFPLT |
| | | | SEQ ID NO:1642 | SEQ ID NO:9654 | SEQ ID NO:17666 |
| iPS:434437 | | NA | CGGGCGAGTCAGGGTATTAG CAGCTGGTTAGCC | GCTGCATCCAGTTTGCAA AGT | CAACAGATTAACAGTTTCCC TCTCACT |
| | 21-225_70A12 | | SEQ ID NO:1643 | SEQ ID NO:9655 | SEQ ID NO:17667 |
| | | AA | RASQGISSWLA | AASSLQS | QQINSFPLT |
| | | | SEQ ID NO:1644 | SEQ ID NO:9656 | SEQ ID NO:17668 |
| iPS:434439 | | NA | CGGGCAAGTCAGGGCATTAA CAATAATTTAAAC | GCTGCATCCAGTTTGCAA AGT | CTACAGCATAATAGTTACCC GCTCACT |
| | 21-225_70E12 | | SEQ ID NO:1645 | SEQ ID NO:9657 | SEQ ID NO:17669 |
| | | AA | RASQGINNNLN | AASSLQS | LQHNSYPLT |
| | | | SEQ ID NO:1646 | SEQ ID NO:9658 | SEQ ID NO:17670 |
| iPS:434441 | | NA | CGTGCAAGTCAGGGCATTAG AAATGATTTAGGA | ATTGCATTCAGATTGCAA ATT | ATACACCATAATAGTTACCC GTGGACG |
| | 21-225_71A2 | | SEQ ID NO:1647 | SEQ ID NO:9659 | SEQ ID NO:17671 |
| | | AA | RASQGIRNDLG | IAFRLQI | IHHNSYPWT |
| | | | SEQ ID NO:1648 | SEQ ID NO:9660 | SEQ ID NO:17672 |
| iPS:434443 | | NA | AAGTCCAGCCAGAGTGTTTT ACACAGCTCCAACAATAACA ACTACTTAGAT | TGGGCATCTACCCGGGA ATTC | CAACAATATTATATTACTCC GTGCAGT |
| | 21-225_71G3 | | SEQ ID NO:1649 | SEQ ID NO:9661 | SEQ ID NO:17673 |

715

FIGURE 49

| | | | | | |
|---|---|---|---|---|---|
| | | AA | KSSQSVLHSSNNNNYLD | WASTREF | QQYYITPCS |
| | | | SEQ ID NO:1650 | SEQ ID NO:9662 | SEQ ID NO:17674 |
| iPS:434447 | | NA | CGGGCAAGTCAGGGCATTAGAAATGATTTAGAT | GCTGCATCCAGTTTGCAAAGT | CTACAGCATAATAGTTACCCATTCACT |
| | 21-225_71B6 | | SEQ ID NO:1651 | SEQ ID NO:9663 | SEQ ID NO:17675 |
| | | AA | RASQGIRNDLD | AASSLQS | LQHNSYPFT |
| | | | SEQ ID NO:1652 | SEQ ID NO:9664 | SEQ ID NO:17676 |
| iPS:434449 | | NA | CGGGCAAGTCAGGGCATTAGAAATGTTTTAGGC | GCTGCATCCAGTTTACAAAGT | CTACAGTATAATAGTTACCCATTCACT |
| | 21-225_71H6 | | SEQ ID NO:1653 | SEQ ID NO:9665 | SEQ ID NO:17677 |
| | | AA | RASQGIRNVLG | AASSLQS | LQYNSYPFT |
| | | | SEQ ID NO:1654 | SEQ ID NO:9666 | SEQ ID NO:17678 |
| iPS:434451 | | NA | CGGGCGAGTCAGGGTATTAGCAGCTGGTTAGCC | GCTGCATCCAGTTTGCAAGGT | CAACAGACTAATAGTTTCCCTCTCACT |
| | 21-225_71B7 | | SEQ ID NO:1655 | SEQ ID NO:9667 | SEQ ID NO:17679 |
| | | AA | RASQGISSWLA | AASSLQG | QQTNSFPLT |
| | | | SEQ ID NO:1656 | SEQ ID NO:9668 | SEQ ID NO:17680 |
| iPS:434453 | | NA | CGGGCAAGTCAGGGCATTAGAAATGATTTAGAT | GCTGCATCCAGTTTGCAAAGT | CTACAGCATAATACTTACCCATTCACT |
| | 21-225_71B11 | | SEQ ID NO:1657 | SEQ ID NO:9669 | SEQ ID NO:17681 |
| | | AA | RASQGIRNDLD | AASSLQS | LQHNTYPFT |
| | | | SEQ ID NO:1658 | SEQ ID NO:9670 | SEQ ID NO:17682 |
| iPS:434455 | | NA | CGGGCAAGTCAGAACATTAGCAGCTATTTAAAT | GCTGCATCCAGTTTGCAAAGT | CAACAGACTTACAGTACCCCCACC |
| | 21-225_72F5 | | SEQ ID NO:1659 | SEQ ID NO:9671 | SEQ ID NO:17683 |
| | | AA | RASQNISSYLN | AASSLQS | QQTYSTPT |
| | | | SEQ ID NO:1660 | SEQ ID NO:9672 | SEQ ID NO:17684 |
| iPS:434457 | | NA | CGGGCGAGTCAGGGCATTAGCAGTTATTTAAAT | GGTGCTTCCAATTTGCAATCT | CAACAGAATTACAATGCCCCGCTCACT |
| | 21-225_72G12 | | SEQ ID NO:1661 | SEQ ID NO:9673 | SEQ ID NO:17685 |

716

FIGURE 49

| | | AA | RASQGISSYLN | GASNLQS | QQNYNAPLT |
|---|---|---|---|---|---|
| | | | SEQ ID NO:1662 | SEQ ID NO:9674 | SEQ ID NO:17686 |
| iPS:434459 | | NA | CGGGCGAGTCAGGGTATTAGCAGCTGGTTAGCC | GCTGCATCCAGTTTGCAAAGT | CAACAGGTTAACAGTTTCCCTCTCACT |
| | 21-225_71A7 | | SEQ ID NO:1663 | SEQ ID NO:9675 | SEQ ID NO:17687 |
| | | AA | RASQGISSWLA | AASSLQS | QQVNSFPLT |
| | | | SEQ ID NO:1664 | SEQ ID NO:9676 | SEQ ID NO:17688 |
| iPS:434461 | | NA | CGGGCGAGTCAGGGTATTAGCAACTGGTTAGCC | GCTGCATCCAGTTTGCAAAGT | CAACAGGTTAACAGTTTCCCTCTCACT |
| | 21-225_73A3 | | SEQ ID NO:1665 | SEQ ID NO:9677 | SEQ ID NO:17689 |
| | | AA | RASQGISNWLA | AASSLQS | QQVNSFPLT |
| | | | SEQ ID NO:1666 | SEQ ID NO:9678 | SEQ ID NO:17690 |
| iPS:434463 | | NA | CGGGCAAGTCAGGGCATTAGAAATGATTTAGGC | GCTGCATCCAATTTGCAAAGT | CTACAGCATAATAGTTACCCATTCACT |
| | 21-225_73A6 | | SEQ ID NO:1667 | SEQ ID NO:9679 | SEQ ID NO:17691 |
| | | AA | RASQGIRNDLG | AASNLQS | LQHNSYPFT |
| | | | SEQ ID NO:1668 | SEQ ID NO:9680 | SEQ ID NO:17692 |
| iPS:434467 | | NA | CGGGCAAGTCAGGACATCAGAAATGATTTAGGC | GCTGCATCCAGTTTGCAAAGT | ATACAGCATAATAGTTACCCTCCGATCACC |
| | 21-225_73H8 | | SEQ ID NO:1669 | SEQ ID NO:9681 | SEQ ID NO:17693 |
| | | AA | RASQDIRNDLG | AASSLQS | IQHNSYPPIT |
| | | | SEQ ID NO:1670 | SEQ ID NO:9682 | SEQ ID NO:17694 |
| iPS:434469 | | NA | CGGGCAAGTCAGGGCATTAGAAATGATTTAGGC | GCTGCATCCAGTTTGCAAAGT | CTACAGCATTATAGTTACCCTCGGACG |
| | 21-225_73C9 | | SEQ ID NO:1671 | SEQ ID NO:9683 | SEQ ID NO:17695 |
| | | AA | RASQGIRNDLG | AASSLQS | LQHYSYPRT |
| | | | SEQ ID NO:1672 | SEQ ID NO:9684 | SEQ ID NO:17696 |
| iPS:434471 | | NA | AGGGCCCGTCAGAATGTTGACAGCAGCTACTTAGCC | GGTGCATCCAGCAGGGCCACT | CAGCAGTATGAACGCTCACCGTGGACG |
| | 21-225_75G3 | | SEQ ID NO:1673 | SEQ ID NO:9685 | SEQ ID NO:17697 |

FIGURE 49

| | | | | | |
|---|---|---|---|---|---|
| | | AA | RARQNVDSSYLA | GASSRAT | QQYERSPWT |
| | | | SEQ ID NO:1674 | SEQ ID NO:9686 | SEQ ID NO:17698 |
| iPS:434473 | 21-225_76D1 | NA | AGGGCCAGTCAGAATATTTACAGCAACTACCTAGCC | GGTGCATCCAGCAGGGCCACT | CAGCAGTATGAAAGTTCACCGTGGACG |
| | | | SEQ ID NO:1675 | SEQ ID NO:9687 | SEQ ID NO:17699 |
| | | AA | RASQNIYSNYLA | GASSRAT | QQYESSPWT |
| | | | SEQ ID NO:1676 | SEQ ID NO:9688 | SEQ ID NO:17700 |
| iPS:434475 | 21-225_74F9 | NA | AAGTCCAGCCAGAGTGTTTTATACAGCTCCAACAATTACAACTACTTAGCT | TGGGCATCTACCCGGGAATCC | CAGCAATATTATAGTAGTCCTCCGACG |
| | | | SEQ ID NO:1677 | SEQ ID NO:9689 | SEQ ID NO:17701 |
| | | AA | KSSQSVLYSSNNYNYLA | WASTRES | QQYYSSPPT |
| | | | SEQ ID NO:1678 | SEQ ID NO:9690 | SEQ ID NO:17702 |
| iPS:434477 | 21-225_74A6 | NA | AAGTCCAGCCAGAGTGTTTTACACAGCTCCAACAATAACAATTACTTAGCC | TGGGCATCAACCCGGGAATCC | CAGCAATATTTTAGTACTCCGTGGACG |
| | | | SEQ ID NO:1679 | SEQ ID NO:9691 | SEQ ID NO:17703 |
| | | AA | KSSQSVLHSSNNNNYLA | WASTRES | QQYFSTPWT |
| | | | SEQ ID NO:1680 | SEQ ID NO:9692 | SEQ ID NO:17704 |
| iPS:434479 | 21-225_76H1 | NA | AGGGCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:1681 | SEQ ID NO:9693 | SEQ ID NO:17705 |
| | | AA | RASQSVSSSYLV | GASTRAT | QQYGCSPLT |
| | | | SEQ ID NO:1682 | SEQ ID NO:9694 | SEQ ID NO:17706 |
| iPS:434481 | 21-225_74B10 | NA | AAGTCCAGCCAGAGTGTTTTACACAGCTCCAACAATAAGAACTACTTAACT | TGGGCATCTACTCGGGAATCC | CAGCAATATTATAGTATTCCTCCGACG |
| | | | SEQ ID NO:1683 | SEQ ID NO:9695 | SEQ ID NO:17707 |
| | | AA | KSSQSVLHSSNNKNYLT | WASTRES | QQYYSIPPT |
| | | | SEQ ID NO:1684 | SEQ ID NO:9696 | SEQ ID NO:17708 |

FIGURE 49

| iPS:434483 | | NA | AAGTCCAGCCAGAGTGTTTT ATACAGCTCCAACAATGCGA ACTACTTAGCT | TGGGCATCTACCCGGGA ATCC | CAGCAATATTATAGTACTCC GTGCAGT |
| --- | --- | --- | --- | --- | --- |
| | 21-225_74C12 | | SEQ ID NO:1685 | SEQ ID NO:9697 | SEQ ID NO:17709 |
| | | AA | KSSQSVLYSSNNANYLA | WASTRES | QQYYSTPCS |
| | | | SEQ ID NO:1686 | SEQ ID NO:9698 | SEQ ID NO:17710 |
| iPS:434485 | | NA | AGGGCCAGTGTGAGTGTTGT CAACAGCTTAGCC | GGTGCATCCACCAGGGC CACT | CAGCAATATAATGACTGGCC GTGCAGT |
| | 21-225_76D2 | | SEQ ID NO:1687 | SEQ ID NO:9699 | SEQ ID NO:17711 |
| | | AA | RASVSVVNSLA | GASTRAT | QQYNDWPCS |
| | | | SEQ ID NO:1688 | SEQ ID NO:9700 | SEQ ID NO:17712 |
| iPS:434487 | | NA | AAGTCCAGCCAGAGTGTTTT ACACAGCTCCAACAATTACA ACTACTTAGCT | TGGGCATCTACCCGGGA ATCC | CAGCAATATTATAGTTCTCC TCCGACG |
| | 21-225_76G2 | | SEQ ID NO:1689 | SEQ ID NO:9701 | SEQ ID NO:17713 |
| | | AA | KSSQSVLHSSNNYNYLA | WASTRES | QQYYSSPPT |
| | | | SEQ ID NO:1690 | SEQ ID NO:9702 | SEQ ID NO:17714 |
| iPS:434489 | | NA | CGGGCAAGTCAGGGCATTAG AAATGATTTAGGC | GCTGCATCCACTTTGCAA AGT | CTACAGCATAGTAATTACCC GCTCACT |
| | 21-225_74E4 | | SEQ ID NO:1691 | SEQ ID NO:9703 | SEQ ID NO:17715 |
| | | AA | RASQGIRNDLG | AASTLQS | LQHSNYPLT |
| | | | SEQ ID NO:1692 | SEQ ID NO:9704 | SEQ ID NO:17716 |
| iPS:434493 | | NA | AAGTCCAGCCAGAGTGTTTT ATTCAGCTCCAACAATTATA ATTACTTAGCT | TGGGCATCTACCCGGGA ATCC | CAGCAATATCATAGTTCTCC TCTGACG |
| | 21-225_76F3 | | SEQ ID NO:1693 | SEQ ID NO:9705 | SEQ ID NO:17717 |
| | | AA | KSSQSVLFSSNNYNYLA | WASTRES | QQYHSSPLT |
| | | | SEQ ID NO:1694 | SEQ ID NO:9706 | SEQ ID NO:17718 |
| iPS:434495 | | NA | AGGGCCAGTCAGAATATTTA CAGCAGTTACTTAGCC | GGTGCATCCAGCAGGGC CACT | CAGCAGTATGAAAGCTCACC GTGGACC |

FIGURE 49

| | 21-225_74B2 | | SEQ ID NO:1695 | SEQ ID NO:9707 | SEQ ID NO:17719 |
|---|---|---|---|---|---|
| | | AA | RASQNIYSSYLA | GASSRAT | QQYESSPWT |
| | | | SEQ ID NO:1696 | SEQ ID NO:9708 | SEQ ID NO:17720 |
| iPS:434497 | 21-225_76A4 | NA | AGGGCCAGTCAGAGTGTTTACAGCAGCTACTTAGCC | GGTGCATCCAGCCGGGCCACT | CAGCACTCTGATAACTCACCGTGGACG |
| | | | SEQ ID NO:1697 | SEQ ID NO:9709 | SEQ ID NO:17721 |
| | | AA | RASQSVYSSYLA | GASSRAT | QHSDNSPWT |
| | | | SEQ ID NO:1698 | SEQ ID NO:9710 | SEQ ID NO:17722 |
| iPS:434501 | 21-225_76G4 | NA | AGGGCCAGTCAGAGTGTTTACAGCAGCTACTTAGCC | GGTGCATCCAGCCGGGCCACT | CAGCACTCTGATAACTCACCGTGGACG |
| | | | SEQ ID NO:1699 | SEQ ID NO:9711 | SEQ ID NO:17723 |
| | | AA | RASQSVYSSYLA | GASSRAT | QHSDNSPWT |
| | | | SEQ ID NO:1700 | SEQ ID NO:9712 | SEQ ID NO:17724 |
| iPS:434503 | 21-225_74D7 | NA | CGGGCAAGTCAGGGCATTAGAAATGATTTAGGC | GCTGCATCCAGTTTGCAAAGT | CTTCAGCATAGTAATTACCCGCTCACT |
| | | | SEQ ID NO:1701 | SEQ ID NO:9713 | SEQ ID NO:17725 |
| | | AA | RASQGIRNDLG | AASSLQS | LQHSNYPLT |
| | | | SEQ ID NO:1702 | SEQ ID NO:9714 | SEQ ID NO:17726 |
| iPS:434507 | 21-225_74C5 | NA | AGGGCCAGTCAGAGTGTTAACAGCAACTACTTAGCC | GGTGCATTCAGCAGGGCCACT | CAGCAGTATGAAAGCTCACCGTGGACG |
| | | | SEQ ID NO:1703 | SEQ ID NO:9715 | SEQ ID NO:17727 |
| | | AA | RASQSVNSNYLA | GAFSRAT | QQYESSPWT |
| | | | SEQ ID NO:1704 | SEQ ID NO:9716 | SEQ ID NO:17728 |
| iPS:434509 | 21-225_76F5 | NA | AAGTCCAGCCAGAGTGTATTACACAGCTCCAACAGTTACAACTACTTAGCT | TGGACATCTACCCGGGAATCC | CAGCAATATTATAGTAGTCCTCCGACG |
| | | | SEQ ID NO:1705 | SEQ ID NO:9717 | SEQ ID NO:17729 |
| | | AA | KSSQSVLHSSNSYNYLA | WTSTRES | QQYYSSPPT |
| | | | SEQ ID NO:1706 | SEQ ID NO:9718 | SEQ ID NO:17730 |

FIGURE 49

| iPS:434511 | 21-225_74B11 | NA | AAGTCCAGCCAGAGTATTTT ATACAACTCCAACAATAACA ACTACTTAGCT | TGGGCATCTACCCGGGA ATCC | CAGCAATATTATAGCACTCC TCCTACT |
|---|---|---|---|---|---|
| | | | SEQ ID NO:1707 | SEQ ID NO:9719 | SEQ ID NO:17731 |
| | | AA | KSSQSILYNSNNNNYLA | WASTRES | QQYYSTPPT |
| | | | SEQ ID NO:1708 | SEQ ID NO:9720 | SEQ ID NO:17732 |
| iPS:434513 | 21-225_76A6 | NA | AGGGCCAGTCAGAGTGTTAG CAGCAGCTACTTAGCC | GGTGCATCCACCAGGGC CACT | CAGCAGTATGGTAACTCACC GCTCACT |
| | | | SEQ ID NO:1709 | SEQ ID NO:9721 | SEQ ID NO:17733 |
| | | AA | RASQSVSSSYLA | GASTRAT | QQYGNSPLT |
| | | | SEQ ID NO:1710 | SEQ ID NO:9722 | SEQ ID NO:17734 |
| iPS:434515 | 21-225_74A5 | NA | AGGGCCAGTCAGAGTGTTAG CAGCAGCTACTTAGTC | GGTGCATCCACCAGGGC CACT | CAGCAGTATGGTTGCTCACC GCTCACT |
| | | | SEQ ID NO:1711 | SEQ ID NO:9723 | SEQ ID NO:17735 |
| | | AA | RASQSVSSSYLV | GASTRAT | QQYGCSPLT |
| | | | SEQ ID NO:1712 | SEQ ID NO:9724 | SEQ ID NO:17736 |
| iPS:434517 | 21-225_76A7 | NA | AGGGCCAGTCCGAGTGTTGA CAGCAGCTACTTAGCC | GGTGCATCCAGCAGGGC CCCT | CAGCAGTATGAAAGTTCACC GTGGACG |
| | | | SEQ ID NO:1713 | SEQ ID NO:9725 | SEQ ID NO:17737 |
| | | AA | RASPSVDSSYLA | GASSRAP | QQYESSPWT |
| | | | SEQ ID NO:1714 | SEQ ID NO:9726 | SEQ ID NO:17738 |
| iPS:434519 | 21-225_74C7 | NA | AGGACCAGTCCGAATGTTGA CAGCAGCTACTTAGCC | GGTGCATCCAGCAGGGC CACT | CAGCAGTATGAACGCTCACC GTGGACG |
| | | | SEQ ID NO:1715 | SEQ ID NO:9727 | SEQ ID NO:17739 |
| | | AA | RTSPNVDSSYLA | GASSRAT | QQYERSPWT |
| | | | SEQ ID NO:1716 | SEQ ID NO:9728 | SEQ ID NO:17740 |
| iPS:434523 | 21-225_75C3 | NA | AGGGCCAGTCAGAGTGTTAG CAGCAGGTACTTAGCC | GGTGCATCCAGCAGGGC CACT | CAGCATTATGATAGCTCACC GTGGACG |
| | | | SEQ ID NO:1717 | SEQ ID NO:9729 | SEQ ID NO:17741 |
| | | AA | RASQSVSSRYLA | GASSRAT | QHYDSSPWT |

FIGURE 49

| | | | SEQ ID NO:1718 | SEQ ID NO:9730 | SEQ ID NO:17742 |
|---|---|---|---|---|---|
| iPS:434525 | 21-225_76E8 | NA | AAGTCCAGCCAGACTGTTTT ACACAGCTCCAACAATTATA ACTACTTAGCT | TGGACATCTACCCGGGA ATCC | CAGCAATATTTTAGTAGTCC TCTGACG |
| | | | SEQ ID NO:1719 | SEQ ID NO:9731 | SEQ ID NO:17743 |
| | | AA | KSSQTVLHSSNNYNYLA | WTSTRES | QQYFSSPLT |
| | | | SEQ ID NO:1720 | SEQ ID NO:9732 | SEQ ID NO:17744 |
| iPS:434529 | 21-225_76B9 | NA | AGGGCCAGTCAGAGTGTTAG CAGCAGCTACTTAGTC | GGTGCATCCACCAGGGC CACT | CAGCAGTATGGTTGCTCACC GCTCACT |
| | | | SEQ ID NO:1721 | SEQ ID NO:9733 | SEQ ID NO:17745 |
| | | AA | RASQSVSSSYLV | GASTRAT | QQYGCSPLT |
| | | | SEQ ID NO:1722 | SEQ ID NO:9734 | SEQ ID NO:17746 |
| iPS:434531 | 21-225_76C9 | NA | AGGGCCAGTCAGAGTGTTAG CAGCAGTTACTTATCC | GGTGCATCCAGCAGGGC CACT | CAGCAGTATGGTAGGTCACG GACG |
| | | | SEQ ID NO:1723 | SEQ ID NO:9735 | SEQ ID NO:17747 |
| | | AA | RASQSVSSSYLS | GASSRAT | QQYGRSRT |
| | | | SEQ ID NO:1724 | SEQ ID NO:9736 | SEQ ID NO:17748 |
| iPS:434533 | 21-225_85F7 | NA | AGGGCCAGTCAGAATATTTA CAGCAACTACTTAGCC | GGTGCATCCAGCAGGGC CACT | CAGCAGTATGAAAGCTCACC GTGGACC |
| | | | SEQ ID NO:1725 | SEQ ID NO:9737 | SEQ ID NO:17749 |
| | | AA | RASQNIYSNYLA | GASSRAT | QQYESSPWT |
| | | | SEQ ID NO:1726 | SEQ ID NO:9738 | SEQ ID NO:17750 |
| iPS:434535 | 21-225_74C8 | NA | CGGGCGAGTCAGGGCATTGG CAAGTATTTAGCC | ACTACATCCAATTTACAA AGT | CAACAGTACAGTAATTACCC GCTCACT |
| | | | SEQ ID NO:1727 | SEQ ID NO:9739 | SEQ ID NO:17751 |
| | | AA | RASQGIGKYLA | TTSNLQS | QQYSNYPLT |
| | | | SEQ ID NO:1728 | SEQ ID NO:9740 | SEQ ID NO:17752 |
| iPS:434537 | 21-225_74E11 | NA | AGGGCCAGTCTGAGTGTTGT CAACAGCTTAGCC | GGTGCATCCACCAGGGC CACT | CAGCAGTATAATGACTGGCC GTGCAGT |
| | | | SEQ ID NO:1729 | SEQ ID NO:9741 | SEQ ID NO:17753 |

722

FIGURE 49

| | | AA | RASLSVVNSLA | GASTRAT | QQYNDWPCS |
|---|---|---|---|---|---|
| | | | SEQ ID NO:1730 | SEQ ID NO:9742 | SEQ ID NO:17754 |
| iPS:434539 | 21-225_74A2 | NA | AGGTCTAGTCAGAGCCTCCT GCATAGTAATGGACACAACT ATTTGGAT | TTGGGTTCTAATCGGGCC TCC | ATGCAACCTCTACAAACTCC GTTCACT |
| | | | SEQ ID NO:1731 | SEQ ID NO:9743 | SEQ ID NO:17755 |
| | | AA | RSSQSLLHSNGHNYLD | LGSNRAS | MQPLQTPFT |
| | | | SEQ ID NO:1732 | SEQ ID NO:9744 | SEQ ID NO:17756 |
| iPS:434547 | 21-225_74H5 | NA | AGGGCCAGTCAGAGTGTTAA CAGCAACTACTTAGCC | GGTGCATCCAGCAGGGC CACT | CAGCAGTATGAAAGCTCGCC GTGGACG |
| | | | SEQ ID NO:1733 | SEQ ID NO:9745 | SEQ ID NO:17757 |
| | | AA | RASQSVNSNYLA | GASSRAT | QQYESSPWT |
| | | | SEQ ID NO:1734 | SEQ ID NO:9746 | SEQ ID NO:17758 |
| iPS:434549 | 21-225_76E11 | NA | AAGTCCCGCCAGAGTGTTTT ACACAGCTCCAACAATTACA ACTACTTAGCT | TGGGCTTCTACCCGGGA ATCC | CAGCAATATTATAGTACTCC TCCGACG |
| | | | SEQ ID NO:1735 | SEQ ID NO:9747 | SEQ ID NO:17759 |
| | | AA | KSRQSVLHSSNNYNYLA | WASTRES | QQYYSTPPT |
| | | | SEQ ID NO:1736 | SEQ ID NO:9748 | SEQ ID NO:17760 |
| iPS:434551 | 21-225_75C4 | NA | AAGTCCAGCCAGAGTATTTT ATACAGCTCCAACAATAATA ACTACTTAGCT | TGGGCATCTACCCGGGA ATCC | CAGCAATATTATATTACTCC TCCGACG |
| | | | SEQ ID NO:1737 | SEQ ID NO:9749 | SEQ ID NO:17761 |
| | | AA | KSSQSILYSSNNNNYLA | WASTRES | QQYYITPPT |
| | | | SEQ ID NO:1738 | SEQ ID NO:9750 | SEQ ID NO:17762 |
| iPS:434559 | 21-225_74D11 | NA | AGGGCCAGTCAGAGTGTTTA CAGCAGCTACTTAGCC | GGTGCATCCAGCCGGGC CACT | CAGCACTATGATAACTCACC GTGGACG |
| | | | SEQ ID NO:1739 | SEQ ID NO:9751 | SEQ ID NO:17763 |
| | | AA | RASQSVYSSYLA | GASSRAT | QHYDNSPWT |
| | | | SEQ ID NO:1740 | SEQ ID NO:9752 | SEQ ID NO:17764 |

FIGURE 49

| iPS:434561 | | NA | AGGGCCAGTCAGAGTGTTTACAGCAGCTACTTAGCC | GGTGCATCCAGCCGGGCCACT | CAGCACTATGATAACTCACCGTGGACG |
| | 21-225_77G1 | | SEQ ID NO:1741 | SEQ ID NO:9753 | SEQ ID NO:17765 |
| | | AA | RASQSVYSSYLA | GASSRAT | QHYDNSPWT |
| | | | SEQ ID NO:1742 | SEQ ID NO:9754 | SEQ ID NO:17766 |
| iPS:434563 | | NA | AGGTCTAGTCAGAGCCTCCTGCATAGTAGTGGATACAACTATTTGGAT | TTGGGTTCTAATCGGGCCTCC | ATGCAAGCTCTACACCCTCCTCTCACT |
| | 21-225_75D8 | | SEQ ID NO:1743 | SEQ ID NO:9755 | SEQ ID NO:17767 |
| | | AA | RSSQSLLHSSGYNYLD | LGSNRAS | MQALHPPLT |
| | | | SEQ ID NO:1744 | SEQ ID NO:9756 | SEQ ID NO:17768 |
| iPS:434565 | | NA | AGGGCCAGTCCGAGTGTTAACAGCTACTACTTAGCC | GGTGCAACCAGCAGGGCCACT | CAGCAGTATGAAGACTCACCGTGGACG |
| | 21-225_75B10 | | SEQ ID NO:1745 | SEQ ID NO:9757 | SEQ ID NO:17769 |
| | | AA | RASPSVNSYYLA | GATSRAT | QQYEDSPWT |
| | | | SEQ ID NO:1746 | SEQ ID NO:9758 | SEQ ID NO:17770 |
| iPS:434569 | | NA | AGGGCCAGTCAGAGTGTTAGCAGCAGCTTAGCC | GGTGCATCCACCAGGGCCACT | CAGCAGTATAATGACTGGCCGTGCAGT |
| | 21-225_77H5 | | SEQ ID NO:1747 | SEQ ID NO:9759 | SEQ ID NO:17771 |
| | | AA | RASQSVSSSLA | GASTRAT | QQYNDWPCS |
| | | | SEQ ID NO:1748 | SEQ ID NO:9760 | SEQ ID NO:17772 |
| iPS:434571 | | NA | AGGGCCAGTCAGAGTGTTGACAGCAACTACTTAGCC | GGTGCATCCAGCAGGGCCCCT | CAGCAGTATGAAAGCTCACCGTGGACG |
| | 21-225_74D2 | | SEQ ID NO:1749 | SEQ ID NO:9761 | SEQ ID NO:17773 |
| | | AA | RASQSVDSNYLA | GASSRAP | QQYESSPWT |
| | | | SEQ ID NO:1750 | SEQ ID NO:9762 | SEQ ID NO:17774 |
| iPS:434573 | | NA | CGGGCGAGTCAGGGCATTAGCAAGTATTTAGCC | GCTGCATCCAGTTTGCAAGGT | CAACAGTACAGTAATTACCCGCTCACT |
| | 21-225_77E6 | | SEQ ID NO:1751 | SEQ ID NO:9763 | SEQ ID NO:17775 |
| | | AA | RASQGISKYLA | AASSLQG | QQYSNYPLT |

724

FIGURE 49

| | | | SEQ ID NO:1752 | SEQ ID NO:9764 | SEQ ID NO:17776 |
|---|---|---|---|---|---|
| iPS:434575 | 21-225_77C7 | NA | AAGTCCAGCCAGACTGTTTTACACAGCTCCAACAATTATAACTACTTAGCT | TGGACATCTACCCGGGAATCC | CAGCAATATTTTAGTAGTCCTCCGACG |
| | | | SEQ ID NO:1753 | SEQ ID NO:9765 | SEQ ID NO:17777 |
| | | AA | KSSQTVLHSSNNYNYLA | WTSTRES | QQYFSSPPT |
| | | | SEQ ID NO:1754 | SEQ ID NO:9766 | SEQ ID NO:17778 |
| iPS:434579 | 21-225_77F7 | NA | AGGGCCAGTCAGAGTGTTTACAGCAGCTACTTAGCG | GGTGCATCCAGCCGGGCCACT | CAGCACTATGATAACTCACCGTGGACG |
| | | | SEQ ID NO:1755 | SEQ ID NO:9767 | SEQ ID NO:17779 |
| | | AA | RASQSVYSSYLA | GASSRAT | QHYDNSPWT |
| | | | SEQ ID NO:1756 | SEQ ID NO:9768 | SEQ ID NO:17780 |
| iPS:434581 | 21-225_74B12 | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:1757 | SEQ ID NO:9769 | SEQ ID NO:17781 |
| | | AA | RASQSVYSSYLA | GASSRST | QHYDNSPWT |
| | | | SEQ ID NO:1758 | SEQ ID NO:9770 | SEQ ID NO:17782 |
| iPS:434583 | 21-225_74B6 | NA | AGGGCCAGTCAGAGTGTTAGCAGCAGCTACTTAGCC | GGTGCATCCACCAGGGCCACT | CAGCAGTATGGTAACTCACCGCTCACT |
| | | | SEQ ID NO:1759 | SEQ ID NO:9771 | SEQ ID NO:17783 |
| | | AA | RASQSVSSSYLA | GASTRAT | QQYGNSPLT |
| | | | SEQ ID NO:1760 | SEQ ID NO:9772 | SEQ ID NO:17784 |
| iPS:434585 | 21-225_75A12 | NA | AGGGCCAGTCAGAGTGTTAGCAGCAGGTACTTAGCC | GGTGCATCCAGCAGGGCCACT | CAGCATTATGATAGCTCACCGTGGACG |
| | | | SEQ ID NO:1761 | SEQ ID NO:9773 | SEQ ID NO:17785 |
| | | AA | RASQSVSSRYLA | GASSRAT | QHYDSSPWT |
| | | | SEQ ID NO:1762 | SEQ ID NO:9774 | SEQ ID NO:17786 |
| iPS:434587 | 21-225_74G3 | NA | AGGGCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:1763 | SEQ ID NO:9775 | SEQ ID NO:17787 |

FIGURE 49

| | | AA | RASQSVSSSYLV | GASTRAT | QQYGCSPLT |
|---|---|---|---|---|---|
| | | | SEQ ID NO:1764 | SEQ ID NO:9776 | SEQ ID NO:17788 |
| iPS:434595 | | NA | AGGGCCAGTCAGAGTGTTCA CAGCAGGTACTTAGCC | GGTGCATCCAGCAGGGC CACT | CAGCATTATGATAGCTCACC GTGGACG |
| | 21-225_77A10 | | SEQ ID NO:1765 | SEQ ID NO:9777 | SEQ ID NO:17789 |
| | | AA | RASQSVHSRYLA | GASSRAT | QHYDSSPWT |
| | | | SEQ ID NO:1766 | SEQ ID NO:9778 | SEQ ID NO:17790 |
| iPS:434597 | | NA | AAGTCCAGCCAGAGTGTTTT ATACACCTCCAACAATAACA ACTACTTAGCT | TGGGCATCTACCCGGGA ATCC | CAGCACTATTATAATACTCC GTGGAAG |
| | 21-225_77C10 | | SEQ ID NO:1767 | SEQ ID NO:9779 | SEQ ID NO:17791 |
| | | AA | KSSQSVLYTSNNNNYLA | WASTRES | QHYYNTPWK |
| | | | SEQ ID NO:1768 | SEQ ID NO:9780 | SEQ ID NO:17792 |
| iPS:434603 | | NA | AGGGCCAGTCAGAGTGTTAG CAGCAGCTACTTAGTC | GGTGCATCCACCAGGGC CACT | CAGCAGTATGGTTGCTACC GCTCACT |
| | 21-225_77D11 | | SEQ ID NO:1769 | SEQ ID NO:9781 | SEQ ID NO:17793 |
| | | AA | RASQSVSSSYLV | GASTRAT | QQYGCSPLT |
| | | | SEQ ID NO:1770 | SEQ ID NO:9782 | SEQ ID NO:17794 |
| iPS:434611 | | NA | AGGGCCAGGCAGAGTGTTG ACAGCAGTTATTTAGCC | GGTGCATCCAGCAGGGC CACT | CAGCAGTATGAAAGCTCACC GTGGACG |
| | 21-225_77C12 | | SEQ ID NO:1771 | SEQ ID NO:9783 | SEQ ID NO:17795 |
| | | AA | RARQSVDSSYLA | GASSRAT | QQYESSPWT |
| | | | SEQ ID NO:1772 | SEQ ID NO:9784 | SEQ ID NO:17796 |
| iPS:434613 | | NA | AGGTCCAGCCAGAGTGTTTT ATACAGCTCCAACAATTATA ACTACTTAGCT | TGGGCATCTACCCGGGA TTCC | CAGCAATATTATACTACTCC GTGCAGT |
| | 21-225_77D12 | | SEQ ID NO:1773 | SEQ ID NO:9785 | SEQ ID NO:17797 |
| | | AA | RSSQSVLYSSNNYNYLA | WASTRDS | QQYYTTPCS |
| | | | SEQ ID NO:1774 | SEQ ID NO:9786 | SEQ ID NO:17798 |

FIGURE 49

| iPS:434615 | | NA | CGGGCGAGTCAGGTCATTAG CAAGTATTTAGCC | GCTGCATCCAGTTTGCAA AGT | CAACAGTACAGTAATTACCC GCTCACT |
|---|---|---|---|---|---|
| | 21-225_76C5 | | SEQ ID NO:1775 | SEQ ID NO:9787 | SEQ ID NO:17799 |
| | | AA | RASQVISKYLA | AASSLQS | QQYSNYPLT |
| | | | SEQ ID NO:1776 | SEQ ID NO:9788 | SEQ ID NO:17800 |
| iPS:434617 | | NA | AAGTCCAGCCAGAGTGTTTT ACACAGCTCTAATAAAAAGA ACTACTTAGCT | TGGGCATCTACCCGGGA ATCC | CAGCAATATTATAGGACTCC GTGGACG |
| | 21-225_74B8 | | SEQ ID NO:1777 | SEQ ID NO:9789 | SEQ ID NO:17801 |
| | | AA | KSSQSVLHSSNKKNYLA | WASTRES | QQYYRTPWT |
| | | | SEQ ID NO:1778 | SEQ ID NO:9790 | SEQ ID NO:17802 |
| iPS:434619 | | NA | AAGTCCAGCCAGAGTGTTTT ATACACCTCCAACAATAACA ACTACTTAGCT | TGGGCATCTACCCGGGA ATCC | CAGCACTATTATAATACTCC GTGGAAG |
| | 21-225_78C1 | | SEQ ID NO:1779 | SEQ ID NO:9791 | SEQ ID NO:17803 |
| | | AA | KSSQSVLYTSNNNNYLA | WASTRES | QHYYNTPWK |
| | | | SEQ ID NO:1780 | SEQ ID NO:9792 | SEQ ID NO:17804 |
| iPS:434621 | | NA | AGGGCCAGTCAGAGTGTTAG CAGAAATTTAGCC | GGTGCATCCATCAGGGC CACT | CAGCAGTATAATAACTGGCC TCCGCTCACT |
| | 21-225_74D1 | | SEQ ID NO:1781 | SEQ ID NO:9793 | SEQ ID NO:17805 |
| | | AA | RASQSVSRNLA | GASIRAT | QQYNNWPPLT |
| | | | SEQ ID NO:1782 | SEQ ID NO:9794 | SEQ ID NO:17806 |
| iPS:434629 | | NA | AGGGCCAGTCAGAGTGTTGC CAGCAGCTTAGCC | GGTACATCCACCAGGGC CACT | CAGCAGTATAATGACTGGCC GTGCAGT |
| | 21-225_74C3 | | SEQ ID NO:1783 | SEQ ID NO:9795 | SEQ ID NO:17807 |
| | | AA | RASQSVASSLA | GTSTRAT | QQYNDWPCS |
| | | | SEQ ID NO:1784 | SEQ ID NO:9796 | SEQ ID NO:17808 |
| iPS:434633 | | NA | AGGGCCAGTCAGAGTTTTAG CAGCGCCTACTTAGCC | GGTACTTCCAGCAGGGC CACT | CAACAGTATGGTAACTCAAG GACG |
| | 21-225_74G8 | | SEQ ID NO:1785 | SEQ ID NO:9797 | SEQ ID NO:17809 |

FIGURE 49

| | | | | | |
|---|---|---|---|---|---|
| | | AA | RASQSFSSAYLA | GTSSRAT | QQYGNSRT |
| | | | SEQ ID NO:1786 | SEQ ID NO:9798 | SEQ ID NO:17810 |
| iPS:434635 | 21-225_78E6 | NA | AAGTCCAGCCAGAGTGTTTTGTACAGCTCCAACAGTCACAACTACTTAGCT | TGGGCATCTATCCGGGAATCC | CAGCAATATTATAGTACTCCGTGCAGT |
| | | | SEQ ID NO:1787 | SEQ ID NO:9799 | SEQ ID NO:17811 |
| | | AA | KSSQSVLYSSNSHNYLA | WASIRES | QQYYSTPCS |
| | | | SEQ ID NO:1788 | SEQ ID NO:9800 | SEQ ID NO:17812 |
| iPS:434637 | 21-225_78E7 | NA | AGGGCCAGTCAGAATGTTGACAGCAACTACTTAGCC | GGTGCATCCAGCAGGGCCACT | CAGCAGTATGAACGCTCACCGTGGACG |
| | | | SEQ ID NO:1789 | SEQ ID NO:9801 | SEQ ID NO:17813 |
| | | AA | RASQNVDSNYLA | GASSRAT | QQYERSPWT |
| | | | SEQ ID NO:1790 | SEQ ID NO:9802 | SEQ ID NO:17814 |
| iPS:434639 | 21-225_74B7 | NA | AAGTCCAGCCAGACTGTTTTACACAGCTCCAACAATTATAACTACTTAGCT | TGGACATCTACCCGGGAATCC | CAGCAATATTTTAGTAGTCCTCCGACG |
| | | | SEQ ID NO:1791 | SEQ ID NO:9803 | SEQ ID NO:17815 |
| | | AA | KSSQTVLHSSNNYNYLA | WTSTRES | QQYFSSPPT |
| | | | SEQ ID NO:1792 | SEQ ID NO:9804 | SEQ ID NO:17816 |
| iPS:434649 | 21-225_78E11 | NA | AAGTCCAGCCAGAGTGTTTTATACAGCTTCAACAATTACAACTACTTAGCT | TGGGCATCTACCCGGGAATCC | CAGCAATATTATAGTAGTCCTCCGACG |
| | | | SEQ ID NO:1793 | SEQ ID NO:9805 | SEQ ID NO:17817 |
| | | AA | KSSQSVLYSFNNYNYLA | WASTRES | QQYYSSPPT |
| | | | SEQ ID NO:1794 | SEQ ID NO:9806 | SEQ ID NO:17818 |
| iPS:434653 | 21-225_74B5 | NA | AAGTCCAGCCAGAGTGTTTTATTCAGCTCCAACAATTATAACTACTTAGCT | TGGGCATCTACCCGGGAATCC | CAGCAATATTATAGTTCTCCTCCGACG |
| | | | SEQ ID NO:1795 | SEQ ID NO:9807 | SEQ ID NO:17819 |
| | | AA | KSSQSVLFSSNNYNYLA | WASTRES | QQYYSSPPT |

FIGURE 49

| | | | SEQ ID NO:1796 | SEQ ID NO:9808 | SEQ ID NO:17820 |
|---|---|---|---|---|---|
| iPS:434655 | 21-225_78H12 | NA | AAGTCCAGCCAGACTGTTTTACACAGCTTCAACAATTATAACTACTTAGCT | TGGACATCTACCCGGGAATCC | CAGCAATATTTTAGTAGTCCTCCGACG |
| | | | SEQ ID NO:1797 | SEQ ID NO:9809 | SEQ ID NO:17821 |
| | | AA | KSSQTVLHSFNNYNYLA | WTSTRES | QQYFSSPPT |
| | | | SEQ ID NO:1798 | SEQ ID NO:9810 | SEQ ID NO:17822 |
| iPS:434657 | 21-225_79G1 | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:1799 | SEQ ID NO:9811 | SEQ ID NO:17823 |
| | | AA | RASQSVYSSYLA | GASSRST | QHYDNSPWT |
| | | | SEQ ID NO:1800 | SEQ ID NO:9812 | SEQ ID NO:17824 |
| iPS:434663 | 21-225_79F3 | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:1801 | SEQ ID NO:9813 | SEQ ID NO:17825 |
| | | AA | RASQSVYSSYLA | GASSRST | QHYDNSPWT |
| | | | SEQ ID NO:1802 | SEQ ID NO:9814 | SEQ ID NO:17826 |
| iPS:434665 | 21-225_74G4 | NA | AAGTCCAGCCAGAGTGTTTTATACAGTTCCAACAATAATAACTACTTAGCT | TGGGCATCTACCCGGGAATCC | CAGCAATATTATAGTATTCCTCCGACG |
| | | | SEQ ID NO:1803 | SEQ ID NO:9815 | SEQ ID NO:17827 |
| | | AA | KSSQSVLYSSNNNNYLA | WASTRES | QQYYSIPPT |
| | | | SEQ ID NO:1804 | SEQ ID NO:9816 | SEQ ID NO:17828 |
| iPS:434669 | 21-225_79F4 | NA | CGGGCGAGTCAGGGCATTAGCAAGTATTTAGCC | GCTGCATCCAGTTTGCAAGGT | CAACAGTACAGTAATTACCCACTCACT |
| | | | SEQ ID NO:1805 | SEQ ID NO:9817 | SEQ ID NO:17829 |
| | | AA | RASQGISKYLA | AASSLQG | QQYSNYPLT |
| | | | SEQ ID NO:1806 | SEQ ID NO:9818 | SEQ ID NO:17830 |
| iPS:434671 | | NA | AGGGCCAGTCAGATTTTTAGCAGCAGCTACTTAGCC | GGTGCATCCAGCAGGGCCACT | CAGCAGTATGGTAGCTCACGGACG |

FIGURE 49

| | 21-225_74F4 | | SEQ ID NO:1807 | SEQ ID NO:9819 | SEQ ID NO:17831 |
|---|---|---|---|---|---|
| | | AA | RASQIFSSSYLA | GASSRAT | QQYGSSRT |
| | | | SEQ ID NO:1808 | SEQ ID NO:9820 | SEQ ID NO:17832 |
| iPS:434673 | | NA | AGGGCCAGTCTGAGTGTTGT CAACAGCTTAGCC | GGTGCATCCACCAGGGC CACT | CAGCAGTATAATGACTGGCC GTGCAGT |
| | 21-225_74E3 | | SEQ ID NO:1809 | SEQ ID NO:9821 | SEQ ID NO:17833 |
| | | AA | RASLSVVNSLA | GASTRAT | QQYNDWPCS |
| | | | SEQ ID NO:1810 | SEQ ID NO:9822 | SEQ ID NO:17834 |
| iPS:434675 | | NA | ATGTCCAGCCAGAGTGTTTT ACACAGCTTCAACAATAAGA ACTACTTAACT | TGGGCATCTACTTGGGA ATCC | CAGCAATATTATAGTATTCC TCCGACG |
| | 21-225_79G6 | | SEQ ID NO:1811 | SEQ ID NO:9823 | SEQ ID NO:17835 |
| | | AA | MSSQSVLHSFNNKNYLT | WASTWES | QQYYSIPPT |
| | | | SEQ ID NO:1812 | SEQ ID NO:9824 | SEQ ID NO:17836 |
| iPS:434679 | | NA | AAGTCCAGCCAGAGTGTTTT GTACAGCTCCAACAGTCACA ACTACTTAGCT | TGGGCATCTATCCGGGA ATCC | CAGCAATATTATAGTACTCC GTGCAGT |
| | 21-225_79G7 | | SEQ ID NO:1813 | SEQ ID NO:9825 | SEQ ID NO:17837 |
| | | AA | KSSQSVLYSSNSHNYLA | WASIRES | QQYYSTPCS |
| | | | SEQ ID NO:1814 | SEQ ID NO:9826 | SEQ ID NO:17838 |
| iPS:434685 | | NA | AAGTCCAGCCAGAGTATTTT ATACAGCTCCAACAATAATA ACTACTTAGCT | TGGGCATCTACCCGGGA ATCC | CAGCAATATTATATTACTCC TCCGACG |
| | 21-225_79E9 | | SEQ ID NO:1815 | SEQ ID NO:9827 | SEQ ID NO:17839 |
| | | AA | KSSQSILYSSNNNNYLA | WASTRES | QQYYITPPT |
| | | | SEQ ID NO:1816 | SEQ ID NO:9828 | SEQ ID NO:17840 |
| iPS:434687 | | NA | AGGGCCAGTCAGAGTGTTTA CAGCAGCTACTTAGCC | GGTGCATCCAGCCGGGC CACT | CAGCACTATGATAACTCACC GTGGACG |
| | 21-225_75A5 | | SEQ ID NO:1817 | SEQ ID NO:9829 | SEQ ID NO:17841 |
| | | AA | RASQSVYSSYLA | GASSRAT | QHYDNSPWT |

FIGURE 49

| | | | SEQ ID NO:1818 | SEQ ID NO:9830 | SEQ ID NO:17842 |
|---|---|---|---|---|---|
| iPS:434689 | 21-225_79G10 | NA | AAGTCCAGCCAGAGTGTTTT ATTCAGCTCCAACAATTATA ATTACTTAGCT | TGGGCATCTACCCGGGA ATCC | CAGCAATATCATAGTTCTCC TCTGACG |
| | | | SEQ ID NO:1819 | SEQ ID NO:9831 | SEQ ID NO:17843 |
| | | AA | KSSQSVLFSSNNYNYLA | WASTRES | QQYHSSPLT |
| | | | SEQ ID NO:1820 | SEQ ID NO:9832 | SEQ ID NO:17844 |
| iPS:434691 | 21-225_75G7 | NA | AGGGCCAGGCAGAGTGTTG ACAGCAGTTATTTAGCC | GGTGCATCCAGCAGGGC CACT | CAGCAGTATGAAAGCTCACC GTGGACG |
| | | | SEQ ID NO:1821 | SEQ ID NO:9833 | SEQ ID NO:17845 |
| | | AA | RARQSVDSSYLA | GASSRAT | QQYESSPWT |
| | | | SEQ ID NO:1822 | SEQ ID NO:9834 | SEQ ID NO:17846 |
| iPS:434693 | 21-225_79F11 | NA | AGGGCCAGTCAGAGTGTTTA CAGCAGCTACTTAGCC | GGTGCATCCAGCCGGGC CACT | CAGCACTCTGATAACTCACC GTGGACG |
| | | | SEQ ID NO:1823 | SEQ ID NO:9835 | SEQ ID NO:17847 |
| | | AA | RASQSVYSSYLA | GASSRAT | QHSDNSPWT |
| | | | SEQ ID NO:1824 | SEQ ID NO:9836 | SEQ ID NO:17848 |
| iPS:434697 | 21-225_79F12 | NA | AAGTCCAGCCAGAGTATTTT ATACAGCTCCAACAATTACA ACTACTTAGCT | TGGGCATCTACCCGGGA ATCC | CAGCAATATTATAGTACTCC GTGGACG |
| | | | SEQ ID NO:1825 | SEQ ID NO:9837 | SEQ ID NO:17849 |
| | | AA | KSSQSILYSSNNYNYLA | WASTRES | QQYYSTPWT |
| | | | SEQ ID NO:1826 | SEQ ID NO:9838 | SEQ ID NO:17850 |
| iPS:434699 | 21-225_79G12 | NA | AGGGCCAGTCAGAGTGTTTA CAGCAGCTACTTAGCC | GGTGCATCCAGCCGGTC CACT | CAGCACTCTGATAACTCACC GTGGACG |
| | | | SEQ ID NO:1827 | SEQ ID NO:9839 | SEQ ID NO:17851 |
| | | AA | RASQSVYSSYLA | GASSRST | QHSDNSPWT |
| | | | SEQ ID NO:1828 | SEQ ID NO:9840 | SEQ ID NO:17852 |
| iPS:434701 | | NA | AGGGCCAGTCAGAGTGTTTA CAGCAGCTACTTAGCC | GGTGCATCCAGCCGGTC CACT | CAGCACTCTGATAACTCACC GTGGACG |

FIGURE 49

| | 21-225_80A1 | | SEQ ID NO:1829 | SEQ ID NO:9841 | SEQ ID NO:17853 |
|---|---|---|---|---|---|
| | | AA | RASQSVYSSYLA | GASSRST | QHSDNSPWT |
| | | | SEQ ID NO:1830 | SEQ ID NO:9842 | SEQ ID NO:17854 |
| iPS:434703 | 21-225_80C1 | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1831 | SEQ ID NO:9843 | SEQ ID NO:17855 |
| | | AA | RASQSVYSSYLA | GASSRST | QHSDNSPWT |
| | | | SEQ ID NO:1832 | SEQ ID NO:9844 | SEQ ID NO:17856 |
| iPS:434705 | 21-225_80A2 | NA | AGGGCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:1833 | SEQ ID NO:9845 | SEQ ID NO:17857 |
| | | AA | RASQSVSSSYLV | GASTRAT | QQYGCSPLT |
| | | | SEQ ID NO:1834 | SEQ ID NO:9846 | SEQ ID NO:17858 |
| iPS:434707 | 21-225_80D3 | NA | AAGTCCAGCCAGAGTGTTTTATACACCTCCAACAATAACAACTACTTAGCT | TGGGCATCTACCCGGGAATCC | CAGCACTACAATGAAACTCCAGGGAAG |
| | | | SEQ ID NO:1835 | SEQ ID NO:9847 | SEQ ID NO:17859 |
| | | AA | KSSQSVLYTSNNNNYLA | WASTRES | QHYNETPGK |
| | | | SEQ ID NO:1836 | SEQ ID NO:9848 | SEQ ID NO:17860 |
| iPS:434709 | 21-225_80E3 | NA | AGGGCCAGTCAGAGTGTTTACAGCAGCTACTTAGCC | GGTGCATCCAGCCGGTCCACT | CAGCATTCTGATAACTCACCGTGGACG |
| | | | SEQ ID NO:1837 | SEQ ID NO:9849 | SEQ ID NO:17861 |
| | | AA | RASQSVYSSYLA | GASSRST | QHSDNSPWT |
| | | | SEQ ID NO:1838 | SEQ ID NO:9850 | SEQ ID NO:17862 |
| iPS:434711 | 21-225_80H3 | NA | AAGTCCAGCCAGAGTGTTTTACACAGGTCCAACAATTACAACTACTTAGCG | TGGGCATCTACCCGGGAATCC | CAGCAATATTATAGTACTCCTCCGACG |
| | | | SEQ ID NO:1839 | SEQ ID NO:9851 | SEQ ID NO:17863 |
| | | AA | KSSQSVLHRSNNYNYLA | WASTRES | QQYYSTPPT |
| | | | SEQ ID NO:1840 | SEQ ID NO:9852 | SEQ ID NO:17864 |

FIGURE 49

| iPS:434715 | | NA | AGGGCCAGTCAGAATATTTACAGCAGCTACTTAGCC | GGTGCATCCAGCAGGGCCACT | CAGCAGTATGAAAGCTCACCGTGGACC |
|---|---|---|---|---|---|
| | 21-225_80D5 | | SEQ ID NO:1841 | SEQ ID NO:9853 | SEQ ID NO:17865 |
| | | AA | RASQNIYSSYLA | GASSRAT | QQYESSPWT |
| | | | SEQ ID NO:1842 | SEQ ID NO:9854 | SEQ ID NO:17866 |
| iPS:434717 | | NA | AGGGCCAGTCAGAGTGTTGACAGCGGCTACTTAGCC | GGTGCATCCAGCAGGGCCCCT | CAGCAGTATGAAAGCTCACCGTGGACG |
| | 21-225_80A6 | | SEQ ID NO:1843 | SEQ ID NO:9855 | SEQ ID NO:17867 |
| | | AA | RASQSVDSGYLA | GASSRAP | QQYESSPWT |
| | | | SEQ ID NO:1844 | SEQ ID NO:9856 | SEQ ID NO:17868 |
| iPS:434725 | | NA | AGGGCCAGTCAGAGTATTAACAGCAACTACTTAGCC | GGTGCATCCAGCAGGGCCACT | CAGCAGTATGAGAGCTCACCGTGGACG |
| | 21-225_80H7 | | SEQ ID NO:1845 | SEQ ID NO:9857 | SEQ ID NO:17869 |
| | | AA | RASQSINSNYLA | GASSRAT | QQYESSPWT |
| | | | SEQ ID NO:1846 | SEQ ID NO:9858 | SEQ ID NO:17870 |
| iPS:434729 | | NA | AAGTCCAGACAGAGTGTTTTATACAGCTCCAACAATTACAACTACTTAACT | TGGGCATCTACCCGGGAATCC | CAGCAATATTATAGTTCTCCTCCTACT |
| | 21-225_80B12 | | SEQ ID NO:1847 | SEQ ID NO:9859 | SEQ ID NO:17871 |
| | | AA | KSRQSVLYSSNNYNYLT | WASTRES | QQYYSSPPT |
| | | | SEQ ID NO:1848 | SEQ ID NO:9860 | SEQ ID NO:17872 |
| iPS:434731 | | NA | AAGTCCAGCCAGAGTGTTTTATACACCTCCAACAATAACAACTACTTAGCT | TGGGCATCTACCCGGGAATCC | CAGCAATATTATAATACTCCGTGGACG |
| | 21-225_80E9 | | SEQ ID NO:1849 | SEQ ID NO:9861 | SEQ ID NO:17873 |
| | | AA | KSSQSVLYTSNNNNYLA | WASTRES | QQYYNTPWT |
| | | | SEQ ID NO:1850 | SEQ ID NO:9862 | SEQ ID NO:17874 |
| iPS:434735 | | NA | AGGGCCAGTCAGAGTGTTGACAGCAGCTACTTAGCC | GGTGCATCCAGCAGGGCCCCT | CAGCAGTATGAAAGCTCACCGTGGACG |
| | 21-225_80B10 | | SEQ ID NO:1851 | SEQ ID NO:9863 | SEQ ID NO:17875 |

FIGURE 49

| | | AA | RASQSVDSSYLA | GASSRAP | QQYESSPWT |
|---|---|---|---|---|---|
| | | | SEQ ID NO:1852 | SEQ ID NO:9864 | SEQ ID NO:17876 |
| iPS:434737 | | NA | CGGGCGAGTCAGGGCATTGGCAAGTATTTAGCC | ACTACATCCAGTTTGCAAAGT | CAACAGTACAGTAATTACCCGCTCACT |
| | 21-225_74G6 | | SEQ ID NO:1853 | SEQ ID NO:9865 | SEQ ID NO:17877 |
| | | AA | RASQGIGKYLA | TTSSLQS | QQYSNYPLT |
| | | | SEQ ID NO:1854 | SEQ ID NO:9866 | SEQ ID NO:17878 |
| iPS:434741 | | NA | CGGGCGAGTCAGGGCATTGGCAGGTATTTAGCC | ACTGCATCCAGTTTGCAAAGT | CAACAGTACAGTAATTACCCGCTCACT |
| | 21-225_80C11 | | SEQ ID NO:1855 | SEQ ID NO:9867 | SEQ ID NO:17879 |
| | | AA | RASQGIGRYLA | TASSLQS | QQYSNYPLT |
| | | | SEQ ID NO:1856 | SEQ ID NO:9868 | SEQ ID NO:17880 |
| iPS:434743 | | NA | AGGGCCAGTCAGAGTGTTTACAGCAGCTACTTAGCC | GGTGCATCCAGCCGGTCCACT | CAGCACTCTGATAACTCACCGTGGACG |
| | 21-225_74A4 | | SEQ ID NO:1857 | SEQ ID NO:9869 | SEQ ID NO:17881 |
| | | AA | RASQSVYSSYLA | GASSRST | QHSDNSPWT |
| | | | SEQ ID NO:1858 | SEQ ID NO:9870 | SEQ ID NO:17882 |
| iPS:434747 | | NA | AGGGCCAGTCAGAGTGTTAGCAGCAGCTACTTAGCC | GGTGCATCCACCAGGGCCACT | CAGCAGTATGGTAACTCACCGCTCACT |
| | 21-225_80C12 | | SEQ ID NO:1859 | SEQ ID NO:9871 | SEQ ID NO:17883 |
| | | AA | RASQSVSSSYLA | GASTRAT | QQYGNSPLT |
| | | | SEQ ID NO:1860 | SEQ ID NO:9872 | SEQ ID NO:17884 |
| iPS:434751 | | NA | AGGGCCAGTCAGAGTGTTTACAGCAGCTACTTAGCC | GGTGCATCCAGCCGGTCCACT | CAGCACTCTGATAACTCACCGTGGACG |
| | 21-225_80H12 | | SEQ ID NO:1861 | SEQ ID NO:9873 | SEQ ID NO:17885 |
| | | AA | RASQSVYSSYLA | GASSRST | QHSDNSPWT |
| | | | SEQ ID NO:1862 | SEQ ID NO:9874 | SEQ ID NO:17886 |
| iPS:434759 | | NA | AGGGCCAGTCAGAGTGTTTACAGCAGCTACTTAGCC | GGTGCATCCAGCCGGTCCACT | CAGCATTCTGATAACTCACCGTGGACG |
| | 21-225_81C5 | | SEQ ID NO:1863 | SEQ ID NO:9875 | SEQ ID NO:17887 |

FIGURE 49

| | | | | | |
|---|---|---|---|---|---|
| | | AA | RASQSVYSSYLA | GASSRST | QHSDNSPWT |
| | | | SEQ ID NO:1864 | SEQ ID NO:9876 | SEQ ID NO:17888 |
| iPS:434761 | 21-225_81E5 | NA | AAGTCCAGCCAGAGTGTTTT ATTCAGCTCCAACAATTATA ATTACTTAGCT | TGGGCATCTACCCGGGA ATCC | CAGCAATATTATAGTTCTCC TCTGACG |
| | | | SEQ ID NO:1865 | SEQ ID NO:9877 | SEQ ID NO:17889 |
| | | AA | KSSQSVLFSSNNYNYLA | WASTRES | QQYYSSPLT |
| | | | SEQ ID NO:1866 | SEQ ID NO:9878 | SEQ ID NO:17890 |
| iPS:434771 | 21-225_81F9 | NA | AAGTCCAGCCAGAGTGTTTT ATACACCTCCAACAATAACA ACTACTTAGCT | TGGGCATCTACCCGGGA ATCC | CAGCACTACAATGATACTCC AGGGAAG |
| | | | SEQ ID NO:1867 | SEQ ID NO:9879 | SEQ ID NO:17891 |
| | | AA | KSSQSVLYTSNNNNYLA | WASTRES | QHYNDTPGK |
| | | | SEQ ID NO:1868 | SEQ ID NO:9880 | SEQ ID NO:17892 |
| iPS:434773 | 21-225_75D9 | NA | AGGGCCAGTCAGAGTGTTAG CAGCAGCTACTTAGCC | GGTGCATCCAGCAGGGC CACT | CAGCAGTATGAAAGCTCACC GTGGACG |
| | | | SEQ ID NO:1869 | SEQ ID NO:9881 | SEQ ID NO:17893 |
| | | AA | RASQSVSSSYLA | GASSRAT | QQYESSPWT |
| | | | SEQ ID NO:1870 | SEQ ID NO:9882 | SEQ ID NO:17894 |
| iPS:434777 | 21-225_81C11 | NA | AGGGCCAGTCAGAGTGTTTA CAGCAGCTACTTAGCC | GGTGCATCCAGCCGGTC CACT | CAGCATTCTGATAACTCACC GTGGACG |
| | | | SEQ ID NO:1871 | SEQ ID NO:9883 | SEQ ID NO:17895 |
| | | AA | RASQSVYSSYLA | GASSRST | QHSDNSPWT |
| | | | SEQ ID NO:1872 | SEQ ID NO:9884 | SEQ ID NO:17896 |
| iPS:434793 | 21-225_82A5 | NA | AGGGCCAGTCAGAGTGTTAG CAGCAGCTACTTAGCC | GGTGCATCCACCAGGGC CACT | CAGCAGTATGGTAACTCACC GCTCACT |
| | | | SEQ ID NO:1873 | SEQ ID NO:9885 | SEQ ID NO:17897 |
| | | AA | RASQSVSSSYLA | GASTRAT | QQYGNSPLT |
| | | | SEQ ID NO:1874 | SEQ ID NO:9886 | SEQ ID NO:17898 |

FIGURE 49

| iPS:434797 | | NA | AGGGCCAGTGAGAGTGTTAG CAGCAGCTACTTAGTC | GGTGCATCCACCAGGGC CACT | CAGCAGTATGGTTGCTCACC GCTCACT |
| | 21-225_82G5 | | SEQ ID NO:1875 | SEQ ID NO:9887 | SEQ ID NO:17899 |
| | | AA | RASESVSSSYLV | GASTRAT | QQYGCSPLT |
| | | | SEQ ID NO:1876 | SEQ ID NO:9888 | SEQ ID NO:17900 |
| iPS:434805 | | NA | AGGGCCAGTGAGAGTGTTAG CAGCAGCTACTTAGTC | GGTGCATCCACCAGGGC CACT | CAGCAGTATGGTTGCTCACC GCTCACT |
| | 21-225_82D9 | | SEQ ID NO:1877 | SEQ ID NO:9889 | SEQ ID NO:17901 |
| | | AA | RASESVSSSYLV | GASTRAT | QQYGCSPLT |
| | | | SEQ ID NO:1878 | SEQ ID NO:9890 | SEQ ID NO:17902 |
| iPS:434809 | | NA | AGGGCCAGTCAGAGTGTTTA CAGCAGCTACTTAGCC | GGTGCATCCAGCCGGTC CACT | CAGCACTCTGATAACTCACC GTGGACG |
| | 21-225_74F5 | | SEQ ID NO:1879 | SEQ ID NO:9891 | SEQ ID NO:17903 |
| | | AA | RASQSVYSSYLA | GASSRST | QHSDNSPWT |
| | | | SEQ ID NO:1880 | SEQ ID NO:9892 | SEQ ID NO:17904 |
| iPS:434813 | | NA | AGGGCCAGTCAGAGTGTTAG CAGCAGCTACTTAGCC | GGTGCATCCACCAGGGC CTCT | CAGCAGTATGGTAACTCACC GCTCACT |
| | 21-225_82C12 | | SEQ ID NO:1881 | SEQ ID NO:9893 | SEQ ID NO:17905 |
| | | AA | RASQSVSSSYLA | GASTRAS | QQYGNSPLT |
| | | | SEQ ID NO:1882 | SEQ ID NO:9894 | SEQ ID NO:17906 |
| iPS:434815 | | NA | CGGGCAAGTCAGGACATTAG AAATGATTTAGGC | GCTGCATCCAGTTTGCAA AGT | CTACAGCATAATGATTACCC ATTCACT |
| | 21-225_74A11 | | SEQ ID NO:1883 | SEQ ID NO:9895 | SEQ ID NO:17907 |
| | | AA | RASQDIRNDLG | AASSLQS | LQHNDYPFT |
| | | | SEQ ID NO:1884 | SEQ ID NO:9896 | SEQ ID NO:17908 |
| iPS:434821 | | NA | AGGGCCAGTCAGAGTGTTTA CAGCAGCTACTTAGCC | GGTGCATCCAGCCGGTC CACG | CAGCACTCTGATAACTCACC GTGGACG |
| | 21-225_83G1 | | SEQ ID NO:1885 | SEQ ID NO:9897 | SEQ ID NO:17909 |
| | | AA | RASQSVYSSYLA | GASSRST | QHSDNSPWT |
| | | | SEQ ID NO:1886 | SEQ ID NO:9898 | SEQ ID NO:17910 |

FIGURE 49

| iPS:434825 | | NA | AGGGCCAGTCAGAGTGTTAG CAGCAGCTACTTAGTC | GGTGCATCCACCAGGGC CACT | CAGCAGTATGGTTGCTCACC GCTCACT |
|---|---|---|---|---|---|
| | 21-225_83C2 | | SEQ ID NO:1887 | SEQ ID NO:9899 | SEQ ID NO:17911 |
| | | AA | RASQSVSSSYLV | GASTRAT | QQYGCSPLT |
| | | | SEQ ID NO:1888 | SEQ ID NO:9900 | SEQ ID NO:17912 |
| iPS:434827 | | NA | AAGTCCAGCCAGAGTGTTTT ATACACCTCCAACAATAACA ACTACTTAGCT | TGGGCATCTACCCGGGA ATCC | CAGCACTATAATGATACTCC ATGGAAG |
| | 21-225_83F3 | | SEQ ID NO:1889 | SEQ ID NO:9901 | SEQ ID NO:17913 |
| | | AA | KSSQSVLYTSNNNNYLA | WASTRES | QHYNDTPWK |
| | | | SEQ ID NO:1890 | SEQ ID NO:9902 | SEQ ID NO:17914 |
| iPS:434829 | | NA | AAGTCCAGCCAGAGTGTTTT ATACACCTCCAACAATAACA ACTACTTAGCT | TGGGCATCTACCCGGGA ATCC | CAGCACTATTATAATACTCC GTGGACG |
| | 21-225_83G3 | | SEQ ID NO:1891 | SEQ ID NO:9903 | SEQ ID NO:17915 |
| | | AA | KSSQSVLYTSNNNNYLA | WASTRES | QHYYNTPWT |
| | | | SEQ ID NO:1892 | SEQ ID NO:9904 | SEQ ID NO:17916 |
| iPS:434833 | | NA | AGGGCCAGTGAGAGTGTTAG CAGCAGCTACTTAGTC | GGTGCATCCACCAGGGC CACT | CAGCAGTATGGTTGCTCACC GCTCACT |
| | 21-225_83C5 | | SEQ ID NO:1893 | SEQ ID NO:9905 | SEQ ID NO:17917 |
| | | AA | RASESVSSSYLV | GASTRAT | QQYGCSPLT |
| | | | SEQ ID NO:1894 | SEQ ID NO:9906 | SEQ ID NO:17918 |
| iPS:434835 | | NA | AGGGCCAGTCAGAGTGTTGA CAGCGGCTACTTAGCC | GGTGCATCCAGCAGGAC CCCT | CAGCAGTATGAAAGCTCACC GTGGACG |
| | 21-225_83B6 | | SEQ ID NO:1895 | SEQ ID NO:9907 | SEQ ID NO:17919 |
| | | AA | RASQSVDSGYLA | GASSRTP | QQYESSPWT |
| | | | SEQ ID NO:1896 | SEQ ID NO:9908 | SEQ ID NO:17920 |
| iPS:434839 | | NA | AGGGCCAGTCAGAGTGTTTA CAGCAGCTACTTAGCG | GGTGCATCCAGCCGGTC CACT | CAGCACTCTGATAACTCACC GTGGACG |
| | 21-225_83B7 | | SEQ ID NO:1897 | SEQ ID NO:9909 | SEQ ID NO:17921 |

FIGURE 49

| | | | | | |
|---|---|---|---|---|---|
| | | AA | RASQSVYSSYLA | GASSRST | QHSDNSPWT |
| | | | SEQ ID NO:1898 | SEQ ID NO:9910 | SEQ ID NO:17922 |
| iPS:434841 | 21-225_83G7 | NA | AAGTCCAGCCAGACTGTTTT ACACAGCTCCAACAATTATA ACTACTTAGCT | TGGACATCTACCCGGGA ATCC | CAGCAATATTTTAGTAGTCC TCTGACG |
| | | | SEQ ID NO:1899 | SEQ ID NO:9911 | SEQ ID NO:17923 |
| | | AA | KSSQTVLHSSNNYNYLA | WTSTRES | QQYFSSPLT |
| | | | SEQ ID NO:1900 | SEQ ID NO:9912 | SEQ ID NO:17924 |
| iPS:434849 | 21-225_83C10 | NA | AGGGCCAGTCCGAGTGTTCA CAGCAACTACTTAGCC | GGTGCATCCAGCAGGGC CACT | CAGCAGTATGAAAGTTCACC GTGGACG |
| | | | SEQ ID NO:1901 | SEQ ID NO:9913 | SEQ ID NO:17925 |
| | | AA | RASPSVHSNYLA | GASSRAT | QQYESSPWT |
| | | | SEQ ID NO:1902 | SEQ ID NO:9914 | SEQ ID NO:17926 |
| iPS:434851 | 21-225_75A6 | NA | AAGTCCAGACAGAGTGTTTT ACACAGCTCCAACAATTACA ACTACTTAGCT | TGGGCATCTACCCGGGA ATCC | CAGCAATATTATAGTACTCC TCCTACT |
| | | | SEQ ID NO:1903 | SEQ ID NO:9915 | SEQ ID NO:17927 |
| | | AA | KSRQSVLHSSNNYNYLA | WASTRES | QQYYSTPPT |
| | | | SEQ ID NO:1904 | SEQ ID NO:9916 | SEQ ID NO:17928 |
| iPS:434863 | 21-225_84G7 | NA | AAGTCCAGCCAGACTGTTTT ACACAGCTCCAACAATTATA ACTACTTAGCT | TGGACATCTACCCGGGA ATCC | CAGCAATATTTTAGTAGTCC TCCGACG |
| | | | SEQ ID NO:1905 | SEQ ID NO:9917 | SEQ ID NO:17929 |
| | | AA | KSSQTVLHSSNNYNYLA | WTSTRES | QQYFSSPPT |
| | | | SEQ ID NO:1906 | SEQ ID NO:9918 | SEQ ID NO:17930 |
| iPS:434867 | 21-225_79A12 | NA | CGGGCGAGTCAGGTCATTAG CAAGTATTTAGCC | GCTGCATCCAGTTTGCAA AGT | CAACAGTACAGTAATTACCC GCTCACT |
| | | | SEQ ID NO:1907 | SEQ ID NO:9919 | SEQ ID NO:17931 |
| | | AA | RASQVISKYLA | AASSLQS | QQYSNYPLT |
| | | | SEQ ID NO:1908 | SEQ ID NO:9920 | SEQ ID NO:17932 |

FIGURE 49

| iPS:434869 | | NA | AGGGCCAGTCAGAGTATTAA CAGCAACTACTTAGCC | GGTGCATCCAGCAGGGC CACT | CAGCAGTATGAGAGCTCACC GTGGACG |
|---|---|---|---|---|---|
| | 21-225_84E12 | | SEQ ID NO:1909 | SEQ ID NO:9921 | SEQ ID NO:17933 |
| | | AA | RASQSINSNYLA | GASSRAT | QQYESSPWT |
| | | | SEQ ID NO:1910 | SEQ ID NO:9922 | SEQ ID NO:17934 |
| iPS:434871 | | NA | AGGGCCAGTCAGGATGTTAT CACCTACTTAGCC | GGTGCATCCACCAGGGC CACT | CAGGAGTATAATGACTGGCC GTGCAGT |
| | 21-225_85H1 | | SEQ ID NO:1911 | SEQ ID NO:9923 | SEQ ID NO:17935 |
| | | AA | RASQDVITYLA | GASTRAT | QEYNDWPCS |
| | | | SEQ ID NO:1912 | SEQ ID NO:9924 | SEQ ID NO:17936 |
| iPS:434877 | | NA | AAGTCCAGCCAGAGTGTTTT ACACAGCTCTAATAAAAAGA ACTACTTAGCT | TGGGCATCTACCCGGGA ATCC | CAGCAATATTATAGGACTCC GTGGACG |
| | 21-225_85H2 | | SEQ ID NO:1913 | SEQ ID NO:9925 | SEQ ID NO:17937 |
| | | AA | KSSQSVLHSSNKKNYLA | WASTRES | QQYYRTPWT |
| | | | SEQ ID NO:1914 | SEQ ID NO:9926 | SEQ ID NO:17938 |
| iPS:434879 | | NA | AGGGCCAGTCAGAGTGTTTA CAGCAGCTACTTAGCC | GGTGCATCCAGCCGGGC CAGT | CAGCACTATGATAACTCACC GTGGACG |
| | 21-225_85A3 | | SEQ ID NO:1915 | SEQ ID NO:9927 | SEQ ID NO:17939 |
| | | AA | RASQSVYSSYLA | GASSRAS | QHYDNSPWT |
| | | | SEQ ID NO:1916 | SEQ ID NO:9928 | SEQ ID NO:17940 |
| iPS:434881 | | NA | AGGGCCAGTCAGAGTGTTTA CAGCAGCTACTTAGCC | GGTGCATCCAGCCGGGC CACT | CAGCACTATGATAACTCACC GTGGACG |
| | 21-225_85B4 | | SEQ ID NO:1917 | SEQ ID NO:9929 | SEQ ID NO:17941 |
| | | AA | RASQSVYSSYLA | GASSRAT | QHYDNSPWT |
| | | | SEQ ID NO:1918 | SEQ ID NO:9930 | SEQ ID NO:17942 |
| iPS:434883 | | NA | AGGTCCAGTCAGAGTGTTAG CAGCAGCTACTTAGTC | GGTGCATCCACCAGGGC CACT | CAGCAGTATGGTTGCTCACC GCTCACT |
| | 21-225_85B5 | | SEQ ID NO:1919 | SEQ ID NO:9931 | SEQ ID NO:17943 |
| | | AA | RSSQSVSSSYLV | GASTRAT | QQYGCSPLT |

FIGURE 49

| | | | SEQ ID NO:1920 | SEQ ID NO:9932 | SEQ ID NO:17944 |
|---|---|---|---|---|---|
| iPS:434887 | 21-225_85D6 | NA | AGGGCCAGTCAGAGTGTTAGCAGCAGGTACTTAGCC | GGTGCATCCAGCAGGGCCACT | CAGCATTATGATAGCTCACCGTGGACG |
| | | | SEQ ID NO:1921 | SEQ ID NO:9933 | SEQ ID NO:17945 |
| | | AA | RASQSVSSRYLA | GASSRAT | QHYDSSPWT |
| | | | SEQ ID NO:1922 | SEQ ID NO:9934 | SEQ ID NO:17946 |
| iPS:434891 | 21-225_85G6 | NA | AGGGCCAGTCCGAGTGTTGACAGCAGCTACTTAGCC | GGTGCAGCCAGCAGGGCCCCT | CAGCAGTATGAAAGTTCACCGTGGACG |
| | | | SEQ ID NO:1923 | SEQ ID NO:9935 | SEQ ID NO:17947 |
| | | AA | RASPSVDSSYLA | GAASRAP | QQYESSPWT |
| | | | SEQ ID NO:1924 | SEQ ID NO:9936 | SEQ ID NO:17948 |
| iPS:434895 | 21-225_74H7 | NA | AGGGCCAGTCAGAATATTTACAGCAGCTACTTAGCC | GGTGCATCCAGCAGGGCCACT | CAGCAGTATGAAAGCTCACCGTGGACC |
| | | | SEQ ID NO:1925 | SEQ ID NO:9937 | SEQ ID NO:17949 |
| | | AA | RASQNIYSSYLA | GASSRAT | QQYESSPWT |
| | | | SEQ ID NO:1926 | SEQ ID NO:9938 | SEQ ID NO:17950 |
| iPS:434899 | 21-225_85B9 | NA | AGGGCCAGTCAGAGTGTTAACAGCAACTACTTAGCC | GGTGCATCCAGCAGGGCCACT | CAGCAGTATGAAAGCTCGCCGTGGACG |
| | | | SEQ ID NO:1927 | SEQ ID NO:9939 | SEQ ID NO:17951 |
| | | AA | RASQSVNSNYLA | GASSRAT | QQYESSPWT |
| | | | SEQ ID NO:1928 | SEQ ID NO:9940 | SEQ ID NO:17952 |
| iPS:434901 | 21-225_85H9 | NA | AAGTCCAGCCAGAGTGTTTTACACAGGTCCAACAATTACAACTACTTAGCG | TGGGCATCTACCCGGGAATCC | CAGCAATATTATAGTACTCCTCCGACG |
| | | | SEQ ID NO:1929 | SEQ ID NO:9941 | SEQ ID NO:17953 |
| | | AA | KSSQSVLHRSNNYNYLA | WASTRES | QQYYSTPPT |
| | | | SEQ ID NO:1930 | SEQ ID NO:9942 | SEQ ID NO:17954 |
| iPS:434907 | 21-225_85G10 | NA | AGGGCCAGTCAGAGTGTTTGGAGCGGCTACTTAGCC | GGTGCATCTAGCAGGGCCACT | CAGCAGTATGAGAGTTCACCGTGGACG |
| | | | SEQ ID NO:1931 | SEQ ID NO:9943 | SEQ ID NO:17955 |

FIGURE 49

| | | | | | |
|---|---|---|---|---|---|
| | | AA | RASQSVWSGYLA | GASSRAT | QQYESSPWT |
| | | | SEQ ID NO:1932 | SEQ ID NO:9944 | SEQ ID NO:17956 |
| iPS:434909 | 21-225_85C11 | NA | AAGTCCAGCCAGAGTGTTTT GTACAGCTCCAACAGTCACA ACTTCTTAGCT | TGGGCATTTATCCGGGA ATCC | CAGCAATATTATAGTACTCC GTGCAGT |
| | | | SEQ ID NO:1933 | SEQ ID NO:9945 | SEQ ID NO:17957 |
| | | AA | KSSQSVLYSSNSHNFLA | WAFIRES | QQYYSTPCS |
| | | | SEQ ID NO:1934 | SEQ ID NO:9946 | SEQ ID NO:17958 |
| iPS:434911 | 21-225_85D11 | NA | AGGTCCAGTCAGAGTGTTAG CAGCAGCTACTTAGTC | GGTGCATCCACCAGGGC CACT | CAGCAGTATGGTTGCTCACC GCTCACT |
| | | | SEQ ID NO:1935 | SEQ ID NO:9947 | SEQ ID NO:17959 |
| | | AA | RSSQSVSSSYLV | GASTRAT | QQYGCSPLT |
| | | | SEQ ID NO:1936 | SEQ ID NO:9948 | SEQ ID NO:17960 |
| iPS:434913 | 21-225_86C1 | NA | AGGGCCAGTCAGAGTGTTTA CAGCAGCTACTTAGCC | GGTGCATCCAGCCGGGC CACT | CAGCACTATGATAACTCACC GTGGACG |
| | | | SEQ ID NO:1937 | SEQ ID NO:9949 | SEQ ID NO:17961 |
| | | AA | RASQSVYSSYLA | GASSRAT | QHYDNSPWT |
| | | | SEQ ID NO:1938 | SEQ ID NO:9950 | SEQ ID NO:17962 |
| iPS:434921 | 21-225_86E4 | NA | AGGGCCAGTCAGAGTGTTTA CAGCAGCTACTTAGCC | GGTGCATCCAGCCGGTC CACT | CAGCACTCTGATAACTCACC GTGGACG |
| | | | SEQ ID NO:1939 | SEQ ID NO:9951 | SEQ ID NO:17963 |
| | | AA | RASQSVYSSYLA | GASSRST | QHSDNSPWT |
| | | | SEQ ID NO:1940 | SEQ ID NO:9952 | SEQ ID NO:17964 |
| iPS:434935 | 21-225_86E9 | NA | AAGTCCAGCCAGAGTGTTTT GCACAGATCCAACAATTATA ATTACTTAGCT | TGGGCATCTACCCGGGA ATCC | CAGCAATATCATAGTAGTCC ACTGACG |
| | | | SEQ ID NO:1941 | SEQ ID NO:9953 | SEQ ID NO:17965 |
| | | AA | KSSQSVLHRSNNYNYLA | WASTRES | QQYHSSPLT |
| | | | SEQ ID NO:1942 | SEQ ID NO:9954 | SEQ ID NO:17966 |

FIGURE 49

| iPS:434939 | | NA | AGGGCCAGTCAGAGTGTTTA CAGCAGCTACTTAGCC | GGTGCATCCAGCCGGTC CACT | CAGCACTCTGATAACTCACC GTGGACG |
|---|---|---|---|---|---|
| | 21-225_86C11 | | SEQ ID NO:1943 | SEQ ID NO:9955 | SEQ ID NO:17967 |
| | | AA | RASQSVYSSYLA | GASSRST | QHSDNSPWT |
| | | | SEQ ID NO:1944 | SEQ ID NO:9956 | SEQ ID NO:17968 |
| iPS:434943 | | NA | AGGGCCAGTCAGAGTGTTGA CAGCAACTACTTAGCC | GGTGCATCTGCCAGGAC CACT | CAGCAGTATGAAAGCTCACC GTGGACG |
| | 21-225_87H1 | | SEQ ID NO:1945 | SEQ ID NO:9957 | SEQ ID NO:17969 |
| | | AA | RASQSVDSNYLA | GASARTT | QQYESSPWT |
| | | | SEQ ID NO:1946 | SEQ ID NO:9958 | SEQ ID NO:17970 |
| iPS:434945 | | NA | AGGGCCAGTCAGAGTGTTTA CAGCAGCTACTTAGCC | GGTGCATCCAGCCGGTC CACT | CAGCATTCTGATAACTCACC GTGGACG |
| | 21-225_87E5 | | SEQ ID NO:1947 | SEQ ID NO:9959 | SEQ ID NO:17971 |
| | | AA | RASQSVYSSYLA | GASSRST | QHSDNSPWT |
| | | | SEQ ID NO:1948 | SEQ ID NO:9960 | SEQ ID NO:17972 |
| iPS:434947 | | NA | CGGGCGAGTCAGGGCATTAG CAATTATTTAGCC | GCTGCATCCAGTTTGCAC AGT | CTACTCTATCTTACTTACCCG CTCACC |
| | 21-225_87B7 | | SEQ ID NO:1949 | SEQ ID NO:9961 | SEQ ID NO:17973 |
| | | AA | RASQGISNYLA | AASSLHS | LLYLTYPLT |
| | | | SEQ ID NO:1950 | SEQ ID NO:9962 | SEQ ID NO:17974 |
| iPS:434955 | | NA | AGGGCCAGTCAGAGTGTTTA CAGCAGCTACTTAGCC | GGTGCATCCAGCCGGTC CACT | CAGCATTCTGATAACTCACC GTGGACG |
| | 21-225_87C9 | | SEQ ID NO:1951 | SEQ ID NO:9963 | SEQ ID NO:17975 |
| | | AA | RASQSVYSSYLA | GASSRST | QHSDNSPWT |
| | | | SEQ ID NO:1952 | SEQ ID NO:9964 | SEQ ID NO:17976 |
| iPS:434957 | | NA | AGGGCCAGTCAGAGTGTTAG CAGCAGCTACTTAGCC | GGTGCATCCACCAGGGC CTCT | CAGCAGTATGGTAACTCACC GCTCACC |
| | 21-225_87A10 | | SEQ ID NO:1953 | SEQ ID NO:9965 | SEQ ID NO:17977 |
| | | AA | RASQSVSSSYLA | GASTRAS | QQYGNSPLT |
| | | | SEQ ID NO:1954 | SEQ ID NO:9966 | SEQ ID NO:17978 |

**FIGURE 49**

| iPS:434959 | | NA | AAGTCCAGCCAGAGTGTTTT ACACAGCTCCAACAATATGA ACTACTTAGCT | TGGGCATCTACCCGGAA ATCC | CAGCAATATTATAGTAGTCC GTGCAGT |
|---|---|---|---|---|---|
| | 21-225_87E10 | | SEQ ID NO:1955 | SEQ ID NO:9967 | SEQ ID NO:17979 |
| | | AA | KSSQSVLHSSNNMNYLA | WASTRKS | QQYYSSPCS |
| | | | SEQ ID NO:1956 | SEQ ID NO:9968 | SEQ ID NO:17980 |
| iPS:434961 | | NA | AGGGCCAGTCAGAGTGTTTA CAGCAGCTACTTAGCC | GGTGCATCCAGCCGGTC CACT | CAGCACTCTGATAACTCACC GTGGACG |
| | 21-225_87A12 | | SEQ ID NO:1957 | SEQ ID NO:9969 | SEQ ID NO:17981 |
| | | AA | RASQSVYSSYLA | GASSRST | QHSDNSPWT |
| | | | SEQ ID NO:1958 | SEQ ID NO:9970 | SEQ ID NO:17982 |
| iPS:434965 | | NA | AAGTCCAGCCAGAGTGTTTT ACACAGCTCCAACAATTACA ACTACTTAACT | TGGGCATCTACCCGGAA ATCC | CAGCAATATTATAGTTCTCC TCCGACG |
| | 21-225_88A1 | | SEQ ID NO:1959 | SEQ ID NO:9971 | SEQ ID NO:17983 |
| | | AA | KSSQSVLHSSNNYNYLT | WASTRKS | QQYYSSPPT |
| | | | SEQ ID NO:1960 | SEQ ID NO:9972 | SEQ ID NO:17984 |
| iPS:434969 | | NA | AGGGCCAGTCAGAGTGTTTA CAGCAGCTACTTAGCC | GGTGCATCCAGCCGGTC CACG | CAGCATTCTGATAACTCACC GTGGACG |
| | 21-225_88H1 | | SEQ ID NO:1961 | SEQ ID NO:9973 | SEQ ID NO:17985 |
| | | AA | RASQSVYSSYLA | GASSRST | QHSDNSPWT |
| | | | SEQ ID NO:1962 | SEQ ID NO:9974 | SEQ ID NO:17986 |
| iPS:434971 | | NA | AAGTCCAGCCAGAGTGTTTT ATACACCTCCAACAATAACA ACTACTTAGCT | TGGGCATCTACCCGGGA ATCC | CAGCAATATTATAATACTCC GTGGACG |
| | 21-225_88G2 | | SEQ ID NO:1963 | SEQ ID NO:9975 | SEQ ID NO:17987 |
| | | AA | KSSQSVLYTSNNNNYLA | WASTRES | QQYYNTPWT |
| | | | SEQ ID NO:1964 | SEQ ID NO:9976 | SEQ ID NO:17988 |

FIGURE 49

| iPS:434973 | | NA | AAGTCCAGCCAGAGTGTTTT ATACATCTCCAACAATAATA ATTACTTAGCT | TGGGCATCTACCCGGGA ATCC | CAGCAATATTATAGTACTCC TCCGACG |
|---|---|---|---|---|---|
| | 21-225_88B4 | | SEQ ID NO:1965 | SEQ ID NO:9977 | SEQ ID NO:17989 |
| | | AA | KSSQSVLYISNNNNYLA | WASTRES | QQYYSTPPT |
| | | | SEQ ID NO:1966 | SEQ ID NO:9978 | SEQ ID NO:17990 |
| iPS:434977 | | NA | CGGGCAAGTCAGGGCATTAG AAATGATTTAGGC | GCTGCATCCAGTTTGCAA AGT | CTACAGCATAATGATTACCC ATTCACT |
| | 21-225_88A5 | | SEQ ID NO:1967 | SEQ ID NO:9979 | SEQ ID NO:17991 |
| | | AA | RASQGIRNDLG | AASSLQS | LQHNDYPFT |
| | | | SEQ ID NO:1968 | SEQ ID NO:9980 | SEQ ID NO:17992 |
| iPS:434981 | | NA | AGGGCCAGTCAGAGTGTTTA CAGCAGCTACTTAGCC | GGTGCATCCAGCCGGTC CACT | CAGCACTCTGATAACTCACC GTGGACG |
| | 21-225_88E7 | | SEQ ID NO:1969 | SEQ ID NO:9981 | SEQ ID NO:17993 |
| | | AA | RASQSVYSSYLA | GASSRST | QHSDNSPWT |
| | | | SEQ ID NO:1970 | SEQ ID NO:9982 | SEQ ID NO:17994 |
| iPS:434983 | | NA | AGGGCCAGTCAGAGTGTTTA CAGCAGCTACTTAGCC | GGTGCATCCAGCCGGTC CACT | CAGCACTCTGATAACTCACC GTGGACG |
| | 21-225_88F7 | | SEQ ID NO:1971 | SEQ ID NO:9983 | SEQ ID NO:17995 |
| | | AA | RASQSVYSSYLA | GASSRST | QHSDNSPWT |
| | | | SEQ ID NO:1972 | SEQ ID NO:9984 | SEQ ID NO:17996 |
| iPS:434995 | | NA | AGGGCCAGTCAGAGTGTTTA CAGCAGCTACTTAGCC | GGTGCATCCAGCCGGTC CACT | CAGCACTCTGATAACTCACC GTGGACG |
| | 21-225_88G9 | | SEQ ID NO:1973 | SEQ ID NO:9985 | SEQ ID NO:17997 |
| | | AA | RASQSVYSSYLA | GASSRST | QHSDNSPWT |
| | | | SEQ ID NO:1974 | SEQ ID NO:9986 | SEQ ID NO:17998 |
| iPS:434997 | | NA | AAGTCCAGCCAGAGTGTTTT ACACAGCTCCAACAATTGGA ATTACTTAGCT | TGGGCATTTACTCGGAA ATCC | CAGCAATATTATAGAGCTCC TCCGACG |
| | 21-225_88C10 | | SEQ ID NO:1975 | SEQ ID NO:9987 | SEQ ID NO:17999 |

FIGURE 49

| | | | | | |
|---|---|---|---|---|---|
| | | AA | KSSQSVLHSSNNWNYLA | WAFTRKS | QQYYRAPPT |
| | | | SEQ ID NO:1976 | SEQ ID NO:9988 | SEQ ID NO:18000 |
| iPS:434999 | | NA | AGGGCCAGTCAGAGTGTTTACAGCAGCTACTTAGCC | GGTGCATCCAGCCGGGCCACT | CAGCACTCTGATAACTCACCGTGGACG |
| | 21-225_75A8 | | SEQ ID NO:1977 | SEQ ID NO:9989 | SEQ ID NO:18001 |
| | | AA | RASQSVYSSYLA | GASSRAT | QHSDNSPWT |
| | | | SEQ ID NO:1978 | SEQ ID NO:9990 | SEQ ID NO:18002 |
| iPS:435009 | | NA | AGGTCTAGTCAGAGCCTCCTGCATAGTAGTGGATACAACTATTTGGAT | TTGGGTTCTAATCGGGCCTCC | ATGCAAGCTCTACATATTCCTCTCACT |
| | 21-225_89G4 | | SEQ ID NO:1979 | SEQ ID NO:9991 | SEQ ID NO:18003 |
| | | AA | RSSQSLLHSSGYNYLD | LGSNRAS | MQALHIPLT |
| | | | SEQ ID NO:1980 | SEQ ID NO:9992 | SEQ ID NO:18004 |
| iPS:435013 | | NA | AGGGCCAGTCAGAGTGTTTACAGCAGCTACTTAGCC | GGTGCATCCAGCCGGTCCACT | CAGCACTATGATAACTCACCGTGGACG |
| | 21-225_89D5 | | SEQ ID NO:1981 | SEQ ID NO:9993 | SEQ ID NO:18005 |
| | | AA | RASQSVYSSYLA | GASSRST | QHYDNSPWT |
| | | | SEQ ID NO:1982 | SEQ ID NO:9994 | SEQ ID NO:18006 |
| iPS:435015 | | NA | AGGGCCAGTCAGAGTGTTAACAGCAACTACTTAGCC | GGTGCATTCAGCAGGGCCACT | CAGCAGTATGAAAGCTCAGTGTGGACG |
| | 21-225_89H5 | | SEQ ID NO:1983 | SEQ ID NO:9995 | SEQ ID NO:18007 |
| | | AA | RASQSVNSNYLA | GAFSRAT | QQYESSVWT |
| | | | SEQ ID NO:1984 | SEQ ID NO:9996 | SEQ ID NO:18008 |
| iPS:435025 | | NA | AGGGCCAGTCAGAGTGTTTACAGCAGCTACTTAGCC | GGTGCATCCAGCCGGTCCACT | CAGCATTCTGATAACTCTCCGTGGACG |
| | 21-225_89E10 | | SEQ ID NO:1985 | SEQ ID NO:9997 | SEQ ID NO:18009 |
| | | AA | RASQSVYSSYLA | GASSRST | QHSDNSPWT |
| | | | SEQ ID NO:1986 | SEQ ID NO:9998 | SEQ ID NO:18010 |
| iPS:435029 | | NA | AGGGCCAGTCAGAGTGTTGACAGCAACTTCTTAGCC | GGTGCATCTGCCAGGACCACT | CAGCAGTATGAAATCTCACCGTGGACG |

FIGURE 49

| | 21-225_89A11 | | SEQ ID NO:1987 | SEQ ID NO:9999 | SEQ ID NO:18011 |
|---|---|---|---|---|---|
| | | AA | RASQSVDSNFLA | GASARTT | QQYEISPWT |
| | | | SEQ ID NO:1988 | SEQ ID NO:10000 | SEQ ID NO:18012 |
| iPS:435039 | 21-225_90G4 | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1989 | SEQ ID NO:10001 | SEQ ID NO:18013 |
| | | AA | RASQSVYSSYLA | GASSRST | QHSDNSPWT |
| | | | SEQ ID NO:1990 | SEQ ID NO:10002 | SEQ ID NO:18014 |
| iPS:435041 | 21-225_90A5 | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:1991 | SEQ ID NO:10003 | SEQ ID NO:18015 |
| | | AA | RASQSVYSSYLA | GASSRAT | QHYDNSPWT |
| | | | SEQ ID NO:1992 | SEQ ID NO:10004 | SEQ ID NO:18016 |
| iPS:435043 | 21-225_90G5 | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:1993 | SEQ ID NO:10005 | SEQ ID NO:18017 |
| | | AA | RASQSVYSSYLA | GASSRAT | QHYDNSPWT |
| | | | SEQ ID NO:1994 | SEQ ID NO:10006 | SEQ ID NO:18018 |
| iPS:435045 | 21-225_90H5 | NA | CGGGCAAGTCAGGGCATTAGAAATGATTTAGGC | ATTGCATCCAGTTTGCAAAGT | CTACAGCATAATAGTTACCCGATCACC |
| | | | SEQ ID NO:1995 | SEQ ID NO:10007 | SEQ ID NO:18019 |
| | | AA | RASQGIRNDLG | IASSLQS | LQHNSYPIT |
| | | | SEQ ID NO:1996 | SEQ ID NO:10008 | SEQ ID NO:18020 |
| iPS:435051 | 21-225_90D9 | NA | AAGTCCAGCCAGACTGTTTTACACAGCTCCAACAATTATAACTACTTAGCT | TGGACATCTACCCGGGAATCC | CAGCAATATCTTAGTAGTCCTCTGACG |
| | | | SEQ ID NO:1997 | SEQ ID NO:10009 | SEQ ID NO:18021 |
| | | AA | KSSQTVLHSSNNYNYLA | WTSTRES | QQYLSSPLT |
| | | | SEQ ID NO:1998 | SEQ ID NO:10010 | SEQ ID NO:18022 |

FIGURE 49

| iPS:435053 | | NA | AAGTCCAGCCAGAGTGTTTT ACACAACTCCAACAATAATA ACTACTTGGCT | TGGGCATCTACGCGGGA GTCC | CAACAATATTATAGTAGTCC TCCGACG |
|---|---|---|---|---|---|
| | 21-225_75F9 | | SEQ ID NO:1999 | SEQ ID NO:10011 | SEQ ID NO:18023 |
| | | AA | KSSQSVLHNSNNNNYLA | WASTRES | QQYYSSPPT |
| | | | SEQ ID NO:2000 | SEQ ID NO:10012 | SEQ ID NO:18024 |
| iPS:435055 | | NA | AGGGCCAGTCAGAGTGTTTA CAGCAGCTACTTAGCC | GGTGCATCCAGCCGGTC CACT | CAGCACTATGATAACTCACC GTGGACG |
| | 21-225_90F10 | | SEQ ID NO:2001 | SEQ ID NO:10013 | SEQ ID NO:18025 |
| | | AA | RASQSVYSSYLA | GASSRST | QHYDNSPWT |
| | | | SEQ ID NO:2002 | SEQ ID NO:10014 | SEQ ID NO:18026 |
| iPS:435059 | | NA | AGGTATAGTCAGAGCCTCGT GCATAGTAGTGGATACAACT ATTTGGAT | TTGGGTTCTAATCGGGCC TCC | ATGCAAGCTCTACACCCTCC TCTCACT |
| | 21-225_90C11 | | SEQ ID NO:2003 | SEQ ID NO:10015 | SEQ ID NO:18027 |
| | | AA | RYSQSLVHSSGYNYLD | LGSNRAS | MQALHPPLT |
| | | | SEQ ID NO:2004 | SEQ ID NO:10016 | SEQ ID NO:18028 |
| iPS:435071 | | NA | AAGTCCAGCCAGAGTGTTTT ATACACCTCCAACAATAACA ACTACTTAGCT | TGGGCATCTACCCGGGA ATCC | CAGCACTATTATAATACTCC GTGGAAG |
| | 21-225_91F1 | | SEQ ID NO:2005 | SEQ ID NO:10017 | SEQ ID NO:18029 |
| | | AA | KSSQSVLYTSNNNNYLA | WASTRES | QHYYNTPWK |
| | | | SEQ ID NO:2006 | SEQ ID NO:10018 | SEQ ID NO:18030 |
| iPS:435073 | | NA | AGGGCCAGTCAGAGTGTTTA CAGCAGCTACTTAGCC | GGTGCATCCAGCCGGTC CACT | CAGCACTATGATAACTCACC GTGGACG |
| | 21-225_91B2 | | SEQ ID NO:2007 | SEQ ID NO:10019 | SEQ ID NO:18031 |
| | | AA | RASQSVYSSYLA | GASSRST | QHYDNSPWT |
| | | | SEQ ID NO:2008 | SEQ ID NO:10020 | SEQ ID NO:18032 |
| iPS:435075 | | NA | AGGGCCAGTCAGAGTGTTTA CAGCAGCTACTTAGCC | GGTGCATCCAGCCGGTC CACT | CAGCACTATGATAACTCACC GTGGACG |

FIGURE 49

| | | | | | |
|---|---|---|---|---|---|
| | **21-225_91B3** | | SEQ ID NO:2009 | SEQ ID NO:10021 | SEQ ID NO:18033 |
| | | AA | RASQSVYSSYLA | GASSRST | QHYDNSPWT |
| | | | SEQ ID NO:2010 | SEQ ID NO:10022 | SEQ ID NO:18034 |
| **iPS:435077** | **21-225_91F3** | NA | AGGGCCAGTCAGAGTGTTTACAGCAGCTACTTAGCC | GGTGCATCCAGCCGGTCCACT | CAGCATTCTGATAACTCACCGTGGACG |
| | | | SEQ ID NO:2011 | SEQ ID NO:10023 | SEQ ID NO:18035 |
| | | AA | RASQSVYSSYLA | GASSRST | QHSDNSPWT |
| | | | SEQ ID NO:2012 | SEQ ID NO:10024 | SEQ ID NO:18036 |
| **iPS:435079** | **21-225_91B4** | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:2013 | SEQ ID NO:10025 | SEQ ID NO:18037 |
| | | AA | RASQSVYSSYLA | GASSRST | QHYDNSPWT |
| | | | SEQ ID NO:2014 | SEQ ID NO:10026 | SEQ ID NO:18038 |
| **iPS:435087** | **21-225_91G8** | NA | AAGTCCAGCCAGAGTGTTTTATACACCTCCAACAATAACAACTACTTAGCT | TGGGCATCTACCCGGGAATCC | CAGCAATATTATACTACTCCGTGGACG |
| | | | SEQ ID NO:2015 | SEQ ID NO:10027 | SEQ ID NO:18039 |
| | | AA | KSSQSVLYTSNNNYLA | WASTRES | QQYYTTPWT |
| | | | SEQ ID NO:2016 | SEQ ID NO:10028 | SEQ ID NO:18040 |
| **iPS:435089** | **21-225_91E9** | NA | AGGGCCAGTCAGAGTGTTTACAGCAGCTACTTAGCG | GGTGCATCCAGCCGGGCCACG | CAGCACTCTGATAACTCACCGTGGACG |
| | | | SEQ ID NO:2017 | SEQ ID NO:10029 | SEQ ID NO:18041 |
| | | AA | RASQSVYSSYLA | GASSRAT | QHSDNSPWT |
| | | | SEQ ID NO:2018 | SEQ ID NO:10030 | SEQ ID NO:18042 |
| **iPS:435097** | **21-225_92B1** | NA | AGGGCCAGTCAGAGTGTTGGCAGCAACTACTTAGCC | GGTGCATCCAGCAGGGCCACT | CAGCAGTATGAAAGTTCACCGTGGACG |
| | | | SEQ ID NO:2019 | SEQ ID NO:10031 | SEQ ID NO:18043 |
| | | AA | RASQSVGSNYLA | GASSRAT | QQYESSPWT |
| | | | SEQ ID NO:2020 | SEQ ID NO:10032 | SEQ ID NO:18044 |

FIGURE 49

| iPS:435103 | | NA | AGGTCTAGTCAGAGCCTCGT GCATAGTAGTGGATACAACT ATTTGGAT | TTGGGTTCTAATCGGGCC TCC | ATGCAAGCTCTACATATTCC TCTCACT |
|---|---|---|---|---|---|
| | 21-225_92B2 | | SEQ ID NO:2021 | SEQ ID NO:10033 | SEQ ID NO:18045 |
| | | AA | RSSQSLVHSSGYNYLD | LGSNRAS | MQALHIPLT |
| | | | SEQ ID NO:2022 | SEQ ID NO:10034 | SEQ ID NO:18046 |
| iPS:435109 | | NA | CGGGCCAGTCAGGATGTTAT CACCTACTTAGCC | GGTGCATCCACCAGGGC CACT | CAGGAGTATAATGACTGGCC GTGCAGT |
| | 21-225_92H5 | | SEQ ID NO:2023 | SEQ ID NO:10035 | SEQ ID NO:18047 |
| | | AA | RASQDVITYLA | GASTRAT | QEYNDWPCS |
| | | | SEQ ID NO:2024 | SEQ ID NO:10036 | SEQ ID NO:18048 |
| iPS:435111 | | NA | AGGGCCAGTCAGAGTGTTTA CAGCAGCTACTTAGCC | GGTGCATCCAGCCGGTC CACT | CAGCATTATGATAACTCTCC GTGGACG |
| | 21-225_92D6 | | SEQ ID NO:2025 | SEQ ID NO:10037 | SEQ ID NO:18049 |
| | | AA | RASQSVYSSYLA | GASSRST | QHYDNSPWT |
| | | | SEQ ID NO:2026 | SEQ ID NO:10038 | SEQ ID NO:18050 |
| iPS:435113 | | NA | AAGTCCAGTCAGAATATTTT ATCCAGCTCCAACAATAAGA ACTACTTAACT | TGGACATCTACCCGGGA ATCC | CAGCAATATTTTAGTGTTCCT CCGACG |
| | 21-225_92E6 | | SEQ ID NO:2027 | SEQ ID NO:10039 | SEQ ID NO:18051 |
| | | AA | KSSQNILSSSNNKNYLT | WTSTRES | QQYFSVPPT |
| | | | SEQ ID NO:2028 | SEQ ID NO:10040 | SEQ ID NO:18052 |
| iPS:435115 | | NA | AGGGCCAGTCAGAGTGTTTA CAGCAGCTACTTAGCC | GGTGCATCCAGCCGGTC CACT | CAGCACTATGATAACTCACC GTGGACG |
| | 21-225_77C5 | | SEQ ID NO:2029 | SEQ ID NO:10041 | SEQ ID NO:18053 |
| | | AA | RASQSVYSSYLA | GASSRST | QHYDNSPWT |
| | | | SEQ ID NO:2030 | SEQ ID NO:10042 | SEQ ID NO:18054 |
| iPS:435167 | 21 225 92F12 | NA | AAGTCCAGCCAGAGTGTTTT ACACAGGTCCAACAATTACA ACTACTTAGCG | TGGGCATCTACCCGGGA ATCC | CAGCAATATTATAGTACTCC TCCGACG |

749

FIGURE 49

| | | | | | |
|---|---|---|---|---|---|
| | **21-225_92F12** | | SEQ ID NO:2031 | SEQ ID NO:10043 | SEQ ID NO:18055 |
| | | AA | KSSQSVLHRSNNYNYLA | WASTRES | QQYYSTPPT |
| | | | SEQ ID NO:2032 | SEQ ID NO:10044 | SEQ ID NO:18056 |
| iPS:435171 | **21-225_93C2** | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:2033 | SEQ ID NO:10045 | SEQ ID NO:18057 |
| | | AA | RASQSVYSSYLA | GASSRAT | QHYDNSPWT |
| | | | SEQ ID NO:2034 | SEQ ID NO:10046 | SEQ ID NO:18058 |
| iPS:435177 | **21-225_93E4** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:2035 | SEQ ID NO:10047 | SEQ ID NO:18059 |
| | | AA | RASQSVYSSYLA | GASSRST | QHSDNSPWT |
| | | | SEQ ID NO:2036 | SEQ ID NO:10048 | SEQ ID NO:18060 |
| iPS:435183 | **21-225_93E9** | NA | AGGGCCAGTCAGAGTGTTGACAGCAGCTACCTAGCC | GGTGCATCCAGCAGGGCCCCT | CAGCAGTATGAAAGCTCACCGTGGACG |
| | | | SEQ ID NO:2037 | SEQ ID NO:10049 | SEQ ID NO:18061 |
| | | AA | RASQSVDSSYLA | GASSRAP | QQYESSPWT |
| | | | SEQ ID NO:2038 | SEQ ID NO:10050 | SEQ ID NO:18062 |
| iPS:435195 | **21-225_94D3** | NA | AGGGCCAGTCAGAGTGTTAGCAGCAGGTACTTAGCC | GGTGCATCCAGCAGGGCCACT | CAGCATTATGATAGCTCACCGTGGACG |
| | | | SEQ ID NO:2039 | SEQ ID NO:10051 | SEQ ID NO:18063 |
| | | AA | RASQSVSSRYLA | GASSRAT | QHYDSSPWT |
| | | | SEQ ID NO:2040 | SEQ ID NO:10052 | SEQ ID NO:18064 |
| iPS:435197 | **21-225_94F3** | NA | CGGGCAAGTCAGGCCATTAGAGATGATTTAGGC | GCTGCATCCAGTTTGCAAAGT | CTCCAGCATTATAGTTACCCTCGGACG |
| | | | SEQ ID NO:2041 | SEQ ID NO:10053 | SEQ ID NO:18065 |
| | | AA | RASQAIRDDLG | AASSLQS | LQHYSYPRT |
| | | | SEQ ID NO:2042 | SEQ ID NO:10054 | SEQ ID NO:18066 |

FIGURE 49

| iPS:435203 | | NA | AAGTCCAGCCAGACTGTTTT ACACAGCTCCAACAATTATA ACTACTTAGCT | TGGACATCTACCCGGGA ATCC | CAGCAATATTTTAGTAGTCC TCCGACG |
|---|---|---|---|---|---|
| | 21-225_75A7 | | SEQ ID NO:2043 | SEQ ID NO:10055 | SEQ ID NO:18067 |
| | | AA | KSSQTVLHSSNNYNYLA | WTSTRES | QQYFSSPPT |
| | | | SEQ ID NO:2044 | SEQ ID NO:10056 | SEQ ID NO:18068 |
| iPS:435209 | | NA | AAGTCCAGCCAGAGTGTTTT ACACAACTCCAACAATTACA ACTACTTAACT | TGGGCATCTACCCGGAA ATCC | CAGCAATATTATAGTTCTCC TCCGACG |
| | 21-225_75A10 | | SEQ ID NO:2045 | SEQ ID NO:10057 | SEQ ID NO:18069 |
| | | AA | KSSQSVLHNSNNYNYLT | WASTRKS | QQYYSSPPT |
| | | | SEQ ID NO:2046 | SEQ ID NO:10058 | SEQ ID NO:18070 |
| iPS:435211 | | NA | AAGTCCAGCCAGAGTGTTTT ATTCAGCTCCAACAATTATA ATTACTTAGCT | TGGGCATCTACCCGGGA ATCC | CAGCAATATCATAGTTCTCC TCTGACG |
| | 21-225_94E11 | | SEQ ID NO:2047 | SEQ ID NO:10059 | SEQ ID NO:18071 |
| | | AA | KSSQSVLFSSNNYNYLA | WASTRES | QQYHSSPLT |
| | | | SEQ ID NO:2048 | SEQ ID NO:10060 | SEQ ID NO:18072 |
| iPS:435215 | | NA | AAGTCCAGCCAGAGTGTTTT ACACAGGTCCAACAATTACA ACTACTTAGCG | TGGGCATCTACCCGGGA ATCC | CAGCAATATTATAGTACTCC TCCGACG |
| | 21-225_94E12 | | SEQ ID NO:2049 | SEQ ID NO:10061 | SEQ ID NO:18073 |
| | | AA | KSSQSVLHRSNNYNYLA | WASTRES | QQYYSTPPT |
| | | | SEQ ID NO:2050 | SEQ ID NO:10062 | SEQ ID NO:18074 |
| iPS:435217 | | NA | AGGGCCAGTCAGAGTGTTTA CAGCAGCTACTTAGCG | GGTGCATCCAGCCGGTC CACT | CAGCATTATGATAACTCACC GTGGACG |
| | 21-225_94F12 | | SEQ ID NO:2051 | SEQ ID NO:10063 | SEQ ID NO:18075 |
| | | AA | RASQSVYSSYLA | GASSRST | QHYDNSPWT |
| | | | SEQ ID NO:2052 | SEQ ID NO:10064 | SEQ ID NO:18076 |

FIGURE 49

| iPS:435219 | | NA | AGGGCCAGTCAGAGTGTTTA CAGCAGCTACTTAGCC | GGTGCATCCAGCCGGGC CACT | CAGCACTATGATAACTCACC GTGGACG |
|---|---|---|---|---|---|
| | 21-225_95D2 | | SEQ ID NO:2053 | SEQ ID NO:10065 | SEQ ID NO:18077 |
| | | AA | RASQSVYSSYLA | GASSRAT | QHYDNSPWT |
| | | | SEQ ID NO:2054 | SEQ ID NO:10066 | SEQ ID NO:18078 |
| iPS:435221 | | NA | AGGGCCAGTATGAGTGTTGT CAACAGCTTAGCC | GGTGCATCCACCAGGGC CACT | CAGCAGTATAATGACTGGCC GTGCAGT |
| | 21-225_95G2 | | SEQ ID NO:2055 | SEQ ID NO:10067 | SEQ ID NO:18079 |
| | | AA | RASMSVVNSLA | GASTRAT | QQYNDWPCS |
| | | | SEQ ID NO:2056 | SEQ ID NO:10068 | SEQ ID NO:18080 |
| iPS:435227 | | NA | AAGTCCAGCCAGAGTGTTTT ATTCAGATCCAACAATTATA ATTACTTAGCT | TGGGCATCTACCCGGGA ATCC | CAGCAATATCATAGTTCTCC TCTGACG |
| | 21-225_95G4 | | SEQ ID NO:2057 | SEQ ID NO:10069 | SEQ ID NO:18081 |
| | | AA | KSSQSVLFRSNNYNYLA | WASTRES | QQYHSSPLT |
| | | | SEQ ID NO:2058 | SEQ ID NO:10070 | SEQ ID NO:18082 |
| iPS:435235 | | NA | AGGGCCAGTCAGAGTGTTTA CAGCAGCTACTTAGCC | GGTGCATCCAGCCGGTC CACT | CAGCACTATGATAACTCACC GTGGACG |
| | 21-225_95F9 | | SEQ ID NO:2059 | SEQ ID NO:10071 | SEQ ID NO:18083 |
| | | AA | RASQSVYSSYLA | GASSRST | QHYDNSPWT |
| | | | SEQ ID NO:2060 | SEQ ID NO:10072 | SEQ ID NO:18084 |
| iPS:435237 | | NA | AGGGCCAGTCAGAGTGTTTA CAGCAGCTACTTAGCG | GGTGCATCCAGCCGGTC CACT | CAGCACTATGATAACTCACC GTGGACG |
| | 21-225_95G9 | | SEQ ID NO:2061 | SEQ ID NO:10073 | SEQ ID NO:18085 |
| | | AA | RASQSVYSSYLA | GASSRST | QHYDNSPWT |
| | | | SEQ ID NO:2062 | SEQ ID NO:10074 | SEQ ID NO:18086 |
| iPS:435239 | | NA | AGGGCCAGTCAGAGTGTTTA CAGCAGCTACTTAGCC | GGTGCATCCAGCCGGGC CACT | CAGCACTATGATAACTCTCC GTGGACG |
| | 21-225_95H10 | | SEQ ID NO:2063 | SEQ ID NO:10075 | SEQ ID NO:18087 |
| | | AA | RASQSVYSSYLA | GASSRAT | QHYDNSPWT |

FIGURE 49

| | | | SEQ ID NO:2064 | SEQ ID NO:10076 | SEQ ID NO:18088 |
|---|---|---|---|---|---|
| iPS:435245 | | NA | AAGTCCAGCCAGAGTGTTTT ATACAGCTCCAACAATGCGA ACTACTTAGCT | TGGGCATCTACCCGGGA ATCC | CAGCAATATTATAGTACTCC GTGCAGT |
| | 21-225_95E12 | | SEQ ID NO:2065 | SEQ ID NO:10077 | SEQ ID NO:18089 |
| | | AA | KSSQSVLYSSNNANYLA | WASTRES | QQYYSTPCS |
| | | | SEQ ID NO:2066 | SEQ ID NO:10078 | SEQ ID NO:18090 |
| iPS:435247 | | NA | AGGGCCAGTCAGAGCGTTAG CAGCAGCTACTTAGCT | GGTGCATCCACCAGGGC CTCT | CAGCAGTATGGTAACTCACC GCTCACT |
| | 21-225_96G1 | | SEQ ID NO:2067 | SEQ ID NO:10079 | SEQ ID NO:18091 |
| | | AA | RASQSVSSSYLA | GASTRAS | QQYGNSPLT |
| | | | SEQ ID NO:2068 | SEQ ID NO:10080 | SEQ ID NO:18092 |
| iPS:435249 | | NA | AAGTCCAGCCAGAGTGTTTT ACACAGCTCTAATAAAAAGA ACTACTTAGCT | TGGGCATCTACCTGGGA ATCC | CAGCAATATTATAGGACTCC GTGGACG |
| | 21-225_96E2 | | SEQ ID NO:2069 | SEQ ID NO:10081 | SEQ ID NO:18093 |
| | | AA | KSSQSVLHSSNKKNYLA | WASTWES | QQYYRTPWT |
| | | | SEQ ID NO:2070 | SEQ ID NO:10082 | SEQ ID NO:18094 |
| iPS:435251 | | NA | CGGGCAAGTCAGGGCATTAG AAATGATTTAGGC | GCTGCATCCACTTTGCAA AGT | CTACAGCATAGTAATTACCC GCTCACT |
| | 21-225_96A3 | | SEQ ID NO:2071 | SEQ ID NO:10083 | SEQ ID NO:18095 |
| | | AA | RASQGIRNDLG | AASTLQS | LQHSNYPLT |
| | | | SEQ ID NO:2072 | SEQ ID NO:10084 | SEQ ID NO:18096 |
| iPS:435253 | | NA | CGGGCAAGTCAGGACATTAG AAATGATTTAGGC | GGTGTATCCAGTTTGCAA AGT | CTACAGCATAATGATTACCC ATTCACT |
| | 21-225_96A4 | | SEQ ID NO:2073 | SEQ ID NO:10085 | SEQ ID NO:18097 |
| | | AA | RASQDIRNDLG | GVSSLQS | LQHNDYPFT |
| | | | SEQ ID NO:2074 | SEQ ID NO:10086 | SEQ ID NO:18098 |

FIGURE 49

| iPS:435255 | | NA | AAGTCCAGCCAGAGTGTTTT GCACAGCTCCAACAATTATA ATTACTTAGCT | TGGGCATCTACCCGGGA ATCC | CAGCAATATTATAGTAGTCC ACCGACG |
|---|---|---|---|---|---|
| | 21-225_96D5 | | SEQ ID NO:2075 | SEQ ID NO:10087 | SEQ ID NO:18099 |
| | | AA | KSSQSVLHSSNNYNYLA | WASTRES | QQYYSSPPT |
| | | | SEQ ID NO:2076 | SEQ ID NO:10088 | SEQ ID NO:18100 |
| iPS:435257 | | NA | AAGTCCAGCCAGAGTGTTTT GTACAGCTCCAACAGTCACA ACTACTTAGCT | TGGGCATCTATCCGGGA ATCC | CAGCAATATTATAGTACTCC GTGCAGT |
| | 21-225_96H5 | | SEQ ID NO:2077 | SEQ ID NO:10089 | SEQ ID NO:18101 |
| | | AA | KSSQSVLYSSNSHNYLA | WASIRES | QQYYSTPCS |
| | | | SEQ ID NO:2078 | SEQ ID NO:10090 | SEQ ID NO:18102 |
| iPS:435259 | | NA | CGGGCGAGTCAGGGCATTAG CAATTATTTAGCC | GCTGCTTCCAGTTTGCAA AGT | CACCAGTATAATGATTACCC ATTCACT |
| | 21-225_96C6 | | SEQ ID NO:2079 | SEQ ID NO:10091 | SEQ ID NO:18103 |
| | | AA | RASQGISNYLA | AASSLQS | HQYNDYPFT |
| | | | SEQ ID NO:2080 | SEQ ID NO:10092 | SEQ ID NO:18104 |
| iPS:435267 | | NA | AAGTCCAGTCAGAATATTTT ATCCAGCTCCAACAATAAGA ACTACTTAACT | TGGACATCTACCCGGGA ATCC | CAGCAATATTTTAGTGTTCCT CCGACG |
| | 21-225_96D10 | | SEQ ID NO:2081 | SEQ ID NO:10093 | SEQ ID NO:18105 |
| | | AA | KSSQNILSSSNNKNYLT | WTSTRES | QQYFSVPPT |
| | | | SEQ ID NO:2082 | SEQ ID NO:10094 | SEQ ID NO:18106 |
| iPS:435273 | | NA | AGGGCCAGTCAGAGTGTTTA CAGCAGCTACTTAGCC | GGTGCATCCAGCCGGTC CACT | CAGCATTCTGATAACTCACC GTGGACG |
| | 21-225_97A2 | | SEQ ID NO:2083 | SEQ ID NO:10095 | SEQ ID NO:18107 |
| | | AA | RASQSVYSSYLA | GASSRST | QHSDNSPWT |
| | | | SEQ ID NO:2084 | SEQ ID NO:10096 | SEQ ID NO:18108 |

FIGURE 49

| iPS:435279 | | NA | AAGTCCAGCCAGAGTGTTTT ATACACCTCCAACAATAACA ACTACTTAGCT | TGGGCATCTACCCGGGA ATCC | CAGCACTACAATGATACTCC ATGGAAG |
|---|---|---|---|---|---|
| | 21-225_97H4 | | SEQ ID NO:2085 | SEQ ID NO:10097 | SEQ ID NO:18109 |
| | | AA | KSSQSVLYTSNNNNYLA | WASTRES | QHYNDTPWK |
| | | | SEQ ID NO:2086 | SEQ ID NO:10098 | SEQ ID NO:18110 |
| iPS:435281 | | NA | AGGGCCAGTCAGAGTGTTTA CAGCAGCTACTTAGCC | GGTGCATCCAGCCGGGC CACT | CAGCACTCTGATAACTCACC GTGGACG |
| | 21-225_97E5 | | SEQ ID NO:2087 | SEQ ID NO:10099 | SEQ ID NO:18111 |
| | | AA | RASQSVYSSYLA | GASSRAT | QHSDNSPWT |
| | | | SEQ ID NO:2088 | SEQ ID NO:10100 | SEQ ID NO:18112 |
| iPS:435291 | | NA | CGGGCGAGTCAGGGTATTAA CAACTGGTTAGTC | GCTGCATCCAGTTTGCAA AGT | CAACAGGCTAACAGTTTCCC ATTCACT |
| | 21-225_146E1 | | SEQ ID NO:2089 | SEQ ID NO:10101 | SEQ ID NO:18113 |
| | | AA | RASQGINNWLV | AASSLQS | QQANSFPFT |
| | | | SEQ ID NO:2090 | SEQ ID NO:10102 | SEQ ID NO:18114 |
| iPS:435293 | | NA | CGGGCAAGTCAGGGCATTAG AAATGATTTAGGC | GCTGCATCCAGTTTGCAA AGT | CTTCAGCATAGTACTTACCC GCTCACT |
| | 21-225_146F1 | | SEQ ID NO:2091 | SEQ ID NO:10103 | SEQ ID NO:18115 |
| | | AA | RASQGIRNDLG | AASSLQS | LQHSTYPLT |
| | | | SEQ ID NO:2092 | SEQ ID NO:10104 | SEQ ID NO:18116 |
| iPS:435295 | | NA | CGGGCAAGTCAGAGCATTAG CGACTATTTAAAT | ACTACATCCAGTTTGCAA AGT | CAACAGAGTTACAGTACCCC CACT |
| | 21-225_146H1 | | SEQ ID NO:2093 | SEQ ID NO:10105 | SEQ ID NO:18117 |
| | | AA | RASQSISDYLN | TTSSLQS | QQSYSTPT |
| | | | SEQ ID NO:2094 | SEQ ID NO:10106 | SEQ ID NO:18118 |
| iPS:435297 | | NA | AAGTCTAGTCAGAGCCTCCT GCATGGTGATGGAAAGACCT ATTTGTAT | GAAGTTTCCAACCGGTTC TCT | ATGCAAAGTATACAGCTTCC GTGGACG |
| | 21-225_146B3 | | SEQ ID NO:2095 | SEQ ID NO:10107 | SEQ ID NO:18119 |

755

FIGURE 49

| | | AA | KSSQSLLHGDGKTYLY | EVSNRFS | MQSIQLPWT |
|---|---|---|---|---|---|
| | | | SEQ ID NO:2096 | SEQ ID NO:10108 | SEQ ID NO:18120 |
| iPS:435299 | 21-225_146D4 | NA | AAGTCCAGCCAGAGTGTTTT ATACAGCTCCAACAATAACA ACTACTTAGCT | TGGGCATCTACCCGGGA ATCC | CAGCAATATTATAGTACTCC GTGCAGT |
| | | | SEQ ID NO:2097 | SEQ ID NO:10109 | SEQ ID NO:18121 |
| | | AA | KSSQSVLYSSNNNNYLA | WASTRES | QQYYSTPCS |
| | | | SEQ ID NO:2098 | SEQ ID NO:10110 | SEQ ID NO:18122 |
| iPS:435301 | 21-225_146G4 | NA | AGGGCCAGTCAGAATATTAT CAGCAGCTATTTAGCC | GGTGTATCTAGTCGGGC CACT | CAACAATATGGTAGGTCACC ATTCAAT |
| | | | SEQ ID NO:2099 | SEQ ID NO:10111 | SEQ ID NO:18123 |
| | | AA | RASQNIISSYLA | GVSSRAT | QQYGRSPFN |
| | | | SEQ ID NO:2100 | SEQ ID NO:10112 | SEQ ID NO:18124 |
| iPS:435303 | 21-225_146A6 | NA | CGGGCGAGTCAGGGTATTAG CAACTGGTTAGCC | GCTGCATCCAGTTTGCAA GGT | CAACAGACTGACAGTTTCCC ATTCACT |
| | | | SEQ ID NO:2101 | SEQ ID NO:10113 | SEQ ID NO:18125 |
| | | AA | RASQGISNWLA | AASSLQG | QQTDSFPFT |
| | | | SEQ ID NO:2102 | SEQ ID NO:10114 | SEQ ID NO:18126 |
| iPS:435305 | 21-225_146C9 | NA | AAGTCCAGCCAGAGTGTTTT ACACAGCTCCAACAATTATA ATTACTTAGCT | TGGGCATCTACCCGGAA ATCC | CAGCAATATTATAGTACTCC GTGCAGT |
| | | | SEQ ID NO:2103 | SEQ ID NO:10115 | SEQ ID NO:18127 |
| | | AA | KSSQSVLHSSNNYNYLA | WASTRKS | QQYYSTPCS |
| | | | SEQ ID NO:2104 | SEQ ID NO:10116 | SEQ ID NO:18128 |
| iPS:435307 | 21-225_146E9 | NA | CGGGCAAGTCAGAGCATTAG CGACTATTTAAAT | ACTACATCCAGTTTGCAA AGT | CAACAGAGTTACAGTACCCC CACT |
| | | | SEQ ID NO:2105 | SEQ ID NO:10117 | SEQ ID NO:18129 |
| | | AA | RASQSISDYLN | TTSSLQS | QQSYSTPT |
| | | | SEQ ID NO:2106 | SEQ ID NO:10118 | SEQ ID NO:18130 |

FIGURE 49

| iPS:435309 | | NA | AAGTCCAGCCAGAATATTTT ACACAGCTCCAACAATAACA ACTACTTAGCT | TGGGCATCTACCCGGGA ATCC | CAGCAATATTATACTACTCC GTGCAGT |
|---|---|---|---|---|---|
| | 21-225_146F9 | | SEQ ID NO:2107 | SEQ ID NO:10119 | SEQ ID NO:18131 |
| | | AA | KSSQNILHSSNNNNYLA | WASTRES | QQYYTTPCS |
| | | | SEQ ID NO:2108 | SEQ ID NO:10120 | SEQ ID NO:18132 |
| iPS:435311 | | NA | CGGGCAAGTCAGGGCATTAG AAATGATTTAGGC | GCTGCATCCAGTTTGCAA AGT | CTACAGCATAATAGTTACCC GCTCACT |
| | 21-225_146H9 | | SEQ ID NO:2109 | SEQ ID NO:10121 | SEQ ID NO:18133 |
| | | AA | RASQGIRNDLG | AASSLQS | LQHNSYPLT |
| | | | SEQ ID NO:2110 | SEQ ID NO:10122 | SEQ ID NO:18134 |
| iPS:435313 | | NA | CGGGCAAGTCAGGACATTAG AAATAATTTTGGC | GCTGCATCCAGTTTGCAA AGT | CTCCAACATGATAGTTACCC GCTCACT |
| | 21-225_146G11 | | SEQ ID NO:2111 | SEQ ID NO:10123 | SEQ ID NO:18135 |
| | | AA | RASQDIRNNFG | AASSLQS | LQHDSYPLT |
| | | | SEQ ID NO:2112 | SEQ ID NO:10124 | SEQ ID NO:18136 |
| iPS:435315 | | NA | AAGTCTAGTCAGAGCCTCCT GCATAGTGAAGGAAAGACC TATTTGTAT | GAAGTTTCCCACCGGGT CTCT | ATGCAAAGTACACAGTTTCC TCCCACT |
| | 21-225_147B2 | | SEQ ID NO:2113 | SEQ ID NO:10125 | SEQ ID NO:18137 |
| | | AA | KSSQSLLHSEGKTYLY | EVSHRVS | MQSTQFPPT |
| | | | SEQ ID NO:2114 | SEQ ID NO:10126 | SEQ ID NO:18138 |
| iPS:435317 | | NA | AGGGCCAGTCAGAGTGTTGG CAGCAGCTACTTAGCC | GGTGCATCCAGCAGGGC CACT | CAGCAGTATGGTAGCTTATT CACT |
| | 21-225_147D2 | | SEQ ID NO:2115 | SEQ ID NO:10127 | SEQ ID NO:18139 |
| | | AA | RASQSVGSSYLA | GASSRAT | QQYGSLFT |
| | | | SEQ ID NO:2116 | SEQ ID NO:10128 | SEQ ID NO:18140 |
| iPS:435319 | | NA | AGGGCCAGTCAGAGTGTTAT CAGTAGCTACTTAGCC | GGTGCATCCAGCAGGGC CACT | CAACAATATGGTAGGTCACC ATTCAAT |
| | 21-225_147E3 | | SEQ ID NO:2117 | SEQ ID NO:10129 | SEQ ID NO:18141 |

FIGURE 49

| | | | | | |
|---|---|---|---|---|---|
| | | AA | RASQSVISSYLA | GASSRAT | QQYGRSPFN |
| | | | SEQ ID NO:2118 | SEQ ID NO:10130 | SEQ ID NO:18142 |
| iPS:435321 | 21-225_147E4 | NA | AAGTCCAGCCAGAGTGTTTT ACACAGCTCCAACAATTACA ACTACTTAGCT | TGGGCATCTACCCGGGA ATCC | CAGCAATATTATAGTACTCC ATCCACT |
| | | | SEQ ID NO:2119 | SEQ ID NO:10131 | SEQ ID NO:18143 |
| | | AA | KSSQSVLHSSNNYNYLA | WASTRES | QQYYSTPST |
| | | | SEQ ID NO:2120 | SEQ ID NO:10132 | SEQ ID NO:18144 |
| iPS:435323 | 21-225_147D5 | NA | AAGTCCAGCCAGAGTGTTTT ATACAGCTCCAACAATAACA ACTACTTAGCT | TGGGCATCTACCCGGGA ATCC | CACCAATATTATAGTACTCC GTGCAGT |
| | | | SEQ ID NO:2121 | SEQ ID NO:10133 | SEQ ID NO:18145 |
| | | AA | KSSQSVLYSSNNNNYLA | WASTRES | HQYYSTPCS |
| | | | SEQ ID NO:2122 | SEQ ID NO:10134 | SEQ ID NO:18146 |
| iPS:435325 | 21-225_147H5 | NA | CGGGCAAGTCGGGGCATTAG AGATGATTTAGGC | GCTGCATCCAGTTTGCAG AGT | CTACAGCATTATAGTTATCC TCGGACG |
| | | | SEQ ID NO:2123 | SEQ ID NO:10135 | SEQ ID NO:18147 |
| | | AA | RASRGIRDDLG | AASSLQS | LQHYSYPRT |
| | | | SEQ ID NO:2124 | SEQ ID NO:10136 | SEQ ID NO:18148 |
| iPS:435327 | 21-225_147G6 | NA | AAGTCCAGCCAGAGTGTTTT ATACAGCTCCAACAGTAACA ACTACTTAGCT | TGGGCATCTGCCCGGGA ATCC | CAGCAATATTATACTACTCC TCCCACT |
| | | | SEQ ID NO:2125 | SEQ ID NO:10137 | SEQ ID NO:18149 |
| | | AA | KSSQSVLYSSNSNNYLA | WASARES | QQYYTTPPT |
| | | | SEQ ID NO:2126 | SEQ ID NO:10138 | SEQ ID NO:18150 |
| iPS:435329 | 21-225_147A8 | NA | AAGACTAGTCAGAGCCTCCT GCATAGTGAAGGAAAGACC TATTTGTAT | GAAGTTTCCAACCGGTTC TCT | ATGCAAAGTATACAGCTAAT CACC |
| | | | SEQ ID NO:2127 | SEQ ID NO:10139 | SEQ ID NO:18151 |
| | | AA | KTSQSLLHSEGKTYLY | EVSNRFS | MQSIQLIT |

FIGURE 49

| | | | SEQ ID NO:2128 | SEQ ID NO:10140 | SEQ ID NO:18152 |
|---|---|---|---|---|---|
| iPS:435331 | | NA | AGGGCCAGTCAGAGAATTTT CAGCAACTACTTAGCC | GGTGCATCCAGCAGGGC CACT | CAGCAGTATGATAGCTCACC GTGGACG |
| | 21-225_147G8 | | SEQ ID NO:2129 | SEQ ID NO:10141 | SEQ ID NO:18153 |
| | | AA | RASQRIFSNYLA | GASSRAT | QQYDSSPWT |
| | | | SEQ ID NO:2130 | SEQ ID NO:10142 | SEQ ID NO:18154 |
| iPS:435333 | | NA | CGGGCGAGTCAGGACATTAA CAATTATTTAGCC | GCTGCATCCAGTTTGCAA AGT | CAACAGTATAATAGTTACCC TCTCACT |
| | 21-225_147E9 | | SEQ ID NO:2131 | SEQ ID NO:10143 | SEQ ID NO:18155 |
| | | AA | RASQDINNYLA | AASSLQS | QQYNSYPLT |
| | | | SEQ ID NO:2132 | SEQ ID NO:10144 | SEQ ID NO:18156 |
| iPS:435335 | | NA | CGGGCGAGTCAGAATATTAG CAACTGGTTAACC | GCTGCATCCAGTTTGCAA AGT | CAACAGACTGACAGTTTCCC ATTCACT |
| | 21-225_147D10 | | SEQ ID NO:2133 | SEQ ID NO:10145 | SEQ ID NO:18157 |
| | | AA | RASQNISNWLT | AASSLQS | QQTDSFPFT |
| | | | SEQ ID NO:2134 | SEQ ID NO:10146 | SEQ ID NO:18158 |
| iPS:435339 | | NA | CGGGCGAGTCAGGGTATTAG CAACTGGTTAGCC | GCTGCATCCAGTTTGCAA AGT | CAACAGACTGACAGTTTCCC ATTCACT |
| | 21-225_147D12 | | SEQ ID NO:2135 | SEQ ID NO:10147 | SEQ ID NO:18159 |
| | | AA | RASQGISNWLA | AASSLQS | QQTDSFPFT |
| | | | SEQ ID NO:2136 | SEQ ID NO:10148 | SEQ ID NO:18160 |
| iPS:435341 | | NA | AAGTCTAGTCAGAGCCTCCT GCATGGTGATGGAAAGACCT ATTTTTAT | GAAGTTTCCCACCGGTTC TCT | ATGCAAAGTATACAGATTCC GTGGACG |
| | 21-225_148B2 | | SEQ ID NO:2137 | SEQ ID NO:10149 | SEQ ID NO:18161 |
| | | AA | KSSQSLLHGDGKTYFY | EVSHRFS | MQSIQIPWT |
| | | | SEQ ID NO:2138 | SEQ ID NO:10150 | SEQ ID NO:18162 |
| iPS:435343 | | NA | CGGGCGAGTCAGGGTATTAG CAACTGGTTAGCC | GCTGCATCCAGTTTGCAA AGT | CAACAGACTGACAGTTTCCC ATTCACT |
| | 21-225_148E2 | | SEQ ID NO:2139 | SEQ ID NO:10151 | SEQ ID NO:18163 |

FIGURE 49

| | | | | | |
|---|---|---|---|---|---|
| | | AA | RASQGISNWLA | AASSLQS | QQTDSFPFT |
| | | | SEQ ID NO:2140 | SEQ ID NO:10152 | SEQ ID NO:18164 |
| iPS:435345 | 21-225_148G3 | NA | AAGTCCAGCCAACGTGTTTTACACAGCTCCAACAATTATAACTACTTAGCT | TGGGCATCTACCCGGGATTCC | CAGCAATATTATAGTACTCCATTCACT |
| | | | SEQ ID NO:2141 | SEQ ID NO:10153 | SEQ ID NO:18165 |
| | | AA | KSSQRVLHSSNNYNYLA | WASTRDS | QQYYSTPFT |
| | | | SEQ ID NO:2142 | SEQ ID NO:10154 | SEQ ID NO:18166 |
| iPS:435347 | 21-225_148C4 | NA | CGGGCAAGTCAGAGCATTATCAACTATTTAAAT | ACTGCATCCAGTTTACAGAGT | CAACAGAGTTACAGTACCCCCACT |
| | | | SEQ ID NO:2143 | SEQ ID NO:10155 | SEQ ID NO:18167 |
| | | AA | RASQSIINYLN | TASSLQS | QQSYSTPT |
| | | | SEQ ID NO:2144 | SEQ ID NO:10156 | SEQ ID NO:18168 |
| iPS:435349 | 21-225_148F5 | NA | AAGTCTAGTCAGAGCCTCCTGCATAGTGAAGGAAAGACCTATTTGTAT | GAAGTTTCCTACCGGGTCTCT | ATGCAAAGTATACAGCTTCCGCTCACT |
| | | | SEQ ID NO:2145 | SEQ ID NO:10157 | SEQ ID NO:18169 |
| | | AA | KSSQSLLHSEGKTYLY | EVSYRVS | MQSIQLPLT |
| | | | SEQ ID NO:2146 | SEQ ID NO:10158 | SEQ ID NO:18170 |
| iPS:435351 | 21-225_148B6 | NA | CGGGCGAGTCAGGGCATTAGCAAATATTTAGCC | GCTGCATCCAGTTTGCAAAGT | CAACAGTATAATAGTTTCCCATTCACT |
| | | | SEQ ID NO:2147 | SEQ ID NO:10159 | SEQ ID NO:18171 |
| | | AA | RASQGISKYLA | AASSLQS | QQYNSFPFT |
| | | | SEQ ID NO:2148 | SEQ ID NO:10160 | SEQ ID NO:18172 |
| iPS:435353 | 21-225_148F8 | NA | AAGTCCAGCCAGAGTGCTTTACACAGCTCCAACAATTACAACTACTTAGCT | TGGGCATCTACCCGGAAATCC | CAGCAATATTATAGTATTCCTCCGACG |
| | | | SEQ ID NO:2149 | SEQ ID NO:10161 | SEQ ID NO:18173 |
| | | AA | KSSQSALHSSNNYNYLA | WASTRKS | QQYYSIPPT |
| | | | SEQ ID NO:2150 | SEQ ID NO:10162 | SEQ ID NO:18174 |

FIGURE 49

| iPS:435355 | | NA | CGGGCAAGTCAGAGCATTAG TAACTATTTAAAT | ATTGCATCCAGTTTGCAA AGT | CAACAGAGTTACAGTACCCC CACT |
|---|---|---|---|---|---|
| | 21-225_148H9 | | SEQ ID NO:2151 | SEQ ID NO:10163 | SEQ ID NO:18175 |
| | | AA | RASQSISNYLN | IASSLQS | QQSYSTPT |
| | | | SEQ ID NO:2152 | SEQ ID NO:10164 | SEQ ID NO:18176 |
| iPS:435357 | | NA | AAGTCTAGTCAGAGCCTCCT GCATGGTGATGGAAAGACCT ATTTGTAT | GAAGTTTCCAACCGGTTC TCT | ATGCAAAGTATACAGCTTCC GTGGACG |
| | 21-225_148G10 | | SEQ ID NO:2153 | SEQ ID NO:10165 | SEQ ID NO:18177 |
| | | AA | KSSQSLLHGDGKTYLY | EVSNRFS | MQSIQLPWT |
| | | | SEQ ID NO:2154 | SEQ ID NO:10166 | SEQ ID NO:18178 |
| iPS:435359 | | NA | AAGTCTAGTCAGAGCCTCCT GCATAGTGAGGGAAAGACC TATTTGTAT | GAAGTTTCCTACCGGGTC TCT | ATGCAGAGTATACAGCTTCC GCTCACT |
| | 21-225_148H10 | | SEQ ID NO:2155 | SEQ ID NO:10167 | SEQ ID NO:18179 |
| | | AA | KSSQSLLHSEGKTYLY | EVSYRVS | MQSIQLPLT |
| | | | SEQ ID NO:2156 | SEQ ID NO:10168 | SEQ ID NO:18180 |
| iPS:435361 | | NA | CGGGCAAGTCAGGGCATTAG AAATGATTTAGGC | GCTGCATCCAGTTTGCAA AGT | CTACAGCATCGTAATTACCC GCTCACT |
| | 21-225_148E11 | | SEQ ID NO:2157 | SEQ ID NO:10169 | SEQ ID NO:18181 |
| | | AA | RASQGIRNDLG | AASSLQS | LQHRNYPLT |
| | | | SEQ ID NO:2158 | SEQ ID NO:10170 | SEQ ID NO:18182 |
| iPS:435363 | | NA | CGGGCAAGTCAGGGCATTAG AAATGCCTTAGGC | GCTGCATCCAGTTTGCAA AGT | CTACAGCATAATAGTTACCC TCTCATT |
| | 21-225_148F12 | | SEQ ID NO:2159 | SEQ ID NO:10171 | SEQ ID NO:18183 |
| | | AA | RASQGIRNALG | AASSLQS | LQHNSYPLI |
| | | | SEQ ID NO:2160 | SEQ ID NO:10172 | SEQ ID NO:18184 |
| iPS:435365 | 21 225 149F1 | NA | AAGTCTAGTCAGAGCCTCCT GCATGGTGATGGAAAGACCT ATTTTTAT | GAAGTTTCCCACCGGTTC TCT | ATGCAAAGTATACAGATTCC GTGGACG |

FIGURE 49

| | | | | | |
|---|---|---|---|---|---|
| | **21-225_149F1** | | SEQ ID NO:2161 | SEQ ID NO:10173 | SEQ ID NO:18185 |
| | | AA | KSSQSLLHGDGKTYFY | EVSHRFS | MQSIQIPWT |
| | | | SEQ ID NO:2162 | SEQ ID NO:10174 | SEQ ID NO:18186 |
| iPS:435367 | **21-225_149G1** | NA | CGGGCAAGTCAGGGCATTAGAAATGATTTAGGC | GCTGCATCCAATTTGCAAAGT | CTACAGCATAATAGTTACCCGCTCACT |
| | | | SEQ ID NO:2163 | SEQ ID NO:10175 | SEQ ID NO:18187 |
| | | AA | RASQGIRNDLG | AASNLQS | LQHNSYPLT |
| | | | SEQ ID NO:2164 | SEQ ID NO:10176 | SEQ ID NO:18188 |
| iPS:435369 | **21-225_149A2** | NA | AAGTCCAGCCAGAGTGTTTTATACAGTCCCAACAATAACAACTACTTAGCT | TGGGCATCTACCCGGGAATCC | CAGCAATATTATAGTACTCCGTGCAGT |
| | | | SEQ ID NO:2165 | SEQ ID NO:10177 | SEQ ID NO:18189 |
| | | AA | KSSQSVLYSPNNNYLA | WASTRES | QQYYSTPCS |
| | | | SEQ ID NO:2166 | SEQ ID NO:10178 | SEQ ID NO:18190 |
| iPS:435371 | **21-225_149A3** | NA | CGGGCAAGTCAGAGCATTAGCAGTTATTTAAAT | ACTGCATCCAGTTTGCAAAGT | CAACAGAGTTACAGTATCCCCACT |
| | | | SEQ ID NO:2167 | SEQ ID NO:10179 | SEQ ID NO:18191 |
| | | AA | RASQSISSYLN | TASSLQS | QQSYSIPT |
| | | | SEQ ID NO:2168 | SEQ ID NO:10180 | SEQ ID NO:18192 |
| iPS:435373 | **21-225_149E3** | NA | AAGTCCAGCCAGACTGTTTTACACAACTCCAATAATCACAATTACTTTGCT | TGGGCATCTACCCTGAGATCC | CAGCAATATTATAGTACTCCTCCGACG |
| | | | SEQ ID NO:2169 | SEQ ID NO:10181 | SEQ ID NO:18193 |
| | | AA | KSSQTVLHNSNHNYFA | WASTLRS | QQYYSTPPT |
| | | | SEQ ID NO:2170 | SEQ ID NO:10182 | SEQ ID NO:18194 |
| iPS:435375 | **21-225_149H4** | NA | AAGTCCAGCCAGAGTGTTTTATCCAGCTCCAACGATAACAACTACTTAGCT | TGGTCATCTACCCGGGAATCC | CACCAATATTATAGTTATCCTCCGACG |
| | | | SEQ ID NO:2171 | SEQ ID NO:10183 | SEQ ID NO:18195 |
| | | AA | KSSQSVLSSSNDNNYLA | WSSTRES | HQYYSYPPT |

EP 4 435 105 A2

FIGURE 49

| | | | SEQ ID NO:2172 | SEQ ID NO:10184 | SEQ ID NO:18196 |
|---|---|---|---|---|---|
| iPS:435377 | | NA | CGGGCAAGTCAGGGCATTAG AAATGCCTTAGGC | GCTGCATCCAGTTTGCAA AGT | CTACAGCATAATAGTTACCC TCTCACT |
| | 21-225_149G5 | | SEQ ID NO:2173 | SEQ ID NO:10185 | SEQ ID NO:18197 |
| | | AA | RASQGIRNALG | AASSLQS | LQHNSYPLT |
| iPS:435379 | | NA | SEQ ID NO:2174 | SEQ ID NO:10186 | SEQ ID NO:18198 |
| | | | CGGGCGAGTCAGGGTATTAT CAGTTGGTTAGCC | GCTGCATCCAGTTTGCAA AGT | CAACAGGGTAACAGTTTCCC ATTCACT |
| | 21-225_149B6 | | SEQ ID NO:2175 | SEQ ID NO:10187 | SEQ ID NO:18199 |
| | | AA | RASQGIISWLA | AASSLQS | QQGNSFPFT |
| iPS:435381 | | NA | SEQ ID NO:2176 | SEQ ID NO:10188 | SEQ ID NO:18200 |
| | | | CGGGCGAGTCAGGGTATTAG CAACTGGTTAGCC | GCTGCATCCAGTTTGCAA GGT | CAACAGACTGACAGTTTCCC ATTCACT |
| | 21-225_149C6 | | SEQ ID NO:2177 | SEQ ID NO:10189 | SEQ ID NO:18201 |
| | | AA | RASQGISNWLA | AASSLQG | QQTDSFPFT |
| iPS:435383 | | NA | SEQ ID NO:2178 | SEQ ID NO:10190 | SEQ ID NO:18202 |
| | | | AGGGCCAGTCAGAGTATTAT CAGCAACTACTTAGCC | GGTGTATCTAGTAGGGC CACT | CAACAATATGGTCGGTCACC ATTCAAT |
| | 21-225_149D7 | | SEQ ID NO:2179 | SEQ ID NO:10191 | SEQ ID NO:18203 |
| | | AA | RASQSIISNYLA | GVSSRAT | QQYGRSPFN |
| iPS:435391 | | NA | SEQ ID NO:2180 | SEQ ID NO:10192 | SEQ ID NO:18204 |
| | | | CGGGCGAGTCAGGGTATTAG CAACTGGTTAGCC | GCTGCATCCAGTTTGCAA AGT | CAACAGACTGACAGTTTCCC ATTCACT |
| | 21-225_149F8 | | SEQ ID NO:2181 | SEQ ID NO:10193 | SEQ ID NO:18205 |
| | | AA | RASQGISNWLA | AASSLQS | QQTDSFPFT |
| iPS:435393 | | NA | SEQ ID NO:2182 | SEQ ID NO:10194 | SEQ ID NO:18206 |
| | | | CGGGCAAGTCGGGGCATTAG AAATGATTTAGGC | GCTGCATCCAGTTTGCAG AGT | CTACAGCATTATAGTTATCC TCGGACG |
| | 21-225_149D10 | | SEQ ID NO:2183 | SEQ ID NO:10195 | SEQ ID NO:18207 |
| | | AA | RASRGIRNDLG | AASSLQS | LQHYSYPRT |

763

FIGURE 49

| | | | SEQ ID NO:2184 | SEQ ID NO:10196 | SEQ ID NO:18208 |
|---|---|---|---|---|---|
| iPS:435395 | | NA | CGGGCGAGTCAGAATATTAG CAACTGGTTAACC | GCTGCATCCAGTTTGCAA AGT | CAACAGACTGACAGTTTCCC ATTCACT |
| | 21-225_149D11 | | SEQ ID NO:2185 | SEQ ID NO:10197 | SEQ ID NO:18209 |
| | | AA | RASQNISNWLT | AASSLQS | QQTDSFPFT |
| | | | SEQ ID NO:2186 | SEQ ID NO:10198 | SEQ ID NO:18210 |
| iPS:435397 | | NA | CGGGCAAGTCAGGGCATTAG AAATGATTTAGGC | GCTGCATCCAATTTGCAA AGT | CTACAGCATAATAGTTACCC GCTCACT |
| | 21-225_149F12 | | SEQ ID NO:2187 | SEQ ID NO:10199 | SEQ ID NO:18211 |
| | | AA | RASQGIRNDLG | AASNLQS | LQHNSYPLT |
| | | | SEQ ID NO:2188 | SEQ ID NO:10200 | SEQ ID NO:18212 |
| iPS:435399 | | NA | AAGTCCAGCCAGAGTGTTTT ATACAGATCCAACAGTAAGA AATACTTAACT | TGGGCATCTACCCGGAA ATCC | CAGCAATATTTTAGTACTCC GTACAAT |
| | 21-225_150D2 | | SEQ ID NO:2189 | SEQ ID NO:10201 | SEQ ID NO:18213 |
| | | AA | KSSQSVLYRSNSKKYLT | WASTRKS | QQYFSTPYN |
| | | | SEQ ID NO:2190 | SEQ ID NO:10202 | SEQ ID NO:18214 |
| iPS:435401 | | NA | CGGGCAAGTCAGGGCATTGG AAATGATTTAGGC | GCTGCATCCAGTTTGCAA AGT | CTACAACATTATAGTTTCCC GTACAGT |
| | 21-225_150E2 | | SEQ ID NO:2191 | SEQ ID NO:10203 | SEQ ID NO:18215 |
| | | AA | RASQGIGNDLG | AASSLQS | LQHYSFPYS |
| | | | SEQ ID NO:2192 | SEQ ID NO:10204 | SEQ ID NO:18216 |
| iPS:435403 | | NA | CGGGCAAGTCAGGGTATTAA CAACTGGTTAGCC | GCTGCATCCAGTTTGCAA GGT | CAACAGACTGACAGTTTCCC ATTCACT |
| | 21-225_150C5 | | SEQ ID NO:2193 | SEQ ID NO:10205 | SEQ ID NO:18217 |
| | | AA | RASQGINNWLA | AASSLQG | QQTDSFPFT |
| | | | SEQ ID NO:2194 | SEQ ID NO:10206 | SEQ ID NO:18218 |
| iPS:435405 | 21_225_150B7 | NA | AAGTCCAGCCAGAATGTTTT ATACAGCTCCCACAATAACA ACTACTTAGCT | TGGGCATCTACCCGGAA ATCC | CAGCAATATTATAGTACTCC ATTCACT |

FIGURE 49

| | | | | | |
|---|---|---|---|---|---|
| | 21-225_150B7 | | SEQ ID NO:2195 | SEQ ID NO:10207 | SEQ ID NO:18219 |
| | | AA | KSSQNVLYSSHNNNYLA | WASTRKS | QQYYSTPFT |
| | | | SEQ ID NO:2196 | SEQ ID NO:10208 | SEQ ID NO:18220 |
| iPS:435407 | 21-225_150E7 | NA | CGGGCAAGTCAGGGCATTAGAAATGATTTAGGC | GCTGCATCCAATTTGCAAAGT | CTACAGCATAATAGTTACCCGCTCACT |
| | | | SEQ ID NO:2197 | SEQ ID NO:10209 | SEQ ID NO:18221 |
| | | AA | RASQGIRNDLG | AASNLQS | LQHNSYPLT |
| | | | SEQ ID NO:2198 | SEQ ID NO:10210 | SEQ ID NO:18222 |
| iPS:435409 | 21-225_150G8 | NA | CGGGCGAGTCAGGGCATTAGCCATTATTTAGCC | GTTGCATCCAGTTTGCAAAAT | CAACAGTATAATAATTACCCGCTCACT |
| | | | SEQ ID NO:2199 | SEQ ID NO:10211 | SEQ ID NO:18223 |
| | | AA | RASQGISHYLA | VASSLQN | QQYNNYPLT |
| | | | SEQ ID NO:2200 | SEQ ID NO:10212 | SEQ ID NO:18224 |
| iPS:435413 | 21-225_150B11 | NA | AAGTCTAGTCAGAGCCTCGTGCATGGTGATGGAAAGACCTATTTGTAT | GAAGTTTCCAACCGGTTCTCT | ATGCAAAGTATACAGCTTCCGTGGACG |
| | | | SEQ ID NO:2201 | SEQ ID NO:10213 | SEQ ID NO:18225 |
| | | AA | KSSQSLVHGDGKTYLY | EVSNRFS | MQSIQLPWT |
| | | | SEQ ID NO:2202 | SEQ ID NO:10214 | SEQ ID NO:18226 |
| iPS:435415 | 21-225_150C11 | NA | CGGGCAAGTCAGAGCATTAGCAGCTATTTAAAT | ACTGCATCCAGTTTGCAAAGT | CAACAGAGTTACAGTATTTACACT |
| | | | SEQ ID NO:2203 | SEQ ID NO:10215 | SEQ ID NO:18227 |
| | | AA | RASQSISSYLN | TASSLQS | QQSYSIYT |
| | | | SEQ ID NO:2204 | SEQ ID NO:10216 | SEQ ID NO:18228 |
| iPS:435417 | 21-225_150D11 | NA | AAGTCTAGTCAGAGCCTCCTGCATAGTGAGGGAAAGACCTATTTGTAT | GAAGTTTCCTACCGGTTCTCT | ATGCAAGGTATACAGCTTCCGCTCACT |
| | | | SEQ ID NO:2205 | SEQ ID NO:10217 | SEQ ID NO:18229 |
| | | AA | KSSQSLLHSEGKTYLY | EVSYRFS | MQGIQLPLT |
| | | | SEQ ID NO:2206 | SEQ ID NO:10218 | SEQ ID NO:18230 |

FIGURE 49

| iPS:435419 | | NA | CGGGCAAGTCAGAGCATTAG CGACTATTTAAAT | ACTACATCCAGTTTGCAA AGT | CAACAGAGTTTCAGTACCCC CACT |
|---|---|---|---|---|---|
| | 21-225_150C12 | | SEQ ID NO:2207 | SEQ ID NO:10219 | SEQ ID NO:18231 |
| | | AA | RASQSISDYLN | TTSSLQS | QQSFSTPT |
| | | | SEQ ID NO:2208 | SEQ ID NO:10220 | SEQ ID NO:18232 |
| iPS:435421 | | NA | AGGGCCAGTCAGAGTATTAA CATCAATATAGCC | GGTGCATCCACCAGGGC CACT | CAGCAGTATAATGACTGGCC TCCGTGGACG |
| | 21-225_151F1 | | SEQ ID NO:2209 | SEQ ID NO:10221 | SEQ ID NO:18233 |
| | | AA | RASQSININIA | GASTRAT | QQYNDWPPWT |
| | | | SEQ ID NO:2210 | SEQ ID NO:10222 | SEQ ID NO:18234 |
| iPS:435423 | | NA | AAGTCTAGTCAGCGCCTCCT GCATGGTGATGGAAAGACCT ATTTGTAT | GAAGTTTCCAACCGGTTC TCT | ATGCAAAGTATACAGGTTCC GTGGACG |
| | 21-225_151G5 | | SEQ ID NO:2211 | SEQ ID NO:10223 | SEQ ID NO:18235 |
| | | AA | KSSQRLLHGDGKTYLY | EVSNRFS | MQSIQVPWT |
| | | | SEQ ID NO:2212 | SEQ ID NO:10224 | SEQ ID NO:18236 |
| iPS:435425 | | NA | CGGGCAAGTCAGAGCATTAG CAACTTTTTAAAT | ACTGCATCCAGTTTGGA AAGT | CAACAGAGTTACAGTACCCC CACT |
| | 21-225_151B12 | | SEQ ID NO:2213 | SEQ ID NO:10225 | SEQ ID NO:18237 |
| | | AA | RASQSISNFLN | TASSLES | QQSYSTPT |
| | | | SEQ ID NO:2214 | SEQ ID NO:10226 | SEQ ID NO:18238 |
| iPS:435427 | | NA | CGGGCGAGTCAGGGCATTAG CAAGTATTTAGCC | GATGCATCCAGGTTGCA AAGT | CATCAGTATAAACATTACCC GATCACC |
| | 21-225_151C9 | | SEQ ID NO:2215 | SEQ ID NO:10227 | SEQ ID NO:18239 |
| | | AA | RASQGISKYLA | DASRLQS | HQYKHYPIT |
| | | | SEQ ID NO:2216 | SEQ ID NO:10228 | SEQ ID NO:18240 |
| iPS:435429 | | NA | AAGTCTAGTCAGAGCCTCCT GCATGGTGATGGAAAGACCT ATTTGTAT | GAAGTTTCCCACCGGTTC TCT | ATGCAAAGTATACAGATTCC GTGGACG |
| | 21-225_151A10 | | SEQ ID NO:2217 | SEQ ID NO:10229 | SEQ ID NO:18241 |

FIGURE 49

| | | | AA | KSSQSLLHGDGKTYLY | EVSHRFS | MQSIQIPWT |
|---|---|---|---|---|---|---|
| | | | | SEQ ID NO:2218 | SEQ ID NO:10230 | SEQ ID NO:18242 |
| iPS:435431 | | NA | | CGGGCAAGTCAGAGCATTAGCGACTATTTAAAT | ACTACATCCAGTTTGCAAAGT | CAACAGAGTTACAGTACCCCCACT |
| | 21-225_152D2 | | | SEQ ID NO:2219 | SEQ ID NO:10231 | SEQ ID NO:18243 |
| | | AA | | RASQSISDYLN | TTSSLQS | QQSYSTPT |
| | | | | SEQ ID NO:2220 | SEQ ID NO:10232 | SEQ ID NO:18244 |
| iPS:435433 | | NA | | AAGTCCAGCCAGAGTGTTTTACACAGCTCCAACAATTACAACTACTTAGTT | TGGGCATCTACCCGGGAATCC | CAGCAATATTACAGTACTCCATTCACT |
| | 21-225_152E3 | | | SEQ ID NO:2221 | SEQ ID NO:10233 | SEQ ID NO:18245 |
| | | AA | | KSSQSVLHSSNNYNYLV | WASTRES | QQYYSTPFT |
| | | | | SEQ ID NO:2222 | SEQ ID NO:10234 | SEQ ID NO:18246 |
| iPS:435435 | | NA | | AAGTCCAGCCAGAGTGTTTTGCACAGCTCCAACAATTACAACTACTTAACT | TGGGCATCTACCCGGAATCC | CAGCAATATTATAGTACTCCGTGCAGT |
| | 21-225_152H3 | | | SEQ ID NO:2223 | SEQ ID NO:10235 | SEQ ID NO:18247 |
| | | AA | | KSSQSVLHSSNNYNYLT | WASTRKS | QQYYSTPCS |
| | | | | SEQ ID NO:2224 | SEQ ID NO:10236 | SEQ ID NO:18248 |
| iPS:435437 | | NA | | AAGTCCAGCCAGAGTGTTTTATACAGCTCCAACAATTACAACTACTTAGCT | TGGGCATCTACCCGGGAATCC | CAGCAATATTTTAATACTCCTCCCACT |
| | 21-225_152F4 | | | SEQ ID NO:2225 | SEQ ID NO:10237 | SEQ ID NO:18249 |
| | | AA | | KSSQSVLYSSNNYNYLA | WASTRKS | QQYFNTPPT |
| | | | | SEQ ID NO:2226 | SEQ ID NO:10238 | SEQ ID NO:18250 |
| iPS:435439 | | NA | | CGGGCAAGTCAGAGCATTAGCGACTATTTAAAT | ACTACATCCAGTTTGCAAAGT | CAACAGAGTTACAGTACCCCCACT |
| | 21-225_152G4 | | | SEQ ID NO:2227 | SEQ ID NO:10239 | SEQ ID NO:18251 |
| | | AA | | RASQSISDYLN | TTSSLQS | QQSYSTPT |
| | | | | SEQ ID NO:2228 | SEQ ID NO:10240 | SEQ ID NO:18252 |

FIGURE 49

| iPS:435441 | | NA | AAGTCTAGTCAGAGCCTCCG GCATGGTGATGGAAAGACCT ATTTGACT | GAAATTTCCAAGCGGTT CACT | ATGCAAAGTATACAGGTTCC GTGGACG |
|---|---|---|---|---|---|
| | 21-225_152F6 | | SEQ ID NO:2229 | SEQ ID NO:10241 | SEQ ID NO:18253 |
| | | AA | KSSQSLRHGDGKTYLT | EISKRFT | MQSIQVPWT |
| | | | SEQ ID NO:2230 | SEQ ID NO:10242 | SEQ ID NO:18254 |
| iPS:435443 | | NA | AGGGCCAGTCAGAGTGTTAT CAGCAGCTACTTAGCC | GGTGTATCTAGTAGGGC CACT | CAACAATATGGTAGGTCACC ATTCAAT |
| | 21-225_152E7 | | SEQ ID NO:2231 | SEQ ID NO:10243 | SEQ ID NO:18255 |
| | | AA | RASQSVISSYLA | GVSSRAT | QQYGRSPFN |
| | | | SEQ ID NO:2232 | SEQ ID NO:10244 | SEQ ID NO:18256 |
| iPS:435445 | | NA | CGGGCAAGTCAGGGCATTAG AAATGATTTAGGC | GCTACATCCAGTTTGCAA AGT | CTACAGCATTATAATTTCCC GTACAGT |
| | 21-225_152F7 | | SEQ ID NO:2233 | SEQ ID NO:10245 | SEQ ID NO:18257 |
| | | AA | RASQGIRNDLG | ATSSLQS | LQHYNFPYS |
| | | | SEQ ID NO:2234 | SEQ ID NO:10246 | SEQ ID NO:18258 |
| iPS:435447 | | NA | CGGGCGAGTCAGGATATTAG CAACTGGTTAGCC | GCTGCATCCAGTTTGCAA GGT | CAACAGACTGACAGTTTCCC ATTCACT |
| | 21-225_152H7 | | SEQ ID NO:2235 | SEQ ID NO:10247 | SEQ ID NO:18259 |
| | | AA | RASQDISNWLA | AASSLQG | QQTDSFPFT |
| | | | SEQ ID NO:2236 | SEQ ID NO:10248 | SEQ ID NO:18260 |
| iPS:435449 | | NA | CGGGCAAGTCAGGGCATTAG AAATGATTTAGGC | GCTGCATCCAGTTTGCAA AGT | CTACAGCATAGTAATTACCC GCTCACT |
| | 21-225_152H9 | | SEQ ID NO:2237 | SEQ ID NO:10249 | SEQ ID NO:18261 |
| | | AA | RASQGIRNDLG | AASSLQS | LQHSNYPLT |
| | | | SEQ ID NO:2238 | SEQ ID NO:10250 | SEQ ID NO:18262 |
| iPS:435451 | | NA | AAGTCCAGCCAGAGTGTTTT ATACAGCTCCAACAATAAGA ACTACTTAGCT | TGGGCATCTACCCGGGA ATCC | CAGCAATATTATCGTAGTCC TAGT |
| | 21-225_152D10 | | SEQ ID NO:2239 | SEQ ID NO:10251 | SEQ ID NO:18263 |

FIGURE 49

| | | AA | KSSQSVLYSSNNKNYLA | WASTRES | QQYYRSPS |
|---|---|---|---|---|---|
| | | | SEQ ID NO:2240 | SEQ ID NO:10252 | SEQ ID NO:18264 |
| iPS:435453 | | NA | CGGGCGAGTCAGGGTATTAG CAACTGGTTAGCC | GCTGCATCCAGTTTGCAA AGT | CAACAGACTGACAGTTTCCC ATTCACT |
| | 21-225_152G10 | | SEQ ID NO:2241 | SEQ ID NO:10253 | SEQ ID NO:18265 |
| | | AA | RASQGISNWLA | AASSLQS | QQTDSFPFT |
| | | | SEQ ID NO:2242 | SEQ ID NO:10254 | SEQ ID NO:18266 |
| iPS:435455 | | NA | CGGGCAAGTCAGAGCATTAG CGACTATTTAAAT | ACTACATCCAGTTTGCAA AGT | CAACAGAGTTACAGTACCCC CACT |
| | 21-225_152B11 | | SEQ ID NO:2243 | SEQ ID NO:10255 | SEQ ID NO:18267 |
| | | AA | RASQSISDYLN | TTSSLQS | QQSYSTPT |
| | | | SEQ ID NO:2244 | SEQ ID NO:10256 | SEQ ID NO:18268 |
| iPS:435457 | | NA | AAGTCTAGTCAGAGCCTCCT GCATGGTGATGGAAAGACCT ATTTATAT | GAAGTTTCCAACCGGTTC TCT | ATGCAAAGTATACAGATTCC GTGGACG |
| | 21-225_152C11 | | SEQ ID NO:2245 | SEQ ID NO:10257 | SEQ ID NO:18269 |
| | | AA | KSSQSLLHGDGKTYLY | EVSNRFS | MQSIQIPWT |
| | | | SEQ ID NO:2246 | SEQ ID NO:10258 | SEQ ID NO:18270 |
| iPS:435459 | | NA | ACGTCCAGCCAGAGTATTTT ACACAGCTCCAATAATTACA ACTACTTAGCT | TGGGCATCTACCCGGGA ATCC | CAACAATATTATAGTGGTCC GTGCAGT |
| | 21-225_152E12 | | SEQ ID NO:2247 | SEQ ID NO:10259 | SEQ ID NO:18271 |
| | | AA | TSSQSILHSSNNYNYLA | WASTRES | QQYYSGPCS |
| | | | SEQ ID NO:2248 | SEQ ID NO:10260 | SEQ ID NO:18272 |
| iPS:435461 | | NA | CGGGCGAGTCAGGTCATTAG CAATTATTTAGCC | GCTGCATCCAGTTTGCGA AGT | CAACAGTATCATAGTTACCC ATTCACT |
| | 21-225_153A1 | | SEQ ID NO:2249 | SEQ ID NO:10261 | SEQ ID NO:18273 |
| | | AA | RASQVISNYLA | AASSLRS | QQYHSYPFT |
| | | | SEQ ID NO:2250 | SEQ ID NO:10262 | SEQ ID NO:18274 |

FIGURE 49

| iPS:435463 | | NA | AAGTCTAGTCAGAGCCTCCG GCATGGTGATGGAAAGACCT ATTTGACT | GAAGTTTCCAAGCGGTT CACT | ATGCAAAGTATACAGGTTCC GTGGACG |
|---|---|---|---|---|---|
| | 21-225_153D2 | | SEQ ID NO:2251 | SEQ ID NO:10263 | SEQ ID NO:18275 |
| | | AA | KSSQSLRHGDGKTYLT | EVSKRFT | MQSIQVPWT |
| | | | SEQ ID NO:2252 | SEQ ID NO:10264 | SEQ ID NO:18276 |
| iPS:435465 | | NA | AGGGCCAGTCAGAGTGTTAT CAGCAGCTACTTAGCC | GGTGTATCTAGTAGGGC CACT | CAACAATATGGTAGGTCACC ATTCAAT |
| | 21-225_153A6 | | SEQ ID NO:2253 | SEQ ID NO:10265 | SEQ ID NO:18277 |
| | | AA | RASQSVISSYLA | GVSSRAT | QQYGRSPFN |
| | | | SEQ ID NO:2254 | SEQ ID NO:10266 | SEQ ID NO:18278 |
| iPS:435467 | | NA | AAGTCCAGCCAGAGTGTTTT ATACAGCTCCAACAATAAGA ACTACTTAGCT | TGGGCATCTACCCGGGA ATTT | CAGCAATATAATCGTAGTCT TAGT |
| | 21-225_153B9 | | SEQ ID NO:2255 | SEQ ID NO:10267 | SEQ ID NO:18279 |
| | | AA | KSSQSVLYSSNNKNYLA | WASTREF | QQYNRSLS |
| | | | SEQ ID NO:2256 | SEQ ID NO:10268 | SEQ ID NO:18280 |
| iPS:435469 | | NA | AAGTCTAGTCAGAGCCTCCT GCATAGTGATGGAAAGACCT ATTTGTAT | GAAGTTTCCAACCGGTTC TCT | ATGCAAAATATAAAGTATCC GCTCACT |
| | 21-225_153G9 | | SEQ ID NO:2257 | SEQ ID NO:10269 | SEQ ID NO:18281 |
| | | AA | KSSQSLLHSDGKTYLY | EVSNRFS | MQNIKYPLT |
| | | | SEQ ID NO:2258 | SEQ ID NO:10270 | SEQ ID NO:18282 |
| iPS:435471 | | NA | AAGTCCAGCCAGAGTGTTTT ATACAGCTCCAACAATTACA AGTACTTAGCT | TGGGCATCTACCCGGAA ATCC | CAGCAATATTATAGTACTCC GTGCAGT |
| | 21-225_153F11 | | SEQ ID NO:2259 | SEQ ID NO:10271 | SEQ ID NO:18283 |
| | | AA | KSSQSVLYSSNNYKYLA | WASTRKS | QQYYSTPCS |
| | | | SEQ ID NO:2260 | SEQ ID NO:10272 | SEQ ID NO:18284 |

FIGURE 49

| iPS:435475 | | NA | AAGTCCAGCCAGAGTGTTTT ACACAGTTCCAACAATTACA ACTATTTAGCT | TGGACATCTACCCGGAA ATCC | CAGCATTATTATAGTACTCC GTGCAGT |
|---|---|---|---|---|---|
| | 21-225_154H6 | | SEQ ID NO:2261 | SEQ ID NO:10273 | SEQ ID NO:18285 |
| | | AA | KSSQSVLHSSNNYNYLA | WTSTRKS | QHYYSTPCS |
| | | | SEQ ID NO:2262 | SEQ ID NO:10274 | SEQ ID NO:18286 |
| iPS:435479 | | NA | CGGGCGAGTCAGGATATTAG CAACTGGTTAGCC | GCTGCATCCAGTTTGCAA AGT | CAACAGGGTAACAGTTTCCC GCTCACT |
| | 21-225_154E9 | | SEQ ID NO:2263 | SEQ ID NO:10275 | SEQ ID NO:18287 |
| | | AA | RASQDISNWLA | AASSLQS | QQGNSFPLT |
| | | | SEQ ID NO:2264 | SEQ ID NO:10276 | SEQ ID NO:18288 |
| iPS:435481 | | NA | AGGTCAAGCCAGAGTGTTTT ACACAGCTCCAACAATTATA ACTACTTAGCT | TGGGCATCTAAACGGGA TTCC | CAGCAATATTTTAGTTCTCCT CGGACG |
| | 21-225_154A11 | | SEQ ID NO:2265 | SEQ ID NO:10277 | SEQ ID NO:18289 |
| | | AA | RSSQSVLHSSNNYNYLA | WASKRDS | QQYFSSPRT |
| | | | SEQ ID NO:2266 | SEQ ID NO:10278 | SEQ ID NO:18290 |
| iPS:435483 | | NA | CGGGCGAGTCAGGGTATTAG CAACTGGTTAGCC | GCTGCATCCAGTTTGCAA GGT | CACCAGACTGACAGTTTCCC ATTCACT |
| | 21-225_155A4 | | SEQ ID NO:2267 | SEQ ID NO:10279 | SEQ ID NO:18291 |
| | | AA | RASQGISNWLA | AASSLQG | HQTDSFPFT |
| | | | SEQ ID NO:2268 | SEQ ID NO:10280 | SEQ ID NO:18292 |
| iPS:435485 | | NA | CGGGCGAGTCAGGATATTAG CAACTGGTTAGCC | GCTGCATCCAGTTTGCAA GGT | CACCAGACTGACAGTTTCCC ATTCACT |
| | 21-225_155B4 | | SEQ ID NO:2269 | SEQ ID NO:10281 | SEQ ID NO:18293 |
| | | AA | RASQDISNWLA | AASSLQG | HQTDSFPFT |
| | | | SEQ ID NO:2270 | SEQ ID NO:10282 | SEQ ID NO:18294 |
| iPS:435487 | | NA | CGGGCAAGTCAGAGCATTAG CAGCTATTTAAAT | ACTGCATCCAGTTTGCAA AGT | CAACAGAGTTACAGTACCCC CACT |
| | 21-225_155C4 | | SEQ ID NO:2271 | SEQ ID NO:10283 | SEQ ID NO:18295 |

FIGURE 49

| | | AA | RASQSISSYLN | TASSLQS | QQSYSTPT |
|---|---|---|---|---|---|
| | | | SEQ ID NO:2272 | SEQ ID NO:10284 | SEQ ID NO:18296 |
| iPS:435489 | 21-225_155A5 | NA | AAGTCTAGTCAGAGCCTCCTGCATGGTGATGGAAAGACCTATTTGTAT | GAAGTTTCCAATCGGTTCTCT | ATGCAAAGTATACAGGTTCCGTGGACG |
| | | | SEQ ID NO:2273 | SEQ ID NO:10285 | SEQ ID NO:18297 |
| | | AA | KSSQSLLHGDGKTYLY | EVSNRFS | MQSIQVPWT |
| | | | SEQ ID NO:2274 | SEQ ID NO:10286 | SEQ ID NO:18298 |
| iPS:435491 | 21-225_155E5 | NA | AAGTCCAGCCAGAGTGTTTTATCCAGCTCCAACAATAATAATTATTTAGCT | TGGGCATCTACCCGGAAATCC | CAGCAATATTATAGTACTCCGTGCAGT |
| | | | SEQ ID NO:2275 | SEQ ID NO:10287 | SEQ ID NO:18299 |
| | | AA | KSSQSVLSSSNNNNYLA | WASTRKS | QQYYSTPCS |
| | | | SEQ ID NO:2276 | SEQ ID NO:10288 | SEQ ID NO:18300 |
| iPS:435495 | 21-225_155B6 | NA | AAGTCCAGCCAGAGTGTTTTACACAGCTCCAATAATTACAACTACTTAGCT | TGGACATCTACCCGGGAATCC | CAACAATATTATAGTACTCCGTGCAGT |
| | | | SEQ ID NO:2277 | SEQ ID NO:10289 | SEQ ID NO:18301 |
| | | AA | KSSQSVLHSSNNYNYLA | WTSTRES | QQYYSTPCS |
| | | | SEQ ID NO:2278 | SEQ ID NO:10290 | SEQ ID NO:18302 |
| iPS:435497 | 21-225_155H9 | NA | AGGGCCAGTCAGAGTGTTAGTAGTAACTTAGCC | GGTGCATCCACCAGGGCCACT | CAGCAGTATGATGACTGGCCTCCGTGGACG |
| | | | SEQ ID NO:2279 | SEQ ID NO:10291 | SEQ ID NO:18303 |
| | | AA | RASQSVSSNLA | GASTRAT | QQYDDWPPWT |
| | | | SEQ ID NO:2280 | SEQ ID NO:10292 | SEQ ID NO:18304 |
| iPS:435499 | 21-225_156G1 | NA | CGGGCAAGTCAGGGCATTAGAAATGATTTAGGC | GCTGCATCCAGTTTGCAAAGT | CTACAGCATAGTAATTACCCGCTCACT |
| | | | SEQ ID NO:2281 | SEQ ID NO:10293 | SEQ ID NO:18305 |
| | | AA | RASQGIRNDLG | AASSLQS | LQHSNYPLT |
| | | | SEQ ID NO:2282 | SEQ ID NO:10294 | SEQ ID NO:18306 |

FIGURE 49

| iPS:435501 | | NA | AAGTCCAGCCAGAGTGTTTT ATACAGCTCCAACAATAACA ACTACTTAGCT | TGGGCATCTACCCGGGA ATCC | CACCAATATTATAGTACTCC GTGCAGT |
|---|---|---|---|---|---|
| | 21-225_156H1 | | SEQ ID NO:2283 | SEQ ID NO:10295 | SEQ ID NO:18307 |
| | | AA | KSSQSVLYSSNNNNYLA | WASTRES | HQYYSTPCS |
| | | | SEQ ID NO:2284 | SEQ ID NO:10296 | SEQ ID NO:18308 |
| iPS:435503 | | NA | CGGGCAAGTCAGAGCATTAG CAGCTATTTAAAT | ACTGCATCCAGTTTGCAA AGT | CAACAGAGTTACAGTGCCCC CACT |
| | 21-225_156E4 | | SEQ ID NO:2285 | SEQ ID NO:10297 | SEQ ID NO:18309 |
| | | AA | RASQSISSYLN | TASSLQS | QQSYSAPT |
| | | | SEQ ID NO:2286 | SEQ ID NO:10298 | SEQ ID NO:18310 |
| iPS:435505 | | NA | CGGGCGAGTCAGGGCATTAG CAATTATTTAGCC | GCTGCATCCAGTTTGCTA AGT | CAACAGTATAATAGTTTTCC ATTCACT |
| | 21-225_157C1 | | SEQ ID NO:2287 | SEQ ID NO:10299 | SEQ ID NO:18311 |
| | | AA | RASQGISNYLA | AASSLLS | QQYNSFPFT |
| | | | SEQ ID NO:2288 | SEQ ID NO:10300 | SEQ ID NO:18312 |
| iPS:435509 | | NA | CGGGCGAGTCAGGACATTAG CAATTATTTAGTC | GCTGCATCCAGTTTGCAA AGT | CAACAATATCATAGTTACCC ATTCACT |
| | 21-225_157H1 | | SEQ ID NO:2289 | SEQ ID NO:10301 | SEQ ID NO:18313 |
| | | AA | RASQDISNYLV | AASSLQS | QQYHSYPFT |
| | | | SEQ ID NO:2290 | SEQ ID NO:10302 | SEQ ID NO:18314 |
| iPS:435511 | | NA | CGGGCAAGTCAGGGCATTAG AAATGATTTAGGC | GCTGCATCCAGTTTGCAA AGT | CTACAGCATAATAGTTACCC ATTCACT |
| | 21-225_157C3 | | SEQ ID NO:2291 | SEQ ID NO:10303 | SEQ ID NO:18315 |
| | | AA | RASQGIRNDLG | AASSLQS | LQHNSYPFT |
| | | | SEQ ID NO:2292 | SEQ ID NO:10304 | SEQ ID NO:18316 |
| iPS:435513 | | NA | CGGGCAAGTCAGAACATTAG CAGTTATTTAAAT | ACTGCATCCAGTTTGCAA AGT | CAACAGAGTTACAATACCCC CACGTGGACG |
| | 21-225_157F3 | | SEQ ID NO:2293 | SEQ ID NO:10305 | SEQ ID NO:18317 |
| | | AA | RASQNISSYLN | TASSLQS | QQSYNTPTWT |

773

FIGURE 49

| | | | SEQ ID NO:2294 | SEQ ID NO:10306 | SEQ ID NO:18318 |
|---|---|---|---|---|---|
| iPS:435515 | | NA | CGGGCGAGTCAGGGCATTAG CAATTATTTAGCC | GCTGCATCCAGTTTGCGA AGT | CAACAGTATCATAGTTATCC ATTCACT |
| | 21-225_157E4 | | SEQ ID NO:2295 | SEQ ID NO:10307 | SEQ ID NO:18319 |
| | | AA | RASQGISNYLA | AASSLRS | QQYHSYPFT |
| | | | SEQ ID NO:2296 | SEQ ID NO:10308 | SEQ ID NO:18320 |
| iPS:435521 | | NA | CGGGCAAGTCAGGGCATTAG AAATGATTTAGGC | CCTGCATCCAGTTTACAA ACT | CTACAGGATAATAGTTACCC ATTCACT |
| | 21-225_157H4 | | SEQ ID NO:2297 | SEQ ID NO:10309 | SEQ ID NO:18321 |
| | | AA | RASQGIRNDLG | PASSLQT | LQDNSYPFT |
| | | | SEQ ID NO:2298 | SEQ ID NO:10310 | SEQ ID NO:18322 |
| iPS:435523 | | NA | CGGGCGAGTCAGGGCATTAA CAATTATTTAGCC | GCTGCATCCAGTTTACAA AGT | CAACAGTATAATAGTTATCC ATTCACT |
| | 21-225_157G5 | | SEQ ID NO:2299 | SEQ ID NO:10311 | SEQ ID NO:18323 |
| | | AA | RASQGINNYLA | AASSLQS | QQYNSYPFT |
| | | | SEQ ID NO:2300 | SEQ ID NO:10312 | SEQ ID NO:18324 |
| iPS:435525 | | NA | CGGGCAAGTCAGAGCTTTAG CAGCTATTTAAAT | GCTGCATCCAGTTTGCAA AGT | CAAGAGAGTTATAGTATCCG CTTCGCC |
| | 21-225_157E7 | | SEQ ID NO:2301 | SEQ ID NO:10313 | SEQ ID NO:18325 |
| | | AA | RASQSFSSYLN | AASSLQS | QESYSIRFA |
| | | | SEQ ID NO:2302 | SEQ ID NO:10314 | SEQ ID NO:18326 |
| iPS:435527 | | NA | CGGGCAAGTCAGGGCATTAG AAATGATTTAGGC | GTTGCATCCAGTTTGCAA AGT | ATACAGGATAATAGTCACCC ATTCACT |
| | 21-225_157G7 | | SEQ ID NO:2303 | SEQ ID NO:10315 | SEQ ID NO:18327 |
| | | AA | RASQGIRNDLG | VASSLQS | IQDNSHPFT |
| | | | SEQ ID NO:2304 | SEQ ID NO:10316 | SEQ ID NO:18328 |
| iPS:435529 | | NA | CGGGCGAGTCAGGACATTAG CAATTTTTTAGCC | ACTGCATCCAGTTTGCAA AGT | CAACAGTATCATAGTTACCC GATCACC |
| | 21-225_157H7 | | SEQ ID NO:2305 | SEQ ID NO:10317 | SEQ ID NO:18329 |
| | | AA | RASQDISNFLA | TASSLQS | QQYHSYPIT |

FIGURE 49

| | | | SEQ ID NO:2306 | SEQ ID NO:10318 | SEQ ID NO:18330 |
|---|---|---|---|---|---|
| iPS:435531 | 21-225_157G8 | NA | AAGTCTAGTCAGAGCCTCCTGCATGGTGATGGAAAGACCTATTTATAT | GAAATTTCCAAGCGGTTCTCT | ATGCAAAGTATACAGGTTCCGTGGACG |
| | | | SEQ ID NO:2307 | SEQ ID NO:10319 | SEQ ID NO:18331 |
| | | AA | KSSQSLLHGDGKTYLY | EISKRFS | MQSIQVPWT |
| | | | SEQ ID NO:2308 | SEQ ID NO:10320 | SEQ ID NO:18332 |
| iPS:435533 | 21-225_157H8 | NA | CGGGCAAGTCAGGGCATTAGAAATGATTTAGGC | GCTGCATCCAGTTTGCAAAGT | CTACAGCATAATAGTTACCCATTCACT |
| | | | SEQ ID NO:2309 | SEQ ID NO:10321 | SEQ ID NO:18333 |
| | | AA | RASQGIRNDLG | AASSLQS | LQHNSYPFT |
| | | | SEQ ID NO:2310 | SEQ ID NO:10322 | SEQ ID NO:18334 |
| iPS:435535 | 21-225_157H10 | NA | CGGGCGAGTCAGGGCATTACCAATTATTTAGCC | ACTGCATCCAATTTGCAAAGT | CAACAGTATCATAGTTACCCATTCACT |
| | | | SEQ ID NO:2311 | SEQ ID NO:10323 | SEQ ID NO:18335 |
| | | AA | RASQGITNYLA | TASNLQS | QQYHSYPFT |
| | | | SEQ ID NO:2312 | SEQ ID NO:10324 | SEQ ID NO:18336 |
| iPS:435537 | 21-225_157H12 | NA | CGGGCAAGTCAGGGCATTAGAAATGATTTTGGC | GCTGCATCCAGTTTACAGAGT | CTACAGCATTATAGTTACCCATTCACT |
| | | | SEQ ID NO:2313 | SEQ ID NO:10325 | SEQ ID NO:18337 |
| | | AA | RASQGIRNDFG | AASSLQS | LQHYSYPFT |
| | | | SEQ ID NO:2314 | SEQ ID NO:10326 | SEQ ID NO:18338 |
| iPS:435539 | 21-225_158G1 | NA | CGGGCAAGTCAGGGCATTAGAAATGATTTAGGC | ACTGCATCCAATTTGCAAAGT | CTACAGCATAATAGTTACCCGTGGACG |
| | | | SEQ ID NO:2315 | SEQ ID NO:10327 | SEQ ID NO:18339 |
| | | AA | RASQGIRNDLG | TASNLQS | LQHNSYPWT |
| | | | SEQ ID NO:2316 | SEQ ID NO:10328 | SEQ ID NO:18340 |
| iPS:435543 | 21-225_158D4 | NA | CGGGCAAGTCAGGACATTAGAAAGGATTTAGGC | GCTGCATCCAGTTTGCAAAGT | CTACAGCATTATAGTTACCCTCGGACG |
| | | | SEQ ID NO:2317 | SEQ ID NO:10329 | SEQ ID NO:18341 |

FIGURE 49

| | | | | | |
|---|---|---|---|---|---|
| | | AA | RASQDIRKDLG | AASSLQS | LQHYSYPRT |
| | | | SEQ ID NO:2318 | SEQ ID NO:10330 | SEQ ID NO:18342 |
| iPS:435545 | | NA | CGGGCAAGTCAGAACATTAGAAAGTATTTACAT | ACTGCATCCACTTTACAAAGT | CAACAGAGTTACAATATTTCATTCACT |
| | 21-225_158F4 | | SEQ ID NO:2319 | SEQ ID NO:10331 | SEQ ID NO:18343 |
| | | AA | RASQNIRKYLH | TASTLQS | QQSYNISFT |
| | | | SEQ ID NO:2320 | SEQ ID NO:10332 | SEQ ID NO:18344 |
| iPS:435547 | | NA | CGGGCAAGTCAGGGCATTAGAAATGATTTAGGC | GCTGCATCCAGTTTGCAAAGT | CTACAGGATAATAGTCACCCATTCACT |
| | 21-225_158F5 | | SEQ ID NO:2321 | SEQ ID NO:10333 | SEQ ID NO:18345 |
| | | AA | RASQGIRNDLG | AASSLQS | LQDNSHPFT |
| | | | SEQ ID NO:2322 | SEQ ID NO:10334 | SEQ ID NO:18346 |
| iPS:435549 | | NA | CGGGCAAGTCAGGGCATGAGAATTGATTTAGGG | CGTGCATCCAGTTTGCAAAGT | GTACAGCATAATAGTTACCCTCTCACT |
| | 21-225_158H5 | | SEQ ID NO:2323 | SEQ ID NO:10335 | SEQ ID NO:18347 |
| | | AA | RASQGMRIDLG | RASSLQS | VQHNSYPLT |
| | | | SEQ ID NO:2324 | SEQ ID NO:10336 | SEQ ID NO:18348 |
| iPS:435551 | | NA | CGGGCAAGTCAGGGCATTAGAAGTGATTTAGGC | ACTGCATCCAGTTTGCAAAGT | CTACAGCATAATAGTTACCCATTCACT |
| | 21-225_158H6 | | SEQ ID NO:2325 | SEQ ID NO:10337 | SEQ ID NO:18349 |
| | | AA | RASQGIRSDLG | TASSLQS | LQHNSYPFT |
| | | | SEQ ID NO:2326 | SEQ ID NO:10338 | SEQ ID NO:18350 |
| iPS:435553 | | NA | CGGGCAAGTCAGGACATTAGAAATGATTTAGGC | ACTGCATCCAGTTTGCAAAGT | CTACAGCATAATAGTTACCCATTCACT |
| | 21-225_158G8 | | SEQ ID NO:2327 | SEQ ID NO:10339 | SEQ ID NO:18351 |
| | | AA | RASQDIRNDLG | TASSLQS | LQHNSYPFT |
| | | | SEQ ID NO:2328 | SEQ ID NO:10340 | SEQ ID NO:18352 |
| iPS:435557 | 21 225 158B12 | NA | AAGTCCAGCCAGAATGTTTTACACAGCTCCAACAATAACAACTACTTAACT | TGGGCATCTACCCGGGAATCC | CAGCAATATTATAGTACTCCTCCGACG |

FIGURE 49

| | 21-225_158B12 | | SEQ ID NO:2329 | SEQ ID NO:10341 | SEQ ID NO:18353 |
|---|---|---|---|---|---|
| | | AA | KSSQNVLHSSNNNNYLT | WASTRES | QQYYSTPPT |
| iPS:435559 | | NA | SEQ ID NO:2330 CGGGCGAGTCAGGGCATTAA CAATTATTTAGCC | SEQ ID NO:10342 GCTGCATCCAGTTTACAA AGT | SEQ ID NO:18354 CAACAGTATCATAGTTACCC ATTCACT |
| | 21-225_158H12 | | SEQ ID NO:2331 | SEQ ID NO:10343 | SEQ ID NO:18355 |
| | | AA | RASQGINNYLA | AASSLQS | QQYHSYPFT |
| iPS:435561 | | NA | SEQ ID NO:2332 CGGGCAAGTCAGCGCGTCAG AAATGATTTAGGC | SEQ ID NO:10344 GATGCATCCAATTTGCA AAGT | SEQ ID NO:18356 CTACAGCATCATAGTTTCCC GATCACC |
| | 21-225_159F1 | | SEQ ID NO:2333 | SEQ ID NO:10345 | SEQ ID NO:18357 |
| | | AA | RASQRVRNDLG | DASNLQS | LQHHSFPIT |
| iPS:435563 | | NA | SEQ ID NO:2334 CGGGCAAGTCAGAGCATTAG CAAATATTTAAAT | SEQ ID NO:10346 GCTACATCCAATTTGCAA AGT | SEQ ID NO:18358 CAACAGAGTTACAGTCTCCC GGTCACT |
| | 21-225_159H2 | | SEQ ID NO:2335 | SEQ ID NO:10347 | SEQ ID NO:18359 |
| | | AA | RASQSISKYLN | ATSNLQS | QQSYSLPVT |
| iPS:435565 | | NA | SEQ ID NO:2336 CAGGCGAGTCAGGACATTAG CGACTATTTAAAT | SEQ ID NO:10348 GATGCCTCCACTTTGGAA ACA | SEQ ID NO:18360 CAACAATATGATAATCTCCC GATCACC |
| | 21-225_159C4 | | SEQ ID NO:2337 | SEQ ID NO:10349 | SEQ ID NO:18361 |
| | | AA | QASQDISDYLN | DASTLET | QQYDNLPIT |
| iPS:435569 | | NA | SEQ ID NO:2338 CGGACAAGTCAGGGCATTAG AAATGATTTAGGC | SEQ ID NO:10350 GCTGCATCCAGTTTGCAA AGT | SEQ ID NO:18362 CTACAGCATAATAGTTATCC ATTCACT |
| | 21-225_159C5 | | SEQ ID NO:2339 | SEQ ID NO:10351 | SEQ ID NO:18363 |
| | | AA | RTSQGIRNDLG | AASSLQS | LQHNSYPFT |
| iPS:435571 | | NA | SEQ ID NO:2340 CGGGCAAGTCAGGACATTAG AAAGGATTTAGGG | SEQ ID NO:10352 GCTGCATCCAGTTTGCAA AGT | SEQ ID NO:18364 CTACAGCATCATAGTTATCC TCGGACG |

FIGURE 49

| | 21-225_159C8 | | SEQ ID NO:2341 | SEQ ID NO:10353 | SEQ ID NO:18365 |
|---|---|---|---|---|---|
| | | AA | RASQDIRKDLG | AASSLQS | LQHHSYPRT |
| | | | SEQ ID NO:2342 | SEQ ID NO:10354 | SEQ ID NO:18366 |
| iPS:435573 | 21-225_159D8 | NA | CGGGCAAGTCGGGACATTGGAAATGATTTAGGC | GCTGCATCCAGTTTGCAAAGT | CTACAGCATTATAGTTACCCTCGGACG |
| | | | SEQ ID NO:2343 | SEQ ID NO:10355 | SEQ ID NO:18367 |
| | | AA | RASRDIGNDLG | AASSLQS | LQHYSYPRT |
| | | | SEQ ID NO:2344 | SEQ ID NO:10356 | SEQ ID NO:18368 |
| iPS:435575 | 21-225_159H11 | NA | CGGGCGAGTCAGGGCATTAGCAAATATTTAGTC | GCTGCATCCAGTCTGCAAAGT | CAACAGTATAATAGTTACCCATTCACT |
| | | | SEQ ID NO:2345 | SEQ ID NO:10357 | SEQ ID NO:18369 |
| | | AA | RASQGISKYLV | AASSLQS | QQYNSYPFT |
| | | | SEQ ID NO:2346 | SEQ ID NO:10358 | SEQ ID NO:18370 |
| iPS:435577 | 21-225_160B1 | NA | AAGTCTAGTCAGAGCCTCCTGCATGGTGATGGGAAGACCTATTTCTAT | GAAGTATCCAAGCGGTTCTCT | ATGCAAAGTATACAGATTCCGTGGACG |
| | | | SEQ ID NO:2347 | SEQ ID NO:10359 | SEQ ID NO:18371 |
| | | AA | KSSQSLLHGDGKTYFY | EVSKRFS | MQSIQIPWT |
| | | | SEQ ID NO:2348 | SEQ ID NO:10360 | SEQ ID NO:18372 |
| iPS:435579 | 21-225_160G1 | NA | CGGGCGAGTCAGGACATTAACAATTATTTAGCC | GCTTCATCCAGTTTGCAAAGT | CAACAATATCATAGTTACCCATTCACT |
| | | | SEQ ID NO:2349 | SEQ ID NO:10361 | SEQ ID NO:18373 |
| | | AA | RASQDINNYLA | ASSSLQS | QQYHSYPFT |
| | | | SEQ ID NO:2350 | SEQ ID NO:10362 | SEQ ID NO:18374 |
| iPS:435581 | 21-225_160H1 | NA | CGGGCAAGTCAGGACATTAGAAATGATTTAGGC | GCTGCATCCAGTTTGCAGAAT | CTACAGCATAATAGTTTCCCGTGGACG |
| | | | SEQ ID NO:2351 | SEQ ID NO:10363 | SEQ ID NO:18375 |
| | | AA | RASQDIRNDLG | AASSLQN | LQHNSFPWT |
| | | | SEQ ID NO:2352 | SEQ ID NO:10364 | SEQ ID NO:18376 |

FIGURE 49

| iPS:435583 | | NA | CGGGCAAGTCAGGGCATTAG AAATGATTTAGGC | ACTGCATCCAATTTGCAA AGT | CTACAGCATAATAGTTACCC GTGGACG |
|---|---|---|---|---|---|
| | 21-225_160F2 | | SEQ ID NO:2353 | SEQ ID NO:10365 | SEQ ID NO:18377 |
| | | AA | RASQGIRNDLG | TASNLQS | LQHNSYPWT |
| | | | SEQ ID NO:2354 | SEQ ID NO:10366 | SEQ ID NO:18378 |
| iPS:435585 | | NA | CGGGCGAGTCAGGACATTAA CAATTATTTAGCC | GCTTCATCCAGTTTGCAA AGT | CAACAATATCATAGTTACCC ATTCACT |
| | 21-225_160G3 | | SEQ ID NO:2355 | SEQ ID NO:10367 | SEQ ID NO:18379 |
| | | AA | RASQDINNYLA | ASSSLQS | QQYHSYPFT |
| | | | SEQ ID NO:2356 | SEQ ID NO:10368 | SEQ ID NO:18380 |
| iPS:435587 | | NA | CGGGCAAGTCAGGGCATTAG AAATGATTTAGGC | GCTGCATCCAGTTTGCAA AGT | CTACAGCATAGTAATTACCC GCTCACT |
| | 21-225_160H3 | | SEQ ID NO:2357 | SEQ ID NO:10369 | SEQ ID NO:18381 |
| | | AA | RASQGIRNDLG | AASSLQS | LQHSNYPLT |
| | | | SEQ ID NO:2358 | SEQ ID NO:10370 | SEQ ID NO:18382 |
| iPS:435589 | | NA | AAGTCCAGCCAGAGTGTTTT ACACAGCTCCAACAATAATA ACTACTTAGCT | TGGGCATCTACCCGGGA ATCC | CAGCAATATTATAATAGTCC GTGCAGT |
| | 21-225_160A4 | | SEQ ID NO:2359 | SEQ ID NO:10371 | SEQ ID NO:18383 |
| | | AA | KSSQSVLHSSNNNNYLA | WASTRES | QQYYNSPCS |
| | | | SEQ ID NO:2360 | SEQ ID NO:10372 | SEQ ID NO:18384 |
| iPS:435591 | | NA | CGGGCAAGTCAGGACATTAG AAAGGATTTAGGG | GCTGCATCCAGTTTGCAA AGT | CTACAGCATTATAGTTATCC TCGGACG |
| | 21-225_160C4 | | SEQ ID NO:2361 | SEQ ID NO:10373 | SEQ ID NO:18385 |
| | | AA | RASQDIRKDLG | AASSLQS | LQHYSYPRT |
| | | | SEQ ID NO:2362 | SEQ ID NO:10374 | SEQ ID NO:18386 |
| iPS:435593 | | NA | CGGGCAAGTCAGGGCATTAG AAATGATTTAGGC | GTTGCATCCAGTTTGCAA AGT | ATACAGGATAATAGTCACCC ATTCACT |
| | 21-225_160F4 | | SEQ ID NO:2363 | SEQ ID NO:10375 | SEQ ID NO:18387 |
| | | AA | RASQGIRNDLG | VASSLQS | IQDNSHPFT |

FIGURE 49

| | | | | | |
|---|---|---|---|---|---|
| | | | SEQ ID NO:2364 | SEQ ID NO:10376 | SEQ ID NO:18388 |
| iPS:435595 | | NA | CGGGCGAGTCAGGACATTAGTAATTATTTAGTC | GTTGCATCCAGTTTGCAAAGT | CAACAGTATAATAGTTACCCTCTCACT |
| | 21-225_160H4 | | SEQ ID NO:2365 | SEQ ID NO:10377 | SEQ ID NO:18389 |
| | | AA | RASQDISNYLV | VASSLQS | QQYNSYPLT |
| | | | SEQ ID NO:2366 | SEQ ID NO:10378 | SEQ ID NO:18390 |
| iPS:435599 | | NA | CGGGCAAGTCAGGGCATTAGAAATGATTTAGGC | GCTGCATCCAGTTTGCAAAGT | CTACAGCATAGTAGTTACCCGCTCACT |
| | 21-225_160B10 | | SEQ ID NO:2367 | SEQ ID NO:10379 | SEQ ID NO:18391 |
| | | AA | RASQGIRNDLG | AASSLQS | LQHSSYPLT |
| | | | SEQ ID NO:2368 | SEQ ID NO:10380 | SEQ ID NO:18392 |
| iPS:435601 | | NA | AAGTCTAGTCAGAGCCTCCTGCACGGTGATGGAAAGACCTATTTGTAT | GAAGTTTCCAAACGGTTCTCT | ATGCAAAGTATACAGCTTCCGTGGACG |
| | 21-225_160G10 | | SEQ ID NO:2369 | SEQ ID NO:10381 | SEQ ID NO:18393 |
| | | AA | KSSQSLLHGDGKTYLY | EVSKRFS | MQSIQLPWT |
| | | | SEQ ID NO:2370 | SEQ ID NO:10382 | SEQ ID NO:18394 |
| iPS:435605 | | NA | AGGTCCAGTCAGAGTGTTAACAGCAACTTAGCC | GGTGCATCCATCAGGGCCACT | CAGCAGTATAATAACTGGTGGACG |
| | 21-225_161A4 | | SEQ ID NO:2371 | SEQ ID NO:10383 | SEQ ID NO:18395 |
| | | AA | RSSQSVNSNLA | GASIRAT | QQYNNWWT |
| | | | SEQ ID NO:2372 | SEQ ID NO:10384 | SEQ ID NO:18396 |
| iPS:435607 | | NA | CAGGCGAGTCAGGACATTTACAATTATTTAAAT | GATGCATCCAATTTGGAAACA | CAACAGTATGATATTCTCCCGATCACC |
| | 21-225_161G4 | | SEQ ID NO:2373 | SEQ ID NO:10385 | SEQ ID NO:18397 |
| | | AA | QASQDIYNYLN | DASNLET | QQYDILPIT |
| | | | SEQ ID NO:2374 | SEQ ID NO:10386 | SEQ ID NO:18398 |
| iPS:435609 | | NA | CGGGCAAGTCAGGGCATTAGAAATGATTTGGGC | GCTGCATCCACTTTGCAAAGT | CTACTATATATTCGTTACCCATTCACT |
| | 21-225_161F7 | | SEQ ID NO:2375 | SEQ ID NO:10387 | SEQ ID NO:18399 |

FIGURE 49

| | | | | | |
|---|---|---|---|---|---|
| | | AA | RASQGIRNDLG | AASTLQS | LLYIRYPFT |
| | | | SEQ ID NO:2376 | SEQ ID NO:10388 | SEQ ID NO:18400 |
| iPS:435611 | 21-225_161F10 | NA | CAGGCGAGTCAGGACATTTA CAACCATTTAAGT | GATGCATCCAATTGGGA AACA | CAACAGTATGAAAATCTCCC GCTCACC |
| | | | SEQ ID NO:2377 | SEQ ID NO:10389 | SEQ ID NO:18401 |
| | | AA | QASQDIYNHLS | DASNWET | QQYENLPLT |
| | | | SEQ ID NO:2378 | SEQ ID NO:10390 | SEQ ID NO:18402 |
| iPS:435613 | 21-225_161D11 | NA | CGGGCAAGTCAGGGCATTAG AAATGATTTGGGC | GCTGCATCCACTTTGCAA AGT | CTACAATATAATCGTTACCC ATTCACT |
| | | | SEQ ID NO:2379 | SEQ ID NO:10391 | SEQ ID NO:18403 |
| | | AA | RASQGIRNDLG | AASTLQS | LQYNRYPFT |
| | | | SEQ ID NO:2380 | SEQ ID NO:10392 | SEQ ID NO:18404 |
| iPS:435615 | 21-225_161G12 | NA | CGGGCAAGTCAGGACATTAG AAAGGATTTAGGG | GCTGCATCCAGTTTGCAA AGT | CTACAGCATCATAGTTATCC TCGGACG |
| | | | SEQ ID NO:2381 | SEQ ID NO:10393 | SEQ ID NO:18405 |
| | | AA | RASQDIRKDLG | AASSLQS | LQHHSYPRT |
| | | | SEQ ID NO:2382 | SEQ ID NO:10394 | SEQ ID NO:18406 |
| iPS:435617 | 21-225_162F2 | NA | CGGGCAAGTCAGGGCATTAG AAATGATTTAGGC | GCTGCATCCAGTTTGCAA AGT | ATACAGGATAATAGTCACCC ATTCACT |
| | | | SEQ ID NO:2383 | SEQ ID NO:10395 | SEQ ID NO:18407 |
| | | AA | RASQGIRNDLG | AASSLQS | IQDNSHPFT |
| | | | SEQ ID NO:2384 | SEQ ID NO:10396 | SEQ ID NO:18408 |
| iPS:435621 | 21-225_162H3 | NA | CGGGCAAGTCAGGGCATTAG AAATGATTTAGGC | GCTGCATCCAGTTTGCAA AGT | CTACAGGATAATAGTCACCC ATTCACT |
| | | | SEQ ID NO:2385 | SEQ ID NO:10397 | SEQ ID NO:18409 |
| | | AA | RASQGIRNDLG | AASSLQS | LQDNSHPFT |
| | | | SEQ ID NO:2386 | SEQ ID NO:10398 | SEQ ID NO:18410 |
| iPS:435623 | 21 225 162D5 | NA | AAGTCCAACCATAGTGTTTT ATACAGGTCCAACAATAATC AATACTTAGCT | CGGACATCTATCCGGAA ATCC | CAGCAATATTATAGTACTCC TCCCACT |

781

FIGURE 49

| | | | | | |
|---|---|---|---|---|---|
| | 21-225_162D5 | | SEQ ID NO:2387 | SEQ ID NO:10399 | SEQ ID NO:18411 |
| | | AA | KSNHSVLYRSNNNQYLA | RTSIRKS | QQYYSTPPT |
| iPS:435627 | 21-225_162F6 | NA | SEQ ID NO:2388 AAGTCCAGCCAGAATGTTTT ACACAGCTCCAACAATAACA ACTACTTAACT | SEQ ID NO:10400 TGGGCATCTACCCGGGA ATCC | SEQ ID NO:18412 CAGCAATATTATAGTACTCC TCCGACG |
| | | AA | SEQ ID NO:2389 KSSQNVLHSSNNNNYLT | SEQ ID NO:10401 WASTRES | SEQ ID NO:18413 QQYYSTPPT |
| iPS:435629 | 21-225_162H6 | NA | SEQ ID NO:2390 AAGTCTACTCAGAGCCTCCT GCATGGTGATGGAAAGACCT ATTTGTAT | SEQ ID NO:10402 GAAGTTTCCAAGCGGTT CTCT | SEQ ID NO:18414 AAGCAAAGTATACAGCTTCC GTGGACG |
| | | AA | SEQ ID NO:2391 KSTQSLLHGDGKTYLY | SEQ ID NO:10403 EVSKRFS | SEQ ID NO:18415 KQSIQLPWT |
| iPS:435635 | 21-225_163F1 | NA | SEQ ID NO:2392 CGGGCGAGTCAGGACATTAG CAATTATTTAGCC | SEQ ID NO:10404 GCTGCATCCAGTTTGCAA AGT | SEQ ID NO:18416 CAACAGTATAATAGTTACCC ATTCACT |
| | | AA | SEQ ID NO:2393 RASQDISNYLA | SEQ ID NO:10405 AASSLQS | SEQ ID NO:18417 QQYNSYPFT |
| iPS:435637 | 21-225_163E2 | NA | SEQ ID NO:2394 CGGGCAAGTCAGGACATTAG AAATGATTTAGGC | SEQ ID NO:10406 CCTGCATCCAGTTTGCAA AGT | SEQ ID NO:18418 CTACAGCATAATAGTCACCC ATTCACT |
| | | AA | SEQ ID NO:2395 RASQDIRNDLG | SEQ ID NO:10407 PASSLQS | SEQ ID NO:18419 LQHNSHPFT |
| iPS:435639 | 21-225_163G6 | NA | SEQ ID NO:2396 CGGGCGAGTCAGGACATTAG CAATTATTTAGTC | SEQ ID NO:10408 GCTGCATCCAGTTTGCTA AGT | SEQ ID NO:18420 CAACAGTATCATAGTTACCC GCTCACT |
| | | AA | SEQ ID NO:2397 RASQDISNYLV | SEQ ID NO:10409 AASSLLS | SEQ ID NO:18421 QQYHSYPLT |
| | | | SEQ ID NO:2398 | SEQ ID NO:10410 | SEQ ID NO:18422 |

EP 4 435 105 A2

FIGURE 49

| iPS:435641 | | NA | CGGGCAAGTCAGGACATTAGAGATGATTTAGGC | CCTGCATCCAGTTTGCAAAGT | CTACAGGATAATAGTTACCCATTCACT |
|---|---|---|---|---|---|
| | 21-225_163F9 | | SEQ ID NO:2399 | SEQ ID NO:10411 | SEQ ID NO:18423 |
| | | AA | RASQDIRDDLG | PASSLQS | LQDNSYPFT |
| | | | SEQ ID NO:2400 | SEQ ID NO:10412 | SEQ ID NO:18424 |
| iPS:435643 | | NA | CGGGCAAGTCAGGACATTAGAAATAATTTAGGC | CCTGCATCCAGTTTGCAAAGT | CTACAGGATTATAGTTACCCATTCACT |
| | 21-225_163G10 | | SEQ ID NO:2401 | SEQ ID NO:10413 | SEQ ID NO:18425 |
| | | AA | RASQDIRNNLG | PASSLQS | LQDYSYPFT |
| | | | SEQ ID NO:2402 | SEQ ID NO:10414 | SEQ ID NO:18426 |
| iPS:435649 | | NA | AAGTCCAGCCAGAGTGTTTTACACAGCTCCAACAATAAGAACTACTTAACT | TGGGCATCTACCCGAGAATCC | CAGCAATCTTATAGTATTCCTCCCACT |
| | 21-225_165H2 | | SEQ ID NO:2403 | SEQ ID NO:10415 | SEQ ID NO:18427 |
| | | AA | KSSQSVLHSSNNKNYLT | WASTRES | QQSYSIPPT |
| | | | SEQ ID NO:2404 | SEQ ID NO:10416 | SEQ ID NO:18428 |
| iPS:435653 | | NA | CGGGCGAGTCAGGACATTAGCCATTATTTAGCC | GCTGCATCCAGTTTGCAAAGT | CAACAGTATAATAGTTTCCCGCTCACT |
| | 21-225_166H12 | | SEQ ID NO:2405 | SEQ ID NO:10417 | SEQ ID NO:18429 |
| | | AA | RASQDISHYLA | AASSLQS | QQYNSFPLT |
| | | | SEQ ID NO:2406 | SEQ ID NO:10418 | SEQ ID NO:18430 |
| iPS:435655 | | NA | AAGTCTAGTCAGAGCCTCCTGCACGGTGATGGGAAGACCTATTTGTAT | GAAGTTTCCAAACGGTTCTCT | ATGCAAAGCATACAGCTTCCGTGGACG |
| | 21-225_167E2 | | SEQ ID NO:2407 | SEQ ID NO:10419 | SEQ ID NO:18431 |
| | | AA | KSSQSLLHGDGKTYLY | EVSKRFS | MQSIQLPWT |
| | | | SEQ ID NO:2408 | SEQ ID NO:10420 | SEQ ID NO:18432 |
| iPS:435657 | 21 225 167H10 | NA | AAGTCTAGTCAGAGCCTCCTGCACGGTGATGGGAAGACCTATTTGTAT | GAAGTTTCCAAACGGTTCTCT | ATGCAAAGCATACAGCTTCCGTGGACG |

FIGURE 49

| | | | SEQ ID NO:2409 | SEQ ID NO:10421 | SEQ ID NO:18433 |
|---|---|---|---|---|---|
| | 21-225_167H10 | AA | KSSQSLLHGDGKTYLY | EVSKRFS | MQSIQLPWT |
| | | | SEQ ID NO:2410 | SEQ ID NO:10422 | SEQ ID NO:18434 |
| iPS:435659 | | NA | CGGGCGAGTCAGGGCATTAACAATTATTTAGCC | GCTGCATCCAGTTTGCAAAGT | CAACAGTATAATAGTTATCCATTCACT |
| | 21-225_167D12 | | SEQ ID NO:2411 | SEQ ID NO:10423 | SEQ ID NO:18435 |
| | | AA | RASQGINNYLA | AASSLQS | QQYNSYPFT |
| | | | SEQ ID NO:2412 | SEQ ID NO:10424 | SEQ ID NO:18436 |
| iPS:435663 | | NA | CGGGCTAGTCAGGGCATTAGAAATGATTTAGGC | GCTGAATCCAGTTTGCAAAGT | CTACAGCATTATAGTTACCCGCTCACT |
| | 21-225_169B1 | | SEQ ID NO:2413 | SEQ ID NO:10425 | SEQ ID NO:18437 |
| | | AA | RASQGIRNDLG | AESSLQS | LQHYSYPLT |
| | | | SEQ ID NO:2414 | SEQ ID NO:10426 | SEQ ID NO:18438 |
| iPS:435665 | | NA | AAGTCCAGCCAGAGTGTTTTATACATCTCCAACAATAAAAACTACTTAGCT | TGGGCATCTACCCGGGAATCC | CAGCAATATTATCGTGCTCCCACC |
| | 21-225_169F2 | | SEQ ID NO:2415 | SEQ ID NO:10427 | SEQ ID NO:18439 |
| | | AA | KSSQSVLYISNNKNYLA | WASTRES | QQYYRAPT |
| | | | SEQ ID NO:2416 | SEQ ID NO:10428 | SEQ ID NO:18440 |
| iPS:435667 | | NA | AGGTCTAGTCAGAGCCTCCTGCATAATAATGGATACAAGTATTTGGAT | TTGGGTTCTAATCGGGCCTCC | ATGCAAGTTCTACAAACTCCGTGGACG |
| | 21-225_169E3 | | SEQ ID NO:2417 | SEQ ID NO:10429 | SEQ ID NO:18441 |
| | | AA | RSSQSLLHNNGYKYLD | LGSNRAS | MQVLQTPWT |
| | | | SEQ ID NO:2418 | SEQ ID NO:10430 | SEQ ID NO:18442 |
| iPS:435669 | | NA | CGGGCAAGTCAGGGCATTAGAAATGATTTAGGC | GCTGCATCCAGTTTGCAAAGT | CTACAGCATTATAGTTACCCGCTCACT |
| | 21-225_169F9 | | SEQ ID NO:2419 | SEQ ID NO:10431 | SEQ ID NO:18443 |
| | | AA | RASQGIRNDLG | AASSLQS | LQHYSYPLT |
| | | | SEQ ID NO:2420 | SEQ ID NO:10432 | SEQ ID NO:18444 |

FIGURE 49

| iPS:435671 | | NA | AAGTCCAGCCAGAGTGTTTT ATACATCTCCAACAATAAAA ACTACTTAGCT | TGGGCATCTACCCGGGA ATCC | CAGCAATATTATCGTGCTCC CACC |
|---|---|---|---|---|---|
| | 21-225_169H5 | | SEQ ID NO:2421 | SEQ ID NO:10433 | SEQ ID NO:18445 |
| | | AA | KSSQSVLYISNNKNYLA | WASTRES | QQYYRAPT |
| | | | SEQ ID NO:2422 | SEQ ID NO:10434 | SEQ ID NO:18446 |
| iPS:435673 | | NA | AGGTCTAGTCAGAGCCTCCT GCATAATAATGGATACAAGT ATTTGGAT | TTGGGTTCTAATCGGGCC TCC | ATGCAAGTTCTACAAACTCC GTGGACG |
| | 21-225_169E6 | | SEQ ID NO:2423 | SEQ ID NO:10435 | SEQ ID NO:18447 |
| | | AA | RSSQSLLHNNGYKYLD | LGSNRAS | MQVLQTPWT |
| | | | SEQ ID NO:2424 | SEQ ID NO:10436 | SEQ ID NO:18448 |
| iPS:435675 | | NA | CGGGCAAGTCAGGGCATTAG AAATGATTTAGGC | GCTGCATCCAGTTTGCAA AGT | CTACAGCATCATAGTTGCCC GTGGACG |
| | 21-225_169D7 | | SEQ ID NO:2425 | SEQ ID NO:10437 | SEQ ID NO:18449 |
| | | AA | RASQGIRNDLG | AASSLQS | LQHHSCPWT |
| | | | SEQ ID NO:2426 | SEQ ID NO:10438 | SEQ ID NO:18450 |
| iPS:435677 | | NA | CGGGCGAGTCAGGACATTAG CAATTATTTAGCC | TCTGCATCCAGTTTGCAA AGT | CAACAATCTGATAGTTACCC TCTCACT |
| | 21-225_169C10 | | SEQ ID NO:2427 | SEQ ID NO:10439 | SEQ ID NO:18451 |
| | | AA | RASQDISNYLA | SASSLQS | QQSDSYPLT |
| | | | SEQ ID NO:2428 | SEQ ID NO:10440 | SEQ ID NO:18452 |
| iPS:435679 | | NA | CGGGCGAGTCAGGACATTAG CAATTATTTAGCC | GCTGCATCCAGTTTGCAA AGT | CAACAGTATCATAGTTACCC ATTCACT |
| | 21-225_169D10 | | SEQ ID NO:2429 | SEQ ID NO:10441 | SEQ ID NO:18453 |
| | | AA | RASQDISNYLA | AASSLQS | QQYHSYPFT |
| | | | SEQ ID NO:2430 | SEQ ID NO:10442 | SEQ ID NO:18454 |
| iPS:435681 | | NA | CGGGCAAGTCAGGGCATTAG AGATGATTTAGGC | GCTGCATCCAGTTTGCAA AGT | CTTCAGCATTATAGTTACCCT CGGACG |
| | 21-225_169D11 | | SEQ ID NO:2431 | SEQ ID NO:10443 | SEQ ID NO:18455 |

FIGURE 49

| | | AA | RASQGIRDDLG | AASSLQS | LQHYSYPRT |
|---|---|---|---|---|---|
| | | | SEQ ID NO:2432 | SEQ ID NO:10444 | SEQ ID NO:18456 |
| iPS:435683 | 21-225_170A1 | NA | AAGTCTAGTCAGAGCCTCCTGCATGGTGATGGAAAGACCTATTTGTTT | GAAGTTTCCAACCGGTTCTCT | ATGCAAAGTATTCAGCTTCCGTGGACG |
| | | | SEQ ID NO:2433 | SEQ ID NO:10445 | SEQ ID NO:18457 |
| | | AA | KSSQSLLHGDGKTYLF | EVSNRFS | MQSIQLPWT |
| | | | SEQ ID NO:2434 | SEQ ID NO:10446 | SEQ ID NO:18458 |
| iPS:435685 | 21-225_170E1 | NA | CGGGCGAGTCAGGGCATTAGCAATTATTTAGCC | GCTGCATCCAGTTTGCAAAGT | CAACAGTATCATAGTTACCCATTCACT |
| | | | SEQ ID NO:2435 | SEQ ID NO:10447 | SEQ ID NO:18459 |
| | | AA | RASQGISNYLA | AASSLQS | QQYHSYPFT |
| | | | SEQ ID NO:2436 | SEQ ID NO:10448 | SEQ ID NO:18460 |
| iPS:435687 | 21-225_170H1 | NA | CGGGCAAGTCAGGGCATTAGAAATGATTTAGGC | GCTACATCCAGTTTGCAAAGT | CTACAGCATAGTAGTAACCCGTGGACG |
| | | | SEQ ID NO:2437 | SEQ ID NO:10449 | SEQ ID NO:18461 |
| | | AA | RASQGIRNDLG | ATSSLQS | LQHSSNPWT |
| | | | SEQ ID NO:2438 | SEQ ID NO:10450 | SEQ ID NO:18462 |
| iPS:435689 | 21-225_170F3 | NA | CGGGCAAGTCGGGGCATTAGAAATGATTTAGGC | GCTGCATCCAGTTTGCAAAGT | CTACAGCATTATAGTTACCCTCGGACG |
| | | | SEQ ID NO:2439 | SEQ ID NO:10451 | SEQ ID NO:18463 |
| | | AA | RASRGIRNDLG | AASSLQS | LQHYSYPRT |
| | | | SEQ ID NO:2440 | SEQ ID NO:10452 | SEQ ID NO:18464 |
| iPS:435693 | 21-225_170G4 | NA | CGGGCAAGTCAGGGCATTAGAAATGATTTAGGC | GCTGCATCCACTTTGCAAAGT | CTACAGCATTATAGTTACCCGCTCACT |
| | | | SEQ ID NO:2441 | SEQ ID NO:10453 | SEQ ID NO:18465 |
| | | AA | RASQGIRNDLG | AASTLQS | LQHYSYPLT |
| | | | SEQ ID NO:2442 | SEQ ID NO:10454 | SEQ ID NO:18466 |
| iPS:435695 | | NA | CGGGCAAGTCAGGGCATTAGAAATGATTTAGGC | GCTGCATCCAGTTTGCAAAAT | CTACAGCATTATAGTTTCCCGCTCACT |

FIGURE 49

| | 21-225_170D5 | | SEQ ID NO:2443 | SEQ ID NO:10455 | SEQ ID NO:18467 |
|---|---|---|---|---|---|
| | | AA | RASQGIRNDLG | AASSLQN | LQHYSFPLT |
| | | | SEQ ID NO:2444 | SEQ ID NO:10456 | SEQ ID NO:18468 |
| iPS:435697 | | NA | CGGGCAAGTCAGGGCATTAG AACTGATTTAGGC | ACTGCATCCAGTTTGCAA AGT | CTACAGCATTATAGTTACCC GCTCACT |
| | 21-225_170G5 | | SEQ ID NO:2445 | SEQ ID NO:10457 | SEQ ID NO:18469 |
| | | AA | RASQGIRTDLG | TASSLQS | LQHYSYPLT |
| | | | SEQ ID NO:2446 | SEQ ID NO:10458 | SEQ ID NO:18470 |
| iPS:435699 | | NA | CGGGCGAGTCAGGACATTGG CAATTGTTTAGCC | TCTGCGTCCAGTTTGCAA AGT | CAACAATCTGATAGTTACCC TCTCACT |
| | 21-225_170D6 | | SEQ ID NO:2447 | SEQ ID NO:10459 | SEQ ID NO:18471 |
| | | AA | RASQDIGNCLA | SASSLQS | QQSDSYPLT |
| | | | SEQ ID NO:2448 | SEQ ID NO:10460 | SEQ ID NO:18472 |
| iPS:435701 | | NA | AAGTCCAGCCAGAGTGTTTT ACACAGCTCCAACAATTACA ACTACTTAGCT | TGGGCATCTACCCGGAA ATCC | CAGCAATATTATAGTACTCC GTGGACG |
| | 21-225_170F6 | | SEQ ID NO:2449 | SEQ ID NO:10461 | SEQ ID NO:18473 |
| | | AA | KSSQSVLHSSNNYNYLA | WASTRKS | QQYYSTPWT |
| | | | SEQ ID NO:2450 | SEQ ID NO:10462 | SEQ ID NO:18474 |
| iPS:435703 | | NA | CGGGCAAGTCAGGGCATTAG AAATGATTTAGGC | GCTGCATCCAGTTTGCAA AAT | CTACAGCATTATAGTTTCCC GCTCACT |
| | 21-225_170D11 | | SEQ ID NO:2451 | SEQ ID NO:10463 | SEQ ID NO:18475 |
| | | AA | RASQGIRNDLG | AASSLQN | LQHYSFPLT |
| | | | SEQ ID NO:2452 | SEQ ID NO:10464 | SEQ ID NO:18476 |
| iPS:435705 | | NA | CGGGCAAGTCAGGGCATTAG AACTGATTTAGGC | ACTGCATCCAGTTTGCAA AGT | CTACAGCATTATAGTTACCC GCTCACT |
| | 21-225_171C3 | | SEQ ID NO:2453 | SEQ ID NO:10465 | SEQ ID NO:18477 |
| | | AA | RASQGIRTDLG | TASSLQS | LQHYSYPLT |
| | | | SEQ ID NO:2454 | SEQ ID NO:10466 | SEQ ID NO:18478 |

FIGURE 49

| iPS:435709 | | NA | CGGGCAAGTCAGGGCATTAG AAATGATTTAGGC | ACTGCATCCAGTTTGCAA AGT | CTACAGCATTATAGTTACCC GCTCACT |
|---|---|---|---|---|---|
| | 21-225_171A4 | | SEQ ID NO:2455 | SEQ ID NO:10467 | SEQ ID NO:18479 |
| | | AA | RASQGIRNDLG | TASSLQS | LQHYSYPLT |
| | | | SEQ ID NO:2456 | SEQ ID NO:10468 | SEQ ID NO:18480 |
| iPS:435711 | | NA | CGGGCGAGTCAGGGTGTTAA CGACTGGTTAGCC | GATGCATCAAGTTTGCA AAGT | CAACAGGCTAACAGTTTCCC GTGGACG |
| | 21-225_171G4 | | SEQ ID NO:2457 | SEQ ID NO:10469 | SEQ ID NO:18481 |
| | | AA | RASQGVNDWLA | DASSLQS | QQANSFPWT |
| | | | SEQ ID NO:2458 | SEQ ID NO:10470 | SEQ ID NO:18482 |
| iPS:435713 | | NA | AGGTCTAGTCAGAGCCTCCT GTATCATAATGGATACAACT ATTTGGAT | GTGGGTTCTAATCGGGC CTCC | ATGCAAACTCTACAAACTCC GCTCACT |
| | 21-225_171D7 | | SEQ ID NO:2459 | SEQ ID NO:10471 | SEQ ID NO:18483 |
| | | AA | RSSQSLLYHNGYNYLD | VGSNRAS | MQTLQTPLT |
| | | | SEQ ID NO:2460 | SEQ ID NO:10472 | SEQ ID NO:18484 |
| iPS:435715 | | NA | CGGGCGAGTCAGGGTATTAG CAACTGGTTAGCC | GCTGCATTCAGTTTGCAA GGT | CAACAGACTAACAGTTTCCC GTGGACG |
| | 21-225_171A8 | | SEQ ID NO:2461 | SEQ ID NO:10473 | SEQ ID NO:18485 |
| | | AA | RASQGISNWLA | AAFSLQG | QQTNSFPWT |
| | | | SEQ ID NO:2462 | SEQ ID NO:10474 | SEQ ID NO:18486 |
| iPS:435717 | | NA | CGGGCGAGTCAGGATATTAC CACCTGGTTAGCC | GATGCATCCAGTTTGCA AAGT | CTACAGACTAACAGTTTCCC GTGGACG |
| | 21-225_171A9 | | SEQ ID NO:2463 | SEQ ID NO:10475 | SEQ ID NO:18487 |
| | | AA | RASQDITTWLA | DASSLQS | LQTNSFPWT |
| | | | SEQ ID NO:2464 | SEQ ID NO:10476 | SEQ ID NO:18488 |
| iPS:435719 | | NA | CGGGCAAGTCAGGGCATTAG AAATAATTTAGGC | CCTGCATCCAGTTTGCAA AGT | CTACAGGATCATAGTTACCC ATTCACT |
| | 21-225_171A11 | | SEQ ID NO:2465 | SEQ ID NO:10477 | SEQ ID NO:18489 |
| | | AA | RASQGIRNNLG | PASSLQS | LQDHSYPFT |

788

FIGURE 49

| | | | SEQ ID NO:2466 | SEQ ID NO:10478 | SEQ ID NO:18490 |
|---|---|---|---|---|---|
| iPS:435721 | | NA | CGGGCTAGTCAGGGCATTAG AAATGATTTAGGC | GCTGAATCCAGTTTGCA AAGT | CTACAGCATTATAGTTACCC GCTCACT |
| | 21-225_172B3 | | SEQ ID NO:2467 | SEQ ID NO:10479 | SEQ ID NO:18491 |
| | | AA | RASQGIRNDLG | AESSLQS | LQHYSYPLT |
| | | | SEQ ID NO:2468 | SEQ ID NO:10480 | SEQ ID NO:18492 |
| iPS:435723 | | NA | AAGTCTAGTCAGAGCCTCCT GCATGGTGATGGAAAGACCT ATTTCTAT | GAAGTTTCCCACCGGTTC TCT | ATGCAAAGTATACAGTTTCC GTGGACG |
| | 21-225_172B7 | | SEQ ID NO:2469 | SEQ ID NO:10481 | SEQ ID NO:18493 |
| | | AA | KSSQSLLHGDGKTYFY | EVSHRFS | MQSIQFPWT |
| | | | SEQ ID NO:2470 | SEQ ID NO:10482 | SEQ ID NO:18494 |
| iPS:435725 | | NA | CGGGCAAGTCAGGGCGTTAG AAATGATTTAGGC | GCTGCATCCAGTTTGCAA AAT | CTACACCATTATAGTTTCCC GCTCACT |
| | 21-225_172G8 | | SEQ ID NO:2471 | SEQ ID NO:10483 | SEQ ID NO:18495 |
| | | AA | RASQGVRNDLG | AASSLQN | LHHYSFPLT |
| | | | SEQ ID NO:2472 | SEQ ID NO:10484 | SEQ ID NO:18496 |
| iPS:435727 | | NA | AAGTCCAGCCAGAGTGTTTT ACACAGCTCCAACAATAACA ACTACTTAGCT | TGGGCATCTACTCGGGA ATCC | CAGCAATATTTTACTACTCC GTGCAGT |
| | 21-225_172E11 | | SEQ ID NO:2473 | SEQ ID NO:10485 | SEQ ID NO:18497 |
| | | AA | KSSQSVLHSSNNNNYLA | WASTRES | QQYFTTPCS |
| | | | SEQ ID NO:2474 | SEQ ID NO:10486 | SEQ ID NO:18498 |
| iPS:435729 | | NA | CGTGCAAGTCAGACCATTAG CAACTATTTAAAT | GCTGCATCCAGTTTGCAA ATT | CAACAGAGTTACAGAACCCC TCAGTGGACG |
| | 21-225_173E7 | | SEQ ID NO:2475 | SEQ ID NO:10487 | SEQ ID NO:18499 |
| | | AA | RASQTISNYLN | AASSLQI | QQSYRTPQWT |
| | | | SEQ ID NO:2476 | SEQ ID NO:10488 | SEQ ID NO:18500 |

FIGURE 49

| iPS:435731 | | NA | AAGTCTAGTCAGAGCCTCCT GCATGGTGATGGAAAGACCT ATTTGTAT | GAAGTTTCCAACCGGTTC TCT | ATGCAAAGTATACAGGTTCC GTGGACG |
|---|---|---|---|---|---|
| | 21-225_173A11 | | SEQ ID NO:2477 | SEQ ID NO:10489 | SEQ ID NO:18501 |
| | | AA | KSSQSLLHGDGKTYLY | EVSNRFS | MQSIQVPWT |
| | | | SEQ ID NO:2478 | SEQ ID NO:10490 | SEQ ID NO:18502 |
| iPS:435733 | | NA | AAGTCTAGTCAGAGCCTCCT GCATAGTGAGGGAAAGACC TACTTGTAT | GAAGTTTCCCACCGGTTC TCT | ATGCAAAGTATACAGCTTCT CACT |
| | 21-225_173C11 | | SEQ ID NO:2479 | SEQ ID NO:10491 | SEQ ID NO:18503 |
| | | AA | KSSQSLLHSEGKTYLY | EVSHRFS | MQSIQLLT |
| | | | SEQ ID NO:2480 | SEQ ID NO:10492 | SEQ ID NO:18504 |
| iPS:435735 | | NA | CGGGCAAGTCAGGGCATTAG AAATGATTTAGGC | ACTACATCCAGTTTGCAA AGT | CTACAGCATTATAGTTTCCC GAACACT |
| | 21-225_173H12 | | SEQ ID NO:2481 | SEQ ID NO:10493 | SEQ ID NO:18505 |
| | | AA | RASQGIRNDLG | TTSSLQS | LQHYSFPNT |
| | | | SEQ ID NO:2482 | SEQ ID NO:10494 | SEQ ID NO:18506 |
| iPS:435737 | | NA | AAGTCCAGCCAGAGTGTATT ACACAGCTCCAACAATTACA ACTACTTAACT | TGGGCATCTACCCGGGA ATCC | CAGCAATATTATCGTACTCC GTGGACG |
| | 21-225_174G5 | | SEQ ID NO:2483 | SEQ ID NO:10495 | SEQ ID NO:18507 |
| | | AA | KSSQSVLHSSNNYNYLT | WASTRES | QQYYRTPWT |
| | | | SEQ ID NO:2484 | SEQ ID NO:10496 | SEQ ID NO:18508 |
| iPS:435739 | | NA | CGGGCGAGTCAGGGTATTAG CAACTGGTTAGCC | GCTGCATTCAGTTTGCAA GGT | CAACAGACTAACAGTTTCCC GTGGACG |
| | 21-225_174G7 | | SEQ ID NO:2485 | SEQ ID NO:10497 | SEQ ID NO:18509 |
| | | AA | RASQGISNWLA | AAFSLQG | QQTNSFPWT |
| | | | SEQ ID NO:2486 | SEQ ID NO:10498 | SEQ ID NO:18510 |
| iPS:435741 | | NA | CGGGCAAGTCAGGGCATTAG AGATGATTTAGGC | GCTGCATCCAGTTTGCAA AGT | CTTCAGCATCATAGTTACCC TCGGACG |

FIGURE 49

| | | | | | |
|---|---|---|---|---|---|
| | **21-225_174G10** | | SEQ ID NO:2487 | SEQ ID NO:10499 | SEQ ID NO:18511 |
| | | AA | RASQGIRDDLG | AASSLQS | LQHHSYPRT |
| | | | SEQ ID NO:2488 | SEQ ID NO:10500 | SEQ ID NO:18512 |
| iPS:435743 | **21-225_175G1** | NA | CGGGCAAGTCAGGGCATTAGAACTGATTTAGGC | ACTGCATCCAGTTTGCAAAGT | CTACAGCATTATAGTTACCCGCTCACT |
| | | | SEQ ID NO:2489 | SEQ ID NO:10501 | SEQ ID NO:18513 |
| | | AA | RASQGIRTDLG | TASSLQS | LQHYSYPLT |
| | | | SEQ ID NO:2490 | SEQ ID NO:10502 | SEQ ID NO:18514 |
| iPS:435745 | **21-225_175G3** | NA | CGGGCGAGTCAGGACATTAGCAATGATTTAGCC | TCTGCATCCAGTTTGCAAAGT | CAACAATCTGATAGTTACCCTCTCACT |
| | | | SEQ ID NO:2491 | SEQ ID NO:10503 | SEQ ID NO:18515 |
| | | AA | RASQDISNDLA | SASSLQS | QQSDSYPLT |
| | | | SEQ ID NO:2492 | SEQ ID NO:10504 | SEQ ID NO:18516 |
| iPS:435747 | **21-225_175C4** | NA | CGGGCGAGTCAGGGCATTGGGAATTATTTAGCC | GCTGCATCCGGTTTGCAAAGT | CAACAGTATTATAGTTACCCATTCACT |
| | | | SEQ ID NO:2493 | SEQ ID NO:10505 | SEQ ID NO:18517 |
| | | AA | RASQGIGNYLA | AASGLQS | QQYYSYPFT |
| | | | SEQ ID NO:2494 | SEQ ID NO:10506 | SEQ ID NO:18518 |
| iPS:435749 | **21-225_175C10** | NA | CGGGCGAGTCAGGGTATTACCGACTGGTTAGCC | GCTGCATCCAGTTTGCAAAGT | CAACAGACTAACAGTTTCCCGTGGACG |
| | | | SEQ ID NO:2495 | SEQ ID NO:10507 | SEQ ID NO:18519 |
| | | AA | RASQGITDWLA | AASSLQS | QQTNSFPWT |
| | | | SEQ ID NO:2496 | SEQ ID NO:10508 | SEQ ID NO:18520 |
| iPS:435751 | **21-225_175D10** | NA | AAGTCCAGCCAGAGTGTTTTATACAGCTCCAACAATAACAACTACTTAGCT | TGGACATCTACCCGGGAATCC | CAGCAATATTATAGTACTCCTCCGACG |
| | | | SEQ ID NO:2497 | SEQ ID NO:10509 | SEQ ID NO:18521 |
| | | AA | KSSQSVLYSSNNNNYLA | WTSTRES | QQYYSTPPT |
| | | | SEQ ID NO:2498 | SEQ ID NO:10510 | SEQ ID NO:18522 |

FIGURE 49

| iPS:435753 | | NA | CGGGCAAGTCAGACCATTGGCAACTATTTAAAT | GCTGCATCCAGTTTGCACAGT | CAACAGAGTTACAGAACCCCTCAGTGGACG |
|---|---|---|---|---|---|
| | 21-225_175G10 | | SEQ ID NO:2499 | SEQ ID NO:10511 | SEQ ID NO:18523 |
| | | AA | RASQTIGNYLN | AASSLHS | QQSYRTPQWT |
| | | | SEQ ID NO:2500 | SEQ ID NO:10512 | SEQ ID NO:18524 |
| iPS:435755 | | NA | AAGTCTAGTCAGAGCCTCCTGCATGGTGATGGAAAGACCTATTTGTAT | GAAGTTTCCAACCGGTTCTCT | ATGCAAAGTATACAGATTCCGTGGACG |
| | 21-225_176H4 | | SEQ ID NO:2501 | SEQ ID NO:10513 | SEQ ID NO:18525 |
| | | AA | KSSQSLLHGDGKTYLY | EVSNRFS | MQSIQIPWT |
| | | | SEQ ID NO:2502 | SEQ ID NO:10514 | SEQ ID NO:18526 |
| iPS:435759 | | NA | AGGTCTAGTCAGAGCCTCCTGCATAATAATGGATACAAGTATTTGGAT | TTGGGTTCTAATCGGGCCTCC | ATGCAAGTTCTACAAACTCCGTGGACG |
| | 21-225_176E6 | | SEQ ID NO:2503 | SEQ ID NO:10515 | SEQ ID NO:18527 |
| | | AA | RSSQSLLHNNGYKYLD | LGSNRAS | MQVLQTPWT |
| | | | SEQ ID NO:2504 | SEQ ID NO:10516 | SEQ ID NO:18528 |
| iPS:435761 | | NA | CGGGCAAGTCAGGGCATTAGAAATGATTTAGGC | GCTGCATCCAGTTTGCAAAGT | CTACAGCATTATAGTTACCCGCTCACT |
| | 21-225_176B11 | | SEQ ID NO:2505 | SEQ ID NO:10517 | SEQ ID NO:18529 |
| | | AA | RASQGIRNDLG | AASSLQS | LQHYSYPLT |
| | | | SEQ ID NO:2506 | SEQ ID NO:10518 | SEQ ID NO:18530 |
| iPS:435763 | | NA | CGGGCAAGTCAGGGCATTAGAAATGATTTAGGC | GCTGCATCCAGTTTGCAAAGT | CTACAGCATAATAGTTACCCTCGCAGT |
| | 21-225_176H12 | | SEQ ID NO:2507 | SEQ ID NO:10519 | SEQ ID NO:18531 |
| | | AA | RASQGIRNDLG | AASSLQS | LQHNSYPRS |
| | | | SEQ ID NO:2508 | SEQ ID NO:10520 | SEQ ID NO:18532 |
| iPS:435765 | | NA | CGGGCGAGTCAGGGCATTACCAATTATTTAGCC | GCTGCATCCAGTTTGCAAAGT | CAACAGTATAATAGTTACCCATTCACT |
| | 21-225_177D3 | | SEQ ID NO:2509 | SEQ ID NO:10521 | SEQ ID NO:18533 |

FIGURE 49

| | | AA | RASQGITNYLA | AASSLQS | QQYNSYPFT |
|---|---|---|---|---|---|
| | | | SEQ ID NO:2510 | SEQ ID NO:10522 | SEQ ID NO:18534 |
| iPS:435767 | | NA | CGGGCAAGTCAGGGCATTAGAAATGATTTAGGC | GCTGCATCCAGTTTGCAAAGT | CTACAGCATTATAGTTTCCCTCGCAGT |
| | 21-225_177B4 | | SEQ ID NO:2511 | SEQ ID NO:10523 | SEQ ID NO:18535 |
| | | AA | RASQGIRNDLG | AASSLQS | LQHYSFPRS |
| | | | SEQ ID NO:2512 | SEQ ID NO:10524 | SEQ ID NO:18536 |
| iPS:435769 | | NA | CGGGCAAGTCAGGACATTAGCAATGATTTAGGC | GCTGCATCCAGTTTGCAAAGT | CTACAGCTTAATAGTTACCCATTCACT |
| | 21-225_177B6 | | SEQ ID NO:2513 | SEQ ID NO:10525 | SEQ ID NO:18537 |
| | | AA | RASQDISNDLG | AASSLQS | LQLNSYPFT |
| | | | SEQ ID NO:2514 | SEQ ID NO:10526 | SEQ ID NO:18538 |
| iPS:435771 | | NA | AAGTCTAGTCAGCGCCTCCTGCATGGTGATGGAAAGACCTATTTGTAT | GAAGTTTCCAACCGGTTCTCT | ATGCAAAGTATACAGATTCCGTGGACG |
| | 21-225_177B11 | | SEQ ID NO:2515 | SEQ ID NO:10527 | SEQ ID NO:18539 |
| | | AA | KSSQRLLHGDGKTYLY | EVSNRFS | MQSIQIPWT |
| | | | SEQ ID NO:2516 | SEQ ID NO:10528 | SEQ ID NO:18540 |
| iPS:435773 | | NA | AAGTCCAGCCAGAGTGTTTTACACAGCTCCAACAATAACAACTACTTAACT | TGGGCATCTACCCGGGAATCC | CAGCAATATTATAGTAGTCCTCCGACG |
| | 21-225_177B12 | | SEQ ID NO:2517 | SEQ ID NO:10529 | SEQ ID NO:18541 |
| | | AA | KSSQSVLHSSNNNNYLT | WASTRES | QQYYSSPPT |
| | | | SEQ ID NO:2518 | SEQ ID NO:10530 | SEQ ID NO:18542 |
| iPS:435775 | | NA | CGGGCGAGTCAGGGTATTAGCAACTGGTTAGCC | GCTGCTTCCAGTTTGCAAAGT | CAACAGGCTAACAGTTTACCGTGGACG |
| | 21-225_178A5 | | SEQ ID NO:2519 | SEQ ID NO:10531 | SEQ ID NO:18543 |
| | | AA | RASQGISNWLA | AASSLQS | QQANSLPWT |
| | | | SEQ ID NO:2520 | SEQ ID NO:10532 | SEQ ID NO:18544 |

FIGURE 49

| iPS:435777 | | NA | CGGGCGAGTCAGGATATTAC TGACTGGTTAGCC | GCTGCATCCAGTTTGCAG AGT | CAACAGGCTAACAGTTTACC GTGGACG |
|---|---|---|---|---|---|
| | 21-225_178F7 | | SEQ ID NO:2521 | SEQ ID NO:10533 | SEQ ID NO:18545 |
| | | AA | RASQDITDWLA | AASSLQS | QQANSLPWT |
| | | | SEQ ID NO:2522 | SEQ ID NO:10534 | SEQ ID NO:18546 |
| iPS:435779 | | NA | CGGGCAAGTCAGGGCATTAG AAATGATTTAGGC | GCTGCATCCAGTTTGCAA AAT | CTACACCATTATAGTTTCCC GCTCACT |
| | 21-225_178B10 | | SEQ ID NO:2523 | SEQ ID NO:10535 | SEQ ID NO:18547 |
| | | AA | RASQGIRNDLG | AASSLQN | LHHYSFPLT |
| | | | SEQ ID NO:2524 | SEQ ID NO:10536 | SEQ ID NO:18548 |
| iPS:435781 | | NA | AAGTCTAGTCAGAGCCTCCT GCATGGTGATGGAAAGACCT ATTTGTAT | GAAGTTTCCAACCGGTTT TCT | ATGCAAAGTATACAGGTTCC GTGGACG |
| | 21-225_178G10 | | SEQ ID NO:2525 | SEQ ID NO:10537 | SEQ ID NO:18549 |
| | | AA | KSSQSLLHGDGKTYLY | EVSNRFS | MQSIQVPWT |
| | | | SEQ ID NO:2526 | SEQ ID NO:10538 | SEQ ID NO:18550 |
| iPS:435783 | | NA | CGGGCGAGTCAGGATATTAG CGACTGGTTAGCC | GCTGCATCCAGTTTGCAA AGT | CAACAGGCTAACAGTTTACC GTGGACG |
| | 21-225_179G1 | | SEQ ID NO:2527 | SEQ ID NO:10539 | SEQ ID NO:18551 |
| | | AA | RASQDISDWLA | AASSLQS | QQANSLPWT |
| | | | SEQ ID NO:2528 | SEQ ID NO:10540 | SEQ ID NO:18552 |
| iPS:435785 | | NA | AAATCTAGTCAGAGCCTCCT GCATAGTGAGGGAAAGACC TACTTGTAT | GAGGTTTCCCACCGGTTC TCT | ATGCAAAGTATACAGGTTCT CACT |
| | 21-225_179C2 | | SEQ ID NO:2529 | SEQ ID NO:10541 | SEQ ID NO:18553 |
| | | AA | KSSQSLLHSEGKTYLY | EVSHRFS | MQSIQVLT |
| | | | SEQ ID NO:2530 | SEQ ID NO:10542 | SEQ ID NO:18554 |
| iPS:435787 | | NA | CGGGCGAGTCAGGATATTAC CAGCTGGTTAGCC | GCTGCATCCAGTTTGCAA AGT | CAACAGGCTAACAGTATCCC ATTCACT |
| | 21-225_180A3 | | SEQ ID NO:2531 | SEQ ID NO:10543 | SEQ ID NO:18555 |

FIGURE 49

| | | AA | RASQDITSWLA | AASSLQS | QQANSIPFT |
|---|---|---|---|---|---|
| | | | SEQ ID NO:2532 | SEQ ID NO:10544 | SEQ ID NO:18556 |
| iPS:435789 | 21-225_180C4 | NA | AAGTCTAGTCAGAGCCTCCT GCATGGTGATGGAAAGACCT ATTTGTAT | GCAACTTCCAACCGGTTC CCT | ATGCAAAGTATACAGGTTCC GTGGACG |
| | | | SEQ ID NO:2533 | SEQ ID NO:10545 | SEQ ID NO:18557 |
| | | AA | KSSQSLLHGDGKTYLY | ATSNRFP | MQSIQVPWT |
| | | | SEQ ID NO:2534 | SEQ ID NO:10546 | SEQ ID NO:18558 |
| iPS:435791 | 21-225_180H7 | NA | CGGGCAAGTCAGGGCATTAG AAATGATTTAGGC | ACTGCATCCAGTTTGCAA AGT | CTACAGCATAATAGTTACCC ATTCACT |
| | | | SEQ ID NO:2535 | SEQ ID NO:10547 | SEQ ID NO:18559 |
| | | AA | RASQGIRNDLG | TASSLQS | LQHNSYPFT |
| | | | SEQ ID NO:2536 | SEQ ID NO:10548 | SEQ ID NO:18560 |
| iPS:435793 | 21-225_180F8 | NA | CGGGCAAGTCAGACCATTCT CAGCTATTTAAAT | GGTGTATCCAGTTTACAA AGT | CAGCAGAGTTACAGTACCCC ATTCACT |
| | | | SEQ ID NO:2537 | SEQ ID NO:10549 | SEQ ID NO:18561 |
| | | AA | RASQTILSYLN | GVSSLQS | QQSYSTPFT |
| | | | SEQ ID NO:2538 | SEQ ID NO:10550 | SEQ ID NO:18562 |
| iPS:435795 | 21-225_181C2 | NA | AAGTCTAGTCAGAGCCTCCT GCATGGTGATGGAAAGACCT ATTTGTAT | GAAGTTTCCAACCGGTTC TCT | ATGCAAAGTATACAGGTTCC CTGGACG |
| | | | SEQ ID NO:2539 | SEQ ID NO:10551 | SEQ ID NO:18563 |
| | | AA | KSSQSLLHGDGKTYLY | EVSNRFS | MQSIQVPWT |
| | | | SEQ ID NO:2540 | SEQ ID NO:10552 | SEQ ID NO:18564 |
| iPS:435797 | 21-225_181G2 | NA | CGGGCGAGTCAGGGCATTAG CAATTATTTAGCC | GCTGCATCCAGTTTGCAA AGT | CAACAGTATAATGGTTACCC ATTCACT |
| | | | SEQ ID NO:2541 | SEQ ID NO:10553 | SEQ ID NO:18565 |
| | | AA | RASQGISNYLA | AASSLQS | QQYNGYPFT |
| | | | SEQ ID NO:2542 | SEQ ID NO:10554 | SEQ ID NO:18566 |

FIGURE 49

| iPS:435799 | | NA | CGGGCAAGTCACAGCATTAGCAACTATTTAAAT | ACTACATTGAATTTGCAAAGT | CAACAGAGTTACAGTTCTCCTCCGTGGACG |
|---|---|---|---|---|---|
| | 21-225_181G3 | | SEQ ID NO:2543 | SEQ ID NO:10555 | SEQ ID NO:18567 |
| | | AA | RASHSISNYLN | TTLNLQS | QQSYSSPPWT |
| | | | SEQ ID NO:2544 | SEQ ID NO:10556 | SEQ ID NO:18568 |
| iPS:435801 | | NA | AAGTCCAGCCAGAGTGTTTTACACAGCTCCAACAATTACAACTACTTAACT | TGGGCATCTACCCGGGAATCC | CATCAGTATTTTATTACTCCGTGGACG |
| | 21-225_181E5 | | SEQ ID NO:2545 | SEQ ID NO:10557 | SEQ ID NO:18569 |
| | | AA | KSSQSVLHSSNNYNYLT | WASTRES | HQYFITPWT |
| | | | SEQ ID NO:2546 | SEQ ID NO:10558 | SEQ ID NO:18570 |
| iPS:435805 | | NA | CGGGCAAGTCAGGGCATTAGAAATGATTTAGGC | ACTGCATCCAGTTTGCAAAGT | CTACAGCATAATAGTTACCCATTCACT |
| | 21-225_181A8 | | SEQ ID NO:2547 | SEQ ID NO:10559 | SEQ ID NO:18571 |
| | | AA | RASQGIRNDLG | TASSLQS | LQHNSYPFT |
| | | | SEQ ID NO:2548 | SEQ ID NO:10560 | SEQ ID NO:18572 |
| iPS:435807 | | NA | AAGTCTAGTCAGAGCCTCCTGCATGGTGATGGAAAGACCTATTTGTAT | GAAGTTTCCAATCGGTTCTCT | ATGCAAAGTATACAGATTCCCTGGACG |
| | 21-225_181C10 | | SEQ ID NO:2549 | SEQ ID NO:10561 | SEQ ID NO:18573 |
| | | AA | KSSQSLLHGDGKTYLY | EVSNRFS | MQSIQIPWT |
| | | | SEQ ID NO:2550 | SEQ ID NO:10562 | SEQ ID NO:18574 |
| iPS:435809 | | NA | CGGGCGAGTCAGGATATTACCAGCTGGTTAGCC | GCTGCATCCAGTTTACAAAGT | CAACAGGTTAACAGTTTCCCATTCACT |
| | 21-225_182H5 | | SEQ ID NO:2551 | SEQ ID NO:10563 | SEQ ID NO:18575 |
| | | AA | RASQDITSWLA | AASSLQS | QQVNSFPFT |
| | | | SEQ ID NO:2552 | SEQ ID NO:10564 | SEQ ID NO:18576 |
| iPS:435811 | | NA | CAGGCGAGTCAGGACATTAGCAACTATTTAAAT | GATGCATCCAATTTGGAAACA | CAACAGTATGATAATCTCCCTCTCACT |
| | 21-225_183H6 | | SEQ ID NO:2553 | SEQ ID NO:10565 | SEQ ID NO:18577 |

FIGURE 49

| | | | AA | QASQDISNYLN | DASNLET | QQYDNLPLT |
|---|---|---|---|---|---|---|
| | | | | SEQ ID NO:2554 | SEQ ID NO:10566 | SEQ ID NO:18578 |
| iPS:435813 | | NA | CGGGCAAGTCGGAACATCAGCAACTATTTAAAT | GTTGTATCCAGTTTGCAAAGT | CAACAGAGTTACAGTTCCCCTCCGTGGACG |
| | 21-225_183A12 | | | SEQ ID NO:2555 | SEQ ID NO:10567 | SEQ ID NO:18579 |
| | | AA | RASRNISNYLN | VVSSLQS | QQSYSSPPWT |
| | | | | SEQ ID NO:2556 | SEQ ID NO:10568 | SEQ ID NO:18580 |
| iPS:435815 | | NA | AGGGCCAGTCCGAGTGTTAGCAGCAGATTCTTAGCC | GGTGCATCCAGCAGGGCCACT | CAGCAGTATGGTAGCTCACCTCCGTGGACG |
| | 21-225_190G10 | | | SEQ ID NO:2557 | SEQ ID NO:10569 | SEQ ID NO:18581 |
| | | AA | RASPSVSSRFLA | GASSRAT | QQYGSSPPWT |
| | | | | SEQ ID NO:2558 | SEQ ID NO:10570 | SEQ ID NO:18582 |
| iPS:435817 | | NA | CGGGCCAGTCAGAGTATTGGTAGTAACTTACAC | TCTGCTTCCCAGTCCTTCTCA | CAGCAGAGTAGTAGTTTACCGTGGACG |
| | 21-225_190B11 | | | SEQ ID NO:2559 | SEQ ID NO:10571 | SEQ ID NO:18583 |
| | | AA | RASQSIGSNLH | SASQSFS | QQSSSLPWT |
| | | | | SEQ ID NO:2560 | SEQ ID NO:10572 | SEQ ID NO:18584 |
| iPS:435819 | | NA | CGGACGAGTCAGGGCATTGGCAATTATTTAGCC | AAAACATCCAGTTTACAAAGT | CAACAGTATATGACTTACCCGCTCACT |
| | 21-225_190C11 | | | SEQ ID NO:2561 | SEQ ID NO:10573 | SEQ ID NO:18585 |
| | | AA | RTSQGIGNYLA | KTSSLQS | QQYMTYPLT |
| | | | | SEQ ID NO:2562 | SEQ ID NO:10574 | SEQ ID NO:18586 |
| iPS:435821 | | NA | AGGGCCAGTCAGAGTTTTCGCATCAACTTAGCC | GGTGCATCCACCAGGGCCACT | CAGCAGTATAATAACTGGCCGCTCACT |
| | 21-225_190E11 | | | SEQ ID NO:2563 | SEQ ID NO:10575 | SEQ ID NO:18587 |
| | | AA | RASQSFRINLA | GASTRAT | QQYNNWPLT |
| | | | | SEQ ID NO:2564 | SEQ ID NO:10576 | SEQ ID NO:18588 |
| iPS:435823 | | NA | CGGGCCAGTCAGAACATTGGTAGTAGCTTACAC | TATGCTTCCCAGTCCTTCTCA | CATCAGAGTAGTAGTTTCCCTCGGACG |
| | 21-225_190F11 | | | SEQ ID NO:2565 | SEQ ID NO:10577 | SEQ ID NO:18589 |

FIGURE 49

| | | | | | |
|---|---|---|---|---|---|
| | | AA | RASQNIGSSLH | YASQSFS | HQSSSFPRT |
| | | | SEQ ID NO:2566 | SEQ ID NO:10578 | SEQ ID NO:18590 |
| iPS:435825 | | NA | CGGACGAGTCAGGGCATTGGCAATTATTTAGCC | AAAGCATCCAGTTTGCAAAGT | CAACAGTATATGACTTACCCGCTCACT |
| | 21-225_190G11 | | SEQ ID NO:2567 | SEQ ID NO:10579 | SEQ ID NO:18591 |
| | | AA | RTSQGIGNYLA | KASSLQS | QQYMTYPLT |
| | | | SEQ ID NO:2568 | SEQ ID NO:10580 | SEQ ID NO:18592 |
| iPS:435827 | | NA | AAGTCTAGTCAGAGCCTCCTCCATAGTGATGGAAGGACCTATTTGTAT | GAGGTTTCCAACCGGTTCGCT | ATGCAAAGTATACAGTTTCCCTGGACG |
| | 21-225_190H11 | | SEQ ID NO:2569 | SEQ ID NO:10581 | SEQ ID NO:18593 |
| | | AA | KSSQSLLHSDGRTYLY | EVSNRFA | MQSIQFPWT |
| | | | SEQ ID NO:2570 | SEQ ID NO:10582 | SEQ ID NO:18594 |
| iPS:435829 | | NA | CGGGCCAGTCAGAGTATTGGTAGTAGCTTACAC | TATGCTTCCCAGTCCTTCTCA | CATCAGACTAGAAGTTTACCTCTCACT |
| | 21-225_190B12 | | SEQ ID NO:2571 | SEQ ID NO:10583 | SEQ ID NO:18595 |
| | | AA | RASQSIGSSLH | YASQSFS | HQTRSLPLT |
| | | | SEQ ID NO:2572 | SEQ ID NO:10584 | SEQ ID NO:18596 |
| iPS:435831 | | NA | CGGGCAAGTCAGGGCATTAGAAAAGATTTAGGC | ACTGCATCCAGTTTGCAAAGT | CTACAGCATAATAGTTACCCGTGGACG |
| | 21-225_190C12 | | SEQ ID NO:2573 | SEQ ID NO:10585 | SEQ ID NO:18597 |
| | | AA | RASQGIRKDLG | TASSLQS | LQHNSYPWT |
| | | | SEQ ID NO:2574 | SEQ ID NO:10586 | SEQ ID NO:18598 |
| iPS:435833 | | NA | CGGGCGAGTCAGGGCATTAGCAATTATTTAGCC | GTTGCATCCACTTTGCAATCA | CAAAAGTATAACAGTGCCCCATTCACT |
| | 21-225_190D12 | | SEQ ID NO:2575 | SEQ ID NO:10587 | SEQ ID NO:18599 |
| | | AA | RASQGISNYLA | VASTLQS | QKYNSAPFT |
| | | | SEQ ID NO:2576 | SEQ ID NO:10588 | SEQ ID NO:18600 |
| iPS:435835 | | NA | CGGGCGAGTCAGGGCATTGGCAAGTATTTAGCC | GCTGCATCCAGTTTGCAAAGT | CAACGATATGATACTTACCCATTCACT |

FIGURE 49

| | 21-225_190F12 | | SEQ ID NO:2577 | SEQ ID NO:10589 | SEQ ID NO:18601 |
|---|---|---|---|---|---|
| | | AA | RASQGIGKYLA | AASSLQS | QRYDTYPFT |
| | | | SEQ ID NO:2578 | SEQ ID NO:10590 | SEQ ID NO:18602 |
| iPS:435837 | | NA | CGGACGAGTCAGGGCATTGG CAAGTATTTAGCC | AAAGCATCCAGTTTGCA AGGT | CAACAGTATATGACTTACCC GCTCACT |
| | 21-225_198G3 | | SEQ ID NO:2579 | SEQ ID NO:10591 | SEQ ID NO:18603 |
| | | AA | RTSQGIGKYLA | KASSLQG | QQYMTYPLT |
| | | | SEQ ID NO:2580 | SEQ ID NO:10592 | SEQ ID NO:18604 |
| iPS:435839 | | NA | AGGTCTAGTCAGAGCCTCCT GCATAGTGATGGAAAGACCT ATTTGTTT | GAACTTTCCAACCGGTTC TCT | ATGCAAAGTTTCCAGCTTCC CTGGACG |
| | 21-225_191B1 | | SEQ ID NO:2581 | SEQ ID NO:10593 | SEQ ID NO:18605 |
| | | AA | RSSQSLLHSDGKTYLF | ELSNRFS | MQSFQLPWT |
| | | | SEQ ID NO:2582 | SEQ ID NO:10594 | SEQ ID NO:18606 |
| iPS:435841 | | NA | AGGTCCAGCCAGAGTGTTTT ACACAGCTCCAACAATTACA ACTACTTAGCT | TGGGCATCTACCCGGGA ATCC | CAACAATATTATAGTACTCC TCCGACG |
| | 21-225_191D8 | | SEQ ID NO:2583 | SEQ ID NO:10595 | SEQ ID NO:18607 |
| | | AA | RSSQSVLHSSNNYNYLA | WASTRES | QQYYSTPPT |
| | | | SEQ ID NO:2584 | SEQ ID NO:10596 | SEQ ID NO:18608 |
| iPS:435843 | | NA | AGGGCCAGTCAGAGTATTAG CCTCAACTTCTTAGCC | GGTGCATCCAGCAGGGC CACT | CAGCAGTATGGTAGGTCACC GTGGACG |
| | 21-225_191F1 | | SEQ ID NO:2585 | SEQ ID NO:10597 | SEQ ID NO:18609 |
| | | AA | RASQSISLNFLA | GASSRAT | QQYGRSPWT |
| | | | SEQ ID NO:2586 | SEQ ID NO:10598 | SEQ ID NO:18610 |
| iPS:435845 | | NA | CGGGCGAGTCAGGGCATTAG CAATTATTTAGCC | GCTGCATCCAGTTTGCAA AGT | CAACATTATCTTACTTACCCT CTCACT |
| | 21-225_191G1 | | SEQ ID NO:2587 | SEQ ID NO:10599 | SEQ ID NO:18611 |
| | | AA | RASQGISNYLA | AASSLQS | QHYLTYPLT |
| | | | SEQ ID NO:2588 | SEQ ID NO:10600 | SEQ ID NO:18612 |

FIGURE 49

| iPS:435847 | | NA | AGGGCCAGTCAGAGTATTCG CAGCAGCTTCTTAGCC | GGTGCATCCAGCAGGGC CACT | CAGCAATATGGTAACTCACC GTGGGCG |
|---|---|---|---|---|---|
| | 21-225_191A3 | | SEQ ID NO:2589 | SEQ ID NO:10601 | SEQ ID NO:18613 |
| | | AA | RASQSIRSSFLA | GASSRAT | QQYGNSPWA |
| | | | SEQ ID NO:2590 | SEQ ID NO:10602 | SEQ ID NO:18614 |
| iPS:435849 | | NA | AGGGCCAGTCAGAGTATTCG CAGCAGCTTCTTAGCC | GGTGCATCCAGCAGGGC CACT | CAGCAGTATGGTAACTCACC GTGGGCG |
| | 21-225_191C3 | | SEQ ID NO:2591 | SEQ ID NO:10603 | SEQ ID NO:18615 |
| | | AA | RASQSIRSSFLA | GASSRAT | QQYGNSPWA |
| | | | SEQ ID NO:2592 | SEQ ID NO:10604 | SEQ ID NO:18616 |
| iPS:435851 | | NA | AGGGCCGGTCAAAGTATTAG AACCAACTTCTTAGCC | GGTGCATCCAGCAGGGC CACT | CAGCAGTATGGTAGCTCACC GTGGACG |
| | 21-225_191D3 | | SEQ ID NO:2593 | SEQ ID NO:10605 | SEQ ID NO:18617 |
| | | AA | RAGQSIRTNFLA | GASSRAT | QQYGSSPWT |
| | | | SEQ ID NO:2594 | SEQ ID NO:10606 | SEQ ID NO:18618 |
| iPS:435853 | | NA | AAGTCTAGTCAGAGCCTCCT CCATAGTGATGGAAGGACCT ATTTGTAT | GAGGTTTCCAACCGGTTC GCT | ATGCAAAGTATACAACTTCC CTGGACG |
| | 21-225_191E3 | | SEQ ID NO:2595 | SEQ ID NO:10607 | SEQ ID NO:18619 |
| | | AA | KSSQSLLHSDGRTYLY | EVSNRFA | MQSIQLPWT |
| | | | SEQ ID NO:2596 | SEQ ID NO:10608 | SEQ ID NO:18620 |
| iPS:435855 | | NA | AAGTCCAGCCAGAGTGTTTT ACACAGCTCCAACAGTTACA ACTACTTAGCT | TGGGCATCTACCCGAAA ATCC | CAGCAATATTATAGTAGTCC TCCCACT |
| | 21-225_191G3 | | SEQ ID NO:2597 | SEQ ID NO:10609 | SEQ ID NO:18621 |
| | | AA | KSSQSVLHSSNSYNYLA | WASTRKS | QQYYSSPPT |
| | | | SEQ ID NO:2598 | SEQ ID NO:10610 | SEQ ID NO:18622 |
| iPS:435857 | | NA | CGGGCAAGTCAGGGCATTAG AAAAGATTTAGGC | ACTGCATCCAGTTTGCAA AAT | CTACAGCATAATAGTTACCC GTGGACG |
| | 21-225_191A4 | | SEQ ID NO:2599 | SEQ ID NO:10611 | SEQ ID NO:18623 |

FIGURE 49

| | | AA | RASQGIRKDLG | TASSLQN | LQHNSYPWT |
|---|---|---|---|---|---|
| | | | SEQ ID NO:2600 | SEQ ID NO:10612 | SEQ ID NO:18624 |
| iPS:435859 | | NA | CGGACGAGTCAGGGCATTGG CAATTATTTAGCC | AAAGCATCCAGTTTGCA AAGT | CAACAGTATATGACTTACCC GCTCACT |
| | 21-225_190E6 | | SEQ ID NO:2601 | SEQ ID NO:10613 | SEQ ID NO:18625 |
| | | AA | RTSQGIGNYLA | KASSLQS | QQYMTYPLT |
| | | | SEQ ID NO:2602 | SEQ ID NO:10614 | SEQ ID NO:18626 |
| iPS:435861 | | NA | CGGGCGAGTCAGGGGATTG GCAATCATTTAGCC | GCTGCATCCAGTTTGCAA AGT | CAACAGTATAGTAATTACCC AGTCACT |
| | 21-225_190A5 | | SEQ ID NO:2603 | SEQ ID NO:10615 | SEQ ID NO:18627 |
| | | AA | RASQGIGNHLA | AASSLQS | QQYSNYPVT |
| | | | SEQ ID NO:2604 | SEQ ID NO:10616 | SEQ ID NO:18628 |
| iPS:435863 | | NA | CGGGCCAATCAGAGCATTGG TAGTAGCTTACAC | TATGCTTCCCAGTCCCTC TCA | CATCAGACTGGTAGGTTACC TCTCACT |
| | 21-225_191H4 | | SEQ ID NO:2605 | SEQ ID NO:10617 | SEQ ID NO:18629 |
| | | AA | RANQSIGSSLH | YASQSLS | HQTGRLPLT |
| | | | SEQ ID NO:2606 | SEQ ID NO:10618 | SEQ ID NO:18630 |
| iPS:435865 | | NA | AGGGCCAGTCAGAGTGTTAG CAGCAGGTTCTTAGCC | GGTGCTTCCAACAGGGC CACT | CAGCAGTATGGTGGTTCACC TCCGTGGACG |
| | 21-225_191A5 | | SEQ ID NO:2607 | SEQ ID NO:10619 | SEQ ID NO:18631 |
| | | AA | RASQSVSSRFLA | GASNRAT | QQYGGSPPWT |
| | | | SEQ ID NO:2608 | SEQ ID NO:10620 | SEQ ID NO:18632 |
| iPS:435867 | | NA | CGGGCCAGTCAGAGCATTGG TAGTAGCTTACAC | TATGCTTCCCAGTCCTTC TCA | CATCAGAGTAGTAGTTTCCC TCGGACG |
| | 21-225_191E5 | | SEQ ID NO:2609 | SEQ ID NO:10621 | SEQ ID NO:18633 |
| | | AA | RASQSIGSSLH | YASQSFS | HQSSSFPRT |
| | | | SEQ ID NO:2610 | SEQ ID NO:10622 | SEQ ID NO:18634 |
| iPS:435869 | | NA | CGGGCGAGTCAGGGCATTAG AAATTATTTAGCC | GTTGCATCCAGTTTGGAA AGT | CAACAGTATCTTAATTACCC AGTCACT |
| | 21-225_190B1 | | SEQ ID NO:2611 | SEQ ID NO:10623 | SEQ ID NO:18635 |

FIGURE 49

| | | AA | RASQGIRNYLA | VASSLES | QQYLNYPVT |
|---|---|---|---|---|---|
| | | | SEQ ID NO:2612 | SEQ ID NO:10624 | SEQ ID NO:18636 |
| iPS:435871 | 21-225_191E6 | NA | AAGTCTAGTCAGAGCCTCCTCCATAGTGATGGAAGGACCTATTTGTAT | GAGGTTTCCAACCGGTTCGCT | ATGCAAAGTATACATTTTCCCTGGACG |
| | | | SEQ ID NO:2613 | SEQ ID NO:10625 | SEQ ID NO:18637 |
| | | AA | KSSQSLLHSDGRTYLY | EVSNRFA | MQSIHFPWT |
| | | | SEQ ID NO:2614 | SEQ ID NO:10626 | SEQ ID NO:18638 |
| iPS:435873 | 21-225_190G4 | NA | CGGGCGAGTCAGGACATTGGCAGATATTTAGCC | GCTGCATCCAGTTTGCAAAGT | CAACAATATAGTACTTACCCGCTCACT |
| | | | SEQ ID NO:2615 | SEQ ID NO:10627 | SEQ ID NO:18639 |
| | | AA | RASQDIGRYLA | AASSLQS | QQYSTYPLT |
| | | | SEQ ID NO:2616 | SEQ ID NO:10628 | SEQ ID NO:18640 |
| iPS:435875 | 21-225_190B9 | NA | CGGGCGAGTCAGGGTATTAACAACTGGTTAGCC | GGTGTTTCCAGTTTGCAGAGT | CAACAGGCTAACAGTTTCCCGTGGACG |
| | | | SEQ ID NO:2617 | SEQ ID NO:10629 | SEQ ID NO:18641 |
| | | AA | RASQGINNWLA | GVSSLQS | QQANSFPWT |
| | | | SEQ ID NO:2618 | SEQ ID NO:10630 | SEQ ID NO:18642 |
| iPS:435877 | 21-225_184E7 | NA | CGGGCGAGTCAGGGCATTAGCAATTATTTAGCC | GCTGCATCCAGTTTGCAAAGT | CAACAGTATAATGGTTACCCATTCACT |
| | | | SEQ ID NO:2619 | SEQ ID NO:10631 | SEQ ID NO:18643 |
| | | AA | RASQGISNYLA | AASSLQS | QQYNGYPFT |
| | | | SEQ ID NO:2620 | SEQ ID NO:10632 | SEQ ID NO:18644 |
| iPS:435879 | 21-225_184H10 | NA | CGGGCAAGTCAGGGCATTAGAAATGATTTAGGC | ATTGCATCCAGTTTGCAAAGT | CTACAGCATAATAGTTACCCATTCACT |
| | | | SEQ ID NO:2621 | SEQ ID NO:10633 | SEQ ID NO:18645 |
| | | AA | RASQGIRNDLG | IASSLQS | LQHNSYPFT |
| | | | SEQ ID NO:2622 | SEQ ID NO:10634 | SEQ ID NO:18646 |
| iPS:435881 | | NA | CGGGCAAGTCAGGGCATTAGAAATGATTTAGGC | ATTGCATCCAGTTTGCAAAGT | CTACAGCATAATAGTTACCCATTCACT |

FIGURE 49

| | 21-225_184D11 | | SEQ ID NO:2623 | SEQ ID NO:10635 | SEQ ID NO:18647 |
|---|---|---|---|---|---|
| | | AA | RASQGIRNDLG | IASSLQS | LQHNSYPFT |
| | | | SEQ ID NO:2624 | SEQ ID NO:10636 | SEQ ID NO:18648 |
| iPS:435883 | | NA | CGGGCGAGTCAGGGCATTAGCAATTATTTAGCC | GTTGCATCCAGTTTGCAAAGT | CGACAATATCATAGTTACCCATTCACT |
| | 21-225_185A1 | | SEQ ID NO:2625 | SEQ ID NO:10637 | SEQ ID NO:18649 |
| | | AA | RASQGISNYLA | VASSLQS | RQYHSYPFT |
| | | | SEQ ID NO:2626 | SEQ ID NO:10638 | SEQ ID NO:18650 |
| iPS:435885 | | NA | CGGGCGAGTCAGGGCATTAGCAATTATTTAGCC | GCTGCATCCAGTTTGCAAAGT | CAACAGTATAATGGTTACCCATTCACT |
| | 21-225_185E10 | | SEQ ID NO:2627 | SEQ ID NO:10639 | SEQ ID NO:18651 |
| | | AA | RASQGISNYLA | AASSLQS | QQYNGYPFT |
| | | | SEQ ID NO:2628 | SEQ ID NO:10640 | SEQ ID NO:18652 |
| iPS:435887 | | NA | AAGTCTAGTCAGAGCCTCCTGCATGGTGATGGAAAGACCTATTTGTGT | GAAGTTTCCAAGCGGTTCTCT | ATGCAAAGTATACAGGTTCCCTGGACG |
| | 21-225_186F7 | | SEQ ID NO:2629 | SEQ ID NO:10641 | SEQ ID NO:18653 |
| | | AA | KSSQSLLHGDGKTYLC | EVSKRFS | MQSIQVPWT |
| | | | SEQ ID NO:2630 | SEQ ID NO:10642 | SEQ ID NO:18654 |
| iPS:435889 | | NA | CGGGCGAGTCAGGATATTACCAGCTGGTTAGCC | GCTGCATCCAGTTTACAAAGT | CAACAGGTTAACAGTTTCCCATTCACT |
| | 21-225_186A11 | | SEQ ID NO:2631 | SEQ ID NO:10643 | SEQ ID NO:18655 |
| | | AA | RASQDITSWLA | AASSLQS | QQVNSFPFT |
| | | | SEQ ID NO:2632 | SEQ ID NO:10644 | SEQ ID NO:18656 |
| iPS:435891 | | NA | CGGGCGAGTCAGGGCATTAGCAATTATTTAGCC | GCTGCTTCCAGTTTGCAAAGT | CAACAGTATAATAGTTATCCATTCACT |
| | 21-225_188H5 | | SEQ ID NO:2633 | SEQ ID NO:10645 | SEQ ID NO:18657 |
| | | AA | RASQGISNYLA | AASSLQS | QQYNSYPFT |
| | | | SEQ ID NO:2634 | SEQ ID NO:10646 | SEQ ID NO:18658 |

FIGURE 49

| iPS:435895 | | NA | CGGGCGAATCAGGATATTTCCAGCTGGTTAGCC | GCTGCATCCAATTTGCAAAGT | CAGCAGGCTAACAGTTTCCCGTGGACG |
| | 21-225_188E8 | | SEQ ID NO:2635 | SEQ ID NO:10647 | SEQ ID NO:18659 |
| | | AA | RANQDISSWLA | AASNLQS | QQANSFPWT |
| | | | SEQ ID NO:2636 | SEQ ID NO:10648 | SEQ ID NO:18660 |
| iPS:435897 | | NA | CGGGCGAGTCAGGGCATTAGCAATTATTTAGCC | GCTGCATCCAGTTTGCAAAGT | CAACAGTATAATAGTTACCCATTCACT |
| | 21-225_188B9 | | SEQ ID NO:2637 | SEQ ID NO:10649 | SEQ ID NO:18661 |
| | | AA | RASQGISNYLA | AASSLQS | QQYNSYPFT |
| | | | SEQ ID NO:2638 | SEQ ID NO:10650 | SEQ ID NO:18662 |
| iPS:435899 | | NA | ATGTCTAGTCAGAGCCTCCTGCATGGTGATGGAAAGACCTATTTATAT | GAAGTTTCCAACCGGTTCTCT | ATGCAAAGTATACAGATTCCTTGGACG |
| | 21-225_188G11 | | SEQ ID NO:2639 | SEQ ID NO:10651 | SEQ ID NO:18663 |
| | | AA | MSSQSLLHGDGKTYLY | EVSNRFS | MQSIQIPWT |
| | | | SEQ ID NO:2640 | SEQ ID NO:10652 | SEQ ID NO:18664 |
| iPS:435901 | | NA | AAGTCTAGTCAGAGCCTCCTGCATGGTGATGGAAAGACCTATTTGTTT | GAAGTTTCCAACCGGTTCTCT | ATGCAAAGTATACAGATTCCGTGGACG |
| | 21-225_189G2 | | SEQ ID NO:2641 | SEQ ID NO:10653 | SEQ ID NO:18665 |
| | | AA | KSSQSLLHGDGKTYLF | EVSNRFS | MQSIQIPWT |
| | | | SEQ ID NO:2642 | SEQ ID NO:10654 | SEQ ID NO:18666 |
| iPS:435903 | | NA | AAGTCCAGCCAGAGTGTTTTATTCAACTCCAACAATAAGAACTACTTAGCT | TGGGCATCTACCCGGGAATCC | CAGCAATATTGTAGTCTTCCATTCACT |
| | 21-225_190E2 | | SEQ ID NO:2643 | SEQ ID NO:10655 | SEQ ID NO:18667 |
| | | AA | KSSQSVLFNSNNKNYLA | WASTRES | QQYCSLPFT |
| | | | SEQ ID NO:2644 | SEQ ID NO:10656 | SEQ ID NO:18668 |
| iPS:435905 | | NA | AGGGCCAGTCAGAATATAAGGAGCAACTTCTTAGCC | GGTGCATCCAGCAGGGCCACT | CAGCAGTATGGTAACTCACCGTGGACG |

FIGURE 49

| | 21-225_190A3 | | SEQ ID NO:2645 | SEQ ID NO:10657 | SEQ ID NO:18669 |
|---|---|---|---|---|---|
| | | AA | RASQNIRSNFLA | GASSRAT | QQYGNSPWT |
| | | | SEQ ID NO:2646 | SEQ ID NO:10658 | SEQ ID NO:18670 |
| iPS:435907 | | NA | CGGGCAAGTCAGGGCATTAG AAAAGATTTAGGC | ACTGCATCCAGTTTGCAA AGT | CTACAGCATAATAGTTACCC GTGGACG |
| | 21-225_190G3 | | SEQ ID NO:2647 | SEQ ID NO:10659 | SEQ ID NO:18671 |
| | | AA | RASQGIRKDLG | TASSLQS | LQHNSYPWT |
| | | | SEQ ID NO:2648 | SEQ ID NO:10660 | SEQ ID NO:18672 |
| iPS:435909 | | NA | CGGGCGAGTCAGGGTCTTAA CAACTGGTTAGCC | GCTGTGTCCAGTTTGCAA AGT | CAACAGGCTAACAGTCTCCC GTGGACG |
| | 21-225_190H3 | | SEQ ID NO:2649 | SEQ ID NO:10661 | SEQ ID NO:18673 |
| | | AA | RASQGLNNWLA | AVSSLQS | QQANSLPWT |
| | | | SEQ ID NO:2650 | SEQ ID NO:10662 | SEQ ID NO:18674 |
| iPS:435911 | | NA | AGGGCCAGTCAGAGTATTCG CAGCAGCTTCTTAGCC | GGTGCATCCAGCAGGGC CACT | CAGCAGTATGGTAACTCACC GTGGGCG |
| | 21-225_190B4 | | SEQ ID NO:2651 | SEQ ID NO:10663 | SEQ ID NO:18675 |
| | | AA | RASQSIRSSFLA | GASSRAT | QQYGNSPWA |
| | | | SEQ ID NO:2652 | SEQ ID NO:10664 | SEQ ID NO:18676 |
| iPS:435913 | | NA | AGGGCCAGTCAGAGTGTTAG AAGCAACTTCTTAGCC | GGTGCATACCGCAGGGC CACT | CAGCAGTATGGTAACTCACC GTGGACG |
| | 21-225_190A7 | | SEQ ID NO:2653 | SEQ ID NO:10665 | SEQ ID NO:18677 |
| | | AA | RASQSVRSNFLA | GAYRRAT | QQYGNSPWT |
| | | | SEQ ID NO:2654 | SEQ ID NO:10666 | SEQ ID NO:18678 |
| iPS:435915 | | NA | AAGTCCAGCCAGAGTGTTTT ACACAGCTCCAACAATTACA ACTACTTAGCT | TGGGCATCTACCCGGGA ATCC | CAGCAATATTATAGTATTCC TCCCACT |
| | 21-225_190H4 | | SEQ ID NO:2655 | SEQ ID NO:10667 | SEQ ID NO:18679 |
| | | AA | KSSQSVLHSSNNYNYLA | WASTRES | QQYYSIPPT |
| | | | SEQ ID NO:2656 | SEQ ID NO:10668 | SEQ ID NO:18680 |

FIGURE 49

| iPS:435917 | | NA | CGGGCCAGTCAGAGTATTGG TAGTAACTTACAC | TCTGCTTCCCAGTCCTTC TCA | CAGCAGAGTAGTAGTTTACC GTGGACG |
|---|---|---|---|---|---|
| | 21-225_190D5 | | SEQ ID NO:2657 | SEQ ID NO:10669 | SEQ ID NO:18681 |
| | | AA | RASQSIGSNLH | SASQSFS | QQSSSLPWT |
| | | | SEQ ID NO:2658 | SEQ ID NO:10670 | SEQ ID NO:18682 |
| iPS:435919 | | NA | CGGGCAAGTCAGGGCATTAG AAAAGATTTAGGC | ACTGCATCCAGTTTGCAA AGT | CTACAGCATAATAATTACCC GTGGACG |
| | 21-225_190H5 | | SEQ ID NO:2659 | SEQ ID NO:10671 | SEQ ID NO:18683 |
| | | AA | RASQGIRKDLG | TASSLQS | LQHNNYPWT |
| | | | SEQ ID NO:2660 | SEQ ID NO:10672 | SEQ ID NO:18684 |
| iPS:435921 | | NA | CGGGCAAGTCAGGGCATTAG AAATGATTTAGGC | GCTGCATCCAGTTTGCAA AGT | CTACAGCATTATAGTTTCCC ATTCACT |
| | 21-225_190D6 | | SEQ ID NO:2661 | SEQ ID NO:10673 | SEQ ID NO:18685 |
| | | AA | RASQGIRNDLG | AASSLQS | LQHYSFPFT |
| | | | SEQ ID NO:2662 | SEQ ID NO:10674 | SEQ ID NO:18686 |
| iPS:435923 | | NA | AAGTCCAGCCAGAGTGTTTT ATTCAACTCCAACAATAAGA ACTACTTAGCT | TGGGCATCTACCCGGGA ATCC | CAGCAATATTGTAGTCTTCC ATTCACT |
| | 21-225_190H6 | | SEQ ID NO:2663 | SEQ ID NO:10675 | SEQ ID NO:18687 |
| | | AA | KSSQSVLFNSNNKNYLA | WASTRES | QQYCSLPFT |
| | | | SEQ ID NO:2664 | SEQ ID NO:10676 | SEQ ID NO:18688 |
| iPS:435925 | | NA | AAGTCCAGCCAGAGTGTTTT ATCCAGCTCCAACAATTACA ACTATTTAGTT | TGGGCATCTACCCGGAA ATCC | CAACAATATTATCGTACTCC GTGGACG |
| | 21-225_190D7 | | SEQ ID NO:2665 | SEQ ID NO:10677 | SEQ ID NO:18689 |
| | | AA | KSSQSVLSSSNNYNYLV | WASTRKS | QQYYRTPWT |
| | | | SEQ ID NO:2666 | SEQ ID NO:10678 | SEQ ID NO:18690 |
| iPS:435927 | 21 225 190E7 | NA | AAGTCTAGTCAGAGCCTCCT CCATAGTGATGGAAGGACCT ATTTGTAT | GAGGTTTCCAACCGGTTC TCT | ATGCAAAGTATACAGCTTCC CTGGACG |

FIGURE 49

| | | | | | |
|---|---|---|---|---|---|
| | 21-225_190E7 | | SEQ ID NO:2667 | SEQ ID NO:10679 | SEQ ID NO:18691 |
| | | AA | KSSQSLLHSDGRTYLY | EVSNRFS | MQSIQLPWT |
| iPS:435929 | | | SEQ ID NO:2668 | SEQ ID NO:10680 | SEQ ID NO:18692 |
| | 21-225_190D9 | NA | CGGGCCAGTCAGAGCATTGGTAGTAGCTTACAC | TATGCTTCCCAGTCCTTCTCA | CATCAGAGTAGTAGTTTCCCTCGGACG |
| | | | SEQ ID NO:2669 | SEQ ID NO:10681 | SEQ ID NO:18693 |
| | | AA | RASQSIGSSLH | YASQSFS | HQSSSFPRT |
| iPS:435933 | | | SEQ ID NO:2670 | SEQ ID NO:10682 | SEQ ID NO:18694 |
| | 21-225_190F8 | NA | CGGACGAGTCAGGGCATTGGCAATTATTTAGCC | AAAGCATCCAGTTTACAAAGT | CAACAGTATATGACTTACCCACTCACT |
| | | | SEQ ID NO:2671 | SEQ ID NO:10683 | SEQ ID NO:18695 |
| | | AA | RTSQGIGNYLA | KASSLQS | QQYMTYPLT |
| iPS:435935 | | | SEQ ID NO:2672 | SEQ ID NO:10684 | SEQ ID NO:18696 |
| | 21-225_190H8 | NA | CGGGCCAGTCAGAGCATTGGTAGTAGCTTACAC | TATGCTTCCCAGTCCTTCTCA | CATCAGAGTAGTAGTTTCCCTCGGACG |
| | | | SEQ ID NO:2673 | SEQ ID NO:10685 | SEQ ID NO:18697 |
| | | AA | RASQSIGSSLH | YASQSFS | HQSSSFPRT |
| iPS:435937 | | | SEQ ID NO:2674 | SEQ ID NO:10686 | SEQ ID NO:18698 |
| | 21-225_190H9 | NA | CGGGCGAGTCAGGGCATTGGCAAGTATTTAGCC | GCTGCATCCAGTTTGCAAAGT | CAACGATATGATACTTACCCATTCACT |
| | | | SEQ ID NO:2675 | SEQ ID NO:10687 | SEQ ID NO:18699 |
| | | AA | RASQGIGKYLA | AASSLQS | QRYDTYPFT |
| iPS:435939 | | | SEQ ID NO:2676 | SEQ ID NO:10688 | SEQ ID NO:18700 |
| | 21-225_191H7 | NA | AGGGCCGGTCAAAGTATTAGAACCGACTTCTTAGCC | GGTCCATCCAGCAGGGCCACT | CAGCAGTATGGTAGCTCACCGTGGACG |
| | | | SEQ ID NO:2677 | SEQ ID NO:10689 | SEQ ID NO:18701 |
| | | AA | RAGQSIRTDFLA | GPSSRAT | QQYGSSPWT |
| iPS:435941 | | | SEQ ID NO:2678 | SEQ ID NO:10690 | SEQ ID NO:18702 |
| | | NA | AGGCCCAGTCAGAGTTTTAGCAGAAACTTAGCC | GGTGCATCCACTAGGGCCACT | CAGCAGTATAATAACTGGCCGCTCACT |

FIGURE 49

| | 21-225_191E8 | | SEQ ID NO:2679 | SEQ ID NO:10691 | SEQ ID NO:18703 |
|---|---|---|---|---|---|
| | | AA | RPSQSFSRNLA | GASTRAT | QQYNNWPLT |
| | | | SEQ ID NO:2680 | SEQ ID NO:10692 | SEQ ID NO:18704 |
| iPS:435943 | | NA | CGGGCCAGTCAGAGTATTGGTAGTAGTTTACAC | TATGCTTCCCAGTCCTTCTCA | CATCAGACTAGAAGTTTACCTCTCACT |
| | 21-225_191C9 | | SEQ ID NO:2681 | SEQ ID NO:10693 | SEQ ID NO:18705 |
| | | AA | RASQSIGSSLH | YASQSFS | HQTRSLPLT |
| | | | SEQ ID NO:2682 | SEQ ID NO:10694 | SEQ ID NO:18706 |
| iPS:435945 | | NA | CGGGCGAGTCAGGGCATTGGCAAGTATTTAGCC | GCTGCATCCAGTTTGCAAAGT | CAACAGTATAGTACTTACCCGCTCACT |
| | 21-225_191A10 | | SEQ ID NO:2683 | SEQ ID NO:10695 | SEQ ID NO:18707 |
| | | AA | RASQGIGKYLA | AASSLQS | QQYSTYPLT |
| | | | SEQ ID NO:2684 | SEQ ID NO:10696 | SEQ ID NO:18708 |
| iPS:435947 | | NA | CGGGCGAGTCAGGGCATTGGCAAGTATTTAGCC | GCTGCATCCAGTTTGCAAAGT | CAACAGTATAGTACTTACCCGCTCACT |
| | 21-225_191E10 | | SEQ ID NO:2685 | SEQ ID NO:10697 | SEQ ID NO:18709 |
| | | AA | RASQGIGKYLA | AASSLQS | QQYSTYPLT |
| | | | SEQ ID NO:2686 | SEQ ID NO:10698 | SEQ ID NO:18710 |
| iPS:435953 | | NA | AAGTCCAGCCAGAGTGTTTTATTCAACTCCAACAATAAGAACTACTTAGCT | TGGGCCTCTACCCGGGAATCC | CAGCAATATTCTAGTCTTCCATTCACT |
| | 21-225_191B12 | | SEQ ID NO:2687 | SEQ ID NO:10699 | SEQ ID NO:18711 |
| | | AA | KSSQSVLFNSNNKNYLA | WASTRES | QQYSSLPFT |
| | | | SEQ ID NO:2688 | SEQ ID NO:10700 | SEQ ID NO:18712 |
| iPS:435957 | | NA | CGGACGAGTCAGGGCATTGGCAATTATTTAGCC | AAAGCATCCAGTTTGCAAAGT | CAACAGTATATCACTTACCCGCTCACT |
| | 21-225_191G12 | | SEQ ID NO:2689 | SEQ ID NO:10701 | SEQ ID NO:18713 |
| | | AA | RTSQGIGNYLA | KASSLQS | QQYITYPLT |
| | | | SEQ ID NO:2690 | SEQ ID NO:10702 | SEQ ID NO:18714 |

FIGURE 49

| iPS:435961 | | NA | CGGGCGAATCAGGGCATTAACAATTATTTAGCC | GCTGCATCCAGTTTGCAAAGT | CAACAGTATAATAGTTACCCATTCACT |
|---|---|---|---|---|---|
| | 21-225_192A2 | | SEQ ID NO:2691 | SEQ ID NO:10703 | SEQ ID NO:18715 |
| | | AA | RANQGINNYLA | AASSLQS | QQYNSYPFT |
| | | | SEQ ID NO:2692 | SEQ ID NO:10704 | SEQ ID NO:18716 |
| iPS:435963 | | NA | CGGGCGAGTCAGGGCATTAGCAATTATTTAGCC | GCTGCATCCAGTTTGCAAAGT | CAACATTATGTTACTTACCCGAACACT |
| | 21-225_192D2 | | SEQ ID NO:2693 | SEQ ID NO:10705 | SEQ ID NO:18717 |
| | | AA | RASQGISNYLA | AASSLQS | QHYVTYPNT |
| | | | SEQ ID NO:2694 | SEQ ID NO:10706 | SEQ ID NO:18718 |
| iPS:435965 | | NA | CGGGCGAGTCAGGGTATAAGTAGTTGGATAGCC | GGTGCATCCAGTTTGCAAAGT | CAACAGTCTAACAGTTTCCCATTCACT |
| | 21-225_192H2 | | SEQ ID NO:2695 | SEQ ID NO:10707 | SEQ ID NO:18719 |
| | | AA | RASQGISSWIA | GASSLQS | QQSNSFPFT |
| | | | SEQ ID NO:2696 | SEQ ID NO:10708 | SEQ ID NO:18720 |
| iPS:435967 | | NA | AGGGCCAGTCAGAGTGTTCGCAGCAGCTTCCTTGCC | GGTGCATCTAGCAGGGCCACT | CAGCAGTATGGTAACTCACCGTGGGCG |
| | 21-225_192B3 | | SEQ ID NO:2697 | SEQ ID NO:10709 | SEQ ID NO:18721 |
| | | AA | RASQSVRSSFLA | GASSRAT | QQYGNSPWA |
| | | | SEQ ID NO:2698 | SEQ ID NO:10710 | SEQ ID NO:18722 |
| iPS:435971 | | NA | CGGGCGAGTCAGGGCATTAGCAATTATTTAGCC | GCTGCATCCAGTTTGCAAAGT | CTACATTATCTTACTTACCCTCTCACT |
| | 21-225_192D3 | | SEQ ID NO:2699 | SEQ ID NO:10711 | SEQ ID NO:18723 |
| | | AA | RASQGISNYLA | AASSLQS | LHYLTYPLT |
| | | | SEQ ID NO:2700 | SEQ ID NO:10712 | SEQ ID NO:18724 |
| iPS:435973 | | NA | AGGGCCAGTCAGAGTGTTAGCAGCAACTTCTTAGCC | GGTGCATCCAGCAGGGCCACT | CAGCAATATGGTATCTCACCGTGGACG |
| | 21-225_192H3 | | SEQ ID NO:2701 | SEQ ID NO:10713 | SEQ ID NO:18725 |
| | | AA | RASQSVSSNFLA | GASSRAT | QQYGISPWT |
| | | | SEQ ID NO:2702 | SEQ ID NO:10714 | SEQ ID NO:18726 |

FIGURE 49

| iPS:435977 | | NA | CGGGCGAGTCAGGGCATTGG CAATTATTTAGCC | GTTGTATCCAGTTTACAA AGT | CAACGGTATGATACTTACCC ATTCACT |
|---|---|---|---|---|---|
| | 21-225_192E4 | | SEQ ID NO:2703 | SEQ ID NO:10715 | SEQ ID NO:18727 |
| | | AA | RASQGIGNYLA | VVSSLQS | QRYDTYPFT |
| | | | SEQ ID NO:2704 | SEQ ID NO:10716 | SEQ ID NO:18728 |
| iPS:435979 | | NA | CGGGCGAGTCAGGACATTAG CAATTATTTAGCC | GCTGCATCCAGTTTGCAA AGT | CTACATTATCTCAATTACCC GCTCACT |
| | 21-225_192H4 | | SEQ ID NO:2705 | SEQ ID NO:10717 | SEQ ID NO:18729 |
| | | AA | RASQDISNYLA | AASSLQS | LHYLNYPLT |
| | | | SEQ ID NO:2706 | SEQ ID NO:10718 | SEQ ID NO:18730 |
| iPS:435983 | | NA | CGGGCCAGTCAGAGCATTGG TAGGAGTTTACAC | TATGCTTCCCAGTCATTC TCA | CATCAGAGTAGTCGTTTACC GCTCACT |
| | 21-225_192E5 | | SEQ ID NO:2707 | SEQ ID NO:10719 | SEQ ID NO:18731 |
| | | AA | RASQSIGRSLH | YASQSFS | HQSSRLPLT |
| | | | SEQ ID NO:2708 | SEQ ID NO:10720 | SEQ ID NO:18732 |
| iPS:435985 | | NA | CGGGCAAGTCAGGGCATTAG AAATGATTTAGGC | GCTGCATCCAGTTTGCAA AGT | CTACAGCATTATAGTTTCCC ATTCACT |
| | 21-225_192F6 | | SEQ ID NO:2709 | SEQ ID NO:10721 | SEQ ID NO:18733 |
| | | AA | RASQGIRNDLG | AASSLQS | LQHYSFPFT |
| | | | SEQ ID NO:2710 | SEQ ID NO:10722 | SEQ ID NO:18734 |
| iPS:435987 | | NA | CGGACGAGTCAGGGCATTGG CAATTATTTAGCC | AAAGCATCCAGTTTGCA AAGT | CAACAGTATATGACTTACCC GCTCACT |
| | 21-225_192G6 | | SEQ ID NO:2711 | SEQ ID NO:10723 | SEQ ID NO:18735 |
| | | AA | RTSQGIGNYLA | KASSLQS | QQYMTYPLT |
| | | | SEQ ID NO:2712 | SEQ ID NO:10724 | SEQ ID NO:18736 |
| iPS:435989 | | NA | CGGGCAAGTCAGGGCATTAG AAAAGATTTAGGC | ACTGCATCCAGTTTGCAA AGT | CTACAGCATACTAGTTACCC GTGGACG |
| | 21-225_192F7 | | SEQ ID NO:2713 | SEQ ID NO:10725 | SEQ ID NO:18737 |
| | | AA | RASQGIRKDLG | TASSLQS | LQHTSYPWT |
| | | | SEQ ID NO:2714 | SEQ ID NO:10726 | SEQ ID NO:18738 |

810

FIGURE 49

| iPS:435993 | | NA | CGGGCGAGTCAGGGCATTAG CAATTATTTAGCC | GCTGCATCCAGTTTGCAA AGT | CAACATTATCTTACTTACCCT CTCACT |
|---|---|---|---|---|---|
| | 21-225_192C8 | | SEQ ID NO:2715 | SEQ ID NO:10727 | SEQ ID NO:18739 |
| | | AA | RASQGISNYLA | AASSLQS | QHYLTYPLT |
| | | | SEQ ID NO:2716 | SEQ ID NO:10728 | SEQ ID NO:18740 |
| iPS:435995 | | NA | AGGGCCAGTCAGAGTATTAG CAGCAGCTACTTAGCC | GGTGCATCCAGCAGGGC CACT | CAGCAGTATGAGAGTTCACC GTGGACG |
| | 21-225_192F8 | | SEQ ID NO:2717 | SEQ ID NO:10729 | SEQ ID NO:18741 |
| | | AA | RASQSISSSYLA | GASSRAT | QQYESSPWT |
| | | | SEQ ID NO:2718 | SEQ ID NO:10730 | SEQ ID NO:18742 |
| iPS:435997 | | NA | CGGACGAGTCAGGGCATTGG CAATTATTTAGCC | AAAGCATCCAGTTTGCA AAGT | CAACAGTATATCACTTACCC GCTCACT |
| | 21-225_192G8 | | SEQ ID NO:2719 | SEQ ID NO:10731 | SEQ ID NO:18743 |
| | | AA | RTSQGIGNYLA | KASSLQS | QQYITYPLT |
| | | | SEQ ID NO:2720 | SEQ ID NO:10732 | SEQ ID NO:18744 |
| iPS:435999 | | NA | AAGTCTAGTCAGAGCCTCCT CCATAGTGATGGAAGGACCT ATTTGTAT | GAGGTTTCCAACCGGTTC GCT | ATGCAAAGTATACAGCTTCC CTGGACG |
| | 21-225_192F9 | | SEQ ID NO:2721 | SEQ ID NO:10733 | SEQ ID NO:18745 |
| | | AA | KSSQSLLHSDGRTYLY | EVSNRFA | MQSIQLPWT |
| | | | SEQ ID NO:2722 | SEQ ID NO:10734 | SEQ ID NO:18746 |
| iPS:436001 | | NA | CGGGCGAGTCAGGGCATTGG CAAGTATTTAGCC | GCTGCATCCAGTTTGCAA AGT | CAACGATATGATACTTACCC ATTCACT |
| | 21-225_192C10 | | SEQ ID NO:2723 | SEQ ID NO:10735 | SEQ ID NO:18747 |
| | | AA | RASQGIGKYLA | AASSLQS | QRYDTYPFT |
| | | | SEQ ID NO:2724 | SEQ ID NO:10736 | SEQ ID NO:18748 |
| iPS:436003 | | NA | CGGGCAAGTCAGAGCATTAG CAACTATTTAAAT | GCTGAATCCAGTTTACA AAGT | CAACAGAGTTACAGTTCCCC TCCGTGGACG |
| | 21-225_192G10 | | SEQ ID NO:2725 | SEQ ID NO:10737 | SEQ ID NO:18749 |
| | | AA | RASQSISNYLN | AESSLQS | QQSYSSPPWT |

FIGURE 49

| | | | SEQ ID NO:2726 | SEQ ID NO:10738 | SEQ ID NO:18750 |
|---|---|---|---|---|---|
| iPS:436005 | | NA | CGGGCGAGTCAGGGCATTGGCAAGTATTTAGCC | GCTGCATCCAGTTTGCAAAGT | CAACAGTATAGTACTTACCCGCTCACT |
| | 21-225_192H10 | | SEQ ID NO:2727 | SEQ ID NO:10739 | SEQ ID NO:18751 |
| | | AA | RASQGIGKYLA | AASSLQS | QQYSTYPLT |
| | | | SEQ ID NO:2728 | SEQ ID NO:10740 | SEQ ID NO:18752 |
| iPS:436007 | | NA | AGGGCCAGTCAGAGTGTCAGAAGCGACTTCTTAGCC | GGTGTATCCCGCAGGGCCACT | CAGCAGTATGGTAACTCACCGTGGACG |
| | 21-225_192G12 | | SEQ ID NO:2729 | SEQ ID NO:10741 | SEQ ID NO:18753 |
| | | AA | RASQSVRSDFLA | GVSRRAT | QQYGNSPWT |
| | | | SEQ ID NO:2730 | SEQ ID NO:10742 | SEQ ID NO:18754 |
| iPS:436009 | | NA | AGGGCCAGTCAGAGTGTTAGAAGCAACTTCTTAGCC | GGTGCATCCCGCAGGGCCACT | CAGCAGTATGGTAACTCACCGTGGACG |
| | 21-225_193A1 | | SEQ ID NO:2731 | SEQ ID NO:10743 | SEQ ID NO:18755 |
| | | AA | RASQSVRSNFLA | GASRRAT | QQYGNSPWT |
| | | | SEQ ID NO:2732 | SEQ ID NO:10744 | SEQ ID NO:18756 |
| iPS:436011 | | NA | AGGGCCAGTCAGAGTGTTAGAAGCAACTTCTTAGCC | GGTGCATCCCGCAGGGCCACT | CAGCAGTATGGTAACTCACCGTGGACG |
| | 21-225_193B1 | | SEQ ID NO:2733 | SEQ ID NO:10745 | SEQ ID NO:18757 |
| | | AA | RASQSVRSNFLA | GASRRAT | QQYGNSPWT |
| | | | SEQ ID NO:2734 | SEQ ID NO:10746 | SEQ ID NO:18758 |
| iPS:436013 | | NA | CGGGCGAGTCAGGGTATTAACAACTGGTTAGCC | GGTGTTTCCAGTTTGCAAAGT | CAACAGGCTAACAGTTTCCCGTGGACG |
| | 21-225_193F2 | | SEQ ID NO:2735 | SEQ ID NO:10747 | SEQ ID NO:18759 |
| | | AA | RASQGINNWLA | GVSSLQS | QQANSFPWT |
| | | | SEQ ID NO:2736 | SEQ ID NO:10748 | SEQ ID NO:18760 |
| iPS:436015 | | NA | AGGGCCAGTCAGAGTATTCGCAGCAGCTTCTTAGCC | GGTGCATCCAACAGGGCCACT | CAGCAGTATGGTAACTCACCGTGGGCG |
| | 21-225_193D3 | | SEQ ID NO:2737 | SEQ ID NO:10749 | SEQ ID NO:18761 |
| | | AA | RASQSIRSSFLA | GASNRAT | QQYGNSPWA |

FIGURE 49

| | | | SEQ ID NO:2738 | SEQ ID NO:10750 | SEQ ID NO:18762 |
|---|---|---|---|---|---|
| iPS:436017 | | NA | AGGGCCGGTCAAAGTATTAG AACCGACTTCTTAGTC | GGTGCATCCAGCAGGGC CACT | CAGCAGTATGGAAGCTCACC GTGGACG |
| | 21-225_193F3 | | SEQ ID NO:2739 | SEQ ID NO:10751 | SEQ ID NO:18763 |
| | | AA | RAGQSIRTDFLV | GASSRAT | QQYGSSPWT |
| | | | SEQ ID NO:2740 | SEQ ID NO:10752 | SEQ ID NO:18764 |
| iPS:436019 | | NA | CGGCCGAGTCAGGGCATTAG CATTTATTTAGCC | GCTGCATCCACTTTACAA TCA | CAAAAGTATAACAGTGCCCC ATTCACT |
| | 21-225_193C4 | | SEQ ID NO:2741 | SEQ ID NO:10753 | SEQ ID NO:18765 |
| | | AA | RPSQGISIYLA | AASTLQS | QKYNSAPFT |
| | | | SEQ ID NO:2742 | SEQ ID NO:10754 | SEQ ID NO:18766 |
| iPS:436021 | | NA | AAGTCCAGCCAGAGTGTTTT ATACAGCTCCAACAATTATA ACTACTTGACT | TGGGCATCTACCCGAAA ATCC | CAGCAATATTATATTACTCC GTGGACG |
| | 21-225_193G4 | | SEQ ID NO:2743 | SEQ ID NO:10755 | SEQ ID NO:18767 |
| | | AA | KSSQSVLYSSNNYNYLT | WASTRKS | QQYYITPWT |
| | | | SEQ ID NO:2744 | SEQ ID NO:10756 | SEQ ID NO:18768 |
| iPS:436023 | | NA | CGGGCAAGTCAGGGCATTAG AAATGATTTAGGG | GCTGCATCCAGTTTGCAA AGT | CTACAGCATAATGATTTCCC GTTCACT |
| | 21-225_193A5 | | SEQ ID NO:2745 | SEQ ID NO:10757 | SEQ ID NO:18769 |
| | | AA | RASQGIRNDLG | AASSLQS | LQHNDFPFT |
| | | | SEQ ID NO:2746 | SEQ ID NO:10758 | SEQ ID NO:18770 |
| iPS:436025 | | NA | CGGGCCAGTCAGAGCATTGG TAGTAGCTTACAC | TATGCTTCCCAGTCCTTC TCA | CATCAGAGTAGTCGTTTACC ATTCACT |
| | 21-225_193B5 | | SEQ ID NO:2747 | SEQ ID NO:10759 | SEQ ID NO:18771 |
| | | AA | RASQSIGSSLH | YASQSFS | HQSSRLPFT |
| | | | SEQ ID NO:2748 | SEQ ID NO:10760 | SEQ ID NO:18772 |
| iPS:436027 | | NA | AGGGCCAGTCAGAGTGTTAG GAGCGGTTACTTAGCC | GGTGCATCCAGCAGGGC CACT | CAGCAGTATGAGAGTTCACC GTGGACG |
| | 21-225_193E6 | | SEQ ID NO:2749 | SEQ ID NO:10761 | SEQ ID NO:18773 |

FIGURE 49

| | | | | | |
|---|---|---|---|---|---|
| | | AA | RASQSVRSGYLA | GASSRAT | QQYESSPWT |
| | | | SEQ ID NO:2750 | SEQ ID NO:10762 | SEQ ID NO:18774 |
| iPS:436029 | | NA | AGGGCCGGTCAAAGTATTAG AACCAACTTCTTAGCC | GGTGCATCCAGCAGGGC CACT | CAGCAGTATGGTAGCTCACC GTGGACG |
| | 21-225_193H6 | | SEQ ID NO:2751 | SEQ ID NO:10763 | SEQ ID NO:18775 |
| | | AA | RAGQSIRTNFLA | GASSRAT | QQYGSSPWT |
| | | | SEQ ID NO:2752 | SEQ ID NO:10764 | SEQ ID NO:18776 |
| iPS:436031 | | NA | CGGGCGAGTCAGGGCATTGG CAAGTATTTAGCC | GCTGCATCCAGTTTGCAA AGT | CAACAGTATAGTACTTACCC GCTCACT |
| | 21-225_193C7 | | SEQ ID NO:2753 | SEQ ID NO:10765 | SEQ ID NO:18777 |
| | | AA | RASQGIGKYLA | AASSLQS | QQYSTYPLT |
| | | | SEQ ID NO:2754 | SEQ ID NO:10766 | SEQ ID NO:18778 |
| iPS:436033 | | NA | CGGGCAAGTCAGGGCATTAG AAATGATTTAGGC | TCTGCATCCAGTTTGCAA AGG | CTACAGCATAAAAGGTACCC GCTCACT |
| | 21-225_193E7 | | SEQ ID NO:2755 | SEQ ID NO:10767 | SEQ ID NO:18779 |
| | | AA | RASQGIRNDLG | SASSLQR | LQHKRYPLT |
| | | | SEQ ID NO:2756 | SEQ ID NO:10768 | SEQ ID NO:18780 |
| iPS:436035 | | NA | AGGGCCAGTCAGAGTATTCG CAGCAGCTTCTTAGCC | GGTGCATCCAGCAGGGC CACT | CAGCAATATGGTAACTCACC GTGGGCG |
| | 21-225_193C8 | | SEQ ID NO:2757 | SEQ ID NO:10769 | SEQ ID NO:18781 |
| | | AA | RASQSIRSSFLA | GASSRAT | QQYGNSPWA |
| | | | SEQ ID NO:2758 | SEQ ID NO:10770 | SEQ ID NO:18782 |
| iPS:436037 | | NA | AGGGCCAGTCAGAGTATAA GGACCAACTTCTTAGCC | GGTGCATCCAGCAGGGC CACT | CAGCAGTATGGTAACTCACC GTGGACG |
| | 21-225_193D8 | | SEQ ID NO:2759 | SEQ ID NO:10771 | SEQ ID NO:18783 |
| | | AA | RASQSIRTNFLA | GASSRAT | QQYGNSPWT |
| | | | SEQ ID NO:2760 | SEQ ID NO:10772 | SEQ ID NO:18784 |
| iPS:436039 | | NA | CGGGCGAGTCAGGGCGTTAG CAATCATTTAGCC | GCTGCATCCAGTTTGCAA AGT | CAACAGTATAATAGTTACCC ATTCACT |
| | 21-225_193F8 | | SEQ ID NO:2761 | SEQ ID NO:10773 | SEQ ID NO:18785 |

FIGURE 49

| | | | | | |
|---|---|---|---|---|---|
| | | AA | RASQGVSNHLA | AASSLQS | QQYNSYPFT |
| | | | SEQ ID NO:2762 | SEQ ID NO:10774 | SEQ ID NO:18786 |
| iPS:436041 | | NA | AGGGCCAGTCAGAGTGTTAG AACCAACTTCTTAGCC | GGTGCATCCCGCAGGGC CACT | CAGCAGTATGGTAACTCACC GTGGACG |
| | 21-225_193G8 | | SEQ ID NO:2763 | SEQ ID NO:10775 | SEQ ID NO:18787 |
| | | AA | RASQSVRTNFLA | GASRRAT | QQYGNSPWT |
| | | | SEQ ID NO:2764 | SEQ ID NO:10776 | SEQ ID NO:18788 |
| iPS:436043 | | NA | CGGGCCAGTCAGAGCATTGG TAGGAGTTTACAC | TATGCTTCCCAGTCATTC TCA | CATCAGAGTAGTCGTTTACC GCTCACT |
| | 21-225_193G9 | | SEQ ID NO:2765 | SEQ ID NO:10777 | SEQ ID NO:18789 |
| | | AA | RASQSIGRSLH | YASQSFS | HQSSRLPLT |
| | | | SEQ ID NO:2766 | SEQ ID NO:10778 | SEQ ID NO:18790 |
| iPS:436045 | | NA | CGGGCGAGTCAGGGCATTAG CAATTATTTAGCC | GCTGCATCCAGTTTGCAA AGT | CAACATTATCTTACTTACCCT CTCACT |
| | 21-225_193A10 | | SEQ ID NO:2767 | SEQ ID NO:10779 | SEQ ID NO:18791 |
| | | AA | RASQGISNYLA | AASSLQS | QHYLTYPLT |
| | | | SEQ ID NO:2768 | SEQ ID NO:10780 | SEQ ID NO:18792 |
| iPS:436047 | | NA | AGGGCCAGTCCGAGTGTTAG CAGCAGCTACTTAGCC | GGTGCATCCAGGAGGGC CACT | CAGCAGTATGGTAGCTCACC TCCGTGGACG |
| | 21-225_193B10 | | SEQ ID NO:2769 | SEQ ID NO:10781 | SEQ ID NO:18793 |
| | | AA | RASPSVSSSYLA | GASRRAT | QQYGSSPPWT |
| | | | SEQ ID NO:2770 | SEQ ID NO:10782 | SEQ ID NO:18794 |
| iPS:436049 | | NA | AGGGCCAGTCAGAGTATTCG CAGCAGCTTCTTAGCC | GATGCATCCAGCAGGGC CACT | CAGCAGTATGGTAACTCACC GTGGGCG |
| | 21-225_193B12 | | SEQ ID NO:2771 | SEQ ID NO:10783 | SEQ ID NO:18795 |
| | | AA | RASQSIRSSFLA | DASSRAT | QQYGNSPWA |
| | | | SEQ ID NO:2772 | SEQ ID NO:10784 | SEQ ID NO:18796 |
| iPS:436051 | | NA | AGGGCCAGTCAGAGTGTTAG CAGCAGCTTAGCC | GGTGCATCCACCAGGGC CACT | CAGCAGTATAATAACTGGCC GTGCAGT |
| | 21-225_193G12 | | SEQ ID NO:2773 | SEQ ID NO:10785 | SEQ ID NO:18797 |

FIGURE 49

| | | | | | |
|---|---|---|---|---|---|
| | | AA | RASQSVSSSLA | GASTRAT | QQYNNWPCS |
| | | | SEQ ID NO:2774 | SEQ ID NO:10786 | SEQ ID NO:18798 |
| iPS:436054 | | NA | CGGGCGAGTCAGGGCATTAGCAATTATTTAGCC | GCTGCATCCAGTTTGCAAAGT | CTACATTATCTTACTTACCCTCTCACT |
| | 21-225_194C1 | | SEQ ID NO:2775 | SEQ ID NO:10787 | SEQ ID NO:18799 |
| | | AA | RASQGISNYLA | AASSLQS | LHYLTYPLT |
| | | | SEQ ID NO:2776 | SEQ ID NO:10788 | SEQ ID NO:18800 |
| iPS:436056 | | NA | CGGGCCAGTCAGAGTATTGGTAGTAACTTACAC | TCTGCTTCCCAGTCCTTCTCA | CAGCAGAGTAGTAGTTTACCGTGGACG |
| | 21-225_194C3 | | SEQ ID NO:2777 | SEQ ID NO:10789 | SEQ ID NO:18801 |
| | | AA | RASQSIGSNLH | SASQSFS | QQSSSLPWT |
| | | | SEQ ID NO:2778 | SEQ ID NO:10790 | SEQ ID NO:18802 |
| iPS:436058 | | NA | AGGGCCAGTCGGGGTGTTAGCAACATCTACTTAGCC | GGTGCTTCCAACAGGGCCACT | CAGCACAATGATTACTCAATGTTCACT |
| | 21-225_194A4 | | SEQ ID NO:2779 | SEQ ID NO:10791 | SEQ ID NO:18803 |
| | | AA | RASRGVSNIYLA | GASNRAT | QHNDYSMFT |
| | | | SEQ ID NO:2780 | SEQ ID NO:10792 | SEQ ID NO:18804 |
| iPS:436060 | | NA | AAGTCTAGTCAGAGCCTCCTCCATAGTGATGGAAGGACCTATTTGTAT | GAGGTTTCCAACCGGTTCTCT | ATGCAAAGTATACAGCTTCCCTGGACG |
| | 21-225_194F4 | | SEQ ID NO:2781 | SEQ ID NO:10793 | SEQ ID NO:18805 |
| | | AA | KSSQSLLHSDGRTYLY | EVSNRFS | MQSIQLPWT |
| | | | SEQ ID NO:2782 | SEQ ID NO:10794 | SEQ ID NO:18806 |
| iPS:436062 | | NA | AGGGCCGGTCAAAGTATTAGAACCAACTTCTTAGCC | GGTGCATCCAGCAGGGCCACT | CAGCAGTATGGTAGTTCACCGTGGACG |
| | 21-225_194E5 | | SEQ ID NO:2783 | SEQ ID NO:10795 | SEQ ID NO:18807 |
| | | AA | RAGQSIRTNFLA | GASSRAT | QQYGSSPWT |
| | | | SEQ ID NO:2784 | SEQ ID NO:10796 | SEQ ID NO:18808 |
| iPS:436064 | | NA | AGGGCCAGTCAGAGTATTAGAAGCAACTTCTTAGCC | GGTGCATCCAGCAGGGCCACT | CAGCAGTATGGTAGCTCACCGTGGACG |

FIGURE 49

| | 21-225_194E6 | | SEQ ID NO:2785 | SEQ ID NO:10797 | SEQ ID NO:18809 |
|---|---|---|---|---|---|
| | | AA | RASQSIRSNFLA | GASSRAT | QQYGSSPWT |
| | | | SEQ ID NO:2786 | SEQ ID NO:10798 | SEQ ID NO:18810 |
| iPS:436066 | 21-225_194B7 | NA | CGGGCGAGTCAGGGCATTAG CAAATATTTAGCC | GGTGCATCCAGGTTGCA AAGT | CAACATTATCTTAATTACCCT CTCACC |
| | | | SEQ ID NO:2787 | SEQ ID NO:10799 | SEQ ID NO:18811 |
| | | AA | RASQGISKYLA | GASRLQS | QHYLNYPLT |
| | | | SEQ ID NO:2788 | SEQ ID NO:10800 | SEQ ID NO:18812 |
| iPS:436068 | 21-225_194F7 | NA | CGGGCGAGTCAGGGTATTAG CAGGTGGTTAGCC | GCTGCATCCAGTTTGCAA AGT | CAACAGGCTAACAGTTTCCC GTGGACG |
| | | | SEQ ID NO:2789 | SEQ ID NO:10801 | SEQ ID NO:18813 |
| | | AA | RASQGISRWLA | AASSLQS | QQANSFPWT |
| | | | SEQ ID NO:2790 | SEQ ID NO:10802 | SEQ ID NO:18814 |
| iPS:436072 | 21-225_194C10 | NA | AGGGCCAGTCCGAGTGTTAA CAGCGGCTACTTAGCC | GGTGCATCCAGCAGGGC CACT | CAGCAGTATGAAAGCTCACC GTGGACG |
| | | | SEQ ID NO:2791 | SEQ ID NO:10803 | SEQ ID NO:18815 |
| | | AA | RASPSVNSGYLA | GASSRAT | QQYESSPWT |
| | | | SEQ ID NO:2792 | SEQ ID NO:10804 | SEQ ID NO:18816 |
| iPS:436074 | 21-225_194F10 | NA | CGGGCAAGTCAGGGCATTAG AAATGATTTAGGC | GCTGCATCCAGTTTACAA AGT | CTACAGCATTATAGTTTCCC ATTCACT |
| | | | SEQ ID NO:2793 | SEQ ID NO:10805 | SEQ ID NO:18817 |
| | | AA | RASQGIRNDLG | AASSLQS | LQHYSFPFT |
| | | | SEQ ID NO:2794 | SEQ ID NO:10806 | SEQ ID NO:18818 |
| iPS:436076 | 21-225_194H11 | NA | CGGGCGAGTCAGGGCATTAG CAATTATTTAGCC | GCTGCATCCAGTTTACAA AGT | CAACATTATCTTACTTACCCT CTCACT |
| | | | SEQ ID NO:2795 | SEQ ID NO:10807 | SEQ ID NO:18819 |
| | | AA | RASQGISNYLA | AASSLQS | QHYLTYPLT |
| | | | SEQ ID NO:2796 | SEQ ID NO:10808 | SEQ ID NO:18820 |
| iPS:436078 | | NA | CGGGCGAGTCAGGGCATTGG CAAGTATTTAGCA | GCTGCATCCAGTTTGCAA AGT | CAACGATATGATACTTACCC ATTCACT |

FIGURE 49

| | 21-225_194H12 | | SEQ ID NO:2797 | SEQ ID NO:10809 | SEQ ID NO:18821 |
|---|---|---|---|---|---|
| | | AA | RASQGIGKYLA | AASSLQS | QRYDTYPFT |
| | | | SEQ ID NO:2798 | SEQ ID NO:10810 | SEQ ID NO:18822 |
| iPS:436080 | | NA | AGGGCCAGTCCGAGTGTTAACAGTAACTACTTAGCC | GGTGCATCCAACAGGGCCACT | CAGCAGTATGAAAGCTCACCGTGGACG |
| | 21-225_195B1 | | SEQ ID NO:2799 | SEQ ID NO:10811 | SEQ ID NO:18823 |
| | | AA | RASPSVNSNYLA | GASNRAT | QQYESSPWT |
| | | | SEQ ID NO:2800 | SEQ ID NO:10812 | SEQ ID NO:18824 |
| iPS:436082 | | NA | CGGGCGAGTCAGGGTATTAGCAGGTGGTTAGCC | GCTGCATCCAGTTTGCTAGGT | CAACGGGCTAACAGTTTCCCGTGCAGT |
| | 21-225_195D9 | | SEQ ID NO:2801 | SEQ ID NO:10813 | SEQ ID NO:18825 |
| | | AA | RASQGISRWLA | AASSLLG | QRANSFPCS |
| | | | SEQ ID NO:2802 | SEQ ID NO:10814 | SEQ ID NO:18826 |
| iPS:436084 | | NA | CGGGCCAGTCAGAGCATTGGTAGTAGCTTACAC | TATGCTTCCCAGTCCTTCTCA | CATCAGAGTAGAACTTTACCGCTCACT |
| | 21-225_195F2 | | SEQ ID NO:2803 | SEQ ID NO:10815 | SEQ ID NO:18827 |
| | | AA | RASQSIGSSLH | YASQSFS | HQSRTLPLT |
| | | | SEQ ID NO:2804 | SEQ ID NO:10816 | SEQ ID NO:18828 |
| iPS:436086 | | NA | CGGACGAGTCAGGGCATTGGCAAGTATTTAGCC | AAAGTATCCAGTTTGCAAAGT | CAACAGTATATGACTTACCCGCTCACT |
| | 21-225_191G10 | | SEQ ID NO:2805 | SEQ ID NO:10817 | SEQ ID NO:18829 |
| | | AA | RTSQGIGKYLA | KVSSLQS | QQYMTYPLT |
| | | | SEQ ID NO:2806 | SEQ ID NO:10818 | SEQ ID NO:18830 |
| iPS:436088 | | NA | AGGGCCAGTCAGAGTATTCGCAGCAGCTTCTTAGCC | GGTGCATTTAGTAGGGCCACT | CAGCAGTATGGTAACTCACCGTGGGCG |
| | 21-225_195C8 | | SEQ ID NO:2807 | SEQ ID NO:10819 | SEQ ID NO:18831 |
| | | AA | RASQSIRSSFLA | GAFSRAT | QQYGNSPWA |
| | | | SEQ ID NO:2808 | SEQ ID NO:10820 | SEQ ID NO:18832 |
| iPS:436090 | | NA | CGGGCGAGTCAGGGCATTAGCAATTATTTAGCC | GCTGCATCCAGTTTGCAAAGT | CAACATTATCTTACTTACCCTCTCACT |

FIGURE 49

| | 21-225_195A9 | | SEQ ID NO:2809 | SEQ ID NO:10821 | SEQ ID NO:18833 |
|---|---|---|---|---|---|
| | | AA | RASQGISNYLA | AASSLQS | QHYLTYPLT |
| | | | SEQ ID NO:2810 | SEQ ID NO:10822 | SEQ ID NO:18834 |
| iPS:436092 | | NA | CGGGCAAGTCAGGGCATAA GAAAAGATTTAGGC | GCTGCATCCGATTTGCAA AGT | CTACAGCATTATCGTTACCC ATTCACT |
| | 21-225_195B9 | | SEQ ID NO:2811 | SEQ ID NO:10823 | SEQ ID NO:18835 |
| | | AA | RASQGIRKDLG | AASDLQS | LQHYRYPFT |
| | | | SEQ ID NO:2812 | SEQ ID NO:10824 | SEQ ID NO:18836 |
| iPS:436094 | | NA | CGGGCCAGTCAGAGCATTGG TAGTAGCTTACAC | TATGCTTCCCAGTCCTTC TCA | CATCAGAGTGGTCGTTACC GCTCACT |
| | 21-225_195B10 | | SEQ ID NO:2813 | SEQ ID NO:10825 | SEQ ID NO:18837 |
| | | AA | RASQSIGSSLH | YASQSFS | HQSGRLPLT |
| | | | SEQ ID NO:2814 | SEQ ID NO:10826 | SEQ ID NO:18838 |
| iPS:436096 | | NA | CGGGCCAGTCAGAGCATTGG TAGTAGCTTACAC | TATGCTTCCCAGTCCTTC TCA | CATCAGAGTAGTCGTTACC ATTCACT |
| | 21-225_195E10 | | SEQ ID NO:2815 | SEQ ID NO:10827 | SEQ ID NO:18839 |
| | | AA | RASQSIGSSLH | YASQSFS | HQSSRLPFT |
| | | | SEQ ID NO:2816 | SEQ ID NO:10828 | SEQ ID NO:18840 |
| iPS:436098 | | NA | AAGTCCAGCCAGAGTGTTTT ATTCAACTCCAACAATAAGA ACTACTTAGCT | TGGGCATCTACCCTGGA ATCT | CAGCAATATTGTAGTTTTCC ATTCACT |
| | 21-225_195G11 | | SEQ ID NO:2817 | SEQ ID NO:10829 | SEQ ID NO:18841 |
| | | AA | KSSQSVLFNSNNKNYLA | WASTLES | QQYCSFPFT |
| | | | SEQ ID NO:2818 | SEQ ID NO:10830 | SEQ ID NO:18842 |
| iPS:436100 | | NA | CGGGCGAGTCAGGGTATTAA CAACTGGTTAGCC | GGTGTTTCCAGTTTGCAG AGT | CAACAGGCTAACAGTTTCCC GTGGACG |
| | 21-225_195G12 | | SEQ ID NO:2819 | SEQ ID NO:10831 | SEQ ID NO:18843 |
| | | AA | RASQGINNWLA | GVSSLQS | QQANSFPWT |
| | | | SEQ ID NO:2820 | SEQ ID NO:10832 | SEQ ID NO:18844 |

FIGURE 49

| iPS:436102 | | NA | AAGTCCAGCCAGAGTATTTT ATTCAGCTCCAACAATAAGA GGTACTTAGCT | TGGGCATCTATCCGGGA ATCC | CAGCAATATTCTAGTCTTCC ATTCACT |
|---|---|---|---|---|---|
| | 21-225_196B1 | | SEQ ID NO:2821 | SEQ ID NO:10833 | SEQ ID NO:18845 |
| | | AA | KSSQSILFSSNNKRYLA | WASIRES | QQYSSLPFT |
| | | | SEQ ID NO:2822 | SEQ ID NO:10834 | SEQ ID NO:18846 |
| iPS:436104 | | NA | AAGTCCAGCCAGAGTGTTTT ATTCAACTCCAACAATAAGA ACTACTTAGCT | TGGGCATCTACCCTGGA ATCT | CAGCAATATTGTAGTTTTCC ATTCACT |
| | 21-225_196C1 | | SEQ ID NO:2823 | SEQ ID NO:10835 | SEQ ID NO:18847 |
| | | AA | KSSQSVLFNSNNKNYLA | WASTLES | QQYCSFPFT |
| | | | SEQ ID NO:2824 | SEQ ID NO:10836 | SEQ ID NO:18848 |
| iPS:436106 | | NA | CGGGCGAGTCAGGGTATTAG CAGCTGGTTAGCC | GCTGCATCCAGTTTGCAA AGT | CAACAGACTAACAGTGTCCC ATTCACT |
| | 21-225_196F2 | | SEQ ID NO:2825 | SEQ ID NO:10837 | SEQ ID NO:18849 |
| | | AA | RASQGISSWLA | AASSLQS | QQTNSVPFT |
| | | | SEQ ID NO:2826 | SEQ ID NO:10838 | SEQ ID NO:18850 |
| iPS:436110 | | NA | CGGGCAAGTCAGCGCATTCA CAGCTATTTAAAT | ACTGCATCCAGTTTGCAA GGT | CAACAGAGCTACGGTTCCCC TCTCACT |
| | 21-225_196F4 | | SEQ ID NO:2827 | SEQ ID NO:10839 | SEQ ID NO:18851 |
| | | AA | RASQRIHSYLN | TASSLQG | QQSYGSPLT |
| | | | SEQ ID NO:2828 | SEQ ID NO:10840 | SEQ ID NO:18852 |
| iPS:436112 | | NA | CGGGCGAATCAGGCCATTAG CAATTATTTAGCC | GCTGCATCCAGTTTGCAA AGT | CAACATTATCTCACTTACCCT CTCACT |
| | 21-225_196C7 | | SEQ ID NO:2829 | SEQ ID NO:10841 | SEQ ID NO:18853 |
| | | AA | RANQAISNYLA | AASSLQS | QHYLTYPLT |
| | | | SEQ ID NO:2830 | SEQ ID NO:10842 | SEQ ID NO:18854 |
| iPS:436114 | 21 225 196G8 | NA | AAGTCCAGCCAGAGTGTTTT ACACAGCTCCAACAATAACA ACTACTTAGCT | TGGGCATCTACCCGGGA ATCC | CAGCAATATTATAATACTCC TCCGACA |

FIGURE 49

| | | | | | |
|---|---|---|---|---|---|
| | 21-225_196G8 | | | SEQ ID NO:2831 | SEQ ID NO:10843 | SEQ ID NO:18855 |
| | | AA | KSSQSVLHSSNNNNYLA | WASTRES | QQYYNTPPT |
| iPS:436116 | | NA | SEQ ID NO:2832 | SEQ ID NO:10844 | SEQ ID NO:18856 |
| | 21-225_196B9 | | CGGGCGAGTCAGGGTATTAGCAACTGCTTAGCC | GCTGCATCCAGTTTGCAAAGT | CAACAGGGTGACAGTTTCCCTCCGACG |
| | | AA | SEQ ID NO:2833 | SEQ ID NO:10845 | SEQ ID NO:18857 |
| | | | RASQGISNCLA | AASSLQS | QQGDSFPPT |
| iPS:436118 | | NA | SEQ ID NO:2834 | SEQ ID NO:10846 | SEQ ID NO:18858 |
| | 21-225_196A10 | | CGGGCGAGTCAGGGTATTAGCAGGTGGTTAGCC | GCTGCATCCAGTTTGCTAGGT | CAACGGGATAACAGTTTACCGTGCAGT |
| | | AA | SEQ ID NO:2835 | SEQ ID NO:10847 | SEQ ID NO:18859 |
| | | | RASQGISRWLA | AASSLLG | QRDNSLPCS |
| iPS:436120 | | NA | SEQ ID NO:2836 | SEQ ID NO:10848 | SEQ ID NO:18860 |
| | 21-225_196C10 | | CGGGCAAGTCAGGGCATTAGAAATGATTTAGGC | GCTGCATCCAGTTTGCAAAGT | CTACAATATAATAGTTACCCTCTCACT |
| | | AA | SEQ ID NO:2837 | SEQ ID NO:10849 | SEQ ID NO:18861 |
| | | | RASQGIRNDLG | AASSLQS | LQYNSYPLT |
| iPS:436122 | | NA | SEQ ID NO:2838 | SEQ ID NO:10850 | SEQ ID NO:18862 |
| | 21-225_196G10 | | AGGGCCAGTCCGAGTGTTAGCAACAGCTTCTTAGCC | GGTGCATCCAGCAGGGCCACT | CAGCAGTATGGTAGCTCACCTCCGTGGACG |
| | | AA | SEQ ID NO:2839 | SEQ ID NO:10851 | SEQ ID NO:18863 |
| | | | RASPSVSNSFLA | GASSRAT | QQYGSSPPWT |
| iPS:436132 | | NA | SEQ ID NO:2840 | SEQ ID NO:10852 | SEQ ID NO:18864 |
| | 21-225_196C12 | | CGGGCAAGTCAGGGCATTAGAAATGATCTAGGC | GCTGCATCCAGTTTGCAAAGT | CTACAGCATAATGATTTCCCGTTCACT |
| | | AA | SEQ ID NO:2841 | SEQ ID NO:10853 | SEQ ID NO:18865 |
| | | | RASQGIRNDLG | AASSLQS | LQHNDFPFT |
| iPS:436134 | | NA | SEQ ID NO:2842 | SEQ ID NO:10854 | SEQ ID NO:18866 |
| | | | AGGGCCAGTCAGAGTGTTAGAAGCAACTTCTTAGCC | GGTGCATACCGCAGGGCCACT | CAGCAGTATGGTAACTCACCGTGGACG |

FIGURE 49

| | 21-225_196H12 | | SEQ ID NO:2843 | SEQ ID NO:10855 | SEQ ID NO:18867 |
|---|---|---|---|---|---|
| | | AA | RASQSVRSNFLA | GAYRRAT | QQYGNSPWT |
| | | | SEQ ID NO:2844 | SEQ ID NO:10856 | SEQ ID NO:18868 |
| iPS:436138 | | NA | CGGACGAGTCAGGGCATTGG CAATTATTTAGCC | AAAACATCCAGTTTGCA AAGT | CAACAATATATCACTTACCC GCTCACT |
| | 21-225_197F2 | | SEQ ID NO:2845 | SEQ ID NO:10857 | SEQ ID NO:18869 |
| | | AA | RTSQGIGNYLA | KTSSLQS | QQYITYPLT |
| | | | SEQ ID NO:2846 | SEQ ID NO:10858 | SEQ ID NO:18870 |
| iPS:436140 | | NA | CGGGCGAGTCAGGGCATTGG CAATCATTTAGCC | GCTGCATCCAGTTTGCAA AGT | CAACAGTATAGTAATTACCC GGTCACT |
| | 21-225_197G3 | | SEQ ID NO:2847 | SEQ ID NO:10859 | SEQ ID NO:18871 |
| | | AA | RASQGIGNHLA | AASSLQS | QQYSNYPVT |
| | | | SEQ ID NO:2848 | SEQ ID NO:10860 | SEQ ID NO:18872 |
| iPS:436146 | | NA | AGGGCCAGTCAGAGTATTCG CAGCAGCTTCTTAGCC | GGTGCATCCAGCAGGGC CACT | CAGCAGTATGGAAACTCACC GTGGGCG |
| | 21-225_197F4 | | SEQ ID NO:2849 | SEQ ID NO:10861 | SEQ ID NO:18873 |
| | | AA | RASQSIRSSFLA | GASSRAT | QQYGNSPWA |
| | | | SEQ ID NO:2850 | SEQ ID NO:10862 | SEQ ID NO:18874 |
| iPS:436150 | | NA | AGGTCCAGCCAGAGTGTTTT ACACAGCTTCAACAATTACA ACTACTTAGCT | TGGGCATCTACCCGGGA ATCC | CAACAATATTATAGTACTCC TCCGACG |
| | 21-225_197H4 | | SEQ ID NO:2851 | SEQ ID NO:10863 | SEQ ID NO:18875 |
| | | AA | RSSQSVLHSFNNYNYLA | WASTRES | QQYYSTPPT |
| | | | SEQ ID NO:2852 | SEQ ID NO:10864 | SEQ ID NO:18876 |
| iPS:436152 | | NA | CGGGCGAGTCAGGGCATTGG CAAATATTTAGCC | GGTGCATCCAGTTTGCA AAGT | CAACAATATAGTACTTACCC GCTCACT |
| | 21-225_197B6 | | SEQ ID NO:2853 | SEQ ID NO:10865 | SEQ ID NO:18877 |
| | | AA | RASQGIGKYLA | GASSLQS | QQYSTYPLT |
| | | | SEQ ID NO:2854 | SEQ ID NO:10866 | SEQ ID NO:18878 |

FIGURE 49

| iPS:436154 | | NA | AGGTCCAGCCAGAGTGTTTT ACACAGCTCCAACAATTACA ACTACTTAGCT | TGGGCATCTACCCGGGA ATCC | CAACAATATTATAGTACTCC TCCGACG |
|---|---|---|---|---|---|
| | 21-225_197C6 | | SEQ ID NO:2855 | SEQ ID NO:10867 | SEQ ID NO:18879 |
| | | AA | RSSQSVLHSSNNYNYLA | WASTRES | QQYYSTPPT |
| | | | SEQ ID NO:2856 | SEQ ID NO:10868 | SEQ ID NO:18880 |
| iPS:436156 | | NA | AAGTCCAGCCAGAGTGTTTT ACACAGTTCCAACAATAAGA ACTACTTAGCT | TGGGCATCTACCCGGGA ATCC | CAGCAGTCTTATACTATTCC ATTCACT |
| | 21-225_197C8 | | SEQ ID NO:2857 | SEQ ID NO:10869 | SEQ ID NO:18881 |
| | | AA | KSSQSVLHSSNNKNYLA | WASTRES | QQSYTIPFT |
| | | | SEQ ID NO:2858 | SEQ ID NO:10870 | SEQ ID NO:18882 |
| iPS:436158 | | NA | AAGTCTAGTCAGAACCTCCT GCATAGTGATGGAAAGACCT ATTTGTAT | GAAGTTTCCAACCGGTTC TCT | ATGCAAAGTATACAGCTTCC CTGGACG |
| | 21-225_197G8 | | SEQ ID NO:2859 | SEQ ID NO:10871 | SEQ ID NO:18883 |
| | | AA | KSSQNLLHSDGKTYLY | EVSNRFS | MQSIQLPWT |
| | | | SEQ ID NO:2860 | SEQ ID NO:10872 | SEQ ID NO:18884 |
| iPS:436160 | | NA | CGGGCGAGTCAGGGTATTAG CAACTGGTTAGCC | GCTGCATCCAGTTTGCAA AGT | CAACAGGCTAACAGTTTCCC GTGGACG |
| | 21-225_197C9 | | SEQ ID NO:2861 | SEQ ID NO:10873 | SEQ ID NO:18885 |
| | | AA | RASQGISNWLA | AASSLQS | QQANSFPWT |
| | | | SEQ ID NO:2862 | SEQ ID NO:10874 | SEQ ID NO:18886 |
| iPS:436164 | | NA | CGGGCAAGTCAGGGCATTAG AAATGATTTAGGC | GGTGCATCCAGTTTACA AAGT | CTACAGCATTATAGGTACCC ATTCACT |
| | 21-225_197G10 | | SEQ ID NO:2863 | SEQ ID NO:10875 | SEQ ID NO:18887 |
| | | AA | RASQGIRNDLG | GASSLQS | LQHYRYPFT |
| | | | SEQ ID NO:2864 | SEQ ID NO:10876 | SEQ ID NO:18888 |
| iPS:436167 | | NA | CGGGCGAGTCAGGGCATTAA CAAGTATTTATCC | GCTGCATCCAGTGTGCA AAGT | CAACGATATGACACTTACCC ATTCACT |

FIGURE 49

| | 21-225_197E11 | | SEQ ID NO:2865 | SEQ ID NO:10877 | SEQ ID NO:18889 |
|---|---|---|---|---|---|
| | | AA | RASQGINKYLS | AASSVQS | QRYDTYPFT |
| iPS:436173 | | NA | CGGACGAGTCAGGGCATTGG CAATTATTTAGCC | AAAACATCCAGTTTGCA AAGT | CAACAATATATGACTTACCC GCTCACT |
| | 21-225_197G12 | | SEQ ID NO:2867 | SEQ ID NO:10879 | SEQ ID NO:18891 |
| | | AA | RTSQGIGNYLA | KTSSLQS | QQYMTYPLT |
| iPS:436177 | | NA | AGGGCCGGTCAAAGTATTAG AACCAACTTCTTAGCC | GGTGCATCCAGCAGGGC CACT | CAGCAGTATGGTAGCTCACC GTGGACG |
| | 21-225_198B1 | | SEQ ID NO:2869 | SEQ ID NO:10881 | SEQ ID NO:18893 |
| | | AA | RAGQSIRTNFLA | GASSRAT | QQYGSSPWT |
| iPS:436179 | | NA | AGGGCCAGTCAGAGTATAA GGAGCAACTTCTTAGCC | GGTGCATTCAGTAGGGC CACT | CAGCAGTATGGTAATTCACC GTGGACG |
| | 21-225_198E1 | | SEQ ID NO:2871 | SEQ ID NO:10883 | SEQ ID NO:18895 |
| | | AA | RASQSIRSNFLA | GAFSRAT | QQYGNSPWT |
| iPS:436181 | | NA | AGGGCCAGTCAGAGTGTTAG AAGCAGCTACTTAGCC | GGTGCATTCAGCAGGGC CAGT | CAGCAGTATGGTAACTCACC GTGGACG |
| | 21-225_198C2 | | SEQ ID NO:2873 | SEQ ID NO:10885 | SEQ ID NO:18897 |
| | | AA | RASQSVRSSYLA | GAFSRAS | QQYGNSPWT |
| iPS:436189 | | NA | CGGGCGAGTCAGGGCATTGG CAATTATTTAGCC | GCTGCATCCAGTTTGCAA AGT | CAACAATATAGTACTTACCC GCTCACT |
| | 21-225_198B6 | | SEQ ID NO:2875 | SEQ ID NO:10887 | SEQ ID NO:18899 |
| | | AA | RASQGIGNYLA | AASSLQS | QQYSTYPLT |
| iPS:436191 | | NA | CGGGCAAGTCAGGGCATAA GAAAAGATTTAGGC | GCTGCATCCAGTTTGCAA AGT | CTACAACATTATCGTTACCC TTTCACT |

FIGURE 49

| | 21-225_198B9 | | SEQ ID NO:2877 | SEQ ID NO:10889 | SEQ ID NO:18901 |
|---|---|---|---|---|---|
| | | AA | RASQGIRKDLG | AASSLQS | LQHYRYPFT |
| | | | SEQ ID NO:2878 | SEQ ID NO:10890 | SEQ ID NO:18902 |
| iPS:436193 | 21-225_198A10 | NA | AAGTCTAGTCAGAGCCTCCT CTATAGTGATGGAAGGACCT ATTTGTAT | GAGGTTTCCAACCGGTTC TCT | ATGCAAAGTATACAGCTTCC CTGGACG |
| | | | SEQ ID NO:2879 | SEQ ID NO:10891 | SEQ ID NO:18903 |
| | | AA | KSSQSLLYSDGRTYLY | EVSNRFS | MQSIQLPWT |
| | | | SEQ ID NO:2880 | SEQ ID NO:10892 | SEQ ID NO:18904 |
| iPS:436195 | 21-225_198G10 | NA | AGGGCCAGTCAGAGTATTCG CAGCAGCTTCTTAGCC | GGTGCATCCAGCAGGGC CACT | CAGCAGTATGGTAACTCACC GTGGGCG |
| | | | SEQ ID NO:2881 | SEQ ID NO:10893 | SEQ ID NO:18905 |
| | | AA | RASQSIRSSFLA | GASSRAT | QQYGNSPWA |
| | | | SEQ ID NO:2882 | SEQ ID NO:10894 | SEQ ID NO:18906 |
| iPS:436197 | 21-225_199C2 | NA | AGGGCCAGTCAGAGTATTCG CAGCAGCTTCTTAGCC | GGTGCATCCAGCAGGGC CACT | CAGCAGTATGGTAACTCACC GTGGGCG |
| | | | SEQ ID NO:2883 | SEQ ID NO:10895 | SEQ ID NO:18907 |
| | | AA | RASQSIRSSFLA | GASSRAT | QQYGNSPWA |
| | | | SEQ ID NO:2884 | SEQ ID NO:10896 | SEQ ID NO:18908 |
| iPS:436199 | 21-225_199E3 | NA | CGGGCAAGTCAGGGCATTAG AAATGATTTAGGC | TCTGCATCCAGTTTGCAA AGG | CTACAGCATAAAAGGTACCC GCTCACT |
| | | | SEQ ID NO:2885 | SEQ ID NO:10897 | SEQ ID NO:18909 |
| | | AA | RASQGIRNDLG | SASSLQR | LQHKRYPLT |
| | | | SEQ ID NO:2886 | SEQ ID NO:10898 | SEQ ID NO:18910 |
| iPS:436201 | 21-225_199C5 | NA | CGGGCGAGTCAGGGCATTAG CAAGTATTTAGCC | GCTGCATCCAGTTTGCAA AGT | CAACAGTATCTTACTTACCC GCTCACT |
| | | | SEQ ID NO:2887 | SEQ ID NO:10899 | SEQ ID NO:18911 |
| | | AA | RASQGISKYLA | AASSLQS | QQYLTYPLT |
| | | | SEQ ID NO:2888 | SEQ ID NO:10900 | SEQ ID NO:18912 |

FIGURE 49

| iPS:436203 | | NA | AGGCCCAGTCAGAGTTTTAG CAGAAACTTAGCC | GGTGCATCCACTAGGGC CACT | CAGCAGTATAATAACTGGCC GCTCACT |
|---|---|---|---|---|---|
| | 21-225_199A6 | | SEQ ID NO:2889 | SEQ ID NO:10901 | SEQ ID NO:18913 |
| | | AA | RPSQSFSRNLA | GASTRAT | QQYNNWPLT |
| | | | SEQ ID NO:2890 | SEQ ID NO:10902 | SEQ ID NO:18914 |
| iPS:436205 | | NA | CGGGCAAGTCAGGGCATAA GAAAAGATTTAGGC | GCTGCATCCAGTTTGCAA AGT | CTACAACATTATCGTTACCC TTTCACT |
| | 21-225_199A7 | | SEQ ID NO:2891 | SEQ ID NO:10903 | SEQ ID NO:18915 |
| | | AA | RASQGIRKDLG | AASSLQS | LQHYRYPFT |
| | | | SEQ ID NO:2892 | SEQ ID NO:10904 | SEQ ID NO:18916 |
| iPS:436207 | | NA | AGGGCCAGTCAGAGTATAA GGACCAACTTCTTAGCC | GGTGCATCCAGCAGGGC CACT | CAGCAGTATGGTAACTCACC GTGGACG |
| | 21-225_199C7 | | SEQ ID NO:2893 | SEQ ID NO:10905 | SEQ ID NO:18917 |
| | | AA | RASQSIRTNFLA | GASSRAT | QQYGNSPWT |
| | | | SEQ ID NO:2894 | SEQ ID NO:10906 | SEQ ID NO:18918 |
| iPS:436210 | | NA | AGGGCCAGTCAGAGTGTTAG AAGCAGCTACTTAGCC | GGTGCATTCAGCAGGGC CACT | CAGCAGTATGGTAACTCACC GTGGACG |
| | 21-225_199G11 | | SEQ ID NO:2895 | SEQ ID NO:10907 | SEQ ID NO:18919 |
| | | AA | RASQSVRSSYLA | GAFSRAT | QQYGNSPWT |
| | | | SEQ ID NO:2896 | SEQ ID NO:10908 | SEQ ID NO:18920 |
| iPS:436212 | | NA | CGGGCAAGTCAGAGCATTAG CAGCTATTTAAAT | GCTGAGTCCAGTTTACA AAGT | CAACAGAGTTACAGTTCCCC TCCGTGGACG |
| | 21-225_200G1 | | SEQ ID NO:2897 | SEQ ID NO:10909 | SEQ ID NO:18921 |
| | | AA | RASQSISSYLN | AESSLQS | QQSYSSPWT |
| | | | SEQ ID NO:2898 | SEQ ID NO:10910 | SEQ ID NO:18922 |
| iPS:436214 | | NA | CGGGCAAGTCAGGACATTAG AAATGATTTAGGC | GCTGCATCCAGTTTGCAA AGT | CTACAGCATTATCGTTACCC ATTCACT |
| | 21-225_200F6 | | SEQ ID NO:2899 | SEQ ID NO:10911 | SEQ ID NO:18923 |
| | | AA | RASQDIRNDLG | AASSLQS | LQHYRYPFT |
| | | | SEQ ID NO:2900 | SEQ ID NO:10912 | SEQ ID NO:18924 |

FIGURE 49

| iPS:436216 | | NA | CGGGCCAGTCAGAACATTGG TAATACCTTGCAC | TATGCTTCCCAGTCCTTC TCA | CATCAGAGTGGTAGTTTACC TCAGACG |
|---|---|---|---|---|---|
| | 21-225_200B7 | | SEQ ID NO:2901 | SEQ ID NO:10913 | SEQ ID NO:18925 |
| | | AA | RASQNIGNTLH | YASQSFS | HQSGSLPQT |
| | | | SEQ ID NO:2902 | SEQ ID NO:10914 | SEQ ID NO:18926 |
| iPS:436218 | | NA | AAGTCCAGCCAGAGTGTTTT ACACAGCTCCAACAATAACA ACTACTTAGCT | TGGGCATCTACCCGGGA ATCC | CAGCAATATTATAATACTCC TCCGACA |
| | 21-225_200G7 | | SEQ ID NO:2903 | SEQ ID NO:10915 | SEQ ID NO:18927 |
| | | AA | KSSQSVLHSSNNNNYLA | WASTRES | QQYYNTPPT |
| | | | SEQ ID NO:2904 | SEQ ID NO:10916 | SEQ ID NO:18928 |
| iPS:436220 | | NA | CGGGCCAGTCAGAGTATTGG TAGTAACTTACAC | TCTGCTTCCCAGTCCTTC TCA | CAGCAGAGTAGTAGTTTACC GTGGACG |
| | 21-225_200F8 | | SEQ ID NO:2905 | SEQ ID NO:10917 | SEQ ID NO:18929 |
| | | AA | RASQSIGSNLH | SASQSFS | QQSSSLPWT |
| | | | SEQ ID NO:2906 | SEQ ID NO:10918 | SEQ ID NO:18930 |
| iPS:436222 | | NA | CGGGCAACTCAGGGCATTAG AAAAGATTTAGGC | ACTGCATCCAGTTTGCAA AGT | CTACAGCATAATAGTTACCC GTGGACG |
| | 21-225_200C9 | | SEQ ID NO:2907 | SEQ ID NO:10919 | SEQ ID NO:18931 |
| | | AA | RATQGIRKDLG | TASSLQS | LQHNSYPWT |
| | | | SEQ ID NO:2908 | SEQ ID NO:10920 | SEQ ID NO:18932 |
| iPS:436226 | | NA | AGGGCCAGTCAGAATATTCG CAGCAGCTTCTTAGCC | GGTGCATCCAGCAGGGC CACT | CAGCAGTATGGTAACTCACC GTGGGCG |
| | 21-225_200F10 | | SEQ ID NO:2909 | SEQ ID NO:10921 | SEQ ID NO:18933 |
| | | AA | RASQNIRSSFLA | GASSRAT | QQYGNSPWA |
| | | | SEQ ID NO:2910 | SEQ ID NO:10922 | SEQ ID NO:18934 |
| iPS:436228 | | NA | CGGGCAAGTCAGGGCATTAG AAATGATTTAGGC | TCTGCATCCAGTTTGCAT ACT | CTACAGCATAAGAGTTACCC GCTCACT |
| | 21-225_200F12 | | SEQ ID NO:2911 | SEQ ID NO:10923 | SEQ ID NO:18935 |
| | | AA | RASQGIRNDLG | SASSLHT | LQHKSYPLT |

FIGURE 49

| | | | SEQ ID NO:2912 | SEQ ID NO:10924 | SEQ ID NO:18936 |
|---|---|---|---|---|---|
| iPS:436230 | | NA | CGGGCAAGTCAGGGCATTAGGAATGATTTAGGC | TCTGCATCCATTTTACAAAGG | CTACAGCATAAAAGTTACCCTCTCACT |
| | 21-225_201A1 | | SEQ ID NO:2913 | SEQ ID NO:10925 | SEQ ID NO:18937 |
| | | AA | RASQGIRNDLG | SASILQR | LQHKSYPLT |
| | | | SEQ ID NO:2914 | SEQ ID NO:10926 | SEQ ID NO:18938 |
| iPS:436232 | | NA | AGGGCCAGTCCGAGTATTAACAGCGGCTTCTTAGCC | GGTGCATCCAGCAGGGCCACT | CACCAGTATGAGACCTCACCGTGGACG |
| | 21-225_201E1 | | SEQ ID NO:2915 | SEQ ID NO:10927 | SEQ ID NO:18939 |
| | | AA | RASPSINSGFLA | GASSRAT | HQYETSPWT |
| | | | SEQ ID NO:2916 | SEQ ID NO:10928 | SEQ ID NO:18940 |
| iPS:436234 | | NA | TCTGGAAGCAACTCCAACATCGGAAGTAATATTGTAACC | AGTAATGATCAGCGGCCCTCA | ACAGCATGGGATGACAGCCTGAATGGTTGGGTG |
| | 21-225_51E3 | | SEQ ID NO:2917 | SEQ ID NO:10929 | SEQ ID NO:18941 |
| | | AA | SGSNSNIGSNIVT | SNDQRPS | TAWDDSLNGWV |
| | | | SEQ ID NO:2918 | SEQ ID NO:10930 | SEQ ID NO:18942 |
| iPS:436236 | | NA | AGGGCCAGTCAGAATATTAAAAACAACTTAGCC | GGTGCATCCACCAGGGCCACT | CAGCAGTTTTATAACTGGCTGTGCAGT |
| | 21-225_201F7 | | SEQ ID NO:2919 | SEQ ID NO:10931 | SEQ ID NO:18943 |
| | | AA | RASQNIKNNLA | GASTRAT | QQFYNWLCS |
| | | | SEQ ID NO:2920 | SEQ ID NO:10932 | SEQ ID NO:18944 |
| iPS:436238 | | NA | AGGGCCAGTCAGAGTGTTAGCAGCAACTACTTAGCC | GGTGCATCCAGCAGGGCCACT | CAGCAGTATGAAAACTCACCGTGGACG |
| | 21-225_201B2 | | SEQ ID NO:2921 | SEQ ID NO:10933 | SEQ ID NO:18945 |
| | | AA | RASQSVSSNYLA | GASSRAT | QQYENSPWT |
| | | | SEQ ID NO:2922 | SEQ ID NO:10934 | SEQ ID NO:18946 |
| iPS:436240 | | NA | CGGGCCAGTCAGAACATTGGTCGTAGTTTACAC | TATGCTTCCCAGTCCTTCTCA | CATCAGAGTCGAAGTTTACCGCTCACT |
| | 21-225_201E8 | | SEQ ID NO:2923 | SEQ ID NO:10935 | SEQ ID NO:18947 |

FIGURE 49

| | | | | | |
|---|---|---|---|---|---|
| | | AA | RASQNIGRSLH | YASQSFS | HQSRSLPLT |
| | | | SEQ ID NO:2924 | SEQ ID NO:10936 | SEQ ID NO:18948 |
| iPS:436242 | 21-225_201A10 | NA | CGGGCAAGTCAGGGCATTAGAAATGATTTAGGC | TCTACATCCAGTTTGCATTCT | CTACAGCATAATAGTTACCCGCTCACT |
| | | | SEQ ID NO:2925 | SEQ ID NO:10937 | SEQ ID NO:18949 |
| | | AA | RASQGIRNDLG | STSSLHS | LQHNSYPLT |
| | | | SEQ ID NO:2926 | SEQ ID NO:10938 | SEQ ID NO:18950 |
| iPS:436244 | 21-225_201H10 | NA | CGGGCAAGTCACAACATTAACAGCTATTTAAAT | GCTGCATCCAGTTTGCAAAGT | CAACAGAGTTACAGTTTCCCGCTCACT |
| | | | SEQ ID NO:2927 | SEQ ID NO:10939 | SEQ ID NO:18951 |
| | | AA | RASHNINSYLN | AASSLQS | QQSYSFPLT |
| | | | SEQ ID NO:2928 | SEQ ID NO:10940 | SEQ ID NO:18952 |
| iPS:436246 | 21-225_201G6 | NA | AGGTCTAGTCAGAGCCTCCTCCATAATAATAGATACAACCATTTGGAT | TTGGGTTCTAATCGGGCCTCC | ATGCAAGCTCTACAAACTCCCACT |
| | | | SEQ ID NO:2929 | SEQ ID NO:10941 | SEQ ID NO:18953 |
| | | AA | RSSQSLLHNNRYNHLD | LGSNRAS | MQALQTPT |
| | | | SEQ ID NO:2930 | SEQ ID NO:10942 | SEQ ID NO:18954 |
| iPS:436248 | 21-225_202A3 | NA | CGGGCAAGTCAGGGCATTAGAAATGATTTAGGC | GCTGCATCCAGGTTGCAAAGT | CTACAGCATCATGACTACCCATTCACT |
| | | | SEQ ID NO:2931 | SEQ ID NO:10943 | SEQ ID NO:18955 |
| | | AA | RASQGIRNDLG | AASRLQS | LQHHDYPFT |
| | | | SEQ ID NO:2932 | SEQ ID NO:10944 | SEQ ID NO:18956 |
| iPS:436250 | 21-225_201A4 | NA | AGGTCCAGTCAGAATATTAAAAGCAACTTAGCC | GGTGCATCCACCAGGGCCACT | CAGCAGTTTTATAACTGGCTGTGCAGT |
| | | | SEQ ID NO:2933 | SEQ ID NO:10945 | SEQ ID NO:18957 |
| | | AA | RSSQNIKSNLA | GASTRAT | QQFYNWLCS |
| | | | SEQ ID NO:2934 | SEQ ID NO:10946 | SEQ ID NO:18958 |
| iPS:436252 | | NA | AGGGCCAGTCAGAGAATTAACAACAACTTAGCC | GGTGCATCCACCAGGGCCACT | CAGCAGTATTATAACTGGCTGTGCAGT |

FIGURE 49

| | 21-225_202A8 | | SEQ ID NO:2935 | SEQ ID NO:10947 | SEQ ID NO:18959 |
|---|---|---|---|---|---|
| | | AA | RASQRINNNLA | GASTRAT | QQYYNWLCS |
| | | | SEQ ID NO:2936 | SEQ ID NO:10948 | SEQ ID NO:18960 |
| iPS:436254 | 21-225_202C12 | NA | AGGTCTAGTCAGAGCCTCCT GCATAATAATAAATACAACC ATTTGGAT | TTGGGTTCTAATCGGGCC TCC | ATGCAAGCTCTACAAACTCC CACT |
| | | | SEQ ID NO:2937 | SEQ ID NO:10949 | SEQ ID NO:18961 |
| | | AA | RSSQSLLHNNKYNHLD | LGSNRAS | MQALQTPT |
| | | | SEQ ID NO:2938 | SEQ ID NO:10950 | SEQ ID NO:18962 |
| iPS:436256 | 21-225_202D9 | NA | AGGGCCAGTCAGAGTGTTAA CAGCGGCTACTTAGCC | GGTGCATCCAGCAGGGC CACT | CAACAATATGAGACCTCACC GTGGACG |
| | | | SEQ ID NO:2939 | SEQ ID NO:10951 | SEQ ID NO:18963 |
| | | AA | RASQSVNSGYLA | GASSRAT | QQYETSPWT |
| | | | SEQ ID NO:2940 | SEQ ID NO:10952 | SEQ ID NO:18964 |
| iPS:436258 | 21-225_202F12 | NA | AGGGCCAGTCAGAGTGTTCT GAACAACTTAGCC | GGTGCATCCACTAGGGC CACT | CAGCAGTATGATAACTGGCC TCCGTGCAGT |
| | | | SEQ ID NO:2941 | SEQ ID NO:10953 | SEQ ID NO:18965 |
| | | AA | RASQSVLNNLA | GASTRAT | QQYDNWPPCS |
| | | | SEQ ID NO:2942 | SEQ ID NO:10954 | SEQ ID NO:18966 |
| iPS:436260 | 21-225_203H1 | NA | CGGGCGAGTCAGGGCATTGG CAATTATTTAGCC | GTTGCATCCAGGTTGCA AAGT | CAACGGTATCATACTTACCC GCTCACT |
| | | | SEQ ID NO:2943 | SEQ ID NO:10955 | SEQ ID NO:18967 |
| | | AA | RASQGIGNYLA | VASRLQS | QRYHTYPLT |
| | | | SEQ ID NO:2944 | SEQ ID NO:10956 | SEQ ID NO:18968 |
| iPS:436262 | 21-225_203E3 | NA | CGGGCAAGTCACAACATTAA CAGCTATTTAAAT | GCTGCATCCAGTTTGCAA AGT | CAACAGAGTTACAGTTTCCC GCTCACT |
| | | | SEQ ID NO:2945 | SEQ ID NO:10957 | SEQ ID NO:18969 |
| | | AA | RASHNINSYLN | AASSLQS | QQSYSFPLT |
| | | | SEQ ID NO:2946 | SEQ ID NO:10958 | SEQ ID NO:18970 |

FIGURE 49

| iPS:436264 | | NA | CGGGCAAGTCAGGGCATTAG ACATGATTTAGGC | GCTGCATCCAGTTTGCAA AGT | CTACAGCATTATAGTTTCCCT CGGACG |
|---|---|---|---|---|---|
| | 21-225_203F7 | | SEQ ID NO:2947 | SEQ ID NO:10959 | SEQ ID NO:18971 |
| | | AA | RASQGIRHDLG | AASSLQS | LQHYSFPRT |
| | | | SEQ ID NO:2948 | SEQ ID NO:10960 | SEQ ID NO:18972 |
| iPS:436268 | | NA | CGGGCAAGTCAGGGCATTAG AAATGATTTAGGC | AGGGCATCCAGTGTGCA AAAT | CTACAGCATAATAGTTACCC ATTCACT |
| | 21-225_203B9 | | SEQ ID NO:2949 | SEQ ID NO:10961 | SEQ ID NO:18973 |
| | | AA | RASQGIRNDLG | RASSVQN | LQHNSYPFT |
| | | | SEQ ID NO:2950 | SEQ ID NO:10962 | SEQ ID NO:18974 |
| iPS:436270 | | NA | AAGTCCAGCCAGAGTGTTTT TTTCCACTCGAACAATAAGA ACTACTTAGCT | TGGGCATCTACCCGGGA ATCC | CAACAATATTTTAGTCTTCC ATTCACT |
| | 21-225_203F10 | | SEQ ID NO:2951 | SEQ ID NO:10963 | SEQ ID NO:18975 |
| | | AA | KSSQSVFFHSNNKNYLA | WASTRES | QQYFSLPFT |
| | | | SEQ ID NO:2952 | SEQ ID NO:10964 | SEQ ID NO:18976 |
| iPS:436272 | | NA | AAGTCCAGCCAGAGTGTTTT ATACAGTTCCAACAATAAGA ACTACTTAGTT | TGGGCATCTACCCGGGA ATCC | CAGCAATATTATAGTACTCC TCCGACG |
| | 21-225_201F5 | | SEQ ID NO:2953 | SEQ ID NO:10965 | SEQ ID NO:18977 |
| | | AA | KSSQSVLYSSNNKNYLV | WASTRES | QQYYSTPPT |
| | | | SEQ ID NO:2954 | SEQ ID NO:10966 | SEQ ID NO:18978 |
| iPS:436274 | | NA | CGGGCAAGTCAGGGCATTAG AAATGATTTAGGC | GCTGCAGCCAGTTTGCA AGGT | CTACAGCATTATAGTTACCC TCGGACG |
| | 21-225_204H3 | | SEQ ID NO:2955 | SEQ ID NO:10967 | SEQ ID NO:18979 |
| | | AA | RASQGIRNDLG | AAASLQG | LQHYSYPRT |
| | | | SEQ ID NO:2956 | SEQ ID NO:10968 | SEQ ID NO:18980 |
| iPS:436276 | | NA | CGGGCAAGTCACAACATTAA CAGCTATTTAAAT | GCTGCATCCAGTTTGCAA AGT | CAACAGAGTTACAGTTTCCC GCTCACT |
| | 21-225_204H4 | | SEQ ID NO:2957 | SEQ ID NO:10969 | SEQ ID NO:18981 |

FIGURE 49

| | | AA | RASHNINSYLN | AASSLQS | QQSYSFPLT |
|---|---|---|---|---|---|
| | | | SEQ ID NO:2958 | SEQ ID NO:10970 | SEQ ID NO:18982 |
| iPS:436278 | | NA | AGGGCCAGTCAGAATATTAAAAGCAACTTAGCC | GGTGCATCCACCAGGGCCACT | CAGCAGTTTTATAACTGGCTGTGCAGT |
| | 21-225_201F2 | | SEQ ID NO:2959 | SEQ ID NO:10971 | SEQ ID NO:18983 |
| | | AA | RASQNIKSNLA | GASTRAT | QQFYNWLCS |
| | | | SEQ ID NO:2960 | SEQ ID NO:10972 | SEQ ID NO:18984 |
| iPS:436280 | | NA | CGGGCAAGTCGGAGCGTTCACACCTATTTAAAT | GGTGCATCCAGTTTGCAACGT | CAACAGAGTTACAGTTCCCCGCTCACT |
| | 21-225_204D6 | | SEQ ID NO:2961 | SEQ ID NO:10973 | SEQ ID NO:18985 |
| | | AA | RASRSVHTYLN | GASSLQR | QQSYSSPLT |
| | | | SEQ ID NO:2962 | SEQ ID NO:10974 | SEQ ID NO:18986 |
| iPS:436282 | | NA | CGGACGAGTCAGGGCATTGGCAATTATTTAGCC | GCTGCATCCAGTTTGCAAAGT | CAACACTATCTTAGTTACCCTCTCACT |
| | 21-225_204G6 | | SEQ ID NO:2963 | SEQ ID NO:10975 | SEQ ID NO:18987 |
| | | AA | RTSQGIGNYLA | AASSLQS | QHYLSYPLT |
| | | | SEQ ID NO:2964 | SEQ ID NO:10976 | SEQ ID NO:18988 |
| iPS:436284 | | NA | CGGGCGAGTCAGGGCATAAGTAATCATTTAGCC | GCTGCATCCAGTTTGCAAAGT | CAACAGTATAGTAATTACCCGGTCACT |
| | 21-225_204G8 | | SEQ ID NO:2965 | SEQ ID NO:10977 | SEQ ID NO:18989 |
| | | AA | RASQGISNHLA | AASSLQS | QQYSNYPVT |
| | | | SEQ ID NO:2966 | SEQ ID NO:10978 | SEQ ID NO:18990 |
| iPS:436286 | | NA | CGGGCAAGTCAGGGCATTAGAAATGATTTAGGC | TCTGCATCCAGTTTGCAAAGT | CTACAACATAATAGTTACCCTCTCACT |
| | 21-225_204H8 | | SEQ ID NO:2967 | SEQ ID NO:10979 | SEQ ID NO:18991 |
| | | AA | RASQGIRNDLG | SASSLQS | LQHNSYPLT |
| | | | SEQ ID NO:2968 | SEQ ID NO:10980 | SEQ ID NO:18992 |
| iPS:436290 | | NA | AGGGCCAGTCAGAATGTTAGTTACAGCTACTTAGCC | GGTGCATCCAGGAGGGCCACT | CAGCAGTATGGTAGCTCACCGTGCAGT |
| | 21-225_205G3 | | SEQ ID NO:2969 | SEQ ID NO:10981 | SEQ ID NO:18993 |

FIGURE 49

| | | AA | RASQNVSYSYLA | GASRRAT | QQYGSSPCS |
|---|---|---|---|---|---|
| | | | SEQ ID NO:2970 | SEQ ID NO:10982 | SEQ ID NO:18994 |
| iPS:436292 | | NA | CGGGCGAGTCAGGCCATTAGTAATCATTTAGCC | GCTGCATCCAGTTTGCAAAGT | CAACAATATAGTAATTACCCACTCACT |
| | 21-225_205H3 | | SEQ ID NO:2971 | SEQ ID NO:10983 | SEQ ID NO:18995 |
| | | AA | RASQAISNHLA | AASSLQS | QQYSNYPLT |
| | | | SEQ ID NO:2972 | SEQ ID NO:10984 | SEQ ID NO:18996 |
| iPS:436294 | | NA | AGGGCCAGTCAGAATATTAAAAGCAACTTAGCC | GGTGCATCCACCAGGGCCACT | CAGCAGTTTTATAACTGGCTGTGCAGT |
| | 21-225_205G4 | | SEQ ID NO:2973 | SEQ ID NO:10985 | SEQ ID NO:18997 |
| | | AA | RASQNIKSNLA | GASTRAT | QQFYNWLCS |
| | | | SEQ ID NO:2974 | SEQ ID NO:10986 | SEQ ID NO:18998 |
| iPS:436296 | | NA | CGGGCGAGTCAGGGCATTGGCAATTATTTAGCC | GGTGTCTCCAGTTTGCAAAGT | CAACAATATAGTAATTACCCTCTCACT |
| | 21-225_205F5 | | SEQ ID NO:2975 | SEQ ID NO:10987 | SEQ ID NO:18999 |
| | | AA | RASQGIGNYLA | GVSSLQS | QQYSNYPLT |
| | | | SEQ ID NO:2976 | SEQ ID NO:10988 | SEQ ID NO:19000 |
| iPS:436302 | | NA | AGGGCCAGTCAGAGTGTTTTCAGCAACTACTTAGCC | GGTGCATCCAGCAGGGCCGCT | CAGCAGTATGAAAGTTCACCGTGGACG |
| | 21-225_205G7 | | SEQ ID NO:2977 | SEQ ID NO:10989 | SEQ ID NO:19001 |
| | | AA | RASQSVFSNYLA | GASSRAA | QQYESSPWT |
| | | | SEQ ID NO:2978 | SEQ ID NO:10990 | SEQ ID NO:19002 |
| iPS:436304 | | NA | AGGTCTAGTCAGAGCCTCCTGCATAATAATAGATACAACCATTTGGAT | TTGGGTTCTAATCGGGCCTCC | ATGCAAGCTCTACAAACTCCCACT |
| | 21-225_201F3 | | SEQ ID NO:2979 | SEQ ID NO:10991 | SEQ ID NO:19003 |
| | | AA | RSSQSLLHNNRYNHLD | LGSNRAS | MQALQTPT |
| | | | SEQ ID NO:2980 | SEQ ID NO:10992 | SEQ ID NO:19004 |
| iPS:436306 | | NA | AGGGCCAGTCAGAGTGTTAATAGCTACTTAGCC | GGTGCATCCACCAGGGCCACT | CAAGAGTATAATGACTGGCCGTGCAGT |

FIGURE 49

| | 21-225_201H4 | | SEQ ID NO:2981 | SEQ ID NO:10993 | SEQ ID NO:19005 |
|---|---|---|---|---|---|
| | | AA | RASQSVNSYLA | GASTRAT | QEYNDWPCS |
| iPS:436308 | | NA | SEQ ID NO:2982<br>CGGGCAAGTCAGGGCATTAG<br>AAATGATTTAGGC | SEQ ID NO:10994<br>TCTGCATCCTTTTTGCAA<br>AGA | SEQ ID NO:19006<br>CTACAGCATAATAGTTACCC<br>GCTCACT |
| | 21-225_205H8 | AA | SEQ ID NO:2983<br>RASQGIRNDLG | SEQ ID NO:10995<br>SASFLQR | SEQ ID NO:19007<br>LQHNSYPLT |
| iPS:436310 | | NA | SEQ ID NO:2984<br>AGGGCCAGTCAGAGTATTAA<br>CAGCAACTACTTAGCC | SEQ ID NO:10996<br>GGTGCATCCAGCAGGGC<br>CACT | SEQ ID NO:19008<br>CAGCAGTATGAAAACTCACC<br>GTGGACG |
| | 21-225_202D11 | AA | SEQ ID NO:2985<br>RASQSINSYLA | SEQ ID NO:10997<br>GASSRAT | SEQ ID NO:19009<br>QQYENSPWT |
| iPS:436312 | | NA | SEQ ID NO:2986<br>CGGGCAAGTCACAACATTAA<br>CAGCTATTTAAAT | SEQ ID NO:10998<br>GCTGCATCCAGTTTGCAA<br>AGT | SEQ ID NO:19010<br>CAACAGAGTTACAGTTTCCC<br>GCTCACT |
| | 21-225_206A4 | AA | SEQ ID NO:2987<br>RASHNINSYLN | SEQ ID NO:10999<br>AASSLQS | SEQ ID NO:19011<br>QQSYSFPLT |
| iPS:436314 | | NA | SEQ ID NO:2988<br>CGGGCCAGTCAGAGCATTGG<br>TCGTAGCTTACAC | SEQ ID NO:11000<br>TATGCTTCCCAGTCCTTC<br>TCA | SEQ ID NO:19012<br>CATCAGAGTAGAAGTTTACC<br>GCTCACT |
| | 21-225_206G4 | AA | SEQ ID NO:2989<br>RASQSIGRSLH | SEQ ID NO:11001<br>YASQSFS | SEQ ID NO:19013<br>HQSRSLPLT |
| iPS:436316 | | NA | SEQ ID NO:2990<br>CGGGCAAGTCACAACATTAA<br>CAGCTATTTAAAT | SEQ ID NO:11002<br>GCTGCATCCAGTTTGCAA<br>AGT | SEQ ID NO:19014<br>CAACAGAGTTACAGTTTCCC<br>GCTCACT |
| | 21-225_206A5 | AA | SEQ ID NO:2991<br>RASHNINSYLN | SEQ ID NO:11003<br>AASSLQS | SEQ ID NO:19015<br>QQSYSFPLT |
| iPS:436324 | | NA | SEQ ID NO:2992<br>CGGGCGAGTCAGGGCATTAG<br>AAATTATTTAGCC | SEQ ID NO:11004<br>GCTGCATCCAGTTTGCAA<br>AGT | SEQ ID NO:19016<br>CAACAGTACAGTAATTACCC<br>TCTCACT |

EP 4 435 105 A2

FIGURE 49

| | 21-225_207G6 | | SEQ ID NO:2993 | SEQ ID NO:11005 | SEQ ID NO:19017 |
|---|---|---|---|---|---|
| | | AA | RASQGIRNYLA | AASSLQS | QQYSNYPLT |
| | | | SEQ ID NO:2994 | SEQ ID NO:11006 | SEQ ID NO:19018 |
| iPS:436328 | | NA | CGGGCAAGTCAGGGCATTAG AAATGATTTAGGC | GCTGCATCCAGTTTGCAA AGT | CTACAGCATAATAGTTACCC TCTCACC |
| | 21-225_207F12 | | SEQ ID NO:2995 | SEQ ID NO:11007 | SEQ ID NO:19019 |
| | | AA | RASQGIRNDLG | AASSLQS | LQHNSYPLT |
| | | | SEQ ID NO:2996 | SEQ ID NO:11008 | SEQ ID NO:19020 |
| iPS:436332 | | NA | CGGGCAAGTCAGGGCATTAG ACATGATTTAGGC | GCTGCATCCAGTTTGCAA AGT | CTACAGCATTATAGTTACCC TCGGACG |
| | 21-225_208B2 | | SEQ ID NO:2997 | SEQ ID NO:11009 | SEQ ID NO:19021 |
| | | AA | RASQGIRHDLG | AASSLQS | LQHYSYPRT |
| | | | SEQ ID NO:2998 | SEQ ID NO:11010 | SEQ ID NO:19022 |
| iPS:436334 | | NA | AGGTCTAGTCAGAGCCTCCT GCATAATAATAAATACAACC ATTTGGAT | TTGGGTTCTAATCGGGCC TCC | ATGCAAGCTCTACAAACTCC CACT |
| | 21-225_208G3 | | SEQ ID NO:2999 | SEQ ID NO:11011 | SEQ ID NO:19023 |
| | | AA | RSSQSLLHNNKYNHLD | LGSNRAS | MQALQTPT |
| | | | SEQ ID NO:3000 | SEQ ID NO:11012 | SEQ ID NO:19024 |
| iPS:436336 | | NA | AGGGCCAGTCAGAGTGTTAG CAGCAACTACTTAGCC | GGTGCATCCAGCAGGGC CACT | CAGCACTACGAAAACTCACC GTGGACG |
| | 21-225_208B5 | | SEQ ID NO:3001 | SEQ ID NO:11013 | SEQ ID NO:19025 |
| | | AA | RASQSVSSNYLA | GASSRAT | QHYENSPWT |
| | | | SEQ ID NO:3002 | SEQ ID NO:11014 | SEQ ID NO:19026 |
| iPS:436338 | | NA | CGGGCAAGTCACAACATTAA CAGCTATTTAAAT | GCTGCATCCAGTTTGCAA AGT | CAACAGAGTTACAGTTTCCC GCTCACT |
| | 21-225_208E8 | | SEQ ID NO:3003 | SEQ ID NO:11015 | SEQ ID NO:19027 |
| | | AA | RASHNINSYLN | AASSLQS | QQSYSFPLT |
| | | | SEQ ID NO:3004 | SEQ ID NO:11016 | SEQ ID NO:19028 |

835

FIGURE 49

| iPS:436340 | | NA | AGGGCCAGTCAGAGTGTTAG CAACAACTACTTAGCC | GGTGCATCCAGCAGGGC CACT | CAGCACTATCATAGCTCACC GTGGACG |
|---|---|---|---|---|---|
| | 21-225_208A9 | | SEQ ID NO:3005 | SEQ ID NO:11017 | SEQ ID NO:19029 |
| | | AA | RASQSVSNNYLA | GASSRAT | QHYHSSPWT |
| | | | SEQ ID NO:3006 | SEQ ID NO:11018 | SEQ ID NO:19030 |
| iPS:436344 | | NA | CGGGCAAGTCACAACATTAA CAGCTATTTAAAT | GCTGCATCCAGTTTGCAA AGT | CAACAGAGTTACAGTTTCCC GCTCACT |
| | 21-225_208B11 | | SEQ ID NO:3007 | SEQ ID NO:11019 | SEQ ID NO:19031 |
| | | AA | RASHNINSYLN | AASSLQS | QQSYSFPLT |
| | | | SEQ ID NO:3008 | SEQ ID NO:11020 | SEQ ID NO:19032 |
| iPS:436350 | | NA | CGGGCAAGTCAGGGCATTAG AAATGATTTAGGC | GCTGCATCCAGTTTGCAA AGT | CTACAGCATAATAGTTACCC ATTCACT |
| | 21-225_210E4 | | SEQ ID NO:3009 | SEQ ID NO:11021 | SEQ ID NO:19033 |
| | | AA | RASQGIRNDLG | AASSLQS | LQHNSYPFT |
| | | | SEQ ID NO:3010 | SEQ ID NO:11022 | SEQ ID NO:19034 |
| iPS:436352 | | NA | CGGGCAAGTCAGGGCATTAG ACATGATTTAGGC | GCTGCATCCAGTTTGCAA AGT | CTACACCATTATAGTTACCC TCGGACG |
| | 21-225_210G5 | | SEQ ID NO:3011 | SEQ ID NO:11023 | SEQ ID NO:19035 |
| | | AA | RASQGIRHDLG | AASSLQS | LHHYSYPRT |
| | | | SEQ ID NO:3012 | SEQ ID NO:11024 | SEQ ID NO:19036 |
| iPS:436354 | | NA | CGGACAAGTCAGGGCATTAG AAATGATTTAGGC | GCTGCATCCAGTTTGTTT AGT | CTACAGTATAATAGTTACCC TCCCACC |
| | 21-225_210G10 | | SEQ ID NO:3013 | SEQ ID NO:11025 | SEQ ID NO:19037 |
| | | AA | RTSQGIRNDLG | AASSLFS | LQYNSYPPT |
| | | | SEQ ID NO:3014 | SEQ ID NO:11026 | SEQ ID NO:19038 |
| iPS:436356 | | NA | AGGGCCAGTCAGAGTGTTAA AAGCAACTTAGCC | GGTGCATCCACCAGGGC CACT | CAGCAGTATTATAACTGGCT GTGCAGT |
| | 21-225_210H10 | | SEQ ID NO:3015 | SEQ ID NO:11027 | SEQ ID NO:19039 |
| | | AA | RASQSVKSNLA | GASTRAT | QQYYNWLCS |
| | | | SEQ ID NO:3016 | SEQ ID NO:11028 | SEQ ID NO:19040 |

FIGURE 49

| iPS:436358 | | NA | CGGGCAAGTCACAACATTAACAGCTATTTAAAT | GCTGCATCCAGTTTGCAAAGT | CAACAGAGTTACAGTTTCCCGCTCACT |
|---|---|---|---|---|---|
| | 21-225_210D11 | | SEQ ID NO:3017 | SEQ ID NO:11029 | SEQ ID NO:19041 |
| | | AA | RASHNINSYLN | AASSLQS | QQSYSFPLT |
| | | | SEQ ID NO:3018 | SEQ ID NO:11030 | SEQ ID NO:19042 |
| iPS:436360 | | NA | CGGGCGAGTCAGGGTATTAGCATCTGGTTAGCC | GCTGCATCCAGTTTGCAAAGT | CAACAGGCTAAAAGTTTCCCATTCACT |
| | 21-225_210H11 | | SEQ ID NO:3019 | SEQ ID NO:11031 | SEQ ID NO:19043 |
| | | AA | RASQGISIWLA | AASSLQS | QQAKSFPFT |
| | | | SEQ ID NO:3020 | SEQ ID NO:11032 | SEQ ID NO:19044 |
| iPS:436362 | | NA | AGGTCTAGTCAGAGCCTCCTGCATTATAATGGACACAACTTTTTGGAT | TTGGTTTCTAATCGGGCCTCC | ATGCAAGCTCTACAAACTCCCATGTGCAGT |
| | 21-225_210C12 | | SEQ ID NO:3021 | SEQ ID NO:11033 | SEQ ID NO:19045 |
| | | AA | RSSQSLLHYNGHNFLD | LVSNRAS | MQALQTPMCS |
| | | | SEQ ID NO:3022 | SEQ ID NO:11034 | SEQ ID NO:19046 |
| iPS:436364 | | NA | CGGGCGAGTCAGGGCATTGGCAATTATTTAGCC | GATGCATCCAGTTTGGAAAGT | CAACACTATATGACTTACCCGCTCACT |
| | 21-225_211A11 | | SEQ ID NO:3023 | SEQ ID NO:11035 | SEQ ID NO:19047 |
| | | AA | RASQGIGNYLA | DASSLES | QHYMTYPLT |
| | | | SEQ ID NO:3024 | SEQ ID NO:11036 | SEQ ID NO:19048 |
| iPS:436366 | | NA | CGGGCGAGTCAGGCCATTGGGAAACATTTAGCC | GCTGCATCCAGATTGCAAAGT | CAACAGTATAGTAATTATCCGCTCACT |
| | 21-225_211A3 | | SEQ ID NO:3025 | SEQ ID NO:11037 | SEQ ID NO:19049 |
| | | AA | RASQAIGKHLA | AASRLQS | QQYSNYPLT |
| | | | SEQ ID NO:3026 | SEQ ID NO:11038 | SEQ ID NO:19050 |
| iPS:436368 | | NA | AGGGCCAGTCAGAGTGTTAGCAGCAGCTTCTTAGCC | GGTGCATCCAGCAGGGCCACT | CAGCAGTATGTTAGCTCACCGCTCACT |
| | 21-225_211G3 | | SEQ ID NO:3027 | SEQ ID NO:11039 | SEQ ID NO:19051 |
| | | AA | RASQSVSSSFLA | GASSRAT | QQYVSSPLT |

FIGURE 49

|  |  |  | SEQ ID NO:3028 | SEQ ID NO:11040 | SEQ ID NO:19052 |
|---|---|---|---|---|---|
| iPS:436370 |  | NA | CGGGCGAGTCAGGGCATTGGCAAGTATTTAGCC | GCTGCATCCAGTTTGCTAAGT | CAAAAGTATGATACTTACCCATTCACT |
|  | 21-225_211A6 |  | SEQ ID NO:3029 | SEQ ID NO:11041 | SEQ ID NO:19053 |
|  |  | AA | RASQGIGKYLA | AASSLLS | QKYDTYPFT |
|  |  |  | SEQ ID NO:3030 | SEQ ID NO:11042 | SEQ ID NO:19054 |
| iPS:436372 |  | NA | CGGGCGAGTCAGGGCATTAGCAGATATTTAGCC | GCTGCATCCAGTTTGCAAAGT | CTACGGTATGATACTTACCCTCTCATT |
|  | 21-225_211A8 |  | SEQ ID NO:3031 | SEQ ID NO:11043 | SEQ ID NO:19055 |
|  |  | AA | RASQGISRYLA | AASSLQS | LRYDTYPLI |
|  |  |  | SEQ ID NO:3032 | SEQ ID NO:11044 | SEQ ID NO:19056 |
| iPS:436374 |  | NA | AGGTCTAGTCAGAGCCTCCTCCATAGTAATGGATACAACTATTTGGAT | TTGGGTTCTAATCGGGCCTCC | ATGCAAGCTCTACTAACTCCCGTGTGCAGT |
|  | 21-225_211C10 |  | SEQ ID NO:3033 | SEQ ID NO:11045 | SEQ ID NO:19057 |
|  |  | AA | RSSQSLLHSNGYNYLD | LGSNRAS | MQALLTPVCS |
|  |  |  | SEQ ID NO:3034 | SEQ ID NO:11046 | SEQ ID NO:19058 |
| iPS:436376 |  | NA | CGGGCGAGTCAGGGCATTAGCAATCATTTAGCC | GCTGCATCCAGTTTGCAAAGT | CAACAGTATGTTACCTACCCTCTCACT |
|  | 21-225_212E6 |  | SEQ ID NO:3035 | SEQ ID NO:11047 | SEQ ID NO:19059 |
|  |  | AA | RASQGISNHLA | AASSLQS | QQYVTYPLT |
|  |  |  | SEQ ID NO:3036 | SEQ ID NO:11048 | SEQ ID NO:19060 |
| iPS:436378 |  | NA | CGGGCGAGTCAGGGCATTAGCAATCATTTAGCC | GCTGCATCCAGTTTGCAAAGT | CAGCAGTATAGTAATTACCCTCTCACT |
|  | 21-225_212D7 |  | SEQ ID NO:3037 | SEQ ID NO:11049 | SEQ ID NO:19061 |
|  |  | AA | RASQGISNHLA | AASSLQS | QQYSNYPLT |
|  |  |  | SEQ ID NO:3038 | SEQ ID NO:11050 | SEQ ID NO:19062 |
| iPS:436380 |  | NA | CGGGCGAGTCAGGGCATTAGCAGTTATTTAGCC | GCTGCATCCAGTTTGCAAAGT | CTACGGTATGATACTTACCCTCTCACT |
|  | 21-225_212H9 |  | SEQ ID NO:3039 | SEQ ID NO:11051 | SEQ ID NO:19063 |

FIGURE 49

| | | | | | |
|---|---|---|---|---|---|
| | | AA | RASQGISSYLA | AASSLQS | LRYDTYPLT |
| | | | SEQ ID NO:3040 | SEQ ID NO:11052 | SEQ ID NO:19064 |
| iPS:436382 | | NA | AGGGCCAGTCAGAGTGTTGCCAGCAGCTTAGCC | GGTACATCCACCAGGGCCACT | CAGCAGTATAATGACTGGCCGTGCAGT |
| | 21-225_212C10 | | SEQ ID NO:3041 | SEQ ID NO:11053 | SEQ ID NO:19065 |
| | | AA | RASQSVASSLA | GTSTRAT | QQYNDWPCS |
| | | | SEQ ID NO:3042 | SEQ ID NO:11054 | SEQ ID NO:19066 |
| iPS:436384 | | NA | CGGGCGAGTCAGGGCATTAGCAATTATTTAGAC | TCTGCATCCAATTTGCAAAGT | CAACACTATAGTAATTACCCGCTCACT |
| | 21-225_212F10 | | SEQ ID NO:3043 | SEQ ID NO:11055 | SEQ ID NO:19067 |
| | | AA | RASQGISNYLD | SASNLQS | QHYSNYPLT |
| | | | SEQ ID NO:3044 | SEQ ID NO:11056 | SEQ ID NO:19068 |
| iPS:436386 | | NA | CGGGCAAGTCAGGGCATTAGAAATGATTTAGGC | GCTGCATCCAGTTTGCAAAGT | CTACTGCATTATAGTTACCCTCGGACG |
| | 21-225_212B11 | | SEQ ID NO:3045 | SEQ ID NO:11057 | SEQ ID NO:19069 |
| | | AA | RASQGIRNDLG | AASSLQS | LLHYSYPRT |
| | | | SEQ ID NO:3046 | SEQ ID NO:11058 | SEQ ID NO:19070 |
| iPS:436388 | | NA | CGGGCGAGTCAGGCCATTGGGAAACATTTAGCC | GCTGCATCCAGATTGCAAAGT | CAACACTATAGTAATTATCCGCTCACT |
| | 21-225_212H11 | | SEQ ID NO:3047 | SEQ ID NO:11059 | SEQ ID NO:19071 |
| | | AA | RASQAIGKHLA | AASRLQS | QHYSNYPLT |
| | | | SEQ ID NO:3048 | SEQ ID NO:11060 | SEQ ID NO:19072 |
| iPS:436390 | | NA | CGGGCGAGTCAGGGCATTAGCAATCATTTAGCC | GCTGCATCCAGTTTGCAAAGT | CACCAGTATAGTAATTACCCTCTCACT |
| | 21-225_213D2 | | SEQ ID NO:3049 | SEQ ID NO:11061 | SEQ ID NO:19073 |
| | | AA | RASQGISNHLA | AASSLQS | HQYSNYPLT |
| | | | SEQ ID NO:3050 | SEQ ID NO:11062 | SEQ ID NO:19074 |
| iPS:436392 | | NA | CGGGCGAGTCAGGGCATTGGCAAGTATTTAGCC | GCTGCATCCAGTGTGCTAAGT | CAAAAGTATGATACTTACCCATTCACT |
| | 21-225_213B3 | | SEQ ID NO:3051 | SEQ ID NO:11063 | SEQ ID NO:19075 |

FIGURE 49

| | | AA | RASQGIGKYLA | AASSVLS | QKYDTYPFT |
|---|---|---|---|---|---|
| | | | SEQ ID NO:3052 | SEQ ID NO:11064 | SEQ ID NO:19076 |
| iPS:436394 | | NA | CGGGCGAGTCAGGCCATTAGGAATTATTTAGCC | GCTGCATCCAGTTTGCAAAGT | CAACAGTATAGTAATTACCCTCTCACT |
| | 21-225_213C4 | | SEQ ID NO:3053 | SEQ ID NO:11065 | SEQ ID NO:19077 |
| | | AA | RASQAIRNYLA | AASSLQS | QQYSNYPLT |
| | | | SEQ ID NO:3054 | SEQ ID NO:11066 | SEQ ID NO:19078 |
| iPS:436396 | | NA | CGGGCGAGTCAGGCCATTGGGAAACATTTAGCC | GCTGCATCCAGATTGCAAAGT | CAACACTATAGTAATTATCCGCTCACT |
| | 21-225_213E5 | | SEQ ID NO:3055 | SEQ ID NO:11067 | SEQ ID NO:19079 |
| | | AA | RASQAIGKHLA | AASRLQS | QHYSNYPLT |
| | | | SEQ ID NO:3056 | SEQ ID NO:11068 | SEQ ID NO:19080 |
| iPS:436398 | | NA | CGGGCGAGTCAGGGCATTAGCAATCATTTAGCC | GCTGCATCCAGTTTGCAAAGT | CAACAGTATAGTAATTACCCTCTCACT |
| | 21-225_213B8 | | SEQ ID NO:3057 | SEQ ID NO:11069 | SEQ ID NO:19081 |
| | | AA | RASQGISNHLA | AASSLQS | QQYSNYPLT |
| | | | SEQ ID NO:3058 | SEQ ID NO:11070 | SEQ ID NO:19082 |
| iPS:436400 | | NA | AAGTCCAGCCAGAATGTTTTAGACATCTCCAACAATAAGAATTCCTTAGGT | TGGGCATCTACCCGGGAATCC | CAGCAATATTATAACATTCCTCCGACG |
| | 21-225_213H7 | | SEQ ID NO:3059 | SEQ ID NO:11071 | SEQ ID NO:19083 |
| | | AA | KSSQNVLDISNNKNSLG | WASTRES | QQYYNIPPT |
| | | | SEQ ID NO:3060 | SEQ ID NO:11072 | SEQ ID NO:19084 |
| iPS:436402 | | NA | AAGTCCAGCCAGAATGTTTTAAAGACCTCCAACAATAGGAACTACTTAGCT | TGGGCATCTACCCGGGAATCC | CACCAATATTATAGTATTCCGTGGACC |
| | 21-225_213H12 | | SEQ ID NO:3061 | SEQ ID NO:11073 | SEQ ID NO:19085 |
| | | AA | KSSQNVLKTSNNRNYLA | WASTRES | HQYYSIPWT |
| | | | SEQ ID NO:3062 | SEQ ID NO:11074 | SEQ ID NO:19086 |

FIGURE 49

| iPS:436404 | | NA | CGGGCGAGTCAGGGCATTGG CAATTATTTAGCC | GCTGCATCCAGTTTGCAA AGT | CAACAATATATGACTTACCC AATCACT |
|---|---|---|---|---|---|
| | 21-225_214C3 | | SEQ ID NO:3063 | SEQ ID NO:11075 | SEQ ID NO:19087 |
| | | AA | RASQGIGNYLA | AASSLQS | QQYMTYPIT |
| | | | SEQ ID NO:3064 | SEQ ID NO:11076 | SEQ ID NO:19088 |
| iPS:436406 | | NA | CGGGCGAGTCAGGGCATTAG CAATTATTTAGCC | GCTGCATCCAGTTTGCAA AGT | CAACAGTATCTTACTTACCC ATTCACT |
| | 21-225_214E4 | | SEQ ID NO:3065 | SEQ ID NO:11077 | SEQ ID NO:19089 |
| | | AA | RASQGISNYLA | AASSLQS | QQYLTYPFT |
| | | | SEQ ID NO:3066 | SEQ ID NO:11078 | SEQ ID NO:19090 |
| iPS:436408 | | NA | CGGGCCAGTCAGAGCATTGG TGTTAGCTTACAC | TATGCTTCCCAGTCCTTC TCA | CATCAGAGTCGTAGTTTACC ATTCACT |
| | 21-225_214H8 | | SEQ ID NO:3067 | SEQ ID NO:11079 | SEQ ID NO:19091 |
| | | AA | RASQSIGVSLH | YASQSFS | HQSRSLPFT |
| | | | SEQ ID NO:3068 | SEQ ID NO:11080 | SEQ ID NO:19092 |
| iPS:436410 | | NA | CGGGCGAGTCAGGGCATTAG CAATCATTTAGCC | GCTGCATCCAGTTTGCAA AGT | CAACAGTATAGTAATTACCC TCTCACT |
| | 21-225_212E10 | | SEQ ID NO:3069 | SEQ ID NO:11081 | SEQ ID NO:19093 |
| | | AA | RASQGISNHLA | AASSLQS | QQYSNYPLT |
| | | | SEQ ID NO:3070 | SEQ ID NO:11082 | SEQ ID NO:19094 |
| iPS:436412 | | NA | CGGGCAAGTCAGGGCATTAG AAATGATTTAGGC | GCTGCATCCAGTTTGCAA AGT | CTACAGCATTATAGTTACCC TCGGACG |
| | 21-225_214H9 | | SEQ ID NO:3071 | SEQ ID NO:11083 | SEQ ID NO:19095 |
| | | AA | RASQGIRNDLG | AASSLQS | LQHYSYPRT |
| | | | SEQ ID NO:3072 | SEQ ID NO:11084 | SEQ ID NO:19096 |
| iPS:436414 | | NA | CGGGCAAGTCAGGGCATTAG AAATGATTTAGGC | GCTGCATCCAGTTTGCAA AGT | CTACTGCATAATAGTTACCC TCGGACG |
| | 21-225_214G10 | | SEQ ID NO:3073 | SEQ ID NO:11085 | SEQ ID NO:19097 |
| | | AA | RASQGIRNDLG | AASSLQS | LLHNSYPRT |
| | | | SEQ ID NO:3074 | SEQ ID NO:11086 | SEQ ID NO:19098 |

FIGURE 49

| iPS:436416 | | NA | CGGGCAAGTCAGGGCATTAG AAATGATTTAGGC | GCTGCATCCAGTTTGCAA AGT | GTAATGCATTATAGTTACCC TCGGACG |
|---|---|---|---|---|---|
| | 21-225_214G12 | | SEQ ID NO:3075 | SEQ ID NO:11087 | SEQ ID NO:19099 |
| | | AA | RASQGIRNDLG | AASSLQS | VMHYSYPRT |
| | | | SEQ ID NO:3076 | SEQ ID NO:11088 | SEQ ID NO:19100 |
| iPS:436418 | | NA | CGGGCAAGTCAGGGCATTAG AAATGATTTAGGC | GCTGCATCCAGTTTGCAA AGT | GTAATGCATAATAGTTACCC TCGGACG |
| | 21-225_215E3 | | SEQ ID NO:3077 | SEQ ID NO:11089 | SEQ ID NO:19101 |
| | | AA | RASQGIRNDLG | AASSLQS | VMHNSYPRT |
| | | | SEQ ID NO:3078 | SEQ ID NO:11090 | SEQ ID NO:19102 |
| iPS:436420 | | NA | CGGGCGAGTCAGGGCATTAG CAATCATTTAGCC | GCTGCATCCAGTTTGCAA AGT | CAGCAGTATATTAATTACCC TCTCACT |
| | 21-225_215B5 | | SEQ ID NO:3079 | SEQ ID NO:11091 | SEQ ID NO:19103 |
| | | AA | RASQGISNHLA | AASSLQS | QQYINYPLT |
| | | | SEQ ID NO:3080 | SEQ ID NO:11092 | SEQ ID NO:19104 |
| iPS:436422 | | NA | CGGGCGAGTCAGGGCATTAG CAATCATTTAGCC | GCTGCATCCAGTTTGCAT AGT | CAACAGTATGTTACTTACCC TCTCACT |
| | 21-225_215D6 | | SEQ ID NO:3081 | SEQ ID NO:11093 | SEQ ID NO:19105 |
| | | AA | RASQGISNHLA | AASSLHS | QQYVTYPLT |
| | | | SEQ ID NO:3082 | SEQ ID NO:11094 | SEQ ID NO:19106 |
| iPS:436424 | | NA | CGGGCCAGTCAGAGCATCGG TGTTAGCTTACAC | TATGCTTCCCAGTCCCTC TCA | CATCAGAGTCGCAGTTTACC ATTCACT |
| | 21-225_215H6 | | SEQ ID NO:3083 | SEQ ID NO:11095 | SEQ ID NO:19107 |
| | | AA | RASQSIGVSLH | YASQSLS | HQSRSLPFT |
| | | | SEQ ID NO:3084 | SEQ ID NO:11096 | SEQ ID NO:19108 |
| iPS:436426 | | NA | AGGGCCAGTCAGAGGATTAC CACCAACTTCTTAGCT | GGTGCATCCAGCAGGGC CACT | CAGCAGTATGTTAGTTCATT GCTCACT |
| | 21-225_215C7 | | SEQ ID NO:3085 | SEQ ID NO:11097 | SEQ ID NO:19109 |
| | | AA | RASQRITTNFLA | GASSRAT | QQYVSSLLT |
| | | | SEQ ID NO:3086 | SEQ ID NO:11098 | SEQ ID NO:19110 |

FIGURE 49

| iPS:436428 | | NA | CGGGCAAGTCAGGGCATTAG AAATGATTTAGGC | GCTGCATCCAGTTTGCAA AGT | GTAATGCATTATAGTTACCC TCGGACG |
|---|---|---|---|---|---|
| | 21-225_215E11 | | SEQ ID NO:3087 | SEQ ID NO:11099 | SEQ ID NO:19111 |
| | | AA | RASQGIRNDLG | AASSLQS | VMHYSYPRT |
| | | | SEQ ID NO:3088 | SEQ ID NO:11100 | SEQ ID NO:19112 |
| iPS:436430 | | NA | CGGACGAGTCAGGACATTGG CAATTATTTAGCC | GCTGCATCCAGTTTACAG AGT | CAACAGTATGTTACTTACCC GCTCACT |
| | 21-225_215A12 | | SEQ ID NO:3089 | SEQ ID NO:11101 | SEQ ID NO:19113 |
| | | AA | RTSQDIGNYLA | AASSLQS | QQYVTYPLT |
| | | | SEQ ID NO:3090 | SEQ ID NO:11102 | SEQ ID NO:19114 |
| iPS:436432 | | NA | AGGGCCAGTCAGAGTGTTAG CAGCAGCTTCTTAGCT | GGTGCATCCAGCAGGGC CATT | CAGCAGTATGTTAGCTCACC GCTCACT |
| | 21-225_215H12 | | SEQ ID NO:3091 | SEQ ID NO:11103 | SEQ ID NO:19115 |
| | | AA | RASQSVSSSFLA | GASSRAI | QQYVSSPLT |
| | | | SEQ ID NO:3092 | SEQ ID NO:11104 | SEQ ID NO:19116 |
| iPS:436434 | | NA | AGGGCCAGTCAGAGTGTTAA CAACAACTTAGCC | GGTGCATCCACCAGGGC CACT | CAGCAGTATAATGACTGGCC GTGCAGT |
| | 21-225_216B10 | | SEQ ID NO:3093 | SEQ ID NO:11105 | SEQ ID NO:19117 |
| | | AA | RASQSVNNNLA | GASTRAT | QQYNDWPCS |
| | | | SEQ ID NO:3094 | SEQ ID NO:11106 | SEQ ID NO:19118 |
| iPS:436436 | | NA | AGGGCCAGTCAGAGTGTTAG CAGCAGCTTCTTAGCC | GGTACATCCACCAGGGC CACT | CAACAGTATGATAGGTCACC ATTCACT |
| | 21-225_216F10 | | SEQ ID NO:3095 | SEQ ID NO:11107 | SEQ ID NO:19119 |
| | | AA | RASQSVSSSFLA | GTSTRAT | QQYDRSPFT |
| | | | SEQ ID NO:3096 | SEQ ID NO:11108 | SEQ ID NO:19120 |
| iPS:436438 | | NA | CGGGCAAGTCAGGGCATTAG AAATGATTTAGGC | GCTGCATCCAGTTTGCAA AGT | CTAATGCATTATAGTTACCC TCGGACG |
| | 21-225_216E8 | | SEQ ID NO:3097 | SEQ ID NO:11109 | SEQ ID NO:19121 |
| | | AA | RASQGIRNDLG | AASSLQS | LMHYSYPRT |
| | | | SEQ ID NO:3098 | SEQ ID NO:11110 | SEQ ID NO:19122 |

FIGURE 49

| iPS:436440 | | NA | CGGGCAAGTCAGGGCATTAG AAATGATTTAGGC | GGTGCATCCAGTTTGCA AAGT | GTAATGCATAATAGTTACCC TCGGACG |
|---|---|---|---|---|---|
| | 21-225_216H12 | | SEQ ID NO:3099 | SEQ ID NO:11111 | SEQ ID NO:19123 |
| | | AA | RASQGIRNDLG | GASSLQS | VMHNSYPRT |
| | | | SEQ ID NO:3100 | SEQ ID NO:11112 | SEQ ID NO:19124 |
| iPS:436448 | | NA | CGGGCCAGTCAGAGCATTGG TAGTAGCTTACAC | TATGCTTCCCAGTCCTTC TCA | CATCAGAGTAGAAGTTTACC GTGGACG |
| | 21-225_217A3 | | SEQ ID NO:3101 | SEQ ID NO:11113 | SEQ ID NO:19125 |
| | | AA | RASQSIGSSLH | YASQSFS | HQSRSLPWT |
| | | | SEQ ID NO:3102 | SEQ ID NO:11114 | SEQ ID NO:19126 |
| iPS:436450 | | NA | CGGGCAAGTCAGGGCATTAG AAATGATTTAGGC | GCTGCATCCAGTTTGCAA AGT | GTAATGCATAATAGTTACCC TCGGACG |
| | 21-225_217E5 | | SEQ ID NO:3103 | SEQ ID NO:11115 | SEQ ID NO:19127 |
| | | AA | RASQGIRNDLG | AASSLQS | VMHNSYPRT |
| | | | SEQ ID NO:3104 | SEQ ID NO:11116 | SEQ ID NO:19128 |
| iPS:436452 | | NA | CGGGCGAGTCAGGGCATTGG CAATTATTTAGCC | GCTGCATCCAGTTTGCAA AGT | CAACAGTATGTTAATTACCC TCTCACT |
| | 21-225_217G5 | | SEQ ID NO:3105 | SEQ ID NO:11117 | SEQ ID NO:19129 |
| | | AA | RASQGIGNYLA | AASSLQS | QQYVNYPLT |
| | | | SEQ ID NO:3106 | SEQ ID NO:11118 | SEQ ID NO:19130 |
| iPS:436454 | | NA | CGGGCGAGTCAGGCCATTGG GAAACATTTAGCC | GCTGCATCCAGATTGCA AAGT | CAACACACCAGTAAATCTCC AGTGCAG |
| | 21-225_217B10 | | SEQ ID NO:3107 | SEQ ID NO:11119 | SEQ ID NO:19131 |
| | | AA | RASQAIGKHLA | AASRLQS | QHTSKSPVQ |
| | | | SEQ ID NO:3108 | SEQ ID NO:11120 | SEQ ID NO:19132 |
| iPS:436456 | | NA | CGGGCAAGTCAGGGCATTAG AAATGATTTAGGC | GCTGCATCCAGTTTGCAA AGT | GTAATGCATAATAGTTACCC TCGGACG |
| | 21-225_217G10 | | SEQ ID NO:3109 | SEQ ID NO:11121 | SEQ ID NO:19133 |
| | | AA | RASQGIRNDLG | AASSLQS | VMHNSYPRT |
| | | | SEQ ID NO:3110 | SEQ ID NO:11122 | SEQ ID NO:19134 |

FIGURE 49

| iPS:436458 | | NA | CGGGCAAGTCAGGGCATTAG AAATGATTTAGGC | GCTGCATCCAGTTTGCAA AGT | CTAATGCATTATAGTTACCC TCGGACG |
| | 21-225_217H12 | | SEQ ID NO:3111 | SEQ ID NO:11123 | SEQ ID NO:19135 |
| | | AA | RASQGIRNDLG | AASSLQS | LMHYSYPRT |
| | | | SEQ ID NO:3112 | SEQ ID NO:11124 | SEQ ID NO:19136 |
| iPS:436462 | | NA | CGGGCAAGTCAGGGCATTAG AAATGATTTAGGC | GCTGCATCCAGTTTGCAA AGT | GTAATGCATTATAGTTACCC TCGGACG |
| | 21-225_218C4 | | SEQ ID NO:3113 | SEQ ID NO:11125 | SEQ ID NO:19137 |
| | | AA | RASQGIRNDLG | AASSLQS | VMHYSYPRT |
| | | | SEQ ID NO:3114 | SEQ ID NO:11126 | SEQ ID NO:19138 |
| iPS:436464 | | NA | CGGGCGAGTCAGGGCATTAG CAATTATTTAGAC | TCTGCATCCAATTTGCAA AGT | CAACACTATAGTAATTACCC GCTCACT |
| | 21-225_219H1 | | SEQ ID NO:3115 | SEQ ID NO:11127 | SEQ ID NO:19139 |
| | | AA | RASQGISNYLD | SASNLQS | QHYSNYPLT |
| | | | SEQ ID NO:3116 | SEQ ID NO:11128 | SEQ ID NO:19140 |
| iPS:436472 | | NA | AGGGCCAGTCAGAGTATTAG CCGCAGCCACTTAGTC | GTTACATCCAGCAGGGC CACT | CAGCAGTATGGTAGCTCACC GTGGACG |
| | 21-225_220E1 | | SEQ ID NO:3117 | SEQ ID NO:11129 | SEQ ID NO:19141 |
| | | AA | RASQSISRSHLV | VTSSRAT | QQYGSSPWT |
| | | | SEQ ID NO:3118 | SEQ ID NO:11130 | SEQ ID NO:19142 |
| iPS:436480 | | NA | CGGGCAAGTCAGGGCATTAG AAATGATTTAGGC | GGTGCATCCAGTTTGCA AAGT | GTAATGCATAATAGTTACCC TCGGACG |
| | 21-225_220F8 | | SEQ ID NO:3119 | SEQ ID NO:11131 | SEQ ID NO:19143 |
| | | AA | RASQGIRNDLG | GASSLQS | VMHNSYPRT |
| | | | SEQ ID NO:3120 | SEQ ID NO:11132 | SEQ ID NO:19144 |
| iPS:436488 | | NA | CGGGCGAGTCAGGGTATTAG CAGCTGGTTAGCC | ACTGCATCCAATTTGCAA AGT | CAACAGGCTAACAGTTTCCC ATTCACT |
| | 21-225_221A6 | | SEQ ID NO:3121 | SEQ ID NO:11133 | SEQ ID NO:19145 |
| | | AA | RASQGISSWLA | TASNLQS | QQANSFPFT |
| | | | SEQ ID NO:3122 | SEQ ID NO:11134 | SEQ ID NO:19146 |

845

FIGURE 49

| iPS:436490 | | NA | CGGGCGAGTCAGGGCATTAG CAATTATTTAGCC | GCTGCATCCAATTTACAA AGT | CAACAGTATATGACTTACCC GCTCACT |
|---|---|---|---|---|---|
| | 21-225_221F6 | | SEQ ID NO:3123 | SEQ ID NO:11135 | SEQ ID NO:19147 |
| | | AA | RASQGISNYLA | AASNLQS | QQYMTYPLT |
| | | | SEQ ID NO:3124 | SEQ ID NO:11136 | SEQ ID NO:19148 |
| iPS:436494 | | NA | CGGGCGAGTCAGGGTATTAG CAGCTGGTTAGCC | ACTGCATCCAATTTGCAA AGT | CAACAGGCTAACAGTTTCCC ATTCACT |
| | 21-225_221F12 | | SEQ ID NO:3125 | SEQ ID NO:11137 | SEQ ID NO:19149 |
| | | AA | RASQGISSWLA | TASNLQS | QQANSFPFT |
| | | | SEQ ID NO:3126 | SEQ ID NO:11138 | SEQ ID NO:19150 |
| iPS:436496 | | NA | CGGGCGAGTCAGGGTATTAG CAGCTGGTTAGCC | ACTGCATCCAATTTGCAA AGT | CAACAGGCTAACAGTTTCCC ATTCACT |
| | 21-225_222E1 | | SEQ ID NO:3127 | SEQ ID NO:11139 | SEQ ID NO:19151 |
| | | AA | RASQGISSWLA | TASNLQS | QQANSFPFT |
| | | | SEQ ID NO:3128 | SEQ ID NO:11140 | SEQ ID NO:19152 |
| iPS:436500 | | NA | AAGTCCAGCCAGAGTGTTTT GAAAAGTTCCAACCATAGGA ACTACTTAGCT | TGGGCATCTACCCGGGA AACC | CAGCAATATTCTTCTATTCCG TGGACG |
| | 21-225_222H3 | | SEQ ID NO:3129 | SEQ ID NO:11141 | SEQ ID NO:19153 |
| | | AA | KSSQSVLKSSNHRNYLA | WASTRET | QQYSSIPWT |
| | | | SEQ ID NO:3130 | SEQ ID NO:11142 | SEQ ID NO:19154 |
| iPS:436502 | | NA | CGGGCGAGTCAGGGCATTAG CAATTATTTGGCC | GCTGCATCCAGTTTGCAA AGT | CTATATTATCTTAATTATCCG CTCACT |
| | 21-225_222A11 | | SEQ ID NO:3131 | SEQ ID NO:11143 | SEQ ID NO:19155 |
| | | AA | RASQGISNYLA | AASSLQS | LYYLNYPLT |
| | | | SEQ ID NO:3132 | SEQ ID NO:11144 | SEQ ID NO:19156 |
| iPS:436504 | | NA | CGGGCAAGTCAGAACATTAG TAATTATGTTAAT | ACTGCATCGAGTTTGCA AAGT | CAGCAGTATTACTTTACCCC ATTCACT |
| | 21-225_222H4 | | SEQ ID NO:3133 | SEQ ID NO:11145 | SEQ ID NO:19157 |
| | | AA | RASQNISNYVN | TASSLQS | QQYYFTPFT |

FIGURE 49

| | | | SEQ ID NO:3134 | SEQ ID NO:11146 | SEQ ID NO:19158 |
|---|---|---|---|---|---|
| iPS:436506 | | NA | AGGGCCAGTCAGAGTGTTTA CAGCAACTACTTAGCC | GGTGCATCAAGCAGGGC CACT | CAGCAGTATGAAGACTCACC GTGGACG |
| | 21-225_222C7 | | SEQ ID NO:3135 | SEQ ID NO:11147 | SEQ ID NO:19159 |
| | | AA | RASQSVYSNYLA | GASSRAT | QQYEDSPWT |
| | | | SEQ ID NO:3136 | SEQ ID NO:11148 | SEQ ID NO:19160 |
| iPS:436508 | | NA | CGGGCGAGTCAGGGTATTAG CAGCTGGTTAGCC | ACTGCATCCAATTTGCAA AGT | CAACAGGCTAACAGTTTCCC ATTCACT |
| | 21-225_222F7 | | SEQ ID NO:3137 | SEQ ID NO:11149 | SEQ ID NO:19161 |
| | | AA | RASQGISSWLA | TASNLQS | QQANSFPFT |
| | | | SEQ ID NO:3138 | SEQ ID NO:11150 | SEQ ID NO:19162 |
| iPS:436510 | | NA | CGGGCAAGTCAGAACATTAG TAATTATGTTAAT | ATTGCATCGAGTTTGCAA AGT | CAGCAGTATTACTTTACCCC ATTCACT |
| | 21-225_222H8 | | SEQ ID NO:3139 | SEQ ID NO:11151 | SEQ ID NO:19163 |
| | | AA | RASQNISNYVN | IASSLQS | QQYYFTPFT |
| | | | SEQ ID NO:3140 | SEQ ID NO:11152 | SEQ ID NO:19164 |
| iPS:436514 | | NA | CGGGCGAGTCAGGGCATTAG CAATTATTTGGCC | GCTGCATCCAGTTTGCAA AGT | CTACATTATCTTAATTACCCG CTCACT |
| | 21-225_222D10 | | SEQ ID NO:3141 | SEQ ID NO:11153 | SEQ ID NO:19165 |
| | | AA | RASQGISNYLA | AASSLQS | LHYLNYPLT |
| | | | SEQ ID NO:3142 | SEQ ID NO:11154 | SEQ ID NO:19166 |
| iPS:436516 | | NA | CGGGTGAGTCAGGGTATTAG CAGCTGGTTAGCC | ACTGCATCCAATTTGCAA AGT | CAACAGGATAACAGTTTCCC ATTCACT |
| | 21-225_222C12 | | SEQ ID NO:3143 | SEQ ID NO:11155 | SEQ ID NO:19167 |
| | | AA | RVSQGISSWLA | TASNLQS | QQDNSFPFT |
| | | | SEQ ID NO:3144 | SEQ ID NO:11156 | SEQ ID NO:19168 |
| iPS:436520 | | NA | AAGTCCAGCCAGAGTATTTT ACTCAGCTCCAACAATAAGA ACTACTTAGCT | TGGGCATCTACCCGGGA ATCC | CTGCAATATTTTAGTACTCC GTGGACG |
| | 21-225_223G10 | | SEQ ID NO:3145 | SEQ ID NO:11157 | SEQ ID NO:19169 |

FIGURE 49

| | | AA | KSSQSILLSSNNKNYLA | WASTRES | LQYFSTPWT |
|---|---|---|---|---|---|
| | | | SEQ ID NO:3146 | SEQ ID NO:11158 | SEQ ID NO:19170 |
| iPS:436522 | | NA | CGGGCGAGTCAGGGCATTAGTAATTATTTGGCC | GCTGCATCCAATTTGCAAAGT | CTACATTATCTTAATTACCCACTCACT |
| | 21-225_223H10 | | SEQ ID NO:3147 | SEQ ID NO:11159 | SEQ ID NO:19171 |
| | | AA | RASQGISNYLA | AASNLQS | LHYLNYPLT |
| | | | SEQ ID NO:3148 | SEQ ID NO:11160 | SEQ ID NO:19172 |
| iPS:436526 | | NA | CGGGCAAGTCAGGGCATTGAAAATGATTTAGGC | GCTGCATCCAGTTTGCAAAGT | CTACAGCATAATAGTTACCCGCTCACT |
| | 21-225_224A1 | | SEQ ID NO:3149 | SEQ ID NO:11161 | SEQ ID NO:19173 |
| | | AA | RASQGIENDLG | AASSLQS | LQHNSYPLT |
| | | | SEQ ID NO:3150 | SEQ ID NO:11162 | SEQ ID NO:19174 |
| iPS:436528 | | NA | CGGGCAAGTCAGGGCATTAGTAATGATTTAGGC | GCTGCATCCAGTTTGCAAAGT | CTACAGCATAATAGTTATCCTCCTACT |
| | 21-225_224B1 | | SEQ ID NO:3151 | SEQ ID NO:11163 | SEQ ID NO:19175 |
| | | AA | RASQGISNDLG | AASSLQS | LQHNSYPPT |
| | | | SEQ ID NO:3152 | SEQ ID NO:11164 | SEQ ID NO:19176 |
| iPS:436534 | | NA | CGGGCAAGTCAGGGCATTAGAGATGATTTAGGC | GCTGCATCCAGTTTGCAAAGT | CTACAGCATTATAGTTACCCTCGGACG |
| | 21-225_224F1 | | SEQ ID NO:3153 | SEQ ID NO:11165 | SEQ ID NO:19177 |
| | | AA | RASQGIRDDLG | AASSLQS | LQHYSYPRT |
| | | | SEQ ID NO:3154 | SEQ ID NO:11166 | SEQ ID NO:19178 |
| iPS:436536 | | NA | AAGTCTGGTCAGAGCCTCCTGCATAGTGATGGAAAGACCTTTTTGTCT | GAAATTTCCAACCGGTTCTCT | ATGCAAAGTACACAGCTTCCTCGGACG |
| | 21-225_224G1 | | SEQ ID NO:3155 | SEQ ID NO:11167 | SEQ ID NO:19179 |
| | | AA | KSGQSLLHSDGKTFLS | EISNRFS | MQSTQLPRT |
| | | | SEQ ID NO:3156 | SEQ ID NO:11168 | SEQ ID NO:19180 |
| iPS:436538 | | NA | CGGGCAAGTCAGGACATTAGAAATGATTTAGGC | GCTACATCCAGTTTGCAAAGT | CTACAGCATAATAGTTACCCGCTCACT |

FIGURE 49

| | 21-225_224C3 | | SEQ ID NO:3157 | SEQ ID NO:11169 | SEQ ID NO:19181 |
|---|---|---|---|---|---|
| | | AA | RASQDIRNDLG | ATSSLQS | LQHNSYPLT |
| | | | SEQ ID NO:3158 | SEQ ID NO:11170 | SEQ ID NO:19182 |
| iPS:436540 | 21-225_224F3 | NA | CGGGCAAGTCAGGGCATTAGAAATGATTTAGGC | GCTGCATCCAGTTTGCAAAGT | CTTCAGCATTATAATTACCCTCGGGCG |
| | | | SEQ ID NO:3159 | SEQ ID NO:11171 | SEQ ID NO:19183 |
| | | AA | RASQGIRNDLG | AASSLQS | LQHYNYPRA |
| | | | SEQ ID NO:3160 | SEQ ID NO:11172 | SEQ ID NO:19184 |
| iPS:436544 | 21-225_224H5 | NA | AAGTCCAGCCAGAGTGTTTTATACAGCTCCAACAATTTCAACTACTTAACT | TGGGCATCTACCCGGGAATCC | CAGCAATATTATAGTACTCCTCCGACG |
| | | | SEQ ID NO:3161 | SEQ ID NO:11173 | SEQ ID NO:19185 |
| | | AA | KSSQSVLYSSNNFNYLT | WASTRES | QQYYSTPPT |
| | | | SEQ ID NO:3162 | SEQ ID NO:11174 | SEQ ID NO:19186 |
| iPS:436546 | 21-225_224D6 | NA | CGGGCGAGTCAGGGTATTAGCAGCTGGTTAGCC | GCTGCATCCAGTTTGCAAAGT | CAACAGGCTAACAGTTTCCCGTGGACG |
| | | | SEQ ID NO:3163 | SEQ ID NO:11175 | SEQ ID NO:19187 |
| | | AA | RASQGISSWLA | AASSLQS | QQANSFPWT |
| | | | SEQ ID NO:3164 | SEQ ID NO:11176 | SEQ ID NO:19188 |
| iPS:436548 | 21-225_224A7 | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3165 | SEQ ID NO:11177 | SEQ ID NO:19189 |
| | | AA | KSSQSLLHSDGKTFLY | EISNRFS | MQSTQLPRT |
| | | | SEQ ID NO:3166 | SEQ ID NO:11178 | SEQ ID NO:19190 |
| iPS:436550 | 21-225_224D8 | NA | AAGTCCAGCCAGAGTGTTTTATACAGCTCCAACAATAAGAACTACTTAGCT | TGGTCGTCTACCCGGAATCC | CAGCAATATTTTAGTACTCCTCCGACG |
| | | | SEQ ID NO:3167 | SEQ ID NO:11179 | SEQ ID NO:19191 |
| | | AA | KSSQSVLYSSNNKNYLA | WSSTRKS | QQYFSTPPT |

FIGURE 49

|  |  |  | SEQ ID NO:3168 | SEQ ID NO:11180 | SEQ ID NO:19192 |
|---|---|---|---|---|---|
| iPS:436554 | 21-225_224C10 | NA | AAGTCCAGCCAGAGTGTTTT ATACAATTCCAACAATAAGA ACTACTTAGCT | TGGGCATCTACCCGGGA GTCC | CAACAATATTATATTAATCC GTGCAGT |
|  |  |  | SEQ ID NO:3169 | SEQ ID NO:11181 | SEQ ID NO:19193 |
|  |  | AA | KSSQSVLYNSNNKNYLA | WASTRES | QQYYINPCS |
|  |  |  | SEQ ID NO:3170 | SEQ ID NO:11182 | SEQ ID NO:19194 |
| iPS:436556 | 21-225_224D10 | NA | CGGGCAAGTCAGGGCATTAG AAATGATTTAGGC | GCTGCATCCAGTTTACAA AGT | CTACAACATAATAGTTACCC GCTCACT |
|  |  |  | SEQ ID NO:3171 | SEQ ID NO:11183 | SEQ ID NO:19195 |
|  |  | AA | RASQGIRNDLG | AASSLQS | LQHNSYPLT |
|  |  |  | SEQ ID NO:3172 | SEQ ID NO:11184 | SEQ ID NO:19196 |
| iPS:436558 | 21-225_224C11 | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
|  |  |  | SEQ ID NO:3173 | SEQ ID NO:11185 | SEQ ID NO:19197 |
|  |  | AA | KSSQSLLHSDGKTFLY | EISNRFS | MQSTQLPRT |
|  |  |  | SEQ ID NO:3174 | SEQ ID NO:11186 | SEQ ID NO:19198 |
| iPS:436560 | 21-225_224F11 | NA | AAGTCCAGCCAGAGTGTTTT ATCCAGCTCCAACAATCACA ACTACTTAGCT | TGGGCATCTACCCGGGA ATCC | CAGCAATATTATACTACTCC GTGCAGT |
|  |  |  | SEQ ID NO:3175 | SEQ ID NO:11187 | SEQ ID NO:19199 |
|  |  | AA | KSSQSVLSSSNNHNYLA | WASTRES | QQYYTTPCS |
|  |  |  | SEQ ID NO:3176 | SEQ ID NO:11188 | SEQ ID NO:19200 |
| iPS:436562 | 21-225_224H11 | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
|  |  |  | SEQ ID NO:3177 | SEQ ID NO:11189 | SEQ ID NO:19201 |
|  |  | AA | KSSQSLLHSDGKTFLY | EISNRFS | MQSTQLPRT |
|  |  |  | SEQ ID NO:3178 | SEQ ID NO:11190 | SEQ ID NO:19202 |

FIGURE 49

| iPS:436564 | | NA | CGGGCAAGTCAGGGCATTAG AGATGATTTAGGC | GCTGCATCCAGTTTGCAA AGT | CTGCAGCATTATAGTTACCC TCGGACG |
|---|---|---|---|---|---|
| | 21-225_225A1 | | SEQ ID NO:3179 | SEQ ID NO:11191 | SEQ ID NO:19203 |
| | | AA | RASQGIRDDLG | AASSLQS | LQHYSYPRT |
| | | | SEQ ID NO:3180 | SEQ ID NO:11192 | SEQ ID NO:19204 |
| iPS:436568 | | NA | AGGGCCAGTCAGAATCTTAG CAGCAGCTACTTAGGC | GATACATCCAGCAGGGC CACT | CAGGAGTATGGTAGCTCACT CATGTGCAGT |
| | 21-225_225B3 | | SEQ ID NO:3181 | SEQ ID NO:11193 | SEQ ID NO:19205 |
| | | AA | RASQNLSSSYLG | DTSSRAT | QEYGSSLMCS |
| | | | SEQ ID NO:3182 | SEQ ID NO:11194 | SEQ ID NO:19206 |
| iPS:436570 | | NA | AAGTCCAGCCAGAGTGTTTT ATATAGCTCCAACAATAAGA ACTACTTAGCT | TGGGCATCTACCCGGGA ATCC | CACCAATATCATAATTCTCC TCCCACT |
| | 21-225_225F4 | | SEQ ID NO:3183 | SEQ ID NO:11195 | SEQ ID NO:19207 |
| | | AA | KSSQSVLYSSNNKNYLA | WASTRES | HQYHNSPPT |
| | | | SEQ ID NO:3184 | SEQ ID NO:11196 | SEQ ID NO:19208 |
| iPS:436572 | | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
| | 21-225_225G4 | | SEQ ID NO:3185 | SEQ ID NO:11197 | SEQ ID NO:19209 |
| | | AA | KSSQSLLHSDGKTFLY | EISNRFS | MQSTQLPRT |
| | | | SEQ ID NO:3186 | SEQ ID NO:11198 | SEQ ID NO:19210 |
| iPS:436574 | | NA | AAGTCCAGCCAGAATGTTTT ATACAACTCCAACAATAACA ACTACTTAGCT | TGGGCATCTACCCGGGA ATCC | CAGCAATATTATAGTTCTCC TCCGACG |
| | 21-225_225F5 | | SEQ ID NO:3187 | SEQ ID NO:11199 | SEQ ID NO:19211 |
| | | AA | KSSQNVLYNSNNNYLA | WASTRES | QQYYSSPPT |
| | | | SEQ ID NO:3188 | SEQ ID NO:11200 | SEQ ID NO:19212 |
| iPS:436576 | | NA | CGGGCAAGTCAGGGCATGA GAAAAGATTTAGGC | GCTGCAACCAGTTTGCA AAGT | CTACAGCATAATAGTTATCC ATTCACT |

FIGURE 49

| | 21-225_225B6 | | SEQ ID NO:3189 | SEQ ID NO:11201 | SEQ ID NO:19213 |
|---|---|---|---|---|---|
| | | AA | RASQGMRKDLG | AATSLQS | LQHNSYPFT |
| | | | SEQ ID NO:3190 | SEQ ID NO:11202 | SEQ ID NO:19214 |
| iPS:436578 | | NA | CGGGCAAGTCAGGGCATTAG AAATGATTTAGGC | GCTGCATCCAGTTTACAA AGT | CTTCAGCATAATACTTACCC ATTCACT |
| | 21-225_225D6 | | SEQ ID NO:3191 | SEQ ID NO:11203 | SEQ ID NO:19215 |
| | | AA | RASQGIRNDLG | AASSLQS | LQHNTYPFT |
| | | | SEQ ID NO:3192 | SEQ ID NO:11204 | SEQ ID NO:19216 |
| iPS:436580 | | NA | AGGGCCAGTCAGAGTGTTTA CAGCAGCTACTTAGCC | GGTGCATCCAGCAGGGC CACT | CAGCAGTATGGTACCTCACC TCGGACG |
| | 21-225_225E7 | | SEQ ID NO:3193 | SEQ ID NO:11205 | SEQ ID NO:19217 |
| | | AA | RASQSVYSSYLA | GASSRAT | QQYGTSPRT |
| | | | SEQ ID NO:3194 | SEQ ID NO:11206 | SEQ ID NO:19218 |
| iPS:436582 | | NA | CGGGCAAGTCAGGGCATTAG AAATGATTTAGGC | GCTGCATCCAGTTTGCTA GGT | CTACAACATAATAGTTACCC ATTCACT |
| | 21-225_225F8 | | SEQ ID NO:3195 | SEQ ID NO:11207 | SEQ ID NO:19219 |
| | | AA | RASQGIRNDLG | AASSLLG | LQHNSYPFT |
| | | | SEQ ID NO:3196 | SEQ ID NO:11208 | SEQ ID NO:19220 |
| iPS:436584 | | NA | AAGTCCAGCCAGAGTGTTTT ATACAGCTCCAACAATAACA ACTACTTAGCT | TGGGCATCTACCCGGGA ATCC | CAACACTCCAAGAGTATTCC TGGTAAG |
| | 21-225_225B9 | | SEQ ID NO:3197 | SEQ ID NO:11209 | SEQ ID NO:19221 |
| | | AA | KSSQSVLYSSNNNNYLA | WASTRES | QHSKSIPGK |
| | | | SEQ ID NO:3198 | SEQ ID NO:11210 | SEQ ID NO:19222 |
| iPS:436586 | | NA | AAGTCCAGCCAGAGTGTTTT ATACAGCTCCAACAATTACA ACTACTTAGCT | TGGGCTTCTACCCGGGA ATCC | CAGCAATATTATACTACTCC TCCGACG |
| | 21-225_225F11 | | SEQ ID NO:3199 | SEQ ID NO:11211 | SEQ ID NO:19223 |
| | | AA | KSSQSVLYSSNNYNYLA | WASTRES | QQYYTTPPT |
| | | | SEQ ID NO:3200 | SEQ ID NO:11212 | SEQ ID NO:19224 |

FIGURE 49

| iPS:436588 | | NA | AAGTCCAGCCAGAGTGTTTT ATACAGCTCCAACAATCAGA ACTACTTAGCT | TGGACATCTACCCGGGA ATCC | CAGCAATATTATATTACTCC GTGCAGT |
|---|---|---|---|---|---|
| | 21-225_225F12 | | SEQ ID NO:3201 | SEQ ID NO:11213 | SEQ ID NO:19225 |
| | | AA | KSSQSVLYSSNNQNYLA | WTSTRES | QQYYITPCS |
| | | | SEQ ID NO:3202 | SEQ ID NO:11214 | SEQ ID NO:19226 |
| iPS:436590 | | NA | AAGTCCAGCCAGAGTGTTTT ATACAACTCCAACAATAACA ACTACTTAGCT | TGGGCATCTACCCGGGA ATCC | CAGCAATATTATATTACTCC GTGCAGT |
| | 21-225_225H12 | | SEQ ID NO:3203 | SEQ ID NO:11215 | SEQ ID NO:19227 |
| | | AA | KSSQSVLYNSNNNNYLA | WASTRES | QQYYITPCS |
| | | | SEQ ID NO:3204 | SEQ ID NO:11216 | SEQ ID NO:19228 |
| iPS:436592 | | NA | AAGTCTAGTCAGAGCCTCCT GCATGGTGATGGAAAGACCT ATTTGTAT | GAAGTTTCCATCCGGTTC TCT | ATGCAAAGTATACAGATTCC GTGGACG |
| | 21-225_226B1 | | SEQ ID NO:3205 | SEQ ID NO:11217 | SEQ ID NO:19229 |
| | | AA | KSSQSLLHGDGKTYLY | EVSIRFS | MQSIQIPWT |
| | | | SEQ ID NO:3206 | SEQ ID NO:11218 | SEQ ID NO:19230 |
| iPS:436594 | | NA | AAGTCTAGTCAGAGCCTCCT ACATGGTGATGGAAAGACCT ATTTGTAT | GAAGTTTCCAACCGGTTC TCT | ATGCAAAGTATACAGATTCC GTGGACG |
| | 21-225_226A5 | | SEQ ID NO:3207 | SEQ ID NO:11219 | SEQ ID NO:19231 |
| | | AA | KSSQSLLHGDGKTYLY | EVSNRFS | MQSIQIPWT |
| | | | SEQ ID NO:3208 | SEQ ID NO:11220 | SEQ ID NO:19232 |
| iPS:436596 | | NA | CGGGCAAGTCAGGGCATTAG AAATGATTTAGGC | GCTGCATCCAGTTTGCAA AGT | CTACAGCATTATAATTACCC TCGGGCG |
| | 21-225_226C6 | | SEQ ID NO:3209 | SEQ ID NO:11221 | SEQ ID NO:19233 |
| | | AA | RASQGIRNDLG | AASSLQS | LQHYNYPRA |
| | | | SEQ ID NO:3210 | SEQ ID NO:11222 | SEQ ID NO:19234 |

FIGURE 49

| iPS:436598 | 21-225_226D6 | NA | AGGTCCAGCCAGAGTATTTT ATACATCTCCAACAATAAGA ACTACTTAGCT | TGGGCATCTACCCGGGA ATCC | CAGCAATATTATAGTTCTCC GTGCAGT |
|---|---|---|---|---|---|
| | | | SEQ ID NO:3211 | SEQ ID NO:11223 | SEQ ID NO:19235 |
| | | AA | RSSQSILYISNNKNYLA | WASTRES | QQYYSSPCS |
| | | | SEQ ID NO:3212 | SEQ ID NO:11224 | SEQ ID NO:19236 |
| iPS:436600 | 21-225_226F6 | NA | AAGTCCAGCCAGAGTATTTT ATACAGCTCCAACAATTACA ACTACTTAGCT | TGGGCTTCTACCCGGGA ATCC | CAGCAATATTATACTACTCC TCCGACG |
| | | | SEQ ID NO:3213 | SEQ ID NO:11225 | SEQ ID NO:19237 |
| | | AA | KSSQSILYSSNNYNYLA | WASTRES | QQYYTTPPT |
| | | | SEQ ID NO:3214 | SEQ ID NO:11226 | SEQ ID NO:19238 |
| iPS:436602 | 21-225_226E7 | NA | AAGTCTAGTCAGAGCCTCCT GCATGGTGATGGAAAGACCT ATTTGTAT | GAAGTTTCCAACCGGTTC TCT | ATGCAAAGTATACAGGTTCC GTGGACG |
| | | | SEQ ID NO:3215 | SEQ ID NO:11227 | SEQ ID NO:19239 |
| | | AA | KSSQSLLHGDGKTYLY | EVSNRFS | MQSIQVPWT |
| | | | SEQ ID NO:3216 | SEQ ID NO:11228 | SEQ ID NO:19240 |
| iPS:436604 | 21-225_226F7 | NA | CGGGCAAGTCAGGGCATTGG GAATGATTTAGGC | GCTGCCTCCAGTTTGCAA AGT | CTACATCATTATAGTTACCCT CGGACG |
| | | | SEQ ID NO:3217 | SEQ ID NO:11229 | SEQ ID NO:19241 |
| | | AA | RASQGIGNDLG | AASSLQS | LHHYSYPRT |
| | | | SEQ ID NO:3218 | SEQ ID NO:11230 | SEQ ID NO:19242 |
| iPS:436606 | 21-225_226G8 | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
| | | | SEQ ID NO:3219 | SEQ ID NO:11231 | SEQ ID NO:19243 |
| | | AA | KSSQSLLHSDGKTFLY | EISNRFS | MQSTQLPRT |
| | | | SEQ ID NO:3220 | SEQ ID NO:11232 | SEQ ID NO:19244 |

FIGURE 49

| iPS:436608 | | NA | CGGGCAAGTCAGGGCATTAG AAATGATTTAGGC | GCTGCATCCAGTTTGCAA AGT | CTACAGCATAATAGTTACCC ATTCACT |
| | 21-225_226A9 | | SEQ ID NO:3221 | SEQ ID NO:11233 | SEQ ID NO:19245 |
| | | AA | RASQGIRNDLG | AASSLQS | LQHNSYPFT |
| | | | SEQ ID NO:3222 | SEQ ID NO:11234 | SEQ ID NO:19246 |
| iPS:436610 | | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
| | 21-225_226F9 | | SEQ ID NO:3223 | SEQ ID NO:11235 | SEQ ID NO:19247 |
| | | AA | KSSQSLLHSDGKTFLY | EISNRFS | MQSTQLPRT |
| | | | SEQ ID NO:3224 | SEQ ID NO:11236 | SEQ ID NO:19248 |
| iPS:436612 | | NA | AAGTCTAGTCAGAGCCTCCT GCATAGTGATGGAAAGACCT TTTTGTAT | GAAGTTTCCAACCGGTTC TCT | ATGCAAAGTACACAGCTTCC TCGGACG |
| | 21-225_226H9 | | SEQ ID NO:3225 | SEQ ID NO:11237 | SEQ ID NO:19249 |
| | | AA | KSSQSLLHSDGKTFLY | EVSNRFS | MQSTQLPRT |
| | | | SEQ ID NO:3226 | SEQ ID NO:11238 | SEQ ID NO:19250 |
| iPS:436614 | | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
| | 21-225_226F10 | | SEQ ID NO:3227 | SEQ ID NO:11239 | SEQ ID NO:19251 |
| | | AA | KSSQSLLHSDGKTFLY | EISNRFS | MQSTQLPRT |
| | | | SEQ ID NO:3228 | SEQ ID NO:11240 | SEQ ID NO:19252 |
| iPS:436616 | | NA | AAGTCCAGCCAGAATGTTTT ACACAGCTCCAACAGTAATA ACTACTTAGTT | TGGGCATCTACCCGGGA ATCC | CAGCAATATTATAAAACTCC GTGGACG |
| | 21-225_226D11 | | SEQ ID NO:3229 | SEQ ID NO:11241 | SEQ ID NO:19253 |
| | | AA | KSSQNVLHSSNSNNYLV | WASTRES | QQYYKTPWT |
| | | | SEQ ID NO:3230 | SEQ ID NO:11242 | SEQ ID NO:19254 |

FIGURE 49

| iPS:436618 | | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
|---|---|---|---|---|---|
| | 21-225_226E11 | | SEQ ID NO:3231 | SEQ ID NO:11243 | SEQ ID NO:19255 |
| | | AA | KSSQSLLHSDGKTFLY | EISNRFS | MQSTQLPRT |
| | | | SEQ ID NO:3232 | SEQ ID NO:11244 | SEQ ID NO:19256 |
| iPS:436620 | | NA | CGGACAAGTCAGGGCATTAG AAATGATTTAGGC | GCTGCATCCAGTTTGCAA AGT | CTACAGCATTATAGTTACCC TCGGACG |
| | 21-225_226H11 | | SEQ ID NO:3233 | SEQ ID NO:11245 | SEQ ID NO:19257 |
| | | AA | RTSQGIRNDLG | AASSLQS | LQHYSYPRT |
| | | | SEQ ID NO:3234 | SEQ ID NO:11246 | SEQ ID NO:19258 |
| iPS:436622 | | NA | AAGTCCAGCCAGAATGTTTT ATACAGTTCCAACAATAACA ACTACTTAGCT | TGGGCATCTACCCGGAA ATCC | CAGCAATATTATAGTAGTCC TCCGACG |
| | 21-225_226A12 | | SEQ ID NO:3235 | SEQ ID NO:11247 | SEQ ID NO:19259 |
| | | AA | KSSQNVLYSSNNNNYLA | WASTRKS | QQYYSSPPT |
| | | | SEQ ID NO:3236 | SEQ ID NO:11248 | SEQ ID NO:19260 |
| iPS:436624 | | NA | AAGTCTAGTAAGACCCTCCT GCATAGTGATGGAAAGACCT TTTTGTAT | GAAATTTCCAACCGGTTC TCT | ATGCAAAGTACACAGCTTCC TCGGACG |
| | 21-225_226H12 | | SEQ ID NO:3237 | SEQ ID NO:11249 | SEQ ID NO:19261 |
| | | AA | KSSKTLLHSDGKTFLY | EISNRFS | MQSTQLPRT |
| | | | SEQ ID NO:3238 | SEQ ID NO:11250 | SEQ ID NO:19262 |
| iPS:436626 | | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
| | 21-225_227C1 | | SEQ ID NO:3239 | SEQ ID NO:11251 | SEQ ID NO:19263 |
| | | AA | KSSQSLLHSDGKTFLY | EISNRFS | MQSTQLPRT |
| | | | SEQ ID NO:3240 | SEQ ID NO:11252 | SEQ ID NO:19264 |

FIGURE 49

| iPS:436628 | | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
|---|---|---|---|---|---|
| | 21-225_227F2 | | SEQ ID NO:3241 | SEQ ID NO:11253 | SEQ ID NO:19265 |
| | | AA | KSSQSLLHSDGKTFLY | EISNRFS | MQSTQLPRT |
| | | | SEQ ID NO:3242 | SEQ ID NO:11254 | SEQ ID NO:19266 |
| iPS:436630 | | NA | CGGGCAAGTCAGGGCATTAG AAATGATTTAGGC | GCTGCATCCAGTTTGCAA AGT | CTACACCATAATAGTTACCC ATTCACT |
| | 21-225_227G3 | | SEQ ID NO:3243 | SEQ ID NO:11255 | SEQ ID NO:19267 |
| | | AA | RASQGIRNDLG | AASSLQS | LHHNSYPFT |
| | | | SEQ ID NO:3244 | SEQ ID NO:11256 | SEQ ID NO:19268 |
| iPS:436632 | | NA | CGGGCGAGTCAGGGTATTAT CAACTGGTTAGCC | GCTGCATCCAGTTTGCAA AGT | CAACAGGCTAACAGTTTCCC GTGGACG |
| | 21-225_227E4 | | SEQ ID NO:3245 | SEQ ID NO:11257 | SEQ ID NO:19269 |
| | | AA | RASQGIINWLA | AASSLQS | QQANSFPWT |
| | | | SEQ ID NO:3246 | SEQ ID NO:11258 | SEQ ID NO:19270 |
| iPS:436634 | | NA | CGGGCAAGTCAGGACATTAG AAATGATTTAGGC | GCTGCATCCAGTTTGCAA AGT | CTACAGCATAATAGTTACCC ATTCACT |
| | 21-225_227H5 | | SEQ ID NO:3247 | SEQ ID NO:11259 | SEQ ID NO:19271 |
| | | AA | RASQDIRNDLG | AASSLQS | LQHNSYPFT |
| | | | SEQ ID NO:3248 | SEQ ID NO:11260 | SEQ ID NO:19272 |
| iPS:436636 | | NA | AAGTCCAGCCAGAGTGTTTT ATACAGCTCCAACGATAAGA ACTACTTAGCT | TGGGGCATCTACCCGGGA ATCC | CAGCAATATTATATTACTCC GTGCAGT |
| | 21-225_227E6 | | SEQ ID NO:3249 | SEQ ID NO:11261 | SEQ ID NO:19273 |
| | | AA | KSSQSVLYSSNDKNYLA | WASTRES | QQYYITPCS |
| | | | SEQ ID NO:3250 | SEQ ID NO:11262 | SEQ ID NO:19274 |
| iPS:436638 | 21 225 227C7 | NA | AGGTCCAGCCAGATTGTTTT ATCCGACTCCAACAATAACA ACTACTTAGCT | TGGGGCATCTACCCGGGA ATCC | CAGCAATATTATAGTTCTCC TCCGACG |

FIGURE 49

| | | | SEQ ID NO:3251 | SEQ ID NO:11263 | SEQ ID NO:19275 |
|---|---|---|---|---|---|
| | 21-225_227C7 | AA | RSSQIVLSDSNNNNYLA | WASTRES | QQYYSSPPT |
| | | | SEQ ID NO:3252 | SEQ ID NO:11264 | SEQ ID NO:19276 |
| iPS:436640 | | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
| | 21-225_227A8 | | SEQ ID NO:3253 | SEQ ID NO:11265 | SEQ ID NO:19277 |
| | | AA | KSSQSLLHSDGKTFLY | EISNRFS | MQSTQLPRT |
| | | | SEQ ID NO:3254 | SEQ ID NO:11266 | SEQ ID NO:19278 |
| iPS:436644 | | NA | AAGTCCAGCCAGAGTGTTTT ACACAGCTCCAACAATAACA ACTACTTAGCT | TGGGGATCTACCCGGGA ATCC | CAGCAATATTATAGTGCTCC GTACAGT |
| | 21-225_227G9 | | SEQ ID NO:3255 | SEQ ID NO:11267 | SEQ ID NO:19279 |
| | | AA | KSSQSVLHSSNNNNYLA | WGSTRES | QQYYSAPYS |
| | | | SEQ ID NO:3256 | SEQ ID NO:11268 | SEQ ID NO:19280 |
| iPS:436646 | | NA | AAGTCCAGCCAGAGTGTTTT ACACAGTTCCAACAATAATA ACTACTTAGCT | TGGGCATCTACCCGGGA ATCC | CAGCAATATTATAATACTCC GTGCAGT |
| | 21-225_227D11 | | SEQ ID NO:3257 | SEQ ID NO:11269 | SEQ ID NO:19281 |
| | | AA | KSSQSVLHSSNNNNYLA | WASTRES | QQYYNTPCS |
| | | | SEQ ID NO:3258 | SEQ ID NO:11270 | SEQ ID NO:19282 |
| iPS:436648 | | NA | TGGTCTAGTCAGAGCCTCCT GCATAGTAATGGATACAACT ATTTGGAT | TTGGGTTCTAATCGGGCC TCC | ATGCAAGCTCTACAAACTCC TCTCACC |
| | 21-225_227F11 | | SEQ ID NO:3259 | SEQ ID NO:11271 | SEQ ID NO:19283 |
| | | AA | WSSQSLLHSNGYNYLD | LGSNRAS | MQALQTPLT |
| | | | SEQ ID NO:3260 | SEQ ID NO:11272 | SEQ ID NO:19284 |
| iPS:436650 | | NA | CGGGCAAGTCAGGGCATTAG AAATGATTTAGGC | GCTGCATCCAGTTTGCAA AGT | CTACAGCATAATAGTTACCC ATTCACT |
| | 21-225_227C12 | | SEQ ID NO:3261 | SEQ ID NO:11273 | SEQ ID NO:19285 |

EP 4 435 105 A2

FIGURE 49

| | | | | | |
|---|---|---|---|---|---|
| | | AA | RASQGIRNDLG | AASSLQS | LQHNSYPFT |
| | | | SEQ ID NO:3262 | SEQ ID NO:11274 | SEQ ID NO:19286 |
| iPS:436652 | 21-225_146B11 | NA | TCTGGAGATAAATTGGGGGATAAATATGCTTCC | CAAGATAGCAAGCGGCCCTCA | CAGGCGTGGGACAGCAGCACTGTGGTA |
| | | | SEQ ID NO:3263 | SEQ ID NO:11275 | SEQ ID NO:19287 |
| | | AA | SGDKLGDKYAS | QDSKRPS | QAWDSSTVV |
| | | | SEQ ID NO:3264 | SEQ ID NO:11276 | SEQ ID NO:19288 |
| iPS:436654 | 21-225_146C11 | NA | TCTGGAGATAAATTGGGGGATAAATATGCTTCC | CAAGATAGCAAGCGGCCCTCA | CAGGCGTGGGACAGCAGCACTGTGGTA |
| | | | SEQ ID NO:3265 | SEQ ID NO:11277 | SEQ ID NO:19289 |
| | | AA | SGDKLGDKYAS | QDSKRPS | QAWDSSTVV |
| | | | SEQ ID NO:3266 | SEQ ID NO:11278 | SEQ ID NO:19290 |
| iPS:436658 | 21-225_146A2 | NA | TCTGGAGATAAATTGGGGGATAAATATGCTTCC | CAAGATAGCAAGCGGCCCTCA | CAGGCGTGGGACAGCAGCACTGTGGTA |
| | | | SEQ ID NO:3267 | SEQ ID NO:11279 | SEQ ID NO:19291 |
| | | AA | SGDKLGDKYAS | QDSKRPS | QAWDSSTVV |
| | | | SEQ ID NO:3268 | SEQ ID NO:11280 | SEQ ID NO:19292 |
| iPS:436660 | 21-225_146D8 | NA | TCTGGAAGCAGCTCCTACATCGGAAGTAATACTGTAGAC | AGTAATAATCAGCGGCCCTCA | GCAGCATGGGATGACAGCCTTAATGGCGTGGTA |
| | | | SEQ ID NO:3269 | SEQ ID NO:11281 | SEQ ID NO:19293 |
| | | AA | SGSSSYIGSNTVD | SNNQRPS | AAWDDSLNGVV |
| | | | SEQ ID NO:3270 | SEQ ID NO:11282 | SEQ ID NO:19294 |
| iPS:436662 | 21-225_147D7 | NA | TCTGGAGATAAATTGGGGGATAAATTTGCTTGC | CAAGATAGGAAGCGGCCCTCA | CAGGCGTGGGACAGGAACACCGCTGTC |
| | | | SEQ ID NO:3271 | SEQ ID NO:11283 | SEQ ID NO:19295 |
| | | AA | SGDKLGDKFAC | QDRKRPS | QAWDRNTAV |
| | | | SEQ ID NO:3272 | SEQ ID NO:11284 | SEQ ID NO:19296 |
| iPS:436664 | | NA | TCTGGAGATAAATTGGGGGATAAATATGCTTCC | CAAGATAGCAAGCGCCCCTCA | CAGGCGTGGGACAGCAGCACTGTGGTA |

859

FIGURE 49

| | 21-225_147E7 | | SEQ ID NO:3273 | SEQ ID NO:11285 | SEQ ID NO:19297 |
|---|---|---|---|---|---|
| | | AA | SGDKLGDKYAS | QDSKRPS | QAWDSSTVV |
| iPS:436666 | | NA | SEQ ID NO:3274 TCTGGAGATAAATTGGGGGA TAAATATGTTTGC | SEQ ID NO:11286 CAAGATAGGAAGCGGCC CTCA | SEQ ID NO:19298 CAGGCGTGGGGCAGTAACAC TGCTGTGGTA |
| | 21-225_147B8 | | SEQ ID NO:3275 | SEQ ID NO:11287 | SEQ ID NO:19299 |
| | | AA | SGDKLGDKYVC | QDRKRPS | QAWGSNTAVV |
| iPS:436668 | | NA | SEQ ID NO:3276 TCTGGAGATAAATTGGGGGA TAAATATGTTTCC | SEQ ID NO:11288 CAAGATAGGAAGCGGCC CTCA | SEQ ID NO:19300 CTGGCGTGGGACAGCAGCAC TTTTGTGGTA |
| | 21-225_147B9 | | SEQ ID NO:3277 | SEQ ID NO:11289 | SEQ ID NO:19301 |
| | | AA | SGDKLGDKYVS | QDRKRPS | LAWDSSTFVV |
| iPS:436670 | | NA | SEQ ID NO:3278 TCTGGAGATAAATTGGGTAA TAAATATGTTTGC | SEQ ID NO:11290 CAAGATAGCAAGCGGCC CTCA | SEQ ID NO:19302 CAGGCGTGGGACAGGAACAC TTATGTGGTG |
| | 21-225_147D9 | | SEQ ID NO:3279 | SEQ ID NO:11291 | SEQ ID NO:19303 |
| | | AA | SGDKLGNKYVC | QDSKRPS | QAWDRNTYVV |
| iPS:436672 | | NA | SEQ ID NO:3280 TCTGGAGATGAATTGGGGAA TAAATATGCTTGC | SEQ ID NO:11292 CAAGATAGCAAGCGGCC CTCA | SEQ ID NO:19304 CAGGCGTGGCACAGCAGCAC TGTGGTA |
| | 21-225_147F9 | | SEQ ID NO:3281 | SEQ ID NO:11293 | SEQ ID NO:19305 |
| | | AA | SGDELGNKYAC | QDSKRPS | QAWHSSTVV |
| iPS:436674 | | NA | SEQ ID NO:3282 TCTGGAGATAAATTGGGGGA TAAATATGCTTGC | SEQ ID NO:11294 CAAGATAGGAAGCGGCC CTCA | SEQ ID NO:19306 CAGGCGTGGCACAGCAGTAC TGTGGTA |
| | 21-225_147G9 | | SEQ ID NO:3283 | SEQ ID NO:11295 | SEQ ID NO:19307 |
| | | AA | SGDKLGDKYAC | QDRKRPS | QAWHSSTVV |
| iPS:436676 | | NA | SEQ ID NO:3284 TCTGGAGATAAATTGGGGGA TAAATATGCTTCC | SEQ ID NO:11296 CAAGATAGCAAGCGGCC CTCA | SEQ ID NO:19308 CAGGCGTGGGACAGCAGCAC TGTGGTA |

FIGURE 49

| | 21-225_147E11 | | SEQ ID NO:3285 | SEQ ID NO:11297 | SEQ ID NO:19309 |
|---|---|---|---|---|---|
| | | AA | SGDKLGDKYAS | QDSKRPS | QAWDSSTVV |
| iPS:436678 | | | SEQ ID NO:3286 | SEQ ID NO:11298 | SEQ ID NO:19310 |
| | 21-225_147B12 | NA | TCTGGAGATAAATTGGGGGATAAATATGCTTCC | CAAGATAGCAAGCGGCCCTCA | CAGGCGTGGGACAGCAGCACTGTGGTA |
| | | | SEQ ID NO:3287 | SEQ ID NO:11299 | SEQ ID NO:19311 |
| | | AA | SGDKLGDKYAS | QDSKRPS | QAWDSSTVV |
| iPS:436680 | | | SEQ ID NO:3288 | SEQ ID NO:11300 | SEQ ID NO:19312 |
| | 21-225_147H12 | NA | TCTGGAAGCAGCTCCAACATCGGAAGTTATGCTGTAAAC | AGTAATAATCACCGGCCCTCA | GAAGCATGGGATGACAGCCTGAATGGTCCGGTA |
| | | | SEQ ID NO:3289 | SEQ ID NO:11301 | SEQ ID NO:19313 |
| | | AA | SGSSSNIGSYAVN | SNNHRPS | EAWDDSLNGPV |
| iPS:436682 | | | SEQ ID NO:3290 | SEQ ID NO:11302 | SEQ ID NO:19314 |
| | 21-225_146A8 | NA | TCTGGAAGCAGCTCCAACATCGGAAGTAATTCTATAAAC | AGTAATGATCAGCGGCCCTCA | GCAGCATGGGATGACAGCCTGAACGGCGTGGTA |
| | | | SEQ ID NO:3291 | SEQ ID NO:11303 | SEQ ID NO:19315 |
| | | AA | SGSSSNIGSNSIN | SNDQRPS | AAWDDSLNGVV |
| iPS:436684 | | | SEQ ID NO:3292 | SEQ ID NO:11304 | SEQ ID NO:19316 |
| | 21-225_146B6 | NA | TCTGGAAGCAGCTCCAACATCGGAAGTAATGCTGTAAAC | AGTAATAATCAGCGGCCCTCA | GCAGCATGGGATGACAGCCTGAATGGCGTGGTA |
| | | | SEQ ID NO:3293 | SEQ ID NO:11305 | SEQ ID NO:19317 |
| | | AA | SGSSSNIGSNAVN | SNNQRPS | AAWDDSLNGVV |
| iPS:436686 | | | SEQ ID NO:3294 | SEQ ID NO:11306 | SEQ ID NO:19318 |
| | 21-225_148G6 | NA | TCTGGAGATAAATTGGGGGATAAATATGCTTCC | CAAGATAGCAAGCGGCCCTCA | CAGGCGTGGGACAGCAGCACTGTGGTA |
| | | | SEQ ID NO:3295 | SEQ ID NO:11307 | SEQ ID NO:19319 |
| | | AA | SGDKLGDKYAS | QDSKRPS | QAWDSSTVV |

FIGURE 49

| | | | SEQ ID NO:3296 | SEQ ID NO:11308 | SEQ ID NO:19320 |
|---|---|---|---|---|---|
| iPS:436688 | | NA | TCTGGAGATAAATTGGGGGA TAAATATGTTTCC | CAAGATAGGAAGCGGCC CTCA | CTGGCGTGGGACAGCAGCAC TTTTGTGGTA |
| | 21-225_148C8 | | SEQ ID NO:3297 | SEQ ID NO:11309 | SEQ ID NO:19321 |
| | | AA | SGDKLGDKYVS | QDRKRPS | LAWDSSTFVV |
| | | | SEQ ID NO:3298 | SEQ ID NO:11310 | SEQ ID NO:19322 |
| iPS:436690 | | NA | TCTGGAGATAAATTGGGGAA TAAATATGTTTGC | CAAGATAGCAAGCGGCC CTCA | CAGGCGTGGCACAGCAGCAC TGTGGTA |
| | 21-225_148A9 | | SEQ ID NO:3299 | SEQ ID NO:11311 | SEQ ID NO:19323 |
| | | AA | SGDKLGNKYVC | QDSKRPS | QAWHSSTVV |
| | | | SEQ ID NO:3300 | SEQ ID NO:11312 | SEQ ID NO:19324 |
| iPS:436694 | | NA | TCTGGAGATAAATTGGGGGA TAAATTTGCTTCC | CAAGATAGCAAGCGGCC CTCA | CAGGCGTGGGACAGCAGCAC TGTGGTA |
| | 21-225_148G11 | | SEQ ID NO:3301 | SEQ ID NO:11313 | SEQ ID NO:19325 |
| | | AA | SGDKLGDKFAS | QDSKRPS | QAWDSSTVV |
| | | | SEQ ID NO:3302 | SEQ ID NO:11314 | SEQ ID NO:19326 |
| iPS:436696 | | NA | TCTGGAAGCAGCTCCAACAT CGGAAGTAATGCTGTAAAC | AGTAATAATCAGCGGCC CTCA | GCAGCATGGGATGACAGCCT GAATGGCGTGGTA |
| | 21-225_149A1 | | SEQ ID NO:3303 | SEQ ID NO:11315 | SEQ ID NO:19327 |
| | | AA | SGSSSNIGSNAVN | SNNQRPS | AAWDDSLNGVV |
| | | | SEQ ID NO:3304 | SEQ ID NO:11316 | SEQ ID NO:19328 |
| iPS:436698 | | NA | TCTGGATATAAATTGGGGTA TAAATATGTTTGC | CAAAATAACCAGCGGCC CTCA | CAGGCGTGGGACAGCAGCAC TGTGGTA |
| | 21-225_149B5 | | SEQ ID NO:3305 | SEQ ID NO:11317 | SEQ ID NO:19329 |
| | | AA | SGYKLGYKYVC | QNNQRPS | QAWDSSTVV |
| | | | SEQ ID NO:3306 | SEQ ID NO:11318 | SEQ ID NO:19330 |
| iPS:436700 | | NA | TCTGGAAATAAATTGGGGGA TAAATATGCTTCC | CAAGATAGCAAGCGGCC CTCA | CAGGCGTGGGACAGCAGCAC TGTGGTA |
| | 21-225_149C7 | | SEQ ID NO:3307 | SEQ ID NO:11319 | SEQ ID NO:19331 |

FIGURE 49

| | | AA | SGNKLGDKYAS | QDSKRPS | QAWDSSTVV |
|---|---|---|---|---|---|
| | | | SEQ ID NO:3308 | SEQ ID NO:11320 | SEQ ID NO:19332 |
| iPS:436702 | | NA | ACCTTACGCAGTGGCATCAC TGTTACTACCTATAGGATAT AC | TACACATCAGACTCAGA TAAACACCAGGGCTCT | ATGATTTGGCACAGCAGCGC TTGGGTG |
| | 21-225_149E8 | | SEQ ID NO:3309 | SEQ ID NO:11321 | SEQ ID NO:19333 |
| | | AA | TLRSGITVTTYRIY | YTSDSDKHQGS | MIWHSSAWV |
| | | | SEQ ID NO:3310 | SEQ ID NO:11322 | SEQ ID NO:19334 |
| iPS:436704 | | NA | TCTGGAGATAAATTGGGGGA TAAATATGCTTCC | CAAGATAACAAGCGGCC CTCA | CAGGCGTGGGACAGCAGCAC TGTGGTA |
| | 21-225_149C10 | | SEQ ID NO:3311 | SEQ ID NO:11323 | SEQ ID NO:19335 |
| | | AA | SGDKLGDKYAS | QDNKRPS | QAWDSSTVV |
| | | | SEQ ID NO:3312 | SEQ ID NO:11324 | SEQ ID NO:19336 |
| iPS:436706 | | NA | TCTGGAGATAAATTGGGGAA TAAATATGTTTCC | CAAGATAGCAGGCGGCC CTCA | CTGGCGTGGGACAGCAGCAC TTTTGTGGTC |
| | 21-225_149A11 | | SEQ ID NO:3313 | SEQ ID NO:11325 | SEQ ID NO:19337 |
| | | AA | SGDKLGNKYVS | QDSRRPS | LAWDSSTFVV |
| | | | SEQ ID NO:3314 | SEQ ID NO:11326 | SEQ ID NO:19338 |
| iPS:436708 | | NA | TCTGGAGATGAATTGGGGAA TAAATATGCTTGC | CAAGATAACAAGCGGCC CTCA | CAGGCGTGGCACAGCAGCAC TGTGGTA |
| | 21-225_150D3 | | SEQ ID NO:3315 | SEQ ID NO:11327 | SEQ ID NO:19339 |
| | | AA | SGDELGNKYAC | QDNKRPS | QAWHSSTVV |
| | | | SEQ ID NO:3316 | SEQ ID NO:11328 | SEQ ID NO:19340 |
| iPS:436710 | | NA | TCTGGAGATAAATTGGGGGA TAAATATGCTTCC | CAAGATAGCAAGCGGCC CTCA | CAGGCGTGGGACAGCAGCAC TGTGGTA |
| | 21-225_150F6 | | SEQ ID NO:3317 | SEQ ID NO:11329 | SEQ ID NO:19341 |
| | | AA | SGDKLGDKYAS | QDSKRPS | QAWDSSTVV |
| | | | SEQ ID NO:3318 | SEQ ID NO:11330 | SEQ ID NO:19342 |

FIGURE 49

| iPS:436712 | | NA | TCTGGAAGCAGCTCCAACAT CGGAAGTAATGCTGTAAAC | AGTAATAGTCAGCGGCC CTCA | GCAGCATGGGATGACAGCCT GAATGGCGTGGTA |
|---|---|---|---|---|---|
| | 21-225_150F9 | | SEQ ID NO:3319 | SEQ ID NO:11331 | SEQ ID NO:19343 |
| | | AA | SGSSSNIGSNAVN | SNSQRPS | AAWDDSLNGVV |
| | | | SEQ ID NO:3320 | SEQ ID NO:11332 | SEQ ID NO:19344 |
| iPS:436714 | | NA | TCTGGAGATAAATTGGGGGA TAAATATGCTTCC | CAAGATAGCAAGCGGCC CTCA | CAGGCGTGGGACAGCAGCAC TGTGGTA |
| | 21-225_150H11 | | SEQ ID NO:3321 | SEQ ID NO:11333 | SEQ ID NO:19345 |
| | | AA | SGDKLGDKYAS | QDSKRPS | QAWDSSTVV |
| | | | SEQ ID NO:3322 | SEQ ID NO:11334 | SEQ ID NO:19346 |
| iPS:436716 | | NA | TCTGGAGATAAATTGGGGGA TAAATATGTTTGC | CAAGATCGCAAGCGGCC CTCA | CAGGCGTGGCACAGCAGCAC TGTGGTA |
| | 21-225_151F3 | | SEQ ID NO:3323 | SEQ ID NO:11335 | SEQ ID NO:19347 |
| | | AA | SGDKLGDKYVC | QDRKRPS | QAWHSSTVV |
| | | | SEQ ID NO:3324 | SEQ ID NO:11336 | SEQ ID NO:19348 |
| iPS:436718 | | NA | TCTGGAGATAAATTGGGGGA TAAATATGCTTCC | CAAGATAGCAAGCGGCC CTCA | CAGGCGTGGGACAGCAGCAC TGTGGTA |
| | 21-225_151H5 | | SEQ ID NO:3325 | SEQ ID NO:11337 | SEQ ID NO:19349 |
| | | AA | SGDKLGDKYAS | QDSKRPS | QAWDSSTVV |
| | | | SEQ ID NO:3326 | SEQ ID NO:11338 | SEQ ID NO:19350 |
| iPS:436720 | | NA | TCTGGAGATAAATTGGGGGA TAAATATGCTTGC | CAAGATACCAAGCGGCC CTCA | CAGGCGTGGGACAGCAGCAC TTATGTC |
| | 21-225_151H6 | | SEQ ID NO:3327 | SEQ ID NO:11339 | SEQ ID NO:19351 |
| | | AA | SGDKLGDKYAC | QDTKRPS | QAWDSSTYV |
| | | | SEQ ID NO:3328 | SEQ ID NO:11340 | SEQ ID NO:19352 |
| iPS:436722 | | NA | TCTGGAGATAAATTGGGGGA TAAATATGCTTCC | CAAGATAGCAAGCGGCC CTCA | CAGGCGTGGGACAGCAGCAC TGTGGTA |
| | 21-225_151H7 | | SEQ ID NO:3329 | SEQ ID NO:11341 | SEQ ID NO:19353 |
| | | AA | SGDKLGDKYAS | QDSKRPS | QAWDSSTVV |

864

FIGURE 49

| | | | SEQ ID NO:3330 | SEQ ID NO:11342 | SEQ ID NO:19354 |
|---|---|---|---|---|---|
| iPS:436724 | | NA | TCTGGAGATAATTTGGGGGATAAATATGCTTCC | CAAGATAGCAAGCGGCCCTCA | CAGGCGTGGGACAGCAGCACTGTGGTA |
| | 21-225_151B9 | | SEQ ID NO:3331 | SEQ ID NO:11343 | SEQ ID NO:19355 |
| | | AA | SGDNLGDKYAS | QDSKRPS | QAWDSSTVV |
| | | | SEQ ID NO:3332 | SEQ ID NO:11344 | SEQ ID NO:19356 |
| iPS:436726 | | NA | TCTGGAGATAAATTGGGGGATAAATATGCTTGC | CAAGATTCCAAGCGGCCCTCA | CAGGCGTGGGACAGCAGCACTTATGTC |
| | 21-225_152G5 | | SEQ ID NO:3333 | SEQ ID NO:11345 | SEQ ID NO:19357 |
| | | AA | SGDKLGDKYAC | QDSKRPS | QAWDSSTYV |
| | | | SEQ ID NO:3334 | SEQ ID NO:11346 | SEQ ID NO:19358 |
| iPS:436728 | | NA | TCTGGAGATAAATTGGGGGATAAATATGCTTCC | CAAGATAGCAAGCGGCCCTCA | CAGGCGTGGGACAACAGCACTGTGGTA |
| | 21-225_152G6 | | SEQ ID NO:3335 | SEQ ID NO:11347 | SEQ ID NO:19359 |
| | | AA | SGDKLGDKYAS | QDSKRPS | QAWDNSTVV |
| | | | SEQ ID NO:3336 | SEQ ID NO:11348 | SEQ ID NO:19360 |
| iPS:436730 | | NA | TCTGGAGATAAATTGGGGGATAAATATGCTTCC | CAAGATAGCAAGCGGCCCTCA | CAGGCGTGGGACAGCAGCACTGTGGTA |
| | 21-225_152D7 | | SEQ ID NO:3337 | SEQ ID NO:11349 | SEQ ID NO:19361 |
| | | AA | SGDKLGDKYAS | QDSKRPS | QAWDSSTVV |
| | | | SEQ ID NO:3338 | SEQ ID NO:11350 | SEQ ID NO:19362 |
| iPS:436732 | | NA | TCTGGCGATAAATTGGGAAATAAATATGCTTGC | CAAGATACCAAGCGGCCCTCA | CAGGCGTGGGACAGCAGCACTTATGTC |
| | 21-225_152B12 | | SEQ ID NO:3339 | SEQ ID NO:11351 | SEQ ID NO:19363 |
| | | AA | SGDKLGNKYAC | QDTKRPS | QAWDSSTYV |
| | | | SEQ ID NO:3340 | SEQ ID NO:11352 | SEQ ID NO:19364 |
| iPS:436734 | | NA | TCTGGAGATAAATTGGGGGATAAATATGCTTGC | CAAGATACCAAGCGGCCCTCA | CAGGCGTGGGACAGCAGCACTTATGTC |
| | 21-225_153A8 | | SEQ ID NO:3341 | SEQ ID NO:11353 | SEQ ID NO:19365 |
| | | AA | SGDKLGDKYAC | QDTKRPS | QAWDSSTYV |

FIGURE 49

| | | | SEQ ID NO:3342 | SEQ ID NO:11354 | SEQ ID NO:19366 |
|---|---|---|---|---|---|
| iPS:436736 | | NA | TCTGGAAGTAAATTGGGTAATAAATATGTTTGC | CAAGATAACAAGCGGCCCTCA | CAGGCGTGGGACAGCAGCACTTATGTGATA |
| | 21-225_153E8 | | SEQ ID NO:3343 | SEQ ID NO:11355 | SEQ ID NO:19367 |
| | | AA | SGSKLGNKYVC | QDNKRPS | QAWDSSTYVI |
| | | | SEQ ID NO:3344 | SEQ ID NO:11356 | SEQ ID NO:19368 |
| iPS:436738 | | NA | TCTGGAGATAAATTGGGGGATAAATATGCTTGC | CAAGATAGGAAGCGGCCCTCA | CAGGCGTGGCACAGCAGTACTGTGGTA |
| | 21-225_153D9 | | SEQ ID NO:3345 | SEQ ID NO:11357 | SEQ ID NO:19369 |
| | | AA | SGDKLGDKYAC | QDRKRPS | QAWHSSTVV |
| | | | SEQ ID NO:3346 | SEQ ID NO:11358 | SEQ ID NO:19370 |
| iPS:436740 | | NA | TCTGGAGATAAATTGGGGGATAAATATGTTTGC | CAAGATAGGAAGCGGCCCTCA | CAGGCGTGGCACAGCAGCACTGTGGTA |
| | 21-225_154C3 | | SEQ ID NO:3347 | SEQ ID NO:11359 | SEQ ID NO:19371 |
| | | AA | SGDKLGDKYVC | QDRKRPS | QAWHSSTVV |
| | | | SEQ ID NO:3348 | SEQ ID NO:11360 | SEQ ID NO:19372 |
| iPS:436742 | | NA | TCTGGAGATAAATTGGGGGATAAATATGCTTCC | CAAGATAGCAAGCGGCCCTCA | CAGGCGTGGGACAGCAGCACTGTGGTA |
| | 21-225_154C4 | | SEQ ID NO:3349 | SEQ ID NO:11361 | SEQ ID NO:19373 |
| | | AA | SGDKLGDKYAS | QDSKRPS | QAWDSSTVV |
| | | | SEQ ID NO:3350 | SEQ ID NO:11362 | SEQ ID NO:19374 |
| iPS:436744 | | NA | TCTGGAGATAAATTGGGAAATAAATATGTTTGT | AAAGATAGTAAGCGGCCCTCA | CAGGCGTGGGACAACAGTACTTTAGTA |
| | 21-225_154F4 | | SEQ ID NO:3351 | SEQ ID NO:11363 | SEQ ID NO:19375 |
| | | AA | SGDKLGNKYVC | KDSKRPS | QAWDNSTLV |
| | | | SEQ ID NO:3352 | SEQ ID NO:11364 | SEQ ID NO:19376 |
| iPS:436746 | | NA | TCTGGAGATAAATTGGGGGATAAATATGCTTCC | CAAGATAGCAAGCGGCCCTCA | CAGGCGTGGGACAACAGCACTGTGGTA |
| | 21-225_154E10 | | SEQ ID NO:3353 | SEQ ID NO:11365 | SEQ ID NO:19377 |
| | | AA | SGDKLGDKYAS | QDSKRPS | QAWDNSTVV |

FIGURE 49

| | | | SEQ ID NO:3354 | SEQ ID NO:11366 | SEQ ID NO:19378 |
|---|---|---|---|---|---|
| iPS:436748 | | NA | TCTGGAGATAAATTGGGGGATAAATATGCTTGC | CAAGATAAGAAGCGGCCCTCA | CAGGCGTGGCACAGCAGTATTGTGGTA |
| | 21-225_154D11 | | SEQ ID NO:3355 | SEQ ID NO:11367 | SEQ ID NO:19379 |
| | | AA | SGDKLGDKYAC | QDKKRPS | QAWHSSIVV |
| | | | SEQ ID NO:3356 | SEQ ID NO:11368 | SEQ ID NO:19380 |
| iPS:436750 | | NA | TCTGGAAGCAGCTCCAACATCGGAAATAATGCTGTAAGC | AGTAATGATCACCGGCCCTCA | GCAGCATGGGATGACAGCCTGAAGGGTCCGGTA |
| | 21-225_154G12 | | SEQ ID NO:3357 | SEQ ID NO:11369 | SEQ ID NO:19381 |
| | | AA | SGSSSNIGNNAVS | SNDHRPS | AAWDDSLKGPV |
| | | | SEQ ID NO:3358 | SEQ ID NO:11370 | SEQ ID NO:19382 |
| iPS:436752 | | NA | ACTGGGAGCAGCTCCAATATCGGGGCAGGTTATGATGTACAC | GGTAACAGCAATCGGCCCTCA | CAGTCCTATGACAGCAGCCTGAGTGGTCCTGTGATA |
| | 21-225_155H1 | | SEQ ID NO:3359 | SEQ ID NO:11371 | SEQ ID NO:19383 |
| | | AA | TGSSSNIGAGYDVH | GNSNRPS | QSYDSSLSGPVI |
| | | | SEQ ID NO:3360 | SEQ ID NO:11372 | SEQ ID NO:19384 |
| iPS:436754 | | NA | TCTGGAGATAAGTTGGGGGATAAATATGTTTGC | CAAGATAGTAAGCGGCCCTCA | CAGGCGTGGGACAATAGTATTTATGTC |
| | 21-225_155G3 | | SEQ ID NO:3361 | SEQ ID NO:11373 | SEQ ID NO:19385 |
| | | AA | SGDKLGDKYVC | QDSKRPS | QAWDNSIYV |
| | | | SEQ ID NO:3362 | SEQ ID NO:11374 | SEQ ID NO:19386 |
| iPS:436756 | | NA | TCTGGAGATAAATTGGGGGATAAATATGTTTGC | CAAGATAGAAAGCGGCCCTCA | CAGGCGTGGGACAGCAGCACTGTGGTG |
| | 21-225_146A10 | | SEQ ID NO:3363 | SEQ ID NO:11375 | SEQ ID NO:19387 |
| | | AA | SGDKLGDKYVC | QDRKRPS | QAWDSSTVV |
| | | | SEQ ID NO:3364 | SEQ ID NO:11376 | SEQ ID NO:19388 |
| iPS:436758 | | NA | TCTGGAGATAAATTGGGGGATAAATATGTTTCC | CAAGATAGCAAGCGGCCCTCA | CAGGCGTGGGACAGCAGCACTGTGGTA |

FIGURE 49

| | 21-225_155C10 | | SEQ ID NO:3365 | SEQ ID NO:11377 | SEQ ID NO:19389 |
|---|---|---|---|---|---|
| | | AA | SGDKLGDKYVS | QDSKRPS | QAWDSSTVV |
| | | | SEQ ID NO:3366 | SEQ ID NO:11378 | SEQ ID NO:19390 |
| iPS:436760 | | NA | TCTGGAGATAAATTGGGGGATAAATATGTTTCC | CAAGATAGGAAGCGGCCCTCA | CTGGCGTGGGACAGCAGCACTTTTGTGGTA |
| | 21-225_155E10 | | SEQ ID NO:3367 | SEQ ID NO:11379 | SEQ ID NO:19391 |
| | | AA | SGDKLGDKYVS | QDRKRPS | LAWDSSTFVV |
| | | | SEQ ID NO:3368 | SEQ ID NO:11380 | SEQ ID NO:19392 |
| iPS:436762 | | NA | TCTGGAAGCAGCTCCAACATCGGAAGTAATACTGTAAAT | AGTAGTAATCAGCGGCCCTCA | GCAGCATGGGATGACAGCCTGAATGGCGTGGTA |
| | 21-225_156H2 | | SEQ ID NO:3369 | SEQ ID NO:11381 | SEQ ID NO:19393 |
| | | AA | SGSSSNIGSNTVN | SSNQRPS | AAWDDSLNGVV |
| | | | SEQ ID NO:3370 | SEQ ID NO:11382 | SEQ ID NO:19394 |
| iPS:436764 | | NA | TCTGGAGATAAATTGGGGGATAAATATGTTTGC | CAAGATAGTAAGCGGCCCTCA | CAGGCGTGGGACAACAGCAGCTTTGTGCTA |
| | 21-225_158E9 | | SEQ ID NO:3371 | SEQ ID NO:11383 | SEQ ID NO:19395 |
| | | AA | SGDKLGDKYVC | QDSKRPS | QAWDNSSFVL |
| | | | SEQ ID NO:3372 | SEQ ID NO:11384 | SEQ ID NO:19396 |
| iPS:436766 | | NA | TCTGGAGATAAATTGGGGGATAAATATGTTTGC | CAAGATCGCAAGCGGCCCTCA | CAGGCGTGGGGCAACAGCAGCTTTGTGGTA |
| | 21-225_158D10 | | SEQ ID NO:3373 | SEQ ID NO:11385 | SEQ ID NO:19397 |
| | | AA | SGDKLGDKYVC | QDRKRPS | QAWGNSSFVV |
| | | | SEQ ID NO:3374 | SEQ ID NO:11386 | SEQ ID NO:19398 |
| iPS:436768 | | NA | TCTGGAGATAAATTGGGGGATAAATATGTTTGC | CAAGATAGGAAGCGGCCCTCA | CAGGCGTGGGGCAACAGCAGCTTTGTGGTA |
| | 21-225_159H8 | | SEQ ID NO:3375 | SEQ ID NO:11387 | SEQ ID NO:19399 |
| | | AA | SGDKLGDKYVC | QDRKRPS | QAWGNSSFVV |
| | | | SEQ ID NO:3376 | SEQ ID NO:11388 | SEQ ID NO:19400 |

FIGURE 49

| iPS:436770 | | NA | TCTGGAGATAAATTGGGGGA TAAATATGTTTGC | CAAGATAGCAAGCGGCC CTCA | CAGGCGTGGGGCAACAGCAG CTTTGTGGTA |
|---|---|---|---|---|---|
| | 21-225_160B12 | | SEQ ID NO:3377 | SEQ ID NO:11389 | SEQ ID NO:19401 |
| | | AA | SGDKLGDKYVC | QDSKRPS | QAWGNSSFVV |
| | | | SEQ ID NO:3378 | SEQ ID NO:11390 | SEQ ID NO:19402 |
| iPS:436772 | | NA | TCTGGAGATAGATTGGGGGA TAAATATGTTTGC | CAAGATAACAAGCGGCC CTCA | CAGGCGTGGGTCAACAACAC TGCAGTGGTT |
| | 21-225_161H3 | | SEQ ID NO:3379 | SEQ ID NO:11391 | SEQ ID NO:19403 |
| | | AA | SGDRLGDKYVC | QDNKRPS | QAWVNNTAVV |
| | | | SEQ ID NO:3380 | SEQ ID NO:11392 | SEQ ID NO:19404 |
| iPS:436774 | | NA | TCTGGAGATAAATTGGGGGA TAAATATGTTTGC | CAAGATAGCAAGCGGCC CTCA | CAGACGTGGGACAACAGTAG TTTTGCGCTT |
| | 21-225_161E10 | | SEQ ID NO:3381 | SEQ ID NO:11393 | SEQ ID NO:19405 |
| | | AA | SGDKLGDKYVC | QDSKRPS | QTWDNSSFAL |
| | | | SEQ ID NO:3382 | SEQ ID NO:11394 | SEQ ID NO:19406 |
| iPS:436776 | | NA | TCTGGAGATAAATTGGGTGA TAAATATGCTTGC | CAAGATACCAAGCGGCC CTCA | CAGGCGTGGGACAGCACCAC TCTGGTT |
| | 21-225_161F12 | | SEQ ID NO:3383 | SEQ ID NO:11395 | SEQ ID NO:19407 |
| | | AA | SGDKLGDKYAC | QDTKRPS | QAWDSTTLV |
| | | | SEQ ID NO:3384 | SEQ ID NO:11396 | SEQ ID NO:19408 |
| iPS:436780 | | NA | TCTGGAGATAAATTGGGTGA TAAATATGCTTGC | CAAGATAGCAAGCGGCC CTCA | CAGGCGTGGGACAGCACCAC TCTGGTT |
| | 21-225_165H3 | | SEQ ID NO:3385 | SEQ ID NO:11397 | SEQ ID NO:19409 |
| | | AA | SGDKLGDKYAC | QDSKRPS | QAWDSTTLV |
| | | | SEQ ID NO:3386 | SEQ ID NO:11398 | SEQ ID NO:19410 |
| iPS:436782 | | NA | TCTGGAGATAAATTGGGGGA TAAATATGTTCAC | CAAGATAGCAAGCGGCC CTCA | CAGGCGTGGGACAACAGCAC TGCGGTA |
| | 21-225_166G11 | | SEQ ID NO:3387 | SEQ ID NO:11399 | SEQ ID NO:19411 |
| | | AA | SGDKLGDKYVH | QDSKRPS | QAWDNSTAV |
| | | | SEQ ID NO:3388 | SEQ ID NO:11400 | SEQ ID NO:19412 |

FIGURE 49

| iPS:436784 | | NA | TCTGGAGATAAATTGGGGGA TAAATATGTTTGT | AAAGATATCAAGCGGCC CTCA | CAGGCGTGGGACACCAACAC TGTGATA |
|---|---|---|---|---|---|
| | 21-225_169C1 | | SEQ ID NO:3389 | SEQ ID NO:11401 | SEQ ID NO:19413 |
| | | AA | SGDKLGDKYVC | KDIKRPS | QAWDTNTVI |
| | | | SEQ ID NO:3390 | SEQ ID NO:11402 | SEQ ID NO:19414 |
| iPS:436786 | | NA | TCTGGAGATAAATTGGGGGA TAAATATGTTTGT | CAGGATTACAAGCGGCC CTCA | CAGGCGTGGGACACCAACAC TGTGCTT |
| | 21-225_169A6 | | SEQ ID NO:3391 | SEQ ID NO:11403 | SEQ ID NO:19415 |
| | | AA | SGDKLGDKYVC | QDYKRPS | QAWDTNTVL |
| | | | SEQ ID NO:3392 | SEQ ID NO:11404 | SEQ ID NO:19416 |
| iPS:436788 | | NA | TCTGGAGATAAATTGGGGGG AAAATATGCTTCC | CAAGATAGGAAGCGGCC CTCA | CAGGCGTGGGACAAGAACAC TGTGGTA |
| | 21-225_169B7 | | SEQ ID NO:3393 | SEQ ID NO:11405 | SEQ ID NO:19417 |
| | | AA | SGDKLGGKYAS | QDRKRPS | QAWDKNTVV |
| | | | SEQ ID NO:3394 | SEQ ID NO:11406 | SEQ ID NO:19418 |
| iPS:436790 | | NA | TCTGGAGATAAATTGGGGGA TAAATATGCTTGC | CAAGATAGTAAGCGGCC CTCA | CAGGCGTGGGACAACAGCAC TGCGGTA |
| | 21-225_169G11 | | SEQ ID NO:3395 | SEQ ID NO:11407 | SEQ ID NO:19419 |
| | | AA | SGDKLGDKYAC | QDSKRPS | QAWDNSTAV |
| | | | SEQ ID NO:3396 | SEQ ID NO:11408 | SEQ ID NO:19420 |
| iPS:436792 | | NA | ACCCGCAGCAGTGGCAGCAT TACCGGCAACTATGTGCAG | GAGGATAAAAAAAGACC CTCT | CAGTCTTATTATAGCGGCAA TTGGGTG |
| | 21-225_169D12 | | SEQ ID NO:3397 | SEQ ID NO:11409 | SEQ ID NO:19421 |
| | | AA | TRSSGSITGNYVQ | EDKKRPS | QSYYSGNWV |
| | | | SEQ ID NO:3398 | SEQ ID NO:11410 | SEQ ID NO:19422 |
| iPS:436794 | | NA | TCTGGAGATAAATTGGGGGA TAAATATTCTTGC | CAAGATAGTAAGCGGCC CTCA | CAGGCGTGGGACAGCAACAC TGCGGTA |
| | 21-225_170F1 | | SEQ ID NO:3399 | SEQ ID NO:11411 | SEQ ID NO:19423 |
| | | AA | SGDKLGDKYSC | QDSKRPS | QAWDSNTAV |

FIGURE 49

| | | | SEQ ID NO:3400 | SEQ ID NO:11412 | SEQ ID NO:19424 |
|---|---|---|---|---|---|
| iPS:436796 | | NA | TCTGGAGATAAATTGGGGGATAAATATGCTTGT | CAAGATTACAAGCGGCCCTCA | CAGGCGTGGGACAACAGCACTATGGTA |
| | 21-225_170A5 | | SEQ ID NO:3401 | SEQ ID NO:11413 | SEQ ID NO:19425 |
| | | AA | SGDKLGDKYAC | QDYKRPS | QAWDNSTMV |
| | | | SEQ ID NO:3402 | SEQ ID NO:11414 | SEQ ID NO:19426 |
| iPS:436798 | | NA | TCTGGAGATAAATTGGGGGGAAAAATATGCTTCC | CAAGATAGGAAGCGGCCCTCA | CAGGCGTGGGACAAGAACACTGTGGTA |
| | 21-225_171F5 | | SEQ ID NO:3403 | SEQ ID NO:11415 | SEQ ID NO:19427 |
| | | AA | SGDKLGGKYAS | QDRKRPS | QAWDKNTVV |
| | | | SEQ ID NO:3404 | SEQ ID NO:11416 | SEQ ID NO:19428 |
| iPS:436800 | | NA | CAAGGAGACAGCCTCAGAAGCTATTATGCAAGC | GCTAAAAACAACCGGCCCTCA | AACTCCCGGGACAGCAGTGGCAGCCATGTGGTA |
| | 21-225_171D12 | | SEQ ID NO:3405 | SEQ ID NO:11417 | SEQ ID NO:19429 |
| | | AA | QGDSLRSYYAS | AKNNRPS | NSRDSSGSHVV |
| | | | SEQ ID NO:3406 | SEQ ID NO:11418 | SEQ ID NO:19430 |
| iPS:436802 | | NA | TCTGGAGATAAATTGGGGGATAAATATGCTTGC | CAAGATCGCAAGCGGCCCTCA | CAGGCGTGGGACATCAGCACTTATGTGGTA |
| | 21-225_171E12 | | SEQ ID NO:3407 | SEQ ID NO:11419 | SEQ ID NO:19431 |
| | | AA | SGDKLGDKYAC | QDRKRPS | QAWDISTYVV |
| | | | SEQ ID NO:3408 | SEQ ID NO:11420 | SEQ ID NO:19432 |
| iPS:436804 | | NA | CAAGGAGACAGCCTCAGAAACTATTATGTAAGC | ACTAAAAACAGCCGGCCCTCA | AACTCCCGGGACAGCAGTGGCAACCATGTGGTA |
| | 21-225_172C3 | | SEQ ID NO:3409 | SEQ ID NO:11421 | SEQ ID NO:19433 |
| | | AA | QGDSLRNYYVS | TKNSRPS | NSRDSSGNHVV |
| | | | SEQ ID NO:3410 | SEQ ID NO:11422 | SEQ ID NO:19434 |
| iPS:436806 | | NA | CAAGGAGACAGCCTCAGAAACTATTATGCAAGC | ACTAAAAACAGCCGGCCCTCA | AACTCCCGGGACAGCAGTGGCAACCATGTGGTA |
| | 21-225_172B12 | | SEQ ID NO:3411 | SEQ ID NO:11423 | SEQ ID NO:19435 |
| | | AA | QGDSLRNYYAS | TKNSRPS | NSRDSSGNHVV |

FIGURE 49

| | | | SEQ ID NO:3412 | SEQ ID NO:11424 | SEQ ID NO:19436 |
|---|---|---|---|---|---|
| iPS:436808 | | NA | TCTGGAAATAAATTGGGGAATAAATATGTTTGC | CAAGATAGCAGGCGGCCCTCA | CAGGCGTGGGACAGCTTCACTGTGGTA |
| | 21-225_173F8 | | SEQ ID NO:3413 | SEQ ID NO:11425 | SEQ ID NO:19437 |
| | | AA | SGNKLGNKYVC | QDSRRPS | QAWDSFTVV |
| | | | SEQ ID NO:3414 | SEQ ID NO:11426 | SEQ ID NO:19438 |
| iPS:436810 | | NA | ACTGGAACCAGCAGTGATGTTGGACGTTTTAACCTTGTCTCC | GAGGTCAGTAAGCGGCCCTCA | TGCTCATATGCAGGTAGTAGCACCTATGTGGTA |
| | 21-225_175F4 | | SEQ ID NO:3415 | SEQ ID NO:11427 | SEQ ID NO:19439 |
| | | AA | TGTSSDVGRFNLVS | EVSKRPS | CSYAGSSTYVV |
| | | | SEQ ID NO:3416 | SEQ ID NO:11428 | SEQ ID NO:19440 |
| iPS:436812 | | NA | TCTGGAGATAAATTGGGGGATAAATATGCTTGT | CAAGATTACAAGCGGCCCTCA | CAGGCGTGGGACAACAGCACTATGGTA |
| | 21-225_175C6 | | SEQ ID NO:3417 | SEQ ID NO:11429 | SEQ ID NO:19441 |
| | | AA | SGDKLGDKYAC | QDYKRPS | QAWDNSTMV |
| | | | SEQ ID NO:3418 | SEQ ID NO:11430 | SEQ ID NO:19442 |
| iPS:436814 | | NA | ACTGGAACCAGCAGTGATGTTGGACGTTTTAACCTTGTCTCC | GAAGTCAGTAAGCGGCCCTCA | TGCTCATATGCAGGTAGTAGCACCTTTGTAGTA |
| | 21-225_178H10 | | SEQ ID NO:3419 | SEQ ID NO:11431 | SEQ ID NO:19443 |
| | | AA | TGTSSDVGRFNLVS | EVSKRPS | CSYAGSSTFVV |
| | | | SEQ ID NO:3420 | SEQ ID NO:11432 | SEQ ID NO:19444 |
| iPS:436816 | | NA | CAAGGAGACAGTCTCAGAAACTATTATGCAAGC | GGTAAAAACAACCGGCCCTCA | AACTCCCGGGACAGCAGTGGTAACCATTGGGTG |
| | 21-225_179H5 | | SEQ ID NO:3421 | SEQ ID NO:11433 | SEQ ID NO:19445 |
| | | AA | QGDSLRNYYAS | GKNNRPS | NSRDSSGNHWV |
| | | | SEQ ID NO:3422 | SEQ ID NO:11434 | SEQ ID NO:19446 |
| iPS:436818 | | NA | TCTGGAGATAAATTGGGGGATAAATATGTTTGC | CAGGATAGTAAGCGGCCCTCA | CAGGCGTGGGACAGCAACACTGCAGTGGTA |

FIGURE 49

| | 21-225_179C7 | | SEQ ID NO:3423 | SEQ ID NO:11435 | SEQ ID NO:19447 |
|---|---|---|---|---|---|
| | | AA | SGDKLGDKYVC | QDSKRPS | QAWDSNTAVV |
| | | | SEQ ID NO:3424 | SEQ ID NO:11436 | SEQ ID NO:19448 |
| iPS:436820 | 21-225_179D10 | NA | ACTGGGAGCAGCTCCAACTTCGGGACAGATTATGATGTACAC | GGTCACAGCAACCGGCCCTCA | CAGTCCTATGATAGAAGCCTGAATGTGGTC |
| | | | SEQ ID NO:3425 | SEQ ID NO:11437 | SEQ ID NO:19449 |
| | | AA | TGSSSNFGTDYDVH | GHSNRPS | QSYDRSLNVV |
| | | | SEQ ID NO:3426 | SEQ ID NO:11438 | SEQ ID NO:19450 |
| iPS:436822 | 21-225_180D4 | NA | TCTGGAGATAGATTGGGGGATAAATATGCTTGC | GAAGATAGGAAGCGGCCCTCA | CAGGCGTGGGACAGTAGGAAAGTGGTA |
| | | | SEQ ID NO:3427 | SEQ ID NO:11439 | SEQ ID NO:19451 |
| | | AA | SGDRLGDKYAC | EDRKRPS | QAWDSRKVV |
| | | | SEQ ID NO:3428 | SEQ ID NO:11440 | SEQ ID NO:19452 |
| iPS:436824 | 21-225_180C5 | NA | TCTGGAGATAAATTGGGGGAAAAATATGCTTGC | CAAGATAGAAAGCGGCCCTCA | CAGGCGTGGGACAGCAGCACTGCGGTA |
| | | | SEQ ID NO:3429 | SEQ ID NO:11441 | SEQ ID NO:19453 |
| | | AA | SGDKLGEKYAC | QDRKRPS | QAWDSSTAV |
| | | | SEQ ID NO:3430 | SEQ ID NO:11442 | SEQ ID NO:19454 |
| iPS:436826 | 21-225_180G5 | NA | TCTGGAGATAAATTGGGGGATAAATATGTTAGC | CAAGATAGCAAGCGGCCCTCA | CAGGCGTGGGACATCACCACTGCGGTA |
| | | | SEQ ID NO:3431 | SEQ ID NO:11443 | SEQ ID NO:19455 |
| | | AA | SGDKLGDKYVS | QDSKRPS | QAWDITTAV |
| | | | SEQ ID NO:3432 | SEQ ID NO:11444 | SEQ ID NO:19456 |
| iPS:436828 | 21-225_181H1 | NA | TCTGGAGATAAATTGGGGGATAAATATGCTTGC | GAAGATAGGAAGCGGCCCTCA | CAGGCGTGGGACAGCAGGAAAGTGGTA |
| | | | SEQ ID NO:3433 | SEQ ID NO:11445 | SEQ ID NO:19457 |
| | | AA | SGDKLGDKYAC | EDRKRPS | QAWDSRKVV |
| | | | SEQ ID NO:3434 | SEQ ID NO:11446 | SEQ ID NO:19458 |

FIGURE 49

| iPS:436830 | | NA | TCTGGAAGCAGCTCCAACAT CGGAAGTAATATTGTGACC | AGTAATGATCAGCGGCC CTCA | ACAGCATGGGATGACAGCCT GAATGGTTGGGTG |
|---|---|---|---|---|---|
| | 21-225_51F4 | | SEQ ID NO:3435 | SEQ ID NO:11447 | SEQ ID NO:19459 |
| | | AA | SGSSSNIGSNIVT | SNDQRPS | TAWDDSLNGWV |
| | | | SEQ ID NO:3436 | SEQ ID NO:11448 | SEQ ID NO:19460 |
| iPS:436832 | | NA | ACTGGGAGCAGCTCCAACAT CGGGGCAGGTTTTGAAGTAC AC | GGTAACAGCAATCGGCC CTCA | CAGTCCTATGACAGCAGCCT GAGTGGTTATGTC |
| | 21-225_51D8 | | SEQ ID NO:3437 | SEQ ID NO:11449 | SEQ ID NO:19461 |
| | | AA | TGSSSNIGAGFEVH | GNSNRPS | QSYDSSLSGYV |
| | | | SEQ ID NO:3438 | SEQ ID NO:11450 | SEQ ID NO:19462 |
| iPS:436834 | | NA | TCTGGAAGCAGCTCCAACAT CGGAAGTAATATTGTGACC | AGTAATGATCAGCGGCC CTCA | GCAGCATGGGATGACAGCCT GAATGGTTGGGTG |
| | 21-225_52F1 | | SEQ ID NO:3439 | SEQ ID NO:11451 | SEQ ID NO:19463 |
| | | AA | SGSSSNIGSNIVT | SNDQRPS | AAWDDSLNGWV |
| | | | SEQ ID NO:3440 | SEQ ID NO:11452 | SEQ ID NO:19464 |
| iPS:436836 | | NA | TCTGGAGATAAATTGGGGGA TAAATATGTTTCC | CAAGATAGAAAGCGGCC CTCA | CAGGCGTGGGACAACAGCAC TGTGGTA |
| | 21-225_52H1 | | SEQ ID NO:3441 | SEQ ID NO:11453 | SEQ ID NO:19465 |
| | | AA | SGDKLGDKYVS | QDRKRPS | QAWDNSTVV |
| | | | SEQ ID NO:3442 | SEQ ID NO:11454 | SEQ ID NO:19466 |
| iPS:436838 | | NA | ACTGGAACCAGCAGTGACGT TGGTGGTTATAACTATGTCT CC | GAGGTCAGTAATCGGCC CTCA | AACTCATATACAAGCAACAT CACTTGGGTG |
| | 21-225_52H4 | | SEQ ID NO:3443 | SEQ ID NO:11455 | SEQ ID NO:19467 |
| | | AA | TGTSSDVGGYNYVS | EVSNRPS | NSYTSNITWV |
| | | | SEQ ID NO:3444 | SEQ ID NO:11456 | SEQ ID NO:19468 |

874

FIGURE 49

| iPS:436840 | | NA | TCTGGAACTAAATTGGGGGA TAAATATGTTTGC | CAAGATACAATGCGGCC CTCA | CAGACGTGGGACAGCAGCAC TGCGGTT |
|---|---|---|---|---|---|
| | 21-225_53E9 | | SEQ ID NO:3445 | SEQ ID NO:11457 | SEQ ID NO:19469 |
| | | AA | SGTKLGDKYVC | QDTMRPS | QTWDSSTAV |
| | | | SEQ ID NO:3446 | SEQ ID NO:11458 | SEQ ID NO:19470 |
| iPS:436842 | | NA | TCTGGAAGCAACTCCAACAT CGGAAATAATATTGTCACC | GTTAATGATCAGCGGCC CTCA | GCAGCATGGGATGACAGCCT GAATGGTTGGGTG |
| | 21-225_54E9 | | SEQ ID NO:3447 | SEQ ID NO:11459 | SEQ ID NO:19471 |
| | | AA | SGSNSNIGNNIVT | VNDQRPS | AAWDDSLNGWV |
| | | | SEQ ID NO:3448 | SEQ ID NO:11460 | SEQ ID NO:19472 |
| iPS:436844 | | NA | TCTGGAAGCAGCTCCAACAT CGGAAGTCATATTGTTACC | AGTAATGATCAGCGGCC CTCA | GCAGTATGGGATGACAGCCT GATTGGTTGGGTG |
| | 21-225_56G1 | | SEQ ID NO:3449 | SEQ ID NO:11461 | SEQ ID NO:19473 |
| | | AA | SGSSSNIGSHIVT | SNDQRPS | AVWDDSLIGWV |
| | | | SEQ ID NO:3450 | SEQ ID NO:11462 | SEQ ID NO:19474 |
| iPS:436846 | | NA | TCTGGAAGCAGCTCCAACAT CGGAAGTAATATTGTTACC | AGTAATAATCAGCGGCC CTCA | GCAGCATGGGATGACAGCCT GAATGGTTGGGTG |
| | 21-225_56E3 | | SEQ ID NO:3451 | SEQ ID NO:11463 | SEQ ID NO:19475 |
| | | AA | SGSSSNIGSNIVT | SNNQRPS | AAWDDSLNGWV |
| | | | SEQ ID NO:3452 | SEQ ID NO:11464 | SEQ ID NO:19476 |
| iPS:436848 | | NA | TCTGGAGATAAACTGGGGGA AAAATATGCTTGC | CAAGATAGGAAGCGGCC CTCA | CAGGCGTGGGACAGCAGCAC TGTGGTA |
| | 21-225_57F1 | | SEQ ID NO:3453 | SEQ ID NO:11465 | SEQ ID NO:19477 |
| | | AA | SGDKLGEKYAC | QDRKRPS | QAWDSSTVV |
| | | | SEQ ID NO:3454 | SEQ ID NO:11466 | SEQ ID NO:19478 |
| iPS:436850 | | NA | TCTGGAGAGAAATTGGGGG AAAAAATTTGCTTGC | CAAGATAGCAAGCGGCC CTCA | CAGGCGTGGGACAGCAGCAC TGTGGTA |

FIGURE 49

| | 21-225_57D9 | | SEQ ID NO:3455 | SEQ ID NO:11467 | SEQ ID NO:19479 |
|---|---|---|---|---|---|
| | | AA | SGEKLGEKFAC | QDSKRPS | QAWDSSTVV |
| | | | SEQ ID NO:3456 | SEQ ID NO:11468 | SEQ ID NO:19480 |
| iPS:436852 | | NA | TCTGGAGATAAACTGGGGGAAAAATATGCTTGC | CAAGATAGGAAGCGGCCCTCA | CAGGCGTGGGACAGCAGCACTGTGGTA |
| | 21-225_57H11 | | SEQ ID NO:3457 | SEQ ID NO:11469 | SEQ ID NO:19481 |
| | | AA | SGDKLGEKYAC | QDRKRPS | QAWDSSTVV |
| | | | SEQ ID NO:3458 | SEQ ID NO:11470 | SEQ ID NO:19482 |
| iPS:436854 | | NA | TCTGGAGATAAATTGGGGAATAAATATGCTTGC | CAAGATAGGAAGCGGCCCTCA | CAGGCGTGGGACAGCAGCACTGCA |
| | 21-225_58C1 | | SEQ ID NO:3459 | SEQ ID NO:11471 | SEQ ID NO:19483 |
| | | AA | SGDKLGNKYAC | QDRKRPS | QAWDSSTA |
| | | | SEQ ID NO:3460 | SEQ ID NO:11472 | SEQ ID NO:19484 |
| iPS:436856 | | NA | TCTGGAAGCAGCTCCAACATTGGGAATAATTATGTATCC | GACAATAATAAGCGACCCTCA | GGAACATGGGATATCAGTCTGAGTGTTGGGGTA |
| | 21-225_58C5 | | SEQ ID NO:3461 | SEQ ID NO:11473 | SEQ ID NO:19485 |
| | | AA | SGSSSNIGNNYVS | DNNKRPS | GTWDISLSVGV |
| | | | SEQ ID NO:3462 | SEQ ID NO:11474 | SEQ ID NO:19486 |
| iPS:436858 | | NA | TCTGGAGATAAATTGGGGGATAAATATACTTGC | CAAGATAACAAGCGGCCCTCA | CAGGCGTGGAACAACTACACTGTGGTA |
| | 21-225_58E7 | | SEQ ID NO:3463 | SEQ ID NO:11475 | SEQ ID NO:19487 |
| | | AA | SGDKLGDKYTC | QDNKRPS | QAWNNYTVV |
| | | | SEQ ID NO:3464 | SEQ ID NO:11476 | SEQ ID NO:19488 |
| iPS:436860 | | NA | TCTGGAGATAAATTGGGGGATAAATATGCTTGC | CAAGATAGGAAGCGGCCCTCA | CAGGCGTGGGACAGCAGCACTGTGGTA |
| | 21-225_58F7 | | SEQ ID NO:3465 | SEQ ID NO:11477 | SEQ ID NO:19489 |
| | | AA | SGDKLGDKYAC | QDRKRPS | QAWDSSTVV |
| | | | SEQ ID NO:3466 | SEQ ID NO:11478 | SEQ ID NO:19490 |

FIGURE 49

| iPS:436862 | | NA | TCTGGAGATAAATTGGGAAA TAAATATGCTTGC | CAAGATAGCAAGCGGCC CTCA | CAGGCGTGGGACAGCAGCAC TGTGGTA |
|---|---|---|---|---|---|
| | 21-225_58F8 | | SEQ ID NO:3467 | SEQ ID NO:11479 | SEQ ID NO:19491 |
| | | AA | SGDKLGNKYAC | QDSKRPS | QAWDSSTVV |
| | | | SEQ ID NO:3468 | SEQ ID NO:11480 | SEQ ID NO:19492 |
| iPS:436864 | | NA | TCTGGAGATAAATTGGGGGA TAAATATGCTTCC | CAAGATAACAAGCGGCC CTCA | CAGGCGTGGGAACAACAACAC TGTAATG |
| | 21-225_58G11 | | SEQ ID NO:3469 | SEQ ID NO:11481 | SEQ ID NO:19493 |
| | | AA | SGDKLGDKYAS | QDNKRPS | QAWNNNTVM |
| | | | SEQ ID NO:3470 | SEQ ID NO:11482 | SEQ ID NO:19494 |
| iPS:436866 | | NA | TCTGGAGATAAATTGGGGGA TAAATATGCTTCT | CAAGATAACAAGCGGCC CTCA | CAGGCGTGGGACAACAACAC TGTGGTC |
| | 21-225_59F2 | | SEQ ID NO:3471 | SEQ ID NO:11483 | SEQ ID NO:19495 |
| | | AA | SGDKLGDKYAS | QDNKRPS | QAWDNNTVV |
| | | | SEQ ID NO:3472 | SEQ ID NO:11484 | SEQ ID NO:19496 |
| iPS:436868 | | NA | TCTGGAGATAAATTGGGGGA TAAATATGCTTGC | CAAGATAGCAAGCGGCC CTCA | CAGGCGTGGGACAGCAGCAC TTATGTGGTA |
| | 21-225_59B11 | | SEQ ID NO:3473 | SEQ ID NO:11485 | SEQ ID NO:19497 |
| | | AA | SGDKLGDKYAC | QDSKRPS | QAWDSSTYVV |
| | | | SEQ ID NO:3474 | SEQ ID NO:11486 | SEQ ID NO:19498 |
| iPS:436870 | | NA | TCTGGAGATAAACTGGGGGA AAAATATGCTTGC | CAAGATAGGAAGCGGCC CTCA | CAGGCGTGGGACAGCAGCAC TGTGGTA |
| | 21-225_60B1 | | SEQ ID NO:3475 | SEQ ID NO:11487 | SEQ ID NO:19499 |
| | | AA | SGDKLGEKYAC | QDRKRPS | QAWDSSTVV |
| | | | SEQ ID NO:3476 | SEQ ID NO:11488 | SEQ ID NO:19500 |
| iPS:436872 | | NA | TCTGGAAATAAATTGGGGGA TAAATATGCTTCT | CAAGATAACAAGCGGCC CTCA | CAGGCGTGGGACAACAACAC TGTGGTC |
| | 21-225_60D2 | | SEQ ID NO:3477 | SEQ ID NO:11489 | SEQ ID NO:19501 |
| | | AA | SGNKLGDKYAS | QDNKRPS | QAWDNNTVV |
| | | | SEQ ID NO:3478 | SEQ ID NO:11490 | SEQ ID NO:19502 |

FIGURE 49

| iPS:436874 | | NA | TCTGGAGATAAATTGGGGAA TAAATATGCTTGC | CAAGATAGGAAGCGGCC CTCA | CAGGCGTGGGACAGCAGCAC TGCT |
|---|---|---|---|---|---|
| | 21-225_60A12 | | SEQ ID NO:3479 | SEQ ID NO:11491 | SEQ ID NO:19503 |
| | | AA | SGDKLGNKYAC | QDRKRPS | QAWDSSTA |
| | | | SEQ ID NO:3480 | SEQ ID NO:11492 | SEQ ID NO:19504 |
| iPS:436876 | | NA | TCTGGAGATAAATTGGGGGA AAAATATGCTTGC | CAAGATAGCAAGCGGCC CTCA | CAGGCGTGGGACAGCAGCAC TGTGGTT |
| | 21-225_61F5 | | SEQ ID NO:3481 | SEQ ID NO:11493 | SEQ ID NO:19505 |
| | | AA | SGDKLGEKYAC | QDSKRPS | QAWDSSTVV |
| | | | SEQ ID NO:3482 | SEQ ID NO:11494 | SEQ ID NO:19506 |
| iPS:436878 | | NA | TCTGGAGATAAATTGGGGAA TAAATATGCTTGC | CAAGATAGGAAGCGGCC CTCA | CAGGCGTGGGACAGCAGCAC TGCGGTA |
| | 21-225_62E3 | | SEQ ID NO:3483 | SEQ ID NO:11495 | SEQ ID NO:19507 |
| | | AA | SGDKLGNKYAC | QDRKRPS | QAWDSSTAV |
| | | | SEQ ID NO:3484 | SEQ ID NO:11496 | SEQ ID NO:19508 |
| iPS:436880 | | NA | TCTGGAGATAGATTGGGGAA TAAATATGCTTCC | CAGGATAGGAAGCGGCC CTCA | CAGGCGTGGGACAGCAGCAC TGCGGTA |
| | 21-225_62E8 | | SEQ ID NO:3485 | SEQ ID NO:11497 | SEQ ID NO:19509 |
| | | AA | SGDRLGNKYAS | QDRKRPS | QAWDSSTAV |
| | | | SEQ ID NO:3486 | SEQ ID NO:11498 | SEQ ID NO:19510 |
| iPS:436882 | | NA | TCTGGAGATAAATTGGGGAA TAAATATGCTTGC | CAAGATAGGAAGCGGCC CTCA | CAGGCGTGGGACAGCAGCAC TGCGGTA |
| | 21-225_62D10 | | SEQ ID NO:3487 | SEQ ID NO:11499 | SEQ ID NO:19511 |
| | | AA | SGDKLGNKYAC | QDRKRPS | QAWDSSTAV |
| | | | SEQ ID NO:3488 | SEQ ID NO:11500 | SEQ ID NO:19512 |
| iPS:436884 | | NA | TCTGGAGATAAATTGGGGAA TAAATATGCTTGC | CAAGATAGGAAGCGGCC CTCA | CAGGCGTGGGACAGCAGCAC TGCGGTA |
| | 21-225_62A12 | | SEQ ID NO:3489 | SEQ ID NO:11501 | SEQ ID NO:19513 |
| | | AA | SGDKLGNKYAC | QDRKRPS | QAWDSSTAV |
| | | | SEQ ID NO:3490 | SEQ ID NO:11502 | SEQ ID NO:19514 |

FIGURE 49

| iPS:436886 | | NA | TCTGGAGATAAATTGGGGAATAAATATACTTGC | CAAGATAGGAAGCGGCCCTCA | CAGGCGTGGGACAGCAGCACTGCGGTA |
|---|---|---|---|---|---|
| | 21-225_62B12 | | SEQ ID NO:3491 | SEQ ID NO:11503 | SEQ ID NO:19515 |
| | | AA | SGDKLGNKYTC | QDRKRPS | QAWDSSTAV |
| | | | SEQ ID NO:3492 | SEQ ID NO:11504 | SEQ ID NO:19516 |
| iPS:436888 | | NA | ACCCGCAGCAATGGCAGCATTGTCAGCAACTATGTGCAG | GAGGATAGCCGAAGACCCTCT | CAGTCTTATGATGGCATCAATGTGGTA |
| | 21-225_63G7 | | SEQ ID NO:3493 | SEQ ID NO:11505 | SEQ ID NO:19517 |
| | | AA | TRSNGSIVSNYVQ | EDSRRPS | QSYDGINVV |
| | | | SEQ ID NO:3494 | SEQ ID NO:11506 | SEQ ID NO:19518 |
| iPS:436890 | | NA | ACCCGCAGCAATGGCAGCATTGTCAGCAACTATGTGCAG | GAGGATAAAAGAAGACCCTCA | CAGTCTTATGATAGCATCAATGTGGTA |
| | 21-225_63A10 | | SEQ ID NO:3495 | SEQ ID NO:11507 | SEQ ID NO:19519 |
| | | AA | TRSNGSIVSNYVQ | EDKRRPS | QSYDSINVV |
| | | | SEQ ID NO:3496 | SEQ ID NO:11508 | SEQ ID NO:19520 |
| iPS:436892 | | NA | TCTGGAGATAAATTGGGGAATAAATATGATTAC | CAAGATAGAAAGCGGCCCTCA | CAGGCGTGGGACAACAGCACTGTGGTA |
| | 21-225_65E9 | | SEQ ID NO:3497 | SEQ ID NO:11509 | SEQ ID NO:19521 |
| | | AA | SGDKLGNKYDY | QDRKRPS | QAWDNSTVV |
| | | | SEQ ID NO:3498 | SEQ ID NO:11510 | SEQ ID NO:19522 |
| iPS:436894 | | NA | TCTGGAGATAAATTGGGGAATAAATATGCTTGC | CAAGATAGGAAGCGGCCCTCA | CAGGCGTGGGACATCAACACTGCGGTA |
| | 21-225_66G9 | | SEQ ID NO:3499 | SEQ ID NO:11511 | SEQ ID NO:19523 |
| | | AA | SGDKLGNKYAC | QDRKRPS | QAWDINTAV |
| | | | SEQ ID NO:3500 | SEQ ID NO:11512 | SEQ ID NO:19524 |
| iPS:436896 | | NA | TCTGGAGATAAATTGGGGTATAAATATGCTTGG | GAAGATAGGAAGCGGCCCTCA | CAGGCGTGGGACAACAGCACTGTGGTA |
| | 21-225_67F10 | | SEQ ID NO:3501 | SEQ ID NO:11513 | SEQ ID NO:19525 |

FIGURE 49

| | | AA | SGDKLGYKYAW | EDRKRPS | QAWDNSTVV |
|---|---|---|---|---|---|
| | | | SEQ ID NO:3502 | SEQ ID NO:11514 | SEQ ID NO:19526 |
| iPS:436898 | | NA | TCTGGAGATAAATTGGGGGATAAATATGCTTGC | CAAGATAGCAAGCGGCCCTCA | CAGGCGTGGGACAACAGCACTGTGGTA |
| | 21-225_68D8 | | SEQ ID NO:3503 | SEQ ID NO:11515 | SEQ ID NO:19527 |
| | | AA | SGDKLGDKYAC | QDSKRPS | QAWDNSTVV |
| | | | SEQ ID NO:3504 | SEQ ID NO:11516 | SEQ ID NO:19528 |
| iPS:436900 | | NA | TCTGGAGATAAATTGGGGAATAAATATGATTAC | CAAGATAGAAAGCGGCCCTCA | CAGGCGTGGGACAACAGCACTGTGGTA |
| | 21-225_69B9 | | SEQ ID NO:3505 | SEQ ID NO:11517 | SEQ ID NO:19529 |
| | | AA | SGDKLGNKYDY | QDRKRPS | QAWDNSTVV |
| | | | SEQ ID NO:3506 | SEQ ID NO:11518 | SEQ ID NO:19530 |
| iPS:436902 | | NA | TCTGGAGATAAATTGGGGGATAAATATGCTTGG | GAAGATAGGAAGCGGCCCTCA | CAGGCGTGGGACAACAGCACTGTGGTA |
| | 21-225_69B11 | | SEQ ID NO:3507 | SEQ ID NO:11519 | SEQ ID NO:19531 |
| | | AA | SGDKLGDKYAW | EDRKRPS | QAWDNSTVV |
| | | | SEQ ID NO:3508 | SEQ ID NO:11520 | SEQ ID NO:19532 |
| iPS:436904 | | NA | TCTGGAGATAAATTGGGGGATAAATATGCTTAC | CAAGATAGGAAGCGGCCCTCA | CAGGCGTGGGTCAACAGCACTGTGGTA |
| | 21-225_71D4 | | SEQ ID NO:3509 | SEQ ID NO:11521 | SEQ ID NO:19533 |
| | | AA | SGDKLGDKYAY | QDRKRPS | QAWVNSTVV |
| | | | SEQ ID NO:3510 | SEQ ID NO:11522 | SEQ ID NO:19534 |
| iPS:436906 | | NA | TCTGGAGATAAATTGGGGGATAAATATGCTTGG | GAAGATAGGAAGCGGCCCTCA | CAGGCGTGGGACAACAGCACTGTGGTA |
| | 21-225_72B4 | | SEQ ID NO:3511 | SEQ ID NO:11523 | SEQ ID NO:19535 |
| | | AA | SGDKLGDKYAW | EDRKRPS | QAWDNSTVV |
| | | | SEQ ID NO:3512 | SEQ ID NO:11524 | SEQ ID NO:19536 |
| iPS:436908 | | NA | TCTGGAGATAAATTGGGGAATAAATATGCTTGC | CAAGATAGGAAGCGGCCCTCA | CAGGCGTGGGACAGCAGCACTGCGGTA |
| | 21-225_72D5 | | SEQ ID NO:3513 | SEQ ID NO:11525 | SEQ ID NO:19537 |

FIGURE 49

| | | AA | SGDKLGNKYAC | QDRKRPS | QAWDSSTAV |
|---|---|---|---|---|---|
| | | | SEQ ID NO:3514 | SEQ ID NO:11526 | SEQ ID NO:19538 |
| iPS:436910 | 21-225_73G1 | NA | GGCTTGAGCTCTGGCTCAGT CTCTACTAGTTACTACCCCA GC | AACACAAACACTCGCTC TTCT | GTTCTATATATGGGTAGTGC CATTTGGGTG |
| | | | SEQ ID NO:3515 | SEQ ID NO:11527 | SEQ ID NO:19539 |
| | | AA | GLSSGSVSTSYYPS | NTNTRSS | VLYMGSAIWV |
| | | | SEQ ID NO:3516 | SEQ ID NO:11528 | SEQ ID NO:19540 |
| iPS:436912 | 21-225_73C4 | NA | TCTGGAGATAAATTGGGGAA TAAATATGCTTGC | CAAGATATGAAGCGGCC CTCA | CAGGCGTGGGACAGCAGCAC TGCGGTA |
| | | | SEQ ID NO:3517 | SEQ ID NO:11529 | SEQ ID NO:19541 |
| | | AA | SGDKLGNKYAC | QDMKRPS | QAWDSSTAV |
| | | | SEQ ID NO:3518 | SEQ ID NO:11530 | SEQ ID NO:19542 |
| iPS:436914 | 21-225_76B4 | NA | TCTGGAGATAGATTGGGGAC TAAATTTGCTTGC | CAAGATAGCAAGCGGCC CTCA | CAGGCGTGGGACAGCAGCAC TGTA |
| | | | SEQ ID NO:3519 | SEQ ID NO:11531 | SEQ ID NO:19543 |
| | | AA | SGDRLGTKFAC | QDSKRPS | QAWDSSTV |
| | | | SEQ ID NO:3520 | SEQ ID NO:11532 | SEQ ID NO:19544 |
| iPS:436916 | 21-225_74A9 | NA | TCTGGAGATAAATTGGGTAA TAAATATGTTTGT | CAAGATAACAGGCGGCC CTCA | CAGGCGTGGGACAGCAGTCC TGTGATA |
| | | | SEQ ID NO:3521 | SEQ ID NO:11533 | SEQ ID NO:19545 |
| | | AA | SGDKLGNKYVC | QDNRRPS | QAWDSSPVI |
| | | | SEQ ID NO:3522 | SEQ ID NO:11534 | SEQ ID NO:19546 |
| iPS:436918 | 21-225_77A2 | NA | TCTGGAGATAGATTGGGGGA TAAATATGCTTGC | CAAGATAGGAAGCGGCC CTCA | CAGGCGTGGGACAGCAGTAC TGCGGTA |
| | | | SEQ ID NO:3523 | SEQ ID NO:11535 | SEQ ID NO:19547 |
| | | AA | SGDRLGDKYAC | QDRKRPS | QAWDSSTAV |
| | | | SEQ ID NO:3524 | SEQ ID NO:11536 | SEQ ID NO:19548 |

FIGURE 49

| iPS:436920 | | NA | GCTTCCAGCACTGAAACAGT CACCAGTGGTTCTTATCCGA AC | AGTACAAGCAACAAACA CTCC | CTGCTCTACTATGGTGGTGC TCAACTGGTA |
|---|---|---|---|---|---|
| | 21-225_74E5 | | SEQ ID NO:3525 | SEQ ID NO:11537 | SEQ ID NO:19549 |
| | | AA | ASSTETVTSGSYPN | STSNKHS | LLYYGGAQLV |
| | | | SEQ ID NO:3526 | SEQ ID NO:11538 | SEQ ID NO:19550 |
| iPS:436922 | | NA | TCAGGAGATAAATTGGGGA ATAAATATGTTTCC | CAAGATAACAGGCGGCC GTCA | CAGGCGTGGGACAGCAGCCC TGTGATA |
| | 21-225_78E9 | | SEQ ID NO:3527 | SEQ ID NO:11539 | SEQ ID NO:19551 |
| | | AA | SGDKLGNKYVS | QDNRRPS | QAWDSSPVI |
| | | | SEQ ID NO:3528 | SEQ ID NO:11540 | SEQ ID NO:19552 |
| iPS:436924 | | NA | TCTGGAGATAAATTGGGGGA TAAATATGCTTGC | CAAGATAGCAAGCGGCC CTCA | CAGGCGTGGGACAGCACCAC TGTGGTA |
| | 21-225_74B3 | | SEQ ID NO:3529 | SEQ ID NO:11541 | SEQ ID NO:19553 |
| | | AA | SGDKLGDKYAC | QDSKRPS | QAWDSTTVV |
| | | | SEQ ID NO:3530 | SEQ ID NO:11542 | SEQ ID NO:19554 |
| iPS:436926 | | NA | GCTTCCAGCACTGGAGCAGT CACCAGTGGTTACTTTCCAA AC | AGTACAGACAACAAACA CTCC | CTCCTCTACTATGGTGGTGCT CAGCTGATG |
| | 21-225_78D10 | | SEQ ID NO:3531 | SEQ ID NO:11543 | SEQ ID NO:19555 |
| | | AA | ASSTGAVTSGYFPN | STDNKHS | LLYYGGAQLM |
| | | | SEQ ID NO:3532 | SEQ ID NO:11544 | SEQ ID NO:19556 |
| iPS:436928 | | NA | TCAGGAGATAAATTGGGGA ATAAATATGTTTCC | CAAGATAACAGGCGGCC CTCA | CAGGCGTGGGACAGCAGCCC TGTGATA |
| | 21-225_79E7 | | SEQ ID NO:3533 | SEQ ID NO:11545 | SEQ ID NO:19557 |
| | | AA | SGDKLGNKYVS | QDNRRPS | QAWDSSPVI |
| | | | SEQ ID NO:3534 | SEQ ID NO:11546 | SEQ ID NO:19558 |
| iPS:436932 | | NA | TCTGGAGATAAATTGGGGAA TAAATATGTTTGC | CAAGATAACAGGCGGCC CTCA | CAGGCGTGGGACAGCAGCCC TGTGATA |
| | 21-225_92A4 | | SEQ ID NO:3535 | SEQ ID NO:11547 | SEQ ID NO:19559 |

FIGURE 49

| | | | | | |
|---|---|---|---|---|---|
| | | AA | SGDKLGNKYVC | QDNRRPS | QAWDSSPVI |
| | | | SEQ ID NO:3536 | SEQ ID NO:11548 | SEQ ID NO:19560 |
| iPS:436934 | | NA | TCTGGAGATAGATTGGGGAC TAAATTTGCTTGC | CAAGATAGCAAGCGGCC CTCA | CAGGCGTGGGACAGCAGCAC TGTA |
| | 21-225_96B5 | | SEQ ID NO:3537 | SEQ ID NO:11549 | SEQ ID NO:19561 |
| | | AA | SGDRLGTKFAC | QDSKRPS | QAWDSSTV |
| | | | SEQ ID NO:3538 | SEQ ID NO:11550 | SEQ ID NO:19562 |
| iPS:436936 | | NA | TCTGGAGATAAATTGGGGAA TAAATATGTTTCC | CAAGATAACAGGCGGCC GTCA | CAGGCGTGGGACAGCACCCC TGTGATA |
| | 21-225_97E6 | | SEQ ID NO:3539 | SEQ ID NO:11551 | SEQ ID NO:19563 |
| | | AA | SGDKLGNKYVS | QDNRRPS | QAWDSTPVI |
| | | | SEQ ID NO:3540 | SEQ ID NO:11552 | SEQ ID NO:19564 |
| iPS:436938 | | NA | TCTGGAAATAAATTGGGGAA TAGATATGCTTGC | CAAGATAGCAAGCGGCC CTCA | CAGGCGTGGGACAGCAGCAC TGTGGTA |
| | 21-225_146A3 | | SEQ ID NO:3541 | SEQ ID NO:11553 | SEQ ID NO:19565 |
| | | AA | SGNKLGNRYAC | QDSKRPS | QAWDSSTVV |
| | | | SEQ ID NO:3542 | SEQ ID NO:11554 | SEQ ID NO:19566 |
| iPS:436940 | | NA | TCTGGAGATAAATTGGGGGA TAAATATGTTTGC | CAAGATAGGAAGCGGCC CTCA | CAGGCGTGGCACAGCAGCAC TGTGGTA |
| | 21-225_146B8 | | SEQ ID NO:3543 | SEQ ID NO:11555 | SEQ ID NO:19567 |
| | | AA | SGDKLGDKYVC | QDRKRPS | QAWHSSTVV |
| | | | SEQ ID NO:3544 | SEQ ID NO:11556 | SEQ ID NO:19568 |
| iPS:436942 | | NA | TCTGGAGATAAATTGGGGGA TAAATATGCTTGC | CAAGATAAGAAGCGGCC CTCA | CAGGCGTGGGACATCAGAAC TGTGGTA |
| | 21-225_146H8 | | SEQ ID NO:3545 | SEQ ID NO:11557 | SEQ ID NO:19569 |
| | | AA | SGDKLGDKYAC | QDKKRPS | QAWDIRTVV |
| | | | SEQ ID NO:3546 | SEQ ID NO:11558 | SEQ ID NO:19570 |
| iPS:436944 | | NA | TCTGGAGATAAATTGGGGGA GAAATATGCTTGC | CAAGATAGAAAGCGGCC CTCA | CAGGCGTGGGACAGTAGAAC TGCGGTA |
| | 21-225_182D12 | | SEQ ID NO:3547 | SEQ ID NO:11559 | SEQ ID NO:19571 |

FIGURE 49

| | | AA | SGDKLGEKYAC | QDRKRPS | QAWDSRTAV |
|---|---|---|---|---|---|
| | | | SEQ ID NO:3548 | SEQ ID NO:11560 | SEQ ID NO:19572 |
| iPS:436946 | | NA | TCTGGAGATAAATTGGGGGATAAATATGCTTGT | CAAGATAAGAAACGGCCCTCA | CAGGCGTGGGACAACAGCACTGCTGTGGTA |
| | 21-225_183F4 | | SEQ ID NO:3549 | SEQ ID NO:11561 | SEQ ID NO:19573 |
| | | AA | SGDKLGDKYAC | QDKKRPS | QAWDNSTAVV |
| | | | SEQ ID NO:3550 | SEQ ID NO:11562 | SEQ ID NO:19574 |
| iPS:436948 | | NA | GGCTTGAGCTCTGGCTCAGTCTCTACTACTTTCTACCCCAGC | AACACAAACACTCGCTCTTCT | GTGCTTTATATGGGTAGTGGCATTTGGGTG |
| | 21-225_183F5 | | SEQ ID NO:3551 | SEQ ID NO:11563 | SEQ ID NO:19575 |
| | | AA | GLSSGSVSTFYPS | NTNTRSS | VLYMGSGIWV |
| | | | SEQ ID NO:3552 | SEQ ID NO:11564 | SEQ ID NO:19576 |
| iPS:436950 | | NA | TCTGGAGATAAATTGGGGGATAAATTTGCTTGC | GAAGATAGGAAGCGGCCCTCA | CAGGCGTGGGACAGCCGCACTGTGGTA |
| | 21-225_184G4 | | SEQ ID NO:3553 | SEQ ID NO:11565 | SEQ ID NO:19577 |
| | | AA | SGDKLGDKFAC | EDRKRPS | QAWDSRTVV |
| | | | SEQ ID NO:3554 | SEQ ID NO:11566 | SEQ ID NO:19578 |
| iPS:436952 | | NA | TCTGGAGATAAATTGGGGGATAAATATGCTTGC | GAAGATAGGAAGCGGCCCTCA | CAGGCGTGGGACAGCAGGAAAGTGGTA |
| | 21-225_185D2 | | SEQ ID NO:3555 | SEQ ID NO:11567 | SEQ ID NO:19579 |
| | | AA | SGDKLGDKYAC | EDRKRPS | QAWDSRKVV |
| | | | SEQ ID NO:3556 | SEQ ID NO:11568 | SEQ ID NO:19580 |
| iPS:436954 | | NA | TCTGGAGATAAATTGGGGCATAAATTTGTTTGC | CAAGATCGCAAGCGGCCCTCA | CAGGCGTGGGACAGCAGCACGGTA |
| | 21-225_185G7 | | SEQ ID NO:3557 | SEQ ID NO:11569 | SEQ ID NO:19581 |
| | | AA | SGDKLGHKFVC | QDRKRPS | QAWDSSTV |
| | | | SEQ ID NO:3558 | SEQ ID NO:11570 | SEQ ID NO:19582 |
| iPS:436956 | | NA | TCTGGAGATAAAATGGGGGAAAAATATGCTTGC | CAAGATAGAAAGCGGCCCTCA | CAGGCGTGGGACAGCAGCACTGCGGTA |

FIGURE 49

| | 21-225_186H6 | | SEQ ID NO:3559 | SEQ ID NO:11571 | SEQ ID NO:19583 |
|---|---|---|---|---|---|
| | | AA | SGDKMGEKYAC | QDRKRPS | QAWDSSTAV |
| | | | SEQ ID NO:3560 | SEQ ID NO:11572 | SEQ ID NO:19584 |
| iPS:436958 | 21-225_190D1 | NA | GCTTCCAGCACTGGAGCAGTCACCAGTGGTTCCTATCCAAAC | AGTACAAGTAACAAACACTCC | CTGCTCTACTATGGTGGTGCTCAGGTGGCA |
| | | | SEQ ID NO:3561 | SEQ ID NO:11573 | SEQ ID NO:19585 |
| | | AA | ASSTGAVTSGSYPN | STSNKHS | LLYYGGAQVA |
| | | | SEQ ID NO:3562 | SEQ ID NO:11574 | SEQ ID NO:19586 |
| iPS:436960 | 21-225_198D2 | NA | TCTGGAAGCAGCTCCAACATTGGGAGTAATTATGTTTCC | GACAATAATAAGCGACCCTCA | GGAACATGGGATAGCAGACTGAATGTTGGGGTA |
| | | | SEQ ID NO:3563 | SEQ ID NO:11575 | SEQ ID NO:19587 |
| | | AA | SGSSSNIGSNYVS | DNNKRPS | GTWDSRLNVGV |
| | | | SEQ ID NO:3564 | SEQ ID NO:11576 | SEQ ID NO:19588 |
| iPS:436962 | 21-225_190H1 | NA | TCTGGAGATAAATTGGGGGATAGATTTGCTTAC | CAAGATAGCAAGCGGCCCTCA | AAGGCGTGGGACAGCAGCACTGTGGTA |
| | | | SEQ ID NO:3565 | SEQ ID NO:11577 | SEQ ID NO:19589 |
| | | AA | SGDKLGDRFAY | QDSKRPS | KAWDSSTVV |
| | | | SEQ ID NO:3566 | SEQ ID NO:11578 | SEQ ID NO:19590 |
| iPS:436964 | 21-225_190B3 | NA | CAAGGAGACAAACTCAGAACCTATTATGCAAGC | GGAAAAAACAACCGGCCCTCA | AACTCCCGGGACAGCAGTGGTAACCATCTTGTACTA |
| | | | SEQ ID NO:3567 | SEQ ID NO:11579 | SEQ ID NO:19591 |
| | | AA | QGDKLRTYYAS | GKNNRPS | NSRDSSGNHLVL |
| | | | SEQ ID NO:3568 | SEQ ID NO:11580 | SEQ ID NO:19592 |
| iPS:436966 | 21-225_190C3 | NA | TCTGGAAGCAGCTCCAACATTGGAAATAATTATGTATCC | GACAGTAATAAGCGACCCTCA | GGAACATGGGATAGCAGCCTGAGTACTGTGGTA |
| | | | SEQ ID NO:3569 | SEQ ID NO:11581 | SEQ ID NO:19593 |
| | | AA | SGSSSNIGNNYVS | DSNKRPS | GTWDSSLSTVV |
| | | | SEQ ID NO:3570 | SEQ ID NO:11582 | SEQ ID NO:19594 |

FIGURE 49

| iPS:436968 | | NA | TCTGGAAGCAGCTCCAACAT TGGGAATAATTATGTATCC | GACAATAATAAGCGACC CTCA | GGAACATGGGATAGCAGCCT GAGTGCTGGGGTT |
|---|---|---|---|---|---|
| | 21-225_190B10 | | SEQ ID NO:3571 | SEQ ID NO:11583 | SEQ ID NO:19595 |
| | | AA | SGSSSNIGNNYVS | DNNKRPS | GTWDSSLSAGV |
| | | | SEQ ID NO:3572 | SEQ ID NO:11584 | SEQ ID NO:19596 |
| iPS:436970 | | NA | CAAGGAGACACCCTCAGACC CTATTATGTAAGC | GGTAAAAACAACCGGCC CTCA | AACTCCCGGGACAGCAGTGG TAACCATCTTGTGGTA |
| | 21-225_190B8 | | SEQ ID NO:3573 | SEQ ID NO:11585 | SEQ ID NO:19597 |
| | | AA | QGDTLRPYYVS | GKNNRPS | NSRDSSGNHLVV |
| | | | SEQ ID NO:3574 | SEQ ID NO:11586 | SEQ ID NO:19598 |
| iPS:436972 | | NA | TCTGGAGGCAGCTCCAACAT TGGGAATAATTATGTATCC | GACAATAATAAGCGACC CTCA | GGAACATGGGATCGCACCCT GAGTGATTGGGTA |
| | 21-225_190C7 | | SEQ ID NO:3575 | SEQ ID NO:11587 | SEQ ID NO:19599 |
| | | AA | SGGSSNIGNNYVS | DNNKRPS | GTWDRTLSDWV |
| | | | SEQ ID NO:3576 | SEQ ID NO:11588 | SEQ ID NO:19600 |
| iPS:436974 | | NA | TCTGGAAGCAGCTCCAACAT TGGGAGTAATTATGTTTCC | GACAATAATAAGCGACC CTCA | GGAACATGGGATGGCAGACT GAATGTTGGGGTA |
| | 21-225_190H7 | | SEQ ID NO:3577 | SEQ ID NO:11589 | SEQ ID NO:19601 |
| | | AA | SGSSSNIGSNYVS | DNNKRPS | GTWDGRLNVGV |
| | | | SEQ ID NO:3578 | SEQ ID NO:11590 | SEQ ID NO:19602 |
| iPS:436976 | | NA | TCTGGAAGCAGCTCCAACAT TGGGAATCATTATGTCTCC | GACAGTAGTAAGCGACC CTCA | GGAACATGGGATAGTAGTCT GAGTACTGTGGTA |
| | 21-225_190D8 | | SEQ ID NO:3579 | SEQ ID NO:11591 | SEQ ID NO:19603 |
| | | AA | SGSSSNIGNHYVS | DSSKRPS | GTWDSSLSTVV |
| | | | SEQ ID NO:3580 | SEQ ID NO:11592 | SEQ ID NO:19604 |
| iPS:436978 | | NA | TCTGGAGATAAATTGGGGGA TAGATTTGCTTAC | CAAGATAACAAGCGGCC CTCA | CAGGCGTGGGACAGCAGCAC TGTGGTA |
| | 21-225_190G9 | | SEQ ID NO:3581 | SEQ ID NO:11593 | SEQ ID NO:19605 |

886

FIGURE 49

| | | | | | |
|---|---|---|---|---|---|
| | | AA | SGDKLGDRFAY | QDNKRPS | QAWDSSTVV |
| | | | SEQ ID NO:3582 | SEQ ID NO:11594 | SEQ ID NO:19606 |
| iPS:436980 | | NA | CAAGGAGACAGCCTCAGACCCTATTATGCAAGC | GGTAAAAACAACCGGCCCTCA | AACTCCCGGGACAGCAGTGGTAACCATCTTGTGGTA |
| | 21-225_190C10 | | SEQ ID NO:3583 | SEQ ID NO:11595 | SEQ ID NO:19607 |
| | | AA | QGDSLRPYYAS | GKNNRPS | NSRDSSGNHLVV |
| | | | SEQ ID NO:3584 | SEQ ID NO:11596 | SEQ ID NO:19608 |
| iPS:436982 | | NA | TCTGGAAGCAGCTCCAACATTGGGAGTAATTATGTTTCC | GACAATAATAAGCGACCCTCA | GGAACATGGGATAGCAGACTGAATGTTGGGGTA |
| | 21-225_190D10 | | SEQ ID NO:3585 | SEQ ID NO:11597 | SEQ ID NO:19609 |
| | | AA | SGSSSNIGSNYVS | DNNKRPS | GTWDSRLNVGV |
| | | | SEQ ID NO:3586 | SEQ ID NO:11598 | SEQ ID NO:19610 |
| iPS:436984 | | NA | GTTTTTAGCACTGGAGCAGTCACCAGTGGTTCCTTTCCAAAC | AGTACAAGCAACAAACACTCC | CTGCTCTACTGTGGTGGTGCTCAGCTGGTG |
| | 21-225_190F10 | | SEQ ID NO:3587 | SEQ ID NO:11599 | SEQ ID NO:19611 |
| | | AA | VFSTGAVTSGSFPN | STSNKHS | LLYCGGAQLV |
| | | | SEQ ID NO:3588 | SEQ ID NO:11600 | SEQ ID NO:19612 |
| iPS:436986 | | NA | TCTGGAAGCAGCTCCAACCTTGGAAATAATTTTGTATCC | GACAATTATAAGCGACCCTCA | GGAACATGGGATAGCAGCCTGAATACTGGGGTA |
| | 21-225_191A1 | | SEQ ID NO:3589 | SEQ ID NO:11601 | SEQ ID NO:19613 |
| | | AA | SGSSSNLGNNFVS | DNYKRPS | GTWDSSLNTGV |
| | | | SEQ ID NO:3590 | SEQ ID NO:11602 | SEQ ID NO:19614 |
| iPS:436988 | | NA | GTTCTTAGCACTGGAGCAGTCACCAGTGGTTCCTTTCCAAAC | AGTACAAGCAACAAACACTCC | ATGCTCTACTGTGGTGGTGCTCAGCTGGTG |
| | 21-225_191A2 | | SEQ ID NO:3591 | SEQ ID NO:11603 | SEQ ID NO:19615 |
| | | AA | VLSTGAVTSGSFPN | STSNKHS | MLYCGGAQLV |
| | | | SEQ ID NO:3592 | SEQ ID NO:11604 | SEQ ID NO:19616 |

FIGURE 49

| iPS:436992 | | NA | CAAGGAGACACCCTCAGACCCTATTATGCAAGT | GGTAAAAACAACCGGCCCTCA | AACTCCCGGGACAGCAGTGGTAACCATCTTGTGGTA |
|---|---|---|---|---|---|
| | 21-225_191B8 | | SEQ ID NO:3593 | SEQ ID NO:11605 | SEQ ID NO:19617 |
| | | AA | QGDTLRPYYAS | GKNNRPS | NSRDSSGNHLVV |
| | | | SEQ ID NO:3594 | SEQ ID NO:11606 | SEQ ID NO:19618 |
| iPS:436994 | | NA | CAAGGAGACAGCCTCAGACCCTATTATGCAAGC | GGTAAAAACAACCGGCCCTCA | AACTCCCGGGACAGCTGTGGTAACCATCTTGTGGTA |
| | 21-225_191A9 | | SEQ ID NO:3595 | SEQ ID NO:11607 | SEQ ID NO:19619 |
| | | AA | QGDSLRPYYAS | GKNNRPS | NSRDSCGNHLVV |
| | | | SEQ ID NO:3596 | SEQ ID NO:11608 | SEQ ID NO:19620 |
| iPS:436996 | | NA | TCTGGAAGCAGCTCCAACATCGGGAATAATTATGTATCC | GACAATAAAAAGCGACCCTCA | GGAACATGGGATAGCAGCCTGAGTGTTTGTGTC |
| | 21-225_191B9 | | SEQ ID NO:3597 | SEQ ID NO:11609 | SEQ ID NO:19621 |
| | | AA | SGSSSNIGNNYVS | DNKKRPS | GTWDSSLSVCV |
| | | | SEQ ID NO:3598 | SEQ ID NO:11610 | SEQ ID NO:19622 |
| iPS:437000 | | NA | ACCTTACGCAGTGGCATCAATGTTGGTACCTACAGGATATAC | TACAAATCAGACTCAGATAAGCAGCAGGGCTCT | ATGATTTGGCACAGCAGCGCTGTGGTA |
| | 21-225_191G9 | | SEQ ID NO:3599 | SEQ ID NO:11611 | SEQ ID NO:19623 |
| | | AA | TLRSGINVGTYRIY | YKSDSDKQQGS | MIWHSSAVV |
| | | | SEQ ID NO:3600 | SEQ ID NO:11612 | SEQ ID NO:19624 |
| iPS:437002 | | NA | GCTTCCAGCACTGGAGCAGTCACCAGTGCTTACTATCCAAAC | AGTACAAACAACAAACACTCC | CTGATCTTCTATGGTGGTGTACATGTGATA |
| | 21-225_191H9 | | SEQ ID NO:3601 | SEQ ID NO:11613 | SEQ ID NO:19625 |
| | | AA | ASSTGAVTSAYYPN | STNNKHS | LIFYGGVHVI |
| | | | SEQ ID NO:3602 | SEQ ID NO:11614 | SEQ ID NO:19626 |

FIGURE 49

| iPS:437006 | | NA | TCTGGAAGCAGCTCCAACATTGGGAATAATTATGTATCC | GACAATAATAAGCGACCCTCA | GGAACATGGGATAGCAGCCTGAGTGCTGGGGTA |
|---|---|---|---|---|---|
| | 21-225_192G2 | | SEQ ID NO:3603 | SEQ ID NO:11615 | SEQ ID NO:19627 |
| | | AA | SGSSSNIGNNYVS | DNNKRPS | GTWDSSLSAGV |
| | | | SEQ ID NO:3604 | SEQ ID NO:11616 | SEQ ID NO:19628 |
| iPS:437008 | | NA | GCTTTCAGCACTGGATCAGTCACCAGTGGTTCCTATCCAAAC | AGTACAAACAACAAACACTCC | CTGCTATACTATGGTGGTGCTCAGCTGGTG |
| | 21-225_192E3 | | SEQ ID NO:3605 | SEQ ID NO:11617 | SEQ ID NO:19629 |
| | | AA | AFSTGSVTSGSYPN | STNNKHS | LLYYGGAQLV |
| | | | SEQ ID NO:3606 | SEQ ID NO:11618 | SEQ ID NO:19630 |
| iPS:437010 | | NA | TCTGGAAGCAGCTCCAACATCGGAAGTAATACTGTAAAC | GGTAATAAGCAGCGGCCCTCA | GCAGCGTGGGATGACAGCCTGAATGGTTGGGTG |
| | 21-225_192G3 | | SEQ ID NO:3607 | SEQ ID NO:11619 | SEQ ID NO:19631 |
| | | AA | SGSSSNIGSNTVN | GNKQRPS | AAWDDSLNGWV |
| | | | SEQ ID NO:3608 | SEQ ID NO:11620 | SEQ ID NO:19632 |
| iPS:437012 | | NA | GCTTCCAGCACTGGAGCAGTCACCAGTGGTAACTATCCACAG | AGTACAACCAACAGACATTCC | CTGTTCTACTATGGTGGTGCTCAGGTGATA |
| | 21-225_192G7 | | SEQ ID NO:3609 | SEQ ID NO:11621 | SEQ ID NO:19633 |
| | | AA | ASSTGAVTSGNYPQ | STTNRHS | LFYYGGAQVI |
| | | | SEQ ID NO:3610 | SEQ ID NO:11622 | SEQ ID NO:19634 |
| iPS:437014 | | NA | GCTTTCAGCACTGGAACAGTCACCAGTGGTTTCTATCCAAAC | AATACAAGCAACAGACACTCC | CTGCTGTACTATGGTGGTGCTCAGCTGATG |
| | 21-225_192H8 | | SEQ ID NO:3611 | SEQ ID NO:11623 | SEQ ID NO:19635 |
| | | AA | AFSTGTVTSGFYPN | NTSNRHS | LLYYGGAQLM |
| | | | SEQ ID NO:3612 | SEQ ID NO:11624 | SEQ ID NO:19636 |

FIGURE 49

| iPS:437016 | | NA | CAAGGAGACAGCCTCAGAA GCTATTATGCAAAC | GCTAAGAACAACCGGCC CTCA | AATTCCCGGGACAGCAGTGG TAACCATCTGGTA |
|---|---|---|---|---|---|
| | 21-225_193A6 | | SEQ ID NO:3613 | SEQ ID NO:11625 | SEQ ID NO:19637 |
| | | AA | QGDSLRSYYAN | AKNNRPS | NSRDSSGNHLV |
| | | | SEQ ID NO:3614 | SEQ ID NO:11626 | SEQ ID NO:19638 |
| iPS:437018 | | NA | TCTGGAGATAAATTGGGGGA TAGATTTGCTTGC | CAAGATAGCAAGCGGCC CTCA | CAGGCGTGGGACAGCAGCAC TGCGGTA |
| | 21-225_193H5 | | SEQ ID NO:3615 | SEQ ID NO:11627 | SEQ ID NO:19639 |
| | | AA | SGDKLGDRFAC | QDSKRPS | QAWDSSTAV |
| | | | SEQ ID NO:3616 | SEQ ID NO:11628 | SEQ ID NO:19640 |
| iPS:437020 | | NA | TTCGGAGGCAGCTCCAACAT TGGGAATAATTATGTATCC | GACAATAATAAGCGACC CTCA | GGAACATGGGATCGCACCAT GAGTGATTGGGTA |
| | 21-225_193F11 | | SEQ ID NO:3617 | SEQ ID NO:11629 | SEQ ID NO:19641 |
| | | AA | FGGSSNIGNNYVS | DNNKRPS | GTWDRTMSDWV |
| | | | SEQ ID NO:3618 | SEQ ID NO:11630 | SEQ ID NO:19642 |
| iPS:437022 | | NA | GCTTCCAGCACTGGAGCAGT CACCAGTGGTAACTATCCAA AC | AGTACAAGCAACAAACA CTCC | CTGATCTACTATGGTGGTGC TCAGCTGATG |
| | 21-225_194G5 | | SEQ ID NO:3619 | SEQ ID NO:11631 | SEQ ID NO:19643 |
| | | AA | ASSTGAVTSGNYPN | STSNKHS | LIYYGGAQLM |
| | | | SEQ ID NO:3620 | SEQ ID NO:11632 | SEQ ID NO:19644 |
| iPS:437024 | | NA | TCTGGAAGCAGCTCCAACAT TGGGAATAATTATGTATCC | GACAATAATAAGCGACC CTCA | GGAACATGGGATAGCAGCCT GAGTGCTGGGGTT |
| | 21-225_194F11 | | SEQ ID NO:3621 | SEQ ID NO:11633 | SEQ ID NO:19645 |
| | | AA | SGSSSNIGNNYVS | DNNKRPS | GTWDSSLSAGV |
| | | | SEQ ID NO:3622 | SEQ ID NO:11634 | SEQ ID NO:19646 |

FIGURE 49

| iPS:437026 | | NA | GCTTCCAGCACTGGAGCAGT CACCAGTGGTTCCTTTCCAA GC | AGTACAAGCAACAGACA CTCC | CTGATCTACTATGGTGGTGC TCAGCTGGCA |
|---|---|---|---|---|---|
| | 21-225_194D12 | | SEQ ID NO:3623 | SEQ ID NO:11635 | SEQ ID NO:19647 |
| | | AA | ASSTGAVTSGSFPS | STSNRHS | LIYYGGAQLA |
| | | | SEQ ID NO:3624 | SEQ ID NO:11636 | SEQ ID NO:19648 |
| iPS:437028 | | NA | TCTGGAAGCAGCTCCAACAT TGGGAATAATTATGTATCC | GACAATAATAAGCGACC CTCA | GGAACATGGGATAGCAGCCT GAGTGTTGGGGTA |
| | 21-225_194G12 | | SEQ ID NO:3625 | SEQ ID NO:11637 | SEQ ID NO:19649 |
| | | AA | SGSSSNIGNNYVS | DNNKRPS | GTWDSSLSVGV |
| | | | SEQ ID NO:3626 | SEQ ID NO:11638 | SEQ ID NO:19650 |
| iPS:437030 | | NA | TCTGGAGATAAATTGGGGTA TAGATCTGTTTGC | GAAGATAGCAAGCGACC CTCA | CAGGCGTGGGACAGTGTCAC TGTGGTA |
| | 21-225_195E3 | | SEQ ID NO:3627 | SEQ ID NO:11639 | SEQ ID NO:19651 |
| | | AA | SGDKLGYRSVC | EDSKRPS | QAWDSVTVV |
| | | | SEQ ID NO:3628 | SEQ ID NO:11640 | SEQ ID NO:19652 |
| iPS:437032 | | NA | TCTGGAAGCAGCTCCAACAT CGGAAGTCATACTGTAAAC | AATAATTATCAGCGGCC CTCA | GCAACATGGGATGACAGCCT GAGTGTTTGGGTG |
| | 21-225_195H6 | | SEQ ID NO:3629 | SEQ ID NO:11641 | SEQ ID NO:19653 |
| | | AA | SGSSSNIGSHTVN | NNYQRPS | ATWDDSLSVWV |
| | | | SEQ ID NO:3630 | SEQ ID NO:11642 | SEQ ID NO:19654 |
| iPS:437034 | | NA | TCAGGAGATAAATTGGGGA ATAAATATGCTTAC | CAAGATAGGAAGCGGCC CTCA | CAGGCGTGGGACAGAGGAAT TGTGGTA |
| | 21-225_195E9 | | SEQ ID NO:3631 | SEQ ID NO:11643 | SEQ ID NO:19655 |
| | | AA | SGDKLGNKYAY | QDRKRPS | QAWDRGIVV |
| | | | SEQ ID NO:3632 | SEQ ID NO:11644 | SEQ ID NO:19656 |

FIGURE 49

| iPS:437036 | | NA | TCTGGAGGCAGCTCCAACAT TGGGAATAATTATGTATCC | GACAATAATAAGCGACC CTCA | GGAACATGGGATCGCACCAT GAGTGATTGGGTA |
|---|---|---|---|---|---|
| | 21-225_195H9 | | SEQ ID NO:3633 | SEQ ID NO:11645 | SEQ ID NO:19657 |
| | | AA | SGGSSNIGNNYVS | DNNKRPS | GTWDRTMSDWV |
| | | | SEQ ID NO:3634 | SEQ ID NO:11646 | SEQ ID NO:19658 |
| iPS:437040 | | NA | GCTTCCAGCACTGGAGCAGT CACCAGTGCTTACTATCCAA AC | AGTACAAACAACAAACA CTCC | CTGATTTTCTATGGTGGTGTA CATGTGATA |
| | 21-225_196E7 | | SEQ ID NO:3635 | SEQ ID NO:11647 | SEQ ID NO:19659 |
| | | AA | ASSTGAVTSAYYPN | STNNKHS | LIFYGGVHVI |
| | | | SEQ ID NO:3636 | SEQ ID NO:11648 | SEQ ID NO:19660 |
| iPS:437042 | | NA | TCTGGAAGCAGCTCCAACAT TGGGAATAAATATGTATCC | GACAATAATAAGCGACC CTCA | GGAATATGGGATCGCAGTCT GAGTGTTATGGTG |
| | 21-225_197E8 | | SEQ ID NO:3637 | SEQ ID NO:11649 | SEQ ID NO:19661 |
| | | AA | SGSSSNIGNKYVS | DNNKRPS | GIWDRSLSVMV |
| | | | SEQ ID NO:3638 | SEQ ID NO:11650 | SEQ ID NO:19662 |
| iPS:437044 | | NA | TCTGGAAGCAGCTCCAACAT CGGAAGTAATACTGTAAAC | AGTAATAATCAGCGGCC CTCA | GCAGCATGGGATGACAGTAT GAATGGTCCGGTG |
| | 21-225_197F9 | | SEQ ID NO:3639 | SEQ ID NO:11651 | SEQ ID NO:19663 |
| | | AA | SGSSSNIGSNTVN | SNNQRPS | AAWDDSMNGPV |
| | | | SEQ ID NO:3640 | SEQ ID NO:11652 | SEQ ID NO:19664 |
| iPS:437048 | | NA | GCTTTCAGCACTGGATCAGT CACCAGTGGTTCCTATCCAA AC | AGTACAAACAACAAACA CTCC | CTGCTCTACTATGGTGGTGC TCAGCTGGTG |
| | 21-225_197B11 | | SEQ ID NO:3641 | SEQ ID NO:11653 | SEQ ID NO:19665 |
| | | AA | AFSTGSVTSGSYPN | STNNKHS | LLYYGGAQLV |
| | | | SEQ ID NO:3642 | SEQ ID NO:11654 | SEQ ID NO:19666 |

FIGURE 49

| iPS:437050 | | NA | GCTTCCAGCACTGGAGCAGT CACCAGTGCTTACTATCCAA AC | AGTACAAGCAACAAACA CTCC | CTGATCTTCTATGGTGGTGT ACATGTGATA |
|---|---|---|---|---|---|
| | 21-225_197C11 | | SEQ ID NO:3643 | SEQ ID NO:11655 | SEQ ID NO:19667 |
| | | AA | ASSTGAVTSAYYPN | STSNKHS | LIFYGGVHVI |
| | | | SEQ ID NO:3644 | SEQ ID NO:11656 | SEQ ID NO:19668 |
| iPS:437054 | | NA | TCTGGAAGCAGCTCCAACAT CGGGAATAATTATATATCC | GACAATAAAAAGCGACC CTCA | GGAACATGGGATAGCAGCCT GAGTGTTTGTGTC |
| | 21-225_194G3 | | SEQ ID NO:3645 | SEQ ID NO:11657 | SEQ ID NO:19669 |
| | | AA | SGSSSNIGNNYIS | DNKKRPS | GTWDSSLSVCV |
| | | | SEQ ID NO:3646 | SEQ ID NO:11658 | SEQ ID NO:19670 |
| iPS:437056 | | NA | GTTCTTAGCACTGGAGCAGT CACCAGTGGTTCCTTTCCAA AC | AGTACAAGCAACAAACA TTCC | ATGCTTTACAGTGGTGGAGC TCAGATGGTG |
| | 21-225_198B8 | | SEQ ID NO:3647 | SEQ ID NO:11659 | SEQ ID NO:19671 |
| | | AA | VLSTGAVTSGSFPN | STSNKHS | MLYSGGAQMV |
| | | | SEQ ID NO:3648 | SEQ ID NO:11660 | SEQ ID NO:19672 |
| iPS:437058 | | NA | TCTGGAAGCAGCTCCAACAT TGGGAATAATTATGTATCC | GACAATAATAAGCGCCC CTCA | GGAACATGGGATAGCAGCCT GAGTGCTTGTGTC |
| | 21-225_199F3 | | SEQ ID NO:3649 | SEQ ID NO:11661 | SEQ ID NO:19673 |
| | | AA | SGSSSNIGNNYVS | DNNKRPS | GTWDSSLSACV |
| | | | SEQ ID NO:3650 | SEQ ID NO:11662 | SEQ ID NO:19674 |
| iPS:437060 | | NA | TCTGGAAGCAGCTCCAACAT CGGAAGTAATACTGTAAAC | AGTAATAATCAGCGGCC CTCA | GCAGCATGGGATGACAGCCT GAATGGTCCGGTG |
| | 21-225_199C3 | | SEQ ID NO:3651 | SEQ ID NO:11663 | SEQ ID NO:19675 |
| | | AA | SGSSSNIGSNTVN | SNNQRPS | AAWDDSLNGPV |
| | | | SEQ ID NO:3652 | SEQ ID NO:11664 | SEQ ID NO:19676 |

FIGURE 49

| iPS:437062 | | NA | GCTTCCAACACTGGAGCAGT CACCAGTGGTTCCTATCCAA AC | CATACAAACAACAAACA CTCC | CTGATCTACTATGGTGGTGC TCAGCTGGTG |
|---|---|---|---|---|---|
| | 21-225_200H1 | | SEQ ID NO:3653 | SEQ ID NO:11665 | SEQ ID NO:19677 |
| | | AA | ASNTGAVTSGSYPN | HTNNKHS | LIYYGGAQLV |
| | | | SEQ ID NO:3654 | SEQ ID NO:11666 | SEQ ID NO:19678 |
| iPS:437064 | | NA | TCTGGAAGCAGCTCCAACCT TGGAAATAATTTTGTATCC | GACAATTATAAGCGACC CTCA | GGAACTTGGGATAGCAGCCT GAATACTGGGGTA |
| | 21-225_200G8 | | SEQ ID NO:3655 | SEQ ID NO:11667 | SEQ ID NO:19679 |
| | | AA | SGSSSNLGNNFVS | DNYKRPS | GTWDSSLNTGV |
| | | | SEQ ID NO:3656 | SEQ ID NO:11668 | SEQ ID NO:19680 |
| iPS:437066 | | NA | GCTTCCAACACTGGAGCAGT CACCAGTGGTTCCTATCCAA AT | CATACAGACAACAAACA CTCC | CTGATCTACTATGGTGGTGC TCAGCTGGTG |
| | 21-225_200G9 | | SEQ ID NO:3657 | SEQ ID NO:11669 | SEQ ID NO:19681 |
| | | AA | ASNTGAVTSGSYPN | HTDNKHS | LIYYGGAQLV |
| | | | SEQ ID NO:3658 | SEQ ID NO:11670 | SEQ ID NO:19682 |
| iPS:437068 | | NA | GCTTCCAGCACTGGAGCAGT CACCAGTGGTTACTATCCAA AC | AGTACAAACAACAAACA CTCC | CTGCTCTATTATGGTGGTGCT CACCTGGCA |
| | 21-225_200A11 | | SEQ ID NO:3659 | SEQ ID NO:11671 | SEQ ID NO:19683 |
| | | AA | ASSTGAVTSGYYPN | STNNKHS | LLYYGGAHLA |
| | | | SEQ ID NO:3660 | SEQ ID NO:11672 | SEQ ID NO:19684 |
| iPS:437070 | | NA | TCTGGAGATAAATTGGGGGA TAGATTTGCTTGC | CAAGATAGCAAGCGGCC CTCA | CAGGCGTGGGACAGCAGCAC TGTGGTA |
| | 21-225_201G11 | | SEQ ID NO:3661 | SEQ ID NO:11673 | SEQ ID NO:19685 |
| | | AA | SGDKLGDRFAC | QDSKRPS | QAWDSSTVV |
| | | | SEQ ID NO:3662 | SEQ ID NO:11674 | SEQ ID NO:19686 |

FIGURE 49

| iPS:437074 | | NA | GGGGGAAACAACATTGGAA GAAAAAATGTGCAC | AGGGATAGCGACCGGCC CTCT | CAGGTGTGGGACAGCGGCAC TGCGGTA |
|---|---|---|---|---|---|
| | 21-225_203B2 | | SEQ ID NO:3663 | SEQ ID NO:11675 | SEQ ID NO:19687 |
| | | AA | GGNNIGRKNVH | RDSDRPS | QVWDSGTAV |
| | | | SEQ ID NO:3664 | SEQ ID NO:11676 | SEQ ID NO:19688 |
| iPS:437076 | | NA | TCTGGAGATAAATTGGGGGA TAGATTTGCTTGC | CAAGATAACAAGCGGCC CTCA | CAGGCGTGGGACAGCAGCAC TGTGGTA |
| | 21-225_203G6 | | SEQ ID NO:3665 | SEQ ID NO:11677 | SEQ ID NO:19689 |
| | | AA | SGDKLGDRFAC | QDNKRPS | QAWDSSTVV |
| | | | SEQ ID NO:3666 | SEQ ID NO:11678 | SEQ ID NO:19690 |
| iPS:437082 | | NA | GGGGGAAACAACATTGGAA GAAAAAATGTGCAC | AGGGATAGCGACCGGCC CTCT | CAGGTGTGGGACAGCGGCAC TGCGGTA |
| | 21-225_205E12 | | SEQ ID NO:3667 | SEQ ID NO:11679 | SEQ ID NO:19691 |
| | | AA | GGNNIGRKNVH | RDSDRPS | QVWDSGTAV |
| | | | SEQ ID NO:3668 | SEQ ID NO:11680 | SEQ ID NO:19692 |
| iPS:437084 | | NA | GGGGGAAACAACATTGGAA GAAAAAATGTGCAC | AGGGATAGCTACCGATC TTCT | CAGGATTGGGACAGCAGCAC TGTGGTG |
| | 21-225_206B5 | | SEQ ID NO:3669 | SEQ ID NO:11681 | SEQ ID NO:19693 |
| | | AA | GGNNIGRKNVH | RDSYRSS | QDWDSSTVV |
| | | | SEQ ID NO:3670 | SEQ ID NO:11682 | SEQ ID NO:19694 |
| iPS:437086 | | NA | TCTGGAAGCAGCTCCAACAT TGGGAGTAATTTTTTATCC | GACAATAATAAGCGACC CTCA | GGAACATGGGATAGCAGCCT GAGTGCTGGGGTA |
| | 21-225_209A8 | | SEQ ID NO:3671 | SEQ ID NO:11683 | SEQ ID NO:19695 |
| | | AA | SGSSSNIGSNFLS | DNNKRPS | GTWDSSLSAGV |
| | | | SEQ ID NO:3672 | SEQ ID NO:11684 | SEQ ID NO:19696 |
| iPS:437088 | | NA | GGGGGAAACAACATTGGAA GAAAAAATGTGCAC | AGGGATAGCTACCGGTC TTCT | CAGGATTGGGACAGCAGCAC TGTGGTG |
| | 21-225_209H10 | | SEQ ID NO:3673 | SEQ ID NO:11685 | SEQ ID NO:19697 |
| | | AA | GGNNIGRKNVH | RDSYRSS | QDWDSSTVV |
| | | | SEQ ID NO:3674 | SEQ ID NO:11686 | SEQ ID NO:19698 |

FIGURE 49

| iPS:437090 | | NA | GCTTTCAGCACTGGAGCAGTCACCAGTGGTAATTATCCAAAC | AGTACAAGCAACAAACACTCC | CTGCTCTACTATGGTGGTGCTCAGCTGGTG |
|---|---|---|---|---|---|
| | 21-225_210F11 | | SEQ ID NO:3675 | SEQ ID NO:11687 | SEQ ID NO:19699 |
| | | AA | AFSTGAVTSGNYPN | STSNKHS | LLYYGGAQLV |
| | | | SEQ ID NO:3676 | SEQ ID NO:11688 | SEQ ID NO:19700 |
| iPS:437092 | | NA | ACTGGAACCAGCAGTGACGTTGGTGGTTATAACTATGTCTCC | GAGGTCAGGAATCGGCCCTCA | AGCTCATATACCAGCAGCCGCACTCTGGTA |
| | 21-225_210B12 | | SEQ ID NO:3677 | SEQ ID NO:11689 | SEQ ID NO:19701 |
| | | AA | TGTSSDVGGYNYVS | EVRNRPS | SSYTSSRTLV |
| | | | SEQ ID NO:3678 | SEQ ID NO:11690 | SEQ ID NO:19702 |
| iPS:437094 | | NA | ACTGGAACCAGCAGTGACGTTGGTGGTTATAATTATGTCTCC | GAGGTCAGTAATCGGCCCTCA | AACTCATATACAAGCAGCATCACTTGGGTG |
| | 21-225_210D12 | | SEQ ID NO:3679 | SEQ ID NO:11691 | SEQ ID NO:19703 |
| | | AA | TGTSSDVGGYNYVS | EVSNRPS | NSYTSSITWV |
| | | | SEQ ID NO:3680 | SEQ ID NO:11692 | SEQ ID NO:19704 |
| iPS:437096 | | NA | ACTGGAACCAGCAGTGACGTTGGTGGTTATAACTATGTCTCC | GAGGTCAGTAATCGGCCCTCA | GGCTCATATGTAAAAGGCATCACTTGGGTG |
| | 21-225_210E12 | | SEQ ID NO:3681 | SEQ ID NO:11693 | SEQ ID NO:19705 |
| | | AA | TGTSSDVGGYNYVS | EVSNRPS | GSYVKGITWV |
| | | | SEQ ID NO:3682 | SEQ ID NO:11694 | SEQ ID NO:19706 |
| iPS:437098 | | NA | ACTGGAACCAGTAGTGACGTTGGTAGTTATAACTATGTCTCC | GAGGTCAGTAATCGGCCCTCA | AACTCATATACAAGCAGCATCACTTGGGTG |
| | 21-225_211C1 | | SEQ ID NO:3683 | SEQ ID NO:11695 | SEQ ID NO:19707 |
| | | AA | TGTSSDVGSYNYVS | EVSNRPS | NSYTSSITWV |
| | | | SEQ ID NO:3684 | SEQ ID NO:11696 | SEQ ID NO:19708 |

FIGURE 49

| iPS:437100 | | NA | GGGGGAAACAACATTGGAC GTAGAAATGTGCAC | AGAGATCGCGACCGGCC CTCT | CAGGTGTGGGACAGCAGTAC TGCGGTG |
|---|---|---|---|---|---|
| | 21-225_211H2 | | SEQ ID NO:3685 | SEQ ID NO:11697 | SEQ ID NO:19709 |
| | | AA | GGNNIGRRNVH | RDRDRPS | QVWDSSTAV |
| | | | SEQ ID NO:3686 | SEQ ID NO:11698 | SEQ ID NO:19710 |
| iPS:437102 | | NA | TCTGGAGATGCATTGCCAAA GCAATATGCTTAT | AAAGACAGTGCGAGGCC CTCA | CAATTAGTGTACAGCAGTGA TACTTATGTC |
| | 21-225_211E5 | | SEQ ID NO:3687 | SEQ ID NO:11699 | SEQ ID NO:19711 |
| | | AA | SGDALPKQYAY | KDSARPS | QLVYSSDTYV |
| | | | SEQ ID NO:3688 | SEQ ID NO:11700 | SEQ ID NO:19712 |
| iPS:437104 | | NA | ACTGGAACCAGCAGTGACGT TGGTGGTTATAACTATGTCT CC | GAGGTCAGTAATCGGCC CTCA | AACTCATATACAAGAAGCAT CACTTGGGTG |
| | 21-225_211G5 | | SEQ ID NO:3689 | SEQ ID NO:11701 | SEQ ID NO:19713 |
| | | AA | TGTSSDVGGYNYVS | EVSNRPS | NSYTRSITWV |
| | | | SEQ ID NO:3690 | SEQ ID NO:11702 | SEQ ID NO:19714 |
| iPS:437106 | | NA | GCTTTCAGCACTGGAGCAGT CACCAGTGGTAACTATCCAA GT | AGTACAAGCAACAGACA CTCC | CTGCTCTACTATGGTGGTGC TCAGCTGGTG |
| | 21-225_211H7 | | SEQ ID NO:3691 | SEQ ID NO:11703 | SEQ ID NO:19715 |
| | | AA | AFSTGAVTSGNYPS | STSNRHS | LLYYGGAQLV |
| | | | SEQ ID NO:3692 | SEQ ID NO:11704 | SEQ ID NO:19716 |
| iPS:437108 | | NA | GGTTCCAGCACTGGATCAGT CACCAGTGGTTACTTTCCAA AC | AGTACAAACAACAAGCA CTCC | CTGCTCTACTATGGTGGTGC TCAGCTGGCA |
| | 21-225_211C9 | | SEQ ID NO:3693 | SEQ ID NO:11705 | SEQ ID NO:19717 |
| | | AA | GSSTGSVTSGYFPN | STNNKHS | LLYYGGAQLA |
| | | | SEQ ID NO:3694 | SEQ ID NO:11706 | SEQ ID NO:19718 |

FIGURE 49

| iPS:437110 |  | NA | GCTTCCAGCACTGGAGCAGT CACCAGTGGTAACTATCCAA AC | AGTACAATCAACAAACA CTCC | CTGCTCTACTATGGTGGTGC TCAGCTGGCA |
|---|---|---|---|---|---|
|  | 21-225_211E9 |  | SEQ ID NO:3695 | SEQ ID NO:11707 | SEQ ID NO:19719 |
|  |  | AA | ASSTGAVTSGNYPN | STINKHS | LLYYGGAQLA |
|  |  |  | SEQ ID NO:3696 | SEQ ID NO:11708 | SEQ ID NO:19720 |
| iPS:437112 |  | NA | ACTGGAACCAGCAGTGACGT TGGTGGTTATAACTATGTCT CC | GAGGTCAGAAATCGGCC CTCA | AGCTCATATACACGCAGCAT CACTTGGGTG |
|  | 21-225_212C2 |  | SEQ ID NO:3697 | SEQ ID NO:11709 | SEQ ID NO:19721 |
|  |  | AA | TGTSSDVGGYNYVS | EVRNRPS | SSYTRSITWV |
|  |  |  | SEQ ID NO:3698 | SEQ ID NO:11710 | SEQ ID NO:19722 |
| iPS:437114 |  | NA | ACTGGAACCAGCAGTGACGT TGGTGGTTATAACTATGTCT CC | GAGGTCAGTAATCGGCC CTCA | GGCTCATATGTAAAAGGCAT CACTTGGGTG |
|  | 21-225_212A4 |  | SEQ ID NO:3699 | SEQ ID NO:11711 | SEQ ID NO:19723 |
|  |  | AA | TGTSSDVGGYNYVS | EVSNRPS | GSYVKGITWV |
|  |  |  | SEQ ID NO:3700 | SEQ ID NO:11712 | SEQ ID NO:19724 |
| iPS:437116 |  | NA | ACTGGAACCAGTAGTGACGT TGGTGGTTATAACTATGTCT CC | GAGGTCAGTAATCGGCC CTCA | AACTCATATACAAGCAGCAT CACTTGGGTG |
|  | 21-225_212F6 |  | SEQ ID NO:3701 | SEQ ID NO:11713 | SEQ ID NO:19725 |
|  |  | AA | TGTSSDVGGYNYVS | EVSNRPS | NSYTSSITWV |
|  |  |  | SEQ ID NO:3702 | SEQ ID NO:11714 | SEQ ID NO:19726 |
| iPS:437118 |  | NA | ACTGGAACCAGCAGTGACGT TGGTGGTTATAATTATGTCT CC | GAGGTCAGTAATCGGCC CTCA | AACTCATATACAAGCAGCAT CACTTGGGTG |
|  | 21-225_212G7 |  | SEQ ID NO:3703 | SEQ ID NO:11715 | SEQ ID NO:19727 |
|  |  | AA | TGTSSDVGGYNYVS | EVSNRPS | NSYTSSITWV |
|  |  |  | SEQ ID NO:3704 | SEQ ID NO:11716 | SEQ ID NO:19728 |

FIGURE 49

| iPS:437120 | | NA | GCTTCCAGCACTGGAGCAGT CACCAGTGGTTACTATCCAA AC | AGTACAAACAACAAACA CTCC | CTGCTCTACTATGGTGGTGC TCAGGTGGGA |
|---|---|---|---|---|---|
| | 21-225_212A9 | | SEQ ID NO:3705 | SEQ ID NO:11717 | SEQ ID NO:19729 |
| | | AA | ASSTGAVTSGYYPN | STNNKHS | LLYYGGAQVG |
| | | | SEQ ID NO:3706 | SEQ ID NO:11718 | SEQ ID NO:19730 |
| iPS:437124 | | NA | GCTTCCAGCACTGGAGCAGT CACCAGTGGTTACTATCCAA AC | AGTACAAGCAACAAACA CTCC | CTGCTCTACTATGGTGGTGC TCATGTGGTA |
| | 21-225_212H12 | | SEQ ID NO:3707 | SEQ ID NO:11719 | SEQ ID NO:19731 |
| | | AA | ASSTGAVTSGYYPN | STSNKHS | LLYYGGAHVV |
| | | | SEQ ID NO:3708 | SEQ ID NO:11720 | SEQ ID NO:19732 |
| iPS:437128 | | NA | ACTGGAACCAGCAGTGACGT TGGTGGTTATAACTATGTCT CC | GAGGTCAGGAATCGGCC CTCA | AACTCATATACACGCAGCAT CACTTGGGTG |
| | 21-225_213G3 | | SEQ ID NO:3709 | SEQ ID NO:11721 | SEQ ID NO:19733 |
| | | AA | TGTSSDVGGYNYVS | EVRNRPS | NSYTRSITWV |
| | | | SEQ ID NO:3710 | SEQ ID NO:11722 | SEQ ID NO:19734 |
| iPS:437130 | | NA | ACTGGAACCAGCAGTGACGT TGGTGGTTATAACTATGTCT CC | GAGGTCCGTAATCGGCC CTCA | TGCTCATATACAAGAAGAAT CACTTGGGTG |
| | 21-225_213D5 | | SEQ ID NO:3711 | SEQ ID NO:11723 | SEQ ID NO:19735 |
| | | AA | TGTSSDVGGYNYVS | EVRNRPS | CSYTRRITWV |
| | | | SEQ ID NO:3712 | SEQ ID NO:11724 | SEQ ID NO:19736 |
| iPS:437132 | | NA | GGTTCCAGCACTGGATCAGT CACCAGTGGTTACTTTCCAA AC | AGTACAAACAACAAGCA CTCC | CTGCTCTACTTTGGTGGTGCT CAGCTGGCA |
| | 21-225_213F5 | | SEQ ID NO:3713 | SEQ ID NO:11725 | SEQ ID NO:19737 |
| | | AA | GSSTGSVTSGYFPN | STNNKHS | LLYFGGAQLA |
| | | | SEQ ID NO:3714 | SEQ ID NO:11726 | SEQ ID NO:19738 |

FIGURE 49

| iPS:437134 | 21-225_213A7 | NA | ACTGGAACCAGCAGTGACGT TGGTGGTTATAACTATGTCT CC | GAGGTCAGGAATCGGCC CTCA | AGCTCATATACCAGCAGCCG CACTCTGGTA |
| --- | --- | --- | --- | --- | --- |
| | | | SEQ ID NO:3715 | SEQ ID NO:11727 | SEQ ID NO:19739 |
| | | AA | TGTSSDVGGYNYVS | EVRNRPS | SSYTSSRTLV |
| | | | SEQ ID NO:3716 | SEQ ID NO:11728 | SEQ ID NO:19740 |
| iPS:437136 | 21-225_214H3 | NA | GCTTCCAGCACTGGAGCAGT CACCAGTGGTTACTATCCAA AC | AGTACAAGCAACAAACA CTCC | CTGCTCTACTATGGTGGTGC TCATGTGGTA |
| | | | SEQ ID NO:3717 | SEQ ID NO:11729 | SEQ ID NO:19741 |
| | | AA | ASSTGAVTSGYYPN | STSNKHS | LLYYGGAHVV |
| | | | SEQ ID NO:3718 | SEQ ID NO:11730 | SEQ ID NO:19742 |
| iPS:437138 | 21-225_214D8 | NA | TCTGGAAGCAGCTCCAACAT TGGGAATAATTATGTATCC | GACAATAATAAGCGACC CTCA | GGAGCATGGGATAGCAGCCT GAGTGCTGTGGTA |
| | | | SEQ ID NO:3719 | SEQ ID NO:11731 | SEQ ID NO:19743 |
| | | AA | SGSSSNIGNNYVS | DNNKRPS | GAWDSSLSAVV |
| | | | SEQ ID NO:3720 | SEQ ID NO:11732 | SEQ ID NO:19744 |
| iPS:437140 | 21-225_214E12 | NA | GCTTCCAGCACTGGAGCAGT CACCAGTGGCTACTATCCAA AC | AGTACAAGCAATAAACA CTCC | CTGCTCTACTGTGATGGTGC CCAGCTGGTG |
| | | | SEQ ID NO:3721 | SEQ ID NO:11733 | SEQ ID NO:19745 |
| | | AA | ASSTGAVTSGYYPN | STSNKHS | LLYCDGAQLV |
| | | | SEQ ID NO:3722 | SEQ ID NO:11734 | SEQ ID NO:19746 |
| iPS:437142 | 21-225_215A3 | NA | GCTTCCAGCACTGAAGCCGT CACCAGTGGTAACTATCCAA GC | AGTACAAGCAACAAACA CTCC | CTGCTCTACTATGGTGGCGC TCAGCTGGCA |
| | | | SEQ ID NO:3723 | SEQ ID NO:11735 | SEQ ID NO:19747 |
| | | AA | ASSTEAVTSGNYPS | STSNKHS | LLYYGGAQLA |
| | | | SEQ ID NO:3724 | SEQ ID NO:11736 | SEQ ID NO:19748 |

900

FIGURE 49

| iPS:437144 | | NA | TCTGGAGATAAATTGGGGGA TAAATTTGCTTGC | CAAGATAGCAAGCGGCC CTCA | CAGGCGTGGGACAGCAGCAC TGTGGTA |
| --- | --- | --- | --- | --- | --- |
| | 21-225_215B3 | | SEQ ID NO:3725 | SEQ ID NO:11737 | SEQ ID NO:19749 |
| | | AA | SGDKLGDKFAC | QDSKRPS | QAWDSSTVV |
| | | | SEQ ID NO:3726 | SEQ ID NO:11738 | SEQ ID NO:19750 |
| iPS:437146 | | NA | ACTGGAACCAGCAGTGACAT TGGTGGTTATAACTATGTCT CC | GAGGTCAGTAATCGGCC CTCA | AACTCATATAAAAGGGGCAG CACTTGGGTG |
| | 21-225_215D3 | | SEQ ID NO:3727 | SEQ ID NO:11739 | SEQ ID NO:19751 |
| | | AA | TGTSSDIGGYNYVS | EVSNRPS | NSYKRGSTWV |
| | | | SEQ ID NO:3728 | SEQ ID NO:11740 | SEQ ID NO:19752 |
| iPS:437148 | | NA | GCTTCCAGCACTGGAGCAGT CACCAGTGGTTACTATCCAA AC | AGTACAAACAACAAACA CTCC | CTGCTCTACTATGGTGGTGC TCAGGTGGGA |
| | 21-225_215H3 | | SEQ ID NO:3729 | SEQ ID NO:11741 | SEQ ID NO:19753 |
| | | AA | ASSTGAVTSGYYPN | STNNKHS | LLYYGGAQVG |
| | | | SEQ ID NO:3730 | SEQ ID NO:11742 | SEQ ID NO:19754 |
| iPS:437150 | | NA | ACTGGAACCAGCAGTGACGT TGGTGGTTATAATTATGTCT CC | GAGGTCAGTAATCGGCC CTCA | AACTCATATACAAGCAGCAT CACTTGGGTG |
| | 21-225_216A3 | | SEQ ID NO:3731 | SEQ ID NO:11743 | SEQ ID NO:19755 |
| | | AA | TGTSSDVGGYNYVS | EVSNRPS | NSYTSSITWV |
| | | | SEQ ID NO:3732 | SEQ ID NO:11744 | SEQ ID NO:19756 |
| iPS:437154 | | NA | GCTTCCAGCACTGGAGCAGT CACCAGTGGTTACTATCCAA AC | AGTACAAACAACAAACA CTCC | CTGCTCTACTATGGTGGTGC TCAGGTGGGA |
| | 21-225_216A7 | | SEQ ID NO:3733 | SEQ ID NO:11745 | SEQ ID NO:19757 |
| | | AA | ASSTGAVTSGYYPN | STNNKHS | LLYYGGAQVG |
| | | | SEQ ID NO:3734 | SEQ ID NO:11746 | SEQ ID NO:19758 |

FIGURE 49

| iPS:437158 | | NA | GCTTCCAGCACTGGAGCAGT CACCAGTGGCTACTATCCAA AC | AGTACAAGCAATAAACA CTCC | CTGCTCTACTGTGATGGTGC TCAGCTGGTG |
|---|---|---|---|---|---|
| | 21-225_216H11 | | SEQ ID NO:3735 | SEQ ID NO:11747 | SEQ ID NO:19759 |
| | | AA | ASSTGAVTSGYYPN | STSNKHS | LLYCDGAQLV |
| | | | SEQ ID NO:3736 | SEQ ID NO:11748 | SEQ ID NO:19760 |
| iPS:437160 | | NA | GGGGGAGACAACATTAGAA GAAGAAATGTGCAC | AGGGATAGCAACCGGCC CTCT | CAGGTGTGGGACAGCAGCAC TGGGGTG |
| | 21-225_216B12 | | SEQ ID NO:3737 | SEQ ID NO:11749 | SEQ ID NO:19761 |
| | | AA | GGDNIRRRNVH | RDSNRPS | QVWDSSTGV |
| | | | SEQ ID NO:3738 | SEQ ID NO:11750 | SEQ ID NO:19762 |
| iPS:437162 | | NA | ACTGGAACCAGCAGTGACGT TGGTGGTTATAACTATGTCT CC | GAGGTCAGTAATCGGCC CTCA | GGCTCATATGTAAAAGGCAT CACTTGGGTG |
| | 21-225_217B2 | | SEQ ID NO:3739 | SEQ ID NO:11751 | SEQ ID NO:19763 |
| | | AA | TGTSSDVGGYNYVS | EVSNRPS | GSYVKGITWV |
| | | | SEQ ID NO:3740 | SEQ ID NO:11752 | SEQ ID NO:19764 |
| iPS:437164 | | NA | TCTGGAGATGCATTGCCAAA GCAATATGCTTAT | AAAGACAGTGAGAGGCC CTCA | CAATTAATAGTCAGCAGTGA TACTTATGTC |
| | 21-225_217C6 | | SEQ ID NO:3741 | SEQ ID NO:11753 | SEQ ID NO:19765 |
| | | AA | SGDALPKQYAY | KDSERPS | QLIVSSDTYV |
| | | | SEQ ID NO:3742 | SEQ ID NO:11754 | SEQ ID NO:19766 |
| iPS:437166 | | NA | TCTGGAGATGCATTGCCAAA ACAATATGCTTAT | AAAGACAGTGAGAGGCC CTCA | CAATTAGTGTACAGCAGTGA TACTTATGTC |
| | 21-225_217G11 | | SEQ ID NO:3743 | SEQ ID NO:11755 | SEQ ID NO:19767 |
| | | AA | SGDALPKQYAY | KDSERPS | QLVYSSDTYV |
| | | | SEQ ID NO:3744 | SEQ ID NO:11756 | SEQ ID NO:19768 |
| iPS:437168 | 21 225 218G4 | NA | TCTGGAAGCAGCTCCAACAT TGGGAATAATTATGTATCC | GACAGTAATAAGCGACC CTCA | GGAACATGGGATAGCAGCCT GAATACTGTGGTA |

FIGURE 49

| | | | | | |
|---|---|---|---|---|---|
| | | | SEQ ID NO:3745 | SEQ ID NO:11757 | SEQ ID NO:19769 |
| | 21-225_218G4 | AA | SGSSSNIGNNYVS | DSNKRPS | GTWDSSLNTVV |
| | | | SEQ ID NO:3746 | SEQ ID NO:11758 | SEQ ID NO:19770 |
| iPS:437170 | | NA | TCTAGAGATGTATTGCCGAAGCAATATGCTTAT | AAAGACAGTGAGAGGCCCTCA | CAATTAGTTGTCAGCAGTGATACTTATGTC |
| | 21-225_218E5 | | SEQ ID NO:3747 | SEQ ID NO:11759 | SEQ ID NO:19771 |
| | | AA | SRDVLPKQYAY | KDSERPS | QLVVSSDTYV |
| | | | SEQ ID NO:3748 | SEQ ID NO:11760 | SEQ ID NO:19772 |
| iPS:437172 | | NA | ACTGGAACCAGCAGTGACGTTGGTGGTTATAATTATGTCTCC | GAGGTCAGAAATCGGCCCTCA | TGCTCATATACAAGGAGCATCACTTGGGTG |
| | 21-225_219A7 | | SEQ ID NO:3749 | SEQ ID NO:11761 | SEQ ID NO:19773 |
| | | AA | TGTSSDVGGYNYVS | EVRNRPS | CSYTRSITWV |
| | | | SEQ ID NO:3750 | SEQ ID NO:11762 | SEQ ID NO:19774 |
| iPS:437182 | | NA | ACTGGAACCAGCAGTGACGTTGGTGGTTATAACTATGTCTCC | GAGGTCAGGAATCGGCCCTCA | AACTCATATACACGCAGCATCACTTGGGTG |
| | 21-225_221H2 | | SEQ ID NO:3751 | SEQ ID NO:11763 | SEQ ID NO:19775 |
| | | AA | TGTSSDVGGYNYVS | EVRNRPS | NSYTRSITWV |
| | | | SEQ ID NO:3752 | SEQ ID NO:11764 | SEQ ID NO:19776 |
| iPS:437184 | | NA | ACTGGAACCAGCAGTGACGTTGGTGGTTATAACTATGTCTCC | GAGGTCAGGAATCGGCCCTCA | AACTCATATACACGCAGCATCACTTGGGTG |
| | 21-225_221G4 | | SEQ ID NO:3753 | SEQ ID NO:11765 | SEQ ID NO:19777 |
| | | AA | TGTSSDVGGYNYVS | EVRNRPS | NSYTRSITWV |
| | | | SEQ ID NO:3754 | SEQ ID NO:11766 | SEQ ID NO:19778 |
| iPS:437186 | | NA | TCTGGAGATAATTTGGGGGTTAAATATACTTAC | CAAGATAGCAAGCGGCCCTCA | CAGGCGTGGGACAGCAGCACTGTGGTA |
| | 21-225_224H2 | | SEQ ID NO:3755 | SEQ ID NO:11767 | SEQ ID NO:19779 |
| | | AA | SGDNLGVKYTY | QDSKRPS | QAWDSSTVV |

EP 4 435 105 A2

FIGURE 49

| | | | SEQ ID NO:3756 | SEQ ID NO:11768 | SEQ ID NO:19780 |
|---|---|---|---|---|---|
| iPS:437188 | 21-225_224B11 | NA | ACTGGGAGCAGCTCCAACAT CGGGGCAGGTTATGATGTAC AC | GGTAACAGCAATCGGCC CTCA | CAGTCCTATGACAACAGCCT GAGTGGTGTG |
| | | | SEQ ID NO:3757 | SEQ ID NO:11769 | SEQ ID NO:19781 |
| | | AA | TGSSSNIGAGYDVH | GNSNRPS | QSYDNSLSGV |
| | | | SEQ ID NO:3758 | SEQ ID NO:11770 | SEQ ID NO:19782 |
| iPS:437190 | 21-225_225A9 | NA | TCTGGAGATAAATTGGGGAA TAAATATGCTTGC | CAAGATAGCAAGCGGCC CTCA | CAGGCGTGGGACAGCAACAC TGCATGTGTC |
| | | | SEQ ID NO:3759 | SEQ ID NO:11771 | SEQ ID NO:19783 |
| | | AA | SGDKLGNKYAC | QDSKRPS | QAWDSNTACV |
| | | | SEQ ID NO:3760 | SEQ ID NO:11772 | SEQ ID NO:19784 |
| iPS:437192 | 21-225_225E9 | NA | TCTGGAGATAATTTGGGGAA TAGATATGCTTGC | CAAGATCGCAAGCGGCC CTCA | CAGGCGTGGGACAGCAGAAC TGCTGTGGTA |
| | | | SEQ ID NO:3761 | SEQ ID NO:11773 | SEQ ID NO:19785 |
| | | AA | SGDNLGNRYAC | QDRKRPS | QAWDSRTAVV |
| | | | SEQ ID NO:3762 | SEQ ID NO:11774 | SEQ ID NO:19786 |
| iPS:437194 | 21-225_226B2 | NA | TCTGGAGATACATTGGGGGG TAAATATGCTTGG | CAAGATCGCAAGCGGCC CTCA | CAGGCGTGGGACAACGGCGC TGCGGTT |
| | | | SEQ ID NO:3763 | SEQ ID NO:11775 | SEQ ID NO:19787 |
| | | AA | SGDTLGGKYAW | QDRKRPS | QAWDNGAAV |
| | | | SEQ ID NO:3764 | SEQ ID NO:11776 | SEQ ID NO:19788 |
| iPS:437196 | 21-225_226B7 | NA | TCTGGAGATGCATTGCCAAG GCATTATGTTTAT | AAAGACAGTGAGAGGCC CTCA | CAATCAGCAGACAGCAGTGG TACTTATGTC |
| | | | SEQ ID NO:3765 | SEQ ID NO:11777 | SEQ ID NO:19789 |
| | | AA | SGDALPRHYVY | KDSERPS | QSADSSGTYV |
| | | | SEQ ID NO:3766 | SEQ ID NO:11778 | SEQ ID NO:19790 |
| iPS:437198 | 21 225 226F8 | NA | ACTGGGAGCAGCTCCAACAT CGGGGCAGGTTATGATGTAC AC | GGTAACAGCAATCGGCC CTCA | CAGTCCTATGACAACAGCCT GAGTGGTGTG |

FIGURE 49

| | | | | | |
|---|---|---|---|---|---|
| | **21-225_226F8** | | | SEQ ID NO:3767 | SEQ ID NO:11779 | SEQ ID NO:19791 |
| | | AA | TGSSSNIGAGYDVH | GNSNRPS | QSYDNSLSGV |
| iPS:437200 | | NA | SEQ ID NO:3768 | SEQ ID NO:11780 | SEQ ID NO:19792 |
| | 21-225_226A10 | NA | TCTGGAGATACATTGGGGGGTAAATATGCTTGG | CAAGATCGCAAGCGGCCCTCA | CAGGCGTGGGACAACGGCGCTGCGGTT |
| | | | SEQ ID NO:3769 | SEQ ID NO:11781 | SEQ ID NO:19793 |
| | | AA | SGDTLGGKYAW | QDRKRPS | QAWDNGAAV |
| iPS:437202 | | | SEQ ID NO:3770 | SEQ ID NO:11782 | SEQ ID NO:19794 |
| | 21-225_227D3 | NA | ACTGGGAGCAGCTCCAACATCGGGGCAGGTTATGATGTACAC | GGTAACAGCAATCGGCCCTCA | CAGTCCTATGACAACAGCCTGAGTGGTGTG |
| | | | SEQ ID NO:3771 | SEQ ID NO:11783 | SEQ ID NO:19795 |
| | | AA | TGSSSNIGAGYDVH | GNSNRPS | QSYDNSLSGV |
| iPS:437204 | | | SEQ ID NO:3772 | SEQ ID NO:11784 | SEQ ID NO:19796 |
| | 21-225_227E5 | NA | TCTGGAGATAAATTGGGGGAAAAATATGCTTGC | CAAGATAGGAAGCGGCCCTCA | CAGGCGTGGGTCAACAACACTATGATA |
| | | | SEQ ID NO:3773 | SEQ ID NO:11785 | SEQ ID NO:19797 |
| | | AA | SGDKLGEKYAC | QDRKRPS | QAWVNNTMI |
| iPS:437208 | | | SEQ ID NO:3774 | SEQ ID NO:11786 | SEQ ID NO:19798 |
| | 21-225_227C10 | NA | ACTGGGAGCAGCTCCAACATCGGGGCAGGTTATGATGTACAC | GGTAACAGCAATCGGCCCTCA | CAGTCCTATGACAACAACCTGAGTGGTGTG |
| | | | SEQ ID NO:3775 | SEQ ID NO:11787 | SEQ ID NO:19799 |
| | | AA | TGSSSNIGAGYDVH | GNSNRPS | QSYDNNLSGV |
| iPS:437210 | | | SEQ ID NO:3776 | SEQ ID NO:11788 | SEQ ID NO:19800 |
| | 21-225_227E12 | NA | TCTGGAGATAAATTGGGGGATAAATATGTTTGT | CAAGATAGCAAGCGGCCCTCA | CAGGCGTGGAACAGCAGCAATGTGGTA |
| | | | SEQ ID NO:3777 | SEQ ID NO:11789 | SEQ ID NO:19801 |
| | | AA | SGDKLGDKYVC | QDSKRPS | QAWNSSNVV |
| | | | SEQ ID NO:3778 | SEQ ID NO:11790 | SEQ ID NO:19802 |

FIGURE 49

| iPS:437214 | | NA | TCTGGAGATGCATTGCCAAA AAAATATGCTTAT | GAGGACAGCAAACGACC CTCC | AACTCAACAGACAGCAGTGG TAATCATGTGGTA |
|---|---|---|---|---|---|
| | 21-225_48B12 | | SEQ ID NO:3779 | SEQ ID NO:11791 | SEQ ID NO:19803 |
| | | AA | SGDALPKKYAY | EDSKRPS | NSTDSSGNHVV |
| | | | SEQ ID NO:3780 | SEQ ID NO:11792 | SEQ ID NO:19804 |
| iPS:437216 | | NA | CGGGCGAGTCAGGGCATTAA CAATTATTTAGCC | GCTGCATCCAGTTTGCGA AGT | CAACAGTATTATAGTTACCC ATTCACT |
| | 21-225_51D5 | | SEQ ID NO:3781 | SEQ ID NO:11793 | SEQ ID NO:19805 |
| | | AA | RASQGINNYLA | AASSLRS | QQYYSYPFT |
| | | | SEQ ID NO:3782 | SEQ ID NO:11794 | SEQ ID NO:19806 |
| iPS:437220 | | NA | CGGGCAAGTCAGGGCATTAG AAACGATTTAGGC | GGTGCATCCAGTTTGCA AAGT | CTACAGCGTGATAGTTACCC GTTCACT |
| | 21-225_55H6 | | SEQ ID NO:3783 | SEQ ID NO:11795 | SEQ ID NO:19807 |
| | | AA | RASQGIRNDLG | GASSLQS | LQRDSYPFT |
| | | | SEQ ID NO:3784 | SEQ ID NO:11796 | SEQ ID NO:19808 |
| iPS:437224 | | NA | CGGGCGAGTCAGGGCATTAG CCATTATTTAGCC | GCTGCATCCGGTTTGCAA AGT | CAACAATATCAGAATTACCC CTTCACT |
| | 21-225_56H1 | | SEQ ID NO:3785 | SEQ ID NO:11797 | SEQ ID NO:19809 |
| | | AA | RASQGISHYLA | AASGLQS | QQYQNYPFT |
| | | | SEQ ID NO:3786 | SEQ ID NO:11798 | SEQ ID NO:19810 |
| iPS:437226 | | NA | AGGTCTAGTCAAAGCCTCGT ATACAGTGATGGAAACACCT ACTTGAAT | GAGGTTTCTAACTGGGA CTCT | GTGCAAGGTACACACTGGCC TCGGACG |
| | 21-225_57C2 | | SEQ ID NO:3787 | SEQ ID NO:11799 | SEQ ID NO:19811 |
| | | AA | RSSQSLVYSDGNTYLN | EVSNWDS | VQGTHWPRT |
| | | | SEQ ID NO:3788 | SEQ ID NO:11800 | SEQ ID NO:19812 |
| iPS:437228 | | NA | AGGGCCAGTCAGAGTGTTAG CAACGACTTAGCC | GGTGCATCCACCAGGGC CACT | CAGCAGTATAGTAACTGGCC ATTCACT |
| | 21-225_60C11 | | SEQ ID NO:3789 | SEQ ID NO:11801 | SEQ ID NO:19813 |
| | | AA | RASQSVSNDLA | GASTRAT | QQYSNWPFT |

FIGURE 49

| | | | SEQ ID NO:3790 | SEQ ID NO:11802 | SEQ ID NO:19814 |
|---|---|---|---|---|---|
| iPS:437230 | | NA | CGGGCAAGTCAGAGCATTAC CAGCTATTTAAAT | ACTGCATCCAGTTTGCAA AGT | CAACAGAGTCACAGTTTCCC ATTCACT |
| | 21-225_62H10 | | SEQ ID NO:3791 | SEQ ID NO:11803 | SEQ ID NO:19815 |
| | | AA | RASQSITSYLN | TASSLQS | QQSHSFPFT |
| | | | SEQ ID NO:3792 | SEQ ID NO:11804 | SEQ ID NO:19816 |
| iPS:437232 | | NA | CGTGCGAGTCAGGGTATTAG CAGCTACTTAGCC | GCTGCATCCAGTTTGCAA AGT | CAACAGGCTAACAGTTTCCC GCTCACT |
| | 21-225_63E1 | | SEQ ID NO:3793 | SEQ ID NO:11805 | SEQ ID NO:19817 |
| | | AA | RASQGISSYLA | AASSLQS | QQANSFPLT |
| | | | SEQ ID NO:3794 | SEQ ID NO:11806 | SEQ ID NO:19818 |
| iPS:437234 | | NA | CGGGCGAGTCAGGGTATTAG CAGGTGGTTAGCC | GCTGCATCCAGTTTGCAA AGT | CAACAGGCTAACAGTTTCCC ATTCACT |
| | 21-225_64E2 | | SEQ ID NO:3795 | SEQ ID NO:11807 | SEQ ID NO:19819 |
| | | AA | RASQGISRWLA | AASSLQS | QQANSFPFT |
| | | | SEQ ID NO:3796 | SEQ ID NO:11808 | SEQ ID NO:19820 |
| iPS:437248 | | NA | AGGTCTAGTCAGAGCCTCCT GCATAGTAATGGACACAACT ATTTGGAT | TTGGGTTCTAATCGGGCC TCC | ATGCAACCTCTACAAACTCC GTTCACT |
| | 21-225_97H3 | | SEQ ID NO:3797 | SEQ ID NO:11809 | SEQ ID NO:19821 |
| | | AA | RSSQSLLHSNGHNYLD | LGSNRAS | MQPLQTPFT |
| | | | SEQ ID NO:3798 | SEQ ID NO:11810 | SEQ ID NO:19822 |
| iPS:437250 | | NA | AGGTCTAGTCAAAGCCTCGT ATACAGTGATGGAAACACCT ACTTGAAT | AAGGTTTCTAACTGGGA CTCT | ATGCAAGGTACACACTGGTC GCTCACT |
| | 21-225_148C6 | | SEQ ID NO:3799 | SEQ ID NO:11811 | SEQ ID NO:19823 |
| | | AA | RSSQSLVYSDGNTYLN | KVSNWDS | MQGTHWSLT |
| | | | SEQ ID NO:3800 | SEQ ID NO:11812 | SEQ ID NO:19824 |

FIGURE 49

| iPS:437252 | | NA | AGGTCTAGTCAAAGCCTCGT ATACAGTGATGGAAACACCT ACTTGAAT | AAGGTTTCTAACTGGGA CTCT | ATGCAAGGTACACACTGGTT GCTCACT |
|---|---|---|---|---|---|
| | 21-225_148H11 | | SEQ ID NO:3801 | SEQ ID NO:11813 | SEQ ID NO:19825 |
| | | AA | RSSQSLVYSDGNTYLN | KVSNWDS | MQGTHWLLT |
| | | | SEQ ID NO:3802 | SEQ ID NO:11814 | SEQ ID NO:19826 |
| iPS:437254 | | NA | AGGTCTAGTCAAAGCCTCGT ATACAGTGATGGAAACACCT CCTTGAAT | AAGGTTTCTAACTGGGA CTCT | ATGCAAGGTACACACTGGCC TCCCACT |
| | 21-225_149F2 | | SEQ ID NO:3803 | SEQ ID NO:11815 | SEQ ID NO:19827 |
| | | AA | RSSQSLVYSDGNTSLN | KVSNWDS | MQGTHWPPT |
| | | | SEQ ID NO:3804 | SEQ ID NO:11816 | SEQ ID NO:19828 |
| iPS:437256 | | NA | AGGTCTAGTCAAAGCCTCGT ATACAGTGATGGAAACACCT CCTTGAAT | AAGGTTTCTAACTGGGA CTAT | ATGCAAGGTACACACTGGCC TCCCACT |
| | 21-225_150F11 | | SEQ ID NO:3805 | SEQ ID NO:11817 | SEQ ID NO:19829 |
| | | AA | RSSQSLVYSDGNTSLN | KVSNWDY | MQGTHWPPT |
| | | | SEQ ID NO:3806 | SEQ ID NO:11818 | SEQ ID NO:19830 |
| iPS:437258 | | NA | CGGGCGAGTCAGGGCATTAG CAATTATTTAGCC | GCTGCATCCAGTTTGCAA AGT | CAACAGTATAATAGTTACCC GCTCAGT |
| | 21-225_153F9 | | SEQ ID NO:3807 | SEQ ID NO:11819 | SEQ ID NO:19831 |
| | | AA | RASQGISNYLA | AASSLQS | QQYNSYPLS |
| | | | SEQ ID NO:3808 | SEQ ID NO:11820 | SEQ ID NO:19832 |
| iPS:437260 | | NA | CGGGCGAGTCAGGACATTAG CAATTATTTAGCC | GCTGCATCCAGTTTGCAA AGT | CAACAGTGTGATAGTTTCCC TCTCACT |
| | 21-225_170D1 | | SEQ ID NO:3809 | SEQ ID NO:11821 | SEQ ID NO:19833 |
| | | AA | RASQDISNYLA | AASSLQS | QQCDSFPLT |
| | | | SEQ ID NO:3810 | SEQ ID NO:11822 | SEQ ID NO:19834 |
| iPS:437262 | | NA | CGGGCAAGTCAGGGCATTAG AAATGATTTAGGC | GTTGCATCCGGTTTGCAA AGT | CTACAGCACAATAGTTACCC TCCGTGGACG |

FIGURE 49

| | 21-225_170E4 | | SEQ ID NO:3811 | SEQ ID NO:11823 | SEQ ID NO:19835 |
|---|---|---|---|---|---|
| | | AA | RASQGIRNDLG | VASGLQS | LQHNSYPPWT |
| | | | SEQ ID NO:3812 | SEQ ID NO:11824 | SEQ ID NO:19836 |
| iPS:437264 | | NA | CGGGCGAGTCAGGACATTAG CAATTATTTAGCC | TCTGCATCCAGTTTGCAA AGT | CAACAATCTGATAGTTACCC TCTCACT |
| | 21-225_171H12 | | SEQ ID NO:3813 | SEQ ID NO:11825 | SEQ ID NO:19837 |
| | | AA | RASQDISNYLA | SASSLQS | QQSDSYPLT |
| | | | SEQ ID NO:3814 | SEQ ID NO:11826 | SEQ ID NO:19838 |
| iPS:437266 | | NA | CGGGCGAGTCAGGACATTAG CAATTATTTAGCC | TCTGCATCCAGTTTGCAA AGT | CAACAATCTGATAGTTACCC TCTCACT |
| | 21-225_177A5 | | SEQ ID NO:3815 | SEQ ID NO:11827 | SEQ ID NO:19839 |
| | | AA | RASQDISNYLA | SASSLQS | QQSDSYPLT |
| | | | SEQ ID NO:3816 | SEQ ID NO:11828 | SEQ ID NO:19840 |
| iPS:437268 | | NA | AGGTCTAGTCAAAGCCTCGT ATACAGTGATGGAAACACCT ACTTGAAT | AAGGTTTCTAACTGGGA CTCT | ATGCAAGGTACACACTGGCC TCTCACT |
| | 21-225_177D2 | | SEQ ID NO:3817 | SEQ ID NO:11829 | SEQ ID NO:19841 |
| | | AA | RSSQSLVYSDGNTYLN | KVSNWDS | MQGTHWPLT |
| | | | SEQ ID NO:3818 | SEQ ID NO:11830 | SEQ ID NO:19842 |
| iPS:437270 | | NA | CGGGCGAGTCAGGACATTAG CAATTATTTAGCC | TCTGCATCCAGTTTGCAA AGT | CAACAATCTAATAGTTACCC TCTCACT |
| | 21-225_178H4 | | SEQ ID NO:3819 | SEQ ID NO:11831 | SEQ ID NO:19843 |
| | | AA | RASQDISNYLA | SASSLQS | QQSNSYPLT |
| | | | SEQ ID NO:3820 | SEQ ID NO:11832 | SEQ ID NO:19844 |
| iPS:437274 | | NA | CGGGCGAGTCAGGGCATTAG CAATTATTTAGCC | GCTGCATCCAGTTTGCAA AGT | CAACATTATCTTAATTACCCT CTCACC |
| | 21-225_196D4 | | SEQ ID NO:3821 | SEQ ID NO:11833 | SEQ ID NO:19845 |
| | | AA | RASQGISNYLA | AASSLQS | QHYLNYPLT |
| | | | SEQ ID NO:3822 | SEQ ID NO:11834 | SEQ ID NO:19846 |

FIGURE 49

| iPS:437280 | | NA | CGGGCAAGTCAGGACATTAG AAATGATTTAGGC | AGAGCATCCAGTTTGCA AAGT | CTACAGCATAATAGTTACCC GTGGACG |
|---|---|---|---|---|---|
| | 21-225_203C10 | | SEQ ID NO:3823 | SEQ ID NO:11835 | SEQ ID NO:19847 |
| | | AA | RASQDIRNDLG | RASSLQS | LQHNSYPWT |
| | | | SEQ ID NO:3824 | SEQ ID NO:11836 | SEQ ID NO:19848 |
| iPS:437282 | | NA | CGGGCAAGTCAGAGGTTTAG TAACTATTTAAAT | ACTGCATCCAGTTTGCAA AGT | CAACAGAGTTACAGTATTCC GCTCACT |
| | 21-225_207C9 | | SEQ ID NO:3825 | SEQ ID NO:11837 | SEQ ID NO:19849 |
| | | AA | RASQRFSNYLN | TASSLQS | QQSYSIPLT |
| | | | SEQ ID NO:3826 | SEQ ID NO:11838 | SEQ ID NO:19850 |
| iPS:437286 | | NA | CGGGCAAGTCAGGGCATTAG ACATGATTTAGGC | GCTGCATCCAGTTTGCAA AGT | CTACAGCATTATAGTTTCCCT CGGACG |
| | 21-225_208F1 | | SEQ ID NO:3827 | SEQ ID NO:11839 | SEQ ID NO:19851 |
| | | AA | RASQGIRHDLG | AASSLQS | LQHYSFPRT |
| | | | SEQ ID NO:3828 | SEQ ID NO:11840 | SEQ ID NO:19852 |
| iPS:437290 | | NA | CGGGCAAGTCAGGGCATTAG ACATGATTTAGGC | GCTGCATCCAGTTCGCA AAGT | GTACAGCATTATAGTTTCCC TCGGACG |
| | 21-225_210G6 | | SEQ ID NO:3829 | SEQ ID NO:11841 | SEQ ID NO:19853 |
| | | AA | RASQGIRHDLG | AASSSQS | VQHYSFPRT |
| | | | SEQ ID NO:3830 | SEQ ID NO:11842 | SEQ ID NO:19854 |
| iPS:437294 | | NA | AGGTCTAGTCAAAGCCTCGT ATACAGTGATGGAAACACCT ACTTGAAT | AAGGTTTCTAACTGGGA CTCT | ATGCAAGGTGCACACTGGTT CACC |
| | 21-225_216D5 | | SEQ ID NO:3831 | SEQ ID NO:11843 | SEQ ID NO:19855 |
| | | AA | RSSQSLVYSDGNTYLN | KVSNWDS | MQGAHWFT |
| | | | SEQ ID NO:3832 | SEQ ID NO:11844 | SEQ ID NO:19856 |
| iPS:437302 | | NA | CAGGCGAGTCAGGACATTTT CAACTATTTAAAT | GATGCATCCACTTTGGA AACA | CAACAGTATGATAATCTCCC GATCACC |
| | 21-225_225B11 | | SEQ ID NO:3833 | SEQ ID NO:11845 | SEQ ID NO:19857 |
| | | AA | QASQDIFNYLN | DASTLET | QQYDNLPIT |

FIGURE 49

| | | | SEQ ID NO:3834 | SEQ ID NO:11846 | SEQ ID NO:19858 |
|---|---|---|---|---|---|
| iPS:437320 | 21-225_75A1 | NA | AGGTCTAGTCAGAGCCTCCT GCATAGTAATGGACACAACT ATTTGGAT | TTGGGTTCTAATCGGGCC TCC | ATGCAACCTCTACAAACTCC GTTCACT |
| | | | SEQ ID NO:3835 | SEQ ID NO:11847 | SEQ ID NO:19859 |
| | | AA | RSSQSLLHSNGHNYLD | LGSNRAS | MQPLQTPFT |
| | | | SEQ ID NO:3836 | SEQ ID NO:11848 | SEQ ID NO:19860 |
| iPS:437322 | 21-225_75B1 | NA | AGGGCCAGTCAGAGTGTTAG CAGCAGCTACTTAGTC | GGTGCATCCACCAGGGC CACT | CAGCAGTATGGTTGCTACC GCTCACT |
| | | | SEQ ID NO:3837 | SEQ ID NO:11849 | SEQ ID NO:19861 |
| | | AA | RASQSVSSSYLV | GASTRAT | QQYGCSPLT |
| | | | SEQ ID NO:3838 | SEQ ID NO:11850 | SEQ ID NO:19862 |
| iPS:437324 | 21-225_75C2 | NA | AGGGCCAGTCAGAGTGTTTA CAGCAGCTACTTAGCC | GGTGCATCCAGCCGGGC CACT | CAGCACTATGATAACTCACC GTGGACG |
| | | | SEQ ID NO:3839 | SEQ ID NO:11851 | SEQ ID NO:19863 |
| | | AA | RASQSVYSSYLA | GASSRAT | QHYDNSPWT |
| | | | SEQ ID NO:3840 | SEQ ID NO:11852 | SEQ ID NO:19864 |
| iPS:437326 | 21-225_75C10 | NA | CGGGCGAGTCAGGGCATTAG CATCTGGTTAGCC | GCTGCATCCAGTTTGCAA AGT | CAACAGGCTAAAAGTTTCCC GCTCACT |
| | | | SEQ ID NO:3841 | SEQ ID NO:11853 | SEQ ID NO:19865 |
| | | AA | RASQGISIWLA | AASSLQS | QQAKSFPLT |
| | | | SEQ ID NO:3842 | SEQ ID NO:11854 | SEQ ID NO:19866 |
| iPS:437328 | 21-225_75D3 | NA | AGGGCCAGTCAGAGTGTTTA CAGCAGCTACTTAGCC | GGTGCATCCAGCCGGTC CACT | CAGCACTATGATAACTCACC GTGGACG |
| | | | SEQ ID NO:3843 | SEQ ID NO:11855 | SEQ ID NO:19867 |
| | | AA | RASQSVYSSYLA | GASSRST | QHYDNSPWT |
| | | | SEQ ID NO:3844 | SEQ ID NO:11856 | SEQ ID NO:19868 |
| iPS:437332 | 21-225_75F3 | NA | AGGGCCAGTCAGAGTGTTTA CAGCAGCTACTTAGCC | GGTGCATCCAGCCGGGC CACT | CAGCACTATGATAACTCACC GTGGACG |
| | | | SEQ ID NO:3845 | SEQ ID NO:11857 | SEQ ID NO:19869 |

FIGURE 49

| | | | | | |
|---|---|---|---|---|---|
| | | AA | RASQSVYSSYLA | GASSRAT | QHYDNSPWT |
| | | | SEQ ID NO:3846 | SEQ ID NO:11858 | SEQ ID NO:19870 |
| iPS:437334 | | NA | AGGGCCAGTCAGAGTGTTAGCAGAAATTTAGCC | GGTGCATCCATCAGGGCCACT | CAGCAGTATAATAACTGGCCTCCGCTCACT |
| | 21-225_75F11 | | SEQ ID NO:3847 | SEQ ID NO:11859 | SEQ ID NO:19871 |
| | | AA | RASQSVSRNLA | GASIRAT | QQYNNWPPLT |
| | | | SEQ ID NO:3848 | SEQ ID NO:11860 | SEQ ID NO:19872 |
| iPS:437340 | | NA | AGGGCCAGTCCGAGTGTTGACAGCAGCTACTTAGCC | GGTGCATCCAGCAGGGCCCCT | CAGCAGTATGAAAGTTCACCGTGGACG |
| | 21-225_75G9 | | SEQ ID NO:3849 | SEQ ID NO:11861 | SEQ ID NO:19873 |
| | | AA | RASPSVDSSYLA | GASSRAP | QQYESSPWT |
| | | | SEQ ID NO:3850 | SEQ ID NO:11862 | SEQ ID NO:19874 |
| iPS:437344 | | NA | AGGGCCAGTCAGAGTGTTTACAGCAGCTACTTAGCC | GGTGCATCCAGCCGGGCCACT | CAGCACTATGATAACTCACCGTGGACG |
| | 21-225_75G12 | | SEQ ID NO:3851 | SEQ ID NO:11863 | SEQ ID NO:19875 |
| | | AA | RASQSVYSSYLA | GASSRAT | QHYDNSPWT |
| | | | SEQ ID NO:3852 | SEQ ID NO:11864 | SEQ ID NO:19876 |
| iPS:437346 | | NA | CGGGCAAGTCAGGGCATAAGAAATGATTTAGGC | GATGCATCCAGTTTGCAAAGT | ATACAGCATAGTAATTACCCGCTCACT |
| | 21-225_75H7 | | SEQ ID NO:3853 | SEQ ID NO:11865 | SEQ ID NO:19877 |
| | | AA | RASQGIRNDLG | DASSLQS | IQHSNYPLT |
| | | | SEQ ID NO:3854 | SEQ ID NO:11866 | SEQ ID NO:19878 |
| iPS:437350 | | NA | AGGGCCAGTCAGAGTGTTTACAGCAGCTACTTAGCC | GGTGCATCCAGCCGGTCCACT | CAGCACTCTGATAACTCACCGTGGACG |
| | 21-225_74A3 | | SEQ ID NO:3855 | SEQ ID NO:11867 | SEQ ID NO:19879 |
| | | AA | RASQSVYSSYLA | GASSRST | QHSDNSPWT |
| | | | SEQ ID NO:3856 | SEQ ID NO:11868 | SEQ ID NO:19880 |
| iPS:437356 | 21 225 74B1 | NA | AAGTCCAGCCAGAGTGTTTTACACAGGTCCAACAATTACAACTACTTAGCG | TGGGCATCTACCCGGGAATCC | CAGCAATATTATAGTACTCCTCCGACG |

912

FIGURE 49

| | | | SEQ ID NO:3857 | SEQ ID NO:11869 | SEQ ID NO:19881 |
|---|---|---|---|---|---|
| | 21-225_74B1 | AA | KSSQSVLHRSNNYNYLA | WASTRES | QQYYSTPPT |
| iPS:437361 | | NA | SEQ ID NO:3858<br>AAGTCCAGCCAGAGTATTTT<br>ACACAGCTCCAACAATTACA<br>ATTACTTAGCT | SEQ ID NO:11870<br>TGGGCATCTACCCGGGA<br>ATCC | SEQ ID NO:19882<br>CAGCAATATTATAGTACTCC<br>GTGGACG |
| | 21-225_74C1 | AA | SEQ ID NO:3859<br>KSSQSILHSSNNYNYLA | SEQ ID NO:11871<br>WASTRES | SEQ ID NO:19883<br>QQYYSTPWT |
| iPS:437363 | | NA | SEQ ID NO:3860<br>AAGTCCAGCCAGAGTGTTTT<br>ATACAGCTCCAACAATGCGA<br>ACTACTTAGCT | SEQ ID NO:11872<br>TGGGCATCTACCCGGGA<br>ATCC | SEQ ID NO:19884<br>CAGCAATATTATAGTACTCC<br>GTGCAGT |
| | 21-225_74C10 | AA | SEQ ID NO:3861<br>KSSQSVLYSSNNANYLA | SEQ ID NO:11873<br>WASTRES | SEQ ID NO:19885<br>QQYYSTPCS |
| iPS:437369 | | NA | SEQ ID NO:3862<br>AGGGCCAGTCAGAGTGTTTA<br>CAGCAGCTACTTAGCC | SEQ ID NO:11874<br>GGTGCATCCAGCCGGGC<br>CACT | SEQ ID NO:19886<br>CAGCATTATGATAACTCACC<br>GTGGACG |
| | 21-225_74D6 | AA | SEQ ID NO:3863<br>RASQSVYSSYLA | SEQ ID NO:11875<br>GASSRAT | SEQ ID NO:19887<br>QHYDNSPWT |
| iPS:437371 | | NA | SEQ ID NO:3864<br>AGGTCTAGTCAGAGCCTCGT<br>GCATAGTAGTGGATACAACT<br>ATTTGGAT | SEQ ID NO:11876<br>TTGGGTTCTAATCGGGCC<br>TCC | SEQ ID NO:19888<br>ATGCAAGCTCTACACCCTCC<br>TCTCACT |
| | 21-225_74D8 | AA | SEQ ID NO:3865<br>RSSQSLVHSSGYNYLD | SEQ ID NO:11877<br>LGSNRAS | SEQ ID NO:19889<br>MQALHPPLT |
| iPS:437377 | | NA | SEQ ID NO:3866<br>AGGGCCAGTCAGAGTGTTAG<br>CAGCAGCTACTTAGTC | SEQ ID NO:11878<br>GGTGCATCCACCAGGGC<br>CACT | SEQ ID NO:19890<br>CAGCAGTATGGTTGCTCACC<br>GCTCACT |
| | 21-225_74G9 | AA | SEQ ID NO:3867<br>RASQSVSSSYLV | SEQ ID NO:11879<br>GASTRAT | SEQ ID NO:19891<br>QQYGCSPLT |

FIGURE 49

| | | | SEQ ID NO:3868 | SEQ ID NO:11880 | SEQ ID NO:19892 |
|---|---|---|---|---|---|
| iPS:437379 | 21-225_74H2 | NA | AAGTCCAGCCAGAGTGTTTT ACACAGCTCCAACAATAAGA ACTACTTAACT | TGGGCATCTACTCGGGA ATCC | CAGCAATATTATAGTATTCC TCCGACG |
| | | | SEQ ID NO:3869 | SEQ ID NO:11881 | SEQ ID NO:19893 |
| | | AA | KSSQSVLHSSNNKNYLT | WASTRES | QQYYSIPPT |
| | | | SEQ ID NO:3870 | SEQ ID NO:11882 | SEQ ID NO:19894 |
| iPS:437383 | 21-225_74H8 | NA | AGGGCCAGTCAGAGTTTTAG CAGCAGCTACTTAGCC | GGTGCATCCAGCAGGGC CACT | CAGCAGTATGGTAGCTCAAG GACG |
| | | | SEQ ID NO:3871 | SEQ ID NO:11883 | SEQ ID NO:19895 |
| | | AA | RASQSFSSSYLA | GASSRAT | QQYGSSRT |
| | | | SEQ ID NO:3872 | SEQ ID NO:11884 | SEQ ID NO:19896 |
| iPS:438664 | 21-225_216G1 | NA | CGGGCGAGTCAGGGCATTAG CAGTTATTTAGCC | GCTGCATCCAGTTTGCAA AGT | CTACGGTATGATACTTACCC TCTCACT |
| | | | SEQ ID NO:3873 | SEQ ID NO:11885 | SEQ ID NO:19897 |
| | | AA | RASQGISSYLA | AASSLQS | LRYDTYPLT |
| | | | SEQ ID NO:3874 | SEQ ID NO:11886 | SEQ ID NO:19898 |
| iPS:441468 | 21-225_25A4.001.001 | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | | | SEQ ID NO:3875 | SEQ ID NO:11887 | SEQ ID NO:19899 |
| | | AA | KSSQSVLYSSHNNNYLA | WASTRES | QQYYSTPPT |
| | | | SEQ ID NO:3876 | SEQ ID NO:11888 | SEQ ID NO:19900 |
| iPS:441475 | 21-225_25A4.001.002 | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | | | SEQ ID NO:3877 | SEQ ID NO:11889 | SEQ ID NO:19901 |
| | | AA | KSSQSVLYSSHNNNYLA | WASTRES | QQYYSTPPT |
| | | | SEQ ID NO:3878 | SEQ ID NO:11890 | SEQ ID NO:19902 |

FIGURE 49

| iPS:441482 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
|---|---|---|---|---|---|
| | 21-225_25A4.001.003 | | SEQ ID NO:3879 | SEQ ID NO:11891 | SEQ ID NO:19903 |
| | | AA | KSSQSVLYSSHNNNYLA | WASTRES | QQYYSTPPT |
| | | | SEQ ID NO:3880 | SEQ ID NO:11892 | SEQ ID NO:19904 |
| iPS:441489 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | 21-225_25A4.001.004 | | SEQ ID NO:3881 | SEQ ID NO:11893 | SEQ ID NO:19905 |
| | | AA | KSSQSVLYSSHNNNYLA | WASTRES | QQYYSTPPT |
| | | | SEQ ID NO:3882 | SEQ ID NO:11894 | SEQ ID NO:19906 |
| iPS:441496 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | 21-225_25A4.001.005 | | SEQ ID NO:3883 | SEQ ID NO:11895 | SEQ ID NO:19907 |
| | | AA | KSSQSVLYSSHNNNYLA | WASTRES | QQYYSTPPT |
| | | | SEQ ID NO:3884 | SEQ ID NO:11896 | SEQ ID NO:19908 |
| iPS:441505 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | 21-225_25A4.001.006 | | SEQ ID NO:3885 | SEQ ID NO:11897 | SEQ ID NO:19909 |
| | | AA | KSSQSVLYSSHNNNYLA | WASTRES | QQYYSTPPT |
| | | | SEQ ID NO:3886 | SEQ ID NO:11898 | SEQ ID NO:19910 |
| iPS:441512 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | 21-225_25A4.001.007 | | SEQ ID NO:3887 | SEQ ID NO:11899 | SEQ ID NO:19911 |
| | | AA | KSSQSVLYSSHNNNYLA | WASTRES | QQYYSTPPT |
| | | | SEQ ID NO:3888 | SEQ ID NO:11900 | SEQ ID NO:19912 |

FIGURE 49

| iPS:441519 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
|---|---|---|---|---|---|
| | 21- 225_25A4.001.008 | | SEQ ID NO:3889 | SEQ ID NO:11901 | SEQ ID NO:19913 |
| | | AA | KSSQSVLYSSHNNNYLA | WASTRES | QQYYSTPPT |
| | | | SEQ ID NO:3890 | SEQ ID NO:11902 | SEQ ID NO:19914 |
| iPS:441554 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | 21- 225_25A4.001.013 | | SEQ ID NO:3891 | SEQ ID NO:11903 | SEQ ID NO:19915 |
| | | AA | KSSQSVLYSSHNNNYLA | WASTRES | QQYYSTPPT |
| | | | SEQ ID NO:3892 | SEQ ID NO:11904 | SEQ ID NO:19916 |
| iPS:441595 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | 21- 225_25A4.001.019 | | SEQ ID NO:3893 | SEQ ID NO:11905 | SEQ ID NO:19917 |
| | | AA | KSSQSVLYSSHNNNYLA | WASTRES | QQYYSTPPT |
| | | | SEQ ID NO:3894 | SEQ ID NO:11906 | SEQ ID NO:19918 |
| iPS:441604 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | 21- 225_25A4.001.020 | | SEQ ID NO:3895 | SEQ ID NO:11907 | SEQ ID NO:19919 |
| | | AA | KSSQSVLYSSHNNNYLA | WASTRES | QQYYSTPPT |
| | | | SEQ ID NO:3896 | SEQ ID NO:11908 | SEQ ID NO:19920 |
| iPS:441613 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | 21- 225_25A4.001.021 | | SEQ ID NO:3897 | SEQ ID NO:11909 | SEQ ID NO:19921 |
| | | AA | KSSQSVLYSSHNNNYLA | WASTRES | QQYYSTPPT |
| | | | SEQ ID NO:3898 | SEQ ID NO:11910 | SEQ ID NO:19922 |

FIGURE 49

| iPS:441841 | | NA | AAGTCCAGCCAGAGTATTTT ACACAGCTCCAACAATAACA ACTACTTAGCT | TGGGCATCTACCCGGGA ATCC | CAGCAATATTATAGTACTCC AGTCACT |
|---|---|---|---|---|---|
| | 21-225_4A2.001.001 | | SEQ ID NO:3899 | SEQ ID NO:11911 | SEQ ID NO:19923 |
| | | AA | KSSQSILHSSNNNNYLA | WASTRES | QQYYSTPVT |
| | | | SEQ ID NO:3900 | SEQ ID NO:11912 | SEQ ID NO:19924 |
| iPS:441847 | | NA | AAGTCCAGCCAGAGTATTTT ACACAGCTCCAACAATAACA ACTACTTAGCT | TGGGCATCTACCCGGGA ATCC | CAGCAATATTATAATGCTCC AGTCACT |
| | 21-225_4A2.001.002 | | SEQ ID NO:3901 | SEQ ID NO:11913 | SEQ ID NO:19925 |
| | | AA | KSSQSILHSSNNNNYLA | WASTRES | QQYYNAPVT |
| | | | SEQ ID NO:3902 | SEQ ID NO:11914 | SEQ ID NO:19926 |
| iPS:441853 | | NA | AAGTCCAGCCAGAGTATTTT ACACAGCTCCAACAATAACA ACTACTTAGCT | TGGGCATCTACCCGGGA ATCC | CAGCAATATTATCAAACTCC AGTCACT |
| | 21-225_4A2.001.003 | | SEQ ID NO:3903 | SEQ ID NO:11915 | SEQ ID NO:19927 |
| | | AA | KSSQSILHSSNNNNYLA | WASTRES | QQYYQTPVT |
| | | | SEQ ID NO:3904 | SEQ ID NO:11916 | SEQ ID NO:19928 |
| iPS:441859 | | NA | AAGTCCAGCCAGAGTATTTT ACACAGCTCCAACAATAACA ACTACTTAGCT | TGGGCATCTACCCGGGA ATCC | CAGCAATATTATAATACTCC AGTCACT |
| | 21-225_4A2.001.004 | | SEQ ID NO:3905 | SEQ ID NO:11917 | SEQ ID NO:19929 |
| | | AA | KSSQSILHSSNNNNYLA | WASTRES | QQYYNTPVT |
| | | | SEQ ID NO:3906 | SEQ ID NO:11918 | SEQ ID NO:19930 |
| iPS:441866 | | NA | AAGTCCAGCCAGAGTATTTT ACACAGCTCCAACAATAACA ACTACTTAGCT | TGGGCATCTACCCGGGA ATCC | CAGCAATATTATAATACTCC AGTCACT |
| | 21-225_4A2.001.005 | | SEQ ID NO:3907 | SEQ ID NO:11919 | SEQ ID NO:19931 |
| | | AA | KSSQSILHSSNNNNYLA | WASTRES | QQYYNTPVT |
| | | | SEQ ID NO:3908 | SEQ ID NO:11920 | SEQ ID NO:19932 |

FIGURE 49

| iPS:441873 | | NA | AAGTCCAGCCAGAGTATTTT ACACAGCTCCAACAATAACA ACTACTTAGCT | TGGGCATCTACCCGGGA ATCC | CAGCAATATTATAATACTCC AGTCACT |
|---|---|---|---|---|---|
| | 21-225_4A2.001.006 | | SEQ ID NO:3909 | SEQ ID NO:11921 | SEQ ID NO:19933 |
| | | AA | KSSQSILHSSNNNNYLA | WASTRES | QQYYNTPVT |
| | | | SEQ ID NO:3910 | SEQ ID NO:11922 | SEQ ID NO:19934 |
| iPS:441880 | | NA | AAGTCCAGCCAGAGTATTTT ACACAGCTCCAACAATAACA ACTACTTAGCT | TGGGCATCTACCCGGGA ATCC | CAGCAATATTATAGTACTCC AGTCACT |
| | 21-225_4A2.001.007 | | SEQ ID NO:3911 | SEQ ID NO:11923 | SEQ ID NO:19935 |
| | | AA | KSSQSILHSSNNNNYLA | WASTRES | QQYYSTPVT |
| | | | SEQ ID NO:3912 | SEQ ID NO:11924 | SEQ ID NO:19936 |
| iPS:441884 | | NA | AAGTCCAGCCAGAGTATTTT ACACAGCTCCAACAATAACA ACTACTTAGCT | TGGGCATCTACCCGGGA ATCC | CAGCAATATTATAGTACTCC AGTCACT |
| | 21-225_4A2.001.008 | | SEQ ID NO:3913 | SEQ ID NO:11925 | SEQ ID NO:19937 |
| | | AA | KSSQSILHSSNNNNYLA | WASTRES | QQYYSTPVT |
| | | | SEQ ID NO:3914 | SEQ ID NO:11926 | SEQ ID NO:19938 |
| iPS:441888 | | NA | AAGTCCAGCCAGAGTATTTT ACACAGCTCCAACAATAACA ACTACTTAGCT | TGGGCATCTACCCGGGA ATCC | CAGCAATATTATAATGCTCC AGTCACT |
| | 21-225_4A2.001.009 | | SEQ ID NO:3915 | SEQ ID NO:11927 | SEQ ID NO:19939 |
| | | AA | KSSQSILHSSNNNNYLA | WASTRES | QQYYNAPVT |
| | | | SEQ ID NO:3916 | SEQ ID NO:11928 | SEQ ID NO:19940 |
| iPS:441892 | | NA | AAGTCCAGCCAGAGTATTTT ACACAGCTCCAACAATAACA ACTACTTAGCT | TGGGCATCTACCCGGGA ATCC | CAGCAATATTATCAAACTCC AGTCACT |
| | 21-225_4A2.001.010 | | SEQ ID NO:3917 | SEQ ID NO:11929 | SEQ ID NO:19941 |
| | | AA | KSSQSILHSSNNNNYLA | WASTRES | QQYYQTPVT |
| | | | SEQ ID NO:3918 | SEQ ID NO:11930 | SEQ ID NO:19942 |

FIGURE 49

| iPS:441896 | | NA | AAGTCCAGCCAGAGTATTTT ACACAGCTCCAACAATAACA ACTACTTAGCT | TGGGCATCTACCCGGGA ATCC | CAGCAATATTATCAAACTCC AGTCACT |
|---|---|---|---|---|---|
| | 21-225_4A2.001.011 | | SEQ ID NO:3919 | SEQ ID NO:11931 | SEQ ID NO:19943 |
| | | AA | KSSQSILHSSNNNNYLA | WASTRES | QQYYQTPVT |
| | | | SEQ ID NO:3920 | SEQ ID NO:11932 | SEQ ID NO:19944 |
| iPS:441900 | | NA | AAGTCCAGCCAGAGTATTTT ACACAGCTCCAACAATAACA ACTACTTAGCT | TGGGCATCTACCCGGGA ATCC | CAGCAATATTATCAAACTCC AGTCACT |
| | 21-225_4A2.001.012 | | SEQ ID NO:3921 | SEQ ID NO:11933 | SEQ ID NO:19945 |
| | | AA | KSSQSILHSSNNNNYLA | WASTRES | QQYYQTPVT |
| | | | SEQ ID NO:3922 | SEQ ID NO:11934 | SEQ ID NO:19946 |
| iPS:441955 | | NA | AAGTCCAGCCAGAGTATTTT ACACAGCTCCAACAATAACA ACTACTTAGCT | TGGGCATCTACCCGGGA ATCC | CAGCAATATTATAGTACTCC AGTCACT |
| | 21-225_4A2.001.022 | | SEQ ID NO:3923 | SEQ ID NO:11935 | SEQ ID NO:19947 |
| | | AA | KSSQSILHSSNNNNYLA | WASTRES | QQYYSTPVT |
| | | | SEQ ID NO:3924 | SEQ ID NO:11936 | SEQ ID NO:19948 |
| iPS:441962 | | NA | AAGTCCAGCCAGAGTATTTT ACACAGCTCCAACAATAACA ACTACTTAGCT | TGGGCATCTACCCGGGA ATCC | CAGCAATATTATAGTACTCC AGTCACT |
| | 21-225_4A2.001.023 | | SEQ ID NO:3925 | SEQ ID NO:11937 | SEQ ID NO:19949 |
| | | AA | KSSQSILHSSNNNNYLA | WASTRES | QQYYSTPVT |
| | | | SEQ ID NO:3926 | SEQ ID NO:11938 | SEQ ID NO:19950 |
| iPS:441971 | | NA | AAGTCCAGCCAGAGTATTTT ACACAGCTCCAACAATAACA ACTACTTAGCT | TGGGCATCTACCCGGGA ATCC | CAGCAATATTATAGTACTCC AGTCACT |
| | 21-225_4A2.001.024 | | SEQ ID NO:3927 | SEQ ID NO:11939 | SEQ ID NO:19951 |
| | | AA | KSSQSILHSSNNNNYLA | WASTRES | QQYYSTPVT |
| | | | SEQ ID NO:3928 | SEQ ID NO:11940 | SEQ ID NO:19952 |

FIGURE 49

| iPS:441999 | | NA | AAGTCCAGCCAGAGTATTTT ACACAGCTCCAACAATAACA ACTACTTAGCT | TGGGCATCTACCCGGGA ATCC | CAGCAATATTATAGTACTCC AGTCACT |
|---|---|---|---|---|---|
| | 21-225_4A2.001.028 | | SEQ ID NO:3929 | SEQ ID NO:11941 | SEQ ID NO:19953 |
| | | AA | KSSQSILHSSNNNNYLA | WASTRES | QQYYSTPVT |
| | | | SEQ ID NO:3930 | SEQ ID NO:11942 | SEQ ID NO:19954 |
| iPS:442006 | | NA | AAGTCCAGCCAGAGTATTTT ACACAGCTCCAACAATAACA ACTACTTAGCT | TGGGCATCTACCCGGGA ATCC | CAGCAATATTATAGTACTCC AGTCACT |
| | 21-225_4A2.001.029 | | SEQ ID NO:3931 | SEQ ID NO:11943 | SEQ ID NO:19955 |
| | | AA | KSSQSILHSSNNNNYLA | WASTRES | QQYYSTPVT |
| | | | SEQ ID NO:3932 | SEQ ID NO:11944 | SEQ ID NO:19956 |
| iPS:442020 | | NA | AAGTCCAGCCAGAGTATTTT ACACAGCTCCAACAATAACA ACTACTTAGCT | TGGGCATCTACCCGGGA ATCC | CAGCAATATTATAATACTCC AGTCACT |
| | 21-225_4A2.001.031 | | SEQ ID NO:3933 | SEQ ID NO:11945 | SEQ ID NO:19957 |
| | | AA | KSSQSILHSSNNNNYLA | WASTRES | QQYYNTPVT |
| | | | SEQ ID NO:3934 | SEQ ID NO:11946 | SEQ ID NO:19958 |
| iPS:442050 | | NA | CGGGCGAGTCAGGGTATTAG CAGGTGGTTAGCC | GGTGCATCCAGTTTGCA AAGT | CAACAGGCTAACAGTTTCCC ATTCACT |
| | 21-225_4H6.004 | | SEQ ID NO:3935 | SEQ ID NO:11947 | SEQ ID NO:19959 |
| | | AA | RASQGISRWLA | GASSLQS | QQANSFPFT |
| | | | SEQ ID NO:3936 | SEQ ID NO:11948 | SEQ ID NO:19960 |
| iPS:442059 | | NA | CGGGCGAGTCAGGGTATTAG CAGGTGGTTAGCC | GGTGCATCCAGTTTGCA AAGT | CAACAGGCTAACAGTTTCCC ATTCACT |
| | 21-225_4H6.005 | | SEQ ID NO:3937 | SEQ ID NO:11949 | SEQ ID NO:19961 |
| | | AA | RASQGISRWLA | GASSLQS | QQANSFPFT |
| | | | SEQ ID NO:3938 | SEQ ID NO:11950 | SEQ ID NO:19962 |
| iPS:442065 | | NA | CGGGCGAGTCAGGGTATTAG CAGGTGGTTAGCC | GGTGCATCCAGTTTGCA AAGT | CAACAGGCTAACAGTTTCCC ATTCACT |

FIGURE 49

| | 21-225_4H6.006 | | SEQ ID NO:3939 | SEQ ID NO:11951 | SEQ ID NO:19963 |
|---|---|---|---|---|---|
| | | AA | RASQGISRWLA | GASSLQS | QQANSFPFT |
| | | | SEQ ID NO:3940 | SEQ ID NO:11952 | SEQ ID NO:19964 |
| iPS:442071 | | NA | CGGGCGAGTCAGGGTATTAG CAGGTGGTTAGCC | GGTGCATCCAGTTTGCA AAGT | CAACAGGCTAACAGTTTCCC ATTCACT |
| | 21-225_4H6.007 | | SEQ ID NO:3941 | SEQ ID NO:11953 | SEQ ID NO:19965 |
| | | AA | RASQGISRWLA | GASSLQS | QQANSFPFT |
| | | | SEQ ID NO:3942 | SEQ ID NO:11954 | SEQ ID NO:19966 |
| iPS:442078 | | NA | CGGGCGAGTCAGGGTATTAG CAGGTGGTTAGCC | GGTGCATCCAGTTTGCA AAGT | CAACAGGCTAACAGTTTCCC ATTCACT |
| | 21-225_4H6.008 | | SEQ ID NO:3943 | SEQ ID NO:11955 | SEQ ID NO:19967 |
| | | AA | RASQGISRWLA | GASSLQS | QQANSFPFT |
| | | | SEQ ID NO:3944 | SEQ ID NO:11956 | SEQ ID NO:19968 |
| iPS:442085 | | NA | CGGGCGAGTCAGGGTATTAG CAGGTGGTTAGCC | GGTGCATCCAGTTTGCA AAGT | CAACAGGCTAACAGTTTCCC ATTCACT |
| | 21-225_4H6.009 | | SEQ ID NO:3945 | SEQ ID NO:11957 | SEQ ID NO:19969 |
| | | AA | RASQGISRWLA | GASSLQS | QQANSFPFT |
| | | | SEQ ID NO:3946 | SEQ ID NO:11958 | SEQ ID NO:19970 |
| iPS:442089 | | NA | CGGGCGAGTCAGGGTATTAG CAGGTGGTTAGCC | GGTGCATCCAGTTTGCA AAGT | CAACAGGCTAACAGTTTCCC ATTCACT |
| | 21-225_4H6.010 | | SEQ ID NO:3947 | SEQ ID NO:11959 | SEQ ID NO:19971 |
| | | AA | RASQGISRWLA | GASSLQS | QQANSFPFT |
| | | | SEQ ID NO:3948 | SEQ ID NO:11960 | SEQ ID NO:19972 |
| iPS:442093 | | NA | CGGGCGAGTCAGGGTATTAG CAGGTGGTTAGCC | GGTGCATCCAGTTTGCA AAGT | CAACAGGCTAACAGTTTCCC ATTCACT |
| | 21-225_4H6.011 | | SEQ ID NO:3949 | SEQ ID NO:11961 | SEQ ID NO:19973 |
| | | AA | RASQGISRWLA | GASSLQS | QQANSFPFT |
| | | | SEQ ID NO:3950 | SEQ ID NO:11962 | SEQ ID NO:19974 |
| iPS:442115 | | NA | CGGGCAAGTCAGGGCATTAG AAATGATTTAGGC | GCTGCATCCAGTTTGCAA AGT | CTACAGCATTATAGTTACCC TCGGACG |

FIGURE 49

| | 21-225_5E5.003 | | SEQ ID NO:3951 | SEQ ID NO:11963 | SEQ ID NO:19975 |
|---|---|---|---|---|---|
| | | AA | RASQGIRNDLG | AASSLQS | LQHYSYPRT |
| | | | SEQ ID NO:3952 | SEQ ID NO:11964 | SEQ ID NO:19976 |
| iPS:442122 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.004 | | SEQ ID NO:3953 | SEQ ID NO:11965 | SEQ ID NO:19977 |
| | | AA | RASQGIRNDLG | AASSLQS | LQHYSYPRT |
| | | | SEQ ID NO:3954 | SEQ ID NO:11966 | SEQ ID NO:19978 |
| iPS:442129 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.005 | | SEQ ID NO:3955 | SEQ ID NO:11967 | SEQ ID NO:19979 |
| | | AA | RASQGIRNDLG | AASSLQS | LQHYSYPRT |
| | | | SEQ ID NO:3956 | SEQ ID NO:11968 | SEQ ID NO:19980 |
| iPS:442136 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.006 | | SEQ ID NO:3957 | SEQ ID NO:11969 | SEQ ID NO:19981 |
| | | AA | RASQGIRNDLG | AASSLQS | LQHYSYPRT |
| | | | SEQ ID NO:3958 | SEQ ID NO:11970 | SEQ ID NO:19982 |
| iPS:442171 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.011 | | SEQ ID NO:3959 | SEQ ID NO:11971 | SEQ ID NO:19983 |
| | | AA | RASQGIRNDLG | AASSLQS | LQHYSYPRT |
| | | | SEQ ID NO:3960 | SEQ ID NO:11972 | SEQ ID NO:19984 |
| iPS:442178 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.012 | | SEQ ID NO:3961 | SEQ ID NO:11973 | SEQ ID NO:19985 |
| | | AA | RASQGIRNDLG | AASSLQS | LQHYSYPRT |
| | | | SEQ ID NO:3962 | SEQ ID NO:11974 | SEQ ID NO:19986 |
| iPS:442199 | | NA | CGGGCAAGTCAGGGCATTAG AAATGATTTAGGC | GCTGCATCCAGTTTGCAA AGT | CTACAGCATTATAGTTACCC TCGGACG |

FIGURE 49

| | 21-225_5E5.015 | | SEQ ID NO:3963 | SEQ ID NO:11975 | SEQ ID NO:19987 |
|---|---|---|---|---|---|
| | | AA | RASQGIRNDLG | AASSLQS | LQHYSYPRT |
| | | | SEQ ID NO:3964 | SEQ ID NO:11976 | SEQ ID NO:19988 |
| iPS:442206 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.016 | | SEQ ID NO:3965 | SEQ ID NO:11977 | SEQ ID NO:19989 |
| | | AA | RASQGIRNDLG | AASSLQS | LQHYSYPRT |
| | | | SEQ ID NO:3966 | SEQ ID NO:11978 | SEQ ID NO:19990 |
| iPS:442213 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.017 | | SEQ ID NO:3967 | SEQ ID NO:11979 | SEQ ID NO:19991 |
| | | AA | RASQGIRNDLG | AASSLQS | LQHYSYPRT |
| | | | SEQ ID NO:3968 | SEQ ID NO:11980 | SEQ ID NO:19992 |
| iPS:442220 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.018 | | SEQ ID NO:3969 | SEQ ID NO:11981 | SEQ ID NO:19993 |
| | | AA | RASQGIRNDLG | AASSLQS | LQHYSYPRT |
| | | | SEQ ID NO:3970 | SEQ ID NO:11982 | SEQ ID NO:19994 |
| iPS:442227 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.019 | | SEQ ID NO:3971 | SEQ ID NO:11983 | SEQ ID NO:19995 |
| | | AA | RASQGIRNDLG | AASSLQS | LQHYSYPRT |
| | | | SEQ ID NO:3972 | SEQ ID NO:11984 | SEQ ID NO:19996 |
| iPS:442255 | | NA | CGGGCAAGTCAGGGCATTAG AAATGATTTAGGC | GCTGCATCCAGTTTGCAA AGT | CTACAGCATTATAGTTACCC TCGGACG |
| | 21-225_5E5.023 | | SEQ ID NO:3973 | SEQ ID NO:11985 | SEQ ID NO:19997 |
| | | AA | RASQGIRNDLG | AASSLQS | LQHYSYPRT |
| | | | SEQ ID NO:3974 | SEQ ID NO:11986 | SEQ ID NO:19998 |
| iPS:442262 | | NA | CGGGCAAGTCAGGGCATTAG AAATGATTTAGGC | GCTGCATCCAGTTTGCAA AGT | CTACAGCATTATAGTTACCC TCGGACG |

FIGURE 49

| | 21-225_5E5.024 | | SEQ ID NO:3975 | SEQ ID NO:11987 | SEQ ID NO:19999 |
|---|---|---|---|---|---|
| | | AA | RASQGIRNDLG | AASSLQS | LQHYSYPRT |
| iPS:442269 | | NA | SEQ ID NO:3976 | SEQ ID NO:11988 | SEQ ID NO:20000 |
| | | | CGGGCAAGTCAGGGCATTAG AAATGATTTAGGC | GCTGCATCCAGTTTGCAA AGT | CTACAGCATTATAGTTACCC TCGGACG |
| | 21-225_5E5.025 | | SEQ ID NO:3977 | SEQ ID NO:11989 | SEQ ID NO:20001 |
| | | AA | RASQGIRNDLG | AASSLQS | LQHYSYPRT |
| iPS:442311 | | NA | SEQ ID NO:3978 | SEQ ID NO:11990 | SEQ ID NO:20002 |
| | | | CGGGCAAGTCAAAACATTAT CAGCTATTTAAAT | ACTGCATCCAGTTTGCAA AGT | CAACAGACTTACAGTACCCC GCTCACT |
| | 21-225_7E11.001.001 | | SEQ ID NO:3979 | SEQ ID NO:11991 | SEQ ID NO:20003 |
| | | AA | RASQNIISYLN | TASSLQS | QQTYSTPLT |
| iPS:442317 | | NA | SEQ ID NO:3980 | SEQ ID NO:11992 | SEQ ID NO:20004 |
| | | | CGGGCAAGTCAAAACATTAT CAGCTATTTAAAT | ACTGCATCCAGTTTGCAA AGT | CAACAGACTTACAGTACCCC GCTCACT |
| | 21-225_7E11.001.002 | | SEQ ID NO:3981 | SEQ ID NO:11993 | SEQ ID NO:20005 |
| | | AA | RASQNIISYLN | TASSLQS | QQTYSTPLT |
| iPS:442323 | | NA | SEQ ID NO:3982 | SEQ ID NO:11994 | SEQ ID NO:20006 |
| | | | CGGGCAAGTCAAAACATTAT CAGCTATTTAAAT | ACTGCATCCAGTTTGCAA AGT | CAACAGACTTACAGTACCCC GCTCACT |
| | 21-225_7E11.001.003 | | SEQ ID NO:3983 | SEQ ID NO:11995 | SEQ ID NO:20007 |
| | | AA | RASQNIISYLN | TASSLQS | QQTYSTPLT |
| iPS:442330 | | NA | SEQ ID NO:3984 | SEQ ID NO:11996 | SEQ ID NO:20008 |
| | | | CGGGCAAGTCAAAACATTAT CAGCTATTTAAAT | ACTGCATCCAGTTTGCAA AGT | CAACAGACTTACAGTACCCC GCTCACT |
| | 21-225_7E11.001.004 | | SEQ ID NO:3985 | SEQ ID NO:11997 | SEQ ID NO:20009 |
| | | AA | RASQNIISYLN | TASSLQS | QQTYSTPLT |
| iPS:442337 | | NA | SEQ ID NO:3986 | SEQ ID NO:11998 | SEQ ID NO:20010 |
| | | | CGGGCAAGTCAAAACATTAT CAGCTATTTAAAT | ACTGCATCCAGTTTGCAA AGT | CAACAGACTTACAGTACCCC GCTCACT |

FIGURE 49

| | 21-225_7E11.001.005 | | SEQ ID NO:3987 | SEQ ID NO:11999 | SEQ ID NO:20011 |
|---|---|---|---|---|---|
| | | AA | RASQNIISYLN | TASSLQS | QQTYSTPLT |
| | | | SEQ ID NO:3988 | SEQ ID NO:12000 | SEQ ID NO:20012 |
| iPS:442344 | 21-225_7E11.001.006 | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:3989 | SEQ ID NO:12001 | SEQ ID NO:20013 |
| | | AA | RASQNIISYLN | TASSLQS | QQTYSTPLT |
| | | | SEQ ID NO:3990 | SEQ ID NO:12002 | SEQ ID NO:20014 |
| iPS:442351 | 21-225_7E11.001.007 | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:3991 | SEQ ID NO:12003 | SEQ ID NO:20015 |
| | | AA | RASQNIISYLN | TASSLQS | QQTYSTPLT |
| | | | SEQ ID NO:3992 | SEQ ID NO:12004 | SEQ ID NO:20016 |
| iPS:442358 | 21-225_7E11.001.008 | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:3993 | SEQ ID NO:12005 | SEQ ID NO:20017 |
| | | AA | RASQNIISYLN | TASSLQS | QQTYSTPLT |
| | | | SEQ ID NO:3994 | SEQ ID NO:12006 | SEQ ID NO:20018 |
| iPS:442365 | 21-225_7E11.001.009 | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:3995 | SEQ ID NO:12007 | SEQ ID NO:20019 |
| | | AA | RASQNIISYLN | TASSLQS | QQTYSTPLT |
| | | | SEQ ID NO:3996 | SEQ ID NO:12008 | SEQ ID NO:20020 |
| iPS:442372 | 21-225_7E11.001.010 | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:3997 | SEQ ID NO:12009 | SEQ ID NO:20021 |
| | | AA | RASQNIISYLN | TASSLQS | QQTYSTPLT |
| | | | SEQ ID NO:3998 | SEQ ID NO:12010 | SEQ ID NO:20022 |
| iPS:442379 | | NA | CGGGCAAGTCAAAACATTAT CAGCTATTTAAAT | ACTGCATCCAGTTTGCAA AGT | CAACAGACTTACAGTACCCC GCTCACT |

FIGURE 49

| | 21-225_7E11.001.011 | | SEQ ID NO:3999 | SEQ ID NO:12011 | SEQ ID NO:20023 |
|---|---|---|---|---|---|
| | | AA | RASQNIISYLN | TASSLQS | QQTYSTPLT |
| iPS:442386 | | NA | SEQ ID NO:4000 CGGGCAAGTCAAAACATTAT CAGCTATTTAAAT | SEQ ID NO:12012 ACTGCATCCAGTTTGCAA AGT | SEQ ID NO:20024 CAACAGACTTACAGTACCCC GCTCACT |
| | 21-225_7E11.001.012 | | SEQ ID NO:4001 | SEQ ID NO:12013 | SEQ ID NO:20025 |
| | | AA | RASQNIISYLN | TASSLQS | QQTYSTPLT |
| iPS:442390 | | NA | SEQ ID NO:4002 CGGGCAAGTCAAAACATTAT CAGCTATTTAAAT | SEQ ID NO:12014 ACTGCATCCAGTTTGCAA AGT | SEQ ID NO:20026 CAACAGACTTACAGTACCCC GCTCACT |
| | 21-225_7E11.001.013 | | SEQ ID NO:4003 | SEQ ID NO:12015 | SEQ ID NO:20027 |
| | | AA | RASQNIISYLN | TASSLQS | QQTYSTPLT |
| iPS:442394 | | NA | SEQ ID NO:4004 CGGGCAAGTCAAAACATTAT CAGCTATTTAAAT | SEQ ID NO:12016 ACTGCATCCAGTTTGCAA AGT | SEQ ID NO:20028 CAACAGACTTACAGTACCCC GCTCACT |
| | 21-225_7E11.001.014 | | SEQ ID NO:4005 | SEQ ID NO:12017 | SEQ ID NO:20029 |
| | | AA | RASQNIISYLN | TASSLQS | QQTYSTPLT |
| iPS:442398 | | NA | SEQ ID NO:4006 CGGGCAAGTCAAAACATTAT CAGCTATTTAAAT | SEQ ID NO:12018 ACTGCATCCAGTTTGCAA AGT | SEQ ID NO:20030 CAACAGACTTACAGTACCCC GCTCACT |
| | 21-225_7E11.001.015 | | SEQ ID NO:4007 | SEQ ID NO:12019 | SEQ ID NO:20031 |
| | | AA | RASQNIISYLN | TASSLQS | QQTYSTPLT |
| iPS:442402 | | NA | SEQ ID NO:4008 CGGGCAAGTCAAAACATTAT CAGCTATTTAAAT | SEQ ID NO:12020 ACTGCATCCAGTTTGCAA AGT | SEQ ID NO:20032 CAACAGACTTACAGTACCCC GCTCACT |
| | 21-225_7E11.001.016 | | SEQ ID NO:4009 | SEQ ID NO:12021 | SEQ ID NO:20033 |
| | | AA | RASQNIISYLN | TASSLQS | QQTYSTPLT |
| iPS:442406 | | NA | SEQ ID NO:4010 CGGGCAAGTCAAAACATTAT CAGCTATTTAAAT | SEQ ID NO:12022 ACTGCATCCAGTTTGCAA AGT | SEQ ID NO:20034 CAACAGACTTACAGTACCCC GCTCACT |

FIGURE 49

| | 21-225_7E11.001.017 | | SEQ ID NO:4011 | SEQ ID NO:12023 | SEQ ID NO:20035 |
|---|---|---|---|---|---|
| | | AA | RASQNIISYLN | TASSLQS | QQTYSTPLT |
| | | | SEQ ID NO:4012 | SEQ ID NO:12024 | SEQ ID NO:20036 |
| iPS:442410 | | NA | CGGGCAAGTCAAAACATTAT CAGCTATTTAAAT | ACTGCATCCAGTTTGCAA AGT | CAACAGACTTACAGTACCCC GCTCACT |
| | 21-225_7E11.001.018 | | SEQ ID NO:4013 | SEQ ID NO:12025 | SEQ ID NO:20037 |
| | | AA | RASQNIISYLN | TASSLQS | QQTYSTPLT |
| | | | SEQ ID NO:4014 | SEQ ID NO:12026 | SEQ ID NO:20038 |
| iPS:442417 | | NA | CGGGCAAGTCAAAACATTAT CAGCTATTTAAAT | ACTGCATCCAGTTTGCAA AGT | CAACAGACTTACAGTACCCC GCTCACT |
| | 21-225_7E11.001.019 | | SEQ ID NO:4015 | SEQ ID NO:12027 | SEQ ID NO:20039 |
| | | AA | RASQNIISYLN | TASSLQS | QQTYSTPLT |
| | | | SEQ ID NO:4016 | SEQ ID NO:12028 | SEQ ID NO:20040 |
| iPS:442431 | | NA | CGGGCAAGTCAAAACATTAT CAGCTATTTAAAT | ACTGCATCCAGTTTGCAA AGT | CAACAGACTTACAGTACCCC GCTCACT |
| | 21-225_7E11.001.021 | | SEQ ID NO:4017 | SEQ ID NO:12029 | SEQ ID NO:20041 |
| | | AA | RASQNIISYLN | TASSLQS | QQTYSTPLT |
| | | | SEQ ID NO:4018 | SEQ ID NO:12030 | SEQ ID NO:20042 |
| iPS:442438 | | NA | CGGGCAAGTCAAAACATTAT CAGCTATTTAAAT | ACTGCATCCAGTTTGCAA AGT | CAACAGACTTACAGTACCCC GCTCACT |
| | 21-225_7E11.001.022 | | SEQ ID NO:4019 | SEQ ID NO:12031 | SEQ ID NO:20043 |
| | | AA | RASQNIISYLN | TASSLQS | QQTYSTPLT |
| | | | SEQ ID NO:4020 | SEQ ID NO:12032 | SEQ ID NO:20044 |
| iPS:442568 | | NA | AGGGCCAGTCAGAGTGTGAT CAGCAGCTACTTAGCC | GGTGTATCTAGTTGGGCC ACT | CAACAATATGGTAGGTCACC ATTCAAT |
| | 21-225_149D8 | | SEQ ID NO:4021 | SEQ ID NO:12033 | SEQ ID NO:20045 |
| | | AA | RASQSVISSYLA | GVSSWAT | QQYGRSPFN |
| | | | SEQ ID NO:4022 | SEQ ID NO:12034 | SEQ ID NO:20046 |

FIGURE 49

| iPS:443003 | | NA | ACTGGGAGCAGCTCCAACAT CGGGGCAGGTTATGATGTAC AC | GGTAACAGCAATCGGCC CTCA | CAGTCCTATGACAACAGCCT GAGTGGTTCGGTA |
|---|---|---|---|---|---|
| | 21-225_43F11_LC2 | | SEQ ID NO:4023 | SEQ ID NO:12035 | SEQ ID NO:20047 |
| | | AA | TGSSSNIGAGYDVH | GNSNRPS | QSYDNSLSGSV |
| | | | SEQ ID NO:4024 | SEQ ID NO:12036 | SEQ ID NO:20048 |
| iPS:443005 | | NA | AGGTCTAGTCAAAGCCTCGT ATACAGTGATGGAAACACCT ACTTGAAT | AAGGTTTCTAACTGGGA CTCT | ATGCAAGGTACACACTGGCC GCTCACT |
| | 21-225_43F11_LC1 | | SEQ ID NO:4025 | SEQ ID NO:12037 | SEQ ID NO:20049 |
| | | AA | RSSQSLVYSDGNTYLN | KVSNWDS | MQGTHWPLT |
| | | | SEQ ID NO:4026 | SEQ ID NO:12038 | SEQ ID NO:20050 |
| iPS:443006 | | NA | AAGTCCAGCCAGAGTGTTTT ATACAGCTCCCACAATAACA ACTACTTAGCT | TGGGCATCTACCCGGGA ATCC | CAGCAGTATTATAGTACTCC TCCGACG |
| | 21-225_25A4.001.029 | | SEQ ID NO:4027 | SEQ ID NO:12039 | SEQ ID NO:20051 |
| | | AA | KSSQSVLYSSHNNNYLA | WASTRES | QQYYSTPPT |
| | | | SEQ ID NO:4028 | SEQ ID NO:12040 | SEQ ID NO:20052 |
| iPS:443016 | | NA | CGGGCGAGTCAGGGTATTAG CAGGTGGTTAGCC | GGTGCATCCAGTTTGCA AAGT | CAACAGGCTAACAGTTTCCC ATTCACT |
| | 21-225_4H6.014 | | SEQ ID NO:4029 | SEQ ID NO:12041 | SEQ ID NO:20053 |
| | | AA | RASQGISRWLA | GASSLQS | QQANSFPFT |
| | | | SEQ ID NO:4030 | SEQ ID NO:12042 | SEQ ID NO:20054 |
| iPS:443027 | | NA | CGGGCAAGTCAAAACATTAT CAGCTATTTAAAT | ACTGCATCCAGTTTGCAA AGT | CAACAGACTTACAGTACCCC GCTCACT |
| | 21-225_7E11.001.023 | | SEQ ID NO:4031 | SEQ ID NO:12043 | SEQ ID NO:20055 |
| | | AA | RASQNIISYLN | TASSLQS | QQTYSTPLT |
| | | | SEQ ID NO:4032 | SEQ ID NO:12044 | SEQ ID NO:20056 |

FIGURE 49

| iPS:446086 | | NA | AAGTCCAGACAGAGTGTTTT ATACAGCTCCAACAATTACA ACTACTTAACT | TGGGCATCTACCCGGGA ATCC | CAGCAATATTATAGTTCTCC TCCTACT |
|---|---|---|---|---|---|
| | 21-225_94D8 | | SEQ ID NO:4033 | SEQ ID NO:12045 | SEQ ID NO:20057 |
| | | AA | KSRQSVLYSSNNYNYLT | WASTRES | QQYYSSPPT |
| | | | SEQ ID NO:4034 | SEQ ID NO:12046 | SEQ ID NO:20058 |
| iPS:446094 | | NA | AAGTCCAGCCAGACTGTCTT ACACAGCTCCAACAATTATA ACTACTTAGCT | TGGACATCTACCCGGGA ATCC | CACCAATATCTTAGTAGTCC TCTGACG |
| | 21-225_77E1 | | SEQ ID NO:4035 | SEQ ID NO:12047 | SEQ ID NO:20059 |
| | | AA | KSSQTVLHSSNNYNYLA | WTSTRES | HQYLSSPLT |
| | | | SEQ ID NO:4036 | SEQ ID NO:12048 | SEQ ID NO:20060 |
| iPS:448904 | | NA | AGGGCCAGTCAGAGTGTTAG CATCAACTTAGCC | GGTGCATCCACCAGGGC CACT | CAGCAGTATAATACCTGGCC TCTCACT |
| | 21-225_65C12 | | SEQ ID NO:4037 | SEQ ID NO:12049 | SEQ ID NO:20061 |
| | | AA | RASQSVSINLA | GASTRAT | QQYNTWPLT |
| | | | SEQ ID NO:4038 | SEQ ID NO:12050 | SEQ ID NO:20062 |
| iPS:448906 | | NA | CGGGCAAGTCAGAGCATTAC CAGCTATTTAAAT | ACTGCATCCAGTTTGCAA AGT | CAACAGAGTCACAGTTTCCC ATTCACT |
| | 21-225_72G9 | | SEQ ID NO:4039 | SEQ ID NO:12051 | SEQ ID NO:20063 |
| | | AA | RASQSITSYLN | TASSLQS | QQSHSFPFT |
| | | | SEQ ID NO:4040 | SEQ ID NO:12052 | SEQ ID NO:20064 |
| iPS:448908 | | NA | TCTGGAGATAAATTGGGGGA TAAATATGCTTGC | CAAGATAGCAAGCGGCC CTCA | CAGGCGCGGAACAGCCGCAG AGGGGTA |
| | 21-225_50G9 | | SEQ ID NO:4041 | SEQ ID NO:12053 | SEQ ID NO:20065 |
| | | AA | SGDKLGDKYAC | QDSKRPS | QARNSRRGV |
| | | | SEQ ID NO:4042 | SEQ ID NO:12054 | SEQ ID NO:20066 |
| iPS:451102 | | NA | TCTGGAGATAAATTGGGGGA TAAATATGCTTCC | CAAGATAGTAAGCGGCC CTCA | CAGGCGTGGGACAACAGAAC TATGGTA |
| | 21-225_45F6 | | SEQ ID NO:4043 | SEQ ID NO:12055 | SEQ ID NO:20067 |

FIGURE 49

| | | AA | SGDKLGDKYAS | QDSKRPS | QAWDNRTMV |
|---|---|---|---|---|---|
| | | | SEQ ID NO:4044 | SEQ ID NO:12056 | SEQ ID NO:20068 |
| iPS:451104 | | NA | TCTGGAAGCAGCTCCAACAT CGGAAGTAATATTGTGACC | AGTAATGATCAGCGGCC CTCA | ACAGCATGGGATGACAGCCT GAATGGTTGGGTG |
| | 21-225_49C5 | | SEQ ID NO:4045 | SEQ ID NO:12057 | SEQ ID NO:20069 |
| | | AA | SGSSSNIGSNIVT | SNDQRPS | TAWDDSLNGWV |
| | | | SEQ ID NO:4046 | SEQ ID NO:12058 | SEQ ID NO:20070 |
| iPS:451106 | | NA | TCTGGAAGCAACTCCAACAT CGGAAGTAATATTGTAACC | AGTAATGATCAGCGGCC CTCA | GCAGCATGGGATGACAGCCT GAATGGTTGGGTG |
| | 21-225_49D10 | | SEQ ID NO:4047 | SEQ ID NO:12059 | SEQ ID NO:20071 |
| | | AA | SGSNSNIGSNIVT | SNDQRPS | AAWDDSLNGWV |
| | | | SEQ ID NO:4048 | SEQ ID NO:12060 | SEQ ID NO:20072 |
| iPS:451108 | | NA | TCTGGAAGCTGCTCCAACAT CGGAAGTAATATTGTGACC | AGTAATGATCAGCGGCC CTCA | ACAGCATGGGATGACAGCCT GAATGATTGGGTG |
| | 21-225_53E8 | | SEQ ID NO:4049 | SEQ ID NO:12061 | SEQ ID NO:20073 |
| | | AA | SGSCSNIGSNIVT | SNDQRPS | TAWDDSLNDWV |
| | | | SEQ ID NO:4050 | SEQ ID NO:12062 | SEQ ID NO:20074 |
| iPS:451110 | | NA | TCAGGAGATAAATCGGGGA ATAAATATGTTTCC | CAAGATAACAGGCGGCC GTCA | CAGGCGTGGGACAGCACCCC TGTGATA |
| | 21-225_74C9 | | SEQ ID NO:4051 | SEQ ID NO:12063 | SEQ ID NO:20075 |
| | | AA | SGDKSGNKYVS | QDNRRPS | QAWDSTPVI |
| | | | SEQ ID NO:4052 | SEQ ID NO:12064 | SEQ ID NO:20076 |
| iPS:451112 | | NA | TCTGGAGATAAATTGGGGAA TAAATATGCTTGC | CAAGATCGCAAGCGGCC CTCA | CAGGCGTGGGACAGCAGCAC TGTGGTA |
| | 21-225_53D10 | | SEQ ID NO:4053 | SEQ ID NO:12065 | SEQ ID NO:20077 |
| | | AA | SGDKLGNKYAC | QDRKRPS | QAWDSSTVV |
| | | | SEQ ID NO:4054 | SEQ ID NO:12066 | SEQ ID NO:20078 |

FIGURE 49

| iPS:451114 | | NA | CGGGCAAGTCAGGACATTAG AAAGGATTTAGGC | GCTGCATCCAGTTTGCAA AGT | CTACAGCATCATAGTTATCC TCGGACG |
|---|---|---|---|---|---|
| | 21-225_159A3 | | SEQ ID NO:4055 | SEQ ID NO:12067 | SEQ ID NO:20079 |
| | | AA | RASQDIRKDLG | AASSLQS | LQHHSYPRT |
| | | | SEQ ID NO:4056 | SEQ ID NO:12068 | SEQ ID NO:20080 |
| iPS:451116 | | NA | AAGTCCAGCCAGAGTGTTTT ATACAGCTCCAACAATAAGA ACTACTTAACT | TGGGCATCTACCCGGGA ATCC | CAGCAATATTTTAGTACTCC GTGGACG |
| | 21-225_164A4 | | SEQ ID NO:4057 | SEQ ID NO:12069 | SEQ ID NO:20081 |
| | | AA | KSSQSVLYSSNNKNYLT | WASTRES | QQYFSTPWT |
| | | | SEQ ID NO:4058 | SEQ ID NO:12070 | SEQ ID NO:20082 |
| iPS:451118 | | NA | AGGGCCAGTCAGAGTGTTCG CAGTAACTTAGCC | GGTGCATCCACCAGGGC CACT | CAGCAGTCTTTTACCTGGCT CCGGACG |
| | 21-225_191C8 | | SEQ ID NO:4059 | SEQ ID NO:12071 | SEQ ID NO:20083 |
| | | AA | RASQSVRSNLA | GASTRAT | QQSFTWLRT |
| | | | SEQ ID NO:4060 | SEQ ID NO:12072 | SEQ ID NO:20084 |
| iPS:451120 | | NA | CGGGCGAGTCAGGGCATTAG AAATTATTTAGCC | GCTGCATCCAGTTTGCAA AGT | CAACATTATCTTACTTACCC GCTCACT |
| | 21-225_197D3 | | SEQ ID NO:4061 | SEQ ID NO:12073 | SEQ ID NO:20085 |
| | | AA | RASQGIRNYLA | AASSLQS | QHYLTYPLT |
| | | | SEQ ID NO:4062 | SEQ ID NO:12074 | SEQ ID NO:20086 |
| iPS:451122 | | NA | AGGGCCAGTCAGAGTGTTAA CAGCAACTATTTAGCC | GGGGCATCCAGCAGGGC CACT | CAGCAGTATGAGATCTCACC GTGGACG |
| | 21-225_200A1 | | SEQ ID NO:4063 | SEQ ID NO:12075 | SEQ ID NO:20087 |
| | | AA | RASQSVNSNYLA | GASSRAT | QQYEISPWT |
| | | | SEQ ID NO:4064 | SEQ ID NO:12076 | SEQ ID NO:20088 |
| iPS:451124 | | NA | AAGTCCAGTCAGAATATTTT ATCCAGCTCCAACAATAAGA ACTACTTAACT | TGGACATCTACCCGGGA ATCC | CAGCAATATTTTAGTGTTCCT CTGACG |
| | 21-225_74F6 | | SEQ ID NO:4065 | SEQ ID NO:12077 | SEQ ID NO:20089 |

FIGURE 49

| | | | | | |
|---|---|---|---|---|---|
| | | AA | KSSQNILSSSNNKNYLT | WTSTRES | QQYFSVPLT |
| | | | SEQ ID NO:4066 | SEQ ID NO:12078 | SEQ ID NO:20090 |
| iPS:451127 | 21-225_164A7 | NA | AAGTCCAGCCAGAGTGTTTTACACAGCTCCAACAATAACAACTACTTAGCT | TGGACATCTACCCGGGAATCC | CAGCAATATTATAGTATTCCTCTGACG |
| | | | SEQ ID NO:4067 | SEQ ID NO:12079 | SEQ ID NO:20091 |
| | | AA | KSSQSVLHSSNNNNYLA | WTSTRES | QQYYSIPLT |
| | | | SEQ ID NO:4068 | SEQ ID NO:12080 | SEQ ID NO:20092 |
| iPS:451129 | 21-225_94D2 | NA | AAGTCCAGCCAGAGTGTTTTACACAGCTCCAACAATAAGAACTACTTAACT | TGGGCATCTACTCGGGAATCC | CAGCAATATCATAGTATTCCTCCGACG |
| | | | SEQ ID NO:4069 | SEQ ID NO:12081 | SEQ ID NO:20093 |
| | | AA | KSSQSVLHSSNNKNYLT | WASTRES | QQYHSIPPT |
| | | | SEQ ID NO:4070 | SEQ ID NO:12082 | SEQ ID NO:20094 |
| iPS:451131 | 21-225_160A7 | NA | AAGTCCAGCCAGAGTGTTTTATCCAACTCCCACAATAACAACTACTTAGCT | TGGGCATCTACCCGGGAATCC | CAGCAATATTATAGTACTCCGTGCAGT |
| | | | SEQ ID NO:4071 | SEQ ID NO:12083 | SEQ ID NO:20095 |
| | | AA | KSSQSVLSNSHNNNYLA | WASTRES | QQYYSTPCS |
| | | | SEQ ID NO:4072 | SEQ ID NO:12084 | SEQ ID NO:20096 |
| iPS:451133 | 21-225_95H4 | NA | AAGTCCAGCCAGAGTGTTTTATTCAGCTCCAACAATTATAATTACTTAGCT | TGGGCATCTACCCGGGAATCC | CAGCAATATCATAGTTCTCCTCTGACG |
| | | | SEQ ID NO:4073 | SEQ ID NO:12085 | SEQ ID NO:20097 |
| | | AA | KSSQSVLFSSNNYNYLA | WASTRES | QQYHSSPLT |
| | | | SEQ ID NO:4074 | SEQ ID NO:12086 | SEQ ID NO:20098 |
| iPS:437240 | 21-225_84H12 | NA | CGGGCAAGTCAGGGCATTAGAAATGATTTAGGC | GCTGCATCCAGTTTGCAAAGT | TTACAGCATAATGATTACCCATTCACT |
| | | | SEQ ID NO:4075 | SEQ ID NO:12087 | SEQ ID NO:20099 |
| | | AA | RASQGIRNDLG | AASSLQS | LQHNDYPFT |

FIGURE 49

| | | | SEQ ID NO:4076 | SEQ ID NO:12088 | SEQ ID NO:20100 |
|---|---|---|---|---|---|
| iPS:434577 | | NA | CGGGCAAGTCAGGGCATTAGAAATGATTTAGGC | GCTGCATCCAGTTTGCAAAGT | CTACAGCATAATGATTACCCATTCACT |
| | 21-225_75C11 | | SEQ ID NO:4077 | SEQ ID NO:12089 | SEQ ID NO:20101 |
| | | AA | RASQGIRNDLG | AASSLQS | LQHNDYPFT |
| | | | SEQ ID NO:4078 | SEQ ID NO:12090 | SEQ ID NO:20102 |
| iPS:435477 | | NA | CGGGCGAGTCAGTTTATTAGCAGCTGGTTAGCC | ACTGCATCCAGTTTGCAAAGT | CAACAGGCTAACAGTTTCCCGTGGACG |
| | 21-225_154E8 | | SEQ ID NO:4079 | SEQ ID NO:12091 | SEQ ID NO:20103 |
| | | AA | RASQFISSWLA | TASSLQS | QQANSFPWT |
| | | | SEQ ID NO:4080 | SEQ ID NO:12092 | SEQ ID NO:20104 |
| iPS:434553 | | NA | CGGGCAAGTCAGGGCATTAGAAATGATTTAGGC | GCTGCATCCAGATTGCAAGT | CTACAGCATAATGATTACCCATTCACT |
| | 21-225_76H12 | | SEQ ID NO:4081 | SEQ ID NO:12093 | SEQ ID NO:20105 |
| | | AA | RASQGIRNDLG | AASRLQS | LQHNDYPFT |
| | | | SEQ ID NO:4082 | SEQ ID NO:12094 | SEQ ID NO:20106 |
| iPS:434927 | | NA | CGGGCAAGTCAGGGCATTAGAAATGATTTAGGC | GCTGCATCCAGTTTGCAAAGT | CTACAGCATAATGATTACCCATTCACT |
| | 21-225_86E5 | | SEQ ID NO:4083 | SEQ ID NO:12095 | SEQ ID NO:20107 |
| | | AA | RASQGIRNDLG | AASSLQS | LQHNDYPFT |
| | | | SEQ ID NO:4084 | SEQ ID NO:12096 | SEQ ID NO:20108 |
| iPS:435385 | | NA | CGGGCGAGTCAGTTTATTAGCAGCTGGTTAGCC | GCTGCATCCAGTTTGCAAAGT | CAACAGGCTAACAGTTTCCCGTGGACG |
| | 21-225_149G7 | | SEQ ID NO:4085 | SEQ ID NO:12097 | SEQ ID NO:20109 |
| | | AA | RASQFISSWLA | AASSLQS | QQANSFPWT |
| | | | SEQ ID NO:4086 | SEQ ID NO:12098 | SEQ ID NO:20110 |

Table 2B

FIGURE 49

Standard
IgG Antibody
VH CDRs

| iPS# | Ab | Type | CDR1 | CDR2 | CDR3 |
|---|---|---|---|---|---|
| iPS:451135 | 21-225_64A11 | NA | AACTATGGCATGCAC | GTTATATCATATGTTGGAAGTAATAAATACTATGCAGACTCCGTGAAGGGC | AGAGGAGCAGTGGCTCCGTACTACGGTATGGACGTC |
| | | | SEQ ID NO:4087 | SEQ ID NO:12099 | SEQ ID NO:20111 |
| | | AA | NYGMH | VISYVGSNKYYADSVKG | RGAVAPYYGMDV |
| | | | SEQ ID NO:4088 | SEQ ID NO:12100 | SEQ ID NO:20112 |
| iPS:451141 | 21-225_164B11 | NA | AATTATGATATCAAC | TGGATGACCCCTAACAGTGGTAACACAGGCTATGCACAGAAGTTCCAGGGC | AGCAGTGGCTGGTACATGTTTGACTAC |
| | | | SEQ ID NO:4089 | SEQ ID NO:12101 | SEQ ID NO:20113 |
| | | AA | NYDIN | WMTPNSGNTGYAQKFQG | SSGWYMFDY |
| | | | SEQ ID NO:4090 | SEQ ID NO:12102 | SEQ ID NO:20114 |
| iPS:451137 | 21-225_74A7 | NA | AATTATGATATCAAC | TGGATGAACCCTAACAGTGGTAACACAGGCTATGCACAGAAGTTCCAGGGC | TCCAGTGGCTGGAACTGGTTCGACCCC |
| | | | SEQ ID NO:4091 | SEQ ID NO:12103 | SEQ ID NO:20115 |
| | | AA | NYDIN | WMNPNSGNTGYAQKFQG | SSGWNWFDP |
| | | | SEQ ID NO:4092 | SEQ ID NO:12104 | SEQ ID NO:20116 |
| iPS:451139 | 21-225_71A6 | NA | AACTATGGCATGCAC | GTTATATCATATGATGGAAGTAATGAATACTATGCAGACTCCGTGAAGGGC | GATCACAGATATGGGGTTCGGGGAGGCTTTGACTAC |
| | | | SEQ ID NO:4093 | SEQ ID NO:12105 | SEQ ID NO:20117 |
| | | AA | NYGMH | VISYDGSNEYYADSVKG | DHRYGVRGGFDY |

FIGURE 49

| | | | SEQ ID NO:4094 | SEQ ID NO:12106 | SEQ ID NO:20118 |
|---|---|---|---|---|---|
| iPS:451143 | 21-225_66H11 | NA | ACCTATGGTATCAGC | TGGATCAGCGCTTACAA TGGTAACACAAACTATG CACAGAAGCTCCAGGGC | GGGGAAGCAGTGGCTGTCTT CGACCCC |
| | | | SEQ ID NO:4095 | SEQ ID NO:12107 | SEQ ID NO:20119 |
| | | AA | TYGIS | WISAYNGNTNYAQKLQG | GEAVAVFDP |
| | | | SEQ ID NO:4096 | SEQ ID NO:12108 | SEQ ID NO:20120 |
| iPS:453445 | 21-225_148E10 | NA | AGCTATGGCATGCAC | GTTATATGGTTTGATGGC AGTAATAAATACTATGT AGACTCCGTGAAGGAC | GATCGGGTGGAGGGTTCGGG GACTCCCTACTACTACTACG GTATGGACGTC |
| | | | SEQ ID NO:4097 | SEQ ID NO:12109 | SEQ ID NO:20121 |
| | | AA | SYGMH | VIWFDGSNKYYVDSVKD | DRVEGSGTPYYYYGMDV |
| | | | SEQ ID NO:4098 | SEQ ID NO:12110 | SEQ ID NO:20122 |
| iPS:453447 | 21-225_65F10 | NA | GGCTACCATATGCAC | TGGATCAACCCTAACAA TGGTGGCACAAGCTATG CACAGAAGTTTCAGGAC | GATAGTAGGTCGTCCTGGGA CTAC |
| | | | SEQ ID NO:4099 | SEQ ID NO:12111 | SEQ ID NO:20123 |
| | | AA | GYHMH | WINPNNGGTSYAQKFQD | DSRSSWDY |
| | | | SEQ ID NO:4100 | SEQ ID NO:12112 | SEQ ID NO:20124 |
| iPS:453449 | 21-225_208A2 | NA | AGTTACTACTGGAGC | CGTATCTATACCAGTGG GAGCACCGACTACAACC CCTCCCTCAAGAGT | GGGGTTCGGTGACTGGGACTA C |
| | | | SEQ ID NO:4101 | SEQ ID NO:12113 | SEQ ID NO:20125 |
| | | AA | SYYWS | RIYTSGSTDYNPSLKS | GFGDWDY |
| | | | SEQ ID NO:4102 | SEQ ID NO:12114 | SEQ ID NO:20126 |

FIGURE 49

| iPS:453451 | | NA | GGCTACTATTTGCAC | TGGATCAACCCTAATAG AAATGGCACAAACTATG CACAGAAGTTTCAGGGC | GACGGTACCAGCAGCTTTGA CTAC |
|---|---|---|---|---|---|
| | 21-225_52G11 | | SEQ ID NO:4103 | SEQ ID NO:12115 | SEQ ID NO:20127 |
| | | AA | GYYLH | WINPNRNGTNYAQKFQG | DGTSSFDY |
| | | | SEQ ID NO:4104 | SEQ ID NO:12116 | SEQ ID NO:20128 |
| iPS:453453 | | NA | GGCTACTATTTGCAC | TGGATCAACCCTAACAG AAATGGCACAAACTATG CACAGAATTTTCAGGGC | GACGGTACCAGTAGCTTTGA CTAC |
| | 21-225_53F2 | | SEQ ID NO:4105 | SEQ ID NO:12117 | SEQ ID NO:20129 |
| | | AA | GYYLH | WINPNRNGTNYAQNFQG | DGTSSFDY |
| | | | SEQ ID NO:4106 | SEQ ID NO:12118 | SEQ ID NO:20130 |
| iPS:468810 | | NA | GGTTGCTACTGGAGC | GAAATCAATTATAGTGG AAGGACCAACTTCAACC CGTCCCTCAAGAGT | GACTACGGCGGTATGGACGT C |
| | 21-225_74D5 | | SEQ ID NO:4107 | SEQ ID NO:12119 | SEQ ID NO:20131 |
| | | AA | GCYWS | EINYSGRTNFNPSLKS | DYGGMDV |
| | | | SEQ ID NO:4108 | SEQ ID NO:12120 | SEQ ID NO:20132 |
| iPS:468812 | | NA | GACTCTCTCATGCAC | GTTATATGGTATGATGG AAGTAATAAATACTATA CAGACTCCGTTAAGGGC | GAAAACTATAGCAGTGGCTG GTACGGGTACGGTATGGACG TC |
| | 21-225_48H4 | | SEQ ID NO:4109 | SEQ ID NO:12121 | SEQ ID NO:20133 |
| | | AA | DSLMH | VIWYDGSNKYYTDSVKG | ENYSSGWYGYGMDV |
| | | | SEQ ID NO:4110 | SEQ ID NO:12122 | SEQ ID NO:20134 |

FIGURE 49

| iPS:468816 | | NA | AGCTATGGCATGCAC | GTTATATGGTATGATGG AAGTAATAAATACTATG CAGACTCCGTGAAGGGC | AGGTATAGCAGCAGCTGGTC GGGCGGTATGGACGTC |
|---|---|---|---|---|---|
| | 21-225_52G8 | | SEQ ID NO:4111 | SEQ ID NO:12123 | SEQ ID NO:20135 |
| | | AA | SYGMH | VIWYDGSNKYYADSVKG | RYSSSWSGGMDV |
| | | | SEQ ID NO:4112 | SEQ ID NO:12124 | SEQ ID NO:20136 |
| iPS:468814 | | NA | AACTATGGCATGGAC | GTTATATGGTATGATGG AAGTAATGACTACTATG CAGACTCCGTGAAGGGC | GATCGGGGGATCGGGTACAA CGATATGGACGTC |
| | 21-225_223D11 | | SEQ ID NO:4113 | SEQ ID NO:12125 | SEQ ID NO:20137 |
| | | AA | NYGMD | VIWYDGSNDYYADSVKG | DRGIGYNDMDV |
| | | | SEQ ID NO:4114 | SEQ ID NO:12126 | SEQ ID NO:20138 |
| iPS:468822 | | NA | AACTATGGCTTACAC | ATTATATGGTATGATGG AAGTAATAAATACTATG CAGACTCCGTGAAGGGC | GATCATTACGATTTTTGGAG TGGTCACTTTGACTAC |
| | 21-225_147E10 | | SEQ ID NO:4115 | SEQ ID NO:12127 | SEQ ID NO:20139 |
| | | AA | NYGLH | IIWYDGSNKYYADSVKG | DHYDFWSGHFDY |
| | | | SEQ ID NO:4116 | SEQ ID NO:12128 | SEQ ID NO:20140 |
| iPS:468824 | | NA | AGCTATGGCATGCAC | GTTATATGGTATGATGTA AGTAATAAATACTATGG AGACTCCGTGAAGGGC | GAAGTGGGGATGACTTCTGA CTAC |
| | 21-225_73G6 | | SEQ ID NO:4117 | SEQ ID NO:12129 | SEQ ID NO:20141 |
| | | AA | SYGMH | VIWYDVSNKYYGDSVKG | EVGMTSDY |
| | | | SEQ ID NO:4118 | SEQ ID NO:12130 | SEQ ID NO:20142 |

FIGURE 49

| iPS:468818 | | NA | AGTTATGATATCAAC | TGGATGAACCCTAAAAGGGGTAACACAGGCTATGCACAGAAGTTCCAGGGC | GGAGACCCGTATAACTGGAACTCCTACGCTATGGACGTC |
|---|---|---|---|---|---|
| | 21-225_190C8 | | SEQ ID NO:4119 | SEQ ID NO:12131 | SEQ ID NO:20143 |
| | | AA | SYDIN | WMNPKRGNTGYAQKFQG | GDPYNWNSYAMDV |
| | | | SEQ ID NO:4120 | SEQ ID NO:12132 | SEQ ID NO:20144 |
| iPS:468826 | | NA | GACTATGTCATGCAC | GTTATATGGTATGATGGAAGTAATAAATACTATGTAGACTCCGTGAAGGGC | GAACGGTATAGCAGTGGCTTGTACGACTACGGTATGGACGTC |
| | 21-225_201C5 | | SEQ ID NO:4121 | SEQ ID NO:12133 | SEQ ID NO:20145 |
| | | AA | DYVMH | VIWYDGSNKYYVDSVKG | ERYSSGLYDYGMDV |
| | | | SEQ ID NO:4122 | SEQ ID NO:12134 | SEQ ID NO:20146 |
| iPS:468828 | | NA | AGCTGTGGCATGCAC | GCTATATGGTATGATGGAAGCAATAAATACTATGCAGACTCCGTGAAGGGC | GACAAAAATATAATGGGAGATACTTGGTTTGACTTC |
| | 21-225_162A10 | | SEQ ID NO:4123 | SEQ ID NO:12135 | SEQ ID NO:20147 |
| | | AA | SCGMH | AIWYDGSNKYYADSVKG | DKNIMGDTWFDF |
| | | | SEQ ID NO:4124 | SEQ ID NO:12136 | SEQ ID NO:20148 |
| iPS:468830 | | NA | GGCTACTATATGCAC | TGGATCAACCCTAATAGTGGTGGCACAAACTTTGCACAGAAGTTTCAGGGC | GGAAAGAACTATGGCTCCTACTTTGACTAC |
| | 21-225_191G11 | | SEQ ID NO:4125 | SEQ ID NO:12137 | SEQ ID NO:20149 |
| | | AA | GYYMH | WINPNSGGTNFAQKFQG | GKNYGSYFDY |
| | | | SEQ ID NO:4126 | SEQ ID NO:12138 | SEQ ID NO:20150 |

FIGURE 49

| iPS:468832 | | NA | GGTTGCTACTGGAGC | GAAATCAATTATAGTGG AAGGACCAACTTCAACC CGTCCCTCAAGAGT | GACTACGGCGGTATGGACGT C |
|---|---|---|---|---|---|
| | 21-225_76H10 | | SEQ ID NO:4127 | SEQ ID NO:12139 | SEQ ID NO:20151 |
| | | AA | GCYWS | EINYSGRTNFNPSLKS | DYGGMDV |
| | | | SEQ ID NO:4128 | SEQ ID NO:12140 | SEQ ID NO:20152 |
| iPS:468834 | | NA | GGTTGCTACTGGAGC | GAAATCAATTATAGTGG AAGGACCAACTTCAACC CGTCCCTCAAGAGT | GACTACGGCGGTATGGACGT C |
| | 21-225_94G10 | | SEQ ID NO:4129 | SEQ ID NO:12141 | SEQ ID NO:20153 |
| | | AA | GCYWS | EINYSGRTNFNPSLKS | DYGGMDV |
| | | | SEQ ID NO:4130 | SEQ ID NO:12142 | SEQ ID NO:20154 |
| iPS:468836 | | NA | AGCTATGGCATGCAC | GTTATATCATATGATGGA GGTTATAAAAACTATGC AGACTCCGTGAAGGGC | GGTACCCACGGGTACTACTA CGGTGTGGACGTC |
| | 21-225_198E3 | | SEQ ID NO:4131 | SEQ ID NO:12143 | SEQ ID NO:20155 |
| | | AA | SYGMH | VISYDGGYKNYADSVKG | GTHGYYYGVDV |
| | | | SEQ ID NO:4132 | SEQ ID NO:12144 | SEQ ID NO:20156 |
| iPS:468838 | | NA | GGTTGCTACTGGAGC | GAAATCAATTATAGTGG AAGGACCAACTTCAACC CGTCCCTCAAGAGT | GACTACGGCGGTATGGACGT C |
| | 21-225_80E12 | | SEQ ID NO:4133 | SEQ ID NO:12145 | SEQ ID NO:20157 |
| | | AA | GCYWS | EINYSGRTNFNPSLKS | DYGGMDV |
| | | | SEQ ID NO:4134 | SEQ ID NO:12146 | SEQ ID NO:20158 |
| iPS:468840 | | NA | AGTGGTGGTGACTACTGGAG C | TACATCTATTACAGTGGG AGCACCTACTACAACCC GTCCCTCAAGAGT | ATGGACTACAGTAACTACTA CTACGGTATGGACGTC |
| | 21-225_200H9 | | SEQ ID NO:4135 | SEQ ID NO:12147 | SEQ ID NO:20159 |
| | | AA | SGGDYWS | YIYYSGSTYYNPSLKS | MDYSNYYYGMDV |
| | | | SEQ ID NO:4136 | SEQ ID NO:12148 | SEQ ID NO:20160 |

FIGURE 49

| iPS:468820 | | NA | GGTTCCTACTGGAGC | GAAATCAATTATAGTGG AAGGACCAACTTCAACC CGTCCCTCAAGAGT | GACTACGGCGGTATGGACGT C |
|---|---|---|---|---|---|
| | 21-225_76E10 | | SEQ ID NO:4137 | SEQ ID NO:12149 | SEQ ID NO:20161 |
| | | AA | GSYWS | EINYSGRTNFNPSLKS | DYGGMDV |
| | | | SEQ ID NO:4138 | SEQ ID NO:12150 | SEQ ID NO:20162 |
| iPS:468842 | | NA | AGCTATGTCATGCAC | GCTATATGGTATGATGG AAGTAATAAATACTATG CAGACTCCGTGAAGGGC | GAGTTGTATAGCAGCAACTG GTACGACTACGGTATGGACG TC |
| | 21-225_50H4 | | SEQ ID NO:4139 | SEQ ID NO:12151 | SEQ ID NO:20163 |
| | | AA | SYVMH | AIWYDGSNKYYADSVKG | ELYSSNWYDYGMDV |
| | | | SEQ ID NO:4140 | SEQ ID NO:12152 | SEQ ID NO:20164 |
| iPS:468844 | | NA | AGCTATAACATGAAC | TCCATTAGTGGTAGTAGT AGTTACATATACTACGC AGACTCAGTGAAGGGC | AGCCTCGACCTC |
| | 21-225_48E10 | | SEQ ID NO:4141 | SEQ ID NO:12153 | SEQ ID NO:20165 |
| | | AA | SYNMN | SISGSSSYIYYADSVKG | SLDL |
| | | | SEQ ID NO:4142 | SEQ ID NO:12154 | SEQ ID NO:20166 |
| iPS:468846 | | NA | AGCTATAGCATGAAC | TCCATTAGTGGTAGTAGC AGTTACATATACTACGTA GACTCAGTGAAGGGC | GTCAACTCTTTTGACTCC |
| | 21-225_53B10 | | SEQ ID NO:4143 | SEQ ID NO:12155 | SEQ ID NO:20167 |
| | | AA | SYSMN | SISGSSSYIYYVDSVKG | VNSFDS |
| | | | SEQ ID NO:4144 | SEQ ID NO:12156 | SEQ ID NO:20168 |
| iPS:468848 | | NA | AGCTATGCCATGAGC | GTTCTTAGTGGTAGTGGT GGTAGCACATTCTACGC AGACTCCGTGAAGGGC | AGAGGCCGTGAATATAGTGG CTACGATTACTTTGACTAC |
| | 21-225_54B1 | | SEQ ID NO:4145 | SEQ ID NO:12157 | SEQ ID NO:20169 |

FIGURE 49

| | | AA | SYAMS | VLSGSGGSTFYADSVKG | RGREYSGYDYFDY |
|---|---|---|---|---|---|
| | | | SEQ ID NO:4146 | SEQ ID NO:12158 | SEQ ID NO:20170 |
| iPS:468850 | 21-225_63F4 | NA | AATTATGATGTCAAC | TGGATGCACCCTAACAG TGGTAACACAGGCTATG CACAGAAGTTCCGGGGC | AGCAGTGGCTGGTACGTTTT TGACTAC |
| | | | SEQ ID NO:4147 | SEQ ID NO:12159 | SEQ ID NO:20171 |
| | | AA | NYDVN | WMHPNSGNTGYAQKFRG | SSGWYVFDY |
| | | | SEQ ID NO:4148 | SEQ ID NO:12160 | SEQ ID NO:20172 |
| iPS:468852 | 21-225_71F3 | NA | AATTATGATGTCAAC | TGGATGCACCCTAACAG TGGTAACACAGGCTATG CACAGAAGTTCCAGGGC | AGCAGTGGCTGGTACGTTTT TGACTCC |
| | | | SEQ ID NO:4149 | SEQ ID NO:12161 | SEQ ID NO:20173 |
| | | AA | NYDVN | WMHPNSGNTGYAQKFQG | SSGWYVFDS |
| | | | SEQ ID NO:4150 | SEQ ID NO:12162 | SEQ ID NO:20174 |
| iPS:468854 | 21-225_72C4 | NA | AATTATGATATCAAC | TGGATGCACCCTAACAG TGGTAACACAGGCTATG CACAGAAGTTCCAGGGC | AGCAGTGGCTGGTACCTCTT TGACTAC |
| | | | SEQ ID NO:4151 | SEQ ID NO:12163 | SEQ ID NO:20175 |
| | | AA | NYDIN | WMHPNSGNTGYAQKFQG | SSGWYLFDY |
| | | | SEQ ID NO:4152 | SEQ ID NO:12164 | SEQ ID NO:20176 |
| iPS:468856 | 21-225_77C9 | NA | AGGAGTAGTTACTACTGGGG C | AGTATCTATTATAGTGGG AGCGCCTACTCCAACCC GTCCCTCAAGAGT | CTTGACTCTAACTGGGGTCT TGACTAC |
| | | | SEQ ID NO:4153 | SEQ ID NO:12165 | SEQ ID NO:20177 |
| | | AA | RSSYYWG | SIYYSGSAYSNPSLKS | LDSNWGLDY |
| | | | SEQ ID NO:4154 | SEQ ID NO:12166 | SEQ ID NO:20178 |

FIGURE 49

| iPS:468858 | | NA | GACTATGGCATGCAC | GTTATATGGTATGATGG AAGTAATAAGTACTATG CAGACTCCGTGAAGGGC | GAGAGGTATAGCAGTGGCTG GTACGACTACGGTTTGGACG TC |
|---|---|---|---|---|---|
| | 21-225_148C9 | | SEQ ID NO:4155 | SEQ ID NO:12167 | SEQ ID NO:20179 |
| | | AA | DYGMH | VIWYDGSNKYYADSVKG | ERYSSGWYDYGLDV |
| | | | SEQ ID NO:4156 | SEQ ID NO:12168 | SEQ ID NO:20180 |
| iPS:468860 | | NA | AGCTATGTCATGCAC | GTTATATGGTATGATGG AAGTAATAAATACTATG CAGACTCCGTGAAGGGC | GAACAGTATAGCAGCAGCTG GTACGACTTCGGTCTGGACG TC |
| | 21-225_224E7 | | SEQ ID NO:4157 | SEQ ID NO:12169 | SEQ ID NO:20181 |
| | | AA | SYVMH | VIWYDGSNKYYADSVKG | EQYSSSWYDFGLDV |
| | | | SEQ ID NO:4158 | SEQ ID NO:12170 | SEQ ID NO:20182 |
| iPS:468862 | | NA | GACTATTATATGCAC | TGGATCAACCCTAACAG AGGTGGCACAAACTATG CTCAGAAGTTTCAGGGC | GAGGAGGATCGCAGTGGCTG GTACTACTACTACGGTATGG ACGTC |
| | 21-225_178H8 | | SEQ ID NO:4159 | SEQ ID NO:12171 | SEQ ID NO:20183 |
| | | AA | DYYMH | WINPNRGGTNYAQKFQG | EEDRSGWYYYYGMDV |
| | | | SEQ ID NO:4160 | SEQ ID NO:12172 | SEQ ID NO:20184 |
| iPS:468864 | | NA | ACTAGTGGAGTGGGTGTGGG C | CTCATTTATTGGAAAGAT GATGAGCGCTACAGCCC ATCTCTGAAGAGC | GCAGTGGCTGTCTCCTTTGA CTAC |
| | 21-225_60D6 | | SEQ ID NO:4161 | SEQ ID NO:12173 | SEQ ID NO:20185 |
| | | AA | TSGVGVG | LIYWKDDERYSPSLKS | AVAVSFDY |
| | | | SEQ ID NO:4162 | SEQ ID NO:12174 | SEQ ID NO:20186 |
| iPS:468866 | 21 225 190C1 | NA | GGCTACTATATGCAC | TGGATCAACCCTTACAGT GGTGGCACAAACTATGC ACAGAAGTTTCAGGGC | GATAGAGCAGTGGCTGGAAA CTACTTCTACTACGGTATGG ACGTC |

FIGURE 49

| | 21-225_190C1 | | SEQ ID NO:4163 | SEQ ID NO:12175 | SEQ ID NO:20187 |
|---|---|---|---|---|---|
| | | AA | GYYMH | WINPYSGGTNYAQKFQG | DRAVAGNYFYYGMDV |
| | | | SEQ ID NO:4164 | SEQ ID NO:12176 | SEQ ID NO:20188 |
| iPS:468868 | 21-225_74A1 | NA | GGTAGTAGTTACTACTGGGGC | AATATCTATTATAGTGGGAGCACCTACCACAACCCGTCCCTCAAGAGT | CATGATTTACTTTGGTCCCTTGACTTC |
| | | | SEQ ID NO:4165 | SEQ ID NO:12177 | SEQ ID NO:20189 |
| | | AA | GSSYYWG | NIYYSGSTYHNPSLKS | HDLLWSLDF |
| | | | SEQ ID NO:4166 | SEQ ID NO:12178 | SEQ ID NO:20190 |
| iPS:468870 | 21-225_74A8 | NA | AATTATGATATCAAC | TGGATGCACCCTAACAGTGGTAACACAGGCTATGCACAGAAGTTCCAGGGC | AGTAGTGGCTGGTACAAATTTGACTAC |
| | | | SEQ ID NO:4167 | SEQ ID NO:12179 | SEQ ID NO:20191 |
| | | AA | NYDIN | WMHPNSGNTGYAQKFQG | SSGWYKFDY |
| | | | SEQ ID NO:4168 | SEQ ID NO:12180 | SEQ ID NO:20192 |
| iPS:472730 | 21-225_14B1_LC1 | NA | AGCTATACCATGAAC | TCCATAAGTGGTAGTAGTAGTTACTTATACTACCCAGACTCAGTGAAGGGC | GATAGAGGCAGCAGC |
| | | | SEQ ID NO:4169 | SEQ ID NO:12181 | SEQ ID NO:20193 |
| | | AA | SYTMN | SISGSSSYLYYPDSVKG | DRGSS |
| | | | SEQ ID NO:4170 | SEQ ID NO:12182 | SEQ ID NO:20194 |
| iPS:472731 | 21-225_14B1_LC2 | NA | AGCTATACCATGAAC | TCCATAAGTGGTAGTAGTAGTTACTTATACTACCCAGACTCAGTGAAGGGC | GATAGAGGCAGCAGC |
| | | | SEQ ID NO:4171 | SEQ ID NO:12183 | SEQ ID NO:20195 |
| | | AA | SYTMN | SISGSSSYLYYPDSVKG | DRGSS |
| | | | SEQ ID NO:4172 | SEQ ID NO:12184 | SEQ ID NO:20196 |

FIGURE 49

| iPS:472732 | 21-225_2B10_LC1 | NA | AGCTATGTCATGCAC | GTTATATGGTATGATGG AAGTAATAAATACTATG CAGACTCCGTGAAGGTC | GAGAGATATACCAGTGGCTG GTATGACTACGGTATGGACG TC |
|---|---|---|---|---|---|
| | | | SEQ ID NO:4173 | SEQ ID NO:12185 | SEQ ID NO:20197 |
| | | AA | SYVMH | VIWYDGSNKYYADSVKV | ERYTSGWYDYGMDV |
| | | | SEQ ID NO:4174 | SEQ ID NO:12186 | SEQ ID NO:20198 |
| iPS:472733 | 21-225_2B10_LC2 | NA | AGCTATGTCATGCAC | GTTATATGGTATGATGG AAGTAATAAATACTATG CAGACTCCGTGAAGGTC | GAGAGATATACCAGTGGCTG GTATGACTACGGTATGGACG TC |
| | | | SEQ ID NO:4175 | SEQ ID NO:12187 | SEQ ID NO:20199 |
| | | AA | SYVMH | VIWYDGSNKYYADSVKV | ERYTSGWYDYGMDV |
| | | | SEQ ID NO:4176 | SEQ ID NO:12188 | SEQ ID NO:20200 |
| iPS:473253 | 21-225_7C3_LC1 | NA | GACTACTATTTGCAC | TGGATCCACCCTAACAG TGGTGGCACAAACTATG CACAGAAGTTTCAGGGC | GATGGTACCAGCTCGTTTGA CTAC |
| | | | SEQ ID NO:4177 | SEQ ID NO:12189 | SEQ ID NO:20201 |
| | | AA | DYYLH | WIHPNSGGTNYAQKFQG | DGTSSFDY |
| | | | SEQ ID NO:4178 | SEQ ID NO:12190 | SEQ ID NO:20202 |
| iPS:473254 | 21-225_7C3_LC2 | NA | GACTACTATTTGCAC | TGGATCCACCCTAACAG TGGTGGCACAAACTATG CACAGAAGTTTCAGGGC | GATGGTACCAGCTCGTTTGA CTAC |
| | | | SEQ ID NO:4179 | SEQ ID NO:12191 | SEQ ID NO:20203 |
| | | AA | DYYLH | WIHPNSGGTNYAQKFQG | DGTSSFDY |
| | | | SEQ ID NO:4180 | SEQ ID NO:12192 | SEQ ID NO:20204 |

FIGURE 49

| iPS:473255 | | NA | GACTACTATTTGCAC | TGGATCCACCCTAACAG TGGTGGCACAAACTTTG CACAGAAGTTTCAGGGC | GATGGTACCAGCTCGTTTGA CTAC |
|---|---|---|---|---|---|
| | 21-225_9F12_LC1 | | SEQ ID NO:4181 | SEQ ID NO:12193 | SEQ ID NO:20205 |
| | | AA | DYYLH | WIHPNSGGTNFAQKFQG | DGTSSFDY |
| | | | SEQ ID NO:4182 | SEQ ID NO:12194 | SEQ ID NO:20206 |
| iPS:473256 | | NA | GACTACTATTTGCAC | TGGATCCACCCTAACAG TGGTGGCACAAACTTTG CACAGAAGTTTCAGGGC | GATGGTACCAGCTCGTTTGA CTAC |
| | 21-225_9F12_LC2 | | SEQ ID NO:4183 | SEQ ID NO:12195 | SEQ ID NO:20207 |
| | | AA | DYYLH | WIHPNSGGTNFAQKFQG | DGTSSFDY |
| | | | SEQ ID NO:4184 | SEQ ID NO:12196 | SEQ ID NO:20208 |
| iPS:472742 | | NA | GGCTACTATCTGCAC | TGGATCAACCCTAACAG TGGTGGCACAAACTATG CACAGAAGTTTCAGGGC | GTGTATTACTATGGTTCGGG GAGTTATTATAACGAGTTTG ACAAC |
| | 21-225_30D9_LC2 | | SEQ ID NO:4185 | SEQ ID NO:12197 | SEQ ID NO:20209 |
| | | AA | GYYLH | WINPNSGGTNYAQKFQG | VYYYGSGSYYNEFDN |
| | | | SEQ ID NO:4186 | SEQ ID NO:12198 | SEQ ID NO:20210 |
| iPS:472741 | | NA | GGCTACTATCTGCAC | TGGATCAACCCTAACAG TGGTGGCACAAACTATG CACAGAAGTTTCAGGGC | GTGTATTACTATGGTTCGGG GAGTTATTATAACGAGTTTG ACAAC |
| | 21-225_30D9_LC1 | | SEQ ID NO:4187 | SEQ ID NO:12199 | SEQ ID NO:20211 |
| | | AA | GYYLH | WINPNSGGTNYAQKFQG | VYYYGSGSYYNEFDN |
| | | | SEQ ID NO:4188 | SEQ ID NO:12200 | SEQ ID NO:20212 |
| iPS:472743 | | NA | GGYTACTATATGCAC | TCGATCTACCGTAACAGT GGTGGCACAAATTATGC ACAGAAGTTTCAGGGC | GCCTTTTACTATGGTTCGGG GACTTATTATAACGAATTTG ACTAC |
| | 21-225_68G6 | | SEQ ID NO:4189 | SEQ ID NO:12201 | SEQ ID NO:20213 |
| | | AA | GYYMH | SIYRNSGGTNYAQKFQG | AFYYGSGTYYNEFDY |
| | | | SEQ ID NO:4190 | SEQ ID NO:12202 | SEQ ID NO:20214 |

FIGURE 49

| iPS:392573 | 21-225_15G2 | NA | ACTAGTGGAGTGGGTGTGGG C | CTCATTTATTGGAATGAT GATAAGCGCTACAGCCC ATCTCTGAAGAGC | ACCGGTGTCAGCTGCTGCTA TTTTCACTAT |
|---|---|---|---|---|---|
| | | | SEQ ID NO:4191 | SEQ ID NO:12203 | SEQ ID NO:20215 |
| | | AA | TSGVGVG | LIYWNDDKRYSPSLKS | TGVSCCYFHY |
| | | | SEQ ID NO:4192 | SEQ ID NO:12204 | SEQ ID NO:20216 |
| iPS:392583 | 21-225_10B10 | NA | ACTGGTGGAGTGGGTGTGGG C | TTCATTTATTGGAGTGAT GATAAGCGCTACAGCCC ATCTCTGAAGAGC | ATAGCAGCAGTTGCCTTTGA CTAC |
| | | | SEQ ID NO:4193 | SEQ ID NO:12205 | SEQ ID NO:20217 |
| | | AA | TGGVGVG | FIYWSDDKRYSPSLKS | IAAVAFDY |
| | | | SEQ ID NO:4194 | SEQ ID NO:12206 | SEQ ID NO:20218 |
| iPS:392585 | 21-225_14H11 | NA | GGCCACTATATGTGC | TGGATCAACCCTAATAG TGGTGGCACAAACTATG CACAGAAGTTTCAGGGC | GGATATTGTAGTAGTTCCAG CTGCTATTTGCAACCGGGTT ATTACGGTATGGACGTC |
| | | | SEQ ID NO:4195 | SEQ ID NO:12207 | SEQ ID NO:20219 |
| | | AA | GHYMC | WINPNSGGTNYAQKFQG | GYCSSSSCYLQPGYYGMDV |
| | | | SEQ ID NO:4196 | SEQ ID NO:12208 | SEQ ID NO:20220 |
| iPS:392587 | 21-225_18G5 | NA | ACTAGTGGAGTGGGTGTGGG C | CTCATTTATTGGAATGAT GATAAGGTCTACAGCCC ATCTCTGAAGAGC | AGGGGACAGCAGCTGGCCCT CGACTAC |
| | | | SEQ ID NO:4197 | SEQ ID NO:12209 | SEQ ID NO:20221 |
| | | AA | TSGVGVG | LIYWNDDKVYSPSLKS | RGQQLALDY |
| | | | SEQ ID NO:4198 | SEQ ID NO:12210 | SEQ ID NO:20222 |
| iPS:392589 | 21-225_27H2 | NA | GGCTATGGCATGCAC | GTTATATGGTATGATGG AAGTAATAAATACTATG CAGACTCCGTGAAGGGC | GATAGGGTATATTGTAGTAG TACCAGCTGCTCCCCTTACT ACTACTACTACGGTATGGAC GTC |
| | | | SEQ ID NO:4199 | SEQ ID NO:12211 | SEQ ID NO:20223 |

FIGURE 49

| | | AA | GYGMH | VIWYDGSNKYYADSVKG | DRVYCSSTSCSPYYYYYGMD V |
|---|---|---|---|---|---|
| | | | SEQ ID NO:4200 | SEQ ID NO:12212 | SEQ ID NO:20224 |
| iPS:392593 | 21-225_3E10 | NA | ACTGGTGGAGTGGGTGTGGG C | CTCATTTATTGGAATGAT GATAAGCGCCACAGCCC ATCTCTGAAGAGC | CTTATAGAAGTGGCCTTTGA CTAT |
| | | | SEQ ID NO:4201 | SEQ ID NO:12213 | SEQ ID NO:20225 |
| | | AA | TGGVGVG | LIYWNDDKRHSPSLKS | LIEVAFDY |
| | | | SEQ ID NO:4202 | SEQ ID NO:12214 | SEQ ID NO:20226 |
| iPS:392596 | 21-225_12D8 | NA | AGCTATGTCATGAGC | ACTATTAGTGTTGGTGGT GGTAGCACATACTACGC AGACTCCGTGAAGGGC | TGGGGACGTGGATACAGCTA TGAATACTACTACGGTATGG ACGTC |
| | | | SEQ ID NO:4203 | SEQ ID NO:12215 | SEQ ID NO:20227 |
| | | AA | SYVMS | TISVGGGSTYYADSVKG | WGRGYSYEYYYGMDV |
| | | | SEQ ID NO:4204 | SEQ ID NO:12216 | SEQ ID NO:20228 |
| iPS:392598 | 21-225_18E10 | NA | GGCTACTATATGCAC | TGGATCAACCCTAACAG TGGTGGCACAAACTATG CACAGAAGTTTCAGGGC | TCGTACTACTATGGTTCGGG GAGTTATTATAACGAGTTTG ACTAC |
| | | | SEQ ID NO:4205 | SEQ ID NO:12217 | SEQ ID NO:20229 |
| | | AA | GYYMH | WINPNSGGTNYAQKFQG | SYYYGSGSYYNEFDY |
| | | | SEQ ID NO:4206 | SEQ ID NO:12218 | SEQ ID NO:20230 |
| iPS:392618 | 21-225_16F10 | NA | GACTATGGCATGCAC | GTCATATGGTATGATGG AAATAATAAATACTATG TAGACTCCGTGAAGGGC | GAGCTTGCCTGGTACGAGGA CTAC |
| | | | SEQ ID NO:4207 | SEQ ID NO:12219 | SEQ ID NO:20231 |
| | | AA | DYGMH | VIWYDGNNKYYVDSVKG | ELAWYEDY |
| | | | SEQ ID NO:4208 | SEQ ID NO:12220 | SEQ ID NO:20232 |

FIGURE 49

| iPS:392620 | | NA | AGCTATAGCATGAAC | TCCATTAGTAGTAGTAGT ACTTACATATACTACGCA GACTCAGTGAAGGGC | GTGGCTTCATTTGACTAC |
|---|---|---|---|---|---|
| | 21-225_17H5 | | SEQ ID NO:4209 | SEQ ID NO:12221 | SEQ ID NO:20233 |
| | | AA | SYSMN | SISSSSTYIYYADSVKG | VASFDY |
| | | | SEQ ID NO:4210 | SEQ ID NO:12222 | SEQ ID NO:20234 |
| iPS:392622 | | NA | AGAAGTAGTTACTACTGGGG C | AATATCTATTATGGTGGG AACACCTACTACAACCC GTCCCTCAAGAGT | CATGGAAAAGACTGGGGCCT TGACTAC |
| | 21-225_17H8 | | SEQ ID NO:4211 | SEQ ID NO:12223 | SEQ ID NO:20235 |
| | | AA | RSSYYWG | NIYYGGNTYYNPSLKS | HGKDWGLDY |
| | | | SEQ ID NO:4212 | SEQ ID NO:12224 | SEQ ID NO:20236 |
| iPS:392624 | | NA | AGCTATACCATGAAC | TCCATTAGTGGTAGTAGT AGTTACATATACTACGC AGACTCAGTGAAGGGC | GATCGAGGCTCCATC |
| | 21-225_17H12 | | SEQ ID NO:4213 | SEQ ID NO:12225 | SEQ ID NO:20237 |
| | | AA | SYTMN | SISGSSSYIYYADSVKG | DRGSI |
| | | | SEQ ID NO:4214 | SEQ ID NO:12226 | SEQ ID NO:20238 |
| iPS:392626 | | NA | GACTATGGCATGCAC | GTTATATGGTTTGATGGA AGTAATAAATACTATGC AGACTCCGTGAAGGGC | GATCTTGGCTGGACGGAAGA GTAC |
| | 21-225_18A5 | | SEQ ID NO:4215 | SEQ ID NO:12227 | SEQ ID NO:20239 |
| | | AA | DYGMH | VIWFDGSNKYYADSVKG | DLGWTEEY |
| | | | SEQ ID NO:4216 | SEQ ID NO:12228 | SEQ ID NO:20240 |
| iPS:392628 | | NA | AGAAGTAGTTACTACTGGGG C | AATATCTATTATAGTGGG ACTGCCTACTGTAATTCG TCCCTCAAGAGT | CATAGTAGCAGCTGGTCCCT TGACAAC |
| | 21-225_20C2 | | SEQ ID NO:4217 | SEQ ID NO:12229 | SEQ ID NO:20241 |
| | | AA | RSSYYWG | NIYYSGTAYCNSSLKS | HSSSWSLDN |

FIGURE 49

| | | | SEQ ID NO:4218 | SEQ ID NO:12230 | SEQ ID NO:20242 |
|---|---|---|---|---|---|
| iPS:392630 | | NA | GACTATGGCATGCAC | GTTATTTGGTATGAAGA AAATAATCAATACTATG CAGACTCCGTGAAGGGC | GAACTGGGGTTCCGGTCTGA CTAC |
| | 21-225_20E5 | | SEQ ID NO:4219 | SEQ ID NO:12231 | SEQ ID NO:20243 |
| | | AA | DYGMH | VIWYEENNQYYADSVKG | ELGFRSDY |
| | | | SEQ ID NO:4220 | SEQ ID NO:12232 | SEQ ID NO:20244 |
| iPS:392632 | | NA | AGCTATAGCATGAAC | TCCATTAGTGGTAGTAGT AGTCTCATATACTACGCA GACTCAGTGAAGGGC | GTAGCAGCCTTTGACTAC |
| | 21-225_16A11 | | SEQ ID NO:4221 | SEQ ID NO:12233 | SEQ ID NO:20245 |
| | | AA | SYSMN | SISGSSSLIYYADSVKG | VAAFDY |
| | | | SEQ ID NO:4222 | SEQ ID NO:12234 | SEQ ID NO:20246 |
| iPS:392634 | | NA | AACTGTGTCATGCAC | GTTATATGGTATGATGG AAGTAATAAATACTATG CAGACTCCGTGAAGGGC | GAAAAGTATAGCAGCAGCTG GTACGACTACGGTATGGACG TC |
| | 21-225_17H3 | | SEQ ID NO:4223 | SEQ ID NO:12235 | SEQ ID NO:20247 |
| | | AA | NCVMH | VIWYDGSNKYYADSVKG | EKYSSSWYDYGMDV |
| | | | SEQ ID NO:4224 | SEQ ID NO:12236 | SEQ ID NO:20248 |
| iPS:392636 | | NA | CGCAACACTGCTGCTTGGAG C | AGGACATACTACAGGTC CAAGTGGTATAATGATT ATGCAGTATCTGTGAAA AGT | GTAAGCAGTGGCTGGTCCCA TCACTACTACTACTACGGTA TGGACGTC |
| | 21-225_17A6 | | SEQ ID NO:4225 | SEQ ID NO:12237 | SEQ ID NO:20249 |
| | | AA | RNTAAWS | RTYYRSKWYNDYAVSVK S | VSSGWSHHYYYYGMDV |

FIGURE 49

| | | | SEQ ID NO:4226 | SEQ ID NO:12238 | SEQ ID NO:20250 |
|---|---|---|---|---|---|
| iPS:392638 | 21-225_17F9 | NA | AGAAGTAGTTACTATTGGGGC | AATATCTATTATAGTGGGAGCACCTACTACAATCCGTCCCTCAAGAGT | CATGGAAAAGACTGGGGCCTTGACTAC |
| | | | SEQ ID NO:4227 | SEQ ID NO:12239 | SEQ ID NO:20251 |
| | | AA | RSSYYWG | NIYYSGSTYYNPSLKS | HGKDWGLDY |
| | | | SEQ ID NO:4228 | SEQ ID NO:12240 | SEQ ID NO:20252 |
| iPS:392640 | 21-225_18A1 | NA | AGCTATGGCATGCAT | GTTATATGGTATGAGGAAAATAATAAATATTATGTAGACTCCGTGAAGGGC | GAGCTAGGCTTCCAGTCTGACTAC |
| | | | SEQ ID NO:4229 | SEQ ID NO:12241 | SEQ ID NO:20253 |
| | | AA | SYGMH | VIWYEENNKYYVDSVKG | ELGFQSDY |
| | | | SEQ ID NO:4230 | SEQ ID NO:12242 | SEQ ID NO:20254 |
| iPS:392642 | 21-225_18C6 | NA | AGGAGTAGTTATTACTGGGGC | AATATCTATTATAGTGGGTACACCTACTACAACCCGTCCCTCAAGAGT | CATAGTAGCAGTTGGTCCCTTGACGAC |
| | | | SEQ ID NO:4231 | SEQ ID NO:12243 | SEQ ID NO:20255 |
| | | AA | RSSYYWG | NIYYSGYTYYNPSLKS | HSSSWSLDD |
| | | | SEQ ID NO:4232 | SEQ ID NO:12244 | SEQ ID NO:20256 |
| iPS:392644 | 21-225_19E1 | NA | AACTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4233 | SEQ ID NO:12245 | SEQ ID NO:20257 |
| | | AA | NYGMH | VIWYEENNQYYADSVKG | ELGFRSDY |
| | | | SEQ ID NO:4234 | SEQ ID NO:12246 | SEQ ID NO:20258 |

FIGURE 49

| iPS:392646 | | NA | AGCGATGACATGCAC | GTTATATGGTTTGATGGA AGTAATAAATACTATGC AGACTCCGTGAAGGGC | GATCTGATAGCAGCAGCTGG TACGGTTGACTAC |
|---|---|---|---|---|---|
| | 21-225_20G2 | | SEQ ID NO:4235 | SEQ ID NO:12247 | SEQ ID NO:20259 |
| | | AA | SDDMH | VIWFDGSNKYYADSVKG | DLIAAAGTVDY |
| | | | SEQ ID NO:4236 | SEQ ID NO:12248 | SEQ ID NO:20260 |
| iPS:392648 | | NA | CGCAACACTGCTGCTTGGAG C | AGGACATACTACAGGTC CAAGTGGTATAATGATT ATGCAGTATCTGTGAAA AGT | GTAAACAGTGGCTGGTCCCA TCACTACTACTACTACGGTA TGGACGTC |
| | 21-225_16D11 | | SEQ ID NO:4237 | SEQ ID NO:12249 | SEQ ID NO:20261 |
| | | AA | RNTAAWS | RTYYRSKWYNDYAVSVK S | VNSGWSHHYYYYGMDV |
| | | | SEQ ID NO:4238 | SEQ ID NO:12250 | SEQ ID NO:20262 |
| iPS:392650 | | NA | GACTACTACATGAGC | CACATTAGTAGTAGTGG TAGTACCATATATTACGC AGACTCTGTGAAGGGC | TATCGGAATAACCGGGGATA CTTCGATCTC |
| | 21-225_17A4 | | SEQ ID NO:4239 | SEQ ID NO:12251 | SEQ ID NO:20263 |
| | | AA | DYYMS | HISSSGSTIYYADSVKG | YRNNRGYFDL |
| | | | SEQ ID NO:4240 | SEQ ID NO:12252 | SEQ ID NO:20264 |
| iPS:392652 | | NA | AGCTATGCCATGAAC | GTTATTAGTGGTCGTGGT GGTAACACATTCTACGC AGACTCCGTGAAGGGC | CGTTTGGCAGTGGCTGGCTC GGAGGCTTTTGATATC |
| | 21-225_17C6 | | SEQ ID NO:4241 | SEQ ID NO:12253 | SEQ ID NO:20265 |
| | | AA | SYAMN | VISGRGGNTFYADSVKG | RLAVAGSEAFDI |
| | | | SEQ ID NO:4242 | SEQ ID NO:12254 | SEQ ID NO:20266 |

FIGURE 49

| iPS:392654 | | NA | AACTATGGCATGCAC | GTTATTTGGTATGAAGA AAATAATCAATACTATG CAGACTCCGTGAAGGGC | GAACTGGGGTTCCGGTCTGA CTAC |
|---|---|---|---|---|---|
| | 21-225_17A10 | | SEQ ID NO:4243 | SEQ ID NO:12255 | SEQ ID NO:20267 |
| | | AA | NYGMH | VIWYEENNQYYADSVKG | ELGFRSDY |
| | | | SEQ ID NO:4244 | SEQ ID NO:12256 | SEQ ID NO:20268 |
| iPS:392656 | | NA | AGGAGTAGTTACTACTGGGG C | AATATTTATTATAGTGGG AGCGCCTACAACAACCC GTCCCTCAAGGGT | CATGGAAAAGACTGGGGCCT TGACTAC |
| | 21-225_1F2 | | SEQ ID NO:4245 | SEQ ID NO:12257 | SEQ ID NO:20269 |
| | | AA | RSSYYWG | NIYYSGSAYNNPSLKG | HGKDWGLDY |
| | | | SEQ ID NO:4246 | SEQ ID NO:12258 | SEQ ID NO:20270 |
| iPS:392658 | | NA | AACTATGGCATGCAC | GTTATTTGGTATGAAGA AAATAATCAATACTATG CAGACTCCGTGAAGGGC | GAACTGGGGTTCCGGTCTGA CTAC |
| | 21-225_18E8 | | SEQ ID NO:4247 | SEQ ID NO:12259 | SEQ ID NO:20271 |
| | | AA | NYGMH | VIWYEENNQYYADSVKG | ELGFRSDY |
| | | | SEQ ID NO:4248 | SEQ ID NO:12260 | SEQ ID NO:20272 |
| iPS:392660 | | NA | AGTTATGACATGCAC | GTTATATGGTATGACGG AAGTGATAAATACTATG CAGACTCCGTGAAGGGC | GATCGGGCCTATAGCAGCTC GTCTGACTAC |
| | 21-225_19B3 | | SEQ ID NO:4249 | SEQ ID NO:12261 | SEQ ID NO:20273 |
| | | AA | SYDMH | VIWYDGSDKYYADSVKG | DRAYSSSSDY |
| | | | SEQ ID NO:4250 | SEQ ID NO:12262 | SEQ ID NO:20274 |

FIGURE 49

| iPS:392664 | | NA | AGCTATGGCATGCAC | GTTATATGGCATGATGG AAGTAATAAATACTATG CAGACTCCGTGAAGGGC | GATCTGAGTATGGGCGGAAT GGACGTC |
|---|---|---|---|---|---|
| | 21-225_20F6 | | SEQ ID NO:4251 | SEQ ID NO:12263 | SEQ ID NO:20275 |
| | | AA | SYGMH | VIWHDGSNKYYADSVKG | DLSMGGMDV |
| | | | SEQ ID NO:4252 | SEQ ID NO:12264 | SEQ ID NO:20276 |
| iPS:392666 | | NA | AGCTATGGCATGCAC | GTTATATGGTATGAGGA AAATAATAAATACTATG TAGACTCCGTGAAGGGC | GAACTAGGCTTCCAGTCTGA CTAC |
| | 21-225_16F11 | | SEQ ID NO:4253 | SEQ ID NO:12265 | SEQ ID NO:20277 |
| | | AA | SYGMH | VIWYEENNKYYVDSVKG | ELGFQSDY |
| | | | SEQ ID NO:4254 | SEQ ID NO:12266 | SEQ ID NO:20278 |
| iPS:392668 | | NA | AGCTATGCCATGAAC | GTTATTAGTGGCCGTGGT GGTAACACATTCTACGC AGACTCCGTGAAGGGC | CGTTTGGCAGTGGCTGGCTC GGAGGCTTTTGATATC |
| | 21-225_17B4 | | SEQ ID NO:4255 | SEQ ID NO:12267 | SEQ ID NO:20279 |
| | | AA | SYAMN | VISGRGGNTFYADSVKG | RLAVAGSEAFDI |
| | | | SEQ ID NO:4256 | SEQ ID NO:12268 | SEQ ID NO:20280 |
| iPS:392674 | | NA | GACTATGGCATGCAC | GTTATATGGTATGATGTA ACTAATAAATACTATGC AGACTCCGTGAAGGGC | GAGCTTGGCTGGTACGAGGA CTAC |
| | 21-225_18C2 | | SEQ ID NO:4257 | SEQ ID NO:12269 | SEQ ID NO:20281 |
| | | AA | DYGMH | VIWYDVTNKYYADSVKG | ELGWYEDY |
| | | | SEQ ID NO:4258 | SEQ ID NO:12270 | SEQ ID NO:20282 |

FIGURE 49

| iPS:392676 | | NA | GGTAGTAGTTACTACTGGGGC | AATATCTATTATAGTGGGAGCACCTACTACAACCCGTCCTTCAAGAGT | CATTCCAGTAGCTGGTCCCTTGACTAC |
|---|---|---|---|---|---|
| | 21-225_19F3 | | SEQ ID NO:4259 | SEQ ID NO:12271 | SEQ ID NO:20283 |
| | | AA | GSSYYWG | NIYYSGSTYYNPSFKS | HSSSWSLDY |
| | | | SEQ ID NO:4260 | SEQ ID NO:12272 | SEQ ID NO:20284 |
| iPS:392678 | | NA | AGCTATGCCATGAAC | GTTATTAGTGGTAGTGGTGGTAGCACATACTACGCAGACTCCGTGAAGGGC | AGGATAGCAGCAGCTGGTACGGAGTACTTCGATCTC |
| | 21-225_20F3 | | SEQ ID NO:4261 | SEQ ID NO:12273 | SEQ ID NO:20285 |
| | | AA | SYAMN | VISGSGGSTYYADSVKG | RIAAAGTEYFDL |
| | | | SEQ ID NO:4262 | SEQ ID NO:12274 | SEQ ID NO:20286 |
| iPS:392680 | | NA | AACTATGGCATGCAC | GTTATTTGGTATGAAGAAAATAATCAATACTATGCAGACTCCGTGAAGGGC | GAACTGGGGTTCCGGTCTGACTAC |
| | 21-225_20A7 | | SEQ ID NO:4263 | SEQ ID NO:12275 | SEQ ID NO:20287 |
| | | AA | NYGMH | VIWYEENNQYYADSVKG | ELGFRSDY |
| | | | SEQ ID NO:4264 | SEQ ID NO:12276 | SEQ ID NO:20288 |
| iPS:392682 | | NA | AGCTATAGAATGAAC | TCCATTAGTGGTAGTAGTACTGACATATACTACGCAGACTCAGTGAAGGGC | AGGGACTTC |
| | 21-225_16A12 | | SEQ ID NO:4265 | SEQ ID NO:12277 | SEQ ID NO:20289 |
| | | AA | SYRMN | SISGSSTDIYYADSVKG | RDF |
| | | | SEQ ID NO:4266 | SEQ ID NO:12278 | SEQ ID NO:20290 |
| iPS:392684 | | NA | AACTATGGCATGAAC | GTTATATGGTATGATGGAAATAATAAACACTATGCAGACTCCGTGAAGGGC | AGTGGGAGCTACTTCTTTGACTAC |
| | 21 225 17F4 | | | | |

FIGURE 49

| | | | SEQ ID NO:4267 | SEQ ID NO:12279 | SEQ ID NO:20291 |
|---|---|---|---|---|---|
| | 21-225_17F4 | AA | NYGMN | VIWYDGNNKHYADSVKG | SGSYFFDY |
| | | | SEQ ID NO:4268 | SEQ ID NO:12280 | SEQ ID NO:20292 |
| iPS:392686 | 21-225_17C7 | NA | GACTATGGCATGCAC | GTTATATGGTTTGATGGA AGTAATAAATACTATGC AGACTCCGTGAAGGGC | GATCTTGGCTGGACGGAAGA ATAC |
| | | | SEQ ID NO:4269 | SEQ ID NO:12281 | SEQ ID NO:20293 |
| | | AA | DYGMH | VIWFDGSNKYYADSVKG | DLGWTEEY |
| | | | SEQ ID NO:4270 | SEQ ID NO:12282 | SEQ ID NO:20294 |
| iPS:392690 | 21-225_18F2 | NA | GACTATGGCATGCAC | GTTATATGGTTTGATGGA AGTAATAAATACTATGT AGACTCCGTGAAGGGC | GATCTTGGCTGGACGGAAGA GTAC |
| | | | SEQ ID NO:4271 | SEQ ID NO:12283 | SEQ ID NO:20295 |
| | | AA | DYGMH | VIWFDGSNKYYVDSVKG | DLGWTEEY |
| | | | SEQ ID NO:4272 | SEQ ID NO:12284 | SEQ ID NO:20296 |
| iPS:392692 | 21-225_18G10 | NA | ACCTATAGCATGAAC | TACATTAGTAGGAGTAG TAGTACCATAGACTACG CAGACTCTGTGAAGGGC | GGAGGTGGGAGCCCTTTTGA CTAC |
| | | | SEQ ID NO:4273 | SEQ ID NO:12285 | SEQ ID NO:20297 |
| | | AA | TYSMN | YISRSSSTIDYADSVKG | GGGSPFDY |
| | | | SEQ ID NO:4274 | SEQ ID NO:12286 | SEQ ID NO:20298 |
| iPS:392694 | 21-225_19A5 | NA | AGCTATGCCATGCAC | GTTATATGGTTTGATGGA AGTGATAAATACTATGC AGACTCCGTGAAGGGC | GATCGGGCCTATAGTAGCTC GTCTGACTAC |
| | | | SEQ ID NO:4275 | SEQ ID NO:12287 | SEQ ID NO:20299 |
| | | AA | SYAMH | VIWFDGSDKYYADSVKG | DRAYSSSSDY |
| | | | SEQ ID NO:4276 | SEQ ID NO:12288 | SEQ ID NO:20300 |

FIGURE 49

| iPS:392696 | 21-225_20A4 | NA | AGCTATGCCATGACC | GTTATAAGTGGTAGTGG TGGTTACACATACAACG CGGACTCCGTGAAGGGC | CGTATAGCAGTGGCTGGCTC GGAGGCTTTTGATATC |
|---|---|---|---|---|---|
| | | | SEQ ID NO:4277 | SEQ ID NO:12289 | SEQ ID NO:20301 |
| | | AA | SYAMT | VISGSGGYTYNADSVKG | RIAVAGSEAFDI |
| | | | SEQ ID NO:4278 | SEQ ID NO:12290 | SEQ ID NO:20302 |
| iPS:392700 | 21-225_16E12 | NA | AACTATGGCATGCAC | GTTATATGGTATGAGGG AAGTAATAAATATTATG TAGACTCCGTGAGGGGC | GAGCTAGGCTTCCAGTCTGA TCAC |
| | | | SEQ ID NO:4279 | SEQ ID NO:12291 | SEQ ID NO:20303 |
| | | AA | NYGMH | VIWYEGSNKYYVDSVRG | ELGFQSDH |
| | | | SEQ ID NO:4280 | SEQ ID NO:12292 | SEQ ID NO:20304 |
| iPS:392702 | 21-225_17F7 | NA | AGCTATGCCATGAAC | ATTATTAGTGGTCGTGGT GGTAACACATTCTACGC AGACTCCGTGAAGGGC | CGTTTGGCAGTGGCTGGCTC GGAGGCTTTTGATATC |
| | | | SEQ ID NO:4281 | SEQ ID NO:12293 | SEQ ID NO:20305 |
| | | AA | SYAMN | IISGRGGNTFYADSVKG | RLAVAGSEAFDI |
| | | | SEQ ID NO:4282 | SEQ ID NO:12294 | SEQ ID NO:20306 |
| iPS:392704 | 21-225_17F11 | NA | AGCTATGCCATGAGC | GTTATTAGTGGTAGAGG TGGTAACACATACTCCG CAGACTCCGTGAAGGGC | CGTTTAGCAGTGGCTGGCTC GGAGGCTTTTCATATC |
| | | | SEQ ID NO:4283 | SEQ ID NO:12295 | SEQ ID NO:20307 |
| | | AA | SYAMS | VISGRGGNTYSADSVKG | RLAVAGSEAFHI |
| | | | SEQ ID NO:4284 | SEQ ID NO:12296 | SEQ ID NO:20308 |
| iPS:392706 | 21-225_18A3 | NA | AGAAGTAGTTATTACTGGGG C | AATATCTATTATAGTGGG TATACCTACTACACTCCG TCCCTCAAGAGT | CATAGCACCAGCTGGTCCCT TGACTAC |
| | | | SEQ ID NO:4285 | SEQ ID NO:12297 | SEQ ID NO:20309 |
| | | AA | RSSYYWG | NIYYSGYTYYTPSLKS | HSTSWSLDY |
| | | | SEQ ID NO:4286 | SEQ ID NO:12298 | SEQ ID NO:20310 |

FIGURE 49

| iPS:392708 | | NA | AGCTATAGCATGAAC | TACATTAGTAGTAGTAGT GGTACCATATACTACGC AGACTCTGTGAAGGGC | GGAGGTGGGAGCCCTTTTGA CTAC |
|---|---|---|---|---|---|
| | 21-225_18D11 | | SEQ ID NO:4287 | SEQ ID NO:12299 | SEQ ID NO:20311 |
| | | AA | SYSMN | YISSSSGTIYYADSVKG | GGGSPFDY |
| | | | SEQ ID NO:4288 | SEQ ID NO:12300 | SEQ ID NO:20312 |
| iPS:392710 | | NA | AGCTATGGCATGCAC | GTTATATGGTATGATGA AAGTAATAAATACTATG CAGACTCCGTGAAGGGC | GAGCTTGCCTGGTACGAGGA CTCC |
| | 21-225_19A10 | | SEQ ID NO:4289 | SEQ ID NO:12301 | SEQ ID NO:20313 |
| | | AA | SYGMH | VIWYDESNKYYADSVKG | ELAWYEDS |
| | | | SEQ ID NO:4290 | SEQ ID NO:12302 | SEQ ID NO:20314 |
| iPS:392714 | | NA | AGCTATGCCATGACC | ACTATTAGTGGTCGTGGT GGTCACACATACTACGC AGACTCCGTGAGGGGC | CAGGACTGC |
| | 21-225_16G12 | | SEQ ID NO:4291 | SEQ ID NO:12303 | SEQ ID NO:20315 |
| | | AA | SYAMT | TISGRGGHTYYADSVRG | QDC |
| | | | SEQ ID NO:4292 | SEQ ID NO:12304 | SEQ ID NO:20316 |
| iPS:392716 | | NA | GACTATGGCATGCAC | GTTATATGGTATGATGA AAGTAATAAACACTATA TAGACTCCGTGAAGGGC | GAACTGGGGTTCCGGTTTGA CTAC |
| | 21-225_17B5 | | SEQ ID NO:4293 | SEQ ID NO:12305 | SEQ ID NO:20317 |
| | | AA | DYGMH | VIWYDESNKHYIDSVKG | ELGFRFDY |
| | | | SEQ ID NO:4294 | SEQ ID NO:12306 | SEQ ID NO:20318 |
| iPS:392718 | | NA | AGCTATGCTATCAAC | TGGATGAACCCTAACAC TGGTAACACAGGCTATG CACAGAAGTTCCAGGGC | AAGGCTGGGTTTGACTAC |

FIGURE 49

| | 21-225_17B8 | | | SEQ ID NO:4295 | SEQ ID NO:12307 | SEQ ID NO:20319 |
|---|---|---|---|---|---|---|
| | | AA | SYAIN | WMNPNTGNTGYAQKFQG | KAGFDY |
| | | | | SEQ ID NO:4296 | SEQ ID NO:12308 | SEQ ID NO:20320 |
| iPS:392720 | 21-225_17A12 | NA | AGCTATGCCATGAGC | ATTATTAGTGGTCGTGGG GGAAACGCATTCTACGC AGACTCCGTGAAGGGC | CGTATAGCAGTGGCTGGCTC GGAGGCTTTTGATATC |
| | | | | SEQ ID NO:4297 | SEQ ID NO:12309 | SEQ ID NO:20321 |
| | | AA | SYAMS | IISGRGGNAFYADSVKG | RIAVAGSEAFDI |
| | | | | SEQ ID NO:4298 | SEQ ID NO:12310 | SEQ ID NO:20322 |
| iPS:392722 | 21-225_18E12 | NA | AGCTATGCCATGAGC | ATTATTAGTGGTCGTGGT GGTAACACATTCTACGC AGACTCCGTGAAGGGC | CGTCTGGCAGTGGCTGGCTC GGAGGCTTTTGATATC |
| | | | | SEQ ID NO:4299 | SEQ ID NO:12311 | SEQ ID NO:20323 |
| | | AA | SYAMS | IISGRGGNTFYADSVKG | RLAVAGSEAFDI |
| | | | | SEQ ID NO:4300 | SEQ ID NO:12312 | SEQ ID NO:20324 |
| iPS:392726 | 21-225_20B5 | NA | AGCTATAGCATGAAC | TCCATTAGTGGGAGTAG TAGTTACATATACTACGC AGACTCAGTGAAGGGC | GATCGTGGGAGCTAC |
| | | | | SEQ ID NO:4301 | SEQ ID NO:12313 | SEQ ID NO:20325 |
| | | AA | SYSMN | SISGSSSYIYYADSVKG | DRGSY |
| | | | | SEQ ID NO:4302 | SEQ ID NO:12314 | SEQ ID NO:20326 |
| iPS:392728 | 21-225_20F7 | NA | GACTATTACATGAGC | CACATTAGTAGTAGTGG TAGTACCATATACTACGC AGACTCTGTGAAGGGC | TATCGGAATAACCGGGGGTA CTTCGATCTC |
| | | | | SEQ ID NO:4303 | SEQ ID NO:12315 | SEQ ID NO:20327 |
| | | AA | DYYMS | HISSSGSTIYYADSVKG | YRNNRGYFDL |
| | | | | SEQ ID NO:4304 | SEQ ID NO:12316 | SEQ ID NO:20328 |

958

FIGURE 49

| iPS:392730 | | NA | AGCTATGCCATGAAC | GTTATTAGTGGTAGTGGT AGTAACACATACTACGC AGACTCCGTGAAGGGC | AGATATACCAGTGACTGGCA TGATGCTTTTGATATC |
|---|---|---|---|---|---|
| | 21-225_17A1 | | SEQ ID NO:4305 | SEQ ID NO:12317 | SEQ ID NO:20329 |
| | | AA | SYAMN | VISGSGSNTYYADSVKG | RYTSDWHDAFDI |
| | | | SEQ ID NO:4306 | SEQ ID NO:12318 | SEQ ID NO:20330 |
| iPS:392732 | | NA | GACTATGGCATGCAC | CTTATATGGTATGATGTA ACTAATAAATACTATGG AGACTCCGTGAAGGGC | GAGCTTGGCTGGTACGAGGA CTAC |
| | 21-225_17E5 | | SEQ ID NO:4307 | SEQ ID NO:12319 | SEQ ID NO:20331 |
| | | AA | DYGMH | LIWYDVTNKYYGDSVKG | ELGWYEDY |
| | | | SEQ ID NO:4308 | SEQ ID NO:12320 | SEQ ID NO:20332 |
| iPS:392734 | | NA | AGCTATGGCTTGAAC | TCCATTAGTGGTAGTGGT AGTCACATATCCTACGC GGACTCAGTGAAGGGC | GATCGGGGCAGTGGC |
| | 21-225_17D8 | | SEQ ID NO:4309 | SEQ ID NO:12321 | SEQ ID NO:20333 |
| | | AA | SYGLN | SISGSGSHISYADSVKG | DRGSG |
| | | | SEQ ID NO:4310 | SEQ ID NO:12322 | SEQ ID NO:20334 |
| iPS:392736 | | NA | AGCTATGCCATGAAC | GTTATTAGTGGTAGTGGT GGTAGCACATACTACGC AGACTCCGTGAAGGGC | AGGTATACCAGTGACTGGCA TGATGCTTTTGATATC |
| | 21-225_17B12 | | SEQ ID NO:4311 | SEQ ID NO:12323 | SEQ ID NO:20335 |
| | | AA | SYAMN | VISGSGGSTYYADSVKG | RYTSDWHDAFDI |
| | | | SEQ ID NO:4312 | SEQ ID NO:12324 | SEQ ID NO:20336 |
| iPS:392738 | | NA | AGCTATGGCATGCAC | ATTATATGGTATGATGG AAGTAATAAATACTATG CAGACTCCGTGAAGGGC | GATCTGAGTATGGGCGGTAT GGACGTC |
| | 21-225_18G4 | | | | |

FIGURE 49

| | | | SEQ ID NO:4313 | SEQ ID NO:12325 | SEQ ID NO:20337 |
|---|---|---|---|---|---|
| | | AA | SYGMH | IIWYDGSNKYYADSVKG | DLSMGGMDV |
| | | | SEQ ID NO:4314 | SEQ ID NO:12326 | SEQ ID NO:20338 |
| iPS:392740 | | NA | GACTATGGCATGCAC | GTTATATGGTATGATGTAACTAATAAATACTATGCAGACTCCGTGAAGGGC | GAGGTTGGCTGGTACGAGGACTAC |
| | 21-225_18H12 | | SEQ ID NO:4315 | SEQ ID NO:12327 | SEQ ID NO:20339 |
| | | AA | DYGMH | VIWYDVTNKYYADSVKG | EVGWYEDY |
| | | | SEQ ID NO:4316 | SEQ ID NO:12328 | SEQ ID NO:20340 |
| iPS:392742 | | NA | AATTATGTCATTCAC | GTTATATGGTATGATGGAAGTAATAAATACTATGCAGACTCCGTGAAGGGC | GAGAAGTATAGCAGCAGCTGGTACGACTACGGTATGGACGTC |
| | 21-225_20B2 | | SEQ ID NO:4317 | SEQ ID NO:12329 | SEQ ID NO:20341 |
| | | AA | NYVIH | VIWYDGSNKYYADSVKG | EKYSSSWYDYGMDV |
| | | | SEQ ID NO:4318 | SEQ ID NO:12330 | SEQ ID NO:20342 |
| iPS:392744 | | NA | AGCTATGGCATGCAC | GTTATTTGGTATGAAGAAAATAATCAATACTATGCAGACTCCGTGAAGGGC | GAACTGGGGGTTCCGGTCTGACTAC |
| | 21-225_20D5 | | SEQ ID NO:4319 | SEQ ID NO:12331 | SEQ ID NO:20343 |
| | | AA | SYGMH | VIWYEENNQYYADSVKG | ELGFRSDY |
| | | | SEQ ID NO:4320 | SEQ ID NO:12332 | SEQ ID NO:20344 |
| iPS:392746 | 21 225 20H7 | NA | AGCTATAGCATGAAC | TCCATTAGTGGTAGTAGTAGTTTCATATACTACGCAGACTCAGTGAAGGGC | GTAGCAGCTCTTGACTAC |

FIGURE 49

| | 21-225_20H7 | | SEQ ID NO:4321 | SEQ ID NO:12333 | SEQ ID NO:20345 |
|---|---|---|---|---|---|
| | | AA | SYSMN | SISGSSSFIYYADSVKG | VAALDY |
| | | | SEQ ID NO:4322 | SEQ ID NO:12334 | SEQ ID NO:20346 |
| iPS:392748 | 21-225_20A8 | NA | AGCTATAGCGTGAAC | TCCATTAGTAGTAGTAGT AGTTTCCTATACTACGCA GACTCAGTGAAGGGC | AACTGGGACTAC |
| | | | SEQ ID NO:4323 | SEQ ID NO:12335 | SEQ ID NO:20347 |
| | | AA | SYSVN | SISSSSSFLYYADSVKG | NWDY |
| | | | SEQ ID NO:4324 | SEQ ID NO:12336 | SEQ ID NO:20348 |
| iPS:392750 | 21-225_20A10 | NA | AGCGATGACATGCAC | GTTATATGGTTTGATGGA AGTAATAAATACTATGC AGACTCCGTGAAGGGC | GATCTAATAGCAGCAGCTGG TACGGTTGACTAC |
| | | | SEQ ID NO:4325 | SEQ ID NO:12337 | SEQ ID NO:20349 |
| | | AA | SDDMH | VIWFDGSNKYYADSVKG | DLIAAAGTVDY |
| | | | SEQ ID NO:4326 | SEQ ID NO:12338 | SEQ ID NO:20350 |
| iPS:392754 | 21-225_21D3 | NA | AGCTATGGCATGCAC | GTTATATCATATGATGGA AGTAATAAATACTATGC AGACTCCGTGAAGGGC | GGGGTATGGTTCGGGGACCT C |
| | | | SEQ ID NO:4327 | SEQ ID NO:12339 | SEQ ID NO:20351 |
| | | AA | SYGMH | VISYDGSNKYYADSVKG | GVWFGDL |
| | | | SEQ ID NO:4328 | SEQ ID NO:12340 | SEQ ID NO:20352 |
| iPS:392758 | 21-225_21G11 | NA | GACTATGGCATGCAC | GTTATATGGTATGATGTA ACTAATGAATACTATGC AGACTCCGTGAAGGGC | GAGCTTGGCTGGTACGAGGA CTAC |
| | | | SEQ ID NO:4329 | SEQ ID NO:12341 | SEQ ID NO:20353 |
| | | AA | DYGMH | VIWYDVTNEYYADSVKG | ELGWYEDY |
| | | | SEQ ID NO:4330 | SEQ ID NO:12342 | SEQ ID NO:20354 |

FIGURE 49

| iPS:392760 | | NA | AGCTATGCCATGAAC | ATTATTAGTGGTCGTGGT GTTAACACATTCTACGCA GACTCCGTGAAGGGC | CGTTTGGCAGTGGCTGGCTC GGAGGCTTTTGATATC |
|---|---|---|---|---|---|
| | 21-225_22G3 | | SEQ ID NO:4331 | SEQ ID NO:12343 | SEQ ID NO:20355 |
| | | AA | SYAMN | IISGRGVNTFYADSVKG | RLAVAGSEAFDI |
| | | | SEQ ID NO:4332 | SEQ ID NO:12344 | SEQ ID NO:20356 |
| iPS:392762 | | NA | AGCTATGCCATGAAC | GTTATTAGTCGTAGTGGT GGTTACACATACTACGC GGACTCCGTGAAGGGC | CGTTTAGCAGTGGCTGGCTC GGAGGCTTTTGATATC |
| | 21-225_22G5 | | SEQ ID NO:4333 | SEQ ID NO:12345 | SEQ ID NO:20357 |
| | | AA | SYAMN | VISRSGGYTYYADSVKG | RLAVAGSEAFDI |
| | | | SEQ ID NO:4334 | SEQ ID NO:12346 | SEQ ID NO:20358 |
| iPS:392764 | | NA | AGCTATGCCATGAAC | GTTATTAGTGGTAGTGGT GGTAACACATTCTACGC AGACTCCGTGAAGGGC | CGTATGGCAGTGGCTGGCTC GGAGGCTTTTGATATC |
| | 21-225_22G10 | | SEQ ID NO:4335 | SEQ ID NO:12347 | SEQ ID NO:20359 |
| | | AA | SYAMN | VISGSGGNTFYADSVKG | RMAVAGSEAFDI |
| | | | SEQ ID NO:4336 | SEQ ID NO:12348 | SEQ ID NO:20360 |
| iPS:392766 | | NA | AGCTATGCCATGAAC | GTTATTAGTGGTAGTGGT GGTACCACATACTTCGC AGACTCCGTGAAGGGC | AGAAATAGCAGTGGCTGGCA TGATGTTTTTGATATC |
| | 21-225_23H4 | | SEQ ID NO:4337 | SEQ ID NO:12349 | SEQ ID NO:20361 |
| | | AA | SYAMN | VISGSGGTTYFADSVKG | RNSSGWHDVFDI |
| | | | SEQ ID NO:4338 | SEQ ID NO:12350 | SEQ ID NO:20362 |
| iPS:392768 | | NA | AGTTATAGCATGAAC | TCCATCAGTGGCAGTGG TAGTCACATATACTACGC GGACTCAGTGAAGGGC | GATCGGGGCAGTGGC |
| | 21-225_20B8 | | SEQ ID NO:4339 | SEQ ID NO:12351 | SEQ ID NO:20363 |
| | | AA | SYSMN | SISGSGSHIYYADSVKG | DRGSG |
| | | | SEQ ID NO:4340 | SEQ ID NO:12352 | SEQ ID NO:20364 |

FIGURE 49

| iPS:392770 | | NA | AGCTATGCCATGAAC | GTTATTAGTGGTAGTGGT GGTACCACATACTACGC AGACTCCGTGAAGGGC | AGGTATACCAGTGACTGGCA TGATGCTTTTGATATC |
|---|---|---|---|---|---|
| | 21-225_20C10 | | SEQ ID NO:4341 | SEQ ID NO:12353 | SEQ ID NO:20365 |
| | | AA | SYAMN | VISGSGGTTYYADSVKG | RYTSDWHDAFDI |
| | | | SEQ ID NO:4342 | SEQ ID NO:12354 | SEQ ID NO:20366 |
| iPS:392772 | | NA | AGCTATGGCATGCAC | GTTATGTGGTATGATGA AAGTAATAAACACTATG CAGACTCCGTGAAGGGC | GAACTGGGGTTCCGGTTTGA CTAC |
| | 21-225_20E12 | | SEQ ID NO:4343 | SEQ ID NO:12355 | SEQ ID NO:20367 |
| | | AA | SYGMH | VMWYDESNKHYADSVKG | ELGFRFDY |
| | | | SEQ ID NO:4344 | SEQ ID NO:12356 | SEQ ID NO:20368 |
| iPS:392774 | | NA | AGAAGTAGTTACTACTGGGG C | AGCATCTATTATAGTGG GAGCACCTACTACAACC CGTCCCTCAAGAGT | CTTAGCAGCAGCTGGGACTT CCAGCAC |
| | 21-225_21F3 | | SEQ ID NO:4345 | SEQ ID NO:12357 | SEQ ID NO:20369 |
| | | AA | RSSYYWG | SIYYSGSTYYNPSLKS | LSSSWDFQH |
| | | | SEQ ID NO:4346 | SEQ ID NO:12358 | SEQ ID NO:20370 |
| iPS:392776 | | NA | AGCTATAGCATGAAC | TCCATTAGTGGTAGTAGT AGTTACATATACTACGC AGACTCAGTGAAGGGC | GCGGCTGGCTTTGACTAC |
| | 21-225_21A12 | | SEQ ID NO:4347 | SEQ ID NO:12359 | SEQ ID NO:20371 |
| | | AA | SYSMN | SISGSSSYIYYADSVKG | AAGFDY |
| | | | SEQ ID NO:4348 | SEQ ID NO:12360 | SEQ ID NO:20372 |
| iPS:392778 | | NA | AGCTATAGCATGAAC | TCCATTAGTAGTAGTAGT AGTTACATATACTACGC AGACTCAGTGAAGGGC | GATAGGGGCAGCCTC |
| | 21-225_22H3 | | SEQ ID NO:4349 | SEQ ID NO:12361 | SEQ ID NO:20373 |

FIGURE 49

| | | | | | |
|---|---|---|---|---|---|
| | | AA | SYSMN | SISSSSSYIYYADSVKG | DRGSL |
| | | | SEQ ID NO:4350 | SEQ ID NO:12362 | SEQ ID NO:20374 |
| iPS:392780 | 21-225_22B7 | NA | AGCTATGGCATGCAC | GTTATTTGGTATGAAGAAAATAATCAATACTATGCAGACTCCGTGAAGGGC | GAAGTGGGGGTTCCGGTCTGACTAC |
| | | | SEQ ID NO:4351 | SEQ ID NO:12363 | SEQ ID NO:20375 |
| | | AA | SYGMH | VIWYEENNQYYADSVKG | EVGFRSDY |
| | | | SEQ ID NO:4352 | SEQ ID NO:12364 | SEQ ID NO:20376 |
| iPS:392782 | 21-225_22B12 | NA | AGCTATAGCATGAAC | TCCATTAGTGGTAGTAGTAGTTACACATACTACGCAGACTCAGTGAAGGGC | GTAGCAGCCCTTGACTCC |
| | | | SEQ ID NO:4353 | SEQ ID NO:12365 | SEQ ID NO:20377 |
| | | AA | SYSMN | SISGSSSYTYYADSVKG | VAALDS |
| | | | SEQ ID NO:4354 | SEQ ID NO:12366 | SEQ ID NO:20378 |
| iPS:392784 | 21-225_23C7 | NA | AGCTATGTTATGAGC | GCTATGAGTGGTAGTGGTGGTAGCACATATTATGTAGACTCCGTGAAGGGC | ACTGGGGTCTTTGACTAC |
| | | | SEQ ID NO:4355 | SEQ ID NO:12367 | SEQ ID NO:20379 |
| | | AA | SYVMS | AMSGSGGSTYYVDSVKG | TGVFDY |
| | | | SEQ ID NO:4356 | SEQ ID NO:12368 | SEQ ID NO:20380 |
| iPS:392786 | 21-225_24E1 | NA | AATTATGATATCAAC | TGGATGCACCCTAACAGTGGTAACACAGGCTATGCACAGAGGTTCCAGGGC | AGCAGTGGCTGGGAGGTCTTTGACTAC |
| | | | SEQ ID NO:4357 | SEQ ID NO:12369 | SEQ ID NO:20381 |
| | | AA | NYDIN | WMHPNSGNTGYAQRFQG | SSGWEVFDY |
| | | | SEQ ID NO:4358 | SEQ ID NO:12370 | SEQ ID NO:20382 |

FIGURE 49

| iPS:392788 | | NA | AGCTATAGCATGAAC | TCCATTAGTGGCAGTAGT AGTTACATATACTACGC AGACTCAGTGAAGGCC | GACAGAGGCAGTCTC |
|---|---|---|---|---|---|
| | 21-225_20C8 | | SEQ ID NO:4359 | SEQ ID NO:12371 | SEQ ID NO:20383 |
| | | AA | SYSMN | SISGSSSYIYYADSVKA | DRGSL |
| | | | SEQ ID NO:4360 | SEQ ID NO:12372 | SEQ ID NO:20384 |
| iPS:392790 | | NA | GACTATGGCATGCAC | GTTATATGGTTTGATGGA AGTAATAAATACTATGT AGACTCCGTGAAGGGC | GATCTTGGCTGGACGGAAGA GTAC |
| | 21-225_20D10 | | SEQ ID NO:4361 | SEQ ID NO:12373 | SEQ ID NO:20385 |
| | | AA | DYGMH | VIWFDGSNKYYVDSVKG | DLGWTEEY |
| | | | SEQ ID NO:4362 | SEQ ID NO:12374 | SEQ ID NO:20386 |
| iPS:392792 | | NA | AGCTATAGCATGAAC | TCCATTAGCGGTAGTAGT AGTTACATCTACTACGCA GACTCACTGAAGGGC | GATCGTGGGAGCTAC |
| | 21-225_20G12 | | SEQ ID NO:4363 | SEQ ID NO:12375 | SEQ ID NO:20387 |
| | | AA | SYSMN | SISGSSSYIYYADSLKG | DRGSY |
| | | | SEQ ID NO:4364 | SEQ ID NO:12376 | SEQ ID NO:20388 |
| iPS:392794 | | NA | AGGAGTAGTTACTACTGGGG C | AATATTTATTATAGTGGG AGCACCTACGACAACCC GTCCCTCAAGAGT | CATGGAAAAGACTGGGGCCT TGACTAC |
| | 21-225_21H3 | | SEQ ID NO:4365 | SEQ ID NO:12377 | SEQ ID NO:20389 |
| | | AA | RSSYYWG | NIYYSGSTYDNPSLKS | HGKDWGLDY |
| | | | SEQ ID NO:4366 | SEQ ID NO:12378 | SEQ ID NO:20390 |
| iPS:392796 | | NA | GACTATGGCATACAC | GTTATATGGTTTGATGGA AGTAATAAATACTATGC AGACTCCGTGAAGGGC | GATCTTGGCTGGACGGAAGA GTAC |
| | 21-225_22A4 | | SEQ ID NO:4367 | SEQ ID NO:12379 | SEQ ID NO:20391 |

FIGURE 49

| | | | AA | DYGIH | VIWFDGSNKYYADSVKG | DLGWTEEY |
|---|---|---|---|---|---|---|
| | | | | SEQ ID NO:4368 | SEQ ID NO:12380 | SEQ ID NO:20392 |
| iPS:392798 | | NA | | AGCTATGGCATGCAC | GTTATATGGCATGATGG AAGTAATAAATACTATG CAGACTCCGTGAAGGGC | GATCTGAGTATGGGCGGTAT GGACGTC |
| | 21-225_22C7 | | | SEQ ID NO:4369 | SEQ ID NO:12381 | SEQ ID NO:20393 |
| | | AA | | SYGMH | VIWHDGSNKYYADSVKG | DLSMGGMDV |
| | | | | SEQ ID NO:4370 | SEQ ID NO:12382 | SEQ ID NO:20394 |
| iPS:392800 | | NA | | AGGAGTAGTTACTACTGGGG C | AATATCTATTATAGTGGG ACCACCTCCTACAACCC GTCCCTCAAGAGT | CTCAGCAGCAGCTGGTCCGT TGACTAC |
| | 21-225_22D12 | | | SEQ ID NO:4371 | SEQ ID NO:12383 | SEQ ID NO:20395 |
| | | AA | | RSSYYWG | NIYYSGTTSYNPSLKS | LSSSWSVDY |
| | | | | SEQ ID NO:4372 | SEQ ID NO:12384 | SEQ ID NO:20396 |
| iPS:392802 | | NA | | AGCTATGCCATGAAC | GCTATTAGTGGTAGTGGT GGTTTCACATACTACGCA GACTCCGTGAAGGGC | ACCAGTGGCTTTGACTAC |
| | 21-225_23E7 | | | SEQ ID NO:4373 | SEQ ID NO:12385 | SEQ ID NO:20397 |
| | | AA | | SYAMN | AISGSGGFTYYADSVKG | TSGFDY |
| | | | | SEQ ID NO:4374 | SEQ ID NO:12386 | SEQ ID NO:20398 |
| iPS:392806 | | NA | | AGCTATGGCATGCAC | GTTATATGGTATGATGG AAGTAATAAATACTATG CAGACTCCGTGAAGGGC | GTAGCAGTGGCAGGCGGTAT GGACGTC |
| | 21-225_24H3 | | | SEQ ID NO:4375 | SEQ ID NO:12387 | SEQ ID NO:20399 |
| | | AA | | SYGMH | VIWYDGSNKYYADSVKG | VAVAGGMDV |
| | | | | SEQ ID NO:4376 | SEQ ID NO:12388 | SEQ ID NO:20400 |

FIGURE 49

| iPS:392808 | | NA | AGCTATGCCATGAGC | GTTATTAGTGGTAGTGGT GGTAGCACATATTACGC AGACTCCGTGAAGGGC | AGGTATAACAGTGGCTGGCA TGATGTTTTTGATATC |
|---|---|---|---|---|---|
| | 21-225_20F8 | | SEQ ID NO:4377 | SEQ ID NO:12389 | SEQ ID NO:20401 |
| | | AA | SYAMS | VISGSGGSTYYADSVKG | RYNSGWHDVFDI |
| | | | SEQ ID NO:4378 | SEQ ID NO:12390 | SEQ ID NO:20402 |
| iPS:392810 | | NA | AACTATGGCATGCAC | GTTATATGGTATGATGA AAATAATAAATACTATG TAGACTCCGTGAAGGGC | GAGTTGGGGGTTCCGGTCTGA CTAC |
| | 21-225_20H12 | | SEQ ID NO:4379 | SEQ ID NO:12391 | SEQ ID NO:20403 |
| | | AA | NYGMH | VIWYDENNKYYVDSVKG | ELGFRSDY |
| | | | SEQ ID NO:4380 | SEQ ID NO:12392 | SEQ ID NO:20404 |
| iPS:392812 | | NA | AGCTATGCCATGAAC | GTTATTAGTGGTCGTGGT GGTAACACATTCTACGC AGACTCCGTGAAGGGC | CGTTTGGCAGTGGCTGGCTC GGAGGCTTGTGATATC |
| | 21-225_21F4 | | SEQ ID NO:4381 | SEQ ID NO:12393 | SEQ ID NO:20405 |
| | | AA | SYAMN | VISGRGGNTFYADSVKG | RLAVAGSEACDI |
| | | | SEQ ID NO:4382 | SEQ ID NO:12394 | SEQ ID NO:20406 |
| iPS:392814 | | NA | AGCTATGGCATGCAC | GTTATGTGGTATGATGG AAGTAATAAATATTATG CAGACTCCGTGAAGGGC | GATGGGGGGATTTTTGGAGTG GTTAGACTAC |
| | 21-225_22A1 | | SEQ ID NO:4383 | SEQ ID NO:12395 | SEQ ID NO:20407 |
| | | AA | SYGMH | VMWYDGSNKYYADSVK G | DGGFLEWLDY |
| | | | SEQ ID NO:4384 | SEQ ID NO:12396 | SEQ ID NO:20408 |
| iPS:392816 | 21 225 22E4 | NA | AGCTATGCCATGAGC | ATTATTAGTGGTCGTGGT ACTAACACATTCTACGC AGACTCCGTGAAGGGC | CGTATAGCAGTGGCTGGCTC GGAGGCTTTTGATATC |

FIGURE 49

| | | | SEQ ID NO:4385 | SEQ ID NO:12397 | SEQ ID NO:20409 |
|---|---|---|---|---|---|
| | 21-225_22E4 | AA | SYAMS | IISGRGTNTFYADSVKG | RIAVAGSEAFDI |
| | | | SEQ ID NO:4386 | SEQ ID NO:12398 | SEQ ID NO:20410 |
| iPS:392818 | 21-225_22D8 | NA | AGCTATGGCATGCAC | ATTATATCATATGATGGA AGTAATAAATACTATGC AGACTCCGTGAAGGGC | GGGGTTTGGTTCGGGGACTT C |
| | | | SEQ ID NO:4387 | SEQ ID NO:12399 | SEQ ID NO:20411 |
| | | AA | SYGMH | IISYDGSNKYYADSVKG | GVWFGDF |
| | | | SEQ ID NO:4388 | SEQ ID NO:12400 | SEQ ID NO:20412 |
| iPS:392820 | 21-225_23D1 | NA | AGAAGTAGTTACTACTGGGG C | AGTATCTATTATAGTGGG AGCGCCCAGTACAACCC GTCCCTCAAGAGT | CTGAGCAGCAGCTGGTCTTT TGACTAC |
| | | | SEQ ID NO:4389 | SEQ ID NO:12401 | SEQ ID NO:20413 |
| | | AA | RSSYYWG | SIYYSGSAQYNPSLKS | LSSSWSFDY |
| | | | SEQ ID NO:4390 | SEQ ID NO:12402 | SEQ ID NO:20414 |
| iPS:392822 | 21-225_23C8 | NA | AGGAGTAGTTACTACTGGGG C | AATATTTATTATAGTGGG ACCACTTACAACAACCC GTCCCTCAAGAGT | CATGGAAAAGACTGGGGCCT TGACTAC |
| | | | SEQ ID NO:4391 | SEQ ID NO:12403 | SEQ ID NO:20415 |
| | | AA | RSSYYWG | NIYYSGTTYNNPSLKS | HGKDWGLDY |
| | | | SEQ ID NO:4392 | SEQ ID NO:12404 | SEQ ID NO:20416 |
| iPS:392824 | 21-225_24E5 | NA | AGGAGTAGTTACTACTGGGG C | AGTATCTATTATAGTGGG AGCGCCAACTACAACCC GTCCCTCAAGAGT | CTCAGCAGCAGCTGGTCCAT TGACAAC |
| | | | SEQ ID NO:4393 | SEQ ID NO:12405 | SEQ ID NO:20417 |
| | | AA | RSSYYWG | SIYYSGSANYNPSLKS | LSSSWSIDN |
| | | | SEQ ID NO:4394 | SEQ ID NO:12406 | SEQ ID NO:20418 |

FIGURE 49

| iPS:392826 | | NA | AGCTATAGCATGAAC | TACATTAGTAGTAGTAGT AGTACCATATACTATGC AGACTCTGTGAAGGGC | TCACTATGGTCCCCCTTTGAC TAC |
|---|---|---|---|---|---|
| | 21-225_20B9 | | SEQ ID NO:4395 | SEQ ID NO:12407 | SEQ ID NO:20419 |
| | | AA | SYSMN | YISSSSSTIYYADSVKG | SLWSPFDY |
| | | | SEQ ID NO:4396 | SEQ ID NO:12408 | SEQ ID NO:20420 |
| iPS:392830 | | NA | AGTTACTTCTGGAGC | CGTATCTATACCAGTGG GATCACCAACTACAACC CCTCCCTCAAGAGT | GGCCCGACTTCGGGGTGGTT CGACCCC |
| | 21-225_21A5 | | SEQ ID NO:4397 | SEQ ID NO:12409 | SEQ ID NO:20421 |
| | | AA | SYFWS | RIYTSGITNYNPSLKS | GPTSGWFDP |
| | | | SEQ ID NO:4398 | SEQ ID NO:12410 | SEQ ID NO:20422 |
| iPS:392832 | | NA | GACTATGGCATGCAC | GTTATATGGTTTGATGGA AGTAATAAATACTATGC AGACTCCGTGAAGGGC | GATCTTGGCTGGACGGAAGA GTAC |
| | 21-225_21H8 | | SEQ ID NO:4399 | SEQ ID NO:12411 | SEQ ID NO:20423 |
| | | AA | DYGMH | VIWFDGSNKYYADSVKG | DLGWTEEY |
| | | | SEQ ID NO:4400 | SEQ ID NO:12412 | SEQ ID NO:20424 |
| iPS:392834 | | NA | AGGAGTAGTTACTACTGGGG C | AATATCTATTATAGTGGG GCCACCTATTATAATTCG TCCCTCAAGAGT | CATAGCGGCAGCTGGTCCCT TGACTAC |
| | 21-225_22C1 | | SEQ ID NO:4401 | SEQ ID NO:12413 | SEQ ID NO:20425 |
| | | AA | RSSYYWG | NIYYSGATYYNSSLKS | HSGSWSLDY |
| | | | SEQ ID NO:4402 | SEQ ID NO:12414 | SEQ ID NO:20426 |
| iPS:392836 | | NA | GACTATGGCATGCAC | GTTATATGGTATGATGG AAGTAATAAATACTATG TAGACTCCGTGAAGGGC | GAAAAGTATAGCAGCAGCTG GTACGACTACGGTATGGACG TC |
| | 21-225_22F4 | | SEQ ID NO:4403 | SEQ ID NO:12415 | SEQ ID NO:20427 |

**FIGURE 49**

| | | | | | |
|---|---|---|---|---|---|
| | | AA | DYGMH | VIWYDGSNKYYVDSVKG | EKYSSSWYDYGMDV |
| | | | SEQ ID NO:4404 | SEQ ID NO:12416 | SEQ ID NO:20428 |
| iPS:392838 | 21-225_22G8 | NA | AGGAGTAGTTACTACTGGGGC | AATATCTATTATAGTGGGAGCACCTACTACAACCCGTCCGTCAAGAGT | CATGGAAAAGACTGGGGCCTTGACTAC |
| | | | SEQ ID NO:4405 | SEQ ID NO:12417 | SEQ ID NO:20429 |
| | | AA | RSSYYWG | NIYYSGSTYYNPSVKS | HGKDWGLDY |
| | | | SEQ ID NO:4406 | SEQ ID NO:12418 | SEQ ID NO:20430 |
| iPS:392840 | 21-225_23G1 | NA | AGCTATGCCATGAGC | GTTATTAGTGGTAGTGGTGGTACCACATATAACACAGACTCCGTGAAGGGC | AGCTCCTTGTTTGACTAC |
| | | | SEQ ID NO:4407 | SEQ ID NO:12419 | SEQ ID NO:20431 |
| | | AA | SYAMS | VISGSGGTTYNTDSVKG | SSLFDY |
| | | | SEQ ID NO:4408 | SEQ ID NO:12420 | SEQ ID NO:20432 |
| iPS:392842 | 21-225_23G8 | NA | AGCTATGCCATGAGC | GCTATTAGTGGTAGTGGTGGTAGCACATACTACGCAGACTCCGTGAAGGGC | AGCAGTGGCTGGTTCGCC |
| | | | SEQ ID NO:4409 | SEQ ID NO:12421 | SEQ ID NO:20433 |
| | | AA | SYAMS | AISGSGGSTYYADSVKG | SSGWFA |
| | | | SEQ ID NO:4410 | SEQ ID NO:12422 | SEQ ID NO:20434 |
| iPS:392844 | 21-225_23E11 | NA | TCCTATACCATGAAC | TCCATTAGTGGTAGTAGTAGTTACATATGGTATGTAGACTCAGTGAAGGGC | GATCGGGGCAGTCTC |
| | | | SEQ ID NO:4411 | SEQ ID NO:12423 | SEQ ID NO:20435 |
| | | AA | SYTMN | SISGSSSYIWYVDSVKG | DRGSL |
| | | | SEQ ID NO:4412 | SEQ ID NO:12424 | SEQ ID NO:20436 |

FIGURE 49

| iPS:392846 | | NA | AACTATGTCATGCAC | GTTATATGGTATGATGG AAGTAATAAATACTATG CAGACTCCGTGAAGGGC | GAGGAATATAGTAGTGGCTG GTACGACTACGGTATGGACG TC |
|---|---|---|---|---|---|
| | 21-225_24B6 | | SEQ ID NO:4413 | SEQ ID NO:12425 | SEQ ID NO:20437 |
| | | AA | NYVMH | VIWYDGSNKYYADSVKG | EEYSSGWYDYGMDV |
| | | | SEQ ID NO:4414 | SEQ ID NO:12426 | SEQ ID NO:20438 |
| iPS:392848 | | NA | AGCTATAGCATGAAC | TCCATTAGTAGTAGTAGT AGTTACATATACTACGC AGACTCAGTGAAGGGC | GATCGTGGGAGCTGC |
| | 21-225_20F9 | | SEQ ID NO:4415 | SEQ ID NO:12427 | SEQ ID NO:20439 |
| | | AA | SYSMN | SISSSSSYIYYADSVKG | DRGSC |
| | | | SEQ ID NO:4416 | SEQ ID NO:12428 | SEQ ID NO:20440 |
| iPS:392850 | | NA | ACCTATAGCATGAAC | TCCATTAGTAGTAGTAGT AGTTACATATACTACGC AGACTCAGTGAAGGGC | GATCGTGGGAGCCTC |
| | 21-225_20H10 | | SEQ ID NO:4417 | SEQ ID NO:12429 | SEQ ID NO:20441 |
| | | AA | TYSMN | SISSSSSYIYYADSVKG | DRGSL |
| | | | SEQ ID NO:4418 | SEQ ID NO:12430 | SEQ ID NO:20442 |
| iPS:392852 | | NA | AGCTATGCCATGAAC | ATTATTAGTGGTCGTGGT GGTAACACATTCTACGC AGACTCCGTGAAGGGC | CGTTTGGCAGTGGCTGGCTC GGAGGCTTTTGATATC |
| | 21-225_21A2 | | SEQ ID NO:4419 | SEQ ID NO:12431 | SEQ ID NO:20443 |
| | | AA | SYAMN | IISGRGGNTFYADSVKG | RLAVAGSEAFDI |
| | | | SEQ ID NO:4420 | SEQ ID NO:12432 | SEQ ID NO:20444 |
| iPS:392854 | | NA | GACTATGGCATGCAC | GTTATATGGTATGATGA AAGTAATAAATACTATG CAGACTCCGTGAAGGGC | GAACTGGGGTTCCGGTCTGA CTAT |
| | 21 225 21E5 | | | | |

FIGURE 49

| | 21-225_21E5 | | SEQ ID NO:4421 | SEQ ID NO:12433 | SEQ ID NO:20445 |
|---|---|---|---|---|---|
| | | AA | DYGMH | VIWYDESNKYYADSVKG | ELGFRSDY |
| | | | SEQ ID NO:4422 | SEQ ID NO:12434 | SEQ ID NO:20446 |
| iPS:392856 | 21-225_22A2 | NA | AGCTATGCCATGAGC | GGTATTAGTGGTAGTGGAGGTAACACACCCTACGCAGACTCCGTGAAGGGC | GTAGTGGGAGCTGTCCAC |
| | | | SEQ ID NO:4423 | SEQ ID NO:12435 | SEQ ID NO:20447 |
| | | AA | SYAMS | GISGSGGNTPYADSVKG | VVGAVH |
| | | | SEQ ID NO:4424 | SEQ ID NO:12436 | SEQ ID NO:20448 |
| iPS:392858 | 21-225_22H4 | NA | AGGAGTAGTTACTACTGGGGC | AATATTTATTATAGTGGGAGCACCTACCACAACCCGTCTCTCAAGAGT | CATGGAAAAGACTGGGGCCTTGACTAC |
| | | | SEQ ID NO:4425 | SEQ ID NO:12437 | SEQ ID NO:20449 |
| | | AA | RSSYYWG | NIYYSGSTYHNPSLKS | HGKDWGLDY |
| | | | SEQ ID NO:4426 | SEQ ID NO:12438 | SEQ ID NO:20450 |
| iPS:392860 | 21-225_22H8 | NA | AGCTATGGCATGCAC | GTTATCTGGTATGATGGAAATAATAAATACTATGCAGACTCCGTGAAGGGC | GAGCTTGCCTGGTACGAAGACTAC |
| | | | SEQ ID NO:4427 | SEQ ID NO:12439 | SEQ ID NO:20451 |
| | | AA | SYGMH | VIWYDGNNKYYADSVKG | ELAWYEDY |
| | | | SEQ ID NO:4428 | SEQ ID NO:12440 | SEQ ID NO:20452 |
| iPS:392864 | 21-225_23B9 | NA | AGTGGTGGTTACTACTGGAGC | TACATCTATTACAGTGGGAGCACCTACTACAACCCGTCCCTCAAGAGT | GAGGACGGTGCCTTCGGCTACTACGGTATGGACGTC |
| | | | SEQ ID NO:4429 | SEQ ID NO:12441 | SEQ ID NO:20453 |
| | | AA | SGGYYWS | YIYYSGSTYYNPSLKS | EDGAFGYYGMDV |

FIGURE 49

| | | | SEQ ID NO:4430 | SEQ ID NO:12442 | SEQ ID NO:20454 |
|---|---|---|---|---|---|
| iPS:392866 | | NA | AGCTATGGCATGCAC | GTTATATGGTATGATGA AAATAATAAATACTATG TAGACTCCGTGAAGGGC | GAATTGGGGTTCCGGTCTGA CTAC |
| | 21-225_23H11 | | SEQ ID NO:4431 | SEQ ID NO:12443 | SEQ ID NO:20455 |
| | | AA | SYGMH | VIWYDENNKYYVDSVKG | ELGFRSDY |
| | | | SEQ ID NO:4432 | SEQ ID NO:12444 | SEQ ID NO:20456 |
| iPS:392868 | | NA | AGCTATGGCATGCAC | ATTATATCATATGCTGGA AGTAATAAATCCTATGC AGACTCCGTGAAGGGC | CGGGGATACAGCTATGGCGG GTACGGTATGGACGTC |
| | 21-225_24D6 | | SEQ ID NO:4433 | SEQ ID NO:12445 | SEQ ID NO:20457 |
| | | AA | SYGMH | IISYAGSNKSYADSVKG | RGYSYGGYGMDV |
| | | | SEQ ID NO:4434 | SEQ ID NO:12446 | SEQ ID NO:20458 |
| iPS:392870 | | NA | AGGAGTAGTTACTACTGGGG C | AGTATCTATTATAGTGGG AGCGCCTCTTACAACCC GTCCCTCAAGAGT | CTGAGCAGCAGCTGGTCTTT TGACTAC |
| | 21-225_20G9 | | SEQ ID NO:4435 | SEQ ID NO:12447 | SEQ ID NO:20459 |
| | | AA | RSSYYWG | SIYYSGSASYNPSLKS | LSSSWSFDY |
| | | | SEQ ID NO:4436 | SEQ ID NO:12448 | SEQ ID NO:20460 |
| iPS:392872 | | NA | AGCTATGTCATGCAC | GTTATATGGTATGATGG AAGTAATAAATACTATG CAGACTCCGTGAAGGTC | GAGAGATATACCAGTGGCTG GTATGACTACGGTATGGACG TC |
| | 21-225_20B11 | | SEQ ID NO:4437 | SEQ ID NO:12449 | SEQ ID NO:20461 |
| | | AA | SYVMH | VIWYDGSNKYYADSVKV | ERYTSGWYDYGMDV |
| | | | SEQ ID NO:4438 | SEQ ID NO:12450 | SEQ ID NO:20462 |

FIGURE 49

| iPS:392874 | | NA | AACTATGCCATGAGC | GTTCTTAGTGGTAGTGGT GGTAGCACATTCTACGC AGACTCCGTGAAGGGC | TATTGTAGTAGTGCCAGGTG CCCTTATGATGCCTTTGATAT C |
|---|---|---|---|---|---|
| | 21-225_21D2 | | SEQ ID NO:4439 | SEQ ID NO:12451 | SEQ ID NO:20463 |
| | | AA | NYAMS | VLSGSGGSTFYADSVKG | YCSSARCPYDAFDI |
| | | | SEQ ID NO:4440 | SEQ ID NO:12452 | SEQ ID NO:20464 |
| iPS:392876 | | NA | GACTATGGCATGCAC | GTTATATGGTTTGATGGA AATAATAAATACTATGT AGACTCCGTGAAGGGC | GATCTTGGCTGGACGGAAGA GTAC |
| | 21-225_21F7 | | SEQ ID NO:4441 | SEQ ID NO:12453 | SEQ ID NO:20465 |
| | | AA | DYGMH | VIWFDGNNKYYVDSVKG | DLGWTEEY |
| | | | SEQ ID NO:4442 | SEQ ID NO:12454 | SEQ ID NO:20466 |
| iPS:392878 | | NA | AGCTATGCCATGAGC | ATTATTAGTGGTAGTGGT GGTTACACATACTACGC GGACTCCGTGAAGGGC | CGTATAGCAGTGGCTGGCTC GGAGGCTTTTGATATC |
| | 21-225_22C5 | | SEQ ID NO:4443 | SEQ ID NO:12455 | SEQ ID NO:20467 |
| | | AA | SYAMS | IISGSGGYTYYADSVKG | RIAVAGSEAFDI |
| | | | SEQ ID NO:4444 | SEQ ID NO:12456 | SEQ ID NO:20468 |
| iPS:392880 | | NA | AGCTATGGCATGCAC | GTTATATGGTATGAGGA AAATAATAAAGACTATG TAGACTCCGTGAAGGGC | GAGTTAGGCTTCCAGTCTGA CTAC |
| | 21-225_22F9 | | SEQ ID NO:4445 | SEQ ID NO:12457 | SEQ ID NO:20469 |
| | | AA | SYGMH | VIWYEENNKDYVDSVKG | ELGFQSDY |
| | | | SEQ ID NO:4446 | SEQ ID NO:12458 | SEQ ID NO:20470 |

FIGURE 49

| iPS:392882 | | NA | AGGAGTAGTTACTACTGGGGC | AATATTTATTATAGTGGGAGCACCTACAACAACCCGTCCCTCAAGAGT | CATGGAAAAGACTGGGGCCTTGACTTC |
|---|---|---|---|---|---|
| | 21-225_23A3 | | SEQ ID NO:4447 | SEQ ID NO:12459 | SEQ ID NO:20471 |
| | | AA | RSSYYWG | NIYYSGSTYNNPSLKS | HGKDWGLDF |
| | | | SEQ ID NO:4448 | SEQ ID NO:12460 | SEQ ID NO:20472 |
| iPS:392884 | | NA | AGCTATGGCATGCAC | GTTATATGGTATGATGGAAGTAATAAATACTATGCAGACTCCGTGAAGGGC | GAAAGGTATAGCAGTGGCTGGCACGACTACGGTATGGACGTC |
| | 21-225_23A10 | | SEQ ID NO:4449 | SEQ ID NO:12461 | SEQ ID NO:20473 |
| | | AA | SYGMH | VIWYDGSNKYYADSVKG | ERYSSGWHDYGMDV |
| | | | SEQ ID NO:4450 | SEQ ID NO:12462 | SEQ ID NO:20474 |
| iPS:392886 | | NA | AATTATGATATCAAC | TGGATGCACCCTAACAGTGGTAACACAGGCTATGCACAGAAGTTCCAGGGC | AGCAGTGGCTGGTACTACTTTGACTAC |
| | 21-225_23A12 | | SEQ ID NO:4451 | SEQ ID NO:12463 | SEQ ID NO:20475 |
| | | AA | NYDIN | WMHPNSGNTGYAQKFQG | SSGWYYFDY |
| | | | SEQ ID NO:4452 | SEQ ID NO:12464 | SEQ ID NO:20476 |
| iPS:392888 | | NA | AGCTATGGCATGCAC | GTTATATGGTATGATGGAAGTAATAAATACTATGCAGACTCCGTGAAGGGC | AGGTATAGCAGCAGCTGGTCAGGGGGGTATGGACGTC |
| | 21-225_25A2 | | SEQ ID NO:4453 | SEQ ID NO:12465 | SEQ ID NO:20477 |
| | | AA | SYGMH | VIWYDGSNKYYADSVKG | RYSSSWSGGMDV |
| | | | SEQ ID NO:4454 | SEQ ID NO:12466 | SEQ ID NO:20478 |

975

FIGURE 49

| iPS:392890 | | NA | AGCTATGCCATGAGC | GCTATTAGTGGTAGTGGTGGTTACACATACTACGCAGACTCCGTGAAGGGC | GGGGGGTCCCTCTTCTAC |
|---|---|---|---|---|---|
| | 21-225_20H9 | | SEQ ID NO:4455 | SEQ ID NO:12467 | SEQ ID NO:20479 |
| | | AA | SYAMS | AISGSGGYTYYADSVKG | GGSLFY |
| | | | SEQ ID NO:4456 | SEQ ID NO:12468 | SEQ ID NO:20480 |
| iPS:392892 | | NA | AGCTATGCCATGAGC | ACTATTAGTGGTCGTGGTGGTCACACATACTACGCAGACTCCGTGAAGGGC | CAGGACTGC |
| | 21-225_20C11 | | SEQ ID NO:4457 | SEQ ID NO:12469 | SEQ ID NO:20481 |
| | | AA | SYAMS | TISGRGGHTYYADSVKG | QDC |
| | | | SEQ ID NO:4458 | SEQ ID NO:12470 | SEQ ID NO:20482 |
| iPS:392894 | | NA | GACTATGGCATGCAC | GTTATATGGTATGATGTAACTAATAAATACTATGCAGACTCCGTGAAGGGC | GAGCTTGGCTGGTACGAGGACTAC |
| | 21-225_21G2 | | SEQ ID NO:4459 | SEQ ID NO:12471 | SEQ ID NO:20483 |
| | | AA | DYGMH | VIWYDVTNKYYADSVKG | ELGWYEDY |
| | | | SEQ ID NO:4460 | SEQ ID NO:12472 | SEQ ID NO:20484 |
| iPS:392896 | | NA | AGAAGTAGTTACTACTGGGGC | AATATCTATTATAGTGGGTATAGTTACTACAATCCGTCCCTCAAGAGT | CATAGCACCAGCTGGTCCCTTGACTAC |
| | 21-225_21G7 | | SEQ ID NO:4461 | SEQ ID NO:12473 | SEQ ID NO:20485 |
| | | AA | RSSYYWG | NIYYSGYSYYNPSLKS | HSTSWSLDY |
| | | | SEQ ID NO:4462 | SEQ ID NO:12474 | SEQ ID NO:20486 |
| iPS:392898 | | NA | AACGCCTGGATGAAC | CGTATTAAAAGCAAAACTGATGGTGGGACAACAGACTACGCTGCACCCGTGAAAGGC | GAAGGCTGGAACACGGACTAC |
| | 21 225 21H10 | | | | |

FIGURE 49

| | | | SEQ ID NO:4463 | SEQ ID NO:12475 | SEQ ID NO:20487 |
|---|---|---|---|---|---|
| | 21-225_21H10 | AA | NAWMN | RIKSKTDGGTTDYAAPVK G | EGWNTDY |
| | | | SEQ ID NO:4464 | SEQ ID NO:12476 | SEQ ID NO:20488 |
| iPS:392900 | 21-225_22F2 | NA | GACTATGGCATGCAC | GTTATATGGTATGAGGG AAGTAATAAATACTATG TAGACTCCGTGAGGGGC | GAGCTAGGCTTCCAGTCTGA CTAC |
| | | | SEQ ID NO:4465 | SEQ ID NO:12477 | SEQ ID NO:20489 |
| | | AA | DYGMH | VIWYEGSNKYYVDSVRG | ELGFQSDY |
| iPS:392902 | 21-225_22D5 | NA | AGCTATGCCATGAGC | GTTATTAGTGGTCGTGGT GGTAACACATTCTACGC AGACTCCGTGAAGGGC | CGTATAGCAGTGGCTGGCTC GGAGGCTTTTGATATC |
| | | | SEQ ID NO:4466 | SEQ ID NO:12478 | SEQ ID NO:20490 |
| | | | SEQ ID NO:4467 | SEQ ID NO:12479 | SEQ ID NO:20491 |
| | | AA | SYAMS | VISGRGGNTFYADSVKG | RIAVAGSEAFDI |
| iPS:392904 | 21-225_22G9 | NA | GGTAGTAATTACTACTGGGG C | AATATCTATTATAGTGGG AGCACCTACTACAACCC GTCCCTCAAGAGT | CATAGCAGTAGCTGGTCCCT TGACTAC |
| | | | SEQ ID NO:4468 | SEQ ID NO:12480 | SEQ ID NO:20492 |
| | | | SEQ ID NO:4469 | SEQ ID NO:12481 | SEQ ID NO:20493 |
| | | AA | GSNYYWG | NIYYSGSTYYNPSLKS | HSSSWSLDY |
| iPS:392908 | 21-225_23F12 | NA | AGCTATGGCATGCAC | GTTATATGGTATGATGA AACTAATAAATACTATG CAGACTCCGTGAAGGGC | GAGCTTGCCTGGTACGAGGA CTAC |
| | | | SEQ ID NO:4470 | SEQ ID NO:12482 | SEQ ID NO:20494 |
| | | | SEQ ID NO:4471 | SEQ ID NO:12483 | SEQ ID NO:20495 |
| | | AA | SYGMH | VIWYDETNKYYADSVKG | ELAWYEDY |

FIGURE 49

| | | | SEQ ID NO:4472 | SEQ ID NO:12484 | SEQ ID NO:20496 |
|---|---|---|---|---|---|
| iPS:392912 | | NA | AGCTATGGCATGCAC | GTTATATGGTATGATGTA ACTAATAAATACTATAC AGGCTCCGTGAAGGGC | GAAATTGGCTGGTTAGATGA CTAC |
| | 21-225_25A9 | | SEQ ID NO:4473 | SEQ ID NO:12485 | SEQ ID NO:20497 |
| | | AA | SYGMH | VIWYDVTNKYYTGSVKG | EIGWLDDY |
| | | | SEQ ID NO:4474 | SEQ ID NO:12486 | SEQ ID NO:20498 |
| iPS:392914 | | NA | AGCGATGGCATGCAC | GTTATATGGTATGATGG AAGTAATAAATACTATG CAGACTCCGTGAAGGGC | GAGAGGTATAGCAGCAGCTG GTACGACTACGGTATGGACG TC |
| | 21-225_25D12 | | SEQ ID NO:4475 | SEQ ID NO:12487 | SEQ ID NO:20499 |
| | | AA | SDGMH | VIWYDGSNKYYADSVKG | ERYSSSWYDYGMDV |
| | | | SEQ ID NO:4476 | SEQ ID NO:12488 | SEQ ID NO:20500 |
| iPS:392916 | | NA | AGCTATAGCATGAAC | TCCACTAGTAGTAGTGAT AGTTATATATACTACGCA GACTCAGTGAAGGGC | GTGGCGTCCTTTGACTGC |
| | 21-225_27C5 | | SEQ ID NO:4477 | SEQ ID NO:12489 | SEQ ID NO:20501 |
| | | AA | SYSMN | STSSSDSYIYYADSVKG | VASFDC |
| | | | SEQ ID NO:4478 | SEQ ID NO:12490 | SEQ ID NO:20502 |
| iPS:392918 | | NA | AGCTATGGCATGCAC | GTTATATGGTATGATGA AAATAATAAATACTATG CAGACTCCGTGAAGGGC | GAATTAGGCTGGTACGACGA CTAC |
| | 21-225_28F5 | | SEQ ID NO:4479 | SEQ ID NO:12491 | SEQ ID NO:20503 |
| | | AA | SYGMH | VIWYDENNKYYADSVKG | ELGWYDDY |

FIGURE 49

| | | | SEQ ID NO:4480 | SEQ ID NO:12492 | SEQ ID NO:20504 |
|---|---|---|---|---|---|
| iPS:392920 | | NA | GACTATGGCATACAC | GTTATATGGTATGATGA AAGTAATAAATACTATG CAGACTCCATGAAGGGC | GAACTGGGAATGACGGGTGA CTAC |
| | 21-225_29G4 | | SEQ ID NO:4481 | SEQ ID NO:12493 | SEQ ID NO:20505 |
| | | AA | DYGIH | VIWYDESNKYYADSMKG | ELGMTGDY |
| | | | SEQ ID NO:4482 | SEQ ID NO:12494 | SEQ ID NO:20506 |
| iPS:392922 | | NA | AGCTATGGCATGCAC | GTTATATGGTATGATGG AACTGATAAATACTATG TAGACTCCGTGAAGGGC | GAAAATAGCAGCTCGTACTA CTTTGACTAC |
| | 21-225_30G4 | | SEQ ID NO:4483 | SEQ ID NO:12495 | SEQ ID NO:20507 |
| | | AA | SYGMH | VIWYDGTDKYYVDSVKG | ENSSSYYFDY |
| | | | SEQ ID NO:4484 | SEQ ID NO:12496 | SEQ ID NO:20508 |
| iPS:392924 | | NA | AGCTATGGCATGCAC | GTTATATGGTATGATGG AAGTAATAAATACTATG CAGACTCCGTGAAGGGC | AGATATAGCAGCAGCTGGAC GGGGGGGTATGGACGTC |
| | 21-225_32H2 | | SEQ ID NO:4485 | SEQ ID NO:12497 | SEQ ID NO:20509 |
| | | AA | SYGMH | VIWYDGSNKYYADSVKG | RYSSSWTGGMDV |
| | | | SEQ ID NO:4486 | SEQ ID NO:12498 | SEQ ID NO:20510 |
| iPS:392928 | | NA | AATTATGATATTAAT | TGGATGTACCCTAACAG TGGTAACACAGGCTATG CACAGAAATTCCAGGGC | AGCAGTGGCTGGTACTACTT TGACTAC |
| | 21-225_25A4 | | SEQ ID NO:4487 | SEQ ID NO:12499 | SEQ ID NO:20511 |

FIGURE 49

| | | AA | NYDIN | WMYPNSGNTGYAQKFQG | SSGWYYFDY |
|---|---|---|---|---|---|
| | | | SEQ ID NO:4488 | SEQ ID NO:12500 | SEQ ID NO:20512 |
| iPS:392930 | 21-225_25H9 | NA | AACTATGGCATGCAC | ATTATATGGTATGATGG AAGTTATAAATACTATG CAGACTCCGTGAAGGGC | GAGGGATACGATTTTTGGAG TGGCTTCTTTGACTCC |
| | | | SEQ ID NO:4489 | SEQ ID NO:12501 | SEQ ID NO:20513 |
| | | AA | NYGMH | IIWYDGSYKYYADSVKG | EGYDFWSGFFDS |
| | | | SEQ ID NO:4490 | SEQ ID NO:12502 | SEQ ID NO:20514 |
| iPS:392934 | 21-225_27D5 | NA | AGCTATGGCATGCAC | GTTATATGGTATGATGA AAGTAATAAATACTATG GAGACTCCGTGAAGGGC | GAACTGGGGTTCCTCTCTGA CTAC |
| | | | SEQ ID NO:4491 | SEQ ID NO:12503 | SEQ ID NO:20515 |
| | | AA | SYGMH | VIWYDESNKYYGDSVKG | ELGFLSDY |
| | | | SEQ ID NO:4492 | SEQ ID NO:12504 | SEQ ID NO:20516 |
| iPS:392936 | 21-225_28B6 | NA | AATTATGATATTAAT | TGGATGCACCCTGACAG TGGTAACACAGGCTATG CACAGAAGTTCCAGGGC | AGCAGTGGCTGGTACTACTT TGACTAC |
| | | | SEQ ID NO:4493 | SEQ ID NO:12505 | SEQ ID NO:20517 |
| | | AA | NYDIN | WMHPDSGNTGYAQKFQG | SSGWYYFDY |
| | | | SEQ ID NO:4494 | SEQ ID NO:12506 | SEQ ID NO:20518 |
| iPS:392938 | 21-225_29H4 | NA | AGCTATGGCATACAC | GTTATATGGTATGATGG AAGTAATAAATACTATG CAGACTCCGTGAAGGGC | AGGTATAGCAGCAGCTGGTC AGGGGGTATGGACGTC |
| | | | SEQ ID NO:4495 | SEQ ID NO:12507 | SEQ ID NO:20519 |

FIGURE 49

| | | | AA | SYGIH | VIWYDGSNKYYADSVKG | RYSSSWSGGMDV |
|---|---|---|---|---|---|---|
| | | | | SEQ ID NO:4496 | SEQ ID NO:12508 | SEQ ID NO:20520 |
| iPS:392940 | | NA | | GACTATGGCATTCAC | GTTATATGGTATGATGA AAGTAATAACTACTATG CAGACTCCGTGAAGGGC | GAAATTGGCTGGTTAGATGA CTAC |
| | 21-225_29D9 | | | SEQ ID NO:4497 | SEQ ID NO:12509 | SEQ ID NO:20521 |
| | | AA | | DYGIH | VIWYDESNNYYADSVKG | EIGWLDDY |
| | | | | SEQ ID NO:4498 | SEQ ID NO:12510 | SEQ ID NO:20522 |
| iPS:392942 | | NA | | AGCTGTGCCATGAAC | GCTATTAGTGGTCGTGGT GGTAGCACATTCTACGC AGACTCCGTGAAGGGC | GGGGAGCTACTAGAGGACTA CTACTACTACGGAATGGACG TC |
| | 21-225_30E9 | | | SEQ ID NO:4499 | SEQ ID NO:12511 | SEQ ID NO:20523 |
| | | AA | | SCAMN | AISGRGGSTFYADSVKG | GELLEDYYYYGMDV |
| | | | | SEQ ID NO:4500 | SEQ ID NO:12512 | SEQ ID NO:20524 |
| iPS:392944 | | NA | | AGCTATGCCATGAGC | GCTATTAGTGGTCGTGGT GGAAGCATATTCCACGC AGACTCCGTGAAGGGC | GGGGAGCTACTAGAGGACTA CTACTTCTACGGTATGGACG TC |
| | 21-225_31H5 | | | SEQ ID NO:4501 | SEQ ID NO:12513 | SEQ ID NO:20525 |
| | | AA | | SYAMS | AISGRGGSIFHADSVKG | GELLEDYYFYGMDV |
| | | | | SEQ ID NO:4502 | SEQ ID NO:12514 | SEQ ID NO:20526 |
| iPS:392948 | | NA | | GACTATGGCATACAC | GTTATTTGGTATGATGGA AATAATAAATATTATGC AGACTCCGTGAAGGGC | GAAATTGGCTGGTTAGATGA CTAC |
| | 21-225_25G5 | | | SEQ ID NO:4503 | SEQ ID NO:12515 | SEQ ID NO:20527 |
| | | AA | | DYGIH | VIWYDGNNKYYADSVKG | EIGWLDDY |
| | | | | SEQ ID NO:4504 | SEQ ID NO:12516 | SEQ ID NO:20528 |

FIGURE 49

| iPS:392950 | | NA | AGCTATAGGATGAAC | TCCATTAGTAGTAGTAGT AGTACCATATACTACGC AGACTCTGTGAAGGGC | ACGGCTGGTTTTGACTAC |
|---|---|---|---|---|---|
| | 21-225_25C10 | | SEQ ID NO:4505 | SEQ ID NO:12517 | SEQ ID NO:20529 |
| | | AA | SYRMN | SISSSSSTIYYADSVKG | TAGFDY |
| | | | SEQ ID NO:4506 | SEQ ID NO:12518 | SEQ ID NO:20530 |
| iPS:392952 | | NA | AGCTATGGCATGAAC | TCCATTAGTGGTAGTAGT AGTTACATATACTACGC GGACTCAGTGAAGGGC | CTGACTACCTTTGACTTC |
| | 21-225_26G1 | | SEQ ID NO:4507 | SEQ ID NO:12519 | SEQ ID NO:20531 |
| | | AA | SYGMN | SISGSSSYIYYADSVKG | LTTFDF |
| | | | SEQ ID NO:4508 | SEQ ID NO:12520 | SEQ ID NO:20532 |
| iPS:392954 | | NA | AGCTATGCCATGAGC | GTTATTAGTGGTAGTGGT GTTAACACATTCTACGCA GACTCCGTGAAGGGC | AAGATAGCAGTGGCTGGTAC TCACTACTTTGACTAC |
| | 21-225_26A10 | | SEQ ID NO:4509 | SEQ ID NO:12521 | SEQ ID NO:20533 |
| | | AA | SYAMS | VISGSGVNTFYADSVKG | KIAVAGTHYFDY |
| | | | SEQ ID NO:4510 | SEQ ID NO:12522 | SEQ ID NO:20534 |
| iPS:392956 | | NA | AGCTATGGCATGCAC | GTTATATGGTATGATGG AAGTAATAAATACTATG CAGACTCCGTGAAGGGC | GATTCCTCCCCCTACGGTAT GGACGTC |
| | 21-225_27A11 | | SEQ ID NO:4511 | SEQ ID NO:12523 | SEQ ID NO:20535 |
| | | AA | SYGMH | VIWYDGSNKYYADSVKG | DSSPYGMDV |
| | | | SEQ ID NO:4512 | SEQ ID NO:12524 | SEQ ID NO:20536 |
| iPS:392958 | | NA | AGCTATGGCATGCAC | GTTATATGGTATGATGA AAGTAATAAATACTATG CAGACTCCGTGAAGGGC | GAATTAGGCTGGTACGACGA CTAC |
| | 21 225 28C7 | | | | |

FIGURE 49

| | | | SEQ ID NO:4513 | SEQ ID NO:12525 | SEQ ID NO:20537 |
|---|---|---|---|---|---|
| | 21-225_28C7 | AA | SYGMH | VIWYDESNKYYADSVKG | ELGWYDDY |
| | | | SEQ ID NO:4514 | SEQ ID NO:12526 | SEQ ID NO:20538 |
| iPS:392960 | | NA | AATTATGATATTAAT | TGGATGCACCCTAACAGTGGTAACACAGGCTATGCACAGAAATTCCAGGGC | AGCAGTGGCTGGTACTACTTTGACTAC |
| | 21-225_29E6 | | SEQ ID NO:4515 | SEQ ID NO:12527 | SEQ ID NO:20539 |
| | | AA | NYDIN | WMHPNSGNTGYAQKFQG | SSGWYYFDY |
| | | | SEQ ID NO:4516 | SEQ ID NO:12528 | SEQ ID NO:20540 |
| iPS:392962 | | NA | AGCTATGTCATGAAC | GCTATTAGTGGTAGTGGTGGTAGCACATACTACGCAGACTCCGTGAAGGGC | ACTGGGGTCTTTGACTAC |
| | 21-225_30A1 | | SEQ ID NO:4517 | SEQ ID NO:12529 | SEQ ID NO:20541 |
| | | AA | SYVMN | AISGSGGSTYYADSVKG | TGVFDY |
| | | | SEQ ID NO:4518 | SEQ ID NO:12530 | SEQ ID NO:20542 |
| iPS:392964 | | NA | AGCTATGCCATGAGC | GCTATTAGTGGTCGTGGTGGTAGCACATTCCACGCAGACTCCGTGAAGGGC | GGGGAGCTACTAGAGGACTACTACTTCTACGGTATGGACGTC |
| | 21-225_31A8 | | SEQ ID NO:4519 | SEQ ID NO:12531 | SEQ ID NO:20543 |
| | | AA | SYAMS | AISGRGGSTFHADSVKG | GELLEDYYFYGMDV |
| | | | SEQ ID NO:4520 | SEQ ID NO:12532 | SEQ ID NO:20544 |
| iPS:392966 | | NA | AGCTATAGCATGAAC | TCCATTAGTGGTAGTAGTAGTTACATATACTACGCAGACTCAGTGAAGGGC | GGCAATATAGCAAGGGACTAC |
| | 21-225_32G3 | | SEQ ID NO:4521 | SEQ ID NO:12533 | SEQ ID NO:20545 |
| | | AA | SYSMN | SISGSSSYIYYADSVKG | GNIARDY |
| | | | SEQ ID NO:4522 | SEQ ID NO:12534 | SEQ ID NO:20546 |

FIGURE 49

| iPS:392968 | | NA | AACTATGGCATGCAC | GTTATATGGTATGAGGA AAGTAATAAATACTATA CAGAGTCCGTGAAGGGC | GAACTGGGGTTCCTCTCTGA CTAC |
|---|---|---|---|---|---|
| | 21-225_25B6 | | SEQ ID NO:4523 | SEQ ID NO:12535 | SEQ ID NO:20547 |
| | | AA | NYGMH | VIWYEESNKYYTESVKG | ELGFLSDY |
| | | | SEQ ID NO:4524 | SEQ ID NO:12536 | SEQ ID NO:20548 |
| iPS:392972 | | NA | AGCTATGGCATGCAC | GTTATATGGTATGAAGG AAGTAATAAATACTATG TAGACTCCGTGAAGGGC | GAATTAGGCTGGTACGACGA CTAC |
| | 21-225_26A2 | | SEQ ID NO:4525 | SEQ ID NO:12537 | SEQ ID NO:20549 |
| | | AA | SYGMH | VIWYEGSNKYYVDSVKG | ELGWYDDY |
| | | | SEQ ID NO:4526 | SEQ ID NO:12538 | SEQ ID NO:20550 |
| iPS:392974 | | NA | AACTGTGTCATGCAC | GTTATATGGTATGATGG AAGTAATAAATACTATG CAGACTCCGTGAAGGGC | GAGGAGTATAGCAGTGGCTG GTACGACTACGGTATGGACG TC |
| | 21-225_26A11 | | SEQ ID NO:4527 | SEQ ID NO:12539 | SEQ ID NO:20551 |
| | | AA | NCVMH | VIWYDGSNKYYADSVKG | EEYSSGWYDYGMDV |
| | | | SEQ ID NO:4528 | SEQ ID NO:12540 | SEQ ID NO:20552 |
| iPS:392976 | | NA | AGCTATAGCCTGAAC | TCCATTAGTGGTAGTAGT AGTAACATATACTACAC AGACTCAGTGAAGGGC | GTGGCGTCCTTTGACTAC |
| | 21-225_27H12 | | SEQ ID NO:4529 | SEQ ID NO:12541 | SEQ ID NO:20553 |
| | | AA | SYSLN | SISGSSSNIYYTDSVKG | VASFDY |
| | | | SEQ ID NO:4530 | SEQ ID NO:12542 | SEQ ID NO:20554 |

FIGURE 49

| iPS:392978 | | NA | GACTATGGCATGCAC | GTTATATGGTATGATGCA AATAATAAATACTATGC AGACTCCGTGAAGGGC | GAAATTGGCTGGTTAGATGA CTAC |
|---|---|---|---|---|---|
| | **21-225_28B8** | | SEQ ID NO:4531 | SEQ ID NO:12543 | SEQ ID NO:20555 |
| | | AA | DYGMH | VIWYDANNKYYADSVKG | EIGWLDDY |
| | | | SEQ ID NO:4532 | SEQ ID NO:12544 | SEQ ID NO:20556 |
| iPS:392980 | | NA | GACTATGGCATGCAC | GTTATATGGTATAATGA AAATAATAAATACTATG CAGACTCCGTGAAGGGC | GAACTGGGGATGACGGGTGA CTCC |
| | **21-225_29H6** | | SEQ ID NO:4533 | SEQ ID NO:12545 | SEQ ID NO:20557 |
| | | AA | DYGMH | VIWYNENNKYYADSVKG | ELGMTGDS |
| | | | SEQ ID NO:4534 | SEQ ID NO:12546 | SEQ ID NO:20558 |
| iPS:392982 | | NA | AGCTATGCCATGAGC | GCTATTAGTGGTCGTGGT GGTAGCACATTCCACGC AGACTCCGTGAAGGGC | GGGGAGCTACTAGAGGACTA CTACTTCTACGGTTTGGACG TC |
| | **21-225_30D1** | | SEQ ID NO:4535 | SEQ ID NO:12547 | SEQ ID NO:20559 |
| | | AA | SYAMS | AISGRGGSTFHADSVKG | GELLEDYYFYGLDV |
| | | | SEQ ID NO:4536 | SEQ ID NO:12548 | SEQ ID NO:20560 |
| iPS:392984 | | NA | ATCTATGCCATGAGC | GTTATTAGTGGTAGTGGT GGTAGCTCATTCTACGCA GACTCCGTGAAGGGC | GATCGGGTGAAAGCTCATGA TGGTTTTGATATC |
| | **21-225_30E11** | | SEQ ID NO:4537 | SEQ ID NO:12549 | SEQ ID NO:20561 |
| | | AA | IYAMS | VISGSGGSSFYADSVKG | DRVKAHDGFDI |
| | | | SEQ ID NO:4538 | SEQ ID NO:12550 | SEQ ID NO:20562 |

FIGURE 49

| iPS:392986 | | NA | AGCTATGCCATGAGC | GCTATTAGTGGTCGTGGT GGTAGCACATTCCACGC AGACTCCGTGAAGGGC | GGGGAGCTACTAGAGGACTA CTACTTCTACGGTATGGACG TC |
|------------|------------|----|------------------|------------------|------------------|
| | 21-225_31B8 | | SEQ ID NO:4539 | SEQ ID NO:12551 | SEQ ID NO:20563 |
| | | AA | SYAMS | AISGRGGSTFHADSVKG | GELLEDYYFYGMDV |
| | | | SEQ ID NO:4540 | SEQ ID NO:12552 | SEQ ID NO:20564 |
| iPS:392988 | | NA | GACTATGGCATGCAC | GTTATATGGTATGATGA AAATAATAAATACTATG CAGACTCCGTGAAGGGC | GAACTGGGGATGACGGGTGA CTCC |
| | 21-225_25E6 | | SEQ ID NO:4541 | SEQ ID NO:12553 | SEQ ID NO:20565 |
| | | AA | DYGMH | VIWYDENNKYYADSVKG | ELGMTGDS |
| | | | SEQ ID NO:4542 | SEQ ID NO:12554 | SEQ ID NO:20566 |
| iPS:392990 | | NA | GACTATGGCATGCAC | GTTATATGGTATGATGA AAGTAATAAATACTATG CAGACTCCATGAAGGGC | GAACTGGGGATGACGGGTGA CTAC |
| | 21-225_25H10 | | SEQ ID NO:4543 | SEQ ID NO:12555 | SEQ ID NO:20567 |
| | | AA | DYGMH | VIWYDESNKYYADSMKG | ELGMTGDY |
| | | | SEQ ID NO:4544 | SEQ ID NO:12556 | SEQ ID NO:20568 |
| iPS:392992 | | NA | AATTATGATATCAAC | TGGATGAACCCTAACAG TGGTAACACAGGCTATG CACAGAGATTCCAGGGC | AGCAGTGGCTGGTACTACTT TGACTAC |
| | 21-225_26C4 | | SEQ ID NO:4545 | SEQ ID NO:12557 | SEQ ID NO:20569 |
| | | AA | NYDIN | WMNPNSGNTGYAQRFQG | SSGWYYFDY |
| | | | SEQ ID NO:4546 | SEQ ID NO:12558 | SEQ ID NO:20570 |

FIGURE 49

| iPS:392994 | | NA | AGCTATGGCATGCAC | GTTATATGGTATGATGG AAGTAATAAATACTATG CAGACTCCGTGAAGGGC | AGGTATAGCAACAGCTGGTC AGGGGGTATGGACGTC |
|---|---|---|---|---|---|
| | 21-225_26G11 | | SEQ ID NO:4547 | SEQ ID NO:12559 | SEQ ID NO:20571 |
| | | AA | SYGMH | VIWYDGSNKYYADSVKG | RYSNSWSGGMDV |
| | | | SEQ ID NO:4548 | SEQ ID NO:12560 | SEQ ID NO:20572 |
| iPS:392996 | | NA | AGCTATGCCATGAGC | GCTATTAGTGGTAGTGG AGGTAACACATTCTACG CAGACTCCGTGAAGGGC | TTGGGGCGTATAGCAGTGAC TGGTCCTTACTTTGACTAC |
| | 21-225_28B1 | | SEQ ID NO:4549 | SEQ ID NO:12561 | SEQ ID NO:20573 |
| | | AA | SYAMS | AISGSGGNTFYADSVKG | LGRIAVTGPYFDY |
| | | | SEQ ID NO:4550 | SEQ ID NO:12562 | SEQ ID NO:20574 |
| iPS:392998 | | NA | AGCTATGGCATACAC | GTTATATGGTTTGATGGA AGTAATAAATACTATGC AGACTCCGTGAAGGGC | GAAATTGGCTGGTTAGATGA CTAC |
| | 21-225_28A9 | | SEQ ID NO:4551 | SEQ ID NO:12563 | SEQ ID NO:20575 |
| | | AA | SYGIH | VIWFDGSNKYYADSVKG | EIGWLDDY |
| | | | SEQ ID NO:4552 | SEQ ID NO:12564 | SEQ ID NO:20576 |
| iPS:393000 | | NA | AGCTATGGCATGCAC | GTTATATGGTATGATGA AAGTAATAAATACTATG GAGACTCCGTGAAGGGC | GAACTGGGGTTCCTCTCTGA CTAC |
| | 21-225_29D7 | | SEQ ID NO:4553 | SEQ ID NO:12565 | SEQ ID NO:20577 |
| | | AA | SYGMH | VIWYDESNKYYGDSVKG | ELGFLSDY |
| | | | SEQ ID NO:4554 | SEQ ID NO:12566 | SEQ ID NO:20578 |

FIGURE 49

| iPS:393002 | | NA | TACTATGGCATGCAC | GTTATATGGCATGATGG AAGTAATAAATACTATG TAGACTCCGTGAAGGGC | GAAAATAGCAGTTCGTACTA CTTTGACTAC |
|---|---|---|---|---|---|
| | 21-225_30G1 | | SEQ ID NO:4555 | SEQ ID NO:12567 | SEQ ID NO:20579 |
| | | AA | YYGMH | VIWHDGSNKYYVDSVKG | ENSSSYYFDY |
| | | | SEQ ID NO:4556 | SEQ ID NO:12568 | SEQ ID NO:20580 |
| iPS:393004 | | NA | AGCTATGCCATGAGC | GCTATTAGTGGTCGTGGT GGTAGCACATTCAACGC AGACTCCGTGAAGGGC | GGGGAGCTACTAGAGGACTA CTACTTCTACGGTATGGACG TC |
| | 21-225_30G11 | | SEQ ID NO:4557 | SEQ ID NO:12569 | SEQ ID NO:20581 |
| | | AA | SYAMS | AISGRGGSTFNADSVKG | GELLEDYYFYGMDV |
| | | | SEQ ID NO:4558 | SEQ ID NO:12570 | SEQ ID NO:20582 |
| iPS:393006 | | NA | AGCTATAGCATGAAC | TCAATTAGTAGTAGTAGT AGTTACATATACTACGC AGACTCAGTGAAGGGC | GATCGAGGCAGCAGC |
| | 21-225_31G9 | | SEQ ID NO:4559 | SEQ ID NO:12571 | SEQ ID NO:20583 |
| | | AA | SYSMN | SISSSSSYIYYADSVKG | DRGSS |
| | | | SEQ ID NO:4560 | SEQ ID NO:12572 | SEQ ID NO:20584 |
| iPS:393010 | | NA | AGCTATGCCATGAGC | GTTATTAGTGGTGGTGGT GGTAGCACATACTACGC AGACTCCGTGAAGGGC | CGTGGATATAGTGGCTACGA GGACCTCCTCTACTTTGACT GC |
| | 21-225_25E11 | | SEQ ID NO:4561 | SEQ ID NO:12573 | SEQ ID NO:20585 |
| | | AA | SYAMS | VISGGGGSTYYADSVKG | RGYSGYEDLLYFDC |
| | | | SEQ ID NO:4562 | SEQ ID NO:12574 | SEQ ID NO:20586 |
| iPS:393012 | | NA | AGCTATGGCATGCAC | GTTATATGGTATGATGG AAGTAATAAATATTATG CAGACTCCGTGAAGGGC | AGGTATAGCAGCAGCTGGTC AGGGGGTATGGACGTC |
| | 21-225_26G7 | | SEQ ID NO:4563 | SEQ ID NO:12575 | SEQ ID NO:20587 |

FIGURE 49

| | | AA | SYGMH | VIWYDGSNKYYADSVKG | RYSSSWSGGMDV |
|---|---|---|---|---|---|
| | | | SEQ ID NO:4564 | SEQ ID NO:12576 | SEQ ID NO:20588 |
| iPS:393014 | | NA | AGTTATGGCATGCAC | GTTATATGGTATGATGG AAGTAATGAATACTATG CAGACTCCGTGAAGGGC | GATTCCTCCCCCTACGGTAT GGACGTC |
| | 21-225_26D12 | | SEQ ID NO:4565 | SEQ ID NO:12577 | SEQ ID NO:20589 |
| | | AA | SYGMH | VIWYDGSNEYYADSVKG | DSSPYGMDV |
| | | | SEQ ID NO:4566 | SEQ ID NO:12578 | SEQ ID NO:20590 |
| iPS:393016 | | NA | AGCTATGCCATGAGC | GTTACTAGTGGTAGTGGT GGTACCACATTCTACGC AGACTCCGTGAAGGGC | CGGACCCAGTTTGATGATTT TGATATC |
| | 21-225_28F11 | | SEQ ID NO:4567 | SEQ ID NO:12579 | SEQ ID NO:20591 |
| | | AA | SYAMS | VTSGSGGTTFYADSVKG | RTQFDDFDI |
| | | | SEQ ID NO:4568 | SEQ ID NO:12580 | SEQ ID NO:20592 |
| iPS:393018 | | NA | GACTATGGCATGCAC | GTTATATGGTATGATGA AAATAATAAATACTATG CAGACTCCGTGAAGGGC | GAACTGGGGATGACGGGTGA CTCC |
| | 21-225_29B8 | | SEQ ID NO:4569 | SEQ ID NO:12581 | SEQ ID NO:20593 |
| | | AA | DYGMH | VIWYDENNKYYADSVKG | ELGMTGDS |
| | | | SEQ ID NO:4570 | SEQ ID NO:12582 | SEQ ID NO:20594 |
| iPS:393020 | | NA | AGCTATGGCATGCAC | GTTATATCATATGGTGGA AGTAATAAATTCTATGC AGTCTCCGTGAAGGGC | AGGGGGTATAGCAGTGGAGG CTACGGTATGGACGTC |
| | 21-225_30E2 | | SEQ ID NO:4571 | SEQ ID NO:12583 | SEQ ID NO:20595 |
| | | AA | SYGMH | VISYGGSNKFYAVSVKG | RGYSSGGYGMDV |
| | | | SEQ ID NO:4572 | SEQ ID NO:12584 | SEQ ID NO:20596 |

FIGURE 49

| iPS:393022 | 21-225_30H11 | NA | AGCTATAGCATGAAC | TCCATTAGTGGTAGTAGT AGTTACATATACTACGC AGACTCAGTGAAGGGC | GATAGGGGGAGCCTC |
|---|---|---|---|---|---|
| | | | SEQ ID NO:4573 | SEQ ID NO:12585 | SEQ ID NO:20597 |
| | | AA | SYSMN | SISGSSSYIYYADSVKG | DRGSL |
| | | | SEQ ID NO:4574 | SEQ ID NO:12586 | SEQ ID NO:20598 |
| iPS:393024 | 21-225_31H9 | NA | AGCTGTGCCATGAAC | GCTATTAGTGGTAGTGGT GGTAGCTCATTCTACGCA GACTCCGTGAAGGGC | CGGACTCCCTATGATGTCTTT GATATC |
| | | | SEQ ID NO:4575 | SEQ ID NO:12587 | SEQ ID NO:20599 |
| | | AA | SCAMN | AISGSGGSSFYADSVKG | RTPYDVFDI |
| | | | SEQ ID NO:4576 | SEQ ID NO:12588 | SEQ ID NO:20600 |
| iPS:393026 | 21-225_32B6 | NA | GACTATGGCATGCAC | GTTATATGGTATGATGA AAATACTAAATACTATG CAGACTCCGTGAAGGGC | GAGTGGGGGGACTAC |
| | | | SEQ ID NO:4577 | SEQ ID NO:12589 | SEQ ID NO:20601 |
| | | AA | DYGMH | VIWYDENTKYYADSVKG | EWGDY |
| | | | SEQ ID NO:4578 | SEQ ID NO:12590 | SEQ ID NO:20602 |
| iPS:393028 | 21-225_25D7 | NA | AGCTATGCCATGAGC | GCTATTAGTGGTAGAGG TGGTACCACATTCTACGC AGACTCCGTGAAGGGC | GACGGGTACGGTGGTAACTC CTTCTTTGACTAC |
| | | | SEQ ID NO:4579 | SEQ ID NO:12591 | SEQ ID NO:20603 |
| | | AA | SYAMS | AISGRGGTTFYADSVKG | DGYGGNSFFDY |
| | | | SEQ ID NO:4580 | SEQ ID NO:12592 | SEQ ID NO:20604 |
| iPS:393030 | 21 225 25H11 | NA | GACTATGGCATGCAC | GTTATATGGTATGATGA AAATAATGAATACTATG CAGACTCCGTGAAGGGC | GAACTGGGGATGACGGGTGA CTCC |

FIGURE 49

| | | | SEQ ID NO:4581 | SEQ ID NO:12593 | SEQ ID NO:20605 |
|---|---|---|---|---|---|
| | 21-225_25H11 | AA | DYGMH | VIWYDENNEYYADSVKG | ELGMTGDS |
| | | | SEQ ID NO:4582 | SEQ ID NO:12594 | SEQ ID NO:20606 |
| iPS:393032 | | NA | GGCTATGGCATGCAC | ATTATATGGTATGATGG AAGTAATAAATACTATG CAGACTCCGTGAAGGGC | GAGAGGTACGATTTTTGGAG TGGTTGTATGGACGTC |
| | 21-225_26F8 | | SEQ ID NO:4583 | SEQ ID NO:12595 | SEQ ID NO:20607 |
| | | AA | GYGMH | IIWYDGSNKYYADSVKG | ERYDFWSGCMDV |
| | | | SEQ ID NO:4584 | SEQ ID NO:12596 | SEQ ID NO:20608 |
| iPS:393034 | | NA | GACTATGGCATGCAC | GTTATATGGTATGATGA AAATAATAAATACTATG TAGACTCCGTGAGGGGC | GAACTGGGGATGACGGGTGA CTCC |
| | 21-225_27F2 | | SEQ ID NO:4585 | SEQ ID NO:12597 | SEQ ID NO:20609 |
| | | AA | DYGMH | VIWYDENNKYYVDSVRG | ELGMTGDS |
| | | | SEQ ID NO:4586 | SEQ ID NO:12598 | SEQ ID NO:20610 |
| iPS:393036 | | NA | ACCTATGGCATGCAC | GTTATATGGTATGATGG AAGTAATAAATACTATG CAGACTCCGTGAAGGGC | GATCGATACGATTTTTGGAG TGGTTATTTTGACTAC |
| | 21-225_28G3 | | SEQ ID NO:4587 | SEQ ID NO:12599 | SEQ ID NO:20611 |
| | | AA | TYGMH | VIWYDGSNKYYADSVKG | DRYDFWSGYFDY |
| | | | SEQ ID NO:4588 | SEQ ID NO:12600 | SEQ ID NO:20612 |

FIGURE 49

| iPS:393038 | | NA | GACTATGGCATACAC | GTTATATGGTTTGATGGAACTAATAAATACTATGCAGACTCCGTGAAGGGC | GAAATTGGCTGGTTAGATGACTAC |
|---|---|---|---|---|---|
| | 21-225_29D8 | | SEQ ID NO:4589 | SEQ ID NO:12601 | SEQ ID NO:20613 |
| | | AA | DYGIH | VIWFDGTNKYYADSVKG | EIGWLDDY |
| | | | SEQ ID NO:4590 | SEQ ID NO:12602 | SEQ ID NO:20614 |
| iPS:393040 | | NA | AGCTATGCCATGAAC | GCTATTAGTGGTCGTGGTGGTAGCACATTCTACGCAGACTCCGAGAAGGGC | GGGGAGCTATTAGAGGACTACTACTACTACGGAATGGACGTC |
| | 21-225_30E3 | | SEQ ID NO:4591 | SEQ ID NO:12603 | SEQ ID NO:20615 |
| | | AA | SYAMN | AISGRGGSTFYADSEKG | GELLEDYYYYGMDV |
| | | | SEQ ID NO:4592 | SEQ ID NO:12604 | SEQ ID NO:20616 |
| iPS:393042 | | NA | GACTACTATATGCAC | TGGATCAACCCTAACAGTGGTGGCACAAACTATGCACAGAAGTTTCAGGGC | GATAGTAGCAATTTCAGCAACTGGTACGATTACTACGGTATGGACGTC |
| | 21-225_31F1 | | SEQ ID NO:4593 | SEQ ID NO:12605 | SEQ ID NO:20617 |
| | | AA | DYYMH | WINPNSGGTNYAQKFQG | DSSNFSNWYDYYGMDV |
| | | | SEQ ID NO:4594 | SEQ ID NO:12606 | SEQ ID NO:20618 |
| iPS:393044 | | NA | AGCTATGGTATCAGC | TGGATCAGCGCTTACAATGGTAACACAACCTATGCACAGAAGCTCCGGGGC | ACCGCTGCTGGGTATAGCAGCAGCTGGTTTGACTAC |
| | 21-225_25B8 | | SEQ ID NO:4595 | SEQ ID NO:12607 | SEQ ID NO:20619 |
| | | AA | SYGIS | WISAYNGNTTYAQKLRG | TAAGYSSSWFDY |
| | | | SEQ ID NO:4596 | SEQ ID NO:12608 | SEQ ID NO:20620 |

FIGURE 49

| iPS:393046 | | NA | AACTGTGTCATGCAC | GTTATATGGTATGATGG AAGTAATAAATACTATG CAGACTCCGTGAAGGGC | GAGGAGTATAGCAGTGGCTG GTACGACTACGGTATGGACG TC |
|---|---|---|---|---|---|
| | 21-225_25A12 | | SEQ ID NO:4597 | SEQ ID NO:12609 | SEQ ID NO:20621 |
| | | AA | NCVMH | VIWYDGSNKYYADSVKG | EEYSSGWYDYGMDV |
| | | | SEQ ID NO:4598 | SEQ ID NO:12610 | SEQ ID NO:20622 |
| iPS:393048 | | NA | GACTATGGCATGCAC | GTTATATGGTATGATGA AAATAATAAATCCTATG CAGACTCCGTGAAGGGC | GAACTGGGGATGACGGGTGA CTCC |
| | 21-225_27C3 | | SEQ ID NO:4599 | SEQ ID NO:12611 | SEQ ID NO:20623 |
| | | AA | DYGMH | VIWYDENNKSYADSVKG | ELGMTGDS |
| | | | SEQ ID NO:4600 | SEQ ID NO:12612 | SEQ ID NO:20624 |
| iPS:393050 | | NA | AGCTATGGTATCAGC | TGGATCAGCGCTTACAA TGGTAACACAACCTATG CACAGAAGCTCCGGGGC | ACCGCTGCTGGGTATAGCAG CAGCTGGTTTGACTAC |
| | 21-225_28C5 | | SEQ ID NO:4601 | SEQ ID NO:12613 | SEQ ID NO:20625 |
| | | AA | SYGIS | WISAYNGNTTYAQKLRG | TAAGYSSSWFDY |
| | | | SEQ ID NO:4602 | SEQ ID NO:12614 | SEQ ID NO:20626 |
| iPS:393054 | | NA | GACTATGGCATGCAC | GTTATATGGTATGATGA AACTAATAAATACTATG CAGACTCCGTGAAGGGC | GAACTGGGGATGACGAGTGA CTAC |
| | 21-225_29G8 | | SEQ ID NO:4603 | SEQ ID NO:12615 | SEQ ID NO:20627 |
| | | AA | DYGMH | VIWYDETNKYYADSVKG | ELGMTSDY |
| | | | SEQ ID NO:4604 | SEQ ID NO:12616 | SEQ ID NO:20628 |

FIGURE 49

| iPS:393056 | | NA | AGCTATGGCATGCAC | GTTATATGGTATGATGTA AGTAATAAATACTATGC AGACTCCGTGAAGGGC | GAAATGGGCTGGTACGATGA CTAC |
|---|---|---|---|---|---|
| | 21-225_30F3 | | SEQ ID NO:4605 | SEQ ID NO:12617 | SEQ ID NO:20629 |
| | | AA | SYGMH | VIWYDVSNKYYADSVKG | EMGWYDDY |
| | | | SEQ ID NO:4606 | SEQ ID NO:12618 | SEQ ID NO:20630 |
| iPS:393058 | 21-225_31H3 | NA | AGCTATGCCATGAAC | GCTATTAGTGGTCGTGGT GGTAACACATTCTACGC AGACTCCGTGAAGGGC | GGGGAGTTACTAGAGGACTA CTACTACTACGGAATGGACG TC |
| | | | SEQ ID NO:4607 | SEQ ID NO:12619 | SEQ ID NO:20631 |
| | | AA | SYAMN | AISGRGGNTFYADSVKG | GELLEDYYYYGMDV |
| | | | SEQ ID NO:4608 | SEQ ID NO:12620 | SEQ ID NO:20632 |
| iPS:393060 | 21-225_32G12 | NA | AGCTATGCCATGAGC | TCTATTAGTGGTCGTGGT GGTAGCACATTCCACGC AGACTCCGTGAAGGGC | GGGGAGCTACTAGAGGACTA CTACTTCTACGGTATGGACG TC |
| | | | SEQ ID NO:4609 | SEQ ID NO:12621 | SEQ ID NO:20633 |
| | | AA | SYAMS | SISGRGGSTFHADSVKG | GELLEDYYFYGMDV |
| | | | SEQ ID NO:4610 | SEQ ID NO:12622 | SEQ ID NO:20634 |
| iPS:393062 | 21-225_33H3 | NA | AGCTATGGCATGCAC | ATTATATCATATGGTGGA AGTAATAACTTCTATGCA GACTCCGTGAAGGGC | AGGGGGGTATAGCAGTGGAGG CTACGGTATGGACGTC |
| | | | SEQ ID NO:4611 | SEQ ID NO:12623 | SEQ ID NO:20635 |
| | | AA | SYGMH | IISYGGSNNFYADSVKG | RGYSSGGYGMDV |
| | | | SEQ ID NO:4612 | SEQ ID NO:12624 | SEQ ID NO:20636 |
| iPS:393064 | 21-225_33A9 | NA | AGTTATGATATCAAC | TGGATGAACCCTAACAG TGGTAACACAGGCTATG CACAGAAGTTCCAGGGC | AAGAAGGCTAACGACTAC |
| | | | SEQ ID NO:4613 | SEQ ID NO:12625 | SEQ ID NO:20637 |

FIGURE 49

| | | | AA | SYDIN | WMNPNSGNTGYAQKFQG | KKANDY |
|---|---|---|---|---|---|---|
| | | | | SEQ ID NO:4614 | SEQ ID NO:12626 | SEQ ID NO:20638 |
| iPS:393066 | | NA | | TACTATGGCATGCAC | GTTATATGGCATGATGG AAGTAATAAATACTATG TAGACTCCGTGAAGGGC | GAAAATAGCAGTTCGTTCTA CTTTGACTAC |
| | 21-225_34D3 | | | SEQ ID NO:4615 | SEQ ID NO:12627 | SEQ ID NO:20639 |
| | | | AA | YYGMH | VIWHDGSNKYYVDSVKG | ENSSSFYFDY |
| | | | | SEQ ID NO:4616 | SEQ ID NO:12628 | SEQ ID NO:20640 |
| iPS:393068 | | NA | | AGCTATGCCATGAGC | GCTATTAGTGGTCGTGGT GGTAGCACATTCCACGC AGACTCCGTGAAGGGC | GGGGAGCTACTAGAGGACTA CTACTTCTACGGTATGGACG TC |
| | 21-225_34G9 | | | SEQ ID NO:4617 | SEQ ID NO:12629 | SEQ ID NO:20641 |
| | | | AA | SYAMS | AISGRGGSTFHADSVKG | GELLEDYYFYGMDV |
| | | | | SEQ ID NO:4618 | SEQ ID NO:12630 | SEQ ID NO:20642 |
| iPS:393072 | | NA | | AGCTATGCCATGAAC | GCTATTAGTGGTCGTGGT GGTAGCACATTCCACGC AGACTCCGTGAAGGGC | GGGGAGCTATTAGAGGACTA CTACTTCTACGGTATGGACG TC |
| | 21-225_36C5 | | | SEQ ID NO:4619 | SEQ ID NO:12631 | SEQ ID NO:20643 |
| | | | AA | SYAMN | AISGRGGSTFHADSVKG | GELLEDYYFYGMDV |
| | | | | SEQ ID NO:4620 | SEQ ID NO:12632 | SEQ ID NO:20644 |
| iPS:393074 | | NA | | GACTATGGCATGCAC | GTTATATGGTATGATAG AAATAATAAATACTATG CAGACTCCGTGAAGGGC | GAAATGGGCTGGTACGATGA CTAC |
| | 21-225_33B1 | | | SEQ ID NO:4621 | SEQ ID NO:12633 | SEQ ID NO:20645 |
| | | | AA | DYGMH | VIWYDRNNKYYADSVKG | EMGWYDDY |

FIGURE 49

| | | | SEQ ID NO:4622 | SEQ ID NO:12634 | SEQ ID NO:20646 |
|---|---|---|---|---|---|
| iPS:393076 | 21-225_33A4 | NA | AGCTATGCCATGAAC | GCTATTAGTCGTCGTGGT GGTAGCACATTCTACGC AGACTCCGTGAAGGGC | GGGGAACTACTAGAGGACTA CTCCTACTACGGTATCGACG TC |
| | | | SEQ ID NO:4623 | SEQ ID NO:12635 | SEQ ID NO:20647 |
| | | AA | SYAMN | AISRRGGSTFYADSVKG | GELLEDYSYYGIDV |
| | | | SEQ ID NO:4624 | SEQ ID NO:12636 | SEQ ID NO:20648 |
| iPS:393078 | 21-225_33H11 | NA | AGCTATAGCATGAAC | TCCATTAGTGGTAGTAGT AGTTACATATACTACGC AGACTCAGTGAAGGGC | ACAAACGGTATGGACGTC |
| | | | SEQ ID NO:4625 | SEQ ID NO:12637 | SEQ ID NO:20649 |
| | | AA | SYSMN | SISGSSSYIYYADSVKG | TNGMDV |
| | | | SEQ ID NO:4626 | SEQ ID NO:12638 | SEQ ID NO:20650 |
| iPS:393080 | 21-225_34F3 | NA | GGCTACCATATGCAC | TGGATCAACCCTAACAG TGGTGGCACAAACTATG CACAGAAGTTTCAGGGC | GACGGTACCAGCTCCTTTGA CTAC |
| | | | SEQ ID NO:4627 | SEQ ID NO:12639 | SEQ ID NO:20651 |
| | | AA | GYHMH | WINPNSGGTNYAQKFQG | DGTSSFDY |
| | | | SEQ ID NO:4628 | SEQ ID NO:12640 | SEQ ID NO:20652 |
| iPS:393082 | 21-225_34C11 | NA | AGTTATACCATGAGC | TCCATTAGTGGTAGTAGT AATTACATATACTACGC AGACTCAGTGAAGGGC | TTAACTGGTTTTGACTAC |
| | | | SEQ ID NO:4629 | SEQ ID NO:12641 | SEQ ID NO:20653 |
| | | AA | SYTMS | SISGSSNYIYYADSVKG | LTGFDY |
| | | | SEQ ID NO:4630 | SEQ ID NO:12642 | SEQ ID NO:20654 |
| iPS:393084 | 21-225_35C6 | NA | GGCGATTATATGCAC | TGGATCAGCCCTAAAAA TGGTGGCACAAACTATG CACAGAAGTTTCAGGGC | GATGGAACTGGGTCCTTTGA CTAC |
| | | | SEQ ID NO:4631 | SEQ ID NO:12643 | SEQ ID NO:20655 |

996

FIGURE 49

| | | AA | GDYMH | WISPKNGGTNYAQKFQG | DGTGSFDY |
|---|---|---|---|---|---|
| | | | SEQ ID NO:4632 | SEQ ID NO:12644 | SEQ ID NO:20656 |
| iPS:393086 | | NA | GACTATCATATGCAC | TGGATCAACCCTAATAG GGGTGGCACAAACTATG CACAGAAGTTTCAGGAC | GATGGAACTGGGTCCTTTGA CTAC |
| | 21-225_36H5 | | SEQ ID NO:4633 | SEQ ID NO:12645 | SEQ ID NO:20657 |
| | | AA | DYHMH | WINPNRGGTNYAQKFQD | DGTGSFDY |
| | | | SEQ ID NO:4634 | SEQ ID NO:12646 | SEQ ID NO:20658 |
| iPS:393088 | | NA | AATTATGACATTAAC | TGGATGCACCCTAACAG TGGTAACACAGGCTTTG CACAGAAGTTCCGGGGC | AGCAGTGGCTGGTACTTTTT TGACTAC |
| | 21-225_33D1 | | SEQ ID NO:4635 | SEQ ID NO:12647 | SEQ ID NO:20659 |
| | | AA | NYDIN | WMHPNSGNTGFAQKFRG | SSGWYFFDY |
| | | | SEQ ID NO:4636 | SEQ ID NO:12648 | SEQ ID NO:20660 |
| iPS:393090 | | NA | AGCTATGTCATAAAC | GCTATTAGTGGTAGTGGT GTTAGCACATACTACGC AGACTCCGTGAAGGGC | ACTTCCCTCTTTGACTAC |
| | 21-225_33A5 | | SEQ ID NO:4637 | SEQ ID NO:12649 | SEQ ID NO:20661 |
| | | AA | SYVIN | AISGSGVSTYYADSVKG | TSLFDY |
| | | | SEQ ID NO:4638 | SEQ ID NO:12650 | SEQ ID NO:20662 |
| iPS:393092 | | NA | CACTATGGCATGCAC | GTTATATGGTATGATGG AAGTAATAAATACTATG CAGACTCCGTGAAGGGC | GAAAATAGCAGCTCGTACTA CTTTGACTAC |
| | 21-225_33C12 | | SEQ ID NO:4639 | SEQ ID NO:12651 | SEQ ID NO:20663 |
| | | AA | HYGMH | VIWYDGSNKYYADSVKG | ENSSSYYFDY |
| | | | SEQ ID NO:4640 | SEQ ID NO:12652 | SEQ ID NO:20664 |

FIGURE 49

| iPS:393094 | | NA | AGAAGTAGTTACTACTGGGGC | AGTATCTATTATAGTGGGAGCACCGCCTACAATCCGTCCCTCAAGAGT | CTGAGCAGCAGCTGGTCTTTTGACTAC |
|---|---|---|---|---|---|
| | 21-225_34C4 | | SEQ ID NO:4641 | SEQ ID NO:12653 | SEQ ID NO:20665 |
| | | AA | RSSYYWG | SIYYSGSTAYNPSLKS | LSSSWSFDY |
| | | | SEQ ID NO:4642 | SEQ ID NO:12654 | SEQ ID NO:20666 |
| iPS:393096 | | NA | AGCTATGCCATGAAC | GCTATTAGTGGTCGTGGTGGTAGCACATTCCACGCAGACTCCGTGAAGGGC | GGGGAGTTAGTAGAGGACTACTACTTCTACGGTATGGACGTC |
| | 21-225_34D11 | | SEQ ID NO:4643 | SEQ ID NO:12655 | SEQ ID NO:20667 |
| | | AA | SYAMN | AISGRGGSTFHADSVKG | GELVEDYYFYGMDV |
| | | | SEQ ID NO:4644 | SEQ ID NO:12656 | SEQ ID NO:20668 |
| iPS:393098 | | NA | GACTACCATATACAC | TGGATCAACCCTAACAATGGTGGCACACACTATGCACAGGAGTTTCAGGGC | GATGGAACTGGGTCCTTTGACTAC |
| | 21-225_35G6 | | SEQ ID NO:4645 | SEQ ID NO:12657 | SEQ ID NO:20669 |
| | | AA | DYHIH | WINPNNGGTHYAQEFQG | DGTGSFDY |
| | | | SEQ ID NO:4646 | SEQ ID NO:12658 | SEQ ID NO:20670 |
| iPS:393100 | | NA | AACTATGGCATGCAC | GTTATATGGCATGATGGAAGTAATAAATACTATGGAGACTCCGTGAAGGGC | GAAAATAGCAGCTCGTACTACTTTGACTAC |
| | 21-225_36B8 | | SEQ ID NO:4647 | SEQ ID NO:12659 | SEQ ID NO:20671 |
| | | AA | NYGMH | VIWHDGSNKYYGDSVKG | ENSSSYYFDY |
| | | | SEQ ID NO:4648 | SEQ ID NO:12660 | SEQ ID NO:20672 |
| iPS:393102 | 21 225 33F1 | NA | AGCTATGCCATGAGC | TCTATTAGTGGTCGTGGTGGTAGCACATTCCACGCAGACTCCGTGAAGGGC | GGGGAGCTACTTGAGGACTACTACTTCTACGGTATGGACGTC |

FIGURE 49

| | | | SEQ ID NO:4649 | SEQ ID NO:12661 | SEQ ID NO:20673 |
|---|---|---|---|---|---|
| | 21-225_33F1 | AA | SYAMS | SISGRGGSTFHADSVKG | GELLEDYYFYGMDV |
| | | | SEQ ID NO:4650 | SEQ ID NO:12662 | SEQ ID NO:20674 |
| iPS:393104 | | NA | AGCTGTGCCATGAGC | GCTATTAGTGGTCGTGGT GGTAGCACATTCCACGC AGACTCCGTGAAGGGC | GGGGAACTACTAGAGGACTA CTACTTCTACGGTATGGACG TC |
| | 21-225_33A7 | | SEQ ID NO:4651 | SEQ ID NO:12663 | SEQ ID NO:20675 |
| | | AA | SCAMS | AISGRGGSTFHADSVKG | GELLEDYYFYGMDV |
| | | | SEQ ID NO:4652 | SEQ ID NO:12664 | SEQ ID NO:20676 |
| iPS:393106 | | NA | AACTATGCCATGAAC | GCTATTAGTCGTCGTGGT GGTAGCACATTCTACGC AGACTCCGTGAAGGGC | GGGGAGCTACTAGAGGACTA CTACTACTTCGCTATGGACG TC |
| | 21-225_34A6 | | SEQ ID NO:4653 | SEQ ID NO:12665 | SEQ ID NO:20677 |
| | | AA | NYAMN | AISRRGGSTFYADSVKG | GELLEDYYYFAMDV |
| | | | SEQ ID NO:4654 | SEQ ID NO:12666 | SEQ ID NO:20678 |
| iPS:393108 | | NA | GGCTACTATATGCAC | TGGATCAACCCTAACAG TGGTGGCACAAACTATG CACAGAAGTTTCAGGGC | GATATTAGTAATTTCAGCAG CTGGTACGATTACTACGCTA TGGACGTC |
| | 21-225_34G11 | | SEQ ID NO:4655 | SEQ ID NO:12667 | SEQ ID NO:20679 |
| | | AA | GYYMH | WINPNSGGTNYAQKFQG | DISNFSSWYDYYAMDV |
| | | | SEQ ID NO:4656 | SEQ ID NO:12668 | SEQ ID NO:20680 |
| iPS:393110 | | NA | AGCTATGCCATGAGC | GCTATTAGTGGTCGTGGT GGTAGCACATTCCACGC AGACTCCGTGAAGGGC | GGGGAGCTACTAGAGGACTA CTACTTCTACGGTATGGACG TC |
| | 21-225_35B7 | | SEQ ID NO:4657 | SEQ ID NO:12669 | SEQ ID NO:20681 |
| | | AA | SYAMS | AISGRGGSTFHADSVKG | GELLEDYYFYGMDV |
| | | | SEQ ID NO:4658 | SEQ ID NO:12670 | SEQ ID NO:20682 |
| iPS:393112 | 21 225 33G1 | NA | GGCTACTATATGCAC | TGGATCAGCCCTAACAA TGGTGGCACAAACTATG CACAGAAGTTTCAGGGC | GATGGAACTGGGTCCTTTGA CTAC |

FIGURE 49

| | | | SEQ ID NO:4659 | SEQ ID NO:12671 | SEQ ID NO:20683 |
|---|---|---|---|---|---|
| | 21-225_33G1 | AA | GYYMH | WISPNNGGTNYAQKFQG | DGTGSFDY |
| | | | SEQ ID NO:4660 | SEQ ID NO:12672 | SEQ ID NO:20684 |
| iPS:393114 | 21-225_33G12 | NA | AGCTATGCCATGAGC | GTTATTAGTGGTAGTGGTGGTAGCTCATTCTACGCAGACTCCGTGAAGGGC | GATCGGGTGAGAGCTCATGATGGTTTTGATATC |
| | | | SEQ ID NO:4661 | SEQ ID NO:12673 | SEQ ID NO:20685 |
| | | AA | SYAMS | VISGSGGSSFYADSVKG | DRVRAHDGFDI |
| | | | SEQ ID NO:4662 | SEQ ID NO:12674 | SEQ ID NO:20686 |
| iPS:393116 | 21-225_34G7 | NA | GACTACCATATTCAC | TGGATCAACCCTAACAATGGTGGCACACACTATGCACAGAAGTTTCAGGGC | GATGGAACTGGGTCCTTTGACTAC |
| | | | SEQ ID NO:4663 | SEQ ID NO:12675 | SEQ ID NO:20687 |
| | | AA | DYHIH | WINPNNGGTHYAQKFQG | DGTGSFDY |
| | | | SEQ ID NO:4664 | SEQ ID NO:12676 | SEQ ID NO:20688 |
| iPS:393118 | 21-225_34H11 | NA | AGCTATGCCATGAAC | GCTATTAGTGGTCGTGGTGGTAGCACATTCTACGCAGACTCCGTGAAGGGC | GGGGAGCTACTAGAGGACTACTACTACTACGGTATGGACGTC |
| | | | SEQ ID NO:4665 | SEQ ID NO:12677 | SEQ ID NO:20689 |
| | | AA | SYAMN | AISGRGGSTFYADSVKG | GELLEDYYYYGMDV |
| | | | SEQ ID NO:4666 | SEQ ID NO:12678 | SEQ ID NO:20690 |
| iPS:393120 | 21-225_35H8 | NA | AGTTATAGCATGAAC | TCCATTAGTGGTACTGGTAGTTTCATATACTACGCAGACTCAGTGAAGGGC | GTCTCTGGCTTTGACTAC |
| | | | SEQ ID NO:4667 | SEQ ID NO:12679 | SEQ ID NO:20691 |
| | | AA | SYSMN | SISGTGSFIYYADSVKG | VSGFDY |
| | | | SEQ ID NO:4668 | SEQ ID NO:12680 | SEQ ID NO:20692 |

FIGURE 49

| iPS:393122 | | NA | AACTATGGCATGCAC | GTTATATGGCATGATGG<br>AAGTAATAAATACTATG<br>CAGACTCCGTGAAGGGC | GAAAATAGCAGCTCGTACTA<br>CTTTGACTAC |
|---|---|---|---|---|---|
| | 21-225_33B2 | | SEQ ID NO:4669 | SEQ ID NO:12681 | SEQ ID NO:20693 |
| | | AA | NYGMH | VIWHDGSNKYYADSVKG | ENSSSYYFDY |
| | | | SEQ ID NO:4670 | SEQ ID NO:12682 | SEQ ID NO:20694 |
| iPS:393124 | | NA | AACTATGCCATGAGC | GCTATTAGTCGTCGTGGT<br>GGTAGCACATTCTACGC<br>AGACTCCGTGAAGGGC | GGGGAGCTACTAGAGGACTA<br>CTCCTACTACGGTATGGACG<br>TC |
| | 21-225_33G7 | | SEQ ID NO:4671 | SEQ ID NO:12683 | SEQ ID NO:20695 |
| | | AA | NYAMS | AISRRGGSTFYADSVKG | GELLEDYSYYGMDV |
| | | | SEQ ID NO:4672 | SEQ ID NO:12684 | SEQ ID NO:20696 |
| iPS:393126 | | NA | AGCTATGCCATGAGC | GCTATTAGTGGTCGTGGT<br>GGTAGCACATTCCACGC<br>AGACTCCGTGAAGGGC | GGGGAGTTACTAGAGGACTA<br>CTACTTCTACGGTATGGACG<br>TC |
| | 21-225_35D1 | | SEQ ID NO:4673 | SEQ ID NO:12685 | SEQ ID NO:20697 |
| | | AA | SYAMS | AISGRGGSTFHADSVKG | GELLEDYYFYGMDV |
| | | | SEQ ID NO:4674 | SEQ ID NO:12686 | SEQ ID NO:20698 |
| iPS:393128 | | NA | AGCTATGCCATGAGC | GCTATTAGTGGTCGTGGT<br>GGTAGCACATTCCACGC<br>AGACTCCATGAAGGGC | GGGGAGCTACTAGAGGACTA<br>CTACTTCTACGGTATGGACG<br>TC |
| | 21-225_35F11 | | SEQ ID NO:4675 | SEQ ID NO:12687 | SEQ ID NO:20699 |
| | | AA | SYAMS | AISGRGGSTFHADSMKG | GELLEDYYFYGMDV |
| | | | SEQ ID NO:4676 | SEQ ID NO:12688 | SEQ ID NO:20700 |
| iPS:393130 | | NA | AGCTATACCATGAAC | TCCATTAGTGGTAGTAGT<br>AGTTACATATACTACGC<br>GGACTCAGTGAAGGGC | GATCGGGGGGGGGACC |
| | 21-225_33C2 | | SEQ ID NO:4677 | SEQ ID NO:12689 | SEQ ID NO:20701 |

FIGURE 49

| | | AA | SYTMN | SISGSSSYIYYADSVKG | DRGGT |
|---|---|---|---|---|---|
| | | | SEQ ID NO:4678 | SEQ ID NO:12690 | SEQ ID NO:20702 |
| iPS:393132 | 21-225_33H7 | NA | GACTACCATATACAC | TGGATCAACCCTAACAATGGTGGCACACACTATGCACAGGAGTTTCAGGGC | GATGGAACTGGGTCCTTTGACTAC |
| | | | SEQ ID NO:4679 | SEQ ID NO:12691 | SEQ ID NO:20703 |
| | | AA | DYHIH | WINPNNGGTHYAQEFQG | DGTGSFDY |
| | | | SEQ ID NO:4680 | SEQ ID NO:12692 | SEQ ID NO:20704 |
| iPS:393134 | 21-225_34C2 | NA | AACTATGTCATGCAC | CTTATATGGTATGATGGAAGTAATAAATACTATGCAGACTCCGTGAAGGGC | GAAAATAGCAGCTCGTACTACTTTGACTAC |
| | | | SEQ ID NO:4681 | SEQ ID NO:12693 | SEQ ID NO:20705 |
| | | AA | NYVMH | LIWYDGSNKYYADSVKG | ENSSSYYFDY |
| | | | SEQ ID NO:4682 | SEQ ID NO:12694 | SEQ ID NO:20706 |
| iPS:393136 | 21-225_34D8 | NA | AACTATGGCATGCAC | GTTATATGGTATGATGGAAGTAATAAATACTATGCAGACTCCGTGAAGGGC | GAAAATAGCAGCTCGTACTACTTTGACTAC |
| | | | SEQ ID NO:4683 | SEQ ID NO:12695 | SEQ ID NO:20707 |
| | | AA | NYGMH | VIWYDGSNKYYADSVKG | ENSSSYYFDY |
| | | | SEQ ID NO:4684 | SEQ ID NO:12696 | SEQ ID NO:20708 |
| iPS:393138 | 21-225_35E3 | NA | AGCTATGGCATGCAC | GTTATATCATATGGTGGAAGTAATAAATACTATGCAGACTCCGTGAAGGGC | AGGGGGTATAGCAGTGGAGGCTACGGTATGGACGTC |
| | | | SEQ ID NO:4685 | SEQ ID NO:12697 | SEQ ID NO:20709 |

FIGURE 49

| | | AA | SYGMH | VISYGGSNKYYADSVKG | RGYSSGGYGMDV |
|---|---|---|---|---|---|
| | | | SEQ ID NO:4686 | SEQ ID NO:12698 | SEQ ID NO:20710 |
| iPS:393140 | 21-225_35H12 | NA | GACTACTATATACAC | TGGATCAACCCTAATAGGGGTGGCACAAACTATGCACAGAAGTTTCAGGGC | GATGGAACTGGGTCCTTTGACTAC |
| | | | SEQ ID NO:4687 | SEQ ID NO:12699 | SEQ ID NO:20711 |
| | | AA | DYYIH | WINPNRGGTNYAQKFQG | DGTGSFDY |
| | | | SEQ ID NO:4688 | SEQ ID NO:12700 | SEQ ID NO:20712 |
| iPS:393142 | 21-225_33A3 | NA | AGCTATGGCATGAAC | TCCATTAGTGGTAGTAGTACTTACATATACTACGCAGACTCAGTGAAGGGC | ACAAACGGTATGGACGTC |
| | | | SEQ ID NO:4689 | SEQ ID NO:12701 | SEQ ID NO:20713 |
| | | AA | SYGMN | SISGSSTYIYYADSVKG | TNGMDV |
| | | | SEQ ID NO:4690 | SEQ ID NO:12702 | SEQ ID NO:20714 |
| iPS:393144 | 21-225_34D2 | NA | AATTATGACATTAAC | TGGCTGCACCCTAACAGTGGTACCACAGGCTTTGCACAGAAGTTCCGGGGC | AGCAGTGGCTGGTACTTTTTTGACTAC |
| | | | SEQ ID NO:4691 | SEQ ID NO:12703 | SEQ ID NO:20715 |
| | | AA | NYDIN | WLHPNSGTTGFAQKFRG | SSGWYFFDY |
| | | | SEQ ID NO:4692 | SEQ ID NO:12704 | SEQ ID NO:20716 |
| iPS:393146 | 21-225_34G8 | NA | AGCTATGCCATGAGC | GCTATTAGTGGTCGTGGTGGTAGCACATTCCACGCAGACTCCGTGAAGGGC | GGGGAGCTACTAGAGGACTACTACTTCTACGGTATGGACGTC |
| | | | SEQ ID NO:4693 | SEQ ID NO:12705 | SEQ ID NO:20717 |
| | | AA | SYAMS | AISGRGGSTFHADSVKG | GELLEDYYFYGMDV |
| | | | SEQ ID NO:4694 | SEQ ID NO:12706 | SEQ ID NO:20718 |
| iPS:393148 | 21-225_35E5 | NA | AGTTATGATATCAAC | TGGATGAACCCTAACAGTGGTAACACAGGCTATGCACAGAAGTTCCAGGGC | AAGAAGTCTAACGACTAC |
| | | | SEQ ID NO:4695 | SEQ ID NO:12707 | SEQ ID NO:20719 |

FIGURE 49

| | | | SYDIN | WMNPNSGNTGYAQKFQG | KKSNDY | |
|---|---|---|---|---|---|---|
| | | AA | SYDIN | WMNPNSGNTGYAQKFQG | KKSNDY | |
| | | | SEQ ID NO:4696 | SEQ ID NO:12708 | SEQ ID NO:20720 | |
| iPS:393150 | 21-225_36A5 | NA | AACTATGCCATGAAC | GCTATTAGTCGTCGTGGT GGTAACACATTCTACGC AGACTCCGTGAAGGGC | GGGGAGCTACTAGAGGACTA CTACTACTACGCTATGGACG TC | |
| | | | SEQ ID NO:4697 | SEQ ID NO:12709 | SEQ ID NO:20721 | |
| | | AA | NYAMN | AISRRGGNTFYADSVKG | GELLEDYYYYAMDV | |
| | | | SEQ ID NO:4698 | SEQ ID NO:12710 | SEQ ID NO:20722 | |
| iPS:393152 | 21-225_25B3 | NA | AGCTATGGCATGCAC | GTTATATGGTATGATGG AAGTAATAAATATTATG CAGACTCCGTGAAGGGC | GATTCCTCCCCCTACGGTAT GGACGTC | |
| | | | SEQ ID NO:4699 | SEQ ID NO:12711 | SEQ ID NO:20723 | |
| | | AA | SYGMH | VIWYDGSNKYYADSVKG | DSSPYGMDV | |
| | | | SEQ ID NO:4700 | SEQ ID NO:12712 | SEQ ID NO:20724 | |
| iPS:393166 | 21-225_27G6 | NA | GGCTATGGCATGCAC | ATTATATGGTATGATGG AAGTAAAAAATACAATG CAGACTCCGTGAAGGGC | GATAGGGTATATTGTAGTAG TACCAGCTGCTCCCCTTACT ACTACTACTACGGTATGGAC GTC | |
| | | | SEQ ID NO:4701 | SEQ ID NO:12713 | SEQ ID NO:20725 | |
| | | AA | GYGMH | IIWYDGSKKYNADSVKG | DRVYCSSTSCSPYYYYYGMD V | |
| | | | SEQ ID NO:4702 | SEQ ID NO:12714 | SEQ ID NO:20726 | |
| iPS:393168 | 21-225_32B11 | NA | GGCTACTATATGCAC | TGGATCAACCCTAACAG TGATGGCACTAACTATG CACAGAAGTTTCAGGGC | GGGGTTTTACTATGGTTCGGG GAGTTATTATAACGACCTCG ACCCC | |
| | | | SEQ ID NO:4703 | SEQ ID NO:12715 | SEQ ID NO:20727 | |
| | | AA | GYYMH | WINPNSDGTNYAQKFQG | GFYYGSGSYYNDLDP | |
| | | | SEQ ID NO:4704 | SEQ ID NO:12716 | SEQ ID NO:20728 | |

FIGURE 49

| iPS:393172 | | NA | TACTATGGCATGCAC | GTTATATCATATGATGGA AGTAATAAATACTATGC AGACTCCGTGAAGGGC | GATCGGAGGGGGGGCTATGG AGTCCCCGATGCTTTTGATA TC |
|---|---|---|---|---|---|
| | 21-225_3B12 | | SEQ ID NO:4705 | SEQ ID NO:12717 | SEQ ID NO:20729 |
| | | AA | YYGMH | VISYDGSNKYYADSVKG | DRRGGYGVPDAFDI |
| | | | SEQ ID NO:4706 | SEQ ID NO:12718 | SEQ ID NO:20730 |
| iPS:393174 | | NA | GGCTATGGCATGCAC | GTTATATCATATGATGGA AGTAATAAATACTATGC AGACTCCGTGAAGGGC | GATCGGGTCTATTGTAGTAG TACCAGCTGCGTCCCTTACT ACGACTACTACGGTATGGAC GTC |
| | 21-225_15D8 | | SEQ ID NO:4707 | SEQ ID NO:12719 | SEQ ID NO:20731 |
| | | AA | GYGMH | VISYDGSNKYYADSVKG | DRVYCSSTSCVPYYDYYGMD V |
| | | | SEQ ID NO:4708 | SEQ ID NO:12720 | SEQ ID NO:20732 |
| iPS:393176 | | NA | AGCTATGGCATGCAC | GTTATATGGTATGATGG AAGTAATAAATACTATG CAGACTCCGTGAAGGGC | GAGGATTCCTATTGTAGTAG TACCAGCTGCCCCTTACTACT ACTACTACGGTATGGACGTC |
| | 21-225_27E7 | | SEQ ID NO:4709 | SEQ ID NO:12721 | SEQ ID NO:20733 |
| | | AA | SYGMH | VIWYDGSNKYYADSVKG | EDSYCSSTSCPYYYYYGMDV |
| | | | SEQ ID NO:4710 | SEQ ID NO:12722 | SEQ ID NO:20734 |
| iPS:393178 | | NA | GGCTACTATATGCAC | TGGATCAACCCTAACAG TGGTGGCACAAACTATG CACAGAAGTTTCAGGGC | GGGTATTACTATGGTTCGGG GAGTTATTATAACGACCTCG ACCCC |
| | 21-225_34D7 | | SEQ ID NO:4711 | SEQ ID NO:12723 | SEQ ID NO:20735 |
| | | AA | GYYMH | WINPNSGGTNYAQKFQG | GYYYGSGSYYNDLDP |
| | | | SEQ ID NO:4712 | SEQ ID NO:12724 | SEQ ID NO:20736 |

FIGURE 49

| iPS:393180 | | NA | AGCTATGCCATGAGC | ACTCTTAGTGGTCGTGGT GGTAGCACATACTACGC AGACTCCGTGAAGGGC | TGGGGACGTGGATACAGCTA TGAGTACTACTACGGTATGG ACGTC |
|---|---|---|---|---|---|
| | 21-225_4G12 | | SEQ ID NO:4713 | SEQ ID NO:12725 | SEQ ID NO:20737 |
| | | AA | SYAMS | TLSGRGGSTYYADSVKG | WGRGYSYEYYYGMDV |
| | | | SEQ ID NO:4714 | SEQ ID NO:12726 | SEQ ID NO:20738 |
| iPS:393182 | | NA | GGCTACTATATGCAC | TGGATCAACCCTAACAG TGGTGGCACAAACTCTG CACAGAAGTTTCAGGGC | TCCTATTACTATGGTTCGGG GAGTTATTATAACGAGTTTG ACTAC |
| | 21-225_4B3 | | SEQ ID NO:4715 | SEQ ID NO:12727 | SEQ ID NO:20739 |
| | | AA | GYYMH | WINPNSGGTNSAQKFQG | SYYYGSGSYYNEFDY |
| | | | SEQ ID NO:4716 | SEQ ID NO:12728 | SEQ ID NO:20740 |
| iPS:393184 | | NA | ACTGGTGGAGTGGGTGTGGG C | CTCATTTATTGGCATGAT GATAAGCGCTACAGTCC ATCTCTGAGGAGC | ATAGTAGCAGTTGCCTTTGA CTAC |
| | 21-225_15H11 | | SEQ ID NO:4717 | SEQ ID NO:12729 | SEQ ID NO:20741 |
| | | AA | TGGVGVG | LIYWHDDKRYSPSLRS | IVAVAFDY |
| | | | SEQ ID NO:4718 | SEQ ID NO:12730 | SEQ ID NO:20742 |
| iPS:393186 | | NA | GGCTACTATATGCAC | TGGATCAACCCTAACAG TGGTGGCACAAAGTATG CACAGAAGTTTCAGGGC | GAGAGGTGTAGTACTACCAG TTGCTATTTAGGAATTACGG GCTACTACGGTATGGACGTC |
| | 21-225_27D9 | | SEQ ID NO:4719 | SEQ ID NO:12731 | SEQ ID NO:20743 |
| | | AA | GYYMH | WINPNSGGTKYAQKFQG | ERCSTTSCYLGITGYYGMDV |
| | | | SEQ ID NO:4720 | SEQ ID NO:12732 | SEQ ID NO:20744 |
| iPS:393188 | | NA | ACTAGTGGAGTGGGTGTGGG C | CTCATTTATTGGAATGAT GATAAGCGCTACAGCCC ATCTCTGAAGAGC | TTAATAGCAGTGACTTTTGA CTCC |
| | 21-225_34B9 | | SEQ ID NO:4721 | SEQ ID NO:12733 | SEQ ID NO:20745 |
| | | AA | TSGVGVG | LIYWNDDKRYSPSLKS | LIAVTFDS |

FIGURE 49

| | | | SEQ ID NO:4722 | SEQ ID NO:12734 | SEQ ID NO:20746 |
|---|---|---|---|---|---|
| iPS:393192 | | NA | AGCTATGGCATGCAC | GTTATATGGAATGATGG AAGTAATAAATACTATG CAGACTCCGTGAAGGGC | GATCGGGTAGCAGCAGCTGG TACCCCCTACTACTACTACG GTATGGACGTC |
| | 21-225_12B1 | | SEQ ID NO:4723 | SEQ ID NO:12735 | SEQ ID NO:20747 |
| | | AA | SYGMH | VIWNDGSNKYYADSVKG | DRVAAAGTPYYYYGMDV |
| | | | SEQ ID NO:4724 | SEQ ID NO:12736 | SEQ ID NO:20748 |
| iPS:393194 | | NA | AACGCCTGGATGAAC | CGTATTAAAAGCAAAAC TGATGGTGGGACAACAG ACTACGCTGCACCCGTG AAAGGC | GATACGGGTCCTATAGCAGC TCGTCTCGCTTACTACTACTA CTACGCTATGGACGTC |
| | 21-225_16D2 | | SEQ ID NO:4725 | SEQ ID NO:12737 | SEQ ID NO:20749 |
| | | AA | NAWMN | RIKSKTDGGTTDYAAPVK G | DTGPIAARLAYYYYYAMDV |
| | | | SEQ ID NO:4726 | SEQ ID NO:12738 | SEQ ID NO:20750 |
| iPS:393196 | | NA | AGCTATGCCATGAGC | GGTATTAGTGGTAGTGG TGGTAGCACATACTACG CAGACTCCGTGAAGGGC | GAATATTGTGGTGGTGACTG CTATTCCCCTTACTACTACTA CTACGGTATGGACGTC |
| | 21-225_16G8 | | SEQ ID NO:4727 | SEQ ID NO:12739 | SEQ ID NO:20751 |
| | | AA | SYAMS | GISGSGGSTYYADSVKG | EYCGGDCYSPYYYYYGMDV |
| | | | SEQ ID NO:4728 | SEQ ID NO:12740 | SEQ ID NO:20752 |
| iPS:393198 | | NA | GGCTATGGCCTGCAC | CTTATATGGTATGATGGA AATAATACATACTATGC AGACTCCGTGAAGGGC | GATAGGGTATATTGTAGTAG TACCAGCTGCTCCCCTTACT ACTACTACTACGGTATGGAC GTC |
| | 21-225_28A11 | | SEQ ID NO:4729 | SEQ ID NO:12741 | SEQ ID NO:20753 |

FIGURE 49

| | | AA | GYGLH | LIWYDGNNTYYADSVKG | DRVYCSSTSCSPYYYYYGMDV |
|---|---|---|---|---|---|
| | | | SEQ ID NO:4730 | SEQ ID NO:12742 | SEQ ID NO:20754 |
| iPS:393200 | 21-225_35E1 | NA | GGCTACTATATGCAC | TGGATCAACCCTAAAAGTGGTGGCACAAATTATGCACAGAAGTTTCAGGGC | GTGTATTACCATGGTTCGGGGAGTTATTATAACGAGTTTGATTAT |
| | | | SEQ ID NO:4731 | SEQ ID NO:12743 | SEQ ID NO:20755 |
| | | AA | GYYMH | WINPKSGGTNYAQKFQG | VYYHGSGSYYNEFDY |
| | | | SEQ ID NO:4732 | SEQ ID NO:12744 | SEQ ID NO:20756 |
| iPS:393202 | 21-225_6B4 | NA | AGCTATGCCATGAGC | GCTATTAGTGGTAGTGGTAGTAGCACATACTACGCAGACTCCGTGAAGGGC | GAATATTGTGGTGGTGACTGCTATTCCCCTTACTACTACTACTACGGTATGGACGTC |
| | | | SEQ ID NO:4733 | SEQ ID NO:12745 | SEQ ID NO:20757 |
| | | AA | SYAMS | AISGSGSSTYYADSVKG | EYCGGDCYSPYYYYYGMDV |
| | | | SEQ ID NO:4734 | SEQ ID NO:12746 | SEQ ID NO:20758 |
| iPS:393204 | 21-225_8C12 | NA | AGCTATGGCATGCAC | CTTATATGGTATGATGGAAGTAATAAATACTATGCAGACTCCGTGAAGGGC | GATCGGGTATCTTGTAGTAGTTCCAGCTGCTATCCTTACTACTACTACGGTATGGACGTC |
| | | | SEQ ID NO:4735 | SEQ ID NO:12747 | SEQ ID NO:20759 |
| | | AA | SYGMH | LIWYDGSNKYYADSVKG | DRVSCSSSSCYPYYYYYGMDV |
| | | | SEQ ID NO:4736 | SEQ ID NO:12748 | SEQ ID NO:20760 |
| iPS:393206 | 21-225_13F6 | NA | GGCTACTATATGCAC | TGGATCAACCCTAACAGTGGTGGCGCAAACTATGCACAGAAGTTTCAGGGC | TCGTTTTACTATGGTTCGGGGACTTATTATAACGAGTTTGACTAC |
| | | | SEQ ID NO:4737 | SEQ ID NO:12749 | SEQ ID NO:20761 |
| | | AA | GYYMH | WINPNSGGANYAQKFQG | SFYYGSGTYYNEFDY |

FIGURE 49

| | | | SEQ ID NO:4738 | SEQ ID NO:12750 | SEQ ID NO:20762 |
|---|---|---|---|---|---|
| iPS:393208 | 21-225_16F3 | NA | GGGCACTATATGCAC | TGGATCAACCCTAACAG TGGTGGCACAAACTATG CACAGAAGTTTCAGGGC | TCGTATTACTATGGTTCGGG GACTTATTATAACGAGTTTG ACTAC |
| | | | SEQ ID NO:4739 | SEQ ID NO:12751 | SEQ ID NO:20763 |
| | | AA | GHYMH | WINPNSGGTNYAQKFQG | SYYYGSGTYYNEFDY |
| | | | SEQ ID NO:4740 | SEQ ID NO:12752 | SEQ ID NO:20764 |
| iPS:393210 | 21-225_17D3 | NA | GGCTACTATATGCAC | TGGATCAACCCTAACAG TGGTGGCACAAACTATG CACAGAAGTTTCAGGGC | GCGAATTACTATGGTTCGGG GAGTTATTATAACGACTTTG ACTAC |
| | | | SEQ ID NO:4741 | SEQ ID NO:12753 | SEQ ID NO:20765 |
| | | AA | GYYMH | WINPNSGGTNYAQKFQG | ANYYGSGSYYNDFDY |
| | | | SEQ ID NO:4742 | SEQ ID NO:12754 | SEQ ID NO:20766 |
| iPS:393212 | 21-225_30H6 | NA | ACTGGTGGAGTGGGTGTGGG C | CTCATTTATTGGCATGAT GATAAGCGCTACAGTCC CTCTCTGAAGAGC | TTAATAGCAGTGGCTTTTGA CTAT |
| | | | SEQ ID NO:4743 | SEQ ID NO:12755 | SEQ ID NO:20767 |
| | | AA | TGGVGVG | LIYWHDDKRYSPSLKS | LIAVAFDY |
| | | | SEQ ID NO:4744 | SEQ ID NO:12756 | SEQ ID NO:20768 |
| iPS:393214 | 21-225_33A1 | NA | GGCTACTATATGCAC | TGGATCAACCCTAACAA TGGTGGCACACACTATG CACAGAAGTTTCAGGGC | GGATATTATTATGCTTCGGG GAGTTATTATAACGACCTCG ACCCC |
| | | | SEQ ID NO:4745 | SEQ ID NO:12757 | SEQ ID NO:20769 |
| | | AA | GYYMH | WINPNNGGTHYAQKFQG | GYYYASGSYYNDLDP |
| | | | SEQ ID NO:4746 | SEQ ID NO:12758 | SEQ ID NO:20770 |
| iPS:393218 | 21 225 14G3 | NA | GGCTACTATATGTAC | TGGATCAACCCTAACAG TGGTGGCACAAACTATG CACAGAAGTTTCAGGGC | TCGTATTTTTATGGTTCGGGG AGTTATTATAACGAGTTTGA CTAC |

1009

FIGURE 49

| | 21-225_14G3 | | SEQ ID NO:4747 | SEQ ID NO:12759 | SEQ ID NO:20771 |
|---|---|---|---|---|---|
| | | AA | GYYMY | WINPNSGGTNYAQKFQG | SYFYGSGSYYNEFDY |
| | | | SEQ ID NO:4748 | SEQ ID NO:12760 | SEQ ID NO:20772 |
| iPS:393222 | 21-225_17F5 | NA | ACTAGTGGAGTGGGTGTGGGC | CTCATTTATTGGGATGATGATAAGCGCTACAGCCCATCTCTGAAGAGC | ATTATAGCAGTGGCTTTTGACTAC |
| | | | SEQ ID NO:4749 | SEQ ID NO:12761 | SEQ ID NO:20773 |
| | | AA | TSGVGVG | LIYWDDDKRYSPSLKS | IIAVAFDY |
| | | | SEQ ID NO:4750 | SEQ ID NO:12762 | SEQ ID NO:20774 |
| iPS:393224 | 21-225_31C2 | NA | ACTGGTGGAGTGGGTGTGGGC | CTCATTTATTGGAATGATGATGAGCGCTACAGCCCATCTCTGAAGAGC | TTAATAGCAGTTTCCTTTGACTAC |
| | | | SEQ ID NO:4751 | SEQ ID NO:12763 | SEQ ID NO:20775 |
| | | AA | TGGVGVG | LIYWNDDERYSPSLKS | LIAVSFDY |
| | | | SEQ ID NO:4752 | SEQ ID NO:12764 | SEQ ID NO:20776 |
| iPS:393226 | 21-225_33E6 | NA | GGCTACTATATGCAC | TGGATCAACCCTAACAGTGGTGGCACAAACTATGCACAGAAGTTTCAGGGC | GTGTATTACTATGGTTCGGGGAGTTATTATAACGAGTTTGACTAC |
| | | | SEQ ID NO:4753 | SEQ ID NO:12765 | SEQ ID NO:20777 |
| | | AA | GYYMH | WINPNSGGTNYAQKFQG | VYYYGSGSYYNEFDY |
| | | | SEQ ID NO:4754 | SEQ ID NO:12766 | SEQ ID NO:20778 |
| iPS:393230 | 21-225_9G9 | NA | GGCTACTATATACAC | TGGATCAACCCTAACAGTGGTGGCACAGACTATGCACAGAAGTTTCAGGGC | TCGTATTACTATGGTTCGGGGACTTATTATAACGAGTTTGACTAC |
| | | | SEQ ID NO:4755 | SEQ ID NO:12767 | SEQ ID NO:20779 |
| | | AA | GYYIH | WINPNSGGTDYAQKFQG | SYYYGSGTYYNEFDY |
| | | | SEQ ID NO:4756 | SEQ ID NO:12768 | SEQ ID NO:20780 |
| iPS:393232 | 21 225 17F12 | NA | AGCTATGCCATGAGC | GCTATTAGTGGTGGTGGTGGTAGCACATACTACGCAGACTCCGTGAAGGGC | TGGGGACGTGGATACAACTATGAGTACTACTACGGTATGGACGTC |

FIGURE 49

| | | | | | |
|---|---|---|---|---|---|
| | 21-225_17F12 | | SEQ ID NO:4757 | SEQ ID NO:12769 | SEQ ID NO:20781 |
| | | AA | SYAMS | AISGGGGSTYYADSVKG | WGRGYNYEYYYGMDV |
| | | | SEQ ID NO:4758 | SEQ ID NO:12770 | SEQ ID NO:20782 |
| iPS:393234 | 21-225_26C10 | NA | GGCTACTATGTGCAC | TGGATCAACCCTAACAG TGGTGGCACAAACTATG CACAGAAGTTTCAGGGC | GAGAGGTGTAGTACTACCAG CTGCTATTTAGGAATTACGG GCTACTACGGTATGGACGTC |
| | | | SEQ ID NO:4759 | SEQ ID NO:12771 | SEQ ID NO:20783 |
| | | AA | GYYVH | WINPNSGGTNYAQKFQG | ERCSTTSCYLGITGYYGMDV |
| | | | SEQ ID NO:4760 | SEQ ID NO:12772 | SEQ ID NO:20784 |
| iPS:393345 | 21-225_5G7 | NA | AGCTATGCCATGAGC | GCTATTAGTGGTAGTGGT GGTAGCACATACTACGC AGACTCCGTGAAGGGC | GAATATTGTGGTGGTGACTG CTATTCCCCTTACTACTACTA CTACGGTATGGACGTC |
| | | | SEQ ID NO:4761 | SEQ ID NO:12773 | SEQ ID NO:20785 |
| | | AA | SYAMS | AISGSGGSTYYADSVKG | EYCGGDCYSPYYYYYGMDV |
| | | | SEQ ID NO:4762 | SEQ ID NO:12774 | SEQ ID NO:20786 |
| iPS:393368 | 21-225_29H8 | NA | AATTATGATATCAAC | TGGATGAACCCTAACAG TGGTAACACAGGCTATG CACAGAGGTTCCAGGGC | AGCAGTGGCTGGTACTACTT TGACTAC |
| | | | SEQ ID NO:4763 | SEQ ID NO:12775 | SEQ ID NO:20787 |
| | | AA | NYDIN | WMNPNSGNTGYAQRFQG | SSGWYYFDY |
| | | | SEQ ID NO:4764 | SEQ ID NO:12776 | SEQ ID NO:20788 |
| iPS:393565 | 21-225_34B11 | NA | GGCTACTATATGCAC | TGGATCAACCCTAACAG TGGTGGCACAAACTATG CACAGAAGTTTCAGGGC | GTGTATTTCTATGGTTCGGG GAGTTATTATAACGAGTTTG ACTAC |
| | | | SEQ ID NO:4765 | SEQ ID NO:12777 | SEQ ID NO:20789 |
| | | AA | GYYMH | WINPNSGGTNYAQKFQG | VYFYGSGSYYNEFDY |
| | | | SEQ ID NO:4766 | SEQ ID NO:12778 | SEQ ID NO:20790 |

FIGURE 49

| iPS:393802 | | NA | ACCGCCTGGATGAAC | CGGATTAAAAACAAAAT TGATGGTGGGACAACAG ACTACGTTGCACCCGTG AAAGGC | GAAGGCTGGAACACGGACTA C |
|---|---|---|---|---|---|
| | 21-225_3D12 | | SEQ ID NO:4767 | SEQ ID NO:12779 | SEQ ID NO:20791 |
| | | AA | TAWMN | RIKNKIDGGTTDYVAPVK G | EGWNTDY |
| | | | SEQ ID NO:4768 | SEQ ID NO:12780 | SEQ ID NO:20792 |
| iPS:393804 | | NA | AGAAGTAGTTATTACTGGGG C | AATATCTATTATAGTGGG ACCACCTACTACAACCC GTCCCTCAAGAGT | CATGGAAAAGACTGGGGCCT TGACTAC |
| | 21-225_5H7 | | SEQ ID NO:4769 | SEQ ID NO:12781 | SEQ ID NO:20793 |
| | | AA | RSSYYWG | NIYYSGTTYYNPSLKS | HGKDWGLDY |
| | | | SEQ ID NO:4770 | SEQ ID NO:12782 | SEQ ID NO:20794 |
| iPS:393806 | | NA | AGAAGTAGTTACTACTGGGG C | AATATCTATTATAGTGGG ATCCCCTACTACAACCCG TCCCTCAAGAGT | CACAGCAGCAGCTGGTCTCT TGACTAC |
| | 21-225_6A6 | | SEQ ID NO:4771 | SEQ ID NO:12783 | SEQ ID NO:20795 |
| | | AA | RSSYYWG | NIYYSGIPYYNPSLKS | HSSSWSLDY |
| | | | SEQ ID NO:4772 | SEQ ID NO:12784 | SEQ ID NO:20796 |
| iPS:393808 | | NA | AGCTATACCATGAAC | TCCATTAGTGGTAGTGGT AGTTACATATACTACGC AGACTCAGTGAAGGGC | GGGAGCAGCTCGTCCGGGTT TGACTAC |
| | 21-225_1A2 | | SEQ ID NO:4773 | SEQ ID NO:12785 | SEQ ID NO:20797 |
| | | AA | SYTMN | SISGSGSYIYYADSVKG | GSSSSGFDY |
| | | | SEQ ID NO:4774 | SEQ ID NO:12786 | SEQ ID NO:20798 |
| iPS:393810 | | NA | AGCTCTGCCATGAGC | GCTATTAGTGGTCGTGGT GGTAACACATTCTACAC AGACTCCGTGAAGGGC | CTGGGGAAAGACTACTACTA CTACGGTATGGACGTC |
| | 21-225_5A4 | | SEQ ID NO:4775 | SEQ ID NO:12787 | SEQ ID NO:20799 |

FIGURE 49

| | | AA | SSAMS | AISGRGGNTFYTDSVKG | LGKDYYYYGMDV |
|---|---|---|---|---|---|
| | | | SEQ ID NO:4776 | SEQ ID NO:12788 | SEQ ID NO:20800 |
| iPS:393812 | | NA | GACTATGGCATGCAC | GTTATATGGTTTGATGGA AGTAATAAATACTATGC AGACTCCGTGAAGGGC | GATCTTGGCTGGACGGAAGA GTAC |
| | 21-225_6A11 | | SEQ ID NO:4777 | SEQ ID NO:12789 | SEQ ID NO:20801 |
| | | AA | DYGMH | VIWFDGSNKYYADSVKG | DLGWTEEY |
| | | | SEQ ID NO:4778 | SEQ ID NO:12790 | SEQ ID NO:20802 |
| iPS:393814 | | NA | AGGAGTAGTTACTACTGGGG C | AATATCTATTATAGTGGG GCCACCTACTACAACCC GTCCCTCAAGAGT | CATAGCGGCAGCTGGTCCCT TGACTAC |
| | 21-225_7F4 | | SEQ ID NO:4779 | SEQ ID NO:12791 | SEQ ID NO:20803 |
| | | AA | RSSYYWG | NIYYSGATYYNPSLKS | HSGSWSLDY |
| | | | SEQ ID NO:4780 | SEQ ID NO:12792 | SEQ ID NO:20804 |
| iPS:393816 | | NA | AGAAGTAGTTCCTACTGGGG C | AATATCTATTATAGTGGG AGCGCCTACTACATTCCG TCCCTCAAGAGT | CACAGCAGCAGCTGGTCTCT TGACTGC |
| | 21-225_6D4 | | SEQ ID NO:4781 | SEQ ID NO:12793 | SEQ ID NO:20805 |
| | | AA | RSSSYWG | NIYYSGSAYYIPSLKS | HSSSWSLDC |
| | | | SEQ ID NO:4782 | SEQ ID NO:12794 | SEQ ID NO:20806 |
| iPS:393818 | | NA | AGCTATGGCATGCAC | GTTATTTGGTATGATAGA AGTAATAACTACTATGC AGACTCCGTGAAGGGC | GAACTGGGGTTCCGGTCTGA CTAC |
| | 21-225_6G12 | | SEQ ID NO:4783 | SEQ ID NO:12795 | SEQ ID NO:20807 |
| | | AA | SYGMH | VIWYDRSNNYYADSVKG | ELGFRSDY |
| | | | SEQ ID NO:4784 | SEQ ID NO:12796 | SEQ ID NO:20808 |

FIGURE 49

| iPS:393820 | | NA | GACTTTGGCATGCAC | GTTATTTGGTATGAAGA AAATAATCAATACTATG CAGACTCCGTGAAGGGC | GAGCTGGGGTTCCGGTCTGA CTAC |
|---|---|---|---|---|---|
| | 21-225_8H7 | | SEQ ID NO:4785 | SEQ ID NO:12797 | SEQ ID NO:20809 |
| | | AA | DFGMH | VIWYEENNQYYADSVKG | ELGFRSDY |
| | | | SEQ ID NO:4786 | SEQ ID NO:12798 | SEQ ID NO:20810 |
| iPS:393822 | | NA | AACTATGGCATGCAC | GTTATATGGTATGAGGA AAGTAATAAATACTATA CAGACTCCGTGAAGGGC | GAAGTGGGATTCACTGAGGA CTAC |
| | 21-225_15B11 | | SEQ ID NO:4787 | SEQ ID NO:12799 | SEQ ID NO:20811 |
| | | AA | NYGMH | VIWYEESNKYYTDSVKG | EVGFTEDY |
| | | | SEQ ID NO:4788 | SEQ ID NO:12800 | SEQ ID NO:20812 |
| iPS:393824 | | NA | AGCTATGCCATGAGC | ATTATTAGTGGTCGTGGT GGTAACACATTCTACGC AGACTCCGTGAAGGGC | CGTGTAGCAGTGGCTGGCTC GGAGGCTTTTGCTATC |
| | 21-225_10F12 | | SEQ ID NO:4789 | SEQ ID NO:12801 | SEQ ID NO:20813 |
| | | AA | SYAMS | IISGRGGNTFYADSVKG | RVAVAGSEAFAI |
| | | | SEQ ID NO:4790 | SEQ ID NO:12802 | SEQ ID NO:20814 |
| iPS:393826 | | NA | AACTATGGCATGCAC | GTTATTTGGTATGAAGA AAATAATCAATACTATG CAGACTCCGTGAAGGGC | GAACTGGGGTTCCGGTCTGA CTAC |
| | 21-225_10G5 | | SEQ ID NO:4791 | SEQ ID NO:12803 | SEQ ID NO:20815 |
| | | AA | NYGMH | VIWYEENNQYYADSVKG | ELGFRSDY |
| | | | SEQ ID NO:4792 | SEQ ID NO:12804 | SEQ ID NO:20816 |

FIGURE 49

| iPS:393828 | | NA | GACTATGGCATGCAC | GTTATTTGGTATGAAGAC AATAATCAATACTATGC AGACTCCGTGAAGGGC | GAACTGGGGTTCCGGTCTGA CTAC |
|---|---|---|---|---|---|
| | 21-225_10H12 | | SEQ ID NO:4793 | SEQ ID NO:12805 | SEQ ID NO:20817 |
| | | AA | DYGMH | VIWYEDNNQYYADSVKG | ELGFRSDY |
| | | | SEQ ID NO:4794 | SEQ ID NO:12806 | SEQ ID NO:20818 |
| iPS:393830 | | NA | AGCTATGGCATGCAC | GTTATTTGGTATGAAGA AAATAATCAATACTATG CAGACTCCGTGAAGGGC | GAACTGGGGTTCCGGTCTGA CTAC |
| | 21-225_12A1 | | SEQ ID NO:4795 | SEQ ID NO:12807 | SEQ ID NO:20819 |
| | | AA | SYGMH | VIWYEENNQYYADSVKG | ELGFRSDY |
| | | | SEQ ID NO:4796 | SEQ ID NO:12808 | SEQ ID NO:20820 |
| iPS:393832 | | NA | AGAAGTAGTTATTACTGGGG T | AATATCTATTATAGTGGG ACCACCTACTACAACCC GTCCCTCAAGAGT | CATGGAAAAGACTGGGGCCT TGACTAC |
| | 21-225_14B2 | | SEQ ID NO:4797 | SEQ ID NO:12809 | SEQ ID NO:20821 |
| | | AA | RSSYYWG | NIYYSGTTYYNPSLKS | HGKDWGLDY |
| | | | SEQ ID NO:4798 | SEQ ID NO:12810 | SEQ ID NO:20822 |
| iPS:393836 | | NA | AGCTATACCATGAAC | TCCATTAGTGGTAGTGGT AGTTACATATACTACGC AGACTCAGTGAAGGGC | GTGGCTTCATTTGACTAC |
| | 21-225_15A2 | | SEQ ID NO:4799 | SEQ ID NO:12811 | SEQ ID NO:20823 |
| | | AA | SYTMN | SISGSGSYIYYADSVKG | VASFDY |
| | | | SEQ ID NO:4800 | SEQ ID NO:12812 | SEQ ID NO:20824 |

FIGURE 49

| iPS:393838 | | NA | GACTATGGCATACAC | GTCATTTGGTTTGATGGA AGTAATAAATACTATGC AGACTCCGTGAAGGGC | GATCTTGGCTGGACGGAAGA GTAC |
|---|---|---|---|---|---|
| | 21-225_6G2 | | SEQ ID NO:4801 | SEQ ID NO:12813 | SEQ ID NO:20825 |
| | | AA | DYGIH | VIWFDGSNKYYADSVKG | DLGWTEEY |
| | | | SEQ ID NO:4802 | SEQ ID NO:12814 | SEQ ID NO:20826 |
| iPS:393840 | | NA | AGCTATGGCATGCAC | GTTATATGGCATGATGG AAGTAATAAATACTATG CAGACTCCGTGAAGGGC | GATCTGAGTATGGGCGGTAT GGACGTC |
| | 21-225_3F8 | | SEQ ID NO:4803 | SEQ ID NO:12815 | SEQ ID NO:20827 |
| | | AA | SYGMH | VIWHDGSNKYYADSVKG | DLSMGGMDV |
| | | | SEQ ID NO:4804 | SEQ ID NO:12816 | SEQ ID NO:20828 |
| iPS:393844 | | NA | AACTATGGCATGCAC | GTCATATGGCATGATGG AAGTAATAAATACTATG TAGACTCCGTGAAGGGC | GATGAGAGGCTGGGGATTTT TGACTAC |
| | 21-225_3G7 | | SEQ ID NO:4805 | SEQ ID NO:12817 | SEQ ID NO:20829 |
| | | AA | NYGMH | VIWHDGSNKYYVDSVKG | DERLGIFDY |
| | | | SEQ ID NO:4806 | SEQ ID NO:12818 | SEQ ID NO:20830 |
| iPS:393848 | | NA | GGCTATGCCATGAAC | GTTATTAGTCGTAGTGGT GGTTACACATACTACGC GGACTCCGTGAAGGGC | CGTTTAGCAGTGGCTGGCTC GGAGGCTTTTGATATC |
| | 21-225_4H2 | | SEQ ID NO:4807 | SEQ ID NO:12819 | SEQ ID NO:20831 |
| | | AA | GYAMN | VISRSGGYTYYADSVKG | RLAVAGSEAFDI |
| | | | SEQ ID NO:4808 | SEQ ID NO:12820 | SEQ ID NO:20832 |

FIGURE 49

| iPS:393852 | | NA | AGCTATGGCATGCAC | GTTATATGGCATGATGA AAGTAATAAATACTATA CAGACTCCGTGAAGGGC | GACGAGAGGCTGGGGATTTT TGACTAC |
|---|---|---|---|---|---|
| | 21-225_12A10 | | SEQ ID NO:4809 | SEQ ID NO:12821 | SEQ ID NO:20833 |
| | | AA | SYGMH | VIWHDESNKYYTDSVKG | DERLGIFDY |
| | | | SEQ ID NO:4810 | SEQ ID NO:12822 | SEQ ID NO:20834 |
| iPS:393854 | | NA | GACTATGGCATGCAC | GTTATATGGTATGATGA AAATAATAAATACTATG CAGACTCCGTGAAGGGC | GAACTGGGGTTCCGGTCTGA CTAC |
| | 21-225_7H11 | | SEQ ID NO:4811 | SEQ ID NO:12823 | SEQ ID NO:20835 |
| | | AA | DYGMH | VIWYDENNKYYADSVKG | ELGFRSDY |
| | | | SEQ ID NO:4812 | SEQ ID NO:12824 | SEQ ID NO:20836 |
| iPS:393856 | | NA | GACTATGGCATGCAC | GTTATATGGTATGACGA AAGTAATAAATACTATG AAGACTCCGTGAAGGGC | GAAGTGGGGATTCCGGTCTGA CTAC |
| | 21-225_14C2 | | SEQ ID NO:4813 | SEQ ID NO:12825 | SEQ ID NO:20837 |
| | | AA | DYGMH | VIWYDESNKYYEDSVKG | EVGFRSDY |
| | | | SEQ ID NO:4814 | SEQ ID NO:12826 | SEQ ID NO:20838 |
| iPS:393862 | | NA | AGCTATGCCATGAGC | GTTATTAGTGGTCGTGGT GTTAACACATTCTACGCA GACTCCGTGAAGGGC | CGTATAGCAGTGGCTGGCTC GGAGGCTTTTGATATC |
| | 21-225_5G2 | | SEQ ID NO:4815 | SEQ ID NO:12827 | SEQ ID NO:20839 |
| | | AA | SYAMS | VISGRGVNTFYADSVKG | RIAVAGSEAFDI |
| | | | SEQ ID NO:4816 | SEQ ID NO:12828 | SEQ ID NO:20840 |

FIGURE 49

| iPS:393864 | | NA | AGCTATGTCCTGCAC | GTTATATGGTATGATGG AAGTAATAAATACTATG CAGACTCCGTGAAGGGC | GAAAAGTATACCAGCAGCTG GTACGACTACGGTATGGACG TC |
|---|---|---|---|---|---|
| | 21-225_4C5 | | SEQ ID NO:4817 | SEQ ID NO:12829 | SEQ ID NO:20841 |
| | | AA | SYVLH | VIWYDGSNKYYADSVKG | EKYTSSWYDYGMDV |
| | | | SEQ ID NO:4818 | SEQ ID NO:12830 | SEQ ID NO:20842 |
| iPS:393866 | | NA | GACTTTGGCATGCAC | GTTATTTGGTATGAAGA AAATAATCAATACTATG CAGACTCCGTGAAGGGC | GAGCTGGGGTTCCGGTCTGA CTAC |
| | 21-225_14E3 | | SEQ ID NO:4819 | SEQ ID NO:12831 | SEQ ID NO:20843 |
| | | AA | DFGMH | VIWYEENNQYYADSVKG | ELGFRSDY |
| | | | SEQ ID NO:4820 | SEQ ID NO:12832 | SEQ ID NO:20844 |
| iPS:393868 | | NA | AGCTATGGCATGCAC | GTTATATGGCATGATGA AACTAATAAATACTATG CAGATTCCGTGAAGGGC | GACGAGAGGCTGGGGATTTT TGACTAC |
| | 21-225_9C11 | | SEQ ID NO:4821 | SEQ ID NO:12833 | SEQ ID NO:20845 |
| | | AA | SYGMH | VIWHDETNKYYADSVKG | DERLGIFDY |
| | | | SEQ ID NO:4822 | SEQ ID NO:12834 | SEQ ID NO:20846 |
| iPS:393870 | | NA | AGCTATGACATGAGC | ACTATTAGTGGTAGTGGT GGTATCACATACTACGC AGACTCCGTGAAGGGC | GATCGGGGCAGCGTC |
| | 21-225_7B1 | | SEQ ID NO:4823 | SEQ ID NO:12835 | SEQ ID NO:20847 |
| | | AA | SYDMS | TISGSGGITYYADSVKG | DRGSV |
| | | | SEQ ID NO:4824 | SEQ ID NO:12836 | SEQ ID NO:20848 |

FIGURE 49

| iPS:393872 | | NA | AGGAGTAGTTACTACTGGGGC | AATATCTATTATAGTGGGAGCACCTACTACAACCCGTCCGTCAAGAGT | CATGGAAAAGACTGGGGCCTTGAAGAC |
|---|---|---|---|---|---|
| | 21-225_2A11 | | SEQ ID NO:4825 | SEQ ID NO:12837 | SEQ ID NO:20849 |
| | | AA | RSSYYWG | NIYYSGSTYYNPSVKS | HGKDWGLED |
| | | | SEQ ID NO:4826 | SEQ ID NO:12838 | SEQ ID NO:20850 |
| iPS:393874 | | NA | AACTATGGCATGCAC | GTTATTTGGTATGAAGAAAATAATCAATACTATGCAGACTCCGTGAAGGGC | GAAATGGGGTTCCTGTCTGACTAC |
| | 21-225_4C8 | | SEQ ID NO:4827 | SEQ ID NO:12839 | SEQ ID NO:20851 |
| | | AA | NYGMH | VIWYEENNQYYADSVKG | EMGFLSDY |
| | | | SEQ ID NO:4828 | SEQ ID NO:12840 | SEQ ID NO:20852 |
| iPS:393876 | | NA | GACTATGGCATGCAC | GTTATATGGTTTGATGGAAGTAATAAATACTATGCAGACTCCGTGAAGGGC | GATCTTGGCTGGACGGAAGAGTAC |
| | 21-225_9A1 | | SEQ ID NO:4829 | SEQ ID NO:12841 | SEQ ID NO:20853 |
| | | AA | DYGMH | VIWFDGSNKYYADSVKG | DLGWTEEY |
| | | | SEQ ID NO:4830 | SEQ ID NO:12842 | SEQ ID NO:20854 |
| iPS:393878 | | NA | AGCTATGCCATGAAC | GTTATTAGTGGTAGTGGTGGTAGCACATACTACGCAGACTCCGTGAAGGGC | AGGTATACCAGTGACTGGCATGATGCTTTTGATATC |
| | 21-225_7G12 | | SEQ ID NO:4831 | SEQ ID NO:12843 | SEQ ID NO:20855 |
| | | AA | SYAMN | VISGSGGSTYYADSVKG | RYTSDWHDAFDI |
| | | | SEQ ID NO:4832 | SEQ ID NO:12844 | SEQ ID NO:20856 |
| iPS:393880 | 21 225 15A1 | NA | AGAAGTAGTTACTACTGGGGC | AGTATCTATTATAGTGGGAGCGCCCAGTACAACCCGTCCCTCAAGAGT | CTGAGCAGCAGCTGGTCTTTTGACTAC |

FIGURE 49

| | 21-225_15A1 | | SEQ ID NO:4833 | SEQ ID NO:12845 | SEQ ID NO:20857 |
|---|---|---|---|---|---|
| | | AA | RSSYYWG | SIYYSGSAQYNPSLKS | LSSSWSFDY |
| | | | SEQ ID NO:4834 | SEQ ID NO:12846 | SEQ ID NO:20858 |
| iPS:393882 | 21-225_15E3 | NA | AGCTATGGCATGCAC | GTTATATGGTATGAGGAAAATAATAAACACTATGCAGACTCCGTGAAGGGC | GAGCTGGGGTTCCTCTCTGACTAC |
| | | | SEQ ID NO:4835 | SEQ ID NO:12847 | SEQ ID NO:20859 |
| | | AA | SYGMH | VIWYEENNKHYADSVKG | ELGFLSDY |
| | | | SEQ ID NO:4836 | SEQ ID NO:12848 | SEQ ID NO:20860 |
| iPS:393884 | 21-225_16F4 | NA | AACTATGGCATGCAC | GTTATATGGTATGAAGGAAGTAATCAATACTATGGAGACTCCGTGAAGGGC | GAGCTGGGGTTCCTCTCTGACTAC |
| | | | SEQ ID NO:4837 | SEQ ID NO:12849 | SEQ ID NO:20861 |
| | | AA | NYGMH | VIWYEGSNQYYGDSVKG | ELGFLSDY |
| | | | SEQ ID NO:4838 | SEQ ID NO:12850 | SEQ ID NO:20862 |
| iPS:393886 | 21-225_2G9 | NA | CCTAATTACTACTGGGGC | AGTATCTATTATAGTGGAAGCACCTCCTACAACCCGTCCCTCAACAGT | CTAAGCAGCAACTGGGACTTTGACAAC |
| | | | SEQ ID NO:4839 | SEQ ID NO:12851 | SEQ ID NO:20863 |
| | | AA | PNYYWG | SIYYSGSTSYNPSLNS | LSSNWDFDN |
| | | | SEQ ID NO:4840 | SEQ ID NO:12852 | SEQ ID NO:20864 |
| iPS:393888 | 21-225_3E3 | NA | AGCTATGTCATGAGC | ATTATTAGTGGTCGTGGTGGTAACACATTCTACGCAGACTCCGTGAAGGGC | CGTTTAGCAGTGGCTGGCTCGGAGGCTTTTGATATC |
| | | | SEQ ID NO:4841 | SEQ ID NO:12853 | SEQ ID NO:20865 |
| | | AA | SYVMS | IISGRGGNTFYADSVKG | RLAVAGSEAFDI |
| | | | SEQ ID NO:4842 | SEQ ID NO:12854 | SEQ ID NO:20866 |

FIGURE 49

| iPS:393890 | | NA | AGTTACTCCTGGAGC | CGTATCTATACCAGTGG GAGCACCAACTACATCC CCTCCCTCAAGAGT | GATTTGAAGAGCAGTGGCTG CCTTTTCTTTGACTAC |
|---|---|---|---|---|---|
| | 21-225_4B1 | | SEQ ID NO:4843 | SEQ ID NO:12855 | SEQ ID NO:20867 |
| | | AA | SYSWS | RIYTSGSTNYIPSLKS | DLKSSGCLFFDY |
| | | | SEQ ID NO:4844 | SEQ ID NO:12856 | SEQ ID NO:20868 |
| iPS:393892 | | NA | AGCTATGGCATGCAC | ATTATATCATATGTTGGA AAGAATAAATATTATGC AGACTCCGTGAAGGGC | CGGGGAAACAGCTATGGCGG GTACGGTATGGACGTC |
| | 21-225_6G7 | | SEQ ID NO:4845 | SEQ ID NO:12857 | SEQ ID NO:20869 |
| | | AA | SYGMH | IISYVGKNKYYADSVKG | RGNSYGGYGMDV |
| | | | SEQ ID NO:4846 | SEQ ID NO:12858 | SEQ ID NO:20870 |
| iPS:393894 | | NA | AGCTATAGCATGAAC | TCCATTAGTGGTAGTAGT ACTTACATATACTACGCA GACTCAGTGAAGGGC | GTGGCTTCATTTGACTAC |
| | 21-225_5E11 | | SEQ ID NO:4847 | SEQ ID NO:12859 | SEQ ID NO:20871 |
| | | AA | SYSMN | SISGSSTYIYYADSVKG | VASFDY |
| | | | SEQ ID NO:4848 | SEQ ID NO:12860 | SEQ ID NO:20872 |
| iPS:393896 | | NA | AGCTATAGCATGAAC | TCCATTAGTGGTAGTAGT ACTTACATATACTACGCA GACTCAGTGAAGGGC | GTGGCTTCATTTGACTAC |
| | 21-225_2A4 | | SEQ ID NO:4849 | SEQ ID NO:12861 | SEQ ID NO:20873 |
| | | AA | SYSMN | SISGSSTYIYYADSVKG | VASFDY |
| | | | SEQ ID NO:4850 | SEQ ID NO:12862 | SEQ ID NO:20874 |
| iPS:393898 | | NA | AGCTATGCCATGAGC | ATTATTAGTGGTCGTGGT GGTAACACATTCTACGC AGACTCCGTGAAGGGC | CGTATAGCAGTGGCTGGCTC GGAGGCTTTTGCTATC |
| | 21-225_5F7 | | SEQ ID NO:4851 | SEQ ID NO:12863 | SEQ ID NO:20875 |
| | | AA | SYAMS | IISGRGGNTFYADSVKG | RIAVAGSEAFAI |

FIGURE 49

|  |  |  | SEQ ID NO:4852 | SEQ ID NO:12864 | SEQ ID NO:20876 |
|---|---|---|---|---|---|
| iPS:393900 | | NA | AACTATGGCATGCAC | GTTATATGGCATGATGG AAGTAATAAATACTATG TAGACTCCGTGAAGGGC | GATGAGAGGCTGGGGATTTT TGACTAC |
| | 21-225_10E12 | | SEQ ID NO:4853 | SEQ ID NO:12865 | SEQ ID NO:20877 |
| | | AA | NYGMH | VIWHDGSNKYYVDSVKG | DERLGIFDY |
| | | | SEQ ID NO:4854 | SEQ ID NO:12866 | SEQ ID NO:20878 |
| iPS:393902 | | NA | GACTATGGCATGCAC | GTTATATGGTATGATGG AAGTAATAAATACTATG CAGACTCCGTGAAGGGC | GAGAAGTATAGCAGCAGCTG GTACGACTACGGTATGGACG TC |
| | 21-225_14E10 | | SEQ ID NO:4855 | SEQ ID NO:12867 | SEQ ID NO:20879 |
| | | AA | DYGMH | VIWYDGSNKYYADSVKG | EKYSSSWYDYGMDV |
| | | | SEQ ID NO:4856 | SEQ ID NO:12868 | SEQ ID NO:20880 |
| iPS:393904 | | NA | ACCTATAACATGCAC | GTTATATGGTATGATGG AAGTGATAGATACTCTG CAGACTCCGTGAAGGGC | GATCGGGCCTATAGCAGCTC GTCTGACTTC |
| | 21-225_8H11 | | SEQ ID NO:4857 | SEQ ID NO:12869 | SEQ ID NO:20881 |
| | | AA | TYNMH | VIWYDGSDRYSADSVKG | DRAYSSSSDF |
| | | | SEQ ID NO:4858 | SEQ ID NO:12870 | SEQ ID NO:20882 |
| iPS:393906 | | NA | AGTTATACCATGAAC | TCCATTAGTGGTAGTAGT AGTTACATATACTACGC GGACTCAGTGAAGGGC | GATCGGGGCAGTGGC |
| | 21-225_13D3 | | SEQ ID NO:4859 | SEQ ID NO:12871 | SEQ ID NO:20883 |
| | | AA | SYTMN | SISGSSSYIYYADSVKG | DRGSG |
| | | | SEQ ID NO:4860 | SEQ ID NO:12872 | SEQ ID NO:20884 |

FIGURE 49

| iPS:393908 | | NA | AACTATGTCATACAC | GTTATATGGTATGATGG AAGTAATAAATACTATG CAGACTCCGTGAAGGGC | GAAAAGTATAGCAGCAGCTG GTACGACTACGGTATGGACG TC |
|---|---|---|---|---|---|
| | 21-225_10E9 | | SEQ ID NO:4861 | SEQ ID NO:12873 | SEQ ID NO:20885 |
| | | AA | NYVIH | VIWYDGSNKYYADSVKG | EKYSSSWYDYGMDV |
| | | | SEQ ID NO:4862 | SEQ ID NO:12874 | SEQ ID NO:20886 |
| iPS:393910 | | NA | AGCTATGGCATGCAC | GTTATATCATATGGTGGA AGTAATAATTACTATGC AGACTCCGTGAAGGGC | CGGGGATACAGCTATGGCGG GTACGGTATGGACGTC |
| | 21-225_15F10 | | SEQ ID NO:4863 | SEQ ID NO:12875 | SEQ ID NO:20887 |
| | | AA | SYGMH | VISYGGSNNYYADSVKG | RGYSYGGYGMDV |
| | | | SEQ ID NO:4864 | SEQ ID NO:12876 | SEQ ID NO:20888 |
| iPS:393912 | | NA | AACTATGGCATGCAC | GTTATATGGTATGAAGG AAGTAATCAATACTATG GAGACTCCGTGAAGGGC | GAGCTGGGGTTCCTCTCTGA TTAC |
| | 21-225_16F6 | | SEQ ID NO:4865 | SEQ ID NO:12877 | SEQ ID NO:20889 |
| | | AA | NYGMH | VIWYEGSNQYYGDSVKG | ELGFLSDY |
| | | | SEQ ID NO:4866 | SEQ ID NO:12878 | SEQ ID NO:20890 |
| iPS:393914 | | NA | AGCTATAGCATGAAC | TCCATTAGTGGTAGTAGT ACTTACATATACTACGCA GACTCAGTGAAGGGC | GTGGCTTCATTCGACTAC |
| | 21-225_16B8 | | SEQ ID NO:4867 | SEQ ID NO:12879 | SEQ ID NO:20891 |
| | | AA | SYSMN | SISGSSTYIYYADSVKG | VASFDY |
| | | | SEQ ID NO:4868 | SEQ ID NO:12880 | SEQ ID NO:20892 |
| iPS:393916 | | NA | AGCTATGTCATGCAC | GTTATATGGTATGATGG AAGTAATAAATACTATG CAGACTCCGTGAAGGGC | GAGAAGTATAGCAGCAGCTG GTACGACTACGGTATGGACG TC |
| | 21 225 2G4 | | | | |

FIGURE 49

| | | | SEQ ID NO:4869 | SEQ ID NO:12881 | SEQ ID NO:20893 |
|---|---|---|---|---|---|
| | 21-225_2G4 | AA | SYVMH | VIWYDGSNKYYADSVKG | EKYSSSWYDYGMDV |
| | | | SEQ ID NO:4870 | SEQ ID NO:12882 | SEQ ID NO:20894 |
| iPS:393920 | | NA | AGCTATGGCATGCAC | ATTATATGGCATGATGG AAGTAATAAATACTATG TAGACTCCGTGAAGGGC | GATGAGAGGCTGGGGATTTT TGACTAC |
| | 21-225_1H12 | | SEQ ID NO:4871 | SEQ ID NO:12883 | SEQ ID NO:20895 |
| | | AA | SYGMH | IIWHDGSNKYYVDSVKG | DERLGIFDY |
| iPS:393922 | | NA | SEQ ID NO:4872 | SEQ ID NO:12884 | SEQ ID NO:20896 |
| | | | AGCTATGGCATGCAC | GTTATATGGTATGAGGA AAATAATAAATACTATG TAGACTCCGTGAAGGGC | GAACTAGGCTTCCAGTCTGA CTAC |
| | 21-225_2B2 | | SEQ ID NO:4873 | SEQ ID NO:12885 | SEQ ID NO:20897 |
| | | AA | SYGMH | VIWYEENNKYYVDSVKG | ELGFQSDY |
| iPS:393926 | | NA | SEQ ID NO:4874 | SEQ ID NO:12886 | SEQ ID NO:20898 |
| | | | AGCTATGGCATGCAC | GTTATATGGCATGATGG AAGTAATAAATACTATG CAGACTCCGTGAAGGGC | GATCTGAGAATGGGCGGTAT GGACGTC |
| | 21-225_4G4 | | SEQ ID NO:4875 | SEQ ID NO:12887 | SEQ ID NO:20899 |
| | | AA | SYGMH | VIWHDGSNKYYADSVKG | DLRMGGMDV |
| iPS:393928 | | NA | SEQ ID NO:4876 | SEQ ID NO:12888 | SEQ ID NO:20900 |
| | 21 225 4E10 | | CGTAGTAGTTACTACTGGGG C | AGTGTCTATTATAGTGGG GCCACCTCCTACAACCC GTCCCTCAAGAGT | CTAAGCAGCAACTGGGACTT TGACTAC |

FIGURE 49

| | 21-225_4E10 | | SEQ ID NO:4877 | SEQ ID NO:12889 | SEQ ID NO:20901 |
|---|---|---|---|---|---|
| | | AA | RSSYYWG | SVYYSGATSYNPSLKS | LSSNWDFDY |
| | | | SEQ ID NO:4878 | SEQ ID NO:12890 | SEQ ID NO:20902 |
| iPS:393930 | 21-225_7E11 | NA | AGCTTTGGCATGCAC | ATTATATGGCATGATGG AAGTAATAAATACTATG CAGACTCCGTGAAGGGC | GATCTGAGTATGGGCGGTAT GGACGTC |
| | | | SEQ ID NO:4879 | SEQ ID NO:12891 | SEQ ID NO:20903 |
| | | AA | SFGMH | IIWHDGSNKYYADSVKG | DLSMGGMDV |
| | | | SEQ ID NO:4880 | SEQ ID NO:12892 | SEQ ID NO:20904 |
| iPS:393932 | 21-225_10F5 | NA | AGCTATGGCATGCAC | ATTATATGGCATGATGG AAGTAATAAATATTATG TAGACTCCGTGAAGGGC | GATGAGAGGCTGGGGATTTT TGACTAC |
| | | | SEQ ID NO:4881 | SEQ ID NO:12893 | SEQ ID NO:20905 |
| | | AA | SYGMH | IIWHDGSNKYYVDSVKG | DERLGIFDY |
| | | | SEQ ID NO:4882 | SEQ ID NO:12894 | SEQ ID NO:20906 |
| iPS:393934 | 21-225_13E6 | NA | AACTATGGCATGCAC | GTTATATGGTATGATGA AAATAATAAATATTATA TAGACTCCGTGAAGGGC | GAATTGGGGTTCCGGTCTGA CTAC |
| | | | SEQ ID NO:4883 | SEQ ID NO:12895 | SEQ ID NO:20907 |
| | | AA | NYGMH | VIWYDENNKYYIDSVKG | ELGFRSDY |
| | | | SEQ ID NO:4884 | SEQ ID NO:12896 | SEQ ID NO:20908 |
| iPS:393936 | 21-225_14A11 | NA | AGCTATGCCATGAAC | GTTATTAGTGGTAGAGG TGGTAGTACATACTACG CAGACTCCGTGAAGGGC | AGGATAGCAGCTGGTATGGA GTACTTCGATCTC |
| | | | SEQ ID NO:4885 | SEQ ID NO:12897 | SEQ ID NO:20909 |
| | | AA | SYAMN | VISGRGGSTYYADSVKG | RIAAGMEYFDL |
| | | | SEQ ID NO:4886 | SEQ ID NO:12898 | SEQ ID NO:20910 |

FIGURE 49

| iPS:393940 | | NA | AGCTATGCCATGACC | GTTATTAGTGGTAGTGGT GGTAGCACATTCTACGC AGACTCCGTGAAGGGC | TATTGTAGTAGTACCAGGTG CCCTTATGATGCCTTTGATAT C |
|---|---|---|---|---|---|
| | 21-225_16B2 | | SEQ ID NO:4887 | SEQ ID NO:12899 | SEQ ID NO:20911 |
| | | AA | SYAMT | VISGSGGSTFYADSVKG | YCSSTRCPYDAFDI |
| | | | SEQ ID NO:4888 | SEQ ID NO:12900 | SEQ ID NO:20912 |
| iPS:393942 | | NA | AATTATGATATCAAC | TGGATGCACCCTAACAG TGGTGCCACCGGCTATG CACAGAGGTTCCAGGGC | AGCAGTGGCTGGGAGGTCTT TGACTAC |
| | 21-225_11E5 | | SEQ ID NO:4889 | SEQ ID NO:12901 | SEQ ID NO:20913 |
| | | AA | NYDIN | WMHPNSGATGYAQRFQG | SSGWEVFDY |
| | | | SEQ ID NO:4890 | SEQ ID NO:12902 | SEQ ID NO:20914 |
| iPS:393944 | | NA | AGTTATACCATGAAC | TCCATTAGTGGTAGTGGT AGTTACATATACTACGC AGACTCAGTGAAGGGC | GTAGCAGCCTTTGACTCC |
| | 21-225_14D6 | | SEQ ID NO:4891 | SEQ ID NO:12903 | SEQ ID NO:20915 |
| | | AA | SYTMN | SISGSGSYIYYADSVKG | VAAFDS |
| | | | SEQ ID NO:4892 | SEQ ID NO:12904 | SEQ ID NO:20916 |
| iPS:393946 | | NA | AGCTATAGCATGAAC | TCCATTAGTGGTAGTAGT ACTTACAAATACTACGC AGACTCAGTGAAGGGC | GATCGTGGGAGCTAC |
| | 21-225_16A4 | | SEQ ID NO:4893 | SEQ ID NO:12905 | SEQ ID NO:20917 |
| | | AA | SYSMN | SISGSSTYKYYADSVKG | DRGSY |
| | | | SEQ ID NO:4894 | SEQ ID NO:12906 | SEQ ID NO:20918 |
| iPS:393948 | | NA | GACTATGGCATGCAC | GTTATATGGTTTGATGGA AGTAATAAATACTATGT AGACTCCGTGAAGGGC | GATCTTGGCTGGACGGAAGA GTAC |
| | 21-225_16A5 | | SEQ ID NO:4895 | SEQ ID NO:12907 | SEQ ID NO:20919 |

FIGURE 49

| | | | | | |
|---|---|---|---|---|---|
| | | AA | DYGMH | VIWFDGSNKYYVDSVKG | DLGWTEEY |
| | | | SEQ ID NO:4896 | SEQ ID NO:12908 | SEQ ID NO:20920 |
| iPS:393950 | 21-225_3H10 | NA | AGCTATGTCATGCAC | GTTATATGGTATGATGG AAGTAATAAATACTATG CAGACTCCGTGAAGGGC | GAGAGATATAGCAGTGGCTG GTATGACTACGGTTTGGACG TC |
| | | | SEQ ID NO:4897 | SEQ ID NO:12909 | SEQ ID NO:20921 |
| | | AA | SYVMH | VIWYDGSNKYYADSVKG | ERYSSGWYDYGLDV |
| | | | SEQ ID NO:4898 | SEQ ID NO:12910 | SEQ ID NO:20922 |
| iPS:393952 | 21-225_1F1 | NA | AGCTATAACATGAAC | TCCATTAGTAGTAGTAGT AGTTACATATACTACGC AGACTCAGTGAAGGGC | GTTAACCTCTTTGACTAC |
| | | | SEQ ID NO:4899 | SEQ ID NO:12911 | SEQ ID NO:20923 |
| | | AA | SYNMN | SISSSSSYIYYADSVKG | VNLFDY |
| | | | SEQ ID NO:4900 | SEQ ID NO:12912 | SEQ ID NO:20924 |
| iPS:393954 | 21-225_4H6 | NA | GACTACTATTTGCAC | TGGATCCACCCTAACAG TGGTGGCACAAACTATG CACAGAAGTTTCAGGGC | GATGGTACCAGCTCGTTTGA CTAC |
| | | | SEQ ID NO:4901 | SEQ ID NO:12913 | SEQ ID NO:20925 |
| | | AA | DYYLH | WIHPNSGGTNYAQKFQG | DGTSSFDY |
| | | | SEQ ID NO:4902 | SEQ ID NO:12914 | SEQ ID NO:20926 |
| iPS:393956 | 21-225_4D7 | NA | AGCTATGCCATGAGC | GTTCTTAGTGGTAGTGGT GGTAGCACATTCTACGC AGACTCCGTGAAGGGC | TATTGTAGTAGTGCCAGGTG CCCTTATGATGCCTTTGATAT C |
| | | | SEQ ID NO:4903 | SEQ ID NO:12915 | SEQ ID NO:20927 |
| | | AA | SYAMS | VLSGSGGSTFYADSVKG | YCSSARCPYDAFDI |
| | | | SEQ ID NO:4904 | SEQ ID NO:12916 | SEQ ID NO:20928 |

FIGURE 49

| iPS:393958 | | NA | AGCTATACCATGAAC | TCCATTAGTGGTAGTAGT AGTTACATATACTACGC AGACTCAGTGAAGGGC | GGGAGCAGCTCGTCCGGCTT TGACTAC |
|---|---|---|---|---|---|
| | 21-225_5H2 | | SEQ ID NO:4905 | SEQ ID NO:12917 | SEQ ID NO:20929 |
| | | AA | SYTMN | SISGSSSYIYYADSVKG | GSSSSGFDY |
| | | | SEQ ID NO:4906 | SEQ ID NO:12918 | SEQ ID NO:20930 |
| iPS:393960 | | NA | GACTATGGCATGCAC | GTTATATGGTATGATGTA ACTAATAAATACTATGC AGACTCCGTGAAGGGC | GAACTTGGCTGGTACGAGGA CTAC |
| | 21-225_7G2 | | SEQ ID NO:4907 | SEQ ID NO:12919 | SEQ ID NO:20931 |
| | | AA | DYGMH | VIWYDVTNKYYADSVKG | ELGWYEDY |
| | | | SEQ ID NO:4908 | SEQ ID NO:12920 | SEQ ID NO:20932 |
| iPS:393962 | | NA | AGAAGTAGTTACTACTGGGG C | AATATCTATTATAGTGGG AGCACCTACTACATCCC GTCCCTCAAGAGT | CACAGTACCAGCTGGTCTCT TGACCAC |
| | 21-225_7H7 | | SEQ ID NO:4909 | SEQ ID NO:12921 | SEQ ID NO:20933 |
| | | AA | RSSYYWG | NIYYSGSTYYIPSLKS | HSTSWSLDH |
| | | | SEQ ID NO:4910 | SEQ ID NO:12922 | SEQ ID NO:20934 |
| iPS:393964 | | NA | AGCTATGGCATGCAC | ATTATATGGTATGATGG AAGTAATAAATACTATG CAGACTCCGTGAAGGGC | GATCTGAGTATGGGCGGTAT GGACGTC |
| | 21-225_6G1 | | SEQ ID NO:4911 | SEQ ID NO:12923 | SEQ ID NO:20935 |
| | | AA | SYGMH | IIWYDGSNKYYADSVKG | DLSMGGMDV |
| | | | SEQ ID NO:4912 | SEQ ID NO:12924 | SEQ ID NO:20936 |

FIGURE 49

| iPS:393966 | | NA | AGCTGTGTCATGCAC | GTTATATGGTATGATGG AAGTAATAAATACTATG CAGACTCCGTGAAGGGC | GAAAGGTATACCAGTGGCTG GCACGACTACGGTATGGACG TC |
|---|---|---|---|---|---|
| | 21-225_7F8 | | SEQ ID NO:4913 | SEQ ID NO:12925 | SEQ ID NO:20937 |
| | | AA | SCVMH | VIWYDGSNKYYADSVKG | ERYTSGWHDYGMDV |
| | | | SEQ ID NO:4914 | SEQ ID NO:12926 | SEQ ID NO:20938 |
| iPS:393968 | | NA | AGCTATGCCATGAAC | GCTATTAGTGGTAGTGGT GGTTACACATACTACGC AGACTCCGTGAAGGGC | GGGGGGTCCCTCTTCTAC |
| | 21-225_5A5 | | SEQ ID NO:4915 | SEQ ID NO:12927 | SEQ ID NO:20939 |
| | | AA | SYAMN | AISGSGGYTYYADSVKG | GGSLFY |
| | | | SEQ ID NO:4916 | SEQ ID NO:12928 | SEQ ID NO:20940 |
| iPS:393972 | | NA | AACTATGTCATGCAC | GTTATATGGTATGATGG AAGTAATAAATACTATG CAGACTCCGTGAAGGGC | GAGAAGTATAGCAGCAGCTG GTACGACTACGGTATGGACG TC |
| | 21-225_7C9 | | SEQ ID NO:4917 | SEQ ID NO:12929 | SEQ ID NO:20941 |
| | | AA | NYVMH | VIWYDGSNKYYADSVKG | EKYSSSWYDYGMDV |
| | | | SEQ ID NO:4918 | SEQ ID NO:12930 | SEQ ID NO:20942 |
| iPS:393974 | | NA | AACTATGGCATGCAC | GTTATTTGGTATGAAGA AAATAATCAATACTATG CAGACTCCGTGAAGGGC | GAACTGGGGTTCCGGTCTGA CTAC |
| | 21-225_7C4 | | SEQ ID NO:4919 | SEQ ID NO:12931 | SEQ ID NO:20943 |
| | | AA | NYGMH | VIWYEENNQYYADSVKG | ELGFRSDY |
| | | | SEQ ID NO:4920 | SEQ ID NO:12932 | SEQ ID NO:20944 |

FIGURE 49

| iPS:393976 | | NA | GACTATGGCATGCAC | GTTATATGGTATGATGA AAATAATAAATACTATG TAGACTCCGTGAAGGGC | GAATTGGGGTTCCGGTCTGA CTAC |
|---|---|---|---|---|---|
| | 21-225_7E9 | | SEQ ID NO:4921 | SEQ ID NO:12933 | SEQ ID NO:20945 |
| | | AA | DYGMH | VIWYDENNKYYVDSVKG | ELGFRSDY |
| | | | SEQ ID NO:4922 | SEQ ID NO:12934 | SEQ ID NO:20946 |
| iPS:393978 | | NA | AGCTATGTCATGCAC | GTTATATGGTATGATGG AAGTAATAAATACTATG CAGACTCCGTGAAGGGC | GAGAAGTATAGCAGCAGCTG GTACGACTACGGTATGGACG TC |
| | 21-225_4C12 | | SEQ ID NO:4923 | SEQ ID NO:12935 | SEQ ID NO:20947 |
| | | AA | SYVMH | VIWYDGSNKYYADSVKG | EKYSSSWYDYGMDV |
| | | | SEQ ID NO:4924 | SEQ ID NO:12936 | SEQ ID NO:20948 |
| iPS:393980 | | NA | AGCTATGCCATGAAC | GTTATTAGTGGTCGTGGT GGTAACACATTCTACGC AGACTCCGTGAAGGGC | CGTTTGGCAGTGGCTGGCTC GGAGGCTTTTGATATC |
| | 21-225_6D3 | | SEQ ID NO:4925 | SEQ ID NO:12937 | SEQ ID NO:20949 |
| | | AA | SYAMN | VISGRGGNTFYADSVKG | RLAVAGSEAFDI |
| | | | SEQ ID NO:4926 | SEQ ID NO:12938 | SEQ ID NO:20950 |
| iPS:393982 | | NA | AGCTATAGCATGAAC | TCCATTAGTAGTAGTAGT AGTTACATATACTACGC AGACTCAGTGAAGGGC | GATCGTGGGAGCCTC |
| | 21-225_6C12 | | SEQ ID NO:4927 | SEQ ID NO:12939 | SEQ ID NO:20951 |
| | | AA | SYSMN | SISSSSSYIYYADSVKG | DRGSL |
| | | | SEQ ID NO:4928 | SEQ ID NO:12940 | SEQ ID NO:20952 |

FIGURE 49

| iPS:393984 | | NA | AGCTATGGCATGCAC | GTTATATGGTATGATGTT ACTAATAAAAAGTATGC AGACTCCGTGAAGGGC | GAAAAGGGGGGTCTATTTGA CTAC |
|---|---|---|---|---|---|
| | 21-225_4F12 | | SEQ ID NO:4929 | SEQ ID NO:12941 | SEQ ID NO:20953 |
| | | AA | SYGMH | VIWYDVTNKKYADSVKG | EKGGLFDY |
| | | | SEQ ID NO:4930 | SEQ ID NO:12942 | SEQ ID NO:20954 |
| iPS:393986 | | NA | AACTATGTCATGCAC | GTTATATGGTATGATGG AAGTAATAAATACTATG CAGACTCCGTGAAGGGC | GAGAAGTATAGCAGCAACTG GTACGACTACGGTATGGACG TC |
| | 21-225_7G4 | | SEQ ID NO:4931 | SEQ ID NO:12943 | SEQ ID NO:20955 |
| | | AA | NYVMH | VIWYDGSNKYYADSVKG | EKYSSNWYDYGMDV |
| | | | SEQ ID NO:4932 | SEQ ID NO:12944 | SEQ ID NO:20956 |
| iPS:393988 | | NA | AGCTATAGCATGAAC | TCCATTAGTAGTAGTAGT AGTTACATATACTACGC AGACTCAGTGAAGGGC | GCTAACCTCTTTGACTAC |
| | 21-225_7F10 | | SEQ ID NO:4933 | SEQ ID NO:12945 | SEQ ID NO:20957 |
| | | AA | SYSMN | SISSSSSYIYYADSVKG | ANLFDY |
| | | | SEQ ID NO:4934 | SEQ ID NO:12946 | SEQ ID NO:20958 |
| iPS:393990 | | NA | AGGAGTACTTACTACTGGGG C | AGTATCTATTATAGTGGG AGCACCTCCTACAGCCC GTCCCTCAAGAGT | CTGAACAGCAGCTGGTCTTT TGACTAC |
| | 21-225_11G7 | | SEQ ID NO:4935 | SEQ ID NO:12947 | SEQ ID NO:20959 |
| | | AA | RSTYYWG | SIYYSGSTSYSPSLKS | LNSSWSFDY |
| | | | SEQ ID NO:4936 | SEQ ID NO:12948 | SEQ ID NO:20960 |

FIGURE 49

| iPS:393992 | | NA | AGCAATAGCATGAAC | TACATTAGTAGTAGTAGT AGTACCATATACTACGC AGACTCTGTGAAGGGC | GGAGGTGGGAGCCCTTTTGA CTAC |
|---|---|---|---|---|---|
| | 21-225_14H8 | | SEQ ID NO:4937 | SEQ ID NO:12949 | SEQ ID NO:20961 |
| | | AA | SNSMN | YISSSSSTIYYADSVKG | GGGSPFDY |
| | | | SEQ ID NO:4938 | SEQ ID NO:12950 | SEQ ID NO:20962 |
| iPS:393994 | | NA | AGCTATGGCATGCAC | GTTATATGGTATGATGA AAATAATAAATACTATG TAGACTCCGTGAAGGGC | GAATTGGGGTTCCGGTCTGA CTAC |
| | 21-225_8C9 | | SEQ ID NO:4939 | SEQ ID NO:12951 | SEQ ID NO:20963 |
| | | AA | SYGMH | VIWYDENNKYYVDSVKG | ELGFRSDY |
| | | | SEQ ID NO:4940 | SEQ ID NO:12952 | SEQ ID NO:20964 |
| iPS:393996 | | NA | GACTATGTCATGCAC | GTTATATGGTATGATGG AAGTAATAAATACTATG CAGACTCCGTGAAGGGC | GAGAAGTATAGCAGCAGCTG GTACGACTACGGTTTGGACG TC |
| | 21-225_15C11 | | SEQ ID NO:4941 | SEQ ID NO:12953 | SEQ ID NO:20965 |
| | | AA | DYVMH | VIWYDGSNKYYADSVKG | EKYSSSWYDYGLDV |
| | | | SEQ ID NO:4942 | SEQ ID NO:12954 | SEQ ID NO:20966 |
| iPS:393998 | | NA | GACTATGGCATGCAC | GTTATATGGTATGATGTA ACTAATAAATACTATGC AGACTCCGTGAAGGGC | GAGCTTGGCTGGTACGAGGA CTAC |
| | 21-225_12B12 | | SEQ ID NO:4943 | SEQ ID NO:12955 | SEQ ID NO:20967 |
| | | AA | DYGMH | VIWYDVTNKYYADSVKG | ELGWYEDY |
| | | | SEQ ID NO:4944 | SEQ ID NO:12956 | SEQ ID NO:20968 |

FIGURE 49

| iPS:394000 | | NA | AGCTATGGCATGCAC | GTTATATCATATGGTGGA AGTAATAAAGACTCTGC AGACTCCGTGAAGGGC | CGGGGATACAGCTATGGCGG GTACGGTATGGACGTC |
|---|---|---|---|---|---|
| | 21-225_11A2 | | SEQ ID NO:4945 | SEQ ID NO:12957 | SEQ ID NO:20969 |
| | | AA | SYGMH | VISYGGSNKDSADSVKG | RGYSYGGYGMDV |
| | | | SEQ ID NO:4946 | SEQ ID NO:12958 | SEQ ID NO:20970 |
| iPS:394002 | 21-225_15G7 | NA | AGGAGTAGTTCCTACTGGGG C | AGTATCTATTATAGTGGG TACACCTATTACACCCCG TCCCTCAAGAGT | CTGAGCAGCAGTTGGTCTTT TGACTTC |
| | | | SEQ ID NO:4947 | SEQ ID NO:12959 | SEQ ID NO:20971 |
| | | AA | RSSSYWG | SIYYSGYTYYTPSLKS | LSSSWSFDF |
| | | | SEQ ID NO:4948 | SEQ ID NO:12960 | SEQ ID NO:20972 |
| iPS:394004 | 21-225_13A1 | NA | AGCTATGGCATGCAC | GTTATATCATATGCGGG AACTAATCAATACTATG CAGACTCCGTGAAGGGC | CGGGGATACAGCTATGGCGG GTACGGTATGGACGTC |
| | | | SEQ ID NO:4949 | SEQ ID NO:12961 | SEQ ID NO:20973 |
| | | AA | SYGMH | VISYAGTNQYYADSVKG | RGYSYGGYGMDV |
| | | | SEQ ID NO:4950 | SEQ ID NO:12962 | SEQ ID NO:20974 |
| iPS:394006 | 21-225_15C2 | NA | AGCTATGGCATGCAC | ATAATATCATATGGTGG ACGTAATAATCACTATG CAGACTCCGTGAAGGGC | CGGGGATACAGCTATGGCGG GTACGGTATGGACGTC |
| | | | SEQ ID NO:4951 | SEQ ID NO:12963 | SEQ ID NO:20975 |
| | | AA | SYGMH | IISYGGRNNHYADSVKG | RGYSYGGYGMDV |
| | | | SEQ ID NO:4952 | SEQ ID NO:12964 | SEQ ID NO:20976 |
| iPS:394008 | 21-225_15H8 | NA | AGCTATGTCATGAAC | TCCATTAGTGGTAGTAGT ACTTACATATACTGCGCA GACTCAATCAAGGGC | GATCGAGGCTCCATC |
| | | | SEQ ID NO:4953 | SEQ ID NO:12965 | SEQ ID NO:20977 |

FIGURE 49

| | | AA | SYVMN | SISGSSTYIYCADSIKG | DRGSI |
|---|---|---|---|---|---|
| | | | SEQ ID NO:4954 | SEQ ID NO:12966 | SEQ ID NO:20978 |
| iPS:394010 | 21-225_12G5 | NA | AACTATGGCATCTAC | GTTATATCATATGATGGAAGTAATAAATACTATGCAGACTCCGTGAAGGGC | GATCGGGGAGCAGTGGCTGCTTACTACTACTACGGTATAGACGTC |
| | | | SEQ ID NO:4955 | SEQ ID NO:12967 | SEQ ID NO:20979 |
| | | AA | NYGIY | VISYDGSNKYYADSVKG | DRGAVAAYYYYGIDV |
| | | | SEQ ID NO:4956 | SEQ ID NO:12968 | SEQ ID NO:20980 |
| iPS:394012 | 21-225_15A3 | NA | AGCTATGGCATTCAC | GTTATATGGCATGATGGAAGTAATAAATACTATGCAGACTCCGTGAAGGGC | GATCTGAGTATGGGCGGTATGGACGTC |
| | | | SEQ ID NO:4957 | SEQ ID NO:12969 | SEQ ID NO:20981 |
| | | AA | SYGIH | VIWHDGSNKYYADSVKG | DLSMGGMDV |
| | | | SEQ ID NO:4958 | SEQ ID NO:12970 | SEQ ID NO:20982 |
| iPS:394014 | 21-225_8G6 | NA | AGCTATGTCATGAAC | GTTATTAGTGGTCGTGGTGGTAACACATTCTACGCAGACTCCGTGAAGGGC | CGTTTGGCAGTGGCTGGCTCGGAGGCTTTTGATATC |
| | | | SEQ ID NO:4959 | SEQ ID NO:12971 | SEQ ID NO:20983 |
| | | AA | SYVMN | VISGRGGNTFYADSVKG | RLAVAGSEAFDI |
| | | | SEQ ID NO:4960 | SEQ ID NO:12972 | SEQ ID NO:20984 |
| iPS:394016 | 21-225_13D4 | NA | AGCTATGGCATGCAC | GTTATATGGCATGATGGAAGTAATAAATACTATGCAGACTCCGTGAAGGGC | GATCTGAGTATGGGCGGTATGGACGTC |
| | | | SEQ ID NO:4961 | SEQ ID NO:12973 | SEQ ID NO:20985 |
| | | AA | SYGMH | VIWHDGSNKYYADSVKG | DLSMGGMDV |

FIGURE 49

| | | | SEQ ID NO:4962 | SEQ ID NO:12974 | SEQ ID NO:20986 |
|---|---|---|---|---|---|
| iPS:394018 | | NA | AGCTATGTCATGAGC | GGTATTAGTGGTAGTGG TGGTAGCACAAACAACG CAGACTCCGTGAAGGGC | AGCTCCTTGTTTGACTAC |
| | 21-225_15B1 | | SEQ ID NO:4963 | SEQ ID NO:12975 | SEQ ID NO:20987 |
| | | AA | SYVMS | GISGSGGSTNNADSVKG | SSLFDY |
| | | | SEQ ID NO:4964 | SEQ ID NO:12976 | SEQ ID NO:20988 |
| iPS:394020 | | NA | AACTATGGCATGCAC | GTTATATGGTATGATGA AAGTAATAAATACTATG AAGACTCCGTGAAGGGC | GAAGTGGGGTTTCTTTCTGA CTAC |
| | 21-225_15H10 | | SEQ ID NO:4965 | SEQ ID NO:12977 | SEQ ID NO:20989 |
| | | AA | NYGMH | VIWYDESNKYYEDSVKG | EVGFLSDY |
| | | | SEQ ID NO:4966 | SEQ ID NO:12978 | SEQ ID NO:20990 |
| iPS:394022 | | NA | AGCTATGCCATGAAC | GTTATTAGTCGTAGTGGT GGTTACACATACTACGC GGACTCCGTGAAGGGC | CGTTTAGCAGTGGCTGGCTC GGAGGCTTTTGATATC |
| | 21-225_16H6 | | SEQ ID NO:4967 | SEQ ID NO:12979 | SEQ ID NO:20991 |
| | | AA | SYAMN | VISRSGGYTYYADSVKG | RLAVAGSEAFDI |
| | | | SEQ ID NO:4968 | SEQ ID NO:12980 | SEQ ID NO:20992 |
| iPS:394024 | | NA | AGCTATGGCATGCAC | GTTATATGGTATGATGA AAGTAATAAATACTATG CAGACTCCGTGAAGGGC | GAACTGGGGTTTCTCTCTGA CTAC |
| | 21-225_16B7 | | SEQ ID NO:4969 | SEQ ID NO:12981 | SEQ ID NO:20993 |
| | | AA | SYGMH | VIWYDESNKYYADSVKG | ELGFLSDY |
| | | | SEQ ID NO:4970 | SEQ ID NO:12982 | SEQ ID NO:20994 |

FIGURE 49

| iPS:394026 | | NA | AGCTATGTCATGACC | ACTATTAGTGGTAGTGGTGGTTGGACATATTATGCAGACTCCGTGAAGGGC | AGCTCCTTGTTTGACTAT |
|---|---|---|---|---|---|
| | 21-225_16C7 | | SEQ ID NO:4971 | SEQ ID NO:12983 | SEQ ID NO:20995 |
| | | AA | SYVMT | TISGSGGWTYYADSVKG | SSLFDY |
| | | | SEQ ID NO:4972 | SEQ ID NO:12984 | SEQ ID NO:20996 |
| iPS:394029 | | NA | AGCTATGGCATGCAC | ATTATATCATATGCTGGAAGTAATAAATCCTATGCAGACTCCGTGAAGGGC | CGGGGATACAGCTATGGCGGGTACGGTATGGACGTC |
| | 21-225_1B12 | | SEQ ID NO:4973 | SEQ ID NO:12985 | SEQ ID NO:20997 |
| | | AA | SYGMH | IISYAGSNKSYADSVKG | RGYSYGGYGMDV |
| | | | SEQ ID NO:4974 | SEQ ID NO:12986 | SEQ ID NO:20998 |
| iPS:394033 | | NA | AGCTATAGCATGAAC | TCCATTAGTGGTAGTAGTAGTTACATATACTACGCAGACTCAGTGAAGGGC | GTTAACAACTTTGACTAC |
| | 21-225_5F4 | | SEQ ID NO:4975 | SEQ ID NO:12987 | SEQ ID NO:20999 |
| | | AA | SYSMN | SISGSSSYIYYADSVKG | VNNFDY |
| | | | SEQ ID NO:4976 | SEQ ID NO:12988 | SEQ ID NO:21000 |
| iPS:394035 | | NA | AATTATGGCATGCAC | ATTATATCATATGCTGGAAGTAATAAATACTATGCAGACTCCGTGAAGGGC | CGTATAACAGCTCGTCTCTACTACGGTATGGACGTC |
| | 21-225_5G9 | | SEQ ID NO:4977 | SEQ ID NO:12989 | SEQ ID NO:21001 |
| | | AA | NYGMH | IISYAGSNKYYADSVKG | RITARLYYGMDV |
| | | | SEQ ID NO:4978 | SEQ ID NO:12990 | SEQ ID NO:21002 |
| iPS:394037 | | NA | AGGAGTAGTTACTACTGGGGC | AATATTTATTATAGTGGGAGCACCTACGACAACCCGTCCCTCAAGAGT | CATGGAAAAGACTGGGGCCTTGACTAC |
| | 21-225_4F4 | | SEQ ID NO:4979 | SEQ ID NO:12991 | SEQ ID NO:21003 |
| | | AA | RSSYYWG | NIYYSGSTYDNPSLKS | HGKDWGLDY |

FIGURE 49

| | | | SEQ ID NO:4980 | SEQ ID NO:12992 | SEQ ID NO:21004 |
|---|---|---|---|---|---|
| iPS:394041 | | NA | AACTATGTCATGCAC | GTTATATGGTATGATGG AAGTAATAAATACTATG CAGACTCCGTGAAGGGC | GAGGTATATAGCAGTGGCTG GTACGACTACGGTATGGACG TC |
| | 21-225_5E5 | | SEQ ID NO:4981 | SEQ ID NO:12993 | SEQ ID NO:21005 |
| | | AA | NYVMH | VIWYDGSNKYYADSVKG | EVYSSGWYDYGMDV |
| | | | SEQ ID NO:4982 | SEQ ID NO:12994 | SEQ ID NO:21006 |
| iPS:394043 | | NA | AGCTATGCCATGAAC | GTTATTAGTGGTCGTGGT ATTAACACATTCTACGCA GACTCCGTGAAGGGC | CGTTTAGCAGTGGCTGGCTC GGAGGCTTTTGATATC |
| | 21-225_3B1 | | SEQ ID NO:4983 | SEQ ID NO:12995 | SEQ ID NO:21007 |
| | | AA | SYAMN | VISGRGINTFYADSVKG | RLAVAGSEAFDI |
| | | | SEQ ID NO:4984 | SEQ ID NO:12996 | SEQ ID NO:21008 |
| iPS:394045 | | NA | AGGAGTAGTTACTACTGGGG C | AATATTTATTATAGTGGG AACACCTACAACAACCC GTCCCTCAAGAGT | CATGGAAAAGACTGGGGCCT TGACTAC |
| | 21-225_4H4 | | SEQ ID NO:4985 | SEQ ID NO:12997 | SEQ ID NO:21009 |
| | | AA | RSSYYWG | NIYYSGNTYNNPSLKS | HGKDWGLDY |
| | | | SEQ ID NO:4986 | SEQ ID NO:12998 | SEQ ID NO:21010 |
| iPS:394047 | | NA | AGCTATGGCATGCAC | ATTATATCATATGTTGGA AATAATAAATACTATGC AGACTCCGTGAAGGGC | CGGGGATACAGCTATGGCGG GTACGGTATGGACGTC |
| | 21-225_5E6 | | SEQ ID NO:4987 | SEQ ID NO:12999 | SEQ ID NO:21011 |
| | | AA | SYGMH | IISYVGNNKYYADSVKG | RGYSYGGYGMDV |
| | | | SEQ ID NO:4988 | SEQ ID NO:13000 | SEQ ID NO:21012 |

FIGURE 49

| iPS:394049 | | NA | AGAAGTAGTTACTACTGGGG C | AGTATCTATTATAGTGGG AGCACCTACTACAACCC GTCCCTCAAGAGT | CTTAGCAGCAGCTGGGACTT CCAGCAC |
|---|---|---|---|---|---|
| | 21-225_13H5 | | SEQ ID NO:4989 | SEQ ID NO:13001 | SEQ ID NO:21013 |
| | | AA | RSSYYWG | SIYYSGSTYYNPSLKS | LSSSWDFQH |
| | | | SEQ ID NO:4990 | SEQ ID NO:13002 | SEQ ID NO:21014 |
| iPS:394051 | | NA | AACTATGCCATGAAC | GCTATTAGTGGTGGTGGT GGTAACACATTCTACGC AGACTCCGTGAAGGGC | CGTATAGCAGTGGCTGGCTC GGAGGCTTTTGCTATC |
| | 21-225_9E5 | | SEQ ID NO:4991 | SEQ ID NO:13003 | SEQ ID NO:21015 |
| | | AA | NYAMN | AISGGGGNTFYADSVKG | RIAVAGSEAFAI |
| | | | SEQ ID NO:4992 | SEQ ID NO:13004 | SEQ ID NO:21016 |
| iPS:394053 | | NA | AGAAGTAGTTACTACTGGGG C | AGTATTTATTATAGTGGG AGCGCCCAGTACAACCC GTCCCTCAAGAGT | CTGAGCAGCAGCTGGTCTTT TGACTAC |
| | 21-225_11F10 | | SEQ ID NO:4993 | SEQ ID NO:13005 | SEQ ID NO:21017 |
| | | AA | RSSYYWG | SIYYSGSAQYNPSLKS | LSSSWSFDY |
| | | | SEQ ID NO:4994 | SEQ ID NO:13006 | SEQ ID NO:21018 |
| iPS:394055 | | NA | AGCCAGAGCATGAAC | TACATTAGTATTAGTAGT ACCATATACTATGCAGA CTCTGTGAAGGGC | GGAGGTGGGAGCCCTTTTGA CTCC |
| | 21-225_9C8 | | SEQ ID NO:4995 | SEQ ID NO:13007 | SEQ ID NO:21019 |
| | | AA | SQSMN | YISISSTIYYADSVKG | GGGSPFDS |
| | | | SEQ ID NO:4996 | SEQ ID NO:13008 | SEQ ID NO:21020 |
| iPS:394057 | | NA | AGAAGTAGTTACTACTGGGG C | AATATCTATTATAGTGGG TATCCCTACTACAATCCG TCCCTCAAGAGT | CATAGCACCAGCTGGTCCCT TGACTAC |
| | 21-225_15H1 | | SEQ ID NO:4997 | SEQ ID NO:13009 | SEQ ID NO:21021 |
| | | AA | RSSYYWG | NIYYSGYPYYNPSLKS | HSTSWSLDY |
| | | | SEQ ID NO:4998 | SEQ ID NO:13010 | SEQ ID NO:21022 |

FIGURE 49

| iPS:394059 | | NA | AGCTATGGCATGCAC | GTTATTTGGTATGAAGA AAATAATCAATACTATG CAGACTCCGTGAAGGGC | GAACTGGGGTTCCGGTCTGA CTAC |
|---|---|---|---|---|---|
| | 21-225_9E8 | | SEQ ID NO:4999 | SEQ ID NO:13011 | SEQ ID NO:21023 |
| | | AA | SYGMH | VIWYEENNQYYADSVKG | ELGFRSDY |
| | | | SEQ ID NO:5000 | SEQ ID NO:13012 | SEQ ID NO:21024 |
| iPS:394061 | | NA | AGCTATAGCATGAAC | TCCATTAGTAGTAGTAGT AGTTACATATACTACGC AGACTCAGTGAAGGGC | TTAGGGGACTAC |
| | 21-225_12D2 | | SEQ ID NO:5001 | SEQ ID NO:13013 | SEQ ID NO:21025 |
| | | AA | SYSMN | SISSSSSYIYYADSVKG | LGDY |
| | | | SEQ ID NO:5002 | SEQ ID NO:13014 | SEQ ID NO:21026 |
| iPS:394063 | | NA | AGGAGTAGTTACTACTGGGG C | AGTATCTATTATAGTGGG AGCGCCTATCACAACCC GTCCCTCAAGAGT | CTGAGCAGCAGCTGGTCCTT TGACTAC |
| | 21-225_16A1 | | SEQ ID NO:5003 | SEQ ID NO:13015 | SEQ ID NO:21027 |
| | | AA | RSSYYWG | SIYYSGSAYHNPSLKS | LSSSWSFDY |
| | | | SEQ ID NO:5004 | SEQ ID NO:13016 | SEQ ID NO:21028 |
| iPS:394065 | | NA | AATTATGATATCAAC | TGGATGAACACTAACAG TGGTAACACAGGCTATG CACAGAAGTTCCAGGGC | AGTCATGGCTGGTTCCTCTTT GACTAC |
| | 21-225_11E2 | | SEQ ID NO:5005 | SEQ ID NO:13017 | SEQ ID NO:21029 |
| | | AA | NYDIN | WMNTNSGNTGYAQKFQG | SHGWFLFDY |
| | | | SEQ ID NO:5006 | SEQ ID NO:13018 | SEQ ID NO:21030 |

FIGURE 49

| iPS:394067 | | NA | GACTATGGCATGCAC | GTTATATGGTTTGATGGA AATAATAAATACTATGT AGATTCCGTGAAGGGC | GAGCTTGCCTGGTCCGAGGA CTAC |
|---|---|---|---|---|---|
| | 21-225_12F2 | | SEQ ID NO:5007 | SEQ ID NO:13019 | SEQ ID NO:21031 |
| | | AA | DYGMH | VIWFDGNNKYYVDSVKG | ELAWSEDY |
| | | | SEQ ID NO:5008 | SEQ ID NO:13020 | SEQ ID NO:21032 |
| iPS:394069 | | NA | AGCTATAGCATGAAC | TCCATTAGTGGTAGTAGT ACTTACATATACTACGCA GACTCAGTGAAGGGC | GTAGCAGCCTTTGACTAC |
| | 21-225_16H1 | | SEQ ID NO:5009 | SEQ ID NO:13021 | SEQ ID NO:21033 |
| | | AA | SYSMN | SISGSSTYIYYADSVKG | VAAFDY |
| | | | SEQ ID NO:5010 | SEQ ID NO:13022 | SEQ ID NO:21034 |
| iPS:394071 | | NA | AGCTATAGCATGAAC | TCCATTAGTAGTAGTAAT AATTACATATACTACGC AGACTCAGTGAAGGGC | TTAGGGGTCTAC |
| | 21-225_10C7 | | SEQ ID NO:5011 | SEQ ID NO:13023 | SEQ ID NO:21035 |
| | | AA | SYSMN | SISSSNNYIYYADSVKG | LGVY |
| | | | SEQ ID NO:5012 | SEQ ID NO:13024 | SEQ ID NO:21036 |
| iPS:394073 | | NA | AGAAGTAGTTACTACTGGGG C | AATATCTATTATAGTGGG AGCACCTACAACAACCC GTCCCTCAAGAGT | CAGGGCAGTGGCTGGGAGGT TGACTAC |
| | 21-225_15C9 | | SEQ ID NO:5013 | SEQ ID NO:13025 | SEQ ID NO:21037 |
| | | AA | RSSYYWG | NIYYSGSTYNNPSLKS | QGSGWEVDY |
| | | | SEQ ID NO:5014 | SEQ ID NO:13026 | SEQ ID NO:21038 |
| iPS:394075 | | NA | AGAAGTAGTTACTACTGGGG C | AATATCTATTATAGTGGG TATCCCTACTACAATCCG TCCCTCAAGAGT | CATAGCACCAGCTGGTCCCT TGACTAC |
| | 21-225_8D12 | | SEQ ID NO:5015 | SEQ ID NO:13027 | SEQ ID NO:21039 |

FIGURE 49

| | | AA | RSSYYWG | NIYYSGYPYYNPSLKS | HSTSWSLDY |
|---|---|---|---|---|---|
| | | | SEQ ID NO:5016 | SEQ ID NO:13028 | SEQ ID NO:21040 |
| iPS:394077 | 21-225_8E12 | NA | AGCTATGCCATGAGC | ATTATTAGTGGTCGTGGT GGTAACACATTCTACGC AGACTCCGTGAAGGGC | CGTATGGCAGTGGCTGGCTC GGAGGCTTTTGATATC |
| | | | SEQ ID NO:5017 | SEQ ID NO:13029 | SEQ ID NO:21041 |
| | | AA | SYAMS | IISGRGGNTFYADSVKG | RMAVAGSEAFDI |
| | | | SEQ ID NO:5018 | SEQ ID NO:13030 | SEQ ID NO:21042 |
| iPS:394079 | 21-225_11F5 | NA | AGGAGTAGTTACTACTGGGG C | AATATTTATTATAGTGGG AGCACCTACACCAACCC GTCCCTCAAGAGT | CATGGAAAAGACTGGGGCCT TGACAAC |
| | | | SEQ ID NO:5019 | SEQ ID NO:13031 | SEQ ID NO:21043 |
| | | AA | RSSYYWG | NIYYSGSTYTNPSLKS | HGKDWGLDN |
| | | | SEQ ID NO:5020 | SEQ ID NO:13032 | SEQ ID NO:21044 |
| iPS:394081 | 21-225_16B3 | NA | AGCTATGGCATGCAC | GTTATATCATATGCTGGA ATTAATAAATCCTATGCA GACTCCGTGAAGGGC | CGGGGATACAGCTATGGCGG GTATGGTATGGACGTC |
| | | | SEQ ID NO:5021 | SEQ ID NO:13033 | SEQ ID NO:21045 |
| | | AA | SYGMH | VISYAGINKSYADSVKG | RGYSYGGYGMDV |
| | | | SEQ ID NO:5022 | SEQ ID NO:13034 | SEQ ID NO:21046 |
| iPS:394083 | 21-225_16E6 | NA | AGCTATGGCATGCAC | GTTATATGGCATGATGG AAGTAATAAATACTATG TAGACTCCGTGAAGGGC | GATCTGAGTATGGGCGGTAT GGACGTC |
| | | | SEQ ID NO:5023 | SEQ ID NO:13035 | SEQ ID NO:21047 |
| | | AA | SYGMH | VIWHDGSNKYYVDSVKG | DLSMGGMDV |
| | | | SEQ ID NO:5024 | SEQ ID NO:13036 | SEQ ID NO:21048 |

FIGURE 49

| iPS:394085 | | NA | AATTATGATATCAAC | TGGATGCACCCTAACAG TGGTAACACAGGCTATG CACAGAAGTTCCAGGGC | AGCAGTGGCTGGTACTTCTT TGACTAC |
|---|---|---|---|---|---|
| | 21-225_8B11 | | SEQ ID NO:5025 | SEQ ID NO:13037 | SEQ ID NO:21049 |
| | | AA | NYDIN | WMHPNSGNTGYAQKFQG | SSGWYFFDY |
| | | | SEQ ID NO:5026 | SEQ ID NO:13038 | SEQ ID NO:21050 |
| iPS:394087 | | NA | AGCTATGCCATGAGC | GTTATTAGTGGTCGTGGT GTTAACACATTCTACGCA GACTCCGTGAAGGGC | CGTATCGCAGTGGCTGGCTC GGAGGCTTTTGATATC |
| | 21-225_11A5 | | SEQ ID NO:5027 | SEQ ID NO:13039 | SEQ ID NO:21051 |
| | | AA | SYAMS | VISGRGVNTFYADSVKG | RIAVAGSEAFDI |
| | | | SEQ ID NO:5028 | SEQ ID NO:13040 | SEQ ID NO:21052 |
| iPS:394089 | | NA | GACTATGGCATGCAC | GTTATATGGTATGATGA AAGTAATAAATACTATG CAGACTCCGTGAAGGGC | GAGCTTGCCTGGTACGAGGA CTAC |
| | 21-225_12E6 | | SEQ ID NO:5029 | SEQ ID NO:13041 | SEQ ID NO:21053 |
| | | AA | DYGMH | VIWYDESNKYYADSVKG | ELAWYEDY |
| | | | SEQ ID NO:5030 | SEQ ID NO:13042 | SEQ ID NO:21054 |
| iPS:394091 | | NA | AGCTATGGCATGCAC | GTTATTTGGTATGAGGA AAGTAATAAATACTATG TAGACTCCGTGAGGGGC | GAACTAGGCTTCCAGTCTGA CTTC |
| | 21-225_13H3 | | SEQ ID NO:5031 | SEQ ID NO:13043 | SEQ ID NO:21055 |
| | | AA | SYGMH | VIWYEESNKYYVDSVRG | ELGFQSDF |
| | | | SEQ ID NO:5032 | SEQ ID NO:13044 | SEQ ID NO:21056 |

FIGURE 49

| iPS:394093 | | NA | GACTATGGCATGCAC | GTTATATGGTATGATGG AAATAATAATTACTATG CAGACTCCGTGAAGGGC | GAGCTTGCCTGGTACGAGGA CTTC |
|---|---|---|---|---|---|
| | 21-225_9D12 | | SEQ ID NO:5033 | SEQ ID NO:13045 | SEQ ID NO:21057 |
| | | AA | DYGMH | VIWYDGNNNYYADSVKG | ELAWYEDF |
| | | | SEQ ID NO:5034 | SEQ ID NO:13046 | SEQ ID NO:21058 |
| iPS:394095 | | NA | AACTATGGCATGCAC | GTTATATGGTATGATGTA AGTAATAAATACTATGC AGACTCCGTGAAGGGC | GAAATGGGCTGGACCGATGA CTGC |
| | 21-225_16H4 | | SEQ ID NO:5035 | SEQ ID NO:13047 | SEQ ID NO:21059 |
| | | AA | NYGMH | VIWYDVSNKYYADSVKG | EMGWTDDC |
| | | | SEQ ID NO:5036 | SEQ ID NO:13048 | SEQ ID NO:21060 |
| iPS:394097 | | NA | GACTATGGCATGCAC | GTTATTTGGTATGATGAA AATAATGAATACTATGC AGACTCCGTGAAGGGC | GAGCTTGCCTGGTACGAGGA CTAC |
| | 21-225_16G7 | | SEQ ID NO:5037 | SEQ ID NO:13049 | SEQ ID NO:21061 |
| | | AA | DYGMH | VIWYDENNEYYADSVKG | ELAWYEDY |
| | | | SEQ ID NO:5038 | SEQ ID NO:13050 | SEQ ID NO:21062 |
| iPS:398470 | | NA | GGCTACTATATGCAC | TGGATCAACCCTAACAG TGGTGGCACAAACTATG TACAGAAGTTTCAGGGC | TCGTTTTTCTATGGTTCGGGG AGTTATTATAACGAATTTGA CTAC |
| | 21-225_14B7 | | SEQ ID NO:5039 | SEQ ID NO:13051 | SEQ ID NO:21063 |
| | | AA | GYYMH | WINPNSGGTNYVQKFQG | SFFYGSGSYYNEFDY |
| | | | SEQ ID NO:5040 | SEQ ID NO:13052 | SEQ ID NO:21064 |

FIGURE 49

| iPS:398472 | | NA | AGCTATGTCATGAGC | ACTATTAGTGTTGGTGGT GGTACCACATACTACGC AGACTCCGTGAAGGGC | TGGGGACGTGGCAACAGCTA TGAGTACTACTACGGTATGG ACGTC |
|---|---|---|---|---|---|
| | 21-225_16E4 | | SEQ ID NO:5041 | SEQ ID NO:13053 | SEQ ID NO:21065 |
| | | AA | SYVMS | TISVGGGTTYYADSVKG | WGRGNSYEYYYGMDV |
| | | | SEQ ID NO:5042 | SEQ ID NO:13054 | SEQ ID NO:21066 |
| iPS:398474 | | NA | AGCTATGCCATGAGC | GTTATTAGTGGTAGTGGT GGTAACACATACTTCGC AGACTCCGTGAAGGGC | AGGGGTATACCAGAGGCTGA TGCTTTTGATATC |
| | 21-225_17B10 | | SEQ ID NO:5043 | SEQ ID NO:13055 | SEQ ID NO:21067 |
| | | AA | SYAMS | VISGSGGNTYFADSVKG | RGIPEADAFDI |
| | | | SEQ ID NO:5044 | SEQ ID NO:13056 | SEQ ID NO:21068 |
| iPS:398476 | | NA | AGCTATGCCATGAGC | GTTATTAGTGGTAGTGGT GGTACCACATTCTACGC AGACTCCGTGAAGGGC | TATTGTAGTAGTACCAGGTG TCCTTATGATGCCTTTGATAT C |
| | 21-225_17C1 | | SEQ ID NO:5045 | SEQ ID NO:13057 | SEQ ID NO:21069 |
| | | AA | SYAMS | VISGSGGTTFYADSVKG | YCSSTRCPYDAFDI |
| | | | SEQ ID NO:5046 | SEQ ID NO:13058 | SEQ ID NO:21070 |
| iPS:398478 | | NA | AGCTATAGCATGAAC | TCCATTAGTGGTAGTAGT AGTTACATGTACTACGC AGACTCAGTGAAGGGC | GATCGTGGGAGCTCC |
| | 21-225_17C10 | | SEQ ID NO:5047 | SEQ ID NO:13059 | SEQ ID NO:21071 |
| | | AA | SYSMN | SISGSSSYMYYADSVKG | DRGSS |
| | | | SEQ ID NO:5048 | SEQ ID NO:13060 | SEQ ID NO:21072 |
| iPS:398480 | | NA | GACTATTATATGCAC | TGGATCAACCCTAACAG TGGTGGCACAAACTATG AACAGAAGTTTCAGGGC | GGATACAGCTATGGGTACAA CTGGTTCGACCCC |
| | 21-225_17G4 | | SEQ ID NO:5049 | SEQ ID NO:13061 | SEQ ID NO:21073 |
| | | AA | DYYMH | WINPNSGGTNYEQKFQG | GYSYGYNWFDP |
| | | | SEQ ID NO:5050 | SEQ ID NO:13062 | SEQ ID NO:21074 |

FIGURE 49

| iPS:398482 | | NA | AGCTATAGCATGAAC | TCCATTAGTGGTAGTAGT AGTTACATATACTACGC AGACTCAGTGAAGGGC | GTGGCTTCATTTGACTAC |
|---|---|---|---|---|---|
| | 21-225_17H6 | | SEQ ID NO:5051 | SEQ ID NO:13063 | SEQ ID NO:21075 |
| | | AA | SYSMN | SISGSSSYIYYADSVKG | VASFDY |
| | | | SEQ ID NO:5052 | SEQ ID NO:13064 | SEQ ID NO:21076 |
| iPS:398484 | | NA | GGCTACTATTTGCAC | TGGATCAACCCTAACAG TAATGGCACAATCTCTGC ACAGAAGTTTCAGGGC | GATGGTACCAGCTCGCTTGA CTAC |
| | 21-225_18D4 | | SEQ ID NO:5053 | SEQ ID NO:13065 | SEQ ID NO:21077 |
| | | AA | GYYLH | WINPNSNGTISAQKFQG | DGTSSLDY |
| | | | SEQ ID NO:5054 | SEQ ID NO:13066 | SEQ ID NO:21078 |
| iPS:398486 | | NA | GGCTACTATATGCAC | TGGATCAATCCTAACAG TGGTGGCACAAACTATG CACAGAAGTTTCAGGGC | GGATACAGCTATGGGTACAA CTGGTTCGACCCC |
| | 21-225_19A1 | | SEQ ID NO:5055 | SEQ ID NO:13067 | SEQ ID NO:21079 |
| | | AA | GYYMH | WINPNSGGTNYAQKFQG | GYSYGYNWFDP |
| | | | SEQ ID NO:5056 | SEQ ID NO:13068 | SEQ ID NO:21080 |
| iPS:398488 | | NA | AACGCCTGGATGAAC | CGTATTAAAAGCAAAAC TGATGGTGGGACAACAG ACTACGCTGCACCCGTG AAAGGC | GATACGGGTCCTATAGCAGC TCGTCTCGCTTACTACTACTA TTACGCTATGGACGTC |
| | 21-225_19F6 | | SEQ ID NO:5057 | SEQ ID NO:13069 | SEQ ID NO:21081 |
| | | AA | NAWMN | RIKSKTDGGTTDYAAPVK G | DTGPIAARLAYYYYYAMDV |
| | | | SEQ ID NO:5058 | SEQ ID NO:13070 | SEQ ID NO:21082 |
| iPS:398490 | | NA | GACTACTATATTCAC | TGGATCAACCCTAACAG TGGTGGGACAAACAATG CACAGAAGTTTCAGGGC | TCGTATTACTATGGTTCGGG GACTTATTATAACGAATTTG ACTAC |
| | 21-225_21D12 | | SEQ ID NO:5059 | SEQ ID NO:13071 | SEQ ID NO:21083 |

FIGURE 49

| | | AA | DYYIH | WINPNSGGTNNAQKFQG | SYYYGSGTYYNEFDY |
|---|---|---|---|---|---|
| | | | SEQ ID NO:5060 | SEQ ID NO:13072 | SEQ ID NO:21084 |
| iPS:398494 | 21-225_21H4 | NA | AGCTATGCCATGAGC | GCTCTTAGTGGTCGTGGT GGTAGCACATACTACGC AGACTCCGTGAAGGGC | TGGGGACGTGGATACAGCTA TGAGTACTACTACGGTATGG ACGTC |
| | | | SEQ ID NO:5061 | SEQ ID NO:13073 | SEQ ID NO:21085 |
| | | AA | SYAMS | ALSGRGGSTYYADSVKG | WGRGYSYEYYYGMDV |
| | | | SEQ ID NO:5062 | SEQ ID NO:13074 | SEQ ID NO:21086 |
| iPS:398496 | 21-225_22D2 | NA | AATTATGATATCAAC | TGGATGCACCCTGACAG TGGTAACACAGGCTATG CACAGAAGTTCCAGGGC | AGTAGTGGCTGGTACTACTT TGACTAC |
| | | | SEQ ID NO:5063 | SEQ ID NO:13075 | SEQ ID NO:21087 |
| | | AA | NYDIN | WMHPDSGNTGYAQKFQG | SSGWYYFDY |
| | | | SEQ ID NO:5064 | SEQ ID NO:13076 | SEQ ID NO:21088 |
| iPS:398498 | 21-225_22E6 | NA | ACTGGTGGAGTGGGTGTGGG C | CTCATTTATTGGAATGAT GATAAGCGCTACAGCCC ATCTCTGAAGAGC | ACTATAGCAGTTCGTGGCTT TGACTAC |
| | | | SEQ ID NO:5065 | SEQ ID NO:13077 | SEQ ID NO:21089 |
| | | AA | TGGVGVG | LIYWNDDKRYSPSLKS | TIAVRGFDY |
| | | | SEQ ID NO:5066 | SEQ ID NO:13078 | SEQ ID NO:21090 |
| iPS:398500 | 21-225_23A11 | NA | AGTTATAGCATGAAC | TCCATTAGTGGTAGTAGT ACTTACATATACTACGCA GACTCAGTGAAGGGC | GTGGCTTCATTTGACTAC |
| | | | SEQ ID NO:5067 | SEQ ID NO:13079 | SEQ ID NO:21091 |
| | | AA | SYSMN | SISGSSTYIYYADSVKG | VASFDY |
| | | | SEQ ID NO:5068 | SEQ ID NO:13080 | SEQ ID NO:21092 |

FIGURE 49

| iPS:398502 | | NA | GGCTACTATCTGCAC | TGGATCAACCCTAACAA TAATGGCACAAACTATG CACAGAAGTTTCAGGGC | GATGGTACCAGCTCGTTTGA CTAC |
|---|---|---|---|---|---|
| | 21-225_23B11 | | SEQ ID NO:5069 | SEQ ID NO:13081 | SEQ ID NO:21093 |
| | | AA | GYYLH | WINPNNNGTNYAQKFQG | DGTSSFDY |
| | | | SEQ ID NO:5070 | SEQ ID NO:13082 | SEQ ID NO:21094 |
| iPS:398504 | | NA | ACTAGTGGAGTGGGTGTGGG C | CTCATTTATTGGAATAAT GATAAGGTCTACAGCCC ATCTCTGAAGAGC | AGGGGACAGCAGCTGGCCCT CGACTAC |
| | 21-225_23D7 | | SEQ ID NO:5071 | SEQ ID NO:13083 | SEQ ID NO:21095 |
| | | AA | TSGVGVG | LIYWNNDKVYSPSLKS | RGQQLALDY |
| | | | SEQ ID NO:5072 | SEQ ID NO:13084 | SEQ ID NO:21096 |
| iPS:398506 | | NA | AATTATGATATCAAC | TGGATGTACCCTAACAG TGGTAACACGGGCTATG CACAGAAGTTCCAGGGC | AGCGGTGGCTGGTACTACTT TGACTAC |
| | 21-225_23G12 | | SEQ ID NO:5073 | SEQ ID NO:13085 | SEQ ID NO:21097 |
| | | AA | NYDIN | WMYPNSGNTGYAQKFQG | SGGWYYFDY |
| | | | SEQ ID NO:5074 | SEQ ID NO:13086 | SEQ ID NO:21098 |
| iPS:398508 | | NA | AGCTATGCCATGAGC | GCTATTAGTGGTCGTGGT GGTAGCACATACTACGC AGACTCCGTGAAGGGC | TGGGGACGTGGATACAGCTA TGAGTACTACTACGGTATGG ACGTC |
| | 21-225_24B1 | | SEQ ID NO:5075 | SEQ ID NO:13087 | SEQ ID NO:21099 |
| | | AA | SYAMS | AISGRGGSTYYADSVKG | WGRGYSYEYYYGMDV |
| | | | SEQ ID NO:5076 | SEQ ID NO:13088 | SEQ ID NO:21100 |
| iPS:398510 | | NA | AATTATGATATCAAC | TGGATGCACCCTAACAG TGGTAACACAGGCTATG CACAGAAGTTCCAGGGC | AGCAGTGGCTGGTATTGGTT CGACCCC |

FIGURE 49

| | 21-225_25A3 | | SEQ ID NO:5077 | SEQ ID NO:13089 | SEQ ID NO:21101 |
|---|---|---|---|---|---|
| | | AA | NYDIN | WMHPNSGNTGYAQKFQG | SSGWYWFDP |
| | | | SEQ ID NO:5078 | SEQ ID NO:13090 | SEQ ID NO:21102 |
| iPS:398512 | 21-225_25E12 | NA | AATTATGATATCAAC | TGGATGAACCCTAACAG TGGTAACACAGGCTATG CACAGAAGTTCCAGGGC | AGCAATGGCTGGTACTACTT TGACTAC |
| | | | SEQ ID NO:5079 | SEQ ID NO:13091 | SEQ ID NO:21103 |
| | | AA | NYDIN | WMNPNSGNTGYAQKFQG | SNGWYYFDY |
| | | | SEQ ID NO:5080 | SEQ ID NO:13092 | SEQ ID NO:21104 |
| iPS:398516 | 21-225_26A9 | NA | AATTATGATATCAAC | TGGATGCACCCTAACAG TGGTAACACAGGCTGTG CACAGAAGTTCCAGGGC | AGCAGTGGCTGGTACTGGTT CGACCCC |
| | | | SEQ ID NO:5081 | SEQ ID NO:13093 | SEQ ID NO:21105 |
| | | AA | NYDIN | WMHPNSGNTGCAQKFQG | SSGWYWFDP |
| | | | SEQ ID NO:5082 | SEQ ID NO:13094 | SEQ ID NO:21106 |
| iPS:398520 | 21-225_31C4 | NA | GGCGATTATATGCAC | TGGATCAGCCCTAAAAA TGGTGGCACAAACTATG CACAGAAGTTTCAGGGC | GATGGAACTGGGTCCTTTGA CTAC |
| | | | SEQ ID NO:5083 | SEQ ID NO:13095 | SEQ ID NO:21107 |
| | | AA | GDYMH | WISPKNGGTNYAQKFQG | DGTGSFDY |
| | | | SEQ ID NO:5084 | SEQ ID NO:13096 | SEQ ID NO:21108 |
| iPS:398522 | 21-225_32A1 | NA | AACTATGATATTAAC | TGGATGAACCCTAACAG TGGTAACACAGGCTATG CACAGAAGTTCCAGGGC | AGCAGTGGCTGGTACTTTTT TGACTAC |
| | | | SEQ ID NO:5085 | SEQ ID NO:13097 | SEQ ID NO:21109 |

FIGURE 49

| | | AA | NYDIN | WMNPNSGNTGYAQKFQG | SSGWYFFDY |
|---|---|---|---|---|---|
| | | | SEQ ID NO:5086 | SEQ ID NO:13098 | SEQ ID NO:21110 |
| iPS:398524 | | NA | AATTATGACATCAAC | TGGATGCACCCTAACAG TGGTAACACAGGCTTTG CACAGAAGTTCCGGGGC | AGCAGTGGCTGGTACTTTTT TGACTAC |
| | 21-225_32A5 | | SEQ ID NO:5087 | SEQ ID NO:13099 | SEQ ID NO:21111 |
| | | AA | NYDIN | WMHPNSGNTGFAQKFRG | SSGWYFFDY |
| | | | SEQ ID NO:5088 | SEQ ID NO:13100 | SEQ ID NO:21112 |
| iPS:398526 | | NA | AGCTATAGCATGAAC | TCCATTAGTGGTAGTAGT ACTTACATATACTACGCA GACTCAGTGAAGGGC | GTGGCTGGCTTTGACTAC |
| | 21-225_32B3 | | SEQ ID NO:5089 | SEQ ID NO:13101 | SEQ ID NO:21113 |
| | | AA | SYSMN | SISGSSTYIYYADSVKG | VAGFDY |
| | | | SEQ ID NO:5090 | SEQ ID NO:13102 | SEQ ID NO:21114 |
| iPS:398528 | | NA | AGCTATGCCATGAGC | GCTATTAGTGGTCGTGGT GGTAGCACATTCCACGC AGACTCCGTGAAGGGC | GGGGAGCTACTAGAGGACTA CTACTTCTACGGTATGGACG TC |
| | 21-225_32G1 | | SEQ ID NO:5091 | SEQ ID NO:13103 | SEQ ID NO:21115 |
| | | AA | SYAMS | AISGRGGSTFHADSVKG | GELLEDYYFYGMDV |
| | | | SEQ ID NO:5092 | SEQ ID NO:13104 | SEQ ID NO:21116 |
| iPS:398530 | | NA | AGTTATGATATCAAC | TGGATGAACCCTAACAG TGGTAACACAGGCTATG CACAGAAGTTCCAGGGC | AAGAAGGCTAACGACTAC |
| | 21-225_32G4 | | SEQ ID NO:5093 | SEQ ID NO:13105 | SEQ ID NO:21117 |
| | | AA | SYDIN | WMNPNSGNTGYAQKFQG | KKANDY |
| | | | SEQ ID NO:5094 | SEQ ID NO:13106 | SEQ ID NO:21118 |

FIGURE 49

| iPS:398532 | | NA | AGCTATAACATGAAC | TCCATTAGTGGTAGTAGT AGTTACATATACTACGC AGACTCAGTGAAGGGC | TTAAATGGTTTTTGACTAC |
|---|---|---|---|---|---|
| | 21-225_33B7 | | SEQ ID NO:5095 | SEQ ID NO:13107 | SEQ ID NO:21119 |
| | | AA | SYNMN | SISGSSSYIYYADSVKG | LNGFDY |
| | | | SEQ ID NO:5096 | SEQ ID NO:13108 | SEQ ID NO:21120 |
| iPS:398534 | | NA | AGCTATGCCATGAGC | GCTATTAGTGGTCGTGGT GGTAGCACATTCCACGC AGACTCCGTGAAGGGC | GGGGAGCTACTAGAGGACTA CTACTTCTACGGTATGGACG TC |
| | 21-225_33B8 | | SEQ ID NO:5097 | SEQ ID NO:13109 | SEQ ID NO:21121 |
| | | AA | SYAMS | AISGRGGSTFHADSVKG | GELLEDYYFYGMDV |
| | | | SEQ ID NO:5098 | SEQ ID NO:13110 | SEQ ID NO:21122 |
| iPS:398536 | | NA | AGTTATGATATCAGC | TGGATGAACCCTAACAG TGGTAACACAGGCTATG CACAGAAGTTCCAGGGC | AAGAGGGCTAACGACTAC |
| | 21-225_33D12 | | SEQ ID NO:5099 | SEQ ID NO:13111 | SEQ ID NO:21123 |
| | | AA | SYDIS | WMNPNSGNTGYAQKFQG | KRANDY |
| | | | SEQ ID NO:5100 | SEQ ID NO:13112 | SEQ ID NO:21124 |
| iPS:398538 | | NA | AACTATGATATTAAC | TGGATGAACCCTAACAG TGGTAACACAGGCTATG CACAGAAGTTCCAGGGC | AGCAGTGGCTGGTACTTTTT TGACTAC |
| | 21-225_34H7 | | SEQ ID NO:5101 | SEQ ID NO:13113 | SEQ ID NO:21125 |
| | | AA | NYDIN | WMNPNSGNTGYAQKFQG | SSGWYFFDY |
| | | | SEQ ID NO:5102 | SEQ ID NO:13114 | SEQ ID NO:21126 |
| iPS:398540 | | NA | AGCTATGCCATGAGC | ACTATTAGTGGTCGTGGT GGTAGCACATTCCACGC AGACTCCGTGAAGGGC | GGGGAGCTACTAGAGGACTA CTACTTCTACGGTATGGACG TC |
| | 21-225_35A6 | | SEQ ID NO:5103 | SEQ ID NO:13115 | SEQ ID NO:21127 |

FIGURE 49

| | | | | | |
|---|---|---|---|---|---|
| | | AA | SYAMS | TISGRGGSTFHADSVKG | GELLEDYYFYGMDV |
| | | | SEQ ID NO:5104 | SEQ ID NO:13116 | SEQ ID NO:21128 |
| iPS:398544 | 21-225_7C8 | NA | AACGCCCGGATGAAC | CGTATTAAAAGCAAAACTGATGGTGGGACAACAGACTACGCTGCACCCGTGAAAGGC | GATACGGGTCCTATAGCAGCTCGTCTCGCTTACTACTACTACTACGCTATGGACGTC |
| | | | SEQ ID NO:5105 | SEQ ID NO:13117 | SEQ ID NO:21129 |
| | | AA | NARMN | RIKSKTDGGTTDYAAPVKG | DTGPIAARLAYYYYYAMDV |
| | | | SEQ ID NO:5106 | SEQ ID NO:13118 | SEQ ID NO:21130 |
| iPS:398546 | 21-225_9H10 | NA | ACTAGTGGAGTGGGTGTGGGC | CTCATTTATTGGAGTGATGATAAGCGCTACAGCCCATCTCTGAAGAGC | ACCGGTTCTAGCTGCTGCTATTTTGACTAC |
| | | | SEQ ID NO:5107 | SEQ ID NO:13119 | SEQ ID NO:21131 |
| | | AA | TSGVGVG | LIYWSDDKRYSPSLKS | TGSSCCYFDY |
| | | | SEQ ID NO:5108 | SEQ ID NO:13120 | SEQ ID NO:21132 |
| iPS:402219 | 21-225_1C12 | NA | AACTATGGCATGCAC | GTTATATGGTATGATGAAAATAATAAATACTATGTAGACTCCGTGAAGGGC | GAATTGGGGTTCCGGTCTGACTAC |
| | | | SEQ ID NO:5109 | SEQ ID NO:13121 | SEQ ID NO:21133 |
| | | AA | NYGMH | VIWYDENNKYYVDSVKG | ELGFRSDY |
| | | | SEQ ID NO:5110 | SEQ ID NO:13122 | SEQ ID NO:21134 |
| iPS:402221 | 21-225_2C12 | NA | AGCTATAGCATGAAC | TCCATTAGTGGTAGTAGTAGTTACATGTACTACGCAGACTCAGTGAAGGGC | GTGAATCTCTTTGACTAC |
| | | | SEQ ID NO:5111 | SEQ ID NO:13123 | SEQ ID NO:21135 |
| | | AA | SYSMN | SISGSSSYMYYADSVKG | VNLFDY |
| | | | SEQ ID NO:5112 | SEQ ID NO:13124 | SEQ ID NO:21136 |

FIGURE 49

| iPS:402223 | | NA | GACTATCATATGCAC | TGGATCAACCCTAATAGGGGTGGCACAAACTATGCACAGAAGTTTCAGGAC | GATGGAACTGGGTCCTTTGACTAC |
|---|---|---|---|---|---|
| | 21-225_30A11 | | SEQ ID NO:5113 | SEQ ID NO:13125 | SEQ ID NO:21137 |
| | | AA | DYHMH | WINPNRGGTNYAQKFQD | DGTGSFDY |
| | | | SEQ ID NO:5114 | SEQ ID NO:13126 | SEQ ID NO:21138 |
| iPS:402225 | | NA | AGCTATAGCATGAAC | TCCATTAGTAGTAGTAGTAGTTACATATACTACGCAGACTCAGTGAAGGGC | CTGGGGAACTAC |
| | 21-225_2B1 | | SEQ ID NO:5115 | SEQ ID NO:13127 | SEQ ID NO:21139 |
| | | AA | SYSMN | SISSSSSYIYYADSVKG | LGNY |
| | | | SEQ ID NO:5116 | SEQ ID NO:13128 | SEQ ID NO:21140 |
| iPS:402229 | | NA | AGCTATAGCATGAAC | TCCATTAGTGGTAGTAGTAGTTACATATACTACGCAGACTCAGTGAAGGGC | GTCAACGGTATGGACGTC |
| | 21-225_16H9 | | SEQ ID NO:5117 | SEQ ID NO:13129 | SEQ ID NO:21141 |
| | | AA | SYSMN | SISGSSSYIYYADSVKG | VNGMDV |
| | | | SEQ ID NO:5118 | SEQ ID NO:13130 | SEQ ID NO:21142 |
| iPS:402231 | | NA | AACGCCTGGATGAAC | CGTATTAAAAGCAAAACTGATGGTGGGACAACAGACTACGCTGCACCCGTGAAAGGC | GATACGGGTCCTATAGCAGCTCGTCTCGCTTACTACTACTACTACGCTATGGACGTC |
| | 21-225_6D9 | | SEQ ID NO:5119 | SEQ ID NO:13131 | SEQ ID NO:21143 |
| | | AA | NAWMN | RIKSKTDGGTTDYAAPVKG | DTGPIAARLAYYYYYAMDV |
| | | | SEQ ID NO:5120 | SEQ ID NO:13132 | SEQ ID NO:21144 |
| iPS:402233 | 21 225 16D10 | NA | ACCTATAACTTGAAC | TCCATTAGTGGTGGTGCCGGTCACATATATTACTCAGACTCAGTGAAGGGC | ACTAATGGGTTTGACTTC |

FIGURE 49

| | | | | | |
|---|---|---|---|---|---|
| | 21-225_16D10 | | SEQ ID NO:5121 | SEQ ID NO:13133 | SEQ ID NO:21145 |
| | | AA | TYNLN | SISGGAGHIYYSDSVKG | TNGFDF |
| iPS:402235 | | | SEQ ID NO:5122 | SEQ ID NO:13134 | SEQ ID NO:21146 |
| | 21-225_20F10 | NA | AGCTATAGCATGAAC | TCCATTAGTACTAGTACTTTCATATACTACGCAGATTCAGTGAAGGGC | AAGGCTGGGCTTGATATC |
| | | | SEQ ID NO:5123 | SEQ ID NO:13135 | SEQ ID NO:21147 |
| | | AA | SYSMN | SISTSTFIYYADSVKG | KAGLDI |
| iPS:402237 | | | SEQ ID NO:5124 | SEQ ID NO:13136 | SEQ ID NO:21148 |
| | 21-225_23D11 | NA | AGCTATAACATAAAC | TCCATTAGTGGTAATAGTGGTTACATATACTACGCAGACTCAGTGAAGGGC | ACTAACCTCTTTGACTAC |
| | | | SEQ ID NO:5125 | SEQ ID NO:13137 | SEQ ID NO:21149 |
| | | AA | SYNIN | SISGNSGYIYYADSVKG | TNLFDY |
| iPS:403868 | | | SEQ ID NO:5126 | SEQ ID NO:13138 | SEQ ID NO:21150 |
| | 21-225_19D11 | NA | AGAAGTAGTTATTACTGGGGC | AGTATCTATTATAGTGGGAGCGCCAACTACAACCCGTCCCTCAAGAGT | CTGGACAGGGGCTGGTCCTTTGACTAC |
| | | | SEQ ID NO:5127 | SEQ ID NO:13139 | SEQ ID NO:21151 |
| | | AA | RSSYYWG | SIYYSGSANYNPSLKS | LDRGWSFDY |
| iPS:403870 | | | SEQ ID NO:5128 | SEQ ID NO:13140 | SEQ ID NO:21152 |
| | 21-225_23G4 | NA | AGCTATGCCATGAGC | GTTATTAGTGGGCGTGGTGGTAGCACATACTACGCAGACTCCGTGAAGGGC | AGGGGGATAGTGGGAGCTACTGAGGCTTTTGATATC |
| | | | SEQ ID NO:5129 | SEQ ID NO:13141 | SEQ ID NO:21153 |
| | | AA | SYAMS | VISGRGGSTYYADSVKG | RGIVGATEAFDI |
| iPS:403872 | | | SEQ ID NO:5130 | SEQ ID NO:13142 | SEQ ID NO:21154 |
| | 21 225 8F11 | NA | AGGACTAGTTACTACTGGGGC | AATATTTATTATAGTGGGAGCGCCTACAACAACCCGTCCCTCAAGAGT | CATGGACAAGACTGGGGCCTTGACTAC |

FIGURE 49

| | 21-225_8F11 | | SEQ ID NO:5131 | SEQ ID NO:13143 | SEQ ID NO:21155 |
|---|---|---|---|---|---|
| | | AA | RTSYYWG | NIYYSGSAYNNPSLKS | HGQDWGLDY |
| | | | SEQ ID NO:5132 | SEQ ID NO:13144 | SEQ ID NO:21156 |
| iPS:404090 | 21-225_8D8 | NA | AGCTATAGCATGAAC | TCCATTAGTAGTAGTAGT AGTTACATATACTACGC AGACTCAGTGAAGGGC | CTGGGTAACTAC |
| | | | SEQ ID NO:5133 | SEQ ID NO:13145 | SEQ ID NO:21157 |
| | | AA | SYSMN | SISSSSSYIYYADSVKG | LGNY |
| | | | SEQ ID NO:5134 | SEQ ID NO:13146 | SEQ ID NO:21158 |
| iPS:412232 | 21-225_4A2 | NA | AATTATGATATCAAC | TGGATGCACCCTAACAG TGGTAACACAGGCTATG CACAGAAGTTCCAGGGC | AGCAGTGGCTGGTACTACTT TGACTAC |
| | | | SEQ ID NO:5135 | SEQ ID NO:13147 | SEQ ID NO:21159 |
| | | AA | NYDIN | WMHPNSGNTGYAQKFQG | SSGWYYFDY |
| | | | SEQ ID NO:5136 | SEQ ID NO:13148 | SEQ ID NO:21160 |
| iPS:422894 | 21-225_4A2.001 | NA | AATTATGATATCAAC | TGGATGCACCCTAACAG TGGTAACACAGGCTATG CACAGAAGTTCCAGGGC | AGCAGTGGCTGGTACTACTT TGACTAC |
| | | | SEQ ID NO:5137 | SEQ ID NO:13149 | SEQ ID NO:21161 |
| | | AA | NYDIN | WMHPNSGNTGYAQKFQG | SSGWYYFDY |
| | | | SEQ ID NO:5138 | SEQ ID NO:13150 | SEQ ID NO:21162 |
| iPS:423018 | 21-225_31D12_LC2 | NA | GGCTACTATATGCAC | TGGATCAACCCTAACAG TGGTGGCACAAACTATG TACAGAAGTTTCAGGGC | GTGTATTACTATGGTTCGGG GAGTTATTATAACGAGTTTG ACTAC |
| | | | SEQ ID NO:5139 | SEQ ID NO:13151 | SEQ ID NO:21163 |
| | | AA | GYYMH | WINPNSGGTNYVQKFQG | VYYYGSGSYYNEFDY |
| | | | SEQ ID NO:5140 | SEQ ID NO:13152 | SEQ ID NO:21164 |

FIGURE 49

| iPS:423019 | | NA | GGCTACTATATGCAC | TGGATCAACCCTAACAG TGGTGGCACAAACTATG TACAGAAGTTTCAGGGC | GTGTATTACTATGGTTCGGG GAGTTATTATAACGAGTTTG ACTAC |
|---|---|---|---|---|---|
| | 21-225_31D12_LC1 | | SEQ ID NO:5141 | SEQ ID NO:13153 | SEQ ID NO:21165 |
| | | AA | GYYMH | WINPNSGGTNYVQKFQG | VYYYGSGSYYNEFDY |
| | | | SEQ ID NO:5142 | SEQ ID NO:13154 | SEQ ID NO:21166 |
| iPS:423314 | | NA | AATTATGATATCAAC | TGGATGCACCCTAACAG TGGTAACACAGGCTATG CAAAGAAGTTCCAGGGC | AGCAGTGGCTGGTACTACTT TGACTAC |
| | 21-225_12F11 | | SEQ ID NO:5143 | SEQ ID NO:13155 | SEQ ID NO:21167 |
| | | AA | NYDIN | WMHPNSGNTGYAKKFQG | SSGWYYFDY |
| | | | SEQ ID NO:5144 | SEQ ID NO:13156 | SEQ ID NO:21168 |
| iPS:424419 | | NA | AATTATGATATTAAT | TGGATGTACCCTAACAG TGGTAACACAGGCTATG CACAGAAATTCCAGGGC | AGCAGTGGCTGGTACTACTT TGACTAC |
| | 21-225_25A4.001 | | SEQ ID NO:5145 | SEQ ID NO:13157 | SEQ ID NO:21169 |
| | | AA | NYDIN | WMYPNSGNTGYAQKFQG | SSGWYYFDY |
| | | | SEQ ID NO:5146 | SEQ ID NO:13158 | SEQ ID NO:21170 |
| iPS:424460 | | NA | AGCTTTGGCATGCAC | ATTATCTGGCATGATGG AAGTAATAAATACTATG CAGACTCCGTGAAGGGC | GATCTGAGTATGGGCGGTAT GGACGTC |
| | 21-225_7E11.001 | | SEQ ID NO:5147 | SEQ ID NO:13159 | SEQ ID NO:21171 |
| | | AA | SFGMH | IIWHDGSNKYYADSVKG | DLSMGGMDV |
| | | | SEQ ID NO:5148 | SEQ ID NO:13160 | SEQ ID NO:21172 |

FIGURE 49

| iPS:426108 | | NA | GCCTACCATATGCAC | TGGATCAACCCTAACAA TAATGGCACAAACTATG CACAGAAGTTTCAGGGC | GATGTTACCAGCTCGTTTGA CTAT |
|---|---|---|---|---|---|
| | 21-225_10G6 | | SEQ ID NO:5149 | SEQ ID NO:13161 | SEQ ID NO:21173 |
| | | AA | AYHMH | WINPNNNGTNYAQKFQG | DVTSSFDY |
| | | | SEQ ID NO:5150 | SEQ ID NO:13162 | SEQ ID NO:21174 |
| iPS:426110 | | NA | GACTACTATTTGCAC | TGGGTCCACCCTAACAG TGGTGGCACAAACTTTG CACAGAAGTTTCAGGAC | GATGGTACCAGCTCGTTTGA CTAC |
| | 21-225_12E9 | | SEQ ID NO:5151 | SEQ ID NO:13163 | SEQ ID NO:21175 |
| | | AA | DYYLH | WVHPNSGGTNFAQKFQD | DGTSSFDY |
| | | | SEQ ID NO:5152 | SEQ ID NO:13164 | SEQ ID NO:21176 |
| iPS:426112 | | NA | AATTATGATATCAAC | TGGATGTACCCTAACAG TGGTAACACGGGCTATG CACAGAAGTTCCAGGGC | AGCAGTGGCTGGTACTACTT TGACTTC |
| | 21-225_12F12 | | SEQ ID NO:5153 | SEQ ID NO:13165 | SEQ ID NO:21177 |
| | | AA | NYDIN | WMYPNSGNTGYAQKFQG | SSGWYYFDF |
| | | | SEQ ID NO:5154 | SEQ ID NO:13166 | SEQ ID NO:21178 |
| iPS:426114 | | NA | AACTATGTCATGCAC | GTTATATGGTATGATGG AAGTAATAAGTACTATG CAGACTCCGTGAAGGGC | GAGGAGTATAGCAGTGGCTG GTACGACTACGGTATGGACG TC |
| | 21-225_28H2 | | SEQ ID NO:5155 | SEQ ID NO:13167 | SEQ ID NO:21179 |
| | | AA | NYVMH | VIWYDGSNKYYADSVKG | EEYSSGWYDYGMDV |
| | | | SEQ ID NO:5156 | SEQ ID NO:13168 | SEQ ID NO:21180 |

FIGURE 49

| iPS:426116 | | NA | AACTGTGTCATGCAC | GTTATATGGTATGATGG AAGTAATAAATACTATG CAGACTCCGTGAAGGGC | GAGGAGTATAGCAGTGGCTG GTACGACTACGGTATGGACG TC |
|---|---|---|---|---|---|
| | 21-225_29E2 | | SEQ ID NO:5157 | SEQ ID NO:13169 | SEQ ID NO:21181 |
| | | AA | NCVMH | VIWYDGSNKYYADSVKG | EEYSSGWYDYGMDV |
| | | | SEQ ID NO:5158 | SEQ ID NO:13170 | SEQ ID NO:21182 |
| iPS:426118 | | NA | AGCTATGGCATGCAC | GTTATATGGTATGATGG AAGTAATAAATACTATG CAGACTCCGTGAAGGGC | GATGAGAGGCTGGGGATTTT TGACTAC |
| | 21-225_7A10 | | SEQ ID NO:5159 | SEQ ID NO:13171 | SEQ ID NO:21183 |
| | | AA | SYGMH | VIWYDGSNKYYADSVKG | DERLGIFDY |
| | | | SEQ ID NO:5160 | SEQ ID NO:13172 | SEQ ID NO:21184 |
| iPS:426124 | | NA | AGCTATGGCATGCAC | GTTATATGGCATGATGG AAGTAATGCATACTATG CAGACTCCGTGAAGGGC | GAAAATAGCAGCTCGTACTA CTTTGACTAC |
| | 21-225_32D6 | | SEQ ID NO:5161 | SEQ ID NO:13173 | SEQ ID NO:21185 |
| | | AA | SYGMH | VIWHDGSNAYYADSVKG | ENSSSYYFDY |
| | | | SEQ ID NO:5162 | SEQ ID NO:13174 | SEQ ID NO:21186 |
| iPS:426126 | | NA | AATTATGATATCAAC | TGGATGCACCCTAACAG TGGTAACACAGGCTATG CAAAGAAGTTCCAGGGC | AGCAGTGGCTGGTACTACTT TGACTAC |
| | 21-225_6G6 | | SEQ ID NO:5163 | SEQ ID NO:13175 | SEQ ID NO:21187 |
| | | AA | NYDIN | WMHPNSGNTGYAKKFQG | SSGWYYFDY |

FIGURE 49

| | | | SEQ ID NO:5164 | SEQ ID NO:13176 | SEQ ID NO:21188 |
|---|---|---|---|---|---|
| iPS:433895 | 21-225_43E1 | NA | AGCTATAGCATGAAC | GCCATTAGTGGTAATAG TACTTACATATACTACGC AGACTCGTTGAAGGGC | GATCGGGGCAGTGAA |
| | | | SEQ ID NO:5165 | SEQ ID NO:13177 | SEQ ID NO:21189 |
| | | AA | SYSMN | AISGNSTYIYYADSLKG | DRGSE |
| | | | SEQ ID NO:5166 | SEQ ID NO:13178 | SEQ ID NO:21190 |
| iPS:433897 | 21-225_43C2 | NA | AGCTATGCCATGAGC | GCTATTAGTGGTCGTGGT GTTAACACATTCGACGC AGACTCCGTGAAGGGC | GAAAGGAGTGGGAGCTATTT TGACTAC |
| | | | SEQ ID NO:5167 | SEQ ID NO:13179 | SEQ ID NO:21191 |
| | | AA | SYAMS | AISGRGVNTFDADSVKG | ERSGSYFDY |
| | | | SEQ ID NO:5168 | SEQ ID NO:13180 | SEQ ID NO:21192 |
| iPS:433899 | 21-225_43C3 | NA | AGCTATGGCATACAC | GTTATATGGTATGATGA AAATAATAAATACTATG CAGACTCCGTGAAGGGC | GAGCTAGGATTTTCCAATGA CTAC |
| | | | SEQ ID NO:5169 | SEQ ID NO:13181 | SEQ ID NO:21193 |
| | | AA | SYGIH | VIWYDENNKYYADSVKG | ELGFSNDY |
| | | | SEQ ID NO:5170 | SEQ ID NO:13182 | SEQ ID NO:21194 |
| iPS:433901 | 21-225_43A4 | NA | AGCTATACCATGAAC | TCCATTAGTGGAAGTAG TACTTACATATACTACGC AGACTCAGTGAAGGGC | GTGACCTCTTTTGACTAC |
| | | | SEQ ID NO:5171 | SEQ ID NO:13183 | SEQ ID NO:21195 |
| | | AA | SYTMN | SISGSSTYIYYADSVKG | VTSFDY |
| | | | SEQ ID NO:5172 | SEQ ID NO:13184 | SEQ ID NO:21196 |
| iPS:433903 | 21 225 43H4 | NA | AGCTATGCCATGAGC | GCTATTAGTGGTCGTGGT ATTAACACATTCGACGC AGACTCCGTGAAGGGC | GAAAGGAGTGGGAGCTATTT TGACTAC |

FIGURE 49

| | | | | | |
|---|---|---|---|---|---|
| | 21-225_43H4 | | SEQ ID NO:5173 | SEQ ID NO:13185 | SEQ ID NO:21197 |
| | | AA | SYAMS | AISGRGINTFDADSVKG | ERSGSYFDY |
| | | | SEQ ID NO:5174 | SEQ ID NO:13186 | SEQ ID NO:21198 |
| iPS:433905 | 21-225_43E5 | NA | GACTACTACATGATC | TACATTAGTAGTAGTGGT ATTACCAAATACTACGC AGACTCTATGAAGGGC | GATACAATCTAC |
| | | | SEQ ID NO:5175 | SEQ ID NO:13187 | SEQ ID NO:21199 |
| | | AA | DYYMI | YISSSGITKYYADSMKG | DTIY |
| | | | SEQ ID NO:5176 | SEQ ID NO:13188 | SEQ ID NO:21200 |
| iPS:433909 | 21-225_43D8 | NA | AATTATGATATCAAC | TGGATGCACCCTAACAG TGGTAACACAGGCTATG CACAGAAGTTCCAGGGC | AGCAGTGGCTGGACCCTCTT TGACTAC |
| | | | SEQ ID NO:5177 | SEQ ID NO:13189 | SEQ ID NO:21201 |
| | | AA | NYDIN | WMHPNSGNTGYAQKFQG | SSGWTLFDY |
| | | | SEQ ID NO:5178 | SEQ ID NO:13190 | SEQ ID NO:21202 |
| iPS:433911 | 21-225_43E8 | NA | AGCTATGCCATGAGC | GCTATTAGTGGTCGTGGT ATTAACACATTCGACGC AGACTCCGTGAAGGGC | GAAAGGAGTGGGAGCTATTT TGACTAC |
| | | | SEQ ID NO:5179 | SEQ ID NO:13191 | SEQ ID NO:21203 |
| | | AA | SYAMS | AISGRGINTFDADSVKG | ERSGSYFDY |
| | | | SEQ ID NO:5180 | SEQ ID NO:13192 | SEQ ID NO:21204 |
| iPS:433913 | 21-225_43H8 | NA | GACTACTACATGAAC | TACATTAGTAGTAGTGGT AGAACCATATTCTACGC AGACTCTTTGAAGGGC | GATACAATCTAC |
| | | | SEQ ID NO:5181 | SEQ ID NO:13193 | SEQ ID NO:21205 |
| | | AA | DYYMN | YISSSGRTIFYADSLKG | DTIY |
| | | | SEQ ID NO:5182 | SEQ ID NO:13194 | SEQ ID NO:21206 |

FIGURE 49

| iPS:433915 | | NA | AGCTATGCCATGAGT | GCTATTAGTGGTAGTGGT AGTAACACATTCTACGC AGACTCCGTGAAGGGC | CGAACGCCCTCTGATGTTTTT GATATC |
|---|---|---|---|---|---|
| | 21-225_43H9 | | SEQ ID NO:5183 | SEQ ID NO:13195 | SEQ ID NO:21207 |
| | | AA | SYAMS | AISGSGSNTFYADSVKG | RTPSDVFDI |
| | | | SEQ ID NO:5184 | SEQ ID NO:13196 | SEQ ID NO:21208 |
| iPS:433917 | | NA | GACTATGGCATGCAC | GTTATATGGTATGATGG AAGTAATAAATACTATG CAGACTCCGTGAAGGGC | CGGTATGTCAGAAGCTGGGT GGGAGGTATGGACGTC |
| | 21-225_43E11 | | SEQ ID NO:5185 | SEQ ID NO:13197 | SEQ ID NO:21209 |
| | | AA | DYGMH | VIWYDGSNKYYADSVKG | RYVRSWVGGMDV |
| | | | SEQ ID NO:5186 | SEQ ID NO:13198 | SEQ ID NO:21210 |
| iPS:433919 | | NA | GACTATGGCATGCAC | GTTATATGGTATGATGG AAGTAATAAATACTATG CAGACTCCGTGAAGGGC | GAGAGGTATAGCAGTGGCTG GTACGACTACGGTATGGACG TC |
| | 21-225_44B3 | | SEQ ID NO:5187 | SEQ ID NO:13199 | SEQ ID NO:21211 |
| | | AA | DYGMH | VIWYDGSNKYYADSVKG | ERYSSGWYDYGMDV |
| | | | SEQ ID NO:5188 | SEQ ID NO:13200 | SEQ ID NO:21212 |
| iPS:433921 | | NA | AGCTATGGCATGCAC | GTTATATGGTTTGAAGG AAGTAATAAATACTATG CAGATTCCGTGAAGGGC | GAACTAGGATTTTCCACCGA CTAC |
| | 21-225_44C3 | | SEQ ID NO:5189 | SEQ ID NO:13201 | SEQ ID NO:21213 |
| | | AA | SYGMH | VIWFEGSNKYYADSVKG | ELGFSTDY |
| | | | SEQ ID NO:5190 | SEQ ID NO:13202 | SEQ ID NO:21214 |

FIGURE 49

| iPS:433923 | | NA | AGCTATGTCATGCAC | GTTATATGGTATGATGG AAGTAATAAATACTATG CTGACTCCGTGAAGGGC | GAAAGGTATAGCAGTGGCTT GTACGACTACGGTATGGACG TC |
|---|---|---|---|---|---|
| | 21-225_44D3 | | SEQ ID NO:5191 | SEQ ID NO:13203 | SEQ ID NO:21215 |
| | | AA | SYVMH | VIWYDGSNKYYADSVKG | ERYSSGLYDYGMDV |
| | | | SEQ ID NO:5192 | SEQ ID NO:13204 | SEQ ID NO:21216 |
| iPS:433925 | | NA | AGCTATGCCATGAGC | ATTCTCAGTGGTGGTGGT AAGACCACATACTACGC AGACTCCGTGAAGGGC | CGAACGCCCTCTGATGCTTT TGATATC |
| | 21-225_44F3 | | SEQ ID NO:5193 | SEQ ID NO:13205 | SEQ ID NO:21217 |
| | | AA | SYAMS | ILSGGGKTTYYADSVKG | RTPSDAFDI |
| | | | SEQ ID NO:5194 | SEQ ID NO:13206 | SEQ ID NO:21218 |
| iPS:433929 | | NA | AGCTATGTCATGCAC | GTTATATGGTATGATGG AAGTAATAAATACTATG CAGACTCCGTGAAGGGC | GTCCCGTATAGCAGCAGCTG GTACGACTACGGTATGGACG TC |
| | 21-225_44D5 | | SEQ ID NO:5195 | SEQ ID NO:13207 | SEQ ID NO:21219 |
| | | AA | SYVMH | VIWYDGSNKYYADSVKG | VPYSSSWYDYGMDV |
| | | | SEQ ID NO:5196 | SEQ ID NO:13208 | SEQ ID NO:21220 |
| iPS:433931 | | NA | AGTTACTACTGGAGC | TATATCTATTACAGTGGA AACACCAACTACAACCC CTCCCTCAAGAGT | GGGGTGGCTATAAAGAACTA C |
| | 21-225_44F6 | | SEQ ID NO:5197 | SEQ ID NO:13209 | SEQ ID NO:21221 |
| | | AA | SYYWS | YIYYSGNTNYNPSLKS | GVAIKNY |
| | | | SEQ ID NO:5198 | SEQ ID NO:13210 | SEQ ID NO:21222 |

FIGURE 49

| iPS:433933 | | NA | AACTATGGCATGCAC | GTTATATGGTATGAAGG AAGTAATAAATACTATG CAGACTCCGTGAAGGGC | GAACTGGGGTTCCTCTCTGA CTAC |
|---|---|---|---|---|---|
| | 21-225_44C8 | | SEQ ID NO:5199 | SEQ ID NO:13211 | SEQ ID NO:21223 |
| | | AA | NYGMH | VIWYEGSNKYYADSVKG | ELGFLSDY |
| | | | SEQ ID NO:5200 | SEQ ID NO:13212 | SEQ ID NO:21224 |
| iPS:433935 | | NA | AGCTATGTCATGCAC | GTTATATGGTATGATGG AAGTAATAAATACTATG CAGACTCCGTGAAGGGC | GAACCATATAGCAGCAGCTG GTACGACTACGGTATGGACG TC |
| | 21-225_44F9 | | SEQ ID NO:5201 | SEQ ID NO:13213 | SEQ ID NO:21225 |
| | | AA | SYVMH | VIWYDGSNKYYADSVKG | EPYSSSWYDYGMDV |
| | | | SEQ ID NO:5202 | SEQ ID NO:13214 | SEQ ID NO:21226 |
| iPS:433937 | | NA | AGCTATGGCATGCAC | GTTATATGGTATGATGG AAGTAATAAATACTATG CAGACTCCGTGAAGGGC | CGGTATAGCAGCAGCTGGGT GGGGGGTATGGACGTC |
| | 21-225_44B10 | | SEQ ID NO:5203 | SEQ ID NO:13215 | SEQ ID NO:21227 |
| | | AA | SYGMH | VIWYDGSNKYYADSVKG | RYSSSWVGGMDV |
| | | | SEQ ID NO:5204 | SEQ ID NO:13216 | SEQ ID NO:21228 |
| iPS:433939 | | NA | GACTGTGTCATGCAC | GTTATATGGTATGATGG AAGTAATAAATACTATG CTGACTCCGTGAAGGGC | GAAAGGTATAGCAGTGGCTT GTACGACTACGGTATGGACG TC |
| | 21-225_44C10 | | SEQ ID NO:5205 | SEQ ID NO:13217 | SEQ ID NO:21229 |

FIGURE 49

| | | AA | DCVMH | VIWYDGSNKYYADSVKG | ERYSSGLYDYGMDV |
|---|---|---|---|---|---|
| | | | SEQ ID NO:5206 | SEQ ID NO:13218 | SEQ ID NO:21230 |
| iPS:433941 | 21-225_44D10 | NA | AGCTATGCCATGAGC | GCTATTAGTGGTCGTGGT GTTAACACATTCGACGC AGACTCCGTGAAGGGC | GAAAGGAGTGGGAGCTATTT TGACTAC |
| | | | SEQ ID NO:5207 | SEQ ID NO:13219 | SEQ ID NO:21231 |
| | | AA | SYAMS | AISGRGVNTFDADSVKG | ERSGSYFDY |
| | | | SEQ ID NO:5208 | SEQ ID NO:13220 | SEQ ID NO:21232 |
| iPS:433943 | 21-225_44E10 | NA | AGCTATGCCATGAAC | GGTGTTGTTGGTAGTGGT GGTAGAACATACTACGC AGACTCCGTGAAGGGC | GATCGGGGGCAGTGGCTCCT AGGCGGTATGGACGTC |
| | | | SEQ ID NO:5209 | SEQ ID NO:13221 | SEQ ID NO:21233 |
| | | AA | SYAMN | GVVGSGGRTYYADSVKG | DRGQWLLGGMDV |
| | | | SEQ ID NO:5210 | SEQ ID NO:13222 | SEQ ID NO:21234 |
| iPS:433945 | 21-225_44C12 | NA | AGCTATAGCGTGAAC | TACATTAGTAGTAGTAGT AGTACCATATACTACGC AGACTCTGTGAAGGGC | AGTGGATACAGCTATGCTTA CTACTACTACTACGGTATGG ACGTC |
| | | | SEQ ID NO:5211 | SEQ ID NO:13223 | SEQ ID NO:21235 |
| | | AA | SYSVN | YISSSSSTIYYADSVKG | SGYSYAYYYYYGMDV |
| | | | SEQ ID NO:5212 | SEQ ID NO:13224 | SEQ ID NO:21236 |
| iPS:433947 | 21-225_44E12 | NA | AGCGATGACACGCAC | GTTATATGGTTTGATGAA TATAATAAATACTATGC AGACTCCGTGAAGGGC | GATCTAATAGCAGCAGCTGG GACGGGAGACTAC |
| | | | SEQ ID NO:5213 | SEQ ID NO:13225 | SEQ ID NO:21237 |
| | | AA | SDDTH | VIWFDEYNKYYADSVKG | DLIAAAGTGDY |
| | | | SEQ ID NO:5214 | SEQ ID NO:13226 | SEQ ID NO:21238 |

FIGURE 49

| iPS:433949 | | NA | GACTACTACATGAAC | TACATTAGTAGTAGTGGT ATTACCAAATACTACGC AGACTCTGTGAAGGGC | GATACAATCTAC |
|---|---|---|---|---|---|
| | 21-225_45H2 | | SEQ ID NO:5215 | SEQ ID NO:13227 | SEQ ID NO:21239 |
| | | AA | DYYMN | YISSSGITKYYADSVKG | DTIY |
| | | | SEQ ID NO:5216 | SEQ ID NO:13228 | SEQ ID NO:21240 |
| iPS:433951 | | NA | GACTGTGTCATGCAC | GTTATATGGTATGATGG AAGTAATAAATACTATG CTGACTCCGTGAAGGGC | GAAAGGTATAGCAGTGGCTT GTACGACTACGGTATGGACG TC |
| | 21-225_45B4 | | SEQ ID NO:5217 | SEQ ID NO:13229 | SEQ ID NO:21241 |
| | | AA | DCVMH | VIWYDGSNKYYADSVKG | ERYSSGLYDYGMDV |
| | | | SEQ ID NO:5218 | SEQ ID NO:13230 | SEQ ID NO:21242 |
| iPS:433953 | | NA | AGCTATGCCATGAGT | GCTATTAGTGGTAGTGGT AGTAACACATTCTACGC AGACTCCGTGAAGGGC | CGAACGCCCTCTGATGTTTTT GATATC |
| | 21-225_45H4 | | SEQ ID NO:5219 | SEQ ID NO:13231 | SEQ ID NO:21243 |
| | | AA | SYAMS | AISGSGSNTFYADSVKG | RTPSDVFDI |
| | | | SEQ ID NO:5220 | SEQ ID NO:13232 | SEQ ID NO:21244 |
| iPS:433955 | | NA | GACTGTGTCATGCAC | GTTATATGGTATGATGG AAGTAATAAATACTATG CTGACTCCGTGAAGGGC | GAAAGGTATAGCAGTGGCTT GTACGACTACGGTATGGACG TC |
| | 21-225_45B8 | | SEQ ID NO:5221 | SEQ ID NO:13233 | SEQ ID NO:21245 |
| | | AA | DCVMH | VIWYDGSNKYYADSVKG | ERYSSGLYDYGMDV |
| | | | SEQ ID NO:5222 | SEQ ID NO:13234 | SEQ ID NO:21246 |

FIGURE 49

| iPS:433957 | | NA | AGCTATGCCATGAGC | GCTATTAGTGGTCGTGGT GTTAACACATTCGACGC AGACTCCGTGAAGGGC | GAAAGGAGTGGGAGCTATTT TGACTAC |
|---|---|---|---|---|---|
| | 21-225_45F8 | | SEQ ID NO:5223 | SEQ ID NO:13235 | SEQ ID NO:21247 |
| | | AA | SYAMS | AISGRGVNTFDADSVKG | ERSGSYFDY |
| | | | SEQ ID NO:5224 | SEQ ID NO:13236 | SEQ ID NO:21248 |
| iPS:433959 | | NA | AGCTATGCCATGAGC | GTTATTAGTGGTCGTGGT GGTACCACATACTACGC AGACTCCGTGAAGGGC | CGAACGCCCTCTGATGCTTT TGATATC |
| | 21-225_45C9 | | SEQ ID NO:5225 | SEQ ID NO:13237 | SEQ ID NO:21249 |
| | | AA | SYAMS | VISGRGGTTYYADSVKG | RTPSDAFDI |
| | | | SEQ ID NO:5226 | SEQ ID NO:13238 | SEQ ID NO:21250 |
| iPS:433961 | | NA | AGCTATACCATGAAC | TCCATTAGTGGAAGTAG TACTTACATATACTACGC AGACTCAGTGAAGGGC | GTGACCTCTTTTGACTAC |
| | 21-225_45D9 | | SEQ ID NO:5227 | SEQ ID NO:13239 | SEQ ID NO:21251 |
| | | AA | SYTMN | SISGSSTYIYYADSVKG | VTSFDY |
| | | | SEQ ID NO:5228 | SEQ ID NO:13240 | SEQ ID NO:21252 |
| iPS:433963 | | NA | AGCGATGACTCGCAC | GTTATATGGTTTGATGAA TATACTAAATACTATGCA GACTCCGTGAAGGGC | GATCTAATAGCAGCAACTGG GACGGGAGACTAC |
| | 21-225_46B1 | | SEQ ID NO:5229 | SEQ ID NO:13241 | SEQ ID NO:21253 |
| | | AA | SDDSH | VIWFDEYTKYYADSVKG | DLIAATGTGDY |
| | | | SEQ ID NO:5230 | SEQ ID NO:13242 | SEQ ID NO:21254 |
| iPS:433965 | | NA | AGCTATGGCATGCAC | ATTATATGGTATGATGG AAGTAATAAATACTATG TAGACTCCGTGAAGGGC | GATCGATACGATTTTTGGAG TGGTTACTTTGACTAC |
| | 21-225_46F2 | | SEQ ID NO:5231 | SEQ ID NO:13243 | SEQ ID NO:21255 |
| | | AA | SYGMH | IIWYDGSNKYYVDSVKG | DRYDFWSGYFDY |

FIGURE 49

| | | | SEQ ID NO:5232 | SEQ ID NO:13244 | SEQ ID NO:21256 |
|---|---|---|---|---|---|
| iPS:433967 | 21-225_46C3 | NA | AGCTATGTCATGCAC | GTTATATGGTATGATGG AAGTAATAAATACTATG CTGACTCCGTGAAGGGC | GAAAGGTATAGCAGTGGCTT GTACGACTACGGTATGGACG TC |
| | | | SEQ ID NO:5233 | SEQ ID NO:13245 | SEQ ID NO:21257 |
| | | AA | SYVMH | VIWYDGSNKYYADSVKG | ERYSSGLYDYGMDV |
| | | | SEQ ID NO:5234 | SEQ ID NO:13246 | SEQ ID NO:21258 |
| iPS:433969 | 21-225_46F3 | NA | GATTATGGCATGCAC | GTTATATGGTTTGAAGG AAGTAATAAATACTATG CAGATTCCGTGAAGGGC | GAACTAGGATTTTCCAATGA CTAC |
| | | | SEQ ID NO:5235 | SEQ ID NO:13247 | SEQ ID NO:21259 |
| | | AA | DYGMH | VIWFEGSNKYYADSVKG | ELGFSNDY |
| | | | SEQ ID NO:5236 | SEQ ID NO:13248 | SEQ ID NO:21260 |
| iPS:433971 | 21-225_46D4 | NA | AGCTATGTCATGCAC | GTTATATGGTATGATGG AAGTAATAAATACTATG CAGACTCCGTGAAGGGC | GTCCCGTATAGCAGCAGTTG GTACGACTACGGTATGGACG TC |
| | | | SEQ ID NO:5237 | SEQ ID NO:13249 | SEQ ID NO:21261 |
| | | AA | SYVMH | VIWYDGSNKYYADSVKG | VPYSSSWYDYGMDV |
| | | | SEQ ID NO:5238 | SEQ ID NO:13250 | SEQ ID NO:21262 |
| iPS:433973 | 21-225_46A6 | NA | AGCTATGCCATGAGC | GCTATTAGTGGTCGTGGT ATTAACACATTCGACGC AGACTCCGTGAAGGGC | GAAAGGAGTGGGAGCTATTT TGACTAC |
| | | | SEQ ID NO:5239 | SEQ ID NO:13251 | SEQ ID NO:21263 |
| | | AA | SYAMS | AISGRGINTFDADSVKG | ERSGSYFDY |
| | | | SEQ ID NO:5240 | SEQ ID NO:13252 | SEQ ID NO:21264 |

EP 4 435 105 A2

FIGURE 49

| iPS:433975 | | NA | AGCTATGGCATACAC | GTTATATGGTATGATGA AAATAATAAATACTATG CAGACTCCGTGAAGGGC | GAGCTAGGATTTTCCAATGA CTAC |
|---|---|---|---|---|---|
| | 21-225_46C6 | | SEQ ID NO:5241 | SEQ ID NO:13253 | SEQ ID NO:21265 |
| | | AA | SYGIH | VIWYDENNKYYADSVKG | ELGFSNDY |
| | | | SEQ ID NO:5242 | SEQ ID NO:13254 | SEQ ID NO:21266 |
| iPS:433977 | | NA | GATTATGGCATACAC | GTTATATGGTTTGAAGG AAGTAATAAATACTATG CAGATTCCGTGAAGGGC | GAACTAGGATTTTCCAATGA CTAC |
| | 21-225_46D8 | | SEQ ID NO:5243 | SEQ ID NO:13255 | SEQ ID NO:21267 |
| | | AA | DYGIH | VIWFEGSNKYYADSVKG | ELGFSNDY |
| | | | SEQ ID NO:5244 | SEQ ID NO:13256 | SEQ ID NO:21268 |
| iPS:433979 | | NA | AGCTATGGCATGCAC | GTTATATGGTATGATGG AAGAAATAAATACTATG CAGACTCCGTGAAGGGC | CGGTATAGCAGCAGCTGGAT GGGAGGTATGGACGTC |
| | 21-225_46B9 | | SEQ ID NO:5245 | SEQ ID NO:13257 | SEQ ID NO:21269 |
| | | AA | SYGMH | VIWYDGRNKYYADSVKG | RYSSSWMGGMDV |
| | | | SEQ ID NO:5246 | SEQ ID NO:13258 | SEQ ID NO:21270 |
| iPS:433981 | | NA | GACTACTACATGAAC | TACATTAATAGTAATGGT TTTACCATATACTACGCA GACTCTGTGAAGGGC | GATACAATCTAC |
| | 21-225_46E9 | | SEQ ID NO:5247 | SEQ ID NO:13259 | SEQ ID NO:21271 |
| | | AA | DYYMN | YINSNGFTIYYADSVKG | DTIY |
| | | | SEQ ID NO:5248 | SEQ ID NO:13260 | SEQ ID NO:21272 |

FIGURE 49

| iPS:433983 | 21-225_47A1 | NA | GACTATGGCATGCAC | GTTATATGGTATGATGACTATAATAAAAAGTATGCAGACTCCGTGAAGGGC | GAACTGGGGATGCTCTTTGACTAC |
|---|---|---|---|---|---|
| | | | SEQ ID NO:5249 | SEQ ID NO:13261 | SEQ ID NO:21273 |
| | | AA | DYGMH | VIWYDDYNKKYADSVKG | ELGMLFDY |
| | | | SEQ ID NO:5250 | SEQ ID NO:13262 | SEQ ID NO:21274 |
| iPS:433985 | 21-225_47C1 | NA | GACTATGGCATGCAC | GTTATATGGTATGATGGAAGTAATAAATACTATGCAGACTCCGTGAAGGGC | CGGTATAGCCGCAGCTGGGTGGGAGGTATGGACGTC |
| | | | SEQ ID NO:5251 | SEQ ID NO:13263 | SEQ ID NO:21275 |
| | | AA | DYGMH | VIWYDGSNKYYADSVKG | RYSRSWVGGMDV |
| | | | SEQ ID NO:5252 | SEQ ID NO:13264 | SEQ ID NO:21276 |
| iPS:433987 | 21-225_47A5 | NA | GACGATGACACACAC | GTTATATGGTTTGATGGAAGTAATAAATACTATGCAGACTCCGTGAAGGGC | GATCTTATAGCAGCAGCTGGTACAGTTGACTAC |
| | | | SEQ ID NO:5253 | SEQ ID NO:13265 | SEQ ID NO:21277 |
| | | AA | DDDTH | VIWFDGSNKYYADSVKG | DLIAAAGTVDY |
| | | | SEQ ID NO:5254 | SEQ ID NO:13266 | SEQ ID NO:21278 |
| iPS:433989 | 21-225_47C7 | NA | AACTATGCCATGAGC | GGTATTAGTGGTAGTGGTAGTCGCACATACTACGCAGACTCCGTGAAGGGC | GATCGGGGGCAGTGGCTCATAGGCGGTATGGACGTC |
| | | | SEQ ID NO:5255 | SEQ ID NO:13267 | SEQ ID NO:21279 |
| | | AA | NYAMS | GISGSGSRTYYADSVKG | DRGQWLIGGMDV |
| | | | SEQ ID NO:5256 | SEQ ID NO:13268 | SEQ ID NO:21280 |

FIGURE 49

| iPS:433991 | | NA | ATCTATGGCATGCAC | GTTATATGGTATGATGG AAGTAATAAATACTATG CAGACTCCGTGAAGGGC | CGGTATAGCAGAAGCTGGGT GGGAGGTATGGACGTC |
|---|---|---|---|---|---|
| | 21-225_47E7 | | SEQ ID NO:5257 | SEQ ID NO:13269 | SEQ ID NO:21281 |
| | | AA | IYGMH | VIWYDGSNKYYADSVKG | RYSRSWVGGMDV |
| | | | SEQ ID NO:5258 | SEQ ID NO:13270 | SEQ ID NO:21282 |
| iPS:433993 | | NA | AGCTATGCCATGAGT | GCTATTAGTGGTCGTGGT GGTAACACATTCTACGC AGAGTCCGTGAGGGGC | ATTATCCGGGAGCAGTGGGC CTTTGACTAC |
| | 21-225_47G7 | | SEQ ID NO:5259 | SEQ ID NO:13271 | SEQ ID NO:21283 |
| | | AA | SYAMS | AISGRGGNTFYAESVRG | IIREQWAFDY |
| | | | SEQ ID NO:5260 | SEQ ID NO:13272 | SEQ ID NO:21284 |
| iPS:433995 | | NA | GACTACTACATGATC | TACATTAATAGTAATGGT TTTACCAAATACTACGCA GACTCTGTGAAGGGC | GATACAGTCTAC |
| | 21-225_47H7 | | SEQ ID NO:5261 | SEQ ID NO:13273 | SEQ ID NO:21285 |
| | | AA | DYYMI | YINSNGFTKYYADSVKG | DTVY |
| | | | SEQ ID NO:5262 | SEQ ID NO:13274 | SEQ ID NO:21286 |
| iPS:433997 | | NA | AGCTATGGCATGCAC | GTTGTATGGTATGATGA AATTAATAAAAGTATG CAGACTCCGTGAAGGGC | GAATTAGGGTGGGAGGCTGA CTAC |
| | 21-225_48C1 | | SEQ ID NO:5263 | SEQ ID NO:13275 | SEQ ID NO:21287 |
| | | AA | SYGMH | VVWYDEINKKYADSVKG | ELGWEADY |
| | | | SEQ ID NO:5264 | SEQ ID NO:13276 | SEQ ID NO:21288 |

FIGURE 49

| iPS:433999 | | NA | AGTTATGCCATGAGC | GTTATTAGTGGTCGTGGT GGTAGCACATTCTACGC AGACTCCGTGAAGGGC | CGTATAGCAGTGGCTGGGAA TGAGGCTTTTGATATC |
|---|---|---|---|---|---|
| | 21-225_48D1 | | SEQ ID NO:5265 | SEQ ID NO:13277 | SEQ ID NO:21289 |
| | | AA | SYAMS | VISGRGGSTFYADSVKG | RIAVAGNEAFDI |
| | | | SEQ ID NO:5266 | SEQ ID NO:13278 | SEQ ID NO:21290 |
| iPS:434001 | | NA | AACTATGTCATGCAC | GTTATATGGTATGATGG AAGTAATAAATACTATG CAGACTCCGTGAAGGGC | GAGAGGTATAGCAGCAGCTG GTACGACTACGGTCTGGACG TC |
| | 21-225_48F2 | | SEQ ID NO:5267 | SEQ ID NO:13279 | SEQ ID NO:21291 |
| | | AA | NYVMH | VIWYDGSNKYYADSVKG | ERYSSSWYDYGLDV |
| | | | SEQ ID NO:5268 | SEQ ID NO:13280 | SEQ ID NO:21292 |
| iPS:434003 | | NA | AGTTATGCCATGAGC | GTTATTAGTGGTCGTGGT GGTAGCACATTCTACGC AGACTCCGTGAAGGGC | CGTATAGCAGTGGCTGGGAA TGAGGCTTTTGATATC |
| | 21-225_48C3 | | SEQ ID NO:5269 | SEQ ID NO:13281 | SEQ ID NO:21293 |
| | | AA | SYAMS | VISGRGGSTFYADSVKG | RIAVAGNEAFDI |
| | | | SEQ ID NO:5270 | SEQ ID NO:13282 | SEQ ID NO:21294 |
| iPS:434007 | | NA | AACTCTGCCATGAAC | GCTATTAGTGGTAGTGGT GGTACCACATTCTACGC AGACTCCGTGAAGGGC | TGTGGGCGGGAGCAGTGGCT TGACTAC |
| | 21-225_48D7 | | SEQ ID NO:5271 | SEQ ID NO:13283 | SEQ ID NO:21295 |
| | | AA | NSAMN | AISGSGGTTFYADSVKG | CGREQWLDY |
| | | | SEQ ID NO:5272 | SEQ ID NO:13284 | SEQ ID NO:21296 |
| iPS:434009 | | NA | AGCTATGGCATGCAC | GTTATATGGTATGAGGA AAATAAGAAATACTATG CAGACTCCGTGAAGGGC | GAACTTGCCTGGTACGAGGA CTAC |
| | 21 225 48A9 | | | | |

FIGURE 49

| | | | | | |
|---|---|---|---|---|---|
| | 21-225_48A9 | | | SEQ ID NO:5273 | SEQ ID NO:13285 | SEQ ID NO:21297 |
| | | AA | SYGMH | VIWYEENKKYYADSVKG | ELAWYEDY |
| iPS:434011 | 21-225_48B10 | NA | SEQ ID NO:5274 GACTACTTCATGACC | SEQ ID NO:13286 TACATTAGTAGTGCTGGT GGTGCCATATACTACGC AGACTCTGTGAAGGGC | SEQ ID NO:21298 GCAGTGGCTGCCCCTGGTGT TTTTGATATC |
| | | | SEQ ID NO:5275 | SEQ ID NO:13287 | SEQ ID NO:21299 |
| | | AA | DYFMT | YISSAGGAIYYADSVKG | AVAAPGVFDI |
| | | | SEQ ID NO:5276 | SEQ ID NO:13288 | SEQ ID NO:21300 |
| iPS:434013 | 21-225_48D12 | NA | GACTATGGCATGCAC | GTTATATGGTATGATGTA AGTAATAAATACTATGT AGACTCCGTGAAGGGC | GAACTGGGGATGAGATCTGA CTAC |
| | | | SEQ ID NO:5277 | SEQ ID NO:13289 | SEQ ID NO:21301 |
| | | AA | DYGMH | VIWYDVSNKYYVDSVKG | ELGMRSDY |
| | | | SEQ ID NO:5278 | SEQ ID NO:13290 | SEQ ID NO:21302 |
| iPS:434015 | 21-225_48F12 | NA | GACTACTTCATGACC | TACATTAGTAGTGCTGGT GGTGCCATATACTACGC AGACTCTGTGAAGGGC | GCAGTGGCTGCCCCTGGTGC TTTTGATATC |
| | | | SEQ ID NO:5279 | SEQ ID NO:13291 | SEQ ID NO:21303 |
| | | AA | DYFMT | YISSAGGAIYYADSVKG | AVAAPGAFDI |
| | | | SEQ ID NO:5280 | SEQ ID NO:13292 | SEQ ID NO:21304 |
| iPS:434017 | 21-225_48G12 | NA | GACTACTTCATGACC | TACATTAGTAGTGCTGGT GGTGCCATATACTACGC AGACTCTGTGAAGGGC | GCAGTGGCTGCCCCTGGTGC TTTTGATATC |
| | | | SEQ ID NO:5281 | SEQ ID NO:13293 | SEQ ID NO:21305 |
| | | AA | DYFMT | YISSAGGAIYYADSVKG | AVAAPGAFDI |
| | | | SEQ ID NO:5282 | SEQ ID NO:13294 | SEQ ID NO:21306 |

FIGURE 49

| iPS:434019 | | NA | GACTATGGCATGCAC | GTTATATGGTATGATGA AGATAATAAATATTATG TAGACTCCGTGAAGGGC | GAACTGGGGTTCCTCTCTGA CTAC |
|---|---|---|---|---|---|
| | 21-225_49A1 | | SEQ ID NO:5283 | SEQ ID NO:13295 | SEQ ID NO:21307 |
| | | AA | DYGMH | VIWYDEDNKYYVDSVKG | ELGFLSDY |
| | | | SEQ ID NO:5284 | SEQ ID NO:13296 | SEQ ID NO:21308 |
| iPS:434021 | | NA | AGCTATGGCATGCAC | GTTATATGGTATGATGG AAGTAATAAATACTATG CAGACTCCGTGAAGGGC | AGGTATAGCAGCAGCTGGTC GGGCGGTATGGACGTC |
| | 21-225_49C1 | | SEQ ID NO:5285 | SEQ ID NO:13297 | SEQ ID NO:21309 |
| | | AA | SYGMH | VIWYDGSNKYYADSVKG | RYSSSWSGGMDV |
| | | | SEQ ID NO:5286 | SEQ ID NO:13298 | SEQ ID NO:21310 |
| iPS:434023 | | NA | AGCTATGCCATGAGC | GTTATTAGTGGTAGTGGT GGTAGCACATTCTACGC AGACTCCGTGAAGGGC | GCTATAGCAGCGGCTGGTGC CCACTATTTTGACTAC |
| | 21-225_49F1 | | SEQ ID NO:5287 | SEQ ID NO:13299 | SEQ ID NO:21311 |
| | | AA | SYAMS | VISGSGGSTFYADSVKG | AIAAAGAHYFDY |
| | | | SEQ ID NO:5288 | SEQ ID NO:13300 | SEQ ID NO:21312 |
| iPS:434025 | | NA | AGTTATGGCATGCAC | GTTATATGGTATGATGG AAGTAATAAATACTATG CAGACTCCGTGAAGGGC | AGGTATAGCAGCAGCTGGTC GGGCGGAATGGACGTC |
| | 21-225_49G3 | | SEQ ID NO:5289 | SEQ ID NO:13301 | SEQ ID NO:21313 |
| | | AA | SYGMH | VIWYDGSNKYYADSVKG | RYSSSWSGGMDV |
| | | | SEQ ID NO:5290 | SEQ ID NO:13302 | SEQ ID NO:21314 |

FIGURE 49

| iPS:434027 | | NA | AGCTATGCCATGACC | GCTATTAGTGGTAGTGGT GGTAACTCATTCTACGCA GACTCCGTGAAGGGC | GCAAGGGCAGTGGCTGGGTC ACACTGGTTCGACCCC |
|---|---|---|---|---|---|
| | 21-225_49H5 | | SEQ ID NO:5291 | SEQ ID NO:13303 | SEQ ID NO:21315 |
| | | AA | SYAMT | AISGSGGNSFYADSVKG | ARAVAGSHWFDP |
| | | | SEQ ID NO:5292 | SEQ ID NO:13304 | SEQ ID NO:21316 |
| iPS:434029 | | NA | AACTATGGCATGCAC | GTTATATGGTTTGATGTA AGTAATAAAAAGTATGT AGACTCCGTGAAGGGC | GATCTGGGGATGATCGAGGA CTAC |
| | 21-225_49C6 | | SEQ ID NO:5293 | SEQ ID NO:13305 | SEQ ID NO:21317 |
| | | AA | NYGMH | VIWFDVSNKKYVDSVKG | DLGMIEDY |
| | | | SEQ ID NO:5294 | SEQ ID NO:13306 | SEQ ID NO:21318 |
| iPS:434031 | | NA | AGCTATGGCATGCAC | GTTATATGGTATGATGG AAGTAATAAATACTATG CAGACTCCGTGAAGGGC | AGGTATAGCAGCAGCTGGTC GGGCGGTATGGACGTC |
| | 21-225_49E7 | | SEQ ID NO:5295 | SEQ ID NO:13307 | SEQ ID NO:21319 |
| | | AA | SYGMH | VIWYDGSNKYYADSVKG | RYSSSWSGGMDV |
| | | | SEQ ID NO:5296 | SEQ ID NO:13308 | SEQ ID NO:21320 |
| iPS:434033 | | NA | AGTTATGGCATGCAC | CTTATATGGTATGATGGA AGGAATAAATACTATGC AGACTCCGTGAAGGGC | AGGTATAGCAGCAGCTGGTC GGGCGGTATGGACGTC |
| | 21-225_49F9 | | SEQ ID NO:5297 | SEQ ID NO:13309 | SEQ ID NO:21321 |
| | | AA | SYGMH | LIWYDGRNKYYADSVKG | RYSSSWSGGMDV |
| | | | SEQ ID NO:5298 | SEQ ID NO:13310 | SEQ ID NO:21322 |

FIGURE 49

| iPS:434035 | | NA | GGCTACCATATGCAC | TGGATCAACCCTAATAA CAATGCCACAAACTATG CTCAGAACTTTCAGGGC | GACGGTACCAGCAGCTTTGA CTTC |
|---|---|---|---|---|---|
| | 21-225_49F10 | | SEQ ID NO:5299 | SEQ ID NO:13311 | SEQ ID NO:21323 |
| | | AA | GYHMH | WINPNNNATNYAQNFQG | DGTSSFDF |
| | | | SEQ ID NO:5300 | SEQ ID NO:13312 | SEQ ID NO:21324 |
| iPS:434037 | | NA | AGCTATGCCATGAGC | GTTATTAGTGGTAGTGGT GGTACCACATTCTACGC AGACTCCGTGAAGGGC | CGTATAGCAGTGGCTGGGAA TGATGCTTTTGATATC |
| | 21-225_49G12 | | SEQ ID NO:5301 | SEQ ID NO:13313 | SEQ ID NO:21325 |
| | | AA | SYAMS | VISGSGGTTFYADSVKG | RIAVAGNDAFDI |
| | | | SEQ ID NO:5302 | SEQ ID NO:13314 | SEQ ID NO:21326 |
| iPS:434039 | | NA | GACTACTACATGAAC | TACATTAATAGTAATGGT TTTACCATATACTACGCA GACTCTGTGAAGGGC | GATACAATCTAC |
| | 21-225_43B1 | | SEQ ID NO:5303 | SEQ ID NO:13315 | SEQ ID NO:21327 |
| | | AA | DYYMN | YINSNGFTIYYADSVKG | DTIY |
| | | | SEQ ID NO:5304 | SEQ ID NO:13316 | SEQ ID NO:21328 |
| iPS:434041 | | NA | AGTTATGCCATGAGC | GTTATTAGTGGTCGTGGT GGTACCACATTCTACGC AGACTCCGTGAAGGGC | CGTATAGCAGTGGCTGGGAA TGAGGCTTTTGATATC |
| | 21-225_50H8 | | SEQ ID NO:5305 | SEQ ID NO:13317 | SEQ ID NO:21329 |
| | | AA | SYAMS | VISGRGGTTFYADSVKG | RIAVAGNEAFDI |
| | | | SEQ ID NO:5306 | SEQ ID NO:13318 | SEQ ID NO:21330 |
| iPS:434043 | | NA | AGCTATAGCATGAAC | TCCATTAGTGGTAGTAGT AGTTACATATACTACGC AGACTCAGTGAAGGGC | GTAGCAACTTTTGACTAC |
| | 21-225_50G10 | | SEQ ID NO:5307 | SEQ ID NO:13319 | SEQ ID NO:21331 |

FIGURE 49

| | | | | | |
|---|---|---|---|---|---|
| | | AA | SYSMN | SISGSSSYIYYADSVKG | VATFDY |
| | | | SEQ ID NO:5308 | SEQ ID NO:13320 | SEQ ID NO:21332 |
| iPS:434045 | 21-225_50H10 | NA | AGTTATGCCATGAGC | GTTATTAGTGGTCGTGGTGGTAGCACATTCTACGCAGACTCCGTGAAGGGC | CGTATAGCAGTGGCTGGGAATGAGGCTTTTGATATC |
| | | | SEQ ID NO:5309 | SEQ ID NO:13321 | SEQ ID NO:21333 |
| | | AA | SYAMS | VISGRGGSTFYADSVKG | RIAVAGNEAFDI |
| | | | SEQ ID NO:5310 | SEQ ID NO:13322 | SEQ ID NO:21334 |
| iPS:434047 | 21-225_50A12 | NA | GGCCACTATATAAAC | TGGGTCAACCCTAACAGTGGTGGCACAAACTCTGCACAGAAGTTTCAGGGC | GGAGGGCAGCTCGGCGGGTTTAACTACTACTACTACGGTATGGACGTC |
| | | | SEQ ID NO:5311 | SEQ ID NO:13323 | SEQ ID NO:21335 |
| | | AA | GHYIN | WVNPNSGGTNSAQKFQG | GGQLGGFNYYYYGMDV |
| | | | SEQ ID NO:5312 | SEQ ID NO:13324 | SEQ ID NO:21336 |
| iPS:434049 | 21-225_50B12 | NA | AGCCATAGCATGAAC | TCCATCAGTAGTAGTAGTAATTACATATACTACGCAGACTCAGTGAAGGGC | GATCGGAGCATAGTAGTGGCTGGTCCCTGGGACTACTACGGTATGGACGTC |
| | | | SEQ ID NO:5313 | SEQ ID NO:13325 | SEQ ID NO:21337 |
| | | AA | SHSMN | SISSSSNYIYYADSVKG | DRSIVVAGPWDYYGMDV |
| | | | SEQ ID NO:5314 | SEQ ID NO:13326 | SEQ ID NO:21338 |
| iPS:434053 | 21-225_51E1 | NA | AGCTATGGCATGCAC | GTTATATGGTATGATGGAAGTAGTAAATACTATGCAGACTCCGTGAAGGGC | AGGTATAGCAGCAGTTGGTCGGGCGGTATGGACGTC |
| | | | SEQ ID NO:5315 | SEQ ID NO:13327 | SEQ ID NO:21339 |
| | | AA | SYGMH | VIWYDGSSKYYADSVKG | RYSSSWSGGMDV |
| | | | SEQ ID NO:5316 | SEQ ID NO:13328 | SEQ ID NO:21340 |
| iPS:434055 | 21 225 51B4 | NA | AGCTATGTCATGAGC | GCTATTAGTGGTCGTGGTAGTAACACATTCTACACAGACTCCGTGAAGGGC | GGGATAACTGGATCACACGGTGCTTTTGATATC |

FIGURE 49

| | | | SEQ ID NO:5317 | SEQ ID NO:13329 | SEQ ID NO:21341 |
|---|---|---|---|---|---|
| | **21-225_51B4** | AA | SYVMS | AISGRGSNTFYTDSVKG | GITGSHGAFDI |
| | | | SEQ ID NO:5318 | SEQ ID NO:13330 | SEQ ID NO:21342 |
| iPS:434057 | | NA | AGCTATGGCATGCAC | GTTATATGGTATGATGAAGTAATAAATACTATGCAGACTCCGTGAAGGGC | GAACTGGGATTTCTCTCTGACTAC |
| | **21-225_51E4** | | SEQ ID NO:5319 | SEQ ID NO:13331 | SEQ ID NO:21343 |
| | | AA | SYGMH | VIWYDESNKYYADSVKG | ELGFLSDY |
| | | | SEQ ID NO:5320 | SEQ ID NO:13332 | SEQ ID NO:21344 |
| iPS:434059 | | NA | AGCTATGTCATGAGC | ACTATGAGTGGTAGTGGTGGTCGCACATACTACGCAGACTCCGTGAACGGC | GTGACTGCTTTTGACTAC |
| | **21-225_51C5** | | SEQ ID NO:5321 | SEQ ID NO:13333 | SEQ ID NO:21345 |
| | | AA | SYVMS | TMSGSGGRTYYADSVNG | VTAFDY |
| | | | SEQ ID NO:5322 | SEQ ID NO:13334 | SEQ ID NO:21346 |
| iPS:434061 | | NA | AACTATGCCATGACC | GTTATTAGTGCTAGTGGTGGTAACTCATTCTACGCAGACTCCGTGAAGGGC | GCAAGGGCAGTGGCTGGGTCACACTGGTTCGACCCC |
| | **21-225_51C7** | | SEQ ID NO:5323 | SEQ ID NO:13335 | SEQ ID NO:21347 |
| | | AA | NYAMT | VISASGGNSFYADSVKG | ARAVAGSHWFDP |
| | | | SEQ ID NO:5324 | SEQ ID NO:13336 | SEQ ID NO:21348 |
| iPS:434063 | | NA | AGCTATAGCATGAAC | TCCATTAGTAGTAGTAGTAGTTACATATACTACGCAGACTCAGTGAAGGGC | GCTCGCCTTGACTAC |
| | **21-225_51G7** | | SEQ ID NO:5325 | SEQ ID NO:13337 | SEQ ID NO:21349 |
| | | AA | SYSMN | SISSSSSYIYYADSVKG | ARLDY |
| | | | SEQ ID NO:5326 | SEQ ID NO:13338 | SEQ ID NO:21350 |

FIGURE 49

| iPS:434065 | 21-225_50D4 | NA | GGCTACCATATGCAC | TGGATCAACCCTAATAA TAATGCCACAAACTATG CTCAGAGCTTTCAGGGC | GACGGTACCAGCAGCTTTGA CTTC |
|---|---|---|---|---|---|
| | | | SEQ ID NO:5327 | SEQ ID NO:13339 | SEQ ID NO:21351 |
| | | AA | GYHMH | WINPNNNATNYAQSFQG | DGTSSFDF |
| | | | SEQ ID NO:5328 | SEQ ID NO:13340 | SEQ ID NO:21352 |
| iPS:434067 | 21-225_51H8 | NA | GGCCACTATATGAAC | TGGGTCAACCCTAACAG TGGTGGCTCAAACTCTGC ACAGCAGTTTCAGGGC | GGAGGGCAGCTCGGCGGCTT TAACTTCTACTACTACGGTA TGGACGTC |
| | | | SEQ ID NO:5329 | SEQ ID NO:13341 | SEQ ID NO:21353 |
| | | AA | GHYMN | WVNPNSGGSNSAQQFQG | GGQLGGFNFYYYGMDV |
| | | | SEQ ID NO:5330 | SEQ ID NO:13342 | SEQ ID NO:21354 |
| iPS:434069 | 21-225_51E9 | NA | GGCTACCATATACAC | TGGATCAACCCTAACAC TAATGGCACACAGTATG CACAGAAGTTTCAGGGC | GATGGCACCTCGTCCTTTGA CTAC |
| | | | SEQ ID NO:5331 | SEQ ID NO:13343 | SEQ ID NO:21355 |
| | | AA | GYHIH | WINPNTNGTQYAQKFQG | DGTSSFDY |
| | | | SEQ ID NO:5332 | SEQ ID NO:13344 | SEQ ID NO:21356 |
| iPS:434071 | 21-225_51F9 | NA | GACTATGGCATGCAC | GTTATATGGTTTGATGGA AATAATAAATACTATGC AGACTCCGTGAAGGGC | GAGCTGGGATTTCTCTCTGA CTAC |
| | | | SEQ ID NO:5333 | SEQ ID NO:13345 | SEQ ID NO:21357 |
| | | AA | DYGMH | VIWFDGNNKYYADSVKG | ELGFLSDY |
| | | | SEQ ID NO:5334 | SEQ ID NO:13346 | SEQ ID NO:21358 |
| iPS:434073 | 21-225_51H10 | NA | AGCTATGCCATGAGC | GTTATTAGTGGTAGTGGT GGTACCACATTCTACGC AGACTCCGTGAAGGGC | CGTATAGCAGTGGCTGGGAA TGATGCTTTTGATATC |
| | | | SEQ ID NO:5335 | SEQ ID NO:13347 | SEQ ID NO:21359 |

FIGURE 49

| | | AA | SYAMS | VISGSGGTTFYADSVKG | RIAVAGNDAFDI |
|---|---|---|---|---|---|
| | | | SEQ ID NO:5336 | SEQ ID NO:13348 | SEQ ID NO:21360 |
| iPS:434075 | 21-225_51B11 | NA | GACTATGGCATGCAC | GTTATATGGTTTGGTGGA AATAATAAATACTATGG AGACTCCGTGAAGGGC | GAGCTGGGATTTCTCTCTGA CTAC |
| | | | SEQ ID NO:5337 | SEQ ID NO:13349 | SEQ ID NO:21361 |
| | | AA | DYGMH | VIWFGGNNKYYGDSVKG | ELGFLSDY |
| | | | SEQ ID NO:5338 | SEQ ID NO:13350 | SEQ ID NO:21362 |
| iPS:434077 | 21-225_51F11 | NA | AACTTTGGCATGCAC | GTTATATGGTATGAGGA AAGTAATAAATACTATG CAGACTCCGTGAAGGGC | GAACTGGGGTTCCTCTCTGA CTTC |
| | | | SEQ ID NO:5339 | SEQ ID NO:13351 | SEQ ID NO:21363 |
| | | AA | NFGMH | VIWYEESNKYYADSVKG | ELGFLSDF |
| | | | SEQ ID NO:5340 | SEQ ID NO:13352 | SEQ ID NO:21364 |
| iPS:434079 | 21-225_52B1 | NA | GGCTATCATATGCAG | TGGATCAACCCTAACAG TGGTGCCACAAACTATG CACAGAACTTTCAGGGC | GATGGCACCTCGTCCTTTGA CTAC |
| | | | SEQ ID NO:5341 | SEQ ID NO:13353 | SEQ ID NO:21365 |
| | | AA | GYHMQ | WINPNSGATNYAQNFQG | DGTSSFDY |
| | | | SEQ ID NO:5342 | SEQ ID NO:13354 | SEQ ID NO:21366 |
| iPS:434081 | 21-225_52B2 | NA | AACTATGGCATGCAC | GTTACATGGTTTGATGGA AGTAATCAACGCTATGC AGACTCCGTGAAGGGC | GATCTGGGGATGATCGAGGA CTTC |
| | | | SEQ ID NO:5343 | SEQ ID NO:13355 | SEQ ID NO:21367 |
| | | AA | NYGMH | VTWFDGSNQRYADSVKG | DLGMIEDF |
| | | | SEQ ID NO:5344 | SEQ ID NO:13356 | SEQ ID NO:21368 |

FIGURE 49

| iPS:434083 | | NA | AGAAATGCCATGAGC | GCTATTAGTGGTCGTGGT GGTAATACATTCTACGC AGACTCCGTGAAGGGC | AATGGGCGAGAGCAGTGGCT TGACTAC |
|---|---|---|---|---|---|
| | 21-225_52H2 | | SEQ ID NO:5345 | SEQ ID NO:13357 | SEQ ID NO:21369 |
| | | AA | RNAMS | AISGRGGNTFYADSVKG | NGREQWLDY |
| | | | SEQ ID NO:5346 | SEQ ID NO:13358 | SEQ ID NO:21370 |
| iPS:434085 | | NA | AGCTATAAAATGAAC | TCCATTAGTAGTGGTAAT AGTTCCATATACTACGCA GACTCAGTGAAGGGC | GTTAGCAGTAATGACTAC |
| | 21-225_52E3 | | SEQ ID NO:5347 | SEQ ID NO:13359 | SEQ ID NO:21371 |
| | | AA | SYKMN | SISSGNSSIYYADSVKG | VSSNDY |
| | | | SEQ ID NO:5348 | SEQ ID NO:13360 | SEQ ID NO:21372 |
| iPS:434087 | | NA | AGCTATGGCATGCAC | ATTATATCATATGGAGG AAGTAATAAATACTATG CAGACTCCGTGAAGGGC | AGGTCAGCAGCTCGGCCGGG CTACGGTATGGACGTC |
| | 21-225_52F6 | | SEQ ID NO:5349 | SEQ ID NO:13361 | SEQ ID NO:21373 |
| | | AA | SYGMH | IISYGGSNKYYADSVKG | RSAARPGYGMDV |
| | | | SEQ ID NO:5350 | SEQ ID NO:13362 | SEQ ID NO:21374 |
| iPS:434091 | | NA | GACTATGGCATGCAC | GTTATATGGTTTGATGGA AATAATAAATACTATGC AGACTCCGTGAAGGGC | GAGCTGGGATTTCTCTCTGA CTAC |
| | 21-225_52B9 | | SEQ ID NO:5351 | SEQ ID NO:13363 | SEQ ID NO:21375 |
| | | AA | DYGMH | VIWFDGNNKYYADSVKG | ELGFLSDY |
| | | | SEQ ID NO:5352 | SEQ ID NO:13364 | SEQ ID NO:21376 |

FIGURE 49

| iPS:434093 | | NA | AGTTATGGCATGCAC | CTTATATGGTATGATGGAAGTAATAAATACCATGCAGACTCCGTGAAGGGC | AGGTATAGCAGCAGCTGGTCGGGCGGTATGGACGTC |
|---|---|---|---|---|---|
| | 21-225_52D10 | | SEQ ID NO:5353 | SEQ ID NO:13365 | SEQ ID NO:21377 |
| | | AA | SYGMH | LIWYDGSNKYHADSVKG | RYSSSWSGGMDV |
| | | | SEQ ID NO:5354 | SEQ ID NO:13366 | SEQ ID NO:21378 |
| iPS:434095 | | NA | TTCTATGGCATGCAC | GTTATATGGGACGATGGAAGTAATAAATACTATGCAGACTCCGTGAAGGGC | GATTCTCTGTATAGCAGCAGCTGGTTGTTTGACTAC |
| | 21-225_52F10 | | SEQ ID NO:5355 | SEQ ID NO:13367 | SEQ ID NO:21379 |
| | | AA | FYGMH | VIWDDGSNKYYADSVKG | DSLYSSSWLFDY |
| | | | SEQ ID NO:5356 | SEQ ID NO:13368 | SEQ ID NO:21380 |
| iPS:434097 | | NA | GGCTACCATATGCAG | TGGATCAACCCTAACAATGGTGGCACACAGTATGCACAGAAGTTTCAGGGC | GATGGCACCTCGTCCTTTGACTAC |
| | 21-225_52H10 | | SEQ ID NO:5357 | SEQ ID NO:13369 | SEQ ID NO:21381 |
| | | AA | GYHMQ | WINPNNGGTQYAQKFQG | DGTSSFDY |
| | | | SEQ ID NO:5358 | SEQ ID NO:13370 | SEQ ID NO:21382 |
| iPS:434101 | | NA | AGCTATAGCATGAAC | TCCATTAGTGGTAGTAGTACTTACATATACTACGCAGACTCAGTGAAGGGC | GTCAACTCCTTTGACTAC |
| | 21-225_52H12 | | SEQ ID NO:5359 | SEQ ID NO:13371 | SEQ ID NO:21383 |
| | | AA | SYSMN | SISGSSTYIYYADSVKG | VNSFDY |
| | | | SEQ ID NO:5360 | SEQ ID NO:13372 | SEQ ID NO:21384 |

FIGURE 49

| iPS:434103 | 21-225_53G1 | NA | AGCTATAGCATGAAC | TCCATTAGTGGTAGTAGTAGTTACATATACTACGCAGACTCAGTGAAGGGC | GATCGGGGCAGCACC |
| | | | SEQ ID NO:5361 | SEQ ID NO:13373 | SEQ ID NO:21385 |
| | | AA | SYSMN | SISGSSSYIYYADSVKG | DRGST |
| | | | SEQ ID NO:5362 | SEQ ID NO:13374 | SEQ ID NO:21386 |
| iPS:434105 | 21-225_53D2 | NA | AACTATGGCATGCAC | GTTGTATGGGATGATGGAAGTAATAAATACTATGCAGACTCCGTGAAGGGC | GGCCTTGGCTTTACGGGAGACTAC |
| | | | SEQ ID NO:5363 | SEQ ID NO:13375 | SEQ ID NO:21387 |
| | | AA | NYGMH | VVWDDGSNKYYADSVKG | GLGFTGDY |
| | | | SEQ ID NO:5364 | SEQ ID NO:13376 | SEQ ID NO:21388 |
| iPS:434107 | 21-225_53E2 | NA | AGCTATGCCATGAGC | GCTATTAGTGGTAGTGGTGGTAACACATTCTACGCAGACTCCGTGAAGGGC | AAGGTCGTGGATACAGCCATGGCTCTTGACTAC |
| | | | SEQ ID NO:5365 | SEQ ID NO:13377 | SEQ ID NO:21389 |
| | | AA | SYAMS | AISGSGGNTFYADSVKG | KVVDTAMALDY |
| | | | SEQ ID NO:5366 | SEQ ID NO:13378 | SEQ ID NO:21390 |
| iPS:434111 | 21-225_53H2 | NA | AGCTATGGCATGCAC | GTTATATCATATGGTGGAAGTAATAAATACCATGCAGACTCCGTGAAGGGC | CGGGGAGCAGCTCGTCCTGGCTACGGTATGGACGTC |
| | | | SEQ ID NO:5367 | SEQ ID NO:13379 | SEQ ID NO:21391 |
| | | AA | SYGMH | VISYGGSNKYHADSVKG | RGAARPGYGMDV |
| | | | SEQ ID NO:5368 | SEQ ID NO:13380 | SEQ ID NO:21392 |
| iPS:434115 | 21 225 53E4 | NA | AGCTATGTCATGAGC | GGTATTAGTGGTAGTGGTGGTCGCACATACTACGCAGACTCCGTGAAGGGC | GTGGCCCTTTTTGACTAC |

FIGURE 49

| | | | | | |
|---|---|---|---|---|---|
| | 21-225_53E4 | | SEQ ID NO:5369 | SEQ ID NO:13381 | SEQ ID NO:21393 |
| | | AA | SYVMS | GISGSGGRTYYADSVKG | VALFDY |
| | | | SEQ ID NO:5370 | SEQ ID NO:13382 | SEQ ID NO:21394 |
| iPS:434117 | 21-225_53C6 | NA | AGCTATGCCATGAAC | GCTATTAGTGGTAGTGGTGGTGCCACATACTACGCAGACTCCGTGAAGGGC | CCTCTAGTGGGAGCCCATGATGCTTTTGAAATC |
| | | | SEQ ID NO:5371 | SEQ ID NO:13383 | SEQ ID NO:21395 |
| | | AA | SYAMN | AISGSGGATYYADSVKG | PLVGAHDAFEI |
| | | | SEQ ID NO:5372 | SEQ ID NO:13384 | SEQ ID NO:21396 |
| iPS:434119 | 21-225_53E6 | NA | GACTATGGCATCCAC | GTTATATGGTATGATGAAGTAATAAATACTATGGAGACTCCGTGAAGGGC | GAACTGGGGATGACGTCTGACTAC |
| | | | SEQ ID NO:5373 | SEQ ID NO:13385 | SEQ ID NO:21397 |
| | | AA | DYGIH | VIWYDESNKYYGDSVKG | ELGMTSDY |
| | | | SEQ ID NO:5374 | SEQ ID NO:13386 | SEQ ID NO:21398 |
| iPS:434121 | 21-225_53F6 | NA | AGCTATGGCATGCAC | GTTATATCATATGGTGGAAGTAATAAATACGATGCAGACTCCGTGAAGGGC | CGACGGGCAGCTCGTCCAGGGTACGGTATGGACGTC |
| | | | SEQ ID NO:5375 | SEQ ID NO:13387 | SEQ ID NO:21399 |
| | | AA | SYGMH | VISYGGSNKYDADSVKG | RRAARPGYGMDV |
| | | | SEQ ID NO:5376 | SEQ ID NO:13388 | SEQ ID NO:21400 |
| iPS:434123 | 21-225_53F7 | NA | GGCTACCATATGCAC | TGGATCAACCCTAACAATAACGGCACAAACTATGCACAGAAGTTTCAGGGC | GACGGTACCAGCAGCTTTGACTAC |
| | | | SEQ ID NO:5377 | SEQ ID NO:13389 | SEQ ID NO:21401 |

FIGURE 49

| | | | | | |
|---|---|---|---|---|---|
| | | AA | GYHMH | WINPNNNGTNYAQKFQG | DGTSSFDY |
| | | | SEQ ID NO:5378 | SEQ ID NO:13390 | SEQ ID NO:21402 |
| iPS:434127 | 21-225_53H8 | NA | AGCTATGGCATGCAC | GTTATATGGTATGATGG AAGTAATAAATACTATG CAGACTCCGTGAAGGGC | GCCCGTATTGGGTACTTTGA CTCC |
| | | | SEQ ID NO:5379 | SEQ ID NO:13391 | SEQ ID NO:21403 |
| | | AA | SYGMH | VIWYDGSNKYYADSVKG | ARIGYFDS |
| | | | SEQ ID NO:5380 | SEQ ID NO:13392 | SEQ ID NO:21404 |
| iPS:434129 | 21-225_53B12 | NA | AACTTTGGCATGCAC | GTTGTATGGTATGATGG AAATAATAGATACTATG CAGACTCCGTGAAGGGC | GAACTGGGATTTCTCTCTGA CTTC |
| | | | SEQ ID NO:5381 | SEQ ID NO:13393 | SEQ ID NO:21405 |
| | | AA | NFGMH | VVWYDGNNRYYADSVKG | ELGFLSDF |
| | | | SEQ ID NO:5382 | SEQ ID NO:13394 | SEQ ID NO:21406 |
| iPS:434131 | 21-225_54D3 | NA | AACTATGGCATGCAC | GTTACATGGTTTGATGGA AATAATAACTACTATGC AGACTCCGTGAAGGGC | GAACTGGGGTTCCTTTCTGA TTAT |
| | | | SEQ ID NO:5383 | SEQ ID NO:13395 | SEQ ID NO:21407 |
| | | AA | NYGMH | VTWFDGNNNYYADSVKG | ELGFLSDY |
| | | | SEQ ID NO:5384 | SEQ ID NO:13396 | SEQ ID NO:21408 |
| iPS:434133 | 21 225 54G3 | NA | ACCTATGCCATGAGT | GCTATTAGTGGTAGTGGT GTTAACACATTCTACGCA GACTCCGTGAAGGGC | CTGGGGAAGGACTACTACTA CTACGGTATGGACGTC |

FIGURE 49

| | 21-225_54G3 | | SEQ ID NO:5385 | SEQ ID NO:13397 | SEQ ID NO:21409 |
|---|---|---|---|---|---|
| | | AA | TYAMS | AISGSGVNTFYADSVKG | LGKDYYYYGMDV |
| iPS:434135 | 21-225_54H3 | NA | SEQ ID NO:5386<br>AGCTATAGCATGATC | SEQ ID NO:13398<br>TCCATTAGTGGTACTAGT<br>AGTTACATATACTACGC<br>AGACTCAGTGAAGGGC | SEQ ID NO:21410<br>ATGACTACAGTAATT |
| | | | SEQ ID NO:5387 | SEQ ID NO:13399 | SEQ ID NO:21411 |
| | | AA | SYSMI | SISGTSSYIYYADSVKG | MTTVI |
| iPS:434137 | 21-225_54D4 | NA | SEQ ID NO:5388<br>AGCTATGGCATGCAC | SEQ ID NO:13400<br>GTTATATGGTATGATGG<br>AAGTAATAAATACTATG<br>CAGACTCCGTGAAGGGC | SEQ ID NO:21412<br>AGGTATAGCAGCAGCTGGTC<br>GGGCGGTATGGACGTC |
| | | | SEQ ID NO:5389 | SEQ ID NO:13401 | SEQ ID NO:21413 |
| | | AA | SYGMH | VIWYDGSNKYYADSVKG | RYSSSWSGGMDV |
| iPS:434141 | 21-225_54C6 | NA | SEQ ID NO:5390<br>GACTATGGCATCCAC | SEQ ID NO:13402<br>GTTATATGGTATGATGA<br>AAATAATAAATACTATG<br>CAGACTCCGTGAAGGGC | SEQ ID NO:21414<br>GAACTGGGGATGACGTCTGA<br>CTAC |
| | | | SEQ ID NO:5391 | SEQ ID NO:13403 | SEQ ID NO:21415 |
| | | AA | DYGIH | VIWYDENNKYYADSVKG | ELGMTSDY |
| iPS:434143 | 21-225_54G7 | NA | SEQ ID NO:5392<br>AACTATGGCATGCAC | SEQ ID NO:13404<br>GTTATATGGTATGAGGA<br>AAGTAATAAATACTATG<br>GAGACTCCGTGAAGGGC | SEQ ID NO:21416<br>GAATTGGGGTTCCTCTCTGA<br>CTAC |
| | | | SEQ ID NO:5393 | SEQ ID NO:13405 | SEQ ID NO:21417 |

FIGURE 49

| | | | | | |
|---|---|---|---|---|---|
| | | AA | NYGMH | VIWYEESNKYYGDSVKG | ELGFLSDY |
| | | | SEQ ID NO:5394 | SEQ ID NO:13406 | SEQ ID NO:21418 |
| iPS:434145 | | NA | GGCTACTATTTCCAC | TGGATCCACCCTAACAA TAATGCCACAAACTATG CACAGAAGTTTCAGGGC | GATGGCAGATCGTCCTTTGA CTAC |
| | 21-225_55B1 | | SEQ ID NO:5395 | SEQ ID NO:13407 | SEQ ID NO:21419 |
| | | AA | GYYFH | WIHPNNNATNYAQKFQG | DGRSSFDY |
| | | | SEQ ID NO:5396 | SEQ ID NO:13408 | SEQ ID NO:21420 |
| iPS:434147 | | NA | AGCTATGCCATGAGC | GCTATTAGTGGTCGTGGT AGTAGCACATTCTACGC AGACTCCGTGAAGGGC | GATCACGGTATAGTGGGAAC TATTTACTTTGACTAC |
| | 21-225_55E1 | | SEQ ID NO:5397 | SEQ ID NO:13409 | SEQ ID NO:21421 |
| | | AA | SYAMS | AISGRGSSTFYADSVKG | DHGIVGTIYFDY |
| | | | SEQ ID NO:5398 | SEQ ID NO:13410 | SEQ ID NO:21422 |
| iPS:434149 | | NA | AGCTATGGCATGCAC | GTTATATGGTATGATGG AAGTAATAAATACTATG CAGACTCCGTGAAGGGC | AGGTATAGCAGCAGCTGGTC GGGCGGTATGGACGTC |
| | 21-225_55H1 | | SEQ ID NO:5399 | SEQ ID NO:13411 | SEQ ID NO:21423 |
| | | AA | SYGMH | VIWYDGSNKYYADSVKG | RYSSSWSGGMDV |
| | | | SEQ ID NO:5400 | SEQ ID NO:13412 | SEQ ID NO:21424 |
| iPS:434151 | | NA | AGTTATGGCATGCAC | CTTATATGGTATGATGGA AGTAATAAATACCATGC AGACTCCGTGAAGGGC | AGGTATAGCAGCAGCTGGTC GGGCGGTATGGACGTC |
| | 21-225_55C2 | | SEQ ID NO:5401 | SEQ ID NO:13413 | SEQ ID NO:21425 |
| | | AA | SYGMH | LIWYDGSNKYHADSVKG | RYSSSWSGGMDV |

FIGURE 49

| | | | SEQ ID NO:5402 | SEQ ID NO:13414 | SEQ ID NO:21426 |
|---|---|---|---|---|---|
| iPS:434155 | 21-225_55B3 | NA | AGCTATGGCATGCAC | GTTATATGGTTTGATGGA AATAATAAATACTATGA AGACTCCGTGAAGGGC | GAACTGGGATTTCTCTCTGA CTAC |
| | | | SEQ ID NO:5403 | SEQ ID NO:13415 | SEQ ID NO:21427 |
| | | AA | SYGMH | VIWFDGNNKYYEDSVKG | ELGFLSDY |
| | | | SEQ ID NO:5404 | SEQ ID NO:13416 | SEQ ID NO:21428 |
| iPS:434157 | 21-225_55D4 | NA | AGTTATAGGATGAAC | TCCATTAGTAGTAGTAGT AATCACATAGACTACGC AGACTCAGTGAAGGGC | GGGACTGACTAC |
| | | | SEQ ID NO:5405 | SEQ ID NO:13417 | SEQ ID NO:21429 |
| | | AA | SYRMN | SISSSSNHIDYADSVKG | GTDY |
| | | | SEQ ID NO:5406 | SEQ ID NO:13418 | SEQ ID NO:21430 |
| iPS:434159 | 21-225_55B8 | NA | GACTATGGCATGCAC | GTTATATGGTATGATGA AAATAATAAATACTATG CAGACTCCGTGAAGGGC | GAATGGTTTGACTAC |
| | | | SEQ ID NO:5407 | SEQ ID NO:13419 | SEQ ID NO:21431 |
| | | AA | DYGMH | VIWYDENNKYYADSVKG | EWFDY |
| | | | SEQ ID NO:5408 | SEQ ID NO:13420 | SEQ ID NO:21432 |
| iPS:434161 | 21-225_55F9 | NA | AGCTATGGCATGCAC | GTTATATGGTATGATGG AAATGGCAAATATTATG CAGACTCCGTGAAGGGC | AGGTATAGCAGCAGCTGGTC GGGCGGTATGGACGTC |
| | | | SEQ ID NO:5409 | SEQ ID NO:13421 | SEQ ID NO:21433 |
| | | AA | SYGMH | VIWYDGNGKYYADSVKG | RYSSSWSGGMDV |
| | | | SEQ ID NO:5410 | SEQ ID NO:13422 | SEQ ID NO:21434 |

FIGURE 49

| iPS:434163 | | NA | AGCTATGGCATGCAC | ATTATATCATATGGTGGA AGTAATAAATACGATGC AGACTCCGTGAAGGGC | CGACGGGCAGCTCGTCCAGG GTACGGTATGGACGTC |
|---|---|---|---|---|---|
| | 21-225_50H1 | | SEQ ID NO:5411 | SEQ ID NO:13423 | SEQ ID NO:21435 |
| | | AA | SYGMH | IISYGGSNKYDADSVKG | RRAARPGYGMDV |
| | | | SEQ ID NO:5412 | SEQ ID NO:13424 | SEQ ID NO:21436 |
| iPS:434165 | | NA | AGCTATGGCATGCAC | GTTATATGGCATGATGG AAGTAATAAATACTATG CAGACTCCGTGAAGGGC | GACGAGCAGCTCGGGACCTT TGACTAC |
| | 21-225_50F2 | | SEQ ID NO:5413 | SEQ ID NO:13425 | SEQ ID NO:21437 |
| | | AA | SYGMH | VIWHDGSNKYYADSVKG | DEQLGTFDY |
| | | | SEQ ID NO:5414 | SEQ ID NO:13426 | SEQ ID NO:21438 |
| iPS:434167 | | NA | ACCTATGCCATGACC | GCTATCAGTGGTAGTGG TGTTAACTCATTCTACGC AGACTCCGTGAAGGGC | GCAAGGGCAGTGGCTGGGTC ACACTGGTTCGACCCC |
| | 21-225_50F3 | | SEQ ID NO:5415 | SEQ ID NO:13427 | SEQ ID NO:21439 |
| | | AA | TYAMT | AISGSGVNSFYADSVKG | ARAVAGSHWFDP |
| | | | SEQ ID NO:5416 | SEQ ID NO:13428 | SEQ ID NO:21440 |
| iPS:434169 | | NA | GACTATGGCATGCAC | GTTATATGGTATGAAGA AACTAATAAATACTATG CAGACTCCGTGAAGGGC | GAAGTGGGGTTCCTGAATGA CTAC |
| | 21-225_50C4 | | SEQ ID NO:5417 | SEQ ID NO:13429 | SEQ ID NO:21441 |
| | | AA | DYGMH | VIWYEETNKYYADSVKG | EVGFLNDY |
| | | | SEQ ID NO:5418 | SEQ ID NO:13430 | SEQ ID NO:21442 |

FIGURE 49

| iPS:434171 | | NA | AGTTACTATATACAC | GTAATCAACCCTAGTAA TGGTAGAACAAGCTACG CACAGAAGTTCCAGGGC | GATCGAGGAGATGGTTACTA CTTCTACTACGGTATGGACG TC |
|---|---|---|---|---|---|
| | 21-225_50G4 | | SEQ ID NO:5419 | SEQ ID NO:13431 | SEQ ID NO:21443 |
| | | AA | SYYIH | VINPSNGRTSYAQKFQG | DRGDGYYFYYGMDV |
| | | | SEQ ID NO:5420 | SEQ ID NO:13432 | SEQ ID NO:21444 |
| iPS:434175 | | NA | AGCTATGGCATGCAC | GTTATATCATATGTTGGA AGTACTAAATACTATGC AGACTCCGTGAGGGGC | GGGAGAGGTCGATATAGTGA CTACGGTCATGATGCTTTTG ATATC |
| | 21-225_55A11 | | SEQ ID NO:5421 | SEQ ID NO:13433 | SEQ ID NO:21445 |
| | | AA | SYGMH | VISYVGSTKYYADSVRG | GRGRYSDYGHDAFDI |
| | | | SEQ ID NO:5422 | SEQ ID NO:13434 | SEQ ID NO:21446 |
| iPS:434177 | | NA | AATTATGATATCAAC | TGGATGCACCCTAACAG TGGTAACACAGGCTATG CACAGAAGTTCCAGGGC | AGCAGTGGCTGGTACGTTTT TGACTAC |
| | 21-225_56A1 | | SEQ ID NO:5423 | SEQ ID NO:13435 | SEQ ID NO:21447 |
| | | AA | NYDIN | WMHPNSGNTGYAQKFQG | SSGWYVFDY |
| | | | SEQ ID NO:5424 | SEQ ID NO:13436 | SEQ ID NO:21448 |
| iPS:434179 | | NA | AGCTATAGCATGAAC | TCCATTAGTAGTAGTAGT ACTTACATATACTACGG AGACTCAGTGAAGGGC | GATCGGGGCAGCAGC |
| | 21-225_56F1 | | SEQ ID NO:5425 | SEQ ID NO:13437 | SEQ ID NO:21449 |
| | | AA | SYSMN | SISSSSTYIYYGDSVKG | DRGSS |
| | | | SEQ ID NO:5426 | SEQ ID NO:13438 | SEQ ID NO:21450 |
| iPS:434181 | | NA | AGCTATGCCATGAGC | GCTATTAGTGGTAGTGGT GGTAACACATTCTACGC AGACTCCGTGAAGGGC | AAGGTCGTGGATACAGCCAT GGCTCTTGACTAC |
| | 21-225_56B2 | | SEQ ID NO:5427 | SEQ ID NO:13439 | SEQ ID NO:21451 |
| | | AA | SYAMS | AISGSGGNTFYADSVKG | KVVDTAMALDY |

FIGURE 49

| | | | SEQ ID NO:5428 | SEQ ID NO:13440 | SEQ ID NO:21452 |
|---|---|---|---|---|---|
| iPS:434187 | 21-225_56A5 | NA | AGCTATAGCATGAAC | TCCATTAGTGGTAGTAGT AGTTACATATACTACGC AGACTCAGTGAAGGGC | GTGGCTACTTTTGACTAC |
| | | | SEQ ID NO:5429 | SEQ ID NO:13441 | SEQ ID NO:21453 |
| | | AA | SYSMN | SISGSSSYIYYADSVKG | VATFDY |
| | | | SEQ ID NO:5430 | SEQ ID NO:13442 | SEQ ID NO:21454 |
| iPS:434189 | 21-225_56E5 | NA | GGCTACCATATGCAC | TGGATCAACCCTAACAA TAATGCCACAAACTATG CACAGAAGTTTCAGGGC | GATGGCACCTCGTCTTTTGA CTAC |
| | | | SEQ ID NO:5431 | SEQ ID NO:13443 | SEQ ID NO:21455 |
| | | AA | GYHMH | WINPNNNATNYAQKFQG | DGTSSFDY |
| | | | SEQ ID NO:5432 | SEQ ID NO:13444 | SEQ ID NO:21456 |
| iPS:434191 | 21-225_56B6 | NA | AGCTATGGCATGCAC | GTTATATGGCATGATGG AAGTAATAAATATTATG TAGACTCCGTGAAGGGC | GACGAGCAGCTCGGGACCTT TGACTAC |
| | | | SEQ ID NO:5433 | SEQ ID NO:13445 | SEQ ID NO:21457 |
| | | AA | SYGMH | VIWHDGSNKYYVDSVKG | DEQLGTFDY |
| | | | SEQ ID NO:5434 | SEQ ID NO:13446 | SEQ ID NO:21458 |
| iPS:434193 | 21-225_56C6 | NA | GACTACCATATGCAC | TGGATCAACCCTAACAG AGGTGGCACAAATTATG TACAGAAGTTTCAGGGT | GATGGCACCTCGTCTTTTGA CTAT |
| | | | SEQ ID NO:5435 | SEQ ID NO:13447 | SEQ ID NO:21459 |
| | | AA | DYHMH | WINPNRGGTNYVQKFQG | DGTSSFDY |
| | | | SEQ ID NO:5436 | SEQ ID NO:13448 | SEQ ID NO:21460 |

FIGURE 49

| iPS:434195 | | NA | GACTACTATATGCAC | TGGATCAACCCTAACAG TGGTGGCACAAACTATG CACAGAGGTTTCAGGGC | GAGGGAGCAACTCGTCCGAC GGGTTTTGACTAC |
|---|---|---|---|---|---|
| | 21-225_56F6 | | SEQ ID NO:5437 | SEQ ID NO:13449 | SEQ ID NO:21461 |
| | | AA | DYYMH | WINPNSGGTNYAQRFQG | EGATRPTGFDY |
| | | | SEQ ID NO:5438 | SEQ ID NO:13450 | SEQ ID NO:21462 |
| iPS:434197 | | NA | GGCCACTATATAAAC | TGGGTCAACCCTAACAG TGGTGGCACAAACTCTG CACAGAAGTTTCAGGGC | GGAGGGCAGCTCGGCGGGTT TAACTACTACTACTACGGTA TGGACGTC |
| | 21-225_56C7 | | SEQ ID NO:5439 | SEQ ID NO:13451 | SEQ ID NO:21463 |
| | | AA | GHYIN | WVNPNSGGTNSAQKFQG | GGQLGGFNYYYYGMDV |
| | | | SEQ ID NO:5440 | SEQ ID NO:13452 | SEQ ID NO:21464 |
| iPS:434199 | | NA | AACTATGGCATGCAC | GTTATATGGTATGATGA AAGTAATAAACACTATG CAGACTCCGTGAAGGGC | GAACTGGGGATGAACGGAG ACTAC |
| | 21-225_59F11 | | SEQ ID NO:5441 | SEQ ID NO:13453 | SEQ ID NO:21465 |
| | | AA | NYGMH | VIWYDESNKHYADSVKG | ELGMNGDY |
| | | | SEQ ID NO:5442 | SEQ ID NO:13454 | SEQ ID NO:21466 |
| iPS:434201 | | NA | AGCTATGGCATGCAC | GTTATATGGTATGATGG AAGTAATAAATACTATG CAGACTCCGTGAAGGGC | CGGTATAGCAGCAGCTGGGA CGGGGGGTATGGACGTC |
| | 21-225_59A12 | | SEQ ID NO:5443 | SEQ ID NO:13455 | SEQ ID NO:21467 |
| | | AA | SYGMH | VIWYDGSNKYYADSVKG | RYSSSWDGGMDV |
| | | | SEQ ID NO:5444 | SEQ ID NO:13456 | SEQ ID NO:21468 |

FIGURE 49

| iPS:434203 | | NA | GACTATGGCATGCAC | ATTATATGGTATGATGA AAATAATAAATACTATG CAGACTCCGTGAAGGGC | GATGTTCTGGACCCTTTTGA CTAC |
|---|---|---|---|---|---|
| | 21-225_60E2 | | SEQ ID NO:5445 | SEQ ID NO:13457 | SEQ ID NO:21469 |
| | | AA | DYGMH | IIWYDENNKYYADSVKG | DVLDPFDY |
| | | | SEQ ID NO:5446 | SEQ ID NO:13458 | SEQ ID NO:21470 |
| iPS:434205 | | NA | AGTTATGGCATGCAC | GTTATATGGTATGATGG AAGTAATAAATACTATG CAGACTCCGTGAAGGGC | AGGTATAGCAGAAGCTGGAC GGGAGGCATGGACGTC |
| | 21-225_60G2 | | SEQ ID NO:5447 | SEQ ID NO:13459 | SEQ ID NO:21471 |
| | | AA | SYGMH | VIWYDGSNKYYADSVKG | RYSRSWTGGMDV |
| | | | SEQ ID NO:5448 | SEQ ID NO:13460 | SEQ ID NO:21472 |
| iPS:434207 | | NA | AGCTATGGCATGCAC | GTTATATGGTATGAAGA AAGTAATAAATACTATG CAGACTCCGTGAAGGGC | GAACTGGGGATGACCGGAGA CTAC |
| | 21-225_60A3 | | SEQ ID NO:5449 | SEQ ID NO:13461 | SEQ ID NO:21473 |
| | | AA | SYGMH | VIWYEESNKYYADSVKG | ELGMTGDY |
| | | | SEQ ID NO:5450 | SEQ ID NO:13462 | SEQ ID NO:21474 |
| iPS:434209 | | NA | GGCCACTATATACAC | TGGATCAACCCTAACAG CGGTGGCACAAACTATG TACAGAAATTTCAGGGC | GGGGGCCTACTGGGAGCTAC CAACTACTATTATTACGGTA TGGACGTC |
| | 21-225_60C3 | | SEQ ID NO:5451 | SEQ ID NO:13463 | SEQ ID NO:21475 |
| | | AA | GHYIH | WINPNSGGTNYVQKFQG | GGLLGATNYYYYGMDV |
| | | | SEQ ID NO:5452 | SEQ ID NO:13464 | SEQ ID NO:21476 |

FIGURE 49

| iPS:434211 | | NA | AATTATGATATCAAC | TGGATGCACCCTAACAG TGGTAACACAGGCTATG CACAGAAGTTCCAGGGC | AGCAGTGGCTGGTACTTCTT TGACTAC |
|---|---|---|---|---|---|
| | 21-225_60F3 | | SEQ ID NO:5453 | SEQ ID NO:13465 | SEQ ID NO:21477 |
| | | AA | NYDIN | WMHPNSGNTGYAQKFQG | SSGWYFFDY |
| | | | SEQ ID NO:5454 | SEQ ID NO:13466 | SEQ ID NO:21478 |
| iPS:434213 | | NA | AGCTATGTCATGAGC | TCTATTAGTGGTAGTGGT GGTTGGACAAACTACGC AGACTCCGTGAAGGGC | CTAACTGGATTTGACTAT |
| | 21-225_60A4 | | SEQ ID NO:5455 | SEQ ID NO:13467 | SEQ ID NO:21479 |
| | | AA | SYVMS | SISGSGGWTNYADSVKG | LTGFDY |
| | | | SEQ ID NO:5456 | SEQ ID NO:13468 | SEQ ID NO:21480 |
| iPS:434215 | | NA | AGCTATGTCATGAGC | GGTATTAGTGGTAGTGG TAATAGAACATACTACG CAGACTCCGTGAAGGGC | TTGGGGATTGAC |
| | 21-225_60F7 | | SEQ ID NO:5457 | SEQ ID NO:13469 | SEQ ID NO:21481 |
| | | AA | SYVMS | GISGSGNRTYYADSVKG | LGID |
| | | | SEQ ID NO:5458 | SEQ ID NO:13470 | SEQ ID NO:21482 |
| iPS:434217 | | NA | AGGAGTAGTTACTACTGGGG C | AGTATCTATTATAGTGGG AGCGCCTCCTACAACCC GTCCCTCAAGAGT | CTGGACAGTGGCTGGTCGTT TGACTAC |
| | 21-225_60E8 | | SEQ ID NO:5459 | SEQ ID NO:13471 | SEQ ID NO:21483 |
| | | AA | RSSYYWG | SIYYSGSASYNPSLKS | LDSGWSFDY |
| | | | SEQ ID NO:5460 | SEQ ID NO:13472 | SEQ ID NO:21484 |
| iPS:434219 | | NA | AGCTATGCCATGAGC | GCTATTAGTGGTAGTGGT GGTAACACATTCTACGC AGACTCCGTGAAGGGC | TTTTTCGGTATAGTGGGAGC CGGGTACTTTGACTAC |
| | 21-225_60E9 | | SEQ ID NO:5461 | SEQ ID NO:13473 | SEQ ID NO:21485 |

FIGURE 49

| | | AA | SYAMS | AISGSGGNTFYADSVKG | FFGIVGAGYFDY |
|---|---|---|---|---|---|
| | | | SEQ ID NO:5462 | SEQ ID NO:13474 | SEQ ID NO:21486 |
| iPS:434221 | 21-225_60A11 | NA | AACTATGCCATGACC | GCTATTAGTGGTAGTGGT GGTAACACATTCTACGC AGACTCCGTGACGGGC | CTGGGGAAGGACTACCACTA CTACGGTATGGACGTC |
| | | | SEQ ID NO:5463 | SEQ ID NO:13475 | SEQ ID NO:21487 |
| | | AA | NYAMT | AISGSGGNTFYADSVTG | LGKDYHYYGMDV |
| | | | SEQ ID NO:5464 | SEQ ID NO:13476 | SEQ ID NO:21488 |
| iPS:434223 | 21-225_60C12 | NA | AGCTATGGCATGCAC | GTTATATGGTATGATGG AAGTAATAAATACTATG TAGACTCCGTGAAGGGC | AGGTATAGCAGAAGCTGGAC GGGAGGTATGGACGTC |
| | | | SEQ ID NO:5465 | SEQ ID NO:13477 | SEQ ID NO:21489 |
| | | AA | SYGMH | VIWYDGSNKYYVDSVKG | RYSRSWTGGMDV |
| | | | SEQ ID NO:5466 | SEQ ID NO:13478 | SEQ ID NO:21490 |
| iPS:434225 | 21-225_60E12 | NA | AGTTACTTCTGGAGC | CGCATCTATACCAGGGG GAGCACCAACTACAACC CCTCCCTCAAGAGT | GAGGGAAAAACTGGGGGGG TTTCTTACTTTGACTAC |
| | | | SEQ ID NO:5467 | SEQ ID NO:13479 | SEQ ID NO:21491 |
| | | AA | SYFWS | RIYTRGSTNYNPSLKS | EGKTGGVSYFDY |
| | | | SEQ ID NO:5468 | SEQ ID NO:13480 | SEQ ID NO:21492 |
| iPS:434227 | 21-225_61A1 | NA | AGTCACTTCTGGAGC | CGCATCTATATCAGGGG GAGCACCAACTACAACC CCTCCCTCAAGAGT | GAGGGAAAAACTGGGGGGG TTTCTTACTTTGACTAC |
| | | | SEQ ID NO:5469 | SEQ ID NO:13481 | SEQ ID NO:21493 |
| | | AA | SHFWS | RIYIRGSTNYNPSLKS | EGKTGGVSYFDY |
| | | | SEQ ID NO:5470 | SEQ ID NO:13482 | SEQ ID NO:21494 |

FIGURE 49

| iPS:434229 | | NA | GACTATGGCATGCAC | ATTATATGGTATGATGA AAGTAATAAATACTATG CAGACTCCGTGAAGGGC | GATGTTCTGGACCCTTTTGA CTAC |
|---|---|---|---|---|---|
| | 21-225_61H1 | | SEQ ID NO:5471 | SEQ ID NO:13483 | SEQ ID NO:21495 |
| | | AA | DYGMH | IIWYDESNKYYADSVKG | DVLDPFDY |
| | | | SEQ ID NO:5472 | SEQ ID NO:13484 | SEQ ID NO:21496 |
| iPS:434231 | | NA | AGCTATGGCATGCAC | GTTATATGGTATGATGG AAGTAATAAATACTATG CAGACTCCGTGAAGGGC | GAGAGGTATAGCAGTGGCTG GTACGACTACGGTATGGACG TC |
| | 21-225_61F2 | | SEQ ID NO:5473 | SEQ ID NO:13485 | SEQ ID NO:21497 |
| | | AA | SYGMH | VIWYDGSNKYYADSVKG | ERYSSGWYDYGMDV |
| | | | SEQ ID NO:5474 | SEQ ID NO:13486 | SEQ ID NO:21498 |
| iPS:434233 | | NA | AGCTATGGCATGCAC | GTTATATGGTATGATGG AAGTAATAAATACTATG CAGACTCCGTGAAGGGC | AGGTATAGCAGAAGCTGGGC GGGAGGCATGGACGTC |
| | 21-225_61B3 | | SEQ ID NO:5475 | SEQ ID NO:13487 | SEQ ID NO:21499 |
| | | AA | SYGMH | VIWYDGSNKYYADSVKG | RYSRSWAGGMDV |
| | | | SEQ ID NO:5476 | SEQ ID NO:13488 | SEQ ID NO:21500 |
| iPS:434235 | | NA | AATTATGATATCAAC | TGGATGACCCCTAACAG TGGTAACACAGGCTATG CACAGAAGTTCCAGGGC | AGCAGTGGCTGGTACCGCTT TGACTAC |
| | 21-225_61E3 | | SEQ ID NO:5477 | SEQ ID NO:13489 | SEQ ID NO:21501 |
| | | AA | NYDIN | WMTPNSGNTGYAQKFQG | SSGWYRFDY |
| | | | SEQ ID NO:5478 | SEQ ID NO:13490 | SEQ ID NO:21502 |

FIGURE 49

| iPS:434237 | | NA | AATTATGATATCAAC | TGGATGCACCCTAACAG TGGTAGCACAGGCTATG CACAGAAGTTCCAGGGC | AGCAGTGGCTGGTACTACTT TGACTAC |
|---|---|---|---|---|---|
| | 21-225_61B5 | | SEQ ID NO:5479 | SEQ ID NO:13491 | SEQ ID NO:21503 |
| | | AA | NYDIN | WMHPNSGSTGYAQKFQG | SSGWYYFDY |
| | | | SEQ ID NO:5480 | SEQ ID NO:13492 | SEQ ID NO:21504 |
| iPS:434239 | | NA | AGCTATGCCATGAGT | GCTATTAGTACTGGTGGT GGTAACACATACTACGC AGACTCCGTGAAGGGC | CGGGGGGGTCTACGGTGACTT TGATGCTTTTGATATC |
| | 21-225_58F1 | | SEQ ID NO:5481 | SEQ ID NO:13493 | SEQ ID NO:21505 |
| | | AA | SYAMS | AISTGGGNTYYADSVKG | RGVYGDFDAFDI |
| | | | SEQ ID NO:5482 | SEQ ID NO:13494 | SEQ ID NO:21506 |
| iPS:434241 | | NA | AGCTATGCCATGAGC | GCTACTAGTGGTAGTGG TGTTAACACATTCTACGC AGACTCCGTGAAGGGC | TTGGAACTGGGGATCTTTGA CTAC |
| | 21-225_61E6 | | SEQ ID NO:5483 | SEQ ID NO:13495 | SEQ ID NO:21507 |
| | | AA | SYAMS | ATSGSGVNTFYADSVKG | LELGIFDY |
| | | | SEQ ID NO:5484 | SEQ ID NO:13496 | SEQ ID NO:21508 |
| iPS:434243 | | NA | AGCTATAGCATGAAC | TCCATTAGTAGTAGTAGT AGTTACATATACTACGC AGACTCAGTGAAGGGC | TTTGGAGTGGAC |
| | 21-225_62C1 | | SEQ ID NO:5485 | SEQ ID NO:13497 | SEQ ID NO:21509 |
| | | AA | SYSMN | SISSSSSYIYYADSVKG | FGVD |
| | | | SEQ ID NO:5486 | SEQ ID NO:13498 | SEQ ID NO:21510 |
| iPS:434245 | | NA | AGCTATGGCATGCAC | ATTATATGGTATGATGG AAGTAATAAATACTATG CAGACTCCGTGAAGGGC | GAAGACCCGCGTACCAGCTG CTCTGACTAC |
| | 21-225_62H1 | | SEQ ID NO:5487 | SEQ ID NO:13499 | SEQ ID NO:21511 |

FIGURE 49

| | | | | | |
|---|---|---|---|---|---|
| | | AA | SYGMH | IIWYDGSNKYYADSVKG | EDPRTSCSDY |
| | | | SEQ ID NO:5488 | SEQ ID NO:13500 | SEQ ID NO:21512 |
| iPS:434247 | 21-225_62D2 | NA | AGCTATGGCATGCAC | GTTATATGGTATGATGG AAGTAATAAATACTCTG CAGACTCCGTGAAGGGC | GATAATGGTAACTGGAACTA CCTTGACTAC |
| | | | SEQ ID NO:5489 | SEQ ID NO:13501 | SEQ ID NO:21513 |
| | | AA | SYGMH | VIWYDGSNKYSADSVKG | DNGNWNYLDY |
| | | | SEQ ID NO:5490 | SEQ ID NO:13502 | SEQ ID NO:21514 |
| iPS:434249 | 21-225_62E2 | NA | AGAAGTAGTTACTACTGGGG C | AGCATCTATTATAGTGG GATCGCCTCCTATAATCC GTCCCTCAAGAGT | CTGAGCAGTGGCTGGTCCTT TGACTAC |
| | | | SEQ ID NO:5491 | SEQ ID NO:13503 | SEQ ID NO:21515 |
| | | AA | RSSYYWG | SIYYSGIASYNPSLKS | LSSGWSFDY |
| | | | SEQ ID NO:5492 | SEQ ID NO:13504 | SEQ ID NO:21516 |
| iPS:434251 | 21-225_62G3 | NA | AGCTATAGCATGAAC | TCCATTAGTAGTAGTAGT AGTTACATATACTACGC AGACTCAGTGAAGGGC | GTTAACTCTTTTGACTCC |
| | | | SEQ ID NO:5493 | SEQ ID NO:13505 | SEQ ID NO:21517 |
| | | AA | SYSMN | SISSSSSYIYYADSVKG | VNSFDS |
| | | | SEQ ID NO:5494 | SEQ ID NO:13506 | SEQ ID NO:21518 |
| iPS:434253 | 21-225_62E4 | NA | GACTATGGCATGCAC | GTTATATGGTATGATAG AAGTAATAAATACTATG CAGACTCCGTGAAGGGC | GAGCTTGGGTTCAGCAGTGA CTAC |
| | | | SEQ ID NO:5495 | SEQ ID NO:13507 | SEQ ID NO:21519 |
| | | AA | DYGMH | VIWYDRSNKYYADSVKG | ELGFSSDY |
| | | | SEQ ID NO:5496 | SEQ ID NO:13508 | SEQ ID NO:21520 |

FIGURE 49

| iPS:434255 | | NA | TCCTATGGCATGCAC | GCTATATGGTATGATGG AAGTAATAAATACTATG GAGACTCCGTGAAGGGC | GATCAGGGCATAGTGGGAGC TACTTGGTTTGACTAC |
|---|---|---|---|---|---|
| | 21-225_62E6 | | SEQ ID NO:5497 | SEQ ID NO:13509 | SEQ ID NO:21521 |
| | | AA | SYGMH | AIWYDGSNKYYGDSVKG | DQGIVGATWFDY |
| | | | SEQ ID NO:5498 | SEQ ID NO:13510 | SEQ ID NO:21522 |
| iPS:434257 | | NA | AGCTATGTTATGAGC | GGTATTAGTGGTAGTGG TGCTAAAACATACTATG CAGACTCCGTGAAGGGC | CTGGGGATAGACTACTACTA CGGTATGGACGTC |
| | 21-225_62F7 | | SEQ ID NO:5499 | SEQ ID NO:13511 | SEQ ID NO:21523 |
| | | AA | SYVMS | GISGSGAKTYYADSVKG | LGIDYYYGMDV |
| | | | SEQ ID NO:5500 | SEQ ID NO:13512 | SEQ ID NO:21524 |
| iPS:434259 | | NA | GGCTACTATATGCAC | TGGATCAAACCTAAAAG TGGTGGCACAAACCAAG CACAGAAGTTTCAGGGC | GCTCCGGGTATAGCAGCAGC TGGTACATGGGGGATACTTTG ACTAC |
| | 21-225_62G7 | | SEQ ID NO:5501 | SEQ ID NO:13513 | SEQ ID NO:21525 |
| | | AA | GYYMH | WIKPKSGGTNQAQKFQG | APGIAAAGTWGYFDY |
| | | | SEQ ID NO:5502 | SEQ ID NO:13514 | SEQ ID NO:21526 |
| iPS:434261 | | NA | AGCTATGTCTTAAAC | GCTATGAGTGGTAGTGG TGGTAGAACATACTACG CAGACTCCGTGAAGGGC | ACTACGCACTTTGACTAC |
| | 21-225_56F7 | | SEQ ID NO:5503 | SEQ ID NO:13515 | SEQ ID NO:21527 |
| | | AA | SYVLN | AMSGSGGRTYYADSVKG | TTHFDY |
| | | | SEQ ID NO:5504 | SEQ ID NO:13516 | SEQ ID NO:21528 |
| iPS:434263 | 21 225 56H7 | NA | AGCTATAGCATGAAC | TCCATTAGTAGTAGTAGT ACTTACATATACTACGG AGACTCAGTGAAGGGC | GATCGGGGCAGCAGC |

FIGURE 49

| | | | | | |
|---|---|---|---|---|---|
| | **21-225_56H7** | | | SEQ ID NO:5505 | SEQ ID NO:13517 | SEQ ID NO:21529 |
| | | AA | SYSMN | SISSSSTYIYYGDSVKG | DRGSS |
| iPS:434265 | | NA | SEQ ID NO:5506 | SEQ ID NO:13518 | SEQ ID NO:21530 |
| | **21-225_57B2** | | AGCTATAGCATGAAC | TCCATTAGTGGTAGTAGTAGTTACATAAACTACACAGACTCAGTGAAGGGC | GTGGCTGGCTTTGACTAC |
| | | | SEQ ID NO:5507 | SEQ ID NO:13519 | SEQ ID NO:21531 |
| | | AA | SYSMN | SISGSSSYINYTDSVKG | VAGFDY |
| iPS:434267 | | NA | SEQ ID NO:5508 | SEQ ID NO:13520 | SEQ ID NO:21532 |
| | **21-225_57F2** | | AGTTACTACTGGAGC | CGCATCTATACCAGGGGGAGCACCAACTACAACCCCTCCCTCAAGAGT | GAGGGAAAAACTGGGGGGGTTTCTTACTTTGACTAC |
| | | | SEQ ID NO:5509 | SEQ ID NO:13521 | SEQ ID NO:21533 |
| | | AA | SYYWS | RIYTRGSTNYNPSLKS | EGKTGGVSYFDY |
| iPS:434269 | | NA | SEQ ID NO:5510 | SEQ ID NO:13522 | SEQ ID NO:21534 |
| | **21-225_57H3** | | AGCTATGGCATGCAC | GCTATATGGTATGATGGAAGTAATAAATACTATGCAGACTCCGTGAAGGGC | GATTATGGTATAGTGGGAGCTACATATTTTGACTAC |
| | | | SEQ ID NO:5511 | SEQ ID NO:13523 | SEQ ID NO:21535 |
| | | AA | SYGMH | AIWYDGSNKYYADSVKG | DYGIVGATYFDY |
| iPS:434271 | | NA | SEQ ID NO:5512 | SEQ ID NO:13524 | SEQ ID NO:21536 |
| | **21-225_57A4** | | GACTATGGCATGCAC | GTTATATGGTATGCTGGAAGTAATAAATACTATGTAGACTCCGTGAAGGGC | GAACTGGGGATGAGGTCTGACTAC |
| | | | SEQ ID NO:5513 | SEQ ID NO:13525 | SEQ ID NO:21537 |
| | | AA | DYGMH | VIWYAGSNKYYVDSVKG | ELGMRSDY |

FIGURE 49

| | | | SEQ ID NO:5514 | SEQ ID NO:13526 | SEQ ID NO:21538 |
|---|---|---|---|---|---|
| iPS:434273 | 21-225_57E4 | NA | AGCTATGCCATGAGC | GTTATTAGTGGTAGTGGT GGTAGTACATTCTACGC AGACTCCGTGAAGGGC | AGGGACTGGAACGACGTTTT TGACTAC |
| | | | SEQ ID NO:5515 | SEQ ID NO:13527 | SEQ ID NO:21539 |
| | | AA | SYAMS | VISGSGGSTFYADSVKG | RDWNDVFDY |
| | | | SEQ ID NO:5516 | SEQ ID NO:13528 | SEQ ID NO:21540 |
| iPS:434275 | 21-225_57F4 | NA | GACTACTACATGAAC | TACATTAGTAGTAGTGGT AGTACCATATACTACGC AGACTCTGTGAAGGGC | GATATGATTACG |
| | | | SEQ ID NO:5517 | SEQ ID NO:13529 | SEQ ID NO:21541 |
| | | AA | DYYMN | YISSSGSTIYYADSVKG | DMIT |
| | | | SEQ ID NO:5518 | SEQ ID NO:13530 | SEQ ID NO:21542 |
| iPS:434277 | 21-225_57A7 | NA | GGCTACCATATACAC | TGGATCAACCCTAACAA TAATGGCACAAACTATG CACAGAAGTTTCAGGGC | GATGGGAGAAGTGGTTTTGA CTAC |
| | | | SEQ ID NO:5519 | SEQ ID NO:13531 | SEQ ID NO:21543 |
| | | AA | GYHIH | WINPNNNGTNYAQKFQG | DGRSGFDY |
| | | | SEQ ID NO:5520 | SEQ ID NO:13532 | SEQ ID NO:21544 |
| iPS:434279 | 21-225_57F7 | NA | AGCTATGCCATGAGC | GCTATTAGTGGTAGTGGT GGTAACACATTCTACGC AGACTCCGTGAAGGGC | TTTTTCGGTGTAGTGGGAGT CGGGTGCTTTGACTAC |
| | | | SEQ ID NO:5521 | SEQ ID NO:13533 | SEQ ID NO:21545 |
| | | AA | SYAMS | AISGSGGNTFYADSVKG | FFGVVGVGCFDY |
| | | | SEQ ID NO:5522 | SEQ ID NO:13534 | SEQ ID NO:21546 |
| iPS:434281 | 21 225 57B8 | NA | AGCTATGCCATGAGC | GCTATTAGTGGTAGTGGT GGTAACACATTCTACGC AGACTCCGTGAAGGGC | TTGGAACTGGGGATCTTTGA CTAC |

FIGURE 49

| | 21-225_57B8 | | SEQ ID NO:5523 | SEQ ID NO:13535 | SEQ ID NO:21547 |
|---|---|---|---|---|---|
| | | AA | SYAMS | AISGSGGNTFYADSVKG | LELGIFDY |
| | | | SEQ ID NO:5524 | SEQ ID NO:13536 | SEQ ID NO:21548 |
| iPS:434283 | 21-225_57F8 | NA | AACTATGCCATGAGC | GCTAGCAGTGGTAGTGG TGGTAACACATTCTACGC AGACTCCGTGACGGGC | CTGGGGAAGGACTACCACTA CTACGGTATGGACGTC |
| | | | SEQ ID NO:5525 | SEQ ID NO:13537 | SEQ ID NO:21549 |
| | | AA | NYAMS | ASSGSGGNTFYADSVTG | LGKDYHYYGMDV |
| | | | SEQ ID NO:5526 | SEQ ID NO:13538 | SEQ ID NO:21550 |
| iPS:434285 | 21-225_57A11 | NA | AATTATGATATCAAC | TGGATGAACCCTAACAG TGTTAACACAGGCTATG CACAGAAGTTCCAGGGC | AGCAGTGGCTGGAACTGGTT CGACCCC |
| | | | SEQ ID NO:5527 | SEQ ID NO:13539 | SEQ ID NO:21551 |
| | | AA | NYDIN | WMNPNSVNTGYAQKFQG | SSGWNWFDP |
| | | | SEQ ID NO:5528 | SEQ ID NO:13540 | SEQ ID NO:21552 |
| iPS:434287 | 21-225_57F12 | NA | AATTATGATATCAAC | TGGATGAACCCTAACAG TGGTAACACAGGCTATG CACAGAAGTTCCAGGGC | AGCAGTGGCTGGTACCGGTT CGACCCC |
| | | | SEQ ID NO:5529 | SEQ ID NO:13541 | SEQ ID NO:21553 |
| | | AA | NYDIN | WMNPNSGNTGYAQKFQG | SSGWYRFDP |
| | | | SEQ ID NO:5530 | SEQ ID NO:13542 | SEQ ID NO:21554 |
| iPS:434289 | 21-225_57H12 | NA | AGCTACGCCATGAGC | GCTATTAGTGGTAGTGGT GGTAACACATTCTACGG AGACTCCGTGAAGGGC | TTTTTCGGTATAGTGGGTGC CGGGTACTTTGACTAC |
| | | | SEQ ID NO:5531 | SEQ ID NO:13543 | SEQ ID NO:21555 |
| | | AA | SYAMS | AISGSGGNTFYGDSVKG | FFGIVGAGYFDY |
| | | | SEQ ID NO:5532 | SEQ ID NO:13544 | SEQ ID NO:21556 |

FIGURE 49

| iPS:434291 | | NA | AGCTACGCCATGAGC | GCTATTAGTGGTAGTGGT GGTAACACATTCTACGG AGACTCCGTGAAGGGC | TTTTTCGGTATAGTGGGAGC CGGGTTCTTTGACTCC |
|---|---|---|---|---|---|
| | 21-225_58A4 | | SEQ ID NO:5533 | SEQ ID NO:13545 | SEQ ID NO:21557 |
| | | AA | SYAMS | AISGSGGNTFYGDSVKG | FFGIVGAGFFDS |
| | | | SEQ ID NO:5534 | SEQ ID NO:13546 | SEQ ID NO:21558 |
| iPS:434293 | | NA | GACTATGGCATGCAC | GTTATATGGTATGCTGGA AGTAATAAATACCATGT AGACTCCGTGAAGGGC | GAACTGGGGATGAGGTCTGA CTAC |
| | 21-225_58F5 | | SEQ ID NO:5535 | SEQ ID NO:13547 | SEQ ID NO:21559 |
| | | AA | DYGMH | VIWYAGSNKYHVDSVKG | ELGMRSDY |
| | | | SEQ ID NO:5536 | SEQ ID NO:13548 | SEQ ID NO:21560 |
| iPS:434295 | | NA | AATTATGATATCAAC | TGGATGAACCCTAACAG TGGTAGCACAGGCTATG CACAGAAGTTCCAGGGC | AGCAGTGGCTGGTACTACTT TGACTAC |
| | 21-225_58B9 | | SEQ ID NO:5537 | SEQ ID NO:13549 | SEQ ID NO:21561 |
| | | AA | NYDIN | WMNPNSGSTGYAQKFQG | SSGWYYFDY |
| | | | SEQ ID NO:5538 | SEQ ID NO:13550 | SEQ ID NO:21562 |
| iPS:434297 | | NA | AGCTATGCCATGAGC | GCTATTAGTGGTAGTGGT GGTAACACATTCTACGC AGACTCCGTGAAGGGC | TTTTTCGGTATAGTGGGAGC CGGGTACTTTGACTAC |
| | 21-225_58A10 | | SEQ ID NO:5539 | SEQ ID NO:13551 | SEQ ID NO:21563 |
| | | AA | SYAMS | AISGSGGNTFYADSVKG | FFGIVGAGYFDY |
| | | | SEQ ID NO:5540 | SEQ ID NO:13552 | SEQ ID NO:21564 |

EP 4 435 105 A2

FIGURE 49

| iPS:434299 | | NA | GACTATGACATACAC | GTTATATGGTATGATGG AAGTAAAAAATATTATG CAGACTCCGTGAAGGGC | GATCGGGTCACTTTTGACTA C |
|---|---|---|---|---|---|
| | 21-225_58D11 | | SEQ ID NO:5541 | SEQ ID NO:13553 | SEQ ID NO:21565 |
| | | AA | DYDIH | VIWYDGSKKYYADSVKG | DRVTFDY |
| | | | SEQ ID NO:5542 | SEQ ID NO:13554 | SEQ ID NO:21566 |
| iPS:434301 | | NA | AGCTATGCCATGAGC | GCTATTAGTGGTAGTGGT GGTAACACATTCTACGC AGACTCCGTGAAGGGC | TTTTTCGGTATGGTGGGAGC CGGATTCTTTGACTAC |
| | 21-225_58F11 | | SEQ ID NO:5543 | SEQ ID NO:13555 | SEQ ID NO:21567 |
| | | AA | SYAMS | AISGSGGNTFYADSVKG | FFGMVGAGFFDY |
| | | | SEQ ID NO:5544 | SEQ ID NO:13556 | SEQ ID NO:21568 |
| iPS:434303 | | NA | AGCTATGGCATGCAC | GTTATATGGTATGATGG AAGTAATAAATACCATG CAGACTCCGTGAAGGGC | CGGTATAGCAGCAGCTGGGA CGGGGGTATGGACGTC |
| | 21-225_58H11 | | SEQ ID NO:5545 | SEQ ID NO:13557 | SEQ ID NO:21569 |
| | | AA | SYGMH | VIWYDGSNKYHADSVKG | RYSSSWDGGMDV |
| | | | SEQ ID NO:5546 | SEQ ID NO:13558 | SEQ ID NO:21570 |
| iPS:434305 | | NA | AATTATGATATCAAC | TGGATGACTCCTAACAG TGGTAACACAGGCTATG CACAGAAGTTCCAGGGC | AGCAGTGGCTGGTACTTCTT TGACTAC |
| | 21-225_59E1 | | SEQ ID NO:5547 | SEQ ID NO:13559 | SEQ ID NO:21571 |
| | | AA | NYDIN | WMTPNSGNTGYAQKFQG | SSGWYFFDY |
| | | | SEQ ID NO:5548 | SEQ ID NO:13560 | SEQ ID NO:21572 |

1102

FIGURE 49

| iPS:434307 | | NA | GGCTACTATATACAC | TGGATCAACCCTAACAG TGGTGGCACAAACTATG CACAGAAGTTTCAGGGC | GATCCGGGGCCCTTTGACTA C |
| --- | --- | --- | --- | --- | --- |
| | 21-225_59B2 | | SEQ ID NO:5549 | SEQ ID NO:13561 | SEQ ID NO:21573 |
| | | AA | GYYIH | WINPNSGGTNYAQKFQG | DPGPFDY |
| | | | SEQ ID NO:5550 | SEQ ID NO:13562 | SEQ ID NO:21574 |
| iPS:434309 | | NA | AGCTATGCCATGAAC | GCTATTAGTGGTAGTGGT GGTAACACATTCTACGC AGACTCCGTGAAGGGC | AGGGGGGGTCTACGGTGACTA CGAGGCTTTTGATATC |
| | 21-225_59B5 | | SEQ ID NO:5551 | SEQ ID NO:13563 | SEQ ID NO:21575 |
| | | AA | SYAMN | AISGSGGNTFYADSVKG | RGVYGDYEAFDI |
| | | | SEQ ID NO:5552 | SEQ ID NO:13564 | SEQ ID NO:21576 |
| iPS:434311 | | NA | AGCTATGGCATGCAC | GTTATATGGTATGATGG AAGTAATAAATACTATG CAGACTCCGTGAAGGGC | GAGAGGGGTATAGCAGTGGG GTACTACGGTATGGACGTC |
| | 21-225_59H5 | | SEQ ID NO:5553 | SEQ ID NO:13565 | SEQ ID NO:21577 |
| | | AA | SYGMH | VIWYDGSNKYYADSVKG | ERGIAVGYYGMDV |
| | | | SEQ ID NO:5554 | SEQ ID NO:13566 | SEQ ID NO:21578 |
| iPS:434313 | | NA | AGAAGTAGTTACTACTGGGG C | AATATCTATTATAGTGGG AGCACCTACTACAACCC GTCCCTCAAGAGT | CATAGCAGCAGCTGGTCCCT TGACTAC |
| | 21-225_59E6 | | SEQ ID NO:5555 | SEQ ID NO:13567 | SEQ ID NO:21579 |
| | | AA | RSSYYWG | NIYYSGSTYYNPSLKS | HSSSWSLDY |
| | | | SEQ ID NO:5556 | SEQ ID NO:13568 | SEQ ID NO:21580 |
| iPS:434315 | | NA | GGCCACTATATACAC | TGGATCAACCCGAACAG TGGTGGCACAAACTATG TACAGAAATTTCAGGGC | GGGGGCCTACTGGGAGCTAC CAACTACTACTACTACGGTA TGGACGTC |
| | 21-225_59G7 | | | | |

FIGURE 49

| | | | | | |
|---|---|---|---|---|---|
| | | | SEQ ID NO:5557 | SEQ ID NO:13569 | SEQ ID NO:21581 |
| | | AA | GHYIH | WINPNSGGTNYVQKFQG | GGLLGATNYYYYGMDV |
| | | | SEQ ID NO:5558 | SEQ ID NO:13570 | SEQ ID NO:21582 |
| iPS:434317 | 21-225_59E8 | NA | AGCTATAGCATGAAT | TACATTAGTAGTAGTAGT GGGACCATATACTACGC AGACTCTGTGAAGGGC | GAATGGGGAATGGCAGTGGC TGGCCCGTTTGACTAC |
| | | | SEQ ID NO:5559 | SEQ ID NO:13571 | SEQ ID NO:21583 |
| | | AA | SYSMN | YISSSSGTIYYADSVKG | EWGMAVAGPFDY |
| | | | SEQ ID NO:5560 | SEQ ID NO:13572 | SEQ ID NO:21584 |
| iPS:434319 | 21-225_59B9 | NA | GGCAATTATATACAC | TGGATCAACCCTAACAG TGGTGGCACAAACTATG TACAGAAGTTTCAGGGC | GGGGGCCTACTGGGAGCTAC CTACTACTACTACTACGGTA TGGACGTC |
| | | | SEQ ID NO:5561 | SEQ ID NO:13573 | SEQ ID NO:21585 |
| | | AA | GNYIH | WINPNSGGTNYVQKFQG | GGLLGATYYYYYGMDV |
| | | | SEQ ID NO:5562 | SEQ ID NO:13574 | SEQ ID NO:21586 |
| iPS:434321 | 21-225_59F10 | NA | AATTATGATATCAAC | TGGATGAACCCTAACAG TGGTAACACAGGCTATG CACAGAAGTTCCAGGGC | AGCAGTGGCTGGTACTACTT TGACTAC |
| | | | SEQ ID NO:5563 | SEQ ID NO:13575 | SEQ ID NO:21587 |
| | | AA | NYDIN | WMNPNSGNTGYAQKFQG | SSGWYYFDY |
| | | | SEQ ID NO:5564 | SEQ ID NO:13576 | SEQ ID NO:21588 |
| iPS:434323 | 21-225_62H8 | NA | ACCTATGGCATGCAC | GTTATATGGCATGATGG AAGTGATAAATATTATG TAGACTCCGTGAAGGGC | GAAGACCCGCGTACCAGCTG CTCTGACTAC |
| | | | SEQ ID NO:5565 | SEQ ID NO:13577 | SEQ ID NO:21589 |
| | | AA | TYGMH | VIWHDGSDKYYVDSVKG | EDPRTSCSDY |
| | | | SEQ ID NO:5566 | SEQ ID NO:13578 | SEQ ID NO:21590 |

FIGURE 49

| iPS:434327 | | NA | AGCTATGGCATGCAC | GTTATATGGTATGATGG AAGTAATAAATACTATG CAGACTCCGTGAAGGGC | GATAGCCTCTCGGGTATAGC AGCAGCTTTTGACTAC |
|---|---|---|---|---|---|
| | 21-225_63G6 | | SEQ ID NO:5567 | SEQ ID NO:13579 | SEQ ID NO:21591 |
| | | AA | SYGMH | VIWYDGSNKYYADSVKG | DSLSGIAAAFDY |
| | | | SEQ ID NO:5568 | SEQ ID NO:13580 | SEQ ID NO:21592 |
| iPS:434331 | | NA | AGCTATAACATGAAC | TCCATTAGTGGTAGTAGC ACTTACATGAACTACAC AGACTCAGTGAAGGGC | CTACGTAATTTTGACTAC |
| | 21-225_63H8 | | SEQ ID NO:5569 | SEQ ID NO:13581 | SEQ ID NO:21593 |
| | | AA | SYNMN | SISGSSTYMNYTDSVKG | LRNFDY |
| | | | SEQ ID NO:5570 | SEQ ID NO:13582 | SEQ ID NO:21594 |
| iPS:434333 | | NA | GGCTACTATATGCAC | TGGATCAACCCTAACAG TGGTGGCACAAACTTTG CACAGAAGTTTCAGGGC | GCTCCGGGTGTAGCAGCAGC TGGTTCATGGGGATACTTTG ACTAC |
| | 21-225_63C9 | | SEQ ID NO:5571 | SEQ ID NO:13583 | SEQ ID NO:21595 |
| | | AA | GYYMH | WINPNSGGTNFAQKFQG | APGVAAAGSWGYFDY |
| | | | SEQ ID NO:5572 | SEQ ID NO:13584 | SEQ ID NO:21596 |
| iPS:434335 | | NA | AGCTATGGCATGCAC | GTCATATGGTATGATGG AAGTAATAAATACTATG CAGACTCCGTGAAGGGC | GATGATCCCAGATCCTCGGC CGGGGACTAC |
| | 21-225_63C10 | | SEQ ID NO:5573 | SEQ ID NO:13585 | SEQ ID NO:21597 |
| | | AA | SYGMH | VIWYDGSNKYYADSVKG | DDPRSSAGDY |
| | | | SEQ ID NO:5574 | SEQ ID NO:13586 | SEQ ID NO:21598 |

FIGURE 49

| iPS:434337 | | NA | AGCTATGGCATGCAC | GTTATATGGTTTGATGAA ACTAATAAATACTATGG AGACTCCGTGAAGGGC | GAGCTTGGGTTCAGCAGTGA CTAT |
|---|---|---|---|---|---|
| | 21-225_64E1 | | SEQ ID NO:5575 | SEQ ID NO:13587 | SEQ ID NO:21599 |
| | | AA | SYGMH | VIWFDETNKYYGDSVKG | ELGFSSDY |
| | | | SEQ ID NO:5576 | SEQ ID NO:13588 | SEQ ID NO:21600 |
| iPS:434339 | | NA | GATTATGTCATGCAC | GTCATATGGTATGATGG AAGTAATAAATACTATG CAGACTCCGTGAAGGGC | GAAAGGTATAGCAGCAGCTG GTACGACTACGGTATGGACG TC |
| | 21-225_64A4 | | SEQ ID NO:5577 | SEQ ID NO:13589 | SEQ ID NO:21601 |
| | | AA | DYVMH | VIWYDGSNKYYADSVKG | ERYSSSWYDYGMDV |
| | | | SEQ ID NO:5578 | SEQ ID NO:13590 | SEQ ID NO:21602 |
| iPS:434341 | | NA | AGTTACTTCTGGAGC | CGTATCTATACCAGTGG GATCTCCAACTACAATCC CTCCCTCAAGAGT | TTTAGCAGTGGCTTTTTTGAC TAC |
| | 21-225_64F7 | | SEQ ID NO:5579 | SEQ ID NO:13591 | SEQ ID NO:21603 |
| | | AA | SYFWS | RIYTSGISNYNPSLKS | FSSGFFDY |
| | | | SEQ ID NO:5580 | SEQ ID NO:13592 | SEQ ID NO:21604 |
| iPS:434343 | | NA | GACTATGTCATGCAC | GTTATATGGTATGATGG AAGTAATAAATACTATG CAGACTCCATGAAGGGC | GAACGGTATAGCAGTGGCTG GTACGACTACGGTATGGACG TC |
| | 21-225_64C8 | | SEQ ID NO:5581 | SEQ ID NO:13593 | SEQ ID NO:21605 |
| | | AA | DYVMH | VIWYDGSNKYYADSMKG | ERYSSGWYDYGMDV |
| | | | SEQ ID NO:5582 | SEQ ID NO:13594 | SEQ ID NO:21606 |

FIGURE 49

| iPS:434345 | | NA | ACCTATGGCATGCAC | ATTATATGGTATGATGG AAGTAATAAATACTATG CAGACTCCGTGAAGGGC | GATACATACGATTTTTGGAG TGGTTATTTGGGCTAC |
|---|---|---|---|---|---|
| | 21-225_64H9 | | SEQ ID NO:5583 | SEQ ID NO:13595 | SEQ ID NO:21607 |
| | | AA | TYGMH | IIWYDGSNKYYADSVKG | DTYDFWSGYLGY |
| | | | SEQ ID NO:5584 | SEQ ID NO:13596 | SEQ ID NO:21608 |
| iPS:434347 | | NA | GGCTACTATATGCAC | TGGATCAAACCAAACAG TGGTGGCACAAACCAAG CACAGAAGTTTCAGGGC | GCTCCGGGTACTGCAGCAAC TGGTACATGGGGATACTTTG ACTAC |
| | 21-225_64H10 | | SEQ ID NO:5585 | SEQ ID NO:13597 | SEQ ID NO:21609 |
| | | AA | GYYMH | WIKPNSGGTNQAQKFQG | APGTAATGTWGYFDY |
| | | | SEQ ID NO:5586 | SEQ ID NO:13598 | SEQ ID NO:21610 |
| iPS:434351 | | NA | AGCTATGGCATGCAC | GTTATATGGTATGATGA AAGTAATAAATACTATG TAGACTCCGTGAAGGGC | GAACTCGGGTTCCTCTCTGA CCAC |
| | 21-225_64A12 | | SEQ ID NO:5587 | SEQ ID NO:13599 | SEQ ID NO:21611 |
| | | AA | SYGMH | VIWYDESNKYYVDSVKG | ELGFLSDH |
| | | | SEQ ID NO:5588 | SEQ ID NO:13600 | SEQ ID NO:21612 |
| iPS:434353 | | NA | AGAAGTAGTTACTACTGGGG C | AGTATCTATTACAGTGG GAGCACCTCCTACAACC CGTCCCTCAAGAGT | CTGGACAGTGGCTGGTCCTT TGACTAC |
| | 21-225_64B12 | | SEQ ID NO:5589 | SEQ ID NO:13601 | SEQ ID NO:21613 |
| | | AA | RSSYYWG | SIYYSGSTSYNPSLKS | LDSGWSFDY |
| | | | SEQ ID NO:5590 | SEQ ID NO:13602 | SEQ ID NO:21614 |

FIGURE 49

| iPS:434355 | | NA | AGTAATGCCATGAGC | GTTATTAGTGGTAGTGGT GGTAGCACATACTACGC AGACTCCGTGAAGGGC | AGGAACTACGACGATGCTTT TGATATC |
|---|---|---|---|---|---|
| | 21-225_64G12 | | SEQ ID NO:5591 | SEQ ID NO:13603 | SEQ ID NO:21615 |
| | | AA | SNAMS | VISGSGGSTYYADSVKG | RNYDDAFDI |
| | | | SEQ ID NO:5592 | SEQ ID NO:13604 | SEQ ID NO:21616 |
| iPS:434357 | | NA | GACTATGGCATGCAC | GTGATATGGTTTGAGGG AAGTAATAAACACTATA CAGACTCCGTGAAGGGC | GAACTTGGGTTCAGCAGTGA CTAC |
| | 21-225_65C1 | | SEQ ID NO:5593 | SEQ ID NO:13605 | SEQ ID NO:21617 |
| | | AA | DYGMH | VIWFEGSNKHYTDSVKG | ELGFSSDY |
| | | | SEQ ID NO:5594 | SEQ ID NO:13606 | SEQ ID NO:21618 |
| iPS:434359 | | NA | GGCTACTATATACAC | TGGATCAACCCTAACAG TGGTGGCACAAACTCTG CACAGAAGTTTCAGGGC | GCTCCGGGTAAAGCAGCAGC TGGTACATGGGGATACTTTG ACTAC |
| | 21-225_65G3 | | SEQ ID NO:5595 | SEQ ID NO:13607 | SEQ ID NO:21619 |
| | | AA | GYYIH | WINPNSGGTNSAQKFQG | APGKAAAGTWGYFDY |
| | | | SEQ ID NO:5596 | SEQ ID NO:13608 | SEQ ID NO:21620 |
| iPS:434361 | | NA | AGCTATGGTATCAGT | TGGATCAGCGCTTACAG TGGTAACACAAACTATG CACAGAAGCTCCAGGGC | GGGGAAGCAGTGGCTGTCTT CGACCCC |
| | 21-225_65D5 | | SEQ ID NO:5597 | SEQ ID NO:13609 | SEQ ID NO:21621 |
| | | AA | SYGIS | WISAYSGNTNYAQKLQG | GEAVAVFDP |
| | | | SEQ ID NO:5598 | SEQ ID NO:13610 | SEQ ID NO:21622 |
| iPS:434363 | 21 225 65A6 | NA | AACTATGGCATGCAC | GTTATATGGTATGATGG AAGTAATAAATACTATG GAGACTCCGTGAAGGGC | GATCAGGGCATAGTGGGAGC TACTTGGTTTGACTAC |

FIGURE 49

|  | 21-225_65A6 |  | SEQ ID NO:5599 | SEQ ID NO:13611 | SEQ ID NO:21623 |
|---|---|---|---|---|---|
|  |  | AA | NYGMH | VIWYDGSNKYYGDSVKG | DQGIVGATWFDY |
|  |  |  | SEQ ID NO:5600 | SEQ ID NO:13612 | SEQ ID NO:21624 |
| iPS:434367 | 21-225_65H11 | NA | AGCTATAGGATGAAC | TCCATTAGTAGTAGTAAT AGTTCCATATACTACGCA GACTCAGTGAAGGGC | ACAAGTGGGAGC |
|  |  |  | SEQ ID NO:5601 | SEQ ID NO:13613 | SEQ ID NO:21625 |
|  |  | AA | SYRMN | SISSSNSSIYYADSVKG | TSGS |
|  |  |  | SEQ ID NO:5602 | SEQ ID NO:13614 | SEQ ID NO:21626 |
| iPS:434369 | 21-225_66B1 | NA | GGCTACTATATGCAC | TGGATCAACCCTAACAG TGGTGGCACAAACAATG CACAGAAGTTTCAGGGC | GCTCCGGGTACAGCAGCAGC TGGTACATGGGGATACTTTG ACTAC |
|  |  |  | SEQ ID NO:5603 | SEQ ID NO:13615 | SEQ ID NO:21627 |
|  |  | AA | GYYMH | WINPNSGGTNNAQKFQG | APGTAAAGTWGYFDY |
|  |  |  | SEQ ID NO:5604 | SEQ ID NO:13616 | SEQ ID NO:21628 |
| iPS:434373 | 21-225_66A7 | NA | GGCTACTATATGCAC | TGGATCAAACCAAACAG TGGTGGCACAAACCAAG CACAGAAGTTCCAGGGC | GCTCCGGGCACAGTAGCAGC TGGTACATGGGGATACTTTG ACTAT |
|  |  |  | SEQ ID NO:5605 | SEQ ID NO:13617 | SEQ ID NO:21629 |
|  |  | AA | GYYMH | WIKPNSGGTNQAQKFQG | APGTVAAGTWGYFDY |
|  |  |  | SEQ ID NO:5606 | SEQ ID NO:13618 | SEQ ID NO:21630 |
| iPS:434375 | 21-225_66C7 | NA | GACTATGGCATGCAC | GTTATATGGTTTGAGGG AAGTCATAAATACTATA CAGACTCCGTGAAGGGC | GAACTTGGGTTCAGCAGTGA CTAC |
|  |  |  | SEQ ID NO:5607 | SEQ ID NO:13619 | SEQ ID NO:21631 |
|  |  | AA | DYGMH | VIWFEGSHKYYTDSVKG | ELGFSSDY |
|  |  |  | SEQ ID NO:5608 | SEQ ID NO:13620 | SEQ ID NO:21632 |

FIGURE 49

| iPS:434379 | | NA | AGCTATGGCATGCAC | GTTATATGGCATGATGG AAGTGATAAATACTATG CAGACTCCGTGAAGGGC | GAAGACCCGCGTACCAGTTG TTCTGACTAC |
|---|---|---|---|---|---|
| | 21-225_66A9 | | SEQ ID NO:5609 | SEQ ID NO:13621 | SEQ ID NO:21633 |
| | | AA | SYGMH | VIWHDGSDKYYADSVKG | EDPRTSCSDY |
| | | | SEQ ID NO:5610 | SEQ ID NO:13622 | SEQ ID NO:21634 |
| iPS:434383 | | NA | AGCTATAGCATGAAC | TCCATTAGTGGTACTAGT AGTTACATATACTACGC AGACTCAGTGAAGGGC | ACCAATGCTTTTGATATC |
| | 21-225_66F9 | | SEQ ID NO:5611 | SEQ ID NO:13623 | SEQ ID NO:21635 |
| | | AA | SYSMN | SISGTSSYIYYADSVKG | TNAFDI |
| | | | SEQ ID NO:5612 | SEQ ID NO:13624 | SEQ ID NO:21636 |
| iPS:434385 | | NA | AGCTATGTTATGAGC | GGTATTAGTGGTAGTGG TGCTAGAACATACTACG CAGACTCCGTGAAGGGC | CTGGGGATAGACTACTACTA CGGTATGGACGTC |
| | 21-225_66C10 | | SEQ ID NO:5613 | SEQ ID NO:13625 | SEQ ID NO:21637 |
| | | AA | SYVMS | GISGSGARTYYADSVKG | LGIDYYYGMDV |
| | | | SEQ ID NO:5614 | SEQ ID NO:13626 | SEQ ID NO:21638 |
| iPS:434387 | | NA | AGTTATGGCATGCAC | GTTATATGGTATGATGG AAGTAATAAATACTATG CAGACTCCGTGAAGGGC | GAGATGTATAGCAGCAACTG GTACGACTACGGTTTGGACG TC |
| | 21-225_66D11 | | SEQ ID NO:5615 | SEQ ID NO:13627 | SEQ ID NO:21639 |
| | | AA | SYGMH | VIWYDGSNKYYADSVKG | EMYSSNWYDYGLDV |
| | | | SEQ ID NO:5616 | SEQ ID NO:13628 | SEQ ID NO:21640 |

FIGURE 49

| iPS:434389 | | NA | GGCTACCATATGCAC | TGGATCAACCCTAACAA TGGTGGCACACACTATG CACAGAAGTTTCAGGAC | GATAGTAGAAGTTCGTGGGA CTAC |
|---|---|---|---|---|---|
| | 21-225_66F11 | | SEQ ID NO:5617 | SEQ ID NO:13629 | SEQ ID NO:21641 |
| | | AA | GYHMH | WINPNNGGTHYAQKFQD | DSRSSWDY |
| | | | SEQ ID NO:5618 | SEQ ID NO:13630 | SEQ ID NO:21642 |
| iPS:434393 | | NA | AGTTATGGCATGCAC | GCTATATGGTATGATGG AAGTAATAAATATTATG GAGACTCCGTGAAGGGC | GATCAGGGCATAGTGGGAGC TACTTGGTTTGACTAC |
| | 21-225_67C3 | | SEQ ID NO:5619 | SEQ ID NO:13631 | SEQ ID NO:21643 |
| | | AA | SYGMH | AIWYDGSNKYYGDSVKG | DQGIVGATWFDY |
| | | | SEQ ID NO:5620 | SEQ ID NO:13632 | SEQ ID NO:21644 |
| iPS:434397 | | NA | GGCTACTATATGCAC | TGGATCAAACCAAACAG TGGTGGCACAAACCAAG CACAGAAGTTTCAGGGC | GCTCCGGGTACTGCAGCAAC TGGTACATGGGGGATACTTTG ACTAC |
| | 21-225_67H4 | | SEQ ID NO:5621 | SEQ ID NO:13633 | SEQ ID NO:21645 |
| | | AA | GYYMH | WIKPNSGGTNQAQKFQG | APGTAATGTWGYFDY |
| | | | SEQ ID NO:5622 | SEQ ID NO:13634 | SEQ ID NO:21646 |
| iPS:434399 | | NA | AACTATGGCATGCAC | GTTATATTATATGATGGA AGTAAGAAATACTATGC AGACTCCGTGAAGGGC | AGTATCCCGGAATTTGACTA T |
| | 21-225_67B7 | | SEQ ID NO:5623 | SEQ ID NO:13635 | SEQ ID NO:21647 |
| | | AA | NYGMH | VILYDGSKKYYADSVKG | SIPEFDY |
| | | | SEQ ID NO:5624 | SEQ ID NO:13636 | SEQ ID NO:21648 |

FIGURE 49

| iPS:434405 | | NA | AGCTTTGGCATGAAC | TACATTAGTAGAAGTAG TAGTCACATATACTACGC AGACTCAGTGAAGGGC | TCTAGTGGGAGCCCCTTTGA CTAC |
|---|---|---|---|---|---|
| | 21-225_68E6 | | SEQ ID NO:5625 | SEQ ID NO:13637 | SEQ ID NO:21649 |
| | | AA | SFGMN | YISRSSSHIYYADSVKG | SSGSPFDY |
| | | | SEQ ID NO:5626 | SEQ ID NO:13638 | SEQ ID NO:21650 |
| iPS:434407 | | NA | AGCTATAGCATGAAC | TCCATTAGTGGTAGTAGT AGTTACATATATTACGCA GACTCAGTGATGGGC | GTCAACAGCTTTGACTCC |
| | 21-225_68G8 | | SEQ ID NO:5627 | SEQ ID NO:13639 | SEQ ID NO:21651 |
| | | AA | SYSMN | SISGSSSYIYYADSVMG | VNSFDS |
| | | | SEQ ID NO:5628 | SEQ ID NO:13640 | SEQ ID NO:21652 |
| iPS:434411 | | NA | GACTATGGCATGCAC | GTTATATGGTATGATGTA AGTAATAAATACTATGC AGACTCCGTGAAGGGC | GAACTGGGGATGACCTCTGA CTGC |
| | 21-225_68F11 | | SEQ ID NO:5629 | SEQ ID NO:13641 | SEQ ID NO:21653 |
| | | AA | DYGMH | VIWYDVSNKYYADSVKG | ELGMTSDC |
| | | | SEQ ID NO:5630 | SEQ ID NO:13642 | SEQ ID NO:21654 |
| iPS:434413 | | NA | AGAAGTAGTTACTACTGGGG C | AATATCTATTATAGTGGG AGCACCTATTACATCCCG TCCCTCAAGAGT | CATAGCACCAGCTGGTCCAT TGACTAC |
| | 21-225_68D12 | | SEQ ID NO:5631 | SEQ ID NO:13643 | SEQ ID NO:21655 |
| | | AA | RSSYYWG | NIYYSGSTYYIPSLKS | HSTSWSIDY |
| | | | SEQ ID NO:5632 | SEQ ID NO:13644 | SEQ ID NO:21656 |
| iPS:434417 | | NA | AACTATGCCATGCAC | GTTATATGGTATGATGG AAGTGATAAATACTATG CAGACTCCGTGAAGGGC | GATATCCCTAGCAACTCGGC GGGGGACTAC |
| | 21-225_69C8 | | SEQ ID NO:5633 | SEQ ID NO:13645 | SEQ ID NO:21657 |

FIGURE 49

| | | | AA | NYAMH | VIWYDGSDKYYADSVKG | DIPSNSAGDY |
|---|---|---|---|---|---|---|
| | | | | SEQ ID NO:5634 | SEQ ID NO:13646 | SEQ ID NO:21658 |
| iPS:434423 | 21-225_70D1 | NA | | GGCTACCATATGCAC | TGGATCAACCCTAACAGTAATGCCACAAACTATGCACAGAAGTTTCAGGGC | GATAGCATATCGTCGTGGGACTAC |
| | | | | SEQ ID NO:5635 | SEQ ID NO:13647 | SEQ ID NO:21659 |
| | | AA | | GYHMH | WINPNSNATNYAQKFQG | DSISSWDY |
| | | | | SEQ ID NO:5636 | SEQ ID NO:13648 | SEQ ID NO:21660 |
| iPS:434425 | 21-225_70A5 | NA | | AGCTATGGCATGCAC | GTTATATGGTATGATGGAAGTAATAAATACTATGCAGACTCCGTGAAGGGC | GATCAGGGCATAGTGGGAGCTACTTGGTTTGACTAC |
| | | | | SEQ ID NO:5637 | SEQ ID NO:13649 | SEQ ID NO:21661 |
| | | AA | | SYGMH | VIWYDGSNKYYADSVKG | DQGIVGATWFDY |
| | | | | SEQ ID NO:5638 | SEQ ID NO:13650 | SEQ ID NO:21662 |
| iPS:434427 | 21-225_70D6 | NA | | GGCTACTATATGCAC | TGGATCAACCCTAAGAGTGGTGGCACAAACTCTGCACAGAAGTTTCAGGGC | GCTCCGGGTAAAGCAGCAGCTGGTACATGGGGGATTCTTTGACTAC |
| | | | | SEQ ID NO:5639 | SEQ ID NO:13651 | SEQ ID NO:21663 |
| | | AA | | GYYMH | WINPKSGGTNSAQKFQG | APGKAAAGTWGFFDY |
| | | | | SEQ ID NO:5640 | SEQ ID NO:13652 | SEQ ID NO:21664 |
| iPS:434429 | 21-225_70H6 | NA | | AGTTATGGCATGCAC | GTTATATGGCATGATGGAAGTAATAAATACTATGCAGACTCCGTGAAGGGC | GATGATCCCAGATCCTCGGCCGGGGACTAC |
| | | | | SEQ ID NO:5641 | SEQ ID NO:13653 | SEQ ID NO:21665 |
| | | AA | | SYGMH | VIWHDGSNKYYADSVKG | DDPRSSAGDY |

FIGURE 49

| | | | | SEQ ID NO:5642 | SEQ ID NO:13654 | SEQ ID NO:21666 |
|---|---|---|---|---|---|---|
| iPS:434431 | 21-225_70E7 | NA | | AATTATGATATCAAC | TGGATGCACCCTAACAG TGGTAACACAGGCTATG CACAGAAGTTCCAGGGC | AGCAGTGGCTGGTACGTCTT TGACTAC |
| | | | | SEQ ID NO:5643 | SEQ ID NO:13655 | SEQ ID NO:21667 |
| | | AA | | NYDIN | WMHPNSGNTGYAQKFQG | SSGWYVFDY |
| | | | | SEQ ID NO:5644 | SEQ ID NO:13656 | SEQ ID NO:21668 |
| iPS:434433 | 21-225_70E8 | NA | | AGCTATGGCATGCTC | ATTATATGGTATGATGA AAGTAATAAATACTATG CAGACTCCGTGAAGGGC | GACCTACTGGACCCACGGGA CTAC |
| | | | | SEQ ID NO:5645 | SEQ ID NO:13657 | SEQ ID NO:21669 |
| | | AA | | SYGML | IIWYDESNKYYADSVKG | DLLDPRDY |
| | | | | SEQ ID NO:5646 | SEQ ID NO:13658 | SEQ ID NO:21670 |
| iPS:434435 | 21-225_70G9 | NA | | GGCTACTATATGCAC | TGGATCAAACCTAACAG TGGTGGCACAAACCAAG CACAGAAGTTTCAGGGC | GCTCCGGGTATAGCAGCAGC TGGTACATGGGGGATACTTTG ACTAC |
| | | | | SEQ ID NO:5647 | SEQ ID NO:13659 | SEQ ID NO:21671 |
| | | AA | | GYYMH | WIKPNSGGTNQAQKFQG | APGIAAAGTWGYFDY |
| | | | | SEQ ID NO:5648 | SEQ ID NO:13660 | SEQ ID NO:21672 |
| iPS:434437 | 21-225_70A12 | NA | | GGCTACTATATGCAC | TGGATCAAACCAAACAG TGGTGGCACAAACCAAG CACAGAAGTTTCAGGGC | GCTCCGGGTACTGCAGCAAC TGGTACATGGGGGATACTTTG ACTAC |
| | | | | SEQ ID NO:5649 | SEQ ID NO:13661 | SEQ ID NO:21673 |
| | | AA | | GYYMH | WIKPNSGGTNQAQKFQG | APGTAATGTWGYFDY |
| | | | | SEQ ID NO:5650 | SEQ ID NO:13662 | SEQ ID NO:21674 |

FIGURE 49

| iPS:434439 | | NA | AGCTATAGCATGAAC | TCCATTAGTGGTAATAGT ACTTACATATACTACACA GACTCAGTGAAGGGC | GTGGCCGCCTTTGACTGC |
|---|---|---|---|---|---|
| | 21-225_70E12 | | SEQ ID NO:5651 | SEQ ID NO:13663 | SEQ ID NO:21675 |
| | | AA | SYSMN | SISGNSTYIYYTDSVKG | VAAFDC |
| | | | SEQ ID NO:5652 | SEQ ID NO:13664 | SEQ ID NO:21676 |
| iPS:434441 | | NA | GACTATGGCATGCAC | GTGATATGGTATGATGA AAGTAATAAATACTATG CAGACTCCGTGAAGGGC | GAATTGGGGTGGCAGGATGA TTAC |
| | 21-225_71A2 | | SEQ ID NO:5653 | SEQ ID NO:13665 | SEQ ID NO:21677 |
| | | AA | DYGMH | VIWYDESNKYYADSVKG | ELGWQDDY |
| | | | SEQ ID NO:5654 | SEQ ID NO:13666 | SEQ ID NO:21678 |
| iPS:434443 | | NA | AATTATGATATCAAC | TGGATGCACCCTAACAG TGGTAGCACAGGCTATG CACAGAAGTTCCAGGGC | AGCAGTGGCTGGTACTACTT TGACTAC |
| | 21-225_71G3 | | SEQ ID NO:5655 | SEQ ID NO:13667 | SEQ ID NO:21679 |
| | | AA | NYDIN | WMHPNSGSTGYAQKFQG | SSGWYYFDY |
| | | | SEQ ID NO:5656 | SEQ ID NO:13668 | SEQ ID NO:21680 |
| iPS:434447 | | NA | AACTATGGCATGCAC | GTTATTTGGTATGATAGA ACAAATAAATACTATGC AGACTCCGTGAAGGGC | GAACTGGGGATGTTGTCTGA CTAC |
| | 21-225_71B6 | | SEQ ID NO:5657 | SEQ ID NO:13669 | SEQ ID NO:21681 |
| | | AA | NYGMH | VIWYDRTNKYYADSVKG | ELGMLSDY |
| | | | SEQ ID NO:5658 | SEQ ID NO:13670 | SEQ ID NO:21682 |

FIGURE 49

| iPS:434449 | | NA | AGCTATAGCATGAAC | TCCATTAGTGGTACTAGT AGTTACATATACTACGC AGACTCAGTGAAGGGC | ACCAATGCTTTTGATATC |
|---|---|---|---|---|---|
| | 21-225_71H6 | | SEQ ID NO:5659 | SEQ ID NO:13671 | SEQ ID NO:21683 |
| | | AA | SYSMN | SISGTSSYIYYADSVKG | TNAFDI |
| | | | SEQ ID NO:5660 | SEQ ID NO:13672 | SEQ ID NO:21684 |
| iPS:434451 | | NA | GGCTACTATATGCAC | TGGATCAACCCTAACAG TGGTGGCACAAACTCTG CACAGAAGTTTCAGGGC | GCTCCGGGTAAAGCAGCAGC TGGTACATGGGGATTCTTTG ACTAC |
| | 21-225_71B7 | | SEQ ID NO:5661 | SEQ ID NO:13673 | SEQ ID NO:21685 |
| | | AA | GYYMH | WINPNSGGTNSAQKFQG | APGKAAAGTWGFFDY |
| | | | SEQ ID NO:5662 | SEQ ID NO:13674 | SEQ ID NO:21686 |
| iPS:434453 | | NA | GACTATGGCATGCAC | GTTATATGGTATGATAG AAATAATAAATACTATG GAGACTCCGTGAAGGGC | GAACTGGGGATGTTGTCTGA CTAC |
| | 21-225_71B11 | | SEQ ID NO:5663 | SEQ ID NO:13675 | SEQ ID NO:21687 |
| | | AA | DYGMH | VIWYDRNNKYYGDSVKG | ELGMLSDY |
| | | | SEQ ID NO:5664 | SEQ ID NO:13676 | SEQ ID NO:21688 |
| iPS:434455 | | NA | AGCTATGCCATGATC | ACTATTAGTGGTAGTGGT GGTTACACATACTCCGC AGACTCCGTGAAGGGC | CGTATAGCAGTGACTGGGAC GGAATGGTACGACCCC |
| | 21-225_72F5 | | SEQ ID NO:5665 | SEQ ID NO:13677 | SEQ ID NO:21689 |
| | | AA | SYAMI | TISGSGGYTYSADSVKG | RIAVTGTEWYDP |
| | | | SEQ ID NO:5666 | SEQ ID NO:13678 | SEQ ID NO:21690 |
| iPS:434457 | | NA | AGCTATGGCATGCAC | GTTATATGGTTTGATGAA AGTAATAAATACTATGC AGACTCCGTGAAGGGC | GAGCTTGGTTTCAGCAGTGA CTAC |
| | 21-225_72G12 | | | | |

FIGURE 49

| | | | SEQ ID NO:5667 | SEQ ID NO:13679 | SEQ ID NO:21691 |
|---|---|---|---|---|---|
| | | AA | SYGMH | VIWFDESNKYYADSVKG | ELGFSSDY |
| iPS:434459 | | | SEQ ID NO:5668 | SEQ ID NO:13680 | SEQ ID NO:21692 |
| | 21-225_71A7 | NA | GGCTACTATATGCAC | TGGATCAACCCTAAAAG TGGTGGCACAAATTATG TACAGAAGTTTCAGGGC | GCTCCGGGTACAGCACCAGC TGGGTCATGGGGATACTTTG ACTAC |
| | | | SEQ ID NO:5669 | SEQ ID NO:13681 | SEQ ID NO:21693 |
| | | AA | GYYMH | WINPKSGGTNYVQKFQG | APGTAPAGSWGYFDY |
| iPS:434461 | | | SEQ ID NO:5670 | SEQ ID NO:13682 | SEQ ID NO:21694 |
| | 21-225_73A3 | NA | GGCTACTATATGCAC | TGGATCAACCCTAAAAG TGGTGGCACGAATCATG TCCAGAAGTTTCAGGGC | GCTCCGGGTACAGCAGCAGC TGGGTCATGGGGATGCTTTG ACTAC |
| | | | SEQ ID NO:5671 | SEQ ID NO:13683 | SEQ ID NO:21695 |
| | | AA | GYYMH | WINPKSGGTNHVQKFQG | APGTAAAGSWGCFDY |
| iPS:434463 | | | SEQ ID NO:5672 | SEQ ID NO:13684 | SEQ ID NO:21696 |
| | 21-225_73A6 | NA | TACTATGGCATGCAC | GTTATATTATATGATGGA AGTAAGAAATACTATGC AGCCTCCGTGAAGGGC | AGTATCCCGGACTTTGACTA C |
| | | | SEQ ID NO:5673 | SEQ ID NO:13685 | SEQ ID NO:21697 |
| | | AA | YYGMH | VILYDGSKKYYAASVKG | SIPDFDY |
| iPS:434467 | | | SEQ ID NO:5674 | SEQ ID NO:13686 | SEQ ID NO:21698 |
| | 21-225_73H8 | NA | AGCAATGCCATGAGC | GACATTAGTCGTAGTGG TGGTACCACATTCTACGC AGACTCCGTGAAGGGC | TGGGATAGCAGCAGCTGGTA CGACGTGACTCCCTTTGACT AC |
| | | | SEQ ID NO:5675 | SEQ ID NO:13687 | SEQ ID NO:21699 |
| | | AA | SNAMS | DISRSGGTTFYADSVKG | WDSSSWYDVTPFDY |
| | | | SEQ ID NO:5676 | SEQ ID NO:13688 | SEQ ID NO:21700 |

FIGURE 49

| iPS:434469 | | NA | AATTATGTCATGCAC | GTTATATGGTATGATGG AAGTAATAAATACTATG CAGACTCCGTGAAGGGC | GAAAGGTATAGCAGCAGCTG GTTCGACTACGGTATGGACG TC |
|---|---|---|---|---|---|
| | 21-225_73C9 | | SEQ ID NO:5677 | SEQ ID NO:13689 | SEQ ID NO:21701 |
| | | AA | NYVMH | VIWYDGSNKYYADSVKG | ERYSSSWFDYGMDV |
| | | | SEQ ID NO:5678 | SEQ ID NO:13690 | SEQ ID NO:21702 |
| iPS:434471 | | NA | GGTTCCTACTGGAGC | GAAATCAATCTTAGTGG AAGTACCAACTACAACC CGTCCCTCAAGAGT | GACTACGGTGGCCTTGACTA C |
| | 21-225_75G3 | | SEQ ID NO:5679 | SEQ ID NO:13691 | SEQ ID NO:21703 |
| | | AA | GSYWS | EINLSGSTNYNPSLKS | DYGGLDY |
| | | | SEQ ID NO:5680 | SEQ ID NO:13692 | SEQ ID NO:21704 |
| iPS:434473 | | NA | GGTTGCTACTGGAGC | GAAATCAATCATAGTGG AAGAACCAACTACAACC CGTCCCTCAAGAGT | GACTACGGCGGTATGGACGT C |
| | 21-225_76D1 | | SEQ ID NO:5681 | SEQ ID NO:13693 | SEQ ID NO:21705 |
| | | AA | GCYWS | EINHSGRTNYNPSLKS | DYGGMDV |
| | | | SEQ ID NO:5682 | SEQ ID NO:13694 | SEQ ID NO:21706 |
| iPS:434475 | | NA | AATTATGATATCAAC | TGGATGAACCCTAACAG TGGTAACACAGGCTGTG CACAGAAGTTCCAGGGC | AGCAGTGGCTGGAACTTCTT TGACTAC |
| | 21-225_74F9 | | SEQ ID NO:5683 | SEQ ID NO:13695 | SEQ ID NO:21707 |
| | | AA | NYDIN | WMNPNSGNTGCAQKFQG | SSGWNFFDY |
| | | | SEQ ID NO:5684 | SEQ ID NO:13696 | SEQ ID NO:21708 |
| iPS:434477 | | NA | AATTATGATATCAAC | TGGATGCACCCTAACAG TGGTAACACAGGCTATG CACAGAAGTTCCGGGGC | AGCAGTGGCTGGTACTACTT TGACTAC |

FIGURE 49

| | 21-225_74A6 | | SEQ ID NO:5685 | SEQ ID NO:13697 | SEQ ID NO:21709 |
|---|---|---|---|---|---|
| | | AA | NYDIN | WMHPNSGNTGYAQKFRG | SSGWYYFDY |
| | | | SEQ ID NO:5686 | SEQ ID NO:13698 | SEQ ID NO:21710 |
| iPS:434479 | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | 21-225_76H1 | | SEQ ID NO:5687 | SEQ ID NO:13699 | SEQ ID NO:21711 |
| | | AA | NYDIN | WMHPNSGNTGYAQKFQG | SSGWYWFDP |
| | | | SEQ ID NO:5688 | SEQ ID NO:13700 | SEQ ID NO:21712 |
| iPS:434481 | | NA | AATTATGATATCAAC | TGGATGCACCCTAACAG TGGTAACACAGGCTTTG CACAGAAGTTCCAGGGC | TCCAGTGGCTGGTACTGGTT CGACCCC |
| | 21-225_74B10 | | SEQ ID NO:5689 | SEQ ID NO:13701 | SEQ ID NO:21713 |
| | | AA | NYDIN | WMHPNSGNTGFAQKFQG | SSGWYWFDP |
| | | | SEQ ID NO:5690 | SEQ ID NO:13702 | SEQ ID NO:21714 |
| iPS:434483 | | NA | AATTATGATATCAAC | TGGATGAACCCTAACAG TGGTAACACAGGCTATG CACAGAAGTTCCAGGGC | AGCAGTGGCTGGTACTGGTT CGACCCC |
| | 21-225_74C12 | | SEQ ID NO:5691 | SEQ ID NO:13703 | SEQ ID NO:21715 |
| | | AA | NYDIN | WMNPNSGNTGYAQKFQG | SSGWYWFDP |
| | | | SEQ ID NO:5692 | SEQ ID NO:13704 | SEQ ID NO:21716 |
| iPS:434485 | | NA | AGCTATGGCATGCAC | GTTATTTGGTATGATGGA AGTAATAAATACTATGC AGACTCCGTGAAGGGC | GATCGCAATATAGTGGGAGC TACTTACTTTGAGTCC |
| | 21-225_76D2 | | SEQ ID NO:5693 | SEQ ID NO:13705 | SEQ ID NO:21717 |

FIGURE 49

| | | AA | SYGMH | VIWYDGSNKYYADSVKG | DRNIVGATYFES |
|---|---|---|---|---|---|
| | | | SEQ ID NO:5694 | SEQ ID NO:13706 | SEQ ID NO:21718 |
| iPS:434487 | 21-225_76G2 | NA | AATTATGATATCAAC | TGGATGAACCCTAACAG TGGTAACACAGGCTATG CACAGAAGTTCCAGGGC | AGCAGTGGCTGGTACATGTT TGACTAC |
| | | | SEQ ID NO:5695 | SEQ ID NO:13707 | SEQ ID NO:21719 |
| | | AA | NYDIN | WMNPNSGNTGYAQKFQG | SSGWYMFDY |
| | | | SEQ ID NO:5696 | SEQ ID NO:13708 | SEQ ID NO:21720 |
| iPS:434489 | 21-225_74E4 | NA | AGTAGTAATTACTACTGGGG C | AGTATCTATTATAGTGGA TACACCTCCTACAACCCG TCCCTCAAGAGT | CTTGACTCTAACTGGGGTCT TGACTAC |
| | | | SEQ ID NO:5697 | SEQ ID NO:13709 | SEQ ID NO:21721 |
| | | AA | SSNYYWG | SIYYSGYTSYNPSLKS | LDSNWGLDY |
| | | | SEQ ID NO:5698 | SEQ ID NO:13710 | SEQ ID NO:21722 |
| iPS:434493 | 21-225_76F3 | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | | | SEQ ID NO:5699 | SEQ ID NO:13711 | SEQ ID NO:21723 |
| | | AA | NYDIN | WMNPNSGNTGYAQKFQG | SSGWNWFDP |
| | | | SEQ ID NO:5700 | SEQ ID NO:13712 | SEQ ID NO:21724 |
| iPS:434495 | 21-225_74B2 | NA | GGTCCCTACTGGAGC | GAAATCAATCATAGTGG AAGCACCAACTACAACC CGTCCCTCACGAGT | GACTACGGGGGTTTGGACGT C |
| | | | SEQ ID NO:5701 | SEQ ID NO:13713 | SEQ ID NO:21725 |
| | | AA | GPYWS | EINHSGSTNYNPSLTS | DYGGLDV |
| | | | SEQ ID NO:5702 | SEQ ID NO:13714 | SEQ ID NO:21726 |

FIGURE 49

| iPS:434497 | | NA | GGTTGCTACTGGAGC | GAAATCAATCATAGTGG AAGCACCAACTACAACC CGTCCCTCAAGAGT | GACTACGGCGGTATGGACGT C |
|---|---|---|---|---|---|
| | 21-225_76A4 | | SEQ ID NO:5703 | SEQ ID NO:13715 | SEQ ID NO:21727 |
| | | AA | GCYWS | EINHSGSTNYNPSLKS | DYGGMDV |
| | | | SEQ ID NO:5704 | SEQ ID NO:13716 | SEQ ID NO:21728 |
| iPS:434501 | | NA | GGTTGCTACTGGAGC | GAAATCAATCATAGTGG AAGCACCAACTACAACC CGTCCCTCAAGAGT | GACTACGGCGGTATGGACGT C |
| | 21-225_76G4 | | SEQ ID NO:5705 | SEQ ID NO:13717 | SEQ ID NO:21729 |
| | | AA | GCYWS | EINHSGSTNYNPSLKS | DYGGMDV |
| | | | SEQ ID NO:5706 | SEQ ID NO:13718 | SEQ ID NO:21730 |
| iPS:434503 | | NA | AGAAGTAGTTACTACTGGGG C | GGTATCTATTATAGTGGG AGCACCTCCTACAACCC GTCCCTCAAGAGT | CTGCGACCTAACTGGGACTT TGACTAC |
| | 21-225_74D7 | | SEQ ID NO:5707 | SEQ ID NO:13719 | SEQ ID NO:21731 |
| | | AA | RSSYYWG | GIYYSGSTSYNPSLKS | LRPNWDFDY |
| | | | SEQ ID NO:5708 | SEQ ID NO:13720 | SEQ ID NO:21732 |
| iPS:434507 | | NA | GGTTGCTACTGGAGC | GAAATCAATCATAGTGG ATGCACCAACTTCAACC CGTCCCTCAAGAGT | GACTACGGCGGTATGGACGT C |
| | 21-225_74C5 | | SEQ ID NO:5709 | SEQ ID NO:13721 | SEQ ID NO:21733 |
| | | AA | GCYWS | EINHSGCTNFNPSLKS | DYGGMDV |
| | | | SEQ ID NO:5710 | SEQ ID NO:13722 | SEQ ID NO:21734 |
| iPS:434509 | | NA | AATTATGATATCAAC | TGGATGAACCCTAACAG TGGTAACACAGGCTATG CACAGAAGTTCCAGGGC | AGCAGTGGCTGGTACTGGTT CGACCCC |
| | 21-225_76F5 | | SEQ ID NO:5711 | SEQ ID NO:13723 | SEQ ID NO:21735 |
| | | AA | NYDIN | WMNPNSGNTGYAQKFQG | SSGWYWFDP |

FIGURE 49

| | | | SEQ ID NO:5712 | SEQ ID NO:13724 | SEQ ID NO:21736 |
|---|---|---|---|---|---|
| iPS:434511 | | NA | AATTATGATATCAAC | TGGATGCACCCTAACAG TGGTAACACAGGCTATG CACAGAAGTTCCAGGGC | AGCAGTGGCTGGTACTACTT TGACTAC |
| | 21-225_74B11 | | SEQ ID NO:5713 | SEQ ID NO:13725 | SEQ ID NO:21737 |
| | | AA | NYDIN | WMHPNSGNTGYAQKFQG | SSGWYYFDY |
| | | | SEQ ID NO:5714 | SEQ ID NO:13726 | SEQ ID NO:21738 |
| iPS:434513 | | NA | AATTATGATATCAAC | TGGATGCACCCTAACAA TGGTAACACAGGCTATG CACAGAAGTTCCAGGGC | AGCAGTGGCTGGTACTGGTT CGACCCC |
| | 21-225_76A6 | | SEQ ID NO:5715 | SEQ ID NO:13727 | SEQ ID NO:21739 |
| | | AA | NYDIN | WMHPNNGNTGYAQKFQG | SSGWYWFDP |
| | | | SEQ ID NO:5716 | SEQ ID NO:13728 | SEQ ID NO:21740 |
| iPS:434515 | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | 21-225_74A5 | | SEQ ID NO:5717 | SEQ ID NO:13729 | SEQ ID NO:21741 |
| | | AA | NYDIN | WMHPNSGNTGYAQKFQG | SSGWYWFDP |
| | | | SEQ ID NO:5718 | SEQ ID NO:13730 | SEQ ID NO:21742 |
| iPS:434517 | | NA | GGTTGCTACTGGAGC | GAAATCAATCATAGTGG AAGGACCAACTACAACC CGTCCCTCAAGAGT | GACTACGGTGGGCTTGACTA C |
| | 21-225_76A7 | | SEQ ID NO:5719 | SEQ ID NO:13731 | SEQ ID NO:21743 |
| | | AA | GCYWS | EINHSGRTNYNPSLKS | DYGGLDY |
| | | | SEQ ID NO:5720 | SEQ ID NO:13732 | SEQ ID NO:21744 |

FIGURE 49

| iPS:434519 | | NA | GGTTCCTACTGGAGC | GAAATCAATCTTAGTGG AAGCACCAACTACAACC CGTCCCTCAAGAGT | GACTACGGTGGCCTTGACTA C |
| | 21-225_74C7 | | SEQ ID NO:5721 | SEQ ID NO:13733 | SEQ ID NO:21745 |
| | | AA | GSYWS | EINLSGSTNYNPSLKS | DYGGLDY |
| | | | SEQ ID NO:5722 | SEQ ID NO:13734 | SEQ ID NO:21746 |
| iPS:434523 | | NA | GGTTGCTACTGGAGC | GAAATCAATTATAGTGG AAGAACCAACTACAACC CGTCCCTCAAGAGT | GACTACGGCGGTATGGACGT C |
| | 21-225_75C3 | | SEQ ID NO:5723 | SEQ ID NO:13735 | SEQ ID NO:21747 |
| | | AA | GCYWS | EINYSGRTNYNPSLKS | DYGGMDV |
| | | | SEQ ID NO:5724 | SEQ ID NO:13736 | SEQ ID NO:21748 |
| iPS:434525 | | NA | AATTATGATATCAAC | TGGATGAACCCTAACAG TGGTAACACAGGCTATG CACAGAAGTTCCAGGGC | TCCAGTGGCTGGCACTGGTT CGACCCC |
| | 21-225_76E8 | | SEQ ID NO:5725 | SEQ ID NO:13737 | SEQ ID NO:21749 |
| | | AA | NYDIN | WMNPNSGNTGYAQKFQG | SSGWHWFDP |
| | | | SEQ ID NO:5726 | SEQ ID NO:13738 | SEQ ID NO:21750 |
| iPS:434529 | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | 21-225_76B9 | | SEQ ID NO:5727 | SEQ ID NO:13739 | SEQ ID NO:21751 |
| | | AA | NYDIN | WMHPNSGNTGYAQKFQG | SSGWYWFDP |
| | | | SEQ ID NO:5728 | SEQ ID NO:13740 | SEQ ID NO:21752 |
| iPS:434531 | 21 225 76C9 | NA | AACGCCTGGATGAAC | CGTATTAAAAACAAAGC TGATGGTGGGACAACAG ACTTCGCTGCACCCGTGA AAGGC | GTGGGACCTACTACGGACTA C |

FIGURE 49

| | | | | SEQ ID NO:5729 | SEQ ID NO:13741 | SEQ ID NO:21753 |
|---|---|---|---|---|---|---|
| | 21-225_76C9 | AA | | NAWMN | RIKNKADGGTTDFAAPVKG | VGPTTDY |
| | | | | SEQ ID NO:5730 | SEQ ID NO:13742 | SEQ ID NO:21754 |
| iPS:434533 | | NA | | GGTCCCTACTGGAGC | GAAATCAATCATAGTGGAAGCACCAACTACAACCCGTCCCTCAAGAGT | GACTACGGGGGTTTGGACGTC |
| | 21-225_85F7 | | | SEQ ID NO:5731 | SEQ ID NO:13743 | SEQ ID NO:21755 |
| | | AA | | GPYWS | EINHSGSTNYNPSLKS | DYGGLDV |
| | | | | SEQ ID NO:5732 | SEQ ID NO:13744 | SEQ ID NO:21756 |
| iPS:434535 | | NA | | AGCTATGGCATGCAC | GTTATATGGTATGATGGAAGTAATAAAAACTATGCAGACTCCGTGAAGGGC | GATGGCAGCTATGGTTATGACGGCCTTGACTAC |
| | 21-225_74C8 | | | SEQ ID NO:5733 | SEQ ID NO:13745 | SEQ ID NO:21757 |
| | | AA | | SYGMH | VIWYDGSNKNYADSVKG | DGSYGYDGLDY |
| | | | | SEQ ID NO:5734 | SEQ ID NO:13746 | SEQ ID NO:21758 |
| iPS:434537 | | NA | | AGCTATGGCATGCAC | GTTATTTGGTATGATGGAAGTAATAAATACTATGCAGACTCCGTGAAGGGC | GATCGCAATATAGTGGGAGCTACTTACTTTGAGTCC |
| | 21-225_74E11 | | | SEQ ID NO:5735 | SEQ ID NO:13747 | SEQ ID NO:21759 |
| | | AA | | SYGMH | VIWYDGSNKYYADSVKG | DRNIVGATYFES |
| | | | | SEQ ID NO:5736 | SEQ ID NO:13748 | SEQ ID NO:21760 |
| iPS:434539 | | NA | | GATTACTACTGGAGC | GAAATCAATCATAGTGGAGACACCAACTACAACCCGTCCCTCAAGAGT | GAGTTTCCATATAGTGGAAGCTACCTCTACTACTACGGTATGGACGTC |
| | 21-225_74A2 | | | SEQ ID NO:5737 | SEQ ID NO:13749 | SEQ ID NO:21761 |

FIGURE 49

| | | AA | DYYWS | EINHSGDTNYNPSLKS | EFPYSGSYLYYYGMDV |
|---|---|---|---|---|---|
| | | | SEQ ID NO:5738 | SEQ ID NO:13750 | SEQ ID NO:21762 |
| iPS:434547 | 21-225_74H5 | NA | GGTTGCTACTGGAGC | GAAATCAATCATAGTGG AAGGACCAACTTCAACC CGTCCCTCAAGAGT | GACTACGGCGGTATGGACGT C |
| | | | SEQ ID NO:5739 | SEQ ID NO:13751 | SEQ ID NO:21763 |
| | | AA | GCYWS | EINHSGRTNFNPSLKS | DYGGMDV |
| | | | SEQ ID NO:5740 | SEQ ID NO:13752 | SEQ ID NO:21764 |
| iPS:434549 | 21-225_76E11 | NA | AATTATGATATCAAC | TGGATGAACCCTAACAG TGGTAACACAGGCTATG CACAGAAGTTCCAGGGC | AGCAGTGGCTGGTACTACTT TGACTAC |
| | | | SEQ ID NO:5741 | SEQ ID NO:13753 | SEQ ID NO:21765 |
| | | AA | NYDIN | WMNPNSGNTGYAQKFQG | SSGWYYFDY |
| | | | SEQ ID NO:5742 | SEQ ID NO:13754 | SEQ ID NO:21766 |
| iPS:434551 | 21-225_75C4 | NA | AATTATGATATCAAC | TGGATGCATCCTAACAG TGGTAACACAGGCTATG CACAGAAGTTCCAGGGC | AGCAGTGGCTGGTACTACTT TGACTAC |
| | | | SEQ ID NO:5743 | SEQ ID NO:13755 | SEQ ID NO:21767 |
| | | AA | NYDIN | WMHPNSGNTGYAQKFQG | SSGWYYFDY |
| | | | SEQ ID NO:5744 | SEQ ID NO:13756 | SEQ ID NO:21768 |
| iPS:434559 | 21-225_74D11 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5745 | SEQ ID NO:13757 | SEQ ID NO:21769 |
| | | AA | GCYWS | EINHSGSTNYNPSLKS | DYGGMDV |
| | | | SEQ ID NO:5746 | SEQ ID NO:13758 | SEQ ID NO:21770 |

FIGURE 49

| iPS:434561 | 21-225_77G1 | NA | GGTTGCTACTGGAGC | GAAATCAATCATAGTGG AAGCACCAACTACAACC CGTCCCTCAAGAGT | GACTACGGCGGTATGGACGT C |
|---|---|---|---|---|---|
| | | | SEQ ID NO:5747 | SEQ ID NO:13759 | SEQ ID NO:21771 |
| | | AA | GCYWS | EINHSGSTNYNPSLKS | DYGGMDV |
| | | | SEQ ID NO:5748 | SEQ ID NO:13760 | SEQ ID NO:21772 |
| iPS:434563 | 21-225_75D8 | NA | AACTACGACATGCAC | GCTATTGGTACTGCTGGT GACACATACTATCCAGG CTCCGTGAAGGGC | GTTCTTGACTACGGTGACTC CTTGGGCTACTACTACTACG GTATGGACGTC |
| | | | SEQ ID NO:5749 | SEQ ID NO:13761 | SEQ ID NO:21773 |
| | | AA | NYDMH | AIGTAGDTYYPGSVKG | VLDYGDSLGYYYYGMDV |
| | | | SEQ ID NO:5750 | SEQ ID NO:13762 | SEQ ID NO:21774 |
| iPS:434565 | 21-225_75B10 | NA | GGTTACTACTGGAGC | GAAATCAATCACAGTGG AAGCACCAACTACAACC CGTCCCTCAAGAGT | GACTACGGTGGTTTGGACGT C |
| | | | SEQ ID NO:5751 | SEQ ID NO:13763 | SEQ ID NO:21775 |
| | | AA | GYYWS | EINHSGSTNYNPSLKS | DYGGLDV |
| | | | SEQ ID NO:5752 | SEQ ID NO:13764 | SEQ ID NO:21776 |
| iPS:434569 | 21-225_77H5 | NA | AATTATGGCATGCAC | GTTATATGGTATGATGG AAGAAATAAATACTATG CAGACTCCGTGAAGGGC | GATCGGAGTATATTGGGAGC TACTTTCTTTGACTAC |
| | | | SEQ ID NO:5753 | SEQ ID NO:13765 | SEQ ID NO:21777 |
| | | AA | NYGMH | VIWYDGRNKYYADSVKG | DRSILGATFFDY |
| | | | SEQ ID NO:5754 | SEQ ID NO:13766 | SEQ ID NO:21778 |
| iPS:434571 | 21-225_74D2 | NA | GGTTGCTACTGGAGC | GAAATCAATCATAGTGG AAGGACCAACTACAACC CGTCCCTCAAGAGT | GACTACGGTGGGCTTGACTA C |
| | | | SEQ ID NO:5755 | SEQ ID NO:13767 | SEQ ID NO:21779 |

FIGURE 49

| | | AA | GCYWS | EINHSGRTNYNPSLKS | DYGGLDY |
|---|---|---|---|---|---|
| | | | SEQ ID NO:5756 | SEQ ID NO:13768 | SEQ ID NO:21780 |
| iPS:434573 | | NA | AGCTATGGCATGCAC | GTTATATGGTATGATGG AAGTAATCAAAACTATG CAGACTCCGTGAAGGGC | GATGGCAGCTATGGTTACGA CGGCCTTGACTAC |
| | 21-225_77E6 | | SEQ ID NO:5757 | SEQ ID NO:13769 | SEQ ID NO:21781 |
| | | AA | SYGMH | VIWYDGSNQNYADSVKG | DGSYGYDGLDY |
| | | | SEQ ID NO:5758 | SEQ ID NO:13770 | SEQ ID NO:21782 |
| iPS:434575 | | NA | AATTATGATATCAAC | TGGATGAACCCTAACAG TGGTAACACAGGCTATG CACAGAAGTTCCAGGGC | TCCAGTGGCTGGCACTGGTT CGACCCC |
| | 21-225_77C7 | | SEQ ID NO:5759 | SEQ ID NO:13771 | SEQ ID NO:21783 |
| | | AA | NYDIN | WMNPNSGNTGYAQKFQG | SSGWHWFDP |
| | | | SEQ ID NO:5760 | SEQ ID NO:13772 | SEQ ID NO:21784 |
| iPS:434579 | | NA | GGTTGCTACTGGAGC | GAAATCAATCATAGTGG AAGCACCAACTACAACC CGTCCCTCAAGAGT | GACTACGGCGGTATGGACGT C |
| | 21-225_77F7 | | SEQ ID NO:5761 | SEQ ID NO:13773 | SEQ ID NO:21785 |
| | | AA | GCYWS | EINHSGSTNYNPSLKS | DYGGMDV |
| | | | SEQ ID NO:5762 | SEQ ID NO:13774 | SEQ ID NO:21786 |
| iPS:434581 | | NA | GGTTGCTACTGGAGC | GAAATCAATCATAGTGG AAGCACCAACTACAACC CGTCCCTCAAGAGT | GACTACGGCGGTATGGACGT C |
| | 21-225_74B12 | | SEQ ID NO:5763 | SEQ ID NO:13775 | SEQ ID NO:21787 |
| | | AA | GCYWS | EINHSGSTNYNPSLKS | DYGGMDV |
| | | | SEQ ID NO:5764 | SEQ ID NO:13776 | SEQ ID NO:21788 |

FIGURE 49

| iPS:434583 | | NA | AATTATGATATCAAC | TGGATGCACCCTAACAA TGGTAACACAGGCTATG CACAGAAGTTCCAGGGC | AGCAGTGGCTGGTACTGGTT CGACCCC |
|---|---|---|---|---|---|
| | 21-225_74B6 | | SEQ ID NO:5765 | SEQ ID NO:13777 | SEQ ID NO:21789 |
| | | AA | NYDIN | WMHPNNGNTGYAQKFQG | SSGWYWFDP |
| | | | SEQ ID NO:5766 | SEQ ID NO:13778 | SEQ ID NO:21790 |
| iPS:434585 | | NA | GGTTGCTACTGGAGC | GAAATCAATTATAGTGG AAGAACCAACTACAACC CGTCCCTCAAGAGT | GACTACGGCGGTATGGACGT C |
| | 21-225_75A12 | | SEQ ID NO:5767 | SEQ ID NO:13779 | SEQ ID NO:21791 |
| | | AA | GCYWS | EINYSGRTNYNPSLKS | DYGGMDV |
| | | | SEQ ID NO:5768 | SEQ ID NO:13780 | SEQ ID NO:21792 |
| iPS:434587 | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | 21-225_74G3 | | SEQ ID NO:5769 | SEQ ID NO:13781 | SEQ ID NO:21793 |
| | | AA | NYDIN | WMHPNSGNTGYAQKFQG | SSGWYWFDP |
| | | | SEQ ID NO:5770 | SEQ ID NO:13782 | SEQ ID NO:21794 |
| iPS:434595 | | NA | GGTTGCTACTGGAGC | GAAATCAATTATAGTGG AAGAACCAACTACAACC CGTCCCTCAAGAGT | GACTACGGCGGTATGGACGT C |
| | 21-225_77A10 | | SEQ ID NO:5771 | SEQ ID NO:13783 | SEQ ID NO:21795 |
| | | AA | GCYWS | EINYSGRTNYNPSLKS | DYGGMDV |
| | | | SEQ ID NO:5772 | SEQ ID NO:13784 | SEQ ID NO:21796 |
| iPS:434597 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | 21-225_77C10 | | SEQ ID NO:5773 | SEQ ID NO:13785 | SEQ ID NO:21797 |

FIGURE 49

| | | AA | NYDIN | WMHPNSGNTGYAQKFQG | SSGWYWFDP |
|---|---|---|---|---|---|
| | | | SEQ ID NO:5774 | SEQ ID NO:13786 | SEQ ID NO:21798 |
| iPS:434603 | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | 21-225_77D11 | | SEQ ID NO:5775 | SEQ ID NO:13787 | SEQ ID NO:21799 |
| | | AA | NYDIN | WMHPNSGNTGYAQKFQG | SSGWYWFDP |
| | | | SEQ ID NO:5776 | SEQ ID NO:13788 | SEQ ID NO:21800 |
| iPS:434611 | | NA | GGTTCCTACTGGAGC | GAAATCAATTATAGGGG AAGCACCAACTACAACC CGTCCCTCAAGAGT | GACTACGGCGGTATGGACGT C |
| | 21-225_77C12 | | SEQ ID NO:5777 | SEQ ID NO:13789 | SEQ ID NO:21801 |
| | | AA | GSYWS | EINYRGSTNYNPSLKS | DYGGMDV |
| | | | SEQ ID NO:5778 | SEQ ID NO:13790 | SEQ ID NO:21802 |
| iPS:434613 | | NA | AATTATGATATCAAC | TGGATGAACCCTAACAG TGGTAACACAGGCTATG CACAGAAGTTCCAGGGC | AGCAGTGGCTGGTACTGGTT CGACCCC |
| | 21-225_77D12 | | SEQ ID NO:5779 | SEQ ID NO:13791 | SEQ ID NO:21803 |
| | | AA | NYDIN | WMNPNSGNTGYAQKFQG | SSGWYWFDP |
| | | | SEQ ID NO:5780 | SEQ ID NO:13792 | SEQ ID NO:21804 |
| iPS:434615 | | NA | AGCTATGGCATGCAC | GTTATATGGTATGATGG AAGTAATAAAAACTATG CAGACTCCGTGAAGGGC | GATGGCAGCTATGGTTACGA CGGCCTTGACTAC |
| | 21-225_76C5 | | SEQ ID NO:5781 | SEQ ID NO:13793 | SEQ ID NO:21805 |
| | | AA | SYGMH | VIWYDGSNKNYADSVKG | DGSYGYDGLDY |

FIGURE 49

| | | | SEQ ID NO:5782 | SEQ ID NO:13794 | SEQ ID NO:21806 |
|---|---|---|---|---|---|
| iPS:434617 | | NA | AATTATGATATCAAC | TGGATGCACCCTAATAG TGGTAACACAGGCTATG CACAGAAGTTCCAGGGC | TCCAGTGGCTGGTACTGGTT CGACCCC |
| | 21-225_74B8 | | SEQ ID NO:5783 | SEQ ID NO:13795 | SEQ ID NO:21807 |
| | | AA | NYDIN | WMHPNSGNTGYAQKFQG | SSGWYWFDP |
| | | | SEQ ID NO:5784 | SEQ ID NO:13796 | SEQ ID NO:21808 |
| iPS:434619 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | 21-225_78C1 | | SEQ ID NO:5785 | SEQ ID NO:13797 | SEQ ID NO:21809 |
| | | AA | NYDIN | WMHPNSGNTGYAQKFQG | SSGWYWFDP |
| | | | SEQ ID NO:5786 | SEQ ID NO:13798 | SEQ ID NO:21810 |
| iPS:434621 | | NA | AGCTATGGCATGCAC | GTTATATGGTATGATGG AAGTAATAAATACCATG CAGACTCCGTGAAGGGC | GATGAGGGGTTCGGGGAGTT CGACTACTACAACTACGGTA TGGACGTC |
| | 21-225_74D1 | | SEQ ID NO:5787 | SEQ ID NO:13799 | SEQ ID NO:21811 |
| | | AA | SYGMH | VIWYDGSNKYHADSVKG | DEGFGEFDYYNYGMDV |
| | | | SEQ ID NO:5788 | SEQ ID NO:13800 | SEQ ID NO:21812 |
| iPS:434629 | | NA | AGCTTTGGCATGCAC | GCTATTTGGTATGATGGA AGTAATAAATACTGTGC AGACTCCGTGAAGGGC | GATCGGAGTATACTGGGAGC TGCTTTCTTTGACTAC |
| | 21-225_74C3 | | SEQ ID NO:5789 | SEQ ID NO:13801 | SEQ ID NO:21813 |
| | | AA | SFGMH | AIWYDGSNKYCADSVKG | DRSILGAAFFDY |
| | | | SEQ ID NO:5790 | SEQ ID NO:13802 | SEQ ID NO:21814 |

FIGURE 49

| iPS:434633 | | NA | AACGCCTGGATGAAC | CGTATTAAAAACAAAGC TGATGGTGGGACAACAG ACTACGCTGCACCCGTG AAAGGC | GTGGGAGCTACTACGGACTA C |
|---|---|---|---|---|---|
| | 21-225_74G8 | | SEQ ID NO:5791 | SEQ ID NO:13803 | SEQ ID NO:21815 |
| | | AA | NAWMN | RIKNKADGGTTDYAAPVK G | VGATTDY |
| | | | SEQ ID NO:5792 | SEQ ID NO:13804 | SEQ ID NO:21816 |
| iPS:434635 | | NA | AATTATGATATCAAC | TGGATGCACCCTAACAG TGGTAACACAGGCTATG CACAGAAGTTCCAGGGC | AGTAGTGGCTGGTACAAATT TGACTAC |
| | 21-225_78E6 | | SEQ ID NO:5793 | SEQ ID NO:13805 | SEQ ID NO:21817 |
| | | AA | NYDIN | WMHPNSGNTGYAQKFQG | SSGWYKFDY |
| | | | SEQ ID NO:5794 | SEQ ID NO:13806 | SEQ ID NO:21818 |
| iPS:434637 | | NA | GGTTCCTACTGGAGC | GAAATCAATCTTAGTGG AAGCACCAACTACAACC CGTCCCTCAAGAGT | GACTACGGTGGCCTTGACTA C |
| | 21-225_78E7 | | SEQ ID NO:5795 | SEQ ID NO:13807 | SEQ ID NO:21819 |
| | | AA | GSYWS | EINLSGSTNYNPSLKS | DYGGLDY |
| | | | SEQ ID NO:5796 | SEQ ID NO:13808 | SEQ ID NO:21820 |
| iPS:434639 | | NA | AATTATGATATCAAC | TGGATGAACCCTAACAG TGGTAACACAGGCTATG CACAGAAGTTCCAGGGC | TCCAGTGGCTGGCACTGGTT CGACCCC |
| | 21-225_74B7 | | SEQ ID NO:5797 | SEQ ID NO:13809 | SEQ ID NO:21821 |
| | | AA | NYDIN | WMNPNSGNTGYAQKFQG | SSGWHWFDP |
| | | | SEQ ID NO:5798 | SEQ ID NO:13810 | SEQ ID NO:21822 |

FIGURE 49

| iPS:434649 | | NA | AATTATGATATCAAC | TGGATGAACCCTAACAG TGGTAACACAGGCTGTG CACAGAAGTTCCAGGGC | AGCAGTGGCTGGAACTTCTT TGACTAC |
|---|---|---|---|---|---|
| | 21-225_78E11 | | SEQ ID NO:5799 | SEQ ID NO:13811 | SEQ ID NO:21823 |
| | | AA | NYDIN | WMNPNSGNTGCAQKFQG | SSGWNFFDY |
| | | | SEQ ID NO:5800 | SEQ ID NO:13812 | SEQ ID NO:21824 |
| iPS:434653 | | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | 21-225_74B5 | | SEQ ID NO:5801 | SEQ ID NO:13813 | SEQ ID NO:21825 |
| | | AA | NYDIN | WMNPNSGNTGYAQKFQG | SSGWNWFDP |
| | | | SEQ ID NO:5802 | SEQ ID NO:13814 | SEQ ID NO:21826 |
| iPS:434655 | | NA | AATTATGATATCAAC | TGGATGAACCCTAACAG TGGTAACACAGGCTATG CACAGAAGTTCCAGGGC | TCCAGTGGCTGGCACTGGTT CGACCCC |
| | 21-225_78H12 | | SEQ ID NO:5803 | SEQ ID NO:13815 | SEQ ID NO:21827 |
| | | AA | NYDIN | WMNPNSGNTGYAQKFQG | SSGWHWFDP |
| | | | SEQ ID NO:5804 | SEQ ID NO:13816 | SEQ ID NO:21828 |
| iPS:434657 | | NA | GGTTGCTACTGGAGC | GAAATCAATCATAGTGG AAGCACCAACTACAACC CGTCCCTCAAGAGT | GACTACGGCGGTATGGACGT C |
| | 21-225_79G1 | | SEQ ID NO:5805 | SEQ ID NO:13817 | SEQ ID NO:21829 |
| | | AA | GCYWS | EINHSGSTNYNPSLKS | DYGGMDV |
| | | | SEQ ID NO:5806 | SEQ ID NO:13818 | SEQ ID NO:21830 |
| iPS:434663 | 21 225 79F3 | NA | GGTTGCTACTGGAGC | GAAATCAATCATAGTGG AAGCACCAACTACAACC CGTCCCTCAAGAGT | GACTACGGCGGTATGGACGT C |

FIGURE 49

| | | | SEQ ID NO:5807 | SEQ ID NO:13819 | SEQ ID NO:21831 |
|---|---|---|---|---|---|
| | 21-225_79F3 | AA | GCYWS | EINHSGSTNYNPSLKS | DYGGMDV |
| | | | SEQ ID NO:5808 | SEQ ID NO:13820 | SEQ ID NO:21832 |
| iPS:434665 | | NA | AATTATGATGTCAAC | TGGATGCACCCTAACAGTGGTAACACAGGCTATGCACAGAAGTTCCAGGGC | AGCAGTGGCTGGTACCATTTTGACTAC |
| | 21-225_74G4 | | SEQ ID NO:5809 | SEQ ID NO:13821 | SEQ ID NO:21833 |
| | | AA | NYDVN | WMHPNSGNTGYAQKFQG | SSGWYHFDY |
| | | | SEQ ID NO:5810 | SEQ ID NO:13822 | SEQ ID NO:21834 |
| iPS:434669 | | NA | AACTATGGCATGCAC | GTTATATGGTATGATGGAAGTAATCAAAACTATGCAGACTCCGTGAAGGGC | GATGGCAGCTATGGTTATGACGGCCTTGACTAC |
| | 21-225_79F4 | | SEQ ID NO:5811 | SEQ ID NO:13823 | SEQ ID NO:21835 |
| | | AA | NYGMH | VIWYDGSNQNYADSVKG | DGSYGYDGLDY |
| | | | SEQ ID NO:5812 | SEQ ID NO:13824 | SEQ ID NO:21836 |
| iPS:434671 | | NA | AACGCCTGGATGAAC | CGAATTAAAAACAAAATTGATGGTGGGACAACAGACTACGCTGCACCCGTGAAAGGC | GTGGGAGCTACTACGGACTAC |
| | 21-225_74F4 | | SEQ ID NO:5813 | SEQ ID NO:13825 | SEQ ID NO:21837 |
| | | AA | NAWMN | RIKNKIDGGTTDYAAPVKG | VGATTDY |
| | | | SEQ ID NO:5814 | SEQ ID NO:13826 | SEQ ID NO:21838 |
| iPS:434673 | 21 225 74E3 | NA | AGCTATGGCATGCAC | GTTATTTGGTATGATGGAAGTAATAAATACTATGCAGACTCCGTGAAGGGC | GATCGCAATATAGTGGGAGCTACTTACTTTGAGTCC |

FIGURE 49

| | 21-225_74E3 | | SEQ ID NO:5815 | SEQ ID NO:13827 | SEQ ID NO:21839 |
|---|---|---|---|---|---|
| | | AA | SYGMH | VIWYDGSNKYYADSVKG | DRNIVGATYFES |
| | | | SEQ ID NO:5816 | SEQ ID NO:13828 | SEQ ID NO:21840 |
| iPS:434675 | | NA | AATTATGATATCAAC | TGGATGCACCCTAACAG TGGTAACACAGGCTTTG CACAGAAGTTCCAGGGC | TCCAGTGGCTGGTACTGGTT CGACCCC |
| | 21-225_79G6 | | SEQ ID NO:5817 | SEQ ID NO:13829 | SEQ ID NO:21841 |
| | | AA | NYDIN | WMHPNSGNTGFAQKFQG | SSGWYWFDP |
| | | | SEQ ID NO:5818 | SEQ ID NO:13830 | SEQ ID NO:21842 |
| iPS:434679 | | NA | AATTATGATATCAAC | TGGATGCACCCTAACAG TGGTAACACAGGCTATG CACAGAAGTTCCAGGGC | AGTAGTGGCTGGTACAAATT TGACTAC |
| | 21-225_79G7 | | SEQ ID NO:5819 | SEQ ID NO:13831 | SEQ ID NO:21843 |
| | | AA | NYDIN | WMHPNSGNTGYAQKFQG | SSGWYKFDY |
| | | | SEQ ID NO:5820 | SEQ ID NO:13832 | SEQ ID NO:21844 |
| iPS:434685 | | NA | AATTATGATATCAAC | TGGATGCATCCTAACAG TGGTAACACAGGCTATG CACAGAAGTTCCAGGGC | AGCAGTGGCTGGTACTACTT TGACTAC |
| | 21-225_79E9 | | SEQ ID NO:5821 | SEQ ID NO:13833 | SEQ ID NO:21845 |
| | | AA | NYDIN | WMHPNSGNTGYAQKFQG | SSGWYYFDY |
| | | | SEQ ID NO:5822 | SEQ ID NO:13834 | SEQ ID NO:21846 |
| iPS:434687 | | NA | GGTTGCTACTGGAGC | GAAATCAATCATAGTGG AAGCACCAACTACAACC CGTCCCTCAAGAGT | GACTACGGCGGTATGGACGT C |
| | 21-225_75A5 | | SEQ ID NO:5823 | SEQ ID NO:13835 | SEQ ID NO:21847 |
| | | AA | GCYWS | EINHSGSTNYNPSLKS | DYGGMDV |

FIGURE 49

| | | | SEQ ID NO:5824 | SEQ ID NO:13836 | SEQ ID NO:21848 |
|---|---|---|---|---|---|
| iPS:434689 | | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | 21-225_79G10 | | SEQ ID NO:5825 | SEQ ID NO:13837 | SEQ ID NO:21849 |
| | | AA | NYDIN | WMNPNSGNTGYAQKFQG | SSGWNWFDP |
| | | | SEQ ID NO:5826 | SEQ ID NO:13838 | SEQ ID NO:21850 |
| iPS:434691 | | NA | GGTTCCTACTGGAGC | GAAATCAATTATAGGGG AAGCACCAACTACAACC CGTCCCTCAAGAGT | GACTACGGCGGTATGGACGT C |
| | 21-225_75G7 | | SEQ ID NO:5827 | SEQ ID NO:13839 | SEQ ID NO:21851 |
| | | AA | GSYWS | EINYRGSTNYNPSLKS | DYGGMDV |
| | | | SEQ ID NO:5828 | SEQ ID NO:13840 | SEQ ID NO:21852 |
| iPS:434693 | | NA | GGTTGCTACTGGAGC | GAAATCAATCATAGTGG AAGCACCAACTACAACC CGTCCCTCAAGAGT | GACTACGGCGGTATGGACGT C |
| | 21-225_79F11 | | SEQ ID NO:5829 | SEQ ID NO:13841 | SEQ ID NO:21853 |
| | | AA | GCYWS | EINHSGSTNYNPSLKS | DYGGMDV |
| | | | SEQ ID NO:5830 | SEQ ID NO:13842 | SEQ ID NO:21854 |
| iPS:434697 | | NA | AATTATGATATCAAC | TGGATGAACCCTAACAG TGGTAACACAGGCTATG CACAGAAGTTCCAGGGC | AGCAGTGGCTGGTACTTCTT TGACTAC |
| | 21-225_79F12 | | SEQ ID NO:5831 | SEQ ID NO:13843 | SEQ ID NO:21855 |
| | | AA | NYDIN | WMNPNSGNTGYAQKFQG | SSGWYFFDY |
| | | | SEQ ID NO:5832 | SEQ ID NO:13844 | SEQ ID NO:21856 |
| iPS:434699 | 21 225 79G12 | NA | GGTTGCTACTGGAGC | GAAATCAATCATAGTGG AAGCACCAACTACAACC CGTCCCTCAAGAGT | GACTACGGCGGTATGGACGT C |

FIGURE 49

| | | | | | |
|---|---|---|---|---|---|
| | 21-225_79G12 | | SEQ ID NO:5833 | SEQ ID NO:13845 | SEQ ID NO:21857 |
| | | AA | GCYWS | EINHSGSTNYNPSLKS | DYGGMDV |
| | | | SEQ ID NO:5834 | SEQ ID NO:13846 | SEQ ID NO:21858 |
| iPS:434701 | 21-225_80A1 | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5835 | SEQ ID NO:13847 | SEQ ID NO:21859 |
| | | AA | GCYWS | EINHSGSTNYNPSLKS | DYGGMDV |
| | | | SEQ ID NO:5836 | SEQ ID NO:13848 | SEQ ID NO:21860 |
| iPS:434703 | 21-225_80C1 | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5837 | SEQ ID NO:13849 | SEQ ID NO:21861 |
| | | AA | GCYWS | EINHSGSTNYNPSLKS | DYGGMDV |
| | | | SEQ ID NO:5838 | SEQ ID NO:13850 | SEQ ID NO:21862 |
| iPS:434705 | 21-225_80A2 | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | | | SEQ ID NO:5839 | SEQ ID NO:13851 | SEQ ID NO:21863 |
| | | AA | NYDIN | WMHPNSGNTGYAQKFQG | SSGWYWFDP |
| | | | SEQ ID NO:5840 | SEQ ID NO:13852 | SEQ ID NO:21864 |
| iPS:434707 | 21-225_80D3 | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | | | SEQ ID NO:5841 | SEQ ID NO:13853 | SEQ ID NO:21865 |
| | | AA | NYDIN | WMHPNSGNTGYAQKFQG | SSGWYWFDP |
| | | | SEQ ID NO:5842 | SEQ ID NO:13854 | SEQ ID NO:21866 |

FIGURE 49

| iPS:434709 | | NA | GGTTGCTACTGGAGC | GAAATCAATCATAGTGG AAGCACCAACTACAACC CGTCCCTCAAGAGT | GACTACGGCGGTATGGACGT C |
|---|---|---|---|---|---|
| | 21-225_80E3 | | SEQ ID NO:5843 | SEQ ID NO:13855 | SEQ ID NO:21867 |
| | | AA | GCYWS | EINHSGSTNYNPSLKS | DYGGMDV |
| | | | SEQ ID NO:5844 | SEQ ID NO:13856 | SEQ ID NO:21868 |
| iPS:434711 | | NA | AATTATGATATCAAC | TGGATGAACCCTAACAG TGGTAACACAGGCTATG CACAGAAGTTCCAGGGC | ACCAGTGGCTGGAACTTCTT TGACTAC |
| | 21-225_80H3 | | SEQ ID NO:5845 | SEQ ID NO:13857 | SEQ ID NO:21869 |
| | | AA | NYDIN | WMNPNSGNTGYAQKFQG | TSGWNFFDY |
| | | | SEQ ID NO:5846 | SEQ ID NO:13858 | SEQ ID NO:21870 |
| iPS:434715 | | NA | GGTCCCTACTGGAGC | GAAATCAATCATAGTGG AAGCACCAACTACAACC CGTCCCTCAAGAGT | GACTACGGGGGTTTGGACGT C |
| | 21-225_80D5 | | SEQ ID NO:5847 | SEQ ID NO:13859 | SEQ ID NO:21871 |
| | | AA | GPYWS | EINHSGSTNYNPSLKS | DYGGLDV |
| | | | SEQ ID NO:5848 | SEQ ID NO:13860 | SEQ ID NO:21872 |
| iPS:434717 | | NA | GGTTGCTACTGGAGC | GAAATCAATCATAGTGG AAGGACCAACTACAACC CGTCCCTCAAGAGT | GACTACGGTGGGCTTGACTA C |
| | 21-225_80A6 | | SEQ ID NO:5849 | SEQ ID NO:13861 | SEQ ID NO:21873 |
| | | AA | GCYWS | EINHSGRTNYNPSLKS | DYGGLDY |
| | | | SEQ ID NO:5850 | SEQ ID NO:13862 | SEQ ID NO:21874 |
| iPS:434725 | | NA | GGTTCCTACTGGAGC | GAAATCAATCAAAGTGG ACGCACCAACTACAACC CGTCCCTCAAGAGT | GACTACGGGGGTATAGACGT C |
| | 21-225_80H7 | | SEQ ID NO:5851 | SEQ ID NO:13863 | SEQ ID NO:21875 |
| | | AA | GSYWS | EINQSGRTNYNPSLKS | DYGGIDV |

FIGURE 49

| | | | SEQ ID NO:5852 | SEQ ID NO:13864 | SEQ ID NO:21876 |
|---|---|---|---|---|---|
| iPS:434729 | | NA | AATTATGATATCAAC | TGGATGAACCCTAACAG TGGTAACACAGGCTATG CACAGAAGTTCCAGGTC | AGCAGTGGCTGGTACATCTT TGACTAC |
| | 21-225_80B12 | | SEQ ID NO:5853 | SEQ ID NO:13865 | SEQ ID NO:21877 |
| | | AA | NYDIN | WMNPNSGNTGYAQKFQV | SSGWYIFDY |
| | | | SEQ ID NO:5854 | SEQ ID NO:13866 | SEQ ID NO:21878 |
| iPS:434731 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | 21-225_80E9 | | SEQ ID NO:5855 | SEQ ID NO:13867 | SEQ ID NO:21879 |
| | | AA | NYDIN | WMHPNSGNTGYAQKFQG | SSGWYWFDP |
| | | | SEQ ID NO:5856 | SEQ ID NO:13868 | SEQ ID NO:21880 |
| iPS:434735 | | NA | GGTTGCTACTGGAGC | GAAATCAATCATAGTGG AAGCACCAACTACAACC CGTCCCTCAAGAGT | GACTACGGTGGGCTTGACTA C |
| | 21-225_80B10 | | SEQ ID NO:5857 | SEQ ID NO:13869 | SEQ ID NO:21881 |
| | | AA | GCYWS | EINHSGSTNYNPSLKS | DYGGLDY |
| | | | SEQ ID NO:5858 | SEQ ID NO:13870 | SEQ ID NO:21882 |
| iPS:434737 | | NA | AGCTATGGCATGCAC | GTTATATGGTATGATGG AAGTAATAAAAACTATG CAGACTCCGTGAAGGGC | GATGGCAGCTATGGTTATGA CGGCCTTGACTAC |
| | 21-225_74G6 | | SEQ ID NO:5859 | SEQ ID NO:13871 | SEQ ID NO:21883 |
| | | AA | SYGMH | VIWYDGSNKNYADSVKG | DGSYGYDGLDY |
| | | | SEQ ID NO:5860 | SEQ ID NO:13872 | SEQ ID NO:21884 |

FIGURE 49

| iPS:434741 | | NA | AGCTATGGCATGCAC | GTTATATGGTATGATGG AAGTAATAAAAACTATG CAGACTCCGTGAAGGGC | GATGGCAGCTATGGGTATGA CGGCCTTGACTAC |
|---|---|---|---|---|---|
| | 21-225_80C11 | | SEQ ID NO:5861 | SEQ ID NO:13873 | SEQ ID NO:21885 |
| | | AA | SYGMH | VIWYDGSNKNYADSVKG | DGSYGYDGLDY |
| | | | SEQ ID NO:5862 | SEQ ID NO:13874 | SEQ ID NO:21886 |
| iPS:434743 | | NA | GGTTGCTACTGGAGC | GAAATCAATCATAGTGG AAGCACCAACTACAACC CGTCCCTCAAGAGT | GACTACGGCGGTATGGACGT C |
| | 21-225_74A4 | | SEQ ID NO:5863 | SEQ ID NO:13875 | SEQ ID NO:21887 |
| | | AA | GCYWS | EINHSGSTNYNPSLKS | DYGGMDV |
| | | | SEQ ID NO:5864 | SEQ ID NO:13876 | SEQ ID NO:21888 |
| iPS:434747 | | NA | AATTATGATATCAAC | TGGATGCACCCTAACAA TGGTAACACAGGCTATG CACAGAAGTTCCAGGGC | AGCAGTGGCTGGTACTGGTT CGACCCC |
| | 21-225_80C12 | | SEQ ID NO:5865 | SEQ ID NO:13877 | SEQ ID NO:21889 |
| | | AA | NYDIN | WMHPNNGNTGYAQKFQG | SSGWYWFDP |
| | | | SEQ ID NO:5866 | SEQ ID NO:13878 | SEQ ID NO:21890 |
| iPS:434751 | | NA | GGTTGCTACTGGAGC | GAAATCAATCATAGTGG AAGCACCAACTACAACC CGTCCCTCAAGAGT | GACTACGGCGGTATGGACGT C |
| | 21-225_80H12 | | SEQ ID NO:5867 | SEQ ID NO:13879 | SEQ ID NO:21891 |
| | | AA | GCYWS | EINHSGSTNYNPSLKS | DYGGMDV |
| | | | SEQ ID NO:5868 | SEQ ID NO:13880 | SEQ ID NO:21892 |
| iPS:434759 | | NA | GGTTGCTACTGGAGC | GAAATCAATCATAGTGG AAGCACCAACTACAACC CGTCCCTCAAGAGT | GACTACGGCGGTATGGACGT C |
| | 21 225 81C5 | | | | |

FIGURE 49

| | 21-225_81C5 | | SEQ ID NO:5869 | SEQ ID NO:13881 | SEQ ID NO:21893 |
|---|---|---|---|---|---|
| | | AA | GCYWS | EINHSGSTNYNPSLKS | DYGGMDV |
| | | | SEQ ID NO:5870 | SEQ ID NO:13882 | SEQ ID NO:21894 |
| iPS:434761 | | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | 21-225_81E5 | | SEQ ID NO:5871 | SEQ ID NO:13883 | SEQ ID NO:21895 |
| | | AA | NYDIN | WMNPNSGNTGYAQKFQG | SSGWNWFDP |
| | | | SEQ ID NO:5872 | SEQ ID NO:13884 | SEQ ID NO:21896 |
| iPS:434771 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | 21-225_81F9 | | SEQ ID NO:5873 | SEQ ID NO:13885 | SEQ ID NO:21897 |
| | | AA | NYDIN | WMHPNSGNTGYAQKFQG | SSGWYWFDP |
| | | | SEQ ID NO:5874 | SEQ ID NO:13886 | SEQ ID NO:21898 |
| iPS:434773 | | NA | GGTCCCTACTGGAGC | GAAATCAATTATAGGGG AAGCACCAACTACAACC CGTCCCTCAAGAGT | GACTACGGCGGTATGGACGT C |
| | 21-225_75D9 | | SEQ ID NO:5875 | SEQ ID NO:13887 | SEQ ID NO:21899 |
| | | AA | GPYWS | EINYRGSTNYNPSLKS | DYGGMDV |
| | | | SEQ ID NO:5876 | SEQ ID NO:13888 | SEQ ID NO:21900 |
| iPS:434777 | | NA | GGTTGCTACTGGAGC | GAAATCAATCATAGTGG AAGCACCAACTACAACC CGTCCCTCAAGAGT | GACTACGGCGGTATGGACGT C |
| | 21-225_81C11 | | SEQ ID NO:5877 | SEQ ID NO:13889 | SEQ ID NO:21901 |
| | | AA | GCYWS | EINHSGSTNYNPSLKS | DYGGMDV |
| | | | SEQ ID NO:5878 | SEQ ID NO:13890 | SEQ ID NO:21902 |

FIGURE 49

| iPS:434793 | | NA | AATTATGATATCAAC | TGGATGCACCCTAACAA TGGTAACACAGGCTATG CACAGAAGTTCCAGGGC | AGCAGTGGCTGGTACTGGTT CGACCCC |
|---|---|---|---|---|---|
| | 21-225_82A5 | | SEQ ID NO:5879 | SEQ ID NO:13891 | SEQ ID NO:21903 |
| | | AA | NYDIN | WMHPNNGNTGYAQKFQG | SSGWYWFDP |
| | | | SEQ ID NO:5880 | SEQ ID NO:13892 | SEQ ID NO:21904 |
| iPS:434797 | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | 21-225_82G5 | | SEQ ID NO:5881 | SEQ ID NO:13893 | SEQ ID NO:21905 |
| | | AA | NYDIN | WMHPNSGNTGYAQKFQG | SSGWYWFDP |
| | | | SEQ ID NO:5882 | SEQ ID NO:13894 | SEQ ID NO:21906 |
| iPS:434805 | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | 21-225_82D9 | | SEQ ID NO:5883 | SEQ ID NO:13895 | SEQ ID NO:21907 |
| | | AA | NYDIN | WMHPNSGNTGYAQKFQG | SSGWYWFDP |
| | | | SEQ ID NO:5884 | SEQ ID NO:13896 | SEQ ID NO:21908 |
| iPS:434809 | | NA | GGTTGCTACTGGAGC | GAAATCAATCATAGTGG AAGCACCAACTACAACC CGTCCCTCAAGAGT | GACTACGGCGGTATGGACGT C |
| | 21-225_74F5 | | SEQ ID NO:5885 | SEQ ID NO:13897 | SEQ ID NO:21909 |
| | | AA | GCYWS | EINHSGSTNYNPSLKS | DYGGMDV |
| | | | SEQ ID NO:5886 | SEQ ID NO:13898 | SEQ ID NO:21910 |
| iPS:434813 | | NA | AATTATGATATCAAC | TGGATGCACCCTAACAA TGGTAACACAGGCTATG CACAGAAGTTCCAGGGC | AGCAGTGGCTGGTACTGGTT CGACCCC |

FIGURE 49

| | 21-225_82C12 | | SEQ ID NO:5887 | SEQ ID NO:13899 | SEQ ID NO:21911 |
|---|---|---|---|---|---|
| | | AA | NYDIN | WMHPNNGNTGYAQKFQG | SSGWYWFDP |
| | | | SEQ ID NO:5888 | SEQ ID NO:13900 | SEQ ID NO:21912 |
| iPS:434815 | | NA | AGTTATGATATCAAC | TGGATGAACCCTAACAGTGGTAACACAGGCTATGCACAGAAGTTCCAGGGC | GGCTTTTACGATACTTTGACTGGTTCCGGCTACTACTACGTTATGGACGTC |
| | 21-225_74A11 | | SEQ ID NO:5889 | SEQ ID NO:13901 | SEQ ID NO:21913 |
| | | AA | SYDIN | WMNPNSGNTGYAQKFQG | GFYDTLTGSGYYYVMDV |
| | | | SEQ ID NO:5890 | SEQ ID NO:13902 | SEQ ID NO:21914 |
| iPS:434821 | | NA | GGTTGCTACTGGAGC | GAAATCAATCATAGTGGAAGCACCAACTACAACCCGTCCCTCAAGAGT | GACTACGGCGGTATGGACGTC |
| | 21-225_83G1 | | SEQ ID NO:5891 | SEQ ID NO:13903 | SEQ ID NO:21915 |
| | | AA | GCYWS | EINHSGSTNYNPSLKS | DYGGMDV |
| | | | SEQ ID NO:5892 | SEQ ID NO:13904 | SEQ ID NO:21916 |
| iPS:434825 | | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | 21-225_83C2 | | SEQ ID NO:5893 | SEQ ID NO:13905 | SEQ ID NO:21917 |
| | | AA | NYDIN | WMHPNSGNTGYAQKFQG | SSGWYWFDP |
| | | | SEQ ID NO:5894 | SEQ ID NO:13906 | SEQ ID NO:21918 |
| iPS:434827 | | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | 21-225_83F3 | | SEQ ID NO:5895 | SEQ ID NO:13907 | SEQ ID NO:21919 |
| | | AA | NYDIN | WMHPNSGNTGYAQKFQG | SSGWYWFDP |

FIGURE 49

| | | | SEQ ID NO:5896 | SEQ ID NO:13908 | SEQ ID NO:21920 |
|---|---|---|---|---|---|
| iPS:434829 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | 21-225_83G3 | | SEQ ID NO:5897 | SEQ ID NO:13909 | SEQ ID NO:21921 |
| | | AA | NYDIN | WMHPNSGNTGYAQKFQG | SSGWYWFDP |
| | | | SEQ ID NO:5898 | SEQ ID NO:13910 | SEQ ID NO:21922 |
| iPS:434833 | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | 21-225_83C5 | | SEQ ID NO:5899 | SEQ ID NO:13911 | SEQ ID NO:21923 |
| | | AA | NYDIN | WMHPNSGNTGYAQKFQG | SSGWYWFDP |
| | | | SEQ ID NO:5900 | SEQ ID NO:13912 | SEQ ID NO:21924 |
| iPS:434835 | | NA | GGTTGTTACTGGAGC | GAAATCAATCATAGTGG AAGGACCAACTACAACC CCTCCCTCAAGAGT | GACTACGGTGGGCTTGACTA C |
| | 21-225_83B6 | | SEQ ID NO:5901 | SEQ ID NO:13913 | SEQ ID NO:21925 |
| | | AA | GCYWS | EINHSGRTNYNPSLKS | DYGGLDY |
| | | | SEQ ID NO:5902 | SEQ ID NO:13914 | SEQ ID NO:21926 |
| iPS:434839 | | NA | GGTTGCTACTGGAGC | GAAATCAATCATAGTGG AAGCACCAACTACAACC CGTCCCTCAAGAGT | GACTACGGCGGTATGGACGT C |
| | 21-225_83B7 | | SEQ ID NO:5903 | SEQ ID NO:13915 | SEQ ID NO:21927 |
| | | AA | GCYWS | EINHSGSTNYNPSLKS | DYGGMDV |
| | | | SEQ ID NO:5904 | SEQ ID NO:13916 | SEQ ID NO:21928 |
| iPS:434841 | | NA | AATTATGATATCAAC | TGGATGAACCCTAACAG TGGTAACACAGGCTATG CACAGAAGTTCCAGGGC | TCCAGTGGCTGGCACTGGTT CGACCCC |

FIGURE 49

| | 21-225_83G7 | | SEQ ID NO:5905 | SEQ ID NO:13917 | SEQ ID NO:21929 |
|---|---|---|---|---|---|
| | | AA | NYDIN | WMNPNSGNTGYAQKFQG | SSGWHWFDP |
| | | | SEQ ID NO:5906 | SEQ ID NO:13918 | SEQ ID NO:21930 |
| iPS:434849 | 21-225_83C10 | NA | GGTTACTACTGGAGC | GAAATCAATCATAGTGG AAGCACCAACTTCAACC CGTCCCTCAAGAGT | GACTACGGTGGGCTTGACTA C |
| | | | SEQ ID NO:5907 | SEQ ID NO:13919 | SEQ ID NO:21931 |
| | | AA | GYYWS | EINHSGSTNFNPSLKS | DYGGLDY |
| | | | SEQ ID NO:5908 | SEQ ID NO:13920 | SEQ ID NO:21932 |
| iPS:434851 | 21-225_75A6 | NA | AATTATGATATCAAC | TGGATGAACCCTAACAG TGGTAACACAGGCTATG CACAGAAGTTCCAGGGC | AGCAGTGGCTGGTACATCTT TGACTAC |
| | | | SEQ ID NO:5909 | SEQ ID NO:13921 | SEQ ID NO:21933 |
| | | AA | NYDIN | WMNPNSGNTGYAQKFQG | SSGWYIFDY |
| | | | SEQ ID NO:5910 | SEQ ID NO:13922 | SEQ ID NO:21934 |
| iPS:434863 | 21-225_84G7 | NA | AATTATGATATCAAC | TGGATGAACCCTAACAG TGGTAACACAGGCTATG CACAGAAGTTCCAGGAC | TCCAGTGGCTGGCACTGGTT CGACCCC |
| | | | SEQ ID NO:5911 | SEQ ID NO:13923 | SEQ ID NO:21935 |
| | | AA | NYDIN | WMNPNSGNTGYAQKFQD | SSGWHWFDP |
| | | | SEQ ID NO:5912 | SEQ ID NO:13924 | SEQ ID NO:21936 |
| iPS:434867 | 21-225_79A12 | NA | AGCTATGGCATGCAC | GTTATATGGTATGATGG AAGTAATAAAAACTATG CAGACTCCGTGAAGGGC | GATGGCAGCTATGGTTACGA CGGCCTTGACTAC |
| | | | SEQ ID NO:5913 | SEQ ID NO:13925 | SEQ ID NO:21937 |

FIGURE 49

| | | AA | SYGMH | VIWYDGSNKNYADSVKG | DGSYGYDGLDY |
|---|---|---|---|---|---|
| | | | SEQ ID NO:5914 | SEQ ID NO:13926 | SEQ ID NO:21938 |
| iPS:434869 | 21-225_84E12 | NA | GGTTCCTACTGGAGC | GAAATCAATCAAAGTGG ACGCACCAACTACAACC CGTCCCTCAAGAGT | GACTACGGGGGTATAGACGT C |
| | | | SEQ ID NO:5915 | SEQ ID NO:13927 | SEQ ID NO:21939 |
| | | AA | GSYWS | EINQSGRTNYNPSLKS | DYGGIDV |
| | | | SEQ ID NO:5916 | SEQ ID NO:13928 | SEQ ID NO:21940 |
| iPS:434871 | 21-225_85H1 | NA | AGCTATGGCATACAC | GTTATATGGTATGATGG AAGTAATAAATACTATG CAGACTCCGTGAAGGGC | GATCCCTTTATAGTGGGAGC TACTTACTTTGACTAC |
| | | | SEQ ID NO:5917 | SEQ ID NO:13929 | SEQ ID NO:21941 |
| | | AA | SYGIH | VIWYDGSNKYYADSVKG | DPFIVGATYFDY |
| | | | SEQ ID NO:5918 | SEQ ID NO:13930 | SEQ ID NO:21942 |
| iPS:434877 | 21-225_85H2 | NA | AATTATGATATCAAC | TGGATGCACCCTAATAG TGGTAACACAGGCTATG CACAGAAGTTCCAGGGC | TCCAGTGGCTGGTACTGGTT CGACCCC |
| | | | SEQ ID NO:5919 | SEQ ID NO:13931 | SEQ ID NO:21943 |
| | | AA | NYDIN | WMHPNSGNTGYAQKFQG | SSGWYWFDP |
| | | | SEQ ID NO:5920 | SEQ ID NO:13932 | SEQ ID NO:21944 |
| iPS:434879 | 21-225_85A3 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5921 | SEQ ID NO:13933 | SEQ ID NO:21945 |
| | | AA | GCYWS | EINHSGSTNYNPSLKS | DYGGMDV |
| | | | SEQ ID NO:5922 | SEQ ID NO:13934 | SEQ ID NO:21946 |

FIGURE 49

| iPS:434881 | | NA | GGTTGCTACTGGAGC | GAAATCAATCATAGTGG AAGCACCAACTACAACC CGTCCCTCAAGAGT | GACTACGGCGGTATGGACGT C |
|---|---|---|---|---|---|
| | 21-225_85B4 | | SEQ ID NO:5923 | SEQ ID NO:13935 | SEQ ID NO:21947 |
| | | AA | GCYWS | EINHSGSTNYNPSLKS | DYGGMDV |
| | | | SEQ ID NO:5924 | SEQ ID NO:13936 | SEQ ID NO:21948 |
| iPS:434883 | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | 21-225_85B5 | | SEQ ID NO:5925 | SEQ ID NO:13937 | SEQ ID NO:21949 |
| | | AA | NYDIN | WMHPNSGNTGYAQKFQG | SSGWYWFDP |
| | | | SEQ ID NO:5926 | SEQ ID NO:13938 | SEQ ID NO:21950 |
| iPS:434887 | | NA | GGTTGCTACTGGAGC | GAAATCAATTATAGTGG AAGAACCAACTACAACC CGTCCCTCAAGAGT | GACTACGGCGGTATGGACGT C |
| | 21-225_85D6 | | SEQ ID NO:5927 | SEQ ID NO:13939 | SEQ ID NO:21951 |
| | | AA | GCYWS | EINYSGRTNYNPSLKS | DYGGMDV |
| | | | SEQ ID NO:5928 | SEQ ID NO:13940 | SEQ ID NO:21952 |
| iPS:434891 | | NA | GATTGCTACTGGAGC | GAAATCAATCATAGTGG AAGGACCAACTACAACC CGTCCCTCAAGAGT | GACTACGGTGGGCTTGACTA C |
| | 21-225_85G6 | | SEQ ID NO:5929 | SEQ ID NO:13941 | SEQ ID NO:21953 |
| | | AA | DCYWS | EINHSGRTNYNPSLKS | DYGGLDY |
| | | | SEQ ID NO:5930 | SEQ ID NO:13942 | SEQ ID NO:21954 |
| iPS:434895 | | NA | GGTCCCTACTGGAGC | GAAATCAATCATAGTGG AAGCACCAACTACAACC CGTCCCTCAAGAGT | GACTACGGGGGGTTTGGACGT C |
| | 21-225_74H7 | | SEQ ID NO:5931 | SEQ ID NO:13943 | SEQ ID NO:21955 |
| | | AA | GPYWS | EINHSGSTNYNPSLKS | DYGGLDV |

FIGURE 49

| | | | SEQ ID NO:5932 | SEQ ID NO:13944 | SEQ ID NO:21956 |
|---|---|---|---|---|---|
| iPS:434899 | | NA | GGTTGCTACTGGAGC | GAAATCAATCATAGTGG AAGGACCAACTTCAACC CGTCCCTCAAGAGT | GACTACGGCGGTATGGACGT C |
| | 21-225_85B9 | | SEQ ID NO:5933 | SEQ ID NO:13945 | SEQ ID NO:21957 |
| | | AA | GCYWS | EINHSGRTNFNPSLKS | DYGGMDV |
| | | | SEQ ID NO:5934 | SEQ ID NO:13946 | SEQ ID NO:21958 |
| iPS:434901 | | NA | AATTATGATATCAAC | TGGATGAACCCTAACAG TGGTAACACAGGCTATG CACAGAAGTTCCAGGGC | ACCAGTGGCTGGAACTTCTT TGACTAC |
| | 21-225_85H9 | | SEQ ID NO:5935 | SEQ ID NO:13947 | SEQ ID NO:21959 |
| | | AA | NYDIN | WMNPNSGNTGYAQKFQG | TSGWNFFDY |
| | | | SEQ ID NO:5936 | SEQ ID NO:13948 | SEQ ID NO:21960 |
| iPS:434907 | | NA | GGTTGTTACTGGAGC | GAAATCAATCATAGTGG AATCACCAACTACAACC CGTCCCTCAAGAGT | GACTACGGTGGTTTGGACGT C |
| | 21-225_85G10 | | SEQ ID NO:5937 | SEQ ID NO:13949 | SEQ ID NO:21961 |
| | | AA | GCYWS | EINHSGITNYNPSLKS | DYGGLDV |
| | | | SEQ ID NO:5938 | SEQ ID NO:13950 | SEQ ID NO:21962 |
| iPS:434909 | | NA | AATTATGATATCAAC | TGGATGCACCCTAACAG TGGTAACACAGGCTATG CACAGAAGTTCCAGGGC | AGTAGTGGCTGGTACAAATT TGACTAC |
| | 21-225_85C11 | | SEQ ID NO:5939 | SEQ ID NO:13951 | SEQ ID NO:21963 |
| | | AA | NYDIN | WMHPNSGNTGYAQKFQG | SSGWYKFDY |
| | | | SEQ ID NO:5940 | SEQ ID NO:13952 | SEQ ID NO:21964 |
| iPS:434911 | | NA | AATTATGATATCAAC | TGGATGCACCCTAACAG TGGTAACACAGGCTATG CACAGAAGTTCCAGGGC | AGTAGTGGCTGGTACTGGTT CGACCCC |

FIGURE 49

| | 21-225_85D11 | | SEQ ID NO:5941 | SEQ ID NO:13953 | SEQ ID NO:21965 |
|---|---|---|---|---|---|
| | | AA | NYDIN | WMHPNSGNTGYAQKFQG | SSGWYWFDP |
| | | | SEQ ID NO:5942 | SEQ ID NO:13954 | SEQ ID NO:21966 |
| iPS:434913 | 21-225_86C1 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5943 | SEQ ID NO:13955 | SEQ ID NO:21967 |
| | | AA | GCYWS | EINHSGSTNYNPSLKS | DYGGMDV |
| | | | SEQ ID NO:5944 | SEQ ID NO:13956 | SEQ ID NO:21968 |
| iPS:434921 | 21-225_86E4 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5945 | SEQ ID NO:13957 | SEQ ID NO:21969 |
| | | AA | GCYWS | EINHSGSTNYNPSLKS | DYGGMDV |
| | | | SEQ ID NO:5946 | SEQ ID NO:13958 | SEQ ID NO:21970 |
| iPS:434935 | 21-225_86E9 | NA | AATTATGATATCAAC | TGGATGAACCCTAACAG TGGTAACACAGGCTATG CACAGAAGTTCCAGGGC | TCCAGTGGCTGGTCCTGGTT CGACCCC |
| | | | SEQ ID NO:5947 | SEQ ID NO:13959 | SEQ ID NO:21971 |
| | | AA | NYDIN | WMNPNSGNTGYAQKFQG | SSGWSWFDP |
| | | | SEQ ID NO:5948 | SEQ ID NO:13960 | SEQ ID NO:21972 |
| iPS:434939 | 21-225_86C11 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5949 | SEQ ID NO:13961 | SEQ ID NO:21973 |
| | | AA | GCYWS | EINHSGSTNYNPSLKS | DYGGMDV |
| | | | SEQ ID NO:5950 | SEQ ID NO:13962 | SEQ ID NO:21974 |

FIGURE 49

| iPS:434943 | | NA | GGTTACTACTGGAGC | GAAATCAATCATAGTGG ACGCACCAACTACAACC CGTCCCTCAAGAGT | GACTACGGTGGTTTGGACGT C |
|---|---|---|---|---|---|
| | 21-225_87H1 | | SEQ ID NO:5951 | SEQ ID NO:13963 | SEQ ID NO:21975 |
| | | AA | GYYWS | EINHSGRTNYNPSLKS | DYGGLDV |
| | | | SEQ ID NO:5952 | SEQ ID NO:13964 | SEQ ID NO:21976 |
| iPS:434945 | | NA | GGTTGCTACTGGAGC | GAAATCAATCATAGTGG AAGCACCAACTACAACC CGTCCCTCAAGAGT | GACTACGGCGGTATGGACGT C |
| | 21-225_87E5 | | SEQ ID NO:5953 | SEQ ID NO:13965 | SEQ ID NO:21977 |
| | | AA | GCYWS | EINHSGSTNYNPSLKS | DYGGMDV |
| | | | SEQ ID NO:5954 | SEQ ID NO:13966 | SEQ ID NO:21978 |
| iPS:434947 | | NA | AGCTATGGCATGCAC | GTTATATGGTATGATGG AAGTAATAAAAACTATG CAGACTCCGTGAAGGGC | GATTTTGGAGTGGGCTACTA CGGTATGGACGTC |
| | 21-225_87B7 | | SEQ ID NO:5955 | SEQ ID NO:13967 | SEQ ID NO:21979 |
| | | AA | SYGMH | VIWYDGSNKNYADSVKG | DFGVGYYGMDV |
| | | | SEQ ID NO:5956 | SEQ ID NO:13968 | SEQ ID NO:21980 |
| iPS:434955 | | NA | GGTTGCTACTGGAGC | GAAATCAATCATAGTGG AAGCACCAACTACAACC CGTCCCTCAAGAGT | GACTACGGCGGTATGGACGT C |
| | 21-225_87C9 | | SEQ ID NO:5957 | SEQ ID NO:13969 | SEQ ID NO:21981 |
| | | AA | GCYWS | EINHSGSTNYNPSLKS | DYGGMDV |
| | | | SEQ ID NO:5958 | SEQ ID NO:13970 | SEQ ID NO:21982 |
| iPS:434957 | | NA | AATTATGATATCAAC | TGGATGCACCCTAACAA TGGTAACACAGGCTATG CACAGAAGTTCCAGGGC | AGCAGTGGCTGGTACTGGTT CGACCCC |
| | 21-225_87A10 | | SEQ ID NO:5959 | SEQ ID NO:13971 | SEQ ID NO:21983 |

FIGURE 49

| | | | | | |
|---|---|---|---|---|---|
| | | AA | NYDIN | WMHPNNGNTGYAQKFQG | SSGWYWFDP |
| | | | SEQ ID NO:5960 | SEQ ID NO:13972 | SEQ ID NO:21984 |
| iPS:434959 | 21-225_87E10 | NA | AATTATGATATCAAC | TGGATGAACCCTAACAGTGGTAACACAGGCTATGCACAGAAGTTCCAGGGC | AGCAGTGGCTGGTACTTCTTTGACTAC |
| | | | SEQ ID NO:5961 | SEQ ID NO:13973 | SEQ ID NO:21985 |
| | | AA | NYDIN | WMNPNSGNTGYAQKFQG | SSGWYFFDY |
| | | | SEQ ID NO:5962 | SEQ ID NO:13974 | SEQ ID NO:21986 |
| iPS:434961 | 21-225_87A12 | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5963 | SEQ ID NO:13975 | SEQ ID NO:21987 |
| | | AA | GCYWS | EINHSGSTNYNPSLKS | DYGGMDV |
| | | | SEQ ID NO:5964 | SEQ ID NO:13976 | SEQ ID NO:21988 |
| iPS:434965 | 21-225_88A1 | NA | AATTATGATATCAAC | TGGATGAACCCTAACAGTGGTAACACAGGCTATGCACAGAAGTTCCAGGGC | AGCAGTGGCTGGTACTACTTTGACTAC |
| | | | SEQ ID NO:5965 | SEQ ID NO:13977 | SEQ ID NO:21989 |
| | | AA | NYDIN | WMNPNSGNTGYAQKFQG | SSGWYYFDY |
| | | | SEQ ID NO:5966 | SEQ ID NO:13978 | SEQ ID NO:21990 |
| iPS:434969 | 21-225_88H1 | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5967 | SEQ ID NO:13979 | SEQ ID NO:21991 |
| | | AA | GCYWS | EINHSGSTNYNPSLKS | DYGGMDV |
| | | | SEQ ID NO:5968 | SEQ ID NO:13980 | SEQ ID NO:21992 |

FIGURE 49

| iPS:434971 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
|---|---|---|---|---|---|
| | 21-225_88G2 | | SEQ ID NO:5969 | SEQ ID NO:13981 | SEQ ID NO:21993 |
| | | AA | NYDIN | WMHPNSGNTGYAQKFQG | SSGWYWFDP |
| | | | SEQ ID NO:5970 | SEQ ID NO:13982 | SEQ ID NO:21994 |
| iPS:434973 | | NA | AATTATGATATCAAC | TGGATGAACCCTAACAG TGGTGACACAGGCTATG CACAGAAGTTCCAGGGC | AGCAGTGGCTGGTACTACTT TGACTAC |
| | 21-225_88B4 | | SEQ ID NO:5971 | SEQ ID NO:13983 | SEQ ID NO:21995 |
| | | AA | NYDIN | WMNPNSGDTGYAQKFQG | SSGWYYFDY |
| | | | SEQ ID NO:5972 | SEQ ID NO:13984 | SEQ ID NO:21996 |
| iPS:434977 | | NA | AGTTATGATATCAAC | TGGATGAACCCTAACAG TGGTAACACAGGCTATG CACAGAAGTTCCAGGGC | GGGTTTTACGATTTTTTGACT GGTTATTCCCCCACCTACTA CTACTACGATATGGACGTC |
| | 21-225_88A5 | | SEQ ID NO:5973 | SEQ ID NO:13985 | SEQ ID NO:21997 |
| | | AA | SYDIN | WMNPNSGNTGYAQKFQG | GFYDFLTGYSPTYYYYDMDV |
| | | | SEQ ID NO:5974 | SEQ ID NO:13986 | SEQ ID NO:21998 |
| iPS:434981 | | NA | GGTTGCTACTGGAGC | GAAATCAATCATAGTGG AAGCACCAACTACAACC CGTCCCTCAAGAGT | GACTACGGCGGTATGGACGT C |
| | 21-225_88E7 | | SEQ ID NO:5975 | SEQ ID NO:13987 | SEQ ID NO:21999 |
| | | AA | GCYWS | EINHSGSTNYNPSLKS | DYGGMDV |
| | | | SEQ ID NO:5976 | SEQ ID NO:13988 | SEQ ID NO:22000 |

FIGURE 49

| iPS:434983 | | NA | GGTTGCTACTGGAGC | GAAATCAATCATAGTGG AAGCACCAACTACAACC CGTCCCTCAAGAGT | GACTACGGCGGTATGGACGT C |
| --- | --- | --- | --- | --- | --- |
| | 21-225_88F7 | | SEQ ID NO:5977 | SEQ ID NO:13989 | SEQ ID NO:22001 |
| | | AA | GCYWS | EINHSGSTNYNPSLKS | DYGGMDV |
| | | | SEQ ID NO:5978 | SEQ ID NO:13990 | SEQ ID NO:22002 |
| iPS:434995 | | NA | GGTTGCTACTGGAGC | GAAATCAATCATAGTGG AAGCACCAACTACAACC CGTCCCTCAAGAGT | GACTACGGCGGTATGGACGT C |
| | 21-225_88G9 | | SEQ ID NO:5979 | SEQ ID NO:13991 | SEQ ID NO:22003 |
| | | AA | GCYWS | EINHSGSTNYNPSLKS | DYGGMDV |
| | | | SEQ ID NO:5980 | SEQ ID NO:13992 | SEQ ID NO:22004 |
| iPS:434997 | | NA | AATTATGATATCAAC | TGGATGACCCCTAACAG TGGTAACACAGGCTATG CACAGAAGTTCCAGGGC | AGCAGTGGCTGGTACTACTT TGACTCC |
| | 21-225_88C10 | | SEQ ID NO:5981 | SEQ ID NO:13993 | SEQ ID NO:22005 |
| | | AA | NYDIN | WMTPNSGNTGYAQKFQG | SSGWYYFDS |
| | | | SEQ ID NO:5982 | SEQ ID NO:13994 | SEQ ID NO:22006 |
| iPS:434999 | | NA | GGTTGCTACTGGAGC | GAAATCAATCATAGTGG AAGCACCAACTACAACC CGTCCCTCAAGAGT | GACTACGGCGGTATGGACGT C |
| | 21-225_75A8 | | SEQ ID NO:5983 | SEQ ID NO:13995 | SEQ ID NO:22007 |
| | | AA | GCYWS | EINHSGSTNYNPSLKS | DYGGMDV |
| | | | SEQ ID NO:5984 | SEQ ID NO:13996 | SEQ ID NO:22008 |
| iPS:435009 | | NA | AGCTACGACATGCAC | GCTATTGGTACTGCTGGT GACACATACTATCCAGG CTCCGTGAAGGGC | GCTCTTGACTACGGTGACTC CTTGGGCTACTACTACTACG GTATGGACGTC |
| | 21-225_89G4 | | SEQ ID NO:5985 | SEQ ID NO:13997 | SEQ ID NO:22009 |
| | | AA | SYDMH | AIGTAGDTYYPGSVKG | ALDYGDSLGYYYYGMDV |

FIGURE 49

| | | | SEQ ID NO:5986 | SEQ ID NO:13998 | SEQ ID NO:22010 |
|---|---|---|---|---|---|
| iPS:435013 | 21-225_89D5 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5987 | SEQ ID NO:13999 | SEQ ID NO:22011 |
| | | AA | GCYWS | EINHSGSTNYNPSLKS | DYGGMDV |
| | | | SEQ ID NO:5988 | SEQ ID NO:14000 | SEQ ID NO:22012 |
| iPS:435015 | 21-225_89H5 | NA | GGTTGCTACTGGAGC | GAAATCAATTATAGTGG AAGCACCAACTTCAACC CGTCCCTCAAGAGT | GACTACGGCGGTATGGACGT C |
| | | | SEQ ID NO:5989 | SEQ ID NO:14001 | SEQ ID NO:22013 |
| | | AA | GCYWS | EINYSGSTNFNPSLKS | DYGGMDV |
| | | | SEQ ID NO:5990 | SEQ ID NO:14002 | SEQ ID NO:22014 |
| iPS:435025 | 21-225_89E10 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5991 | SEQ ID NO:14003 | SEQ ID NO:22015 |
| | | AA | GCYWS | EINHSGSTNYNPSLKS | DYGGMDV |
| | | | SEQ ID NO:5992 | SEQ ID NO:14004 | SEQ ID NO:22016 |
| iPS:435029 | 21-225_89A11 | NA | GGTTACTACTGGAGC | GAAATCAATCATAGTGG ACGCACCAGCTACAACC CGTCCCTCAAGAGT | GACTACGGTGGTTTGGACGT C |
| | | | SEQ ID NO:5993 | SEQ ID NO:14005 | SEQ ID NO:22017 |
| | | AA | GYYWS | EINHSGRTSYNPSLKS | DYGGLDV |
| | | | SEQ ID NO:5994 | SEQ ID NO:14006 | SEQ ID NO:22018 |
| iPS:435039 | 21-225_90G4 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5995 | SEQ ID NO:14007 | SEQ ID NO:22019 |
| | | AA | GCYWS | EINHSGSTNYNPSLKS | DYGGMDV |

FIGURE 49

| | | | SEQ ID NO:5996 | SEQ ID NO:14008 | SEQ ID NO:22020 |
|---|---|---|---|---|---|
| iPS:435041 | | NA | GGTTGCTACTGGAGC | GAAATCAATCATAGTGG AAGCACCAACTACAACC CGTCCCTCAAGAGT | GACTACGGCGGTATGGACGT C |
| | 21-225_90A5 | | SEQ ID NO:5997 | SEQ ID NO:14009 | SEQ ID NO:22021 |
| | | AA | GCYWS | EINHSGSTNYNPSLKS | DYGGMDV |
| | | | SEQ ID NO:5998 | SEQ ID NO:14010 | SEQ ID NO:22022 |
| iPS:435043 | | NA | GGTTGCTACTGGAGC | GAAATCAATCATAGTGG AAGCACCAACTACAACC CGTCCCTCAAGAGT | GACTACGGCGGTATGGACGT C |
| | 21-225_90G5 | | SEQ ID NO:5999 | SEQ ID NO:14011 | SEQ ID NO:22023 |
| | | AA | GCYWS | EINHSGSTNYNPSLKS | DYGGMDV |
| | | | SEQ ID NO:6000 | SEQ ID NO:14012 | SEQ ID NO:22024 |
| iPS:435045 | | NA | GACTATGGCATGCAC | GTTATATGGTATGAAGG AAGTAATACATACTATG CAGACTCCGTGAAGGGC | GAGATGGGGTGGTTAGATGA CTAC |
| | 21-225_90H5 | | SEQ ID NO:6001 | SEQ ID NO:14013 | SEQ ID NO:22025 |
| | | AA | DYGMH | VIWYEGSNTYYADSVKG | EMGWLDDY |
| | | | SEQ ID NO:6002 | SEQ ID NO:14014 | SEQ ID NO:22026 |
| iPS:435051 | | NA | AATTATGATATCAAC | TGGATGAACCCTAACAG TGGTAACACAGGCTATG CACAGAAGTTCCAGGGC | TCCAGTGGCTGGCACTGGTT CGACCCC |
| | 21-225_90D9 | | SEQ ID NO:6003 | SEQ ID NO:14015 | SEQ ID NO:22027 |
| | | AA | NYDIN | WMNPNSGNTGYAQKFQG | SSGWHWFDP |
| | | | SEQ ID NO:6004 | SEQ ID NO:14016 | SEQ ID NO:22028 |
| iPS:435053 | | NA | AATTATGATATCAAC | TGGATGCACCCTAACAG TGGTAACACAGGCTATG CACAGAAGTTCCAGGGC | AGCAGTGGCTGGTACATCTT TGACTAC |
| | 21-225_75F9 | | SEQ ID NO:6005 | SEQ ID NO:14017 | SEQ ID NO:22029 |

FIGURE 49

| | | AA | NYDIN | WMHPNSGNTGYAQKFQG | SSGWYIFDY |
|---|---|---|---|---|---|
| | | | SEQ ID NO:6006 | SEQ ID NO:14018 | SEQ ID NO:22030 |
| iPS:435055 | | NA | GGTTGCTACTGGAGC | GAAATCAATCATAGTGG AAGCACCAACTACAACC CGTCCCTCAAGAGT | GACTACGGCGGTATGGACGT C |
| | 21-225_90F10 | | SEQ ID NO:6007 | SEQ ID NO:14019 | SEQ ID NO:22031 |
| | | AA | GCYWS | EINHSGSTNYNPSLKS | DYGGMDV |
| | | | SEQ ID NO:6008 | SEQ ID NO:14020 | SEQ ID NO:22032 |
| iPS:435059 | | NA | AACTACGACATGCAC | GCTATTGGTACTGCTGGT GACACATACTATCCAGG CTCCGTGAAGGGC | GTTCTTGACTACGGTGACTC CTTGGGCTACTACTACTACG GTATGGACGTC |
| | 21-225_90C11 | | SEQ ID NO:6009 | SEQ ID NO:14021 | SEQ ID NO:22033 |
| | | AA | NYDMH | AIGTAGDTYYPGSVKG | VLDYGDSLGYYYYGMDV |
| | | | SEQ ID NO:6010 | SEQ ID NO:14022 | SEQ ID NO:22034 |
| iPS:435071 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | 21-225_91F1 | | SEQ ID NO:6011 | SEQ ID NO:14023 | SEQ ID NO:22035 |
| | | AA | NYDIN | WMHPNSGNTGYAQKFQG | SSGWYWFDP |
| | | | SEQ ID NO:6012 | SEQ ID NO:14024 | SEQ ID NO:22036 |
| iPS:435073 | | NA | GGTTGCTACTGGAGC | GAAATCAATCATAGTGG AAGCACCAACTACAACC CGTCCCTCAAGAGT | GACTACGGCGGTATGGACGT C |
| | 21-225_91B2 | | SEQ ID NO:6013 | SEQ ID NO:14025 | SEQ ID NO:22037 |
| | | AA | GCYWS | EINHSGSTNYNPSLKS | DYGGMDV |
| | | | SEQ ID NO:6014 | SEQ ID NO:14026 | SEQ ID NO:22038 |

FIGURE 49

| iPS:435075 | 21-225_91B3 | NA | GGTTGCTACTGGAGC | GAAATCAATCATAGTGG AAGCACCAACTACAACC CGTCCCTCAAGAGT | GACTACGGCGGTATGGACGT C |
|---|---|---|---|---|---|
| | | | SEQ ID NO:6015 | SEQ ID NO:14027 | SEQ ID NO:22039 |
| | | AA | GCYWS | EINHSGSTNYNPSLKS | DYGGMDV |
| | | | SEQ ID NO:6016 | SEQ ID NO:14028 | SEQ ID NO:22040 |
| iPS:435077 | 21-225_91F3 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6017 | SEQ ID NO:14029 | SEQ ID NO:22041 |
| | | AA | GCYWS | EINHSGSTNYNPSLKS | DYGGMDV |
| | | | SEQ ID NO:6018 | SEQ ID NO:14030 | SEQ ID NO:22042 |
| iPS:435079 | 21-225_91B4 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6019 | SEQ ID NO:14031 | SEQ ID NO:22043 |
| | | AA | GCYWS | EINHSGSTNYNPSLKS | DYGGMDV |
| | | | SEQ ID NO:6020 | SEQ ID NO:14032 | SEQ ID NO:22044 |
| iPS:435087 | 21-225_91G8 | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | | | SEQ ID NO:6021 | SEQ ID NO:14033 | SEQ ID NO:22045 |
| | | AA | NYDIN | WMHPNSGNTGYAQKFQG | SSGWYWFDP |
| | | | SEQ ID NO:6022 | SEQ ID NO:14034 | SEQ ID NO:22046 |
| iPS:435089 | 21-225_91E9 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6023 | SEQ ID NO:14035 | SEQ ID NO:22047 |
| | | AA | GCYWS | EINHSGSTNYNPSLKS | DYGGMDV |

FIGURE 49

|  |  |  | SEQ ID NO:6024 | SEQ ID NO:14036 | SEQ ID NO:22048 |
|---|---|---|---|---|---|
| iPS:435097 | 21-225_92B1 | NA | GGTTCCTACTGGAGC | GAAATCAATTATAGGGG AAGCACCAACTACAACC CGTCCCTCAAGAGT | GACTACGGCGGTTTGGACGT C |
|  |  |  | SEQ ID NO:6025 | SEQ ID NO:14037 | SEQ ID NO:22049 |
|  |  | AA | GSYWS | EINYRGSTNYNPSLKS | DYGGLDV |
|  |  |  | SEQ ID NO:6026 | SEQ ID NO:14038 | SEQ ID NO:22050 |
| iPS:435103 | 21-225_92B2 | NA | AACTACGACATGCAC | GCTATTGGTACTGCTGGT GACACATACTATCCAGG CTCCGTGAAGGGC | GCTCTTGACTACGGTGACTC CTTGGGCTACTACTACTACG GTATGGACGTC |
|  |  |  | SEQ ID NO:6027 | SEQ ID NO:14039 | SEQ ID NO:22051 |
|  |  | AA | NYDMH | AIGTAGDTYYPGSVKG | ALDYGDSLGYYYYGMDV |
|  |  |  | SEQ ID NO:6028 | SEQ ID NO:14040 | SEQ ID NO:22052 |
| iPS:435109 | 21-225_92H5 | NA | AGCTATGGCATGCAC | GTTATATGGTATGATGG AAGTAATAAATACTATG CAGACTCCGTGAAGGGC | GATCCCTTTATAGTGGGAGC TACTTACTTTGACTAC |
|  |  |  | SEQ ID NO:6029 | SEQ ID NO:14041 | SEQ ID NO:22053 |
|  |  | AA | SYGMH | VIWYDGSNKYYADSVKG | DPFIVGATYFDY |
|  |  |  | SEQ ID NO:6030 | SEQ ID NO:14042 | SEQ ID NO:22054 |
| iPS:435111 | 21-225_92D6 | NA | GGTTGCTACTGGAGC | GAAATCAATCATAGTGG AAGCACCAACTACAACC CGTCCCTCAAGAGT | GACTACGGCGGTATGGACGT C |
|  |  |  | SEQ ID NO:6031 | SEQ ID NO:14043 | SEQ ID NO:22055 |
|  |  | AA | GCYWS | EINHSGSTNYNPSLKS | DYGGMDV |
|  |  |  | SEQ ID NO:6032 | SEQ ID NO:14044 | SEQ ID NO:22056 |
| iPS:435113 |  | NA | AATTATGATATCAAC | TGGATGCACCCTAACAG TGGTAACACAGGCTATG CACAGAAGTTCCAGGGC | AGCAGTGGCTGGTACTTTTT TGACTAC |

FIGURE 49

| | 21-225_92E6 | | SEQ ID NO:6033 | SEQ ID NO:14045 | SEQ ID NO:22057 |
|---|---|---|---|---|---|
| | | AA | NYDIN | WMHPNSGNTGYAQKFQG | SSGWYFFDY |
| | | | SEQ ID NO:6034 | SEQ ID NO:14046 | SEQ ID NO:22058 |
| iPS:435115 | 21-225_77C5 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6035 | SEQ ID NO:14047 | SEQ ID NO:22059 |
| | | AA | GCYWS | EINHSGSTNYNPSLKS | DYGGMDV |
| | | | SEQ ID NO:6036 | SEQ ID NO:14048 | SEQ ID NO:22060 |
| iPS:435167 | 21-225_92F12 | NA | AATTATGATATCAAC | TGGATGAACCCTAACAG TGGTAACACAGGCTATG CACAGAAGTTCCAGGGC | ACCAGTGGCGGGAAGTTCTT CGACTAC |
| | | | SEQ ID NO:6037 | SEQ ID NO:14049 | SEQ ID NO:22061 |
| | | AA | NYDIN | WMNPNSGNTGYAQKFQG | TSGGKFFDY |
| | | | SEQ ID NO:6038 | SEQ ID NO:14050 | SEQ ID NO:22062 |
| iPS:435171 | 21-225_93C2 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6039 | SEQ ID NO:14051 | SEQ ID NO:22063 |
| | | AA | GCYWS | EINHSGSTNYNPSLKS | DYGGMDV |
| | | | SEQ ID NO:6040 | SEQ ID NO:14052 | SEQ ID NO:22064 |
| iPS:435177 | 21-225_93E4 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6041 | SEQ ID NO:14053 | SEQ ID NO:22065 |
| | | AA | GCYWS | EINHSGSTNYNPSLKS | DYGGMDV |
| | | | SEQ ID NO:6042 | SEQ ID NO:14054 | SEQ ID NO:22066 |

FIGURE 49

| iPS:435183 | | NA | GGTTGCTACTGGAGC | GAAATCAATCATAGTGG AAGGACCAACTACAACC CGTCCCTCAAGAGT | GACTACGGTGGGCTTGACTA C |
|---|---|---|---|---|---|
| | 21-225_93E9 | | SEQ ID NO:6043 | SEQ ID NO:14055 | SEQ ID NO:22067 |
| | | AA | GCYWS | EINHSGRTNYNPSLKS | DYGGLDY |
| | | | SEQ ID NO:6044 | SEQ ID NO:14056 | SEQ ID NO:22068 |
| iPS:435195 | | NA | GGTTGCTACTGGAGC | GAAATCAATTATAGTGG AAGAACCAACTACAACC CGTCCCTCAAGAGT | GACTACGGCGGTATGGACGT C |
| | 21-225_94D3 | | SEQ ID NO:6045 | SEQ ID NO:14057 | SEQ ID NO:22069 |
| | | AA | GCYWS | EINYSGRTNYNPSLKS | DYGGMDV |
| | | | SEQ ID NO:6046 | SEQ ID NO:14058 | SEQ ID NO:22070 |
| iPS:435197 | | NA | AACGATATCATGCAC | GTTATATGGTATGATGG AAGTAATAAATACTATG CAGACTCCGTGAAGGGC | GAAAAATATAGCAGTGGCTG GTACGACTACGGTATGGACG TC |
| | 21-225_94F3 | | SEQ ID NO:6047 | SEQ ID NO:14059 | SEQ ID NO:22071 |
| | | AA | NDIMH | VIWYDGSNKYYADSVKG | EKYSSGWYDYGMDV |
| | | | SEQ ID NO:6048 | SEQ ID NO:14060 | SEQ ID NO:22072 |
| iPS:435203 | | NA | AATTATGATATCAAC | TGGATGAACCCTAACAG TGGTAACACAGGCTATG CACAGAAGTTCCAGGAC | TCCAGTGGCTGGCACTGGTT CGACCCC |
| | 21-225_75A7 | | SEQ ID NO:6049 | SEQ ID NO:14061 | SEQ ID NO:22073 |
| | | AA | NYDIN | WMNPNSGNTGYAQKFQD | SSGWHWFDP |
| | | | SEQ ID NO:6050 | SEQ ID NO:14062 | SEQ ID NO:22074 |

FIGURE 49

| iPS:435209 | | NA | AATTATGATATCAAC | TGGATGAACCCTAACAG TGGTAACACAGGCTATG CACAGAAGTTCCAGGGC | AGCAGTGGCTGGTACTACTT TGACTAC |
| --- | --- | --- | --- | --- | --- |
| | 21-225_75A10 | | SEQ ID NO:6051 | SEQ ID NO:14063 | SEQ ID NO:22075 |
| | | AA | NYDIN | WMNPNSGNTGYAQKFQG | SSGWYYFDY |
| | | | SEQ ID NO:6052 | SEQ ID NO:14064 | SEQ ID NO:22076 |
| iPS:435211 | | NA | AATTATGATATCAAC | TGGATGAACCCTAACAG TGGTAACACAGGCTATG CACAGAAGTTCCAGGGC | TCCAGTGGCTGGAAGTGGTT CGACCCC |
| | 21-225_94E11 | | SEQ ID NO:6053 | SEQ ID NO:14065 | SEQ ID NO:22077 |
| | | AA | NYDIN | WMNPNSGNTGYAQKFQG | SSGWKWFDP |
| | | | SEQ ID NO:6054 | SEQ ID NO:14066 | SEQ ID NO:22078 |
| iPS:435215 | | NA | AATTATGATATCAAC | TGGATGAACCCTAACAG TGGTAACACAGGCTATG CACAGAAGTTCCAGGGC | ACCAGTGGCTGGAAGTTCTT TGACTAC |
| | 21-225_94E12 | | SEQ ID NO:6055 | SEQ ID NO:14067 | SEQ ID NO:22079 |
| | | AA | NYDIN | WMNPNSGNTGYAQKFQG | TSGWKFFDY |
| | | | SEQ ID NO:6056 | SEQ ID NO:14068 | SEQ ID NO:22080 |
| iPS:435217 | | NA | GGTTGCTACTGGAGC | GAAATCAATCATAGTGG AAGCACCAACTACAACC CGTCCCTCAAGAGT | GACTACGGCGGTATGGACGT C |
| | 21-225_94F12 | | SEQ ID NO:6057 | SEQ ID NO:14069 | SEQ ID NO:22081 |
| | | AA | GCYWS | EINHSGSTNYNPSLKS | DYGGMDV |
| | | | SEQ ID NO:6058 | SEQ ID NO:14070 | SEQ ID NO:22082 |
| iPS:435219 | 21 225 95D2 | NA | GGTTGCTACTGGAGC | GAAATCAATCATAGTGG AAGCACCAACTACAACC CGTCCCTCAAGAGT | GACTACGGCGGTATGGACGT C |

FIGURE 49

| | 21-225_95D2 | | SEQ ID NO:6059 | SEQ ID NO:14071 | SEQ ID NO:22083 |
|---|---|---|---|---|---|
| | | AA | GCYWS | EINHSGSTNYNPSLKS | DYGGMDV |
| | | | SEQ ID NO:6060 | SEQ ID NO:14072 | SEQ ID NO:22084 |
| iPS:435221 | 21-225_95G2 | NA | AGCTATGGCATGCAC | GTTATTTGGTATGATGGA AGTAATAAATACTATGC AGACTCCGTGAAGGGC | GATCGCAATATAGTGGGAGC TACTTACTTTGAGTCC |
| | | | SEQ ID NO:6061 | SEQ ID NO:14073 | SEQ ID NO:22085 |
| | | AA | SYGMH | VIWYDGSNKYYADSVKG | DRNIVGATYFES |
| | | | SEQ ID NO:6062 | SEQ ID NO:14074 | SEQ ID NO:22086 |
| iPS:435227 | 21-225_95G4 | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | | | SEQ ID NO:6063 | SEQ ID NO:14075 | SEQ ID NO:22087 |
| | | AA | NYDIN | WMNPNSGNTGYAQKFQG | SSGWNWFDP |
| | | | SEQ ID NO:6064 | SEQ ID NO:14076 | SEQ ID NO:22088 |
| iPS:435235 | 21-225_95F9 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6065 | SEQ ID NO:14077 | SEQ ID NO:22089 |
| | | AA | GCYWS | EINHSGSTNYNPSLKS | DYGGMDV |
| | | | SEQ ID NO:6066 | SEQ ID NO:14078 | SEQ ID NO:22090 |
| iPS:435237 | 21-225_95G9 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6067 | SEQ ID NO:14079 | SEQ ID NO:22091 |
| | | AA | GCYWS | EINHSGSTNYNPSLKS | DYGGMDV |
| | | | SEQ ID NO:6068 | SEQ ID NO:14080 | SEQ ID NO:22092 |

FIGURE 49

| iPS:435239 | | NA | GGTTGCTACTGGAGC | GAAATCAATCATAGTGG AAGCACCAACTACAACC CGTCCCTCAAGAGT | GACTACGGCGGTATGGACGT C |
|---|---|---|---|---|---|
| | 21-225_95H10 | | SEQ ID NO:6069 | SEQ ID NO:14081 | SEQ ID NO:22093 |
| | | AA | GCYWS | EINHSGSTNYNPSLKS | DYGGMDV |
| | | | SEQ ID NO:6070 | SEQ ID NO:14082 | SEQ ID NO:22094 |
| iPS:435245 | | NA | AATTATGATATCAAC | TGGATGAACCCTAACAG TGGTAACACAGGCTATG CACAGAAGTTCCAGGGC | AGCAGTGGCTGGTACTGGTT CGACCCC |
| | 21-225_95E12 | | SEQ ID NO:6071 | SEQ ID NO:14083 | SEQ ID NO:22095 |
| | | AA | NYDIN | WMNPNSGNTGYAQKFQG | SSGWYWFDP |
| | | | SEQ ID NO:6072 | SEQ ID NO:14084 | SEQ ID NO:22096 |
| iPS:435247 | | NA | AATTATGATATCAAC | TGGATGCACCCTAACAA TGGTAACACAGGCTATG CACAGAAGTTCCAGGGC | AGCAGTGGCTGGTACTGGTT CGACCCC |
| | 21-225_96G1 | | SEQ ID NO:6073 | SEQ ID NO:14085 | SEQ ID NO:22097 |
| | | AA | NYDIN | WMHPNNGNTGYAQKFQG | SSGWYWFDP |
| | | | SEQ ID NO:6074 | SEQ ID NO:14086 | SEQ ID NO:22098 |
| iPS:435249 | | NA | AATTATGATATCAAC | TGGATGCACCCTAATAG TGGTAACACAGGCTATG CACAGAAGTTCCAGGGC | TCCAGTGGCTGGTACTGGTT CGACCCC |
| | 21-225_96E2 | | SEQ ID NO:6075 | SEQ ID NO:14087 | SEQ ID NO:22099 |
| | | AA | NYDIN | WMHPNSGNTGYAQKFQG | SSGWYWFDP |
| | | | SEQ ID NO:6076 | SEQ ID NO:14088 | SEQ ID NO:22100 |
| iPS:435251 | 21 225 96A3 | NA | AGTAGTAATTACTACTGGGG C | AGTATCTATTATAGTGGA TACACCTCCTACAACCCG TCCCTCAAGAGT | CTTGACTCTAACTGGGGTCT TGACTAC |

FIGURE 49

| | 21-225_96A3 | | SEQ ID NO:6077 | SEQ ID NO:14089 | SEQ ID NO:22101 |
|---|---|---|---|---|---|
| | | AA | SSNYYWG | SIYYSGYTSYNPSLKS | LDSNWGLDY |
| | | | SEQ ID NO:6078 | SEQ ID NO:14090 | SEQ ID NO:22102 |
| iPS:435253 | 21-225_96A4 | NA | AGTTATGATATCAAC | TGGATGAACCCTAACAGTGGTAACACAGGCTATGCACAGAAGTTCCAGGGC | GGCTTTTACGATACTTTGACTGGTTCCGGCTACTACTACGTTATGGACGTC |
| | | | SEQ ID NO:6079 | SEQ ID NO:14091 | SEQ ID NO:22103 |
| | | AA | SYDIN | WMNPNSGNTGYAQKFQG | GFYDTLTGSGYYYVMDV |
| | | | SEQ ID NO:6080 | SEQ ID NO:14092 | SEQ ID NO:22104 |
| iPS:435255 | 21-225_96D5 | NA | AATTATGATATCAAC | TGGATGAACCCTAACAGTGGTAACACAGGCTATGCACAGAAGTTCCAGGGC | TCCAGTGGCTGGTCCTGGTTCGACCCC |
| | | | SEQ ID NO:6081 | SEQ ID NO:14093 | SEQ ID NO:22105 |
| | | AA | NYDIN | WMNPNSGNTGYAQKFQG | SSGWSWFDP |
| | | | SEQ ID NO:6082 | SEQ ID NO:14094 | SEQ ID NO:22106 |
| iPS:435257 | 21-225_96H5 | NA | AATTATGATATCAAC | TGGATGCACCCTAACAGTGGTAACACAGGCTATGCACAGAAGTTCCAGGGC | AGTAGTGGCTGGTACAAATTTGACTAC |
| | | | SEQ ID NO:6083 | SEQ ID NO:14095 | SEQ ID NO:22107 |
| | | AA | NYDIN | WMHPNSGNTGYAQKFQG | SSGWYKFDY |
| | | | SEQ ID NO:6084 | SEQ ID NO:14096 | SEQ ID NO:22108 |
| iPS:435259 | 21-225_96C6 | NA | AGTTATGATATCAAC | TGGATGAACCCTAACAGTCGTAACACAGGCTATGCACAGAAGTTCCAGGGC | GGCGGCTACGATGTTTTGCCTGGGAATAACTACTACTACGATATGGACGTC |
| | | | SEQ ID NO:6085 | SEQ ID NO:14097 | SEQ ID NO:22109 |
| | | AA | SYDIN | WMNPNSRNTGYAQKFQG | GGYDVLPGNNYYYDMDV |

FIGURE 49

| | | | SEQ ID NO:6086 | SEQ ID NO:14098 | SEQ ID NO:22110 |
|---|---|---|---|---|---|
| iPS:435267 | | NA | AATTATGATATCAAC | TGGATGCACCCTAACAG TGGTAACACAGGCTATG CACAGAAGTTCCAGGGC | AGCAGTGGCTGGTACTTTTT TGACTAC |
| | 21-225_96D10 | | SEQ ID NO:6087 | SEQ ID NO:14099 | SEQ ID NO:22111 |
| | | AA | NYDIN | WMHPNSGNTGYAQKFQG | SSGWYFFDY |
| | | | SEQ ID NO:6088 | SEQ ID NO:14100 | SEQ ID NO:22112 |
| iPS:435273 | | NA | GGTTGCTACTGGAGC | GAAATCAATCATAGTGG AAGCACCAACTACAACC CGTCCCTCAAGAGT | GACTACGGCGGTATGGACGT C |
| | 21-225_97A2 | | SEQ ID NO:6089 | SEQ ID NO:14101 | SEQ ID NO:22113 |
| | | AA | GCYWS | EINHSGSTNYNPSLKS | DYGGMDV |
| | | | SEQ ID NO:6090 | SEQ ID NO:14102 | SEQ ID NO:22114 |
| iPS:435279 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | 21-225_97H4 | | SEQ ID NO:6091 | SEQ ID NO:14103 | SEQ ID NO:22115 |
| | | AA | NYDIN | WMHPNSGNTGYAQKFQG | SSGWYWFDP |
| | | | SEQ ID NO:6092 | SEQ ID NO:14104 | SEQ ID NO:22116 |
| iPS:435281 | | NA | GGTTGCTACTGGAGC | GAAATCAATCATAGTGG AAGCACCAACTACAACC CGTCCCTCAAGAGT | GACTACGGCGGTATGGACGT C |
| | 21-225_97E5 | | SEQ ID NO:6093 | SEQ ID NO:14105 | SEQ ID NO:22117 |
| | | AA | GCYWS | EINHSGSTNYNPSLKS | DYGGMDV |
| | | | SEQ ID NO:6094 | SEQ ID NO:14106 | SEQ ID NO:22118 |

FIGURE 49

| iPS:435291 | | NA | AGCTATGGCATGCAC | ATTATATGGTATGATGG AAGTAATAAATACTATG CAGACTCCGTGAAGGGC | GATCGTTTAGTGGGAGCTAC CGCTGATGCTTTTGATATC |
|---|---|---|---|---|---|
| | 21-225_146E1 | | SEQ ID NO:6095 | SEQ ID NO:14107 | SEQ ID NO:22119 |
| | | AA | SYGMH | IIWYDGSNKYYADSVKG | DRLVGATADAFDI |
| | | | SEQ ID NO:6096 | SEQ ID NO:14108 | SEQ ID NO:22120 |
| iPS:435293 | | NA | AGAAGTAGTTACTACTGGGG C | AGTATATATTATAGTGG GAGTACCTCCTACAACC CGTCCCTCAAGAGT | CTTGATCTCCTGTGGAGTTTT GACTAC |
| | 21-225_146F1 | | SEQ ID NO:6097 | SEQ ID NO:14109 | SEQ ID NO:22121 |
| | | AA | RSSYYWG | SIYYSGSTSYNPSLKS | LDLLWSFDY |
| | | | SEQ ID NO:6098 | SEQ ID NO:14110 | SEQ ID NO:22122 |
| iPS:435295 | | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
| | 21-225_146H1 | | SEQ ID NO:6099 | SEQ ID NO:14111 | SEQ ID NO:22123 |
| | | AA | SYAMS | AISGRGGNTFYADSVKG | RVTDYGGNDWFDP |
| | | | SEQ ID NO:6100 | SEQ ID NO:14112 | SEQ ID NO:22124 |
| iPS:435297 | | NA | AGCTATGGCATGCAC | GTTATATGGTATGATGG AAGTTATAAATACTATG CAGACTCCGTGAAGGGC | ATGGGTATAGAAGTGGCTGT GGACTACTACTACGGTATGG ACGTC |
| | 21-225_146B3 | | SEQ ID NO:6101 | SEQ ID NO:14113 | SEQ ID NO:22125 |
| | | AA | SYGMH | VIWYDGSYKYYADSVKG | MGIEVAVDYYYGMDV |
| | | | SEQ ID NO:6102 | SEQ ID NO:14114 | SEQ ID NO:22126 |
| iPS:435299 | | NA | AATTATGATATCAAC | TGGGTGCACCCTAACAG TGGTAACACAGGCTATG CACAGAAGTTCCAGGGC | AGCAGTGGCTGGTACTACTT TGACTAC |
| | 21-225_146D4 | | SEQ ID NO:6103 | SEQ ID NO:14115 | SEQ ID NO:22127 |

FIGURE 49

| | | AA | NYDIN | WVHPNSGNTGYAQKFQG | SSGWYYFDY | |
|---|---|---|---|---|---|---|
| | | | SEQ ID NO:6104 | SEQ ID NO:14116 | SEQ ID NO:22128 | |
| iPS:435301 | 21-225_146G4 | NA | AACAGTGGTTACTACTGGAGC | TACAGCTATTACAGTGGGAGCACCTACTACAACCCGTCCCTCAAGAGT | GGGAAATATAACTGGAACCATGCTTTTGATATC | |
| | | | SEQ ID NO:6105 | SEQ ID NO:14117 | SEQ ID NO:22129 | |
| | | AA | NSGYYWS | YSYYSGSTYYNPSLKS | GKYNWNHAFDI | |
| | | | SEQ ID NO:6106 | SEQ ID NO:14118 | SEQ ID NO:22130 | |
| iPS:435303 | 21-225_146A6 | NA | AGCTATGCCATGAAC | GCTATTAGTGGTAGTGGTGGTAACACATTCTACGCAGACTCCGTGAAGGGC | AAGGATAATGATTACGTTTGGGGGAGTCCTTACTTTGACTAC | |
| | | | SEQ ID NO:6107 | SEQ ID NO:14119 | SEQ ID NO:22131 | |
| | | AA | SYAMN | AISGSGGNTFYADSVKG | KDNDYVWGSPYFDY | |
| | | | SEQ ID NO:6108 | SEQ ID NO:14120 | SEQ ID NO:22132 | |
| iPS:435305 | 21-225_146C9 | NA | AATTATGATATCAAC | TGGATGCACCCTAACAGTGGTAACACAGGCTATGCACAGAAGTTCCAGGGC | AGCAGTGGCTGGTACTCCTTTGACTAC | |
| | | | SEQ ID NO:6109 | SEQ ID NO:14121 | SEQ ID NO:22133 | |
| | | AA | NYDIN | WMHPNSGNTGYAQKFQG | SSGWYSFDY | |
| | | | SEQ ID NO:6110 | SEQ ID NO:14122 | SEQ ID NO:22134 | |
| iPS:435307 | 21-225_146E9 | NA | AGCTATGCCATGAGC | GCTATTAGTGGTCGTGGTGGTAACACATTCTACGCAGACTCCGTGAAGGGC | CGGGTGACGGACTACGGTGGTAACGACTGGTTCGACCCC | |
| | | | SEQ ID NO:6111 | SEQ ID NO:14123 | SEQ ID NO:22135 | |
| | | AA | SYAMS | AISGRGGNTFYADSVKG | RVTDYGGNDWFDP | |
| | | | SEQ ID NO:6112 | SEQ ID NO:14124 | SEQ ID NO:22136 | |

FIGURE 49

| iPS:435309 | | NA | AATTATGATATCAAC | TGGATGCACCCTAACAG TGGTAACACAGGCTATG CACAGAAGTTCCAGGGC | AGCAGTGGCTGGTACTTTTT TGACTAC |
|---|---|---|---|---|---|
| | 21-225_146F9 | | SEQ ID NO:6113 | SEQ ID NO:14125 | SEQ ID NO:22137 |
| | | AA | NYDIN | WMHPNSGNTGYAQKFQG | SSGWYFFDY |
| | | | SEQ ID NO:6114 | SEQ ID NO:14126 | SEQ ID NO:22138 |
| iPS:435311 | | NA | AGCTATGGCATGCAC | GTGATATGGTTTGATGA AAGTAATAAACACTATG GAGACTCCGTGAAGGGC | GAATTGGGATTTCTCTCTGA CTAT |
| | 21-225_146H9 | | SEQ ID NO:6115 | SEQ ID NO:14127 | SEQ ID NO:22139 |
| | | AA | SYGMH | VIWFDESNKHYGDSVKG | ELGFLSDY |
| | | | SEQ ID NO:6116 | SEQ ID NO:14128 | SEQ ID NO:22140 |
| iPS:435313 | | NA | AGCTATAGCATGAAC | TCCATTAGTGGTAGTGGT AGCTACACATACTACGC AGACTCAGTGAAGGGC | GGTAGCAGCTCGTCCGGGTT TGACTAC |
| | 21-225_146G11 | | SEQ ID NO:6117 | SEQ ID NO:14129 | SEQ ID NO:22141 |
| | | AA | SYSMN | SISGSGSYTYYADSVKG | GSSSSGFDY |
| | | | SEQ ID NO:6118 | SEQ ID NO:14130 | SEQ ID NO:22142 |
| iPS:435315 | | NA | AGCTATGGCATGCAC | GTTATATGGTATGATGG AAGTAATAAATACTATG CAGACTCCGTGAAGGGC | AGGTATAGCAGCAGCTGGTC GGGGGGTATGGACGTC |
| | 21-225_147B2 | | SEQ ID NO:6119 | SEQ ID NO:14131 | SEQ ID NO:22143 |
| | | AA | SYGMH | VIWYDGSNKYYADSVKG | RYSSSWSGGMDV |
| | | | SEQ ID NO:6120 | SEQ ID NO:14132 | SEQ ID NO:22144 |

FIGURE 49

| iPS:435317 | | NA | AGTGGTGATTACTACTGGAAC | TTCATCTATTACACTGGGAGCACCTACTACAACCCGTCCCTCAAGAGT | GGGGGAGCTTACTACTCCTACTACGGTATGGACGTC |
|---|---|---|---|---|---|
| | 21-225_147D2 | | SEQ ID NO:6121 | SEQ ID NO:14133 | SEQ ID NO:22145 |
| | | AA | SGDYYWN | FIYYTGSTYYNPSLKS | GGAYYSYYGMDV |
| | | | SEQ ID NO:6122 | SEQ ID NO:14134 | SEQ ID NO:22146 |
| iPS:435319 | | NA | AATAGTGGTTACTACTATAGC | TACATCTATTACAGTGGGGGCACCTACTACAACCCGTCCCTCAAGAGT | GGGGGATATAACTGGAACCATGCTTTTGATTTC |
| | 21-225_147E3 | | SEQ ID NO:6123 | SEQ ID NO:14135 | SEQ ID NO:22147 |
| | | AA | NSGYYYS | YIYYSGGTYYNPSLKS | GGYNWNHAFDF |
| | | | SEQ ID NO:6124 | SEQ ID NO:14136 | SEQ ID NO:22148 |
| iPS:435321 | | NA | AATTATGATATCAAC | TGGATGAACCCTAACAGTGGTAACACAGGCTATGCACAGAAGTTCCAGGGC | AGCAGTGGCTGGTACTTTTTGACTAC |
| | 21-225_147E4 | | SEQ ID NO:6125 | SEQ ID NO:14137 | SEQ ID NO:22149 |
| | | AA | NYDIN | WMNPNSGNTGYAQKFQG | SSGWYFFDY |
| | | | SEQ ID NO:6126 | SEQ ID NO:14138 | SEQ ID NO:22150 |
| iPS:435323 | | NA | AATTATGATATCAAC | TGGGTGCACCCTAACAGTGGTAACACAGGCTATGCACAGAAGTTCCAGGGC | AGCAGTGGCTGGTACTACTTTGACTAC |
| | 21-225_147D5 | | SEQ ID NO:6127 | SEQ ID NO:14139 | SEQ ID NO:22151 |
| | | AA | NYDIN | WVHPNSGNTGYAQKFQG | SSGWYYFDY |
| | | | SEQ ID NO:6128 | SEQ ID NO:14140 | SEQ ID NO:22152 |
| iPS:435325 | | NA | GACTATGGCATGCAC | GTTATATGGTATGATGGAAGTAATAAGTACTATGCAGACTCCGTGAAGGGC | GAGAGGTATAGCAGTGGCTGGTACGACTACGGTTTGGACGTC |
| | 21-225_147H5 | | SEQ ID NO:6129 | SEQ ID NO:14141 | SEQ ID NO:22153 |

FIGURE 49

| | | AA | DYGMH | VIWYDGSNKYYADSVKG | ERYSSGWYDYGLDV |
|---|---|---|---|---|---|
| | | | SEQ ID NO:6130 | SEQ ID NO:14142 | SEQ ID NO:22154 |
| iPS:435327 | | NA | AATTATGATATCAAC | TGGATGCACCCCAACAG TGGTAACACAGGCTATG CACAGAAGTTCCAGGGC | AGCAGTGGCTGGTACTGGTT CGACCCC |
| | 21-225_147G6 | | SEQ ID NO:6131 | SEQ ID NO:14143 | SEQ ID NO:22155 |
| | | AA | NYDIN | WMHPNSGNTGYAQKFQG | SSGWYWFDP |
| | | | SEQ ID NO:6132 | SEQ ID NO:14144 | SEQ ID NO:22156 |
| iPS:435329 | | NA | AGCTATGGCATGCAC | GTTATATGGTATGATGG AAGTAATAAATACTATG CAGACTCCGTGAAGGGC | AGGTATAGCAGCAGCTGGAC GGGGGGTATGGACGTC |
| | 21-225_147A8 | | SEQ ID NO:6133 | SEQ ID NO:14145 | SEQ ID NO:22157 |
| | | AA | SYGMH | VIWYDGSNKYYADSVKG | RYSSSWTGGMDV |
| | | | SEQ ID NO:6134 | SEQ ID NO:14146 | SEQ ID NO:22158 |
| iPS:435331 | | NA | GCTTACTACTGGAGC | GAAATCAATCATAGTGG AAGTACCAACTACAAAC CGTCCCTCAAGAGT | GACTACGGTGTTTTTGACTA C |
| | 21-225_147G8 | | SEQ ID NO:6135 | SEQ ID NO:14147 | SEQ ID NO:22159 |
| | | AA | AYYWS | EINHSGSTNYKPSLKS | DYGVFDY |
| | | | SEQ ID NO:6136 | SEQ ID NO:14148 | SEQ ID NO:22160 |
| iPS:435333 | | NA | AGCTATAGCATGAAC | TCCATTAGTGGTAGAAA CACTACCATATACTATGC AGACTCTGTGAAGGGC | GATCGGGGCAGTTGC |
| | 21-225_147E9 | | SEQ ID NO:6137 | SEQ ID NO:14149 | SEQ ID NO:22161 |
| | | AA | SYSMN | SISGRNTTIYYADSVKG | DRGSC |
| | | | SEQ ID NO:6138 | SEQ ID NO:14150 | SEQ ID NO:22162 |

FIGURE 49

| iPS:435335 | 21-225_147D10 | NA | AGCTATGCCATGAGC | GCTATTAGTGGTCGTGGT GGTAACACATTCTACGC AGACTCCGTGAAGGGC | AAGGATTATGATTACGTTTG GGGGAGTCCTTACTTTGACT AC |
|---|---|---|---|---|---|
| | | | SEQ ID NO:6139 | SEQ ID NO:14151 | SEQ ID NO:22163 |
| | | AA | SYAMS | AISGRGGNTFYADSVKG | KDYDYVWGSPYFDY |
| | | | SEQ ID NO:6140 | SEQ ID NO:14152 | SEQ ID NO:22164 |
| iPS:435339 | 21-225_147D12 | NA | AGCTATGCCATGAGC | GCTATTAGTGGTCGTGGT GGTAACACATTCTACGC AGACTCCGTGAAGGGC | AAGGATTATGATTACGTTTG GGGGAGTCCTTACTTTGACT AC |
| | | | SEQ ID NO:6141 | SEQ ID NO:14153 | SEQ ID NO:22165 |
| | | AA | SYAMS | AISGRGGNTFYADSVKG | KDYDYVWGSPYFDY |
| | | | SEQ ID NO:6142 | SEQ ID NO:14154 | SEQ ID NO:22166 |
| iPS:435341 | 21-225_148B2 | NA | AGCTATGGCATGCAC | ATTATCTGGTATGATGGA AGTTATAAATACTATGC AGACTCCGTGAAGGGC | GATCATTTCGATTTTTGGAGT GGTCACTTTGACTAC |
| | | | SEQ ID NO:6143 | SEQ ID NO:14155 | SEQ ID NO:22167 |
| | | AA | SYGMH | IIWYDGSYKYYADSVKG | DHFDFWSGHFDY |
| | | | SEQ ID NO:6144 | SEQ ID NO:14156 | SEQ ID NO:22168 |
| iPS:435343 | 21-225_148E2 | NA | AGCTATGCCATGAGC | GCTATTAGTGGTCGTGGT GGTAACACATTCTACGC AGACTCCGTGAAGGGC | AAGGATTATGATTACGTTTG GGGGAGTCCTTACTTTGACT AC |
| | | | SEQ ID NO:6145 | SEQ ID NO:14157 | SEQ ID NO:22169 |
| | | AA | SYAMS | AISGRGGNTFYADSVKG | KDYDYVWGSPYFDY |
| | | | SEQ ID NO:6146 | SEQ ID NO:14158 | SEQ ID NO:22170 |
| iPS:435345 | 21-225_148G3 | NA | AATTATGATATCAAC | TGGATGCACCCTAACAG TGGTAACACAGGCTATG CACAGAACTTCCAGGGC | AGCAGTGGCTGGTACTTTTT TGACTAC |
| | | | SEQ ID NO:6147 | SEQ ID NO:14159 | SEQ ID NO:22171 |
| | | AA | NYDIN | WMHPNSGNTGYAQNFQG | SSGWYFFDY |

FIGURE 49

| | | | SEQ ID NO:6148 | SEQ ID NO:14160 | SEQ ID NO:22172 |
|---|---|---|---|---|---|
| iPS:435347 | | NA | AGCTATGCCATGAAC | GCTATTAGTGGTAGTGGT GGTAACACATTCTACGC AGACTCCGTGAAGGGC | CGGGTGACGGACTACGGTGG TAACGACTGGTTCGACCCC |
| | 21-225_148C4 | | SEQ ID NO:6149 | SEQ ID NO:14161 | SEQ ID NO:22173 |
| | | AA | SYAMN | AISGSGGNTFYADSVKG | RVTDYGGNDWFDP |
| | | | SEQ ID NO:6150 | SEQ ID NO:14162 | SEQ ID NO:22174 |
| iPS:435349 | | NA | AGCTATGGCATGCAC | GTTATATGGTATGATGG AAGTAATAAATACTATG CAGACTCCGTGAAGGGC | AGGTATAGCAGCAGCTGGTC GGGGGGTATGGACGTC |
| | 21-225_148F5 | | SEQ ID NO:6151 | SEQ ID NO:14163 | SEQ ID NO:22175 |
| | | AA | SYGMH | VIWYDGSNKYYADSVKG | RYSSSWSGGMDV |
| | | | SEQ ID NO:6152 | SEQ ID NO:14164 | SEQ ID NO:22176 |
| iPS:435351 | | NA | GGCTACTATATGCAC | TGGATCCACCCTAACAA TGGTGGCACAAACTATG CACAGACGTTTCAGGGC | GATCCTGTAGTAGTACCAGC TGCCCCCTTTGACTAC |
| | 21-225_148B6 | | SEQ ID NO:6153 | SEQ ID NO:14165 | SEQ ID NO:22177 |
| | | AA | GYYMH | WIHPNNGGTNYAQTFQG | DPVVVPAAPFDY |
| | | | SEQ ID NO:6154 | SEQ ID NO:14166 | SEQ ID NO:22178 |
| iPS:435353 | | NA | AATTATGATATCAAC | TGGATGAACCCTAACAG TGGTAACACAGGCTATG CACAGAAGTTCCAGGGC | AGCAGTGGCTGGTACTACTT TGACTAC |
| | 21-225_148F8 | | SEQ ID NO:6155 | SEQ ID NO:14167 | SEQ ID NO:22179 |
| | | AA | NYDIN | WMNPNSGNTGYAQKFQG | SSGWYYFDY |
| | | | SEQ ID NO:6156 | SEQ ID NO:14168 | SEQ ID NO:22180 |

FIGURE 49

| iPS:435355 | 21-225_148H9 | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
|---|---|---|---|---|---|
| | | | SEQ ID NO:6157 | SEQ ID NO:14169 | SEQ ID NO:22181 |
| | | AA | SYAMS | AISGRGGNTFYADSVKG | RVTDYGGNDWFDP |
| | | | SEQ ID NO:6158 | SEQ ID NO:14170 | SEQ ID NO:22182 |
| iPS:435357 | 21-225_148G10 | NA | AGCTATGGCATGCAC | ATTATATGGTATGATGG AAGTAATAAATACTATG CAGACTCCGTGAAGGGC | GATCGTTACGATTTTTGGAG TGGTCACTTTGACTAC |
| | | | SEQ ID NO:6159 | SEQ ID NO:14171 | SEQ ID NO:22183 |
| | | AA | SYGMH | IIWYDGSNKYYADSVKG | DRYDFWSGHFDY |
| | | | SEQ ID NO:6160 | SEQ ID NO:14172 | SEQ ID NO:22184 |
| iPS:435359 | 21-225_148H10 | NA | AGCTATGGCATGCAC | GTTATATGGTATGATGG AAGTAATAAATACTATG GAGACTCCGTGAAGGGC | AGGTATAGCAGCAGCTGGTC GGGGGGGTATGGACGTC |
| | | | SEQ ID NO:6161 | SEQ ID NO:14173 | SEQ ID NO:22185 |
| | | AA | SYGMH | VIWYDGSNKYYGDSVKG | RYSSSWSGGMDV |
| | | | SEQ ID NO:6162 | SEQ ID NO:14174 | SEQ ID NO:22186 |
| iPS:435361 | 21-225_148E11 | NA | AGAAGTAGTTACTACTGGGG C | AGTATCTATTATAGTGGG AGCACCTCCTACAACCC GTCCCTCAAGAGT | CTTGATCCCCAGTGGAGTTT TGACTAC |
| | | | SEQ ID NO:6163 | SEQ ID NO:14175 | SEQ ID NO:22187 |
| | | AA | RSSYYWG | SIYYSGSTSYNPSLKS | LDPQWSFDY |
| | | | SEQ ID NO:6164 | SEQ ID NO:14176 | SEQ ID NO:22188 |
| iPS:435363 | 21 225 148F12 | NA | AATGGTGGTTACTACTGGAA C | TACATCTATTACAGTGGG AGCACCTACTACAACCC GTCCCTCAAGAGT | TACAGTACCTACGACTACTA CTACGGTATGGACGTC |

FIGURE 49

| | | | SEQ ID NO:6165 | SEQ ID NO:14177 | SEQ ID NO:22189 |
|---|---|---|---|---|---|
| | 21-225_148F12 | AA | NGGYYWN | YIYYSGSTYYNPSLKS | YSTYDYYYGMDV |
| iPS:435365 | | NA | SEQ ID NO:6166<br>AGCTATGGCATGCAC | SEQ ID NO:14178<br>ATTATCTGGTATGATGGA<br>AGTTATAAATACTATGC<br>AGACTCCGTGAAGGGC | SEQ ID NO:22190<br>GATCATTTCGATTTTTGGAGT<br>GGTCACTTTGACTAC |
| | 21-225_149F1 | | SEQ ID NO:6167 | SEQ ID NO:14179 | SEQ ID NO:22191 |
| | | AA | SYGMH | IIWYDGSYKYYADSVKG | DHFDFWSGHFDY |
| | | | SEQ ID NO:6168 | SEQ ID NO:14180 | SEQ ID NO:22192 |
| iPS:435367 | | NA | GACTATGGCATGCAC | GTTATATGGTATGAAGG<br>AAGTAATAAATACTATG<br>CAGACTCCGTGAAGGGC | GAAATAGGATTCAGTGAGGA<br>CTAC |
| | 21-225_149G1 | | SEQ ID NO:6169 | SEQ ID NO:14181 | SEQ ID NO:22193 |
| | | AA | DYGMH | VIWYEGSNKYYADSVKG | EIGFSEDY |
| | | | SEQ ID NO:6170 | SEQ ID NO:14182 | SEQ ID NO:22194 |
| iPS:435369 | | NA | AATTATGATATCAAC | TGGGTGCACCCTAACAG<br>TGGTAACACAGGCTATG<br>CACAGAAGTTCCAGGGC | AGCAGTGGCTGGTACTACTT<br>TGACTAC |
| | 21-225_149A2 | | SEQ ID NO:6171 | SEQ ID NO:14183 | SEQ ID NO:22195 |
| | | AA | NYDIN | WVHPNSGNTGYAQKFQG | SSGWYYFDY |
| | | | SEQ ID NO:6172 | SEQ ID NO:14184 | SEQ ID NO:22196 |
| iPS:435371 | | NA | AACTATGCCATGACC | GCTATTAGTGGTCGTGGT<br>GGTAACACATTCTACGC<br>AGACTCCGTGAAGGGC | CGGGTGACGGACTACGGTGG<br>TAACGACTGGTTCGACCCC |
| | 21-225_149A3 | | SEQ ID NO:6173 | SEQ ID NO:14185 | SEQ ID NO:22197 |
| | | AA | NYAMT | AISGRGGNTFYADSVKG | RVTDYGGNDWFDP |
| | | | SEQ ID NO:6174 | SEQ ID NO:14186 | SEQ ID NO:22198 |

FIGURE 49

| iPS:435373 | | NA | AATTATGATATCAAC | TGGATGCACCCTAACAGTGGTAACACAGGCTATGCACAGAAGTTCCAGGGC | AGCAGTGGCTGGTACTGGTTTGACTAC |
|---|---|---|---|---|---|
| | 21-225_149E3 | | SEQ ID NO:6175 | SEQ ID NO:14187 | SEQ ID NO:22199 |
| | | AA | NYDIN | WMHPNSGNTGYAQKFQG | SSGWYWFDY |
| | | | SEQ ID NO:6176 | SEQ ID NO:14188 | SEQ ID NO:22200 |
| iPS:435375 | | NA | AATTATGATATCAAC | TGGATGCACCCTAACAGTGGTAACACAGACTATGCACAGAAGTTCCAGGGC | AGCAGTGGCTGGTACTACTTTGACTAC |
| | 21-225_149H4 | | SEQ ID NO:6177 | SEQ ID NO:14189 | SEQ ID NO:22201 |
| | | AA | NYDIN | WMHPNSGNTDYAQKFQG | SSGWYYFDY |
| | | | SEQ ID NO:6178 | SEQ ID NO:14190 | SEQ ID NO:22202 |
| iPS:435377 | | NA | AATGGTGGTTACTACTGGAAC | TACATCTATTACAGTGGGAGCACCTACTACAACCCGTCCCTCAAGAGT | TACAGTACCTACGACTACTACTACGGTATGGACGTC |
| | 21-225_149G5 | | SEQ ID NO:6179 | SEQ ID NO:14191 | SEQ ID NO:22203 |
| | | AA | NGGYYWN | YIYYSGSTYYNPSLKS | YSTYDYYYGMDV |
| | | | SEQ ID NO:6180 | SEQ ID NO:14192 | SEQ ID NO:22204 |
| iPS:435379 | | NA | AGCTATGCCATGAGC | GTTATTAGTGGTAGTGGTGGTAGCACATTCTACGCAGACTCCGTGAAGGGC | CGAACTCCGGAAGATGTTTTTGATATC |
| | 21-225_149B6 | | SEQ ID NO:6181 | SEQ ID NO:14193 | SEQ ID NO:22205 |
| | | AA | SYAMS | VISGSGGSTFYADSVKG | RTPEDVFDI |
| | | | SEQ ID NO:6182 | SEQ ID NO:14194 | SEQ ID NO:22206 |
| iPS:435381 | | NA | AGCTATGCCATGAGC | GCTATTAGTGGTAGTGGTGGTAACACATTCTACGCAGACTCCGTGAAGGGC | AAGGATTATGATTACGTTTGGGGGAGTCCTTACTTTGACTAC |
| | 21-225_149C6 | | SEQ ID NO:6183 | SEQ ID NO:14195 | SEQ ID NO:22207 |

**FIGURE 49**

| | | | | | |
|---|---|---|---|---|---|
| | | AA | SYAMS | AISGSGGNTFYADSVKG | KDYDYVWGSPYFDY |
| | | | SEQ ID NO:6184 | SEQ ID NO:14196 | SEQ ID NO:22208 |
| iPS:435383 | 21-225_149D7 | NA | AACAGTGGTTACTACTGGAGC | TACAGCTATTACAGTGGGAGCACCTACTACAACCCGTCCCTCAAGAGT | GGGGGATATAACTGGAACCATGCTTTTGATATC |
| | | | SEQ ID NO:6185 | SEQ ID NO:14197 | SEQ ID NO:22209 |
| | | AA | NSGYYWS | YSYYSGSTYYNPSLKS | GGYNWNHAFDI |
| | | | SEQ ID NO:6186 | SEQ ID NO:14198 | SEQ ID NO:22210 |
| iPS:435391 | 21-225_149F8 | NA | AGCTATGCCATGAGC | GCTATTAGTGGTCGTGGTGGTAACACATTCTACGCAGACTCCGTGAAGGGC | AAGGATTATGATTACGTTTGGGGGAGTCCTTACTTTGACTAC |
| | | | SEQ ID NO:6187 | SEQ ID NO:14199 | SEQ ID NO:22211 |
| | | AA | SYAMS | AISGRGGNTFYADSVKG | KDYDYVWGSPYFDY |
| | | | SEQ ID NO:6188 | SEQ ID NO:14200 | SEQ ID NO:22212 |
| iPS:435393 | 21-225_149D10 | NA | GACTATGGCATGCAC | GTTATATGGTATGATGGAAGTAATAAGTACTATGCAGACTCCGTGAAGGGC | GAGAGGTATAGCAGTGGCTGGTACGACTACGGTATGGACGTC |
| | | | SEQ ID NO:6189 | SEQ ID NO:14201 | SEQ ID NO:22213 |
| | | AA | DYGMH | VIWYDGSNKYYADSVKG | ERYSSGWYDYGMDV |
| | | | SEQ ID NO:6190 | SEQ ID NO:14202 | SEQ ID NO:22214 |
| iPS:435395 | 21-225_149D11 | NA | AGCTATGCCATGAGC | GCTATTAGTGGTCGTGGTGGTAACACATTCTACGCAGACTCCGTGAAGGGC | AAGGATTATGATTACGTTTGGGGGAGTCCTTACTTTGACTAC |
| | | | SEQ ID NO:6191 | SEQ ID NO:14203 | SEQ ID NO:22215 |
| | | AA | SYAMS | AISGRGGNTFYADSVKG | KDYDYVWGSPYFDY |
| | | | SEQ ID NO:6192 | SEQ ID NO:14204 | SEQ ID NO:22216 |

FIGURE 49

| iPS:435397 | | NA | GACTATGGCATGCAC | GTTATATGGTATGAAGA AAATAATAAATACTATG CAGACTCCGTGAAGGGC | GAAATAGGGTTCAGTGAGGA CTAC |
|---|---|---|---|---|---|
| | 21-225_149F12 | | SEQ ID NO:6193 | SEQ ID NO:14205 | SEQ ID NO:22217 |
| | | AA | DYGMH | VIWYEENNKYYADSVKG | EIGFSEDY |
| | | | SEQ ID NO:6194 | SEQ ID NO:14206 | SEQ ID NO:22218 |
| iPS:435399 | | NA | AATTATGATATCAAC | TGGATGCACCCTAACAG TGGTAACACAGGCTATG CACAGAAGTTCCAGGGC | AGCAGTGGCTGGTACTACTT TGACTAC |
| | 21-225_150D2 | | SEQ ID NO:6195 | SEQ ID NO:14207 | SEQ ID NO:22219 |
| | | AA | NYDIN | WMHPNSGNTGYAQKFQG | SSGWYYFDY |
| | | | SEQ ID NO:6196 | SEQ ID NO:14208 | SEQ ID NO:22220 |
| iPS:435401 | | NA | AGCTATGTCATGCAC | GTTATATGGTATGATGG AAGTAATAAATACTATG CAGACTCCGTGAAGGGC | GAGGAGTATAGCAGCAGCTG GTACGGGTACGGTATGGACG TC |
| | 21-225_150E2 | | SEQ ID NO:6197 | SEQ ID NO:14209 | SEQ ID NO:22221 |
| | | AA | SYVMH | VIWYDGSNKYYADSVKG | EEYSSSWYGYGMDV |
| | | | SEQ ID NO:6198 | SEQ ID NO:14210 | SEQ ID NO:22222 |
| iPS:435403 | | NA | AGCTATGCCATGAGC | GCTATTAGTGGTAGTGGT GGTAACACATTCTACGC AGACTCCGTGAAGGGC | AAGGATTATGATTACGTTTG GGGGAGTCCTTACTTTGACT AC |
| | 21-225_150C5 | | SEQ ID NO:6199 | SEQ ID NO:14211 | SEQ ID NO:22223 |
| | | AA | SYAMS | AISGSGGNTFYADSVKG | KDYDYVWGSPYFDY |
| | | | SEQ ID NO:6200 | SEQ ID NO:14212 | SEQ ID NO:22224 |

FIGURE 49

| iPS:435405 | | NA | AATTATGATATCAAC | TGGATGCACCCTAACAG TGGTAACACAGGCTATG CACAGAAGTTCCAGGGC | AGCAGTGGCTGGTACTTTTT TGACTAC |
|---|---|---|---|---|---|
| | 21-225_150B7 | | SEQ ID NO:6201 | SEQ ID NO:14213 | SEQ ID NO:22225 |
| | | AA | NYDIN | WMHPNSGNTGYAQKFQG | SSGWYFFDY |
| | | | SEQ ID NO:6202 | SEQ ID NO:14214 | SEQ ID NO:22226 |
| iPS:435407 | | NA | GACTATGGCATGCAC | GTTATATGGTATGAAGA AAATAATAAATACTATG CAGACTCCGTGAAGGGC | GAAATAGGGTTCAGTGAGGA CTAC |
| | 21-225_150E7 | | SEQ ID NO:6203 | SEQ ID NO:14215 | SEQ ID NO:22227 |
| | | AA | DYGMH | VIWYEENNKYYADSVKG | EIGFSEDY |
| | | | SEQ ID NO:6204 | SEQ ID NO:14216 | SEQ ID NO:22228 |
| iPS:435409 | | NA | ACCTATAGCATGACT | TACATTAGTAGGAGTAG TAGTACCATATACTACGC AGACTCTGTGAAGGGC | TCGGCATTTAGCCCTTTTGAT TAC |
| | 21-225_150G8 | | SEQ ID NO:6205 | SEQ ID NO:14217 | SEQ ID NO:22229 |
| | | AA | TYSMT | YISRSSSTIYYADSVKG | SAFSPFDY |
| | | | SEQ ID NO:6206 | SEQ ID NO:14218 | SEQ ID NO:22230 |
| iPS:435413 | | NA | AGCTATGGCATGCAC | ATTATATGGTATGATGG AAGTAATAAATACTATG CAGACTCCGTGAAGGGC | GATCGTTACGATTTTTGGAG TGGTCACTTTGACTAC |
| | 21-225_150B11 | | SEQ ID NO:6207 | SEQ ID NO:14219 | SEQ ID NO:22231 |
| | | AA | SYGMH | IIWYDGSNKYYADSVKG | DRYDFWSGHFDY |
| | | | SEQ ID NO:6208 | SEQ ID NO:14220 | SEQ ID NO:22232 |

FIGURE 49

| iPS:435415 | | NA | AGCTATGCCATGAAC | GCTATTAGTGGTAGTGGT GGTAACACATTCTACGC AGACTCCGTGAAGGGC | CGGGTGACGGACTACGGTGG TAACGACTGGTTCGACCCC |
|---|---|---|---|---|---|
| | 21-225_150C11 | | SEQ ID NO:6209 | SEQ ID NO:14221 | SEQ ID NO:22233 |
| | | AA | SYAMN | AISGSGGNTFYADSVKG | RVTDYGGNDWFDP |
| | | | SEQ ID NO:6210 | SEQ ID NO:14222 | SEQ ID NO:22234 |
| iPS:435417 | | NA | AGCTATGGCATGCAC | GTTATATTCTATGATGGA AGTAATAAACACTATGC AGACTCCGTGAAGGGC | AGGTTTAGCAGCAGCTGGTC GGGGGGGTATGGACGTC |
| | 21-225_150D11 | | SEQ ID NO:6211 | SEQ ID NO:14223 | SEQ ID NO:22235 |
| | | AA | SYGMH | VIFYDGSNKHYADSVKG | RFSSSWSGGMDV |
| | | | SEQ ID NO:6212 | SEQ ID NO:14224 | SEQ ID NO:22236 |
| iPS:435419 | | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
| | 21-225_150C12 | | SEQ ID NO:6213 | SEQ ID NO:14225 | SEQ ID NO:22237 |
| | | AA | SYAMS | AISGRGGNTFYADSVKG | RVTDYGGNDWFDP |
| | | | SEQ ID NO:6214 | SEQ ID NO:14226 | SEQ ID NO:22238 |
| iPS:435421 | | NA | AGCTTTAGCATGAAC | TCCATTAGTAGTAGTAGT TATTACATATACTACGCA GACTCAGTGAAGGGC | GATACACCACTGGTTTAC |
| | 21-225_151F1 | | SEQ ID NO:6215 | SEQ ID NO:14227 | SEQ ID NO:22239 |
| | | AA | SFSMN | SISSSSYYIYYADSVKG | DTPLVY |
| | | | SEQ ID NO:6216 | SEQ ID NO:14228 | SEQ ID NO:22240 |
| iPS:435423 | | NA | AGCTATGGCATGCAC | ATTATATGGTATGATGG AAGTAATAAATACTATG CAGACTCCGTGAAGGGC | GATCGATACGATTTTTGGAG TGGTCACTTTGACTAC |
| | 21-225_151G5 | | SEQ ID NO:6217 | SEQ ID NO:14229 | SEQ ID NO:22241 |

FIGURE 49

| | | AA | SYGMH | IIWYDGSNKYYADSVKG | DRYDFWSGHFDY |
|---|---|---|---|---|---|
| | | | SEQ ID NO:6218 | SEQ ID NO:14230 | SEQ ID NO:22242 |
| iPS:435425 | 21-225_151B12 | NA | AGCTATGCCATGAAC | GCTATTAGTGGTAGTGGT AAAAACACATTCTACGC AGACTCCGTGAAGGGC | CGGGTGACGGACTACGGTGG TAACGACTGGTTCGACCCC |
| | | | SEQ ID NO:6219 | SEQ ID NO:14231 | SEQ ID NO:22243 |
| | | AA | SYAMN | AISGSGKNTFYADSVKG | RVTDYGGNDWFDP |
| | | | SEQ ID NO:6220 | SEQ ID NO:14232 | SEQ ID NO:22244 |
| iPS:435427 | 21-225_151C9 | NA | AGCTATGGCATGCAC | GTTATATCATATGATGGA AGTAATAAATACTATGC AGACTCCGTGAAGGGC | GGGGTATTACTATGGTTCGG GGAGCTAGAAGATGACTGGT TCGACCCC |
| | | | SEQ ID NO:6221 | SEQ ID NO:14233 | SEQ ID NO:22245 |
| | | AA | SYGMH | VISYDGSNKYYADSVKG | GVLLWFGELEDDWFDP |
| | | | SEQ ID NO:6222 | SEQ ID NO:14234 | SEQ ID NO:22246 |
| iPS:435429 | 21-225_151A10 | NA | AACTATGGCATGCAC | ATTATATGGTATGATGG AAGTAATAAATACTATG CAGACTCCGTGAAGGGC | GATCATTACGATTTTTGGAG TGGTCACTTTGACTAC |
| | | | SEQ ID NO:6223 | SEQ ID NO:14235 | SEQ ID NO:22247 |
| | | AA | NYGMH | IIWYDGSNKYYADSVKG | DHYDFWSGHFDY |
| | | | SEQ ID NO:6224 | SEQ ID NO:14236 | SEQ ID NO:22248 |
| iPS:435431 | 21-225_152D2 | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
| | | | SEQ ID NO:6225 | SEQ ID NO:14237 | SEQ ID NO:22249 |
| | | AA | SYAMS | AISGRGGNTFYADSVKG | RVTDYGGNDWFDP |
| | | | SEQ ID NO:6226 | SEQ ID NO:14238 | SEQ ID NO:22250 |

FIGURE 49

| iPS:435433 | | NA | AATTATGATATCAAC | TGGATGCACCCTAACAG TGGTAACACAGGCTATG CACAGAAGTTCCAGGGC | AGCAGTGGCTGGTACTTTTT TGACTAC |
|---|---|---|---|---|---|
| | 21-225_152E3 | | SEQ ID NO:6227 | SEQ ID NO:14239 | SEQ ID NO:22251 |
| | | AA | NYDIN | WMHPNSGNTGYAQKFQG | SSGWYFFDY |
| | | | SEQ ID NO:6228 | SEQ ID NO:14240 | SEQ ID NO:22252 |
| iPS:435435 | | NA | AATTATGATATCAAC | TGGATGCACCCTAACAG TGGTAACACAGGCTATG CACAGAAGTTCCAGGGC | AGCAGTGGCTGGTACTGGTT TGACTAC |
| | 21-225_152H3 | | SEQ ID NO:6229 | SEQ ID NO:14241 | SEQ ID NO:22253 |
| | | AA | NYDIN | WMHPNSGNTGYAQKFQG | SSGWYWFDY |
| | | | SEQ ID NO:6230 | SEQ ID NO:14242 | SEQ ID NO:22254 |
| iPS:435437 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | 21-225_152F4 | | SEQ ID NO:6231 | SEQ ID NO:14243 | SEQ ID NO:22255 |
| | | AA | NYDIN | WMHPNSGNTGYAQKFQG | SSGWYWFDP |
| | | | SEQ ID NO:6232 | SEQ ID NO:14244 | SEQ ID NO:22256 |
| iPS:435439 | | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
| | 21-225_152G4 | | SEQ ID NO:6233 | SEQ ID NO:14245 | SEQ ID NO:22257 |
| | | AA | SYAMS | AISGRGGNTFYADSVKG | RVTDYGGNDWFDP |
| | | | SEQ ID NO:6234 | SEQ ID NO:14246 | SEQ ID NO:22258 |
| iPS:435441 | 21 225 152F6 | NA | AGCTATGGCATGCAC | ATTATATGGTATGATGG AAGTTATAAATACTATG CAGACTCCGTGAAGGGC | GAGGGGTACGATTTTTGGAG TGGTTACCTTGGCTAC |

FIGURE 49

| | | | | | |
|---|---|---|---|---|---|
| | 21-225_152F6 | | SEQ ID NO:6235 | SEQ ID NO:14247 | SEQ ID NO:22259 |
| | | AA | SYGMH | IIWYDGSYKYYADSVKG | EGYDFWSGYLGY |
| | | | SEQ ID NO:6236 | SEQ ID NO:14248 | SEQ ID NO:22260 |
| iPS:435443 | 21-225_152E7 | NA | AACAGTGGTTACTACTGGAGC | TACAGTTATTACAGTGGGAGCACCTACTACAACCCGTCCCTCAAGAGT | GGGGGATATAACTGGAACCATGCTTTTGATATC |
| | | | SEQ ID NO:6237 | SEQ ID NO:14249 | SEQ ID NO:22261 |
| | | AA | NSGYYWS | YSYYSGSTYYNPSLKS | GGYNWNHAFDI |
| | | | SEQ ID NO:6238 | SEQ ID NO:14250 | SEQ ID NO:22262 |
| iPS:435445 | 21-225_152F7 | NA | AGCTATATCATGCAC | GTTATATGGTATGATGGAAGTAATAAATACTATGCAGACTCCGTGAAGGGC | GAGGAGTATAGCAGCAGCTGGTACGGGTACGGTATGGACGTC |
| | | | SEQ ID NO:6239 | SEQ ID NO:14251 | SEQ ID NO:22263 |
| | | AA | SYIMH | VIWYDGSNKYYADSVKG | EEYSSSWYGYGMDV |
| | | | SEQ ID NO:6240 | SEQ ID NO:14252 | SEQ ID NO:22264 |
| iPS:435447 | 21-225_152H7 | NA | AGCTATGCCATGAAC | GCTATTAGTGGTAGTGGTGGTAACACATTCTACGCAGACTCCGTGAAGGGC | AAGGATAATGATTACGTTTGGGGGAGTCCTTACTTTGACTAC |
| | | | SEQ ID NO:6241 | SEQ ID NO:14253 | SEQ ID NO:22265 |
| | | AA | SYAMN | AISGSGGNTFYADSVKG | KDNDYVWGSPYFDY |
| | | | SEQ ID NO:6242 | SEQ ID NO:14254 | SEQ ID NO:22266 |
| iPS:435449 | 21-225_152H9 | NA | AGAAGTAGTTACTACTGGGGC | AGTATCTATTATAGTGGGAGCGCCTCCTACAACCCGTCCCTCAAGAGT | CTTGATCTCCAGTGGAGTTTTGACTTC |
| | | | SEQ ID NO:6243 | SEQ ID NO:14255 | SEQ ID NO:22267 |
| | | AA | RSSYYWG | SIYYSGSASYNPSLKS | LDLQWSFDF |
| | | | SEQ ID NO:6244 | SEQ ID NO:14256 | SEQ ID NO:22268 |

FIGURE 49

| iPS:435451 | | NA | AATTACTACTGGAGC | CGTATCGATACCAGTGG GATCACCAACTACAACC CCTCCCTCAAGAGT | GAGGGGGGATTGGGAGCTAC CTTCTTTGACTAC |
|---|---|---|---|---|---|
| | 21-225_152D10 | | SEQ ID NO:6245 | SEQ ID NO:14257 | SEQ ID NO:22269 |
| | | AA | NYYWS | RIDTSGITNYNPSLKS | EGGLGATFFDY |
| | | | SEQ ID NO:6246 | SEQ ID NO:14258 | SEQ ID NO:22270 |
| iPS:435453 | | NA | AGCTATGCCATGAGC | GCTATTAGTGGTCGTGGT GGTAACACATTCTACGC AGACTCCGTGAAGGGC | AAGGATTATGATTACGTTTG GGGGAGTCCTTACTTTGACT AC |
| | 21-225_152G10 | | SEQ ID NO:6247 | SEQ ID NO:14259 | SEQ ID NO:22271 |
| | | AA | SYAMS | AISGRGGNTFYADSVKG | KDYDYVWGSPYFDY |
| | | | SEQ ID NO:6248 | SEQ ID NO:14260 | SEQ ID NO:22272 |
| iPS:435455 | | NA | AGCTATGCCATGAAC | GCTATTAGTGGTCGTGGT GGTAACACATTCTACGC AGACTCCGTGAAGGGC | CGGGTGACGGACTACGGTGG TAACGACTGGTTCGACCCC |
| | 21-225_152B11 | | SEQ ID NO:6249 | SEQ ID NO:14261 | SEQ ID NO:22273 |
| | | AA | SYAMN | AISGRGGNTFYADSVKG | RVTDYGGNDWFDP |
| | | | SEQ ID NO:6250 | SEQ ID NO:14262 | SEQ ID NO:22274 |
| iPS:435457 | | NA | AACTATGGCATGCAC | ATTATATGGTATGATGG AAGTAATAAATACTATG CAGACTCCGTGAAGGGC | GAGGCTTACGATTTTTGGAG TGGTTATTTTGACTAC |
| | 21-225_152C11 | | SEQ ID NO:6251 | SEQ ID NO:14263 | SEQ ID NO:22275 |
| | | AA | NYGMH | IIWYDGSNKYYADSVKG | EAYDFWSGYFDY |
| | | | SEQ ID NO:6252 | SEQ ID NO:14264 | SEQ ID NO:22276 |
| iPS:435459 | | NA | AATTATGATATCAAC | TGGATGAACCCTAACAG TGGTAACACAGGCTATG CACAGAAGTTCCAGGGC | AGCAGTGGCTGGTACTACTT TGACTAC |
| | 21-225_152E12 | | SEQ ID NO:6253 | SEQ ID NO:14265 | SEQ ID NO:22277 |

FIGURE 49

| | | | | | |
|---|---|---|---|---|---|
| | | AA | NYDIN | WMNPNSGNTGYAQKFQG | SSGWYYFDY |
| | | | SEQ ID NO:6254 | SEQ ID NO:14266 | SEQ ID NO:22278 |
| iPS:435461 | 21-225_153A1 | NA | AGCTATGTCATGAGT | GCTATTAGTGGAAGTGGTGATAGAACATACTACGCAGACTCCGTGAAGGGC | ACGGCGACTAAGGACTAC |
| | | | SEQ ID NO:6255 | SEQ ID NO:14267 | SEQ ID NO:22279 |
| | | AA | SYVMS | AISGSGDRTYYADSVKG | TATKDY |
| | | | SEQ ID NO:6256 | SEQ ID NO:14268 | SEQ ID NO:22280 |
| iPS:435463 | 21-225_153D2 | NA | AGCTATGGCATGCAC | ATTATATGGTATGATGGAAGTTATAAATACTATGCAGACTCCGTGAAGGGC | GAGGGGTACGATTTTTGGAGTGGTTACCTTGGCTAC |
| | | | SEQ ID NO:6257 | SEQ ID NO:14269 | SEQ ID NO:22281 |
| | | AA | SYGMH | IIWYDGSYKYYADSVKG | EGYDFWSGYLGY |
| | | | SEQ ID NO:6258 | SEQ ID NO:14270 | SEQ ID NO:22282 |
| iPS:435465 | 21-225_153A6 | NA | AACAGTGGTTACTACTGGAGC | TACAGCTATTACAGTGGGAGCACCTACTACAACCCGTCCCTCAAGAGT | GGGGGATATAACTGGAACCATGCTTTTGATATC |
| | | | SEQ ID NO:6259 | SEQ ID NO:14271 | SEQ ID NO:22283 |
| | | AA | NSGYYWS | YSYYSGSTYYNPSLKS | GGYNWNHAFDI |
| | | | SEQ ID NO:6260 | SEQ ID NO:14272 | SEQ ID NO:22284 |
| iPS:435467 | 21-225_153B9 | NA | AGTTACTACTGGAGC | CGTATCGATACCAGTGGGATCACCAACTACAACCCCTCCCTCAAGAGT | GAGGGGGGGAGTGGGAGCTACGTACTTTGACTAC |
| | | | SEQ ID NO:6261 | SEQ ID NO:14273 | SEQ ID NO:22285 |
| | | AA | SYYWS | RIDTSGITNYNPSLKS | EGGVGATYFDY |
| | | | SEQ ID NO:6262 | SEQ ID NO:14274 | SEQ ID NO:22286 |

FIGURE 49

| iPS:435469 | | NA | AGCTATGGCATGCAC | CTTATATTCTATGATGGA AGTAATAAATACTATGC AGACTCCGTGAAGGGC | CGCTATAGCCGCAGCTGGGC CGGGGGTATGGACGTC |
|---|---|---|---|---|---|
| | 21-225_153G9 | | SEQ ID NO:6263 | SEQ ID NO:14275 | SEQ ID NO:22287 |
| | | AA | SYGMH | LIFYDGSNKYYADSVKG | RYSRSWAGGMDV |
| | | | SEQ ID NO:6264 | SEQ ID NO:14276 | SEQ ID NO:22288 |
| iPS:435471 | | NA | AATTATGATATCAAC | TGGATGCACCCTAACAG TGGTAACACAGGCTATG CACAGAAGTTCCAGGGC | AGCAGTGGCTGGTACTTCTT TGACAAC |
| | 21-225_153F11 | | SEQ ID NO:6265 | SEQ ID NO:14277 | SEQ ID NO:22289 |
| | | AA | NYDIN | WMHPNSGNTGYAQKFQG | SSGWYFFDN |
| | | | SEQ ID NO:6266 | SEQ ID NO:14278 | SEQ ID NO:22290 |
| iPS:435475 | | NA | AATTATGATATCAAC | TGGATGAACCCTAACAG TGGTAACACAGGCTATG CACAGAAGTTCCAGGGC | AGCAGTGGCTGGTACATCTT TGACTAC |
| | 21-225_154H6 | | SEQ ID NO:6267 | SEQ ID NO:14279 | SEQ ID NO:22291 |
| | | AA | NYDIN | WMNPNSGNTGYAQKFQG | SSGWYIFDY |
| | | | SEQ ID NO:6268 | SEQ ID NO:14280 | SEQ ID NO:22292 |
| iPS:435479 | | NA | AGCTATGCCATGAGC | GCTATTAGTGGTCGTGGT GGTAACACATTCTACGC AGACTCCGTGAAGGGC | AGGGGATTTCGATTTTTGGA GTGGTTGGGGGGGCTTTGACT AC |
| | 21-225_154E9 | | SEQ ID NO:6269 | SEQ ID NO:14281 | SEQ ID NO:22293 |
| | | AA | SYAMS | AISGRGGNTFYADSVKG | RGFRFLEWLGGFDY |
| | | | SEQ ID NO:6270 | SEQ ID NO:14282 | SEQ ID NO:22294 |
| iPS:435481 | | NA | AATTATGATATCAAC | TGGATGAACCCTAACAG TGGTAACACAGGCTATG CACAGAAGTTCCAGGGC | AGCAGTGGCTGGTACTACTT CGACTAC |

FIGURE 49

|  | 21-225_154A11 |  | SEQ ID NO:6271 | SEQ ID NO:14283 | SEQ ID NO:22295 |
|---|---|---|---|---|---|
|  |  | AA | NYDIN | WMNPNSGNTGYAQKFQG | SSGWYYFDY |
|  |  |  | SEQ ID NO:6272 | SEQ ID NO:14284 | SEQ ID NO:22296 |
| iPS:435483 | 21-225_155A4 | NA | AGCTATGCCATGAGC | GCTATTAGTGGTAGTGGT GGTAACACATTCTACGC AGACTCCGTGAAGGGC | AAGGATTATGATTACGTTTG GGGGAGTCCTTACTTTGACT AC |
|  |  |  | SEQ ID NO:6273 | SEQ ID NO:14285 | SEQ ID NO:22297 |
|  |  | AA | SYAMS | AISGSGGNTFYADSVKG | KDYDYVWGSPYFDY |
|  |  |  | SEQ ID NO:6274 | SEQ ID NO:14286 | SEQ ID NO:22298 |
| iPS:435485 | 21-225_155B4 | NA | AGCTATGCCATGAGC | GCTATTAGTGGTAGTGGT GGTAACACATTCTACGC AGACTCCGTGAAGGGC | AAGGATTATGATTACGTTTG GGGGAGTCCTTACTTTGACT AC |
|  |  |  | SEQ ID NO:6275 | SEQ ID NO:14287 | SEQ ID NO:22299 |
|  |  | AA | SYAMS | AISGSGGNTFYADSVKG | KDYDYVWGSPYFDY |
|  |  |  | SEQ ID NO:6276 | SEQ ID NO:14288 | SEQ ID NO:22300 |
| iPS:435487 | 21-225_155C4 | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
|  |  |  | SEQ ID NO:6277 | SEQ ID NO:14289 | SEQ ID NO:22301 |
|  |  | AA | SYAMS | AISGRGGNTFYADSVKG | RVTDYGGNDWFDP |
|  |  |  | SEQ ID NO:6278 | SEQ ID NO:14290 | SEQ ID NO:22302 |
| iPS:435489 | 21-225_155A5 | NA | AGCTATGGCATGCAC | ATTATATGGTATGATGG AAGTAGTAAATACTATG CAGACTCCGTGAAGGGC | GATCGATACGATTTTTGGAG TGGTCACTTTGACTAC |
|  |  |  | SEQ ID NO:6279 | SEQ ID NO:14291 | SEQ ID NO:22303 |
|  |  | AA | SYGMH | IIWYDGSSKYYADSVKG | DRYDFWSGHFDY |
|  |  |  | SEQ ID NO:6280 | SEQ ID NO:14292 | SEQ ID NO:22304 |

FIGURE 49

| iPS:435491 | | NA | AATTATGATATCAAC | TGGATGCACCCTAACAG TGGTAGTACAGGCTATG CACAGAGGTTCCAGGGC | AGCAGTGGCTGGTACTATTT TGACTAC |
|---|---|---|---|---|---|
| | 21-225_155E5 | | SEQ ID NO:6281 | SEQ ID NO:14293 | SEQ ID NO:22305 |
| | | AA | NYDIN | WMHPNSGSTGYAQRFQG | SSGWYYFDY |
| | | | SEQ ID NO:6282 | SEQ ID NO:14294 | SEQ ID NO:22306 |
| iPS:435495 | | NA | AATTATGATATCAAC | TGGATGAACCCTAACAG TGGTAACACAGGCTATG CACAGAAGTTCCAGGGC | AGCAGTGGCTGGTACTACTT TGACTAC |
| | 21-225_155B6 | | SEQ ID NO:6283 | SEQ ID NO:14295 | SEQ ID NO:22307 |
| | | AA | NYDIN | WMNPNSGNTGYAQKFQG | SSGWYYFDY |
| | | | SEQ ID NO:6284 | SEQ ID NO:14296 | SEQ ID NO:22308 |
| iPS:435497 | | NA | AGCTATGCCATGAAC | ACTATTAGTGGTAGAGG TCTTGGCACATACTACGC AGACTCCGTGAAGGGC | GACCATGACTACGGTGACTA CAATATCTACTTTGACTAC |
| | 21-225_155H9 | | SEQ ID NO:6285 | SEQ ID NO:14297 | SEQ ID NO:22309 |
| | | AA | SYAMN | TISGRGLGTYYADSVKG | DHDYGDYNIYFDY |
| | | | SEQ ID NO:6286 | SEQ ID NO:14298 | SEQ ID NO:22310 |
| iPS:435499 | | NA | AGAAGTAGTTACTACTGGGG C | AGTATCTATTATAGTGGG AGCGCCTCCTACAACCC GTCCCTCAAGAGT | CTTGATCTCCAGTGGAGTTTT GACTTC |
| | 21-225_156G1 | | SEQ ID NO:6287 | SEQ ID NO:14299 | SEQ ID NO:22311 |
| | | AA | RSSYYWG | SIYYSGSASYNPSLKS | LDLQWSFDF |
| | | | SEQ ID NO:6288 | SEQ ID NO:14300 | SEQ ID NO:22312 |
| iPS:435501 | | NA | AATTATGATATCAAC | TGGGTGCACCCTAACAG TGGTAACACAGGCTATG CACAGAAGTTCCAGGGC | AGCAGTGGCTGGTACTACTT TGACTAC |
| | 21-225_156H1 | | SEQ ID NO:6289 | SEQ ID NO:14301 | SEQ ID NO:22313 |

FIGURE 49

| | | | | | |
|---|---|---|---|---|---|
| | | AA | NYDIN | WVHPNSGNTGYAQKFQG | SSGWYYFDY |
| | | | SEQ ID NO:6290 | SEQ ID NO:14302 | SEQ ID NO:22314 |
| iPS:435503 | 21-225_156E4 | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
| | | | SEQ ID NO:6291 | SEQ ID NO:14303 | SEQ ID NO:22315 |
| | | AA | SYAMS | AISGRGGNTFYADSVKG | RVTDYGGNDWFDP |
| | | | SEQ ID NO:6292 | SEQ ID NO:14304 | SEQ ID NO:22316 |
| iPS:435505 | 21-225_157C1 | NA | AGCTATAGAATGAAC | TCCATGAGTAATAGTAG TAGTTCCATATACTACGC AGACTCAGTGAAGGGC | CAGGCAGCCCAGGACTAC |
| | | | SEQ ID NO:6293 | SEQ ID NO:14305 | SEQ ID NO:22317 |
| | | AA | SYRMN | SMSNSSSSIYYADSVKG | QAAQDY |
| | | | SEQ ID NO:6294 | SEQ ID NO:14306 | SEQ ID NO:22318 |
| iPS:435509 | 21-225_157H1 | NA | AGTTATGCCATGAGG | GATATTAGTGGTAGTGG TGGTACCACATACTACG CAGACTCCGTGAAGGGC | ACCTACCTC |
| | | | SEQ ID NO:6295 | SEQ ID NO:14307 | SEQ ID NO:22319 |
| | | AA | SYAMR | DISGSGGTTYYADSVKG | TYL |
| | | | SEQ ID NO:6296 | SEQ ID NO:14308 | SEQ ID NO:22320 |
| iPS:435511 | 21-225_157C3 | NA | ACCTATGGCATGCAC | GTTATATGGTATGATGTA AATAATAAATACTATGC AGACTCCGTGAAGGGC | GAGCTGGGGTTCCTCTCTGA CTAT |
| | | | SEQ ID NO:6297 | SEQ ID NO:14309 | SEQ ID NO:22321 |
| | | AA | TYGMH | VIWYDVNNKYYADSVKG | ELGFLSDY |
| | | | SEQ ID NO:6298 | SEQ ID NO:14310 | SEQ ID NO:22322 |

FIGURE 49

| iPS:435513 | | NA | ACCTATGCCATGAGC | GTTATTAGTGGTAGTGGT GGTAGCACATACTACGC AGACTCCGTGAAGGGC | AGGAGCAGTGGCTGGTACGA GGATGCTCTTGATATC |
|---|---|---|---|---|---|
| | 21-225_157F3 | | SEQ ID NO:6299 | SEQ ID NO:14311 | SEQ ID NO:22323 |
| | | AA | TYAMS | VISGSGGSTYYADSVKG | RSSGWYEDALDI |
| | | | SEQ ID NO:6300 | SEQ ID NO:14312 | SEQ ID NO:22324 |
| iPS:435515 | | NA | AGCTATACCATGAAC | TCCATTAGTGGTAGTAGT AGTTACATATACTACGC AGACTCAGTGAAGGGC | GTAGCTACCTTTGACTAC |
| | 21-225_157E4 | | SEQ ID NO:6301 | SEQ ID NO:14313 | SEQ ID NO:22325 |
| | | AA | SYTMN | SISGSSSYIYYADSVKG | VATFDY |
| | | | SEQ ID NO:6302 | SEQ ID NO:14314 | SEQ ID NO:22326 |
| iPS:435521 | | NA | AGTTATAGCATGAAC | TCCATTAGTAGTAGTAGT ACTTACATATACTACGCA GACTCAGTGAAGGGC | GATAGAGGGTCCATC |
| | 21-225_157H4 | | SEQ ID NO:6303 | SEQ ID NO:14315 | SEQ ID NO:22327 |
| | | AA | SYSMN | SISSSSTYIYYADSVKG | DRGSI |
| | | | SEQ ID NO:6304 | SEQ ID NO:14316 | SEQ ID NO:22328 |
| iPS:435523 | | NA | AGCTATGTCATGAGC | GCTATGAGTGGTAGTGG TGGTAGAACATACTACG CAGACTCCGTGAAGGGC | TATACCTGGAACGGCTAC |
| | 21-225_157G5 | | SEQ ID NO:6305 | SEQ ID NO:14317 | SEQ ID NO:22329 |
| | | AA | SYVMS | AMSGSGGRTYYADSVKG | YTWNGY |
| | | | SEQ ID NO:6306 | SEQ ID NO:14318 | SEQ ID NO:22330 |
| iPS:435525 | | NA | AGTGGTAGTTACTACTGGGG C | AGTATCTACTATAGTGG GAGCACCTACTACAATC CGTCCCTCAAGAGT | CATAAAGTGGCTGGTCCCTT TGACTAC |
| | 21-225_157E7 | | SEQ ID NO:6307 | SEQ ID NO:14319 | SEQ ID NO:22331 |
| | | AA | SGSYYWG | SIYYSGSTYYNPSLKS | HKVAGPFDY |
| | | | SEQ ID NO:6308 | SEQ ID NO:14320 | SEQ ID NO:22332 |

FIGURE 49

| iPS:435527 | 21-225_157G7 | NA | AGTTATAGCATGAAC | TCCATTAGTGGTAGTAGT ACGTACATATACTACGC AGACTCAGTGAAGGGC | GATCGGGGCAGCAGC |
| | | | SEQ ID NO:6309 | SEQ ID NO:14321 | SEQ ID NO:22333 |
| | | AA | SYSMN | SISGSSTYIYYADSVKG | DRGSS |
| | | | SEQ ID NO:6310 | SEQ ID NO:14322 | SEQ ID NO:22334 |
| iPS:435529 | 21-225_157H7 | NA | AGCTATAGCATGAAC | TGCATTAGTGGTAGTAGT AGTTACATATATTATGCA GACTCAGTGAAGGGC | GATCGAGGGGGCTAT |
| | | | SEQ ID NO:6311 | SEQ ID NO:14323 | SEQ ID NO:22335 |
| | | AA | SYSMN | CISGSSSYIYYADSVKG | DRGGY |
| | | | SEQ ID NO:6312 | SEQ ID NO:14324 | SEQ ID NO:22336 |
| iPS:435531 | 21-225_157G8 | NA | AATTATGGCATGCAC | ATTATATGGTATGATGG AAGTTATAAATACTATG CAGACTCCGTGAAGGGC | GAGGGATACGATTTTTGGAG TGGTTTCTTTGACTCC |
| | | | SEQ ID NO:6313 | SEQ ID NO:14325 | SEQ ID NO:22337 |
| | | AA | NYGMH | IIWYDGSYKYYADSVKG | EGYDFWSGFFDS |
| | | | SEQ ID NO:6314 | SEQ ID NO:14326 | SEQ ID NO:22338 |
| iPS:435533 | 21-225_157H8 | NA | AGCTATGGCATGCAC | GTTATATGGTATGATGTA AATAATAAATACTATGC AGACTCCGTGAAGGGC | GAGCTGGGGTTCCTCTCTGA CTAC |
| | | | SEQ ID NO:6315 | SEQ ID NO:14327 | SEQ ID NO:22339 |
| | | AA | SYGMH | VIWYDVNNKYYADSVKG | ELGFLSDY |
| | | | SEQ ID NO:6316 | SEQ ID NO:14328 | SEQ ID NO:22340 |
| iPS:435535 | 21-225_157H10 | NA | AGCTATACCATGAAC | TCCATTAGTGGTAGCAGT AGTTACATAAACTACGC AGACTCAGTGAAGGGC | GTGGCTCACTTTGACTAC |
| | | | SEQ ID NO:6317 | SEQ ID NO:14329 | SEQ ID NO:22341 |

FIGURE 49

| | | AA | SYTMN | SISGSSSYINYADSVKG | VAHFDY |
|---|---|---|---|---|---|
| | | | SEQ ID NO:6318 | SEQ ID NO:14330 | SEQ ID NO:22342 |
| iPS:435537 | 21-225_157H12 | NA | AGCTATAGCATGAAC | TCCATTAGTGGTAGTGGT AGTTACATAAACTACGC AGACTCAGTGAAGGGC | TCCAAGTTTGACTCC |
| | | | SEQ ID NO:6319 | SEQ ID NO:14331 | SEQ ID NO:22343 |
| | | AA | SYSMN | SISGSGSYINYADSVKG | SKFDS |
| | | | SEQ ID NO:6320 | SEQ ID NO:14332 | SEQ ID NO:22344 |
| iPS:435539 | 21-225_158G1 | NA | AGTTATGGCATGAAC | TCCATTAGTGGTAGTGGT AGTTACATATACTACGC AGACTCAGTGAAGGGC | AGCAGTGGCTGGTCT |
| | | | SEQ ID NO:6321 | SEQ ID NO:14333 | SEQ ID NO:22345 |
| | | AA | SYGMN | SISGSGSYIYYADSVKG | SSGWS |
| | | | SEQ ID NO:6322 | SEQ ID NO:14334 | SEQ ID NO:22346 |
| iPS:435543 | 21-225_158D4 | NA | GACTATGTCATGCAC | GTTATATGGTATGATGG AAGTAATAAATACTATG CAGACTCCGTGAAGGGC | GAACCGTATACTAGTGGCTG GTACGACTACGGTATGGACG TC |
| | | | SEQ ID NO:6323 | SEQ ID NO:14335 | SEQ ID NO:22347 |
| | | AA | DYVMH | VIWYDGSNKYYADSVKG | EPYTSGWYDYGMDV |
| | | | SEQ ID NO:6324 | SEQ ID NO:14336 | SEQ ID NO:22348 |
| iPS:435545 | 21-225_158F4 | NA | AGTCACTTCTGGAGC | CGTATCTATACCAGTGG GACCACCAACTACACCC CCTCCCTCAAGAGT | TTGAGCAGTGGCTGGTTTGA CTAC |
| | | | SEQ ID NO:6325 | SEQ ID NO:14337 | SEQ ID NO:22349 |
| | | AA | SHFWS | RIYTSGTTNYTPSLKS | LSSGWFDY |
| | | | SEQ ID NO:6326 | SEQ ID NO:14338 | SEQ ID NO:22350 |

FIGURE 49

| iPS:435547 | | NA | AGTTATAGCATGAAC | TCCATTAGTGGTAGTAGT ACGTACATATACTACGC AGACTCAGTGAAGGGC | GATCGGGGCAGCAGC |
|---|---|---|---|---|---|
| | 21-225_158F5 | | SEQ ID NO:6327 | SEQ ID NO:14339 | SEQ ID NO:22351 |
| | | AA | SYSMN | SISGSSTYIYYADSVKG | DRGSS |
| | | | SEQ ID NO:6328 | SEQ ID NO:14340 | SEQ ID NO:22352 |
| iPS:435549 | | NA | AGCTATAGCATGAAC | TCCATCAGTGGTAGTAGT ACTTACATATACTACGCA GACTCAGTGAAGGGC | GATCGGGGCAGCAGC |
| | 21-225_158H5 | | SEQ ID NO:6329 | SEQ ID NO:14341 | SEQ ID NO:22353 |
| | | AA | SYSMN | SISGSSTYIYYADSVKG | DRGSS |
| | | | SEQ ID NO:6330 | SEQ ID NO:14342 | SEQ ID NO:22354 |
| iPS:435551 | | NA | AGCTATGGCATGCAC | GTTATATGGTATGATGTA ACTAATAAATACTATGC AGACTCCGTGAAGGGC | GAACTGGGATGGGCGGAGG ACTAC |
| | 21-225_158H6 | | SEQ ID NO:6331 | SEQ ID NO:14343 | SEQ ID NO:22355 |
| | | AA | SYGMH | VIWYDVTNKYYADSVKG | ELGWAEDY |
| | | | SEQ ID NO:6332 | SEQ ID NO:14344 | SEQ ID NO:22356 |
| iPS:435553 | | NA | AGCTATACCATGAAC | TTGATTAGTGGCAGTAGT AGTTACATATACTACGC AGACTCAGTGAAGGGC | GATCGAGGCAGCCTC |
| | 21-225_158G8 | | SEQ ID NO:6333 | SEQ ID NO:14345 | SEQ ID NO:22357 |
| | | AA | SYTMN | LISGSSSYIYYADSVKG | DRGSL |
| | | | SEQ ID NO:6334 | SEQ ID NO:14346 | SEQ ID NO:22358 |
| iPS:435557 | | NA | AATTATGATATCAAC | TGGATGCACCCTAACAG TGGTAACACAGGCTATG CACAGAAGTTCCAGGGC | AGCAGTGGCTGGTACCGCTT TGACTAC |
| | 21-225_158B12 | | SEQ ID NO:6335 | SEQ ID NO:14347 | SEQ ID NO:22359 |

FIGURE 49

| | | AA | NYDIN | WMHPNSGNTGYAQKFQG | SSGWYRFDY |
|---|---|---|---|---|---|
| | | | SEQ ID NO:6336 | SEQ ID NO:14348 | SEQ ID NO:22360 |
| iPS:435559 | 21-225_158H12 | NA | AGCTATGTCATGAGC | GCTATTAGTGGTAGTGGT GGTAGGACAGACTACGC AGACTCCGTAAAGGGC | GGGGGCTGGAACCACGAC |
| | | | SEQ ID NO:6337 | SEQ ID NO:14349 | SEQ ID NO:22361 |
| | | AA | SYVMS | AISGSGGRTDYADSVKG | GGWNHD |
| | | | SEQ ID NO:6338 | SEQ ID NO:14350 | SEQ ID NO:22362 |
| iPS:435561 | 21-225_159F1 | NA | AGCTATAGAATGAAC | TCCATAAGTGGTAGTGG TAATTACATAGACTACG CAGACTCAGTGAAGGGC | GGTTGGGACGTC |
| | | | SEQ ID NO:6339 | SEQ ID NO:14351 | SEQ ID NO:22363 |
| | | AA | SYRMN | SISGSGNYIDYADSVKG | GWDV |
| | | | SEQ ID NO:6340 | SEQ ID NO:14352 | SEQ ID NO:22364 |
| iPS:435563 | 21-225_159H2 | NA | AGTTATGATATCAAC | TGGATGAACCCTAACAG TGGTAACACAGGCTATG TACAGAAGTTCCAGGGC | AAGAAAACTGGGGACTAC |
| | | | SEQ ID NO:6341 | SEQ ID NO:14353 | SEQ ID NO:22365 |
| | | AA | SYDIN | WMNPNSGNTGYVQKFQG | KKTGDY |
| | | | SEQ ID NO:6342 | SEQ ID NO:14354 | SEQ ID NO:22366 |
| iPS:435565 | 21-225_159C4 | NA | AACTATGGCATGCAC | GTTATATCATATTCTGGA AACAATAAATACTATGC AGACTCCGTGAAGGGC | CGGAGCAGCTCGTGGGGGGG CTACGGTATGGACGTC |
| | | | SEQ ID NO:6343 | SEQ ID NO:14355 | SEQ ID NO:22367 |
| | | AA | NYGMH | VISYSGNNKYYADSVKG | RSSSWGGYGMDV |
| | | | SEQ ID NO:6344 | SEQ ID NO:14356 | SEQ ID NO:22368 |

FIGURE 49

| iPS:435569 | | NA | AGCTATGGCATACAC | GTTGTATGGTATGATGTA AATAATAAATACTATGC AGACTCCGTGAAGGGC | GAGCTGGGGTTCCTCTCTGA CTAC |
|---|---|---|---|---|---|
| | 21-225_159C5 | | SEQ ID NO:6345 | SEQ ID NO:14357 | SEQ ID NO:22369 |
| | | AA | SYGIH | VVWYDVNNKYYADSVK G | ELGFLSDY |
| | | | SEQ ID NO:6346 | SEQ ID NO:14358 | SEQ ID NO:22370 |
| iPS:435571 | | NA | GACTATGTCATGCAG | GTTATATGGTATGATGG AAGTAATAAATACTATG CAGACTCCGTGAAGGGC | GAACCGTATAATAGTGGCTG GTACGACTACGGTATGGACG TC |
| | 21-225_159C8 | | SEQ ID NO:6347 | SEQ ID NO:14359 | SEQ ID NO:22371 |
| | | AA | DYVMQ | VIWYDGSNKYYADSVKG | EPYNSGWYDYGMDV |
| | | | SEQ ID NO:6348 | SEQ ID NO:14360 | SEQ ID NO:22372 |
| iPS:435573 | | NA | AACTATGTCATGCAC | GTTATATGGTATGATGG AAGTAATAAATACTATG CAGACTCCGTGAAGGGC | GAACCGTATAGTAGTGGCTG GTACGACTACGGTATGGACG TC |
| | 21-225_159D8 | | SEQ ID NO:6349 | SEQ ID NO:14361 | SEQ ID NO:22373 |
| | | AA | NYVMH | VIWYDGSNKYYADSVKG | EPYSSGWYDYGMDV |
| | | | SEQ ID NO:6350 | SEQ ID NO:14362 | SEQ ID NO:22374 |
| iPS:435575 | | NA | AGCTATACCATGAAC | TCCATTAGTGGTAGTAGT AGTTACATATACTACGC AGACTCAGTGAAGGGC | GTGAGCTGGGCTGACTGC |
| | 21-225_159H11 | | SEQ ID NO:6351 | SEQ ID NO:14363 | SEQ ID NO:22375 |
| | | AA | SYTMN | SISGSSSYIYYADSVKG | VSWADC |
| | | | SEQ ID NO:6352 | SEQ ID NO:14364 | SEQ ID NO:22376 |

FIGURE 49

| iPS:435577 | | NA | AGCTATGGCATGCAC | GTTATATGGTATGATGG AAGTTATAAATACTATG CAGACTCCGTGAAGGGC | GAGGCCTACGATTTTTGGAG TGGTTATTATGACTAC |
|---|---|---|---|---|---|
| | 21-225_160B1 | | SEQ ID NO:6353 | SEQ ID NO:14365 | SEQ ID NO:22377 |
| | | AA | SYGMH | VIWYDGSYKYYADSVKG | EAYDFWSGYYDY |
| | | | SEQ ID NO:6354 | SEQ ID NO:14366 | SEQ ID NO:22378 |
| iPS:435579 | | NA | AGCTATGTCATGAGC | GCTATGAGTGGTAGTGG TGGTCACACATACTACG CAGACTCCGTGAAGGGC | CATGGATACAGC |
| | 21-225_160G1 | | SEQ ID NO:6355 | SEQ ID NO:14367 | SEQ ID NO:22379 |
| | | AA | SYVMS | AMSGSGGHTYYADSVKG | HGYS |
| | | | SEQ ID NO:6356 | SEQ ID NO:14368 | SEQ ID NO:22380 |
| iPS:435581 | | NA | AGCTATCGCATGAAC | TCCATTAGTAGTAGTACT GGTTACATGTACTACGC AGACTCAGTGAAGGGC | GATAAAGATTAC |
| | 21-225_160H1 | | SEQ ID NO:6357 | SEQ ID NO:14369 | SEQ ID NO:22381 |
| | | AA | SYRMN | SISSSTGYMYYADSVKG | DKDY |
| | | | SEQ ID NO:6358 | SEQ ID NO:14370 | SEQ ID NO:22382 |
| iPS:435583 | | NA | AGTTATGGCATGAAC | TCCATTAGTGGTAGTGGT AGTTACATATACTACGC AGACTCAGTGAAGGGC | AGCAGTGGCTGGTCT |
| | 21-225_160F2 | | SEQ ID NO:6359 | SEQ ID NO:14371 | SEQ ID NO:22383 |
| | | AA | SYGMN | SISGSGSYIYYADSVKG | SSGWS |
| | | | SEQ ID NO:6360 | SEQ ID NO:14372 | SEQ ID NO:22384 |
| iPS:435585 | | NA | AGCTATGTCATGAGC | GCTATGAGTGGTAGTGG TGGTCACACATACTACG CAGACTCCGTGAAGGGC | CATGGATACAGC |
| | 21-225_160G3 | | SEQ ID NO:6361 | SEQ ID NO:14373 | SEQ ID NO:22385 |

FIGURE 49

| | | AA | SYVMS | AMSGSGGHTYYADSVKG | HGYS |
|---|---|---|---|---|---|
| | | | SEQ ID NO:6362 | SEQ ID NO:14374 | SEQ ID NO:22386 |
| iPS:435587 | 21-225_160H3 | NA | AGAAGTAGTTACTACTGGGGC | AGTATCTATTATAGTGGGAGTACCTCCTACAACCCGTCTCTCGAGAGT | CTCTCTCAACGGTGGGACTTTGACTAC |
| | | | SEQ ID NO:6363 | SEQ ID NO:14375 | SEQ ID NO:22387 |
| | | AA | RSSYYWG | SIYYSGSTSYNPSLES | LSQRWDFDY |
| | | | SEQ ID NO:6364 | SEQ ID NO:14376 | SEQ ID NO:22388 |
| iPS:435589 | 21-225_160A4 | NA | AATTATGATATCAAC | TGGATGCACCCTAACAGTGGTAACACAGGCTATCCACAGAAGTTCCAGGGC | AGCAGCGGCTGGTACATTTTGACTAC |
| | | | SEQ ID NO:6365 | SEQ ID NO:14377 | SEQ ID NO:22389 |
| | | AA | NYDIN | WMHPNSGNTGYPQKFQG | SSGWYIFDY |
| | | | SEQ ID NO:6366 | SEQ ID NO:14378 | SEQ ID NO:22390 |
| iPS:435591 | 21-225_160C4 | NA | GACTATGTCATGCAG | GTTATATGGTATGATGGAAGTAATAAATACTATGCAGACTCCGTGAAGGGC | GAACCGTATAATAGTGGCTGGTACGACTACGGTATGGACGTC |
| | | | SEQ ID NO:6367 | SEQ ID NO:14379 | SEQ ID NO:22391 |
| | | AA | DYVMQ | VIWYDGSNKYYADSVKG | EPYNSGWYDYGMDV |
| | | | SEQ ID NO:6368 | SEQ ID NO:14380 | SEQ ID NO:22392 |
| iPS:435593 | 21-225_160F4 | NA | AGTTATAGCATGAAC | TCCATTAGTGGTAGTAGTACGTACATATACTACGCAGACTCAGTGAAGGGC | GATCGGGGCAGCAGC |
| | | | SEQ ID NO:6369 | SEQ ID NO:14381 | SEQ ID NO:22393 |
| | | AA | SYSMN | SISGSSTYIYYADSVKG | DRGSS |
| | | | SEQ ID NO:6370 | SEQ ID NO:14382 | SEQ ID NO:22394 |

FIGURE 49

| iPS:435595 | | NA | AGCTATAGGATGAAC | TCCATTAGTGGTAGTAGT AGTTACATAGACTACGC AGACTCAGTGAAGGGC | AAGAGTTGGTTTGACTAC |
|---|---|---|---|---|---|
| | 21-225_160H4 | | SEQ ID NO:6371 | SEQ ID NO:14383 | SEQ ID NO:22395 |
| | | AA | SYRMN | SISGSSSYIDYADSVKG | KSWFDY |
| | | | SEQ ID NO:6372 | SEQ ID NO:14384 | SEQ ID NO:22396 |
| iPS:435599 | | NA | AGAAGTAGCTACTACTGGGG C | AATATCTATTATAGTGGG AGCGCCTACCACATTCC GTCCCTCAAGAGT | CATGACCCAAACTGGGGAGT TGACTAC |
| | 21-225_160B10 | | SEQ ID NO:6373 | SEQ ID NO:14385 | SEQ ID NO:22397 |
| | | AA | RSSYYWG | NIYYSGSAYHIPSLKS | HDPNWGVDY |
| | | | SEQ ID NO:6374 | SEQ ID NO:14386 | SEQ ID NO:22398 |
| iPS:435601 | | NA | AGCTTTGGCATGCAC | GTCATATGGTATGATGG AAGTTATAAATACTATG CAGACTCCGTGAAGGGC | GTTGGTATAGAAGTGGCTGG TGACTACTACTTCGGTATGG AAGTC |
| | 21-225_160G10 | | SEQ ID NO:6375 | SEQ ID NO:14387 | SEQ ID NO:22399 |
| | | AA | SFGMH | VIWYDGSYKYYADSVKG | VGIEVAGDYYFGMEV |
| | | | SEQ ID NO:6376 | SEQ ID NO:14388 | SEQ ID NO:22400 |
| iPS:435605 | | NA | AGCAACTACATGAGC | GTTATTTATACCGGTGGT AGCACATACAACGCAGA CTCCGTGAAGGGC | AATTGGGGAATGGCTGGCCC CTTTGACTAC |
| | 21-225_161A4 | | SEQ ID NO:6377 | SEQ ID NO:14389 | SEQ ID NO:22401 |
| | | AA | SNYMS | VIYTGGSTYNADSVKG | NWGMAGPFDY |
| | | | SEQ ID NO:6378 | SEQ ID NO:14390 | SEQ ID NO:22402 |
| iPS:435607 | | NA | AGCTATGGCATGCAC | GTTATATCATATGGTGGA AGTAATAAATACCATGC AGACTCCGTGAAGGGC | CGGAGCAGCTCGTCGGGGGG CTACGGTATGGACGTC |
| | 21-225_161G4 | | SEQ ID NO:6379 | SEQ ID NO:14391 | SEQ ID NO:22403 |

FIGURE 49

| | | | | | |
|---|---|---|---|---|---|
| | | AA | SYGMH | VISYGGSNKYHADSVKG | RSSSSGGYGMDV |
| | | | SEQ ID NO:6380 | SEQ ID NO:14392 | SEQ ID NO:22404 |
| iPS:435609 | 21-225_161F7 | NA | GACTTTGGCTTGCAC | GTTATATGGTTTGATGGA AGTAATAAATACTATGC AGACTCCGTGAAGGGC | GAGATTGGCTGGCTCTCTGA CTAC |
| | | | SEQ ID NO:6381 | SEQ ID NO:14393 | SEQ ID NO:22405 |
| | | AA | DFGLH | VIWFDGSNKYYADSVKG | EIGWLSDY |
| | | | SEQ ID NO:6382 | SEQ ID NO:14394 | SEQ ID NO:22406 |
| iPS:435611 | 21-225_161F10 | NA | AGCTATGGCATGCAC | ATTATATCATATTCTGGA AGAAATGATTTCTATGC AGACTCCGTGAAGGGC | CGTATAGCAGCAGCTGGTCA CTACGGTATGGACGTC |
| | | | SEQ ID NO:6383 | SEQ ID NO:14395 | SEQ ID NO:22407 |
| | | AA | SYGMH | IISYSGRNDFYADSVKG | RIAAAGHYGMDV |
| | | | SEQ ID NO:6384 | SEQ ID NO:14396 | SEQ ID NO:22408 |
| iPS:435613 | 21-225_161D11 | NA | GACTTTGGCTTGCAC | GTTATATGGTTTGATGGA AGTAATAAATACTATGC AGACTCCGTGAAGGGC | GAGATTGGCTGGCTCTCTGA CTAC |
| | | | SEQ ID NO:6385 | SEQ ID NO:14397 | SEQ ID NO:22409 |
| | | AA | DFGLH | VIWFDGSNKYYADSVKG | EIGWLSDY |
| | | | SEQ ID NO:6386 | SEQ ID NO:14398 | SEQ ID NO:22410 |
| iPS:435615 | 21-225_161G12 | NA | GACTATGTCATGCAG | GTTATATGGTATGATGG AAGTAATAAATACTATG CAGACTCCGTGAAGGGC | GAACCGTATAATAGTGGCTG GTACGACTACGGTATGGACG TC |
| | | | SEQ ID NO:6387 | SEQ ID NO:14399 | SEQ ID NO:22411 |
| | | AA | DYVMQ | VIWYDGSNKYYADSVKG | EPYNSGWYDYGMDV |
| | | | SEQ ID NO:6388 | SEQ ID NO:14400 | SEQ ID NO:22412 |

FIGURE 49

| iPS:435617 | 21-225_162F2 | NA | AGTTATAGCATGAAC | TCCATTAGTGGTAGTAGT ACGTACATATACTACGC AGACTCAGTGAAGGGC | GATCGGGGCAGCAGC |
|---|---|---|---|---|---|
| | | | SEQ ID NO:6389 | SEQ ID NO:14401 | SEQ ID NO:22413 |
| | | AA | SYSMN | SISGSSTYIYYADSVKG | DRGSS |
| | | | SEQ ID NO:6390 | SEQ ID NO:14402 | SEQ ID NO:22414 |
| iPS:435621 | 21-225_162H3 | NA | AGTTATAGCATGAAC | TCCATTAGTGGTAGTAGT ACGTACATATACTACGC AGACTCAGTGAAGGGC | GATCGGGGCAGCAGC |
| | | | SEQ ID NO:6391 | SEQ ID NO:14403 | SEQ ID NO:22415 |
| | | AA | SYSMN | SISGSSTYIYYADSVKG | DRGSS |
| | | | SEQ ID NO:6392 | SEQ ID NO:14404 | SEQ ID NO:22416 |
| iPS:435623 | 21-225_162D5 | NA | AATTATGATATCAAC | TGGATGCACCCTAACAG TGGTAACACAGGCTATG CACAGAAGTTCCAGGGC | AGCAGTGGCTGGTACTTCTT TGACTAC |
| | | | SEQ ID NO:6393 | SEQ ID NO:14405 | SEQ ID NO:22417 |
| | | AA | NYDIN | WMHPNSGNTGYAQKFQG | SSGWYFFDY |
| | | | SEQ ID NO:6394 | SEQ ID NO:14406 | SEQ ID NO:22418 |
| iPS:435627 | 21-225_162F6 | NA | AATTATGATATCAAC | TGGATGCACCCTAACAG TGGTAACACAGGCTATG CACAGAAGTTCCAGGGC | AGCAGTGGCTGGTACCGCTT TGACTAC |
| | | | SEQ ID NO:6395 | SEQ ID NO:14407 | SEQ ID NO:22419 |
| | | AA | NYDIN | WMHPNSGNTGYAQKFQG | SSGWYRFDY |
| | | | SEQ ID NO:6396 | SEQ ID NO:14408 | SEQ ID NO:22420 |
| iPS:435629 | 21 225 162H6 | NA | AACTATGGCATGCAC | GTTATATGGTATGATGG AAGTAATAAATACTATG CAGACTCCGTGAAGGGC | AAAGGTATAGCAGCAGTTGG AGACTACTACTACGGTATGG ACGTC |

FIGURE 49

| | | | | | |
|---|---|---|---|---|---|
| | 21-225_162H6 | | SEQ ID NO:6397 | SEQ ID NO:14409 | SEQ ID NO:22421 |
| | | AA | NYGMH | VIWYDGSNKYYADSVKG | KGIAAVGDYYYGMDV |
| | | | SEQ ID NO:6398 | SEQ ID NO:14410 | SEQ ID NO:22422 |
| iPS:435635 | 21-225_163F1 | NA | AGCTATAGCATGAAC | TCCATTAGTGGTAGTGGTAGTTACATATACTACGCAGACTCAGTGAAGGGC | TATAGCAGCTCGCACTAT |
| | | | SEQ ID NO:6399 | SEQ ID NO:14411 | SEQ ID NO:22423 |
| | | AA | SYSMN | SISGSGSYIYYADSVKG | YSSSHY |
| | | | SEQ ID NO:6400 | SEQ ID NO:14412 | SEQ ID NO:22424 |
| iPS:435637 | 21-225_163E2 | NA | AGCTATAGCATGAAC | TCCACTAGTGGGAGTTCTACTTACATATACTACGCAGACTCAGTGAAGGGC | GATCGAGGCAGCCTC |
| | | | SEQ ID NO:6401 | SEQ ID NO:14413 | SEQ ID NO:22425 |
| | | AA | SYSMN | STSGSSTYIYYADSVKG | DRGSL |
| | | | SEQ ID NO:6402 | SEQ ID NO:14414 | SEQ ID NO:22426 |
| iPS:435639 | 21-225_163G6 | NA | AGTTATAGCATGAGC | TCCATTAGTGGTAGTAGTGCTTACATATACTACGCAGACTCAGTGAAGGGC | TTGAGCGGTATGGACGTC |
| | | | SEQ ID NO:6403 | SEQ ID NO:14415 | SEQ ID NO:22427 |
| | | AA | SYSMS | SISGSSAYIYYADSVKG | LSGMDV |
| | | | SEQ ID NO:6404 | SEQ ID NO:14416 | SEQ ID NO:22428 |
| iPS:435641 | 21-225_163F9 | NA | AGCTATAGCATGAAC | TCCATTAGTGGTAGTAGTACTTACATATACTACGCAGACTCAGTGAAGGGC | GATCGAGGCAGCCTC |
| | | | SEQ ID NO:6405 | SEQ ID NO:14417 | SEQ ID NO:22429 |
| | | AA | SYSMN | SISGSSTYIYYADSVKG | DRGSL |
| | | | SEQ ID NO:6406 | SEQ ID NO:14418 | SEQ ID NO:22430 |

FIGURE 49

| iPS:435643 | 21-225_163G10 | NA | AGCTATAGCATGAAC | TCCATTAGTGGTAGTAGT ACTTACATATACTACGCA GACTCAGTGAAGGGC | GCCCGTATGGACGTC |
| | | | SEQ ID NO:6407 | SEQ ID NO:14419 | SEQ ID NO:22431 |
| | | AA | SYSMN | SISGSSTYIYYADSVKG | ARMDV |
| | | | SEQ ID NO:6408 | SEQ ID NO:14420 | SEQ ID NO:22432 |
| iPS:435649 | 21-225_165H2 | NA | CATTATGATATCAAC | TGGATGCACCCTAACAG TCATAAGACAGGCTATG CACAGAAGTTCCAGGGC | AGCAGTGGCTGGTACATGTT TGACTAC |
| | | | SEQ ID NO:6409 | SEQ ID NO:14421 | SEQ ID NO:22433 |
| | | AA | HYDIN | WMHPNSHKTGYAQKFQG | SSGWYMFDY |
| | | | SEQ ID NO:6410 | SEQ ID NO:14422 | SEQ ID NO:22434 |
| iPS:435653 | 21-225_166H12 | NA | AGCTATAGCATGAGC | TCCATTAGTGGGAGTAG TAGTTACAGTTACTACGC AGACTCAGTGAAGGGC | CTAACTGGCTTTGACTAC |
| | | | SEQ ID NO:6411 | SEQ ID NO:14423 | SEQ ID NO:22435 |
| | | AA | SYSMS | SISGSSSYSYYADSVKG | LTGFDY |
| | | | SEQ ID NO:6412 | SEQ ID NO:14424 | SEQ ID NO:22436 |
| iPS:435655 | 21-225_167E2 | NA | AGCTTTGGCATGCAC | GTCATATGGTATGATGG AACTTATAAATACTATGC AGACTCCGTGAAGGGC | GTTGGTATTGAAGTGGCTGG TGACTACTACTACGGTATGG AAGTC |
| | | | SEQ ID NO:6413 | SEQ ID NO:14425 | SEQ ID NO:22437 |
| | | AA | SFGMH | VIWYDGTYKYYADSVKG | VGIEVAGDYYYGMEV |
| | | | SEQ ID NO:6414 | SEQ ID NO:14426 | SEQ ID NO:22438 |
| iPS:435657 | 21-225_167H10 | NA | AGCTTTGGCATGCAC | GTCATATGGTATGATGG AAGTTATAAGTACCATG CAGACTCCGTGAAGGGC | GTTGGTATAGAAGTGGCTGG TGACTACTACTACGGTATGG AAGTC |
| | | | SEQ ID NO:6415 | SEQ ID NO:14427 | SEQ ID NO:22439 |

FIGURE 49

| | | | | | | |
|---|---|---|---|---|---|---|
| | | AA | SFGMH | VIWYDGSYKYHADSVKG | VGIEVAGDYYYGMEV | |
| | | | SEQ ID NO:6416 | SEQ ID NO:14428 | SEQ ID NO:22440 | |
| iPS:435659 | | NA | AGCTATGTCATGAGC | GCTATGAGTGGTAGTGG TGGTAGAACATACTACG CAGACTCCGTGAAGGGC | TATACCTGGAACGGCTAC | |
| | 21-225_167D12 | | SEQ ID NO:6417 | SEQ ID NO:14429 | SEQ ID NO:22441 | |
| | | AA | SYVMS | AMSGSGGRTYYADSVKG | YTWNGY | |
| | | | SEQ ID NO:6418 | SEQ ID NO:14430 | SEQ ID NO:22442 | |
| iPS:435663 | | NA | AGCTATGGCATGCAC | GTTATATGGTATGATGG AAGTAATAAATACTATG CAGACTCCGTGAGGGGC | GATCCCTTACGTGGATACAA TGACCCGGTTATGGACTAC | |
| | 21-225_169B1 | | SEQ ID NO:6419 | SEQ ID NO:14431 | SEQ ID NO:22443 | |
| | | AA | SYGMH | VIWYDGSNKYYADSVRG | DPLRGYNDPVMDY | |
| | | | SEQ ID NO:6420 | SEQ ID NO:14432 | SEQ ID NO:22444 | |
| iPS:435665 | | NA | AGTTACTACTGGAGT | CGTATCGATACCAGTGG GATCACCAACTACAACC CCTCCCTCAAGAGT | GAGGGAGGAGTGGGAGCTA CCTACTTTGACTAC | |
| | 21-225_169F2 | | SEQ ID NO:6421 | SEQ ID NO:14433 | SEQ ID NO:22445 | |
| | | AA | SYYWS | RIDTSGITNYNPSLKS | EGGVGATYFDY | |
| | | | SEQ ID NO:6422 | SEQ ID NO:14434 | SEQ ID NO:22446 | |
| iPS:435667 | | NA | GGCCATAGCATGAAC | TACATTAGCCTTAGTGGT AGTACCATAAAGTACGC AGACTCTGTGAAGGGC | AGGGGGATTACTGTGGTTCG GAATGAGGACGGTTTGGACG TC | |
| | 21-225_169E3 | | SEQ ID NO:6423 | SEQ ID NO:14435 | SEQ ID NO:22447 | |
| | | AA | GHSMN | YISLSGSTIKYADSVKG | RGITVVRNEDGLDV | |
| | | | SEQ ID NO:6424 | SEQ ID NO:14436 | SEQ ID NO:22448 | |

FIGURE 49

| iPS:435669 | | NA | ACCTATGGCATGCAC | ATTATATGGTATGATGG AACTAATAAATACTATG CAGACTCCGTGAAGGGC | GATCCCTTACGTGGATACAA TGACCCGGTTATGGACTAC |
|---|---|---|---|---|---|
| | 21-225_169F9 | | SEQ ID NO:6425 | SEQ ID NO:14437 | SEQ ID NO:22449 |
| | | AA | TYGMH | IIWYDGTNKYYADSVKG | DPLRGYNDPVMDY |
| | | | SEQ ID NO:6426 | SEQ ID NO:14438 | SEQ ID NO:22450 |
| iPS:435671 | | NA | AGTTACTACTGGAGT | CGTATCGATACCAGTGG GATCACCAACTACAACC CCTCCCTCAAGAGT | GAGGGAGGAGTGGGAGCTA CCTACTTTGACTAC |
| | 21-225_169H5 | | SEQ ID NO:6427 | SEQ ID NO:14439 | SEQ ID NO:22451 |
| | | AA | SYYWS | RIDTSGITNYNPSLKS | EGGVGATYFDY |
| | | | SEQ ID NO:6428 | SEQ ID NO:14440 | SEQ ID NO:22452 |
| iPS:435673 | | NA | GGCCATAGCATGAAC | TACATTAGCATTAGTAGT AGTACCATAAAGTACGC AGACTCTGTGAAGGGC | AGGGGGGATTACTGTGGTTCG GAATGAGGACGGTTTGGACG TC |
| | 21-225_169E6 | | SEQ ID NO:6429 | SEQ ID NO:14441 | SEQ ID NO:22453 |
| | | AA | GHSMN | YISISSSTIKYADSVKG | RGITVVRNEDGLDV |
| | | | SEQ ID NO:6430 | SEQ ID NO:14442 | SEQ ID NO:22454 |
| iPS:435675 | | NA | AGTTACTACTGGACC | CGTATCTATACCAGTGG GAGCACCAACTACAACC CCTCCCTCAAGAGT | GTCGGGGAGGTACTACTACGG TATGGACGTC |
| | 21-225_169D7 | | SEQ ID NO:6431 | SEQ ID NO:14443 | SEQ ID NO:22455 |
| | | AA | SYYWT | RIYTSGSTNYNPSLKS | VGRYYYGMDV |
| | | | SEQ ID NO:6432 | SEQ ID NO:14444 | SEQ ID NO:22456 |
| iPS:435677 | | NA | GGCTACTTTATGCAC | TGGATCAAGCCTAAGAG TGGTGGCACAAACTCTG CACAGAGGTTTCAGGGC | GGGGGGGACTACGGTGGCTAC GTGGGGGGGTCTTTGACTAC |
| | 21-225_169C10 | | SEQ ID NO:6433 | SEQ ID NO:14445 | SEQ ID NO:22457 |
| | | AA | GYFMH | WIKPKSGGTNSAQRFQG | GGTTVATWGVFDY |

FIGURE 49

| | | | SEQ ID NO:6434 | SEQ ID NO:14446 | SEQ ID NO:22458 |
|---|---|---|---|---|---|
| iPS:435679 | 21-225_169D10 | NA | AGCTATGTCATGAGT | GCTATTAGTGGTAGTGGT AGTAGAATATACTACGC GGACTCCGTGAAGGGC | GTGGCTTTCTTTGACTAT |
| | | | SEQ ID NO:6435 | SEQ ID NO:14447 | SEQ ID NO:22459 |
| | | AA | SYVMS | AISGSGSRIYYADSVKG | VAFFDY |
| | | | SEQ ID NO:6436 | SEQ ID NO:14448 | SEQ ID NO:22460 |
| iPS:435681 | 21-225_169D11 | NA | GACTATGTCATGCAC | GTTATATGGTATGATGG AAGTAATAAATACTATG CAGACTCCGTGAAGGGC | GAAAGGTATAGCAGTGGCTG GTACGACTACGGTATGGACG TC |
| | | | SEQ ID NO:6437 | SEQ ID NO:14449 | SEQ ID NO:22461 |
| | | AA | DYVMH | VIWYDGSNKYYADSVKG | ERYSSGWYDYGMDV |
| | | | SEQ ID NO:6438 | SEQ ID NO:14450 | SEQ ID NO:22462 |
| iPS:435683 | 21-225_170A1 | NA | AGCTATGGCATGCAC | ATTATATGGTATGATGG AAGTTATAAATACTATG CAGATTCCGTGAAGGGC | GATGCCCACGATTTTTGGAG TGGTTACTTTGACTCC |
| | | | SEQ ID NO:6439 | SEQ ID NO:14451 | SEQ ID NO:22463 |
| | | AA | SYGMH | IIWYDGSYKYYADSVKG | DAHDFWSGYFDS |
| | | | SEQ ID NO:6440 | SEQ ID NO:14452 | SEQ ID NO:22464 |
| iPS:435685 | 21-225_170E1 | NA | AGCTATGTCATGAGT | GCTATTAGTGGTAGTGGT AATAGAATATACTACGC AGACTCCGTGAAGGGC | GTGGCTTTCTTTGACTAT |
| | | | SEQ ID NO:6441 | SEQ ID NO:14453 | SEQ ID NO:22465 |
| | | AA | SYVMS | AISGSGNRIYYADSVKG | VAFFDY |
| | | | SEQ ID NO:6442 | SEQ ID NO:14454 | SEQ ID NO:22466 |
| iPS:435687 | 21 225 170H1 | NA | AGTTATTACTGGAGC | CGTATCTATACCAGTGG GAGCACCAACTACAACC CCTCCCTCAAGAGT | GTCGGGAGGTACTACTATGG TATGGACGTC |

FIGURE 49

| | 21-225_170H1 | | SEQ ID NO:6443 | SEQ ID NO:14455 | SEQ ID NO:22467 |
|---|---|---|---|---|---|
| | | AA | SYYWS | RIYTSGSTNYNPSLKS | VGRYYYGMDV |
| iPS:435689 | | NA | SEQ ID NO:6444 GACTATGTCATGCAC | SEQ ID NO:14456 GTTATATGGTATGATGG AAGTAATAAATACTATG AAGACTCCGTGAAGGGC | SEQ ID NO:22468 GAGACGTATAGCAGCAGCTG GTACGACTACGGTATGGACG TC |
| | 21-225_170F3 | | SEQ ID NO:6445 | SEQ ID NO:14457 | SEQ ID NO:22469 |
| | | AA | DYVMH | VIWYDGSNKYYEDSVKG | ETYSSSWYDYGMDV |
| iPS:435693 | | NA | SEQ ID NO:6446 ACCTATGGCATGCAC | SEQ ID NO:14458 ATTATATGGTATGATGG AACTAATAAATACTATG CAGACTCCGTGAAGGGC | SEQ ID NO:22470 GATCCCTTACGTGGATACAA TGACCCGGTTATGGACTAC |
| | 21-225_170G4 | | SEQ ID NO:6447 | SEQ ID NO:14459 | SEQ ID NO:22471 |
| | | AA | TYGMH | IIWYDGTNKYYADSVKG | DPLRGYNDPVMDY |
| iPS:435695 | | NA | SEQ ID NO:6448 AGCTATGGCATGCAC | SEQ ID NO:14460 ATTATATGGTATGATGG AACTAATAAATACTATG CAGACTCCGTGAAGGGC | SEQ ID NO:22472 GATCCCTTACGTGGATACAA TGACCCGGTTATGGACTAC |
| | 21-225_170D5 | | SEQ ID NO:6449 | SEQ ID NO:14461 | SEQ ID NO:22473 |
| | | AA | SYGMH | IIWYDGTNKYYADSVKG | DPLRGYNDPVMDY |
| iPS:435697 | | NA | SEQ ID NO:6450 ACCTATGGCATGCAC | SEQ ID NO:14462 ATTATATGGTATGATGG GACTAATAAATACTATG CAGACTCCGTGAAGGGC | SEQ ID NO:22474 GATCCCTTACGTGGATACAA TGACCCGGTTATGGACTAC |
| | 21-225_170G5 | | SEQ ID NO:6451 | SEQ ID NO:14463 | SEQ ID NO:22475 |
| | | AA | TYGMH | IIWYDGTNKYYADSVKG | DPLRGYNDPVMDY |

FIGURE 49

| | | | SEQ ID NO:6452 | SEQ ID NO:14464 | SEQ ID NO:22476 |
|---|---|---|---|---|---|
| iPS:435699 | | NA | GGCTACTTTATACAC | TGGATCAAGCCTAACAG TGGTGGCACAAACTCTG CACAGAGGTTTCAGGGC | GGGGGGGACTACGGTGGCTAC GTGGGGGGGTCTTTGACTAC |
| | 21-225_170D6 | | SEQ ID NO:6453 | SEQ ID NO:14465 | SEQ ID NO:22477 |
| | | AA | GYFIH | WIKPNSGGTNSAQRFQG | GGTTVATWGVFDY |
| | | | SEQ ID NO:6454 | SEQ ID NO:14466 | SEQ ID NO:22478 |
| iPS:435701 | | NA | AATTATGATATCAAC | TGGATGAACCCTAACAG TGGTAACACAGGCTATG CACAGAAGTTCCAGGAC | AGCAGTGGCTGGTACTGGTT CGACCCC |
| | 21-225_170F6 | | SEQ ID NO:6455 | SEQ ID NO:14467 | SEQ ID NO:22479 |
| | | AA | NYDIN | WMNPNSGNTGYAQKFQD | SSGWYWFDP |
| | | | SEQ ID NO:6456 | SEQ ID NO:14468 | SEQ ID NO:22480 |
| iPS:435703 | | NA | AGCTATGGCATGCAC | ATTATATGGTATGATGG AACTAATAAATACTATG CAGACTCCGTGAAGGGC | GATCCCTTACGTGGATACAA TGACCCGGTTATGGACTAC |
| | 21-225_170D11 | | SEQ ID NO:6457 | SEQ ID NO:14469 | SEQ ID NO:22481 |
| | | AA | SYGMH | IIWYDGTNKYYADSVKG | DPLRGYNDPVMDY |
| | | | SEQ ID NO:6458 | SEQ ID NO:14470 | SEQ ID NO:22482 |
| iPS:435705 | | NA | ACCTATGGCATGCAC | ATTATATGGTATGATGG GACTAATAAATACTATG CAGACTCCGTGAAGGGC | GATCCCTTACGTGGATACAA TGACCCGGTTATGGACTAC |
| | 21-225_171C3 | | SEQ ID NO:6459 | SEQ ID NO:14471 | SEQ ID NO:22483 |
| | | AA | TYGMH | IIWYDGTNKYYADSVKG | DPLRGYNDPVMDY |
| | | | SEQ ID NO:6460 | SEQ ID NO:14472 | SEQ ID NO:22484 |

FIGURE 49

| iPS:435709 | | NA | ACCTATGGCATGCAC | ATTATATGGTATGATGG AAGTAATAAATACTATG CAGACTCCGTGAAGGGC | GATCCCTTACGTGGATACAA TGACCCGGTTATGGACTAC |
|---|---|---|---|---|---|
| | 21-225_171A4 | | SEQ ID NO:6461 | SEQ ID NO:14473 | SEQ ID NO:22485 |
| | | AA | TYGMH | IIWYDGSNKYYADSVKG | DPLRGYNDPVMDY |
| | | | SEQ ID NO:6462 | SEQ ID NO:14474 | SEQ ID NO:22486 |
| iPS:435711 | | NA | AGCTGTGCCATGACC | GCTATTAGTGGTCGTGGT GGTACCACGTTCTACGC AGACTCCGTGAGGGGC | GATCTTATTGGGGGAGCTAC TTACTTTGACTAC |
| | 21-225_171G4 | | SEQ ID NO:6463 | SEQ ID NO:14475 | SEQ ID NO:22487 |
| | | AA | SCAMT | AISGRGGTTFYADSVRG | DLIGGATYFDY |
| | | | SEQ ID NO:6464 | SEQ ID NO:14476 | SEQ ID NO:22488 |
| iPS:435713 | | NA | AGCTATGGCATGCAC | GTTATATCATATGACGG AAACAATAGACACTATG CAGACTCCGTGCAGGGC | GATCGTCACCGTTTGGACTA CTACGCTTTGGACGTC |
| | 21-225_171D7 | | SEQ ID NO:6465 | SEQ ID NO:14477 | SEQ ID NO:22489 |
| | | AA | SYGMH | VISYDGNNRHYADSVQG | DRHRLDYYALDV |
| | | | SEQ ID NO:6466 | SEQ ID NO:14478 | SEQ ID NO:22490 |
| iPS:435715 | | NA | AGCTCTGCCATGAGC | GTTATTAGTGGTAGTGGT GGTAGCACATTCTACAC AGACTCCGTGAAGGGC | TCGAATAGCAGTGGCTGGTT TGACTAC |
| | 21-225_171A8 | | SEQ ID NO:6467 | SEQ ID NO:14479 | SEQ ID NO:22491 |
| | | AA | SSAMS | VISGSGGSTFYTDSVKG | SNSSGWFDY |
| | | | SEQ ID NO:6468 | SEQ ID NO:14480 | SEQ ID NO:22492 |
| iPS:435717 | | NA | AGCTATGCCATGACT | GCTATTAGTGGTAGTGGT GGTAACACATTCAACGC AGACTCCGTGAAGGGC | CTGGGGATCGACTACTACTA CTACGGTATGGACGTC |
| | 21-225_171A9 | | SEQ ID NO:6469 | SEQ ID NO:14481 | SEQ ID NO:22493 |

FIGURE 49

| | | AA | SYAMT | AISGSGGNTFNADSVKG | LGIDYYYYGMDV |
|---|---|---|---|---|---|
| | | | SEQ ID NO:6470 | SEQ ID NO:14482 | SEQ ID NO:22494 |
| iPS:435719 | 21-225_171A11 | NA | AGCTATAGCATGAAC | TCCATTAGTGGTAGTAGT AGTTACATATACTACGC AGACTCAGTGAAGGGC | GATAGGGGCAGCTCC |
| | | | SEQ ID NO:6471 | SEQ ID NO:14483 | SEQ ID NO:22495 |
| | | AA | SYSMN | SISGSSSYIYYADSVKG | DRGSS |
| | | | SEQ ID NO:6472 | SEQ ID NO:14484 | SEQ ID NO:22496 |
| iPS:435721 | 21-225_172B3 | NA | ACCTATGGCATGCAC | GTTATATGGTATGATGG AAGTAATAAATACTATG CAGACTCCGTGAAGGGC | GATCCCTTACGTGGATACAA TGACCCGGTTATGGACTAC |
| | | | SEQ ID NO:6473 | SEQ ID NO:14485 | SEQ ID NO:22497 |
| | | AA | TYGMH | VIWYDGSNKYYADSVKG | DPLRGYNDPVMDY |
| | | | SEQ ID NO:6474 | SEQ ID NO:14486 | SEQ ID NO:22498 |
| iPS:435723 | 21-225_172B7 | NA | AACTATGGCATGCAC | ATTATATGGTATGATGG AAGTAATAAATACTATG TAGACTCCGTGAAGGGC | GAGGCGTACGATTTTTGGAG TGGTTATTGGGACTAC |
| | | | SEQ ID NO:6475 | SEQ ID NO:14487 | SEQ ID NO:22499 |
| | | AA | NYGMH | IIWYDGSNKYYVDSVKG | EAYDFWSGYWDY |
| | | | SEQ ID NO:6476 | SEQ ID NO:14488 | SEQ ID NO:22500 |
| iPS:435725 | 21-225_172G8 | NA | ACCTATGGCATGCAC | ATTATATGGTATGATGG AACTAATAAATACTATG CAGACTCCGTGAAGGGC | GATCCCTTACGTGGATACAA TGACCCGGTTATGGACTAC |
| | | | SEQ ID NO:6477 | SEQ ID NO:14489 | SEQ ID NO:22501 |
| | | AA | TYGMH | IIWYDGTNKYYADSVKG | DPLRGYNDPVMDY |
| | | | SEQ ID NO:6478 | SEQ ID NO:14490 | SEQ ID NO:22502 |

FIGURE 49

| iPS:435727 | | NA | AATTATGATATCAAC | TGGATGCACCCTAACAG TGGTAACACAGGCTATG CACAGAAGTTCCAGGGC | AGCAGTGGCTGGTACCGGTT TGACTAC |
|---|---|---|---|---|---|
| | 21-225_172E11 | | SEQ ID NO:6479 | SEQ ID NO:14491 | SEQ ID NO:22503 |
| | | AA | NYDIN | WMHPNSGNTGYAQKFQG | SSGWYRFDY |
| | | | SEQ ID NO:6480 | SEQ ID NO:14492 | SEQ ID NO:22504 |
| iPS:435729 | | NA | AGCTATGCCATGAGC | TTTATTAGTGGTAGTGGT GGTAACACATTCTACGC AGACTCCGTGAAGGGC | AGGGATACCTACAACGGTTG GGATGCTTTTGATATC |
| | 21-225_173E7 | | SEQ ID NO:6481 | SEQ ID NO:14493 | SEQ ID NO:22505 |
| | | AA | SYAMS | FISGSGGNTFYADSVKG | RDTYNGWDAFDI |
| | | | SEQ ID NO:6482 | SEQ ID NO:14494 | SEQ ID NO:22506 |
| iPS:435731 | | NA | AGCTATGGCATGCAC | ATTATATGGTATGATGG AAGTAATAAATACTATG CAGACTCCGTGAAGGGC | GAGGCCTACGATTTTTGGAG TGGTTTCTTTGACTCC |
| | 21-225_173A11 | | SEQ ID NO:6483 | SEQ ID NO:14495 | SEQ ID NO:22507 |
| | | AA | SYGMH | IIWYDGSNKYYADSVKG | EAYDFWSGFFDS |
| | | | SEQ ID NO:6484 | SEQ ID NO:14496 | SEQ ID NO:22508 |
| iPS:435733 | | NA | AGCTATGGCATACAC | CTTATATTTTATGATGGA AGTAATAAATACTATGC AGACTCCGTGAAGGGC | CGGTATAGCAGCAGCTGGTC CGGTGGTATGGACGTC |
| | 21-225_173C11 | | SEQ ID NO:6485 | SEQ ID NO:14497 | SEQ ID NO:22509 |
| | | AA | SYGIH | LIFYDGSNKYYADSVKG | RYSSSWSGGMDV |
| | | | SEQ ID NO:6486 | SEQ ID NO:14498 | SEQ ID NO:22510 |

FIGURE 49

| iPS:435735 | | NA | AGCTATGGCATGCAC | ATTATATGGTATGATGG AACTAACAAATACTATG CAGACTCCGTGAAGGGC | GATCCCTTACGTGGATACAA TGACCCGGTTATGGACTAC |
|---|---|---|---|---|---|
| | 21-225_173H12 | | SEQ ID NO:6487 | SEQ ID NO:14499 | SEQ ID NO:22511 |
| | | AA | SYGMH | IIWYDGTNKYYADSVKG | DPLRGYNDPVMDY |
| | | | SEQ ID NO:6488 | SEQ ID NO:14500 | SEQ ID NO:22512 |
| iPS:435737 | | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | 21-225_174G5 | | SEQ ID NO:6489 | SEQ ID NO:14501 | SEQ ID NO:22513 |
| | | AA | NYDIN | WMHPNSGNTGYAQKFQG | SSGWYWFDP |
| | | | SEQ ID NO:6490 | SEQ ID NO:14502 | SEQ ID NO:22514 |
| iPS:435739 | | NA | AGCTCTGCCATGAGC | GTTATTAGTGGTAGTGGT GGTAGCACATTCTACAC AGACTCCGTGAAGGGC | TCGAATAGCAGTGGCTGGTT TGACTAC |
| | 21-225_174G7 | | SEQ ID NO:6491 | SEQ ID NO:14503 | SEQ ID NO:22515 |
| | | AA | SSAMS | VISGSGGSTFYTDSVKG | SNSSGWFDY |
| | | | SEQ ID NO:6492 | SEQ ID NO:14504 | SEQ ID NO:22516 |
| iPS:435741 | | NA | GACTATGTCATGCAC | GTTATATGGTATGATGG AAGTAATAAATACTATG CAGACTCCGTGAAGGGC | GAAAGGTATAGCAGTGGCTG GTACGACTACGGTATGGACG TC |
| | 21-225_174G10 | | SEQ ID NO:6493 | SEQ ID NO:14505 | SEQ ID NO:22517 |
| | | AA | DYVMH | VIWYDGSNKYYADSVKG | ERYSSGWYDYGMDV |
| | | | SEQ ID NO:6494 | SEQ ID NO:14506 | SEQ ID NO:22518 |

FIGURE 49

| iPS:435743 | | NA | ACCTATGGCATGCAC | GTTATATGGTATGATGG AAGTAATAAATACTATG CAGACTCCGTGAAGGGC | GATCCCTTACGTGGATACAA TGACCCGGTTATGGACTAC |
|---|---|---|---|---|---|
| | 21-225_175G1 | | SEQ ID NO:6495 | SEQ ID NO:14507 | SEQ ID NO:22519 |
| | | AA | TYGMH | VIWYDGSNKYYADSVKG | DPLRGYNDPVMDY |
| | | | SEQ ID NO:6496 | SEQ ID NO:14508 | SEQ ID NO:22520 |
| iPS:435745 | | NA | GGCTACTTTATGCAC | TGGATCAAGCCTAAAAG TGGTGGCACAAACTGTG CACAGAGGTTTCAGGGC | GGGGGGGACTACGGTGACTAC GTGGGGGGGTCTTTGACTAC |
| | 21-225_175G3 | | SEQ ID NO:6497 | SEQ ID NO:14509 | SEQ ID NO:22521 |
| | | AA | GYFMH | WIKPKSGGTNCAQRFQG | GGTTVTTWGVFDY |
| | | | SEQ ID NO:6498 | SEQ ID NO:14510 | SEQ ID NO:22522 |
| iPS:435747 | | NA | AGCTATGTCATGAGC | GCTATTAGTGGTAGTGGT GATAGAACATACTACGC AGACTCCGTGAAGGGC | ACAGCGGGCTTTGACTAC |
| | 21-225_175C4 | | SEQ ID NO:6499 | SEQ ID NO:14511 | SEQ ID NO:22523 |
| | | AA | SYVMS | AISGSGDRTYYADSVKG | TAGFDY |
| | | | SEQ ID NO:6500 | SEQ ID NO:14512 | SEQ ID NO:22524 |
| iPS:435749 | | NA | AGCTATGCCATGAGC | TCTATTAGTGGTCGTGGT GGTAGCACGTTCTACGC AGACTCCGTGAAGGGC | TCGAATAGCAGTGGCTGGTT TGACTAC |
| | 21-225_175C10 | | SEQ ID NO:6501 | SEQ ID NO:14513 | SEQ ID NO:22525 |
| | | AA | SYAMS | SISGRGGSTFYADSVKG | SNSSGWFDY |
| | | | SEQ ID NO:6502 | SEQ ID NO:14514 | SEQ ID NO:22526 |
| iPS:435751 | | NA | AATTATGATCTCAAC | TGGATGCACCCTAACAG TGGTAACACAGGCTATG CACAGAAGTTCCAGGGC | AGCAGTGGCTGGTACTACTT TGACTAC |

FIGURE 49

| | 21-225_175D10 | | SEQ ID NO:6503 | SEQ ID NO:14515 | SEQ ID NO:22527 |
|---|---|---|---|---|---|
| | | AA | NYDLN | WMHPNSGNTGYAQKFQG | SSGWYYFDY |
| | | | SEQ ID NO:6504 | SEQ ID NO:14516 | SEQ ID NO:22528 |
| iPS:435753 | 21-225_175G10 | NA | AGCTATGCCATGAGC | ATTATTAGTGGTAGTGGT GGTAACACATACTACGC AGACTCCGTGAAGGGC | AGGGATACCTGGAACGGTTG GGATGCTTTTGATATC |
| | | | SEQ ID NO:6505 | SEQ ID NO:14517 | SEQ ID NO:22529 |
| | | AA | SYAMS | IISGSGGNTYYADSVKG | RDTWNGWDAFDI |
| | | | SEQ ID NO:6506 | SEQ ID NO:14518 | SEQ ID NO:22530 |
| iPS:435755 | 21-225_176H4 | NA | AGCTATGGCATGCAC | ATTATATGGTATGATGG AAGTTATAAATACTATG CAGACTCCGTGAAGGGC | GATGCCCACGATTTTTGGAG TGGTTACTTTGCCTAC |
| | | | SEQ ID NO:6507 | SEQ ID NO:14519 | SEQ ID NO:22531 |
| | | AA | SYGMH | IIWYDGSYKYYADSVKG | DAHDFWSGYFAY |
| | | | SEQ ID NO:6508 | SEQ ID NO:14520 | SEQ ID NO:22532 |
| iPS:435759 | 21-225_176E6 | NA | GGCCATAGTATGAAC | TACATTAGCATTAGTGGT AGTACCATAAAGTACGC AGACTCTGTGAAGGGC | AGGGGGATTACTGTGGTTCG GAATGAGGACGGTTTGGACG TC |
| | | | SEQ ID NO:6509 | SEQ ID NO:14521 | SEQ ID NO:22533 |
| | | AA | GHSMN | YISISGSTIKYADSVKG | RGITVVRNEDGLDV |
| | | | SEQ ID NO:6510 | SEQ ID NO:14522 | SEQ ID NO:22534 |
| iPS:435761 | 21-225_176B11 | NA | ACCTATGGCATGCAC | ATTATATGGTATGATGG AACTAATAAATACTATG CAGACTCCGTGAAGGGC | GATCCCTTACGTGGATACAA TGACCCGGTTTTGGACTAC |
| | | | SEQ ID NO:6511 | SEQ ID NO:14523 | SEQ ID NO:22535 |
| | | AA | TYGMH | IIWYDGTNKYYADSVKG | DPLRGYNDPVLDY |
| | | | SEQ ID NO:6512 | SEQ ID NO:14524 | SEQ ID NO:22536 |

FIGURE 49

| iPS:435763 | | NA | GACTATGTCATGCAC | GTTATATGGTATGATGG AAGTAATAAATACTATG CCGACTCCGTGAAGGGC | GAAAAGTATAGCAGCAACTG GTACGACTACGGTATGGACG TC |
| | 21-225_176H12 | | SEQ ID NO:6513 | SEQ ID NO:14525 | SEQ ID NO:22537 |
| | | AA | DYVMH | VIWYDGSNKYYADSVKG | EKYSSNWYDYGMDV |
| | | | SEQ ID NO:6514 | SEQ ID NO:14526 | SEQ ID NO:22538 |
| iPS:435765 | | NA | AGCTATGTCATGAAC | GGTATGAGCGGTAGTGG TGGTAGAACATACTACG CAGACTCCGTGAAGGAC | GTGACTTTCTTTGACTAT |
| | 21-225_177D3 | | SEQ ID NO:6515 | SEQ ID NO:14527 | SEQ ID NO:22539 |
| | | AA | SYVMN | GMSGSGGRTYYADSVKD | VTFFDY |
| | | | SEQ ID NO:6516 | SEQ ID NO:14528 | SEQ ID NO:22540 |
| iPS:435767 | | NA | GATTATGTCATGCAC | GTTATATGGTATGATGG AAGTAATAAATACTATG CAGACTCCGTGAAGGGC | GAAAAGTATAGCAGCAGCTG GTACGACTACGGTTTGGACG TC |
| | 21-225_177B4 | | SEQ ID NO:6517 | SEQ ID NO:14529 | SEQ ID NO:22541 |
| | | AA | DYVMH | VIWYDGSNKYYADSVKG | EKYSSSWYDYGLDV |
| | | | SEQ ID NO:6518 | SEQ ID NO:14530 | SEQ ID NO:22542 |
| iPS:435769 | | NA | AGTTATGCCATGAGC | GTTATTAGTGGTAGTGGT AGTAACACATACTACGT AGACTCCGTGAAGGGC | GGTTACTATGATAGTAGTGG TTATTACTACCCTTTTGACTT C |
| | 21-225_177B6 | | SEQ ID NO:6519 | SEQ ID NO:14531 | SEQ ID NO:22543 |
| | | AA | SYAMS | VISGSGSNTYYVDSVKG | GYYDSSGYYYPFDF |
| | | | SEQ ID NO:6520 | SEQ ID NO:14532 | SEQ ID NO:22544 |

FIGURE 49

| iPS:435771 | | NA | AGCTATGGCATGCAC | ATTATATGGTATGATGG AAGTTATAAATACTATA CAGACTCCGTGAAGGGC | GAGACTTACGATTTTTGGAG TGGTTATTTTGTCTTC |
|---|---|---|---|---|---|
| | 21-225_177B11 | | SEQ ID NO:6521 | SEQ ID NO:14533 | SEQ ID NO:22545 |
| | | AA | SYGMH | IIWYDGSYKYYTDSVKG | ETYDFWSGYFVF |
| | | | SEQ ID NO:6522 | SEQ ID NO:14534 | SEQ ID NO:22546 |
| iPS:435773 | | NA | AATTATGATATCAAC | TGGATGCACCCTAACAG TGGTAACACAGGCTATG CACAGAAGTTCCAGGGC | AGCAGTGGCTGGTACTACTT TGACTTC |
| | 21-225_177B12 | | SEQ ID NO:6523 | SEQ ID NO:14535 | SEQ ID NO:22547 |
| | | AA | NYDIN | WMHPNSGNTGYAQKFQG | SSGWYYFDF |
| | | | SEQ ID NO:6524 | SEQ ID NO:14536 | SEQ ID NO:22548 |
| iPS:435775 | | NA | AGCTATGCCATGACC | GTTATTAGTGGTAGTGGT GGTAATACATTCTACGC AGACTCCGTGAAGGGC | CGGGACGGTGACTACTTTGA CTAC |
| | 21-225_178A5 | | SEQ ID NO:6525 | SEQ ID NO:14537 | SEQ ID NO:22549 |
| | | AA | SYAMT | VISGSGGNTFYADSVKG | RDGDYFDY |
| | | | SEQ ID NO:6526 | SEQ ID NO:14538 | SEQ ID NO:22550 |
| iPS:435777 | | NA | AGCTATGCCATGACC | GTTATTAGTGGTAGTGGT GGTAACACATTCTACGC AGACTCCGTGAAGGGC | CGGTACGGTGACTACTTTGA CTAC |
| | 21-225_178F7 | | SEQ ID NO:6527 | SEQ ID NO:14539 | SEQ ID NO:22551 |
| | | AA | SYAMT | VISGSGGNTFYADSVKG | RYGDYFDY |
| | | | SEQ ID NO:6528 | SEQ ID NO:14540 | SEQ ID NO:22552 |
| iPS:435779 | | NA | ACCTATGGCATGCAC | ATTATATGGTATGATGG AACTAATAAATACTATG CAGACTCCGTGAAGGGC | GATCCCTTACGTGGATACAA TGACCCGGTTATGGACTAC |
| | 21-225_178B10 | | SEQ ID NO:6529 | SEQ ID NO:14541 | SEQ ID NO:22553 |

FIGURE 49

| | | AA | TYGMH | IIWYDGTNKYYADSVKG | DPLRGYNDPVMDY |
|---|---|---|---|---|---|
| | | | SEQ ID NO:6530 | SEQ ID NO:14542 | SEQ ID NO:22554 |
| iPS:435781 | 21-225_178G10 | NA | ACCTATGGCATGCAC | GTTATATGGTATGATGG AAGTAATAAATACTACG CAGACTCCGTGAAGGGC | GAACGGTACGATTTTTGGAG TGGTCATTTTGACTAC |
| | | | SEQ ID NO:6531 | SEQ ID NO:14543 | SEQ ID NO:22555 |
| | | AA | TYGMH | VIWYDGSNKYYADSVKG | ERYDFWSGHFDY |
| | | | SEQ ID NO:6532 | SEQ ID NO:14544 | SEQ ID NO:22556 |
| iPS:435783 | 21-225_179G1 | NA | AGCTATGCCATGACC | GTTATTAGTGGTTTTGGT GGTAACACATTCTACGC AGACTCCGTGAAGGGC | CGGTACGGTGACTACTTTGA CTAC |
| | | | SEQ ID NO:6533 | SEQ ID NO:14545 | SEQ ID NO:22557 |
| | | AA | SYAMT | VISGFGGNTFYADSVKG | RYGDYFDY |
| | | | SEQ ID NO:6534 | SEQ ID NO:14546 | SEQ ID NO:22558 |
| iPS:435785 | 21-225_179C2 | NA | AGCTATGGCATGCAC | CTTATATTTTATGATGGA AGTAATAAATACTATGC AGACTCCGTGAAGGGC | CGGTATAGCGGCAGCTGGTC CGGTGGTATGGACGTC |
| | | | SEQ ID NO:6535 | SEQ ID NO:14547 | SEQ ID NO:22559 |
| | | AA | SYGMH | LIFYDGSNKYYADSVKG | RYSGSWSGGMDV |
| | | | SEQ ID NO:6536 | SEQ ID NO:14548 | SEQ ID NO:22560 |
| iPS:435787 | 21-225_180A3 | NA | AGCTTTGCCATGAAC | GTTATTAGCGGTCGCGGT GGTAACACATTCTACGC AGACTCCGTGAAGGGC | CGGACTGGGGATGATGTTTT TGATGTC |
| | | | SEQ ID NO:6537 | SEQ ID NO:14549 | SEQ ID NO:22561 |
| | | AA | SFAMN | VISGRGGNTFYADSVKG | RTGDDVFDV |
| | | | SEQ ID NO:6538 | SEQ ID NO:14550 | SEQ ID NO:22562 |

FIGURE 49

| iPS:435789 | | NA | GCCTATGGCATGCAC | ATTATTTGGTATGATGGA AGTTATAAATACTATGC AGACTCCGTGAAGGGC | ACCGGTGTGGATCCCTGGGA CTACTACAACGGAATGGACG TC |
|---|---|---|---|---|---|
| | 21-225_180C4 | | SEQ ID NO:6539 | SEQ ID NO:14551 | SEQ ID NO:22563 |
| | | AA | AYGMH | IIWYDGSYKYYADSVKG | TGVDPWDYYNGMDV |
| | | | SEQ ID NO:6540 | SEQ ID NO:14552 | SEQ ID NO:22564 |
| iPS:435791 | | NA | GACTATGGCATGCAC | GTTATATGGTATGATGA AAATAATAAACACTATG CAGACTCCGCGAAGGGC | GAGGTTGGCTGGTCCGATGA CTAC |
| | 21-225_180H7 | | SEQ ID NO:6541 | SEQ ID NO:14553 | SEQ ID NO:22565 |
| | | AA | DYGMH | VIWYDENNKHYADSAKG | EVGWSDDY |
| | | | SEQ ID NO:6542 | SEQ ID NO:14554 | SEQ ID NO:22566 |
| iPS:435793 | | NA | AGCTATGGCATGCAC | GTTATATGGTATGATGG AAGTGATAAATACTATG AAGACTCCGTGAAGGGC | GATCATCCCCGGTGGAGCTA CGGAGACTAC |
| | 21-225_180F8 | | SEQ ID NO:6543 | SEQ ID NO:14555 | SEQ ID NO:22567 |
| | | AA | SYGMH | VIWYDGSDKYYEDSVKG | DHPRWSYGDY |
| | | | SEQ ID NO:6544 | SEQ ID NO:14556 | SEQ ID NO:22568 |
| iPS:435795 | | NA | AGCTATGGCATGCAC | ATTATATGGTATGATGG AAGTTATAAATACTATG CAGACTCCGTGAAGGGC | GATCATTACGATTTTTGGAG TGGGCACTTTGACTTC |
| | 21-225_181C2 | | SEQ ID NO:6545 | SEQ ID NO:14557 | SEQ ID NO:22569 |
| | | AA | SYGMH | IIWYDGSYKYYADSVKG | DHYDFWSGHFDF |
| | | | SEQ ID NO:6546 | SEQ ID NO:14558 | SEQ ID NO:22570 |

FIGURE 49

| iPS:435797 | | NA | AGCTACAATATGCAC | TGGATCAACCCTAACAA TGGTGGCTCAAACTATA CACAGAAGTTTCAGGGC | AAGTTTGGGGAC |
|---|---|---|---|---|---|
| | 21-225_181G2 | | SEQ ID NO:6547 | SEQ ID NO:14559 | SEQ ID NO:22571 |
| | | AA | SYNMH | WINPNNGGSNYTQKFQG | KFGD |
| | | | SEQ ID NO:6548 | SEQ ID NO:14560 | SEQ ID NO:22572 |
| iPS:435799 | | NA | AGCTATGCCATGAGT | GTTATTAGTGGTAGTGGT GGTAACACATTCTACGG AGACTCCGTGAAGGGC | CGGGAGACCTACGACTGGGG ATCCGATGCTTTTGATATC |
| | 21-225_181G3 | | SEQ ID NO:6549 | SEQ ID NO:14561 | SEQ ID NO:22573 |
| | | AA | SYAMS | VISGSGGNTFYGDSVKG | RETYDWGSDAFDI |
| | | | SEQ ID NO:6550 | SEQ ID NO:14562 | SEQ ID NO:22574 |
| iPS:435801 | | NA | AATTATGATATCAAC | TGGATGAACCCTAACAG TGGTAACACAGGCTATG CACAGAAGTTCCAGGGC | AGTAGTGGCTGGTACATCTT TGACTAC |
| | 21-225_181E5 | | SEQ ID NO:6551 | SEQ ID NO:14563 | SEQ ID NO:22575 |
| | | AA | NYDIN | WMNPNSGNTGYAQKFQG | SSGWYIFDY |
| | | | SEQ ID NO:6552 | SEQ ID NO:14564 | SEQ ID NO:22576 |
| iPS:435805 | | NA | GACTATGGCATGCAC | GTTATATGGTATGATGA AAATAATAAACACTATG CAGACTCCGCGAAGGGC | GAGGTTGGCTGGTCCGATGA CTAC |
| | 21-225_181A8 | | SEQ ID NO:6553 | SEQ ID NO:14565 | SEQ ID NO:22577 |
| | | AA | DYGMH | VIWYDENNKHYADSAKG | EVGWSDDY |
| | | | SEQ ID NO:6554 | SEQ ID NO:14566 | SEQ ID NO:22578 |

FIGURE 49

| iPS:435807 | | NA | AGCTATGGCATGCAC | ATTATATGGTATGATGG AAGTTATAAATACTATG CAGACTCCGTGAAGGGC | GATCATTACGATTTTTGGAG TGGGCACTTTGACTAC |
|---|---|---|---|---|---|
| | 21-225_181C10 | | SEQ ID NO:6555 | SEQ ID NO:14567 | SEQ ID NO:22579 |
| | | AA | SYGMH | IIWYDGSYKYYADSVKG | DHYDFWSGHFDY |
| | | | SEQ ID NO:6556 | SEQ ID NO:14568 | SEQ ID NO:22580 |
| iPS:435809 | | NA | AGCTATGCCATGAGT | GTTATTAGTGGTAGAGG TGGTACCACATTCTACGC AGACTCCGTGAAGGGC | CGGACTGGGGATGATGTTTT TGATATC |
| | 21-225_182H5 | | SEQ ID NO:6557 | SEQ ID NO:14569 | SEQ ID NO:22581 |
| | | AA | SYAMS | VISGRGGTTFYADSVKG | RTGDDVFDI |
| | | | SEQ ID NO:6558 | SEQ ID NO:14570 | SEQ ID NO:22582 |
| iPS:435811 | | NA | AGCTATGGCATGCAC | ATTATATCATATGCTGGA AGTACTAAATTCTATGCA GACTCCGTGAAGGGC | AGGCCCCCGCAGTGGCTGGT AGAGGGCTACGGTATGGACG TC |
| | 21-225_183H6 | | SEQ ID NO:6559 | SEQ ID NO:14571 | SEQ ID NO:22583 |
| | | AA | SYGMH | IISYAGSTKFYADSVKG | RPPQWLVEGYGMDV |
| | | | SEQ ID NO:6560 | SEQ ID NO:14572 | SEQ ID NO:22584 |
| iPS:435813 | | NA | AGCTATGGCATGCAC | GTTATATCATCTGCTGGA AGTAATAAATACTATGC AGACTCCGTGAAGGGC | AGGTATAGCAGTGGCTGGGA CTGGTTCGACCCC |
| | 21-225_183A12 | | SEQ ID NO:6561 | SEQ ID NO:14573 | SEQ ID NO:22585 |
| | | AA | SYGMH | VISSAGSNKYYADSVKG | RYSSGWDWFDP |
| | | | SEQ ID NO:6562 | SEQ ID NO:14574 | SEQ ID NO:22586 |
| iPS:435815 | | NA | GACTATAGCATGAAC | TCTATTAGTAGTGGTAGT GGTTACATACACTACGC AGACTCAGTGAAGGGC | GCAACTATGGCCCTTGACTA C |
| | 21-225_190G10 | | SEQ ID NO:6563 | SEQ ID NO:14575 | SEQ ID NO:22587 |
| | | AA | DYSMN | SISSGSGYIHYADSVKG | ATMALDY |

FIGURE 49

| | | | SEQ ID NO:6564 | SEQ ID NO:14576 | SEQ ID NO:22588 |
|---|---|---|---|---|---|
| iPS:435817 | | NA | AATTACTACTGGAGC | CGTATCTATACCAGTGG GAGCACCAACTACAACC CCTCCCTCAAGAGT | GATCGGGGATACTATGGCTA CTACGGTATGGACGTC |
| | 21-225_190B11 | | SEQ ID NO:6565 | SEQ ID NO:14577 | SEQ ID NO:22589 |
| | | AA | NYYWS | RIYTSGSTNYNPSLKS | DRGYYGYYGMDV |
| | | | SEQ ID NO:6566 | SEQ ID NO:14578 | SEQ ID NO:22590 |
| iPS:435819 | | NA | AGCTATGGCATGCAC | GTTATATGGTATGATGG AAGTAATAAAAACTATG CAGACTCCGTGAAGGGC | GATCAGGGCGTGGGCTACGA CGGTTTGGACGTC |
| | 21-225_190C11 | | SEQ ID NO:6567 | SEQ ID NO:14579 | SEQ ID NO:22591 |
| | | AA | SYGMH | VIWYDGSNKNYADSVKG | DQGVGYDGLDV |
| | | | SEQ ID NO:6568 | SEQ ID NO:14580 | SEQ ID NO:22592 |
| iPS:435821 | | NA | AACTATGGCATGCAC | ATTATATGGTTTGATGGA AGTAATAAATACTATGC AGACTCCGTGAAGGGC | GCCCAGGGGGTCTACTACTA CGTTATGGACGTC |
| | 21-225_190E11 | | SEQ ID NO:6569 | SEQ ID NO:14581 | SEQ ID NO:22593 |
| | | AA | NYGMH | IIWFDGSNKYYADSVKG | AQGVYYYVMDV |
| | | | SEQ ID NO:6570 | SEQ ID NO:14582 | SEQ ID NO:22594 |
| iPS:435823 | | NA | AGCTATGCCATGAAC | ACTATTAGTGGTACTGGT CGTAGGACATACTACGC AGACTCCGTGAAGGGC | GAGGAGGATTACTATGATAG TAGTGGCCCGGGGTTCGACC CC |
| | 21-225_190F11 | | SEQ ID NO:6571 | SEQ ID NO:14583 | SEQ ID NO:22595 |
| | | AA | SYAMN | TISGTGRRTYYADSVKG | EEDYYDSSGPGFDP |
| | | | SEQ ID NO:6572 | SEQ ID NO:14584 | SEQ ID NO:22596 |

FIGURE 49

| iPS:435825 | | NA | ATTTATGGCATGCAC | GTTATATGGTATGATGG AAGTAATAAAAACTATG CAGACTCCGTGAAGGGC | GATCAGGGCGTGGGCTACGA CGGTTTGGACGTC |
|---|---|---|---|---|---|
| | 21-225_190G11 | | SEQ ID NO:6573 | SEQ ID NO:14585 | SEQ ID NO:22597 |
| | | AA | IYGMH | VIWYDGSNKNYADSVKG | DQGVGYDGLDV |
| | | | SEQ ID NO:6574 | SEQ ID NO:14586 | SEQ ID NO:22598 |
| iPS:435827 | | NA | AGCTACCACTGGAGC | CTTATCTATACCAGTAGG AGCACCATTTACAACCC CTCCCTCAAGAGT | CTCCGGTATAACTGGAACTT CCCTTACTTTGACTAC |
| | 21-225_190H11 | | SEQ ID NO:6575 | SEQ ID NO:14587 | SEQ ID NO:22599 |
| | | AA | SYHWS | LIYTSRSTIYNPSLKS | LRYNWNFPYFDY |
| | | | SEQ ID NO:6576 | SEQ ID NO:14588 | SEQ ID NO:22600 |
| iPS:435829 | | NA | AGTGGTGGTTACTACTGGAA C | TATATCTATTACAGTGGG AGCACCTACTACAACCC GTCCCTCAAGAGT | TCCGGGTATAATTGGGACGC CGGGGTCGACCCC |
| | 21-225_190B12 | | SEQ ID NO:6577 | SEQ ID NO:14589 | SEQ ID NO:22601 |
| | | AA | SGGYYWN | YIYYSGSTYYNPSLKS | SGYNWDAGVDP |
| | | | SEQ ID NO:6578 | SEQ ID NO:14590 | SEQ ID NO:22602 |
| iPS:435831 | | NA | AGCTATGGCATGCAC | GTTATATCATATGATGGA GGTTATAAAAACTATGT AGACTCCGTGAAGGGC | GGTACCCACGGGTACTACTA CGGTGTGGACGTC |
| | 21-225_190C12 | | SEQ ID NO:6579 | SEQ ID NO:14591 | SEQ ID NO:22603 |
| | | AA | SYGMH | VISYDGGYKNYVDSVKG | GTHGYYYGVDV |
| | | | SEQ ID NO:6580 | SEQ ID NO:14592 | SEQ ID NO:22604 |
| iPS:435833 | | NA | AGCTATGCCATGAGC | GCTATTATTGGTAATGGT GGTAGGACATACTACGC AGACTCCGTGAAGGGC | GATATGGGTAGATACAGCTA TGGTTTCTTTGACTAC |
| | 21-225_190D12 | | SEQ ID NO:6581 | SEQ ID NO:14593 | SEQ ID NO:22605 |

FIGURE 49

|  |  | AA | SYAMS | AIIGNGGRTYYADSVKG | DMGRYSYGFFDY |
|---|---|---|---|---|---|
|  |  |  | SEQ ID NO:6582 | SEQ ID NO:14594 | SEQ ID NO:22606 |
| iPS:435835 | 21-225_190F12 | NA | AACTATGGCATGCAC | GTTATATGGTTTGATGGA AGTAATGACTACTATGC AGACTCCGTGAAGGGC | GATAGAAGCGTCGGCTACGA CGGTTTAGATGTC |
|  |  |  | SEQ ID NO:6583 | SEQ ID NO:14595 | SEQ ID NO:22607 |
|  |  | AA | NYGMH | VIWFDGSNDYYADSVKG | DRSVGYDGLDV |
|  |  |  | SEQ ID NO:6584 | SEQ ID NO:14596 | SEQ ID NO:22608 |
| iPS:435837 | 21-225_198G3 | NA | ACCTATGGCATGCAC | GTTATATGGTATGATGG AACTAATAAAAACTATG CAGACTCCGTGAAGGGC | GATCAGGGCGTGGGCTACGA CGGTTTGGACGTC |
|  |  |  | SEQ ID NO:6585 | SEQ ID NO:14597 | SEQ ID NO:22609 |
|  |  | AA | TYGMH | VIWYDGTNKNYADSVKG | DQGVGYDGLDV |
|  |  |  | SEQ ID NO:6586 | SEQ ID NO:14598 | SEQ ID NO:22610 |
| iPS:435839 | 21-225_191B1 | NA | AGTTATCACTGGAGC | CATATCTATACCAGTGG GAGCACCAAGTACAACC CCTCCCTCAAGAGT | CTCCGGTATAACTGGAACTT CCCTTTCTTTGACTAT |
|  |  |  | SEQ ID NO:6587 | SEQ ID NO:14599 | SEQ ID NO:22611 |
|  |  | AA | SYHWS | HIYTSGSTKYNPSLKS | LRYNWNFPFFDY |
|  |  |  | SEQ ID NO:6588 | SEQ ID NO:14600 | SEQ ID NO:22612 |
| iPS:435841 | 21-225_191D8 | NA | AATTATGATATCAAC | TGGATGCACCCTAACAG TGGTAACACAGGCTATG CACAGAAGTTCCAGGGC | AGCAGTGGCTGGTACTACTT TGACTAC |
|  |  |  | SEQ ID NO:6589 | SEQ ID NO:14601 | SEQ ID NO:22613 |
|  |  | AA | NYDIN | WMHPNSGNTGYAQKFQG | SSGWYYFDY |
|  |  |  | SEQ ID NO:6590 | SEQ ID NO:14602 | SEQ ID NO:22614 |

FIGURE 49

| iPS:435843 | 21-225_191F1 | NA | AGTGGTGGTTACTACTGGAAC | TACATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6591 | SEQ ID NO:14603 | SEQ ID NO:22615 |
| | | AA | SGGYYWN | YIFYSGSTYYNPSLKS | GDYDGSGSYHYYYGMDV |
| | | | SEQ ID NO:6592 | SEQ ID NO:14604 | SEQ ID NO:22616 |
| iPS:435845 | 21-225_191G1 | NA | AGCTATGGCATGCAC | GTTATATGGTATGATGGAAGTAATGAACACTATGCAGACTCCGTGAAGGGC | GATAGGGGGGTGGGTTACTACGGTTTGGACGTC |
| | | | SEQ ID NO:6593 | SEQ ID NO:14605 | SEQ ID NO:22617 |
| | | AA | SYGMH | VIWYDGSNEHYADSVKG | DRGVGYYGLDV |
| | | | SEQ ID NO:6594 | SEQ ID NO:14606 | SEQ ID NO:22618 |
| iPS:435847 | 21-225_191A3 | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTACTACGGTATGGACGTC |
| | | | SEQ ID NO:6595 | SEQ ID NO:14607 | SEQ ID NO:22619 |
| | | AA | SGDYYWN | YIFYSGSTYYNPSLKS | GDYDGSGSYHYYYGMDV |
| | | | SEQ ID NO:6596 | SEQ ID NO:14608 | SEQ ID NO:22620 |
| iPS:435849 | 21-225_191C3 | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTTCTACTACGGTATGGACGTC |
| | | | SEQ ID NO:6597 | SEQ ID NO:14609 | SEQ ID NO:22621 |
| | | AA | SGDYYWN | YIFYSGSTYYNPSLKS | GDYDGSGSYHFYYGMDV |
| | | | SEQ ID NO:6598 | SEQ ID NO:14610 | SEQ ID NO:22622 |
| iPS:435851 | 21-225_191D3 | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6599 | SEQ ID NO:14611 | SEQ ID NO:22623 |

FIGURE 49

| | | | | | |
|---|---|---|---|---|---|
| | | AA | SGDYYWN | YIFYSGSTYYNPSLKS | GDYDGSGSYHYYYGMDV |
| | | | SEQ ID NO:6600 | SEQ ID NO:14612 | SEQ ID NO:22624 |
| iPS:435853 | 21-225_191E3 | NA | AGCTACCACTGGAGC | CTTATCTATACCAGTAGG AGCACCAATTACAACCC CTCCCTCAAGAGT | CTCCGGTATAACTGGAACTT CCCTTACTTTGACTAC |
| | | | SEQ ID NO:6601 | SEQ ID NO:14613 | SEQ ID NO:22625 |
| | | AA | SYHWS | LIYTSRSTNYNPSLKS | LRYNWNFPYFDY |
| | | | SEQ ID NO:6602 | SEQ ID NO:14614 | SEQ ID NO:22626 |
| iPS:435855 | 21-225_191G3 | NA | AATTATGATATCAAC | CGGATGAACCCTAACAG TGGTAACACAGGCTATG CACAGAAGTTCCAGGGC | AGCAGTGGCTGGTACATCTT TGACTAC |
| | | | SEQ ID NO:6603 | SEQ ID NO:14615 | SEQ ID NO:22627 |
| | | AA | NYDIN | RMNPNSGNTGYAQKFQG | SSGWYIFDY |
| | | | SEQ ID NO:6604 | SEQ ID NO:14616 | SEQ ID NO:22628 |
| iPS:435857 | 21-225_191A4 | NA | AGCTATGGCATGCAC | GTTATATCATATGATGGA GGTTATAAAAACTATGC AGACTCCGTGAAGGGC | GGTACCCACGGGTACTACTA CGGTGTGGACGTC |
| | | | SEQ ID NO:6605 | SEQ ID NO:14617 | SEQ ID NO:22629 |
| | | AA | SYGMH | VISYDGGYKNYADSVKG | GTHGYYYGVDV |
| | | | SEQ ID NO:6606 | SEQ ID NO:14618 | SEQ ID NO:22630 |
| iPS:435859 | 21-225_190E6 | NA | AGCTATGGCATGCAC | GTTATATGGTATGATGG AAGTAATAAAAACTATG CAGACTCCGTGAAGGGC | GATCAGGGCGTGGGCTACGA CGGTTTGGACGTC |
| | | | SEQ ID NO:6607 | SEQ ID NO:14619 | SEQ ID NO:22631 |
| | | AA | SYGMH | VIWYDGSNKNYADSVKG | DQGVGYDGLDV |
| | | | SEQ ID NO:6608 | SEQ ID NO:14620 | SEQ ID NO:22632 |

FIGURE 49

| iPS:435861 | | NA | AGTTATGGCATGCAC | GTTATATGGTATGATGG AAGTAATAAAAACTATG CAGACTCCGTGAAGGGC | GATTTCTCTGTAGGGTACGA CGGTATGGACGTC |
|---|---|---|---|---|---|
| | 21-225_190A5 | | SEQ ID NO:6609 | SEQ ID NO:14621 | SEQ ID NO:22633 |
| | | AA | SYGMH | VIWYDGSNKNYADSVKG | DFSVGYDGMDV |
| | | | SEQ ID NO:6610 | SEQ ID NO:14622 | SEQ ID NO:22634 |
| iPS:435863 | | NA | AGTGGTGGTTACTACTGGAA C | TACATCTTTTACAGTGGG AGCACCTACTACAACCC GTCCCTCAGGAGT | TCCGGGTATAACTGGGACAA CGGGGTCGACCCC |
| | 21-225_191H4 | | SEQ ID NO:6611 | SEQ ID NO:14623 | SEQ ID NO:22635 |
| | | AA | SGGYYWN | YIFYSGSTYYNPSLRS | SGYNWDNGVDP |
| | | | SEQ ID NO:6612 | SEQ ID NO:14624 | SEQ ID NO:22636 |
| iPS:435865 | | NA | GACTATAGCATGAAC | TCCATTAGTAGTGGTAGT GGTTACATATATTATGCA GACTCAGTGAAGGGC | GCTACTATGGCCCTTGACTA C |
| | 21-225_191A5 | | SEQ ID NO:6613 | SEQ ID NO:14625 | SEQ ID NO:22637 |
| | | AA | DYSMN | SISSGSGYIYYADSVKG | ATMALDY |
| | | | SEQ ID NO:6614 | SEQ ID NO:14626 | SEQ ID NO:22638 |
| iPS:435867 | | NA | AGCTATGCCATGAAC | ACTATTAGTGGTACTGGT CGTAGGACATACTACGC AGACTCCGTGAAGGGC | GAGGAGGATTACTATGATAG TAGTGGCCCGGGGTTCGACC CC |
| | 21-225_191E5 | | SEQ ID NO:6615 | SEQ ID NO:14627 | SEQ ID NO:22639 |
| | | AA | SYAMN | TISGTGRRTYYADSVKG | EEDYYDSSGPGFDP |
| | | | SEQ ID NO:6616 | SEQ ID NO:14628 | SEQ ID NO:22640 |
| iPS:435869 | | NA | AGCTATGGCATGCAC | GTTATATGGTATGATGG AAGTAATAAACATTATG CAGACTCCGTGAAGGGC | GATAGAACAGTGGGATACTC CGGTATGGACGTC |
| | 21 225 190B1 | | | | |

FIGURE 49

| | | | SEQ ID NO:6617 | SEQ ID NO:14629 | SEQ ID NO:22641 |
|---|---|---|---|---|---|
| | 21-225_190B1 | AA | SYGMH | VIWYDGSNKHYADSVKG | DRTVGYSGMDV |
| | | | SEQ ID NO:6618 | SEQ ID NO:14630 | SEQ ID NO:22642 |
| iPS:435871 | | NA | AGCTACCACTGGAGC | CTTATCTATACCAGTAGG AGCACCAATTACAACCC CTCCCTCAAGAGT | CTCCGGTATAACTGGAACTT CCCTTACTTTGACTTC |
| | 21-225_191E6 | | SEQ ID NO:6619 | SEQ ID NO:14631 | SEQ ID NO:22643 |
| | | AA | SYHWS | LIYTSRSTNYNPSLKS | LRYNWNFPYFDF |
| | | | SEQ ID NO:6620 | SEQ ID NO:14632 | SEQ ID NO:22644 |
| iPS:435873 | | NA | AGCTATGGCATGCAC | GTTATATGGTATGATGG AAGTAATAAAAACTACG CAGACTCCGTGAAGGGC | GATCAGGGCGTGGGCTACGA CGGTTTGGACGTC |
| | 21-225_190G4 | | SEQ ID NO:6621 | SEQ ID NO:14633 | SEQ ID NO:22645 |
| | | AA | SYGMH | VIWYDGSNKNYADSVKG | DQGVGYDGLDV |
| | | | SEQ ID NO:6622 | SEQ ID NO:14634 | SEQ ID NO:22646 |
| iPS:435875 | | NA | ACCTATGCCATGAGT | GCTATTAGTCGTAGTGGT GGTAACACACACTACGC AGACTCCGTGAAGGGC | GATGGATTCGGTGGGAGCTC CTACTTTGACTAC |
| | 21-225_190B9 | | SEQ ID NO:6623 | SEQ ID NO:14635 | SEQ ID NO:22647 |
| | | AA | TYAMS | AISRSGGNTHYADSVKG | DGFGGSSYFDY |
| | | | SEQ ID NO:6624 | SEQ ID NO:14636 | SEQ ID NO:22648 |
| iPS:435877 | | NA | AGCTACAATATGCAC | TGGATCAACCCTAACAA TGGTGGCTCAAACTATA CACAGAAGTTTCAGGGC | AAGTTTGGGGAC |
| | 21-225_184E7 | | SEQ ID NO:6625 | SEQ ID NO:14637 | SEQ ID NO:22649 |
| | | AA | SYNMH | WINPNNGGSNYTQKFQG | KFGD |

FIGURE 49

| | | | SEQ ID NO:6626 | SEQ ID NO:14638 | SEQ ID NO:22650 |
|---|---|---|---|---|---|
| iPS:435879 | | NA | GACTATGGCATGCAC | GTTATTTGGTATGATGAAACTAATAAACACTATGGAGACTCCGTGAAGGGC | GAGGTTGGCTGGCACGATGACTAT |
| | 21-225_184H10 | | SEQ ID NO:6627 | SEQ ID NO:14639 | SEQ ID NO:22651 |
| | | AA | DYGMH | VIWYDETNKHYGDSVKG | EVGWHDDY |
| | | | SEQ ID NO:6628 | SEQ ID NO:14640 | SEQ ID NO:22652 |
| iPS:435881 | | NA | GACTATGGCATGCAC | GTTATTTGGTATGATGAAACTAATAAACACTATGGAGACTCCGTGAAGGGC | GAGGTTGGCTGGCACGATGACTAT |
| | 21-225_184D11 | | SEQ ID NO:6629 | SEQ ID NO:14641 | SEQ ID NO:22653 |
| | | AA | DYGMH | VIWYDETNKHYGDSVKG | EVGWHDDY |
| | | | SEQ ID NO:6630 | SEQ ID NO:14642 | SEQ ID NO:22654 |
| iPS:435883 | | NA | AGCTATAGCATGAAC | TCCATTAGCAGTAGTGGTAGTTACATATATTACGCAGACTCAGTGAAGGGC | AGCAACCTTTTTGACTGC |
| | 21-225_185A1 | | SEQ ID NO:6631 | SEQ ID NO:14643 | SEQ ID NO:22655 |
| | | AA | SYSMN | SISSSGSYIYYADSVKG | SNLFDC |
| | | | SEQ ID NO:6632 | SEQ ID NO:14644 | SEQ ID NO:22656 |
| iPS:435885 | | NA | AGCTACAATATGCAC | TGGATCAACCCTAACAATGGTGGCTCAAACTATACACAGAAGTTTCAGGGC | AAGTTTGGGGAC |
| | 21-225_185E10 | | SEQ ID NO:6633 | SEQ ID NO:14645 | SEQ ID NO:22657 |
| | | AA | SYNMH | WINPNNGGSNYTQKFQG | KFGD |
| | | | SEQ ID NO:6634 | SEQ ID NO:14646 | SEQ ID NO:22658 |

FIGURE 49

| iPS:435887 | | NA | ACCTATGGCATGCAC | ATTATATGGTATGATGG AAGTTATAAATATTATGC AGACTCCGTGAAGGGC | GATCATTACGATTTTTGGAG TGGGCACTTTGACTAC |
|---|---|---|---|---|---|
| | 21-225_186F7 | | SEQ ID NO:6635 | SEQ ID NO:14647 | SEQ ID NO:22659 |
| | | AA | TYGMH | IIWYDGSYKYYADSVKG | DHYDFWSGHFDY |
| | | | SEQ ID NO:6636 | SEQ ID NO:14648 | SEQ ID NO:22660 |
| iPS:435889 | | NA | AGCTATGCCATGAGT | GTTATTAGTGGTAGAGG TGGTACCACATTCTACGC AGACTCCGTGAAGGGC | CGGACTGGGGATGATGTTTT TGATATC |
| | 21-225_186A11 | | SEQ ID NO:6637 | SEQ ID NO:14649 | SEQ ID NO:22661 |
| | | AA | SYAMS | VISGRGGTTFYADSVKG | RTGDDVFDI |
| | | | SEQ ID NO:6638 | SEQ ID NO:14650 | SEQ ID NO:22662 |
| iPS:435891 | | NA | AGCTACAATATGCAC | TGGATCAACCCTAACAG TGGTGGCTCAAACTATA CACAGAAGTTTCAGGGC | AAGTTTGGGGAC |
| | 21-225_188H5 | | SEQ ID NO:6639 | SEQ ID NO:14651 | SEQ ID NO:22663 |
| | | AA | SYNMH | WINPNSGGSNYTQKFQG | KFGD |
| | | | SEQ ID NO:6640 | SEQ ID NO:14652 | SEQ ID NO:22664 |
| iPS:435895 | | NA | AGCTCTGCCATGAAC | GTTATTAGTGGTAGTGGT GGTTACACATACTACGC AGACTCCGTGAAGGGC | AGGAACACCGATGATGCTTT TGATATC |
| | 21-225_188E8 | | SEQ ID NO:6641 | SEQ ID NO:14653 | SEQ ID NO:22665 |
| | | AA | SSAMN | VISGSGGYTYYADSVKG | RNTDDAFDI |
| | | | SEQ ID NO:6642 | SEQ ID NO:14654 | SEQ ID NO:22666 |
| iPS:435897 | | NA | AGCTACAATATGCAC | TGGATCAACCCTAACAG TGGTGGCTCAAACTATA CACAGAAGTTTCAGGGC | AAGTTTGGGGAC |
| | 21-225_188B9 | | SEQ ID NO:6643 | SEQ ID NO:14655 | SEQ ID NO:22667 |
| | | AA | SYNMH | WINPNSGGSNYTQKFQG | KFGD |
| | | | SEQ ID NO:6644 | SEQ ID NO:14656 | SEQ ID NO:22668 |

FIGURE 49

| iPS:435899 | | NA | AGTTATGGCATGCAC | ATTATATGGTATGATGG AAGTTATAAATACTATG CAGACTCCGTGAAGGGC | GAGAGATACGATTTTTGGAG TGGTCATTTTGACTAC |
|---|---|---|---|---|---|
| | 21-225_188G11 | | SEQ ID NO:6645 | SEQ ID NO:14657 | SEQ ID NO:22669 |
| | | AA | SYGMH | IIWYDGSYKYYADSVKG | ERYDFWSGHFDY |
| | | | SEQ ID NO:6646 | SEQ ID NO:14658 | SEQ ID NO:22670 |
| iPS:435901 | | NA | AACTATGGCATGCAC | ATTATATGGTATGATGG AAGTTATAAATACTATG CAGACTCCGTGAAGGGC | GATCGATTCGATTTTTGGAG TGGTTATTCCGACTAC |
| | 21-225_189G2 | | SEQ ID NO:6647 | SEQ ID NO:14659 | SEQ ID NO:22671 |
| | | AA | NYGMH | IIWYDGSYKYYADSVKG | DRFDFWSGYSDY |
| | | | SEQ ID NO:6648 | SEQ ID NO:14660 | SEQ ID NO:22672 |
| iPS:435903 | | NA | GACTACTACATGAGC | TACATTAGTAGTAGTGGT ACTACCGTATTCTACGCA GACTCTGTGAAGGGC | GAATGGGTGGGAGCCGACTA C |
| | 21-225_190E2 | | SEQ ID NO:6649 | SEQ ID NO:14661 | SEQ ID NO:22673 |
| | | AA | DYYMS | YISSSGTTVFYADSVKG | EWVGADY |
| | | | SEQ ID NO:6650 | SEQ ID NO:14662 | SEQ ID NO:22674 |
| iPS:435905 | | NA | AGTGGTGGTTACTACTGGAA C | TTCATCTTTTATAGTGGG AGCACCTACTACAACCC GTCCCTCAAGAGT | GGGGATTACGATGGTTCGGG GAGTTATCACTACTATTACG GTATGGACGTC |
| | 21-225_190A3 | | SEQ ID NO:6651 | SEQ ID NO:14663 | SEQ ID NO:22675 |
| | | AA | SGGYYWN | FIFYSGSTYYNPSLKS | GDYDGSGSYHYYYGMDV |
| | | | SEQ ID NO:6652 | SEQ ID NO:14664 | SEQ ID NO:22676 |
| iPS:435907 | | NA | AGCTATGGCATGCAC | GTTATATCATATGATGGA GGTTATAAAAACTATGT AGACTCCGTGAAGGGC | GGTACCCACGGGTACTACTA CGGTGTGGACGTC |
| | 21-225_190G3 | | SEQ ID NO:6653 | SEQ ID NO:14665 | SEQ ID NO:22677 |
| | | AA | SYGMH | VISYDGGYKNYVDSVKG | GTHGYYYGVDV |
| | | | SEQ ID NO:6654 | SEQ ID NO:14666 | SEQ ID NO:22678 |

FIGURE 49

| iPS:435909 | | NA | AGCTATGCCATGAGC | GCTATTAGTGGTCGTGGT GGTAACACATACTACGC AGACTCCGTGAAGGGC | GATGGATTCGGTGGGAGCTC CTATTTTGACTAC |
|---|---|---|---|---|---|
| | 21-225_190H3 | | SEQ ID NO:6655 | SEQ ID NO:14667 | SEQ ID NO:22679 |
| | | AA | SYAMS | AISGRGGNTYYADSVKG | DGFGGSSYFDY |
| | | | SEQ ID NO:6656 | SEQ ID NO:14668 | SEQ ID NO:22680 |
| iPS:435911 | | NA | AGTGGTGATTACTACTGGAA C | TACATCTTTTACAGTGGG AGCACCTACTACAACCC GTCCCTCAAGAGT | GGGGATTACGATGGTTCGGG GAGTTATCACTACTACTACG GTATGGACGTC |
| | 21-225_190B4 | | SEQ ID NO:6657 | SEQ ID NO:14669 | SEQ ID NO:22681 |
| | | AA | SGDYYWN | YIFYSGSTYYNPSLKS | GDYDGSGSYHYYYGMDV |
| | | | SEQ ID NO:6658 | SEQ ID NO:14670 | SEQ ID NO:22682 |
| iPS:435913 | | NA | AGTGGTGTTTACTACTGGAG C | AACATCTATTACAGTGG GAGCACCTACAACAACC CGTCCCTCAAGAGT | GGGGATTACGATGGTTCGGG GAGTTATCACTACTACTACG GTTTGGACGTC |
| | 21-225_190A7 | | SEQ ID NO:6659 | SEQ ID NO:14671 | SEQ ID NO:22683 |
| | | AA | SGVYYWS | NIYYSGSTYNNPSLKS | GDYDGSGSYHYYYGLDV |
| | | | SEQ ID NO:6660 | SEQ ID NO:14672 | SEQ ID NO:22684 |
| iPS:435915 | | NA | AATTATGATATCAAC | TGGATGAACCCTAACAG TGGTAACACAGGCTATG CACAGAAGTTCCAGGGC | AGCAGTGGCTGGTACATCTT TGACTAC |
| | 21-225_190H4 | | SEQ ID NO:6661 | SEQ ID NO:14673 | SEQ ID NO:22685 |
| | | AA | NYDIN | WMNPNSGNTGYAQKFQG | SSGWYIFDY |
| | | | SEQ ID NO:6662 | SEQ ID NO:14674 | SEQ ID NO:22686 |
| iPS:435917 | | NA | AATTACTACTGGAGC | CGTATCTATGCCAGTGG GAGCACCAACTACAACC CCTCCCTCAAGAGT | GATCGGGGATACTATGGCTA CTACGGTATGGACGTC |
| | 21-225_190D5 | | SEQ ID NO:6663 | SEQ ID NO:14675 | SEQ ID NO:22687 |
| | | AA | NYYWS | RIYASGSTNYNPSLKS | DRGYYGYYGMDV |

FIGURE 49

| | | | SEQ ID NO:6664 | SEQ ID NO:14676 | SEQ ID NO:22688 |
|---|---|---|---|---|---|
| iPS:435919 | 21-225_190H5 | NA | AGCTATGGCATGCAC | GTTATATCATATGATGGAGGTTATAAAAACTATGCAGACTCCGTGAAGGGC | GGTACCCACGGGTACTACTACGGTGTGGACGTC |
| | | | SEQ ID NO:6665 | SEQ ID NO:14677 | SEQ ID NO:22689 |
| | | AA | SYGMH | VISYDGGYKNYADSVKG | GTHGYYYGVDV |
| | | | SEQ ID NO:6666 | SEQ ID NO:14678 | SEQ ID NO:22690 |
| iPS:435921 | 21-225_190D6 | NA | AGCTTTATCATGCAC | GTTATATGGTATGATGGAAGTAATAAATACTATGCAGACTCCGTGAAGGGC | GAGGAGTATAGCAGTGGCTGGTTCGGGTACGGTATGGACGTC |
| | | | SEQ ID NO:6667 | SEQ ID NO:14679 | SEQ ID NO:22691 |
| | | AA | SFIMH | VIWYDGSNKYYADSVKG | EEYSSGWFGYGMDV |
| | | | SEQ ID NO:6668 | SEQ ID NO:14680 | SEQ ID NO:22692 |
| iPS:435923 | 21-225_190H6 | NA | GACTACTACATGAGC | TACATTAGTAGTAGTGGTACTACCGTATTCTACGCAGACTCTGTGAAGGGC | GAATGGGTGGGAGCCGACTAC |
| | | | SEQ ID NO:6669 | SEQ ID NO:14681 | SEQ ID NO:22693 |
| | | AA | DYYMS | YISSSGTTVFYADSVKG | EWVGADY |
| | | | SEQ ID NO:6670 | SEQ ID NO:14682 | SEQ ID NO:22694 |
| iPS:435925 | 21-225_190D7 | NA | AATTATGATATCAAC | TGGATGAACCCTAATAGTGGTAATACAGGCTATGCACAGAAGTTCCAGGGC | AGCAGTGGCTGGTACTTCTTTGACTAC |
| | | | SEQ ID NO:6671 | SEQ ID NO:14683 | SEQ ID NO:22695 |
| | | AA | NYDIN | WMNPNSGNTGYAQKFQG | SSGWYFFDY |
| | | | SEQ ID NO:6672 | SEQ ID NO:14684 | SEQ ID NO:22696 |

FIGURE 49

| iPS:435927 | | NA | AGTTACCACTGGAGT | CATATCTATACCAGTAG GAGCACCAACTACAACC CCTCCCTCAAGAGT | CTCCGGTATAACTGGAACTT CCCTTACTTTGACTAC |
|---|---|---|---|---|---|
| | 21-225_190E7 | | SEQ ID NO:6673 | SEQ ID NO:14685 | SEQ ID NO:22697 |
| | | AA | SYHWS | HIYTSRSTNYNPSLKS | LRYNWNFPYFDY |
| | | | SEQ ID NO:6674 | SEQ ID NO:14686 | SEQ ID NO:22698 |
| iPS:435929 | | NA | AGCTATGCCATGAGT | ACTATTAGTGGTACTGGT CGTAGGACATACTACGC AGACTCCGTGAAGGGC | GAGGAGGATTACTATGATAG TAGTGGCCCGGGGTTCGACC CC |
| | 21-225_190D9 | | SEQ ID NO:6675 | SEQ ID NO:14687 | SEQ ID NO:22699 |
| | | AA | SYAMS | TISGTGRRTYYADSVKG | EEDYYDSSGPGFDP |
| | | | SEQ ID NO:6676 | SEQ ID NO:14688 | SEQ ID NO:22700 |
| iPS:435933 | | NA | ACTTATGGCATGCAC | GTTATATGGTATGATGG AAGTAATAAAAACTATG CAGACTCCGTGAAGGGC | GATCAGGGCGTGGGCTACGA CGGTTTGGACGTC |
| | 21-225_190F8 | | SEQ ID NO:6677 | SEQ ID NO:14689 | SEQ ID NO:22701 |
| | | AA | TYGMH | VIWYDGSNKNYADSVKG | DQGVGYDGLDV |
| | | | SEQ ID NO:6678 | SEQ ID NO:14690 | SEQ ID NO:22702 |
| iPS:435935 | | NA | AGCTATGCCATGAGC | ACTATTAGTGGTACTGGT CGTAGGACATATTACGC AGACTCCGTGAAGGGC | GAGGAGGATTACTATGATAG TAGTGGCCCGGGGTTCGACC CC |
| | 21-225_190H8 | | SEQ ID NO:6679 | SEQ ID NO:14691 | SEQ ID NO:22703 |
| | | AA | SYAMS | TISGTGRRTYYADSVKG | EEDYYDSSGPGFDP |
| | | | SEQ ID NO:6680 | SEQ ID NO:14692 | SEQ ID NO:22704 |
| iPS:435937 | | NA | AACTATGGCATGCAC | GTTATATGGTTTGATGGA AGTAATGACTACTATGC AGACTCCGTGAAGGGC | GATAGAAGCGTCGGCTACGA CGGTTTAGATGTC |
| | 21-225_190H9 | | SEQ ID NO:6681 | SEQ ID NO:14693 | SEQ ID NO:22705 |

FIGURE 49

| | | | | | |
|---|---|---|---|---|---|
| | | AA | NYGMH | VIWFDGSNDYYADSVKG | DRSVGYDGLDV |
| | | | SEQ ID NO:6682 | SEQ ID NO:14694 | SEQ ID NO:22706 |
| iPS:435939 | 21-225_191H7 | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6683 | SEQ ID NO:14695 | SEQ ID NO:22707 |
| | | AA | SGDYYWN | YIFYSGSTYYNPSLKS | GDYDGSGSYHYYYGMDV |
| | | | SEQ ID NO:6684 | SEQ ID NO:14696 | SEQ ID NO:22708 |
| iPS:435941 | 21-225_191E8 | NA | AACTATGGCATGCAC | ATTATATGGTTTGATGGAAGTAATCAATACTATGCCGACTCCGTGAAGGGC | GCCCACGGGGTCTACTACTACGCTATGGACGTC |
| | | | SEQ ID NO:6685 | SEQ ID NO:14697 | SEQ ID NO:22709 |
| | | AA | NYGMH | IIWFDGSNQYYADSVKG | AHGVYYYAMDV |
| | | | SEQ ID NO:6686 | SEQ ID NO:14698 | SEQ ID NO:22710 |
| iPS:435943 | 21-225_191C9 | NA | AGTGGTGGTTACTACTGGAAC | TATATCTATTACAGTGGGAGCACCTACTACAACCCGTCCCTCAAGAGT | TCCGGGTATAATTGGGACGCCGGGGTCGACCCC |
| | | | SEQ ID NO:6687 | SEQ ID NO:14699 | SEQ ID NO:22711 |
| | | AA | SGGYYWN | YIYYSGSTYYNPSLKS | SGYNWDAGVDP |
| | | | SEQ ID NO:6688 | SEQ ID NO:14700 | SEQ ID NO:22712 |
| iPS:435945 | 21-225_191A10 | NA | AGCTATGGCATGCAC | GTTATATGGTATGATGGAAGTAATAAAAACTACGCAGACTCCGTGAAGGGC | GATCAGGGCGTGGGCTACGACGGTTTGGACGTC |
| | | | SEQ ID NO:6689 | SEQ ID NO:14701 | SEQ ID NO:22713 |
| | | AA | SYGMH | VIWYDGSNKNYADSVKG | DQGVGYDGLDV |
| | | | SEQ ID NO:6690 | SEQ ID NO:14702 | SEQ ID NO:22714 |

FIGURE 49

| iPS:435947 | | NA | AGCTATGGCATGCAC | GTTATATGGTATGATGG AAGTAATAAAAACTACG CAGACTCCGTGAAGGGC | GATCAGGGCGTGGGCTACGA CGGTTTGGACGTC |
|---|---|---|---|---|---|
| | 21-225_191E10 | | SEQ ID NO:6691 | SEQ ID NO:14703 | SEQ ID NO:22715 |
| | | AA | SYGMH | VIWYDGSNKNYADSVKG | DQGVGYDGLDV |
| | | | SEQ ID NO:6692 | SEQ ID NO:14704 | SEQ ID NO:22716 |
| iPS:435953 | | NA | GACTACTACATGAGC | TACATTAGTAGTAGTGGT ACTACCGTATTCTACGCA GACTCTGTGAAGGGC | GAATGGGTGGGAGCCGACTA C |
| | 21-225_191B12 | | SEQ ID NO:6693 | SEQ ID NO:14705 | SEQ ID NO:22717 |
| | | AA | DYYMS | YISSSGTTVFYADSVKG | EWVGADY |
| | | | SEQ ID NO:6694 | SEQ ID NO:14706 | SEQ ID NO:22718 |
| iPS:435957 | | NA | AGCTATGGCATGCAC | GTTATATGGTATGATGG AAGTAATAAAAACTATG CAGACTCCGTGAAGGGC | GATCAGGGCGTGGGCTACGA CGGTTTGGACGTC |
| | 21-225_191G12 | | SEQ ID NO:6695 | SEQ ID NO:14707 | SEQ ID NO:22719 |
| | | AA | SYGMH | VIWYDGSNKNYADSVKG | DQGVGYDGLDV |
| | | | SEQ ID NO:6696 | SEQ ID NO:14708 | SEQ ID NO:22720 |
| iPS:435961 | | NA | AGCTATGGCATGCAC | GTTATATGGTATGATGG AAGTTATAAATACTATG CAGACTCCGTGAAGGGC | GAGGATTCCCCTTATAGTGG CTACGCCTTGGACTACTTCT ACGGTATGGACGTC |
| | 21-225_192A2 | | SEQ ID NO:6697 | SEQ ID NO:14709 | SEQ ID NO:22721 |
| | | AA | SYGMH | VIWYDGSYKYYADSVKG | EDSPYSGYALDYFYGMDV |
| | | | SEQ ID NO:6698 | SEQ ID NO:14710 | SEQ ID NO:22722 |

FIGURE 49

| iPS:435963 | | NA | AGCTATGGCATGCAC | GTTATATGGTATGATGG AAGTAATAAAAACTATG CAGACTCCGTGAAGGGC | GATCGTGGGGTTGGCTACTA CGGTATGGACGTC |
|---|---|---|---|---|---|
| | 21-225_192D2 | | SEQ ID NO:6699 | SEQ ID NO:14711 | SEQ ID NO:22723 |
| | | AA | SYGMH | VIWYDGSNKNYADSVKG | DRGVGYYGMDV |
| | | | SEQ ID NO:6700 | SEQ ID NO:14712 | SEQ ID NO:22724 |
| iPS:435965 | | NA | AGCTCTGCCATGAGC | GCCATTAGTGGTAGTGG TGGTAACACATTCTACGC AGACTCCGTGAAGGGC | CTCATAGCAGTAGTTGGGTC CCACTACTTTGACTAC |
| | 21-225_192H2 | | SEQ ID NO:6701 | SEQ ID NO:14713 | SEQ ID NO:22725 |
| | | AA | SSAMS | AISGSGGNTFYADSVKG | LIAVVGSHYFDY |
| | | | SEQ ID NO:6702 | SEQ ID NO:14714 | SEQ ID NO:22726 |
| iPS:435967 | | NA | AGTGGTGATTACTACTGGAA C | TTCATCTTTTACAGTGGG AGCACCTACTACAACCC GTCCCTCAAGAGT | GGGGATTACGATGGTTCGGG GAGTTATCACCACTACTACG GTATGGACGTC |
| | 21-225_192B3 | | SEQ ID NO:6703 | SEQ ID NO:14715 | SEQ ID NO:22727 |
| | | AA | SGDYYWN | FIFYSGSTYYNPSLKS | GDYDGSGSYHHYYGMDV |
| | | | SEQ ID NO:6704 | SEQ ID NO:14716 | SEQ ID NO:22728 |
| iPS:435971 | | NA | AGCTATGGCATGCAC | GTTATATGGTATGATGG AAGTAATGAACACTATG CAGACTCCGTGAAGGGC | GATAGGGGGGTGGGTTACTA CGGTTTGGACGTC |
| | 21-225_192D3 | | SEQ ID NO:6705 | SEQ ID NO:14717 | SEQ ID NO:22729 |
| | | AA | SYGMH | VIWYDGSNEHYADSVKG | DRGVGYYGLDV |
| | | | SEQ ID NO:6706 | SEQ ID NO:14718 | SEQ ID NO:22730 |

FIGURE 49

| iPS:435973 | | NA | AGTGTTAGTTACTACTGGAGC | AACCTCTATTACAGTGGGAGCACCTACTACAACCCGTCCCTCAGGAGT | GGGGATTACGATGGTTCGGGGAGTTATCACTACTACCACGGTATGGACGTC |
|---|---|---|---|---|---|
| | 21-225_192H3 | | SEQ ID NO:6707 | SEQ ID NO:14719 | SEQ ID NO:22731 |
| | | AA | SVSYYWS | NLYYSGSTYYNPSLRS | GDYDGSGSYHYYHGMDV |
| | | | SEQ ID NO:6708 | SEQ ID NO:14720 | SEQ ID NO:22732 |
| iPS:435977 | | NA | AGTTATGGCATGCAC | GTTATATGGTATGATGGAAGTAACAAAAACTATGTAGACTCCGTGAGGGGC | GATAGAAGCGTCGGCTACGACGGTATGGACGTC |
| | 21-225_192E4 | | SEQ ID NO:6709 | SEQ ID NO:14721 | SEQ ID NO:22733 |
| | | AA | SYGMH | VIWYDGSNKNYVDSVRG | DRSVGYDGMDV |
| | | | SEQ ID NO:6710 | SEQ ID NO:14722 | SEQ ID NO:22734 |
| iPS:435979 | | NA | AGCTATGGCATGCAC | GTTATATGGTATGATGGAAGCAATAAAAACTATGCAGACTCCGTGAAGGGC | GATCAAGGTGTGGGGTACTACGGTATGGACGTC |
| | 21-225_192H4 | | SEQ ID NO:6711 | SEQ ID NO:14723 | SEQ ID NO:22735 |
| | | AA | SYGMH | VIWYDGSNKNYADSVKG | DQGVGYYGMDV |
| | | | SEQ ID NO:6712 | SEQ ID NO:14724 | SEQ ID NO:22736 |
| iPS:435983 | | NA | AATGGTGGATACTACTGGAGC | TACATCTTTTACAGCGGGAGCACCTACTACAACCCGTCCCTCAAGAGT | GCGGGATATAACTGGGACAACGGGTTTGACTAC |
| | 21-225_192E5 | | SEQ ID NO:6713 | SEQ ID NO:14725 | SEQ ID NO:22737 |
| | | AA | NGGYYWS | YIFYSGSTYYNPSLKS | AGYNWDNGFDY |
| | | | SEQ ID NO:6714 | SEQ ID NO:14726 | SEQ ID NO:22738 |

FIGURE 49

| iPS:435985 | | NA | AGCTTTATCATGCAC | GTTATATGGTATGATGG AAGTAATAAATACTATG TAGACTCCGTGAAGGGC | GAGGAGTATAGTAGCGGCTG GTTCGGGTACGGTATGGACG TC |
|---|---|---|---|---|---|
| | 21-225_192F6 | | SEQ ID NO:6715 | SEQ ID NO:14727 | SEQ ID NO:22739 |
| | | AA | SFIMH | VIWYDGSNKYYVDSVKG | EEYSSGWFGYGMDV |
| | | | SEQ ID NO:6716 | SEQ ID NO:14728 | SEQ ID NO:22740 |
| iPS:435987 | | NA | AGCTATGGCATGCAC | GTTTTATGGTATGATGGA ACTAATAAAAACTATGC AGACTCCGTGAAGGGC | GATCAGGGCGTGGGCTACGA CGGTTTGGACGTC |
| | 21-225_192G6 | | SEQ ID NO:6717 | SEQ ID NO:14729 | SEQ ID NO:22741 |
| | | AA | SYGMH | VLWYDGTNKNYADSVKG | DQGVGYDGLDV |
| | | | SEQ ID NO:6718 | SEQ ID NO:14730 | SEQ ID NO:22742 |
| iPS:435989 | | NA | AGCTATGGCATGCAC | GTTATATCATATGATGGA GGTTATAAAAACTATGC AGACTCCGTGAAGGGC | GGTACCCACGGGTACTACTA CGGTGTGGACGTC |
| | 21-225_192F7 | | SEQ ID NO:6719 | SEQ ID NO:14731 | SEQ ID NO:22743 |
| | | AA | SYGMH | VISYDGGYKNYADSVKG | GTHGYYYGVDV |
| | | | SEQ ID NO:6720 | SEQ ID NO:14732 | SEQ ID NO:22744 |
| iPS:435993 | | NA | AGCTATGGCATGCAC | GTTATATGGTATGATGG AAGTAATGAACACTATG CAGACTCCGTGAAGGGC | GATAGGGGAGTGGGTTACTA CGGTATGGACGTC |
| | 21-225_192C8 | | SEQ ID NO:6721 | SEQ ID NO:14733 | SEQ ID NO:22745 |
| | | AA | SYGMH | VIWYDGSNEHYADSVKG | DRGVGYYGMDV |
| | | | SEQ ID NO:6722 | SEQ ID NO:14734 | SEQ ID NO:22746 |

FIGURE 49

| iPS:435995 | | NA | GGTTGCTACTGGAGC | GAAATCAATCAAAGTGG AAGGTCCAACTACAACC CGTCCCTCAAGAGT | GACTACGGTGTCTTTGACTA C |
|---|---|---|---|---|---|
| | 21-225_192F8 | | SEQ ID NO:6723 | SEQ ID NO:14735 | SEQ ID NO:22747 |
| | | AA | GCYWS | EINQSGRSNYNPSLKS | DYGVFDY |
| | | | SEQ ID NO:6724 | SEQ ID NO:14736 | SEQ ID NO:22748 |
| iPS:435997 | | NA | AGCTATGGCATGCAC | GTTATATGGTATGATGG AAGTAATAAAAACTATG CAGACTCCGTGAAGGGC | GATCAGGGCGTGGGCTACGA CGGTTTGGACGTC |
| | 21-225_192G8 | | SEQ ID NO:6725 | SEQ ID NO:14737 | SEQ ID NO:22749 |
| | | AA | SYGMH | VIWYDGSNKNYADSVKG | DQGVGYDGLDV |
| | | | SEQ ID NO:6726 | SEQ ID NO:14738 | SEQ ID NO:22750 |
| iPS:435999 | | NA | AGCTACCACTGGAGC | CTTATCTATACCAGTAGG AGCACCAATTACAACCC CTCCCTCAAGAGT | CTCCGGTATAACTGGAACTT CCCTTACTTTGACTAC |
| | 21-225_192F9 | | SEQ ID NO:6727 | SEQ ID NO:14739 | SEQ ID NO:22751 |
| | | AA | SYHWS | LIYTSRSTNYNPSLKS | LRYNWNFPYFDY |
| | | | SEQ ID NO:6728 | SEQ ID NO:14740 | SEQ ID NO:22752 |
| iPS:436001 | | NA | AACTATGGCATGCAC | GTTATATGGTTTGATGGA AGTAATGACTACTATGC AGACTCCGTGAAGGGC | GATAGAAGCGTCGGCTACGA CGGTTTAGATGTC |
| | 21-225_192C10 | | SEQ ID NO:6729 | SEQ ID NO:14741 | SEQ ID NO:22753 |
| | | AA | NYGMH | VIWFDGSNDYYADSVKG | DRSVGYDGLDV |
| | | | SEQ ID NO:6730 | SEQ ID NO:14742 | SEQ ID NO:22754 |
| iPS:436003 | | NA | AGCTATGCCATGAGC | GCTATTAGTGGTAGAGG CGGTAGTACATTCTACGC AGACTCCGTGAAGGGC | CGTTTAGCACTGGATGGCTA TGATGCTTTTGATATC |
| | 21-225_192G10 | | SEQ ID NO:6731 | SEQ ID NO:14743 | SEQ ID NO:22755 |

FIGURE 49

| | | | | | |
|---|---|---|---|---|---|
| | | AA | SYAMS | AISGRGGSTFYADSVKG | RLALDGYDAFDI |
| | | | SEQ ID NO:6732 | SEQ ID NO:14744 | SEQ ID NO:22756 |
| iPS:436005 | 21-225_192H10 | NA | AGCTATGGCATGCAC | GTTATATGGTATGATGG AAGTAATAAAAACTACG CAGACTCCGTGAAGGGC | GATCAGGGCGTGGGCTACGA CGGTTTGGACGTC |
| | | | SEQ ID NO:6733 | SEQ ID NO:14745 | SEQ ID NO:22757 |
| | | AA | SYGMH | VIWYDGSNKNYADSVKG | DQGVGYDGLDV |
| | | | SEQ ID NO:6734 | SEQ ID NO:14746 | SEQ ID NO:22758 |
| iPS:436007 | 21-225_192G12 | NA | AGTGGTGTTACCACTGGAG C | AACATCCATTACAGCGG GAGCACCTACAACAACC CGTCCCTCAAGAGT | GGGGATTACGATGGTTCGGG GAGTTATCACTACTACTACG GTATGGACGTC |
| | | | SEQ ID NO:6735 | SEQ ID NO:14747 | SEQ ID NO:22759 |
| | | AA | SGVYHWS | NIHYSGSTYNNPSLKS | GDYDGSGSYHYYYGMDV |
| | | | SEQ ID NO:6736 | SEQ ID NO:14748 | SEQ ID NO:22760 |
| iPS:436009 | 21-225_193A1 | NA | AGTGGTGTTACTACTGGAG C | AACATCTATTACAGTGG GAGCACCTACAACAACC CGTCCCTCAAGAGT | GGGGATTACGATGGTTCGGG GAGTTATCACTACTACTACG GTATGGACGTC |
| | | | SEQ ID NO:6737 | SEQ ID NO:14749 | SEQ ID NO:22761 |
| | | AA | SGVYYWS | NIYYSGSTYNNPSLKS | GDYDGSGSYHYYYGMDV |
| | | | SEQ ID NO:6738 | SEQ ID NO:14750 | SEQ ID NO:22762 |
| iPS:436011 | 21-225_193B1 | NA | AGTGGTGTTACTACTGGAG C | AACATCTATTACAGTGG GAGCACCTACAACAACC CGTCCCTCAAGAGT | GGGGATTACGATGGTTCGGG GAGTTATCACTACTACTACG GTATGGACGTC |
| | | | SEQ ID NO:6739 | SEQ ID NO:14751 | SEQ ID NO:22763 |
| | | AA | SGVYYWS | NIYYSGSTYNNPSLKS | GDYDGSGSYHYYYGMDV |
| | | | SEQ ID NO:6740 | SEQ ID NO:14752 | SEQ ID NO:22764 |

FIGURE 49

| iPS:436013 | | NA | ACCTTTGCCATGAGT | GCTATTAGTCGTAGTGGT GGTAACACACACTACGC AGACTCCGTGAAGGGC | GATGGATTCGGTGGGAGCTC CTACTTTGACTAC |
|---|---|---|---|---|---|
| | 21-225_193F2 | | SEQ ID NO:6741 | SEQ ID NO:14753 | SEQ ID NO:22765 |
| | | AA | TFAMS | AISRSGGNTHYADSVKG | DGFGGSSYFDY |
| | | | SEQ ID NO:6742 | SEQ ID NO:14754 | SEQ ID NO:22766 |
| iPS:436015 | | NA | AGTGGTGATTACTACTGGAA C | TACATCTTTTACAGTGGG AGCACCTACTACAACCC GTCCCTCAAGAGT | GGGGATTACGATGGTTCGGG GAGTTATCACTTCTACTACG GTATGGACGTC |
| | 21-225_193D3 | | SEQ ID NO:6743 | SEQ ID NO:14755 | SEQ ID NO:22767 |
| | | AA | SGDYYWN | YIFYSGSTYYNPSLKS | GDYDGSGSYHFYYGMDV |
| | | | SEQ ID NO:6744 | SEQ ID NO:14756 | SEQ ID NO:22768 |
| iPS:436017 | | NA | AGTGGTGATTACTACTGGAA C | TACATCTTTTACAGTGGG AGCACCTACTACAACCC GTCCCTCAAGAGT | GGGGATTACGATGGTTCGGG GAGTTATCACTACTATTACG GTATGGACGTC |
| | 21-225_193F3 | | SEQ ID NO:6745 | SEQ ID NO:14757 | SEQ ID NO:22769 |
| | | AA | SGDYYWN | YIFYSGSTYYNPSLKS | GDYDGSGSYHYYYGMDV |
| | | | SEQ ID NO:6746 | SEQ ID NO:14758 | SEQ ID NO:22770 |
| iPS:436019 | | NA | AGCTATGCCATGAAC | GCTATTATTGGTAATGGT GGTAGAACATACTACGC AGACTCCGTGAAGGGC | GATCTGGGTAGATACAGCTA TGGTTTCTTTGACTAC |
| | 21-225_193C4 | | SEQ ID NO:6747 | SEQ ID NO:14759 | SEQ ID NO:22771 |
| | | AA | SYAMN | AIIGNGGRTYYADSVKG | DLGRYSYGFFDY |
| | | | SEQ ID NO:6748 | SEQ ID NO:14760 | SEQ ID NO:22772 |
| iPS:436021 | | NA | AATTATGATATCAAC | TGGATGAACCCTAACAG TGGTAACACAGGCTATG CACAGAAGTTCCAGGGC | AGCAGTGGCTGGTACTTCTT TGACTAC |
| | 21-225_193G4 | | SEQ ID NO:6749 | SEQ ID NO:14761 | SEQ ID NO:22773 |
| | | AA | NYDIN | WMNPNSGNTGYAQKFQG | SSGWYFFDY |

FIGURE 49

| | | | SEQ ID NO:6750 | SEQ ID NO:14762 | SEQ ID NO:22774 |
|---|---|---|---|---|---|
| iPS:436023 | | NA | AGTTATGATATCAAC | TGGATGAACCCTAAAAG GGGTAACACAGGCTATG CACAGAAGTTCCAGGGC | GGAGACCCGTATAACTGGAA CTCCTACGCTATGGACGTC |
| | 21-225_193A5 | | SEQ ID NO:6751 | SEQ ID NO:14763 | SEQ ID NO:22775 |
| | | AA | SYDIN | WMNPKRGNTGYAQKFQG | GDPYNWNSYAMDV |
| | | | SEQ ID NO:6752 | SEQ ID NO:14764 | SEQ ID NO:22776 |
| iPS:436025 | | NA | AGTGGTGGTTACTACTGGAG C | TACATCTATTACAGTGGG AGCACCTACTACAACCC GTCCCTCAAGAGT | GGAGAGTATAACTGGAACCA CGGTATGGACGTC |
| | 21-225_193B5 | | SEQ ID NO:6753 | SEQ ID NO:14765 | SEQ ID NO:22777 |
| | | AA | SGGYYWS | YIYYSGSTYYNPSLKS | GEYNWNHGMDV |
| | | | SEQ ID NO:6754 | SEQ ID NO:14766 | SEQ ID NO:22778 |
| iPS:436027 | | NA | GGTCCCTACTGGAGT | GAATCCAATCATAGTGG ACGCACCAACTACAACC CGTCCCTCAAGAGT | GACTACGGTGGTTTGGACTA C |
| | 21-225_193E6 | | SEQ ID NO:6755 | SEQ ID NO:14767 | SEQ ID NO:22779 |
| | | AA | GPYWS | ESNHSGRTNYNPSLKS | DYGGLDY |
| | | | SEQ ID NO:6756 | SEQ ID NO:14768 | SEQ ID NO:22780 |
| iPS:436029 | | NA | AGTGGTGATTACTACTGGAA C | TACATCTTTTACAGTGGG AGCACCTACTACAACCC GTCCCTCAAGAGT | GGGGATTACGATGGTTCGGG GAGTTATCACTACTATTACG GTATGGACGTC |
| | 21-225_193H6 | | SEQ ID NO:6757 | SEQ ID NO:14769 | SEQ ID NO:22781 |
| | | AA | SGDYYWN | YIFYSGSTYYNPSLKS | GDYDGSGSYHYYYGMDV |
| | | | SEQ ID NO:6758 | SEQ ID NO:14770 | SEQ ID NO:22782 |

FIGURE 49

| iPS:436031 | | NA | AGCTATGGCATGCAC | GTTATATGGTATGATGG AAGTAATAAAAACTACG CAGACTCCGTGAAGGGC | GATCAGGGCGTGGGCTACGA CGGCTTGGACGTC |
|---|---|---|---|---|---|
| | 21-225_193C7 | | SEQ ID NO:6759 | SEQ ID NO:14771 | SEQ ID NO:22783 |
| | | AA | SYGMH | VIWYDGSNKNYADSVKG | DQGVGYDGLDV |
| | | | SEQ ID NO:6760 | SEQ ID NO:14772 | SEQ ID NO:22784 |
| iPS:436033 | | NA | GGTTACTTCTGGACC | GAAATCAATCATAGTGG AAGCACCAACTACAACC CGTCCCTCAAGAGT | GACTACGGTGCTGACTAC |
| | 21-225_193E7 | | SEQ ID NO:6761 | SEQ ID NO:14773 | SEQ ID NO:22785 |
| | | AA | GYFWT | EINHSGSTNYNPSLKS | DYGADY |
| | | | SEQ ID NO:6762 | SEQ ID NO:14774 | SEQ ID NO:22786 |
| iPS:436035 | | NA | AGTGGTGATTACTACTGGAAC | TACATCTTTTACAGTGGG AGCACCTACTACAACCC GTCCCTCAAGAGT | GGGGATTACGATGGTTCGGG GAGTTATCACTACTACTACG GTATGGACGTC |
| | 21-225_193C8 | | SEQ ID NO:6763 | SEQ ID NO:14775 | SEQ ID NO:22787 |
| | | AA | SGDYYWN | YIFYSGSTYYNPSLKS | GDYDGSGSYHYYYGMDV |
| | | | SEQ ID NO:6764 | SEQ ID NO:14776 | SEQ ID NO:22788 |
| iPS:436037 | | NA | AGTGGTGGTTACTACTGGAAC | TTCATCTTTTACAGTGGG AGCACCTACTACAACCC GTCCCTCAAGAGT | GGGGATTACGATGGTTCGGG GAGTTATCACTACTATTACG GTATGGACGTC |
| | 21-225_193D8 | | SEQ ID NO:6765 | SEQ ID NO:14777 | SEQ ID NO:22789 |
| | | AA | SGGYYWN | FIFYSGSTYYNPSLKS | GDYDGSGSYHYYYGMDV |
| | | | SEQ ID NO:6766 | SEQ ID NO:14778 | SEQ ID NO:22790 |
| iPS:436039 | | NA | ATCTATGGCATGGAC | GTTATATGGTATGATGG AAGTTATAAATACTATG CAGACTCCGTGAAGGGC | GAGGATTCCCCTTATAGTGG CTACGGCTTGGACTACTACT ACGGTATGGACGTC |
| | 21-225_193F8 | | SEQ ID NO:6767 | SEQ ID NO:14779 | SEQ ID NO:22791 |

FIGURE 49

| | | | | | |
|---|---|---|---|---|---|
| | | AA | IYGMD | VIWYDGSYKYYADSVKG | EDSPYSGYGLDYYYGMDV |
| | | | SEQ ID NO:6768 | SEQ ID NO:14780 | SEQ ID NO:22792 |
| iPS:436041 | 21-225_193G8 | NA | AGTGGTGTTTACTACTGGAGC | AACATCTATTACAGTGGGAGCACCTACAACAACCCGTCCCTCAAGAGT | GGGGATTACGATGGTTCGGGGAGTTATCACTTCTACTACGGTTTGGACGTC |
| | | | SEQ ID NO:6769 | SEQ ID NO:14781 | SEQ ID NO:22793 |
| | | AA | SGVYYWS | NIYYSGSTYNNPSLKS | GDYDGSGSYHFYYGLDV |
| | | | SEQ ID NO:6770 | SEQ ID NO:14782 | SEQ ID NO:22794 |
| iPS:436043 | 21-225_193G9 | NA | AATGGTGGATACTACTGGAGC | TACATCTTTTACAGCGGGAGCACCTACTACAACCCGTCCCTCAAGAGT | GCGGGATATAACTGGGACAACGGGTTTGACTAC |
| | | | SEQ ID NO:6771 | SEQ ID NO:14783 | SEQ ID NO:22795 |
| | | AA | NGGYYWS | YIFYSGSTYYNPSLKS | AGYNWDNGFDY |
| | | | SEQ ID NO:6772 | SEQ ID NO:14784 | SEQ ID NO:22796 |
| iPS:436045 | 21-225_193A10 | NA | AGCTATGGCATGCAC | GTTATATGGTATGATGGAAGTAATGAACACTATGCAGACTCCGTGAAGGGC | GATAGGGGGGTGGGTTACTACGGTTTGGACGTC |
| | | | SEQ ID NO:6773 | SEQ ID NO:14785 | SEQ ID NO:22797 |
| | | AA | SYGMH | VIWYDGSNEHYADSVKG | DRGVGYYGLDV |
| | | | SEQ ID NO:6774 | SEQ ID NO:14786 | SEQ ID NO:22798 |
| iPS:436047 | 21-225_193B10 | NA | GACTATAGCATGAAC | TCCATTAGTAGTGCTGGTGGTTACATATACTACGCAGACTCACTGAAGGGC | GCAACTATGGCCCTTGACTAC |
| | | | SEQ ID NO:6775 | SEQ ID NO:14787 | SEQ ID NO:22799 |
| | | AA | DYSMN | SISSAGGYIYYADSLKG | ATMALDY |
| | | | SEQ ID NO:6776 | SEQ ID NO:14788 | SEQ ID NO:22800 |

FIGURE 49

| iPS:436049 | | NA | AGTGCTGATTACTACTGGAAC | TACATCTTTTACAGTGGGAGCACCTACTACAACCCGTCCCTCAAGAGT | GGGGATTACGATGGTTCGGGGAGTTATCACTTCTACTACGGTATGGACGTC |
|---|---|---|---|---|---|
| | 21-225_193B12 | | SEQ ID NO:6777 | SEQ ID NO:14789 | SEQ ID NO:22801 |
| | | AA | SADYYWN | YIFYSGSTYYNPSLKS | GDYDGSGSYHFYYGMDV |
| | | | SEQ ID NO:6778 | SEQ ID NO:14790 | SEQ ID NO:22802 |
| iPS:436051 | | NA | AGCTATGCCATGCAC | GTTATATGGTATGATGGAACTAATAAATACTATGGAGACTCCGTGAAGGGC | GATTTCACTATAACTGGAGCTACATATTTTGACTAC |
| | 21-225_193G12 | | SEQ ID NO:6779 | SEQ ID NO:14791 | SEQ ID NO:22803 |
| | | AA | SYAMH | VIWYDGTNKYYGDSVKG | DFTITGATYFDY |
| | | | SEQ ID NO:6780 | SEQ ID NO:14792 | SEQ ID NO:22804 |
| iPS:436054 | | NA | AGCTATGGCATGCAC | GTTATATGGTATGATGGAAGTAATGAACACTATGCAGACTCCGTGAAGGGC | AATAGGGGGGGTGGGTTACTACGGTTTGGACGTC |
| | 21-225_194C1 | | SEQ ID NO:6781 | SEQ ID NO:14793 | SEQ ID NO:22805 |
| | | AA | SYGMH | VIWYDGSNEHYADSVKG | NRGVGYYGLDV |
| | | | SEQ ID NO:6782 | SEQ ID NO:14794 | SEQ ID NO:22806 |
| iPS:436056 | | NA | AATTACTACTGGAGC | CGTATCTATGCCAGTGGGAGCACCAACTACAACCCCTCCCTCAAGAGT | GATCGGGGATACTATGGCTACTACGGTATGGACGTC |
| | 21-225_194C3 | | SEQ ID NO:6783 | SEQ ID NO:14795 | SEQ ID NO:22807 |
| | | AA | NYYWS | RIYASGSTNYNPSLKS | DRGYYGYYGMDV |
| | | | SEQ ID NO:6784 | SEQ ID NO:14796 | SEQ ID NO:22808 |

FIGURE 49

| iPS:436058 | | NA | GTCTACTATTTGAAC | TGGATCAACCCTAACAG TGGTGGCACAAACTATG CACAGAAGTTTCAGGGC | GGCTACGATATTTTGACTGG T |
|---|---|---|---|---|---|
| | 21-225_194A4 | | SEQ ID NO:6785 | SEQ ID NO:14797 | SEQ ID NO:22809 |
| | | AA | VYYLN | WINPNSGGTNYAQKFQG | GYDILTG |
| | | | SEQ ID NO:6786 | SEQ ID NO:14798 | SEQ ID NO:22810 |
| iPS:436060 | | NA | AGTTACCACTGGAGC | CTTATCTATACCAGTAGG AGCACCAACTACAACCC CTCCCTCAAGAGT | CTCCGGTATAACTGGAACTT CCCTTACTTTGACTAC |
| | 21-225_194F4 | | SEQ ID NO:6787 | SEQ ID NO:14799 | SEQ ID NO:22811 |
| | | AA | SYHWS | LIYTSRSTNYNPSLKS | LRYNWNFPYFDY |
| | | | SEQ ID NO:6788 | SEQ ID NO:14800 | SEQ ID NO:22812 |
| iPS:436062 | | NA | AGTGGTGATTACTACTGGAA C | TACATCTTTTACAGTGGG AGCACCTACTACAACCC GTCCCTCAAGAGT | GGGGATTACGATGGTTCGGG GAGTTATCACTACTATTACG GTATGGACGTC |
| | 21-225_194E5 | | SEQ ID NO:6789 | SEQ ID NO:14801 | SEQ ID NO:22813 |
| | | AA | SGDYYWN | YIFYSGSTYYNPSLKS | GDYDGSGSYHYYYGMDV |
| | | | SEQ ID NO:6790 | SEQ ID NO:14802 | SEQ ID NO:22814 |
| iPS:436064 | | NA | AGTGGTGATTACTACTGGAA C | TTCATCTTTTACAGTGGG AGCACCTACTACAACCC GTCCCTCAAGAGT | GGGGATTACGATGGTTCGGG GAGTTATCACTACTATTACG GTATGGACGTC |
| | 21-225_194E6 | | SEQ ID NO:6791 | SEQ ID NO:14803 | SEQ ID NO:22815 |
| | | AA | SGDYYWN | FIFYSGSTYYNPSLKS | GDYDGSGSYHYYYGMDV |
| | | | SEQ ID NO:6792 | SEQ ID NO:14804 | SEQ ID NO:22816 |
| iPS:436066 | | NA | AGCTATGGCATGCAC | GTTATATGGTATGATGG AAGTAATAAAAACTATG CAGACTCCGTGAAGGGC | GATCGGTCTAAGGGTTACGA CGGTATGGACGTC |
| | 21-225_194B7 | | SEQ ID NO:6793 | SEQ ID NO:14805 | SEQ ID NO:22817 |

FIGURE 49

| | | AA | SYGMH | VIWYDGSNKNYADSVKG | DRSKGYDGMDV |
|---|---|---|---|---|---|
| | | | SEQ ID NO:6794 | SEQ ID NO:14806 | SEQ ID NO:22818 |
| iPS:436068 | 21-225_194F7 | NA | GACTACTACATACAC | TGGATCAACCCTAACAATGGTGGCACAAACTATGCACAGAAATTTCAGGGC | GAACCCCTTGGTTACTATGGTTCGGGGAGTTATGGGGCCTACGGTATGGACGTC |
| | | | SEQ ID NO:6795 | SEQ ID NO:14807 | SEQ ID NO:22819 |
| | | AA | DYYIH | WINPNNGGTNYAQKFQG | EPLGYYGSGSYGAYGMDV |
| | | | SEQ ID NO:6796 | SEQ ID NO:14808 | SEQ ID NO:22820 |
| iPS:436072 | 21-225_194C10 | NA | TATTACTACTGGAGC | GAAATCAATCATAGTGGAAGCACCAACTACAACCCGTCCCTCAAGAGT | GACTACGGTGCTTTTGATATC |
| | | | SEQ ID NO:6797 | SEQ ID NO:14809 | SEQ ID NO:22821 |
| | | AA | YYYWS | EINHSGSTNYNPSLKS | DYGAFDI |
| | | | SEQ ID NO:6798 | SEQ ID NO:14810 | SEQ ID NO:22822 |
| iPS:436074 | 21-225_194F10 | NA | AGCTTTATCATGCAC | GTTATATGGTATGATGGAAGTAATAAATACTATGCAGACTCCGTGAAGGGC | GAGGAGTATAGCAGTGGCTGGTTCGGGTACGGTATGGACGTC |
| | | | SEQ ID NO:6799 | SEQ ID NO:14811 | SEQ ID NO:22823 |
| | | AA | SFIMH | VIWYDGSNKYYADSVKG | EEYSSGWFGYGMDV |
| | | | SEQ ID NO:6800 | SEQ ID NO:14812 | SEQ ID NO:22824 |
| iPS:436076 | 21-225_194H11 | NA | AGCTATGGCATGCAC | GTTATATGGTATGATGGAAGTAATGAACACTATGCAGACTCCGTGAAGGGC | GATAGGGGGGTGGGTTATTACGGTTTGGACGTC |
| | | | SEQ ID NO:6801 | SEQ ID NO:14813 | SEQ ID NO:22825 |

FIGURE 49

| | | AA | SYGMH | VIWYDGSNEHYADSVKG | DRGVGYYGLDV |
|---|---|---|---|---|---|
| | | | SEQ ID NO:6802 | SEQ ID NO:14814 | SEQ ID NO:22826 |
| iPS:436078 | 21-225_194H12 | NA | AACTATGGCATGCAC | GTTATATGGTTTGATGGA AGTAATGACTACTATGC AGACTCCGTGAAGGGC | GATAGAAGCGTCGGCTACGA CGGTTTAGATGTC |
| | | | SEQ ID NO:6803 | SEQ ID NO:14815 | SEQ ID NO:22827 |
| | | AA | NYGMH | VIWFDGSNDYYADSVKG | DRSVGYDGLDV |
| | | | SEQ ID NO:6804 | SEQ ID NO:14816 | SEQ ID NO:22828 |
| iPS:436080 | 21-225_195B1 | NA | TATTACTTCTGGAGC | GAAATCAATCATAGTGG ACGCACCAACTACAACC CGTCCCTCAAGAGT | GACTACGGTGCTTTTGATAT C |
| | | | SEQ ID NO:6805 | SEQ ID NO:14817 | SEQ ID NO:22829 |
| | | AA | YYFWS | EINHSGRTNYNPSLKS | DYGAFDI |
| | | | SEQ ID NO:6806 | SEQ ID NO:14818 | SEQ ID NO:22830 |
| iPS:436082 | 21-225_195D9 | NA | CACTATGTCATGCAC | GTTATTTGGTATGATGGA ACTAATAAATACTATGC AGACTCCGTGAAGGGC | GATTGGTTCGGGGGAGGGGAA CTACTACGGTATGGACGTC |
| | | | SEQ ID NO:6807 | SEQ ID NO:14819 | SEQ ID NO:22831 |
| | | AA | HYVMH | VIWYDGTNKYYADSVKG | DWFGEGNYYGMDV |
| | | | SEQ ID NO:6808 | SEQ ID NO:14820 | SEQ ID NO:22832 |
| iPS:436084 | 21-225_195F2 | NA | AGCGGTGGTTACTACTGGAG C | TACAGCTATTACAGTGG GAGCACCAACTATAACC CGTCCCTCAAGAGT | GGGGGGGTATAACTGGAACAA CGGGTTTGACTAC |
| | | | SEQ ID NO:6809 | SEQ ID NO:14821 | SEQ ID NO:22833 |
| | | AA | SGGYYWS | YSYYSGSTNYNPSLKS | GGYNWNNGFDY |
| | | | SEQ ID NO:6810 | SEQ ID NO:14822 | SEQ ID NO:22834 |

FIGURE 49

| iPS:436086 | | NA | AGCTATGGCATGCAC | GTTATATGGTATGATGG AAGTAATAAAAACTATG CAGACTCCGTGAAGGGC | GATCAGGGCGTGGGCTACGA CGGTTTGGACGTC |
|---|---|---|---|---|---|
| | 21-225_191G10 | | SEQ ID NO:6811 | SEQ ID NO:14823 | SEQ ID NO:22835 |
| | | AA | SYGMH | VIWYDGSNKNYADSVKG | DQGVGYDGLDV |
| | | | SEQ ID NO:6812 | SEQ ID NO:14824 | SEQ ID NO:22836 |
| iPS:436088 | | NA | AGTGGTGATTACTACTGGAAC | TACATCTTTTACAGTGGG AGCACCTACTACAACCC GTCCCTCAAGAGT | GGGGATTACGATGGTTCGGG GAGTTATCACTTCTACTACG GTATGGACGTC |
| | 21-225_195C8 | | SEQ ID NO:6813 | SEQ ID NO:14825 | SEQ ID NO:22837 |
| | | AA | SGDYYWN | YIFYSGSTYYNPSLKS | GDYDGSGSYHFYYGMDV |
| | | | SEQ ID NO:6814 | SEQ ID NO:14826 | SEQ ID NO:22838 |
| iPS:436090 | | NA | AGCTATGGCATGCAC | GTTATATGGTATGATGG AAGTAATGAACACTATG CAGACTCCGTGAAGGGC | GATAGGGGGGTGGGTTACTA CGGTTTGGACGTC |
| | 21-225_195A9 | | SEQ ID NO:6815 | SEQ ID NO:14827 | SEQ ID NO:22839 |
| | | AA | SYGMH | VIWYDGSNEHYADSVKG | DRGVGYYGLDV |
| | | | SEQ ID NO:6816 | SEQ ID NO:14828 | SEQ ID NO:22840 |
| iPS:436092 | | NA | AGCTATGGCATGCAC | GTTATATGGTATGATGG AAGTAATAAATACTATG CAGACTCCGTGAAGGGC | GAATGGCTACAATTCAGGTA CTACTACGGTATGGACGTC |
| | 21-225_195B9 | | SEQ ID NO:6817 | SEQ ID NO:14829 | SEQ ID NO:22841 |
| | | AA | SYGMH | VIWYDGSNKYYADSVKG | EWLQFRYYYGMDV |
| | | | SEQ ID NO:6818 | SEQ ID NO:14830 | SEQ ID NO:22842 |

FIGURE 49

| iPS:436094 | | NA | AATAGTGGTTACTACTGGAGC | TACATGTATTACAGTGGGAGCACCTACTACAACCCGTCCCTCAAGAGT | GGGGGGGTATAACTGGAACAATGGGTTTGACTGT |
|---|---|---|---|---|---|
| | 21-225_195B10 | | SEQ ID NO:6819 | SEQ ID NO:14831 | SEQ ID NO:22843 |
| | | AA | NSGYYWS | YMYYSGSTYYNPSLKS | GGYNWNNGFDC |
| | | | SEQ ID NO:6820 | SEQ ID NO:14832 | SEQ ID NO:22844 |
| iPS:436096 | | NA | AGTGGTGGTTACTACTGGAGC | TACATCTATTACAGTGGGAGCACCTACTACAACCCGTCCCTCAAGAGT | GGGGGGGTATAACTGGAACCACGGTATGGACGTC |
| | 21-225_195E10 | | SEQ ID NO:6821 | SEQ ID NO:14833 | SEQ ID NO:22845 |
| | | AA | SGGYYWS | YIYYSGSTYYNPSLKS | GGYNWNHGMDV |
| | | | SEQ ID NO:6822 | SEQ ID NO:14834 | SEQ ID NO:22846 |
| iPS:436098 | | NA | GACTACTACATGAGC | TACATTAGTAGTAGTGGTACTACCGTATTCTACGCAGACTCTGTGAAGGGC | GAATGGGTGGGAGCCGACTAC |
| | 21-225_195G11 | | SEQ ID NO:6823 | SEQ ID NO:14835 | SEQ ID NO:22847 |
| | | AA | DYYMS | YISSSGTTVFYADSVKG | EWVGADY |
| | | | SEQ ID NO:6824 | SEQ ID NO:14836 | SEQ ID NO:22848 |
| iPS:436100 | | NA | ACCTATGCCATGAGT | GCTATTAGTCGTAGTGGTGGTAACACACACTACGCAGACTCCGTGAAGGGC | GATGGATTCGGTGGGAGCTCCTACTTTGACTAC |
| | 21-225_195G12 | | SEQ ID NO:6825 | SEQ ID NO:14837 | SEQ ID NO:22849 |
| | | AA | TYAMS | AISRSGGNTHYADSVKG | DGFGGSSYFDY |
| | | | SEQ ID NO:6826 | SEQ ID NO:14838 | SEQ ID NO:22850 |
| iPS:436102 | | NA | GACTACTACATGAGC | TACATTAGTAGTAGTGGTATTACCATGTACTACGCAGACTCTGTGAAGGGC | GAATGGGTGGGAGCCGACTAC |
| | 21-225_196B1 | | SEQ ID NO:6827 | SEQ ID NO:14839 | SEQ ID NO:22851 |
| | | AA | DYYMS | YISSSGITMYYADSVKG | EWVGADY |
| | | | SEQ ID NO:6828 | SEQ ID NO:14840 | SEQ ID NO:22852 |

FIGURE 49

| iPS:436104 | | NA | GACTACTACATGAGC | TACATTAGTAGTAGTGGT ACTACCGTATTCTACGCA GACTCTGTGAAGGGC | GAATGGGTGGGAGCCGACTA C |
|---|---|---|---|---|---|
| | 21-225_196C1 | | SEQ ID NO:6829 | SEQ ID NO:14841 | SEQ ID NO:22853 |
| | | AA | DYYMS | YISSSGTTVFYADSVKG | EWVGADY |
| | | | SEQ ID NO:6830 | SEQ ID NO:14842 | SEQ ID NO:22854 |
| iPS:436106 | | NA | AGCTTTGGCATGCAC | GTTATATTAAATGATGG AAGTAATAAAAAGTGTG CAGACTCCGTGAAGGGC | GGACAGCAGTGGCTGGTAAA CGGTGTGGACGTC |
| | 21-225_196F2 | | SEQ ID NO:6831 | SEQ ID NO:14843 | SEQ ID NO:22855 |
| | | AA | SFGMH | VILNDGSNKKCADSVKG | GQQWLVNGVDV |
| | | | SEQ ID NO:6832 | SEQ ID NO:14844 | SEQ ID NO:22856 |
| iPS:436110 | | NA | AGCTGTGCCATGACC | GCTATTAGTGGTAGTGGT GGTAGCACATACTACGC AGACTCCGTGAAGGGC | GTGGGGGGGTTTGACTGGCTC CTACTACTACTACGGTATGG ACGTC |
| | 21-225_196F4 | | SEQ ID NO:6833 | SEQ ID NO:14845 | SEQ ID NO:22857 |
| | | AA | SCAMT | AISGSGGSTYYADSVKG | VGGLTGSYYYYGMDV |
| | | | SEQ ID NO:6834 | SEQ ID NO:14846 | SEQ ID NO:22858 |
| iPS:436112 | | NA | AGCTATGGCATGCAC | GTTATATGGTATGATGG AAGTAATGAACACTATG CAGACTCCGTGAAGGGC | GATAGGGGGGTGGGTTACTA CGGTTTGGACGTC |
| | 21-225_196C7 | | SEQ ID NO:6835 | SEQ ID NO:14847 | SEQ ID NO:22859 |
| | | AA | SYGMH | VIWYDGSNEHYADSVKG | DRGVGYYGLDV |
| | | | SEQ ID NO:6836 | SEQ ID NO:14848 | SEQ ID NO:22860 |
| iPS:436114 | | NA | AATTATGATATCAAC | TGGATGCACCTTAACAG TGGTAACACAGGCTATG CACCGAAGTTCCAGGGC | AGCGGTGGCTGGTACGTGTT CGACCCC |

FIGURE 49

| | 21-225_196G8 | | SEQ ID NO:6837 | SEQ ID NO:14849 | SEQ ID NO:22861 |
|---|---|---|---|---|---|
| | | AA | NYDIN | WMHLNSGNTGYAPKFQG | SGGWYVFDP |
| | | | SEQ ID NO:6838 | SEQ ID NO:14850 | SEQ ID NO:22862 |
| iPS:436116 | 21-225_196B9 | NA | GGCTACTATATGCAC | TGGATCAACCCTAACAGTGGTGGCACAAACTTTGCACAGAAGTTTCGGGGC | GGGGGGGGTTCGGGGAGTTCCCAACTACTACTACGTTATGGACGTC |
| | | | SEQ ID NO:6839 | SEQ ID NO:14851 | SEQ ID NO:22863 |
| | | AA | GYYMH | WINPNSGGTNFAQKFRG | GGVRGVPNYYYVMDV |
| | | | SEQ ID NO:6840 | SEQ ID NO:14852 | SEQ ID NO:22864 |
| iPS:436118 | 21-225_196A10 | NA | CACTATGTCATGCAC | GTTATTTGGTATGATGGAACTAATAAATACTATGCAGACTCCGTGAAGGGC | GATTGGTTCGGGGAGGGGAACTACTACGGTATGGACGTC |
| | | | SEQ ID NO:6841 | SEQ ID NO:14853 | SEQ ID NO:22865 |
| | | AA | HYVMH | VIWYDGTNKYYADSVKG | DWFGEGNYYGMDV |
| | | | SEQ ID NO:6842 | SEQ ID NO:14854 | SEQ ID NO:22866 |
| iPS:436120 | 21-225_196C10 | NA | AGTGGTGGTGACTACTGGAGC | TTCATCTATTACAGTGGGAGCACCTACTACAATCCGTCCCTCAAGAGT | ATGGACTACAGTAACTACTACTACGGTATGGACGTC |
| | | | SEQ ID NO:6843 | SEQ ID NO:14855 | SEQ ID NO:22867 |
| | | AA | SGGDYWS | FIYYSGSTYYNPSLKS | MDYSNYYYGMDV |
| | | | SEQ ID NO:6844 | SEQ ID NO:14856 | SEQ ID NO:22868 |
| iPS:436122 | 21-225_196G10 | NA | GACTATAGCATGAAC | TCTATTAGTAGTGGTAGTGGTTACATACACTACGCAGACTCAGTGAAGGGC | GCAACTATGGCCCTTGACTAC |
| | | | SEQ ID NO:6845 | SEQ ID NO:14857 | SEQ ID NO:22869 |
| | | AA | DYSMN | SISSGSGYIHYADSVKG | ATMALDY |
| | | | SEQ ID NO:6846 | SEQ ID NO:14858 | SEQ ID NO:22870 |

FIGURE 49

| iPS:436132 | | NA | AGTTATGATATCAAC | TGGATGAACCCTAAAAG GGGTAACACAGGCTATG CACAGAAGTTCCAGGGC | GGAGACCCGTATAACTGGAA CTCCTACGCTATGGACGTC |
|---|---|---|---|---|---|
| | 21-225_196C12 | | SEQ ID NO:6847 | SEQ ID NO:14859 | SEQ ID NO:22871 |
| | | AA | SYDIN | WMNPKRGNTGYAQKFQG | GDPYNWNSYAMDV |
| | | | SEQ ID NO:6848 | SEQ ID NO:14860 | SEQ ID NO:22872 |
| iPS:436134 | | NA | AGTGGTGTTTACTACTGGAG C | AACATCTATTACAGTGG GAGCACCTACAACAACC CGTCCCTCAAGAGT | GGGGATTACGATGGTTCGGG GAGTTATCACTACTACTACG GTTTGGACGTC |
| | 21-225_196H12 | | SEQ ID NO:6849 | SEQ ID NO:14861 | SEQ ID NO:22873 |
| | | AA | SGVYYWS | NIYYSGSTYNNPSLKS | GDYDGSGSYHYYYGLDV |
| | | | SEQ ID NO:6850 | SEQ ID NO:14862 | SEQ ID NO:22874 |
| iPS:436138 | | NA | AGCTATGGCATGCAC | GTTATATGGTATGATGG AAGTAATAAAAACTATG CAGACTCCGTGAAGGGC | GATCAGGGCGTGGGCTACGA CGGTTTGGACGTC |
| | 21-225_197F2 | | SEQ ID NO:6851 | SEQ ID NO:14863 | SEQ ID NO:22875 |
| | | AA | SYGMH | VIWYDGSNKNYADSVKG | DQGVGYDGLDV |
| | | | SEQ ID NO:6852 | SEQ ID NO:14864 | SEQ ID NO:22876 |
| iPS:436140 | | NA | AGCCATGGCATGCAC | GTTATATGGTATGATGG AAGTAATAAAAACTATG CAGACTCCGTGAAGGGC | GATCCCTCTGTAGGGTACGA CGGTATGGACGTC |
| | 21-225_197G3 | | SEQ ID NO:6853 | SEQ ID NO:14865 | SEQ ID NO:22877 |
| | | AA | SHGMH | VIWYDGSNKNYADSVKG | DPSVGYDGMDV |
| | | | SEQ ID NO:6854 | SEQ ID NO:14866 | SEQ ID NO:22878 |

FIGURE 49

| iPS:436146 | | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTACTACGGTTTGGACGTC |
|---|---|---|---|---|---|
| | 21-225_197F4 | | SEQ ID NO:6855 | SEQ ID NO:14867 | SEQ ID NO:22879 |
| | | AA | SGDYYWN | YIFYSGSTYYNPSLKS | GDYDGSGSYHYYYGLDV |
| | | | SEQ ID NO:6856 | SEQ ID NO:14868 | SEQ ID NO:22880 |
| iPS:436150 | | NA | AATTATGATATCAAC | TGGATGCACCCTAACAGTGGTAACACAGGCTATGCACAGAAGTTCCAGGGC | AGCAGTGGCTGGTACTACTTTGACTAC |
| | 21-225_197H4 | | SEQ ID NO:6857 | SEQ ID NO:14869 | SEQ ID NO:22881 |
| | | AA | NYDIN | WMHPNSGNTGYAQKFQG | SSGWYYFDY |
| | | | SEQ ID NO:6858 | SEQ ID NO:14870 | SEQ ID NO:22882 |
| iPS:436152 | | NA | AGCTATGGCATGCAC | GTTATATGGTATGATGGAAGTAATAAAAACTACGCAGACTCCGTGAAGGGC | GATCAGGGCGTGGGCTACGACGGTTTGGACGTC |
| | 21-225_197B6 | | SEQ ID NO:6859 | SEQ ID NO:14871 | SEQ ID NO:22883 |
| | | AA | SYGMH | VIWYDGSNKNYADSVKG | DQGVGYDGLDV |
| | | | SEQ ID NO:6860 | SEQ ID NO:14872 | SEQ ID NO:22884 |
| iPS:436154 | | NA | AATTATGATATCAAC | TGGATGCACCCTAACAGTGGTAACACAGGCTATGCACAGAAGTTCCAGGGC | AGCAGTGGCTGGTACTACTTTGACTAC |
| | 21-225_197C6 | | SEQ ID NO:6861 | SEQ ID NO:14873 | SEQ ID NO:22885 |
| | | AA | NYDIN | WMHPNSGNTGYAQKFQG | SSGWYYFDY |
| | | | SEQ ID NO:6862 | SEQ ID NO:14874 | SEQ ID NO:22886 |

FIGURE 49

| iPS:436156 | | NA | AGCTCTGCCATGACC | GCTATCATTGGTAATGGT GGTAGAGCATACTACGC AGACTCCGTGAAGGGC | GATCGGGGATATAGCAGGAT AGCAGTGGCTGGTACCTTTG ACTAC |
|---|---|---|---|---|---|
| | 21-225_197C8 | | SEQ ID NO:6863 | SEQ ID NO:14875 | SEQ ID NO:22887 |
| | | AA | SSAMT | AIIGNGGRAYYADSVKG | DRGYSRIAVAGTFDY |
| | | | SEQ ID NO:6864 | SEQ ID NO:14876 | SEQ ID NO:22888 |
| iPS:436158 | | NA | GCTTACTCCTGGAGC | CGTCTCTCTCCTGGTGGG AGCACCAACTTCAACCC CTCCCTCAAGAGT | CTCCGGTATAACTGGAACTT CCCTTACTTTGACTAC |
| | 21-225_197G8 | | SEQ ID NO:6865 | SEQ ID NO:14877 | SEQ ID NO:22889 |
| | | AA | AYSWS | RLSPGGSTNFNPSLKS | LRYNWNFPYFDY |
| | | | SEQ ID NO:6866 | SEQ ID NO:14878 | SEQ ID NO:22890 |
| iPS:436160 | | NA | AGCTATGCCATGAGC | GTTATTAGTGGTAGAGG TGGTAACACATACTACG CAGACTCCGTGAAGGGC | GGCATAGCAGTGGCTGGCTC GCACTACTTTGACTAC |
| | 21-225_197C9 | | SEQ ID NO:6867 | SEQ ID NO:14879 | SEQ ID NO:22891 |
| | | AA | SYAMS | VISGRGGNTYYADSVKG | GIAVAGSHYFDY |
| | | | SEQ ID NO:6868 | SEQ ID NO:14880 | SEQ ID NO:22892 |
| iPS:436164 | | NA | AGCTATGGCATGCAC | GTTACATGGTATGATGG AAGTAATAAATACTATG CAGACTCCGTGAAGGGC | GAATGGCTACAATTTAGGTA CTACTACGGTATAGACGTC |
| | 21-225_197G10 | | SEQ ID NO:6869 | SEQ ID NO:14881 | SEQ ID NO:22893 |
| | | AA | SYGMH | VTWYDGSNKYYADSVKG | EWLQFRYYYGIDV |
| | | | SEQ ID NO:6870 | SEQ ID NO:14882 | SEQ ID NO:22894 |
| iPS:436167 | | NA | AACTATGGCATGCAC | GTTATATGGTTTGATGGA AGTAATGACTACTATGC AGACTCCGTGAAGGGC | GATAGAAGCGTCGGCTACGA CGGTTTAGATGTC |
| | 21-225_197E11 | | SEQ ID NO:6871 | SEQ ID NO:14883 | SEQ ID NO:22895 |

FIGURE 49

| | | AA | NYGMH | VIWFDGSNDYYADSVKG | DRSVGYDGLDV |
|---|---|---|---|---|---|
| | | | SEQ ID NO:6872 | SEQ ID NO:14884 | SEQ ID NO:22896 |
| iPS:436173 | 21-225_197G12 | NA | AGCTATGGCATGCAC | GTTATATGGTATGATGG AAGTAATAAAAACTATG CAGACTCCGTGAAGGGC | GATCAGGGCGTGGGCTACGA CGGTTTGGACGTC |
| | | | SEQ ID NO:6873 | SEQ ID NO:14885 | SEQ ID NO:22897 |
| | | AA | SYGMH | VIWYDGSNKNYADSVKG | DQGVGYDGLDV |
| | | | SEQ ID NO:6874 | SEQ ID NO:14886 | SEQ ID NO:22898 |
| iPS:436177 | 21-225_198B1 | NA | AGTGGTGATTACTACTGGAA C | TACATCTTCCACAGTGGG AGCACCTACTACAACCC GTCCCTCAAGAGT | GGGGATTACGATGGTTCGGG GAGTTATCACTACTATTACG GTATGGACGTC |
| | | | SEQ ID NO:6875 | SEQ ID NO:14887 | SEQ ID NO:22899 |
| | | AA | SGDYYWN | YIFHSGSTYYNPSLKS | GDYDGSGSYHYYYGMDV |
| | | | SEQ ID NO:6876 | SEQ ID NO:14888 | SEQ ID NO:22900 |
| iPS:436179 | 21-225_198E1 | NA | AGTGGTGGTTACTACTGGAA C | TTCATCTTTTACAGTGGG AGCACCTACTACAACCC GTCCCTCAAGAGT | GGGGATTACGATGGTTCGGG GAGTTATCACTACTATTACG GTATGGACGTC |
| | | | SEQ ID NO:6877 | SEQ ID NO:14889 | SEQ ID NO:22901 |
| | | AA | SGGYYWN | FIFYSGSTYYNPSLKS | GDYDGSGSYHYYYGMDV |
| | | | SEQ ID NO:6878 | SEQ ID NO:14890 | SEQ ID NO:22902 |
| iPS:436181 | 21-225_198C2 | NA | AGTGGTGGTTACTACTGGAG C | AACATCTATTACAGTGG GAGCACCTACTACAACC CGTCCCTCAAGAGT | GGGGATTACTATGGTTCGGG GAGTTATCACAACTACTACG GTTTGGACGTC |
| | | | SEQ ID NO:6879 | SEQ ID NO:14891 | SEQ ID NO:22903 |
| | | AA | SGGYYWS | NIYYSGSTYYNPSLKS | GDYYGSGSYHNYYGLDV |
| | | | SEQ ID NO:6880 | SEQ ID NO:14892 | SEQ ID NO:22904 |

FIGURE 49

| iPS:436189 | | NA | AGCTATGGCATGCAC | GTTATATGGTATGATGG AAGTAATAAACACTATG CAGACTCCGTGAAGGGC | GATCAGGGCGTGGGCTACTA CGGTATGGACGTC |
|---|---|---|---|---|---|
| | 21-225_198B6 | | SEQ ID NO:6881 | SEQ ID NO:14893 | SEQ ID NO:22905 |
| | | AA | SYGMH | VIWYDGSNKHYADSVKG | DQGVGYYGMDV |
| | | | SEQ ID NO:6882 | SEQ ID NO:14894 | SEQ ID NO:22906 |
| iPS:436191 | | NA | AACTATGGCATGCAC | ATTATATGGTTTGATGGA AGTAATAAATACTATGT AGACTCCGTGAAGGGC | GAATGGCTACAATTCAGGTA CTACTACGGTATGGACGTC |
| | 21-225_198B9 | | SEQ ID NO:6883 | SEQ ID NO:14895 | SEQ ID NO:22907 |
| | | AA | NYGMH | IIWFDGSNKYYVDSVKG | EWLQFRYYYGMDV |
| | | | SEQ ID NO:6884 | SEQ ID NO:14896 | SEQ ID NO:22908 |
| iPS:436193 | | NA | AGTTACCACTGGAGT | CATATCTATACCAGTAG GAGCACCAACTACAACC CCTCCCTCAAGAGT | CTCCGGTATAACTGGAACTT CCCTTACTTTGACTAC |
| | 21-225_198A10 | | SEQ ID NO:6885 | SEQ ID NO:14897 | SEQ ID NO:22909 |
| | | AA | SYHWS | HIYTSRSTNYNPSLKS | LRYNWNFPYFDY |
| | | | SEQ ID NO:6886 | SEQ ID NO:14898 | SEQ ID NO:22910 |
| iPS:436195 | | NA | AGTGGTGATTACTACTGGAA C | TACATCTTTTACAGTGGG AGCACCTACTACAACCC GTCCCTCAAGAGT | GGGGATTACGATGGTTCGGG GAGTTATCACTTCTACTACG GTATGGACGTC |
| | 21-225_198G10 | | SEQ ID NO:6887 | SEQ ID NO:14899 | SEQ ID NO:22911 |
| | | AA | SGDYYWN | YIFYSGSTYYNPSLKS | GDYDGSGSYHFYYGMDV |
| | | | SEQ ID NO:6888 | SEQ ID NO:14900 | SEQ ID NO:22912 |
| iPS:436197 | 21 225 199C2 | NA | AGTGGTGATTACTACTGGAA C | TACATCTTTTACAGTGGG AGCACCTACTACAACCC GTCCCTCAAGAGT | GGGGATTACGATGGTTCGGG GAGTTATCACTACTACTACG GTATGGACGTC |

FIGURE 49

| | | | | | |
|---|---|---|---|---|---|
| | 21-225_199C2 | | SEQ ID NO:6889 | SEQ ID NO:14901 | SEQ ID NO:22913 |
| | | AA | SGDYYWN | YIFYSGSTYYNPSLKS | GDYDGSGSYHYYYGMDV |
| | | | SEQ ID NO:6890 | SEQ ID NO:14902 | SEQ ID NO:22914 |
| iPS:436199 | 21-225_199E3 | NA | GGTTACTTCTGGACC | GAAATCAATCATAGTGG AAGCACCAACTACAACC CGTCCCTCAAGAGT | GACTACGGTGCTGACTAC |
| | | | SEQ ID NO:6891 | SEQ ID NO:14903 | SEQ ID NO:22915 |
| | | AA | GYFWT | EINHSGSTNYNPSLKS | DYGADY |
| | | | SEQ ID NO:6892 | SEQ ID NO:14904 | SEQ ID NO:22916 |
| iPS:436201 | 21-225_199C5 | NA | AGCTATGGCATGCAC | GTTATATGGTATGATGG AAGTAATAAAAACTATG CAGACTCCGTGAAGGGC | GATCAGGGCGTGGGCTACTA CGGTATGGACGTC |
| | | | SEQ ID NO:6893 | SEQ ID NO:14905 | SEQ ID NO:22917 |
| | | AA | SYGMH | VIWYDGSNKNYADSVKG | DQGVGYYGMDV |
| | | | SEQ ID NO:6894 | SEQ ID NO:14906 | SEQ ID NO:22918 |
| iPS:436203 | 21-225_199A6 | NA | AACTATGGCATGCAC | ATTATATGGTTTGATGGA AGTAATCAATACTATGC CGACTCCGTGAAGGGC | GCCCACGGGGTCTACTACTA CGCTATGGACGTC |
| | | | SEQ ID NO:6895 | SEQ ID NO:14907 | SEQ ID NO:22919 |
| | | AA | NYGMH | IIWFDGSNQYYADSVKG | AHGVYYYAMDV |
| | | | SEQ ID NO:6896 | SEQ ID NO:14908 | SEQ ID NO:22920 |
| iPS:436205 | 21-225_199A7 | NA | AACTATGGCATGCAC | ATTATATGGTTTGATGGA AGTAATAAATACTATGC AGACTCCGTGAAGGGC | GAATGGCTACAATTCAGGTA CTACTACGGTATGGACGTC |
| | | | SEQ ID NO:6897 | SEQ ID NO:14909 | SEQ ID NO:22921 |
| | | AA | NYGMH | IIWFDGSNKYYADSVKG | EWLQFRYYYGMDV |
| | | | SEQ ID NO:6898 | SEQ ID NO:14910 | SEQ ID NO:22922 |

FIGURE 49

| iPS:436207 | 21-225_199C7 | NA | AGTGGTGGTTACTACTGGAAC | TTCATCTTTTACAGTGGGAGCACCTACTACAACCCGTCCCTCAAGAGT | GGGGATTACGATGGTTCGGGGAGTTATCACTACTATTACGGTATGGACGTC |
|---|---|---|---|---|---|
| | | | SEQ ID NO:6899 | SEQ ID NO:14911 | SEQ ID NO:22923 |
| | | AA | SGGYYWN | FIFYSGSTYYNPSLKS | GDYDGSGSYHYYYGMDV |
| | | | SEQ ID NO:6900 | SEQ ID NO:14912 | SEQ ID NO:22924 |
| iPS:436210 | 21-225_199G11 | NA | AGTGGTGGTTACTACTGGAGC | AACATCTATTACAGTGGGAGCACCTACTACAACCCGTCCCTCAAGAGT | GGGGATTACTATGGTTCGGGGAGTTATCACAACTACTACGGTTTGGACGTC |
| | | | SEQ ID NO:6901 | SEQ ID NO:14913 | SEQ ID NO:22925 |
| | | AA | SGGYYWS | NIYYSGSTYYNPSLKS | GDYYGSGSYHNYYGLDV |
| | | | SEQ ID NO:6902 | SEQ ID NO:14914 | SEQ ID NO:22926 |
| iPS:436212 | 21-225_200G1 | NA | AGCTATGCCATGAGC | GCTATTAGTGGTAGAGGCGGTAATACATTCTACGCAGACTCCGTGAGGGGC | CGTATAGCAGTGGATGGCTATGATGCTTTTGATGTC |
| | | | SEQ ID NO:6903 | SEQ ID NO:14915 | SEQ ID NO:22927 |
| | | AA | SYAMS | AISGRGGNTFYADSVRG | RIAVDGYDAFDV |
| | | | SEQ ID NO:6904 | SEQ ID NO:14916 | SEQ ID NO:22928 |
| iPS:436214 | 21-225_200F6 | NA | AGTTATGGCATGCAC | GTTATATGGTATGATGGAAGTAATAAATACTATGCAGACTCCGTGAAGGGC | GAATGGCTACAATTTAGGTATTACTACGGTATGGACGTC |
| | | | SEQ ID NO:6905 | SEQ ID NO:14917 | SEQ ID NO:22929 |
| | | AA | SYGMH | VIWYDGSNKYYADSVKG | EWLQFRYYYGMDV |
| | | | SEQ ID NO:6906 | SEQ ID NO:14918 | SEQ ID NO:22930 |
| iPS:436216 | 21-225_200B7 | NA | AGTGGTGGTTACTACTGGAGC | TACATCTTTTACAGTGGGAGCACCAACTACAACCCGTCCCTCAGGAGT | GCCGGGTATAACTGGAACAACGGTATGGACGTC |
| | | | SEQ ID NO:6907 | SEQ ID NO:14919 | SEQ ID NO:22931 |

FIGURE 49

| | | AA | SGGYYWS | YIFYSGSTNYNPSLRS | AGYNWNNGMDV |
|---|---|---|---|---|---|
| | | | SEQ ID NO:6908 | SEQ ID NO:14920 | SEQ ID NO:22932 |
| iPS:436218 | 21-225_200G7 | NA | AATTATGATATCAAC | TGGATGCACCTTAACAG TGGTAACACAGGCTATG CACCGAAGTTCCAGGGC | AGCGGTGGCTGGTACGTGTT CGACCCC |
| | | | SEQ ID NO:6909 | SEQ ID NO:14921 | SEQ ID NO:22933 |
| | | AA | NYDIN | WMHLNSGNTGYAPKFQG | SGGWYVFDP |
| | | | SEQ ID NO:6910 | SEQ ID NO:14922 | SEQ ID NO:22934 |
| iPS:436220 | 21-225_200F8 | NA | AATTACTACTGGAGC | CGTATCTATACCAGTGG GAGCACCAACTACAACC CCTCCCTCAAGAGT | GATCGGGGATACTATGGCTA CTACGGTATGGACGTC |
| | | | SEQ ID NO:6911 | SEQ ID NO:14923 | SEQ ID NO:22935 |
| | | AA | NYYWS | RIYTSGSTNYNPSLKS | DRGYYGYYGMDV |
| | | | SEQ ID NO:6912 | SEQ ID NO:14924 | SEQ ID NO:22936 |
| iPS:436222 | 21-225_200C9 | NA | AGCTATGGCATGCAC | GTTATATCATATGATGGA GGTTATAAAAACTATAT AGACTCCGTGAAGGGC | GGTACCCACGGGTACTACTA CGGTGTGGACGTC |
| | | | SEQ ID NO:6913 | SEQ ID NO:14925 | SEQ ID NO:22937 |
| | | AA | SYGMH | VISYDGGYKNYIDSVKG | GTHGYYYGVDV |
| | | | SEQ ID NO:6914 | SEQ ID NO:14926 | SEQ ID NO:22938 |
| iPS:436226 | 21-225_200F10 | NA | AGTGGTGATTACTACTGGAA C | TACATCTTTTACAGTGGG AGCACCTACTACAACCC GTCCCTCAAGAGT | GGGGATTACGATGGTTCGGG GAGTTATCACTACTACTACG GTATGGACGTC |
| | | | SEQ ID NO:6915 | SEQ ID NO:14927 | SEQ ID NO:22939 |
| | | AA | SGDYYWN | YIFYSGSTYYNPSLKS | GDYDGSGSYHYYYGMDV |
| | | | SEQ ID NO:6916 | SEQ ID NO:14928 | SEQ ID NO:22940 |

FIGURE 49

| iPS:436228 | 21-225_200F12 | NA | GGTTACTTCTGGACC | GAAATCAGTCATAGTGG AAGCACCAACTACAACC CGTCCCTCAAGAGT | GACTACGGGGCGGACTAC |
| | | | SEQ ID NO:6917 | SEQ ID NO:14929 | SEQ ID NO:22941 |
| | | AA | GYFWT | EISHSGSTNYNPSLKS | DYGADY |
| | | | SEQ ID NO:6918 | SEQ ID NO:14930 | SEQ ID NO:22942 |
| iPS:436230 | 21-225_201A1 | NA | GGTTACTTCTGGACC | GAAATCAGTCATAGTGG ACGCACCAACTACAACC CGTCCCTCAAGAGT | GACTACGGGGCGGACTAC |
| | | | SEQ ID NO:6919 | SEQ ID NO:14931 | SEQ ID NO:22943 |
| | | AA | GYFWT | EISHSGRTNYNPSLKS | DYGADY |
| | | | SEQ ID NO:6920 | SEQ ID NO:14932 | SEQ ID NO:22944 |
| iPS:436232 | 21-225_201E1 | NA | CCTTACTACTGGAGC | GAAGTCAATCATAGTGG AAGCACCAACTACAACC CGTCCCTCAAGAGT | GACTACGGGGGTTTAGACTAC |
| | | | SEQ ID NO:6921 | SEQ ID NO:14933 | SEQ ID NO:22945 |
| | | AA | PYYWS | EVNHSGSTNYNPSLKS | DYGGLDY |
| | | | SEQ ID NO:6922 | SEQ ID NO:14934 | SEQ ID NO:22946 |
| iPS:436234 | 21-225_51E3 | NA | AGCTATGGTATCAGC | TGGATCAGCGCTTATAAT GGTAACACAAAGAATGC ACAGAGGTTCCAGGGC | CACGATTTTTGGAGTGGTTA TTATAAGGGTATGGACGTC |
| | | | SEQ ID NO:6923 | SEQ ID NO:14935 | SEQ ID NO:22947 |
| | | AA | SYGIS | WISAYNGNTKNAQRFQG | HDFWSGYYKGMDV |
| | | | SEQ ID NO:6924 | SEQ ID NO:14936 | SEQ ID NO:22948 |
| iPS:436236 | 21-225_201F7 | NA | AGCAACAGTGCTGCTTGGAA C | AGGACATACTACAGGTC CAAGTGGTATAATTATTA TGAAGTATCTGTGAGAA GT | GATCAACGGTACTACGGTAT GGACGTC |
| | | | SEQ ID NO:6925 | SEQ ID NO:14937 | SEQ ID NO:22949 |

FIGURE 49

| | | AA | SNSAAWN | RTYYRSKWYNYYEVSVRS | DQRYYGMDV |
|---|---|---|---|---|---|
| | | | SEQ ID NO:6926 | SEQ ID NO:14938 | SEQ ID NO:22950 |
| iPS:436238 | 21-225_201B2 | NA | GTTTACTACTGGACC | GAAATCAATCATAGTGG AAGCACCAACTACAACC CGTCCCTCAAGAGT | GACTATGGTGTCTTTGATTA C |
| | | | SEQ ID NO:6927 | SEQ ID NO:14939 | SEQ ID NO:22951 |
| | | AA | VYYWT | EINHSGSTNYNPSLKS | DYGVFDY |
| | | | SEQ ID NO:6928 | SEQ ID NO:14940 | SEQ ID NO:22952 |
| iPS:436240 | 21-225_201E8 | NA | GGCTACTATATGCAC | TGGATCGACCCTAACAG TGGTGGCACAAACTATC CACAGAAGTTTCAGGGC | GATCAAGGGTATAACTGGAA CTCTTTTGACTAC |
| | | | SEQ ID NO:6929 | SEQ ID NO:14941 | SEQ ID NO:22953 |
| | | AA | GYYMH | WIDPNSGGTNYPQKFQG | DQGYNWNSFDY |
| | | | SEQ ID NO:6930 | SEQ ID NO:14942 | SEQ ID NO:22954 |
| iPS:436242 | 21-225_201A10 | NA | GGTTACTTCTGGACC | GAAATCAGTCATAGTGG AAGCACCAACTACAACC CGTCCCTCAAGAGT | GACTACGGGGCGGACTAC |
| | | | SEQ ID NO:6931 | SEQ ID NO:14943 | SEQ ID NO:22955 |
| | | AA | GYFWT | EISHSGSTNYNPSLKS | DYGADY |
| | | | SEQ ID NO:6932 | SEQ ID NO:14944 | SEQ ID NO:22956 |
| iPS:436244 | 21-225_201H10 | NA | GGCTACTATATCCAC | TGGATCAACCCTAATAG TGGTGGCACAAACTATG CACAGAAGTTTCAGGGC | GGATACAGCTATGGTTACAA CTGGTTCGACCCC |
| | | | SEQ ID NO:6933 | SEQ ID NO:14945 | SEQ ID NO:22957 |
| | | AA | GYYIH | WINPNSGGTNYAQKFQG | GYSYGYNWFDP |
| | | | SEQ ID NO:6934 | SEQ ID NO:14946 | SEQ ID NO:22958 |
| iPS:436246 | 21 225 201G6 | NA | AGCTATGCCATGAGC | ACTATTAGTGGTAGTGGT GTTAGAACATACTACGC AGACTCCGTGAAGGGC | GGGGGAGCTAGGAGCAGTG GCTGGTTCCACTTTGACTAC |

FIGURE 49

| | 21-225_201G6 | | SEQ ID NO:6935 | SEQ ID NO:14947 | SEQ ID NO:22959 |
|---|---|---|---|---|---|
| | | AA | SYAMS | TISGSGVRTYYADSVKG | GGARSSGWFHFDY |
| | | | SEQ ID NO:6936 | SEQ ID NO:14948 | SEQ ID NO:22960 |
| iPS:436248 | | NA | AGTTATGATATCAAC | TGGATGAACCCTAAGAG AGGTAACACAGGCTATG CACAGAAGTTCCAGGGC | GGAAGGTATAGCAGGGAGG ATTACTACTACTATTATGAT ATGGACGTC |
| | 21-225_202A3 | | SEQ ID NO:6937 | SEQ ID NO:14949 | SEQ ID NO:22961 |
| | | AA | SYDIN | WMNPKRGNTGYAQKFQG | GRYSREDYYYYYDMDV |
| | | | SEQ ID NO:6938 | SEQ ID NO:14950 | SEQ ID NO:22962 |
| iPS:436250 | | NA | AGCAACAGTGCTGCTTGGAA C | AGGACATACTACAGGTC CAAGTGGTATAATTATTA TGAAGTATCTGTGAAAA GT | GATCAACGGTACTACGGTAT GGACGTC |
| | 21-225_201A4 | | SEQ ID NO:6939 | SEQ ID NO:14951 | SEQ ID NO:22963 |
| | | AA | SNSAAWN | RTYYRSKWYNYYEVSVK S | DQRYYGMDV |
| | | | SEQ ID NO:6940 | SEQ ID NO:14952 | SEQ ID NO:22964 |
| iPS:436252 | | NA | AGCAACAGTGCTGCTTGGAA C | AGGACATACTACAGGTC CAAGTGGTATAATGAGT ATGCAGTATCTGTGAGA AGT | GATCAACGGTACTACGGTAT GGACGTC |
| | 21-225_202A8 | | SEQ ID NO:6941 | SEQ ID NO:14953 | SEQ ID NO:22965 |
| | | AA | SNSAAWN | RTYYRSKWYNEYAVSVRS | DQRYYGMDV |
| | | | SEQ ID NO:6942 | SEQ ID NO:14954 | SEQ ID NO:22966 |
| iPS:436254 | | NA | AGCTATGCCATGAGC | ACTATTAGTGGTAGTGGT GTTAGAACATACTACGC AGACTCCGTGAAGGGC | GGGGGAGCTAGGAGCAGTG GCTGGTTCCACTTTGACTAC |
| | 21-225_202C12 | | SEQ ID NO:6943 | SEQ ID NO:14955 | SEQ ID NO:22967 |

FIGURE 49

| | | AA | SYAMS | TISGSGVRTYYADSVKG | GGARSSGWFHFDY |
|---|---|---|---|---|---|
| | | | SEQ ID NO:6944 | SEQ ID NO:14956 | SEQ ID NO:22968 |
| iPS:436256 | 21-225_202D9 | NA | CCTTACTACTGGAGC | GAAATCAATCATAGTGGAAGCACCAACTACAATCCGTCCCTCAAGAGT | GACTACGGGGGTTTAGACTAC |
| | | | SEQ ID NO:6945 | SEQ ID NO:14957 | SEQ ID NO:22969 |
| | | AA | PYYWS | EINHSGSTNYNPSLKS | DYGGLDY |
| | | | SEQ ID NO:6946 | SEQ ID NO:14958 | SEQ ID NO:22970 |
| iPS:436258 | 21-225_202F12 | NA | TACTATGGCATGCAC | ATTATATGGTATGATGGAAGTAATAAATTCTATGCAGACTCCGTGAAGGGC | AATATAGCAGCAGCTGCCCCTTACTTTGACTAC |
| | | | SEQ ID NO:6947 | SEQ ID NO:14959 | SEQ ID NO:22971 |
| | | AA | YYGMH | IIWYDGSNKFYADSVKG | NIAAAAPYFDY |
| | | | SEQ ID NO:6948 | SEQ ID NO:14960 | SEQ ID NO:22972 |
| iPS:436260 | 21-225_203H1 | NA | AGCTATGGCATGCAC | GTTATATGGTATGATGGAAGTAATAAAAACTATGCAGACTCCGTGAAGGGC | GATAGAACAGTTGGCTACAACGGTATGGACGTC |
| | | | SEQ ID NO:6949 | SEQ ID NO:14961 | SEQ ID NO:22973 |
| | | AA | SYGMH | VIWYDGSNKNYADSVKG | DRTVGYNGMDV |
| | | | SEQ ID NO:6950 | SEQ ID NO:14962 | SEQ ID NO:22974 |
| iPS:436262 | 21-225_203E3 | NA | GGCTACTATATCCAC | TGGATCAACCCTAATAGTGGTGGCACAAACTATGCACAGAAGTTTCAGGGC | GGATACAGCTATGGTTACAACTGGTTCGACCCC |
| | | | SEQ ID NO:6951 | SEQ ID NO:14963 | SEQ ID NO:22975 |
| | | AA | GYYIH | WINPNSGGTNYAQKFQG | GYSYGYNWFDP |
| | | | SEQ ID NO:6952 | SEQ ID NO:14964 | SEQ ID NO:22976 |

FIGURE 49

| iPS:436264 | | NA | GACTATGTCATGCAC | GTTATATGGTATGATGG AAGTAATAAATACTATG TAGACTCCGTGAAGGGC | GAACGGTATAGCAGTGGCTT GTACGACTACGGTATGGACG TC |
|---|---|---|---|---|---|
| | 21-225_203F7 | | SEQ ID NO:6953 | SEQ ID NO:14965 | SEQ ID NO:22977 |
| | | AA | DYVMH | VIWYDGSNKYYVDSVKG | ERYSSGLYDYGMDV |
| | | | SEQ ID NO:6954 | SEQ ID NO:14966 | SEQ ID NO:22978 |
| iPS:436268 | | NA | AGTTTTGGCATGCAC | GTTATATGGTATGATGTA AATAATAAATACTATGC AGACTCCGTGAAGGGC | GAACTGGGGTTCCTCTCTGA CTAC |
| | 21-225_203B9 | | SEQ ID NO:6955 | SEQ ID NO:14967 | SEQ ID NO:22979 |
| | | AA | SFGMH | VIWYDVNNKYYADSVKG | ELGFLSDY |
| | | | SEQ ID NO:6956 | SEQ ID NO:14968 | SEQ ID NO:22980 |
| iPS:436270 | | NA | GACTACTACATGAGC | TACATTAGTGGTAGTGGT ACTACCACATACTACGC AGACTCTGTGAAGGGC | GATAGGGGGGGTTTGGACGT C |
| | 21-225_203F10 | | SEQ ID NO:6957 | SEQ ID NO:14969 | SEQ ID NO:22981 |
| | | AA | DYYMS | YISGSGTTTYYADSVKG | DRGGLDV |
| | | | SEQ ID NO:6958 | SEQ ID NO:14970 | SEQ ID NO:22982 |
| iPS:436272 | | NA | AATTATGATATCAAC | TGGATGCACCCTAACAG TGGTAACACAGGCTATG CACAGAAGTTCCAGGGC | AGCAGTGGCTGGTACTACTT TGACTAC |
| | 21-225_201F5 | | SEQ ID NO:6959 | SEQ ID NO:14971 | SEQ ID NO:22983 |
| | | AA | NYDIN | WMHPNSGNTGYAQKFQG | SSGWYYFDY |
| | | | SEQ ID NO:6960 | SEQ ID NO:14972 | SEQ ID NO:22984 |

FIGURE 49

| iPS:436274 | | NA | AGCTATGTCATGCAC | GTTATATGGTATGATGG AAGTAATAAATATAATG CAGACTCCGTGAAGGGC | GAACCGTATAGCAGCAGCTG GTACGACTACGGTATGGACG TC |
|---|---|---|---|---|---|
| | 21-225_204H3 | | SEQ ID NO:6961 | SEQ ID NO:14973 | SEQ ID NO:22985 |
| | | AA | SYVMH | VIWYDGSNKYNADSVKG | EPYSSSWYDYGMDV |
| | | | SEQ ID NO:6962 | SEQ ID NO:14974 | SEQ ID NO:22986 |
| iPS:436276 | | NA | GGCTACTATATCCAC | TGGATCAACCCTAATAG TGGTGGCACAAACTATG CACAGAAGTTTCAGGGC | GGATACAGCTATGGTTACAA CTGGTTCGACCCC |
| | 21-225_204H4 | | SEQ ID NO:6963 | SEQ ID NO:14975 | SEQ ID NO:22987 |
| | | AA | GYYIH | WINPNSGGTNYAQKFQG | GYSYGYNWFDP |
| | | | SEQ ID NO:6964 | SEQ ID NO:14976 | SEQ ID NO:22988 |
| iPS:436278 | | NA | AGCAACAGTGCTGCTTGGAA C | AGGACATACTACAGGTC CAAGTGGTATAATTATTA TGAAGTATCTGTGAGAA GT | GATCAACGGTACTACGGTAT GGACGTC |
| | 21-225_201F2 | | SEQ ID NO:6965 | SEQ ID NO:14977 | SEQ ID NO:22989 |
| | | AA | SNSAAWN | RTYYRSKWYNYYEVSVRS | DQRYYGMDV |
| | | | SEQ ID NO:6966 | SEQ ID NO:14978 | SEQ ID NO:22990 |
| iPS:436280 | | NA | ACCTATGCCATGAGC | GCTATTAGTGGTAGTGGT GGTAGCACATATTACGC AGACTCCGTGAAGGGC | GGGATAAGTGGAACCGGCTC CTACTACTACTACGGTGTGG ACGTC |
| | 21-225_204D6 | | SEQ ID NO:6967 | SEQ ID NO:14979 | SEQ ID NO:22991 |
| | | AA | TYAMS | AISGSGGSTYYADSVKG | GISGTGSYYYYGVDV |
| | | | SEQ ID NO:6968 | SEQ ID NO:14980 | SEQ ID NO:22992 |

FIGURE 49

| iPS:436282 | | NA | AGCTATGGCATGCAC | GTTATATGGTATGATGG AAGTAATAAAAACTATG CAGACTCCGTGAAGGGC | GATCGAGGTGTCGGCTACGA CGGTATGGACGTC |
|---|---|---|---|---|---|
| | 21-225_204G6 | | SEQ ID NO:6969 | SEQ ID NO:14981 | SEQ ID NO:22993 |
| | | AA | SYGMH | VIWYDGSNKNYADSVKG | DRGVGYDGMDV |
| | | | SEQ ID NO:6970 | SEQ ID NO:14982 | SEQ ID NO:22994 |
| iPS:436284 | | NA | AGCTATGGCATGCAC | GTTATATGGTATGATGGT AGCAATGAAAATTATGT AGCCTCCGTGAAGGGC | GATCTGGGGATAGGGTATTA CGGTATGGACGTC |
| | 21-225_204G8 | | SEQ ID NO:6971 | SEQ ID NO:14983 | SEQ ID NO:22995 |
| | | AA | SYGMH | VIWYDGSNENYVASVKG | DLGIGYYGMDV |
| | | | SEQ ID NO:6972 | SEQ ID NO:14984 | SEQ ID NO:22996 |
| iPS:436286 | | NA | GGTTACTTCTGGACC | GAAATCAGTCATAGTGG AAGCACCAGTTACAACC CGTCCCTCAAGAGT | GACTACGGGGCCGACTAC |
| | 21-225_204H8 | | SEQ ID NO:6973 | SEQ ID NO:14985 | SEQ ID NO:22997 |
| | | AA | GYFWT | EISHSGSTSYNPSLKS | DYGADY |
| | | | SEQ ID NO:6974 | SEQ ID NO:14986 | SEQ ID NO:22998 |
| iPS:436290 | | NA | GGTCACTACTGGAGC | GAAATGTATCATTTTGGA AACACCAACTACAACCC GTCCCTCAAGAGT | GTGGGGCAGTGGCTGGCTTT TGATATC |
| | 21-225_205G3 | | SEQ ID NO:6975 | SEQ ID NO:14987 | SEQ ID NO:22999 |
| | | AA | GHYWS | EMYHFGNTNYNPSLKS | VGQWLAFDI |
| | | | SEQ ID NO:6976 | SEQ ID NO:14988 | SEQ ID NO:23000 |

FIGURE 49

| iPS:436292 | | NA | AGCTATGGCATGCAC | GTTATATGGTATGATGG AAGTAATAAATACTATG CAGACTCCGTGAAGGGC | GATAGATCAGTTGGCTACGA CGGTACGGACGTC |
|---|---|---|---|---|---|
| | 21-225_205H3 | | SEQ ID NO:6977 | SEQ ID NO:14989 | SEQ ID NO:23001 |
| | | AA | SYGMH | VIWYDGSNKYYADSVKG | DRSVGYDGTDV |
| | | | SEQ ID NO:6978 | SEQ ID NO:14990 | SEQ ID NO:23002 |
| iPS:436294 | | NA | AGCAACAGTGCTGCTTGGAA C | AGGACATATTACAGGTC CAAGTGGTATAATTATTA TGAAGTATCTGTGAGAA GT | GATCAACGGTACTACGGTAT GGACGTC |
| | 21-225_205G4 | | SEQ ID NO:6979 | SEQ ID NO:14991 | SEQ ID NO:23003 |
| | | AA | SNSAAWN | RTYYRSKWYNYYEVSVRS | DQRYYGMDV |
| | | | SEQ ID NO:6980 | SEQ ID NO:14992 | SEQ ID NO:23004 |
| iPS:436296 | | NA | AGATATGGCATGCAC | GTTATATGGTATGATGG AAGTAATGAGAATTATG TAGACTCCGTGAAGGGC | GATATGGGGATAGGGTATTA TGGTATGGACGTC |
| | 21-225_205F5 | | SEQ ID NO:6981 | SEQ ID NO:14993 | SEQ ID NO:23005 |
| | | AA | RYGMH | VIWYDGSNENYVDSVKG | DMGIGYYGMDV |
| | | | SEQ ID NO:6982 | SEQ ID NO:14994 | SEQ ID NO:23006 |
| iPS:436302 | | NA | GTTTATTACTGGAGC | GAAAGCAATCAGAGTGG ACGCACCACCTACAACC CGTCCCTCAAGAGT | GACTACGGTGTCTTTGACTA C |
| | 21-225_205G7 | | SEQ ID NO:6983 | SEQ ID NO:14995 | SEQ ID NO:23007 |
| | | AA | VYYWS | ESNQSGRTTYNPSLKS | DYGVFDY |
| | | | SEQ ID NO:6984 | SEQ ID NO:14996 | SEQ ID NO:23008 |

FIGURE 49

| iPS:436304 | 21-225_201F3 | NA | AGCTATGCCATGAGC | ACTATTAGTGGTAGTGGT GTTAGAACATACTACGC AGACTCCGTGAAGGGC | GGGGGAGCTAGGAGCAGTG GCTGGTTCCACTTTGACTAC |
| --- | --- | --- | --- | --- | --- |
| | | | SEQ ID NO:6985 | SEQ ID NO:14997 | SEQ ID NO:23009 |
| | | AA | SYAMS | TISGSGVRTYYADSVKG | GGARSSGWFHFDY |
| | | | SEQ ID NO:6986 | SEQ ID NO:14998 | SEQ ID NO:23010 |
| iPS:436306 | 21-225_201H4 | NA | AGCTATGCCATGCAC | GCTATATGGTATGATGG AAGTAATAAATACAATG CAGACTCCGTGAAGGGC | GATGTGGGTACAGTGGGAGC TACCTACTTTGACTGC |
| | | | SEQ ID NO:6987 | SEQ ID NO:14999 | SEQ ID NO:23011 |
| | | AA | SYAMH | AIWYDGSNKYNADSVKG | DVGTVGATYFDC |
| | | | SEQ ID NO:6988 | SEQ ID NO:15000 | SEQ ID NO:23012 |
| iPS:436308 | 21-225_205H8 | NA | GGTTACTTCTGGAGC | GAAATCAGTCATAGTGG ACGCACCAACTACAACC CGTCCCTCAAGAGC | GACTACGGGGCGGACTAC |
| | | | SEQ ID NO:6989 | SEQ ID NO:15001 | SEQ ID NO:23013 |
| | | AA | GYFWS | EISHSGRTNYNPSLKS | DYGADY |
| | | | SEQ ID NO:6990 | SEQ ID NO:15002 | SEQ ID NO:23014 |
| iPS:436310 | 21-225_202D11 | NA | GGTCCCTACTGGAGC | GAAATCAATCATAGTGG AAGCACCAACTACAACC CGTCCCTCAAGAGT | GACTACGGTGTCCTTGACTA C |
| | | | SEQ ID NO:6991 | SEQ ID NO:15003 | SEQ ID NO:23015 |
| | | AA | GPYWS | EINHSGSTNYNPSLKS | DYGVLDY |
| | | | SEQ ID NO:6992 | SEQ ID NO:15004 | SEQ ID NO:23016 |
| iPS:436312 | 21-225_206A4 | NA | GGCTACTATATCCAC | TGGATCAACCCTAATAG TGGTGGCACAAACTATG CACAGAAGTTTCAGGGC | GGATACAGCTATGGTTACAA CTGGTTCGACCCC |
| | | | SEQ ID NO:6993 | SEQ ID NO:15005 | SEQ ID NO:23017 |

FIGURE 49

| | | | AA | GYYIH | WINPNSGGTNYAQKFQG | GYSYGYNWFDP |
|---|---|---|---|---|---|---|
| | | | | SEQ ID NO:6994 | SEQ ID NO:15006 | SEQ ID NO:23018 |
| iPS:436314 | 21-225_206G4 | | NA | GGCTACTATATACAC | TGGATCGACCCTAATAG TGGTGGCACAAACTATG CACAGAAGTTTCAGGGC | GATCAAGGGTATAACTGGAA CTCTTTTGACTAC |
| | | | | SEQ ID NO:6995 | SEQ ID NO:15007 | SEQ ID NO:23019 |
| | | | AA | GYYIH | WIDPNSGGTNYAQKFQG | DQGYNWNSFDY |
| | | | | SEQ ID NO:6996 | SEQ ID NO:15008 | SEQ ID NO:23020 |
| iPS:436316 | 21-225_206A5 | | NA | GGCTACTATATCCAC | TGGATCAACCCTAATAG TGGTGGCACAAACTATG CACAGAAGTTTCAGGGC | GGATACAGCTATGGTTACAA CTGGTTCGACCCC |
| | | | | SEQ ID NO:6997 | SEQ ID NO:15009 | SEQ ID NO:23021 |
| | | | AA | GYYIH | WINPNSGGTNYAQKFQG | GYSYGYNWFDP |
| | | | | SEQ ID NO:6998 | SEQ ID NO:15010 | SEQ ID NO:23022 |
| iPS:436324 | 21-225_207G6 | | NA | AGCTATGGCATGCAC | GTTATATGGTATGATGG AAGTAATAAAAACTATG CAGAGTCCGTGAAGGGC | GATGCGGGTATTGGATACTA CGGTATAGACGTC |
| | | | | SEQ ID NO:6999 | SEQ ID NO:15011 | SEQ ID NO:23023 |
| | | | AA | SYGMH | VIWYDGSNKNYAESVKG | DAGIGYYGIDV |
| | | | | SEQ ID NO:7000 | SEQ ID NO:15012 | SEQ ID NO:23024 |
| iPS:436328 | 21-225_207F12 | | NA | AACTATGGCATGCAC | GTTATATGGTATGATAG AAATAATAAATACTATG GTGACTCCGTGAAGGGC | GAACTGGGGTTCCTCTTTGA CTAC |
| | | | | SEQ ID NO:7001 | SEQ ID NO:15013 | SEQ ID NO:23025 |
| | | | AA | NYGMH | VIWYDRNNKYYGDSVKG | ELGFLFDY |
| | | | | SEQ ID NO:7002 | SEQ ID NO:15014 | SEQ ID NO:23026 |

FIGURE 49

| iPS:436332 | | NA | GACTGTGTCATGCAC | GTTATATGGTATGATGG AAGTAATAAATACTATG TAGACTCCGTGAAGGGC | GAACGGTATAGCAGTGGCTT GTACGACTACGGTTTGGACG TC |
|---|---|---|---|---|---|
| | 21-225_208B2 | | SEQ ID NO:7003 | SEQ ID NO:15015 | SEQ ID NO:23027 |
| | | AA | DCVMH | VIWYDGSNKYYVDSVKG | ERYSSGLYDYGLDV |
| | | | SEQ ID NO:7004 | SEQ ID NO:15016 | SEQ ID NO:23028 |
| iPS:436334 | | NA | AGCTATGCCATGAGC | ACTATTAGTGGTAGTGGT GTTAGAACATACTACGC AGACTCCGTGAAGGGC | GGGGGAGCTAGGAGCAGTG GCTGGTTCCACTTTGACTAC |
| | 21-225_208G3 | | SEQ ID NO:7005 | SEQ ID NO:15017 | SEQ ID NO:23029 |
| | | AA | SYAMS | TISGSGVRTYYADSVKG | GGARSSGWFHFDY |
| | | | SEQ ID NO:7006 | SEQ ID NO:15018 | SEQ ID NO:23030 |
| iPS:436336 | | NA | GTTTACTACTGGACC | GAAATCAATCATAGTGG AAGCACCAACTACAACC CGTCCCTCAAGAGT | GACTATGGTGTCTTTGATTA C |
| | 21-225_208B5 | | SEQ ID NO:7007 | SEQ ID NO:15019 | SEQ ID NO:23031 |
| | | AA | VYYWT | EINHSGSTNYNPSLKS | DYGVFDY |
| | | | SEQ ID NO:7008 | SEQ ID NO:15020 | SEQ ID NO:23032 |
| iPS:436338 | | NA | GGCTACTATATCCAC | TGGATCAACCCTAATAG TGGTGGCACAAACTATG CACAGAAGTTTCAGGGC | GGATACAGCTATGGTTACAA CTGGTTCGACCCC |
| | 21-225_208E8 | | SEQ ID NO:7009 | SEQ ID NO:15021 | SEQ ID NO:23033 |
| | | AA | GYYIH | WINPNSGGTNYAQKFQG | GYSYGYNWFDP |
| | | | SEQ ID NO:7010 | SEQ ID NO:15022 | SEQ ID NO:23034 |
| iPS:436340 | | NA | GTTTCCTACTGGAGC | GAAATCAATCATAGTGG ACGCGCCAACTACAACC CGTCCCTCAAGAGT | GACTACGGTGTCCTTGACTA C |
| | 21-225_208A9 | | SEQ ID NO:7011 | SEQ ID NO:15023 | SEQ ID NO:23035 |

FIGURE 49

| | | AA | VSYWS | EINHSGRANYNPSLKS | DYGVLDY |
|---|---|---|---|---|---|
| | | | SEQ ID NO:7012 | SEQ ID NO:15024 | SEQ ID NO:23036 |
| iPS:436344 | 21-225_208B11 | NA | GGCTACTATATCCAC | TGGATCAACCCTAATAG TGGTGGCACAAACTATG CACAGAAGTTTCAGGGC | GGATACAGCTATGGTTACAA CTGGTTCGACCCC |
| | | | SEQ ID NO:7013 | SEQ ID NO:15025 | SEQ ID NO:23037 |
| | | AA | GYYIH | WINPNSGGTNYAQKFQG | GYSYGYNWFDP |
| | | | SEQ ID NO:7014 | SEQ ID NO:15026 | SEQ ID NO:23038 |
| iPS:436350 | 21-225_210E4 | NA | AACTATGGCATGCAC | GTTATATGGTATGATGA AAATAATAAATACTATG TAGACTCCGTGAAGGGC | GAGACGGGTTTCTTGAGCGA CTAC |
| | | | SEQ ID NO:7015 | SEQ ID NO:15027 | SEQ ID NO:23039 |
| | | AA | NYGMH | VIWYDENNKYYVDSVKG | ETGFLSDY |
| | | | SEQ ID NO:7016 | SEQ ID NO:15028 | SEQ ID NO:23040 |
| iPS:436352 | 21-225_210G5 | NA | GACTGTGTCATGCAC | GTTATATGGTATGATGG AAGTAATAAATACTATG TAGACTCCGTGAAGGGC | GAACGGTATAGCAGTGGCTT GTACGACTACGGTTTGGACG TC |
| | | | SEQ ID NO:7017 | SEQ ID NO:15029 | SEQ ID NO:23041 |
| | | AA | DCVMH | VIWYDGSNKYYVDSVKG | ERYSSGLYDYGLDV |
| | | | SEQ ID NO:7018 | SEQ ID NO:15030 | SEQ ID NO:23042 |
| iPS:436354 | 21-225_210G10 | NA | AGTTACTACTGGAGC | CGTATCTATACCAGTGG GAGCACCGACTACAACC CCTCCCTCAAGAGT | GGGGTTCGGTGACTGGGACTA C |
| | | | SEQ ID NO:7019 | SEQ ID NO:15031 | SEQ ID NO:23043 |
| | | AA | SYYWS | RIYTSGSTDYNPSLKS | GFGDWDY |
| | | | SEQ ID NO:7020 | SEQ ID NO:15032 | SEQ ID NO:23044 |

FIGURE 49

| iPS:436356 | | NA | AGCAACAGTGCTGCTTGGAAC | AGGACATACTACAGGTCCAAGTGGTATAATTATTATCCAGTATCTGTGAGAAGT | GATCAACGGTACTACGGTATGGACGTC |
|---|---|---|---|---|---|
| | 21-225_210H10 | | SEQ ID NO:7021 | SEQ ID NO:15033 | SEQ ID NO:23045 |
| | | AA | SNSAAWN | RTYYRSKWYNYYPVSVRS | DQRYYGMDV |
| | | | SEQ ID NO:7022 | SEQ ID NO:15034 | SEQ ID NO:23046 |
| iPS:436358 | | NA | GGCTACTATATCCAC | TGGATCAACCCTAATAGTGGTGGCACAAACTATGCACAGAAGTTTCAGGGC | GGATACAGCTATGGTTACAACTGGTTCGACCCC |
| | 21-225_210D11 | | SEQ ID NO:7023 | SEQ ID NO:15035 | SEQ ID NO:23047 |
| | | AA | GYYIH | WINPNSGGTNYAQKFQG | GYSYGYNWFDP |
| | | | SEQ ID NO:7024 | SEQ ID NO:15036 | SEQ ID NO:23048 |
| iPS:436360 | | NA | AGCCATGGCATGCAC | GTTACATGGTATGATGGAAGTGATAAATACTATGCAGACTCCGTGAAGGGC | GACCGGCTAGTGGGAGCTACTACCGATGCTTTTGATATC |
| | 21-225_210H11 | | SEQ ID NO:7025 | SEQ ID NO:15037 | SEQ ID NO:23049 |
| | | AA | SHGMH | VTWYDGSDKYYADSVKG | DRLVGATTDAFDI |
| | | | SEQ ID NO:7026 | SEQ ID NO:15038 | SEQ ID NO:23050 |
| iPS:436362 | | NA | AACAATGGTATCAGC | TGGATCAACGCTTACAATGGTCACACAAACTATGCACAGAAGTTCCAGGGC | GATCCTACGGTGACCCACTACTATTACTACGGTATGGACGTC |
| | 21-225_210C12 | | SEQ ID NO:7027 | SEQ ID NO:15039 | SEQ ID NO:23051 |
| | | AA | NNGIS | WINAYNGHTNYAQKFQG | DPTVTHYYYYGMDV |
| | | | SEQ ID NO:7028 | SEQ ID NO:15040 | SEQ ID NO:23052 |

FIGURE 49

| iPS:436364 | | NA | AGCTATGGCATGCAC | GTTCTTTGGTTTGATGGA AGTAATAGAAACTATGC AGACTCCGTGAAGGGC | GATCGGGGAGTGGGCTACTA CGGTACGGACGTC |
|---|---|---|---|---|---|
| | 21-225_211A11 | | SEQ ID NO:7029 | SEQ ID NO:15041 | SEQ ID NO:23053 |
| | | AA | SYGMH | VLWFDGSNRNYADSVKG | DRGVGYYGTDV |
| | | | SEQ ID NO:7030 | SEQ ID NO:15042 | SEQ ID NO:23054 |
| iPS:436366 | | NA | AGTTATGGCATGCAC | GTTATATGGTATGATGG AAGTAATAAAAACTATG AAGACTCCGTGAAGGGC | GATGGGAGTTATGGTTACGA CGGTATGGACGTC |
| | 21-225_211A3 | | SEQ ID NO:7031 | SEQ ID NO:15043 | SEQ ID NO:23055 |
| | | AA | SYGMH | VIWYDGSNKNYEDSVKG | DGSYGYDGMDV |
| | | | SEQ ID NO:7032 | SEQ ID NO:15044 | SEQ ID NO:23056 |
| iPS:436368 | | NA | AACTATGGCATGCAC | GTTATATGGCATGATGG AAGTAATAAATACTATG CAGACTCCGTGAAGGGC | TTAGACTACAGTAACTACGG GTGGTTCGACCCC |
| | 21-225_211G3 | | SEQ ID NO:7033 | SEQ ID NO:15045 | SEQ ID NO:23057 |
| | | AA | NYGMH | VIWHDGSNKYYADSVKG | LDYSNYGWFDP |
| | | | SEQ ID NO:7034 | SEQ ID NO:15046 | SEQ ID NO:23058 |
| iPS:436370 | | NA | AGCTATGGCATGCAC | GTTATATGGTATGATGG AAGTAATAAAAACTATG CAGACTCCGTGAAGGGC | GATAGGACGGTGGGCTATGA TGGTTTTGATATC |
| | 21-225_211A6 | | SEQ ID NO:7035 | SEQ ID NO:15047 | SEQ ID NO:23059 |
| | | AA | SYGMH | VIWYDGSNKNYADSVKG | DRTVGYDGFDI |

FIGURE 49

| | | | SEQ ID NO:7036 | SEQ ID NO:15048 | SEQ ID NO:23060 |
|---|---|---|---|---|---|
| iPS:436372 | | NA | AGCTATGGCATGCAC | GTTATATGGTATGATGG AAGTAATGAACACTATG CAGACTCCGTGAAGGGC | GACCACGGTGTCGGGTACTA CGGTATGGACGTC |
| | 21-225_211A8 | | SEQ ID NO:7037 | SEQ ID NO:15049 | SEQ ID NO:23061 |
| | | AA | SYGMH | VIWYDGSNEHYADSVKG | DHGVGYYGMDV |
| | | | SEQ ID NO:7038 | SEQ ID NO:15050 | SEQ ID NO:23062 |
| iPS:436374 | | NA | AGGCATGGTATCAGC | TGGATCAGCGCTTACAA TGGTCTCACAAACTATGC ACAGAAGTTCCAGGGC | GATCCTACGGTGACCCACTA CTACTACTACGGTATGGACG TC |
| | 21-225_211C10 | | SEQ ID NO:7039 | SEQ ID NO:15051 | SEQ ID NO:23063 |
| | | AA | RHGIS | WISAYNGLTNYAQKFQG | DPTVTHYYYYGMDV |
| | | | SEQ ID NO:7040 | SEQ ID NO:15052 | SEQ ID NO:23064 |
| iPS:436376 | | NA | AGCTATGGCATGCAC | GTTATATGGTATGATGG AAGTAATAAAAACTATG TAGACTCCGTGAAGGGC | GACTACGGTGTCGGGTACTA CGGTACGGACGTC |
| | 21-225_212E6 | | SEQ ID NO:7041 | SEQ ID NO:15053 | SEQ ID NO:23065 |
| | | AA | SYGMH | VIWYDGSNKNYVDSVKG | DYGVGYYGTDV |
| | | | SEQ ID NO:7042 | SEQ ID NO:15054 | SEQ ID NO:23066 |
| iPS:436378 | | NA | AGCTATGGCATGCAC | GTTATATGGTATGATGG AAGTAATAAAAATTATG CAGACTCCGTGAAGGGC | GACTACGGTGTCGGGTACTA CGGTACGGACGTC |
| | 21-225_212D7 | | SEQ ID NO:7043 | SEQ ID NO:15055 | SEQ ID NO:23067 |
| | | AA | SYGMH | VIWYDGSNKNYADSVKG | DYGVGYYGTDV |

FIGURE 49

| | | | SEQ ID NO:7044 | SEQ ID NO:15056 | SEQ ID NO:23068 |
|---|---|---|---|---|---|
| iPS:436380 | | NA | AGCTATGGCATGCAC | GTTATATGGTATGATGG AAGTAATGAACACTATG CAGACTCCGTGAAGGGC | GACCACGGTGTCGGGTACTA CGGTATGGACGTC |
| | 21-225_212H9 | | SEQ ID NO:7045 | SEQ ID NO:15057 | SEQ ID NO:23069 |
| | | AA | SYGMH | VIWYDGSNEHYADSVKG | DHGVGYYGMDV |
| | | | SEQ ID NO:7046 | SEQ ID NO:15058 | SEQ ID NO:23070 |
| iPS:436382 | | NA | AGCTATGGCATGCAC | GCTATATGGTATGATGG AAGTCATAAATACTATA CAGATTCCGTGAAGGGC | GATCGGAGTATAGTGGGAGC TACCTACTTTGACTAC |
| | 21-225_212C10 | | SEQ ID NO:7047 | SEQ ID NO:15059 | SEQ ID NO:23071 |
| | | AA | SYGMH | AIWYDGSHKYYTDSVKG | DRSIVGATYFDY |
| | | | SEQ ID NO:7048 | SEQ ID NO:15060 | SEQ ID NO:23072 |
| iPS:436384 | | NA | AGATATGGCATGCAC | GTTATATGGTATGATGG AAGTAATAAACACTATG CAGACTCCGTGAAGGGC | GATCGGGGAGTGGGCTACAA CGGTATGGACGTC |
| | 21-225_212F10 | | SEQ ID NO:7049 | SEQ ID NO:15061 | SEQ ID NO:23073 |
| | | AA | RYGMH | VIWYDGSNKHYADSVKG | DRGVGYNGMDV |
| | | | SEQ ID NO:7050 | SEQ ID NO:15062 | SEQ ID NO:23074 |
| iPS:436386 | | NA | GACTATGTCATGCAC | GTTATATGGTATGATGG AAGTAATAAATATTATG CAGACTCCGTGAAGGGC | GAACGTTATAGCAGTGGCTG GTACGACTACGGTATGGACG TC |
| | 21-225_212B11 | | SEQ ID NO:7051 | SEQ ID NO:15063 | SEQ ID NO:23075 |

FIGURE 49

| | | AA | DYVMH | VIWYDGSNKYYADSVKG | ERYSSGWYDYGMDV |
|---|---|---|---|---|---|
| | | | SEQ ID NO:7052 | SEQ ID NO:15064 | SEQ ID NO:23076 |
| iPS:436388 | 21-225_212H11 | NA | AGTTATGGCATGCAC | GTTATATGGTATGATGG AAGTAATAAAAACTATG AAGACTCCGTGAAGGGC | GATGGGAGTTATGGTTACGA CGGTATGGACGTC |
| | | | SEQ ID NO:7053 | SEQ ID NO:15065 | SEQ ID NO:23077 |
| | | AA | SYGMH | VIWYDGSNKNYEDSVKG | DGSYGYDGMDV |
| | | | SEQ ID NO:7054 | SEQ ID NO:15066 | SEQ ID NO:23078 |
| iPS:436390 | 21-225_213D2 | NA | AGCTATGGCATGCAC | GTTATATGGTATGATGG AAGTAATAAAAATTATG CAGACTCCGTGAAGGGC | GACTACGGTGTCGGGTACTA CGGTACGGACGTC |
| | | | SEQ ID NO:7055 | SEQ ID NO:15067 | SEQ ID NO:23079 |
| | | AA | SYGMH | VIWYDGSNKNYADSVKG | DYGVGYYGTDV |
| | | | SEQ ID NO:7056 | SEQ ID NO:15068 | SEQ ID NO:23080 |
| iPS:436392 | 21-225_213B3 | NA | AGCTATGGCATGCAC | GTTATATGGTATGATGG AAGTAATAAAAACTATG CAGACTCCGTGAAGGGC | GATAGGACGGTGGGCTATGA TGGTTTTGATATC |
| | | | SEQ ID NO:7057 | SEQ ID NO:15069 | SEQ ID NO:23081 |
| | | AA | SYGMH | VIWYDGSNKNYADSVKG | DRTVGYDGFDI |
| | | | SEQ ID NO:7058 | SEQ ID NO:15070 | SEQ ID NO:23082 |

FIGURE 49

| iPS:436394 | 21-225_213C4 | NA | AGCTATGGCATGCAC | GTTATATGGTATGATGG AAGTAATAAACACTATG CAGACTCCGTGAAGGGC | GACTACGGTGTCGGGTACGA CGGTATGGACGTC |
|---|---|---|---|---|---|
| | | | SEQ ID NO:7059 | SEQ ID NO:15071 | SEQ ID NO:23083 |
| | | AA | SYGMH | VIWYDGSNKHYADSVKG | DYGVGYDGMDV |
| | | | SEQ ID NO:7060 | SEQ ID NO:15072 | SEQ ID NO:23084 |
| iPS:436396 | 21-225_213E5 | NA | AGTTATGGCATGCAC | GTTATATGGTATGATGG AAGTAATAAAAACTATG AAGACTCCGTGAAGGGC | GATGGGAGTTATGGTTACGA CGGTATGGACGTC |
| | | | SEQ ID NO:7061 | SEQ ID NO:15073 | SEQ ID NO:23085 |
| | | AA | SYGMH | VIWYDGSNKNYEDSVKG | DGSYGYDGMDV |
| | | | SEQ ID NO:7062 | SEQ ID NO:15074 | SEQ ID NO:23086 |
| iPS:436398 | 21-225_213B8 | NA | AGCTATGGCATGCAC | GTTATATGGTATGATGG AAGTAATAAACACTATG CAGACTCCGTGAAGGGC | GACTACGGTGTCGGGTACTA CGGTACGGACGTC |
| | | | SEQ ID NO:7063 | SEQ ID NO:15075 | SEQ ID NO:23087 |
| | | AA | SYGMH | VIWYDGSNKHYADSVKG | DYGVGYYGTDV |
| | | | SEQ ID NO:7064 | SEQ ID NO:15076 | SEQ ID NO:23088 |
| iPS:436400 | 21-225_213H7 | NA | GGCTACCATATGCAC | TGGATCAATCCTAAGAG TGATGGCACAAACTATG CACAGAAGTTTCAGGGC | GAAAAGCCTGGGAGCTACTA CAAATAC |
| | | | SEQ ID NO:7065 | SEQ ID NO:15077 | SEQ ID NO:23089 |
| | | AA | GYHMH | WINPKSDGTNYAQKFQG | EKPGSYYKY |
| | | | SEQ ID NO:7066 | SEQ ID NO:15078 | SEQ ID NO:23090 |

FIGURE 49

| iPS:436402 | | NA | AGCTATGGTATCAAC | TGGATCAGCGTTCACAA TGGTAACACAGACTATG CACAGAAGTTCCAGGGC | GACTACTACTACGGTATGGA CGTC |
|---|---|---|---|---|---|
| | 21-225_213H12 | | SEQ ID NO:7067 | SEQ ID NO:15079 | SEQ ID NO:23091 |
| | | AA | SYGIN | WISVHNGNTDYAQKFQG | DYYYGMDV |
| | | | SEQ ID NO:7068 | SEQ ID NO:15080 | SEQ ID NO:23092 |
| iPS:436404 | | NA | AGCTATGGCATGCAC | GTTATATGGTATGATGG AAGTAATAAAAACTATG GAGACTCCGTGAAGGGC | GATCGGGGAGTGGGCTACGA CGGAATGGACGTC |
| | 21-225_214C3 | | SEQ ID NO:7069 | SEQ ID NO:15081 | SEQ ID NO:23093 |
| | | AA | SYGMH | VIWYDGSNKNYGDSVKG | DRGVGYDGMDV |
| | | | SEQ ID NO:7070 | SEQ ID NO:15082 | SEQ ID NO:23094 |
| iPS:436406 | | NA | AGCTATGGCATGCAC | GTTATATGGTATGATGG AAGTAATGAAAACTATG CAGACTCCGTGAAGGGC | GATAGGACGGTGGGCTATGA TGGTTGTGATATC |
| | 21-225_214E4 | | SEQ ID NO:7071 | SEQ ID NO:15083 | SEQ ID NO:23095 |
| | | AA | SYGMH | VIWYDGSNENYADSVKG | DRTVGYDGCDI |
| | | | SEQ ID NO:7072 | SEQ ID NO:15084 | SEQ ID NO:23096 |
| iPS:436408 | | NA | GGCCACTATATACAC | TGGATCAACTCTAACAG TGGTGGCACAAACTATG CACAGAAGTTTCAGGGC | GACGGGAGATACAGCTATGG TTACGACTGGTTCGACCCC |
| | 21-225_214H8 | | SEQ ID NO:7073 | SEQ ID NO:15085 | SEQ ID NO:23097 |
| | | AA | GHYIH | WINSNSGGTNYAQKFQG | DGRYSYGYDWFDP |
| | | | SEQ ID NO:7074 | SEQ ID NO:15086 | SEQ ID NO:23098 |

FIGURE 49

| iPS:436410 | | NA | AGCTATGGCATGCAC | GTTATATGGTATGATGG AAGTAATAAATACTATG CAGACTCCGTGAAGGGC | GACTACGGTGTCGGGTACTA CGGTACGGACGTC |
|---|---|---|---|---|---|
| | 21-225_212E10 | | SEQ ID NO:7075 | SEQ ID NO:15087 | SEQ ID NO:23099 |
| | | AA | SYGMH | VIWYDGSNKYYADSVKG | DYGVGYYGTDV |
| | | | SEQ ID NO:7076 | SEQ ID NO:15088 | SEQ ID NO:23100 |
| iPS:436412 | | NA | AGCTATGTCATGCAC | GTTATATGGTATGATGG AAGTAATAAATACTATG CAGACTCCGTGAAGGGC | GAGAGGTATACCAGCAGCTG GTACGACTACGGTATGGACG TC |
| | 21-225_214H9 | | SEQ ID NO:7077 | SEQ ID NO:15089 | SEQ ID NO:23101 |
| | | AA | SYVMH | VIWYDGSNKYYADSVKG | ERYTSSWYDYGMDV |
| | | | SEQ ID NO:7078 | SEQ ID NO:15090 | SEQ ID NO:23102 |
| iPS:436414 | | NA | GACTATGTCATGCAC | GTTATATGGTATGATGG AAGTAATAAATATTATG CAGACTCCGTGAAGGGC | GAACGTTATAGCAGTGGCTG GTACGACTACGGTATGGACG TC |
| | 21-225_214G10 | | SEQ ID NO:7079 | SEQ ID NO:15091 | SEQ ID NO:23103 |
| | | AA | DYVMH | VIWYDGSNKYYADSVKG | ERYSSGWYDYGMDV |
| | | | SEQ ID NO:7080 | SEQ ID NO:15092 | SEQ ID NO:23104 |
| iPS:436416 | | NA | GACTATGTCATGCAC | GTTATATGGTATGATGG AAGTAATAAATATTATG CAGACTCCGTGAAGGGC | GAACGTTATAGCAGTGGCTG GTACGACTACGGTATGGACG TC |
| | 21-225_214G12 | | SEQ ID NO:7081 | SEQ ID NO:15093 | SEQ ID NO:23105 |
| | | AA | DYVMH | VIWYDGSNKYYADSVKG | ERYSSGWYDYGMDV |
| | | | SEQ ID NO:7082 | SEQ ID NO:15094 | SEQ ID NO:23106 |

FIGURE 49

| iPS:436418 | | NA | GACTATGTCATACAC | GTTATATGGTATGATGG AAGTAATAAATATTATG CAGACTCCGTGAAGGGC | GAACGTTATAGCAGTGGCTG GTACGACTACGGTATGGACG TC |
|---|---|---|---|---|---|
| | 21-225_215E3 | | SEQ ID NO:7083 | SEQ ID NO:15095 | SEQ ID NO:23107 |
| | | AA | DYVIH | VIWYDGSNKYYADSVKG | ERYSSGWYDYGMDV |
| | | | SEQ ID NO:7084 | SEQ ID NO:15096 | SEQ ID NO:23108 |
| iPS:436420 | | NA | AGCTATGGCATGCAC | GTTATATGGTATGATGG AAGTAATAAAAATTATG CAGACTCCGTGAAGGGC | GACTACGGTGTCGGGTACTA CGGTACGGACGTC |
| | 21-225_215B5 | | SEQ ID NO:7085 | SEQ ID NO:15097 | SEQ ID NO:23109 |
| | | AA | SYGMH | VIWYDGSNKNYADSVKG | DYGVGYYGTDV |
| | | | SEQ ID NO:7086 | SEQ ID NO:15098 | SEQ ID NO:23110 |
| iPS:436422 | | NA | AGCTATGGCATGCAC | GTTATATGGTATGATGG AAGTAATAAAAACTATG CAGATTCCGTGAAGGGC | GACTGCGGTGTCGGATACTA CGGTACGGACGTC |
| | 21-225_215D6 | | SEQ ID NO:7087 | SEQ ID NO:15099 | SEQ ID NO:23111 |
| | | AA | SYGMH | VIWYDGSNKNYADSVKG | DCGVGYYGTDV |
| | | | SEQ ID NO:7088 | SEQ ID NO:15100 | SEQ ID NO:23112 |
| iPS:436424 | | NA | GGCCACTATATACAC | TGGATCAACTCTAACAG TGGTGGCACAAATTATG CAGAGAAGTTTCAGGGC | GACGGGAGATACAGCTATGG TCACGACTGGTTCGACCCC |
| | 21-225_215H6 | | SEQ ID NO:7089 | SEQ ID NO:15101 | SEQ ID NO:23113 |
| | | AA | GHYIH | WINSNSGGTNYAEKFQG | DGRYSYGHDWFDP |
| | | | SEQ ID NO:7090 | SEQ ID NO:15102 | SEQ ID NO:23114 |

FIGURE 49

| iPS:436426 | | NA | TACTATGGCATGCAC | GTTATATGGCATGATGG AAGTAATAAATACTATG CAGACTCCGTGAAGGGC | TTAGACTACAGTAATTACGG GTGGTTCGACCCC |
|---|---|---|---|---|---|
| | 21-225_215C7 | | SEQ ID NO:7091 | SEQ ID NO:15103 | SEQ ID NO:23115 |
| | | AA | YYGMH | VIWHDGSNKYYADSVKG | LDYSNYGWFDP |
| | | | SEQ ID NO:7092 | SEQ ID NO:15104 | SEQ ID NO:23116 |
| iPS:436428 | | NA | GACTATGTCATGCAC | GTTATATGGTATGATGG AAGTAATAAATATTATG CAGACTCCGTGAAGGGC | GAACGTTATAGCAGTGGCTG GTACGACTACGGTATGGACG TC |
| | 21-225_215E11 | | SEQ ID NO:7093 | SEQ ID NO:15105 | SEQ ID NO:23117 |
| | | AA | DYVMH | VIWYDGSNKYYADSVKG | ERYSSGWYDYGMDV |
| | | | SEQ ID NO:7094 | SEQ ID NO:15106 | SEQ ID NO:23118 |
| iPS:436430 | | NA | AGCTATGGCATGCAC | GTTATATGGTATGATGG AAGTAATGAACACTATG CAGACTCCGTGAAGGGC | GATCGGGGAGTGGGCTACTA CGGTATGGACGTC |
| | 21-225_215A12 | | SEQ ID NO:7095 | SEQ ID NO:15107 | SEQ ID NO:23119 |
| | | AA | SYGMH | VIWYDGSNEHYADSVKG | DRGVGYYGMDV |
| | | | SEQ ID NO:7096 | SEQ ID NO:15108 | SEQ ID NO:23120 |
| iPS:436432 | | NA | AACTATGGCATGCAC | GTTATATGGCATGATGG AAGTAATAAATACTATG CAGACTCCGTGAAGGGC | TTAGACTACAGTAACTACGG GTGGTTCGACCCC |
| | 21-225_215H12 | | SEQ ID NO:7097 | SEQ ID NO:15109 | SEQ ID NO:23121 |
| | | AA | NYGMH | VIWHDGSNKYYADSVKG | LDYSNYGWFDP |

FIGURE 49

| | | | SEQ ID NO:7098 | SEQ ID NO:15110 | SEQ ID NO:23122 |
|---|---|---|---|---|---|
| iPS:436434 | | NA | AGCTATGGCATGCAC | GCTATATGGTATGATGG AAGTAATAAATACTATG CAGACTCCGTGAAGGGC | GATCCCAACATAGTGGGAGC TACTTGGTTTGACTAC |
| | 21-225_216B10 | | SEQ ID NO:7099 | SEQ ID NO:15111 | SEQ ID NO:23123 |
| | | AA | SYGMH | AIWYDGSNKYYADSVKG | DPNIVGATWFDY |
| | | | SEQ ID NO:7100 | SEQ ID NO:15112 | SEQ ID NO:23124 |
| iPS:436436 | | NA | AGCTATAGCATGAAC | TACATTACTGGTAGTAGT AGTACCATATACTACGC AGACTCTGTGAAGGGC | TCGGGTTTAGCAGTGGAGGA CTAC |
| | 21-225_216F10 | | SEQ ID NO:7101 | SEQ ID NO:15113 | SEQ ID NO:23125 |
| | | AA | SYSMN | YITGSSSTIYYADSVKG | SGLAVEDY |
| | | | SEQ ID NO:7102 | SEQ ID NO:15114 | SEQ ID NO:23126 |
| iPS:436438 | | NA | GACTATGTCATGCAC | GTTATATGGTATGATGG AAGTAATAAATATTATG CAGACTCCGTGAAGGGC | GAACGTTATAGCAGTGGCTG GTACGACTACGGTATGGACG TC |
| | 21-225_216E8 | | SEQ ID NO:7103 | SEQ ID NO:15115 | SEQ ID NO:23127 |
| | | AA | DYVMH | VIWYDGSNKYYADSVKG | ERYSSGWYDYGMDV |
| | | | SEQ ID NO:7104 | SEQ ID NO:15116 | SEQ ID NO:23128 |
| iPS:436440 | | NA | GACTATGTCATGCAC | GTTATATGGTATGATGG AAGTAATAAATATTATG CAGACTCCGTGAAGGGC | GAACGTTATAGCAGTGGCTG GTACGACTACGGTATGGACG TC |
| | 21-225_216H12 | | SEQ ID NO:7105 | SEQ ID NO:15117 | SEQ ID NO:23129 |
| | | AA | DYVMH | VIWYDGSNKYYADSVKG | ERYSSGWYDYGMDV |
| | | | SEQ ID NO:7106 | SEQ ID NO:15118 | SEQ ID NO:23130 |

FIGURE 49

| iPS:436448 | 21-225_217A3 | NA | AGCTATAATATGAAC | TACATTAGTAGTAGTCGT AATATCATATATTACGCA GACTCTGTGAAGGGC | GATGGCTCTTATAGCAGTGG CTGGTACTGGGGTTTTGACT AC |
|---|---|---|---|---|---|
| | | | SEQ ID NO:7107 | SEQ ID NO:15119 | SEQ ID NO:23131 |
| | | AA | SYNMN | YISSSRNIIYYADSVKG | DGSYSSGWYWGFDY |
| | | | SEQ ID NO:7108 | SEQ ID NO:15120 | SEQ ID NO:23132 |
| iPS:436450 | 21-225_217E5 | NA | GACTATGTCATGCAC | GTTATATGGTATGATGG AAGTAATAAATATTATG CAGACTCCGTGAAGGGC | GAACGTTATAGCAGTGGCTG GTACGACTACGGTATGGACG TC |
| | | | SEQ ID NO:7109 | SEQ ID NO:15121 | SEQ ID NO:23133 |
| | | AA | DYVMH | VIWYDGSNKYYADSVKG | ERYSSGWYDYGMDV |
| | | | SEQ ID NO:7110 | SEQ ID NO:15122 | SEQ ID NO:23134 |
| iPS:436452 | 21-225_217G5 | NA | AGCAATGGCATGCAC | GTTATATGGTATGATGG AAGTAATAAAAACTATG CAGACTCCGTGAAGGGC | GACTACGGTGTCGGGTACTA CGGTCTGGACGTC |
| | | | SEQ ID NO:7111 | SEQ ID NO:15123 | SEQ ID NO:23135 |
| | | AA | SNGMH | VIWYDGSNKNYADSVKG | DYGVGYYGLDV |
| | | | SEQ ID NO:7112 | SEQ ID NO:15124 | SEQ ID NO:23136 |
| iPS:436454 | 21-225_217B10 | NA | AGTTATGGCATGCAC | GTTATATGGTATGATGG AAGTAATAAAAACTATG AAGACTCCGTGAAGGGC | GATGGGAGTTATGGTTACGA CGGTATGGACGTC |
| | | | SEQ ID NO:7113 | SEQ ID NO:15125 | SEQ ID NO:23137 |
| | | AA | SYGMH | VIWYDGSNKNYEDSVKG | DGSYGYDGMDV |
| | | | SEQ ID NO:7114 | SEQ ID NO:15126 | SEQ ID NO:23138 |

FIGURE 49

| iPS:436456 | | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | 21-225_217G10 | | SEQ ID NO:7115 | SEQ ID NO:15127 | SEQ ID NO:23139 |
| | | AA | DYVMH | VIWYDGSNKYYADSVKG | ERYSSGWYDYGMDV |
| | | | SEQ ID NO:7116 | SEQ ID NO:15128 | SEQ ID NO:23140 |
| iPS:436458 | | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | 21-225_217H12 | | SEQ ID NO:7117 | SEQ ID NO:15129 | SEQ ID NO:23141 |
| | | AA | DYVMH | VIWYDGSNKYYADSVKG | ERYSSGWYDYGMDV |
| | | | SEQ ID NO:7118 | SEQ ID NO:15130 | SEQ ID NO:23142 |
| iPS:436462 | | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | 21-225_218C4 | | SEQ ID NO:7119 | SEQ ID NO:15131 | SEQ ID NO:23143 |
| | | AA | DYVMH | VIWYDGSNKYYADSVKG | ERYSSGWYDYGMDV |
| | | | SEQ ID NO:7120 | SEQ ID NO:15132 | SEQ ID NO:23144 |
| iPS:436464 | | NA | AGATATGGCATGCAC | GTTATATGGTATGATGGAAGTAATAAACACTATGCAGACTCCGTGAAGGGC | GATCGGGGAGTGGGCTACAACGGTATGGACGTC |
| | 21-225_219H1 | | SEQ ID NO:7121 | SEQ ID NO:15133 | SEQ ID NO:23145 |
| | | AA | RYGMH | VIWYDGSNKHYADSVKG | DRGVGYNGMDV |
| | | | SEQ ID NO:7122 | SEQ ID NO:15134 | SEQ ID NO:23146 |

FIGURE 49

| iPS:436472 | | NA | ACTTACTACTGGAGC | TATATCTATTACAGTGGG ACCACCAACTACAACCC CTCCCTCAAGAGT | GACCAGCAGTGGCTGGTACG TGGGAGGGACAACTACTACT ACGGTATGGACGTC |
| | 21-225_220E1 | | SEQ ID NO:7123 | SEQ ID NO:15135 | SEQ ID NO:23147 |
| | | AA | TYYWS | YIYYSGTTNYNPSLKS | DQQWLVRGRDNYYYGMDV |
| | | | SEQ ID NO:7124 | SEQ ID NO:15136 | SEQ ID NO:23148 |
| iPS:436480 | | NA | GACTATGTCATGCAC | GTTATATGGTATGATGG AAGTAATAAATATTATG CAGACTCCGTGAAGGGC | GAACGTTATAGCAGTGGCTG GTACGACTACGGTATGGACG TC |
| | 21-225_220F8 | | SEQ ID NO:7125 | SEQ ID NO:15137 | SEQ ID NO:23149 |
| | | AA | DYVMH | VIWYDGSNKYYADSVKG | ERYSSGWYDYGMDV |
| | | | SEQ ID NO:7126 | SEQ ID NO:15138 | SEQ ID NO:23150 |
| iPS:436488 | | NA | GGCTACTATATGCAC | TGGATCCACCCTAACAG TGGTGGCACAAACTATG CACAGAAATTTCAGGGC | GATGGGACCAGCTCGTTTGA CTAC |
| | 21-225_221A6 | | SEQ ID NO:7127 | SEQ ID NO:15139 | SEQ ID NO:23151 |
| | | AA | GYYMH | WIHPNSGGTNYAQKFQG | DGTSSFDY |
| | | | SEQ ID NO:7128 | SEQ ID NO:15140 | SEQ ID NO:23152 |
| iPS:436490 | | NA | AGCAATGGCATGCAC | GTTATATGGTACGATGG AAGTAATGAAAACTATG CAGACTCCGTGAAGGGC | GATCGGACAGTGGGCTACAA CGGTATGGACGTC |
| | 21-225_221F6 | | SEQ ID NO:7129 | SEQ ID NO:15141 | SEQ ID NO:23153 |
| | | AA | SNGMH | VIWYDGSNENYADSVKG | DRTVGYNGMDV |
| | | | SEQ ID NO:7130 | SEQ ID NO:15142 | SEQ ID NO:23154 |
| iPS:436496 | 21 225 222E1 | NA | GGCTACTATATGCAC | TGGATCCACCCTAACAG TGGTGGCACAAACTATG CACAGAAATTTCAGGGC | GATGGGACCAGCTCGTTTGA CTAC |

FIGURE 49

| | | | SEQ ID NO:7131 | SEQ ID NO:15143 | SEQ ID NO:23155 |
|---|---|---|---|---|---|
| | 21-225_222E1 | AA | GYYMH | WIHPNSGGTNYAQKFQG | DGTSSFDY |
| iPS:436500 | | NA | SEQ ID NO:7132<br>AGCTATGGTATCAAC | SEQ ID NO:15144<br>TGGATCAGCGTTTACAATGGTAACACAAACTATGCACAGAAGCTCCAGGGC | SEQ ID NO:23156<br>GACTACTACTACGGTTTTGACGTC |
| | 21-225_222H3 | AA | SEQ ID NO:7133<br>SYGIN | SEQ ID NO:15145<br>WISVYNGNTNYAQKLQG | SEQ ID NO:23157<br>DYYYGFDV |
| iPS:436502 | | NA | SEQ ID NO:7134<br>AGCTATGGCATGCAC | SEQ ID NO:15146<br>GTTATATGGTATGATGGAAGTAATAAAAACTATGCAGACTCCGTGAAGGGC | SEQ ID NO:23158<br>GATCGGGATGTCGGGTACAACGGTATGGACGTC |
| | 21-225_222A11 | AA | SEQ ID NO:7135<br>SYGMH | SEQ ID NO:15147<br>VIWYDGSNKNYADSVKG | SEQ ID NO:23159<br>DRDVGYNGMDV |
| iPS:436504 | | NA | SEQ ID NO:7136<br>AACTTTGCCATGAGT | SEQ ID NO:15148<br>AGTATTGTTGGTAGTGGTGGTCGCACGTACTACGCAGACTCCGTGAAGGGC | SEQ ID NO:23160<br>GACCCTTATCGTGTAGCAGTGGCTGGGGCCTTTGACTAC |
| | 21-225_222H4 | AA | SEQ ID NO:7137<br>NFAMS | SEQ ID NO:15149<br>SIVGSGGRTYYADSVKG | SEQ ID NO:23161<br>DPYRVAVAGAFDY |
| iPS:436506 | | NA | SEQ ID NO:7138<br>GGTCGCTACTGGAGC | SEQ ID NO:15150<br>GAAATCAATCATAGTGGAAGCGCCAACTACAACCCGTCCCTCAAGAGT | SEQ ID NO:23162<br>GACTACGGCGCCCTTGATTTC |
| | 21-225_222C7 | AA | SEQ ID NO:7139<br>GRYWS | SEQ ID NO:15151<br>EINHSGSANYNPSLKS | SEQ ID NO:23163<br>DYGALDF |

FIGURE 49

| | | | SEQ ID NO:7140 | SEQ ID NO:15152 | SEQ ID NO:23164 |
|---|---|---|---|---|---|
| iPS:436508 | 21-225_222F7 | NA | GGCTACTATATGCAC | TGGATCCACCCTAACAG TGGTGGCACAAACTATG CACAGAAATTTCAGGGC | GATGGGACCAGCTCGTTTGA CTAC |
| | | | SEQ ID NO:7141 | SEQ ID NO:15153 | SEQ ID NO:23165 |
| | | AA | GYYMH | WIHPNSGGTNYAQKFQG | DGTSSFDY |
| | | | SEQ ID NO:7142 | SEQ ID NO:15154 | SEQ ID NO:23166 |
| iPS:436510 | 21-225_222H8 | NA | AACTTTGCCATGAGT | AGTATTGTTGGTAGTGGT GGTCGCACGTACTACGC AGACTCCGTGAAGGGC | GACCCTTATCGTGTAGCAGT GGCTGGGGCCTTTGACTAC |
| | | | SEQ ID NO:7143 | SEQ ID NO:15155 | SEQ ID NO:23167 |
| | | AA | NFAMS | SIVGSGGRTYYADSVKG | DPYRVAVAGAFDY |
| | | | SEQ ID NO:7144 | SEQ ID NO:15156 | SEQ ID NO:23168 |
| iPS:436514 | 21-225_222D10 | NA | AGCTATGGCATGCAC | GTTATATGGTATGATGG AAGTAATAAAAACTATG CAGACTCCGTGAAGGGC | GATCGGGATGTCGGGTACAA CGGTATGGACGTC |
| | | | SEQ ID NO:7145 | SEQ ID NO:15157 | SEQ ID NO:23169 |
| | | AA | SYGMH | VIWYDGSNKNYADSVKG | DRDVGYNGMDV |
| | | | SEQ ID NO:7146 | SEQ ID NO:15158 | SEQ ID NO:23170 |
| iPS:436516 | 21-225_222C12 | NA | GGCTACTATATGCAC | TGGATCCACCCTAACAG TGGTGGCACAAACTATG CACAGAAATTTCAGGGC | GATGGGACCAGCTCGTTTGA CTAC |
| | | | SEQ ID NO:7147 | SEQ ID NO:15159 | SEQ ID NO:23171 |
| | | AA | GYYMH | WIHPNSGGTNYAQKFQG | DGTSSFDY |
| | | | SEQ ID NO:7148 | SEQ ID NO:15160 | SEQ ID NO:23172 |

FIGURE 49

| iPS:436520 | | NA | AGCTATGGTATCAAC | TGGATCAGCGTTTACAGT GGTAACACAAACTATGC ACAGAAGCTCCAGGGC | GACTACTACTACGGTATGGA CGTC |
| | 21-225_223G10 | | SEQ ID NO:7149 | SEQ ID NO:15161 | SEQ ID NO:23173 |
| | | AA | SYGIN | WISVYSGNTNYAQKLQG | DYYYGMDV |
| | | | SEQ ID NO:7150 | SEQ ID NO:15162 | SEQ ID NO:23174 |
| iPS:436522 | | NA | AGCTATGGCATGCAC | GTTATATGGTATGATGG AAGTAATAAAAACTATG CAGACTCCGTGAAGGGC | GATCGGGATGTCGGGTACAA CGGTATGGACGTC |
| | 21-225_223H10 | | SEQ ID NO:7151 | SEQ ID NO:15163 | SEQ ID NO:23175 |
| | | AA | SYGMH | VIWYDGSNKNYADSVKG | DRDVGYNGMDV |
| | | | SEQ ID NO:7152 | SEQ ID NO:15164 | SEQ ID NO:23176 |
| iPS:436526 | | NA | AGCTATGCCATGAGC | GCTATTAGTGGCAGAGG CGGCAGCACATACTACG CAGACGCCGTGAAGGGC | GGCTCCTACGATAGTAGTGG TTATTACCACTACTTAGACC AC |
| | 21-225_224A1 | | SEQ ID NO:7153 | SEQ ID NO:15165 | SEQ ID NO:23177 |
| | | AA | SYAMS | AISGRGGSTYYADAVKG | GSYDSSGYYHYLDH |
| | | | SEQ ID NO:7154 | SEQ ID NO:15166 | SEQ ID NO:23178 |
| iPS:436528 | | NA | AGCTATGCCATGAGC | GCTATTAGTGGTAGTGGT GGTAACACATACTACGC AGACTCCGTGAAGGGC | GAGGGGGGGCTACTACTATTA CTACGGTGTGGACGTC |
| | 21-225_224B1 | | SEQ ID NO:7155 | SEQ ID NO:15167 | SEQ ID NO:23179 |
| | | AA | SYAMS | AISGSGGNTYYADSVKG | EGGYYYYYGVDV |
| | | | SEQ ID NO:7156 | SEQ ID NO:15168 | SEQ ID NO:23180 |

FIGURE 49

| iPS:436534 | | NA | AGCTATATCATGCAC | GTTATATGGTATGATGG AAGTAATAAATACTATG CAGACTCCGTGAAGGGC | GAGAGGTATAGCAGCAACTG GTACGACTACGGTATGGACG TC |
|---|---|---|---|---|---|
| | 21-225_224F1 | | SEQ ID NO:7157 | SEQ ID NO:15169 | SEQ ID NO:23181 |
| | | AA | SYIMH | VIWYDGSNKYYADSVKG | ERYSSNWYDYGMDV |
| | | | SEQ ID NO:7158 | SEQ ID NO:15170 | SEQ ID NO:23182 |
| iPS:436536 | | NA | GGCTACTATATACAC | TGGATCAACCCTTACAGT GGTGACACAAACTATGC ACAGAAGTTTCAGGGC | GATTGGGGTGGCTACAGTTC TTACTACTACGGTATGGACG TC |
| | 21-225_224G1 | | SEQ ID NO:7159 | SEQ ID NO:15171 | SEQ ID NO:23183 |
| | | AA | GYYIH | WINPYSGDTNYAQKFQG | DWGGYSSYYYGMDV |
| | | | SEQ ID NO:7160 | SEQ ID NO:15172 | SEQ ID NO:23184 |
| iPS:436538 | | NA | AGAAGTAGTTACTACTGGGG C | AATATCTATTATAGTGGG AGCACCTACTACAACCC GTCCCTCAAGAGT | CAGGGTCGGGACTGGGGTGT TGACTAC |
| | 21-225_224C3 | | SEQ ID NO:7161 | SEQ ID NO:15173 | SEQ ID NO:23185 |
| | | AA | RSSYYWG | NIYYSGSTYYNPSLKS | QGRDWGVDY |
| | | | SEQ ID NO:7162 | SEQ ID NO:15174 | SEQ ID NO:23186 |
| iPS:436540 | | NA | AGCTATGGCATGCAC | GTTATATGGTATGATGG AAGTAATAAATACTATG CAGACTCCGTGAAGGGC | GAGAGGTATAGCAGCAGCTG GTACGACTACGGTATGGACG TC |
| | 21-225_224F3 | | SEQ ID NO:7163 | SEQ ID NO:15175 | SEQ ID NO:23187 |
| | | AA | SYGMH | VIWYDGSNKYYADSVKG | ERYSSSWYDYGMDV |
| | | | SEQ ID NO:7164 | SEQ ID NO:15176 | SEQ ID NO:23188 |

FIGURE 49

| iPS:436544 | | NA | AATTATGATATCAAC | TGGATGAACCCTAACAG TGGTAACACAGGCTATG CACAGAAGTTCCAGGGC | TCCAGTGGCTGGAACTGGTT CGACCCC |
|---|---|---|---|---|---|
| | **21-225_224H5** | | SEQ ID NO:7165 | SEQ ID NO:15177 | SEQ ID NO:23189 |
| | | AA | NYDIN | WMNPNSGNTGYAQKFQG | SSGWNWFDP |
| | | | SEQ ID NO:7166 | SEQ ID NO:15178 | SEQ ID NO:23190 |
| iPS:436546 | | NA | AGCGATGCCATGAGC | GCTATTAGTGGTAGTGGT GATAACACATTCTACGC AGACTCCGTGAAGGGC | GTCTATAGTGCCTACGATTC TCACTGGTTCGACCCC |
| | **21-225_224D6** | | SEQ ID NO:7167 | SEQ ID NO:15179 | SEQ ID NO:23191 |
| | | AA | SDAMS | AISGSGDNTFYADSVKG | VYSAYDSHWFDP |
| | | | SEQ ID NO:7168 | SEQ ID NO:15180 | SEQ ID NO:23192 |
| iPS:436548 | | NA | GGCTACTATATACAC | TGGATCAACCCTTACAGT GGTGACACAAACTATGC ACAGAAGTTTCAGGGC | GATTGGGGGTGGCTACAGTTC TTACTACTACGGTATGGACG TC |
| | **21-225_224A7** | | SEQ ID NO:7169 | SEQ ID NO:15181 | SEQ ID NO:23193 |
| | | AA | GYYIH | WINPYSGDTNYAQKFQG | DWGGYSSYYYGMDV |
| | | | SEQ ID NO:7170 | SEQ ID NO:15182 | SEQ ID NO:23194 |
| iPS:436550 | | NA | AATTATGATATCAAC | TGGTTGTACCCTAACAGT GGTAACACAGGCTATGC ACAGAAGTTCCAGGGC | AGCAGTGGCTGGTACTACTT TGACTAC |
| | **21-225_224D8** | | SEQ ID NO:7171 | SEQ ID NO:15183 | SEQ ID NO:23195 |
| | | AA | NYDIN | WLYPNSGNTGYAQKFQG | SSGWYYFDY |
| | | | SEQ ID NO:7172 | SEQ ID NO:15184 | SEQ ID NO:23196 |
| iPS:436554 | | NA | AATTATGATATCAAC | TGGATGCACCCTAACAG TGGTAACACAGGCTATG CACAGAAGTTCCAGGGC | AGCAGTGGCTGGTACAAGTT TGACTAC |
| | **21-225_224C10** | | SEQ ID NO:7173 | SEQ ID NO:15185 | SEQ ID NO:23197 |

FIGURE 49

| | | | | | |
|---|---|---|---|---|---|
| | | AA | NYDIN | WMHPNSGNTGYAQKFQG | SSGWYKFDY |
| | | | SEQ ID NO:7174 | SEQ ID NO:15186 | SEQ ID NO:23198 |
| iPS:436556 | 21-225_224D10 | NA | AGCTATGGCATGCAC | GTTATATGGTATGAGGG AAGTAATAAATACTATG TAGACTCCGTGAGGGGC | GAGCTAGGCTTCCAGTCTGA CTAC |
| | | | SEQ ID NO:7175 | SEQ ID NO:15187 | SEQ ID NO:23199 |
| | | AA | SYGMH | VIWYEGSNKYYVDSVRG | ELGFQSDY |
| | | | SEQ ID NO:7176 | SEQ ID NO:15188 | SEQ ID NO:23200 |
| iPS:436558 | 21-225_224C11 | NA | GGCTACTATATACAC | TGGATCAACCCTTACAGT GGTGACACAAACTATGC ACAGAAGTTTCAGGGC | GATTGGGGTGGCTACAGTTC TTACTACTTCGGTATGGACG TC |
| | | | SEQ ID NO:7177 | SEQ ID NO:15189 | SEQ ID NO:23201 |
| | | AA | GYYIH | WINPYSGDTNYAQKFQG | DWGGYSSYYFGMDV |
| | | | SEQ ID NO:7178 | SEQ ID NO:15190 | SEQ ID NO:23202 |
| iPS:436560 | 21-225_224F11 | NA | AATTATGATATCAAC | TGGATGAACCCTAACAG TGGTAACACAGGCTATG CACAGAAGTTCCAGGGC | AGCAGTGGCTGGTACAAGTT TGACTAC |
| | | | SEQ ID NO:7179 | SEQ ID NO:15191 | SEQ ID NO:23203 |
| | | AA | NYDIN | WMNPNSGNTGYAQKFQG | SSGWYKFDY |
| | | | SEQ ID NO:7180 | SEQ ID NO:15192 | SEQ ID NO:23204 |
| iPS:436562 | 21-225_224H11 | NA | GGCTACTATATACAC | TGGATCAACCCTTACAGT GGTGACACAAACTATGC ACAGAAGTTTCAGGGC | GATTGGGGTGGCTACAGTTC TTACTACTACGGTATGGACG TC |
| | | | SEQ ID NO:7181 | SEQ ID NO:15193 | SEQ ID NO:23205 |
| | | AA | GYYIH | WINPYSGDTNYAQKFQG | DWGGYSSYYYGMDV |
| | | | SEQ ID NO:7182 | SEQ ID NO:15194 | SEQ ID NO:23206 |

FIGURE 49

| iPS:436564 | | NA | GACTATGTCATCCAC | GTTATATGGTATGATGG AAGTAATAAATACTATG CAGACTCCGTGAAGGGC | GAGAGGTATAGCAGTGGCTG GTACGACTACGGTATGGACG TC |
|---|---|---|---|---|---|
| | 21-225_225A1 | | SEQ ID NO:7183 | SEQ ID NO:15195 | SEQ ID NO:23207 |
| | | AA | DYVIH | VIWYDGSNKYYADSVKG | ERYSSGWYDYGMDV |
| | | | SEQ ID NO:7184 | SEQ ID NO:15196 | SEQ ID NO:23208 |
| iPS:436568 | | NA | AGCTACTATATGCAC | ATAATCAACCCTAGTGG TGGTAGCACAAGCTACG CACAGAAGTTCCAGGGC | GATTTAGCAGCTCGTTCTTA CTACTACTACTTCGGTATGG ACGTC |
| | 21-225_225B3 | | SEQ ID NO:7185 | SEQ ID NO:15197 | SEQ ID NO:23209 |
| | | AA | SYYMH | IINPSGGSTSYAQKFQG | DLAARSYYYYFGMDV |
| | | | SEQ ID NO:7186 | SEQ ID NO:15198 | SEQ ID NO:23210 |
| iPS:436570 | | NA | AATTATGATATCAAC | TGGATGCACCCTAACAG TGGTAGCACAGGCTATG CACAGAAGTTCCAGGGC | AGCAGTGGCTGGTACTGGTT CGACCCC |
| | 21-225_225F4 | | SEQ ID NO:7187 | SEQ ID NO:15199 | SEQ ID NO:23211 |
| | | AA | NYDIN | WMHPNSGSTGYAQKFQG | SSGWYWFDP |
| | | | SEQ ID NO:7188 | SEQ ID NO:15200 | SEQ ID NO:23212 |
| iPS:436572 | | NA | GGCTACTATATACAC | TGGATCAACCCTTACAGT GGTGACACAAACTATGC ACAGAAGTTTCAGGGC | GATTGGGGTGGCTACAGTTC TTACTACTACGGTATGGACG TC |
| | 21-225_225G4 | | SEQ ID NO:7189 | SEQ ID NO:15201 | SEQ ID NO:23213 |
| | | AA | GYYIH | WINPYSGDTNYAQKFQG | DWGGYSSYYYGMDV |
| | | | SEQ ID NO:7190 | SEQ ID NO:15202 | SEQ ID NO:23214 |
| iPS:436574 | | NA | AATTATGATATCAAC | TGGATGCATCCTAACAG TGGTAACACAGGCTTTG CACAGAAGTTCCAGGGC | AGCAGTGGCTGGTACCGCTT TGACTAC |

FIGURE 49

| | 21-225_225F5 | | SEQ ID NO:7191 | SEQ ID NO:15203 | SEQ ID NO:23215 |
|---|---|---|---|---|---|
| | | AA | NYDIN | WMHPNSGNTGFAQKFQG | SSGWYRFDY |
| | | | SEQ ID NO:7192 | SEQ ID NO:15204 | SEQ ID NO:23216 |
| iPS:436576 | | NA | GACTATGGCATGCAC | GTTATATGGTATGATGA AAATAATAAATACTATG CAGACTCCGTGAAGGGC | GAAGTGGGATTCACTGAGGA CTAC |
| | 21-225_225B6 | | SEQ ID NO:7193 | SEQ ID NO:15205 | SEQ ID NO:23217 |
| | | AA | DYGMH | VIWYDENNKYYADSVKG | EVGFTEDY |
| | | | SEQ ID NO:7194 | SEQ ID NO:15206 | SEQ ID NO:23218 |
| iPS:436578 | | NA | AACTATGGCATGCAC | GTTATATGGTATGATGA AAATAATAAATACTATG CAGACTCCGTGAAGGGC | GAAGTGGGATTCACTGAGGA CTAC |
| | 21-225_225D6 | | SEQ ID NO:7195 | SEQ ID NO:15207 | SEQ ID NO:23219 |
| | | AA | NYGMH | VIWYDENNKYYADSVKG | EVGFTEDY |
| | | | SEQ ID NO:7196 | SEQ ID NO:15208 | SEQ ID NO:23220 |
| iPS:436580 | | NA | AGTGGTCATTACTACTGGAG C | TTCATCTATTACACTGGG AGCACCTACTACAACCC GTCCCTCAAGAGT | GAGGCCGGTGACTACGGCTA CTACGGTATGGACGTC |
| | 21-225_225E7 | | SEQ ID NO:7197 | SEQ ID NO:15209 | SEQ ID NO:23221 |
| | | AA | SGHYYWS | FIYYTGSTYYNPSLKS | EAGDYGYYGMDV |
| | | | SEQ ID NO:7198 | SEQ ID NO:15210 | SEQ ID NO:23222 |
| iPS:436582 | 21 225 225F8 | NA | AGCTATGGCATGCAC | GTTATATGGTATGATGA AAATAATAAATACTATG TAGACTCCGTGAAGGGC | GAAGTGGGATTTACTGAGGA CTAC |

FIGURE 49

| | | | SEQ ID NO:7199 | SEQ ID NO:15211 | SEQ ID NO:23223 |
|---|---|---|---|---|---|
| | **21-225_225F8** | AA | SYGMH | VIWYDENNKYYVDSVKG | EVGFTEDY |
| | | | SEQ ID NO:7200 | SEQ ID NO:15212 | SEQ ID NO:23224 |
| **iPS:436584** | | NA | AATTATGATATCAAC | TGGATGCACCCTAACAG TGGTAACACAGGCTATG CACAGAAGTTCCGGGGC | AGCAGTGGCTGGACCCTTTT TGACTAC |
| | **21-225_225B9** | | SEQ ID NO:7201 | SEQ ID NO:15213 | SEQ ID NO:23225 |
| | | AA | NYDIN | WMHPNSGNTGYAQKFRG | SSGWTLFDY |
| | | | SEQ ID NO:7202 | SEQ ID NO:15214 | SEQ ID NO:23226 |
| **iPS:436586** | | NA | AATTATGATATCAAC | TGGATGCACCCTAACAG TGGTAACACAGGCTATG CACAGAAGTTCCAGGGC | AGCAGTGGCTGGTACCGCTT TGACTAC |
| | **21-225_225F11** | | SEQ ID NO:7203 | SEQ ID NO:15215 | SEQ ID NO:23227 |
| | | AA | NYDIN | WMHPNSGNTGYAQKFQG | SSGWYRFDY |
| | | | SEQ ID NO:7204 | SEQ ID NO:15216 | SEQ ID NO:23228 |
| **iPS:436588** | | NA | CATTATGATATCAAC | TGGATGCACCCAAACAG TGGTAACACAGGCTATG CACAGAAGTTCCAGGGC | AGCAGTGGCTGGTACAAGTT TGACTAC |
| | **21-225_225F12** | | SEQ ID NO:7205 | SEQ ID NO:15217 | SEQ ID NO:23229 |
| | | AA | HYDIN | WMHPNSGNTGYAQKFQG | SSGWYKFDY |
| | | | SEQ ID NO:7206 | SEQ ID NO:15218 | SEQ ID NO:23230 |
| **iPS:436590** | | NA | AATTATGATATCAAC | TGGATGCACCCTAACAG TGGTAACACAGGCTATG CACAGAAGTTCCAGGGC | AGCAGTGGCTGGTACAAGTT TGACTAC |
| | **21-225_225H12** | | SEQ ID NO:7207 | SEQ ID NO:15219 | SEQ ID NO:23231 |

FIGURE 49

| | | AA | NYDIN | WMHPNSGNTGYAQKFQG | SSGWYKFDY |
|---|---|---|---|---|---|
| | | | SEQ ID NO:7208 | SEQ ID NO:15220 | SEQ ID NO:23232 |
| iPS:436592 | 21-225_226B1 | NA | ACCTATGGCATGCAC | ATTATATGGTATGATGG AGGTTATAAATACTATG CAGACTCCGTGAAGGGC | GATCACTACGATTTTTGGAG TGGTTATCTTACCCAC |
| | | | SEQ ID NO:7209 | SEQ ID NO:15221 | SEQ ID NO:23233 |
| | | AA | TYGMH | IIWYDGGYKYYADSVKG | DHYDFWSGYLTH |
| | | | SEQ ID NO:7210 | SEQ ID NO:15222 | SEQ ID NO:23234 |
| iPS:436594 | 21-225_226A5 | NA | AACTATGGCATGCAC | ATTATATGGTATGATGG AACTAATAAATACTATA CAGACTCCGTGAAGGGC | GAGGGTCACGATTTTTGGAG TGGCTTTTTTTGTTAC |
| | | | SEQ ID NO:7211 | SEQ ID NO:15223 | SEQ ID NO:23235 |
| | | AA | NYGMH | IIWYDGTNKYYTDSVKG | EGHDFWSGFFCY |
| | | | SEQ ID NO:7212 | SEQ ID NO:15224 | SEQ ID NO:23236 |
| iPS:436596 | 21-225_226C6 | NA | GACTATGGCATGCAC | GTTATATGGTATGATGG AAGTAATAAATACTATG CAGACTCCGTGAAGGGC | GAGAGGTATAGCAGCAGCTG GTACGACTACGGTATGGACG TC |
| | | | SEQ ID NO:7213 | SEQ ID NO:15225 | SEQ ID NO:23237 |
| | | AA | DYGMH | VIWYDGSNKYYADSVKG | ERYSSSWYDYGMDV |
| | | | SEQ ID NO:7214 | SEQ ID NO:15226 | SEQ ID NO:23238 |
| iPS:436598 | 21-225_226D6 | NA | AATTATGATATCAAC | TGGATGCACCCTAACAG TGGTAACACAGGCTATG CACAGAAGTTCCAGGGC | AGCAGTGGCTGGTACAAGTT TGACTAC |
| | | | SEQ ID NO:7215 | SEQ ID NO:15227 | SEQ ID NO:23239 |
| | | AA | NYDIN | WMHPNSGNTGYAQKFQG | SSGWYKFDY |

FIGURE 49

|  |  |  | SEQ ID NO:7216 | SEQ ID NO:15228 | SEQ ID NO:23240 |
|---|---|---|---|---|---|
| iPS:436600 |  | NA | AATTATGATATCAAC | TGGATGCACCCTAACAG TGGTAACACAGGCTATG CACAGAAGTTCCAGGGC | AGCAGTGGCTGGTACCGCTT TGACTAC |
|  | 21-225_226F6 |  | SEQ ID NO:7217 | SEQ ID NO:15229 | SEQ ID NO:23241 |
|  |  | AA | NYDIN | WMHPNSGNTGYAQKFQG | SSGWYRFDY |
|  |  |  | SEQ ID NO:7218 | SEQ ID NO:15230 | SEQ ID NO:23242 |
| iPS:436602 |  | NA | ACCTATGGCATGCAC | ATTATATGGTATGATGG AAGTAATAAATACTATG CAGACTCCGTGAAGGGC | GAGAATTACGATTTTTGGAG TGGTTATTATGGCTAC |
|  | 21-225_226E7 |  | SEQ ID NO:7219 | SEQ ID NO:15231 | SEQ ID NO:23243 |
|  |  | AA | TYGMH | IIWYDGSNKYYADSVKG | ENYDFWSGYYGY |
|  |  |  | SEQ ID NO:7220 | SEQ ID NO:15232 | SEQ ID NO:23244 |
| iPS:436604 |  | NA | AGCTATGGCATGCAC | ATTATATGGTATGATGG AAGTAATAAATACTATG CAGACTCCGTGAAGGGC | GAGAGGTATAACAGCGGCTG GTACGACTACGGTTTGGACG TC |
|  | 21-225_226F7 |  | SEQ ID NO:7221 | SEQ ID NO:15233 | SEQ ID NO:23245 |
|  |  | AA | SYGMH | IIWYDGSNKYYADSVKG | ERYNSGWYDYGLDV |
|  |  |  | SEQ ID NO:7222 | SEQ ID NO:15234 | SEQ ID NO:23246 |
| iPS:436606 |  | NA | GGCTACTATATACAC | TGGATCAACCCTTACAGT GGTGACACAAAGTATGC ACAGAAGTTTCAGGGC | GATTGGGGGTGGCTACAGTTC TTACTACTACGGTATGGACG TC |
|  | 21-225_226G8 |  | SEQ ID NO:7223 | SEQ ID NO:15235 | SEQ ID NO:23247 |
|  |  | AA | GYYIH | WINPYSGDTKYAQKFQG | DWGGYSSYYYGMDV |
|  |  |  | SEQ ID NO:7224 | SEQ ID NO:15236 | SEQ ID NO:23248 |

FIGURE 49

| iPS:436608 | | NA | AACTATGGCATGCAC | GTTATATGGTATGAGGA AAGTAATAAATACTATA CAGACTCCGTGAAGGGC | GAAGTGGGATTCACTGAGGA CTAC |
|---|---|---|---|---|---|
| | 21-225_226A9 | | SEQ ID NO:7225 | SEQ ID NO:15237 | SEQ ID NO:23249 |
| | | AA | NYGMH | VIWYEESNKYYTDSVKG | EVGFTEDY |
| | | | SEQ ID NO:7226 | SEQ ID NO:15238 | SEQ ID NO:23250 |
| iPS:436610 | | NA | GGCTACTATATACAC | TGGATCAACCCTTACAGT GGTGACACAAAGTATGC ACAGAAGTTTCAGGGC | GATTGGGGTGGCTACAGTTC TTACTACTACGGTATGGACG TC |
| | 21-225_226F9 | | SEQ ID NO:7227 | SEQ ID NO:15239 | SEQ ID NO:23251 |
| | | AA | GYYIH | WINPYSGDTKYAQKFQG | DWGGYSSYYYGMDV |
| | | | SEQ ID NO:7228 | SEQ ID NO:15240 | SEQ ID NO:23252 |
| iPS:436612 | | NA | GGCTACTATATACAC | TGGATCAACCCTTACAGT GGTGACACAAACTCTGC ACAGAAGTTTCAGGGC | GATTGGGGTGGCTACAGTTC TTACTACTACGGTATGGACG TC |
| | 21-225_226H9 | | SEQ ID NO:7229 | SEQ ID NO:15241 | SEQ ID NO:23253 |
| | | AA | GYYIH | WINPYSGDTNSAQKFQG | DWGGYSSYYYGMDV |
| | | | SEQ ID NO:7230 | SEQ ID NO:15242 | SEQ ID NO:23254 |
| iPS:436614 | | NA | GGCTATTATATACAC | TGGATCAACCCATACAG TGGTGACACAAACTATG CACAGAAGTTTCAGGGC | GATTGGGGTGGCTACAGTTC TTACTACTACGGTATGGACG TC |
| | 21-225_226F10 | | SEQ ID NO:7231 | SEQ ID NO:15243 | SEQ ID NO:23255 |
| | | AA | GYYIH | WINPYSGDTNYAQKFQG | DWGGYSSYYYGMDV |
| | | | SEQ ID NO:7232 | SEQ ID NO:15244 | SEQ ID NO:23256 |
| iPS:436616 | | NA | AATTATGATATCAAC | TGGATGCACCCTAACAG TGGTAACACAGGCTATG CACAGAAGTTCCAGGGC | AGCAGTGGCTGGTACTACTT TGACTAC |
| | 21-225_226D11 | | SEQ ID NO:7233 | SEQ ID NO:15245 | SEQ ID NO:23257 |

FIGURE 49

| | | AA | NYDIN | WMHPNSGNTGYAQKFQG | SSGWYYFDY |
|---|---|---|---|---|---|
| | | | SEQ ID NO:7234 | SEQ ID NO:15246 | SEQ ID NO:23258 |
| iPS:436618 | 21-225_226E11 | NA | GGCTACTATATACAC | TGGATCAACCCTTACAGTGGTGACACAAACTATGCACAGAAGTTTCAGGGC | GATTGGGGTGGCTACAGTTCTTACTACTACGGTATGGACGTC |
| | | | SEQ ID NO:7235 | SEQ ID NO:15247 | SEQ ID NO:23259 |
| | | AA | GYYIH | WINPYSGDTNYAQKFQG | DWGGYSSYYYGMDV |
| | | | SEQ ID NO:7236 | SEQ ID NO:15248 | SEQ ID NO:23260 |
| iPS:436620 | 21-225_226H11 | NA | AACTGTGGCATGCAC | GTTATATGGTATGATGGAAGTAATAAATACTATGCAGACTCCGTGAAGGGC | GAGCTGTATAGCAGCAGCTGGTACGACTACGGTTTGGACGTC |
| | | | SEQ ID NO:7237 | SEQ ID NO:15249 | SEQ ID NO:23261 |
| | | AA | NCGMH | VIWYDGSNKYYADSVKG | ELYSSSWYDYGLDV |
| | | | SEQ ID NO:7238 | SEQ ID NO:15250 | SEQ ID NO:23262 |
| iPS:436622 | 21-225_226A12 | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:7239 | SEQ ID NO:15251 | SEQ ID NO:23263 |
| | | AA | NYDIN | WMHPNSGNTGYAQKFQG | SSGWYYFDY |
| | | | SEQ ID NO:7240 | SEQ ID NO:15252 | SEQ ID NO:23264 |
| iPS:436624 | 21-225_226H12 | NA | GGCTACTATATACAC | TGGATCAACCCTTACAGTGGTGACACAAACTATGCACAGAAGTTTCAGGGC | GATTGGGGTGGCTACAGTTCTTACTACTACGGTATGGACGTC |
| | | | SEQ ID NO:7241 | SEQ ID NO:15253 | SEQ ID NO:23265 |
| | | AA | GYYIH | WINPYSGDTNYAQKFQG | DWGGYSSYYYGMDV |
| | | | SEQ ID NO:7242 | SEQ ID NO:15254 | SEQ ID NO:23266 |

FIGURE 49

| iPS:436626 | | NA | GGCTACTATACACAC | TGGATCAACCCTTACAGT GGTGGCACAAACTATGC ACAGAAGTTTCAGGGC | GATTGGGGTGGCTACAGTTC TTACTACTACGGTATGGACG TC |
|---|---|---|---|---|---|
| | 21-225_227C1 | | SEQ ID NO:7243 | SEQ ID NO:15255 | SEQ ID NO:23267 |
| | | AA | GYYTH | WINPYSGGTNYAQKFQG | DWGGYSSYYYGMDV |
| | | | SEQ ID NO:7244 | SEQ ID NO:15256 | SEQ ID NO:23268 |
| iPS:436628 | | NA | GGCTACTATATACAC | TGGATCAACCCTTACAGT GGTGACACAAAGTATGC ACAGAAGTTTCAGGGC | GATTGGGGTGGCTACAGTTC TTACTACTACGGTATGGACG TC |
| | 21-225_227F2 | | SEQ ID NO:7245 | SEQ ID NO:15257 | SEQ ID NO:23269 |
| | | AA | GYYIH | WINPYSGDTKYAQKFQG | DWGGYSSYYYGMDV |
| | | | SEQ ID NO:7246 | SEQ ID NO:15258 | SEQ ID NO:23270 |
| iPS:436630 | | NA | AGCTATGGCATGCAC | GTTATATGGTATGTTGGA AGTAATCAATACTATGC AGACTCCGTGAAGGGC | GAAGTGGGATTCACTGAGGA CTAC |
| | 21-225_227G3 | | SEQ ID NO:7247 | SEQ ID NO:15259 | SEQ ID NO:23271 |
| | | AA | SYGMH | VIWYVGSNQYYADSVKG | EVGFTEDY |
| | | | SEQ ID NO:7248 | SEQ ID NO:15260 | SEQ ID NO:23272 |
| iPS:436632 | | NA | ACTTTTGCCATGACC | GTTATTAGTGGTAGAGG TGGTAGCTCATTCTACGC AGACTCCGTGAAGGGC | GATCAACTATGGTTTGACTA C |
| | 21-225_227E4 | | SEQ ID NO:7249 | SEQ ID NO:15261 | SEQ ID NO:23273 |
| | | AA | TFAMT | VISGRGGSSFYADSVKG | DQLWFDY |
| | | | SEQ ID NO:7250 | SEQ ID NO:15262 | SEQ ID NO:23274 |
| iPS:436634 | | NA | AACTATGGCATGCAC | GTTATATGGTATGAAGA AAGTAATAAATACTATG CAGACTCCGTGAAGGGC | GAAGTGGGATTCACTGAGGA CTAC |
| | 21-225_227H5 | | SEQ ID NO:7251 | SEQ ID NO:15263 | SEQ ID NO:23275 |

FIGURE 49

| | | AA | NYGMH | VIWYEESNKYYADSVKG | EVGFTEDY |
|---|---|---|---|---|---|
| | | | SEQ ID NO:7252 | SEQ ID NO:15264 | SEQ ID NO:23276 |
| iPS:436636 | | NA | AATTATGATATCAAC | TGGATGCACCCTAACAG TGGTAACACAGGCTATG CACAGAAGTTCCAGGGC | AGCAGTGGCTGGTACAAGTT TGACTAC |
| | 21-225_227E6 | | SEQ ID NO:7253 | SEQ ID NO:15265 | SEQ ID NO:23277 |
| | | AA | NYDIN | WMHPNSGNTGYAQKFQG | SSGWYKFDY |
| | | | SEQ ID NO:7254 | SEQ ID NO:15266 | SEQ ID NO:23278 |
| iPS:436638 | | NA | AATTATGATATCAAC | TGGATGCATCCTAACAG TGGTAACACAGGCTATG CACAGAAGTTCCAGGGC | AGCAGTGGCTGGTACCGCTT TGACTAC |
| | 21-225_227C7 | | SEQ ID NO:7255 | SEQ ID NO:15267 | SEQ ID NO:23279 |
| | | AA | NYDIN | WMHPNSGNTGYAQKFQG | SSGWYRFDY |
| | | | SEQ ID NO:7256 | SEQ ID NO:15268 | SEQ ID NO:23280 |
| iPS:436640 | | NA | GGCTACTATATACAC | TGGATCAACCCTTACAGT GGTGACACAAACTATGC ACAGAAGTTTCAGGGC | GATTGGGGGTGGCTACAGTTC TTACTACTACGGTATGGACG TC |
| | 21-225_227A8 | | SEQ ID NO:7257 | SEQ ID NO:15269 | SEQ ID NO:23281 |
| | | AA | GYYIH | WINPYSGDTNYAQKFQG | DWGGYSSYYYGMDV |
| | | | SEQ ID NO:7258 | SEQ ID NO:15270 | SEQ ID NO:23282 |
| iPS:436644 | | NA | AATTATGATATCAAC | TGGATGTACCCTAACAG TGGTAACACAGGCTATG CACAGAAGTTCCAGGGC | AGCAGTGGCTGGTACTGGTT CGACCCC |
| | 21-225_227G9 | | SEQ ID NO:7259 | SEQ ID NO:15271 | SEQ ID NO:23283 |
| | | AA | NYDIN | WMYPNSGNTGYAQKFQG | SSGWYWFDP |
| | | | SEQ ID NO:7260 | SEQ ID NO:15272 | SEQ ID NO:23284 |

FIGURE 49

| iPS:436646 | | NA | AATTATGATATCAAC | TGGATGCACCCTAACAGTGGTAACACAGGCTATGCACAGAAGTTCCAGGGC | AGCAGTGGCTGGTACTACTTTGACTAC |
|---|---|---|---|---|---|
| | 21-225_227D11 | | SEQ ID NO:7261 | SEQ ID NO:15273 | SEQ ID NO:23285 |
| | | AA | NYDIN | WMHPNSGNTGYAQKFQG | SSGWYYFDY |
| | | | SEQ ID NO:7262 | SEQ ID NO:15274 | SEQ ID NO:23286 |
| iPS:436648 | | NA | ACCTATAGCATGAAC | TCCATTAGTAGTAGTATTAATTACATGTACTACGCAGACTCAGTGAAGGGC | TTAGGGGTCTAC |
| | 21-225_227F11 | | SEQ ID NO:7263 | SEQ ID NO:15275 | SEQ ID NO:23287 |
| | | AA | TYSMN | SISSSINYMYYADSVKG | LGVY |
| | | | SEQ ID NO:7264 | SEQ ID NO:15276 | SEQ ID NO:23288 |
| iPS:436650 | | NA | AACTATGGCATGCAC | GTTATATGGTATATTGGAAGTAATCAATACTATGCGGACTCCGTGAAGGGC | GAAGTGGGATTCACTGAGGACTAC |
| | 21-225_227C12 | | SEQ ID NO:7265 | SEQ ID NO:15277 | SEQ ID NO:23289 |
| | | AA | NYGMH | VIWYIGSNQYYADSVKG | EVGFTEDY |
| | | | SEQ ID NO:7266 | SEQ ID NO:15278 | SEQ ID NO:23290 |
| iPS:436652 | | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | 21-225_146B11 | | SEQ ID NO:7267 | SEQ ID NO:15279 | SEQ ID NO:23291 |
| | | AA | SYAMS | VISGGGSSTYYADSVKG | WRGNPTDYGMDV |
| | | | SEQ ID NO:7268 | SEQ ID NO:15280 | SEQ ID NO:23292 |
| iPS:436654 | | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | 21-225_146C11 | | SEQ ID NO:7269 | SEQ ID NO:15281 | SEQ ID NO:23293 |
| | | AA | SYAMS | VISGGGSSTYYADSVKG | WRGNPTDYGMDV |

FIGURE 49

| | | | SEQ ID NO:7270 | SEQ ID NO:15282 | SEQ ID NO:23294 |
|---|---|---|---|---|---|
| iPS:436658 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_146A2 | | SEQ ID NO:7271 | SEQ ID NO:15283 | SEQ ID NO:23295 |
| | | AA | SYAMS | VISGGGSSTYYADSVKG | WRGNPTDYGMDV |
| | | | SEQ ID NO:7272 | SEQ ID NO:15284 | SEQ ID NO:23296 |
| iPS:436660 | | NA | AACTATAACATGAAC | TACATTAGTAGAAGTAG TAATACCAAATACTATGT AGACTCTGTGAAGGGC | GATAGGAGTGGGAGCTACGG GTACTTCTACTACTACGGTTT GGACGTC |
| | 21-225_146D8 | | SEQ ID NO:7273 | SEQ ID NO:15285 | SEQ ID NO:23297 |
| | | AA | NYNMN | YISRSSNTKYYVDSVKG | DRSGSYGYFYYYGLDV |
| | | | SEQ ID NO:7274 | SEQ ID NO:15286 | SEQ ID NO:23298 |
| iPS:436662 | | NA | AGTTATGATATCAAC | TGGATGAACCCTAATAG TGGTAACACAGGCTATG CACAGAAGTTCCAGGGC | GCGGATATTGTATTAGTACC AGCTGCTATCCCTTATAATT ACTACTTCGCTATGGACGTC |
| | 21-225_147D7 | | SEQ ID NO:7275 | SEQ ID NO:15287 | SEQ ID NO:23299 |
| | | AA | SYDIN | WMNPNSGNTGYAQKFQG | ADIVLVPAAIPYNYYFAMDV |
| | | | SEQ ID NO:7276 | SEQ ID NO:15288 | SEQ ID NO:23300 |
| iPS:436664 | | NA | AGCTATGCCATGAGC | GTTATTAGTGGTGGTGGT AGTAGTACATACTACGC AGACTCCGTGAAGGGC | TGGCGAGGTAACCCCACTGA CTACGGTATGGACGTC |
| | 21-225_147E7 | | SEQ ID NO:7277 | SEQ ID NO:15289 | SEQ ID NO:23301 |
| | | AA | SYAMS | VISGGGSSTYYADSVKG | WRGNPTDYGMDV |
| | | | SEQ ID NO:7278 | SEQ ID NO:15290 | SEQ ID NO:23302 |
| iPS:436666 | | NA | GACTACTATTTGCAC | TGGATCAACCCTAACAG TGGTGACACAAACTATG CACAGAAGTTTCAGGGC | GATCGGGACTCTGGTTCGGG GAGTTACCCCTACTACTACT ACTACGGTATGGACGTC |
| | 21-225_147B8 | | SEQ ID NO:7279 | SEQ ID NO:15291 | SEQ ID NO:23303 |

FIGURE 49

| | | | | | |
|---|---|---|---|---|---|
| | | AA | DYYLH | WINPNSGDTNYAQKFQG | DRDSGSGSYPYYYYYGMDV |
| | | | SEQ ID NO:7280 | SEQ ID NO:15292 | SEQ ID NO:23304 |
| iPS:436668 | 21-225_147B9 | NA | AGCTATGGCATGCAC | GTTATATGGTATGATGG AAGTAATAAATACTATG CAGACTCCGTGAAGGGC | GATCGTGACTACGGTGACCC CCCCTACTACTACTACTACG GTATGGACGTC |
| | | | SEQ ID NO:7281 | SEQ ID NO:15293 | SEQ ID NO:23305 |
| | | AA | SYGMH | VIWYDGSNKYYADSVKG | DRDYGDPPYYYYYGMDV |
| | | | SEQ ID NO:7282 | SEQ ID NO:15294 | SEQ ID NO:23306 |
| iPS:436670 | 21-225_147D9 | NA | AGCTATGGCATGCAC | GATATATGGTTTGATGGC AGTAATAAATACTATGT AGACTCCGTGAAGGAC | GATCGGGTGGAGGGTTCGGG GACTCCCTACTACTACTACG GTATGGACGTC |
| | | | SEQ ID NO:7283 | SEQ ID NO:15295 | SEQ ID NO:23307 |
| | | AA | SYGMH | DIWFDGSNKYYVDSVKD | DRVEGSGTPYYYYGMDV |
| | | | SEQ ID NO:7284 | SEQ ID NO:15296 | SEQ ID NO:23308 |
| iPS:436672 | 21-225_147F9 | NA | ACCTATGGCATGCAC | GTTATATGGTATGGTGG AAGTGATAAAGACTATG CAGACTCCGTGAAGGGC | GATCGGGATTATTGTAGTGG TGGTACTTGTCCTTACTACTA CTACTACGGTATGGACGTC |
| | | | SEQ ID NO:7285 | SEQ ID NO:15297 | SEQ ID NO:23309 |
| | | AA | TYGMH | VIWYGGSDKDYADSVKG | DRDYCSGGTCPYYYYYGMDV |
| | | | SEQ ID NO:7286 | SEQ ID NO:15298 | SEQ ID NO:23310 |
| iPS:436674 | 21-225_147G9 | NA | ACCTATGGCATGCAC | GTTATATGGTATGGTGG AAGTGATAAAGACTATG CAGACTCCGTGAAGGGC | GATCGGGATTATTGTAGTGG TGGTAGCTGCCCTTACTACT ACTACTACGGTATGGACGTC |
| | | | SEQ ID NO:7287 | SEQ ID NO:15299 | SEQ ID NO:23311 |

FIGURE 49

| | | AA | TYGMH | VIWYGGSDKDYADSVKG | DRDYCSGGSCPYYYYYGMDV |
|---|---|---|---|---|---|
| | | | SEQ ID NO:7288 | SEQ ID NO:15300 | SEQ ID NO:23312 |
| iPS:436676 | 21-225_147E11 | NA | AACTATGTCATGAGC | GTTATTAGTGGTGGTGGT AGTAGTACATACTACGC AGACTCCGTGAAGGGC | TGGCGAGGTAACCCCACTGA CTACGGTATGGACGTC |
| | | | SEQ ID NO:7289 | SEQ ID NO:15301 | SEQ ID NO:23313 |
| | | AA | NYVMS | VISGGGSSTYYADSVKG | WRGNPTDYGMDV |
| | | | SEQ ID NO:7290 | SEQ ID NO:15302 | SEQ ID NO:23314 |
| iPS:436678 | 21-225_147B12 | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | | | SEQ ID NO:7291 | SEQ ID NO:15303 | SEQ ID NO:23315 |
| | | AA | SYAMS | VISGGGSSTYYADSVKG | WRGNPTDYGMDV |
| | | | SEQ ID NO:7292 | SEQ ID NO:15304 | SEQ ID NO:23316 |
| iPS:436680 | 21-225_147H12 | NA | AGTGGTTATTACCACTGGAG C | TACATCTATTACAGTGGG AGCACCTACTACAACCC GTCCCTCAAGAGT | GATTGGGGTGGCTACGATTC GAGTGGCTGGTTCGACCCC |
| | | | SEQ ID NO:7293 | SEQ ID NO:15305 | SEQ ID NO:23317 |
| | | AA | SGYYHWS | YIYYSGSTYYNPSLKS | DWGGYDSSGWFDP |
| | | | SEQ ID NO:7294 | SEQ ID NO:15306 | SEQ ID NO:23318 |
| iPS:436682 | 21-225_146A8 | NA | AACTATAACATGAAC | TACATTAGTAGAAGTAG TAATACCAAATACTACG CAGACTCTGTGAGGGGC | GATAGGAGTGGGAGCTACGG GTACTTCTACTACTACGGTTT GGACGTC |
| | | | SEQ ID NO:7295 | SEQ ID NO:15307 | SEQ ID NO:23319 |
| | | AA | NYNMN | YISRSSNTKYYADSVRG | DRSGSYGYFYYYGLDV |
| | | | SEQ ID NO:7296 | SEQ ID NO:15308 | SEQ ID NO:23320 |

FIGURE 49

| iPS:436684 | | NA | AGCTATAACATGAAC | TACATTAGTAGAAGTAG TAATACCAAACACTACG CAGACTCTGTGAAGGGC | GATAGGAGTGGGAGCTACGG GTACTTCTACTACTACGGTTT GGACGTC |
|---|---|---|---|---|---|
| | 21-225_146B6 | | SEQ ID NO:7297 | SEQ ID NO:15309 | SEQ ID NO:23321 |
| | | AA | SYNMN | YISRSSNTKHYADSVKG | DRSGSYGYFYYYGLDV |
| | | | SEQ ID NO:7298 | SEQ ID NO:15310 | SEQ ID NO:23322 |
| iPS:436686 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_148G6 | | SEQ ID NO:7299 | SEQ ID NO:15311 | SEQ ID NO:23323 |
| | | AA | SYAMS | VISGGGSSTYYADSVKG | WRGNPTDYGMDV |
| | | | SEQ ID NO:7300 | SEQ ID NO:15312 | SEQ ID NO:23324 |
| iPS:436688 | | NA | AGCTATGGCATGCAC | GTTATATGGTATGATGG AAGTAATAAATACTATG CAGACTCCGTGAAGGGC | GATCGTGACTACGGTGACCC CCCCTACTACTACTACTACG GTATGGACGTC |
| | 21-225_148C8 | | SEQ ID NO:7301 | SEQ ID NO:15313 | SEQ ID NO:23325 |
| | | AA | SYGMH | VIWYDGSNKYYADSVKG | DRDYGDPPYYYYYGMDV |
| | | | SEQ ID NO:7302 | SEQ ID NO:15314 | SEQ ID NO:23326 |
| iPS:436690 | | NA | ACCTATGGCATGCAC | GTTATATGGTATGGTGG AAGTGATAAAGACTATG CAGACTCCGTGAAGGGC | GATCGGGATTATTGTAGTGG TGGTACTTGTCCTTACTACTA CTACTACGGTATGGACGTC |
| | 21-225_148A9 | | SEQ ID NO:7303 | SEQ ID NO:15315 | SEQ ID NO:23327 |
| | | AA | TYGMH | VIWYGGSDKDYADSVKG | DRDYCSGGTCPYYYYYGMDV |
| | | | SEQ ID NO:7304 | SEQ ID NO:15316 | SEQ ID NO:23328 |

FIGURE 49

| iPS:436694 | | NA | AGCTATCCCATGAGC | GTTATTAGTGGTGGTGGT AGTAGTGCATACTACGC AGACTCCGTGAAGGGC | TGGCGAGGTAACCCCACTGA CTACGGTATGGACGTC |
|---|---|---|---|---|---|
| | 21-225_148G11 | | SEQ ID NO:7305 | SEQ ID NO:15317 | SEQ ID NO:23329 |
| | | AA | SYPMS | VISGGGSSAYYADSVKG | WRGNPTDYGMDV |
| | | | SEQ ID NO:7306 | SEQ ID NO:15318 | SEQ ID NO:23330 |
| iPS:436696 | | NA | AGCTATAACATGAAC | TACATTAGTAGAAGTAG TAATACCAAACACTACG CAGACTCTGTGAAGGGC | GATAGGAGTGGGAGCTACGG GTACTTCTACTACTACGGTTT GGACGTC |
| | 21-225_149A1 | | SEQ ID NO:7307 | SEQ ID NO:15319 | SEQ ID NO:23331 |
| | | AA | SYNMN | YISRSSNTKHYADSVKG | DRSGSYGYFYYYGLDV |
| | | | SEQ ID NO:7308 | SEQ ID NO:15320 | SEQ ID NO:23332 |
| iPS:436698 | | NA | GGCTATTGGATGAAC | AACATAAAGCAAGATGG AAGTGAGAAATACTATG TGGACTCTGTGAAGGGC | GGGATGTATAGCAGTGGCTG GTACGTCTTTGACTAC |
| | 21-225_149B5 | | SEQ ID NO:7309 | SEQ ID NO:15321 | SEQ ID NO:23333 |
| | | AA | GYWMN | NIKQDGSEKYYVDSVKG | GMYSSGWYVFDY |
| | | | SEQ ID NO:7310 | SEQ ID NO:15322 | SEQ ID NO:23334 |
| iPS:436700 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_149C7 | | SEQ ID NO:7311 | SEQ ID NO:15323 | SEQ ID NO:23335 |
| | | AA | SYAMS | VISGGGSSTYYADSVKG | WRGNPTDYGMDV |
| | | | SEQ ID NO:7312 | SEQ ID NO:15324 | SEQ ID NO:23336 |
| iPS:436702 | | NA | AGTTATAGCATGAAC | GCCATTAGTAGTACTGGT AGTTACATATATTACGCA GACTCAGTGAAGGGC | ACGGCAGTGGCTGGTACTGG GTGGTTCGACCCC |
| | 21-225_149E8 | | SEQ ID NO:7313 | SEQ ID NO:15325 | SEQ ID NO:23337 |

FIGURE 49

| | | AA | SYSMN | AISSTGSYIYYADSVKG | TAVAGTGWFDP |
|---|---|---|---|---|---|
| | | | SEQ ID NO:7314 | SEQ ID NO:15326 | SEQ ID NO:23338 |
| iPS:436704 | | NA | AGCCACGCCATGAGC | GTTATAAGTGGAGGTGG TAGTAGCACATATTACG CAGACTCCGTGAAGGGC | TGGCGAGGTAACCCCACTGA CTACGGTATGGACGTC |
| | 21-225_149C10 | | SEQ ID NO:7315 | SEQ ID NO:15327 | SEQ ID NO:23339 |
| | | AA | SHAMS | VISGGGSSTYYADSVKG | WRGNPTDYGMDV |
| | | | SEQ ID NO:7316 | SEQ ID NO:15328 | SEQ ID NO:23340 |
| iPS:436706 | | NA | AGCTATGGCATGCAC | GTTATATGGTATGATGG AAGTAATAAATACTATG CAGACTCCGTGAAGGGC | GATCGTGACTACGGTGACCC CCCCTACTACTACTACTACG GTATGGACGTC |
| | 21-225_149A11 | | SEQ ID NO:7317 | SEQ ID NO:15329 | SEQ ID NO:23341 |
| | | AA | SYGMH | VIWYDGSNKYYADSVKG | DRDYGDPPYYYYYGMDV |
| | | | SEQ ID NO:7318 | SEQ ID NO:15330 | SEQ ID NO:23342 |
| iPS:436708 | | NA | ACCTATGGCATGCAC | GTTATATGGTATGGTGG AAGTAATAAAGACTATG CAGACTCCGTGAAGGGC | GATCGGGATTATTGTAGTGG TGGTACCTGCCCTTACTACT ACTACTACGGTATGGACGTC |
| | 21-225_150D3 | | SEQ ID NO:7319 | SEQ ID NO:15331 | SEQ ID NO:23343 |
| | | AA | TYGMH | VIWYGGSNKDYADSVKG | DRDYCSGGTCPYYYYYGMDV |
| | | | SEQ ID NO:7320 | SEQ ID NO:15332 | SEQ ID NO:23344 |
| iPS:436710 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_150F6 | | SEQ ID NO:7321 | SEQ ID NO:15333 | SEQ ID NO:23345 |
| | | AA | SYAMS | VISGGGSSTYYADSVKG | WRGNPTDYGMDV |
| | | | SEQ ID NO:7322 | SEQ ID NO:15334 | SEQ ID NO:23346 |

FIGURE 49

| iPS:436712 | | NA | AGCTATAACATGAAC | TACATTAGTAGAAGTAG TAATACCAAACACTACG CAGACTCTGTGAAGGGC | GATAGGAGTGGGAGCTACGG GTACTTCTACTACTACGGTTT GGACGTC |
|---|---|---|---|---|---|
| | 21-225_150F9 | | SEQ ID NO:7323 | SEQ ID NO:15335 | SEQ ID NO:23347 |
| | | AA | SYNMN | YISRSSNTKHYADSVKG | DRSGSYGYFYYYGLDV |
| | | | SEQ ID NO:7324 | SEQ ID NO:15336 | SEQ ID NO:23348 |
| iPS:436714 | | NA | ACCTATGCCATGAGC | ATTATTAGTGGTGGTGGT AGTAGCACATACTACGC AGACTCCGTGAAGGGC | TGGCGAGGTAACCCCACTGA CTACGGTATGGACGTC |
| | 21-225_150H11 | | SEQ ID NO:7325 | SEQ ID NO:15337 | SEQ ID NO:23349 |
| | | AA | TYAMS | IISGGGSSTYYADSVKG | WRGNPTDYGMDV |
| | | | SEQ ID NO:7326 | SEQ ID NO:15338 | SEQ ID NO:23350 |
| iPS:436716 | | NA | ACCTATGGCATGCAC | GTTATATGGTATGGTGG AAGTAATACAGACTATG CAGACTCCGTGAAGGGC | GATCGGGATTATTGTAGTGG TACTAGCTGCCCTTACTACT ACTACTACGGTATGGACGTC |
| | 21-225_151F3 | | SEQ ID NO:7327 | SEQ ID NO:15339 | SEQ ID NO:23351 |
| | | AA | TYGMH | VIWYGGSNTDYADSVKG | DRDYCSGTSCPYYYYYGMDV |
| | | | SEQ ID NO:7328 | SEQ ID NO:15340 | SEQ ID NO:23352 |
| iPS:436718 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_151H5 | | SEQ ID NO:7329 | SEQ ID NO:15341 | SEQ ID NO:23353 |
| | | AA | SYAMS | VISGGGSSTYYADSVKG | WRGNPTDYGMDV |
| | | | SEQ ID NO:7330 | SEQ ID NO:15342 | SEQ ID NO:23354 |

FIGURE 49

| iPS:436720 | | NA | GACTATGGCATGCAC | CTTATATGGTATGATGGA AGTAATAAATACTATGC AGACTCCGTGAAGGGC | GATGACCGATCTTGTAGTAG AACCAGCTGCCCTTACTACT ACTACTACGGTTTGGACGTC |
| | 21-225_151H6 | | SEQ ID NO:7331 | SEQ ID NO:15343 | SEQ ID NO:23355 |
| | | AA | DYGMH | LIWYDGSNKYYADSVKG | DDRSCSRTSCPYYYYYGLDV |
| | | | SEQ ID NO:7332 | SEQ ID NO:15344 | SEQ ID NO:23356 |
| iPS:436722 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_151H7 | | SEQ ID NO:7333 | SEQ ID NO:15345 | SEQ ID NO:23357 |
| | | AA | SYAMS | VISGGGSSTYYADSVKG | WRGNPTDYGMDV |
| | | | SEQ ID NO:7334 | SEQ ID NO:15346 | SEQ ID NO:23358 |
| iPS:436724 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_151B9 | | SEQ ID NO:7335 | SEQ ID NO:15347 | SEQ ID NO:23359 |
| | | AA | SYAMS | VISGGGSSTYYADSVKG | WRGNPTDYGMDV |
| | | | SEQ ID NO:7336 | SEQ ID NO:15348 | SEQ ID NO:23360 |
| iPS:436726 | | NA | GACTATGGCATGCAC | CTTATATGGTATGATGGA AGTAATAAATACTATGC AGACTCCGTGAAGGGC | GATGACCGATCTTGTAGTAG AACCAGCTGCCCTTACTACT ACTACTACGGTTTGGACGTC |
| | 21-225_152G5 | | SEQ ID NO:7337 | SEQ ID NO:15349 | SEQ ID NO:23361 |
| | | AA | DYGMH | LIWYDGSNKYYADSVKG | DDRSCSRTSCPYYYYYYGLDV |
| | | | SEQ ID NO:7338 | SEQ ID NO:15350 | SEQ ID NO:23362 |

FIGURE 49

| iPS:436728 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
|---|---|---|---|---|---|
| | 21-225_152G6 | | SEQ ID NO:7339 | SEQ ID NO:15351 | SEQ ID NO:23363 |
| | | AA | SYAMS | VISGGGSSTYYADSVKG | WRGNPTDYGMDV |
| | | | SEQ ID NO:7340 | SEQ ID NO:15352 | SEQ ID NO:23364 |
| iPS:436730 | | NA | AGCTATGCCATGAGC | GTTATTAGTGGTGGTGGT AGTAGCACATACTACGC AGACTCCGTGAAGGGC | TGGCGAGGTAACCCCACTGA CTCCGGTATGGACGTC |
| | 21-225_152D7 | | SEQ ID NO:7341 | SEQ ID NO:15353 | SEQ ID NO:23365 |
| | | AA | SYAMS | VISGGGSSTYYADSVKG | WRGNPTDSGMDV |
| | | | SEQ ID NO:7342 | SEQ ID NO:15354 | SEQ ID NO:23366 |
| iPS:436732 | | NA | GACTATGGCATGCAC | GTTATATGGTATGATGG AAGTAATAAATACTATG CAGACTCCGTGAAGGGC | GATGACCGATCTTGTAGTAG TACCAGCTGCCCTTACTACT ACTACTACGGTTTGGACGTC |
| | 21-225_152B12 | | SEQ ID NO:7343 | SEQ ID NO:15355 | SEQ ID NO:23367 |
| | | AA | DYGMH | VIWYDGSNKYYADSVKG | DDRSCSSTSCPYYYYYGLDV |
| | | | SEQ ID NO:7344 | SEQ ID NO:15356 | SEQ ID NO:23368 |
| iPS:436734 | | NA | GACTATGGCATGCAC | CTTATATGGTATGATGGA AGTAATAAATACTATGC AGACTCCGTGAAGGGC | GATGACCGATCTTGTAGTAG AACCAGCTGCCCTTACTACT ACTACTACGGTTTGGACGTC |
| | 21-225_153A8 | | SEQ ID NO:7345 | SEQ ID NO:15357 | SEQ ID NO:23369 |
| | | AA | DYGMH | LIWYDGSNKYYADSVKG | DDRSCSRTSCPYYYYYGLDV |
| | | | SEQ ID NO:7346 | SEQ ID NO:15358 | SEQ ID NO:23370 |

FIGURE 49

| iPS:436736 | | NA | AACTATGGCATGCAC | GTTATATGGTTTGATGGC AGTAATAAATACTATGTT GACTCCGTGAAGGAC | GATCGGGTGGAGGGTTCGGG GACTCCCTACTACTACTACG GTATGGACGTC |
|---|---|---|---|---|---|
| | 21-225_153E8 | | SEQ ID NO:7347 | SEQ ID NO:15359 | SEQ ID NO:23371 |
| | | AA | NYGMH | VIWFDGSNKYYVDSVKD | DRVEGSGTPYYYYGMDV |
| | | | SEQ ID NO:7348 | SEQ ID NO:15360 | SEQ ID NO:23372 |
| iPS:436738 | | NA | ACCTATGGCATGCAC | GTTATATGGTATGGTGG AAGTAATAAAGACTATG CAGACTCCGTGAAGGGC | GATCGGGATTATTGTAGTGG TGGTAGCTGTCCTTACTACT ACTACTACGGTATGGACGTC |
| | 21-225_153D9 | | SEQ ID NO:7349 | SEQ ID NO:15361 | SEQ ID NO:23373 |
| | | AA | TYGMH | VIWYGGSNKDYADSVKG | DRDYCSGGSCPYYYYYGMDV |
| | | | SEQ ID NO:7350 | SEQ ID NO:15362 | SEQ ID NO:23374 |
| iPS:436740 | | NA | ACCTATGGCATGCAC | GTTGTATGGTATGGTGG AAATAATAAAGACTATG CAGACTCCGTGAAGGGC | GATCGGGATTATTGTAGTGG TGGTAGCTGCCCTTACTACT ACTACTACGGTATGGACGTC |
| | 21-225_154C3 | | SEQ ID NO:7351 | SEQ ID NO:15363 | SEQ ID NO:23375 |
| | | AA | TYGMH | VVWYGGNNKDYADSVK G | DRDYCSGGSCPYYYYYGMDV |
| | | | SEQ ID NO:7352 | SEQ ID NO:15364 | SEQ ID NO:23376 |
| iPS:436742 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_154C4 | | SEQ ID NO:7353 | SEQ ID NO:15365 | SEQ ID NO:23377 |
| | | AA | SYAMS | VISGGGSSTYYADSVKG | WRGNPTDYGMDV |
| | | | SEQ ID NO:7354 | SEQ ID NO:15366 | SEQ ID NO:23378 |

FIGURE 49

| iPS:436744 | | NA | AGCTATGGCATGCAC | GTTATATGGTATGATGG AAGTAATAAATACTATG CAGACTCCGTGAAGGGC | GAGGATTCCTATTGTAGTGG TACCAGCTGCCCCTACTACT ACTACTACGGTATGGACGTC |
|---|---|---|---|---|---|
| | 21-225_154F4 | | SEQ ID NO:7355 | SEQ ID NO:15367 | SEQ ID NO:23379 |
| | | AA | SYGMH | VIWYDGSNKYYADSVKG | EDSYCSGTSCPYYYYYGMDV |
| | | | SEQ ID NO:7356 | SEQ ID NO:15368 | SEQ ID NO:23380 |
| iPS:436746 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_154E10 | | SEQ ID NO:7357 | SEQ ID NO:15369 | SEQ ID NO:23381 |
| | | AA | SYAMS | VISGGGSSTYYADSVKG | WRGNPTDYGMDV |
| | | | SEQ ID NO:7358 | SEQ ID NO:15370 | SEQ ID NO:23382 |
| iPS:436748 | | NA | ACCTATGGCATGCAC | GTTATATGGTATGGTGG AAGTAATAAAGACTATG CAGACTCTGTGAAGGGC | GATCGGGATTATTGTAGTGG TGGTAGTTGCCCCTTACTACT ACTACTACGGTATGGACGTC |
| | 21-225_154D11 | | SEQ ID NO:7359 | SEQ ID NO:15371 | SEQ ID NO:23383 |
| | | AA | TYGMH | VIWYGGSNKDYADSVKG | DRDYCSGGSCPYYYYYGMDV |
| | | | SEQ ID NO:7360 | SEQ ID NO:15372 | SEQ ID NO:23384 |
| iPS:436750 | | NA | AGTGGTTATTACTACTGGAG C | TACATCTATTATAGTGGG AGCACCTACTACAACCC GTCCCTCAAGAGT | GATTGGGGGTGGCTACGATTC GAGTGGCTGGTTCGACCCC |
| | 21-225_154G12 | | SEQ ID NO:7361 | SEQ ID NO:15373 | SEQ ID NO:23385 |
| | | AA | SGYYYWS | YIYYSGSTYYNPSLKS | DWGGYDSSGWFDP |
| | | | SEQ ID NO:7362 | SEQ ID NO:15374 | SEQ ID NO:23386 |

FIGURE 49

| iPS:436752 | | NA | AGCTACTGGATCGGC | CTCATCTATCCTGGTGCC TCTGATACCAGATACAG CCCGTCCTTCCAAGGGC | CAGGCCATAGCAAGTCGAGG GAGGTACTACTACTACGGTA TGGACGTC |
|---|---|---|---|---|---|
| | 21-225_155H1 | | SEQ ID NO:7363 | SEQ ID NO:15375 | SEQ ID NO:23387 |
| | | AA | SYWIG | LIYPGASDTRYSPSFQG | QAIASRGRYYYYGMDV |
| | | | SEQ ID NO:7364 | SEQ ID NO:15376 | SEQ ID NO:23388 |
| iPS:436754 | | NA | AGCTATGGCATGCAC | GTTATATCATATGATGGA AGTAATAAATACTATGC AGACTCCGTGAAGGGC | GATACGGAGAGATGGCTACC ATACTCCTACGGTATGGACG TC |
| | 21-225_155G3 | | SEQ ID NO:7365 | SEQ ID NO:15377 | SEQ ID NO:23389 |
| | | AA | SYGMH | VISYDGSNKYYADSVKG | DTERWLPYSYGMDV |
| | | | SEQ ID NO:7366 | SEQ ID NO:15378 | SEQ ID NO:23390 |
| iPS:436756 | | NA | GGCTATGGCATGCAC | CTTATACGGTATGATGG AAGCGATAAAAACTATG CAGACTCCGTGAAGGGC | GATCGGGTTTTTTGTAGTAG TACCAGCTGCCTCTCTTACTA CTACTACTACGGTATGGACG TC |
| | 21-225_146A10 | | SEQ ID NO:7367 | SEQ ID NO:15379 | SEQ ID NO:23391 |
| | | AA | GYGMH | LIRYDGSDKNYADSVKG | DRVFCSSTSCLSYYYYYGMDV |
| | | | SEQ ID NO:7368 | SEQ ID NO:15380 | SEQ ID NO:23392 |
| iPS:436758 | | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | 21-225_155C10 | | SEQ ID NO:7369 | SEQ ID NO:15381 | SEQ ID NO:23393 |
| | | AA | SYAMS | VISGGGSSTYYADSVKG | WRGNPTDYGMDV |
| | | | SEQ ID NO:7370 | SEQ ID NO:15382 | SEQ ID NO:23394 |

FIGURE 49

| iPS:436760 | | NA | AGCTATGGCATGCAC | GTTATATGGTATGATGG AAGTAATAAATACTATG CAGACTCCGTGAAGGGC | GATCGTGACTACGGTGACCC CCCCTACTACTACTACTACG GTATGGACGTC |
|---|---|---|---|---|---|
| | 21-225_155E10 | | SEQ ID NO:7371 | SEQ ID NO:15383 | SEQ ID NO:23395 |
| | | AA | SYGMH | VIWYDGSNKYYADSVKG | DRDYGDPPYYYYYGMDV |
| | | | SEQ ID NO:7372 | SEQ ID NO:15384 | SEQ ID NO:23396 |
| iPS:436762 | | NA | AACTATAACATGAAC | TACATTAGTAGAAGTAG TAATACCAAATACTACG CAGACTCTGTGAAGGGC | GATAGGAGTGGGAGCTACGG GTACTTCTACTACTACGGTA TGGACGTC |
| | 21-225_156H2 | | SEQ ID NO:7373 | SEQ ID NO:15385 | SEQ ID NO:23397 |
| | | AA | NYNMN | YISRSSNTKYYADSVKG | DRSGSYGYFYYYGMDV |
| | | | SEQ ID NO:7374 | SEQ ID NO:15386 | SEQ ID NO:23398 |
| iPS:436764 | | NA | AGCTATGGCATGCAC | GTTATATGGTATGATGG AAGTAGTAAATACTATG CAGACTCCGTGAAGGGC | GATCGGGTTTTTTGTAGTGG TACCAGCTGCCCTTACTACT ACTACTACGGTATGGACGTC |
| | 21-225_158E9 | | SEQ ID NO:7375 | SEQ ID NO:15387 | SEQ ID NO:23399 |
| | | AA | SYGMH | VIWYDGSSKYYADSVKG | DRVFCSGTSCPYYYYYGMDV |
| | | | SEQ ID NO:7376 | SEQ ID NO:15388 | SEQ ID NO:23400 |
| iPS:436766 | | NA | AGCTATGGCATGCAC | GTTATATGGTATGATGG AAGTAATAAATACTATG CAGACTCCGTGAAGGGC | GATCGGGTTTCTTGTAGTAG TACCAGCTGCCCTTACTACT ACTACTACGGTATGGACGTC |
| | 21-225_158D10 | | SEQ ID NO:7377 | SEQ ID NO:15389 | SEQ ID NO:23401 |
| | | AA | SYGMH | VIWYDGSNKYYADSVKG | DRVSCSSTSCPYYYYYGMDV |

FIGURE 49

| | | | SEQ ID NO:7378 | SEQ ID NO:15390 | SEQ ID NO:23402 |
|---|---|---|---|---|---|
| iPS:436768 | | NA | ACCTATGGCATGCAC | GTTATATGGTATGATGG AAGTAATAAATACTATG CAGACTCCGTGAAGGGC | GATCGGGTTTCTTGTAGTAG TACCAGCTGCCCTTACTACT ACTACTACGGTATGGACGTC |
| | 21-225_159H8 | | SEQ ID NO:7379 | SEQ ID NO:15391 | SEQ ID NO:23403 |
| | | AA | TYGMH | VIWYDGSNKYYADSVKG | DRVSCSSTSCPYYYYYGMDV |
| | | | SEQ ID NO:7380 | SEQ ID NO:15392 | SEQ ID NO:23404 |
| iPS:436770 | | NA | AGCTATGGCATGCAC | GTTATATGGTATGATGG AAGTAATAAATACTATG CAGACTCCGTGAAGGGC | GATCGGGTTTCTTGTAGTAG TACCAGCTGCCCTTACTACT ACTACTACGGTATGGACGTC |
| | 21-225_160B12 | | SEQ ID NO:7381 | SEQ ID NO:15393 | SEQ ID NO:23405 |
| | | AA | SYGMH | VIWYDGSNKYYADSVKG | DRVSCSSTSCPYYYYYYGMDV |
| | | | SEQ ID NO:7382 | SEQ ID NO:15394 | SEQ ID NO:23406 |
| iPS:436772 | | NA | AGCTATGGCATGCAC | GTTATATGGTATGATGG AAGTAATAAATACTATG CAGACTCCGTGAAGGGC | GTCGGGTATAGCGGTGGCTG GTACATCTTTGACTAC |
| | 21-225_161H3 | | SEQ ID NO:7383 | SEQ ID NO:15395 | SEQ ID NO:23407 |
| | | AA | SYGMH | VIWYDGSNKYYADSVKG | VGYSGGWYIFDY |
| | | | SEQ ID NO:7384 | SEQ ID NO:15396 | SEQ ID NO:23408 |
| iPS:436774 | | NA | AGCTATGGCATGCAC | GTTATATGGTATGATGG AAGTAATAAATACTATG TAGACTCCGTGAAGGGC | GATCGGGTTTTTTGTAGTGG TACCAGCTGCCCTTACTACT ACTACTACGGTATGGACGTC |
| | 21-225_161E10 | | SEQ ID NO:7385 | SEQ ID NO:15397 | SEQ ID NO:23409 |

FIGURE 49

| | | | | | |
|---|---|---|---|---|---|
| | | AA | SYGMH | VIWYDGSNKYYVDSVKG | DRVFCSGTSCPYYYYYGMDV |
| | | | SEQ ID NO:7386 | SEQ ID NO:15398 | SEQ ID NO:23410 |
| iPS:436776 | 21-225_161F12 | NA | AGTGGTGGTTACTACTGGAGC | TACATCTATTACAGTGGGAGCCCCTACTACAACCCGTCCCTCAAGAGT | TCGAATTGTAGTAGTGCCAACTGCTATACGGTGGGGTTCTACTACTACGGTTTGGACGTC |
| | | | SEQ ID NO:7387 | SEQ ID NO:15399 | SEQ ID NO:23411 |
| | | AA | SGGYYWS | YIYYSGSPYYNPSLKS | SNCSSANCYTVGFYYYGLDV |
| | | | SEQ ID NO:7388 | SEQ ID NO:15400 | SEQ ID NO:23412 |
| iPS:436780 | 21-225_165H3 | NA | AGTGGTGGTTACTACTGGAGC | TACATCTATTACAGTGGGAGCCCCTACTACAATCCGTCCCTCAAGAGT | TCGAATTGTAGTAGTGCCAACTGCTATACGGTGGGGTTCTACTACTACGGTATGGACGTC |
| | | | SEQ ID NO:7389 | SEQ ID NO:15401 | SEQ ID NO:23413 |
| | | AA | SGGYYWS | YIYYSGSPYYNPSLKS | SNCSSANCYTVGFYYYGMDV |
| | | | SEQ ID NO:7390 | SEQ ID NO:15402 | SEQ ID NO:23414 |
| iPS:436782 | 21-225_166G11 | NA | GGCTATGGCATGCAC | GTTATATGGTATGATGGAAGTAATAAATACTATGCAGACTCCGTGAAGGGC | GATGATAGATATTGTAGTAGTCCCACCTGCCATCCTTACTACTACTACGGTCTGGACGTC |
| | | | SEQ ID NO:7391 | SEQ ID NO:15403 | SEQ ID NO:23415 |
| | | AA | GYGMH | VIWYDGSNKYYADSVKG | DDRYCSSPTCHPYYYYYGLDV |
| | | | SEQ ID NO:7392 | SEQ ID NO:15404 | SEQ ID NO:23416 |

FIGURE 49

| iPS:436784 | | NA | AGCAATGGCATGCAC | GTTATATGGTATGATGG AAGTAATAAATACTATG CAGACTCCGTGAAGGGC | GATCAGTACAACCGGAACGA CGGACCACCAGCTTACTACT ACTACTACGGTTTGGACGTC |
|---|---|---|---|---|---|
| | 21-225_169C1 | | SEQ ID NO:7393 | SEQ ID NO:15405 | SEQ ID NO:23417 |
| | | AA | SNGMH | VIWYDGSNKYYADSVKG | DQYNRNDGPPAYYYYYGLDV |
| | | | SEQ ID NO:7394 | SEQ ID NO:15406 | SEQ ID NO:23418 |
| iPS:436786 | | NA | AGCAATGGCATGCAC | GTTATATGGTATGATGG AAGTAATAAATACTATG CAGACTCCGTGAAGGGC | GATCAGTACAACCGGAACGA CGGACCACCAGCTTACTACT ACTACTACGGTATGGACGTC |
| | 21-225_169A6 | | SEQ ID NO:7395 | SEQ ID NO:15407 | SEQ ID NO:23419 |
| | | AA | SNGMH | VIWYDGSNKYYADSVKG | DQYNRNDGPPAYYYYYGMD V |
| | | | SEQ ID NO:7396 | SEQ ID NO:15408 | SEQ ID NO:23420 |
| iPS:436788 | | NA | AGCTATAGCTTGAAC | TACATTGGTAGTAGTGG CAGTATCATATTCTACGC AGACTCTGTGAAGGGC | GGGGATACAGCTGGGGTTAC CTATTACTACGGTATGGACG TC |
| | 21-225_169B7 | | SEQ ID NO:7397 | SEQ ID NO:15409 | SEQ ID NO:23421 |
| | | AA | SYSLN | YIGSSGSIIFYADSVKG | GDTAGVTYYYGMDV |
| | | | SEQ ID NO:7398 | SEQ ID NO:15410 | SEQ ID NO:23422 |
| iPS:436790 | | NA | AGCTATGGCATGCAC | ATTATATGGTATGATGG AAGTAATAAATACTATG CAGACTCCGTGAAGGGC | GAGGGGGCTACGTATTACCA TGGTTCGGGGAGTTATTATC CGGCTACTAACTACGGTATG GACGTC |
| | 21-225_169G11 | | SEQ ID NO:7399 | SEQ ID NO:15411 | SEQ ID NO:23423 |
| | | AA | SYGMH | IIWYDGSNKYYADSVKG | EGATYYHGSGSYYPATNYGM DV |
| | | | SEQ ID NO:7400 | SEQ ID NO:15412 | SEQ ID NO:23424 |

FIGURE 49

| iPS:436792 | | NA | AGCTATGGCATGCAC | GTTATATGGTATGATGG AAGTAATAAATATTATG CAGACTCCGTGAAGGGC | CCCCTTTACGATATGGGACT CTACTACGATATGGACGTC |
|---|---|---|---|---|---|
| | 21-225_169D12 | | SEQ ID NO:7401 | SEQ ID NO:15413 | SEQ ID NO:23425 |
| | | AA | SYGMH | VIWYDGSNKYYADSVKG | PLYDMGLYYDMDV |
| | | | SEQ ID NO:7402 | SEQ ID NO:15414 | SEQ ID NO:23426 |
| iPS:436794 | | NA | GGCTATGGCATGAAC | ATTATATGGTATGATGG AAATAATAAATACTATG CAGACTCCGTGAAGGGC | GATCGGGTTTATTGTAGTAG TACCAGCTGCCATCCCTATT ACTACTACTACGCTATGGAC GTC |
| | 21-225_170F1 | | SEQ ID NO:7403 | SEQ ID NO:15415 | SEQ ID NO:23427 |
| | | AA | GYGMN | IIWYDGNNKYYADSVKG | DRVYCSSTSCHPYYYYYAMD V |
| | | | SEQ ID NO:7404 | SEQ ID NO:15416 | SEQ ID NO:23428 |
| iPS:436796 | | NA | AACTGTGGCATGCAC | ATTATATGGTATGATGG AAGTAATAAATACTATG CAGACTCCGTGAAGGGC | GATCAGTACAACAGGAACGA CGGACCACCAGCTTACTACT ACTACTACGGTTTGGACGTC |
| | 21-225_170A5 | | SEQ ID NO:7405 | SEQ ID NO:15417 | SEQ ID NO:23429 |
| | | AA | NCGMH | IIWYDGSNKYYADSVKG | DQYNRNDGPPAYYYYYGLDV |
| | | | SEQ ID NO:7406 | SEQ ID NO:15418 | SEQ ID NO:23430 |
| iPS:436798 | | NA | AGCTATAGCTTGAAC | TACATTGGTAGTAGTGG CAGTATCATATTCTACGC AGACTCTGTGAAGGGC | GGGGATACAGCTGGGGTTAC CTATTACTACGGTATGGACG TC |
| | 21-225_171F5 | | SEQ ID NO:7407 | SEQ ID NO:15419 | SEQ ID NO:23431 |
| | | AA | SYSLN | YIGSSGSIIFYADSVKG | GDTAGVTYYYGMDV |
| | | | SEQ ID NO:7408 | SEQ ID NO:15420 | SEQ ID NO:23432 |

FIGURE 49

| iPS:436800 | | NA | AGTTACTATATGTAT | ATAATCAACCCTAGTGG TGGTAGCACAAACTACG CACAGAAGTTCCAGGGC | GGTTGGGAGTTAAACTAC |
|---|---|---|---|---|---|
| | 21-225_171D12 | | SEQ ID NO:7409 | SEQ ID NO:15421 | SEQ ID NO:23433 |
| | | AA | SYYMY | IINPSGGSTNYAQKFQG | GWELNY |
| | | | SEQ ID NO:7410 | SEQ ID NO:15422 | SEQ ID NO:23434 |
| iPS:436802 | | NA | AGTTATGGCATGCAC | GTTATATGGAATGATGG AGGTAATAAATATAATG GAGACTCCGTGAAGGGC | GACCGTACGTATTACTCTGG TTCGGGGAGCCCCCCCTACT ACTACTACTACGGTATGGAC GTC |
| | 21-225_171E12 | | SEQ ID NO:7411 | SEQ ID NO:15423 | SEQ ID NO:23435 |
| | | AA | SYGMH | VIWNDGGNKYNGDSVKG | DRTYYSGSGSPPYYYYYYGMD V |
| | | | SEQ ID NO:7412 | SEQ ID NO:15424 | SEQ ID NO:23436 |
| iPS:436804 | | NA | AGTTACTATATGTAT | ACAATCAACCCTAGTGG TGGTAGCACAAACTACG CACAGAAGTTCCAGGGC | GGCTGGGAGTTAAACTAC |
| | 21-225_172C3 | | SEQ ID NO:7413 | SEQ ID NO:15425 | SEQ ID NO:23437 |
| | | AA | SYYMY | TINPSGGSTNYAQKFQG | GWELNY |
| | | | SEQ ID NO:7414 | SEQ ID NO:15426 | SEQ ID NO:23438 |
| iPS:436806 | | NA | AGTTACTATATGTAT | ACAATCAACCCTAGTGG TGGTAGCACAGACTACG CACAGAAGTTCCAGGGC | GGCTGGGAATTAAACTAC |
| | 21-225_172B12 | | SEQ ID NO:7415 | SEQ ID NO:15427 | SEQ ID NO:23439 |
| | | AA | SYYMY | TINPSGGSTDYAQKFQG | GWELNY |
| | | | SEQ ID NO:7416 | SEQ ID NO:15428 | SEQ ID NO:23440 |
| iPS:436808 | | NA | AGCTATGGCATGCAC | GTTATATCATATGATGGA AGTCCTAAATACTGTGC AGACTCCGTGAAGGGC | GATGAAAGGCAGTGGCTGCC GGCCCCCTACGGTATGGACG TC |
| | 21-225_173F8 | | SEQ ID NO:7417 | SEQ ID NO:15429 | SEQ ID NO:23441 |

FIGURE 49

| | | AA | SYGMH | VISYDGSPKYCADSVKG | DERQWLPAPYGMDV |
|---|---|---|---|---|---|
| | | | SEQ ID NO:7418 | SEQ ID NO:15430 | SEQ ID NO:23442 |
| iPS:436810 | 21-225_175F4 | NA | AGCAACAGTGCTGCTTGGAAC | AGGACATACTACAGGTCCAAGTGGTATAATGCTTATCCAGTATCTATGGAAAGT | GATAAGGCAGCTGGGAGGAATGACTTCTACTACTACGGTATGGACGTC |
| | | | SEQ ID NO:7419 | SEQ ID NO:15431 | SEQ ID NO:23443 |
| | | AA | SNSAAWN | RTYYRSKWYNAYPVSMES | DKAAGRNDFYYYGMDV |
| | | | SEQ ID NO:7420 | SEQ ID NO:15432 | SEQ ID NO:23444 |
| iPS:436812 | 21-225_175C6 | NA | AACTGTGGCATGCAC | ATTATATGGTATGATGGAAGTAATAAATACTATGCAGACTCCGTGAAGGGC | GATCAGTACAACAGGAACGACGGACCACCAGCTTACTACTACTACTACGGTTTGGACGTC |
| | | | SEQ ID NO:7421 | SEQ ID NO:15433 | SEQ ID NO:23445 |
| | | AA | NCGMH | IIWYDGSNKYYADSVKG | DQYNRNDGPPAYYYYYGLDV |
| | | | SEQ ID NO:7422 | SEQ ID NO:15434 | SEQ ID NO:23446 |
| iPS:436814 | 21-225_178H10 | NA | AGCAACAGTGCTGCTTGGAAC | AGGACATACTACAGGTCCAAGTGGTATAGTGCTTATCCAGTATCTATGGAAAGT | GATAAGGCAGCTGGGAGGAATGACTTCTACTACTACGGTATGGACGTC |
| | | | SEQ ID NO:7423 | SEQ ID NO:15435 | SEQ ID NO:23447 |
| | | AA | SNSAAWN | RTYYRSKWYSAYPVSMES | DKAAGRNDFYYYGMDV |
| | | | SEQ ID NO:7424 | SEQ ID NO:15436 | SEQ ID NO:23448 |
| iPS:436816 | 21-225_179H5 | NA | AGCTATGGCATGCAC | GTTATATGGTATGATGGAAGTAATGAATACTATGCAGACTCCGTGAAGGGC | GATATCCGGAACTACTACTACGGTTTGGACGTC |
| | | | SEQ ID NO:7425 | SEQ ID NO:15437 | SEQ ID NO:23449 |

FIGURE 49

| | | AA | SYGMH | VIWYDGSNEYYADSVKG | DIRNYYYGLDV |
|---|---|---|---|---|---|
| | | | SEQ ID NO:7426 | SEQ ID NO:15438 | SEQ ID NO:23450 |
| iPS:436818 | | NA | AACTCTGGCATGCAC | ATTATATATTATGATGGA AGTTATAAATACAATGC AGACTCCGTGAAGGGC | GACCGTCATTACGATTTCCA CGTTCCCTACTATTACTATTA CGGTATGGACGTC |
| | 21-225_179C7 | | SEQ ID NO:7427 | SEQ ID NO:15439 | SEQ ID NO:23451 |
| | | AA | NSGMH | IIYYDGSYKYNADSVKG | DRHYDFHVPYYYYYGMDV |
| | | | SEQ ID NO:7428 | SEQ ID NO:15440 | SEQ ID NO:23452 |
| iPS:436820 | | NA | AGCTATAGCATGAAC | TACATTAGTAGTAGTGG AAGTACCACATACTACG CAGACTCTGTGCAGGGC | GATAGTAGGAAGGGGTTCTA CTACGGTCTGGACGTC |
| | 21-225_179D10 | | SEQ ID NO:7429 | SEQ ID NO:15441 | SEQ ID NO:23453 |
| | | AA | SYSMN | YISSSGSTTYYADSVQG | DSRKGFYYGLDV |
| | | | SEQ ID NO:7430 | SEQ ID NO:15442 | SEQ ID NO:23454 |
| iPS:436822 | | NA | AACTTTGGCATGCAC | ATTATATGGTATGATGG AAGTGATAAATACTATG CAGACTCCGTGAAGGGC | GGGGGGGCCCCCGTTCTCTAC GGTGACTATGTACTTTGACT AC |
| | 21-225_180D4 | | SEQ ID NO:7431 | SEQ ID NO:15443 | SEQ ID NO:23455 |
| | | AA | NFGMH | IIWYDGSDKYYADSVKG | GGPPFSTVTMYFDY |
| | | | SEQ ID NO:7432 | SEQ ID NO:15444 | SEQ ID NO:23456 |
| iPS:436824 | | NA | ACTAGTGGAGTGGGTGTGGG C | TTCATTTCTTGGAATGAT GATAAGCGCTACAGCCC ATCTCTGAAGAGC | AAAGCAGCAGCTGTTGCTTT TGATATC |
| | 21-225_180C5 | | SEQ ID NO:7433 | SEQ ID NO:15445 | SEQ ID NO:23457 |
| | | AA | TSGVGVG | FISWNDDKRYSPSLKS | KAAAVAFDI |
| | | | SEQ ID NO:7434 | SEQ ID NO:15446 | SEQ ID NO:23458 |

FIGURE 49

| iPS:436826 | | NA | AGTTATGATATCAAC | TGGATGAACCCTAACAG TGGTAACACAGGCTATG CACAGAAGTTCCAGGGC | GGGTTTTATTACTATGGTTCG GGGAGTCATGTCCCTTACCA CTACTACTACGGTTTGGACG TC |
|---|---|---|---|---|---|
| | 21-225_180G5 | | SEQ ID NO:7435 | SEQ ID NO:15447 | SEQ ID NO:23459 |
| | | AA | SYDIN | WMNPNSGNTGYAQKFQG | GFYYYGSGSHVPYHYYYGLD V |
| | | | SEQ ID NO:7436 | SEQ ID NO:15448 | SEQ ID NO:23460 |
| iPS:436828 | | NA | AACTATGGCATGCAC | ATTATATGGTATGATGG AAGTGATAAATACTATG CAGACTCCGTGAAGGGC | GGGGGGGCCCCCGTTTTCTAC GGTGACTATGTACTTCGACT AC |
| | 21-225_181H1 | | SEQ ID NO:7437 | SEQ ID NO:15449 | SEQ ID NO:23461 |
| | | AA | NYGMH | IIWYDGSDKYYADSVKG | GGPPFSTVTMYFDY |
| | | | SEQ ID NO:7438 | SEQ ID NO:15450 | SEQ ID NO:23462 |
| iPS:436830 | | NA | AGCTATGGTATCAGC | TGGATCAGCGCTTATAAT GGTAACACAAAGTATGC ACAGAAGCTCCAGGGC | CACGATTTTTGGAGTGGTTA TTATAAGGGTATGGACGTC |
| | 21-225_51F4 | | SEQ ID NO:7439 | SEQ ID NO:15451 | SEQ ID NO:23463 |
| | | AA | SYGIS | WISAYNGNTKYAQKLQG | HDFWSGYYKGMDV |
| | | | SEQ ID NO:7440 | SEQ ID NO:15452 | SEQ ID NO:23464 |
| iPS:436832 | | NA | AGTAACAGTGCTGCTTGGAA C | AGGACATACTACAGGTC CAAGTGGTATAATGATT ATGCAGTATCTGTGAAA AGT | GACCGCTATAACTGGAACTA CCCCTACTGGTACTTCGATCT C |
| | 21-225_51D8 | | SEQ ID NO:7441 | SEQ ID NO:15453 | SEQ ID NO:23465 |
| | | AA | SNSAAWN | RTYYRSKWYNDYAVSVK S | DRYNWNYPYWYFDL |
| | | | SEQ ID NO:7442 | SEQ ID NO:15454 | SEQ ID NO:23466 |

FIGURE 49

| iPS:436834 | | NA | AGCTATGGTGTCAGC | TGGATCAGCGCTTATAAT GGTAACAGAAAGTATGC ACAGAAGCTCCAGGGC | CACGATTTTTGGAGTGGTTA TTATAAGGGTATGGACGTC |
|---|---|---|---|---|---|
| | 21-225_52F1 | | SEQ ID NO:7443 | SEQ ID NO:15455 | SEQ ID NO:23467 |
| | | AA | SYGVS | WISAYNGNRKYAQKLQG | HDFWSGYYKGMDV |
| | | | SEQ ID NO:7444 | SEQ ID NO:15456 | SEQ ID NO:23468 |
| iPS:436836 | | NA | GGCTATGGCATGCAC | GTTATATGGTATGATGG AAGTAATAAATACTATG CAGACTCCGTGAAGGGC | GATCGGGTCTATTGTAGTAG TTCCAGCTGCTCATATTACTA CTACTACTACGGTATGGACG TC |
| | 21-225_52H1 | | SEQ ID NO:7445 | SEQ ID NO:15457 | SEQ ID NO:23469 |
| | | AA | GYGMH | VIWYDGSNKYYADSVKG | DRVYCSSSSCSYYYYYYGMD V |
| | | | SEQ ID NO:7446 | SEQ ID NO:15458 | SEQ ID NO:23470 |
| iPS:436838 | | NA | CACTTTGGCATGCAC | GTTATTTGGTATGATGGA AGTAATAAATACTATGC AGACTCCGTGAAGGGC | GGGGACTGGAACTACGAGGG TTTTGACTAC |
| | 21-225_52H4 | | SEQ ID NO:7447 | SEQ ID NO:15459 | SEQ ID NO:23471 |
| | | AA | HFGMH | VIWYDGSNKYYADSVKG | GDWNYEGFDY |
| | | | SEQ ID NO:7448 | SEQ ID NO:15460 | SEQ ID NO:23472 |
| iPS:436840 | | NA | GGCTACTATATGCAC | TGGATCATCCCTAACAGT GGTGACACAAACTATGC ACAGAAGTTTCAGGGC | GATGGGTATAGCAGTGGCTG GTTCAACTGGTTCGACCCC |
| | 21-225_53E9 | | SEQ ID NO:7449 | SEQ ID NO:15461 | SEQ ID NO:23473 |
| | | AA | GYYMH | WIIPNSGDTNYAQKFQG | DGYSSGWFNWFDP |
| | | | SEQ ID NO:7450 | SEQ ID NO:15462 | SEQ ID NO:23474 |

FIGURE 49

| iPS:436842 | | NA | AGCTATGGTATCAGC | TGGATTAGTGCTTATAATGGTAACACAAAGAATGCACAGAAGCTCCAGGGC | CACGATTTTTGGAGTGGTTATTATAAGGGTATGGACGTC |
|---|---|---|---|---|---|
| | 21-225_54E9 | | SEQ ID NO:7451 | SEQ ID NO:15463 | SEQ ID NO:23475 |
| | | AA | SYGIS | WISAYNGNTKNAQKLQG | HDFWSGYYKGMDV |
| | | | SEQ ID NO:7452 | SEQ ID NO:15464 | SEQ ID NO:23476 |
| iPS:436844 | | NA | AGCTATGGTATCAGC | TGGATCAGCGCTTATAATGGTAACACAAAGTATGCACAGAAGTTCCAGGGC | CACGATTTTTGGAGTGGTTATTATAAGGGTATGGACGTC |
| | 21-225_56G1 | | SEQ ID NO:7453 | SEQ ID NO:15465 | SEQ ID NO:23477 |
| | | AA | SYGIS | WISAYNGNTKYAQKFQG | HDFWSGYYKGMDV |
| | | | SEQ ID NO:7454 | SEQ ID NO:15466 | SEQ ID NO:23478 |
| iPS:436846 | | NA | AGCTATGGTTTCAGC | TGGATCAGCGCTTATAATGGTAACACAAAGGAAGCACAGAAGTTCCAGGGC | CACGATTTTTGGAGTGGTTATTATAAGGGTATGGACGTC |
| | 21-225_56E3 | | SEQ ID NO:7455 | SEQ ID NO:15467 | SEQ ID NO:23479 |
| | | AA | SYGFS | WISAYNGNTKEAQKFQG | HDFWSGYYKGMDV |
| | | | SEQ ID NO:7456 | SEQ ID NO:15468 | SEQ ID NO:23480 |
| iPS:436848 | | NA | ACTAGTGGAGTGGGTGTGGGC | CTCATTTATTGGCATGAAGATAAGCGCTACAGCCCATCTCTGAAGAGC | GTCACAGGTATAGCAGCTCCCTAC |
| | 21-225_57F1 | | SEQ ID NO:7457 | SEQ ID NO:15469 | SEQ ID NO:23481 |
| | | AA | TSGVGVG | LIYWHEDKRYSPSLKS | VTGIAAPY |
| | | | SEQ ID NO:7458 | SEQ ID NO:15470 | SEQ ID NO:23482 |

FIGURE 49

| iPS:436850 | | NA | ACTAGTGGAGTGGGTGTGGGC | CTCATTTATTGGAATGATGATAAGCGCTACAGTCCATCTCTGAAGAGC | GCAGTGGCTGTCTCCTTTGACTAC |
|---|---|---|---|---|---|
| | 21-225_57D9 | | SEQ ID NO:7459 | SEQ ID NO:15471 | SEQ ID NO:23483 |
| | | AA | TSGVGVG | LIYWNDDKRYSPSLKS | AVAVSFDY |
| | | | SEQ ID NO:7460 | SEQ ID NO:15472 | SEQ ID NO:23484 |
| iPS:436852 | | NA | ACTAGTGGAGTGGGTGTGGGC | CTCATTTATTGGCATGAAGATAGGCGCTACAGCCCATCTCTGAAGAGC | GTCACAGGTATAGCAGCTCCCTAC |
| | 21-225_57H11 | | SEQ ID NO:7461 | SEQ ID NO:15473 | SEQ ID NO:23485 |
| | | AA | TSGVGVG | LIYWHEDRRYSPSLKS | VTGIAAPY |
| | | | SEQ ID NO:7462 | SEQ ID NO:15474 | SEQ ID NO:23486 |
| iPS:436854 | | NA | ACTAGTGGAGTGGGTGTGGGC | CTCATTTATTGGGATGATGATAAGCGCTACAGCCCATCTCTGAAGAGC | CTTATAGCAGTGGCCTTTGACTCC |
| | 21-225_58C1 | | SEQ ID NO:7463 | SEQ ID NO:15475 | SEQ ID NO:23487 |
| | | AA | TSGVGVG | LIYWDDDKRYSPSLKS | LIAVAFDS |
| | | | SEQ ID NO:7464 | SEQ ID NO:15476 | SEQ ID NO:23488 |
| iPS:436856 | | NA | AGCTATAGCATGAAC | TCCATTAGTAGTAGTAGTTATTACTTATACTACGCAGACTCAGTGAAGGGC | ACCTATAGTGGGAGTTTTGACTAC |
| | 21-225_58C5 | | SEQ ID NO:7465 | SEQ ID NO:15477 | SEQ ID NO:23489 |
| | | AA | SYSMN | SISSSSYYLYYADSVKG | TYSGSFDY |
| | | | SEQ ID NO:7466 | SEQ ID NO:15478 | SEQ ID NO:23490 |
| iPS:436858 | | NA | TTCTATGGCATGCAC | GTTACATCATATGATGGAAGTGATAAATACTATGCAGACTCCGTGAAGGGC | GATGACTATGGTTCGGGGAGTCCCCTATACTACGGTATGGACGTC |
| | 21-225_58E7 | | SEQ ID NO:7467 | SEQ ID NO:15479 | SEQ ID NO:23491 |
| | | AA | FYGMH | VTSYDGSDKYYADSVKG | DDYGSGSPLYYGMDV |

FIGURE 49

| | | | SEQ ID NO:7468 | SEQ ID NO:15480 | SEQ ID NO:23492 |
|---|---|---|---|---|---|
| iPS:436860 | | NA | AGCTTTTGGATGAGC | CACATAAAGCAAGATGGAAGTGAGAAATACTATGTGGACTCTGTGAAGGGC | GGGGACCTCCCATACAGCTCGGGCTACTACTACGGTATGGACGTC |
| | 21-225_58F7 | | SEQ ID NO:7469 | SEQ ID NO:15481 | SEQ ID NO:23493 |
| | | AA | SFWMS | HIKQDGSEKYYVDSVKG | GDLPYSSGYYYGMDV |
| | | | SEQ ID NO:7470 | SEQ ID NO:15482 | SEQ ID NO:23494 |
| iPS:436862 | | NA | AGCTATGGCATGCAC | GTTATATCATATGATGGAAGTAATAAATACTATGCAGACTCCGTGAAGGGC | GATGAGGGACGTGGATATGGTGGCTACGAGAGGGGATATTACTACTACTACGGTATGGACGTC |
| | 21-225_58F8 | | SEQ ID NO:7471 | SEQ ID NO:15483 | SEQ ID NO:23495 |
| | | AA | SYGMH | VISYDGSNKYYADSVKG | DEGRGYGGYERGYYYYYYGMDV |
| | | | SEQ ID NO:7472 | SEQ ID NO:15484 | SEQ ID NO:23496 |
| iPS:436864 | | NA | AGCTATAGCATGAAC | TACATTAGTACTAGTAGTAGTACCATATTCTACGCAGACTCTGTGAAGGGC | GGGGATACAGCTATGGTCCTCTACTACTACGGTATGGACGTC |
| | 21-225_58G11 | | SEQ ID NO:7473 | SEQ ID NO:15485 | SEQ ID NO:23497 |
| | | AA | SYSMN | YISTSSSTIFYADSVKG | GDTAMVLYYYGMDV |
| | | | SEQ ID NO:7474 | SEQ ID NO:15486 | SEQ ID NO:23498 |
| iPS:436866 | | NA | AGCTATAGCATGAAC | TACATTAGTGGGAGTAGTAATATCATATACTACACAGACTCTGTGAAGGGC | GCGGATACACCTATGGTCCTTTACTTCTACGGTATGGACGTC |
| | 21-225_59F2 | | SEQ ID NO:7475 | SEQ ID NO:15487 | SEQ ID NO:23499 |
| | | AA | SYSMN | YISGSSNIIYYTDSVKG | ADTPMVLYFYGMDV |
| | | | SEQ ID NO:7476 | SEQ ID NO:15488 | SEQ ID NO:23500 |

FIGURE 49

| iPS:436868 | | NA | AGTTATGGCGTGCAC | GCTATATGGTATGATGG AAGTAATAAATACTATG CAGACTCCGTGAAGGGC | GATAGGGACTATTGTAGTAG TTCCAGCTGCCCTTACTACTA CTACTACGGTATGGACGTC |
|---|---|---|---|---|---|
| | 21-225_59B11 | | SEQ ID NO:7477 | SEQ ID NO:15489 | SEQ ID NO:23501 |
| | | AA | SYGVH | AIWYDGSNKYYADSVKG | DRDYCSSSSCPYYYYYGMDV |
| | | | SEQ ID NO:7478 | SEQ ID NO:15490 | SEQ ID NO:23502 |
| iPS:436870 | | NA | ACTAGTGGAGTGGGTGTGGG C | CTCATTTATTGGCATGAA GATAAGCGCTACAGCCC ATCTCTGAAGAGC | GTCACATATATAGCAGCTCC CTAC |
| | 21-225_60B1 | | SEQ ID NO:7479 | SEQ ID NO:15491 | SEQ ID NO:23503 |
| | | AA | TSGVGVG | LIYWHEDKRYSPSLKS | VTYIAAPY |
| | | | SEQ ID NO:7480 | SEQ ID NO:15492 | SEQ ID NO:23504 |
| iPS:436872 | | NA | AGCTATAGCATGAAC | TACATTAGTGAGAGTAG TAATATCATATACTACAC AGACTCTGTGAAGGGC | GCGGATACACCTATGGTCCT TTACTTCTACGGTATGGACG TC |
| | 21-225_60D2 | | SEQ ID NO:7481 | SEQ ID NO:15493 | SEQ ID NO:23505 |
| | | AA | SYSMN | YISESSNIIYYTDSVKG | ADTPMVLYFYGMDV |
| | | | SEQ ID NO:7482 | SEQ ID NO:15494 | SEQ ID NO:23506 |
| iPS:436874 | | NA | ACTAGTGGAGTGGGTGTGGG C | CTCATTTATTGGGATGAT GATAAGCGCTACAGCCC ATCTCTGAAGAGC | CTTATAGCAGTGGCCTTTGA CTCC |
| | 21-225_60A12 | | SEQ ID NO:7483 | SEQ ID NO:15495 | SEQ ID NO:23507 |
| | | AA | TSGVGVG | LIYWDDDKRYSPSLKS | LIAVAFDS |
| | | | SEQ ID NO:7484 | SEQ ID NO:15496 | SEQ ID NO:23508 |
| iPS:436876 | | NA | ACTAGTGGGTTGGGTGTGGG C | CTCATTTATTCACATGAA GATAAGCGCTACAGCCC ATCTCTGAAGAGC | GTCACAGGTATAGCAGCTCC CTAC |
| | 21-225_61F5 | | SEQ ID NO:7485 | SEQ ID NO:15497 | SEQ ID NO:23509 |

FIGURE 49

| | | | | | |
|---|---|---|---|---|---|
| | | AA | TSGLGVG | LIYSHEDKRYSPSLKS | VTGIAAPY |
| | | | SEQ ID NO:7486 | SEQ ID NO:15498 | SEQ ID NO:23510 |
| iPS:436878 | 21-225_62E3 | NA | ACTAGTGGAGTGGGTGTGGGC | CTCATTAATTGGAATGATGATAAGCGCTACAGCCCATCTCTGAAGAGC | AAAGCTACCTGGGTGGCTTTTGATATC |
| | | | SEQ ID NO:7487 | SEQ ID NO:15499 | SEQ ID NO:23511 |
| | | AA | TSGVGVG | LINWNDDKRYSPSLKS | KATWVAFDI |
| | | | SEQ ID NO:7488 | SEQ ID NO:15500 | SEQ ID NO:23512 |
| iPS:436880 | 21-225_62E8 | NA | ACTAGTGGAGTGGGTGTGGGC | CTCATTAATTGGAATGATGATAAGCGCTACAGCCCATCTCTGAAGAGC | AAAGCTACCTGGGTGGCTTTTGATATC |
| | | | SEQ ID NO:7489 | SEQ ID NO:15501 | SEQ ID NO:23513 |
| | | AA | TSGVGVG | LINWNDDKRYSPSLKS | KATWVAFDI |
| | | | SEQ ID NO:7490 | SEQ ID NO:15502 | SEQ ID NO:23514 |
| iPS:436882 | 21-225_62D10 | NA | ACTAGTGGAGTGGGTGTGGGC | CTCATTAATTGGAATGATGATAAGCGCTACAGCCCATCTCTGAAGAGC | AAAGCTACCTGGGTGGCTTTTGATATC |
| | | | SEQ ID NO:7491 | SEQ ID NO:15503 | SEQ ID NO:23515 |
| | | AA | TSGVGVG | LINWNDDKRYSPSLKS | KATWVAFDI |
| | | | SEQ ID NO:7492 | SEQ ID NO:15504 | SEQ ID NO:23516 |
| iPS:436884 | 21-225_62A12 | NA | ACTAGTGGAGTGGGTGTGGGC | CTCATTAATTGGAATGATGATAAACGCTACAGCCCATCTCTGAAGAGC | AAAACTACCTGGGTGGCTTTTGATATC |
| | | | SEQ ID NO:7493 | SEQ ID NO:15505 | SEQ ID NO:23517 |
| | | AA | TSGVGVG | LINWNDDKRYSPSLKS | KTTWVAFDI |
| | | | SEQ ID NO:7494 | SEQ ID NO:15506 | SEQ ID NO:23518 |
| iPS:436886 | 21-225_62B12 | NA | ACTAGTGGAGTGGGTGTGGGC | CTCATTAATTGGAATGATGATAAGCGCTACAGCCCGTCTCTGAAGAGC | AAAGCTACCTGGGTGGCTTTTGATATC |
| | | | SEQ ID NO:7495 | SEQ ID NO:15507 | SEQ ID NO:23519 |

FIGURE 49

| | | | AA | TSGVGVG | LINWNDDKRYSPSLKS | KATWVAFDI |
|---|---|---|---|---|---|---|
| | | | | SEQ ID NO:7496 | SEQ ID NO:15508 | SEQ ID NO:23520 |
| iPS:436888 | 21-225_63G7 | | NA | AGCTATAGCATGAAC | TACATTAGTAGTAGTACT AGTACCATATACTACGC AGCCTCTGTGAAGGGC | GATCACCGTTACTATGATAG TAGTGGTTATTACTCTGATG CTTTTGATATC |
| | | | | SEQ ID NO:7497 | SEQ ID NO:15509 | SEQ ID NO:23521 |
| | | | AA | SYSMN | YISSSTSTIYYAASVKG | DHRYYDSSGYYSDAFDI |
| | | | | SEQ ID NO:7498 | SEQ ID NO:15510 | SEQ ID NO:23522 |
| iPS:436890 | 21-225_63A10 | | NA | AGCTATAGCATGAAC | TACATTAGTAGTAGTACT AGTACCATATACTACGC AGCCTCTGTGAAGGGC | GATCACCGTTACTATGATAG TAGTGGTTATTACTCTGATG CTTTTGATATC |
| | | | | SEQ ID NO:7499 | SEQ ID NO:15511 | SEQ ID NO:23523 |
| | | | AA | SYSMN | YISSSTSTIYYAASVKG | DHRYYDSSGYYSDAFDI |
| | | | | SEQ ID NO:7500 | SEQ ID NO:15512 | SEQ ID NO:23524 |
| iPS:436892 | 21-225_65E9 | | NA | GGCTACTATATGCAC | TGGATCAACCCTAACAG TGGTGGCACAAATTATG CACAGAAGTTTCAGGGC | GCGTATTATTATGGTTCGGG GAGTTATTATAATGAATTTG ATATG |
| | | | | SEQ ID NO:7501 | SEQ ID NO:15513 | SEQ ID NO:23525 |
| | | | AA | GYYMH | WINPNSGGTNYAQKFQG | AYYYGSGSYYNEFDM |
| | | | | SEQ ID NO:7502 | SEQ ID NO:15514 | SEQ ID NO:23526 |
| iPS:436894 | 21-225_66G9 | | NA | ACTAGTGGAGTGGGTGTGGG C | CTCATTAATTGGAATGAT GATAAGCGCTTCAGCCC ATCTCTGAAGAGC | AAAGCTACCTGGGTGGCTTT TGATATC |
| | | | | SEQ ID NO:7503 | SEQ ID NO:15515 | SEQ ID NO:23527 |
| | | | AA | TSGVGVG | LINWNDDKRFSPSLKS | KATWVAFDI |
| | | | | SEQ ID NO:7504 | SEQ ID NO:15516 | SEQ ID NO:23528 |
| iPS:436896 | 21-225_67F10 | | NA | GGCTACTATATGCAC | TGGATCAACCCTAACAG TGGTGGCACAAACTATG GACAGAAGTTTCAGGGC | ACGTATTTCTATGGTTCGGG GAGTTATTATAACGGCTTTG ACTAC |
| | | | | SEQ ID NO:7505 | SEQ ID NO:15517 | SEQ ID NO:23529 |

FIGURE 49

| | | AA | GYYMH | WINPNSGGTNYGQKFQG | TYFYGSGSYYNGFDY |
|---|---|---|---|---|---|
| | | | SEQ ID NO:7506 | SEQ ID NO:15518 | SEQ ID NO:23530 |
| iPS:436898 | 21-225_68D8 | NA | AGCAACAGTGCTGCTTGGAAC | AGGACATACTACAGGTCCGAGTGCTATAATGATTATGCAGTATCTGTGCAGAGT | GATAGAGGGCATAGAGGGTTCTACGGTATGGACGTC |
| | | | SEQ ID NO:7507 | SEQ ID NO:15519 | SEQ ID NO:23531 |
| | | AA | SNSAAWN | RTYYRSECYNDYAVSVQS | DRGHRGFYGMDV |
| | | | SEQ ID NO:7508 | SEQ ID NO:15520 | SEQ ID NO:23532 |
| iPS:436900 | 21-225_69B9 | NA | GGCTACCATATGCAC | TGGATCAACCCTAACAGTGGTGGCACAAATTATGCACAGAAGTTTCAGGGC | GCGTATTATTATGGTTCGGGGAGTTATTATAATGAATCTGATATG |
| | | | SEQ ID NO:7509 | SEQ ID NO:15521 | SEQ ID NO:23533 |
| | | AA | GYHMH | WINPNSGGTNYAQKFQG | AYYYGSGSYYNESDM |
| | | | SEQ ID NO:7510 | SEQ ID NO:15522 | SEQ ID NO:23534 |
| iPS:436902 | 21-225_69B11 | NA | GGCTACTATATGCAC | TGGATCAACCCTAACAGTGGTGGCACAAACTATGGACAGAAGTTTCAGGAC | ACGTATTACTATGGGTCGGGGAGTTATTATAACGGCTTTGACTAC |
| | | | SEQ ID NO:7511 | SEQ ID NO:15523 | SEQ ID NO:23535 |
| | | AA | GYYMH | WINPNSGGTNYGQKFQD | TYYYGSGSYYNGFDY |
| | | | SEQ ID NO:7512 | SEQ ID NO:15524 | SEQ ID NO:23536 |
| iPS:436904 | 21-225_71D4 | NA | GGCTACTGTATGCAC | TGGATCAACCCTAACAGTGGTGGCACAAACTATGCACAGAAGTTTCAGGTC | GCGTATTACTATGGTTCGGGGACTTATCATAACGAATTTGACTAC |
| | | | SEQ ID NO:7513 | SEQ ID NO:15525 | SEQ ID NO:23537 |
| | | AA | GYCMH | WINPNSGGTNYAQKFQV | AYYYGSGTYHNEFDY |
| | | | SEQ ID NO:7514 | SEQ ID NO:15526 | SEQ ID NO:23538 |

FIGURE 49

| iPS:436906 | | NA | GGCTACTATATGCAC | TGGATCAACCCTAACAG TGGTGGCACAAACTATG GACAGAAGTTTCAGGGC | ACGTATTACTATGGTTCGGG GAGTTATTATAACGGCTTTG ACTAC |
|---|---|---|---|---|---|
| | 21-225_72B4 | | SEQ ID NO:7515 | SEQ ID NO:15527 | SEQ ID NO:23539 |
| | | AA | GYYMH | WINPNSGGTNYGQKFQG | TYYYGSGSYYNGFDY |
| | | | SEQ ID NO:7516 | SEQ ID NO:15528 | SEQ ID NO:23540 |
| iPS:436908 | | NA | ACTAGTGGAGTGGGTGTGGG C | CTCATTAATTGGAATGAT GATAAGCGCTACAGCCC ATCTCTGAAGAGC | AAAGCTACCTGGGTGGCTTT TGATATC |
| | 21-225_72D5 | | SEQ ID NO:7517 | SEQ ID NO:15529 | SEQ ID NO:23541 |
| | | AA | TSGVGVG | LINWNDDKRYSPSLKS | KATWVAFDI |
| | | | SEQ ID NO:7518 | SEQ ID NO:15530 | SEQ ID NO:23542 |
| iPS:436910 | | NA | TACTATGGCATGCAC | GTTACAACATATGATGG AAGTAATAAATACTATG CAGACTCCGTGAAGGGC | GAGACTGGAACCTGGGCTTT TGATATC |
| | 21-225_73G1 | | SEQ ID NO:7519 | SEQ ID NO:15531 | SEQ ID NO:23543 |
| | | AA | YYGMH | VTTYDGSNKYYADSVKG | ETGTWAFDI |
| | | | SEQ ID NO:7520 | SEQ ID NO:15532 | SEQ ID NO:23544 |
| iPS:436912 | | NA | ACTAGTGGAGTGGGTGTGGG C | CTCATTAATTGGAATGAT GATAAGCGCTACAGCCC ATCTCTGAAGAGC | AAAACTACCTGGGTGGCTTT TGATATC |
| | 21-225_73C4 | | SEQ ID NO:7521 | SEQ ID NO:15533 | SEQ ID NO:23545 |
| | | AA | TSGVGVG | LINWNDDKRYSPSLKS | KTTWVAFDI |
| | | | SEQ ID NO:7522 | SEQ ID NO:15534 | SEQ ID NO:23546 |
| iPS:436914 | | NA | ACTGGTGGAGTGGGTGTGGG C | CTCATTTATTGGGATGAT GATAAGCGCTACAGCCC ATCTCTGAAGAGC | CTTATAGCAGTGGCCTTTGA CTAC |
| | 21-225_76B4 | | SEQ ID NO:7523 | SEQ ID NO:15535 | SEQ ID NO:23547 |

FIGURE 49

| | | | | | |
|---|---|---|---|---|---|
| | | AA | TGGVGVG | LIYWDDDKRYSPSLKS | LIAVAFDY |
| | | | SEQ ID NO:7524 | SEQ ID NO:15536 | SEQ ID NO:23548 |
| iPS:436916 | 21-225_74A9 | NA | AGCTATGGCATGCAC | GTTATATGGTATGATGG AAATAATAAATCCTATG CAGACTCCGTGAAGGGC | GATCGAGATTATTGTAGTGG TACCAGCTGCCCTTATTATTA CTACTACGGTATGGACGTC |
| | | | SEQ ID NO:7525 | SEQ ID NO:15537 | SEQ ID NO:23549 |
| | | AA | SYGMH | VIWYDGNNKSYADSVKG | DRDYCSGTSCPYYYYYGMDV |
| | | | SEQ ID NO:7526 | SEQ ID NO:15538 | SEQ ID NO:23550 |
| iPS:436918 | 21-225_77A2 | NA | ACTAGTGGAGTGGGTGTGGG C | TTCATTTATTGGGATGAT GATAAGCGCTACAGCCC ATCTCTGAAGAGC | CTTATAGCAGTGGCCTTTGA CTAC |
| | | | SEQ ID NO:7527 | SEQ ID NO:15539 | SEQ ID NO:23551 |
| | | AA | TSGVGVG | FIYWDDDKRYSPSLKS | LIAVAFDY |
| | | | SEQ ID NO:7528 | SEQ ID NO:15540 | SEQ ID NO:23552 |
| iPS:436920 | 21-225_74E5 | NA | AGTGGTGGTTACTACTGGAG C | TACATCTATTACAGTGGG AGCACCTACTACAACCC GTCCCTCAGGAGT | GATTCACCAGTGGCTGGTAC TGACTAC |
| | | | SEQ ID NO:7529 | SEQ ID NO:15541 | SEQ ID NO:23553 |
| | | AA | SGGYYWS | YIYYSGSTYYNPSLRS | DSPVAGTDY |
| | | | SEQ ID NO:7530 | SEQ ID NO:15542 | SEQ ID NO:23554 |
| iPS:436922 | 21-225_78E9 | NA | AGCTATGGCATGCAC | GTTATATGGTATGATGG AAATAATAAATCCTATG CAGACTCCGTGAAGGGC | GATCGAGATTATTGTAGTAG TACCAGCTGCCCTTATTATTA CTACTACGGTATGGACGTC |
| | | | SEQ ID NO:7531 | SEQ ID NO:15543 | SEQ ID NO:23555 |
| | | AA | SYGMH | VIWYDGNNKSYADSVKG | DRDYCSSTSCPYYYYYGMDV |
| | | | SEQ ID NO:7532 | SEQ ID NO:15544 | SEQ ID NO:23556 |

FIGURE 49

| iPS:436924 | | NA | CGATATGGCATGCAC | GTTTTTTGGTATGATGGA AGTAATAAAGACTATGC AGACTCCGTGAAGGGC | GATCGAGATTATTGTAGTAG TACCAGCTGCCCTTACTACT ACTACTACGGTATGGACGTC |
|---|---|---|---|---|---|
| | 21-225_74B3 | | SEQ ID NO:7533 | SEQ ID NO:15545 | SEQ ID NO:23557 |
| | | AA | RYGMH | VFWYDGSNKDYADSVKG | DRDYCSSTSCPYYYYYGMDV |
| | | | SEQ ID NO:7534 | SEQ ID NO:15546 | SEQ ID NO:23558 |
| iPS:436926 | | NA | AGTGGTGGTTACTACTGGAG C | TACATCTATTACATTGGG AGTGTTTACTACAACCCG TCCCTCAAGAGT | GATGCCCCCGACTTCGGTAT GGACGTC |
| | 21-225_78D10 | | SEQ ID NO:7535 | SEQ ID NO:15547 | SEQ ID NO:23559 |
| | | AA | SGGYYWS | YIYYIGSVYYNPSLKS | DAPDFGMDV |
| | | | SEQ ID NO:7536 | SEQ ID NO:15548 | SEQ ID NO:23560 |
| iPS:436928 | | NA | AGCTATGGCATGCAC | GTTATATGGTATGATGG AAATAATAAATCCTATG CAGACTCCGTGAAGGGC | GATCGAGATTATTGTAGTAG TACCAGCTGCCCTTATTATTA CTACTACGGTATGGACGTC |
| | 21-225_79E7 | | SEQ ID NO:7537 | SEQ ID NO:15549 | SEQ ID NO:23561 |
| | | AA | SYGMH | VIWYDGNNKSYADSVKG | DRDYCSSTSCPYYYYYGMDV |
| | | | SEQ ID NO:7538 | SEQ ID NO:15550 | SEQ ID NO:23562 |
| iPS:436932 | | NA | AGCTATGGCATGCAC | GTTATATGGTATGATGG AAATAATAAATCCTATG CAGACTCCGTGAAGGGC | GATCGAGATTATTGTAGTAG TACCAGCTGCCCTTATTATTA CTACTACGGTATGGACGTC |
| | 21-225_92A4 | | SEQ ID NO:7539 | SEQ ID NO:15551 | SEQ ID NO:23563 |
| | | AA | SYGMH | VIWYDGNNKSYADSVKG | DRDYCSSTSCPYYYYYGMDV |
| | | | SEQ ID NO:7540 | SEQ ID NO:15552 | SEQ ID NO:23564 |

FIGURE 49

| iPS:436934 | | NA | ACTGGTGGAGTGGGTGTGGGC | CTCATTTATTGGGATGATGATAAGCGCTACAGCCCATCTCTGAAGAGC | CTTATAGCAGTGGCCTGTGACTAC |
|---|---|---|---|---|---|
| | 21-225_96B5 | | SEQ ID NO:7541 | SEQ ID NO:15553 | SEQ ID NO:23565 |
| | | AA | TGGVGVG | LIYWDDDKRYSPSLKS | LIAVACDY |
| | | | SEQ ID NO:7542 | SEQ ID NO:15554 | SEQ ID NO:23566 |
| iPS:436936 | | NA | AGCTATGGCATGCAC | GTTATATGGTATGATGGAAATAATAAATCCTATGCAGACTCCGTGAAGGGC | GATCGAGATTATTGTAGTAGTACCAGCTGCCCTTATTATTACTACTACGGTATGGACGTC |
| | 21-225_97E6 | | SEQ ID NO:7543 | SEQ ID NO:15555 | SEQ ID NO:23567 |
| | | AA | SYGMH | VIWYDGNNKSYADSVKG | DRDYCSSTSCPYYYYYGMDV |
| | | | SEQ ID NO:7544 | SEQ ID NO:15556 | SEQ ID NO:23568 |
| iPS:436938 | | NA | AGCTATGCCATGAGC | GTTATTAGTGGTGGTGGTACTACCACATACTACGCAGACTCCGTGAAGGGC | TGGCGAGGTAACCCCACTGACTACGGTATGGACGTC |
| | 21-225_146A3 | | SEQ ID NO:7545 | SEQ ID NO:15557 | SEQ ID NO:23569 |
| | | AA | SYAMS | VISGGGTTTYYADSVKG | WRGNPTDYGMDV |
| | | | SEQ ID NO:7546 | SEQ ID NO:15558 | SEQ ID NO:23570 |
| iPS:436940 | | NA | ACCTATGGCATGCAC | GTTGTATGGTATGGTGGAAATGATAAAGACTTTGCAGACTCCGTGACGGGC | GATCGGGATTATTGTAGTGGTGGTAGCTGCCCTTACTACTACTACTACGGTATGGACGTC |
| | 21-225_146B8 | | SEQ ID NO:7547 | SEQ ID NO:15559 | SEQ ID NO:23571 |
| | | AA | TYGMH | VVWYGGNDKDFADSVTG | DRDYCSGGSCPYYYYYGMDV |
| | | | SEQ ID NO:7548 | SEQ ID NO:15560 | SEQ ID NO:23572 |

FIGURE 49

| iPS:436942 | | NA | AGTTATGATATCAAT | TGGATGAACCCTAACAG TGGTAACACAGGCTATG CACAGAAGTTCCAGGGC | GGAGATTATTACTATGATAG TAGTGGTCACCAGCCTTACT ACTACTACTACGGTATGGAC GTC |
|---|---|---|---|---|---|
| | 21-225_146H8 | | SEQ ID NO:7549 | SEQ ID NO:15561 | SEQ ID NO:23573 |
| | | AA | SYDIN | WMNPNSGNTGYAQKFQG | GDYYYDSSGHQPYYYYYGMD V |
| | | | SEQ ID NO:7550 | SEQ ID NO:15562 | SEQ ID NO:23574 |
| iPS:436944 | | NA | ACTACTGGAGTGGGTGTGGG C | ATCCTTTTTTGGAATGAT GATGAGCGCTACAGCCC ATCTCTGAAGAGC | AAATCGCAGCTCGTCTACTT TGACTAC |
| | 21-225_182D12 | | SEQ ID NO:7551 | SEQ ID NO:15563 | SEQ ID NO:23575 |
| | | AA | TTGVGVG | ILFWNDDERYSPSLKS | KSQLVYFDY |
| | | | SEQ ID NO:7552 | SEQ ID NO:15564 | SEQ ID NO:23576 |
| iPS:436946 | | NA | AGCTATGGCATGCAC | GTTATATGGTATGATGG AAGTAATAAATACTATG CAGACTCCGTGAAGGGC | GAAAGGACGTATTGTAGTGG TACCACCTGCCCCTACTACT ACTACTACGGTCTGGGCGTC |
| | 21-225_183F4 | | SEQ ID NO:7553 | SEQ ID NO:15565 | SEQ ID NO:23577 |
| | | AA | SYGMH | VIWYDGSNKYYADSVKG | ERTYCSGTTCPYYYYYGLGV |
| | | | SEQ ID NO:7554 | SEQ ID NO:15566 | SEQ ID NO:23578 |
| iPS:436948 | | NA | AGTTATGGCATGCTC | GTTATATGGTATGATGG AAGTGGTAAATACTATG CAGACTCCGTGAAGGGC | GAGAATTTTTGGAGTGGTGA CTAC |
| | 21-225_183F5 | | SEQ ID NO:7555 | SEQ ID NO:15567 | SEQ ID NO:23579 |
| | | AA | SYGML | VIWYDGSGKYYADSVKG | ENFWSGDY |
| | | | SEQ ID NO:7556 | SEQ ID NO:15568 | SEQ ID NO:23580 |

FIGURE 49

| iPS:436950 | | NA | AGCTATGGCATGCAC | ATTATATGGTATGATGG AAGTAATAAATACTATG CAGACTCCGTGAAGGGC | GGGGGGGCCCCCGTTCTCTAC GGTGACTATGTACTTTGACT AC |
|---|---|---|---|---|---|
| | 21-225_184G4 | | SEQ ID NO:7557 | SEQ ID NO:15569 | SEQ ID NO:23581 |
| | | AA | SYGMH | IIWYDGSNKYYADSVKG | GGPPFSTVTMYFDY |
| | | | SEQ ID NO:7558 | SEQ ID NO:15570 | SEQ ID NO:23582 |
| iPS:436952 | | NA | AACTATGGCATGCAC | ATTATATGGTATGATGG AAGTGATAAATACTATG CAGACTCCGTGAAGGGC | GGGGGGGCCCCCGTTCTCTAC GGTGACTATGTACTTTGACT AC |
| | 21-225_185D2 | | SEQ ID NO:7559 | SEQ ID NO:15571 | SEQ ID NO:23583 |
| | | AA | NYGMH | IIWYDGSDKYYADSVKG | GGPPFSTVTMYFDY |
| | | | SEQ ID NO:7560 | SEQ ID NO:15572 | SEQ ID NO:23584 |
| iPS:436954 | | NA | ACTGGTGGAGTGGGTGTGGG C | CTCATTTATTGGAATGAT GATGAGCGCTACAGCCC ATCTCTGAAGAGC | ATTATAGCAGTGGCCTTCCA GCAT |
| | 21-225_185G7 | | SEQ ID NO:7561 | SEQ ID NO:15573 | SEQ ID NO:23585 |
| | | AA | TGGVGVG | LIYWNDDERYSPSLKS | IIAVAFQH |
| | | | SEQ ID NO:7562 | SEQ ID NO:15574 | SEQ ID NO:23586 |
| iPS:436956 | | NA | ACTAGTGGAGTGGGTGTGGG C | TTCATTTCTTGGAATGAT GATAAGCGCTACAGCCC ATCTCTGAAGAGC | AAAGCAGCAGCTGTTGCTTT TGATATC |
| | 21-225_186H6 | | SEQ ID NO:7563 | SEQ ID NO:15575 | SEQ ID NO:23587 |
| | | AA | TSGVGVG | FISWNDDKRYSPSLKS | KAAAVAFDI |
| | | | SEQ ID NO:7564 | SEQ ID NO:15576 | SEQ ID NO:23588 |
| iPS:436958 | | NA | AGTGGTGGTTACTACTGGAG C | TACATCTATTACAGTGGG AGCACCTACTACAACCC GTCCCTCAAGAGT | GATTCCCCACTACGAGGCTT TGACTAC |
| | 21-225_190D1 | | SEQ ID NO:7565 | SEQ ID NO:15577 | SEQ ID NO:23589 |
| | | AA | SGGYYWS | YIYYSGSTYYNPSLKS | DSPLRGFDY |

FIGURE 49

| | | | SEQ ID NO:7566 | SEQ ID NO:15578 | SEQ ID NO:23590 |
|---|---|---|---|---|---|
| iPS:436960 | | NA | AGCTATGGCATGCAT | GTTATAATATATGATGG AAGTTATAAGTACTATG CAGACTCCGTGAAGGGC | ACGTATAGCGGGGGGTATGGA CGTC |
| | 21-225_198D2 | | SEQ ID NO:7567 | SEQ ID NO:15579 | SEQ ID NO:23591 |
| | | AA | SYGMH | VIIYDGSYKYYADSVKG | TYSGGMDV |
| | | | SEQ ID NO:7568 | SEQ ID NO:15580 | SEQ ID NO:23592 |
| iPS:436962 | | NA | AGGAAAAGTGCTACTTGGAA C | AAGACATACTACAGGTC CAAGTGGTATAATGATT ATGCAGTATCTGTGAAA AGT | GATCCGGGTGGCCTCTTTGA CTAC |
| | 21-225_190H1 | | SEQ ID NO:7569 | SEQ ID NO:15581 | SEQ ID NO:23593 |
| | | AA | RKSATWN | KTYYRSKWYNDYAVSVK S | DPGGLFDY |
| | | | SEQ ID NO:7570 | SEQ ID NO:15582 | SEQ ID NO:23594 |
| iPS:436964 | | NA | AACTATGGCATACAC | GTTATATGGTTTGATGGA GATAATAAATACTATGC AGACTCCGTGAAGGGC | GATAACTGGAACTACGGCGA TCACTACTACTACTTCGGTAT GGACGTC |
| | 21-225_190B3 | | SEQ ID NO:7571 | SEQ ID NO:15583 | SEQ ID NO:23595 |
| | | AA | NYGIH | VIWFDGDNKYYADSVKG | DNWNYGDHYYYFGMDV |
| | | | SEQ ID NO:7572 | SEQ ID NO:15584 | SEQ ID NO:23596 |
| iPS:436966 | | NA | AGCTATGGCATGCAC | GTTATATGGTATGATGG AAGTAATAAATACTATG CAGACTCCGTGAAGGGC | TGGTACTACTACTACTACGG TATGGACGTC |
| | 21-225_190C3 | | SEQ ID NO:7573 | SEQ ID NO:15585 | SEQ ID NO:23597 |
| | | AA | SYGMH | VIWYDGSNKYYADSVKG | WYYYYYGMDV |

FIGURE 49

|  |  |  | SEQ ID NO:7574 | SEQ ID NO:15586 | SEQ ID NO:23598 |
|---|---|---|---|---|---|
| iPS:436968 | | NA | AGCTATGGCATGCAC | GTTATATGGAATGATGG AAGTAAAAAAATACCATG TAGACTCCGTGAAGGGC | GATCTGGATAAGAGGAACTT TCCTTATTACTACTACTACGG TATGGACGTC |
| | 21-225_190B10 | | SEQ ID NO:7575 | SEQ ID NO:15587 | SEQ ID NO:23599 |
| | | AA | SYGMH | VIWNDGSKKYHVDSVKG | DLDKRNFPYYYYYGMDV |
| | | | SEQ ID NO:7576 | SEQ ID NO:15588 | SEQ ID NO:23600 |
| iPS:436970 | | NA | AGCTATGGCATGCAC | GTTATATGGTTTGATGGA AGTAATAAATACTATAC AGACTCCGTGAAGGGC | GATAACTGGAACTACGGCGA TTACTACTACTACTACGGTA TGGACGTC |
| | 21-225_190B8 | | SEQ ID NO:7577 | SEQ ID NO:15589 | SEQ ID NO:23601 |
| | | AA | SYGMH | VIWFDGSNKYYTDSVKG | DNWNYGDYYYYYGMDV |
| | | | SEQ ID NO:7578 | SEQ ID NO:15590 | SEQ ID NO:23602 |
| iPS:436972 | | NA | GGCTACTATATGCAC | TGGATCAACCCTAACAG TGGTGGCACAAACTATG CACAGAAGTTTCAGGGC | GATAGAGCAGTGGCTGGAAA CTACTTCTACTACGGTATGG ACGTC |
| | 21-225_190C7 | | SEQ ID NO:7579 | SEQ ID NO:15591 | SEQ ID NO:23603 |
| | | AA | GYYMH | WINPNSGGTNYAQKFQG | DRAVAGNYFYYGMDV |
| | | | SEQ ID NO:7580 | SEQ ID NO:15592 | SEQ ID NO:23604 |
| iPS:436974 | | NA | AGCTATGGCATGCAT | GTTATAATATATGATGG AAGTTATAAGTACTATG CAGACTCCGTGAAGGGC | ACGTATAGCGGGGGGTATGGA CGTC |
| | 21-225_190H7 | | SEQ ID NO:7581 | SEQ ID NO:15593 | SEQ ID NO:23605 |
| | | AA | SYGMH | VIIYDGSYKYYADSVKG | TYSGGMDV |
| | | | SEQ ID NO:7582 | SEQ ID NO:15594 | SEQ ID NO:23606 |

FIGURE 49

| iPS:436976 | 21-225_190D8 | NA | AGCTATGGCCTGCAC | GTTATATGGTATGATGGAAGTAATAAATACTATGCAGACTCCGTGAAGGGC | TGGTACTACTACTACTACGGTATGGACGTC |
|---|---|---|---|---|---|
| | | | SEQ ID NO:7583 | SEQ ID NO:15595 | SEQ ID NO:23607 |
| | | AA | SYGLH | VIWYDGSNKYYADSVKG | WYYYYYGMDV |
| | | | SEQ ID NO:7584 | SEQ ID NO:15596 | SEQ ID NO:23608 |
| iPS:436978 | 21-225_190G9 | NA | AGGAAAAGTGCTACTTGGAAC | AGGACATACTACAGGTCCAAGTGGTATAATGATTATGCAGTATCTGTGAAAAGT | GATCCGGGTGGCCTCTTTGACTAC |
| | | | SEQ ID NO:7585 | SEQ ID NO:15597 | SEQ ID NO:23609 |
| | | AA | RKSATWN | RTYYRSKWYNDYAVSVKS | DPGGLFDY |
| | | | SEQ ID NO:7586 | SEQ ID NO:15598 | SEQ ID NO:23610 |
| iPS:436980 | 21-225_190C10 | NA | AACTATGGCATGCAC | GTTATATGGTTTGGTGGAGATAATAAATACTATGCAGACTCCGTGAGGGGC | GATAACTGGAACTACGGCGATCACTACTACTATTACGGAATGGACGTC |
| | | | SEQ ID NO:7587 | SEQ ID NO:15599 | SEQ ID NO:23611 |
| | | AA | NYGMH | VIWFGGDNKYYADSVRG | DNWNYGDHYYYYGMDV |
| | | | SEQ ID NO:7588 | SEQ ID NO:15600 | SEQ ID NO:23612 |
| iPS:436982 | 21-225_190D10 | NA | AGCTATGGCATGCAT | GTTATAATATATGATGGAAGTTATAAGTACTATGCAGACTCCGTGAAGGGC | ACGTATAGCGGGGGGTATGGACGTC |
| | | | SEQ ID NO:7589 | SEQ ID NO:15601 | SEQ ID NO:23613 |
| | | AA | SYGMH | VIIYDGSYKYYADSVKG | TYSGGMDV |
| | | | SEQ ID NO:7590 | SEQ ID NO:15602 | SEQ ID NO:23614 |

FIGURE 49

| iPS:436984 | | NA | AGTGGTGGTGACTACTGGAGC | TACATCTATTACAGTGGGATCACCTACTACAATCCGTCCCTCAAGAGT | GATAGCAGCTCGCGGGGTATGGACGTC |
|---|---|---|---|---|---|
| | 21-225_190F10 | | SEQ ID NO:7591 | SEQ ID NO:15603 | SEQ ID NO:23615 |
| | | AA | SGGDYWS | YIYYSGITYYNPSLKS | DSSSRGMDV |
| | | | SEQ ID NO:7592 | SEQ ID NO:15604 | SEQ ID NO:23616 |
| iPS:436986 | | NA | AGTTACTACTGGATC | TATATCTATTACAGTGGGAGTACTAAGTACAACCCCTCCCTCAAGAGT | AAGGGAGTGGGAACCATCCACTTTGACTAC |
| | 21-225_191A1 | | SEQ ID NO:7593 | SEQ ID NO:15605 | SEQ ID NO:23617 |
| | | AA | SYYWI | YIYYSGSTKYNPSLKS | KGVGTIHFDY |
| | | | SEQ ID NO:7594 | SEQ ID NO:15606 | SEQ ID NO:23618 |
| iPS:436988 | | NA | AGTGGTGGTGACTACTGGAGC | TACATCTATTACAGTGGGATCACCTACTACAATCCGTCCCTCAAGAGT | GATAGCAGCTCGCGGGGTATGGACGTC |
| | 21-225_191A2 | | SEQ ID NO:7595 | SEQ ID NO:15607 | SEQ ID NO:23619 |
| | | AA | SGGDYWS | YIYYSGITYYNPSLKS | DSSSRGMDV |
| | | | SEQ ID NO:7596 | SEQ ID NO:15608 | SEQ ID NO:23620 |
| iPS:436992 | | NA | AGCTATGGCATGCAC | GTTATATGGTTTGATGGAAGTAATAAATACTATGCAGACTCCGTGAAGGGC | GATAACTGGAACTACGGCGATCACTACTACTACTACGGTATGGACGTC |
| | 21-225_191B8 | | SEQ ID NO:7597 | SEQ ID NO:15609 | SEQ ID NO:23621 |
| | | AA | SYGMH | VIWFDGSNKYYADSVKG | DNWNYGDHYYYYGMDV |
| | | | SEQ ID NO:7598 | SEQ ID NO:15610 | SEQ ID NO:23622 |
| iPS:436994 | | NA | AATTATGGCATGCAC | GTTATATGGTTTGGTGGAGATAATAAATACTATGCAGACTCCGTGAAGGGC | GATAACTGGAACTACGGCGATCACTACTACTATTACGGTATGGACGTC |
| | 21-225_191A9 | | SEQ ID NO:7599 | SEQ ID NO:15611 | SEQ ID NO:23623 |

FIGURE 49

| | | | AA | NYGMH | VIWFGGDNKYYADSVKG | DNWNYGDHYYYYGMDV |
|---|---|---|---|---|---|---|
| | | | | SEQ ID NO:7600 | SEQ ID NO:15612 | SEQ ID NO:23624 |
| iPS:436996 | 21-225_191B9 | NA | | TTCCATGGCATGCAC | GTTATATGGTATGATGGAAGTAAAAAAATACTATGCAGACTCCGTGAAGGGC | GAAGGGTATAGCAGTGGCTTTTACAGGGGGTTTGACAAC |
| | | | | SEQ ID NO:7601 | SEQ ID NO:15613 | SEQ ID NO:23625 |
| | | AA | | FHGMH | VIWYDGSKKYYADSVKG | EGYSSGFYRGFDN |
| | | | | SEQ ID NO:7602 | SEQ ID NO:15614 | SEQ ID NO:23626 |
| iPS:437000 | 21-225_191G9 | NA | | ACCTATGGCATGCAC | CTTATATGGTTTGATGGAAGTAATAAATACTATGCAGACTCCGTGAAGGGC | GATCGGGTGGGAGGTACTAGTCCTCCTTACTACTACTACGGTATGGACGTC |
| | | | | SEQ ID NO:7603 | SEQ ID NO:15615 | SEQ ID NO:23627 |
| | | AA | | TYGMH | LIWFDGSNKYYADSVKG | DRVGGTSPPYYYYYGMDV |
| | | | | SEQ ID NO:7604 | SEQ ID NO:15616 | SEQ ID NO:23628 |
| iPS:437002 | 21-225_191H9 | NA | | GGCTACAATATGCAC | TGGATCAACCCTAATAGTGGTGGCACAAACTATGCACACAAGTTTCAGGGC | GATTTCTATGATAGTGGTGGAGAAGGGTGGTTCGACCCC |
| | | | | SEQ ID NO:7605 | SEQ ID NO:15617 | SEQ ID NO:23629 |
| | | AA | | GYNMH | WINPNSGGTNYAHKFQG | DFYDSGGEGWFDP |
| | | | | SEQ ID NO:7606 | SEQ ID NO:15618 | SEQ ID NO:23630 |
| iPS:437006 | 21-225_192G2 | NA | | AGCTATGGCATGCAC | GTTATATGGAATGATGGAAGTAATAAATACTATGCAGACTCCGTGAAGGGC | GATCTGGATAAGAGGAACTTTCCTTATTACTACTACTACGGTATGGACGTC |
| | | | | SEQ ID NO:7607 | SEQ ID NO:15619 | SEQ ID NO:23631 |

FIGURE 49

| | | | AA | SYGMH | VIWNDGSNKYYADSVKG | DLDKRNFPYYYYYGMDV |
|---|---|---|---|---|---|---|
| | | | | SEQ ID NO:7608 | SEQ ID NO:15620 | SEQ ID NO:23632 |
| iPS:437008 | 21-225_192E3 | | NA | AGTGGTGGTTACTACTGGAGC | TACATCTATTACACTGGGAGCACCTACTACAACCCGTCCCTCAAGAGT | GACGATCCCCTCTACGGAATGGACGTC |
| | | | | SEQ ID NO:7609 | SEQ ID NO:15621 | SEQ ID NO:23633 |
| | | | AA | SGGYYWS | YIYYTGSTYYNPSLKS | DDPLYGMDV |
| | | | | SEQ ID NO:7610 | SEQ ID NO:15622 | SEQ ID NO:23634 |
| iPS:437010 | 21-225_192G3 | | NA | AATTACTACTGGAGC | CGGATCTATTCCAGTGGGAGCACCAACTACAACCCCTCCCTCAAGAGT | GGGTGGGAGCTAAACTAC |
| | | | | SEQ ID NO:7611 | SEQ ID NO:15623 | SEQ ID NO:23635 |
| | | | AA | NYYWS | RIYSSGSTNYNPSLKS | GWELNY |
| | | | | SEQ ID NO:7612 | SEQ ID NO:15624 | SEQ ID NO:23636 |
| iPS:437012 | 21-225_192G7 | | NA | AGTGGTGGTTACTACTGGAGC | TACATCTATTACAGAGGGAGTACCTACTACAATCCGTCCCTCAAGAGT | GACTCCCCGGTGACAGGATTTGACTAT |
| | | | | SEQ ID NO:7613 | SEQ ID NO:15625 | SEQ ID NO:23637 |
| | | | AA | SGGYYWS | YIYYRGSTYYNPSLKS | DSPVTGFDY |
| | | | | SEQ ID NO:7614 | SEQ ID NO:15626 | SEQ ID NO:23638 |
| iPS:437014 | 21-225_192H8 | | NA | AGTGGTGGTGACTACTGGAGC | TACATCTATTACAGTGGGCCCACCTACTACAACCCGTCCCTCAAGAGT | GATAGCTCCCTCTACGGTATGGACGTC |
| | | | | SEQ ID NO:7615 | SEQ ID NO:15627 | SEQ ID NO:23639 |
| | | | AA | SGGDYWS | YIYYSGPTYYNPSLKS | DSSLYGMDV |
| | | | | SEQ ID NO:7616 | SEQ ID NO:15628 | SEQ ID NO:23640 |
| iPS:437016 | 21 225 193A6 | | NA | AGTTACTACTGGAGC | TATATCTATTACAGTGGGAGCACCAACTACAACCCCTCCCTCAAGAGT | GGATGGGAGCTAAACTAC |

FIGURE 49

| | 21-225_193A6 | | SEQ ID NO:7617 | SEQ ID NO:15629 | SEQ ID NO:23641 |
|---|---|---|---|---|---|
| | | AA | SYYWS | YIYYSGSTNYNPSLKS | GWELNY |
| iPS:437018 | | NA | SEQ ID NO:7618 | SEQ ID NO:15630 | SEQ ID NO:23642 |
| | 21-225_193H5 | | AACGCCTACATGACC | CGTATTAAAAGCAAAACTGATGGTGGGACAACAGACTACGCTGCACCCGTGAAAGGC | GATCCCGGTGGTATCTTTGACTAC |
| | | | SEQ ID NO:7619 | SEQ ID NO:15631 | SEQ ID NO:23643 |
| | | AA | NAYMT | RIKSKTDGGTTDYAAPVKG | DPGGIFDY |
| iPS:437020 | | NA | SEQ ID NO:7620 | SEQ ID NO:15632 | SEQ ID NO:23644 |
| | 21-225_193F11 | | GGCTACTATATGCAC | TGGATCAACCCTTACAGTGGTGGCACAAACTATGCACAGAAGTTTCAGGGC | GATAGAGCAGTGGCTGGAAACTACTTCTACTACGGTATGGACGTC |
| | | | SEQ ID NO:7621 | SEQ ID NO:15633 | SEQ ID NO:23645 |
| | | AA | GYYMH | WINPYSGGTNYAQKFQG | DRAVAGNYFYYGMDV |
| iPS:437022 | | NA | SEQ ID NO:7622 | SEQ ID NO:15634 | SEQ ID NO:23646 |
| | 21-225_194G5 | | AGTGGTGGTGACTACTGGAGC | TACATCTATTACAGTGGGAGCACCTACTACAACCCGTCTCTCAAGAGT | GATCACTCCCTCTACGGTATGGACGTC |
| | | | SEQ ID NO:7623 | SEQ ID NO:15635 | SEQ ID NO:23647 |
| | | AA | SGGDYWS | YIYYSGSTYYNPSLKS | DHSLYGMDV |
| iPS:437024 | | NA | SEQ ID NO:7624 | SEQ ID NO:15636 | SEQ ID NO:23648 |
| | 21-225_194F11 | | AGCTATGGCATGCAC | GTTATATGGAATGATGGAAGTAAAAAATACCATGTAGACTCCGTGAAGGGC | GATCTGGATAAGAGGAACTTTCCTTATTACTACTACTACGGTATGGACGTC |
| | | | SEQ ID NO:7625 | SEQ ID NO:15637 | SEQ ID NO:23649 |
| | | AA | SYGMH | VIWNDGSKKYHVDSVKG | DLDKRNFPYYYYYGMDV |

FIGURE 49

| | | | SEQ ID NO:7626 | SEQ ID NO:15638 | SEQ ID NO:23650 |
|---|---|---|---|---|---|
| iPS:437026 | 21-225_194D12 | NA | AGTGGTGGTGACTACTGGAGC | TACATCTATTACAGTGGGAGTACCTACTACAACCCGTCCCTCAAGAGT | GATGGGGCTCGGCACGGTATGGACGTC |
| | | | SEQ ID NO:7627 | SEQ ID NO:15639 | SEQ ID NO:23651 |
| | | AA | SGGDYWS | YIYYSGSTYYNPSLKS | DGARHGMDV |
| | | | SEQ ID NO:7628 | SEQ ID NO:15640 | SEQ ID NO:23652 |
| iPS:437028 | 21-225_194G12 | NA | AGCTATGGCATGCAC | GTTATATGGAATGATGGAAGTAATAAATACTATGCAGACTCCGTGAAGGGC | GATCTGGATAAGAGGAACTTTCCTTATTACTACTACTACGGTATGGACGTC |
| | | | SEQ ID NO:7629 | SEQ ID NO:15641 | SEQ ID NO:23653 |
| | | AA | SYGMH | VIWNDGSNKYYADSVKG | DLDKRNFPYYYYYGMDV |
| | | | SEQ ID NO:7630 | SEQ ID NO:15642 | SEQ ID NO:23654 |
| iPS:437030 | 21-225_195E3 | NA | GACTACTACATGAGC | TATATTACTAGTAGTGGTAATACCATATACTACGCAGACTCTGTGAAGGGC | GATAGTCGATATTTTGACTGGTTTGACTAC |
| | | | SEQ ID NO:7631 | SEQ ID NO:15643 | SEQ ID NO:23655 |
| | | AA | DYYMS | YITSSGNTIYYADSVKG | DSRYFDWFDY |
| | | | SEQ ID NO:7632 | SEQ ID NO:15644 | SEQ ID NO:23656 |
| iPS:437032 | 21-225_195H6 | NA | AATTACTACTGGAGC | CGTATCTATAGCAGTGGGAGCACCAACTACAACCCCTCCCTCAAGAGT | GGGTGGGAGCTAAACAAC |
| | | | SEQ ID NO:7633 | SEQ ID NO:15645 | SEQ ID NO:23657 |
| | | AA | NYYWS | RIYSSGSTNYNPSLKS | GWELNN |
| | | | SEQ ID NO:7634 | SEQ ID NO:15646 | SEQ ID NO:23658 |
| iPS:437034 | 21 225 195E9 | NA | GGCTACTATATGCAC | TGGATCAACCCTAACAGTGGTGCCACAAACTATGCACAGAAGTTTCAGGGC | GCCTATTACTATGGTTCGGGGACTTATTATAACGAGTTCGACTAC |

FIGURE 49

| | | | | | |
|---|---|---|---|---|---|
| | 21-225_195E9 | | SEQ ID NO:7635 | SEQ ID NO:15647 | SEQ ID NO:23659 |
| | | AA | GYYMH | WINPNSGATNYAQKFQG | AYYYGSGTYYNEFDY |
| iPS:437036 | | NA | SEQ ID NO:7636 | SEQ ID NO:15648 | SEQ ID NO:23660 |
| | 21-225_195H9 | | GGCTACTATATGCAC | TGGATCAACCCTTACAGTGGTGGCACAAACTATGCACAGAAGTTTCAGGAC | GATAGAGCAGTGGCTGGAAACTACTTCTACTACGGTATGGACGTC |
| | | | SEQ ID NO:7637 | SEQ ID NO:15649 | SEQ ID NO:23661 |
| | | AA | GYYMH | WINPYSGGTNYAQKFQD | DRAVAGNYFYYGMDV |
| iPS:437040 | | NA | SEQ ID NO:7638 | SEQ ID NO:15650 | SEQ ID NO:23662 |
| | 21-225_196E7 | | GGCTACAATATGCAC | TGGATCAACCCTAATAGTGGTGGCACAAACTATGCACACAAGTTTCAGGGC | GATTACTATGATACTAGTGGAGAAGGGTGGTTCGACCCC |
| | | | SEQ ID NO:7639 | SEQ ID NO:15651 | SEQ ID NO:23663 |
| | | AA | GYNMH | WINPNSGGTNYAHKFQG | DYYDTSGEGWFDP |
| iPS:437042 | | NA | SEQ ID NO:7640 | SEQ ID NO:15652 | SEQ ID NO:23664 |
| | 21-225_197E8 | | GGCTACTATATACAC | TGGATCAACCCTAACAGTGGTGGCACAAACTATGCACAGAGGTTTCAGGGC | GAGATAGCAGTGGCTGGGAACTACTTCTACTACGGTATGGGCGTC |
| | | | SEQ ID NO:7641 | SEQ ID NO:15653 | SEQ ID NO:23665 |
| | | AA | GYYIH | WINPNSGGTNYAQRFQG | EIAVAGNYFYYGMGV |
| iPS:437044 | | NA | SEQ ID NO:7642 | SEQ ID NO:15654 | SEQ ID NO:23666 |
| | 21-225_197F9 | | ATTTACTACTGGAGC | TATGTCTATTACAGTGGGAGCACCACCTACAACCCCTCCCTCAAGAGT | GAAAGGGGGGAGTAGCCACAGATGGGGGGGACTACTACGGAATGGACGTC |
| | | | SEQ ID NO:7643 | SEQ ID NO:15655 | SEQ ID NO:23667 |
| | | AA | IYYWS | YVYYSGSTTYNPSLKS | ERGSSHRWGDYYGMDV |
| iPS:437048 | | NA | SEQ ID NO:7644 | SEQ ID NO:15656 | SEQ ID NO:23668 |
| | 21 225 197B11 | | AGTGGTGGTTACTACTGGAGC | TACATCTATTACACTGGGAGCACCTACTACAACCCCGTCCCTCAAGAGT | GACGATCCCCTCTACGGAATGGACGTC |

FIGURE 49

| | | | | | |
|---|---|---|---|---|---|
| | 21-225_197B11 | | SEQ ID NO:7645 | SEQ ID NO:15657 | SEQ ID NO:23669 |
| | | AA | SGGYYWS | YIYYTGSTYYNPSLKS | DDPLYGMDV |
| | | | SEQ ID NO:7646 | SEQ ID NO:15658 | SEQ ID NO:23670 |
| iPS:437050 | 21-225_197C11 | NA | GGCTACAATATGCAC | TGGATCAACCCTAATAGTGGTGGCACAAACTATGCACACAAGTTTCAGGGC | GATTACTATGATAGTAGTGGAGAAGGGTGGTTCGACCCC |
| | | | SEQ ID NO:7647 | SEQ ID NO:15659 | SEQ ID NO:23671 |
| | | AA | GYNMH | WINPNSGGTNYAHKFQG | DYYDSSGEGWFDP |
| | | | SEQ ID NO:7648 | SEQ ID NO:15660 | SEQ ID NO:23672 |
| iPS:437054 | 21-225_194G3 | NA | TTCCATGGCATGCAC | GTTATATGGTATGATGGAAGTAAAAAATACTATGCAGACTCCGTGAAGGGC | GAAGGGTTTAGCAGTGGCTTTTACAGGGGGGTTTGACAAC |
| | | | SEQ ID NO:7649 | SEQ ID NO:15661 | SEQ ID NO:23673 |
| | | AA | FHGMH | VIWYDGSKKYYADSVKG | EGFSSGFYRGFDN |
| | | | SEQ ID NO:7650 | SEQ ID NO:15662 | SEQ ID NO:23674 |
| iPS:437056 | 21-225_198B8 | NA | AGTGGTGGTGACTACTGGAGC | TACATCTATTACAGTGGGATCACCTACTACAATCCGTCCCTCAAGAGT | GATAGCAGCTCGCGGGGTATGGACGTC |
| | | | SEQ ID NO:7651 | SEQ ID NO:15663 | SEQ ID NO:23675 |
| | | AA | SGGDYWS | YIYYSGITYYNPSLKS | DSSSRGMDV |
| | | | SEQ ID NO:7652 | SEQ ID NO:15664 | SEQ ID NO:23676 |
| iPS:437058 | 21-225_199F3 | NA | TTCTATGGCATGCAC | GTTATTTGGTATGATGGAAGTAGTAAATACTATGCAGACTCCGTGAAGGGC | GAAGGGTATAGCAGTGGCTTTTACAGGGGATTTGCCAAC |
| | | | SEQ ID NO:7653 | SEQ ID NO:15665 | SEQ ID NO:23677 |
| | | AA | FYGMH | VIWYDGSSKYYADSVKG | EGYSSGFYRGFAN |
| | | | SEQ ID NO:7654 | SEQ ID NO:15666 | SEQ ID NO:23678 |

FIGURE 49

| iPS:437060 | | NA | ATTTACTACTGGAGC | TATATCTATTACAGTGGG AGCACCACCTACAACCC CTCCCTCAAGAGT | GAAAGGGGGAGTAGCCACA GATGGGGGGACTACTACGGA ATGGACGTC |
|---|---|---|---|---|---|
| | 21-225_199C3 | | SEQ ID NO:7655 | SEQ ID NO:15667 | SEQ ID NO:23679 |
| | | AA | IYYWS | YIYYSGSTTYNPSLKS | ERGSSHRWGDYYGMDV |
| | | | SEQ ID NO:7656 | SEQ ID NO:15668 | SEQ ID NO:23680 |
| iPS:437062 | | NA | AGTGGTGGTGACTATTGGAG C | TACATCTATTATAGTGGG AGCACCTACTACAACCC GTCCCTCAAGAGT | GATGGAGCAGCTCTGGGTAT GGACGTC |
| | 21-225_200H1 | | SEQ ID NO:7657 | SEQ ID NO:15669 | SEQ ID NO:23681 |
| | | AA | SGGDYWS | YIYYSGSTYYNPSLKS | DGAALGMDV |
| | | | SEQ ID NO:7658 | SEQ ID NO:15670 | SEQ ID NO:23682 |
| iPS:437064 | | NA | AGTTACTACTGGAGC | TATATCTATTACAGTGGG AGTACTAAGTACAACCC CTCCCTCAAGAGT | AAGGGAGTGGGAACCATCCA CTTTGACTAC |
| | 21-225_200G8 | | SEQ ID NO:7659 | SEQ ID NO:15671 | SEQ ID NO:23683 |
| | | AA | SYYWS | YIYYSGSTKYNPSLKS | KGVGTIHFDY |
| | | | SEQ ID NO:7660 | SEQ ID NO:15672 | SEQ ID NO:23684 |
| iPS:437066 | | NA | AGTGGTGGTGACTACTGGAG C | TACATCTATTACAGTGGG AGCACCTACTACAACCC GTCCCTCAAGAGT | GATGGAGCAGCTCTGGGTAT GGACGTC |
| | 21-225_200G9 | | SEQ ID NO:7661 | SEQ ID NO:15673 | SEQ ID NO:23685 |
| | | AA | SGGDYWS | YIYYSGSTYYNPSLKS | DGAALGMDV |
| | | | SEQ ID NO:7662 | SEQ ID NO:15674 | SEQ ID NO:23686 |
| iPS:437068 | | NA | AGTGGTGGTGACTACTGGAG C | TACATCTATTACAGAGG GAGCACCTACTACAACC CGTCCCTCAAGAGT | GATGCAGCAGCCCACGGCAT GGACGTC |
| | 21-225_200A11 | | SEQ ID NO:7663 | SEQ ID NO:15675 | SEQ ID NO:23687 |
| | | AA | SGGDYWS | YIYYRGSTYYNPSLKS | DAAAHGMDV |
| | | | SEQ ID NO:7664 | SEQ ID NO:15676 | SEQ ID NO:23688 |

FIGURE 49

| iPS:437070 | | NA | CGCATCAATCCTACTTGGAAC | AGGACATACTACAGGTCCAAGTGGTATCATGTTTATGCAGTATCTGTGAAAAGT | GATCCTGGGGGGCTCTTTGACTAC |
|---|---|---|---|---|---|
| | 21-225_201G11 | | SEQ ID NO:7665 | SEQ ID NO:15677 | SEQ ID NO:23689 |
| | | AA | RINPTWN | RTYYRSKWYHVYAVSVKS | DPGGLFDY |
| | | | SEQ ID NO:7666 | SEQ ID NO:15678 | SEQ ID NO:23690 |
| iPS:437074 | | NA | AACTATGGCATGCAC | ATTATATGGTTTGATGGAAGTAATGAATACTATGCAGACTCCGTGAAGGGC | GAAAGTGGGAGCTATGCTCTTTATATC |
| | 21-225_203B2 | | SEQ ID NO:7667 | SEQ ID NO:15679 | SEQ ID NO:23691 |
| | | AA | NYGMH | IIWFDGSNEYYADSVKG | ESGSYALYI |
| | | | SEQ ID NO:7668 | SEQ ID NO:15680 | SEQ ID NO:23692 |
| iPS:437076 | | NA | CGCACCAATCCTACTTGGAAC | AGGACATACTACAGGTCCAAGTGGTATCATGTTTATGCACTATCTGTGAAAAGT | GATCCTGGGGGGCCTCTTTGACTAC |
| | 21-225_203G6 | | SEQ ID NO:7669 | SEQ ID NO:15681 | SEQ ID NO:23693 |
| | | AA | RTNPTWN | RTYYRSKWYHVYALSVKS | DPGGLFDY |
| | | | SEQ ID NO:7670 | SEQ ID NO:15682 | SEQ ID NO:23694 |
| iPS:437082 | | NA | AACTATGGCATGCAC | ATTATATGGTTTGATGGAAGTAATGAATACTATGCAGACTCCGTGAAGGGC | GAAAGTGGGAGCTATGCTCTTTATATC |
| | 21-225_205E12 | | SEQ ID NO:7671 | SEQ ID NO:15683 | SEQ ID NO:23695 |
| | | AA | NYGMH | IIWFDGSNEYYADSVKG | ESGSYALYI |
| | | | SEQ ID NO:7672 | SEQ ID NO:15684 | SEQ ID NO:23696 |

FIGURE 49

| iPS:437084 | | NA | AGCTATGGCATGCAC | GTTATATGGTATGATGG AAGTAATAAATACTATG CAGACTCCGTGAAGGGC | GAGGGTGGGAGCTACCACCT TGACTAC |
|---|---|---|---|---|---|
| | 21-225_206B5 | | SEQ ID NO:7673 | SEQ ID NO:15685 | SEQ ID NO:23697 |
| | | AA | SYGMH | VIWYDGSNKYYADSVKG | EGGSYHLDY |
| | | | SEQ ID NO:7674 | SEQ ID NO:15686 | SEQ ID NO:23698 |
| iPS:437086 | | NA | AGTTATAGCATGAAC | TACATTAGTAGTAGTAGT AGTATCAAAAAGTACGC AGACTCTGTGAAGGGC | GATGATGGGAGCTACTACTT TGACTAC |
| | 21-225_209A8 | | SEQ ID NO:7675 | SEQ ID NO:15687 | SEQ ID NO:23699 |
| | | AA | SYSMN | YISSSSSIKKYADSVKG | DDGSYYFDY |
| | | | SEQ ID NO:7676 | SEQ ID NO:15688 | SEQ ID NO:23700 |
| iPS:437088 | | NA | AGCTATGGCATGCAC | GTTATATGGTATGATGG AAGTAATAAATACTATG CAGACTCCGTGAAGGGC | GAGGGTGGGAGCTACCACCT TGACTAC |
| | 21-225_209H10 | | SEQ ID NO:7677 | SEQ ID NO:15689 | SEQ ID NO:23701 |
| | | AA | SYGMH | VIWYDGSNKYYADSVKG | EGGSYHLDY |
| | | | SEQ ID NO:7678 | SEQ ID NO:15690 | SEQ ID NO:23702 |
| iPS:437090 | | NA | AGTGGTGGTTCCTACTGGAG C | TACATCTATTACATTGGG ACCACCTACTACAACCC GTCCCTCAAGAGT | GATGAGCCATTGACCGGTAT GGACGTC |
| | 21-225_210F11 | | SEQ ID NO:7679 | SEQ ID NO:15691 | SEQ ID NO:23703 |
| | | AA | SGGSYWS | YIYYIGTTYYNPSLKS | DEPLTGMDV |
| | | | SEQ ID NO:7680 | SEQ ID NO:15692 | SEQ ID NO:23704 |

FIGURE 49

| iPS:437092 | | NA | GACTACTATATGAAC | TGGATCAACCCTAACAG TGGTGGCACAAACTATG CACAGAAGTTTCAGGGC | GGGTATGACTCGTTCGCCCC C |
|---|---|---|---|---|---|
| | 21-225_210B12 | | SEQ ID NO:7681 | SEQ ID NO:15693 | SEQ ID NO:23705 |
| | | AA | DYYMN | WINPNSGGTNYAQKFQG | GYDSFAP |
| | | | SEQ ID NO:7682 | SEQ ID NO:15694 | SEQ ID NO:23706 |
| iPS:437094 | | NA | CACTATGGCATGCAC | GTTATATGGTATGATGG AAGTAATAAATACTATG CAGACTCCGTGAAGGGC | GGGGACTGGAACCCCGAGGG TTTGGACGTC |
| | 21-225_210D12 | | SEQ ID NO:7683 | SEQ ID NO:15695 | SEQ ID NO:23707 |
| | | AA | HYGMH | VIWYDGSNKYYADSVKG | GDWNPEGLDV |
| | | | SEQ ID NO:7684 | SEQ ID NO:15696 | SEQ ID NO:23708 |
| iPS:437096 | | NA | AACTATGGCATGCAC | GTTATATGGTATGATGG AAGTAATAAATACTATG CAGACTCCGTGAAGGGC | GGGGACTGGAACCCCGAGGG TATGGACGTC |
| | 21-225_210E12 | | SEQ ID NO:7685 | SEQ ID NO:15697 | SEQ ID NO:23709 |
| | | AA | NYGMH | VIWYDGSNKYYADSVKG | GDWNPEGMDV |
| | | | SEQ ID NO:7686 | SEQ ID NO:15698 | SEQ ID NO:23710 |
| iPS:437098 | | NA | CACTATGGCATGCAC | GTTATATGGTATGATGG AAGTAATAAATACTATG CAGACTCCGTGAAGGGC | GGGGACTGGAACCCCGAGGG TTTGGACGTC |
| | 21-225_211C1 | | SEQ ID NO:7687 | SEQ ID NO:15699 | SEQ ID NO:23711 |
| | | AA | HYGMH | VIWYDGSNKYYADSVKG | GDWNPEGLDV |
| | | | SEQ ID NO:7688 | SEQ ID NO:15700 | SEQ ID NO:23712 |

FIGURE 49

| iPS:437100 | | NA | AGCTATGCCATGCAC | GTTATATGGTATGATGG AAGTAATAAATACTATG CAGACTCCGTGAAGGGC | GATCCTGGGAGCTACGGGTT CGACCCC |
|---|---|---|---|---|---|
| | 21-225_211H2 | | SEQ ID NO:7689 | SEQ ID NO:15701 | SEQ ID NO:23713 |
| | | AA | SYAMH | VIWYDGSNKYYADSVKG | DPGSYGFDP |
| | | | SEQ ID NO:7690 | SEQ ID NO:15702 | SEQ ID NO:23714 |
| iPS:437102 | | NA | AACTATGGCATGCAC | ATTATATGGTTTGATGGA AGTGATCAATACTATGC AGACTCCGTGAAGGGC | GGCCTCTCTGTCTACTACTAC GGTATGGGCGTC |
| | 21-225_211E5 | | SEQ ID NO:7691 | SEQ ID NO:15703 | SEQ ID NO:23715 |
| | | AA | NYGMH | IIWFDGSDQYYADSVKG | GLSVYYYGMGV |
| | | | SEQ ID NO:7692 | SEQ ID NO:15704 | SEQ ID NO:23716 |
| iPS:437104 | | NA | CACTATGGCATGCAC | GTTATATGGTATGATGG AAGTAATAAATACTATG CAGACTCCGTGAAGGGC | GGGGACTGGAACCCCGAGGG TTTGGACGTC |
| | 21-225_211G5 | | SEQ ID NO:7693 | SEQ ID NO:15705 | SEQ ID NO:23717 |
| | | AA | HYGMH | VIWYDGSNKYYADSVKG | GDWNPEGLDV |
| | | | SEQ ID NO:7694 | SEQ ID NO:15706 | SEQ ID NO:23718 |
| iPS:437106 | | NA | AGTGGTGGTTACTACTGGAG C | TACATCTATTACGTTGGG AGCACCTACTACAACCC GTCCCTCAAGAGT | GATGGGCCATTGAGCGGTAT GGACGTC |
| | 21-225_211H7 | | SEQ ID NO:7695 | SEQ ID NO:15707 | SEQ ID NO:23719 |
| | | AA | SGGYYWS | YIYYVGSTYYNPSLKS | DGPLSGMDV |
| | | | SEQ ID NO:7696 | SEQ ID NO:15708 | SEQ ID NO:23720 |

FIGURE 49

| iPS:437108 | 21-225_211C9 | NA | AGTGGTGGTGACTACTGGAG C | TACATCTATTATAGTGGG AGCACCTACTACAACCC GTCCCTCAAGAGT | GATTCAGCAGTGTACAATAT GGACGTC |
|---|---|---|---|---|---|
| | | | SEQ ID NO:7697 | SEQ ID NO:15709 | SEQ ID NO:23721 |
| | | AA | SGGDYWS | YIYYSGSTYYNPSLKS | DSAVYNMDV |
| | | | SEQ ID NO:7698 | SEQ ID NO:15710 | SEQ ID NO:23722 |
| iPS:437110 | 21-225_211E9 | NA | AGTGGTGGTGACTACTGGAG C | TACATCTATTACACTGGG AGCAACTACTACAACCC GTCCCTCAAGAGT | GATTCAGCAGTGTACGGTAT GGACGTC |
| | | | SEQ ID NO:7699 | SEQ ID NO:15711 | SEQ ID NO:23723 |
| | | AA | SGGDYWS | YIYYTGSNYYNPSLKS | DSAVYGMDV |
| | | | SEQ ID NO:7700 | SEQ ID NO:15712 | SEQ ID NO:23724 |
| iPS:437112 | 21-225_212C2 | NA | CACTATGGCATGCAC | GTTATATGGTATGATGG AAGTAATAAATACTATG CAGACTCCGTGAAGGGC | GGGGACTGGAACCCCGAGGG TATGGACGTC |
| | | | SEQ ID NO:7701 | SEQ ID NO:15713 | SEQ ID NO:23725 |
| | | AA | HYGMH | VIWYDGSNKYYADSVKG | GDWNPEGMDV |
| | | | SEQ ID NO:7702 | SEQ ID NO:15714 | SEQ ID NO:23726 |
| iPS:437114 | 21-225_212A4 | NA | AACTATGGCATGCAC | GTTATATGGTATGATGG AAGTAATAAATACTATG CAGACTCCGTGAAGGGC | GGGGACTGGAACCCCGAGGG TATGGACGTC |
| | | | SEQ ID NO:7703 | SEQ ID NO:15715 | SEQ ID NO:23727 |
| | | AA | NYGMH | VIWYDGSNKYYADSVKG | GDWNPEGMDV |
| | | | SEQ ID NO:7704 | SEQ ID NO:15716 | SEQ ID NO:23728 |

FIGURE 49

| iPS:437116 | | NA | CACTATGGCATGCAC | GTTATATGGTATGATGG AAGTAATAAATACTATG CGGACTCCGTGAAGGGC | GGGGACTGGAACCCCGAGGG TTTGGACGTC |
|---|---|---|---|---|---|
| | 21-225_212F6 | | SEQ ID NO:7705 | SEQ ID NO:15717 | SEQ ID NO:23729 |
| | | AA | HYGMH | VIWYDGSNKYYADSVKG | GDWNPEGLDV |
| | | | SEQ ID NO:7706 | SEQ ID NO:15718 | SEQ ID NO:23730 |
| iPS:437118 | | NA | CACTATGGCATGCAC | GTTATATGGTATGATGG AAGTAATAAATACTGTG CAGACTCCGTGAAGGGC | GGAGACTGGAACCCCGAGGG TTTGGACGTC |
| | 21-225_212G7 | | SEQ ID NO:7707 | SEQ ID NO:15719 | SEQ ID NO:23731 |
| | | AA | HYGMH | VIWYDGSNKYCADSVKG | GDWNPEGLDV |
| | | | SEQ ID NO:7708 | SEQ ID NO:15720 | SEQ ID NO:23732 |
| iPS:437120 | | NA | AGTGGTGGTGACTACTGGAG C | TATATGTATTACAGTGGG AGCACCTACTACAACCC GTCCCTCAAGAGT | GATTCAGCAGTGTACGGTAT GGACGTC |
| | 21-225_212A9 | | SEQ ID NO:7709 | SEQ ID NO:15721 | SEQ ID NO:23733 |
| | | AA | SGGDYWS | YMYYSGSTYYNPSLKS | DSAVYGMDV |
| | | | SEQ ID NO:7710 | SEQ ID NO:15722 | SEQ ID NO:23734 |
| iPS:437124 | | NA | AGTGGTGGTGACTACTGGAG T | TACATCTATTACAGTGGG AGCACCTACTACAACCC GTCCCTCAAGAGT | GATAGCAGCTCCTACGGTAT GGACGTC |
| | 21-225_212H12 | | SEQ ID NO:7711 | SEQ ID NO:15723 | SEQ ID NO:23735 |
| | | AA | SGGDYWS | YIYYSGSTYYNPSLKS | DSSSYGMDV |
| | | | SEQ ID NO:7712 | SEQ ID NO:15724 | SEQ ID NO:23736 |

FIGURE 49

| iPS:437128 | | NA | CACTATGGCATGCAC | GTTATATGGTATGATGG AAGTAATAAATACTATG CAGACTCCGTGAAGGGC | GGGGACTGGAACCCCGAGGG TATGGACGTC |
|---|---|---|---|---|---|
| | 21-225_213G3 | | SEQ ID NO:7713 | SEQ ID NO:15725 | SEQ ID NO:23737 |
| | | AA | HYGMH | VIWYDGSNKYYADSVKG | GDWNPEGMDV |
| | | | SEQ ID NO:7714 | SEQ ID NO:15726 | SEQ ID NO:23738 |
| iPS:437130 | | NA | CACTATGGCATGCAC | GTTATATGGTATGATGG AAGTAATAAATACTATG CAGACTCCGTGAAGGGC | GGGGACTGGAACCCCGAGGG TATGGACGTC |
| | 21-225_213D5 | | SEQ ID NO:7715 | SEQ ID NO:15727 | SEQ ID NO:23739 |
| | | AA | HYGMH | VIWYDGSNKYYADSVKG | GDWNPEGMDV |
| | | | SEQ ID NO:7716 | SEQ ID NO:15728 | SEQ ID NO:23740 |
| iPS:437132 | | NA | AGTGGTGGTGACTACTGGAG C | TACATCTATTATAGTGGG AGCACCTACTACAACCC GTCCCTCAAGAGT | GATTCAGCAGTGTACAATAT GGACGTC |
| | 21-225_213F5 | | SEQ ID NO:7717 | SEQ ID NO:15729 | SEQ ID NO:23741 |
| | | AA | SGGDYWS | YIYYSGSTYYNPSLKS | DSAVYNMDV |
| | | | SEQ ID NO:7718 | SEQ ID NO:15730 | SEQ ID NO:23742 |
| iPS:437134 | | NA | GACTACTATATGAAC | TGGATCAACCCTAAGAA TGGTGGCACAAACTATG CACAGAAGTTTCAGGGC | GGGTATGATTCGTTCGCCCC C |
| | 21-225_213A7 | | SEQ ID NO:7719 | SEQ ID NO:15731 | SEQ ID NO:23743 |
| | | AA | DYYMN | WINPKNGGTNYAQKFQG | GYDSFAP |
| | | | SEQ ID NO:7720 | SEQ ID NO:15732 | SEQ ID NO:23744 |

FIGURE 49

| iPS:437136 | 21-225_214H3 | NA | AGTGGTGGTGACTACTGGAG T | TACATCTATTACAGTGGG AGCACCTACTACAACCC GTCCCTCAAGAGT | GATAGCAGCTCCTACGGTAT GGACGTC |
|---|---|---|---|---|---|
| | | | SEQ ID NO:7721 | SEQ ID NO:15733 | SEQ ID NO:23745 |
| | | AA | SGGDYWS | YIYYSGSTYYNPSLKS | DSSSYGMDV |
| | | | SEQ ID NO:7722 | SEQ ID NO:15734 | SEQ ID NO:23746 |
| iPS:437138 | 21-225_214D8 | NA | ACTGCTTTTTACTACTGGAG C | TACATCTATTTCAGTGGG AGCACCTACTACAACCC GTCCCTCAAGAGT | GCAAGGGGATATCACTACAG TATCTTTGACTAC |
| | | | SEQ ID NO:7723 | SEQ ID NO:15735 | SEQ ID NO:23747 |
| | | AA | TAFYYWS | YIYFSGSTYYNPSLKS | ARGYHYSIFDY |
| | | | SEQ ID NO:7724 | SEQ ID NO:15736 | SEQ ID NO:23748 |
| iPS:437140 | 21-225_214E12 | NA | AGTGGTGGTGATTACTGGAG C | TACATCTATTACAGTGGG CCCACCTACTACAACCC GTCCCTCAAGAGT | GATGGGGCTGCGGAGGGTAT GGACGTC |
| | | | SEQ ID NO:7725 | SEQ ID NO:15737 | SEQ ID NO:23749 |
| | | AA | SGGDYWS | YIYYSGPTYYNPSLKS | DGAAEGMDV |
| | | | SEQ ID NO:7726 | SEQ ID NO:15738 | SEQ ID NO:23750 |
| iPS:437142 | 21-225_215A3 | NA | AGTGGTGGTGACTACTGGAG C | TACATCTATTACACTGGG AGCAACTACTACAACCC GTCCCTCAAGAGT | GATTCAGCAGTGTACGGTAT GGACGTC |
| | | | SEQ ID NO:7727 | SEQ ID NO:15739 | SEQ ID NO:23751 |
| | | AA | SGGDYWS | YIYYTGSNYYNPSLKS | DSAVYGMDV |
| | | | SEQ ID NO:7728 | SEQ ID NO:15740 | SEQ ID NO:23752 |
| iPS:437144 | 21-225_215B3 | NA | AACGCCTGGATGCAC | CGTATTAAAAGCAAAAC TAATGGTGGGACAACAG ACTACGCTGCACCCGTG AAAGGC | GATCCGGGGGGGGATCTTTGA CTAC |
| | | | SEQ ID NO:7729 | SEQ ID NO:15741 | SEQ ID NO:23753 |

FIGURE 49

| | | AA | NAWMH | RIKSKTNGGTTDYAAPVKG | DPGGIFDY |
|---|---|---|---|---|---|
| | | | SEQ ID NO:7730 | SEQ ID NO:15742 | SEQ ID NO:23754 |
| iPS:437146 | | NA | CACTATGGCATGCAC | GTTATATGGTATGATGGAAGTAATGAGTACTATGCAGACTCCGTGAAGGGC | GGGGACTGGAACCCCGAGGGTATGGACGTC |
| | 21-225_215D3 | | SEQ ID NO:7731 | SEQ ID NO:15743 | SEQ ID NO:23755 |
| | | AA | HYGMH | VIWYDGSNEYYADSVKG | GDWNPEGMDV |
| | | | SEQ ID NO:7732 | SEQ ID NO:15744 | SEQ ID NO:23756 |
| iPS:437148 | | NA | AGTGGTGGTGACTACTGGAGC | TATATGTATTACAGTGGGAGCACCTACTACAACCCGTCCCTCAAGAGT | GATTCAGCAGTGTACGGTATGGACGTC |
| | 21-225_215H3 | | SEQ ID NO:7733 | SEQ ID NO:15745 | SEQ ID NO:23757 |
| | | AA | SGGDYWS | YMYYSGSTYYNPSLKS | DSAVYGMDV |
| | | | SEQ ID NO:7734 | SEQ ID NO:15746 | SEQ ID NO:23758 |
| iPS:437150 | | NA | CACTATGGCATGCAC | GTTATATGGTATGATGGAAGTAATAAATACTATGCAGACTCCGTGAAGGGC | GGGGACTGGAACCCCGAGGGTTTGGACGTC |
| | 21-225_216A3 | | SEQ ID NO:7735 | SEQ ID NO:15747 | SEQ ID NO:23759 |
| | | AA | HYGMH | VIWYDGSNKYYADSVKG | GDWNPEGLDV |
| | | | SEQ ID NO:7736 | SEQ ID NO:15748 | SEQ ID NO:23760 |
| iPS:437154 | | NA | AGTGGTGGTGACTACTGGAGC | TATATGTATTACAGTGGGAGCACCTACTACAACCCGTCCCTCAAGAGT | GATTCAGCAGTGTACGGTATGGACGTC |
| | 21-225_216A7 | | SEQ ID NO:7737 | SEQ ID NO:15749 | SEQ ID NO:23761 |
| | | AA | SGGDYWS | YMYYSGSTYYNPSLKS | DSAVYGMDV |
| | | | SEQ ID NO:7738 | SEQ ID NO:15750 | SEQ ID NO:23762 |

FIGURE 49

| iPS:437158 | | NA | AGTGGTGGTGATTACTGGAGC | TACATCTATTACAGTGGGCCCACCTACTACAACCCGTCCCTCAAGAGT | GATGGGGCTGCGGAGGGTTTGGACGTC |
|---|---|---|---|---|---|
| | 21-225_216H11 | | SEQ ID NO:7739 | SEQ ID NO:15751 | SEQ ID NO:23763 |
| | | AA | SGGDYWS | YIYYSGPTYYNPSLKS | DGAAEGLDV |
| | | | SEQ ID NO:7740 | SEQ ID NO:15752 | SEQ ID NO:23764 |
| iPS:437160 | | NA | AGCTATGCCATGCAC | GTTATATGGTATGATGGAAGTAATAAATACTATGCAGACTCCGTGAAGGGC | GATCTGGGACTGGGATACTTCTTTGACTAC |
| | 21-225_216B12 | | SEQ ID NO:7741 | SEQ ID NO:15753 | SEQ ID NO:23765 |
| | | AA | SYAMH | VIWYDGSNKYYADSVKG | DLGLGYFFDY |
| | | | SEQ ID NO:7742 | SEQ ID NO:15754 | SEQ ID NO:23766 |
| iPS:437162 | | NA | AACTATGGCATGCAC | GTTATATGGTATGATGGAAGTAATAAATACTATGCAGACTCCGTGAAGGGC | GGGGACTGGAACCCCGAGGGTATGGACGTC |
| | 21-225_217B2 | | SEQ ID NO:7743 | SEQ ID NO:15755 | SEQ ID NO:23767 |
| | | AA | NYGMH | VIWYDGSNKYYADSVKG | GDWNPEGMDV |
| | | | SEQ ID NO:7744 | SEQ ID NO:15756 | SEQ ID NO:23768 |
| iPS:437164 | | NA | AACTATGGCATGCAC | ATTATATGGTTTGATGGAAGTGATGAATACTATGCAGACTCCGTGAAGGGC | GGCCTATCTGTCTACTACTACGGTATGGACGTC |
| | 21-225_217C6 | | SEQ ID NO:7745 | SEQ ID NO:15757 | SEQ ID NO:23769 |
| | | AA | NYGMH | IIWFDGSDEYYADSVKG | GLSVYYYGMDV |
| | | | SEQ ID NO:7746 | SEQ ID NO:15758 | SEQ ID NO:23770 |

FIGURE 49

| iPS:437166 | | NA | AACTATGGCATGCAC | ATTATATGGTTTGATGGA AGTGATCAGTACTATGC AGACTCCGTGAAGGGC | GGCCTCTCTGTCTACTACTAC GGTATGGACGTC |
|---|---|---|---|---|---|
| | 21-225_217G11 | | SEQ ID NO:7747 | SEQ ID NO:15759 | SEQ ID NO:23771 |
| | | AA | NYGMH | IIWFDGSDQYYADSVKG | GLSVYYYGMDV |
| | | | SEQ ID NO:7748 | SEQ ID NO:15760 | SEQ ID NO:23772 |
| iPS:437168 | | NA | AGCTATGGCTTGCAC | GTTATATGGTATGATGG AAGTAATAAATACTATG CAGACTCCGTGAAGGGC | TGGTACTACTATTACTACGG TATGGACGTC |
| | 21-225_218G4 | | SEQ ID NO:7749 | SEQ ID NO:15761 | SEQ ID NO:23773 |
| | | AA | SYGLH | VIWYDGSNKYYADSVKG | WYYYYYGMDV |
| | | | SEQ ID NO:7750 | SEQ ID NO:15762 | SEQ ID NO:23774 |
| iPS:437170 | | NA | AACTATGGCATGCAC | ATTATATGGTTTGATGGA AGTGATGAATACTATGC AGACTCCGTGAAGGGC | GGCCTATCTGTCTACTACTA CGGTATGGACGTC |
| | 21-225_218E5 | | SEQ ID NO:7751 | SEQ ID NO:15763 | SEQ ID NO:23775 |
| | | AA | NYGMH | IIWFDGSDEYYADSVKG | GLSVYYYGMDV |
| | | | SEQ ID NO:7752 | SEQ ID NO:15764 | SEQ ID NO:23776 |
| iPS:437172 | | NA | CACTATGGCATGCAC | GTCATATGGTATGATGG AAGTAATAAATACTATG CAGACTCCGTGAAGGGC | GGGGACTGGAACCCCGAGGG TATGGACGTC |
| | 21-225_219A7 | | SEQ ID NO:7753 | SEQ ID NO:15765 | SEQ ID NO:23777 |
| | | AA | HYGMH | VIWYDGSNKYYADSVKG | GDWNPEGMDV |
| | | | SEQ ID NO:7754 | SEQ ID NO:15766 | SEQ ID NO:23778 |

FIGURE 49

| iPS:437182 | | NA | CACTATGGCATGCAC | GTTATATGGTATGATGG AAGTAATAAATACTATG CAGACTCCGTGAAGGGC | GGGGACTGGAACCCCGAGGG TATGGACGTC |
|---|---|---|---|---|---|
| | 21-225_221H2 | | SEQ ID NO:7755 | SEQ ID NO:15767 | SEQ ID NO:23779 |
| | | AA | HYGMH | VIWYDGSNKYYADSVKG | GDWNPEGMDV |
| | | | SEQ ID NO:7756 | SEQ ID NO:15768 | SEQ ID NO:23780 |
| iPS:437184 | | NA | CACTATGGCATGCAC | GTTATATGGTATGATGG AAGTAATAAATACTATG CAGACTCCGTGAAGGGC | GGGGACTGGAACCCCGAGGG TATGGACGTC |
| | 21-225_221G4 | | SEQ ID NO:7757 | SEQ ID NO:15769 | SEQ ID NO:23781 |
| | | AA | HYGMH | VIWYDGSNKYYADSVKG | GDWNPEGMDV |
| | | | SEQ ID NO:7758 | SEQ ID NO:15770 | SEQ ID NO:23782 |
| iPS:437186 | | NA | AGCAACAGTGCTGCTTGGAAC | AGGACATACTACAGGTC CAAGTGGTATAATGATT ATGCAGTATCTGTGAAA AGT | GAGGGGGGCCTAGGATATTG TAGTAGTACCAGCTGCTATG GAGGCTGGTTCGACCCC |
| | 21-225_224H2 | | SEQ ID NO:7759 | SEQ ID NO:15771 | SEQ ID NO:23783 |
| | | AA | SNSAAWN | RTYYRSKWYNDYAVSVK S | EGGLGYCSSTSCYGGWFDP |
| | | | SEQ ID NO:7760 | SEQ ID NO:15772 | SEQ ID NO:23784 |
| iPS:437188 | | NA | GGCTACTATATACAC | TGGATCAACCCTAAAAA TGGTGGCACAAACTATG CACAGAAGTTTCAGGGC | GGAGCGTTTGATTACTTCTA CTACTACGCTATGGACGTC |
| | 21-225_224B11 | | SEQ ID NO:7761 | SEQ ID NO:15773 | SEQ ID NO:23785 |

FIGURE 49

| | | AA | GYYIH | WINPKNGGTNYAQKFQG | GAFDYFYYYAMDV |
|---|---|---|---|---|---|
| | | | SEQ ID NO:7762 | SEQ ID NO:15774 | SEQ ID NO:23786 |
| iPS:437190 | 21-225_225A9 | NA | ACCTATGGCATGCAC | GTTATATGGTATGATGG AAGTAATAAATACTATG CAGACTCCGTGAAGGGC | GATAACCACTATTGTAGTAG TACCAGCTGCTCCCCATACT ACTACTACTTCGGTATGGAC GTC |
| | | | SEQ ID NO:7763 | SEQ ID NO:15775 | SEQ ID NO:23787 |
| | | AA | TYGMH | VIWYDGSNKYYADSVKG | DNHYCSSTSCSPYYYYFGMDV |
| | | | SEQ ID NO:7764 | SEQ ID NO:15776 | SEQ ID NO:23788 |
| iPS:437192 | 21-225_225E9 | NA | AGCTATGGCATGCAC | GTTATGTGGTATGATGG AGGTAATAAAGACTATG CAGACTCCGTGAAGGGC | GATCGGGAATATTGTACTAG TACCAGCTGCCCTTACTACT ACTACTACGGTATGGACGTC |
| | | | SEQ ID NO:7765 | SEQ ID NO:15777 | SEQ ID NO:23789 |
| | | AA | SYGMH | VMWYDGGNKDYADSVKG | DREYCTSTSCPYYYYYGMDV |
| | | | SEQ ID NO:7766 | SEQ ID NO:15778 | SEQ ID NO:23790 |
| iPS:437194 | 21-225_226B2 | NA | GGCTACTTTATGCAC | TGGATCAACCCTAACAG TGGTGACACAAACTATG CACAGAAGTTTCAGGGC | GGGACTTACTATGGTTCGGG GAGTTATTTTAACGAACTTG ACTCC |
| | | | SEQ ID NO:7767 | SEQ ID NO:15779 | SEQ ID NO:23791 |
| | | AA | GYFMH | WINPNSGDTNYAQKFQG | GTYYGSGSYFNELDS |
| | | | SEQ ID NO:7768 | SEQ ID NO:15780 | SEQ ID NO:23792 |
| iPS:437196 | 21-225_226B7 | NA | GGCTACTATATGCAC | TGGATCAACCCTAACAG TGGAGGCACAAACTATG CACAGAAGTTTCAGGAC | GGATATTACTATGGTTCGGG GAGTTATTATAACTGGTTCG ACTCC |
| | | | SEQ ID NO:7769 | SEQ ID NO:15781 | SEQ ID NO:23793 |
| | | AA | GYYMH | WINPNSGGTNYAQKFQD | GYYYGSGSYYNWFDS |

FIGURE 49

| | | | SEQ ID NO:7770 | SEQ ID NO:15782 | SEQ ID NO:23794 |
|---|---|---|---|---|---|
| iPS:437198 | 21-225_226F8 | NA | GGCTACTATATACAC | TGGATCAACCCTAACAG TGGTGGCACAAACTATG CACGGAAGTTTCAGGGC | GGAGCGTTTGATTACTACTA CTACTACGCTTTGGACGTC |
| | | | SEQ ID NO:7771 | SEQ ID NO:15783 | SEQ ID NO:23795 |
| | | AA | GYYIH | WINPNSGGTNYARKFQG | GAFDYYYYYALDV |
| | | | SEQ ID NO:7772 | SEQ ID NO:15784 | SEQ ID NO:23796 |
| iPS:437200 | 21-225_226A10 | NA | GGCTACTTTATGCAC | TGGATCAACCCTAACAG TGGTGACACAAACTATG CACAGAAGTTTCAGGGC | GGGACTTACTATGGTTCGGG GAGTTATTTTAACGAACTTG ACTCC |
| | | | SEQ ID NO:7773 | SEQ ID NO:15785 | SEQ ID NO:23797 |
| | | AA | GYFMH | WINPNSGDTNYAQKFQG | GTYYGSGSYFNELDS |
| | | | SEQ ID NO:7774 | SEQ ID NO:15786 | SEQ ID NO:23798 |
| iPS:437202 | 21-225_227D3 | NA | GGCTACTATATGCAC | TGGATCAACCCTAAGAG TGGTGGCACAAACTTTG CACAGAAGTTTCAGGGC | GGAGCGTTTGATTACTTCTA CTACTACGGTATGGACGTC |
| | | | SEQ ID NO:7775 | SEQ ID NO:15787 | SEQ ID NO:23799 |
| | | AA | GYYMH | WINPKSGGTNFAQKFQG | GAFDYFYYYGMDV |
| | | | SEQ ID NO:7776 | SEQ ID NO:15788 | SEQ ID NO:23800 |
| iPS:437204 | 21-225_227E5 | NA | AGCTATGCCATGAGC | GCTATTAGTGGTAGTGGT GGTAGCACATACTACGC AGACTCCGTGAAGGGC | GAATATTGTGGTGGTGACTG CTATTCCCCTTACTACTACTA CTACGGTATGGACGTC |
| | | | SEQ ID NO:7777 | SEQ ID NO:15789 | SEQ ID NO:23801 |
| | | AA | SYAMS | AISGSGGSTYYADSVKG | EYCGGDCYSPYYYYYGMDV |
| | | | SEQ ID NO:7778 | SEQ ID NO:15790 | SEQ ID NO:23802 |
| iPS:437208 | 21-225_227C10 | NA | GGCTACTATATGCAC | TGGATCAACCCTAAGAG TGGTGGCACAAACTATG CACAGAAGTTTCAGGGC | GGAGCGTTTGATTACTTCTA CTACTACGGTATGGACGTC |
| | | | SEQ ID NO:7779 | SEQ ID NO:15791 | SEQ ID NO:23803 |

FIGURE 49

| | | | | | |
|---|---|---|---|---|---|
| | | AA | GYYMH | WINPKSGGTNYAQKFQG | GAFDYFYYYGMDV |
| | | | SEQ ID NO:7780 | SEQ ID NO:15792 | SEQ ID NO:23804 |
| iPS:437210 | 21-225_227E12 | NA | ACTAGTGGAGTGGGTGTGGGC | CTCATTTATTGGAATGATGATAAGGTCTACAGCCCATCTCTGAAGAGC | AGGGGACAGCAGCTGGCCCTCGACTAC |
| | | | SEQ ID NO:7781 | SEQ ID NO:15793 | SEQ ID NO:23805 |
| | | AA | TSGVGVG | LIYWNDDKVYSPSLKS | RGQQLALDY |
| | | | SEQ ID NO:7782 | SEQ ID NO:15794 | SEQ ID NO:23806 |
| iPS:437214 | 21-225_48B12 | NA | AGCTATGCCATGAAC | GCTATTAGTGGTCGTGGTGGTAACACATTCTACGCAGACTCCGTGAAGGGC | AGAGAGACGTATAACTGGAACTACGAAGGGTTTGACTAC |
| | | | SEQ ID NO:7783 | SEQ ID NO:15795 | SEQ ID NO:23807 |
| | | AA | SYAMN | AISGRGGNTFYADSVKG | RETYNWNYEGFDY |
| | | | SEQ ID NO:7784 | SEQ ID NO:15796 | SEQ ID NO:23808 |
| iPS:437216 | 21-225_51D5 | NA | AGCTATGTCATGAGC | ACTATGAGTGGTAGTGGTGGTCGCACATACTACGCAGACTCCGTGAACGGC | GTGACTGCTTTTGACTAC |
| | | | SEQ ID NO:7785 | SEQ ID NO:15797 | SEQ ID NO:23809 |
| | | AA | SYVMS | TMSGSGGRTYYADSVNG | VTAFDY |
| | | | SEQ ID NO:7786 | SEQ ID NO:15798 | SEQ ID NO:23810 |
| iPS:437220 | 21-225_55H6 | NA | AGCTATAGCATGAAC | TCCATTAGTGGTAGTAGTACTTACATATACTACGCAGACTCAGTGAAGGGC | ACTGGGGTCTTTGACTAC |
| | | | SEQ ID NO:7787 | SEQ ID NO:15799 | SEQ ID NO:23811 |
| | | AA | SYSMN | SISGSSTYIYYADSVKG | TGVFDY |
| | | | SEQ ID NO:7788 | SEQ ID NO:15800 | SEQ ID NO:23812 |
| iPS:437224 | 21-225_56H1 | NA | AACTATAGAATGAAC | TCCATTAGTGGTAGTAGTACTGACATATACTACGCAGACTCAGTGAAGGGC | GTGGCCTCCTTTGACTAC |
| | | | SEQ ID NO:7789 | SEQ ID NO:15801 | SEQ ID NO:23813 |

FIGURE 49

| | | AA | NYRMN | SISGSSTDIYYADSVKG | VASFDY |
|---|---|---|---|---|---|
| | | | SEQ ID NO:7790 | SEQ ID NO:15802 | SEQ ID NO:23814 |
| iPS:437226 | 21-225_57C2 | NA | AGCTTTGGCATGAAC | TCTATTAGTAGTAGTACTGGTTACATATACAACGCAGACTCAGTGAAGGGC | ACCTATAGTGGGAGCCTGGACGTC |
| | | | SEQ ID NO:7791 | SEQ ID NO:15803 | SEQ ID NO:23815 |
| | | AA | SFGMN | SISSSTGYIYNADSVKG | TYSGSLDV |
| | | | SEQ ID NO:7792 | SEQ ID NO:15804 | SEQ ID NO:23816 |
| iPS:437228 | 21-225_60C11 | NA | AGCTATGCCATGAGC | GCTATTAGTGGTAGTGGTGGTAACACATTCTACGCAGACTCCGTGAAGGGC | TTTTTCGGTGTAGTGGGAGTCGGGGTGCTTTGACTAC |
| | | | SEQ ID NO:7793 | SEQ ID NO:15805 | SEQ ID NO:23817 |
| | | AA | SYAMS | AISGSGGNTFYADSVKG | FFGVVGVGCFDY |
| | | | SEQ ID NO:7794 | SEQ ID NO:15806 | SEQ ID NO:23818 |
| iPS:437230 | 21-225_62H10 | NA | AGCTATAGCATGAAC | TCCATTAGTAGTAGTAGTAGTTACATATACTACGCAGACTCAGTGAAGGGC | GGGGGGTTCGAGGGGGGTTCGACCCC |
| | | | SEQ ID NO:7795 | SEQ ID NO:15807 | SEQ ID NO:23819 |
| | | AA | SYSMN | SISSSSSYIYYADSVKG | GGSRGFDP |
| | | | SEQ ID NO:7796 | SEQ ID NO:15808 | SEQ ID NO:23820 |
| iPS:437232 | 21-225_63E1 | NA | ACTTCTGCCATGAGC | GCTATTAGTGGTAGTGGTGCTAACACATTCTACGCAGACTCCGTGAAGGGC | GTTATAGCAGTGGCTGGAGGGCACTTTTTCGACCCC |
| | | | SEQ ID NO:7797 | SEQ ID NO:15809 | SEQ ID NO:23821 |
| | | AA | TSAMS | AISGSGANTFYADSVKG | VIAVAGGHFFDP |
| | | | SEQ ID NO:7798 | SEQ ID NO:15810 | SEQ ID NO:23822 |
| iPS:437234 | 21 225 64E2 | NA | GGCTACTATATGCAC | TGGATCAACCCTAACAATAATGGCACAAACTATGCACAGAAGTTTCAGGGC | GATGGGAGCAGTGGCTTTGACTAC |

FIGURE 49

| | | | | | |
|---|---|---|---|---|---|
| | **21-225_64E2** | | SEQ ID NO:7799 | SEQ ID NO:15811 | SEQ ID NO:23823 |
| | | AA | GYYMH | WINPNNNGTNYAQKFQG | DGSSGFDY |
| | | | SEQ ID NO:7800 | SEQ ID NO:15812 | SEQ ID NO:23824 |
| **iPS:437248** | **21-225_97H3** | NA | GATTACTACTGGAGC | GAAATCAATCATAGTGG AGACACCAACTACAACC CGTCCCTCAAGAGT | GAGTTTCCATATAGTGGAAG CTACCTCTACTACTACGGTA TGGACGTC |
| | | | SEQ ID NO:7801 | SEQ ID NO:15813 | SEQ ID NO:23825 |
| | | AA | DYYWS | EINHSGDTNYNPSLKS | EFPYSGSYLYYYGMDV |
| | | | SEQ ID NO:7802 | SEQ ID NO:15814 | SEQ ID NO:23826 |
| **iPS:437250** | **21-225_148C6** | NA | AGCTATGCCATGAGC | GTTATTAGTGGTGGTGGT AGTAGTACATACTACGC AGACTCCGTGAAGGGC | TGGCGAGGTAACCCCACTGA CTACGGTATGGACGTC |
| | | | SEQ ID NO:7803 | SEQ ID NO:15815 | SEQ ID NO:23827 |
| | | AA | SYAMS | VISGGGSSTYYADSVKG | WRGNPTDYGMDV |
| | | | SEQ ID NO:7804 | SEQ ID NO:15816 | SEQ ID NO:23828 |
| **iPS:437252** | **21-225_148H11** | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | | | SEQ ID NO:7805 | SEQ ID NO:15817 | SEQ ID NO:23829 |
| | | AA | SYAMS | VISGGGSSTYYADSVKG | WRGNPTDYGMDV |
| | | | SEQ ID NO:7806 | SEQ ID NO:15818 | SEQ ID NO:23830 |
| **iPS:437254** | **21-225_149F2** | NA | CGCTATGGCATGCAC | TTTATATGGTATGATGGA AGTGAGAACTACTATGC AGACTCCGTGAAGGGC | GATCGGGTGGAGGGTTCGGG GACTCCCTACTACTACTACG GTATGGACGTC |
| | | | SEQ ID NO:7807 | SEQ ID NO:15819 | SEQ ID NO:23831 |
| | | AA | RYGMH | FIWYDGSENYYADSVKG | DRVEGSGTPYYYYGMDV |
| | | | SEQ ID NO:7808 | SEQ ID NO:15820 | SEQ ID NO:23832 |

FIGURE 49

| iPS:437256 | | NA | CGCTATGGCATGCAC | TTTATATGGTATGATGGA AGTGAGAACTACTATGC AGACTCCGTGAAGGGC | GATCGGGTGGAGGGTTCGGG GACTCCCTACTACTACTACG GTATGGACGTC |
|---|---|---|---|---|---|
| | 21-225_150F11 | | SEQ ID NO:7809 | SEQ ID NO:15821 | SEQ ID NO:23833 |
| | | AA | RYGMH | FIWYDGSENYYADSVKG | DRVEGSGTPYYYYGMDV |
| | | | SEQ ID NO:7810 | SEQ ID NO:15822 | SEQ ID NO:23834 |
| iPS:437258 | | NA | ACCTATGGCATGCAC | GTTATATGGTATGGTGG AAGTGATACAGACTATG CAGACTCCGTGAGGGGC | GATCGGGATTATTGTAGTGG TGGTAACTGCCCTTACTACT ACTACTACGGTATGGACGTC |
| | 21-225_153F9 | | SEQ ID NO:7811 | SEQ ID NO:15823 | SEQ ID NO:23835 |
| | | AA | TYGMH | VIWYGGSDTDYADSVRG | DRDYCSGGNCPYYYYYGMDV |
| | | | SEQ ID NO:7812 | SEQ ID NO:15824 | SEQ ID NO:23836 |
| iPS:437260 | | NA | GGCTACTTTATGCAC | TGGATCAAGCCTAAAAG CGGTGGCACAAACTGTG CACAGAAGTTTCAGGGC | GGGGGGGCTACGGTGACTAC GTGGGGGGGTCTTTGACTAC |
| | 21-225_170D1 | | SEQ ID NO:7813 | SEQ ID NO:15825 | SEQ ID NO:23837 |
| | | AA | GYFMH | WIKPKSGGTNCAQKFQG | GGATVTTWGVFDY |
| | | | SEQ ID NO:7814 | SEQ ID NO:15826 | SEQ ID NO:23838 |
| iPS:437262 | | NA | AGCTATAGCATGAAC | TACATTAGCAGTAGTGG TAGTACCAAATACTACG CAGACTCTGTGGAGGGC | GATAGTAGGAAGGGGTTCTA CTACGGTCTGGACGTC |
| | 21-225_170E4 | | SEQ ID NO:7815 | SEQ ID NO:15827 | SEQ ID NO:23839 |
| | | AA | SYSMN | YISSSGSTKYYADSVEG | DSRKGFYYGLDV |
| | | | SEQ ID NO:7816 | SEQ ID NO:15828 | SEQ ID NO:23840 |
| iPS:437264 | 21 225 171H12 | NA | GGCTACTTTATGCAC | TGGATCAAGCCTAAGAG TGGTGGCACAAACTCTG CACAGAGGTTTCAGGGC | GGGGGGGACTACGGTGGCTAC GTGGGGGGGTCTTTGACTAC |

FIGURE 49

| | | | | | |
|---|---|---|---|---|---|
| | 21-225_171H12 | | SEQ ID NO:7817 | SEQ ID NO:15829 | SEQ ID NO:23841 |
| | | AA | GYFMH | WIKPKSGGTNSAQRFQG | GGTTVATWGVFDY |
| | | | SEQ ID NO:7818 | SEQ ID NO:15830 | SEQ ID NO:23842 |
| iPS:437266 | 21-225_177A5 | NA | GGCTACTTTATGCAC | TGGATCAAGCCTAAGAG TGGTGGCACAAACTCTG CACAGAGGTTTCAGGGC | GGGGGGGACTACGGTGGCTAC GTGGGGGGTCTTTGACTAC |
| | | | SEQ ID NO:7819 | SEQ ID NO:15831 | SEQ ID NO:23843 |
| | | AA | GYFMH | WIKPKSGGTNSAQRFQG | GGTTVATWGVFDY |
| | | | SEQ ID NO:7820 | SEQ ID NO:15832 | SEQ ID NO:23844 |
| iPS:437268 | 21-225_177D2 | NA | AGCTATGGCATGGAC | ATTATATGGTTTGATGGA AGTAATAAATACTATGC AGACTCCGTGAAGGGC | GCATATTGTGGTGGTGACTG CTATTTCCCCCATCTCCATTA CTACGGTATGGACGTC |
| | | | SEQ ID NO:7821 | SEQ ID NO:15833 | SEQ ID NO:23845 |
| | | AA | SYGMD | IIWFDGSNKYYADSVKG | AYCGGDCYFPHLHYYGMDV |
| | | | SEQ ID NO:7822 | SEQ ID NO:15834 | SEQ ID NO:23846 |
| iPS:437270 | 21-225_178H4 | NA | GGCTACTTTATGCAC | TGGATCAAGCCTAAAAG TGGTGGCACAAACTGTG CACAGAAGTTTCAGGGC | GGGGGGGACTACGGTGACTAC GTGGGGGGGTCTTTGACTAC |
| | | | SEQ ID NO:7823 | SEQ ID NO:15835 | SEQ ID NO:23847 |
| | | AA | GYFMH | WIKPKSGGTNCAQKFQG | GGTTVTTWGVFDY |
| | | | SEQ ID NO:7824 | SEQ ID NO:15836 | SEQ ID NO:23848 |
| iPS:437274 | 21-225_196D4 | NA | AGCTATGGCATGCAC | GTTATATGGTATGATGG AAGTAATAGAAACTATG CAGACTCCGTGAAGGGC | GATCGGTCTAAGGGTTACGA CGGTATGGACGTC |
| | | | SEQ ID NO:7825 | SEQ ID NO:15837 | SEQ ID NO:23849 |

FIGURE 49

| | | AA | SYGMH | VIWYDGSNRNYADSVKG | DRSKGYDGMDV |
|---|---|---|---|---|---|
| | | | SEQ ID NO:7826 | SEQ ID NO:15838 | SEQ ID NO:23850 |
| iPS:437280 | | NA | GACTATGGCATGCAC | GTTATATGGTATGATGG AGGTAATACACATTATA CAGACTCCGTGAAGGGC | GAAGTGGGTTGGCTTGATGA CTAC |
| | 21-225_203C10 | | SEQ ID NO:7827 | SEQ ID NO:15839 | SEQ ID NO:23851 |
| | | AA | DYGMH | VIWYDGGNTHYTDSVKG | EVGWLDDY |
| | | | SEQ ID NO:7828 | SEQ ID NO:15840 | SEQ ID NO:23852 |
| iPS:437282 | | NA | AGCTATGCCATGAGC | GCTATTAGTGGTAGTGGT GGTAGCACATACTACGC AGACTCCGTGAAGGGC | GCTGGTGGAACTACGGGGAG CTACTACTACAACGGTATGG ACGTC |
| | 21-225_207C9 | | SEQ ID NO:7829 | SEQ ID NO:15841 | SEQ ID NO:23853 |
| | | AA | SYAMS | AISGSGGSTYYADSVKG | AGGTTGSYYYNGMDV |
| | | | SEQ ID NO:7830 | SEQ ID NO:15842 | SEQ ID NO:23854 |
| iPS:437286 | | NA | GACTATGTCATGCAC | GTTATATGGTATGATGG AAGTAATAAATACTATG TAGACTCCGTGAAGGGC | GAACGGTATAGCAGTGGCTT GTACGACTACGGTATGGACG TC |
| | 21-225_208F1 | | SEQ ID NO:7831 | SEQ ID NO:15843 | SEQ ID NO:23855 |
| | | AA | DYVMH | VIWYDGSNKYYVDSVKG | ERYSSGLYDYGMDV |
| | | | SEQ ID NO:7832 | SEQ ID NO:15844 | SEQ ID NO:23856 |
| iPS:437290 | | NA | GACTATGTCATGCAC | GTTATATGGTATGATGG AAGTAATAAATACTATG TAGACTCCGTGAAGGGC | GAACGGTATAGCAGTGGCTT GTACGACTACGGTATGGACG TC |
| | 21-225_210G6 | | SEQ ID NO:7833 | SEQ ID NO:15845 | SEQ ID NO:23857 |

FIGURE 49

| | | | | | |
|---|---|---|---|---|---|
| | | AA | DYVMH | VIWYDGSNKYYVDSVKG | ERYSSGLYDYGMDV |
| | | | SEQ ID NO:7834 | SEQ ID NO:15846 | SEQ ID NO:23858 |
| iPS:437294 | 21-225_216D5 | NA | AGTGGTGGTTACTACTGGAGC | TACATCTATTACAGTGGGAGCACCTACTACAACCCGTCCCTCAAGAGT | GATTCCCCTGACAGGGGGTTTGACTAC |
| | | | SEQ ID NO:7835 | SEQ ID NO:15847 | SEQ ID NO:23859 |
| | | AA | SGGYYWS | YIYYSGSTYYNPSLKS | DSPDRGFDY |
| | | | SEQ ID NO:7836 | SEQ ID NO:15848 | SEQ ID NO:23860 |
| iPS:437302 | 21-225_225B11 | NA | AGCTATGGCATGCAC | ATTATATCATATAGTGGAAGTAATAAATACTATGCAGACTCCGTGAAGGGC | CGGGGATACAGCTATGGCGGGTACGGTATGGACGTC |
| | | | SEQ ID NO:7837 | SEQ ID NO:15849 | SEQ ID NO:23861 |
| | | AA | SYGMH | IISYSGSNKYYADSVKG | RGYSYGGYGMDV |
| | | | SEQ ID NO:7838 | SEQ ID NO:15850 | SEQ ID NO:23862 |
| iPS:437320 | 21-225_75A1 | NA | GATTACTACTGGAGC | GAAATCAATCATAGTGGAGACACCAACTACAACCCGTCCCTCAAGAGT | GAGTTTCCATATAGTGGAAGCTACCTCTACTACTACGGTATGGACGTC |
| | | | SEQ ID NO:7839 | SEQ ID NO:15851 | SEQ ID NO:23863 |
| | | AA | DYYWS | EINHSGDTNYNPSLKS | EFPYSGSYLYYYGMDV |
| | | | SEQ ID NO:7840 | SEQ ID NO:15852 | SEQ ID NO:23864 |
| iPS:437322 | 21-225_75B1 | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | | | SEQ ID NO:7841 | SEQ ID NO:15853 | SEQ ID NO:23865 |
| | | AA | NYDIN | WMHPNSGNTGYAQKFQG | SSGWYWFDP |
| | | | SEQ ID NO:7842 | SEQ ID NO:15854 | SEQ ID NO:23866 |

FIGURE 49

| iPS:437324 | 21-225_75C2 | NA | GGTTGCTACTGGAGC | GAAATCAATCATAGTGG AAGCACCAACTACAACC CGTCCCTCAAGAGT | GACTACGGCGGTATGGACGT C |
|---|---|---|---|---|---|
| | | | SEQ ID NO:7843 | SEQ ID NO:15855 | SEQ ID NO:23867 |
| | | AA | GCYWS | EINHSGSTNYNPSLKS | DYGGMDV |
| | | | SEQ ID NO:7844 | SEQ ID NO:15856 | SEQ ID NO:23868 |
| iPS:437326 | 21-225_75C10 | NA | AGTTATGGCATGCAT | GTTATATGGTATGATGG AAGTGATAAATACTATG CAGACTCCGTGAAGGGC | GATCGGTTAGTGGGAGCTAC GGTTGATGCTTTTGATATC |
| | | | SEQ ID NO:7845 | SEQ ID NO:15857 | SEQ ID NO:23869 |
| | | AA | SYGMH | VIWYDGSDKYYADSVKG | DRLVGATVDAFDI |
| | | | SEQ ID NO:7846 | SEQ ID NO:15858 | SEQ ID NO:23870 |
| iPS:437328 | 21-225_75D3 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:7847 | SEQ ID NO:15859 | SEQ ID NO:23871 |
| | | AA | GCYWS | EINHSGSTNYNPSLKS | DYGGMDV |
| | | | SEQ ID NO:7848 | SEQ ID NO:15860 | SEQ ID NO:23872 |
| iPS:437332 | 21-225_75F3 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:7849 | SEQ ID NO:15861 | SEQ ID NO:23873 |
| | | AA | GCYWS | EINHSGSTNYNPSLKS | DYGGMDV |
| | | | SEQ ID NO:7850 | SEQ ID NO:15862 | SEQ ID NO:23874 |
| iPS:437334 | 21-225_75F11 | NA | ACTGGTGGAGTGGGTGTGGG C | CTCATTTATTGGGATGAT GATAAGCGCTACAGCCC ATCTCTGAAGAGC | CTTATAGCAGTGGCCTTTGA CTAC |
| | | | SEQ ID NO:7851 | SEQ ID NO:15863 | SEQ ID NO:23875 |
| | | AA | TGGVGVG | LIYWDDDKRYSPSLKS | LIAVAFDY |

FIGURE 49

| | | | SEQ ID NO:7852 | SEQ ID NO:15864 | SEQ ID NO:23876 |
|---|---|---|---|---|---|
| iPS:437340 | | NA | GGTTGCTACTGGAGC | GAAATCAATCATAGTGG AAGGACCAACTACAACC CGTCCCTCAAGAGT | GACTACGGTGGGCTTGACTA C |
| | 21-225_75G9 | | SEQ ID NO:7853 | SEQ ID NO:15865 | SEQ ID NO:23877 |
| | | AA | GCYWS | EINHSGRTNYNPSLKS | DYGGLDY |
| | | | SEQ ID NO:7854 | SEQ ID NO:15866 | SEQ ID NO:23878 |
| iPS:437344 | | NA | GGTTGCTACTGGAGC | GAAATCAATCATAGTGG AAGCACCAACTACAACC CGTCCCTCAAGAGT | GACTACGGCGGTATGGACGT C |
| | 21-225_75G12 | | SEQ ID NO:7855 | SEQ ID NO:15867 | SEQ ID NO:23879 |
| | | AA | GCYWS | EINHSGSTNYNPSLKS | DYGGMDV |
| | | | SEQ ID NO:7856 | SEQ ID NO:15868 | SEQ ID NO:23880 |
| iPS:437346 | | NA | AGGAGTAGTTACTACTGGGG C | AGTATCTATTATAGTGGG AGCGCCTACTCCAACCC GTCCCTCAAGAGT | CTTGACTCTAACTGGGGTCT TGACTAC |
| | 21-225_75H7 | | SEQ ID NO:7857 | SEQ ID NO:15869 | SEQ ID NO:23881 |
| | | AA | RSSYYWG | SIYYSGSAYSNPSLKS | LDSNWGLDY |
| | | | SEQ ID NO:7858 | SEQ ID NO:15870 | SEQ ID NO:23882 |
| iPS:437350 | | NA | GGTTGCTACTGGAGC | GAAATCAATCATAGTGG AAGCACCAACTACAACC CGTCCCTCAAGAGT | GACTACGGCGGTATGGACGT C |
| | 21-225_74A3 | | SEQ ID NO:7859 | SEQ ID NO:15871 | SEQ ID NO:23883 |
| | | AA | GCYWS | EINHSGSTNYNPSLKS | DYGGMDV |
| | | | SEQ ID NO:7860 | SEQ ID NO:15872 | SEQ ID NO:23884 |
| iPS:437356 | | NA | AATTATGATATCAAC | TGGATGAACCCTAACAG TGGTAACACAGGCTATG CACAGAAGTTCCAGGGC | ACCAGTGGCTGGAACTTCTT TGACTAC |
| | 21-225_74B1 | | SEQ ID NO:7861 | SEQ ID NO:15873 | SEQ ID NO:23885 |

FIGURE 49

| | | | | | | |
|---|---|---|---|---|---|---|
| | | AA | NYDIN | WMNPNSGNTGYAQKFQG | TSGWNFFDY |
| | | | SEQ ID NO:7862 | SEQ ID NO:15874 | SEQ ID NO:23886 |
| iPS:437361 | 21-225_74C1 | NA | AATTATGATATCAAC | TGGATGAACCCTGACAG TGGTAACACAGGCTTTG CACAGAAGTTCCAGGGC | TCCAGTGGCTGGTACTGGTT CGACCCC |
| | | | SEQ ID NO:7863 | SEQ ID NO:15875 | SEQ ID NO:23887 |
| | | AA | NYDIN | WMNPDSGNTGFAQKFQG | SSGWYWFDP |
| | | | SEQ ID NO:7864 | SEQ ID NO:15876 | SEQ ID NO:23888 |
| iPS:437363 | 21-225_74C10 | NA | AATTATGATATCAAC | TGGATGAACCCTAACAG TGGTAACATAGGCTATG CACAGAAGTTCCAGGGC | AGCAGTGGCTGGTACTGGTT CGACCCC |
| | | | SEQ ID NO:7865 | SEQ ID NO:15877 | SEQ ID NO:23889 |
| | | AA | NYDIN | WMNPNSGNIGYAQKFQG | SSGWYWFDP |
| | | | SEQ ID NO:7866 | SEQ ID NO:15878 | SEQ ID NO:23890 |
| iPS:437369 | 21-225_74D6 | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:7867 | SEQ ID NO:15879 | SEQ ID NO:23891 |
| | | AA | GCYWS | EINHSGSTNYNPSLKS | DYGGMDV |
| | | | SEQ ID NO:7868 | SEQ ID NO:15880 | SEQ ID NO:23892 |
| iPS:437371 | 21-225_74D8 | NA | AACTACGACATGCAC | GCTATTGGTACTGCTGGT GACACATACTATCCAGG CTCCGTGAAGGGC | GTTCTTGACTACGGTGACTC CTTGGGCTACTACTACTACG GTATGGACGTC |
| | | | SEQ ID NO:7869 | SEQ ID NO:15881 | SEQ ID NO:23893 |
| | | AA | NYDMH | AIGTAGDTYYPGSVKG | VLDYGDSLGYYYYGMDV |
| | | | SEQ ID NO:7870 | SEQ ID NO:15882 | SEQ ID NO:23894 |

FIGURE 49

| iPS:437377 | 21-225_74G9 | NA | ACTGGTGGAGTGGGTGTGGGC | CTCATTTATTGGGATGATGATAAGCGCTACAGCCCATCTCTGAAGAGC | CTTATAGCAGTGGCCTTTGACTAC |
|---|---|---|---|---|---|
| | | | SEQ ID NO:7871 | SEQ ID NO:15883 | SEQ ID NO:23895 |
| | | AA | TGGVGVG | LIYWDDDKRYSPSLKS | LIAVAFDY |
| | | | SEQ ID NO:7872 | SEQ ID NO:15884 | SEQ ID NO:23896 |
| iPS:437379 | 21-225_74H2 | NA | AATTATGATATCAAC | TGGATGCACCCTAACAGTGGTAACACAGGCTTTGCACAGAAGTTCCAGGGC | TCCAGTGGCTGGTACTGGTTCGACCCC |
| | | | SEQ ID NO:7873 | SEQ ID NO:15885 | SEQ ID NO:23897 |
| | | AA | NYDIN | WMHPNSGNTGFAQKFQG | SSGWYWFDP |
| | | | SEQ ID NO:7874 | SEQ ID NO:15886 | SEQ ID NO:23898 |
| iPS:437383 | 21-225_74H8 | NA | AACGCCTGGATGAAC | CGTATTAAAAGCAAAACTGATGGTGGGACAACAGACTACGCTGCACCCGTGAAAGGC | GTGGGAGCTACTACGGACTAC |
| | | | SEQ ID NO:7875 | SEQ ID NO:15887 | SEQ ID NO:23899 |
| | | AA | NAWMN | RIKSKTDGGTTDYAAPVKG | VGATTDY |
| | | | SEQ ID NO:7876 | SEQ ID NO:15888 | SEQ ID NO:23900 |
| iPS:438664 | 21-225_216G1 | NA | AACTATGGCATGCAC | GTTATATGGTATGATGGAAGTAATAAATACTATGCAGACTCCGTGAAGGGC | GGGGACTGGAACCCCGAGGGTATGGACGTC |
| | | | SEQ ID NO:7877 | SEQ ID NO:15889 | SEQ ID NO:23901 |
| | | AA | NYGMH | VIWYDGSNKYYADSVKG | GDWNPEGMDV |
| | | | SEQ ID NO:7878 | SEQ ID NO:15890 | SEQ ID NO:23902 |

FIGURE 49

| iPS:441468 | | NA | AATTATGATATTAAT | TGGATGTACCCTAACAG TGGTAGCACAGGCTATG CACAGAAATTCCAGGGC | AGCAGTGGCTGGTACTACTT TGACTAC |
|---|---|---|---|---|---|
| | 21-225_25A4.001.001 | | SEQ ID NO:7879 | SEQ ID NO:15891 | SEQ ID NO:23903 |
| | | AA | NYDIN | WMYPNSGSTGYAQKFQG | SSGWYYFDY |
| | | | SEQ ID NO:7880 | SEQ ID NO:15892 | SEQ ID NO:23904 |
| iPS:441475 | | NA | AATTATGATATTAAT | TGGATGTACCCTAACAG TGGTAACGCAGGCTATG CACAGAAATTCCAGGGC | AGCAGTGGCTGGTACTACTT TGACTAC |
| | 21-225_25A4.001.002 | | SEQ ID NO:7881 | SEQ ID NO:15893 | SEQ ID NO:23905 |
| | | AA | NYDIN | WMYPNSGNAGYAQKFQG | SSGWYYFDY |
| | | | SEQ ID NO:7882 | SEQ ID NO:15894 | SEQ ID NO:23906 |
| iPS:441482 | | NA | AATTATGATATTAAT | TGGATGTACCCTAACAG TGGTAACGTAGGCTATG CACAGAAATTCCAGGGC | AGCAGTGGCTGGTACTACTT TGACTAC |
| | 21-225_25A4.001.003 | | SEQ ID NO:7883 | SEQ ID NO:15895 | SEQ ID NO:23907 |
| | | AA | NYDIN | WMYPNSGNVGYAQKFQG | SSGWYYFDY |
| | | | SEQ ID NO:7884 | SEQ ID NO:15896 | SEQ ID NO:23908 |
| iPS:441489 | | NA | AATTATGATATTAAT | TGGATGTACCCTAACAG TGGTCAAACAGGCTATG CACAGAAATTCCAGGGC | AGCAGTGGCTGGTACTACTT TGACTAC |
| | 21-225_25A4.001.004 | | SEQ ID NO:7885 | SEQ ID NO:15897 | SEQ ID NO:23909 |

FIGURE 49

| | | AA | NYDIN | WMYPNSGQTGYAQKFQG | SSGWYYFDY |
|---|---|---|---|---|---|
| | | | SEQ ID NO:7886 | SEQ ID NO:15898 | SEQ ID NO:23910 |
| iPS:441496 | | NA | AATTATGATATTAAT | TGGATGTACCCTAACAG TGGTAGCACAGGCTATG CACAGAAATTCCAGGGC | AGCAGTGGCTGGTACTACTT TGACTAC |
| | 21-225_25A4.001.005 | | SEQ ID NO:7887 | SEQ ID NO:15899 | SEQ ID NO:23911 |
| | | AA | NYDIN | WMYPNSGSTGYAQKFQG | SSGWYYFDY |
| | | | SEQ ID NO:7888 | SEQ ID NO:15900 | SEQ ID NO:23912 |
| iPS:441505 | | NA | AATTATGATATTAAT | TGGATGTACCCTAACAG TGGTAACGCAGGCTATG CACAGAAATTCCAGGGC | AGCAGTGGCTGGTACTACTT TGACTAC |
| | 21-225_25A4.001.006 | | SEQ ID NO:7889 | SEQ ID NO:15901 | SEQ ID NO:23913 |
| | | AA | NYDIN | WMYPNSGNAGYAQKFQG | SSGWYYFDY |
| | | | SEQ ID NO:7890 | SEQ ID NO:15902 | SEQ ID NO:23914 |
| iPS:441512 | | NA | AATTATGATATTAAT | TGGATGTACCCTAACAG TGGTAACGTAGGCTATG CACAGAAATTCCAGGGC | AGCAGTGGCTGGTACTACTT TGACTAC |
| | 21-225_25A4.001.007 | | SEQ ID NO:7891 | SEQ ID NO:15903 | SEQ ID NO:23915 |
| | | AA | NYDIN | WMYPNSGNVGYAQKFQG | SSGWYYFDY |
| | | | SEQ ID NO:7892 | SEQ ID NO:15904 | SEQ ID NO:23916 |

FIGURE 49

| iPS:441519 | | NA | AATTATGATATTAAT | TGGATGTACCCTAACAG TGGTCAAACAGGCTATG CACAGAAATTCCAGGGC | AGCAGTGGCTGGTACTACTT TGACTAC |
|---|---|---|---|---|---|
| | 21-225_25A4.001.008 | | SEQ ID NO:7893 | SEQ ID NO:15905 | SEQ ID NO:23917 |
| | | AA | NYDIN | WMYPNSGQTGYAQKFQG | SSGWYYFDY |
| | | | SEQ ID NO:7894 | SEQ ID NO:15906 | SEQ ID NO:23918 |
| iPS:441554 | | NA | AATTATGATATTAAT | TGGATGTACCCTAACAG TGGTAGCACAGGCTATG CACAGAAATTCCAGGGC | AGCAGTGGCTGGTACTACTT TGACTAC |
| | 21-225_25A4.001.013 | | SEQ ID NO:7895 | SEQ ID NO:15907 | SEQ ID NO:23919 |
| | | AA | NYDIN | WMYPNSGSTGYAQKFQG | SSGWYYFDY |
| | | | SEQ ID NO:7896 | SEQ ID NO:15908 | SEQ ID NO:23920 |
| iPS:441595 | | NA | AATTATGATATTAAT | TGGATGTACCCTAACAG TGGTAGCACAGGCTATG CACAGAAATTCCAGGGC | AGCAGTGGCTGGTACTACTT TGACTAC |
| | 21-225_25A4.001.019 | | SEQ ID NO:7897 | SEQ ID NO:15909 | SEQ ID NO:23921 |
| | | AA | NYDIN | WMYPNSGSTGYAQKFQG | SSGWYYFDY |
| | | | SEQ ID NO:7898 | SEQ ID NO:15910 | SEQ ID NO:23922 |
| iPS:441604 | | NA | AATTATGATATTAAT | TGGATGTACCCTAACAG TGGTCAAACAGGCTATG CACAGAAATTCCAGGGC | AGCAGTGGCTGGTACTACTT TGACTAC |
| | 21-225_25A4.001.020 | | SEQ ID NO:7899 | SEQ ID NO:15911 | SEQ ID NO:23923 |

FIGURE 49

| | | AA | NYDIN | WMYPNSGQTGYAQKFQG | SSGWYYFDY |
|---|---|---|---|---|---|
| | | | SEQ ID NO:7900 | SEQ ID NO:15912 | SEQ ID NO:23924 |
| iPS:441613 | | NA | AATTATGATATTAAT | TGGATGTACCCTAACAG TGGTAACGCAGGCTATG CACAGAAATTCCAGGGC | AGCAGTGGCTGGTACTACTT TGACTAC |
| | 21-225_25A4.001.021 | | SEQ ID NO:7901 | SEQ ID NO:15913 | SEQ ID NO:23925 |
| | | AA | NYDIN | WMYPNSGNAGYAQKFQG | SSGWYYFDY |
| | | | SEQ ID NO:7902 | SEQ ID NO:15914 | SEQ ID NO:23926 |
| iPS:441841 | | NA | AATTATGATATCAAC | TGGATGCACCCTAACAG TGGTAACACAGGCTATG CACAGAAGTTCCAGGGC | AGCAGTGGCTGGTACTACTT TGACTAC |
| | 21-225_4A2.001.001 | | SEQ ID NO:7903 | SEQ ID NO:15915 | SEQ ID NO:23927 |
| | | AA | NYDIN | WMHPNSGNTGYAQKFQG | SSGWYYFDY |
| | | | SEQ ID NO:7904 | SEQ ID NO:15916 | SEQ ID NO:23928 |
| iPS:441847 | | NA | AATTATGATATCAAC | TGGATGCACCCTAACAG TGGTAACACAGGCTATG CACAGAAGTTCCAGGGC | AGCAGTGGCTGGTACTACTT TGACTAC |
| | 21-225_4A2.001.002 | | SEQ ID NO:7905 | SEQ ID NO:15917 | SEQ ID NO:23929 |
| | | AA | NYDIN | WMHPNSGNTGYAQKFQG | SSGWYYFDY |
| | | | SEQ ID NO:7906 | SEQ ID NO:15918 | SEQ ID NO:23930 |
| iPS:441853 | | NA | AATTATGATATCAAC | TGGATGCACCCTAACAG TGGTAACACAGGCTATG CACAGAAGTTCCAGGGC | AGCAGTGGCTGGTACTACTT TGACTAC |
| | 21-225_4A2.001.003 | | SEQ ID NO:7907 | SEQ ID NO:15919 | SEQ ID NO:23931 |

FIGURE 49

| | | AA | NYDIN | WMHPNSGNTGYAQKFQG | SSGWYYFDY |
|---|---|---|---|---|---|
| | | | SEQ ID NO:7908 | SEQ ID NO:15920 | SEQ ID NO:23932 |
| iPS:441859 | | NA | AATTATGATATCAAC | TGGATGCACCCTAACAG TGGTAACGCAGGCTATG CACAGAAGTTCCAGGGC | AGCAGTGGCTGGTACTACTT TGACTAC |
| | 21-225_4A2.001.004 | | SEQ ID NO:7909 | SEQ ID NO:15921 | SEQ ID NO:23933 |
| | | AA | NYDIN | WMHPNSGNAGYAQKFQG | SSGWYYFDY |
| | | | SEQ ID NO:7910 | SEQ ID NO:15922 | SEQ ID NO:23934 |
| iPS:441866 | | NA | AATTATGATATCAAC | TGGATGCACCCTAACAG TGGTAGCACAGGCTATG CACAGAAGTTCCAGGGC | AGCAGTGGCTGGTACTACTT TGACTAC |
| | 21-225_4A2.001.005 | | SEQ ID NO:7911 | SEQ ID NO:15923 | SEQ ID NO:23935 |
| | | AA | NYDIN | WMHPNSGSTGYAQKFQG | SSGWYYFDY |
| | | | SEQ ID NO:7912 | SEQ ID NO:15924 | SEQ ID NO:23936 |
| iPS:441873 | | NA | AATTATGATATCAAC | TGGATGCACCCTAACAG TGGTCAAACAGGCTATG CACAGAAGTTCCAGGGC | AGCAGTGGCTGGTACTACTT TGACTAC |
| | 21-225_4A2.001.006 | | SEQ ID NO:7913 | SEQ ID NO:15925 | SEQ ID NO:23937 |
| | | AA | NYDIN | WMHPNSGQTGYAQKFQG | SSGWYYFDY |
| | | | SEQ ID NO:7914 | SEQ ID NO:15926 | SEQ ID NO:23938 |
| iPS:441880 | | NA | AATTATGATATCAAC | TGGATGCACCCTAACAG TGGTCAAACAGGCTATG CACAGAAGTTCCAGGGC | AGCAGTGGCTGGTACTACTT TGACTAC |
| | 21-225_4A2.001.007 | | SEQ ID NO:7915 | SEQ ID NO:15927 | SEQ ID NO:23939 |
| | | AA | NYDIN | WMHPNSGQTGYAQKFQG | SSGWYYFDY |

FIGURE 49

| | | | SEQ ID NO:7916 | SEQ ID NO:15928 | SEQ ID NO:23940 |
|---|---|---|---|---|---|
| iPS:441884 | | NA | AATTATGATATCAAC | TGGATGCACCCTAACAG TGGTAACGCAGGCTATG CACAGAAGTTCCAGGGC | AGCAGTGGCTGGTACTACTT TGACTAC |
| | 21-225_4A2.001.008 | | SEQ ID NO:7917 | SEQ ID NO:15929 | SEQ ID NO:23941 |
| | | AA | NYDIN | WMHPNSGNAGYAQKFQG | SSGWYYFDY |
| | | | SEQ ID NO:7918 | SEQ ID NO:15930 | SEQ ID NO:23942 |
| iPS:441888 | | NA | AATTATGATATCAAC | TGGATGCACCCTAACAG TGGTCAAACAGGCTATG CACAGAAGTTCCAGGGC | AGCAGTGGCTGGTACTACTT TGACTAC |
| | 21-225_4A2.001.009 | | SEQ ID NO:7919 | SEQ ID NO:15931 | SEQ ID NO:23943 |
| | | AA | NYDIN | WMHPNSGQTGYAQKFQG | SSGWYYFDY |
| | | | SEQ ID NO:7920 | SEQ ID NO:15932 | SEQ ID NO:23944 |
| iPS:441892 | | NA | AATTATGATATCAAC | TGGATGCACCCTAACAG TGGTAACGCAGGCTATG CACAGAAGTTCCAGGGC | AGCAGTGGCTGGTACTACTT TGACTAC |
| | 21-225_4A2.001.010 | | SEQ ID NO:7921 | SEQ ID NO:15933 | SEQ ID NO:23945 |
| | | AA | NYDIN | WMHPNSGNAGYAQKFQG | SSGWYYFDY |
| | | | SEQ ID NO:7922 | SEQ ID NO:15934 | SEQ ID NO:23946 |
| iPS:441896 | | NA | AATTATGATATCAAC | TGGATGCACCCTAACAG TGGTCAAACAGGCTATG CACAGAAGTTCCAGGGC | AGCAGTGGCTGGTACTACTT TGACTAC |
| | 21-225_4A2.001.011 | | SEQ ID NO:7923 | SEQ ID NO:15935 | SEQ ID NO:23947 |
| | | AA | NYDIN | WMHPNSGQTGYAQKFQG | SSGWYYFDY |
| | | | SEQ ID NO:7924 | SEQ ID NO:15936 | SEQ ID NO:23948 |

FIGURE 49

| iPS:441900 | | NA | AATTATGATATCAAC | TGGATGCACCCTAACAG TGGTAGCACAGGCTATG CACAGAAGTTCCAGGGC | AGCAGTGGCTGGTACTACTT TGACTAC |
|---|---|---|---|---|---|
| | 21-225_4A2.001.012 | | SEQ ID NO:7925 | SEQ ID NO:15937 | SEQ ID NO:23949 |
| | | AA | NYDIN | WMHPNSGSTGYAQKFQG | SSGWYYFDY |
| | | | SEQ ID NO:7926 | SEQ ID NO:15938 | SEQ ID NO:23950 |
| iPS:441955 | | NA | AATTATGATATCAAC | TGGATGCACCCTAACAG TGGTAACACAGGCTATG CACAGAAGTTCCAGGGC | AGCAGTGGCTGGTACTACTT TGACTAC |
| | 21-225_4A2.001.022 | | SEQ ID NO:7927 | SEQ ID NO:15939 | SEQ ID NO:23951 |
| | | AA | NYDIN | WMHPNSGNTGYAQKFQG | SSGWYYFDY |
| | | | SEQ ID NO:7928 | SEQ ID NO:15940 | SEQ ID NO:23952 |
| iPS:441962 | | NA | AATTATGATATCAAC | TGGATGCACCCTAACAG TGGTAACACAGGCTATG CACAGAAGTTCCAGGGC | AGCAGTGGCTGGTACTACTT TGACTAC |
| | 21-225_4A2.001.023 | | SEQ ID NO:7929 | SEQ ID NO:15941 | SEQ ID NO:23953 |
| | | AA | NYDIN | WMHPNSGNTGYAQKFQG | SSGWYYFDY |
| | | | SEQ ID NO:7930 | SEQ ID NO:15942 | SEQ ID NO:23954 |
| iPS:441971 | | NA | AATTATGATATCAAC | TGGATGCACCCTAACAG TGGTCAAACAGGCTATG CACAGAAGTTCCAGGGC | AGCAGTGGCTGGTACTACTT TGACTAC |
| | 21-225_4A2.001.024 | | SEQ ID NO:7931 | SEQ ID NO:15943 | SEQ ID NO:23955 |
| | | AA | NYDIN | WMHPNSGQTGYAQKFQG | SSGWYYFDY |
| | | | SEQ ID NO:7932 | SEQ ID NO:15944 | SEQ ID NO:23956 |

FIGURE 49

| iPS:441999 | | NA | AATTATGATATCAAC | TGGATGCACCCTAACAG TGGTCAAACAGGCTATG CACAGAAGTTCCAGGGC | AGCAGTGGCTGGTACTACTT TGACTAC |
|---|---|---|---|---|---|
| | 21-225_4A2.001.028 | | SEQ ID NO:7933 | SEQ ID NO:15945 | SEQ ID NO:23957 |
| | | AA | NYDIN | WMHPNSGQTGYAQKFQG | SSGWYYFDY |
| | | | SEQ ID NO:7934 | SEQ ID NO:15946 | SEQ ID NO:23958 |
| iPS:442006 | | NA | AATTATGATATCAAC | TGGATGCACCCTAACAG TGGTAGCACAGGCTATG CACAGAAGTTCCAGGGC | AGCAGTGGCTGGTACTACTT TGACTAC |
| | 21-225_4A2.001.029 | | SEQ ID NO:7935 | SEQ ID NO:15947 | SEQ ID NO:23959 |
| | | AA | NYDIN | WMHPNSGSTGYAQKFQG | SSGWYYFDY |
| | | | SEQ ID NO:7936 | SEQ ID NO:15948 | SEQ ID NO:23960 |
| iPS:442020 | | NA | AATTATGATATCAAC | TGGATGCACCCTAACAG TGGTAACGAAGGCTATG CACAGAAGTTCCAGGGC | AGCAGTGGCTGGTACTACTT TGACTAC |
| | 21-225_4A2.001.031 | | SEQ ID NO:7937 | SEQ ID NO:15949 | SEQ ID NO:23961 |
| | | AA | NYDIN | WMHPNSGNEGYAQKFQG | SSGWYYFDY |
| | | | SEQ ID NO:7938 | SEQ ID NO:15950 | SEQ ID NO:23962 |
| iPS:442050 | | NA | GACTACTATTTGCAC | TGGATCCACCCTAACAG TGGTGGCACAAACTATG CACAGAAGTTTCAGGGC | GATGGTACCAGCTCGTTTGA CTAC |
| | 21-225_4H6.004 | | SEQ ID NO:7939 | SEQ ID NO:15951 | SEQ ID NO:23963 |
| | | AA | DYYLH | WIHPNSGGTNYAQKFQG | DGTSSFDY |
| | | | SEQ ID NO:7940 | SEQ ID NO:15952 | SEQ ID NO:23964 |
| iPS:442059 | 21 225 4H6 005 | NA | GACTACTATTTGCAC | TGGATCCACCCTAACAG TGGTGGCACAAACTATG CACAGAAGTTTCAGGGC | GATGGTACCAGCTCGTTTGA CTAC |

FIGURE 49

| | 21-225_4H6.005 | | SEQ ID NO:7941 | SEQ ID NO:15953 | SEQ ID NO:23965 |
|---|---|---|---|---|---|
| | | AA | DYYLH | WIHPNSGGTNYAQKFQG | DGTSSFDY |
| | | | SEQ ID NO:7942 | SEQ ID NO:15954 | SEQ ID NO:23966 |
| iPS:442065 | 21-225_4H6.006 | NA | GACTACTATTTGCAC | TGGATCCACCCTAACAGTGGTGGCACAAACTATGCACAGAAGTTTCAGGGC | GATGGTACCAGCTCGTTTGACTAC |
| | | | SEQ ID NO:7943 | SEQ ID NO:15955 | SEQ ID NO:23967 |
| | | AA | DYYLH | WIHPNSGGTNYAQKFQG | DGTSSFDY |
| | | | SEQ ID NO:7944 | SEQ ID NO:15956 | SEQ ID NO:23968 |
| iPS:442071 | 21-225_4H6.007 | NA | GACTACTATTTGCAC | TGGATCCACCCTAACAGTGGTGGCACAAACTATGCACAGAAGTTTCAGGGC | GATGCTACCAGCTCGTTTGACTAC |
| | | | SEQ ID NO:7945 | SEQ ID NO:15957 | SEQ ID NO:23969 |
| | | AA | DYYLH | WIHPNSGGTNYAQKFQG | DATSSFDY |
| | | | SEQ ID NO:7946 | SEQ ID NO:15958 | SEQ ID NO:23970 |
| iPS:442078 | 21-225_4H6.008 | NA | GACTACTATTTGCAC | TGGATCCACCCTAACAGTGGTGGCACAAACTATGCACAGAAGTTTCAGGGC | AGTGGTACCAGCTCGTTTGACTAC |
| | | | SEQ ID NO:7947 | SEQ ID NO:15959 | SEQ ID NO:23971 |
| | | AA | DYYLH | WIHPNSGGTNYAQKFQG | SGTSSFDY |
| | | | SEQ ID NO:7948 | SEQ ID NO:15960 | SEQ ID NO:23972 |
| iPS:442085 | 21-225_4H6.009 | NA | GACTACTATTTGCAC | TGGATCCACCCTAACAGTGGTGGCACAAACTATGCACAGAAGTTTCAGGGC | AGTGGTACCAGCTCGTTTGACTAC |
| | | | SEQ ID NO:7949 | SEQ ID NO:15961 | SEQ ID NO:23973 |
| | | AA | DYYLH | WIHPNSGGTNYAQKFQG | SGTSSFDY |
| | | | SEQ ID NO:7950 | SEQ ID NO:15962 | SEQ ID NO:23974 |
| iPS:442089 | 21 225 4H6 010 | NA | GACTACTATTTGCAC | TGGATCCACCCTAACAGTGGTGGCACAAACTATGCACAGAAGTTTCAGGGC | GATGCTACCAGCTCGTTTGACTAC |

FIGURE 49

| | 21-225_4H6.010 | | SEQ ID NO:7951 | SEQ ID NO:15963 | SEQ ID NO:23975 |
|---|---|---|---|---|---|
| | | AA | DYYLH | WIHPNSGGTNYAQKFQG | DATSSFDY |
| | | | SEQ ID NO:7952 | SEQ ID NO:15964 | SEQ ID NO:23976 |
| iPS:442093 | 21-225_4H6.011 | NA | GACTACTATTTGCAC | TGGATCCACCCTAACAG TGGTGGCACAAACTATG CACAGAAGTTTCAGGGC | GATGCTACCAGCTCGTTTGA CTAC |
| | | | SEQ ID NO:7953 | SEQ ID NO:15965 | SEQ ID NO:23977 |
| | | AA | DYYLH | WIHPNSGGTNYAQKFQG | DATSSFDY |
| | | | SEQ ID NO:7954 | SEQ ID NO:15966 | SEQ ID NO:23978 |
| iPS:442115 | 21-225_5E5.003 | NA | AACTATGTCATGCAC | GTTATCTGGTATGATGCA AGTAATAAATACTATGC AGACTCCGTGAAGGGC | GAGGTATATAGCAGTGGCTG GTACGACTACGGTATGGACG TC |
| | | | SEQ ID NO:7955 | SEQ ID NO:15967 | SEQ ID NO:23979 |
| | | AA | NYVMH | VIWYDASNKYYADSVKG | EVYSSGWYDYGMDV |
| | | | SEQ ID NO:7956 | SEQ ID NO:15968 | SEQ ID NO:23980 |
| iPS:442122 | 21-225_5E5.004 | NA | AACTATGTCATGCAC | GTTATCTGGTATGATGGA AGTAATAAATACTATGC AGAATCCGTGAAGGGC | GAGGTATATAGCAGTGGCTG GTACGACTACGGTATGGACG TC |
| | | | SEQ ID NO:7957 | SEQ ID NO:15969 | SEQ ID NO:23981 |
| | | AA | NYVMH | VIWYDGSNKYYAESVKG | EVYSSGWYDYGMDV |
| | | | SEQ ID NO:7958 | SEQ ID NO:15970 | SEQ ID NO:23982 |
| iPS:442129 | 21-225_5E5.005 | NA | AACTATGTCATGCAC | GTTATCTGGTATGATGGA AGTAATAAATACTATGC AGGCTCCGTGAAGGGC | GAGGTATATAGCAGTGGCTG GTACGACTACGGTATGGACG TC |
| | | | SEQ ID NO:7959 | SEQ ID NO:15971 | SEQ ID NO:23983 |

FIGURE 49

| | | AA | NYVMH | VIWYDGSNKYYAGSVKG | EVYSSGWYDYGMDV |
|---|---|---|---|---|---|
| | | | SEQ ID NO:7960 | SEQ ID NO:15972 | SEQ ID NO:23984 |
| iPS:442136 | 21-225_5E5.006 | NA | AACTATGTCATGCAC | GTTATCTGGTATGATGGAAGTAATAAATACTATGCAGACGCCGTGAAGGGC | GAGGTATATAGCAGTGGCTGGTACGACTACGGTATGGACGTC |
| | | | SEQ ID NO:7961 | SEQ ID NO:15973 | SEQ ID NO:23985 |
| | | AA | NYVMH | VIWYDGSNKYYADAVKG | EVYSSGWYDYGMDV |
| | | | SEQ ID NO:7962 | SEQ ID NO:15974 | SEQ ID NO:23986 |
| iPS:442171 | 21-225_5E5.011 | NA | AACTATGTCATGCAC | GTTATCTGGTATGATGCAAGTAATAAATACTATGCAGAATCCGTGAAGGGC | GAGGTATATAGCAGTGGCTGGTACGACTACGGTATGGACGTC |
| | | | SEQ ID NO:7963 | SEQ ID NO:15975 | SEQ ID NO:23987 |
| | | AA | NYVMH | VIWYDASNKYYAESVKG | EVYSSGWYDYGMDV |
| | | | SEQ ID NO:7964 | SEQ ID NO:15976 | SEQ ID NO:23988 |
| iPS:442178 | 21-225_5E5.012 | NA | AACTATGTCATGCAC | GTTATCTGGTATGATGCAAGTAATAAATACTATGCAGACGCCGTGAAGGGC | GAGGTATATAGCAGTGGCTGGTACGACTACGGTATGGACGTC |
| | | | SEQ ID NO:7965 | SEQ ID NO:15977 | SEQ ID NO:23989 |
| | | AA | NYVMH | VIWYDASNKYYADAVKG | EVYSSGWYDYGMDV |
| | | | SEQ ID NO:7966 | SEQ ID NO:15978 | SEQ ID NO:23990 |

FIGURE 49

| iPS:442199 | | NA | AACTATGTCATGCAC | GTTATCTGGTATGATGCA<br>AGTAATAAATACTATGC<br>AGACTCCGTGAAGGGC | GAGGTATATAGCAGTGGCTA<br>TTACGACTACGGTATGGACG<br>TC |
|---|---|---|---|---|---|
| | 21-225_5E5.015 | | SEQ ID NO:7967 | SEQ ID NO:15979 | SEQ ID NO:23991 |
| | | AA | NYVMH | VIWYDASNKYYADSVKG | EVYSSGYYDYGMDV |
| | | | SEQ ID NO:7968 | SEQ ID NO:15980 | SEQ ID NO:23992 |
| iPS:442206 | | NA | AACTATGTCATGCAC | GTTATCTGGTATGATGCA<br>AGTAATAAATACTATGC<br>AGACTCCGTGAAGGGC | GAGGTATATAGCAGTGGCTT<br>CTACGACTACGGTATGGACG<br>TC |
| | 21-225_5E5.016 | | SEQ ID NO:7969 | SEQ ID NO:15981 | SEQ ID NO:23993 |
| | | AA | NYVMH | VIWYDASNKYYADSVKG | EVYSSGFYDYGMDV |
| | | | SEQ ID NO:7970 | SEQ ID NO:15982 | SEQ ID NO:23994 |
| iPS:442213 | | NA | AACTATGTCATGCAC | GTTATCTGGTATGATGGA<br>AGTAATAAATACTATGC<br>AGAATCCGTGAAGGGC | GAGGTATATAGCAGTGGCTA<br>TTACGACTACGGTATGGACG<br>TC |
| | 21-225_5E5.017 | | SEQ ID NO:7971 | SEQ ID NO:15983 | SEQ ID NO:23995 |
| | | AA | NYVMH | VIWYDGSNKYYAESVKG | EVYSSGYYDYGMDV |
| | | | SEQ ID NO:7972 | SEQ ID NO:15984 | SEQ ID NO:23996 |
| iPS:442220 | | NA | AACTATGTCATGCAC | GTTATCTGGTATGATGGA<br>AGTAATAAATACTATGC<br>AGACTCCGTGAAGGGC | GAGGTATATAGCAGTGGCTA<br>TTACGACTACGGTATGGACG<br>TC |
| | 21-225_5E5.018 | | SEQ ID NO:7973 | SEQ ID NO:15985 | SEQ ID NO:23997 |

FIGURE 49

| | | AA | NYVMH | VIWYDGSNKYYADSVKG | EVYSSGYYDYGMDV |
|---|---|---|---|---|---|
| | | | SEQ ID NO:7974 | SEQ ID NO:15986 | SEQ ID NO:23998 |
| iPS:442227 | 21-225_5E5.019 | NA | AACTATGTCATGCAC | GTTATCTGGTATGATGGA AGTAATAAATACTATGC AGACTCCGTGAAGGGC | GAGGTATATAGCAGTGGCTT CTACGACTACGGTATGGACG TC |
| | | | SEQ ID NO:7975 | SEQ ID NO:15987 | SEQ ID NO:23999 |
| | | AA | NYVMH | VIWYDGSNKYYADSVKG | EVYSSGFYDYGMDV |
| | | | SEQ ID NO:7976 | SEQ ID NO:15988 | SEQ ID NO:24000 |
| iPS:442255 | 21-225_5E5.023 | NA | AACTATGTCATGCAC | GTTATCTGGTATGATGCA AGTAATAAATACTATGC AGAATCCGTGAAGGGC | GAGGTATATAGCAGTGGCTA TTACGACTACGGTATGGACG TC |
| | | | SEQ ID NO:7977 | SEQ ID NO:15989 | SEQ ID NO:24001 |
| | | AA | NYVMH | VIWYDASNKYYAESVKG | EVYSSGYYDYGMDV |
| | | | SEQ ID NO:7978 | SEQ ID NO:15990 | SEQ ID NO:24002 |
| iPS:442262 | 21-225_5E5.024 | NA | AACTATGTCATGCAC | GTTATCTGGTATGATGCA AGTAATAAATACTATGC AGACGCCGTGAAGGGC | GAGGTATATAGCAGTGGCTA TTACGACTACGGTATGGACG TC |
| | | | SEQ ID NO:7979 | SEQ ID NO:15991 | SEQ ID NO:24003 |
| | | AA | NYVMH | VIWYDASNKYYADAVKG | EVYSSGYYDYGMDV |
| | | | SEQ ID NO:7980 | SEQ ID NO:15992 | SEQ ID NO:24004 |

FIGURE 49

| iPS:442269 | | NA | AACTATGTCATGCAC | GTTATCTGGTATGATGGA AGTAATAAATACTATGC AGAATCCGTGAAGGGC | GAGGTATATAGCAGTGGCTG GTACGACTACGGTATGGACG TC |
|---|---|---|---|---|---|
| | 21-225_5E5.025 | | SEQ ID NO:7981 | SEQ ID NO:15993 | SEQ ID NO:24005 |
| | | AA | NYVMH | VIWYDGSNKYYAESVKG | EVYSSGWYDYGMDV |
| | | | SEQ ID NO:7982 | SEQ ID NO:15994 | SEQ ID NO:24006 |
| iPS:442311 | | NA | AGCTTTGGCATGCAC | ATTATCTGGCATGATGG AAGTAATAAATACTATG CAGACTCCGTGAAGGGC | GATCTGAGTATGGGCGGTAT GGACGTC |
| | 21-225_7E11.001.001 | | SEQ ID NO:7983 | SEQ ID NO:15995 | SEQ ID NO:24007 |
| | | AA | SFGMH | IIWHDGSNKYYADSVKG | DLSMGGMDV |
| | | | SEQ ID NO:7984 | SEQ ID NO:15996 | SEQ ID NO:24008 |
| iPS:442317 | | NA | AGCTTTGGCATGCAC | ATTATCTGGCATGATGG AAGTAATAAATACTATG CAGACTCCGTGAAGGGC | GATCTGAGTATGGGCGGTAT GGACGTC |
| | 21-225_7E11.001.002 | | SEQ ID NO:7985 | SEQ ID NO:15997 | SEQ ID NO:24009 |
| | | AA | SFGMH | IIWHDGSNKYYADSVKG | DLSMGGMDV |
| | | | SEQ ID NO:7986 | SEQ ID NO:15998 | SEQ ID NO:24010 |
| iPS:442323 | | NA | AGCTTTGGCATGCAC | ATTATCTGGCATAGTGG AAGTAATAAATACTATG CAGACTCCGTGAAGGGC | GATCTGAGTATGGGCGGTAT GGACGTC |
| | 21-225_7E11.001.003 | | SEQ ID NO:7987 | SEQ ID NO:15999 | SEQ ID NO:24011 |
| | | AA | SFGMH | IIWHSGSNKYYADSVKG | DLSMGGMDV |
| | | | SEQ ID NO:7988 | SEQ ID NO:16000 | SEQ ID NO:24012 |

FIGURE 49

| iPS:442330 | | NA | AGCTTTGGCATGCAC | ATTATCTGGCATGAAGG AAGTAATAAATACTATG CAGACTCCGTGAAGGGC | GATCTGAGTATGGGCGGTAT GGACGTC |
|---|---|---|---|---|---|
| | 21-225_7E11.001.004 | | SEQ ID NO:7989 | SEQ ID NO:16001 | SEQ ID NO:24013 |
| | | AA | SFGMH | IIWHEGSNKYYADSVKG | DLSMGGMDV |
| | | | SEQ ID NO:7990 | SEQ ID NO:16002 | SEQ ID NO:24014 |
| iPS:442337 | | NA | AGCTTTGGCATGCAC | ATTATCTGGCATGATGCA AGTAATAAATACTATGC AGACTCCGTGAAGGGC | GATCTGAGTATGGGCGGTAT GGACGTC |
| | 21-225_7E11.001.005 | | SEQ ID NO:7991 | SEQ ID NO:16003 | SEQ ID NO:24015 |
| | | AA | SFGMH | IIWHDASNKYYADSVKG | DLSMGGMDV |
| | | | SEQ ID NO:7992 | SEQ ID NO:16004 | SEQ ID NO:24016 |
| iPS:442344 | | NA | AGCTTTGGCATGCAC | ATTATCTGGCATGATGG AAGTAATAAATACTATG CAGAATCCGTGAAGGGC | GATCTGAGTATGGGCGGTAT GGACGTC |
| | 21-225_7E11.001.006 | | SEQ ID NO:7993 | SEQ ID NO:16005 | SEQ ID NO:24017 |
| | | AA | SFGMH | IIWHDGSNKYYAESVKG | DLSMGGMDV |
| | | | SEQ ID NO:7994 | SEQ ID NO:16006 | SEQ ID NO:24018 |
| iPS:442351 | | NA | AGCTTTGGCATGCAC | ATTATCTGGCATGATGG AAGTAATAAATACTATG CAGACGCCGTGAAGGGC | GATCTGAGTATGGGCGGTAT GGACGTC |
| | 21-225_7E11.001.007 | | SEQ ID NO:7995 | SEQ ID NO:16007 | SEQ ID NO:24019 |
| | | AA | SFGMH | IIWHDGSNKYYADAVKG | DLSMGGMDV |
| | | | SEQ ID NO:7996 | SEQ ID NO:16008 | SEQ ID NO:24020 |

FIGURE 49

| iPS:442358 | | NA | AGCTTTGGCATGCAC | ATTATCTGGCATGAAGG AAGTAATAAATACTATG CAGACGCCGTGAAGGGC | GATCTGAGTATGGGCGGTAT GGACGTC |
|---|---|---|---|---|---|
| | 21-225_7E11.001.008 | | SEQ ID NO:7997 | SEQ ID NO:16009 | SEQ ID NO:24021 |
| | | AA | SFGMH | IIWHEGSNKYYADAVKG | DLSMGGMDV |
| | | | SEQ ID NO:7998 | SEQ ID NO:16010 | SEQ ID NO:24022 |
| iPS:442365 | | NA | AGCTTTGGCATGCAC | ATTATCTGGCATGAAGG AAGTAATAAATACTATG CAGAATCCGTGAAGGGC | GATCTGAGTATGGGCGGTAT GGACGTC |
| | 21-225_7E11.001.009 | | SEQ ID NO:7999 | SEQ ID NO:16011 | SEQ ID NO:24023 |
| | | AA | SFGMH | IIWHEGSNKYYAESVKG | DLSMGGMDV |
| | | | SEQ ID NO:8000 | SEQ ID NO:16012 | SEQ ID NO:24024 |
| iPS:442372 | | NA | AGCTTTGGCATGCAC | ATTATCTGGCATAGTGG AAGTAATAAATACTATG CAGAATCCGTGAAGGGC | GATCTGAGTATGGGCGGTAT GGACGTC |
| | 21-225_7E11.001.010 | | SEQ ID NO:8001 | SEQ ID NO:16013 | SEQ ID NO:24025 |
| | | AA | SFGMH | IIWHSGSNKYYAESVKG | DLSMGGMDV |
| | | | SEQ ID NO:8002 | SEQ ID NO:16014 | SEQ ID NO:24026 |
| iPS:442379 | | NA | AGCTTTGGCATGCAC | ATTATCTGGCATAGTGG AAGTAATAAATACTATG CAGACGCCGTGAAGGGC | GATCTGAGTATGGGCGGTAT GGACGTC |
| | 21-225_7E11.001.011 | | SEQ ID NO:8003 | SEQ ID NO:16015 | SEQ ID NO:24027 |
| | | AA | SFGMH | IIWHSGSNKYYADAVKG | DLSMGGMDV |
| | | | SEQ ID NO:8004 | SEQ ID NO:16016 | SEQ ID NO:24028 |

FIGURE 49

| iPS:442386 | | NA | AGCTTTGGCATGCAC | ATTATCTGGCATAGTGG AAGTAATAAATACTATG CAGACTCCGTGAAGGGC | GATCTGAGTATGGGCGGTAT GGACGTC |
|---|---|---|---|---|---|
| | 21-225_7E11.001.012 | | SEQ ID NO:8005 | SEQ ID NO:16017 | SEQ ID NO:24029 |
| | | AA | SFGMH | IIWHSGSNKYYADSVKG | DLSMGGMDV |
| | | | SEQ ID NO:8006 | SEQ ID NO:16018 | SEQ ID NO:24030 |
| iPS:442390 | | NA | AGCTTTGGCATGCAC | ATTATCTGGCATGATGG AAGTAATAAATACTATG CAGAATCCGTGAAGGGC | GATCTGAGTATGGGCGGTAT GGACGTC |
| | 21-225_7E11.001.013 | | SEQ ID NO:8007 | SEQ ID NO:16019 | SEQ ID NO:24031 |
| | | AA | SFGMH | IIWHDGSNKYYAESVKG | DLSMGGMDV |
| | | | SEQ ID NO:8008 | SEQ ID NO:16020 | SEQ ID NO:24032 |
| iPS:442394 | | NA | AGCTTTGGCATGCAC | ATTATCTGGCATAGTGG AAGTAATAAATACTATG CAGAATCCGTGAAGGGC | GATCTGAGTATGGGCGGTAT GGACGTC |
| | 21-225_7E11.001.014 | | SEQ ID NO:8009 | SEQ ID NO:16021 | SEQ ID NO:24033 |
| | | AA | SFGMH | IIWHSGSNKYYAESVKG | DLSMGGMDV |
| | | | SEQ ID NO:8010 | SEQ ID NO:16022 | SEQ ID NO:24034 |
| iPS:442398 | | NA | AGCTTTGGCATGCAC | ATTATCTGGCATAGTGG AAGTAATAAATACTATG CAGACTCCGTGAAGGGC | GATCTGAGTATGGGCGGTAT GGACGTC |
| | 21-225_7E11.001.015 | | SEQ ID NO:8011 | SEQ ID NO:16023 | SEQ ID NO:24035 |
| | | AA | SFGMH | IIWHSGSNKYYADSVKG | DLSMGGMDV |
| | | | SEQ ID NO:8012 | SEQ ID NO:16024 | SEQ ID NO:24036 |

FIGURE 49

| iPS:442402 | | NA | AGCTTTGGCATGCAC | ATTATCTGGCATAGTGG AAGTAATAAATACTATG CAGAATCCGTGAAGGGC | GATCTGAGTATGGGCGGTAT GGACGTC |
|---|---|---|---|---|---|
| | 21-225_7E11.001.016 | | SEQ ID NO:8013 | SEQ ID NO:16025 | SEQ ID NO:24037 |
| | | AA | SFGMH | IIWHSGSNKYYAESVKG | DLSMGGMDV |
| | | | SEQ ID NO:8014 | SEQ ID NO:16026 | SEQ ID NO:24038 |
| iPS:442406 | | NA | AGCTTTGGCATGCAC | ATTATCTGGCATGAAGG AAGTAATAAATACTATG CAGACGCCGTGAAGGGC | GATCTGAGTATGGGCGGTAT GGACGTC |
| | 21-225_7E11.001.017 | | SEQ ID NO:8015 | SEQ ID NO:16027 | SEQ ID NO:24039 |
| | | AA | SFGMH | IIWHEGSNKYYADAVKG | DLSMGGMDV |
| | | | SEQ ID NO:8016 | SEQ ID NO:16028 | SEQ ID NO:24040 |
| iPS:442410 | | NA | AGCTTTGGCATGCAC | ATTATCTGGCATGATGCA AGTAATAAATACTATGC AGAATCCGTGAAGGGC | GATCTGAGTATGGGCGGTAT GGACGTC |
| | 21-225_7E11.001.018 | | SEQ ID NO:8017 | SEQ ID NO:16029 | SEQ ID NO:24041 |
| | | AA | SFGMH | IIWHDASNKYYAESVKG | DLSMGGMDV |
| | | | SEQ ID NO:8018 | SEQ ID NO:16030 | SEQ ID NO:24042 |
| iPS:442417 | | NA | AGCTTTGGCATGCAC | ATTATCTGGCATGATGG AAGTAATAAATACTATG CAGAATCCGTGAAGGGC | GATCTGAGTATGGGCGGTAT GGACGTC |
| | 21-225_7E11.001.019 | | SEQ ID NO:8019 | SEQ ID NO:16031 | SEQ ID NO:24043 |
| | | AA | SFGMH | IIWHDGSNKYYAESVKG | DLSMGGMDV |
| | | | SEQ ID NO:8020 | SEQ ID NO:16032 | SEQ ID NO:24044 |

FIGURE 49

| iPS:442431 | | NA | AGCTTTGGCATGCAC | ATTATCTGGCATAGTGG AAGTAATAAATACTATG CAGAATCCGTGAAGGGC | GATCTGAGTATGGGCGGTAT GGACGTC |
|---|---|---|---|---|---|
| | 21-225_7E11.001.021 | | SEQ ID NO:8021 | SEQ ID NO:16033 | SEQ ID NO:24045 |
| | | AA | SFGMH | IIWHSGSNKYYAESVKG | DLSMGGMDV |
| | | | SEQ ID NO:8022 | SEQ ID NO:16034 | SEQ ID NO:24046 |
| iPS:442438 | | NA | AGCTTTGGCATGCAC | ATTATCTGGCATGAAGG AAGTAATAAATACTATG CAGAATCCGTGAAGGGC | GATCTGAGTATGGGCGGTAT GGACGTC |
| | 21-225_7E11.001.022 | | SEQ ID NO:8023 | SEQ ID NO:16035 | SEQ ID NO:24047 |
| | | AA | SFGMH | IIWHEGSNKYYAESVKG | DLSMGGMDV |
| | | | SEQ ID NO:8024 | SEQ ID NO:16036 | SEQ ID NO:24048 |
| iPS:442568 | | NA | AACAGTGGTTACTACTGGAG C | TACAGCTATTACAGTGG GAGCACCTACTACAACC CGTCCCTCAAGAGT | GGGGGATATAACTGGAACCA TGCTTTTGATATC |
| | 21-225_149D8 | | SEQ ID NO:8025 | SEQ ID NO:16037 | SEQ ID NO:24049 |
| | | AA | NSGYYWS | YSYYSGSTYYNPSLKS | GGYNWNHAFDI |
| | | | SEQ ID NO:8026 | SEQ ID NO:16038 | SEQ ID NO:24050 |
| iPS:443003 | | NA | GGCTACTATATACAC | TGGATCAACCCTAACAG TGGTGGCACAAACTATG CACAGAAGTTTCAGGGC | GGAGGGAATTACTTCTACAA CCACGTTATGGACGTC |
| | 21-225_43F11_LC2 | | SEQ ID NO:8027 | SEQ ID NO:16039 | SEQ ID NO:24051 |
| | | AA | GYYIH | WINPNSGGTNYAQKFQG | GGNYFYNHVMDV |
| | | | SEQ ID NO:8028 | SEQ ID NO:16040 | SEQ ID NO:24052 |
| iPS:443005 | | NA | GGCTACTATATACAC | TGGATCAACCCTAACAG TGGTGGCACAAACTATG CACAGAAGTTTCAGGGC | GGAGGGAATTACTTCTACAA CCACGTTATGGACGTC |
| | 21-225_43F11_LC1 | | SEQ ID NO:8029 | SEQ ID NO:16041 | SEQ ID NO:24053 |

FIGURE 49

| | | | | | |
|---|---|---|---|---|---|
| | | AA | GYYIH | WINPNSGGTNYAQKFQG | GGNYFYNHVMDV |
| | | | SEQ ID NO:8030 | SEQ ID NO:16042 | SEQ ID NO:24054 |
| iPS:443006 | 21-225_25A4.001.029 | NA | AATTATGATATTAAT | TGGATGTACCCTAACAGTGGTCAAACAGGCTATGCACAGAAATTCCAGGGC | AGCAGTGGCTGGTACTACTTTGACTAC |
| | | | SEQ ID NO:8031 | SEQ ID NO:16043 | SEQ ID NO:24055 |
| | | AA | NYDIN | WMYPNSGQTGYAQKFQG | SSGWYYFDY |
| | | | SEQ ID NO:8032 | SEQ ID NO:16044 | SEQ ID NO:24056 |
| iPS:443016 | 21-225_4H6.014 | NA | GACTACTATTTGCAC | TGGATCCACCCTAACAGTGGTGGCACAAACTATGCACAGAAGTTTCAGGGC | GATGCTACCAGCTCGTTTGACTAC |
| | | | SEQ ID NO:8033 | SEQ ID NO:16045 | SEQ ID NO:24057 |
| | | AA | DYYLH | WIHPNSGGTNYAQKFQG | DATSSFDY |
| | | | SEQ ID NO:8034 | SEQ ID NO:16046 | SEQ ID NO:24058 |
| iPS:443027 | 21-225_7E11.001.023 | NA | AGCTTTGGCATGCAC | ATTATCTGGCATGATGCAAGTAATAAATACTATGCAGACGCCGTGAAGGGC | GATCTGAGTATGGGCGGTATGGACGTC |
| | | | SEQ ID NO:8035 | SEQ ID NO:16047 | SEQ ID NO:24059 |
| | | AA | SFGMH | IIWHDASNKYYADAVKG | DLSMGGMDV |
| | | | SEQ ID NO:8036 | SEQ ID NO:16048 | SEQ ID NO:24060 |
| iPS:446086 | 21-225_94D8 | NA | AATTATGATATCAAC | TGGATGAACCCTAACAGTGGTAACACAGGCTATGCACAGAAGTTCCAGGTC | AGCAGTGGCTGGTACATCTTTGACTAC |
| | | | SEQ ID NO:8037 | SEQ ID NO:16049 | SEQ ID NO:24061 |
| | | AA | NYDIN | WMNPNSGNTGYAQKFQV | SSGWYIFDY |

FIGURE 49

| | | | SEQ ID NO:8038 | SEQ ID NO:16050 | SEQ ID NO:24062 |
|---|---|---|---|---|---|
| iPS:446094 | | NA | AATTATGATATCAAC | TGGATGAACCCTAACAG TGGTAACACAGGCTATG CACAGAAGTTCCAGGGC | TCCAGTGGCTGGCACTGGTT CGACCCC |
| | 21-225_77E1 | | SEQ ID NO:8039 | SEQ ID NO:16051 | SEQ ID NO:24063 |
| | | AA | NYDIN | WMNPNSGNTGYAQKFQG | SSGWHWFDP |
| | | | SEQ ID NO:8040 | SEQ ID NO:16052 | SEQ ID NO:24064 |
| iPS:448904 | | NA | AGCTTTAGCTTGAAC | TCCATTAGTAGTAGTAGT AGTTACATATACTACGC AGACTCAGTGAAGGGC | GATGCGTATAGCCACTAC |
| | 21-225_65C12 | | SEQ ID NO:8041 | SEQ ID NO:16053 | SEQ ID NO:24065 |
| | | AA | SFSLN | SISSSSSYIYYADSVKG | DAYSHY |
| | | | SEQ ID NO:8042 | SEQ ID NO:16054 | SEQ ID NO:24066 |
| iPS:448906 | | NA | AGTTATAGCATGAAC | TCCATTAGTGGTAGTAGT AGTTACATATACTACGC AGACTCAGTGAAGGGC | GGGGGTTCGAGGGGGTTCGA CCCC |
| | 21-225_72G9 | | SEQ ID NO:8043 | SEQ ID NO:16055 | SEQ ID NO:24067 |
| | | AA | SYSMN | SISGSSSYIYYADSVKG | GGSRGFDP |
| | | | SEQ ID NO:8044 | SEQ ID NO:16056 | SEQ ID NO:24068 |
| iPS:448908 | | NA | AGCTATGGCATGCAC | GTTATATCACAAGATGG AATTATTAGATACTATGC AGACTCCGTGAAGGGC | GATGTGAAGCAGTGGCTGGT ACGGACCTACGGTATGGACG TC |
| | 21-225_50G9 | | SEQ ID NO:8045 | SEQ ID NO:16057 | SEQ ID NO:24069 |
| | | AA | SYGMH | VISQDGIIRYYADSVKG | DVKQWLVRTYGMDV |
| | | | SEQ ID NO:8046 | SEQ ID NO:16058 | SEQ ID NO:24070 |
| iPS:451102 | | NA | TACTATGGCTTGCAC | GTTATATCATATGATGGA AGTAATAAATATTATGC AGACTCCGTGAAGGGC | GAGGATCGATATTGTAGTGG TACCAGCTGCCCCTACTACT ACTACTACGGTATGGACGTC |
| | 21 225 45F6 | | | | |

FIGURE 49

| | | | SEQ ID NO:8047 | SEQ ID NO:16059 | SEQ ID NO:24071 |
|---|---|---|---|---|---|
| | 21-225_45F6 | AA | YYGLH | VISYDGSNKYYADSVKG | EDRYCSGTSCPYYYYYGMDV |
| | | | SEQ ID NO:8048 | SEQ ID NO:16060 | SEQ ID NO:24072 |
| iPS:451104 | 21-225_49C5 | NA | AGCTATGGTATCAGC | TGGATCAGCGCTTATAATGGTAACACAAAGTATGCACAGAAGCTCCAGGGC | CACGATTTTTGGAGTGGTTATTATAAGGGTATGGACGTC |
| | | | SEQ ID NO:8049 | SEQ ID NO:16061 | SEQ ID NO:24073 |
| | | AA | SYGIS | WISAYNGNTKYAQKLQG | HDFWSGYYKGMDV |
| | | | SEQ ID NO:8050 | SEQ ID NO:16062 | SEQ ID NO:24074 |
| iPS:451106 | 21-225_49D10 | NA | AGCTATGGTATCAGC | TGGATCAGCGCTTATAATGGTAACACAAAGAATGCACAGAAGCTCCAGGGC | CACGATTTTTGGAGTGGTTATTATAAGGGTATGGACGTC |
| | | | SEQ ID NO:8051 | SEQ ID NO:16063 | SEQ ID NO:24075 |
| | | AA | SYGIS | WISAYNGNTKNAQKLQG | HDFWSGYYKGMDV |
| | | | SEQ ID NO:8052 | SEQ ID NO:16064 | SEQ ID NO:24076 |
| iPS:451108 | 21-225_53E8 | NA | AGCTATGGTATCAGC | TGGATCAGCGCTTATAATGGTAACACAAAGTTTGCACAGAAGCTCCAGGGC | CACGATTTTTGGAGTGGTTATTATAAGGGTATGGACGTC |
| | | | SEQ ID NO:8053 | SEQ ID NO:16065 | SEQ ID NO:24077 |
| | | AA | SYGIS | WISAYNGNTKFAQKLQG | HDFWSGYYKGMDV |
| | | | SEQ ID NO:8054 | SEQ ID NO:16066 | SEQ ID NO:24078 |

FIGURE 49

| iPS:451110 | | NA | AGCTATGGCATGCAC | GTTATATGGTATGATGG AAATAATAAATCCTATG CAGACTCCGTGAAGGGC | GATCGAGATTATTGTAGTAG TACCAGCTGCCCTTATTATTA CTACTACGGTATGGACGTC |
| --- | --- | --- | --- | --- | --- |
| | 21-225_74C9 | | SEQ ID NO:8055 | SEQ ID NO:16067 | SEQ ID NO:24079 |
| | | AA | SYGMH | VIWYDGNNKSYADSVKG | DRDYCSSTSCPYYYYYGMDV |
| | | | SEQ ID NO:8056 | SEQ ID NO:16068 | SEQ ID NO:24080 |
| iPS:451112 | | NA | GGCTACTATATACAC | TGGATCAACCCTAACAG TGGTGGCACAAACTATG CACAGAAGTTTCAGGGC | GAAAACGAAAGTCTAGCAAC TCGTCCTTTCTACGACTACTA CGGTATGGACGTC |
| | 21-225_53D10 | | SEQ ID NO:8057 | SEQ ID NO:16069 | SEQ ID NO:24081 |
| | | AA | GYYIH | WINPNSGGTNYAQKFQG | ENESLATRPFYDYYGMDV |
| | | | SEQ ID NO:8058 | SEQ ID NO:16070 | SEQ ID NO:24082 |
| iPS:451114 | | NA | GACTATGTCATGCAG | GTTATATGGTATGATGG AAGTAATAAATACTATG CAGACTCCGTGAAGGGC | GAACCGTATAATAGTGGCTG GTACGACTACGGTATGGACG TC |
| | 21-225_159A3 | | SEQ ID NO:8059 | SEQ ID NO:16071 | SEQ ID NO:24083 |
| | | AA | DYVMQ | VIWYDGSNKYYADSVKG | EPYNSGWYDYGMDV |
| | | | SEQ ID NO:8060 | SEQ ID NO:16072 | SEQ ID NO:24084 |
| iPS:451116 | | NA | AATTATGATATCAAC | TGGATGCACCCTAACAG TGGTAACACAGGCTATG CACAGAAGTTCCAGGGC | AGCAGTGGCTGGTACTTCTT TGACTAC |
| | 21-225_164A4 | | SEQ ID NO:8061 | SEQ ID NO:16073 | SEQ ID NO:24085 |
| | | AA | NYDIN | WMHPNSGNTGYAQKFQG | SSGWYFFDY |
| | | | SEQ ID NO:8062 | SEQ ID NO:16074 | SEQ ID NO:24086 |

FIGURE 49

| iPS:451118 | | NA | AGTGGTGGTTACTACTGGAGC | TATATCTATTACAGTGGGACCACCATTTACAACCCCTCCCTCAAGAGT | GACACGTTTTGCTTTGATGGTTGTGGTTATTTCTTTGACTCC |
|---|---|---|---|---|---|
| | 21-225_191C8 | | SEQ ID NO:8063 | SEQ ID NO:16075 | SEQ ID NO:24087 |
| | | AA | SGGYYWS | YIYYSGTTIYNPSLKS | DTFCFDGCGYFFDS |
| | | | SEQ ID NO:8064 | SEQ ID NO:16076 | SEQ ID NO:24088 |
| iPS:451120 | | NA | AGCCATGGCATGCAC | GTTATATGGTATGATGGAAGTAATGAACACTATGCAGACTCCGTGAAGGGC | GATCAAGGTGTGGGGTACTACGGTATGGACGTC |
| | 21-225_197D3 | | SEQ ID NO:8065 | SEQ ID NO:16077 | SEQ ID NO:24089 |
| | | AA | SHGMH | VIWYDGSNEHYADSVKG | DQGVGYYGMDV |
| | | | SEQ ID NO:8066 | SEQ ID NO:16078 | SEQ ID NO:24090 |
| iPS:451122 | | NA | GTTTACTATTGGAGC | GAAATCAATCATAGTGGAAGCACCAACTACAACCCGTCCCTCAAGAGT | GACTACGGTGTCTTTGACTAC |
| | 21-225_200A1 | | SEQ ID NO:8067 | SEQ ID NO:16079 | SEQ ID NO:24091 |
| | | AA | VYYWS | EINHSGSTNYNPSLKS | DYGVFDY |
| | | | SEQ ID NO:8068 | SEQ ID NO:16080 | SEQ ID NO:24092 |
| iPS:451124 | | NA | AATTATGATATCAAC | TGGATGCACCCTAACAGTGGTAACACAGGCTATGCACAGAAGTTCCAGGGC | AGCAGTGGCTGGTACTTTTTTGACTAC |
| | 21-225_74F6 | | SEQ ID NO:8069 | SEQ ID NO:16081 | SEQ ID NO:24093 |
| | | AA | NYDIN | WMHPNSGNTGYAQKFQG | SSGWYFFDY |
| | | | SEQ ID NO:8070 | SEQ ID NO:16082 | SEQ ID NO:24094 |
| iPS:451127 | | NA | AATTATGATGTCAAC | TGGATGCACCCTAACAGTGGTAACACAGGCTATGCACAGAAGTTCCAGGGC | AGCAGTGGCTGGTACCTCTTTGACTAC |

FIGURE 49

| | 21-225_164A7 | | SEQ ID NO:8071 | SEQ ID NO:16083 | SEQ ID NO:24095 |
|---|---|---|---|---|---|
| | | AA | NYDVN | WMHPNSGNTGYAQKFQG | SSGWYLFDY |
| | | | SEQ ID NO:8072 | SEQ ID NO:16084 | SEQ ID NO:24096 |
| iPS:451129 | | NA | AATTATGATATCAAC | TGGATGCACCCTAACAG TGGTAACACAGGCTTTG CACAGAAGTTCCAGGGC | TCCAGTGGCTGGTACTGGTT CGACCCC |
| | 21-225_94D2 | | SEQ ID NO:8073 | SEQ ID NO:16085 | SEQ ID NO:24097 |
| | | AA | NYDIN | WMHPNSGNTGFAQKFQG | SSGWYWFDP |
| | | | SEQ ID NO:8074 | SEQ ID NO:16086 | SEQ ID NO:24098 |
| iPS:451131 | | NA | AATTATGATATCAAC | TGGATGCACCCTCACAG TGGTAACACAGGCTATG CACAGAAGTTCCAGGGC | AGCAGTGGCTGGTACTACTT TGACTAC |
| | 21-225_160A7 | | SEQ ID NO:8075 | SEQ ID NO:16087 | SEQ ID NO:24099 |
| | | AA | NYDIN | WMHPHSGNTGYAQKFQG | SSGWYYFDY |
| | | | SEQ ID NO:8076 | SEQ ID NO:16088 | SEQ ID NO:24100 |
| iPS:451133 | | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | 21-225_95H4 | | SEQ ID NO:8077 | SEQ ID NO:16089 | SEQ ID NO:24101 |
| | | AA | NYDIN | WMNPNSGNTGYAQKFQG | SSGWNWFDP |
| | | | SEQ ID NO:8078 | SEQ ID NO:16090 | SEQ ID NO:24102 |
| iPS:437240 | | NA | AGTTATGATATCAAC | TGGCTGAACCCTCACAG TGGTAACACAGGCTATG CACAGAAGTTCCAGGGC | GGGTTTTACGATATTTTGACT GGTTATTCCCCCACCTACTA CTACTACGATATGGACGTC |
| | 21-225_84H12 | | SEQ ID NO:8079 | SEQ ID NO:16091 | SEQ ID NO:24103 |

FIGURE 49

| | | AA | SYDIN | WLNPHSGNTGYAQKFQG | GFYDILTGYSPTYYYYDMDV |
|---|---|---|---|---|---|
| | | | SEQ ID NO:8080 | SEQ ID NO:16092 | SEQ ID NO:24104 |
| iPS:434577 | 21-225_75C11 | NA | AGTTATGATATCAAC | TGGCTGAACCCTCACAG TGGTAACACAGGCTATG CACAGAAGTTCCAGGGC | GGGTTTTACGATATTTTGACT GGTTATTCCCCCACCTACTA CTACTACGATATGGACGTC |
| | | | SEQ ID NO:8081 | SEQ ID NO:16093 | SEQ ID NO:24105 |
| | | AA | SYDIN | WLNPHSGNTGYAQKFQG | GFYDILTGYSPTYYYYDMDV |
| | | | SEQ ID NO:8082 | SEQ ID NO:16094 | SEQ ID NO:24106 |
| iPS:435477 | 21-225_154E8 | NA | AGCTATGCCATGAGC | GCTATTAGTGGTAGTGGT GGTAACACATTCTACGC AGACTCCGTGAAGGGC | CACGGTATAGCAGTGGCTGG TACTGGGGCTCACTACTTTG ACTAC |
| | | | SEQ ID NO:8083 | SEQ ID NO:16095 | SEQ ID NO:24107 |
| | | AA | SYAMS | AISGSGGNTFYADSVKG | HGIAVAGTGAHYFDY |
| | | | SEQ ID NO:8084 | SEQ ID NO:16096 | SEQ ID NO:24108 |
| iPS:434553 | 21-225_76H12 | NA | AGTTATGATATCAAC | TGGCTGAACCCTCACAG TGGTAACACAGGCTATG CACAGAAGTTCCAGGGC | GGGTTTTACGATATTTTGACT GGTTATTCCCCCACCTACTA CTACTACGATATGGACGTC |
| | | | SEQ ID NO:8085 | SEQ ID NO:16097 | SEQ ID NO:24109 |
| | | AA | SYDIN | WLNPHSGNTGYAQKFQG | GFYDILTGYSPTYYYYDMDV |
| | | | SEQ ID NO:8086 | SEQ ID NO:16098 | SEQ ID NO:24110 |
| iPS:434927 | 21-225_86E5 | NA | AGTTATGATATCAAC | TGGATGAACCCTAACAG TGGTAACACAGGCTATG CACAGAAGTTCCAGGGC | GGGTTTTACGATATTTTGACT GGTTATTCCCCCACCTACTA CTACTACGATATGGACGTC |
| | | | SEQ ID NO:8087 | SEQ ID NO:16099 | SEQ ID NO:24111 |

FIGURE 49

| | | | AA | SYDIN | WMNPNSGNTGYAQKFQG | GFYDILTGYSPTYYYYDMDV |
|---|---|---|---|---|---|---|
| | | | | SEQ ID NO:8088 | SEQ ID NO:16100 | SEQ ID NO:24112 |
| iPS:435385 | | | NA | AGCTATGCCATGAGC | GCTATTAGTGGTAGTGGT GGTAACACATTCTACGC AGACTCCGTGAAGGGC | CACGGTATAGCAGTGGCTGG TACTGGGGCTCACTACTTTG ACTAC |
| | 21-225_149G7 | | | SEQ ID NO:8089 | SEQ ID NO:16101 | SEQ ID NO:24113 |
| | | | AA | SYAMS | AISGSGGNTFYADSVKG | HGIAVAGTGAHYFDY |
| | | | | SEQ ID NO:8090 | SEQ ID NO:16102 | SEQ ID NO:24114 |

FIGURE 50

Table 3
Standard IgG Antibody Variable Region Sequences

| iPS# | Ab | Type | LC V-region | HC V-region |
|---|---|---|---|---|
| iPS:451135 | 21-225_64A11 | NA | GACATCCAGATGACCCAGTCTCCATTCTCCCT GTCTGCATCTGTAGGCGACAGAGTCACCATCA CTTGCCGGGCAAGTCGGAGCGTTAGCAGATAT TTAAATTGGTATCAGCAGACACTGGGGAAAGC CCTTAAGCTCTTGATATCTGTTGCATCCCGTTT GCAAAGTGGGGTCCCATCGAGGTTCAGTGGCA GTGGATCTGGGACAGATTTCACTCTCACCATC AGCAGTGTGCAACGTGAAGATTTTGCAACTTA CTTCTGTCAACAGAGTGACAGTTTCCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAACTATGGCAT GCACTGGGTCCGCCAGGCTCCAGGCAAGGGGCT GGAGTGGGTGGCAGTTATATCATATGTTGGAAGT AATAAATACTATGCAGACTCCGTGAAGGGCCGA TTCACCATCTCCAGAGACAATTCCAAGAACACGC TGTATCTGCAAATGAACACCCTGAGAGCTGAGG ACACGGCTGTGTATTACTGTGCGAGGAGAGGAG CAGTGGCTCCGTACTACGGTATGGACGTCTGGGG CCAAGGGACCACGGTCACCGTCTCCTCA |
|  |  |  | SEQ ID NO: 24115 | SEQ ID NO: 28121 |
|  |  | AA | DIQMTQSPFSLSASVGDRVTITCRASRSVSRYLN WYQQTLGKALKLLISVASRLQSGVPSRFSGSGSG TDFTLTISSVQREDFATYFCQQSDSFPLTFGGGTK VEIK | QVQLVESGGGVVQPGRSLRLSCAASGFTFSNYGM HWVRQAPGKGLEWVAVISYVGSNKYYADSVKGR FTISRDNSKNTLYLQMNTLRAEDTAVYYCARRGAV APYYGMDVWGQGTTVTVSS |
|  |  |  | SEQ ID NO: 24116 | SEQ ID NO: 28122 |

FIGURE 50

| iPS:451141 | 21-225_164B11 | NA | GACATCGTGATGACCCAGTCTCCAGACTCCCT GGCTGTGTCTCTGGGCGAGAGGGCCACCATCA ACTGCAAGTCCAGCCAGAGTCTTTTAAAGAGC TCCAACAATAAGAGCTACTTAGCTTCGTACCA GCAGAAGCCAGGACAGCTTCCTAAACTGCTCA TTTACTGGGCATCTTCCCGGGAATCCGGGGTC CCTGACCGATTCAGTGGCAGCGGGTCTGGGAC AGATTTCACTCTCACCATCAGCAGCCTGCAGG CTGAAGATGTGGCACTTTATTACTGTCAGCAA TATTATAGTATTCCTCCCACTTTCGGCCATGGG ACCAATGTGGATATCACG | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGACCCCTAACAGTGG TAACACAGGCTATGCACAGAAGTTCCAGGGCAG AGTCACCATGACCAGGAACACTTCCATGAGCACC GCCTACATGGAGCTGAGCAGCCTGAGATCTGAG GACTCGGCCGTGTATTACTGTTCCTATAGCAGTG GCTGGTACATGTTTGACTACTGGGGCCAGGGAAC CCTGGTCACCGTCTCCTCA |
| | | | SEQ ID NO: 24117 | SEQ ID NO: 28123 |
| | | AA | DIVMTQSPDSLAVSLGERATINCKSSQSLLKSSN NKSYLASYQQKPGQLPKLLIYWASSRESGVPDR FSGSGSGTDFTLTISSLQAEDVALYYCQQYYSIPP TFGHGTNVDIT | QVQLVQSGAEVKKPGASVKVSCKASGYTFTNYDIN WVRQATGQGLEWMGWMTPNSGNTGYAQKFQGR VTMTRNTSMSTAYMELSSLRSEDSAVYYCSYSSG WYMFDYWGQGTLVTVSS |
| | | | SEQ ID NO: 24118 | SEQ ID NO: 28124 |
| iPS:451137 | 21-225_74A7 | NA | GACATCGTTATGACCCAGTCTCCAGACTCCCT GGCTGTGTCTCTGGGCGAGAGGGCCACCATCA ACTGCAAGTCCAGCCAGAGTGTTTTATTCAGC TCCAACAATTATAACTACTTAGCTTGGTACCA GCAGAGACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGCTGATGTGGCAGTTTATTACTGTCAGCAA TATCATAGTTCTCCTCCGACGTTCGGCCAAGG GACCACGGTGCAAATCAAA | CAGGTGCAGTTGGTGCAGTCTGGGGCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGAACCCTAACAGTGG TAACACAGGCTATGCACAGAAGTTCCAGGGCAG AGTCACCATGACCAGGAACACCTCCACAAGCAC AGCCCACATGGAGCTGAGCAGCCTGAGATCTGA GGACACGGCCGTGTATTACTGTGCGGGTCTCCAGT GGCTGGAACTGGTTCGACCCCTGGGGCCAGGGA ACCCTGGTCACCGTCTCCTCA |
| | | | SEQ ID NO: 24119 | SEQ ID NO: 28125 |

FIGURE 50

| | | | | |
|---|---|---|---|---|
| | | AA | DIVMTQSPDSLAVSLGERATINCKSSQSVLFSSN NYNYLAWYQQRPGQPPNLLIYWASTRESGVPD RFSGSGSGTDFTLTISSLQAADVAVYYCQQYHSS PPTFGQGTTVQIK | QVQLVQSGAEVKKPGASVKVSCKASGYTFTNYDIN WVRQATGQGLEWMGWMNPNSGNTGYAQKFQGR VTMTRNTSTSTAHMELSSLRSEDTAVYYCAVSSGW NWFDPWGQGTLVTVSS |
| | | | SEQ ID NO: 24120 | SEQ ID NO: 28126 |
| iPS:451139 | 21-225_71A6 | NA | GATATTGTGATGACCCAGACTCCACTCTCTCTC TCTGTCACACCTGGACAGCCGGCCTCCATCTC CTGCAAGTCTAGTCAGAGCCTCCTGCGTAGTG ATGGAAAGACCCCATTTGTATTGGTACCTGCAG AAGCCAGGCCAGCCTCCACAGCTCTTAATCTA TGAAGTTTCCAACCGGTTCTCTGGAGTGTCAG ATAGGTTCAGTGGCAGCGGGTCAGGGACAGA TTTCACACTGAAAATCAGCCGGGTGGAGGCTG AGGATGTTGGGGTTTATTACTGCATGCAAAGT AAACAGCTTCCTCTCACTTTCGGCGGAGGGAC CAAGGTGGAGTTCAAA | CAGGTGCAACTGGTGGAGTCTGGGGGAGGCGTG GTCCAGCCTGGGAGGTCCCTGAGACTCTCCTGTG CAGCCTCTGGATTCACCTTCAGTAACTATGGCAT GCACTGGGTCCGCCAGGCTCCAGGCAAGGGGCT GGAGTGGGTGGCAGTTATATCATATGATGGAAGT AATGAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGCGAGAGATCACAG ATATGGGGTTCGGGGAGGCTTTGACTACTGGGGC CAGGGAACCCTGGTCACCGTCTCCTCA |
| | | | SEQ ID NO: 24121 | SEQ ID NO: 28127 |
| | | AA | DIVMTQTPLSLSVTPGQPASISCKSSQSLLRSDGK THLYWYLQKPGQPPQLLIYEVSNRFSGVSDRFSG SGSGTDFTLKISRVEAEDVGVYYCMQSKQLPLT FGGGTKVEFK | QVQLVESGGGVVQPGRSLRLSCAASGFTFSNYGM HWVRQAPGKGLEWVAVISYDGSNEYYADSVKGRF TISRDNSKNTLYLQMNSLRAEDTAVYYCARDHRY GVRGGFDYWGQGTLVTVSS |
| | | | SEQ ID NO: 24122 | SEQ ID NO: 28128 |

1400

FIGURE 50

| iPS:451143 | 21-225_66H11 | NA | GACATCCAGATGACCCAGTTTCCATCCTCACT GTTTGCATTTGTAGGAGACAGAGTCACCATCA CTTGTCCGGCGAGTCAGGGCATTAGCAATTAT TTAGCTTGGTTTCAGCAGAAACCAGGGAAAGC CCCTAAGTCCCTTATTTATGGTGCATTCAATTT GCACAGTGGGGTCCCATCAAAGTTCAGCGGCA GTGGATTTGGGACAGATTTCACTCTCACCATC AACAGCCTGCAGCCTGAAGATTTTGCAAATTA TTACTGCCAACAGTATAGTTGTTACCCATTCAC TTTCGGCCATGGGACCAAAGTGGATATCAAA | CAGGTTCAGCTGGTCCAGTCTGGAGCTGAGGTGA AGAAGCCTGGGGCCTCAGTGAAGGTCTCCTGCA AGGCTTCTGGTTACACCTTTGCCACCTATGGTAT CAGCTGGGTGCGACAGGCCCCTGGACAGGGGCT TGAGTGGATGGGATGGATCAGCGCTTACAATGGT AACACAAACTATGCACAGAAGCTCCAGGGCAGA GTCACCATGACCACAGACACATCCACGAGCACA GCCTACATGGAGCTGAGGAGCCTGAGATCTGAC GACACGGCCGTGTATTACTGTGCGAGAGGGGAA GCAGTGGCTGTCTTCGACCCCTGGGGCCAGGGAA CCCTGGTCACCGTCTCCTCA |
| | | | SEQ ID NO: 24123 | SEQ ID NO: 28129 |
| | | AA | DIQMTQFPSSLFAFVGDRVTITCPASQGISNYLA WFQQKPGKAPKSLIYGAFNLHSGVPSKFSGSGF GTDFTLTINSLQPEDFANYYCQQYSCYPFTFGHG TKVDIK | QVQLVQSGAEVKKPGASVKVSCKASGYTFATYGIS WVRQAPGQGLEWMGWISAYNGNTNYAQKLQGR VTMTTDTSTSTAYMELRSLRSDDTAVYYCARGEA VAVFDPWGQGTLVTVSS |
| | | | SEQ ID NO: 24124 | SEQ ID NO: 28130 |
| iPS:453445 | 21-225_148E10 | NA | TCCTATGAGCTGACTCAGCCACCCTCAGTGTC CGTGTCCCCAGGACAGACAGCCAGCATCACCT GCTCTGGAGATAAATTGGGTAATAAATATGTT TGTTGGTATCAGCAGAGGCCAGGCCATGCTGC TGTGCTGATCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT TCTGTCAGGCGTGGGACAGGAACACTTATGTG GTGTTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTTTGATGGCAGT AATAAATACTATGTAGACTCCGTGAAGGACCGAT TCACCATCTCCAGAGACAATTCCAAGAACACGCT GTATCTGCAAATGAACAGCCTGAGAGCCGAGGA CACGGCTGTTTATTACTGTGCGAGAGATCGGGTG GAGGGTTCGGGGACTCCCTACTACTACTACGGTA TGGACGTCTGGGGCCAAGGGACCACGGTCACCG TCTCCTCA |
| | | | SEQ ID NO: 24125 | SEQ ID NO: 28131 |

FIGURE 50

| | | | | |
|---|---|---|---|---|
| | | AA | SYELTQPPSVSVSPGQTASITCSGDKLGNKYVCW YQQRPGHAAVLIIYQDSKRPSGIPERFSGSNSGNT ATLTISGTQAMDEADYFCQAWDRNTYVVFGGG TKLTVL | QVQLVESGGGVVQPGRSLRLSCAASGFTFSSYGMH WVRQAPGKGLEWVAVIWFDGSNKYYVDSVKDRF TISRDNSKNTLYLQMNSLRAEDTAVYYCARDRVEG SGTPYYYYGMDVWGQGTTVTVSS |
| | | | SEQ ID NO: 24126 | SEQ ID NO: 28132 |
| IPS:453447 | 21-225_65F10 | NA | GACATCCAGATGACCCAGTCTCCATCTTCCGT GTCTGCATCTGTAGGAGACAGAGTCACCATCA CTTGTCGGGGGGGTCAGGGTATTAGCACATGG TTAGCATGGTATCAGCAGAAACCAGGGAAAG CCCCTAAGCTCCTGATCTATGCTGCATCCATTT TGCAAAGTGGGGTCCCATCAAGGTTCAGAGGC AGGGGATCTGGGACAGATTTCACTCTCACCAT CAGCAGCCTGCAGCCTGAAGATTTTGCAACTT ATTATTGTCAACAGGGTAACATTTTCCCATTCA CTTTCGGCCGAGGGACCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACCATA TGCACTGGGTGCGACAGGCCCCTGGACAAGGGC TTGAGTGGATGGGATGGATCAACCCTAACAATGG TGGCACAAGCTATGCACAGAAGTTTCAGGACAG GGTCAACATGACCAGGGACACGTCCATCAGCAC AGCCTACATGGAGCTGAGCAGGCTGAGATCTGA CGACACGGCCGTGTACTACTGTGCGAGAGATAGT AGGTCGTCCTGGGACTACTGGGGCCAGGGAACC CTGGTCACCGTCTCCTCA |
| | | | SEQ ID NO: 24127 | SEQ ID NO: 28133 |
| | | AA | DIQMTQSPSSVSASVGDRVTITCRGGQGISTWLA WYQQKPGKAPKLLIYAASILQSGVPSRFRGRGS GTDFTLTISSLQPEDFATYYCQQGNIFPFTFGRGT KVDIK | QVQLVQSGAEVKKPGASVKVSCKASGYTFTGYHM HWVRQAPGQGLEWMGWINPNNGGTSYAQKFQDR VNMTRDTSISTAYMELSRLSDDTAVYYCARDSRS SWDYWGQGTLVTVSS |
| | | | SEQ ID NO: 24128 | SEQ ID NO: 28134 |

FIGURE 50

| iPS:453449 | 21-225_208A2 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGCAGCAACCAGGGAAAA CCCCTAAGCGCCTGATCTATGCTGCATCCAGT TTGCTTAGTGGGGTCCCATCAAGGTTCAGCGG CAGTAGATCTGGGACAGATTTCACTCTCACAA TCAGCAGCCTGCAGCCTGAAGATTTTGCAACT TATTACTGTCTACAGTATAATAGTTACCCTCCC ACCTTCGGCCAAGGGACACGACTGGAGATTAA A | CAGGTGCAGCTGCAGGAGTCGGGCCCAGGACTG GTGAAGCCTTCGGAGACCCTGTCCCTCAACTGCA CTGTCTCTGGTGGCTCCATCAGGAGTTACTACTG GAGCTGGATCCGGCAGCCCGCCGGGAAGGGACT GGAGTGGATTGGGCGTATCTATACCAGTGGGAGC ACCGACTACAACCCCTCCCTCAAGAGTCGAATCA CCATGTCAGTAGACACGTCCAAGAACCAGTTCTC TTTGAAGCTGAGCTCTGTGACCGCCGCGGACACG GCCGTGTATTACTGTGCGAGAGGGTTCGGTGACT GGGACTACTGGGGCCAGGGAACCCTGGTCACCG TCTCCTCA |
|---|---|---|---|---|
| | | | SEQ ID NO: 24129 | SEQ ID NO: 28135 |
| | | AA | DIQMTQSPSSLSASVGDRVTITCRTSQGIRNDLG WYQQQPGKTPKRLIYAASSLLSGVPSRFSGSRSG TDFTLTISSLQPEDFATYYCLQYNSYPPTFGQGT RLEIK | QVQLQESGPGLVKPSETLSLNCTVSGGSIRSYYWS WIRQPAGKGLEWIGRIYTSGSTDYNPSLKSRITMSV DTSKNQFSLKLSSVTAADTAVYYCARGFGDWDYW GQGTLVTVSS |
| | | | SEQ ID NO: 24130 | SEQ ID NO: 28136 |
| iPS:453451 | 21-225_52G11 | NA | GACATCCAGATGACCCAGTCTCCATCTTCCGT GTCTGCATCTGTAGGAGACAGAGTCACCATCA CTTGTCGGGCGAGTCAGGGTATTAGCAAATGG TTAGCCTGGTATCAGCAGAAACCAGGGAAAG CCCCTAAGCTCCTGCTCTATGCTGCATCCAGTT TGCAAAGTGGGGTCCCATCAAGATTCAGCGCC AGTGGATCTGGGACAGATTTCACTCTCACCAT CAGCAGTTTGCAGCCTGAAGATTTTGCAACTT ACTATTGTCAACAGGCTAACAGTTTCCCATTC ACTTTCGGCCCTGGGACCAAAGTGGATGTCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATT TGCACTGGGTGCGACAGGCCCCTGGACAAGGGC TTGAGTGGATGGGATGGATCAACCCTAATAGAA ATGGCACAAACTATGCACAGAAGTTTCAGGGCA GGGTCACCATGACCAGGGACACGTCCATCAGCA CAGCCTTCATGGAGCTGAGCAGGCTGAGATCTGA CGACACGGCCGTGTATTACTGTGCGAGAGACGGT ACCAGCAGCTTTGACTACTGGGGCCAGGGAACC CTGGTCACCGTCTCCTCA |
| | | | SEQ ID NO: 24131 | SEQ ID NO: 28137 |

1403

FIGURE 50

| | | | | |
|---|---|---|---|---|
| | | AA | DIQMTQSPSSVSASVGDRVTITCRASQGISKWLA WYQQKPGKAPKLLLYAASSLQSGVPSRFSASGS GTDFTLTISSLQPEDFATYYCQQANSFPFTFGPGT KVDVK | QVQLVQSGAEVKKPGASVKVSCKASGYTFTGYYL HWVRQAPGQGLEWMGWINPNRNGTNYAQKFQGR VTMTRDTSISTAFMELSRLRSDDTAVYYCARDGTS SFDYWGQGTLVTVSS |
| | | | SEQ ID NO: 24132 | SEQ ID NO: 28138 |
| IPS:453453 | 21-225_53F2 | NA | GACATCCAGATGACCCAGTCTCCATCTTCCGT GTCTGCATCTGTAGGAGACAGAGTCACCATCA CTTGTCGGGCGAGTCAGGGTATTAGCAAGTGG TTAGCCTGGTATCAGCAGAAACCAGGGAAAG CCCCTAACCTCCTTCTCTATGCTGCATCCAGTT TGCAAAGTGGGGTCCCATCAAAATTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATGTCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATT TGCACTGGGTGCGACAGGCCCCTGGACAAGGGC TTGAGTGGATGGGATGGATCAACCCTAACAGAA ATGGCACAAACTATGCACAGAATTTTCAGGGCA GGGTCACCATGACCAGGGACACGTCCATCAGCA CAGCCTACATGGAGCTGAGCAGACTGAAATCTG ACGACACGGCCGTGTATTACTGTGCGAGAGACG GTACCAGTAGCTTTGACTACTGGGGGCCAGGGAAC CCTGGTCACCGTCTCCTCA |
| | | | SEQ ID NO: 24133 | SEQ ID NO: 28139 |
| | | AA | DIQMTQSPSSVSASVGDRVTITCRASQGISKWLA WYQQKPGKAPNLLLYAASSLQSGVPSKFSGSGS GTDFTLTISSLQPEDFATYYCQQANSFPFTFGPGT KVDVK | QVQLVQSGAEVKKPGASVKVSCKASGYTFTGYYL HWVRQAPGQGLEWMGWINPNRNGTNYAQNFQGR VTMTRDTSISTAYMELSRLKSDDTAVYYCARDGTS SFDYWGQGTLVTVSS |
| | | | SEQ ID NO: 24134 | SEQ ID NO: 28140 |

FIGURE 50

| iPS:468810 | 21-225_74D5 | NA | GAAATTGTGTTGACGCAGTCTCCAGGCACCCT GTCTTTGTCTCCAGGGGAGAGAGCCACCCTCT CCTGCAGGGCCAGTCAGAGTGTTAACAGCAAC TACTTAGCCTGGTACCGGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCATTCTCAT CATCAGCAGACTGGAGCCTGAGGATTTTGCAG TGTATTACTGTCAGCAGTATGAAAGCTCGCCG TGGACGTTCGGCCAAGGGACCAAGGTGGAAA TCAAA | CAGGTGCAGCTACAACAGTGGGGCGCAGGACTG TTGAAGCCTTCGGAGACCCTGTCCCTCACTTGCG CTGTCAATGGTGGGCCCTTCAGCGGTTGCTACTG GAGCTGGATCCGCCAGCCCCCAGGGAAGGGGCT GGAGTGGATTGGGGAAATCAATTATAGTGGAAG GACCAACTTCAACCCGTCCCTCAAGAGTCGAGTC ACCATATCAGTTGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
|---|---|---|---|---|
| | | | SEQ ID NO: 24135 | SEQ ID NO: 28141 |
| | | AA | EIVLTQSPGTLSLSPGERATLSCRASQSVNSNYLA WYRQKPGQAPRLLIYGASSRATGIPDRFSGSGSG TDFILIISRLEPEDFAVYYCQQYESSPWTFGQGTK VEIK | QVQLQQWGAGLLKPSETLSLTCAVNGGPFSGCYW SWIRQPPGKGLEWIGEINYSGRTNFNPSLKSRVTISV DTSKNQFSLKLSSVTAADTAVYYCARDYGGMDV WGQGTTVTVSS |
| | | | SEQ ID NO: 24136 | SEQ ID NO: 28142 |
| iPS:468812 | 21-225_48H4 | NA | GACATCCAGATGACCCAGTTTCCATCCTCCCT GTCTGCATCTGTAGGAGACAGAGTCACCATCA CTTGCCGGGCAAGTCGAGACATTAGAAATGAT TTAGGCTGGTATCAGCAGAAACCAGGAAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATTATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTCTCTCAT GCACTGGGTCCGCCAGGCTCCAGGCAAGGGGCT GGAGTGGGTGGCAGTTATATGGTATGATGGAAGT AATAAATACTATACAGACTCCGTTAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAAAACTAT AGCAGTGGCTGGTACGGGTACGGTATGGACGTCT GGGGCCAAGGGACCACGGTCACCGTCTCCTCA |
| | | | SEQ ID NO: 24137 | SEQ ID NO: 28143 |

FIGURE 50

| | | | | |
|---|---|---|---|---|
| | | AA | DIQMTQFPSSLSASVGDRVTITCRASRDIRNDLG WYQQKPGKAPKRLIYAASSLQSGVPSRFSGSGS GTEFTLTISSLQPEDFATYYCLQHYSYPLTFGGG TKVEIK | QVQLVESGGGVVQPGRSLRLSCAASGFTFSDSLMH WVRQAPGKGLEWVAVIWYDGSNKYYTDSVKGRF TISRDNSKNTLYLQMNSLRAEDTAVYYCARENYSS GWYGYGMDVWGQGTTVTVSS |
| | | | SEQ ID NO: 24138 | SEQ ID NO: 28144 |
| iPS:468816 | 21-225_52G8 | NA | GATATTGTGATGACCCAGACTCCACTCTCTCT GTCCGTCACCCCTGGACAGCCGGCCTCCATGT CCTGCAAGTCTAGTCAGAGCCTCCTGCATAGT GAAGGAAAGACCTATTTGTATTGGTACCTGCA GAAGACAGGCCAGCCTCCACACCTCCTGATCT ATGAAGTTTCCAAGCGGCTCTCTGGCGTGCCA GATAGGTTCAGTGGCAGCGGGTCAGGGACAG ATTTCACACTGAAAATCAGCCGGATGGAGGCT GAGGATGTTGGGGTTTATTACTGCATGCAAAG TATGCAGCTTCCGATTATCTTCGGCCAGGGGA CACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTCTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 24139 | SEQ ID NO: 28145 |
| | | AA | DIVMTQTPLSLSVTPGQPASMSCKSSQSLLHSEG KTYLYWYLQKTGQPPHLLIYEVSKRLSGVPDRF SGSGSGTDFTLKISRMEAEDVGVYYCMQSMQLP IIFGQGTRLEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTALYYCARRYSSS WSGGMDVWGQGTTVTVSS |
| | | | SEQ ID NO: 24140 | SEQ ID NO: 28146 |

FIGURE 50

| | | | | |
|---|---|---|---|---|
| iPS:468814 | 21-225_223D11 | NA | GACATCCAGATGACCCAGTCTCCCTCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCACTTT GCAAAGTGGGGTCCCATCAAAGTTCAGCGGCA GTAGATCTGGGACAGATTTTAATCTCACCATC AGCAACCTGCAGCCTGAAGATTTTGCAACTTA TTACTGCCAACAGTATAGTGGTTACCCATTCA CTTTCGGCCCTGGGACCAAAGTGGATACCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GGACTGGGTCCGCCAGGCTCCAGGCAAGGGGCT GGAGTGGGTGGCAGTTATATGGTATGATGGAAGT AATGACTACTATGCAGACTCCGTGAAGGGCCGAT TCACCATCTCCAGAGACAATTCCAAGAACACGCT GTTTCTGCAAATGAACAGCCTGAGAGCCGAGGA CACGGCTGTGTATTACTGTGCGAGAGATCGGGGG ATCGGGTACAACGATATGGACGTCTGGGGCCAA GGGACCACGGTCACCGTCTCCTCA |
| | | | SEQ ID NO: 24141 | SEQ ID NO: 28147 |
| | | AA | DIQMTQSPSSLSASVGDRVTITCRASQGISNYLA WFQQKPGKAPKSLIYAASTLQSGVPSKFSGSRSG TDFNLTISNLQPEDFATYYCQQYSGYPFTFGPGT KVDTK | QVQLVESGGGVVQPGRSLRLSCAASGFTFSNYGM DWVRQAPGKGLEWVAVIWYDGSNDYYADSVKGR FTISRDNSKNTLFLQMNSLRAEDTAVYYCARDRGI GYNDMDVWGQGTTVTVSS |
| | | | SEQ ID NO: 24142 | SEQ ID NO: 28148 |
| iPS:468822 | 21-225_147E10 | NA | GCTATTGTGATGACCCAGACTCCACTCTCTCTG TCCGTCACCCCTGGACAGCCGGCCTCCATCTC CTGCAAGTCTAGTCAGCGCCTCCTGCATGGTG ATGGAAAGACCTATTTGTATTGGTACCTGCAG AAGCCAGGCCAGCCTCCACACCTCCTGATCTC TGAAGTTTCCAACCGGTTCTCTGGAGTGCCAG ATAGGTTCAGTGGCAGCGGGTCAGGGACAGA TTTCACACTGAAAATCAGCCGGGTGGAGGCTG AGGATGTTGGGGTTTATTACTGCATGCAAAGT ATACAGGTTCCGTGGACGTTCGGCCAAGGGAC CAAGGTGGAAATCAAA | CAGGTGCAGCTGGTGGAGTCTGGGGGAGGCGTG GTCCAGCCTGGGAAGTCCCTGAGACTCTCCTGTG CTGCGTCTGGATTCACCTTCAGTAACTATGGCTT ACACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCATTA CGATTTTTGGAGTGGTCACTTTGACTACTGGGGC CAGGGAACCCTGGTCACCGTCTCCTCA |
| | | | SEQ ID NO: 24143 | SEQ ID NO: 28149 |

FIGURE 50

| | | | | |
|---|---|---|---|---|
| | | AA | AIVMTQTPLSLSVTPGQPASISCKSSQRLLHGDG KTYLYWYLQKPGQPPHLLISEVSNRFSGVPDRFS GSGSGTDFTLKISRVEAEDVGVYYCMQSIQVPW TFGQGTKVEIK | QVQLVESGGGVVQPGKSLRLSCAASGFTFSNYGLH WVRQAPGKGLEWVAIIWYDGSNKYYADSVKGRFT ISRDNSKNTLYLQMNSLRAEDTAVYYCARDHYDF WSGHFDYWGQGTLVTVSS |
| | | | SEQ ID NO: 24144 | SEQ ID NO: 28150 |
| IPS:468824 | 21-225_73G6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCGTCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGTAAGT AATAAATACTATGGAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGAAGTGGG GATGACTTCTGACTACTGGGGCCAGGGAACCCTG GTCACCGTCTCCTCA |
| | | | SEQ ID NO: 24145 | SEQ ID NO: 28151 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSSYGMH WVRQAPGKGLEWVAVIWYDVSNKYYGDSVKGRF TISRDNSKNTLYLQMNSLRAEDTAVYYCAREVGM TSDYWGQGTLVTVSS |
| | | | SEQ ID NO: 24146 | SEQ ID NO: 28152 |

FIGURE 50

| iPS:468818 | 21-225_190C8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGAAACT TATTACTGTCTACAGCATAATGATTACCCGTTC ACTTTCGGCGGAGGGACCAAGGTGGAGATCA AA | CAGGTGCAGCTGGTGCAGTCTGGGGGCTGAGGTG AAGAAGCCTGGGGCCTCAGTGAAGGTCTCCTGC AAGGCTTCTGGATACACCTTCATCAGTTATGATA TCAACTGGGTGCGACAGGCCACTGGACAAGGGC TTGAGTGGATGGGATGGATGAACCCTAAAAGGG GTAACACAGGCTATGCACAGAAGTTCCAGGGCA GAGTCACCATGACCAGGGACACCTCCATAAGCA CAGCCTACATGGAGCTGAGCAGCCTGAGATCTG AGGACACGGCCGTTTATTACTGTGCGAGAGGAG ACCCGTATAACTGGAACTCCTACGCTATGGACGT CTGGGGCCAAGGGGCCACGGTCACCGTCTCCTCA |
|---|---|---|---|---|
| | | | SEQ ID NO: 24147 | SEQ ID NO: 28153 |
| | | AA | DIQMTQSPSSLSASVGDRVTITCRASQGIRNDLG WYQQKPGKAPKRLIYAASSLQSGVPSRFSGSGS GTEFTLTISSLQPEDFETYYCLQHNDYPFTFGGG TKVEIK | QVQLVQSGAEVKKPGASVKVSCKASGYTFISYDIN WVRQATGQGLEWMGWMNPKRGNTGYAQKFQGR VTMTRDTSISTAYMELSSLRSEDTAVYYCARGDPY NWNSYAMDVWGQGATVTVSS |
| | | | SEQ ID NO: 24148 | SEQ ID NO: 28154 |
| iPS:468826 | 21-225_201C5 | NA | GACATCCAGATGACCCAGTCTCCATCCTCCCT GTCTGCATCTGTAGGAGACAGAGTCACCATCA CTTGCCGGGCAAGTCAGGGCATTAGACATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTTCCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAGGAACACGCT GTATCTGCAAATGAACAGCCTGAGAGCCGAGGA CACGGCTGTGTATTACTGTGCGAGAGAACGGTAT AGCAGTGGCTTGTACGACTACGGTATGGACGTCT GGGGCCAAGGGACCACGGTCACCGTCTCCTCA |
| | | | SEQ ID NO: 24149 | SEQ ID NO: 28155 |

FIGURE 50

| | | | | |
|---|---|---|---|---|
| | | AA | DIQMTQSPSSLSASVGDRVTITCRASQGIRHDLG WYQQKPGKAPKRLIYAASSLQSGVPSRFSGSGS GTEFTLTISSLQPEDFATYYCLQHYSFPRTFGQGT KVEIK | QVQLVESGGGVVQPGRSLRLSCAASGFTFSDYVM HWVRQAPGKGLEWVAVIWYDGSNKYYVDSVKGR FTISRDNSRNTLYLQMNSLRAEDTAVYYCARERYS SGLYDYGMDVWGQGTTVTVSS |
| | | | SEQ ID NO: 24150 | SEQ ID NO: 28156 |
| iPS:468828 | 21-225_162A10 | NA | GAAATAGTGATGACGCAGTCTCCAGCCACCCT GTCTGTGTCTCCAGGGGAAAGAGCCACCCTCT CCTGCAGGGCCAGTCAGACTGTTAACAGCAAC TTAGCCTGGTACCAGCAGAAATCTGGCCAGGC TCCCAGGCTCCTCATCTTTGGTGCATCCACCAG GGCCACTGTTATCCCAGCCAGGATCAATGGCA GTGGGTCTGGGACAGAGTTCACTCTCACCATC AGCAGCCTGCGGTCTGAAGATTTTGCAGTTTA TTTCTGTCAGCAGTATAATGACTGGCCGTGCA GTTTTGGCCAGGGGACCAAGCTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTGTGGCAT GCACTGGGTCCGCCAGGCTCCAGGCAAGGGGCT GGAATGGGTGGCAGCTATATGGTATGATGGAAG CAATAAATACTATGCAGACTCCGTGAAGGGCCG ATTCACCATCTCCAGAGACAATTCCAAGAACACG CTGTTTCTGCAAATGAACAGCCTGAGAGCCGAGG ACACGGCTGTGTATTACTGTGCGAGAGACAAAA ATATAATGGGAGATACTTGGTTTGACTTCTGGGG CCAGGGAACCCTGGTCACCGTCTCCTCA |
| | | | SEQ ID NO: 24151 | SEQ ID NO: 28157 |
| | | AA | EIVMTQSPATLSVSPGERATLSCRASQTVNSNLA WYQQKSGQAPRLLIFGASTRATVIPARINGSGSG TEFTLTISSLRSEDFAVYFCQQYNDWPCSFGQGT KLEIK | QVQLVESGGGVVQPGRSLRLSCAASGFTFSSCGMH WVRQAPGKGLEWVAAIWYDGSNKYYADSVKGRF TISRDNSKNTLFLQMNSLRAEDTAVYYCARDKNIM GDTWFDFWGQGTLVTVSS |
| | | | SEQ ID NO: 24152 | SEQ ID NO: 28158 |

FIGURE 50

| iPS:468830 | 21-225_191G11 | NA | GAAATAGTGATGACGCAGTCTCCAGCCACCCT GTCTGTGTCTCCAGGGGAAAGAGCCAACCTCT CCTGCAGGACCAGTCAGAGTGTTTGGATTAGC GTAGCCTGGTACCACCAGAAACCTGGCCAGGC TCCCAGGCTCCTCATCTATGGTGCAGCCACCA GGGCCACTGGTATCCCAGCCAGGTTTAGTGGC AGTGGGTCTGGGACAGAGTTCACTCTCACCAT CAGCAGCCTGCAGTCTGAAGATTTTGCAGTTT ATTACTGTCAGCAGTATAATTACTGGCCGCTC ACTTTCGGCGGAGGGACCAAGGTGGAGATCA AA | CAGGTGCAACTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGAC TTGAGTGGATGGGATGGATCAACCCTAATAGTGG TGGCACAAACTTTGCACAGAAGTTTCAGGGCAG GGTCACCTTGACCAGGGACACGTCCATCAACACA GCCTACATGGAGCTGAGCTGGCTGCGATCTGACG ACACGGCCGTATATTACTGTGCGCGTGGAAAGA ACTATGGCTCCTACTTTGACTACTGGGGCCAGGG AACCCTGGTCACCGTCTCCTCG |
|  |  |  | SEQ ID NO: 24153 | SEQ ID NO: 28159 |
|  |  | AA | EIVMTQSPATLSVSPGERANLSCRTSQSVWISVA WYHQKPGQAPRLLIYGAATRATGIPARFSGSGS GTEFTLTISSLQSEDFAVYYCQQYNYWPLTFGG GTKVEIK | QVQLVQSGAEVKKPGASVKVSCKASGYTFTGYYM HWVRQAPGQGLEWMGWINPNSGGTNFAQKFQGR VTLTRDTSINTAYMELSWLRSDDTAVYYCARGKN YGSYFDYWGQGTLVTVSS |
|  |  |  | SEQ ID NO: 24154 | SEQ ID NO: 28160 |
| iPS:468832 | 21-225_76H10 | NA | GACATCCAGATGACCCAGTCTCCATCCTCCCT GTCTGCCTCTGCAGGAGACAGAGTCACCATCA CTTGCCGGGCAAGTCAGGACATTAGAAATTAT TTAGGCTGGTATCAGCAGAAACCAGGGAAAG CCCCTAAGCGACTGATCTATGGTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TTTTACTGTCTACAGTATAATAGTTACCCATTC ACTTTCGGCCCTGGGACCAAAGTGGATATCAA A | CAGGTGCAGCTACAACAGTGGGGCGCAGGACTG TTGAAGCCTTCGGGAGACCCTGTCCCTCACTTGCG CTGTCAATGGTGGGCCCTTCAGCGGTTGCTACTG GAGCTGGATCCGCCAGCCCCCAGGGAAGGGGCT GGAGTGGATTGGGGAAATCAATTATAGTGGAAG GACCAACTTCAACCCGTCCCTCAAGAGTCGAGTC ACCATATCAGTTGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
|  |  |  | SEQ ID NO: 24155 | SEQ ID NO: 28161 |

FIGURE 50

| | | | | |
|---|---|---|---|---|
| | | AA | DIQMTQSPSSLSASAGDRVTITCRASQDIRNYLG WYQQKPGKAPKRLIYGASSLQSGVPSRFSGSGS GTEFTLTISSLQPEDFATFYCLQYNSYPFTFGPGT KVDIK | QVQLQQWGAGLLKPSETLSLTCAVNGGPFSGCYW SWIRQPPGKGLEWIGEINYSGRTNFNPSLKSRVTISV DTSKNQFSLKLSSVTAADTAVYYCARDYGGMDV WGQGTTVTVSS |
| | | | SEQ ID NO: 24156 | SEQ ID NO: 28162 |
| IPS:468834 | 21-225_94G10 | NA | GAAATTGTGTTGACGCAGTCTCCAGGCACCCT GTCTTTGTCTCCAGGGGAGAGAGCCACCCTCT CCTGCAGGGCCAGTCAGAGTGTTAACAGCAAC TACTTAGCCTGGTACCGGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCATTCTCAT CATCAGCAGACTGGAGCCTGAGGATTTTGCAG TGTATTACTGTCAGCAGTATGAAAGCTCGCCG TGGACGTTCGGCCAAGGGACCAAGGTGGAAA TCAAA | CAGGTGCAGCTACAACAGTGGGGCGCAGGACTG TTGAAGCCTTCGGAGACCCTGTCCCTCACTTGCG CTGTCAATGGTGGGCCCTTCAGCGGTTGCTACTG GAGCGGGATCCGCCAGCCCCCAGGGAAGGGGCG GGAGTGGATTGGGGAAATCAATTATAGTGGAAG GACCAACTTCAACCCGTCCCTCAAGAGTCGAGTC ACCATATCAGTTGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
| | | | SEQ ID NO: 24157 | SEQ ID NO: 28163 |
| | | AA | EIVLTQSPGTLSLSPGERATLSCRASQSVNSNYLA WYRQKPGQAPRLLIYGASSRATGIPDRFSGSGSG TDFILIISRLEPEDFAVYYCQQYESSPWTFGQGTK VEIK | QVQLQQWGAGLLKPSETLSLTCAVNGGPFSGCYW SGIRQPPGKGREWIGEINYSGRTNFNPSLKSRVTISV DTSKNQFSLKLSSVTAADTAVYYCARDYGGMDV WGQGTTVTVSS |
| | | | SEQ ID NO: 24158 | SEQ ID NO: 28164 |

FIGURE 50

| iPS:468836 | 21-225_198E3 | NA | GACATCCAGATGACCCAGTCTCCATCCTCCCT GTCTGCATCTGTAGGAGACAGAGTCACCATCA CTTGCCGGGCAAGTCAGGGCATAAGAAAAGA TTTAGGCTGGTATCAGCAGAAACCAGGGAAA GCCCCTAAGCGCCTGATCTATGCTGCATCCAG TTTGCAAAGTGGGGTCCCATCAAGGTTCAGCG GCAGTGGATCTGGGACAGAATTCACTCTCACA ATCAGCAGCCTGCAGCCTGAAGATTTTGCAAC TTATGTCTGTCTACAACATTATCGTTACCCTTT CACTTTCGGCCCTGGGACCAAAGTGGATTTCA AA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGATGGAGGT TATAAAAACTATGCAGACTCCGTGAAGGGCCGA TTCACCATCTCCAGAGACAATTCCAAGAACACGC TGTATCTGCAAATGAATAGCCTGAGAGCTGAGGA CACGGCTGTGTATTACTGTGCGAGAGGTACCCAC GGGTACTACTACGGTGTGGACGTCTGGGGCCAA GGGACCACGGTCACCGTCTCCTCA |
| | | | SEQ ID NO: 24159 | SEQ ID NO: 28165 |
| | | AA | DIQMTQSPSSLSASVGDRVTITCRASQGIRKDLG WYQQKPGKAPKRLIYAASSLQSGVPSRFSGSGS GTEFTLTISSLQPEDFATYVCLQHYRYPFTFGPGT KVDFK | QVQLVESGGGVVQPGRSLRLSCAASGFTFSSYGMH WVRQAPGKGLEWVAVISYDGGYKNYADSVKGRF TISRDNSKNTLYLQMNSLRAEDTAVYYCARGTHG YYYGVDVWGQGTTVTVSS |
| | | | SEQ ID NO: 24160 | SEQ ID NO: 28166 |
| iPS:468838 | 21-225_80E12 | NA | GAAATTGTGTTGACGCAGTGTCCAGGCACCCT GTCTTTGTCTCCAGGGGAGAGAGCCACCGTCT CCTGCAGGGCCAGTCAGAGCGTTAACAGCAAC TACTTAGCCTGGTACCGGCAGAAACCTGACCA GGCTCCCAGGCTCCTCATCTATGGTGCATCCA GCAGGGCCACTGGCATCCCAGACAGGTTCAGT GGCAGTGGGTCTGGGACAGACTTCATTCTCAT CATCAGCAGACTGGAGCCTGAGGATTTTGCAG TGTATTACTGTCAGCAGTATGAAAGCTCGCCG TGGACGTTCGGCCAAGGGACCAAGGTGGAAA TCAAA | CAGGTGCAGCTACAACAGTGGGGCGCAGGACTG TTGAAGCCTTCGGGAGACCCTGTCCCTCACTTGCG CTGTCAATGGTGGGCCCTTCAGCGGTTGCTACTG GAGCTGGATCCGCCAGCCCCCAGGGAAGGGGCT GGAGTGGATTGGGGAAATCAATTATAGTGGAAG GACCAACTTCAACCCGTCCCTCAAGAGTCGAGTC ACCATATCAGTTGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
| | | | SEQ ID NO: 24161 | SEQ ID NO: 28167 |

FIGURE 50

| | | | | |
|---|---|---|---|---|
| | | AA | EIVLTQCPGTLSLSPGERATVSCRASQSVNSNYL AWYRQKPDQAPRLLIYGASSRATGIPDRFSGSGS GTDFILIISRLEPEDFAVYYCQQYESSPWTFGQGT KVEIK | QVQLQQWGAGLLKPSETLSLTCAVNGGPFSGCYW SWIRQPPGKGLEWIGEINYSGRTNFNPSLKSRVTISV DTSKNQFSLKLSSVTAADTAVYYCARDYGGMDV WGQGTTVTVSS |
| | | | SEQ ID NO: 24162 | SEQ ID NO: 28168 |
| iPS:468840 | 21-225_200H9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGATCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCTCTC ACTTTCGGCGGAGGGACCAAGGTGGAGATCA AA | CAGGTGCAGCTGCAGGAGTCGGGCCCAGGACTG GTGAAGCCTTCACAGACCCTGTCCCTCACCTGCA TTGTCTCTGGTGGCTCCATGAGGAGTGGTGGTGA CTACTGGAGCTGGATCCGCCAGCACCCAGGGAA GGGCCTGGAGTGGTTTGGGTACATCTATTACAGT GGGAGCACCTACTACAACCCGTCCCTCAAGAGTC GAGTTACCTTATCAGTAGACACGTCTAAGAACCA GTTCTCCCTGAAGCTGAGCTCTGTGACTGCCGCG GACACGGCCGTGTATTACTGTGCGAGAATGGACT ACAGTAACTACTACTACGGTATGGACGTCTGGGG CCAAGGGACCTCGGTCACCGTCTCCTCA |
| | | | SEQ ID NO: 24163 | SEQ ID NO: 28169 |
| | | AA | DIQMTQSPSSLSASVGDRVTITCRASQGIRNDLG WYQQKPGKAPKRLIYAASSLQSGIPSRFSGSGSG TEFTLTISSLQPEDFATYYCLQHNSYPLTFGGGT KVEIK | QVQLQESGPGLVKPSQTLSLTCIVSGGSMRSGGDY WSWIRQHPGKGLEWFGYIYYSGSTYYNPSLKSRVT LSVDTSKNQFSLKLSSVTAADTAVYYCARMDYSN YYYGMDVWGQGTSVTVSS |
| | | | SEQ ID NO: 24164 | SEQ ID NO: 28170 |

FIGURE 50

| iPS:468820 | 21-225_76E10 | NA | GAAATTGTGTTGACGCAGTCTCCAGGCACCCT GTCTTTGTCTCCAGGGGAGAGAGCCACCCTCT CCTGCAGGGCCAGTCAGAGTGTTAACAGCAAC TACTTAGCCTGGTACCGGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCATTCTCAT CATCAGCAGACTGGAGCCTGAGGATTTTGCAG TGTATTACTGTCAGCAGTATGAAAGCTCGCCG TGGACGTTCGGCCAAGGGACCAAGGTGGAAA TCAAA | CAGGTGCAGCTCCAACAGTGGGGCGCAGGACTG TTGAAGCCTTCGGAGACCCTGTCCCTCACTTGCG CTGTCAATGGTGGGCCCTTCAGCGGTTCCTACTG GAGCTGGATCCGCCAGCCCCCAGGGAAGGGGCT GGAGTGGATTGGGGAAATCAATTATAGTGGAAG GACCAACTTCAACCCGTCCCTCAAGAGTCGAGTC ACCATATCAGTTGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
| | | | SEQ ID NO: 24165 | SEQ ID NO: 28171 |
| | | AA | EIVLTQSPGTLSLSPGERATLSCRASQSVNSNYLA WYRQKPGQAPRLLIYGASSRATGIPDRFSGSGSG TDFILIISRLEPEDFAVYYCQQYESSPWTFGQGTK VEIK | QVQLQQWGAGLLKPSETLSLTCAVNGGPFSGSYW SWIRQPPGKGLEWIGEINYSGRTNFNPSLKSRVTISV DTSKNQFSLKLSSVTAADTAVYYCARDYGGMDV WGQGTTVTVSS |
| | | | SEQ ID NO: 24166 | SEQ ID NO: 28172 |
| iPS:468842 | 21-225_50H4 | NA | GACATCCAGATGACCCAGTCTCCATCCTCCCT GTCTGCATCTGTTGGAGACAGAGTCACCATCA CTTGCCGGGCAAGTCAGGGCATTAGAAATGAT TTAGGCTGGTATCAGCAGAAACCAGGGAAGG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTACAGCCTGAAGATTTTGCAACT TATTACTGTCTACAACATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCAGCTATATGGTATGATGGAAG TAATAAATACTATGCAGACTCCGTGAAGGGCCG ATTCACCATCTCCAGAGACAATTCCAAGAACACG CTGGATCTGCAAATGAACAGCCTGAGAGCCGAG GACACGGCTGTGTATTACTGTGCGAGAGAGTTGT ATAGCAGCAACTGGTACGACTACGGTATGGACG TCTGGGGCCAAGGGACCACGGTCACCGTCTCCTC A |
| | | | SEQ ID NO: 24167 | SEQ ID NO: 28173 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEIK | QVQLVESGGGVVQPGRSLRLSCAASGFTFSSYVMH WVRQAPGKGLEWVAAIWYDGSNKYYADSVKGRF TISRDNSKNTLDLQMNSLRAEDTAVYYCARELYSS NWYDYGMDVWGQGTTVTVSS |
| | | | SEQ ID NO: 24168 | SEQ ID NO: 28174 |
| IPS:468844 | 21-225_48E10 | NA | GACATCCAGATGACCCAGTCTCCATCCTCCCT GTCTGCATCTGTAGGAGACAGAGTCACCATCA CTTGCCGGGCAAGTCAGGGCATTAGAAGTGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGTATAATAGTTACCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAACAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAAGCCTCGAC CTCTGGGGCCAGGGAACCCTGGTCACCGTCTCCT CA |
| | | | SEQ ID NO: 24169 | SEQ ID NO: 28175 |
| | | AA | DIQMTQSPSSLSASVGDRVTITCRASQGIRSDLG WYQQKPGKAPKRLIYTASSLQSGVPSRFSGSGSG TEFTLTISSLQPEDFATYYCLQYNSYPFTFGPGTK VDIK | EVQLVESGGGLVKPGGSLRLSCAASGFTFSSYNMN WVRQAPGKGLEWVSSISGSSSYIYYADSVKGRFTIS RDNAKNSLYLQMNSLRAEDTAVYYCARSLDLWG QGTLVTVSS |
| | | | SEQ ID NO: 24170 | SEQ ID NO: 28176 |

FIGURE 50

| iPS:468846 | 21-225_53B10 | NA | GACATCCAGATGACCCAGTCTCCATCCTCCCTGTCTGCCTCTGCAGGAGACAGAGTCACCATCACTTGCCGGGCAAGTCAGGACATTAGAAATTATTTAGGCTGGTATCAGCAGAAACCAGGGAAAGCCCCTAAGCGACTGATCTATGGTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTTTTACTGTCTACAGTATAATAGTTACCCATTCACTTTCGGCCCTGGGACCAAAGTGGATATCAAA | GAGGTGCAGTTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGCAGTTACATATACTACGTAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTCAACTCTTTTGACTCCTGGGGCCAGGGAACCCTGGTCACCGTCTCCTCA |
|---|---|---|---|---|
| | | | SEQ ID NO: 24171 | SEQ ID NO: 28177 |
| | | AA | DIQMTQSPSSLSASAGDRVTITCRASQDIRNYLGWYQQKPGKAPKRLIYGASSLQSGVPSRFSGSGSGTEFTLTISSLQPEDFATFYCLQYNSYPFTFGPGTKVDIK | EVQLVESGGGLVKPGGSLRLSCAASGFTFSSYSMNWVRQAPGKGLEWVSSISGSSSYIYYVDSVKGRFTISRDNAKNSLYLQMNSLRAEDTAVYYCARVNSFDSWGQGTLVTVSS |
| | | | SEQ ID NO: 24172 | SEQ ID NO: 28178 |
| iPS:468848 | 21-225_54B1 | NA | GACATCCAGATGACCCAGTCTCCATCCTCCCTGTCTGCTTCTATTGGAGACAGAGTCACCATCACTTGCCGGGCAAGTCAGAACATTAGCAGCTATTTAAATTGGTATCAGCAGAAACCAGGGAAGGCCCCCAAGTTCCTGATCTATGCTGCATCCAGTTGCATAGTGGGGTCCCACCAAGGTTCAGTGGCAGTGGATCTGGGACAGATTTCACTCTCACCATCAGCAGTCTGCAACCTGAAGATTTTGCAATTTACTACTGTCAACAGAGTTACAGAACCCCTCTGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTCTTAGTGGTAGTGGTGGTAGCACATTCTACGCAGACTCCGTGAAGGGCCGGTTCACCATCTCCAGAGAGAATTCCAAGAACACGCTGTATCTGCAAATGAGCAGCCTGAGAGCCGAGGACACGGCCGTATATTACTGTGCCCGAAGAGGCCGTGAATATAGTGGCTACGATTACTTTGACTACTGGGGCCAGGGAACCCTGGTCACCGTCTCCTCA |
| | | | SEQ ID NO: 24173 | SEQ ID NO: 28179 |

FIGURE 50

| | | | | |
|---|---|---|---|---|
| | | AA | DIQMTQSPSSLSASIGDRVTITCRASQNISSYLNW YQQKPGKAPKFLIYAASSLHSGVPPRFSGSGSGT DFTLTISSLQPEDFAIYYCQQSYRTPLWTFGQGT KVEIK | EVQLLESGGGLVQPGGSLRLSCAASGFTFSSYAMS WVRQAPGKGLEWVSVLSGSGGSTFYADSVKGRFTI SRENSKNTLYLQMSSLRAEDTAVYYCARRGREYS GYDYFDYWGQGTLVTVSS |
| | | | SEQ ID NO: 24174 | SEQ ID NO: 28180 |
| iPS:468850 | 21-225_63F4 | NA | GACATCGTGATGACCCAATCTCCAGACTCCCT GGCTGTGTCTCTGGGCGAGAGGGCCACCATCA ACTGCAAGTCCAGCCAGAGTGTTTTATCCAGC TCCAACAATAACAACTACTTAGCTTGGTACCA GCAGAAACCAGGACAGCCTCCTAAGCTGCTCA TTTACTGGGCATCTACCCGGGAATCCGGGATC CCTGACCGATTCAGTGGCAGCGGGTCTGGGAC AGGTTTCACTCTCACCATCAGCAGCCTGCAGG CTGAGGATGTGGCAGTTTATTACTGTCAGCAA TATTATACTACTCCGTGCAGTTTTGGCCAGGG GACCAAGCTGGAGATCAAT | CAGGTGCAGCTGGTGCAATCTGGGGCTGAGGTG AAGAAGCCTGGGGCCTCAGTGAAGGTCTCCTGC AAGGCTTCTGGATACACCTTCACCAATTATGATG TCAACTGGGTGCGACAGGCCACTGGACAAGGGC TTGAGTGGATGGGATGGATGCACCCTAACAGTGG TAACACAGGCTATGCACAGAAGTTCCGGGGCAG AGTCACCATGACCAGGAACACCTCCCTAAGCAC AGTCTACATGGAGCTGAGCAGCCTGCGATCTGAG GACACGGCCGTGTATTACTGTGCCTATAGCAGTG GCTGGTACGTTTTTGACTACTGGGGCCAGGGAAC CCTGGTCACCGTCTCCTCA |
| | | | SEQ ID NO: 24175 | SEQ ID NO: 28181 |
| | | AA | DIVMTQSPDSLAVSLGERATINCKSSQSVLSSSN NNNYLAWYQQKPGQPPKLLIYWASTRESGIPDR FSGSGSGTGFTLTISSLQAEDVAVYYCQQYYTTP CSFGQGTKLEIN | QVQLVQSGAEVKKPGASVKVSCKASGYTFTNYDV NWVRQATGQGLEWMGWMHPNSGNTGYAQKFRG RVTMTRNTSLSTVYMELSSLRSEDTAVYYCAYSSG WYVFDYWGQGTLVTVSS |
| | | | SEQ ID NO: 24176 | SEQ ID NO: 28182 |

FIGURE 50

| iPS:468852 | 21-225_71F3 | NA | GACATCGTGATGACCCAATCTCCAGACTCCCT<br>GGCTGTGTCTCTGGGCGCGAGGGCCACCATCA<br>ACTGCAAGTCCAGCCAGAGTGTTTTATCCAAC<br>TCCAACAATAACAACTACTTAGCTTGGTACCA<br>GCAGAAACCAGGACAGCCTCCTAAGCTGCTCA<br>TTTACTGGGCATCTACCCGGGAATCCGGGATC<br>CCTGACCGATTCAGTGGCAGCGGGTCTGGGAC<br>AGATTTCACTCTCACCATCAACAGCCTGCAGG<br>CTGAAGATGTGGCAGTTTATTACTGTCAGCAA<br>TATTATACTACTCCGTGCAGTTTTGGCCAGGG<br>GACCAAGCTGGAGATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATG<br>TCAACTGGGTGCGACAGGCCACTGGACAAGGGC<br>TTGAGTGGATGGGATGGATGCACCCTAACAGTGG<br>TAACACAGGCTATGCACAGAAGTTCCAGGGCAG<br>AGTCACCATGACCAGGAACACCTCCATAAGCAC<br>AGCCTACATGGAGCTGAGCAGCCTGCGATCTGA<br>GGACACGGCCGTGTATTACTGTGCCTATAGCAGT<br>GGCTGGTACGTTTTTGACTCCTGGGGCCAGGGAA<br>CCCTGGTCACCGTCTCCTCA |
| | | | SEQ ID NO: 24177 | SEQ ID NO: 28183 |
| | | AA | DIVMTQSPDSLAVSLGARATINCKSSQSVLSNSN<br>NNNYLAWYQQKPGQPPKLLIYWASTRESGIPDR<br>FSGSGSGTDFTLTINSLQAEDVAVYYCQQYYTTP<br>CSFGQGTKLEIK | QVQLVQSGAEVKKPGASVKVSCKASGYTFTNYDV<br>NWVRQATGQGLEWMGWMHPNSGNTGYAQKFQG<br>RVTMTRNTSISTAYMELSSLRSEDTAVYYCAYSSG<br>WYVFDSWGQGTLVTVSS |
| | | | SEQ ID NO: 24178 | SEQ ID NO: 28184 |
| iPS:468854 | 21-225_72C4 | NA | GATGTTGTAATGACTCAGTCTCCGCTCTCCCTG<br>CCCGTCACCCTTGGACAGCCGGCCTCCATCTC<br>CTGCAGGTCTGGTCAAAGCCTCGTATACAGTG<br>ATGGAAACACCTACTTGAATTGGTTTCAGCAG<br>AGGCCAGGCCAATCTCCAAGGCGCCTAATTTA<br>TGAGGTTTCTAAGTGGGACTCTGGGGTCCCAG<br>ACAGATTCAGTGGCAGTGGGTCAGGCACTAAT<br>TTCACACTGAAAATCAGCAGGGTGGAGGCTGA<br>GGATGTTGGGGTCTTTTACTGCATGCAAGGTA<br>CACACTGGCCGCTCACTTTCGGCGGAGGGACC<br>AAGGTGGAGATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGTCACTGGACAAGGGC<br>TTGAGTGGATGGGATGGATGCACCCTAACAGTGG<br>TAACACAGGCTATGCACAGAAGTTCCAGGGCAG<br>AGTCACCATGACCAGGAACACCTCCATAAGCAC<br>AGCCTACATGGAACTGAACAGCCTGAGATCTGA<br>GGACACGGCCGTGTATTACTGTTCGCATAGCAGT<br>GGCTGGTACCTCTTTGACTACTGGGGCCAGGGAA<br>CCCTGGTCACCGTCTCCTCA |
| | | | SEQ ID NO: 24179 | SEQ ID NO: 28185 |

FIGURE 50

| | | | | |
|---|---|---|---|---|
| | | AA | DVVMTQSPLSLPVTLGQPASISCRSGQSLVYSDG NTYLNWFQQRPGQSPRRLIYEVSKWDSGVPDRF SGSGSGTNFTLKISRVEAEDVGVFYCMQGTHWP LTFGGGTKVEIK | QVQLVQSGAEVKKPGASVKVSCKASGYTFTNYDIN WVRQVTGQGLEWMGWMHPNSGNTGYAQKFQGR VTMTRNTSISTAYMELNSLRSEDTAVYYCSHSSGW YLFDYWGQGTLVTVSS |
| | | | SEQ ID NO: 24180 | SEQ ID NO: 28186 |
| iPS:468856 | 21-225_77C9 | NA | GATGTTGTGATGACTCAGTCTCCACTCTCCCTG CCCGTCACCCTTGGACAGCCGGCCTCCATCTC CTGCAGGTCTAGTCAAAGCCTCGTTTACAGTG TTGGAAACACCTCCTTGAGTTGGTTTCAGCAG AGGCCAGGCCAATCTCCAAGGCGCCTAATTTA TAAGGTTTCTAACTGGGACTCTGGGGTCCCAG ACAGATTCAGCGGCAGTGGGTCAGGCACTGAT TTCACACTGAAAATCAGCAGGGTGGAGGCTGA GGATGTTGGGGTTTATTACCGCATGCAAGGTA CACACTGGCCATTCACTTTCGGCCCTGGGACC AAAGTGGATATCAAA | CAGCTGCAGCTGCAGGAGTCGGGCCCAGGACTG GTGACGCCTTCGGAGACCCTGTCCCTCACCTGCA CTGTCTCTGGCGGCTCCATCAGCAGGAGTAGTTA CTACTGGGGCTGGATCCGCCAGCCCCCAGGGAA GGGGCTGGAGTGGATTGGGAGTATCTATTATAGT GGGAGCGCCTACTCCAACCCGTCCCTCAAGAGTC GAGTCACCATATCCGTAGACACGTCCAAGAACC AGTTCTCCCTGAAGCTGAGCTCTGTGACCGCCGC GGACACGGCTCTGTTTTACTGTGCGAGACTTGAC TCTAACTGGGGTCTTGACTACTGGGGCCAGGGAA CCCTGGTCACCGTCTCCTCA |
| | | | SEQ ID NO: 24181 | SEQ ID NO: 28187 |
| | | AA | DVVMTQSPLSLPVTLGQPASISCRSSQSLVYSVG NTSLSWFQQRPGQSPRRLIYKVSNWDSGVPDRF SGSGSGTDFTLKISRVEAEDVGVYYRMQGTHWP FTFGPGTKVDIK | QLQLQESGPGLVTPSETLSLTCTVSGGSISRSSYYW GWIRQPPGKGLEWIGSIYYSGSAYSNPSLKSRVTIS VDTSKNQFSLKLSSVTAADTALFYCARLDSNWGLD YWGQGTLVTVSS |
| | | | SEQ ID NO: 24182 | SEQ ID NO: 28188 |

FIGURE 50

| iPS:468858 | 21-225_148C9 | NA | GACATCCAGATGACCCAGTCTCCATCCTCCCT GTCTGCATCTGTAGGAGACAGAGTCACCATCA CTTGCCGGGCAAGTCGGGGCATTAGAGATGAT TTAGGCTGGTATCAGCAGAAACCAGGGAAAG CCCCTAAGCGCCTGATCTATGCTGCATCCAGT TTGCAGAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTATCCTCGG ACGTTCGGCCAAGGGACCAAGGTGGAAATCA AA | CAGGTGCAGCTGGTGGAGTCTGGGGGAGGCGTG GCCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGGAAGT AATAAGTACTATGCAGACTCCGTGAAGGGCCGA CTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAGCAGTGGCTGGTACGACTACGGTTTGGACGTC TGGGGCCAAGGGACCACGGTCACCGTCTCCTCA |
| | | | SEQ ID NO: 24183 | SEQ ID NO: 28189 |
| | | AA | DIQMTQSPSSLSASVGDRVTITCRASRGIRDDLG WYQQKPGKAPKRLIYAASSLQSGVPSRFSGSGS GTEFTLTISSLQPEDFATYYCLQHYSYPRTFGQG TKVEIK | QVQLVESGGGVAQPGRSLRLSCAASGFTFSDYGM HWVRQAPGKGLEWVAVIWYDGSNKYYADSVKGR LTISRDNSKNTLYLQMNSLRAEDTAVYYCARERYS SGWYDYGLDVWGQGTTVTVSS |
| | | | SEQ ID NO: 24184 | SEQ ID NO: 28190 |
| iPS:468860 | 21-225_224E7 | NA | GACATCCAGATGACCCAGTCTCCTTCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTACGCGCCTGATCTATGCTGCATCCAGTT TGCAAAGTGGGGTCCCATCGAGGTTCAGCGGC AGTGGATCTGGGACAGAATTCACTCTCACAAT CAGCAGCCTGCAGCCTGAAGATTTTGCAACTT ATTACTGTCTACAACATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGCCTCTCCTGTG CAGCGTCTGGATTCACCTTCAGTAGCTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACAGTA TAGCAGCAGCTGGTACGACTTCGGTCTGGACGTC TGGGGCCAAGGGACCACGGTCACCGTCTCCTCA |
| | | | SEQ ID NO: 24185 | SEQ ID NO: 28191 |

FIGURE 50

| | | | | |
|---|---|---|---|---|
| | | AA | DIQMTQSPSSLSASVGDRVTITCRASQGIRNDLG WYQQKPGKAPTRLIYAASSLQSGVPSRFSGSGSG TEFTLTISSLQPEDFATYYCLQHYSYPRTFGQGT KVEIK | QVQLVESGGGVVQPGRSLSLSCAASGFTFSSYVMH WVRQAPGKGLEWVAVIWYDGSNKYYADSVKGRF TISRDNSKNTLYLQMNSLRAEDTAVYYCAREQYSS SWYDFGLDVWGQGTTVTVSS |
| | | | SEQ ID NO: 24186 | SEQ ID NO: 28192 |
| iPS:468862 | 21-225_178H8 | NA | CAGTCTGCCCTGACTCAGTCTGCCTCCGTGTCG GGGTCTCCTGGACAGTCGATCACCATCTCCTG CACTGGAACCAGCAGTGACGTTGGTGGTTATA ATTTTGTCTCCTGGTACCAACAGCACCCAGGC AAAGTCCCCAAATTCATGATTTATGAGGTCAG TAATCGGCCCTCAGGGGTTCCTAATCGTTTTTC TGGCTCCAAGTCTGGCAACACGGCCTCCCTGA CCATCTCTGGCCTCCAGGCTGAGGACGAGGCT GATTATTACTGCAGCTCATATACAAGCAGCTA CACTTGGGTGTTCGGCGGAGGGACCAAACTGA CCGTCCTA | CAGGTGCAGCTGGTGCAGTCTGGGGGCTGAGGTG AGGACGCCTGGGGCCTCAGTGAAGGTCTCCTGCA AGGCTTCTGGATACACCTTCACCGACTATTATAT GCACTGGGTGCGACAGGCCCCTGGACAAGGGCT TGAGTGGATGGGATGGATCAACCCTAACAGAGG TGGCACAAACTATGCTCAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGAGGA GGATCGCAGTGGCTGGTACTACTACTACGGTATG GACGTCTGGGGCCAAGGGACCACGGTCACCGTC TCCTCA |
| | | | SEQ ID NO: 24187 | SEQ ID NO: 28193 |
| | | AA | QSALTQSASVSGSPGQSITISCTGTSSDVGGYNFV SWYQQHPGKVPKFMIYEVSNRPSGVPNRFSGSK SGNTASLTISGLQAEDEADYYCSSYTSSYTWVFG GGTKLTVL | QVQLVQSGAEVRTPGASVKVSCKASGYTFTDYYM HWVRQAPGQGLEWMGWINPNRGGTNYAQKFQGR VTMTRDTSISTAYMELSRLRSDDTAVYYCAREEDR SGWYYYYGMDVWGQGTTVTVSS |
| | | | SEQ ID NO: 24188 | SEQ ID NO: 28194 |

FIGURE 50

| iPS:468864 | 21-225_60D6 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTAAT ACTGTAAACTGGTACCAGCAGCTCCCAGGAAC GGCCCCCAAACTCCTCATCTATAGTAATAATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AATGGTCCGGTCGGCGGAGGGACCAAGCTGA CCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTTATTGGAAAG ATGATGAGCGCTACAGCCCATCTCTGAAGAGCA GGCTCACCATCACCAAGGACACTTCCAAAAACC AGGTGGTCCTTACAATGACCAACATGGACCCTGT GGACACAGCCACATATTACTGTGCACATGCAGTG GCTGTCTCCTTTGACTACTGGGGCCAGGGAACCC TGGTCACCGTCTCCTCA |
|---|---|---|---|---|
| | | | SEQ ID NO: 24189 | SEQ ID NO: 28195 |
| | | AA | QSVLTQPPSASGTPGQRVTISCSGSSSNIGSNTVN WYQQLPGTAPKLLIYSNNQRPSGVPDRFSGSKS GTSASLAISGLQSEDEADYYCAAWDDSLNGPVG GGTKLTVL | QITLKESGPTLVKPTQTLTLTCTFSGFSLSTSGVGVG WIRQPPGKALEWLALIYWKDDERYSPSLKSRLTITK DTSKNQVVLTMTNMDPVDTATYYCAHAVAVSFD YWGQGTLVTVSS |
| | | | SEQ ID NO: 24190 | SEQ ID NO: 28196 |
| iPS:468866 | 21-225_190C1 | NA | TCCTATGAGCTGACACAGCCACCCTCGGTGTC AGTGTCCCCAGGACAAACGGCCAGGATCACCT GCACTGGAGATGCAATGCCGAAAAAAATATGCT TATTGGGACCAGCAGAAGTCAGGCCAGGCCCC TGTGCTGGTCATCTCTGAGGACAGCAAGCGAC CCTCCGGGATCCCTGAGAGATTCTCTGGCTCC AGCTCAGGGACAATGGCCCCCTTGACTATCAG TGGGGCCCAGGTGGAGGATGAAACTGACTAC GACTGTAACTCAACAGACAGCAGTGGTAATCG GGTGTTCGGCGGAGGGACCAAGCTGACCGTCC TA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGAC TTGAGTGGATGGGATGGATCAACCCTTACAGTGG TGGCACAAACTATGCACAGAAGTTTCAGGGCAG GGTCACCATGACCAGGGACACGTCCATCAGCAC AGCCTACATGGAACTGAGCAGGCTGAGATCTGA CGACACGGCCGTGTATTACTGTGCGAGAGATAG AGCAGTGGCTGGAAACTACTTCTACTACGGTATG GACGTCTGGGGGCCAAGGGACCACGGTCACCGTC TCCTCA |
| | | | SEQ ID NO: 24191 | SEQ ID NO: 28197 |

FIGURE 50

| | | | | |
|---|---|---|---|---|
| | | AA | SYELTQPPSVSVSPGQTARITCTGDAMPKKYAY WDQQKSGQAPVLVISEDSKRPSGIPERFSGSSSG TMAPLTISGAQVEDETDYDCNSTDSSGNRVFGG GTKLTVL | QVQLVQSGAEVKKPGASVKVSCKASGYTFTGYYM HWVRQAPGQGLEWMGWINPYSGGTNYAQKFQGR VTMTRDTSISTAYMELSRLRSDDTAVYYCARDRAV AGNYFYYGMDVWGQGTTVTVSS |
| | | | SEQ ID NO: 24192 | SEQ ID NO: 28198 |
| iPS:468868 | 21-225_74A1 | NA | GACATCCAGATGACCCAGTCTCCATCCTCCCT GTCGGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTACGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATGATAGTTACCCTCTC ACTTTCGGCGGAGGGGCCAAGGTGGAGATCA AA | CAACTGCAGCTGCAGGAGTCGGGCCCGGGACTG GTGAAGCCTTCGGAGACCCTGTCCCTCACCTGCA CTGTCTCTGGTGGCTCCATCAGCGGTAGTAGTTA CTACTGGGGCTGGATCCGCCAGCCCCCGGGGAA GGGGCTGGAGTGGATTGGGAATATCTATTATAGT GGGAGCACCTACCACAACCCGTCCCTCAAGAGTC GAGTCACCATATCCGTGGACACGTCCAAGAACC AGTTCTCCCTGAAGCTGACCTCTGTGACCGCCGC AGACACGGCTGTGTTTTACTGTGCGAGACATGAT TTACTTTGGTCCCTTGACTTCTGGGGCCAGGGAA TTCTGGTCACCGTCTCCTCA |
| | | | SEQ ID NO: 24193 | SEQ ID NO: 28199 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHDSYPLTFGGG AKVEIK | QLQLQESGPGLVKPSETLSLTCTVSGGSISGSSYYW GWIRQPPGKGLEWIGNIYYSGSTYHNPSLKSRVTIS VDTSKNQFSLKLTSVTAADTAVFYCARHDLLWSLD FWGQGILVTVSS |
| | | | SEQ ID NO: 24194 | SEQ ID NO: 28200 |

FIGURE 50

| | | | | |
|---|---|---|---|---|
| iPS:468870 | 21-225_74A8 | NA | GACATCGTGATGACCCAGTCTCCAGACTCCCT GGCTGTGTCTCTGGGCGAGAGGGCCACCATCA ACTGCAAGTCCAGCCAGAGTGTTTTGTACAGC TCCAACAGTCACAACTACTTAGCTTGGTACCA GCAGAAACCAGGACAGCCTCCTAAACTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGAC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACTCGGCCGTATATTACTGTGCGTATAGTAGT GGCTGGTACAAATTTGACTACTGGAGCCAGGGA ACCCTGGTCACCGTCTCCTCA |
| | | | SEQ ID NO: 24195 | SEQ ID NO: 28201 |
| | | AA | DIVMTQSPDSLAVSLGERATINCKSSQSVLYSSN SHNYLAWYQQKPGQPPKLLIYWASTRESGVPDR FSGSGSGTDFTLTISSLQAEDVAVYYCQQYYSTP CSFGQGTKLEIK | QVQLVQSGAEVKKPGASVKVSCKASGYTFTNYDIN WVRQASGQGLEWMGWMHPNSGNTGYAQKFQGR VTMTRNTSISTAYMELSSLRSEDSAVYYCAYSSGW YKFDYWSQGTLVTVSS |
| | | | SEQ ID NO: 24196 | SEQ ID NO: 28202 |
| iPS:472730 | 21-225_14B1_LC1 | NA | GACATCCAGATGACCCAGTCTCCATCCTACCT GTCTGCATCTGTAGGAGACAGAGTCACCATCA CTTGCCGGGCAAGTCAGGACATTAGAGATAAT TTAGGCTGGTATCAGCAGAAACCAGGGAAAG CCCCTAAACGCCTGATCTATACTGCATACAGT TTGCAAAGTGGGGTCCCATCAAGGTTCAGCGG CAGTGGATCTGGGACAGAATTCACTCTCACAA TCAGCAGCCTGCAGCCTGAGGATTTTGCAACT TATTACTGTCTACAACATTATAATTACCCGCTC ACTTTCGGCGGAGGGACCAAGGTGGAGATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATACCAT GAACTGGGTCCGCCAGGCTCCAGGGAAGGGGCT GGAGTGGGTCTCATCCATAAGTGGTAGTAGTAGT TACTTATACTACCCAGACTCAGTGAAGGGCCGAT TCACCATCTCCAGAGACAACGCCAAGAACTCTCT GTATCTGCAAATGAACAGCCTGAGAGCCGAGGA CACGGCTGTGTATTACTGTGCGAGAGATAGAGGC AGCAGCTGGGGCCAGGGAACCCTGGTCACCGTC TCCTCA |
| | | | SEQ ID NO: 24197 | SEQ ID NO: 28203 |

FIGURE 50

| | | | | |
|---|---|---|---|---|
| | | AA | DIQMTQSPSYLSASVGDRVTITCRASQDIRDNLG WYQQKPGKAPKRLIYTAYSLQSGVPSRFSGSGS GTEFTLTISSLQPEDFATYYCLQHYNYPLTFGGG TKVEIK | EVQLVESGGGLVKPGGSLRLSCAASGFTFSSYTMN WVRQAPGKGLEWVSSISGSSSYLYYPDSVKGRFTIS RDNAKNSLYLQMNSLRAEDTAVYYCARDRGSSW GQGTLVTVSS |
| | | | SEQ ID NO: 24198 | SEQ ID NO: 28204 |
| IPS:472731 | 21-225_14B1_LC2 | NA | TCCTTTGAGCTGACTCAGCCACCCTCAGTGTCC GTGTCCCCAGGACAGACAGCCAGCATCACCTG CTCTGGAGATAAATTGGGGGATAAATATGCTT ACTGGTATCAGCAGAAGCCAGGCCAGTCCCCT GTGTTGGTCATCTATCAAGATAGGAAGCGCCC CTCAGGGATCCCTGAGCGATTCTCTGGCTCCA ACTCTGGGAACACAGCCACTCTGACCATCAGC GGGACCCAGGCTATGGATGAGGCTGACTATTA CTGTCAGGCGTGGGACAACAGCACTGTGGTGT TCGGCGGAGGGACCAAGCTGACCGTCCTA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATACCAT GAACTGGGTCCGCCAGGCTCCAGGGAAGGGGCT GGAGTGGGTCTCATCCATAAGTGGTAGTAGTAGT TACTTATACTACCCAGACTCAGTGAAGGGCCGAT TCACCATCTCCAGAGACAACGCCAAGAACTCTCT GTATCTGCAAATGAACAGCCTGAGAGCCGAGGA CACGGCTGTGTATTACTGTGCGAGAGATAGAGGC AGCAGCTGGGGCCAGGGAACCCTGGTCACCGTC TCCTCA |
| | | | SEQ ID NO: 24199 | SEQ ID NO: 28205 |
| | | AA | SFELTQPPSVSVSPGQTASITCSGDKLGDKYAYW YQQKPGQSPVLVIYQDRKRPSGIPERFSGSNSGN TATLTISGTQAMDEADYYCQAWDNSTVVFGGG TKLTVL | EVQLVESGGGLVKPGGSLRLSCAASGFTFSSYTMN WVRQAPGKGLEWVSSISGSSSYLYYPDSVKGRFTIS RDNAKNSLYLQMNSLRAEDTAVYYCARDRGSSW GQGTLVTVSS |
| | | | SEQ ID NO: 24200 | SEQ ID NO: 28206 |

FIGURE 50

| iPS:472732 | 21-225_2B10_LC1 | NA | GATGTTGTGATGACTCAGTCTCCACTCTCCCTG CCCGTCACCCTTGGACAGCCGGCCTCCATCTC CTGCAGGTCTAGTCAAAGCCTCGTATACAGTG ATGGAAACACCTTCTTGAATTGGTTTCAGCAG AGGCCAGGCCAATCTCCAAGGCGCCTAATTTA TAAGGTTTCTAACTGGGACTCTGGGGTCCCAG ACAGATTCAGCGGCAGTGGGTCAGGCACTGGT TTCACACTGAAAATCAGCAGGGTGGAGGCTGA GGATGTTGGGGTTTATTACTGCATACAAGGTA CGCACTGGCCTTTCCCCTTCGGCCAAGGGACA CGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCGGTTATATGGTATGATGGAAGT AATAAATACTATGCAGACTCCGTGAAGGTCCGAT TCACCGTCTCCAGAGACAATTCCAAGAACACGCT GTCTCTGCAAATGAACAGCCTGAGAGCCGAGGA CACGGCTGTGTATTACTGTGCGAGAGAGAGATAT ACCAGTGGCTGGTATGACTACGGTATGGACGTCT GGGGCCAAGGGACCACGGTCACCGTCTCCTCA |
|---|---|---|---|---|
| | | | SEQ ID NO: 24201 | SEQ ID NO: 28207 |
| | | AA | DVVMTQSPLSLPVTLGQPASISCRSSQSLVYSDG NTFLNWFQQRPGQSPRRLIYKVSNWDSGVPDRF SGSGSGTGFTLKISRVEAEDVGVYYCIQGTHWPF PFGQGTRLEIK | QVQLVESGGGVVQPGRSLRLSCAASGFTFSSYVMH WVRQAPGKGLEWVAVIWYDGSNKYYADSVKVRF TVSRDNSKNTLSLQMNSLRAEDTAVYYCARERYTS GWYDYGMDVWGQGTTVTVSS |
| | | | SEQ ID NO: 24202 | SEQ ID NO: 28208 |
| iPS:472733 | 21-225_2B10_LC2 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ATTTTGTCTCCTGGTACCAACAGCACCCAGAC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAGCTCATATACAAGCACCGG CACTGTGGTAATCGGCGGAGGGACCAAACTG ACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCGGTTATATGGTATGATGGAAGT AATAAATACTATGCAGACTCCGTGAAGGTCCGAT TCACCGTCTCCAGAGACAATTCCAAGAACACGCT GTCTCTGCAAATGAACAGCCTGAGAGCCGAGGA CACGGCTGTGTATTACTGTGCGAGAGAGAGATAT ACCAGTGGCTGGTATGACTACGGTATGGACGTCT GGGGCCAAGGGACCACGGTCACCGTCTCCTCA |
| | | | SEQ ID NO: 24203 | SEQ ID NO: 28209 |

FIGURE 50

| | | | | |
|---|---|---|---|---|
| | | AA | QSALTQPASVSGSPGQSITISCTGTSSDVGGYNFV SWYQQHPDKAPKLMIYEVSNRPSGVSNRFSGSK SGNTASLTISGLQAEDEADYYCSSYTSTGTVVIG GGTKLTVL | QVQLVESGGGVVQPGRSLRLSCAASGFTFSSYVMH WVRQAPGKGLEWVAVIWYDGSNKYYADSVKVRF TVSRDNSKNTLSLQMNSLRAEDTAVYYCARERYTS GWYDYGMDVWGQGTTVTVSS |
| | | | SEQ ID NO: 24204 | SEQ ID NO: 28210 |
| IPS:473253 | 21-225_7C3_LC1 | NA | GACATCCAGATGACCCAGTCTCCATCCTCCCT GTCTGCATCTGTAGGAGACAGAGTCACCATCA CTTGCCGGGCAAGTCAGGGCATTAGAAGTGAT TTAGGCTGGTATCAGCAGAATCCAGTCAAAGC CCCTAAGCGCCTGATCTATGCTGCATCCAGTTT GCAAAGTGGGGTCCCATCGAGGTTCAGCGGCA GTGGATCTGGGACAGAATTCACTCTCACAGTC AGCAGCCTGCAGCCTGAAGATTTTGCATTTTA TTACTGTCTACAGCATAATAGTTACCTCCCCAT CACCTTCGGCCAAGGGACACGACTGGAAATTA AA | CAGGTGCAGCTGGTACAGTCTGGGGGCTGAGGTG AAGAAGCCTGGGGCCTCAGTGAAGGTCTCCTGC AAGGCTTCTGGATACACCTTCAACGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGCCTGAGATCTGAC GACACGGCCGTGTATTCCTGTGCGAGAGATGGTA CCAGCTCGTTTGACTACTGGGGCCAGGGAACCCT GGTCACCGTCTCCTCA |
| | | | SEQ ID NO: 24205 | SEQ ID NO: 28211 |
| | | AA | DIQMTQSPSSLSASVGDRVTITCRASQGIRSDLG WYQQNPVKAPKRLIYAASSLQSGVPSRFSGSGS GTEFTLTVSSLQPEDFAFYYCLQHNSYLPITFGQ GTRLEIK | QVQLVQSGAEVKKPGASVKVSCKASGYTFNDYYL HWVRQAPGQGLEWMGWIHPNSGGTNYAQKFQGR VTMTRDTSISTAYMELSSLRSDDTAVYSCARDGTSS FDYWGQGTLVTVSS |
| | | | SEQ ID NO: 24206 | SEQ ID NO: 28212 |

FIGURE 50

| iPS:473254 | 21-225_7C3_LC2 | NA | GACATCCAGATGACCCAGTCTCCATCTTCCGT GTCTGCATCTCTAGGAGACAGAGTCACCATCA CTTGTCGGGCGAGTCAGGGTATTAGCAGCTGG TTAGCCTGGTATCAGCAGAAACAAGGGAAAG CCCCTAAGCTCCTGATCTTTGCTGCATCCAGGT TGCAAAGTGGGGCCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTACAGTCTGGGGGCTGAGGTG AAGAAGCCTGGGGCCTCAGTGAAGGTCTCCTGC AAGGCTTCTGGATACACCTTCAACGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGCCTGAGATCTGAC GACACGGCCGTGTATTCCTGTGCGAGAGATGGTA CCAGCTCGTTTGACTACTGGGGCCAGGGAACCCT GGTCACCGTCTCCTCA |
|  |  |  | SEQ ID NO: 24207 | SEQ ID NO: 28213 |
|  |  | AA | DIQMTQSPSSVSASLGDRVTITCRASQGISSWLA WYQQKQGKAPKLLIFAASRLQSGAPSRFSGSGS GTDFTLTISSLQPEDFATYYCQQANSFPFTFGPGT KVDIK | QVQLVQSGAEVKKPGASVKVSCKASGYTFNDYYL HWVRQAPGQGLEWMGWIHPNSGGTNYAQKFQGR VTMTRDTSISTAYMELSSLRSDDTAVYSCARDGTSS FDYWGQGTLVTVSS |
|  |  |  | SEQ ID NO: 24208 | SEQ ID NO: 28214 |
| iPS:473255 | 21-225_9F12_LC1 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGCTGCATCCAGA TTGCAAAGTGGGGTCCCATCAAGGTTCAGCGG CAGTGGATCTGGGACAGATTTCACTCTCACCA TCAGCGGCCTGCTGCCTGAAGATTTTGCAATT TATTATTGTCAACAGGCTAACAGTTTCCCATTC ACTTTCGGCCCTGGGACCAAAGTGGATTTCAA A | CAGGTGCAGCTGGTACAGTCTGGGGGCTGAGGTG AAGAAGCCTGGGGCCTCAGTGAAGGTCTCCTGC AAGGCTTCTGGATACACCTTCGCCGACTACTATT TGCACTGGGTGCGACAGGCCCCTGGACAAGGTCT TGAGTGGATGGGATGGATCCACCCTAACAGTGGT GGCACAAACTTTGCACAGAAGTTTCAGGGCAGG GTCACCATGACCAGGGACACGTCCATCAGCACA GCCTACCTGGAACTGAGCAGTCTGAGATCTGACG ACACGGCCTTCTATTACTGTGCGAGAGATGGTAC CAGCTCGTTTGACTACTGGGGCCAGGGAACCCTG GTCACCGTCTCCTCA |
|  |  |  | SEQ ID NO: 24209 | SEQ ID NO: 28215 |

FIGURE 50

| | | | | |
|---|---|---|---|---|
| | | AA | DIQMTQSPSSVSASVGDRVTITCRASQGISRWLA WYQQKPGKAPKLLIYAASRLQSGVPSRFSGSGS GTDFTLTISGLLPEDFAIYYCQQANSFPFTFGPGT KVDFK | QVQLVQSGAEVKKPGASVKVSCKASGYTFADYYL HWVRQAPGQGLEWMGWIHPNSGGTNFAQKFQGR VTMTRDTSISTAYLELSSLRSDDTAFYYCARDGTSS FDYWGQGTLVTVSS |
| | | | SEQ ID NO: 24210 | SEQ ID NO: 28216 |
| IPS:473256 | 21-225_9F12_LC2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGTCAAAGC CCCTAAGCGCCTTATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATAATAGTTACCTCCCCAT CACCTTCGGCCAAGGGACACGACTGGAGATTA AA | CAGGTGCAGCTGGTACAGTCTGGGGCTGAGGTG AAGAAGCCTGGGGCCTCAGTGAAGGTCTCCTGC AAGGCTTCTGGATACACCTTCGCCGACTACTATT TGCACTGGGTGCGACAGGCCCCTGGACAAGGTCT TGAGTGGATGGGATGGATCCACCCTAACAGTGGT GGCACAAACTTTGCACAGAAGTTTCAGGGCAGG GTCACCATGACCAGGGACACGTCCATCAGCACA GCCTACCTGGAACTGAGCAGTCTGAGATCTGACG ACACGGCCTTCTATTACTGTGCGAGAGATGGTAC CAGCTCGTTTGACTACTGGGGGCCAGGGAACCCTG GTCACCGTCTCCTCA |
| | | | SEQ ID NO: 24211 | SEQ ID NO: 28217 |
| | | AA | DIQMTQSPSSLSASVGDRVTITCRASQGIRNDLG WYQQKPVKAPKRLIYAASSLQSGVPSRFSGSGS GTEFTLTISSLQPEDFATYYCLQHNSYLPITFGQG TRLEIK | QVQLVQSGAEVKKPGASVKVSCKASGYTFADYYL HWVRQAPGQGLEWMGWIHPNSGGTNFAQKFQGR VTMTRDTSISTAYLELSSLRSDDTAFYYCARDGTSS FDYWGQGTLVTVSS |
| | | | SEQ ID NO: 24212 | SEQ ID NO: 28218 |

FIGURE 50

| iPS:472742 | 21-225_30D9_LC2 | NA | TCCTATGAGCTGACTCAGCCACCCTCAGTGTC CGTGTCCCCAGGACAGACAGCCAGCATCACCT GCTCTGGAGATAAATTGGGGGATAAATATGTT TACTGGTTTCAGCAGAAGCCAGGCCAGTCCCC TGTGCTAGTCATCTATCAAGATAGGAAGCGGC CCTCAGGGATCCCTGAGCGATTCTCTGGCTCC AACTCTGGGAACACAGCCACTCTGACCATCAG CGGGACGCAGGCTCTGGATGAGGCTGACTATT ACTGTCAGGCGTGGGACAACAGCACTGCGGTA TTCGGCGGAGGGACCAAGCTGACCGTCCTA | CAGGTGAAACTGGTGCAGTCTGGGGCTGAGGTG GAGAAGCCTGGGGCCTCAGTGAAGGTCTCCTGC AAGGCTTCAGGATACACCTTCACCGGCTACTATC TGCACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGTGTAT TACTATGGTTCGGGGAGTTATTATAACGAGTTTG ACAACTGGGGCCAGGGAACCCTGGTCACCGTCTC CTCA |
| | | | SEQ ID NO: 24213 | SEQ ID NO: 28219 |
| | | AA | SYELTQPPSVSVSPGQTASITCSGDKLGDKYVY WFQQKPGQSPVLVIYQDRKRPSGIPERFSGSNSG NTATLTISGTQALDEADYYCQAWDNSTAVFGG GTKLTVL | QVKLVQSGAEVEKPGASVKVSCKASGYTFTGYYL HWVRQAPGQGLEWMGWINPNSGGTNYAQKFQGR VTMTRDTSISTAYMELSRLRSDDTAVYYCARVYYY GSGSYYNEFDNWGQGTLVTVSS |
| | | | SEQ ID NO: 24214 | SEQ ID NO: 28220 |
| iPS:472741 | 21-225_30D9_LC1 | NA | GATGTCGTGATGACTCAGTCTCCACTCTCCCTG CCCGTCACCCTTGGACAGCCGGCCTCCATCTC CTGCAGGTCTAGTCAAAGCCTCGTATCCAGTG ATGGAAACACCTTCTTGAATTGGTTTCAGCAG AGGCCAGGCCAATCTCCAAGGCGCCTAATTTA TAAGGTTTCTAACTGGGACTCTGGGGTCCCAG ACAGATTCAGCGGCAGTGGGTCAGGCACTGAT TTCACACTGAAAATCAGCAGGTTGGAGGCTGA GGATGTTGGGGTTTATTACTGCTTGCAAGGTA CACACTGGCCTCTCACCTTCGGCCAAGGGACA CGACTGGAGATTAAA | CAGGTGAAACTGGTGCAGTCTGGGGCTGAGGTG GAGAAGCCTGGGGCCTCAGTGAAGGTCTCCTGC AAGGCTTCAGGATACACCTTCACCGGCTACTATC TGCACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGTGTAT TACTATGGTTCGGGGAGTTATTATAACGAGTTTG ACAACTGGGGCCAGGGAACCCTGGTCACCGTCTC CTCA |
| | | | SEQ ID NO: 24215 | SEQ ID NO: 28221 |

FIGURE 50

| | | | | |
|---|---|---|---|---|
| | | AA | DVVMTQSPLSLPVTLGQPASISCRSSQSLVSSDG NTFLNWFQQRPGQSPRRLIYKVSNWDSGVPDRF SGSGSGTDFTLKISRLEAEDVGVYYCLQGTHWP LTFGQGTRLEIK | QVKLVQSGAEVEKPGASVKVSCKASGYTFTGYYL HWVRQAPGQGLEWMGWINPNSGGTNYAQKFQGR VTMTRDTSISTAYMELSRLRSDDTAVYYCARVYYY GSGSYYNEFDNWGQGTLVTVSS |
| | | | SEQ ID NO: 24216 | SEQ ID NO: 28222 |
| iPS:472743 | 21-225_68G6 | NA | TCCTATGAGGTGACTCAGCCACCCTCAGTGTC CGTGTCCCCAGGACAGACAGCCAGCATCACCT GCTCTGGAGATAAATTGGGGGATAAATATACT TACTGGTATCAGCAGAAGGCAGGCCAGTCCCC TTTCCTGGTCATCTATCAAGATAGGAAGCGGC CCTCAGGGATCCCTGACCGATTCTCTGGCTCC AACTCTGGGAACACAGCCACTCTGACCATCAG CGGGACCCAGGCTATGGATGCGGCTGACTTTT ACTGTCAGGCGTGGGACAATAGTACTGCGGTA TTCGGCGGAGGGACCAAGCTGACCGTCCTA | CAGGTGCAGTTGGTGCAGTTTGGGGGGTGAGGTGA AGAAGCCTGGGTCCTCAGTGAAGGTNTCCTGCAA GGCTTCAGGATACACCTTCACCGGYTACTATATG CACTGGGTGCGACAGGCCCCTGGACAAGGGCTT GAGTGGATGGGATCGATCTACCGTAACAGTGGTG GCACAAATTATGCACAGAAGTTTCAGGGCAGGG TCACCATGACCAGGGACAAGTCCATCAGCACCG CCTACATGGAGAAGAGCAGGATCAGATCTGATG ACACGGCCGTGTATTACTGTGCGAGAGCCTTTTA CTATGGTTCGGGGGACTTATTATAACGAATTTGAC TACTGGGGCCAGGGAACCCTGGTCACCGTCTCCT CA |
| | | | SEQ ID NO: 24217 | SEQ ID NO: 28223 |
| | | AA | SYEVTQPPSVSVSPGQTASITCSGDKLGDKYTY WYQQKAGQSPFLVIYQDRKRPSGIPDRFSGSNSG NTATLTISGTQAMDAADFYCQAWDNSTAVFGG GTKLTVL | QVQLVQFGGGEVKKPGSSVKVSCKASGYTFTGYYM HWVRQAPGQGLEWMGSIYRNSGGTNYAQKFQGR VTMTRDKSISTAYMEKSRIRSDDTAVYYCARAFYY GSGTYYNEFDYWGQGTLVTVSS |
| | | | SEQ ID NO: 24218 | SEQ ID NO: 28224 |

FIGURE 50

| | | | | |
|---|---|---|---|---|
| iPS:392573 | 21-225_15G2 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAGCACCCAGGC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCACCTCATATACAAGCACCAG CACTGTGGTCTTCGGCGGAGGGACCAAGCTGA CCGTCCTA | CAGATCACCTTGAAGGAGTCTGGTCCTACGCTGG TGAAACCCACACAGACCCTCACGCTGACCTGCAC CTTCTCTGGATTCTCACTCAGCACTAGTGGAGTG GGTGTGGGCTGGATCCGTCAGCCCCCAGGAAAG GCCCTGGAGTGGCTTGCACTCATTTATTGGAATG ATGATAAGCGCTACAGCCCATCTCTGAAGAGCA GGCTCACCATCACCAAGGACACCTCCAAAAACC AGGTGGTCCTTACAATGACCAACATGGACCCTGT GGACACAGCCACATATTACTGTGCAGACACCGGT GTCAGCTGCTGCTATTTTCACTATTGGGGCCAGG GAACCCTGGTCACCGTCTCCTCA |
| | | | SEQ ID NO: 24219 | SEQ ID NO: 28225 |
| | | AA | QSALTQPASVSGSPGQSITISCTGTSSDVGGYNY VSWYQQHPGKAPKLMIYEVSNRPSGVSNRFSGS KSGNTASLTISGLQAEDEADYYCTSYTSTSTVVF GGGTKLTVL | QITLKESGPTLVKPTQTLTLTCTFSGFSLSTSGVGVG WIRQPPGKALEWLALIYWNDDKRYSPSLKSRLTIT KDTSKNQVVLTMTNMDPVDTATYYCADTGVSCC YFHYWGQGTLVTVSS |
| | | | SEQ ID NO: 24220 | SEQ ID NO: 28226 |
| iPS:392583 | 21-225_10B10 | NA | TCCTATGAGCTGACTCAGCCACCCTCAGTGTC CGTGTCCCCAGGACAGACAGCCAGCATCACCT GCTCTGGAGATAAAATTGGGGGAATAAATATGCT TGGTGGTATCAGCAGAAGCCAGGCCAGTCCCC TGTGCTGGTCATCTATCAAGATAGGAAGCGGC CCTCAGGGATCCCTGAGCGATTCTCTGGCTCC AACTCTGGGAACACAGCCACTCTGACCATCAG CGGGGACCCAGGCTATGGATGAGGCTGACTATT ACTGTCAGGCGTGGGACAACAGCACTGTGGTT TTCGGCGGAGGGACCAAACTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTGGTGGAGTG GGTGTGGGCTGGATCCGTCAGCCCCCAGGAAAG GCCCTGGAGTGGCTTGTATTCATTTATTGGAGTG ATGATAAGCGCTACAGCCCATCTCTGAAGAGCA GGCTCTCCATCACCAAGGACACCTCCAAAAACCA GGTGGTCCTTACAATGACCAACATGGACCCTGTG GACACAGCCACATATTACTGTGCACGTATAGCAG CAGTTGCCTTTGACTACTGGGGCCAGGGAACCCT GGTCACCGTCTCCTCA |
| | | | SEQ ID NO: 24221 | SEQ ID NO: 28227 |

1433

FIGURE 50

| | | | | | |
|---|---|---|---|---|---|
| | | AA | SYELTQPPSVSVSPGQTASITCSGDKLGNKYAW WYQQKPGQSPVLVIYQDRKRPSGIPERFSGSNSG NTATLTISGTQAMDEADYYCQAWDNSTVVFGG GTKLTVL | QITLKESGPTLVKPTQTLTLTCTFSGFSLSTGGVGVG WIRQPPGKALEWLVFIYWSDDKRYSPSLKSRLSITK DTSKNQVVLTMTNMDPVDTATYYCARIAAVAFDY WGQGTLVTVSS | |
| | | | SEQ ID NO: 24222 | SEQ ID NO: 28228 | |
| iPS:392585 | 21-225_14H11 | NA | ACCTATGAGCTGACTCAGCCATCCTCAGTGTC CGTGTCCCCAGGACAGACAGCCAGCATCACCT GCTCTGGAGATAAATTGGGGGAAAAATATGTT TGCTGGTATCAGCAGAAGCCAGGCCAGTCCCC TGTGCTGGTCATATATCAAGATACCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTATATTC GGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGCC AGGGTTCTGGATACACCTTCACCGGCCACTATAT GTGCTGGGTGCGACAGGCCCCTGGACAAGGGCT TGAGTGGATGGGATGGATCAACCCTAATAGTGGT GGCACAAACTATGCACAGAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGGCTGGATATT GTAGTAGTTCCAGCTGCTATTTGCAACCGGGTTA TTACGGTATGGACGTCTGGGGGCCAAGGGACCAC GGTCACCGTCTCCTCA | |
| | | | SEQ ID NO: 24223 | SEQ ID NO: 28229 | |
| | | AA | TYELTQPSSVSVSPGQTASITCSGDKLGEKYVCW YQQKPGQSPVLVIYQDTKRPSGIPERFSGSNSGN TATLTISGTQAMDEADYYCQAWDSSTIFGGGTK LTVL | QVQLVQSGAEVKKPGASVKVSCQGSGYTFTGHYM CWVRQAPGQGLEWMGWINPNSGGTNYAQKFQGR VTMTRDTSISTAYMELSRLRSDDTAVYYCAAGYCS SSSCYLQPGYYGMDVWGQGTTVTVSS | |
| | | | SEQ ID NO: 24224 | SEQ ID NO: 28230 | |

FIGURE 50

| iPS:392587 | 21-225_18G5 | NA | TCCTATGAGCTGACTCAGCCACCCTCAGTGTC CGTGTCCCCAGGACAGACAGCCAGCATCACCT GCTCTGGAGAGAAATTGGGGGATAAATATGTT TGTTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGAACAGCAGCAATGTGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGCCTTTCACTCATTTATTGGAATG ATGATAAGGTCTACAGCCCATCTCTGAAGAGCAG GCTCACCATCACCAAGTACACCTCCAAAAACCAG GTGGTCCTTACAATGACCAACATGGACCCTGTGG ACACAGCCACATATTACTGTGCACACAGGGGAC AGCAGCTGGCCCTCGACTACTGGGGCCAGGGAA CCCTGGTCACCGTCTCCTCA |
| | | | SEQ ID NO: 24225 | SEQ ID NO: 28231 |
| | | AA | SYELTQPPSVSVSPGQTASITCSGEKLGDKYVCW YQQKPGQSPVLVIYQDSKRPSGIPERFSGSNSGN TATLTISGTQAMDEADYYCQAWNSSNVVFGGG TKLTVL | QITLKESGPTLVKPTQTLTLTCTFSGFSLSTSGVGVG WIRQPPGKALECLSLIYWNDDKVYSPSLKSRLTITK YTSKNQVVLTMTNMDPVDTATYYCAHRGQQLAL DYWGQGTLVTVSS |
| | | | SEQ ID NO: 24226 | SEQ ID NO: 28232 |
| iPS:392589 | 21-225_27H2 | NA | TCCTATGAGCTGACTCAGCCACCCTCAGTGTC CGTATCCCCAGGACAGACAGCCAGCATCACCT GCTCTGGAGATAAATTGGGGGATAAATATGCT TCCTGGTATCAGCAGAAGCCAGGCCAGTCCCC TGTTTTGGTCATCTATCAAGATGGCAAGCGGC CCTCAGGGATCCCTGAGCGATTCTCTGGCTCC AACTCTGGGAACACAGCCACTCTGACCCTCAG CGGGACCCAGGCTATGGATGAGGCTGACTATT ACTGTCAGGCGTGGGACAGCAGCACTTATGTG GTATTCGGCGGAGGGACCAAGCTGACCGTCCT A | CAGGTGCAGTTGGTGGAGTCTGGGGGGAGGCGTG GTCCAGCCTGGGAGGTCCCTGAGACTCTCCTGTG CAGCGTCTGGATTCACCTTCAGTGGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTTTCTGCAAATGAACAGCCTGAGAGCCGAGG ACACGGCTGTGTATTACTGTGCGAGAGATAGGGT ATATTGTAGTAGTACCAGCTGCTCCCCTTACTAC TACTACTACGGTATGGACGTCTGGGGCCAAGGG ACCACGGTCACCGTCTCCTCA |

FIGURE 50

| | | | SEQ ID NO: 24227 | SEQ ID NO: 28233 |
|---|---|---|---|---|
| | | AA | SYELTQPPSVSVSPGQTASITCSGDKLGDKYASW YQQKPGQSPVLVIYQDGKRPSGIPERFSGSNSGN TATLTLSGTQAMDEADYYCQAWDSSTYVVFGG GTKLTVL | QVQLVESGGGVVQPGRSLRLSCAASGFTFSGYGM HWVRQAPGKGLEWVAVIWYDGSNKYYADSVKGR FTISRDNSKNTLFLQMNSLRAEDTAVYYCARDRVY CSSTSCSPYYYYYGMDVWGQGTTVTVSS |
| | | | SEQ ID NO: 24228 | SEQ ID NO: 28234 |
| iPS:392593 | 21-225_3E10 | NA | TCCTATGAGCTGACTCAGCCACACTCAGTGTC AGTGGCCACAGCACAGATGGCCAGGATCACCT GTGGGGGAAACAACATTGGAAGTAAAGCTGT GCACTGGTACCAGCAAAAGCCAGGCCAGGAC CCTGTGCTGGTCATCTATAGCGATAGCAACCG GCCCTCAGGGATCCCTGAGCGATTCTCTGGCT CCAACCCAGGGAACACCGCCACCCTAACCATC AGCAGGATCGAGGCTGGGGATGAGGCTGACT ATTACTGTCAGGTGTGGGACAGTAGTAGTGAT CATGTGGTATTCGGCGGAGGGACCAAGCTGAC CGTCCTA | CAGATCACCTTGAAGGAGTCTGGTCCTACGCTGG TGAAACCCACACAGACCCTCACGCTGACCTGCAC CTTCTCTGGGTTCTCACTCAATACTGGTGGAGTG GGTGTGGGCTGGATCCGTCAGCCCCCAGGAAAG GCCCTGGAGTGGCTTGCACTCATTTATTGGAATG ATGATAAGCGCCACAGCCCATCTCTGAAGAGCA GGCTCACCATCACCAAAGACACCTCCAAAAACC AGGTGGTCCTTACAATGACCCACATGGCCCCTGT GGACACAGCCACATATTACTGTGCACACCTTATA GAAGTGGCCTTTGACTATTGGGGCCAGGGAACCC TGGTCACCGTCTCCTCA |
| | | | SEQ ID NO: 24229 | SEQ ID NO: 28235 |
| | | AA | SYELTQPHSVSVATAQMARITCGGNNIGSKAVH WYQQKPGQDPVLVIYSDSNRPSGIPERFSGSNPG NTATLTISRIEAGDEADYYCQVWDSSSDHVVFG GGTKLTVL | QITLKESGPTLVKPTQTLTLTCTFSGFSLNTGGVGV GWIRQPPGKALEWLALIYWNDDKRHSPSLKSRLTI TKDTSKNQVVLTMTHMAPVDTATYYCAHLIEVAF DYWGQGTLVTVSS |
| | | | SEQ ID NO: 24230 | SEQ ID NO: 28236 |

FIGURE 50

| | | | | |
|---|---|---|---|---|
| iPS:392596 | 21-225_12D8 | NA | CTGCCTGTGCTGACTCAGCCCCCGTCTGCATCT GCCTTGCTGGGAGCCTCGATCAAGCTCACCTG CACCCTAAGCAGTGAGCACAGCACCTACACCA TCGAATGGTATCAACAGAGACCAGGGAGGTC CCCCCAGTATATAATGAAGGTTAAGAGTGATG GCAGCCACAGCAAGGGGGACGGGATCCCCGA TCGCTTCATGGGCTCCAGTTCTGGGGCTGACC GCTACCTCACCTTCTCCAACCTCCAGTCTGACG ATGAGGATGAGTATCACTGTGGAGAGAGCCA CACGATTGATGGCCAAGTCGGTGTGGTATTCG GCGGAGGGACCAAGCTGACCGTCCTA<br><br>SEQ ID NO: 24231 | GAGGTGCAGTTGTTGGAATCTGGGGGAGGCTTGG TACAGCCTGGGGGGGTCCCTGAGACTCTCCTGTGC AGCCTCTGGATTCACCTTTAGCAGCTATGTCATG AGCTGGGTCCGCCAGGCTCCAGGGAAGGGGCTG GAGTGGGTCTCAACTATTAGTGTTGGTGGTGGTA GCACATACTACGCAGACTCCGTGAAGGGCCGGTT CACCATCTCCAGAGACAATTCCAAGACCACGCTG TATCTGCAAATGAACAGCCTGAGAGCCGAGGAC ACGGCCGTATATTACTGTGCGAAATGGGGACGTG GATACAGCTATGAATACTACTACGGTATGGACGT CTGGGGCCAAGGGACCACGGTCACCGTCTCCTCA<br><br>SEQ ID NO: 28237 |
| | | AA | LPVLTQPPSASALLGASIKLTCTLSSEHSTYTIEW YQQRPGRSPQYIMKVKSDGSHSKGDGIPDRFMG SSSGADRYLTFSNLQSDDEDEYHCGESHTIDGQV GVVFGGGTKLTVL<br><br>SEQ ID NO: 24232 | EVQLLESGGGLVQPGGSLRLSCAASGFTFSSYVMS WVRQAPGKGLEWVSTISVGGGSTYYADSVKGRFTI SRDNSKTTLYLQMNSLRAEDTAVYYCAKWGRGYS YEYYYGMDVWGQGTTVTVSS<br><br>SEQ ID NO: 28238 |
| iPS:392598 | 21-225_18E10 | NA | TCCTATGAACTGACGCAGCCACCCTCAGTGTC CGTGTCCCCAGGACAGACAGCCAGCATCACCT GCTCTGGAGATAGATTGGGGGGATAAATATGCT TGGTGGTATCAGCAGAAGCCAGGCCAGTCCCC TGTGCTGGTCATATATCAAGATCGCAAGCGGC CCTCAGGGATCCCTGAGCGATTCTCTGGCTCC AATTCAGGGAACACAGCCACTCTGACCATCAG CGGGACCCAGGCTATGGATGAGGCTGACTATT ACTGTCAGGCGTGGGACAGCAGCACAGTGGT ATTCGGCGGAGGGACCAAGCTGACCGTCCTA<br><br>SEQ ID NO: 24233 | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGGTGGATCAACCCTAACAGTGG TGGCACAAACTATGCACAGAAGTTTCAGGGCAG GGTCACCATGACCAGGGACTCGTCCATCAACACA GCCTACATGGAGCTGAGCAGGCTGAGATCGAC GACACGGCCGTGTATTACTGTGCGAGGTCGTACT ACTATGGTTCGGGGAGTTATTATAACGAGTTTGA CTACTGGGGCCAGGGAACCCTGGTCACCGTCTCC TCA<br><br>SEQ ID NO: 28239 |

FIGURE 50

| | | | | |
|---|---|---|---|---|
| | | AA | SYELTQPPSVSVSPGQTASITCSGDRLGDKYAW WYQQKPGQSPVLVIYQDRKRPSGIPERFSGSNSG NTATLTISGTQAMDEADYYCQAWDSSTVVFGG GTKLTVL | QVQLVQSGAEVKKPGASVKVSCKASGYTFTGYYM HWVRQAPGQGLEWMGWINPNSGGTNYAQKFQGR VTMTRDSSINTAYMELSRLRSDDTAVYYCARSYYY GSGSYYNEFDYWGQGTLVTVSS |
| | | | SEQ ID NO: 24234 | SEQ ID NO: 28240 |
| IPS:392618 | 21-225_16F10 | NA | GATATTGTGATGACCCAGACTCCACTCTCTCT GTCCGTCATTCCTGGACAGCCGGCCTCCATCT CCTGCAAGTCTAGTCAGAGCCTCCTGCATAGT GATGGAAAGACCCATTTGAATTGGTACCTGCA GAAGCCAGGCCAGCCTCCACAGCTCCTGATCT ATGAAGTTTCCTACCGGTTCTCTGGAGTGCCA GATAGGTTCAGTGGCAGCGGGTCAGGGACAG TTTTCACACTGGAGATCAGCCGGGTGGAGGCT GCGGATGTTGGGGTTTATTACTGCTTTCAAAG TATACAGCTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTCATATGGTATGATGGAAA TAATAAATACTATGTAGACTCCGTGAAGGGCCGA TTCACCATCTCCAGAGACAATTCCAAGAATACGC TGTATCTGCAAATGAACAGCCTGAGAGCCGAGG ACACGGCTGTGTATTACTGTGCGAGAGAGCTTGC CTGGTACGAGGACTACTGGGGCCAGGGAACCCT GGTCACCGTCTCCTCA |
| | | | SEQ ID NO: 24235 | SEQ ID NO: 28241 |
| | | AA | DIVMTQTPLSLSVIPGQPASISCKSSQSLLHSDGK THLNWYLQKPGQPPQLLIYEVSYRFSGVPDRFSG SGSGTVFTLEISRVEAADVGVYYCFQSIQLPLTF GGGTKVEIK | QVQLVESGGGVVQPGRSLRLSCAASGFTFSDYGM HWVRQAPGKGLEWVAVIWYDGNNKYYVDSVKG RFTISRDNSKNTLYLQMNSLRAEDTAVYYCARELA WYEDYWGQGTLVTVSS |
| | | | SEQ ID NO: 24236 | SEQ ID NO: 28242 |

FIGURE 50

| iPS:392620 | 21-225_17H5 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCAATGCTGCATCCAGTTT ACAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATCATAGTTACCCATTCA CTTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTACT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTGGCTTCA TTTGACTACTGGGGCCAGGGAACCCTGGTCACCG TCTCCTCA |
|---|---|---|---|---|
| | | | SEQ ID NO: 24237 | SEQ ID NO: 28243 |
| | | AA | DIQMTQSPSSLSASVGDRVTITCRASQGISNYLA WFQQKPGKAPKSLINAASSLQSGVPSKFSGSGSG TDFTLTISSLQPEDFATYYCQQYHSYPFTFGPGT KVDIK | EVQLVESGGGLVKPGGSLRLSCAASGFTFSSYSMN WVRQAPGKGLEWVSSISSSSTYIYYADSVKGRFTIS RDNAKNSLYLQMNSLRAEDTAVYYCARVASFDY WGQGTLVTVSS |
| | | | SEQ ID NO: 24238 | SEQ ID NO: 28244 |
| iPS:392622 | 21-225_17H8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGGTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTACAACT TATTACTGTCTACAGCATAATAGTTACCCACTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGAAGTAGTTA CTACTGGGGCTGGATCCGCCAGCCCCCAGGGAG GGGGCTGGAGTGGATTGGGAATATCTATTATGGT GGGAACACCTACTACAACCCGTCCCTCAAGAGTC GAGTCACCATATCCGTAGACACGTCCAAGAACC AGTTCTCCCTGAAGCTGAGCTCTGTGACCGCCGC AGACACGGCTGTGTATTACTGTGCGAGACATGGA AAAGACTGGGGCCTTGACTACTGGGGCCAGGGA ACCCTGGTCACCGTCTCCTCA |
| | | | SEQ ID NO: 24239 | SEQ ID NO: 28245 |

EP 4 435 105 A2

FIGURE 50

| | | AA | DIQMTQSPSSLSASVGDRVTITCRASQGIRNDLG WYQQKPGKAPKRLIYGASSLQSGVPSRFSGSGS GTEFTLTISSLQPEDFTTYYCLQHNSYPLTFGGGT KVEIK | QLQLQESGPGLVKPSETLSLTCTVSGGSISRSSYYW GWIRQPPGRGLEWIGNIYYGGNTYYNPSLKSRVTIS VDTSKNQFSLKLSSVTAADTAVYYCARHGKDWGL DYWGQGTLVTVSS |
|---|---|---|---|---|
| | | | SEQ ID NO: 24240 | SEQ ID NO: 28246 |
| IPS:392624 | 21-225_17H12 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAAGCAGGGAAAG CCCCTAAGCGCCTGATCAATGCTGCATCCAGT TTGCAAAGTGGGGTCCCATCAAGGTTCAGCGG CATTGGCTCTGGGACAGAATTCACTCTCACAA TCACCGGCCTGCAGCCTGAAGATTTTGCAACT TATTACTGTCTACAGCATTATAGTTATATGTTC ACTTTCGGCGGAGGGACCAAGGTGGAGATCA AA | GAGGTGCAACTGGTGGAGTCTGGGGGAGGCCTG GTCAAGCCTGGGGGGTCCCTGAGACTCTCCTGTG CAGCCTCTGGATTCACCTTCAGTAGCTATACCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATTTGCAAATGAACAGCCTGAGAGCCGAGGA CACGGCTGTATATTACTGTGCGAGAGATCGAGGC TCCATCTGGGGCCAAGGGACAATGGTCACCGTCT CTTCA |
| | | | SEQ ID NO: 24241 | SEQ ID NO: 28247 |
| | | AA | DIQMTQSPSSLSASVGDRVTITCRASQDIRNDLG WYQQKAGKAPKRLINAASSLQSGVPSRFSGIGS GTEFTLTITGLQPEDFATYYCLQHYSYMFTFGGG TKVEIK | EVQLVESGGGLVKPGGSLRLSCAASGFTFSSYTMN WVRQAPGKGLEWVSSISGSSSYIYYADSVKGRFTIS RDNAKNSLYLQMNSLRAEDTAVYYCARDRGSIWG QGTMVTVSS |
| | | | SEQ ID NO: 24242 | SEQ ID NO: 28248 |

1440

FIGURE 50

| iPS:392626 | 21-225_18A5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCTTGCAGCCTGAAGATTTTGCAACT TATTACTGTCTACAGCATAATAGTTACCCGTG GACGTTCGGCCAAGGGACCAAGGTGGAAATC AAA | CAGGTGCAGTTGGTGGAGTCTGGGGGAGGCGTG GTCCAGCCTGGGAGGTCCCTGAGACTCTCCTATA CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCTTGG CTGGACGGAAGAGTACTGGGGCCAGGGAACCCT GGTCACCGTCTCCTCA |
|---|---|---|---|---|
| | | | SEQ ID NO: 24243 | SEQ ID NO: 28249 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPWTFGQG TKVEIK | QVQLVESGGGVVQPGRSLRLSYTASGFTFSDYGMH WVRQAPGKGLEWVAVIWFDGSNKYYADSVKGRF TISRDNSKNTLYLQMNSLRAEDTAVYYCARDLGW TEEYWGQGTLVTVSS |
| | | | SEQ ID NO: 24244 | SEQ ID NO: 28250 |
| iPS:392628 | 21-225_20C2 | NA | GACATCCAGATGACCCAGTCTCCATCCTCCCT GTCTGCATCTGTACAAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTTTGCTGCATCCAGTT TGCAAAGTGGGGTCCCGTCAAGGTTCAGCGGC AGTGGATCTGGGACAGAATTCACTCTCACAAT CAGCAGCCTGCAGCCTGAAGATTTTGCAACTT ATTACTGTCTTCAACATGCTAGTTACCCGCTCA CTTTCGGCGGAGGGACCAAGGTGGAGATCGA A | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAATATCTATTATAGT GGGACTGCCTACTGTAATTCGTCCCTCAAGAGTC GAGTCATTATATCCGTAGACACGTCCAAGAACCA GTTCTCCCTGAAGCTGAGCTCTGTGACCGCCACA GACACGGCTGTGTATTACTGTGCGAGACATAGTA GCAGCTGGTCCCTTGACAACTGGGGCCAGGGAA CCCTGGTCACCGTCTCCTCA |
| | | | SEQ ID NO: 24245 | SEQ ID NO: 28251 |

FIGURE 50

| | | AA | DIQMTQSPSSLSASVQDRVTITCRASQGIRNDLG WYQQKPGKAPKRLIFAASSLQSGVPSRFSGSGSG TEFTLTISSLQPEDFATYYCLQHASYPLTFGGGT KVEIE | QLQLQESGPGLVKPSETLSLTCTVSGGSISRSSYYW GWIRQPPGKGLEWIGNIYYSGTAYCNSSLKSRVIIS VDTSKNQFSLKLSSVTATDTAVYYCARHSSSWSLD NWGQGTLVTVSS |
|---|---|---|---|---|
| | | | SEQ ID NO: 24246 | SEQ ID NO: 28252 |
| IPS:392630 | 21-225_20E5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATTTGGTATGAAGAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCGGTCTGACTACTGGGGCCAGGGAACCCTGG TCACCGTCTCCTCA |
| | | | SEQ ID NO: 24247 | SEQ ID NO: 28253 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYEENNQYYADSVKGR FTISRDNSKNTLYLQMNSLRAEDTAVYYCARELGF RSDYWGQGTLVTVSS |
| | | | SEQ ID NO: 24248 | SEQ ID NO: 28254 |

FIGURE 50

| | | | | |
|---|---|---|---|---|
| iPS:392632 | 21-225_16A11 | NA | GACATCCAGATGACCCAGTCTCCATCCTCCCT GTCTGCATCTGTCGGAGACAGTGTCACCATCT CTTGCCGGGCAAGTCAGGACATTAGAAATCAT TTAGGCTGGTATCAGCGTAACCCAGGGAAAGC CCCTAAGCGCCTGATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGACTTTGCAACTTA TTACTGTCTACAGTATAATAGTTATCCATTCAC TTTCGGCCCTGGGACCAAAGTGGACATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT CTCATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTAGCAGCC TTTGACTACTGGGGCCAGGGAACCCTGGTCACCG TCTCCTCA |
| | | | SEQ ID NO: 24249 | SEQ ID NO: 28255 |
| | | AA | DIQMTQSPSSLSASVGDSVTISCRASQDIRNHLG WYQRNPGKAPKRLIYAASSLQSGVPSRFSGSGS GTEFTLTISSLQPEDFATYYCLQYNSYPFTFGPGT KVDIK | EVQLVESGGGLVKPGGSLRLSCAASGFTFSSYSMN WVRQAPGKGLEWVSSISGSSSLIYYADSVKGRFTIS RDNAKNSLYLQMNSLRAEDTAVYYCARVAAFDY WGQGTLVTVSS |
| | | | SEQ ID NO: 24250 | SEQ ID NO: 28256 |
| iPS:392634 | 21-225_17H3 | NA | GACATCCAGATGACCCAGTCTCCATCCTCCCT GTCTGCATCTGTAGGAGACAGAGTCACCTTCA CTTGCCGGGCAAGTCAGGGCATTAGAAGTGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCAATATAGTTACCCTCG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTGTGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAAAAGTA TAGCAGCAGCTGGTACGACTACGGTATGGACGTC TGGGGCCAAGGGACCACGGTCACCGTCTCCTCA |
| | | | SEQ ID NO: 24251 | SEQ ID NO: 28257 |

FIGURE 50

| | | | | |
|---|---|---|---|---|
| | | AA | DIQMTQSPSSLSASVGDRVTFTCRASQGIRSDLG WYQQKPGKAPKRLIYAASSLQSGVPSRFSGSGS GTEFTLTISSLQPEDFATYYCLQQYSYPRTFGQG TKVEIK | QVQLVESGGGVVQPGRSLRLSCAASGFTFSNCVMH WVRQAPGKGLEWVAVIWYDGSNKYYADSVKGRF TISRDNSKNTLYLQMNSLRAEDTAVYYCAREKYSS SWYDYGMDVWGQGTTVTVSS |
| | | | SEQ ID NO: 24252 | SEQ ID NO: 28258 |
| iPS:392636 | 21-225_17A6 | NA | GACATCCAGATGACCCAGTCTCCATCCTCCCT GTCTGCATCTGTCGGAGACAGAGTCACCATCA CTTGCCGGGCAAGTCAGACCATTAGCAACTAT TTAAATTGGTATCATCAGAAACCAGGGAAAGC CCCTAAGCTCCTGATCTATGCTGCTTCCAGTTT GCAAAGTGGGGTCCCATCAAGGTTCAGTGGCA GTGGATCTGGGACAGATTTCACTCTCACCATC AGCAGTCTGCAACCTGAAGATTTTGCAACTTA CTACTGTCAACAGAGTCACACTTCCCCGCTCA CTTTCGGCGGAGGGACCAAGGTGGAGATCAA A | CAGGTACAGCTGCAGCAGTCAGGTCCAGGACTG GTGAAGCCCTCGCAGACCCTCTCACTCACCTGTG CCATCTCCGGGGACAGTGTCTCTCGCAACACTGC TGCTTGGAGCTGGATCAGGCAGTCCCCATCGAGA GGCCTTGAGTGGCTGGGAAGGACATACTACAGG TCCAAGTGGTATAATGATTATGCAGTATCTGTGA AAAGTCGAGTAACCATCAACCCAGACACATCCA AGAACCAGTTCTCCCTGCAGCTGAACTCTGTGAC TCCCGAGGACACGGCTGTGTATTACTGTGCAAGA GTAAGCAGTGGCTGGTCCCATCACTACTACTACT ACGGTATGGACGTCTGGGGCCAAGGGACCACGG TCACCGTCTCCTCA |
| | | | SEQ ID NO: 24253 | SEQ ID NO: 28259 |
| | | AA | DIQMTQSPSSLSASVGDRVTITCRASQTISNYLN WYHQKPGKAPKLLIYAASSLQSGVPSRFSGSGS GTDFTLTISSLQPEDFATYYCQQSHTSPLTFGGGT KVEIK | QVQLQQSGPGLVKPSQTLSLTCAISGDSVSRNTAA WSWIRQSPSRGLEWLGRTYYRSKWYNDYAVSVKS RVTINPDTSKNQFSLQLNSVTPEDTAVYYCARVSSG WSHHYYYYGMDVWGQGTTVTVSS |
| | | | SEQ ID NO: 24254 | SEQ ID NO: 28260 |

FIGURE 50

| iPS:392638 | 21-225_17F9 | NA | GACATCCAGATGACCCAGTCTCCATCCTCCCT GTCTGCATCTGTAGGAGACAGAGTCACCATCA CTTGCCGGGCAAGTCAGGTCATTAGAAATGAT TTAGGCTGGTATCAGCAGAAACCAGGGAAAG CCCCTAAGCGCCTGATCTATGCTGTATCCAGTT TGCAAAGTGGGGTCCCATCAAGGTTCAGCGGC AGTGGATCTGGGACAGAATTCACTCTCACAAT CAACAGCCTGCAGCCTGAAGATTTTGCAACTT ATTACTGTCTACAACATAATACTTATCCGCTCA CTTTCGGCGGCGGGACCAAGGTGGAGTTCAAA | CAGCTGCAGCTGCAGGAGTCGGGCCCAGGACTG GTGAAGCCTTCGGAGACCCTGTCCCTCACCTGCA CTGTCTCTGGCGGCTCCATCAGCAGAAGTAGTTA CTATTGGGGCTGGATCCGCCAGCCCCCAGGGAA GGGCCTGGAGTGGATTGGGAATATCTATTATAGT GGGAGCACCTACTACAATCCGTCCCTCAAGAGTC GAGTCACCATATCCGTAGACTCGTCCAAGAACCA GTTCTCCCTGAACCTGAACTCTGTGACCGCCGCA GACACGGCTGTGTATTCCTGTGCGAGACATGGAA AAGACTGGGGCCTTGACTACTGGGGCCAGGGAA CCCTGGTCACCGTCTCCTCA |
|---|---|---|---|---|
| | | | SEQ ID NO: 24255 | SEQ ID NO: 28261 |
| | | AA | DIQMTQSPSSLSASVGDRVTITCRASQVIRNDLG WYQQKPGKAPKRLIYAVSSLQSGVPSRFSGSGS GTEFTLTINSLQPEDFATYYCLQHNTYPLTFGGG TKVEFK | QLQLQESGPGLVKPSETLSLTCTVSGGSISRSSYYW GWIRQPPGKGLEWIGNIYYSGSTYYNPSLKSRVTIS VDSSKNQFSLNLNSVTAADTAVYSCARHGKDWGL DYWGQGTLVTVSS |
| | | | SEQ ID NO: 24256 | SEQ ID NO: 28262 |
| iPS:392640 | 21-225_18A1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGTGTCTGGATTCACCTTCAGTAGCTATGGCAT GCATTGGGTCCGCCAGGCTCCAGGCAAGGGGCT GGAGTGGGTGGCAGTTATATGGTATGAGGAAAA TAATAAATATTATGTAGACTCCGTGAAGGGCCGA TTCACCATCTCCAGAGACAATTCCAAGAGCACGC TGTATCTGCAAATGAACAGCCTGAGAGCCGAGG ACACGGCTGTGTATTACTGTGTGAGAGAGCTAGG CTTCCAGTCTGACTACTGGGGCCAGGGAACCCCG GTCACCGTCTCCTCA |
| | | | SEQ ID NO: 24257 | SEQ ID NO: 28263 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAVSGFTFSSYGMH WVRQAPGKGLEWVAVIWYEENNKYYVDSVKGRF TISRDNSKSTLYLQMNSLRAEDTAVYYCVRELGFQ SDYWGQGTPVTVSS |
| | | | SEQ ID NO: 24258 | SEQ ID NO: 28264 |
| iPS:392642 | 21-225_18C6 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGCAGAAACCGGGGAAAG CCCCTAAGCGCCTGATCTATGCTGCATCCAGT TTGCAAAGTGGGGTCCCGTCAAGGTTCAGCGG CAGTGGATCTGGGACAGAATTCACTCTCACAA TCAGCAGCCTGCAGCCTGAAGATTTTGCAACT TATTACTGTCTACAGCATAGTTCTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGGAGTAGTTA TTACTGGGGCTGGATCCGCCAGCCCCCAGGGAA GGGGCTGGAGTGGATTGGGAATATCTATTATAGT GGGTACACCTACTACAACCCGTCCCTCAAGAGTC GAGTCATCATATCCGTAGACACGTCCAAGAACCA GTTCTCCCTGAAGCTGAGCTCTGTGACCGCCGCA GACACGGCTCTGTATTACTGTGCGAGACATAGTA GCAGTTGGTCCCTTGACGACTGGGGCCAGGGAA CCCTGGTCACCGTCTCCTCT |
| | | | SEQ ID NO: 24259 | SEQ ID NO: 28265 |
| | | AA | DIQMTQSPSSLSASVGDRVTITCRTSQGIRNDLG WYQQKPGKAPKRLIYAASSLQSGVPSRFSGSGS GTEFTLTISSLQPEDFATYYCLQHSSYPLTFGGGT KVEIK | QLQLQESGPGLVKPSETLSLTCTVSGGSISRSSYYW GWIRQPPGKGLEWIGNIYYSGYTYYNPSLKSRVIIS VDTSKNQFSLKLSSVTAADTALYYCARHSSSWSLD DWGQGTLVTVSS |
| | | | SEQ ID NO: 24260 | SEQ ID NO: 28266 |

1446

FIGURE 50

| iPS:392644 | 21-225_19E1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAAT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTTCCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCCGGCAAGGGGCT GGAGTGGGTGGCAGTTATTTGGTATGAAGAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCGGTCTGACTACTGGGGCCAGGGAACCCTGG TCACCGTCTCCTCA |
|---|---|---|---|---|
| | | | SEQ ID NO: 24261 | SEQ ID NO: 28267 |
| | | AA | DIQMTQSPSSLSASVGDRVTITCRASQGIRNDLG WYQQKPGKAPKRLIYAASNLQSGVPSRFSGSGS GTEFTLTISSLQPEDFATYYCLQHNSFPLTFGGGT KVEIK | QVQLVESGGGVVQPGRSLRLSCAASGFTFSNYGM HWVRQAPGKGLEWVAVIWYEENNQYYADSVKGR FTISRDNSKNTLYLQMNSLRAEDTAVYYCARELGF RSDYWGQGTLVTVSS |
| | | | SEQ ID NO: 24262 | SEQ ID NO: 28268 |
| iPS:392646 | 21-225_20G2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTCCAAAGTGGGGTCCCATCAAGGTTCAGCGG CAGTGGATCTGGGACAGAATTCACTCTCACAA TCAGCAGCCTGCAGCCTGAAGATTTTGCAAGT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCCTCAGTAGCGATGACAT GCACTGGGTCCGCCAGGAACCAGGCAAGGGGCT GGAGTGGGTGGCAGTTATATGGTTTGATGGAAGT AATAAATACTATGCAGACTCCGTGAAGGGCCGA TTCATCATGTCCAGAGACAATTCCAAGAACACCC TGTATCTGCAAATGAACAGCCTGAGAGCCGGGG ACACGGCTGTGTATTACTGTGCGAGAGATCTGAT AGCAGCAGCTGGTACGGTTGACTACTGGGGCCA GGGAACCCTGGTCACCGTCTCCTCA |
| | | | SEQ ID NO: 24263 | SEQ ID NO: 28269 |

FIGURE 50

| | | | | |
|---|---|---|---|---|
| | | AA | DIQMTQSPSSLSASVGDRVTITCRASQGIRNDLG WYQQKPGKAPKRLIYAASSFQSGVPSRFSGSGS GTEFTLTISSLQPEDFASYYCLQHNSYPLTFGGGT KVEIK | QVQLVESGGGVVQPGRSLRLSCAASGFTLSSDDMH WVRQEPGKGLEWVAVIWFDGSNKYYADSVKGRFI MSRDNSKNTLYLQMNSLRAGDTAVYYCARDLIAA AGTVDYWGQGTLVTVSS |
| | | | SEQ ID NO: 24264 | SEQ ID NO: 28270 |
| iPS:392648 | 21-225_16D11 | NA | GACATCCAGATGACCCAGTCTCCATCCTCCCT GTCTGCATCTGTCGGAGACAGAGTCACCATCA CTTGCCGGGCAAGTCAGACCATTAGCAACTAT TTAAATTGGTATCATCAGAAACCAGGGAAAGC CCCTAAGCTCCTGATCTATGCTGCATCCAGTTT GCAAAGTGGGGTCCCATCAAGGTTCAGTGGCA GTGGATCTGGGACAGATTTCACTCTCACCATC AGCAGTCTGCAACCTGAAGATTTTGCAACTTA CTACTGTCAACAGAGTCACAGTTCCCCGCTCA CTTTCGGCGGAGGGACCAAGGTGGAGATCAA A | CAGGTACAGCTGCAGCAGTCAGGTCCAGGACTG GTGAAGCCCTCGCAGACCCTCTCACTCACCTGTG CCATCTCCGGGGACAGTGTCTCTCGCAACACTGC TGCTTGGAGCTGGATCAGGCAGTCCCCATCGAGA GGCCTTGAGTGGCTGGGAAGGACATACTACAGG TCCAAGTGGTATAATGATTATGCAGTATCTGTGA AAAGTCGAATAACCATCAACCCAGACACATCCA AGAACCAGTTCTCCCTGCAGCTGAACTCTGTGAC TCCCGAGGACACGGCTGTGTATTACTGTGCAAGA GTAAACAGTGGCTGGTCCCATCACTACTACTACT ACGGTATGGACGTCTGGGGCCAAGGGACCACGG TCACCGTCTCCTCA |
| | | | SEQ ID NO: 24265 | SEQ ID NO: 28271 |
| | | AA | DIQMTQSPSSLSASVGDRVTITCRASQTISNYLN WYHQKPGKAPKLLIYAASSLQSGVPSRFSGSGS GTDFTLTISSLQPEDFATYYCQQSHSSPLTFGGGT KVEIK | QVQLQQSGPGLVKPSQTLSLTCAISGDSVSRNTAA WSWIRQSPSRGLEWLGRTYYRSKWYNDYAVSVKS RITINPDTSKNQFSLQLNSVTPEDTAVYYCARVNSG WSHHYYYYGMDVWGQGTTVTVSS |
| | | | SEQ ID NO: 24266 | SEQ ID NO: 28272 |

FIGURE 50

| iPS:392650 | 21-225_17A4 | NA | GACATCCAGATGACCCAGTCTCCATCTTCCGT GTCTGCATCTGTAGGAGACAGAGTCACCATCA CTTGTCGGGCGAGTCAGGGTATTGGCAACTGG TTAGCCTGGTATCAGCAGAAACCAGGGAAAG CCCCTAAACTCCTGATCTTTGCTGCATCCAGTT TGCAAAGTGGGGTCCCATCACGGTTCAGCGGC AGTGGATCTGGGACAGATTTCACTCTCACCAT CAGCAGCCTGCAGCCTGAAGATTTTGTAACTT ACTATTGTCAACAGGCTAACAGTTTCCCTCGG ACGTTCGGCCAAGGGACCAAGGTGGAAATCA AA | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTACAT GAGCTGGATCCGCCAGGCTCCAGGGAAGGGGCT GGAGTGGGTTTCACACATTAGTAGTAGTGGTAGT ACCATATATTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGAGATATCGGAAT AACCGGGGATACTTCGATCTCTGGGGCCGTGGCA CCCTGGTCACTGTCTCTTCA |
|  |  |  | SEQ ID NO: 24267 | SEQ ID NO: 28273 |
|  |  | AA | DIQMTQSPSSVSASVGDRVTITCRASQGIGNWLA WYQQKPGKAPKLLIFAASSLQSGVPSRFSGSGSG TDFTLTISSLQPEDFVTYYCQQANSFPRTFGQGT KVEIK | QVQLVESGGGLVKPGGSLRLSCAASGFTFSDYYMS WIRQAPGKGLEWVSHISSSGSTIYYADSVKGRFTIS RDNAKNSLYLQMNSLRAEDTAVYYCARYRNNRG YFDLWGRGTLVTVSS |
|  |  |  | SEQ ID NO: 24268 | SEQ ID NO: 28274 |
| iPS:392652 | 21-225_17C6 | NA | GACATCCAGATGACCCAGTCTCCATCCTCCCT GTCTGCATCTGTAGGAGACAGAGTCACCATCA CTTGCCGGGCAAGTCAGAGCATTAATACTTAT TTAAATTGGTATCAGCAAAAACCAGGGAAAG CCCCTAAGCTCCTGATCTATGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTTCTGTCAACAGAGTTACAGAACCCCCTTT TTCACTTTCGGCCCTGGGACCAAAGTGGATAT CAAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAACTGGGTCCGCCAGGCTCCAGGGAAGGGGCT GGAGTGGGTCTCAGTTATTAGTGGTCGTGGTGGT AACACATTCTACGCAGACTCCGTGAAGGGCCGGT TCACCATCTCCAGAGACAATTCCAAGAACACGCT CTTTCTGCAAATGAACAGCCTGAGAGCCGAGGA CACGGCCGTATATTATTGTGCGTCCCGTTTGGCA GTGGCTGGCTCGGAGGCTTTTGATATCTGGGGCC AAGGGACAATGGTCACCGTCTCTTCA |
|  |  |  | SEQ ID NO: 24269 | SEQ ID NO: 28275 |

FIGURE 50

| | | | | |
|---|---|---|---|---|
| | | AA | DIQMTQSPSSLSASVGDRVTITCRASQSINTYLN WYQQKPGKAPKLLIYAASSLQSGVPSRFSGSGS GTDFTLTISSLQPEDFATYFCQQSYRTPFFTFGPG TKVDIK | EVQLLESGGGLVQPGGSLRLSCAASEFTFSSYAMN WVRQAPGKGLEWVSVISGRGGNTFYADSVKGRFTI SRDNSKNTLFLQMNSLRAEDTAVYYCASRLAVAG SEAFDIWGQGTMVTVSS |
| | | | SEQ ID NO: 24270 | SEQ ID NO: 28276 |
| IPS:392654 | 21-225_17A10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGCCAAGGGGCT GGAGTGGGTGGCAGTTATTTGGTATGAAGAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCGGTCTGACTACTGGGGCCAGGGAACCCTGG TCACCGTCTCCTCA |
| | | | SEQ ID NO: 24271 | SEQ ID NO: 28277 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSNYGM HWVRQAPAKGLEWVAVIWYEENNQYYADSVKGR FTISRDNSKNTLYLQMNSLRAEDTAVYYCARELGF RSDYWGQGTLVTVSS |
| | | | SEQ ID NO: 24272 | SEQ ID NO: 28278 |

FIGURE 50

| iPS:392656 | 21-225_1F2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT GTGCAAAGTGGGGTCCCATCAAGGTTCAGCGG CAGTGGATCTGGGACAGAATTCACTCTCACAA TCAGCAGCCTGCAGCCTGAAGATTTTGCAATT TATTACTGTCTACAGCATAATAGTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGGAGTAGTTA CTACTGGGGCTGGATCCGCCAGCCCCCAGGGAA GGGGCTGGAGTGGATTGGGAATATTTATTATAGT GGGAGCGCCTACAACAACCCGTCCCTCAAGGGT CGAGTCACCATATCCGTAGACACGTCCAAGAACC AGTTCTCCCTGAAGCTGAACTCTGTGACCGCCGC AGACACGGCTGTGTATTACTGTGGGAGACATGGA AAAGACTGGGGCCTTGACTACTGGGGCCAGGGA ACCCTGGTCACCGTCTCCTCA |
| | | | SEQ ID NO: 24273 | SEQ ID NO: 28279 |
| | | AA | DIQMTQSPSSLSASVGDRVTITCRASQGIRNDLG WYQQKPGKAPKRLIYAASSVQSGVPSRFSGSGS GTEFTLTISSLQPEDFAIYYCLQHNSYPLTFGGGT KVEIK | QLQLQESGPGLVKPSETLSLTCTVSGGSISRSSYYW GWIRQPPGKGLEWIGNIYYSGSAYNNPSLKGRVTIS VDTSKNQFSLKLNSVTAADTAVYYCGRHGKDWGL DYWGQGTLVTVSS |
| | | | SEQ ID NO: 24274 | SEQ ID NO: 28280 |
| iPS:392658 | 21-225_18E8 | NA | GACATCCAGATGACCCAGGCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATTTGGTATGAAGAAAT AATCAATACTATGCAGACTCCGTGAAGGGCCGAT TCACCGTCTCCAGAGACAATTCCAAGAACACGCT GTTTCTGCAAATGAACAGCCTGAGAGCCGAGGA CACGGCTGTGTATTACTGTGCGAGAGAACTGGGG TTCCGGTCTGACTACTGGGGCCAGGGAACCCTGG TCACCGTCTCCTCA |
| | | | SEQ ID NO: 24275 | SEQ ID NO: 28281 |

FIGURE 50

| | | | | |
|---|---|---|---|---|
| | | AA | DIQMTQAPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSNYGM HWVRQAPGKGLEWVAVIWYEENNQYYADSVKGR FTVSRDNSKNTLFLQMNSLRAEDTAVYYCARELGF RSDYWGQGTLVTVSS |
| | | | SEQ ID NO: 24276 | SEQ ID NO: 28282 |
| iPS:392660 | 21-225_19B3 | NA | GACATCCAGATGACCCAGTCTCCATCCTCCCT GTCTGCTTCTGTCGGAGACAGAATCACCATCA CTTGCCGGGCAGGTCAGAACATTATCAACTAT TTAAATTGGTATCAGCAGAAACCAGGGAAAG CCCCTAACCTCCTGATATATGTTGCATCCAGTT TGCAAAGTGGGGTCCCATCAAGGTTCAATGGC AGTGGATCTGGGACAGATTTCACTCTCACCAT CAGCAGTCTGCAACCTGAAGATTTTGCAACTT ACTACTGTCAACAGAGTTACAGTACCCCTTTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGACAT GCACTGGGTCCGCCAGGCTCCAGGCAAGGGGCT GGAGTGGGTGGCAGTTATATGGTATGACGGAAG TGATAAATACTATGCAGACTCCGTGAAGGGCCG ATTCACCATCTCCAGAGACAATTCCAAGAACACG CTCTTTCTGCAAATGAACAGCCTGAGAGCCGAGG ACACGGCTGTGTATTACTGTGCGAGAGATCGGGC CTATAGCAGCTCGTCTGACTACTGGGGGCCAGGGA ACCCTGGTCACCGTCTCCTCA |
| | | | SEQ ID NO: 24277 | SEQ ID NO: 28283 |
| | | AA | DIQMTQSPSSLSASVGDRITITCRAGQNIINYLNW YQQKPGKAPNLLIYVASSLQSGVPSRFNGSGSGT DFTLTISSLQPEDFATYYCQQSYSTPFTFGPGTKV DIK | QVQLVESGGGVVQPGRSLRLSCAASGFTFSSYDMH WVRQAPGKGLEWVAVIWYDGSDKYYADSVKGRF TISRDNSKNTLFLQMNSLRAEDTAVYYCARDRAYS SSSDYWGQGTLVTVSS |
| | | | SEQ ID NO: 24278 | SEQ ID NO: 28284 |

FIGURE 50

| | | | | |
|---|---|---|---|---|
| iPS:392664 | 21-225_20F6 | NA | GACATCCAGATGACCCAGTCTCCATCCTCCCT GTCTGCATCTGTAGGAGACAGAGTCACCATCA CTTGCCGGGCAAGTCAGAGCATTATCACCTAT TTAAAATTGGTATCAGCAGAAACCAGGGAAAG CCCCTAAGGTCCTGATCCATACTGCATCCAGT TTGCAAAGTGGGGTCCCATCAAGGTTCAGTGG CAGTGGATCTGGGACAGATTTCACTCTCACCA TCAGCAGTCTGCAACCTGAAGATTTTGCAACT TACTACTGTCAACAGACTTACAGTCCCCCGCT CACTTTCGGCGGAGGGACCAAGGTGGAGATC AAA | CAGGTGCAGCTGGTGGAGTCTGGGGGAGGCGTG GTCCAGCCTGGGAGGTCCCTGAGACTCTCCTGTG TAGCGTCTGGATTCACCTTCAGTAGCTATGGCAT GCACTGGGTCCGCCAGGCTCCAGGCAAGGGGCT GGAGTGGGGGGGCAGTTATATGGCATGATGGAAG TAATAAATACTATGCAGACTCCGTGAAGGGCCG ATTCACCATCTCCAGAGACAATGCCAAGAACAC GCTGTATCTGCAAATGAACAGCCTGAGAGCCGA GGACACGGCTGTGTATTACTGTGCGAGAGATCTG AGTATGGGCGGAATGGACGTCTGGGGCCAAGGG ACCACGGTCACCGTCTCCTCA |
| | | | SEQ ID NO: 24279 | SEQ ID NO: 28285 |
| | | AA | DIQMTQSPSSLSASVGDRVTITCRASQSIITYLNW YQQKPGKAPKVLIHTASSLQSGVPSRFSGSGSGT DFTLTISSLQPEDFATYYCQQTYSPPLTFGGGTK VEIK | QVQLVESGGGVVQPGRSLRLSCVASGFTFSSYGMH WVRQAPGKGLEWGAVIWHDGSNKYYADSVKGRF TISRDNAKNTLYLQMNSLRAEDTAVYYCARDLSM GGMDVWGQGTTVTVSS |
| | | | SEQ ID NO: 24280 | SEQ ID NO: 28286 |
| iPS:392666 | 21-225_16F11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT GTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGATGGTGGAGTCTGGGGGAGGCGTG GTCCAGCCTGGGAGGTCCCTGAGACTCTCCTTGTG AAGCGTCTGGATTCACCTTCAGTAGCTATGGCAT GCACTGGGTCCGCCAGGCTCCAGGCAAGGGGCT GGAGTGGGTGGCAGTTATATGGTATGAGGAAAA TAATAAATACTATGTAGACTCCGTGAAGGGCCGA TTCACCATCTCCAGAGACAATTCCAAGAGCACGC TGTATCTGCAAATGAATAGCCTGAGAGCCGAGG ACACGGCTGTGTATTACTGTGTGAGAGAACTAGG CTTCCAGTCTGACTACTGGGGCCAGGGAACCCCG GTCACCGTCTCCTCA |
| | | | SEQ ID NO: 24281 | SEQ ID NO: 28287 |

FIGURE 50

| | | | | |
|---|---|---|---|---|
| | | AA | DIQMTQSPSSLSASVGDRVTITCRASQGIRNDLG WYQQKPGKAPKRLIYAASSVQSGVPSRFSGSGS GTEFTLTISSLQPEDFATYYCLQHNSYPLTFGGG TKVEIK | QVQMVESGGGVVQPGRSLRLSCEASGFTFSSYGM HWVRQAPGKGLEWVAVIWYEENNKYYVDSVKGR FTISRDNSKSTLYLQMNSLRAEDTAVYYCVRELGF QSDYWGQGTPVTVSS |
| | | | SEQ ID NO: 24282 | SEQ ID NO: 28288 |
| iPS:392668 | 21-225_17B4 | NA | GACATCCAGATGACCCAGTCTCCATCCTCCCT GTCTGCATCTGTAGGCGACAGAGTCACCATCA CTTGCCGGGCAAGTCAGAACATTAGTAGTTAT TTAAAATTGGTATCAGCAGAAACCAGGGAAAG CCCCTAAGCTCCTGATCTTTGGTGCATCCAGTT TGCAAACTGGGGTCCCATCAAGGTTCAGTGGC AGTGGATCTGGGACAGATTTCACTCTCACCAT CAACAGTCTGCAACCTGAAGATTTTGCAACTT ACTTCTGTCAACAGAGTTACAGAACCCCCTTT TTCACTTTCGGCCCTGGGACCAAAGTGGATAT CAAA | GAGGTGCAGCTGTTGGAGTCTGGGGGAGGCTTG GTACAGCCTGGGGGGTCCCTGAGACTCTCCTGTG CAGCCTCTGAATTTACCTTTAGCAGCTATGCCAT GAACTGGGTCCGCCAGGCTCCAGGGAAGGGGCT GGAGTGGGTCTCAGTTATTAGTGGCCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTTTGGCA GTGGCTGGCTCGGAGGCTTTTGATATCTGGGGCC AAGGGACAATGGTCACCGTCTCTTCA |
| | | | SEQ ID NO: 24283 | SEQ ID NO: 28289 |
| | | AA | DIQMTQSPSSLSASVGDRVTITCRASQNISSYLN WYQQKPGKAPKLLIFGASSLQTGVPSRFSGSGSG TDFTLTINSLQPEDFATYFCQQSYRTPFFTFGPGT KVDIK | EVQLLESGGGLVQPGGSLRLSCAASEFTFSSYAMN WVRQAPGKGLEWVSVISGRGGNTFYADSVKGRFTI SRDNSKNTLYLQMNSLRAEDTAVYYCASRLAVAG SEAFDIWGQGTMVTVSS |
| | | | SEQ ID NO: 24284 | SEQ ID NO: 28290 |

FIGURE 50

| iPS:392674 | 21-225_18C2 | NA | GACATCCAGATGACCCAGTCTCCATCCTCCCT GTCTGCGTCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGATTCAGCGG CAGTGGATTTGGGACAGAATTCACTCTCACAA TCAGCAGCCTGCAGCCTGAAGATTTTGCAACT TATTACTGTCTACAACATAATAGTTACCCGTG GACGTTCGGCCTGGGGACCAAGGTGGTCATCA AA | CAGGTGCAACTGGTGGAGTCTGGGGGAGGCGTG GTCCAGCCTGGGAGGTCCCTGAGACTCTCCTGTG TAGCGTCTGGATTCACCTTCAGTGACTATGGCAT GCACTGGGTCCGCCAGGCTCCAGGCAAGGGGCT GGAGTGGATGGCAGTTATATGGTATGATGTAACT AATAAATACTATGCAGACTCCGTGAAGGGCCGA TTCACCATCTCCAGAGACAATTCCAAGAACACGC TGTATCTGCAAATGAACAGCCTGAGCGCCGAGG ACACGGCTGTTTATTACTGTGCGAGAGAGCTTGG CTGGTACGAGGACTACTGGGGCCAGGGAACCCT GGTCACCGTCTCCTCA |
| | | | SEQ ID NO: 24285 | SEQ ID NO: 28291 |
| | | AA | DIQMTQSPSSLSASVGDRVTITCRASQGIRNDLG WYQQKPGKAPKRLIYTASSLQSGVPSRFSGSGFG TEFTLTISSLQPEDFATYYCLQHNSYPWTFGLGT KVVIK | QVQLVESGGGVVQPGRSLRLSCVASGFTFSDYGM HWVRQAPGKGLEWMAVIWYDVTNKYYADSVKG RFTISRDNSKNTLYLQMNSLSAEDTAVYYCARELG WYEDYWGQGTLVTVSS |
| | | | SEQ ID NO: 24286 | SEQ ID NO: 28292 |
| iPS:392676 | 21-225_19F3 | NA | GACATCCAGATGACCCAGTCTCCATCCTCCCT GTCTGCATCTGTACGAGACAGAGTCACCATCA CTTGCCGGGCAAGTCAGGGCATAAGAAATGAT TTAGGCTGGTATCAGCAGAAACCAGGGAAAG CCCCTAAGCGCCTGATCTATGCTGTTTCCAGTT TGCAAAGTGGGGTCCCATCAAGGTTCAGCGGC AGTGGATCTGGGACAGAGTTCACTCTCACAAT CAGCAGCCTGCAGCCTGAAGATTTTGCAACTT ATTACTGTCTACAGCATGCCAGTTACCCGCTC ACTTTCGGCGGAGGGACCAAGGTGGAGATCA AA | CAGGTGCAGCTGCAGGAGTCGGGCCCAGGACTG GTGAAGCCTTCGGAGACCCTGTCCCTCACCTGCA CTGTCTCCGGTGGCGCCATCAGCGGTAGTAGTTA CTACTGGGGCTGGATCCGCCAGCCCCCAGGGAA ACAACTGGAGTGGATTGGGAATATCTATTATAGT GGGAGCACCTACTACAACCCGTCCTTCAAGAGTC GAGTCACCATATCCGTAGACACGTCCAAGAACC AGTTCTCCCTGAAGCTGAGCTCTGTGACCGCCGA AGACACGGCTGTCTATTACTGTGCGAGACATTCC AGTAGCTGGTCCCTTGACTACTGGGGCCAGGGAA CCCTGGTCACCGTCTCCTCT |
| | | | SEQ ID NO: 24287 | SEQ ID NO: 28293 |

FIGURE 50

| | | | | |
|---|---|---|---|---|
| | | AA | DIQMTQSPSSLSASVRDRVTITCRASQGIRNDLG WYQQKPGKAPKRLIYAVSSLQSGVPSRFSGSGS GTEFTLTISSLQPEDFATYYCLQHASYPLTFGGG TKVEIK | QVQLQESGPGLVKPSETLSLTCTVSGGAISGSSYYW GWIRQPPGKQLEWIGNIYYSGSTYYNPSFKSRVTIS VDTSKNQFSLKLSSVTAEDTAVYYCARHSSSWSLD YWGQGTLVTVSS |
| | | | SEQ ID NO: 24288 | SEQ ID NO: 28294 |
| iPS:392678 | 21-225_20F3 | NA | GACATCCAGATGACCCAGTCTCCATCCTCCCT GTCTGCATCTGTAGGAGACAGAGTCACCATCA CTTGCCGGGCAAGTCAGAGCATTAGTAGTTAT TTATATTGGTATCAGCAGAAACCAGGGAAAGC CCCTAAGCTCCTGATCTATGCTGCATCCAGTTT GCAAAGTGGGGTCCCATCAAGGTTCAGTGGCA GTGGATCTGGGACAGATTTCACTCTCACCATC AGCAGTCTGCAACCTGAAGATTTTGCAACTTA CTACTGTCAACAGAGTTACAGTGCCCCTCCAT TCACTTTCGGCCCTGGGACCAAAGTGGATATC AAA | GAGGTGCAGCTGTTGGAGTCTGGGGGAGGCTTG GTACAGCCGGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGTTATTAGTGGTAGTGGTGGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAAAGGATAG CAGCAGCTGGTACGGAGTACTTCGATCTCTGGGG CCGTGGCACCCTGGTCACTGTCTCCTCA |
| | | | SEQ ID NO: 24289 | SEQ ID NO: 28295 |
| | | AA | DIQMTQSPSSLSASVGDRVTITCRASQSISSYLYW YQQKPGKAPKLLIYAASSLQSGVPSRFSGSGSGT DFTLTISSLQPEDFATYYCQQSYSAPPFTFGPGTK VDIK | EVQLLESGGGLVQPGGSLRLSCAASGFTFSSYAMN WVRQAPGKGLEWVSVISGSGGSTYYADSVKGRFTI SRDNSKNTLYLQMNSLRAEDTAVYYCAKRIAAAG TEYFDLWGRGTLVTVSS |
| | | | SEQ ID NO: 24290 | SEQ ID NO: 28296 |

FIGURE 50

| iPS:392680 | 21-225_20A7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAAAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCTCTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATTTGGTATGAAGAAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTATTTATTACTGTGCGAGAGAACTGGGG TTCCGGTCTGACTACTGGGGCCAGGGAACCCTGG TCACCGTCTCCTCA |
|  |  |  | SEQ ID NO: 24291 | SEQ ID NO: 28297 |
|  |  | AA | DIQMTQSPSSLSASVGDRVTITCRASQGIRNDLG WYQQKPGKAPKRLIYAASSLQSGVPSRFSGSGS GTEFSLTISSLQPEDFATYYCLQHNSYPLTFGGGT KVEIK | QVQLVESGGGVVQPGRSLRLSCAASGFTFSNYGM HWVRQAPGKGLEWVAVIWYEENNQYYADSVKGR FTISRDNSKNTLYLQMNSLRAEDTAIYYCARELGFR SDYWGQGTLVTVSS |
|  |  |  | SEQ ID NO: 24292 | SEQ ID NO: 28298 |
| iPS:392682 | 21-225_16A12 | NA | GACATCCAGATGACCCAGTCTCCATCCTCACT GTCTGCATCTGTAGGAGACAGAGTCACCATCA CTTGTCGGGCGAGTCAGGCCATTAACACTTAT TTAGCCTGGTTTCAGCAGAAACCAGGGAAAGC CCCTAAGTCCCTGATTTCTGCTGCATCCAGTTT GCAAAGTGGGGTCCCATCAAAGTTCAGCGGCA GTGGATCTGGGACAGAATTCACTCTCACCATC AGCAGCCTGCAGCCTGAAGATTTTGCAACTTA TTACTGCCAACAGTATTATAGTTATCCGCTCAC TTTCGGCGGAGGGACCAAGGTGGAGATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGAAT GAACTGGTTCCGCCAGGCTCCAGGGAAGGGGCT GGAGTGGGTCTCATCCATTAGTGGTAGTAGTACT GACATATACTACGCAGACTCAGTGAAGGGCCGA TTCACCATCTCCAGAGACAACGCCGAGAACTCAC TGTATCTGCAAATGAACAGCCTGAGAGCCGAGG ACACGGCTGTGTATTACTGTGCGAGAAGGGACTT CTGGGGCCAGGGAACCCTGGTCACCGTCTCCTCA |
|  |  |  | SEQ ID NO: 24293 | SEQ ID NO: 28299 |

FIGURE 50

| | | | DIQMTQSPSSLSASVGDRVTITCRASQAINTYLA WFQQKPGKAPKSLISAASSLQSGVPSKFSGSGSG TEFTLTISSLQPEDFATYYCQQYYSYPLTFGGGT KVEIK | EVQLVESGGGLVKPGGSLRLSCAASGFTFSSYRMN WFRQAPGKGLEWVSSISGSSTDIYYADSVKGRFTIS RDNAENSLYLQMNSLRAEDTAVYYCARRDFWGQ GTLVTVSS |
| | | AA | SEQ ID NO: 24294 | SEQ ID NO: 28300 |
| IPS:392684 | 21-225_17F4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GAACTGGGTCCGCCAGGCTCCAGGCAAGGGGCT GGAGTGGGTGGCAGTTATATGGTATGATGGAAAT AATAAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGTAGTGGGAG CTACTTCTTTGACTACTGGGGCCAGGGAACCCTG GTCACCGTCTCCTCA |
| | | | SEQ ID NO: 24295 | SEQ ID NO: 28301 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSNYGM NWVRQAPGKGLEWVAVIWYDGNNKHYADSVKG RFTISRDNSKNTLYLQMNSLRAEDTAVYYCASSGS YFFDYWGQGTLVTVSS |
| | | | SEQ ID NO: 24296 | SEQ ID NO: 28302 |

FIGURE 50

| | | | | |
|---|---|---|---|---|
| iPS:392686 | 21-225_17C7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGATTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAAGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG GCACGGCTGTGTATTACTGTGCGAGAGATCTTGG CTGGACGGAAGAATACTGGGGCCAGGGAACCCT GGTCACCGTCTCCTCA |
| | | | SEQ ID NO: 24297 | SEQ ID NO: 28303 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPWTFGQG TKVEIK | QVQLVESGGGVVQPGKSLRLSCAASGFTFSDYGM HWVRQAPGKGLEWVAVIWFDGSNKYYADSVKGR FTISRDNSKNTLYLQMNSLRAEGTAVYYCARDLG WTEEYWGQGTLVTVSS |
| | | | SEQ ID NO: 24298 | SEQ ID NO: 28304 |
| iPS:392690 | 21-225_18F2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAGT AATAAATACTATGTAGACTCCGTGAAGGGCCGAT TCACCATCTCCAGAGACAATTCCAAGAACACGCT GTATCTGCAAATGAACAGCCTGAGAGTCGAGGA CACGGCTGTGTATTACTGTGCGAGAGATCTTGGC TGGACGGAAGAGTACTGGGGCCAGGGAACCCTG GTCACCGTCTCCTCA |
| | | | SEQ ID NO: 24299 | SEQ ID NO: 28305 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPWTFGQG TKVEIK | QVQLVESGGGVVQPGRSLRLSCAASGFTFSDYGM HWVRQAPGKGLEWVAVIWFDGSNKYYVDSVKGR FTISRDNSKNTLYLQMNSLRVEDTAVYYCARDLG WTEEYWGQGTLVTVSS |
| | | | SEQ ID NO: 24300 | SEQ ID NO: 28306 |
| IPS:392692 | 21-225_18G10 | NA | GACATCCAGATGACCCAGTCTCCATCCTCACT GTCTGCATCTGTAGGAGACAGAGTCACCATCA CTTGTCGGGCGAGTCAGGACATTAGCTATTAT TTAGCCTGGTTTCAGCAGAAACCAGGGAAAGC CCCTAAGTCCCTGATTTATGTTGCATCCAGTTT GCAAAGTGGGGTCCCATCAAAGTTCGGCGGCA GTGGATTTGGGACAGATTTCACTCTCACCATC AGCAGCCTGCAGCCTGAAGATTTTGCAACTTA TTACTGCTTACAGTATAATAGTTACCCATTCAC TTTCGGCCCTGGGACCAAAGTGGATATCAAA | GAGGTGCAGCTGGTGGAGTCTGGGGGAGGCTTG GTACAGCCTGGGGGGTCCCTGAGACTCCTCTGTG CAGCCTCTGGAATCACCTTCAGTACCTATAGCAT GAACTGGGTCCGCCAGGCTCCAGGGAAGGGGCT GGAGTGGGTTTCATACATTAGTAGGAGTAGTAGT ACCATAGACTACGCAGACTCTGTGAAGGGCCGA TTCACCATCTCCAGAGACAATGCCAAGAACTCAC TGTATCTGCAAATGAACAGCCTGAGAGACGAGG ACACGGCTGTGTATTACTGTGCGAGAGGAGGTGG GAGCCCTTTTGACTACTGGGGCCAGGGAACCCTG GTCACCGTCTCCTCA |
| | | | SEQ ID NO: 24301 | SEQ ID NO: 28307 |
| | | AA | DIQMTQSPSSLSASVGDRVTITCRASQDISYYLA WFQQKPGKAPKSLIYVASSLQSGVPSKFGGSGF GTDFTLTISSLQPEDFATYYCLQYNSYPFTFGPGT KVDIK | EVQLVESGGGLVQPGGSLRLLCAASGITFSTYSMN WVRQAPGKGLEWVSYISRSSSTIDYADSVKGRFTIS RDNAKNSLYLQMNSLRDEDTAVYYCARGGGSPFD YWGQGTLVTVSS |
| | | | SEQ ID NO: 24302 | SEQ ID NO: 28308 |

FIGURE 50

| iPS:392694 | 21-225_19A5 | NA | GACATCCAGATGACCCAGTCTCCATCCTCCCT GTCTGCACCTGTAGGAGACAGAGTCTCCATCA CTTGCCGGGCAAGTCAGAACATTATCAACTAT TTAAATTGGTATCAGCAGAAACCAGGGAAAG CCCCTAAGCTCCTGATAGATGTTGCATCCAAT TTACAAGGTGGGGTCCCATCAAGGTTCAGTGG CAGTGGATCTGGGACAGATTTCACTCTCACCA TCAGCAGTCTGCAACCTGAAGATTTTGCAACT TACTACTGTCAACAGAGTTACAGTACCCCTTT CACTTTCGGCCCTGGGACCAAAGTGGATATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGCCAT GCACTGGGTCCGCCAGGCTCCAGGCAAGGGGCT GGAGTGGGTGGCAGTTATATGGTTTGATGGAAGT GATAAATACTATGCAGACTCCGTGAAGGGCCGA TTCACCATCTCCAGAGACAATTCCAAGAACACGC TGTATCTACAAATGAACAGCCTGAGAGCCGAGG ACACGGCTGTGTATTACTGTGCGAGAGATCGGGC CTATAGTAGCTCGTCTGACTACTGGGGCCAGGGA ACCCTGGTCACCGTCTCCTCA |
|---|---|---|---|---|
| | | | SEQ ID NO: 24303 | SEQ ID NO: 28309 |
| | | AA | DIQMTQSPSSLSAPVGDRVSITCRASQNIINYLN WYQQKPGKAPKLLIDVASNLQGGVPSRFSGSGS GTDFTLTISSLQPEDFATYYCQQSYSTPFTFGPGT KVDIK | QVQLVESGGGVVQPGRSLRLSCAASGFTFSSYAMH WVRQAPGKGLEWVAVIWFDGSDKYYADSVKGRF TISRDNSKNTLYLQMNSLRAEDTAVYYCARDRAYS SSSDYWGQGTLVTVSS |
| | | | SEQ ID NO: 24304 | SEQ ID NO: 28310 |
| iPS:392696 | 21-225_20A4 | NA | GACATCCAGATGACCCAGTCTCCAGCCTCCCT GTCTGCATCTGTAGGAGACAGAGTCACCATCA CTTGCCGGGCAAGTCAGAGCATTATCAACTAT TTAAATTGGTATCAGCAGAGACCAGGGAAATC CCCTAAGCTCCTGATCTATGCTGCATCCAGTTT GCACAGTGGGGTCCCATCAAGGTTCAGTGGCA GAGGATCTGGGACAGATTTCACTCTCACCATC AGTAGTCTGCAACCTGAAGATTTTGCAACTTA CTTCTGTCAACAGAGTTACAGAACCCCCTTAT TCACTTTCGGCCCTGGGACCAAAGTAGATTTC AAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GACCTGGGTCCGCCAGGGTCCAGGGATGGGGCT GGAGTGGGTCTCAGTTATAAGTGGTAGTGGTGGT TACACATACAACGCGGACTCCGTGAAGGGCCGG TTCACCATCTCCAGAGACAATTCCAAGAACACGC TGTATCTGCAAATGAACAGCCTGAGAGTCGAGG ACACGGCCGTATATTACTGTGCGTCCCGTATAGC AGTGGCTGGCTCGGAGGCTTTTGATATCTGGGGC CAAGGGACAATGGTCACCGTCTCTTCA |
| | | | SEQ ID NO: 24305 | SEQ ID NO: 28311 |

FIGURE 50

| | | | | |
|---|---|---|---|---|
| | | AA | DIQMTQSPASLSASVGDRVTITCRASQSIINYLN WYQQRPGKSPKLLIYAASSLHSGVPSRFSGRGSG TDFTLTISSLQPEDFATYFCQQSYRTPLFTFGPGT KVDFK | EVQLLESGGGLVQPGGSLRLSCAASGFTFSSYAMT WVRQGPGMGLEWVSVISGSGGYTYNADSVKGRFT ISRDNSKNTLYLQMNSLRVEDTAVYYCASRIAVAG SEAFDIWGQGTMVTVSS |
| | | | SEQ ID NO: 24306 | SEQ ID NO: 28312 |
| IPS:392700 | 21-225_16E12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCCTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGAGGGAAG TAATAAATATTATGTAGACTCCGTGAGGGGCCGA TTCACCATCTCCAGAGACAATTCCAAGAGCACGC TGTATCTGCAAATGAACAGCCTGAGAGCCGAGG ACACGGCTGTGTATTACTGTGTGAGAGAGCTAGG CTTCCAGTCTGATCACTGGGGCCAGGGAACCCCG GTCACCGTCTCCTCA |
| | | | SEQ ID NO: 24307 | SEQ ID NO: 28313 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGLVQPGRSLRLSCAASGFTFSNYGMH WVRQAPGKGLEWVAVIWYEGSNKYYVDSVRGRF TISRDNSKSTLYLQMNSLRAEDTAVYYCVRELGFQ SDHWGQGTPVTVSS |
| | | | SEQ ID NO: 24308 | SEQ ID NO: 28314 |

FIGURE 50

| iPS:392702 | 21-225_17F7 | NA | GACATCCAGATGACCCAGTCTCCATCCTCCCT GTCTGCATCTGTTGGAGACAGAGTCACCATCA CTTGCCGGGCAAGTCAGACCATTAGTAGTTTT TTAAATTGGTATCAGCAGAAACCAGGGAAAG CCCCTAAACTCCTGATCTATGCTGCTTCCAGTT TGCAAAGTGGGGTCCCGTCAAGGTTCAGTGGC AGTGGATCTGGGACAGATTTCACTCTCACCAT CAGCGGTCTGCAACCTGAAGATTTTGCAACTT ACTTCTGTCAACAGAGTTACAGAACCCCCTTT TTCACTTTCGGCCCTGGGACCAAAGTGGATAT CAAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAACTGGGTCCGCCAGGCTCCAGGGAAGGGGCT GGAGTGGGTCTCAATTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTTTGGCA GTGGCTGGCTCGGAGGCTTTTGATATCTGGGGCC AAGGGACAATGGTCACCGTCTCTTCA |
| | | | SEQ ID NO: 24309 | SEQ ID NO: 28315 |
| | | AA | DIQMTQSPSSLSASVGDRVTITCRASQTISSFLNW YQQKPGKAPKLLIYAASSLQSGVPSRFSGSGSGT DFTLTISGLQPEDFATYFCQQSYRTPFFTFGPGTK VDIK | EVQLLESGGGLVQPGGSLRLSCAASEFTFSSYAMN WVRQAPGKGLEWVSIISGRGGNTFYADSVKGRFTI SRDNSKNTLYLQMNSLRAEDTAVYYCASRLAVAG SEAFDIWGQGTMVTVSS |
| | | | SEQ ID NO: 24310 | SEQ ID NO: 28316 |
| iPS:392704 | 21-225_17F11 | NA | GACATCCAGATGACCCAGTCTCCATCCTCCCT GTCTGCATCTATAGGAGACAGAGTCTCCATCA CTTGCCGGGCAAGTCGGACCATTAACAACTAT TTAAATTGGTATCAGCAGAAACCAGGGAAAG CCCCTAAACTCCTGATCTTTGCTACATCCAGTT TACAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGACTTACAGTACCCCCTTA TTCGCTTTCGGCCCTGGGACCAAAGTGGATAT CAAA | GAGGCGCAGCTGTTGGAGTCTGGGGGAGGCTTG GAACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTAGAGGTGGT AACACATACTCCGCAGACTCCGTGAAGGGCCGG TTCACCATCTCCAGAGACAATTCCAAGAACACGC TATATCTACAAATGAACAGCCTGAGAGCCGAGG ACACGGCCGTATATTACTGTGCGTCCCGTTTAGC AGTGGCTGGCTCGGAGGCTTTTCATATCTGGGGC CAAGGGACAATGGTCACCGTCTCTTCA |
| | | | SEQ ID NO: 24311 | SEQ ID NO: 28317 |

FIGURE 50

| | | | | |
|---|---|---|---|---|
| | | AA | DIQMTQSPSSLSASIGDRVSITCRASRTINNYLNW YQQKPGKAPKLLIFATSSLQSGVPSRFSGSGSGT DFTLTISSLQPEDFATYYCQQTYSTPLFAFGPGTK VDIK | EAQLLESGGGLEQPGGSLRLSCAASGFTFSSYAMS WVRQAPGKGLEWVSVISGRGGNTYSADSVKGRFTI SRDNSKNTLYLQMNSLRAEDTAVYYCASRLAVAG SEAFHIWGQGTMVTVSS |
| | | | SEQ ID NO: 24312 | SEQ ID NO: 28318 |
| iPS:392706 | 21-225_18A3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGTTGCATCCAGTT TGCAAAGTGGGGTCCCATCAAGGTTCAACGGC AGTGGATCTGGGACAGAATTCACTCTCACAAT CAGCAGCCTGCAGCCTGAAGATTTTGCAACTT ATTACTGTCTACAGCATAGTAGTTACCCGCTC ACTTTCGGCGGAGGGACCAAGGTGGAGATCA AA | CAGCTGCAGCTGCAGGAGTCGGGCCCAGGACTG GTGAAGTTTTCGGAGACCCTGTCCCTCACCTGCA CTGTCTCTGGTGGCTCCATCAGCAGAAGTAGTTA TTACTGGGGCTGGATCCGCCAGCCCCCAGGGAA GGGGCTGGAGTGGATAGGGAATATCTATTATAGT GGGTATACCTACTACACTCCGTCCCTCAAGAGTC GAGTCACCATATCCGTAGACACGTCCAAGAACC AGTTCTCCCTGAAGCTGAGCTCTGTGACCGCCGC AGACACGGCTGTGTATTACTGTGCGAGACATAGC ACCAGCTGGTCCCTTGACTACTGGGGCCAGGGAA CCCTGGTCACCGTCTCCTCA |
| | | | SEQ ID NO: 24313 | SEQ ID NO: 28319 |
| | | AA | DIQMTQSPSSLSASVGDRVTITCRASQGIRNDLG WYQQKPGKAPKRLIYVASSLQSGVPSRFNGSGS GTEFTLTISSLQPEDFATYYCLQHSSYPLTFGGGT KVEIK | QLQLQESGPGLVKFSETLSLTCTVSGGSISRSSYYW GWIRQPPGKGLEWIGNIYYSGYTYYTPSLKSRVTIS VDTSKNQFSLKLSSVTAADTAVYYCARHSTSWSLD YWGQGTLVTVSS |
| | | | SEQ ID NO: 24314 | SEQ ID NO: 28320 |

1464

FIGURE 50

| iPS:392708 | 21-225_18D11 | NA | GACATCCAGATGACCCAGTCTCCTTCCTCACT GTTTGCATTTGTAGGAGACAGAGTCACCATCA CTTGTCGGGCGAGTCAGGGCATTAGCTATTAT TTAGCCTGGTTTCAGCAGAAACCAGGGAAAGC CCCTAAGTCCCTGATTTATGTTGCATCCAGTTT GCAAAGTGGGGTCCCATCAAAGTTCAGCGGCA GTGGATTTGGGACAGATTTCACTCTCACCATC AGCAGCCTGCAGCCTGAAGATTTTGCAACTTA TTACTGCCAACAGTATAATACTTACCCATTCA CTTTCGGCCCTGGGACCACAGTGGATATCAAG | GAGGTGCAGCTGGTGGAGTCTGGGGGAGGCTTG GTACAGCCTGGGGGGTCCCTGAGACTCTCCTGTG CAGCCTCTGGATTCACCTTCAGAAGCTATAGCAT GAACTGGGTCCGCCAGGCTCCAGGGAAGGGGCT GGAGTGGCTTTCATACATTAGTAGTAGTAGTGGT ACCATATACTACGCAGACTCTGTGAAGGGCCGAT TCACCATCTCCAGAGACAACGCCAGGAACTCACT GAATCTGCAAATGAACAGCCTGAGAGACGAGGA CACGGCTGTGTATTACTGTGCGAGAGGAGGTGGG AGCCCTTTTGACTACTGGGGCCAGGGAATCCTGG TCACCGTCTCCTCA |
|---|---|---|---|---|
| | | | SEQ ID NO: 24315 | SEQ ID NO: 28321 |
| | | AA | DIQMTQSPSSLFAFVGDRVTITCRASQGISYYLA WFQQKPGKAPKSLIYVASSLQSGVPSKFSGSGFG TDFTLTISSLQPEDFATYYCQQYNTYPFTFGPGTT VDIK | EVQLVESGGGLVQPGGSLRLSCAASGFTFRSYSMN WVRQAPGKGLEWLSYISSSSGTIYYADSVKGRFTIS RDNARNSLNLQMNSLRDEDTAVYYCARGGGSPFD YWGQGILVTVSS |
| | | | SEQ ID NO: 24316 | SEQ ID NO: 28322 |
| iPS:392710 | 21-225_19A10 | NA | GACATCCAGATGACCCAGTCTCCATCCTCCCT GTCTGCATCTGTAGGAGACAGAGTCACCATCA CTTGCCGGGCAAGTCAGGGCATTAGAACTGAT TTAGGCTGGTATCAGCAGAGACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGGCTGCAGCCTGAAGATTTTGCAACT TATTACTGTCTACAGCATAATGGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGCGAGAGAGCTTGC CTGGTACGAGGACTCCTGGGGCCAGGGAACCCT GGTCACCGTCTCCTCA |
| | | | SEQ ID NO: 24317 | SEQ ID NO: 28323 |

FIGURE 50

| | | | | |
|---|---|---|---|---|
| | | AA | DIQMTQSPSSLSASVGDRVTITCRASQGIRTDLG WYQQRPGKAPKRLIYTASSLQSGVPSRFSGSGSG TEFTLTISRLQPEDFATYYCLQHNGYPWTFGQGT KVEIK | QVQLVESGGGVVQPGRSLRLSCAASGFTFSSYGMH WVRQAPGKGLEWVAVIWYDESNKYYADSVKGRF TISRDNSKNTLYLQMNSLRAEDTAVYYCARELAW YEDSWGQGTLVTVSS |
| | | | SEQ ID NO: 24318 | SEQ ID NO: 28324 |
| IPS:392714 | 21-225_16G12 | NA | GACATCCAGATGACCCAGTCTCCATCCTCACT GTCTGCATCTGTTGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGAGGC AGTGGATCTGGGACAGATTTCACTCTCACCAT CAGCAACCTGCAGCCTGAAGATTTTGCATCTT ATTACTGCCAACAGTATCATAGTTTCCCATTCA CTTTCGGCCCTGGGACCAAAGTGGATATCAAA | GAGGTGCAACTGTTGGAGTCGGGGGGAGGCTTG GTACAGCCTGGGGGGTCCCTGAGACTCTCCTGTG CAGCCTCTGGATTCTCCTTTAGTAGCTATGCCAT GACCTGGGTCCGCCAGGCTCCAGGGAAGGGGCT GGAGTGGGTCTCAACTATTAGTGGTCGTGGTGGT CACACATACTACGCAGACTCCGTGAGGGGCCGG TTCGCCATCTCCAGAGACAGTTCCAAGAACACGC TGTATCTGCAAATGAACAGCCTGAGAGCCGAGG ACACGGCCGTATATTACTGTGCGAAACAGGACTG CTGGGGCCAGGGAACCCTGGTCACCGTCTCCTCA |
| | | | SEQ ID NO: 24319 | SEQ ID NO: 28325 |
| | | AA | DIQMTQSPSSLSASVGDRVTITCRASQDISNYLA WFQQKPGKAPKSLIYAASSLQSGVPSKFRGSGSG TDFTLTISNLQPEDFASYYCQQYHSFPFTFGPGTK VDIK | EVQLLESGGGLVQPGGSLRLSCAASGFSFSSYAMT WVRQAPGKGLEWVSTISGRGGHTYYADSVRGRFA ISRDSSKNTLYLQMNSLRAEDTAVYYCAKQDCWG QGTLVTVSS |
| | | | SEQ ID NO: 24320 | SEQ ID NO: 28326 |

FIGURE 50

| | | | | |
|---|---|---|---|---|
| iPS:392716 | 21-225_17B5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAAT TTGCAAAGTGGGGTCCCATCAAGGTTCAGCGG CAGTGGATCTGGGACAGAATTCAGTTTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAAGT AATAAACACTATATAGACTCCGTGAAGGGCCGA TTCACCATCTCCAGAGACAATTCCAAGAACACGC TGTATCTGCAAATGAACAGCCTGAGAGCCGAGG ACACGGCTGTGTATTATTGCGTGAGAGAACTGGG GTTCCGGTTTGACTACTGGGGCCAGGGAACCCTG GTCACCGTCTCCTCA |
| | | | SEQ ID NO: 24321 | SEQ ID NO: 28327 |
| | | AA | DIQMTQSPSSLSASVGDRVTITCRASQGIRNDLG WYQQKPGKAPKRLIYAASNLQSGVPSRFSGSGS GTEFSFTISSLQPEDFATYYCLQHNSYPLTFGGGT KVEIK | QVQLVESGGGVVQPGRSLRLSCAASGFTFSDYGM HWVRQAPGKGLEWVAVIWYDESNKHYIDSVKGRF TISRDNSKNTLYLQMNSLRAEDTAVYYCVRELGFR FDYWGQGTLVTVSS |
| | | | SEQ ID NO: 24322 | SEQ ID NO: 28328 |
| iPS:392718 | 21-225_17B8 | NA | GATATTGTGATGACTCAGTCTCCACTCTCCCTG CCCGTCACCCCTGGAGAGCCGGCCTCCATCTC CTGTAGGTCTAGTCAGAGCCTCCTGCATAGTA ATGGGAACAACTCTTTGGATTGGTACCTGCAG AAGCCAGGGCAGTCTCCACAGCTCCTGATCTA TTTGGGTTCTCATCGGGCCTCCGGGGTCCCTG ACAGGTTCAGTGACAGTGGATCAGGCACAGAT TTTACACTGAAAATCAGCAGAGTGGAGGCTGA GGATGTTGGGGTTTATTACTGCATGCAAGTTC TACAAACTCCTCCCCTCACTTTCGGCGGAGGG ACCAAGGTGGAGATCAAA | CAGGTGCAGCTGGTGCAGTCTGGGGGCTGAGGTG AAGAAGCCTGGGGCCTCAGTGAAGGTCTCCTGC AAGGCGTCTGGATACACCTTCACCAGCTATGCTA TCAACTGGGTGCGACAGGCCACTGGACAAGGGC TTGAGTGGATGGGATGGATGAACCCTAACACTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTATATTTCTGTACGAGAAAGGCT GGGTTTGACTACTGGGGCCAGGGAACCCTGGTCA CCGTCTCCTCA |
| | | | SEQ ID NO: 24323 | SEQ ID NO: 28329 |

FIGURE 50

| | | | | |
|---|---|---|---|---|
| | | AA | DIVMTQSPLSLPVTPGEPASISCRSSQSLLHSNGN NSLDWYLQKPGQSPQLLIYLGSHRASGVPDRFS DSGSGTDFTLKISRVEAEDVGVYYCMQVLQTPP LTFGGGTKVEIK | QVQLVQSGAEVKKPGASVKVSCKASGYTFTSYAIN WVRQATGQGLEWMGWMNPNTGNTGYAQKFQGR VTMTRNTSISTAYMELSSLRSEDTAVYFCTRKAGF DYWGQGTLVTVSS |
| | | | SEQ ID NO: 24324 | SEQ ID NO: 28330 |
| iPS:392720 | 21-225_17A12 | NA | GACATCCAGATGACCCAGTCTCCATCCTCCCT GTCTGCATCTGTAGGAGACAGAGTCACCATCA CTTGCCGGGCAAGTCAGAGCATTAGTAGTTAT TTAAAATTGGTATCACCAGAAACCAGGGAAAGC CCCTAAGCTCCTGATCTATGCTGCATCCAGTTT GCAAAGTGGGGTCCCATCAAGGTTCAGTGGCA GTGGATCTGGGACAGATTTCACTCTCACCATC AGCAATCTGCAACCTGAAGATTTTGCAACTTA CTACTGTCAACAGAGTTACAATACCCCCTTAT TCACTTTCGGCCCTGGGACCAAAGTGGATATC AAA | GAGGTGCAGCTGTTGGAGTCTGGGGGAGGCTTG ATACAGCCTGGGGGGTCCCTGAGACTCTCCTGTG CAGCCTCTGAATTCACCTTTAGCAGCTATGCCAT GAGCTGGGTCCGCCAGGATCCAGGGAAGGGGCT GGAGTGGGTCTCAATTATTAGTGGTCGTGGGGGA AACGCATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTATAGCA GTGGCTGGCTCGGAGGCTTTTGATATCTGGGGCC AAGGGACAATGGTCACCGTCTCTTCA |
| | | | SEQ ID NO: 24325 | SEQ ID NO: 28331 |
| | | AA | DIQMTQSPSSLSASVGDRVTITCRASQSISSYLNW YHQKPGKAPKLLIYAASSLQSGVPSRFSGSGSGT DFTLTISNLQPEDFATYYCQQSYNTPLFTFGPGT KVDIK | EVQLLESGGGLIQPGGSLRLSCAASEFTFSSYAMSW VRQDPGKGLEWVSIISGRGGNAFYADSVKGRFTISR DNSKNTLYLQMNSLRAEDTAVYYCASRIAVAGSE AFDIWGQGTMVTVSS |
| | | | SEQ ID NO: 24326 | SEQ ID NO: 28332 |

FIGURE 50

| iPS:392722 | 21-225_18E12 | NA | GACATCCAGATGACCCAGTCTCCATCCTCCCT GTCTGCATCTGTAGGAGACAGAGTCACCATCA CTTGCCGGGCAAGTCAGAGCATTAGTAGTTAT TTAAAATTGGTATCAGCAGAAACCAGGGAAAG CCCCTACGCTCCTGATCTATGCTGCATCCAGTT TACAAAGTGGGGTCCCATCAAGGTTCAGTGGC AGTGGATCTGGGACAGATTTCACTCTCACCAT CAACAGTCTGCAACCTGAAGATTTTGCAACTT ACTTCTGTCAACAGAGTTACAGAACCCCCTTT TTCACTTTCGGCCCTGGGACCAAAGTGGATAT CAAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAGCTGGGTCCGCCAGGCTCCAGGGACGGGGCT GGAGTGGGTCTCAATTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTCTGGCA GTGGCTGGCTCGGAGGCTTTTGATATCTGGGGCC AAGGGACGATGGTCACCGTCTCTTCA |
| | | | SEQ ID NO: 24327 | SEQ ID NO: 28333 |
| | | AA | DIQMTQSPSSLSASVGDRVTITCRASQSISSYLNW YQQKPGKAPTLLIYAASSLQSGVPSRFSGSGSGT DFTLTINSLQPEDFATYFCQQSYRTPFFTFGPGTK VDIK | EVQLLESGGGLVQPGGSLRLSCAASEFTFSSYAMS WVRQAPGTGLEWVSIISGRGGNTFYADSVKGRFTI SRDNSKNTLYLQMNSLRAEDTAVYYCASRLAVAG SEAFDIWGQGTMVTVSS |
| | | | SEQ ID NO: 24328 | SEQ ID NO: 28334 |
| iPS:392726 | 21-225_20B5 | NA | GACATCCAGATGACCCAGTCTCCATCCTCCCT GTCTGCATCTGTAGGAGACAGAGTCACCATCA CTTGCCGGGCGAGTCAGGGCATTAACAATTAT TTAGCCTGGTATCAGCAGAAACCAGGGAAAAT TCCTAAGCTCCTGATCTATGCTGCATCCACTTT GCAATCAGGGGTCCCCTCTCGGTTCAGTGGCA GTGGATCTGGGACAGATTTCACTCTCACCATC AGCAGCCTGCAGCCTGAAGATGTTGCAACTTA TTGCTGTCAAAAGTATAACAGTGCCCCTCCGA TCACCTTCGGCCAAGGGACACGACTGGAGATT AAA | GAGGTGCAGCTGGTGGAGGCCGGGGGAGGCCTG GTCAAGCCGGGGGGGTCCCTGAGACTCTCCTGTG CAGCCTCTGGATTCACCTTCACCAGCTATAGCAT GAACTGGGTCCGCCAGGCTCCAGGGAAGGGGCT GGAGTGGGTCTCATCCATTAGTGGGAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATCGTGGG AGCTACTGGGGCCAGGGTACCCTGGTCACCGTCT CCTCA |
| | | | SEQ ID NO: 24329 | SEQ ID NO: 28335 |

FIGURE 50

| | | | | |
|---|---|---|---|---|
| | | AA | DIQMTQSPSSLSASVGDRVTITCRASQGINNYLA WYQQKPGKIPKLLIYAASTLQSGVPSRFSGSGSG TDFTLTISSLQPEDVATYCCQKYNSAPPITFGQGT RLEIK | EVQLVEAGGGLVKPGGSLRLSCAASGFTFTSYSMN WVRQAPGKGLEWVSSISGSSSYIYYADSVKGRFTIS RDNAKNSLYLQMNSLRAEDTAVYYCARDRGSYW GQGTLVTVSS |
| | | | SEQ ID NO: 24330 | SEQ ID NO: 28336 |
| iPS:392728 | 21-225_20F7 | NA | GACATCCAGATGACCCAGTCTCCATCTTCCGT GTCTGCATCTGTAGGAGACGGAGTCACCATCA CTTGTCGGGCGAGTCAGGGTATTAGCAGCTGG TTAGCCTGGTATCAGCAGAAACCAGGGAAAG CCCCTAAACTCTTGATTTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCTCGG ACGTTCGGCCAAGGGACCAAGGTGGAAATCA AA | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTATTACAT GAGCTGGATCCGCCAGGCTCCAGGGAAGGGGCT GGAGTGGGTTTCACACATTAGTAGTAGTGGTAGT ACCATATACTACGCAGACTCTGTGAAGGGCCGAT TCACCATCTCCAGGGACAACGGCGAGAACTCACT GTATCTGCAAATGAACAGCCTGAGAGCCGAGGA CACGGCCGTGTATTACTGTGCGAGATATCGGAAT AACCGGGGGTACTTCGATCTCTGGGGCCGTGGCT CCCTGGTCACTGTCTCCTCA |
| | | | SEQ ID NO: 24331 | SEQ ID NO: 28337 |
| | | AA | DIQMTQSPSSVSASVGDGVTITCRASQGISSWLA WYQQKPGKAPKLLIYAASSLQSGVPSRFSGSGS GTDFTLTISSLQPEDFATYYCQQANSFPRTFGQG TKVEIK | QVQLVESGGGLVKPGGSLRLSCAASGFTFSDYYMS WIRQAPGKGLEWVSHISSSGSTIYYADSVKGRFTIS RDNGENSLYLQMNSLRAEDTAVYYCARYRNNRG YFDLWGRGSLVTVSS |
| | | | SEQ ID NO: 24332 | SEQ ID NO: 28338 |

FIGURE 50

| iPS:392730 | 21-225_17A1 | NA | GACATCCAGATGACCCAGTCTCCATCCTCCCT GTCTGCATCTGTAGGAGACAGAGTCACCATCA CCTGCCGGGCAAGTCAGAACATTAACAATTAT TTAAATTGGTATCAGCAGAAACCAGGGAAAG GCCCTAAGGTCCTGATCTTTACTACATCTAGTT TACAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACACTACCCCCACG TGGACGTTCGGCCAAGGGACCAAGGTGGAAA TCAAA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGTTATTAGTGGTAGTGGTAGT AACACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAAAGATATAC CAGTGACTGGCATGATGCTTTTGATATCTGGGGC CAAGGGACAATGGTCACCGTCTCTTCA |
| | | | SEQ ID NO: 24333 | SEQ ID NO: 28339 |
| | | AA | DIQMTQSPSSLSASVGDRVTITCRASQNINNYLN WYQQKPGKGPKVLIFTTSSLQSGVPSRFSGSGSG TDFTLTISSLQPEDFATYYCQQSYTTPTWTFGQG TKVEIK | EVQLLESGGGLVQPGGSLRLSCAASGFTFSSYAMN WVRQAPGKGLEWVSVISGSGSNTYYADSVKGRFTI SRDNSKNTLYLQMNSLRAEDTAVYYCAKRYTSDW HDAFDIWGQGTMVTVSS |
| | | | SEQ ID NO: 24334 | SEQ ID NO: 28340 |
| iPS:392732 | 21-225_17E5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAGTTACCCGTTG ACGTTCGGCCCAGGGACCAAGGTGGTCATCAA A | CAGGTGCAACTGGTGGAGTCTGGGGGAGGCGTG GTCCAGCCTGGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCACTTATATGGTATGATGTAACT AATAAATACTATGGAGACTCCGTGAAGGGCCGA TTCACCATCTCCAGAGACAATTCCCAGAACACGC TGTATCTGCAACTGAACAGCCTGAGAGCCGAGG ACACGGCTGTGTATTACTGTGCGAGAGAGCTTGG CTGGTACGAGGACTACTGGGGCCAGGGAACCCT GGTCACCGTCTCCTCA |
| | | | SEQ ID NO: 24335 | SEQ ID NO: 28341 |

FIGURE 50

| | | | | |
|---|---|---|---|---|
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHNSYPLTFGPGTK VVIK | QVQLVESGGGVVQPGRSLRLSCAASGFTFSDYGM HWVRQAPGKGLEWVALIWYDVTNKYYGDSVKGR FTISRDNSQNTLYLQLNSLRAEDTAVYYCARELGW YEDYWGQGTLVTVSS |
| | | | SEQ ID NO: 24336 | SEQ ID NO: 28342 |
| IPS:392734 | 21-225_17D8 | NA | GAAATAGTGATGACGCAGTCTCCATCCACCCT GTCTGTGTCTCCAGGGGAAAGAGCCACCCTCT CCTGCAGGGCCAGTCAGAGTGTTAGCAGCAAC TTAGCCTGGTTCCAGCAGAAACCTGGCCAGGC TCCCAGGCTCCTCATCAATGGTGCATCCACCA GGGCCAGTGGTATCCCAGCCAGGTTCAGTGGC AGTGGGTCTGGGACAGAGTTCACTCTCACCAT CAGCAGCCTGCAGTCTGAAGATTTTGCAGTTT ATTACTGTCAGCAGTATAATAACTGGCCTCTG ACGTTCGGCCAAGGGACCAAGGTGGAAATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATGGCTT GAACTGGGTCCGCCAGGCTCCAGGGAAGGGGCT GGAGTGGGTCTCATCCATTAGTGGTAGTGGTAGT CACATATCCTACGCGGGACTCAGTGAAGGGCCGAT TCACCATCTCCAGAGACAACGCCAAGAACTCACT GTATCTGCAACTGAACAGCCTGAGAGCCGAGGA CACGGCTGTGTATTACTGTGCGAGAGATCGGGGC AGTGGCTGGGGCCAGGGAACCCTGGTCACCGTCT CCTCA |
| | | | SEQ ID NO: 24337 | SEQ ID NO: 28343 |
| | | AA | EIVMTQSPSTLSVSPGERATLSCRASQSVSSNLA WFQQKPGQAPRLLINGASTRASGIPARFSGSGSG TEFTLTISSLQSEDFAVYYCQQYNNWPLTFGQGT KVEIK | EVQLVESGGGLVKPGGSLRLSCAASGFTFSSYGLN WVRQAPGKGLEWVSSISGSGSHISYADSVKGRFTIS RDNAKNSLYLQLNSLRAEDTAVYYCARDRGSGWG QGTLVTVSS |
| | | | SEQ ID NO: 24338 | SEQ ID NO: 28344 |

FIGURE 50

| | | | | |
|---|---|---|---|---|
| iPS:392736 | 21-225_17B12 | NA | GACATCCAGATGACCCAGTCTCCATCCTCCCT GTCTGCATCTGTAGGAGACAGAGTCTCCATCA CTTGCCGGGCAAGTCAGAATATTAACAACTAT TTAAATTGGTATCAGCAGAAACCAGGGAAAG GCCCTAAGGTCCTGATCCTTACTGCATCCAGTT TGCAAAGTGGGGTCCCATCAAGGTTCAGTGGC AGTGGATCTGGGACAGATTTCACTCTCACCAT CAGCAGTCTACAACCTGAAGATTTTGCAACTT ACTACTGTCAACAGACTTACACTACCCCCACG TGGACGTTCGGCCAAGGGACCAAGGTGGAAA TCAAA | GAGGTGCAGCTGTTGGAGTCTGGGGGAGGCTTG GTACAGCCTGGGGGGTCCCTGAGACTCTCCTGTG TAGCCTCTGGATTCACCTTTAGCAGCTATGCCAT GAACTGGGTCCGCCAGGCTCCAGGGAAGGGGCT GGAGTGGGTCTCAGTTATTAGTGGTAGTGGTGGT AGCACATACTACGCAGACTCCGTGAAGGGCCGG TTCACCATCTCCAGAGACAATTCCAAGAACACGC TGTATCTGCAAATGAACACCCTGAGAGCCGAGG ACACGGCCGTATATTACTGTGCGAAAAGGTATAC CAGTGACTGGCATGATGCTTTTGATATCTGGGGC CAAGGGACAATGGTCACCGTCTCTTCA |
| | | | SEQ ID NO: 24339 | SEQ ID NO: 28345 |
| | | AA | DIQMTQSPSSLSASVGDRVSITCRASQNINNYLN WYQQKPGKGPKVLILTASSLQSGVPSRFSGSGSG TDFTLTISSLQPEDFATYYCQQTYTTPTWTFGQG TKVEIK | EVQLLESGGGLVQPGGSLRLSCVASGFTFSSYAMN WVRQAPGKGLEWVSVISGSGGSTYYADSVKGRFTI SRDNSKNTLYLQMNTLRAEDTAVYYCAKRYTSDW HDAFDIWGQGTMVTVSS |
| | | | SEQ ID NO: 24340 | SEQ ID NO: 28346 |
| iPS:392738 | 21-225_18G4 | NA | GACATCCACATGACCCAGTCTCCATCCTCCCT GTCTGCATCTGTAGGAGACAGAGTCACCATCA CTTGCCGGGCAAGTCAGAGCATTATCAGCTAT TTAAATTGGTATCAGCAGAAACCAGGGAAAG CCCCTAAGGTCCTGATCTATACTGCATCCAGTT TGCAAACTGGGGTCCCATCAGGGTTCAGTGGC AGTGGATCTGGGACAGATTTCACTCTCACCAT CAGCAGTCTGCAACCTGAAGATTTTGCAACTT ACTACTGTCAACAGACTTACAGTCCCCCGCTC ACTTTCGGCGGTGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCTGAG TATGGGCGGTATGGACGTCTGGGGCCAAGGGAC CACGGTCACCGTCTCCTCA |
| | | | SEQ ID NO: 24341 | SEQ ID NO: 28347 |

FIGURE 50

| | | | | |
|---|---|---|---|---|
| | | AA | DIHMTQSPSSLSASVGDRVTITCRASQSIISYLNW YQQKPGKAPKVLIYTASSLQTGVPSGFSGSGSGT DFTLTISSLQPEDFATYYCQQTYSPPLTFGGGTK VEIK | QVQLVESGGGVVQPGRSLRLSCAASGFTFSSYGMH WVRQAPGKGLEWVAIIWYDGSNKYYADSVKGRFT ISRDNSKNTLYLQMNSLRAEDTAVYYCARDLSMG GMDVWGQGTTVTVSS |
| | | | SEQ ID NO: 24342 | SEQ ID NO: 28348 |
| iPS:392740 | 21-225_18H12 | NA | GACATCCAGATGACCCAGTCTCCATCCTCCCT GTCTGCGTCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAAT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAATTACCCGTG GACGTTCGGCCTAGGGACCAAGGTGGTCATCA AA | CAGGTGCAACTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGATGGCAGTTATATGGTATGATGTAACT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTTTATTACTGTGCGAGAGAGGTTGG CTGGTACGAGGACTACTGGGGCCAGGGAACCCT GGTCACCGTCTCCTCA |
| | | | SEQ ID NO: 24343 | SEQ ID NO: 28349 |
| | | AA | DIQMTQSPSSLSASVGDRVTITCRASQGIRNDLG WYQQKPGKAPKRLIYTASNLQSGVPSRFSGSGS GTEFTLTISSLQPEDFATYYCLQHNNYPWTFGLG TKVVIK | QVQLVESGGGVVQPGRSLRLSCAASGFTFSDYGM HWVRQAPGKGLEWMAVIWYDVTNKYYADSVKG RFTISRDNSKNTLYLQMNSLRAEDTAVYYCAREVG WYEDYWGQGTLVTVSS |
| | | | SEQ ID NO: 24344 | SEQ ID NO: 28350 |

FIGURE 50

| iPS:392742 | 21-225_20B2 | NA | GACATCCAGATGACCCAGTCTCCATCCTCCCT GTCTGCCTCTGTAGGAGACAGAGTCAATATCA CTTGCCGGGCAAGTCAGGACATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACGA TCAGCAGCCTGCAGCCTGAAGATTTTGCAACT TATTACTGTCTACAGCATTATAATTACCCTCGG GCGTTCGGCCAGGGGACCAAGGTGGATATCA AA | CAGGTGCAACTGGTGGAGTCTGGGGGAGGCGTG GTCCAGCCTGGGAGGTCCCTGAGACTCTCCTGTG CAGCGTCTGGATTCACCTTCAGTAATTATGTCATT CACTGGGTCCGCCAGGCTCCAGGCAAGGGACTG GAGTGGGTGGCAGTTATATGGTATGATGGAAGTA ATAAATACTATGCAGACTCCGTGAAGGGCCGCTT CACCATCTCCAGAGACAATTCCAAGAACACGCTG TATCTGCAAATGAACAGCCTGAGAGCCGAGGAC ACGGCTGTGTATTCCTGTGCGAGAGAGAAGTATA GCAGCAGCTGGTACGACTACGGTATGGACGTCTG GGGCCAAGGGACCACGGTCACCGTCTCCTCA |
|---|---|---|---|---|
| | | | SEQ ID NO: 24345 | SEQ ID NO: 28351 |
| | | AA | DIQMTQSPSSLSASVGDRVNITCRASQDIRNDLG WYQQKPGKAPKRLIYAASSLQSGVPSRFSGSGS GTEFTLTISSLQPEDFATYYCLQHYNYPRAFGQG TKVDIK | QVQLVESGGGVVQPGRSLRLSCAASGFTFSNYVIH WVRQAPGKGLEWVAVIWYDGSNKYYADSVKGRF TISRDNSKNTLYLQMNSLRAEDTAVYSCAREKYSS SWYDYGMDVWGQGTTVTVSS |
| | | | SEQ ID NO: 24346 | SEQ ID NO: 28352 |
| iPS:392744 | 21-225_20D5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATTTGGTATGAAGAAAAT AATCAATACTATGCAGACTCCGTGAAGGGCCGAT TCACCATCTCCAGAGACAATTCCAAGAACACGCT GTATCTGCAAATGAACAGCCTGAGAGCCGACGA CACGGCTGTGTATTACTGTGCGAGAGAACTGGGG TTCCGGTCTGACTACTGGGGCCAGGGAACCCTGG TCACCGTCTCCTCA |
| | | | SEQ ID NO: 24347 | SEQ ID NO: 28353 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGGGT KVEIK | QVQLVESGGGVVQPGRSLRLSCAASGFTFSSYGMH WVRQAPGKGLEWVAVIWYEENNQYYADSVKGRF TISRDNSKNTLYLQMNSLRADDTAVYYCARELGFR SDYWGQGTLVTVSS |
| | | | SEQ ID NO: 24348 | SEQ ID NO: 28354 |
| IPS:392746 | 21-225_20H7 | NA | GACATCCAGATGACCCAGTCTCCATCCTCACT GTCTGCATCTGTAGGAGACAGAGTCACCATCA CTTGTCGGACGAGTCAGGGCATTAACAATTAT TTAGTCTGGTTTCAGCAGAAACCAGGGAAAGC CCCTAAGCGCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATTATAGTTACCCATTCAC TTTCGGCCCTGGGACCAAAATGGATTTCAAA | GAGGTGCACCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TTCATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTAGCAGCT CTTGACTACTGGGGCCAGGGAACCCTGGTCACCG TCTCCTCA |
| | | | SEQ ID NO: 24349 | SEQ ID NO: 28355 |
| | | AA | DIQMTQSPSSLSASVGDRVTITCRTSQGINNYLV WFQQKPGKAPKRLIYAASSLQSGVPSKFSGSGSG TDFTLTISSLQPEDFATYYCQQYYSYPFTFGPGT KMDFK | EVHLVESGGGLVKPGGSLRLSCAASGFTFSSYSMN WVRQAPGKGLEWVSSISGSSSFIYYADSVKGRFTIS RDNAKNSLYLQMNSLRAEDTAVYYCARVAALDY WGQGTLVTVSS |
| | | | SEQ ID NO: 24350 | SEQ ID NO: 28356 |

FIGURE 50

| | | | | |
|---|---|---|---|---|
| iPS:392748 | 21-225_20A8 | NA | GACATCCAGATGACCCAGTCTCCATCCTCACT GTCTGCATCTGTAGGAGACAGAGTCACCATCA CTTGTCGGGCGAGTCAGGGCATTAATAATTAT TTAGTCTGGTTTCAGCAGAAACCAGGGAAAGC CCCTAAGTCCCTGATCTATGCTGCATCCAGTTT GCTGAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCGATCA CCTTCGGCCAAGGGACACGACTGGAGATTAAA | GAGGTGCAGCTGGTGGAGTCTGGGGGAGGCCTG GTCAAGCCTGGGGGGTCCCTAAGACTCTCCTGTG CAGCCTCTGGATTCACCTTCAGTAGCTATAGCGT GAACTGGGTCCGCCGGGCTCCAGGGAAGGGGCT GGAGTGGGTCTCATCCATTAGTAGTAGTAGTAGT TTCCTATACTACGCAGACTCAGTGAAGGGCCGAT TCACCATCTCCAGAGACAACGCCAAGAACTCAGT GTATCTGCAAATGAACAGCCTGAGAGCCGAGGA CACGGCTGTATATTACTGTGCGAGAAACTGGGAC TACTGGGGCCAGGGAACCCTGGTCACCGTCTCCT CA |
| | | | SEQ ID NO: 24351 | SEQ ID NO: 28357 |
| | | AA | DIQMTQSPSSLSASVGDRVTITCRASQGINNYLV WFQQKPGKAPKSLIYAASSLLSGVPSKFSGSGSG TDFTLTISSLQPEDFATYYCQQYNSYPITFGQGTR LEIK | EVQLVESGGGLVKPGGSLRLSCAASGFTFSSYSVN WVRRAPGKGLEWVSSISSSSSFLYYADSVKGRFTIS RDNAKNSVYLQMNSLRAEDTAVYYCARNWDYW GQGTLVTVSS |
| | | | SEQ ID NO: 24352 | SEQ ID NO: 28358 |
| iPS:392750 | 21-225_20A10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACAAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACCA TCAGCAGCCTGCAGCCTGAAGATTTTGCAACT TATTACTGTCTACAGCATAATAGATACCCGCT CACTTTCGGCGGAGGGACCAAGGTGGAGATC AAA | CAGGTGCAGCTGGTGGAGTCTGGGGGAGGCGTG GTCCAGCCTGGGAGGTCCCTGAGACTCTCCTGTG CAGCGTCTGGATTCACCCTCAGTAGCGATGACAT GCACTGGGTCCGCCAGGCTCCAGGCAAGGGGCT GGAGTGGGTGGCAGTTATATGGTTTGATGGAAGT AATAAATACTATGCAGACTCCGTGAAGGGCCGA TTCATCATCTCCAGAGACAATTCCAAGAACACGC TGTATCTGCAAATGAACAGCCTGAGAGCCGAGG ACACGGCTATGTATTACTGTGCGAGAGATCTAAT AGCAGCAGCTGGTACGGTTGACTACTGGGGCCA GGGAACCCTGGTCACCGTCTCCTCA |
| | | | SEQ ID NO: 24353 | SEQ ID NO: 28359 |

FIGURE 50

| | | | | |
|---|---|---|---|---|
| | | AA | DIQMTQSPSSLSASVGDRVTITCRASQGIRNDLG WYQQKQGKAPKRLIYAASSLQSGVPSRFSGSGS GTEFTLTISSLQPEDFATYYCLQHNRYPLTFGGG TKVEIK | QVQLVESGGGVVQPGRSLRLSCAASGFTLSSDDMH WVRQAPGKGLEWVAVIWFDGSNKYYADSVKGRFI ISRDNSKNTLYLQMNSLRAEDTAMYYCARDLIAAA GTVDYWGQGTLVTVSS |
| | | | SEQ ID NO: 24354 | SEQ ID NO: 28360 |
| IPS:392754 | 21-225_21D3 | NA | GACATCCAGATGACCCAGTCTCCATCCTCCCT GTCTGCATCTGTAGGAGACAGAGTCACCATCA CTTGCCGGGCAAGTCAGAGCATTACTGGTTAT TCAAATTGGTATCAGCAGAAGCCAGGGAAAA CCCCTAAACTCCTGATCTTTGCTACATACAGTT TGGAAAGTGGGGTCCCATCAAGGTTCAGTGGC AGTGGATTTGGGACAAATTTCACTCTCACCAT CACCAGTCTGCAACCTGAAGATTTTGCAACTT ACTACTGTCAACAGAGTTACAGTACCTCGATC ACCTTCGGCCAAGGGACACGACTGGAGATTAA A | CAGGTGCAGCTGGTGGAGTCTGGGGGAGGCGTG GTCCAGCCTGGGAGGTCCCTGAGACTCTCCTGTG TTGCCTCTGGATTCACCTTCAGTAGCTATGGCAT GCACTGGGTCCGCCAGGCTCCAGGCAAGGGGCT GGAGTGGGTGACAGTTATATCATATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGCGAGAGGGGTATG GTTCGGGGACCTCTGGGGGCCAGGGAACCCTGGTC ACCGTCTCCTCA |
| | | | SEQ ID NO: 24355 | SEQ ID NO: 28361 |
| | | AA | DIQMTQSPSSLSASVGDRVTITCRASQSITGYSN WYQQKPGKTPKLLIFATYSLESGVPSRFSGSGFG TNFTLTITSLQPEDFATYYCQQSYSTSITFGQGTR LEIK | QVQLVESGGGVVQPGRSLRLSCVASGFTFSSYGMH WVRQAPGKGLEWVTVISYDGSNKYYADSVKGRFT ISRDNSKNTLYLQMNSLRAEDTAVYYCARGVWFG DLWGQGTLVTVSS |
| | | | SEQ ID NO: 24356 | SEQ ID NO: 28362 |

FIGURE 50

| iPS:392758 | 21-225_21G11 | NA | GACATCCAGATGACCCAGTCTCCATCCTCCCT GTCTGCGTCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAATTACCCGTG GACGTTCGGCCTAGGGACCAAGGTGGTCATCA AA | CAGGTGCAACTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGATGGCAGTTATATGGTATGATGTAACT AATGAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTTTATTACTGTGCGAGAGAGCTTGG CTGGTACGAGGACTACTGGGGCCAGGGAACCCT GGTCACCGTCTCCTCA |
| | | | SEQ ID NO: 24357 | SEQ ID NO: 28363 |
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHNNYPWTFGLGT KVVIK | QVQLVESGGGVVQPGRSLRLSCAASGFTFSDYGM HWVRQAPGKGLEWMAVIWYDVTNEYYADSVKG RFTISRDNSKNTLYLQMNSLRAEDTAVYYCARELG WYEDYWGQGTLVTVSS |
| | | | SEQ ID NO: 24358 | SEQ ID NO: 28364 |
| iPS:392760 | 21-225_22G3 | NA | GACATCCAGATGACCCAGTCTCCATCCTCCCT GTCTGCATCTGTAGGAGACAGAGTCACCATCA CTTGCCGGGCAAGTCAGAGCATTAGTAATTAT TTAAATTGGTATCAGCAGAAACCAGGGAAAG CCCCTAAGCTCCTGATCTTTGCTGCTTCCAGTT TGCAAAGTGGGGTCCCATCAAGGTTCAGTGGC AGTGGATCTGGGACAGATTTCACTCTCACCAT CAGCGGTCTACAACCTGAAGATTTTGCAACTT ACTTCTGTCAACAGAGTTTCAGAACCCCCTTTT TCACTTTCGGCCCTGGGACCAAAGTGGATATC AAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAACTGGGTCCGCCAGGCTCCAGGGAAGGGGCT GGAGTGGGTCTCAATTATTAGTGGTCGTGGTGTT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTTTGGCA GTGGCTGGCTCGGAGGCTTTTGATATCTGGGGCC AAGGGACAATGGTCACCGTCTCTTCA |
| | | | SEQ ID NO: 24359 | SEQ ID NO: 28365 |

FIGURE 50

| | | | | |
|---|---|---|---|---|
| | | AA | DIQMTQSPSSLSASVGDRVTITCRASQSISNYLN WYQQKPGKAPKLLIFAASSLQSGVPSRFSGSGSG TDFTLTISGLQPEDFATYFCQQSFRTPFFTFGPGT KVDIK | EVQLLESGGGLVQPGGSLRLSCAASEFTFSSYAMN WVRQAPGKGLEWVSIISGRGVNTFYADSVKGRFTI SRDNSKNTLYLQMNSLRAEDTAVYYCASRLAVAG SEAFDIWGQGTMVTVSS |
| | | | SEQ ID NO: 24360 | SEQ ID NO: 28366 |
| iPS:392762 | 21-225_22G5 | NA | GACATCCAGATGACCCAGTCTCCATCCTCCCT GTCTGCATCTGTAGGAGACAGAGTCACCATCA CTTGCCGGGCAAGTCAGAACATTAGCAGCTAT TTAAAATTGGTATCAGCAGAAACAAGGGAAAG CCCCTAAGCTCCTGATCTATGCTGCATCCAGTT TGCAAAATGGGGTCCCATCAAGGTTCAGTGGC AGAGGATCTGGGACAGATTTCACTCTCACCAT CAGTAGTCTGCAACCTGAAGATTTTGCAACTT ACTACTGTCAACAGAGTTACAGAACCCCCTTA TTCACTTTCGGCCCTGGGACCAAGGTTGATTTC AAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGATGGGGCT GGAGTGGGTCTCAGTTATTAGTCGTAGTGGTGGT TACACATACTACGCGGACTCCGTGAAGGGCCGGT TCACCATCTCCAGAGACAATTCCAAGAACACGCT GTATCTGCAAATGAACAGCCTGAGAGCCGAGGA CACGGCCGTCTATTACTGTGCGTCCCGTTTAGCA GTGGCTGGCTCGGAGGCTTTTGATATCTGGGGCC AAGGGACACTGGTCACCGTCTCTTCA |
| | | | SEQ ID NO: 24361 | SEQ ID NO: 28367 |
| | | AA | DIQMTQSPSSLSASVGDRVTITCRASQNISSYLN WYQQKQGKAPKLLIYAASSLQNGVPSRFSGRGS GTDFTLTISSLQPEDFATYYCQQSYRTPLFTFGPG TKVDFK | EVQLLESGGGLVQPGGSLRLSCAASGFTFSSYAMN WVRQAPGMGLEWVSVISRSGGYTYYADSVKGRFT ISRDNSKNTLYLQMNSLRAEDTAVYYCASRLAVAG SEAFDIWGQGTLVTVSS |
| | | | SEQ ID NO: 24362 | SEQ ID NO: 28368 |

1480

FIGURE 50

| iPS:392764 | 21-225_22G10 | NA | GACATCCAGATGACCCAGTCTCCATCCTCCCT GTCTGCATCTGTAGGAGACAGAGTCACCATCA CTTGCCGGGCAAGTCAGAGCATTTTCAGCTAT TTAAATTGGTATCAGCAGAAACCAGGGAAAG CCCCTAAACTCCTGATCTATGCTGCATCCAGTT TACAAAGTGGGGTCCCATCAAGGTTCAGTGGC AGTGGATCTGGGACAGATTTCACTCTCACCAT CAGCAGTCTCCAACCTGAAGATTTTGCAACTT ACTTCTGTCAACAGAGTTTCAGAACCCCCTTA TTCACTTTCGGCCCTGGGACCAAGGTGGATTT CAAA | GAGGTGCAGCTGTTGGAGTCTGGGGGAGACTTG GTACAGCCTGGGGGGTCCCTGAGACTCTCCTGTA CAGCCTCTGGATTCACCTTTAGCAGCTATGCCAT GAACTGGGTCCGCCAGGCTCCAGGGAAGGGGCT GGAGTGGGTCTCAGTTATTAGTGGTAGTGGTGGT AACACATTCTACGCAGACTCCGTGAAGGGCCGAT TCACCATCTCCAGAGACAATTCCAAGAACACGCT GTTTCTGCACATGAACAGCCTGAGAGCCGAGGA CACGGCCGTTTATTACTGTGCGTCCCGTATGGCA GTGGCTGGCTCGGAGGCTTTTGATATCTGGGGCC AAGGGACAATGGTCACCGTCTCTTCA |
| | | | SEQ ID NO: 24363 | SEQ ID NO: 28369 |
| | | AA | DIQMTQSPSSLSASVGDRVTITCRASQSIFSYLNW YQQKPGKAPKLLIYAASSLQSGVPSRFSGSGSGT DFTLTISSLQPEDFATYFCQQSFRTPLFTFGPGTK VDFK | EVQLLESGGDLVQPGGSLRLSCTASGFTFSSYAMN WVRQAPGKGLEWVSVISGSGGNTFYADSVKGRFTI SRDNSKNTLFLHMNSLRAEDTAVYYCASRMAVAG SEAFDIWGQGTMVTVSS |
| | | | SEQ ID NO: 24364 | SEQ ID NO: 28370 |
| iPS:392766 | 21-225_23H4 | NA | GACATCCAGATGACCCAGTCTCCATCCTCCCT GTCTGCATCTGTAGGAGACAGAGTCACCATCA CTTGCCGGGCAAGTCAGAGCATTAGCAGGTAT TTAAATTGGTATCAGCAGAAACCAGGGAGAG CCCCTAAACTCCTGATCTGTTCTACATCCAGTT TGCAAAGTGGGGTCCCATCAAGGTTCAGTGGC AGTGGATCTGGGACAGATTTCACTCTCACCAT CAGCAGTCTCCAACCTGAAGATTTTGCAACTT ATTACTGTCAACAGAGTTACAGTACCCCCACG TGGACGTTCGGCCAAGGGACCAAGGTGGAAA TCAGA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGTTATTAGTGGTAGTGGTGGT ACCACATACTTCGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAAAGAAATAG CAGTGGCTGGCATGATGTTTTTGATATCTGGGGC CAAGGGACAAAGGTCACCGTCTCTTCA |
| | | | SEQ ID NO: 24365 | SEQ ID NO: 28371 |

FIGURE 50

| | | | | |
|---|---|---|---|---|
| | | AA | DIQMTQSPSSLSASVGDRVTITCRASQSISRYLN WYQQKPGRAPKLLICSTSSLQSGVPSRFSGSGSG TDFTLTISSLQPEDFATYYCQQSYSTPTWTFGQG TKVEIR | EVQLLESGGGLVQPGGSLRLSCAASGFTFSSYAMN WVRQAPGKGLEWVSVISGSGGTTYFADSVKGRFTI SRDNSKNTLYLQMNSLRAEDTAVYYCAKRNSSGW HDVFDIWGQGTKVTVSS |
| | | | SEQ ID NO: 24366 | SEQ ID NO: 28372 |
| IPS:392768 | 21-225_20B8 | NA | GAAATAGTGATGACGCAGTCTCCATCCACCCT GTCTGTGTCTCCAGGGGAAAGAGTCACCCTCT CTTGCAGGGCCAGTCAGAGTGTTAGCAGCAAC TTAGCCTGGTTTCAGCAGAAACCTGGCCAGGC TCCCAGGCTCCTCATCAATGGTGCATCCACCA GGGCCAGTGGTATCCCAGCCAGGTTCAGTGGC AGTGGGTCTGGGACAGAGTTCACTCTCACCAT CAGCAGCCTGCAGTCTGAAGATTTTGCAGTTT ATTACTGTCAGCAGTATAATAACTGTCCTCTG ACGTTCGGCCAAGGGACCAAGGTGGAAATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGTTATAGCAT GAACTGGGTCCGCCAGGCTCCAGGGAAGGGGCT GGAGTGGGTCTCATCCATCAGTGGCAGTGGTAGT CACATATACTACGCGGGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAGATCGGGG CAGTGGCTGGGGCCAGGGAACCCTGGTCACCGT CTCCTCA |
| | | | SEQ ID NO: 24367 | SEQ ID NO: 28373 |
| | | AA | EIVMTQSPSTLSVSPGERVTLSCRASQSVSSNLA WFQQKPGQAPRLLINGASTRASGIPARFSGSGSG TEFTLTISSLQSEDFAVYYCQQYNNCPLTFGQGT KVEIK | EVQLVESGGGLVKPGGSLRLSCAASGFTFSSYSMN WVRQAPGKGLEWVSSISGSGSHIYYADSVKGRFTIS RDNAKNSLYLQMNSLRAEDTAVYYCARDRGSGW GQGTLVTVSS |
| | | | SEQ ID NO: 24368 | SEQ ID NO: 28374 |

FIGURE 50

| iPS:392770 | 21-225_20C10 | NA | GACATCCACATGACCCAGTCTCCATCCTCCCT GTCTGCATCTGTGGGAGACAAAGTCTCCATCA CTTGCCGGGCAAGTCACCATATTAGCAACTAT TTAAATTGGTATCAGCAGAAACCAGGGAAAG GCCCTAAGGTCCTGATCCTTACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACACTACCCCCACG TGGACGTTCGGCCAAGGGACCAAGGTGGAAA TCAAA | GAGGTGCAGCTGTTGGAGTCTGGGGGAGGCTTG GTACAGCCTGGGGGGTCCCTGAGACTCTCCTGTG CAGCCTCTGGATTCACTTTTAGCAGCTATGCCAT GAACTGGGTCCGCCAGGCTCCAGGGAAGGGGCT GGAGTGGGTCTCAGTTATTAGTGGTAGTGGTGGT ACCACATACTACGCAGACTCCGTGAAGGGCCGG TTCACCATCTCCAGAGACAATTCCAAGAACACGC TGTATCTGCAAATGAACACCCTGAGAGCCGAGG ACACGGCCGTATATTACTGTGCGAAAAGGTATAC CAGTGACTGGCATGATGCTTTTGATATCTGGGGC CAAGGGACAATGGTCACCGTCTCTTCA |
| | | | SEQ ID NO: 24369 | SEQ ID NO: 28375 |
| | | AA | DIHMTQSPSSLSASVGDKVSITCRASHHISNYLN WYQQKPGKGPKVLILTASSLQSGVPSRFSGSGSG TDFTLTISSLQPEDFATYYCQQSYTTPTWTFGQG TKVEIK | EVQLLESGGGLVQPGGSLRLSCAASGFTFSSYAMN WVRQAPGKGLEWVSVISGSGGTTYYADSVKGRFTI SRDNSKNTLYLQMNTLRAEDTAVYYCAKRYTSDW HDAFDIWGQGTMVTVSS |
| | | | SEQ ID NO: 24370 | SEQ ID NO: 28376 |
| iPS:392772 | 21-225_20E12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTTTGCTGCATCCAGTT TACAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGTTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATGTGGTATGATGAAAGT AATAAACACTATGCAGACTCCGTGAAGGGCCGA TTCACCATCTCCAGAGACAATTCCAGGAACACGC TGTATCTGCAAATGAACAGCCTGAGAGCCGAGG ACACGGCTGTGTATTACTGTGCGAGAGAACTGGG GTTCCGGTTTGACTACTGGGGCCAGGGAACCCTG GTCACCGTCTCCTCA |
| | | | SEQ ID NO: 24371 | SEQ ID NO: 28377 |

FIGURE 50

| | | | | |
|---|---|---|---|---|
| | | AA | DIQMTQSPSSLSASVGDRVTITCRASQGIRNDLG WYQQKPGKAPKRLIFAASSLQSGVPSRFSGSGSG TEFTLTISSLQPEDFATYYCLQHNSYPFTFGGGTK VEIK | QVQLVESGGGVVQPGRSLRLSCAASGFTFSSYGMH WVRQAPGKGLEWVAVMWYDESNKHYADSVKGR FTISRDNSRNTLYLQMNSLRAEDTAVYYCARELGF RFDYWGQGTLVTVSS |
| | | | SEQ ID NO: 24372 | SEQ ID NO: 28378 |
| iPS:392774 | 21-225_21F3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGATTTCATTCTCACAA TCAGCAGCCTGCAGCCTGAAGATTTTGCAACT TATTACTGTCTACAGCATAGTAGTTACCCCCTC ACTTTCGGCGGAGGGACCAAGGTGGAGATCA AA | CAGCTGCAGCTGCAGGAGTCGGGCCCAGGACTG GTGAAGCCTGCGGAGACCCTGTCCCTCACCTGCA CTGTCTCTGGTGGCTCCATCAGCAGAAGTAGTTA CTACTGGGGCTGGATCCGCCAGCCCCCAGGGAA GGTGCTGGAGTGGATTGGGAGCATCTATTATAGT GGGAGCACCTACTACAACCCGTCCCTCAAGAGTC GAGTCACCATATCCGTCGACACGTCCAAGAACCA GTTCTCCCTGAAGCTGAGCTCTGTGACCGCCGCA GACACGGCTGAGTATTACTGTGCGAGCCTTAGCA GCAGCTGGGACTTCCAGCACTGGGGCCAGGGCA CCCTGGTCACCGTCTCCTCA |
| | | | SEQ ID NO: 24373 | SEQ ID NO: 28379 |
| | | AA | DIQMTQSPSSLSASVGDRVTITCRASQGIRNDLG WYQQKPGKAPKRLIYAASSLQSGVPSRFSGSGS GTDFILTISSLQPEDFATYYCLQHSSYPLTFGGGT KVEIK | QLQLQESGPGLVKPAETLSLTCTVSGGSISRSSYYW GWIRQPPGKVLEWIGSIYYSGSTYYNPSLKSRVTIS VDTSKNQFSLKLSSVTAADTAEYYCASLSSSWDFQ HWGQGTLVTVSS |
| | | | SEQ ID NO: 24374 | SEQ ID NO: 28380 |

FIGURE 50

| iPS:392776 | 21-225_21A12 | NA | GACATCCAGATGACCCAGTCTCCATCCTCACT GTCTGCATCTGTGGGAGACAGAGTCACCATCA CTTGTCGGGCGAGTCAAGGCATTAGCAAATAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCGTTCA GGTTTGGCCAGGGGACCAAGCTGGAGATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAATAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTCTGTATTACTGTGCGAGAGCGGCTGGC TTTGACTACTGGGGCCAGGGAACCCTGGTCACCG TCTCCTCA |
|---|---|---|---|---|
| | | | SEQ ID NO: 24375 | SEQ ID NO: 28381 |
| | | AA | DIQMTQSPSSLSASVGDRVTITCRASQGISKYLA WFQQKPGKAPKSLIYAASSLQSGVPSKFSGSGSG TDFTLTISSLQPEDFATYYCQQYNSYPFRFGQGT KLEIK | EVQLVESGGGLVKPGGSLRLSCAASGFTFNSYSMN WVRQAPGKGLEWVSSISGSSSYIYYADSVKGRFTIS RDNAKNSLYLQMNSLRAEDTALYYCARAAGFDY WGQGTLVTVSS |
| | | | SEQ ID NO: 24376 | SEQ ID NO: 28382 |
| iPS:392778 | 21-225_22H3 | NA | GACATCCAGATGACCCAGTCTCCATCCTCCCT GTCTGCATCTGTAGGAGACAGAGTCACCATCA CTTGCCGGGCAAGTCAGGACATTAGAAATAAT TTAGGCTGGTATCAGCAGAAACCAGGGAAAG CCCCTAAGCGCCTGATCTATCCTGCATCCAGTT TGCAAACTGGGGTCCCATCAAGGTTCAGCGGC AGTGGATCTGGGACAGAATTCACTCTCACAAT CAGCAGCCTGCAGCCTGAAGATTTTGCAACTT ATTTCTGTCTACAGGATAATAGTTACCCATTCA CTTTCGGCCCTGGGACCAAAGTGGATATCAAA | GCGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTTTTACTGTGCGAGAGATAGGGGC AGCCTCTGGGGCCAGGGAACCCTGGTCACCGTCT CCTCT |
| | | | SEQ ID NO: 24377 | SEQ ID NO: 28383 |

FIGURE 50

| | | | | |
|---|---|---|---|---|
| | | AA | DIQMTQSPSSLSASVGDRVTITCRASQDIRNNLG WYQQKPGKAPKRLIYPASSLQTGVPSRFSGSGSG TEFTLTISSLQPEDFATYFCLQDNSYPFTFGPGTK VDIK | AVQLVESGGGLVKPGGSLRLSCAASGFTFSSYSMN WVRQAPGKGLEWVSSISSSSSYIYYADSVKGRFTIS RDNAKNSLYLQMNSLRAEDTAVFYCARDRGSLWG QGTLVTVSS |
| | | | SEQ ID NO: 24378 | SEQ ID NO: 28384 |
| iPS:392780 | 21-225_22B7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAACGCCTGATCTATACTGCATCCAGT TTGCAAAGTGGGGTCCCATCAAGGTTCAGCGG CAGTGGTTCTGGGACAGAATTCACTCTCACAA TCAGCAGCCTGCAGCCTGAAGATTTTGCAACT TATTACTGTCTACAGCATAATACTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATTTGGTATGAAGAAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAAGTGGGG TTCCGGTCTGACTACTGGGGCCAGGGAACCCTGG TCACCGTCTCCTCA |
| | | | SEQ ID NO: 24379 | SEQ ID NO: 28385 |
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHNTYPLTFGGGT KVEIK | QVQLVESGGGVVQPGRSLRLSCAASGFTFSSYGMH WVRQAPGKGLEWVAVIWYEENNQYYADSVKGRF TISRDNSKNTLYLQMNSLRAEDTAVYYCAREVGFR SDYWGQGTLVTVSS |
| | | | SEQ ID NO: 24380 | SEQ ID NO: 28386 |

FIGURE 50

| | | | | |
|---|---|---|---|---|
| iPS:392782 | 21-225_22B12 | NA | GACATCCAGATGACCCAGTCTCCATCCTCACT GTCTGCGTCTGTAGGAGACAGAGTCACCATCA TTTGTCGGGCGAGTCAGGACATTAGCAATTAT TTAGCCTGGTTTCAGGAGAAACCAGGGAAAGC CCATAAGTCCCTGATCTATGGTGCATCCAGTTT GCGGAGTGGGGTCCCATCAAAGTTCAGCGGCA GTGGATCTGGGACAGATTTCAATCTCACCATC AGCAGCCTGCAGCCTGAAGATCTTGCAACTTA TTACTGCCAACAGTATCATAGTTACCCATTCA CTTTCGGCCCTGGGACCAAAGTGGATTTCAAA<br>SEQ ID NO: 24381 | GAGGTGCAGCTGGTGGAGTCTGGGGGAGGCCTG GTCAAGCCTGGGGGGTCCCTGAGACTCTCCTGTG CAGCCTCTGGATTCACCTTCAGCAGCTATAGCAT GAACTGGGTCCGCCAGGCTCCAGGGAAGGGGCT GGAGTGGGTCTCATCCATTAGTGGTAGTAGTAGT TACACATACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAGTAGCAGC CCTTGACTCCTGGGGCCAGGGAACCCTGGTCACC GTCTCCTCA<br>SEQ ID NO: 28387 |
| | | AA | DIQMTQSPSSLSASVGDRVTIICRASQDISNYLA WFQEKPGKAHKSLIYGASSLRSGVPSKFSGSGSG TDFNLTISSLQPEDLATYYCQQYHSYPFTFGPGT KVDFK<br>SEQ ID NO: 24382 | EVQLVESGGGLVKPGGSLRLSCAASGFTFSSYSMN WVRQAPGKGLEWVSSISGSSSYTYYADSVKGRFTI SRDNAKNSLYLQMNSLRAEDTAVYYCARVAALDS WGQGTLVTVSS<br>SEQ ID NO: 28388 |
| iPS:392784 | 21-225_23C7 | NA | GACATCCAGATGACCCAGTCTCCATCCTCACT GTCTGCATCTGTAGGAGACAGAGTCACCATCA CTTGCCGGGCGAGTCAGGGCATTGGCATTTAT TTAGCCTGGTTTCAGCAGAAACCAGGGAAAGC CCCTAAGTCCCTGATCTCTGCTGCATCCAGTTT GCAAAGTGGGGTCCCATCAAAGTTCAGCGGCA GTGGATCTGGGACAGAGTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA<br>SEQ ID NO: 24383 | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGTTAT GAGCTGGGTCCGCCAGGCTCCAGGGAAGGGGCT GGAGTGGGTCTCAGCTATGAGTGGTAGTGGTGGT AGCACATATTATGTAGACTCCGTGAAGGGCCGGT TCACCATCTCCAGAGACAATTCCAAGAACACGCT GTATCTGCAAATGAACAGCCTGAGAGCCGAGGA CACGGCCGTATATTATTGTGCGCGAACTGGGGTC TTTGACTACTGGGGCCAGGGAACCCTGGTCATCG TCTCCTCA<br>SEQ ID NO: 28389 |

1487

FIGURE 50

| | | | | |
|---|---|---|---|---|
| | | AA | DIQMTQSPSSLSASVGDRVTITCRASQGIGIYLA WFQQKPGKAPKSLISAASSLQSGVPSKFSGSGSG TEFTLTISSLQPEDFATYYCQQYNSYPFTFGPGTK VDIK | EVQLLESGGGLVQPGGSLRLSCAASGFTFSSYVMS WVRQAPGKGLEWVSAMSGSGGSTYYVDSVKGRF TISRDNSKNTLYLQMNSLRAEDTAVYYCARTGVFD YWGQGTLVIVSS |
| | | | SEQ ID NO: 24384 | SEQ ID NO: 28390 |
| iPS:392786 | 21-225_24E1 | NA | GACATCGTGATGACCCAGTCTCCAGACTCCCT GGCTGTGTCTCTGGGCGAGAGGGCCACCATCA ACTGCAAGTCCAGCCAGAGTGTTTTATACACC TCCAACAATAACAACTACTTAACTTGGTACCA GCAGAAACCTGGACAGCGTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TTTTATAGTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAGGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGACAAGCAGT GGCTGGGAGGTCTTTGACTACTGGGGCCAGGGA ACCCTGGTCACCGTCTCCTCA |
| | | | SEQ ID NO: 24385 | SEQ ID NO: 28391 |
| | | AA | DIVMTQSPDSLAVSLGERATINCKSSQSVLYTSN NNNYLTWYQQKPGQRPNLLIYWASTRESGVPD RFSGSGSGTDFTLTISSLQAEDVAVYYCQQFYST PPTFGQGTKVEIK | QVQLVQSGAEVKKPGASVKVSCKASGYTFTNYDIN WVRQATGQGLEWMGWMHPNSGNTGYAQRFQGR VTMTRNTSISTAYMELSSLRSEDTAVYYCATSSGW EVFDYWGQGTLVTVSS |
| | | | SEQ ID NO: 24386 | SEQ ID NO: 28392 |

FIGURE 50

| iPS:392788 | 21-225_20C8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGAAGAAACCAGGGAAAG CCCCTAAGCGCCTGATCAATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAAGATAATAGTTACCCGTTC ACTTTCGGCGGAGGGACCAAGGTGGAGATCA CA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGCCT GGAGTGGGTCTCATCCATTAGTGGCAGTAGTAGT TACATATACTACGCAGACTCAGTGAAGGCCCGAT TCACCATCTCCAGAGACAACGCCAAGAACTCACT GTATCTGCAAATGAACAGCCTGAGAGCCGAGGA CACGGCTGTGTATTACTGTGCGAGAGACAGAGG CAGTCTCTGGGGCCAGGGAACCCTGGTCACCGTC TCCTCA |
|  |  |  | SEQ ID NO: 24387 | SEQ ID NO: 28393 |
|  |  | AA | DIQMTQSPSSLSASVGDRVTITCRASQGIRNDLG WYQKKPGKAPKRLINAASSLQSGVPSRFSGSGS GTEFTLTISSLQPEDFATYYCLQDNSYPFTFGGGT KVEIT | EVQLVESGGGLVKPGGSLRLSCAASGFTFSSYSMN WVRQAPGKGLEWVSSISGSSSYIYYADSVKARFTIS RDNAKNSLYLQMNSLRAEDTAVYYCARDRGSLW GQGTLVTVSS |
|  |  |  | SEQ ID NO: 24388 | SEQ ID NO: 28394 |
| iPS:392790 | 21-225_20D10 | NA | GACATCCAGATGACCCAGTCTCCATCCTCCCT GTCTGCATTTGTAGGAGACAGAGTCACCATCA CTTGCCGGGCAAGTCAGGGCATTAGAAATGAT TTAGGCTGGTATCAGCAGAAACCAGGGAAAG CCCGTAAGCGCCTGATCTATGTTGCATACAGT TTGCAAAGTGGGGTCCCATCAAGGTTCAGCGG CAGTGGATATGGGACAGAATTCACTCTCACAA TCAGCAGCTTGCAGCCTGAAGATTTTGCAACT TATTACTGTATACAGCAAAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAGT AATAAATACTATGTAGACTCCGTGAAGGGCCGAT TCACCATCTCCAGAGACGATTCCAAGAACACTCT GTATCTGCAAATGAACAGCCTGAGAGCCGAGGA CACGGCTGTGTATTACTGCGCGAGAGATCTTGGC TGGACGGAAGAGTACTGGGGCCAGGGAACCCTG GTCACCGTCTCCTCA |
|  |  |  | SEQ ID NO: 24389 | SEQ ID NO: 28395 |

FIGURE 50

| | | | | |
|---|---|---|---|---|
| | | AA | DIQMTQSPSSLSAFVGDRVTITCRASQGIRNDLG WYQQKPGKARKRLIYVAYSLQSGVPSRFSGSGY GTEFTLTISSLQPEDFATYYCIQQNSYPWTFGQG TKVEIK | QVQLVESGGGVVQPGRSLRLSCAASGFTFSDYGM HWVRQAPGKGLEWVAVIWFDGSNKYYVDSVKGR FTISRDDSKNTLYLQMNSLRAEDTAVYYCARDLG WTEEYWGQGTLVTVSS |
| | | | SEQ ID NO: 24390 | SEQ ID NO: 28396 |
| IPS:392792 | 21-225_20G12 | NA | GACATCCAGATGACCCAGTCTCCATCCTCCCT GTCTGCATCTGTAGGAGACAGAGTCACCATCA CTTGCCGGGCGAGTCAGGGCATTAGCAATTAT TTAGCCTGGTATCAGCAGAAACCAGGGAAAGT TCCTAAGGTCTTGATCTATACTGCATCCACTTT GCAATCAGGGGTCCCATCTCGGTTCAGTGGCA GTGGATCTGGGACAGATTTCACTCTCACCGTC AGCAGCCTGCAGCCTGAAGATGTTGCAACTTA TTACTGTCAAAAGTATAACAGTGCCCCTCCGA TCACCTTCGGCCAAGGGACACGACTGGAGATT AAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGCGGTAGTAGTAGT TACATCTACTACGCAGACTCACTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATCGTGGG AGCTACTGGGGCCAGGGAACCCTGGTCACCGTCT CCTCA |
| | | | SEQ ID NO: 24391 | SEQ ID NO: 28397 |
| | | AA | DIQMTQSPSSLSASVGDRVTITCRASQGISNYLA WYQQKPGKVPKVLIYTASTLQSGVPSRFSGSGS GTDFTLTVSSLQPEDVATYYCQKYNSAPPITFGQ GTRLEIK | EVQLVESGGGLVKPGGSLRLSCAASGFTFSSYSMN WVRQAPGKGLEWVSSISGSSSYIYYADSLKGRFTIS RDNAKNSLYLQMNSLRAEDTAVYYCARDRGSYW GQGTLVTVSS |
| | | | SEQ ID NO: 24392 | SEQ ID NO: 28398 |

FIGURE 50

| iPS:392794 | 21-225_21H3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCGTCCAGT GTGCAAACTGGGGTCCCATCAAGGTTCAGCGG CAGTGGATCTGGGACAGAATTCACTCTCACAA TCAGCAGCCTGCAGGCTGAAGATTTGGCAATT TATTACTGTCTACAGCATAATAGTTATCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGGAGTAGTTA CTACTGGGGCTGGATCCGCCAGCCCCCAGGGAA GGGGCTGGAGTGGATTGGGAATATTTATTATAGT GGGAGCACCTACGACAACCCGTCCCTCAAGAGT CGAGTCACCATATCCGTAGACACGTCCAAGAACC AGTTCTCCCTGAAGCTGAGCTCTGTGACCGCCGC GGACACGGCTGTTTATTACTGTGGGAGACATGGA AAAGACTGGGGCCTTGACTACTGGGGCCAGGGA ACCCTGGTCACCGTCTCCTCA |
| | | | SEQ ID NO: 24393 | SEQ ID NO: 28399 |
| | | AA | DIQMTQSPSSLSASVGDRVTITCRASQGIRNDLG WYQQKPGKAPKRLIYAASSVQTGVPSRFSGSGS GTEFTLTISSLQAEDLAIYYCLQHNSYPLTFGGGT KVEIK | QLQLQESGPGLVKPSETLSLTCTVSGGSISRSSYYW GWIRQPPGKGLEWIGNIYYSGSTYDNPSLKSRVTIS VDTSKNQFSLKLSSVTAADTAVYYCGRHGKDWGL DYWGQGTLVTVSS |
| | | | SEQ ID NO: 24394 | SEQ ID NO: 28400 |
| iPS:392796 | 21-225_22A4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTTCAGCATAATAGTTACCCGTGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT ACACTGGGTCCGCCAGGCTCCAGGCAAGGGGCT GGAGTGGGTGGCAGTTATATGGTTTGATGGAAGT AATAAATACTATGCAGACTCCGTGAAGGGCCGA ATCACCATCTCTAGAGACAATTCCAAGAACACGC TGTATCTGCAAATGAACAGCCTGAGAGCCGAGG ACACGGCTGTGTATTACTGTGCGAGAGATCTTGG CTGGACGGAAGAGTACTGGGGCCAGGGAACCCT GGTCACCGTCTCCTCA |
| | | | SEQ ID NO: 24395 | SEQ ID NO: 28401 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPWTFGQG TKVEIK | QVQLVESGGGVVQPGRSLRLSCAASGFTFSDYGIH WVRQAPGKGLEWVAVIWFDGSNKYYADSVKGRIT ISRDNSKNTLYLQMNSLRAEDTAVYYCARDLGWT EEYWGQGTLVTVSS |
| | | | SEQ ID NO: 24396 | SEQ ID NO: 28402 |
| iPS:392798 | 21-225_22C7 | NA | GACATCCAGATGACCCAGTCTCCATCCTCCCT GTCTGCATCTGTAGGAGACAGAGTCACCATCA CTTGCCGGGCAAGTCAGAACATTATCAGCTAT TTAAATTGGTATCAGCAGAAACCAGAGAAAG CCCCTAAACTCCTGATCCATATTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGACTTACAGTACCCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGGGGGCAGTTATATGGCATGATGGAAG TAATAAATACTATGCAGACTCCGTGAAGGGCCG ATTCACCATCTCCAGAGACAATGCCAAGAACAC GCTGTATCTGCAAATGAACAGCCTGAGAGCCGA GGACACGGCTGTGTATTACTGTGCGAGAGATCTG AGTATGGGCGGTATGGACGTCTGGGGCCAAGGG ACCACGGTCACCGTCTCCTCA |
| | | | SEQ ID NO: 24397 | SEQ ID NO: 28403 |
| | | AA | DIQMTQSPSSLSASVGDRVTITCRASQNIISYLNW YQQKPEKAPKLLIHIASSLQSGVPSRFSGSGSGTD FTLTISSLQPEDFATYYCQQTYSTPLTFGGGTKV EIK | QVQLVESGGGVVQPGRSLRLSCAASGFTFSSYGMH WVRQAPGKGLEWGAVIWHDGSNKYYADSVKGRF TISRDNAKNTLYLQMNSLRAEDTAVYYCARDLSM GGMDVWGQGTTVTVSS |
| | | | SEQ ID NO: 24398 | SEQ ID NO: 28404 |

FIGURE 50

| iPS:392800 | 21-225_22D12 | NA | GACATCCAGATGACCCAGTCTCCATCCTCCCT GTCTGCATCTGTAGGAGACAGAGTCACCATCA CTTGTCGGGCAAGTCAGGGCATTAGAAATGAT TTAGGCTGGTATCAGCAGAAACCAGGGAAAG CCCCTAAGCGCCTGATCTATGCTGCATCCAGT TTGCAGAGTGGGGTCCCATCTAGGTTCAGCGG CAGTGGATCTGGGACAGATTTCACTCTCACAA TCAGCAGCCTGCAGCCTGAAGATTTTGCAACT TATTACTGTCTACAGCATAGTACTTACCCTCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGGAGTAGTTA CTACTGGGGCTGGATCCGCCAGCCCCCAGGGAA GGGGCTGGAGTGGATTGGAAATATCTATTATAGT GGGACCACCTCCTACAACCCGTCCCTCAAGAGTC GAGTCACCATATCCGTTGACACGTCCAGGAACCA GTTCTCCCTGAAGCTGAGCTCTGTGACCGCCGCA GACACGGCTGTGTTTTACTGTGCGAGACTCAGCA GCAGCTGGTCCGTTGACTACTGGGGCCAGGGAA CCCTGGTCACCGTCTCCTCA |
| | | | SEQ ID NO: 24399 | SEQ ID NO: 28405 |
| | | AA | DIQMTQSPSSLSASVGDRVTITCRASQGIRNDLG WYQQKPGKAPKRLIYAASSLQSGVPSRFSGSGS GTDFTLTISSLQPEDFATYYCLQHSTYPLTFGGG TKVEIK | QLQLQESGPGLVKPSETLSLTCTVSGGSISRSSYYW GWIRQPPGKGLEWIGNIYYSGTTSYNPSLKSRVTIS VDTSRNQFSLKLSSVTAADTAVFYCARLSSSWSVD YWGQGTLVTVSS |
| | | | SEQ ID NO: 24400 | SEQ ID NO: 28406 |
| iPS:392802 | 21-225_23E7 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGATACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTTTTATAGTTACCCATTCAC TTTCGGCCCTGGGACCAAAGTGGATATCAAT | GAGGTGCAGCTGTTGGAGTCTGGGGGAGGCTTG GTACAGCCTGGGGGGTCCCTGAGACTCTCCTGTA CAGCCTCTGGATTCACCTTTAGCAGCTATGCCAT GAACTGGGTCCGCCAGGCTCCAGGGAAGGGGCT GGAGTGGGTCTCAGCTATTAGTGGTAGTGGTGGT TTCACATACTACGCAGACTCCGTGAAGGGCCGGT TCACCATCTCCAGAGACAATTCCAGGAACACGCT GTATCTGCAAATGAACAGCCTGAGAGCCGAGGA CACGGCCGTATATTACTGTGCGCGTACCAGTGGC TTTGACTACTGGGGCCAGGGAACCCTGGTCACCG TCTCCTCA |
| | | | SEQ ID NO: 24401 | SEQ ID NO: 28407 |

FIGURE 50

| | | | | |
|---|---|---|---|---|
| | | AA | DIQMTQSPSSLSASVGDRVTITCRASQGISNYLA WFQQIPGKAPKSLIYAASSLQSGVPSKFSGSGSG TDFTLTISSLQPEDFATYYCQQFYSYPFTFGPGTK VDIN | EVQLLESGGGLVQPGGSLRLSCTASGFTFSSYAMN WVRQAPGKGLEWVSAISGSGGFTYYADSVKGRFTI SRDNSRNTLYLQMNSLRAEDTAVYYCARTSGFDY WGQGTLVTVSS |
| | | | SEQ ID NO: 24402 | SEQ ID NO: 28408 |
| IPS:392806 | 21-225_24H3 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCAAC TTAGCCTGGTACCAGCAGAAACCTGGCCAGGC TCCCAGGCTCCTCATCTATTTTGCATCCATCAG GGCCACTGGTATCCCAGCCAGGTTCAGTGGCA GTGGGTCTGGGACAGAGTTCACTCTCACCATC AGCAGCCTGCAGTCTGAAGATTTTGCAGTTTA TTACTGTCAGCAGTATAATAACTGGCCCATGT GCAGTTTTGGCCAGGGGACCAAGCTGGAGATC AAA | CAGGTGCAGCTGGTGGAGTCTGGGGGAGGCGTG GTCCAGCCTGGGAGGTCCCTGAGACTCTCCTGTG GAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGTAGCAGT GGCAGGCGGTATGGACGTCTGGGGCCAAGGGAC CACGGTCACCGTCTCCTCA |
| | | | SEQ ID NO: 24403 | SEQ ID NO: 28409 |
| | | AA | EIVMTQSPATLSVSPGERATLSCRASQSVSSNLA WYQQKPGQAPRLLIYFASIRATGIPARFSGSGSG TEFTLTISSLQSEDFAVYYCQQYNNWPMCSFGQ GTKLEIK | QVQLVESGGGVVQPGRSLRLSCGASGFTFSSYGMH WVRQAPGKGLEWVAVIWYDGSNKYYADSVKGRF TISRDNSKNTLYLQMNSLRAEDTAVYYCARVAVA GGMDVWGQGTTVTVSS |
| | | | SEQ ID NO: 24404 | SEQ ID NO: 28410 |

FIGURE 50

| iPS:392808 | 21-225_20F8 | NA | GACATCCAGATGACCCAGTCTCCATCCTCCCT GTCTGCATCTGTAGGAGACAGAGTCACCATCA CTTGCCGGGCAAGTCAGAGCATTAGCAGGTAT TTAAATTGGTATCAGCAGAAACCAGGGAAAG CCCCTGAGCTCCTGATCTATGCTGCTTCCAGTT TGCAAAGTGGGGTCCCATCAAGGTTCAGTGGC AGTGGATCTGGGACAGATTTCATTCTCACCAT CAGCAGTCTACAACCTGAAGATTTTGCAACTT ACTACTGTCAACAGAGTTACAATACCCCCACG TGGACGTTCGGCCAAGGGACCAAGGTGGAAA TCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGAAGCTATGCCAT GAGCTGGATCCGCCAGGCTCCAGGGAGGGGGCT GGAGTGGTCTTCAGTTATTAGTGGTAGTGGTGGT AGCACATATTACGCAGACTCCGTGAAGGGCCGG TTCACCATCTCCAGAGACAATTCCAAGAACACGC TGTATCTGCAAATGAGCAGCCTGAGAGCCGAAG ACACGGCCGTATATTACTGTGCGAAAAGGTATAA CAGTGGCTGGCATGATGTTTTTGATATCTGGGGC CAAGGGACAATGGTCACCGTCTCTTCA |
| | | | SEQ ID NO: 24405 | SEQ ID NO: 28411 |
| | | AA | DIQMTQSPSSLSASVGDRVTITCRASQSISRYLN WYQQKPGKAPELLIYAASSLQSGVPSRFSGSGSG TDFILTISSLQPEDFATYYCQQSYNTPTWTFGQG TKVEIK | EVQLLESGGGLVQPGGSLRLSCAASGFTFRSYAMS WIRQAPGRGLEWSSVISGSGGSTYYADSVKGRFTIS RDNSKNTLYLQMSSLRAEDTAVYYCAKRYNSGW HDVFDIWGQGTMVTVSS |
| | | | SEQ ID NO: 24406 | SEQ ID NO: 28412 |
| iPS:392810 | 21-225_20H12 | NA | GACATCCAGATGACCCAGTCTCCATCCTCCCT GTCTGCTTCTGTAGGAGACAGAGTCACCATCA CTTGCCGGGCAAGTCAGGGCATTAGAAATGAT TTAGACTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGAAAAT AATAAATACTATGTAGACTCCGTGAAGGGCCGAT TCACCATCTCCAGAGACAACTCCAAGAACACGCT GTATCTGCAAATGAACAGCCTGAGAGCCGAGGA CACGGCTGTGTATTACTGTGCGAGAGAGTTGGGG TTCCGGTCTGACTACTGGGGCCAGGGAACCCTGG TCACCGTCTCCTCA |
| | | | SEQ ID NO: 24407 | SEQ ID NO: 28413 |

FIGURE 50

| | | | | |
|---|---|---|---|---|
| | | AA | DIQMTQSPSSLSASVGDRVTITCRASQGIRNDLD WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSNYGM HWVRQAPGKGLEWVAVIWYDENNKYYVDSVKGR FTISRDNSKNTLYLQMNSLRAEDTAVYYCARELGF RSDYWGQGTLVTVSS |
| | | | SEQ ID NO: 24408 | SEQ ID NO: 28414 |
| iPS:392812 | 21-225_21F4 | NA | GACATCCAGATGACCCAGTCTCCATCCTCCCT GTCTGCATCTGTAGGAGACAGAGTCACCATCA CTTGCCGGGCAAGTCAGAACATTGGTAGTTAT TTAAATTGGTATCAGCAGAAACCAGGGAAAG CCCCTAAGCTCCTGATCTTTGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT TCTTCTGTCAACAGAGTTACAGAACCCCCTTTT TCACTTTCGGCCCTGGGACCAAAGTGGATTTC AAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAACTGGGTCCGCCAGGCTCCAGGGAAGGGGCT GGAGTGGGTCTCAGTTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTTTGGCA GTGGCTGGCTCGGAGGCTTGTGATATCTGGGGCC AAGGGACAATGGTCACCGTCTCTTCA |
| | | | SEQ ID NO: 24409 | SEQ ID NO: 28415 |
| | | AA | DIQMTQSPSSLSASVGDRVTITCRASQNIGSYLN WYQQKPGKAPKLLIFAASSLQSGVPSRFSGSGSG TDFTLTISSLQPEDFATFFCQQSYRTPFFTFGPGT KVDFK | EVQLLESGGGLVQPGGSLRLSCAASEFTFSSYAMN WVRQAPGKGLEWVSVISGRGGNTFYADSVKGRFTI SRDNSKNTLYLQMNSLRAEDTAVYYCASRLAVAG SEACDIWGQGTMVTVSS |
| | | | SEQ ID NO: 24410 | SEQ ID NO: 28416 |

FIGURE 50

| iPS:392814 | 21-225_22A1 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GGTGGAAAGACCTATTTATATTGGTACCTGCA GAAGCCAGGCCAGCCTCCACAGCTCCTGATCT ATGAAGTTTCCAACCGGTTCTCTGGACTGCCA GATAGGTTCAGTGGCAGCGGGTCAGGGACAG ATTTCACACTGAAAATCAGCCGGGTGGAGGCT GCGGATGTTGGGGTTTATTACTGCATGCAAAC TTTACACCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATGTGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGATGGGGGA TTTTTGGAGTGGTTAGACTACTGGGGCCAGGGAA CCCTGGTCACCGTCTCCTCA |
| | | | SEQ ID NO: 24411 | SEQ ID NO: 28417 |
| | | AA | DIVMTQTPLSLSVTPGQPASISCKSSQSLLHSGGK TYLYWYLQKPGQPPQLLIYEVSNRFSGLPDRFSG SGSGTDFTLKISRVEAADVGVYYCMQTLHLPWT FGQGTKVEIK | QVQLVESGGGVVQPGRSLRLSCAASGFTFSSYGMH WVRQAPGKGLEWVAVMWYDGSNKYYADSVKGR FTISRDNSKNTLYLQMNSLRAEDTAVYYCARDGGF LEWLDYWGQGTLVTVSS |
| | | | SEQ ID NO: 24412 | SEQ ID NO: 28418 |
| iPS:392816 | 21-225_22E4 | NA | GACATCCAGATGACCCAGTCTCCATCCTCCCT GTCTGCATCTGTAGGAGACAGAGTCACCATCA CTTGCCGGGCAAGTCAGAACATTAGTAGTTAT TTAAATTGGTATCAGCAGAAACCAGGGAAAG CCCCTAAGCTCCTGATCTATGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAATACCCCCTTA TTCACTTTCGGCCCTGGGACCAAAGTGGATAT CAAA | GAGGTGCAGCTGTTGGAGTCTGGGGGAGGCTTG GTGCAGCCTGGGGGGTCCCTGAGACTCTCCTGTG CAGCCTCTGAATTCACCTTTAGCAGCTATGCCAT GAGCTGGGTCCGCCAGGCTCCAGGGAAGGGGCT GGAGTGGGTCTCAATTATTAGTGGTCGTGGTACT AACACATTCTACGCAGACTCCGTGAAGGGCCGGT TCACCATCTCCAGAGTCAATTCCAAGAACACGCT GTATCTGCAAATGAACAGCCTGAGAGCCGAGGA CACGGCCGTATATTACTGTGCGTCCCGTATAGCA GTGGCTGGCTCGGAGGCTTTTGATATCTGGGGCC AAGGGACAATGGTCACCGTCTCTTCA |
| | | | SEQ ID NO: 24413 | SEQ ID NO: 28419 |

1497

FIGURE 50

| | | | | |
|---|---|---|---|---|
| | | AA | DIQMTQSPSSLSASVGDRVTITCRASQNISSYLN WYQQKPGKAPKLLIYAASSLQSGVPSRFSGSGS GTDFTLTISSLQPEDFATYYCQQSYNTPLFTFGPG TKVDIK | EVQLLESGGGLVQPGGSLRLSCAASEFTFSSYAMS WVRQAPGKGLEWVSIISGRGTNTFYADSVKGRFTI SRVNSKNTLYLQMNSLRAEDTAVYYCASRIAVAGS EAFDIWGQGTMVTVSS |
| | | | SEQ ID NO: 24414 | SEQ ID NO: 28420 |
| IPS:392818 | 21-225_22D8 | NA | GACATCCAGATGACCCAGTCTCCATCCTCCCT GTCTGCATCTGTAGGAGACAGAGTCACCATCA CTTGCCGGACAAGTCAGAACAGTAACAGTTAT TTAAATTGGTATCAGCAGAAACCAGGGAAAG CCCCTAAGCTCCAGATCTTTGCTGCATACAGTT TGGAAAGTGGGGTCCCATCAAGGTTCAGTGGC AATAGATCTGGGACAGAGTTCACTCTCACCAT CAGCAGTCTGCAACCTGAAGATTTTGCAACTT ACTACTGTCAACAGACTTACGGTACCTCGATC ACCTTCGGCCAAGGGACACGACTGGAGATTAA A | CAGGTGCAGCTGGTGGAGTCTGGGGGAGGCGTG GTCCAGCCTGGGAGGTCCCTGAGACTCTCCTGTG TAGCCTCTGGATTCACCTTCAGGAGCTATGGCAT GCACTGGGTCCGCCAGGCTCCAGGCAAGGGGCT GGAGTGGGTGACAATTATATCATATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTATATTTCTGTGCGAGAGGGGTTTG GTTCGGGGACTTCTGGGGCCAGGGAACCCTGGTC ACCGTCTCCTCA |
| | | | SEQ ID NO: 24415 | SEQ ID NO: 28421 |
| | | AA | DIQMTQSPSSLSASVGDRVTITCRTSQNSNSYLN WYQQKPGKAPKLQIFAAYSLESGVPSRFSGNRS GTEFTLTISSLQPEDFATYYCQQTYGTSITFGQGT RLEIK | QVQLVESGGGVVQPGRSLRLSCVASGFTFRSYGMH WVRQAPGKGLEWVTIISYDGSNKYYADSVKGRFTI SRDNSKNTLYLQMNSLRAEDTAVYFCARGVWFGD FWGQGTLVTVSS |
| | | | SEQ ID NO: 24416 | SEQ ID NO: 28422 |

FIGURE 50

| iPS:392820 | 21-225_23D1 | NA | GACATCCAGATGACCCAGTCTCCATCCTCCCT GTCTGCATCTGTAGGAGACAGAGTCACCATCA CTTGCCGGGCAAGTCAGGGCATCAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGTTCTGGGACAGAATTCACTCTCACAG TCAGCAGCCTGCAGCCTGAAGATTTTGCAACT TATTACTGTCTACAGCATAGTAGTTACCCTCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGACTGGAGTGGATTGGGAGTATCTATTATAGT GGGAGCGCCCAGTACAACCCGTCCCTCAAGAGT CGAGTCACCATATCCGTCGACACGTCCAAGAACC AGTTCTCCCTGACGCTGAGCTCTGTGACCGCCGC AGACACGGCTTTATATTACTGTGCGAGACTGAGC AGCAGCTGGTCTTTTGACTACTGGGGGCCAGGGAA CCCTGGTCACCGTCTCCTCA |
| | | | SEQ ID NO: 24417 | SEQ ID NO: 28423 |
| | | AA | DIQMTQSPSSLSASVGDRVTITCRASQGIRNDLG WYQQKPGKAPKRLIYAASSLQSGVPSRFSGSGS GTEFTLTVSSLQPEDFATYYCLQHSSYPLTFGGG TKVEIK | QLQLQESGPGLVKPSETLSLTCTVSGGSISRSSYYW GWIRQPPGKGLEWIGSIYYSGSAQYNPSLKSRVTIS VDTSKNQFSLTLSSVTAADTALYYCARLSSSWSFD YWGQGTLVTVSS |
| | | | SEQ ID NO: 24418 | SEQ ID NO: 28424 |
| iPS:392822 | 21-225_23C8 | NA | GACATCCAGATGACCCAGTCTCCATCCTCCCG GTCTGCATCTGTAGGGGACAGAGTCACCATCA CTTGCCGGGCAAGTCAGGACATTAGAAATGAT TTAGGCTGGTATCAGCAGAAACCAGGGAGAG CCCCTAAGCGCCTGATCAATGGTGCATCCAGT GTGCAAAGTGGGGTCCCATCAAGGTTCAGCGG CAGTGGATCTGGGACAGAATTCACTCTCACAA TCAGCAGCCTGCAGCCTGAAGATTTTGTAATT TATTACTGTCTACAGCATAATAGTTACCCGCTC ACTTTCGGCGGAGGGACCAAGGTGGAGATCA AA | CAGTTGCAACTGCAGGAGTCGGGCCCAGGACTG GTGAAGCCTTCGGAGACCCTGTCCCTCACCTGCA CTGTCTCTGGTGGCTCCATCAGTAGGAGTAGTTA CTACTGGGGCTGGATCCGCCAGCCCCCAGGGAA GGGGCTGGAGTGGATTGGGAATATTTATTATAGT GGGACCACTTACAACAACCCGTCCCTCAAGAGTC GAGTCACCATATCCGTAGACACGTCCAAGAACC ACTTCTCCCTGAAGCTGAGCTCTGTGACCGCCGC AGACACGGCTGTGTATTACTGTGGGAGACATGGA AAAGACTGGGGCCTTGACTACTGGGGCCAGGGA ACCCTGGTCACCGTCTCCTCA |
| | | | SEQ ID NO: 24419 | SEQ ID NO: 28425 |

FIGURE 50

| | | | | |
|---|---|---|---|---|
| | | AA | DIQMTQSPSSRSASVGDRVTITCRASQDIRNDLG WYQQKPGRAPKRLINGASSVQSGVPSRFSGSGS GTEFTLTISSLQPEDFVIYYCLQHNSYPLTFGGGT KVEIK | QLQLQESGPGLVKPSETLSLTCTVSGGSISRSSYYW GWIRQPPGKGLEWIGNIYYSGTTYNNPSLKSRVTIS VDTSKNHFSLKLSSVTAADTAVYYCGRHGKDWGL DYWGQGTLVTVSS |
| | | | SEQ ID NO: 24420 | SEQ ID NO: 28426 |
| iPS:392824 | 21-225_24E5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAGTAATTACCCGCTC ACTTTCGGCGGAGGGACCAAGGTGGAGATCA AA | CAGCTGCAGCTGCAGGAGTCGGGCCCAGGACTG GTGAAGCCCTCGGAGACCCTGTCCCTCACCTGCA CTGTCTCTGGTGGCGCCATCAGCAGGAGTAGTTA CTACTGGGGCTGGATCCGCCAGCCCCCAGGGAA GGGGCTGGAGTGGATTGGGAGTATCTATTATAGT GGGAGCGCCAACTACAACCCGTCCCTCAAGAGT CGAGTCACCATATCCGTAGACACGTCCAAGAACC AGTTCTCCCTGAAGCTGAGCTCTGTGACCGCCGC AGACACGGCTGTGTATTACTGTGCGAGACTCAGC AGCAGCTGGTCCATTGACAACTGGGGCCAGGGA ACCCTGGTCACCGTCTCCTCA |
| | | | SEQ ID NO: 24421 | SEQ ID NO: 28427 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHSNYPLTFGGG TKVEIK | QLQLQESGPGLVKPSETLSLTCTVSGGAISRSSYYW GWIRQPPGKGLEWIGSIYYSGSANYNPSLKSRVTIS VDTSKNQFSLKLSSVTAADTAVYYCARLSSSWSID NWGQGTLVTVSS |
| | | | SEQ ID NO: 24422 | SEQ ID NO: 28428 |

FIGURE 50

| iPS:392826 | 21-225_20B9 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGTTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATACTTATCCATTCAC TTTCGGCCCTGGGACCAAAGTGGATATCAAA | GAGGTGCAGCTGGTGGAGTCTGGGGGAGGCTTG GTACAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTTTCATACATTAGTAGTAGTAGTAGT ACCATATACTATGCAGACTCTGTGAAGGGCCGAT TCACCATCTCCAGAGACAATGCCAAGAACTCACT GTACCTGCAAATGAACAGCCTGAGAGACGAGGA CACGGCTGTGTATTACTGTGCGAGGTCACTATGG TCCCCCTTTGACTACTGGGGCCAGGGAACCCTGG TCACCGTCTCCTCA |
|---|---|---|---|---|
| | | | SEQ ID NO: 24423 | SEQ ID NO: 28429 |
| | | AA | DIQMTQSPSSLSASVGDRVTITCRASQGISNYLA WFQQKPGKAPKSLIYVASSLQSGVPSKFSGSGSG TDFTLTISSLQPEDFATYYCQQYNTYPFTFGPGT KVDIK | EVQLVESGGGLVQPGGSLRLSCAASGFTFSSYSMN WVRQAPGKGLEWVSYISSSSSTIYYADSVKGRFTIS RDNAKNSLYLQMNSLRDEDTAVYYCARSLWSPFD YWGQGTLVTVSS |
| | | | SEQ ID NO: 24424 | SEQ ID NO: 28430 |
| iPS:392830 | 21-225_21A5 | NA | GACATCCAGATGACCCAGTCTCCATCCTCCCT GTGTGCATCTGTAGGAGACAGAGTCACCATCA CTTGCCGGGCAAGTCAGACCATTAGCAGCCAT TTAAATTGGTATCAGCGGAAACCAGGGAAAG CCCCTAAACTCCTGATCTATGCTGCATCCAGTT TGCAAAGTGGGGTCCCATCACGGTTCAGTGGC AGTGGATCTGGGACAGATTTCACTCTCACCAT CAGCAGTGTGCAACCTGAAGATTTTGCAACTT ATTACTGTCAACAGAGTTACAATATCTCATTC ACTTTCGGCCCTGGGACCAAAGTGGATATCAA A | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTACTTCTGG AGCTGGATCCGGCAGCCCGCCGGGAAGGGACTG GAGTGGATTGGGCGTATCTATACCAGTGGGATCA CCAACTACAACCCCTCCCTCAAGAGTCGAGTCAC CATGTCAGTAGACACGTCCAAGAACCAGTTCTCC CTGAAACTGAGTTCTGTGACCGCCGCGGACACGG CCATATATTACTGTGCGAGAGGCCCGACTTCGGG GTGGTTCGACCCCTGGGGCCAGGGAACCCTGGTC ACCGTCTCCTCA |
| | | | SEQ ID NO: 24425 | SEQ ID NO: 28431 |

FIGURE 50

| | | | | |
|---|---|---|---|---|
| | | AA | DIQMTQSPSSLCASVGDRVTITCRASQTISSHLN WYQRKPGKAPKLLIYAASSLQSGVPSRFSGSGSG TDFTLTISSVQPEDFATYYCQQSYNISFTFGPGTK VDIK | QVQLQESGPGLVKPSETLSLTCTVSGGSISSYFWSW IRQPAGKGLEWIGRIYTSGITNYNPSLKSRVTMSVD TSKNQFSLKLSSVTAADTAIYYCARGPTSGWFDPW GQGTLVTVSS |
| | | | SEQ ID NO: 24426 | SEQ ID NO: 28432 |
| IPS:392832 | 21-225_21H8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGAGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGTCTGGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCTTGG CTGGACGGAAGAGTACTGGGGCCAGGGAACCCT GGTCACCGTCTCCTCA |
| | | | SEQ ID NO: 24427 | SEQ ID NO: 28433 |
| | | AA | DIQMTQSPSSLSASVGDRVTITCRASQGIRNDLG WYQQKPGKAPKRLIYAASSLQSGVPSRFRGSGS GTEFTLTISSLQPEDFATYYCLQHNSYPWTFGQG TKVEIK | QVQLVESGGGVVQSGRSLRLSCAASGFTFSDYGM HWVRQAPGKGLEWVAVIWFDGSNKYYADSVKGR FTISRDNSKNTLYLQMNSLRAEDTAVYYCARDLG WTEEYWGQGTLVTVSS |
| | | | SEQ ID NO: 24428 | SEQ ID NO: 28434 |

FIGURE 50

| iPS:392834 | 21-225_22C1 | NA | GACATCCAGATGACCCAGTCTCCATCCTCCCT GTCTGCATCGTAGGAGACAGAGTCACCATCA CTTGCCGGACAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAGTACTTACCCGCTC ACTTTCGGCGGAGGGACCAAGGTGGAGATCA AA | CAACTGCAGCTGCAGGAGTCGGGCCCAGGACTG GTGAAGCCTTCGGAGACCCTGTCCCTCACCTGTA CTGTTTCTGGTGGCTCCATCAACAGGAGTAGTTA CTACTGGGGCTGGATCCGCCAGCCCCCAGGGAA GGGGCTGGAGTGGATTGGGAATATCTATTATAGT GGGGCCACCTATTATAATTCGTCCCTCAAGAGTC GAGTCACCATCTCCGTAGACACGTCCACGAACCA GTTCTCCCTGAAGCTGAGCTCTGTGACCGCCGCA GACACGGCTGTGTATTACTGTGCGAGACATAGCG GCAGCTGGTCCCTTGACTACTGGGGCCAGGGAAC CCTGGTCACCGTCTCCTCA |
| | | | SEQ ID NO: 24429 | SEQ ID NO: 28435 |
| | | AA | DIQMTQSPSSLSASVGDRVTITCRTSQGIRNDLG WYQQKPGKAPKRLIYAASSLQSGVPSRFSGSGS GTEFTLTISSLQPEDFATYYCLQHSTYPLTFGGGT KVEIK | QLQLQESGPGLVKPSETLSLTCTVSGGSINRSSYYW GWIRQPPGKGLEWIGNIYYSGATYYNSSLKSRVTIS VDTSTNQFSLKLSSVTAADTAVYYCARHSGSWSLD YWGQGTLVTVSS |
| | | | SEQ ID NO: 24430 | SEQ ID NO: 28436 |
| iPS:392836 | 21-225_22F4 | NA | GACATCCAGATGACCCAGTCTCCATCCTCCCT GTCTGCTTCTGTTGGAGACAGAGTCACCATCA CTTGCCGGGCAAGTCAGGTCATTAGAGATGAT TTAGGCTGGTATCAGCAGAAACCAGGGAAAG CCCCTAAGCGCCTGATCTATGCTGCATCCAGT TTGCAAAGTGGGGTCCCATCGAGGTTCAGCGG CAGTAGATCTGGGACAGACTTCACTCTCACAA TCAGCAGCCTGCAGCCTGAAGATTTTGCAACT TATTACTGTCTACACCACTATAGTTATCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGCGACTATGGCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAAAAGTAT AGCAGCAGCTGGTACGACTACGGTATGGACGTCT GGGGGCCAAGGGACCACGGTCACCGTCTCCTCA |
| | | | SEQ ID NO: 24431 | SEQ ID NO: 28437 |

FIGURE 50

| | | | | |
|---|---|---|---|---|
| | | AA | DIQMTQSPSSLSASVGDRVTITCRASQVIRDDLG WYQQKPGKAPKRLIYAASSLQSGVPSRFSGSRS GTDFTLTISSLQPEDFATYYCLHHYSYPRTFGQG TKVEIK | QVQLVESGGGVVQPGRSLRLSCAASGFTFSDYGM HWVRQAPGKGLEWVAVIWYDGSNKYYVDSVKGR FTISRDNSKNTLYLQMNSLRAEDTAVYYCAREKYS SSWYDYGMDVWGQGTTVTVSS |
| | | | SEQ ID NO: 24432 | SEQ ID NO: 28438 |
| iPS:392838 | 21-225_22G8 | NA | GACATCCAGATGATCCAGTCTCCATCCTCCCT GTTCGCATCTGTAGGAGACAGAGTCACCATCA CTTGCCGGACAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAAATTCAGCGG CAGTGGATCTGGGACAGAATTCACTCTCTCAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAACAGGAGTAGTTA CTACTGGGGCTGGATCCGCCAGCCCCCAGGGAA GGGGCTGGACTGGATTGGGAATATCTATTATAGT GGGAGCACCTACTACAACCCGTCCGTCAAGAGTC GATTCACCATATCCGTAGACACGTCCAAGAACCA GTTCTCCCTGAAGCTGAGCTCTGTGACCGCCGCA GACACGGCTGTGTATTACTGTGCGAGACATGGAA AAGACTGGGGCCTTGACTACTGGGGCCAGGGAA CCCTGGTCACCGTCTCCTCA |
| | | | SEQ ID NO: 24433 | SEQ ID NO: 28439 |
| | | AA | DIQMIQSPSSLFASVGDRVTITCRTSQGIRNDLG WYQQKPGKAPKRLIYAASSLQSGVPSKFSGSGS GTEFTLSISSLQPEDFATYYCLQHNSYPLTFGGGT KVEIK | QLQLQESGPGLVKPSETLSLTCTVSGGSINRSSYYW GWIRQPPGKGLDWIGNIYYSGSTYYNPSVKSRFTIS VDTSKNQFSLKLSSVTAADTAVYYCARHGKDWGL DYWGQGTLVTVSS |
| | | | SEQ ID NO: 24434 | SEQ ID NO: 28440 |

FIGURE 50

| iPS:392840 | 21-225_23G1 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTCTGCTGCATCCAGTTT ACAGAGTGGGGTCCCATCACAGTTCAGCGGCA GTGGATTTGGGACAGATTTCACTCTCACCATC AGCAGCCTGCAGCCTGAGGATTTTGCAACTTA TTACTGCCAACAGTATAATAGTTACCCATTCA CTTTCGGCCCTGGGACCAAAGTGGATATCAAA<br><br>SEQ ID NO: 24435 | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAGGGGGCT GGAGTGGGTCTCAGTTATTAGTGGTAGTGGTGGT ACCACATATAACACAGACTCCGTGAAGGGCCGG TTCACCATCTCCAGAGACAATTCCAAGAACACGC TATATCTGCAAATGAACAGCCTGAGAGCCGAGG ACACGGCCGTATATTACTGTGCCCGCAGCTCCTT GTTTGACTACTGGGGCCAGGGAACCCTGGTCACC GTCTCCTCA<br><br>SEQ ID NO: 28441 |
|---|---|---|---|---|
| | | AA | DIQMTQSPSSLSASVGDRVTITCRASQGISNYLA WFQQKPGKAPKSLISAASSLQSGVPSQFSGSGFG TDFTLTISSLQPEDFATYYCQQYNSYPFTFGPGT KVDIK<br><br>SEQ ID NO: 24436 | EVQLLESGGGLVQPGGSLRLSCAASGFTFSSYAMS WVRQAPGRGLEWVSVISGSGGTTYNTDSVKGRFTI SRDNSKNTLYLQMNSLRAEDTAVYYCARSSLFDY WGQGTLVTVSS<br><br>SEQ ID NO: 28442 |
| iPS:392842 | 21-225_23G8 | NA | GACATCCAGATGACCCAGTCTCCATCCTCACT GTCTGCATCTGTAGGAGACAGAGTCACCATCA CTTGTCGGGCGAGTCAGGGCATTAGAAATTAT TTAGCCTGGTTTCAGCAGAAACCAGGGAAAGC CCCTAAGTCCCTGATCTATGCTGCATCCAGTTT GCAAAGTGGGGTCCCATCAAACTTCAGCGGCA GTGGATCTGGGACAGATTTCACTCTCACCATC AGTAGCCTGCAGCCTGAAGATTTTGCAACTTA TTACTGCCAACAGTATAATAGTTACCCTTTCAC TATCGGCCCTGGGACCAAGGTGGATATCAAA<br><br>SEQ ID NO: 24437 | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGGTTAGCAGTGG CTGGTTCGCCTGGGGCCAGGGAACCCTGGTCACC GTCTCCTCA<br><br>SEQ ID NO: 28443 |

1505

FIGURE 50

| | | | | |
|---|---|---|---|---|
| | | AA | DIQMTQSPSSLSASVGDRVTITCRASQGIRNYLA WFQQKPGKAPKSLIYAASSLQSGVPSNFSGSGSG TDFTLTISSLQPEDFATYYCQQYNSYPFTIGPGTK VDIK | EVQLLESGGGLVQPGGSLRLSCAASGFTFSSYAMS WVRQAPGKGLEWVSAISGSGGSTYYADSVKGRFTI SRDNSKNTLYLQMNSLRAEDTAVYYCAVSSGWFA WGQGTLVTVSS |
| | | | SEQ ID NO: 24438 | SEQ ID NO: 28444 |
| IPS:392844 | 21-225_23E11 | NA | GACATCCAGATGACCCAGTCTCCATCCTCCCT GTCTGCATCTGTAGGAGACAGAATCACCATCA CTTGCCGGGCAAGTCAGGGCATTAGAAATGAT TTAGGCTGGTATCAGCAGAAACCAGGGAAAG CCCCTAAGCGCCTGATCTATCCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGTGG ACGTTCGGCCAAGGGACCAAGGTGGAAATCA AA | GAGGTGCAACTGGTGGAGTCTGGGGGAGGCCTG GTCAAGCCTGGGGGGTCCCTGAGACTCTCCTGTG CAGCCTCTGGATTCACCTTCAGTTCCTATACCAT GAACTGGGTCCGCCAGGCTCCAGGGAAGGGGCT GGAGTGGGTCTCATCCATTAGTGGTAGTAGTAGT TACATATGGTATGTAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATCGGGGC AGTCTCTGGGGCCAGGGAACCCTGGTCACCGTCT CCTCA |
| | | | SEQ ID NO: 24439 | SEQ ID NO: 28445 |
| | | AA | DIQMTQSPSSLSASVGDRITITCRASQGIRNDLG WYQQKPGKAPKRLIYPASSLQSGVPSRFSGSGSG TEFTLTISSLQPEDFATYYCLQHNSYPWTFGQGT KVEIK | EVQLVESGGGLVKPGGSLRLSCAASGFTFSSYTMN WVRQAPGKGLEWVSSISGSSSYIWYVDSVKGRFTI SRDNAKNSLYLQMNSLRAEDTAVYYCARDRGSLW GQGTLVTVSS |
| | | | SEQ ID NO: 24440 | SEQ ID NO: 28446 |

FIGURE 50

| iPS:392846 | 21-225_24B6 | NA | GACATCCAGATGACCCAGTCTCCATCCTCCCT GTCTGCATCTGTCGGAGACAGAGTCACCATCA CTTGCCGGGCAAGTCAGGACATTAGAAATGAT TTAGGCTGGTATCAGCAGAAGCCAGGGAAAG CCCCTAAGCGCCTGATCTATGCTGCATCCAGT TTGCACAGTGGGGTCCCATCAAGGTTCAGCGG CAGTGGATCTGGGACAGAATTCACTCTCACAA TCAGCAGCCTGCAGACTGAAGATTTTGCAACT TATTACTGTCTACAGCATTATAGTTACCCTCGG ACGTTCGGCCAAGGGACCAAGGTGGAAGTCA AG | CAGGTGCAGCTGGTGGAGTCTGGGGGAGGCGTG GTCCAGCCTGGGAGGTCCCCGAGACTCTCCTGTG CAGCGTCTGGATTCATCTTCAGTAACTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGAATA TAGTAGTGGCTGGTACGACTACGGTATGGACGTC TGGGGCCAAGGGACCACGGTCACCGTCTCCTCA |
| | | | SEQ ID NO: 24441 | SEQ ID NO: 28447 |
| | | AA | DIQMTQSPSSLSASVGDRVTITCRASQDIRNDLG WYQQKPGKAPKRLIYAASSLHSGVPSRFSGSGS GTEFTLTISSLQTEDFATYYCLQHYSYPRTFGQG TKVEVK | QVQLVESGGGVVQPGRSPRLSCAASGFIFSNYVMH WVRQAPGKGLEWVAVIWYDGSNKYYADSVKGRF TISRDNSKNTLYLQMNSLRAEDTAVYYCAREEYSS GWYDYGMDVWGQGTTVTVSS |
| | | | SEQ ID NO: 24442 | SEQ ID NO: 28448 |
| iPS:392848 | 21-225_20F9 | NA | GACATCCAGATGACCCAGTCTCCATCCTCACT GTCTGCATCTGTAGGAGACAGAATCACCATCA CTTGTCGGGCGAGTCAGGGCATTAGCAATTAT TTAGCCTGGTTTCAGCAGAAACCAGGGAAAGC CCCTAAGTCCCTGATCTCTGCTGCATCCAGTTT GCAAAGTGGGGTCCCTTCAAAGTTCAGCGGCA GTGGATCCGGGACAGATTTCACTCTCACCATC AGCAGCCTGCAGCCTGAAGATTTTGCAACTTA TTGCTGCCAACAGTATCATAGTTACCCGTGGA CGTTCGGCCAAGGGACCAAGGTGGAAATCAA A | GAAGTGCAGCTGGTGGAATCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTATTGTGCGAGAGATCGTGGG AGCTGCTGGGGCCAGGGAACCCTGGTCACCATCT CCTCA |
| | | | SEQ ID NO: 24443 | SEQ ID NO: 28449 |

FIGURE 50

| | | | | |
|---|---|---|---|---|
| | | AA | DIQMTQSPSSLSASVGDRITITCRASQGISNYLAWFQQKPGKAPKSLISAASSLQSGVPSKFSGSGSGTDFTLTISSLQPEDFATYCCQQYHSYPWTFGQGTKVEIK | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARDRGSCWGQGTLVTISS |
| | | | SEQ ID NO: 24444 | SEQ ID NO: 28450 |
| IPS:392850 | 21-225_20H10 | NA | GGCATCCAGATGACCCAGTCTCCATCCTCCCTGTCTGCATCTGTAGGAGACAGAGTCACCATCACTTGCCGGGCAAGTCAGGGCATTAAAAATAATTTAGGCTGGTATCAGCAGAAACCAGGGAAAGGCCCTAAGTGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTACCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTATTTATTACTGTGCGAGAGATCGTGGGAGCCTCTGGGGCCAGGGAACCCTGGTCACCGTCTCCTCA |
| | | | SEQ ID NO: 24445 | SEQ ID NO: 28451 |
| | | AA | GIQMTQSPSSLSASVGDRVTITCRASQGIKNNLGWYQQKPGKGPKCLIYAASSLQSGVPSRFSGSGSGTEFTLTISSLQPEDFATYYCLQHNSYPLTFGGGTKVEIK | EVQLVESGGGLVKPGGSLRLSCAASGFTFSTYSMNWVRQAPGKGLEWVSSISSSSSYIYYADSVKGRFTISRDNAKNSLYLQMNSLRAEDTAIYYCARDRGSLWGQGTLVTVSS |
| | | | SEQ ID NO: 24446 | SEQ ID NO: 28452 |

FIGURE 50

| iPS:392852 | 21-225_21A2 | NA | GACATCCAGATGACCCAGTCTCCATCCTCCCTGTCTGCATCTGTAGGAGACAGAGTCACCATCACTTGCCGGGCAAGTCAGAGTATTAGTAGTTATTTAAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGACTTCACTCTCACCATCAGTAGTCTGCAACCTGAAGATTTTGCAACTTACTTCTGTCAACAGAGTTACAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTAAATTCACCTTTAGCAGCTATGCCATGAACTGGGTCCGCCAGGCTCCAGGGAAGGGGCTGGAGTGGATCTCAATTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTTTGGCAGTGGCTGGCTCGGAGGCTTTTGATATCTGGGGCCAAGGGACAATGGTCACCGTCTCTTCA |
| | | | SEQ ID NO: 24447 | SEQ ID NO: 28453 |
| | | AA | DIQMTQSPSSLSASVGDRVTITCRASQSISSYLNWYQQKPGKAPKLLIYAASSLQSGVPSRFSGSGSGTDFTLTISSLQPEDFATYFCQQSYRTPFFTFGPGTKVDIK | EVQLLESGGGLVQPGGSLRLSCAASKFTFSSYAMNWVRQAPGKGLEWISIISGRGGNTFYADSVKGRFTISRDNSKNTLYLQMNSLRAEDTAVYYCASRLAVAGSEAFDIWGQGTMVTVSS |
| | | | SEQ ID NO: 24448 | SEQ ID NO: 28454 |
| iPS:392854 | 21-225_21E5 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTACATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCCCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTTCAGCGTCTGGATTCACCTTCAGTGACTATGGCATGCACTGGGTCCGCCAGGCTCCAGGCAAGGGGCTGGAGTGGGTGGCAGTTATATGGTATGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTACGAGAGAACTGGGGTTCCGGTCTGACTATTGGGGCCAGGGAACCCTGGTCACCGTCTCCTCA |
| | | | SEQ ID NO: 24449 | SEQ ID NO: 28455 |

FIGURE 50

| | | | | |
|---|---|---|---|---|
| | | AA | DIQMTQSPSSLSASVGDRVTITCRASQGIRNDLG WYQQKPGKAPKRLIYATSSLQSGVPSRFSGSGSG TEFTLTISSLQPEDFATYYCLQHNSYPLTFGGGT KVEIK | QVQLVESGGGVVQPGRSLRLSCSASGFTFSDYGMH WVRQAPGKGLEWVAVIWYDESNKYYADSVKGRF TISRDNSKNTLYLQMNSLRAEDTAMYYCTRELGFR SDYWGQGTLVTVSS |
| | | | SEQ ID NO: 24450 | SEQ ID NO: 28456 |
| IPS:392856 | 21-225_22A2 | NA | GACATCCAGATGACCCAGTCTCCATCCTCACT GTCTGCATCTGTCGGAGACAGAGTCACCATCA CTTGTCGGGCGAGTCAGGACATTAGCAATTAT TTAGCCTGGGTTCAGCAGAAACCAGGGAAAG CCCCTAAGTCCCTGATCTACGCTGCATCCAGTT TGCAAAGTGGGGTCCCATCAAAGTTCAGCGGC AGTGGATCTGGGACAGATTTCACTCTCACCAT CAGCAGCCTGCAGCCTGAAGATTTTGCAAGTT ATTACTGCCAACAGTATAATAGTTTCCCGCTC ACTTTCGGCGGAGGGACCAAGGTGGAGATCA AA | GAGGTGCAGCTGTTGGAGTCTGGGGGAGGCTTG GTCCAACCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGGTATTAGTGGTAGTGGAGGT AACACACCCTACGCAGACTCCGTGAAGGGCCGG TTCACCATCTCCAGAGACATTTCCAAGAACACGC TGTATCTGCAAATGAACAGCCTGAGAGCCGAAG ACACGGCCGTATATTACTGTGCGAAAGTAGTGGG AGCTGTCCACTGGGGCCGGGGAACCCTGGTCACC GTCTCCTCA |
| | | | SEQ ID NO: 24451 | SEQ ID NO: 28457 |
| | | AA | DIQMTQSPSSLSASVGDRVTITCRASQDISNYLA WVQQKPGKAPKSLIYAASSLQSGVPSKFSGSGS GTDFTLTISSLQPEDFASYYCQQYNSFPLTFGGG TKVEIK | EVQLLESGGGLVQPGGSLRLSCAASGFTFSSYAMS WVRQAPGKGLEWVSGISGSGGNTPYADSVKGRFTI SRDISKNTLYLQMNSLRAEDTAVYYCAKVVGAVH WGRGTLVTVSS |
| | | | SEQ ID NO: 24452 | SEQ ID NO: 28458 |

FIGURE 50

| | | | | |
|---|---|---|---|---|
| iPS:392858 | 21-225_22H4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT GTGCAAAGTGGGGTCCCATCAAGGTTCAGCGG CAGTGGATCTGGGACAGAATTCACTCTCACAA TCAGCAGCCTGCAGCCTGAAGATTTTGCACTT TATTACTGTCTACAGCATAATAGTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGGAGTAGTTA CTACTGGGGCTGGATCCGCCAGCCCCCAGGGAA GGGGCTGGAGTGGATTGGGAATATTTATTATAGT GGGAGCACCTACCACAACCCGTCTCTCAAGAGTC GAGTCACCATATCCGTAGACACGTCCATGAACCA GTTCTCCCTGAAGTTGACCTCTGTGACCGCCGCA GACACGGCTGTGTATTTCTGTGGGAGACATGGAA AAGACTGGGGCCTTGACTACTGGGGCCAGGGAA CCCTGGTCACCGTCTCCTCA |
| | | | SEQ ID NO: 24453 | SEQ ID NO: 28459 |
| | | AA | DIQMTQSPSSLSASVGDRVTITCRASQGIRNDLG WYQQKPGKAPKRLIYAASSVQSGVPSRFSGSGS GTEFTLTISSLQPEDFALYYCLQHNSYPLTFGGG TKVEIK | QLQLQESGPGLVKPSETLSLTCTVSGGSISRSSYYW GWIRQPPGKGLEWIGNIYYSGSTYHNPSLKSRVTIS VDTSMNQFSLKLTSVTAADTAVYFCGRHGKDWGL DYWGQGTLVTVSS |
| | | | SEQ ID NO: 24454 | SEQ ID NO: 28460 |
| iPS:392860 | 21-225_22H8 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTTGTATTGGTACCTGCA GAAGCCAGGCCATCCTCCACAGCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGAATTTATTACTGCATGCAAAG TATACAGCTTCCGCTCTCATTCGGCGGAGGGA CCAAGGTGGAGATCAAC | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATCTGGTATGATGGAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGCTTGC CTGGTACGAAGACTACTGGGGCCAGGGAACCCT GGTCACCGTCTCCTCA |
| | | | SEQ ID NO: 24455 | SEQ ID NO: 28461 |

FIGURE 50

| | | | | |
|---|---|---|---|---|
| | | AA | DIVMTQTPLSLSVTPGQPASISCKSSQSLLHSDGK TFLYWYLQKPGHPPQLLIYEVSNRFSGVPDRFSG SGSGTDFTLKISRVEAEDVGIYYCMQSIQLPLSFG GGTKVEIN | QVQLVESGGGVVQPGRSLRLSCAASGFTFSSYGMH WVRQAPGKGLEWVAVIWYDGNNKYYADSVKGRF TISRDNSKNTLYLQMNSLRAEDTAVYYCARELAW YEDYWGQGTLVTVSS |
| | | | SEQ ID NO: 24456 | SEQ ID NO: 28462 |
| iPS:392864 | 21-225_23B9 | NA | GAAATTGTGTTGACGCAGTCTCCAGGCACCCT GTCTTTGTCTCCAGGAGAAAGAGCCACCCTCT CCTGCAGGGCCAGTCAGAATGTTTACAGCAGC TACTTAGCCTGGTACCAGCAGAAACCTGGCCA GACTCCCAGGCTCCTCATCTATGGTGCATCCA GCAGGGCCAGTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAGCTCACCT CGGACGTTCGGCCAAGGGACCAAGGTGGAAA TCAAA | CAGGTGCAGCTGCAGGCGTCGGGCCCAGGACTG GTGAAGCCTTCACAGACCCTGTCCCTCACCTGCA CTGTCTCTGATGGCTCCATCAGCAGTGGTGGTTA CTACTGGAGCTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGTAGACACGTCTAAGAACCA GTTCTCCCTGAAGCTGAGTTCTGTGACTGCCGCG GACACGGCCGTATATTACTGTGCGAGAGAGGAC GGTGCCTTCGGCTACTACGGTATGGACGTCTGGG GCCAAGGGACCACGGTCACCGTCTCCTCA |
| | | | SEQ ID NO: 24457 | SEQ ID NO: 28463 |
| | | AA | EIVLTQSPGTLSLSPGERATLSCRASQNVYSSYLA WYQQKPGQTPRLLIYGASSRASGIPDRFSGSGSG TDFTLTISRLEPEDFAVYYCQQYGSSPRTFGQGT KVEIK | QVQLQASGPGLVKPSQTLSLTCTVSDGSISSGGYY WSWIRQHPGKGLEWIGYIYYSGSTYYNPSLKSRVTI SVDTSKNQFSLKLSSVTAADTAVYYCAREDGAFGY YGMDVWGQGTTVTVSS |
| | | | SEQ ID NO: 24458 | SEQ ID NO: 28464 |

FIGURE 50

| iPS:392866 | 21-225_23H11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGACTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATCGTTACCCGCTC ACTTTCGGCGGAGGGACCAAGGTGGAGATCA AA | CAGGAGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAAT AATAAATACTATGTAGACTCCGTGAAGGGCCGAT TCACCATCTCCAGAGACAACTCCAAGAACACGCT GTATCTGCAAATGAACAGCCTGAGAGCCGAGGA CACGGCTGTGTATTACTGTGCGAGAGAATTGGGG TTCCGGTCTGACTACTGGGGCCAGGGAACCCTGG TCACCGTCTCCTCA |
| | | | SEQ ID NO: 24459 | SEQ ID NO: 28465 |
| | | AA | DIQMTQSPSSLSASVGDRVTITCRASQGIRNDLD WYQQKPGKAPKRLIYAASSLQSGVPSRFSGSGS GTEFTLTISSLQPEDFATYYCLQHNRYPLTFGGG TKVEIK | QEQLVESGGGVVQPGRSLRLSCAASGFTFSSYGMH WVRQAPGKGLEWVAVIWYDENNKYYVDSVKGRF TISRDNSKNTLYLQMNSLRAEDTAVYYCARELGFR SDYWGQGTLVTVSS |
| | | | SEQ ID NO: 24460 | SEQ ID NO: 28466 |
| iPS:392868 | 21-225_24D6 | NA | GACATCCAGATGACCCAGTCTCCATCCTCCCT GTCTGCATCTGTAGGAGACAGAGTCACCATCA CTTGCCAGGCGAGTCAGGACATTAACAACTAT TTAAATTGGTATCAGCAGAAACCAGGGAAAG CCCTTAAGCTCTTGATATACGATGCATCCGATT TGGAAACAGGGGTCCCATCAAGGTTCAGTGGA AGTGGATCTGGGACAGATTTTACTTTCACCAT CAGCAGTCTGCAGCCTGAAGATATTGCAATAT ATTACTGTCAACAGTATGAAAATCTCCCGATC ACCTTCGGCCAAGGGACACGACTGGAGATTAA A | CAGGTGCAACTGGTAGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGTTCCAGGCAAGGGGCT GGAGTGGGTGGCAATTATATCATATGCTGGAAGT AATAAATCCTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGTGAGACGGGGATAC AGCTATGGCGGGTACGGTATGGACGTCTGGGGC CAAGGGGCCACGGTCACCGTCTCCTCA |
| | | | SEQ ID NO: 24461 | SEQ ID NO: 28467 |

FIGURE 50

| | | | | |
|---|---|---|---|---|
| | | AA | DIQMTQSPSSLSASVGDRVTITCQASQDINNYLN WYQQKPGKALKLLIYDASDLETGVPSRFSGSGS GTDFTFTISSLQPEDIAIYYCQQYENLPITFGQGT RLEIK | QVQLVESGGGVVQPGRSLRLSCAASGFTFSSYGMH WVRQVPGKGLEWVAIISYAGSNKSYADSVKGRFTI SRDNSKNTLYLQMNSLRAEDTAVYYCVRRGYSYG GYGMDVWGQGATVTVSS |
| | | | SEQ ID NO: 24462 | SEQ ID NO: 28468 |
| iPS:392870 | 21-225_20G9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAGTACTTACCCTCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGGAGTAGTTA CTACTGGGGCTGGATCCGCCAGCCCCCAGGGAA GGGACTGGAGTGGATTGGGAGTATCTATTATAGT GGGAGCGCCTCTTACAACCCGTCCCTCAAGAGTC GAGTCACCATATCCGTAGACACGTCCAGGAACC AGTTCTCCCTGAAGCTGAGCTCTGTGACCGCCGC AGACACGGCTGCGTATTACTGTGCGAGACTGAGC AGCAGCTGGTCTTTTGACTACTGGGGCCAGGGAA CCCTGGTCACCGTCTCCTCA |
| | | | SEQ ID NO: 24463 | SEQ ID NO: 28469 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHSTYPLTFGGGT KVEIK | QLQLQESGPGLVKPSETLSLTCTVSGGSISRSSYYW GWIRQPPGKGLEWIGSIYYSGSASYNPSLKSRVTIS VDTSRNQFSLKLSSVTAADTAAYYCARLSSSWSFD YWGQGTLVTVSS |
| | | | SEQ ID NO: 24464 | SEQ ID NO: 28470 |

FIGURE 50

| iPS:392872 | 21-225_20B11 | NA | GACATCCAGATGACCCAGTCTCCATCCTCCCT GTCTGCATCTGTAGGAGACAGAGTCACCATCA CTTGCCGGGCAAGTCAGGGCATTGGAAATGAT TTAGGCTGGTATCAGCAGAAACCAGAGAAAG CCCCTAAGCGCCTGATCTATGCTGCATCCAGT TTGCATAGTGGGGTCCCATCCAGGTTCAGCGG CAGTGGCTCTGGGACAGAATTCACTCTCACAA TCAGCAGCCTGCAGCCTGAAGATTTTGCAACT TATTACTGTCTACAGCATTATAGTTACCCTCGG ACGTTTGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGTCCGAT TCACCGTCTCCAGAGACAATTCCAAGAACACGCT GTCTCTGCAAATGAACAGCCTGAGAGCCGAGGA CACGGCTGTGTATTACTGTGCGAGAGAGAGATAT ACCAGTGGCTGGTATGACTACGGTATGGACGTCT GGGGCCAAGGGACCACGGTCACCGTCTCCTCA |
| | | | SEQ ID NO: 24465 | SEQ ID NO: 28471 |
| | | AA | DIQMTQSPSSLSASVGDRVTITCRASQGIGNDLG WYQQKPEKAPKRLIYAASSLHSGVPSRFSGSGSG TEFTLTISSLQPEDFATYYCLQHYSYPRTFGQGT KVEIK | QVQLVESGGGVVQPGRSLRLSCAASGFTFSSYVMH WVRQAPGKGLEWVAVIWYDGSNKYYADSVKVRF TVSRDNSKNTLSLQMNSLRAEDTAVYYCARERYTS GWYDYGMDVWGQGTTVTVSS |
| | | | SEQ ID NO: 24466 | SEQ ID NO: 28472 |
| iPS:392874 | 21-225_21D2 | NA | GACATCCAGATGACCCAGTCTCCATCCTCCCT GTTTGCATCTGTAGGAGACAGAGTCACCATCA CTTGCCGGGCAAGTCAGAGCATTAGCGACTAT TTAAATTGGTATCAGCAGAAACCAGGGAGAG CCCCTAAACTCCTGATCTATGATACATCCAGTT TGCAAAGTGGGGTCCCATCAAGGTTCAGTGGC AGTGGATCTGGGACAGATTTCACTCTCACCAT CAACAGTCTGCAACCTGAAGATTTTGCAACTT ACTACTGTCAACAGACTTACAATATTCTTCCG GAGCGCAGTTTTGGCCGGGGGACCAAGCTGG AGATCAAA | GAGGTGAAGCTGTTGGAGTCTGGGGGAGGCTTG GTACAGCCGGGGGGGTCCCTGAGACTCTCCTGTG CAGCCTCTGGATTCACCTTTAACAACTATGCCAT GAGCTGGGTCCGCCAGGCTCCAGGGAAGGGGCT GGAGTGGGTCTCAGTTCTTAGTGGTAGTGGTGGT AGCACATTCTACGCAGACTCCGTGAAGGGCCGGT TCACCATCTCCAGAGACAATTCCAAGAACACGCT GTATCTGCAAATGAGCAGCCTGAGAGCCGGGGA CACGGCCGTATATTTCTGTGCCCGATATTGTAGT AGTGCCAGGTGCCCTTATGATGCCTTTGATATCT GGGGCCAAGGGACAATGGTCACCGTCTCCTCA |
| | | | SEQ ID NO: 24467 | SEQ ID NO: 28473 |

FIGURE 50

| | | | | |
|---|---|---|---|---|
| | | AA | DIQMTQSPSSLFASVGDRVTITCRASQSISDYLN WYQQKPGRAPKLLIYDTSSLQSGVPSRFSGSGSG TDFTLTINSLQPEDFATYYCQQTYNILPERSFGRG TKLEIK | EVKLLESGGGLVQPGGSLRLSCAASGFTFNNYAMS WVRQAPGKGLEWVSVLSGSGGSTFYADSVKGRFTI SRDNSKNTLYLQMSSLRAGDTAVYFCARYCSSARC PYDAFDIWGQGTMVTVSS |
| | | | SEQ ID NO: 24468 | SEQ ID NO: 28474 |
| IPS:392876 | 21-225_21F7 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGGGAAAG CCCCTAAGCGCCTGATCTATGCTGCATCCAAT TTTCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAATTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAAT AATAAATACTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGATCTTGGC TGGACGGAAGAGTACTGGGGCCAGGGAACCCTG GTCACCGTCTCCTCA |
| | | | SEQ ID NO: 24469 | SEQ ID NO: 28475 |
| | | AA | DIQMTQSPSSLSASVGDRVTITCRASQDIRNDLG WYQQKPGKAPKRLIYAASNFQSGVPSRFSGSGS GTEFTLTISSLQPEDFATYYCLQHNNYPWTFGQG TKVEIK | QVQLVESGGGVVQPGRSLRLSCAASGFTFSDYGM HWVRQAPGKGLEWVAVIWFDGNNKYYVDSVKGR FTISRDNSKNTLYLQMNSLRAEDTAVYYCARDLG WTEEYWGQGTLVTVSS |
| | | | SEQ ID NO: 24470 | SEQ ID NO: 28476 |

FIGURE 50

| iPS:392878 | 21-225_22C5 | NA | GACATCCAGATGACCCAGTCTCCAGCCTCCCT GTCTGCGTCTGTTGGAGACAGAGTCACCATCA CTTGCCGGGCAAGTCAGAACATTAGCAGCTAT TTAAATTGGTATCAGCAGAAACCAGGGAAGG CCCCTAAACTCCTGATCTATGCTGCATCCGTTT TGCAACATGGGATCCCATCAAGGTTCAGTGGC AGGGGATCTGGGACAGATTTCACTCTCATCAT CAGTAGTCTGCAACCTGAAGATTTTGCAACTT ACTACTGTCAACAGAGTTACAGAACCCCCTTA TTCACTTTCGGCCCTGGGACCAAAGTAGATTT CAAA | GAGGTGCAGCTGTTGGAGTCTGGGGGAGGCTTG GTACAGGTTGGGGGGTCCCTGAGACTCTCCTGTG CAGCCTCTGGATTCACTTTTAGCAGCTATGCCAT GAGCTGGGTCCGCCAGGCTCCAGGGATGGGGCT GGAGTGGGTCTCAATTATTAGTGGTAGTGGTGGT TACACATACTACGCGGACTCCGTGAAGGGCCGAT TCACCATCTCCAGAGACAATTCCAAGAACACGCT GTATCTGCAAATGAACAGCCTGAGAGCCGAGGA CACGGCCGTATATTATTGTGCGTCCCGTATAGCA GTGGCTGGCTCGGAGGCTTTTGATATCTGGGGCC AAGGGACAATGGTCACCGTCTCTTCA |
| | | | SEQ ID NO: 24471 | SEQ ID NO: 28477 |
| | | AA | DIQMTQSPASLSASVGDRVTITCRASQNISSYLN WYQQKPGKAPKLLIYAASVLQHGIPSRFSGRGS GTDFTLIISSLQPEDFATYYCQQSYRTPLFTFGPG TKVDFK | EVQLLESGGGLVQVGGSLRLSCAASGFTFSSYAMS WVRQAPGMGLEWVSIISGSGGYTYYADSVKGRFTI SRDNSKNTLYLQMNSLRAEDTAVYYCASRIAVAGS EAFDIWGQGTMVTVSS |
| | | | SEQ ID NO: 24472 | SEQ ID NO: 28478 |
| iPS:392880 | 21-225_22F9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGATGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGAGGAAAA TAATAAAGACTATGTAGACTCCGTGAAGGGCCG ATTCACCATCTCCAGAGACAATTCCAAGAGCACG CTGTATCTGCAAATGAATAGTCTGAGAGCCGAGG ACACGGCTGTGTATTACTGTGTGAGAGAGTTAGG CTTCCAGTCTGACTACTGGGGCCAGGGAACCCCG GTCACCGTCTCCTCA |
| | | | SEQ ID NO: 24473 | SEQ ID NO: 28479 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQMVESGGGVVQPGRSLRLSCAASGFTFSSYGM HWVRQAPGKGLEWVAVIWYEENNKDYVDSVKGR FTISRDNSKSTLYLQMNSLRAEDTAVYYCVRELGF QSDYWGQGTPVTVSS |
| | | | SEQ ID NO: 24474 | SEQ ID NO: 28480 |
| iPS:392882 | 21-225_23A3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT GTGCAAAGTGGGGTCCCATCAAGGTTCAGCGG CAGTGGATCTGGGACAGAATTCACTCTCACAA TCAGCAGCCTGCAGCCTGAAGATTTTGCAATT TATTTCTGTCTACAGCATAATAGTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGGAGTAGTTA CTACTGGGGCTGGATCCGCCAGCCCCCAGGGCA GGGGCTGGAGTGGATTGGGAATATTTATTATAGT GGGAGCACCTACAACAACCCGTCCCTCAAGAGT CGAGTCTCCATATCCGTTGACACGTCCAAGAACC AGTTCTCCCTGAACCTGAGCTCTGTGACCGCCGC AGACACGGCTGTGTATTACTGTGGGAGACATGGA AAAGACTGGGGCCTTGACTTCTGGGGCCAGGGA ACCCTGGTCACCGTCTCCTCA |
| | | | SEQ ID NO: 24475 | SEQ ID NO: 28481 |
| | | AA | DIQMTQSPSSLSASVGDRVTITCRASQGIRNDLG WYQQKPGKAPKRLIYAASSVQSGVPSRFSGSGS GTEFTLTISSLQPEDFAIYFCLQHNSYPLTFGGGT KVEIK | QLQLQESGPGLVKPSETLSLTCTVSGGSISRSSYYW GWIRQPPGQGLEWIGNIYYSGSTYNNPSLKSRVSIS VDTSKNQFSLNLSSVTAADTAVYYCGRHGKDWGL DFWGQGTLVTVSS |
| | | | SEQ ID NO: 24476 | SEQ ID NO: 28482 |

FIGURE 50

| iPS:392884 | 21-225_23A10 | NA | GACATCCAGATGACCCAGTCTCCATCCTCCCT GTCTGCATCGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCGGCAGCCTGCAGCCTGAAGATTTTGCAACT TATTACTGTCTGCAACATTATAGTTACCCTCGG ACGTTCGGCCTAGGGACCAAGGTGGAAATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAAAGGTA TAGCAGTGGCTGGCACGACTACGGTATGGACGTC TGGGGCCAAGGGACCACGGTCACCGTCTCCTCA |
| | | | SEQ ID NO: 24477 | SEQ ID NO: 28483 |
| | | AA | DIQMTQSPSSLSASVGDRVTITCRASQGIRNDLG WYQQKPGKAPKRLIYAASSLQSGVPSRFSGSGS GTEFTLTIGSLQPEDFATYYCLQHYSYPRTFGLG TKVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARERYSS GWHDYGMDVWGQGTTVTVSS |
| | | | SEQ ID NO: 24478 | SEQ ID NO: 28484 |
| iPS:392886 | 21-225_23A12 | NA | GACATCGTGATGACCCAGTCTCCAGACTCCCT GGCTGTGTCTCTGGGCGAGAGGGGCCACCATCA ACTGCAAGTCCAGCCAGAGTGTTTTATACAGC TCCAACAATAACAATTATTTAGCTTGGTACCA GCAGAAACCAGGACAGCCTCCTAAGCTGCTCA TTTACTGGACATCTACCCGGGAATCCGGGGTC CCTGACCGATTCAGTGGCAGCGGGTCTGGGAC AGATTTCACTCTCACCATCAGCAGCCTGCAGG CTGAAGATGTGGCCGTTTATTACTGTCAGCAG TATTATGATACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACCCCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAACAC AGCCTACATGGAACTGAGCAGCCTGAGATCTGA GGACACGGCCGTGTATTACTGTGCGGGGTAGCAGT GGCTGGTACTACTTTGACTACTGGGGCCAGGGAA CCCTGGTCACCGTCTCCTCA |
| | | | SEQ ID NO: 24479 | SEQ ID NO: 28485 |

FIGURE 50

| | | | | |
|---|---|---|---|---|
| | | AA | DIVMTQSPDSLAVSLGERATINCKSSQSVLYSSN NNNYLAWYQQKPGQPPKLLIYWTSTRESGVPDR FSGSGSGTDFTLTISSLQAEDVAVYYCQQYYDTP PTFGQGTKVEIK | QVQLVQSGAEVKKPGASVKVSCKASGYPFTNYDIN WVRQATGQGLEWMGWMHPNSGNTGYAQKFQGR VTMTRNTSINTAYMELSSLRSEDTAVYYCAGSSGW YYFDYWGQGTLVTVSS |
| | | | SEQ ID NO: 24480 | SEQ ID NO: 28486 |
| iPS:392888 | 21-225_25A2 | NA | GATATTGTGATGAACCAGACTCCACTCTCTCT GTCCGTCACCCCTGGACAGCCGGCCTCCATCT CCTGCAAGTCTAGTCAGAGCCTCCTACATAGT GAAGGAAAGACCTATTTGTATTGGTATCTGCA GAAGCCAGGCCAGCCTCCACAACTCCTGATCT ATGAAATTTCCAACCGGTTCTCTGGAGTGCCA GCTAGGTTAAGTGGCAGCGGGTCAGGGACAG ATTTCACACTGAAAATCAGCCGGGTGGAGGCT GAGGATGTTGGGGTTTATTACTGCATGCAAAG TACACAGTTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCAGGGGGGTATGGACGTCTGGGG CCAAGGGACCACGGTCACCGTCTCCTCA |
| | | | SEQ ID NO: 24481 | SEQ ID NO: 28487 |
| | | AA | DIVMNQTPLSLSVTPGQPASISCKSSQSLLHSEGK TYLYWYLQKPGQPPQLLIYEISNRFSGVPARLSG SGSGTDFTLKISRVEAEDVGVYYCMQSTQFPLTF GGGTKVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WSGGMDVWGQGTTVTVSS |
| | | | SEQ ID NO: 24482 | SEQ ID NO: 28488 |

FIGURE 50

| iPS:392890 | 21-225_20H9 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTCTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCCGGATTCACCTTTAGCAGCTATGCCAT GAGCTGGGTCCGCCAGGCTCCAGGGAAGGGGCT GGAGTGGGTCTCAGCTATTAGTGGTAGTGGTGGT TACACATACTACGCAGACTCCGTGAAGGGCCGGT TCACCATCTCCAGAGACAATTCCGAGAACACGCT GTATCTGCAAATGAGCAGCCTGAGAGCCGAGGA CACGGCCGTTTATTACTGTGCGAAAGGGGGGTCC CTCTTCTACTGGGGCCAGGGAACCCTGGTCACCG TCTCCTCA |
| | | | SEQ ID NO: 24483 | SEQ ID NO: 28489 |
| | | AA | DIQMTQSPSSLSASVGDRVTITCRASQGISNYLA WFQQKPGKAPKSLISAASSLQSGVPSKFSGSGSG TDFTLTISSLQPEDFATYYCQQYNSYPFTFGPGT KVDIK | EVQLLESGGGLVQPGGSLRLSCAASGFTFSSYAMS WVRQAPGKGLEWVSAISGSGGYTYYADSVKGRFT ISRDNSENTLYLQMSSLRAEDTAVYYCAKGGGSLFY WGQGTLVTVSS |
| | | | SEQ ID NO: 24484 | SEQ ID NO: 28490 |
| iPS:392892 | 21-225_20C11 | NA | GACATCCAGATGACCCAGTCTCCATCCTCACT GTCTGCATCTGTTGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGAGGC AGTGGATCTGGGACAGATTTCACTCTCACCAT CAGCAGCCTGCAGCCTGAAGATTTTGCAACTT ATTACTGCCAACAGTATCATAGTTTCCCATTCA CTTTCGGCCCTGGGACCAAAGTGGATGTCAAA | GAGGTGCAGCTGTTGGAGTCGGGGGGGAGGCTTG GTACAGCCTGGGGGGTCCCTGAGACTCTCCTGTG CAGCCTCTGGATTCTCCTTTAGTAGCTATGCCAT GAGCTGGGTCCGCCAGGCTCCAGGGAAGGGGCT GGAGTGGGTCTCAACTATTAGTGGTCGTGGTGGT CACACATACTACGCAGACTCCGTGAAGGGCCGG TTCGCCATCTCCAGAGACAGTTCCAAGAACACGC TGTATCTGCAAATGAACAGCCTGAGAGCCGAGG ACACGGCCGTATATTACTGTGCGAAACAGGACTG CTGGGGCCAGGGAACCCTGGTCACCGTCTCCTCA |
| | | | SEQ ID NO: 24485 | SEQ ID NO: 28491 |

FIGURE 50

| | | AA | DIQMTQSPSSLSASVGDRVTITCRASQDISNYLA WFQQKPGKAPKSLIYAASSLQSGVPSKFRGSGSG TDFTLTISSLQPEDFATYYCQQYHSFPFTFGPGTK VDVK | EVQLLESGGGLVQPGGSLRLSCAASGFSFSSYAMS WVRQAPGKGLEWVSTISGRGGHTYYADSVKGRFA ISRDSSKNTLYLQMNSLRAEDTAVYYCAKQDCWG QGTLVTVSS |
|---|---|---|---|---|
| | | | SEQ ID NO: 24486 | SEQ ID NO: 28492 |
| IPS:392894 | 21-225_21G2 | NA | GACATCCAGATGACCCAGTCTCCATCCTCCCT GTCTGCGTCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTTCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAACATAATAGTTACCCGTGG ACGTTCGGCCTAGGGACCAAGGTGGTCATCAA A | CAGGTGCAACTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGATGGCAGTTATATGGTATGATGTAACT AATAAATACTATGCAGACTCCGTGAAGGGCCGA TTCACCATCTCCAGAGACAATTCCAAGAACACGC TGTATCTGGAAATGAACAGCCTGAGAGCCGAGG ACACGGCTGTTTATTACTGTGCGAGAGAGCTTGG CTGGTACGAGGACTACTGGGGCCAGGGAACCCT GGTCACCGTCTCCTCA |
| | | | SEQ ID NO: 24487 | SEQ ID NO: 28493 |
| | | AA | DIQMTQSPSSLSASVGDRVTITCRASQGIRNDLG WYQQKPGKAPKRLIYTSSSLQSGVPSRFSGSGSG TEFTLTISSLQPEDFATYYCLQHNSYPWTFGLGT KVVIK | QVQLVESGGGVVQPGRSLRLSCAASGFTFSDYGM HWVRQAPGKGLEWMAVIWYDVTNKYYADSVKG RFTISRDNSKNTLYLEMNSLRAEDTAVYYCARELG WYEDYWGQGTLVTVSS |
| | | | SEQ ID NO: 24488 | SEQ ID NO: 28494 |

FIGURE 50

| iPS:392896 | 21-225_21G7 | NA | GACATCCAGATGACCCAGTCTCCATCCTCCCT GTCTGCATCTGTAGGAGACAGAGTCACCATCA CTTGCCGGGCAAGTCAGGGCGTTAGAAATGAT TTAGGCTGGTATCAGCAGAAACCAGGGAAAG CCCCTCAGCGCCTGATCTATGCTGCATCCAGTT TGCAAAGTGGGGTCCCATCAAGGTTCAACGGC AGTGGATCTGGGACAGAATTCACTCTCACAAT CAGCAGCCTGCAGCCTGAAGATTTTGCAACTT ATTACTGTCTACAGCATAGTAGTTACCCGCTC ACTTTCGGCGGAGGGACCAAGGTGGAGATCA AA | CAGCTGCAGCTGCAGGAGTCGGGCCCAGGACTG GTGAAGTTTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCAGGAGTGGATAGGGAATATCTATTATAG TGGGTATAGTTACTACAATCCGTCCCTCAAGAGT CGAGTCACCATATCCGTAGACACGTCCAAGAACC AGTTCTCCCTGAAGCTGAGCTCTGTGACCGCCGC AGACACGGCTGTGTATTACTGTGCGAGACATAGC ACCAGCTGGTCCCTTGACTACTGGGGCCAGGGAA CCCTGGTCACCGTCTCCTCA |
|---|---|---|---|---|
| | | | SEQ ID NO: 24489 | SEQ ID NO: 28495 |
| | | AA | DIQMTQSPSSLSASVGDRVTITCRASQGVRNDLG WYQQKPGKAPQRLIYAASSLQSGVPSRFNGSGS GTEFTLTISSLQPEDFATYYCLQHSSYPLTFGGGT KVEIK | QLQLQESGPGLVKFSETLSLTCTVSGGSISRSSYYW GWIRQPPGKGQEWIGNIYYSGYSYYNPSLKSRVTIS VDTSKNQFSLKLSSVTAADTAVYYCARHSTSWSLD YWGQGTLVTVSS |
| | | | SEQ ID NO: 24490 | SEQ ID NO: 28496 |
| iPS:392898 | 21-225_21H10 | NA | GAAATTGTGTTGACGCAGTCTCCAGGCACCCT GTCTTTGTCTCCAGGGGAAAGAGCCACCCTCT CCTGCAGGGCCAGTCAGAGTTTTAGCAGCAGC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAGCTCACGC AGTTTTGGCCAGGGGACCAAGCTGGAGATCAA A | GAGGTGCAGCTGGTGGAGTCTGGGGGAGGCTTG GTAAAGCCTGGGGGGTCCCTTAGACTCTCCTGTG CAGCCTCTGGATTCACTTTCAGTAACGCCTGGAT GAACTGGGTCCGCCAGGCTCCAGGGAAGGGGCT GGAGTGGGTTGGCCGTATTAAAAGCAAAACTGA TGGTGGGACAACAGACTACGCTGCACCCGTGAA AGGCAGATTCACCATCTCAAGAGATGATTCAAA AAACACGCTGTATCTGCAAATGAACAGCCTGAA AACCGAGGACACAGCCGTGTATTACTGTACCACA GAAGGCTGGAACACGGACTACTGGGGCCAGGGA ACCCTGGTCACCGTCTCCTCA |
| | | | SEQ ID NO: 24491 | SEQ ID NO: 28497 |

1523

FIGURE 50

| | | | | |
|---|---|---|---|---|
| | | AA | EIVLTQSPGTLSLSPGERATLSCRASQSFSSSYLA WYQQKPGQAPRLLIYGASSRATGIPDRFSGSGSG TDFTLTISRLEPEDFAVYYCQQYGSSRSFGQGTK LEIK | EVQLVESGGGLVKPGGSLRLSCAASGFTFSNAWM NWVRQAPGKGLEWVGRIKSKTDGGTTDYAAPVK GRFTISRDDSKNTLYLQMNSLKTEDTAVYYCTTEG WNTDYWGQGTLVTVSS |
| | | | SEQ ID NO: 24492 | SEQ ID NO: 28498 |
| IPS:392900 | 21-225_22F2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGAGGGAAG TAATAAATACTATGTAGACTCCGTGAGGGGCCGA TTCACCATCTCCAGAGACAATTCCAAGAGCACGC TGTATCTGCAAATGAACAGCCTGAGAGCCGAGG ACACGGCTGTTTATTACTGTGTGAGAGAGCTAGG CTTCCAGTCTGACTACTGGGGCCAGGGAACCCCG GTCACCGTCTCCTCA |
| | | | SEQ ID NO: 24493 | SEQ ID NO: 28499 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYEGSNKYYVDSVRGR FTISRDNSKSTLYLQMNSLRAEDTAVYYCVRELGF QSDYWGQGTPVTVSS |
| | | | SEQ ID NO: 24494 | SEQ ID NO: 28500 |

FIGURE 50

| iPS:392902 | 21-225_22D5 | NA | GACATCCAGATGACCCAGTCTCCATCCTCCCT GTCTGCATCTGTAGGAGACAGAGTCACCATCA CTTGCCGGGCAAGTCAGAACATTTTTAGTTAT TTAAATTGGTATCATCAGAAACCAGGGAAAGC CCCTAAGCTCCTGATCTATGCTGCATCCAGTTT GCAAAGTGGGGTCCCATCAAGGTTCAGTGGCA GTGGATCTGGGACAGATTTCACTCTCACCATC AGCAGTCTACAACCTGAAGATTTTGCAACTTA CTACTGTCAACAGAGTTACAGTACCCCCTTAT TCACTTTCGGCCCTGGGACCAAAGTGGATATC AAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAGCTGGGTCCGCCAGGCTCCAGGGAAGGGGCT GGAGTGGGTCTCAGTTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTATAGCA GTGGCTGGCTCGGAGGCTTTTGATATCTGGGGCC AAGGGACAATGGTCACCGTCTCTTCA |
| | | | SEQ ID NO: 24495 | SEQ ID NO: 28501 |
| | | AA | DIQMTQSPSSLSASVGDRVTITCRASQNIFSYLN WYHQKPGKAPKLLIYAASSLQSGVPSRFSGSGS GTDFTLTISSLQPEDFATYYCQQSYSTPLFTFGPG TKVDIK | EVQLLESGGGLVQPGGSLRLSCAASEFTFSSYAMS WVRQAPGKGLEWVSVISGRGGNTFYADSVKGRFTI SRDNSKNTLYLQMNSLRAEDTAVYYCASRIAVAGS EAFDIWGQGTMVTVSS |
| | | | SEQ ID NO: 24496 | SEQ ID NO: 28502 |
| iPS:392904 | 21-225_22G9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGAAAAG CCCCTAAGCGCCTGATCTATGCTGCTTCCAGTT TGCAAAGTGGGGTCCCATCAAGGTTCAGCGGC AGTGGATCTGGGACAGAGTTCACTCTCACAAT CAGCAGCCTGCAGCCTGAAGATTTTGCAACTT ATTACTGTCTACAGCATGCCAGTTACCCCGCTC ACTTTCGGCGGAGGGACCAAGGTGGAGATCA AA | CAGCTGCAGCTGCAGGAGTCGGGGCCCAGGACTG GTGAAGCCTTCGGAGACCCTGTCCCTCACCTGCA CTGTCTCCGGTGGCGCCATCAGCGGTAGTAATTA CTACTGGGGCTGGATCCGCCAGCCCCCAGGGAA GGAACTGGAGTGGATTGGAAATATCTATTATAGT GGGAGCACCTACTACAACCCGTCCCTCAAGAGTC GAGTCACCATATCCGTAGACACGTCCAAGAACC AGTTCTCCCTGAAGCTGAGGTCTGTGACCGCCGA AGACACGGCTGTGTATTACTGTGCGAGACATAGC AGTAGCTGGTCCCTTGACTACTGGGGCCAGGGAA CCCTGGTCACCGTCTCCTCA |
| | | | SEQ ID NO: 24497 | SEQ ID NO: 28503 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHASYPLTFGGG TKVEIK | QLQLQESGPGLVKPSETLSLTCTVSGGAISGSNYYW GWIRQPPGKELEWIGNIYYSGSTYYNPSLKSRVTIS VDTSKNQFSLKLRSVTAEDTAVYYCARHSSSWSLD YWGQGTLVTVSS |
| | | | SEQ ID NO: 24498 | SEQ ID NO: 28504 |
| IPS:392908 | 21-225_23F12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGTTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAAGTT ATTACTGTCTACAGCATAATAGTTACCCGTGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG TAGCGTCTGGATTCACCTTCAGTAGCTATGGCAT GCACTGGGTCCGCCAGGCTCCAGGCAAGGGGCT GGAGTGGGTGGCAGTTATATGGTATGATGAAACT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTATTTATTACTGTGCGAGAGAGCTTGC CTGGTACGAGGACTACTGGGGCCAGGGATCCCT GGTCACCGTCTCCTCA |
| | | | SEQ ID NO: 24499 | SEQ ID NO: 28505 |
| | | AA | DIQMTQSPSSLSASVGDRVTITCRASQGIRNDLG WYQQKPGKAPKRLIYVASSLQSGVPSRFSGSGS GTEFTLTISSLQPEDFASYYCLQHNSYPWTFGQG TKVEIK | QVQLVESGGGVVQPGRSLRLSCVASGFTFSSYGMH WVRQAPGKGLEWVAVIWYDETNKYYADSVKGRF TISRDNSKNTLYLQMNSLRAEDTAIYYCARELAWY EDYWGQGSLVTVSS |
| | | | SEQ ID NO: 24500 | SEQ ID NO: 28506 |

FIGURE 50

| iPS:392912 | 21-225_25A9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGTGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGTAACT AATAAATACTATACAGGCTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGCGAGAGAAATTGG CTGGTTAGATGACTACTGGGGCCAGGGAACCCTG GTCACCGTCTCCTCA |
| | | | SEQ ID NO: 24501 | SEQ ID NO: 28507 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSSYGMH WVRQAPGKGLEWVAVIWYDVTNKYYTGSVKGRF TISRDNSKNTLYLQMNSLRAEDTAVYYCAREIGWL DDYWGQGTLVTVSS |
| | | | SEQ ID NO: 24502 | SEQ ID NO: 28508 |
| iPS:392914 | 21-225_25D12 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTTCCCTCGG ACGTTCGGCCAAGGGACCAAAGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCGATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAGCAGCAGCTGGTACGACTACGGTATGGACGTC TGGGGCCAAGGGACCACGGTCACCGTCTCCTCA |
| | | | SEQ ID NO: 24503 | SEQ ID NO: 28509 |

FIGURE 50

| | | | | |
|---|---|---|---|---|
| | | AA | DIQMTQSPSSLSASVGDRVTITCRTSQGIRNDLG WYQQKPGKAPKRLIYAASSLQSGVPSRFSGSGS GTEFTLTISSLQPEDFATYYCLQHYSFPRTFGQGT KVEIK | QVQLVESGGGVVQPGRSLRLSCAASGFTFSSDGMH WVRQAPGKGLEWVAVIWYDGSNKYYADSVKGRF TISRDNSKNTLYLQMNSLRAEDTAVYYCARERYSS SWYDYGMDVWGQGTTVTVSS |
| | | | SEQ ID NO: 24504 | SEQ ID NO: 28510 |
| IPS:392916 | 21-225_27C5 | NA | GACATCCAGATGACCCAGTCTCCCTCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGTTCCTGATCTATGGTGCATCCAGTT TGCAAAGTGGGGTCCCATCAAGGTTCAGCGCC AGTGGATCTGGGACAGAGTTCACTCTCACCAT CAGCAGCCTGCAGCCTGAAGATTTTGCAACTT ACTGTTGTCAACAGTATGACAGTTTCCCTCGG ACGTTCGGCCAAGGGACCAAGGTGGAAATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCACTAGTAGTAGTGATAGT TATATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTGGCGTCC TTTGACTGCTGGGGCCAGGGAACCCTGGTCACCG TCTCCTCA |
| | | | SEQ ID NO: 24505 | SEQ ID NO: 28511 |
| | | AA | DIQMTQSPSSVSASVGDRVTITCRASQGISSWLA WYQQKPGKAPKFLIYGASSLQSGVPSRFSASGSG TEFTLTISSLQPEDFATYCCQQYDSFPRTFGQGTK VEIK | EVQLVESGGGLVKPGGSLRLSCAASGFTFSSYSMN WVRQAPGKGLEWVSSTSSSDSYIYYADSVKGRFTI SRDNAKNSLYLQMNSLRAEDTAVYYCARVASFDC WGQGTLVTVSS |
| | | | SEQ ID NO: 24506 | SEQ ID NO: 28512 |

FIGURE 50

| iPS:392918 | 21-225_28F5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATACTTACCCGTG GACGTTCGGCCAAGGGACCAAGGTGGAAGTC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAATTAGG CTGGTACGACGACTACTGGGGCCAGGGAACCCT GGTCACCGTCTCCTCA |
| | | | SEQ ID NO: 24507 | SEQ ID NO: 28513 |
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHNTYPWTFGQGT KVEVK | QVQLVESGGGVVQPGRSLRLSCAASGFTFSSYGMH WVRQAPGKGLEWVAVIWYDENNKYYADSVKGRF TISRDNSKNTLYLQMNSLRAEDTAVYYCARELGW YDDYWGQGTLVTVSS |
| | | | SEQ ID NO: 24508 | SEQ ID NO: 28514 |
| iPS:392920 | 21-225_29G4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA CA | CAGGTGCAGCTGGTGGAGTCTGGGGGAGGCGTG GTCCAGCCTGGGAGGTCCCTGAGACTCACCTGTG CAGCGTCTGGATTCACCTTCAGTGACTATGGCAT ACACTGGGTCCGCCAGGCTCCAGGCAAGGGGCT GGAGTGGGTGGCAGTTATATGGTATGATGAAAGT AATAAATACTATGCAGACTCCATGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACTGGG AATGACGGGTGACTACTGGGGCCAGGGAACCCT GGTCACCGTCTCCTCA |
| | | | SEQ ID NO: 24509 | SEQ ID NO: 28515 |

1529

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIT | QVQLVESGGGVVQPGRSLRLTCAASGFTFSDYGIH WVRQAPGKGLEWVAVIWYDESNKYYADSMKGRF TISRDNSKNTLYLQMNSLRAEDTAVYYCARELGMT GDYWGQGTLVTVSS |
| | | | SEQ ID NO: 24510 | SEQ ID NO: 28516 |
| iPS:392922 | 21-225_30G4 | NA | GACATCCAGATGACCCAGTCTCCACCCTCCCT GTCTACATCTGTAGGAGACAGAGTCACCATCA CTTGCCGGGCAACTCAGAACATTTTCAGCTAT TTAAAATTGGCATCAGCAGAAACCAGGGAAAG CCCCTAAGCTCCTGATCCATACTGCATCCAGTT TGCAAGGTGGGGTCCCATCAAGGTTCAGTGGC AGTGGATCTGGGACAGATTTCACTCTCACCAT CATCAGTATGCAACCTGAAGATTTTTCCACTT ACTACTGTCAACTCAGCTACAGTCCCCCGTAC ACTTTTGGCGGAGGGACCAAGGTGGAGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCCGGGGGCT GGAATGGGTGGCAGTTATATGGTATGATGGAACT GATAAATACTATGTAGACTCCGTGAAGGGCCGAT TCACCATCTCCAGAGACAATTCCAAGAACACGCT GTATCTGCAAATGAACAGCCTGAGAGCCGAGGA CTCGGCTGTGTATTACTGTGCGAGAGAAAATAGC AGCTCGTACTACTTTGACTACTGGGGCCAGGGAA CCCTGGTCACCGTCTCCTCA |
| | | | SEQ ID NO: 24511 | SEQ ID NO: 28517 |
| | | AA | DIQMTQSPPSLSTSVGDRVTITCRATQNIFSYLN WHQQKPGKAPKLLIHTASSLQGGVPSRFSGSGS GTDFTLTIISMQPEDFSTYYCQLSYSPPYTFGGGT KVEIK | QVQLVESGGGVVQPGRSLRLSCAASGFTFSSYGMH WVRQAPGRGLEWVAVIWYDGTDKYYVDSVKGRF TISRDNSKNTLYLQMNSLRAEDSAVYYCARENSSS YYFDYWGQGTLVTVSS |
| | | | SEQ ID NO: 24512 | SEQ ID NO: 28518 |

FIGURE 50

| iPS:392924 | 21-225_32H2 | NA | GATATTGTGATGACCCAGACTCCACTCTCTCT GTCCGTCACCCCTGGACAGCCGGCCTCCATCT CCTGCAGGTCTAGTCAGAGCCTCCTCCATAGT GATGGAAGGACCTATTTGTATTGGTACCTGCA GAAGCCAGGCCAGCCTCCACAGCTCCTGATCT ATGAACTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTCCTGCTTGCAAAG TATACAATATCCCATCACCTTCGGCCAAGGGA CACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGAATCTGCAAATGAACAGCCTGAGAGCCGAGG ACACGGGTGTGTATTACTGTGCGAGAAGATATAG CAGCAGCTGGACGGGGGGTATGGACGTCTGGGG CCAAGGGACCACGGTCACCGTCTCCTCA |
| | | | SEQ ID NO: 24513 | SEQ ID NO: 28519 |
| | | AA | DIVMTQTPLSLSVTPGQPASISCRSSQSLLHSDGR TYLYWYLQKPGQPPQLLIYELSNRFSGVPDRFSG SGSGTDFTLKISRVEAEDVGIYSCLQSIQYPITFG QGTRLEIK | QVQLVESGGGVVQPGRSLRLSCAASGFTFSSYGMH WVRQAPGKGLEWVAVIWYDGSNKYYADSVKGRF TISRDNSKNTLNLQMNSLRAEDTGVYYCARRYSSS WTGGMDVWGQGTTVTVSS |
| | | | SEQ ID NO: 24514 | SEQ ID NO: 28520 |
| iPS:392928 | 21-225_25A4 | NA | GACATCGTGATGACCCAGTTTCCAGACTCCCT GGCTGTGTCTCTGGGCGAGAGGGGCCACCATCA AGTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGGAGG CTGAAGATGTGGCAGTTTATTACTGTCAGCAG TATTATAGTACTCCTCCGACGTTCGGCCAAGG GACCAAGGTGGAAATCAAA | CAGGTGCTCCTGGTGCAGTCTGGGGCTGAGGTGA AGAGGCCTGGGGCCTCAGTGAAGGTCTCCTGCA AGGCTTCTGGATACACCTTCACCAATTATGATAT TAATTGGGTGCGACAGGCCACTGGACAAGGGCT TGAGTGGATGGGATGGATGTACCCTAACAGTGGT AACACAGGCTATGCACAGAAATTCCAGGGCAGA GTCACCATGACCAGGAACACCTCCATAAGCACA GCCTACATGGAGCTGAGCAGCCTGAGATCTGAG GACACGGCCGTCTATTACTGTGCGAGTAGCAGTG GCTGGTACTACTTTGACTACTGGGGCCAGGGAAC CCTGGTCACCGTCTCCTCA |
| | | | SEQ ID NO: 24515 | SEQ ID NO: 28521 |

FIGURE 50

| | | | | |
|---|---|---|---|---|
| | | AA | DIVMTQFPDSLAVSLGERATIKCKSSQSVLYSSH NNNYLAWYQQKPGQPPKLLLYWASTRESGVPD RFSGSGSGTEFTLTISSLEAEDVAVYYCQQYYST PPTFGQGTKVEIK | QVLLVQSGAEVKRPGASVKVSCKASGYTFTNYDIN WVRQATGQGLEWMGWMYPNSGNTGYAQKFQGR VTMTRNTSISTAYMELSSLRSEDTAVYYCASSSGW YYFDYWGQGTLVTVSS |
| | | | SEQ ID NO: 24516 | SEQ ID NO: 28522 |
| iPS:392930 | 21-225_25H9 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTTTTGGTACCTGCA GAAGCCAGGCCAGCCTCCACAGCTCCTGATCT ATGAAGTTTCCAATCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCCCCTTCAATAACTATGGCAT GCACTGGGTCCGCCAGGCTCCAGGCAAGGGGCT GGAGTGGGTGTCAATTATATGGTATGATGGAAGT TATAAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAGGGATAC GATTTTTGGAGTGGCTTCTTTGACTCCTGGGGCC AGGGAACCCTGGTCACCGTCTCCTCA |
| | | | SEQ ID NO: 24517 | SEQ ID NO: 28523 |
| | | AA | DIVMTQTPLSLSVTPGQPASISCKSSQSLLHGDG KTYLFWYLQKPGQPPQLLIYEVSNRFSGVPDRFS GSGSGTDFTLKISRVEAEDVGVYYCMQSIQLPW TFGQGTKVEIK | QVQLVESGGGVVQPGRSLRLSCAASGFPFNNYGM HWVRQAPGKGLEWVSIIWYDGSYKYYADSVKGRF TISRDNSKNTLYLQMNSLRAEDTAVYYCAREGYDF WSGFFDSWGQGTLVTVSS |
| | | | SEQ ID NO: 24518 | SEQ ID NO: 28524 |

EP 4 435 105 A2

FIGURE 50

| iPS:392934 | 21-225_27D5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAACAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAACTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAGT AATAAATACTATGGAGACTCCGTGAAGGGCCGA TTCACCATCTCCAGAGACAATTCCAAGAATATGC TGTATCTGCAAATGAACAGCCTGAGAGGCGAGG ACACGGCTCTGTATTACTGTGCGAGAGAACTGGG GTTCCTCTCTGACTACTGGGGCCAGGGAACCCTG GTCACCGTCTCCTCA |
|---|---|---|---|---|
| | | | SEQ ID NO: 24519 | SEQ ID NO: 28525 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSSYGMH WVRQAPGKGLEWVAVIWYDESNKYYGDSVKGRF TISRDNSKNMLYLQMNSLRGEDTALYYCARELGFL SDYWGQGTLVTVSS |
| | | | SEQ ID NO: 24520 | SEQ ID NO: 28526 |
| iPS:392936 | 21-225_28B6 | NA | GATGTTGTGATGACTCAGTCTCCACTCTCCCTG CCCGTCACCCTTGGACAGCCGGCCTCCATCTC CTGCCGGTCTAGTCAAAGCCTCGTATATAGTG ATGGAGACACCTACTTGAATTGGTTTCAGCAG AGGCCAGGCCAATCTCCAAGGCGCCAAATTTA TAAGGTTTCTAACTGGGACTCTGGGGTCCCAG ACAGATTCAGCGGCAGTGGGTCAGGCACTGAT TTCACACTGAACATCAGCAGGGTGGAGGCTGA GGATGTTGGGATTTATTTCTGCATGCATTGTAC ACACTGGCTCCTTTTCGGCCCTGGGACCAAAG TGGATATCAAA | CAGGTGCTCCTGGTGCAGTCTGGGGCTGAGGTGA AGAGGCCTGGGGCCTCAGTGAAGGTCTCCTGCA AGGCTTCTGGATACACCTTCACCAATTATGATAT TAATTGGGTGCGACAGGCCACTGGACAAGGTCTT GAGTGGATGGGATGGATGCACCCTGACAGTGGT AACACAGGCTATGCACAGAAGTTCCAGGGCAGA GTCACCATGACCAGGAACACCTCCATAAGCACG GCCTACATGGAGCTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTCTCCTCA |
| | | | SEQ ID NO: 24521 | SEQ ID NO: 28527 |

1533

FIGURE 50

| | | | | | |
|---|---|---|---|---|---|
| | | AA | DVVMTQSPLSLPVTLGQPASISCRSSQSLVYSDG DTYLNWFQQRPGQSPRRQIYKVSNWDSGVPDRF SGSGSGTDFTLNISRVEAEDVGIYFCMHCTHWLL FGPGTKVDIK | QVLLVQSGAEVKRPGASVKVSCKASGYTFTNYDIN WVRQATGQGLEWMGWMHPDSGNTGYAQKFQGR VTMTRNTSISTAYMELSSLRSEDTAVYYCASSSGW YYFDYWGQGTLVTVSS | |
| | | | SEQ ID NO: 24522 | SEQ ID NO: 28528 | |
| iPS:392938 | 21-225_29H4 | NA | GATATTGTGATGACCCAGACTCCACTCTCTCT GTCCGTCACCCCTGGACAGCCGGCCTCCTTCT CCTGCAAGTCTAGTCAGAGCCTCCTGCATAGT GATGGAAAGACCTATTTGTATTGGTACCTGCA GAAGCCAGGCCAGCCTCCACAGCTCCTGATCT TTGAGGTTTCCCACCGGTTCTCTGGACTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCATCCGTTCACTTTCGGCGGAGGGA CCAGGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT ACACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCAGGGGGGTATGGACGTCTGGGG CCAAGGGACCACGGTCACCGTCTCCTCA | |
| | | | SEQ ID NO: 24523 | SEQ ID NO: 28529 | |
| | | AA | DIVMTQTPLSLSVTPGQPASFSCKSSQSLLHSDG KTYLYWYLQKPGQPPQLLIFEVSHRFSGLPDRFS GSGSGTDFTLKISRVEAEDVGVYYCMQSIQHPFT FGGGTRVEIK | QVQLVESGGGVVQPGRSLRLSCAASGFTFSSYGIH WVRQAPGKGLEWVAVIWYDGSNKYYADSVKGRF TISRDNSKNTLYLQMNSLRAEDTAVYYCARRYSSS WSGGMDVWGQGTTVTVSS | |
| | | | SEQ ID NO: 24524 | SEQ ID NO: 28530 | |

FIGURE 50

| iPS:392940 | 21-225_29D9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTTTCAGCAGAAACCGGGGAAAGC CCCTAAGCGCCTGATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATAATACTTACCCATTCAC TTTCGGCCCTGGGACCAAAGTGGATTTCAAA | CAGGTGCAGCTGGTGGAGTCTGGGGGAGGCGTG GTCCAGCCTGGGAGGTCCCTGAAACTCTCCTGTT CAGCGTCTGGATTCACCTTCAGTGACTATGGCAT TCACTGGGTCCGCCAGGCTCCAGGCAAGGGGCT GGAGTGGGTGGCAGTTATATGGTATGATGAAAGT AATAACTACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTACTACTGTGCGAGAGAAATTGGC TGGTTAGATGACTACTGGGGCCAGGGAACCCAG GTCACCGTCTCCTCA |
|---|---|---|---|---|
| | | | SEQ ID NO: 24525 | SEQ ID NO: 28531 |
| | | AA | DIQMTQSPSSLSASVGDRVTITCRASQGIRNDLG WFQQKPGKAPKRLIYAASSLQSGVPSRFSGSGSG TEFTLTISSLQPEDFATYYCLQHNTYPFTFGPGTK VDFK | QVQLVESGGGVVQPGRSLKLSCSASGFTFSDYGIH WVRQAPGKGLEWVAVIWYDESNNYYADSVKGRF TISRDNSKNTLYLQMNSLRAEDTAVYYCAREIGWL DDYWGQGTQVTVSS |
| | | | SEQ ID NO: 24526 | SEQ ID NO: 28532 |
| iPS:392942 | 21-225_30E9 | NA | GACATCCAGATGACCCAGTCTCCATCCTCCCT GTCTGCATCTGTAGGAGACAGAGTCACCATCA CTTGCCGGGCAAGTCAGGACATTAGAGATGAT TTAGGCTGGTATCAGCAGAAACCAGGGAAAG CCCCTAGGCGCCTGATCTATGGTGCATTCAGC TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATACTAGTTACCCTCCT ACTTTCGGCGGAGGGACCAAGGTGGAGATCA AA | GAGGTGCAGGTGTTGGAGTCTGGGGGAGGCTTG GTACAGCCTGGGGGGTCCCTGAGACTCTCCTGTG CAGCCTCTGGATTCACCTTTAGCAGCTGTGCCAT GAACTGGGTCCGCCAGGCTCCAGGGAAGGGGCT GGAGTGGGTCTCCGCTATTAGTGGTCGTGGTGGT AGCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAAGGGGAGCT ACTAGAGGACTACTACTACGGAATGGACGTC TGGGGCCAAGGGACCACGGTCACCGTCTCCTCA |
| | | | SEQ ID NO: 24527 | SEQ ID NO: 28533 |

FIGURE 50

| | | | | |
|---|---|---|---|---|
| | | AA | DIQMTQSPSSLSASVGDRVTITCRASQDIRDDLGWYQQKPGKAPRRLIYGAFSLQSGVPSRFSGSGSGTEFTLTISSLQPEDFATYYCLQHTSYPPTFGGGTKVEIK | EVQVLESGGGLVQPGGSLRLSCAASGFTFSSCAMNWVRQAPGKGLEWVSAISGRGGSTFYADSVKGRFTISRDNSKNTLYLQMNSLRAEDTAVYYCAKGELLEDYYYYGMDVWGQGTTVTVSS |
| | | | SEQ ID NO: 24528 | SEQ ID NO: 28534 |
| iPS:392944 | 21-225_31H5 | NA | GACATCCAGATGACCCAGTCTCCATCCTCCCTGTCTGCATCTGTAGGAGACAGATTCACCATCTCTTTCCGGGCAAGTCAGGACATTAGAAGTGATTTAGGCTGGTATCAGCAGAAACCAGGGAAAGCCCATAAGCGCATCATCTATGCTGCATCCAGTTTGCAAAGTGGGGTCCCATCAAGGTTCAGCGTCAGTGGATCTGGGACAGAATTCACTTTCACAATCAGCAGCATGCAGCCTGACGATTTTTCAAATTATTACTGTATACAACATATTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGAAGCATATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGGAGCTACTAGAGGACTACTACTTCTACGGTATGGACGTCTGGGGCCAAGGGACCACGGTCACCGTCTCCTCA |
| | | | SEQ ID NO: 24529 | SEQ ID NO: 28535 |
| | | AA | DIQMTQSPSSLSASVGDRFTISFRASQDIRSDLGWYQQKPGKAHKRIIYAASSLQSGVPSRFSVSGSGTEFTFTISSMQPDDFSNYYCIQHIIYPPTFGGGTKVEIK | EVQLLESGGGLVQPGGSLRLSCAASGFTFSSYAMSWVRQAPGKGLEWVSAISGRGGSIFHADSVKGRFTISRDNSKNTLYLQMNSLRAEDTAVYYCVKGELLEDYYFYGMDVWGQGTTVTVSS |
| | | | SEQ ID NO: 24530 | SEQ ID NO: 28536 |

FIGURE 50

| iPS:392948 | 21-225_25G5 | NA | GACATCCAGATGACCCAGTCTCCATCCTCACT GTCTGCATCTGTTGGAGACAGAGTCACCATCA CTTGCCGGGCAAGTCAGGACATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAG TCAGCAGCCTGCAGCCTGAAGATTTTGCAACT TATTACTGTCTACAGCATAATAGTTACCCCTTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT ACACTGGGTCCGCCAGGCTCCAGGCAAGGGGCT GGAGTGGGTGGCAGTTATTTGGTATGATGGAAAT AATAAATATTATGCAGACTCCGTGAAGGGCCGAT TCACCATCTCCAGAGACAATTCCAAGAACACGCT GTATCTGCAAATGAACAACCTGAGAGCCGAGGA CACGGCTGTGTATTACTGTGCGAGAGAAATTGGC TGGTTAGATGACTACTGGGGCCAGGGAACCCTG GTCACCGTCTCCTCA |
| | | | SEQ ID NO: 24531 | SEQ ID NO: 28537 |
| | | AA | DIQMTQSPSSLSASVGDRVTITCRASQDIRNDLG WYQQKPGKAPKRLIYAASSLQSGVPSRFSGSGS GTEFTLTVSSLQPEDFATYYCLQHNSYPFTFGPG TKVDIK | QVQLVESGGGVVQPGRSLRLSCAASGFTFSDYGIH WVRQAPGKGLEWVAVIWYDGNNKYYADSVKGRF TISRDNSKNTLYLQMNNLRAEDTAVYYCAREIGWL DDYWGQGTLVTVSS |
| | | | SEQ ID NO: 24532 | SEQ ID NO: 28538 |
| iPS:392950 | 21-225_25C10 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTTCCCATCAAAGTTCAGCGGCA GTGGATCTGGGACAGATTTCACTCTCACCATC AGCAGCCTGCAGCCTGAAGATTTTGCAACTTA TTACTGCCAACAGTATCATAGTTACCCATTCA CTTTCGGCCCTGGGACCAAAGTGGATATCAAA | GAGGTGCAGTTGGTGGAGTCTGGGGGAGGCTTG GTACAGCCTGGGGGGTCCCTGAGACTCTCCTGTG CAGCCTCTGGATTCACCTTCAGTAGCTATAGGAT GAACTGGGTCCGCCAGGCTCCAGGGAAGGGGCT GGAGTGGGTTTCATCCATTAGTAGTAGTAGTAGT ACCATATACTACGCAGACTCTGTGAAGGGCCGAT TCACCATCTCCAGAGACAATGCCAAGAACTCACT GTATCTGCAAATGAACAGCCTGAGAGACGAGGA CACGGCTGTGTATTACTGTGCGAGAACGGCTGGT TTTGACTACTGGGGCCAGGGAACCCTGGTCACCG TCTCCTCA |
| | | | SEQ ID NO: 24533 | SEQ ID NO: 28539 |

FIGURE 50

| | | | | |
|---|---|---|---|---|
| | | AA | DIQMTQSPSSLSASVGDRVTITCRASQGISNYLA WFQQKPGKAPKSLIYAASSLQSGFPSKFSGSGSG TDFTLTISSLQPEDFATYYCQQYHSYPFTFGPGT KVDIK | EVQLVESGGGLVQPGGSLRLSCAASGFTFSSYRMN WVRQAPGKGLEWVSSISSSSSTIYYADSVKGRFTIS RDNAKNSLYLQMNSLRDEDTAVYYCARTAGFDY WGQGTLVTVSS |
| | | | SEQ ID NO: 24534 | SEQ ID NO: 28540 |
| IPS:392952 | 21-225_26G1 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTATGCTGCATCCAGTTT GCGAAGTGGGGTCCCATCAAACTTCAGCGGCA GTGGATCTGGGACAGATTTCACTCTCACCATC AGCAGCCTGCAGCCTGAAAATTTTGCAACTTA TTACTGCCAACAGTATCATAGTTACCCATTCA CTTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATGGCAT GAACTGGGTCCGCCAGGCTCCAGGGAAGGGGCT GGAGTGGGTCTCATCCATTAGTGGTAGTAGTAGT TACATATACTACGCGGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGACTGACTACC TTTGACTTCTGGGGCCAGGGAACCCTGGTCACCG TCTCCTCA |
| | | | SEQ ID NO: 24535 | SEQ ID NO: 28541 |
| | | AA | DIQMTQSPSSLSASVGDRVTITCRASQDISNYLA WFQQKPGKAPKSLIYAASSLRSGVPSNFSGSGSG TDFTLTISSLQPENFATYYCQQYHSYPFTFGPGT KVDIK | EVQLVESGGGLVKPGGSLRLSCAASGFTFSSYGMN WVRQAPGKGLEWVSSISGSSSYIYYADSVKGRFTIS RDNAKNSLYLQMNSLRAEDTAVYYCARLTTFDFW GQGTLVTVSS |
| | | | SEQ ID NO: 24536 | SEQ ID NO: 28542 |

FIGURE 50

| | | | | |
|---|---|---|---|---|
| iPS:392954 | 21-225_26A10 | NA | GACATCCAGATGACCCAGTCTCCATCCTCCCT GTCTGCATCGTAGGAGACAGAATCACCATCA CTTGCCGGGCAAGTCAGAGCATTAGCAGCTAT TTAAATTGGTATCAGCAGAAACCAGGGAAAG CCCCTAAGGTCCTGATCTATGCTGCATCCAGC TTGCAAAGTGGGGTCCCATCAAGATTCAGTGG CAGTGGATCTGGGACAGATTTCACTCTCACCA TCAGCAGTCTGCAACCTGAAGATTTTGCAACT TACTACTGTCAACAGAGTTACAGTACCCCTAC GTGGACGTTCGGCCAAGGGACCAAGGTGGAA ATCAAA | GAGGTGCAGCTGCTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGTCT GGAGTGGGTCTCAGTTATTAGTGGTAGTGGTGTT AACACATTCTACGCAGACTCCGTGAAGGGCCGGT TCACCATCTCCAGAGACAATTCCAAGAACACGCT GTATCTGCTAATGAACAGCCTGAGAGCCGAGGA CACGGCCGTATATTACTGTGCGAAAAAGATAGC AGTGGCTGGTACTCACTACTTTGACTACTGGGGC CAGGGAACCCTGGTCACCGTCTCCTCA |
| | | | SEQ ID NO: 24537 | SEQ ID NO: 28543 |
| | | AA | DIQMTQSPSSLSASVGDRITITCRASQSISSYLNW YQQKPGKAPKVLIYAASSLQSGVPSRFSGSGSGT DFTLTISSLQPEDFATYYCQQSYSTPTWTFGQGT KVEIK | EVQLLESGGGLVQPGGSLRLSCAASGFTFSSYAMS WVRQAPGKGLEWVSVISGSGVNTFYADSVKGRFTI SRDNSKNTLYLLMNSLRAEDTAVYYCAKKIAVAG THYFDYWGQGTLVTVSS |
| | | | SEQ ID NO: 24538 | SEQ ID NO: 28544 |
| iPS:392956 | 21-225_27A11 | NA | GACATCCAGATGACCCAGTCTCCCTCTTCCGT GTCTGCATCTGTAGGAGACAGAGTCACCATCA CTTGTCGGGCGAGTCAGGGTATTAGTAGTTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGCC AGTGGATCTGGGACAGATTTCACTCTCACCAT CAGCAGCCTGCAGCCTGAAGATTTTGCAACTT ACTGTTGTCAACAGTCTGACAGTTTCCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGCAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TATATCTACAAATGAACAGCCTGAGAGCCGAGG ACACGGCTGTGTATCACTGTGCGAGAGATTCCTC CCCCTACGGTATGGACGTCTGGGGCCAAGGGAC CACGGTCACCGTCTCCTCA |
| | | | SEQ ID NO: 24539 | SEQ ID NO: 28545 |

FIGURE 50

| | | | | |
|---|---|---|---|---|
| | | AA | DIQMTQSPSSVSASVGDRVTITCRASQGISSWLA WYQQKPGKAPKLLIYGASSLQSGVPSRFSASGS GTDFTLTISSLQPEDFATYCCQQSDSFPRTFGQGT KVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYHCARDSSPY GMDVWGQGTTVTVSS |
| | | | SEQ ID NO: 24540 | SEQ ID NO: 28546 |
| IPS:392958 | 21-225_28C7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATATTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATACTTACCCGTGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAATTAGG CTGGTACGACGACTACTGGGGCCAGGGAACCCT GGTCACCGTCTCCTCT |
| | | | SEQ ID NO: 24541 | SEQ ID NO: 28547 |
| | | AA | DIQMTQSPSSLSASVGDRVTITCRASQGIRNDLG WYQQKPGKAPKRLIYIASSLQSGVPSRFSGSGSG TEFTLTISSLQPEDFATYYCLQHNTYPWTFGQGT KVEIK | QVQLVESGGGVVQPGRSLRLSCAASGFTFSSYGMH WVRQAPGKGLEWVAVIWYDESNKYYADSVKGRF TISRDNSKNTLYLQMNSLRAEDTAVYYCARELGW YDDYWGQGTLVTVSS |
| | | | SEQ ID NO: 24542 | SEQ ID NO: 28548 |

FIGURE 50

| iPS:392960 | 21-225_29E6 | NA | GACATCGTGATGACCCAGTTTCCAGACTCCCTGGCTGTGTCTCTGGGCGAGAGGGCCACCATCAACTGTAAGTCCAGCCAGAGTGTTTTATACAGCTCCCACAATAACTACTACTTAACTTGGTACCAGCAGAAACCAGGACAGCCTCATAAGTTGCTCCTTTACTGGGCATCTTCCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGGCTGAGGTGAAGAGGCCTGGGGCCTCAGTGAAGGTCTCCTGCAAGGCTTCTGGATACACCTTCACCAATTATGATATTAATTGGGTGCGACAGGCCACTGGACAAGGGCTTGAGTGGATGGGATGGATGCACCCTAACAGTGGTAACACAGGCTATGCACAGAAATTCCAGGGCAGAGTCACCATGACCAGGAACACCTCCATAAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTCTCCTCA |
|---|---|---|---|---|
| | | | SEQ ID NO: 24543 | SEQ ID NO: 28549 |
| | | AA | DIVMTQFPDSLAVSLGERATINCKSSQSVLYSSHNNYYLTWYQQKPGQPHKLLLYWASSRESGVPDRFSGSGSGTEFTLTISSLQAEDVAVYYCQQYYSTPPTFGQGTKVEIK | QVLLVQSGAEVKRPGASVKVSCKASGYTFTNYDINWVRQATGQGLEWMGWMHPNSGNTGYAQKFQGRVTMTRNTSISTAYMELSSLRSEDTAVYYCASSSGWYYFDYWGQGTLVTVSS |
| | | | SEQ ID NO: 24544 | SEQ ID NO: 28550 |
| iPS:392962 | 21-225_30A1 | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTCTGCTGCATCCAGTTTGCAAACTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATATCAAA | GAGGTGCAGCTGTTGGAGTCTGGGGGAGGCCTGGTACAGCCGGGGGGGTCCCTGAGACTCTCCTGTGCAGCCTCTGGATTCACCTTTAGCAGCTATGTCATGAACTGGGTCCGCCAGGCTCCAGGGAAGGGGCTGGAGTGGGTCTCAGCTATTAGTGGTAGTGGTGGTAGCACATACTACGCAGACTCCGTGAAGGGCCGGTTCACCATCTCCAGAAACAATTCCAAGAACACGCTGTATCTGCAAATGAACAGCCTGAGAGCCGAGGACACGGCCGTATATTACTGTGCGCGAACTGGGGTCTTTGACTACTGGGGCCAGGGAACCCTGGTCACCGTCTCCTCA |
| | | | SEQ ID NO: 24545 | SEQ ID NO: 28551 |

FIGURE 50

| | | | | |
|---|---|---|---|---|
| | | AA | DIQMTQSPSSLSASVGDRVTITCRASQGISNYLA WFQQKPGKAPKSLISAASSLQTGVPSKFSGSGSG TDFTLTISSLQPEDFATYYCQQYNSYPFTFGPGT KVDIK | EVQLLESGGGLVQPGGSLRLSCAASGFTFSSYVMN WVRQAPGKGLEWVSAISGSGGSTYYADSVKGRFTI SRNNSKNTLYLQMNSLRAEDTAVYYCARTGVFDY WGQGTLVTVSS |
| | | | SEQ ID NO: 24546 | SEQ ID NO: 28552 |
| iPS:392964 | 21-225_31A8 | NA | GACATCCAGATGACCCAGTCTCCATCCTCCCT GTCTGCATCTGTAGGAGACAGAGTCACCATCA CTTGCCGGGCAAGTCAGGACATTAGAAGTGAT TTAGGCTGGTATCAGCAGAAACCAGGGAAAG CCCCTAAGCGCCTGATCTATGCTGTATCCAGTT TGCAAAGTGGGGTCCCATCAAGGTTCAGCGGC AGTGGCTCTGGGACAGAATTCACTCTCACAAT CAGCAGCCTGCAGCCTGAAGATTTTGCCACTT ATTACTGTCTACAGCATACTATTTACCCTCCTA CTTTCGGCGGAGGGACCAAGGTGGAGATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCCGCTATTAGTGGTCGTGGTGGT AGCACATTCCACGCAGACTCCGTGAAGGGCCGG TTCACCATCTCCAGAGACAATTCCAAGAACACGC TGTATCTGCAAATGAACAGCCTGAGAGACGAGG ACACGGCCGTATATTACTGTGTGAAAGGGGAGCT ACTAGAGGACTACTACTTCTACGGTATGGACGTC TGGGGCCAAGGGACCACGGTCACCGTCTCCTCA |
| | | | SEQ ID NO: 24547 | SEQ ID NO: 28553 |
| | | AA | DIQMTQSPSSLSASVGDRVTITCRASQDIRSDLG WYQQKPGKAPKRLIYAVSSLQSGVPSRFSGSGS GTEFTLTISSLQPEDFATYYCLQHTIYPPTFGGGT KVEIK | EVQLLESGGGLVQPGGSLRLSCAASGFTFSSYAMS WVRQAPGKGLEWVSAISGRGGSTFHADSVKGRFTI SRDNSKNTLYLQMNSLRDEDTAVYYCVKGELLED YYFYGMDVWGQGTTVTVSS |
| | | | SEQ ID NO: 24548 | SEQ ID NO: 28554 |

FIGURE 50

| iPS:392966 | 21-225_32G3 | NA | GACATCCAGATGACCCAGTCTCCAACCTCACT GTCTGCATCTGTCGGAGACAGAGTCACCATCA CTTGTCGGGCGAGTCAGGCCATTAGCAATTAT TTAGCCTGGTTTCAGCAGAAACCAGGGAAAGC CCCTAAGTCCCTGATCTATGATACATCCAGTTT GCAAAGTGGGGTCCCATCAAAATTCAGCGGCA GTGGGTCTGGGACAGATTTCACTCTCACCATC AGCACCCTACAGCCTGAAGATTTTGCAACTTA TTACTGCCAACAGTATCATAGTTACCCGCTCA CTTTCGGCGGAGGGACCAAGGTGGAGATCAA G | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATCAACAGCCTGAGAGCCGAGGA CACGGCTGTGTATTACTGTGCGAGAGGCAATATA GCAAGGGACTACTGGGGCCAGGGAACCCTGGTC ACCGTCTCCTCA |
|---|---|---|---|---|
| | | | SEQ ID NO: 24549 | SEQ ID NO: 28555 |
| | | AA | DIQMTQSPTSLSASVGDRVTITCRASQAISNYLA WFQQKPGKAPKSLIYDTSSLQSGVPSKFSGSGSG TDFTLTISTLQPEDFATYYCQQYHSYPLTFGGGT KVEIK | EVQLVESGGGLVKPGGSLRLSCAASGFTFSSYSMN WVRQAPGKGLEWVSSISGSSSYIYYADSVKGRFTIS RDNAKNSLYLQINSLRAEDTAVYYCARGNIARDY WGQGTLVTVSS |
| | | | SEQ ID NO: 24550 | SEQ ID NO: 28556 |
| iPS:392968 | 21-225_25B6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGAAAAG CCCCTAAGCGCCTGATCTATCGTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGAGTCTCCTGTA CAGCGTCTGGATTCACCCTCAGAAACTATGGCAT GCACTGGGTCCGCCAGGCTCCAGGCAAGGGGCT GGAGTGGGTGGCAGTTATATGGTATGAGGAAAG TAATAAATACTATACAGAGTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGAACTGG GGTTCCTCTCTGACTACTGGGGCCAGGGAACCCT GGTCACCGTCTCCTCA |
| | | | SEQ ID NO: 24551 | SEQ ID NO: 28557 |

FIGURE 50

| | | | | |
|---|---|---|---|---|
| | | AA | DIQMTQSPSSLSASVGDRVTITCRASQGIRNDLG WYQQKPGKAPKRLIYRASSLQSGVPSRFSGSGS GTEFTLTISSLQPEDFATYYCLQHNSYPFTFGPGT KVDIK | QVQLVESGGGVVQPGRSLRVSCTASGFTLRNYGM HWVRQAPGKGLEWVAVIWYEESNKYYTESVKGR FTISRDNSKNTLYLQMNSLRAEDTAVYYCARELGF LSDYWGQGTLVTVSS |
| | | | SEQ ID NO: 24552 | SEQ ID NO: 28558 |
| IPS:392972 | 21-225_26A2 | NA | GACATCCAGATGACCCAGTCTCCATCCTCCCT GTCTGCATCTGTAGGAGACAGAGTCACCATCA CTTGCCGGGCAAGTCAGGGCATTAGAAGTGAT TTAGGCTGGTATCAGCAGAAACCAGGGAAAG CCCCTAAGCGCCTGATCTATACTGCATCCAGT TTGCAGAGTGGGGTCCCATCAAGATTCAGCGG CAGTGGATCTGGGACAGAATTCACTCTCACAT TCAGCAGCCTGCAGCCTGAAGATTTTGCAACG TATTACTGTCTACAGCATAATCGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGAAGGAAG TAATAAATACTATGTAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAATTAGG CTGGTACGACGACTACTGGGGCCAGGGAACCCT GGTCACCGTCTCCTCA |
| | | | SEQ ID NO: 24553 | SEQ ID NO: 28559 |
| | | AA | DIQMTQSPSSLSASVGDRVTITCRASQGIRSDLG WYQQKPGKAPKRLIYTASSLQSGVPSRFSGSGSG TEFTLTFSSLQPEDFATYYCLQHNRYPWTFGQGT KVEIK | QVQLVESGGGVVQPGRSLRLSCAASGFTFSSYGMH WVRQAPGKGLEWVAVIWYEGSNKYYVDSVKGRF TISRDNSKNTLYLQMNSLRAEDTAVYYCARELGW YDDYWGQGTLVTVSS |
| | | | SEQ ID NO: 24554 | SEQ ID NO: 28560 |

1544

FIGURE 50

| iPS:392974 | 21-225_26A11 | NA | GACATCCAGATGACCCAGTCTCCAATTTCCCT GTCTGCATCTGTAGGAGACAGAGTCACCATCA CTTGCCGGGCAAGTCAGGCCATTAGAAATGAT TTAGGCTGGTATCAGCAGAGACCAGGGAAAG CCCCTAAGCGCCTGATCTATGCTGCATCCAGT TTGCAAAGTGGGGTCCCATCAAGGTTCAGCGG CAGTGGATCTGGGACAGAATTTATTCTCACAA TCAGCAGCCTGCAGCCTGAAGATTTTGCAACT TATTACTGTCTACAGCATTATAATTACCCTCGC AGTTTTGGCCAGGGGACCAAGCTGGAGATCAG A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTGTGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGAGTA TAGCAGTGGCTGGTACGACTACGGTATGGACGTC TGGGGCCAAGGGACCACGGTCACCGTCTCCTCA |
| | | | SEQ ID NO: 24555 | SEQ ID NO: 28561 |
| | | AA | DIQMTQSPISLSASVGDRVTITCRASQAIRNDLG WYQQRPGKAPKRLIYAASSLQSGVPSRFSGSGS GTEFILTISSLQPEDFATYYCLQHYNYPRSFGQGT KLEIR | QVQLVESGGGVVQPGRSLRLSCAASGFTFSNCVMH WVRQAPGKGLEWVAVIWYDGSNKYYADSVKGRF TISRDNSKNTLYLQMNSLRAEDTAVYYCAREEYSS GWYDYGMDVWGQGTTVTVSS |
| | | | SEQ ID NO: 24556 | SEQ ID NO: 28562 |
| iPS:392976 | 21-225_27H12 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCAATGGTGCATCCAGTT TGCAAAGTGGGGTCCCATCAAAGTTCAGCGGC AGTGGATCTGGGACAGATTTCACTCTCACCAT CAGCAGCCTGCAGCCTGAAGATTTTGCAACTT ATTACTGCCAACAGTATTATAGTTACCCATTC ACTTTCGGCCCTGGGACCAAAGTGAATATCAA T | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCCT GAACTGGGTCCGCCAGGCTCCAGGGAAGGGGCT GGAGTGGGTCTCATCCATTAGTGGTAGTAGTAGT AACATATACTACACAGACTCAGTGAAGGGCCGA TTCACCATCTCCAGAGACAACGCCAAGAACTCAC TGTTTCTGCAAATGAACAGCCTGAGAGCCGAGG ACACGGCTGTGTATTACTGTGCGAGAGTGGCGTC CTTTGACTACTGGGGCCAGGGAACCCTGGTCACC GTCTCCTCA |
| | | | SEQ ID NO: 24557 | SEQ ID NO: 28563 |

FIGURE 50

| | | | | |
|---|---|---|---|---|
| | | AA | DIQMTQSPSSLSASVGDRVTITCRASQGISNYLA WFQQKPGKAPKSLINGASSLQSGVPSKFSGSGSG TDFTLTISSLQPEDFATYYCQQYYSYPFTFGPGT KVNIN | EVQLVESGGGLVKPGGSLRLSCAASGFTFSSYSLN WVRQAPGKGLEWVSSISGSSSNIYYTDSVKGRFTIS RDNAKNSLFLQMNSLRAEDTAVYYCARVASFDYW GQGTLVTVSS |
| | | | SEQ ID NO: 24558 | SEQ ID NO: 28564 |
| IPS:392978 | 21-225_28B8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCACAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA T | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGACAGTTATATGGTATGATGCAAAT AATAAATACTATGCAGACTCCGTGAAGGGCCGA TTCACCATCTCCAGAGACAATTTCAAAAACACGG TGTATCTGCAAATGAACAGCCTGAGAGCCGAGG ACACGGCTGTGTATTACTGTGCGAGAGAAATTGG CTGGTTAGATGACTACTGGGGCCAGGGAACCCTG GTCACCGTCTCCTCA |
| | | | SEQ ID NO: 24559 | SEQ ID NO: 28565 |
| | | AA | DIQMTQSPSSLSASVGDRVTITCRASQGIRNDLG WYQHKPGKAPKRLIYAASSLQSGVPSRFSGSGS GTEFTLTISSLQPEDFATYYCLQHNSYPFTFGPGT KVDIN | QVQLVESGGGVVQPGRSLRLSCAASGFTFSDYGM HWVRQAPGKGLEWVTVIWYDANNKYYADSVKGR FTISRDNFKNTVYLQMNSLRAEDTAVYYCAREIGW LDDYWGQGTLVTVSS |
| | | | SEQ ID NO: 24560 | SEQ ID NO: 28566 |

FIGURE 50

| iPS:392980 | 21-225_29H6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAATCAGGGAAAA CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGTCTGCAGCCTGAAGATTTTGCAACC TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGACAGTTATATGGTATAATGAAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACTGGG GATGACGGGTGACTCCTGGGGCCAGGGAACCCT GGTCACCGTCTCCTCA |
| | | | SEQ ID NO: 24561 | SEQ ID NO: 28567 |
| | | AA | DIQMTQSPSSLSASVGDRVTITCRASQGIRNDLG WYQQKSGKTPKRLIYAASSLQSGVPSRFSGSGSG TEFTLTISSLQPEDFATYYCLQHNSYPLTFGGGT KVEIK | QVQLVESGGGVVQPGRSLRLSCAASGFTFSDYGM HWVRQAPGKGLEWVTVIWYNENNKYYADSVKGR FTISRDNSKNTLYLQMNSLRAEDTAVYYCARELGM TGDSWGQGTLVTVSS |
| | | | SEQ ID NO: 24562 | SEQ ID NO: 28568 |
| iPS:392982 | 21-225_30D1 | NA | GACATCCAGATGACCCAGTCTCCATCCTCCCT GTCTGCATCTGTAGGAGACAGAGTCACCATCA CTTGCCGGGCAAGTCAGGACATTAGAAGTGAT TTAGGCTGGTATCAACAGAAACCAGGGAAAG CCCCTGAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTACAACT TATTACTGTCTACAGCATACTATTTACCCTCCT ACTTTCGGCGGAGGGACCAAGGTGGAGATCA AA | GAGGTGCAGGTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCCGCTATTAGTGGTCGTGGTGGT AGCACATTCCACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGTGAAAGGGGGAGCT ACTAGAGGACTACTACTTCTACGGTTTGGACGTC TGGGGCCAAGGGACCACGGTCACCGTCTCCTCA |
| | | | SEQ ID NO: 24563 | SEQ ID NO: 28569 |

FIGURE 50

| | | | | |
|---|---|---|---|---|
| | | AA | DIQMTQSPSSLSASVGDRVTITCRASQDIRSDLG WYQQKPGKAPERLIYAASSLQSGVPSRFSGSGSG TEFTLTISSLQPEDFTTYYCLQHTIYPPTFGGGTK VEIK | EVQVLESGGGLVQPGGSLRLSCAASGFTFSSYAMS WVRQAPGKGLEWVSAISGRGGSTFHADSVKGRFTI SRDNSKNTLYLQMNSLRAEDTAVYYCVKGELLED YYFYGLDVWGQGTTVTVSS |
| | | | SEQ ID NO: 24564 | SEQ ID NO: 28570 |
| iPS:392984 | 21-225_30E11 | NA | GACATCCAGATGACCCAGTCTCCATCCTCCCT GTCTGCATCTGTAGGAGACAGAGTCACCATCA CTTGCCGGGCAAGTCAGAGCATTAGCAACTAT TTAAATTGGTATCAACAGCAAACAGGGAAAG CCCCTAAGTTCCTGATCTATGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTACCCCATTC ACTTTCGGCCCTGGGACCAAAGTGGATATCAA A | GAGGTGCAGCTGTTGGAGTCTGGGGGAGACATG GTCCAGCCTGGGGGGTCCCTGAGACTCTCCTGTG CAGCCTCCGGATTCACCTTTAGCATCTATGCCAT GAGCTGGGTCCGCCAGGCTCCAGGGAAGGGGCT GGAGTGGGTCTCAGTTATTAGTGGTAGTGGTGGT AGCTCATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTACGAAAGATCGGGTG AAAGCTCATGATGGTTTTGATATCTGGGGCCAAG GGACAATGGTCACCGTCTCTTCA |
| | | | SEQ ID NO: 24565 | SEQ ID NO: 28571 |
| | | AA | DIQMTQSPSSLSASVGDRVTITCRASQSISNYLN WYQQQTGKAPKFLIYAASSLQSGVPSRFSGSGS GTDFTLTISSLQPEDFATYYCQQSYSTPFTFGPGT KVDIK | EVQLLESGGDMVQPGGSLRLSCAASGFTFSIYAMS WVRQAPGKGLEWVSVISGSGGSSFYADSVKGRFTI SRDNSKNTLYLQMNSLRAEDTAVYYCTKDRVKAH DGFDIWGQGTMVTVSS |
| | | | SEQ ID NO: 24566 | SEQ ID NO: 28572 |

FIGURE 50

| iPS:392986 | 21-225_31B8 | NA | GACATCCAGATGATCCAGTCTCCATCCTCCCT GTCTGCATCTGTAGGAGACAGAGTCACCATCA CTTGCCGGGCAAGTCAGGACATTAGAAGTGAT TTAGGCTGGTATCAGCAGAAACCAGGGAAAG CCCCTAAGCGCCTGATCTATGGTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATATTATTTACCCTCCT ACTTTCGGCGGAGGGACCAAGGTGGAGATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCCGCTATTAGTGGTCGTGGTGGT AGCACATTCCACGCAGACTCCGTGAAGGGCCGG TTCACCATCTCCAGAGACAATTCCAAGAACACGC TGTATCTACAACTGAACAGCCTGAGAGCCGATGA CACGGCCGTATATTACTGTGTGAAAGGGGGAGCTA CTAGAGGACTACTACTTCTACGGTATGGACGTCT GGGGCCAAGGGACCACGGTCACCGTCTCCTCA |
| | | | SEQ ID NO: 24567 | SEQ ID NO: 28573 |
| | | AA | DIQMIQSPSSLSASVGDRVTITCRASQDIRSDLGW YQQKPGKAPKRLIYGASSLQSGVPSRFSGSGSGT EFTLTISSLQPEDFATYYCLQHIIYPPTFGGGTKV EIK | EVQLLESGGGLVQPGGSLRLSCAASGFTFSSYAMS WVRQAPGKGLEWVSAISGRGGSTFHADSVKGRFTI SRDNSKNTLYLQLNSLRADDTAVYYCVKGELLED YYFYGMDVWGQGTTVTVSS |
| | | | SEQ ID NO: 24568 | SEQ ID NO: 28574 |
| iPS:392988 | 21-225_25E6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCGGAAACCAGGGAAAG CCCCTAAGCGCCTGATCTATGCTGCATCCAGT TTGCAAAGTGGGGTCCCATCAAGGTTCCGCGG CAGTGGATCTGGGACAGAATTCACTCTCACAA TCAACAGTCTGCAGCCTGAAGATTTTGCAACC TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTCTGTATTACTGTGCGACAGAACTGGG GATGACGGGTGACTCCTGGGGCCAGGGAACCCT GGTCACCGTCTCCTCA |
| | | | SEQ ID NO: 24569 | SEQ ID NO: 28575 |

FIGURE 50

| | | | | |
|---|---|---|---|---|
| | | AA | DIQMTQSPSSLSASVGDRVTITCRASQGIRNDLG WYQRKPGKAPKRLIYAASSLQSGVPSRFRGSGS GTEFTLTINSLQPEDFATYYCLQHNSYPLTFGGG TKVEIK | QVQLVESGGGVVQPGRSLRLSCAASGFTFSDYGM HWVRQAPGKGLEWVAVIWYDENNKYYADSVKGR FTISRDNSKNTLYLQMNSLRAEDTALYYCATELGM TGDSWGQGTLVTVSS |
| | | | SEQ ID NO: 24570 | SEQ ID NO: 28576 |
| IPS:392990 | 21-225_25H10 | NA | GACATCCAGATGACCCAGTCTCCATCCTCCCT GTCTGCATCTGTAGGAGACAGAGTCACCATCA CTTGCCGGGCAAGTCGGGGCATTAGAAATGAT TTAGGCTGGTATCAGCAGAAACCAGGGAAAG CCCCTAAGCGCCTGATCTATGCTGCATTCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAAGT AATAAATACTATGCAGACTCCATGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACTGGG GATGACGGGTGACTACTGGGGCCAGGGAACCCT GGTCACCGTCTCCTCA |
| | | | SEQ ID NO: 24571 | SEQ ID NO: 28577 |
| | | AA | DIQMTQSPSSLSASVGDRVTITCRASRGIRNDLG WYQQKPGKAPKRLIYAAFSLQSGVPSRFSGSGS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYDESNKYYADSMKGR FTISRDNSKNTLYLQMNSLRAEDTAVYYCARELGM TGDYWGQGTLVTVSS |
| | | | SEQ ID NO: 24572 | SEQ ID NO: 28578 |

FIGURE 50

| iPS:392992 | 21-225_26C4 | NA | GACATCGTGATGACCCAGTCTCCAGACTCCCT GGCTGTGTCTCTGGGCGAGAGGGCCACCATCA ACTGCAAGTCCAGCCAGAGTGTTTTATACCGC TCCAACAATTACAACTACTTAGCTTGGTACCA GCACAAACCAGGACAGCCTCCTAAGCTGCTCA TTTACTGGGCATCTACCCGGGAATCCGGGGTC CCTGACCGCTTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAATTCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGGC TTGAGTGGATGGGATGGATGAACCCTAACAGTG GTAACACAGGCTATGCACAGAGATTCCAGGGCA GAGTCACCATGACCAGGAACACCTCCATAAACA CAGCCTACATGGAGCTGAGCAGCCTGAGATCTG AGGACACGGCCGTATATTACTGTGCGAGTAGCA GTGGCTGGTACTACTTTGACTACTGGGGCCAGGG AACCCTGGTCACCGTCTCCTCA |
| | | | SEQ ID NO: 24573 | SEQ ID NO: 28579 |
| | | AA | DIVMTQSPDSLAVSLGERATINCKSSQSVLYRSN NYNYLAWYQHKPGQPPKLLIYWASTRESGVPD RFSGSGSGTDFTLTISSLQAEDVAVYYCQQYYST PPTFGQGTKVEFK | QVQLVQSGAEVKKPGASVKVSCKASGYTFTNYDIN WVRQASGQGLEWMGWMNPNSGNTGYAQRFQGR VTMTRNTSINTAYMELSSLRSEDTAVYYCASSSGW YYFDYWGQGTLVTVSS |
| | | | SEQ ID NO: 24574 | SEQ ID NO: 28580 |
| iPS:392994 | 21-225_26G11 | NA | GATATTGTGATGACCCAGACTCCACTCTCTCT GTCCGTCACCCCTGGACAGCCGGCCTCCATCT CCTGCAAGTCTAGTCAGACCCTCCTGCATGGT GAAGGAAAGACCTATTTGTATTGGTACCTGCA GAAGCCAGGCCAGCCTCCACACCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TATAAAGCTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAACAGCTGGTCAGGGGGGTATGGACGTCTGGGG CCAAGGGACCACGGTCACCGTCTCCTCA |
| | | | SEQ ID NO: 24575 | SEQ ID NO: 28581 |

FIGURE 50

| | | | | |
|---|---|---|---|---|
| | | AA | DIVMTQTPLSLSVTPGQPASISCKSSQTLLHGEGK TYLYWYLQKPGQPPHLLIYEVSNRFSGVPDRFSG SGSGTDFTLKISRVEAEDVGIYYCMQSIKLPLTFG GGTKVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSNS WSGGMDVWGQGTTVTVSS |
| | | | SEQ ID NO: 24576 | SEQ ID NO: 28582 |
| iPS:392996 | 21-225_28B1 | NA | GACATCCAGATGACCCAGTCTCCATCTTCCGT GTCTGCATCTGTAGGAGACAGAGTCACCATCA CTTGTCGGGCGAGTCAGGCTATCAATGACTGG TTAGCCTGGTATCAGCAGAAACCAGGGAAAG CCCCTAAGCTCCTGATCTATGCTGCATCCAGTT TCCAAAGTGGGGTCCCATCAAGGTTCAGCGGC AGTGGATCTGGGACAGATTTCACTCTCACCAT CACCAGCCTGCAGCCTGAAGATTTTGCAACTT ACTATTGTCAACAGGCTAGCAGTTTCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGATTGTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGAGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAATTGGGGCGT ATAGCAGTGACTGGTCCTTACTTTGACTACTGGG GCCAGGGAACCCTGGTCACCGTCTCCTCA |
| | | | SEQ ID NO: 24577 | SEQ ID NO: 28583 |
| | | AA | DIQMTQSPSSVSASVGDRVTITCRASQAINDWLA WYQQKPGKAPKLLIYAASSFQSGVPSRFSGSGSG TDFTLTITSLQPEDFATYYCQQASSFPFTFGPGTK VDIK | EVQLLESGGGLVQPGGSLRLSCAASGFTFSSYAMS WVRQAPGKGLEWVSAISGSGGNTFYADSVKGRFTI SRDNSKNTLYLQMNSLRAEDTAVYYCAKLGRIAV TGPYFDYWGQGTLVTVSS |
| | | | SEQ ID NO: 24578 | SEQ ID NO: 28584 |

FIGURE 50

| iPS:392998 | 21-225_28A9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCTTCTAGTT TGCAAAATGGAGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATCGTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT ACACTGGGTCCGCCAGGCTCCAGGCAAGGGGCT GGAGTGGGTGGCAGTTATATGGTTTGATGGAAGT AATAAATACTATGCAGACTCCGTGAAGGGCCGA TTCACCATCTCCAGAGACAATTCCAAGAACACGC TGTTTCTGCAAATGAACAGCCTGAGAGTCGAGGA CACGGCTGTGTATTACTGTGCGAGAGAAATTGGC TGGTTAGATGACTACTGGGGCCAGGGAACCCTG GTCACCGTCTCCTCA |
| | | | SEQ ID NO: 24579 | SEQ ID NO: 28585 |
| | | AA | DIQMTQSPSSLSASVGDRVTITCRASQGIRNDLG WYQQKPGKAPKRLIYAASSLQNGVPSRFSGSGS GTEFTLTISSLQPEDFATYYCLQHNRYPFTFGPGT KVDIK | QVQLVESGGGVVQPGRSLRLSCAASGFTFSSYGIH WVRQAPGKGLEWVAVIWFDGSNKYYADSVKGRF TISRDNSKNTLFLQMNSLRVEDTAVYYCAREIGWL DDYWGQGTLVTVSS |
| | | | SEQ ID NO: 24580 | SEQ ID NO: 28586 |
| iPS:393000 | 21-225_29D7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAACAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAACTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAGT AATAAATACTATGGAGACTCCGTGAAGGGCCGA TTCACCATCTCCAGAGACAATTCCAAGAACACGC TGTATCTGCAAATGAACAGCCTGAGAGGCGAGG ACACGGCTCTGTATTACTGTGCGAGAGAACTGGG GTTCCTCTCTGACTACTGGGGCCAGGGAACCCTG GTCACCGTCTCCTCA |
| | | | SEQ ID NO: 24581 | SEQ ID NO: 28587 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSSYGMH WVRQAPGKGLEWVAVIWYDESNKYYGDSVKGRF TISRDNSKNTLYLQMNSLRGEDTALYYCARELGFL SDYWGQGTLVTVSS |
| | | | SEQ ID NO: 24582 | SEQ ID NO: 28588 |
| iPS:393002 | 21-225_30G1 | NA | GACATCCAGATGACCCAGTCTCCATCCTCCCT GTCTGCATCTGTAGGAGACAGAGTCACCATCA CTTGCCGGGCAAGTCAGAACATTTACAGCTAT TTAAAATTGGTATCAGCAGAAACCAGGGAAAG ACCCTAAGCTCCTGATCTATGCTGCATCCAGTT TGCATAGTGGGGTCCCGTCACGGTTCAGTGGC AGTGGTTCTGGGACAGATTTCACTCTCACCAT CAGCAGTCTGCAACCTGAAGATTTTGCAACTT ACTACTGTCAACAGAGTTACAGTACTCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTTACTATGGCAT GCACTGGGTCCGCCAGGCTCCAGGCAAGGGGCT GGAGTGGGTGGCAGTTATATGGCATGATGGAAG TAATAAATACTATGTAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAAAATAG CAGTTCGTACTACTTTGACTACTGGGGCCAGGGA ACCCTGGTCACCGTCTCCTCA |
| | | | SEQ ID NO: 24583 | SEQ ID NO: 28589 |
| | | AA | DIQMTQSPSSLSASVGDRVTITCRASQNIYSYLN WYQQKPGKDPKLLIYAASSLHSGVPSRFSGSGS GTDFTLTISSLQPEDFATYYCQQSYSTPLTFGGGT KVEIK | QVQLVESGGGVVQPGRSLRLSCAASGFTFSYYGM HWVRQAPGKGLEWVAVIWHDGSNKYYVDSVKGR FTISRDNSKNTLYLQMNSLRAEDTAVYYCARENSS SYYFDYWGQGTLVTVSS |
| | | | SEQ ID NO: 24584 | SEQ ID NO: 28590 |

FIGURE 50

| iPS:393004 | 21-225_30G11 | NA | GACATCCAGATGACCCAGTCTCCATCCTCCCT GTCTGCATCTGTAGGAGACAGAGTCACCATCA CTTGCCGGGCAAGTCAGGACATTAGAAGTGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCGGCAGCCTGCAGCCTGAAGATTTTGCAACT TATTTCTGTCTACAGCATACTATTTACCCTCCT ACTTTCGGCGGAGGGACCAAGGTGGAGATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCCGCTATTAGTGGTCGTGGTGGT AGCACATTCAACGCAGACTCCGTGAAGGGCCGG TTCACCATCTCCAGAGACAATTCCAAGACCACGC TGTATCTGCAAATGAACAGCCTGAGAGCCGAGG ACACGGCCGTATATTACTGTGTGAAAGGGGAGCT ACTAGAGGACTACTACTTCTACGGTATGGACGTC TGGGGCCAAGGGACCACGGTCACCGTCTCCTCA |
| | | | SEQ ID NO: 24585 | SEQ ID NO: 28591 |
| | | AA | DIQMTQSPSSLSASVGDRVTITCRASQDIRSDLG WYQQKPGKAPKRLIYAASSLQSGVPSRFSGSGS GTEFTLTIGSLQPEDFATYFCLQHTIYPPTFGGGT KVEIK | EVQLLESGGGLVQPGGSLRLSCAASGFTFSSYAMS WVRQAPGKGLEWVSAISGRGGSTFNADSVKGRFTI SRDNSKTTLYLQMNSLRAEDTAVYYCVKGELLED YYFYGMDVWGQGTTVTVSS |
| | | | SEQ ID NO: 24586 | SEQ ID NO: 28592 |
| iPS:393006 | 21-225_31G9 | NA | GACATCCAGATGACCCAGTCTCCATCCTCCCT GTCTGCATCTATAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATCCTGCATCCAGTT TGCAAAGTGGGGTCCCATCAAGGTTCAGCGGC AGTGGATCTGGGACAGAGTTCACTCTCACAAT CAGCAGCCTGCAGCCTGAAGATTTTGCAACTT ATTACTGTCTACAGGATAATAGTTACCCTTTCA CTTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCAATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATCGAGGC AGCAGCTGGGGCCAGGGAACCCTGGTCACCGTC TCCTCA |
| | | | SEQ ID NO: 24587 | SEQ ID NO: 28593 |

FIGURE 50

| | | | | |
|---|---|---|---|---|
| | | AA | DIQMTQSPSSLSASIGDRVTITCRASQGIRNDLG WYQQKPGKAPKRLIYPASSLQSGVPSRFSGSGSG TEFTLTISSLQPEDFATYYCLQDNSYPFTFGPGTK VDIK | EVQLVESGGGLVKPGGSLRLSCAASGFTFSSYSMN WVRQAPGKGLEWVSSISSSSSYIYYADSVKGRFTIS RDNAKNSLYLQMNSLRAEDTAVYYCARDRGSSW GQGTLVTVSS |
| | | | SEQ ID NO: 24588 | SEQ ID NO: 28594 |
| iPS:393010 | 21-225_25E11 | NA | GACATCCAGATGACCCAGTCTCCATCTTCCGT GTCTGCATCTGTAGGAGACAGAGTCACCATCA CTTGTCGGGCGAGTCAGGGTATTAGCAACTGG TTAGCCTGGTATCAGCAGAAACCAGGGAAAG CCCCTAAGCTCCTGATCTATACTGCATCCAGTT TGCAAGGTGGGGTCCCATCAAGGTTCAGCGGC AGTGGATCTGGGACAGATTTCACTATCAGCAT CAGCAGCCTGCAGCCTGAAGATTTTGCAACTT ACTATTGTCAACAGGCTAACAGTTTCCCAATC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTGGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAACGTGGATA TAGTGGCTACGAGGACCTCCTCTACTTTGACTGC TGGGGCCAGGGAACCCTGGTCACCGTCTCCTCA |
| | | | SEQ ID NO: 24589 | SEQ ID NO: 28595 |
| | | AA | DIQMTQSPSSVSASVGDRVTITCRASQGISNWLA WYQQKPGKAPKLLIYTASSLQGGVPSRFSGSGS GTDFTISISSLQPEDFATYYCQQANSFPITFGPGT KVDIK | EVQLLESGGGLVQPGGSLRLSCAASGFTFSSYAMS WVRQAPGKGLEWVSVISGGGGSTYYADSVKGRFT ISRDNSKNTLYLQMNSLRAEDTAVYYCAKRGYSG YEDLLYFDCWGQGTLVTVSS |
| | | | SEQ ID NO: 24590 | SEQ ID NO: 28596 |

FIGURE 50

| iPS:393012 | 21-225_26G7 | NA | GATATCTTGATGACCCAGACTCCACTCTCTCTG TCCGTCACCCCTGGACAGCCGGCCTCCATCTC CTGCAAGTCTAGTCAGAGCCTCCTGCATAGTG AGGGAAAGACCTATTTGTATTGGTACCTGCAG AAGCCAGGCCAGCCTCCACAGTTCCTGATCTA TGAAGTTTCCCACCGGCTCTCTGGAGTGCCAG ATAGGTTCAGTGGCAGCGGGTCAGGGACAGA TTTCACACTGAAAATCAGCCGGGTTGAGGCTG AGGATGTTGGGGTTTATTACTGCATGCAAAGT ATACAGCTTCCGCTCACTTTCGGCGGAGGGAC CAAGGTGGAGATCAAA | CAGGTGCAGCTGGTGGAGTCTGGGGGAGGCGTG GTCCAGCCTGGGAGGTCCCTGAGACTGTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATATTATGCAGACTCCGTGAAGGGCCGAT TCACCATCTCCAGAGACAATTCCAATAACACGCT GTATCTGCAAATGAACAGCCTGAGAGCCGAGGA CACGGCTGTGTATTACTGTGCGAGAAGGTATAGC AGCAGCTGGTCAGGGGGTATGGACGTCTGGGGC CAAGGGACCACGGTCACCGTCTCCTCA |
|  |  |  | SEQ ID NO: 24591 | SEQ ID NO: 28597 |
|  |  | AA | DILMTQTPLSLSVTPGQPASISCKSSQSLLHSEGK TYLYWYLQKPGQPPQFLIYEVSHRLSGVPDRFSG SGSGTDFTLKISRVEAEDVGVYYCMQSIQLPLTF GGGTKVEIK | QVQLVESGGGVVQPGRSLRLSCAASGFTFSSYGMH WVRQAPGKGLEWVAVIWYDGSNKYYADSVKGRF TISRDNSNNTLYLQMNSLRAEDTAVYYCARRYSSS WSGGMDVWGQGTTVTVSS |
|  |  |  | SEQ ID NO: 24592 | SEQ ID NO: 28598 |
| iPS:393014 | 21-225_26D12 | NA | GACATCCAGATGACCCAGTCTCCCTCTTCCGT GTCTGCATCTGTAGGAGACAGAGTCACCATCA CTTGTCGGGCGAGTCAGGGTATTAGTAGTTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGCC AGTGGATCTGGGACAGATTTCACTCTCACCAT CAGCAGCCTGCAGCCTGAAGATTTTGCAACTT ACTGTTGTCAACAGTCTGACAGTTTCCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGCAGTTATGGCAT GCACTGGGTCCGCCAGGCTCCAGGCAAGGGGCT GGAGTGGGTGGCAGTTATATGGTATGATGGAAGT AATGAATACTATGCAGACTCCGTGAAGGGCCGA TTCACCATCTCCAGAGACAATTCCAAGAACACGC TATATCTACAAATGAACAGCCTGAGAGCCGCGG ACACGGCTGTGTATTACTGTGCGAGAGATTCCTC CCCCTACGGTATGGACGTCTGGGGCCAAGGGAC CACGGTCACCGTCTCCTCA |
|  |  |  | SEQ ID NO: 24593 | SEQ ID NO: 28599 |

FIGURE 50

| | | | | |
|---|---|---|---|---|
| | | AA | DIQMTQSPSSVSASVGDRVTITCRASQGISSWLA WYQQKPGKAPKLLIYGASSLQSGVPSRFSASGS GTDFTLTISSLQPEDFATYCCQQSDSFPRTFGQGT KVEIK | QVQLVESGGGVVQPGRSLRLSCAASGFTFSSYGMH WVRQAPGKGLEWVAVIWYDGSNEYYADSVKGRF TISRDNSKNTLYLQMNSLRAADTAVYYCARDSSPY GMDVWGQGTTVTVSS |
| | | | SEQ ID NO: 24594 | SEQ ID NO: 28600 |
| IPS:393016 | 21-225_28F11 | NA | GACATCCAGATGACCCAGTCTCCATCTTCCGT GTCTGCATCTGTAGGAGACAGAGTCACCATCA CTTGTCGGGCGAGTCAGGGTATTAGCAACTGG TTAGCCTGGTATCAGCAGAAACCAGGGAAAG CCCCTAAGCTCCTGATCTCTGCTGCATCCAATT TGCAAAGTGGGGTCCCATCAAGGTTCAGAGGC AGTGGATCTGGGACAGATTTCACTCTCACCAT CACCAGCCTGCAGCCTGAAGATTTTGCAACTT ACTGTTGTCAACAGGCTAACAGTCTCCCATTC ACTTTCGGCCCTGGGACCAAAGTGGATATCAA A | GAGGTGCAGCTGTTGGAGTCTGGGGGAGGCTTG GTACAGCCGGGGGGGTCCCTGAGACTCTCCTGTG CAGCCTCTGGATTCATCTTTAGCAGCTATGCCAT GAGCTGGGTCCGCCAGGCTCCAGGGAAGGGGCT GGAGTGGGTCTCAGTTACTAGTGGTAGTGGTGGT ACCACATTCTACGCAGACTCCGTGAAGGGCCGGT TCACCATCTCCAGAGACAATTCCAAGAACACGCT TTATCTGCAAATGAACAGCCTGAGAGCCGAGGA CACGGCCGTTTATTACTGTGCGAAACGGACCCAG TTTGATGATTTTGATATCTGGGGCCAAGGGACAA TGGTCACCGTCTCTTCA |
| | | | SEQ ID NO: 24595 | SEQ ID NO: 28601 |
| | | AA | DIQMTQSPSSVSASVGDRVTITCRASQGISNWLA WYQQKPGKAPKLLISAASNLQSGVPSRFRGSGS GTDFTLTITSLQPEDFATYCCQQANSLPFTFGPGT KVDIK | EVQLLESGGGLVQPGGSLRLSCAASGFIFSSYAMS WVRQAPGKGLEWVSVTSGSGGTTFYADSVKGRFT ISRDNSKNTLYLQMNSLRAEDTAVYYCAKRTQFDD FDIWGQGTMVTVSS |
| | | | SEQ ID NO: 24596 | SEQ ID NO: 28602 |

FIGURE 50

| | | | | |
|---|---|---|---|---|
| iPS:393018 | 21-225_29B8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTAGATCTGGGACAGAATTCACTCTCACAA TCAGCAGTCTGCAGCCTGAAGATTTTGCAACC TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACTGGG GATGACGGGTGACTCCTGGGGCCAGGGAACCCT GGTCACCGTCTCCTCA |
| | | | SEQ ID NO: 24597 | SEQ ID NO: 28603 |
| | | AA | DIQMTQSPSSLSASVGDRVTITCRASQGIRNDLG WYQQKPGKAPKRLIYAASSLQSGVPSRFSGSRS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYDENNKYYADSVKGR FTISRDNSKNTLYLQMNSLRAEDTAVYYCARELGM TGDSWGQGTLVTVSS |
| | | | SEQ ID NO: 24598 | SEQ ID NO: 28604 |
| iPS:393020 | 21-225_30E2 | NA | GACATCCAGATGACCCAGTCTCCACATTCCCT GTCTGCATCTGTAGGAGACAGAGTCACCATCA CTTGCCAGGCGAGTCAGTACATTAGCAACTAT TTAAATTGGTATCAGCAGAAATCAGGAAAAGC CCCTAAGCTCCTGATCTACGATGGATCCAGTT TGGAAACAGGGGTCCCATCAAGGTTCAGTGGA AGTGGATCTGGGACAGATTTTACTTTCACCAT CAGCAGCCTGCAGCCTGAAGATCTTGCAACAT ATTACTGTCAACAGTATGATAATCTCCCGATC ACCTTCGGCCAAGGGACACGACTGGAGATTAA A | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGGTGGAAGT AATAAATTCTATGCAGTCTCCGTGAAGGGCCGAT TCAACATCTCCAGAGACAATTCCAAGAACACGCT GTATCTGCAAATGAACAGCCTGAGAGCTGAGGA CACGGCTGTGTATTACTGTGTGAGAAGGGGGTAT AGCAGTGGAGGCTACGGTATGGACGTCTGGGGC CAAGGGACCACGGTCACCGTCTCCTCA |
| | | | SEQ ID NO: 24599 | SEQ ID NO: 28605 |

FIGURE 50

| | | | | |
|---|---|---|---|---|
| | | AA | DIQMTQSPHSLSASVGDRVTITCQASQYISNYLN WYQQKSGKAPKLLIYDGSSLETGVPSRFSGSGSG TDFTFTISSLQPEDLATYYCQQYDNLPITFGQGTR LEIK | QVQLVESGGGVVQPGRSLRLSCAASGFTFSSYGMH WVRQAPGKGLEWVAVISYGGSNKFYAVSVKGRFN ISRDNSKNTLYLQMNSLRAEDTAVYYCVRRGYSSG GYGMDVWGQGTTVTVSS |
| | | | SEQ ID NO: 24600 | SEQ ID NO: 28606 |
| IPS:393022 | 21-225_30H11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGGTCTATCCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGGATAATAGTCATCCATTC ACTTTCGGCCCTGGGACCAAAGTGGATATCAC A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATAGGGGG AGCCTCTGGGGCCAGGGAACCCTGGTCACCGTCT CCTCA |
| | | | SEQ ID NO: 24601 | SEQ ID NO: 28607 |
| | | AA | DIQMTQSPSSLSASVGDRVTITCRASQGIRNDLG WYQQKPGKAPKRLVYPASSLQSGVPSRFSGSGS GTEFTLTISSLQPEDFATYYCLQDNSHPFTFGPGT KVDIT | EVQLVESGGGLVKPGGSLRLSCAASGFTFSSYSMN WVRQAPGKGLEWVSSISGSSSYIYYADSVKGRFTIS RDNAKNSLYLQMNSLRAEDTAVYYCARDRGSLW GQGTLVTVSS |
| | | | SEQ ID NO: 24602 | SEQ ID NO: 28608 |

FIGURE 50

| iPS:393024 | 21-225_31H9 | NA | GACATCCAGATGACCCAGTCTCCATCTTCCGT GTCTGCATCTGTAGGAGACAGAGTCACCATCA CTTGTCGGGCGAGTCAGGGTATTACCAGCTGG TTAACTTGGTATCAGCAGAGACCAGGGAAAGC CCCTAAACTCCTGATCTATGATACATCCAGTTT GCAAAGTGGGGTCCCATCAAGGTTCAGCGGCA GTGGATCTGGGACAGATTTCATTTTCACCATC AGCAGCCTGCAGCCTGAAGATTTTGCCACTTA TTATTGTCAACAGGGTAACAGTTTCCCATTCA CTTTCGGCCAAGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTGTGCCAT GAACTGGGTCCGCCAGGCTCCAGGGAAGGGGCT GGAGTGGGTCTCAGCTATTAGTGGTAGTGGTGGT AGCTCATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAACGGACTCCC TATGATGTCTTTGATATCTGGGGCCAAGGGACAA TGGTCACCGTCTCTTCA |
|---|---|---|---|---|
| | | | SEQ ID NO: 24603 | SEQ ID NO: 28609 |
| | | AA | DIQMTQSPSSVSASVGDRVTITCRASQGITSWLT WYQQRPGKAPKLLIYDTSSLQSGVPSRFSGSGSG TDFIFTISSLQPEDFATYYCQQGNSFPFTFGQGTK VDIK | EVQLLESGGGLVQPGGSLRLSCAASGFTFSSCAMN WVRQAPGKGLEWVSAISGSGGSSFYADSVKGRFTI SRDNSKNTLYLQMNSLRAEDTAVYYCAKRTPYDV FDIWGQGTMVTVSS |
| | | | SEQ ID NO: 24604 | SEQ ID NO: 28610 |
| iPS:393026 | 21-225_32B6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATATTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGTGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGACAAGGGGCT GGAGTGGGTGGCAGTTATATGGTATGATGAAAAT ACTAAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAGTGGGGG GACTACTGGGGCCAGGGAACCCTGGTCACCGTCT CCTCA |
| | | | SEQ ID NO: 24605 | SEQ ID NO: 28611 |

FIGURE 50

| | | | | |
|---|---|---|---|---|
| | | AA | DIQMTQSPSSLSASVGDRVTITCRASQGIRNDLGWYQQKPGKAPKRLIYIASSLQSGVPSRFSGSGSGTEFTLTISSLQPEDFATYYCLQHNSYPWTFGQGTKVEIK | QVQLVESGGGVVQPGRSLRLSCAASGFTFSDYGMHWVRQAPDKGLEWVAVIWYDENTKYYADSVKGRFTISRDNSKNTLYLQMNSLRAEDTAVYYCAREWGDYWGQGTLVTVSS |
| | | | SEQ ID NO: 24606 | SEQ ID NO: 28612 |
| iPS:393028 | 21-225_25D7 | NA | GACATCCAGATGACCCAGTCTCCATCTTCCGTGTCTGCATCTGTAGGAGACAGAGTCACCTTCACTTGTCGGGCGAGTCAGGATATTTTCGACTGGTTAGCCTGGTATCAGCAGAAACCCGGGACAGCCCCTAAGCTCCTGATCTATGCTGCATCCAGTTTGCAAAGTGGGGTCCCATCAAGGTTCAGCGGCAGTGGATCTGGGACAAATTTCACTCTCACCGTCAGCGGCCTGCAGCCTGAAGATTTTGCTACTTACTATTGTCAACAGGCTTACAGTTTCCCGTGGACGTTCGGCCAAGGGACCAAAGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGACTCCAGGGCAGGGGCTGGAGTGGGTCTCAGCTATTAGTGGTAGAGGTGGTACCACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGACGGGTACGGTGGTAACTCCTTCTTTGACTACTGGGGCCAGGGAACCCTGGTCACCGTCTCCTCA |
| | | | SEQ ID NO: 24607 | SEQ ID NO: 28613 |
| | | AA | DIQMTQSPSSVSASVGDRVTFTCRASQDIFDWLAWYQQKPGTAPKLLIYAASSLQSGVPSRFSGSGSGTNFTLTVSGLQPEDFATYYCQQAYSFPWTFGQGTKVEIK | EVQLLESGGGLVQPGGSLRLSCAASGFTFSSYAMSWVRQTPGQGLEWVSAISGRGGTTFYADSVKGRFTISRDNSKNTLYLQMNSLRAEDTAVYYCAKDGYGGNSFFDYWGQGTLVTVSS |
| | | | SEQ ID NO: 24608 | SEQ ID NO: 28614 |

FIGURE 50

| iPS:393030 | 21-225_25H11 | NA | GACATCCAGATGACCCAGTCTCCATCCTCCCT GTCTGCGTCTGTAGGAGACAGAGTCACCATCA CTTGCCGGGCAAGTCAGGGCATTAGAACTGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGTAGTCTGCAGCCTGAAGATTTTGCAACC TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAAAT AATGAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACTGGG GATGACGGGTGACTCCTGGGGCCAGGGAACCCT GGTCACCGTCTCCTCA |
| | | | SEQ ID NO: 24609 | SEQ ID NO: 28615 |
| | | AA | DIQMTQSPSSLSASVGDRVTITCRASQGIRTDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYDENNEYYADSVKGR FTISRDNSKNTLYLQMNSLRAEDTAVYYCARELGM TGDSWGQGTLVTVSS |
| | | | SEQ ID NO: 24610 | SEQ ID NO: 28616 |
| iPS:393032 | 21-225_26F8 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAAGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCTTCCGTGGACGTTCGGCCAGGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA CGATTTTTGGAGTGGTTGTATGGACGTCTGGGGC CAAGGGACCACGGTCACCGTCTCCTCA |
| | | | SEQ ID NO: 24611 | SEQ ID NO: 28617 |

FIGURE 50

| | | | | |
|---|---|---|---|---|
| | | AA | DIVMTQTPLSLSVTPGQPASISCKSSQSLLHGDG KTYLYWYLQKPGQPPQLLIYEVSNRFSGVPDRFS GSGSGTDFTLKISRVEAEDVGVYYCMQSIQLPW TFGQGTKVEIK | QVQLVESGGGVVQPGRSLRLSCAASGFTFSGYGM HWVRQAPGKGLEWVAIIWYDGSNKYYADSVKGR FTISRDNSKNTLYLQMNSLRAEDTAVYYCARERYD FWSGCMDVWGQGTTVTVSS |
| | | | SEQ ID NO: 24612 | SEQ ID NO: 28618 |
| IPS:393034 | 21-225_27F2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGTTGCATCCAGTT TGCAAAGTGGGGTCCCATCAAGGTTCAGCGGC AGTGGATCTGGGACAGAATTCACTCTCACAAT CAGCAGTCTGCAGCCTGAAGATTTTGCAACCT ATTACTGTCTACAGCATAATAGTTACCCGCTC ACTTTCGGCGGAGGGACCAAGGTGGAGATCA AA | CAGGAGCAGCTGGTGGAGTCTGGGGGAGGCGTG GTCCAGCCTGGGAGGTCCCTGAGACTCTCCTGTG CAGCGTCTGGATTCATCTTCAGTGACTATGGCAT GCACTGGGTCCGCCAGGCTCCAGGCAAGGGGCT GGAGTGGGTGGCAGTTATATGGTATGATGAAAAT AATAAATACTATGTAGACTCCGTGAGGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG ATGACGGGTGACTCCTGGGGCCAGGGAACCCTG GTCACCGTCTCCTCA |
| | | | SEQ ID NO: 24613 | SEQ ID NO: 28619 |
| | | AA | DIQMTQSPSSLSASVGDRVTITCRASQGIRNDLG WYQQKPGKAPKRLIYVASSLQSGVPSRFSGSGS GTEFTLTISSLQPEDFATYYCLQHNSYPLTFGGG TKVEIK | QEQLVESGGGVVQPGRSLRLSCAASGFIFSDYGMH WVRQAPGKGLEWVAVIWYDENNKYYVDSVRGRF TISRDNSKNTLYLQMNSLRAEDTAVYYCARELGMT GDSWGQGTLVTVSS |
| | | | SEQ ID NO: 24614 | SEQ ID NO: 28620 |

FIGURE 50

| iPS:393036 | 21-225_28G3 | NA | GAAATTGTGATGACCCAGACTCCACTCTCTCT GTCCGTCACCCCTGGACAGCCGGCCTCCATCT CCTGCAAGTCTAGTCAGAGCCTCCTACATGGT GATGGAAAGACCTATTTGTATTGGTACCTGCA GAAGCCAGGCCAGCCTCCACAGCTCCTGATCT ATGAAGTTTCCAACCGGTTCTCTGGAGTGCCA GAAAGGTTCAGTGGTAGCGGGTCAGGGACAG ATTTCACACTGAAAATCAGCCGGGTGGAGGCT GAGGATGTTGGGGTTTATTACTGTATGCAAAG TATACAGATTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCATCTTCAGTACCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGATA CGATTTTTGGAGTGGTTATTTTGACTACTGGGGC CAGGGAACCCTGGTCACCGTCTCCTCA |
| | | | SEQ ID NO: 24615 | SEQ ID NO: 28621 |
| | | AA | EIVMTQTPLSLSVTPGQPASISCKSSQSLLHGDGK TYLYWYLQKPGQPPQLLIYEVSNRFSGVPERFSG SGSGTDFTLKISRVEAEDVGVYYCMQSIQIPWTF GQGTKVEIK | QVQLVESGGGVVQPGRSLRLSCAASGFIFSTYGMH WVRQAPGKGLEWVAVIWYDGSNKYYADSVKGRF TISRDNSKNTLYLQMNSLRAEDTAVYYCARDRYDF WSGYFDYWGQGTLVTVSS |
| | | | SEQ ID NO: 24616 | SEQ ID NO: 28622 |
| iPS:393038 | 21-225_29D8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGGGACTATGGCAT ACACTGGGTCCGCCAGGCTCCAGGCAAGGGGCT GGAGTGGGTGGCAGTTATATGGTTTGATGGAACT AATAAATACTATGCAGACTCCGTGAAGGGCCGA TTCACCATCTCCAGAGACAATTCCAAGAACACGC TGTATCTGCAAATGAACAGCCTGAGAGCCGAAG ACACGGCTGTGTATTACTGTGGGAGAGAAATTGG CTGGTTAGATGACTACTGGGGCCAGGGAACCCTG GTCACCGTCTCCTCA |
| | | | SEQ ID NO: 24617 | SEQ ID NO: 28623 |

FIGURE 50

| | | | DIQMTQSPSSLSASVGDRVTITCRASQGIRNDLG WYQQKPGKAPKRLIYTASSLQSGVPSRFSGSGSG TEFTLTISSLQPEDFATYYCLQHNSYPFTFGPGTK VDIK | QVQLVESGGGVVQPGRSLRLSCAASGFTFRDYGIH WVRQAPGKGLEWVAVIWFDGTNKYYADSVKGRF TISRDNSKNTLYLQMNSLRAEDTAVYYCGREIGWL DDYWGQGTLVTVSS |
|---|---|---|---|---|
| | | AA | SEQ ID NO: 24618 | SEQ ID NO: 28624 |
| iPS:393040 | 21-225_30E3 | NA | GACATCCAGATGACCCAGTCTCCATCCTCCCT GTCTGCATCTGTAGGAGACAGAGTCACCATCA CTTGCCGGGCAAGTCAGGACATTAGAGATGAT TTAGGCTGGTATCAGCAGAAACCAGGGAAAG CCCCTAGGCGCCTGATCTATGCTGCATTCAGC TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATACTAGTTACCCTCCT ACTTTCGGCGGAGGGACCAAGGTGGAGATCA AA | GAGGTGCAGGTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCCGCTATTAGTGGTCGTGGTGGT AGCACATTCTACGCAGACTCCGAGAAGGGCCGG TTCACCATCTCCAGAGACAATTCCAAGAACACGC TGTATCTGCAAATGAACAGCCTGAGAGCCGAGG ACACGGCCCTATATTACTGTGCGAAAGGGGAGCT ATTAGAGGACTACTACTACTACGGAATGGACGTC TGGGGCCAAGGGACCACGGTCACCGTCTCCTCA |
| | | | SEQ ID NO: 24619 | SEQ ID NO: 28625 |
| | | AA | DIQMTQSPSSLSASVGDRVTITCRASQDIRDDLG WYQQKPGKAPRRLIYAAFSLQSGVPSRFSGSGS GTEFTLTISSLQPEDFATYYCLQHTSYPPTFGGGT KVEIK | EVQVLESGGGLVQPGGSLRLSCAASGFTFSSYAMN WVRQAPGKGLEWVSAISGRGGSTFYADSEKGRFTI SRDNSKNTLYLQMNSLRAEDTALYYCAKGELLED YYYYGMDVWGQGTTVTVSS |
| | | | SEQ ID NO: 24620 | SEQ ID NO: 28626 |

1566

FIGURE 50

| iPS:393042 | 21-225_31F1 | NA | GACATCCAGATGACCCAGTCTCCATCCTCCCTGTCTGCATCTGTAGGAGACAGAGTCACCATCACTTGCCGGGCAAGTCAGAGGATTAGCAGCTATTTAAATTGGTATCAGCAGAAACCAGGGAAAGCCCCTAAGCTCCTGATCTTTGCTGCATCCAGTTCGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACATTACCCCGCTCACTTTCGGCGGAGGGACCACGGTGGAGATCGA | CAGGTGCAGCTGGTGCAGTCTGGGGGCTGAGGTGAAGAAGCCTGGGGCCTCAGTGAAGGTCTCCTGCAAGGCTTCTGGATACACCTTCACCGACTACTATATGCACTGGGTGCGACAGGCCCCTGGACAGGGGCTTGAGTGGATGGGATGGATCAACCCTAACAGTGGTGGCACAAACTATGCACAGAAGTTTCAGGGCAGGGTCACCATGACCAGGGACACGTCCATTATGACAGCCTATATGGAGCTGAGCAGGCTGAGATCTGACGACACGGCCGTGTATTACTGTGCGAGAGATAGTAGCAATTTCAGCAACTGGTACGATTACTACGGTATGGACGTCTGGGGCCAAGGGACCACGGTCACCGTCTCCTCA |
| | | | SEQ ID NO: 24621 | SEQ ID NO: 28627 |
| | | AA | DIQMTQSPSSLSASVGDRVTITCRASQRISSYLNWYQQKPGKAPKLLIFAASSSQSGVPSRFSGSGSGTDFTLTISSLQPEDFATYYCQQSYITPLTFGGGTTVEIR | QVQLVQSGAEVKKPGASVKVSCKASGYTFTDYYMHWVRQAPGQGLEWMGWINPNSGGTNYAQKFQGRVTMTRDTSIMTAYMELSRLRSDDTAVYYCARDSSNFSNWYDYYGMDVWGQGTTVTVSS |
| | | | SEQ ID NO: 24622 | SEQ ID NO: 28628 |
| iPS:393044 | 21-225_25B8 | NA | GAAATAGTGATGACGCAGTCTCCAGCCACCCTGTCTGTGTCTCCAGGGGAAAGAGCCACCCTCTCCTGCAGGGCCAGTCAGAGTGTTAGAAGTAATTTAGCCTGGTACCAGCAGAAACCTGGCCAGGCTCCCAGGCTCCTCATCTATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCACTTTATTACTGTCAGCAGTATAATAATTGGCCTCCGTGGCCCGTTCGGCCAAGGGACCAAGGTGGAAATCAAA | CAGGGTTCAGTTGGTGCAGTCTGGAGCTGAGGTGAAGAAGCCTGGGGCCTCAGTGAAGGTCTCCTGCAAGGCTTCTGGTTACACCTTCACCAGCTATGGTATCAGCTGGGTGCGACAGGCCCCTGGACAAGGGCTTGAGTGGATGGGATGGATCAGCGCTTACAATGGTAACACAACCTATGCACAGAAGCTCCGGGGCAGAGTCACCATGACCACAGACACATCCACGAGCACAGCCTACATGGATCTGAGGAGCCTGAGATCTGACGACACGGCCGTGTATTACTGTGCGAGAACCGCTGCTGGGTATAGCAGCAGCTGGTTTGACTACTGGGGCCAGGGAACCCTGGTCACCGTCTCCTCA |
| | | | SEQ ID NO: 24623 | SEQ ID NO: 28629 |

FIGURE 50

| | | | | |
|---|---|---|---|---|
| | | AA | EIVMTQSPATLSVSPGERATLSCRASQSVRSNLA WYQQKPGQAPRLLIYGASTRATGIPARFSGSGSG TEFTLTISSLQSEDFALYYCQQYNNWPPWPFGQ GTKVEIK | QVQLVQSGAEVKKPGASVKVSCKASGYTFTSYGIS WVRQAPGQGLEWMGWISAYNGNTTYAQKLRGRV TMTTDTSTSTAYMDLRSLRSDDTAVYYCARTAAG YSSSWFDYWGQGTLVTVSS |
| | | | SEQ ID NO: 24624 | SEQ ID NO: 28630 |
| iPS:393046 | 21-225_25A12 | NA | GACATCCAGATGACCCAGTCTCCATCCTCCCT GTCTGCATCTGTAGGAGACAGAGTCACCGTCA CTTGCCGGGCAAGTCAGGCCATTAGAGATGAT TTAGGCTGGTATCAGCAGAGACCAGGGAAAG CCCCTAAGCGCCTGATCTATGCTGCATCCAGT TTGCAAAGTGGGGTCCCATCAAGGTTCAGCGG CAGTGGATCTGGGACAGAATTTATTCTCACAA TCAGCAGCCTGCAGCCTGAAGATTTTGCAACT TATTACTGTCTACAGCATTATAATTACCCTCGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTGTGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGAGTA TAGCAGTGGCTGGTACGACTACGGTATGGACGTC TGGGGCCAAGGGACCATGGTCACCGTCTCCTCA |
| | | | SEQ ID NO: 24625 | SEQ ID NO: 28631 |
| | | AA | DIQMTQSPSSLSASVGDRVTVTCRASQAIRDDLG WYQQRPGKAPKRLIYAASSLQSGVPSRFSGSGS GTEFILTISSLQPEDFATYYCLQHYNYPRSFGQGT KLEIK | QVQLVESGGGVVQPGRSLRLSCAASGFTFSNCVMH WVRQAPGKGLEWVAVIWYDGSNKYYADSVKGRF TISRDNSKNTLYLQMNSLRAEDTAVYYCAREEYSS GWYDYGMDVWGQGTMVTVSS |
| | | | SEQ ID NO: 24626 | SEQ ID NO: 28632 |

FIGURE 50

| | | | | |
|---|---|---|---|---|
| iPS:393048 | 21-225_27C3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGTCTGCAGCCTGAAGATTTTGCAACC TATTACTGTCTACAGCATAATCGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAAAT AATAAATCCTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG ATGACGGGTGACTCCTGGGGCCAGGGAACCCTG GTCACCGTCTCCTCA |
| | | | SEQ ID NO: 24627 | SEQ ID NO: 28633 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNRYPLTFGGG TKVEIK | QVQLVESGGGVVQPGRSLRLSCAASGFTFSDYGM HWVRQAPGKGLEWVAVIWYDENNKSYADSVKGR FTISRDNSKNTLYLQMNSLRAEDTAVYYCARELGM TGDSWGQGTLVTVSS |
| | | | SEQ ID NO: 24628 | SEQ ID NO: 28634 |
| iPS:393050 | 21-225_28C5 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCAAC TTAGCCTGGTACCATCAGAAACCTGGCCAGGC TCCCAGGCTCCTCATCTATGGTGCATCCACCA GGGCCACTGGTATCCCAGCCAGGTTCAGTGGC AGTGGGTCTGGGACAGAGTTCACTCTCACCAT CAGCAGCCTGCAGTCTGAAGATTTTGCACTTT ATTACTGTCAGCAGTATAATAATTGGCCTCCG TGGCCGTTCGGCCAAGGGACCAAGGTGGAAA TCAAA | CAGGTTCAGTTGGTGCAGTCTGGAGCTGAGGTGA AGAAGCCTGGGGCCTCAGTGAAGGTCTCCTGCA AGGCTTCTGATTACACCTTCACCAGCTATGGTAT CAGCTGGGTGCGACAGGCCCCTGGACAAGGGCT TGAGTGGATGGGATGGATCAGCGCTTACAATGGT AACACAACCTATGCACAGAAGCTCCGGGGCAGA GTCACCATGACCACAGACACATCCACGAGCACA GCCTACATGGATCTGAGGAGCCTGAGATCTGACG ACACGGCCGTGTATTACTGTGCGAGAACCGCTGC TGGGTATAGCAGCAGCTGGTTTGACTACTGGGGC CAGGGAACCCTGGTCACCGTCTCCTCA |
| | | | SEQ ID NO: 24629 | SEQ ID NO: 28635 |

FIGURE 50

| | | | | |
|---|---|---|---|---|
| | | AA | EIVMTQSPATLSVSPGERATLSCRASQSVSSNLA WYHQKPGQAPRLLIYGASTRATGIPARFSGSGSG TEFTLTISSLQSEDFALYYCQQYNNWPPWPFGQ GTKVEIK | QVQLVQSGAEVKKPGASVKVSCKASDYTFTSYGIS WVRQAPGQGLEWMGWISAYNGNTTYAQKLRGRV TMTTDTSTSTAYMDLRSLRSDDTAVYYCARTAAG YSSSWFDYWGQGTLVTVSS |
| | | | SEQ ID NO: 24630 | SEQ ID NO: 28636 |
| IPS:393054 | 21-225_29G8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCTGAAACCAGGGAAAGC CCCTAAGCGCCTGATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TAACTGTCTACAGCATAATAGTTATCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAACT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACTGGG GATGACGAGTGACTACTGGGGCCAGGGAACCCT GGTCACCGTCTCCTCA |
| | | | SEQ ID NO: 24631 | SEQ ID NO: 28637 |
| | | AA | DIQMTQSPSSLSASVGDRVTITCRASQGIRNDLG WYQLKPGKAPKRLIYAASSLQSGVPSRFSGSGSG TEFTLTISSLQPEDFATYNCLQHNSYPLTFGGGT KVEIK | QVQLVESGGGVVQPGRSLRLSCAASGFTFSDYGM HWVRQAPGKGLEWVAVIWYDETNKYYADSVKGR FTISRDNSKNTLYLQMNSLRAEDTAVYYCARELGM TSDYWGQGTLVTVSS |
| | | | SEQ ID NO: 24632 | SEQ ID NO: 28638 |

1570

FIGURE 50

| iPS:393056 | 21-225_30F3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTACAAAGTGGGGTCCCATCAAGGTTCAGCGG CAGTGGATCTGGGACAGATTTCACTCTCACAA TCAGCAGCCTGCAGCCTGAAGATTTTGCAAGT TATTACTGTCTACAGCATAATAGTTACCCGTTC ACTTTCGGCGGAGGGACCAAGGTGGAGATCA AA | CAGGTGCAGCTGGTGGAATCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGTAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAAATGGG CTGGTACGATGACTACTGGGGCCAGGGAACCCT GGTCACCGTCTCCTCA |
| | | | SEQ ID NO: 24633 | SEQ ID NO: 28639 |
| | | AA | DIQMTQSPSSLSASVGDRVTITCRASQGIRNDLG WYQQKPGKAPKRLIYTASSLQSGVPSRFSGSGSG TDFTLTISSLQPEDFASYYCLQHNSYPFTFGGGT KVEIK | QVQLVESGGGVVQPGRSLRLSCAASGFTFSSYGMH WVRQAPGKGLEWVAVIWYDVSNKYYADSVKGRF TISRDNSKNTLYLQMNSLRAEDTAVYYCAREMGW YDDYWGQGTLVTVSS |
| | | | SEQ ID NO: 24634 | SEQ ID NO: 28640 |
| iPS:393058 | 21-225_31H3 | NA | GACATCCAGATGACACAGTCTCCATCCTCCCT GTCTGCATCTGTAGGAGACAGAGTCACCATCA CTTGCCGGGCAAGTCAGGACATTAGAGATGAT TTAGGCTGGTATCAGCAGAAACCAGGGAAAG CCCCTAGGCGCCTGATCTATGCTGCATTCAGC TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATACTAGTTACCCTCCT ACTTTCGGCGGAGGGACCAAGGTGGAGATCA AA | GAGGTGCAGGTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCCGCTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAAGGGGAGTTA CTAGAGGACTACTACTACTACGGAATGGACGTCT GGGGCCAAGGGACCACGGTCACCGTCTCCTCA |
| | | | SEQ ID NO: 24635 | SEQ ID NO: 28641 |

FIGURE 50

| | | | | |
|---|---|---|---|---|
| | | AA | DIQMTQSPSSLSASVGDRVTITCRASQDIRDDLG WYQQKPGKAPRRLIYAAFSLQSGVPSRFSGSGS GTEFTLTISSLQPEDFATYYCLQHTSYPPTFGGGT KVEIK | EVQVLESGGGLVQPGGSLRLSCAASGFTFSSYAMN WVRQAPGKGLEWVSAISGRGGNTFYADSVKGRFTI SRDNSKNTLYLQMNSLRAEDTAVYYCAKGELLED YYYYGMDVWGQGTTVTVSS |
| | | | SEQ ID NO: 24636 | SEQ ID NO: 28642 |
| iPS:393060 | 21-225_32G12 | NA | GACATCCAGATGACCCAGTCTCCATCCTCCCT GTCTGCATCTGTAGGAGACAGAGTCACCATCA CTTGCCGGGCAAGTCAGGACATTAGAAGTGAT TTAGGCTGGTATCAGCAGAGACCAGGAAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTACAGCCTGAAGATTTTGCAACT TATTACTGTCTGCAGCATACTATTTACCCTCCT ACTTTCGGCGGAGGGACCAAGGTGGAGATCA AA | GAGGTGCAGCTGTTGGAGTCTGGGGGAGGCTTGC TACAGCCTGGGGGGGTCCCTGAGACTCTCCTGTGC AGCCTCTGGATTCACCTTTAATAGCTATGCCATG AGCTGGGTCCGCCAGGCTCCAGGGAAGGGGCTG GAGTGGGTCTCCTCTATTAGTGGTCGTGGTGGTA GCACATTCCACGCAGACTCCGTGAAGGGCCGGTT CACCATCTCCAGAGACAATTCCAGGAACACGCTG TATCTGCAAATGAACAGCCTGAGAGCCGAGGAC ACGGCCGTATATTACTGTGTGAAAGGGGGAGCTAC TAGAGGACTACTACTTCTACGGTATGGACGTCTG GGGCCAAGGGACCACGGTCACCGTCTCCTCA |
| | | | SEQ ID NO: 24637 | SEQ ID NO: 28643 |
| | | AA | DIQMTQSPSSLSASVGDRVTITCRASQDIRSDLG WYQQRPGKAPKRLIYAASSLQSGVPSRFSGSGS GTEFTLTISSLQPEDFATYYCLQHTIYPPTFGGGT KVEIK | EVQLLESGGGLLQPGGSLRLSCAASGFTFNSYAMS WVRQAPGKGLEWVSSISGRGGSTFHADSVKGRFTI SRDNSRNTLYLQMNSLRAEDTAVYYCVKGELLED YYFYGMDVWGQGTTVTVSS |
| | | | SEQ ID NO: 24638 | SEQ ID NO: 28644 |

FIGURE 50

| iPS:393062 | 21-225_33H3 | NA | GACATCCAGATGACCCAGTCTCCATCCTCCCT GTCTGCATCTGTTGGAGACAGAGTCACCATCA CTTGCCAGGCGAGTCAGGACATTTCCAACTTT TTAAAATTGGTTTCGGCAGAAACCAGGAAAAGC CCCTAACTCCCTGATCTACGATGCATCCAATTT GGTAACAGGGGTCCCATCAAGGTTCAGTGGAC GTGGATCTGGGACAGATTTTACTTTCACCATC AGCAGCCTGCAGCCTGAAGATTTTGCAACATA TTACTGTCAACAGTATGATAATCTCCCGATCA CCTTCGGCCAAGGGACACGGCTGGAGATTAAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCGGGCTCCAGGCAAGGGGCT GGAGTGGGTGGCAATTATATCATATGGTGGAAGT AATAACTTCTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGCGAGAAGGGGGTAT AGCAGTGGAGGCTACGGTATGGACGTCTGGGGC CAAGGGACCACGGTCACCGTCTCCTCA |
| | | | SEQ ID NO: 24639 | SEQ ID NO: 28645 |
| | | AA | DIQMTQSPSSLSASVGDRVTITCQASQDISNFLN WFRQKPGKAPNSLIYDASNLVTGVPSRFSGRGS GTDFTFTISSLQPEDFATYYCQQYDNLPITFGQGT RLEIK | QVQLVESGGGVVQPGRSLRLSCAASGFTFSSYGMH WVRRAPGKGLEWVAIISYGGSNNFYADSVKGRFTI SRDNSKNTLYLQMNSLRAEDTAVYYCARRGYSSG GYGMDVWGQGTTVTVSS |
| | | | SEQ ID NO: 24640 | SEQ ID NO: 28646 |
| iPS:393064 | 21-225_33A9 | NA | GACATCCAGATGACCCAGTCTCCATCCTCCCT GTCTGCATCTGTAGGAGACAGAGTCACCATCA CTTGCCGGGCAAGTCAGAGCATTAGCAGGTAT TTAAGTTGGTATCAGCAGAAACCAGGGAGAG CCCCTAACCTCCAGATCTATGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAATATCCCGATC ACCTTCGGCCAAGGGACACGACTGGAGATTAA A | CAGGTGCAGCTGGTGCAGTCTGGGGGCTGAGGTG AAGAAGCCTGGGGCCTCAGTGAAGGTCTCCTGTA AGGCTTCTGGATACACCTTCACCAGTTATGATAT CAACTGGGTGCGACAGGCCACTGGTCAAGGGCT TGAGTGGATGGGATGGATGAACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGGGATCTGA GGACACGGCCGTGTATTACTGTGCGAGAAAGAA GGCTAACGACTACTGGGGCCAGGGAACCCTGGT CACCGTCTCCTCA |
| | | | SEQ ID NO: 24641 | SEQ ID NO: 28647 |

FIGURE 50

| | | | | |
|---|---|---|---|---|
| | | AA | DIQMTQSPSSLSASVGDRVTITCRASQSISRYLSWYQQKPGRAPNLQIYAASSLQSGVPSRFSGSGSGTDFTLTISSLQPEDFATYYCQQSYNIPITFGQGTRLEIK | QVQLVQSGAEVKKPGASVKVSCKASGYTFTSYDINWVRQATGQGLEWMGWMNPNSGNTGYAQKFQGRVTMTRNTSISTAYMELSSLGSEDTAVYYCARKKANDYWGQGTLVTVSS |
| | | | SEQ ID NO: 24642 | SEQ ID NO: 28648 |
| iPS:393066 | 21-225_34D3 | NA | GACATCCAGATGACCCAGTCTCCATCCTCCCTGTCTGCATCTGTTGGAGACAGAGTCACCATCACTTGCCGGGCAAGTCAGAACATTTACAGCTATTTAAAATTGGTATCAGCAGAAACCAGGGAAAGACCCTAAACTCCTGATCTATGCTGCATCCAGTTTGCATAGTGGGGTCCCGTCACGGTTCAGTGGCAGTGGTTCTGGGACAGATTTCACTCTCACCATCAGCAGTCTGCAACCTGAAGATTTTGCAACTTTCTACTGTCAACAGAGTTACAGTACTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTTACTATGGCATGCACTGGGTCCGCCAGGCTCCAGGCAAGGGGCTGGAGTGGGTGGCAGTTATATGGCATGATGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGTTCGTTCTACTTTGACTACTGGGGCCAGGGAACCCTGGTCACCGTCTCCTCA |
| | | | SEQ ID NO: 24643 | SEQ ID NO: 28649 |
| | | AA | DIQMTQSPSSLSASVGDRVTITCRASQNIYSYLNWYQQKPGKDPKLLIYAASSLHSGVPSRFSGSGSGTDFTLTISSLQPEDFATFYCQQSYSTPLTFGGGTKVEIK | QVQLVESGGGVVQPGRSLRLSCAASGFTFSYYGMHWVRQAPGKGLEWVAVIWHDGSNKYYVDSVKGRFTISRDNSKNTLYLQMNSLRAEDTAVYYCARENSSSFYFDYWGQGTLVTVSS |
| | | | SEQ ID NO: 24644 | SEQ ID NO: 28650 |

FIGURE 50

| iPS:393068 | 21-225_34G9 | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCACCGGCAGTGGCTCTGGGACAGAATTCACTCTCACAATCAGCAGCCTGCAGCCTGAAGCTTTTGCAATTTATTACTGTCTCCAGCATACTATTTACCCTCCTACTTTCGGCGGAGGGACCAAGGTGTGGATCAAA | GAGGTGCAGCTGGTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGGAGCTACTAGAGGACTACTACTTCTACGGTATGGACGTCTGGGGCCAAGGGACCACGGTCACCGTCTCCTCA |
| | | | SEQ ID NO: 24645 | SEQ ID NO: 28651 |
| | | AA | DIQMTQSPSSLSASVGDRVTITCRASQDIRSDLGWYQQKPGKAPKRLIYAASSLQSGVPSRFTGSGSGTEFTLTISSLQPEAFAIYYCLQHTIYPPTFGGGTKVWIK | EVQLVESGGGLVQPGGSLRLSCAASGFTFSSYAMSWVRQAPGKGLEWVSAISGRGGSTFHADSVKGRFTISRDNSKNTLYLQMNSLRAEDTAVYYCVKGELLEDYYFYGMDVWGQGTTVTVSS |
| | | | SEQ ID NO: 24646 | SEQ ID NO: 28652 |
| iPS:393072 | 21-225_36C5 | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCACCGGCAGTGGATCTGGGACAGAATTCACTCTCACAATCAGCAGCGTGCAGCCTGAAGATTTTGCAACTTATTACTGTCTCCATCATCCTATTTACCCTCCTACTTTCGGCGGAGGGACCAAGGTGTGGATCAAA | GAGGTGCAGCTGGTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGGAGCTATTAGAGGACTACTACTTCTACGGTATGGACGTCTGGGGCCAAGGGACCACGGTCACCGTCTCCTCA |
| | | | SEQ ID NO: 24647 | SEQ ID NO: 28653 |

FIGURE 50

| | | | | |
|---|---|---|---|---|
| | | AA | DIQMTQSPSSLSASVGDRVTITCRASQDIRSDLG WYQQKPGKAPKRLIYAASSLQSGVPSRFTGSGS GTEFTLTISSVQPEDFATYYCLHHPIYPPTFGGGT KVWIK | EVQLVESGGGLVQPGGSLRLSCAASGFTFSSYAMN WVRQAPGKGLEWVSAISGRGGSTFHADSVKGRFTI SRDNSKNTLYLQMNSLRAEDTAVYYCVKGELLED YYFYGMDVWGQGTTVTVSS |
| | | | SEQ ID NO: 24648 | SEQ ID NO: 28654 |
| IPS:393074 | 21-225_33B1 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTTTCAGCAGAAACCAGGGAAAGC CCCTAAGCGCCTGATCTATACTGCATCCAGTTT GCAAAGTGGGGTCCCATCAAGGTTCAGCGGCA GTGGATCTGGGACAGAATTCACTCTCACAATT AGCAGCCTGCAGCCTGAAGATTTTGCAACTTA TTACTGTCTACAGCATAATAGTTACCCGCTCA CTTTCGGCGGAGGGACCAAGGTGGAGGTCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGCCAAGGGGCT GGAGTGGGTGGCAGTTATATGGTATGATAGAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGCGAGAGAAATGGG CTGGTACGATGACTACTGGGGCCAGGGAACCCT GGTCACCGTCTCCTCA |
| | | | SEQ ID NO: 24649 | SEQ ID NO: 28655 |
| | | AA | DIQMTQSPSSLSASVGDRVTITCRTSQGIRNDLG WFQQKPGKAPKRLIYTASSLQSGVPSRFSGSGSG TEFTLTISSLQPEDFATYYCLQHNSYPLTFGGGT KVEVK | QVQLVESGGGVVQPGRSLRLSCAASGFTFSDYGM HWVRQAPAKGLEWVAVIWYDRNNKYYADSVKG RFTISRDNSKNTLYLQMNSLRAEDTAVYYCAREMG WYDDYWGQGTLVTVSS |
| | | | SEQ ID NO: 24650 | SEQ ID NO: 28656 |

FIGURE 50

| iPS:393076 | 21-225_33A4 | NA | GACATCCAGATGACCCAGTCTCCATCCTCCCT GTCTGCATCTGTAGGAGACAGAGTCACCATCA CTTGCCGGGCAAGTCAGGACATTAGAAATGAT GTAGGCTGGTATCAGCAGAAACCAGGGAAAG CCCCTGAGCGCCTGATCTATGCTGCATCCAGT TTGCAACGTGGGGTCCCATCAAGGTTCAGCGG CAGTGGATCTGGGACAGAATTCACTCTCACAA TCAGCAGCCTGCAGCCTGAAGATTTTGCAAGA TATTACTGTCTACAGCATTATAGTTACCCTCCT ACTTTCGGCGGAGGGACCAAGGTGGAGATCA AA | GAGGTGCAGTTGTTGGAGTCTGGGGGAGGCTTGG TACAGTCTGGGGGGTCCCTGAGACTCTCCTGTGA AGCCTCAGGATTCATCTTTAGCAGCTATGCCATG AACTGGGTCCGCCAGGTTCCAGGGAAGGGGCTG GAGTGGGTCTCAGCTATTAGTCGTCGTGGTGGTA GCACATTCTACGCAGACTCCGTGAAGGGCCGGTT CACCATTTCCAGAGACAATTCCAAGAACACGCTG TATCTGCAAATGAACAGCCTGAGAGCCGAGGAC ACGGCCGTATATTTCTGTGCGAAAGGGGAACTAC TAGAGGACTACTCCTACTACGGTATCGACGTCTG GGGCCAGGGGACCACGGTCACCGTCTCCTCA |
| | | | SEQ ID NO: 24651 | SEQ ID NO: 28657 |
| | | AA | DIQMTQSPSSLSASVGDRVTITCRASQDIRNDVG WYQQKPGKAPERLIYAASSLQRGVPSRFSGSGS GTEFTLTISSLQPEDFARYYCLQHYSYPPTFGGG TKVEIK | EVQLLESGGGLVQSGGSLRLSCEASGFIFSSYAMN WVRQVPGKGLEWVSAISRRGGSTFYADSVKGRFTI SRDNSKNTLYLQMNSLRAEDTAVYFCAKGELLED YSYYGIDVWGQGTTVTVSS |
| | | | SEQ ID NO: 24652 | SEQ ID NO: 28658 |
| iPS:393078 | 21-225_33H11 | NA | GACATCCAGATGACCCAGTCTCCATCCTCACT GTCTGCATTTGTAGGAGACAGAGTCACCATCA CTTGTTGGGCGAGTCAGGGCATTAACAGTTAT TTAGCCTGGTTTCAGCAGAGACCAGGGAAAGC CCATAAGTCCTTGATCTATGCTGCATCCAGTTT GCAAGGTGGGGTCCCATCAAAGTTCAGCGGCA GTGGATCTGGGACAGATTTCATTCTCACCATC AGCAGCCTGCAGCGTGAAGATTTTGCAACTTA TTACTGCCAACAGTTTAATAGTTACCCTCTGAC GTTCGGCCAAGGGACCAAGGTGGAAATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAGCAGCCTGAGAGCCGAGGA CACGGCTGTGTACTACTGTGCGAGAACAAACGGT ATGGACGTCTGGGGCCAAGGGACCACGGTTACC GTCTCCTCA |
| | | | SEQ ID NO: 24653 | SEQ ID NO: 28659 |

FIGURE 50

| | | | | |
|---|---|---|---|---|
| | | AA | DIQMTQSPSSLSAFVGDRVTITCWASQGINSYLA WFQQRPGKAHKSLIYAASSLQGGVPSKFSGSGS GTDFILTISSLQREDFATYYCQQFNSYPLTFGQGT KVEIK | EVQLVESGGGLVKPGGSLRLSCAASGFTFSSYSMN WVRQAPGKGLEWVSSISGSSSYIYYADSVKGRFTIS RDNAKNSLYLQMSSLRAEDTAVYYCARTNGMDV WGQGTTVTVSS |
| | | | SEQ ID NO: 24654 | SEQ ID NO: 28660 |
| IPS:393080 | 21-225_34F3 | NA | GACATCCAGATGACCCAGTCTCCGTCTTCCGT GTCTGCAACTGTAGGAGACAGAGTCACCAGCA CTTGTCGGGCGAGTCAGGGTATTAGTAAGTGG TTAGCCTGGTATCAGCAGAAGCCAGGGAAAG CCCCTAAACTCCTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGACTCTGCAACTT ACTATTGTCAACAGGCTAACAGTTTCCCTTTCA CTTTCGGCCCTGGGACCAAAGTGGATATCAAA | CAGGTGCAGCTGGTGCAGTCTGGGGGCTGAGGTG AAGAAGCCTGGGGGCCTCAGTGAAGGTCTCCTGC AAGGCTTCTGGATACACCTTCACCGGCTACCATA TGCACTGGGTGCGACAGGCCCCTGGACAAGGGC TTGACTGGATGGGATGGATCAACCCTAACAGTGG TGGCACAAACTATGCACAGAAGTTTCAGGGCAG GGTCACCATGACCAGGGACACGTCCATCAGCAC AGCCTATATGGAGCTGAGCAGGCTGAGATCTGA CGACACGGCCGTGTATTACTGTGCGAGAGACGGT ACCAGCTCCTTTGACTACTGGGGGCCAGGGAACCC TGGTCACCGTCTCCTCA |
| | | | SEQ ID NO: 24655 | SEQ ID NO: 28661 |
| | | AA | DIQMTQSPSSVSATVGDRVTSTCRASQGISKWLA WYQQKPGKAPKLLIYAASSLQSGVPSRFSGSGS GTDFTLTISSLQPEDSATYYCQQANSFPFTFGPGT KVDIK | QVQLVQSGAEVKKPGASVKVSCKASGYTFTGYHM HWVRQAPGQGLDWMGWINPNSGGTNYAQKFQGR VTMTRDTSISTAYMELSRLRSDDTAVYYCARDGTS SFDYWGQGTLVTVSS |
| | | | SEQ ID NO: 24656 | SEQ ID NO: 28662 |

FIGURE 50

| iPS:393082 | 21-225_34C11 | NA | GACATCCAGATGACCCAGTTTCCATCTTCCCT GTCTACATCTGTAGGAGACAGAGTCACCAGCA CTTGCCGGGCAAGTCAGAACATTAGGAACTTT TTAAATTGGTATCAGCAGAAACCTGAGAAAGA CCCTAAGCTCCAGATCTATGGTGCATCCACTTT GCAAAGTGGGGTCCCATCAAGGTTCAGTGGCA GTGGATTTGGGACAGATTTCACTCTCACCATC AGTAGTCTGCAACCTAAAGATTTTGCAACTTA CTACTGTCAACAGACTTGCAGTACCCCGCTCA CTTTCGGCGGAGGGACCAAGGTGGAGATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGTTATACCAT GAGCTGGGTCCGCCAGGCTCCAGGGAAGGGGCT GGAGTGGGTCTCATCCATTAGTGGTAGTAGTAAT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGATTAACTGGT TTTGACTACTGGGGCCAGGGAACCCTGGTCACCG TCTCCTCA |
|---|---|---|---|---|
| | | | SEQ ID NO: 24657 | SEQ ID NO: 28663 |
| | | AA | DIQMTQFPSSLSTSVGDRVTSTCRASQNIRNFLN WYQQKPEKDPKLQIYGASTLQSGVPSRFSGSGF GTDFTLTISSLQPKDFATYYCQQTCSTPLTFGGG TKVEIK | EVQLVESGGGLVKPGGSLRLSCAASGFTFSSYTMS WVRQAPGKGLEWVSSISGSSNYIYYADSVKGRFTIS RDNAKNSLYLQMNSLRAEDTAVYYCARLTGFDY WGQGTLVTVSS |
| | | | SEQ ID NO: 24658 | SEQ ID NO: 28664 |
| iPS:393084 | 21-225_35C6 | NA | GACATCCAGATGACCCAGTCTCCATCTTCCGT GTCTGCATCTGTAGGAGACAGAGTCACCATCA CTTGTCGGGCGAGTCAGGGTATTAGCAAATGG TTAGCCTGGTATCAGCAGAAACCAGGGAAAG CCCCTAAACCCCTGATCTATGCTGCATCCAGTT TGCAGAGTGGGGTCCCAACAAGGTTCAGCGGC AGTGGATCTGGGACAGATTTCACTCTCACCAT CAGCAGCCTGCAGCCTGAAGATTTTGCAATTT ACTATTGTCAACAGGCTAACAGTTTCCCATTC ACTTTCGGCCCTGGGACCAAAGTGGATATCAA A | CAGGTGCAGTTGGTGCAGTCTGGGACTGAGGTGA AGAAGCCTGGGGCCTCAGTGAAGGTCTCCTGCA AGGCTTCTGGATACACCTTCACCGGCGATTATAT GCACTGGGTGCGACAGGCCCCTGGACAAGGGCT TGAGTGGATGGGATGGATCAGCCCTAAAAATGG TGGCACAAACTATGCACAGAAGTTTCAGGGCAG GGTCACCATGACCAGGGACACGTCCATCAGCAC AGCCTACATGGAGCTGAACAGGCTGAGATCTGA CGACACGGCCGTGTATTACTGTGCGAGAGATGG AACTGGGTCCTTTGACTACTGGGGCCAGGGAACC CTGGTCACCGTCTCCTCA |
| | | | SEQ ID NO: 24659 | SEQ ID NO: 28665 |

1579

FIGURE 50

| | | | | |
|---|---|---|---|---|
| | | AA | DIQMTQSPSSVSASVGDRVTITCRASQGISKWLA WYQQKPGKAPKPLIYAASSLQSGVPTRFSGSGS GTDFTLTISSLQPEDFAIYYCQQANSFPFTFGPGT KVDIK | QVQLVQSGTEVKKPGASVKVSCKASGYTFTGDYM HWVRQAPGQGLEWMGWISPKNGGTNYAQKFQGR VTMTRDTSISTAYMELNRLRSDDTAVYYCARDGT GSFDYWGQGTLVTVSS |
| | | | SEQ ID NO: 24660 | SEQ ID NO: 28666 |
| IPS:393086 | 21-225_36H5 | NA | GACATCCAGATGACCCAGTCTCCATCTTCCGT GTCTGCATCTGTAGGAGACAGAGTCACCATCA CTTGTCGGGCGAGTCAGGGTATTAGCAGATGG TTAGCCTGGTATCAGCAGAAACCAGGGAAAG CCCCTGAACTCCTGATCTATGCTGCATCCCGTT TGCAAAGTGGGATCCCATCCAGGTTCAGCGGC AGTGGATCTGGGACAGACTTCACTCTCACCAT CAGCAGCCTGCAGCCTGAAGATTTTGCAACTT ACTATTGTCAACAGGCTAACAGTTTCCCTTTCA CTTTCGGCCCTGGGACCAAAGTGGATATCAAA | CAGGTGCAGCTGGTGCAGTCTGGGGGCTGAGGTG AAGAAGCCTGGGGGCCTCAATGAAGGTCTCCTGC AAGGCTTCTGGATACACCTTCACCGACTATCATA TGCACTGGGTGCGACAGGCCCCTGGACAAGGGC TTGAGTGGATGGGATGGATCAACCCTAATAGGG GTGGCACAAACTATGCACAGAAGTTTCAGGACA GGGTCACCATGACCAGGGACACGTCCATCAGCA CAGCCTACATGGAACTGAGCAGGCTGAGATCTG ACGACACGGCCGTGTATTTCTGTGCGAGAGATGG AACTGGGTCCTTTGACTACTGGGGCCAGGGAACC CTGGTCACCGTCTCCTCA |
| | | | SEQ ID NO: 24661 | SEQ ID NO: 28667 |
| | | AA | DIQMTQSPSSVSASVGDRVTITCRASQGISRWLA WYQQKPGKAPELLIYAASRLQSGIPSRFSGSGSG TDFTLTISSLQPEDFATYYCQQANSFPFTFGPGTK VDIK | QVQLVQSGAEVKKPGASMKVSCKASGYTFTDYHM HWVRQAPGQGLEWMGWINPNRGGTNYAQKFQDR VTMTRDTSISTAYMELSRLRSDDTAVYFCARDGTG SFDYWGQGTLVTVSS |
| | | | SEQ ID NO: 24662 | SEQ ID NO: 28668 |

FIGURE 50

| iPS:393088 | 21-225_33D1 | NA | GACATCGTGATGACCCAGTCTCCAGACTCCCT GTCTGTGTCTTTGGGCGAGAGGGCCACCATCA ACTGCAAGTCCATCCAGAGTGTTTTATACAGA TCCAACAATAAGAACTACTTAACTTGGTATCA GCAGAAACCAGGACAGCCTCGTAAACTGTTCA TTTATTGGGCATCTACCCGGGAATCCGGGGTC CTTGACCGATTCAGTGGCAGCGGGTGTGGGAC AGATTTCACTCTCACCATCATCAGCCTGCAGG CTGAAGATGTGGCACTTTATTACTGTCAGCAA TATTATAGTTCTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGACA TTAACTGGGTGCGACAGGCCACTGGACAAGGGC TTGAGTGGATGGGATGGATGCACCCTAACAGTGG TAACACAGGCTTTGCACAGAAGTTCCGGGGCAG AGTCACCATGACCAGAAACACCTCCATAAGCAC AGCCTACATGGAACTGAGCAGCCTGAGATCTGA GGACACGGCCGTGTATTACTGTGCGAGCAGCAGT GGCTGGTACTTTTTTGACTACTGGGGCCAGGGAA CCCTGGTCACCGTCTCCTCA |
|---|---|---|---|---|
| | | | SEQ ID NO: 24663 | SEQ ID NO: 28669 |
| | | AA | DIVMTQSPDSLSVSLGERATINCKSIQSVLYRSNN KNYLTWYQQKPGQPRKLFIYWASTRESGVLDRF SGSGCGTDFTLTIISLQAEDVALYYCQQYYSSPC SFGQGTKLEIK | QVQLVQSGAEVKKPGASVKVSCKASGYTFTNYDIN WVRQATGQGLEWMGWMHPNSGNTGFAQKFRGR VTMTRNTSISTAYMELSSLRSEDTAVYYCASSSGW YFFDYWGQGTLVTVSS |
| | | | SEQ ID NO: 24664 | SEQ ID NO: 28670 |
| iPS:393090 | 21-225_33A5 | NA | GACATCCAGATGACCCAGTCTCCATCCTCACT GTCTGCATCTGTAGGAGACAGAGTCACCATCA CTTGTCGGGCGAGTCAGGGCATTAGCAATTAC TTAGCCTGGTTTCAGCAGAAACCAGGGAAAGC CCCTAAGTCCCTGATCTCTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAACGTGGATATCAAA | GAGGTGCAGCTGTTAGAGTCTGGGGGAGGCTTG GTACAGCCTGGGGGGTCCCTGAGACTCTCCTGTG CAGCCTCTGGATTCACCTTTAGCAGCTATGTCAT AAACTGGGTCCGCCAGGCTCCAGGGAAGGGGCT GGAGTGGGTCTCAGCTATTAGTGGTAGTGGTGTT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAGAACTTCCCT CTTTGACTACTGGGGCCAGGGAACCCTGGTCACC GTCTCCTCA |
| | | | SEQ ID NO: 24665 | SEQ ID NO: 28671 |

FIGURE 50

| | | | | |
|---|---|---|---|---|
| | | AA | DIQMTQSPSSLSASVGDRVTITCRASQGISNYLA WFQQKPGKAPKSLISAASSLQSGVPSKFSGSGSG TDFTLTISSLQPEDFATYYCQQYNSYPFTFGPGT NVDIK | EVQLLESGGGLVQPGGSLRLSCAASGFTFSSYVINW VRQAPGKGLEWVSAISGSGVSTYYADSVKGRFTIS RDNSKNTLYLQMNSLRAEDTAVYYCARTSLFDYW GQGTLVTVSS |
| | | | SEQ ID NO: 24666 | SEQ ID NO: 28672 |
| iPS:393092 | 21-225_33C12 | NA | GACATCCAGATGACCCAGTCTCCATCCTCCCT GTCTACATCTGTAGGAGACAGAGTCACCATCA CTTGCCGGGCAAGTCAGAGCATTATCAGCTAT TTAAATTGGTATCAGCAGAAACCAGGGAAAG CCCCTAAACTCCTGATCTATGTTGCATCCAGTT TGCAAGGTGGGGTCCCATCAAGGTTCAATGGC AGTGGATCTGGGACAGATTTCACTCTCACCAT CAGCAGTCTGCAACCTGAAGATTTTGCAACTT ACTACTGTCAACAGAGTTACAGTACCCCGTAC ACTTTCGGCGGGGGGACCAAGGTGGAGATCA AA | CAGGTGCAGTTGGTGGAATCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAAAATAG CAGCTCGTACTACTTTGACTACTGGGGCCAGGGA ACCCTGGTCACCGTCTCCTCA |
| | | | SEQ ID NO: 24667 | SEQ ID NO: 28673 |
| | | AA | DIQMTQSPSSLSTSVGDRVTITCRASQSIISYLNW YQQKPGKAPKLLIYVASSLQGGVPSRFNGSGSG TDFTLTISSLQPEDFATYYCQQSYSTPYTFGGGT KVEIK | QVQLVESGGGVVQPGRSLRLSCAASGFTFSHYGM HWVRQAPGKGLEWVAVIWYDGSNKYYADSVKGR FTISRDNSKNTLYLQMNSLRAEDTAVYYCARENSS SYYFDYWGQGTLVTVSS |
| | | | SEQ ID NO: 24668 | SEQ ID NO: 28674 |

FIGURE 50

| iPS:393094 | 21-225_34C4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAACAGAAACCAGGGAAAG CCCCTAAGCGCCTGATCTATACTGCATCCAAT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAGTTCTTACCCCATC ACCTTCGGCCAAGGGACACGACTGGAGATTAA A | CAGCTGCAGCTGCAGGAGTCGGGCCCAGGACTG GTGAAGCCTTCGGAGACCCTGTCCCTCACCTGCA CTGTCTCTGGTGGCTCCATCAGTAGAAGTAGTTA CTACTGGGGCTGGATCCGCCAGTCCCCAGGGAA GGGGCTGGAGTGGATTGGGAGTATCTATTATAGT GGGAGCACCGCCTACAATCCGTCCCTCAAGAGTC GAGTCACCATATCCGTAGACACGTCCAAGAACC AGGTCTCCCTGAAGCTGAGCTCCGTGACCGCCGC AGACACGGCTGTGTATTACTGTGCGAGACTGAGC AGCAGCTGGTCTTTTGACTACTGGGGCCAGGGAA CCCTGGTCACCGTCTCCTCA |
| | | | SEQ ID NO: 24669 | SEQ ID NO: 28675 |
| | | AA | DIQMTQSPSSLSASVGDRVTITCRASQGIRNDLG WYQQKPGKAPKRLIYTASNLQSGVPSRFSGSGS GTEFTLTISSLQPEDFATYYCLQHSSYPITFGQGT RLEIK | QLQLQESGPGLVKPSETLSLTCTVSGGSISRSSYYW GWIRQSPGKGLEWIGSIYYSGSTAYNPSLKSRVTIS VDTSKNQVSLKLSSVTAADTAVYYCARLSSSWSFD YWGQGTLVTVSS |
| | | | SEQ ID NO: 24670 | SEQ ID NO: 28676 |
| iPS:393096 | 21-225_34D11 | NA | GACATCCAGATGACCCAGTCTCCATCCTCCCT GTCTGCATCTGTAGGAGACAGAGTCACCATCA CTTGCCGGGCAAGTCAGGACATTAGAAGTGAT TTAGGCTGGTATCAGCAGAAACCAGGGAAAG CCCCTAAGCGCCTGATCTATGCTGCATCCAGT TTGCAAAGTGGGGTCCCATCCAGGTTCAGCGG CAGTGGATCTGGGACAGAATTCACTCTCACAG TCATCAGCCTGCAGCCTGAAGATTTTGCAACT TATTACTGTCTACAGCATACTATTTACCCTCCT ACTTTCGGCGGAGGGACCAAGGTGGGGATCA AA | GAGGTGCAGCTGTCGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCCGCTATTAGTGGTCGTGGTGGT AGCACATTCCACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGTGAAAGGGGGAGTT AGTAGAGGACTACTACTTCTACGGTATGGACGTC TGGGGCCAAGGGACCACGGTCACCGTCTCCTCA |
| | | | SEQ ID NO: 24671 | SEQ ID NO: 28677 |

FIGURE 50

| | | | | |
|---|---|---|---|---|
| | | AA | DIQMTQSPSSLSASVGDRVTITCRASQDIRSDLG WYQQKPGKAPKRLIYAASSLQSGVPSRFSGSGS GTEFTLTVISLQPEDFATYYCLQHTIYPPTFGGGT KVGIK | EVQLSESGGGLVQPGGSLRLSCAASGFTFSSYAMN WVRQAPGKGLEWVSAISGRGGSTFHADSVKGRFTI SRDNSKNTLYLQMNSLRAEDTAVYYCVKGELVED YYFYGMDVWGQGTTVTVSS |
| | | | SEQ ID NO: 24672 | SEQ ID NO: 28678 |
| IPS:393098 | 21-225_35G6 | NA | GACATCCAGATGACCCAGTCTCCATCTTCCGT GTCTGCATCTGTAGGCGACAGACTCACCATCA CTTGTCGGGCGAGTCAGGGTATTAGCCGGTGG TTAGCCTGGTATCAGCAGAAAGTGGGGAAAGT CCCCAAACTCCTGATCTATGCTGCATCCAGGT TGCAAAGTGGGGTCCCATCAAGGTTCAGCGGC AGTGGATCTGGGACAGCTTTCACTCTCACCAT CGGCAGCCTGCAGCCTGAAGATTTTGCAACTT ACTATTGTCAACAGGCTAACAGTTTCCCGTTC ACTTTCGGCCCTGGGACCAAAGTGGATCTCAA A | CAGGTGCAGCTGGTGCAGTCTGGGGCTGACGTG AAGAAGCCTGGGGCCTCAGTGAAGGTCTCCTGC AAGGCTTCTGGATACACCTTCACCGACTACCATA TACACTGGGTGCGACAGGCCCCTGGACAAGGGC TTGAGTGGATGGGATGGATCAACCCTAACAATGG TGGCACACACTATGCACAGGAGTTTCAGGGCAG GGTCACCATGACCAGGGACACGTCCATCAGCAC AGCCTACATGGAGCTGAGTAGCCTGAGATCTGAC GACACGGCCGTGTATTACTGTGCGAGAGATGGA ACTGGGTCCTTTGACTACTGGGGCCAGGGAACCC TGGTCACCGTCTCCTCA |
| | | | SEQ ID NO: 24673 | SEQ ID NO: 28679 |
| | | AA | DIQMTQSPSSVSASVGDRLTITCRASQGISRWLA WYQQKVGKVPKLLIYAASRLQSGVPSRFSGSGS GTAFTLTIGSLQPEDFATYYCQQANSFPFTFGPG TKVDLK | QVQLVQSGADVKKPGASVKVSCKASGYTFTDYHI HWVRQAPGQGLEWMGWINPNNGGTHYAQEFQGR VTMTRDTSISTAYMELSSLRSDDTAVYYCARDGTG SFDYWGQGTLVTVSS |
| | | | SEQ ID NO: 24674 | SEQ ID NO: 28680 |

FIGURE 50

| iPS:393100 | 21-225_36B8 | NA | GACATCCAGATGACCCAGTCTCCATCCTCCCT GTCTACTTCTGTAGGAGACAGAGTCACCATCA CTTGCCGGGCAAGTCAGAGCATTATCAGCTAT TTAAATTGGTATCAGCAGAAACCAGGGAAAG CCCCTAAACTCCTGATCTATGTTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCGACTT ACTACTGTCAACAGAGTTACAGTACCCCGTAC ACTTTCGGCGGAGGGACCAAGATGGAGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGCATGATGGAAG TAATAAATACTATGGAGACTCCGTGAAGGGCCG ATTCACCATCTCCAGAGACAATTCCAAGAACACA CTGTATCTGCAAATGAACAGCCTGAGAGCCGAG GACACGGCTGTGTATTACTGTGCGAGAGAAAATA GCAGCTCGTACTACTTTGACTACTGGGGCCAGGG AACCCTGGTCACCGTCTCCTCA |
| | | | SEQ ID NO: 24675 | SEQ ID NO: 28681 |
| | | AA | DIQMTQSPSSLSTSVGDRVTITCRASQSIISYLNW YQQKPGKAPKLLIYVASSLQSGVPSRFSGSGSGT DFTLTISSLQPEDFATYYCQQSYSTPYTFGGGTK MEIK | QVQLVESGGGVVQPGRSLRLSCAASGFTFSNYGM HWVRQAPGKGLEWVAVIWHDGSNKYYGDSVKGR FTISRDNSKNTLYLQMNSLRAEDTAVYYCARENSS SYYFDYWGQGTLVTVSS |
| | | | SEQ ID NO: 24676 | SEQ ID NO: 28682 |
| iPS:393102 | 21-225_33F1 | NA | GACATCCAGATGACCCAGTCTCCATCCTCCCT GTCTGCATCTGTAGGAGACAGAGTCACCATCA CTTGCCGGACAAGTCAGGACATTAGAAGTGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAATT TATTACTGTCTACAGCATACTATTTACCCTCCT ACTTTCGGCGGAGGGACCAAGGTGGAGATCA AA | GAGGTGCAGCTGTTGGAGTCTGGGGGAGGCTTGC TACAGCCGGGGGGGTCCCTGAGACTCTCCTGTGC AGCCTCTGGATTCACCTTTAGCAGCTATGCCATG AGCTGGGTCCGCCAGGCTCCAGGGAAGGGGCTG GAGTGGGTCTCTTCTATTAGTGGTCGTGGTGGTA GCACATTCCACGCAGACTCCGTGAAGGGCCGGTT CACCATCTCCAGAGACAATTCCAAGAACACGCTG TATCTGCAAATGAACAGCCTGAGAGCCGAGGAC ACGGCCGTATATTACTGTGTGAAGGGGGGAGCTAC TTGAGGACTACTACTTCTACGGTATGGACGTCTG GGGCCAAGGGACCACGGTCACCGTCTCCTCA |
| | | | SEQ ID NO: 24677 | SEQ ID NO: 28683 |

FIGURE 50

| | | | | |
|---|---|---|---|---|
| | | AA | DIQMTQSPSSLSASVGDRVTITCRTSQDIRSDLG WYQQKPGKAPKRLIYAASSLQSGVPSRFSGSGS GTEFTLTISSLQPEDFAIYYCLQHTIYPPTFGGGT KVEIK | EVQLLESGGGLLQPGGSLRLSCAASGFTFSSYAMS WVRQAPGKGLEWVSSISGRGGSTFHADSVKGRFTI SRDNSKNTLYLQMNSLRAEDTAVYYCVKGELLED YYFYGMDVWGQGTTVTVSS |
| | | | SEQ ID NO: 24678 | SEQ ID NO: 28684 |
| iPS:393104 | 21-225_33A7 | NA | GACATCCAGATGACCCAGTCTCCATCCTCCCT GTCTGCATCTGTAGGAGACAGAGTCACCATCA CTTGCCGGGCAAGTCAGGACATTAGAAGTGAT TTAGGCTGGTATCAGCAGAAACCAGGGAAAG CCCCTAAGCGCCTGATCTATGTTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAGCAT ATTACTGTCTACAGCATACTATTTACCCTCCTA CTTTCGGCGGAGGGACCAAGGTGGAGATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTGTGCCAT GAGCTGGGTCCGCCAGGCTCCAGGGAAGGGGCT GGAGTGGGTCTCCGCTATTAGTGGTCGTGGTGGT AGCACATTCCACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGTGAAAGGGGAACT ACTAGAGGACTACTACTTCTACGGTATGGACGTC TGGGGCCAAGGGACCACGGTCACCGTCTCCTCA |
| | | | SEQ ID NO: 24679 | SEQ ID NO: 28685 |
| | | AA | DIQMTQSPSSLSASVGDRVTITCRASQDIRSDLG WYQQKPGKAPKRLIYVASSLQSGVPSRFSGSGS GTEFTLTISSLQPEDFAAYYCLQHTIYPPTFGGGT KVEIK | EVQLLESGGGLVQPGGSLRLSCAASGFTFSSCAMS WVRQAPGKGLEWVSAISGRGGSTFHADSVKGRFTI SRDNSKNTLYLQMNSLRAEDTAVYYCVKGELLED YYFYGMDVWGQGTTVTVSS |
| | | | SEQ ID NO: 24680 | SEQ ID NO: 28686 |

FIGURE 50

| iPS:393106 | 21-225_34A6 | NA | GACATCCAGATGACCCAGTCTCCATCCTCCCT GTCTGCATCTGTAGGAGACAGAGTCACCATCA CTTGCCGGACAAGTCAGGACATCAGAAATGAT TTAGGCTGGTATCAGCAGAAACCAGGGAAAG CCCCTAAGCGCCTGATCTTTGCTGCATCCAGTT TGCAAAGTGGGGTCCCATCAAGATTCAGCGGC AGTGGATCTGGGACAGAATTCACTCTCACAGT CAGCAGCCTGCAGCCTGAAGATTTTGCAACTT ATTACTGTCTACAGCATAATAGTTACCCTCCTA CTTTCGGCGGAGGGACCAAGGTGGAGATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAACAACTATGCCAT GAACTGGGTCCGCCAGGCTCCAGGGAAGGGGCT GGAGTGGGTCTCAGCTATTAGTCGTCGTGGTGGT AGCACATTCTACGCAGACTCCGTGAAGGGCCGGT TCACCATCTCCAGAGACAATTCCAAGAACACTCT GTATCTCCAAATGAACAGCCTGAGAGCCGAGGA CACGGCCGTATATTACTGTGCGAAAGGGGAGCT ACTAGAGGACTACTACTACTTCGCTATGGACGTC TGGGGCCAAGGGACCACGGTCACCGTCTCCTCA |
| | | | SEQ ID NO: 24681 | SEQ ID NO: 28687 |
| | | AA | DIQMTQSPSSLSASVGDRVTITCRTSQDIRNDLG WYQQKPGKAPKRLIFAASSLQSGVPSRFSGSGSG TEFTLTVSSLQPEDFATYYCLQHNSYPPTFGGGT KVEIK | EVQLLESGGGLVQPGGSLRLSCAASGFTFNNYAMN WVRQAPGKGLEWVSAISRRGGSTFYADSVKGRFTI SRDNSKNTLYLQMNSLRAEDTAVYYCAKGELLED YYYFAMDVWGQGTTVTVSS |
| | | | SEQ ID NO: 24682 | SEQ ID NO: 28688 |
| iPS:393108 | 21-225_34G11 | NA | GACATCCAGATGACCCAGTCTCCATCCTCCCT GTCTGCATCTGTGGGAGACAGAGTCACCATCA CTTGCCGGGCAAGTCAGAACATTAACAGGTAT TTAAATTGGTATCAGCAGAAACCAGGGAAAG CCCCTAAGCTCCTGATCTATGGTGCATCCAGTT TGCAAAGTGGGGTCCCATCAAGGTTCAGTGGC AGTGGATCTGGGACAGATTTCACTCTCACCAT CAGCACTCTGCAACCTGAAGATTTTGCAACTT ACTACTGTCAACAGACTTACATTACCCCGCTC ACTTTCGGCGGAGGGACCAAGGTGGAGATCA AA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAGGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGATATT AGTAATTTCAGCAGCTGGTACGATTACTACGCTA TGGACGTCTGGGGCCAAGGGACCACGGTCACCG TCTCCTCA |
| | | | SEQ ID NO: 24683 | SEQ ID NO: 28689 |

FIGURE 50

| | | | | |
|---|---|---|---|---|
| | | AA | DIQMTQSPSSLSASVGDRVTITCRASQNINRYLNWYQQKPGKAPKLLIYGASSLQSGVPSRFSGSGSGTDFTLTISTLQPEDFATYYCQQTYITPLTFGGGTKVEIK | QVQLVQSGAEVKKPGASVKVSCKASGYTFTGYYMHWVRQAPGQGLEWMGWINPNSGGTNYAQKFQGRVTMTRDTSISTAYMELSRLRSDDTAVYYCARDISNFSSWYDYYAMDVWGQGTTVTVSS |
| | | | SEQ ID NO: 24684 | SEQ ID NO: 28690 |
| iPS:393110 | 21-225_35B7 | NA | GACATCCAGATGACCCAGTCTCCATCCTCCCTGTCTGCATCTGTAGGAGACAGAATCACCATCACTTGCCGGGCAAGTCAGGACATTAGAAGTGATTTAGGCTGGTATCAGCAGAAACCAGGGAAAGCCCCTGAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGAGCTACTAGAGGACTACTACTTCTACGGTATGGACGTCTGGGGCCAAGGGGCCACGGTCACCGTCTCCTCA |
| | | | SEQ ID NO: 24685 | SEQ ID NO: 28691 |
| | | AA | DIQMTQSPSSLSASVGDRITITCRASQDIRSDLGWYQQKPGKAPERLIYAASSLQSGVPSRFSGSGSGTEFTLTISSLQPEDFATYYCLQHTIYPPTFGGGTKVEIK | EVQLLESGGGLVQPGGSLRLSCAASGFTFSSYAMSWVRQAPGKGLEWVSAISGRGGSTFHADSVKGRFTISRDNSKNTLYLQMNSLRAEDTAVYYCVKGELLEDYYFYGMDVWGQGATVTVSS |
| | | | SEQ ID NO: 24686 | SEQ ID NO: 28692 |

FIGURE 50

| iPS:393112 | 21-225_33G1 | NA | GACATCCAGATGACCCAGTCTCCATCTTCCGT GTCTGTGTCTGTCGGAGACAGAGTCACCATCA CTTGTCGGGCGAGTCAGGGTATTAGCAGGTGG TTAGCCTGGTATCAGCAGAAACCTGGGAAAGC CCCAAAGCTCCTGATCTATGGTGCATACAGTC TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGTTGGCGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGGTGGATCAGCCCTAACAATGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGATGG AACTGGGTCCTTTGACTACTGGGGCCAGGGAACC CTGGTCACCGTCTCCTCA |
| | | | SEQ ID NO: 24687 | SEQ ID NO: 28693 |
| | | AA | DIQMTQSPSSVSVSVGDRVTITCRASQGISRWLA WYQQKPGKAPKLLIYGAYSLQSGVPSRFSGSGS GTDFTLTISSLQPEDFATYYCQQANSFPFTFGPGT KVDIK | QVQLAQSGAEVKKPGASVKVSCKASGYTFTGYYM HWVRQAPGQGLEWMGWISPNNGGTNYAQKFQGR VTMRDTSISTAYMELSRLRSDDTAVYYCARDGTG SFDYWGQGTLVTVSS |
| | | | SEQ ID NO: 24688 | SEQ ID NO: 28694 |
| iPS:393114 | 21-225_33G12 | NA | GACATCCAGATGACCCAGTCTCCATCCTCCCT GTCTGCATCTGTAGGAGACAGAGTCACCATCA CTTGCCGGGCAAGTCAGAGCATTAGCAACTAT TTAAATTGGTATCAACAGCAAACAGGGAAAG CCCCTAAGTTCCTGATTTATGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTACCCCATTC ACTTTCGGCCCTGGGACCAAAGTGGATATCAA A | GAGGTGCAGCTGTTGGAGTCTGGGGGAGACATG GTCCAGCCTGGGGGGTCCCTGAGACTCTCCTGTG CAGCCTCCGGATTCACCTTTAGCAGCTATGCCAT GAGCTGGGTCCGCCAGGCTCCAGGGAAGGGGCT GGAGTGGGTCTCAGTTATTAGTGGTAGTGGTGGT AGCTCATTCTACGCAGACTCCGTGAAGGGCCGGT TCACCATCTCCAGAGACAATTCCAAGAACACGCT GTATCTGCAAATGAACAGCCTGAGGGCCGAGGA CACGGCCGTATATTACTGTGCGAAAGATCGGGTG AGAGCTCATGATGGTTTTGATATCTGGGGCCAAG GGACAATGGTCACCGTCTCTTCA |
| | | | SEQ ID NO: 24689 | SEQ ID NO: 28695 |

FIGURE 50

| | | | | |
|---|---|---|---|---|
| | | AA | DIQMTQSPSSLSASVGDRVTITCRASQSISNYLN WYQQQTGKAPKFLIYAASSLQSGVPSRFSGSGS GTDFTLTISSLQPEDFATYYCQQSYSTPFTFGPGT KVDIK | EVQLLESGGDMVQPGGSLRLSCAASGFTFSSYAMS WVRQAPGKGLEWVSVISGSGGSSFYADSVKGRFTI SRDNSKNTLYLQMNSLRAEDTAVYYCAKDRVRAH DGFDIWGQGTMVTVSS |
| | | | SEQ ID NO: 24690 | SEQ ID NO: 28696 |
| IPS:393116 | 21-225_34G7 | NA | GACATCCAGATGACCCAGTCTCCATCTTCCGT GTCTGCATCTGTAGGAGACAGAGTCACCATCA CTTGTCGGGCGAGTCAGCTTATTAGCAAGTGG TTAGCCTGGTATCAGCAGAAACCAGGGAAAG CCCCTAAACTCCTGATCTATGCTGCATCCAGTT TGCAAAGTGGGGTCCCATTAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGC AGGGCTTCTGGATACACCTTCACCGACTACCATA TTCACTGGGTGCGACAGGCCCCTGGACAAGGGCT TGAGTGGATGGGATGGATCAACCCTAACAATGGT GGCACACACTATGCACAGAAGTTTCAGGGCAGG GTCACCATGACCAGGGACACGTCCATCAGCACA GCCTACATGGAGCTGAGCAGGCTGAGATCTGAC GACACGGCCGTGTATTATTGTGCGAGAGATGGAA CTGGGTCCTTTGACTACTGGGGCCAGGGAAACCT GGTCACCGTCTCCTCA |
| | | | SEQ ID NO: 24691 | SEQ ID NO: 28697 |
| | | AA | DIQMTQSPSSVSASVGDRVTITCRASQLISKWLA WYQQKPGKAPKLLIYAASSLQSGVPLRFSGSGS GTDFTLTISSLQPEDFATYYCQQANSFPFTFGPGT KVDIK | QVQLVQSGAEVKKPGASVKVSCRASGYTFTDYHIH WVRQAPGQGLEWMGWINPNNGGTHYAQKFQGRV TMTRDTSISTAYMELSRLRSDDTAVYYCARDGTGS FDYWGQGNLVTVSS |
| | | | SEQ ID NO: 24692 | SEQ ID NO: 28698 |

FIGURE 50

| iPS:393118 | 21-225_34H11 | NA | GACATCCAGATGACCCAGTCTCCATCCTCCCT GTCTGCATCTGTCGGAGACAGAGTCACCATCA CTTGCCGGGCAAGTCAGGACATTAGAAATGAT TTAGGCTGGTATCAGCAGAAACCAGGGAAAG CCCCTAAGCGCCTGATCTATGCTACATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCTCCT ACTTTCGGCGGAGGGACCAAGGTGGAGATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGGAGGGGCT GGAGTGGGTCTCCGCTATTAGTGGTCGTGGTGGT AGCACATTCTACGCAGACTCCGTGAAGGGCCGGT TCACCATCTCCAGAGACAATTCCAAGAACACGCT GTTTCTGCAAATGAACAGCCTGAGAGCCGAGGA CACGGCCGTTTATTACTGTGCGAAAGGGGAGCTA CTAGAGGACTACTACTACTACGGTATGGACGTCT GGGGGCCAAGGGACCACGGTCACCGTCTCCTCA |
| | | | SEQ ID NO: 24693 | SEQ ID NO: 28699 |
| | | AA | DIQMTQSPSSLSASVGDRVTITCRASQDIRNDLG WYQQKPGKAPKRLIYATSSLQSGVPSRFSGSGSG TEFTLTISSLQPEDFATYYCLQHNSYPPTFGGGTK VEIK | EVQLLESGGGLVQPGGSLRLSCAASGFTFSSYAMN WVRQAPGEGLEWVSAISGRGGSTFYADSVKGRFTI SRDNSKNTLFLQMNSLRAEDTAVYYCAKGELLED YYYYGMDVWGQGTTVTVSS |
| | | | SEQ ID NO: 24694 | SEQ ID NO: 28700 |
| iPS:393120 | 21-225_35H8 | NA | GACATCCAGATGACCCAGTCTCCATCCTCACT GTCTGCATCTGTAGGAGACAGAGTCACCATCA CTTGTCGGGCGAGTCAGGCCATTAGTAATTAT TTAGCCTGGTTTCAGCAGAAACCAGGGAAAGC CCCTAAGTCCCTGATCTATGGTGCGTCCGGTTT GCAAAGTGGGGTCCCATCAAAGTTCAGCGGCA GTGGATCTGGGACAGATTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATTTCAAA | GAGGTTCAACTGGTGGAGTCTGGGGGGGGCCTG GTCAAGCCTGGGGGGTCCCTGAGACTCTCCTGTG CAGCCTCTGGATTCACCTTCAGTAGTTATAGCAT GAACTGGGTCCGCCAGGCTCCAGGGAAGGGGCT GGAGTGGGTCTCGTCCATTAGTGGTACTGGTAGT TTCATATACTACGCAGACTCAGTGAAGGGCCGAT TCACCATCTCCAGAGACAACGCCAAGAAATCAG TGTATCTGCAAATGAACAGCCTGAGAGCCGAGG ACACGGCTGTGTATTACTGTGCGAGAGTCTCTGG CTTTGACTACTGGGGCCAGGGAACCCTGGTCACC GTCTCCTCA |
| | | | SEQ ID NO: 24695 | SEQ ID NO: 28701 |

FIGURE 50

| | | | | |
|---|---|---|---|---|
| | | AA | DIQMTQSPSSLSASVGDRVTITCRASQAISNYLAWFQQKPGKAPKSLIYGASGLQSGVPSKFSGSGSGTDFTLTISSLQPEDFATYYCQQYNSYPFTFGPGTKVDFK | EVQLVESGGGLVKPGGSLRLSCAASGFTFSSYSMNWVRQAPGKGLEWVSSISGTGSFIYYADSVKGRFTISRDNAKKSVYLQMNSLRAEDTAVYYCARVSGFDYWGQGTLVTVSS |
| | | | SEQ ID NO: 24696 | SEQ ID NO: 28702 |
| iPS:393122 | 21-225_33B2 | NA | GACATCCAGATGACCCAGTCTCCATCCTCCCTGTCTACATCTGTCGGAGACAAAGTCACCATCACTTGCCGGGCAAGTCAGAGCATTATCAGCTATTTAAAATTGGTATCAGCAGAAACCAGGGAAAGCCCCTAAACTCCTGATCTATGTTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCGTACACTTTCGGCGGGGGGGGACTAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGCATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGCTCGTACTACTTTGACTACTGGGGCCAGGGAACCCTGGTCACCGTCTCCTCA |
| | | | SEQ ID NO: 24697 | SEQ ID NO: 28703 |
| | | AA | DIQMTQSPSSLSTSVGDKVTITCRASQSIISYLNWYQQKPGKAPKLLIYVASSLQSGVPSRFSGSGSGTDFTLTISSLQPEDFATYYCQQSYSTPYTFGGGTKVEIK | QVQLVESGGGVVQPGRSLRLSCAASGFTFSNYGMHWVRQAPGKGLEWVAVIWHDGSNKYYADSVKGRFTISRDNSKNTLYLQMNSLRAEDTAVYYCARENSSSYYFDYWGQGTLVTVSS |
| | | | SEQ ID NO: 24698 | SEQ ID NO: 28704 |

EP 4 435 105 A2

FIGURE 50

| iPS:393124 | 21-225_33G7 | NA | GACATCCAGATGACCCAGTCTCCATCCTCCCTGTCTGCATCGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACCCTCACAATCAGCAGCCTGCAGCCTGAAGATTTTGCAACATATTACTGTCTACAGCATTATAGTTACCCTCCTACTTTCGGCGGAGGGACCAAGGTGGAGATCAAA | GAGGTTCAATTGTTGGAGTCTGGGGGAGGCTTGGTACAGCCTGGGGGGGTCCCTGAGACTCTCCTGTGCAGCCTCTGGATTCACCTTTAGCAACTATGCCATGAGCTGGGTCCGCCAGGCTCCAGGGAAGGGGCTGGAGTGGGTCTCAGCTATTAGTCGTCGTGGTGGTAGCACATTCTACGCAGACTCCGTGAAGGGCCAGTTCACCATTTCCAGAGACAATTCCAAGAACACGCTGTATCTGCAAATGAACAGCCTGAGAGCCGAGGACACGGCCGTGTATTACTGTGCGAAAGGGGGAGCTACTAGAGGACTACTCCTACTACGGTATGGACGTCTGGGGGCCAGGGGACCACGGTCACCGTCTCCTCA |
| | | | SEQ ID NO: 24699 | SEQ ID NO: 28705 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPPTFGGGTKVEIK | EVQLLESGGGLVQPGGSLRLSCAASGFTFSNYAMSWVRQAPGKGLEWVSAISRRGGSTFYADSVKGQFTISRDNSKNTLYLQMNSLRAEDTAVYYCAKGELLEDYSYYGMDVWGQGTTVTVSS |
| | | | SEQ ID NO: 24700 | SEQ ID NO: 28706 |
| iPS:393126 | 21-225_35D1 | NA | GACATCCAGATGACCCAGTCTCCATCCTCCCTGTCTGCATCGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTATTTACCCTCCCACTTTCGGCGGGGGGACCAAGGTGGAGATCAAA | GAGGTGCAACTGTTGGAGTCTGGGGGAGGCTTGGTACAGCCTGGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAACAACACGCTGTATCTGCAAATGAACAGCCTGAGAGCCGAGGACACGGCCGTATATTACTGTGTGAAAGGGGGAGTTACTAGAGGACTACTACTTCTACGGTATGGACGTCTGGGGGCCAAGGGGCCACGGTCACCGTCTCCTCA |
| | | | SEQ ID NO: 24701 | SEQ ID NO: 28707 |

1593

FIGURE 50

| | | | | |
|---|---|---|---|---|
| | | AA | DIQMTQSPSSLSASVGDRVTITCRASQDIRSDLG WYQQKPGKAPKRLIYAASSLQSGVPSRFSGSGS GTEFTLTISSLQPEDFATYYCLQHTIYPPTFGGGT KVEIK | EVQLLESGGGLVQPGGSLRLSCAASGFTFSSYAMS WVRQAPGKGLEWVSAISGRGGSTFHADSVKGRFTI SRDNSNNTLYLQMNSLRAEDTAVYYCVKGELLED YYFYGMDVWGQGATVTVSS |
| | | | SEQ ID NO: 24702 | SEQ ID NO: 28708 |
| iPS:393128 | 21-225_35F11 | NA | GACATCCAGATGACCCAGTCTCCATCCTCCCT GTCTGCATCTGTAGGAGACAGAGTCACCATCA CTTGCCGGGCAAGTCAGGACATTAGAAGTGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATACTGTTTACCCTCCT ACTTTCGGCGGAGGGACCAAGGTGGAGATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCCGCTATTAGTGGTCGTGGTGGT AGCACATTCCACGCAGACTCCATGAAGGGCCGG TTCACCATCTCCAGAGACAATTCCAAGAACACGC TGTATCTGCAAATGAACAGCCTGAGAGCCGAGG ACACGGCCGTATATTACTGTGTGAAAGGGGGAGCT ACTAGAGGACTACTACTTCTACGGTATGGACGTC TGGGGCCAAGGGACCACGGTCACCGTCTCCTCA |
| | | | SEQ ID NO: 24703 | SEQ ID NO: 28709 |
| | | AA | DIQMTQSPSSLSASVGDRVTITCRASQDIRSDLG WYQQKPGKAPKRLIYAASSLQSGVPSRFSGSGS GTEFTLTISSLQPEDFATYYCLQHTVYPPTFGGG TKVEIK | EVQLLESGGGLVQPGGSLRLSCAASGFTFSSYAMS WVRQAPGKGLEWVSAISGRGGSTFHADSMKGRFTI SRDNSKNTLYLQMNSLRAEDTAVYYCVKGELLED YYFYGMDVWGQGTTVTVSS |
| | | | SEQ ID NO: 24704 | SEQ ID NO: 28710 |

1594

FIGURE 50

| iPS:393130 | 21-225_33C2 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGTGAAAGCCCCTAAGCGCCTGATCTATGCTGCACCCAGTTTGCAAAGTGGGGTCCCATCAAGGTTCAGCGGCAGTGGATCTGGGACAGAATTCACTCTCACAATCAGCAGCCTGCAGCCTGAACATTTTGCAACTTATTACTGTCTACAGCATAATAGTTACCCGTGGACGTTCGGCCAGGGGACCAAGGTGGAAATCAAA | GAGGTGCAACTGGTGGAGTCTGGGGGAGGCCTGGTCAAGCCGGGGGGGTCCCTGAGACTCTCCTGTGCAGCCTCTGGATTCACCTTCAGTAGCTATACCATGAACTGGGTCCGCCAGGCTCCAGGGAAGGGGCTGCAGTGGGTCTCATCCATTAGTGGTAGTAGTAGTTACATATACTACGCGGACTCAGTGAAGGGCCGATTCACCATCTCCAGAGACAACGCCAAGAACTCACTGTTTCTGCAAATGAACAGCCTGAGAGCCGAGGACACGGCTATCTTTTACTGTGCGCGAGATCGGGGGGGGGACCTGGGGCCAGGGAACCCTGGTCACCGTCTCCTCA |
| | | | SEQ ID NO: 24705 | SEQ ID NO: 28711 |
| | | AA | DIQMTQSPSSLSASVGDRVTITCRASQGIRNDLGWYQQKPVKAPKRLIYAAPSLQSGVPSRFSGSGSGTEFTLTISSLQPEHFATYYCLQHNSYPWTFGQGTKVEIK | EVQLVESGGGLVKPGGSLRLSCAASGFTFSSYTMNWVRQAPGKGLQWVSSISGSSSYIYYADSVKGRFTISRDNAKNSLFLQMNSLRAEDTAIFYCARDRGGTWGQGTLVTVSS |
| | | | SEQ ID NO: 24706 | SEQ ID NO: 28712 |
| iPS:393132 | 21-225_33H7 | NA | GACATCCAGATGACCCAGTCTCCATCTTCCGTGTCTGCATCTGTAGGCGACAGAGTCACCATCACTTGTCGGGCGAGTCAGGGTATTAGCCGGTGGTTAGCCTGGTATCAGCAGAAAGTGGGGAAAGTCCCCAAACTCCTGATCTATGCTGCATCCAGGTTGCAAAGTGGGGTCCCATCAAGGTTCAGCGGCAGTGGATCTGGGACAGATTTCACTCTCACCATCGGCAGCCTGCAGCCTGAAGATTTTGCAACTTACTATTGTCAACAGGCTAACATTTTCCCGTTCACTTTCGGCCCTGGGACCAAAGTGGATCTCAAA | CAGGTGCAGCTGGTGCAGTCTGGGGCTGACGTGAAGAAGCCTGGGGCCTCAGTGAAGGTCTCCTGCAAGGCTTCTGGATACACCTTCACCGACTACCATATACACTGGGTGCGACAGGCCCCTGGACAAGGGCTTGAGTGGATGGGATGGATCAACCCTAACAATGGTGGCACACACTATGCACAGGAGTTTCAGGGCAGGGTCACCATGACCAGGGACACGTCCATCAGCACAGCCTACATGGAACTGAGTAGCCTGAGATCTGACGACACGGCCGTGTATCACTGTGCGAGAGATGGAACTGGGTCCTTTGACTACTGGGGCCAGGGAACCCTGGTCACCGTCTCCTCA |
| | | | SEQ ID NO: 24707 | SEQ ID NO: 28713 |

FIGURE 50

| | | | | |
|---|---|---|---|---|
| | | AA | DIQMTQSPSSVSASVGDRVTITCRASQGISRWLAWYQQKVGKVPKLLIYAASRLQSGVPSRFSGSGSGTDFTLTIGSLQPEDFATYYCQQANIFPFTFGPGTKVDLK | QVQLVQSGADVKKPGASVKVSCKASGYTFTDYHIHWVRQAPGQGLEWMGWINPNNGGTHYAQEFQGRVTMTRDTSISTAYMELSSLRSDDTAVYHCARDGTGSFDYWGQGTLVTVSS |
| | | | SEQ ID NO: 24708 | SEQ ID NO: 28714 |
| iPS:393134 | 21-225_34C2 | NA | GACATCCAGATGACCCAGTCTCCATCCTCCCTGTCTACATTTGTAGGAGACAGAGTCACCATTACTTGTCGGGCAAGTCAGAGAATTATCAGCTATTTAAAATTGGTTTCAGCAGAAACCAGGGAAAGCCCCTAAACTCCTGATCTATGTTGCATCCAGTTTGCAAAGTGGGGTCCCGTCAAGGTTCAGTGGCAGTGGATCTGGGACATATTTCACTCTCACCATCAGCAGTCTGCAACCTGAAGATTTTGCGACTTACTACTGTCAACAGAGTTACAGTACCCCGTACACTTTCGGCGGAGGGACCAAGATGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCACTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGCTCGTACTACTTTGACTACTGGGGCCAGGGAACCCTGGTCACCGTCTCCTCA |
| | | | SEQ ID NO: 24709 | SEQ ID NO: 28715 |
| | | AA | DIQMTQSPSSLSTFVGDRVTITCRASQRIISYLNWFQQKPGKAPKLLIYVASSLQSGVPSRFSGSGSGTYFTLTISSLQPEDFATYYCQQSYSTPYTFGGGTKMEIK | QVQLVESGGGVVQPGRSLRLSCAASGFTFSNYVMHWVRQAPGKGLEWVALIWYDGSNKYYADSVKGRFTISRDNSKNTLYLQMNSLRAEDTAVYYCARENSSSYYFDYWGQGTLVTVSS |
| | | | SEQ ID NO: 24710 | SEQ ID NO: 28716 |

FIGURE 50

| iPS:393136 | 21-225_34D8 | NA | GACATCCAGATGACCCAGTCTCCATCCTCCCT GTCTACATCTGTAGGAGACAGAGTCACCATCA CTTGCCGGGCAAGTCAGATCATTATCAGCTAT TTAAATTGGTATCAGCAGAAACCAGGGAAAG CCCCTAAACTCCTGATCTTTGTTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTACCCCGTAC ACTTTCGGCGGGGGGACCAAGGTGGAGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAAAATAG CAGCTCGTACTACTTTGACTACTGGGGCCAGGGA ACCCTGGTCACCGTCTCCTCA |
| | | | SEQ ID NO: 24711 | SEQ ID NO: 28717 |
| | | AA | DIQMTQSPSSLSTSVGDRVTITCRASQIISYLNW YQQKPGKAPKLLIFVASSLQSGVPSRFSGSGSGT DFTLTISSLQPEDFATYYCQQSYSTPYTFGGGTK VEIK | QVQLVESGGGVVQPGRSLRLSCAASGFTFSNYGM HWVRQAPGKGLEWVAVIWYDGSNKYYADSVKGR FTISRDNSKNTLYLQMNSLRAEDTAVYYCARENSS SYYFDYWGQGTLVTVSS |
| | | | SEQ ID NO: 24712 | SEQ ID NO: 28718 |
| iPS:393138 | 21-225_35E3 | NA | GACATCCAGATGACCCAGTCTCCATCCTCCCT GTCTGCATCTGTAGGAGACAGAGTCACCATCA CTTGCCAGGCGAGTCAGGACATTTTCAACTAT TTAAATTGGTATCAGCAGAAACCAGGAAAAG CCCCTAACCTCCTGATCTACGATGCCTCCAATT TGGAAACAGGGGTCCCATCAAGGTTCAGTGGA AGTGGATCTGGGACAGATTTTACTTTCACCAT CAGCAGCCTGCAGCCTGAAGATATTGCGACAT ATTTCTGTCAACAGTATGATAATCTCCCGATC ACCTTCGGCCAAGGGACACGACTGGATATTAG A | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGGTGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGTGAGAAGGGGGTA TAGCAGTGGAGGCTACGGTATGGACGTCTGGGG CCAAGGGACCACGGTCACCGTCTCCTCA |
| | | | SEQ ID NO: 24713 | SEQ ID NO: 28719 |

FIGURE 50

| | | | | |
|---|---|---|---|---|
| | | AA | DIQMTQSPSSLSASVGDRVTITCQASQDIFNYLN WYQQKPGKAPNLLIYDASNLETGVPSRFSGSGS GTDFTFTISSLQPEDIATYFCQQYDNLPITFGQGT RLDIR | QVQLVESGGGVVQPGRSLRLSCAASGFTFSSYGMH WVRQAPGKGLEWVAVISYGGSNKYYADSVKGRFT ISRDNSKNTLYLQMNSLRAEDTAVYYCVRRGYSSG GYGMDVWGQGTTVTVSS |
| | | | SEQ ID NO: 24714 | SEQ ID NO: 28720 |
| IPS:393140 | 21-225_35H12 | NA | GACATCCAGATGACCCAGTCTCCATCTTCCGT GTCTGCATCTGTAGGAGACAGAGTCACCATCA CTTGTCGGGCGAGTCAGGGTATTAGCAGATGG TTAGCCTGGTATCAGCAGAAACCAGGGAAAG CCCCTGAACTCCTGATCTATGCTGCATCCCGTT TGCAAAGTGGGATCCCATCCAGGTTCAGCGGC AGTGGATCTGGGACAGACTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGGCTGAGGTG AAGAAGCCTGGGGCCTCAGTGAAGGTCTCCTGC AAGGCTTCTGGATACACCTTCACCGACTACTATA TACACTGGGTGCGACAGGCCCCTGGACAAGGGC TTGAGTGGATGGGATGGATCAACCCTAATAGGG GTGGCACAAACTATGCACAGAAGTTTCAGGGCA GGGTCACCATGACCAGGGACACGTCCATCAGCA CAGCCTACATGGAACTGAGCAGGCTGAGATCTG ACGACACGGCCGTGTATTACTGTGCGAGAGATG GAACTGGGTCCTTTGACTACTGGGGGCCAGGGAAC CCTGGTCACCGTCTCCTCA |
| | | | SEQ ID NO: 24715 | SEQ ID NO: 28721 |
| | | AA | DIQMTQSPSSVSASVGDRVTITCRASQGISRWLA WYQQKPGKAPELLIYAASRLQSGIPSRFSGSGSG TDFTLTISSLQPEDFATYYCQQANSFPFTFGPGTK VDIK | QVQLVQSGAEVKKPGASVKVSCKASGYTFTDYYIH WVRQAPGQGLEWMGWINPNRGGTNYAQKFQGRV TMTRDTSISTAYMELSRLRSDDTAVYYCARDGTGS FDYWGQGTLVTVSS |
| | | | SEQ ID NO: 24716 | SEQ ID NO: 28722 |

FIGURE 50

| iPS:393142 | 21-225_33A3 | NA | GACATCCAGATGACCCAGTCTCCATCCTCACT GTCTGCATCTGTAGGAGACAGAGTCACCATCA CTTGTCGGGCGAGTCAGGGCATTAACAATTAT TTAGCCTGGTTTCAACAGAAACCAGGGAAAGC CCCTAAGTCCCTGATCTACGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTTTAATAGTTACCCTCCGA CGTTCGGCCAAGGGACCAAGGTGGAAATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATGGCAT GAACTGGGTCCGCCAGGCTCCAGGGAAGGGGCT GGAGTGGGTCTCATCCATTAGTGGTAGTAGTACT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAACAAACGGT ATGGACGTCTGGGGCCAAGGGACCACGGTCACC GTCTCCTCA |
| | | | SEQ ID NO: 24717 | SEQ ID NO: 28723 |
| | | AA | DIQMTQSPSSLSASVGDRVTITCRASQGINNYLA WFQQKPGKAPKSLIYAASSLQSGVPSKFSGSGSG TDFTLTISSLQPEDFATYYCQQFNSYPPTFGQGT KVEIK | EVQLVESGGGLVKPGGSLRLSCAASGFTFSSYGMN WVRQAPGKGLEWVSSISGSSTYIYYADSVKGRFTIS RDNAKNSLYLQMNSLRAEDTAVYYCARTNGMDV WGQGTTVTVSS |
| | | | SEQ ID NO: 24718 | SEQ ID NO: 28724 |
| iPS:393144 | 21-225_34D2 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTATTTGTATTGGTACCTGCA GAAGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACACTAAAAATCAGCCGGGTGGAGGCT GAGGATGTTGGGGTTTATTACTGCATGCAAAG TAAACAGCTTCCTCCTTTCGGCGGAGGGACCA AGGTGGAGATCAGA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGACA TTAACTGGGTGCGACAGGCCACTGGACAAGGGC TTGAGTGGGTGGGATGGCTGCACCCTAACAGTGG TACCACAGGCTTTGCACAGAAGTTCCGGGGCAG AGTCACCATGACCAGAAACACCTCCATAAGCAC AGCCTACTTGGAACTGAGCAGCCTGAGATCTGAG GACACGGCCGTGTATTACTGTGCGAGCAGCAGTG GCTGGTACTTTTTTGACTACTGGGGCCAGGGAAC CCTGGTCACCGTCTCCTCA |
| | | | SEQ ID NO: 24719 | SEQ ID NO: 28725 |

FIGURE 50

| | | | | |
|---|---|---|---|---|
| | | AA | DIVMTQTPLSLSVTPGQPASISCKSSQSLLHSDGK TYLYWYLQKPGQPPQLLIYEVSNRFSGVPDRFSG SGSGTDFTLKISRVEAEDVGVYYCMQSKQLPPF GGGTKVEIR | QVQLVQSGAEVKKPGASVKVSCKASGYTFTNYDIN WVRQATGQGLEWVGWLHPNSGTTGFAQKFRGRV TMTRNTSISTAYLELSSLRSEDTAVYYCASSSGWYF FDYWGQGTLVTVSS |
| | | | SEQ ID NO: 24720 | SEQ ID NO: 28726 |
| iPS:393146 | 21-225_34G8 | NA | GACATCCAGATGACCCAGTCTCCATCCTCCCT GTCTGCATCTGTAGGAGACAGAGTCACCATCA CTTGCCGGGCAAGTCAGGACATTAGAAGTGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATACTATTTACCCTCCT ACTTTCGGCGGAGGGACCAAGGTGGAGATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCCGCTATTAGTGGTCGTGGTGGT AGCACATTCCACGCAGACTCCGTGAAGGGCCGG TTCACCATCTCCAGAGACAATTCCAAGAACACGC TGTATCTGCAAATTAACAGCCTGAGAGCCGAGG ACACGGCCGTATATTACTGTGTGAAAGGGGAGCT ACTAGAGGACTACTACTTCTACGGTATGGACGTC TGGGGCCAAGGGACCACGGTCACCGTCTCCTCA |
| | | | SEQ ID NO: 24721 | SEQ ID NO: 28727 |
| | | AA | DIQMTQSPSSLSASVGDRVTITCRASQDIRSDLG WYQQKPGKAPKRLIYAASSLQSGVPSRFSGSGS GTEFTLTISSLQPEDFATYYCLQHTIYPPTFGGGT KVEIK | EVQLLESGGGLVQPGGSLRLSCAASGFTFSSYAMS WVRQAPGKGLEWVSAISGRGGSTFHADSVKGRFTI SRDNSKNTLYLQINSLRAEDTAVYYCVKGELLEDY YFYGMDVWGQGTTVTVSS |
| | | | SEQ ID NO: 24722 | SEQ ID NO: 28728 |

FIGURE 50

| iPS:393148 | 21-225_35E5 | NA | GACATCCAGATGATCCAGTCTCCATCCTCCCT GTCTGCATCTGTAGGAGACAGAGTCACCATCA CTTACCGGGCAAGTCAGAGCATTAGCAGCTAT TTAAATTGGTATCAGCAGAAACCAGCGAAAGC CCCTAAGCTCCACATCTATGGTGCATCCAGTTT CCAAAGTTGGGTCCCATCAAGGTTCAGTGGCA GTGGATCTTCGACAGATTTCACGCTCACCATC ATCAGTATGCAACCTGGAGATTATGCAACTTA CTACTGTCACCAGAGTTACAATCTCCCGATCA CCTTCGGCCAAGGGACCCGACTGGAGATTAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGAAAGA AGTCTAACGACTACTGGGGCCAGGGAACCCTGG TCACCGTCTCCTCA |
|---|---|---|---|---|
| | | | SEQ ID NO: 24723 | SEQ ID NO: 28729 |
| | | AA | DIQMIQSPSSLSASVGDRVTITYRASQSISSYLNW YQQKPAKAPKLHIYGASSFQSWVPSRFSGSGSST DFTLTIISMQPGDYATYYCHQSYNLPITFGQGTR LEIK | QVQLVQSGAEVKKPGASVKVSCKASGYTFTSYDIN WVRQATGQGLEWMGWMNPNSGNTGYAQKFQGR VTMTRNTSISTAYMELSSLRSEDTAVYYCARKKSN DYWGQGTLVTVSS |
| | | | SEQ ID NO: 24724 | SEQ ID NO: 28730 |
| iPS:393150 | 21-225_36A5 | NA | GACATCCAGATGACCCAGTCTCCATCCTCCCT GTCTGCATCTGTAGGAGACAGAGTCACCATCA CTTGCCGGACAAGTCAGGACATTAGAAATGAT TTAGGCTGGTATCAGCAGAAACCAGGGAAAG CCCCTAAGCGCCTGATCTTTGCTGCATCCAGTT TGCAAAGTGGGGTCCCATCAAGATTCAGCGGC AGTGGATCTGGGACAGAATTCACTCTCACAAT CAGCAGCCTGCAGCCTGAAGATTTTGCAACTT ATTACTGTCTACACCACAATAGTTACCCTCCTA AGTTTGGCGGAGGGATCAAGGTGGAGATCAC A | GAGGTGCAGCTGTTGGAGTCTGGGGGAGGCTTG GTACAGCCTGGGGGGTCCCTGAGACTCTCCTGTG CAGCCTCTGGATTCACCTTTAACAACTATGCCAT GAACTGGGTCCGCCAGGCTCCAGGGAAGGGGCT GGAGTGGGTCTCAGCTATTAGTCGTCGTGGTGGT AACACATTCTACGCAGACTCCGTGAAGGGCCGGT TCACCATCTCCAGAGACAATTCCAAGAACACGCT GTATCTCCAAATGAACAGCCTGAGAGCCGAGGA CACGGCCGTATATTACTGTGCGAAAGGGGAGCT ACTAGAGGACTACTACTACTACGCTATGGACGTC TGGGGCCAAGGGACCACGGTCACCGTCTCCTCA |
| | | | SEQ ID NO: 24725 | SEQ ID NO: 28731 |

FIGURE 50

| | | | | |
|---|---|---|---|---|
| | | AA | DIQMTQSPSSLSASVGDRVTITCRTSQDIRNDLG WYQQKPGKAPKRLIFAASSLQSGVPSRFSGSGSG TEFTLTISSLQPEDFATYYCLHHNSYPPKFGGGIK VEIT | EVQLLESGGGLVQPGGSLRLSCAASGFTFNNYAMN WVRQAPGKGLEWVSAISRRGGNTFYADSVKGRFTI SRDNSKNTLYLQMNSLRAEDTAVYYCAKGELLED YYYYAMDVWGQGTTVTVSS |
| | | | SEQ ID NO: 24726 | SEQ ID NO: 28732 |
| iPS:393152 | 21-225_25B3 | NA | GACATCCAGATGACCCAGTCTCCCTCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGCC AGTGGATCTGGGACAGATTTCACTCTCACCAT CAGCAGCCTGCAGCCTGAAGATTTTGCGACTT ACTGTTGTCAACAGTCTGACAGTTTCCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGCAGCTATGGCAT GCACTGGGTCCGCCAGGCTCCAGGCAAGGGGCT GGAGTGGGTGGCAGTTATATGGTATGATGGAAGT AATAAATATTATGCAGACTCCGTGAAGGGCCGAT TCACCATCTCCAGAGACAATTCCAAGAACACGCT ATATCTACAAATGAACAGCCTGAGAGCCGAGGA CACGGCTGTGTATTACTGTGCGAGAGATTCCTCC CCCTACGGTATGGACGTCTGGGGCCAAGGGACC ACGGTCACCGTCTCCTCA |
| | | | SEQ ID NO: 24727 | SEQ ID NO: 28733 |
| | | AA | DIQMTQSPSSVSASVGDRVTITCRASQGISSWLA WYQQKPGKAPKLLIYGASSLQSGVPSRFSASGS GTDFTLTISSLQPEDFATYCCQQSDSFPRTFGQGT KVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARDSSPY GMDVWGQGTTVTVSS |
| | | | SEQ ID NO: 24728 | SEQ ID NO: 28734 |

FIGURE 50

| iPS:393166 | 21-225_27G6 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGTTGGTCATCTATCAAGATAGGAAGCGGC CCTCAGGGATCCCTGAGCGATTCTCTGGCTCC AACTCTGGGAACACAGCCACTCTGACCATCAG CGGGACCCAGACTATGGATGAGGCTGACTATT ACTGTCAGGCGTGGGACAGCAGCTCTTATGTG GTATTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGGCTATGGCAT GCACTGGGTCCGCCAGGCTCCAGGCAAGGGGCT GGAGTGGGTGGCAATTATATGGTATGATGGAAGT AAAAAATACAATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATAGGGT ATATTGTAGTAGTACCAGCTGCTCCCCTTACTAC TACTACTACGGTATGGACGTCTGGGGCCAAGGG ACCACGGTCACCGTCTCCTCA |
|---|---|---|---|---|
| | | | SEQ ID NO: 24729 | SEQ ID NO: 28735 |
| | | AA | SYELTQPPSVSVSPGQTASITCSGDKLGDKYACW YQQKPGQSPVLVIYQDRKRPSGIPERFSGSNSGN TATLTISGTQTMDEADYYCQAWDSSSYVVFGG GTKLTVL | QVQLVESGGGVVQPGRSLRLSCAASGFTFSGYGM HWVRQAPGKGLEWVAIIWYDGSKKYNADSVKGR FTISRDNSKNTLYLQMNSLRAEDTAVYYCARDRVY CSSTSCSPYYYYYGMDVWGQGTTVTVSS |
| | | | SEQ ID NO: 24730 | SEQ ID NO: 28736 |

FIGURE 50

| iPS:393168 | 21-225_32B11 | NA | TCCTATGAACTGACTCAGCCACCCTCAGTGTC CGTGTCCCCAGGACAGACAGCCAGCATCACCT GCTCTGGAGATAAATTGGGGGATAAATATGCT TACTGGTATCAGCAGAAGCCAGGCCAGTCCCC TGTGTTGGTCATCTATCAAGATAGTAAGCGGT CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAGCACTGTGGTA TTCGGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGA TGGCACTAACTATGCACAGAAGTTTCAGGGCAG GGTCACCATGACCAGGGACACGTCCATCAGCAC AGCCTACATGGAGCTGAACAGGCTGAGATCTGA CGACACGGCCGTGTATTACTGTGCGAGGGGGTTT TACTATGGTTCGGGGGAGTTATTATAACGACCTCG ACCCCTGGGGCCAGGGAACCCTGGTCACCGTCTC CTCA |
|---|---|---|---|---|
| | | | SEQ ID NO: 24731 | SEQ ID NO: 28737 |
| | | AA | SYELTQPPSVSVSPGQTASITCSGDKLGDKYAY WYQQKPGQSPVLVIYQDSKRSSGIPERFSGSNSG NTATLTISGTQAMDEADYYCQAWDNSTVVFGG GTKLTVL | QVQLVQSGAEVKKPGASVKVSCKASGYTFTGYYM HWVRQAPGQGLEWMGWINPNSDGTNYAQKFQGR VTMTRDTSISTAYMELNRLRSDDTAVYYCARGFYY GSGSYYNDLDPWGQGTLVTVSS |
| | | | SEQ ID NO: 24732 | SEQ ID NO: 28738 |
| iPS:393172 | 21-225_3B12 | NA | TCCTATGAGCTGAGTCAGCCACCCTCAGTGTC CGTGTCCCCAGGACAGACAGCCAGCATCACCT GCTCTGGAGATAAATTGGGGGGAAAAAATATGCT TGCTGGTATCAGCAGAAGCCAGGCCAGTCCCC TGTGCTGGTCATCTATCAAGATAGGAAGCGGC CCTCAGGGATCCCTGAGCGATTCTCTGGCTCC AACTCTGGGAACAAAGCCACTCTGACCATCAG CGGGACCCAGGCTATGGATGAGGCTGACTATT ACTGTCAGGCGTGGGTCAACAACACTATGATA TTCGGCGGAGGGACCAAGCTGACCGTCCTA | CAGGTGCACCTGGTGGAGTCTGGGGGAGGCGTG GTCCAGCCTGGGAGGTCCCTGAGACTCTCCTGTG CAGCCTCTGGATTCACCTTCAGTTACTATGGCAT GCACTGGGTCCGCCAGGCTCCAGGCAAGGGGCT GGAGTGGGTGGCAGTTATATCATATGATGGAAGT AATAAATACTATGCAGACTCCGTGAAGGGCCGA TTCACCATCTCCAGAGACAATTCCAAGAACACGC TGCATCTGCAAATGAACAGCCTGAGAGCTGAGG ACACGGCTGTGTATTACTGTGCGAGAGATCGGAG GGGGGGCTATGGAGTCCCCGATGCTTTTGATATC TGGGGCCAAGGGACAATGGTCACCGTCTCTTCA |
| | | | SEQ ID NO: 24733 | SEQ ID NO: 28739 |

FIGURE 50

| | | | | |
|---|---|---|---|---|
| | | AA | SYELSQPPSVSVSPGQTASITCSGDKLGEKYACW YQQKPGQSPVLVIYQDRKRPSGIPERFSGSNSGN KATLTISGTQAMDEADYYCQAWVNNTMIFGGG TKLTVL | QVHLVESGGGVVQPGRSLRLSCAASGFTFSYYGM HWVRQAPGKGLEWVAVISYDGSNKYYADSVKGR FTISRDNSKNTLHLQMNSLRAEDTAVYYCARDRRG GYGVPDAFDIWGQGTMVTVSS |
| | | | SEQ ID NO: 24734 | SEQ ID NO: 28740 |
| iPS:393174 | 21-225_15D8 | NA | CTGCCTGTGCTGACTCAGCCCCCGTCTGCATCT GCCTTGCTGGGAGCCTCGATCAAGCTCACCTG CACCCTAAGCAGTGAGCACAGCACCTACACCA TCGAATGGTATCAACAGAGACCAGGGAGGTC CCCCCAGTATATAATGAAGGTTAAGAGTGATG GCAGCCACAGCAAGGGGGACGGGATCCCCGA TCGCTTCATGGGCTCCAGTTCTGGGGCTGACC GCTACATCACCTTCTCCAACCTCCAGTCTGAC GATGAGGAGGAGTATCACTGTGGAGAGAGCC ACACGATCGATGGCCAAGTCGGTGTGGTATTC GGCGGAGGGACCAAGCTGACCGTCCTA | CAGGTGCAGTTGGTGGAGTCTGGGGGAGGCGTG GTCCAGACTGGGAGGTCCCTGAGACTCTCCTGTG CAGCCTCTGGATTCACCTTCAGTGGCTATGGCAT GCACTGGGTCCGCCAGGCTCCAGGCAAGGGGCT GGAGTGGGTGTCAGTTATATCATATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGCGAGAGATCGGGT CTATTGTAGTAGTACCAGCTGCGTCCCTTACTAC GACTACTACGGTATGGACGTCTGGGGCCAAGGG ACCACGGTCACCGTCTCCTCA |
| | | | SEQ ID NO: 24735 | SEQ ID NO: 28741 |
| | | AA | LPVLTQPPSASALLGASIKLTCTLSSEHSTYTIEW YQQRPGRSPQYIMKVKSDGSHSKGDGIPDRFMG SSSGADRYITFSNLQSDDEEEYHCGESHTIDGQV GVVFGGGTKLTVL | QVQLVESGGGVVQTGRSLRLSCAASGFTFSGYGM HWVRQAPGKGLEWVSVISYDGSNKYYADSVKGRF TISRDNSKNTLYLQMNSLRAEDTAVYYCARDRVY CSSTSCVPYYDYYGMDVWGQGTTVTVSS |
| | | | SEQ ID NO: 24736 | SEQ ID NO: 28742 |

FIGURE 50

| iPS:393176 | 21-225_27E7 | NA | TCCTATGAGCTGACTCAGCCACCCTCAGTGTC CGTGTCCCCAGGACAGACAGCCAGCATCACCT GCTCTGGAGATAAATTGGGGAATAAATATGCT TGCTGGTATCAGCAGAAGCCAGGCCAGTCCCC TGTGGAGGTCATCTATCAAGATAGCAAGCGGC CCTTAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGTAGTACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGATTC CTATTGTAGTAGTACCAGCTGCCCTTACTACTAC TACTACGGTATGGACGTCTGGGGCCAAGGGACC ACGGTCACCGTCTCCTCA |
| | | | SEQ ID NO: 24737 | SEQ ID NO: 28743 |
| | | AA | SYELTQPPSVSVSPGQTASITCSGDKLGNKYACW YQQKPGQSPVEVIYQDSKRPLGIPERFSGSNSGN TATLTISGTQAMDEADYYCQAWDSSTVVFGGG TKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCAREDSYC SSTSCPYYYYYGMDVWGQGTTVTVSS |
| | | | SEQ ID NO: 24738 | SEQ ID NO: 28744 |

FIGURE 50

| iPS:393178 | 21-225_34D7 | NA | TCCTATGAGCTGACTCAGCCACCCTCAGTGTC CGTGTCCCCAGGACAGACAGCCAGCATCACCT GCTCTGGAGATAAATTGGGGGAGAAATATGCT TACTGGTATCAGCAGAAGCCAGGCCAGTCCCC TGTGCTGGTCCTCTATCAAGATAGCAAGCGGC CCTCAGGGATCCCTGAGCGATTCTCTGGCTCC AACTCTGGGAACACAGCCACTCTGACCATCAG CGGGACCCAGACTATGGATGAGGCTGACTTTT ACTGTCAGGCGTGGGACAACACCACTGTGGTA TTCGGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGGGGGTAT TACTATGGTTCGGGGGAGTTATTATAACGACCTCG ACCCCTGGGGTCAGGGAACCCTGGTCACCGTCTC CTCA |
|---|---|---|---|---|
| | | | SEQ ID NO: 24739 | SEQ ID NO: 28745 |
| | | AA | SYELTQPPSVSVSPGQTASITCSGDKLGEKYAYW YQQKPGQSPVLVLYQDSKRPSGIPERFSGSNSGN TATLTISGTQTMDEADFYCQAWDNTTVVFGGG TKLTVL | QVQLVQSGAEVKKPGASVKVSCKASGYTFTGYYM HWVRQAPGQGLEWMGWINPNSGGTNYAQKFQGR VTMTRDTSISTAYMELSRLRSDDTAVYYCARGYYY GSGSYYNDLDPWGQGTLVTVSS |
| | | | SEQ ID NO: 24740 | SEQ ID NO: 28746 |
| iPS:393180 | 21-225_4G12 | NA | CAGTCTGTGCTGACTCAGCCACCCTCAGCGTC TGGGACCCCCGGGCAGAGGGTCAACATGTCTT GTTCTGGAACCAACTCCAACATCGGAAGTTAT ACTGTAAACTGGTACCAGCAGCTCCCAGGAAC GGCCCCCAAAACTCCTCATCTATATTAATAATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AATGGTCATGTGGTATTCGGCGGAAGGACCAA GCTGACCGTCCTA | GAGGTGCAGCTGTTGGAGTCTGGGGGAGGCTTG GTACAGCCGGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAACTCTTAGTGGTCGTGGTGGT AGCACATACTACGCAGACTCCGTGAAGGGCCGG TCCACCATCTCCAGAGACAATTCCAAGAACACGC TGTATCTGCAAATGAGCAGCCTGAGAGCCGAGG ACACGGCCGTATATTACTGTGCGAAATGGGGAC GTGGATACAGCTATGAGTACTACTACGGTATGGA CGTCTGGGGCCAAGGGACCACGGTCACCGTCTCC TCA |
| | | | SEQ ID NO: 24741 | SEQ ID NO: 28747 |

FIGURE 50

| | | | | |
|---|---|---|---|---|
| | | AA | QSVLTQPPSASGTPGQRVNMSCSGTNSNIGSYTV NWYQQLPGTAPKLLIYINNQRPSGVPDRFSGSKS GTSASLAISGLQSEDEADYYCAAWDDSLNGHVV FGGRTKLTVL | EVQLLESGGGLVQPGGSLRLSCAASGFTFSSYAMS WVRQAPGKGLEWVSTLSGRGGSTYYADSVKGRST ISRDNSKNTLYLQMSSLRAEDTAVYYCAKWGRGY SYEYYYGMDVWGQGTTVTVSS |
| | | | SEQ ID NO: 24742 | SEQ ID NO: 28748 |
| iPS:393182 | 21-225_4B3 | NA | TCCTATGAGCTGACTCAGCCACCCTCAGTGTC CGTGTCCCCAAGACAGACAGTCAGCATCACCT GCTCTGGAGATAAATTGGGGGATAAATATGCT TGCTGGTATCAGCAGAAGCCAGGCCAGTCCCC TGTGCTGGTCATCTATCAAGATCGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAACACTGTGATA TTCGGCGGAGGGACCAAGCTGACCGTCCTA | CAGGTGCAACTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTCTGCACAGAAGTTTCAGGGCAG GGTCACCATGACCAGGGACACGTCCATCAGCAC AGCCTACATGGAGCTGAGCAGGCTGAGATCTGA CGACACGGCCGTATATTACTGTGCGAGGTCCTAT TACTATGGTTCGGGGGAGTTATTATAACGAGTTTG ACTACTGGGGCCAGGGAACCCTGGTCACCGTCTC CTCA |
| | | | SEQ ID NO: 24743 | SEQ ID NO: 28749 |
| | | AA | SYELTQPPSVSVSPRQTVSITCSGDKLGDKYACW YQQKPGQSPVLVIYQDRKRPSGIPERFSGSNSGN TATLTISGTQAMDEADYYCQAWDNNTVIFGGG TKLTVL | QVQLVQSGAEVKKPGASVKVSCKASGYTFTGYYM HWVRQAPGQGLEWMGWINPNSGGTNSAQKFQGR VTMTRDTSISTAYMELSRLRSDDTAVYYCARSYYY GSGSYYNEFDYWGQGTLVTVSS |
| | | | SEQ ID NO: 24744 | SEQ ID NO: 28750 |

FIGURE 50

| | | | | |
|---|---|---|---|---|
| iPS:393184 | 21-225_15H11 | NA | TCCTATGAGCTGACTCAGCCACCCTCAGTGTC CGTGTCCCCAGGACAGACAGCCAGCATCACCT GCTCTGGAGATAAATTGGGGGAGAAATATGCT TGCTGGTATCAGCAGAAGCCAGGCCAGTCCCC TGTGGTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATAGATGAGGCTGACTATT ACTGTCAGGCGTGGGACAGCAGCACTGCGGTA TTCGGCGGAGGGACCAAGTTGACCGTCCTA | CAGATCACCTTGAAGGAGTCTGGTCTTACGCTGA TGAAACCCACACAGACCCTCACGCTGACCTGCAC CTTCTCTGGGTTCTCACTCAGCACTGGTGGAGTG GGTGTGGGCTGGATCCGTCAGCCCCCAGGAAAG GCCCTGGAGTGGCTTGCACTCATTTATTGGCATG ATGATAAGCGCTACAGTCCATCTCTGAGGAGCAG GCTCACCATCACCAAGGACACCTCCAAAAACCA GGTGGTCCTTACAATGACCAACATGGACCCTGTG GACACAGCCACATATTACTGTGCACGTATAGTAG CAGTTGCCTTTGACTACTGGGGCCAGGGAACCCT GATCACCGTCTCCTCA |
| | | | SEQ ID NO: 24745 | SEQ ID NO: 28751 |
| | | AA | SYELTQPPSVSVSPGQTASITCSGDKLGEKYACW YQQKPGQSPVVVIYQDRKRPSGIPERFSGSNSGN TATLTISGTQAIDEADYYCQAWDSSTAVFGGGT KLTVL | QITLKESGLTLMKPTQTLTLTCTFSGFSLSTGGVGV GWIRQPPGKALEWLALIYWHDDKRYSPSLRSRLTI TKDTSKNQVVLTMTNMDPVDTATYYCARIVAVAF DYWGQGTLITVSS |
| | | | SEQ ID NO: 24746 | SEQ ID NO: 28752 |
| iPS:393186 | 21-225_27D9 | NA | TCCTATGAGCTGACTCAGCCACCCTCAATGTC CGTGTCCCCAGGACAGACAGCCAGCATCACCT GCTCTGGATATAAATTGGGGGATAAATATGCT TGCTGGTTTCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGTCAACAACACTGTATTC GGCGGAGGGACCAAGCTGACCGTCCTA | CAGGTGCAGTTGGTGCAGTCTGGGGGCTGAGGTGA AGAAGCCTGGGGCCTCAGTGAAGGTCTCCTGCA AGGCTTCTGGATACACCTTCACCGGCTACTATAT GCACTGGGTGCGACAGGCCCCTGGACAAGGGCT TGAGTGGATGGGATGGATCAACCCTAACAGTGGT GGCACAAAGTATGCACAGAAGTTTCAGGGCAGG GTCACCATGACCAGGGACACGTCCATCAGCACA GCCTACATGGAGCTGAACAGGCTGAGATCTGAC GACACGGCCGTGTATTACTGTGCGAGAGAGAGG TGTAGTACTACCAGTTGCTATTTAGGAATTACGG GCTACTACGGTATGGACGTCTGGGGCCAAGGGA CCACGGTCACCGTCTCCTCA |

1609

FIGURE 50

| | | | SEQ ID NO: 24747 | SEQ ID NO: 28753 |
|---|---|---|---|---|
| | | AA | SYELTQPPSMSVSPGQTASITCSGYKLGDKYAC WFQQKPGQSPVLVIYQDSKRPSGIPERFSGSNSG NTATLTISGTQAMDEADYYCQAWVNNTVFGGG TKLTVL | QVQLVQSGAEVKKPGASVKVSCKASGYTFTGYYM HWVRQAPGQGLEWMGWINPNSGGTKYAQKFQGR VTMTRDTSISTAYMELNRLRSDDTAVYYCARERCS TTSCYLGITGYYGMDVWGQGTTVTVSS |
| | | | SEQ ID NO: 24748 | SEQ ID NO: 28754 |
| iPS:393188 | 21-225_34B9 | NA | TCCTATGAGCTGACTCAGGCACCCTCAGTGTC CGTGTCCCCAGGACAGACAGCCAGCATCACCT GCTCTGGAGATAAATTGGGGGAGAAATATGTT TCCTGGTATCAGGAGAAGCCAGGCCAGTCCCC TGTGCTGGTCATCTATCAAGATAGTAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTATTC GGCGGAGGGACCAAGCTGACCGTCCTA | CAGATCACCTTGAGGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTTATTGGAATG ATGATAAGCGCTACAGCCCATCTCTGAAGAGCA GACTCACCATCACCAAGGACACCTCCAAAAACC AGGTGGTCCTTACAATGACCAACATGGACCCTGT GGACACAGCCACATATTACTGTGCACACTTAATA GCAGTGACTTTTGACTCCTGGGGCCAGGGATCCC TGGTCACCGTCTCCTCA |
| | | | SEQ ID NO: 24749 | SEQ ID NO: 28755 |
| | | AA | SYELTQAPSVSVSPGQTASITCSGDKLGEKYVSW YQEKPGQSPVLVIYQDSKRPSGIPERFSGSNSGNT ATLTISGTQAMDEADYYCQAWDSSTVFGGGTK LTVL | QITLRESGPTLVKPTQTLTLTCTFSGFSLSTSGVGVG WIRQPPGKALEWLALIYWNDDKRYSPSLKSRLTIT KDTSKNQVVLTMTNMDPVDTATYYCAHLIAVTFD SWGQGSLVTVSS |
| | | | SEQ ID NO: 24750 | SEQ ID NO: 28756 |

FIGURE 50

| | | | | |
|---|---|---|---|---|
| iPS:393192 | 21-225_12B1 | NA | TCCTATGAGCTGACTCAGCCACCCTCAGTGTC CGTGTCCCCAAGACAGACAGTCAGCATCACCT GCTCTGGAGATAAATTGGGGGATAAATATGCT TGCTGGTATCAGCAGAAGCCAGGCCAGTCCCC TGTGCTGGTCATCTATCAAGATCGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAACACTGTGATA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGAATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGATCGGG TAGCAGCAGCTGGTACCCCCTACTACTACTACGG TATGGACGTCTGGGGCCAAGGGACCACGGTCAC CGTCTCCTCA |
| | | | SEQ ID NO: 24751 | SEQ ID NO: 28757 |
| | | AA | SYELTQPPSVSVSPRQTVSITCSGDKLGDKYACW YQQKPGQSPVLVIYQDRKRPSGIPERFSGSNSGN TATLTISGTQAMDEADYYCQAWDNNTVIFGGG TKLTVL | QVQLVESGGGVVQPGRSLRLSCAASGFTFSSYGMH WVRQAPGKGLEWVAVIWNDGSNKYYADSVKGRF TISRDNSKNTLYLQMNSLRAEDTAVYYCARDRVA AAGTPYYYYGMDVWGQGTTVTVSS |
| | | | SEQ ID NO: 24752 | SEQ ID NO: 28758 |
| iPS:393194 | 21-225_16D2 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTTATGTG GTATTCGGCGGAGGGACCAAGCTGACCGTCCT A | GAGGTGCAGCTGGTGGAGTCTGGGGGAGGCTTG GTAAAGCCTGGGGGGTCCCTTAGACTCTCCTGTG CAGCCTCTGGATTCACTTTCAGTAACGCCTGGAT GAACTGGGTCCGCCAGGCTCCAGGGAAGGGGCT GGAGTGGGTTGGCCGTATTAAAAAGCAAAACTGA TGGTGGGACAACAGACTACGCTGCACCCGTGAA AGGCAGATTCACCATCTCAAGAGATGATTCAAA AAACACGCTGTATCTGCAAATGCACAGCCTGAA AACCGAGGACACAGCCGTGTATTACTGTACCACA GATACGGGTCCTATAGCAGCTCGTCTCGCTTACT ACTACTACTACGCTATGGACGTCTGGGGCCAAGG GACCACGGTCACCGTCTCCTCA |

FIGURE 50

| | | | SEQ ID NO: 24753 | SEQ ID NO: 28759 |
|---|---|---|---|---|
| | | AA | SYELTQPPSVSVSPGQTASITCSGDKLGDKYACW YQQKPGQSPVLVIYQDSKRPSGIPERFSGSNSGN TATLTISGTQAMDEADYYCQAWDSSTYVVFGG GTKLTVL | EVQLVESGGGLVKPGGSLRLSCAASGFTFSNAWM NWVRQAPGKGLEWVGRIKSKTDGGTTDYAAPVK GRFTISRDDSKNTLYLQMHSLKTEDTAVYYCTTDT GPIAARLAYYYYYAMDVWGQGTTVTVSS |
| | | | SEQ ID NO: 24754 | SEQ ID NO: 28760 |
| iPS:393196 | 21-225_16G8 | NA | TCCTATGAGCTGAGTCAGCCACCCTCAGTGTC CGTGTCCCCAGGACAGACAGCCAGCATCACCT GCTCTGGAGATAAATTGGGGGAAAAATATGCT TGCTGGTATCAGCAGAAGCCAGGCCAGTCCCC TGTGCTGGTCATCTATCAAGATAGGAAGCGGC CCTCAGGGATCCCTGAGCGATTCTCTGGCTCC AACTCTGGGAACAAAGCCACTCTGACCATCAG CGGGACCCAGGCTATGGATGAGGCTGACTATT ACTGTCAGGCGTGGGTCAATAACACTATGATA TTCGGCGGAGGGACCAAGCTGACCGTCCTA | GAGGTGCAGCTGTTGGAGTCTGGGGGAGGCTTG GTACAGCCTGAGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGGTATTAGTGGTAGTGGTGGT AGCACATACTACGCAGACTCCGTGAAGGGCCGG TTCACCATCTCCAGAGACAATTCCAAGAACACGC TGTGTCTGCACATGAACAGCCTGAGAGCCGAGG ACACGGCCGTATATTACTGTGCGAAAGAATATTG TGGTGGTGACTGCTATTCCCCTTACTACTACTACT ACGGTATGGACGTCTGGGGGCCAAGGGACCACGG TCACCGTCTCCTCA |
| | | | SEQ ID NO: 24755 | SEQ ID NO: 28761 |
| | | AA | SYELSQPPSVSVSPGQTASITCSGDKLGEKYACW YQQKPGQSPVLVIYQDRKRPSGIPERFSGSNSGN KATLTISGTQAMDEADYYCQAWVNNTMIFGGG TKLTVL | EVQLLESGGGLVQPEGSLRLSCAASGFTFSSYAMS WVRQAPGKGLEWVSGISGSGGSTYYADSVKGRFTI SRDNSKNTLCLHMNSLRAEDTAVYYCAKEYCGGD CYSPYYYYYGMDVWGQGTTVTVSS |
| | | | SEQ ID NO: 24756 | SEQ ID NO: 28762 |

FIGURE 50

| iPS:393198 | 21-225_28A11 | NA | TCCTATGAGTTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGTAGCACTTATGTG GTATTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGGCTATGGCCT GCACTGGGTCCGCCAGGCTCCAGGCAAGGGGCT GGAGTGGGTGGCACTTATATGGTATGATGGAAAT AATACATACTATGCAGACTCCGTGAAGGGCCGAT TCACCATCTCCAGAGACAATTCCAAGAACACGCT GTATCTGCAAATGAACAGCCTGAGAGCCGAAGA CACGGCTGTGTATTACTGTGCGAGAGATAGGGTA TATTGTAGTAGTACCAGCTGCTCCCCTTACTACT ACTACTACGGTATGGACGTCTGGGGCCAAGGGA CCACGGTCACCGTCTCCTCA |
|---|---|---|---|---|
| | | | SEQ ID NO: 24757 | SEQ ID NO: 28763 |
| | | AA | SYELTQPPSVSVSPGQTASITCSGDKLGDKYACW YQQKPGQSPVLVIYQDSKRPSGIPERFSGSNSGN TATLTISGTQAMDEADYYCQAWDSSTYVVFGG GTKLTVL | QVQLVESGGGVVQPGRSLRLSCAASGFTFSGYGLH WVRQAPGKGLEWVALIWYDGNNTYYADSVKGRF TISRDNSKNTLYLQMNSLRAEDTAVYYCARDRVY CSSTSCSPYYYYYGMDVWGQGTTVTVSS |
| | | | SEQ ID NO: 24758 | SEQ ID NO: 28764 |

FIGURE 50

| iPS:393200 | 21-225_35E1 | NA | TCCTATGAGCTGACTCAGCCACCCTCAGTGTC CGTGTCCCCAGGACAGACAGCCAGCATCACCT GCTCTGGAGATAAATTGGGGGAAAAATATGCT TACTGGTTTCAGCAGAAGCCAGGCCAGTCCCC TGTGATAGTCATCTATCAAGATAGGAAGCGGC CCTCAGGGATCCCTGAGCGATTCTCTGGCTCC AACTCTGGGAACACAGCCACTCTGACCATCAG CGGGACGCAGGCTATGGATGAGGCTGACTATT ACTGTCAGGCGTGGGACAACAGCACTGCGGTA TTCGGCGGAGGGACCAAGCTGACCGTCCTA | CAGGTGAAGCTGGTGCAGTCTGGGGCTGAGGTG AAGAAGCCTGGGGCCTCAGTGAAGGTCTCCTGC AAGGCTTCAGGATACACCTTCACCGGCTACTATA TGCACTGGGTGCGACAGGCCCCTGGACAAGGGC TTGAGTGGATGGGATGGATCAACCCTAAAAGTG GTGGCACAAATTATGCACAGAAGTTTCAGGGCA GGGTCACCATGACCAGGGACACGTCCATCAGCA CAGTCTACATGGAGCCGAGCAGGCTGAGATCTG ACGACACGGCCGTGTATTACTGTGCGAGAGTGTA TTACCATGGTTCGGGGAGTTATTATAACGAGTTT GATTATTGGGGCCAGGGAACCCTGGTCACCGTCT CCTCA |
|---|---|---|---|---|
| | | | SEQ ID NO: 24759 | SEQ ID NO: 28765 |
| | | AA | SYELTQPPSVSVSPGQTASITCSGDKLGEKYAYW FQQKPGQSPVIVIYQDRKRPSGIPERFSGSNSGNT ATLTISGTQAMDEADYYCQAWDNSTAVFGGGT KLTVL | QVKLVQSGAEVKKPGASVKVSCKASGYTFTGYYM HWVRQAPGQGLEWMGWINPKSGGTNYAQKFQGR VTMTRDTSISTVYMEPSRLRSDDTAVYYCARVYYH GSGSYYNEFDYWGQGTLVTVSS |
| | | | SEQ ID NO: 24760 | SEQ ID NO: 28766 |
| iPS:393202 | 21-225_6B4 | NA | TCCTATGAGCTGACTCAGCCACCCTCAGTGTC CGTGTCCCCAAGACAGACAGTCAGCATCACCT GCTCTGGAGATAAATTGGGGGATAAATATGCT TGCTGGTATCAGCAGAAGCCAGGCCAGTCCCC TGTGCTGGTCATCTATCAAGATCGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAACACTGTGATA TTCGGCGGAGGGACCAAGCTGACCGTCCTA | GAGGTGCAGCTGTTGGAGTCTGGGGGAGGCTTG GTACAGCCTGAGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTAGT AGCACATACTACGCAGACTCCGTGAAGGGCCGG TTCACCATCTCCAGAGACAATTCCAAGAACACGC TGTGTCTGCAAATGAACAGCCTGAGAGCCGAGG ACACGGCCGTATATTACTGTGCGAAAGAATATTG TGGTGGTGACTGCTATTCCCCTTACTACTACTACT ACGGTATGGACGTCTGGGGCCAAGGGACCACGG TCACCGTCTCCTCA |
| | | | SEQ ID NO: 24761 | SEQ ID NO: 28767 |

FIGURE 50

| | | | | |
|---|---|---|---|---|
| | | AA | SYELTQPPSVSVSPRQTVSITCSGDKLGDKYACW YQQKPGQSPVLVIYQDRKRPSGIPERFSGSNSGN TATLTISGTQAMDEADYYCQAWDNNTVIFGGG TKLTVL | EVQLLESGGGLVQPEGSLRLSCAASGFTFSSYAMS WVRQAPGKGLEWVSAISGSGSSTYYADSVKGRFTI SRDNSKNTLCLQMNSLRAEDTAVYYCAKEYCGGD CYSPYYYYYGMDVWGQGTTVTVSS |
| | | | SEQ ID NO: 24762 | SEQ ID NO: 28768 |
| iPS:393204 | 21-225_8C12 | NA | TCCTATGAGCTGACTCAGCCACACTCAGTGTC AGTGGCCACAGCACAGATGGCCAGGATCACCT GTGGGGGAAACAACATTGGAAGTAAAGCTGT GCACTGGTACCAGCAAAAGCCAGGCCAGGAC CCTGTGCTGGTCATCTATAGCGATAGCAACCG GCCCTCAGGGATCCCTGAGCGATTCTCTGGCT CCAACCCAGGGAACACCGCCACCCTAACCATC AGCAGGATCGAGGCTGGGGATGAGGCTGACT ATTACTGTCAGGTGTGGGACAGTAGTAGTGAT CATGTGGTATTCGGCGGAGGGACCAAGCTGAC CGTCCTA | CAGGTGCAGTTGGTGGAGTCTGGGGGAGGCGTG GTCCAGCCTGGGAGGTCCCTGAGACTCTCCTGTG CAGCGTCTGGATTCACCTTCGGTAGCTATGGCAT GCACTGGGTCCGCCAGGCTCCAGGCAAGGGGCT GGAGTGGGTGGCACTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGT ATCTTGTAGTAGTTCCAGCTGCTATCCTTACTACT ACTACTACGGTATGGACGTCTGGGGCCAAGGGA CCACGGTCACCGTCTCCTCA |
| | | | SEQ ID NO: 24763 | SEQ ID NO: 28769 |
| | | AA | SYELTQPHSVSVATAQMARITCGGNNIGSKAVH WYQQKPGQDPVLVIYSDSNRPSGIPERFSGSNPG NTATLTISRIEAGDEADYYCQVWDSSSDHVVFG GGTKLTVL | QVQLVESGGGVVQPGRSLRLSCAASGFTFGSYGM HWVRQAPGKGLEWVALIWYDGSNKYYADSVKGR FTISRDNSKNTLYLQMNSLRAEDTAVYYCARDRVS CSSSSCYPYYYYYGMDVWGQGTTVTVSS |
| | | | SEQ ID NO: 24764 | SEQ ID NO: 28770 |

FIGURE 50

| iPS:393206 | 21-225_13F6 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGGCAACAGCACTGCTGTG GTATTCGGCGGAGGGACCAAGCTGACCGTCCT G<br><br>SEQ ID NO: 24765 | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCGCAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTTCTGTGCGAGGTCGTTT TACTATGGTTCGGGGACTTATTATAACGAGTTTG ACTACTGGGGCCAGGGAACCCTGGTCACCGTCTC CTCA<br><br>SEQ ID NO: 28771 |
|---|---|---|---|---|
| | | AA | SYELTQPPSVSVSPGQTASITCSGDKLGDKYACW YQQKPGQSPVLVIYQDSKRPSGIPERFSGSNSGN TATLTISGTQAMDEADYYCQAWGNSTAVVFGG GTKLTVL<br><br>SEQ ID NO: 24766 | QVQLVQSGAEVKKPGASVKVSCKASGYTFTGYYM HWVRQAPGQGLEWMGWINPNSGGANYAQKFQGR VTMTRDTSISTAYMELSRLRSDDTAVYFCARSFYY GSGTYYNEFDYWGQGTLVTVSS<br><br>SEQ ID NO: 28772 |
| iPS:393208 | 21-225_16F3 | NA | TCCTATGTGCTGACTCAGCCACCCTCGGTGTC AGTGGCCCCAGGACAGACGGCCAGGATTACCT GTGGGGGAAACAACATTGGAAGTAAAAGTGT GCACTGGTACCAGCAGAAGCCAGGCCAGGCC CCTGTGCTGGTCGTCTATGATGATACCGACCG GCCCTCAGGGATCCCTGAGCGATTCTCTGGCT CCAACTCTGGGAACACGGCCACCCTGACCATC AGCAGGGTCGAAGCCGGGGATGAGGCCGACT ATTACTGTCAGGTGTGGGATAGTAGCAGTGAT CATGTGGTATTCGGCGGAGGGACCAAGCTGAC CGTCCTA<br><br>SEQ ID NO: 24767 | CAGGTGCAGCTGGTGCAGTCTGGGGCTGAGGTG AAGAAGCCTGGGGCCTCAGTGAAGGTCTCCTGC AAGGCTTCTGGATACACCTTCACCGGGCACTATA TGCACTGGGTGCGACAGGCCCCTGGACAAGGAC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGGTCGTAT TACTATGGTTCGGGGACTTATTATAACGAGTTTG ACTACTGGGGCCAGGGAACCCTGGTCACCGTCTC CTCA<br><br>SEQ ID NO: 28773 |

FIGURE 50

| | | | | |
|---|---|---|---|---|
| | | AA | SYVLTQPPSVSVAPGQTARITCGGNNIGSKSVHW YQQKPGQAPVLVVYDDTDRPSGIPERFSGSNSG NTATLTISRVEAGDEADYYCQVWDSSSDHVVFG GGTKLTVL | QVQLVQSGAEVKKPGASVKVSCKASGYTFTGHYM HWVRQAPGQGLEWMGWINPNSGGTNYAQKFQGR VTMTRDTSISTAYMELSRLRSDDTAVYYCARSYYY GSGTYYNEFDYWGQGTLVTVSS |
| | | | SEQ ID NO: 24768 | SEQ ID NO: 28774 |
| iPS:393210 | 21-225_17D3 | NA | TCCTATGAGCTGACGCAGTCACCCTCAGTGTC CGTGTCCCCAGGACAGACAGCCAGCATCACCT GCTCTGGAGATAAATTGGGGGATAAATATGTT TACTGGTATCAGCAGAAGCCAGGCCAGTCCCC TGTGGTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCATCACTGCAGTA TTCGGCGGAGGGACCAAGCTGACCGTCCGA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCGGA CGACACGGCCGTGTATTACTGTGCGAGAGCGAAT TACTATGGTTCGGGGGAGTTATTATAACGACTTTG ACTACTGGGGCCAGGGAACCCTGGTCACCGTCTC CTCA |
| | | | SEQ ID NO: 24769 | SEQ ID NO: 28775 |
| | | AA | SYELTQSPSVSVSPGQTASITCSGDKLGDKYVY WYQQKPGQSPVVVIYQDRKRPSGIPERFSGSNSG NTATLTISGTQAMDEADYYCQAWDSITAVFGGG TKLTVR | QVQLVQSGAEVKKPGASVKVSCKASGYTFTGYYM HWVRQAPGQGLEWMGWINPNSGGTNYAQKFQGR VTMTRDTSISTAYMELSRLRSDDTAVYYCARANYY GSGSYYNDFDYWGQGTLVTVSS |
| | | | SEQ ID NO: 24770 | SEQ ID NO: 28776 |

FIGURE 50

| iPS:393212 | 21-225_30H6 | NA | TCCTATGAGCTGACTCAGCCACCCTCAGTGTC CGTGTCCCCAGGACAGACAGCCAGCATCACCT GCTCTGGAGATAAATTGGGTAATAAATATGCT TGCTGGTATCAGCAGAAGCCAGGCCAGTCCCC TGTGTTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTTTTC GGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGATTCTCACTCAGCACTGGTGGAGTG GGTGTGGGCTGGATCCGTCAGCCCCCAGGAAAG GCCCTGGAGTGGCTTGCACTCATTTATTGGCATG ATGATAAGCGCTACAGTCCCTCTCTGAAGAGCAG GCTCGCCATCACCAAGGACACCTCCAAAAACCA GGTGGTCCTTACAATTACCAACATGGACCCTGTG GACACAGCCACATATTACTGTGCTCACTTAATAG CAGTGGCTTTTGACTATTGGGGCCAGGGAACCCT GGTCACCGTCTCCTCA |
| | | | SEQ ID NO: 24771 | SEQ ID NO: 28777 |
| | | AA | SYELTQPPSVSVSPGQTASITCSGDKLGNKYACW YQQKPGQSPVLVIYQDSKRPSGIPERFSGSNSGN TATLTISGTQAMDEADYYCQAWDSSTVFGGGT KLTVL | QITLKESGPTLVKPTQTLTLTCTFSGFSLSTGGVGVG WIRQPPGKALEWLALIYWHDDKRYSPSLKSRLAIT KDTSKNQVVLTITNMDPVDTATYYCAHLIAVAFDY WGQGTLVTVSS |
| | | | SEQ ID NO: 24772 | SEQ ID NO: 28778 |
| iPS:393214 | 21-225_33A1 | NA | TCCTATGAGCTGACTCAGCCACCCTCAGTGTC CGTGTCCCCAGGACAGACAGCCAGCATCACCT GCTCTGGAGATAAATTGGGGGATAAATTTGTT TATTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCACCACCGTGGTA TTCGGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCAGCGGCTACTATA TGCACTGGGTGCGACAGGCCCCTGGACAAGGAC TTGAGTGGATGGGATGGATCAACCCTAACAATGG TGGCACACACTATGCACAGAAGTTTCAGGGCAG GGTCACCATGACCAGGGACACGTCCATCCGCAC AGCCTCCATGGAGCTGAGCAGGCTGAGATCTGA CGACACGGCCGTGTATTTCTGTGCGAGGGGATAT TATTATGCTTCGGGGAGTTATTATAACGACCTCG ACCCCTGGGGCCAGGGAACCCTGGTCACCGTCTC CTCA |
| | | | SEQ ID NO: 24773 | SEQ ID NO: 28779 |

FIGURE 50

| | | | | |
|---|---|---|---|---|
| | | AA | SYELTQPPSVSVSPGQTASITCSGDKLGDKFVYW YQQKPGQSPVLVIYQDSKRPSGIPERFSGSNSGN TATLTISGTQAMDEADYYCQAWDSTTVVFGGG TKLTVL | QVQLVQSGAEVKKPGASVKVSCKASGYTFSGYYM HWVRQAPGQGLEWMGWINPNNGGTHYAQKFQGR VTMTRDTSIRTASMELSRLRSDDTAVYFCARGYYY ASGSYYNDLDPWGQGTLVTVSS |
| | | | SEQ ID NO: 24774 | SEQ ID NO: 28780 |
| iPS:393218 | 21-225_14G3 | NA | TCCTATGAGCTGACTCAGCCACCCTCAGTGTC CGTGTCCCCAGGACAGACAGCCAGCATCACCT GCTCTGGAGATAAATTGGGGGGATAAATATGTT TGCTGGTATCAGCAGAAGCCAGGCCAGTCCCC TGTGCTGGTCATCTATCAAGATCGCAAGCGGC CCTCAGGGATCCCTGAGCGATTCTCTGGCTCC AACTCTGGGAACACAGCCACTCTGACCATCAG CGGGACCCAGGCTATGGATGAGGCTGATTATT ACTGTCAGGCGTGGGGCAACAGCACTGCTGTG GTATTCGGCGGAGGGACCAAGCTGACCGTCCT G | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGTACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTTTATTACTGTGCGAGGTCGTAT TTTTATGGTTCGGGGGAGTTATTATAACGAGTTTG ACTACTGGGGCCAGGGAACCCTGGTCACCGTCTC CTCA |
| | | | SEQ ID NO: 24775 | SEQ ID NO: 28781 |
| | | AA | SYELTQPPSVSVSPGQTASITCSGDKLGDKYVCW YQQKPGQSPVLVIYQDRKRPSGIPERFSGSNSGN TATLTISGTQAMDEADYYCQAWGNSTAVVFGG GTKLTVL | QVQLVQSGAEVKKPGASVKVSCKASGYTFTGYYM YWVRQAPGQGLEWMGWINPNSGGTNYAQKFQGR VTMTRDTSISTAYMELSRLRSDDTAVYYCARSYFY GSGSYYNEFDYWGQGTLVTVSS |
| | | | SEQ ID NO: 24776 | SEQ ID NO: 28782 |

FIGURE 50

| iPS:393222 | 21-225_17F5 | NA | TCCTATGAGCTGACTCAGCCACCCTCAGTGTC CGTGTCCCCAGGACAGACAGCCAGCATCACCT GCTCTGGAGATAAATTGGGGGAAAAAATATGCT TGCTGGTATCAGCAGAAGCCAGGCCAGTCCCC TGTGCTGGTCATCTATCAAGATAGAAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACGGTATTC GGCGGAGGGACCAAACTGACCGTCCTA | CAGATCACCTTGAAGGAGTCTGGTCCTTCGCTGG TGAAGCCCACACAGACCCTCACGCTGACCTGCAC CTTCTCTGGATTCTCACTCAGCACTAGTGGAGTG GGTGTGGGCTGGATCCGTCAGCCCCCCGGAAAG GCCCTGGAGTGGCTTGCACTCATTTATTGGGATG ATGATAAGCGCTACAGCCCATCTCTGAAGAGCA GGCTCACCATCACCAAGGACACCTCCAAAAACC AGGTGGTCCTGACAATGACCAACATGGACCCTGT GGACACAGCCACATATTCCTGTGCACACATTATA GCAGTGGCTTTTGACTACTGGGGCCAGGGAACCC TGGTCACCGTCTCCTCA |
|---|---|---|---|---|
| | | | SEQ ID NO: 24777 | SEQ ID NO: 28783 |
| | | AA | SYELTQPPSVSVSPGQTASITCSGDKLGEKYACW YQQKPGQSPVLVIYQDRKRPSGIPERFSGSNSGN TATLTISGTQAMDEADYYCQAWDSSTVFGGGT KLTVL | QITLKESGPSLVKPTQTLTLTCTFSGFSLSTSGVGVG WIRQPPGKALEWLALIYWDDDKRYSPSLKSRLTIT KDTSKNQVVLTMTNMDPVDTATYSCAHIIAVAFD YWGQGTLVTVSS |
| | | | SEQ ID NO: 24778 | SEQ ID NO: 28784 |
| iPS:393224 | 21-225_31C2 | NA | TCCTATGAGCTGACTCAGCCACCCTCAGTGTC CGTGTCCCCAGGGCAGACAGCCAGCATCACCT GCTCTGGAGATAAATTGGGAAATAAATATGCT TGCTGGTATCAGCAGAAGCCAGGCCAGTCCCC TGTGCTGGTCATCTATCAAGATTCCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTATTC GGCGGAGGGACCAAACTGACCGTCCTA | CAGATCACCTTGAAGGAGTCTGGTCCTACGCTGG TGAAACCCACACAGACCCTCACGCTGACCTGCAC CTTCTCTGGGTTCTCCCTCAACACTGGTGGAGTG GGTGTGGGCTGGATCCGTCAGCCCCCCAGGAAAG GCCCTGGAGTGGCTTGCACTCATTTATTGGAATG ATGATGAGCGCTACAGCCCATCTCTGAAGAGCA GGCTCACCATCACCAAGGACACCTCCAAAAACC AGGTGGTCCTTACAATGACCAACATGGACCCTTT GGACACAGCCTCATATTACTGTGCACACTTAATA GCAGTTTCCTTTGACTACTGGGGCCAGGGAGCCC TGGTCACCGTCTCCTCA |
| | | | SEQ ID NO: 24779 | SEQ ID NO: 28785 |

FIGURE 50

| | | | | |
|---|---|---|---|---|
| | | AA | SYELTQPPSVSVSPGQTASITCSGDKLGNKYACW YQQKPGQSPVLVIYQDSKRPSGIPERFSGSNSGN TATLTISGTQAMDEADYYCQAWDSSTVFGGGT KLTVL | QITLKESGPTLVKPTQTLTLTCTFSGFSLNTGGVGV GWIRQPPGKALEWLALIYWNDDERYSPSLKSRLTIT KDTSKNQVVLTMTNMDPLDTASYYCAHLIAVSFD YWGQGALVTVSS |
| | | | SEQ ID NO: 24780 | SEQ ID NO: 28786 |
| iPS:393226 | 21-225_33E6 | NA | TCCTATGAGCTGACTCAGCCACCCTCAGTGTC CGTGTCCCCAGGACAGACAGCCAGCATCACCT GCTCTGGAGATAAAATTGGGGGATAAATATGCT TACTGGTTTCAGCAGAAGCCAGGCCAGTCCCC TGTGATAGTCATCTATCAAGATAGGAAGCGGC CCTCAGGGATCCCTGAGCGATTCTCTGGCTCC AACTCTGGGAACACAGCCACTCTGACCATCAG CGGGACGCAGGCTATGGATGAGGCTGACTATT ACTGTCAGGCGTGGGACAACAGCACTGCGGTA TTCGGCGGAGGGACCAAGCTGACCGTCCTA | CAGGTGAAGCTGGTGCAGTCTGGGGGCTGAGGTG AAGAAGCCTGGGGCCTCAGTGAAGGTCTCCTGC AAGGCTTCAGGATACACCTTCACCGGCTACTATA TGCACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGTGTAT TACTATGGTTCGGGGGAGTTATTATAACGAGTTTG ACTACTGGGGCCAGGGAACCCTGGTCACCGTCTC CTCA |
| | | | SEQ ID NO: 24781 | SEQ ID NO: 28787 |
| | | AA | SYELTQPPSVSVSPGQTASITCSGDKLGDKYAY WFQQKPGQSPVIVIYQDRKRPSGIPERFSGSNSG NTATLTISGTQAMDEADYYCQAWDNSTAVFGG GTKLTVL | QVKLVQSGAEVKKPGASVKVSCKASGYTFTGYYM HWVRQAPGQGLEWMGWINPNSGGTNYAQKFQGR VTMTRDTSISTAYMELSRLRSDDTAVYYCARVYYY GSGSYYNEFDYWGQGTLVTVSS |
| | | | SEQ ID NO: 24782 | SEQ ID NO: 28788 |

FIGURE 50

| iPS:393230 | 21-225_9G9 | NA | CAGTCTGTGCTGACTCAGCCACCCTCAGCGTC TGGGACCCCCGGGCAGAGGGTCAACATGTCTT GTTCTGGAACCAACTCCAACATCGGAAGTTAT ACTGTAAACTGGTACCAGCAGCTCCCAGGAAC GGCCCCCAAACTCCTCATCTATATTAATAATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AATGGTCATGTGGTATTCGGCGGAAGGACCAA GCTGACCGTCCTA<br><br>SEQ ID NO: 24783 | CAGGTGCAGCTGGTGCAGTCTGGGGGCTGAGGTG AAGAAGCCTGGGGGCCTCAGTGAAGGTCTCCTGC AAGGCTTCTGGATACACCTTCACCGGCTACTATA TACACTGGGTGCGACAGGCCCCTGGACAAGGAC TTGAGTGGATGGGATGGATCAACCCTAACAGTGG TGGCACAGACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGGTCGTAT TACTATGGTTCGGGGGACTTATTATAACGAGTTTG ACTACTGGGGCCAGGGAACCCTGGTCACCGTCTC CTCA<br><br>SEQ ID NO: 28789 |
| | | AA | QSVLTQPPSASGTPGQRVNMSCSGTNSNIGSYTV NWYQQLPGTAPKLLIYINNQRPSGVPDRFSGSKS GTSASLAISGLQSEDEADYYCAAWDDSLNGHVV FGGRTKLTVL<br><br>SEQ ID NO: 24784 | QVQLVQSGAEVKKPGASVKVSCKASGYTFTGYYIH WVRQAPGQGLEWMGWINPNSGGTDYAQKFQGRV TMTRDTSISTAYMELSRLRSDDTAVYYCARSYYYG SGTYYNEFDYWGQGTLVTVSS<br><br>SEQ ID NO: 28790 |
| iPS:393232 | 21-225_17F12 | NA | CAGTCTGCCCTGACTCAGCCTGCCTCCGTGTCT GGGTCTCCTGGACAGTCGATCACCATCTCCTG CACTGGAGCCAGCAGTGACGTTGGTGATTATA ACTCTGTCTCCTGGTACCAACAGCACCCAGGC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAGCTCATATACAAGCAGCAT CACTGTGGTATTCGGCGGAGGGACCAAACTGA CCGTCCTA<br><br>SEQ ID NO: 24785 | GAGGTGCAGTTGTTGGAGTCTGGGGGGAGGCTTGG TACAGCCTGGGGGGTCCCTGAGACTCTCCTGTGC AGCCTCTGGATTCACCTTTAGCAGCTATGCCATG AGCTGGGTCCGCCAGGCTCCAGGGAAGGGGCTG GAGTGGGTCTCAGCTATTAGTGGTGGTGGTGGTA GCACATACTACGCAGACTCCGTGAAGGGCCGGG TCACCATCTCCAGAGACAATTCCAAGAACACGCT GTATCTGCAAATGAACAGCCTGAGAGCCGAGGA CACGGCCGTATATTACTGTGCGAAATGGGGACGT GGATACAACTATGAGTACTACTACGGTATGGACG TCTGGGGCCAAGGGACCACGGTCACCGTCTCCTC A<br><br>SEQ ID NO: 28791 |

FIGURE 50

| | | | | |
|---|---|---|---|---|
| | | AA | QSALTQPASVSGSPGQSITISCTGASSDVGDYNS VSWYQQHPGKAPKLMIYEVSNRPSGVSNRFSGS KSGNTASLTISGLQAEDEADYYCSSYTSSITVVF GGGTKLTVL | EVQLLESGGGLVQPGGSLRLSCAASGFTFSSYAMS WVRQAPGKGLEWVSAISGGGGSTYYADSVKGRVT ISRDNSKNTLYLQMNSLRAEDTAVYYCAKWGRGY NYEYYYGMDVWGQGTTVTVSS |
| | | | SEQ ID NO: 24786 | SEQ ID NO: 28792 |
| iPS:393234 | 21-225_26C10 | NA | TCCTATGAAGTGACTCAGCCACCCTCAATGTC CGTGTCCCCAGGACAGACAGCCAGCATCACCT GCTCTGGAGATAAATTGGGGGATAAATATGTT TGCTGGTTTCAGCAGAAGCCAGGCCAGTCCCC TGTGTTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGTCAACAACACTGTATTC GGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATG TGCACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGAGAG GTGTAGTACTACCAGCTGCTATTTAGGAATTACG GGCTACTACGGTATGGACGTCTGGGGGCCAAGGG ACCACGGTCACCGTCTCCTCA |
| | | | SEQ ID NO: 24787 | SEQ ID NO: 28793 |
| | | AA | SYEVTQPPSMSVSPGQTASITCSGDKLGDKYVC WFQQKPGQSPVLVIYQDSKRPSGIPERFSGSNSG NTATLTISGTQAMDEADYYCQAWVNNTVFGGG TKLTVL | QVQLVQSGAEVKKPGASVKVSCKASGYTFTGYYV HWVRQAPGQGLEWMGWINPNSGGTNYAQKFQGR VTMTRDTSISTAYMELSRLRSDDTAVYYCARERCS TTSCYLGITGYYGMDVWGQGTTVTVSS |
| | | | SEQ ID NO: 24788 | SEQ ID NO: 28794 |

FIGURE 50

| | | | | |
|---|---|---|---|---|
| iPS:393345 | 21-225_5G7 | NA | TCCTATGAGCTGACTCAGCCACCCTCAGTGTC CGTGTCCCCAGGACAGACAGCCAGCATCACCT GCTCTGGAGATAAATTGGGGAATAAATATGCT TGGTGGTATCAGCAGAAGCCAGGCCAGTCCCC TGTGCTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAGCACTGTGGTT TTCGGCGGAGGGACCAAACTGACCGTCCTA | GAGGTGCAGCTGTTGGAGTCTGGGGGAGGCTTG GTACAGCCTGAGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AGCACATACTACGCAGACTCCGTGAAGGGCCGG TTCACCATCTCCAGAGACAATTCCAAGAACACGC TGTGTCTGCAAATGAACAGCCTGAGAGCCGAGG ACACGGCCGTATATTACTGTGCGAAAGAATATTG TGGTGGTGACTGCTATTCCCCTTACTACTACTACT ACGGTATGGACGTCTGGGGCCAAGGGACCACGG TCACCGTCTCCTCA |
| | | | SEQ ID NO: 24789 | SEQ ID NO: 28795 |
| | | AA | SYELTQPPSVSVSPGQTASITCSGDKLGNKYAW WYQQKPGQSPVLVIYQDRKRPSGIPERFSGSNSG NTATLTISGTQAMDEADYYCQAWDNSTVVFGG GTKLTVL | EVQLLESGGGLVQPEGSLRLSCAASGFTFSSYAMS WVRQAPGKGLEWVSAISGSGGSTYYADSVKGRFTI SRDNSKNTLCLQMNSLRAEDTAVYYCAKEYCGGD CYSPYYYYYGMDVWGQGTTVTVSS |
| | | | SEQ ID NO: 24790 | SEQ ID NO: 28796 |
| iPS:393368 | 21-225_29H8 | NA | GACATCGTGATGACCCAGTCTCCAGACTCCCT GGCTGTGTCTCTGGGCGAGAGGGCCACCATCA ACTGCAGGTCCAGCCAGACTATTTTACACAGC TCCAACAATTACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATTGTACTCCTCCGACGTTCGGCCAAGG GACCAAGGTGGAAATCAAA | CAGGTGCAGCTGGTGCAGTCTGGGGGCTGAGGTG CAGAAGCCTGGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGCTGGATGAACCCTAACAGTGGT AACACAGGCTATGCACAGAGGTTCCAGGGCAGA GTCACCATGACCAGGAACACCTCCATAAGCGCA GCCTACATGGAGCTGAGCAGCCTGAGATCTGAG GACACGGCCGTATATTACTGTGCGAGTAGCAGTG GCTGGTACTACTTTGACTACTGGGGCCAGGGAAC CCTGGTCACCGTCTCCTCA |
| | | | SEQ ID NO: 24791 | SEQ ID NO: 28797 |

FIGURE 50

| | | | | |
|---|---|---|---|---|
| | | AA | DIVMTQSPDSLAVSLGERATINCRSSQTILHSSNN YNYLAWYQQKPGQPPKLLIYWASTRESGVPDRF SGSGSGTDFTLTISSLQAEDVAVYYCQQYYCTPP TFGQGTKVEIK | QVQLVQSGAEVQKPGASVKVSCKASGYTFTNYDIN WVRQATGQGLEWMGWMNPNSGNTGYAQRFQGR VTMTRNTSISAAYMELSSLRSEDTAVYYCASSSGW YYFDYWGQGTLVTVSS |
| | | | SEQ ID NO: 24792 | SEQ ID NO: 28798 |
| iPS:393565 | 21-225_34B11 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGGTGGTCATCTATCAAGATATGAAGCGGC CCTCAGGGATCCCTGAGCGATTCTCTGGCTCC AACTCTGGGAACACAGCCACTCTGACCATCAG CGGGACGCAGGCTATGGATGAGGCTGACTATT ACTGTCAGGCGTGGGACAACAGCACTGCGGTA TTCGGCGGAGGGACCAAGCTGACCGTCCTA | CAGGTGAAACTGGTGCAGTCTGGGGGCTGAGGTG AAGAAGCCTGGGGCCTCAGTGAAGGTCTCCTGC AAGGCTTCAGGATACACCTTCACCGGCTACTATA TGCACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGTGTAT TTCTATGGTTCGGGGAGTTATTATAACGAGTTTG ACTACTGGGGCCAGGGAACCCTGGTCACCGTCTC CTCA |
| | | | SEQ ID NO: 24793 | SEQ ID NO: 28799 |
| | | AA | SYELTQPPSVSVSPGQTASITCSGDKLGDKYACW YQQKPGQSPVVVIYQDMKRPSGIPERFSGSNSGN TATLTISGTQAMDEADYYCQAWDNSTAVFGGG TKLTVL | QVKLVQSGAEVKKPGASVKVSCKASGYTFTGYYM HWVRQAPGQGLEWMGWINPNSGGTNYAQKFQGR VTMTRDTSISTAYMELSRLRSDDTAVYYCARVYFY GSGSYYNEFDYWGQGTLVTVSS |
| | | | SEQ ID NO: 24794 | SEQ ID NO: 28800 |

FIGURE 50

| iPS:393802 | 21-225_3D12 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAGCAGC TACTTAGCCTGGTACCAGCAGAAACCTGGCCA GGCTCCCAGGCTCCTCATCTATGGTACATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAGTTCACGC AGTTTTGGCCAGGGGACCAAGCTGGAGATCAA A | GAGGTGCAGCTGGTGGAGTCTGGGGGAGGCTTG GTAAAGCCTGGGGGGTCCCTTAGACTCTCCTGTG CAGCCTCTGGAGTCACTTTCAGTACCGCCTGGAT GAACTGGGTCCGCCAGGCTCCAGGGAAGGGGCT GGAGTGGGTTGGCCGGATTAAAAAACAAAATTGA TGGTGGGACAACAGACTACGTTGCACCCGTGAA AGGCAGATTCACCATCTCAAGAGATGATTCAAA AAACACGCTGTATCTGCAAATGAACAGCCTGAA AACCGAGGACACAGCCGTATATTACTGTACCACA GAAGGCTGGAACACGGACTACTGGGGCCAGGGA ACCCTGGTCACCGTCTCCTCA |
|---|---|---|---|---|
| | | | SEQ ID NO: 24795 | SEQ ID NO: 28801 |
| | | AA | EIVLTQSPGTLSLSPGERATLSCRASQSVSSSYLA WYQQKPGQAPRLLIYGTSSRATGIPDRFSGSGSG TDFTLTISRLEPEDFAVYYCQQYGSSRSFGQGTK LEIK | EVQLVESGGGLVKPGGSLRLSCAASGVTFSTAWM NWVRQAPGKGLEWVGRIKNKIDGGTTDYVAPVKG RFTISRDDSKNTLYLQMNSLKTEDTAVYYCTTEGW NTDYWGQGTLVTVSS |
| | | | SEQ ID NO: 24796 | SEQ ID NO: 28802 |
| iPS:393804 | 21-225_5H7 | NA | GACATCCAGATGACCCAGTCTCCATCCTCCCT GTCTGCATCTGTTGGAGACACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGTTACATCCAGTT TGCAAGGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAACATAATAGTTACCCGCTC ACTTTCGGCGGAGGGACCAAGGTGGAGATCA AA | CAGCTGCAGCTGCAGGAGTCGGGGCCCAGGACTG GTGAAGCCTTCGGAGACCCTGTCCCTCACCTGCA CTGTCTCTGGTGGCTCCATCAACAGAAGTAGTTA TTACTGGGGCTGGATCCGCCAGCCCCCAGGGAA GGGGCTGGAGTGGATTGGGAATATCTATTATAGT GGGACCACCTACTACAACCCGTCCCTCAAGAGTC GAGTCACCATATCCGTAGACACGTCCAAGAACC AGTTCTCCCTGAACCTGAGCTCTGTGACCGCCGC AGACACGGCTGTATATTCCTGTGCGAGACATGGA AAAGACTGGGGCCTTGACTACTGGGGCCAGGGA ACCCTGGTCACCGTCTCCTCA |
| | | | SEQ ID NO: 24797 | SEQ ID NO: 28803 |

FIGURE 50

| | | | | |
|---|---|---|---|---|
| | | AA | DIQMTQSPSSLSASVGDRVTITCRASQGIRNDLG WYQQKPGKAPKRLIYVTSSLQGGVPSRFSGSGS GTEFTLTISSLQPEDFATYYCLQHNSYPLTFGGG TKVEIK | QLQLQESGPGLVKPSETLSLTCTVSGGSINRSSYYW GWIRQPPGKGLEWIGNIYYSGTTYYNPSLKSRVTIS VDTSKNQFSLNLSSVTAADTAVYSCARHGKDWGL DYWGQGTLVTVSS |
| | | | SEQ ID NO: 24798 | SEQ ID NO: 28804 |
| iPS:393806 | 21-225_6A6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAGTAGTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAATATCTATTATAGT GGGATCCCCTACTACAACCCGTCCCTCAAGAGTC GGGTCAACATATCCGTAGACACGTCCAAGAACC AGTTCTCCCTGAAGCTGAACTCTGTGACCGCCGC AGACACGGCTGTGTATTACTGTGCGAGACACAGC AGCAGCTGGTCTCTTGACTACTGGGGCCAGGGAA CCCTAGTCACCGTCTCCTCA |
| | | | SEQ ID NO: 24799 | SEQ ID NO: 28805 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHSSYPLTFGGGT KVEIK | QLQLQESGPGLVKPSETLSLTCTVSGGSISRSSYYW GWIRQPPGKGLEWIGNIYYSGIPYYNPSLKSRVNIS VDTSKNQFSLKLNSVTAADTAVYYCARHSSSWSL DYWGQGTLVTVSS |
| | | | SEQ ID NO: 24800 | SEQ ID NO: 28806 |

FIGURE 50

| iPS:393808 | 21-225_1A2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTCACCCTCTC ACTTTCGGCGGAGGGACCAAGGTGGAGATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATACCAT GAACTGGGTCCGCCAGGCTCCAGGGAAGGGGCT GGAGTGGGTCTCATCCATTAGTGGTAGTGGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGGGAGCAG CTCGTCCGGGTTTGACTACTGGGGCCAGGGAACC CTGGTCACCGTCTCCTCA |
|---|---|---|---|---|
| | | | SEQ ID NO: 24801 | SEQ ID NO: 28807 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSHPLTFGGG TKVEIK | EVQLVESGGGLVKPGGSLRLSCAASGFTFSSYTMN WVRQAPGKGLEWVSSISGSGSYIYYADSVKGRFTIS RDNAKNSLYLQMNSLRAEDTAVYYCARGSSSSGF DYWGQGTLVTVSS |
| | | | SEQ ID NO: 24802 | SEQ ID NO: 28808 |
| iPS:393810 | 21-225_5A4 | NA | GACATCCAGATGACCCAGTCTCCATCCTTCCGT GTCTGCATCTGTAGGAGACAGAGTCACCATCA CTTGTCGGGCGAGTCAGGGTATTAGCACCTGG TTAGCCTGGTATCAACAGAAACCAGGGAAAG CCCCTAAACTCCTGATCTATGATGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCGTGG ACGTTCGGCCAAGGGACCAAGGTGGAAATCA AA | GAGGTGCAGGTGTTGGAGTCTGGGGGAGGCTTG GTACAGCCGGGGGGGTCCCTGAGACTCTCCTGTG CAGCCTCTGGACTCACCTTTAGCAGCTCTGCCAT GAGCTGGGTCCGCCAGGCTCCAGGGAAGGGGCT GGAGTGGGTCTCAGCTATTAGTGGTCGTGGTGGT AACACATTCTACACAGACTCCGTGAAGGGCCGGT TCACCATTTCCAGAGACAATTCCAAGAACACGCT GTATCTGCAAATGAACAGCCTGAGAGCCGAGGA CACGGCCGTATATTACTGTGCGAAACTGGGGAA AGACTACTACTACTACGGTATGGACGTCTGGGGC CAAGGGACCACGGTCACCGTCTCCTCA |
| | | | SEQ ID NO: 24803 | SEQ ID NO: 28809 |

EP 4 435 105 A2

FIGURE 50

| | | | DIQMTQSPSSVSASVGDRVTITCRASQGISTWLA WYQQKPGKAPKLLIYDASSLQSGVPSRFSGSGS GTDFTLTISSLQPEDFATYYCQQANSFPWTFGQG TKVEIK | EVQVLESGGGLVQPGGSLRLSCAASGLTFSSSAMS WVRQAPGKGLEWVSAISGRGGNTFYTDSVKGRFTI SRDNSKNTLYLQMNSLRAEDTAVYYCAKLGKDYY YYGMDVWGQGTTVTVSS |
|---|---|---|---|---|
| | | AA | SEQ ID NO: 24804 | SEQ ID NO: 28810 |
| IPS:393812 | 21-225_6A11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATGACTGTGCGAGAGATCTTGG CTGGACGGAAGAGTACTGGGGCCAGGGAACCCT GGTCACCGTCTCCTCA |
| | | | SEQ ID NO: 24805 | SEQ ID NO: 28811 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPWTFGQG TKVEIK | QVQLVESGGGVVQPGRSLRLSCAASGFTFSDYGM HWVRQAPGKGLEWVAVIWFDGSNKYYADSVKGR FTISRDNSKNTLYLQMNSLRAEDTAVYDCARDLG WTEEYWGQGTLVTVSS |
| | | | SEQ ID NO: 24806 | SEQ ID NO: 28812 |

1629

FIGURE 50

| iPS:393814 | 21-225_7F4 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGCAGAAACCAGGGAAAG CCCCTAAGCGCCTGATTTATGCTGCATCCAATT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAGTGCTTACCCGCTC ACTTTCGGCGGAGGGACCAAGGTGGAGATCA AA | CAACTGCAGCTGCAGGAGTCGGGCCCAGGACTG GTGAAGCCTTCGGAGACCCTGTCCCTCACCTGCA CTGTCTCTGGTGGCTCCATCAGCAGGAGTAGTTA CTACTGGGGCTGGATCCGCCAGCCCCCAGGGAA GGGGCTGGAGTGGATTGGGAATATCTATTATAGT GGGGCCACCTACTACAACCCGTCCCTCAAGAGTC GAGTCACCATCTCCGTAGACACGTCCACGAACCA GTTCTCCCTGAAGCTGAGCTCTGTGACCGCCGCA GACACGGCTGTGTATTACTGTGCGAGACATAGCG GCAGCTGGTCCCTTGACTACTGGGGCCAGGGAAC CCTGGTCACCGTCTCCTCA |
| | | | SEQ ID NO: 24807 | SEQ ID NO: 28813 |
| | | AA | DIQMTQSPSSLSASVGDRVTITCRTSQGIRNDLG WYQQKPGKAPKRLIYAASNLQSGVPSRFSGSGS GTEFTLTISSLQPEDFATYYCLQHSAYPLTFGGG TKVEIK | QLQLQESGPGLVKPSETLSLTCTVSGGSISRSSYYW GWIRQPPGKGLEWIGNIYYSGATYYNPSLKSRVTIS VDTSTNQFSLKLSSVTAADTAVYYCARHSGSWSLD YWGQGTLVTVSS |
| | | | SEQ ID NO: 24808 | SEQ ID NO: 28814 |
| iPS:393816 | 21-225_6D4 | NA | GACATCCAGATGACCCAGTCTCCATCCTCCCT GTCTGCTTCTGTAGGAGACAGAGTCACCATCA CTTGCCGGGCAAGTCAGGCCATTAGAAATGAT TTAGGCTGGTATCAGCAGAAACCAGGGAAAG CCCCTAAGCGTCTGATCTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAGTAGTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTC CTACTGGGGCTGGATCCGCCAGCCCCCAGGGAA GGGGCTGGAGTGGATTGGAAATATCTATTATAGT GGGAGCGCCTACTACATTCCGTCCCTCAAGAGTC GAGTCACCATATCCGTAGCCACGTCCAAGAACCA GTTCTCCCTGAACCTGACCTCTGTGACCGCCGCA GACACGGCTGTGTATTACTGTGCGCGACACAGCA GCAGCTGGTCTCTTGACTGCTGGGGCCAGGGAAC CCTGGTCACCGTCTCCTCA |
| | | | SEQ ID NO: 24809 | SEQ ID NO: 28815 |

FIGURE 50

| | | | | |
|---|---|---|---|---|
| | | AA | DIQMTQSPSSLSASVGDRVTITCRASQAIRNDLGWYQQKPGKAPKRLIYAASSLQSGVPSRFSGSGSGTEFTLTISSLQPEDFATYYCLQHSSYPLTFGGGTKVEIK | QLQLQESGPGLVKPSETLSLTCTVSGGSISRSSSYWGWIRQPPGKGLEWIGNIYYSGSAYYIPSLKSRVTISVATSKNQFSLNLTSVTAADTAVYYCARHSSSWSLDCWGQGTLVTVSS |
| | | | SEQ ID NO: 24810 | SEQ ID NO: 28816 |
| IPS:393818 | 21-225_6G12 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCACTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATTTGGTATGATAGAAGTAATAACTACTATGCAGACTCCGTGAAGGGCCGATTCACCATCTCCAGAGACAATTCCAAGAATACGCTGTATCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24811 | SEQ ID NO: 28817 |
| | | AA | DIQMTQSPSSLSASVGDRVTITCRASQGIRNDLGWYQQKPGKAPKRLIYAASTLQSGVPSRFSGSGSGTEFTLTISSLQPEDFATYYCLQHNSYPLTFGGGTKVEIK | QVQLVESGGGVVQPGRSLRLSCAASGFTFSSYGMHWVRQAPGKGLEWVAVIWYDRSNNYYADSVKGRFTISRDNSKNTLYLQMNSLRAEDTAVYYCARELGFRSDYWGQGTLVTVSS |
| | | | SEQ ID NO: 24812 | SEQ ID NO: 28818 |

FIGURE 50

| iPS:393820 | 21-225_8H7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATTTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGTTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTC CAGCGTCTGGATTCACCTTCAGTGACTTTGGCAT GCACTGGGTCCGCCAGGCTCCAGGCAAGGGGCT GGAGTGGGTGGCAGTTATTTGGTATGAAGAAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAGCTGGGG TTCCGGTCTGACTACTGGGGCCAGGGAACCCTGG TCACCGTCTCCTCA |
| | | | SEQ ID NO: 24813 | SEQ ID NO: 28819 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGGGT KVEIK | QVQLVESGGGVVQPGRSLRLSCPASGFTFSDFGMH WVRQAPGKGLEWVAVIWYEENNQYYADSVKGRF TISRDNSKNTLYLQMNSLRAEDTAVYYCARELGFR SDYWGQGTLVTVSS |
| | | | SEQ ID NO: 24814 | SEQ ID NO: 28820 |
| iPS:393822 | 21-225_15B11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAATGGGTGGCAGTTATATGGTATGAGGAAAG TAATAAATACTATACAGACTCCGTGAAGGGCCG ATTCACCATCTCCAGAGACAATTCCAAGAACACG CTGTATCTGCAAATGAACAGCCTGAGACCCGAG GACACGGCTGTGTATTACTGTGCGAGAGAAGTGG GATTCACTGAGGACTACTGGGGCCAGGGAACCC TGGTCACCGTCTCCTCA |
| | | | SEQ ID NO: 24815 | SEQ ID NO: 28821 |

EP 4 435 105 A2

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSNYGM HWVRQAPGKGLEWVAVIWYEESNKYYTDSVKGR FTISRDNSKNTLYLQMNSLRPEDTAVYYCAREVGF TEDYWGQGTLVTVSS |
| | | | SEQ ID NO: 24816 | SEQ ID NO: 28822 |
| iPS:393824 | 21-225_10F12 | NA | GACATCCAGATGACCCAGTCTCCATCCTCCCT GTCTGCATCTGTAGGAGACAGAGTCACCATCA CTTGCCGGGCAAGTCAGAACATTAGTAGTTAT TTAAAATTGGTATCAGCAGAAACCAGGGAAAG CCCCTAAACTCCTGATCTATGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAATACCCCCTTC TTCACTTTCGGCCCTGGGACCAAAGTGGATAT CAAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAGCTGGGTCCGCCAGGCTCCAGGGAAGGGGCT GGAGTGGGTCTCAATTATTAGTGGTCGTGGTGGT AACACATTCTACGCAGACTCCGTGAAGGGCCGGT TCACCATCTCCAGAGACAATTCCAAGAACACTCT GTATCTGCAAATGAACAGCCTGAGAGCCGAGGA CACGGCCGTATATTACTGTGCGTCCCGTGTAGCA GTGGCTGGCTCGGAGGCTTTTGCTATCTGGGGCC AAGGGACAATGGTCACCGTCTCTTCA |
| | | | SEQ ID NO: 24817 | SEQ ID NO: 28823 |
| | | AA | DIQMTQSPSSLSASVGDRVTITCRASQNISSYLN WYQQKPGKAPKLLIYAASSLQSGVPSRFSGSGS GTDFTLTISSLQPEDFATYYCQQSYNTPFFTFGPG TKVDIK | EVQLLESGGGLVQPGGSLRLSCAASEFTFSSYAMS WVRQAPGKGLEWVSIISGRGGNTFYADSVKGRFTI SRDNSKNTLYLQMNSLRAEDTAVYYCASRVAVAG SEAFAIWGQGTMVTVSS |
| | | | SEQ ID NO: 24818 | SEQ ID NO: 28824 |

1633

FIGURE 50

| | | | | |
|---|---|---|---|---|
| iPS:393826 | 21-225_10G5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATTTGGTATGAAGAAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTTTATTACTGTACGAGAGAACTGGGG TTCCGGTCTGACTACTGGGGCCAGGGAACCCTGG TCACCGTCTCCTCA |
| | | | SEQ ID NO: 24819 | SEQ ID NO: 28825 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSNYGM HWVRQAPGKGLEWVAVIWYEENNQYYADSVKGR FTISRDNSKNTLYLQMNSLRAEDTAVYYCTRELGF RSDYWGQGTLVTVSS |
| | | | SEQ ID NO: 24820 | SEQ ID NO: 28826 |
| iPS:393828 | 21-225_10H12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGGTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATTTGGTATGAAGACAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCGGTCTGACTACTGGGGCCAGGGAACCCTGG TCACCGTCTCCTCA |
| | | | SEQ ID NO: 24821 | SEQ ID NO: 28827 |

FIGURE 50

| | | | | |
|---|---|---|---|---|
| | | AA | DIQMTQSPSSLSASVGDRVTITCRASQGIRNDLG WYQQKPGKAPKRLIYGASSLQSGVPSRFSGSGS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYEDNNQYYADSVKGR FTISRDNSKNTLYLQMNSLRAEDTAVYYCARELGF RSDYWGQGTLVTVSS |
| | | | SEQ ID NO: 24822 | SEQ ID NO: 28828 |
| IPS:393830 | 21-225_12A1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATTTGGTATGAAGAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCGGTCTGACTACTGGGGCCAGGGAACCCTGG TCACCGTCTCCTCA |
| | | | SEQ ID NO: 24823 | SEQ ID NO: 28829 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSSYGMH WVRQAPGKGLEWVAVIWYEENNQYYADSVKGRF TISRDNSKNTLYLQMNSLRAEDTAVYYCARELGFR SDYWGQGTLVTVSS |
| | | | SEQ ID NO: 24824 | SEQ ID NO: 28830 |

FIGURE 50

| iPS:393832 | 21-225_14B2 | NA | GACATCCAGATGACCCAGTCTCCATCCTCCCT GTCTGCATCTGTTGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGTTACATCCAGTT TGCAAGGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAACATAATAGTTACCCGCTC ACTTTCGGCGGAGGGACCAAGGTGGAGATCA AA | CAGCTGCAGCTGCAGGAGTCGGGGGCCAGGACTG GTGAAGCCTTCGGAGACCCTGTCCCTCACCTGCA CTGTCTCTGGTGGCTCCATCAACAGAAGTAGTTA TTACTGGGGTTGGATCCGCCAGCCCCCAGGGAAG GGGCTGGAGTGGATTGGGAATATCTATTATAGTG GGACCACCTACTACAACCCGTCCCTCAAGAGTCG AGTCACCATATCCGTAGACACGTCCAAGAACCA GTTCTCCCTGAACCTGAGCTCTGTGACCGCCGCA GACACGGCTGTATATTCCTGTGCGAGACATGGAA AAGACTGGGGCCTTGACTACTGGGGCCAGGGAG CCCTGGTCACCGTCTCCTCA |
| | | | SEQ ID NO: 24825 | SEQ ID NO: 28831 |
| | | AA | DIQMTQSPSSLSASVGDRVTITCRASQGIRNDLG WYQQKPGKAPKRLIYVTSSLQGGVPSRFSGSGS GTEFTLTISSLQPEDFATYYCLQHNSYPLTFGGG TKVEIK | QLQLQESGPGLVKPSETLSLTCTVSGGSINRSSYYW GWIRQPPGKGLEWIGNIYYSGTTYYNPSLKSRVTIS VDTSKNQFSLNLSSVTAADTAVYSCARHGKDWGL DYWGQGALVTVSS |
| | | | SEQ ID NO: 24826 | SEQ ID NO: 28832 |
| iPS:393836 | 21-225_15A2 | NA | GACATCCAGATGACCCAGTCTCCATCCTCACT GTTTGCATTTATAGGAGACAGAGTCACCATCA CTTGTCGGGCGAGTCAGGGCATTAGCAATTAT TTAGCCTGGTTTCAGCAGAAACCTGGAAAAGC CCCTAAGTCCCTGATTTTTGCTGCATCCAGTTT GCAAAGTGGGGTCCCATCAAAGTTTAGCGGCA GTGGATTTGGGACAGATTTCACTTTTCCCATCA GCAGCCTGCAGCCTGAAGATTTTGCAAATTAT TACTGCCAACAGTATTATAGTTACCCATTCACT TTCGGCCCTGGGACCCAAGTGGATGTCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATACCAT GAACTGGGTCCGCCAGGCTCCAGGGAAGGGGCT GGAGTGGGTCTCATCCATTAGTGGTAGTGGTAGT TACATATACTACGCAGACTCAGTGAAGGGCCGAT TCACCATCTCCAGAGACAACGCCAAGAACTCACT GTATCTGCAAATGAACGCCCTGAGAGCCGAGGA CACGGCTGTGTATTACTGTGCGAGAGTGGCTTCA TTTGACTACTGGGGCCAGGGAACCCTGGTCACCG TCTCCTCA |
| | | | SEQ ID NO: 24827 | SEQ ID NO: 28833 |

FIGURE 50

| | | | | |
|---|---|---|---|---|
| | | AA | DIQMTQSPSSLFAFIGDRVTITCRASQGISNYLAW FQQKPGKAPKSLIFAASSLQSGVPSKFSGSGFGT DFTFPISSLQPEDFANYYCQQYYSYPFTFGPGTQ VDVK | EVQLVESGGGLVKPGGSLRLSCAASGFTFSSYTMN WVRQAPGKGLEWVSSISGSGSYIYYADSVKGRFTIS RDNAKNSLYLQMNALRAEDTAVYYCARVASFDY WGQGTLVTVSS |
| | | | SEQ ID NO: 24828 | SEQ ID NO: 28834 |
| IPS:393838 | 21-225_6G2 | NA | GACATCCAGATGACCCAGTCTCCATCCTCCCG GTCTGCATTTGTTGGAGACAGAGTCACCATCA CTTACCGGGCAAGTCAGGGCATTAGAAATGAT TTAGGCTGGTATCAGCAGAAACCAGGGAAAG CCCCTCAGCGCCTGATCTATGCTGCATCCAGT CTGCAAAGTGGGGTCCCATCAAGGTTCAGCGG CAGTGGATATGGGACTGAATTCAATATCACAA TCAGCAGCTTGCAGCCTGAAGATTTTGCAATT TATTACTGTATACAGCATAATAGTTACCTGTG GACGTTCGGCCAAGGGACCAAGGTGGAAATC ACA | CTGGTGCAGCTGGTGGAGTCTGGGGGAGGCGTG GTCCAGCCTGGGAAGTCCCTGAGACTCTCCTGTG CAGCGTCTGGATTCACCTTCAGTGACTATGGCAT ACACTGGGTCCGCCAGGCTCCAGGCAAGGGGCT GGAGTGGGTGGCAGTCATTTGGTTTGATGGAAGT AATAAATACTATGCAGACTCCGTGAAGGGCCGA TTCACCATCTCCAGCGACAATTCCAAAAACACGC TGTATCTGCAAATGAACAGCCTGAGAGCCGAGG ACACGGCTGTGTATTACTGTGCGAGAGATCTTGG CTGGACGGAAGAGTACTGGGGCCAGGGAACCCT GGTCACCGTCTCCTCA |
| | | | SEQ ID NO: 24829 | SEQ ID NO: 28835 |
| | | AA | DIQMTQSPSSRSAFVGDRVTITYRASQGIRNDLG WYQQKPGKAPQRLIYAASSLQSGVPSRFSGSGY GTEFNITISSLQPEDFAIYYCIQHNSYLWTFGQGT KVEIT | LVQLVESGGGVVQPGKSLRLSCAASGFTFSDYGIH WVRQAPGKGLEWVAVIWFDGSNKYYADSVKGRF TISSDNSKNTLYLQMNSLRAEDTAVYYCARDLGW TEEYWGQGTLVTVSS |
| | | | SEQ ID NO: 24830 | SEQ ID NO: 28836 |

FIGURE 50

| iPS:393840 | 21-225_3F8 | NA | GACTTCCAGATGACCCAGTCTCCATCCTCCCT GTCTGCATCTGTAGGAGACAGAGTCACCATCA CTTGCCGGGCAAGTCAGAGTATTCTCAGCTAT TTAAATTGGTATCGGCAGAAACCAGGGAGAG CCCCTCAGGTCCTGATCCATACTACATCCAGTT TGCAAAGTGGGGTCCCATCAAGGTTCAGTGGC AGTGGATCTGGGACAGTTTTCACTCTCACCAT CAGCAGTCTGCAACCTGAAGATTTTGCAACTT ACTACTGTCAACAGACTTACAGTACCCCGCTC ACTTTCGGCGGAGGGACCAGGGTGGAGATCA AC | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGGGGCAGTTATATGGCATGATGGAAG TAATAAATACTATGCAGACTCCGTGAAGGGCCG ATTCACCATCTCCAGAGACAATGCCAAGAACAC GCTGTATCTGCAAATGAACAGCCTGAGAGCCGA GGACACGGCTGTGTATTACTGTGCGAGAGATCTG AGTATGGGCGGTATGGACGTCTGGGGCCAAGGG ACCACGGTCACCGTCTCCTCA |
|---|---|---|---|---|
| | | | SEQ ID NO: 24831 | SEQ ID NO: 28837 |
| | | AA | DFQMTQSPSSLSASVGDRVTITCRASQSILSYLN WYRQKPGRAPQVLIHTTSSLQSGVPSRFSGSGSG TVFTLTISSLQPEDFATYYCQQTYSTPLTFGGGTR VEIN | QVQLVESGGGVVQPGRSLRLSCAASGFTFSSYGMH WVRQAPGKGLEWGAVIWHDGSNKYYADSVKGRF TISRDNAKNTLYLQMNSLRAEDTAVYYCARDLSM GGMDVWGQGTTVTVSS |
| | | | SEQ ID NO: 24832 | SEQ ID NO: 28838 |
| iPS:393844 | 21-225_3G7 | NA | GACATCCAGATGACCCAGTCTCCATCCTCCCT GTCTGCATCTGTCGGAGACAGAGTCACCGTCA CTTGCCGGGCAAGTCAGAACATTTACAGGTAT TTAAATTGGTATCAGGGGAGACCAGGGAGAG CCCCTAAACTCATGATCTATGCTGCATCCAGTT CGCAAAGTGGGGTCCCATCAAGATTCAGTGGC AGTGGATCTGGGACAGATTTCACTGTCACCAT CAGTAGTCTTCAACCTGAAGATTTTGCAACTT ATTACTGTCAACAGAGTTACAGTCCCCCTTTC ACTTTCGGCGGAGGGGCCAAGGTGGAGATCG AC | CAGGTGCAGCTGGTGGAGTCTGGGGGAGGCGTG GTCCAGCCTGGGAGGTCCCTGAGACTCTCCTGTG CAGCGTCTGGATTCAACTTCAGTAACTATGGCAT GCACTGGGTCCGCCAGGCTCCAGGCAAGGGGCT GGAGTGGGTGGCAGTCATATGGCATGATGGAAG TAATAAATACTATGTAGACTCCGTGAAGGGCCGA TTCACCATCTCCAGAGACAATTCCAAGAACACGG TGTATCTGCAAATGAACAGCCTGAGAGCCGAGG ACACGGCTGTGTATTACTGTGCGAGAGATGAGAG GCTGGGGATTTTTGACTACTGGGGCCAGGGAACC CTGGTCACCGTCTCCTCA |
| | | | SEQ ID NO: 24833 | SEQ ID NO: 28839 |

FIGURE 50

| | | | | |
|---|---|---|---|---|
| | | AA | DIQMTQSPSSLSASVGDRVTVTCRASQNIYRYLN WYQGRPGRAPKLMIYAASSSQSGVPSRFSGSGS GTDFTVTISSLQPEDFATYYCQQSYSPPFTFGGG AKVEID | QVQLVESGGGVVQPGRSLRLSCAASGFNFSNYGM HWVRQAPGKGLEWVAVIWHDGSNKYYVDSVKGR FTISRDNSKNTVYLQMNSLRAEDTAVYYCARDERL GIFDYWGQGTLVTVSS |
| | | | SEQ ID NO: 24834 | SEQ ID NO: 28840 |
| iPS:393848 | 21-225_4H2 | NA | GACATCCAGATGACCCTGTCTCCATCCTCCCT GTCTGCATCTGTAGGAGACAGAGTCACCATCA CTTGCCGGGCAATTCAGAACATTAGCAGCTAT TTAAATTGGTATCAGCAGAAACCAGGGAAAG CCCCTAAACTCGTGATCTATGCTGCATCCAGC TTGCAAAGTGGGGTCCCATCAAGGTTCAGTGG CAGAGGATCTGGGACAGATTTCACTCTCACCA TCGGTTGTGTGCAACGTGAAGATTTTGCAACT TACTACTGTCAACAGAGTTACAGAACCCCCTT ATTCACTTTCGGCCCTGGGACCAAGGTTGATA TCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCGGCTATGCCAT GAACTGGGTCCGCCAGGCTCCAGGGATGGGGCT GGAGTGGGTCTCAGTTATTAGTCGTAGTGGTGGT TACACATACTACGCGGACTCCGTGAAGGGCCGGT TCACCATCTCCAGAGACAATTCCAAGAACACGCT GTATCTGCAAATGAACAGCCTGAGAGCCGAGGA CACGGCCGTCTATTACTGTGCGTCCCGTTTAGCA GTGGCTGGCTCGGAGGCTTTTGATATCTGGGGCC AAGGGACAATGGTCACCGTCTCTTCA |
| | | | SEQ ID NO: 24835 | SEQ ID NO: 28841 |
| | | AA | DIQMTLSPSSLSASVGDRVTITCRAIQNISSYLNW YQQKPGKAPKLVIYAASSLQSGVPSRFSGRGSGT DFTLTIGCVQREDFATYYCQQSYRTPLFTFGPGT KVDIK | EVQLLESGGGLVQPGGSLRLSCAASGFTFSGYAMN WVRQAPGMGLEWVSVISRSGGYTYYADSVKGRFT ISRDNSKNTLYLQMNSLRAEDTAVYYCASRLAVAG SEAFDIWGQGTMVTVSS |
| | | | SEQ ID NO: 24836 | SEQ ID NO: 28842 |

FIGURE 50

| iPS:393852 | 21-225_12A10 | NA | GACGTCCAGATGACCCAGTCTCCATCCTCCCTGTCTGCATCTGCAGGAGACAGAGTCACCATCACTTGCCGGGCAAGTCAGAACATTTACAGCTATTTAAAATTGGTATCAGCAGAAACCAGGGAGAGCCCCTAAGGTCCTGATCCATACTGCATCCAGTTTGCAAAGTGGGGTCCCATCAAGGTTCAGTGGCAGTGGATCTGGGGCAGATTTCACTCTCACCATCAACAGTCTGCAACCTGAAGATTTTGCAACTTACTACTGTCAGCAGAGTTACAGTCCCCCTCTCACTTTCGGCGGAGGGACCAAGGTAGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGCATGATGAAAGTAATAAATACTATACAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGACGAGAGGCTGGGGATTTTTGACTACTGGGGCCAGGGAACCCTGGTCACCGTCTCCTCA |
|---|---|---|---|---|
| | | | SEQ ID NO: 24837 | SEQ ID NO: 28843 |
| | | AA | DVQMTQSPSSLSASAGDRVTITCRASQNIYSYLNWYQQKPGRAPKVLIHTASSLQSGVPSRFSGSGSGADFTLTINSLQPEDFATYYCQQSYSPPLTFGGGTKVEIK | QVQLVESGGGVVQPGRSLRLSCAASGFTFSSYGMHWVRQAPGKGLEWVAVIWHDESNKYYTDSVKGRFTISRDNSKNTLYLQMNSLRAEDTAVYYCARDERLGIFDYWGQGTLVTVSS |
| | | | SEQ ID NO: 24838 | SEQ ID NO: 28844 |
| iPS:393854 | 21-225_7H11 | NA | GACATCCAGATGACCCAGTCTCCATCCTCCGCATCTGCATCTGTAGGAGTCAGAGTCACCATCATTTGCCTGGCAAGTCAGGGCATTAGAAATGATTTAGGCTGGTATCAGCAGAAACCAGGGAAAGCCCCTAAGCGCATAATCTATGTTGCATGTAGTTTCCAAAGTGGGGTCCCATCAAGGTTCAGCGGCAGTGGATATGGGACAGAATTCACTCTCACAATCAGCATCATGCAGCCTGAAGATTTCGCAACTTATTACTGTCTACAACATAATCTTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTTCAGCGTCTGGATTCACCTTCAGTGACTATGGCATGCACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24839 | SEQ ID NO: 28845 |

FIGURE 50

| | | | | |
|---|---|---|---|---|
| | | AA | DIQMTQSPSSASASVGVRVTIICLASQGIRNDLG WYQQKPGKAPKRIIYVACSFQSGVPSRFSGSGY GTEFTLTISIMQPEDFATYYCLQHNLYPLTFGGG TKVEIK | QVQLVESGGGVVQPGRSLRLSCSASGFTFSDYGMH WVRQAPGKGLEWVAVIWYDENNKYYADSVKGRF TISRDNSKNTLYLQMNSLRAEDTAVYYCARELGFR SDYWGQGTLVTVSS |
| | | | SEQ ID NO: 24840 | SEQ ID NO: 28846 |
| IPS:393856 | 21-225_14C2 | NA | GACATCCAGATGATCCAGTCTCCATCCTCCCT GTTTGCATGTGTAGGAGACAGAGTCATCATCA CTTGCCGGGCAAGTCAGGGCATTAGAAATGAT TTAGACTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCGTGCAGCGTGAAGATTTTGCAACT TATTACTGTGTACAGCATAATAGTTACCCGCT CACTTTCGGCGGAGGGACCAAGGTGGAGATC AGA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGACGAAAG TAATAAATACTATGAAGACTCCGTGAAGGGCCG ATTCACCATCTCCAGAGACAATTCCAAGAACACG CTGTATCTGCAAATGAAAAGCCTGAGAGCCGAG GACACGGGTGTGTATTACTGTGCGAGAGAAGTG GGATTCCGGTCTGACTACTGGGGGCCAGGGAACCC TGGTCACCGTCTCCTCA |
| | | | SEQ ID NO: 24841 | SEQ ID NO: 28847 |
| | | AA | DIQMIQSPSSLFACVGDRVIITCRASQGIRNDLDW YQQKPGKAPKRLIYAASSLQSGVPSRFSGSGSGT EFTLTISSVQREDFATYYCVQHNSYPLTFGGGTK VEIR | QVQLVESGGGVVQPGRSLRLSCAASGFTFSDYGM HWVRQAPGKGLEWVAVIWYDESNKYYEDSVKGR FTISRDNSKNTLYLQMKSLRAEDTGVYYCAREVGF RSDYWGQGTLVTVSS |
| | | | SEQ ID NO: 24842 | SEQ ID NO: 28848 |

FIGURE 50

| iPS:393862 | 21-225_5G2 | NA | GACATCCAGATGACCCAGTCTCCATCCTCCCC GTCTGCATCGTAGGAGACAGAGTCACCATCA CTTTCCGGGCAAGTCAGAACATTATTAGTTAT TTAAATTGGTATCAGCAGAAACCAGGGAAAG CCCGTAAGCTCGTGATCTATGGTGCATCCAGT TTGCAAAGTGGGGTCCCATCAAGGTTCAGTGG CAGTGGATCTGGGACAGATTTCACTCTCAACA TCAGAAGTCTGCAACCTGAAGATTTTGCAACT TACTACTGTCAACAGAGTTACAGTACTCCCTT ATTCACTTTCGGCCCTGGGACCAAAGTGGATA TCAAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAGCTGGGTCCGCCAGGCTCCAGGGAAGGGGCT GGAGTGGGTCTCAGTTATTAGTGGTCGTGGTGTT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTATAGCA GTGGCTGGCTCGGAGGCTTTTGATATCTGGGGCC AAGGGACAATGGTCACCGTCTCTTCA |
| | | | SEQ ID NO: 24843 | SEQ ID NO: 28849 |
| | | AA | DIQMTQSPSSPSASVGDRVTITFRASQNIISYLNW YQQKPGKARKLVIYGASSLQSGVPSRFSGSGSGT DFTLNIRSLQPEDFATYYCQQSYSTPLFTFGPGTK VDIK | EVQLLESGGGLVQPGGSLRLSCAASEFTFSSYAMS WVRQAPGKGLEWVSVISGRGVNTFYADSVKGRFTI SRDNSKNTLYLQMNSLRAEDTAVYYCASRIAVAGS EAFDIWGQGTMVTVSS |
| | | | SEQ ID NO: 24844 | SEQ ID NO: 28850 |
| iPS:393864 | 21-225_4C5 | NA | GACATCCAGATGACCCAGTCTCCATCCTCCCT GTCTGCATCGTAGGACACAGAGTCCACCTCA CTTGCCGGGCAAGTCGGGGCATCAGAGGTGAT TTAGGTTGGTATCGCCAGAAACCAGGGAAAGC CCCTAAGCGCTTGATCTATGCTGCATCCAATTT GCAAAGTGGGGTCCCATCAAGGTTCAGCGGCA GTGGATATGGGACAGAATTCACTCTCACAATC GGCAGCCTGCAGCCTGAAGATTTTGCAACTTA TTACTGTCTACAGCATTATAGTTACCCTCGGAC GTTCGGCCAAGGGACCAAGGTGGAAATCAAA | CAGGTGCAACTGGTGGAGTCTGGGGGAGGCGTG GTCCAGCCTGGGAGGTCCCTGAGACTCTCCTGTG CAGCGTCTGGATTCACCTTCAGTAGCTATGTCCT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TATATCTGCAAATGAACAGCCTGAGAGCCGAGG ACACGGCTGTTTATTACTGTGCGCGAGAAAAGTA TACCAGCAGCTGGTACGACTACGGTATGGACGTC TGGGGCCAAGGGACCACGGTCACCGTCTCCTCA |
| | | | SEQ ID NO: 24845 | SEQ ID NO: 28851 |

FIGURE 50

| | | | | |
|---|---|---|---|---|
| | | AA | DIQMTQSPSSLSASVGHRVHLTCRASRGIRGDLG WYRQKPGKAPKRLIYAASNLQSGVPSRFSGSGY GTEFTLTIGSLQPEDFATYYCLQHYSYPRTFGQG TKVEIK | QVQLVESGGGVVQPGRSLRLSCAASGFTFSSYVLH WVRQAPGKGLEWVAVIWYDGSNKYYADSVKGRF TISRDNSKNTLYLQMNSLRAEDTAVYYCAREKYTS SWYDYGMDVWGQGTTVTVSS |
| | | | SEQ ID NO: 24846 | SEQ ID NO: 28852 |
| IPS:393866 | 21-225_14E3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAACAAAAACCAGGGAAAG CCCCTAAGCGCATTATTTATTCTGCATCCAGTT TGCAAAGTGGGGTCCCATCAAGGTTCATCGGC AGTGGATGTGGGACTGAATTCACTCTCACAAT CAGCAGCCTGCAGCGTGAAGATTTTGCAGCTT ATTACAGTGTACAGCATTATAGTTACCCGTTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTTTGGCAT GCACTGGGTCCGCCAGGCTCCAGGCAAGGGGCT GGAGTGGGTGGCAGTTATTTGGTATGAAGAAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTTTTTATTACTGTGCGAGAGAGCTGGGG TTCCGGTCTGACTACTGGGGCCAGGGAACCCTGG TCACCGTCTCCTCA |
| | | | SEQ ID NO: 24847 | SEQ ID NO: 28853 |
| | | AA | DIQMTQSPSSLSASVGDRVTITCRASQGIRNDLG WYQQKPGKAPKRIIYSASSLQSGVPSRFIGSGCG TEFTLTISSLQREDFAAYYSVQHYSYPFTFGGGT KVEIK | QVQLVESGGGVVQPGRSLRLSCAASGFTFSDFGMH WVRQAPGKGLEWVAVIWYEENNQYYADSVKGRF TISRDNSKNTLYLQMNSLRAEDTAFYYCARELGFR SDYWGQGTLVTVSS |
| | | | SEQ ID NO: 24848 | SEQ ID NO: 28854 |

FIGURE 50

| iPS:393868 | 21-225_9C11 | NA | GACATCCAGATGACCCAGTCTCCATCCTCCCT GTCTGCATCTGTAGGAGACAGAGTCACCATCA CTTGCCGGGCAAGTCAGAACATTAGAAATTAT TTAAATTGGTATCAGCAGAAATCAGGGAGAGC CCCTAAGCTCCTGATCTATGTTGCATCCAGTTT GCAAAGTGGGGTCCCATCAAGGTTCAGTGGCA GTGGATCTGGGACAGAATTCACTCTCACCATC AACAGTCTGCAACCTGAAGATTTTGCAATTTA TCACTGTCATCAGAGTAACAGTACTCCTCTCA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGCATGATGAAACT AATAAATACTATGCAGATTCCGTGAAGGGCCGAT TCACCATCTCCAGAGACAATTCCAAGAACACGCT GTCTCTGCAAATGAACAGCCTGAGAGCCGAGGA CACGGCTGTGTATTACTGTGCGAGAGACGAGAG GCTGGGGATTTTTGACTACTGGGGCCAGGGAACC CTGGTCACCGTCTCCTCA |
| | | | SEQ ID NO: 24849 | SEQ ID NO: 28855 |
| | | AA | DIQMTQSPSSLSASVGDRVTITCRASQNIRNYLN WYQQKSGRAPKLLIYVASSLQSGVPSRFSGSGSG TEFTLTINSLQPEDFAIYHCHQSNSTPLTFGQGTK VEIK | QVQLVESGGGVVQPGRSLRLSCAASGFTFSSYGMH WVRQAPGKGLEWVAVIWHDETNKYYADSVKGRF TISRDNSKNTLSLQMNSLRAEDTAVYYCARDERLG IFDYWGQGTLVTVSS |
| | | | SEQ ID NO: 24850 | SEQ ID NO: 28856 |
| iPS:393870 | 21-225_7B1 | NA | GATATCCAGATGACCCAGGCTCCATCCTCACT GTCTGCTTCTGTAGGAGACAGAGTCACCATCA CTTGTCGGGCGAGTCAGGACATTAGCAATCAT TTAGTCTGGTTTCAGCAGAAACCAGGGAAAGC CCCTAAGTCCCTGATCTTTGCTGCATCCAGTTT ACAAAGTGGGGTCCCATCACAGTTCAGCGGCA GTGGATCTGGAACAGATTTCACTCTCACCATC AGCATCCTGCAGCCTGAGGATTTTGCAACTTA TTACTGCCACCAGTATAATAGTTACCCCTTCAC TTTCGGCCCTGGGACCAAAGTGGATTTCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGACAT GAGCTGGGTCCGCCAGGCTCCAGGAAAGGGGCT GGAGTGGGTCTCAACTATTAGTGGTAGTGGTGGT ATCACATACTACGCAGACTCCGTGAAGGGCCGGT TCACCATCTCCAGAGACAATTCCAAGAAAACGCT GTATCTGCAAATGAACAGCCTGAGAGCCGAGGA CACGGCCGTATATTTCTGTGCGAGAGATCGGGGC AGCGTCTGGGGCCAGGGAACCCTGGTCACCGTCT CCTCA |
| | | | SEQ ID NO: 24851 | SEQ ID NO: 28857 |

FIGURE 50

| | | | | |
|---|---|---|---|---|
| | | AA | DIQMTQAPSSLSASVGDRVTITCRASQDISNHLV WFQQKPGKAPKSLIFAASSLQSGVPSQFSGSGSG TDFTLTISILQPEDFATYYCHQYNSYPFTFGPGTK VDFK | EVQLLESGGGLVQPGGSLRLSCAASGFTFSSYDMS WVRQAPGKGLEWVSTISGSGGITYYADSVKGRFTI SRDNSKKTLYLQMNSLRAEDTAVYFCARDRGSVW GQGTLVTVSS |
| | | | SEQ ID NO: 24852 | SEQ ID NO: 28858 |
| iPS:393872 | 21-225_2A11 | NA | GACATCCAGATGACCCAGTCTCCATCCTCCCT GTCTCCATCTGTAGGAGACAGAGTCACCATCA CTTGCCGGGCAAGTCAGGGCATTAGAAATGAT TTAGGCTGGTATCAGCAGAAACCAGGGAAAG CCCCTCAGCGCCTGATCTCTGCTGCATCCAGTT TGCAAAGTGGGGTCCCATCAAGGTTCAGCGGC AGTGGATCTGGGACAGATTTCACTCTCACAAT CAGCAGTTTGCAGCCTGAAGATTTTGCAACTT ATTACTGTCTACAGCATAATAGTTACCCGCTC ACTTTCGGCGGAGGGACCAAGATGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGGAGTAGTTA CTACTGGGGCTGGATCCGGCAGCCCCCAGGGAA GGGGCTGGAGTGGATTGGGAATATCTATTATAGT GGGAGCACCTACTACAACCCGTCCGTCAAGAGTC GAGTCACCATATCCGTAGACACGTCCAAGAACC AGTTCTCCCTGAAGCTGAGTACTGTGACCGCCGC AGACACGGCTGTGTATTACTGTGCGAGACATGGA AAAGACTGGGGCCTTGAAGACTGGGGCCAGGGA ACCCTGGTCACCGTCTCCTCA |
| | | | SEQ ID NO: 24853 | SEQ ID NO: 28859 |
| | | AA | DIQMTQSPSSLSPSVGDRVTITCRASQGIRNDLG WYQQKPGKAPQRLISAASSLQSGVPSRFSGSGSG TDFTLTISSLQPEDFATYYCLQHNSYPLTFGGGT KMEIK | QLQLQESGPGLVKPSETLSLTCTVSGGSISRSSYYW GWIRQPPGKGLEWIGNIYYSGSTYYNPSVKSRVTIS VDTSKNQFSLKLSTVTAADTAVYYCARHGKDWGL EDWGQGTLVTVSS |
| | | | SEQ ID NO: 24854 | SEQ ID NO: 28860 |

FIGURE 50

| iPS:393874 | 21-225_4C8 | NA | GACATCCAGATGATCCAGTCTCCATCCTTCCT GTTTGCATGTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATATATGCTGCATCCAGT TTGCAAAGTGGGGTCCCATCAAGGTTCAGCGG CAGTGTATCTGGGACAGAATTCACTCTCACAA TCAGCAGCCTGCAGCCTGAAGATTTCGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATTTGGTATGAAGAAAT AATCAATACTATGCAGACTCCGTGAAGGGCCGAT TCACCATCTCCAGAGACAATTCCAAGAACACGCT GTATCTGCAAATGAACAGCCTGACAGCCGAGGA CACGGCTGTTTATTACAGTCCGAGAGAAATGGGG TTCCTGTCTGACTACTGGGGCCAGGGAACCCTGG TCACCGTCTCCTCA |
|---|---|---|---|---|
| | | | SEQ ID NO: 24855 | SEQ ID NO: 28861 |
| | | AA | DIQMIQSPSFLFACVGDRVTITCRASQGIRNDLG WYQQKPGKAPKRLIYAASSLQSGVPSRFSGSVS GTEFTLTISSLQPEDFATYYCLQHNSYPLTFGGG TKVEIK | QVQLVESGGGVVQPGRSLRLSCAASGFTFSNYGM HWVRQAPGKGLEWVAVIWYEENNQYYADSVKGR FTISRDNSKNTLYLQMNSLTAEDTAVYYSPREMGF LSDYWGQGTLVTVSS |
| | | | SEQ ID NO: 24856 | SEQ ID NO: 28862 |
| iPS:393876 | 21-225_9A1 | NA | GACATCCAGATGACCCAGTCTCCATCCTCCCG GTCTGCATTTGTTGGAGACAGAGTCACCATCA CTTGCCGGGCAAGTCAGGGCATTAGAAATGAT TTAGGGTGGTATCAGCAGAAACCAGGGAAAG CCCGTAAGCGCCTGATCTATACTGCATCCAGT TTGCAAAGTGGGGTCCCATCAAGGTTCAGCGG CAGTGGATATGGGACTGAATTCACTATCACAA TCAGCAGCTTGCAGCCTGAAGATTTTGCAACT TATTACTGTATACAGCATAATAGTTACCTGTG GACGTTCGGCCAAGGGACCAAGGTGGAAATC AAA | CAGGTGCAGGTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAGT AATAAATACTATGCAGACTCCGTGAAGGGCCGA TTCACCATCTCCAGAGACAATTCCAAGAACACGC TGTATCTGCAAATGAACAGTCTGAGAGCCGAGG ACACGGCTGTGTATTACTGTGCGAGAGATCTTGG CTGGACGGAAGAGTACTGGGGCCAGGGAACCCC GGTCACCGTCTCCTCA |
| | | | SEQ ID NO: 24857 | SEQ ID NO: 28863 |

FIGURE 50

| | | | | |
|---|---|---|---|---|
| | | AA | DIQMTQSPSSRSAFVGDRVTITCRASQGIRNDLG WYQQKPGKARKRLIYTASSLQSGVPSRFSGSGY GTEFTITISSLQPEDFATYYCIQHNSYLWTFGQGT KVEIK | QVQVVESGGGVVQPGRSLRLSCAASGFTFSDYGM HWVRQAPGKGLEWVAVIWFDGSNKYYADSVKGR FTISRDNSKNTLYLQMNSLRAEDTAVYYCARDLG WTEEYWGQGTPVTVSS |
| | | | SEQ ID NO: 24858 | SEQ ID NO: 28864 |
| iPS:393878 | 21-225_7G12 | NA | GACATCCAGATGACCCAGTCTCCATCCTCCCT GTCTGCATCTGTAGGAGACAGAGTCTCCATCA CTTGCCGGGCAAGTCAGAATATTAACAACTAT TTAAATTGGTATCAGCAGAAACCAGGGAAAG GCCCTAAGGTCCTGATCCTTACTGCATCCAGTT TGCAAAGTGGGGTCCCATCAAGGTTCAGTGGC AGTGGATCTGGGACAGATTTCACTCTCACCAT CAACAGTCTGCAACCTGAAGATTTTGCAACTT ACTACTGTCAACAGAGTTACACTACCCCCACG TGGACGTTCGGCCAAGGGACCAAGGTGGAAA TCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGTTATTAGTGGTAGTGGTGGT AGCACATACTACGCAGACTCCGTGAAGGGCCGG TTCACCATCTCCAGAGACAATTCCAAGAACACGC TGTATTTGCAAATGAACACCCTGAGAGCCGAGG ACACGGCCGTATATTACTGTGCGAAAAGGTATAC CAGTGACTGGCATGATGCTTTTGATATCTGGGGC CAAGGGACAATGGTCACCGTCTCTTCA |
| | | | SEQ ID NO: 24859 | SEQ ID NO: 28865 |
| | | AA | DIQMTQSPSSLSASVGDRVSITCRASQNINNYLN WYQQKPGKGPKVLILTASSLQSGVPSRFSGSGSG TDFTLTINSLQPEDFATYYCQQSYTTPTWTFGQG TKVEIK | EVQLLESGGGLVQPGGSLRLSCAASGFTFSSYAMN WVRQAPGKGLEWVSVISGSGGSTYYADSVKGRFTI SRDNSKNTLYLQMNTLRAEDTAVYYCAKRYTSDW HDAFDIWGQGTMVTVSS |
| | | | SEQ ID NO: 24860 | SEQ ID NO: 28866 |

FIGURE 50

| iPS:393880 | 21-225_15A1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGTTCTGGGACAGAATTCACTCTCACAG TCAGCAGCCTGCAGCCTGAAGATTTTGCAAGT TATTACTGTCTACACAACAGTAGTTACCCTGTT AAGTTTGGGGGAGGGATAAAGGTGGAGATCA CA | CAGCTGCATCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAGTATCTATTATAGT GGGAGCGCCCAGTACAACCCGTCCCTCAAGAGT CGAGTCACCATATCCGTAGACACGACCAAGAAC CAGTTCTCCCTGACGCTGAGCTCTGTGACCGCCG CAGACACGGCTGTATATTACTGTGCGAGACTGAG CAGCAGCTGGTCTTTTGACTACTGGGGCCAGGGA ACCCTGGTCACCGTCTCCTCA |
|---|---|---|---|---|
| | | | SEQ ID NO: 24861 | SEQ ID NO: 28867 |
| | | AA | DIQMTQSPSSLSASVGDRVTITCRASQGIRNDLG WYQQKPGKAPKRLIYAASSLQSGVPSRFSGSGS GTEFTLTVSSLQPEDFASYYCLHNSSYPVKFGGG IKVEIT | QLHLQESGPGLVKPSETLSLTCTVSGGSISRSSYYW GWIRQPPGKGLEWIGSIYYSGSAQYNPSLKSRVTIS VDTTKNQFSLTLSSVTAADTAVYYCARLSSSWSFD YWGQGTLVTVSS |
| | | | SEQ ID NO: 24862 | SEQ ID NO: 28868 |
| iPS:393882 | 21-225_15E3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TCGCAAAGTGGTGTCCCATCAAGGTTCAGCGG CAGTCGATCTGGGACAGAATTCACTCTCACAA TCAGCAGCCTGCAGCCTGAAGATCTTGCAGCT TATTACTGTCTACAGCATCATAGTTACCCGCTC ACTTTCGGCGGAGGGACCGAGGTGGAGATCTA C | CAGGTGCAGTTGGTGGAGTCTGGGGGAACCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGAGGAAAA TAATAAACACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGAGCTGG GGTTCCTCTCTGACTACTGGGGCCAGGGAACCCT GGTCACCGTCTCCTCA |
| | | | SEQ ID NO: 24863 | SEQ ID NO: 28869 |

FIGURE 50

| | | | | |
|---|---|---|---|---|
| | | AA | DIQMTQSPSSLSASVGDRVTITCRASQGIRNDLG WYQQKPGKAPKRLIYAASSSQSGVPSRFSGSRSG TEFTLTISSLQPEDLAAYYCLQHHSYPLTFGGGT EVEIY | QVQLVESGGTVVQPGRSLRLSCAASGFTFSSYGMH WVRQAPGKGLEWVAVIWYEENNKHYADSVKGRF TISRDNSKNTLYLQMNSLRAEDTAVYYCARELGFL SDYWGQGTLVTVSS |
| | | | SEQ ID NO: 24864 | SEQ ID NO: 28870 |
| IPS:393884 | 21-225_16F4 | NA | GACATCCAGATGATCCAGTCTCCATCCTCCCC GTCTGCATCTGTAGGAGACAGAGTCACCATCA CTTGCCGGGCAAGTCAGGGCATTAGAAATGAT TTAGGCTGGTATCAGCAGAAATCAGGGAAAG CCCCTAAGCGCCTGATATATGTTGCATCCAGT TTGCAAAGTGGGGTCCCATCAAGGTTCAGCGG CAGTGGATCTGGGACAGAATTCACTATCACAA TCAGCAGCGTGCAGCCTGAAGATTTTGCAACT TATTACTGTATACAGCATAATAGTTATCCGTTC ACTTTCGGCGGAGGGACCAAGGTGGAGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGAAGGAAG TAATCAATACTATGGAGACTCCGTGAAGGGCCG ATTCACCATCTCCAGAGACAATTCCAAGAACACG GTGTATCTGCAAATGCACAGCCTGAGAGCCGAG GACACGGCTGTGTATTACTGTGCGAGAGAGCTGG GGTTCCTCTCTGACTACTGGGGGCCAGGGAACCCT GGTCACCGTCTCCTCA |
| | | | SEQ ID NO: 24865 | SEQ ID NO: 28871 |
| | | AA | DIQMIQSPSSPSASVGDRVTITCRASQGIRNDLG WYQQKSGKAPKRLIYVASSLQSGVPSRFSGSGS GTEFTITISSVQPEDFATYYCIQHNSYPFTFGGGT KVEIK | QVQLVESGGGVVQPGRSLRLSCAASGFTFSNYGM HWVRQAPGKGLEWVAVIWYEGSNQYYGDSVKGR FTISRDNSKNTVYLQMHSLRAEDTAVYYCARELGF LSDYWGQGTLVTVSS |
| | | | SEQ ID NO: 24866 | SEQ ID NO: 28872 |

FIGURE 50

| iPS:393886 | 21-225_2G9 | NA | GACATCCAGATGACCCAGTCTCCATCCTCCCT GTTTGCATCGTCGGAGACAGAGTCACCATCA CTTGCCGGGCAAGTCAGGGCATTAGAAATGAT TTAGGTTGGTATCAGCAGAAACCAGGGAAAG CCCCTCAGCGCCTGATCTATGCTGCATCCAGTT TGCAAAGTGGAGTCCCATCAAGGTTCAGCGGC AGTGGATTTGGGACAGAATTCACTCTCACAAT CAGCAGCCTGCAGCTTGAAGATTTTGCAACTT ATTACTGTTTACAGCATGAAAGTTACCCTCTC ACTTTCGGCGGAGGGACCAAGGTGGAAATCA AA | CAACTGCAGCTGCAGGAGTCGGGCCCAGGACTG GTGAAGCCTTCGGAGACCCTGTCCCTCACCTGCA CTGTCTCTGGTGGCTCCATCAGACCTAATTACTA CTGGGGCTGGATCCGCCAGCCCCCAGGGAAGGG GCTGGAGTGGATTGGTAGTATCTATTATAGTGGA AGCACCTCCTACAACCCGTCCCTCAACAGTCGAG TCACCATATCCGTGGACACGTCCAAGAACCAGTT CTCCCTGAAGCTGAACTCTGTGACCGCCGCAGAC ACGGCTGTGTATTACTGTGCGAGACTAAGCAGCA ACTGGGACTTTGACAACTGGGGCCAGGGAACCC TGGTCACCGTCTCCTCA |
| | | | SEQ ID NO: 24867 | SEQ ID NO: 28873 |
| | | AA | DIQMTQSPSSLFASVGDRVTITCRASQGIRNDLG WYQQKPGKAPQRLIYAASSLQSGVPSRFSGSGF GTEFTLTISSLQLEDFATYYCLQHESYPLTFGGGT KVEIK | QLQLQESGPGLVKPSETLSLTCTVSGGSIRPNYYWG WIRQPPGKGLEWIGSIYYSGSTSYNPSLNSRVTISVD TSKNQFSLKLNSVTAADTAVYYCARLSSNWDFDN WGQGTLVTVSS |
| | | | SEQ ID NO: 24868 | SEQ ID NO: 28874 |
| iPS:393888 | 21-225_3E3 | NA | GACATCCAGATGACCCAGTCTCCATCCTCCCC GTCTGCATCTGTAGGAGACAGAGTCACCATCA CTTTCCGGGCAAGTCAGAGCATTAGAAGTTAT TTAAATTGGTATCAGCAGAAACCAGGGAAAG CCCATAAACTCGTGATCTATGGTACATCCAGT TTGCAAAGTGGGGTCCCATCAAGGTTCAGTGG CAGTGGATCTGGGACAGACTTCACTCTCACCA TCAGCAGTCTGCAACCTGAAGATTTTGCAACT TACTACTGTCAACAGAGTTACAGTACCCCCTT GTTCACTTTCGGCCCTGGGACCAAAGTGGATA TCAAA | GAGGTGCAGCTGTTGGAGTCTGGGGGAACCTTGG TACAGCCTGGGGGGTCCCTGAGACTCTCCTGTGC AGCCTCTGAATTCACCTTTAGCAGCTATGTCATG AGCTGGGTCCGCCAGGCTCCAGGGAAGGGGCTG GAGTGGGTCTCAATTATTAGTGGTCGTGGTGGTA ACACATTCTACGCAGACTCCGTGAAGGGCCGGTT CACCATCTCCAGAGACAATTCCAAGAACACGCTG TATCTGCAAATGAACAGCCTGAGAGCCGAGGAC ACGGCCTTATATTACTGTGCGTCCCGTTTAGCAG TGGCTGGCTCGGAGGCTTTTGATATCTGGGGCCA AGGGACAATGGTCACCGTCTCTTCA |
| | | | SEQ ID NO: 24869 | SEQ ID NO: 28875 |

FIGURE 50

| | | | | |
|---|---|---|---|---|
| | | AA | DIQMTQSPSSPSASVGDRVTITFRASQSIRSYLNW YQQKPGKAHKLVIYGTSSLQSGVPSRFSGSGSGT DFTLTISSLQPEDFATYYCQQSYSTPLFTFGPGTK VDIK | EVQLLESGGTLVQPGGSLRLSCAASEFTFSSYVMS WVRQAPGKGLEWVSIISGRGGNTFYADSVKGRFTI SRDNSKNTLYLQMNSLRAEDTALYYCASRLAVAG SEAFDIWGQGTMVTVSS |
| | | | SEQ ID NO: 24870 | SEQ ID NO: 28876 |
| iPS:393890 | 21-225_4B1 | NA | GACATCCAGATGACCCAGTCTCCATCCTCCCT GTCTGCATCTGTAGGCGACAGAGTCACCATCA CTTGCCGGGCAAGTCATACCATTAGAACCTAT TTAAACTGGTATCAGCAGAAACCAGGGAAAG CCCCTAAGCTCCTGATCTATGCTGCATCCAGTT TGCAAAGTGGGGTCCCATCAAGGATCAATGGC AGTGGATCTGGGACAGATTTCACTCTCACCAT CACCAATCTACAGCCTGAAGATTTTGCAACTT ACTACTGTCAACAGAGTTACAATATCTCATTC ACTTTCGGCCCTGGGACCAAAGTGGATATCAA A | CAGGTGCAGCTGCAGGAGTCGGGCCCAGGACTG GTGAAGCCTTCGGAGACCCTGTCCCTCACCTGTA GTGTCTCTGGTGACTCCATCAGTAGTTACTCCTG GAGCTGGATCCGGCAGCCCGCCGGGAAGGGACT GGAATGGATTGGGCGTATCTATACCAGTGGGAGC ACCAACTACATCCCCTCCCTCAAGAGTCGAATCA CCATGTCAGTAGACACGTCCAAGAAGCAGTTCTC CCTGAAGCTGAGCTCTGTGACCGCCGCGGACACG GCCGTGTATTACTGTGCGAGAGATTTGAAGAGCA GTGGCTGCCTTTTCTTTGACTACTGGGGCCAGGG AACCCTGGTCACCGTCTCCTCA |
| | | | SEQ ID NO: 24871 | SEQ ID NO: 28877 |
| | | AA | DIQMTQSPSSLSASVGDRVTITCRASHTIRTYLN WYQQKPGKAPKLLIYAASSLQSGVPSRINGSGSG TDFTLTITNLQPEDFATYYCQQSYNISFTFGPGTK VDIK | QVQLQESGPGLVKPSETLSLTCSVSGDSISSYSWSW IRQPAGKGLEWIGRIYTSGSTNYIPSLKSRITMSVDT SKKQFSLKLSSVTAADTAVYYCARDLKSSGCLFFD YWGQGTLVTVSS |
| | | | SEQ ID NO: 24872 | SEQ ID NO: 28878 |

FIGURE 50

| iPS:393892 | 21-225_6G7 | NA | GACATCCAGATGACCCAGTCTCCATCCTCCCG GTCTGCATCTGTAGGAGACAGAGTCACCATCA CTTGCCAGGCGAGTCAGGACATTAGCAACTAT TTAAATTGGTGTCAACAGAAACCAGGGAAAG CCCTTAAGCTCTTGATATACGATGCATCCACTT TGGAAACAGGGGTCCCCTCAAGGTTCAGTGGA AGTGGATCTGGGACAGATTTTACTTTCACCAT CAGCAGCGTGCAGCCTGAAGATATTGCAACAT ATTACTGCCAACAGTATGATAATGTCCCGATC ACCTTCGGCCAAGGGACACGACTGGAGATTAA A | CAGGTGCAGCTGGTGGAGTCTGGGGGAGGCGTG GTCCAGCCTGGGAGGTCCCTGAGACTCTCCTGTG CAGCCTCTGGATTTACCTTCAGTAGCTATGGCAT GCACTGGGTCCGCCAGACTCCAGGCAAGGGGCT GGAGTGGGTGGCAATTATATCATATGTTGGAAAG AATAAATATTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGCGAGACGGGGAAA CAGCTATGGCGGGTACGGTATGGACGTCTGGGG CCAAGGGACTACGGTCACCGTCTCCTCA |
| | | | SEQ ID NO: 24873 | SEQ ID NO: 28879 |
| | | AA | DIQMTQSPSSRSASVGDRVTITCQASQDISNYLN WCQQKPGKALKLLIYDASTLETGVPSRFSGSGS GTDFTFTISSVQPEDIATYYCQQYDNVPITFGQGT RLEIK | QVQLVESGGGVVQPGRSLRLSCAASGFTFSSYGMH WVRQTPGKGLEWVAIISYVGKNKYYADSVKGRFTI SRDNSKNTLYLQMNSLRAEDTAVYYCARRGNSYG GYGMDVWGQGTTVTVSS |
| | | | SEQ ID NO: 24874 | SEQ ID NO: 28880 |
| iPS:393894 | 21-225_5E11 | NA | GTCATCCAGATGACCCAGTCTCCATCCTCACT GTCTGCCTCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCAATGCTGCATCCAGTG TGCAGAGTGGGGTCCCATCAAAATTCAGCGGC AATGGATCTGGGACAGACTTCACTCTCACCAT CAGCAGCCTGCAGCCTGAGGATTTTGCAACTT ATTACTGCCACCAGTATCACAGTTACCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTACT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTTTTACTGTGCGAGAGTGGCTTCA TTTGACTACTGGGGCCAGGGAACCCTGGTCACCG TCTCCTCA |
| | | | SEQ ID NO: 24875 | SEQ ID NO: 28881 |

FIGURE 50

| | | | | |
|---|---|---|---|---|
| | | AA | VIQMTQSPSSLSASVGDRVTITCRASQGISNYLA WFQQKPGKAPKSLINAASSVQSGVPSKFSGNGS GTDFTLTISSLQPEDFATYYCHQYHSYPFTFGPG TKVDIK | EVQLVESGGGLVKPGGSLRLSCAASGFTFSSYSMN WVRQAPGKGLEWVSSISGSSTYIYYADSVKGRFTIS RDNAKNSLYLQMNSLRAEDTAVFYCARVASFDYW GQGTLVTVSS |
| | | | SEQ ID NO: 24876 | SEQ ID NO: 28882 |
| IPS:393896 | 21-225_2A4 | NA | GACATCCAGATGACCCAGTCTCCATCCTCACT GTTTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGCGCCTGATCTATACTGCATCCAGTTT GCAAAGTGGGGTCCCATCAAAGTTCAGCGGCA GTGGATTTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTACT TACATATACTACGCAGACTCAGTGAAGGGCCGA ATCGCCATCTCCAGAGACAACGCCAAGAACTCG CTGTATCTGCAAATGAACAGCCTGAGAGCCGAG GACACGGCTGTATATTACTGTGCGAGAGTGGCTT CATTTGACTACTGGGGCCAGGGAACCCTGGTCAC CGTCTCCTCA |
| | | | SEQ ID NO: 24877 | SEQ ID NO: 28883 |
| | | AA | DIQMTQSPSSLFASVGDRVTITCRASQGISNYLA WFQQKPGKAPKRLIYTASSLQSGVPSKFSGSGFG TDFTLTISSLQPEDFATYYCQQYNSYPFTFGPGT KVDIK | EVQLVESGGGLVKPGGSLRLSCAASGFTFSSYSMN WVRQAPGKGLEWVSSISGSSTYIYYADSVKGRIAIS RDNAKNSLYLQMNSLRAEDTAVYYCARVASFDY WGQGTLVTVSS |
| | | | SEQ ID NO: 24878 | SEQ ID NO: 28884 |

FIGURE 50

| | | | | |
|---|---|---|---|---|
| iPS:393898 | 21-225_5F7 | NA | GACATTCAGATGACCCAGTCTCCATCCTCCCC GTCTGCATCGTAGGAGACAGAGTCACCATCA CTTTCCGGGCAAGTCAGACCATTAGTAGTTAT TTAAATTGGTATCAGCAGAAACCAGGGAAAG CCCCTAAACTCCTGATCTCTGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAATACCCCCTTA TTCACTTTCGGCCCTGGGACCAAAGTGGTTAT CAAA | GAGGTGCAGTTGTTGGAGTCTGGGGGAGGCTTGG TACAGCCTGGGGGGGTCCCTGAGACTCTCCTGTGC AGCCTCTGAATTCACCTTTAGCAGCTATGCCATG AGCTGGGTCCGCCAGGCTCCAGGGAAGGGGCTG GAGTGGGTCTCAATTATTAGTGGTCGTGGTGGTA ACACATTCTACGCAGACTCCGTGAAGGGCCGGTT CACCATCTCCAGAGACAATTCCAAGAACACGCTG TATCTGCAAATGAACAGCCTGAGAGCCGAGGAC ACGGCCGTATATTACTGTGCGTCCCGTATAGCAG TGGCTGGCTCGGAGGCTTTTGCTATCTGGGGCCA AGGGACAATGGTCACCGTCTCTTCA |
| | | | SEQ ID NO: 24879 | SEQ ID NO: 28885 |
| | | AA | DIQMTQSPSSPSASVGDRVTITFRASQTISSYLNW YQQKPGKAPKLLISAASSLQSGVPSRFSGSGSGT DFTLTISSLQPEDFATYYCQQSYNTPLFTFGPGTK VVIK | EVQLLESGGGLVQPGGSLRLSCAASEFTFSSYAMS WVRQAPGKGLEWVSIISGRGGNTFYADSVKGRFTI SRDNSKNTLYLQMNSLRAEDTAVYYCASRIAVAGS EAFAIWGQGTMVTVSS |
| | | | SEQ ID NO: 24880 | SEQ ID NO: 28886 |
| iPS:393900 | 21-225_10E12 | NA | GACATCCAGATGACCCAGTCTCCATCCTCCCT GTCTGCATCTGTAGGAGACAGAGTCACCATCA CTTGCCGGGCAAGTCAGAACATTTACAGTTAT TTAAATTGGTATCAGGAGAAACCAGGGAGAG CCCCTAAGCTCCTGATCTATGCTACATCCAGTT TGCAAAGTGGGGTCCCATCAAGGTTCGGTGGC AGTGGATCTGGGACAGATTTCACTCTCACCAT CAGCAGTCTACAACCTGAAGATTTTGCAACTT ACTACTGTCAACAGAATTACAGTCCCCCTCTC ACTTTCGGCGGAGGGACCAAGGTGGAGATCG AA | CAGGTGCAGCTGGTGGAGTCGGGGGGAGGCGTG GTCCAGCCTGGGAGGTCCCTGAGACTCTCCTGTG CAGCGTCTGGATTCAACTTCAGTAACTATGGCAT GCACTGGGTCCGCCAGGTTCCAGGCAAGGGGCT GGAGTGGGTGGCAGTTATATGGCATGATGGAAG TAATAAATACTATGTAGACTCCGTGAAGGGCCGA TTCACCATCTCCAGAGACAATTCCAAGAACACGC TGTCTCTGCAAATGAACAGCCTGAGAGCCGAGG ACACGGCTGTGTATTGCTGTGCGAGAGATGAGAG GCTGGGGGATTTTTGACTACTGGGGCCAGGGAACC CTGGTCACCGTCTCCTCA |
| | | | SEQ ID NO: 24881 | SEQ ID NO: 28887 |

FIGURE 50

| | | | | |
|---|---|---|---|---|
| | | AA | DIQMTQSPSSLSASVGDRVTITCRASQNIYSYLN WYQEKPGRAPKLLIYATSSLQSGVPSRFGGSGSG TDFTLTISSLQPEDFATYYCQQNYSPPLTFGGGT KVEIE | QVQLVESGGGVVQPGRSLRLSCAASGFNFSNYGM HWVRQVPGKGLEWVAVIWHDGSNKYYVDSVKGR FTISRDNSKNTLSLQMNSLRAEDTAVYCCARDERL GIFDYWGQGTLVTVSS |
| | | | SEQ ID NO: 24882 | SEQ ID NO: 28888 |
| iPS:393902 | 21-225_14E10 | NA | GACATCCAGATGACCCAGTCTCCATCCTCCCT GTCTGCATCTGTAGGAGACCGAGTCACCATCA CTTGCCGGACAAGTCAGGGCATTAGAAATGAT TTAGGCTGGTATCAGCACAAACCAGGGCAAGC CCCTAAGCGCCTGATCTATGCTGCATCCAGTTT ACAAAGTGGGGTCCCATCAAGGTTCAGCGGCA GTGGATCTGGGACAGAATTCACTCTCACAATC AGCAGCCTGCAGCCTGAAGATTTTACAGCTTA TTACTGTCTGCAGCATTATAGTTACCCTCGGAC GTTCGGCCAAGGGACCAAGGTGGAAATCAAG | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TCTATCTGCAAATGAACAGCCTGAGAGCCGAGG ACACGGCTGTGTATTACTGTGCGAGAGAGAAGTA TAGCAGCAGCTGGTACGACTACGGTATGGACGTC TGGGGCCAAGGGACCACGGTCACCGTCTCCTCA |
| | | | SEQ ID NO: 24883 | SEQ ID NO: 28889 |
| | | AA | DIQMTQSPSSLSASVGDRVTITCRTSQGIRNDLG WYQHKPGQAPKRLIYAASSLQSGVPSRFSGSGS GTEFTLTISSLQPEDFTAYYCLQHYSYPRTFGQG TKVEIK | QVQLVESGGGVVQPGRSLRLSCAASGFTFSDYGM HWVRQAPGKGLEWVAVIWYDGSNKYYADSVKGR FTISRDNSKNTLYLQMNSLRAEDTAVYYCAREKYS SSWYDYGMDVWGQGTTVTVSS |
| | | | SEQ ID NO: 24884 | SEQ ID NO: 28890 |

FIGURE 50

| | | | | |
|---|---|---|---|---|
| iPS:393904 | 21-225_8H11 | NA | GACATCCAGATGACCCAGTCTCCATCCTCCCT GTCTGCATTTGTAGGAGACAGAGTCACCATCA CTTGCCGGGCAAGTCAGAACATTATCAGCTAT TTAAATTGGTATCAACAGAAACCAGGGAAAG CCCCTAACCTCATGATATATGTTACATCCAGTT TGCACAGTGGGGTCCCATCAAGGTTCAGTGGC AGTGGATCTGGGACAGATTTCTCTCTCACCAT CAGCAGTCTCCAACCTGAGGATTTTGCAACTT ACTACTGTCAACAGAGTTACAGTACCCCTTTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATAACAT GCACTGGGTCCGCCAGGCTCCAGGCAAGGGGCT GGAGTGGGTGGCAGTTATATGGTATGATGGAAGT GATAGATACTCTGCAGACTCCGTGAAGGGCCGAT TCACCATCTCCCGAGACAATTCCAAGAACACGCT GTATCTGCAAATGAACAGCCTGAGAGCCGAGGA CACGGCTGTGTATTACTGTGCGAGAGATCGGGCC TATAGCAGCTCGTCTGACTTCTGGGGCCAGGGAA CCCTGGTCACCGTCTCCTCA |
| | | | SEQ ID NO: 24885 | SEQ ID NO: 28891 |
| | | AA | DIQMTQSPSSLSAFVGDRVTITCRASQNIISYLNW YQQKPGKAPNLMIYVTSSLHSGVPSRFSGSGSGT DFSLTISSLQPEDFATYYCQQSYSTPFTFGPGTKV DIK | QVQLVESGGGVVQPGRSLRLSCAASGFTFSTYNMH WVRQAPGKGLEWVAVIWYDGSDRYSADSVKGRF TISRDNSKNTLYLQMNSLRAEDTAVYYCARDRAYS SSSDFWGQGTLVTVSS |
| | | | SEQ ID NO: 24886 | SEQ ID NO: 28892 |
| iPS:393906 | 21-225_13D3 | NA | GAAATAGTGATGACGCAGTCTCCAGCCACCCT GTCTGTGTCTCCAGGGGAAAGCGCCACCCTCT CCTGCAGGGCCAGTCAGACTGTTAGCAGCAAC TTAGCCTGGTTCCAGCAGAAGCCTGGCCAGGC TCCCAGGCTCCTCATCAATGGTGCATCCACCA GGGCCACTGGTATCCCAGCCAGGTTCAGTGGC AGTGGGTCTGGGACAGAGTTCACTCTCACCAT CAGCAGCCTGCAGTCTGAAGATTTTGCAGTTT ATTTCTGTCAGCAGTATCATGACTGGCCTCCG ACGTTCGGCCAAGGGACCAAGGTGGAAATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGTTATACCAT GAACTGGGTCCGCCAGGCTCCAGGGAAGGGGCT GGACTGGGTCTCATCCATTAGTGGTAGTAGTAGT TACATATACTACGCGGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATCGGGGC AGTGGCTGGGGCCAGGGAACCCTGGTCACCGTCT CCTCA |
| | | | SEQ ID NO: 24887 | SEQ ID NO: 28893 |

FIGURE 50

| | | | | |
|---|---|---|---|---|
| | | AA | EIVMTQSPATLSVSPGESATLSCRASQTVSSNLA WFQQKPGQAPRLLINGASTRATGIPARFSGSGSG TEFTLTISSLQSEDFAVYFCQQYHDWPPTFGQGT KVEIK | EVQLVESGGGLVKPGGSLRLSCAASGFTFSSYTMN WVRQAPGKGLDWVSSISGSSSYIYYADSVKGRFTIS RDNAKNSLYLQMNSLRAEDTAVYYCARDRGSGW GQGTLVTVSS |
| | | | SEQ ID NO: 24888 | SEQ ID NO: 28894 |
| iPS:393908 | 21-225_10E9 | NA | GACATCCAGATGACCCAGTCTCCATCCTCCCT GTCTGCATCTGTAGGAGACAGAGTCACCTTCA CTTGCCGGGCAAGTCAGGACATTAGAAGTGAT TTAGGCTGGTATCAGCAGAAACCAGGGAAAG CCCCTACGCGCCTGATCTTTGCTGCATCCAGTT TGCAAAGTGGGGTCCCATCAAGGTTCAGCGGC AGTGGATCTGGGACAGAATTCACTCTCACAAT CAACAGCCTGCAGCCTGAAGATTTTGCAACTT ATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT ACACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAAAAGTA TAGCAGCAGCTGGTACGACTACGGTATGGACGTC TGGGGCCAAGGGACCACGGTCACCGTCTCCTCA |
| | | | SEQ ID NO: 24889 | SEQ ID NO: 28895 |
| | | AA | DIQMTQSPSSLSASVGDRVTFTCRASQDIRSDLG WYQQKPGKAPTRLIFAASSLQSGVPSRFSGSGSG TEFTLTINSLQPEDFATYYCLQHYSYPRTFGQGT KVEIK | QVQLVESGGGVVQPGRSLRLSCAASGFTFSNYVIH WVRQAPGKGLEWVAVIWYDGSNKYYADSVKGRF TISRDNSKNTLYLQMNSLRAEDTAVYYCAREKYSS SWYDYGMDVWGQGTTVTVSS |
| | | | SEQ ID NO: 24890 | SEQ ID NO: 28896 |

FIGURE 50

| iPS:393910 | 21-225_15F10 | NA | GACATCCAGATGACCCAGTCTCCATCCTCCCT GTCTGCTTCTGTAGGAGACAGAGTCACCATCA CTTGCCAGGCGAATCAGGACATTACCAACTTT TTAAAATTGGTATCAGCTGAAACCAGGGAAAGC CCCTAACCTCCTGATCTCCGATGCATCCAATTT GGAAACAGGGGTCCCATCAAGGTTCAGTGGA AGTGGATCTGGGACAGATTTTACTTTCACCAT CAGCAGCCTGCAGCCTGAAGATGTTGCGACAT ATTACTGTCAACAGTATGATAATCTCCCGATC ACCTTCGGCCAAGGGACACGACTGGAGATTAA A | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGTCAGTTATATCATATGGTGGAAGT AATAATTACTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGTGAGACGGGGATAC AGCTATGGCGGGTACGGTATGGACGTCTGGGGC CAAGGGACCACGGTCACCGTCTCCTCA |
|---|---|---|---|---|
| | | | SEQ ID NO: 24891 | SEQ ID NO: 28897 |
| | | AA | DIQMTQSPSSLSASVGDRVTITCQANQDITNFLN WYQLKPGKAPNLLISDASNLETGVPSRFSGSGSG TDFTFTISSLQPEDVATYYCQQYDNLPITFGQGT RLEIK | QVQLVESGGGVVQPGRSLRLSCAASGFTFSSYGMH WVRQAPGKGLEWVSVISYGGSNNYYADSVKGRFT ISRDNSKNTLYLQMNSLRAEDTAVYYCVRRGYSY GGYGMDVWGQGTTVTVSS |
| | | | SEQ ID NO: 24892 | SEQ ID NO: 28898 |
| iPS:393912 | 21-225_16F6 | NA | GACATCCAGATGACCCAGTCTCCATCCTCCCT GTCTGCTTCTGTAGGAGACAGAGTCACCATCA CTTGCCAGGCGAATCAGGACATTACCAACTTT TTAAAATTGGTATCAGCTGAAACCAGGGAAAGC CCCTAACCTCCTGATCTCCGATGCATCCAATTT GGAAACAGGGGTCCCATCAAGGTTCAGTGGA AGTGGATCTGGGACAGATTTTACTTTCACCAT CAGCAGCCTGCAGCCTGAAGATGTTGCGACAT ATTACTGTCAACAGTATGATAATCTCCCGATC ACCTTCGGCCAAGGGACACGACTGGAGATTAA A | CAGGTGCAGTTGGTGGAGTCTGGGGGAGGCGTG GTCCAGCCTGGGAGGTCCCTGAGACTCTCCTGTG CAGCGTCTGGATTCACCTTCAGTAACTATGGCAT GCACCGGGTCCGCCAGGCTCCAGGCAAGGGGCT GGAGTGGGTGGCAGTTATATGGTATGAAGGAAG TAATCAATACTATGGAGACTCCGTGAAGGGCCG ATTCACCATCTCCAGAGACAATTCCAAGAACACG GTGTATCTGCAAATGCACAGCCTGAGAGCCGAG GACACGGCTGTGTATTACTGTGCGAGAGAGCTGG GGTTCCTCTCTGATTACTGGGGCCAGGGAACCCT GGTCACCGTCTCCTCA |
| | | | SEQ ID NO: 24893 | SEQ ID NO: 28899 |

FIGURE 50

| | | | | |
|---|---|---|---|---|
| | | AA | DIQMTQSPSSLSASVGDRVTITCQANQDITNFLN WYQLKPGKAPNLLISDASNLETGVPSRFSGSGSG TDFTFTISSLQPEDVATYYCQQYDNLPITFGQGT RLEIK | QVQLVESGGGVVQPGRSLRLSCAASGFTFSNYGM HRVRQAPGKGLEWVAVIWYEGSNQYYGDSVKGR FTISRDNSKNTVYLQMHSLRAEDTAVYYCARELGF LSDYWGQGTLVTVSS |
| | | | SEQ ID NO: 24894 | SEQ ID NO: 28900 |
| IPS:393914 | 21-225_16B8 | NA | GACATCCAGATGACCCAGTCTCCATCCTCACT GTCTGCCTCTGTAGGAGACAGAGTCACCATCA CTTGTCGGGCGAGTCAGGGCATTAACAATTAT TTAGCCTGGTTTCAGCAGAAACCAGGGAAAGC CCTTAAGTCCCTGATCAATGCTGCATCCAGTG TGCAGAGTGGGGTCCCATCAAAGTTCAGCGGC AGTGGATCTGGGACAGACTTCACTCTCACCAT CAGCAGCCTGCAGCCTGAAGATTTTGCAACTT ATTACTGCCACCAGTATCACAGTTACCCATTC ACTTTCGGCCCTGGGACCAAAGTGGATATCGT A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGATCTCATCCATTAGTGGTAGTAGTACT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTGGCTTCA TTCGACTACTGGGGCCAGGGAACCCTGGTCACCG TCTCCTCA |
| | | | SEQ ID NO: 24895 | SEQ ID NO: 28901 |
| | | AA | DIQMTQSPSSLSASVGDRVTITCRASQGINNYLA WFQQKPGKALKSLINAASSVQSGVPSKFSGSGS GTDFTLTISSLQPEDFATYYCHQYHSYPFTFGPG TKVDIV | EVQLVESGGGLVKPGGSLRLSCAASGFTFSSYSMN WVRQAPGKGLEWISSISGSSTYIYYADSVKGRFTIS RDNAKNSLYLQMNSLRAEDTAVYYCARVASFDY WGQGTLVTVSS |
| | | | SEQ ID NO: 24896 | SEQ ID NO: 28902 |

FIGURE 50

| iPS:393916 | 21-225_2G4 | NA | GACATCCAGATGACCCAGTCTCCATCCTCCCT GTCTGCTTCTGTAGGCGACAGAGTCACCATCA CTTGCCGGGCAAGTCAGGGCATTAGAAATGAT TTAGGCTGGTATCAGCAGAAACCAGGGAAAG CCCCTACGCGCCTGATCTATGCTGCATCCAGTT TGCACAGTGGGGTCCCATCACGGTTCAGCGGC AGTGGATCTGGGACAGAATTCACTCTCACAAT CAGCAGCCTGCAGCCTGAAGATCTTGCAACTT ATTACTGTCTACAACATTATAGTTTCCCTCGGA CGTTCGGCCGAGGGACCAAGGTGGAAATCAA A | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAAGTA TAGCAGCAGCTGGTACGACTACGGTATGGACGTC TGGGGCCACGGGACCACGGTCACCGTCTCCTCA |
|---|---|---|---|---|
| | | | SEQ ID NO: 24897 | SEQ ID NO: 28903 |
| | | AA | DIQMTQSPSSLSASVGDRVTITCRASQGIRNDLG WYQQKPGKAPTRLIYAASSLHSGVPSRFSGSGSG TEFTLTISSLQPEDLATYYCLQHYSFPRTFGRGTK VEIK | QVQLVESGGGVVQPGRSLRLSCAASGFTFSSYVMH WVRQAPGKGLEWVAVIWYDGSNKYYADSVKGRF TISRDNSKNTLYLQMNSLRAEDTAVYYCAREKYSS SWYDYGMDVWGHGTTVTVSS |
| | | | SEQ ID NO: 24898 | SEQ ID NO: 28904 |
| iPS:393920 | 21-225_1H12 | NA | GACATCCAGATGACCCAGTCTCCATCCTCCCT GTCTGCATCTGTTGGAGACAGAGTCACCATCA CTTGCCGGGCAAGTCAGAACATTTACAGGTAT TTAAATTGGTATCAGGAGAAACCAGGGAGAG CCCCTAAGCTCCTGATCTATACTGCATCCAGTT TACAAAGTGGGGTCCCATCAAGGTTCAGTGGC AGTGACTCTGGGACAGATTTCACTCTCACCAT CAGCAGTCTGCAACCTGAAGATTTTGCAACTT ACTACTGTCAACAGAGTTACAGTCCCCCTCTC ACTTTCGGCGGAGGGACCAAGGTGGAGATCA AA | CAGGTGCAGCTGGTGGAGTCTGGGGGAGGCGTG GTCCAGTCTGGGAGGTCCCTGAGACTCTCCTGTG CAGCGTCTGGATTCAACTTCAGTAGCTATGGCAT GCACTGGGTCCGCCAGGCTCCAGGCAAGGGGCT GGAGTGGGTGGCAATTATATGGCATGATGGAAG TAATAAATACTATGTAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATGAGAG GCTGGGGATTTTTGACTACTGGGGCCAGGGAACC CTGGTCACCGTCTCCTCA |
| | | | SEQ ID NO: 24899 | SEQ ID NO: 28905 |

FIGURE 50

| | | | | |
|---|---|---|---|---|
| | | AA | DIQMTQSPSSLSASVGDRVTITCRASQNIYRYLN WYQEKPGRAPKLLIYTASSLQSGVPSRFSGSDSG TDFTLTISSLQPEDFATYYCQQSYSPPLTFGGGTK VEIK | QVQLVESGGGVVQSGRSLRLSCAASGFNFSSYGMH WVRQAPGKGLEWVAIIWHDGSNKYYVDSVKGRFT ISRDNSKNTLYLQMNSLRAEDTAVYYCARDERLGI FDYWGQGTLVTVSS |
| | | | SEQ ID NO: 24900 | SEQ ID NO: 28906 |
| IPS:393922 | 21-225_2B2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGCAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATCTTGCAACT TATCACTGTCTACAGCATAATAGTTACCCGCT CACTTTCGGCGGAGGGACCAAGGTGGAGATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGAGGAAAA TAATAAATACTATGTAGACTCCGTGAAGGGCCGA TTCACCATCTCCAGAGACAATTCCAAGAGCACGC TGTATCTGCAAATGAACAGCCTGAGAGCCGAGG ACACGGCTGTGTATTACTGTGTGAGAGAACTAGG CTTCCAGTCTGACTACTGGGGCCAGGGAACCCCG GTCACCGTCTCCTCA |
| | | | SEQ ID NO: 24901 | SEQ ID NO: 28907 |
| | | AA | DIQMTQSPSSLSASVGDRVTITCRASQGIRNDLG WYQQKPGQAPKRLIYAASSLQSGVPSRFSGSGS GTEFTLTISSLQPEDLATYHCLQHNSYPLTFGGG TKVEIK | QVQLVESGGGVVQPGRSLRLSCAASGFTFSSYGMH WVRQAPGKGLEWVAVIWYEENNKYYVDSVKGRF TISRDNSKSTLYLQMNSLRAEDTAVYYCVRELGFQ SDYWGQGTPVTVSS |
| | | | SEQ ID NO: 24902 | SEQ ID NO: 28908 |

FIGURE 50

| iPS:393926 | 21-225_4G4 | NA | GACATCCAGATGACCCAGTCTCCATCCTCCCT GGCTGCATCTGTAGGAGACAGAGTCACCATCA CTTGCCGGGCAAGTCAGACCATTATCAGCTAT TTAAATTGGTATCAGCAGAAACCAGGGAAAG CCCCTAAACTTCTGATCCATACTGCATCCAGTT TGCAAAGTGGGGTCCCATCAAGGTTCAGTGGC AGTGGATCTGGGACAGATTTCACTCTCTCCAT CAGCAGTCTGCAACCTGAAGATTTTGCAACTT ACTACTGTCAACAGACTTACAGTACTCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCCGGATTCACCTTCAGTAGCTATGGCAT GCACTGGGTCCGCCAGGCTCCAGGCAAGGGGCT GGAGTGGGGGGCAGTTATATGGCATGATGGAAG TAATAAATACTATGCAGACTCCGTGAAGGGCCG ATTCACCATCTCCAGAGACAATGCCAAGAACAC GCTGTATCTGCAAATGAACAGCCTGAGAGCCGA GGACACGGCTGTGTATTACTGTGCGAGAGATCTG AGAATGGGCGGTATGGACGTCTGGGGCCAAGGG ACCACGGTCACCGTCTCCTCA |
|  |  |  | SEQ ID NO: 24903 | SEQ ID NO: 28909 |
|  |  | AA | DIQMTQSPSSLAASVGDRVTITCRASQTIISYLNW YQQKPGKAPKLLIHTASSLQSGVPSRFSGSGSGT DFTLSISSLQPEDFATYYCQQTYSTPLTFGGGTK VEIK | QVQLVESGGGVVQPGRSLRLSCAASGFTFSSYGMH WVRQAPGKGLEWGAVIWHDGSNKYYADSVKGRF TISRDNAKNTLYLQMNSLRAEDTAVYYCARDLRM GGMDVWGQGTTVTVSS |
|  |  |  | SEQ ID NO: 24904 | SEQ ID NO: 28910 |
| iPS:393928 | 21-225_4E10 | NA | GACATCCAGATGACCCAGTCTCCATCCTCCCT GTTTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATTTGGGACAGAATTCACTCTCTCACAA TCAGCAGCCTGCAGCTTGAAGATTTTGCAACT TATTACTGTTTACAGCATGATAATTACCCTCTC ACTTTCGGCGGAGGGACCAAGGTGGAGATCA AA | CAGCTGCAGCTACAGGAGTCGGGGCCCAGGACTG GTGAAGCCTTCGGAGACCCTGTCCCTCACCTGCA CTGTCTCTGGTGGCTCCATCAGCCGTAGTAGTTA CTACTGGGGCTGGATCCGCCAGCCCCCAGGGAA GGGGCTGGAGTGGATTGGGAGTGTCTATTATAGT GGGGGCCACCTCCTACAACCCGTCCCTCAAGAGTC GAGTCACCATATCCGTAGACACGTCCAAGAACC AGTTTTCCCTGAAGCTGAACTCTGTGACCGCCGC AGACACGGCTTTGTATTACTGTGTGAGACTAAGC AGCAACTGGGACTTTGACTACTGGGGCCAGGGA ACCCTGTTCACCGTCTCCTCA |
|  |  |  | SEQ ID NO: 24905 | SEQ ID NO: 28911 |

FIGURE 50

| IPS:393930 | 21-225_7E11 | AA | DIQMTQSPSSLFASVGDRVTITCRASQGIRNDLG WYQQKPGKAPKRLIYAASSLQSGVPSRFSGSGF GTEFTLTISSLQLEDFATYYCLQHDNYPLTFGGG TKVEIK | QLQLQESGPGLVKPSETLSLTCTVSGGSISRSSYYW GWIRQPPGKGLEWIGSVYYSGATSYNPSLKSRVTIS VDTSKNQFSLKLNSVTAADTALYYCVRLSSNWDF DYWGQGTLFTVSS |
|---|---|---|---|---|
| | | | SEQ ID NO: 24906 | SEQ ID NO: 28912 |
| | | NA | GACATCCAGATGACCCAGTCTCCATCCTCCCT GTCTGCATCTGTAGGAGACAGAGTCACCATCG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATATGGCATGATGGAAG TAATAAATACTATGCAGACTCCGTGAAGGGCCG ATTCACCATCTCCAGAGACAATTCCAACAACACG CTGTATCTGCAAATGAGCAGCCTGCGAGCCGAG GACACGGCTGTGTATTACTGTGCGAGAGATCTGA GTATGGGCGGTATGGACGTCTGGGGCCAAGGGA CCACGGTCACCGTCTCCTCA |
| | | | SEQ ID NO: 24907 | SEQ ID NO: 28913 |
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHDGSNKYYADSVKGRFT ISRDNSNNTLYLQMSSLRAEDTAVYYCARDLSMGG MDVWGQGTTVTVSS |
| | | | SEQ ID NO: 24908 | SEQ ID NO: 28914 |

FIGURE 50

| iPS:393932 | 21-225_10F5 | NA | GACATCCAGATGACCCAGTCTCCATCCTCCCT GTCTGCATCTGTTGGAGACAGAGTCACCATCA CTTGCCGGGCAAGTCAGAACATTTACAGGTAT TTAAAATTGGTATCAGGAGAAACCAGGGAGAG CCCCTAAACTCCTGATCTATACTGCATCCAGTT TGCAAAGTGGGGTCCCATCAAGGTTCAGTGGC AGTGACTCTGGGACAGATTTCACTCTCACCAT CAGCAGTCTGCAACCTGAAGATTTTGCAACTT ACTACTGTCAACAGAGTTACAGTCCCCCTCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCAACTTCAGTAGCTATGGCAT GCACTGGGTCCGCCAGGCTCCAGGCAAGGGGCT GGAGTGGGTGTCAATTATATGGCATGATGGAAGT AATAAATATTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGATGAGAGG CTGGGGATTTTTGACTACTGGGGCCAGGGAACCC TGGTCACCGTCTCCTCG |
|---|---|---|---|---|
| | | | SEQ ID NO: 24909 | SEQ ID NO: 28915 |
| | | AA | DIQMTQSPSSLSASVGDRVTITCRASQNIYRYLN WYQEKPGRAPKLLIYTASSLQSGVPSRFSGSDSG TDFTLTISSLQPEDFATYYCQQSYSPPLTFGGGTK VEIK | QVQLVESGGGVVQPGRSLRLSCAASGFNFSSYGMH WVRQAPGKGLEWVSIIWHDGSNKYYVDSVKGRFT ISRDNSKNTLYLQMNSLRAEDTAVYYCARDERLGI FDYWGQGTLVTVSS |
| | | | SEQ ID NO: 24910 | SEQ ID NO: 28916 |
| iPS:393934 | 21-225_13E6 | NA | GACATCCAGGTGACCCAGTCTCCATCTTCCCT GTCTGCATCTGTAGGAGACAGAGTCACCATCA CTAGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTGTACAGCATAATAGTTACCCGCT CACTTTCGGCGGAGGGACCAAGGTGGCGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGAAAAT AATAAATATTATATAGACTCCGTGAAGGGCCGAT TCACCATCTCCAGAGACAACTCCAAGAACACGCT GTATCTGCAAATGAACAGCCTGAGAGCCGAGGA CACGGCTGTGTATTACTGTGCGAGAGAATTGGGG TTCCGGTCTGACTACTGGGGCCAGGGAACCCTGG TCACCGTCTCCTCA |
| | | | SEQ ID NO: 24911 | SEQ ID NO: 28917 |

FIGURE 50

| | | | | |
|---|---|---|---|---|
| | | AA | DIQVTQSPSSLSASVGDRVTITSRASQGIRNDLG WYQQKPGKAPKRLIYAASSLQSGVPSRFSGSGS GTEFTLTISSLQPEDFATYYCVQHNSYPLTFGGG TKVAIK | QVQLVESGGGVVQPGRSLRLSCAASGFTFSNYGM HWVRQAPGKGLEWVAVIWYDENNKYYIDSVKGR FTISRDNSKNTLYLQMNSLRAEDTAVYYCARELGF RSDYWGQGTLVTVSS |
| | | | SEQ ID NO: 24912 | SEQ ID NO: 28918 |
| iPS:393936 | 21-225_14A11 | NA | GACATCCAGATGACCCAGTCTCCATCCTCCCT GTCTGCATCTATAGGAGACAGAGTCACCATCA CTTGCCGGGCAAGTCAGAGCATTAGCAGTTAT TTAAATTGGTATCAGCAGAAACCAGGGAAAG CCCCTAAGCTCCTGATCTTTGCTGCATCCAGTT TGCAAAATGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGACTTACAGTAGCCCTCCA TTCACTTTCGCCCCTGGGACCAAAGTGGATAT CAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGTTATTAGTGGTAGAGGTGGT AGTACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAAAGGATAG CAGCTGGTATGGAGTACTTCGATCTCTGGGGCCG TGGCACCCTGGTCACTGTCTCCTCA |
| | | | SEQ ID NO: 24913 | SEQ ID NO: 28919 |
| | | AA | DIQMTQSPSSLSASIGDRVTITCRASQSISSYLNW YQQKPGKAPKLLIFAASSLQNGVPSRFSGSGSGT DFTLTISSLQPEDFATYYCQQTYSSPPFTFAPGTK VDIK | EVQLLESGGGLVQPGGSLRLSCAASGFTFSSYAMN WVRQAPGKGLEWVSVISGRGGSTYYADSVKGRFTI SRDNSKNTLYLQMNSLRAEDTAVYYCAKRIAAGM EYFDLWGRGTLVTVSS |
| | | | SEQ ID NO: 24914 | SEQ ID NO: 28920 |

FIGURE 50

| | | | | |
|---|---|---|---|---|
| iPS:393940 | 21-225_16B2 | NA | GGCGTCCAGATGACCCAGTCTCCATCCTCCCT GTCTGCATCTGTAGGAGACAGAGTCACCATCA CTTGCCGGGCAAGTCAGAGCATTAGCGGCTAT TTAAATTGGTATCAGCAGAAACCAGGGAAAG CCCCTAAGCTCCTGATCTATGCTGCATCCAGTT TGCAAAGTGGGGTCCCATCAAGGTTCAGTGGC GGTGGATCTGGGACAGATTTCACTCTCACCAT CAGCAGTCTACAACCTGAAGATTTTGCAACTT ACTACTGTCAACAGACTTACAATACCCCTCCG GAGCGCAGTTTTGGCCAGGGGACCAAGCTGG AGATCAAA | GAGGTGCAGTTGTTGGAGTCTGGGGGAGGCTTGG TACAGCCGGGGGGGTCCCTGAGACTCTCCTGTGC AGCCTCTGGATTCACCTTTAGCAGCTATGCCATG ACCTGGGTCCGCCAGGCTCCAGGGAAGGGGCCG GAGTGGGTCTCAGTTATTAGTGGTAGTGGTGGTA GCACATTCTACGCAGACTCCGTGAAGGGCCGGTT CACCATCTCCAGAGACAATTCCAAGAACACGCTT TATCTGCAAATGAGCAGCCTGAGAGCCGAGGAC ACGGCCGTTTATTTCTGTGCCCGATATTGTAGTA GTACCAGGTGCCCTTATGATGCCTTTGATATCTG GGGCCAAGGGACAATGGTCACCGTCTCCTCA |
| | | | SEQ ID NO: 24915 | SEQ ID NO: 28921 |
| | | AA | GVQMTQSPSSLSASVGDRVTITCRASQSISGYLN WYQQKPGKAPKLLIYAASSLQSGVPSRFSGGGS GTDFTLTISSLQPEDFATYYCQQTYNTPPERSFG QGTKLEIK | EVQLLESGGGLVQPGGSLRLSCAASGFTFSSYAMT WVRQAPGKGPEWVSVISGSGGSTFYADSVKGRFTI SRDNSKNTLYLQMSSLRAEDTAVYFCARYCSSTRC PYDAFDIWGQGTMVTVSS |
| | | | SEQ ID NO: 24916 | SEQ ID NO: 28922 |
| iPS:393942 | 21-225_11E5 | NA | GACATCGTGATGACCCAGTCTCCAGACTCCCT GGCTGTGTCTCTGGGCGAGAGGGCCACCCTCA ACTGCAAGTCCAGCCAGAGTGTTTTATACAGC TCCAACAATAACAACTACTTAACTTGGTACCA GCAGAAACCTGGACAGCGACCTAAGTTGCTCA TTTACTGGGCATCTACCCGGGAATCCGGGGTC CCTGACCGATTCAGTGGCAGCGGGTCTGGGAC AAATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAACTGGTGCAGTCTGGGCCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TGCCACCGGCTATGCACAGAGGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGACAAGCAGT GGCTGGGAGGTCTTTGACTACTGGGGCCAGGGA ACCCTGGTCACCGTCTCCTCA |
| | | | SEQ ID NO: 24917 | SEQ ID NO: 28923 |

FIGURE 50

| | | | | |
|---|---|---|---|---|
| | | AA | DIVMTQSPDSLAVSLGERATLNCKSSQSVLYSSN NNNYLTWYQQKPGQRPKLLIYWASTRESGVPD RFSGSGSGTNFTLTISSLQAEDVAVYYCQQYYST PPTFGQGTKVEIK | QVQLVQSGPEVKKPGASVKVSCKASGYTFTNYDIN WVRQATGQGLEWMGWMHPNSGATGYAQRFQGR VTMTRNTSISTAYMELSSLRSEDTAVYYCATSSGW EVFDYWGQGTLVTVSS |
| | | | SEQ ID NO: 24918 | SEQ ID NO: 28924 |
| IPS:393944 | 21-225_14D6 | NA | GACATCCAAATGACCCAGTCTCCATCCTCCCT GTCTGCATCTGTCGGAGACAGTGTCACCATCA CTTGCCGGGCAAGTCAGGACATTAGAAATCAT TTAGGCTGGTATCAGCATAAACCAGGGAAAGC CCCTAAGCGCCTGATCTATGCTGCATCCAGTTT GCAAAGTGGGGTCCCATCTAGGTTCAGCGGCA GTGGATCTGGGACAGAATTCACTCTCACAATC AGCAGCCTGCAGCCTGAAGATTTTGCAACTTA TTACTGTCTACAGTATAATAGTTACCCATTCAC TTTCGGCCCTGGGACCAAAGTGGATATCAAC | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGTTATACCAT GAACTGGGTCCGCCAGGCTCCAGGGAAGGGGCT GGAGTGGGTCTCATCCATTAGTGGTAGTGGTAGT TACATATACTACGCAGACTCAGTGAAGGGCCGAT TCACCATGTCCAGAGACAACGCCAAGAACTCACT GTATCTGCAAATGAACAGCCTGAGAGCCGAGGA CACGGCTGTGTATTACTGTGCGAGAGTAGCAGCC TTTGACTCCTGGGGCCAGGGAACCCTGGTCTCCG TCTCCTCA |
| | | | SEQ ID NO: 24919 | SEQ ID NO: 28925 |
| | | AA | DIQMTQSPSSLSASVGDSVTITCRASQDIRNHLG WYQHKPGKAPKRLIYAASSLQSGVPSRFSGSGS GTEFTLTISSLQPEDFATYYCLQYNSYPFTFGPGT KVDIN | EVQLVESGGGLVKPGGSLRLSCAASGFTFSSYTMN WVRQAPGKGLEWVSSISGSGSYIYYADSVKGRFTM SRDNAKNSLYLQMNSLRAEDTAVYYCARVAAFDS WGQGTLVSVSS |
| | | | SEQ ID NO: 24920 | SEQ ID NO: 28926 |

FIGURE 50

| | | | | |
|---|---|---|---|---|
| iPS:393946 | 21-225_16A4 | NA | GACATCCAGATGACCCAGTCTCCATCCTCACT GTCTGCATCTGTAGGAGACAGAGTCACCATCA CTTGTCGGGCGAGTCAGGACATTAGTAATTAT TTAGCCTGGTTTCAGCAGAAACCAGGGAAAGC CCCTAAGTCCCTGATCTCTGCTGCATCCAGTTT GCAAAGTGGGGTCCCATCAAAATTCAGCGGCA GTGGATCTGGGACAGATTTCACTCTCACCATC AGCAGCCTGCAGCCTGAAGATTTTGCAACTTA TTACTGCCAACAGTATCATAGTTACCCGTGGA CGTTCGGCCAAGGGACCAAGGTGGAAATCAA T | GAGGTGCAGCTGGTGGAGTCTGGGGGAGGCCTG GTCAAGCCTGGGGGGTCCCTGAGACTCTCCTGTG CAGCCTCTGGATTCACCTTCAGCAGCTATAGCAT GAACTGGGTCCGCCAGGCTCCAGGGAAGGGGCT GGAGTGGGTCTCATCCATTAGTGGTAGTAGTACT TACAAATACTACGCAGACTCAGTGAAGGGCCGA TTCACCATCTCCAGAGACAACGCCAAGAACTCAC TGTATCTGCAAATGAACAGCCTGAGAACCGAGG ACACGGCTGTGTATTACTGTGCGAGAGATCGTGG GAGCTACTGGGGCCAGGGAACCCAGGTCACCGT CTCCTCA |
| | | | SEQ ID NO: 24921 | SEQ ID NO: 28927 |
| | | AA | DIQMTQSPSSLSASVGDRVTITCRASQDISNYLA WFQQKPGKAPKSLISAASSLQSGVPSKFSGSGSG TDFTLTISSLQPEDFATYYCQQYHSYPWTFGQGT KVEIN | EVQLVESGGGLVKPGGSLRLSCAASGFTFSSYSMN WVRQAPGKGLEWVSSISGSSTYKYYADSVKGRFTI SRDNAKNSLYLQMNSLRTEDTAVYYCARDRGSYW GQGTQVTVSS |
| | | | SEQ ID NO: 24922 | SEQ ID NO: 28928 |
| iPS:393948 | 21-225_16A5 | NA | GACATCCAGATGACCCAGTCTCCTTCCTCCCT GTCTGCATCTGTAGGAGACAGAGTCACCATCA CTTGCCGGGCAAGTCAGGGCATTAGAAATGAT TTAGGCTGCTATCAGCAGAAACCAGGGAAAG CCCCTAAGCGCCTGATCTATGCTGCATCCAGT TTGCAAAGTGGGGTCCCATCAAGGTTCAGCGG CAATGGATATGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTAAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAGT AATAAATACTATGTAGACTCCGTGAAGGGCCGAT TCACCATCTCCAGAGACAATTCCAAGAACACGCT GTATCTGCAAATGAGCAGCCTGAGAGCCGAGGA CACGGCTGTGTATTACTGTGCGAGAGATCTTGGC TGGACGGAAGAGTACTGGGGCCAGGGAACCCTG GTCACCGTCTCCTCA |
| | | | SEQ ID NO: 24923 | SEQ ID NO: 28929 |

FIGURE 50

| | | | | |
|---|---|---|---|---|
| | | AA | DIQMTQSPSSLSASVGDRVTITCRASQGIRNDLGCYQQKPGKAPKRLIYAASSLQSGVPSRFSGNGYGTEFTLTISSLQPEDFATYYCLQHNSYPWTFGQGTKVEIK | QVQLVESGGGVVQPGRSLRLSCAASGFTFSDYGMHWVRQAPGKGLEWVAVIWFDGSNKYYVDSVKGRFTISRDNSKNTLYLQMSSLRAEDTAVYYCARDLGWTEEYWGQGTLVTVSS |
| | | | SEQ ID NO: 24924 | SEQ ID NO: 28930 |
| iPS:393950 | 21-225_3H10 | NA | TCCTATGAGCTGAGTCAGCCACCCTCAGTGTCCGTGTCCCCAGGACAGACAGCCAGCATCACCTGCTCTGGAGATAAATTGGGGGAAAAATATGCTTGCTGGTATCAGCAGAAGCCAGGCCAGTCCCCTGTGCTGGTCATCTATCAAGATAGGAAGCGGCCCTCAGGGATCCCTGAGCGATTCTCTGGCTCCAACTCTGGGAACAAAGCCACTCTGACCATCAGCGGGACCCAGGCTATGGATGAGGCTGACTATTACTGTCAGGCGTGGGTCAACAACACTATGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGATATAGCAGTGGCTGGTATGACTACGGTTTGGACGTCTGGGGCCAAGGGACCACGGTCACCGTCTCCTCA |
| | | | SEQ ID NO: 24925 | SEQ ID NO: 28931 |
| | | AA | SYELSQPPSVSVSPGQTASITCSGDKLGEKYACWYQQKPGQSPVLVTYQDRKRPSGIPERFSGSNSGNKATLTISGTQAMDEADYYCQAWVNNTMIFGGGTKLTVL | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCARERYSSGWYDYGLDVWGQGTTVTVSS |
| | | | SEQ ID NO: 24926 | SEQ ID NO: 28932 |

FIGURE 50

| iPS:393952 | 21-225_1F1 | NA | GACATCCAGATGACCCAGTCTCCATCCTCACT GTCTGCATCTGTAGGAGACAGAGTCACCATCA CTTGTCGGGCGAGTCAGGGCATTAACAATTAT TTAGCCTGGTTTCAGCAGAAATCAGGGAAAGC CCCTAAGTCCCTGATCTCTGTTGCATCCAGTTT GCAAACTGGGGTCCCATCAAGGTTCAGCGGCA GTGGATCTGGGACAGATTTCACTCTCACCATC AGCAGCCTGCAGCCTGAAGATTTTGCAACTTA TTACTGCCAACAGTATAATAGTTACCCTCTCA CTTTCGGCGGAGGGACCAAGGTGGAGATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAACAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTTAACCTC TTTGACTACTGGGGCCAGGGAACCCTGGTCACCG TCTCCTCA |
|---|---|---|---|---|
| | | | SEQ ID NO: 24927 | SEQ ID NO: 28933 |
| | | AA | DIQMTQSPSSLSASVGDRVTITCRASQGINNYLA WFQQKSGKAPKSLISVASSLQTGVPSRFSGSGSG TDFTLTISSLQPEDFATYYCQQYNSYPLTFGGGT KVEIK | EVQLVESGGGLVKPGGSLRLSCAASGFTFSSYNMN WVRQAPGKGLEWVSSISSSSSYIYYADSVKGRFTIS RDNAKNSLYLQMNSLRAEDTAVYYCARVNLFDY WGQGTLVTVSS |
| | | | SEQ ID NO: 24928 | SEQ ID NO: 28934 |
| iPS:393954 | 21-225_4H6 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAATTT ATTATTGTCAACAGGCTAACAGTTTCCCATTC ACTTTCGGCCCTGGGACCAAAGTGGATATCAA A | CAGGTGCAGCTGGTACAGTCTGGGGCTGAGGTG AAGAAGCCTGGGGCCTCAGTGAAGGTCTCCTGC AAGGCTTCTGGATACACCTTCACCGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGGGCTGAGCAGTCTGAGATCTGACG ACACGGCCGTGTATTACTGTGCGAGAGATGGTAC CAGCTCGTTTGACTACTGGGGCCAGGGAACCCTG GTCACCGTCTCCTCA |
| | | | SEQ ID NO: 24929 | SEQ ID NO: 28935 |

FIGURE 50

| | | | | |
|---|---|---|---|---|
| | | AA | DIQMTQSPSSVSASVGDRVTITCRASQGISRWLA WYQQKPGKAPKLLIYGASSLQSGVPSRFSGSGS GTDFTLTISSLQPEDFAIYYCQQANSFPFTFGPGT KVDIK | QVQLVQSGAEVKKPGASVKVSCKASGYTFTDYYL HWVRQAPGQGLEWMGWIHPNSGGTNYAQKFQGR VTMTRDTSISTAYMGLSSLRSDDTAVYYCARDGTS SFDYWGQGTLVTVSS |
| | | | SEQ ID NO: 24930 | SEQ ID NO: 28936 |
| iPS:393956 | 21-225_4D7 | NA | GACATCCAGATGACCCAGTCTCCATCCTCCCT GTCTGCATCTGTAGGAGACAGAGTCACCATCA CTTGCCGGGCAAGTCAGAGCATTAGCGACTAT TTAAAATTGGTATCAGCAGAAACCAGGGAAAG CCCCTAAACTCCTGATCTTTGATACAACCAGTT TGCAAAGTGGGGTCCCATCAAGGTTCAGTGGC AGTGGATCTGGGACAGATTTCACTCTCACCAT CAACAGTCTGCAACCTGAAGATTTTGCAACTT ACTACTGTCAACAGACTTACAATACCCCTCCG GAGCGCAGTTTTGGCCAGGGGACCAAGCTGG AGATCAAA | GAGGTGAAGCTGTTGGAGTCTGGGGGAGGCTTG GTACAGCCGGGGGGGTCCCTGAGACTCTCCTGTG CAGCCTCTGGATTCACCTTCAGCAGCTATGCCAT GAGCTGGGTCCGCCAGGCTCCAGGGAAGGGGCT GGAGTGGGTCTCAGTTCTTAGTGGTAGTGGTGGT AGCACATTCTACGCAGACTCCGTGAAGGGCCGGT TCACCATCTCCAGAGACAATTCCAAGAACACGCT GTATCTGCAAATGAGCAGCCTGAGAGCCGGGGA CACGGCCGTATATTTCTGTGCCCGATATTGTAGT AGTGCCAGGTGCCCTTATGATGCCTTTGATATCT GGGGCCAAGGGACAATGGTCACCGTCTCCTCA |
| | | | SEQ ID NO: 24931 | SEQ ID NO: 28937 |
| | | AA | DIQMTQSPSSLSASVGDRVTITCRASQSISDYLN WYQQKPGKAPKLLIFDTTSLQSGVPSRFSGSGSG TDFTLTINSLQPEDFATYYCQQTYNTPPERSFGQ GTKLEIK | EVKLLESGGGLVQPGGSLRLSCAASGFTFSSYAMS WVRQAPGKGLEWVSVLSGSGGSTFYADSVKGRFTI SRDNSKNTLYLQMSSLRAGDTAVYFCARYCSSARC PYDAFDIWGQGTMVTVSS |
| | | | SEQ ID NO: 24932 | SEQ ID NO: 28938 |

FIGURE 50

| iPS:393958 | 21-225_5H2 | NA | GACATCCAGATGACCCAGTCTCCATCCTCCCT GTCTGCATCTGTAACAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCAGTCTCACAA TCAGCAGCCTGCAGCCTGAAGATTTTGCAACT TATTACTGTCTACAGCATAATAGTTACCCTCTC ACTTTCGGCGGAGGGACCAAGGTGGAGATCA AA | GAGGTGCAGCTGGTGGAGTCTGGGGGAGGCCTG GTCAAGCCTGGGGGGTCCTTGAGACTCTCCTGTG CAGCCTCTGGATTCACATTCAGTAGCTATACCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGCCAACGCCAAGAACTCATT GTATCTGCAAATGAACAGCCTGAGAGCCGAGGA CACGGCTGTGTATTACTGTGCGAGAGGGAGCAG CTCGTCCGGCTTTGACTACTGGGGCCAGGGAACC CTGGTCACCGTCTCCTCA |
|  |  |  | SEQ ID NO: 24933 | SEQ ID NO: 28939 |
|  |  | AA | DIQMTQSPSSLSASVTDRVTITCRASQGIRNDLG WYQQKPGKAPKRLIYAASSLQSGVPSRFSGSGS GTEFSLTISSLQPEDFATYYCLQHNSYPLTFGGGT KVEIK | EVQLVESGGGLVKPGGSLRLSCAASGFTFSSYTMN WVRQAPGKGLEWVSSISGSSSYIYYADSVKGRFTIS RANAKNSLYLQMNSLRAEDTAVYYCARGSSSSGF DYWGQGTLVTVSS |
|  |  |  | SEQ ID NO: 24934 | SEQ ID NO: 28940 |
| iPS:393960 | 21-225_7G2 | NA | GACATCCAGATGACCCAGTCTCCATCCTCCCT GTCTGCGTCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAGTTACCCGTG GACGTTCGGCCTAGGGACCAAGGTGGTCATCA AA | CAGGTGCAACTGGTGGAGTCTGGGGGAGGCGTG GTCCAGCCTGGGAGGTCCCTGAGACTCTCCTGTG CAGCGTCAGGATTCACCTTCAGTGACTATGGCAT GCACTGGGTCCGCCAGGCTCCAGGCAAGGGGCT GGAGTGGATGGCAGTTATATGGTATGATGTAACT AATAAATACTATGCAGACTCCGTGAAGGGCCGA TTCACCATCTCCAGAGACAATTCCAAGAACACGC TGTATCTGCAACTGAACAGCCTGAGAGCCGAGG ACACGGCTGTTTATTACTGTGCGAGAGAACTTGG CTGGTACGAGGACTACTGGGGCCAGGGAACCCT GGTCACCGTCTCCTCA |
|  |  |  | SEQ ID NO: 24935 | SEQ ID NO: 28941 |

FIGURE 50

| | | | | |
|---|---|---|---|---|
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHNSYPWTFGLGT KVVIK | QVQLVESGGGVVQPGRSLRLSCAASGFTFSDYGM HWVRQAPGKGLEWMAVIWYDVTNKYYADSVKG RFTISRDNSKNTLYLQLNSLRAEDTAVYYCARELG WYEDYWGQGTLVTVSS |
| | | | SEQ ID NO: 24936 | SEQ ID NO: 28942 |
| iPS:393962 | 21-225_7H7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCACATCAAGGTTCAGCGG CAGTGGATCTGGGACAGAATTCACTCTCACAA TCAGCAGCCTGCAGCCTGAAGATTTTGCAACT TATTACTGTCTACAGCATAGTAGTTACCCGTTC ACTTTCGTCGGAGGGACCAAAGTGGAGATCAA A | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAATATCTATTATAGT GGGAGCACCTACTACATCCCGTCCCTCAAGAGTC GAGTCACCATATCCGTTGACACGTCCAAGAACCA GTTCTCCCTGAAACTGAGCTCTGTGACCGCCGCA GACACGGCTGTGTATTACTGTGCGAGACACAGTA CCAGCTGGTCTCTTGACCACTGGGGGCCAGGGAAC CCTGGTCACCGTCTCCTCA |
| | | | SEQ ID NO: 24937 | SEQ ID NO: 28943 |
| | | AA | DIQMTQSPSSLSASVGDRVTITCRASQGIRNDLG WYQQKPGKAPKRLIYAASSLQSGVTSRFSGSGS GTEFTLTISSLQPEDFATYYCLQHSSYPFTFVGGT KVEIK | QLQLQESGPGLVKPSETLSLTCTVSGGSISRSSYYW GWIRQPPGKGLEWIGNIYYSGSTYYIPSLKSRVTISV DTSKNQFSLKLSSVTAADTAVYYCARHSTSWSLDH WGQGTLVTVSS |
| | | | SEQ ID NO: 24938 | SEQ ID NO: 28944 |

FIGURE 50

| | | | | |
|---|---|---|---|---|
| iPS:393964 | 21-225_6G1 | NA | GACATCCAGATGACCCAGTCTCCATCCTCCCT GTCTGCATCTGTAGGAGACAGAGTCACCATCA CTTGCCGGACAAGTCAGAACATTATCAGCTAT TTAAATTGGTATCAGCAGAAACCAGGGAAAG CCCCTAAGGTCCTGATCTATACTGCATCCAATT TGCAAACTGGGGTCCCATCAGGGTTCAGTGGC AGTGGATCTGGGACAGATTTCACTCTCACCAT CAGCAGTCTGCAACCTGAAGATTTTGCAACTT ACTACTGTCAACAGCCTCACAGTCCCCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCATCTTCAGTAGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTATGTATTACTGTGCGAGAGATCTGAG TATGGGCGGTATGGACGTCTGGGGGCCAAGGGAC CACGGTCACCGTCTCCTCA |
| | | | SEQ ID NO: 24939 | SEQ ID NO: 28945 |
| | | AA | DIQMTQSPSSLSASVGDRVTITCRTSQNIISYLNW YQQKPGKAPKVLIYTASNLQTGVPSGFSGSGSGT DFTLTISSLQPEDFATYYCQQPHSPPLTFGGGTK VEIK | QVQLVESGGGVVQPGRSLRLSCAASGFIFSSYGMH WVRQAPGKGLEWVAIIWYDGSNKYYADSVKGRFT ISRDNSKNTLYLQMNSLRAEDTAMYYCARDLSMG GMDVWGQGTTVTVSS |
| | | | SEQ ID NO: 24940 | SEQ ID NO: 28946 |
| iPS:393966 | 21-225_7F8 | NA | GACATCCAGATGACCCAGTCTCCATCCTCCCT GTCTGCATCTGTGGGAGACAGAGTCACCATCA CTTGCCGGGCAAGTCAGGGCATTGGAAATGAT TTAGGCTGGTATCAGCAGAAACCAGGGAAAG CCCCTAAGCGCCTGATCTATGCTGCATCCAGT TTGCAAAGTGGGGTCCCATCACGGTTCAGCGG CAGTGTATCTGGGACAGAATTCACTCTCCCAA TCAGCAGCCTGCAGCCTGAAGATTTTGCAACT TATTACTGTCTACAACATTATACTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTGTGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAATAACACGC TGTATCTGCAAATGAACAGCCTGAGAGCCGAGG ACACGGCTGTGTATTACTGTGCGAGAGAAAGGTA TACCAGTGGCTGGCACGACTACGGTATGGACGTC TGGGGCCAAGGGACCACGGTCACCGTCTCCTCG |
| | | | SEQ ID NO: 24941 | SEQ ID NO: 28947 |

FIGURE 50

| | | | | |
|---|---|---|---|---|
| | | AA | DIQMTQSPSSLSASVGDRVTITCRASQGIGNDLG WYQQKPGKAPKRLIYAASSLQSGVPSRFSGSVS GTEFTLPISSLQPEDFATYYCLQHYTYPRTFGQG TKVEIK | QVQLVESGGGVVQPGRSLRLSCAASGFTFSSCVMH WVRQAPGKGLEWVAVIWYDGSNKYYADSVKGRF TISRDNSNNTLYLQMNSLRAEDTAVYYCARERYTS GWHDYGMDVWGQGTTVTVSS |
| | | | SEQ ID NO: 24942 | SEQ ID NO: 28948 |
| IPS:393968 | 21-225_5A5 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTCTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA | GAGGTGCAGCTGTGGGAGTCTGGGGGAGGCTTG GTACAGCCTGGGGGGTCCCTGAGACTCTCCTGTG CAGCCTCCGGATTCACCTTTAGCAGCTATGCCAT GAACTGGGTCCGCCAGGCTCCAGGGAAGGGGCT GGAGTGGGTCTCAGCTATTAGTGGTAGTGGTGGT TACACATACTACGCAGACTCCGTGAAGGGCCGGT TCACCATCTCCCGAGACAATTCCAAGAACACGCT GTATCTGCAAATGAGCAGCCTGAGAGCCGAGGA CACGGCCGTTTATTACTGTGCGAAAGGGGGGTCC CTCTTCTACTGGGGCCAGGGAACCTTGGTCACCG TCTCTTCA |
| | | | SEQ ID NO: 24943 | SEQ ID NO: 28949 |
| | | AA | DIQMTQSPSSLSASVGDRVTITCRASQGISNYLA WFQQKPGKAPKSLISAASSLQSGVPSKFSGSGSG TDFTLTISSLQPEDFATYYCQQYNSYPFTFGPGT KVDIK | EVQLWESGGGLVQPGGSLRLSCAASGFTFSSYAMN WVRQAPGKGLEWVSAISGSGGYTYYADSVKGRFT ISRDNSKNTLYLQMSSLRAEDTAVYYCAKGGSLFY WGQGTLVTVSS |
| | | | SEQ ID NO: 24944 | SEQ ID NO: 28950 |

FIGURE 50

| iPS:393972 | 21-225_7C9 | NA | GACATCCAGATGACCCAGTCTCCATCCTCCCT GTCTGCATCTGGAGGAGACAGAGTCACCATCA CTTGCCGGGCAAGTCAGGGCATTAGAAACGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCTTTATAGTTACCCTCGG ACGTTCGGCCAGGGGACCAAGGTGGATATCA AA | CAGGTGCAGCTGGTGGAGTCTGGGGGAGGCGTG GTCCAGCCTGGGAGGTCCCTGAGACTCTCCTGTG CAGCGTCTGGACTCACCTTCAGTAACTATGTCAT GCACTGGGTCCGCCAGGCTCCAGGCAAGGGGCT GGAGTGGGTGGCTGTTATATGGTATGATGGAAGT AATAAATACTATGCAGACTCCGTGAAGGGCCGA TTCACCATCTCCAGAGACAATTCCAAGAACACGT TGTATCTGCAAATGAACAGCCTGAGAGCCGAGG ACACGGCTGTGTATTACTGTGCGAGAGAGAAGTA TAGCAGCAGCTGGTACGACTACGGTATGGACGTC TGGGGCCAAGGGACCACGGTCACCGTCTCCTCA |
|---|---|---|---|---|
| | | | SEQ ID NO: 24945 | SEQ ID NO: 28951 |
| | | AA | DIQMTQSPSSLSASGGDRVTITCRASQGIRNDLG WYQQKPGKAPKRLIYAASSLQSGVPSRFSGSGS GTEFTLTISSLQPEDFATYYCLQLYSYPRTFGQGT KVDIK | QVQLVESGGGVVQPGRSLRLSCAASGLTFSNYVM HWVRQAPGKGLEWVAVIWYDGSNKYYADSVKGR FTISRDNSKNTLYLQMNSLRAEDTAVYYCAREKYS SSWYDYGMDVWGQGTTVTVSS |
| | | | SEQ ID NO: 24946 | SEQ ID NO: 28952 |
| iPS:393974 | 21-225_7C4 | NA | GACATCCAGATGACCCAGTCTCCATCCTCCCT GTTTGCATCTGTAGGAGACAGAGTCACCATCA CTTGCCGGGCAAGTCAGGGCATTAGAAATGAT TTAGGCTGGTATCAGCAGAAACCAGGGAAAG CCCATAAGCGCCTTATATATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAAGTCCCTGAGACTCTCCTGTG CAGCGTCTGGATTCACCTTCAGTAACTATGGCAT GCACTGGGTCCGCCAGGCTCCAGGCAAGGGGCT GGAATGGGTGGCAGTTATTTGGTATGAAGAAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCGGTCTGACTACTGGGGCCAGGGAACCCTGG TCACCGTCTCCTCA |
| | | | SEQ ID NO: 24947 | SEQ ID NO: 28953 |

1676

FIGURE 50

| | | | | |
|---|---|---|---|---|
| | | AA | DIQMTQSPSSLFASVGDRVTITCRASQGIRNDLG WYQQKPGKAHKRLIYAASSLQSGVPSRFSGSGS GTEFTLTISSLQPEDFATYYCLQHNSYPLTFGGG TKVEIK | QVQLVESGGGVVQPGKSLRLSCAASGFTFSNYGM HWVRQAPGKGLEWVAVIWYEENNQYYADSVKGR FTISRDNSKNTLYLQMNSLRAEDTAVYYCARELGF RSDYWGQGTLVTVSS |
| | | | SEQ ID NO: 24948 | SEQ ID NO: 28954 |
| IPS:393976 | 21-225_7E9 | NA | GACATCCAGATGACCCAGTCTCCATCCTCCCT GTTTGCATCTGTAGGAGACAGAGTCACCATCA CTTGCCGGGCAAGTCAGGGCATTAGAAATGAT TTAGGCTGGTATGAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAAAT AATAAATACTATGTAGACTCCGTGAAGGGCCGAT TCACCATCTCCAGAGACAACTCCAAGAACACGCT GTATCTGCAAATGAACAGCCTGAGAGCCGAGGA CACGGCTGTATATTATTGTGCGAGAGAATTGGGG TTCCGGTCTGACTACTGGGGCCAGGGAACCCTGG TCACCGTCTCCTCA |
| | | | SEQ ID NO: 24949 | SEQ ID NO: 28955 |
| | | AA | DIQMTQSPSSLFASVGDRVTITCRASQGIRNDLG WYEQKPGKAPKRLIYTASSLQSGVPSRFSGSGSG TEFTLTISSLQPEDFATYYCLQHNSYPLTFGGGT KVEIK | QVQLVESGGGVVQPGRSLRLSCAASGFTFSDYGM HWVRQAPGKGLEWVAVIWYDENNKYYVDSVKGR FTISRDNSKNTLYLQMNSLRAEDTAVYYCARELGF RSDYWGQGTLVTVSS |
| | | | SEQ ID NO: 24950 | SEQ ID NO: 28956 |

FIGURE 50

| iPS:393978 | 21-225_4C12 | NA | GACATCCAGATGACCCAGTCTCCATCCTCCCT GTCTGCTTCTGTAGGCGACAGAGTCACCATCA CTTGCCGGGCAAGTCAGGGCATTAGAAATGAT TTAGGCTGGTATCAGCAGAAACCAGGGAAAG CCCCTACGCGCCTGATCTATGCTGCATCCAGTT TGCACAGTGGGGTCCCATCACGGTTCAGCGGC AGTGGATCTGGGACAGAATTCACTCTCACAAT CAGCAGCCTGCAGCCTGAAGATCTTGCAACTT ATTACTGTCTACAACATTATAGTTTCCCTCGGA CGTTCGGCCAAGGGACCAAGGTGGAAATCAA A | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAAGTA TAGCAGCAGCTGGTACGACTACGGTATGGACGTC TGGGGCCACGGGACCACGGTCACCGTCTCCTCA |
|---|---|---|---|---|
| | | | SEQ ID NO: 24951 | SEQ ID NO: 28957 |
| | | AA | DIQMTQSPSSLSASVGDRVTITCRASQGIRNDLG WYQQKPGKAPTRLIYAASSLHSGVPSRFSGSGSG TEFTLTISSLQPEDLATYYCLQHYSFPRTFGQGTK VEIK | QVQLVESGGGVVQPGRSLRLSCAASGFTFSSYVMH WVRQAPGKGLEWVAVIWYDGSNKYYADSVKGRF TISRDNSKNTLYLQMNSLRAEDTAVYYCAREKYSS SWYDYGMDVWGHGTTVTVSS |
| | | | SEQ ID NO: 24952 | SEQ ID NO: 28958 |
| iPS:393980 | 21-225_6D3 | NA | GACATCCAGATGACCCAGTCTCCATCCTCCCT GTCTGCATCTGTAGGAGACAGAGTCACCATCA CTTGCCGGACAAGTCAGAGCATTAGTACTTAT TTAAATTGGTATCAGCAGAAACCAGGGAAAG CCCCTAAGCTCCTGATCTTTGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTTCTGTCAACAGAGTTACAGAACCCCCTTT TTCACTTTCGGCCCTGGGACCAAAGTGGATAT CAAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAACTGGGTCCGCCAGGCTCCAGGGAAGGGGCT GGAGTGGGTCTCAGTTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTTCTGTGCGTCCCGTTTGGCA GTGGCTGGCTCGGAGGCTTTTGATATCTGGGGCC AAGGGACAATGGTCACCGTCTCTTCA |
| | | | SEQ ID NO: 24953 | SEQ ID NO: 28959 |

FIGURE 50

| | | | | |
|---|---|---|---|---|
| | | AA | DIQMTQSPSSLSASVGDRVTITCRTSQSISTYLNW YQQKPGKAPKLLIFAASSLQSGVPSRFSGSGSGT DFTLTISSLQPEDFATYFCQQSYRTPFFTFGPGTK VDIK | EVQLLESGGGLVQPGGSLRLSCAASEFTFSSYAMN WVRQAPGKGLEWVSVISGRGGNTFYADSVKGRFTI SRDNSKNTLYLQMNSLRAEDTAVYFCASRLAVAG SEAFDIWGQGTMVTVSS |
| | | | SEQ ID NO: 24954 | SEQ ID NO: 28960 |
| IPS:393982 | 21-225_6C12 | NA | GACATCCAGATGACCCAGTCTCCATCCTCCCT GTCTGCATCTGTAGGAGACAGAGGCACCATCA CTTGCCGGGCAAGTCAGGGCATTAGAAGTAAT TTAGGCTGGTATCAGCAGAAACCAGGGAAAG CCCCTAAGCGCCTGATCTATGCTGCATCCAGT TTGGAAAGTGGGGTCCCATCAAGGTTCAGCGG CAGTGGATCTGGGACAGAATTCACTCTCACAA TCAGCAGCCTGCAGCCTGAAGATTTTGCAACT TATTTCTGTCTACAGGATAATAGTTATCCGTTC ACTTTCGGCGGAGGGACCAAGGTGGAGATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATCGTGGG AGCCTCTGGGGCCAGGGAACCCTGGTCACCGTCT CCTCA |
| | | | SEQ ID NO: 24955 | SEQ ID NO: 28961 |
| | | AA | DIQMTQSPSSLSASVGDRGTITCRASQGIRSNLG WYQQKPGKAPKRLIYAASSLESGVPSRFSGSGSG TEFTLTISSLQPEDFATYFCLQDNSYPFTFGGGTK VEIK | EVQLVESGGGLVKPGGSLRLSCAASGFTFSSYSMN WVRQAPGKGLEWVSSISSSSSYIYYADSVKGRFTIS RDNAKNSLYLQMNSLRAEDTAVYYCARDRGSLW GQGTLVTVSS |
| | | | SEQ ID NO: 24956 | SEQ ID NO: 28962 |

FIGURE 50

| iPS:393984 | 21-225_4F12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CGGTGGATCTGGGACAATATTCACTCTCACAA TCAGCAGCCTGCAGCCTGAAGATTTTGCAACT TATTACTGTCTACAGCATAATAGTTACGCGCT CACTTTCGGCGGAGGGACCAAGGTGGAGATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGTTACT AATAAAAAGTATGCAGACTCCGTGAAGGGCCGA TTCACCATCTCCAGAGACAATTCCAAGAACACGC TGTATCTGCAAATGAACAGCCTGACACCAGAGA ACACGGGTGGATATGAGAACCAGAGAGAAAAGG GGGGTCTATTTGACTACTGGGGCCAGGGAACCCT GGTCACCGTCTCCTCA |
|---|---|---|---|---|
| | | | SEQ ID NO: 24957 | SEQ ID NO: 28963 |
| | | AA | DIQMTQSPSSLSASVGDRVTITCRASQGIRNDLG WYQQKPGKAPKRLIYAASSLQSGVPSRFSGGGS GTIFTLTISSLQPEDFATYYCLQHNSYALTFGGGT KVEIK | QVQLVESGGGVVQPGRSLRLSCAASGFTFSSYGMH WVRQAPGKGLEWVAVIWYDVTNKKYADSVKGRF TISRDNSKNTLYLQMNSLTPENTGGYENQREKGGL FDYWGQGTLVTVSS |
| | | | SEQ ID NO: 24958 | SEQ ID NO: 28964 |
| iPS:393986 | 21-225_7G4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAGACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTCTCAACT TATTACTGTCTACATCAATATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAATAACTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGACCAGCCTGAGAGCCGAGG ACACGGCTGTGTATTACTGTGCGAGAGAGAAGTA TAGCAGCAACTGGTACGACTACGGTATGGACGTC TGGGGCCAAGGGACCACGGTCACCGTCTCCTCA |
| | | | SEQ ID NO: 24959 | SEQ ID NO: 28965 |

FIGURE 50

| | | | | |
|---|---|---|---|---|
| | | AA | DIQMTQSPSSLSASVGDRVTITCRASQGIRNDLG WYQQRPGKAPKRLIYAASSLQSGVPSRFSGSGS GTEFTLTISSLQPEDSATYYCLHQYSYPRTFGQG TKVEIK | QVQLVESGGGVVQPGRSLRLSCAASGFTFNNYVM HWVRQAPGKGLEWVAVIWYDGSNKYYADSVKGR FTISRDNSKNTLYLQMTSLRAEDTAVYYCAREKYS SNWYDYGMDVWGQGTTVTVSS |
| | | | SEQ ID NO: 24960 | SEQ ID NO: 28966 |
| IPS:393988 | 21-225_7F10 | NA | GACATCCAGATGACCCAGTCGCCATCCGCACT GTCTGCATCTGTAGGAGACAGAGTCACCATCA CTTGTCGGGCGAGTCAGGACATTAGGAATTAT TTAGCCTGGTTTCAGCAGAAACCAGGGAAAGC CCCTAAGTCCCTGATCTCTGTTGCATCCAGTTT GCAAAGTGGGGTCCCATCAAGGTTCAGCGGG AGTGGATCTGGGACAGATTTCACTCTCACCAT CAGCAGCCTGCAGCCTGAAGATTTTGCAACTT ATTATTGCCAACAGTATAATAGTTACCCTCTC ACTTTCGGCGGAGGGACCAAGGTGGAGATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAACCGAGGA CACGGCTGTGTATTACTGTGCGAGAGCTAACCTC TTTGACTACTGGGGCCAGGGAACCCTGGTCACCG TCTCCTCA |
| | | | SEQ ID NO: 24961 | SEQ ID NO: 28967 |
| | | AA | DIQMTQSPSALSASVGDRVTITCRASQDIRNYLA WFQQKPGKAPKSLISVASSLQSGVPSRFSGSGSG TDFTLTISSLQPEDFATYYCQQYNSYPLTFGGGT KVEIK | EVQLVESGGGLVKPGGSLRLSCAASGFTFSSYSMN WVRQAPGKGLEWVSSISSSSSYIYYADSVKGRFTIS RDNAKNSLYLQMNSLRTEDTAVYYCARANLFDY WGQGTLVTVSS |
| | | | SEQ ID NO: 24962 | SEQ ID NO: 28968 |

FIGURE 50

| iPS:393990 | 21-225_11G7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAGTAATTACCCTCTC ACTTTCGGCGGAGGGACCATGGTGGAGATCAG A | CAGCTGCAGCTGCAGGAGTCGGGCCCAGGACTG GTGAAGCCTTCGGAGACCCTGTCCCTCACCTGCA CTGTCTCTGGTGGCTTCATCAGCAGGAGTACTTA CTACTGGGGCTGGATCCGCCAGCCCCCAGGGAA GGGGCTGGAGTGGATTGGGAGTATCTATTATAGT GGGAGCACCTCCTACAGCCCGTCCCTCAAGAGTC GAGTCACCATATCCGTAGACACGTCCAAAAACC AGTTCTCCCTGAAGTTGAGCTCTGTGACCGCCGC AGACACGGCTGTGTATTACTGTGCGAGACTGAAC AGCAGCTGGTCTTTTGACTACTGGGGCCAGGGAA CCCTGGTCACCGTCTCCTCA |
| | | | SEQ ID NO: 24963 | SEQ ID NO: 28969 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHSNYPLTFGGG TMVEIR | QLQLQESGPGLVKPSETLSLTCTVSGGFISRSTYYW GWIRQPPGKGLEWIGSIYYSGSTSYSPSLKSRVTISV DTSKNQFSLKLSSVTAADTAVYYCARLNSSWSFDY WGQGTLVTVSS |
| | | | SEQ ID NO: 24964 | SEQ ID NO: 28970 |
| iPS:393992 | 21-225_14H8 | NA | GACATCCAGATGACCCAGTCTCCATCCTCACT GTCTGCATCTGTAGGAGACAGAGTCACCATCA CTTGTCGGGCGAGTCAGGGCATTAGCTATTAT TTAGCCTGGTTTCAGCAGAAACCAGGGAAAGC CCCTAAGTCCCTGATCTATGTTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAT | GAGGTGCAGCTGGTGGAGTCTGGGGGAGGCTTG GTACAGCCTGGGGGGTCCCTGAGACTCTCCTGTG CAGCCTCTGGATTCACCTTCAGTAGCAATAGCAT GAACTGGGTCCGCCAGGCTCCAGGGAAGGGGCT GGAGTGGGTTTCATACATTAGTAGTAGTAGTAGT ACCATATACTACGCAGACTCTGTGAAGGGCCGAT TCACCATCGCCAGAGACAATGCCAAGAACTCACT GTATCTGCAAATGAACAGCCTGAGAGACGAGGA CACGGCTGTGTATTACTGTGCGAGAGGAGGTGGG AGCCCTTTTGACTACTGGGGCCAGGGAACCCTGG TCACCGTCTCCTCA |
| | | | SEQ ID NO: 24965 | SEQ ID NO: 28971 |

EP 4 435 105 A2

FIGURE 50

| | | | | |
|---|---|---|---|---|
| | | AA | DIQMTQSPSSLSASVGDRVTITCRASQGISYYLA WFQQKPGKAPKSLIYVASSLQSGVPSKFSGSGSG TDFTLTISSLQPEDFATYYCQQYNSYPFTFGPGT KVDIN | EVQLVESGGGLVQPGGSLRLSCAASGFTFSSNSMN WVRQAPGKGLEWVSYISSSSSTIYYADSVKGRFTIA RDNAKNSLYLQMNSLRDEDTAVYYCARGGGSPFD YWGQGTLVTVSS |
| | | | SEQ ID NO: 24966 | SEQ ID NO: 28972 |
| IPS:393994 | 21-225_8C9 | NA | GACATCCAGATGACCCAGTCTCCATCCTCCCT GTCTGCATCTATAGGAGACAGAGTCACCATCA CTTGCCGGGCAAGTCAGGCCATTAGAAATGAT TTAGACTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCACTCTCACAAT CAGCAGCCTGCAGACTGAAGATTTTGCAACTT ATTACTGTCTACAGCATAATAGTTATCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAAT AATAAATACTATGTAGACTCCGTGAAGGGCCGAT TCACCATCTCCAGAGACAACTCCAAGAACACGCT GTATCTGCAAATGAACAGCCTGAGAGCCGAGGA CACGGCTGTGTATTACTGTGCGAGAGAATTGGGG TTCCGGTCTGACTACTGGGGCCAGGGAACCCTGG TCACCGTCTCCTCA |
| | | | SEQ ID NO: 24967 | SEQ ID NO: 28973 |
| | | AA | DIQMTQSPSSLSASIGDRVTITCRASQAIRNDLD WYQQKPGKAPKRLIYAASSLQSGVPSRFSGSGS GTEFTLTISSLQTEDFATYYCLQHNSYPLTFGGG TKVEIK | QVQLVESGGGVVQPGRSLRLSCAASGFTFSSYGMH WVRQAPGKGLEWVAVIWYDENNKYYVDSVKGRF TISRDNSKNTLYLQMNSLRAEDTAVYYCARELGFR SDYWGQGTLVTVSS |
| | | | SEQ ID NO: 24968 | SEQ ID NO: 28974 |

1683

FIGURE 50

| iPS:393996 | 21-225_15C11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAGCCAGGGAAAG TCCCTAAGCGCCTGATCTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACTGCATTATAGTTACCCTCGGA CGTTCGGCCGAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAAG ACACGGCTGTGTATTACTGTGCGAGAGAGAAGTA TAGCAGCAGCTGGTACGACTACGGTTTGGACGTC TGGGGCCAAGGGACCACGGTCACCGTCTCCTCA |
| | | | SEQ ID NO: 24969 | SEQ ID NO: 28975 |
| | | AA | DIQMTQSPSSLSASVGDRVTITCRASQGIRNDLG WYQQKPGKVPKRLIYAASSLQSGVPSRFSGSGS GTEFTLTISSLQPEDFATYYCLLHYSYPRTFGRGT KVEIK | QVQLVESGGGVVQPGRSLRLSCAASGFTFSDYVM HWVRQAPGKGLEWVAVIWYDGSNKYYADSVKGR FTISRDNSKNTLYLQMNSLRAEDTAVYYCAREKYS SSWYDYGLDVWGQGTTVTVSS |
| | | | SEQ ID NO: 24970 | SEQ ID NO: 28976 |
| iPS:393998 | 21-225_12B12 | NA | GACATCCAGATGACCCAGTCTCCATCCTCCCT GTCTGCGTCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAGTTACCCGTG GACGTTCGGCCTAGGGACCAAGGTGGTCATCA AA | CAGGTGCAACTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGATGGCAGTTATATGGTATGATGTAACT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTTTATTACTGTGCGAGAGAGCTTGG CTGGTACGAGGACTACTGGGGCCAGGGAACCCT GGTCACCGTCTCCTCA |
| | | | SEQ ID NO: 24971 | SEQ ID NO: 28977 |

FIGURE 50

| | | | | |
|---|---|---|---|---|
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHNSYPWTFGLGT KVVIK | QVQLVESGGGVVQPGRSLRLSCAASGFTFSDYGM HWVRQAPGKGLEWMAVIWYDVTNKYYADSVKG RFTISRDNSKNTLYLQMNSLRAEDTAVYYCARELG WYEDYWGQGTLVTVSS |
| | | | SEQ ID NO: 24972 | SEQ ID NO: 28978 |
| iPS:394000 | 21-225_11A2 | NA | GACATCCAGATGACCCAGTCTCCATCCTCCCT GTCTGCATCTGTAGGAGACAGAGTCACCATCA CTTGCCAGGCGAGTCAGGACATTAGCAACTAT TTAAAATTGGTATCAGCAGAAACCAGGGAAAG CCCCTAAGCTCCTGATCTACGATGCATCCAAT TTGGAAACAGGGGTCCCCTCAAGATTCAGTGG AAGTGGATCTGGGACAGATTTTTACTTTCACCA TCAGCAGCCTGCAACCTGAGGATGTTGCAACA TATTACTGTCAACAGTATGATAATCTCCCGAT CACCTTCGGCCAAGGGACACGACTGGACATTA AA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGGTGGAAGT AATAAAGACTCTGCAGACTCCGTGAAGGGCCGA TTCATCATCTCCAGAGACAATTCCAAGAACACGC TGTATCTGCAAATGAACAGCCTGAGAGCTGAGG ACACGGCTGTGTATTACTGTGTGAGACGGGGATA CAGCTATGGCGGGTACGGTATGGACGTCTGGGG CCAAGGGACCACGGTCACCGTCTCCTCA |
| | | | SEQ ID NO: 24973 | SEQ ID NO: 28979 |
| | | AA | DIQMTQSPSSLSASVGDRVTITCQASQDISNYLN WYQQKPGKAPKLLIYDASNLETGVPSRFSGSGS GTDFTFTISSLQPEDVATYYCQQYDNLPITFGQG TRLDIK | QVQLVESGGGVVQPGRSLRLSCAASGFTFSSYGMH WVRQAPGKGLEWVAVISYGGSNKDSADSVKGRFII SRDNSKNTLYLQMNSLRAEDTAVYYCVRRGYSYG GYGMDVWGQGTTVTVSS |
| | | | SEQ ID NO: 24974 | SEQ ID NO: 28980 |

1685

FIGURE 50

| iPS:394002 | 21-225_15G7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGATCCCATCAAGGTTCAGCGG CAGTGGATTTGGGACAGAATTCACTCTCACAA TCAGCAGCCTGCAGCCTGAAGATTTTGCAACT TATTACTGTCTACAGCATAGTAATTACCCTCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGGAGTAGTTC CTACTGGGGCTGGATCCGCCAGCCCCCAGGGAA GGGACTGGAGTGGATTGGGAGTATCTATTATAGT GGGTACACCTATTACACCCCGTCCCTCAAGAGTC GAGTCACCATATCCGTGGACACGTCCAAGAACC AGTTCTCCCTGAGGCTGAGCTCTGTGACCGCCGC AGACACGGCTTCGTATTACTGTGCGAGACTGAGC AGCAGTTGGTCTTTTGACTTCTGGGGCCAGGGAA CCCTGGTCACCGTCTCCTCA |
| | | | SEQ ID NO: 24975 | SEQ ID NO: 28981 |
| | | AA | DIQMTQSPSSLSASVGDRVTITCRASQGIRNDLG WYQQKPGKAPKRLIYAASSLQSGIPSRFSGSGFG TEFTLTISSLQPEDFATYYCLQHSNYPLTFGGGT KVEIK | QLQLQESGPGLVKPSETLSLTCTVSGGSISRSSSYW GWIRQPPGKGLEWIGSIYYSGYTYYTPSLKSRVTIS VDTSKNQFSLRLSSVTAADTASYYCARLSSSWSFD FWGQGTLVTVSS |
| | | | SEQ ID NO: 24976 | SEQ ID NO: 28982 |
| iPS:394004 | 21-225_13A1 | NA | GACATCCAGATGACCCAGTCTCCATCCTCCCT GTCTGCATCTGTAGGAGACAGAGTCACCATCA CTTGCCAGGCGAGTCAGGACATTAACAACTAT TTAAATTGGTATCAGCAGAAACTAGGGACAGC CCCTAAGCTCCTGATCTACGATGGATCCAATT TGGAAACAGGGGTCCCATCAAGGTTCAGTGGA AGTGGATCTGGGACAGATTTTACTTTCACCAT CAGCAGCCTGCAGCCTGAAGATATTGCAACAT ATTACTGTCAACAGTATGAAAATCTCCCGATC ACTTTCGGCCAAGGGACACGACTGGAGATTAA A | CAGGTGCAGTTGGTGGAGTCTGGGGGAGGCGTG GTCCAGCCTGGGAGGTCCCTGAGACTCTCCTGTG CAGCCTCTGGATTCACCTTCAGCAGCTATGGCAT GCACTGGGTCCGCCAGGCTCCAGGCAAGGGGCT GGAGTGGGTGGCAGTTATATCATATGCGGGAACT AATCAATACTATGCAGACTCCGTGAAGGGCCGAT TCACCATTTCCAGAGACAATTCCAAGAACACGCT CTATCTGCAAATGAACAGCCTGAGAGCTGAGGA CACGGCTGTTTATTATTGTGTGAGACGGGGGATAC AGCTATGGCGGGTACGGTATGGACGTCTGGGGC CAAGGGACCACGGTCACCGTCTCCTCA |
| | | | SEQ ID NO: 24977 | SEQ ID NO: 28983 |

FIGURE 50

| | | | | |
|---|---|---|---|---|
| | | AA | DIQMTQSPSSLSASVGDRVTITCQASQDINNYLN WYQQKLGTAPKLLIYDGSNLETGVPSRFSGSGS GTDFTFTISSLQPEDIATYYCQQYENLPITFGQGT RLEIK | QVQLVESGGGVVQPGRSLRLSCAASGFTFSSYGMH WVRQAPGKGLEWVAVISYAGTNQYYADSVKGRF TISRDNSKNTLYLQMNSLRAEDTAVYYCVRRGYSY GGYGMDVWGQGTTVTVSS |
| | | | SEQ ID NO: 24978 | SEQ ID NO: 28984 |
| iPS:394006 | 21-225_15C2 | NA | GACATCCAGATGACCCAGTCTCCATCCTCCCT GTCTGCATCTGTAGGAGACAGAGTCACCATCA CTTGCCAGGCGAGTCAGGACATTACCAACTAT TTAAAATTGGTATCAGCAGAAACCAGGGAAAG CCCCTAACCTCCTGATCTACGATGCATCCAATT TGGAAACAGGGGTCCCATCAAGGTTCAGTGGA AGTGGATCTGGGACAGATTTTACTTTCACCAT CAGCAGCCTGCAGCCTGAAGATATTGCGACAT ATTACTGTCAACAGTATGATAATCTCCCGATC ACCTTCGCCCAAGGGACACGACTGGAGATTAA A | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGTCAATAATATCATATGGTGGACGT AATAATCACTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGTGAGACGGGGGATAC AGCTATGGCGGGTACGGTATGGACGTCTGGGGC CAAGGGACCACGGTCACCGTCTCCTCA |
| | | | SEQ ID NO: 24979 | SEQ ID NO: 28985 |
| | | AA | DIQMTQSPSSLSASVGDRVTITCQASQDITNYLN WYQQKPGKAPNLLIYDASNLETGVPSRFSGSGS GTDFTFTISSLQPEDIATYYCQQYDNLPITFAQGT RLEIK | QVQLVESGGGVVQPGRSLRLSCAASGFTFSSYGMH WVRQAPGKGLEWVSIISYGGRNNHYADSVKGRFTI SRDNSKNTLYLQMNSLRAEDTAVYYCVRRGYSYG GYGMDVWGQGTTVTVSS |
| | | | SEQ ID NO: 24980 | SEQ ID NO: 28986 |

FIGURE 50

| iPS:394008 | 21-225_15H8 | NA | GACATCCAGATGACCCAGTCTCCATCCTCCCT GTCTGCATCGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTTTGCTGCATCCAGTT TGCAAAGTGGCGTCCCATCACGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGCTC ACTTTCGGCGGAGGGACCAAGGTGGAGATCA GA | GAGGTGCAGCTGGTGGAGTCTGGGGGAGGCCTG GTCAAGCCTGGGGGGTCCCTGAGACTCTCCTGTG CAGCCTCTGGATTCACCTTCAGCAGCTATGTCAT GAACTGGGTCCGCCAGGCTCCAGGGAAGGGGCT GGAGTGGGTCTCATCCATTAGTGGTAGTAGTACT TACATATACTGCGCAGACTCAATCAAGGGCCGAT TCACCATCTCCCGAGACAACGCCAAGAACTCTCT GTATCTGCAAATGAACAGCCTGAGAGCCGATGA CACGGCTGTGTATTACTGTGCGAGAGATCGAGGC TCCATCTGGGGCCAAGGGACAATGGTCACCGTCT CTTCA |
| | | | SEQ ID NO: 24981 | SEQ ID NO: 28987 |
| | | AA | DIQMTQSPSSLSASVGDRVTITCRASQGIRNDLG WYQQKPGKAPKRLIFAASSLQSGVPSRFSGSGSG TEFTLTISSLQPEDFATYYCLQHNSYPLTFGGGT KVEIR | EVQLVESGGGLVKPGGSLRLSCAASGFTFSSYVMN WVRQAPGKGLEWVSSISGSSTYIYCADSIKGRFTIS RDNAKNSLYLQMNSLRADDTAVYYCARDRGSIWG QGTMVTVSS |
| | | | SEQ ID NO: 24982 | SEQ ID NO: 28988 |
| iPS:394010 | 21-225_12G5 | NA | GACATCCAGATGACCCAGTCTCCATCCTCCCT GTCTGCCTCTGTAGGAGACAGAGTCACCATCC CTTGCCGGGCGAGTCAGGACATTAGCAATTAT TTAGCCTGGTATCAGCAGAAACCAGGGAAAGT TCCTAAGCTCCTGATCTATGCTGCATACATTTT GCAATCAGGGGTCCCATCTCGGTTCAGTGGCA GTGGATCTGGGACAGATTTCACTCTCACCATC AGCAGCCTGCAGCCTGAAGATGTTGCAGCTTA TTACTGTCAAAAGTATGACAGTGCCCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAACTATGGCAT CTACTGGGTCCGCCAGGCTCCAGGCAAGGGGCT GGAGTGGGTGGCAGTTATATCATATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGCGAGAGATCGGGG AGCAGTGGCTGCTTACTACTACTACGGTATAGAC GTCTGGGGCCAAGGGACCACGGTCACCGTCTCCT CA |
| | | | SEQ ID NO: 24983 | SEQ ID NO: 28989 |

FIGURE 50

| | | | | |
|---|---|---|---|---|
| | | AA | DIQMTQSPSSLSASVGDRVTIPCRASQDISNYLA WYQQKPGKVPKLLIYAAYILQSGVPSRFSGSGSG TDFTLTISSLQPEDVAAYYCQKYDSAPFTFGPGT KVDIK | QVQLVESGGGVVQPGRSLRLSCAASGFTFSNYGIY WVRQAPGKGLEWVAVISYDGSNKYYADSVKGRFT ISRDNSKNTLYLQMNSLRAEDTAVYYCARDRGAV AAYYYYGIDVWGQGTTVTVSS |
| | | | SEQ ID NO: 24984 | SEQ ID NO: 28990 |
| iPS:394012 | 21-225_15A3 | NA | GACATCCAGATGACCCAGTCTCCATCCTCCCT GTCTGCATCTGTAGGAGACAGAGTCACCATCA CTTGCCGGGCAAGTCAGAGCATTATCAGCTAT TTAAATTGGTATCTGCAGAAACCAGGGAAAGC CCCTAAGTTCCTGATCTATACTGCATCCAGTTT GCAAAGTGGGGTCCCATCAAGGTTCAGTGGCA GTGGATCTGGGACAGATTTCACTCTCACCATC AGCAGTCTGCAACCTGAAGATTTTGCAACTTA CTACTGTCAACAGACTTACAGTACCCCGCTCA CTTTCGGCGGAGGGACCAAGGTGGAGATCAA G | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT TCACTGGGTCCGCCAGGCTCCAGGCAAGGGGCT GGAGTGGGTGGCAGTTATATGGCATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGATCTGA GTATGGGCGGTATGGACGTCTGGGGCCAAGGGA CCACGGTCACCGTCTCCTCA |
| | | | SEQ ID NO: 24985 | SEQ ID NO: 28991 |
| | | AA | DIQMTQSPSSLSASVGDRVTITCRASQSIISYLNW YLQKPGKAPKFLIYTASSLQSGVPSRFSGSGSGT DFTLTISSLQPEDFATYYCQQTYSTPLTFGGGTK VEIK | QVQLVESGGGVVQPGRSLRLSCAASGFTFSSYGIH WVRQAPGKGLEWVAVIWHDGSNKYYADSVKGRF TISRDNSKNTLYLQMNSLRAEDTAVYYCARDLSM GGMDVWGQGTTVTVSS |
| | | | SEQ ID NO: 24986 | SEQ ID NO: 28992 |

FIGURE 50

| iPS:394014 | 21-225_8G6 | NA | GACATCCAGATGACCCAGTCTCCATCCTCCCT GTCTGCATCGTAGGAGACAGAGTCACCATCA CTTGCCGGGCAAGTCAGAGCATTAGTAGTTAT TTAAATTGGTATCAGCAGAAACCAGGGAAAG CCCCCAAGCTCCTGATCTTTGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTTCTGTCAACAGAGTTACAGAACCCCCTTT TTCACTTTCGGCCCTGGGACCAAAGTGGATAT CAAA | GAGGTGCAGCTGTTGGAGTCTGGGGGAGGCTTG GTACAGCCTGGGGGGTCCCTGAGACTCTCCTGTG TAGCCTCTGAATTCACCTTTAGCAGCTATGTCAT GAACTGGGTCCGCCAGGCTCCAGGGAAGGGGCT GGAGTGGGTCTCAGTTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTTTGGCA GTGGCTGGCTCGGAGGCTTTTGATATCTGGGGCC AAGGGACAATGGTCAGCGTCTCTTCA |
| | | | SEQ ID NO: 24987 | SEQ ID NO: 28993 |
| | | AA | DIQMTQSPSSLSASVGDRVTITCRASQSISSYLNW YQQKPGKAPKLLIFAASSLQSGVPSRFSGSGSGT DFTLTISSLQPEDFATYFCQQSYRTPFFTFGPGTK VDIK | EVQLLESGGGLVQPGGSLRLSCVASEFTFSSYVMN WVRQAPGKGLEWVSVISGRGGNTFYADSVKGRFTI SRDNSKNTLYLQMNSLRAEDTAVYYCASRLAVAG SBAFDIWGQGTMVSVSS |
| | | | SEQ ID NO: 24988 | SEQ ID NO: 28994 |
| iPS:394016 | 21-225_13D4 | NA | GACCTCCAGATGACCCAGTCTCCATCCTCCCT GTCTGCATCTGTAGGAGACCGAGTCACCATCA CTTGCCGGGCAAGTCAGAGCATTTTCAGCTAC TTAAATTGGTATCAGCAGAAACCAGGGAAAG CCCCTAAGCTCCTGATCTGTACTGCATCCAGTT TGCAAAATGGGGTCCCATCAAGGTTCAGTGGC AGTGGATCTGGGACAGATTTCACTCTCACCAT CAGCAGTCTACAGCCTGAGGATTTTGCAACTT ACTACTGTCAACAGACTTACAGTCTTCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGCATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGATCTGA GTATGGGCGGTATGGACGTCTGGGGCCAAGGGA CCACGGTCACCGTCTCCTCA |
| | | | SEQ ID NO: 24989 | SEQ ID NO: 28995 |

FIGURE 50

| | | | | |
|---|---|---|---|---|
| | | AA | DLQMTQSPSSLSASVGDRVTITCRASQSIFSYLN WYQQKPGKAPKLLICTASSLQNGVPSRFSGSGS GTDFTLTISSLQPEDFATYYCQQTYSLPLTFGGG TKVEIK | QVQLVESGGGVVQPGRSLRLSCAASGFTFSSYGMH WVRQAPGKGLEWVAVIWHDGSNKYYADSVKGRF TISRDNSKNTLYLQMNSLRAEDTAVYYCARDLSM GGMDVWGQGTTVTVSS |
| | | | SEQ ID NO: 24990 | SEQ ID NO: 28996 |
| iPS:394018 | 21-225_15B1 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTAATCTCTGCTGCATCCAGTTT GCAAAGTGGGGTCCCATCAAACTTCAGCGGCA GTGGATCTGGGACAGATTTCACTCTCACCATC AGCAGCCTGCAGCCTGAAGATTTTGCAACTTA TTACTGCCAACAGTATCATAGTTACCCATTCA CTTTCGGCCCTGGGACCAAAGTGGATATCAAA | GAGGTGCAGCTGTTGGAGTCTGGGGGAGGCTTG GTACAGCCGGGGGGGTCCCTGAGACTCTCCTGTG CAACCTCAGGATTCACCTTTAGCAGCTATGTCAT GAGCTGGGTCCGCCAGGCTCCAGGGAAGGGGCT GGAGTGGGTCTCAGGTATTAGTGGTAGTGGTGGT AGCACAAACAACGCAGACTCCGTGAAGGGCCGG TTCACCATCTCCAGAGACAATTCCAAGAACACGC TGTATCTGCAAGTGAACAGCCTGAGAGCCGAGG ACACGGCCGTATATTACTGTGCCCGCAGCTCCTT GTTTGACTACTGGGGCCAGGGAACCCTGGTCACC GTCTCCTCA |
| | | | SEQ ID NO: 24991 | SEQ ID NO: 28997 |
| | | AA | DIQMTQSPSSLSASVGDRVTITCRASQGISNYLA WFQQKPGKAPKSLISAASSLQSGVPSNFSGSGSG TDFTLTISSLQPEDFATYYCQQYHSYPFTFGPGT KVDIK | EVQLLESGGGLVQPGGSLRLSCATSGFTFSSYVMS WVRQAPGKGLEWVSGISGSGGSTNNADSVKGRFTI SRDNSKNTLYLQVNSLRAEDTAVYYCARSSLFDY WGQGTLVTVSS |
| | | | SEQ ID NO: 24992 | SEQ ID NO: 28998 |

FIGURE 50

| iPS:394020 | 21-225_15H10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAAGT TATTACTGTCTACAGCATAATAGTTACCCGCTC ACTTTCGGCGGAGGGACCAAGGTGGAGATCA AT | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGAAAGT AATAAATACTATGAAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGTGAGGGAAGTGGG GTTTCTTTCTGACTACTGGGGCCAGGGAATCCTG GTCACCGTCTCCTCA |
| | | | SEQ ID NO: 24993 | SEQ ID NO: 28999 |
| | | AA | DIQMTQSPSSLSASVGDRVTITCRASQGIRNDLG WYQQKPGKAPKRLIYAASSLQSGVPSRFSGSGS GTEFTLTISSLQPEDFASYYCLQHNSYPLTFGGGT KVEIN | QVQLVESGGGVVQPGRSLRLSCAASGFTFSNYGM HWVRQAPGKGLEWVAVIWYDESNKYYEDSVKGR FTISRDNSKNTLYLQMNSLRAEDTAVYYCVREVGF LSDYWGQGILVTVSS |
| | | | SEQ ID NO: 24994 | SEQ ID NO: 29000 |
| iPS:394022 | 21-225_16H6 | NA | GACATCCAGATGACCCAGTCTCCATCCTCCCT GTCTGCATCTGTAGGAGACAGAGTCACCATCA CTTGCCGGGCAAGTCAGAACATTAGCAGCTAT TTAAATTGGTATCAGCAGAAACCAGGGAAAG CCCCTAAGCTCCTGATCTATGCTGCATCCAGTT TGCAAAGTGGGGTCCCATCAAGGTTCAGTGGC AGAGGATCTGGGACAGATTTCACTCTCACCAT CAGTAGTCTGCAACCTGAAGATTTTGCAACTT ACTACTGTCAACAGAGTTACAGAACCCCCTTA TTCACTTTCGGCCCTGGGACCAAGGTTGATTTC AAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGATGGGGCT GGAGTGGGTCTCAGTTATTAGTCGTAGTGGTGGT TACACATACTACGCGGACTCCGTGAAGGGCCGGT TCACCATCTCCAGAGACAATTCCAAGAACACGCT GTATCTGCAAATGAACAGCCTGAGAGCCGAGGA CACGGCCGTCTATTACTGTGCGTCCCGTTTAGCA GTGGCTGGCTCGGAGGCTTTTGATATCTGGGGCC AAGGGACAATGGTCACCGTCTCTTCA |
| | | | SEQ ID NO: 24995 | SEQ ID NO: 29001 |

FIGURE 50

| | | | | |
|---|---|---|---|---|
| | | AA | DIQMTQSPSSLSASVGDRVTITCRASQNISSYLN WYQQKPGKAPKLLIYAASSLQSGVPSRFSGRGS GTDFTLTISSLQPEDFATYYCQQSYRTPLFTFGPG TKVDFK | EVQLLESGGGLVQPGGSLRLSCAASGFTFSSYAMN WVRQAPGMGLEWVSVISRSGGYTYYADSVKGRFT ISRDNSKNTLYLQMNSLRAEDTAVYYCASRLAVAG SEAFDIWGQGTMVTVSS |
| | | | SEQ ID NO: 24996 | SEQ ID NO: 29002 |
| IPS:394024 | 21-225_16B7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGTTCAA T | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGAACTGGG GTTTCTCTCTGACTACTGGGGCCAGGGAACCCTG GTCACCGTCTCCTCA |
| | | | SEQ ID NO: 24997 | SEQ ID NO: 29003 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEFN | QVQLVESGGGVVQPGRSLRLSCAASGFTFSSYGMH WVRQAPGKGLEWVAVIWYDESNKYYADSVKGRF TISRDNSKNTLYLQMNSLRAEDTAVYYCARELGFL SDYWGQGTLVTVSS |
| | | | SEQ ID NO: 24998 | SEQ ID NO: 29004 |

FIGURE 50

| iPS:394026 | 21-225_16C7 | NA | GACATCCAGATGACCCAGTCTCCATCCTCACT GTCTGCTTCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTCTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGTCAT GACCTGGGTCCGCCAGGCTCCAGGGAAGGGGCT GGAGTGGGTCTCAACTATTAGTGGTAGTGGTGGT TGGACATATTATGCAGACTCCGTGAAGGGCCGGT TCACCATTTCCAGAGACAATTCCAAGAACACGCT GTATCTGCAAATGAACAGCCTGAGAGCCGAGGA CACGGCCGAATATTACTGTGCCCGCAGCTCCTTG TTTGACTATTGGGGCCAGGGAACCCTGGTCACCG TCTCCTCA |
|---|---|---|---|---|
| | | | SEQ ID NO: 24999 | SEQ ID NO: 29005 |
| | | AA | DIQMTQSPSSLSASVGDRVTITCRASQGISNYLA WFQQKPGKAPKSLISAASSLQSGVPSKFSGSGSG TDFTLTISSLQPEDFATYYCQQYNSYPFTFGPGT KVDIK | EVQLLESGGGLVQPGGSLRLSCAASGFTFSSYVMT WVRQAPGKGLEWVSTISGSGGWTYYADSVKGRFT ISRDNSKNTLYLQMNSLRAEDTAEYYCARSSLFDY WGQGTLVTVSS |
| | | | SEQ ID NO: 25000 | SEQ ID NO: 29006 |
| iPS:394029 | 21-225_1B12 | NA | GACATCCAGATGACCCAGTCTCCATCCTCCCT GTCTGCATCTGTAGGAGACAGAGTCACCATCA CTTGCCAGGCGAGTCAGGACATTAACAACTAT TTAAATTGGTATCAACAGAAACCAGGGAAAG CCCCTAAGCTCCTGATATACGATGCATCCAAT TTGGAAACAGGGGTCCCATCAAGGTTCAGTGG AAGTGGATCTGGGACAGATTTTACTTTCACCA TCAGCAGTCTGCAGCCTGAAGATATTACAACA TATTACTGTCAACAGTATGAAAATCTCCCGAT CACCTTCGGCCAAGGGACACGACTGGAGATTA AA | CAGGTGCAGCTGGTAGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAATTATATCATATGCTGGAAGT AATAAATCCTATGCAGACTCCGTGAAGGGCCGAT TCACCATCTCCAGAGACAATTCCAAGAACATGCT GTATCTGCAAATGAATAGCCTGAGAGCTGAGGA CACGGCTGTGTATTACTGTGTGAGACGGGGGATAC AGCTATGGCGGGTACGGTATGGACGTCTGGGGC CAAGGGGCCACGGTCACCGTCTCCTCA |
| | | | SEQ ID NO: 25001 | SEQ ID NO: 29007 |

FIGURE 50

| | | | | |
|---|---|---|---|---|
| | | AA | DIQMTQSPSSLSASVGDRVTITCQASQDINNYLN WYQQKPGKAPKLLIYDASNLETGVPSRFSGSGS GTDFTFTISSLQPEDITTYYCQQYENLPITFGQGT RLEIK | QVQLVESGGGVVQPGRSLRLSCAASGFTFSSYGMH WVRQAPGKGLEWVAIISYAGSNKSYADSVKGRFTI SRDNSKNMLYLQMNSLRAEDTAVYYCVRRGYSY GGYGMDVWGQGATVTVSS |
| | | | SEQ ID NO: 25002 | SEQ ID NO: 29008 |
| IPS:394033 | 21-225_5F4 | NA | GACATCCAGATGACCCAGTCTCCATCCTCCCT GTCTGCATCTGTAGGAGACAGAGTCACCATCA CTTGCCGGGCAAGTCAGGGCATTCGAAATCAT TTAGGCTGGTATCAGCAGAAACCAGGGAAAG CCCCTAAGCGCCTGATCTATGCTGCCTCCAGTT TGCAAAGTGGGGTCCCATCAAGGTTCAGCGGC AGTGGATCTGGGACAGAATTCACTCTCACAGT CAGCAGCCTGCAGCCTGAAGATTTTGCAACTT ATTACTGTCTACAGTATAATGGTTACCCATTCA CTTTCGGCCCTGGGACCAAAGTGGATATCAAA | GAGGTGCAGTTGGTGGAGTCTGGGGGAGGCCTG GTCAAGCCTGGGGGGTCCCTGAGACTCTCCTGTG TAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTTTCTGCAAATGAACAGCCTGAGAGCCGAGGA CACGGCTGTGTATTACTGTGCGAGAGTTAACAAC TTTGACTACTGGGGCCAGGGAACCCTGGTCACCG TCTCCTCA |
| | | | SEQ ID NO: 25003 | SEQ ID NO: 29009 |
| | | AA | DIQMTQSPSSLSASVGDRVTITCRASQGIRNHLG WYQQKPGKAPKRLIYAASSLQSGVPSRFSGSGS GTEFTLTVSSLQPEDFATYYCLQYNGYPFTFGPG TKVDIK | EVQLVESGGGLVKPGGSLRLSCVASGFTFSSYSMN WVRQAPGKGLEWVSSISGSSSYIYYADSVKGRFTIS RDNAKNSLFLQMNSLRAEDTAVYYCARVNNFDY WGQGTLVTVSS |
| | | | SEQ ID NO: 25004 | SEQ ID NO: 29010 |

FIGURE 50

| iPS:394035 | 21-225_5G9 | NA | GACATCCAGATGACCCAGTCTCCATCCTCCCT GTCTGCATCTGTAGGAGACAGAGTCACCATCA CTTGCCAGGCGAGTCAGGGCATTAGCAACTCT TTAAATTGGTATCAGCAGAAACCAGGAAAAG CCCCTAAACTCCTGATCTACGATGCATCCAAT TTGGAAACAGGGGTCCCATCAAGGTTCAGTGG AAGTGGAGCTGGGACAGATTTTACTTTCACCA TCAGCAGCCTGCAGCCTGAAGATATTGCAACA TATTACTGTCAACAATATGATAATCTCCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAATTATGGCAT GCACTGGGTCCGCCAGGCTCCAGGCAAGGGGCT GGAGTGGGTGGCAATTATATCATATGCTGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGTGAGACGTATAAC AGCTCGTCTCTACTACGGTATGGACGTCTGGGGC CAAGGGACCACGGTCACCGTCTCCTCA |
| | | | SEQ ID NO: 25005 | SEQ ID NO: 29011 |
| | | AA | DIQMTQSPSSLSASVGDRVTITCQASQGISNSLN WYQQKPGKAPKLLIYDASNLETGVPSRFSGSGA GTDFTFTISSLQPEDIATYYCQQYDNLPLTFGGG TKVEIK | QVQLVESGGGVVQPGRSLRLSCAASGFTFSNYGM HWVRQAPGKGLEWVAIISYAGSNKYYADSVKGRF TISRDNSKNTLYLQMNSLRAEDTAVYYCVRRITAR LYYGMDVWGQGTTVTVSS |
| | | | SEQ ID NO: 25006 | SEQ ID NO: 29012 |
| iPS:394037 | 21-225_4F4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCGTCCAGT GTGCAAACTGGGGTCCCATCAAGGTTCAGCGG CAGTGGATCTGGGACAGAATTCACTCTCACAA TCAGCAGCCTGCAGCCTGAAGATTTTGCAATT TATTACTGTCTACAGCATAATAGTTACCCGCTC ACTTTCGGCGGAGGGACCAAGGTGGAGATCA AA | CAGCTGCAGCTGCAGGAGTCGGGCCCAGGACTG GTGAGGCCTTCGGAGACCCTGTCCCTCACCTGCA CTGTCTCTGGTGGCTCCATCAGTAGGAGTAGTTA CTACTGGGGCTGGATCCGCCAGCCCCCAGGAAA GGGGCTGGAGTGGATTGGGAATATTTATTATAGT GGGAGCACCTACGACAACCCGTCCCTCAAGAGT CGAGTCACCATATCCGTAGACACGTCCAAGAACC AGTTCTCCCTGAAGCTGAGCTCTGTGACCGCCGC GGACACGGCTGTTTATTACTGTGGGAGACATGGA AAAGACTGGGGCCTTGACTACTGGGGCCAGGGA ACCCTGGTCACCGTCTCCTCA |
| | | | SEQ ID NO: 25007 | SEQ ID NO: 29013 |

FIGURE 50

| | | | | |
|---|---|---|---|---|
| | | AA | DIQMTQSPSSLSASVGDRVTITCRASQGIRNDLG WYQQKPGKAPKRLIYAASSVQTGVPSRFSGSGS GTEFTLTISSLQPEDFAIYYCLQHNSYPLTFGGGT KVEIK | QLQLQESGPGLVRPSETLSLTCTVSGGSISRSSYYW GWIRQPPGKGLEWIGNIYYSGSTYDNPSLKSRVTIS VDTSKNQFSLKLSSVTAADTAVYYCGRHGKDWGL DYWGQGTLVTVSS |
| | | | SEQ ID NO: 25008 | SEQ ID NO: 29014 |
| iPS:394041 | 21-225_5E5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTGGTACGACTACGGTATGGACGTC TGGGGCCAAGGGACCACGGTCACCGTCTCCTCA |
| | | | SEQ ID NO: 25009 | SEQ ID NO: 29015 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDGSNKYYADSVKGR FTISRDNSKNTLYLQMNSLRAEDTAVYYCTREVYS SGWYDYGMDVWGQGTTVTVSS |
| | | | SEQ ID NO: 25010 | SEQ ID NO: 29016 |

FIGURE 50

| | | | | |
|---|---|---|---|---|
| iPS:394043 | 21-225_3B1 | NA | GACATCCAGATGACCCAGTCTCCATCCTCCCTGTCTGCATCTGTAGGAGACAGAGTCACCATCACTTGCCGGGCAAGTCAGAGTATTAATAATTATTTAAATTGGTATCAGCAGAAACCAGGGAAAGCCCCTAAGCTCCTGATCTATGCTACATCCAGTTTGCAAAATGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCCTTATTCACTTTCGGCCCTGGGACCAAAGTGGATATCAAT | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAACTGGGTCCGCCAGGCTCCAGGGAAGGGGCTGGAGTGGGTCTCAGTTATTAGTGGTCGTGGTATTAACACATTCTACGCAGACTCCGTGAAGGGCCGGTTCACCATTTCCAGAGACAATTCCAAGAACACGCTGTATCTGCAAATGAACAGCCTGAGAGCCGAGGACACGGCCGTATATTACTGTGCGTCCCGTTTAGCAGTGGCTGGCTCGGAGGCTTTTGATATCTGGGGCCAAGGGACAATGGTCACCGTCTCTTCA |
| | | | SEQ ID NO: 25011 | SEQ ID NO: 29017 |
| | | AA | DIQMTQSPSSLSASVGDRVTITCRASQSINNYLNWYQQKPGKAPKLLIYATSSLQNGVPSRFSGSGSGTDFTLTISSLQPEDFATYYCQQSYSTPLFTFGPGTKVDIN | EVQLLESGGGLVQPGGSLRLSCAASEFTFSSYAMNWVRQAPGKGLEWVSVISGRGINTFYADSVKGRFTISRDNSKNTLYLQMNSLRAEDTAVYYCASRLAVAGSBAFDIWGQGTMVTVSS |
| | | | SEQ ID NO: 25012 | SEQ ID NO: 29018 |
| iPS:394045 | 21-225_4H4 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCGGGGAAAGCCCCTAAGCGCCTGATCTATGCTGCATCCAGTGTGCAAAGTGGGGTCCCATCAAGGTTCAGCGGCAGTGGATCTGGGACAGAATTCACTCTCACAATCAGCAGCCTGCAGCCTGAAGATTTTGCAATTTATTACTGTCTACAGCATAATAGTTACCCGCTCACTTTCGGCGGAGGGACCAAGGTGGAGATCAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGGAGTAGTTACTACTGGGGCTGGATCCGCCAGCCCCCAGGGATGGGGCTGGAATGGATTGGGAATATTTATTATAGTGGGAACACCTACAACAACCCGTCCCTCAAGAGTCGAGTCACCATATCCGTAGACACGTCCAAGAACCAGTTCTCCCTGAAGCTGAACTCTGTGACCGCCGCAGACACGGCTGTGTATTACTGTGGGAGACATGGAAAAGACTGGGGCCTTGACTACTGGGGCCAGGGAACCCTGGTCATCGTCTCCTCA |
| | | | SEQ ID NO: 25013 | SEQ ID NO: 29019 |

FIGURE 50

| | | | | |
|---|---|---|---|---|
| | | AA | DIQMTQSPSSLSASVGDRVTITCRASQGIRNDLG WYQQKPGKAPKRLIYAASSVQSGVPSRFSGSGS GTEFTLTISSLQPEDFAIYYCLQHNSYPLTFGGGT KVEIK | QLQLQESGPGLVKPSETLSLTCTVSGGSISRSSYYW GWIRQPPGMGLEWIGNIYYSGNTYNNPSLKSRVTIS VDTSKNQFSLKLNSVTAADTAVYYCGRHGKDWGL DYWGQGTLVIVSS |
| | | | SEQ ID NO: 25014 | SEQ ID NO: 29020 |
| iPS:394047 | 21-225_5E6 | NA | GACATCCAGATGACCCAGTCTCCATCCTCCCT GTCTGCATCTGTAGGAGACAGAGTCACCATCA CTTGCCAGGCGAGTCAGGACATTAACAACTAT TTAAATTGGTGTCAACAGAAACCAGGGAAAG CCCCTAAGCTCTTGATATACGATGCATCCAAT TTGGAAACAGGGGTCCCATCAAGGTTCAGTGG AAGTGGATCTGGGACAGATTTTACTTTCACCA TCAGCAGCCTGCAGCCTGAAGATATTGCAACA TATTACTGTCAACAGTATGATAATCTCCCGAT CACCTTCGGCCAAGGGACACGACTGGAGATTA AA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAATTATATCATATGTTGGAAAT AATAAATACTATGCAGACTCCGTGAAGGGCCGA TTCACCATCTCCAGAGACAATTCCAAGAACACGC TGTATCTGCAAATGAACAGCCTGAGAGTTGAGGA CACGGCTGTGTATTACTGTGCGAGACGGGGATAC AGCTATGGCGGGTACGGTATGGACGTCTGGGGC CAAGGGACCACGGTCACCGTCTCCTCA |
| | | | SEQ ID NO: 25015 | SEQ ID NO: 29021 |
| | | AA | DIQMTQSPSSLSASVGDRVTITCQASQDINNYLN WCQQKPGKAPKLLIYDASNLETGVPSRFSGSGS GTDFTFTISSLQPEDIATYYCQQYDNLPITFGQGT RLEIK | QVQLVESGGGVVQPGRSLRLSCAASGFTFSSYGMH WVRQAPGKGLEWVAIISYVGNNKYYADSVKGRFT ISRDNSKNTLYLQMNSLRVEDTAVYYCARRGYSY GGYGMDVWGQGTTVTVSS |
| | | | SEQ ID NO: 25016 | SEQ ID NO: 29022 |

FIGURE 50

| | | | | |
|---|---|---|---|---|
| iPS:394049 | 21-225_13H5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAACTGGGGTCCCATCAAGATTCAGCGG CAGTGGATCTGGGACAGAATTCATTCTCACAA TCAGCAGCCTGCAGCCTGAAGATTTTGCAACT TATTACTGTCTACAGCATAATAGTTACCCTCTC ACTTTCGGCGGAGGGACCAAGGTGGAGATCA AA | CAGCTGCAGCTGCAGGAGTCGGGCCCAGGACTG GTGAAGCCTGCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAGTATCTATTATAGT GGGAGCACCTACTACAACCCGTCCCTCAAGAGTC GAGTCACCATATCCGTGGACACGTCCAAGAACC AGTTCTCCCTGAAGCTGAGCTCTGTGACCGCCGC AGACACGGCTGTGTATTACTGTGCGAGCCTTAGC AGCAGCTGGGACTTCCAGCACTGGGGCCAGGGC ACCCTGGTCACCGTCTCCTCA |
| | | | SEQ ID NO: 25017 | SEQ ID NO: 29023 |
| | | AA | DIQMTQSPSSLSASVGDRVTITCRASQGIRNDLG WYQQKPGKAPKRLIYAASSLQTGVPSRFSGSGS GTEFILTISSLQPEDFATYYCLQHNSYPLTFGGGT KVEIK | QLQLQESGPGLVKPAETLSLTCTVSGGSISRSSYYW GWIRQPPGKGLEWIGSIYYSGSTYYNPSLKSRVTIS VDTSKNQFSLKLSSVTAADTAVYYCASLSSSWDFQ HWGQGTLVTVSS |
| | | | SEQ ID NO: 25018 | SEQ ID NO: 29024 |
| iPS:394051 | 21-225_9E5 | NA | GACATCCAGATGACCCAGTCTCAATCCTCCCT GTCTGCATCTGTAGGAGACAGAGTCACCATCA CTTGCCGGGCAAGTCAGAGCATTGCCAGTTAT TTAAATTGGTATCAGCAGAAACCAGGGAAAG CCCCTAAACTCCTGATCTATGGTGCATCCAGTT TGCAAAGTGGGGTCCCATCAAGGTTCAGTGGC AGTGGATCTGGGACAGATTTCACTCTCACCAT CAGCAGTCTCCAACCTGAAGATTTTGCAACTT ACTACTGTCAACAGAGTTACAGTACCCCCTTA TTCAGTTTCGGCCCTGGGACCAAAGTGGATAT CAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAACTATGCCAT GAACTGGGTCCGCCAGGCTCCAGGGAAGGGGCT GGAGTGGGTCTCAGCTATTAGTGGTGGTGGTGGT AACACATTCTACGCAGACTCCGTGAAGGGCCGGT TCACCATCTCCAGAGACAATTCCAAGAACACGCT GTATCTGCAAATGAACGGCCTGAGAGCCGAGGA CACGGCCGTATATTACTGTGCGTCCCGTATAGCA GTGGCTGGCTCGGAGGCTTTTGCTATCTGGGGCC AAGGGACATTGGTCACCGTCTCTTCA |
| | | | SEQ ID NO: 25019 | SEQ ID NO: 29025 |

FIGURE 50

| | | | | |
|---|---|---|---|---|
| | | AA | DIQMTQSQSSLSASVGDRVTITCRASQSIASYLN WYQQKPGKAPKLLIYGASSLQSGVPSRFSGSGS GTDFTLTISSLQPEDFATYYCQQSYSTPLFSFGPG TKVDIK | EVQLLESGGGLVQPGGSLRLSCAASGFTFSNYAMN WVRQAPGKGLEWVSAISGGGGNTFYADSVKGRFT ISRDNSKNTLYLQMNGLRAEDTAVYYCASRIAVAG SEAFAIWGQGTLVTVSS |
| | | | SEQ ID NO: 25020 | SEQ ID NO: 29026 |
| iPS:394053 | 21-225_11F10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGTTCTGGGACAGAATTCACTCTCACAG TCAGCAGCCTGCAGCCTGAAGATTTTGCAACT TATTACTGTCTACAGCATAGTAGTTACCCTCTC ACTTTCGGCGGAGGGACCAAGGTGGAGATCA AA | CAGCTGCATCTGCAGGAGTCGGGCCCAGGACTG GTGAAGCCTTCGGAGACCCTGTCCCTCACCTGCA CTGTCTCTGGTGCCTCCATCAGCAGAAGTAGTTA CTACTGGGGCTGGATCCGCCAGCCCCCAGGGAA GGGGCTGGAGTGGATTGGGAGTATTTATTATAGT GGGAGCGCCCAGTACAACCCGTCCCTCAAGAGT CGAGTCACCATATCCGTAGACACGTCCAAGAACC AGTTCTCCCTGACGCTGAGCTCTGTGACCGCCGC AGACACGGCTGTATATTACTGTGCGAGACTGAGC AGCAGCTGGTCTTTTGACTACTGGGGCCAGGGAA CCCTGGTCACCGTCTCCTCA |
| | | | SEQ ID NO: 25021 | SEQ ID NO: 29027 |
| | | AA | DIQMTQSPSSLSASVGDRVTITCRASQGIRNDLG WYQQKPGKAPKRLIYAASSLQSGVPSRFSGSGS GTEFTLTVSSLQPEDFATYYCLQHSSYPLTFGGG TKVEIK | QLHLQESGPGLVKPSETLSLTCTVSGASISRSSYYW GWIRQPPGKGLEWIGSIYYSGSAQYNPSLKSRVTIS VDTSKNQFSLTLSSVTAADTAVYYCARLSSSWSFD YWGQGTLVTVSS |
| | | | SEQ ID NO: 25022 | SEQ ID NO: 29028 |

FIGURE 50

| iPS:394055 | 21-225_9C8 | NA | GACATCCAGATGACCCAGTCTCCATCCTCACT GTCTGCATCTGTAGGAGACAGAGTCACCATCA CTTGTCGGGCGAGTCAGGGCATTAGCTATTAT TTAGCCTGGTTTCAGCAGAAACCAGGGAAAGC CCCTAAGTCCCTGATCTATGTTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATGATAGTTACCCATTCA CTTTCGGCCCTGGGACCAAAGTGGATATCAAA | GAGGTGCAACTGGTGGAGTCTGGGGGAGGCTTG GTACAGCCTGGGGGGTCCCTGAGACTCTCCTGTG TAGTCTCTGGATTCACCTTCAGTAGCCAGAGCAT GAACTGGGTCCGCCAGGCTCCAGGGAAGGGGCT GGAGTGGGTCTCATACATTAGTATTAGTAGTACC ATATACTATGCAGACTCTGTGAAGGGCCGATTCA CCATCTCCAGAGACAATGCCAAGAACTCACTGTA TCTGCAAATGAACAGCCTGAGAGACGAGGACAC GGCTGTGTATTACTGTGCGAGAGGAGGTGGGAG CCCTTTTGACTCCTGGGGCCAGGGAACCCTGGTC ACCGTCTCCTCA |
|---|---|---|---|---|
| | | | SEQ ID NO: 25023 | SEQ ID NO: 29029 |
| | | AA | DIQMTQSPSSLSASVGDRVTITCRASQGISYYLA WFQQKPGKAPKSLIYVASSLQSGVPSKFSGSGSG TDFTLTISSLQPEDFATYYCQQYDSYPFTFGPGT KVDIK | EVQLVESGGGLVQPGGSLRLSCVVSGFTFSSQSMN WVRQAPGKGLEWVSYISISSTIYYADSVKGRFTISR DNAKNSLYLQMNSLRDEDTAVYYCARGGGSPFDS WGQGTLVTVSS |
| | | | SEQ ID NO: 25024 | SEQ ID NO: 29030 |
| iPS:394057 | 21-225_15H1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGGTTCAACGG CAGTGGATCTGGGACAGAATTCACTCTCACAA TCAGCAGCCTGCAGCCTGAAGATTTTGCAACT TATTACTGTCTACAGCATAGTAGTTACCCGCTC ACTTTCGGCGGAGGGACCAAGGTGGAGATCA AA | CAGCTGCAGTTGCAGGAGTCGGGCCCAGGACTG GTGAAGTTTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATAGGGAATATCTATTATAGT GGGTATCCCTACTACAATCCGTCCCTCAAGAGTC GAGTCACCATATCCGTAGACACGTCCAAGAACC AGTTCTCCCTGAAGCTGAGCTCTGTGACCGCCGC AGACACGGCTGTGTATTACTGTGCGAGACATAGC ACCAGCTGGTCCCTTGACTACTGGGGCCAGGGAA CCCTGGTCACCGTCTCCTCA |
| | | | SEQ ID NO: 25025 | SEQ ID NO: 29031 |

FIGURE 50

| | | | | |
|---|---|---|---|---|
| | | AA | DIQMTQSPSSLSASVGDRVTITCRASQGIRNDLG WYQQKPGKAPKRLIYTASSLQSGVPSRFNGSGS GTEFTLTISSLQPEDFATYYCLQHSSYPLTFGGGT KVEIK | QLQLQESGPGLVKFSETLSLTCTVSGGSISRSSYYW GWIRQPPGKGLEWIGNIYYSGYPYYNPSLKSRVTIS VDTSKNQFSLKLSSVTAADTAVYYCARHSTSWSLD YWGQGTLVTVSS |
| | | | SEQ ID NO: 25026 | SEQ ID NO: 29032 |
| IPS:394059 | 21-225_9E8 | NA | GACATCCAGATGACCCAGTCTCCATCCTCCCT GTTTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG AAGCGTCTGGATTCACCTTCAGTAGCTATGGCAT GCACTGGGTCCGCCAGGCTCCAGGCAAGGGGCT GGAGTGGGTGGCAGTTATTTGGTATGAAGAAAAT AATCAATACTATGCAGACTCCGTGAAGGGCCGAT TCACCATCTCCAGAGACAATTCCAAGAACACGCT GTATCTACAAATGAACAGCCTGAGAGCCGAGGA CACGGCTGTGTATTACTGTGCGAGAGAACTGGGG TTCCGGTCTGACTACTGGGGCCAGGGAACCCTGG TCACCGTCTCCTCA |
| | | | SEQ ID NO: 25027 | SEQ ID NO: 29033 |
| | | AA | DIQMTQSPSSLFASVGDRVTITCRASQGIRNDLG WYQQKPGKAPKRLIYAASSLQSGVPSRFSGSGS GTEFTLTISSLQPEDFATYYCLQHSYPLTFGGG TKVEIK | QVQLVESGGGVVQPGRSLRLSCEASGFTFSSYGMH WVRQAPGKGLEWVAVIWYEENNQYYADSVKGRF TISRDNSKNTLYLQMNSLRAEDTAVYYCARELGFR SDYWGQGTLVTVSS |
| | | | SEQ ID NO: 25028 | SEQ ID NO: 29034 |

FIGURE 50

| | | | | |
|---|---|---|---|---|
| iPS:394061 | 21-225_12D2 | NA | GATATTGTGATGACTCAGTCTCCACTCTCCCTG CCCGTCACCCCTGGTGAGCCGGCCTCCATCTC CTGCAGGTCTAGTCAGAGCCTCCTCCATAGTA ATGGATACAACTATTTGGATTGGTACCTGCAG AAGCCAGGGCAGTCTCCACAGGTCCTGATCTA TTTGGGTTCTAATCGGGCCTCCGGGGTCCCTG ACAGGTTCAGTGGCAGTGGATCAGGCACAGAT TTTACTCTGAAAATCAGCAGAGTGGAGGCTGA GGATGTTGGGGTTTATTACTGCATGCAAGCTC TACAAACTCCTATCACCTTCGGCCAAGGGACA CGACTGGAGATTAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGATTAGGGGAC TACTGGGGCCAGGGAACCCTGGTCGCCGTCTCCT CA |
| | | | SEQ ID NO: 25029 | SEQ ID NO: 29035 |
| | | AA | DIVMTQSPLSLPVTPGEPASISCRSSQSLLHSNGY NYLDWYLQKPGQSPQVLIYLGSNRASGVPDRFS GSGSGTDFTLKISRVEAEDVGVYYCMQALQTPIT FGQGTRLEIK | EVQLVESGGGLVKPGGSLRLSCAASGFTFSSYSMN WVRQAPGKGLEWVSSISSSSSYIYYADSVKGRFTIS RDNAKNSLYLQMNSLRAEDTAVYYCARLGDYWG QGTLVAVSS |
| | | | SEQ ID NO: 25030 | SEQ ID NO: 29036 |
| iPS:394063 | 21-225_16A1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCGTCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACACCATAGTAATTACCCTCTC ACTTTCGGCGGAGGGACCAAGGTGGAGATCG AA | CAGCTGCAGCTGCAGGAGTCGGGCCCAGGACTG GTGAAGCCTTCGGAGACCCTGTCCCTCATTTGTA CTGTCTCTGGTGGCTCCATCGACAGGAGTAGTTA CTACTGGGGCTGGATCCGCCAGCCCCCAGGGAA GGGGCTGGAGTGGATTGGGAGTATCTATTATAGT GGGAGCGCCTATCACAACCCGTCCCTCAAGAGTC GAGGCACCATATCCGTAGATACGTCCAAGAACC AGTTCTCCCTGAAGCTGAGCTCTGTGACCGCCGC AGACACGGCTGCGTATTACTGTGCGAGACTGAGC AGCAGCTGGTCCTTTGACTACTGGGGCCAGGGAA CCCTGGTCACCGTCTCCTCT |
| | | | SEQ ID NO: 25031 | SEQ ID NO: 29037 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLHHSNYPLTFGGG TKVEIE | QLQLQESGPGLVKPSETLSLICTVSGGSIDRSSYYW GWIRQPPGKGLEWIGSIYYSGSAYHNPSLKSRGTIS VDTSKNQFSLKLSSVTAADTAAYYCARLSSSWSFD YWGQGTLVTVSS |
| | | | SEQ ID NO: 25032 | SEQ ID NO: 29038 |
| iPS:394065 | 21-225_11E2 | NA | GACATCGTGATGACCCAGTCTCCAGACTCCCT GGCTGTGTCTCTGGGCGAGAGGGCCACCATCA ACTGCAAGTCCAACCAGAGAGTTTTATCCAGC TCCAACAATCACAACTACTTAGCTTGGTACCA GCAGAGACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTTTAGTACTCCATTCACTTTCGGCCCTGGG ACCAAAGTGGATATCAAA | CAGGTGCAGGTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACACTAACAGTG GTAACACAGGCTATGCACAGAAGTTCCAGGGCA GAGTCACCATGACCAGGAACACCTCCATAAGCA CAGCCTACATGGACCTGAGCAGCCTGAGATCTGA GGACACGGCCGTATATTACTGTGCGTATAGTCAT GGCTGGTTCCTCTTTGACTACTGGGGCCAGGGAA TCCTGGTCACCGTCTCCTCA |
| | | | SEQ ID NO: 25033 | SEQ ID NO: 29039 |
| | | AA | DIVMTQSPDSLAVSLGERATINCKSNQRVLSSSN NHNYLAWYQQRPGQPPKLLIYWASTRESGVPD RFSGSGSGTDFTLTISSLQAEDVAVYYCQQYFST PFTFGPGTKVDIK | QVQVVQSGAEVKKPGASVKVSCKASGYTFTNYDI NWVRQATGQGLEWMGWMNTNSGNTGYAQKFQG RVTMTRNTSISTAYMDLSSLRSEDTAVYYCAYSHG WFLFDYWGQGILVTVSS |
| | | | SEQ ID NO: 25034 | SEQ ID NO: 29040 |

FIGURE 50

| | | | | |
|---|---|---|---|---|
| iPS:394067 | 21-225_12F2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTACCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGTAACT TATTACTGTCTACAGCATAATAGTTATCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAAT AATAAATACTATGTAGATTCCGTGAAGGGCCGAT TCACCATCTCCAGAGACAATTCCAAGAACACGCT GTTTCTGCAAATGAACAGCCTGAGAGCCGAGGA CACGGCTGTGTATTACTGTGTGAGAGAGCTTGCC TGGTCCGAGGACTACTGGGGCCAGGGAACCCTG GTCACCGTCTCCTCA |
| | | | SEQ ID NO: 25035 | SEQ ID NO: 29041 |
| | | AA | DIQMTQSPSSLSASVGDRVTITCRASQGIRNDLG WYQQKPGKAPKRLIYAASSLQSGVPSRFSGSGS GTEFTLTISSLQPEDFVTYYCLQHNSYPWTFGQG TKVEIK | QVQLVESGGGVVQPGRSLRLSCAASGFTFSDYGM HWVRQAPGKGLEWVAVIWFDGNNKYYVDSVKGR FTISRDNSKNTLFLQMNSLRAEDTAVYYCVRELAW SEDYWGQGTLVTVSS |
| | | | SEQ ID NO: 25036 | SEQ ID NO: 29042 |
| iPS:394069 | 21-225_16H1 | NA | GACATCCAGATGACCCAGTCTCCATCCTCCCT GTCTGCATCTGTAGGAGACAGAGTCACCATCA CTTGCCGGGCAAGTCAGGGCATTAGAGATATT TTAGGCTGGTATCAGCAGAAACCAGGGAAAG CCCCTAAGCGCCTGATCTATGCTGCATCCAGT TTGCAAAATGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGTATCATAGTTACCCATTC ACTTTCGGCCCTGGGACCAAAGTGGATGTCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTACT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTAGCAGCC TTTGACTACTGGGGCCAGGGAACCCTGGTCACCG TCTCCTCA |
| | | | SEQ ID NO: 25037 | SEQ ID NO: 29043 |

FIGURE 50

| | | | | |
|---|---|---|---|---|
| | | AA | DIQMTQSPSSLSASVGDRVTITCRASQGIRDILG WYQQKPGKAPKRLIYAASSLQNGVPSRFSGSGS GTEFTLTISSLQPEDFATYYCLQYHSYPFTFGPGT KVDVK | EVQLVESGGGLVKPGGSLRLSCAASGFTFSSYSMN WVRQAPGKGLEWVSSISGSSTYIYYADSVKGRFTIS RDNAKNSLYLQMNSLRAEDTAVYYCARVAAFDY WGQGTLVTVSS |
| | | | SEQ ID NO: 25038 | SEQ ID NO: 29044 |
| IPS:394071 | 21-225_10C7 | NA | GATATTGTGATGACTCAGTCTCCACTCTCCCTG CCCGTCACCCCTGGAGAGCCGGCCTCCATCTC CTGCAGGTCTAGTCAGAGCCTCCTGCATAGTA AGGGATACAACTATTTGGATTGGTACCTGCAG AAGCCAGGGCAGTCTCCACAGGTCCTGATCTA TTTGGGTTCTAATCGGGCCTCCGGGGTCCCTG ACAGGTTCAGTGGCAGTGGATCAGGCACAGAT TTTACACTGAAAATCAGCAGAGTGGAGGCTGA GGATGTTGGGGTTTATTACTGCATGCAAGCTC TACAAACTCCTCTCACCTTCGGCCAAGGGACA CGACTGGAGATTAAA | GAGGTGCAGCTGGTGGAGTCTGGGGGAGGCCTG GTCAAGCCGGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAATAAT TACATATACTACGCAGACTCAGTGAAGGGCCGAT TCACCATCTCCAGAGACAACGCCAAGAACTCACT GTATCTGCAAATGAACAGCCTGAGAGCCGAGGA CTCGGCTGTGTATTACTGTGCGAGATTAGGGGTC TACTGGGGCCAGGGAACCCTGGTCACCGTCTCCT CA |
| | | | SEQ ID NO: 25039 | SEQ ID NO: 29045 |
| | | AA | DIVMTQSPLSLPVTPGEPASISCRSSQSLLHSKGY NYLDWYLQKPGQSPQVLIYLGSNRASGVPDRFS GSGSGTDFTLKISRVEAEDVGVYYCMQALQTPL TFGQGTRLEIK | EVQLVESGGGLVKPGGSLRLSCAASGFTFSSYSMN WVRQAPGKGLEWVSSISSSNNYIYYADSVKGRFTIS RDNAKNSLYLQMNSLRAEDSAVYYCARLGVYWG QGTLVTVSS |
| | | | SEQ ID NO: 25040 | SEQ ID NO: 29046 |

FIGURE 50

| iPS:394073 | 21-225_15C9 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAACAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAGTCAGCAGCCTGCAGCCTGAAGATTTTGCTACTTATTACTGTCTACAACATACTAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATTGGGAATATCTATTATAGTGGGAGCACCTACAACAACCCGTCCCTCAAGAGTCGAGTCACCATATCCGTAGACACGTCCAAGAACCAGTTCTCCCTGAAGCTGAGCTCTGTGACCGCCGCAGACACGGCTGTGTATTACTGTGCGAGACAGGGCAGTGGCTGGGAGGTTGACTACTGGGGCCAGGGAACCCTGGTCACCGTCTCCTCA |
| | | | SEQ ID NO: 25041 | SEQ ID NO: 29047 |
| | | AA | DIQMTQSPSSLSASVGDRVTITCRASQGIRNDLGWYQQKPGKAPKRLIYAASSLQSGVPSRFSGSGSGTEFTLTVSSLQPEDFATYYCLQHTSYPLTFGGGTKVEIK | QLQLQESGPGLVKPSETLSLTCTVSGGSISRSSYYWGWIRQPPGKGLEWIGNIYYSGSTYNNPSLKSRVTISVDTSKNQFSLKLSSVTAADTAVYYCARQGSGWEVDYWGQGTLVTVSS |
| | | | SEQ ID NO: 25042 | SEQ ID NO: 29048 |
| iPS:394075 | 21-225_8D12 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAACAGAAACCAGGGAAAGCCCCTAAGCGCCTGATCTATGCTGCATCCAGTTTGCAAAGTGGGGTCCCATCAAGGTTCAACGGCAGTGGATCTGGGACAGAATTCACTCTCACAATCAGCAGCCTGCAGCCTGAAGATTTTGCAACTTATTACTGTCTACAGCATAGTAGTTACCCGCTCACTTTCGGCGGAGGGACCAAGGTGGAGATCAAA | CAGCTGCAGTTGCAGGAGTCGGGCCCAGGACTGGTGAAGTTTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATAGGGAATATCTATTATAGTGGGTATCCCTACTACAATCCGTCCCTCAAGAGTCGAGTCACCATATCCATAGACACGTCCAAGAACCAGTTCTCCCTGAAGCTGAGCTCTGTGACCGCCGCAGACACGGCTGTGTATTACTGTGCGAGACATAGCACCAGCTGGTCCCTTGACTACTGGGGCCAGGGAACCCTGGTCACCGTCTCCTCA |
| | | | SEQ ID NO: 25043 | SEQ ID NO: 29049 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFNGSGS GTEFTLTISSLQPEDFATYYCLQHSSYPLTFGGGT KVEIK | QLQLQESGPGLVKFSETLSLTCTVSGGSISRSSYYW GWIRQPPGKGLEWIGNIYYSGYPYYNPSLKSRVTISI DTSKNQFSLKLSSVTAADTAVYYCARHSTSWSLDY WGQGTLVTVSS |
| | | | SEQ ID NO: 25044 | SEQ ID NO: 29050 |
| iPS:394077 | 21-225_8E12 | NA | GACATCCAGATGACCCAGTCTCCATCCTCCCT GTCTGCATCTGTAGGAGACAGAGTCACCATCA CTTGCCGGGCAAGTCAGAGCATTAGTAATTAT TTAAATTGGTATCAGCAGAAACCAGGGAAAG CCCCTAAGCTCCTGATCTATGCTGCATCCAGTT TACAAAGTGGGGTCCCATCAAGGTTCAGTGGC AGTGGATCTGGGACAGATTTCACTCTCACCAT CAGCAGTCTGCAACCTGAAGATTTTGCAACTT ACTTCTGTCAACAGAGTTACAGAACCCCCTTT TTCACTTTCGGCCCTGGGACCAAAGTGGATAT CAAA | GAGGTGCAGCTGTTGGAGTCTGGGGGAGGCTTG GTACAGCCTGGGGGGTCCCTGAGACTCTCCTGTG CAGCCTCTGAATTCACCTTTAGCAGCTATGCCAT GAGCTGGGTCCGCCAGGCTCCAGGGAAGGGGCT GGAGTGGGTCTCAATTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGTCCCGTATGGCA GTGGCTGGCTCGGAGGCTTTTGATATCTGGGGCC AAGGGACAATGGTCACCGTCTCTTCA |
| | | | SEQ ID NO: 25045 | SEQ ID NO: 29051 |
| | | AA | DIQMTQSPSSLSASVGDRVTITCRASQSISNYLN WYQQKPGKAPKLLIYAASSLQSGVPSRFSGSGS GTDFTLTISSLQPEDFATYFCQQSYRTPFFTFGPG TKVDIK | EVQLLESGGGLVQPGGSLRLSCAASEFTFSSYAMS WVRQAPGKGLEWVSIISGRGGNTFYADSVKGRFTI SRDNSKNTLYLQMNSLRAEDTAVYYCASRMAVAG SEAFDIWGQGTMVTVSS |
| | | | SEQ ID NO: 25046 | SEQ ID NO: 29052 |

FIGURE 50

| iPS:394079 | 21-225_11F5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGTCTGATCTATGCTGCATCCAGT GTGCAAAGTGGGGTCCCATCAAGGTTCAGCGG CAGTGGATCTGGGACAGAATTCACTCTCACAA TCAGCAGCCTGCAGCCTGAAGATTTTGCAATT TATTACTGTCTACAGCATAATAGTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGGAGTAGTTA CTACTGGGGCTGGATCCGCCAGCCCCCAGGGAA GGGGCTGGAATGGATTGGAAATATTTATTATAGT GGGAGCACCTACACCAACCCGTCCCTCAAGAGTC GAGTCACCATATCCGTAGACACGTCCAAGAACC ACTTCTCCCTGAAGCTGAGCTCTGTGACCGCCGC AGACACGGCTGTGTATTACTGTGGGAGACATGGA AAAGACTGGGGCCTTGACAACTGGGGCCAGGGA ACCCTGGTCACCGTCTCCTCA |
|---|---|---|---|---|
| | | | SEQ ID NO: 25047 | SEQ ID NO: 29053 |
| | | AA | DIQMTQSPSSLSASVGDRVTITCRASQGIRNDLG WYQQKPGKAPKRLIYAASSVQSGVPSRFSGSGS GTEFTLTISSLQPEDFAIYYCLQHNSYPLTFGGGT KVEIK | QLQLQESGPGLVKPSETLSLTCTVSGGSISRSSYYW GWIRQPPGKGLEWIGNIYYSGSTYTNPSLKSRVTIS VDTSKNHFSLKLSSVTAADTAVYYCGRHGKDWGL DNWGQGTLVTVSS |
| | | | SEQ ID NO: 25048 | SEQ ID NO: 29054 |
| iPS:394081 | 21-225_16B3 | NA | GACATCCAGATGACCCAGTCTCCATCCTCCCT GTCTGCATCTGTAGGAGACAGAGTCACCATCA CTTGCCAGGCGAGTCAGGACATTAACAACTAT TTAAATTGGTATCAGCAGAAACCAGGGAGAG CCCCTAAGCTCCTGATCTACGATGCATCCAAT TTGGAAACAGGGGTCCCATCAAGGTTCAGTGG AAGTGGCTCTGGGACAGATTTTACTTTCACCA TCAGCAGTCTGCAGCCTGAAGATATTGCAACA TATTACTGTCAACAGTTTGATAATCTCCCGATC ACCTTCGGCCAAGGGACACGACTGGAGATTAA A | CAGGTGCAGCTGGTAGAGTCTGGGGGAGGCGTG GTCCAGCCTGGGAGGTCCCTGAGACTCTCCTGTG TAGCCTCTGGATTCACCTTCAGTAGCTATGGCAT GCACTGGGTCCGCCAGGCTCCAGGCAAGGGGCT GGAGTGGGTGGCAGTTATATCATATGCTGGAATT AATAAATCCTATGCAGACTCCGTGAAGGGCCGAT TCACCATCTCCAGAGACAATTCCAATAACACGCT GTATCTGCAAATGAACAGCCTGAGAGCTGAGGA CACGGCTGTGTATTACTGTGTGAGACGGGGATAC AGCTATGGCGGGTATGGTATGGACGTCTGGGGCC AAGGGGCCACGGTCACCGTCTCCTCA |
| | | | SEQ ID NO: 25049 | SEQ ID NO: 29055 |

FIGURE 50

| | | | | |
|---|---|---|---|---|
| | | AA | DIQMTQSPSSLSASVGDRVTITCQASQDINNYLN WYQQKPGRAPKLLIYDASNLETGVPSRFSGSGS GTDFTFTISSLQPEDIATYYCQQFDNLPITFGQGT RLEIK | QVQLVESGGGVVQPGRSLRLSCVASGFTFSSYGMH WVRQAPGKGLEWVAVISYAGINKSYADSVKGRFTI SRDNSNNTLYLQMNSLRAEDTAVYYCVRRGYSYG GYGMDVWGQGATVTVSS |
| | | | SEQ ID NO: 25050 | SEQ ID NO: 29056 |
| iPS:394083 | 21-225_16E6 | NA | GACATCCAGATGACCCAGTCTCCATCCTCCCT GTCTGCATCTGTAGGAGACAGAGTCACCATCA CTTGCCGGGCAAGTCAGAGCATTATCAGCTAT TTAAATTGGTATCAGCAGAAACCAGGGAAAG CCCCTAAGTTCCTGATCTATACTACATCCAGTT TGCAAAGTGGGGTCCCATCAAGGTTCAGTGGC AGTGGATCTGGGACAGATTTCACTCTCACCAT CAGTAGTCTGCAACCTGAAGATTTTGCAACTT ACTACTGTCAGCAGACTTACAGTACCCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGCATGATGGAAG TAATAAATACTATGTAGACTCCGTGAAGGGCCGA TTCACCATCTCCAGAGACAATTCCAAGAACACGC TGAATCTGCAAATGAACAGCCTGAGAGCCGAGG ACACGGCTGTGTATTACTGTGCGAGAGATCTGAG TATGGGCGGTATGGACGTCTGGGGCCAAGGGAC CACGGTCACCGTCTCCTCA |
| | | | SEQ ID NO: 25051 | SEQ ID NO: 29057 |
| | | AA | DIQMTQSPSSLSASVGDRVTITCRASQSIISYLNW YQQKPGKAPKFLIYTTSSLQSGVPSRFSGSGSGT DFTLTISSLQPEDFATYYCQQTYSTPLTFGGGTK VEIK | QVQLVESGGGVVQPGRSLRLSCAASGFTFSSYGMH WVRQAPGKGLEWVAVIWHDGSNKYYVDSVKGRF TISRDNSKNTLNLQMNSLRAEDTAVYYCARDLSM GGMDVWGQGTTVTVSS |
| | | | SEQ ID NO: 25052 | SEQ ID NO: 29058 |

FIGURE 50

| iPS:394085 | 21-225_8B11 | NA | GACATCGTGATGACCCAGTCTCCAGACTCCCT GGCTGTGTCTCTGGGCGAGAGGGCCACCATCA ACTGCAAGTCCAGCCAGAATGTTTTATACAAC TCCAACAATAACAACTACTTAGCTTGGTACCA GCAGAAACCAGGACAGCCTCCTAAGCTGCTCA TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATACTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCGCTGGACAAGGGC TTGAGTGGATGGGATGGATGCACCCTAACAGTGG TAACACAGGCTATGCACAGAAGTTCCAGGGCAG AGTCACCATGACCAGGAACACCTCCAGAAGCAC AGCCTACATGGAGCTGAGCAGCCTGAGATCTGA GGACACGGCCGTGTACTACTGTGCGTATAGCAGT GGCTGGTACTTCTTTGACTACTGGGGCCAGGGAA CCCTGGTCACCGTCTCCTCA |
| | | | SEQ ID NO: 25053 | SEQ ID NO: 29059 |
| | | AA | DIVMTQSPDSLAVSLGERATINCKSSQNVLYNSN NNNYLAWYQQKPGQPPKLLIYWASTRKSGVPD RFSGSGSGTDFTLTISSLQAEDVAVYYCQQYYTT PCSFGQGTKLEIK | QVQLVQSGAEVKKPGASVKVSCKASGYTFTNYDIN WVRQAAGQGLEWMGWMHPNSGNTGYAQKFQGR VTMTRNTSRSTAYMELSSLRSEDTAVYYCAYSSG WYFFDYWGQGTLVTVSS |
| | | | SEQ ID NO: 25054 | SEQ ID NO: 29060 |
| iPS:394087 | 21-225_11A5 | NA | GACATCCAGATGACCCAGTCTCCATCCTCCCT GTCTGCATCTGTAGGAGACAGAGTCACCATCA CTTGCCGGGCAAGTCAGAACATTTATAGTTAT TTAAATTGGTATCAGCAGAAACCAGGGAAAG CCCCTAAGCTCCTGATCTATGCTGCATCCAGTT TGCAAAGTGGGGTCCCATCAAGGTTCAGTGGC AGTGGATCTGGGACAGATTTCACTCTCACCAT CAGCAGTCTACAACCTGAAGATTTTGCAACTT ACTACTGTCAACAGAGTTACAATACCCCCTTA TTCACTTTCGGCCCTGGGACCAAAGTGGATAT CAAA | GAGGTGCAGCTGTTGGAGTCTGGGGGAGACTTG GTTCAGCCTGGGGGGTCCCTGAGACTCTCCTGTG CAGCCTCTGAATTCACCTTTAGCAGCTATGCCAT GAGCTGGGTCCGCCAGGCTCCAGGGAAGGGGCT GGAGTGGATCTCAGTTATTAGTGGTCGTGGTGTT AACACATTCTACGCAGACTCCGTGAAGGGCCGGT TCACCATCTCCAGAGACAATTCCAAGAACACGCT CTATCTACAAATGAACAGCCTGAGAGCCGAGGA CACGGCCGTATATTACTGTGCGTCCCGTATCGCA GTGGCTGGCTCGGAGGCTTTTGATATCTGGGGCC AAGGGACAATGGTCACCGTCTCTTCA |
| | | | SEQ ID NO: 25055 | SEQ ID NO: 29061 |

FIGURE 50

| | | | | | |
|---|---|---|---|---|---|
| | | AA | DIQMTQSPSSLSASVGDRVTITCRASQNIYSYLN WYQQKPGKAPKLLIYAASSLQSGVPSRFSGSGS GTDFTLTISSLQPEDFATYYCQQSYNTPLFTFGPG TKVDIK | EVQLLESGGDLVQPGGSLRLSCAASEFTFSSYAMS WVRQAPGKGLEWISVISGRGVNTFYADSVKGRFTI SRDNSKNTLYLQMNSLRAEDTAVYYCASRIAVAGS EAFDIWGQGTMVTVSS | |
| | | | SEQ ID NO: 25056 | SEQ ID NO: 29062 | |
| IPS:394089 | 21-225_12E6 | NA | GACATCCAGATGACCCAGTCTCCATCCTCCCT GTCTGCATCTGTAGGAGACAGAGTCACCATCA CTTGCCGGGCAAGTCAGGGCATTAGAAGTGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGACAGTTATATGGTATGATGAAAGT AATAAATACTATGCAGACTCCGTGAAGGGCCGA TTCACCATCTCCAGAGACGATTCCAAAAACACGC TGTATCTGCAAATGAACAGTCTGAGAGCCGAGG ACACGGCTGTGTATTACTGTGCGAGAGAGCTTGC CTGGTACGAGGACTACTGGGGGCCAGGGAACCCT GGTCACCGTCTCCTCA | |
| | | | SEQ ID NO: 25057 | SEQ ID NO: 29063 | |
| | | AA | DIQMTQSPSSLSASVGDRVTITCRASQGIRSDLG WYQQKPGKAPKRLIYAASSLQSGVPSRFSGSGS GTEFTLTISSLQPEDFATYYCLQHNSYPWTFGQG TKVEIK | QVQLVESGGGVVQPGRSLRLSCAASGFTFSDYGM HWVRQAPGKGLEWVTVIWYDESNKYYADSVKGR FTISRDDSKNTLYLQMNSLRAEDTAVYYCARELAW YEDYWGQGTLVTVSS | |
| | | | SEQ ID NO: 25058 | SEQ ID NO: 29064 | |

FIGURE 50

| iPS:394091 | 21-225_13H3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCCTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATTTGGTATGAGGAAAGT AATAAATACTATGTAGACTCCGTGAGGGGCCGAT TCACCATCTCCAGAGACAATTCCAAGAGCACGCT GTATCTGCAAATGAACAGCCTGAGAGCCGAGGA CACGGCTGTGTATTACTGTGTGAGAGAACTAGGC TTCCAGTCTGACTTCTGGGGCCAGGGAACCCCGG TCACCGTCTCCTCA |
|---|---|---|---|---|
| | | | SEQ ID NO: 25059 | SEQ ID NO: 29065 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGLVQPGRSLRLSCAASGFTFSSYGMH WVRQAPGKGLEWVAVIWYEESNKYYVDSVRGRF TISRDNSKSTLYLQMNSLRAEDTAVYYCVRELGFQ SDFWGQGTPVTVSS |
| | | | SEQ ID NO: 25060 | SEQ ID NO: 29066 |
| iPS:394093 | 21-225_9D12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGGAAAT AATAATTACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAGCTTGCC TGGTACGAGGACTTCTGGGGCCAGGGAACCCTG GTCACCGTCTCCTCA |
| | | | SEQ ID NO: 25061 | SEQ ID NO: 29067 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPWTFGQG TKVEIK | QVQLVESGGGVVQPGRSLRLSCAASGFTFSDYGM HWVRQAPGKGLEWVAVIWYDGNNNYYADSVKG RFTISRDNSKNTLYLQMNSLRAEDTAVYYCARELA WYEDFWGQGTLVTVSS |
| | | | SEQ ID NO: 25062 | SEQ ID NO: 29068 |
| IPS:394095 | 21-225_16H4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATACTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGTGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGTAAGT AATAAATACTATGCAGACTCCGTGAAGGGCCGA TTCACCATCTCCAGAGACAATTCCAAGAACATGT TGTATCTGCAAATGAACAGCCTGAGAGCCGAGG ACACGGCTGTGTATTACTGTGCGCGAGAAATGGG CTGGACCGATGACTGCTGGGGCCAGGGAACCCT GGTCACCGTCTCCTCA |
| | | | SEQ ID NO: 25063 | SEQ ID NO: 29069 |
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHNSYPWTFGQGT KVEIK | QVQLVESGGGVVQPGRSLRLSCAASGFTFSNYGM HWVRQAPGKGLEWVAVIWYDVSNKYYADSVKGR FTISRDNSKNMLYLQMNSLRAEDTAVYYCAREMG WTDDCWGQGTLVTVSS |
| | | | SEQ ID NO: 25064 | SEQ ID NO: 29070 |

FIGURE 50

| iPS:394097 | 21-225_16G7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCACTGACTATGGCAT GCACTGGGTCCGCCAGGCTCCAGGCAAGGGGCT GGAGTGGGTGGCAGTTATTTGGTATGATGAAAAT AATGAATACTATGCAGACTCCGTGAAGGGCCGA TTCACCATCTCCAGAGCCAATTCCAAGAACACGC TGTATCTGCAAATGAACAGCCTGAGAGCCGAGG ACACGGCTGTGTATTACTGTGCGAGAGAGCTTGC CTGGTACGAGGACTACTGGGGCCAGGGAACCCT GGTCACCGTCTCCTCA |
| | | | SEQ ID NO: 25065 | SEQ ID NO: 29071 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPWTFGQG TKVEIK | QVQLVESGGGVVQPGRSLRLSCAASGFTFTDYGM HWVRQAPGKGLEWVAVIWYDENNEYYADSVKGR FTISRANSKNTLYLQMNSLRAEDTAVYYCARELAW YEDYWGQGTLVTVSS |
| | | | SEQ ID NO: 25066 | SEQ ID NO: 29072 |
| iPS:398470 | 21-225_14B7 | NA | TCCTATGAGCTGACTCAGCCACCCTCAGTGTC CGTGTCCCCAGGACGGACAGCCAGCATCACCT GCTCTGGAGATAAATTGGGGAATAAATATGCT TACTGGTATCAGCAGAAGCCAGGCCAGTCCCC TGTGCTGGTCATCTATCAAGATAGAAAGCGGC CCTCAGGGATCCCTGAGCGATTCTCTGGCTCC AACTCTGGGAACACAGCCACTCTGACCATCAG CGGGACCCAGACTATGGATGAGGCTGACTATT ACTGTCAGGCGTGGAACAACAGCACTGTGGTA TTCGGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTATGTACAGAAGTTTCAGGGCAG GGTCACCATGACCAGGGACACGTCCATCAGCAC AGCCTGCATGGAGCTGAGCAGGCTGAAATCTGA CGACACGGCCGTGTATTTCTGTGCGAGGTCGTTT TTCTATGGTTCGGGGGAGTTATTATAACGAATTTG ACTACTGGGGCCAGGGAACCCTGGTCACCGTCTC CTCA |
| | | | SEQ ID NO: 25067 | SEQ ID NO: 29073 |

FIGURE 50

| | | | | |
|---|---|---|---|---|
| | | AA | SYELTQPPSVSVSPGRTASITCSGDKLGNKYAYW YQQKPGQSPVLVIYQDRKRPSGIPERFSGSNSGN TATLTISGTQTMDEADYYCQAWNNSTVVFGGG TKLTVL | QVQLVQSGAEVKKPGASVKVSCKASGYTFTGYYM HWVRQAPGQGLEWMGWINPNSGGTNYVQKFQGR VTMTRDTSISTACMELSRLKSDDTAVYFCARSFFY GSGSYYNEFDYWGQGTLVTVSS |
| | | | SEQ ID NO: 25068 | SEQ ID NO: 29074 |
| iPS:398472 | 21-225_16E4 | NA | CCCTATGAGCTGACTCAGCCACCCTCAGTGTC CGTGTCCCCAGGACAGACAGCCAGCATCACCT GCTCTGGAGATAAATTGGGGGATAAATATGTT TACTGGTATCAGCAGAAGTCAGGCCAGTCCCC TGTGCTGGTCATCTATCAAGATAGCAAGCGGC CCTCAGGGATCCCTGAGCGATTCTCTGGCTCC AACTCTGGAAACACAGCCGCTCTGACCATCAG CGGGACCCAGGCTATGGATGAGGCTGACTATT ACTGTCAGGCGTGGGACAGCAGCACTGTGGTT TTCGGCGGAGGGACCAAGCTGACCGTCCTA | GAGGTGCAGCTGTTGGAGTCTGGGGGAGGCTTG ATACAGCCTGGGGGGTCCCTGAGACTCTCCTGTG CAGCCTCTGGATTCACCTTTAGTAGCTATGTCAT GAGCTGGGTCCGCCAGGCTCCAGGGAAGGGGCT GGAGTGGGTCTCCACTATTAGTGTTGGTGGTGGT ACCACATACTACGCAGACTCCGTGAAGGGCCGG TTCACCATCTCCAGAGACAATTCCAAGAATACGC TGTATCTGCAAATGAACAGCCTGAGAGCCGAGG ACACGGCCGTATATTACTGTGCGAAATGGGGAC GTGGCAACAGCTATGAGTACTACTACGGTATGGA CGTCTGGGGCCAAGGGACCACGGTCACCGTCTCC TCA |
| | | | SEQ ID NO: 25069 | SEQ ID NO: 29075 |
| | | AA | PYELTQPPSVSVSPGQTASITCSGDKLGDKYVY WYQQKSGQSPVLVIYQDSKRPSGIPERFSGSNSG NTAALTISGTQAMDEADYYCQAWDSSTVVFGG GTKLTVL | EVQLLESGGGLIQPGGSLRLSCAASGFTFSSYVMSW VRQAPGKGLEWVSTISVGGGTTYYADSVKGRFTIS RDNSKNTLYLQMNSLRAEDTAVYYCAKWGRGNS YEYYYGMDVWGQGTTVTVSS |
| | | | SEQ ID NO: 25070 | SEQ ID NO: 29076 |

FIGURE 50

| iPS:398474 | 21-225_17B10 | NA | GACATCCAGATGACCCAGTCTCCATCCTCCCT ATCTGCATCTGTAGGAGACAGAGTCACCATCA CTTGCCGGTCAAGTCAGAGCATTAACAGCTAT TTAAATTGGTATCAGCAGAAACCTGGGAAAGC CCCTAAGCTCCTGATCTTTGCTGCATCCAGTTT GCACAGTGGGGTCCCATCAAGGTTCAGTGGCA GTGGATCTGGGACGGATTTCACTCTCACCATC AGCAGTCTGCAACCTGAAGATTTTGCAACTTA CTACTGTCAACAGGGTTACAATACCCCCACGT GGACGTTCGGCCAAGGGACCAAGGTGGAAAT CAAT | GAGGTGCAGCTGTTGGAGTCTGGGGGAGGCTTG GTACAGCCTGGGGGGTCCCTGAGACTCTCCTGTG CAGCCTCTGGATTCACCTTTAGAAGCTATGCCAT GAGCTGGGTCCGCCAGGCTCCAGGGAAGGGGCT GGAGTGGGTCTCAGTTATTAGTGGTAGTGGTGGT AACACATACTTCGCAGACTCCGTGAAGGGCCGGT TCACCATCTCCAGAGACAATTCCAAGAACACGCT GTATCTGCAAATGGACAGCCTGAGAGCCGAGGA CACGGCCGTATATTACTGTGCGAAAAGGGGTATA CCAGAGGCTGATGCTTTTGATATCTGGGGCCAAG GGACAATGGTCACTGTCTCTTCA |
| | | | SEQ ID NO: 25071 | SEQ ID NO: 29077 |
| | | AA | DIQMTQSPSSLSASVGDRVTITCRSSQSINSYLNW YQQKPGKAPKLLIFAASSLHSGVPSRFSGSGSGT DFTLTISSLQPEDFATYYCQQGYNTPTWTFGQGT KVEIN | EVQLLESGGGLVQPGGSLRLSCAASGFTFRSYAMS WVRQAPGKGLEWVSVISGSGGNTYFADSVKGRFTI SRDNSKNTLYLQMDSLRAEDTAVYYCAKRGIPEAD AFDIWGQGTMVTVSS |
| | | | SEQ ID NO: 25072 | SEQ ID NO: 29078 |
| iPS:398476 | 21-225_17C1 | NA | GACATCCAGATGACCCAGTCTCCATCCTCCCT GTCTGCATCTGTAGGAGACAGAGTCACCATCA CTTGCCGGGCAAGTCAGAACATTAACGACTAT TTAAATTGGTATCAGCAGAAACCAGGGAAAG CCCCTAAGCTCCTGATCTATGCTGCATCCAATT TGCAAAGTGGGGTCCCAGCAAGGTTCAGTGGC AGTCGATCTGGGACAGATTTCACTCTCACCAT CAGCAGTCTGCAACCTGAAGATTTTGCAACTT ACTACTGTCAACAGACTTACAATACCCCTCCG GAGCGCAGTTTTGGCCAGGGGACCAAGCTGG AGATCAAA | GAGGTGCAGCTGTTGGAGTCTGGGGGAGGCTTG GAACAGCCGGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTAGTGGTGGT ACCACATTCTACGCAGACTCCGTGAAGGGCCGGT TCACCATCTCCAGAGACAATTCCAAGAACACGCT GTATCTGCAAATGAGCAGCCTGAGAGCCGAGGA CACGGCCGTATATTTCTGTGCCCGATATTGTAGT AGTACCAGGTGTCCTTATGATGCCTTTGATATCT GGGGCCAAGGGACAATGGTCACCGTCTCCTCA |
| | | | SEQ ID NO: 25073 | SEQ ID NO: 29079 |

FIGURE 50

| | | | | |
|---|---|---|---|---|
| | | AA | DIQMTQSPSSLSASVGDRVTITCRASQNINDYLN WYQQKPGKAPKLLIYAASNLQSGVPARFSGSRS GTDFTLTISSLQPEDFATYYCQQTYNTPPERSFG QGTKLEIK | EVQLLESGGGLEQPGGSLRLSCAASGFTFSSYAMS WVRQAPGKGLEWVSVISGSGGTTFYADSVKGRFTI SRDNSKNTLYLQMSSLRAEDTAVYFCARYCSSTRC PYDAFDIWGQGTMVTVSS |
| | | | SEQ ID NO: 25074 | SEQ ID NO: 29080 |
| IPS:398478 | 21-225_17C10 | NA | GACATCCAGATGACCCAGTCTCCATCCTCCCA GTCTGCATCTGTAGGAGACAGAGTCACCATCA CTTGCCGGGCGAGTCAGGGCATTAGCAATTAT TTAGCCTGGTATCAGCAGAAACCAGGGAGAGT TCCTAAGCTCCTGATCTATGCTGCATCCACTTT GCAATCAGGGGTCCCATCTCGGTTCAGTGGCA GTGGATCTGGGACAGATTTCACTCTCACCATC AGCAGCCTGCAGCCTGACGATGTTGCAACTTA TTACTGTCAAAAGTATAACAGTGCCCCTCCGC TCACCTTCGGCCAAGGGACACGACTGGAGATT AAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATGTACTACGCAGACTCAGTGAAGGGCCGAT TCACCATCTCCAGAGACAACGCCAAGAACTCACT GTATCTGCAAATGAACAGCCTGCGAGCCGAGGA CACGGCTCTGTATTACTGTGCGAGAGATCGTGGG AGCTCCTGGGGCCAGGGAACCCTGGTCACCGTCT CCTCA |
| | | | SEQ ID NO: 25075 | SEQ ID NO: 29081 |
| | | AA | DIQMTQSPSSQSASVGDRVTITCRASQGISNYLA WYQQKPGRVPKLLIYAASTLQSGVPSRFSGSGS GTDFTLTISSLQPDDVATYYCQKYNSAPPLTFGQ GTRLEIK | EVQLVESGGGLVKPGGSLRLSCAASGFTFSSYSMN WVRQAPGKGLEWVSSISGSSSYMYYADSVKGRFTI SRDNAKNSLYLQMNSLRAEDTALYYCARDRGSSW GQGTLVTVSS |
| | | | SEQ ID NO: 25076 | SEQ ID NO: 29082 |

FIGURE 50

| iPS:398480 | 21-225_17G4 | NA | GACATCCAGATGACCCAGTCTCCATCCTCCCTGTCTGCATCTGCAGGAGACAGAGTCACCATCACTTGCCGGACAAGTCAGAATATTAGCAACTATTTAAAATTGGTATCAGCAGAAACCAGGAAAAGCCCCTAAGCTCCTGATCTATGTTGCGTCCAGTTTCCCAAGTGGGGTCCCATCAAGGTTCAGTGGCAGTGGATCTGGGACAGAGTTCACTCTCACCATCAGCAGTCTGCAACCTGAAGATTTTGCAACTTACTACTGTCAACAGAGTAACTTTTTTCCCGCTCACTTTCGGCGGAGGGACCAAGGTGGAGATCATA | CAGGTGCAGTTGGTGCAGTCTGGGGGCTGAGGTGAAGAAGCCTGGGGCCTCAGTGAAGGTCTCCTGCAAGACTTCTGGATACACCTTCACCGACTATTATATGCACTGGGTGCGACAGGCCCCTGGACAAGGGCTTGAGTGGATGGGATGGATCAACCCTAACAGTGGTGGCACAAACTATGAACAGAAGTTTCAGGGCAGGGTCACCATGACCAGGGACACGTCCATCAGCACAGCCTACATGGAACTGAGTAGGCTGAGATCTGACGACACGGCCGTGTATTACTGTGCGAGTGGATACAGCTATGGGTACAACTGGTTCGACCCCTGGGGCCAGGGAACCCTGGTCACCGTCTCCTCA |
| | | | SEQ ID NO: 25077 | SEQ ID NO: 29083 |
| | | AA | DIQMTQSPSSLSASAGDRVTITCRTSQNISNYLNWYQQKPGKAPKLLIYVASSFPSGVPSRFSGSGSGTEFTLTISSLQPEDFATYYCQQSNFFPLTFGGGTKVEII | QVQLVQSGAEVKKPGASVKVSCKTSGYTFTDYYMHWVRQAPGQGLEWMGWINPNSGGTNYEQKFQGRVTMTRDTSISTAYMELSRLRSDDTAVYYCASGYSYGYNWFDPWGQGTLVTVSS |
| | | | SEQ ID NO: 25078 | SEQ ID NO: 29084 |
| iPS:398482 | 21-225_17H6 | NA | GACATCCAGATGACCCAGTCTCCATCCTCACTGTCTGCATCTGTAGGAGACAGAGTCACCATCACTTGTCGGGCGAGTCGGGACATTAGCAATTATTTAGCCTGGTTTCAGCAGAAACCAGGGAAAGCCCCTAAGTCCCTGATCTCTACTGCATCCAGTTTGCAAAGTGGGGTCCCATCAAGGTTCAGTGGCAGTGGATCTGGGACAGATTTCACTCTCACCATCAGCAGCCTGCAGCCTGAAGATTTTGCAATTTATTACTGCCAACAGTATCATAGTTACCCATTCACTTTCGGCCCTGGGACCAAAGTGGATATCCAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAGGGGGCTGGAGTGGGTCTCATCCATTAGTGGTAGTAGTAGTTACATATACTACGCAGACTCAGTGAAGGGCCGATTCACCATCTTCAGAGACAACGCCAAGAACTCACTGTATCTGCAAATGAACAGCCTGAGAGCCGAGGACACGGCTGTGTATTACTGTGCGAGAGTGGCTTCATTTGACTACTGGGGCCAGGGAACCCTGGTCACCGTCTCCTCA |
| | | | SEQ ID NO: 25079 | SEQ ID NO: 29085 |

FIGURE 50

| | | | | |
|---|---|---|---|---|
| | | AA | DIQMTQSPSSLSASVGDRVTITCRASRDISNYLA WFQQKPGKAPKSLISTASSLQSGVPSKFSGSGSG TDFTLTISSLQPEDFAIYYCQQYHSYPFTFGPGTK VDIQ | EVQLVESGGGLVKPGGSLRLSCAASGFTFSSYSMN WVRQAPGRGLEWVSSISGSSSYIYYADSVKGRFTIF RDNAKNSLYLQMNSLRAEDTAVYYCARVASFDY WGQGTLVTVSS |
| | | | SEQ ID NO: 25080 | SEQ ID NO: 29086 |
| IPS:398484 | 21-225_18D4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGGAAAGTGGGGTCCCATCAAGGTTCAGCGG CAGTGGATCTGGGACAGAATTCACTCTCACAG TCAGCAGCCTGCAGCCTGAAGATTTTGCAACT TATTACTGTCTACATCATAATAATTACCTCCCC ATCACCTTCGGCCAAGGGACACGACTGGAGAT TAAA | CAGGTGCAACTGGTGCAGTCTGGGGCTGAGGTG AAGAAGCCTGGGGCCTCCGTGAAGGTCTCCTGCA AGGCTTCTGGATACACCTTCACCGGCTACTATTT GCACTGGGTGCGACAGGCCCCTGGACAAGGGCT TGAGTGGCTGGGATGGATCAACCCTAACAGTAAT GGCACAATCTCTGCACAGAAGTTTCAGGGCAGG GTCACCATGACCAGGGACACGTCCATCAGCACA GCCTACATGGAGCTGAGCAGGCTGATATCTGACG ACACGGCCGTATATTACTGTGCGAGAGATGGTAC CAGCTCGCTTGACTACTGGGGCCAGGGAACCCTG GTCACCGTCTCCTCA |
| | | | SEQ ID NO: 25081 | SEQ ID NO: 29087 |
| | | AA | DIQMTQSPSSLSASVGDRVTITCRASQGIRNDLG WYQQKPGKAPKRLIYAASSLESGVPSRFSGSGSG TEFTLTVSSLQPEDFATYYCLHHNNYLPITFGQG TRLEIK | QVQLVQSGAEVKKPGASVKVSCKASGYTFTGYYL HWVRQAPGQGLEWLGWINPNSNGTISAQKFQGRV TMTRDTSISTAYMELSRLISDDTAVYYCARDGTSSL DYWGQGTLVTVSS |
| | | | SEQ ID NO: 25082 | SEQ ID NO: 29088 |

FIGURE 50

| iPS:398486 | 21-225_19A1 | NA | GACATCCAGATGACCCAGTCTCCATCCTCCCT GTCTGCATCTGTAGGAGACAGAGTCACCATCA CTTGCCGGGCAAGTCATACCATTACCAGCTAT TTAAATTGGTATCAGCAGAAACCAGGGAAAG CCCCTAAGTTCCTGATCTATGCTACATCCAATC TCCAAAGTGGGGTCCCATCAAGGTTCAGTGGC AGTGGATCTGGGACAGATTTCACTTTTACCAT CAGCAGTCTGCAACCTGAAGATTTTGCAATTT ACTACTGTCAACAGAGTTACAACTTCCCGCTC ACTTTCGGCGGAGGGACCAAGGTGGAGATCA AA | CAGGTGCAACTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAATCCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGTGGATAC AGCTATGGGTACAACTGGTTCGACCCCTGGGGCC AGGGAACCCTGGTCACCGTCTCCTCA |
| | | | SEQ ID NO: 25083 | SEQ ID NO: 29089 |
| | | AA | DIQMTQSPSSLSASVGDRVTITCRASHTITSYLN WYQQKPGKAPKFLIYATSNLQSGVPSRFSGSGS GTDFTFTISSLQPEDFAIYYCQQSYNFPLTFGGGT KVEIK | QVQLVQSGAEVKKPGASVKVSCKASGYTFTGYYM HWVRQAPGQGLEWMGWINPNSGGTNYAQKFQGR VTMTRDTSISTAYMELSRLRSDDTAVYYCASGYSY GYNWFDPWGQGTLVTVSS |
| | | | SEQ ID NO: 25084 | SEQ ID NO: 29090 |
| iPS:398488 | 21-225_19F6 | NA | TCCTATGAGCTGACTCAGCCACCCTCAGTGTC CGTGTCCCCAGGACAGACAGTCAGCATCACCT GCTCTGGAGATAAATTGGGGGATAAATATGCT TGCTGGTATCAGCAGAAGCCAGGCCAGTCCCC TGTGCTGGTCATCTATCAAGATAGCAAGCGGC CCTCAGGGACCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAACACTGTGGTA TTCGGCGGAGGGACCAAGCTGACCGTCCTA | GAGGTGCAGCTGGTGGAGTCTGGGGGAGGCTTG GTGAAGCCTGGGGGGTCCCTTAGACTCTCCTGTG CAGCCTCTGGATTCACTTTCAGTAACGCCTGGAT GAACTGGGTCCGCCAGGCTCCAGGGAAGGGGCT GGAGTGGGTTGGCCGTATTAAAAGCAAAACTGA TGGTGGGACAACAGACTACGCTGCACCCGTGAA AGGCAGATTCACCATCTCAAGAGATGATTCAAA AAACACGCTGTATCTGCAAATGAACAGCCTGAA AACCGAGGACACAGCCGTGTATTACTGTACCACA GATACGGGTCCTATAGCAGCTCGTCTCGCTTACT ACTACTATTACGCTATGGACGTCTGGGGCCACGG GACCACGGTCACCGTCTCCTCA |

FIGURE 50

| | | | SEQ ID NO: 25085 | SEQ ID NO: 29091 |
|---|---|---|---|---|
| | | AA | SYELTQPPSVSVSPGQTVSITCSGDKLGDKYACW YQQKPGQSPVLVIYQDSKRPSGTPERFSGSNSGN TATLTISGTQAMDEADYYCQAWDNNTVVFGGG TKLTVL | EVQLVESGGGLVKPGGSLRLSCAASGFTFSNAWM NWVRQAPGKGLEWVGRIKSKTDGGTTDYAAPVK GRFTISRDDSKNTLYLQMNSLKTEDTAVYYCTTDT GPIAARLAYYYYYAMDVWGHGTTVTVSS |
| | | | SEQ ID NO: 25086 | SEQ ID NO: 29092 |
| iPS:398490 | 21-225_21D12 | NA | TCCTATGAGCTGACTCAGCCACCCTCAGTGTC CGTGTCCCCAGGACAGACAGCCAGCATCACCT GCTCTGGAGATAAATTGGGGGAATAAATATGCT TACTGGTATCAGCAGAAGCCAGGCCAGTCCCC TGTGTTGGTCATCTATCAAGATAGAAAGAGGC CCTCAGGGATCCCTGAGCGATTCTCTGGCTCC AACTCTGGGAACACAGCCACTCTGACCATCAG CGGGACCCAGGCTATGGATGAGGCTGACTTTT ACTGTCAGGCGTGGGACAACAGCACTGTGGTA TTCGGCGGAGGGACCAGGCTGACCGTCCTA | CAGGTGCAGCTGGTGCAGTCTGGGGGCTGAGGTG AAGAAACCTGGGGCCTCAGTGAAGGTCTCCTGC AAGGCTTCTGGATACACCTTCACCGACTACTATA TTCACTGGGTGCGACAGGCCCCTGGACAAGGGCT TGAGTGGATGGGGATGGATCAACCCTAACAGTGGT GGGACAAACAATGCACAGAAGTTTCAGGGCAGG GTCACCATGACCAGGGACACGTCCATCAGTACA GCCTACATGGAGCTGAGCAGGCTGAGATCTGAC GATACGGCCGTGTATTCCTGTGCGAGGTCGTATT ACTATGGTTCGGGGACTTATTATAACGAATTTGA CTACTGGGGCCAGGGAACCCTGGTCACCGTCTCC TCA |
| | | | SEQ ID NO: 25087 | SEQ ID NO: 29093 |
| | | AA | SYELTQPPSVSVSPGQTASITCSGDKLGNKYAY WYQQKPGQSPVLVIYQDRKRPSGIPERFSGSNSG NTATLTISGTQAMDEADFYCQAWDNSTVVFGG GTRLTVL | QVQLVQSGAEVKKPGASVKVSCKASGYTFTDYYIH WVRQAPGQGLEWMGWINPNSGGTNNAQKFQGRV TMTRDTSISTAYMELSRLRSDDTAVYSCARSYYYG SGTYYNEFDYWGQGTLVTVSS |
| | | | SEQ ID NO: 25088 | SEQ ID NO: 29094 |

FIGURE 50

| iPS:398494 | 21-225_21H4 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTCTGTCTCCTGGTACCAACAGCACCCAGAC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAGCTCATATACAAGGAGCAG CACTGTGGTATTCGGCGGAGGGACCAAGCTGA CCGTCCTA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTCTTAGTGGTCGTGGTGGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGGGAC GTGGATACAGCTATGAGTACTACTACGGTATGGA CGTCTGGGGCCAAGGGACCACGGTCACCGTCTCC TCA |
| | | | SEQ ID NO: 25089 | SEQ ID NO: 29095 |
| | | AA | QSALTQPASVSGSPGQSITISCTGTSSDVGGYNSV SWYQQHPDKAPKLMIYEVSNRPSGVSNRFSGSK SGNTASLTISGLQAEDEADYYCSSYTRSSTVVFG GGTKLTVL | EVQLLESGGGLVQPGGSLRLSCAASGFTFSSYAMS WVRQAPGKGLEWVSALSGRGGSTYYADSVKGRFT ISRDNSKNTLYLQMNSLRAEDTAVYYCAKWGRGY SYEYYYGMDVWGQGTTVTVSS |
| | | | SEQ ID NO: 25090 | SEQ ID NO: 29096 |
| iPS:398496 | 21-225_22D2 | NA | GACATCGTGATGACCCAGTCTCCAGACTCCCT GGCTGTGTCTCTGGGCGAGAGGGGCCACCATCA CCTGCAAGTCCAGCCAGAGTGTTTTACACAGC TCCAACAATAACAACTACTTAGCTTGGTACCA GCAGAAACCAGGACAGCCTCCTAAGCTACTCA TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTTCTGTCAGCAA TATTATAGTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCATAAATTATGATA TCAACTGGGTGCGACAGGCCACTGGACAAGGGC TTGAGTGGATGGGATGGATGCACCCTGACAGTGG TAACACAGGCTATGCACAGAAGTTCCAGGGCAG AGTCACCATGACCAGGAACACCTCCATAAGCAC AGCCTACATGGAGCTGAGCAGTTTGAGATCTGAG GACACGGCCGTGTATTATTGTGCGTATAGTAGTG GCTGGTACTACTTTGACTACTGGGGCCAGGGAAC CCTGGTCACCGTCTCCTCA |
| | | | SEQ ID NO: 25091 | SEQ ID NO: 29097 |

EP 4 435 105 A2

FIGURE 50

| | | | | |
|---|---|---|---|---|
| | | AA | DIVMTQSPDSLAVSLGERATITCKSSQSVLHSSN NNNYLAWYQQKPGQPPKLLIYWASTRKSGVPD RFSGSGSGTDFTLTISSLQAEDVAVYFCQQYYST PCSFGQGTKLEIK | QVQLVQSGAEVKKPGASVKVSCKASGYTFINYDIN WVRQATGQGLEWMGWMHPDSGNTGYAQKFQGR VTMTRNTSISTAYMELSSLRSEDTAVYYCAYSSGW YYFDYWGQGTLVTVSS |
| | | | SEQ ID NO: 25092 | SEQ ID NO: 29098 |
| iPS:398498 | 21-225_22E6 | NA | TCCTATGAGCTGACTCAGCCACCCTCAGTGTC CGTGTCCCCAGGACAGACAGCCAGCATCACCT GCTCTGGAGATAAAATTGGGGGGAGAAATATGCT TGCTGGTATCAGCAGAAGCCAGGCCAGTCCCC TGTGTTGGTCATCTATCAAGATAGAAAGCGGC CCTCAGGGATCCCTGAGCGATACTCTGGCTCC AACACTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTGGTGGAGTG GGTGTGGGCTGGATCCGTCAGCCCCCAGGAAAG GCCCTGGAGTGGCTTGCACTCATTTATTGGAATG ATGATAAGCGCTACAGCCCATCTCTGAAGAGCA GGCTCACCATCACCAGGGACACCTCCAAAAACC AGGTGGTCCTTACAATGACCAACATGGACCCTGT GGACACAGCCACATATTACTGTGCACACACTATA GCAGTTCGTGGCTTTGACTACTGGGGCCAGGGAA CCCTGGTCACCGTCTCCTCA |
| | | | SEQ ID NO: 25093 | SEQ ID NO: 29099 |
| | | AA | SYELTQPPSVSVSPGQTASITCSGDKLGEKYACW YQQKPGQSPVLVTYQDRKRPSGIPERYSGSNTGN TATLTISGTQAMDEADYYCQAWDSSTAVFGGG TKLTVL | QITLKESGPTLVKPTQTLTLTCTFSGFSLSTGGVGVG WIRQPPGKALEWLALIYWNDDKRYSPSLKSRLTIT RDTSKNQVVLTMTNMDPVDTATYYCAHTIAVRGF DYWGQGTLVTVSS |
| | | | SEQ ID NO: 25094 | SEQ ID NO: 29100 |

1725

FIGURE 50

| iPS:398500 | 21-225_23A11 | NA | GACATCCAGATGACCCAGTCTCCATCCTCACT GTCTACATCTGTAGGAGACAGAGTCACCATCA CTTGTCGGGCGAGTCAGGACATTAGCAATTAT TTAGCCTGGTTTCAGCAGAAACCAGGGAAAGC CCCTAAGCGCCTGATCTATGCTGCATCCACTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATCTCAAG | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGTTATAGCAT GAACTGGATCCGCCAGGCTCCAGGGAAGGGGCT GGAGTGGGTCTCATCCATTAGTGGTAGTAGTACT TACATATACTACGCAGACTCAGTGAAGGGCCGA ATCGCCATCTCCAGAGACAACGCCAAGAACTCA CTGTATCTGCAAATGAACAGCCTGAGAGCCGAG GACACGGCTGTGTATTACTGTGCGAGAGTGGCTT CATTTGACTACTGGGGCCAGGGAACCCTGGTCAC CGTCTCCTCA |
| | | | SEQ ID NO: 25095 | SEQ ID NO: 29101 |
| | | AA | DIQMTQSPSSLSTSVGDRVTITCRASQDISNYLA WFQQKPGKAPKRLIYAASTLQSGVPSKFSGSGS GTDFTLTISSLQPEDFATYYCQQYNSYPFTFGPG TKVDLK | EVQLVESGGGLVKPGGSLRLSCAASGFTFSSYSMN WIRQAPGKGLEWVSSISGSSTYIYYADSVKGRIAIS RDNAKNSLYLQMNSLRAEDTAVYYCARVASFDY WGQGTLVTVSS |
| | | | SEQ ID NO: 25096 | SEQ ID NO: 29102 |
| iPS:398502 | 21-225_23B11 | NA | GACATCCAGATGACCCAGTCTCCATCTTCCGT GTCTGCATCTGTAGGAGACAGAGTCACCATCA CTTGTCGGGCGAGTCAGGGTATTACCAAGTGG TTAGCCTGGTATCAGCAGAAACCAGGGAAAG CCCCTAAAGTCCTGATCTATGCTGCATCCAGTT TGCAAAGTCGGGTCCCATCAAGGTTCAGCGGC AGTAGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATC TGCACTGGGTGCGACAGGCCCCTGGACAAGGGC TTGAGTGGATGGGATGGATCAACCCTAACAATAA TGGCACAAACTATGCACAGAAGTTTCAGGGCAG GGTCACCATGACCAGGGACACGTCCATCAGCAC AGCCTACATGGAGCTGAGCCGGCTGAGATCTGA CGACACGGCCGTGTATTACTGTGCGAGAGATGGT ACCAGCTCGTTTGACTACTGGGGCCAGGGAACCC TGGTCACCGTCTCCTCA |
| | | | SEQ ID NO: 25097 | SEQ ID NO: 29103 |

FIGURE 50

| | | | | |
|---|---|---|---|---|
| | | AA | DIQMTQSPSSVSASVGDRVTITCRASQGITKWLA WYQQKPGKAPKVLIYAASSLQSRVPSRFSGSRS GTDFTLTISSLQPEDFATYYCQQANSFPFTFGPGT KVDIK | QVQLVQSGAEVKKPGASVKVSCKASGYTFTGYYL HWVRQAPGQGLEWMGWINPNNNGTNYAQKFQGR VTMTRDTSISTAYMELSRLRSDDTAVYYCARDGTS SFDYWGQGTLVTVSS |
| | | | SEQ ID NO: 25098 | SEQ ID NO: 29104 |
| iPS:398504 | 21-225_23D7 | NA | TCCTATGAACTGACTCAGCCACCCTCAGTGTC CGTGTCCCCAGGACAGACAGCCAGCATCACCT GCTCTGGAGAAAAATTGGGGGATAAATATGTT TGTTGGTATCAGCAGAAGCCAGGCCAGTCCCC TGTGGTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGAACAGCAGCAATGTGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGCCTTTCACTCATTTATTGGAATA ATGATAAGGTCTACAGCCCATCTCTGAAGAGCAG GCTCACCATCACCAAGTACACCTCCAAAAACCAG GTGGTCCTTACAATGTCCAACATGGACCCTGTGG ACACAGCCACATATTACTGTGCACACAGGGGAC AGCAGCTGGCCCTCGACTACTGGGGCCAGGGAA CCCTGGTCACCGTCTCCTCA |
| | | | SEQ ID NO: 25099 | SEQ ID NO: 29105 |
| | | AA | SYELTQPPSVSVSPGQTASITCSGEKLGDKYVCW YQQKPGQSPVVVIYQDSKRPSGIPERFSGSNSGN TATLTISGTQAMDEADYYCQAWNSSNVVFGGG TKLTVL | QITLKESGPTLVKPTQTLTLTCTFSGFSLSTSGVGVG WIRQPPGKALECLSLIYWNNDKVYSPSLKSRLTITK YTSKNQVVLTMSNMDPVDTATYYCAHRGQQLAL DYWGQGTLVTVSS |
| | | | SEQ ID NO: 25100 | SEQ ID NO: 29106 |

FIGURE 50

| | | | | |
|---|---|---|---|---|
| iPS:398506 | 21-225_23G12 | NA | GACATCGTGATGACCCAGTCTCCAGACTCCCT GGCTGTGTCTCTGGGCGAGAGGGCCACCATCA ACTGCAAGTCCAGCCAGAGTATTTTATTCAGC TCCAACAATAACAACTACTTAGCTTGGTACCA GCAGAAACCAGGACAACCTCCTAAGTTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTTCTGTCAGCAA TATTCTAGTACTCCGTGGACGTTCGGCCAAGG GACCAAGGTGGAAATCAAA | CAGGTGCAGCTGCTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGTACCCTAACAGTGG TAACACGGGCTATGCACAGAAGTTCCAGGGCAG AGTCACCATGACCATGAACACCTCCATAAGCACA GCCTACATGGAGTTGAGCAGCCTGAGATCTGAG GACACGGCCGTGTATTACTGTGCGATTAGCGGTG GCTGGTACTACTTTGACTACTGGGGCCAGGGAAC CCTGGTCACCGTCTCCTCT |
| | | | SEQ ID NO: 25101 | SEQ ID NO: 29107 |
| | | AA | DIVMTQSPDSLAVSLGERATINCKSSQSILFSSNN NNYLAWYQQKPGQPPKLLIYWASTRESGVPDRF SGSGSGTDFTLTISSLQAEDVAVYFCQQYSSTPW TFGQGTKVEIK | QVQLLQSGAEVKKPGASVKVSCKASGYTFTNYDIN WVRQATGQGLEWMGWMYPNSGNTGYAQKFQGR VTMTMNTSISTAYMELSSLRSEDTAVYYCAISGGW YYFDYWGQGTLVTVSS |
| | | | SEQ ID NO: 25102 | SEQ ID NO: 29108 |
| iPS:398508 | 21-225_24B1 | NA | GATGTTGTGATGACTCAGTCTCCACTCTCCCTG CCCGTCACCCTTGGACAGCCGGCCTCCATCTC CTGCAGGTCTAGTCAAAGCCTCGTATACAGTG ATGGAAACACCTACTTGAATTGGTTTCAGCAG AGGCCAGGCCAATCTCCAAGGCGCCTAATTTA TAAGGTTTCTAACTGGGACTCTGGGGTCCCAG ACAGATTCAGCGGCAGTGGGTCAGGCACTGAT TTCACACTGAAAATCAGCTGGGTGGAGGCTGA GGATGTTGGGGTTTGTTACTGCATGCAAGGTG CACACTGGCCTCCGATCACCTTCGGCCAAGGG ACACGACTGGAGATTAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGGGAC GTGGATACAGCTATGAGTACTACTACGGTATGGA CGTCTGGGGCCAGGGGACCACGGTCACCGTCTCC TCA |
| | | | SEQ ID NO: 25103 | SEQ ID NO: 29109 |

FIGURE 50

| | | | | |
|---|---|---|---|---|
| | | AA | DVVMTQSPLSLPVTLGQPASISCRSSQSLVYSDG NTYLNWFQQRPGQSPRRLIYKVSNWDSGVPDRF SGSGSGTDFTLKISWVEAEDVGVCYCMQGAHW PPITFGQGTRLEIK | EVQLLESGGGLVQPGGSLRLSCAASGFTFSSYAMS WVRQAPGKGLEWVSAISGRGGSTYYADSVKGRFTI SRDNSKNTLYLQMNSLRAEDTAVYYCAKWGRGY SYEYYYGMDVWGQGTTVTVSS |
| | | | SEQ ID NO: 25104 | SEQ ID NO: 29110 |
| iPS:398510 | 21-225_25A3 | NA | GACATCGTGATGACCCAGTCTCCAGACTCCCT GACTGTGTCTCTGGGCGAGAGGGCCACCATCA ACTGCAAGTCCAGCCAGAGTGTTTTATACAGC TCCAACAATAAGAACTACTTAGCTTGGTACCA GCAGAAACCTGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAGGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACATCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCTGGAACACCTCCATAAGCACA GCCAACATGGAGCTGAGCAGCCTGAGATCTGAG GACACGGCCGTGTATTACTGTGCGAGTAGCAGTG GCTGGTATTGGTTCGACCCCTGGGGCCAGGGAAC CCTGGTCACCGTCTCCTCA |
| | | | SEQ ID NO: 25105 | SEQ ID NO: 29111 |
| | | AA | DIVMTQSPDSLTVSLGERATINCKSSQSVLYSSN NKNYLAWYQQKPGQPPKLLIYWASTRESGVPD RFSGSGSGTDFTLTISSLQAEDVAVYYCQQYYST PCSFGQGTRLEIK | QVQLVQSGAEVKKPGASVKVSCKASGYIFTNYDIN WVRQATGQGLEWMGWMHPNSGNTGYAQKFQGR VTMTWNTSISTANMELSSLRSEDTAVYYCASSSGW YWFDPWGQGTLVTVSS |
| | | | SEQ ID NO: 25106 | SEQ ID NO: 29112 |

FIGURE 50

| iPS:398512 | 21-225_25E12 | NA | GACATCGTGCTGACCCAGTCTCCAGACTTCCT GGCTATGTCTCTGGGCGAGAGGGCCACCATCA ACTGCAAGTCCAGCCAGAGTGTTTTATACCAC TCCAACAATTACAACTACTTAGCTTGGTACCA GCAGAAACCAAAACAGCCTCCTAAGCTGCTCA TTTACTGGGCATCTACCCGGGAGTCCGGGGTC CCTGACCGATTCAGTGGCAGCGGGTCTGGGAC ATATTTCACTCTCACCATCAGCAGCCTCCAGG CTGAAGATGTGGCAGTTTATTACTGTCAGCAA TATTACAGTACTCCGTGCAGTTTTGGCCAGGG GACCAACCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACCTGGAGCTGAGCAGCCTGAGATCTGA GGACACGGCCGTGTATTACTGTGCGGGGGAGCAA TGGCTGGTACTACTTTGACTACTGGGGCCAGGGA ACCCTGGTCACCGTCTCCTCA |
| | | | SEQ ID NO: 25107 | SEQ ID NO: 29113 |
| | | AA | DIVLTQSPDFLAMSLGERATINCKSSQSVLYHSN NYNYLAWYQQKPKQPPKLLIYWASTRESGVPD RFSGSGSGTYFTLTISSLQAEDVAVYYCQQYYST PCSFGQGTNLEIK | QVQLVQSGAEVKKPGASVKVSCKASGYTFTNYDIN WVRQATGQGLEWMGWMNPNSGNTGYAQKFQGR VTMTRNTSISTAYLELSSLRSEDTAVYYCAGSNGW YYFDYWGQGTLVTVSS |
| | | | SEQ ID NO: 25108 | SEQ ID NO: 29114 |
| iPS:398516 | 21-225_26A9 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATAAGAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGACTTCACTCTCACTATCAGCAGCCTGCAGG CTGAAGATGTGGCAGTTTATTACTGTCAGCAA TATTATAGTAGTCCGTGCAGTTTTGGCCAGGG GACCAGGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAATGGATGGGATGGATGCACCCTAACAGTGG TAACACAGGCTGTGCACAGAAGTTCCAGGGCAG AGTCACCATGACCTGGAACATGTCCATAAGCACA GCCTACATGGAGCTGAGCAGCCTGAGATCTGAG GACACGGCCGTATATTACTGTGCGAGTAGCAGTG GCTGGTACTGGTTCGACCCCTGGGGCCAGGGAAC CCTGGTCACCGTCTCCTCA |
| | | | SEQ ID NO: 25109 | SEQ ID NO: 29115 |

FIGURE 50

| | | | | |
|---|---|---|---|---|
| | | AA | DIVMTQSPDSLAVSLGERATINCKSSQSVLYSSN NKNYLAWYQQKPGQPPKLLIYWASTRESGVPD RFSGSGSGTDFTLTISSLQAEDVAVYYCQQYYSS PCSFGQGTRLEIK | QVQLVQSGAEVKKPGASVKVSCKASGYTFTNYDIN WVRQATGQGLEWMGWMHPNSGNTGCAQKFQGR VTMTWNMSISTAYMELSSLRSEDTAVYYCASSSG WYWFDPWGQGTLVTVSS |
| | | | SEQ ID NO: 25110 | SEQ ID NO: 29116 |
| IPS:398520 | 21-225_31C4 | NA | GACATCCAGATGACCCAGTCTCCATCTTCCGT GTCTGCATCTGTAGGAGACAGAGTCACCATCA CTTGTCGGGCGAGTCAGGGTATTAGCAAATGG TTAGCCTGGTATCAGCAGAAACCAGGGAAAG CCCCTAAACCCCTGATCTATGCTGCATCCAGTT TGCAGAGTGGGGTCCCAACAAGGTTCAGCGGC AGTGGATCTGGGACAGATTTCACTCTCACCAT CAGCAGCCTGCAGCCTGAAGATTTTGCAATTT ACTATTGTCAACAGGCTAACAGTTTCCCATTC ACTTTCGGCCCTGGGACCAAAGTGGATATCAA A | CAGGTGCAGTTGGTGCAGTCTGGGACTGAGGTGA AGAAGCCTGGGGCCTCAGTGAAGGTCTCCTGCA AGGCTTCTGGATACACCTTCACCGGCGATTATAT GCACTGGGTGCGACAGGCCCCTGGACAAGGGCT TGAGTGGATGGGATGGATCAGCCCTAAAAATGG TGGCACAAACTATGCACAGAAGTTTCAGGGCAG GGTCACCATGACCAGGGACACGTCCATCAGCAC AGTCTACATGGAGCTGAACAGGCTGAGATCTGA CGACACGGCCGTGTATTACTGTGCGAGAGATGG AACTGGGTCCTTTGACTACTGGGGCCAGGGAACC CTAGTCACCGTCTCCTCA |
| | | | SEQ ID NO: 25111 | SEQ ID NO: 29117 |
| | | AA | DIQMTQSPSSVSASVGDRVTITCRASQGISKWLA WYQQKPGKAPKPLIYAASSLQSGVPTRFSGSGS GTDFTLTISSLQPEDFAIYYCQQANSFPFTFGPGT KVDIK | QVQLVQSGTEVKKPGASVKVSCKASGYTFTGDYM HWVRQAPGQGLEWMGWISPKNGGTNYAQKFQGR VTMTRDTSISTVYMELNRLRSDDTAVYYCARDGT GSFDYWGQGTLVTVSS |
| | | | SEQ ID NO: 25112 | SEQ ID NO: 29118 |

FIGURE 50

| | | | | |
|---|---|---|---|---|
| iPS:398522 | 21-225_32A1 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATTACAACTACTTAGCTTGGTACCA GCTGAAACCAGGACAGCCTCCTAAGCTGCTCA TTTACTGGGCATCTACCCGGAAATCCGGGGTC CCTGACCGATTCAGTGGCAGCGGGTCTGGGAC AGATTTCACTCTCACCATCAGCAGCCTGCAGA CTGAAGATGTGGCACTTTATTACTGTCAACAA TATTATACTTCTCCGTGCAGTTTTGGCCAGGGG ACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAACTATGATA TTAACTGGGTGCGACAGGCCACTGGACAAGGGC TTGAGTGGATGGGATGGATGAACCCTAACAGTG GTAACACAGGCTATGCACAGAAGTTCCAGGGCC GAGTCACCATGACCAGGAACACCTCCATAAGCA CAGCCTACATGGAGCTGAGCAGCCTGAGATCTG AGGACACGGCCGTGTATTACTGTGCGAGCAGCA GTGGCTGGTACTTTTTTGACTACTGGGGCCAGGG AACCCTGGTCACCGTCTCCTCA |
| | | | SEQ ID NO: 25113 | SEQ ID NO: 29119 |
| | | AA | DIVMTQSPDSLAVSLGERATINCKSSQSVLYSSN NYNYLAWYQLKPGQPPKLLIYWASTRKSGVPD RFSGSGSGTDFTLTISSLQTEDVALYYCQQYYTS PCSFGQGTKLEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCASSSGW YFFDYWGQGTLVTVSS |
| | | | SEQ ID NO: 25114 | SEQ ID NO: 29120 |
| iPS:398524 | 21-225_32A5 | NA | GACATCGTGATGACCCAGTCGCCAGACTCCCT GGCTGTGTCTCTGGGCGAGAGGGCCACCATCA ACTGCAAGTCCAGCCAGAGTGTTTTACACAGC TCCAACAATAAGAACTACTTAGCTTGGTATCA GCAGAAACCAGGACAGCCTCCTAAGCTGCTCA TTTACTGGGCATCTACCCGGGAATCCGGGGTC CCTGACCGCTTCAGTGGCAGCGGGTCTGGGAC AGATTTCACTCTCGCCATCAGCAGCCTGCAGG CTGAAGATGTGGCACTTTATCACTGTCAGCAA TATTATAGTTCTCCGTGCAGTTTTGGCCAGGG GACCGGGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGACA TCAACTGGGTGCGACAGGCCACTGGACAAGGGC TTGAGTGGATGGGATGGATGCACCCTAACAGTGG TAACACAGGCTTTGCACAGAAGTTCCGGGGCAG AGTCACCATGACCAGAAACACCTCCATAAGCAC AGCCTACATGGAACTGAGCAGCCTGAGATCTGA GGACACGGCCGTGTATTACTGTTCGAGCAGCAGT GGCTGGTACTTTTTTGACTACTGGGGCCAGGGAA CCCTGGTCACCGTCTCCTCA |
| | | | SEQ ID NO: 25115 | SEQ ID NO: 29121 |

FIGURE 50

| | | | | |
|---|---|---|---|---|
| | | AA | DIVMTQSPDSLAVSLGERATINCKSSQSVLHSSN NKNYLAWYQQKPGQPPKLLIYWASTRESGVPD RFSGSGSGTDFTLAISSLQAEDVALYHCQQYYSS PCSFGQGTGLEIK | QVQLVQSGAEVKKPGASVKVSCKASGYTFTNYDIN WVRQATGQGLEWMGWMHPNSGNTGFAQKFRGR VTMTRNTSISTAYMELSSLRSEDTAVYYCSSSSGW YFFDYWGQGTLVTVSS |
| | | | SEQ ID NO: 25116 | SEQ ID NO: 29122 |
| IPS:398526 | 21-225_32B3 | NA | GACATCCAGATGACCCAGTCTCCATCCTCACT GTCTGCATCTATAGGAGACAGAGTCACCATCA CTTGTCGGGCGAGTCAGGGCATTAGCAATTAT TTAGCCTGGTTTCAGCAGAAACCAGGGAAAGC CCCTAGGTCCCTGATCTATGCTGCATCCAGTTT ACAAAGTGGGGTCCCATCAACGTTCAGCGGCA GTGGATCTGGGACAGATTTCACTCTCACCATC AGCAGCCTGCAGCCTGAAGATTTTGCAACTTA TTACTGCCAACAGTATAATAGTTATCCATTCA CTTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTACT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTGGCTGGC TTTGACTACTGGGGCCAGGGAACCCTGGTCACCG TCTCCTCA |
| | | | SEQ ID NO: 25117 | SEQ ID NO: 29123 |
| | | AA | DIQMTQSPSSLSASIGDRVTITCRASQGISNYLAW FQQKPGKAPRSLIYAASSLQSGVPSTFSGSGSGT DFTLTISSLQPEDFATYYCQQYNSYPFTFGPGTK VDIK | EVQLVESGGGLVKPGGSLRLSCAASGFTFSSYSMN WVRQAPGKGLEWVSSISGSSTYIYYADSVKGRFTIS RDNAKNSLYLQMNSLRAEDTAVYYCARVAGFDY WGQGTLVTVSS |
| | | | SEQ ID NO: 25118 | SEQ ID NO: 29124 |

FIGURE 50

| iPS:398528 | 21-225_32G1 | NA | GACATCCAGATGACCCAGTCTCCATCCTCCCT GTCTGCATCTGTAGGAGACAGAGTCACCATCA CTTGCCGGGCAAGTCAGGACATGAGAAGTGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATACTATTTCCCCTCCT ACTTTCGGCGGAGGGACCAAGGTGGAGATCA AA | GAGGTGCAGCTGTTGGAGTCTGGGGGAGGCTTG GCACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCCGCTATTAGTGGTCGTGGTGGT AGCACATTCCACGCAGACTCCGTGAAGGGCCGG TTCACGATCTCCAGAGACAATTCCAAGAACACGC TGTATCTGCAAATGAACAGCCTGAGAGCCGAGG ACACGGCCGTATATTACTGTGTGAAAGGGGAGCT ACTAGAGGACTACTACTTCTACGGTATGGACGTC TGGGGCCAAGGGACCACGGTCACCGTCTCCTCA |
| | | | SEQ ID NO: 25119 | SEQ ID NO: 29125 |
| | | AA | DIQMTQSPSSLSASVGDRVTITCRASQDMRSDLG WYQQKPGKAPKRLIYAASSLQSGVPSRFSGSGS GTEFTLTISSLQPEDFATYYCLQHTISPPTFGGGT KVEIK | EVQLLESGGGLAQPGGSLRLSCAASGFTFSSYAMS WVRQAPGKGLEWVSAISGRGGSTFHADSVKGRFTI SRDNSKNTLYLQMNSLRAEDTAVYYCVKGELLED YYFYGMDVWGQGTTVTVSS |
| | | | SEQ ID NO: 25120 | SEQ ID NO: 29126 |
| iPS:398530 | 21-225_32G4 | NA | GATGTTGTGATGACTCAGTCTCCACTCTCCCTG TCCGTCACCCTTGGACAGCCGGCCTCCATCTC CTGCAGGTCAAGTCAAAGCCTCGTATACAGTG ATGGAAACACCTACTTGAATTGGTTTCAGCAG AGGCCAGGCCAATCTCCAAGGCGCCTAATTTA TAAGGTTTCTAACTGGGACTCTGGGGTCCCAG ACAGATTCAGCGGCAGTGGGTCAGGCACAGA TTTCACACTGAAAATCAGCAGGGTGGAGGCTG AGGATGTTGGGGTTTATTACTGCATGCAAGGT ATACACTGGCTCACTTTCGGCGGAGGGACCAA GGTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGCGAAAGA AGGCTAACGACTACTGGGGCCAGGGAACCCTGG TCACCGTCTCCTCA |
| | | | SEQ ID NO: 25121 | SEQ ID NO: 29127 |

FIGURE 50

| | | | | |
|---|---|---|---|---|
| | | AA | DVVMTQSPLSLSVTLGQPASISCRSSQSLVYSDG NTYLNWFQQRPGQSPRRLIYKVSNWDSGVPDRF SGSGSGTDFTLKISRVEAEDVGVYYCMQGIHWL TFGGGTKVEIK | QVQLVQSGAEVKKPGASVKVSCKASGYTFTSYDIN WVRQATGQGLEWMGWMNPNSGNTGYAQKFQGR VTMTRNTSISTAYMELSSLRSEDTAVYYCARKKAN DYWGQGTLVTVSS |
| | | | SEQ ID NO: 25122 | SEQ ID NO: 29128 |
| IPS:398532 | 21-225_33B7 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTGGCCTGGTTTCAGCAGAAACCAGGGAAAGC CCCTACGTCCCTGATCTATGCTGCATCTAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTT TTACTGCCAACAGTATCATAGTTACCCGCTCA CCTTCGGCCAAGGGACACGACTGGAAATTAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAACAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGGTTAAATGGT TTTGACTACTGGGGCCAGGGAACCCTGGTCACCG TCTCCTCA |
| | | | SEQ ID NO: 25123 | SEQ ID NO: 29129 |
| | | AA | DIQMTQSPSSLSASVGDRVTITCRASQDISNYLA WFQQKPGKAPTSLIYAASSLQSGVPSKFSGSGSG TDFTLTISSLQPEDFATFYCQQYHSYPLTFGQGT RLEIK | EVQLVESGGGLVKPGGSLRLSCAASGFTFSSYNMN WVRQAPGKGLEWVSSISGSSSYIYYADSVKGRFTIS RDNAKNSLYLQMNSLRAEDTAVYYCARLNGFDY WGQGTLVTVSS |
| | | | SEQ ID NO: 25124 | SEQ ID NO: 29130 |

FIGURE 50

| | | | | |
|---|---|---|---|---|
| iPS:398534 | 21-225_33B8 | NA | GACATCCAGATGACCCAGTCTCCATCCTCCCT GTCTGCATCTGTAGGAGACAGAGTCACCATCA CTTGCCGGGCAAGTCAGGACATTAGAAGTGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTTCTGTCTACAGCATACTATTTACCCTCCT ACTTTCGGCGGAGGGACCAAGGTGGAGATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCCGCTATTAGTGGTCGTGGTGGT AGCACATTCCACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGTGAAAGGGGAGCT ACTAGAGGACTACTACTTCTACGGTATGGACGTC TGGGGCCAAGGGACCACGGTCACCGTCTCCTCA |
| | | | SEQ ID NO: 25125 | SEQ ID NO: 29131 |
| | | AA | DIQMTQSPSSLSASVGDRVTITCRASQDIRSDLG WYQQKPGKAPKRLIYAASSLQSGVPSRFSGSGS GTEFTLTISSLQPEDFATYFCLQHTIYPPTFGGGT KVEIK | EVQLLESGGGLVQPGGSLRLSCAASGFTFSSYAMS WVRQAPGKGLEWVSAISGRGGSTFHADSVKGRFTI SRDNSKNTLYLQMNSLRAEDTAVYYCVKGELLED YYFYGMDVWGQGTTVTVSS |
| | | | SEQ ID NO: 25126 | SEQ ID NO: 29132 |
| iPS:398536 | 21-225_33D12 | NA | GACGTCCAGATGACCCAGTCTCCATCCTCCCT GTCTGCATCTTTAGGAGACAGAGTCACCATCA CTTGCCGGGCAAGTCAGAGCATTAGAAGCTAT TTAAATTGGTATCAGCAGAAACCAGGGAAAG CCCCTAACCTCCTGATCTATAGTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAAATT ACTACTGTCAACAGAGTTACAGTATCCCGATC ACCTTCGGCCAAGGGACACGACTGGAGATTAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCAGTTATGATA TCAGCTGGGTGCGACTGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGAAAGA GGGCTAACGACTACTGGGGCCAGGGAACCCTGG TCACCGTCTCCTCA |
| | | | SEQ ID NO: 25127 | SEQ ID NO: 29133 |

FIGURE 50

| | | | | |
|---|---|---|---|---|
| | | AA | DVQMTQSPSSLSASLGDRVTITCRASQSIRSYLN WYQQKPGKAPNLLIYSASSLQSGVPSRFSGSGSG TDFTLTISSLQPEDFANYYCQQSYSIPITFGQGTR LEIK | QVQLVQSGAEVKKPGASVKVSCKASGYTFTSYDIS WVRLATGQGLEWMGWMNPNSGNTGYAQKFQGR VTMTRNTSISTAYMELSSLRSEDTAVYYCARKRAN DYWGQGTLVTVSS |
| | | | SEQ ID NO: 25128 | SEQ ID NO: 29134 |
| iPS:398538 | 21-225_34H7 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATTACAACTACTTAGCTTGGTACCA GCTGAAACCAGGACAGCCTCCTAAGCTGCTCA TTTACTGGGCATCTACCCGGAAATCCGGGGTC CCTGACCGATTCAGTGGCAGCGGGTCTGGGAC AGATTTCACTCTCACCATCAGCAGCCTGCAGA CTGAAGATGTGGCACTTTATTACTGTCAGCAA TATTATACTTCTCCGTGCAGTTTTGGCCAGGGG ACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAACTATGATA TTAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAACAGCCTGAGATCTG AGGACACGGCCGTGTATTACTGTGCGAGCAGCA GTGGCTGGTACTTTTTTGACTACTGGGGCCAGGG AACCCTGGTCACCGTCTCCTCA |
| | | | SEQ ID NO: 25129 | SEQ ID NO: 29135 |
| | | AA | DIVMTQSPDSLAVSLGERATINCKSSQSVLYSSN NYNYLAWYQLKPGQPPKLLIYWASTRKSGVPD RFSGSGSGTDFTLTISSLQTEDVALYYCQQYYTS PCSFGQGTKLEIK | QVQLVQSGAEVKKPGASVKVSCKASGYTFTNYDIN WVRQATGQGLEWMGWMNPNSGNTGYAQKFQGR VTMTRNTSISTAYMELNSLRSEDTAVYYCASSSGW YFFDYWGQGTLVTVSS |
| | | | SEQ ID NO: 25130 | SEQ ID NO: 29136 |

FIGURE 50

| iPS:398540 | 21-225_35A6 | NA | GACATCCAGATGACCCAGTCTCCATCCTCCCT GTCCGCATCGTAGGAGACAGAGTCACCATCA CTTGCCGGGCAAGTCAGGACATTAGAAGTGAT TTAGGCTGGTATCAGCAGAAACCAGGGAAAG CCCCTAAGCGCCTGATCTATGCTACATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATACTATTTACCCTCCT ACTTTCGGCGGAGGGACCAAGGTGGAGATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCCACTATTAGTGGTCGTGGTGGT AGCACATTCCACGCAGACTCCGTGAAGGGCCGG TTCACCATCTCCAGAGACAATTCCAAGAACACGC TGTATCTGCAAATGAACAGCCTGAGAGCCGAAG ACACGGCCGTATATTACTGTGTGAAAGGGGAGCT ACTAGAGGACTACTACTTCTACGGTATGGACGTC TGGGGCCAAGGGACCACGGTCACCGTCTCCTCA |
| | | | SEQ ID NO: 25131 | SEQ ID NO: 29137 |
| | | AA | DIQMTQSPSSLSASVGDRVTITCRASQDIRSDLG WYQQKPGKAPKRLIYATSSLQSGVPSRFSGSGSG TEFTLTISSLQPEDFATYYCLQHTIYPPTFGGGTK VEIK | EVQLLESGGGLVQPGGSLRLSCAASGFTFSSYAMS WVRQAPGKGLEWVSTISGRGGSTFHADSVKGRFTI SRDNSKNTLYLQMNSLRAEDTAVYYCVKGELLED YYFYGMDVWGQGTTVTVSS |
| | | | SEQ ID NO: 25132 | SEQ ID NO: 29138 |
| iPS:398544 | 21-225_7C8 | NA | CTGCCTGTGCTGACTCAGCCCCCGTCTGCATCT GCCTTGCTGGGAGCCTCGATCAAGCTCACCTG CACCCTAAGCAGTGAGCACAGCACCTACACCA TCGAATGGTATCAACAGAGACCAGGGAGGTC CCCCCAGTATATAATGAAGGTTAAGAGTGATG GCAGCCACAGCAAGGGGGACGGGATCCCCGA TCGCTTCATGGGCTCCAGTTCTGGGGGTGACC GCTACCTCACCTTCTCCAACCTCCAGTCTGACG ATGAGGATGAGTATCACTGTGGAGAGAGCCA CCCGATTGATGGCCAAGTCGGTGTGGTATTCG GCGGAGGGACCAAGCTGACCGTCCTA | GAGGTGCAGCTGGTGGAGTCTGGGGGAGGCTTG GTAAAGCCTGGGGGGTCCCTTAGACTCTCCTGTG CAGCCTCTGGATTCACTTTCAGTAACGCCCGGAT GAACTGGGTCCGCCTGGCTCCAGGGAAGGGGCT GGAGTGGGTTGGCCGTATTAAAAGCAAAACTGA TGGTGGGACAACAGACTACGCTGCACCCGTGAA AGGCAGATTCACCATCTCAAGAGATGAATCAGA AAACACGCTGTATCTGCAAATGAACAGCCTGAA AACCGAGGACACAGGTGTGTATTACTGTTCCACA GATACGGGTCCTATAGCAGCTCGTCTCGCTTACT ACTACTACTACGCTATGGACGTCTGGGGCCAAGG GACCACGGTCACCGTCTCCTCA |

EP 4 435 105 A2

FIGURE 50

| | | | SEQ ID NO: 25133 | SEQ ID NO: 29139 |
|---|---|---|---|---|
| | | AA | LPVLTQPPSASALLGASIKLTCTLSSEHSTYTIEW YQQRPGRSPQYIMKVKSDGSHSKGDGIPDRFMG SSSGGDRYLTFSNLQSDDEDEYHCGESHPIDGQV GVVFGGGTKLTVL | EVQLVESGGGLVKPGGSLRLSCAASGFTFSNARMN WVRLAPGKGLEWVGRIKSKTDGGTTDYAAPVKGR FTISRDESENTLYLQMNSLKTEDTGVYYCSTDTGPI AARLAYYYYYAMDVWGQGTTVTVSS |
| | | | SEQ ID NO: 25134 | SEQ ID NO: 29140 |
| iPS:398546 | 21-225_9H10 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTTATGTG GTATTCGGCGGAGGGACCAAGCTGACCGTCCT A | CAGATCACCTTGAAGGAGTCTGGTCCTACGCTGG TGAAACCCACACAGACCCTCACGCTGACCTGCAC CTTCTCTGGGTTCTCACTCACCACTAGTGGAGTG GGTGTGGGCTGGATCCGTCAGCCCCCAGGAAAG GCCCTGGAATGGCTTGCACTCATTTATTGGAGTG ATGATAAGCGCTACAGCCCATCTCTGAAGAGCA GGCTCACCATCACCAAGGACACCTCCAAAAACC AGGTGGTCCTTACAATGACCAACATGGCCCCTGT GGACACAGCCACATATTACTGTGCACACACCGGT TCTAGCTGCTGCTATTTTGACTACTGGGGCCAGG GAACCCTGGTCACCGTCTCCTCA |
| | | | SEQ ID NO: 25135 | SEQ ID NO: 29141 |
| | | AA | SYELTQPPSVSVSPGQTASITCSGDKLGDKYACW YQQKPGQSPVLVIYQDSKRPSGIPERFSGSNSGN TATLTISGTQAMDEADYYCQAWDSSTYVVFGG GTKLTVL | QITLKESGPTLVKPTQTLTLTCTFSGFSLTTSGVGVG WIRQPPGKALEWLALIYWSDDKRYSPSLKSRLTITK DTSKNQVVLTMTNMAPVDTATYYCAHTGSSCCYF DYWGQGTLVTVSS |
| | | | SEQ ID NO: 25136 | SEQ ID NO: 29142 |

1739

FIGURE 50

| iPS:402219 | 21-225_1C12 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGCTCACTTTCGGCGGAGGGACCAAGGTGGCGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGAAAATAATAAATACTATGTAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGAATTGGGGTTCCGGTCTGACTACTGGGGCCAGGGAACCCTGGTCACCGTCTCCTCA |
| | | | SEQ ID NO: 25137 | SEQ ID NO: 29143 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVAIK | QVQLVESGGGVVQPGRSLRLSCAASGFTFSNYGMHWVRQAPGKGLEWVAVIWYDENNKYYVDSVKGRFTISRDNSKNTLYLQMNSLRAEDTAVYYCARELGFRSDYWGQGTLVTVSS |
| | | | SEQ ID NO: 25138 | SEQ ID NO: 29144 |
| iPS:402221 | 21-225_2C12 | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAATCAGGGAAAGCCCCTAAGTCTCTGATCTCTGCTGCAACCAGTTTGCAAAGTGGGGTCCCATCACAGTTCAGCGGCAGTGGATCTGGGACAGATTTCACTCTCACCATCAGCAGCCTGCAGCCTGAAGATTTTGCAACTTATTACTGCCAACAGTATTATAGTTACCCGATCACCTTCGGCCAAGGGACACGACTGGAGATTAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATGTACTACGCAGACTCAGTGAAGGGCCGATTCACCATCTCCAGAGACAACGCCAAGGACTCACTGTATCTGCAAATGAACAGCCTGAGAGCCGAGGACACGGCTGTGTATTACTGTGCGAGAGTGAATCTCTTTGACTACTGGGGCCAGGGAACCCTGGTCACCGTCTCCTCA |
| | | | SEQ ID NO: 25139 | SEQ ID NO: 29145 |

FIGURE 50

| | | | | |
|---|---|---|---|---|
| | | AA | DIQMTQSPSSLSASVGDRVTITCRASQGISNYLA WFQQKSGKAPKSLISAATSLQSGVPSQFSGSGSG TDFTLTISSLQPEDFATYYCQQYYSYPITFGQGTR LEIK | EVQLVESGGGLVKPGGSLRLSCAASGFTFSSYSMN WVRQAPGKGLEWVSSISGSSSYMYYADSVKGRFTI SRDNAKDSLYLQMNSLRAEDTAVYYCARVNLFDY WGQGTLVTVSS |
| | | | SEQ ID NO: 25140 | SEQ ID NO: 29146 |
| IPS:402223 | 21-225_30A11 | NA | GACATCCAGATGACCCAGTCTCCATCTTCCGT GTCTGCATCTGTAGGAGACAGAGTCACCATCA CTTGTCGGGCGAGTCAGGGTATTAGCAGATGG TTAGCCTGGTATCAGCAGAAACCAGGGAGAG CCCCTGAACTCCTGATCTATGCTGCATCCCGTT TGCAAAGTGGGATCCCATCCAGGTTCAGCGGC AGTGGATCTGGGACAGACTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATAACAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGACTATCATA TGCACTGGGTGCGACAGGCCCCTGGACAAGGGC TTGAGTGGATGGGATGGATCAACCCTAATAGGG GTGGCACAAACTATGCACAGAAGTTTCAGGACA GGGTCACCATGACCAGGGACACGTCCATCAGCA CAGCCTACATGGAACTGAGCAGGCTGAGATCTG ACGACACGGCCGTCTATTTCTGTGCGAGAGATGG AACTGGGTCCTTTGACTACTGGGGCCAGGGAACC CTGGTCACCGTCTCCTCA |
| | | | SEQ ID NO: 25141 | SEQ ID NO: 29147 |
| | | AA | DIQMTQSPSSVSASVGDRVTITCRASQGISRWLA WYQQKPGRAPELLIYAASRLQSGIPSRFSGSGSG TDFTLTISSLQPEDFATYYCQQANSFPFTFGPGTK VDNK | QVQLVQSGAEVKKPGASVKVSCKASGYTFTDYHM HWVRQAPGQGLEWMGWINPNRGGTNYAQKFQDR VTMTRDTSISTAYMELSRLRSDDTAVYFCARDGTG SFDYWGQGTLVTVSS |
| | | | SEQ ID NO: 25142 | SEQ ID NO: 29148 |

FIGURE 50

| | | | | |
|---|---|---|---|---|
| iPS:402225 | 21-225_2B1 | NA | TCCTATGAGCTGACTCAGCCACCCTCAGTGTC CGTGTCCCCAGGACAGACAGTCAGCATCACCT GCTCTGGAGATAAATTGGGGGATAAATATGCT TGCTGGTATCAGCAGAAGCCAGGCCAGTCCCC TGTGCTGGTCATCTATCAAGATCGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAACACTGTGGTA TTCGGCGGAGGGACCAAGCTGACCGTCCTA<br><br>SEQ ID NO: 25143 | GAGGTGCAGCTGGTGGAGTCTGGGGGAGGCCTG GTCAAGCCTGGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGCGTCTGGGGAAC TACTGGGGCCAGGGAACCCTGGTCACCGTCTCCT CA<br><br>SEQ ID NO: 29149 |
| | | AA | SYELTQPPSVSVSPGQTVSITCSGDKLGDKYACW YQQKPGQSPVLVIYQDRKRPSGIPERFSGSNSGN TATLTISGTQAMDEADYYCQAWDNNTVVFGGG TKLTVL<br><br>SEQ ID NO: 25144 | EVQLVESGGGLVKPGGSLRLSCAASGFTFSSYSMN WVRQAPGKGLEWVSSISSSSSYIYYADSVKGRFTIS RDNAKNSLYLQMNSLRAEDTAVYYCARLGNYWG QGTLVTVSS<br><br>SEQ ID NO: 29150 |
| iPS:402229 | 21-225_16H9 | NA | GACATCCAGATGACCCAGTCTCCATCCTCCCT GTCTGCATCTGTAGGAGACAGAGTCACCATCA CTTGCCGGGCAAGTCAGGGCATTAGAAATTAT TTAGGCTGGTTTCAGCAGAAACCAGGGAAAGC CCCTAAGCGCCTGATCTATGGTGCATCCAGTT TGCAAAGTGGGGTCCCATCAAGGATCAGCGGC AGTGGATCTGGGACAGAATTCACTCTCACAAT CAGCAGCCTGCAGCCTGAAGATTTTGCAACTT ATTACTGTCTACAGTATCATAGTTATCTATTCA CTTTCGGCCCTGGGACCAAAGTGGATATCAAA<br><br>SEQ ID NO: 25145 | GAGGTGCAGCTGGTGGAGTCTGGGGGGAGGCCTG GTCAAGCCTGGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTCAACGGT ATGGACGTCTGGGGCCAAGGGACCACGGTCACC GTCTCCTCA<br><br>SEQ ID NO: 29151 |

EP 4 435 105 A2

FIGURE 50

| | | | DIQMTQSPSSLSASVGDRVTITCRASQGIRNYLG WFQQKPGKAPKRLIYGASSLQSGVPSRISGSGSG TEFTLTISSLQPEDFATYYCLQYHSYLFTFGPGTK VDIK | EVQLVESGGGLVKPGGSLRLSCAASGFTFSSYSMN WVRQAPGKGLEWVSSISGSSSYIYYADSVKGRFTIS RDNAKNSLYLQMNSLRAEDTAVYYCARVNGMDV WGQGTTVTVSS |
|---|---|---|---|---|
| | | AA | SEQ ID NO: 25146 | SEQ ID NO: 29152 |
| iPS:402231 | 21-225_6D9 | NA | TCCTATGAGCTGACTCAGCCACCCTCAGTGTC CGTGTCCCCAGGACAGACAGCCAGCATCACCT GCTCTGGAGATAAATTGGGGGAAAAATATGCT TGCTGGTATCAGCAGAAGCCAGGCCAGTCCCC TGTACTGGTCATCTATCAAGATAAGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTATTC GGCGGAGGGACCAAACTGACCGTCCTA | GAGGTGCAGCTGGTGGAGTCTGGGGGAGGCTTG GTAAAGCCTGGGGGGTCCCTTAGACTCTCCTGTG CAGCCTCTGGATTCACTTTCAGTAACGCCTGGAT GAACTGGGTCCGCCTGGCTCCAGGGAAGGGGCT GGAGTGGGTTGGCCGTATTAAAAAGCAAAACTGA TGGTGGGACAACAGACTACGCTGCACCCGTGAA AGGCAGATTCACCATCTCAAGAGATGATTCAGA AAACACGTTGTATCTGCAAATGAACAGCCTGAA AACCGAGGACACAGCCGTGTATTACTGTTCCACA GATACGGGTCCTATAGCAGCTCGTCTCGCTTACT ACTACTACTACGCTATGGACGTCTGGGGCCAAGG GACCACGGTCACCGTCTCCTCA |
| | | | SEQ ID NO: 25147 | SEQ ID NO: 29153 |
| | | AA | SYELTQPPSVSVSPGQTASITCSGDKLGEKYACW YQQKPGQSPVLVIYQDKKRPSGIPERFSGSNSGN TATLTISGTQAMDEADYYCQAWDSSTVFGGGT KLTVL | EVQLVESGGGLVKPGGSLRLSCAASGFTFSNAWM NWVRLAPGKGLEWVGRIKSKTDGGTTDYAAPVKG RFTISRDDSENTLYLQMNSLKTEDTAVYYCSTDTGP IAARLAYYYYYAMDVWGQGTTVTVSS |
| | | | SEQ ID NO: 25148 | SEQ ID NO: 29154 |

1743

FIGURE 50

| iPS:402233 | 21-225_16D10 | NA | GACATCCAGATGACCCAGTCTCCATCCTCACT GTCTGCATCTGTAGGAGACAGAGTCACCATCA CTTGTCGGGCGAGTCAGGACATAAGTAATTAT TTAGCCTGGTTTCAGCAGAAACCAGGGAAAGC CCCTAAGTCCCTGATCTATGCTACACCCAGTTT GCAGAGTGGGGTCCCATCAAAGTTCAGCGGCA GTGGATCTGGGACAGAGTTCACTCTCACCATC AGCAGCCTGCAGCCTGAAGATTTTGCAACTTA TTACTGCCAACAGTATAATAGTTACCCGCTCA CTTTCGGCGGAGGGACCAAGGTGGAGATCAA A | GAGGTGCAACTGGTGGAGTCTGGGGGAGGCCTG GTCAAGCCTGGGGGGTCCCTGAGACTCTCCTGTG CAGCCTCTGGATTCACCTTCAGTACCTATAACTT GAACTGGGTCCGCCAGGCTCCAGGGAAGGGGCT GGAGTGGGTCTCATCCATTAGTGGTGGTGCCGGT CACATATATTACTCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGGACTAATGGG TTTGACTTCTGGGGCCAGGGAACCCTGGTCACCG TCTCCTCA |
| | | | SEQ ID NO: 25149 | SEQ ID NO: 29155 |
| | | AA | DIQMTQSPSSLSASVGDRVTITCRASQDISNYLA WFQQKPGKAPKSLIYATPSLQSGVPSKFSGSGSG TEFTLTISSLQPEDFATYYCQQYNSYPLTFGGGT KVEIK | EVQLVESGGGLVKPGGSLRLSCAASGFTFSTYNLN WVRQAPGKGLEWVSSISGGAGHIYYSDSVKGRFTI SRDNAKNSLYLQMNSLRAEDTAVYYCARTNGFDF WGQGTLVTVSS |
| | | | SEQ ID NO: 25150 | SEQ ID NO: 29156 |
| iPS:402235 | 21-225_20F10 | NA | GACATCCAGTTGACCCAGTCTCCATCCTCACT GTCTGCATCTGTCGGAGACAGAGTCACCATCA CTTGTCGGGCGAGTCAGGGCATTAACAATTAT TTAGCCTGGTTTCAGCAGAAACCAGGGAAAGC CCCTAAGTCCCTGATTTATGCTGCATCCAGTTT GCAAAGTGGGGTCCCATCAAGGTTCAGCGGCA GTGGATTTGGGACAGATTTCACTCTCACCATC AGCAGCCTGCAGCCTGAAGATTTTGCAACTTT TTACTGCCAGCAATATAATAGTTACCCATTCA CTTTTGGCCCTGGGACCAAAGTGGATAACAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTACTAGTACTTTC ATATACTACGCAGATTCAGTGAAGGGCCGATTCA CCATCTCAAGAGACAACGCCAAGAACTCACTGT ATCTGCAAATGAACAGCCTGAGAGCCGAGGACA CGGCTGTGTATTACTGTGCGAGAAAGGCTGGGCT TGATATCTGGGGCCAAGGGACAATGGTCACCGTC TCTTCA |
| | | | SEQ ID NO: 25151 | SEQ ID NO: 29157 |

FIGURE 50

| | | | | |
|---|---|---|---|---|
| | | AA | DIQLTQSPSSLSASVGDRVTITCRASQGINNYLA WFQQKPGKAPKSLIYAASSLQSGVPSRFSGSGFG TDFTLTISSLQPEDFATFYCQQYNSYPFTFGPGTK VDNK | EVQLVESGGGLVKPGGSLRLSCAASGFTFSSYSMN WVRQAPGKGLEWVSSISTSTFIYYADSVKGRFTISR DNAKNSLYLQMNSLRAEDTAVYYCARKAGLDIWG QGTMVTVSS |
| | | | SEQ ID NO: 25152 | SEQ ID NO: 29158 |
| IPS:402237 | 21-225_23D11 | NA | GACATCCAGATGACCCAGTCTCCATCCTCACT GTCTGCATCTGTAGGGGACAGAGTCACCATCA CTTGTCGGGCGAGTCAGGGCATTGCCAATTAT TTAGCCTGGTTTCAGCAGAGACCAGGGAAAGC CCCTAAGTCCCTGATCTCTGCTGCATCCAGTTT GCAAAGTGGGGTCCCATCAAAGTTCAGCGGCA GTGGATCTGGGACAGAGTTCACTCTCACCATC AGCAGCCTGCAGCCTGAAGATTTTGCAACTTA TTACTGCCAACAGTATCATAGTTACCCGCTCA CTTTCGGCGGAGGGTCCAAGGTGGAGATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAACAT AAACTGGGTCCGCCAGGCTCCAGGGAAGGGGCT GGAGTGGGTCTCATCCATTAGTGGTAATAGTGGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGCGCGAACTAACCTC TTTGACTACTGGGGCCAGGGAACCCTGGTCACCG TCTCCTCA |
| | | | SEQ ID NO: 25153 | SEQ ID NO: 29159 |
| | | AA | DIQMTQSPSSLSASVGDRVTITCRASQGIANYLA WFQQRPGKAPKSLISAASSLQSGVPSKFSGSGSG TEFTLTISSLQPEDFATYYCQQYHSYPLTFGGGS KVEIK | EVQLVESGGGLVKPGGSLRLSCAASGFTFSSYNIN WVRQAPGKGLEWVSSISGNSGYIYYADSVKGRFTI SRDNAKNSLYLQMNSLRAEDTAVYYCARTNLFDY WGQGTLVTVSS |
| | | | SEQ ID NO: 25154 | SEQ ID NO: 29160 |

FIGURE 50

| iPS:403868 | 21-225_19D11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTACATCCAGT TTGCAAAGTGGGGTCCCATCAAGGTTCAGCGG CAGTGGATCTGGGACAGAATTCACTCTCACAA TCAGCAGCCTGCAGCCTGAGGATTTTGCAACT TATTACTGTCTACAGTATTATAGTTACCCGCTC ACTTTCGGCGGAGGGACCGAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA TTACTGGGGCTGGATCCGCCAGCCCCCCGGGAAG GGGCTGGACTGGATTGGGAGTATCTATTATAGTG GGAGCGCCAACTACAACCCGTCCCTCAAGAGTC GAGTCACCATATCCGTAGACACGTCCAAGAACC AGTTCTCCCTGAAACTGAGTTCCGTGACCGCCGC AGACGCGGCTGTGTATTACTGTGCGAGACTGGAC AGGGGCTGGTCCTTTGACTACTGGGGCCAGGGA ACCCTGGTCACCGTCTCCTCA |
| | | | SEQ ID NO: 25155 | SEQ ID NO: 29161 |
| | | AA | DIQMTQSPSSLSASVGDRVTITCRASQGIRNDLG WYQQKPGKAPKRLIYATSSLQSGVPSRFSGSGSG TEFTLTISSLQPEDFATYYCLQYYSYPLTFGGGTE VEIK | QLQLQESGPGLVKPSETLSLTCTVSGGSISRSSYYW GWIRQPPGKGLDWIGSIYYSGSANYNPSLKSRVTIS VDTSKNQFSLKLSSVTAADAAVYYCARLDRGWSF DYWGQGTLVTVSS |
| | | | SEQ ID NO: 25156 | SEQ ID NO: 29162 |
| iPS:403870 | 21-225_23G4 | NA | GACATCCAGATGACCCAGTCTCCATCCTCCCT GTCTGCATCTGTGGGAGACAGAGTCACCATCA CTTGCCGGGCAAGTCAGAACATTTACAGCTAT TTAAATTGGTATCAGCAGAAACCTGGGAAAGC CCCTAAGCTCCTGATCTATGCTGCATCCAGTTT GCAAAGTGGGGTTCCATCAAGGTTCAGTGGCA GTGGATCTGGGACAGAATTCACTCTCACCATC AGCATTCTGCAACCTGAAGATTTTGCAACTTA CTACTGTCAACAGAGTTACAATACCCCTCCGG AGTGCAATTTTGGCCAGGGGACCAAGCTGGAG ATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGGCGTGGTGGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAAAGGGGGA TAGTGGGAGCTACTGAGGCTTTTGATATCTGGGG CCAAGGGACAATGGTCACCGTCTCTTCA |
| | | | SEQ ID NO: 25157 | SEQ ID NO: 29163 |

FIGURE 50

| | | | | |
|---|---|---|---|---|
| | | AA | DIQMTQSPSSLSASVGDRVTITCRASQNIYSYLN WYQQKPGKAPKLLIYAASSLQSGVPSRFSGSGS GTEFTLTISILQPEDFATYYCQQSYNTPPECNFGQ GTKLEIK | EVQLLESGGGLVQPGGSLRLSCAASGFTFSSYAMS WVRQAPGKGLEWVSVISGRGGSTYYADSVKGRFTI SRDNSKNTLYLQMNSLRAEDTAVYYCAKRGIVGA TEAFDIWGQGTMVTVSS |
| | | | SEQ ID NO: 25158 | SEQ ID NO: 29164 |
| iPS:403872 | 21-225_8F11 | NA | GACATCCAGATGACCCAGTCTCCATCCTCCCT GTCTGCATCTGTAGGAGACAGAGTCACCATCA CTTGCCGGGCAAGTCAGGGCATTAGGAGTGAT TTAGGCTGGTTTCAGCAGAAACCAGGGAAAGC CCCTAAGCGCCTGATCTTTGATGCATCCAGTG TGCAAAGTGGGGTCCCATCAAGGTTCAGCGGC AGTGGATCTGGGACAGAATTCACTCTCACAAT CAGCAGCCTGCAGCCTGAAGATTTTGCAATTT ATTACTGTCTACAACATTATACTTACCCGCTCA CTTTCGGCGGAGGGACCAAGGTGGAGATCAA G | CAGCTGCAGCTGCAGGAGTCGGGCCCAGGACTG GTGCAGCCTTCGGAGACCCTGTCCCTCACCTGCA CTGTCTCTGGTGTCTCCATCAGTAGGACTAGTTA CTACTGGGGCTGGCTCCGCCAGCCCCCAGGGAA GGGGCTGGAGTGGATTGGCAATATTTATTATAGT GGGAGCGCCTACAACAACCCGTCCCTCAAGAGT CGAGTCACCATATCCGTTGACACGTCCAAGAACC AGTTCTCCCTGAAGCTGAGTTCTGTGACCGCCGC AGACACGGCTGTGTATTACTGTGGGAGACATGGA CAAGACTGGGGCCTTGACTACTGGGGCCAGGGA ACCCTGGTCACCGTCTCCTCA |
| | | | SEQ ID NO: 25159 | SEQ ID NO: 29165 |
| | | AA | DIQMTQSPSSLSASVGDRVTITCRASQGIRSDLG WFQQKPGKAPKRLIFDASSVQSGVPSRFSGSGSG TEFTLTISSLQPEDFAIYYCLQHYTYPLTFGGGTK VEIK | QLQLQESGPGLVQPSETLSLTCTVSGVSISRTSYYW GWLRQPPGKGLEWIGNIYYSGSAYNNPSLKSRVTIS VDTSKNQFSLKLSSVTAADTAVYYCGRHGQDWGL DYWGQGTLVTVSS |
| | | | SEQ ID NO: 25160 | SEQ ID NO: 29166 |

FIGURE 50

| iPS:404090 | 21-225_8D8 | NA | TCCTATGAGCTGACTCAGCCACCCTCAGTGTCCGTGTCCCCAGGACAGACAGCCAGCATCACCTGCTCTGGAGATAAATTGGGGGAGAAATATGCTTGCTGGTATCAGCAGAAGCCAGGCCAGTCCCCTGTGGTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATAGATGAGGCTGACTATTACTGTCAGGCGTGGGACAGCAGCACTGCGGTATTCGGCGGAGGGACCAAGTTGACCGTCCTA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGCGTCTGGGTAACTACTGGGGCCAGGGAACCCTGGTCACCGTCTCCTCA |
|---|---|---|---|---|
| | | | SEQ ID NO: 25161 | SEQ ID NO: 29167 |
| | | AA | SYELTQPPSVSVSPGQTASITCSGDKLGEKYACWYQQKPGQSPVVVIYQDRKRPSGIPERFSGSNSGNTATLTISGTQAIDEADYYCQAWDSSTAVFGGGTKLTVL | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARLGNYWGQGTLVTVSS |
| | | | SEQ ID NO: 25162 | SEQ ID NO: 29168 |
| iPS:412232 | 21-225_4A2 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAATACTCCAGTCACTTTCGGCCCTGGGACCAAAGTGGGGTATCAAA | CAGGTGCAGCTGGTGCAGTCTGGGACTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCTTGACCAGGAACACCTCCATAAGCACAGCCTACATGGAACTGAGCAGCCTGAGATCGAGGACACGGCCGTGTATTACTGTGCGAGTAGCAGTGGCTGGTACTACTTTGACTACTGGGGCCAGGGAACCCTGGTCACCGTCTCCTCA |
| | | | SEQ ID NO: 25163 | SEQ ID NO: 29169 |

FIGURE 50

| | | | | |
|---|---|---|---|---|
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYNTP VTFGPGTKVGIK | QVQLVQSGTEVKKPGASVKVSCKASGYTFTNYDIN WVRQATGQGLEWMGWMHPNSGNTGYAQKFQGR VTLTRNTSISTAYMELSSLRSEDTAVYYCASSSGWY YFDYWGQGTLVTVSS |
| | | | SEQ ID NO: 25164 | SEQ ID NO: 29170 |
| iPS:422894 | 21-225_4A2.001 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAATACTCCAGTCACTTTCGGCCCTGGG ACCAAAGTGGGGTATCAAA | CAGGTGCAGCTGGTGCAGTCTGGGACTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCTTGACCAGGGACACCTCCATAAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 25165 | SEQ ID NO: 29171 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYNTP VTFGPGTKVGIK | QVQLVQSGTEVKKPGASVKVSCKASGYTFTNYDIN WVRQATGQGLEWMGWMHPNSGNTGYAQKFQGR VTLTRDTSISTAYMELSSLRSEDTAVYYCASSSGWY YFDYWGQGTLVTVSS |
| | | | SEQ ID NO: 25166 | SEQ ID NO: 29172 |

FIGURE 50

| iPS:423018 | 21-225_31D12_LC2 | NA | TCCTATGAGCTGACTCAGCCACCCTCAGTGTC CGTGTCCCCAGGACAGACAGCCAGCATCACCT GCTCTGGAGATAAATTGGGGGGATAAATATGCT TACTGGTTTCAGCAGAAGCCAGGCCAGTCCCC TGTGATAGTCATCTATCAAGATAGGAAGCGGC CCTCAGGGATCCCTGAGCGATTCTCTGGCTCC AACTCTGGGAACACAGCCACTCTGACCATCAG CGGGACGCAGGCTATGGATGAGGCTGACTATT ACTGTCAGGCGTGGGACAACAGCACTGCGGTA TTCGGCGGAGGGACCAAGCTGACCGTCCTA | CAGGTGAAGCTGGTGCAGTCTGGGGCTGAGGTG AAGAAGCCTGGGGCCTCAGTGAAGGTCTCCTGC AAGGCTTCAGGATACACCTTCACCGGCTACTATA TGCACTGGGTGCGACAGGCCCCTGGACAAGGGC TTGAGTGGATGGGATGGATCAACCCTAACAGTGG TGGCACAAACTATGTACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGTGTAT TACTATGGTTCGGGGGAGTTATTATAACGAGTTTG ACTACTGGGGCCAGGGAACCCTGGTCACCGTCTC CTCA |
|---|---|---|---|---|
| | | | SEQ ID NO: 25167 | SEQ ID NO: 29173 |
| | | AA | SYELTQPPSVSVSPGQTASITCSGDKLGDKYAY WFQQKPGQSPVIVIYQDRKRPSGIPERFSGSNSG NTATLTISGTQAMDEADYYCQAWDNSTAVFGG GTKLTVL | QVKLVQSGAEVKKPGASVKVSCKASGYTFTGYYM HWVRQAPGQGLEWMGWINPNSGGTNYVQKFQGR VTMTRDTSISTAYMELSRLRSDDTAVYYCARVYYY GSGSYYNEFDYWGQGTLVTVSS |
| | | | SEQ ID NO: 25168 | SEQ ID NO: 29174 |
| iPS:423019 | 21-225_31D12_LC1 | NA | GATGTTGTGATGACTCAGTCTCCACTCTCCCTG CCCGTCACCCTTGGACAGCCGGCCTCCATCTC CTGCAGGTCTAGTCAAAGCCTCATATACAGTG ATGGAAACACCTTCTTGAATTGGTTTCAGCAG AGGCCAGGCCAATCTCCAAGGCGCCTAATTTA TAAGGTTTCTAATTGGGACTCTGGGGTCCCAG ACAGATTCAGCGGCAGTGGGTCAGGCACTGAT TTCACACTGAAAATCAGCAGGTTGGAGGCTGA GGATGTTGGGATTTATTACTGCATGCAAGGTA CACACTGGCCTCTCACCTTCGGCCAAGGGACA CGACTGGAGATTAAA | CAGGTGAAGCTGGTGCAGTCTGGGGCTGAGGTG AAGAAGCCTGGGGCCTCAGTGAAGGTCTCCTGC AAGGCTTCAGGATACACCTTCACCGGCTACTATA TGCACTGGGTGCGACAGGCCCCTGGACAAGGGC TTGAGTGGATGGGATGGATCAACCCTAACAGTGG TGGCACAAACTATGTACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGTGTAT TACTATGGTTCGGGGGAGTTATTATAACGAGTTTG ACTACTGGGGCCAGGGAACCCTGGTCACCGTCTC CTCA |
| | | | SEQ ID NO: 25169 | SEQ ID NO: 29175 |

FIGURE 50

| | | | | |
|---|---|---|---|---|
| | | AA | DVVMTQSPLSLPVTLGQPASISCRSSQSLIYSDGN TFLNWFQQRPGQSPRRLIYKVSNWDSGVPDRFS GSGSGTDFTLKISRLEAEDVGIYYCMQGTHWPL TFGQGTRLEIK | QVKLVQSGAEVKKPGASVKVSCKASGYTFTGYYM HWVRQAPGQGLEWMGWINPNSGGTNYVQKFQGR VTMTRDTSISTAYMELSRLRSDDTAVYYCARVYYY GSGSYYNEFDYWGQGTLVTVSS |
| | | | SEQ ID NO: 25170 | SEQ ID NO: 29176 |
| iPS:423314 | 21-225_12F11 | NA | GACATCGTGATGACCCAGTCTCCAGACTCCCT GGCTGTGTCTCTGGGCGAGAGGGCCACCATCA ATTGCAAGTCCAGCCAGAGTGTTTTACACAGC TCCAACAATTATAACTACTTAGCTTGGTACCA GCAGAAACCAGGACAGCCTCCTAACCTGCTCA TTTTCTGGGCATCTACCCGGGAATCCGGGGTC CCTGACCGATTCAGTGGCAGCGGGTTTGGGAC AGATTTCACTCTCAACATCAGCAGCCTGCAGG CTGAAGATGTGGCAGTTTATTACTGTCAGCAA TATTATGATACTCCATTCACTTTCGGCCCTGGG ACCAAAGTGGATATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC CTGAGTGGATGGGATGGATGCACCCTAACAGTG GTAACACAGGCTATGCAAAGAAGTTCCAGGGCA GAGTCACCATGACCAGGAACACCTCCATAAGCG CAGCCTATATGGTTCTGAGCAGCCTGAGATCTGA GGACACGGCCGTGTATTACTGTGCGCTTAGCAGT GGCTGGTACTACTTTGACTACTGGGGCCAGGGAA CCCTGGTCACCGTCTCCTCA |
| | | | SEQ ID NO: 25171 | SEQ ID NO: 29177 |
| | | AA | DIVMTQSPDSLAVSLGERATINCKSSQSVLHSSN NYNYLAWYQQKPGQPPNLLIFWASTRESGVPDR FSGSGFGTDFTLNISSLQAEDVAVYYCQQYYDTP FTFGPGTKVDIK | QVQLVQSGAEVKKPGASVKVSCKASGYTFTNYDIN WVRQATGQGPEWMGWMHPNSGNTGYAKKFQGR VTMTRNTSISAAYMVLSSLRSEDTAVYYCALSSGW YYFDYWGQGTLVTVSS |
| | | | SEQ ID NO: 25172 | SEQ ID NO: 29178 |

FIGURE 50

| iPS:424419 | 21-225_25A4.001 | NA | GACATCGTGATGACCCAGTTTCCAGACTCCCT GGCTGTGTCTCTGGGCGAGAGGGCCACCATCA AGTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGGAGG CTGAAGATGTGGCAGTTTATTACTGTCAGCAG TATTATAGTACTCCTCCGACGTTCGGCCAAGG GACCAAGGTGGAAATCAAA | CAGGTGCTCCTGGTGCAGTCTGGGGGCTGAGGTGA AGAGGCCTGGGGCCTCAGTGAAGGTCTCCTGCA AGGCTTCTGGATACACCTTCACCAATTATGATAT TAATTGGGTGCGACAGGCCACTGGACAAGGGCT TGAGTGGATGGGATGGATGTACCCTAACAGTGGT AACACAGGCTATGCACAGAAATTCCAGGGCAGA GTCACCATGACCAGGGACACCTCCATCAGCACA GCCTACATGGAGCTGAGCAGCCTGAGATCTGAG GACACGGCCGTCTATTACTGTGCGAGTAGCAGTG GCTGGTACTACTTTGACTACTGGGGCCAGGGAAC CCTGGTCACCGTGTCCTCA |
| | | | SEQ ID NO: 25173 | SEQ ID NO: 29179 |
| | | AA | DIVMTQFPDSLAVSLGERATIKCKSSQSVLYSSH NNNYLAWYQQKPGQPPKLLLYWASTRESGVPD RFSGSGSGTEFTLTISSLEAEDVAVYYCQQYYST PPTFGQGTKVEIK | QVLLVQSGAEVKRPGASVKVSCKASGYTFTNYDIN WVRQATGQGLEWMGWMYPNSGNTGYAQKFQGR VTMTRDTSISTAYMELSSLRSEDTAVYYCASSSGW YYFDYWGQGTLVTVSS |
| | | | SEQ ID NO: 25174 | SEQ ID NO: 29180 |
| iPS:424460 | 21-225_7E11.001 | NA | GACATCCAGATGACCCAGTCTCCATCCTCCCT GTCTGCATCTGTAGGAGACAGAGTCACCATCG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGATGGAAGT AATAAATACTATGCAGACTCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGCCAAGGGAC CACGGTCACCGTGTCCTCA |
| | | | SEQ ID NO: 25175 | SEQ ID NO: 29181 |

FIGURE 50

| | | | | |
|---|---|---|---|---|
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHDGSNKYYADSVKGRFT ISRDNSKNTLYLQMSSLRAEDTAVYYCARDLSMGG MDVWGQGTTVTVSS |
| | | | SEQ ID NO: 25176 | SEQ ID NO: 29182 |
| IPS:426108 | 21-225_10G6 | NA | GACATCCAGATGACCCAGTCTCCATCTTCCGT GTCTGCATCTGTGGGAGACAGAGTCACCATCA CTTGTCGGGCGAGTCAGGGTATTAGCAAATGG TTAGCCTGGTATCAGCAGAAACCAGGGAAAG CCCCTAAGATCCTGATATATGCTGCATATAGT TTACAAAGTGGGGTCCCAGCAAGGTTCAGCGG CAGTGGATCTGGGACAGATTTCACTCTCACCA TCAGGAGCCTGCAGCCTGAAGATTTTGCAACT TACTATTGTCAACAGGCTAACAGTTTCCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGCCTACCATA TGCACTGGGTGCGACAGGCCCCTGGACAAGGAC TTGAGTGGATGGGATGGATCAACCCTAACAATAA TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTTTATTACTGTGGGAGAGATGTT ACCAGCTCGTTTGACTATTGGGGGCCAGGGAACCC TGGTCACCGTCTCCTCA |
| | | | SEQ ID NO: 25177 | SEQ ID NO: 29183 |
| | | AA | DIQMTQSPSSVSASVGDRVTITCRASQGISKWLA WYQQKPGKAPKILIYAAYSLQSGVPARFSGSGS GTDFTLTIRSLQPEDFATYYCQQANSFPFTFGPGT KVDIK | QVQLVQSGAEVKKPGASVKVSCKASGYTFTAYHM HWVRQAPGQGLEWMGWINPNNNGTNYAQKFQGR VTMTRDTSISTAYMELSRLRSDDTAVYYCGRDVTS SFDYWGQGTLVTVSS |
| | | | SEQ ID NO: 25178 | SEQ ID NO: 29184 |

FIGURE 50

| | | | | |
|---|---|---|---|---|
| iPS:426110 | 21-225_12E9 | NA | GACATCCAGATGACCCAGTCTCCATCTTCCGT GTCTGCATCTGTAAGAGACAGAGTCACCATCA CTTGTCGGGCGAGTCAGGGTATTAGCAGCTGG TTAGCCTGGTATCAGCAGAAACCAGGGGAAG CCCCTAAGCTCCTGATCTATGCTGCATCCAGG TTGCAAAGTGGGGTCCCATCAAGGTTCAGCGG CAGTGGATCTGGGACAGATTTCACTCTCACCA TCAGCAGCCTGCAGCCTGAAGATTTTGCAACT TACTATTGTCAACAGGCTAACAGTTTCCCATTC ACTTTCGGCCCTGGGACCAAAGTGGATATCAA A | CAGGTGCAGCTGGTACAGTCTGGGGCTGAGGTG AAGAAGCCTGGGGCCTCAGTGAAGGTCTCCTGC AAGGCTTCTGGATACACCTTCACCGACTACTATT TGCACTGGGTGCGACAGGCCCCTGGACAAGGTCT TGAGTGGATGGGATGGGTCCACCCTAACAGTGGT GGCACAAACTTTGCACAGAAGTTTCAGGACAGG GTCACCATGACCAGGGACACGTCCATCAGCACA GCCTACATGGAGCTGAGCAGCCTGAGATCTGAC GACACGGCCATATATTCCTGTGCGCGAGATGGTA CCAGCTCGTTTGACTACTGGGGCCAGGGAACCCT GGTCACCGTCTCCTCA |
| | | | SEQ ID NO: 25179 | SEQ ID NO: 29185 |
| | | AA | DIQMTQSPSSVSASVRDRVTITCRASQGISSWLA WYQQKPGEAPKLLIYAASRLQSGVPSRFSGSGS GTDFTLTISSLQPEDFATYYCQQANSFPFTFGPGT KVDIK | QVQLVQSGAEVKKPGASVKVSCKASGYTFTDYYL HWVRQAPGQGLEWMGWVHPNSGGTNFAQKFQD RVTMTRDTSISTAYMELSSLRSDDTAIYSCARDGTS SFDYWGQGTLVTVSS |
| | | | SEQ ID NO: 25180 | SEQ ID NO: 29186 |
| iPS:426112 | 21-225_12F12 | NA | GACATCGTGATGACCCAGTCTCCAGACTCCCT GGCTGTGTCTCTGGGCGAGAGGGCCACCATCA ACTGCAAGTCCAGCCAGACTGTTTTATTCAGC TCCAACAATAACCACTACTTAGCATGGTACCA GCAGAAACCAGGACAACCTCCTAACCTCCTCA TTTACTGGGCATCTACCCGGGCATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTAGTCCGTGGACGTTCGGCCAAGG GACCAAGGTGGAAATCAAA | CAGGTGCAGCTGCTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGTACCCTAACAGTGG TAACACGGGCTATGCACAGAAGTTCCAGGGCAG AGTCACCATGACCATGAACACCTCCATAAGCACA GCCTACATGGAGTTGAGCAGCCTGAGATCTGAG GACACGGCCGTGTATTACTGTGCGATGAGCAGTG GCTGGTACTACTTTGACTTCTGGGGCCAGGGAAC CCTGGTCACCGTCTCCTCA |
| | | | SEQ ID NO: 25181 | SEQ ID NO: 29187 |

FIGURE 50

| | | | | |
|---|---|---|---|---|
| | | AA | DIVMTQSPDSLAVSLGERATINCKSSQTVLFSSN NNHYLAWYQQKPGQPPNLLIYWASTRASGVPD RFSGSGSGTDFTLTISSLQAEDVAVYYCQQYYSS PWTFGQGTKVEIK | QVQLLQSGAEVKKPGASVKVSCKASGYTFTNYDIN WVRQATGQGLEWMGWMYPNSGNTGYAQKFQGR VTMTMNTSISTAYMELSSLRSEDTAVYYCAMSSG WYYFDFWGQGTLVTVSS |
| | | | SEQ ID NO: 25182 | SEQ ID NO: 29188 |
| iPS:426114 | 21-225_28H2 | NA | GACATCCAGATGACCCAGTCTCCTTCCTCCCT GTCTGCATCTATAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATTTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAACATTATAGTTACCCTCGCA GTTTTGGCCAGGGGACCAAGCTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATATGGTATGATGGAAGT AATAAGTACTATGCAGACTCCGTGAAGGGCCGA TTCACCATCTCCAGAGACAATTCCAAGAACACGC TGTATCTACAAATGAACAGCCTGAGAGCCGTGG ACACGGCTGTGTATTACTGTGCGAGAGAGGAGTA TAGCAGTGGCTGGTACGACTACGGTATGGACGTC TGGGGCCAAGGGACCACGGTCACCGTCTCCTCA |
| | | | SEQ ID NO: 25183 | SEQ ID NO: 29189 |
| | | AA | DIQMTQSPSSLSASIGDRVTITCRASQGIRNDLG WYQQKPGKAPKRLIYAASSLQSGVPSRFSGSGS GTEFTLTISSLQPEDFATYYCLQHYSYPRSFGQG TKLEIK | QVQLVESGGGVVQPGRSLRLSCAASGFTFSNYVM HWVRQAPGKGLEWVAVIWYDGSNKYYADSVKGR FTISRDNSKNTLYLQMNSLRAVDTAVYYCAREEYS SGWYDYGMDVWGQGTTVTVSS |
| | | | SEQ ID NO: 25184 | SEQ ID NO: 29190 |

FIGURE 50

| iPS:426116 | 21-225_29E2 | NA | GACATCCAGATGACCCAGTCTCCATCCTCCCT GTCTGCATCGTAGGAGACAGAGTCACCATCA CTTGCCGGGCAAGTCAGGCCATTAGAAATGAT TTAGGCTGGTATCAGCAGAGACCAGGGAAAG CCCCTAAGCGCCTGATCTATGCTGCATCCAGT TTGCAAAGTGGGGTCCCATCAAGGTTCAGCGG CAGTGGATCTGGGACAGAATTTATTCTCACAA TCAGCAGCCTGCAGCCTGAAGATTTTGCAACT TATTACTGTTTACAGCATTATAATTACCCTCGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTGTGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA ATCACCATCTCCAGAGACAATTCCAAGAACACGC TGTATCTGCAAATGAACAGCCTGAGAGCCGAGG ACACGGCTGTGTATTACTGTGCGAGAGAGGAGTA TAGCAGTGGCTGGTACGACTACGGTATGGACGTC TGGGGCCAAGGGACCACGGTCACCGTCTCCTCA |
| | | | SEQ ID NO: 25185 | SEQ ID NO: 29191 |
| | | AA | DIQMTQSPSSLSASVGDRVTITCRASQAIRNDLG WYQQRPGKAPKRLIYAASSLQSGVPSRFSGSGS GTEFILTISSLQPEDFATYYCLQHYNYPRSFGQGT KLEIK | QVQLVESGGGVVQPGRSLRLSCAASGFTFSNCVMH WVRQAPGKGLEWVAVIWYDGSNKYYADSVKGRI TISRDNSKNTLYLQMNSLRAEDTAVYYCAREEYSS GWYDYGMDVWGQGTTVTVSS |
| | | | SEQ ID NO: 25186 | SEQ ID NO: 29192 |
| iPS:426118 | 21-225_7A10 | NA | GACATCCAGATGACCCAGTCTCCATCCTCCCT GTCTGCATCGTAGGAGACAGAGTCACCATCA CTTGCCGGGCAAGTCAGAACATTTACAGCTAT TTAAATTGGTATCAGCAGAAACCAGGGAGAG CCCCTAAACTCGTGATCTATTCTACATCCAGTT TGCAAAGTGGGGTCCCATCAAGGTTCAGTGGC AGTGGATCTGGGACAGATTTCAGTCTCACCAT CAGCAATCTGCAACCTGAAGATTTTTCAACTT ACTACTGTCAACAGAGTTACAGTCCCCCTCTC ACTTTCGGCGGAGGGACCAAGGTGGAGATCA GA | CAGGTGCAGCTGGTGGAGTCTGGGGGAGGCGTG GTCCAGCCTGGGAGGTCCCTGAGACTCTCCTGTG CAGCGTCTGGATTCAACTTCAGTAGCTATGGCAT GCACTGGGTCCGCCAGGCTCCAGGCAAGGGGCT GGAGTGGATGGCAGTTATATGGTATGATGGAAGT AATAAATACTATGCAGACTCCGTGAAGGGCCGA TTCACCATCTCCAGAGACAATTCCAAGAACACGC TGTATCTGCAAATGCACAGCCTGAGAGCCGAGG ACACGGCTGTGTATTACTGTGCGAGAGATGAGAG GCTGGGGATTTTTGACTACTGGGGCCAGGGAACC CTGGTCACCGTCTCCTCA |
| | | | SEQ ID NO: 25187 | SEQ ID NO: 29193 |

FIGURE 50

| | | | | |
|---|---|---|---|---|
| | | AA | DIQMTQSPSSLSASVGDRVTITCRASQNIYSYLN WYQQKPGRAPKLVIYSTSSLQSGVPSRFSGSGSG TDFSLTISNLQPEDFSTYYCQQSYSPPLTFGGGTK VEIR | QVQLVESGGGVVQPGRSLRLSCAASGFNFSSYGMH WVRQAPGKGLEWMAVIWYDGSNKYYADSVKGRF TISRDNSKNTLYLQMHSLRAEDTAVYYCARDERLG IFDYWGQGTLVTVSS |
| | | | SEQ ID NO: 25188 | SEQ ID NO: 29194 |
| iPS:426124 | 21-225_32D6 | NA | GACATCCAGATGACCCAGTCTCCATCCTCCCT GTCTACATCTGTAGGAGACAGAGTCACCATCA CTTGCCGGGCAAGTCAGAACATTATCAGCTAT TTAAATTGGTATCAGCAGAAACCAGGGAAAG CCCCTAAGCTCCTGATGTATGTTGCATCCCGTT TGCAAAGTGGGGTCCCATCAAGGTTCAGTGGC AGTGGATCTGGGACAGATTTCACTCTCACCAT CAGCAGTCTGCAAGCTGAAGATTTTGCAACTT ACTACTGTCAACAGAGTTACAGTACCCCGTAC ACTTTCGGCGGAGGGACCAAGGTGGCGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGACT GGAGTGGGTGACAGTTATATGGCATGATGGAAG TAATGCATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAAAATAG CAGCTCGTACTACTTTGACTACTGGGGCCAGGGA ACCCTGGTCACCGTCTCCTCA |
| | | | SEQ ID NO: 25189 | SEQ ID NO: 29195 |
| | | AA | DIQMTQSPSSLSTSVGDRVTITCRASQNIISYLNW YQQKPGKAPKLLMYVASRLQSGVPSRFSGSGSG TDFTLTISSLQAEDFATYYCQQSYSTPYTFGGGT KVAIK | QVQLVESGGGVVQPGRSLRLSCAASGFTFSSYGMH WVRQAPGKGLEWVTVIWHDGSNAYYADSVKGRF TISRDNSKNTLYLQMNSLRAEDTAVYYCARENSSS YYFDYWGQGTLVTVSS |
| | | | SEQ ID NO: 25190 | SEQ ID NO: 29196 |

FIGURE 50

| iPS:426126 | 21-225_6G6 | NA | GACATCGTGATGACCCAGTCTCCAGACTCCCT GGCTGTGTCTCTGGGCGAGAGGGCCACCATCA ATTGCAAGTCCAGCCAGAGTGTTTTACACAAC TCCAACAATTATAACTATTTAGCTTGGTACCA GCAGAAACCAGGACAGCCTCCTAACCTGCTCA TTTTCTGGGCATCTACCCGGGAATCCGGGGTC CCTGACCGATTCAGTGGCAGCGGGTTTGGGAC AGATTTCACTCTCAACATCAGCAGCCTGCAGG CTGAAGATGTGGCAGTTTATTACTGTCAGCAA TATTATGATACTCCATTCACTTTCGGCCATGGG ACCAAAGTGGATATCAAA | CAGGTGCAGCTGGTGCAGTCTGGGGCTGAGGTG AGGAAGCCTGGGGCCTCAGTGAAGGTCTCCTGC AAGGCTTCTGGATACACCTTCACCAATTATGATA TCAACTGGGTGCGACAGGCCACTGGACAAGGGC CTGAGTGGATGGGATGGATGCACCCTAACAGTG GTAACACAGGCTATGCAAAGAAGTTCCAGGGCA GAGTCACCATGACCAGGAACACCTCCATAAGCG CAGCCTATATGGTTCTGAGCAGCCTGAGATCTGA GGACACGGCCGTGTATTACTGTGCGCTTAGCAGT GGCTGGTACTACTTTGACTACTGGGGCCAGGGAA CCCTGGTCACCGTCTCCTCA |
| | | | SEQ ID NO: 25191 | SEQ ID NO: 29197 |
| | | AA | DIVMTQSPDSLAVSLGERATINCKSSQSVLHNSN NYNYLAWYQQKPGQPPNLLIFWASTRESGVPDR FSGSGFGTDFTLNISSLQAEDVAVYYCQQYYDTP FTFGHGTKVDIK | QVQLVQSGAEVRKPGASVKVSCKASGYTFTNYDIN WVRQATGQGPEWMGWMHPNSGNTGYAKKFQGR VTMTRNTSISAAYMVLSSLRSEDTAVYYCALSSGW YYFDYWGQGTLVTVSS |
| | | | SEQ ID NO: 25192 | SEQ ID NO: 29198 |
| iPS:433895 | 21-225_43E1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGAAGAAACCAGGGAAAG CCCCTAAGCGCCTGATCAATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCCGCCATTAGTGGTAATAGTACT TACATATACTACGCAGACTCGTTGAAGGGCCGAT TCACCATCTCCAGAGACAACGCCAAGAACTCACT GTTTCTGCAACTGAACAGCCTGAGAGCCGAGGA CACGGCTGTTTATTACTGTGCGAGAGATCGGGGC AGTGAATGGGGCCAGGGAACCCTGGTCACCGTC TCCTCA |
| | | | SEQ ID NO: 25193 | SEQ ID NO: 29199 |

FIGURE 50

| | | | | |
|---|---|---|---|---|
| | | AA | DIQMTQSPSSLSASVGDRVTITCRASQGIRNDLG WYQKKPGKAPKRLINAASSLQSGVPSRFSGSGS GTEFTLTISSLQPEDFATYYCLQHNSYPFTFGPGT KVDIK | EVQLVESGGGLVKPGGSLRLSCAASGFTFSSYSMN WVRQAPGKGLEWVSAISGNSTYIYYADSLKGRFTI SRDNAKNSLFLQLNSLRAEDTAVYYCARDRGSEW GQGTLVTVSS |
| | | | SEQ ID NO: 25194 | SEQ ID NO: 29200 |
| IPS:433897 | 21-225_43C2 | NA | GACATCCAGATGACCCAGTCTCCATCTTCCGT GTCTGCATCTGTAGGAGACAGAGTCACCATCA CTTGTCGGGCGAGTCAGGGTATTAGCGACTGG TTAGCCTGGTATCAGCAGAAACCAGGGAAAG CCCCTAGGCTCCTGATCTATGCTGCATCCAGTT TGCAAAGTGGGGTCCCATCAAGGTTCAGCGGC AGTGGATCTGGGACAGATTTCACTCTCACCAT CAGCAACCTGCAGCCTGAAGATTTTGCAACTT ACTATTGTCAACAGACTAACAGTTTCCCGTGG ACGTTCGGCCAAGGGACCAAGGTGGAAATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGTT AACACATTCGACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAGGAAAGGA GTGGGAGCTATTTTGACTACTGGGGCCAGGGAAC CCTGGTCACCGTCTCCTCA |
| | | | SEQ ID NO: 25195 | SEQ ID NO: 29201 |
| | | AA | DIQMTQSPSSVSASVGDRVTITCRASQGISDWLA WYQQKPGKAPRLLIYAASSLQSGVPSRFSGSGSG TDFTLTISNLQPEDFATYYCQQTNSFPWTFGQGT KVEIK | EVQLLESGGGLVQPGGSLRLSCAASGFTFSSYAMS WVRQAPGKGLEWVSAISGRGVNTFDADSVKGRFTI SRDNSKNTLYLQMNSLRAEDTAVYYCAKERSGSY FDYWGQGTLVTVSS |
| | | | SEQ ID NO: 25196 | SEQ ID NO: 29202 |

FIGURE 50

| iPS:433899 | 21-225_43C3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCTCTCACTTTCGGCGGAGGGACCAAGGTGGGGATCACA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATACACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTAGGATTTTCCAATGACTACTGGGGCCAGGGAACCCTGGTCACCGTCTCCTCA |
| | | | SEQ ID NO: 25197 | SEQ ID NO: 29203 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVGIT | QVQLVESGGGVVQPGRSLRLSCAASGFTFSSYGIHWVRQAPGKGLEWVAVIWYDENNKYYADSVKGRFTISRDNSKNTLYLQMNSLRAEDTAVYYCARELGFSNDYWGQGTLVTVSS |
| | | | SEQ ID NO: 25198 | SEQ ID NO: 29204 |
| iPS:433901 | 21-225_43A4 | NA | GACATCCAGATGACCCAGTCTCCATCCTCACTGTCTGCATCTGTAGGAGACAGAGTCACCATCACTTGTCGGGCGAGTCAGGGCATTAACAATTATTTAGCCTGGTTTCAGCAGAAACCAGGGAAAGCCCCTAAGTCCCTGATCAATGCTGCATCCAGTTTGCAAAGTGGGGTCCCATCAAGGTTCAGCGGCAGTGGATCTGGGACAGATTTCACTCTCGCCATCAGCAGCCTACAGCCTGAAGATTTTGCAACTTATTACTGCCAACAGTATTATAGTTACCCATTCACTTTCGGCCCTGGGACCAAAGTGGATATCAAA | GAGGTGCAGCTGGTGGAGTCTGGGGGAGGCCTGGTCAAGCCGGGGGGGTCCCTGAGACTCTCCTGTGCAGCCTCTGGATTCACCTTCAGTAGCTATACCATGAACTGGGTCCGCCAGGTTCCAGGGAAGGGGCTGGAGTGGGTCTCATCCATTAGTGGAAGTAGTACTTACATATACTACGCAGACTCAGTGAAGGGCCGATTCACCATCTCCAGAGACGACGCCCAGAACTCACTGTATCTGCAAATGAACAGCCTGAGAGGCGAGGACACGGCTGTGTATTACTGTGCGAGGGTGACCTCTTTTGACTACTGGGGCCAGGGAGCCCTGGTCACCGTCTCCTCA |
| | | | SEQ ID NO: 25199 | SEQ ID NO: 29205 |

FIGURE 50

| | | | | |
|---|---|---|---|---|
| | | AA | DIQMTQSPSSLSASVGDRVTITCRASQGINNYLA WFQQKPGKAPKSLINAASSLQSGVPSRFSGSGSG TDFTLAISSLQPEDFATYYCQQYYSYPFTFGPGT KVDIK | EVQLVESGGGLVKPGGSLRLSCAASGFTFSSYTMN WVRQVPGKGLEWVSSISGSSTYIYYADSVKGRFTIS RDDAQNSLYLQMNSLRGEDTAVYYCARVTSFDY WGQGALVTVSS |
| | | | SEQ ID NO: 25200 | SEQ ID NO: 29206 |
| IPS:433903 | 21-225_43H4 | NA | GACATCCAGATGACCCAGTCTCCATCTTCCGT GTCTGCATCTGTAGGAGACAGAGTCACCATCA CTTGTCGGGCGAGTCAGGGTATTATCAACTGG TTAGCCTGGTATCAACAGAAACCAGGGAAAG CCCCTAAGCTCCTGATCTATGCTGCATCCAGTT TGCAAAGTGGGGTCCCATCAAGGTTCAGCGGC AGTGGATCTGGGACAGATTTCACTCTCACCAT CAGCAACCTGCAGCCTGAAGATTTTGCAACTT ACTATTGTCAACAGACTAACAGTTTCCCGTGG ACGTTCGGCCAAGGGACCAAGGTGGAAATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTATT AACACATTCGACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAGGAAAGGA GTGGGAGCTATTTTGACTACTGGGGCCAGGGAAC CCTGGTCACCGTCTCCTCA |
| | | | SEQ ID NO: 25201 | SEQ ID NO: 29207 |
| | | AA | DIQMTQSPSSVSASVGDRVTITCRASQGIINWLA WYQQKPGKAPKLLIYAASSLQSGVPSRFSGSGS GTDFTLTISNLQPEDFATYYCQQTNSFPWTFGQG TKVEIK | EVQLLESGGGLVQPGGSLRLSCAASGFTFSSYAMS WVRQAPGKGLEWVSAISGRGINTFDADSVKGRFTI SRDNSKNTLYLQMNSLRAEDTAVYYCAKERSGSY FDYWGQGTLVTVSS |
| | | | SEQ ID NO: 25202 | SEQ ID NO: 29208 |

EP 4 435 105 A2

FIGURE 50

| iPS:433905 | 21-225_43E5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGGTGCATCCAAT TTGCAAAGTGGGGTCCCATCAAGGTTCAGCGG CAGTGGATCTGGGACAGAATTCACTCTCACAG TCAGCAGCCTGCAGCCTGAAGATTTTGCAACT TATTTCTGTCTACAGCATACTAGTTTCCCATTC ACTTTCGGCCCTGGGACCAAAGTGGATATCAA A | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTACAT GATCTGGATCCGCCAGGCTCCAGGGAAGGGGCT GGAGTGGGTTTCATACATTAGTAGTAGTGGTATT ACCAAATACTACGCAGACTCTATGAAGGGCCGA TTCACCATCTCCAGGGACAACGCCAAGAACTCAC TGTATCTGCAAATGAACAGCCTGAGAGCCGAGG ACACGGCCGTGTATTACTGTGCGGCCGATACAAT CTACTGGGGCCAGGGAACCCTGGTCACCGTCTCC TCA |
| | | | SEQ ID NO: 25203 | SEQ ID NO: 29209 |
| | | AA | DIQMTQSPSSLSASVGDRVTITCRASQGIRNDLG WYQQKPGKAPKRLIYGASNLQSGVPSRFSGSGS GTEFTLTVSSLQPEDFATYFCLQHTSFPFTFGPGT KVDIK | QVQLVESGGGLVKPGGSLRLSCAASGFTFSDYYMI WIRQAPGKGLEWVSYISSSGITKYYADSMKGRFTIS RDNAKNSLYLQMNSLRAEDTAVYYCAADTIYWGQ GTLVTVSS |
| | | | SEQ ID NO: 25204 | SEQ ID NO: 29210 |
| iPS:433909 | 21-225_43D8 | NA | GACATCGTGATGACCCAGTCTCCAGACTCCCT GGCTGTGTCTCTGGGCGAGAGGGCCACCATCA ACTGCAAGTCCAGCCAGAGTGTTTTAATGACC TCCAACGATAAGAACTACTTAACTTGGTACCA GCAGAGACCAGGACAGCCTCCTAAGCTGCTCA TTTACTGGGCTTCTACCCGGGAATCCGGGGTC CCTGACCGATTCAGTGGCGGCGGGTCTGGGAC AGATTTCACTCTCACCATCAGCGGCCTGCAGG CTGAAGATGTGGCAGTTTATTACTGTCAGCAA TATTATAGTACTCCTCCGACGTTCGGCCAAGG GACCAAGGTGGAAATCAAG | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGACATCTGA GGACACGGCCGTGTATTACTGTGCCCATAGCAGT GGCTGGACCCTCTTTGACTACTGGGGCCAGGGAA CCCTGGTCACCGTCTCCGCA |
| | | | SEQ ID NO: 25205 | SEQ ID NO: 29211 |

1762

FIGURE 50

| | | | | |
|---|---|---|---|---|
| | | AA | DIVMTQSPDSLAVSLGERATINCKSSQSVLMTSN DKNYLTWYQQRPGQPPKLLIYWASTRESGVPDR FSGGGSGTDFTLTISGLQAEDVAVYYCQQYYST PPTFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLTSEDTAVYYCAHSSGW TLFDYWGQGTLVTVSA |
| | | | SEQ ID NO: 25206 | SEQ ID NO: 29212 |
| IPS:433911 | 21-225_43E8 | NA | GACATCCAGATGAACCAGTCTCCATCTTCCGT GTCTGCATCTGTAGGAGACAGAGTCACCATCA CTTGTCGGGCGAGTCAGGGTATTAGCAACTGG TTAGCCTGGTATCAGCAGAAACCAGGGAGAG CCCCTAAGCTCCTGATCTATGCTGCATCCAGTT TGCAAAGTGGGGTCCCTTCAAGGTTCAGCGGC AGTGGATCTGGGACAGATTTCACTCTCACCAT CAGCAACCTGCAGCCTGAAGATTTTGCAACTT ACTATTGTCAACAGACTAACAGTTTCCCGTGG ACGTTCGGCCAAGGGACCAAGGTGGAAATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTATT AACACATTCGACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAGGAAAGGA GTGGGAGCTATTTTGACTACTGGGGCCAGGGAAC CCTGGTCACCGTCTCCTCA |
| | | | SEQ ID NO: 25207 | SEQ ID NO: 29213 |
| | | AA | DIQMNQSPSSVSASVGDRVTITCRASQGISNWLA WYQQKPGRAPKLLIYAASSLQSGVPSRFSGSGSG TDFTLTISNLQPEDFATYYCQQTNSFPWTFGQGT KVEIK | EVQLLESGGGLVQPGGSLRLSCAASGFTFSSYAMS WVRQAPGKGLEWVSAISGRGINTFDADSVKGRFTI SRDNSKNTLYLQMNSLRAEDTAVYYCAKERSGSY FDYWGQGTLVTVSS |
| | | | SEQ ID NO: 25208 | SEQ ID NO: 29214 |

FIGURE 50

| | | | | |
|---|---|---|---|---|
| iPS:433913 | 21-225_43H8 | NA | GACATCCAAATGACCCAGTCTCCATCCTCCCT GTCTGCATCTGTAGGAGACAGAGTCACCATCA CTTGCCGGGCAAGTCAGGGCATTAGAAATGAT TTAGGCTGGCATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCTCAA TCAGCAGCCTGCAGCCTGAAGATTTTGCAACT TATTACTGTCTACAGCATAATAGTTTCCCATTC ACTTTCGGCCCTGGGACCAAAGTGGATATCAA A | CAGGTGCAGCTGGTGGAGTCTGGGGGAGGCTTG GTCAAGCCTGGAGGGTCCCTGAGACTCTCCTGTG CAGCCTCTGGAATCACCTTCAGTGACTACTACAT GAACTGGATCCGCCAGGCTCCAGGGAAGGGGCT GGAGTGGGTTTCATACATTAGTAGTAGTGGTAGA ACCATATTCTACGCAGACTCTTTGAAGGGCCGAT TCACCATCTCCAGGGACAACGCCAAGAACTCACT GTATCTGCAAATGAACAGCCTGAGAGCCGAGGA CACGGCCGTGTATTACTGTGCGGCCGATACAATC TACTGGGGCCAGGGAACCCTGGTCACCGTCTCCT CA |
| | | | SEQ ID NO: 25209 | SEQ ID NO: 29215 |
| | | AA | DIQMTQSPSSLSASVGDRVTITCRASQGIRNDLG WHQQKPGKAPKRLIYAASSLQSGVPSRFSGSGS GTEFTLSISSLQPEDFATYYCLQHNSFPFTFGPGT KVDIK | QVQLVESGGGLVKPGGSLRLSCAASGITFSDYYMN WIRQAPGKGLEWVSYISSSGRTIFYADSLKGRFTISR DNAKNSLYLQMNSLRAEDTAVYYCAADTIYWGQ GTLVTVSS |
| | | | SEQ ID NO: 25210 | SEQ ID NO: 29216 |
| iPS:433915 | 21-225_43H9 | NA | GACATCCAGATGACCCAGTCTCCATCTTCCGT GTCTGCATCTGTAGGAGACAGAGTCACCATCA CTTGTCGGGCGAGTCAGGATATTAGCAGCTGG TTAGCCTGGTATCAGAAGAAACCAGGGAAAG CCCCTAAACTCCTGATCTATGATGCATCCAGTT TGCAAAGTGGGGTCCCATCAAGGTTCAGCGGC AGTGGATCTGGGACAGATTTCATTCTCACCAT CAGCAGCCTGCAGCCTGAAGATTTTGCAACTT ACTTTTGTCAACAGGCTAACAGTCTCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGTTGGGTCCGCCAGGCTCCAGGGATGGGGCT GGAGTGGGTCTCAGCTATTAGTGGTAGTGGTAGT AACACATTCTACGCAGACTCCGTGAAGGGCCGGT TCACCATCTCCAGAGACAATTCCAAGAACACGCT GTATCTGCACATGAACAGCCTGAGAGCCGAGGA CACTGCCGTATATTTCTGTGCGAAACGAACGCCC TCTGATGTTTTTGATATCTGGGGCCAAGGGACAA TGGTCACCGTCTCTTCA |
| | | | SEQ ID NO: 25211 | SEQ ID NO: 29217 |

FIGURE 50

| | | | | |
|---|---|---|---|---|
| | | AA | DIQMTQSPSSVSASVGDRVTITCRASQDISSWLA WYQKKPGKAPKLLIYDASSLQSGVPSRFSGSGS GTDFILTISSLQPEDFATYFCQQANSLPFTFGPGT KVDIK | EVQLLESGGGLVQPGGGSLRLSCAASGFTFSSYAMS WVRQAPGMGLEWVSAISGSGSNTFYADSVKGRFTI SRDNSKNTLYLHMNSLRAEDTAVYFCAKRTPSDVF DIWGQGTMVTVSS |
| | | | SEQ ID NO: 25212 | SEQ ID NO: 29218 |
| iPS:433917 | 21-225_43E11 | NA | GATATTGTGATGACCCAGACTCCACTCTCTCT GTCCGTCACCCCTGGACAGCCGGCCTCCATCT CCTGCAAGTCTAGTCAGAGCCTCCTGCACAGT GATGGAAGGACCTATTTGTATTGGTACCTTCA GAAGCCAGGCCAGCCTCCGCAGCTCCTGATCT ATGAAGTTTCCAACCGGTTCTCTGGAGTGCCA GATAGGTTCAGTGGCAGCGGGTCAGGGACAG ACTTCACACTGAAAATCAGCCGGGTGGAGGCT GAGGATGTTGGGGTTTATTACTGTATGCAAAG TATACAGCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCTCCTTCAGTGACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA CTCACCATCTCCAGAGACAATTCCAAGAACACGC TGTATCTGCAAATGAACAGCCTGAGAGCCGAGG ACACGGCTGTGTATTACTGTGCGAGGCGGTATGT CAGAAGCTGGGTGGGAGGTATGGACGTCTGGGG CCAAGGGACCACGGTCACCGTCTCCTCA |
| | | | SEQ ID NO: 25213 | SEQ ID NO: 29219 |
| | | AA | DIVMTQTPLSLSVTPGQPASISCKSSQSLLHSDGR TYLYWYLQKPGQPPQLLIYEVSNRFSGVPDRFSG SGSGTDFTLKISRVEAEDVGVYYCMQSIQLPWTF GQGTKVEIK | QVQLVESGGGVVQPGRSLRLSCAASGFSFSDYGMH WVRQAPGKGLEWVAVIWYDGSNKYYADSVKGRL TISRDNSKNTLYLQMNSLRAEDTAVYYCARRYVRS WVGGMDVWGQGTTVTVSS |
| | | | SEQ ID NO: 25214 | SEQ ID NO: 29220 |

FIGURE 50

| iPS:433919 | 21-225_44B3 | NA | GACATCCAGATGACCCAGTCTCCTTCCTCCCT GTCTGCATCTGTAGGAGACAGAGTCACCATCA CTTGCCGGGCAAGTCAGGGCATTAGAAATGAT TTAGGCTGGTATCAACAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACTGCATTATAATTACCCTCGG ACGTTCGGCCAAGGGACCAAAGTGGAAATCA AG | CAGGTGCAGCTGGTGGAGTCTGGGGGAGGCGTG GTCCAGCCTGGGAGGTCCCTGAGACTCTCCTGTA CAGCGTCTGGATTCACCTTCAGTGACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAGCAGTGGCTGGTACGACTACGGTATGGACGTC TGGGGCCAAGGGACCACGGTCACCGTCTCCTCA |
|  |  |  | SEQ ID NO: 25215 | SEQ ID NO: 29221 |
|  |  | AA | DIQMTQSPSSLSASVGDRVTITCRASQGIRNDLG WYQQKPGKAPKRLIYAASSLQSGVPSRFSGSGS GTEFTLTISSLQPEDFATYYCLLHYNYPRTFGQG TKVEIK | QVQLVESGGGVVQPGRSLRLSCTASGFTFSDYGMH WVRQAPGKGLEWVAVIWYDGSNKYYADSVKGRF TISRDNSKNTLYLQMNSLRAEDTAVYYCARERYSS GWYDYGMDVWGQGTTVTVSS |
|  |  |  | SEQ ID NO: 25216 | SEQ ID NO: 29222 |
| iPS:433921 | 21-225_44C3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CGGTGGATCTGGGACAGAATTCACTCTCACAA TCAGCAGCCTGCAGCCTGAAGATTTTGCGACT TATTACTGTCTACAGCATAGTAGTTACCCTCTC ACTTTCGGCGGAGGGACCAAGGTGGAGATCA AG | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTTTGAAGGAAGT AATAAATACTATGCAGATTCCGTGAAGGGCCGAT TCACCATCTCCAGAGACAATTCCAAGAACACGCT GTATCTGCAAATGAGCAGCCTGAGAGCCGAGGA CACGGCTGTGTATTACTGTGTGAGAGAACTAGGA TTTTCCACCGACTACTGGGGCCAGGGAACCCTGG TCACCGTCTCCTCA |
|  |  |  | SEQ ID NO: 25217 | SEQ ID NO: 29223 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGGGS GTEFTLTISSLQPEDFATYYCLQHSSYPLTFGGGT KVEIK | QVQLVESGGGVVQPGRSLRLSCAASGFTFSSYGMH WVRQAPGKGLEWVAVIWFEGSNKYYADSVKGRF TISRDNSKNTLYLQMSSLRAEDTAVYYCVRELGFS TDYWGQGTLVTVSS |
| | | | SEQ ID NO: 25218 | SEQ ID NO: 29224 |
| iPS:433923 | 21-225_44D3 | NA | GACATCCAGATGACCCAGTCTCCATCCTCCCT GTCTGCATCTGTGGGAGACAGAGTCACCTTCA CTTGCCGGGCAAGTCAGGGCATTAGAGATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTAGATCTGGGAGAGAATTCACTCTCACAA TCAGCAGCCTGCAACCTGAAGATTTTGCAACT TATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCAGTTATATGGTATGATGGAAGT AATAAATACTATGCTGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAAAGGTAT AGCAGTGGCTTGTACGACTACGGTATGGACGTCT GGGGCCAAGGGACCACGGTCACCGTCTCCTCA |
| | | | SEQ ID NO: 25219 | SEQ ID NO: 29225 |
| | | AA | DIQMTQSPSSLSASVGDRVTFTCRASQGIRDDLG WYQQKPGKAPKRLIYAASSLQSGVPSRFSGSRS GREFTLTISSLQPEDFATYYCLQHYSYPRTFGQG TKVEIK | QVQLVESGGGVVQPGRSLRLSCAASGFTFSSYVMH WVRQAPGKGLEWVAVIWYDGSNKYYADSVKGRF TISRDNSKNTLYLQMNSLRAEDTAVYYCARERYSS GLYDYGMDVWGQGTTVTVSS |
| | | | SEQ ID NO: 25220 | SEQ ID NO: 29226 |

FIGURE 50

| iPS:433925 | 21-225_44F3 | NA | GACATCCAGATGACCCAGTCTCCATCTTCCGT GTCTGCATCTGTAGGAGACAGAGTCACCATCA CTTGTCGGGCGAGTCAGGGTATTAGCGACTGG TTAGCCTGGTATCAGCAGAAACCAGGGAAAG CCCCTAAGCTCCTGATTTATGCTGCATCCAGTT TACAAAGTGGAGTCCCATCAAGGTTCAGCGGC AGTGGATTTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | GAGGTGCACCTGTTGGAGTCTGGGGGAGGCTTGG TACAGCCTGGGGGGGTCCCTGAGACTCTCCTGTGC AGCCTCTGGATTCACCTTTAGCAGCTATGCCATG AGCTGGGTCCGCCAGGCTCCAGGGAAGGGGCTG GAGTGGGTCTCAATTCTCAGTGGTGGTGGTAAGA CCACATACTACGCAGACTCCGTGAAGGGCCGGTT CACCATCTCCAGAGACAATTCCAAGAACACGCTG TTTCTGCAAATGAACAGCCTGAGAGCCGAGGAC ACGGCCGTATATTACTGTGCGAAACGAACGCCCT CTGATGCTTTTGATATCTGGGGCCAAGGGACAAT GGTCACCGTCTCTTCA |
| | | | SEQ ID NO: 25221 | SEQ ID NO: 29227 |
| | | AA | DIQMTQSPSSVSASVGDRVTITCRASQGISDWLA WYQQKPGKAPKLLIYAASSLQSGVPSRFSGSGF GTDFTLTISSLQPEDFATYYCQQANSFPFTFGPGT KVDIK | EVHLLESGGGLVQPGGSLRLSCAASGFTFSSYAMS WVRQAPGKGLEWVSILSGGGKTTYYADSVKGRFTI SRDNSKNTLFLQMNSLRAEDTAVYYCAKRTPSDAF DIWGQGTMVTVSS |
| | | | SEQ ID NO: 25222 | SEQ ID NO: 29228 |
| iPS:433929 | 21-225_44D5 | NA | GACATCCAGATGACCCAGTCTCCATCCTCCCT GTCTGCATCTGTAGGAGACAGAGTCACCATCA CTTGCCGGGCAAGTCAGGACATTAGAAAAGAT TTAGGCTGGTATCAGCAGAAACCAGGGAAAG CCCCTAAGCGCCTGATCTATGCTGCATCCACTT TGGAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATTATAGTTTCCCGTGGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGTCCCGTA TAGCAGCAGCTGGTACGACTACGGTATGGACGTC TGGGGCCAAGGGACCACGGTCACCGTCTCCTCA |
| | | | SEQ ID NO: 25223 | SEQ ID NO: 29229 |

FIGURE 50

| | | | | |
|---|---|---|---|---|
| | | AA | DIQMTQSPSSLSASVGDRVTITCRASQDIRKDLG WYQQKPGKAPKRLIYAASTLESGVPSRFSGSGS GTEFTLTISSLQPEDFATYYCLQHYSFPWTFGQG TKVEIK | QVQLVESGGGVVQPGRSLRLSCAASGFTFSSYVMH WVRQAPGKGLEWVAVIWYDGSNKYYADSVKGRF TISRDNSKNTLYLQMNSLRAEDTAVYYCARVPYSS SWYDYGMDVWGQGTTVTVSS |
| | | | SEQ ID NO: 25224 | SEQ ID NO: 29230 |
| IPS:433931 | 21-225_44F6 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGTGGAAGC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TTTATTACTGTCAGCAGTATGGTAGTTCACCGT GGACGTTCGGCCAAGGGACCAAGGTGGAAAT CAAA | CAGGTGCACCTGCAGGAGTCGGGCCCAGGACTG GTGAAGCCTTCGGAGACCCTGTCCCTCACCTGCA CTGTCTCTGGTGGCTCCATCAGTAGTTACTACTG GAGCTGGATCCGGCAGCCCCCAGGGAAGGGACT GGAGTGGATTGGGTATATCTATTACAGTGGAAAC ACCAACTACAACCCCTCCCTCAAGAGTCGAGTCA CCATATCAGTAGACACGTCCAAGAACCAGTTCTC CCTGAAGCTGAGCTCTGTGACCGCTGCGGACACG GCCGTGTATTACTGTGTGAGAGGGGTGGCTATAA AGAACTACTGGGGCCAGGGAATCCTGGTCACCG TCTCCTCA |
| | | | SEQ ID NO: 25225 | SEQ ID NO: 29231 |
| | | AA | EIVLTQSPGTLSLSPGERATLSCRASQSVSGSYLA WYQQKPGQAPRLLIYGASSRATGIPDRFSGSGSG TDFTLTISRLEPEDFAVYYCQQYGSSPWTFGQGT KVEIK | QVHLQESGPGLVKPSETLSLTCTVSGGSISSYYWSW IRQPPGKGLEWIGYIYYSGNTNYNPSLKSRVTISVD TSKNQFSLKLSSVTAADTAVYYCVRGVAIKNYWG QGILVTVSS |
| | | | SEQ ID NO: 25226 | SEQ ID NO: 29232 |

FIGURE 50

| iPS:433933 | 21-225_44C8 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAATTTGCAAAGTGGGGTCCCATCAAGGTTCAGCGGCAGTAGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAATAACTATGGCATGCACTGGGTCCGCCAGGCTCCAGGCAAGGGGCTGGAGTGGGTGGCAGTTATATGGTATGAAGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGTGAGAGAACTGGGGTTCCTCTCTGACTACTGGGGCCAGGGAACCCTGGTCACCGTCTCCTCA |
| | | | SEQ ID NO: 25227 | SEQ ID NO: 29233 |
| | | AA | DIQMTQSPSSLSASVGDRVTITCRASQGIRNDLGWYQQKPGKAPKRLIYAASNLQSGVPSRFSGSRSGTEFTLTISSLQPEDFATYYCLQHNSYPFTFGPGTKVDIK | QVQLVESGGGVVQPGRSLRLSCAASGFTFNNYGMHWVRQAPGKGLEWVAVIWYEGSNKYYADSVKGRFTISRDNSKNTLYLQMNSLRAEDTAVYYCVRELGFLSDYWGQGTLVTVSS |
| | | | SEQ ID NO: 25228 | SEQ ID NO: 29234 |
| iPS:433935 | 21-225_44F9 | NA | GACGTCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTCATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTCCACCATTATAATTACCCTCGGACGTTCGGCCAAGGGACCAAGGTGGAAATCACA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACCATATAGCAGCAGCTGGTACGACTACGGTATGGACGTCGGGGGCCAAGGGACCACGGTCACCGTCTCCTCA |
| | | | SEQ ID NO: 25229 | SEQ ID NO: 29235 |

FIGURE 50

| | | | | |
|---|---|---|---|---|
| | | AA | DVQMTQSPSSLSASVGDRVTITCRASQGIRNDLG WYQQKPGKAPKRLIYAASSLQSGVPSRFSGSGS GTEFTLTISSLQPEDFATYYCLHHYNYPRTFGQG TKVEIT | QVQLVESGGGVVQPGRSLRLSCAASGFTFSSYVMH WVRQAPGKGLEWVAVIWYDGSNKYYADSVKGRF TISRDNSKNTLYLQMNSLRAEDTAVYYCAREPYSS SWYDYGMDVGGQGTTVTVSS |
| | | | SEQ ID NO: 25230 | SEQ ID NO: 29236 |
| iPS:433937 | 21-225_44B10 | NA | CATATTGTGATGACCCAGACTCCACTCTCTCTG TCCGTCACCCCTGGACAGCCGGCCTCCATCTC CTGCAAGTCTAGTCAGAGCCTCCTGCATAGTG AGGGAAGGACCTATTTGTATTGGTACCTGCAG AAGCCAGGCCAGCCTCCACAGCTCCTGATCTA TGAAATTTCCCACCGGTTCTCTGGAGTGCCAG ATAGATTCAGTGGCAGCGGGTCAGGGACAGA TTTCACACTGAAAATCAGCCGGGTGGAGGCTG AGGATGTTGGGGTTTATTACTGCATGCAAAGT ATCCACCTTCCGTTCACTTTCGGCGGAGGGAC CAAGGTGGAGATCAAA | CAGGTGCAGTTGGTGGAGTCTGGGGGAGGCGTG GTCCAGCCTGGGAGGTCCCTGAGACTCTCCTGTG TAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTATTGTGCGAGGCGGTATAG CAGCAGCTGGGTGGGGGGGTATGGACGTCTGGGG CCAAGGGACCACGGTCACCGTCTCCTCA |
| | | | SEQ ID NO: 25231 | SEQ ID NO: 29237 |
| | | AA | HIVMTQTPLSLSVTPGQPASISCKSSQSLLHSEGR TYLYWYLQKPGQPPQLLIYEISHRFSGVPDRFSG SGSGTDFTLKISRVEAEDVGVYYCMQSIHLPFTF GGGTKVEIK | QVQLVESGGGVVQPGRSLRLSCVASGFTFSSYGMH WVRQAPGKGLEWVAVIWYDGSNKYYADSVKGRF TISRDNSKNTLYLQMNSLRAEDTAVYYCARRYSSS WVGGMDVWGQGTTVTVSS |
| | | | SEQ ID NO: 25232 | SEQ ID NO: 29238 |

FIGURE 50

| iPS:433939 | 21-225_44C10 | NA | GACATCCAGATGACCCAGTCTCCATCCTCCCT GTCTGCATCTGTAGGAGACAGAGTCACCTTCA CTTGCCGGGCAAGTCAGGGCATTAGAGATGAT TTAGGCTGGTATCAGCAGAAACCAGGGAAAG CCCCTAAGCGCCTGATCTATGCTACATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTGTGTCAT GCACTGGGTCCGCCAGGCTCCAGGCAAGGGGCT GGAGTGGGTGGCAGTTATATGGTATGATGGAAGT AATAAATACTATGCTGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAAAGGTAT AGCAGTGGCTTGTACGACTACGGTATGGACGTCT GGGGCCAAGGGACCACGGTCACCGTCTCCTCA |
| | | | SEQ ID NO: 25233 | SEQ ID NO: 29239 |
| | | AA | DIQMTQSPSSLSASVGDRVTFTCRASQGIRDDLG WYQQKPGKAPKRLIYATSSLQSGVPSRFSGSGSG TEFTLTISSLQPEDFATYYCLQHYSYPRTFGQGT KVEIK | QVQLVESGGGVVQPGRSLRLSCAASGFTFSDCVMH WVRQAPGKGLEWVAVIWYDGSNKYYADSVKGRF TISRDNSKNTLYLQMNSLRAEDTAVYYCARERYSS GLYDYGMDVWGQGTTVTVSS |
| | | | SEQ ID NO: 25234 | SEQ ID NO: 29240 |
| iPS:433941 | 21-225_44D10 | NA | GACATCCAGATGACCCAGTCTCCATCTTCCGT GTCTGCATCTGTAGGAGACAGAGTCACCATCA CTTGTCGGGCGAGTCAGGGTATTAGCGACTGG TTAGCCTGGTATCAACAGAAACCAGGGAAAG CCCCTAGGCTCCTGATCTATGCTGCATCCAGT TGCAAAGTGGGGTCCCATCAAAATTCAGCGGC AGTGGATCTGGGACAGATTTCACTCTCACCAT CAGCAACCTGCAGCCTGAAGATTTTGCAACTT ACTATTGTCAACAGACTAACAGTTTCCCGTGG ACGTTCGGCCAAGGGACCAAGGTGGAAATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGTT AACACATTCGACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAGGAAAGGA GTGGGAGCTATTTTGACTACTGGGGCCAGGGAAC CCTGGTCACCGTCTCCTCA |
| | | | SEQ ID NO: 25235 | SEQ ID NO: 29241 |

FIGURE 50

| | | | | |
|---|---|---|---|---|
| | | AA | DIQMTQSPSSVSASVGDRVTITCRASQGISDWLA WYQQKPGKAPRLLIYAASSLQSGVPSKFSGSGS GTDFTLTISNLQPEDFATYYCQQTNSFPWTFGQG TKVEIK | EVQLLESGGGLVQPGGSLRLSCAASGFTFSSYAMS WVRQAPGKGLEWVSAISGRGVNTFDADSVKGRFTI SRDNSKNTLYLQMNSLRAEDTAVYYCAKERSGSY FDYWGQGTLVTVSS |
| | | | SEQ ID NO: 25236 | SEQ ID NO: 29242 |
| iPS:433943 | 21-225_44E10 | NA | GATATTGTGATGACTCAGTCTCCACTCTCCCTG CCCGTCACCCCTGGAGAGCCGGCCTCCATCTC CTGCAGGTCTAGTCAGAGCCTCCTGCATAGTA ATGGATACAGCTATTTGGAGTGGTACCTGCAG AAGCCAGGACAGTCTCCACAACTCCTGATCTA TTTGGGTTCTAATCGGGCCTCCGGGGTCCCTG ACAGGTTCAGTGGCAGTGGATCAGGCACAGAT TTTACACTGAAAATCAGCAGAGTAGAGGCTGA GGATGTTGGGGTTTATTACTGCATGCAAACTC TACAAACTCCATTCACTTTCGGCCCTGGGACC AAAGTGGATATCAAA | GAGGTGCAGCTGTTGGAGTCTGGGGGAGGCTTG GTACAGTCTGGAGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGGTGTTGTTGGTAGTGGTGGT AGAACATACTACGCAGACTCCGTGAAGGGCCGG TTCATCATCTCCAGAGACAATTCCAAGAACACGC TGTATCTGCAAATGAACAGCCTGAGAGCCGAGG ACACGGCCGTATATTACTGTGCGAAAGATCGGG GGCAGTGGCTCCTAGGCGGTATGGACGTCTGGG GCCAAGGGACCACGGTCACCGTCTCCTCA |
| | | | SEQ ID NO: 25237 | SEQ ID NO: 29243 |
| | | AA | DIVMTQSPLSLPVTPGEPASISCRSSQSLLHSNGY SYLEWYLQKPGQSPQLLIYLGSNRASGVPDRFSG SGSGTDFTLKISRVEAEDVGVYYCMQTLQTPFTF GPGTKVDIK | EVQLLESGGGLVQSGGGSLRLSCAASGFTFSSYAMN WVRQAPGKGLEWVSGVVGSGGRTYYADSVKGRFI ISRDNSKNTLYLQMNSLRAEDTAVYYCAKDRGQW LLGGMDVWGQGTTVTVSS |
| | | | SEQ ID NO: 25238 | SEQ ID NO: 29244 |

FIGURE 50

| | | | | |
|---|---|---|---|---|
| iPS:433945 | 21-225_44C12 | NA | GACATCCAGATGACCCAGTCTCCATCTTCCGT GTCTGCATCTGTAGGAGACAGAGTCACCATCA CTTGTCGGGCGAGTCAGGGTATTAGCAACTGG TTAGCCTGGTATCAGCAGAAACCAGGGAAAG CCCCTAAGCTCCTGATCTCTGCTGCATTCAGTT TGCAAAGTGGGGTCCCATCAAGGTTCAGCGGC AGTGGATCTGGGACAGATTTCACTCTCAGCAT CAGCAGCCTGCAGCCTGAAGATTTTGCAACTT ACTATTGTCAACAGTCTAACAGTTTCCCGTGG ACGTTCGGCCAAGGGACCAAGGTGGAAATCA AA | GAGGTGCAGCTGGTGGAGTCTGGGGGAGGCTTG GTACAGCCTGGGGGGTCCCTGAGACTCTCCTGTG CAGCCTCTGGATTCACCTTCAGTAGCTATAGCGT GAACTGGGTCCGCCAGGCTCCAGGGAAGGGGCT GGAGTGGGTTTCATACATTAGTAGTAGTAGTAGT ACCATATACTACGCAGACTCTGTGAAGGGCCGAT TCACCATCTCCAGAGACAATGCCAAGAACTCACT GTATCTGCAAATGAACAGCCTGAGAGACGAGGA CACGGCTGTGTATTACTGTGCGAGAAGTGGATAC AGCTATGCTTACTACTACTACTACGGTATGGACG TCTGGGGCCAAGGGACCACGGTCACCGTCTCCTC A |
| | | | SEQ ID NO: 25239 | SEQ ID NO: 29245 |
| | | AA | DIQMTQSPSSVSASVGDRVTITCRASQGISNWLA WYQQKPGKAPKLLISAAFSLQSGVPSRFSGSGSG TDFTLSISSLQPEDFATYYCQQSNSFPWTFGQGT KVEIK | EVQLVESGGGLVQPGGSLRLSCAASGFTFSSYSVN WVRQAPGKGLEWVSYISSSSSTIYYADSVKGRFTIS RDNAKNSLYLQMNSLRDEDTAVYYCARSGYSYAY YYYYGMDVWGQGTTVTVSS |
| | | | SEQ ID NO: 25240 | SEQ ID NO: 29246 |
| iPS:433947 | 21-225_44E12 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAGTTACCCGCTC ACTTTCGGCGGAGGGACCAAGGTGGAGATCA AA | CAGGTGCACCTGGCGGAGTCTGGGGGAGGCGTG GTCCAGCCTGGGAGGTCCCTGAGACTCTCCTGTG AAGCGTCTGGATTCACCCTCAGTAGCGATGACAC GCACTGGGTCCGCCAGCCTCCAGGCAAGGGGCT GGAGTGGGTGGCAGTTATATGGTTTGATGAATAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACGCGGCTGTGTATTACTGTGCGAGAGATCTAAT AGCAGCAGCTGGGACGGGAGACTACTGGGGCCA GGGAACCCTGGTCACCGTCTCCTCA |
| | | | SEQ ID NO: 25241 | SEQ ID NO: 29247 |

1774

FIGURE 50

| | | | | |
|---|---|---|---|---|
| | | AA | DIQMTQSPSSLSASVGDRVTITCRTSQGIRNDLG WYQQKPGKAPKRLIYAASSLQSGVPSRFSGSGS GTEFTLTISSLQPEDFATYYCLQHNSYPLTFGGG TKVEIK | QVHLAESGGGVVQPGRSLRLSCEASGFTLSSDDTH WVRQPPGKGLEWVAVIWFDEYNKYYADSVKGRF TISRDNSKNTLYLQMNSLRAEDAAVYYCARDLIAA AGTGDYWGQGTLVTVSS |
| | | | SEQ ID NO: 25242 | SEQ ID NO: 29248 |
| IPS:433949 | 21-225_45H2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGGTGCATCCAAT TTGCAAAGTGGGGTCCCATCAAGGTTCAGCGG CAGTGGATCTGGGACAGAATTCACTCTCACAG TCAGCAGCCTGCAGCCTGAAGATTTTGCAACT TATTTCTGTCTACAGCATACTAGTTTCCCATTC ACTTTCGGCCCTGGGACCAAAGTGGATATCAA A | CAGGTGCAGCTGGTGGAGTCTGGGGGAGGCTTG GTCAAGCCTGGAGGGTCCCTGAGACTCTCCTGTG CAGCCTCTGGATTCACCTTTAGTGACTACTACAT GAACTGGATCCGCCAGGCTCCAGGGAAGGGGCT GGAGTGGGTTTCATACATTAGTAGTAGTGGTATT ACCAAATACTACGCAGACTCTGTGAAGGGCCGA TTCACCATCTCCAGGGACAACGCCAAGAACTCAC TGTATCTGCAAATGAACAGCCTGAGAGCCGAGG ACACGGCCGTGTATTACTGTGCGGCCGATACAAT CTACTGGGGCCAGGGAACCCTGGTCACCGTCTCC TCA |
| | | | SEQ ID NO: 25243 | SEQ ID NO: 29249 |
| | | AA | DIQMTQSPSSLSASVGDRVTITCRASQGIRNDLG WYQQKPGKAPKRLIYGASNLQSGVPSRFSGSGS GTEFTLTVSSLQPEDFATYFCLQHTSFPFTFGPGT KVDIK | QVQLVESGGGLVKPGGSLRLSCAASGFTFSDYYMN WIRQAPGKGLEWVSYISSSGITKYYADSVKGRFTIS RDNAKNSLYLQMNSLRAEDTAVYYCAADTIYWGQ GTLVTVSS |
| | | | SEQ ID NO: 25244 | SEQ ID NO: 29250 |

FIGURE 50

| iPS:433951 | 21-225_45B4 | NA | GACATCCAGATGACCCAGTCTCCATCCTCCCT GTCTGCATCTGTAGGAGACAGAGTCACCTTCA CTTGCCGGGCAAGTCAGGGCATTAGAGATGAT TTAGGCTGGTATCAACAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTGTGTCAT GCACTGGGTCCGCCAGGCTCCAGGCAAGGGGCT GGAGTGGGTGGCAGTTATATGGTATGATGGAAGT AATAAATACTATGCTGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAAAGGTAT AGCAGTGGCTTGTACGACTACGGTATGGACGTCT GGGGGCCAAGGGACCACGGTCACCGTCTCCTCA |
| | | | SEQ ID NO: 25245 | SEQ ID NO: 29251 |
| | | AA | DIQMTQSPSSLSASVGDRVTFTCRASQGIRDDLG WYQQKPGKAPKRLIYAASSLQSGVPSRFSGSGS GTEFTLTISSLQPEDFATYYCLQHYSYPRTFGQG TKVEIK | QVQLVESGGGVVQPGRSLRLSCAASGFTFSDCVMH WVRQAPGKGLEWVAVIWYDGSNKYYADSVKGRF TISRDNSKNTLYLQMNSLRAEDTAVYYCARERYSS GLYDYGMDVWGQGTTVTVSS |
| | | | SEQ ID NO: 25246 | SEQ ID NO: 29252 |
| iPS:433953 | 21-225_45H4 | NA | GACATCCAGATGACCCAGTCTCCATCTTCCGT GTCTGCATCTGTAGGAGACAGAGTCACCATCA CTTGTCGGGCGAGTCAGGATATTAGCAGCTGG TTAGCCTGGTATCAGAAGAAACCAGGGAAAG CCCCTAAGTACCTGATCTATGATGCATCCAGT TTGCAAAGTGGGGTCCCATCAAGGTTCAGCGG CAGTGGATCTGGGACAGATTTCATTCTCACCA TCAGCAGCCTGCAGCCTGAAGATTTTGCAATT TACTTTTGTCAACAGGCTAACAGTCTCCCTTTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGTTGGGTCCGCCAGGCTCCAGGGATGGGGCT GGAGTGGGTCTCAGCTATTAGTGGTAGTGGTAGT AACACATTCTACGCAGACTCCGTGAAGGGCCGGT TCACCATCTCCAGAGACAATTCCAAGAACACGCT GTATCTGCACATGAACAGCCTGAGAGCCGAGGA CACTGCCGTATATTTCTGTGCGAAACGAACGCCC TCTGATGTTTTTGATATCTGGGGCCAAGGGACAA TGGTCACCGTCTCTTCA |
| | | | SEQ ID NO: 25247 | SEQ ID NO: 29253 |

FIGURE 50

| | | | | |
|---|---|---|---|---|
| | | AA | DIQMTQSPSSVSASVGDRVTITCRASQDISSWLA WYQKKPGKAPKYLIYDASSLQSGVPSRFSGSGS GTDFILTISSLQPEDFAIYFCQQANSLPFTFGPGTK VDIK | EVQLLESGGGLVQPGGSLRLSCAASGFTFSSYAMS WVRQAPGMGLEWVSAISGSGSNTFYADSVKGRFTI SRDNSKNTLYLHMNSLRAEDTAVYFCAKRTPSDVF DIWGQGTMVTVSS |
| | | | SEQ ID NO: 25248 | SEQ ID NO: 29254 |
| iPS:433955 | 21-225_45B8 | NA | GACATCCAGATGACCCAGTCTCCATCCTCCCT GTCTGCATCTGTAGGAGACAGAGTCACCTTCA CTTGCCGGGCAAGTCAGGACATTAGAGATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAATTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTGTGTCAT GCACTGGGTCCGCCAGGCTCCAGGCAAGGGGCT GGAGTGGGTGGCAGTTATATGGTATGATGGAAGT AATAAATACTATGCTGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAAAGGTAT AGCAGTGGCTTGTACGACTACGGTATGGACGTCT GGGGCCAAGGGACCACGGTCACCGTCTCCTCA |
| | | | SEQ ID NO: 25249 | SEQ ID NO: 29255 |
| | | AA | DIQMTQSPSSLSASVGDRVTFTCRASQDIRDDLG WYQQKPGKAPKRLIYAASSLQSGVPSRFSGSGS GTEFTLTISSLQPEDFATYYCLQHYNYPRTFGQG TKVEIK | QVQLVESGGGVVQPGRSLRLSCAASGFTFSDCVMH WVRQAPGKGLEWVAVIWYDGSNKYYADSVKGRF TISRDNSKNTLYLQMNSLRAEDTAVYYCARERYSS GLYDYGMDVWGQGTTVTVSS |
| | | | SEQ ID NO: 25250 | SEQ ID NO: 29256 |

FIGURE 50

| | | | | |
|---|---|---|---|---|
| iPS:433957 | 21-225_45F8 | NA | GACATCCAGATGACCCAGTCTCCATCTTCCGT GTCTGCATCTGTAGGAGACAGAGTCACCATCA CTTGTCGGGCGAGTCAGGGTATTAGCGACTGG TTAGCCTGGTATCAGCAGAAACCAGGGAAAG CCCCTAGGCTCCTGATCTATGGTGCATCCAGTT TGCAAAGTGGGGTCCCATCAAGGTTCAGCGGC AGTGGATCTGGGACAGATTTCACTCTCACCAT CAGCAACCTGCAGCCTGAAGATTTTGCAACTT ACTATTGTCAACAGACTAACAGTTTCCCGTGG ACGTTCGGCCAAGGGACCAAGGTGGAAATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGTT AACACATTCGACGCAGACTCCGTGAAGGGCCGG TTCACCATATCCAGAGACAATTCCAAGAACACGC TGTATCTGCAAATGAACAGCCTGAGAGCCGAGG ACACGGCCGTATATTACTGTGCGAAGGAAAGGA GTGGGAGCTATTTTGACTACTGGGGCCAGGGAAC CCTGGTCACCGTCTCCTCA |
| | | | SEQ ID NO: 25251 | SEQ ID NO: 29257 |
| | | AA | DIQMTQSPSSVSASVGDRVTITCRASQGISDWLA WYQQKPGKAPRLLIYGASSLQSGVPSRFSGSGSG TDFTLTISNLQPEDFATYYCQQTNSFPWTFGQGT KVEIK | EVQLLESGGGLVQPGGSLRLSCAASGFTFSSYAMS WVRQAPGKGLEWVSAISGRGVNTFDADSVKGRFTI SRDNSKNTLYLQMNSLRAEDTAVYYCAKERSGSY FDYWGQGTLVTVSS |
| | | | SEQ ID NO: 25252 | SEQ ID NO: 29258 |
| iPS:433959 | 21-225_45C9 | NA | GACATCCAGATGACCCAGTCTCCATCTTCCGT GTCTGTTTCTGTAGGAGACAGAGTCACCATCA CTTGTCGGGCGAGTCAGGATATTAGCGACTGG TTAGCTTGGTATCAGCAGAGACCAGGGAAAGC CCCTAAGCTCTTGATCTATGCTGCATCCAGTTT GGAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAACAGCTATGCCAT GAGCTGGGTCCGCCAGGCTCCAGGGAAGGGGCT GGAGTGGGTCTCAGTTATTAGTGGTCGTGGTGGT ACCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAACGAACGCC CTCTGATGCTTTTGATATCTGGGGCCAAGGGACA ATGGTCACCGTCTCTTCA |
| | | | SEQ ID NO: 25253 | SEQ ID NO: 29259 |

FIGURE 50

| | | | | | |
|---|---|---|---|---|---|
| | | AA | DIQMTQSPSSVSVSVGDRVTITCRASQDISDWLA WYQQRPGKAPKLLIYAASSLESGVPSRFSGSGSG TDFTLTISSLQPEDFATYYCQQANSFPFTFGPGTK VDIK | EVQLLESGGGLVQPGGSLRLSCAASGFTFNSYAMS WVRQAPGKGLEWVSVISGRGGTTYYADSVKGRFT ISRDNSKNTLYLQMNSLRAEDTAVYYCAKRTPSDA FDIWGQGTMVTVSS | |
| | | | SEQ ID NO: 25254 | SEQ ID NO: 29260 | |
| IPS:433961 | 21-225_45D9 | NA | GACATCCAGATGACCCAGTCTCCATCCTCACT GTCTGCATCTGTAGGAGACAGAGTCACCATCA CTTGTCGGGCGAGTCAGGGCATTAACAATTAT TTAGCCTGGTTTCAGCAGAAACCAGGGAAAGC CCCTAAGTCCCTGATCAATGCTGCATCCAGTTT GCAAAGTGGGGTCCCATCAAGGTTCAGCGGCA GTGGATCTGGGACAGATTTCACTCTCGCCATC AGCAGCCTACAGCCTGAAGATTTTGCAACTTA TTACTGCCAACACTATTATAGTTACCCATTCAC TTTCGGCCGTGGGACCAAAGTGGATATCAAA | GAGGTGCAGCTGGTGGAGTCTGGGGGAGGCCTG GTCAAGCCGGGGGGGTCCCTGAGACTCTCCTGTG CAGCCTCTGGATTCACCTTCAGTAGCTATACCAT GAACTGGGTCCGCCAGGTTCCAGGGAAGGGGCT GGAGTGGGTCTCATCCATTAGTGGAAGTAGTACT TACATATACTACGCAGACTCAGTGAAGGGCCGAT TCACCATCTCCAGAGACGACGCCCAGAACTCACT GTATCTGCAAATGAACAGCCTGAGAGGCGAGGA CACGGCTGTGTATTACTGTGCGAGGGTGACCTCT TTTGACTACTGGGGCCAGGGAGCCCTGGTCACCG TCTCCTCA | |
| | | | SEQ ID NO: 25255 | SEQ ID NO: 29261 | |
| | | AA | DIQMTQSPSSLSASVGDRVTITCRASQGINNYLA WFQQKPGKAPKSLINAASSLQSGVPSRFSGSGSG TDFTLAISSLQPEDFATYYCQHYYSYPFTFGRGT KVDIK | EVQLVESGGGLVKPGGSLRLSCAASGFTFSSYTMN WVRQVPGKGLEWVSSISGSSTYIYYADSVKGRFTIS RDDAQNSLYLQMNSLRGEDTAVYYCARVTSFDY WGQGALVTVSS | |
| | | | SEQ ID NO: 25256 | SEQ ID NO: 29262 | |

FIGURE 50

| iPS:433963 | 21-225_46B1 | NA | GACATCCAGATGACCCAGTCTCCATCCTCCCTGTCTGCATCGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAGGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAAA | CAGGTGCACCTGGCGGAGTCTGGGGGAGGCGTGGTCCAGCCTGGGAGGTCCCTGAGACTCTCCTGTGAAGCGTCTGGATTCACCCTCAGTAGCGATGACTCGCACTGGGTCCGCCAGCCTCCAGGCAAGGGGCTGGAGTGGGTGGCAGTTATATGGTTTGATGAATATACTAAATACTATGCAGACTCCGTGAAGGGCCGATTCACCATCTCCAGAGACAATTCCAAGAACACGCTGTATCTGCAAATGAACAACCTGAGAGCCGAGGACGCGGCTGTGTATTACTGTGCGAGAGATCTAATAGCAGCAACTGGGACGGGAGACTACTGGGGCCAGGGAACCCTGGTCACCGTCTCCTCA |
| | | | SEQ ID NO: 25257 | SEQ ID NO: 29263 |
| | | AA | DIQMTQSPSSLSASVGDRVTITCRASQGIRNDLGWYQQKPGKAPKRLIYAASSLQGGVPSRFSGSGSGTEFTLTISSLQPEDFATYYCLQHNSYPLTFGGGTKVEIK | QVHLAESGGGVVQPGRSLRLSCEASGFTLSSDDSHWVRQPPGKGLEWVAVIWFDEYTKYYADSVKGRFTISRDNSKNTLYLQMNNLRAEDAAVYYCARDLIAATGTGDYWGQGTLVTVSS |
| | | | SEQ ID NO: 25258 | SEQ ID NO: 29264 |
| iPS:433965 | 21-225_46F2 | NA | GATATCGTGATGACCCAGACTCCACTCTCTCTGACCGTCACCCCTGGACAGCCGGCCTCCATCTCCTGCAAGTCTAGTCAGAGCCTCCTGCATGGTGATGGAAAGACATATTTGTATTGGTACCTGCAGAAGCCAGGCCAGCCTCCACAGGTCCTGATCTATGAAGTTTCCAATCGGTTCTCTGGAGTGCCAGATAGGTTCAGTGGCAGCGGGTCAGGGACAGATTTCACACTGAAACTCAGCCGGGTGGAGGCTGAGGATGTTGGGGTTTATTACTGCATGCAAAGTATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGATACGATTTTTGGAGTGGTTACTTTGACTACTGGGGCCAGGGAACCCTGGTCACCGTCTCCTCA |
| | | | SEQ ID NO: 25259 | SEQ ID NO: 29265 |

FIGURE 50

| | | | | |
|---|---|---|---|---|
| | | AA | DIVMTQTPLSLTVTPGQPASISCKSSQSLLHGDGKTYLYWYLQKPGQPPQVLIYEVSNRFSGVPDRFSGSGSGTDFTLKLSRVEAEDVGVYYCMQSIQLPWTFGQGTKVEIK | QVQLVESGGGVVQPGRSLRLSCAASGFTFSSYGMHWVRQAPGKGLEWVAIIWYDGSNKYYVDSVKGRFTISRDNSKNTLYLQMNSLRAEDTAVYYCARDRYDFWSGYFDYWGQGTLVTVSS |
| | | | SEQ ID NO: 25260 | SEQ ID NO: 29266 |
| iPS:433967 | 21-225_46C3 | NA | GACATCCAGATGACCCAGTCTCCATCCTCCCTGTCTGCATCTGTAGGAGACAGAGTCACCTTCACTTGCCGGGCAAGTCAGGGCATTAGAGATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTAGATCTGGGACAGAATTCACTCTCACAATCAGCAGCCTGCAACCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCTGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAGGTATAGCAGTGGCTTGTACGACTACGGTATGGACGTCTGGGGGCCAAGGGACCACGGTCACCGTCTCCTCA |
| | | | SEQ ID NO: 25261 | SEQ ID NO: 29267 |
| | | AA | DIQMTQSPSSLSASVGDRVTFTCRASQGIRDDLGWYQQKPGKAPKRLIYAASSLQSGVPSRFSGSRSGTEFTLTISSLQPEDFATYYCLQHYSYPRTFGQGTKVEIK | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCARERYSSGLYDYGMDVWGQGTTVTVSS |
| | | | SEQ ID NO: 25262 | SEQ ID NO: 29268 |

FIGURE 50

| iPS:433969 | 21-225_46F3 | NA | GACATCCAGATGACCCAGTCTCCATCCTCCCTGTCTGCATCTGTAGGAGACAGAGTCACCATCACTTGCCGGGCAAGTCAGGGCATTAGAAAAGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCAGTCTCACAATCAGCAGCCTGCAGCCTGAAGATTTTGCAACTTATTACTGTCTACAGCATAATAGTTACCCTCTCACTTTCGGCGGAGGGACCAAGGTGGAGATCAAG | CAGGTGCAGCTGGTGGAGTCTGGGGGAGGCGTGGTCCAGCCTGGGAGGTCCCTGAGACTCTCCTGTGCAGCGTCTGGATTCACCTTCAGTGATTATGGCATGCACTGGGTCCGCCAGGCTCCAGGCAAGGGGCTGGAGTGGGTGGCAGTTATATGGTTTGAAGGAAGTAATAAATACTATGCAGATTCCGTGAAGGGCCGATTCACCATCTCCAGAGACAATTCCAAGAACACGCTGTATGTGCAAATGAACAGCCTGAGAGCCGAGGACACGGCTGTGTATTACTGTGTGAGAGAACTAGGATTTTCCAATGACTACTGGGGCCAGGGAACCCTGGTCACCGTCTCCTCA |
| | | | SEQ ID NO: 25263 | SEQ ID NO: 29269 |
| | | AA | DIQMTQSPSSLSASVGDRVTITCRASQGIRKDLGWYQQKPGKAPKRLIYAASSLQSGVPSRFSGSGSGTEFSLTISSLQPEDFATYYCLQHNSYPLTFGGGTKVEIK | QVQLVESGGGVVQPGRSLRLSCAASGFTFSDYGMHWVRQAPGKGLEWVAVIWFEGSNKYYADSVKGRFTISRDNSKNTLYVQMNSLRAEDTAVYYCVRELGFSNDYWGQGTLVTVSS |
| | | | SEQ ID NO: 25264 | SEQ ID NO: 29270 |
| iPS:433971 | 21-225_46D4 | NA | GACATCCAGATGACCCAGTCTCCATCCTCCCTGTCTGCATCTGTAGGAGACAGAGTCACCATCACTTGCCGGACAAGTCAGGACATTAGAAAAGATTTAGGCTGGTATCAGCAGAAAGTAGGGAAAGCCCCTAAGCGCCTGATCTATGCTGCATCCAGTTTGGAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTTCCCGTGGACGTTCGGCCAAGGGACCAAGGTGGAAATCAAA | CAGGTGCAGCTGGTGGAGTCTGGGGGAGGCGTAGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGTCCCGTATAGCAGCAGTTGGTACGACTACGGTATGGACGTCTGGGGCCAAGGGACCACGGTCACCGTCTCCTCA |
| | | | SEQ ID NO: 25265 | SEQ ID NO: 29271 |

FIGURE 50

| | | | | |
|---|---|---|---|---|
| | | AA | DIQMTQSPSSLSASVGDRVTITCRTSQDIRKDLG WYQQKVGKAPKRLIYAASSLESGVPSRFSGSGS GTEFTLTISSLQPEDFATYYCLQHYSFPWTFGQG TKVEIK | QVQLVESGGGVVQPGRSLRLSCAASGFTFSSYVMH WVRQAPGKGLEWVAVIWYDGSNKYYADSVKGRF TISRDNSKNTLYLQMNSLRAEDTAVYYCARVPYSS SWYDYGMDVWGQGTTVTVSS |
| | | | SEQ ID NO: 25266 | SEQ ID NO: 29272 |
| IPS:433973 | 21-225_46A6 | NA | GACATCCAGATGAACCAGTCTCCATCTTCCGT GTCTGCATCTGTAGGAGACAGAGTCAACATCA CTTGTCGGGCGAGTCAGGGTATTAGCAACTGG TTAGCCTGGTATCAGCAGAAACCAGGGAAAGT CCCTAAGCTCCTGATCTATGCTGCATCCAGTTT GCAAAGTGGGGTCCCATCAAGGTTCAGCGGCA GTGGATCTGGGACAGATTTCACTCTCACCATC AGCAACCTGCAGCCTGAAGATTTTGCAACTTA CTATTGTCAACAGACTAACAGTTTCCCGTGGA CGTTCGGCCAAGGGACCAAGGTGGAAATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTATT AACACATTCGACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAGGAAAGGA GTGGGAGCTATTTTGACTACTGGGGGCCAGGGAAC CCTGGTCACCGTCTCCTCA |
| | | | SEQ ID NO: 25267 | SEQ ID NO: 29273 |
| | | AA | DIQMNQSPSSVSASVGDRVNITCRASQGISNWLA WYQQKPGKVPKLLIYAASSLQSGVPSRFSGSGS GTDFTLTISNLQPEDFATYYCQQTNSFPWTFGQG TKVEIK | EVQLLESGGGLVQPGGSLRLSCAASGFTFSSYAMS WVRQAPGKGLEWVSAISGRGINTFDADSVKGRFTI SRDNSKNTLYLQMNSLRAEDTAVYYCAKERSGSY FDYWGQGTLVTVSS |
| | | | SEQ ID NO: 25268 | SEQ ID NO: 29274 |

FIGURE 50

| iPS:433975 | 21-225_46C6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCTCTC ACTTTCGGCGGAGGGACCAAGGTGGAGATCA CA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT ACACTGGGTCCGCCAGGCTCCAGGCAAGGGGCT GGAGTGGGTGGCAGTTATATGGTATGATGAAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGCTAGG ATTTTCCAATGACTACTGGGGCCAGGGAACCCTG GTCACCGTCTCCTCA |
| | | | SEQ ID NO: 25269 | SEQ ID NO: 29275 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIT | QVQLVESGGGVVQPGRSLRLSCAASGFTFSSYGIH WVRQAPGKGLEWVAVIWYDENNKYYADSVKGRF TISRDNSKNTLYLQMNSLRAEDTAVYYCARELGFS NDYWGQGTLVTVSS |
| | | | SEQ ID NO: 25270 | SEQ ID NO: 29276 |
| iPS:433977 | 21-225_46D8 | NA | GACATCCAGATGACCCAGTCTCCATCCTCCCT GTCTGCATCTGTAGGAGACAGAGTCACCATCA CTTGCCGGGCAAGTCAGGGCATTAGAAAAGAT TTAGGCTGGTATCAGCAGAAACCAGGGAAAG CCCCTAAGCGCCTGATCTATGCTGCATCCAGT TTGCAAAGTGGGGTCCCATCAAGGTTCAGTGG CAGTGGATCTGGGACAGAATTCAGTCTCACAA TCAGCAGCCTGCAGCCTGAAGATTTTGCAACT TATTACTGTCTACAGCATAATAGTTACCCTCTC ACTTTCGGCGGAGGGACCAAGGTGGAGATCA AG | CAGGTGCAGCTGGTGGAGTCTGGGGGAGGCGTG GTCCAGCCTGGGAGGTCCCTGAGACTCTCCTGTG CAGCGTCTGGATTCACCTTCAGTGATTATGGCAT ACACTGGGTCCGCCAGGCTCCAGGCAAGGGGCT GGAGTGGGTGGCAGTTATATGGTTTGAAGGAAGT AATAAATACTATGCAGATTCCGTGAAGGGCCGAT TCACCATCTCCAGAGACAATTCCAAGAACACGCT GTATGTGCAAATGAACAGCCTGAGAGCCGAGGA CACGGCTGTGTATTACTGTGTGAGAGAACTAGGA TTTTCCAATGACTACTGGGGCCAGGGAACCCTGG TCACCGTCTCCTCA |
| | | | SEQ ID NO: 25271 | SEQ ID NO: 29277 |

FIGURE 50

| | | | | |
|---|---|---|---|---|
| | | AA | DIQMTQSPSSLSASVGDRVTITCRASQGIRKDLG WYQQKPGKAPKRLIYAASSLQSGVPSRFSGSGS GTEFSLTISSLQPEDFATYYCLQHNSYPLTFGGGT KVEIK | QVQLVESGGGVVQPGRSLRLSCAASGFTFSDYGIH WVRQAPGKGLEWVAVIWFEGSNKYYADSVKGRF TISRDNSKNTLYVQMNSLRAEDTAVYYCVRELGFS NDYWGQGTLVTVSS |
| | | | SEQ ID NO: 25272 | SEQ ID NO: 29278 |
| iPS:433979 | 21-225_46B9 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GAGGGAAAGACCTATTTGTATTGGTACCTGCA GAAGCCAGGCCAGCCTCCACAGCTCCTGATCT ATGAAGTTTCTTACCGGTTCTCTGGAGTGCCA GATAGGTTCAGTGGCAGCGGGTCAGGGACAG ATTTCACACTGAAAATCAGCCGGATGGAGGCT GAGGATGTTGGGGTTTATTACTGCATGCACAG TATACAGTATCCGCTCACGTTTGGCGGAGGGA CCAAGGTGGAGATCCAA | CAGGTGCAGCTGGTGGAGTCTGGGGGAGGCGTG GTCCAGCCTGGGAGGTCCCTGAGACTCTCCTGTT CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAG AAATAAATACTATGCAGACTCCGTGAAGGGCCG AATCACCATCTCCAGAGACAATTCCAAGAACAC GCTGTATCTGCAAATGAACAGCCTGAGAGCCGA GGACACGGCTGTGTATTACTGTGCGAGGCGGTAT AGCAGCAGCTGGATGGGGAGGTATGGACGTCTGG GGCCAAGGGACCACGGTCACCGTCTCCTCA |
| | | | SEQ ID NO: 25273 | SEQ ID NO: 29279 |
| | | AA | DIVMTQTPLSLSVTPGQPASISCKSSQSLLHSEGK TYLYWYLQKPGQPPQLLIYEVSYRFSGVPDRFSG SGSGTDFTLKISRMEAEDVGVYYCMHSIQYPLTF GGGTKVEIQ | QVQLVESGGGVVQPGRSLRLSCSASGFTFSSYGMH WVRQAPGKGLEWVAVIWYDGRNKYYADSVKGRI TISRDNSKNTLYLQMNSLRAEDTAVYYCARRYSSS WMGGMDVWGQGTTVTVSS |
| | | | SEQ ID NO: 25274 | SEQ ID NO: 29280 |

FIGURE 50

| iPS:433981 | 21-225_46E9 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAGTCAGCAGCCTGCAGCCTGAAGATTTTGCAACTTATTTCTGTCTTCAGCATACTAGTTTCCCATTCACTTTCGGCCCTGGGACCAAAGTGGATATCAAA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTACATGAACTGGATCCGCCAGGCTCCAGGGAAGGGGCTGGAGTGGGTTTCATACATTAATAGTAATGGTTTTACCATATACTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGGCCGATACAATCTACTGGGGCCAGGGAACCCTGGTCACCGTCTCCTCA |
| | | | SEQ ID NO: 25275 | SEQ ID NO: 29281 |
| | | AA | DIQMTQSPSSLSASVGDRVTITCRASQGIRNDLGWYQQKPGKAPKRLIYAASSLQSGVPSRFSGSGSGTEFTLTVSSLQPEDFATYFCLQHTSFPFTFGPGTKVDIK | QVQLVESGGGLVKPGGSLRLSCAASGFTFSDYYMNWIRQAPGKGLEWVSYINSNGFTIYYADSVKGRFTISRDNAKNSLYLQMNSLRAEDTAVYYCAADTIYWGQGTLVTVSS |
| | | | SEQ ID NO: 25276 | SEQ ID NO: 29282 |
| iPS:433983 | 21-225_47A1 | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGGGAAAGCCCCTAAGCGCCTGATCTATGCTGCATTCAGTTTGCAAAGTGGGGTCCCATCAAGGTTCAGCGGCAGTAGATCTGGGACAGAATTCACTCTCACAATCAGCAGCCTGCAGCCTGAAGATTTTGCAACTTATTACTGTCTGCAACATAATAGTTACCCGCTCACTTTCGGCGGAGGGACCAAGGTGGAGATCAAA | CAGGTGCAGCTGGTGGAGTCGGGGGGAGGCGTGGTCCAGCCTGGGAGGTCCCTGAGACTCTCCTGTGCAGCGTCTGGATTCACCTTCAGTGACTATGGCATGCACTGGGTCCGCCAAGCTCCAGGCAAGGGGCTGGAGTGGGTGGCAGTTATATGGTATGATGACTATAATAAAAAGTATGCAGACTCCGTGAAGGGCCGATTCACCATCTCCAGAGACAATGCCAAGAACACGCTGTATCTGCAAGTGAACAGCCTGAGAGTCGAAGACACGGCTGTGTATTACTGTGCGACAGAACTGGGGATGCTCTTTGACTACTGGGGCCAGGGAACCCTGGTCACCGTCTCCTCA |
| | | | SEQ ID NO: 25277 | SEQ ID NO: 29283 |

FIGURE 50

| | | | | |
|---|---|---|---|---|
| | | AA | DIQMTQSPSSLSASVGDRVTITCRASQDIRNDLG WYQQKPGKAPKRLIYAAFSLQSGVPSRFSGSRS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYDDYNKKYADSVKG RFTISRDNAKNTLYLQVNSLRVEDTAVYYCATELG MLFDYWGQGTLVTVSS |
| | | | SEQ ID NO: 25278 | SEQ ID NO: 29284 |
| iPS:433985 | 21-225_47C1 | NA | GATATTGTGATGACCCAGACTCCACTCTCTCT GTCCGTCACCCCTGGACAGCCGGCCTCCATCT CCTGCACGTCTAGTCAGAGCCTCCTGCATAGT GAAGGAAAGACCTATTTGTATTGGTACCTCCA GAAGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACACTAAAAATCAGCCGGGTGGAGGCT GAGGATGTTGGGGTTTTTTACTGTATGCAAAG TATACAGCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGACTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTTTCTGCAAATGAACAGCCTGAGAGACGAGG ACACGGCTGTGTATTACTGTGCGAGACGGTATAG CCGCAGCTGGGTGGGAGGTATGGACGTCTGGGG CCAAGGGACCACGGTCACCGTCTCCTCA |
| | | | SEQ ID NO: 25279 | SEQ ID NO: 29285 |
| | | AA | DIVMTQTPLSLSVTPGQPASISCTSSQSLLHSEGK TYLYWYLQKPGQPPQLLIYEVSNRFSGVPDRFSG SGSGTDFTLKISRVEAEDVGVFYCMQSIQLPWTF GQGTKVEIK | QVQLVESGGGVVQPGRSLRLSCAASGFTFSDYGM HWVRQTPGKGLEWVAVIWYDGSNKYYADSVKGR FTISRDNSKNTLFLQMNSLRDEDTAVYYCARRYSR SWVGGMDVWGQGTTVTVSS |
| | | | SEQ ID NO: 25280 | SEQ ID NO: 29286 |

FIGURE 50

| iPS:433987 | 21-225_47A5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACGATGACAC ACACTGGGTCCGCCAGGCTCCAGGCAAGGGGCT GGAGTGGGTGGCAGTTATATGGTTTGATGGAAGT AATAAATACTATGCAGACTCCGTGAAGGGCCGA TTCACCATCTCCAGAGACAATTCCAAGAACACGC TGTATCTGCAAATGAACAGCCTGAGCGCCGAGG ACACGGCTGTGTATTACTGTGCGAGAGATCTTAT AGCAGCAGCTGGTACAGTTGACTACTGGGGCCA GGGAACCCTGGTCACCGTCTCCTCA |
| | | | SEQ ID NO: 25281 | SEQ ID NO: 29287 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSDDDTH WVRQAPGKGLEWVAVIWFDGSNKYYADSVKGRF TISRDNSKNTLYLQMNSLSAEDTAVYYCARDLIAA AGTVDYWGQGTLVTVSS |
| | | | SEQ ID NO: 25282 | SEQ ID NO: 29288 |
| iPS:433989 | 21-225_47C7 | NA | GATATTGTGATGACTCAGTCTCCACTCTCCCCG CCCGTCACCCCTGGAGAGCCGGCCTCCATCTC CTGCAGGTCTAGTCAGAGCCTCCTGCATAGTA ATGGATACAACTATTTGGAATGGTACCTGCAG AAGTCAGGGCAGTCTCCACAGTTCCTGATCTA TTTGGGTTTTAATCGGGCCTCCGGGGTCCCTG ACAGGTTCACTGGCAGTGGATCAGGCACAGAT TTTACACTGAAAAATCAGCAGAGTGGAGGCTGA GGATGTTGGGGTTTATTACTGCATGCAAGTTC TACAAACTCCATTCACTTTCGGCCCTGGGACC AAAGTGGATATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGTAACTATGCCAT GAGCTGGGTCCGCCAGGCTCCAGGGAAGGGGCT GGAGTGGGTCTCAGGTATTAGTGGTAGTGGTAGT CGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAAGATCGGG GGCAGTGGCTCATAGGCGGTATGGACGTCTGGG GCCAAGGGACCACGGTCACCGTCTCCTCA |
| | | | SEQ ID NO: 25283 | SEQ ID NO: 29289 |

FIGURE 50

| | | | | |
|---|---|---|---|---|
| | | AA | DIVMTQSPLSPPVTPGEPASISCRSSQSLLHSNGY NYLEWYLQKSGQSPQFLIYLGFNRASGVPDRFT GSGSGTDFTLKISRVEAEDVGVYYCMQVLQTPF TFGPGTKVDIK | EVQLLESGGGLVQPGGSLRLSCAASGFTFSNYAMS WVRQAPGKGLEWVSGISGSGSRTYYADSVKGRFTI SRDNSKNTLYLQMNSLRAEDTAVYYCAKDRGQW LIGGMDVWGQGTTVTVSS |
| | | | SEQ ID NO: 25284 | SEQ ID NO: 29290 |
| iPS:433991 | 21-225_47E7 | NA | GATATTGTGATGACCCAAACTCCACTCTCTCT GTCCGTCACCCCTGGACAGCCGGCCTCCATCT CCTGCAAGTCTAGTCAGAGCCTCCTGCATAGT GATGGAAGGACCTATTTGTATTGGTACCTGCA GAAGCCAGGCCAGCCTCCACAGCTCCTGATCT ATGAAGTTTCCAGCCGGTTCTCTGGAGTGCCA GATAGGTTCAGTGGCAGCGGGTCAGGGACAG ATTTCGCACTGAAAATCAGCCGGGTGGAGGCT GAGGATGTTGGGGTTTATTACTGCATGCAAAG TACACAACTTCCGTGGACGTTCGGCCAAGGGA CCAAGGCGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTATCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTTCAAATGAACAGCCTGAGAGCCGAGG ACACGGCTGTGTATTACTGTGCGAGACGGTATAG CAGAAGCTGGGTGGGAGGTATGGACGTCTGGGG CCAAGGGACCACGGTCACCGTCTCCTCA |
| | | | SEQ ID NO: 25285 | SEQ ID NO: 29291 |
| | | AA | DIVMTQTPLSLSVTPGQPASISCKSSQSLLHSDGR TYLYWYLQKPGQPPQLLIYEVSSRFSGVPDRFSG SGSGTDFALKISRVEAEDVGVYYCMQSTQLPWT FGQGTKABIK | QVQLVESGGGVVQPGRSLRLSCAASGFTFSIYGMH WVRQAPGKGLEWVAVIWYDGSNKYYADSVKGRF TISRDNSKNTLYLQMNSLRAEDTAVYYCARRYSRS WVGGMDVWGQGTTVTVSS |
| | | | SEQ ID NO: 25286 | SEQ ID NO: 29292 |

FIGURE 50

| iPS:433993 | 21-225_47G7 | NA | GACATCCAGATGACCCAGTCTCCATCTTCCGT GTCTGCATCTGTAGGAGACAGAGTCACCATCA CTTGTCGGGCGAGTCAGGGTATTAGCAACTGG TTAGCCTGGTATCAGCAGAAACCAGGGAAAG CCCCTAAGCTCCTGATCTTTGCTGCCTCCAATT TGCAAAGTGGGGTCCCATCAAGGTTCAGCGGC AGTGGATCTGGGACAGATTTCACTCTCACCAT CAGCAGTCTGCAGCCTGCAGATTTTGCAACTT ACTGTTGTCAACAGGTTAACAGTTTCCCGTGG ACGTTCGGCCAAGGGACCAAGGTGGAAATCA AA | GAGGTGCAGTTGTTGGACTCTGGGGGAGGCTTGG TGCAGCCTGGGGGGTCCCTGAGACTCTCCTGTGC AGCCTCTGGATTCACCTTTAGCAGCTATGCCATG AGTTGGGTCCGCCAGGCTCCAGGGAAGGGGCTG GAGTGGGTCTCAGCTATTAGTGGTCGTGGTGGTA ACACATTCTACGCAGAGTCCGTGAGGGGCCGGTT CACCATCTCCAGAGACAATTCCAAGAACACGCTG TATCTGCAAATGAACAGCCTGAGAGCCGAGGAC ACGGCCGTATATTACTGTGCGAAGATTATCCGGG AGCAGTGGGCCTTTGACTACTGGGGCCAGGGAA CCCTGGTCACCGTCTCCTCA |
|---|---|---|---|---|
| | | | SEQ ID NO: 25287 | SEQ ID NO: 29293 |
| | | AA | DIQMTQSPSSVSASVGDRVTITCRASQGISNWLA WYQQKPGKAPKLLIFAASNLQSGVPSRFSGSGS GTDFTLTISSLQPADFATYCCQQVNSFPWTFGQG TKVEIK | EVQLLDSGGGLVQPGGSLRLSCAASGFTFSSYAMS WVRQAPGKGLEWVSAISGRGGNTFYAESVRGRFTI SRDNSKNTLYLQMNSLRAEDTAVYYCAKIIREQWA FDYWGQGTLVTVSS |
| | | | SEQ ID NO: 25288 | SEQ ID NO: 29294 |
| iPS:433995 | 21-225_47H7 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGAAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAG TCAGCAGCCTGCAGCCTGAAGATTTTGCAACT TATTTCTGTCTACAACATACTAGTTTCCCATTC ACTTTCGGCCCTGGGACCAAAGTGGATATCAA A | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTACAT GATCTGGATCCGCCAGGCTCCAGGGAAGGGGCT GGAGTGGGTTTCATACATTAATAGTAATGGTTTT ACCAAATACTACGCAGACTCTGTGAAGGGCCGA TTCACCATCTCCAGGGACAACGCCAAGAATTCAC TGTATCTGCAAATGAACAGTCTGAGAGCCGAGG ACACGGCCGTGTATTACTGTGCGGCCGATACAGT CTACTGGGGCCAGGGAACCCTGGTCACCGTCTCC TCA |
| | | | SEQ ID NO: 25289 | SEQ ID NO: 29295 |

FIGURE 50

| | | | | |
|---|---|---|---|---|
| | | AA | DIQMTQSPSSLSASVGDRVTITCRTSQGIRNDLG WYQKKPGKAPKRLIYAASSLQSGVPSRFSGSGS GTEFTLTVSSLQPEDFATYFCLQHTSFPFTFGPGT KVDIK | QVQLVESGGGLVKPGGSLRLSCAASGFTFSDYYMI WIRQAPGKGLEWVSYINSNGFTKYYADSVKGRFTI SRDNAKNSLYLQMNSLRAEDTAVYYCAADTVYW GQGTLVTVSS |
| | | | SEQ ID NO: 25290 | SEQ ID NO: 29296 |
| IPS:433997 | 21-225_48C1 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGCAGAAACCAGGGAAAG CCCCTAAGCGCCTGATCTATAGAGCATCCAGT TTGCAAAGTGGGGTCCCAGCAAGGTTCAGCGG CAGTGGATCTGGGACAGAATTCACTCTTACAA TCAGCAGCCTGCAGCCTGAAGATTTTGCAATT TATTACTGTCTACAGCATAATTTTTACCCGTGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTGTATGGTATGATGAAATT AATAAAAAGTATGCAGACTCCGTGAAGGGCCGA GTCACCATCTCCAGAGACAATTCCAAGAACACGC TGTATCTGCAAATGAACAGCCTGAGAGCCGAGG ACACGGCTATGTATTACTGTGCGAGAGAATTAGG GTGGGAGGCTGACTACTGGGGCCAGGGAACCCT GGTCACCGTCTCCTCA |
| | | | SEQ ID NO: 25291 | SEQ ID NO: 29297 |
| | | AA | DIQMTQSPSSLSASVGDRVTITCRTSQGIRNDLG WYQQKPGKAPKRLIYRASSLQSGVPARFSGSGS GTEFTLTISSLQPEDFAIYYCLQHNFYPWTFGQG TKVEIK | QVQLVESGGGVVQPGRSLRLSCAASGFTFSSYGMH WVRQAPGKGLEWVAVVWYDEINKKYADSVKGRV TISRDNSKNTLYLQMNSLRAEDTAMYYCARELGW EADYWGQGTLVTVSS |
| | | | SEQ ID NO: 25292 | SEQ ID NO: 29298 |

FIGURE 50

| iPS:433999 | 21-225_48D1 | NA | GACATCCAGATGACCCAGTCTCCATCCTCCCT GTCTGCGTCTGTAGGAGACAGAGTCAACATCA CTTGCCGGGCAAGTCAGAGCATTAGCAGCTAT TTAATTTGGTATCAGCAGAAACCAGGGAAAGC CCCTAAGCTCCTGATCTATGCTGCATCCAGTTT GCAAAGTGGGGTCCCATCAAGGTTCAGTGCCA GTGGATCTGGGACAGATTTCACTCTCACCATC AGCAGTCTGCAACCTGAAGATTTTGCAAGTTA CTACTGTCAACAGAGTAACAGTATTCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTAGATTCACCTTTAGCAGTTATGCCAT GAGCTGGGTCCGCCAGGCTCCAGGGAAGGGGCT GGAGTGGGTCTCAGTTATTAGTGGTCGTGGTGGT AGCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGCGTCGTATAGCA GTGGCTGGGAATGAGGCTTTTGATATCTGGGGCC AAGGGACAATGGTCACCGTCTCTTCA |
| | | | SEQ ID NO: 25293 | SEQ ID NO: 29299 |
| | | AA | DIQMTQSPSSLSASVGDRVNITCRASQSISSYLIW YQQKPGKAPKLLIYAASSLQSGVPSRFSASGSGT DFTLTISSLQPEDFASYYCQQSNSIPFTFGPGTKV DIK | EVQLLESGGGLVQPGGSLRLSCAASRFTFSSYAMS WVRQAPGKGLEWVSVISGRGGSTFYADSVKGRFTI SRDNSKNTLYLQMNSLRAEDTAVYYCARRIAVAG NEAFDIWGQGTMVTVSS |
| | | | SEQ ID NO: 25294 | SEQ ID NO: 29300 |
| iPS:434001 | 21-225_48F2 | NA | GACATCCAGATGACCCAGTCTCCATCCTCCCT GTCTGCATCTGTAGGAGACAGAGTCACCATCA CTTGCCGGGCAAGTCGGGGCATTAGAGATGAT TTAGGCTGGTATCAACAGAAACCAGGGAAAC CCCCTAAGCGCCTGATCTATGCTGCATCCAGT TTGCAAAGTGGGGTCCCATCAAGGTTCAACGG CAGTGGATCTGGGACAGAATTCACTCTCACAA TCAGCAGCCTGCAGTCTGAAGATCTTGCAACT TATTACTGTCTACAGCAATATAGTTATCCTCGG ACGTTCGGCCAAGGGACCAAGGTGGAAATCA AA | CAGGTGCAGCTGGTGGAGTCTGGGGGAGGCGTG GTCCAGCCTGGGAGGTCTCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAGCAGCAGCTGGTACGACTACGGTCTGGACGTC TGGGGCCAAGGGACCACGGTCACCGTCTCCTCA |
| | | | SEQ ID NO: 25295 | SEQ ID NO: 29301 |

FIGURE 50

| | | | | |
|---|---|---|---|---|
| | | AA | DIQMTQSPSSLSASVGDRVTITCRASRGIRDDLG WYQQKPGKPPKRLIYAASSLQSGVPSRFNGSGS GTEFTLTISSLQSEDLATYYCLQQYSYPRTFGQG TKVEIK | QVQLVESGGGVVQPGRSLRLSCAASGFTFSNYVM HWVRQAPGKGLEWVAVIWYDGSNKYYADSVKGR FTISRDNSKNTLYLQMNSLRAEDTAVYYCARERYS SSWYDYGLDVWGQGTTVTVSS |
| | | | SEQ ID NO: 25296 | SEQ ID NO: 29302 |
| iPS:434003 | 21-225_48C3 | NA | GACATCCAGATGACCCAGTCTCCATCCTCCCT GTCTGCGTCTGTAGGAGACAGAGTCAACATCA CTTGCCGGGCAAGTCAGAGCATTATCAGCTAT TTAATTTGGTATCAGCAGAAACCAGGGAAAGC CCCTAGGCTCCTGATCTATGCTGCATCCAGTTT GCAAAGTGGGGTCCCATCAAGGTTCAGTGCCA GTGGATCTGGGACAGATTTCACTCTCACCATC AGCAGTCTGCAACCTGAAGATTTTGCAAGTTA CTACTGTCAACAGACTAACAGTATTCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTAGATTCACCTTTAGCAGTTATGCCAT GAGCTGGGTCCGCCAGGCTCCAGGGAAGGGGCT GGAGTGGGTCTCAGTTATTAGTGGTCGTGGTGGT AGCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGCGTCGTATAGCA GTGGCTGGGAATGAGGCTTTTGATATCTGGGGCC AAGGGACAATGGTCACCGTCTCTTCA |
| | | | SEQ ID NO: 25297 | SEQ ID NO: 29303 |
| | | AA | DIQMTQSPSSLSASVGDRVNITCRASQSIISYLIW YQQKPGKAPRLLIYAASSLQSGVPSRFSASGSGT DFTLTISSLQPEDFASYYCQQTNSIPFTFGPGTKV DIK | EVQLLESGGGLVQPGGSLRLSCAASRFTFSSYAMS WVRQAPGKGLEWVSVISGRGGSTFYADSVKGRFTI SRDNSKNTLYLQMNSLRAEDTAVYYCARRIAVAG NEAFDIWGQGTMVTVSS |
| | | | SEQ ID NO: 25298 | SEQ ID NO: 29304 |

1793

FIGURE 50

| | | | | |
|---|---|---|---|---|
| iPS:434007 | 21-225_48D7 | NA | GACATCCAGATGACCCAGTCTCCATCTTCCGT GTCTGCATCTGTAGGAGACAGAGTCACCATCA CTTGTCGGGCGAGTCAAAATATTACCAGCTGG TTAGCCTGGTATCAGCAGAAACCAGGGAAAG CCCCTAAGCTCCTGATCTATAGTGCATCCAGTT TGCAAAATGGGGTCCCATCAAGGTTCAGCGGC AGTGGATCTGGGACAGATTTCACTCTCACCAT CAGCAGCCTGCAGCCTGAAGATTTTGTAACTT ACTGTTGTCAACAGGCTAACAGTTTCCCGTGG ACGTTCGGCCAAGGGACCAAGGTGGAAATCA AA | GAGGTGCAGCTGTTGGAGTCTGGGGGAGGCTTG GTACAGCCGGGGGGGTCCCTGAGACTCTCCTGTG CAGCCTCTGGATTCACCTTTAGAAACTCTGCCAT GAACTGGGTCCGCCAGGCTCCAGGGAAGGGGCT GGAGTGGGTCTCAGCTATTAGTGGTAGTGGTGGT ACCACATTCTACGCAGACTCCGTGAAGGGCCGGT TCACCATCTCCAGAGACAATTCCAAGAACACGTT GTATCTGCAAATAAACAGCCTGAGAGCCGAGGA CACGGCCGTATATTACTGTGCGAAATGTGGGCGG GAGCAGTGGCTTGACTACTGGGGCCAGGGAACC CTGGTCACCGTCTCCTCA |
| | | | SEQ ID NO: 25299 | SEQ ID NO: 29305 |
| | | AA | DIQMTQSPSSVSASVGDRVTITCRASQNITSWLA WYQQKPGKAPKLLIYSASSLQNGVPSRFSGSGS GTDFTLTISSLQPEDFVTYCCQQANSFPWTFGQG TKVEIK | EVQLLESGGGLVQPGGSLRLSCAASGFTFRNSAMN WVRQAPGKGLEWVSAISGSGGTTFYADSVKGRFTI SRDNSKNTLYLQINSLRAEDTAVYYCAKCGREQW LDYWGQGTLVTVSS |
| | | | SEQ ID NO: 25300 | SEQ ID NO: 29306 |
| iPS:434009 | 21-225_48A9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATATTGCATCCAGTT TGCAAAGTGGGGTCCCATCAAGGTTCAGCGGC AGTGGATCTGGGACAGAATTCACTCTCACTAT CAGCGGCTTGCAGCCTGAAGATTTTGCAATTT ATTACTGTCTACAGCATAATCGTTACCCGTGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCCCCAGGCAAGGGGCT GGAGTGGGTGGCAGTTATATGGTATGAGGAAAA TAAGAAATACTATGCAGACTCCGTGAAGGGCCG ATTCACCATCTCCAGAGACAATTCCAAGAACACG CTGTTTCTGCAAATGAATAGCCTGAGAGCCGAGG ACACGGCTATGTATTTCTGTGCGAGAGAACTTGC CTGGTACGAGGACTACTGGGGCCAGGGAACCCT GGTCACCGTCTCCTCA |
| | | | SEQ ID NO: 25301 | SEQ ID NO: 29307 |

FIGURE 50

| | | | | |
|---|---|---|---|---|
| | | AA | DIQMTQSPSSLSASVGDRVTITCRASQGIRNDLG WYQQKPGKAPKRLIYIASSLQSGVPSRFSGSGSG TEFTLTISGLQPEDFAIYYCLQHNRYPWTFGQGT KVEIK | QVQLVESGGGVVQPGRSLRLSCAASGFTFSSYGMH WVRQAPGKGLEWVAVIWYEENKKYYADSVKGRF TISRDNSKNTLFLQMNSLRAEDTAMYFCARELAWY EDYWGQGTLVTVSS |
| | | | SEQ ID NO: 25302 | SEQ ID NO: 29308 |
| iPS:434011 | 21-225_48B10 | NA | GACATCCAGATGACCCAGTCTCCATCCTCCCT GTCTGCGTCTGTAGGAGACAGAGTCACCATCA CTTGCCGGGCAAGTCAGAGCATTAGGAAGTAT TTAAATTGGTATCAGAAGACACCAGGGAAAG CCCCTAAACTCTTGATCTATGCTGCTTCCAGTT TGCAAAGTGGGGTCCCATCAAGGTTCAGTGGC AGTGGATCTGGGACAGATTTCACTCTCACCAT CAGCAGTGTGCAACCTGAAGATTTTGCAACTT ACTACTGTCAACAGACTTACAGTAACCCACTC ACTTTCGGCGGAGGGACCAAGGTGGAGTTCAC A | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTTCAT GACCTGGATCCGCCAGGCTCCAGGGCAGGGGCT GGAGTGGGTTTCATACATTAGTAGTGCTGGTGGT GCCATATACTACGCAGACTCTGTGAAGGGCCGAT TCACCATCTCCAGGGACAACGCCAAAAACTCACT GTATCTGCAAATGAACAGCCTGAGAGCCGAGGA CACGGCCGTGTATTACTGTGCAATAGCAGTGGCT GCCCCTGGTGTTTTTGATATCTGGGGCCAAGGGA CAATGGTCACCGTCTCTTCA |
| | | | SEQ ID NO: 25303 | SEQ ID NO: 29309 |
| | | AA | DIQMTQSPSSLSASVGDRVTITCRASQSIRKYLN WYQKTPGKAPKLLIYAASSLQSGVPSRFSGSGSG TDFTLTISSVQPEDFATYYCQQTYSNPLTFGGGT KVEFT | QVQLVESGGGLVKPGGSLRLSCAASGFTFSDYFMT WIRQAPGQGLEWVSYISSAGGAIYYADSVKGRFTIS RDNAKNSLYLQMNSLRAEDTAVYYCAIAVAAPGV FDIWGQGTMVTVSS |
| | | | SEQ ID NO: 25304 | SEQ ID NO: 29310 |

FIGURE 50

| iPS:434013 | 21-225_48D12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCACTT TGCAAAGTGGGGTCCCATCAAGGATCAGCGGC AGTGGATCTGGGACAGAATTCACTCTCACAAT CAGCAGCCTGCAGCCTGAAGATTTTGCAACTT ATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAGGGGCCT GGAGTGGGTGGCAGTTATATGGTATGATGTAAGT AATAAATACTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG ATGAGATCTGACTACTGGGGCCAGGGAACCCTG GTCACCGTCTCCTCA |
| | | | SEQ ID NO: 25305 | SEQ ID NO: 29311 |
| | | AA | DIQMTQSPSSLSASVGDRVTITCRASQGIRNDLG WYQQKPGKAPKRLIYAASTLQSGVPSRISGSGSG TEFTLTISSLQPEDFATYYCLQHNSYPLTFGGGT KVEIK | QVQLVESGGGVVQPGRSLRLSCAASGFTFSDYGM HWVRQAPGRGLEWVAVIWYDVSNKYYVDSVKGR FTISRDNSKNTLYLQMNSLRAEDTAVYYCARELGM RSDYWGQGTLVTVSS |
| | | | SEQ ID NO: 25306 | SEQ ID NO: 29312 |
| iPS:434015 | 21-225_48F12 | NA | GACATCCAGATGACCCAGTCTCCATCCTCCCT GTCTGCGTCTGTAGGAGACAGAGTCACCATCA CTTGCCGGGCAAGTCAGAGCATTAGGAAGTAT TTAAATTGGTATCAGAAGACACCAGGGAAAG CCCCTAAACTCTTGATCTATGCTGCTTCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAAATT ATTACTGTCAACAGACTTACAGTAACCCGCTC ACTTTCGGCGGAGGGACCGAGGTGGAGATCA CA | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTTCAT GACCTGGATCCGCCAGGCTCCAGGGCAGGGGCT GGAGTGGGTTTCATACATTAGTAGTGCTGGTGGT GCCATATACTACGCAGACTCTGTGAAGGGCCGAT TCACCATCTCCAGGGACAACGCCAAGAACTCACT GTTTCTGCAAATGAACAGCCTGAGAGCCGAGGA CACGGCCGTGTATTACTGTGCTATAGCAGTGGCT GCCCCTGGTGCTTTTGATATCTGGGGCCAAGGGA CATTGGTCACCGTCTCTTCA |
| | | | SEQ ID NO: 25307 | SEQ ID NO: 29313 |

FIGURE 50

|  |  |  | DIQMTQSPSSLSASVGDRVTITCRASQSIRKYLN WYQKTPGKAPKLLIYAASSLQSGVPSRFSGSGSG TDFTLTISSLQPEDFANYYCQQTYSNPLTFGGGT EVEIT | QVQLVESGGGLVKPGGSLRLSCAASGFTFSDYFMT WIRQAPGQGLEWVSYISSAGGAIYYADSVKGRFTIS RDNAKNSLFLQMNSLRAEDTAVYYCAIAVAAPGA FDIWGQGTLVTVSS |
| :--- | :--- | :--- | :--- | :--- |
|  |  | AA | SEQ ID NO: 25308 | SEQ ID NO: 29314 |
| iPS:434017 | 21-225_48G12 | NA | GACATCCAGATGACCCAGTCTCCATCCTCCCT GTCTGCGTCTGTAGGAGACAGAGTCACCATCA CTTGCCGGGCAAGTCAGAGCATTAGGAAGTAT TTAAATTGGTATCAGAAGACACCAGGGAAAG CCCCTAAACTCTTGATATATGCTGCTTCCAGTT TGCAAAGTGGGGTCCCATCAAGGTTCAGTGGC AGTGGATCTGGGACAGATTTCACTCTCACCAT CAGCAGTGTGCAACCTGAAGATTTTGCAAATT ATTACTGTCAACAGACTTACAGTAACCCGCTC ACTTTCGGCGGAGGGACCGAGGTGGAGATCA CA | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTTCAT GACCTGGATCCGCCAGGCTCCAGGGCAGGGGCT GGAGTGGGTTTCATACATTAGTAGTGCTGGTGGT GCCATATACTACGCAGACTCTGTGAAGGGCCGAT TCACCATCTCCAGGGACAACGCCAAGAACTCACT GTTTCTGCAAATGAACAGCCTGAGAGCCGAGGA CACGGCCGTGTATTACTGTGCTATAGCAGTGGCT GCCCCTGGTGCTTTTGATATCTGGGGCCAAGGGA CATTGGTCACCGTCTCTTCA |
|  |  |  | SEQ ID NO: 25309 | SEQ ID NO: 29315 |
|  |  | AA | DIQMTQSPSSLSASVGDRVTITCRASQSIRKYLN WYQKTPGKAPKLLIYAASSLQSGVPSRFSGSGSG TDFTLTISSVQPEDFANYYCQQTYSNPLTFGGGT EVEIT | QVQLVESGGGLVKPGGSLRLSCAASGFTFSDYFMT WIRQAPGQGLEWVSYISSAGGAIYYADSVKGRFTIS RDNAKNSLFLQMNSLRAEDTAVYYCAIAVAAPGA FDIWGQGTLVTVSS |
|  |  |  | SEQ ID NO: 25310 | SEQ ID NO: 29316 |

FIGURE 50

| iPS:434019 | 21-225_49A1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGACTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGTAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAGAT AATAAATATTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCTCTCTGACTACTGGGGCCAGGGAACCCTGG TCACCGTCTCCTCA |
|  |  |  | SEQ ID NO: 25311 | SEQ ID NO: 29317 |
|  |  | AA | DIQMTQSPSSLSASVGDRVTITCRASQGIRNDLD WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYDEDNKYYVDSVKGR FTISRDNSKNTLYLQMNSLRAEDTAVYYCARELGF LSDYWGQGTLVTVSS |
|  |  |  | SEQ ID NO: 25312 | SEQ ID NO: 29318 |
| iPS:434021 | 21-225_49C1 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGG GAAGGAAAGACCTATTTGTACTGGTACCTGCA GAAGCCAGGCCAGGCTCCACAGTTCCTGATCT TTGAAGTTTCCCACCGGTTCTCTGGCGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGATTCCGATCACCCTCGGCCAAGGGA CACGACTGGAGATTAAA | CAGGTGCAGCTGGTGGAGTCTGGGGGAGGCGTG GTCCAGCCTGGGAGGTCCCTGAGACTCTCCTGTA CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCGGGCGGTATGGACGTCTGGGG CCAAGGGACCACGGTCACCGTCTCCTCA |
|  |  |  | SEQ ID NO: 25313 | SEQ ID NO: 29319 |

FIGURE 50

| | | | | |
|---|---|---|---|---|
| | | AA | DIVMTQTPLSLSVTPGQPASISCKSSQSLLHREGK TYLYWYLQKPGQAPQFLIFEVSHRFSGVPDRFSG SGSGTDFTLKISRVEAEDVGVYYCMQSIQIPITLG QGTRLEIK | QVQLVESGGGVVQPGRSLRLSCTASGFTFSSYGMH WVRQAPGKGLEWVAVIWYDGSNKYYADSVKGRF TISRDNSKNTLYLQMNSLRAEDTAVYYCARRYSSS WSGGMDVWGQGTTVTVSS |
| | | | SEQ ID NO: 25314 | SEQ ID NO: 29320 |
| iPS:434023 | 21-225_49F1 | NA | GACATCCAGATGACCCAGTCTCCATCTTCCGT GTCTGCATCTGTAGGAGACAGAGTCACCATCA CTTGTCGGGCGAGTCGGGATATTAACGGCTGG TTAGCCTGGTATCAGCAGAAACCAGGGAAAG CCCCTAAGCTCCTGATCTATACTGTCTCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGTCTAACAGTTTCCCATTCA CTTTCGGCCCTGGGACCAAAGTGGATATCAAA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGACTGGGTCTCAGTTATTAGTGGTAGTGGTGGT AGCACATTCTACGCAGACTCCGTGAAGGGCCGGT TCACCATCTCCAGAGACAATTCCAAGAGCACGCT GTATCTGCAAATGAACAGCCTGAGAGCCGAGGA CACGGCCGTATATTATTGTGCGAGCGCTATAGCA GCGGCTGGTGCCCACTATTTTGACTACTGGGGCC AGGGAACCCTGGTCACCGTCTCCTCA |
| | | | SEQ ID NO: 25315 | SEQ ID NO: 29321 |
| | | AA | DIQMTQSPSSVSASVGDRVTITCRASRDINGWLA WYQQKPGKAPKLLIYTVSSLQSGVPSRFSGSGSG TDFTLTISSLQPEDFATYYCQQSNSFPFTFGPGTK VDIK | EVQLLESGGGLVQPGGSLRLSCAASGFTFSSYAMS WVRQAPGKGLDWVSVISGSGGSTFYADSVKGRFTI SRDNSKSTLYLQMNSLRAEDTAVYYCASAIAAAGA HYFDYWGQGTLVTVSS |
| | | | SEQ ID NO: 25316 | SEQ ID NO: 29322 |

FIGURE 50

| iPS:434025 | 21-225_49G3 | NA | GATATTGTGATGACCCAGACTCCACTCTCTCT GTCCGTCACCCCTGGACAGCCGGCCTCCATGT CCTGCAAGTCTAGTCAGAGCCTCCTGCATAGT GAAGGAAAGACCTATTTGTATTGGTACCTGCA GAAGACAGGCCAGCCTCCACACCTCCTGATCT ATGAAGTTTCCAACCGGCTCTCTGGCGTGCCA GATAGGTTCAGTGGCAGCGGGTCAGGGACAG ATTTCACACTGAAAATCAGCCGGATGGAGGCT GAGGATGTTGGGGTTTATTTCTGCATGCAAAG TATGCAGCTTCCGATCACCTTCGGCCAGGGGA CACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCGGGCGGAATGGACGTCTGGGG CCAAGGGACCACGGTCACCGTCTCCTCA |
| | | | SEQ ID NO: 25317 | SEQ ID NO: 29323 |
| | | AA | DIVMTQTPLSLSVTPGQPASMSCKSSQSLLHSEG KTYLYWYLQKTGQPPHLLIYEVSNRLSGVPDRF SGSGSGTDFTLKISRMEAEDVGVYFCMQSMQLP ITFGQGTRLEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WSGGMDVWGQGTTVTVSS |
| | | | SEQ ID NO: 25318 | SEQ ID NO: 29324 |
| iPS:434027 | 21-225_49H5 | NA | GACATCCAGATGACCCAGTCTCCATCTTCCGT GTCTGCATCTGTAGGAGACAGAGTCACCATCA CTTGTCGGGCGAGTCAGGGTTTTAGCACCTGG TTAGCCTGGTTTCAGCAGAAACCAGGGAAAGC CCCTAAGCTCCTGATCTATGCTGCATCCAGTTT GCAAGATGGGGTCCCATCAAGGTTCAGCGGCA GTGGATCTGGGACAGATTTCACTCTCACCATC AGCAGCCTGCAGCCTGAAGATTTTGCAACTTA CTATTGTCAACAGACTAACAGTTTCCCGTTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GACCTGGGTCCGCCAGACTCCAGGGAAGGGGCT GGAGTGGGTCTCAGCTATTAGTGGTAGTGGTGGT AACTCATTCTACGCAGACTCCGTGAAGGGCCGGT TCACCATCTCCAGAGACAATTCCGAGAACACGTT GTATCTGCAAATGAACAGCCTGAGAGCCGAGGA CACGGCCGTATATTACTGTGCGAAAGCAAGGGC AGTGGCTGGGTCACACTGGTTCGACCCCTGGGGC CAGGGAACCCTGGTCACCGTCTCCTCA |
| | | | SEQ ID NO: 25319 | SEQ ID NO: 29325 |

FIGURE 50

| | | | | |
|---|---|---|---|---|
| | | AA | DIQMTQSPSSVSASVGDRVTITCRASQGFSTWLA WFQQKPGKAPKLLIYAASSLQDGVPSRFSGSGS GTDFTLTISSLQPEDFATYYCQQTNSFPFTFGPGT KVDIK | EVQLLESGGGLVQPGGSLRLSCAASGFTFSSYAMT WVRQTPGKGLEWVSAISGSGGNSFYADSVKGRFTI SRDNSENTLYLQMNSLRAEDTAVYYCAKARAVAG SHWFDPWGQGTLVTVSS |
| | | | SEQ ID NO: 25320 | SEQ ID NO: 29326 |
| IPS:434029 | 21-225_49C6 | NA | GACATCCAGATGACCCAGTCTCCATCCTCCCT GTCTGCATCTGTCGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGTTTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCTCTC ACTTTCGGCGGAGGGACCAAGGTGGAGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTTTGATGTAAGT AATAAAAAGTATGTAGACTCCGTGAAGGGCCGA TTCACCATCTCCAGAGACAATTCCAAGAACACGC TGTATCTGCAAATGAGCAGCCTGAGAGCCGAGG ACACGGCTGTGTATTATTGTGCGAGAGATCTGGG GATGATCGAGGACTACTGGGGCCAGGGAACCCT GGTCACCGTCTCCTCA |
| | | | SEQ ID NO: 25321 | SEQ ID NO: 29327 |
| | | AA | DIQMTQSPSSLSASVGDRVTITCRASQGIRNDLG WYQQKPGKAPKRLIYAASSLQSGFPSRFSGSGSG TEFTLTISSLQPEDFATYYCLQHNSYPLTFGGGT KVEIK | QVQLVESGGGVVQPGRSLRLSCAASGFTFSNYGM HWVRQAPGKGLEWVAVIWFDVSNKKYVDSVKGR FTISRDNSKNTLYLQMSSLRAEDTAVYYCARDLGM IEDYWGQGTLVTVSS |
| | | | SEQ ID NO: 25322 | SEQ ID NO: 29328 |

FIGURE 50

| iPS:434031 | 21-225_49E7 | NA | GATATTGTGATGACCCAGACTCCACTCTCTCT GTCTGTCACCCCTGGACAGCCGGCCTTCATGT CCTGCAAGTCTAGTCAGATCTTCTTGCATAGT GAAGGAAAGACCTATTTGTATTGGTACCTGCA GAAGACAGGCCAGCCTCCACACCTCCTGATCT ATGAAGTTTCCAAGCGGCTCTCTGGCGTGCCA GATAGGTTCAGTGGCAGCGGGTCAGGGACAG ATTTCACACTGAAAATCAGCCGGATGGAGGCT GAGGATGTCGGGGGTTTATTACTGCATGCAAAG TATGCAGCTTCCGATTATCTTCGGCCAGGGGA CACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTCTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 25323 | SEQ ID NO: 29329 |
| | | AA | DIVMTQTPLSLSVTPGQPAFMSCKSSQIFLHSEG KTYLYWYLQKTGQPPHLLIYEVSKRLSGVPDRF SGSGSGTDFTLKISRMEAEDVGVYYCMQSMQLP IIFGQGTRLEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTALYYCARRYSSS WSGGMDVWGQGTTVTVSS |
| | | | SEQ ID NO: 25324 | SEQ ID NO: 29330 |
| iPS:434033 | 21-225_49F9 | NA | GATATTGTGATGACCCAGACTCCACTCTCTCT GTCCGTCACCCCTGGACAGCCGGCCTCCATCT CCTGCAAGTCTAATCAGAGCCTCGTGCATAAT GAAGGAAAGACCTATTTGTATTGGTATTTGCA GAAGCCAGGCCAGCCTCCACAGCTCCTGATCT TTGAAGTTTCCAACCGGTTCTCTGGAGTGCCA GATAGGTTCAGTGGCAGCGGGTCAGGGACAG ATTTCACACTGAAAATCAGTCGGGTGGAGGCT GAGGATGTTGGGGTTTATTACTGCATGCAAAG TATACAGTATCCGATCACCTTCGGCCAAGGGA CACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCACTTATATGGTATGATGGAAG GAATAAATACTATGCAGACTCCGTGAAGGGCCG ATTCACCATCTCCAGAGACAATCCCAAGAACACG CTGTATCTGCAAATGAACAGCCTGAGAGCCGAG GACACGGCTGTGTATCACTGTGCGAGAAGGTATA GCAGCAGCTGGTCGGGCGGTATGGACGTCTGGG GCCAAGGGACCACGGTCACCGTCTCCTCA |
| | | | SEQ ID NO: 25325 | SEQ ID NO: 29331 |

FIGURE 50

| | | | | |
|---|---|---|---|---|
| | | AA | DIVMTQTPLSLSVTPGQPASISCKSNQSLVHNEG KTYLYWYLQKPGQPPQLLIFEVSNRFSGVPDRFS GSGSGTDFTLKISRVEAEDVGVYYCMQSIQYPIT FGQGTRLEIK | QVQLVESGGGVVQPGRSLRLSCAASGFTFSSYGMH WVRQAPGKGLEWVALIWYDGRNKYYADSVKGRF TISRDNPKNTLYLQMNSLRAEDTAVYHCARRYSSS WSGGMDVWGQGTTVTVSS |
| | | | SEQ ID NO: 25326 | SEQ ID NO: 29332 |
| IPS:434035 | 21-225_49F10 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAAGGTCCTGATCTATGCTGCATCCACTT TGCAAAGTGGGGTCCCATCAAGGTTCAGCGGC AGTGGATCTGGGACAGATTTCACTCTCACCAT CAGCAGCCTGCAACCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACCATA TGCACTGGGTGCGACAGGCCCCTGGACAAGGGC TTGAGTGGATGGGATGGATCAACCCTAATAACAA TGCCACAAACTATGCTCAGAACTTTCAGGGCAGG GTCACCCTGACCAGGGACACGTCCATCAGCACA GCCTACATGGAGCTGAGCAGGCTGAGATCTGAC GACACGGCCGTGTATTACTGTGCGAGAGACGGT ACCAGCAGCTTTGACTTCTGGGGGCCAGGGAACCC TGGTCACCGTCTCCTCA |
| | | | SEQ ID NO: 25327 | SEQ ID NO: 29333 |
| | | AA | DIQMTQSPSSVSASVGDRVTITCRASQGISRWLA WYQQKPGKAPKVLIYAASTLQSGVPSRFSGSGS GTDFTLTISSLQPEDFATYYCQQANSFPFTFGPGT KVDIK | QVQLVQSGAEVKKPGASVKVSCKASGYTFTGYHM HWVRQAPGQGLEWMGWINPNNNATNYAQNFQGR VTLTRDTSISTAYMELSRLRSDDTAVYYCARDGTSS FDFWGQGTLVTVSS |
| | | | SEQ ID NO: 25328 | SEQ ID NO: 29334 |

1803

FIGURE 50

| iPS:434037 | 21-225_49G12 | NA | GACATCCAGATGACCCAGTCTCCATCCTCCCT GTCTGCGTCTGTAGGAGACAGAGTCACCATCA CTTGCCGGTCAAGTCAGAGCATTAGTACCTAT TTAATGTGGTATCAGCAGAAACCAGGGAAAG CCCCTAAGCTCTTGATCTATGCTGCATCCAGTT TGCAAATTGGGGTCCCATCAGAGTTCAGTGCC AGTGGATCTGGGACAGATTTCACTCTCACCAT CAGCAGTCTGCAACCTGAAGATTTTGCAACTT ACTACTGTCAACAGAGTTACAGTATCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTA CAGCCTCTGGATTCACCTTTAGCAGCTATGCCAT GAGCTGGGTCCGCCAGGCTCCAGGGAAGGGGCT GGAGTGGGTCTCAGTTATTAGTGGTAGTGGTGGT ACCACATTCTACGCAGACTCCGTGAAGGGCCGGC TCACCATCTCCAGAGACAATTCCAAGAACACACT GTATCTGCAAATGAACAGCCTGAGAGCCGAGGA CACGGCCGTATATTACTGTGCGCGTCGTATAGCA GTGGCTGGGAATGATGCTTTTGATATCTGGGGCC AAGGGACAATGGTCACCGTCTCTTCA |
| | | | SEQ ID NO: 25329 | SEQ ID NO: 29335 |
| | | AA | DIQMTQSPSSLSASVGDRVTITCRSSQSISTYLM WYQQKPGKAPKLLIYAASSLQIGVPSEFSASGSG TDFTLTISSLQPEDFATYYCQQSYSIPFTFGPGTK VDIK | EVQLLESGGGLVQPGGSLRLSCTASGFTFSSYAMS WVRQAPGKGLEWVSVISGSGGTTFYADSVKGRLTI SRDNSKNTLYLQMNSLRAEDTAVYYCARRIAVAG NDAFDIWGQGTMVTVSS |
| | | | SEQ ID NO: 25330 | SEQ ID NO: 29336 |
| iPS:434039 | 21-225_43B1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAG TCAGCAGCCTGCAGCCTGAAGATTTTGCAACT TATTTCTGTCTACAGCATACTAGTTTCCCATTC ACTTTCGGCCCTGGGACCAAAGTGGATATCAA A | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTACAT GAACTGGATCCGCCAGGCTCCAGGGAAGGGGCT GGAGTGGGTTTCATACATTAATAGTAATGGTTTT ACCATATACTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGGCCGATACAATC TACTGGGGCCAGGGAACCCGGGTCACCGTCTCCT CA |
| | | | SEQ ID NO: 25331 | SEQ ID NO: 29337 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTVSSLQPEDFATYFCLQHTSFPFTFGPGT KVDIK | QVQLVESGGGLVKPGGSLRLSCAASGFTFSDYYMN WIRQAPGKGLEWVSYINSNGFTIYYADSVKGRFTIS RDNAKNSLYLQMNSLRAEDTAVYYCAADTIYWGQ GTRVTVSS |
| | | | SEQ ID NO: 25332 | SEQ ID NO: 29338 |
| iPS:434041 | 21-225_50H8 | NA | GACATCCAGATGACCCAGTCTCCATCCTCCCT GTCTGCGTCTGTAGGAGACAGAGTCAACATCA CTTGCCGGGCAAGTCAGAGCATTAGCAGCTAT TTAATTTGGTATCAGCAGAAACCAGGGAAAGC CCCTAAGCTCCTGATCTATGCTGCATCCAGTCT GCAAAGTGGGGTCCCATCAAGGTTCAGTGCCA GTGGATCTGGGACAGATTTCACTCTCACCATC AGCAGTCTGCAACCTGAAGATTTTGCAAGTTA CTACTGTCAACAGAGTAACAGTCTTCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTAGATTCACCTTTAGCAGTTATGCCAT GAGCTGGGTCCGCCAGGCTCCAGGGAAGGGGCT GGAGTGGGTCTCAGTTATTAGTGGTCGTGGTGGT ACCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGCGTCGTATAGCA GTGGCTGGGAATGAGGCTTTTGATATCTGGGGCC AAGGGACAATGGGTCACCGTCTCTTCA |
| | | | SEQ ID NO: 25333 | SEQ ID NO: 29339 |
| | | AA | DIQMTQSPSSLSASVGDRVNITCRASQSISSYLIW YQQKPGKAPKLLIYAASSLQSGVPSRFSASGSGT DFTLTISSLQPEDFASYYCQQSNSLPFTFGPGTKV DIK | EVQLLESGGGLVQPGGSLRLSCAASRFTFSSYAMS WVRQAPGKGLEWVSVISGRGGTTFYADSVKGRFTI SRDNSKNTLYLQMNSLRAEDTAVYYCARRIAVAG NEAFDIWGQGTMVTVSS |
| | | | SEQ ID NO: 25334 | SEQ ID NO: 29340 |

FIGURE 50

| iPS:434043 | 21-225_50G10 | NA | GACATCCAGATGACCCAGTCTCCATCCTCCCT GTCTGCATCTGTAGGAGACAGAGTCACCATCA CTTGCCGGGCAAGTCAGGGCATTAGAAATAAT TTAGGCTGGTATCAGCAGAAACCAGGGAAAG TCCCTAAGCGCCTGATCTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGTATAATAGTTACCCGTTCA CTTTCGGCGGAGGGACCAAGGTGGAGAGCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCTGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTAGCAACT TTTGACTACTGGGGCCAGGGAACCCTGGTCACCG TCTCCTCA |
|---|---|---|---|---|
| | | | SEQ ID NO: 25335 | SEQ ID NO: 29341 |
| | | AA | DIQMTQSPSSLSASVGDRVTITCRASQGIRNNLG WYQQKPGKVPKRLIYAASSLQSGVPSRFSGSGS GTEFTLTISSLQPEDFATYYCLQYNSYPFTFGGGT KVESK | EVQLVESGGGLVKPGGSLRLSCAASGFTFSSYSMN WVRQAPGKGLEWVSSISGSSSYTYYADSVKGRFTIS RDNAKNSLYLQMNSLRAEDTAVYYCARVATFDY WGQGTLVTVSS |
| | | | SEQ ID NO: 25336 | SEQ ID NO: 29342 |
| iPS:434045 | 21-225_50H10 | NA | GACATCCAGATGACCCAGTCTCCATCCTCCCT GTCTGCGTCTGTAGGAGACAGAGTCAACATCA CTTGCCGGGCAAGTCAGAGCATTTACAGCTAT TTAATTTGGTATCAGCAGAAACCAGGGAAAGC CCCTAAGCTCCTGATCTATGCTGCATCCAGTTT GCAAAGTGGGGTCCCATCAAGGTTCAGTGCCA GTGGATCTGGGACAGATTTCACTCTCACCATC AGCAGTCTGCAACCTGAAGATTTTGCAAGTTA CTACTGTCAACAGAGTAACAGTATTCCATTCA CTTTCGGCCCTGGGACCAAAGTGGATATCAAA | GAGGGGCAGTTGTTGGAGTCTGGGGGAGGCTTG GTACAGCCTGGGGGGTCCCTGAGACTCTCCTGTG CAGCCTCTAGATTCACCTTTAGCAGTTATGCCAT GAGCTGGGTCCGCCAGGCTCCAGGGAAGGGGCT GGAGTGGGTCTCAGTTATTAGTGGTCGTGGTGGT AGCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGCGTCGTATAGCA GTGGCTGGGAATGAGGCTTTTGATATCTGGGGCC AAGGGACAATGGTCACCGTCTCTTCA |
| | | | SEQ ID NO: 25337 | SEQ ID NO: 29343 |

FIGURE 50

| | | | | |
|---|---|---|---|---|
| | | AA | DIQMTQSPSSLSASVGDRVNITCRASQSIYSYLIW YQQKPGKAPKLLIYAASSLQSGVPSRFSASGSGT DFTLTISSLQPEDFASYYCQQSNSIPFTFGPGTKV DIK | EGQLLESGGGLVQPGGSLRLSCAASRFTFSSYAMS WVRQAPGKGLEWVSVISGRGGSTFYADSVKGRFTI SRDNSKNTLYLQMNSLRAEDTAVYYCARRIAVAG NEAFDIWGQGTMVTVSS |
| | | | SEQ ID NO: 25338 | SEQ ID NO: 29344 |
| iPS:434047 | 21-225_50A12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAAT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAACTGGTGCAGTCTGGGGGCTGAGGTG AAGAAGCCTGGGGCCTCAGTGAAGGTCTCCTGC AAGGCTTCTGGATACACCTTCACCGGCCACTATA TAAACTGGGTGCGACAGGCCCCTGGACAAGGGC CTGAGTGGATGGCATGGGTCAACCCTAACAGTG GTGGCACAAACTCTGCACAGAAGTTTCAGGGCA GGGTCACCATGACCAGGGACACGTCCATCAGCA CAGTCTACATGGAGCTGAGCAGGCTGAGATCTG ACGACACGGCCGTGTATTACTGTGCGAGGGGGAG GGCAGCTCGGCGGGTTTAACTACTACTACTACGG TATGGACGTCTGGGGCCAAGGGACCACGGTCAC CGTCTCCTCA |
| | | | SEQ ID NO: 25339 | SEQ ID NO: 29345 |
| | | AA | DIQMTQSPSSLSASVGDRVTITCRASQGIRNDLG WYQQKPGKAPKRLIYTASNLQSGVPSRFSGSGS GTEFTLTISSLQPEDFATYYCLQHNSYPWTFGQG TKVEIK | QVQLVQSGAEVKKPGASVKVSCKASGYTFTGHYIN WVRQAPGQGPEWMAWVNPNSGGTNSAQKFQGRV TMTRDTSISTVYMELSRLRSDDTAVYYCARGGQLG GFNYYYYGMDVWGQGTTVTVSS |
| | | | SEQ ID NO: 25340 | SEQ ID NO: 29346 |

FIGURE 50

| iPS:434049 | 21-225_50B12 | NA | GACATCCAGATGACCCAGTCTCCATCCTCCCT GTCTGCATCTGTGGGAGACAGAGTCACCATCA CTTGTCGGGCAAGTCAGAGCATTAGTAGTTAT TTAAAATTGGTATCAGCATAAACCAGGGAAAGC CCCTAGGCTCCTGATCTATGCTGCATCCAGTTT GCAAAGTGGGGTCCCATCAAGGTTCAGTGGCA GTGAATCTGGGACAGATTTCATTCTCACTATC AGCAGTCTGCAACCTGAAGATTTTACAACTTA TTACTGTCAACAGAGTTACATTGCCCCATTCA CTTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCCATAGCAT GAACTGGGTCCGCCAGGCTCCAGGGAAGGGGCT GGAGTGGGTCTCATCCATCAGTAGTAGTAGTAAT TACATATACTACGCAGACTCAGTGAAGGGCCGAT TCACCATCTCCAGAGACTACGCCAAGAACTCACT TTATCTGCAAATGAACAGCCTGAGAGCCGAGGA CACGGCTGTGTATTACTGTGCGAGAGATCGGAGC ATAGTAGTGGCTGGTCCCTGGGACTACTACGGTA TGGACGTCTGGGGCCAAGGGACCACGGTCACCG TCTCCTCA |
|---|---|---|---|---|
| | | | SEQ ID NO: 25341 | SEQ ID NO: 29347 |
| | | AA | DIQMTQSPSSLSASVGDRVTITCRASQSISSYLNW YQHKPGKAPRLLIYAASSLQSGVPSRFSGSESGT DFILTISSLQPEDFTTYYCQQSYIAPFTFGPGTKV DIK | EVQLVESGGGLVKPGGSLRLSCAASGFTFSSHSMN WVRQAPGKGLEWVSSISSSSNYIYYADSVKGRFTIS RDYAKNSLYLQMNSLRAEDTAVYYCARDRSIVVA GPWDYYGMDVWGQGTTVTVSS |
| | | | SEQ ID NO: 25342 | SEQ ID NO: 29348 |
| iPS:434053 | 21-225_51E1 | NA | GATATTGTGATGACCCAGACTCCACTCTCTCT GTCCGTCACCCCTGGACAGCCGGCCTCCATGT CCTGCAAGTCTAGTCAGAGCCTCCTGCATAGT GAGGGAAAGACCTATTTGTATTGGTACCTGCG GAAGCCAGGCCAGCCTCCACAGTTCCTGATCT TTGAAGTTTCCAACCGGTTCTCTGGAGTGCCA GATAGGTTCAGTGGCAGCGGGTCAGGGACAG AATTCACACTGAAAATCAGCCGGGTGGAGGCT GAGGATGTTGGGGTTTATTACTGCATGCAAAG TATACAGCTTCCATTCACTTTCGGCCCTGGGAC CAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AGTAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGTTGGTCGGGCGGTATGGACGTCTGGGG CCAAGGGACCACGGTCACCGTCTCCTCA |
| | | | SEQ ID NO: 25343 | SEQ ID NO: 29349 |

FIGURE 50

| | | | | |
|---|---|---|---|---|
| | | AA | DIVMTQTPLSLSVTPGQPASMSCKSSQSLLHSEG KTYLYWYLRKPGQPPQFLIFEVSNRFSGVPDRFS GSGSGTEFTLKISRVEAEDVGVYYCMQSIQLPFT FGPGTKVDIK | QVQLVESGGGVVQPGRSLRLSCAASGFTFSSYGMH WVRQAPGKGLEWVAVIWYDGSSKYYADSVKGRF TISRDNSKNTLYLQMNSLRAEDTAVYYCARRYSSS WSGGMDVWGQGTTVTVSS |
| | | | SEQ ID NO: 25344 | SEQ ID NO: 29350 |
| iPS:434055 | 21-225_51B4 | NA | GACATCCAGATGACCCAGTCTCCATCCTCCCT GTCTGCATCTGTAGGAGACAGAGTCACCATCA CTTGCCAGGCGAGTCGGGACATTACCTTCTAT TTAAAATTGGTATCAGCAGAAACCAGGGAAAG CCCCTAAGCTCCTGATCTACGATGCATCCAAT TTGGAAACAGGGGTCCCATCAAGGTTCAGTGG CAGTGGATCTGGGACAGATTTTACTTTCACCA TCAGCTGCGTGCATCCTGAAGATATTGCAACA TATTTATGTCAACAGTATGATAATCTTCCATTC ACTTTCGGCCCAGGGACCACAGTGGATATCAA A | GAGGTGCAGCTGTTGGAGTCTGGGGGAGGCTTG GTACAGCCTGGGGGGTCCCTGAGTCTCTCCTGTG CAGCCTCTGGATTCACCTTTAGAAGCTATGTCAT GAGCTGGGTCCGCCAGGCTCCAGGGAAGGGGCT GGAGTGGGTCTCAGCTATTAGTGGTCGTGGTAGT AACACATTCTACACAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAAGGGATAACT GGATCACACGGTGCTTTTGATATCTGGGGCCAAG GGACAATGGTCACCGTCTCTTCA |
| | | | SEQ ID NO: 25345 | SEQ ID NO: 29351 |
| | | AA | DIQMTQSPSSLSASVGDRVTITCQASRDITFYLN WYQQKPGKAPKLLIYDASNLETGVPSRFSGSGS GTDFTFTISCVHPEDIATYLCQQYDNLPFTFGPGT TVDIK | EVQLLESGGGLVQPGGSLSLSCAASGFTFRSYVMS WVRQAPGKGLEWVSAISGRGSNTFYTDSVKGRFTI SRDNSKNTLYLQMNSLRAEDTAVYYCAKGITGSH GAFDIWGQGTMVTVSS |
| | | | SEQ ID NO: 25346 | SEQ ID NO: 29352 |

FIGURE 50

| | | | | |
|---|---|---|---|---|
| iPS:434057 | 21-225_51E4 | NA | GACATCCAGATGACCCAGTCTCCATCCTCCCT GTCTGCATCTGTGGGAGACAGAGTCACCATCA CTTGCCGGGCAAGTCAGGGCATTAGAAATGAT TTAGGCTGGTATCAGCAGAAACCAGGGAAAG CCCCTCAGCGCCTGATCTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAGGATTTTGCAGCTT ATTATTGTCTACAGCATAATAGTTACCCATTCA CTTTCGGCCCTGGGACCAAAGTGGATATCAAA<br><br>SEQ ID NO: 25347 | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGAACTGGG ATTTCTCTCTGACTACTGGGGCCAGGGAACCCTG GTCACCGTCTCCTCA<br><br>SEQ ID NO: 29353 |
| | | AA | DIQMTQSPSSLSASVGDRVTITCRASQGIRNDLG WYQQKPGKAPQRLIYAASSLQSGVPSRFSGSGS GTEFTLTISSLQPEDFAAYYCLQHNSYPFTFGPGT KVDIK<br><br>SEQ ID NO: 25348 | QVQLVESGGGVVQPGRSLRLSCAASGFTFSSYGMH WVRQAPGKGLEWVAVIWYDESNKYYADSVKGRF TISRDNSKNTLYLQMNSLRAEDTAVYYCARELGFL SDYWGQGTLVTVSS<br><br>SEQ ID NO: 29354 |
| iPS:434059 | 21-225_51C5 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGGAAGC CCCTAAGTCCCTTATCTATGCTGCATCCAGTTT GCGAAGTGGGGTCCCATCACAGTTCAGCGGCA GTGGATCTGGAACAGATTTCATTCTCACCATC AGCAGCCTGCAGCCTGAAGATTTTGCAACTTA TTACTGCCAACAGTATTATAGTTACCCATTCAC TTTCGGCCCTGGGACCAAAGTGGATATCAAA<br><br>SEQ ID NO: 25349 | GAGGTGCAGTTGTTGGAGTCTGGGGGGAGGCTTGG TACAGCCTGGGGGGGTCCCTGAGACTCTCCTGTGC AGCCTCTGGATTCACCTTTAGCAGCTATGTCATG AGCTGGGTCCGCCAGACTCCAGGGAAGGGGCTG GAGTGGGTCTCAACTATGAGTGGTAGTGGTGGTC GCACATACTACGCAGACTCCGTGAACGGCCGATT CACCGTCTCCAGAGACAATTCCAAGAACACGCTG TATTTGCAAATGAGCAGCCTGAGAGCCGAGGAC ACGGCCGTATATTACTGTGCGCGGGTGACTGCTT TTGACTACTGGGGCCAGGGAACCCTGGTCACCGT CTCCTCA<br><br>SEQ ID NO: 29355 |

FIGURE 50

| iPS:434061 | 21-225_51C7 | AA | DIQMTQSPSSLSASVGDRVTITCRASQGISNYLA WFQQKPGEAPKSLIYAASSLRSGVPSQFSGSGSG TDFILTISSLQPEDFATYYCQQYYSYPFTFGPGTK VDIK | EVQLLESGGGLVQPGGSLRLSCAASGFTFSSYVMS WVRQTPGKGLEWVSTMSGSGGRTYYADSVNGRFT VSRDNSKNTLYLQMSSLRAEDTAVYYCARVTAFD YWGQGTLVTVSS |
|---|---|---|---|---|
| | | | SEQ ID NO: 25350 | SEQ ID NO: 29356 |
| | | NA | GACATCCAGATGACCCAGTCTCCATCTTCCGT GTCTGCATCTGTAGGAGACAGAGTCACCATCA CTTGTCGGGCGAGTCAGGATGTTAACAACTAC TTAGCCTGGTTTCAGCAGAAACCAGGGAAAGC CCCTAAGCTCCTGATCTATGCTGCATCCAGTTT GCAAAATGGGGTCCCATCAAGGTTCAGCGGCA GTGGATCTGGGACAGATTTCACTCTCACCATC AGCAGCCTGCTGCCTGAAGATTTTGCAACTTA CTATTGTCAACAAACTAACAGTTTCCCATTCA CTTTCGGCCCTGGGACCAAAGTGGATATCAAA | GAGGTGCAGCTGTTGGAGTCTGGGGGAGGCTTG GTGCAGCCTGGGGGGTCCCTGAGACTCTCCTGTG CAGCCTCTGGATTCACCATTAGCAACTATGCCAT GACCTGGGTCCGCCAGACTCCAGGGAAGGGGCT GGAGTGGGTCTCAGTTATTAGTGCTAGTGGTGGT AACTCATTCTACGCAGACTCCGTGAAGGGCCGGT TCACCATCTCCAGAGACAATTCCGAGAACACGTT CTATCTGCAAATGAACAGCCTGAGAGCCGAGGA CACGGCCGTATATTACTGTGCGAAAGCAAGGGC AGTGGCTGGGTCACACTGGTTCGACCCCTGGGGC CAGGGAACCCTGGTCACCGTCTCCTCA |
| | | | SEQ ID NO: 25351 | SEQ ID NO: 29357 |
| | | AA | DIQMTQSPSSVSASVGDRVTITCRASQDVNNYL AWFQQKPGKAPKLLIYAASSLQNGVPSRFSGSG SGTDFTLTISSLLPEDFATYYCQQTNSFPFTFGPG TKVDIK | EVQLLESGGGLVQPGGSLRLSCAASGFTISNYAMT WVRQTPGKGLEWVSVISASGGNSFYADSVKGRFTI SRDNSENTFYLQMNSLRAEDTAVYYCAKARAVAG SHWFDPWGQGTLVTVSS |
| | | | SEQ ID NO: 25352 | SEQ ID NO: 29358 |

FIGURE 50

| iPS:434063 | 21-225_51G7 | NA | GACATCCAGATGACCCAGTCTCCATCTTCCGT GTCTGCATCTGTAGGAGACAGAGTCACCATCA CTTGTCGGGCGAGTCAGGATATTAGCAGTTGG TTAGCCTGGTATCAGCAGAAACCAGGGAAAG CCCCTAAGGTCCTGATCTATGCTCCATCCAATT TGCAAAGTGCGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTCACAGTTTCCCGTGG ACGTTCGGCCAAGGGACCAAGGTGGAAATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGCTCGCCTT GACTACTGGGGCCAGGGAACCCTGGTCACCGTCT CCTCA |
| | | | SEQ ID NO: 25353 | SEQ ID NO: 29359 |
| | | AA | DIQMTQSPSSVSASVGDRVTITCRASQDISSWLA WYQQKPGKAPKVLIYAPSNLQSAVPSRFSGSGS GTDFTLTISSLQPEDFATYYCQQAHSFPWTFGQG TKVEIK | EVQLVESGGGLVKPGGSLRLSCAASGFTFSSYSMN WVRQAPGKGLEWVSSISSSSSYIYYADSVKGRFTIS RDNAKNSLYLQMNSLRAEDTAVYYCARARLDYW GQGTLVTVSS |
| | | | SEQ ID NO: 25354 | SEQ ID NO: 29360 |
| iPS:434065 | 21-225_50D4 | NA | GACATCCAGATGACCCAGTCTCCATCTTCCGT GTCTGCATCTGTGGGAGACAGACTCACCATCA CTTGTCGGGCGAGTCAGGGTATTAGCAGGTGG TTAGCCTGGTATCAGCAGAAACCAGGGAAAG CCCCTAAGGTCCTGATCTATGCTGCATCCACTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAG A | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACCATA TGCACTGGGTGCGACAGGCCCCTGGACAAGGGC TTGAGTGGATGGGATGGATCAACCCTAATAATAA TGCCACAAACTATGCTCAGAGCTTTCAGGGCAGG GTCACCCTGACCAGGGACACGTCCATCAGCACA GCCTACATGGAGCTGAGCAGGCTGAGATCTGAC GACACGGCCGTGTATTACTGTGCGAGAGACGGT ACCAGCAGCTTTGACTTCTGGGGCCAGGGAACCC TGGTCACCGTCTCCTCA |
| | | | SEQ ID NO: 25355 | SEQ ID NO: 29361 |

FIGURE 50

| | | | | |
|---|---|---|---|---|
| | | AA | DIQMTQSPSSVSASVGDRLTITCRASQGISRWLA WYQQKPGKAPKVLIYAASTLQSGVPSRFSGSGS GTDFTLTISSLQPEDFATYYCQQANSFPFTFGPGT KVDIR | QVQLVQSGAEVKKPGASVKVSCKASGYTFTGYHM HWVRQAPGQGLEWMGWINPNNNATNYAQSFQGR VTLTRDTSISTAYMELSRLRSDDTAVYYCARDGTSS FDFWGQGTLVTVSS |
| | | | SEQ ID NO: 25356 | SEQ ID NO: 29362 |
| iPS:434067 | 21-225_51H8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTGGGCTGGTATCAGCAGAAACCAGGGAAAG CCCCTAAGCGCCTGATCTATGCTGCTTCCAGTT TGCAAAGTGGGGTCCCATCAAGGTTCAGCGGC GGTGGATCTGGGACACACTTCACTCTCACAAT CAGCAGCCTGCAGCCTGAAGATTTTGCAACTT ATTACTGTCTACAGCATAATAGTTACCCGTGG ACGTTCGGCCAAGGGACCAAGGTGGAAATCA AA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCCACTATA TGAACTGGGTGCGACAGGCCCCTGGACAAGGAC TTGAGTGGATGGGATGGGTCAACCCTAACAGTGG TGGCTCAAACTCTGCACAGCAGTTTCAGGGCAGG GTCACCATGACCAGGGACACGTCCATCAGCACA GTCTACATGGAGCTGAGCAGGCTGAGTTCTGACG ACACGGCCGTGTATTACTGTGCGAGGGGGAGGGC AGCTCGGCGGCTTTAACTTCTACTACTACGGTAT GGACGTCTGGGGCCAAGGGACCACGGTCACCGT CTCCTCA |
| | | | SEQ ID NO: 25357 | SEQ ID NO: 29363 |
| | | AA | DIQMTQSPSSLSASVGDRVTITCRASQGIRNDLG WYQQKPGKAPKRLIYAASSLQSGVPSRFSGGGS GTHFTLTISSLQPEDFATYYCLQHNSYPWTFGQG TKVEIK | QVQLVQSGAEVKKPGASVKVSCKASGYTFTGHYM NWVRQAPGQGLEWMGWVNPNSGGSNSAQQFQGR VTMTRDTSISTVYMELSRLSSDDTAVYYCARGGQL GGFNFYYYGMDVWGQGTTVTVSS |
| | | | SEQ ID NO: 25358 | SEQ ID NO: 29364 |

FIGURE 50

| iPS:434069 | 21-225_51E9 | NA | GACATCCAGATGACCCAGTCTCCATCTTCCGT GTCTGCCTCTGTAGGAGACAGAGTCACCATCA CTTGTCGGGCGAGTCAGGGTATTAGCAGTTGG TTAGCCTGGTATCAGCAGAAACCAGGGAAAG CCCCTAAGCTCCTGATCTATGTTGCATCCAGTT TGCAAAGTGGGGTCCCATCAAGGTTCAGCGGC AGTGGATCTGGGACAGATTTCACTCTCACCAT CAGGAGCCTGCAGCCTGAAGATTTTGCAACTT ACTATTGTCAACAGGCTAAAAGTTTCCCATTC ACTTTCGGCCCTGGGACCAAAGTGGATATCAA A | CAGGTGCAGCTGGTGCAGTCTGGGGGCTGAGGTG AAGAAGCCTGGGGCCTCAGTGAAGGTCTCCTGC AAGGCTTCTAGATACACCTTCACCGGCTACCATA TACACTGGGTGCGACAGGCCCCTGGACAAGGGC TTGAGTGGATGGGATGGATCAACCCTAACACTAA TGGCACACAGTATGCACAGAAGTTTCAGGGCCG GGTCACCATGACCAGGGACACGTCCATCAGCAC AGCCTACATGGAGCTGAGCAGGCTGAGATCTGA CGACACGGCCGTGTATTACTGTGCGAGAGATGGC ACCTCGTCCTTTGACTACTGGGGCCAGGGAACCC TGGTCACCGTCTCCTCA |
| | | | SEQ ID NO: 25359 | SEQ ID NO: 29365 |
| | | AA | DIQMTQSPSSVSASVGDRVTITCRASQGISSWLA WYQQKPGKAPKLLIYVASSLQSGVPSRFSGSGS GTDFTLTIRSLQPEDFATYYCQQAKSFPFTFGPGT KVDIK | QVQLVQSGAEVKKPGASVKVSCKASRYTFTGYHIH WVRQAPGQGLEWMGWINPNTNGTQYAQKFQGRV TMTRDTSISTAYMELSRLRSDDTAVYYCARDGTSS FDYWGQGTLVTVSS |
| | | | SEQ ID NO: 25360 | SEQ ID NO: 29366 |
| iPS:434071 | 21-225_51F9 | NA | GACATCCAGATGACCCAGTCTCCATCCTCCCT GTCTGCATCTGTAGGAGACAGAGTCACCATCA CTTGCCGGGCAAGTCAGGGCATTAGGACTGAT TTAGGCTGGTATCAGCAGAAACCAAGGAAAG CCCCTCAGCGCCTGATCTATGCTGCATCCAGTT TGCAACGTGGAGTCCCATCAAGATTCAGCGGC AGTGGATCTGGGACAGACTTCACTCTCACAAT CAGCAGCCTGCAGCCTGAAGATTTTGCATCTT ATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCGGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAAT AATAAATACTATGCAGACTCCGTGAAGGGCCGA TTCACCATCTCCAGAGACAATTCCAAGAACACGC TGTATCTGCAAATGAACAGCCTGAGCGCCGAGG ACACGGCTGTGTATTACTGTGCGAGGGAGCTGGG ATTTCTCTCTGACTACTGGGGCCAGGGAACCCTG GTCACCGTCTCCTCA |
| | | | SEQ ID NO: 25361 | SEQ ID NO: 29367 |

FIGURE 50

| | | | | |
|---|---|---|---|---|
| | | AA | DIQMTQSPSSLSASVGDRVTITCRASQGIRTDLG WYQQKPRKAPQRLIYAASSLQRGVPSRFSGSGS GTDFTLTISSLQPEDFASYYCLQHNSYPFTFGPGT KVDIK | QVQLVESGGGVVQPGRSLRLSCAASGFTFSDYGM HWVRQAPGKGLEWVAVIWFDGNNKYYADSVKGR FTISRDNSKNTLYLQMNSLSAEDTAVYYCARELGF LSDYWGQGTLVTVSS |
| | | | SEQ ID NO: 25362 | SEQ ID NO: 29368 |
| iPS:434073 | 21-225_51H10 | NA | GACATCCAGATGACCCAGTCTCCATCCTCCCT GTCTGCGTCTGTAGGAGACAGAGTCACCATCA CTTGCCGGGCAAGTCAGAGCATTAGTACCTAT TTAATGTGGTATCAGCAGAAACCAGGGAAAG CCCCTAAGCTCTTGATATATGCTGCATCCAGTT TGCAAAGTGGGGTCCCATCAGAGTTCAGTGCC AGTGGATCTGGGACAGATTTCACTCTCACCAT CAGCAGTCTGCAACCTGAAGATTTTGCAACTT ACTACTGTCAACAGAGTTACAGTATCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTA CAGCCTCTGGATTCACCTTTAGCAGCTATGCCAT GAGCTGGGTCCGCCAGGCTCCAGGGAAGGGGCT GGAGTGGGTCTCAGTTATTAGTGGTAGTGGTGGT ACCACATTCTACGCAGACTCCGTGAAGGGCCGGC TCACCATCTCCAGAGACAATTCCAAGAACACACT GTATCTGCAAATGAACAGCCTGAGAGCCGAGGA CACGGCCGTATATTACTGTGCGCGTCGTATAGCA GTGGCTGGGAATGATGCTTTTGATATCTGGGGCC AAGGGACAATGGTCACCGTCTCTTCA |
| | | | SEQ ID NO: 25363 | SEQ ID NO: 29369 |
| | | AA | DIQMTQSPSSLSASVGDRVTITCRASQSISTYLM WYQQKPGKAPKLLIYAASSLQSGVPSEFSASGSG TDFTLTISSLQPEDFATYYCQQSYSIPFTFGPGTK VDIK | EVQLLESGGGLVQPGGSLRLSCTASGFTFSSYAMS WVRQAPGKGLEWVSVISGSGGTTFYADSVKGRLTI SRDNSKNTLYLQMNSLRAEDTAVYYCARRIAVAG NDAFDIWGQGTMVTVSS |
| | | | SEQ ID NO: 25364 | SEQ ID NO: 29370 |

FIGURE 50

| iPS:434075 | 21-225_51B11 | NA | GCCATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGCAGAAACCAAGGAAAG CCCCTAAACGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCGGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGGTGGAAAT AATAAATACTATGGAGACTCCGTGAAGGGCCGA TTCACCATCTCCAGAGACAATTCCAAGAACACGC TGTATCTGCAAATGAACAGCCTGAGCGCCGAGG ACACGGCTGTGTATTACTGTGCGAGGGAGCTGGG ATTTCTCTCTGACTACTGGGGCCAGGGAACCCTG GTCACCGTCTCCTCA |
| | | | SEQ ID NO: 25365 | SEQ ID NO: 29371 |
| | | AA | AIQMTQSPSSLSASVGDRVTITCRTSQGIRNDLG WYQQKPRKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSDYGM HWVRQAPGKGLEWVAVIWFGGNNKYYGDSVKGR FTISRDNSKNTLYLQMNSLSAEDTAVYYCARELGF LSDYWGQGTLVTVSS |
| | | | SEQ ID NO: 25366 | SEQ ID NO: 29372 |
| iPS:434077 | 21-225_51F11 | NA | GACATCCAGATGACCCAGTCTCCATCCGCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTTTGGCAT GCACTGGGTCCGCCAGGCTCCAGGCAAGGGGCT GGAGTGGGTGGCAGTTATATGGTATGAGGAAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGAACTGG GGTTCCTCTCTGACTTCTGGGGCCAGGGAACCCT GGTCACCGTCTCCTCA |
| | | | SEQ ID NO: 25367 | SEQ ID NO: 29373 |

FIGURE 50

| | | | | |
|---|---|---|---|---|
| | | AA | DIQMTQSPSALSASVGDRVTITCRASQGIRNDLG WYQQKPGKAPKRLIYAASSLQSGVPSRFSGSGS GTEFTLTISSLQPEDFATYYCLQHNSYPFTFGGGT KVEIK | QVQLVESGGGVVQPGRSLRLSCAASGFTFSNFGMH WVRQAPGKGLEWVAVIWYEESNKYYADSVKGRF TISRDNSKNTLYLQMNSLRAEDTAVYYCARELGFL SDFWGQGTLVTVSS |
| | | | SEQ ID NO: 25368 | SEQ ID NO: 29374 |
| IPS:434079 | 21-225_52B1 | NA | GACATCCAGATGACCCAGTCTCCATCTTCCGT GTCTACATTTGTAGGAGACAGAATCACCATCA CTTGTCGGGCGAGTCAGGATATTCGCACCTGG TTAGCCTGGTATCAGCAGAAACCTGGGAAAGC CCCTAAGCTCCTGATCTATGCTGCATCCAGTTT GCAAAATGGGGTCCCATCAAGGTTCAGCGGCA GTGGATCTGGGACAGATTTCACTCTCACCATC AGTAGCCTGCAGCCTGAAGATTTTGCAACTTA CTTTTGTCAACAGGCTAAAAGTTTCCCATTCAC TTTCGGCCCTGGGACCAAAGTGGATATCAAA | CAGGTGCAGTTGGTGCAGTCTGGGGCTGAGGTGA AGAAGCCTGGGGCCTCAGTGAAGGTCTCCTGCA AGGCTTCTGGATACACCTTCACCGGCTATCATAT GCAGTGGGTGCGACAGGCCCCTGGACAAGGGCT TGAGTGGATGGGATGGATCAACCCTAACAGTGGT GCCACAAACTATGCACAGAACTTTCAGGGCAGG GTCACCATGACCCGGGACACGTCCATCAGCACA GCCTACCTGGACCTGAGCAGGCTGAGATCTGACG ACACGGCCGTGTATTACTGTGCGAGAGATGGCAC CTCGTCCTTTGACTACTGGGGGCCAGGGAACCCTG GTCACCGTCTCCTCA |
| | | | SEQ ID NO: 25369 | SEQ ID NO: 29375 |
| | | AA | DIQMTQSPSSVSTFVGDRITITCRASQDIRTWLA WYQQKPGKAPKLLIYAASSLQNGVPSRFSGSGS GTDFTLTISSLQPEDFATYFCQQAKSFPFTFGPGT KVDIK | QVQLVQSGAEVKKPGASVKVSCKASGYTFTGYHM QWVRQAPGQGLEWMGWINPNSGATNYAQNFQGR VTMTRDTSISTAYLDLSRLRSDDTAVYYCARDGTS SFDYWGQGTLVTVSS |
| | | | SEQ ID NO: 25370 | SEQ ID NO: 29376 |

FIGURE 50

| iPS:434081 | 21-225_52B2 | NA | GACATCCAGATGACCCAGTCTCCATCGTCACT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAGGCGCCTGATCTATGCTGCATCCTTTT TGCAAAGTGGGGTCCCATCGACATTCAGCGGC AGTGGATCTGGGACAGAATTCACTCTCACAAT CAGCAGCCTGCAGCCTGAAGATTTTGCAACTT ATTACTGTCTGCAGCATAATAGCTACCCTCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTACATGGTTTGATGGAAGT AATCAACGCTATGCAGACTCCGTGAAGGGCCGA TTCACCATCTCCAGAGACATTTCCAAGAACACGC TGTATCTGCAAATGAACAGCCTGAGTGCCGAGG ACACGGCTGTGTATTACTGTGCGAGAGATCTGGG GATGATCGAGGACTTCTGGGGCCAGGGAACCCT GGTCACCGTCTCCTCA |
|---|---|---|---|---|
| | | | SEQ ID NO: 25371 | SEQ ID NO: 29377 |
| | | AA | DIQMTQSPSSLSASVGDRVTITCRASQGIRNDLG WYQQKPGKAPRRLIYAASFLQSGVPSTFSGSGSG TEFTLTISSLQPEDFATYYCLQHNSYPLTFGGGT KVEIK | QVQLVESGGGVVQPGRSLRLSCAASGFTFSNYGM HWVRQAPGKGLEWVAVTWFDGSNQRYADSVKGR FTISRDISKNTLYLQMNSLSAEDTAVYYCARDLGMI EDFWGQGTLVTVSS |
| | | | SEQ ID NO: 25372 | SEQ ID NO: 29378 |
| iPS:434083 | 21-225_52H2 | NA | GACATCCAGATGACCCAGTCTCCATCTTCCGT GTCTGCATCTGTAGGAGACAGAGTCACCATCA CTTGTCGGGCGAGTCAGAATATTACCAACTGG TTAGCCTGGTTTCAGCAGAAACCAGGGAGAGC CCCTAAGCTCCTGATCTATACTACATCCAGTTT GCAAAGTGGGGTCCCATCAAGGTTCAGCGGCA GTGGATCTGGGACAGATTTCACTCTCACCATC AGCAGCCTGCAGCCTGAAGATTTTGCAACTTA CTGTTGTCAACAGACTAACAGTTTCCCGTGGA CGTTCGGCCATGGGACCAAGGTAGAAGTCAA A | GAGGTGCAGCTGTTGGAGTCTGGGGGAGGCTTG GTACAGCCGGGGGGGTCCCTGAGACTCTCCTGTG CAGCCTCTGGATTCACCTTTAGCAGAAATGCCAT GAGCTGGGTCCGCCAGGCTCCAGGGATGGGGCT GGAGTGGGTCTCAGCTATTAGTGGTCGTGGTGGT AATACATTCTACGCAGACTCCGTGAAGGGCCGGT TCACCGTCTCCAGAGACAATTCCAAGAACACGCT GTTTCTGCAAATGAACAGCCTGAGAGCCGAGGA CACGGCCGTATATTACTGTGCGAAAAATGGGCG AGAGCAGTGGCTTGACTACTGGGGCCAGGGAAC CCTGGTCACCGTCTCCTCA |
| | | | SEQ ID NO: 25373 | SEQ ID NO: 29379 |

FIGURE 50

| | | | | |
|---|---|---|---|---|
| | | AA | DIQMTQSPSSVSASVGDRVTITCRASQNITNWLA WFQQKPGRAPKLLIYTTSSLQSGVPSRFSGSGSG TDFTLTISSLQPEDFATYCCQQTNSFPWTFGHGT KVEVK | EVQLLESGGGLVQPGGSLRLSCAASGFTFSRNAMS WVRQAPGMGLEWVSAISGRGGNTFYADSVKGRFT VSRDNSKNTLFLQMNSLRAEDTAVYYCAKNGREQ WLDYWGQGTLVTVSS |
| | | | SEQ ID NO: 25374 | SEQ ID NO: 29380 |
| IPS:434085 | 21-225_52E3 | NA | GACATCCAGATGACCCAATCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCAATGCTGCATCCAGTTT ACAAAGTGGGGTCCCATCAAAGTTCAGCGGCA GTGGATCTGGGACAGATTTCACTCTCACTATC AGCAGCCTGCAGCCTGAAGATTTTGCAACTTA TTACTGCCAACAGTATAATAGTTTCCCTTTCAC TTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAAAAT GAACTGGGTCCGCCAGGCTCCAGGGAAGGGGCT GGAGTGGGTCTCATCCATTAGTAGTGGTAATAGT TCCATATACTACGCAGACTCAGTGAAGGGCCGAT TCACCATCTCCAGAGACAACGCCGAAAACTCACT GTATCTGCAAATGAACAGCCTGAGAGCCGAAGA CACGGCTGTGTATTACTGTGCGAGAGTTAGCAGT AATGACTACTGGGGCCAGGGAACCCTGGTCACC GTCTCCTCA |
| | | | SEQ ID NO: 25375 | SEQ ID NO: 29381 |
| | | AA | DIQMTQSPSSLSASVGDRVTITCRASQGISNYLA WFQQKPGKAPKSLINAASSLQSGVPSKFSGSGSG TDFTLTISSLQPEDFATYYCQQYNSFPFTFGPGTK VDIK | EVQLVESGGGLVKPGGSLRLSCAASGFTFSSYKMN WVRQAPGKGLEWVSSISSGNSSIYYADSVKGRFTIS RDNAENSLYLQMNSLRAEDTAVYYCARVSSNDY WGQGTLVTVSS |
| | | | SEQ ID NO: 25376 | SEQ ID NO: 29382 |

FIGURE 50

| | | | | |
|---|---|---|---|---|
| iPS:434087 | 21-225_52F6 | NA | GACATCCAGATGACCCAGTCTCCATCCTCCCT GTCTGCATCTGTAGGAGACAGAGTCACCATCA CTTGCCAGGCGAGTCAGGACATTAGTAACTAT TTACATTGGTATCAGCAGAAACCAGGGAAAGC CCCTAAACTCCTGATCTACGATGCATCCACTTT GGGAACAGGGGTCCCATTAAGGTTCAGTGGA AGTGGATCTGGGACAGAATTTACTTTCACCAT TAACAGCCTGCAGCCTGAAGATATTGCAACAT ATTCCTGTCAACAGTGTGATAATCTCCCGCTC ACTTTCGGCGGGGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAATTATATCATATGGAGGAAG TAATAAATACTATGCAGACTCCGTGAAGGGCCG ATTCACCATCTCCAGAGACAATTCCAAGAATACG CTGTATCTGCAAATGAACAGCCTGAGAGCTGATG ACACGGCTGTGTATTACTGTGCGAGAAGGTCAGC AGCTCGGCCGGGCTACGGTATGGACGTCTGGGG CCAGGGGACCACGGTCACCGTCTCCTCA |
| | | | SEQ ID NO: 25377 | SEQ ID NO: 29383 |
| | | AA | DIQMTQSPSSLSASVGDRVTITCQASQDISNYLH WYQQKPGKAPKLLIYDASTLGTGVPLRFSGSGS GTEFTFTINSLQPEDIATYSCQQCDNLPLTFGGGT KVEIK | QVQLVESGGGVVQPGRSLRLSCAASGFTFSSYGMH WVRQAPGKGLEWVAIISYGGSNKYYADSVKGRFTI SRDNSKNTLYLQMNSLRADDTAVYYCARRSAARP GYGMDVWGQGTTVTVSS |
| | | | SEQ ID NO: 25378 | SEQ ID NO: 29384 |
| iPS:434091 | 21-225_52B9 | NA | GCCATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAAGGAAAG CCCCTAAAGCGCCTGATCTATGCTGCATCCAGT TTGCAAAGTGGGGTCCCATTAAGGTTCAGCGG CAGTGGATCTGGGACAGAATTCACTCTCACAA TCAGAAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCGGGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTTTGATGGAAAT AATAAATACTATGCAGACTCCGTGAAGGGCCGA TTCACCATCTCCAGAGACAATTCCAAGAACACGC TGTATCTGCAAATGAACAGCCTGAGCGCCGAGG ACACGGCTGTGTATTACTGTGCGAGGGAGCTGGG ATTTCTCTCTGACTACTGGGGCCAGGGAACCCTG GTCACCGTCTCCTCA |
| | | | SEQ ID NO: 25379 | SEQ ID NO: 29385 |

FIGURE 50

| | | | | |
|---|---|---|---|---|
| | | AA | AIQMTQSPSSLSASVGDRVTITCRASQGIRNDLG WYQQKPRKAPKRLIYAASSLQSGVPLRFSGSGS GTEFTLTIRSLQPEDFATYYCLQHNSYPFTFGPGT KVDIK | QVQLVESGGGVVQPGRSLRLSCAASGFTFSDYGM HWVRQAPGKGLEWVAVIWFDGNNKYYADSVKGR FTISRDNSKNTLYLQMNSLSAEDTAVYYCARELGF LSDYWGQGTLVTVSS |
| | | | SEQ ID NO: 25380 | SEQ ID NO: 29386 |
| iPS:434093 | 21-225_52D10 | NA | GATGTTATGATGACCCAGATTCCACTCTCTCTG TCCGTCACCCCTGGACAGCCGGCCTCCATCTC CTGCAAGTCTAGTCAGAGCCTCCTGCATAGTG AAGGAAAGACCTATTTGTATTGGTACTTGCAG AAGCCAGGCCAGCCTCCACAGCTCCTGATCTT TGAAGTTTCCAACCGGGTCTCTGGAGTGCCAG ATAGGTTCAGTGGCAGAGGGTCAGGGACAGA TTTCACACTGAAAATCAGCCGGGTGGAGGCTG AGGATGTTGGGGTTTATTACTGCATGCAAAGT ATACAGTATCCGATCACCTTCGGCCAAGGGAC ACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCACTTATATGGTATGATGGAAGT AATAAATACCATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 25381 | SEQ ID NO: 29387 |
| | | AA | DVMMTQIPLSLSVTPGQPASISCKSSQSLLHSEG KTYLYWYLQKPGQPPQLLIFEVSNRVSGVPDRFS GRGSGTDFTLKISRVEAEDVGVYYCMQSIQYPIT FGQGTRLEIK | QVQLVESGGGVVQPGRSLRLSCAASGFTFSSYGMH WVRQAPGKGLEWVALIWYDGSNKYHADSVKGRF TISRDNSKNTLYLQMNSLRAEDTAVYYCARRYSSS WSGGMDVWGQGTTVTVSS |
| | | | SEQ ID NO: 25382 | SEQ ID NO: 29388 |

FIGURE 50

| iPS:434095 | 21-225_52F10 | NA | GACATCCAGATGACCCAGTCTCCATCCTCACT GTCTGTATCTGTAGGAGACAGAGTCACCATCA CTTGTCGGGCGAGTCAGGGTATTAGTAATTAT TTAGGCTGGTTTCAGCAGAAACCAGGGAAAGC CCCGAAGTCCCTGATTTATGCTGCATCTAGTTT GCAAAGTGGGGTCCCATCAAGGTTCAGCGGCA GTGGATCTGGGACAGATTTCACTCTCACCATC AGCAGCCTACAGCCTGAAGATTTTGCAACTTA TTACTGCCAACAGTATAATAGTTATCCTCCGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTTTCTATGGCAT GCACTGGGTCCGCCAGGCTCCAGGCAAGGGGCT GGAGTGGGTGGCAGTTATATGGGACGATGGAAG TAATAAATACTATGCAGACTCCGTGAAGGGCCG ATTCACCATCTCCAGAGACAATTCCAAGAACACG CTGTTTCTGCAAATGATGAGCCTGAGAGCCGAGG ACACGGCTGTGTATTACTGTGCGAGAGATTCTCT GTATAGCAGCAGCTGGTTGTTTGACTACTGGGGC CAGGGAACCCTGGTCACCGTCTCCTCA |
| | | | SEQ ID NO: 25383 | SEQ ID NO: 29389 |
| | | AA | DIQMTQSPSSLSVSVGDRVTITCRASQGISNYLG WFQQKPGKAPKSLIYAASSLQSGVPSRFSGSGSG TDFTLTISSLQPEDFATYYCQQYNSYPPTFGQGT KVEIK | QVQLVESGGGVVQPGRSLRLSCAASGFTFSFYGMH WVRQAPGKGLEWVAVIWDDGSNKYYADSVKGRF TISRDNSKNTLFLQMMSLRAEDTAVYYCARDSLYS SSWLFDYWGQGTLVTVSS |
| | | | SEQ ID NO: 25384 | SEQ ID NO: 29390 |
| iPS:434097 | 21-225_52H10 | NA | GACATCCAGATGACCCAGTCTCCATCTTCCGT GTCTGCATCTGTAGGAGACAGAGTCACCATCA CTTGTCGGGCGAGTCAGGATATTAACAGTTGG TTAGCCTGGTATCAGCAGAAACCAGGGAAAG CCCCTAAACTCCTGATCTATGTTGCATCCAGTT TGCAAAGTGGGGCCCCATCAAGGTTCAGCGGC AGTGGATCTGGGACAGATTTCACTCTCACCAT CAGGAGCCTGCAGCCTGAAGATTTTGCTACTT ACTATTGTCAACAGGCTAAAAGTTTCCCATTC ACTTTCGGCCCTGGGACCAAAGTGGATATCAA A | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACCATA TGCAGTGGGTGCGACAGGCCCCTGGACAAGGGC TTGAGTGGATGGGATGGATCAACCCTAACAATGG TGGCACACAGTATGCACAGAAGTTTCAGGGCCG GGTCACCATGACCAGGGACACGTCCATCAGCAC AGCCTACATGGAACTGAGCAGGCTGAGATCTGA CGACACGGCCGTGTATTACTGTGCGAGAGATGGC ACCTCGTCCTTTGACTACTGGGGCCAGGGAACCC TGGTCACCGTCTCCTCG |
| | | | SEQ ID NO: 25385 | SEQ ID NO: 29391 |

FIGURE 50

| | | | | |
|---|---|---|---|---|
| | | AA | DIQMTQSPSSVSASVGDRVTITCRASQDINSWLA WYQQKPGKAPKLLIYVASSLQSGAPSRFSGSGS GTDFTLTIRSLQPEDFATYYCQQAKSFPFTFGPGT KVDIK | QVQLVQSGAEVKKPGASVKVSCKASGYTFTGYHM QWVRQAPGQGLEWMGWINPNNGGTQYAQKFQGR VTMTRDTSISTAYMELSRLRSDDTAVYYCARDGTS SFDYWGQGTLVTVSS |
| | | | SEQ ID NO: 25386 | SEQ ID NO: 29392 |
| IPS:434101 | 21-225_52H12 | NA | GACATCCAGATGACCCAGTCTCCATCCTCCCT GTCTGCATCTGTAGGAGACAGAGTCACCATCA CTTGCCGGGCAAGTCAGGGCATAAGAAATAAT TTAGGCTGGTATCAGCAGAAACCAGGGAAAG CCCCTAAGCGCCTGATCTATGGTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGTATAATAGTTATCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTACT TACATATACTACGCAGACTCAGTGAAGGGCCGAT TCACCGTCTCCAGAGACAACGCCAAGAACTCACT GTATCTGCAAGTGAACAGCCTGAGAGCCGAGGA CACGGCTGTGTATTACTGTGCGAGAGTCAACTCC TTTGACTACTGGGGCCAGGGAACCCTGGTCACCG TCTCCTCA |
| | | | SEQ ID NO: 25387 | SEQ ID NO: 29393 |
| | | AA | DIQMTQSPSSLSASVGDRVTITCRASQGIRNNLG WYQQKPGKAPKRLIYGASSLQSGVPSRFSGSGS GTEFTLTISSLQPEDFATYYCLQYNSYPFTFGPGT KVDIK | EVQLVESGGGLVKPGGSLRLSCAASGFTFSSYSMN WVRQAPGKGLEWVSSISGSSTYIYYADSVKGRFTV SRDNAKNSLYLQVNSLRAEDTAVYYCARVNSFDY WGQGTLVTVSS |
| | | | SEQ ID NO: 25388 | SEQ ID NO: 29394 |

FIGURE 50

| iPS:434103 | 21-225_53G1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATCCTGCATCCAGTT TACAAAGTGGGGTCCCATCAAGGTTCAGCGGC AGCGGATCTGGGACAGAATTCAGTCTCACAAT CAGCAGCCTGCAGCCTGAAGATTTTGCAACTT ATTACTGTCTACAGGATAATAGTTACCCATTC ACTTTCGGCCCTGGGACCAAAGTGGATATCAA A | GAGGTGCAGCTGGTGGAGTCTGGGGGAGGCCTG GTCAAGCCTGGGGAGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATCGGGGC AGCACCTGGGGCCAGGGAACCCTGGTCACCGTCT CCTCA |
|---|---|---|---|---|
| | | | SEQ ID NO: 25389 | SEQ ID NO: 29395 |
| | | AA | DIQMTQSPSSLSASVGDRVTITCRASQGIRNDLG WYQQKPGKAPKRLIYPASSLQSGVPSRFSGSGSG TEFSLTISSLQPEDFATYYCLQDNSYPFTFGPGTK VDIK | EVQLVESGGGLVKPGESLRLSCAASGFTFSSYSMN WVRQAPGKGLEWVSSISGSSSYIYYADSVKGRFTIS RDNAKNSLYLQMNSLRAEDTAVYYCARDRGSTW GQGTLVTVSS |
| | | | SEQ ID NO: 25390 | SEQ ID NO: 29396 |
| iPS:434105 | 21-225_53D2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAGATACCCGCT CACTTTCGGCGGAGGGACCAAGGTGGAGATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAATGGGTGACAGTTGTATGGGATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTACGAGGGGCCTTG GCTTTACGGGAGACTACTGGGGCCAGGGAGCCC TGGTCACCGTCTCCTCA |
| | | | SEQ ID NO: 25391 | SEQ ID NO: 29397 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNRYPLTFGGG TKVEIK | QVQLVESGGGVVQPGRSLRLSCAASGFTFSNYGM HWVRQAPGKGLEWVTVVWDDGSNKYYADSVKG RFTISRDNSKNTLYLQMNSLRAEDTAVYYCTRGLG FTGDYWGQGALVTVSS |
| | | | SEQ ID NO: 25392 | SEQ ID NO: 29398 |
| iPS:434107 | 21-225_53E2 | NA | GACATCCAGATGACCCAGTCTCCATCCTCCCT GTCTGCATCTGTAGGAGACAGAGTCACCATCA CTTGCCGGGCAAGTCAGAGTTTTAGCCACTAT TTAAAATTGGTATCAGCAGAAACCAGGGAAAG CCCCTAACCTCCTGATCTTTGCTGTATCCAGTT TGCAAAGTGGGGTCCCATCACGGTTCAGTGGC AGTGGATCTGGGTCAGATTTCACTCTCCCCAT CAGTAGTTTGCAACCTGAAGATTTTGCAATTT ACTTCTGTCAACAGAGTTTCAGTACCCCATTC ACTTTCGGCCCTGGGACCAAAGTGGATATCAA A | GAGGTGCAGTTGTTGGAGTCTGGGGGAGGCTTGA TACAGCCTGGGGGGGTCCCTGAGACTCTCCTGTGC AGCCTCTGGATTCACCTTTAGGAGCTATGCCATG AGCTGGGTCCGCCAGGCTCCAGGGAAGGGGCTG GAGTGGGTCTCAGCTATTAGTGGTAGTGGTGGTA ACACATTCTACGCAGACTCCGTGAAGGGCCGGTT CACCATCTCCAGAGACAATTCCAAGAACACGCTG TATTTGCAAATGAACACCCTGAGAGCCGACGAC ACGGCCGTATATTACTGTGCGAAAAAGGTCGTGG ATACAGCCATGGCTCTTGACTACTGGGGCCAGGG AACCCTGGTCACCGTCTCCTCA |
| | | | SEQ ID NO: 25393 | SEQ ID NO: 29399 |
| | | AA | DIQMTQSPSSLSASVGDRVTITCRASQSFSHYLN WYQQKPGKAPNLLIFAVSSLQSGVPSRFSGSGSG SDFTLPISSLQPEDFAIYFCQQSFSTPFTFGPGTKV DIK | EVQLLESGGGLIQPGGSLRLSCAASGFTFRSYAMS WVRQAPGKGLEWVSAISGSGGNTFYADSVKGRFTI SRDNSKNTLYLQMNTLRADDTAVYYCAKKVVDT AMALDYWGQGTLVTVSS |
| | | | SEQ ID NO: 25394 | SEQ ID NO: 29400 |

FIGURE 50

| iPS:434111 | 21-225_53H2 | NA | GACATCCAGATGACCCAGTCTCCATCCTCCCTGTCTGCATCTGTAGGAGACAGAGTCACCATCACTTGCCAGGCGAGTCAGGACATTAGCAACTATTTACATTGGTATCAGCAGAAACCAGGGAAAGCCCCTCAGCTCCTGATCTACGATGCATCCAATTTGGAAACAGGGGTCCCATCAAGGTTCACTGGAAGTGGATCTGGGACAGATTTTACTTTCACCATCAGCAGCCTGCAGCCTGAAGATATTGCAACATATTACTGTCATCAGTATGATAATCTCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCCGGAGTGGGTGGCAGTTATATCATATGGTGGAAGTAATAAATACCATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGGCGGGGAGCAGCTCGTCCTGGCTACGGTATGGACGTCTGGGGCCAAGGGACCACGGTCACCGTCTCCTCA |
| | | | SEQ ID NO: 25395 | SEQ ID NO: 29401 |
| | | AA | DIQMTQSPSSLSASVGDRVTITCQASQDISNYLHWYQQKPGKAPQLLIYDASNLETGVPSRFTGSGSGTDFTFTISSLQPEDIATYYCHQYDNLPLTFGGGTKVEIK | QVQLVESGGGVVQPGRSLRLSCAASGFTFSSYGMHWVRQAPGKGPEWVAVISYGGSNKYHADSVKGRFTISRDNSKNTLYLQMNSLRAEDTAVYYCARRGAARPGYGMDVWGQGTTVTVSS |
| | | | SEQ ID NO: 25396 | SEQ ID NO: 29402 |
| iPS:434115 | 21-225_53E4 | NA | GACATCCAGATGACCCAGTCTCCATCCTCACTGTCTGCATCTGTAGGAGACAGAGTCACCATCACTTGTCGGGCGAGTCAGGTCATTAGCAATTATTTAGCCTGGTTTCAGCAGAAACCAGGGAAAGCCCCTAAGTCCCTGATCTCTGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTACTGCCAACAGTATCATAGTTACCCACTCACTTTCGGCCGTGGGACCAAAGTGGATATCAAA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGGCTCCAGGGAAGGGGCTGGAGTGGGTCTCAGGTATTAGTGGTAGTGGTGGTCGCACATACTACGCAGACTCCGTGAAGGGCCGGTTCAACATCTCCAGAGACAATTCCAAGAACACGCTGTATCTGCAAATGAACAGCCTGAGAGCCGAGGACACGGCCGTCTATTACTGTGCGAGGGTGGCCCTTTTTGACTACTGGGGCCAGGGAACCCTGGTCACCGTCTCCTCA |
| | | | SEQ ID NO: 25397 | SEQ ID NO: 29403 |

FIGURE 50

| | | | | |
|---|---|---|---|---|
| | | AA | DIQMTQSPSSLSASVGDRVTITCRASQVISNYLA WFQQKPGKAPKSLISAASSLQSGVPSKFSGSGSG TDFTLTISSLQPEDFATYYCQQYHSYPLTFGRGT KVDIK | EVQLLESGGGLVQPGGSLRLSCAASGFTFSSYVMS WVRQAPGKGLEWVSGISGSGGRTYYADSVKGRFN ISRDNSKNTLYLQMNSLRAEDTAVYYCARVALFDY WGQGTLVTVSS |
| | | | SEQ ID NO: 25398 | SEQ ID NO: 29404 |
| iPS:434117 | 21-225_53C6 | NA | GACATCCAGATGACCCAGTCTCCATCCTCCCT GTCTGCATCTGTAGGAGACAGAGTCACCATCA CTTGCCGGGCAAGTCAGTACAGTAGCGACTAT TTAAAATTGGTATCAGCAGAAACCAGGGAAAG CCCCTAAGGTCCTGATCTTTGCTGCATCCAGTT TGAAAAGTGGGGTCCCATCAAGGTTCAGTGGC AGTGGATCTGGGACAGATTTCACTCTCACCAT CAGCAGTCTGGAACCTGAGGATTTTGCGACTT ACTTCTGTCAACAGAGTTACAGTACCCCGTTC ACCTTCGGCCAAGGGACACGACTGGAGATTAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGCTATTAGTGGTAGTGGTGGT GCCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTATTGTGCGAAACCTCTAGT GGGAGCCCATGATGCTTTTGAAATCTGGGGCCAA GGGACAATGGTCACCGTCTCTTCA |
| | | | SEQ ID NO: 25399 | SEQ ID NO: 29405 |
| | | AA | DIQMTQSPSSLSASVGDRVTITCRASQYSSDYLN WYQQKPGKAPKVLIFAASSLKSGVPSRFSGSGSG TDFTLTISSLEPEDFATYFCQQSYSTPFTFGQGTR LEIK | EVQLLESGGGLVQPGGSLRLSCAASGFTFSSYAMN WVRQAPGKGLEWVSAISGSGGATYYADSVKGRFT ISRDNSKNTLYLQMNSLRAEDTAVYYCAKPLVGA HDAFEIWGQGTMVTVSS |
| | | | SEQ ID NO: 25400 | SEQ ID NO: 29406 |

FIGURE 50

| iPS:434119 | 21-225_53E6 | NA | GACATCCAGATGACCCAGTCTCCATCCTCCCT GTCTGCCTCTGTAGGCGCCAGAGTCACCATCA CTTGCCGGGCAAGTCAGGGCATTAGAAATGAT TTAGGCTGGTATCAGCAGAATCCGGGGAAAGC CCCTAAGCGCCTGATCTATGCTGCATCCAATTT GCAAAGTGGGGTCCCATCAAGGTTCAGCGGCA GTGGATCTGGGACAGAATTCACTCTCACTATC AGCAGCCTGCAGCCTGAAGATTTTGCAACTTA TTTCTGTCTACAACATAATCGTTACCCGCTCAC TTTCGGCGGAGGGACCAAGGTGGAGATCAAA | CAGGTCCAGCTGGTGGAGTCTGGGGGAGGCGTG GTCCAGCCTGGGAGGTCCCTGAGACTCTCCTGTA CAACGTCTGGATTCACCTTCAGTGACTATGGCAT CCACTGGGTCCGCCAGGCTCCAGGCAAGGGGCT GGAGTGGGTGGCAGTTATATGGTATGATGAAAGT AATAAATACTATGGAGACTCCGTGAAGGGCCGA TTCGCCATCTCCAGAGACAATTCCAAGAACACGC TGTATCTGCAAATGAACAGCCTGAGAGCCGAGG ACACGGCTGTGTATTATTGTGTGAGAGAACTGGG GATGACGTCTGACTACTGGGGCCAGGGAACCCT GGTCACCGTCTCCTCA |
| | | | SEQ ID NO: 25401 | SEQ ID NO: 29407 |
| | | AA | DIQMTQSPSSLSASVGARVTITCRASQGIRNDLG WYQQNPGKAPKRLIYAASNLQSGVPSRFSGSGS GTEFTLTISSLQPEDFATYFCLQHNRYPLTFGGGT KVEIK | QVQLVESGGGVVQPGRSLRLSCTTSGFTFSDYGIH WVRQAPGKGLEWVAVIWYDESNKYYGDSVKGRF AISRDNSKNTLYLQMNSLRAEDTAVYYCVRELGM TSDYWGQGTLVTVSS |
| | | | SEQ ID NO: 25402 | SEQ ID NO: 29408 |
| iPS:434121 | 21-225_53F6 | NA | GACATCCAGATGACCCAGTCTCCATCCTCCCT GTCTGCATCTGTAGGAGACAGAGTCACCATCA CTTGCCAGGCGAGTCAAGACATTACCAACTAT TTAGATTGGTATCAGCAGAAACCGGGGAAAG CCCCTAAACTCCTGATCTACGATGCATCCAAT TTGGGAACAGGGGTCCCATCAAGGTTCAGTGG AAGTGGATCTGGGACAGATTTTACTTTCACCA TCAGCAGCCTGCAGCCTGAAGATATTGCAACA TATTACTGTCAACAGTGTGATAATCTCCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGGTGGAAGT AATAAATACGATGCAGACTCCGTGAAGGGCCGA TTCACCATCTCCAGAGACAATTCCAAGAACACGC TGTATCTGCAAATGACCAGCCTGAGAGCTGAGG ACACGGCTGTGTATTACTGTGCGAGACGACGGGC AGCTCGTCCAGGGTACGGTATGGACGTCTGGGGC CAAGGGACCACGGTCACCGTCTCCTCA |
| | | | SEQ ID NO: 25403 | SEQ ID NO: 29409 |

FIGURE 50

| | | | | |
|---|---|---|---|---|
| | | AA | DIQMTQSPSSLSASVGDRVTITCQASQDITNYLD WYQQKPGKAPKLLIYDASNLGTGVPSRFSGSGS GTDFTFTISSLQPEDIATYYCQQCDNLPLTFGGGT KVEIK | QVQLVESGGGVVQPGRSLRLSCAASGFTFSSYGMH WVRQAPGKGLEWVAVISYGGSNKYDADSVKGRFT ISRDNSKNTLYLQMTSLRAEDTAVYYCARRRAARP GYGMDVWGQGTTVTVSS |
| | | | SEQ ID NO: 25404 | SEQ ID NO: 29410 |
| IPS:434123 | 21-225_53F7 | NA | GACATCCAGATGTCCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAACCTCCTGATCTATGCTGCATCCAGTT TGCAAAGTGGGGTCCCATCAAGGTTCAGCGGC AGTGGCTCTGGGACAGATTTCACTCTCACCAT CAGCAGCCTGCAGCCTGAAGATTTTGCAATTT ACTATTGTCAGCAGGCTAACAGTTTCCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACCATA TGCACTGGGTGCGACAGGCCCCTGGACAAGGGC TTGAGTGGATGGGATGGATCAACCCTAACAATAA CGGCACAAACTATGCACAGAAGTTTCAGGGCAG GGTCACCATGACCAGGGACACGTCCATCAGCAC AGCCTACATGGAGCTGAACAGGCTGACATCTGA CGACACGGCCGTGTATTACTGTGCGAGAGACGGT ACCAGCAGCTTTGACTACTGGGGCCAGGGAACC CTGGTCACCGTCTCCTCA |
| | | | SEQ ID NO: 25405 | SEQ ID NO: 29411 |
| | | AA | DIQMSQSPSSVSASVGDRVTITCRASQGISRWLA WYQQKPGKAPNLLIYAASSLQSGVPSRFSGSGS GTDFTLTISSLQPEDFAIYYCQQANSFPFTFGPGT KVDIK | QVQLVQSGAEVKKPGASVKVSCKASGYTFTGYHM HWVRQAPGQGLEWMGWINPNNNGTNYAQKFQGR VTMTRDTSISTAYMELNRLTSDDTAVYYCARDGTS SFDYWGQGTLVTVSS |
| | | | SEQ ID NO: 25406 | SEQ ID NO: 29412 |

FIGURE 50

| iPS:434127 | 21-225_53H8 | NA | GAAATTGTGTTGACGCAGTCTCCAGGCACCCT GTCTTTGTCTCCAGGGGAAAGAGCCACCCTCT CCTGCAGGGCCAGTCAGAGTATTACAAGCAGC TACTTAGCCTGGTACCAGCAGAAACCTGGCCA GGCTCCCAGGCTCCTCATCTATGGTGCGTCCG GCAGGGCCACTGGCATCCCAGACAGGTTCAGT GGCAGTGGGTCTGGGACAGACTTCACCCTCAC CATCAGCAGACTGGAGCCTGAAGATTTTGCAG TGTATTACTGTCAGCAGTTTGAAAGCTCACCC ATGTGCAGTTTTGGCCAGGGGACCAACCTGGA GATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGCCCGTAT TGGGTACTTTGACTCCTGGGGCCAGGGAACCCTG GTCACCGTCTCCTCA |
| | | | SEQ ID NO: 25407 | SEQ ID NO: 29413 |
| | | AA | EIVLTQSPGTLSLSPGERATLSCRASQSITSSYLA WYQQKPGQAPRLLIYGASGRATGIPDRFSGSGS GTDFTLTISRLEPEDFAVYYCQQFESSPMCSFGQ GTNLEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARARIGY FDSWGQGTLVTVSS |
| | | | SEQ ID NO: 25408 | SEQ ID NO: 29414 |
| iPS:434129 | 21-225_53B12 | NA | GACATCCAGATGACCCAGTCTCCATCCTCCCT GTCTGCATCTGTAGGAGACAGAGTCACCATCA CTTGTCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCGGGGAGGTCCCTGAGACTCTCCTGTG CAGCGTCTGGATTCACCTTCAGTAACTTTGGCAT GCACTGGGTCCGCCAGGCTCCAGGCAAGGGGCT GGAGTGGGTGGCAGTTGTATGGTATGATGGAAAT AATAGATACTATGCAGACTCCGTGAAGGGCCGA TTCACCATCTCCAGAGACAATTCCAAGAACACGC TCTATCTGCAAATGCACAGCCTGAGAGCCGAGG ACACGGCTGTGTATTACTGTGCGAGGGAACTGGG ATTTCTCTCTGACTTCTGGGGCCAGGGAACCCTG GTCACCGTCTCCTCA |
| | | | SEQ ID NO: 25409 | SEQ ID NO: 29415 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSNFGMH WVRQAPGKGLEWVAVVWYDGNNRYYADSVKGR FTISRDNSKNTLYLQMHSLRAEDTAVYYCARELGF LSDFWGQGTLVTVSS |
| | | | SEQ ID NO: 25410 | SEQ ID NO: 29416 |
| IPS:434131 | 21-225_54D3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGATTCAGCGG CAGTGGATCTGGGACAGAATTCACTCTCACAA TCAGCAGCCTGCAGCCTGAAGATTTTGCAACT TATTACTGTCTACAACATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTACATGGTTTGATGGAAAT AATAACTACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCTTTCTGATTATTGGGGCCAGGGAACCCTGG TCACCGTCTCCTCA |
| | | | SEQ ID NO: 25411 | SEQ ID NO: 29417 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSNYGM HWVRQAPGKGLEWVAVTWFDGNNNYYADSVKG RFTISRDNSKNTLYLQMNSLRAEDTAVYYCARELG FLSDYWGQGTLVTVSS |
| | | | SEQ ID NO: 25412 | SEQ ID NO: 29418 |

FIGURE 50

| | | | | |
|---|---|---|---|---|
| iPS:434133 | 21-225_54G3 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAACTCCTGATCTATGATGCATCCAGTT TGCAAAGTGGGGTCCCATCAAGGTTCAGCGGC AGTGGATCTGAGACAGATTTCACTCTCACCAT CAGCAGCCTGCAGCCTGAAGATTTTGCAACTT ACTATTGTCAACAGGCTAACAGTTTCCCGTGG ACGTTCGGCCAAGGGACCAAGGTGGAAATCA AA | GAGGTGCAGCTGTTGGAGTCTGGGGGAGGCTTG GTACAGCCTGGGGGGTCCCTGAGACTCTCCTGTG CAGCCTCTGGATTCACCTTTAGCACCTATGCCAT GAGTTGGGTCCGCCAGGCTCCAGGGAAGGGGCT GGAGTGGGTCTCAGCTATTAGTGGTAGTGGTGTT AACACATTCTACGCAGACTCCGTGAAGGGCCGGT TCACCATCTCCAGAGACAATTCCAAGAACACGCT GTTTCTGCAAATGAACAGCCTGAGAGCCGAGGA CACGGCCGTATATTACTGTGCGAAACTGGGGAA GGACTACTACTACTACGGTATGGACGTCTGGGGC CAAGGGACCACGGTCACCGTCTCCTCA |
| | | | SEQ ID NO: 25413 | SEQ ID NO: 29419 |
| | | AA | DIQMTQSPSSVSASVGDRVTITCRASQGISSWLA WYQQKPGKAPKLLIYDASSLQSGVPSRFSGSGSE TDFTLTISSLQPEDFATYYCQQANSFPWTFGQGT KVEIK | EVQLLESGGGLVQPGGSLRLSCAASGFTFSTYAMS WVRQAPGKGLEWVSAISGSGVNTFYADSVKGRFTI SRDNSKNTLFLQMNSLRAEDTAVYYCAKLGKDYY YYGMDVWGQGTTVTVSS |
| | | | SEQ ID NO: 25414 | SEQ ID NO: 29420 |
| iPS:434135 | 21-225_54H3 | NA | GACATCCAGATGACCCAATCTCCATCCTCCCT GTCTGCATCTGTTGGAGACAGAGTCACCATCA CTTGCCGGGCAAGTCAGGACATTAGAAATATT TTAGGCTGGTATCAGCAGAAACCAGGGAAAG CCCCTAAGCGCCTGATCTATGCTGCATCCAAT TTGCAAAGTGGAGTCCCATCAAGGTTCAGCGG CAGTGGATCTGGGACAGAATTCACTCTCACAA TCAGCAGCCTGCAGCCTGAGGATTTCGCAACT TATTACTGTCTACAGTATAATAGTTACCCGTG GACGTTCGGCCAAGGGACCAAGGTGGAAATC AAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GATCTGGGTCCGCCAGGCTCCAGGGAAGGGGCT GGAGTGGGTCTCATCCATTAGTGGTACTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGGGAATGACTACA GTAATTTGGGGCCAGGGAACCCTGGTCACCGTCT CCTCA |
| | | | SEQ ID NO: 25415 | SEQ ID NO: 29421 |

FIGURE 50

| | | | | |
|---|---|---|---|---|
| | | AA | DIQMTQSPSSLSASVGDRVTITCRASQDIRNILG WYQQKPGKAPKRLIYAASNLQSGVPSRFSGSGS GTEFTLTISSLQPEDFATYYCLQYNSYPWTFGQG TKVEIK | EVQLVESGGGLVKPGGSLRLSCAASGFTFSSYSMI WVRQAPGKGLEWVSSISGTSSYIYYADSVKGRFTIS RDNAKNSLYLQMNSLRAEDTAVYYCAGMTTVIW GQGTLVTVSS |
| | | | SEQ ID NO: 25416 | SEQ ID NO: 29422 |
| iPS:434137 | 21-225_54D4 | NA | GATATTGTGATGACCCAGACTCCACTCTCTCT GTCCGTCACCCCTGGACAGCCGGCCTCCATGT CCTGCAAGTCTAGTCAGAGCCTCCTGCATAGT GAGGGAAAGACCTATTTGTATTGGTACCTGCG GAAGCCAGGCCAGCCTCCACAGTTCCTGATCT TTGAAGTTTCCAACCGGTTCTCTGGAGTGCCA GATAGGTTCAGTGGCAGCGGGTCAGGGACAG AATTCACACTGAAAATCAGCCGGGTGGAGGCT GAGGATGTTGGGATTTATTACTGCATGCAAAG TATACAGTTTCCATTCACTTTCGGCCCTGGGAC CAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 25417 | SEQ ID NO: 29423 |
| | | AA | DIVMTQTPLSLSVTPGQPASMSCKSSQSLLHSEG KTYLYWYLRKPGQPPQFLIFEVSNRFSGVPDRFS GSGSGTEFTLKISRVEAEDVGIYYCMQSIQFPFTF GPGTKVDIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WSGGMDVWGQGTTVTVSS |
| | | | SEQ ID NO: 25418 | SEQ ID NO: 29424 |

FIGURE 50

| | | | | |
|---|---|---|---|---|
| iPS:434141 | 21-225_54C6 | NA | GACATCCAGATGACCCAGTCTCCATCCTCCCT GTCTGCCTCTGTAGGCGCCAGAGTCACCATCA CTTGCCGGGCAAGTCAGGGCATTAGAAATGAT TTAGGCTGGTATCAGCAGAATCCGGGGAAAGC CCCTAAGCGCCTGATCTATGCTGCATCCAATTT GCAAAGTGGGGTCCCATCAAGGTTCAGCGGCA GTGGATCTGGGACAGAATTCACTCTCACTATC AGCAGCCTGCAGCCTGAAGATTTTGCAACTTA TTTCTGTCTACAGCATAATCGTTACCCGCTCAC TTTCGGCGGAGGGACCAAGGTGGAGATCAAA | CAGGTCCAGCTGGTGGAGTCTGGGGGAGGCGTG GTCCAGCCTGGGAGGTCCCTGAGACTCTCCTGTA CAACGTCTGGATTCACCTTCAGTGACTATGGCAT CCACTGGGTCCGCCAGGCTCCAGGCAAGGGGCT GGACTGGGTGGCAGTTATATGGTATGATGAAAAT AATAAATACTATGCAGACTCCGTGAAGGGCCGA TTCGCCATCTCCAGAGACAATTCCAAGAACACGC TGTATCTGCAAATGAACAGCCTGAGAGCCGAGG ACACGGCTGTGTATTATTGTGTGAGAGAACTGGG GATGACGTCTGACTACTGGGGCCAGGGAACCCT GGTCACCGTCTCCTCA |
| | | | SEQ ID NO: 25419 | SEQ ID NO: 29425 |
| | | AA | DIQMTQSPSSLSASVGARVTITCRASQGIRNDLG WYQQNPGKAPKRLIYAASNLQSGVPSRFSGSGS GTEFTLTISSLQPEDFATYFCLQHNRYPLTFGGGT KVEIK | QVQLVESGGGVVQPGRSLRLSCTTSGFTFSDYGIH WVRQAPGKGLDWVAVIWYDENNKYYADSVKGRF AISRDNSKNTLYLQMNSLRAEDTAVYYCVRELGM TSDYWGQGTLVTVSS |
| | | | SEQ ID NO: 25420 | SEQ ID NO: 29426 |
| iPS:434143 | 21-225_54G7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCTAGGTTCAGCGG CAGTGGATCTGGGACAGAATTCACTCTCACAA TCAGCAGCCTGCAGCCTGAAGATTTTGCAACT TATTACTGTCTACATCATAATACTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGAGGAAAG TAATAAATACTATGGAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGAATTGG GGTTCCTCTCTGACTACTGGGGCCAGGGAACCCT GGTCACCGTCTCCTCA |
| | | | SEQ ID NO: 25421 | SEQ ID NO: 29427 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLHHNTYPFTFGPGT KVDIK | QVQLVESGGGVVQPGRSLRLSCAASGFTFSNYGM HWVRQAPGKGLEWVAVIWYEESNKYYGDSVKGR FTISRDNSKNTLYLQMNSLRAEDTAVYYCARELGF LSDYWGQGTLVTVSS |
| | | | SEQ ID NO: 25422 | SEQ ID NO: 29428 |
| IPS:434145 | 21-225_55B1 | NA | GACATCCAGATGACCCAGTCTCCATCTTCCGT GTCTGCATCTGTAGGGGACAGAGTCACCATCA CTTGTCGGGCGAGTCAGGTTATTAGCCGCTGG TTAGCCTGGTATCAGCAGAAACCAGGGAAAG CCCCTAACCTCCTGATCTATGCTGCATCCAGTT TGCAAAGTGGGGTCCCATCAAGGTTCAGCGGC AGTGGATCTGGGACAGATTTCACTCTGACCAT CAGCAGCCTGCAGCCTGAAGATTTTGCAACTT ACTATTGTCAACAGGCTAACAGTTTCCCATTC ACTTTCGGCCCTGGGACCAAAGTGGATCTCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATT TCCACTGGGTGCGACAGGCCCCTGGTCAAGGGCT TGAGTGGATGGGATGGATCCACCCTAACAATAAT GCCACAAACTATGCACAGAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGAAGGATGGC AGATCGTCCTTTGACTACTGGGGGCCAGGGAACCC TGGTCACCGTCTCCTCA |
| | | | SEQ ID NO: 25423 | SEQ ID NO: 29429 |
| | | AA | DIQMTQSPSSVSASVGDRVTITCRASQVISRWLA WYQQKPGKAPNLLIYAASSLQSGVPSRFSGSGS GTDFTLTISSLQPEDFATYYCQQANSFPFTFGPGT KVDLK | QVQLVQSGAEVKKPGASVKVSCKASGYTFTGYYF HWVRQAPGQGLEWMGWIHPNNNATNYAQKFQGR VTMTRDTSISTAYMELSRLRSDDTAVYYCAKDGRS SFDYWGQGTLVTVSS |
| | | | SEQ ID NO: 25424 | SEQ ID NO: 29430 |

FIGURE 50

| iPS:434147 | 21-225_55E1 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCGAC TTAGCCTGGTACCAGCTGAAACCTGGCCAGGC TCCCAGGCTCCTCATCTATGATGCATCCGCCA GGGCCACTGGTATCCCAGCCAGGTTCAGTGGC AGTGGGTCTGGGACAGAGTTCACTCTCACCAT CAGCAGCCTGCAGTCTGAAGATTTTGCATTTT ATTACTGTCAGCAGTATTATAACTGGCCTCTC ACTTTCGGCGGAGGGACCAAGGTGGAGATCA AA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTAGT AGCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTTTTTTACTGTGCGAAAGATCACGGT ATAGTGGGAACTATTTACTTTGACTACTGGGGCC AGGGAACCCTGGTCACCGTCTCCTCA |
| | | | SEQ ID NO: 25425 | SEQ ID NO: 29431 |
| | | AA | EIVMTQSPATLSVSPGERATLSCRASQSVSSDLA WYQLKPGQAPRLLIYDASARATGIPARFSGSGSG TEFTLTISSLQSEDFAFYYCQQYYNWPLTFGGGT KVEIK | EVQLLESGGGLVQPGGSLRLSCAASGFTFSSYAMS WVRQAPGKGLEWVSAISGRGSSTFYADSVKGRFTI SRDNSKNTLYLQMNSLRAEDTAVFYCAKDHGIVG TIYFDYWGQGTLVTVSS |
| | | | SEQ ID NO: 25426 | SEQ ID NO: 29432 |
| iPS:434149 | 21-225_55H1 | NA | GATATTGTGATGACCCAGACTCCACTCTCTCT GTCCGTCACCCCTGGACAGCCGGCCTCCATCT CCTGCAAATCTAGTCAGAGCCTCCTGCATAGT GAAGGAAAGACCTATTTGTATTGGTACCTGCA GAAGCCAGGCCAGCCTCCACAGTTCCTGATCT TTGAAGTTTCCCACCGGTTCTCTGGAGTGCCA GATAGGTTCAGTGGCAGCGGGTCAGGGACAG ATTTCACTCTGAAAATCAGCCGGGTGGAGGCT GAGGATGTTGGGGTTTATTACTGCATGCAAAG TATACAGCTTCCATTCACTTTCGGCCCTGGGAC CAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 25427 | SEQ ID NO: 29433 |

FIGURE 50

| | | | | |
|---|---|---|---|---|
| | | AA | DIVMTQTPLSLSVTPGQPASISCKSSQSLLHSEGK TYLYWYLQKPGQPPQFLIFEVSHRFSGVPDRFSG SGSGTDFTLKISRVEAEDVGVYYCMQSIQLPFTF GPGTKVDIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WSGGMDVWGQGTTVTVSS |
| | | | SEQ ID NO: 25428 | SEQ ID NO: 29434 |
| iPS:434151 | 21-225_55C2 | NA | GATGTTATGATGACCCAGATTCCACTCTCTCTG TCCGTCACCCCTGGACAGCCGGCCTCCATCTC CTGCAAGTCTAGTCAGAGCCTCGTGCATAGTG AAGGAAAGACCTATTTGTATTGGTATTTGCAG AAGCCAGGCCAGCCTCCACAGCTCCTGATCTT TGAAGTTTCCAACCGGGTCTCTGGAGTGCCAG ATAGGTTCAGTGGCAGAGGGTCAGGGACAGA TTTCACACTGAAAAATCAGCCGGGTGGAGGCTG AGGATGTTGGGGTTTATTACTGCATGCAAAGT ATACTGTATCCGATCACCTTCGGCCAAGGGAC ACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCACTTATATGGTATGATGGAAGT AATAAATACCATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGCGCGAGAAGGTATAG CAGCAGCTGGTCGGGCGGTATGGACGTCTGGGG CCAAGGGACCACGGTCACCGTCTCCTCA |
| | | | SEQ ID NO: 25429 | SEQ ID NO: 29435 |
| | | AA | DVMMTQIPLSLSVTPGQPASISCKSSQSLVHSEG KTYLYWYLQKPGQPPQLLIFEVSNRVSGVPDRFS GRGSGTDFTLKISRVEAEDVGVYYCMQSILYPIT FGQGTRLEIK | QVQLVESGGGVVQPGRSLRLSCAASGFTFSSYGMH WVRQAPGKGLEWVALIWYDGSNKYHADSVKGRF TISRDNSKNTLYLQMNSLRAEDTAVYYCARRYSSS WSGGMDVWGQGTTVTVSS |
| | | | SEQ ID NO: 25430 | SEQ ID NO: 29436 |

FIGURE 50

| iPS:434155 | 21-225_55B3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTAGATCTGGGACAGAATTCACTCTCACAATCAGCAGCCTGCAGCCTGAAGATTTTGCATCTTATTACTGTCTACAACATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTTTGATGGAAATAATAAATACTATGAAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGTGAGGGAACTGGGATTTCTCTCTGACTACTGGGGCCAGGGAACCCTGGTCACCGTCTCCTCA |
|---|---|---|---|---|
| | | | SEQ ID NO: 25431 | SEQ ID NO: 29437 |
| | | AA | DIQMTQSPSSLSASVGDRVTITCRASQGIRNDLGWYQQKPGKAPKRLIYAASSLQSGVPSRFSGSRSGTEFTLTISSLQPEDFASYYCLQHNSYPFTFGPGTKVDIK | QVQLVESGGGVVQPGRSLRLSCAASGFTFSSYGMHWVRQAPGKGLEWVAVIWFDGNNKYYEDSVKGRFTISRDNSKNTLYLQMNSLRAEDTAVYYCVRELGFLSDYWGQGTLVTVSS |
| | | | SEQ ID NO: 25432 | SEQ ID NO: 29438 |
| iPS:434157 | 21-225_55D4 | NA | GACATCCAGATGACCCAGTCTCCATCCTCTCTGTTTGCATCTGTAGGAGACAGAGTCACCATCACTTGTCGGGCGAGTCAGGACATTAGCAATTATTTAATCTGGTTTCAGCAGAAACCAGGGAAAGCCCCTAAGTCCCTGATTTATACTGCATCCAGTTTGCAAAGTGGGGTCCCCTCAAAGTTCAGCGGCAGTGGATTTGGGACAGATTTCACTCTCACCATCAGCAACCTGCAGCCTGAAGATTTTGCGACTTATTACTGCCAACAGTATCATAGTTTCCCTCTCACTTTCGGCGGAGGGACCAGGGTGGAGATCAGA | GAGGTGCAACTGGTGGAGTCTGGGGGAGGCCTGGTCAAGCCTGGGGGGTCCCTGAGACTCTCCTGTGCAGCCTCTGGATTCACCTTCAATAGTTATAGGATGAACTGGGTCCGCCAGGCTCCAGGGAAGGGGCTGGAGTGGGTCTCATCCATTAGTAGTAGTAGTAATCACATAGACTACGCAGACTCAGTGAAGGGCCGATTCACCATCTCCAGAGACAACGCCAAAAACTCACTATATTTGCAAATGAACAGCCTGAGAGCCGAGGACACGGCTTTATATTACTGTGCGAGAGGGACTGACTACTGGGGCCAGGGAACCCTGGTCTCCGTCTCCTCA |
| | | | SEQ ID NO: 25433 | SEQ ID NO: 29439 |

FIGURE 50

| | | | | | |
|---|---|---|---|---|---|
| | | AA | DIQMTQSPSSLFASVGDRVTITCRASQDISNYLIW FQQKPGKAPKSLIYTASSLQSGVPSKFSGSGFGT DFTLTISNLQPEDFATYYCQQYHSFPLTFGGGTR VEIR | EVQLVESGGGLVKPGGSLRLSCAASGFTFNSYRMN WVRQAPGKGLEWVSSISSSSNHIDYADSVKGRFTIS RDNAKNSLYLQMNSLRAEDTALYYCARGTDYWG QGTLVSVSS | |
| | | | SEQ ID NO: 25434 | SEQ ID NO: 29440 | |
| IPS:434159 | 21-225_55B8 | NA | GACATCCAGATGACCCAGTCTCCATCCTCCCT GTCTTCATCTGTAGGAGACAGAGTCACCATCA CTTGCCGGGCAAGTCAGGCCATTAGAAATGAT TTAGGCTGGTATCAGCAGAAACCAGGGAAAG CCCCTAAGCGCCTGATCCATGCTGCATTCAGG TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCTCTC ACTTTCGGCGGAGGGACCAAGGTGGGGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAATGGTT TGACTACTGGGGCCAGGGAACCCTGGTCACCGTC TCCTCA | |
| | | | SEQ ID NO: 25435 | SEQ ID NO: 29441 | |
| | | AA | DIQMTQSPSSLSSSVGDRVTITCRASQAIRNDLG WYQQKPGKAPKRLIHAAFRLQSGVPSRFSGSGS GTEFTLTISSLQPEDFATYYCLQHNSYPLTFGGG TKVGIK | QVQLVESGGGVVQPGRSLRLSCAASGFTFSDYGM HWVRQAPGKGLEWVAVIWYDENNKYYADSVKGR FTISRDNSKNTLYLQMNSLRAEDTAVYYCTREWFD YWGQGTLVTVSS | |
| | | | SEQ ID NO: 25436 | SEQ ID NO: 29442 | |

FIGURE 50

| iPS:434161 | 21-225_55F9 | NA | GATATTGTGATGACCCAGACTCCACTCTCTCT GTCCGTCACCCCTGGACAGTCGGCCTCCATCT CCTGCAAGTCTAGTCAAAGCCTCCTGCATAGT GAAGGAAAGACCTATTTGTATTGGTACTTGCA GAAGCCAGGCCAGCCTCCACAGCTCCTGATCT ATGAAGTTTCCAACCGGTTCTCTGGAGTGCCA GATAGGTTCAGCGGCAGCGGGTCAGGGACAG ATTTTACACTGAAAATCAGCCGGGTGGAGGCT GAGGATGTTGGGGTTTATTACTGCATACAAAG TATACAACTTCCGATCACCTTCGGCCAAGGGA CACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAAT GGCAAATATTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 25437 | SEQ ID NO: 29443 |
| | | AA | DIVMTQTPLSLSVTPGQSASISCKSSQSLLHSEGK TYLYWYLQKPGQPPQLLIYEVSNRFSGVPDRFSG SGSGTDFTLKISRVEAEDVGVYYCIQSIQLPITFG QGTRLEIK | QVQLVESGGGVVQPGRSLRLSCAASGFTFSSYGMH WVRQAPGKGLEWVAVIWYDGNGKYYADSVKGRF TISRDNSKNTLYLQMNSLRAEDTAVYYCARRYSSS WSGGMDVWGQGTTVTVSS |
| | | | SEQ ID NO: 25438 | SEQ ID NO: 29444 |
| iPS:434163 | 21-225_50H1 | NA | GACATCCAGATGACCCAGTCTCCATCCTCCCC GTCTGCTTCTGTAGGAGACACAGAGTCACCATCA CTTGCCAGGCGAGTCAAGACATTAGCAACTAT TTAGATTGGTATCAGCAGAAACCAGGGAAAG CCCCTAAACTCCTGATATACGATGCATCCAAT TTGGAAACAGGGGTCCCATCAAGGTTCAGTGG AAGTGGATCTGGGACAGATTTTGCTTTCACCA TCAGCAGCCTGCAGCCTGAAGATATTGCAACA TATTACTGTCAACAGTGTGATAATCTCCCCGCTC ACTTTCGGCGGAGGGACCAAGGTGGAGATCA AA | CAGGTGCAGTTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGTCAATTATATCATATGGTGGAAGT AATAAATACGATGCAGACTCCGTGAAGGGCCGA TTCACCATCTCCAGAGACAATTCCAAGAACACGC TGTATCTGCAAATGACCAGCCTGAGAGCTGAGG ACACGGCTGTGTATTACTGTGCGAGACGACGGGC AGCTCGTCCAGGGTACGGTATGGACGTCTGGGGC CAAGGGATCACGGTCACCGTCTCCTCA |
| | | | SEQ ID NO: 25439 | SEQ ID NO: 29445 |

FIGURE 50

| | | | | |
|---|---|---|---|---|
| | | AA | DIQMTQSPSSPSASVGDRVTITCQASQDISNYLD WYQQKPGKAPKLLIYDASNLETGVPSRFSGSGS GTDFAFTISSLQPEDIATYYCQQCDNLPLTFGGG TKVEIK | QVQLVESGGGVVQPGRSLRLSCAASGFTFSSYGMH WVRQAPGKGLEWVSIISYGGSNKYDADSVKGRFTI SRDNSKNTLYLQMTSLRAEDTAVYYCARRRAARP GYGMDVWGQGITVTVSS |
| | | | SEQ ID NO: 25440 | SEQ ID NO: 29446 |
| iPS:434165 | 21-225_50F2 | NA | GACATCCAGATGACCCAGTCTCCATCCTCCCT GTCTGCATCTGTAGGAGACAGAATCACCATCA CTTGCCGGGCAAGTCAGAGCATTCTCAGCTAT TTGAATTGGTATCAGCAGAAACCAGGAAAGG CCCCTAAACTCCTGATCTATGTTGCATCCAGTT TCCAAAGTGGGGTCCCATCAAGGTTCAGTGGC AGTGGATCTGGGACAGATTTCACTCTCACCAT CAGCAGTCTGCAACCTGATGATTTTGCAACTT ACTACTGTCAACAGAGTTACAGTCCCCCTCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGCATTCACCTTCAGTAGCTATGGCAT GCACTGGGTCCGCCAGACTCCAGGCAAGGGGCT GGAGTGGGTGGCAGTTATATGGCATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGACGAG CAGCTCGGGACCTTTGACTACTGGGGCCAGGGA ACCCTGGTCACCGTCTCCTCA |
| | | | SEQ ID NO: 25441 | SEQ ID NO: 29447 |
| | | AA | DIQMTQSPSSLSASVGDRITITCRASQSILSYLNW YQQKPGKAPKLLIYVASSFQSGVPSRFSGSGSGT DFTLTISSLQPDDFATYYCQQSYSPPLTFGGGTK VEIK | QVQLVESGGGVVQPGRSLRLSCAASAFTFSSYGMH WVRQTPGKGLEWVAVIWHDGSNKYYADSVKGRF TISRDNSKNTLYLQMNSLRAEDTAVYYCARDEQLG TFDYWGQGTLVTVSS |
| | | | SEQ ID NO: 25442 | SEQ ID NO: 29448 |

FIGURE 50

| | | | | |
|---|---|---|---|---|
| iPS:434167 | 21-225_50F3 | NA | GACATCCAGATGACCCAGTCTCCATCTTCCGT GTCTGCATCTGTAGGAGACAGAGTCACCATCA CTTGTCGGGCGAGTCAGGATATTAGCAGCTGG TTGGCCTGGTTTCAGCAGAAACCAGGGAAAGC CCCTAAGCTCCTGATCTATGCTGCATCCAGTTT GCAAAATGGGGTCCCGTCAAGGTTCAGCGGCA GTGGATCTGGGACAGATTTCACTCTCACCATC AGCAGCCTGCAGCCTGAAGATTTTGCAACTTA CTATTGCCAACAGACTAACAGTTTCCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGAACCTATGCCAT GACCTGGGTCCGCCAGGCTCCAGGGAAGGGGCT GGAGTGGGTCTCAGCTATCAGTGGTAGTGGTGTT AACTCATTCTACGCAGACTCCGTGAAGGGCCGGT TCACCATCTCCAGAGACAATTCCGAGAACACGCT GTATCTGCAAATGAACAGCCTGAGAGCCGAGGA CACGGCCGTATATTACTGTGCGAAAGCAAGGGC AGTGGCTGGGTCACACTGGTTCGACCCCTGGGGC CAGGGAACCCTGGTCACCGTCTCCTCA |
| | | | SEQ ID NO: 25443 | SEQ ID NO: 29449 |
| | | AA | DIQMTQSPSSVSASVGDRVTITCRASQDISSWLA WFQQKPGKAPKLLIYAASSLQNGVPSRFSGSGS GTDFTLTISSLQPEDFATYYCQQTNSFPFTFGPGT KVDIK | EVQLLESGGGLVQPGGSLRLSCAASGFTFRTYAMT WVRQAPGKGLEWVSAISGSGVNSFYADSVKGRFTI SRDNSENTLYLQMNSLRAEDTAVYYCAKARAVAG SHWFDPWGQGTLVTVSS |
| | | | SEQ ID NO: 25444 | SEQ ID NO: 29450 |
| iPS:434169 | 21-225_50C4 | NA | GACATCCAGATGACCCAGTCTCCATCCTCCCT GTCTGCATCTGTAGGAGACAGAGTCACCCTCA CTTGCCGGGCAAGTCAGGGCATTAGAAATGAT TTAGGCTGGTATCAGCAGAAACCAGGGGATAGC CCCTAAGCGCCTGATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATAATCGTTACCCATTCAC TTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATATGGTATGAAGAAAC TAATAAATACTATGCAGACTCCGTGAAGGGCCG ATTCACCATCTCCAGAGACAATTCCAAGAACACG CTGTATCTGCAAATGAACAGCCTGAGAGGCGAG GACACGGCTGTGTATTACTGTGCGAGAGAAGTGG GGTTCCTGAATGACTACTGGGGCCAGGGAATCCT GGTCACCGTCTCCTCA |
| | | | SEQ ID NO: 25445 | SEQ ID NO: 29451 |

FIGURE 50

| | | | | |
|---|---|---|---|---|
| | | AA | DIQMTQSPSSLSASVGDRVTLTCRASQGIRNDLG WYQQKPGIAPKRLIYAASSLQSGVPSRFSGSGSG TEFTLTISSLQPEDFATYYCLQHNRYPFTFGPGTK VDIK | QVQLVESGGGVVQPGRSLRLSCAASGFTFSDYGM HWVRQAPGKGLEWVAVIWYEETNKYYADSVKGR FTISRDNSKNTLYLQMNSLRGEDTAVYYCAREVGF LNDYWGQGILVTVSS |
| | | | SEQ ID NO: 25446 | SEQ ID NO: 29452 |
| iPS:434171 | 21-225_50G4 | NA | GACATCCAGATGACCCAGTCTCCAACCTCCCT GTCTGCATCTGTGGGAGACAGAGTCACCATCA CTTGCCAGGCGAGTCAGGACATTACCAACTTT TTAAATTGGTATCAGCAGAAACCAGGGAAAG CCCCTAAGCTCCTGATCTACGATGCCTCCAATT TGGAAACAGGGGTCCCATCAAGGTTCAGTGGA AGTGGATCTGGGACAGATTTTACTTTCACCAT CAGCAGCCTTCAGCCTGAAGATATTGCAACAT ATTACTGTCAACAGTATGATAATCTGATCACC TTCGGCCAAGGGACACGACTGGAGATTAAA | CAGGTGCAGCTGGTGCAGTCTGGGGGCTGAGGTG AAGGAGCCTGGGGCCTCAGTGAAGGTTTCCTGCA AGGCATCTGGATACACCTTCACCAGTTACTATAT ACACTGGGTGCGACAGGCCCCTGGACAAGGGCT TGAGTGGATGGGAGTAATCAACCCTAGTAATGGT AGAACAAGCTACGCACAGAAGTTCCAGGGCAGA GTCACCATGACCAGGGACACGTCCACGAGCACA GTCTACATGGAGCTGAGCAGCCTGAGATCTGAG GACACGGCCGTGTATTACTGTGCGAGAGATCGA GGAGATGGTTACTACTTCTACTACGGTATGGACG TCTGGGGCCAAGGGACCACGGTCACCGTCTCCTC A |
| | | | SEQ ID NO: 25447 | SEQ ID NO: 29453 |
| | | AA | DIQMTQSPTSLSASVGDRVTITCQASQDITNFLN WYQQKPGKAPKLLIYDASNLETGVPSRFSGSGS GTDFTFTISSLQPEDIATYYCQQYDNLITFGQGTR LEIK | QVQLVQSGAEVKEPGASVKVSCKASGYTFTSYYIH WVRQAPGQGLEWMGVINPSNGRTSYAQKFQGRVT MTRDTSTSTVYMELSSLRSEDTAVYYCARDRGDG YYFYYGMDVWGQGTTVTVSS |
| | | | SEQ ID NO: 25448 | SEQ ID NO: 29454 |

FIGURE 50

| iPS:434175 | 21-225_55A11 | NA | GACATCCAGATGACCCAGTCTCCATCCTCACT GTCTGCATCTGTAGGAGACAGAGTCACCATCA CTTGTCGGGCGAGTCAGGACATTAACATTTAT TTAGCCTGGTTTCAGCAGAAACCAGGGAAAGC CCCTAAGTCCCTGATCTATGCTGCATCCAGTTT GCAAAGTGGGGTCCCATCGAAGTTCAGCGGCA GTGGATCTGGGACAGATTTCACTCTCACCATC AGCAGTCTGCAGCCTGAAGATTTTGCAACTTA TTACTGCCAACAGTATAATAGTTATCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGACTCCAGGCAAGGGGCT GGAGTGGGTGGCAGTTATATCATATGTTGGAAGT ACTAAATACTATGCAGACTCCGTGAGGGGCCGAT TCACCATCTCCAGAGACAATTCCAAGAACACGCT GTATCTGCAAATGAACAGCCTGAGAACTGAGGA CACGGCTGTGTATTACTGTGCGAGAGGGGAGAGGT CGATATAGTGACTACGGTCATGATGCTTTTGATA TCTGGGGCCAAGGGACAATGGTCACCGTCTCTTC A |
| | | | SEQ ID NO: 25449 | SEQ ID NO: 29455 |
| | | AA | DIQMTQSPSSLSASVGDRVTITCRASQDINIYLA WFQQKPGKAPKSLIYAASSLQSGVPSKFSGSGSG TDFTLTISSLQPEDFATYYCQQYNSYPLTFGGGT KVEIK | QVQLVESGGGVVQPGRSLRLSCAASGFTFSSYGMH WVRQTPGKGLEWVAVISYVGSTKYYADSVRGRFT ISRDNSKNTLYLQMNSLRTEDTAVYYCARGRGRYS DYGHDAFDIWGQGTMVTVSS |
| | | | SEQ ID NO: 25450 | SEQ ID NO: 29456 |
| iPS:434177 | 21-225_56A1 | NA | GACATCGTCATGACCCAGTCTCCAGACTCCCT GGCTGTGTCTCTGGGCGAGAGGGGCCACCATCA ACTGCAAGTCCAGCCAGAGTGTTTTACATAGT TCCAACAATAAGAACTACTTAGTTTGGTACCA GCAGAGACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTACAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGCTGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCATATAGCAGT GGCTGGTACGTTTTTGACTACTGGGGCCAGGGAA CCCTGGTCACCGTCTCCTCA |
| | | | SEQ ID NO: 25451 | SEQ ID NO: 29457 |

FIGURE 50

| | | | DIVMTQSPDSLAVSLGERATINCKSSQSVLHSSN NKNYLVWYQQRPGQPPNLLIYWASTRESGVPD RFSGSGSGTDFTLTISSLQAEDVAVYYCQQYYST PPTFGQGTKVEIK | QVQLVQSGAEVKKPGASVKVSCKASGYTFTNYDIN WVRQATGQGLEWLGWMHPNSGNTGYAQKFQGR VTMTRNTSISTAYMELSSLRSEDTAVYYCAYSSGW YVFDYWGQGTLVTVSS |
| | | AA | | |
| | | | SEQ ID NO: 25452 | SEQ ID NO: 29458 |
| IPS:434179 | 21-225_56F1 | NA | GACATCCAGATGACCCAGTCTCCATCCTCCCT GTCTGCATCTGTAGGAGACAGAGTCACCATCA CTTGCCGGGCAAGTCAGGACATTAGAAATAAT TTAGGCTGGTATCAGCAGAAACCAGGGAAAG CCCCTAAGCGCCTGATCAATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGGATAATAGTCACCCGTT CACTTTCGGCGGAGGGACCAAGGTGGAGATC AAA | GAGGTGCAGCTGGTGGAGTCTGGGGGAGGCCTG GTCAAGTTTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTACT TACATATACTACGGAGACTCAGTGAAGGGCCGA TTCACCATCTCCCGAGACAACGCCAAGAACTCAC TATATCTGCAGATGAACAGCCTGAGAGCCGAGG ACACGGCTGTGTATTACTGTGCGAGAGATCGGGG CAGCAGCTGGGGCCAGGGAACCCTGGTCACCGT CTCCTCA |
| | | | SEQ ID NO: 25453 | SEQ ID NO: 29459 |
| | | AA | DIQMTQSPSSLSASVGDRVTITCRASQDIRNNLG WYQQKPGKAPKRLINAASSLQSGVPSRFSGSGS GTEFTLTISSLQPEDFATYYCLQDNSHPFTFGGGT KVEIK | EVQLVESGGGLVKFGGSLRLSCAASGFTFSSYSMN WVRQAPGKGLEWVSSISSSSTYIYYGDSVKGRFTIS RDNAKNSLYLQMNSLRAEDTAVYYCARDRGSSW GQGTLVTVSS |
| | | | SEQ ID NO: 25454 | SEQ ID NO: 29460 |

1845

FIGURE 50

| iPS:434181 | 21-225_56B2 | NA | GACATCCAGATGACCCAGTCTCCATCCTCCCT GTCTGCATCTGTAGGAGACAGAGTCACCATCA CTTGCCGGGCAAGTCAGAGTTTTAGCCACTAT TTAAATTGGTATCAGCAGAAACCAGGGAAAG CCCCTAACCTCCTGATCTTTGCTGTATCCAGTT TGCAAAGTGGGGTCCCATCAAGGTTCAGTGGC AGTGGATCTGGGACAGATTTCACTCTCACCAT CAGTAGTCTGCAACCTGAAGATTTTGCAATTT ACTTCTGTCAACAGAGTTACAGTACCCCATTC ACTTTCGGCCCTGGGACCAAAGTGGATATCAA A | GAGGTGCAGTTGTTGGAGTCTGGGGGAGGCTTGA TACAGCCTGGGGGGTCCCTGAGACTCTCCTGTGC AGCCTCTGGATTCACCTTTAGGAGCTATGCCATG AGCTGGGTCCGCCAGGCTCCAGGGAAGGGGCTG GAGTGGGTCTCAGCTATTAGTGGTAGTGGTGGTA ACACATTCTACGCAGACTCCGTGAAGGGCCGGTT CACCATCTCCAGAGACAATTCCAAGAACACGCTG TATTTGCAAATGAACACCCTGAGAGCCGACGAC ACGGCCGTATATTACTGTGCGAAAAAGGTCGTGG ATACAGCCATGGCTCTTGACTACTGGGGCCAGGG AACCCTGGTCACCGTCTCCTCA |
| | | | SEQ ID NO: 25455 | SEQ ID NO: 29461 |
| | | AA | DIQMTQSPSSLSASVGDRVTITCRASQSFSHYLN WYQQKPGKAPNLLIFAVSSLQSGVPSRFSGSGSG TDFTLTISSLQPEDFAIYFCQQSYSTPFTFGPGTK VDIK | EVQLLESGGGLIQPGGSLRLSCAASGFTFRSYAMS WVRQAPGKGLEWVSAISGSGGNTFYADSVKGRFTI SRDNSKNTLYLQMNTLRADDTAVYYCAKKVVDT AMALDYWGQGTLVTVSS |
| | | | SEQ ID NO: 25456 | SEQ ID NO: 29462 |
| iPS:434187 | 21-225_56A5 | NA | GACATCCAGATGACCCAGTCTCCATCCTCCCT GTCTGCATCTGTAGGAGACAGAGTCACCATCA CTTGCCGGGCAAGTCAGGACATTAGAAATCTT TTAGGCTGGTATCAGCAGAAACCAGGGAAAG CCCCTAAGCGCTTGATCTATGCTGCATCCAGTT TGCAAAGTGGGGTCCCATCAAGGTTCAGCGGC AGTGGATCTGGGACAGAGTTCACTCTCACAAT CAGCAGCCTGCAGCCTGAAGATTTTGCAACTT ATTACTGTCTACAGTATAATAGTTACCCATTCA CTTTCGGCCCTGGGACCAAAGTGGATATCAGA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT ATATCTGCAAATGAACAGCCTGAGAGCCGAGGA CACGGCTGTGTATTACTGTGCGAGAGTGGCTACT TTTGACTACTGGGGCCAGGGAACCCTGGTCACCG TCTCCTCA |
| | | | SEQ ID NO: 25457 | SEQ ID NO: 29463 |

FIGURE 50

| | | | | |
|---|---|---|---|---|
| | | AA | DIQMTQSPSSLSASVGDRVTITCRASQDIRNLLG WYQQKPGKAPKRLIYAASSLQSGVPSRFSGSGS GTEFTLTISSLQPEDFATYYCLQYNSYPFTFGPGT KVDIR | EVQLVESGGGLVKPGGSLRLSCAASGFTFSSYSMN WVRQAPGKGLEWVSSISGSSSYIYYADSVKGRFTIS RDNAKNSLYLQMNSLRAEDTAVYYCARVATFDY WGQGTLVTVSS |
| | | | SEQ ID NO: 25458 | SEQ ID NO: 29464 |
| IPS:434189 | 21-225_56E5 | NA | GACATCCAGATGACCCAGTCTCCATCTTCCGT GTCTGCATCTGTAGGCGACAGAGTCACCATCA CTTGTCGGGCGAGTCAGGGTATTAGGAAGTGG TTAGCCTGGTATCAGCAGAAACCAGGGAAAG CCCCTAAGCTCCTGATCTATGCTGCATCCAGTT TGCAAAGTGGGGTCCCGTCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGCC AGGCTTCTGGATACACCTTCACCGGCTACCATAT GCACTGGGTGCGACAGGCCCCTGGACAAGGGCT TGAGTGGATGGGATGGATCAACCCTAACAATAAT GCCACAAACTATGCACAGAAGTTTCAGGGCAGG GTCACCATGACCAGGGACACGTCCATCAGCACA GCCTACATGGAACTGAGGAGGCTGAGATCTGAC GACACGGCCGTGTATCACTGTGCGAGAGATGGC ACCTCGTCTTTTGACTACTGGGGCCAGGGAACCC TGGTCACCGTCTCCTCA |
| | | | SEQ ID NO: 25459 | SEQ ID NO: 29465 |
| | | AA | DIQMTQSPSSVSASVGDRVTITCRASQGIRKWLA WYQQKPGKAPKLLIYAASSLQSGVPSRFSGSGS GTDFTLTISSLQPEDFATYYCQQANSFPFTFGPGT KVDIK | QVQLVQSGAEVKKPGASVKVSCQASGYTFTGYHM HWVRQAPGQGLEWMGWINPNNNATNYAQKFQGR VTMTRDTSISTAYMELRRLRSDDTAVYHCARDGTS SFDYWGQGTLVTVSS |
| | | | SEQ ID NO: 25460 | SEQ ID NO: 29466 |

1847

FIGURE 50

| iPS:434191 | 21-225_56B6 | NA | GACATCCAGATGACCCAGTCTCCATCCTCCCT GTCTGTATCTGTAGGAGACAGAGTCACCATCA CTTGCCGGGCAAGTCAGAGCATTTTCAGATAT TTAAATTGGTATCAGCAGAAACCAGGAAGAG CCCCTAAGCTCCTGATCTTTGCTGCATCCAGTT TCCAAAGTGGGGTCCCATCAAGGTTCAGTGGC AGTGGATCTGGGACAGACTTCACTCTCACCAT CAGCAGCCTACAACCTGAAGATTTTGCAACTT ACTACTGTCAACAGACTTACAGTCCCCCTCTC ACTTTCGGCGGAGGGACCAAGGTGGAGATCA AA | CAGGTGCAACTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGCATGATGGAAG TAATAAATATTATGTAGACTCCGTGAAGGGCCGA TTCACCATCTCCAGAGACAATTCCAAGAATACGC TGTATCTGCAAATGAACAGCCTGAGAGCCGAGG ACACGGCTGTGTATTACTGTGCGAGAGACGAGC AGCTCGGGACCTTTGACTACTGGGGCCAGGGAA CCCTGGTCACCGTCTCCTCA |
|---|---|---|---|---|
| | | | SEQ ID NO: 25461 | SEQ ID NO: 29467 |
| | | AA | DIQMTQSPSSLSVSVGDRVTITCRASQSIFRYLN WYQQKPGRAPKLLIFAASSFQSGVPSRFSGSGSG TDFTLTISSLQPEDFATYYCQQTYSPPLTFGGGTK VEIK | QVQLVESGGGVVQPGRSLRLSCAASGFTFSSYGMH WVRQAPGKGLEWVAVIWHDGSNKYYVDSVKGRF TISRDNSKNTLYLQMNSLRAEDTAVYYCARDEQLG TFDYWGQGTLVTVSS |
| | | | SEQ ID NO: 25462 | SEQ ID NO: 29468 |
| iPS:434193 | 21-225_56C6 | NA | GACATCCAGATGACCCAGTCTCCATCTTCCGT GTCTGCATCTGTGGGAGACAGAGTCACCATCA CTTGTCGGGCGAGTCAGGGTATTAGCAGCTGG TTAGCCTGGTATCAGCAGAAATCAGGGAATGC CCCTAAGCTCCTGATCTATGCTGCATCCAGATT GCAAAGTGGGGTCCCATCAAGGTTCAGCGGCA GTGGATCTGGTACATATTTCACTCTCATTATCA GCAGCCTGCAGTCTGAAGATTTTGCAACTTAC TATTGTCAACAGGCTAACAGTTTCCCATTCACT TTCGGCCCTGGGACCAAAGTGGATATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGACTACCATA TGCACTGGGTGCGACAGGCCCCTGGACAAGGGC TTGAGTGGATGGGATGGATCAACCCTAACAGAG GTGGCACAAATTATGTACAGAAGTTTCAGGGTAG GGTCGCCATGACCAATGACACGTCCATCAGCACA GCCTATATGGAGCTGAGTGGGCTGAGATCTGACG ACACGGCCGTGTATTACTGTGCGAGAGATGGCAC CTCGTCTTTTGACTATTGGGGCCGGGGAACCCTG GTCACCGTCTCCTCA |
| | | | SEQ ID NO: 25463 | SEQ ID NO: 29469 |

FIGURE 50

| | | | | |
|---|---|---|---|---|
| | | AA | DIQMTQSPSSVSASVGDRVTITCRASQGISSWLA WYQQKSGNAPKLLIYAASRLQSGVPSRFSGSGS GTYFTLIISSLQSEDFATYYCQQANSFPFTFGPGT KVDIK | QVQLVQSGAEVKKPGASVKVSCKASGYTFTDYHM HWVRQAPGQGLEWMGWINPNRGGTNYVQKFQGR VAMTNDTSISTAYMELSGLRSDDTAVYYCARDGTS SFDYWGRGTLVTVSS |
| | | | SEQ ID NO: 25464 | SEQ ID NO: 29470 |
| iPS:434195 | 21-225_56F6 | NA | GACATCCAGATGACCCAGTCTCCATCTTCTGT GTGTGCATATGTAGGAGACAGAGTCACCATCA CTTGTCGGGTGAGTCAGGATATTAGCAAATGG TTAGCCTGGTTTCAGCAGAAACCAGGGAAAGC CCCTAAATTCTTGATATATGTTGCATCCGGTTT GCAAAGTGGGGTCCCATCAAGGTTCAGCGGCA GTGGATCTGGGACAGATTTCACTTTCACCATC AGCAGCCTGCAGCCTGAAGATTTTGCAACTTA CTATTGTCAACAGGCTAACAGTTTCCCATTCA CTTTCGGCCCTGGGACCAAAGTGGATATCAAA | CAGGTGCAGCTGGTGCAGTCTGGGGGCTGAGGTG AAGAAGCCTGGGGCCTCAGTGAAGGTCTCCTGC AAGGCTTCTGGATTCACCTTCACCGACTACTATA TGCACTGGGTGCGACAGGCCCCTGGACAAGGGC TTGAGTGGATGGGATGGATCAACCCTAACAGTGG TGGCACAAACTATGCACAGAGGTTTCAGGGCAG GGTCACCATGACCAGGGACACGTCCATCAGCAC AGCCTACATGGAACTGAGCAGACTGAGATCTGA CGACACGGCCGTGTATTACTGTACGAGAGAGGG AGCAACTCGTCCGACGGGTTTTGACTACTGGGGC CAGGGAACCCTGGTCACCGTCTCCTCA |
| | | | SEQ ID NO: 25465 | SEQ ID NO: 29471 |
| | | AA | DIQMTQSPSSVCAYVGDRVTITCRVSQDISKWLA WFQQKPGKAPKFLIYVASGLQSGVPSRFSGSGSG TDFTFTISSLQPEDFATYYCQQANSFPFTFGPGTK VDIK | QVQLVQSGAEVKKPGASVKVSCKASGFTFTDYYM HWVRQAPGQGLEWMGWINPNSGGTNYAQRFQGR VTMTRDTSISTAYMELSRLRSDDTAVYYCTREGAT RPTGFDYWGQGTLVTVSS |
| | | | SEQ ID NO: 25466 | SEQ ID NO: 29472 |

FIGURE 50

| iPS:434197 | 21-225_56C7 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAATTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTATCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCCACTATATAAACTGGGTGCGACAGGCCCCTGGACAAGGGCTTGAGTGGATGGCATGGGTCAACCCTAACAGTGGTGGCACAAACTCTGCACAGAAGTTTCAGGGCAGGGTCACCATGACCAGGGACACGTCCATCAGCACAGTCTACATGGAGCTGAGCAGGCTGAGATCTGACGACACGGCCGTGTATTACTGTGCGAGGGGAGGGCAGCTCGGCGGGTTTAACTACTACTACTACGGTATGGACGTCTGGGGCCAAGGGACCACGGTCACCGTCTCCTCA |
| | | | SEQ ID NO: 25467 | SEQ ID NO: 29473 |
| | | AA | DIQMTQSPSSLSASVGDRVTITCRASQGIRNDLGWYQQKPGKAPKRLIYTASNLQSGVPSRFSGSGSGTEFTLTISSLQPEDFATYYCLQHNSYPWTFGQGTKVEIK | QVQLVQSGAEVKKPGASVKVSCKASGYTFTGHYINWVRQAPGQGLEWMAWVNPNSGGTNSAQKFQGRVTMTRDTSISTVYMELSRLRSDDTAVYYCARGGQLGGFNYYYYGMDVWGQGTTVTVSS |
| | | | SEQ ID NO: 25468 | SEQ ID NO: 29474 |
| iPS:434199 | 21-225_59F11 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATCGTTACCCTTTCACTTTCGGCCCTGGGACCAAAGTGGATTTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGAAAGTAATAAACACTATGCAGACTCCGTGAAGGGCCGATTCACCATCTCCAGAGACAATTCCAAGACCACGCTGTATCTGCAAATGAGCAGCCTGAGAGCCGAGGACACGGCTGTGTATTATTGTTCGAGAGAACTGGGGATGAACGGAGACTACTGGGGCCAGGGAACCCTGGTCACCGTCTCCTCA |
| | | | SEQ ID NO: 25469 | SEQ ID NO: 29475 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNRYPFTFGPGT KVDFK | QVQLVESGGGVVQPGRSLRLSCAASGFTFSNYGM HWVRQAPGKGLEWVAVIWYDESNKHYADSVKGR FTISRDNSKTTLYLQMSSLRAEDTAVYYCSRELGM NGDYWGQGTLVTVSS |
| | | | SEQ ID NO: 25470 | SEQ ID NO: 29476 |
| iPS:434201 | 21-225_59A12 | NA | GATATTGTGATGACCCAGACTCCACTCTCTCT GTCCGTCACCCCTGGACAGCCGGCCTCCATCT CCTGCAAGTCTAGTCAGAGCCTCCAGCATGGT GAAGGAAAGACCTATTTGTATTGGTACGTGCA GAAGCCAGGCCAGCCTCCACAGCTCCTGATCT ATGAAGTTTCCTATCGGTTTTCTGGAGTGCCA GATAGGTTCAGTGGTAGTGGGTCAGGGACAG ATTTCACACTGAAAATCAGCCGGGTGGAGGTT GAGGATGTTGGGGTTTATTACTGCATGCAAAG TACACAGCTTCCGCTCACCTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGCGGTATAG CAGCAGCTGGGACGGGGGGTATGGACGTCTGGGG CCAAGGGACCACGGTCACCGTCTCCTCA |
| | | | SEQ ID NO: 25471 | SEQ ID NO: 29477 |
| | | AA | DIVMTQTPLSLSVTPGQPASISCKSSQSLQHEG KTYLYWYVQKPGQPPQLLIYEVSYRFSGVPDRF SGSGSGTDFTLKISRVEVEDVGVYYCMQSTQLP LTFGGGTKVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WDGGMDVWGQGTTVTVSS |
| | | | SEQ ID NO: 25472 | SEQ ID NO: 29478 |

FIGURE 50

| iPS:434203 | 21-225_60E2 | NA | GACATCCAGATGACCCAGTCTCCATCCTCCCT GTCTGCATCTGTAGGAGACAGAGTCACCATCA CTTGCCGGGCAAGTCAGGGCATTAGAAAAGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAATGACTATGGCAT GCACTGGGTCCGCCAGGCTCCAGGCAAGGGGCT GGAGTGGGTGGCAATTATATGGTATGATGAAAAT AATAAATACTATGCAGACTCCGTGAAGGGCCGA TTCACCATCTCCAGAGACAATTCCAAGAACACGC TGTATCTGCAAATGAGCAGCCTGAGAGCCGAGG ACACGGCTGTATATTACTGTGCGAGAGATGTTCT GGACCCTTTTGACTACTGGGGCCAGGGAACCCTG GTCACCGTCTCCTCA |
|---|---|---|---|---|
| | | | SEQ ID NO: 25473 | SEQ ID NO: 29479 |
| | | AA | DIQMTQSPSSLSASVGDRVTITCRASQGIRKDLG WYQQKPGKAPKRLIYAASSLQSGVPSRFSGSGS GTEFTLTISSLQPEDFATYYCLQHNSYPWTFGQG TKVEIK | QVQLVESGGGVVQPGRSLRLSCAASGFTFNDYGM HWVRQAPGKGLEWVAIIWYDENNKYYADSVKGR FTISRDNSKNTLYLQMSSLRAEDTAVYYCARDVLD PFDYWGQGTLVTVSS |
| | | | SEQ ID NO: 25474 | SEQ ID NO: 29480 |
| iPS:434205 | 21-225_60G2 | NA | GATATTGTGATGACCCAGACTCCACTCTCTCT GTCCGTCACACCTGGACAGTCGGCCTCCATCT CCTGCAAGTCTAGTCAGAGCCTCCTGCATAGT GAAGGAAAGACCTATTTGTATTGGTACCTGCA GAAGCCAGGCCAGCCTCCACAGCTCCTGATCT ATGAAGTTTCCAACCGGATCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGAAGCTGGACGGGAGGCATGGACGTCTGGGG CCAAGGGACCACGGTCACCGTCTCCTCA |
| | | | SEQ ID NO: 25475 | SEQ ID NO: 29481 |

FIGURE 50

| | | | | |
|---|---|---|---|---|
| | | AA | DIVMTQTPLSLSVTPGQSASISCKSSQSLLHSEGK TYLYWYLQKPGQPPQLLIYEVSNRISGVPDRFSG SGSGTDFTLKISRVEAEDVGVYYCMQSIQLPLTF GGGTKVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSRS WTGGMDVWGQGTTVTVSS |
| | | | SEQ ID NO: 25476 | SEQ ID NO: 29482 |
| IPS:434207 | 21-225_60A3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATTTAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCTTTCA CTTTCGGCCCTGGGACCAAAGTGGATATCAAA | CAGGTGCAACTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGAAGAAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTATATTACTGTGCGAGAGAACTGG GGATGACCGGAGACTACTGGGGCCAGGGAACCC TGGTCACCGTCTCCTCA |
| | | | SEQ ID NO: 25477 | SEQ ID NO: 29483 |
| | | AA | DIQMTQSPSSLSASVGDRVTITCRASQGIRNDLG WYQQKPGKAPKRLIYAAFSLQSGVPSRFSGSGS GTEFTLTISSLQPEDFATYYCLQHNSYPFTFGPGT KVDIK | QVQLVESGGGVVQPGRSLRLSCAASGFTFSSYGMH WVRQAPGKGLEWVAVIWYEESNKYYADSVKGRF TISRDNSKNTLYLQMNSLRAEDTAVYYCARELGMT GDYWGQGTLVTVSS |
| | | | SEQ ID NO: 25478 | SEQ ID NO: 29484 |

EP 4 435 105 A2

FIGURE 50

| iPS:434209 | 21-225_60C3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATTCTGCATCCAGTT TACAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGTGG ACGTTCGGCCTAGGGACCAAGGTGGAAATCA AA | CAGGTGCAACTAGTGCAGTCTGGGGGCTGAGGTG AAGAAGCCTGGGGCCTCAGTGAAGGTCTCCTGC AAGGCTTCTGGATACAGTTTCACCGGCCACTATA TACACTGGGTGCGACAGGCCCCTGGACAAGGGC TTGAGTACATGGGATGGATCAACCCTAACAGCG GTGGCACAAACTATGTACAGAAATTTCAGGGCA GGGTCACCATGACCAGGGACACGTCCATCAGCA CAGCCTACATGGAGCTGAGCAGGCTGAGATCTG ACGACACGGCCATATATTACTGTTCGAGAGGGG GCCTACTGGGAGCTACCAACTACTATTATTACGG TATGGACGTCTGGGGCCAAGGGACCACGGTCAC CGTCTCCTCA |
|---|---|---|---|---|
| | | | SEQ ID NO: 25479 | SEQ ID NO: 29485 |
| | | AA | DIQMTQSPSSLSASVGDRVTITCRASQGIRNDLG WYQQKPGKAPKRLIYSASSLQSGVPSRFSGSGSG TEFTLTISSLQPEDFATYYCLQHNSYPWTFGLGT KVEIK | QVQLVQSGAEVKKPGASVKVSCKASGYSFTGHYIH WVRQAPGQGLEYMGWINPNSGGTNYVQKFQGRV TMTRDTSISTAYMELSRLRSDDTAIYYCSRGGLLGA TNYYYYGMDVWGQGTTVTVSS |
| | | | SEQ ID NO: 25480 | SEQ ID NO: 29486 |
| iPS:434211 | 21-225_60F3 | NA | GACATCGTGATGACCCAGTCTCCAGACTCCCT GACTGTGTCTCTGGGCGAGAGGGGCCACCATCA ACTGCAAGTCCAGCCAGAGTGTTTTATACAGC TCCAACAATAAGAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAATTTATTACTGTCAGCAA TATTATAGTACTCCGTGCAGTTTTGGCCAGGG GACCAAGCTGGAGATCAAC | CAGGTGCTGCTGGTGCAGTCTGGGGGCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGCACCCTAACAGTGGT AACACAGGCTATGCACAGAAGTTCCAGGGCAGA GTCACCATGACCAGGAACACCTCCATAAGCACA GCCTACATGGAGCTGAGCAGCCTGAGATCTGAG GACACGGCCGTGTATTACTGTGCGTATAGCAGTG GCTGGTACTTCTTTGACTACTGGGGCCAGGGAAC CCTGGTCACCGTCTCCTCA |
| | | | SEQ ID NO: 25481 | SEQ ID NO: 29487 |

1854

FIGURE 50

| | | | | |
|---|---|---|---|---|
| | | AA | DIVMTQSPDSLTVSLGERATINCKSSQSVLYSSN NKNYLAWYQQKPGQPPKLLIYWASTRESGVPD RFSGSGSGTDFTLTISSLQAEDVAIYYCQQYYSTP CSFGQGTKLEIN | QVLLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YFFDYWGQGTLVTVSS |
| | | | SEQ ID NO: 25482 | SEQ ID NO: 29488 |
| IPS:434213 | 21-225_60A4 | NA | GACATCCAGATGACCCAGTCTCCATCCTCACT GTCTGCATCTGTAGGAGACAGAGTCACCATCA CATGTCGGGCGAGTCAGGGCATTAGCAATTAC TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGTCTGAAGATTTTGCAACGTA CTACTGCCAACAATATAAAAGTCACCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGTCAT GAGCTGGGTCCGCCAGGCTCCAGGGAAGGGGCT GGAGTGGGTCTCATCTATTAGTGGTAGTGGTGGT TGGACAAACTACGCAGACTCCGTGAAGGGCCGA TTCACCACCTCCAGAGACAATTCCAAGAACACGC TGTATCTGCAAATGAACAGCCTGAGAGCCGAGG ACACGGCCGTTTATTACTGTGCGAGACTAACTGG ATTTGACTATTGGGGCCAGGGAACCCTGGTCACC GTCTCCTCA |
| | | | SEQ ID NO: 25483 | SEQ ID NO: 29489 |
| | | AA | DIQMTQSPSSLSASVGDRVTITCRASQGISNYLA WFQQKPGKAPKSLIYAASSLQSGVPSKFSGSGSG TDFTLTISSLQSEDFATYYCQQYKSHPFTFGPGT KVDIK | EVQLLESGGGLVQPGGSLRLSCAASGFTFSSYVMS WVRQAPGKGLEWVSSISGSGGWTNYADSVKGRFT TSRDNSKNTLYLQMNSLRAEDTAVYYCARLTGFD YWGQGTLVTVSS |
| | | | SEQ ID NO: 25484 | SEQ ID NO: 29490 |

FIGURE 50

| iPS:434215 | 21-225_60F7 | NA | GACATCCAGATGACCCAGTCTCCATCCTCACT GTCTGCATCTGTAGGAGACAGAGTCACCATCT CTTGTCGGGCGAGTCAGGTCATTAAGAATTAT TTAGTCTGGGTTCAGCAGAAACCAGGGAAAGC CCCTAAGTCCCTGATCTATGCTGCGTCCAGTTT GCAAAGTGGGGTCCCATCAACGTTCAGCGGCA GTGGATCTGGGACAGATTTCACTCTCACCATC AGCAGCCTGCAGCCTGAAGATTTTGCAACTTA TTACTGCCTACAGTTTCATAGTTACCCATTCAC TTTCGGCCCTGGGACCAAAATGGATATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGTCAT GAGCTGGGTCCGCCAGGCTCCAGGGAAGGGGCT GGAGTGGGTCTCAGGTATTAGTGGTAGTGGTAAT AGAACATACTACGCAGACTCCGTGAAGGGCCGG TTCACCATCTCCAGAGACAATTCCAAGAATACGC TGTATCTGCAAATGAACAGCCTGAGAGCCGAGG ACTCGGCCGTTTATTACTGTGGGAGTTTGGGGAT TGACTGGGGCCAGGGAACCCTGGTCACCGTCTCC TCA |
| | | | SEQ ID NO: 25485 | SEQ ID NO: 29491 |
| | | AA | DIQMTQSPSSLSASVGDRVTISCRASQVIKNYLV WVQQKPGKAPKSLIYAASSLQSGVPSTFSGSGSG TDFTLTISSLQPEDFATYYCLQFHSYPFTFGPGTK MDIK | EVQLLESGGGLVQPGGSLRLSCAASGFTFSSYVMS WVRQAPGKGLEWVSGISGSGNRTYYADSVKGRFTI SRDNSKNTLYLQMNSLRAEDSAVYYCGSLGIDWG QGTLVTVSS |
| | | | SEQ ID NO: 25486 | SEQ ID NO: 29492 |
| iPS:434217 | 21-225_60E8 | NA | GACATCCAGATGACCCAGTCTCCATCCTCCCT GTCTGCATCTGTAGGAGACAGAGTCACCTTCA CTTGCCGGGCAAGTCAGGGCATTAGAAATGAT TTAGGCTGGTTTCAGCAGAAACCAGGGAAAGC CCCTAAGCGCCTGATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATAGTAGTTACCCGCTCA CTTTCGGCGGAGGGACCAAGGTGGAGATCAA A | CAGGTGCAGCTGCAGGAGTCGGGCCCAGGACTG GTGAGGCCTTCGGCCGACCCTGTCCCTCACCTGCA CTGTCTCTGGTGGCTCCATCAGCAGGAGTAGTTA CTACTGGGGCTGGATCCGCCAGCCCCCAGGGAA GGGACTGGAGTGGATTGGGAGTATCTATTATAGT GGGAGCGCCTCCTACAACCCGTCCCTCAAGAGTC GAGTCACCATATCCGTAGACACGTCCGAGAACC AATTCTCCCTGAGGCTGAGCTCTGTGACCGCCGC AGACACGGCTGTGTATTACTGTGCGAGACTGGAC AGTGGCTGGTCGTTTGACTACTGGGGCCAGGGAA CCCTGGTCACCGTCTCCTCA |
| | | | SEQ ID NO: 25487 | SEQ ID NO: 29493 |

FIGURE 50

| | | | | |
|---|---|---|---|---|
| | | AA | DIQMTQSPSSLSASVGDRVTFTCRASQGIRNDLG WFQQKPGKAPKRLIYAASSLQSGVPSRFSGSGSG TEFTLTISSLQPEDFATYYCLQHSSYPLTFGGGTK VEIK | QVQLQESGPGLVRPSATLSLTCTVSGGSISRSSYYW GWIRQPPGKGLEWIGSIYYSGSASYNPSLKSRVTIS VDTSENQFSLRLSSVTAADTAVYYCARLDSGWSFD YWGQGTLVTVSS |
| | | | SEQ ID NO: 25488 | SEQ ID NO: 29494 |
| iPS:434219 | 21-225_60E9 | NA | GAAATAGTGATGACGCAGTCTCCAGCCACCCT GTCTGTGTCTCCAGGGGAAAGAGCCACCCTCT CCTGTAGGGCCAGTCAGAGTGTTAGCAGTTCC TTAGCCTGGTACCGGCAGAAACCTGGCCAGGC TCCCAGGCTCCTCATCTATGGTGCATCCACCA GGGCCACTGGTATCCCGGCCAGGTTCAGTGGC AGTGGGTCTGGGACAGAGTTCACTCTCACCAT CAGCAGCCTGCAGTCTGAAGATTTTGCAGTTT ATTGCTGTCAGCAGTATAATAACTGGCCATTC ACTTTCGGCCCTGGGACCAAAATAGATATCAA A | GAGGTGCAGTTGTTGGAGTCTGGGGGAGGCTTGG TACAGCCTGGGGGGGTCCCTGAGACTCTCCTGTGC AGCCTCTGGATTCACCTTTAGCAGCTATGCCATG AGCTGGGTCCGCCAGGCTCCAGGGAAGGGGCTG GAGTGGGTCTCAGCTATTAGTGGTAGTGGTGGTA ACACATTCTACGCAGACTCCGTGAAGGGCCGGTT CACCATCTCCAGAGACAATTCCAAGAACACGCTG TTTCTTCAAATGAACAGCCTGAGAGCCGAGGACA CGGCCGTATATTACTGTGCGAAATTTTTCGGTAT AGTGGGAGCCGGGTACTTTGACTACTGGGGCCA GGGAACCCTGGTCACCGTCTCCTCA |
| | | | SEQ ID NO: 25489 | SEQ ID NO: 29495 |
| | | AA | EIVMTQSPATLSVSPGERATLSCRASQSVSSSLA WYRQKPGQAPRLLIYGASTRATGIPARFSGSGSG TEFTLTISSLQSEDFAVYCCQQYNNWPFTFGPGT KIDIK | EVQLLESGGGLVQPGGSLRLSCAASGFTFSSYAMS WVRQAPGKGLEWVSAISGSGGNTFYADSVKGRFTI SRDNSKNTLFLQMNSLRAEDTAVYYCAKFFGIVGA GYFDYWGQGTLVTVSS |
| | | | SEQ ID NO: 25490 | SEQ ID NO: 29496 |

FIGURE 50

| iPS:434221 | 21-225_60A11 | NA | GACATCCAGATGACCCAGTCTCCATCTTCCGT GTCTGCATCGTAGGAGACAGAGTCACCATCA CTTGTCGGGCGAGTCAGGTTATTAGCAACTGG TTAGCCTGGTATCAGCTGAAACCAGGGAAAGC CCCTAAGCTCCTGATCTATACTGCATCCAGTTT GCAAAGTGGGGTCCCATCAAGGTTCAGCGGCA ATGAATCTGGGACAGATTTCACTCTCACCATC AGCAGCCTGCAGCCTGAAGATTTTGCAACTTA CTATTGTCAACAGGCTAACAGTTTCCCGTGGA CGTTCGGCCAAGGGACCAAGGTGGAAATCAA A | GAGGTGCAGCTGTTGGAGTCTGGGGGAGGCTTG GTACAGCCGGGGGGGTCCCTGAGACTCTCCTGTG CAGCCTCTGGATTCACCTTTAGCAACTATGCCAT GACCTGGGTCCGCCAGGCTCCAGGGAAGGGGCT GGAGTGGGTCTCAGCTATTAGTGGTAGTGGTGGT AACACATTCTACGCAGACTCCGTGACGGGCCGG GTCACCATCTCCAGAGACAATTCCAAGAACACGC TGTATCTGCAAATGAACAGTCTGAGAGCCGAGG ACACGGCCGTATATTACTGTGCGAAACTGGGGA AGGACTACCACTACTACGGTATGGACGTCTGGGG CCAAGGGACCACGGTCACCGTCTCCTCA |
|---|---|---|---|---|
| | | | SEQ ID NO: 25491 | SEQ ID NO: 29497 |
| | | AA | DIQMTQSPSSVSASVGDRVTITCRASQVISNWLA WYQLKPGKAPKLLIYTASSLQSGVPSRFSGNESG TDFTLTISSLQPEDFATYYCQQANSFPWTFGQGT KVEIK | EVQLLESGGGLVQPGGSLRLSCAASGFTFSNYAMT WVRQAPGKGLEWVSAISGSGGNTFYADSVTGRVTI SRDNSKNTLYLQMNSLRAEDTAVYYCAKLGKDYH YYGMDVWGQGTTVTVSS |
| | | | SEQ ID NO: 25492 | SEQ ID NO: 29498 |
| iPS:434223 | 21-225_60C12 | NA | GACATTGTGATGACCCAGACTCCACTCTCTCT GTCCGTCACCCCTGGACAGCCGGCCTCCATCT CCTGCAAGTCTAGTCAGAGCCTCCTGCATAGT GAGGGAAAGACCTATTTGTATTGGTACCTGCA GAAGCCAGGCCAGCCTCCACAGTTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TATAAAGTATCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAAGGTATAGC AGAAGCTGGACGGGAGGTATGGACGTCTGGGGC CAGGGGACCACGGTCACCGTCTCCTCA |
| | | | SEQ ID NO: 25493 | SEQ ID NO: 29499 |

FIGURE 50

| | | | | |
|---|---|---|---|---|
| | | AA | DIVMTQTPLSLSVTPGQPASISCKSSQSLLHSEGK TYLYWYLQKPGQPPQFLIYEVSNRFSGVPDRFSG SGSGTDFTLKISRVEAEDVGIYYCMQSIKYPLTF GGGTKVEIK | QVQLVESGGGVVQPGRSLRLSCAASGFTFSSYGMH WVRQAPGKGLEWVAVIWYDGSNKYYVDSVKGRF TISRDNSKNTLYLQMNSLRAEDTAVYYCARRYSRS WTGGMDVWGQGTTVTVSS |
| | | | SEQ ID NO: 25494 | SEQ ID NO: 29500 |
| iPS:434225 | 21-225_60E12 | NA | GACATCCAGATGACCCAGTCTCCATCCTCCCT GTCTGCATCTGTAGGAGACAGAGTCACCATCA CTTGCCGGGCAAGTCAGAGCATTAGCAGCTAT TTAAATTGGTATCAGCAGAAACCAGGGAAAG CCCCTAAGCTCCTGATCTATGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCACCTT ACTACTGTCAACAGAGTTACAATATTTCATTC ACTTTCGGCCCTGGGACCAAAGTGGATATCAA A | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTACTTCTGG AGCTGGATCCGGCAGCCCGCCGGGAAGGGACTG GAGTGGATTGGGCGCATCTATACCAGGGGGGAGC ACCAACTACAACCCCTCCCTCAAGAGTCGAGTCA CCATGTCAGTAGACACGTCCAAGAACCAGTTCTC CCTGAAGCTGAGCTCTGTGACCGCCGCGGACACG GCCGTGTATTACTGTGCGAGAGAGGGAAAAACT GGGGGGGGTTTCTTACTTTGACTACTGGGGCCAGG GAACCCTGGTCACCGTCTCCTCA |
| | | | SEQ ID NO: 25495 | SEQ ID NO: 29501 |
| | | AA | DIQMTQSPSSLSASVGDRVTITCRASQSISSYLNW YQQKPGKAPKLLIYAASSLQSGVPSRFSGSGSGT DFTLTISSLQPEDFAPYYCQQSYNISFTFGPGTKV DIK | QVQLQESGPGLVKPSETLSLTCTVSGGSISSYFWSW IRQPAGKGLEWIGRIYTRGSTNYNPSLKSRVTMSVD TSKNQFSLKLSSVTAADTAVYYCAREGKTGGVSYF DYWGQGTLVTVSS |
| | | | SEQ ID NO: 25496 | SEQ ID NO: 29502 |

FIGURE 50

| iPS:434227 | 21-225_61A1 | NA | GACATCCAGATGACCCAGTCTCCATCCTCCCT GTCTGCATCTGTAGGAGACAGAGTCACCATCA CTTGCCGGGCAAGTCAGAGCATTAGCAGCTAT TTAAAATTGGTATCAGCAGAAACCAGGGAAAG CCCCTAAGCTCCTGATCTATGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCACCTT ACTACTGTCAACAGAGTTACAATATTTCATTC ACTTTCGGCCCTGGGACCAAAGTGGATATCAA A | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTCACTTCTG GAGCTGGATCCGGCAGCCCGCCGGGAAGGGACT GGAGTGGATTGGGCGCATCTATATCAGGGGGAG CACCAACTACAACCCCTCCCTCAAGAGTCGAGTC ACCATGTCAGTAGACACGTCCAAGAACCAGTTCT CCCTGAAGCTGAGCTCTGTGACCGCCGCGGACAC GGCCGTGTATTACTGTGCGAGAGAGGGAAAAAC TGGGGGGGGTTCTTACTTTGACTACTGGGGCCAG GGAACCCTGGTCACCGTCTCCTCA |
| | | | SEQ ID NO: 25497 | SEQ ID NO: 29503 |
| | | AA | DIQMTQSPSSLSASVGDRVTITCRASQSISSYLNW YQQKPGKAPKLLIYAASSLQSGVPSRFSGSGSGT DFTLTISSLQPEDFAPYYCQQSYNISFTFGPGTKV DIK | QVQLQESGPGLVKPSETLSLTCTVSGGSISSHFWSW IRQPAGKGLEWIGRIYIRGSTNYNPSLKSRVTMSVD TSKNQFSLKLSSVTAADTAVYYCAREGKTGGVSYF DYWGQGTLVTVSS |
| | | | SEQ ID NO: 25498 | SEQ ID NO: 29504 |
| iPS:434229 | 21-225_61H1 | NA | GACATCCAGATGACCCAGTCTCCATCCTCCCT GTCTGCATCTGTAGGAGACAGAGTCACCATCA CTTGCCGGGCAAGTCAGGGCATTAGAAAAGAT TTAGGCTGGTATCAGCAGAAACCAGGGAAAG CCCCTAAGCGCCTGATCTTTGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGTGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAATGACTATGGCAT GCACTGGGTCCGCCAGGCTCCAGGCAAGGGGCT GGAGTGGGTGGCAATTATATGGTATGATGAAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGCGAGAGATGTTCT GGACCCTTTTGACTACTGGGGCCAGGGAACCCTG GTCACCGTCTCCTCA |
| | | | SEQ ID NO: 25499 | SEQ ID NO: 29505 |

FIGURE 50

| | | | | |
|---|---|---|---|---|
| | | AA | DIQMTQSPSSLSASVGDRVTITCRASQGIRKDLG WYQQKPGKAPKRLIFAASSLQSGVPSRFSGSGSG TEFTLTISSLQPEDFATYYCLQHNSYPWTFGQGT KVEIK | QVQLVESGGGVVQPGRSLRLSCAASGFTFNDYGM HWVRQAPGKGLEWVAIIWYDESNKYYADSVKGRF TISRDNSKNTLYLQMNSLRAEDTAVYYCARDVLDP FDYWGQGTLVTVSS |
| | | | SEQ ID NO: 25500 | SEQ ID NO: 29506 |
| iPS:434231 | 21-225_61F2 | NA | GACATCCAGATGACCCAGTCTCCATCCTCCCT GTCTGCATCTGTAGGAGACAGAGTCACCGTCA CTTGCCGGGCAAGTCAGGGCATTAGAGATGAT TTAGGCTGGTATCAGCAGAAACCAGGGAAAG CCCCTGAGCGCCTGATCTATGCTGCATCCAGT TTGCAAAGTGGGGTCCCATCAAGGTTCAGCGG CAGTGGATCTGGGACAGAGTTCACTCTCACAA TCAGCAGCCTGCAGCCTGAAGATTTTGCAACT TATTACTGTCTACAGCATTATAGTTACCCTCGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAGCAGTGGCTGGTACGACTACGGTATGGACGTC TGGGGCCAAGGGACCACGGTCACCGTCTCCTCA |
| | | | SEQ ID NO: 25501 | SEQ ID NO: 29507 |
| | | AA | DIQMTQSPSSLSASVGDRVTVTCRASQGIRDDLG WYQQKPGKAPERLIYAASSLQSGVPSRFSGSGSG TEFTLTISSLQPEDFATYYCLQHYSYPRSFGQGT KLEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARERYSS GWYDYGMDVWGQGTTVTVSS |
| | | | SEQ ID NO: 25502 | SEQ ID NO: 29508 |

1861

FIGURE 50

| iPS:434233 | 21-225_61B3 | NA | GATATTGTGATGACCCAGACTCCACTCTCTCT GTCCGTCACACCTGGACAGCCGGCCTCCATCT CCTGCAAGTCTAGTCAGAGCCTCCTGCATAGT GAAGGAAAGACCTATTTGTATTGGTACCTGCA GAAGCCAGGCCAGCCTCCACAGCTCCTGATCT ATGAAGTTTCCAACCGGATCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGAAGCTGGGCGGGAGGCATGGACGTCTGGGG CCAAGGGACCACGGTCACCGTCTCCTCA |
| | | | SEQ ID NO: 25503 | SEQ ID NO: 29509 |
| | | AA | DIVMTQTPLSLSVTPGQPASISCKSSQSLLHSEGK TYLYWYLQKPGQPPQLLIYEVSNRISGVPDRFSG SGSGTDFTLKISRVEAEDVGVYYCMQSIQLPLTF GGGTKVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSRS WAGGMDVWGQGTTVTVSS |
| | | | SEQ ID NO: 25504 | SEQ ID NO: 29510 |
| iPS:434235 | 21-225_61E3 | NA | GACATCGTGATGACCCAGTCTCCAGACTCCCT GGCTGTGTCTCTGGGCGAGAGGGGCCACCATCA ACTGCAAGTCCAGCCAGAGTGTTTTACACATC TCCAACAATAACAATTACTTAGCTTGGTACCA GCAGCAACCAGGACAGCCTCCTAAACTGCTCA TTTACTGGGCATCTATCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTATTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGACCCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAAATCTGA GGACACGGCCGTGTATTACTGTGCGTATAGCAGT GGCTGGTACCGCTTTGACTACTGGGGCCAGGGAA CCCTGGTCACCGTCTCCTCA |
| | | | SEQ ID NO: 25505 | SEQ ID NO: 29511 |

FIGURE 50

| | | | | |
|---|---|---|---|---|
| | | AA | DIVMTQSPDSLAVSLGERATINCKSSQSVLHISN NNNYLAWYQQQPGQPPKLLIYWASIRESGVPDR FSGSGSGTDFTLTISSLQAEDVAVYYCQQYYSIP CSFGQGTKLEIK | QVQLVQSGAEVKKPGASVKVSCKASGYTFTNYDIN WVRQATGQGLEWMGWMTPNSGNTGYAQKFQGR VTMTRNTSISTAYMELSSLKSEDTAVYYCAYSSGW YRFDYWGQGTLVTVSS |
| | | | SEQ ID NO: 25506 | SEQ ID NO: 29512 |
| iPS:434237 | 21-225_61B5 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATAACAACTCCTTAACTTGGTACCA GCTGAAACCAGGACAGCCTCCTAAGAAGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GTCCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAGCACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGTATAGCAGT GGCTGGTACTACTTTGACTACTGGGGCCAGGGAA CCCTGGTCACCGTCTCCTCA |
| | | | SEQ ID NO: 25507 | SEQ ID NO: 29513 |
| | | AA | DIVMTQSPDSLAVSLGERATINCKSSQSVLYSSN NNNSLTWYQLKPGQPPKKLIYWASTRESGVPDR FSGSGSGTDFTLTISSLQAEDVAVYYCQQYYSTP PTFGQGSKVEIK | QVQLVQSGAEVKKPGASVKVSCKASGYTFTNYDIN WVRQATGQGLEWMGWMHPNSGSTGYAQKFQGR VTMTRNTSISTAYMELSSLRSEDTAVYYCAYSSGW YYFDYWGQGTLVTVSS |
| | | | SEQ ID NO: 25508 | SEQ ID NO: 29514 |

FIGURE 50

| | | | | |
|---|---|---|---|---|
| iPS:434239 | 21-225_58F1 | NA | GACATCCAGATGACCCAGTCTCCATCCTCCCT GTCTGCATCTGTAGGAGACAGAGTCACCATCA CTTGCCGGGCAAGTCAGAGCATTACCAACTTT TTAAATTGGTATCAGCAGAAACCAGGGAAAG CGCCTAAAACTCCTGATCTTCGCTGCATCCAGTT TGCAAAGTGGAATCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTATCCCGTGG ACGTTCGGCCAAGGGACCAAGGTGGAAATCA AA | GAGGTGCAGCTGTTGGAGTCTGGGGGAGGCTTG GTTCAGCCTGGGGGGTCCCTGAGACTCTCCTGTG CAGCCTCTGGATTCACCTTTAGCAGCTATGCCAT GAGTTGGGTCCGCCAGGCTCCAGGGAAGGGGCT GGAGTGGGTCTCAGCTATTAGTACTGGTGGTGGT AACACATACTACGCAGACTCCGTGAAGGGCCGG TTCACCATTTCCAGAGACAATTCCAAGAACACGC TGTATCTGCAAATGAACAGCCTGAGAGCCGAGG ACACGGCCGTATATTACTGTGCGAAACGGGGGG TCTACGGTGACTTTGATGCTTTTGATATCTGGGGC CAAGGGACAATGGTCACCGTCTCTTCA |
| | | | SEQ ID NO: 25509 | SEQ ID NO: 29515 |
| | | AA | DIQMTQSPSSLSASVGDRVTITCRASQSITNFLN WYQQKPGKAPKLLIFAASSLQSGIPSRFSGSGSG TDFTLTISSLQPEDFATYYCQQSYSIPWTFGQGT KVEIK | EVQLLESGGGLVQPGGSLRLSCAASGFTFSSYAMS WVRQAPGKGLEWVSAISTGGGNTYYADSVKGRFT ISRDNSKNTLYLQMNSLRAEDTAVYYCAKRGVYG DFDAFDIWGQGTMVTVSS |
| | | | SEQ ID NO: 25510 | SEQ ID NO: 29516 |
| iPS:434241 | 21-225_61E6 | NA | GACATCCAGATGACCCAGTCTCCATCCTCCCT GTCTGCATCTGTAGGAGACAGAATCACCATCA CTTGCCGGGCAAGTCAGGGCATTGGAAATGAT TTAGGCTGGTATCAGCAGAAACCAGGGAAAG CCCCTGAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAGTTTCCCTCCG TGGACGTTCGGCCAAGGGACCAAGGTGGAAA TCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGAAGCTATGCCAT GAGCTGGGTCCGCCAGGCTCCAGGGAAGGGGCT GGAGTGGGTCTCAGCTACTAGTGGTAGTGGTGTT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAATTGGAACTG GGGATCTTTGACTACTGGGGCCAGGGAACCCTGG TCACCGTCTCCTCA |
| | | | SEQ ID NO: 25511 | SEQ ID NO: 29517 |

FIGURE 50

| | | | | |
|---|---|---|---|---|
| | | AA | DIQMTQSPSSLSASVGDRITITCRASQGIGNDLG WYQQKPGKAPERLIYAASSLQSGVPSRFSGSGSG TEFTLTISSLQPEDFATYYCLQHNSFPPWTFGQG TKVEIK | EVQLLESGGGLVQPGGSLRLSCAASGFTFRSYAMS WVRQAPGKGLEWVSATSGSGVNTFYADSVKGRFT ISRDNSKNTLYLQMNSLRAEDTAVYYCAKLELGIF DYWGQGTLVTVSS |
| | | | SEQ ID NO: 25512 | SEQ ID NO: 29518 |
| IPS:434243 | 21-225_62C1 | NA | GATATTGTGATGACTCAGTCTCCACTCTCCCTG CCCGTCACCCCTGGAGAGCCGGCCTCCATCTC CTGCAGGTCTAGTCAGAGCCTCCTACATAGTA ATGGATACAACTATTTGGATTGGTACCTGCAG AAGCCAGGGCAGTCTCCACAGCTCCTGATCTA TTTGGTTTCTAATCGGGCCTCCGGGGTCCCTGA CAGGTTCAGTGGCAGTGGATCAGGCACAGATT TTACACTGAAAATCAGCAGAGTGGGGGCTGA GGATGTTGGGGTTTATTTCTGCCTGCAAGCTCT ACAAACTCCTCTCACCTTCGGCCAAGGGACAC GACTGGAGATTAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGACT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGATTTTTGGAGTG GACTGGGGCCAGGGAACCCTGGTCACCGTCTCCT CA |
| | | | SEQ ID NO: 25513 | SEQ ID NO: 29519 |
| | | AA | DIVMTQSPLSLPVTPGEPASISCRSSQSLLHSNGY NYLDWYLQKPGQSPQLLIYLVSNRASGVPDRFS GSGSGTDFTLKISRVGAEDVGVYFCLQALQTPLT FGQGTRLEIK | EVQLVESGGGLVKPGGSLRLSCAASGFTFSSYSMN WVRQAPGKGLEWVSSISSSSSYIYYADSVKGRFTIS RDNAKNSLYLQMNSLRAEDTAVYYCAIFGVDWGQ GTLVTVSS |
| | | | SEQ ID NO: 25514 | SEQ ID NO: 29520 |

FIGURE 50

| iPS:434245 | 21-225_62H1 | NA | GACATCCAGATGACCCAGTCTCCATCCTCCCT GTCTGCATATGTAGGAGACAGAGTCACCATCA CTTGCCGGGCAAGTCAGAACATTTTCAGCTAT TTAAATTGGTATCAGCAGAAACCAGGGAAAG CCCCTAAGGTCCTGATCTATGCTGTATTTAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTACCCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCCAGGGGCT GGAGTGGATGGCAATTATATGGTATGATGGAAGT AATAAATACTATGCAGACTCCGTGAAGGGCCGA TTCACCATCTCCAGAGACAATTCCAAGAACACGC TGTATCTGCAAATCAACAGCCTGAGAGCCGAGG ACACGGCTGTGTATTACTGTGCGAGAGAAGACCC GCGTACCAGCTGCTCTGACTACTGGGGCCAGGGA ACCCTGGTCACCGTCTCCTCA |
| | | | SEQ ID NO: 25515 | SEQ ID NO: 29521 |
| | | AA | DIQMTQSPSSLSAYVGDRVTITCRASQNIFSYLN WYQQKPGKAPKVLIYAVFSLQSGVPSRFSGSGS GTDFTLTISSLQPEDFATYYCQQSYSTPFTFGPGT KVDIK | QVQLVESGGGVVQPGRSLRLSCAASGFTFSSYGMH WVRQAPGQGLEWMAIIWYDGSNKYYADSVKGRF TISRDNSKNTLYLQINSLRAEDTAVYYCAREDPRTS CSDYWGQGTLVTVSS |
| | | | SEQ ID NO: 25516 | SEQ ID NO: 29522 |
| iPS:434247 | 21-225_62D2 | NA | GACATCCAGATGACCCAGTCTCCATCCTCCCT GTCTGCATCTGTAGGAGACAGAGTCACCATCA TTTGCCGGGCAAGTCAGAGCATTATCAGTTAT TTAAATTGGTATCAGCAGAAACCAGGGAAAG CCCCTAAGCTCCTGATCTATGCTACATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGACTTACAGTCCCCCGCTC ACTTTCGGCGGAGGGACCAAGGTGGAGATCA AA | CAGGTGTATCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTCTGCAGACTCCGTGAAGGGCCGAT TCACCATCTCCAGAGACAATTCCAAGAACACGCT GGATCTGCAAATGAACAGCCTGAGAGCCGAGGA CACGGCTGTGTATTACTGTGCGAGAGATAATGGT AACTGGAACTACCTTGACTACTGGGGCCAGGGA ACCCTGGTCACCGTCTCCTCA |
| | | | SEQ ID NO: 25517 | SEQ ID NO: 29523 |

1866

FIGURE 50

| | | | | |
|---|---|---|---|---|
| | | AA | DIQMTQSPSSLSASVGDRVTIICRASQSIISYLNW YQQKPGKAPKLLIYATSSLQSGVPSRFSGSGSGT DFTLTISSLQPEDFATYYCQQTYSPPLTFGGGTK VEIK | QVYLVESGGGVVQPGRSLRLSCAASGFTFSSYGMH WVRQAPGKGLEWVAVIWYDGSNKYSADSVKGRF TISRDNSKNTLDLQMNSLRAEDTAVYYCARDNGN WNYLDYWGQGTLVTVSS |
| | | | SEQ ID NO: 25518 | SEQ ID NO: 29524 |
| iPS:434249 | 21-225_62E2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCCGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAGTAATTACCCTCTCA CTTTCGGCGGAGGGACCAGGGTTGAGATCAAA | CAGCTGCAGCTGCAGGAGTCGGGCCCAGGACTG GTGAAGCCTTCGGAGACCCTGTCCCTCACCTGTA TTGTCTCTGGTGGCTCCATCAGCAGAAGTAGTTA CTACTGGGGCTGGATCCGCCAGCCCCCAGGGAA GGGGCTGGAGTGGATTGGGAGCATCTATTATAGT GGGATCGCCTCCTATAATCCGTCCCTCAAGAGTC GAGTCACCATATCCGTAGACACGTCCAAGAACC AGTTCTCCCTGAAGCTGAACTCTGTGACCGCCAC AGACACGGCTGTATATTACTGTGCGAGACTGAGC AGTGGCTGGTCCTTTGACTACTGGGGCCAGGGAA CCCTGGTCACCGTCTCCTCA |
| | | | SEQ ID NO: 25519 | SEQ ID NO: 29525 |
| | | AA | DIQMTQSPSSLSASVGDRVTITCRASQGIRNDLG WYQQKPGKAPKRLIYAASRLQSGVPSRFSGSGS GTEFTLTISSLQPEDFATYYCLQHSNYPLTFGGG TRVEIK | QLQLQESGPGLVKPSETLSLTCIVSGGSISRSSYYWG WIRQPPGKGLEWIGSIYYSGIASYNPSLKSRVTISVD TSKNQFSLKLNSVTATDTAVYYCARLSSGWSFDY WGQGTLVTVSS |
| | | | SEQ ID NO: 25520 | SEQ ID NO: 29526 |

FIGURE 50

| iPS:434251 | 21-225_62G3 | NA | GACATCCATATGACCCAGTCTCCATCCTCCCT GTCTGCATCTGTAGGAGACAGAGTCACCATCA CTTGCCGGGCAAGTCAGGACATTAGAAATAAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCGACT TATTACTGTCTACAGTATAATAGTTACCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTATATTACTGTGCGAGGGTTAACTCT TTTGACTCCTGGGGCCAGGGAACCCTGGTCACCG TCTCCTCA |
| | | | SEQ ID NO: 25521 | SEQ ID NO: 29527 |
| | | AA | DIHMTQSPSSLSASVGDRVTITCRASQDIRNNLG WYQQKPGKAPKRLIYTASSLQSGVPSRFSGSGSG TEFTLTISSLQPEDFATYYCLQYNSYPFTFGPGTK VDIK | EVQLVESGGGLVKPGGSLRLSCAASGFTFSSYSMN WVRQAPGKGLEWVSSISSSSSYIYYADSVKGRFTIS RDNAKNSLYLQMNSLRAEDTAVYYCARVNSFDSW GQGTLVTVSS |
| | | | SEQ ID NO: 25522 | SEQ ID NO: 29528 |
| iPS:434253 | 21-225_62E4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTAATCTATGCTGCATTCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTATCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATAGAAGT AATAAATACTATGCAGACTCCGTGAAGGGCCGA TTCACCATCTCCAGAGACAATTCCAAGAACACGC TGTATCTGCAAATGAACAGCCTGAGAGTCGAGG ACACGGCTGTGTATCACTGTGCGAGAGAGCTTGG GTTCAGCAGTGACTACTGGGGCCAGGGAACCCT GGTCACCGTCTCCTCA |
| | | | SEQ ID NO: 25523 | SEQ ID NO: 29529 |

FIGURE 50

| | | | | |
|---|---|---|---|---|
| | | AA | DIQMTQSPSSLSASVGDRVTITCRASQGIRNDLG WYQQKPGKAPKRLIYAAFSLQSGVPSRFSGSGS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYDRSNKYYADSVKGR FTISRDNSKNTLYLQMNSLRVEDTAVYHCARELGF SSDYWGQGTLVTVSS |
| | | | SEQ ID NO: 25524 | SEQ ID NO: 29530 |
| iPS:434255 | 21-225_62E6 | NA | GAAATAGTGATGACGCAGTCTCCAGCCACCCT GTCTGTGTCTCCAGGGGAAAGAGCCACCCTCT CCTGCAGGGCCAGTCAGAGTGTTAACAGCAAC TTAGCCTGGTACCAGCAGAAACCTGGCCAGGC TCCCAGGCTCCTCATCTCTGTTGCATCCACCAG GGCCACTGGTATCCCAGCCAGGTTCAATGGCA GTGGGTCTGGGACAGAGTTCACTCTCACCATC AGCAGCCTGCAGTCTGAAGATTTTGCAGTTTA TTACTGTCAGCAGTATAATGACTGGCCGTGTA GTTTTGGCCAGGGGACCAAGCTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTTCCTATGGCAT GCACTGGGTCCGCCAGGCTCCAGGCAAGGGGCT GGAGTGGGTGGCAGCTATATGGTATGATGGAAG TAATAAATACTATGGAGACTCCGTGAAGGGCCG AGTCACCATCTCCAGAGACAATTCCAAGAACTCG CTGCATCTGCAAATGAACAGCCTGAGAGCCGAG GACACGGCTGTGTATTATTGTGCGAGAGATCAGG GCATAGTGGGAGCTACTTGGTTTGACTACTGGGG CCAGGGAACCCTGGTCACCGTCTCCTCA |
| | | | SEQ ID NO: 25525 | SEQ ID NO: 29531 |
| | | AA | EIVMTQSPATLSVSPGERATLSCRASQSVNSNLA WYQQKPGQAPRLLISVASTRATGIPARFNGSGSG TEFTLTISSLQSEDFAVYYCQQYNDWPCSFGQGT KLEIK | QVQLVESGGGVVQPGRSLRLSCAASGFTFSSYGMH WVRQAPGKGLEWVAAIWYDGSNKYYGDSVKGRV TISRDNSKNSLHLQMNSLRAEDTAVYYCARDQGIV GATWFDYWGQGTLVTVSS |
| | | | SEQ ID NO: 25526 | SEQ ID NO: 29532 |

FIGURE 50

| iPS:434257 | 21-225_62F7 | NA | GACATCCAGATGACCCAGTCTCCATCCTCCCT GTCTGCATCGTAGGAGACAGAGTCACCATCA CTTGCCGGGCAAGTCAGGCCATTAGAAATGAT TTAGGCTGGTATCAGCAGAAACCAGGGAAAG CCCCTAAGCGCCTGATCTATCCTGCATCCCGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGTATAATAGTTACCCTCCGT GGACGTTCGGCCAAGGGTCCAAGGTGGAAAT CAAA | GAGGTGCAGCTGTTGGAGTCTGGGGGAGGCTTG GTTCAGCCTGGGGGGTCCCTGAGACTCTCCTGTG CAGCCTCTGGATTCACCTTTAGCAGCTATGTTAT GAGCTGGGTCCGCCAGGCTCCAGGGAAGGGGCT GGAGTGGGTCTCAGGTATTAGTGGTAGTGGTGCT AAAACATACTATGCAGACTCCGTGAAGGGCCGA TTCACCATCTCCAGAGACAATTCCAAGAACACGC TGTATCTGCAAATGAACAGCCTGAGAGCCGAGG ACACGGCCGTATATTACTGTGCGGAACTGGGGAT AGACTACTACTACGGTATGGACGTCTGGGGCCAA GGGACCACGGTCACCGTCTCCTCA |
| | | | SEQ ID NO: 25527 | SEQ ID NO: 29533 |
| | | AA | DIQMTQSPSSLSASVGDRVTITCRASQAIRNDLG WYQQKPGKAPKRLIYPASRLQSGVPSRFSGSGS GTEFTLTISSLQPEDFATYYCLQYNSYPPWTFGQ GSKVEIK | EVQLLESGGGLVQPGGSLRLSCAASGFTFSSYVMS WVRQAPGKGLEWVSGISGSGAKTYYADSVKGRFT ISRDNSKNTLYLQMNSLRAEDTAVYYCAELGIDYY YGMDVWGQGTTVTVSS |
| | | | SEQ ID NO: 25528 | SEQ ID NO: 29534 |
| iPS:434259 | 21-225_62G7 | NA | GACATCCAGATGACCCAGTCTCCATCTTCCGT GTCTGCATCGTAGGAGACAGAGTCACCTTCA CTTGTCGGGCGAGTCAGGATATTAGCAGCTGG TTAGCCTGGTATCAGCAGAATCCAGGGAAGGC CCCTAAACTCCTGATCTATGCTGCATCCAGTTT GCAAAGTGGGGTCCCATCAAGGTTCAGCGGCA GTGGATCTGGGACAAATTTCACTCTCACCATC AGCAGCCTCCAGCCTGAAGATTTTGCAACTTA CTATTGTCAACAGACTAACAGTTTCCCTCTCAC TTTCGGCGGAGGGACCAAGGTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGAC TTGAGTGGATGGGATGGATCAAACCTAAAAGTG GTGGCACAAACCAAGCACAGAAGTTTCAGGGCA GGGTCACCATGACCAGGGACACGTCCATCAGCA CAGCCTACATGGAGCTGAGCAGTCTGAGATCTGA CGACACGGCCGTGTATTACTGTGCGAGAGCTCCG GGTATAGCAGCAGCTGGTACATGGGGATACTTTG ACTACTGGGGCCAGGGAACCCTGGTCACCGTCTC CTCA |
| | | | SEQ ID NO: 25529 | SEQ ID NO: 29535 |

FIGURE 50

| | | | | |
|---|---|---|---|---|
| | | AA | DIQMTQSPSSVSASVGDRVTFTCRASQDISSWLA WYQQNPGKAPKLLIYAASSLQSGVPSRFSGSGS GTNFTLTISSLQPEDFATYYCQQTNSFPLTFGGGT KVEIK | QVQLVQSGAEVKKPGASVKVSCKASGYTFTGYYM HWVRQAPGQGLEWMGWIKPKSGGTNQAQKFQGR VTMTRDTSISTAYMELSSLRSDDTAVYYCARAPGI AAAGTWGYFDYWGQGTLVTVSS |
| | | | SEQ ID NO: 25530 | SEQ ID NO: 29536 |
| IPS:434261 | 21-225_56F7 | NA | GACATCCTGATGACCCAGTCTCCATCCTCACT GTCTGCATCTGTAGGAGACAGAGTCACCATCA CTTGTCGGGCGAGTCAGGGCATTAGCACTTAT TTAGCCTGGTTTCAGCAGACACCAGGGACAGC CCCTAAGTCCCTGATCTATGCTGCATCCAGTTT GCAAGGTGGGGTCCCATCAAAGTTCAGCGGCA GTGGATCTGGGACAGATTTCACTCTCACCATC AGCAGCCTGCAGCCTGAAGATTTTGCAACTTA TTACTGCCATCAGTATAATAGTTTCCCATTTAA GTTCGGGCGTGGGACCAAAGTGGATATCACA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGTCTT AAACTGGGTCCGCCAGGCTCCAGGGAAGGGGCT GGAGTGGGTCTCAGCTATGAGTGGTAGTGGTGGT AGAACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTTCTGTGCGATGACTACGCA CTTTGACTACTGGGGCCAGGGAACCCTGGTCACC GTCTCCTCA |
| | | | SEQ ID NO: 25531 | SEQ ID NO: 29537 |
| | | AA | DILMTQSPSSLSASVGDRVTITCRASQGISTYLA WFQQTPGTAPKSLIYAASSLQGGVPSKFSGSGSG TDFTLTISSLQPEDFATYYCHQYNSFPFKFGRGT KVDIT | EVQLLESGGGLVQPGGSLRLSCAASGFTFSSYVLN WVRQAPGKGLEWVSAMSGSGGRTYYADSVKGRF TISRDNSKNTLYLQMNSLRAEDTAVYFCAMTTHFD YWGQGTLVTVSS |
| | | | SEQ ID NO: 25532 | SEQ ID NO: 29538 |

EP 4 435 105 A2

FIGURE 50

| iPS:434263 | 21-225_56H7 | NA | GACATCCAGCTGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAGGCCAGGGAAAG CCCCTAAGCGCCTGATCTATCCTGCATCCAGTT TGCTAAGTGGGGTCCCATCAAGGTTCAGCGGC AGTGGATCTGGGACAGAATTCACTCTCACAAT CAGCAGCCTGCAGCCTGAAGATTTTGCAACTT ACTACTGTCTACAGGATAATAGTTACCCATTC ACTTTCGGCCCTGGGACCAAAGTGGATAGCAA A | GAGGTGCAGCTGGTGGAGTCTGGGGGAGGCCTG GTCAAGCCTGGGGGGTCCCTGAGACTCTCCTGTG CAGCCTCTGGATTCTCCTTCAGTAGCTATAGCAT GAACTGGGTCCGCCAGGCTCCAGGGAAGGGGCT GGAGTGGGTCTCATCCATTAGTAGTAGTAGTACT TACATATACTACGGAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAGATCGGGG CAGCAGCTGGGGCCAGGGAACCCTGGTCACCGT CTCCTCA |
| | | | SEQ ID NO: 25533 | SEQ ID NO: 29539 |
| | | AA | DIQLTQSPSSLSASVGDRVTITCRASQDIRNDLG WYQQRPGKAPKRLIYPASSLLSGVPSRFSGSGSG TEFTLTISSLQPEDFATYYCLQDNSYPFTFGPGTK VDSK | EVQLVESGGGLVKPGGSLRLSCAASGFSFSSYSMN WVRQAPGKGLEWVSSISSSSTYIYYGDSVKGRFTIS RDNAKNSLYLQMNSLRAEDTAVYYCARDRGSSW GQGTLVTVSS |
| | | | SEQ ID NO: 25534 | SEQ ID NO: 29540 |
| iPS:434265 | 21-225_57B2 | NA | GACATCCAGATGACCCAGTCTCCATCCTCCCT GTCTGTATCTGTAGGAGACAGAGTCACCATCA CTTGCCGGGCAAGTCAGGGCATTAGAAATGCT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATAAACTACACAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAGTGGCTGG CTTTGACTACTGGGGCCAGGGAACCCTGGTCACC GTCTCCTCA |
| | | | SEQ ID NO: 25535 | SEQ ID NO: 29541 |

1872

FIGURE 50

| | | | | |
|---|---|---|---|---|
| | | AA | DIQMTQSPSSLSVSVGDRVTITCRASQGIRNALG WYQQKPGKAPKRLIYAASSLQSGVPSRFSGSGS GTEFTLTISSLQPEDFATYYCLQHNSYPFTFGPGT KVDIK | EVQLVESGGGLVKPGGSLRLSCAASGFTFSSYSMN WVRQAPGKGLEWVSSISGSSSYINYTDSVKGRFTIS RDNAKNSLYLQMNSLRAEDTAVYYCARVAGFDY WGQGTLVTVSS |
| | | | SEQ ID NO: 25536 | SEQ ID NO: 29542 |
| iPS:434267 | 21-225_57F2 | NA | GACATCCAGATGACCCAGTCTCCATCCTCCCT GTCTGCATCTGTAGGAGACAGAGTCACCATCA CTTGCCGGGCAAGTCAGAGCATTAGCAGCTAT TTAAAATTGGTATCAGCAGAAACCAGGGAAAG CCCCTAAGCTCCTGATCTATGCTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCACCTT ACTACTGTCAACAGAGTTACAATATTTCATTC ACTTTCGGCCCTGGGACCAAAGTGGATATCAA A | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTACTACTG GAGCTGGATCCGGCAGCCCGCCGGGAAGGGACT GGAGTGGATTGGGCGCATCTATACCAGGGGGAG CACCAACTACAACCCCTCCCTCAAGAGTCGAGTC ACCATGTCAATAGACACGTCCAAGAACCAGTTCT CCCTGAAGCTGAGCTCTGTGACCGCCGCGGACAC GGCCGTGTATTACTGTGCGAGAGAGGGGAAAAAC TGGGGGGGTTTCTTACTTTGACTACTGGGGCCAG GGAACCCTGGTCACCGTCTCCTCA |
| | | | SEQ ID NO: 25537 | SEQ ID NO: 29543 |
| | | AA | DIQMTQSPSSLSASVGDRVTITCRASQSISSYLNW YQQKPGKAPKLLIYAASSLQSGVPSRFSGSGSGT DFTLTISSLQPEDFAPYYCQQSYNISFTFGPGTKV DIK | QVQLQESGPGLVKPSETLSLTCTVSGGSISSYYWSW IRQPAGKGLEWIGRIYTRGSTNYNPSLKSRVTMSID TSKNQFSLKLSSVTAADTAVYYCAREGKTGGVSYF DYWGQGTLVTVSS |
| | | | SEQ ID NO: 25538 | SEQ ID NO: 29544 |

FIGURE 50

| | | | | |
|---|---|---|---|---|
| iPS:434269 | 21-225_57H3 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCAGC TTAGCCTGGTACCAGCAGAAACCTGGCCAGGC TCCCAGGCTCCTCATCTATGGTGCATCCACCA GGGCCACTGGTTTCCCAGCCAGGTTCAATGGC AGTGGGTCTTGGACAGAATTCACTCTCACCAT CAGCAGCCTGCAGTCTGAAGATTTTGCAATTT ATTACTGTCAGCAGTATAATGACTGGCCGTGC AGTTTTGGCCAGGGGACCAAGCTGGAGATCAA A | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGCTATATGGTATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGATTATG GTATAGTGGGAGCTACATATTTTGACTACTGGGG CCAGGGAACCCTGGTCACCGTCTCCTCA |
| | | | SEQ ID NO: 25539 | SEQ ID NO: 29545 |
| | | AA | EIVMTQSPATLSVSPGERATLSCRASQSVSSSLA WYQQKPGQAPRLLIYGASTRATGFPARFNGSGS WTEFTLTISSLQSEDFAIYYCQQYNDWPCSFGQG TKLEIK | QVQLVESGGGVVQPGRSLRLSCAASGFTFSSYGMH WVRQAPGKGLEWVAAIWYDGSNKYYADSVKGRF TISRDNSKNTLYLQMNSLRAEDTAVYYCARDYGIV GATYFDYWGQGTLVTVSS |
| | | | SEQ ID NO: 25540 | SEQ ID NO: 29546 |
| iPS:434271 | 21-225_57A4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCTGCAGAAACCAGGGAAAGC CCCTAAGCGCCTGATCTATGCTGCATCCAGTTT GCTAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATAATAGTTACCCATTCAC TTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG TAGCGTCTGGATTCACCTTCAGTGACTATGGCAT GCACTGGGTCCGCCAGGCTCCAGGCAAGGGGCT GGAGTGGGTGGCAGTTATATGGTATGCTGGAAGT AATAAATACTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGGGAACTGGGG ATGAGGTCTGACTACTGGGGCCAGGGAACCCTG GTCACCGTCTCCTCA |
| | | | SEQ ID NO: 25541 | SEQ ID NO: 29547 |

# EP 4 435 105 A2

FIGURE 50

| | | | | |
|---|---|---|---|---|
| | | AA | DIQMTQSPSSLSASVGDRVTITCRASQGIRNDLG WYLQKPGKAPKRLIYAASSLLSGVPSRFSGSGSG TEFTLTISSLQPEDFATYYCLQHNSYPFTFGPGTK VDIK | QVQLVESGGGVVQPGRSLRLSCVASGFTFSDYGM HWVRQAPGKGLEWVAVIWYAGSNKYYVDSVKGR FTISRDNSKNTLYLQMNSLRAEDTAVYYCARELGM RSDYWGQGTLVTVSS |
| | | | SEQ ID NO: 25542 | SEQ ID NO: 29548 |
| IPS:434273 | 21-225_57E4 | NA | GACATCCAGATGACCCAGTCTCCATCTTCCGT GTCTGCATCTGTAGGAGACAGAGTCTCCATCA CTTGTCGGGCGAGTCAGGATATTAGCAACTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AGTGGTTCTGGGACAGATTTCACTCTCACCAT CAGCAGCCTGCAGCCTGAAGATTTTGCAACTT ACTCTTGTCAACAGGGTAACAGTTTCCCATTC ACTTTCGGCCCTGGGACCAAAGTGGATATCAA A | GAGGTGCAGCTGTTGGAGTCTGGGGGAGGCTTG GTACAGCCGGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTAGTGGTGGT AGTACATTCTACGCAGACTCCGTGAAGGGCCGGT TCACCATCTCCAGAGACAATTCCAAGACCACGCT GTATCTGCAAATGAACAGCCTGAGAGCCGAGGA CACGGCCGTATATTACTGTGCGAAAAGGGACTG GAACGACGTTTTTGACTACTGGGGCCAGGGAACC CTGGTCACCGTCTCCTCA |
| | | | SEQ ID NO: 25543 | SEQ ID NO: 29549 |
| | | AA | DIQMTQSPSSVSASVGDRVSITCRASQDISNWLA WYQQKPGKAPKLLIYAASSLQSGVPSRFSGSGS GTDFTLTISSLQPEDFATYSCQQGNSFPFTFGPGT KVDIK | EVQLLESGGGLVQPGGSLRLSCAASGFTFSSYAMS WVRQAPGKGLEWVSVISGSGGSTFYADSVKGRFTI SRDNSKTTLYLQMNSLRAEDTAVYYCAKRDWND VFDYWGQGTLVTVSS |
| | | | SEQ ID NO: 25544 | SEQ ID NO: 29550 |

1875

FIGURE 50

| iPS:434275 | 21-225_57F4 | NA | GACATCCAGATGACCCAGTCTCCATCCTCCCT GTCTGCATCTGTAGGAGACAGAGTCACCATCA CTTGCCGGGCAAGTCAGGTCATTAGAAATGAT TTAGGCTGGTATCAGCAGAAGCCAGGGAAAG CCCCTAAGCGCTTGATCTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATCACTGTCTACAGTATGGTAGTTTCCCATTCA CTTTCGGCCCTGGGACCAAAGTGGATATCAAA | CAGGTGCAGCTGGTGGAGTCTGGGGGAGGCTTG GTCAAGCCTGGAGGGTCCCTGAGACTGTCCTGTG CAGCCTCTGGATTCATCTTCAGTGACTACTACAT GAACTGGATCCGCCAGGCTCCAGGGAAGGGGCT GGAGTGGGTTTCATACATTAGTAGTAGTGGTAGT ACCATATACTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGAGAGATATGATT ACGTGGGGCCAGGGAACCCTGGTCACCGTCTCCT CA |
|  |  |  | SEQ ID NO: 25545 | SEQ ID NO: 29551 |
|  |  | AA | DIQMTQSPSSLSASVGDRVTITCRASQVIRNDLG WYQQKPGKAPKRLIYAASSLQSGVPSRFSGSGS GTEFTLTISSLQPEDFATYHCLQYGSFPFTFGPGT KVDIK | QVQLVESGGGLVKPGGSLRLSCAASGFIFSDYYMN WIRQAPGKGLEWVSYISSSGSTIYYADSVKGRFTIS RDNAKNSLYLQMNSLRAEDTAVYYCARDMITWG QGTLVTVSS |
|  |  |  | SEQ ID NO: 25546 | SEQ ID NO: 29552 |
| iPS:434277 | 21-225_57A7 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAACCTCCTGATCTATGCTGCATCCAATT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTAGATATCAA A | CAGGTGCAACTGGTGCAGTCTGGGGCTGAGGTG AAGAAGCCTGGGGCCTCAGTGAAGGTCTCCTGC AAGGCTTCTGGATACACCTTCACCGGCTACCATA TACACTGGGTGCGACAGGCCCCTGGACAAGATCT TGAGTGGATGGGATGGATCAACCCTAACAATAAT GGCACAAACTATGCACAGAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGAGAGATGGG AGAAGTGGTTTTGACTACTGGGGCCAGGGAACC CTGGTCACCGTCTCCTCA |
|  |  |  | SEQ ID NO: 25547 | SEQ ID NO: 29553 |

FIGURE 50

| | | | | |
|---|---|---|---|---|
| | | AA | DIQMTQSPSSVSASVGDRVTITCRASQGISRWLA WYQQKPGKAPNLLIYAASNLQSGVPSRFSGSGS GTDFTLTISSLQPEDFATYYCQQANSFPFTFGPGT KVDIK | QVQLVQSGAEVKKPGASVKVSCKASGYTFTGYHIH WVRQAPGQDLEWMGWINPNNNGTNYAQKFQGRV TMTRDTSISTAYMELSRLRSDDTAVYYCARDGRSG FDYWGQGTLVTVSS |
| | | | SEQ ID NO: 25548 | SEQ ID NO: 29554 |
| iPS:434279 | 21-225_57F7 | NA | GAAATAGTGATGACGCAGTCTCCAGCCATCCT GTCTGTGTTTCCAGGGGAAAGAGCCACCCTCT CCTGCAGGGCCAGTCAGAGTGTTAGCAGCGAC TTAGCCTGGTACCAGCAGAAACCTGGCCAGGC TCCCAGGCTCCTCATCTATGGTGCATCCACCA GGGCCACTGGTATGCCAGCCAGGTTCAGTGGC GGTGGGTCTGGGACAGAGTTCACTCTCACCAT CAGCAGCCTGCAGTCTGAACATTTTGCAGTTT ATTACTGTCAGCAGTATAGTAACTGGCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AACACATTCTACGCAGACTCCGTGAAGGGCCGGT TCACCATCTCCAGAGACAATTCCAAGAACACGCT GTATTTGCAAATGAACAGCCTGAGAGCCGAGGA CACGGCCGTATATTACTGTGCGAAATTTTTCGGT GTAGTGGGAGTCGGGTGCTTTGACTACTGGGGCC AGGGAACCCTGGTCACCGTCTCCTCA |
| | | | SEQ ID NO: 25549 | SEQ ID NO: 29555 |
| | | AA | EIVMTQSPAILSVFPGERATLSCRASQSVSSDLA WYQQKPGQAPRLLIYGASTRATGMPARFSGGGS GTEFTLTISSLQSEHFAVYYCQQYSNWPFTFGPG TKVDIK | EVQLLESGGGLVQPGGSLRLSCAASGFTFSSYAMS WVRQAPGKGLEWVSAISGSGGNTFYADSVKGRFTI SRDNSKNTLYLQMNSLRAEDTAVYYCAKFFGVVG VGCFDYWGQGTLVTVSS |
| | | | SEQ ID NO: 25550 | SEQ ID NO: 29556 |

FIGURE 50

| iPS:434281 | 21-225_57B8 | NA | GACATCCAGATGACCCAGTCTCCATCCTCCCT GTCTGCATCTGTAGGAGACAGAATCACCATCA CTTGCCGGGCAAGTCAGGGCATTGGAAATGAT TTAGGCTGGTATCAGCAGAAACCAGGGAAAG CCCCTGAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAACATAATAGTTTCCCTCCG TGGACGTTCGGCCAAGGGACCAAGGTGGAAA TCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGAAGCTATGCCAT GAGCTGGGTCCGCCAGGCTCCAGGGAAGGGGCT GGAGTGGGTCTCAGCTATTAGTGGTAGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAATTGGAACTG GGGATCTTTGACTACTGGGGCCAGGGAACCCTGG TCACCGTCTCCTCA |
| | | | SEQ ID NO: 25551 | SEQ ID NO: 29557 |
| | | AA | DIQMTQSPSSLSASVGDRITITCRASQGIGNDLG WYQQKPGKAPERLIYAASSLQSGVPSRFSGSGSG TEFTLTISSLQPEDFATYYCLQHNSFPPWTFGQG TKVEIK | EVQLLESGGGLVQPGGSLRLSCAASGFTFRSYAMS WVRQAPGKGLEWVSAISGSGGNTFYADSVKGRFTI SRDNSKNTLYLQMNSLRAEDTAVYYCAKLELGIFD YWGQGTLVTVSS |
| | | | SEQ ID NO: 25552 | SEQ ID NO: 29558 |
| iPS:434283 | 21-225_57F8 | NA | GACATCCAGATGACCCAGTCTCCATCTTCCGT GTCTGCATCTGTAGGAGACAGAGTCACCATCA CTTGTCGGGCGAGTCAGGGTATTAGCAACTGG TTAGCCTGGTATCAGCAGAAACCAGGGAAAG CCCCTAAGCTCCTGATCTATACTGCATCCAGTT TGCAAAGTGGGGTCCCATCAAGGTTCAGCGGC AATGAATCTGGGACAGATTTCACTCTCACCAT CAGCAGCCTGCAGCCTGAAGATTTTGCAACTT ACTATTGTCAACAGGCTAACAGTTTCCCGTGG ACGTTCGGCCAAGGGACCAAGGTGGAAATCA AA | GAGGTGCAGCTGTTGGAGTCTGGGGGAGGCTTG GTACAGCCGGGGGGGTCCCTGAGACTCTCCTGTG TAGTCTCTGGATTCACCTTTAGCAACTATGCCAT GAGCTGGGTCCGCCAGGCTCCAGGGAAGGGGCT GGAGTGGGTCTCAGCTAGCAGTGGTAGTGGTGGT AACACATTCTACGCAGACTCCGTGACGGGCCGG GTCACCATCTCCAGAGACAATTCCAAGAACACGC TGTATCTGCAAATGAACAGTCTGAGAGCCGAGG ACACGGCCGTATATTACTGTGCGAAACTGGGGA AGGACTACCACTACTACGGTATGGACGTCTGGGG CCAAGGGACCACGGTCACCGTCTCCTCA |
| | | | SEQ ID NO: 25553 | SEQ ID NO: 29559 |

FIGURE 50

| | | | | |
|---|---|---|---|---|
| | | AA | DIQMTQSPSSVSASVGDRVTITCRASQGISNWLA WYQQKPGKAPKLLIYTASSLQSGVPSRFSGNESG TDFTLTISSLQPEDFATYYCQQANSFPWTFGQGT KVEIK | EVQLLESGGGLVQPGGSLRLSCVVSGFTFSNYAMS WVRQAPGKGLEWVSASSGSGGNTFYADSVTGRVT ISRDNSKNTLYLQMNSLRAEDTAVYYCAKLGKDY HYYGMDVWGQGTTVTVSS |
| | | | SEQ ID NO: 25554 | SEQ ID NO: 29560 |
| iPS:434285 | 21-225_57A11 | NA | GACATCGTGATGACCCAGTCTCCAGACTCCCT GACTGTGTCTCTGGGCGAGAGGGCCACCATCA ACTGCAAGTCCAGCCAAAGTGTTTTACACAGC TCCAACAATTATAACTACTTAGCTTGGTACCA GCAGAGACCAGGACAGCCTCCTAAGCTGGTCA TTTACTGGGCATCTACCCGGGCATCCGGGGTC CCTGACCGATTCAGTGGCAGCGGGTCTGGGAC AGATTTCACTCTCACCATCAGCAGCCTGCAGG CTGAAGATATGGCAGTTTATTACTGTCAGCAA TATTATAGTACTCCGTGGACGTTCGGCCAAGG GACCAAGGTGGAATTCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTGT TAACACAGGCTATGCACAGAAGTTCCAGGGCAG AGTCACCATGACCAGGGACACCTCCATAAGCAC AGCCTACATGGAGCTGAGCAGCCTGAGATCTGA GGACACGGCCGTGTATTACTGTGCGATCAGCAGT GGCTGGAACTGGTTCGACCCCTGGGGGCCAGGGA ACCCTGGTCACCGTCTCCTCA |
| | | | SEQ ID NO: 25555 | SEQ ID NO: 29561 |
| | | AA | DIVMTQSPDSLTVSLGERATINCKSSQSVLHSSN NYNYLAWYQQRPGQPPKLVIYWASTRASGVPD RFSGSGSGTDFTLTISSLQAEDMAVYYCQQYYST PWTFGQGTKVEFK | QVQLVQSGAEVKKPGASVKVSCKASGYTFTNYDIN WVRQATGQGLEWMGWMNPNSVNTGYAQKFQGR VTMTRDTSISTAYMELSSLRSEDTAVYYCAISSGW NWFDPWGQGTLVTVSS |
| | | | SEQ ID NO: 25556 | SEQ ID NO: 29562 |

FIGURE 50

| iPS:434287 | 21-225_57F12 | NA | GACATCGTGATGACCCAGTCTCCAGACTCCCT GGCTGTGTCTCTGGGCGAGAGGGCCACCATCA ACTGCAAGTCCAGCCAGAGTGTTTTATTCAGC TCCAACAATTACAATTACTTAGCTTGGTACCA GCAGAAAACAGGACAGCCTCCCAAGCTGATC ATTTACTGGGCATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAATTTATTACTGTCAGCA ATATTATAGTAATCCGTGTAGTTTTGGCCAGG GGACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGATAAGCA GTGGCTGGTACCGGTTCGACCCCTGGGGCCAGGG AACCCTGGTCACCGTCTCCTCA |
| | | | SEQ ID NO: 25557 | SEQ ID NO: 29563 |
| | | AA | DIVMTQSPDSLAVSLGERATINCKSSQSVLFSSN NYNYLAWYQQKTGQPPKLIIYWASTRESGVPDR FSGSGSGTDFTLTISSLQAEDVAIYYCQQYYSNP CSFGQGTKLEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAISSGW YRFDPWGQGTLVTVSS |
| | | | SEQ ID NO: 25558 | SEQ ID NO: 29564 |
| iPS:434289 | 21-225_57H12 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCGAC TTAGCCTGGTACCAGCAGAAACCTGGCCAGGC TCCCAGGCTCCTCATCTATGCTGCATCTACCAG GGCCACTGGTATCCCAGCCAGGTTCAGTGGCA GTGGGTCTGGGACAGAGTTCACTCTCACCATC AGCAGCCTGCAGTCTGAAGATTTTGCAGTTTA TTACTGTCAGCAGTATGATAACTGGCCATTCA CTTTCGGCCCTGGGACCAAAGTGGATAACAAG | GAGGTGCAGCTGTTGGAGTCTGGGGGAGGCTTG GTACAGCCGGGGGGGTCCCTGAGACTCTCCTGTG CAGCCTCTGGATTAACGTTTAGTAGCTACGCCAT GAGCTGGGTCCGCCAGGATCCAGGGAAGGGGCT GGAGTGGGTCTCAGCTATTAGTGGTAGTGGTGGT AACACATTCTACGGAGACTCCGTGAAGGGCCGG TTCACCATCTCCAGAGACAATTCCAAGAAAACGC TGTATCTGCAAATGAACAGCCTGAGAGCCGAGG ACACGGCCGTATATTACTGTGCGAAATTTTTCGG TATAGTGGGTGCCGGGTACTTTGACTACTGGGGC CAGGGAACCCTGGTCACCGTCTCCTCA |
| | | | SEQ ID NO: 25559 | SEQ ID NO: 29565 |

FIGURE 50

| | | | | |
|---|---|---|---|---|
| | | AA | EIVMTQSPATLSVSPGERATLSCRASQSVSSDLA WYQQKPGQAPRLLIYAASTRATGIPARFSGSGSG TEFTLTISSLQSEDFAVYYCQQYDNWPFTFGPGT KVDNK | EVQLLESGGGLVQPGGSLRLSCAASGLTFSSYAMS WVRQDPGKGLEWVSAISGSGGNTFYGDSVKGRFTI SRDNSKKTLYLQMNSLRAEDTAVYYCAKFFGIVGA GYFDYWGQGTLVTVSS |
| | | | SEQ ID NO: 25560 | SEQ ID NO: 29566 |
| iPS:434291 | 21-225_58A4 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCGAC TTAGCCTGGTACCAGCAGAGACCTGGCCAGGC TCCCAGGCTCCTCATCTATGCTGCATCTACCAG GGCCACTGGTATCCCAGCCAGGTTCAGTGGCA GTGGGTCTGGGACAGAGTTCACTCTCACCATC AGCAGCCTGCAGTCTGAGGATTTTGCAGTTTA TTACTGTCAGCAGTTTAATAACTGGCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTGGATTAACGTTTAGTAGCTACGCCAT GAGCTGGGTCCGCCAGGATCCAGGGAAGGGGCT GGAGTGGGTCTCAGCTATTAGTGGTAGTGGTGGT AACACATTCTACGGAGACTCCGTGAAGGGCCGG TTCACCATCTCCAGAGACAATTCCAAGAAAACGC TGTATCTGCAAATGAACAGCCTGAGAGCCGAGG ACACGGCCGTATATTACTGTGCGAAATTTTTCGG TATAGTGGGAGCCGGGTTCTTTGACTCCTGGGGC CAGGGAACCCTGGTCACCGTCTCCTCA |
| | | | SEQ ID NO: 25561 | SEQ ID NO: 29567 |
| | | AA | EIVMTQSPATLSVSPGERATLSCRASQSVSSDLA WYQQRPGQAPRLLIYAASTRATGIPARFSGSGSG TEFTLTISSLQSEDFAVYYCQQFNNWPFTFGPGT KVDIK | EVQLLESGGGLVQPGGSLRLSCAASGLTFSSYAMS WVRQDPGKGLEWVSAISGSGGNTFYGDSVKGRFTI SRDNSKKTLYLQMNSLRAEDTAVYYCAKFFGIVGA GFFDSWGQGTLVTVSS |
| | | | SEQ ID NO: 25562 | SEQ ID NO: 29568 |

FIGURE 50

| iPS:434293 | 21-225_58F5 | NA | GACATCCAGATGACCCAGTCTCCATCCTCCCT GTCTGCATCTGTAGGGGACAGAGTCACCATCA CTTGCCGGGCAAGTCAGGGCATTAGAAATGAT TTAGGCTGGTTTCTGCAGACACCAGGGAAAGC CCCTAAGCGCCTGATCTATGCTGCATCCAGTTT GCTAAGTGGGGTCCCATCACGGTTCGGCGGCA GTGGATCTGGGACAGAATTCACTCTCACAATC AGCAGCCTGCAGCCTGAAGATTTTGCAACTTA TTACTGTCTACAGCATAATAGTTACCCATTCAC TTTCGGCCCTGGGACCAAAGTGGAGATCAAA | CAGGTGCAGCTGGTGGAGTCTGGGGGAGGCGTG GTCCAGCCTGGGAGGTCCCTGAGACTCTCCTGTG TAGCGTCTGGATTCACCTTCAGTGACTATGGCAT GCACTGGGTCCGCCAGGCTCCAGGCAAGGGGCT GGAGTGGGTGGCAGTTATATGGTATGCTGGAAGT AATAAATACCATGTAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGAACTGGG GATGAGGTCTGACTACTGGGGCCAGGGAACCCT GGTCACCGTCTCCTCA |
| | | | SEQ ID NO: 25563 | SEQ ID NO: 29569 |
| | | AA | DIQMTQSPSSLSASVGDRVTITCRASQGIRNDLG WFLQTPGKAPKRLIYAASSLLSGVPSRFGGSGSG TEFTLTISSLQPEDFATYYCLQHNSYPFTFGPGTK VEIK | QVQLVESGGGVVQPGRSLRLSCVASGFTFSDYGM HWVRQAPGKGLEWVAVIWYAGSNKYHVDSVKGR FTISRDNSKNTLYLQMNSLRAEDTAVYYCARELGM RSDYWGQGTLVTVSS |
| | | | SEQ ID NO: 25564 | SEQ ID NO: 29570 |
| iPS:434295 | 21-225_58B9 | NA | GACATCGTGATGACCCAGTCTCCAGACTCCCT GGCTGTGTCTCTGGGCGAGAGGGCCACCATCA ACTGCAAGTCCGGCCAGAGTATTTTATACAGC TCCAACAATAACAACTACTTAGCTTGGTACCA GCAGAAACCAGGACAGCCTCCTAAGAAACTC ATTTACTGGGCATCTACCCGGGATTCCGGGGT CCCTGCCCGATTCAGTGGCAGCGGGTCTGGGA CAGATTTCACTCTCACCATCAGCAGCCTGCAG GCTGAAGATGTGGCAGTTTATTACTGTCAGCA ATATTATAGTACTCCTCCGACGTTCGGCCAAG GGTCCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAGCACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGTATAGCAG TGGCTGGTACTACTTTGACTACTGGGGCCAGGGA ACCCTGGTCACCGTCTCCTCA |
| | | | SEQ ID NO: 25565 | SEQ ID NO: 29571 |

FIGURE 50

| | | | | |
|---|---|---|---|---|
| | | AA | DIVMTQSPDSLAVSLGERATINCKSGQSILYSSN NNNYLAWYQQKPGQPPKKLIYWASTRDSGVPA RFSGSGSGTDFTLTISSLQAEDVAVYYCQQYYST PPTFGQGSKVEIK | QVQLVQSGAEVKKPGASVKVSCKASGYTFTNYDIN WVRQATGQGLEWMGWMNPNSGSTGYAQKFQGR VTMTRNTSISTAYMELSSLRSEDTAVYYCAYSSGW YYFDYWGQGTLVTVSS |
| | | | SEQ ID NO: 25566 | SEQ ID NO: 29572 |
| iPS:434297 | 21-225_58A10 | NA | GAAATAGTGATGACGCAGTCTCCAGCCACCCT GTCTGTGTGTCCAGGGGAAAGAGCCACCCTCT CCTGCAGGGCCAGTCAGAGTGTTAGCAGCTCC TTAGCCTGGTACCAGCAGAAACCTGGCCAGGC TCCCAGGCTCCTCATCTATGGTGCATCCACCA GGGCCACTGGTATCCCAGCCAGGTTCAGTGGC AGTGGGTCTGGGACAGAATTCACTCTCACCAT CAGCAGCCTGCAGTCTGAAGATTTTGCAGTTT ATTACTGTCAGCAGTATAATAACTGGCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGAAGCTATGCCAT GAGCTGGGTCCGCCAGGCTCCAGGGAAGGGGCT GGAGTGGGTCTCAGCTATTAGTGGTAGTGGTGGT AACACATTCTACGCAGACTCCGTGAAGGGCCGGT TCACCATCTCCAGAGACAATTCCAAGAACACGCT GTTTCTTCAAATGAACAGCCTGAGAGCCGAGGAC ACGGCCGTATATTACTGTGCGAAATTTTTCGGTA TAGTGGGAGCCGGGTACTTTGACTACTGGGGCCA GGGAACCCTGGTCACCGTCTCCTCA |
| | | | SEQ ID NO: 25567 | SEQ ID NO: 29573 |
| | | AA | EIVMTQSPATLSVCPGERATLSCRASQSVSSSLA WYQQKPGQAPRLLIYGASTRATGIPARFSGSGSG TEFTLTISSLQSEDFAVYYCQQYNNWPFTFGPGT KVDIK | EVQLLESGGGLVQPGGSLRLSCAASGFTFRSYAMS WVRQAPGKGLEWVSAISGSGGNTFYADSVKGRFTI SRDNSKNTLFLQMNSLRAEDTAVYYCAKFFGIVGA GYFDYWGQGTLVTVSS |
| | | | SEQ ID NO: 25568 | SEQ ID NO: 29574 |

FIGURE 50

| iPS:434299 | 21-225_58D11 | NA | GACATCCAGATGACCCAGTCTCCATCCTCCCT GTCTGCATCTGTAGGAGACAGAGTCACCATCA CTTGCCGGGCAAGTCAGGGCATTAGAAGTGAT TTAGACTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCGCAA TCAGCAGCCTGCGGCCTGAAGATTTTGCAACT TATTACTGTCTCCAGCATAATAATTTCCCATTC ACTTTCGGCCCTGGGACCAAGGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGACAT ACACTGGGTCCGCCAGTCTCCAGGCAAGGGGCT GGAGTGGGTGGCAGTTATATGGTATGATGGAAGT AAAAAATATTATGCAGACTCCGTGAAGGGCCGA TTCACCATCTCCAGAGACAATTCCAAGAACACGT TGTATCTGCAAATGAACAGCCTGAGAGCCGAGG ACACGGCTCTGTATTACTGTGCGAGAGATCGGGT CACTTTTGACTACTGGGGCCAGGGAACCCTGGTC ACCGTCTCCTCA |
|---|---|---|---|---|
| | | | SEQ ID NO: 25569 | SEQ ID NO: 29575 |
| | | AA | DIQMTQSPSSLSASVGDRVTITCRASQGIRSDLD WYQQKPGKAPKRLIYAASSLQSGVPSRFSGSGS GTEFTLAISSLRPEDFATYYCLQHNNFPFTFGPGT KVDIK | QVQLVESGGGVVQPGRSLRLSCAASGFTFSDYDIH WVRQSPGKGLEWVAVIWYDGSKKYYADSVKGRF TISRDNSKNTLYLQMNSLRAEDTALYYCARDRVTF DYWGQGTLVTVSS |
| | | | SEQ ID NO: 25570 | SEQ ID NO: 29576 |
| iPS:434301 | 21-225_58F11 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCGAC TTAGTCTGGTACCAGCAGAAACCTGGCCAGGC TCCCAGGCTCCTCATCTATGGTGTATCCACCA GGGCCACTGGTATCCCAGCCAGGTTCAGTGGC AGTGGGTCTGGGACAGAGTTCACTCTCACCAT CAGCAGCCTGCAGTCTGAAGATTTTGCAGTTT ATTACTGTCAGCAGTATAATAACTGGCCATTC ACTTTCGGCCCTGGGACCAAAGTGGATATCAA A | GAGGTGCAGTTGTTGGAGTCTGGGGGAGGCTTGG TACAGCCTGGGGGGGTCCCTGAGACTCTCCTGTGC AGCCTCTGGATTCACCTTTAGCAGCTATGCCATG AGCTGGGTCCGCCAGGCTCCAGGGAAGGGGCTG GAGTGGGTCTCAGCTATTAGTGGTAGTGGTGGTA ACACATTCTACGCAGACTCCGTGAAGGGCCGGTT CACCATCTCCAGATACAATTCCAAGAACACGCTG TATCTGCAAATGAACAGCCTGAGAGCCGAGGAC ACGGCCGTATATTACTGTGCGAAATTTTTCGGTA TGGTGGGAGCCGGATTCTTTGACTACTGGGGCCA GGGAACCCTGGTCACCGTCTCCTCA |
| | | | SEQ ID NO: 25571 | SEQ ID NO: 29577 |

1884

FIGURE 50

| | | | | |
|---|---|---|---|---|
| | | AA | EIVMTQSPATLSVSPGERATLSCRASQSVSSDLV WYQQKPGQAPRLLIYGVSTRATGIPARFSGSGSG TEFTLTISSLQSEDFAVYYCQQYNNWPFTFGPGT KVDIK | EVQLLESGGGLVQPGGSLRLSCAASGFTFSSYAMS WVRQAPGKGLEWVSAISGSGGNTFYADSVKGRFTI SRYNSKNTLYLQMNSLRAEDTAVYYCAKFFGMVG AGFFDYWGQGTLVTVSS |
| | | | SEQ ID NO: 25572 | SEQ ID NO: 29578 |
| iPS:434303 | 21-225_58H11 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GAGGGAAAGACCTATTTGTATTGGTACGTGCA GAAGCCAGGCCAGCCTCCACAACTCCTGATCT ATGAAGTTTCCTATCGGTTTTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACCATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGCGGTATAG CAGCAGCTGGGACGGGGGGTATGGACGTCTGGGG CCAAGGGACCACGGTCACCGTCTCCTCA |
| | | | SEQ ID NO: 25573 | SEQ ID NO: 29579 |
| | | AA | DIVMTQTPLSLSVTPGQPASISCKSSQSLLHSEGK TYLYWYVQKPGQPPQLLIYEVSYRFSGVPDRFS GSGSGTDFTLKISRVEAEDVGVYYCMQSIQLPLT FGGGTKVEIK | QVQLVESGGGVVQPGRSLRLSCAASGFTFSSYGMH WVRQAPGKGLEWVAVIWYDGSNKYHADSVKGRF TISRDNSKNTLYLQMNSLRAEDTAVYYCARRYSSS WDGGMDVWGQGTTVTVSS |
| | | | SEQ ID NO: 25574 | SEQ ID NO: 29580 |

FIGURE 50

| iPS:434305 | 21-225_59E1 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATAACAACTACTTAGCTTGGTACCA GCAGAGACCAGGACAGCCTCCTAAGTTGCTCA TTTACTGGTCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTTTAGTATTCCGTGCAGTTTTGGCCAGGGG ACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGACTCCTAACAGTGG TAACACAGGCTATGCACAGAAGTTCCAGGGCAG AGTCACCATGACCAGGACCACCTCCATAAGCAC AGCCTACATGGAGCTGAGCAGCCTGAGATCTGA GGACACGGCCGTGTATTACTGTGCGTTTAGCAGT GGCTGGTACTTCTTTGACTACTGGGGCCAGGGAA CCCTGGTCACCGTCTCCTCA |
|---|---|---|---|---|
| | | | SEQ ID NO: 25575 | SEQ ID NO: 29581 |
| | | AA | DIVMTQSPDSLAVSLGERATINCKSSQSVLYSSN NNNYLAWYQQRPGQPPKLLIYWSSTRESGVPDR FSGSGSGTDFTLTISSLQAEDVAVYYCQQYFSIPC SFGQGTKLEIK | QVQLVQSGAEVKKPGASVKVSCKASGYTFTNYDIN WVRQATGQGLEWMGWMTPNSGNTGYAQKFQGR VTMTRTTSISTAYMELSSLRSEDTAVYYCAFSSGW YFFDYWGQGTLVTVSS |
| | | | SEQ ID NO: 25576 | SEQ ID NO: 29582 |
| iPS:434307 | 21-225_59B2 | NA | GAAATTGTGTTGACGCAGTCTCCAGGCACCCT GTCTTTGTCTCCAGGGGAAAGAGCCACCCTCT CCTGCTGGGCCAGTCAGAGTGTTTACAGCAGC TTCTTAGCCTGGTTCCAGCAGAAATCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTTTTACTGTCAGCAATATGGTACCTCACCGT GGACGTTCGGCCAAGGGACCAAGGTGGAAAT CAAA | CAGGTGCAGTTGGTGCAGTCTGGGGGCTGAGGTGA AGAAGCCTGGGGGCCTCAGTGAAGGTCTCCTGCA AGGCTTCTGGATACACCTTCGCCGGCTACTATAT ACACTGGGTGCGACAGGCCCCTGGACAAGGACT TGAGTGGTTGGGTTGGATCAACCCTAACAGTGGT GGCACAAACTATGCACAGAAGTTTCAGGGCAGG GTCACCATGACCAGGGACACGTCCATCAGCACA GCCTACATGGAGCTGAGCAGGCTGAGATTTGAC GACACGGCCGTGTATTACTGTGCGAGAGATCCGG GGCCCTTTGACTACTGGGGCCAGGGAACCCTGGT CACCGTCTCCTCA |
| | | | SEQ ID NO: 25577 | SEQ ID NO: 29583 |

FIGURE 50

| | | | | |
|---|---|---|---|---|
| | | AA | EIVLTQSPGTLSLSPGERATLSCWASQSVYSSFLA WFQQKSGQAPRLLIYGASSRATGIPDRFSGSGSG TDFTLTISRLEPEDFAVFYCQQYGTSPWTFGQGT KVEIK | QVQLVQSGAEVKKPGASVKVSCKASGYTFAGYYI HWVRQAPGQGLEWLGWINPNSGGTNYAQKFQGR VTMTRDTSISTAYMELSRLRFDDTAVYYCARDPGP FDYWGQGTLVTVSS |
| | | | SEQ ID NO: 25578 | SEQ ID NO: 29584 |
| iPS:434309 | 21-225_59B5 | NA | GACATCCAGATGACCCAGTCTCCATCCTCCCT GTCTGCATCTGTAGGAGACAGAGTCACCATCA CTTGCCGGGCAAGTCAGAGCATTATCAGCTAT TTAAAATTGGTATCAGCAGAAACCAGGGAAAG CCCCTAAACTCCTGATCTATGGTGCATCCAGTT TGCAGAGTGGGGTCCCATCAAGGTTCAGTGGC AGTGGATCCGGGACAGATTTCACTCTCACCAT CAGCAGTCTGCAACCTGAAGATTTTGCAACTT ACTACTGTCAACAGAGTTACAGTACCCCTATG TTCAGTTTTGGCCAGGGGACCAAGCTGGAGAT CAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGCTATTAGTGGTAGTGGTGGT AACACATTCTACGCAGACTCCGTGAAGGGCCGGT TCACCATTTCCAGAGACAATTCCAAGAACACGCT GTATCTGCAAATGAACAGCCTGAGAGCCGAGGA CACGGCCGTATATTACTGTGCGAAAAGGGGGGGT CTACGGTGACTACGAGGCTTTTGATATCTGGGGC CAAGGGACAATGGTCACCGTCTCTTCA |
| | | | SEQ ID NO: 25579 | SEQ ID NO: 29585 |
| | | AA | DIQMTQSPSSLSASVGDRVTITCRASQSIISYLNW YQQKPGKAPKLLIYGASSLQSGVPSRFSGSGSGT DFTLTISSLQPEDFATYYCQQSYSTPMFSFGQGT KLEIK | EVQLLESGGGLVQPGGSLRLSCAASGFTFSSYAMN WVRQAPGKGLEWVSAISGSGGNTFYADSVKGRFTI SRDNSKNTLYLQMNSLRAEDTAVYYCAKRGVYGD YEAFDIWGQGTMVTVSS |
| | | | SEQ ID NO: 25580 | SEQ ID NO: 29586 |

FIGURE 50

| | | | | |
|---|---|---|---|---|
| iPS:434311 | 21-225_59H5 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAGCATC TACTTAGCCTGGTTCCTGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTGAC CATCAGCAGACTGGAGCCTGAAGATTTTGCAG TGTATTACTGTCACCAGTATGGTAACTCACCA TTCACTTTCGGCCCTGGGACCAAAGTGGATTT CAAA | CAGGTACAACTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGG GTATAGCAGTGGGGTACTACGGTATGGACGTCTG GGGCCAAGGGACCACGGTCACCGTCTCCTCA |
| | | | SEQ ID NO: 25581 | SEQ ID NO: 29587 |
| | | AA | EIVLTQSPGTLSLSPGERATLSCRASQSVSSIYLA WFLQKPGQAPRLLIYGASSRATGIPDRFSGSGSG TDFTLTISRLEPEDFAVYYCHQYGNSPFTFGPGT KVDFK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARERGIA VGYYGMDVWGQGTTVTVSS |
| | | | SEQ ID NO: 25582 | SEQ ID NO: 29588 |
| iPS:434313 | 21-225_59E6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAGGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGAAGTAGTTA CTACTGGGGCTGGATCCGCCAGCCCCCAGGGAA GGGGCTGGAGTGGATTGGGAATATCTATTATAGT GGGAGCACCTACTACAACCCGTCCCTCAAGAGTC GAGTCACCATATCCGTAGACACGTCCAAGAACC AGTTCTCCCTGAAGCTGAGCTCTGTGACCGCCGC AGACACGGCTCTGTATTACTGTGCGAGACATAGC AGCAGCTGGTCCCTTGACTACTGGGGCCAGGGA ACCCTGGTCACCGTCTCCTCA |
| | | | SEQ ID NO: 25583 | SEQ ID NO: 29589 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TRVEIK | QLQLQESGPGLVKPSETLSLTCTVSGGSISRSSYYW GWIRQPPGKGLEWIGNIYYSGSTYYNPSLKSRVTIS VDTSKNQFSLKLSSVTAADTALYYCARHSSSWSLD YWGQGTLVTVSS |
| | | | SEQ ID NO: 25584 | SEQ ID NO: 29590 |
| iPS:434315 | 21-225_59G7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATTCTGCATCCAGTT TACAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGTGG ACGTTCGGCCAAGGGACCAAGGTGGAAATCA AA | CAGGTGCAACTAGTGCAGTCTGGGGGCTGAGGTG AAGAAGCCTGGGGCCTCAGTGAAGGTCTCCTGC AAGGCTTCTGGATACAGTTTCACCGGCCACTATA TACACTGGGTGCGACAGGCCCCTGGACAAGGGC TTGAGTACATGGGATGGATCAACCCGAACAGTG GTGGCACAAACTATGTACAGAAATTTCAGGGCA GGGTCACCATGACCAGGGACACGTCCATCAGCA CAGCCTACATGGAGCTGAGCAGGCTGAGATCTG ACGACACGGCCATATATTACTGTTCGAGAGGGG GCCTACTGGGAGCTACCAACTACTACTACTACGG TATGGACGTCTGGGGCCAAGGGACCACGGTCAC CGTCACCTCA |
| | | | SEQ ID NO: 25585 | SEQ ID NO: 29591 |
| | | AA | DIQMTQSPSSLSASVGDRVTITCRASQGIRNDLG WYQQKPGKAPKRLIYSASSLQSGVPSRFSGSGSG TEFTLTISSLQPEDFATYYCLQHNSYPWTFGQGT KVEIK | QVQLVQSGAEVKKPGASVKVSCKASGYSFTGHYIH WVRQAPGQGLEYMGWINPNSGGTNYVQKFQGRV TMTRDTSISTAYMELSRLRSDDTAIYYCSRGGLLGA TNYYYYGMDVWGQGTTVTVTS |
| | | | SEQ ID NO: 25586 | SEQ ID NO: 29592 |

FIGURE 50

| iPS:434317 | 21-225_59E8 | NA | GACATCCAGATGACCCAGTCTCCATCCTCCCTGTCTGCATCTGTAGGAGACAGAGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTTCAGTAATTCGATCACCTTCGGCCAAGGGACACGACTGGAGATTAAA | GAGGTGCAGCTGGTGGAGTCTGGGGGAGGCTTGGTACAGCCTGGGGGGTCCCTGAGACTCTCCTGTGCAGCCTCTGGATTCACTTTCAGTAGCTATAGCATGAATTGGGTCCGCCAGGCTCCAGGGAAGGGGCTGGGGTGGGTGTCATACATTAGTAGTAGTAGTGGGACCATATACTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGAATGGGGAATGGCAGTGGCTGGCCCGTTTGACTACTGGGGCCAGGGAACCCTGGTCACCGTCTCCTCA |
|---|---|---|---|---|
| | | | SEQ ID NO: 25587 | SEQ ID NO: 29593 |
| | | AA | DIQMTQSPSSLSASVGDRVTITCRASQSISSYLNWYQQKPGKAPKLLIYAASSLQSGVPSRFSGSGSTDFTLTISSLQPEDFATYFCQQSFSNSITFGQGTRLEIK | EVQLVESGGGLVQPGGSLRLSCAASGFTFSSYSMNWVRQAPGKGLGWVSYISSSSGTIYYADSVKGRFTISRDNAKNSLYLQMNSLRDEDTAVYYCAREWGMAVAGPFDYWGQGTLVTVSS |
| | | | SEQ ID NO: 25588 | SEQ ID NO: 29594 |
| iPS:434319 | 21-225_59B9 | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGCACCCAGGGAAAGCCCCTAAGCGCCTGATCTATGCTGCATCCAGTTTACAAAGTGGGGTCCCATCAAGGTTCAGCGGCACTAGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGCAGTCTGGGCCTGAGGTGAAGAAGCCTGGGGCCTCAGTGAAGGTCTCCTGCAAGGCTTCTGGATACATCTTCACCGGCAATTATATACACTGGGTGCGACAGGCCCCTGGACAAGGGCTTGAGTACATGGGATGGATCAACCCTAACAGTGGTGGCACAAACTATGTACAGAAGTTTCAGGGCAGGGTCACCATGACCAGGGACACGTCCATCAGCACAGCCAACATGGAACTGACCAGTCTGAGATCTGACGACACGGCCGTGTATTACTGTTCGAGAGGGGGCCTACTGGGAGCTACCTACTACTACTACGGTATGGACGTCTGGGGGCCAAGGGACCACGGTCACCGTCTCCTCA |
| | | | SEQ ID NO: 25589 | SEQ ID NO: 29595 |

FIGURE 50

| | | | | |
|---|---|---|---|---|
| | | AA | DIQMTQSPSSLSASVGDRVTITCRASQDIRNDLG WYQQHPGKAPKRLIYAASSLQSGVPSRFSGTRS GTEFTLTISSLQPEDFATYYCLQHNSYPWTFGQG TKVEIK | QVQLVQSGPEVKKPGASVKVSCKASGYIFTGNYIH WVRQAPGQGLEYMGWINPNSGGTNYVQKFQGRV TMTRDTSISTANMELTSLRSDDTAVYYCSRGGLLG ATYYYYYGMDVWGQGTTVTVSS |
| | | | SEQ ID NO: 25590 | SEQ ID NO: 29596 |
| iPS:434321 | 21-225_59F10 | NA | GACATCGTGATGACCCAGTTTCCAGACTCCCT GGCTGTGTCTCTGGGCGAGAGGGCCACCATCA ACTGCAAGTCCAGCCAGACTGTTTTATACAGG TCCAACAATTACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTTTAGTACTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACAAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATAACTGTGCGGTTAGCA GTGGCTGGTACTACTTTGACTACTGGGGCCAGGG AACCCTGGTCACCGTCTCCTCA |
| | | | SEQ ID NO: 25591 | SEQ ID NO: 29597 |
| | | AA | DIVMTQFPDSLAVSLGERATINCKSSQTVLYRSN NYNYLAWYQQKPGQPPKLLIYWASTRESGVPD RFSGSGSGTDFTLTISSLQAEDVAVYYCQQYFST PPTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYNCAVSSGW YYFDYWGQGTLVTVSS |
| | | | SEQ ID NO: 25592 | SEQ ID NO: 29598 |

FIGURE 50

| iPS:434323 | 21-225_62H8 | NA | GACATCCAGATGACCCAGTCTCCATCCTCCCTGTCTGCATCTGTAGGAGACAGAGTCACCATCACTTGCCGGGCAAGTCAGAGCATTTTCAGCTATTTAAATTGGTATCAGCAGAATCCGGGGAAAGCCCCTAAGCTCCTGATCTATGCGTCATCCAGTTTGCAAAGTGGGGTCCCATCAAGGTTAAGTGGCAATGGATCTGGGACAGATTTCATTCTCACCATCAGCAGTCTGCAACCTGAAGATTTTGCAACTTACTACTGTCAACAGAGTTACAGTACCCCATTCACTTTCGGCCCTGGGACCAGAGTGGATATCAAA | CAGGTGCAGCTGGTGGAATCTGGGGGAGGCGTGGTCCAGCCTGGGAGGTCCCTGAGACTCTCCTGTGCAGCGTCTGGATTCACCTTCAGCACCTATGGCATGCACTGGGTCCGCCAGGCTCCAGGCAAGGGGCTGGAGTGGGTGGCAGTTATATGGCATGATGGAAGTGATAAATATTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAGACCCGCGTACCAGCTGCTCTGACTACTGGGGCCAGGGAACCCTGGTCACCGTCTCCTCA |
|---|---|---|---|---|
| | | | SEQ ID NO: 25593 | SEQ ID NO: 29599 |
| | | AA | DIQMTQSPSSLSASVGDRVTITCRASQSIFSYLNWYQQNPGKAPKLLIYASSSLQSGVPSRLSGNGSGTDFILTISSLQPEDFATYYCQQSYSTPFTFGPGTRVDIK | QVQLVESGGGVVQPGRSLRLSCAASGFTFSTYGMHWVRQAPGKGLEWVAVIWHDGSDKYYVDSVKGRFTISRDNSKNTLYLQMNSLRAEDTAVYYCAREDPRTSCSDYWGQGTLVTVSS |
| | | | SEQ ID NO: 25594 | SEQ ID NO: 29600 |
| iPS:434327 | 21-225_63G6 | NA | GACATCCAGATGACCCAGTCTCCATCCTCCCTGTCTGCATCTGTAGGAGACAGAGTCATCATCTCTTGCCGGGCAAGTCAGAGCATTTTCAGCTATTTAAATTGGTATCAGGTGAAACCAGGGAAAGCCCCTAAGCTCCTGATCTATGATACATCCACTTTGCAAACTGGGGTCCCATCAAGGTTCAGTGGCAGTGGATCTGGGACAGATTTCACTCTCACCATCAACAGTCTGCAACCTGAAGATTTTGCAACTTACTACTGTCAACAGAGTTACGGTATCCCCATCACCTTCGGCCAAGGGACACGACTGGAGATTCAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTAGAATGGGTGACAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATAGCCTCTCGGGTATAGCAGCAGCTTTTGACTACTGGGGCCAGGGAACCCTGGTCACCGTCTCCTCA |
| | | | SEQ ID NO: 25595 | SEQ ID NO: 29601 |

FIGURE 50

| | | | | |
|---|---|---|---|---|
| | | AA | DIQMTQSPSSLSASVGDRVIISCRASQSIFSYLNW YQVKPGKAPKLLIYDTSTLQTGVPSRFSGSGSGT DFTLTINSLQPEDFATYYCQQSYGIPITFGQGTRL EIQ | QVQLVESGGGVVQPGRSLRLSCAASGFTFSSYGMH WVRQAPGKGLEWVTVIWYDGSNKYYADSVKGRF TISRDNSKNTLYLQMNSLRAEDTAVYYCARDSLSG IAAAFDYWGQGTLVTVSS |
| | | | SEQ ID NO: 25596 | SEQ ID NO: 29602 |
| IPS:434331 | 21-225_63H8 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCATAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAATATCATAGTTACCCATTCA CTTTCGGCCCTGGGACCAAAGTGGATATCAGA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAACAT GAACTGGGTCCGCCAGGCTCCAGGGAAGGGGCT GGAGTGGGTCTCATCCATTAGTGGTAGTAGCACT TACATGAACTACACAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGACTACGTAA TTTTGACTACTGGGGCCAGGGAACCCTGGTCACC GTCTCCTCA |
| | | | SEQ ID NO: 25597 | SEQ ID NO: 29603 |
| | | AA | DIQMTQSPSSLSASVGDRVTITCRASQGISNYLA WFQQKPGKAHKSLIYAASSLQSGVPSKFSGSGS GTDFTLTISSLQPEDFATYYCQQYHSYPFTFGPG TKVDIR | EVQLVESGGGLVKPGGSLRLSCAASGFTFSSYNMN WVRQAPGKGLEWVSSISGSSTYMNYTDSVKGRFTI SRDNAKNSLYLQMNSLRAEDTAVYYCARLRNFDY WGQGTLVTVSS |
| | | | SEQ ID NO: 25598 | SEQ ID NO: 29604 |

FIGURE 50

| iPS:434333 | 21-225_63C9 | NA | GACATCCAGATGACCCAGTCTCCATCTTCCGT GTCTGCATCTGTCGGAGACAGAGTCACCATCA CTTGTCGGGCGAGTCAGGGTATTAGCAGCTGG TTAGCCTGGTATCAGCAGAAACCAGGGAAAG CCCCTAACCTCCTGATCTATGCTGCATCCAGTT TGCAAAGTGGGGTCCCATCAAGGTTCAGCGGC AGTGGATCTGGGACAGATTTCACGCTCACCAT CAGCGGCCTGCAGCCTGAAGATTTTGCAACTT ACTTTTGTCAACAGATTAACAGTTTCCCTCTCA CTTTCGGCGGAGGGACCAAGGTGGCGATCAA A | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTTTGCACAGAAGTTTCAGGGCAG GGTCACCATGACCAGGGACGCGTCCATCAACAC AGCCTACATGGAGCTGCGCAGCCTGATATCTGAC GACACGGCCGTATATTACTGTGCGAGAGCTCCGG GTGTAGCAGCAGCTGGTTCATGGGGATACTTTGA CTACTGGGGCCAGGGAACCCTGGTCACCGTCTCC TCA |
| | | | SEQ ID NO: 25599 | SEQ ID NO: 29605 |
| | | AA | DIQMTQSPSSVSASVGDRVTITCRASQGISSWLA WYQQKPGKAPNLLIYAASSLQSGVPSRFSGSGS GTDFTLTISGLQPEDFATYFCQQINSFPLTFGGGT KVAIK | QVQLVQSGAEVKKPGASVKVSCKASGYTFTGYYM HWVRQAPGQGLEWMGWINPNSGGTNFAQKFQGR VTMTRDASINTAYMELRSLISDDTAVYYCARAPGV AAAGSWGYFDYWGQGTLVTVSS |
| | | | SEQ ID NO: 25600 | SEQ ID NO: 29606 |
| iPS:434335 | 21-225_63C10 | NA | GACATCCAGATGACCCAGTCTCCGTCCTCCCT GTCTACATCTGTAGGAGACAGAGTCACCATCA CTTGCCGGGCAAGTCAGAGCATTTTCAGCTAT TTACATTGGTATCAGCAGAAACCAGGGAAAGC CCCTAAGCTCCTGATCTCTGCTGCATCCAGTTT ACAAAGTGGGGTCCCATCAAGGTTCAGTGGCA GTGGATCTGGGACAGATTTCACTCTCACCATC AGTAGTCTGCAACCTGAAGATTTTGCAACTTT CTACTGTCAACAGACTTACAGTCCCCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGACTGGGTGGCAGTCATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATGATCC CAGATCCTCGGCCGGGGACTACTGGGGCCAGGG AACCCTGGTCACCGTCTCCTCA |
| | | | SEQ ID NO: 25601 | SEQ ID NO: 29607 |

FIGURE 50

| | | | | |
|---|---|---|---|---|
| | | AA | DIQMTQSPSSLSTSVGDRVTITCRASQSIFSYLHW YQQKPGKAPKLLISAASSLQSGVPSRFSGSGSGT DFTLTISSLQPEDFATFYCQQTYSPPLTFGGGTKV EIK | QVQLVESGGGVVQPGRSLRLSCAASGFTFSSYGMH WVRQAPGKGLDWVAVIWYDGSNKYYADSVKGRF TISRDNSKNTLYLQMNSLRAEDTAVYYCARDDPRS SAGDYWGQGTLVTVSS |
| | | | SEQ ID NO: 25602 | SEQ ID NO: 29608 |
| IPS:434337 | 21-225_64E1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCACGGTTCAGCGG CAGTAAATCTGGGACAGAATTCACTCTCACAA TCAGCAGCTTGCAGCCTGAAGATCTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG TAGCGTCTGGATTCACCTTCAGTAGCTATGGCAT GCACTGGGTCCGCCAGGCTCCAGGCAAGGGGCT GGAGTGGGTGGCAGTTATATGGTTTGATGAAACT AATAAATACTATGGAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGCTTGG GTTCAGCAGTGACTATTGGGGCCAGGGAACCCTG GTCACCGTCTCCTCA |
| | | | SEQ ID NO: 25603 | SEQ ID NO: 29609 |
| | | AA | DIQMTQSPSSLSASVGDRVTITCRASQGIRNDLG WYQQKPGKAPKRLIYAASSLQSGVPSRFSGSKS GTEFTLTISSLQPEDLATYYCLQHNSYPLTFGGG TKVEIK | QVQLVESGGGVVQPGRSLRLSCVASGFTFSSYGMH WVRQAPGKGLEWVAVIWFDETNKYYGDSVKGRF TISRDNSKNTLYLQMNSLRAEDTAVYYCARELGFS SDYWGQGTLVTVSS |
| | | | SEQ ID NO: 25604 | SEQ ID NO: 29610 |

EP 4 435 105 A2

FIGURE 50

| iPS:434339 | 21-225_64A4 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAACGCCTGATCTATGCTGCATCCAGTTTGCAAAGTGGGGTCCCATCAAGGTTCAGCGGCAGTGGATCTGGGACAGAATTCACTCTCACAATCAGCAGCCTACAGCCTGAAGATCTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGCGATTATGTCATGCACTGGGTCCGCCAGGCTCCAGGCAAGGGGCTGGAGTGGGTGGCAGTCATATGGTATGATGGAAGTAATAAATACTATGCAGACTCCGTGAAGGGCCGATTCACCATCTCCAGAGACAATTCCCAGAACACGCTGTATCTGCAAATGAATAGCCTGAGAGCCGAGGACACGGCTGTGTATTACTGTGCGAGAGAAAGGTATAGCAGCAGCTGGTACGACTACGGTATGGACGTCTGGGGCCAAGGGACCACGGTCACCGTCTCCTCA |
| | | | SEQ ID NO: 25605 | SEQ ID NO: 29611 |
| | | AA | DIQMTQSPSSLSASVGDRVTITCRASQGIRNDLGWYQQKPGKAPKRLIYAASSLQSGVPSRFSGSGSGTEFTLTISSLQPEDLATYYCLQHYSYPRTFGQGTKVEIK | QVQLVESGGGVVQPGRSLRLSCAASGFTFSDYVMHWVRQAPGKGLEWVAVIWYDGSNKYYADSVKGRFTISRDNSQNTLYLQMNSLRAEDTAVYYCARERYSSSWYDYGMDVWGQGTTVTVSS |
| | | | SEQ ID NO: 25606 | SEQ ID NO: 29612 |
| iPS:434341 | 21-225_64F7 | NA | GACATCCAGATGACCCAGTCTCCATCCTCCCTGTCTGCATCTGTAGGAGACAGAGTCACCATCACTTGCCGGGCAAGTCAGAACATTAAGAAATATTTAAATTGGTATCAGCAGAAACCAGGGAAAGCCCCTAAGTTCCTGATCTATGGTGCATCCAGTTTGCAAAGTGGGGTCCCATCAAGGTTCAGTGGCAGTGGATCTGGGACAGATTTCACTCTCACCATCAGTAGTCTGCAACCTGAAGATTTTGCAGCTTACTACTGTCAACAGAGTTACAATATTTCGTTCACTTTCGGCGGAGGGACCAAGGTGGAGCTCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTACTTCTGGAGCTGGATCCGGCAGCCCGCCGGGAAGGGACTGGAGTGGATTGGGCGTATCTATACCAGTGGGATCTCCAACTACAATCCCTCCCTCAAGAGTCGAGTCACCATGTCAGTTGACACGTCCAAGAACCAGTTCTCCCTGAAGCTGAGCTCTGTGACCGCCGCGGACACGGCCGTGTATTACTGTGCGAGGTTTAGCAGTGGCTTTTTTGACTACTGGGGCCAGGGTACCCTGGTCACCGTCTCCTCA |
| | | | SEQ ID NO: 25607 | SEQ ID NO: 29613 |

1896

FIGURE 50

| | | | | |
|---|---|---|---|---|
| | | AA | DIQMTQSPSSLSASVGDRVTITCRASQNIKKYLN WYQQKPGKAPKFLIYGASSLQSGVPSRFSGSGSG TDFTLTISSLQPEDFAAYYCQQSYNISFTFGGGTK VELK | QVQLQESGPGLVKPSETLSLTCTVSGGSISSYFWSW IRQPAGKGLEWIGRIYTSGISNYNPSLKSRVTMSVD TSKNQFSLKLSSVTAADTAVYYCARFSSGFFDYWG QGTLVTVSS |
| | | | SEQ ID NO: 25608 | SEQ ID NO: 29614 |
| iPS:434343 | 21-225_64C8 | NA | GACATCCAGATGACCCAGTCTCCATCCTCCCT GTCTGCAGCTGTAGGAGACAGAGTCACCATCA CTTGCCGGGCAAGTCAGGGCATTAGAAATGAT TTAGGCTGGTATCAGCAGAAACCAGGGAAAG CCCCTAAGTGCCTGATCTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTATTGTCTACACCATTATAGTTACCCTCGGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGCAGACTCCATGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACGGTA TAGCAGTGGCTGGTACGACTACGGTATGGACGTC TGGGGCCAAGGGACCACGGTCACCGTCTCCTCA |
| | | | SEQ ID NO: 25609 | SEQ ID NO: 29615 |
| | | AA | DIQMTQSPSSLSAAVGDRVTITCRASQGIRNDLG WYQQKPGKAPKCLIYAASSLQSGVPSRFSGSGS GTEFTLTISSLQPEDFATYYCLHHYSYPRTFGQG TKVEIK | QVQLVESGGGVVQPGRSLRLSCAASGFTFSDYVM HWVRQAPGKGLEWVAVIWYDGSNKYYADSMKG RFTISRDNSKNTLYLQMNSLRAEDTAVYYCARERY SSGWYDYGMDVWGQGTTVTVSS |
| | | | SEQ ID NO: 25610 | SEQ ID NO: 29616 |

FIGURE 50

| iPS:434345 | 21-225_64H9 | NA | GAAATTGTGATGACCCAGACTCCACTCTCTCT GTCCGTCACCCCTGGACAGCCGGCCTCCATCT CCTGCAAGTCTAGTCAGAGCCTCCTTCATGGT GATGGAAAGACCTATTTGTTTTGGTACCTGCA GAAGCCAGGCCAGCCTCCACAGGTCCTGATCT ATGAAGTTTCCAACCGGTTGTGTGGAGTGCCA GACAGGTTCAGTGGCAGCGGGTCAGGGACAG ATTTCTCATTGAAAATCAGCCGGGTGGAGGCT GAGGACGTTGGGGTTTATTACTGCATGCAAAG TATACAGGTTCCGTGGACGTTCGGCCAAGGGA CCAAGGTGGAAATCACA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TATATCTACAAATGAACAGCCTGAGAGCCGAGG ACACGGCTGTGTATTACTGTGCGAGAGATACATA CGATTTTTGGAGTGGTTATTTGGGCTACTGGGGC CAGGGAACCCTGGTCACCGTCTCCTCA |
| | | | SEQ ID NO: 25611 | SEQ ID NO: 29617 |
| | | AA | EIVMTQTPLSLSVTPGQPASISCKSSQSLLHGDGK TYLFWYLQKPGQPPQVLIYEVSNRLCGVPDRFS GSGSGTDFSLKISRVEAEDVGVYYCMQSIQVPW TFGQGTKVEIT | QVQLVESGGGVVQPGRSLRLSCAASGFTFSTYGMH WVRQAPGKGLEWVAIIWYDGSNKYYADSVKGRFT ISRDNSKNTLYLQMNSLRAEDTAVYYCARDTYDF WSGYLGYWGQGTLVTVSS |
| | | | SEQ ID NO: 25612 | SEQ ID NO: 29618 |
| iPS:434347 | 21-225_64H10 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCTTAAGCTCTTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGATTAACAGTTTCCCTCTCA CTTTCGGCGGAGGGACCAAGGTGGAGATCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAAACCAAACAGTG GTGGCACAAACCAAGCACAGAAGTTTCAGGGCA GGGTCACCATGACCAGGGACACGTCCATCAGCA CAGCCTACATGGAGCTGAGCGGGCTGAGATCTG ACGACACGGCCGTGTATTACTGTGCGAGAGCTCC GGGTACTGCAGCAACTGGTACATGGGGATACTTT GACTACTGGGGCCAGGGAACCCTGGTCACCGTCT CCTCA |
| | | | SEQ ID NO: 25613 | SEQ ID NO: 29619 |

FIGURE 50

| | | | | |
|---|---|---|---|---|
| | | AA | DIQMTQSPSSVSASVGDRVTITCRASQGISSWLA WYQQKPGKALKLLIYAASSLQSGVPSRFSGSGS GTDFTLTISSLQPEDFATYYCQQINSFPLTFGGGT KVEIK | QVQLVQSGAEVKKPGASVKVSCKASGYTFTGYYM HWVRQAPGQGLEWMGWIKPNSGGTNQAQKFQGR VTMTRDTSISTAYMELSGLRSDDTAVYYCARAPGT AATGTWGYFDYWGQGTLVTVSS |
| | | | SEQ ID NO: 25614 | SEQ ID NO: 29620 |
| IPS:434351 | 21-225_64A12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTACACGCCTGATCTATACTGCATCCACTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATGGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAGT AATAAATACTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGGGAACTCGGG TTCCTCTCTGACCACTGGGGGCCAGGGAACCCTGG TCACCGTCTCCTCA |
| | | | SEQ ID NO: 25615 | SEQ ID NO: 29621 |
| | | AA | DIQMTQSPSSLSASVGDRVTITCRASQGIRNDLG WYQQKPGKAPTRLIYTASTLQSGVPSRFSGSGSG TEFTLTISSLQPEDFATYYCLQHNGYPFTFGPGTK VDIK | QVQLVESGGGVVQPGRSLRLSCAASGFTFSSYGMH WVRQAPGKGLEWVAVIWYDESNKYYVDSVKGRF TISRDNSKNTLYLQMNSLRAEDTAVYYCARELGFL SDHWGQGTLVTVSS |
| | | | SEQ ID NO: 25616 | SEQ ID NO: 29622 |

FIGURE 50

| iPS:434353 | 21-225_64B12 | NA | GACATCCAGATGACCCAGTCTCCATCCTCCCT GTCTGCATCTGTAGGAGACAGAGTCACCATCA CTTGCCGGGCGAGTCAGGACATTAGCAATTAT TTAAATTGGTATCAGCAGAAACCAGGGACAGC CCCTAACCTCCTGATCTCTGATGCATCCATTTT GGAAACAGGGGTCCCATCAACGTTCAGTGGA AGTGGATCTGGGACAGATTTTACTTTCACCAT CAGCAGCCTGCAGCCTGAAGATATTGCAACAT ATTACTGTCAACAGAGTGATAATCTCCCGTGC AGTTTTGGCCAGGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGTCTCCATCAGCAGAAGTAGTTA CTACTGGGGCTGGATCCGCCAGCCCCCAGGGAA GGGGCTGGAGTGGATTGGGAGTATCTATTACAGT GGGAGCACCTCCTACAACCCGTCCCTCAAGAGTC GAGTCACCATATCCGTAGACACGTCCAAGAACC AGTTCTCCCTGAAGCTGAGCTCTGTGACCGCCGC AGACACGGCTGTGTATTATTGTGCGAGACTGGAC AGTGGCTGGTCCTTTGACTACTGGGGCCAGGGAA CCCTGGTCACCGTCTCCTCA |
|---|---|---|---|---|
| | | | SEQ ID NO: 25617 | SEQ ID NO: 29623 |
| | | AA | DIQMTQSPSSLSASVGDRVTITCRASQDISNYLN WYQQKPGTAPNLLISDASILETGVPSTFSGSGSG TDFTFTISSLQPEDIATYYCQQSDNLPCSFGQGTK VEIK | QLQLQESGPGLVKPSETLSLTCTVSGVSISRSSYYW GWIRQPPGKGLEWIGSIYYSGSTSYNPSLKSRVTISV DTSKNQFSLKLSSVTAADTAVYYCARLDSGWSFD YWGQGTLVTVSS |
| | | | SEQ ID NO: 25618 | SEQ ID NO: 29624 |
| iPS:434355 | 21-225_64G12 | NA | GACATCCAGATGACCCAGTCTCCCTCTTCCGT GTCTGCATCTGTAGGAGACAGAGTCACCATCA CTTGTCGGGCGAGTCAGAATATTACCACCTGG TTAGCCTGGTATCAGCAGAAACCAGGGAAAG CCCCTAAACTCCTGATCTCTGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ATATTTGTCAACAGGCTAACAGTTTTCCATTCA CTTTCGGCCCTGGGACCAAACTGGATATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGTAATGCCAT GAGCTGGGTCCGCCAGGCTCCAGGGAAGGGGCT GGAGTGGGTCTCAGTTATTAGTGGTAGTGGTGGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCTTATATTACTGTGCGAAAAGGAACTA CGACGATGCTTTTGATATCTGGGGCCAAGGGACA ATGGTCACCGTCTCTTCA |
| | | | SEQ ID NO: 25619 | SEQ ID NO: 29625 |

FIGURE 50

| | | | | |
|---|---|---|---|---|
| | | AA | DIQMTQSPSSVSASVGDRVTITCRASQNITTWLA WYQQKPGKAPKLLISAASSLQSGVPSRFSGSGSG TDFTLTISSLQPEDFATYICQQANSFPFTFGPGTK LDIK | EVQLLESGGGLVQPGGSLRLSCAASGFTFSSNAMS WVRQAPGKGLEWVSVISGSGGSTYYADSVKGRFTI SRDNSKNTLYLQMNSLRAEDTALYYCAKRNYDDA FDIWGQGTMVTVSS |
| | | | SEQ ID NO: 25620 | SEQ ID NO: 29626 |
| IPS:434357 | 21-225_65C1 | NA | GACATCCAGTTGACCCAGTCTCCATCCTCCCT GTCTGCATCTGTAGGAGACAGAGTCACCATCA CTTGCCGGGCGAGTCAGGTCATTAGCAGTTAT TTACATTGGTATCAGCAGAAACCAGGGAAAGT TCCTAAGGTCCTGATCTATAGTGCATCCAATTT GCAATGTGGAGTCCCATCTCGGTTCAGCGGCA GTGGATCTGGGACAGATTTCACTCTCACCTTC AGCAGCCTGCAGCCTGAAGATGTTGCAACTTA TTACGGTCAACGGCCTTACAATGCCCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTGATATGGTTTGAGGGAAG TAATAAACACTATACAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGAACTTG GGTTCAGCAGTGACTACTGGGGCCAGGGAACCC TGGTCACCGTCTCCTCA |
| | | | SEQ ID NO: 25621 | SEQ ID NO: 29627 |
| | | AA | DIQLTQSPSSLSASVGDRVTITCRASQVISSYLHW YQQKPGKVPKVLIYSASNLQCGVPSRFSGSGSGT DFTLTFSSLQPEDVATYYGQRPYNAPLTFGGGT KVEIK | QVQLVESGGGVVQPGRSLRLSCAASGFTFSDYGM HWVRQAPGKGLEWVAVIWFEGSNKHYTDSVKGR FTISRDNSKNTLYLQMNSLRAEDTAVYYCARELGF SSDYWGQGTLVTVSS |
| | | | SEQ ID NO: 25622 | SEQ ID NO: 29628 |

FIGURE 50

| iPS:434359 | 21-225_65G3 | NA | GACATCCAGATGACCCAGTCTCCATCCTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGCTCTTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT TCTATTGTCAACAGGTTAACAGTTTCCCTCTCA CTTTCGGCGGAGGGACCAAGGTGGAGATCAA A | CAGGTGCAACTGGTGCAGTCTGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTAACAGTGG TGGCACAAACTCTGCACAGAAGTTTCAGGGCAG GGTCACCATGACCAGGGACACGTCCATCAGCAC AGCCTACATGGAGCTGAGCAGGCTGAGATCTGA CGACACGGCCGTATATTACTGTGCGAGAGCTCCG GGTAAAGCAGCAGCTGGTACATGGGGATACTTTG ACTACTGGGGCCAGGGAACCCTGGTCACCGTCTC CTCA |
| | | | SEQ ID NO: 25623 | SEQ ID NO: 29629 |
| | | AA | DIQMTQSPSSVSASVGDRVTITCRASQGISSWLA WYQQKPGKAPKLLIYAASSLQSGVPSRFSGSGS GTDFTLTISSLQPEDFATFYCQQVNSFPLTFGGGT KVEIK | QVQLVQSGAEVKKPGASVKVSCKASGYTFTGYYIH WVRQAPGQGLEWMGWINPNSGGTNSAQKFQGRV TMTRDTSISTAYMELSRLRSDDTAVYYCARAPGKA AGTWGYFDYWGQGTLVTVSS |
| | | | SEQ ID NO: 25624 | SEQ ID NO: 29630 |
| iPS:434361 | 21-225_65D5 | NA | GACATCCAGATGACCCAGTCTCCGTCCTCACT ATCTGCATCTGTAGGAGACAGAGTCACCATCA CTTGTCGGGCGAGTCAGGACATTAACAATTAT TTAGCCTGGTTTCAGCAGAAACCAGGGAAAGC CCCTAGGTCCCTAATTTATGCTGCATCCAGTTT GCACAGTGGGGTCCCATCACAGTTCAGCGCCA GTGGTTCTGGGTCAGATTTCACTCTTACTATCA GCAGCCTGCAGCCTGAAGATTTTGCAACTTAT TACTGCCCACTGTATAAAAGTTATCCACTCAC TTTTGGCCCTGGGACCAAAGTGGATATCAAA | CAGGTTCAGCTGGTGCAGTCTGGAGCTGAGGTGA AGAAGCCTGGGGCCTCAGTGAAGGTCTCCTGCA AGGCTTCTGGTTACACCTTTCCCAGCTATGGTAT CAGTTGGGTGCGACAGGCCCCTGGACAAGGACT TGAGTGGATGGGATGGATCAGCGCTTACAGTGGT AACACAAACTATGCACAGAAGCTCCAGGGCAGA GTCACCATGACCACAGACACATCCACGAGCACA GCCTACATGGAGCTGAGGAGCCTGAGATCTGAC GACACGGCCGTGTATTTCTGTGCGAGAGGGGAA GCAGTGGCTGTCTTCGACCCCTGGGGCCAGGGAA CCCTGGTCACCGTCTCCTCA |
| | | | SEQ ID NO: 25625 | SEQ ID NO: 29631 |

FIGURE 50

| | | | | |
|---|---|---|---|---|
| | | AA | DIQMTQSPSSLSASVGDRVTITCRASQDINNYLA WFQQKPGKAPRSLIYAASSLHSGVPSQFSASGSG SDFTLTISSLQPEDFATYYCPLYKSYPLTFGPGTK VDIK | QVQLVQSGAEVKKPGASVKVSCKASGYTFPSYGIS WVRQAPGQGLEWMGWISAYSGNTNYAQKLQGRV TMTTDTSTSTAYMELRSLRSDDTAVYFCARGEAVA VFDPWGQGTLVTVSS |
| | | | SEQ ID NO: 25626 | SEQ ID NO: 29632 |
| iPS:434363 | 21-225_65A6 | NA | GAAATAGTGATGACGCAGTCTCCAGTCACCCT GTTTGTGTCTCCAGGGGAAAGAGCCACCCTCT CCTGCAGGGCCAGTCAGAGTGTTAACAGCAAC TTAGCCTGGTACCAGCAGAAACCTGGCCAGGC TCCCAGGCTCCTCATCTATGGTGCATCCACCA GGGCCACTGGTATCCCAGCCAGGTTCAATGGC AGTGGGTCTGGGACAGAGTTCACTCTCACCAT CAGCAGCCTGCAGTCTGAAGATTTTGCAGTTT ATTATTGTCAGCAGTATAATGACTGGCCGTGC AGTTTTGGCCTGGAGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGGAAGT AATAAATACTATGGAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CATAGTGGGAGCTACTTGGTTTGACTACTGGGGC CAGGGAACCCTGGTCACCGTCTCCTCA |
| | | | SEQ ID NO: 25627 | SEQ ID NO: 29633 |
| | | AA | EIVMTQSPVTLFVSPGERATLSCRASQSVNSNLA WYQQKPGQAPRLLIYGASTRATGIPARFNGSGS GTEFTLTISSLQSEDFAVYYCQQYNDWPCSFGLE TKLEIK | QVQLVESGGGVVQPGRSLRLSCAASGFTFSNYGM HWVRQAPGKGLEWVAVIWYDGSNKYYGDSVKGR FTISRDNSKNTLYLQMNSLRAEDTAVYYCARDQGI VGATWFDYWGQGTLVTVSS |
| | | | SEQ ID NO: 25628 | SEQ ID NO: 29634 |

FIGURE 50

| iPS:434367 | 21-225_65H11 | NA | GACATCCAGATGACCCAGTCTCCATCCTCTCT GTCTGCATCTGTAGGAGACAGAGTCACCATCA CTTGTCGGGCGAGTCAGGACATTAGCACTTAT TTAGCCTGGTTTCAGCAGAAACCAGGGAAAGC CCCTAAGTCACTGATCTATGCTGCATCCAGTTT GCAAAGTGGGGTCCCATCAAAGTTCAGCGGCA GTGGATCTGGGACAGATTTCACTCTCACCATC AGTAGCCTGCAGCCTGAAGATTTTGCAACTTA TTACTGCCAACAGTATAATAGTTTCCCTCTCAC TTTCGGCGGAGGGACCAAGGTGGAGATCAAA | GAGGTGCAGCTGGTGGAGTCTGGGGGAGGCCTG GTCAAGCCGGGGGGGTCCCTGAGACTCTCCTGTG CAGCCTCTGGATTCACCTTCAGTAGCTATAGGAT GAACTGGGTCCGCCAGGCTCCAGGGAAGGGGCT GGAGTGGGTCTCATCCATTAGTAGTAGTAATAGT TCCATATACTACGCAGACTCAGTGAAGGGCCGAT TCACCACCTCCAGAGACAACGCCAAGAACTCACT GTATCTGCAAATGAACAGCCTGAGAGCCGAGGA CACGGCTGTGTATTACTGTACGAGTACAAGTGGG AGCTGGGGCCAGGGAACCCTGGTCACCGTCTCCT CA |
| | | | SEQ ID NO: 25629 | SEQ ID NO: 29635 |
| | | AA | DIQMTQSPSSLSASVGDRVTITCRASQDISTYLA WFQQKPGKAPKSLIYAASSLQSGVPSKFSGSGSG TDFTLTISSLQPEDFATYYCQQYNSFPLTFGGGT KVEIK | EVQLVESGGGLVKPGGSLRLSCAASGFTFSSYRMN WVRQAPGKGLEWVSSISSSNSSIYYADSVKGRFTTS RDNAKNSLYLQMNSLRAEDTAVYYCTSTSGSWGQ GTLVTVSS |
| | | | SEQ ID NO: 25630 | SEQ ID NO: 29636 |
| iPS:434369 | 21-225_66B1 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCTTAAGCTCTTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGACTAACAGTTTCCCTCTC ACTTTCGGCGGAGGGACCAAGGTGGAGATCA AA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACAATGCACAGAAGTTTCAGGGCAG GGTCACCATGACCAGGGCCACGTCCATCAGCAC AGCCTACATGGAACTGAGCAGGCTGAGATCTGA CGACACGGCCGTGTATTATTGTGCGAGAGCTCCG GGTACAGCAGCAGCTGGTACATGGGGATACTTTG ACTACTGGGGCCAGGGAACCCTGGTCACCGTCTC CTCA |
| | | | SEQ ID NO: 25631 | SEQ ID NO: 29637 |

FIGURE 50

| | | | | |
|---|---|---|---|---|
| | | AA | DIQMTQSPSSVSASVGDRVTITCRASQGISSWLA WYQQKPGKALKLLIYAASSLQSGVPSRFSGSGS GTDFTLTISSLQPEDFATYYCQQTNSFPLTFGGGT KVEIK | QVQLVQSGAEVKKPGASVKVSCKASGYTFTGYYM HWVRQAPGQGLEWMGWINPNSGGTNNAQKFQGR VTMTRATSISTAYMELSRLRSDDTAVYYCARAPGT AAAGTWGYFDYWGQGTLVTVSS |
| | | | SEQ ID NO: 25632 | SEQ ID NO: 29638 |
| iPS:434373 | 21-225_66A7 | NA | GACATCCAGATGACCCAGTCTCCATCTTCCGT GTCTGCATCTGTAGGGGACAGAGTCACCATCA CTTGTCGGGCGAGTCAGGGTATTAGCAGCTGG TTAGCCTGGTATCAGCAGAAACCAGGGAAAG CCCCTAAGCTCTTGATCTATGCTGCATCCAGTT TGCAAAGTGGGGTCCCTTCAAGGTTCAGCGGC AGTGGATCTGGGACAGATTTCACTCTCACCAT CAGCAGCCTGCAGCCTGAAGATTTTGCAACTT ACTATTGTCAACAGATTAATAGTTTCCCTCTCA CTTTCGGCGGAGGGACCAAGGTGGAGATCAA A | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAAACCAAACAGTG GTGGCACAAACCAAGCACAGAAGTTCCAGGGCA GGGTCACCATGACCAGGGACACGTCCATCAGCA CAGGTTACATGGAGCTGAGCAGGCTGAGATCTG ACGACACGGCCGTGTATTACTGTGCGAGAGCTCC GGGCACAGTAGCAGCTGGTACATGGGGATACTTT GACTATTGGGGCCAGGGAACCCTGGTCACCGTCT CCTCA |
| | | | SEQ ID NO: 25633 | SEQ ID NO: 29639 |
| | | AA | DIQMTQSPSSVSASVGDRVTITCRASQGISSWLA WYQQKPGKAPKLLIYAASSLQSGVPSRFSGSGS GTDFTLTISSLQPEDFATYYCQQINSFPLTFGGGT KVEIK | QVQLVQSGAEVKKPGASVKVSCKASGYTFTGYYM HWVRQAPGQGLEWMGWIKPNSGGTNQAQKFQGR VTMTRDTSISTGYMELSRLRSDDTAVYYCARAPGT VAAGTWGYFDYWGQGTLVTVSS |
| | | | SEQ ID NO: 25634 | SEQ ID NO: 29640 |

FIGURE 50

| iPS:434375 | 21-225_66C7 | NA | GACATCCAGTTGACCCAGTCTCCATCCTCCCT GTCTGCATCTGTAGGAGACAGAGTCACCATCA CTCGCCGGGCGAGTCAGGGCATTAGCAATTAT TTACATTGGTATCAGCAGAAACCAGGGAAAGC TCCTAAGCTCTTGATCTATTGTGCATCCAATTT ACAATGTGGAGTCCCATCACGGTTCAGCGGCA GTGGATCTGGGACAGATTTCACTCTCACAATC AGCAGCCTGCAGCCTGAAGATGTTGCAACTTA TTACTGTCAACAGCATAATAATTCCCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG TAGCGTCTGGATTCACCTTCAGTGACTATGGCAT GCACTGGGTCCGCCAGGCTCCAGGCAAGGGGCT GGAGTGGGTGGCAGTTATATGGTTTGAGGGAAGT CATAAATACTATACAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTTGGG TTCAGCAGTGACTACTGGGGCCAGGGAACCCTG GTCACCGTCTCCTCA |
|---|---|---|---|---|
| | | | SEQ ID NO: 25635 | SEQ ID NO: 29641 |
| | | AA | DIQLTQSPSSLSASVGDRVTITRRASQGISNYLH WYQQKPGKAPKLLIYCASNLQCGVPSRFSGSGS GTDFTLTISSLQPEDVATYYCQQHNNSPLTFGGG TKVEIK | QVQLVESGGGVVQPGRSLRLSCVASGFTFSDYGM HWVRQAPGKGLEWVAVIWFEGSHKYYTDSVKGR FTISRDNSKNTLYLQMNSLRAEDTAVYYCARELGF SSDYWGQGTLVTVSS |
| | | | SEQ ID NO: 25636 | SEQ ID NO: 29642 |
| iPS:434379 | 21-225_66A9 | NA | GACATCCAGATGACCCAGTCTCCATCCTCCCT GTCTGCATCTGTAGGAGACAGAGTCACCATCA CTTGCCGGGCAAGTCAGAACATTTTCAGCTAT TTAAATTGGTATCAGCAGAAACCAGGGAAAG CCCCTAAACTCCTGATCTTTGCTGCATCCAGTT TACAAAGTGGGGTCCCATCAAGGTTCAGTGGC AGTGGATCTGGGACAGATTTCACTCTCACCAT CAGCAGTCTGCAACCTGAGGACTTTGCAACTT ACTACTGTCAACAGACTTACAGTGTCCCTTTC ACTTTCGGCCCTGGGACTAAGGTGGATTTCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGCATGATGGAAG TGATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGAAGAC CCGCGTACCAGTTGTTCTGACTACTGGGGCCAGG GAACCCTGGTCACCGTCTCCTCA |
| | | | SEQ ID NO: 25637 | SEQ ID NO: 29643 |

1906

FIGURE 50

| | | | | |
|---|---|---|---|---|
| | | AA | DIQMTQSPSSLSASVGDRVTITCRASQNIFSYLN WYQQKPGKAPKLLIFAASSLQSGVPSRFSGSGSG TDFTLTISSLQPEDFATYYCQQTYSVPFTFGPGTK VDFK | QVQLVESGGGVVQPGRSLRLSCAASGFTFSSYGMH WVRQAPGKGLEWVAVIWHDGSDKYYADSVKGRF TISRDNSKNTLYLQMNSLRAEDTAVYYCAREDPRT SCSDYWGQGTLVTVSS |
| | | | SEQ ID NO: 25638 | SEQ ID NO: 29644 |
| IPS:434383 | 21-225_66F9 | NA | GACATCCAGATGACCCAGTCTCCATCCTCCCT GTCTGCATCTGTAGGAGACAGAGTCACCATCA CTTGCCGGGCAAGTCAGGACATTAGAAATGTT TTAGGCTGGTATCAGCAGAAACCAGGGAAAG CCCCTAAGCGCCTGATCTATACTGCATCCAGT TTACAAAGTGGGGTCCCATCAGGGTTCAGCGG CAGTGGATCTGGGACAGAATTCACTCTCACAA TCAGCAGCCTGCAGCCTGAAGATTTTGCAACT TATTACTGTCTACAGTATAATAGTTACCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGACT GGAGTGGGTCTCATCCATTAGTGGTACTAGTAGT TACATATACTACGCAGACTCAGTGAAGGGCCGAT TCACCATCTCCAGAGACAACGCCAAGAACTCACT GTATCTGCAAATGAACGGCCTGAGAGCCGAGGA CACGGCTGTGTATTTCTGTGCGAGAACCAATGCT TTTGATATCTGGGGCCAGGGGACAATGGTCACCG TCTCTTCA |
| | | | SEQ ID NO: 25639 | SEQ ID NO: 29645 |
| | | AA | DIQMTQSPSSLSASVGDRVTITCRASQDIRNVLG WYQQKPGKAPKRLIYTASSLQSGVPSGFSGSGS GTEFTLTISSLQPEDFATYYCLQYNSYPFTFGPGT KVDIK | EVQLVESGGGLVKPGGSLRLSCAASGFTFSSYSMN WVRQAPGKGLEWVSSISGTSSYIYYADSVKGRFTIS RDNAKNSLYLQMNGLRAEDTAVYFCARTNAFDIW GQGTMVTVSS |
| | | | SEQ ID NO: 25640 | SEQ ID NO: 29646 |

FIGURE 50

| iPS:434385 | 21-225_66C10 | NA | GACATCCAGATGACCCAGTCTCCATCCTCCCT GTCTGCATCTGTAGGAGACAGAGTCACCATCA CTTGCCGGGCAAGTCAGGCCATTAGAAATGAT TTAGGCTGGTATCAGCAGAAACCAGGGAAAG CCCCTAAGCGCCTGATCTATCCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGTATAATAGTTACCCTCCGT GGACGTTCGGCCAAGGGTCCAAGGTGGAAAT CAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGTTAT GAGCTGGGTCCGCCAGGCTCCAGGGAAGGGGCT GGAGTGGGTCTCAGGTATTAGTGGTAGTGGTGCT AGAACATACTACGCAGACTCCGTGAAGGGCCGA TTCACCATCTCCAGAGACAATTCCAAGAACACGC TGTATCTGCAAATGAACAGCCTGAGAGCCGAGG ACACGGCCGTATATTACTGTGCGGAACTGGGGAT AGACTACTACTACGGTATGGACGTCTGGGGCCAA GGGACCACGGTCACCGTCTCCTCA |
| | | | SEQ ID NO: 25641 | SEQ ID NO: 29647 |
| | | AA | DIQMTQSPSSLSASVGDRVTITCRASQAIRNDLG WYQQKPGKAPKRLIYPASSLQSGVPSRFSGSGSG TEFTLTISSLQPEDFATYYCLQYNSYPPWTFGQG SKVEIK | EVQLLESGGGLVQPGGSLRLSCAASGFTFSSYVMS WVRQAPGKGLEWVSGISGSGARTYYADSVKGRFTI SRDNSKNTLYLQMNSLRAEDTAVYYCAELGIDYY YGMDVWGQGTTVTVSS |
| | | | SEQ ID NO: 25642 | SEQ ID NO: 29648 |
| iPS:434387 | 21-225_66D11 | NA | GATATCCAGATGACCCAGTTTCCATCCTCCCA GTCTGCATCTGTAGGAGACAGAGTCACCATCA CTTGCCGGGCAAGTCAGGGCATTAGAAATGAT TTAGGTTGGTATCAGCAGAAACCAGGGAAAG CCCATAAGCGCTTGATCTATGCTGCATCCAGT TGTCAAAGTGGGGTCCCATCAAGGTTCAGCGG CAGTGGATGTGGGACAGAATTCACTATCTCAA TCAGCAGCATGCAGCGTGAAGATTTTGCAACT TATTACTGTATAGTGCATAATAGTTACCCTCG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG GAGCGTCTGGATTCACCTTCAGTAGTTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTCTCTGCAAATGAACAGCCTGAGAGCCGAGG ACACGGCTCTGTATTACTGTGCGAGAGAGATGTA TAGCAGCAACTGGTACGACTACGGTTTGGACGTC TGGGGCCAAGGGACCACGGTCACCGTCTCCTCA |
| | | | SEQ ID NO: 25643 | SEQ ID NO: 29649 |

FIGURE 50

| | | | | |
|---|---|---|---|---|
| | | AA | DIQMTQFPSSQSASVGDRVTITCRASQGIRNDLG WYQQKPGKAHKRLIYAASSCQSGVPSRFSGSGC GTEFTISISSMQREDFATYYCIVHNSYPRTFGQGT KVEIK | QVQLVESGGGVVQPGRSLRLSCGASGFTFSSYGMH WVRQAPGKGLEWVAVIWYDGSNKYYADSVKGRF TISRDNSKNTLSLQMNSLRAEDTALYYCAREMYSS NWYDYGLDVWGQGTTVTVSS |
| | | | SEQ ID NO: 25644 | SEQ ID NO: 29650 |
| IPS:434389 | 21-225_66F11 | NA | GACATCCAGATGACCCAGTCTCCATCTTCCGT GTGTGCATCTGTAGGAGACAGAGTCACCATCA CTTGTCGGGAGAGTCAGGGTATTAGCATCTGG TTAGCCTGGTATCAGCAGAAACCAGGGAAAG CCCCTAAGCTTTTGATCTATGCTGCATCCAGTT TGCAAAGTGGGGTCCCATCAAGGTTCAGCGGC AGTGGATATGGGACAGATTTCACTCTCACCAT CAGCAGCGTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACCATA TGCACTGGGTCCGACAGGCCCCTGGACAAGGGC TTGAGTGGATGGGATGGATCAACCCTAACAATGG TGGCACACACTATGCACAGAAGTTTCAGGACTGG GTCACCATGACCAGGGACACGTCCATCAGCACA GCCTATATGGAACTGAGCAGGCTGAGATCTGAC GACACGGCCGTGTATTACTGTGCGAGAGATAGTA GAAGTTCGTGGGACTACTGGGGCCAGGGAACCC TGGTCTCCGTCTCCTCA |
| | | | SEQ ID NO: 25645 | SEQ ID NO: 29651 |
| | | AA | DIQMTQSPSSVCASVGDRVTITCRESQGISIWLA WYQQKPGKAPKLLIYAASSLQSGVPSRFSGSGY GTDFTLTISSVQPEDFATYYCQQANSFPFTFGPGT KVDIK | QVQLVQSGAEVKKPGASVKVSCKASGYTFTGYHM HWVRQAPGQGLEWMGWINPNNGGTHYAQKFQD WVTMTRDTSISTAYMELSRLRSDDTAVYYCARDS RSSWDYWGQGTLVSVSS |
| | | | SEQ ID NO: 25646 | SEQ ID NO: 29652 |

FIGURE 50

| | | | | |
|---|---|---|---|---|
| iPS:434393 | 21-225_67C3 | NA | GAAATAGTGATGACGCAGTCTCCAGCCACCCT GTCTGTGTCTCCAGGGGAAAGAGCCACCCTCT CCTGCAGGGCCAGTCAGAGTGTTAACAGCAAC TTAGCCTGGTACCAGCAGAAACCTGGCCAGGC TCCCAGGCTCCTCATCTCTATTGCATCCACCAG GGCCACTGGTATCCCAGCCAGGTTCAATGGCA GTGGGTCTGGGACAGAGTTCACTCTCACCATC AGCAGCCTGCAGTCTGAAGATTTTGCAGTTTA TTACTGTCAGCAGTATAATGACTGGCCGTGTA GTTTTGGCCAGGGGACCAAGCTGGAGATCAAA<br><br>SEQ ID NO: 25647 | CAGGTGCAGCTGGTGGAGTCGGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCAGCTATATGGTATGATGGAAG TAATAAATATTATGGAGACTCCGTGAAGGGCCGA GTCACCATCTCCAGAGACAATTCCAAGAACTCGC TGCATCTGCAAATGAACAGCCTGAGAGCCGAGG ACACGGCTGTGTATTACTGTGCGAGAGATCAGGG CATAGTGGGAGCTACTTGGTTTGACTACTGGGGC CAGGGAACCCTGGTCACCGTCTCCTCA<br><br>SEQ ID NO: 29653 |
| | | AA | EIVMTQSPATLSVSPGERATLSCRASQSVNSNLA WYQQKPGQAPRLLISIASTRATGIPARFNGSGSG TEFTLTISSLQSEDFAVYYCQQYNDWPCSFGQGT KLEIK<br><br>SEQ ID NO: 25648 | QVQLVESGGGVVQPGRSLRLSCAASGFTFSSYGMH WVRQAPGKGLEWVAAIWYDGSNKYYGDSVKGRV TISRDNSKNSLHLQMNSLRAEDTAVYYCARDQGIV GATWFDYWGQGTLVTVSS<br><br>SEQ ID NO: 29654 |
| iPS:434397 | 21-225_67H4 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGCTCTTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGATTAACAGTTTCCCTCTCA CTTTCGGCGGAGGGACCAAGGTGGAGATCAA A<br><br>SEQ ID NO: 25649 | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAAACCAAACAGTG GTGGCACAAACCAAGCACAGAAGTTTCAGGGCA GGGTCACCATGACCAGGGACACGTCCATCAGCA CAGCCTACATGGAGCTGAGCGGGCTGAGATCTG ACGACACGGCCGTGTATTACTGTGCGAGAGCTCC GGGTACTGCAGCAACTGGTACATGGGGATACTTT GACTACTGGGGCCAGGGAACCCTGGTCACCGTCT CCTCA<br><br>SEQ ID NO: 29655 |

FIGURE 50

| | | | | |
|---|---|---|---|---|
| | | AA | DIQMTQSPSSVSASVGDRVTITCRASQGISSWLA WYQQKPGKAPKLLIYAASSLQSGVPSRFSGSGS GTDFTLTISSLQPEDFATYYCQQINSFPLTFGGGT KVEIK | QVQLVQSGAEVKKPGASVKVSCKASGYTFTGYYM HWVRQAPGQGLEWMGWIKPNSGGTNQAQKFQGR VTMTRDTSISTAYMELSGLRSDDTAVYYCARAPGT AATGTWGYFDYWGQGTLVTVSS |
| | | | SEQ ID NO: 25650 | SEQ ID NO: 29656 |
| IPS:434399 | 21-225_67B7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTTTCAGCAGAAACCAGGGAAAGC CCCTAAGCGCCTGATCTATGCTGCATCCAGTTT GCAAAGTGGGGTCCCATCAAGGTTCAGCGGCA GTGGATCTGGGACAGAATTCTCTCTCACAATC AGCAGCCTGCAGCCTGAAGATTTTGCAACTTA TTACTGTCTACACCATAATAGTTATCCATTCAA ATTTGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAACTATGGCAT GCACTGGGTCCGCCAGGCTCCAGGCAAGGGGCT GGAATGGGTGGCAGTTATATTATATGATGGAAGT AAGAAATACTATGCAGACTCCGTGAAGGGCCGA TTCACCATCTCCAGAGACAATTCCAAGAACACGC TTTATCTGCAAATGAACAGCCTGAGAGCTGAGGA CACGGCTGTGTATTACTGTGCGAGGAGTATCCCG GAATTTGACTATTGGGGCCAGGGAACCCTGGTCA CCGTCTCCTCA |
| | | | SEQ ID NO: 25651 | SEQ ID NO: 29657 |
| | | AA | DIQMTQSPSSLSASVGDRVTITCRASQGIRNDLG WFQQKPGKAPKRLIYAASSLQSGVPSRFSGSGSG TEFSLTISSLQPEDFATYYCLHHNSYPFKFGPGTK VDIK | QVQLVESGGGVVQPGRSLRLSCAASGFTFSNYGM HWVRQAPGKGLEWVAVILYDGSKKYYADSVKGR FTISRDNSKNTLYLQMNSLRAEDTAVYYCARSIPEF DYWGQGTLVTVSS |
| | | | SEQ ID NO: 25652 | SEQ ID NO: 29658 |

FIGURE 50

| iPS:434405 | 21-225_68E6 | NA | GACATCCAGATGACCCAGTCTCCATCCTCACT GTCTGCATCTGTAGGAGACAGAGTCACCATCA CTTGTCGGGCGAGTCAGGGCATTAGCTATTAT TTAGCCTGGTTTCAGCAGAAACCAGGGAGAGC CCCTAAGTCCCTGATCTATGTTGCATCCAGTTT GCAAAGTGGGGTCCCATCAAGGTTCAGCGGCA GTGGATCTGGGACAGATTTCACTCTCACCATC AGCAGCCTGCAGCCTGAAGATTTTGCAATTTA TTACTGCCAACAGTATGATAGTTACCCATTCA CTTTCGGCCCTGGGACCAAAGTGGATATCAGA | GAGGTGCAGCTGGTGGAGTCTGGGGGAGGCCTG GTCAAGCCGGGGGGGTCCCTGAGACTCTCCTGTG CAGCCTCTGGATTCACCTTAAGTAGCTTTGGCAT GAACTGGGTCCGCCAGGCTCCAGGGAAGGGGCT GGAGTGGGTCTCATACATTAGTAGAAGTAGTAGT CACATATACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGGTCTCTAGTGG GAGCCCCTTTGACTACTGGGGCCAGGGAACCCTG GTCACCGTCTCCTCA |
| | | | SEQ ID NO: 25653 | SEQ ID NO: 29659 |
| | | AA | DIQMTQSPSSLSASVGDRVTITCRASQGISYYLA WFQQKPGRAPKSLIYVASSLQSGVPSRFSGSGSG TDFTLTISSLQPEDFAIYYCQQYDSYPFTFGPGTK VDIR | EVQLVESGGGLVKPGGSLRLSCAASGFTLSSFGMN WVRQAPGKGLEWVSYISRSSSHIYYADSVKGRFTIS RDNAKNSLYLQMNSLRAEDTAVYYCAVSSGSPFD YWGQGTLVTVSS |
| | | | SEQ ID NO: 25654 | SEQ ID NO: 29660 |
| iPS:434407 | 21-225_68G8 | NA | GACATCCAGATGACCCAGTCTCCATCCTCCCT GTCTGCATCTGTAGGAGACAGAGTCACCATCA CTTGCCGGGCAAGTCAGGGCATTAGAAATAAT TTAGGCTGGTATCAGCAGAGACCAGGGAAAG CCCCTAAGCGCCTGATCTATGCTGCATCCAGT GTGCAAAGTGGGGTCCCATCACGGTTCAGCGG CAGTGGATCTGGGACAGAATTCACTCTCACAA TCAGCAGCCTGCAGCCTGAAGATTTTGCAACT TATTACTGTCTACAGTATAATAGTTACCCATTC ACTTTCGGCCCTGGGACCAAAGTGGATATCAA A | GAGGTGCAGTTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATATTACGCAGACTCAGTGATGGGCCGAT TCACCATCTCCAGAGACAACGCCAAGAACTCTCT GTATCTGCAAATGAACAGCCTGAGAGCCGAGGA CACGGCTGTGTATTACTGTGCGAGAGTCAACAGC TTTGACTCCTGGGGCCAGGGAACCCTGGTCACCG TCTCCTCA |
| | | | SEQ ID NO: 25655 | SEQ ID NO: 29661 |

FIGURE 50

| | | | | |
|---|---|---|---|---|
| | | AA | DIQMTQSPSSLSASVGDRVTITCRASQGIRNNLG WYQQRPGKAPKRLIYAASSVQSGVPSRFSGSGS GTEFTLTISSLQPEDFATYYCLQYNSYPFTFGPGT KVDIK | EVQLVESGGGLVKPGGSLRLSCAASGFTFSSYSMN WVRQAPGKGLEWVSSISGSSSYIYYADSVMGRFTIS RDNAKNSLYLQMNSLRAEDTAVYYCARVNSFDSW GQGTLVTVSS |
| | | | SEQ ID NO: 25656 | SEQ ID NO: 29662 |
| IPS:434411 | 21-225_68F11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGCCAGAATTCACTCTCTCAA TCAGCAGCCTGCAGCCTGAAGATTTTGCAACT TATTACTGTCTACAGCATAATAGTTATCCGTTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCTGTTATATGGTATGATGTAAGT AATAAATACTATGCAGACTCCGTGAAGGGCCGA TTCACCATCTCCAGAGACAATTCCAAGAACACGC TGTATCTTCAAATGAACAGCCTGAGAGCCGAGG ACACGGCTGTGTATTACTGTGTGAGAGAACTGGG GATGACCTCTGACTGCTGGGGCCAGGGAACCCTG GTCACCGTCTCCTCA |
| | | | SEQ ID NO: 25657 | SEQ ID NO: 29663 |
| | | AA | DIQMTQSPSSLSASVGDRVTITCRASQGIRNDLG WYQQKPGKAPKRLIYAASSLQSGVPSRFSGSGS GPEFTLSISSLQPEDFATYYCLQHNSYPFTFGGGT KVEIK | QVQLVESGGGVVQPGRSLRLSCAASGFTFSDYGM HWVRQAPGKGLEWVAVIWYDVSNKYYADSVKGR FTISRDNSKNTLYLQMNSLRAEDTAVYYCVRELGM TSDCWGQGTLVTVSS |
| | | | SEQ ID NO: 25658 | SEQ ID NO: 29664 |

FIGURE 50

| iPS:434413 | 21-225_68D12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGATTCAGCGG CAGTGGATCTGGGACAGAATTCACTCTCACAA TCAGCAGCCTGCAGCCTGAAGATTTTGCAACT TATTACTGTCTACAGCATAGTACTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAATATCTATTATAGT GGGAGCACCTATTACATCCCGTCCCTCAAGAGTC GAGTCACCATATCCGTAGACACGTCCAAGAACC AGTTCTCCCTGAAGCTGACCTCTGTGACCGCCGC AGACACGGCTGTGTATTACTGTGCGAGACATAGC ACCAGCTGGTCCATTGACTACTGGGGCCAGGGA ACCCTGGTCACCGTCTCCTCA |
|---|---|---|---|---|
| | | | SEQ ID NO: 25659 | SEQ ID NO: 29665 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHSTYPLTFGGGT KVEIK | QLQLQESGPGLVKPSETLSLTCTVSGGSISRSSYYW GWIRQPPGKGLEWIGNIYYSGSTYYIPSLKSRVTISV DTSKNQFSLKLTSVTAADTAVYYCARHSTSWSIDY WGQGTLVTVSS |
| | | | SEQ ID NO: 25660 | SEQ ID NO: 29666 |
| iPS:434417 | 21-225_69C8 | NA | GACATCCAGATGACCCAGTCTCCATCCTCCCT GTCTGCATCTGTAGGAGACAGAGTCACCTTCA CTTGCCGGGCAGGTCAGACCATTTACAATTAT TTAAATTGGTATCAGCAGAAACCAGGGAAAG CCCCTAAGCTCCTGATCCATGTTGCGTCCAGTT TGCAAAGTGGGGTCCCATCAAGGTTCAGTGGC AGTGGATCTGGGACAGATTTCACTCTCGTCAT TAGTAGTCTGCAACCTGAAGATTTTGCAACTT ACTACTGTCAACAGAGTTACAGTACCCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGCCAT GCACTGGGTCCGCCAGGCTCCAGGCAAGGGGCT GGAGTGGGTGGCAGTTATATGGTATGATGGAAGT GATAAATACTATGCAGACTCCGTGAAGGGCCGA TTCACCATCTCCAGAGACAATTCCAAGAACACGC TGTATCTGCAAATGATCAGCCTGAGAGCCGAGG ACACGGCTGTTTATTACTGTGCGAGAGATATCCC TAGCAACTCGGCGGGGGACTACTGGGGCCAGGG AACCCTGGTCACCGTCTCCTCA |
| | | | SEQ ID NO: 25661 | SEQ ID NO: 29667 |

FIGURE 50

| | | | | |
|---|---|---|---|---|
| | | AA | DIQMTQSPSSLSASVGDRVTFTCRAGQTIYNYLN WYQQKPGKAPKLLIHVASSLQSGVPSRFSGSGS GTDFTLVISSLQPEDFATYYCQQSYSTPFTFGPGT KVDIK | QVQLVESGGGVVQPGRSLRLSCAASGFTFSNYAM HWVRQAPGKGLEWVAVIWYDGSDKYYADSVKGR FTISRDNSKNTLYLQMISLRAEDTAVYYCARDIPSN SAGDYWGQGTLVTVSS |
| | | | SEQ ID NO: 25662 | SEQ ID NO: 29668 |
| IPS:434423 | 21-225_70D1 | NA | GACATCCAGATGACCCAGTCTCCATCTTCCGT GTCTGCATCTGTAGGAGACAGAGTCACCATCA CTTGTCGGGCGAGTCAGGGTGTTAGCAGGTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AGTGGATCTGGGACAGATTTCACTGTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGTA AGGCTTCTGGATACACCTTCACCGGCTACCATAT GCACTGGGTGCGACAGGCCCCTGGACAAGGGCT TGAGTGGATGGGATGGATCAACCCTAACAGTAAT GCCACAAACTATGCACAGAAGTTTCAGGGCAGG GTCACCATGACCAGGGACACGTCCATCAGCACA GCCTACATGGAACTGAGCAGGCTGAGATCTGAC GACACGGCCGTGTATTACTGTGCGAGAGATAGC ATATCGTCGTGGGACTACTGGGGCCAGGGAACC CTGGTCACCGTCTCCTCA |
| | | | SEQ ID NO: 25663 | SEQ ID NO: 29669 |
| | | AA | DIQMTQSPSSVSASVGDRVTITCRASQGVSRWL AWYQQKPGKAPKLLIYAASSLQSGVPSRFSGSG SGTDFTVTISSLQPEDFATYYCQQANSFPFTFGPG TKVDIK | QVQLVQSGAEVKKPGASVKVSCKASGYTFTGYHM HWVRQAPGQGLEWMGWINPNSNATNYAQKFQGR VTMTRDTSISTAYMELSRLRSDDTAVYYCARDSISS WDYWGQGTLVTVSS |
| | | | SEQ ID NO: 25664 | SEQ ID NO: 29670 |

FIGURE 50

| iPS:434425 | 21-225_70A5 | NA | GAAATAGTGATGACGCAGTCTCCAGCCACCCTGTCTGTGTCTCCAGGGGAAAGAGCCACCCTCTCCTGCAGGGCCAGTCAGAGTGTTAACAGCAACTTAGCCTGGTACCAGCTGAAACCTGGCCAGGCTCCCAGGCTCCTCATCTCTATTGCATCCACCAGGGCCACTGGTATCCCACCCCGGTTCAATGGCAGTGGGTCTGGGACAGAGTTCACTCTCACCATCAGCAGCCTGCAGTCTGAAGATTTTGCAGTTTATTACTGTCAGCAGTATAATGACTGGCCGTGTAGTTTTGGCCAGGGGACCAAGCTGGAGATCAAA | CAGGTTCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACTCGCTGTATCTGCAAATGAACAGCCTGAGCGCCGAGGACACGGCTGTTTATTACTGTGCGAGAGATCAGGGCATAGTGGGAGCTACTTGGTTTGACTACTGGGGCCAGGGAACCCTGGTCACCGTCTCCTCA |
| | | | SEQ ID NO: 25665 | SEQ ID NO: 29671 |
| | | AA | EIVMTQSPATLSVSPGERATLSCRASQSVNSNLAWYQLKPGQAPRLLISIASTRATGIPPRFNGSGSGTEFTLTISSLQSEDFAVYYCQQYNDWPCSFGQGTKLEIK | QVQLVESGGGVVQPGRSLRLSCAASGFTFSSYGMHWVRQAPGKGLEWVAVIWYDGSNKYYADSVKGRFTISRDNSKNSLYLQMNSLSAEDTAVYYCARDQGIVGATWFDYWGQGTLVTVSS |
| | | | SEQ ID NO: 25666 | SEQ ID NO: 29672 |
| iPS:434427 | 21-225_70D6 | NA | GACATCCAGATGACCCAGTCTCCATCTTCCGTGTCTGCATCTGTAGGAGACAGAGTCACCATCACTTGTCGGGCGAGTCAGGGTATTAGCAAATGGTTAGCCTGGTATCAGCAGAATCCAGGGAAAGCCCTTAAGCTCTTGATCTTTGCTGCATCCAGTTTGCAAAGTGGGGTCCCATCAAGGTTCAGCGGCAGTGGATCTGGGACAGATTTCACTCTCACCATCAGCAGCCTCCAGCCTGAAGATTTTGCAAATTACTATTGTCAACAGACTAACAGTTTCCCTCTCACTTTCGGCGGAGGGACCAAGGTGGAGATCAAA | CAGGTGCAGCTGGTGCAGTCTGGGGGCTGAGGTGAAGAAGCCTGGGGCCTCAGTGAAGGTCTCCTGCAAGGCCTCTGGATACATCTTCACCGGCTACTATATGCACTGGGTGCGACAGGCCCCTGGACAAGGGCTTGAGTGGATGGGATGGATCAACCCTAAGAGTGGTGGCACAAACTCTGCACAGAAGTTTCAGGGCAGGGTCTCCATGACCAGGGACACGTCCATCGGCACAGCCTACATGGAGCTGCGCGGGCTAAGATCTGACGACACGGCCGAGTATTACTGTGCGAGAGCTCCGGGTAAAGCAGCAGCTGGTACATGGGGATTCTTTGACTACTGGGGCCAGGGAACCCTGGTCACCGTCTCCTCA |
| | | | SEQ ID NO: 25667 | SEQ ID NO: 29673 |

FIGURE 50

| | | | | |
|---|---|---|---|---|
| | | AA | DIQMTQSPSSVSASVGDRVTITCRASQGISKWLA WYQQNPGKALKLLIFAASSLQSGVPSRFSGSGSG TDFTLTISSLQPEDFANYYCQQTNSFPLTFGGGT KVEIK | QVQLVQSGAEVKKPGASVKVSCKASGYIFTGYYM HWVRQAPGQGLEWMGWINPKSGGTNSAQKFQGR VSMTRDTSIGTAYMELRGLRSDDTAEYYCARAPGK AAAGTWGFFDYWGQGTLVTVSS |
| | | | SEQ ID NO: 25668 | SEQ ID NO: 29674 |
| iPS:434429 | 21-225_70H6 | NA | GACATCCAGATGACCCAGTCTCCATCCTCCCT GTCTGCATCTTTAGGAGACAGAGTCACCATCA CTTGCCGGACAAGTCAGAGCATTTTCAACTAT TTAAATTGGTTTCAGCGGAAACCAGGGAAAGC CCCTAAGGTCCTGATCTATACTGCATCCAGTTT GCAAAGTGGGATCCCATCAAGGTTCAGTGGCA GTGGATCTGGGACAGACTTCACTCTCACCATC AGCAGTCTGCAACCTGAAGATTCTGCAACTTA CTACTGTCAACAGAGTTACAGTATCCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGACTCCAGGCAAGGGGCT GGACTGGGTGGCAGTTATATGGCATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATGATCC CAGATCCTCGGCCGGGGACTACTGGGGCCAGGG AACCCTGGTCACCGTCTCCTCA |
| | | | SEQ ID NO: 25669 | SEQ ID NO: 29675 |
| | | AA | DIQMTQSPSSLSASLGDRVTITCRTSQSIFNYLNW FQRKPGKAPKVLIYTASSLQSGIPSRFSGSGSGTD FTLTISSLQPEDSATYYCQQSYSIPLTFGGGTKVE IK | QVQLVESGGGVVQPGRSLRLSCAASGFTFSSYGMH WVRQTPGKGLDWVAVIWHDGSNKYYADSVKGRF TISRDNSKNTLYLQMNSLRAEDTAVYYCARDDPRS SAGDYWGQGTLVTVSS |
| | | | SEQ ID NO: 25670 | SEQ ID NO: 29676 |

FIGURE 50

| iPS:434431 | 21-225_70E7 | NA | GACATCGTGATGACCCAGTCTCCAGACTCCCT GGCTGTGTCTCTGGGCGAGAGGGCCACCCTCA ACTGCAAGTCCAGCCAGAGTGTTTTATACAGC TCCAACAATAACAACTACTTGGCTTGGTACCA GCAGAAACCAGGACAGCCTCCTAAACTGCTCA TTTACTGGGCATCTACCCGGGAATCCGGGGTC CCTGACCGATTCAGTGGCAGCGGGTCTGGGAC AGATTTCACTCTCACTATCAGCAGCCTGCAGG CTGAAGATGTGGCAGTTTATTACTGTCAGCAA TATTATAATATTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGGTGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGTATAGCAGT GGCTGGTACGTCTTTGACTACTGGGGCCAGGGAA CCCTGGTCACCGTCTCCTCA |
|---|---|---|---|---|
| | | | SEQ ID NO: 25671 | SEQ ID NO: 29677 |
| | | AA | DIVMTQSPDSLAVSLGERATLNCKSSQSVLYSSN NNNYLAWYQQKPGQPPKLLIYWASTRESGVPD RFSGSGSGTDFTLTISSLQAEDVAVYYCQQYYNI PPTFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YVFDYWGQGTLVTVSS |
| | | | SEQ ID NO: 25672 | SEQ ID NO: 29678 |
| iPS:434433 | 21-225_70E8 | NA | GACATCCAGATGACCCAGTCTCCATCCTCCCT GTCTACATCTGTCGGAGACAGAGTCACCATCA CTTGCCGGGCAAGTCAGGGCATTAGAAAGGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATCGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCTCTGGGTCCGCCAGGCTCCAGGCAAGGGTCTG GAGTGGGTGGCAATTATATGGTATGATGAAAGTA ATAAATACTATGCAGACTCCGTGAAGGGCCGATT CACCATCTCCAGAGACAATTCCAAGAACACGCTG TATCTGCAAATGAACAGCCTGAGAGCCGAGGAC ACGGCTGTGTATTACTGTGCGAGAGACCTACTGG ACCCACGGGACTACTGGGGCCAGGGAACCCTGG TCACCGTCTCCTCA |
| | | | SEQ ID NO: 25673 | SEQ ID NO: 29679 |

FIGURE 50

| | | | | |
|---|---|---|---|---|
| | | AA | DIQMTQSPSSLSTSVGDRVTITCRASQGIRKDLG WYQQKPGKAPKRLIYAASSLQSGVPSRFSGSGS GTEFTLTISSLQPEDFATYYCLQHNRYPLTFGGG TKVEIK | QVQLVESGGGVVQPGRSLRLSCAASGFTFSSYGML WVRQAPGKGLEWVAIIWYDESNKYYADSVKGRFT ISRDNSKNTLYLQMNSLRAEDTAVYYCARDLLDPR DYWGQGTLVTVSS |
| | | | SEQ ID NO: 25674 | SEQ ID NO: 29680 |
| iPS:434435 | 21-225_70G9 | NA | GACATCCAAATGACCCAGTCTCCATCTTCCGT GTCTGCATCTGTAGGAGACAGAGTCACCATCA CTTGTCGGGCGAGTCAGGATATTAGCAGCTGG TTAGCCTGGTATCAGCAGAATCCAGGGAAAGC CCCTAAACTCTTGATCTATGCTGCATCCAGTTT GCAAAGTGGGGTCCCATCAAGGTTCAGCGGCA GTGGATCTGGGACAGATTTCACTCTCACCATC AGCAGCCTCCAGCCTGAAGATTTTGCAACTTA CTATTGTCAACAGACTAACAGTTTCCCTCTCAC TTTCGGCGGAGGGACCAAGGTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGAC TTGAGTGGATGGGATGGATCAAACCTAACAGTG GTGGCACAAACCAAGCACAGAAGTTTCAGGGCA GGGTCACCATGACCAGGGACACGTCCATCAGCA CAGCCTACATGGAGCTGAGCAGTCTGAGATCTGA CGACACGGCCGTGTATTACTGTGCGAGAGCTCCG GGTATAGCAGCAGCTGGTACATGGGGGATACTTTG ACTACTGGGGCCAGGGAACCCTGGTCACCGTCTC CTCA |
| | | | SEQ ID NO: 25675 | SEQ ID NO: 29681 |
| | | AA | DIQMTQSPSSVSASVGDRVTITCRASQDISSWLA WYQQNPGKAPKLLIYAASSLQSGVPSRFSGSGS GTDFTLTISSLQPEDFATYYCQQTNSFPLTFGGGT KVEIK | QVQLVQSGAEVKKPGASVKVSCKASGYTFTGYYM HWVRQAPGQGLEWMGWIKPNSGGTNQAQKFQGR VTMTRDTSISTAYMELSSLRSDDTAVYYCARAPGI AAAGTWGYFDYWGQGTLVTVSS |
| | | | SEQ ID NO: 25676 | SEQ ID NO: 29682 |

FIGURE 50

| iPS:434437 | 21-225_70A12 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCTTAAGCTCTTGATATATGCTGCATCCAGTT TGCAAAGTGGGGTCCCATCAAGGTTCAGCGGC AGTGGATCTGGGACAGATTTCACTCTCACCAT CAGCAGCGTGCAGCCTGAAGATTTTGCAACTT ACTATTGTCAACAGATTAACAGTTTCCCTCTCA CTTTCGGCGGAGGGACCAAGGTGGAGATCAA A | CAGGTGCAGCTGGTGCAGTCTGGGGGCTGAGGTG AAGAAGCCTGGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAAACCAAACAGTG GTGGCACAAACCAAGCACAGAAGTTTCAGGGCA GGGTCACCATGACCAGGGACACGTCCATCAGCA CAGCCTACATGGAGCTGAGCGGGCTGAGATCTG ACGACACGGCCGTGTATTACTGTGCGAGAGCTCC GGGTACTGCAGCAACTGGTACATGGGGATACTTT GACTACTGGGGCCAGGGAACCCTGGTCACCGTCT CCTCA |
| | | | SEQ ID NO: 25677 | SEQ ID NO: 29683 |
| | | AA | DIQMTQSPSSVSASVGDRVTITCRASQGISSWLA WYQQKPGKALKLLIYAASSLQSGVPSRFSGSGS GTDFTLTISSVQPEDFATYYCQQINSFPLTFGGGT KVEIK | QVQLVQSGAEVKKPGASVKVSCKASGYTFTGYYM HWVRQAPGQGLEWMGWIKPNSGGTNQAQKFQGR VTMTRDTSISTAYMELSGLRSDDTAVYYCARAPGT AATGTWGYFDYWGQGTLVTVSS |
| | | | SEQ ID NO: 25678 | SEQ ID NO: 29684 |
| iPS:434439 | 21-225_70E12 | NA | GACATCCAGATGACCCAGTCTCCATCCTCCCT ATCTGCATCTGTAGGAGACAGAGTCACCATCA CTTGCCGGGCAAGTCAGGGCATTAACAATAAT TTAAACTGGTTTCAGCAGAAACCAGGGAAAGC CCCTAAGCGCCTGATCTATGCTGCATCCAGTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATAATAGTTACCCGCTCA CTTTCGGCGGAGGGTCCAAGGTGGAGATCAAA | GAGGTGCAGCTGGTGGAGTCTGGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCTTCCATTAGTGGTAATAGTACT TACATATACTACACAGACTCAGTGAAGGGCCGAT TCACCATCTCCAGAGACAACGCCAAGAACTCACT GTATCTGCAAATGGACAGCCTGACAGCCGAGGA CACGGCTGTGTATTACTGTGCGAGAGTGGCCGCC TTTGACTGCTGGGGCCAGGGAACCCTGGTCACCG TCTCCTCA |
| | | | SEQ ID NO: 25679 | SEQ ID NO: 29685 |

FIGURE 50

| | | | | |
|---|---|---|---|---|
| | | AA | DIQMTQSPSSLSASVGDRVTITCRASQGINNNLN WFQQKPGKAPKRLIYAASSLQSGVPSRFSGSGSG TEFTLTISSLQPEDFATYYCLQHNSYPLTFGGGSK VEIK | EVQLVESGGGLVKPGGSLRLSCAASGFTFSSYSMN WVRQAPGKGLEWVSSISGNSTYIYYTDSVKGRFTIS RDNAKNSLYLQMDSLTAEDTAVYYCARVAAFDC WGQGTLVTVSS |
| | | | SEQ ID NO: 25680 | SEQ ID NO: 29686 |
| IPS:434441 | 21-225_71A2 | NA | GACATCCAGATGACCCAGTCTCCATCCTCCCG GTCTGCATCTGTAGGAGACAGAGTCACCATCA CTTGCCGTGCAAGTCAGGGCATTAGAAATGAT TTAGGATGGTATCAGCAGAAACCAGGAAAAG CCCCTAAGCGCCTGATCTATATTGCATTCAGA TTGCAAATTGGGGTCCCATCAAGGTTCAGCGG CAGTGGATCTGGGACAGAATTCACTCTCACAA TCAGCAGCCTGCAGCCTGAAGATTTTGCAACT TATTACTGTATACACCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAACGTCTGGATTCACCTTCAGTGACTATGGCAT GCACTGGGTCCGCCAGGCTCCAGGCAAGGGGCT GGAGTGGGTGGCAGTGATATGGTATGATGAAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGAATTGG GGTGGCAGGATGATTACTGGGGCCAGGGAACCC TGGTCACCGTCTCCTCA |
| | | | SEQ ID NO: 25681 | SEQ ID NO: 29687 |
| | | AA | DIQMTQSPSSRSASVGDRVTITCRASQGIRNDLG WYQQKPGKAPKRLIYIAFRLQIGVPSRFSGSGSG TEFTLTISSLQPEDFATYYCIHHNSYPWTFGQGT KVEIK | QVQLVESGGGVVQPGRSLRLSCATSGFTFSDYGMH WVRQAPGKGLEWVAVIWYDESNKYYADSVKGRF TISRDNSKNTLYLQMNSLRAEDTAVYYCARELGW QDDYWGQGTLVTVSS |
| | | | SEQ ID NO: 25682 | SEQ ID NO: 29688 |

FIGURE 50

| iPS:434443 | 21-225_71G3 | NA | GACATCGTGATGACCCAGTCTCCAGACTCCCTGGCTGTGTCTCTGGGCGAGAGGGTCGCCATCAATTGCAAGTCCAGCCAGAGTGTTTTACACAGCTCCAACAATAACAACTACTTAGATTGGTATCAGCAGAAACCAGGACAGCTTCCTAAACTGCTCATTTTCTGGGCATCTACCCGGGAATTCGGGGTTCCTGACCGATTCAGTGGCAGCGGGTTTGGGACAGATTTCACTCTCACCATCAGCAGCCTGCAGGCTGAAGATGTGGCAGATTACTACTGTCAACAATATTATATTACTCCGTGCAGTTTTGGCCAGGGGACCAAGCTGGAGATCAAA | CAGGTGCAACTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAGCACAGGCTATGCACAGAAGTTCCAGGGCAGAGTCACCATGACCAGGGACACCTCCGTCAGCACAGCCTACATGGAGCTGAGCAGCCTGAGATCTGAGGACACGGCCGTCTATTACTGTGCATATAGCAGTGGCTGGTACTACTTTGACTACTGGGGCCAGGGAACCCTGGTCACCGTCTCCTCA |
|------------|-------------|-----|---|---|
| | | | SEQ ID NO: 25683 | SEQ ID NO: 29689 |
| | | AA | DIVMTQSPDSLAVSLGERVAINCKSSQSVLHSSNNNNYLDWYQQKPGQLPKLLIFWASTREFGVPDRFSGSGFGTDFTLTISSLQAEDVADYYCQQYYITPCSFGQGTKLEIK | QVQLVQSGAEVKKPGASVKVSCKASGYTFTNYDINWVRQATGQGLEWMGWMHPNSGSTGYAQKFQGRVTMTRDTSVSTAYMELSSLRSEDTAVYYCAYSSGWYYFDYWGQGTLVTVSS |
| | | | SEQ ID NO: 25684 | SEQ ID NO: 29690 |
| iPS:434447 | 21-225_71B6 | NA | GACATCCAGATGACCCAGTCTCCATCCTCCCCGTCTGCATCTGTAGGAGACAGAGTCACCATCACTTGCCGGGCAAGTCAGGGCATTAGAAATGATTTAGATTGGTATCAGCAGAAGCCAGGGAAGGCCCCTCAGCGCCTTATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGACGATTTTGCAACTTATTACTGTCTACAGCATAATAGTTACCCATTCACTTTCGGCCCTGGGACCAAAGTGGATATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCCCCAGGCAAGGGGCTGGAGTGGGTGGCAGTTATTTGGTATGATAGAACAAATAAATACTATGCAGACTCCGTGAAGGGCCGATTCACCATCTCCAGAGACAATTCCAAGAACACGCTGCATCTGCAAATGAACAGCCTGAGAGCCGAGGACACGGCTGTGTATTACTGTGCGAGAGAACTGGGGATGTTGTCTGACTACTGGGGCCAGGGAACCCTGGTCACCGTCTCCTCA |
| | | | SEQ ID NO: 25685 | SEQ ID NO: 29691 |

FIGURE 50

| | | | | | |
|---|---|---|---|---|---|
| | | AA | DIQMTQSPSSPSASVGDRVTITCRASQGIRNDLD WYQQKPGKAPQRLIYAASSLQSGVPSRFSGSGS GTEFTLTISSLQPDDFATYYCLQHNSYPFTFGPGT KVDIK | QVQLVESGGGVVQPGRSLRLSCAASGFTFSNYGM HWVRQAPGKGLEWVAVIWYDRTNKYYADSVKGR FTISRDNSKNTLHLQMNSLRAEDTAVYYCARELGM LSDYWGQGTLVTVSS | |
| | | | SEQ ID NO: 25686 | SEQ ID NO: 29692 | |
| IPS:434449 | 21-225_71H6 | NA | GACATCCAGATGACCCAGTCTCCATCCTCCCT GTCTGCATCTGTAGGAGACAGAGTCACCATCA CTTGCCGGGCAAGTCAGGGCATTAGAAATGTT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGTATAATAGTTACCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTGTCCTGTG CAGCCTCTGGATTCACCTTCAGTAGCTATAGCAT GAACTGGGTCCGCCAGGCTCCAGGGAAGGGACT GGAGTGGGTCTCATCCATTAGTGGTACTAGTAGT TACATATACTACGCAGACTCAGTGAAGGGCCGAT TCACCATCTCCAGAGACAACGCCAAGAACTCACT GTATCTGCAAATGAACGGCCTGAGAGCCGAGGA CACGGCTGTGTATTTCTGTGCGAAAACCAATGCT TTTGATATCTGGGGCCAGGGGACAATGGTCACCG TCTCTTCA | |
| | | | SEQ ID NO: 25687 | SEQ ID NO: 29693 | |
| | | AA | DIQMTQSPSSLSASVGDRVTITCRASQGIRNVLG WYQQKPGKAPKRLIYAASSLQSGVPSRFSGSGS GTEFTLTISSLQPEDFATYYCLQYNSYPFTFGPGT KVDIK | EVQLVESGGGLVKPGGSLRLSCAASGFTFSSYSMN WVRQAPGKGLEWVSSISGTSSYIYYADSVKGRFTIS RDNAKNSLYLQMNGLRAEDTAVYFCAKTNAFDIW GQGTMVTVSS | |
| | | | SEQ ID NO: 25688 | SEQ ID NO: 29694 | |

FIGURE 50

| | | | | |
|---|---|---|---|---|
| iPS:434451 | 21-225_71B7 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAATCCAGGGGAAGC CCCTAAGCTCCTGATCTATGCTGCATCCAGTTT GCAAGGTGGGGTCCCATCAAGGTTCAGCGGCA GTGGATCTGGGACAGATTTCACTCTCACCATC AGCAGCCTCCAGCCTGAAGATTTTGCAAATTA CTATTGTCAACAGACTAATAGTTTCCCTCTCAC TTTCGGCGGAGGGACCAAGGTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACATCTTCACCGGCTACTATA TGCACTGGGTGCGACAGGCCCCTGGACAAGGGC TTGAGTGGATGGGATGGATCAACCCTAACAGTGG TGGCACAAACTCTGCACAGAAGTTTCAGGGCAG GGTCACCATGACCAGGGACACGTCCATCGGCAC AGCCTACATGGAGCTGAGCGGGCTGAGATCTGA CGACACGGCCGTGTATTACTGTGCGAGAGCTCCG GGTAAAGCAGCAGCTGGTACATGGGGATTCTTTG ACTACTGGGGCCAGGGAACCCTGGTCACCGTCTC CTCA |
| | | | SEQ ID NO: 25689 | SEQ ID NO: 29695 |
| | | AA | DIQMTQSPSSVSASVGDRVTITCRASQGISSWLA WYQQNPGEAPKLLIYAASSLQGGVPSRFSGSGS GTDFTLTISSLQPEDFANYYCQQTNSFPLTFGGG TKVEIK | QVQLVQSGAEVKKPGASVKVSCKASGYIFTGYYM HWVRQAPGQGLEWMGWINPNSGGTNSAQKFQGR VTMTRDTSIGTAYMELSGLRSDDTAVYYCARAPG KAAAGTWGFFDYWGQGTLVTVSS |
| | | | SEQ ID NO: 25690 | SEQ ID NO: 29696 |
| iPS:434453 | 21-225_71B11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGATTGGTATCAGCAGACACCAGGGAAAGC CCCTAAGCGCCTGATCTATGCTGCATCCAGTTT GCAAAGTGGGGTCCCATCAAGGTTCAGCGGCG GTGGATCTGGGACAGAATTCTCTCTCACAATC AGCAGCCTGCAGCCTGAAGATTTTACAACTTA TTACTGTCTACAGCATAATACTTACCCATTCAC TTTCGGCCCTGGGACCAAAGTGGATGTCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATAGAAAT AATAAATACTATGGAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACTGGG GATGTTGTCTGACTACTGGGGCCAGGGAACCCTG GTCACCGTCTCCTCA |
| | | | SEQ ID NO: 25691 | SEQ ID NO: 29697 |

FIGURE 50

| | | | | |
|---|---|---|---|---|
| | | AA | DIQMTQSPSSLSASVGDRVTITCRASQGIRNDLD WYQQTPGKAPKRLIYAASSLQSGVPSRFSGGGS GTEFSLTISSLQPEDFTTYYCLQHNTYPFTFGPGT KVDVK | QVQLVESGGGVVQPGRSLRLSCAASGFTFSDYGM HWVRQAPGKGLEWVAVIWYDRNNKYYGDSVKG RFTISRDNSKNTLYLQMNSLRAEDTAVYYCARELG MLSDYWGQGTLVTVSS |
| | | | SEQ ID NO: 25692 | SEQ ID NO: 29698 |
| iPS:434455 | 21-225_72F5 | NA | GACATCCAGATGACCCAGTCTCCATCCTCCCT GTCTGCATCTGTAGGAGACAGAGTCATCATCC CTTGCCGGGCAAGTCAGAACATTAGCAGCTAT TTAAATTGGTATCAGCAGAAACCAGGGAAAG CCCCTAAACTCCTGATCTATGCTGCATCCAGTT TGCAAAGTGGGGTCCCATCAAGGTTCAGTGGC AGTGGATCTGGGACAGATTTCACTCTGACCAT CAGCAGTCTGCAGCCTGAAGATTTTGCAACTT ACTCCTGTCAACAGACTTACAGTACCCCCACC TTCGGCCAAGGGACACGACTGGATATTAAT | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GATCTGGGTCCGCCAGGCTCCAGGGAAGGGGCT GGAGTGGGTCTCAACTATTAGTGGTAGTGGTGGT TACACATACTCCGCAGACTCCGTGAAGGGCCGGT TCACCATCTCCAGAGACAATTCCAAGAACACGCT GTATCTTCAAATGAACAGCCTGAGAGCCGAGGA CACGGCCGTATATTACTGTGCGAGACGTATAGCA GTGACTGGGACGGAATGGTACGACCCCTGGGGC CAGGGAACCCTGGTCACCGTCTCCTCA |
| | | | SEQ ID NO: 25693 | SEQ ID NO: 29699 |
| | | AA | DIQMTQSPSSLSASVGDRVIIPCRASQNISSYLNW YQQKPGKAPKLLIYAASSLQSGVPSRFSGSGSGT DFTLTISSLQPEDFATYSCQQTYSTPTFGQGTRLD IN | EVQLLESGGGLVQPGGGSLRLSCAASGFTFSSYAMI WVRQAPGKGLEWVSTISGSGGYTYSADSVKGRFTI SRDNSKNTLYLQMNSLRAEDTAVYYCARRIAVTGT EWYDPWGQGTLVTVSS |
| | | | SEQ ID NO: 25694 | SEQ ID NO: 29700 |

FIGURE 50

| iPS:434457 | 21-225_72G12 | NA | GACATCCAGTTGACCCAGTCTCCATCCTCCCTGTCTGCATCTGTAGGAGACAGAGTCACCATCACTTGCCGGGCGAGTCAGGGCATTAGCAGTTATTTAAATTGGTCTCAGCAGAAACCAGGGAAAGTTCCTAAGCTCCTGATCTGTGGTGCTTCCAATTTGCAATCTGGAGTCCCATCTCGGTTCAGCGGCAGTGCATCTGGGACAGAATTCATTCTCACTATCAGCAGCCTGCAGCCTGAAGATGTTACAACTTATTACGGTCAACAGAATTACAATGCCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGACACGGGGCTGGAGTGGGTGGCAGTTATATGGTTTGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTTGGTTTCAGCAGTGACTACTGGGGCCAGGGAACCCTGGTCACCGTCTCCTCA |
|---|---|---|---|---|
| | | | SEQ ID NO: 25695 | SEQ ID NO: 29701 |
| | | AA | DIQLTQSPSSLSASVGDRVTITCRASQGISSYLNWSQQKPGKVPKLLICGASNLQSGVPSRFSGSASGTEFILTISSLQPEDVTTYYGQQNYNAPLTFGGGTKVEIK | QVQLVESGGGVVQPGRSLRLSCAASGFTFSSYGMHWVRQAPDTGLEWVAVIWFDESNKYYADSVKGRFTISRDNSKNTLYLQMNSLRAEDTAVYYCARELGFSSDYWGQGTLVTVSS |
| | | | SEQ ID NO: 25696 | SEQ ID NO: 29702 |
| iPS:434459 | 21-225_71A7 | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCTGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGTTAACAGTTTCCCTCTCACTTTCGGCGGAGGGACCAAGGTGGAGCTCAAA | CAGGTGCAGCTGGTGCAGTCTGGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAAAAGTGGTGGCACAAATTATGTACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAGCTCCGGGTACAGCACCAGCTGGGTCATGGGGATACTTTGACTACTGGGGCCAGGGAACCCTGGTCACCGTCTCCTCA |
| | | | SEQ ID NO: 25697 | SEQ ID NO: 29703 |

FIGURE 50

| | | | | |
|---|---|---|---|---|
| | | AA | DIQMTQSPSSVSASVGDRVTITCRASQGISSWLA WYQQKPGKAPKLLIYAASSLQSGVPSRFSGSGS GTDFTLTISSLQPEDFATYYCQQVNSFPLTFGGG TKVELK | QVQLVQSGAEVKKPGASVKVSCKASGYTFTGYYM HWVRQAPGQGLEWMGWINPKSGGTNYVQKFQGR VTMTRDTSISTAYMELSSLRSDDTAVYYCARAPGT APAGSWGYFDYWGQGTLVTVSS |
| | | | SEQ ID NO: 25698 | SEQ ID NO: 29704 |
| iPS:434461 | 21-225_73A3 | NA | GACATCCAGATGACCCAGTCTCCATCTTCCGT GTCTGCATCTATAGGAGACAGAGTCACCATCA CTTGTCGGGCGAGTCAGGGTATTAGCAACTGG TTAGCCTGGTATCAGCAGAAACCAGGGAAAG CCCCTAAGCTCCTGATCTATGCTGCATCCAGTT TGCAAAGTGGGGTCCCGTCAAGGTTCAGCGGC AGTGGATCTGAGACAGATTTCACTCTCACCAT CAGCAGCCTGCAGCCTGAAGATTTTGCAACTT ACTATTGTCAACAGGTTAACAGTTTCCCTCTCA CTTTCGGCGGAGGGACCAAGGTGGAGATCAA A | CAGGTGCAACTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTACGACAGGCCCCTGGACAAGGAC TTGACTGGATGGGATGGATCAACCCTAAAAGTGG TGGCACGAATCATGTCCAGAAGTTTCAGGGCAG GGTCGCCATGACCAGGGACACGTCCATCAGCAC AGCCTACATGGAGCTGAGCAGTCTGAGATCTGAC GACACGGCCGTGTATTACTGTGCGAGAGCTCCGG GTACAGCAGCAGCTGGGTCATGGGGGATGCTTTGA CTACTGGGGCCAGGGAACCCTGGTCACCGTCTCC TCA |
| | | | SEQ ID NO: 25699 | SEQ ID NO: 29705 |
| | | AA | DIQMTQSPSSVSASIGDRVTITCRASQGISNWLA WYQQKPGKAPKLLIYAASSLQSGVPSRFSGSGSE TDFTLTISSLQPEDFATYYCQQVNSFPLTFGGGT KVEIK | QVQLVQSGAEVKKPGASVKVSCKASGYTFTGYYM HWVRQAPGQGLDWMGWINPKSGGTNHVQKFQGR VAMTRDTSISTAYMELSSLRSDDTAVYYCARAPGT AAAGSWGCFDYWGQGTLVTVSS |
| | | | SEQ ID NO: 25700 | SEQ ID NO: 29706 |

1927

FIGURE 50

| iPS:434463 | 21-225_73A6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTTTCAGCAGAAACCAGGGAAAGC CCCTAAGCGCCTGATCTATGCTGCATCCAATTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATAATAGTTACCCATTCAC TTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTTACTATGGCAT GCACTGGGTCCGCCAGGCTCCAGGCAAGGGGCT GGAGTGGGTGGCAGTTATATTATATGATGGAAGT AAGAAATACTATGCAGCCTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGCGAGGAGTATCCC GGACTTTGACTACTGGGGCCAGGGAACCCTGGTC ACCGTCTCCTCA |
|---|---|---|---|---|
| | | | SEQ ID NO: 25701 | SEQ ID NO: 29707 |
| | | AA | DIQMTQSPSSLSASVGDRVTITCRASQGIRNDLG WFQQKPGKAPKRLIYAASNLQSGVPSRFSGSGS GTEFTLTISSLQPEDFATYYCLQHNSYPFTFGPGT KVDIK | QVQLVESGGGVVQPGRSLRLSCAASGFTFSYYGM HWVRQAPGKGLEWVAVILYDGSKKYYAASVKGR FTISRDNSKNTLYLQMNSLRAEDTAVYYCARSIPDF DYWGQGTLVTVSS |
| | | | SEQ ID NO: 25702 | SEQ ID NO: 29708 |
| iPS:434467 | 21-225_73H8 | NA | GACATCCAGATGACCCAGTCTCCATCCTCCCT GTATGCATCTGTAGGAGACAGAGTCACCATCA CTCGCCGGGCAAGTCAGGACATCAGAAATGAT TTAGGCTGGTATCAGCAGAAACCAGGGAAAG CCCTTAAGCGCGTGATCTATGCTGCATCCAGT TTGCAAAGTGGGGTCCCATCAAGCTTCAGCGG CAGTGGATCTGGGACAGAATTCACTCTCACAA TCAGCAGCTTGCAGCCTGAAGATTTTGCAACT TATTACGGTATACAGCATAATAGTTACCCTCC GATCACCGTCGGCCAAGGGACACGACTGGAG ATTAAA | GAGGTGCAGTTACTGGAGTCTGGGGGAGGCTGG GTACAGTCTGGGGGGTCCCTGAGACTCTCCTGTG CAGCCTCTGGATTCACCTTTAGCAGCAATGCCAT GAGCTGGGTCCGCCAGGCTCCAGGGAAGGGGCT GGAGTGGGTCTCAGACATTAGTCGTAGTGGTGGT ACCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAATGGGATAGC AGCAGCTGGTACGACGTGACTCCCTTTGACTACT GGGGCCAGGGAACCCTGGTCACCGTCTCCTCA |
| | | | SEQ ID NO: 25703 | SEQ ID NO: 29709 |

FIGURE 50

| | | | | |
|---|---|---|---|---|
| | | AA | DIQMTQSPSSLYASVGDRVTITRRASQDIRNDLG WYQQKPGKALKRVIYAASSLQSGVPSSFSGSGS GTEFTLTISSLQPEDFATYYGIQHNSYPPITVGQG TRLEIK | EVQLLESGGGWVQSGGSLRLSCAASGFTFSSNAMS WVRQAPGKGLEWVSDISRSGGTTFYADSVKGRFTI SRDNSKNTLYLQMNSLRAEDTAVYYCAKWDSSSW YDVTPFDYWGQGTLVTVSS |
| | | | SEQ ID NO: 25704 | SEQ ID NO: 29710 |
| iPS:434469 | 21-225_73C9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGCAATTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGCGAGAGAAAGGTA TAGCAGCAGCTGGTTCGACTACGGTATGGACGTC TGGGGCCAAGGGACCACGGTCACCGTCTCCTCA |
| | | | SEQ ID NO: 25705 | SEQ ID NO: 29711 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEIK | QVQLVESGGGVVQPGRSLRLSCAASGFTFSNYVM HWVRQAPGKGLEWVAVIWYDGSNKYYADSVKGR FTISRDNSKNTLYLQMNSLRAEDTAVYYCARERYS SSWFDYGMDVWGQGTTVTVSS |
| | | | SEQ ID NO: 25706 | SEQ ID NO: 29712 |

FIGURE 50

| | | | | |
|---|---|---|---|---|
| iPS:434471 | 21-225_75G3 | NA | GAAATTGTGTTGACGCAGTCGCCAGGCACCCT GTCTTTGTCTCCAGGGGAGAGAGCCACCCTCT CCTGCAGGGCCCGTCAGAATGTTGACAGCAGC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTTTTACTGTCAGCAGTATGAACGCTCACCG TGGACGTTCGGCCAAGGGACCAAGGTGGAGA TCAAA | CAGGTGCAGCTACAGCAGTGGGGCGCAGGACTG TTGAAGCCTTCGGAGACCCTGTCCCTCACCTGCG CTGTCTATGGTGGGTCCCTCAGTGGTTCCTACTG GAGCTGGATCCGCCAGCCCCCAGGGAAGGGGCT GGAGTGGATTGGGGAAATCAATCTTAGTGGAAG TACCAACTACAACCCGTCCCTCAAGAGTCGAGTC ACCATATCAGTAGACACGTCCAAGAGCCAGTTCT CCCTGACGCTGCGCTCTGTGACCGCCGCGGACAC GGCTGTGTATTACTGTGCGAGAGACTACGGTGGC CTTGACTACTGGGGCCAGGGAACCCTGGTCACCG TCTCCTCA |
| | | | SEQ ID NO: 25707 | SEQ ID NO: 29713 |
| | | AA | EIVLTQSPGTLSLSPGERATLSCRARQNVDSSYL AWYQQKPGQAPRLLIYGASSRATGIPDRFSGSGS GTDFTLTISRLEPEDFAVFYCQQYERSPWTFGQG TKVEIK | QVQLQQWGAGLLKPSETLSLTCAVYGGSLSGSYW SWIRQPPGKGLEWIGEINLSGSTNYNPSLKSRVTISV DTSKSQFSLTLRSVTAADTAVYYCARDYGGLDYW GQGTLVTVSS |
| | | | SEQ ID NO: 25708 | SEQ ID NO: 29714 |
| iPS:434473 | 21-225_76D1 | NA | GAAATTGTGTTGACGCAGTCTCCAGGCACCCT GTCTTTGTCTCCAGGGGAAAGAGCCACCCTCT CCTGCAGGGCCAGTCAGAATATTTACAGCAAC TACCTAGCCTGGTACCAGGAGAAGCCTGGCCA GGCTCCCAGGACTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGTAG TGTATTACTGTCAGCAGTATGAAAGTTCACCG TGGACGTTCGGCCAAGGGACCAAGGTGGAAA TCAAA | CAGGTACAACTACAGCAGTGGGGCGCAGGACTG TTGAAGCCTTCGGAGACCCTGTCCCTCACCTGCG CTGTCTATAGTGGGTCCTTCAGTGGTTGCTACTG GAGCTGGATCCGCCAGCCCCCAGGGAAGGGGCT GGAGTGGATTGGGGAAATCAATCATAGTGGAAG AACCAACTACAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAGGGGACCACGGTCACC GTCTCCTCA |
| | | | SEQ ID NO: 25709 | SEQ ID NO: 29715 |

FIGURE 50

| | | | | |
|---|---|---|---|---|
| | | AA | EIVLTQSPGTLSLSPGERATLSCRASQNIYSNYLA WYQEKPGQAPRLLIYGASSRATGIPDRFSGSGSG TDFTLTISRLEPEDFVVYYCQQYESSPWTFGQGT KVEIK | QVQLQQWGAGLLKPSETLSLTCAVYSGSFSGCYW SWIRQPPGKGLEWIGEINHSGRTNYNPSLKSRVTIS VDTSKNQFSLKLSSVTAADTAVYYCARDYGGMDV WGQGTTVTVSS |
| | | | SEQ ID NO: 25710 | SEQ ID NO: 29716 |
| iPS:434475 | 21-225_74F9 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATTACAACTACTTAGCTTGGTACCA GCAGAAACCAGGACAGCCTCCTAAGAAGCTC ATTTACTGGGCATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTCCTGTCAGCA ATATTATAGTAGTCCTCCGACGTTCGGCCAAG GGACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTGTGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGTAGCA GTGGCTGGAACTTCTTTGACTACTGGGGCCAGGG AACCCTGGTCACCGTCTCCTCA |
| | | | SEQ ID NO: 25711 | SEQ ID NO: 29717 |
| | | AA | DIVMTQSPDSLAVSLGERATINCKSSQSVLYSSN NYNYLAWYQQKPGQPPKKLIYWASTRESGVPD RFSGSGSGTDFTLTISSLQAEDVAVYSCQQYYSS PPTFGQGTKVEIK | QVQLVQSGAEVKKPGASVKVSCKASGYTFTNYDIN WVRQATGQGLEWMGWMNPNSGNTGCAQKFQGR VTMTRNTSISTAYMELSSLRSEDTAVYYCASSSGW NFFDYWGQGTLVTVSS |
| | | | SEQ ID NO: 25712 | SEQ ID NO: 29718 |

FIGURE 50

| | | | | |
|---|---|---|---|---|
| iPS:434477 | 21-225_74A6 | NA | GACATCGTGATGACCCAGTCTCCAGACTCCCT GGCTGTGTCTCCGGGCGAGAGGGCCACCATCA ACTGCAAGTCCAGCCAGAGTGTTTTACACAGC TCCAACAATAACAATTACTTAGCCTGGTACCA GCAGAAACCAGGACAGCCTCCTGACCTGCTCA TTTACTGGGCATCAACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTTTAGTACTCCGTGGACGTTCGGCCAAGG GACCCAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCGGGGCAG AGTCACCATGACCTGGAACACCTCCATAAGCACA GCCTACATGGAGCTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTCTCCTCA |
| | | | SEQ ID NO: 25713 | SEQ ID NO: 29719 |
| | | AA | DIVMTQSPDSLAVSPGERATINCKSSQSVLHSSN NNNYLAWYQQKPGQPPDLLIYWASTRESGVPD RFSGSGSGTDFTLTISSLQAEDVAVYYCQQYFST PWTFGQGTQVEIK | QVQLVQSGAEVKKPGASVKVSCKASGYTFTNYDIN WVRQATGQGLEWMGWMHPNSGNTGYAQKFRGR VTMTWNTSISTAYMELSSLRSEDTAVYYCASSSGW YYFDYWGQGTLVTVSS |
| | | | SEQ ID NO: 25714 | SEQ ID NO: 29720 |
| iPS:434479 | 21-225_76H1 | NA | GAATTTATGTTGACGCAGTCTCCAGGCACCCT GTATTGGTCTCCAGGGGAAAGAGCCCACCCTCT CGTGCAGGGCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
| | | | SEQ ID NO: 25715 | SEQ ID NO: 29721 |

FIGURE 50

| | | | | |
|---|---|---|---|---|
| | | AA | EFMLTQSPGTLYWSPGERATLSCRASQSVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25716 | SEQ ID NO: 29722 |
| iPS:434481 | 21-225_74B10 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATAAGAACTACTTAACTTGGTACCA GCAGAAACCAGGACAGCCTCCTAAGCTGCTCA TTTACTGGGCATCTACTCGGGAATCCGGGGTC CCTGACCGATTCAGTGGCAGCGGGTCTGGGAC AGATTTCAGTCTCACGATCGGCAGCCTGCAGG CTGAAGATGTGGCAGTTTATTACTGTCAGCAA TATTATAGTATTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATTCACCTTCACCAATTATGATA TCAACTGGGTGCGACAGGCCACTGGACAAGGGC TTGAGTGGATGGGATGGATGCACCCTAACAGTGG TAACACAGGCTTTGCACAGAAGTTCCAGGGCAG AGTCACCATGACCAGGAACACCTCCATAAGCAC AGCCTACATGGAACTGAGCAGCCTGAGATCTGA GGACACGGCCGTGTATTACTGTGCGGTTTCCAGT GGCTGGTACTGGTTCGACCCCTGGGGCCAGGGA ACCCTGGTCACCGTCTCCTCA |
| | | | SEQ ID NO: 25717 | SEQ ID NO: 29723 |
| | | AA | DIVMTQSPDSLAVSLGERATINCKSSQSVLHSSN NKNYLTWYQQKPGQPPKLLIYWASTRESGVPDR FSGSGSGTDFSLTIGSLQAEDVAVYYCQQYYSIP PTFGQGTKVEIK | QVQLVQSGAEVKKPGASVKVSCKASGFTFTNYDIN WVRQATGQGLEWMGWMHPNSGNTGFAQKFQGR VTMTRNTSISTAYMELSSLRSEDTAVYYCAVSSGW YWFDPWGQGTLVTVSS |
| | | | SEQ ID NO: 25718 | SEQ ID NO: 29724 |

FIGURE 50

| iPS:434483 | 21-225_74C12 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATGCGAACTACTTAGCTTGGTACCA GCAGAAACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGATTAGCAG TGGCTGGTACTGGTTCGACCCCTGGGGCCAGGGA ACCCTGGTCACCGTCTCCTCA |
| | | | SEQ ID NO: 25719 | SEQ ID NO: 29725 |
| | | AA | DIVMTQSPDSLAVSLGERATINCKSSQSVLYSSN NANYLAWYQQKPGQPPNLLIYWASTRESGVPD RFSGSGSGTDFTLTISSLQAEDVAVYYCQQYYST PCSFGQGTKLEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAISSGW YWFDPWGQGTLVTVSS |
| | | | SEQ ID NO: 25720 | SEQ ID NO: 29726 |
| iPS:434485 | 21-225_76D2 | NA | GAAATAGTGATGACGCAGTCTCCAGCCACCCC GTCTGTGTCTCCAGGGGAAAGAGCCACCCTCT CCTGCAGGGCCAGTGTGAGTGTTGTCAACAGC TTAGCCTGGTACCAGCAGAAACCTGGCCAGGC TCCCAGGCTCCTCATCCATGGTGCATCCACCA GGGCCACTGGTATCCCAGCCAGGTTCAGTGGC AGTGGGTCTGGGACAGAGTTCACTCTCACCAT CAGCAGTGTGCAGTCTGAAGATTTTGCAATTT ATTACTGTCAGCAATATAATGACTGGCCGTGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATTTGGTATGATGGAAGT AATAAATACTATGCAGACTCCGTGAAGGGCCGA TTCACCATCTCCAGAGACAATTCCAAGAACACGC TGTTTCTGCAAATGAACAGCCTGAGAGCCGAGG ACACGGCTGTGTATTACTGTGCGAGCGATCGCAA TATAGTGGGAGCTACTTACTTTGAGTCCTGGGGC CAGGGAACCCTGGTCACCGTCTCCTCA |
| | | | SEQ ID NO: 25721 | SEQ ID NO: 29727 |

FIGURE 50

| | | | | |
|---|---|---|---|---|
| | | AA | EIVMTQSPATPSVSPGERATLSCRASVSVVNSLA WYQQKPGQAPRLLIHGASTRATGIPARFSGSGSG TEFTLTISSVQSEDFAIYYCQQYNDWPCSFGQGT KLEIK | QVQLVESGGGVVQPGRSLRLSCAASGFTFSSYGMH WVRQAPGKGLEWVAVIWYDGSNKYYADSVKGRF TISRDNSKNTLFLQMNSLRAEDTAVYYCASDRNIV GATYFESWGQGTLVTVSS |
| | | | SEQ ID NO: 25722 | SEQ ID NO: 29728 |
| iPS:434487 | 21-225_76G2 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATTACAACTACTTAGCTTGGTACCA GCAGAGACCAGGACAGCCTCCTAGGCTGCTCA TTTACTGGGCATCTACCCGGGAATCCGGGGTC CCTGACCGATTCAGTGGCAGCGGTTCTGGGAC AGATTTCACTCTCACCATCAGCAGCCTGCAGG TTGAAGATGTGGCAGTTTATTACTGTCAGCAA TATTATAGTTCTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGAC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTATATGGAGCTGAGCAGCCTGAGATCTGA AGACACGGCCGTGTATTACTGTGCGGGTAGCAGT GGCTGGTACATGTTTGACTACTGGGGGCCAGGGAA CTCTGGTCACCGTCTCCTCA |
| | | | SEQ ID NO: 25723 | SEQ ID NO: 29729 |
| | | AA | DIVMTQSPDSLAVSLGERATINCKSSQSVLHSSN NYNYLAWYQQRPGQPPRLLIYWASTRESGVPDR FSGSGSGTDFTLTISSLQVEDVAVYYCQQYYSSP PTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAGSSGW YMFDYWGQGTLVTVSS |
| | | | SEQ ID NO: 25724 | SEQ ID NO: 29730 |

FIGURE 50

| iPS:434489 | 21-225_74E4 | NA | GACATCCAGATGACCCAGTCTCCATCCTCCCT GTCTGCTTCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCACTT TGCAAAGTGGGGTCCCATCAAGGTTCAGCGGC AGTGGATCTGGGACAGAATTCACTCTCACAAT CAGCGGCCTGCAGCCTGAAGATTTTGCAACTT ATCACTGTCTACAGCATAGTAATTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCATCTGTA CTGTCTCTGGTGGCTCCATCAGCAGTAGTAATTA CTACTGGGGCTGGATCCGCCAGCCCCCAGGGAA GGGGCTGGAGTGGATTGGGAGTATCTATTATAGT GGATACACCTCCTACAACCCGTCCCTCAAGAGTC GAGTCACCATATCCGTAGACTCGTCCAAGAACCA CTTCTCCCTGAGGCTGAGCTCTGTGACCGCCGCA GACACGGCTGTGTATTACTGTGCGAGACTTGACT CTAACTGGGGTCTTGACTACTGGGGCCAGGGAAC CCTGGTCACCGTCTCCTCA |
| | | | SEQ ID NO: 25725 | SEQ ID NO: 29731 |
| | | AA | DIQMTQSPSSLSASVGDRVTITCRASQGIRNDLG WYQQKPGKAPKRLIYAASTLQSGVPSRFSGSGS GTEFTLTISGLQPEDFATYHCLQHSNYPLTFGGG TKVEIK | QLQLQESGPGLVKPSETLSLICTVSGGSISSSNYYW GWIRQPPGKGLEWIGSIYYSGYTSYNPSLKSRVTIS VDSSKNHFSLRLSSVTAADTAVYYCARLDSNWGL DYWGQGTLVTVSS |
| | | | SEQ ID NO: 25726 | SEQ ID NO: 29732 |
| iPS:434493 | 21-225_76F3 | NA | GACATCGTTATGACCCAGTGTCCAGACTCCCC GGCTGTGTCTCTGGGCGAGAGGGGCCACCATCA ATTGCAAGTCCAGCCAGAGTGTTTTATTCAGC TCCAACAATTATAATTACTTAGCTTGGTACCA GCAGAGACCAGGACAGCCTCATGACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGCTGATGTGGCAGTTTATTACTGTCAGCAA TATCATAGTTCTCCTCTGACGTTCGGCCAAGG GACCACGGTGCAAATCAAA | CAGGTGCAGTTGGTGCAGTCTGGGGCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGAACCCTAACAGTGG TAACACAGGCTATGCACAGAAGTTCCAGGGCAG AGTCACCATGACCAGGAACACCTCCACAAGCAC AGCCCACATGGAGCTGAGCAGCCTGAGATCTGA GGACACGGCCGTGTATTACTGTGCGGTCTCCAGT GGCTGGAACTGGTTCGACCCCTGGGGCCAGGGA ACCCTGGTCACCGTCTCCTCA |
| | | | SEQ ID NO: 25727 | SEQ ID NO: 29733 |

FIGURE 50

| | | | | |
|---|---|---|---|---|
| | | AA | DIVMTQCPDSPAVSLGERATINCKSSQSVLFSSN NYNYLAWYQQRPGQPHDLLIYWASTRESGVPD RFSGSGSGTDFTLTISSLQAADVAVYYCQQYHSS PLTFGQGTTVQIK | QVQLVQSGAEVKKPGASVKVSCKASGYTFTNYDIN WVRQATGQGLEWMGWMNPNSGNTGYAQKFQGR VTMTRNTSTSTAHMELSSLRSEDTAVYYCAVSSGW NWFDPWGQGTLVTVSS |
| | | | SEQ ID NO: 25728 | SEQ ID NO: 29734 |
| IPS:434495 | 21-225_74B2 | NA | GAACTTGTGTTGACGCAGTCTCCAGGCACCCT GTCTTTGTCTCCAGGGGAAAGAGCCACCCTCT CCTGCAGGGCCAGTCAGAATATTTACAGCAGT TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTGCTGTCAGCAGTATGAAAGCTCACCG TGGACCTTCGGCCAAGGGACCAAGGTGGAAA TCAAA | CAAGTGCAGCTACAGCAGTGGGGCGCAGGACTG TTGAAGCCTTCGGAGCCCCTGTCCCTCACCTGCG CTGTCTATGGTGGGTCCTTCAGTGGTCCCTACTG GAGCTGGATCCGCCAGCCCCCCGGGAAGGGGCT GGAGTGGATTGGGGAAATCAATCATAGTGGAAG CACCAACTACAACCCGTCCCTCACGAGTCGAGTC ACCATATCAGTAGACACGTCCAAGAACCAGTTCT CCCTGAAACTGACCTCTGTGACCGCCGCGGACTC GGCTGTGTATTACTGTGCGAGAGACTACGGGGGT TTGGACGTCTGGGGCCAAGGGACCACGGTCACC GTCTCCTCA |
| | | | SEQ ID NO: 25729 | SEQ ID NO: 29735 |
| | | AA | ELVLTQSPGTLSLSPGERATLSCRASQNIYSSYLA WYQQKPGQAPRLLIYGASSRATGIPDRFSGSGSG TDFTLTISRLEPEDFAVYCCQQYESSPWTFGQGT KVEIK | QVQLQQWGAGLLKPSEPLSLTCAVYGGSFSGPYW SWIRQPPGKGLEWIGEINHSGSTNYNPSLTSRVTISV DTSKNQFSLKLTSVTAADSAVYYCARDYGGLDVW GQGTTVTVSS |
| | | | SEQ ID NO: 25730 | SEQ ID NO: 29736 |

FIGURE 50

| iPS:434497 | 21-225_76A4 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
|---|---|---|---|---|
| | | | SEQ ID NO: 25731 | SEQ ID NO: 29737 |
| | | AA | EIVLTQSPGTLYLSPGERATLSCRASQSVYSSYLAWYQQKPGQAPRLLIYGASSRATGIPDRFSGSGSGTDFALTISRLEPEDFAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25732 | SEQ ID NO: 29738 |
| iPS:434501 | 21-225_76G4 | NA | GAAATTGTGTTGACGCAGTCTCCAGGCACCCTGTCTTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25733 | SEQ ID NO: 29739 |

FIGURE 50

| | | | | |
|---|---|---|---|---|
| | | AA | EIVLTQSPGTLSLSPGERATLSCRASQSVYSSYLA WYQQKPGQAPRLLIYGASSRATGIPDRFSGSGSG TDFALTISRLEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25734 | SEQ ID NO: 29740 |
| iPS:434503 | 21-225_74D7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATTTGGGACAGAATTCACTCTCACCA TCAGCAGCCTGCAGCCTGAAGATTTTGCAACT TATTACTGTCTTCAGCATAGTAATTACCCGCTC ACTTTCGGCGGAGGGACCAAGGTGGAGATCA AA | CAACTGCAGCTGCAGGAGTCGGGCCCAGGACTG GTGAAGCCTTCGGAGACCCTGTCCCTCACCTGCA GTGTCTCTGGTGGCTCCATCTTCAGAAGTAGTTA CTACTGGGGCTGGATCCGCCAGCCCCCAGGGAA GGGGCTGGAGTGGATTGGGGGGTATCTATTATAGT GGGAGCACCTCCTACAACCCGTCCCTCAAGAGTC GAGTCACCATATCCGTAGACACGTCCGAGAACC AGTTCTCCCTGAAGCTGAGCTCTGTGACCGCCGC AGACACGGCTGTGTATTACTGTGCGAGACTGCGA CCTAACTGGGACTTTGACTACTGGGGCCAGGGAA CCCTGGTCACCGTCTCCTCA |
| | | | SEQ ID NO: 25735 | SEQ ID NO: 29741 |
| | | AA | DIQMTQSPSSLSASVGDRVTITCRASQGIRNDLG WYQQKPGKAPKRLIYAASSLQSGVPSRFSGSGF GTEFTLTISSLQPEDFATYYCLQHSNYPLTFGGG TKVEIK | QLQLQESGPGLVKPSETLSLTCSVSGGSIFRSSYYW GWIRQPPGKGLEWIGGIYYSGSTSYNPSLKSRVTIS VDTSENQFSLKLSSVTAADTAVYYCARLRPNWDF DYWGQGTLVTVSS |
| | | | SEQ ID NO: 25736 | SEQ ID NO: 29742 |

FIGURE 50

| iPS:434507 | 21-225_74C5 | NA | GAAATTGTGTTGACGCAGTCTCCAGGCACCCT GTCTTTGTCTCCAGGGGAAAGAGCCACCCTCT CTTGCAGGGCCAGTCAGAGTGTTAACAGCAAC TACTTAGCCTGGTACCAGCAGAAACCTGGCCA GGCTCCCAGGCTCCTCATCTATGGTGCATTCA GCAGGGCCACTGGCATCCCAGACAGGGTCAGT GGCAGTGGGTCTGGGACAGACTTCAATCTCAT CATCAGCAGACTGGAGCCTGAAGATTTTGCAG TGTATTACTGTCAGCAGTATGAAAGCTCACCG TGGACGTTCGGCCAAGGGACCAAGGTGGAAA TCAAA | CAGGTGCAGCTACAACAGTGGGGCGCAGGACTG TTGAAGCCTTCGGAGACCCTGTCCCTCACTTGCG CTGTCTATGGTGGGTCCTTCAGTGGTTGCTACTG GAGCTGGATCCGCCAGCCCCCAGGGAAGGGGCT GGAGTGGATTGGGGAAATCAATCATAGTGGATG CACCAACTTCAACCCGTCCCTCAAGAGTCGAGTC ACCATATCAGTTGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
|---|---|---|---|---|
| | | | SEQ ID NO: 25737 | SEQ ID NO: 29743 |
| | | AA | EIVLTQSPGTLSLSPGERATLSCRASQSVNSNYLA WYQQKPGQAPRLLIYGAFSRATGIPDRVSGSGS GTDFNLIISRLEPEDFAVYYCQQYESSPWTFGQG TKVEIK | QVQLQQWGAGLLKPSETLSLTCAVYGGSFSGCYW SWIRQPPGKGLEWIGEINHSGCTNFNPSLKSRVTISV DTSKNQFSLKLSSVTAADTAVYYCARDYGGMDV WGQGTTVTVSS |
| | | | SEQ ID NO: 25738 | SEQ ID NO: 29744 |
| iPS:434509 | 21-225_76F5 | NA | GTCATCGTGTTGACCCAGTCTCCAGACTCCCT GGCTGTGTCTCTGGGCGAGAGGGCCACCATCA ACTGCAAGTCCAGCCAGAGTGTATTACACAGC TCCAACAGTTACAACTACTTAGCTTGGTACCA GCAGAAACCAGGACAGTCTCCTAAGGTGCTCA TTTACTGGACATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTAGTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTATATGGAGCTGAGCAGCCTGAGATCTGA GGACACGGCCGTGTATTACTGTGCGAGTAGCAGT GGCTGGTACTGGTTCGACCCCTGGGGCCAGGGA ACCCTGGTCACCGTCTCCTCA |
| | | | SEQ ID NO: 25739 | SEQ ID NO: 29745 |

1940

FIGURE 50

| | | | | |
|---|---|---|---|---|
| | | AA | VIVLTQSPDSLAVSLGERATINCKSSQSVLHSSNS YNYLAWYQQKPGQSPKVLIYWTSTRESGVPDRF SGSGSGTDFTLTISSLQAEDVAVYYCQQYYSSPP TFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCASSSGW YWFDPWGQGTLVTVSS |
| | | | SEQ ID NO: 25740 | SEQ ID NO: 29746 |
| iPS:434511 | 21-225_74B11 | NA | GATATCGTGATGACCCAGTCTCCAGACTCCCT GACTGTGTCTCTGGGCGAGAGGGCCACCATCA ACTGCAAGTCCAGCCAGAGTATTTTATACAAC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCATTCTCACCATCAGCAGCCTGCAGG CTGAAGATGTGGCAGTTTACTACTGTCAGCAA TATTATAGCACTCCTCCTACTTTCGGCGGAGG GACCAAGGTGGAGATCAAA | CAGGTGCAGCTGGTGCAGTCTGGGGGCTGAGGTG AAGAAGCCTGGGGCCTCAGTGAGGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGTATAGCAGT GGCTGGTACTACTTTGACTACTGGGGCCAGGGAA CCCTGGTCACCGTCTCCTCA |
| | | | SEQ ID NO: 25741 | SEQ ID NO: 29747 |
| | | AA | DIVMTQSPDSLTVSLGERATINCKSSQSILYNSNN NNYLAWYQQKPGQPPKLLIYWASTRESGVPDRF SGSGSGTDFILTISSLQAEDVAVYYCQQYYSTPP TFGGGTKVEIK | QVQLVQSGAEVKKPGASVRVSCKASGYTFTNYDIN WVRQATGQGLEWMGWMHPNSGNTGYAQKFQGR VTMTRNTSISTAYMELSSLRSEDTAVYYCAYSSGW YYFDYWGQGTLVTVSS |
| | | | SEQ ID NO: 25742 | SEQ ID NO: 29748 |

FIGURE 50

| iPS:434513 | 21-225_76A6 | NA | GAAATTGTGTTGACGCAGTCTCCAGGCACCCG GTCTTGGTCTCCAGGGGAAAGAGCCACCCTCT CGTGCAGGGCCAGTCAGAGTGTTAGCAGCAGC TACTTAGCCTGGTACCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTAACTCACCG CTCACTTTCGGCGGAGGGACCAAGGTGGAGAT CAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAATGG TAACACAGGCTATGCACAGAAGTTCCAGGGCAG AGTCACCATGACCAGGAACACCTCCATAAGCAC AGCCTACATGGAGCTGAACAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTTA |
| | | | SEQ ID NO: 25743 | SEQ ID NO: 29749 |
| | | AA | EIVLTQSPGTRSWSPGERATLSCRASQSVSSSYL AWYQQKPGQAPRLLIYGASTRATGIPDRFSGSGS GTDFTLTISRLEPEYFAVYYCQQYGNSPLTFGGG TKVEIK | QVQLVQSGAEVKKPGASVKVSCKASGYTFTNYDIN WVRQATGQGLEWMGWMHPNNGNTGYAQKFQGR VTMTRNTSISTAYMELNSLRSEDTAVYYCAISSGW YWFDPWGQGTLVTVSL |
| | | | SEQ ID NO: 25744 | SEQ ID NO: 29750 |
| iPS:434515 | 21-225_74A5 | NA | GAATTTATGTTGACGCAGTCTCCAGGCACCCT GTCTTTGTCTCCAGGGGAAAGAGCCACCCTCT CGTGCAGGGCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
| | | | SEQ ID NO: 25745 | SEQ ID NO: 29751 |

1942

FIGURE 50

| | | | | |
|---|---|---|---|---|
| | | AA | EFMLTQSPGTLSLSPGERATLSCRASQSVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25746 | SEQ ID NO: 29752 |
| IPS:434517 | 21-225_76A7 | NA | GAAATTGCGTTGACGCAGTCTCCAGGCACCCT GTCTTTGTCTCCAGGGGAAAGAGCCACCCTCT CCTGCAGGGCCAGTCCGAGTGTTGACAGCAGC TACTTAGCCTGGTACCAGCAGAGACCTGGCCA GGCTCCCAGGCTCCTCATCTATGGTGCATCCA GCAGGGCCCCTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGAAAGTTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATCATAGTGGAAG GACCAACTACAACCCGTCCCTCAAGAGTCGAGTC ACCATATCAGTAGACACGTCCGAGAACCAGTTCT CCCTGAAGCTGAGCTCTGTGACCGCCGCGGACAC GGCTGTGTATTACTGTGCGAGAGACTACGGTGGG CTTGACTACTGGGGGCCAGGGAGCCCTGGTCACCG TCTCCTCA |
| | | | SEQ ID NO: 25747 | SEQ ID NO: 29753 |
| | | AA | EIALTQSPGTLSLSPGERATLSCRASPSVDSSYLA WYQQRPGQAPRLLIYGASSRAPGIPDRFSGSGSG TDFTLTISRLEPEDFAVYYCQQYESSPWTFGQGT KVEIK | QVQLQQWGAGLLKPSETLSLTCAVYGGSFSGCYW SWIRQPPGKGLEWIGEINHSGRTNYNPSLKSRVTIS VDTSENQFSLKLSSVTAADTAVYYCARDYGGLDY WGQGALVTVSS |
| | | | SEQ ID NO: 25748 | SEQ ID NO: 29754 |

FIGURE 50

| | | | | |
|---|---|---|---|---|
| iPS:434519 | 21-225_74C7 | NA | GAAATTGTGTTGACGCAGTCGCCAGGCACCCTGTCTTTGTCTCCAGGGGAGAGAGCCACCCTCTCCTGCAGGACCAGTCCGAATGTTGACAGCAGCTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTTTTACTGTCAGCAGTATGAACGCTCACCGTGGACGTTCGGCCAAGGGACCAAGGTGGAAATCAAA | CAGGTGCAGTTACAGCAGTGGGGCGCAGGACTGTTGAAGCCTTCGGAGACCCTGTCCCTCACCTGCGCTGTCTATGGTGGGTCCCTCAGTGGTTCCTACTGGAGCTGGATCCGCCAGCCCCCAGGGAAGGGGCTGGAGTGGATTGGGGAAATCAATCTTAGTGGAAGCACCAACTACAACCCGTCCCTCAAGAGTCGAGTCACCATATCAGTAGACACGTCCAAGAGCCAGTTCTCCCTGACGCTGCGCTCTGTGACCGCCGCGGACACGGCTGTGTATTACTGTGCGAGAGACTACGGTGGCCTTGACTACTGGGGCCAGGGAACCCTGGTCACCGTCTCCTCA |
| | | | SEQ ID NO: 25749 | SEQ ID NO: 29755 |
| | | AA | EIVLTQSPGTLSLSPGERATLSCRTSPNVDSSYLAWYQQKPGQAPRLLIYGASSRATGIPDRFSGSGSGTDFTLTISRLEPEDFAVFYCQQYERSPWTFGQGTKVEIK | QVQLQQWGAGLLKPSETLSLTCAVYGGSLSGSYWSWIRQPPGKGLEWIGEINLSGSTNYNPSLKSRVTISVDTSKSQFSLTLRSVTAADTAVYYCARDYGGLDYWGQGTLVTVSS |
| | | | SEQ ID NO: 25750 | SEQ ID NO: 29756 |
| iPS:434523 | 21-225_75C3 | NA | GAAATTGTGTTGACGCAGTCTCCAGGCACCCTGTCTTTGTCTCAAGGGGAAAGAGCCACCCTCTCCTGCAGGGCCAGTCAGAGTGTTAGCAGCAGGTACTTAGCCTGGTACCAGCAGAAACCTGGCCAGGCTCCCAGACTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTATATTACTGTCAGCATTATGATAGCTCACCGTGGACGTTCGGCCAAGGGACCAAGGTGGAAATCAAA | CAGGTGCAGCTACAGCAGGGGGGGCGCAGGACCGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATTATAGTGGAAGAACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 25751 | SEQ ID NO: 29757 |

FIGURE 50

| | | | | |
|---|---|---|---|---|
| | | AA | EIVLTQSPGTLSLSQGERATLSCRASQSVSSRYLA WYQQKPGQAPRLLIYGASSRATGIPDRFSGSGSG TDFTLTISRLEPEDFAVYYCQHYDSSPWTFGQGT KVEIK | QVQLQQGGAGPLKPSETLSLTCAVYGGSFSGCYWS WIRQPPGKGLEWIGEINYSGRTNYNPSLKSRVTISV DTSKNQFSLKLSSVTAADTAVYYCARDYGGMDV WGQGTTVTVSS |
| | | | SEQ ID NO: 25752 | SEQ ID NO: 29758 |
| iPS:434525 | 21-225_76E8 | NA | GACATCGTGATGACCCAGTCTCCAGACTCCCT GGCTGTGTCTCTGGGCGAGAGGGCCACCATCA ACTGCAAGTCCAGCCAGACTGTTTTACACAGC TCCAACAATTATAACTACTTAGCTTGGTACCA GCAGAAGCCAGGACAGCCCCCTAAGGTGCTC ATTTACTGGACATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTACTGTCAGCA ATATTTTAGTAGTCCTCTGACGTTCGGCCAAG GGACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCCCCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTATTGTGCGGTTTCCAG TGGCTGGCACTGGTTCGACCCCTGGGGGCCAGGGA ACCCTGGTCACCGTCGCCTCA |
| | | | SEQ ID NO: 25753 | SEQ ID NO: 29759 |
| | | AA | DIVMTQSPDSLAVSLGERATINCKSSQTVLHSSN NYNYLAWYQQKPGQPPKVLIYWTSTRESGVPD RFSGSGSGTDFTLTISSLQAEDVAVYYCQQYFSS PLTFGQGTKVEIK | QVQLVQSGAEVKKPGASVKVSCKASGYTFPNYDIN WVRQATGQGLEWMGWMNPNSGNTGYAQKFQGR VTMTRNTSISTAYMELSSLRSEDTAVYYCAVSSGW HWFDPWGQGTLVTVAS |
| | | | SEQ ID NO: 25754 | SEQ ID NO: 29760 |

FIGURE 50

| | | | | |
|---|---|---|---|---|
| iPS:434529 | 21-225_76B9 | NA | GAATTTATGTTGACGCAGTCTCCAGGCACCCTGTCTTGGTCTCCAGGGGAAAGAGCCACCCTCTCGTGCAGGGCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25755 | SEQ ID NO: 29761 |
| | | AA | EFMLTQSPGTLSWSPGERATLSCRASQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25756 | SEQ ID NO: 29762 |
| iPS:434531 | 21-225_76C9 | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGTTACTTATCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAGGTCACGGACGTTCGGCCAAGGGACCAAGGTGGAAATCAGA | GAGGTGCAGCTGGTGGAGTCTGGGGGAGGCTTGGTAAAGCCTGGGGGGTCCCTTAGACTCTCCTGTGCAGCCTCTGGATTCAGTTTCAGTAACGCCTGGATGAACTGGGTCCGCCAGGCTCCAGGGAAGGGGCTGGAGTGGGTTGGCCGTATTAAAAACAAAGCTGATGGTGGGACAACAGACTTCGCTGCACCCGTGAAAGGCAGATTCACCATCTCAAGAGATGATTCAAAACACACGCTGTATCTGCAAATGAACAGCCTGAAAACCGAGGACACAGCCGTGTATTACTGTACCACAGTGGGACCTACTACGGACTACTGGGGCCAGGGAACCCTGGTCACCGTCTCCTCA |
| | | | SEQ ID NO: 25757 | SEQ ID NO: 29763 |

FIGURE 50

| | | | | |
|---|---|---|---|---|
| | | AA | EIVLTQSPGTLSLSPGERATLSCRASQSVSSSYLS WYQQKPGQAPRLLIYGASSRATGIPDRFSGSGSG TDFTLTISRLEPEDFAVYYCQQYGRSRTFGQGTK VEIR | EVQLVESGGGLVKPGGSLRLSCAASGFSFSNAWM NWVRQAPGKGLEWVGRIKNKADGGTTDFAAPVK GRFTISRDDSKHTLYLQMNSLKTEDTAVYYCTTVG PTTDYWGQGTLVTVSS |
| | | | SEQ ID NO: 25758 | SEQ ID NO: 29764 |
| IPS:434533 | 21-225_85F7 | NA | GAACCTGTGTTGACGCAGTCTCCAGGCACCCT GTCTTTGTCTCCAGGGGAAAGAGCCACCCTCT CCTGCAGGGCCAGTCAGAATATTTACAGCAAC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTGCTGTCAGCAGTATGAAAGCTCACCG TGGACCTTCGGCCAAGGGACCAAGGTGGAAA TCAAA | CAAGTGCAGCTACAGCAGTGGGGCGCAGGACTG TTGAAGCCTTCGGAGCCCCTGTCCCTCACCTGCG CTGTCTATGGTGGGTCCTTCAGTGGTCCCTACTG GAGCTGGATCCGCCAGCCCCCCGGGAAGGGGCT GGAGTGGATTGGGGAAATCAATCATAGTGGAAG CACCAACTACAACCCGTCCCTCAAGAGTCGAGTC ACCATATCAGTAGACACGTCCAAGAACCAGTTCT CCCTGAAACTGACCTCTGTGACCGCCGCGGACAC GGCTGTGTATTACTGTGCGAGAGACTACGGGGGT TTGGACGTCTGGGGCCAAGGGACCACGGTCACC GTCTCCTCA |
| | | | SEQ ID NO: 25759 | SEQ ID NO: 29765 |
| | | AA | EPVLTQSPGTLSLSPGERATLSCRASQNIYSNYLA WYQQKPGQAPRLLIYGASSRATGIPDRFSGSGSG TDFTLTISRLEPEDFAVYCCQQYESSPWTFGQGT KVEIK | QVQLQQWGAGLLKPSEPLSLTCAVYGGSFSGPYW SWIRQPPGKGLEWIGEINHSGSTNYNPSLKSRVTISV DTSKNQFSLKLTSVTAADTAVYYCARDYGGLDVW GQGTTVTVSS |
| | | | SEQ ID NO: 25760 | SEQ ID NO: 29766 |

FIGURE 50

| | | | | |
|---|---|---|---|---|
| iPS:434535 | 21-225_74C8 | NA | GACATCCAGATGACCCAGTCTCCATCCTCACT GTCTGCATCAGTAGGAGACAGAGTCACCATCA CTTGTCGGGCGAGTCAGGGCATTGGCAAGTAT TTAGCCTGGTTTCAGCAGAAACCAGGGAAAGC CCCTAAGTCCCTGATCTATACTACATCCAATTT ACAAAGTGGGGCCCCATCAAAGTTCAGCGGC AGTGGATCTGGGACAGATTTCACTCTCACCAT CAGCAGCCTGCAGTATGAAGATTTTGCAACTT ATTACTGCCAACAGTACAGTAATTACCCGCTC ACTTTCGGCGGAGGGACCAAGGTGGAGATCA AT | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATGGCAG CTATGGTTATGACGGCCTTGACTACTGGGGCCAG GGAACCCTGGTCACCGTCTCCTCA |
| | | | SEQ ID NO: 25761 | SEQ ID NO: 29767 |
| | | AA | DIQMTQSPSSLSASVGDRVTITCRASQGIGKYLA WFQQKPGKAPKSLIYTTSNLQSGAPSKFSGSGSG TDFTLTISSLQYEDFATYYCQQYSNYPLTFGGGT KVEIN | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDGSYG YDGLDYWGQGTLVTVSS |
| | | | SEQ ID NO: 25762 | SEQ ID NO: 29768 |
| iPS:434537 | 21-225_74E11 | NA | GAAATAGTGATGACGCAGTCTCCAGCCACCCT GTCTGTGTCTCCAGGGGAAAGAGCCACCCTCT CCTGCAGGGCCAGTCTGAGTGTTGTCAACAGC TTAGCCTGGTACCAGCAGAAACCTGGCCAGGC TCCCAGGCTCCTCATCCATGGTGCATCCACCA GGGCCACTGGTATCCCAGCCAGGTTCAGTGGC AGTGGGTCTGGGACAGAGTTCACTCTCACCAT CAGCAGTCTGCAGTCTGAAGATTTTGCAATTT ATTACTGTCAGCAGTATAATGACTGGCCGTGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATTTGGTATGATGGAAGT AATAAATACTATGCAGACTCCGTGAAGGGCCGA TTCACCATCTCCAGAGACAATTCCAAGAACACGC TGTTTCTGCAAATGAACAGCCTGAGAGCCGAGG ACACGGCTGTGTATTACTGTGCGAGCGATCGCAA TATAGTGGGAGCTACTTACTTTGAGTCCTGGGGC CAGGGAACCCTGGTCACCGTCTCCTCA |
| | | | SEQ ID NO: 25763 | SEQ ID NO: 29769 |

FIGURE 50

| | | | | |
|---|---|---|---|---|
| | | AA | EIVMTQSPATLSVSPGERATLSCRASLSVVNSLA WYQQKPGQAPRLLIHGASTRATGIPARFSGSGSG TEFTLTISSLQSEDFAIYYCQQYNDWPCSFGQGT KLEIK | QVQLVESGGGVVQPGRSLRLSCAASGFTFSSYGMH WVRQAPGKGLEWVAVIWYDGSNKYYADSVKGRF TISRDNSKNTLFLQMNSLRAEDTAVYYCASDRNIV GATYFESWGQGTLVTVSS |
| | | | SEQ ID NO: 25764 | SEQ ID NO: 29770 |
| iPS:434539 | 21-225_74A2 | NA | GATATTGTGATGACTCAGTCTCCACTCTCCCTG CCCGTCACCCCTGGAGAGCCGGCCTCCATCTC CTGCAGGTCTAGTCAGAGCCTCCTGCATAGTA ATGGACACAACTATTTGGATTGGTACCTACAG AAGCCAGGGCGGTCTCCACAGCTCCTGATCTA TTTGGGTTCTAATCGGGCCTCCGGGGTCCCTG AGAGGTTCAGTGGCAGTGGATCAGGCACAGA TTTTACACTGAAAATCAGCAGAGTGGAGGCTG AGGATGTTGGGGTTTATTACTGCATGCAACCT CTACAAACTCCGTTCACTTTCGGCGGAGGGAC CAAGGTGGAGATCAAA | CAGGTGCAGGTACAGCAGTGGGGCGCAGGACTG TTGAAGCCTTCGGAGACCCTGTCCCTCACCTGCG CTGTCTATGGTGGGTCCTTCACTGATTACTACTGG AGCTGGATCCGCCAGCCCCCAGGGAAGGGGCTG GAGTGGATTGGGGAAATCAATCATAGTGGAGAC ACCAACTACAACCCGTCCCTCAAGAGTCGAGTCA CCATATCAGTAGACACGTCCAAGAACCAGTTCTC CCTGAAGCTGAGTTCTGTGACCGCCGCGGACACG GTGGAAGCTACCTCTACTACTACGGTATGGACGT CTGGGGCCAAGGGACCACGGTCACCGTCTCCTCA |
| | | | SEQ ID NO: 25765 | SEQ ID NO: 29771 |
| | | AA | DIVMTQSPLSLPVTPGEPASISCRSSQSLLHSNGH NYLDWYLQKPGRSPQLLIYLGSNRASGVPERFS GSGSGTDFTLKISRVEAEDVGVYYCMQPLQTPF TFGGGTKVEIK | QVQVQQWGAGLLKPSETLSLTCAVYGGSFTDYYW SWIRQPPGKGLEWIGEINHSGDTNYNPSLKSRVTIS VDTSKNQFSLKLSSVTAADTAVYYCAREFPYSGSY LYYYGMDVWGQGTTVTVSS |
| | | | SEQ ID NO: 25766 | SEQ ID NO: 29772 |

FIGURE 50

| iPS:434547 | 21-225_74H5 | NA | GAAATTGTGTTGACGCAGTCTCCAGGCACCCT GTCTTTGTCTCCAGGGGAGAGAGCCACCCTCT CCTGCAGGGCCAGTCAGAGTGTTAACAGCAAC TACTTAGCCTGGTACCGGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCATTCTCAT CATCAGCAGACTGGAGCCTGAGGATTTTGCAG TGTATTACTGTCAGCAGTATGAAAGCTCGCCG TGGACGTTCGGCCAAGGGACCAAGGTGGAAA TCAAA | CAGGTGCAGCTACAACAGTGGGGCGCAGGACTG TTGAAGCCTTCGGAGACCCTGTCCCTCACTTGCG CTGTCAATGGTGGGCCCTTCAGTGGTTGCTACTG GAGCTGGATCCGCCAGCCCCCAGGGAAGGGGCT GGAGTGGATTGGGGAAATCAATCATAGTGGAAG GACCAACTTCAACCCGTCCCTCAAGAGTCGAGTC ACCATATCAGTTGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
| | | | SEQ ID NO: 25767 | SEQ ID NO: 29773 |
| | | AA | EIVLTQSPGTLSLSPGERATLSCRASQSVNSNYLA WYRQKPGQAPRLLIYGASSRATGIPDRFSGSGSG TDFILIISRLEPEDFAVYYCQQYESSPWTFGQGTK VEIK | QVQLQQWGAGLLKPSETLSLTCAVNGGPFSGCYW SWIRQPPGKGLEWIGEINHSGRTNFNPSLKSRVTISV DTSKNQFSLKLSSVTAADTAVYYCARDYGGMDV WGQGTTVTVSS |
| | | | SEQ ID NO: 25768 | SEQ ID NO: 29774 |
| iPS:434549 | 21-225_76E11 | NA | GACATCGTGATGACCCAGTCTCCAGACTCCCT GGCTGTGTCTCTGGGCGAGGGGGCCACCATCA ACTGCAAGTCCCGCCAGAGTGTTTTACACAGC TCCAACAATTACAACTACTTAGCTTGGTACCA GCAGAAAGCAGGACAGCCTCCTAAACTGCTCA TTTACTGGGCTTCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAGA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GACTCACCATGACCAGGAACACCTCCATAAGCA CAGCCTACATGGAGCTGAGCAGCCTGAGATCTG AGGACACGGCCGTGTTTTACTGTGCATATAGCAG TGGCTGGTACTACTTTGACTACTGGGGCCAGGGA ACCCTGGTCACCGTCTCCTCA |
| | | | SEQ ID NO: 25769 | SEQ ID NO: 29775 |

FIGURE 50

| | | | | |
|---|---|---|---|---|
| | | AA | DIVMTQSPDSLAVSLGEGATINCKSRQSVLHSSN NYNYLAWYQQKAGQPPKLLIYWASTRESGVPD RFSGSGSGTDFTLTISSLQAEDVAVYYCQQYYST PPTFGQGTKVEIR | QVQLVQSGAEVKKPGASVKVSCKASGYTFTNYDIN WVRQATGQGLEWMGWMNPNSGNTGYAQKFQGR LTMTRNTSISTAYMELSSLRSEDTAVFYCAYSSGW YYFDYWGQGTLVTVSS |
| | | | SEQ ID NO: 25770 | SEQ ID NO: 29776 |
| iPS:434551 | 21-225_75C4 | NA | GACATCGTGATGACCCAGTCTCCAGACTCCCT GGCTGTGTCTCTGGGCGAGAGGGCCACCATCA ACTGTAAGTCCAGCCAGAGTATTTTATACAGC TCCAACAATAATAACTACTTAGCTTGGTTCCA GCAGAAACCAGGACAGCCTCCTAAGCTGCTCA TTTATTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATATTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCATCCTAACAGTGG TAACACAGGCTATGCACAGAAGTTCCAGGGCAG AGTCACCATGACCAGGAACACCTCCATAAGCAC AGCCTACATGGAACTGAGCAGCCTGAGATCTGA GGACACGGCCGTGTATTACTGTGCGAGTAGCAGT GGCTGGTACTACTTTGACTACTGGGGCCAGGGAA CCCTGGTCACCGTCTCCTCA |
| | | | SEQ ID NO: 25771 | SEQ ID NO: 29777 |
| | | AA | DIVMTQSPDSLAVSLGERATINCKSSQSILYSSNN NNYLAWFQQKPGQPPKLLIYWASTRESGVPDRF SGSGSGTDFTLTISSLQAEDVAVYYCQQYYITPP TFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCASSSGW YYFDYWGQGTLVTVSS |
| | | | SEQ ID NO: 25772 | SEQ ID NO: 29778 |

FIGURE 50

| iPS:434559 | 21-225_74D11 | NA | GAAATTGTGTTGACGCAGTCTCCAGGCACCCT GTCTTTGTCTCCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25773 | SEQ ID NO: 29779 |
| | | AA | EIVLTQSPGTLSLSPGERATLSCRASQSVYSSYLA WYQQKPGQAPRLLIYGASSRATGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPPGKGLEWIGEINHSGSTNYNPSLKSRVTISV DTSKNQFSLKLSSVTAADTAVYYCARDYGGMDV WGQGTTVTVSS |
| | | | SEQ ID NO: 25774 | SEQ ID NO: 29780 |
| iPS:434561 | 21-225_77G1 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25775 | SEQ ID NO: 29781 |

FIGURE 50

| | | | | |
|---|---|---|---|---|
| | | AA | EIVLTQSPGTLYLSPGERATLSCRASQSVYSSYLA WYQQKPGQAPRLLIYGASSRATGIPDRFSGSGSG TDFALTISRLEPEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25776 | SEQ ID NO: 29782 |
| iPS:434563 | 21-225_75D8 | NA | GATATTGTGATGACTCAGTCTCCACTCTCCCTG CCCGTCACCCCTGGAGAGCCGGCCTCCATCTC CTGCAGGTCTAGTCAGAGCCTCCTGCATAGTA GTGGATACAACTATTTGGATTGGTACCTGCAG AAGCCAGGGCAGTCTCCACAGCTCCTGATCTA TTTGGGTTCTAATCGGGCCTCCGGGGTCCCTG ACAGGTTCAGTGGCAGTGGATCAGGCTCAGAT TTTACACTGAAGATCAGCAGAGTGGAGGCTGA GGATGTTGGACTTTATTACTGCATGCAAGCTC TACACCCTCCTCTCACTTTCGGCGGAGGGACC AAGGTGGAGATCAAA | GAGGTGCAGCTGGTGGAGTCTGGGGGAGGCTTG GTACAGCCGGGGGGGTCCCTGAGACTCTCCTGTG CAGCCTCTGGATTCACCTTCAGTAACTACGACAT GCACTGGGTCCGCCAAGCTACAGGAAAAGGTCT GGAGTGGGTCTCAGCTATTGGTACTGCTGGTGAC ACATACTATCCAGGCTCCGTGAAGGGCCGATTCA CCATCTCCAGAGAAAATGCCAAGAACTCCTTGTA TCTTCAAATGAACAGCCTGAGAGCCGGGGACAC GGCTGTGTATTACTGTGCAAGAGTTCTTGACTAC GGTGACTCCTTGGGCTACTACTACTACGGTATGG ACGTCTGGGGCCAAGGGACCACGGTCACCGTCTC CTCA |
| | | | SEQ ID NO: 25777 | SEQ ID NO: 29783 |
| | | AA | DIVMTQSPLSLPVTPGEPASISCRSSQSLLHSSGY NYLDWYLQKPGQSPQLLIYLGSNRASGVPDRFS GSGSGSDFTLKISRVEAEDVGLYYCMQALHPPL TFGGGTKVEIK | EVQLVESGGGLVQPGGSLRLSCAASGFTFSNYDMH WVRQATGKGLEWVSAIGTAGDTYYPGSVKGRFTIS RENAKNSLYLQMNSLRAGDTAVYYCARVLDYGDS LGYYYYGMDVWGQGTTVTVSS |
| | | | SEQ ID NO: 25778 | SEQ ID NO: 29784 |

FIGURE 50

| iPS:434565 | 21-225_75B10 | NA | GAAATTGTGTTGACGCAGTCTCCAGGCACCCT ATCTTTGTCTCCAGGGGAAAGAGCCACCCTCT CCTGCAGGGCCAGTCCGAGTGTTAACAGCTAC TACTTAGCCTGGTACCAGCAGAAACCTGGCCA GGCTCCCAGGCTCCTCATCTATGGTGCAACCA GCAGGGCCACTGGCATCCCAGACAGGTTCAGT GGCAGTGGGTCTGGAACAGACTTCACTCTCAC CATCAGCAGACTGGAGCCTGAAGATTTTGCAG TTTATTTCTGTCAGCAGTATGAAGACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCAAATGCG ATGTCTATGGTGGGTCCTTCAGTGGTTACTACTG GAGCTGGATCCGCCAGCCCCCAGGGAAGGGGCT GGAGTGGATTGGGGAAATCAATCACAGTGGAAG CACCAACTACAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTGTATTACTGTGCGAGAGACTACGGTGGT TTGGACGTCTGGGGCCAAGGGACCACGGTCACC GTCTCCTCA |
| | | | SEQ ID NO: 25779 | SEQ ID NO: 29785 |
| | | AA | EIVLTQSPGTLSLSPGERATLSCRASPSVNSYYLA WYQQKPGQAPRLLIYGATSRATGIPDRFSGSGSG TDFTLTISRLEPEDFAVYFCQQYEDSPWTFGQGT KVEIK | QVQLQQWGAGLLKPSETLSLKCDVYGGSFSGYYW SWIRQPPGKGLEWIGEINHSGSTNYNPSLKSRVTISV DTSKNQFSLKLSSVTAADTAVYYCARDYGGLDVW GQGTTVTVSS |
| | | | SEQ ID NO: 25780 | SEQ ID NO: 29786 |
| iPS:434569 | 21-225_77H5 | NA | GAAATAGTGATGACGCAGTCTCCAGTCACCCT GTCTGTGTCTCCAGGGGAAAGAGCCACCCTCT CCTGCAGGGCCAGTCAGAGTGTTAGCAGCAGC TTAGCCTGGTACCAGCAGAAACCTGGCCTGGC TCCCAGGCTCCTCATCTATGGTGCATCCACCA GGGCCACTGGTATCCCAGCCAGGTTCAGTGGC AGTGGGTCTGGGACAGAGTTCTCTTTTACCAT CAGCAGCCTGCAGTCTGAAGATTTTGCAGTTT ATTTCTGTCAGCAGTATAATGACTGGCCGTGC AGTTTTGGCCAGGGCTCCAAGCTGGAGATCCA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAATTATGGCAT GCACTGGGTCCGCCAGGCTCCAGGCAAGGGGCT GGAGTGGGTGGCAGTTATATGGTATGATGGAAG AAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTCTATATTACTGTGCGAGAGATCGGA GTATATTGGGAGCTACTTTCTTTGACTACTGGGG CCAGGGAACCCTGGTCACCGTCTCCTCA |
| | | | SEQ ID NO: 25781 | SEQ ID NO: 29787 |

FIGURE 50

| | | | | |
|---|---|---|---|---|
| | | AA | EIVMTQSPVTLSVSPGERATLSCRASQSVSSSLA WYQQKPGLAPRLLIYGASTRATGIPARFSGSGSG TEFSFTISSLQSEDFAVYFCQQYNDWPCSFGQGS KLEIQ | QVQLVESGGGVVQPGRSLRLSCAASGFTFSNYGM HWVRQAPGKGLEWVAVIWYDGRNKYYADSVKG RFTISRDNSKNTLYLQMNSLRAEDTALYYCARDRSI LGATFFDYWGQGTLVTVSS |
| | | | SEQ ID NO: 25782 | SEQ ID NO: 29788 |
| IPS:434571 | 21-225_74D2 | NA | GAAATTGTGTTGACGCAGTCTCCAGGCACCCT GTCTTTGTCTCCAGGGGAAAGAGCCACCCTCT CCTGCAGGGCCAGTCAGAGTGTTGACAGCAAC TACTTAGCCTGGTACCAGCAGAAACCTGGCCA GGCTCCCAGGCTCCTCATCTATGGTGCATCCA GCAGGGCCCCTGGCATCCCAGACAGGTTCAGT GGCAGTGGGTCTGGGACAGACTTCACTCTCAC CATCAACAGACTGGAGCCTGAAGATTTTGCAG TGTATTTCTGTCAGCAGTATGAAAGCTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATCATAGTGGAAG GACCAACTACAACCCGTCCCTCAAGAGTCGAGTC ACCATCTCAGTAGACACGTCCGAGAACCAGTTCT CCCTGAAGTTGAGCTCTGTGACCGCCGCGGACAC GGCTGTGTATTACTGTGCGAGAGACTACGGTGGG CTTGACTACTGGGGGCCAGGGAACCCTGGTCACCG TCTCCTCA |
| | | | SEQ ID NO: 25783 | SEQ ID NO: 29789 |
| | | AA | EIVLTQSPGTLSLSPGERATLSCRASQSVDSNYLA WYQQKPGQAPRLLIYGASSRAPGIPDRFSGSGSG TDFTLTINRLEPEDFAVYFCQQYESSPWTFGQGT KVEIK | QVQLQQWGAGLLKPSETLSLTCAVYGGSFSGCYW SWIRQPPGKGLEWIGEINHSGRTNYNPSLKSRVTIS VDTSENQFSLKLSSVTAADTAVYYCARDYGGLDY WGQGTLVTVSS |
| | | | SEQ ID NO: 25784 | SEQ ID NO: 29790 |

FIGURE 50

| iPS:434573 | 21-225_77E6 | NA | GACATCCAGATGACCCAGTCTCCATCCTCACTGTCTGCATCGTAGGAGACAGAGTCACCATCACTTGTCGGGCGAGTCAGGGCATTAGCAAGTATTTAGCCTGGTTTCAGCAGAAACCAGGGAAAGCCCCTAAGTCCCTGATCTATGCTGCATCCAGTTTGCAAGGTGGGGCCCCATCAAAGTTCAGCGGCAGTGGATCTGGGACAGATTTCACTCTCACCATCAGCAGCCTGCAGCCTGAAGATTTTGCAACTTATTACTGCCAACAGTACAGTAATTACCCGCTCACTTTCGGCGGAGGGACCAGGGTGGAGATCAAA | CAGGTGCAGCTGGTGGAGTCTGGGGGAGGCGTGGTCCAGTCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATCAAAACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGATGGCAGCTATGGTTACGACGGCCTTGACTACTGGGGCCAGGGAACCCTGGTCACCGTCTCCTCA |
| | | | SEQ ID NO: 25785 | SEQ ID NO: 29791 |
| | | AA | DIQMTQSPSSLSASVGDRVTITCRASQGISKYLAWFQQKPGKAPKSLIYAASSLQGGAPSKFSGSGSGTDFTLTISSLQPEDFATYYCQQYSNYPLTFGGGTRVEIK | QVQLVESGGGVVQSGRSLRLSCAASGFTFSSYGMHWVRQAPGKGLEWVAVIWYDGSNQNYADSVKGRFTISRDNSKNTLFLQMNSLRAEDTAVYYCARDGSYGYDGLDYWGQGTLVTVSS |
| | | | SEQ ID NO: 25786 | SEQ ID NO: 29792 |
| iPS:434575 | 21-225_77C7 | NA | GACATCGTGATGACCCAGTCTCCAGACTCCCTGGCTGTGTCTCTGGGCGAGAGGGCCACCATCAACTGCAAGTCCAGCCAGACTGTTTTACACAGCTCCAACAATTATAACTACTTAGCTTGGTACCAGCAGAAGCCAGGACAGCCCCCTAAGGTGCTCCTTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTTTAGTAGTCCTCCGACGTTCGGCCAAGGGACCAGGGTGGAAATCAAA | CAGGTGCAGCTGGTGCAGTCTGGGGGCTGAGGTGAAGAAGCCTGGGGCCTCAGTGAAGGTCTCCTGCAAGGCTTCTGGATACACCTTCCCCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTATTGTGCGGTTTCCAGTGGCTGGCACTGGTTCGACCCCTGGGGCCAGGGAACCCTGGTCACCGTCGCCTCA |
| | | | SEQ ID NO: 25787 | SEQ ID NO: 29793 |

FIGURE 50

| | | | | |
|---|---|---|---|---|
| | | AA | DIVMTQSPDSLAVSLGERATINCKSSQTVLHSSN NYNYLAWYQQKPGQPPKVLLYWTSTRESGVPD RFSGSGSGTDFTLTISSLQAEDVAVYYCQQYFSS PPTFGQGTRVEIK | QVQLVQSGAEVKKPGASVKVSCKASGYTFPNYDIN WVRQATGQGLEWMGWMNPNSGNTGYAQKFQGR VTMTRNTSISTAYMELSSLRSEDTAVYYCAVSSGW HWFDPWGQGTLVTVAS |
| | | | SEQ ID NO: 25788 | SEQ ID NO: 29794 |
| IPS:434579 | 21-225_77F7 | NA | GAAATTGTGTTGACGCAGTCTCCAGGCACCCT GTATTTGTCTCCAGGGGAAAGAGCCACCCTGT CATGCAGGGCCAGTCAGAGTGTTTACAGCAGC TACTTAGCGTGGTACCAGCAGAAACCTGGCCA GGCTCCCAGGCTCATCATTTATGGTGCATCCA GCCGGGCCACTGGCATCCCAGACAGGTTCAGT GGCAGTGGGTGTGGGACAGACTTCGCTCTCAC CATCAGCAGAGTGGAGCATGAAGATTGTGCA GTGTATTACTGTCAGCACTATGATAACTCACC GTGGACGTTCGGCCAAGGGACCAAGGTGGAA ATCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25789 | SEQ ID NO: 29795 |
| | | AA | EIVLTQSPGTLYLSPGERATLSCRASQSVYSSYLA WYQQKPGQAPRLIIYGASSRATGIPDRFSGSGCG TDFALTISRVEHEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25790 | SEQ ID NO: 29796 |

FIGURE 50

| | | | | |
|---|---|---|---|---|
| iPS:434581 | 21-225_74B12 | NA | GAAATTGTGTTGACGCAGTCTCCAGGCACCCT GTATTTGTCTCCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25791 | SEQ ID NO: 29797 |
| | | AA | EIVLTQSPGTLYLSPGERATLSCRASQSVYSSYLA WYQQKPGQAPRLLIYGASSRSTGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25792 | SEQ ID NO: 29798 |
| iPS:434583 | 21-225_74B6 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAGCAGC TACTTAGCCTGGTACCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTAACTCACCG CTCACTTTCGGCGGAGGGACCAAGGTGGAGAT CAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAATGG TAACACAGGCTATGCACAGAAGTTCCAGGGCAG AGTCACCATGACCAGGAACACCTCCATAAGCAC AGCCTACATGGAGCTGAACAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTTA |
| | | | SEQ ID NO: 25793 | SEQ ID NO: 29799 |

FIGURE 50

| | | | | |
|---|---|---|---|---|
| | | AA | EIVLTQSPGTLSLSPGERATLSCRASQSVSSSYLA WYQQKPGQAPRLLIYGASTRATGIPDRFSGSGSG TDFTLTISRLEPEYFAVYYCQQYGNSPLTFGGGT KVEIK | QVQLVQSGAEVKKPGASVKVSCKASGYTFTNYDIN WVRQATGQGLEWMGWMHPNNGNTGYAQKFQGR VTMTRNTSISTAYMELNSLRSEDTAVYYCAISSGW YWFDPWGQGTLVTVSL |
| | | | SEQ ID NO: 25794 | SEQ ID NO: 29800 |
| IPS:434585 | 21-225_75A12 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAGCAGG TACTTAGCCTGGTACCAGCAGAAACCTGGCCA GGCTCCCAGACTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGCAGATTTTGCAG TATATTACTGTCAGCATTATGATAGCTCACCGT GGACGTTCGGCCAAGGGACCAAGGTGGAAAT CAAA | CAGGTGCAGCTACAGCAGGGGGGCGCAGGACCG TTGAAGCCTTCGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATTATAGTGGAAG AACCAACTACAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
| | | | SEQ ID NO: 25795 | SEQ ID NO: 29801 |
| | | AA | EIVLTQSPGTLSLSPGERATLSCRASQSVSSRYLA WYQQKPGQAPRLLIYGASSRATGIPDRFSGSGSG TDFTLTISRLEPADFAVYYCQHYDSSPWTFGQGT KVEIK | QVQLQQGGAGPLKPSETLSLTCAVYGGSFSGCYWS WIRQPPGKGLEWIGEINYSGRTNYNPSLKSRVTISV DTSKNQFSLKLSSVTAADTAVYYCARDYGGMDV WGQGTTVTVSS |
| | | | SEQ ID NO: 25796 | SEQ ID NO: 29802 |

FIGURE 50

| | | | | |
|---|---|---|---|---|
| iPS:434587 | 21-225_74G3 | NA | GAATTTATGTTGACGCAGTCTCCAGGCACCCT GTGTTGGTCTCCAGGGGAAAGAGCCACCCTCT CGTGCAGGGCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
| | | | SEQ ID NO: 25797 | SEQ ID NO: 29803 |
| | | AA | EFMLTQSPGTLCWSPGERATLSCRASQSVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25798 | SEQ ID NO: 29804 |
| iPS:434595 | 21-225_77A10 | NA | GAAATTGTGTTGACGCAGTCTCCAGGCACCCT GTCTTTGTCTCCAGGGGAGAGAGCCACCCTCT CCTGCAGGGCCAGTCAGAGTGTTCACAGCAGG TACTTAGCCTGGTACCAGCAGAAACCTGGCCA GGCTCCCAAACTCCTCATCTTTGGTGCATCCA GCAGGGCCACTGGCATCCCAGACAGGTTCAGT GGCAGTGGGTCTGGGACGGACTTCACTCTCAC CATCAGCAGACTGGAGCCTGAAGATTTTGCAG TATATTACTGTCAGCATTATGATAGCTCACCGT GGACGTTCGGCCAAGGGACCAAGGTGGAAAT CAAA | CAGGTGCAGCTACAACAGGGGGGGCGCAGGACCG TTGAAGCCTTCGGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATTATAGTGGAAG AACCAACTACAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
| | | | SEQ ID NO: 25799 | SEQ ID NO: 29805 |

FIGURE 50

| | | | | |
|---|---|---|---|---|
| | | AA | EIVLTQSPGTLSLSPGERATLSCRASQSVHSRYLA WYQQKPGQAPKLLIFGASSRATGIPDRFSGSGSG TDFTLTISRLEPEDFAVYYCQHYDSSPWTFGQGT KVEIK | QVQLQQGGAGPLKPSETLSLTCAVYGGSFSGCYWS WIRQPPGKGLEWIGEINYSGRTNYNPSLKSRVTISV DTSKNQFSLKLSSVTAADTAVYYCARDYGGMDV WGQGTTVTVSS |
| | | | SEQ ID NO: 25800 | SEQ ID NO: 29806 |
| iPS:434597 | 21-225_77C10 | NA | GACATCGTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCACT ATTATAATACTCCGTGGAAGTTTGTCCAAGGG ACCAAGGTGGAAATCACA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGGCCAGGGA ACCCTGGTCACCGTCTCCTCA |
| | | | SEQ ID NO: 25801 | SEQ ID NO: 29807 |
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQHYYNT PWKFVQGTKVEIT | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25802 | SEQ ID NO: 29808 |

FIGURE 50

| iPS:434603 | 21-225_77D11 | NA | GAATTTATGTTGACGCAGTCTCCAGGCACCCT GTATTGGTCTCCAGGGGAAAGAGCCACCATCT CGTGCAGGGCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
|---|---|---|---|---|
| | | | SEQ ID NO: 25803 | SEQ ID NO: 29809 |
| | | AA | EFMLTQSPGTLYWSPGERATISCRASQSVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25804 | SEQ ID NO: 29810 |
| iPS:434611 | 21-225_77C12 | NA | GAAATTGTGTTGACGCAGTCTCCAGGCACCCT GTCTTTGTCTCCAGGGGAAAGAGCCACCCTCT CCTGCAGGGCCAGGCAGAGTGTTGACAGCAGT TATTTAGCCTGGTACCAGCAGAAACGTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGGCTGGAGCCTGAAGATTTTGCAG TATATTACTGTCAGCAGTATGAAAGCTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCTATGGTGGGGCCTTCAGTGGTTCCTACTG GAGCTGGATCCGCCAGTCCCCAGGGAAGGGGCT GGAGTGGATTGGGGAAATCAATTATAGGGGAAG CACCCAACTACAACCCGTCCCTCAAGAGTCGAGTC ACCATATCAGTAGACACGTCCAAGAACCAGTTCT CCCTGAACCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25805 | SEQ ID NO: 29811 |

FIGURE 50

| | | | | |
|---|---|---|---|---|
| | | AA | EIVLTQSPGTLSLSPGERATLSCRARQSVDSSYLA WYQQKRGQAPRLLIYGASSRATGIPDRFSGSGSG TDFTLTISRLEPEDFAVYYCQQYESSPWTFGQGT KVEIK | QVQLQQWGAGLLKPSETLSLTCAVYGGAFSGSYW SWIRQSPGKGLEWIGEINYRGSTNYNPSLKSRVTIS VDTSKNQFSLNLSSVTAADTAVYYCARDYGGMDV WGQGTTVTVSS |
| | | | SEQ ID NO: 25806 | SEQ ID NO: 29812 |
| iPS:434613 | 21-225_77D12 | NA | GACATCGTGATGACCCAGTCTCCAGACTCCCT GGCTGTGTCTCTGGGCGCGAGGGCCACCATCA ATTGCAGGTCCAGCCAGAGTGTTTTATACAGC TCCAACAATTATAACTACTTAGCTTGGTACCA GCAGAAACCAGGACAGCCTCCTAAGCTGCTCA TTTACTGGGCATCTACCCGGGATTCCGGGGTC CCTGACCGATTCAGTGGCAGCGGGTCTGGGAC AGATTTCACTCTCACCATCAGCAGCCTGCAGG CTGAAGATGTGGCCGTTTATTACTGTCAGCAA TATTATACTACTCCGTGCAGTTTTGGCCAGGG GACCAAGCTGGAGATCAAG | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAACA CAGCCTACATGGAGCTGAGCAGCCTGAGATCTG AGGACACGGCCGTGTATTACTGTGCGATTAGCAG TGGCTGGTACTGGTTCGACCCCTGGGGCCAGGGA ACCCTGGTCACCGTCTCCTCA |
| | | | SEQ ID NO: 25807 | SEQ ID NO: 29813 |
| | | AA | DIVMTQSPDSLAVSLGARATINCRSSQSVLYSSN NYNYLAWYQQKPGQPPKLLIYWASTRDSGVPD RFSGSGSGTDFTLTISSLQAEDVAVYYCQQYYTT PCSFGQGTKLEIK | QVQLVQSGAEVKKPGASVKVSCKASGYTFTNYDIN WVRQATGQGLEWMGWMNPNSGNTGYAQKFQGR VTMTRNTSINTAYMELSSLRSEDTAVYYCAISSGW YWFDPWGQGTLVTVSS |
| | | | SEQ ID NO: 25808 | SEQ ID NO: 29814 |

FIGURE 50

| | | | | |
|---|---|---|---|---|
| iPS:434615 | 21-225_76C5 | NA | GACATCCAGATGACCCAGTCTCCATCCTCACT GTCTGCATCTGTAGGAGACAGAGTCACCATCA CTTGTCGGGCGAGTCAGGTCATTAGCAAGTAT TTAGCCTGGTTTCAGCAGAAACCAGGGAAAGC CCCTAAGTCCCTGATCTATGCTGCATCCAGTTT GCAAAGTGGGGCCCCATCAAAGTTCAGCGGC AGTGGATCTGGGACAGATTTCACTCTCACCAT CAGCAGCCTGCAGCCTGAAGATTTTGCAACTT ATTACTGCCAACAGTACAGTAATTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATGGCAG CTATGGTTACGACGGCCTTGACTACTGGGGCCAG GGAACCCTGGTCACCGTCTCCTCA |
| | | | SEQ ID NO: 25809 | SEQ ID NO: 29815 |
| | | AA | DIQMTQSPSSLSASVGDRVTITCRASQVISKYLA WFQQKPGKAPKSLIYAASSLQSGAPSKFSGSGSG TDFTLTISSLQPEDFATYYCQQYSNYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDGSYG YDGLDYWGQGTLVTVSS |
| | | | SEQ ID NO: 25810 | SEQ ID NO: 29816 |
| iPS:434617 | 21-225_74B8 | NA | GACAGCGTGATGACCCAGTCTCCAGACTCCCT GGCTGTGTCTCTGGGCGAGAGGGCCACCATCA ACTGCAAGTCCAGCCAGAGTGTTTTACACAGC TCTAATAAAAAGAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGTCTGCAGG CTGAAGATGTGGCAGTTTATTACTGTCAGCAA TATTATAGGACTCCGTGGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAATAGTGG TAACACAGGCTATGCACAGAAGTTCCAGGGCAG AGTCACCCTGACCAGGAACACCTCCATAAGCAC AGCCTACATGGAGCTGAGCAGCCTGAGATCTGA GGACACGGCCGTGTATTACTGTGCGGTTTCCAGT GGCTGGTACTGGTTCGACCCCTGGGGCCAGGGA ACCCTGGTCACCGTCTCCTCA |
| | | | SEQ ID NO: 25811 | SEQ ID NO: 29817 |

FIGURE 50

| | | | | |
|---|---|---|---|---|
| | | AA | DSVMTQSPDSLAVSLGERATINCKSSQSVLHSSN KKNYLAWYQQKPGQPPKLLIYWASTRESGVPD RFSGSGSGTDFTLTISSLQAEDVAVYYCQQYYRT PWTFGQGTKVEIK | QVQLVQSGAEVKKPGASVKVSCKASGYTFTNYDIN WVRQATGQGLEWMGWMHPNSGNTGYAQKFQGR VTLTRNTSISTAYMELSSLRSEDTAVYYCAVSSGW YWFDPWGQGTLVTVSS |
| | | | SEQ ID NO: 25812 | SEQ ID NO: 29818 |
| iPS:434619 | 21-225_78C1 | NA | GACATCGTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCACT ATTATAATACTCCGTGGAAGTTTGTCCAAGGG ACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGGCCAGGGA ACCCTGGTCACCGTCTCCTCA |
| | | | SEQ ID NO: 25813 | SEQ ID NO: 29819 |
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQHYYNT PWKFVQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25814 | SEQ ID NO: 29820 |

FIGURE 50

| iPS:434621 | 21-225_74D1 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGAAAT TTAGCCTGGTTCCAGCAGAAACCTGGCCAGGC TCCCAGGCTCCTCATCTATGGTGCATCCATCA GGGCCACTGGTATCCCAGCCAGGTTCAGTGGC AGTGGGTCTGGGACAGAGTTCACTCTCACCAT CTACAGCCTGCAGTCTGAAGATTTTGCAGTTT ATTACTGTCAGCAGTATAATAACTGGCCTCCG CTCACTTTCGGCGGAGGGACCAAGGTGGAGAT CAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACCATGCAGACTCCGTGAAGGGCCGA TTCACCATCTCCAGAGACAATTCCAAGAACACGC TGTATCTGCAAATGAACAGCCTGAGAGCCGAGG ACACGGCTATGTATTACTGTGCGAGAGATGAGGG GTTCGGGGAGTTCGACTACTACAACTACGGTATG GACGTCTGGGGCCAAGGGACCACGGTCACCGTC TCCTCA |
| | | | SEQ ID NO: 25815 | SEQ ID NO: 29821 |
| | | AA | EIVMTQSPATLSVSPGERATLSCRASQSVSRNLA WFQQKPGQAPRLLIYGASIRATGIPARFSGSGSG TEFTLTIYSLQSEDFAVYYCQQYNNWPPLTFGG GTKVEIK | QVQLVESGGGVVQPGRSLRLSCAASGFTFSSYGMH WVRQAPGKGLEWVAVIWYDGSNKYHADSVKGRF TISRDNSKNTLYLQMNSLRAEDTAMYYCARDEGF GEFDYYNYGMDVWGQGTTVTVSS |
| | | | SEQ ID NO: 25816 | SEQ ID NO: 29822 |
| iPS:434629 | 21-225_74C3 | NA | GAAATAGTGATGACGCAGTCTCCAGCCACCCT GTCTGTGTCTCCAGGGGAAAGAGCCACCCTCT CCTGCAGGGCCAGTCAGAGTGTTGCCAGCAGC TTAGCCTGGTACCAGCAGAAACCTGGCCAGGC TCCCAGGCTCCTCATCTTTGGTACATCCACCAG GGCCACTGGTATCCCAGCCAGGTTCAGTGGCA GTGGGTCTGGGACAGAGTTCACTCTCATAATC AGCAGCCTGCAGTCTGAAGATTTTGCAGTTTA TTACTGTCAGCAGTATAATGACTGGCCGTGCA GTTTTGGCCTGGGGACCAAGCTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTTTGGCAT GCACTGGGCCCGCCAGGCTCCAGGCAAGGGGCT GGAATGGGTGGCAGCTATTTGGTATGATGGAAGT AATAAATACTGTGCAGACTCCGTGAAGGGCCGA TTCACCATCTCCAGAGACAATTCCAAGAACACAC TGTCTCTGCAAATGAACAGCCTGAGAGCCGAGG ACTCGGCTGTGTATTACTGTGCGAGAGATCGGAG TATACTGGGAGCTGCTTTCTTTGACTACTGGGGC CAGGGAACCCTGGTCACCGTCTCCTCA |
| | | | SEQ ID NO: 25817 | SEQ ID NO: 29823 |

FIGURE 50

| | | | | |
|---|---|---|---|---|
| | | AA | EIVMTQSPATLSVSPGERATLSCRASQSVASSLA WYQQKPGQAPRLLIFGTSTRATGIPARFSGSGSG TEFTLIISSLQSEDFAVYYCQQYNDWPCSFGLGT KLEIK | QVQLVESGGGVVQPGRSLRLSCAASGFTFSSFGMH WARQAPGKGLEWVAAIWYDGSNKYCADSVKGRF TISRDNSKNTLSLQMNSLRAEDSAVYYCARDRSILG AAFFDYWGQGTLVTVSS |
| | | | SEQ ID NO: 25818 | SEQ ID NO: 29824 |
| iPS:434633 | 21-225_74G8 | NA | GAAATTGTGTTGACGCAGTCTCCAGGCACCCT GTCTTTGTCTCCAGGGGAAAGAGCCACCCTCT CCTGCAGGGCCAGTCAGAGTTTTAGCAGCGCC TACTTAGCCTGGTACCAGCAGAAACCTGGCCA GGCTCCCAGGCTCCTCATCTATGGTACTTCCA GCAGGGCCACTGGCATCCCAGACAGGTTCAGT GGCAGTGGGTCTGGGACAGATTTCACTCTCAC CATCGGCAGACTGGAGCCTGAAGATTTTGCAG TGTATTACTGTCAACAGTATGGTAACTCAAGG ACGTTCGGCCAAGGGACCAAGGTGGAAATCA AA | GAGGTGCAGCTGGTGGAGTCTGGGGGAGGCTTG GTAAAGCCTGGGGGGTCCCTTAGACTCTCCTGTG CAGCCTCTGGATTCACTTTCAGTAACGCCTGGAT GAACTGGGTCCGCCAGGCTCCAGGGAAGGGGCT GGAGTGGGTTGGCCGTATTAAAAACAAAGCTGA TGGTGGGACAACAGACTACGCTGCACCCGTGAA AGGCAGATTCACCATCTCAAGAGATGATTCAAA AAACACGCTGTATCTGCAAATGAACAGCCTGAA AACCGAGGACACAGCCGTGTATTACTGTACCACA GTGGGAGCTACTACGGACTACTGGGGCCAGGGA ACCCTGGTCACCGTCTCCTCA |
| | | | SEQ ID NO: 25819 | SEQ ID NO: 29825 |
| | | AA | EIVLTQSPGTLSLSPGERATLSCRASQSFSSAYLA WYQQKPGQAPRLLIYGTSSRATGIPDRFSGSGSG TDFTLTIGRLEPEDFAVYYCQQYGNSRTFGQGT KVEIK | EVQLVESGGGLVKPGGSLRLSCAASGFTFSNAWM NWVRQAPGKGLEWVGRIKNKADGGTTDYAAPVK GRFTISRDDSKNTLYLQMNSLKTEDTAVYYCTTVG ATTDYWGQGTLVTVSS |
| | | | SEQ ID NO: 25820 | SEQ ID NO: 29826 |

FIGURE 50

| iPS:434635 | 21-225_78E6 | NA | GACATCGTGATGACCCAGTCTCCAGACTCCCT GGCTGTGTCTCTGGGCGAGAGGGCCACCATCA ACTGCAAGTCCAGCCAGAGTGTTTTGTACAGC TCCAACAGTCACAACTACTTAGCTTGGTACCA GCAGAAACCAGGACAGCCTCCTAAACTGCTCA TTTACTGGGCATCTATCCGGGAATCCGGGGTC CCTGACCGATTCAGTGGCAGCGGGTCTGGGAC AGATTTCACTCTCTCCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGAC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACTCGGCCGTATATTACTGTGCGTATAGTAGT GGCTGGTACAAATTTGACTACTGGAGCCAGGGA ACCCTGGTCACCGTCTCCTCA |
| | | | SEQ ID NO: 25821 | SEQ ID NO: 29827 |
| | | AA | DIVMTQSPDSLAVSLGERATINCKSSQSVLYSSN SHNYLAWYQQKPGQPPKLLIYWASIRESGVPDR FSGSGSGTDFTLSISSLQAEDVAVYYCQQYYSTP CSFGQGTKLEIK | QVQLVQSGAEVKKPGASVKVSCKASGYTFTNYDIN WVRQASGQGLEWMGWMHPNSGNTGYAQKFQGR VTMTRNTSISTAYMELSSLRSEDSAVYYCAYSSGW YKFDYWSQGTLVTVSS |
| | | | SEQ ID NO: 25822 | SEQ ID NO: 29828 |
| iPS:434637 | 21-225_78E7 | NA | GAAATTGTGTTGACGCAGTCGCCAGGCACCCT GTCTTTGTCTCCAGGGGAGAGAGCCACCCTCT CCTGCAGGGCCAGTCAGAATGTTGACAGCAAC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTTTTACTGTCAGCAGTATGAACGCTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCTATGGTGGGTCCCTCAGTGGTTCCTACTG GAGCTGGATCCGCCAGCCCCCAGGGAAGGGGCT GGAGTGGATTGGGGAAATCAATCTTAGTGGAAG CACCAACTACAACCCGTCCCTCAAGAGTCGAGTC ACCATATCAGTAGACACGTCCAAGAGCCAGTTCT CCCTGACGCTGCGCTCTGTGACCGCCGCGGACAC GGCTGTGTATTACTGTGCGAGAGACTACGGTGGC CTTGACTACTGGGGCCAGGGAACCCTGGTCACCG TCTCCTCA |
| | | | SEQ ID NO: 25823 | SEQ ID NO: 29829 |

FIGURE 50

| | | | | |
|---|---|---|---|---|
| | | AA | EIVLTQSPGTLSLSPGERATLSCRASQNVDSNYL AWYQQKPGQAPRLLIYGASSRATGIPDRFSGSGS GTDFTLTISRLEPEDFAVFYCQQYERSPWTFGQG TKVEIK | QVQLQQWGAGLLKPSETLSLTCAVYGGSLSGSYW SWIRQPPGKGLEWIGEINLSGSTNYNPSLKSRVTISV DTSKSQFSLTLRSVTAADTAVYYCARDYGGLDYW GQGTLVTVSS |
| | | | SEQ ID NO: 25824 | SEQ ID NO: 29830 |
| iPS:434639 | 21-225_74B7 | NA | GACATCGTGATGACCCAGTCTCCAGACTCCCT GGCTGTGTCTCTGGGCGAGAGGGCCACCATCA ACTGCAAGTCCAGCCAGACTGTTTTACACAGC TCCAACAATTATAACTACTTAGCTTGGTACCA GCAGAAGCCAGGACAGCCCCCTAAGGTGCTC ATTTACTGGACATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTACTGTCAGCA ATATTTTAGTAGTCCTCCGACGTTCGGCCAAG GGACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCCCCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTATTGTGCGGTTTCCAG TGGCTGGCACTGGTTCGACCCCTGGGGCCAGGGA ACCCTGGTCACCGTCGCCTCA |
| | | | SEQ ID NO: 25825 | SEQ ID NO: 29831 |
| | | AA | DIVMTQSPDSLAVSLGERATINCKSSQTVLHSSN NYNYLAWYQQKPGQPPKVLIYWTSTRESGVPD RFSGSGSGTDFTLTISSLQAEDVAVYYCQQYFSS PPTFGQGTKVEIK | QVQLVQSGAEVKKPGASVKVSCKASGYTFPNYDIN WVRQATGQGLEWMGWMNPNSGNTGYAQKFQGR VTMTRNTSISTAYMELSSLRSEDTAVYYCAVSSGW HWFDPWGQGTLVTVAS |
| | | | SEQ ID NO: 25826 | SEQ ID NO: 29832 |

FIGURE 50

| | | | | |
|---|---|---|---|---|
| iPS:434649 | 21-225_78E11 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TTCAACAATTACAACTACTTAGCTTGGTACCA GCAGAAACCAGGACAGCCTCCTAAGAAGCTC ATTTACTGGGCATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTCCTGTCAGCA ATATTATAGTAGTCCTCCGACGTTCGGCCAAG GGACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTGTGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGTAGCA GTGGCTGGAACTTCTTTGACTACTGGGGCCAGGG AACCCTGGTCACCGTCTCCTCA |
| | | | SEQ ID NO: 25827 | SEQ ID NO: 29833 |
| | | AA | DIVMTQSPDSLAVSLGERATINCKSSQSVLYSFN NYNYLAWYQQKPGQPPKKLIYWASTRESGVPD RFSGSGSGTDFTLTISSLQAEDVAVYSCQQYYSS PPTFGQGTKVEIK | QVQLVQSGAEVKKPGASVKVSCKASGYTFTNYDIN WVRQATGQGLEWMGWMNPNSGNTGCAQKFQGR VTMTRNTSISTAYMELSSLRSEDTAVYYCASSSGW NFFDYWGQGTLVTVSS |
| | | | SEQ ID NO: 25828 | SEQ ID NO: 29834 |
| iPS:434653 | 21-225_74B5 | NA | GACATCGTTATGACCCAGTCTCCAGACTCCCT GGCTGTGTCTCTGGGCGAGAGGGCCACCATCA ACTGCAAGTCCAGCCAGAGTGTTTTATTCAGC TCCAACAATTATAACTACTTAGCTTGGTACCA GCAGAGACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGCTGATGTGGCAGTTTATTACTGTCAGCAA TATTATAGTTCTCCTCCGACGTTCGGCCAAGG GACCACGGTGCAAATCAAA | CAGGTGCAGTTGGTGCAGTCTGGGGCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGAACCCTAACAGTGG TAACACAGGCTATGCACAGAAGTTCCAGGGCAG AGTCACCATGACCAGGAACACCTCCACAAGCAC AGCCCACATGGAGCTGAGCAGCCTGAGATCTGA GGACACGGCCGTGTATTACTGTGCGGTCTCCAGT GGCTGGAACTGGTTCGACCCCTGGGGCCAGGGA ACCCTGGTCACCGTCTCCTCA |
| | | | SEQ ID NO: 25829 | SEQ ID NO: 29835 |

FIGURE 50

| | | | | |
|---|---|---|---|---|
| | | AA | DIVMTQSPDSLAVSLGERATINCKSSQSVLFSSN NYNYLAWYQQRPGQPPNLLIYWASTRESGVPD RFSGSGSGTDFTLTISSLQAADVAVYYCQQYSS PPTFGQGTTVQIK | QVQLVQSGAEVKKPGASVKVSCKASGYTFTNYDIN WVRQATGQGLEWMGWMNPNSGNTGYAQKFQGR VTMTRNTSTSTAHMELSSLRSEDTAVYYCAVSSGW NWFDPWGQGTLVTVSS |
| | | | SEQ ID NO: 25830 | SEQ ID NO: 29836 |
| iPS:434655 | 21-225_78H12 | NA | GACATCGTGATGACCCAGTCTCCAGACTCCCT GGCTGTGTCTCTGGGCGAGAGGGCCACCATCA ACTGCAAGTCCAGCCAGACTGTTTTACACAGC TTCAACAATTATAACTACTTAGCTTGGTACCA GCAGAAGCCAGGACAGCCCCCTAAGGTGCTC ATTTACTGGACATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTACTGTCAGCA ATATTTTAGTAGTCCTCCGACGTTCGGCCAAG GGACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCCCCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTATTGTGCGGTTTCCAG TGGCTGGCACTGGTTCGACCCCTGGGGCCAGGGA ACCCTGGTCACCGTCGCCTCA |
| | | | SEQ ID NO: 25831 | SEQ ID NO: 29837 |
| | | AA | DIVMTQSPDSLAVSLGERATINCKSSQTVLHSFN NYNYLAWYQQKPGQPPKVLIYWTSTRESGVPD RFSGSGSGTDFTLTISSLQAEDVAVYYCQQYFSS PPTFGQGTKVEIK | QVQLVQSGAEVKKPGASVKVSCKASGYTFPNYDIN WVRQATGQGLEWMGWMNPNSGNTGYAQKFQGR VTMTRNTSISTAYMELSSLRSEDTAVYYCAVSSGW HWFDPWGQGTLVTVAS |
| | | | SEQ ID NO: 25832 | SEQ ID NO: 29838 |

FIGURE 50

| iPS:434657 | 21-225_79G1 | NA | GAAATTGTGTTGACGCAGTCTCCAGGCACCCT GTATTTGTCTTCAGGGGAAAGAGCCACCCTCT CCTGCAGGGCCAGTCAGAGTGTTTACAGCAGC TACTTAGCCTGGTACCAGCAGAAACCTGCCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25833 | SEQ ID NO: 29839 |
| | | AA | EIVLTQSPGTLYLSSGERATLSCRASQSVYSSYLA WYQQKPAQAPRLLIYGASSRSTGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25834 | SEQ ID NO: 29840 |
| iPS:434663 | 21-225_79F3 | NA | GAAATTGTGTTGACGCAGTCTCCAGGCACCCT GTATTTGTCTCCAGGGGAAAGAGCCACCCTCT CCTGCAGGGCCAGTCAGAGTGTTTACAGCAGC TACTTAGCCTGGTACCAGCAGAAACCTGCCCA GGCTCCCAGGCTCCTCATCTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25835 | SEQ ID NO: 29841 |

FIGURE 50

| | | | | |
|---|---|---|---|---|
| | | AA | EIVLTQSPGTLYLSPGERATLSCRASQSVYSSYLA WYQQKPAQAPRLLIYGASSRSTGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25836 | SEQ ID NO: 29842 |
| iPS:434665 | 21-225_74G4 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGT TCCAACAATAATAACTACTTAGCTTGGTACCA GCAGAAGGCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CGGAAGATGTGGCAGTTTATTACTGTCAGCAA TATTATAGTATTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATG TCAACTGGGTGCGACAGGCCACTGGACAAGGGC TTGAATGGATGGGATGGATGCACCCTAACAGTGG TAACACAGGCTATGCACAGAAGTTCCAGGGCAG AGTCACCATGACCAGGAACACCTCCATACGCAC AGCCTACATGGAGCTGAGCAGCCTGAGATCTGA GGACACGGCCGTGTATTACTGTGCGAGTAGCAGT GGCTGGTACCATTTTGACTACTGGGGCCAGGGAA CCCTGGTCACCGTCTCCTCA |
| | | | SEQ ID NO: 25837 | SEQ ID NO: 29843 |
| | | AA | DIVMTQSPDSLAVSLGERATINCKSSQSVLYSSN NNNYLAWYQQKAGQPPKLLIYWASTRESGVPD RFSGSGSGTDFTLTISSLQAEDVAVYYCQQYYSI PPTFGQGTKVEIK | QVQLVQSGAEVKKPGASVKVSCKASGYTFTNYDV NWVRQATGQGLEWMGWMHPNSGNTGYAQKFQG RVTMTRNTSIRTAYMELSSLRSEDTAVYYCASSSG WYHFDYWGQGTLVTVSS |
| | | | SEQ ID NO: 25838 | SEQ ID NO: 29844 |

FIGURE 50

| iPS:434669 | 21-225_79F4 | NA | GACATCCAGATGACCCAGTCTCCATCCTCACT GTCTGCATCGTAGGAGACAGAGTCACCATCA CTTGTCGGGCGAGTCAGGGCATTAGCAAGTAT TTAGCCTGGTTTCAGCAGAAACCAGGGAAAGC CCCTAAGTCCCTGATCTATGCTGCATCCAGTTT GCAAGGTGGGGCCCCATCTAAGTTCAGCGGCA GTGGATCTGGGACAGATTTCACTCTCACCATC AGCAGCCTGCAGCCTGAAGATTTTGCAACTTA TTACTGCCAACAGTACAGTAATTACCCACTCA CTTTCGGCGGAGGGACCAGGGTGGAGATCAA A | CAGGTGCAGTTGGTGGAGTCTGGGGGAGGCGTG GTCCAGTCTGGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGGAAGT AATCAAAACTATGCAGACTCCGTGAAGGGCCGA TTCACCATCTCCAGAGACAATTCCAAGAACACGC TGTTTCTGCAAATGAACAGCCTGAGAGCCGAGG ACACGGCTGTGTACTACTGTGCGAGAGATGGCA GCTATGGTTATGACGGCCTTGACTACTGGGGCCA GGGAACCCTGGTCACCGTCTCCTCA |
| | | | SEQ ID NO: 25839 | SEQ ID NO: 29845 |
| | | AA | DIQMTQSPSSLSASVGDRVTITCRASQGISKYLA WFQQKPGKAPKSLIYAASSLQGGAPSKFSGSGS GTDFTLTISSLQPEDFATYYCQQYSNYPLTFGGG TRVEIK | QVQLVESGGGVVQSGRSLRLSCAASGFTFSNYGM HWVRQAPGKGLEWVAVIWYDGSNQNYADSVKGR FTISRDNSKNTLFLQMNSLRAEDTAVYYCARDGSY GYDGLDYWGQGTLVTVSS |
| | | | SEQ ID NO: 25840 | SEQ ID NO: 29846 |
| iPS:434671 | 21-225_74F4 | NA | GAAATTGTGTTGACGCAGTCTCCAGGCACCCT GTCTTTGTCTCCAGGGGAAAGAGCCACCCTCT CCTGCAGGGCCAGTCAGATTTTTAGCAGCAGC TACTTAGCCTGGTACCAGCAGAAACCTGGCCA GTCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAGCTCACGG ACGTTCGGCCAAGGGACCAAGGTGGAAATCA AA | GAGGTGCAGCTGGTGGAGTCTGGGGGAGGCTTG GTAAAGCCTGGGGGGTCCCTTAGACTCTCCTGTG CAGCCTCTGGATTCACTTTCAGTAACGCCTGGAT GAACTGGGTCCGCCAGGCTCCAGGGAAGGGGCT GGAGTGGGTTGGCCGAATTAAAAACAAAATTGA TGGTGGGACAACAGACTACGCTGCACCCGTGAA AGGCAGATTCACCATCTCAAGAGATGATTCAAA AAACACGCTGTATCTGCAAATGAACAGCCTGAA AACCGAGGACACAGCCGTGTATTACTGTACCACA GTGGGAGCTACTACGGACTACTGGGGCCAGGGA ACCCTGGTCACCGTCTCCTCA |
| | | | SEQ ID NO: 25841 | SEQ ID NO: 29847 |

FIGURE 50

| | | | | |
|---|---|---|---|---|
| | | AA | EIVLTQSPGTLSLSPGERATLSCRASQIFSSSYLA WYQQKPGQSPRLLIYGASSRATGIPDRFSGSGSG TDFTLTISRLEPEDFAVYYCQQYGSSRTFGQGTK VEIK | EVQLVESGGGLVKPGGSLRLSCAASGFTFSNAWM NWVRQAPGKGLEWVGRIKNKIDGGTTDYAAPVKG RFTISRDDSKNTLYLQMNSLKTEDTAVYYCTTVGA TTDYWGQGTLVTVSS |
| | | | SEQ ID NO: 25842 | SEQ ID NO: 29848 |
| iPS:434673 | 21-225_74E3 | NA | GAAATAGTGATGACGCAGTCTCCAGCCACCCT GTCTCTGTCTCCAGGGGAAAGAGCCACCCTCT CCTGCAGGGCCAGTCTGAGTGTTGTCAACAGC TTAGCCTGGTACCAGCAGAAACCTGGCCAGGC TCCCAGGCTCCTCATCTATGGTGCATCCACCA GGGCCACTGGTATCCCAGCCAGGTTCAGTGGC AGTGGGTCTGGGACAGAGTTCACTCTCACCAT CAGCAGCCTGCAGTCTGAAGATTTTGCAATTT ATTACTGTCAGCAGTATAATGACTGGCCGTGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTA CAGCGTCTGGATTCACCTTCAGTAGCTATGGCAT GCACTGGGTCCGCCAGGCTCCAGGCAAGGGGCT GGAGTGGGTGGCAGTTATTTGGTATGATGGAAGT AATAAATACTATGCAGACTCCGTGAAGGGCCGA TTCACCATCTCCAGAGACAATTCCAAGAACACGC TGTTTCTGCAAATGAACAGCCTGAGAGCCGAGG ACACGGCTGTGTATTACTGTGCGAGCGATCGCAA TATAGTGGGAGCTACTTACTTTGAGTCCTGGGGC CAGGGAACCCTGGTCACCGTCTCCTCA |
| | | | SEQ ID NO: 25843 | SEQ ID NO: 29849 |
| | | AA | EIVMTQSPATLSLSPGERATLSCRASLSVVNSLA WYQQKPGQAPRLLIYGASTRATGIPARFSGSGSG TEFTLTISSLQSEDFAIYYCQQYNDWPCSFGQGT KLEIK | QVQLVESGGGVVQPGRSLRLSCTASGFTFSSYGMH WVRQAPGKGLEWVAVIWYDGSNKYYADSVKGRF TISRDNSKNTLFLQMNSLRAEDTAVYYCASDRNIV GATYFESWGQGTLVTVSS |
| | | | SEQ ID NO: 25844 | SEQ ID NO: 29850 |

FIGURE 50

| iPS:434675 | 21-225_79G6 | NA | GACATCGTGATGACCCAGTCTCCAGACTGCCT GGCTGTGTCTCTGGGCGAGAGGGCCACCATCA GCTGCATGTCCAGCCAGAGTGTTTTACACAGC TTCAACAATAAGAACTACTTAACTTGGTACCA GCAGAAACCAGGACAGCCTCCTAAGCTGCTCA TTTACTGGGCATCTACTTGGGAATCCGGGGTC CCTGACCGATTCAGTGGCAGCGGGTCTGGGAC AGATTTCAGTCTCCCGATCGGCAGCCTGCAGG CTGAAGATGTGGCAGTTTATTACTGTCAGCAA TATTATAGTATTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATTCACCTTCACCAATTATGATA TCAACTGGGTGCGACAGGCCACTGGACAAGGGC TTGAGTGGATGGGATGGATGCACCCTAACAGTGG TAACACAGGCTTTGCACAGAAGTTCCAGGGCAG AGTCACCATGACCAGGAACACCTCCATAAGCAC AGCCTACATGGAACTGAGCAGCCTGAGATCTGA GGACACGGCCGTGTATTACTGTGCGGTTTCCAGT GGCTGGTACTGGTTCGACCCCTGGGGCCAGGGA ACCCTGGTCACCGTCTCCTCA |
|---|---|---|---|---|
| | | | SEQ ID NO: 25845 | SEQ ID NO: 29851 |
| | | AA | DIVMTQSPDCLAVSLGERATISCMSSQSVLHSFN NKNYLTWYQQKPGQPPKLLIYWASTWESGVPD RFSGSGSGTDFSLPIGSLQAEDVAVYYCQQYYSI PPTFGQGTKVEIK | QVQLVQSGAEVKKPGASVKVSCKASGFTFTNYDIN WVRQATGQGLEWMGWMHPNSGNTGFAQKFQGR VTMTRNTSISTAYMELSSLRSEDTAVYYCAVSSGW YWFDPWGQGTLVTVSS |
| | | | SEQ ID NO: 25846 | SEQ ID NO: 29852 |
| iPS:434679 | 21-225_79G7 | NA | GACATCGTGATGACCCAGTCTCCAGACTCCCT GGCTGTGTCTCTGGGCGAGAGGGCCACCATCA ACTGCAAGTCCAGCCAGAGTGTTTTGTACAGC TCCAACAGTCACAACTACTTAGCTTGGTACCA GCAGAAACCAGGACAGCCTCCTAAACTGCTCA TTTTCTGGGCATCTATCCGGGAATCCGGGGTC CCTGACCGATTCAGTGGCAGCGGGTCTGGGAC AGATTTCACTCTCTCCATCAGCAGCATGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGAC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACTCGGCCGTATATTACTGTGCGTATAGTAGT GGCTGGTACAAATTTGACTACTGGAGCCAGGGA ACCCTGGTCACCGTCTCCTCA |
| | | | SEQ ID NO: 25847 | SEQ ID NO: 29853 |

FIGURE 50

| | | | | |
|---|---|---|---|---|
| | | AA | DIVMTQSPDSLAVSLGERATINCKSSQSVLYSSN SHNYLAWYQQKPGQPPKLLIFWASIRESGVPDR FSGSGSGTDFTLSISSMQAEDVAVYYCQQYYSTP CSFGQGTKLEIK | QVQLVQSGAEVKKPGASVKVSCKASGYTFTNYDIN WVRQASGQGLEWMGWMHPNSGNTGYAQKFQGR VTMTRNTSISTAYMELSSLRSEDSAVYYCAYSSGW YKFDYWSQGTLVTVSS |
| | | | SEQ ID NO: 25848 | SEQ ID NO: 29854 |
| iPS:434685 | 21-225_79E9 | NA | GACATCGTGATGACCCAGTCTCCAGACTCCCT GGCTGTGTCTCTGGGCGAGAGGGCCACCATCA ACTGTAAGTCCAGCCAGAGTATTTTATACAGC TCCAACAATAATAACTACTTAGCTTGGTTCCA GCAGAAACCAGGACAGCCTCCTAAGCTGCTCA TTTATTGGGCATCTACCCGGGAATCCGGGGTC CCTGACCGATTCAGTGGCAGCGGGTGTGGGAC AGATTTCACTCTCACCATCAGCAGCCTGCAGG CTGAAGATGTGGCAGTTTATTACTGTCAGCAA TATTATATTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCATCCTAACAGTGG TAACACAGGCTATGCACAGAAGTTCCAGGGCAG AGTCACCATGACCAGGAACACCTCCATAAGCAC AGCCTACATGGAACTGAGCAGCCTGAGATCTGA GGACACGGCCGTGTATTACTGTGCGAGTAGCAGT GGCTGGTACTACTTTGACTACTGGGGCCAGGGAA CCCTGGTCACCGTCTCCTCA |
| | | | SEQ ID NO: 25849 | SEQ ID NO: 29855 |
| | | AA | DIVMTQSPDSLAVSLGERATINCKSSQSILYSSNN NNYLAWFQQKPGQPPKLLIYWASTRESGVPDRF SGSGCGTDFTLTISSLQAEDVAVYYCQQYYITPP TFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCASSSGW YYFDYWGQGTLVTVSS |
| | | | SEQ ID NO: 25850 | SEQ ID NO: 29856 |

FIGURE 50

| iPS:434687 | 21-225_75A5 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
|---|---|---|---|---|
| | | | SEQ ID NO: 25851 | SEQ ID NO: 29857 |
| | | AA | EIVLTQSPGTLYLSPGERATLSCRASQSVYSSYLA WYQQKPGQAPRLLIYGASSRATGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25852 | SEQ ID NO: 29858 |
| iPS:434689 | 21-225_79G10 | NA | GACATCGTTATGACCCAGTCTCCAGACTCCCT GGCTGTGTCTCTGGGCGAGAGGGCCACCATCA ATTGCAAGTCCAGCCAGAGTGTTTTATTCAGC TCCAACAATTATAATTACTTAGCTTGGTACCA GCAGAAACCAGGACAGCCTCATAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGCTGATGTGGCAGTTTATTACTGTCAGCAA TATCATAGTTCTCCTCTGACGTTCGGCCAAGG GACCACGGTGCAAATCAAA | CAGGTGCAGTTGGTGCAGTCTGGGGCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGAACCCTAACAGTGG TAAACACAGGCTATGCACAGAAGTTCCAGGGCAG AGTCACCATGACCAGGAACACCTCCACAAGCAC AGCCCACATGGAGCTGAGCAGCCTGAGATCTGA GGACACGGCCGTGTATTACTGTGCGGTCTCCAGT GGCTGGAACTGGTTCGACCCCTGGGGCCAGGGA ACCCTGGTCACCGTCTCCTCA |
| | | | SEQ ID NO: 25853 | SEQ ID NO: 29859 |

FIGURE 50

| | | | | |
|---|---|---|---|---|
| | | AA | DIVMTQSPDSLAVSLGERATINCKSSQSVLFSSN NYNYLAWYQQRPGQPHNLLIYWASTRESGVPD RFSGSGSGTDFTLTISSLQAADVAVYYCQQYHSS PLTFGQGTTVQIK | QVQLVQSGAEVKKPGASVKVSCKASGYTFTNYDIN WVRQATGQGLEWMGWMNPNSGNTGYAQKFQGR VTMTRNTSTSTAHMELSSLRSEDTAVYYCAVSSGW NWFDPWGQGTLVTVSS |
| | | | SEQ ID NO: 25854 | SEQ ID NO: 29860 |
| IPS:434691 | 21-225_75G7 | NA | GAAATTGTGTTGACGCAGTCTCCAGGCACCCT GTCTTTGTCTCCAGGGGAAAGAGCCACCCTCT CCTGCAGGGCCAGGCAGAGTGTTGACAGCAGT TATTTAGCCTGGTACCAGCAGAAACGTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGGCTGGAGCCTGAAGATTTTGCAG TATATTACTGTCAGCAGTATGAAAGCTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCA CTGTCTATGGTGGGGCCTTCAGTGGTTCCTACTG GAGCTGGATCCGCCAGTCCCCAGGGAAGGGGCT GGAGTGGATTGGGGAAATCAATTATAGGGGAAG CACCAACTACAACCCGTCCCTCAAGAGTCGAGTC ACCATATCAGTAGACACGTCCAAGAACCAGTTCT CCCTGAACCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25855 | SEQ ID NO: 29861 |
| | | AA | EIVLTQSPGTLSLSPGERATLSCRARQSVDSSYLA WYQQKRGQAPRLLIYGASSRATGIPDRFSGSGSG TDFTLTISRLEPEDFAVYYCQQYESSPWTFGQGT KVEIK | QVQLQQWGAGLLKPSETLSLTCTVYGGAFSGSYW SWIRQSPGKGLEWIGEINYRGSTNYNPSLKSRVTIS VDTSKNQFSLNLSSVTAADTAVYYCARDYGGMDV WGQGTTVTVSS |
| | | | SEQ ID NO: 25856 | SEQ ID NO: 29862 |

FIGURE 50

| iPS:434693 | 21-225_79F11 | NA | GAAATTGTGTTGACGCAGTCTCCAGGCACCCT GTCTTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25857 | SEQ ID NO: 29863 |
| | | AA | EIVLTQSPGTLSLSPGERATLSCRASQSVYSSYLA WYQQKPGQAPRLLIYGASSRATGIPDRFSGSGSG TDFALTISRLEPEDFAVYYCQHSDNSPWTFGQGT KVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25858 | SEQ ID NO: 29864 |
| iPS:434697 | 21-225_79F12 | NA | GACATCGTGATGACCCAGTCTCCAGACTCCCT GGCTGTGTCTCTGGGCGAGAGGGCCACCATCA ACTGCAAGTCCAGCCAGAGTATTTTATACAGC TCCAACAATTACAACTACTTAGCTTGGTACCA GCAGAAACCAGGACAGCCTCCTAAGTTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGGACGTTCGGCCAAGG GACCAAGGTGGAAATCAAA | CAGGTGCAGCTGGTGCAGTCTGGGGCTGAGGTG AAGAAGCCTGGGGCCTCTGTGAAGGTCTCCTGCA AGGCTTCTGGATACACCTTCACCAATTATGATAT CAACTGGGTGCGACAGGCCACTGGACAAGGGCT TGAGTGGATGGGATGGATGAACCCTAACAGTGG TAACACAGGCTATGCACAGAAGTTCCAGGGCAG AGTCACCATGACCAGAAACACCTCCATAAGCAC AGCCTACATGGAGCTGAGCAGCCTGAGATCTGA GGACACGGCCGTATATTACTGTGCGAGTAGCAGT GGCTGGTACTTCTTTGACTACTGGGGCCAGGGAA CCCTGGTCACCCTCTCCTCA |
| | | | SEQ ID NO: 25859 | SEQ ID NO: 29865 |

FIGURE 50

| | | | | |
|---|---|---|---|---|
| | | AA | DIVMTQSPDSLAVSLGERATINCKSSQSILYSSNN YNYLAWYQQKPGQPPKLLIYWASTRESGVPDRF SGSGSGTDFTLTISSLQAEDVAVYYCQQYYSTP WTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCASSSGW YFFDYWGQGTLVTLSS |
| | | | SEQ ID NO: 25860 | SEQ ID NO: 29866 |
| IPS:434699 | 21-225_79G12 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25861 | SEQ ID NO: 29867 |
| | | AA | EIVLTQSPGTLYLSPGERATLSCRASQSVYSSYLA WYQQKPGQAPRLLIYGASSRSTGIPDRFSGSGSG TDFALTISRLEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25862 | SEQ ID NO: 29868 |

FIGURE 50

| iPS:434701 | 21-225_80A1 | NA | GAAATTGTGTTGACGCAGTCTCCAGGCACCCG GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTGTGGGACAGACTTCGCTCTCAC CATCAGCAGAGTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
|---|---|---|---|---|
| | | | SEQ ID NO: 25863 | SEQ ID NO: 29869 |
| | | AA | EIVLTQSPGTRYLSPGERATLSCRASQSVYSSYL AWYQQKPGQAPRLLIYGASSRSTGIPDRFSGSGC GTDFALTISRVEPEDCAVYYCQHSDNSPWTFGQ GTKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25864 | SEQ ID NO: 29870 |
| iPS:434703 | 21-225_80C1 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGAGTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25865 | SEQ ID NO: 29871 |

FIGURE 50

| | | | | |
|---|---|---|---|---|
| | | AA | EIVLTQSPGTLYLSPGERATLSCRASQSVYSSYLA WYQQKPGQAPRLLIYGASSRSTGIPDRFSGSGSG TDFALTISRVEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25866 | SEQ ID NO: 29872 |
| iPS:434705 | 21-225_80A2 | NA | GAATTTATGTTGACGCAGTCTCCAGGCACCCT GTATTTGTCTCCAGGGGAAAGAGCCACCCTCT CGTGCAGGGCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
| | | | SEQ ID NO: 25867 | SEQ ID NO: 29873 |
| | | AA | EFMLTQSPGTLYLSPGERATLSCRASQSVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25868 | SEQ ID NO: 29874 |

FIGURE 50

| iPS:434707 | 21-225_80D3 | NA | GACATCGTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCACT ACAATGAAACTCCAGGGAAGTTTGTCCAAGTG ACCAAGGTGGAAATCACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTCA |
| | | | SEQ ID NO: 25869 | SEQ ID NO: 29875 |
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQHYNET PGKFVQVTKVEIT | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25870 | SEQ ID NO: 29876 |
| iPS:434709 | 21-225_80E3 | NA | GAAATTGTGTTGACGCAGTCTCCAGGCACCCT GTATTTGTCTTCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTGTGGGACAGACTTCGCTCTCAC CATCAGCAGAGTGGAGCCTGAAGATTTTGCAG TGTATTACTGTCAGCATTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25871 | SEQ ID NO: 29877 |

FIGURE 50

| | | | | |
|---|---|---|---|---|
| | | AA | EIVLTQSPGTLYLSSGERATLSCRASQSVYSSYLA WYQQKPGQAPRLLIYGASSRSTGIPDRFSGSGCG TDFALTISRVEPEDFAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25872 | SEQ ID NO: 29878 |
| iPS:434711 | 21-225_80H3 | NA | GACATCGTGATGACCCAGTCTCCAGACTCCCT GGCTGTGTCTCTGGGCGAGAGGGCCACCACCA ATTGTAAGTCCAGCCAGAGTGTTTTACACAGG TCCAACAATTACAACTACTTAGCGTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTGACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAACTGAGCAGCCTGAGATCTG AGGACACGGCCGTGTATTACTGTGGGAGTACCA GTGGCTGGAACTTCTTTGACTACTGGGGCCAGGG AACCCTGGTCACCGTCTCCTCA |
| | | | SEQ ID NO: 25873 | SEQ ID NO: 29879 |
| | | AA | DIVMTQSPDSLAVSLGERATTNCKSSQSVLHRSN NYNYLAWYQQKPGQPPKLLIYWASTRESGVPD RFSGSGSGTDFTLTISSLQAEDVAVYYCQQYYST PPTFGQGTKVEIK | QVQLVQSGAEVKKPGASVKVSCKASGYTLTNYDI NWVRQATGQGLEWMGWMNPNSGNTGYAQKFQG RVTMTRNTSISTAYMELSSLRSEDTAVYYCGSTSG WNFFDYWGQGTLVTVSS |
| | | | SEQ ID NO: 25874 | SEQ ID NO: 29880 |

FIGURE 50

| iPS:434715 | 21-225_80D5 | NA | GAACTTGTGTTGACGCAGTCTCCAGGCACCCTGTCTTTGTCTCCAGGGAAAAGAGTCACCCTCTCCTGCAGGGCCAGTCAGAATATTTACAGCAGCTACTTAGCCTGGTACCAGCAGAAACCTGGCCAGGCTCCCAGACTCCTCATCTATGGTGCATCCAGCAGGGCCACTGGCATCCCAGACAGGTTCAGTGGCAGTGGGTCTGGGACAGACTTCACTCTCACCATCACCAGACTGGAGCCTGAAGATTTTGCAGTGTATTGCTGTCAGCAGTATGAAAGCTCACCGTGGACCTTCGGCCAAGGGACCAAGGTGGAAATCAAA | CAAGTGCAGCTACAGCAGTGGGGCGCAGGACTGTTGAAGCCTTCGGAGCCCCTGTCCCTCACCTGCGCTGTCTATGGTGGGTCCTTCAGTGGTCCCTACTGGAGCTGGATCCGCCAGCCCCCCGGGAAGGGGCTGGAGTGGATTGGGGAAATCAATCATAGTGGAAGCACCAACTACAACCCGTCCCTCAAGAGTCGAGTCACCATATCAGTAGACACGTCCAAGAACCAGTTCTCCCTGAAACTGACCTCTGTGACCGCCGCGGACATGGCTGTGTATTACTGTGCGAGAGACTACGGGGGTTTGGACGTCTGGGGGCCAAGGGACCACGGTCACCGTCTCCTCA |
| | | | SEQ ID NO: 25875 | SEQ ID NO: 29881 |
| | | AA | ELVLTQSPGTLSLSPGKRVTLSCRASQNIYSSYLAWYQQKPGQAPRLLIYGASSRATGIPDRFSGSGSGTDFTLTITRLEPEDFAVYCCQQYESSPWTFGQGTKVEIK | QVQLQQWGAGLLKPSEPLSLTCAVYGGSFSGPYWSWIRQPPGKGLEWIGEINHSGSTNYNPSLKSRVTISVDTSKNQFSLKLTSVTAADMAVYYCARDYGGLDVWGQGTTVTVSS |
| | | | SEQ ID NO: 25876 | SEQ ID NO: 29882 |
| iPS:434717 | 21-225_80A6 | NA | GAAATTGTGTTGACGCAGTCTCCAGGCACCCTGTCTTTGTCTCCAGGGGAAATACCCACCCTCTCCTGCAGGGCCAGTCAGAGTGTTGACAGCGGCTACTTAGCCTGGTACCAGCAGAAACCTGGCCAGGCTCCCAGGCTCCTCATCTATGGTGCATCCAGCAGGGCCCCTGGCATCCCAGACAGGTTCAGTGGCAGTGGGTCTGGGACAGACTTCACTCTCACCATCAGCAGACTGGAACCTGAAGATTTTGCAGTGTATTACTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTGCTACTGGAGCTGGATCCGCCAGCCCCCAGGAAAGGGGCTGGAGTGGATTGGGGAAATCAATCATAGTGGAAGGACCAACTACAACCCGTCCCTCAAGAGTCGAGTCACCATATCAGTAGACACGTCCGAGAAGCAGTTCTCCCTGAAACTGAGCTCTGTGACCGCCGCGGACACGGCTGTGTATTACTGTGCGAGAGACTACGGTGGGCTTGACTACTGGGGCCAGGGAACCCTGGTCACCGTCTCCTCA |
| | | | SEQ ID NO: 25877 | SEQ ID NO: 29883 |

FIGURE 50

| | | | | |
|---|---|---|---|---|
| | | AA | EIVLTQSPGTLSLSPGEIPTLSCRASQSVDSGYLA WYQQKPGQAPRLLIYGASSRAPGIPDRFSGSGSG TDFTLTISRLEPEDFAVYYCQQYESSPWTFGQGT KVEIK | QVQLQQWGAGLLKPSETLSLTCAVYGGSFSGCYW SWIRQPPGKGLEWIGEINHSGRTNYNPSLKSRVTIS VDTSEKQFSLKLSSVTAADTAVYYCARDYGGLDY WGQGTLVTVSS |
| | | | SEQ ID NO: 25878 | SEQ ID NO: 29884 |
| IPS:434725 | 21-225_80H7 | NA | GAAATTGTGTTGACGCAGTCTCCAGGCACCCT GTCTTTGTCTCCAGGGGAAAGAGCCACCCTCT CCTGCAGGGCCAGTCAGAGTATTAACAGCAAC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTTTTACTGTCAGCAGTATGAGAGCTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGTTGGGTCCTTCAGTGGTTCCTACTGG AGCTGGATCCGCCAGCCCCCAGGGAAGGGGCTA GAGTGGATTGGGGAAATCAATCAAAGTGGACGC ACCAACTACAACCCGTCCCTCAAGAGTCGAGTCA CCATATCAGTAGACACGTCCAAGAACCAGTTCTC CCTGAAGCTGAGCTCTGTGACCGCCGCGGACACG GCTGTGTATTACTGTGCGAGAGACTACGGGGGTA TAGACGTCTGGGGCCAAGGGACCACGGTCACCG TCTCCTCA |
| | | | SEQ ID NO: 25879 | SEQ ID NO: 29885 |
| | | AA | EIVLTQSPGTLSLSPGERATLSCRASQSINSNYLA WYQQKPGQAPRLLIYGASSRATGIPDRFSGSGSG TDFTLTISRLEPEDFAVFYCQQYESSPWTFGQGT KVEIK | QVQLQQWGAGLLKPSETLSLTCAVYVGSFSGSYW SWIRQPPGKGLEWIGEINQSGRTNYNPSLKSRVTIS VDTSKNQFSLKLSSVTAADTAVYYCARDYGGIDV WGQGTTVTVSS |
| | | | SEQ ID NO: 25880 | SEQ ID NO: 29886 |

FIGURE 50

| iPS:434729 | 21-225_80B12 | NA | GACATCGTGTTGACCCAGTCGCCAGACTCCCT GGCTGTGTCTCTGGGCGAGAGGGCCACCATCA ACTGCAAGTCCAGACAGAGTGTTTTATACAGC TCCAACAATTACAACTACTTAACTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGACGTGGCAGTTTATTACTGTCAGCAA TATTATAGTTCTCCTCCTACTTTCGGCGGAGGG ACCAAGGTGGAGATCAAA | CAGGTGCAGCTGGTGCAGTCTGGGGCTGAGGTG AAGAAGCCTGGGGCCTCAGTGAAGGTCTCCTGC AAGACTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGTCA GAGTCACCATGACCAGGAACACCTCCATAAGCA CAGCCTACATGGAGCTGAGCAGCCTGAGATCTG AGGACACGGCCGTCTATTACTGTGCGTATAGCAG TGGCTGGTACATCTTTGACTACTGGGGCCAGGGA ACCCTGGTCACCGTCTCCTCA |
|  |  |  | SEQ ID NO: 25881 | SEQ ID NO: 29887 |
|  |  | AA | DIVLTQSPDSLAVSLGERATINCKSRQSVLYSSN NYNYLTWYQQKPGQPPKLLIYWASTRESGVPDR FSGSGSGTDFTLTISSLQAEDVAVYYCQQYYSSP PTFGGGTKVEIK | QVQLVQSGAEVKKPGASVKVSCKTSGYTFTNYDIN WVRQATGQGLEWMGWMNPNSGNTGYAQKFQVR VTMTRNTSISTAYMELSSLRSEDTAVYYCAYSSGW YIFDYWGQGTLVTVSS |
|  |  |  | SEQ ID NO: 25882 | SEQ ID NO: 29888 |
| iPS:434731 | 21-225_80E9 | NA | GACATCGTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCAA TATTATAATACTCCGTGGACGTTCGTCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTCA |
|  |  |  | SEQ ID NO: 25883 | SEQ ID NO: 29889 |

FIGURE 50

| | | | | |
|---|---|---|---|---|
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQQYYNT PWTFVQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25884 | SEQ ID NO: 29890 |
| IPS:434735 | 21-225_80B10 | NA | GAAATTGTGTTGACGCAGTCTCCAGGCACCCT GTCTTTGTCTCCAGGGGAAAGAGCCACCCTCT CCTGCAGGGCCAGTCAGAGTGTTGACAGCAGC TACTTAGCCTGGTACCAGCAGAAACCTGGCCA GGCTCCCAGGCTCCTCATCTATGGTGCATCCA GCAGGGCCCCTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGAAAGCTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTGTATTACTGTGCGAGAGACTACGGTGGG CTTGACTACTGGGGGCCAGGGAACCCTGGTCACCG TCTCCTCA |
| | | | SEQ ID NO: 25885 | SEQ ID NO: 29891 |
| | | AA | EIVLTQSPGTLSLSPGERATLSCRASQSVDSSYLA WYQQKPGQAPRLLIYGASSRAPGIPDRFSGSGSG TDFTLTISRLEPEDFAVYYCQQYESSPWTFGQGT KVEIK | QVQLQQWGAGLLKPSETLSLTCAVYGGSFSGCYW SWIRQPPGKGLEWIGEINHSGSTNYNPSLKSRVTISV DTSKNQFSLKLSSVTAADTAVYYCARDYGGLDYW GQGTLVTVSS |
| | | | SEQ ID NO: 25886 | SEQ ID NO: 29892 |

FIGURE 50

| iPS:434737 | 21-225_74G6 | NA | GACATCCAGATGACCCAGTCTCCATCCTCACT GTCTGCATCAGTAGGAGACAGAGTCACCATCA CTTGTCGGGCGAGTCAGGGCATTGGCAAGTAT TTAGCCTGGTTTCAGCAGAAACCAGGGAAAGC CCCTAAGTCCCTGATCTATACTACATCCAGTTT GCAAAGTGGGGCCCCATCAAAGTTCAGCGGC AGTGGATCTGGGACAGATTTCACTCTCACCAT CAGCAGCCTGCAGTATGAAGATTTTGCAACTT ATTACTGCCAACAGTACAGTAATTACCCGCTC ACTTTCGGCGGAGGGACCAAGGTGGAGATCA AT | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATGGCAG CTATGGTTATGACGGCCTTGACTACTGGGGCCAG GGAACCCTGGTCACCGTCTCCTCA |
| | | | SEQ ID NO: 25887 | SEQ ID NO: 29893 |
| | | AA | DIQMTQSPSSLSASVGDRVTITCRASQGIGKYLA WFQQKPGKAPKSLIYTTSSLQSGAPSKFSGSGSG TDFTLTISSLQYEDFATYYCQQYSNYPLTFGGGT KVEIN | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDGSYG YDGLDYWGQGTLVTVSS |
| | | | SEQ ID NO: 25888 | SEQ ID NO: 29894 |
| iPS:434741 | 21-225_80C11 | NA | GACATCCAGATGACCCAGTCTCCATCCTCACT GTCTGCTTCTGTAGGGGACAGAGTCACCATCA CTTGTCGGGCGAGTCAGGGCATTGGCAGGTAT TTAGCCTGGTTTCAGCAGAAACCAGGGAGAGC CCCTAAGTCCCTGATCTATACTGCATCCAGTTT GCAAAGTGGGGCCCCATCAAAGTTCAGCGGC AGTGGATCTGGGACAGATTTCACTCTCACCAT CAGCAGCCTGCAGCCTGAAGATTTTGCAACTT ATTACTGCCAACAGTACAGTAATTACCCGCTC ACTTTCGGCGGAGGGACCAAAGTGGAGATCA AT | CAGGTGCAGCTGGTGGAGTCTGGGGGAGGCGTG GTCCAGCCTGGGAGGTCCCTGAGACTCTCCTGTG CAGCGTCTGGATTCACCTTCAGCAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTCTCTGCAAATGAACAGCCTGAGAGCCGAGG ACACGGCTGTGTATTACTGTGCGAGAGATGGCAG CTATGGGTATGACGGCCTTGACTACTGGGGCCAG GGAACCCTGGTCACCGTCTCCTCA |
| | | | SEQ ID NO: 25889 | SEQ ID NO: 29895 |

FIGURE 50

| | | | | |
|---|---|---|---|---|
| | | AA | DIQMTQSPSSLSASVGDRVTITCRASQGIGRYLA WFQQKPGRAPKSLIYTASSLQSGAPSKFSGSGSG TDFTLTISSLQPEDFATYYCQQYSNYPLTFGGGT KVEIN | QVQLVESGGGVVQPGRSLRLSCAASGFTFSSYGMH WVRQAPGKGLEWVAVIWYDGSNKNYADSVKGRF TISRDNSKNTLSLQMNSLRAEDTAVYYCARDGSYG YDGLDYWGQGTLVTVSS |
| | | | SEQ ID NO: 25890 | SEQ ID NO: 29896 |
| IPS:434743 | 21-225_74A4 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTGTGGGACAGACTTCGCTCTCAC CATCAGCAGACTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC ATGGACGTCTGGGGCCAAGGGACCACGGTCACC GTCTCCTCA |
| | | | SEQ ID NO: 25891 | SEQ ID NO: 29897 |
| | | AA | EIVLTQSPGTLYLSPGERATLSCRASQSVYSSYLA WYQQKPGQAPRLLIYGASSRSTGIPDRFSGSGCG TDFALTISRLEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25892 | SEQ ID NO: 29898 |

FIGURE 50

| iPS:434747 | 21-225_80C12 | NA | GAAATTGTGTTGACGCAGTCTCCAGGCACCCT GTATTTGTCTCCAGGGGAAAGAGCCACCCTCT CGTGCAGGGCCAGTCAGAGTGTTAGCAGCAGC TACTTAGCCTGGTACCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTAACTCACCG CTCACTTTCGGCGGAGGGACCAAGGTGGAGAT CAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAATGG TAACACAGGCTATGCACAGAAGTTCCAGGGCAG AGTCACCATGACCAGGAACACCTCCATAAGCAC AGCCTACATGGAGCTGAACAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTTA |
| | | | SEQ ID NO: 25893 | SEQ ID NO: 29899 |
| | | AA | EIVLTQSPGTLYLSPGERATLSCRASQSVSSSYLA WYQQKPGQAPRLLIYGASTRATGIPDRFSGSGSG TDFTLTISRLEPEYFAVYYCQQYGNSPLTFGGGT KVEIK | QVQLVQSGAEVKKPGASVKVSCKASGYTFTNYDIN WVRQATGQGLEWMGWMHPNNGNTGYAQKFQGR VTMTRNTSISTAYMELNSLRSEDTAVYYCAISSGW YWFDPWGQGTLVTVSL |
| | | | SEQ ID NO: 25894 | SEQ ID NO: 29900 |
| iPS:434751 | 21-225_80H12 | NA | GAAATTGTGTTGACGCAGTCTCCAGGCACCCT GTCTTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25895 | SEQ ID NO: 29901 |

FIGURE 50

| | | | | |
|---|---|---|---|---|
| | | AA | EIVLTQSPGTLSLSPGERATLSCRASQSVYSSYLA WYQQKPGQAPRLLIYGASSRSTGIPDRFSGSGSG TDFALTISRLEPEDFAVYYCQHSDNSPWTFGQGT KVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25896 | SEQ ID NO: 29902 |
| IPS:434759 | 21-225_81C5 | NA | GAAATTGTGTTGACGCAGTCTCCAGGCACCCT GTATTTGTCTTCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTGTGGGACAGACTTCGCTCTCAC CATCAGCAGAGTGGAGCCTGAAGATTGTGCAG TGTATTACTGTCAGCATTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25897 | SEQ ID NO: 29903 |
| | | AA | EIVLTQSPGTLYLSSGERATLSCRASQSVYSSYLA WYQQKPGQAPRLLIYGASSRSTGIPDRFSGSGCG TDFALTISRVEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25898 | SEQ ID NO: 29904 |

FIGURE 50

| iPS:434761 | 21-225_81E5 | NA | GACATCGTTATGACCCAGTCTCCAGACTCCCT GGCTGTGTCTCTGGGCGAGAGGGCCACCATCA ATTGCAAGTCCAGCCAGAGTGTTTTATTCAGC TCCAACAATTATAATTACTTAGCTTGGTACCA GCAGAGACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGCTGATGTGGCAGTTTATTACTGTCAGCAA TATTATAGTTCTCCTCTGACGTTCGGCCAAGG GACCACGGTGCAAATCAAA | CAGGTGCAGTTGGTGCAGTCTGGGGCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGAACCCTAACAGTGG TAACACAGGCTATGCACAGAAGTTCCAGGGCAG AGTCACCATGACCAGGAACACCTCCACAAGCAC AGCCCACATGGAGCTGAGCAGCCTGAGATCTGA GGACACGGCCGTGTATTACTGTGCGGTCTCCAGT GGCTGGAACTGGTTCGACCCCTGGGGCCAGGGA ACCCTGGTCACCGTCTCCTCA |
|---|---|---|---|---|
| | | | SEQ ID NO: 25899 | SEQ ID NO: 29905 |
| | | AA | DIVMTQSPDSLAVSLGERATINCKSSQSVLFSSN NYNYLAWYQQRPGQPPNLLIYWASTRESGVPD RFSGSGSGTDFTLTISSLQAADVAVYYCQQYYSS PLTFGQGTTVQIK | QVQLVQSGAEVKKPGASVKVSCKASGYTFTNYDIN WVRQATGQGLEWMGWMNPNSGNTGYAQKFQGR VTMTRNTSTSTAHMELSSLRSEDTAVYYCAVSSGW NWFDPWGQGTLVTVSS |
| | | | SEQ ID NO: 25900 | SEQ ID NO: 29906 |
| iPS:434771 | 21-225_81F9 | NA | GACATCGTTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATCGTCAGCAC TACAATGATACTCCAGGGAAGTTTGTCCAAGG CATCATGGTGGAAATCACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTCA |
| | | | SEQ ID NO: 25901 | SEQ ID NO: 29907 |

FIGURE 50

| | | | | |
|---|---|---|---|---|
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYRQHYNDT PGKFVQGIMVEIT | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25902 | SEQ ID NO: 29908 |
| IPS:434773 | 21-225_75D9 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAGCAGC TACTTAGCCTGGTACCAGCAGAAACGTGGCCA GGCTCCCAGGCTCCTCATCTATGGTGCATCCA GCAGGGCCACTGGCATTCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGAAAGCTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTCCCTACTG GAGCTGGATCCGCCAGCCCCCAGGGAAGGGGCT GGAGTGGATTGGGGAAATCAATTATAGGGGAAG CACCAACTACAACCCGTCCCTCAAGAGTCGAGTC ACCATTTCAGTAGACACGTCCAAGAACCAGTTCT CCCTGAACCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25903 | SEQ ID NO: 29909 |
| | | AA | EIVLTQSPGTLSLSPGERATLSCRASQSVSSSYLA WYQQKRGQAPRLLIYGASSRATGIPDRFSGSGSG TDFTLTISRLEPEDFAVYYCQQYESSPWTFGQGT KVEIK | QVQLQQWGAGLLKPSETLSLTCAVYGGSFSGPYW SWIRQPPGKGLEWIGEINYRGSTNYNPSLKSRVTIS VDTSKNQFSLNLSSVTAADTAVYYCARDYGGMDV WGQGTTVTVSS |
| | | | SEQ ID NO: 25904 | SEQ ID NO: 29910 |

FIGURE 50

| iPS:434777 | 21-225_81C11 | NA | GAAATTGTGTTGACGCAGTCTCCAGGCACCCG GTATTTGTCTCCAGTGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCCTCCCAGACAGGTTCAGT GGCAGTGGGTGTGGGACAGACTTCGCTCTCAC CATCAGCAGAGTGGAGCCTGAAGATTGTGCAG TGTATTACTGTCAGCATTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
|---|---|---|---|---|
| | | | SEQ ID NO: 25905 | SEQ ID NO: 29911 |
| | | AA | EIVLTQSPGTRYLSPVERATLSCRASQSVYSSYL AWYQQKPGQAPRLLIYGASSRSTGLPDRFSGSG CGTDFALTISRVEPEDCAVYYCQHSDNSPWTFG QGTKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25906 | SEQ ID NO: 29912 |
| iPS:434793 | 21-225_82A5 | NA | GAAATTGTGTTGACGCAGTCTCCAGGCACCCT GTCTTGGTCTCCAGGGGAAAGAGCCACCCTCT CGTGCAGGGCCAGTCAGAGTGTTAGCAGCAGC TACTTAGCCTGGTACCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTAACTCACCG CTCACTTTCGGCGGAGGGACCAAGGTGGAGAT CAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAATGG TAACACAGGCTATGCACAGAAGTTCCAGGGCAG AGTCACCATGACCAGGAACACCTCCATAAGCAC AGCCTACATGGAGCTGAACAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTTA |
| | | | SEQ ID NO: 25907 | SEQ ID NO: 29913 |

FIGURE 50

| | | | | |
|---|---|---|---|---|
| | | AA | EIVLTQSPGTLSWSPGERATLSCRASQSVSSSYLA WYQQKPGQAPRLLIYGASTRATGIPDRFSGSGSG TDFTLTISRLEPEYFAVYYCQQYGNSPLTFGGGT KVEIK | QVQLVQSGAEVKKPGASVKVSCKASGYTFTNYDIN WVRQATGQGLEWMGWMHPNNGNTGYAQKFQGR VTMTRNTSISTAYMELNSLRSEDTAVYYCAISSGW YWFDPWGQGTLVTVSL |
| | | | SEQ ID NO: 25908 | SEQ ID NO: 29914 |
| iPS:434797 | 21-225_82G5 | NA | GAATTTATGTTGACGCAGTCTCCAGGCACCCT GTATTGGTCTCCAGGGGAAAGAGCCACCCTCT CGAGCAGGGCCAGTGAGAGTGTTAGCAGCAG CTACTTAGTCTGGTATCAGCAGAAACCTGGCC AGGCTCCCAGGCTCCTCATCTATGGTGCATCC ACCAGGGCCACTGGCATCCCAGACAGGTTCAG TGGCAGTGGGTCTGGGACAGACTTCACTCTCA CCATCAGCAGACTGGAGCCTGAATATTTTGCA GTTTATTACTGTCAGCAGTATGGTTGCTCACCG CTCACTTTCGGCGGAGGGACCAAGGTGGAGAT CACA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGGCCAGGGA ACCCTGGTCACCGTCTCCTCA |
| | | | SEQ ID NO: 25909 | SEQ ID NO: 29915 |
| | | AA | EFMLTQSPGTLYWSPGERATLSSRASESVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25910 | SEQ ID NO: 29916 |

FIGURE 50

| iPS:434805 | 21-225_82D9 | NA | GAATTTATGTTGACGCAGTCTCCAGGCACCCT GTCTTGGTCTCCAGGGGAAAGAGCCACCCTCT CGTGCAGGGCCAGTGAGAGTGTTAGCAGCAG CTACTTAGTCTGGTATCAGCAGAAACCTGGCC AGGCTCCCAGGCTCCTCATCTATGGTGCATCC ACCAGGGCCACTGGCATCCCAGACAGGTTCAG TGGCAGTGGGTCTGGGACAGACTTCACTCTCA CCATCAGCAGACTGGAGCCTGAATATTTTGCA GTTTATTACTGTCAGCAGTATGGTTGCTCACCG CTCACTTTCGGCGGAGGGACCAAGGTGGAGAT CACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
|---|---|---|---|---|
| | | | SEQ ID NO: 25911 | SEQ ID NO: 29917 |
| | | AA | EFMLTQSPGTLSWSPGERATLSCRASESVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25912 | SEQ ID NO: 29918 |
| iPS:434809 | 21-225_74F5 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25913 | SEQ ID NO: 29919 |

FIGURE 50

| | | | | |
|---|---|---|---|---|
| | | AA | EIVLTQSPGTLYLSPGERATLSCRASQSVYSSYLA WYQQKPGQAPRLLIYGASSRSTGIPDRFSGSGSG TDFALTISRLEPEDFAVYYCQHSDNSPWTFGQGT KVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25914 | SEQ ID NO: 29920 |
| iPS:434813 | 21-225_82C12 | NA | GAAATTGTGTTGACGCAGTCTCCAGGCACCCT GTCTTGGTCTCCAGGGGAAAGAGCCACCCTCT CGTGCAGGGCCAGTCAGAGTGTTAGCAGCAGC TACTTAGCCTGGTACCAGCAGAAACCTGGCCA GGCTCCCAGGCTCCTCATCTATGGTGCATCCA CCAGGGCCTCTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTAACTCACCG CTCACTTTCGGCGGAGGGACCAAGGTGGAGAT CAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAATGG TAACACAGGCTATGCACAGAAGTTCCAGGGCAG AGTCACCATGACCAGGAACACCTCCATAAGCAC AGCCTACATGGAGCTGAACAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTTA |
| | | | SEQ ID NO: 25915 | SEQ ID NO: 29921 |
| | | AA | EIVLTQSPGTLSWSPGERATLSCRASQSVSSSYLA WYQQKPGQAPRLLIYGASTRASGIPDRFSGSGSG TDFTLTISRLEPEYFAVYYCQQYGNSPLTFGGGT KVEIK | QVQLVQSGAEVKKPGASVKVSCKASGYTFTNYDIN WVRQATGQGLEWMGWMHPNNGNTGYAQKFQGR VTMTRNTSISTAYMELNSLRSEDTAVYYCAISSGW YWFDPWGQGTLVTVSL |
| | | | SEQ ID NO: 25916 | SEQ ID NO: 29922 |

1999

FIGURE 50

| iPS:434815 | 21-225_74A11 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGGGAAAG CCCCTAAGCGCCTGATCTGTGCTGCATCCAGT TTGCAAAGTGGGGTCCCATCAAGGTTCAGCGG CAGTGGATCTGGGACCGAGTTCACTCTCACAA TCAGCAGCCTGCAGCCTGAGGATTTTGCAACT TATTTCTGTCTACAGCATAATGATTACCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGTA AGGCTTCTGGATACACCTTCACCAGTTATGATAT CAACTGGGTGCGACAGGCCACTGGACAAGGGCT TGAGTGGATGGGATGGATGAACCCTAACAGTGG TAACACAGGCTATGCACAGAAGTTCCAGGGCAG AGTCACCATGACCTGGAACACCTCCAAAAGCAC AGCCTACATGGAGCTGAGCAGCCTGAGATCTGA GGACACGGCCGTGTATTACTGTGCGAGAGGCTTT TACGATACTTTGACTGGTTCCGGCTACTACTACG TTATGGACGTCTGGGGCCAAGGGACCACGGTCA CCGTCTCCTCA |
|---|---|---|---|---|
| | | | SEQ ID NO: 25917 | SEQ ID NO: 29923 |
| | | AA | DIQMTQSPSSLSASVGDRVTITCRASQDIRNDLG WYQQKPGKAPKRLICAASSLQSGVPSRFSGSGS GTEFTLTISSLQPEDFATYFCLQHNDYPFTFGPGT KVDIK | QVQLVQSGAEVKKPGASVKVSCKASGYTFTSYDIN WVRQATGQGLEWMGWMNPNSGNTGYAQKFQGR VTMTWNTSKSTAYMELSSLRSEDTAVYYCARGFY DTLTGSGYYYVMDVWGQGTTVTVSS |
| | | | SEQ ID NO: 25918 | SEQ ID NO: 29924 |
| iPS:434821 | 21-225_83G1 | NA | GAAATTGTGTTGACGCAGTCTCCAGGCACCCG GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACGGTCCTCCCAGACAGGTTCAGT GGCAGTGGGTCTGGGACAGACTTCGCTCTCAC CATCAGCAGAGTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25919 | SEQ ID NO: 29925 |

FIGURE 50

| | | | | |
|---|---|---|---|---|
| | | AA | EIVLTQSPGTRYLSPGERATLSCRASQSVYSSYL AWYQQKPGQAPRLLIYGASSRSTVLPDRFSGSGS GTDFALTISRVEPEDCAVYYCQHSDNSPWTFGQ GTKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25920 | SEQ ID NO: 29926 |
| iPS:434825 | 21-225_83C2 | NA | GAATTTATGTTGACGCAGTCTCCAGGCACCCT GTGTTTGTCTCCAGGGGAAAGAGCCACCCTCT CGTGCAGGGCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
| | | | SEQ ID NO: 25921 | SEQ ID NO: 29927 |
| | | AA | EFMLTQSPGTLCLSPGERATLSCRASQSVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25922 | SEQ ID NO: 29928 |

FIGURE 50

| iPS:434827 | 21-225_83F3 | NA | GACATCGTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCACT ATAATGATACTCCATGGAAGTTTGTCCAAGGG ATCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTCA |
|---|---|---|---|---|
| | | | SEQ ID NO: 25923 | SEQ ID NO: 29929 |
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQHYNDT PWKFVQGIKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25924 | SEQ ID NO: 29930 |
| iPS:434829 | 21-225_83G3 | NA | GACATCGTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCACT ATTATAATACTCCGTGGACGTTTGTCCAAGGG ACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTCA |
| | | | SEQ ID NO: 25925 | SEQ ID NO: 29931 |

EP 4 435 105 A2

FIGURE 50

| | | | | |
|---|---|---|---|---|
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQHYYNT PWTFVQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25926 | SEQ ID NO: 29932 |
| iPS:434833 | 21-225_83C5 | NA | GAATTTATGTTGACGCAGTCTCCAGGCACCCT GTGTTGGTCTCCAGGGGAAAGAGCCACCCTCT CGTGCAGGGCCAGTGAGAGTGTTAGCAGCAG CTACTTAGTCTGGTATCAGCAGAAACCTGGCC AGGCTCCCAGGCTCCTCATCTATGGTGCATCC ACCAGGGCCACTGGCATCCCAGACAGGTTCAG TGGCAGTGGGTCTGGGACAGACTTCACTCTCA CCATCAGCAGACTGGAGCCTGAATATTTTGCA GTTTATTACTGTCAGCAGTATGGTTGCTCACCG CTCACTTTCGGCGGAGGGACCAAGGTGGAGAT CACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGGCCAGGGA ACCCTGGTCACCGTCTCCTCA |
| | | | SEQ ID NO: 25927 | SEQ ID NO: 29933 |
| | | AA | EFMLTQSPGTLCWSPGERATLSCRASESVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25928 | SEQ ID NO: 29934 |

FIGURE 50

| iPS:434835 | 21-225_83B6 | NA | GAAATTGTGTTGACGCAGTCTCCAGGCACTTT GTCTTTGTCTCCAGGGGAAAGAGCCACCCTCT CCTGCAGGGCCAGTCAGAGTGTTGACAGCGGC TACTTAGCCTGGTACCAGCAGAAACCTGGCCA GGCTCCCAGGCTCCTCATCTATGGTGCATCCA GCAGGACCCCTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGAAAGCTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTGTTACTGG AGCTGGATCCGCCAGCCCCCAGGGAAGGGGCTG GAGTGGATTGGGGAAATCAATCATAGTGGAAGG ACCAACTACAACCCCTCCCTCAAGAGTCGAGTCA CCATATCAGTAGACACGTCCGAGAACCAGTTCTC CCTGAAGCTGAGCTCTGTGACCGCCGCGGACACG GCTGTGTATTACTGTGCGAGAGACTACGGTGGGC TTGACTACTGGGGCCAGGGAACCCTGGTCACCGT CTCCTCA |
|---|---|---|---|---|
| | | | SEQ ID NO: 25929 | SEQ ID NO: 29935 |
| | | AA | EIVLTQSPGTLSLSPGERATLSCRASQSVDSGYLA WYQQKPGQAPRLLIYGASSRTPGIPDRFSGSGSG TDFTLTISRLEPEDFAVYYCQQYESSPWTFGQGT KVEIK | QVQLQQWGAGLLKPSETLSLTCAVYGGSFSGCYW SWIRQPPGKGLEWIGEINHSGRTNYNPSLKSRVTIS VDTSENQFSLKLSSVTAADTAVYYCARDYGGLDY WGQGTLVTVSS |
| | | | SEQ ID NO: 25930 | SEQ ID NO: 29936 |
| iPS:434839 | 21-225_83B7 | NA | GAAATTGTGTTGACGCAGTCTCCAGGCACCCG GTATTTGTCTTCAGTGGAAAGAGCCACCCTCT CATGCAGGGCCAGTCAGAGTGTTTACAGCAGC TACTTAGCGTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25931 | SEQ ID NO: 29937 |

EP 4 435 105 A2

FIGURE 50

| | | | | |
|---|---|---|---|---|
| | | AA | EIVLTQSPGTRYLSSVERATLSCRASQSVYSSYL AWYQQKPGQAPRLLIYGASSRSTGIPDRFSGSGS GTDFALTISRLEPEDCAVYYCQHSDNSPWTFGQ GTKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25932 | SEQ ID NO: 29938 |
| iPS:434841 | 21-225_83G7 | NA | GACATCGTGATGACCCAGTCTCCAGACTCCCT GGCTGTGTCTCTGGGCGAGAGGGCCACCATCA TCTGCAAGTCCAGCCAGACTGTTTTACACAGC TCCAACAATTATAACTACTTAGCTTGGTACCA GCAGAAGCCAGGACAGCCCCCTAAGGTGCTC ATTTACTGGACATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTACTGTCAGCA ATATTTTAGTAGTCCTCTGACGTTCGGCCAAG GGACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCCCCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTATTGTGCGGTTTCCAG TGGCTGGCACTGGTTCGACCCCTGGGGCCAGGGA ACCCTGGTCACCGTCGCCTCA |
| | | | SEQ ID NO: 25933 | SEQ ID NO: 29939 |
| | | AA | DIVMTQSPDSLAVSLGERATIICKSSQTVLHSSNN YNYLAWYQQKPGQPPKVLIYWTSTRESGVPDRF SGSGSGTDFTLTISSLQAEDVAVYYCQQYFSSPL TFGQGTKVEIK | QVQLVQSGAEVKKPGASVKVSCKASGYTFPNYDIN WVRQATGQGLEWMGWMNPNSGNTGYAQKFQGR VTMTRNTSISTAYMELSSLRSEDTAVYYCAVSSGW HWFDPWGQGTLVTVAS |
| | | | SEQ ID NO: 25934 | SEQ ID NO: 29940 |

2005

FIGURE 50

| iPS:434849 | 21-225_83C10 | NA | GAAATTGTGTTGACGCAGTCTCCAGGCACCCT GTCTTTGTCTCCAGGGGAAAGAGCCACCCTCT CCTGCAGGGCCAGTCCGAGTGTTCACAGCAAC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCATTCTCAC CATCAGCAGACTGGAGCCTGAAGATTTTGCAG TGTATTACTGTCAGCAGTATGAAAGTTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCA CTGTCTATGGTGGGTCCTTCAGTGGTTACTACTG GAGCTGGATCCGCCAGCCCCCAGGGAAGGGGCT GGAATGGATTGGGGAAATCAATCATAGTGGAAG CACCAACTTCAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTGTTTTACTGTGCGAGAGACTACGGTGGG CTTGACTACTGGGGCCAGGGAACCCTGGTCACCG TCTCCTCA |
| | | | SEQ ID NO: 25935 | SEQ ID NO: 29941 |
| | | AA | EIVLTQSPGTLSLSPGERATLSCRASPSVHSNYLA WYQQKPGQAPRLLIYGASSRATGIPDRFSGSGSG TDFILTISRLEPEDFAVYYCQQYESSPWTFGQGT KVEIK | QVQLQQWGAGLLKPSETLSLTCTVYGGSFSGYYW SWIRQPPGKGLEWIGEINHSGSTNFNPSLKSRVTISV DTSKNQFSLKLSSVTAADTAVFYCARDYGGLDYW GQGTLVTVSS |
| | | | SEQ ID NO: 25936 | SEQ ID NO: 29942 |
| iPS:434851 | 21-225_75A6 | NA | GACATCGTGATGACCCAGTCGCCAGACTCCCT GGCTGTGTCTCTGGGCGAGAGGGCCACCATCA ACTGCAAGTCCAGACAGAGTGTTTTACACAGC TCCAACAATTACAACTACTTAGCTTGGTACCA GCAGAAACCAGGACAGCCTCCTGAACTACTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAGGATGTGGCAGTTTATTACTGTCAGCAA TATTATAGTACTCCTCCTACTTTCGGCGGAGG GACCAAGGTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGTATAGCAG TGGCTGGTACATCTTTGACTACTGGGGCCAGGGA ACCCTGGTCACCGTCTCCTCA |
| | | | SEQ ID NO: 25937 | SEQ ID NO: 29943 |

FIGURE 50

| | | | | |
|---|---|---|---|---|
| | | AA | DIVMTQSPDSLAVSLGERATINCKSRQSVLHSSN NYNYLAWYQQKPGQPPELLIYWASTRESGVPDR FSGSGSGTDFTLTISSLQAEDVAVYYCQQYYSTP PTFGGGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAYSSGW YIFDYWGQGTLVTVSS |
| | | | SEQ ID NO: 25938 | SEQ ID NO: 29944 |
| iPS:434863 | 21-225_84G7 | NA | GACATCGTGATGACCCAGTCTCCAGACTCCCC GGCTGTGTCTCTGGGCGAGAGGGCCACCATCA TCTGCAAGTCCAGCCAGACTGTTTTACACAGC TCCAACAATTATAACTACTTAGCTTGGTACCA GCAGAAGCCAGGACAGCCCCCTAAGGTGCTC ATTTACTGGACATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTACTGTCAGCA ATATTTTAGTAGTCCTCCGACGTTCGGCCAAG GGACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCCCCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGACA GAGTCACCATGACCAGGAACACCTCCATAAGCA CAGCCTACATGGAGCTGAGCAGCCTGAGATCTG AGGACACGGCCGTGTATTATTGTGCGGTTTCCAG TGGCTGGCACTGGTTCGACCCCTGGGGGCCAGGGA ACCCTGGTCACCGTCGCCTCA |
| | | | SEQ ID NO: 25939 | SEQ ID NO: 29945 |
| | | AA | DIVMTQSPDSPAVSLGERATIICKSSQTVLHSSNN YNYLAWYQQKPGQPPKVLIYWTSTRESGVPDRF SGSGSGTDFTLTISSLQAEDVAVYYCQQYFSSPP TFGQGTKVEIK | QVQLVQSGAEVKKPGASVKVSCKASGYTFPNYDIN WVRQATGQGLEWMGWMNPNSGNTGYAQKFQDR VTMTRNTSISTAYMELSSLRSEDTAVYYCAVSSGW HWFDPWGQGTLVTVAS |
| | | | SEQ ID NO: 25940 | SEQ ID NO: 29946 |

FIGURE 50

| iPS:434867 | 21-225_79A12 | NA | GACATCCAGATGACCCAGTCTCCATCCTCACT GTCTGCATCTGTAGGAGACAGAGTCACCATCA CTTGTCGGGCGAGTCAGGTCATTAGCAAGTAT TTAGCCTGGTTTCAGCAGAAACCAGGGAAAGC CCCTAAGTCCCTGATCTATGCTGCATCCAGTTT GCAAAGTGGGGCCCCATCAAAGTTCAGCGGC AGTGGATCTGGGACAGATTTCACTCTCACCAT CAGCAGCCTGCAGCCTGAAGATTTTGCAACTT ATTACTGCCAACAGTACAGTAATTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATGGCAG CTATGGTTACGACGGCCTTGACTACTGGGGCCAG GGAACCCTGGTCGCCGTCTCCTCA |
| | | | SEQ ID NO: 25941 | SEQ ID NO: 29947 |
| | | AA | DIQMTQSPSSLSASVGDRVTITCRASQVISKYLA WFQQKPGKAPKSLIYAASSLQSGAPSKFSGSGSG TDFTLTISSLQPEDFATYYCQQYSNYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDGSYG YDGLDYWGQGTLVAVSS |
| | | | SEQ ID NO: 25942 | SEQ ID NO: 29948 |
| iPS:434869 | 21-225_84E12 | NA | GAAATTGTGTTGACGCAGTCTCCAGGCACCCT GTCTTTGTCTCCAGGGGAAAGAGCCACCCTCT CCTGCAGGGCCAGTCAGAGTATTAACAGCAAC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGACGATTTTGCAG TGTTTTACTGTCAGCAGTATGAGAGCTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCTATGGTGGGTCCTTCAGTGGTTCCTACTGG AGCTGGATCCGCCAGCCCCCAGGGAAGGGGCTA GAGTGGATTGGGGAAATCAATCAAAGTGGACGC ACCAACTACAACCCGTCCCTCAAGAGTCGAGTCA CCATATCAGTAGACACGTCCAAGAACCAGTTCTC CCTGAAGCTGAGCTCTGTGACCGCCGCGGACACG GCTGTGTATTACTGTGCGAGAGACTACGGGGGTA TAGACGTCTGGGGCCAAGGGACCACGGTCACCG TCTCCTCA |
| | | | SEQ ID NO: 25943 | SEQ ID NO: 29949 |

FIGURE 50

| | | | | |
|---|---|---|---|---|
| | | AA | EIVLTQSPGTLSLSPGERATLSCRASQSINSNYLA WYQQKPGQAPRLLIYGASSRATGIPDRFSGSGSG TDFTLTISRLEPDDFAVFYCQQYESSPWTFGQGT KVEIK | QVQLQQWGAGLLKPSETLSLTCAVYGGSFSGSYW SWIRQPPGKGLEWIGEINQSGRTNYNPSLKSRVTIS VDTSKNQFSLKLSSVTAADTAVYYCARDYGGIDV WGQGTTVTVSS |
| | | | SEQ ID NO: 25944 | SEQ ID NO: 29950 |
| iPS:434871 | 21-225_85H1 | NA | GAGATAGTGATGACGCAGTCTCCAGCCACCCT GTCTGTGTCTCCAGGGGAAAGAGCCACCCTCT CCTGCAGGGCCAGTCAGGATGTTATCACCTAC TTAGCCTGGTACCAGCAGAAACCTGGCCAGGC TCCCAGGCTCCTCATCTATGGTGCATCCACCA GGGCCACTGGTGTCCCAGCCAGGTTCAGTGGC AGTGGGTCTGGGACAGAGTTCACTCTCACCAT CAGCAGCCTGCAGTCTGAAGATTTTGCACTTT ATTACTGTCAGGAGTATAATGACTGGCCGTGC AGTTTTGGCCAGGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT ACACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCCCTT TATAGTGGGAGCTACTTACTTTGACTACTGGGGC CAGGGAACCCTGGTCACCGTCTCCTCA |
| | | | SEQ ID NO: 25945 | SEQ ID NO: 29951 |
| | | AA | EIVMTQSPATLSVSPGERATLSCRASQDVITYLA WYQQKPGQAPRLLIYGASTRATGVPARFSGSGS GTEFTLTISSLQSEDFALYYCQEYNDWPCSFGQG TKVEIK | QVQLVESGGGVVQPGRSLRLSCAASGFTFSSYGIH WVRQAPGKGLEWVAVIWYDGSNKYYADSVKGRF TISRDNSKNTLYLQMNSLRAEDTAVYYCARDPFIV GATYFDYWGQGTLVTVSS |
| | | | SEQ ID NO: 25946 | SEQ ID NO: 29952 |

2009

FIGURE 50

| iPS:434877 | 21-225_85H2 | NA | GACAGCATGATGACCCAGTCTCCAGACTCCCT GGCTGTGTCTCTGGGCGAGAGGGCCACCATCA ACTGCAAGTCCAGCCAGAGTGTTTTACACAGC TCTAATAAAAAGAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGTCTGCAGG CTGAAGATGTGGCAGTTTATTACTGTCAGCAA TATTATAGGACTCCGTGGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAATAGTGG TAACACAGGCTATGCACAGAAGTTCCAGGGCAG AGTCACCCTGACCAGGAACACCTCCATAAGCAC AGCCTACATGGAGCTGAGCAGCCTGAGATCTGA GGACACGGCCGTGTATTACTGTGCGGTTTCCAGT GGCTGGTACTGGTTCGACCCCTGGGGCCAGGGA ACCCTGGTCACCGTCTCCTCA |
|---|---|---|---|---|
| | | | SEQ ID NO: 25947 | SEQ ID NO: 29953 |
| | | AA | DSMMTQSPDSLAVSLGERATINCKSSQSVLHSSN KKNYLAWYQQKPGQPPKLLIYWASTRESGVPD RFSGSGSGTDFTLTISSLQAEDVAVYYCQQYYRT PWTFGQGTKVEIK | QVQLVQSGAEVKKPGASVKVSCKASGYTFTNYDIN WVRQATGQGLEWMGWMHPNSGNTGYAQKFQGR VTLTRNTSISTAYMELSSLRSEDTAVYYCAVSSGW YWFDPWGQGTLVTVSS |
| | | | SEQ ID NO: 25948 | SEQ ID NO: 29954 |
| iPS:434879 | 21-225_85A3 | NA | GAAATTGTGTTGACGCAGTCTCCAGGCACCCT GTTTTTGTCTCCAGGGGAAAGAGCCACCCTCT CCTGCAGGGCCAGTCAGAGTGTTTACAGCAGC TACTTAGCCTGGTACCAGCAGAAACCTGCCCA GGCTCCCAGGCTCCTCATCTATGGTGCATCCA GCCGGGCCAGTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25949 | SEQ ID NO: 29955 |

FIGURE 50

| | | | | |
|---|---|---|---|---|
| | | AA | EIVLTQSPGTLFLSPGERATLSCRASQSVYSSYLA WYQQKPAQAPRLLIYGASSRASGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25950 | SEQ ID NO: 29956 |
| IPS:434881 | 21-225_85B4 | NA | GAAATTGTGTTGACGCAGTCTCCAGGCACCCT GTTTTTGTCTCCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATCTATGGTGCATCCA GCCGGGCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25951 | SEQ ID NO: 29957 |
| | | AA | EIVLTQSPGTLFLSPGERATLSCRASQSVYSSYLA WYQQKPGQAPRLLIYGASSRATGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25952 | SEQ ID NO: 29958 |

FIGURE 50

| iPS:434883 | 21-225_85B5 | NA | GAATTTATGTTGACGCAGTCTCCAGGCACCCT GTCTTTGTCTCCAGGGGAAAGAGCCACCCTCT CGTGCAGGTCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
|---|---|---|---|---|
| | | | SEQ ID NO: 25953 | SEQ ID NO: 29959 |
| | | AA | EFMLTQSPGTLSLSPGERATLSCRSSQSVSSSYLV WYQQKPGQAPRLLIYGASTRATGIPDRFSGSGSG TDFTLTISRLEPEYFAVYYCQQYGCSPLTFGGGT KVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25954 | SEQ ID NO: 29960 |
| iPS:434887 | 21-225_85D6 | NA | GAAATTGTGTTGACGCAGTCTCCAGGCACCCT GTTTTTGTCTCAAGGGGAAAGAGCCACCCTCT CCTGCAGGGCCAGTCAGAGTGTTAGCAGCAGG TACTTAGCCTGGTACCAGCAGAAACCTGGCCA GGCTCCCAGACTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TATATTACTGTCAGCATTATGATAGCTCACCGT GGACGTTCGGCCAAGGGACCAAGGTGGAAAT CAAA | CAGGTGCAGCTACAGCAGGGGGGCGCAGGACCG TTGAAGCCTTCGGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATTATAGTGGAAG AACCAACTACAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
| | | | SEQ ID NO: 25955 | SEQ ID NO: 29961 |

FIGURE 50

| | | | | |
|---|---|---|---|---|
| | | AA | EIVLTQSPGTLFLSQGERATLSCRASQSVSSRYLA WYQQKPGQAPRLLIYGASSRATGIPDRFSGSGSG TDFTLTISRLEPEDFAVYYCQHYDSSPWTFGQGT KVEIK | QVQLQQGGAGPLKPSETLSLTCAVYGGSFSGCYWS WIRQPPGKGLEWIGEINYSGRTNYNPSLKSRVTISV DTSKNQFSLKLSSVTAADTAVYYCARDYGGMDV WGQGTTVTVSS |
| | | | SEQ ID NO: 25956 | SEQ ID NO: 29962 |
| IPS:434891 | 21-225_85G6 | NA | GAAATTGCGTTGACGCAGTCTCCAGGCACCCT GTCTTTGTCTCCAGGGGAAAGAGCCACCCTCT CCTGCAGGGCCAGTCCGAGTGTTGACAGCAGC TACTTAGCCTGGTACCAGCAGAAACCTGGCCA GGCTCCCAGGCTCCTCATCTATGGTGCAGCCA GCAGGGCCCCTGGCATCCCAGACAGGTTCAGT GGCAGTGGGTCTGGGACAGACTTCACTCTCAC CATCAGCAGACTGGAGCCTGAAGACTTTGTAG TGTTACTACTGTCAGCAGTATGAAAGTTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGATTGCTACTG GAGCTGGATCCGCCAGCCCCCAGGGAAGGGGCT GGAGTGGATTGGGGAAATCAATCATAGTGGAAG GACCAACTACAACCCGTCCCTCAAGAGTCGAGTC ACCATATCAGTAGACACGTCCGAGAACCAGTTCT CCCTGAAGCTGAGCTCTGTGACCGCCGCGGACAC GGCTGTGTATTACTGTGCGAGAGACTACGGTGGG CTTGACTACTGGGGCCAGGGAACCCTGGTCACCG TCTCCTCA |
| | | | SEQ ID NO: 25957 | SEQ ID NO: 29963 |
| | | AA | EIALTQSPGTLSLSPGERATLSCRASPSVDSSYLA WYQQKPGQAPRLLIYGAASRAPGIPDRFSGSGSG TDFTLTISRLEPEDFVVYYCQQYESSPWTFGQGT KVEIK | QVQLQQWGAGLLKPSETLSLTCAVYGGSFSDCYW SWIRQPPGKGLEWIGEINHSGRTNYNPSLKSRVTIS VDTSENQFSLKLSSVTAADTAVYYCARDYGGLDY WGQGTLVTVSS |
| | | | SEQ ID NO: 25958 | SEQ ID NO: 29964 |

FIGURE 50

| iPS:434895 | 21-225_74H7 | NA | GAACTTGTGTTGACGCAGTCTCCAGGCACCCT GTCTTTGTCTCCAGGGGAAAGAGCCACCCTCT CCTGCAGGGCCAGTCAGAATATTTACAGCAGC TACTTAGCCTGGTACCAACAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTGCTGTCAGCAGTATGAAAGCTCACCG TGGACCTTCGGCCAAGGGACCAAGGTGGAAA TCAAA | CAAGTGCAGCTACAGCAGTGGGGCGCAGGACTG TTGAAGCCTTCGGAGCCCCTGTCCCTCACCTGCG CTGTCTATGGTGGGTCCTTCAGTGGTCCCTACTG GAGCTGGATCCGCCAGCCCCCCGGGAAGGGGCT GGAGTGGATTGGGGAAATCAATCATAGTGGAAG CACCAACTACAACCCGTCCCTCAAGAGTCGAGTC ACCATATCAGTAGACACGTCCAAGAACCAGTTCT CCCTGAAACTGACCTCTGTGACCGCCGCGGACAC GGCTGTGTATTACTGTGCGAGAGACTACGGGGGT TTGGACGTCTGGGGCCAAGGGACCACGGTCACC GTCTCCTCA |
| | | | SEQ ID NO: 25959 | SEQ ID NO: 29965 |
| | | AA | ELVLTQSPGTLSLSPGERATLSCRASQNIYSSYLA WYQQKPGQAPRLLIYGASSRATGIPDRFSGSGSG TDFTLTISRLEPEDFAVYCCQQYESSPWTFGQGT KVEIK | QVQLQQWGAGLLKPSEPLSLTCAVYGGSFSGPYW SWIRQPPGKGLEWIGEINHSGSTNYNPSLKSRVTISV DTSKNQFSLKLTSVTAADTAVYYCARDYGGLDVW GQGTTVTVSS |
| | | | SEQ ID NO: 25960 | SEQ ID NO: 29966 |
| iPS:434899 | 21-225_85B9 | NA | GAAATTGTGTTGACGCAGTCTCCAGGCACCCT GTTTTTGTCTCCAGGGGAGAGAGCCACCCTCT CCTGCAGGGCCAGTCAGAGTGTTAACAGCAAC TACTTAGCCTGGTACCGGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCATTCTCAT CATCAGCAGACTGGAGCCTGAGGATTTTGCAG TGTATTACTGTCAGCAGTATGAAAGCTCGCCG TGGACGTTCGGCCAAGGGACCAAGGTGGAAA TCAAA | CAGGTGCAGCTACAACAGTGGGGCGCAGGACTG TTGAAGCCTTCGGAGACCCTGTCCCTCACTTGCG CTGTCAATGGTGGGCCCTTCAGTGGTTGCTACTG GAGCTGGATCCGCCAGCCCCCAGGGAAGGGGCT GGAGTGGATTGGGGAAATCAATCATAGTGGAAG GACCAACTTCAACCCGTCCCTCAAGAGTCGAGTC ACCATATCAGTTGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
| | | | SEQ ID NO: 25961 | SEQ ID NO: 29967 |

FIGURE 50

| | | | | |
|---|---|---|---|---|
| | | AA | EIVLTQSPGTLFLSPGERATLSCRASQSVNSNYLA WYRQKPGQAPRLLIYGASSRATGIPDRFSGSGSG TDFILIISRLEPEDFAVYYCQQYESSPWTFGQGTK VEIK | QVQLQQWGAGLLKPSETLSLTCAVNGGPFSGCYW SWIRQPPGKGLEWIGEINHSGRTNFNPSLKSRVTISV DTSKNQFSLKLSSVTAADTAVYYCARDYGGMDV WGQGTTVTVSS |
| | | | SEQ ID NO: 25962 | SEQ ID NO: 29968 |
| iPS:434901 | 21-225_85H9 | NA | GACATCGTGATGACCCAGTCTCCAGACTCCCT GGCTGTGTCTCTGGGCGAGAGGGCCACCACCA ATTGTAAGTCCAGCCAGAGTGTTTTACACAGG TCCAACAATTACAACTACTTAGCGTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTGACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAACTGAGCAGCCTGAGATCTG AGGACACGGCCGTGTATTACCGTGGGAGTACCA GTGGCTGGAACTTCTTTGACTACTGGGGCCAGGG AACCCTGGTCACCGTCTCCTCA |
| | | | SEQ ID NO: 25963 | SEQ ID NO: 29969 |
| | | AA | DIVMTQSPDSLAVSLGERATTNCKSSQSVLHRSN NYNYLAWYQQKPGQPPKLLIYWASTRESGVPD RFSGSGSGTDFTLTISSLQAEDVAVYYCQQYYST PPTFGQGTKVEIK | QVQLVQSGAEVKKPGASVKVSCKASGYTLTNYDI NWVRQATGQGLEWMGWMNPNSGNTGYAQKFQG RVTMTRNTSISTAYMELSSLRSEDTAVYYRGSTSG WNFFDYWGQGTLVTVSS |
| | | | SEQ ID NO: 25964 | SEQ ID NO: 29970 |

FIGURE 50

| iPS:434907 | 21-225_85G10 | NA | GAAATTGTGTTGACGCAGTCTCCAGGCTCCCT GTCTTTGTCTCCAGGGGAAAGAGCCACCCTCT CCTGCAGGGCCAGTCAGAGTGTTTGGAGCGGC TACTTAGCCTGGTACCAGCAGAAACCTGGCCA GGCTCCCAGGCTCCTCATCTATGGTGCATCTA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTTCTGTCAGCAGTATGAGAGTTCACCG TGGACGTTCGGCCAAGGGACCAAGGTGGAAA TCAAA | CAGGTACAGCTACAGCAGCGGGGCGCAGGACTG TTGAAGCCTTCGGAGACCCTGTCCCTCACCTGCG CTGTCTATGGTGGGTCCTTCAGTGGTTGTTACTGG AGCTGGATCCGCCAGCCCCCCGGGAAGGGGCTG GAGTGGATTGGGGAAATCAATCATAGTGGAATC ACCAACTACAACCCGTCCCTCAAGAGTCGAGTCA CCATATCAGTAGACACGTCCAAGAACCAGTTCTC CCTGAAGCTGACCTCTGTGACCGCCGCGGACACG GCTGTGTATTACTGTGCGAGAGACTACGGTGGTT TGGACGTCTGGGGCCAAGGGACCACGGTCACCG TCTCCTCA |
| | | | SEQ ID NO: 25965 | SEQ ID NO: 29971 |
| | | AA | EIVLTQSPGSLSLSPGERATLSCRASQSVWSGYL AWYQQKPGQAPRLLIYGASSRATGIPDRFSGSGS GTDFTLTISRLEPEDFAVYFCQQYESSPWTFGQG TKVEIK | QVQLQQRGAGLLKPSETLSLTCAVYGGSFSGCYWS WIRQPPGKGLEWIGEINHSGITNYNPSLKSRVTISVD TSKNQFSLKLTSVTAADTAVYYCARDYGGLDVWG QGTTVTVSS |
| | | | SEQ ID NO: 25966 | SEQ ID NO: 29972 |
| iPS:434909 | 21-225_85C11 | NA | GACATCGTGATGACCCAGTCTCCAGACTCCCT GGCTGTGTCTCTGGGCGAGAGGGCCACCATCA ACTGCAAGTCCAGCCAGAGTGTTTTGTACAGC TCCAACAGTCACAACTTCTTAGCTTGGTACCA GCAGAAACCCAGGACAGCCTCCTAAACTGCTCA TTTTCTGGGCATTTATCCGGGAATCCGGGGTC CCTGAAGGATTCAGTGGCAGCGGGTCTGGGGC AGATTTCACTCTCTCCATCAGCGGCCTACAGG CAGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGAC TTGAGTGGATGGGATGGATGCACCCTAACAGTGG TAAACACAGGCTATGCACAGAAGTTCCAGGGCAG AGTCACCATGACCAGGAACACCTCCATAAGCAC AGCCTACATGGAGCTGAGCAGCCTGAGATCTGA GGACTCGGCCGTATATTACTGTGCGTATAGTAGT GGCTGGTACAAATTTGACTACTGGAGCCAGGGA ACCCTGGTCACCGTCTCCTCA |
| | | | SEQ ID NO: 25967 | SEQ ID NO: 29973 |

FIGURE 50

| | | | | |
|---|---|---|---|---|
| | | AA | DIVMTQSPDSLAVSLGERATINCKSSQSVLYSSN SHNFLAWYQQNPGQPPKLLIFWAFIRESGVPEGF SGSGSGADFTLSISGLQAEDVAVYYCQQYYSTP CSFGQGTKLEIK | QVQLVQSGAEVKKPGASVKVSCKASGYTFTNYDIN WVRQASGQGLEWMGWMHPNSGNTGYAQKFQGR VTMTRNTSISTAYMELSSLRSEDSAVYYCAYSSGW YKFDYWSQGTLVTVSS |
| | | | SEQ ID NO: 25968 | SEQ ID NO: 29974 |
| iPS:434911 | 21-225_85D11 | NA | GAATTTATGTTGACGCAGTCTCCAGGCACCCT GTCTTTGTCTACAGGGGAAAGAGCCACCCTCT CGTGCAGGTCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGGCCAGGGA ACCCTGGTCACCGTCTCCTCA |
| | | | SEQ ID NO: 25969 | SEQ ID NO: 29975 |
| | | AA | EFMLTQSPGTLSLSTGERATLSCRSSQSVSSSYLV WYQQKPGQAPRLLIYGASTRATGIPDRFSGSGSG TDFTLTISRLEPEYFAVYYCQQYGCSPLTFGGGT KVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25970 | SEQ ID NO: 29976 |

FIGURE 50

| iPS:434913 | 21-225_86C1 | NA | GAAATTGTGTTGACGCAGTCTCCAGGCACCCT GTATTTGTCTCCAGGGGAAAGAGCCACCCTCT CCTGCAGGGCCAGTCAGAGTGTTTACAGCAGC TACTTAGCCTGGTACCAGCAGAAACCTGCCCA GGCTCCCAGGCTCCTCATCTATGGTGCATCCA GCCGGGCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
|            |             |    | SEQ ID NO: 25971 | SEQ ID NO: 29977 |
|            |             | AA | EIVLTQSPGTLYLSPGERATLSCRASQSVYSSYLA WYQQKPAQAPRLLIYGASSRATGIPDRFSGSGSG TDFALTISRLEPEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
|            |             |    | SEQ ID NO: 25972 | SEQ ID NO: 29978 |
| iPS:434921 | 21-225_86E4 | NA | GAAATTGTGTTGACGCAGTCTCCAGGCACCCG GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
|            |             |    | SEQ ID NO: 25973 | SEQ ID NO: 29979 |

FIGURE 50

| | | | | |
|---|---|---|---|---|
| | | AA | EIVLTQSPGTRYLSPGERATLSCRASQSVYSSYL AWYQQKPGQAPRLLIYGASSRSTGIPDRFSGSGS GTDFALTISRLEPEDCAVYYCQHSDNSPWTFGQ GTKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25974 | SEQ ID NO: 29980 |
| iPS:434935 | 21-225_86E9 | NA | GACATCGTGATGACCCAGTGTCCAGACTCCCC GGCTGTGTCTCTGGGCGAGAGGGCCACCATCA ATTGCAAGTCCAGCCAGAGTGTTTTGCACAGA TCCAACAATTATAATTACTTAGCTTGGTACCA GCAGAAACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGCTGATGTGGCAGTTTATTACTGTCAGCAA TATCATAGTAGTCCACTGACGTTCGGCCAAGG GACCACGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAGCACCTCCACAAGCA CAGCCCACATGGAGCTGAGCAGCCTGAGATCTG AGGACACGGCCGTGTATTACTGTGCGGTTTCCAG TGGCTGGTCCTGGTTCGACCCCTGGGGGCCAGGGA ACCCTGGTCACCGTCTCCTCA |
| | | | SEQ ID NO: 25975 | SEQ ID NO: 29981 |
| | | AA | DIVMTQCPDSPAVSLGERATINCKSSQSVLHRSN NYNYLAWYQQKPGQPPNLLIYWASTRESGVPD RFSGSGSGTDFTLTISSLQAADVAVYYCQQYHSS PLTFGQGTTVEIK | QVQLVQSGAEVKKPGASVKVSCKASGYTFTNYDIN WVRQATGQGLEWMGWMNPNSGNTGYAQKFQGR VTMTRSTSTSTAHMELSSLRSEDTAVYYCAVSSGW SWFDPWGQGTLVTVSS |
| | | | SEQ ID NO: 25976 | SEQ ID NO: 29982 |

FIGURE 50

| iPS:434939 | 21-225_86C11 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCCTCCCAGACAGGTTCAGTGGCAGTGGGTCTGGGACAGACTTCGCTCTCACCATCAGCAGACTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
|---|---|---|---|---|
|  |  |  | SEQ ID NO: 25977 | SEQ ID NO: 29983 |
|  |  | AA | EIVLTQSPGTLYLSPGERATLSCRASQSVYSSYLAWYQQKPGQAPRLLIYGASSRSTGLPDRFSGSGSGTDFALTISRLEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
|  |  |  | SEQ ID NO: 25978 | SEQ ID NO: 29984 |
| iPS:434943 | 21-225_87H1 | NA | GAAATTGTGTTGACGCAGTCTCCAGGCACCCTGTCTTTGTCTCCAGGGGAAAGAGCCACCCTCTCCTGCAGGGCCAGTCAGAGTGTTGACAGCAACTACTTAGCCTGGTACCAGCAGAAACCTGGCCAGGCTCCCAGGCTCCTCATCTATGGTGCATCTGCCAGGACCACTGGCATCCCAGACAGGTTCAGTGGCAGTGGGTCTGGGACAGACTTCACTCTCACCATCAGCAGACTGGAGCCTGAAGATTTTGCAGTGTATTCCTGTCAGCAGTATGAAAGCTCACCGTGGACGTTCGGCCAAGGGACCAAGGTGGAAATCAAA | CAGGTGCAGCTGCAGCCGTGGGGCGCAGGACTGTTGAAGCCTTCGGAGACCCTGTCCCTCACCTGCGCTGTCTATGGTGGGTCCTTCAGTGGTTACTACTGGAGCTGGATCCGCCAGCCCCCAGGGAAGGGGCTGGAGTGGATTGGGGAAATCAATCATAGTGGACGCACCAACTACAACCCGTCCCTCAAGAGTCGAGTCACCATATCAGTAGACACGTCCAAGGACCAGTTCTCCCTGAAGCTGAGCTCTGTGACCGCCGCGGACACGGCTGTGTATTACTGTGCGAGAGACTACGGTGGTTTGGACGTCTGGGGCCAAGGGACCACGGTCACCGTCTCCTCA |
|  |  |  | SEQ ID NO: 25979 | SEQ ID NO: 29985 |

FIGURE 50

| | | | | |
|---|---|---|---|---|
| | | AA | EIVLTQSPGTLSLSPGERATLSCRASQSVDSNYLA WYQQKPGQAPRLLIYGASARTTGIPDRFSGSGSG TDFTLTISRLEPEDFAVYSCQQYESSPWTFGQGT KVEIK | QVQLQPWGAGLLKPSETLSLTCAVYGGSFSGYYW SWIRQPPGKGLEWIGEINHSGRTNYNPSLKSRVTIS VDTSKDQFSLKLSSVTAADTAVYYCARDYGGLDV WGQGTTVTVSS |
| | | | SEQ ID NO: 25980 | SEQ ID NO: 29986 |
| IPS:434945 | 21-225_87E5 | NA | GAATTTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGAGTGGAGCCTGAAGATTGTGCAG TGTATTACTGTCAGCATTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25981 | SEQ ID NO: 29987 |
| | | AA | EFVLTQSPGTLYLSPGERATLSCRASQSVYSSYL AWYQQKPGQAPRLLIYGASSRSTGIPDRFSGSGS GTDFALTISRVEPEDCAVYYCQHSDNSPWTFGQ GTKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25982 | SEQ ID NO: 29988 |

FIGURE 50

| iPS:434947 | 21-225_87B7 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCACAGTGGGGTCCCATCAAAGTTCAGCGGCA GTGGATCTGGGACAGATTTCACTCTCACCATC AGCAGCCTGCAGCCTGAAGATTCTGCAACTTA TTTCTGCCTACTCTATCTTACTTACCCGCTCAC CTTCGGCCAAGGGACACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATTTTGG AGTGGGCTACTACGGTATGGACGTCTGGGGCCA AGGGACCACGGTCACCGTCTCCTCA |
| | | | SEQ ID NO: 25983 | SEQ ID NO: 29989 |
| | | AA | DIQMTQSPSSLSASVGDRVTITCRASQGISNYLA WFQQKPGKAPKSLIYAASSLHSGVPSKFSGSGSG TDFTLTISSLQPEDSATYFCLLYLTYPLTFGQGTR LEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDFGVG YYGMDVWGQGTTVTVSS |
| | | | SEQ ID NO: 25984 | SEQ ID NO: 29990 |
| iPS:434955 | 21-225_87C9 | NA | GAAATTGTGTTGACGCAGTCTCCAGGCACCCT GTATTTGTCTCCAGTGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCATTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25985 | SEQ ID NO: 29991 |

FIGURE 50

| | | | | |
|---|---|---|---|---|
| | | AA | EIVLTQSPGTLYLSPVERATLSCRASQSVYSSYLA WYQQKPGQAPRLLIYGASSRSTGIPDRFSGSGSG TDFALTISRLEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25986 | SEQ ID NO: 29992 |
| iPS:434957 | 21-225_87A10 | NA | GAATTTGTGTTGACGCAGTCTCCAGGCACCCT GTATTTGTCTCCAGGGGAAAGAGCCACCCTCT CGTGCAGGGCCAGTCAGAGTGTTAGCAGCAGC TACTTAGCCTGGTACCAGCAGAAACCTGGCCA GGCTCCCAGGCTCCTCATCTATGGTGCATCCA CCAGGGCCTCTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTAACTCACCG CTCACTTTCGGCGGAGGGACCAAGGTGGAGAT CAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAATGG TAACACAGGCTATGCACAGAAGTTCCAGGGCAG AGTCACCATGACCAGGAACACCTCCATAAGCAC AGCCTACATGGAGCTGAACAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTTA |
| | | | SEQ ID NO: 25987 | SEQ ID NO: 29993 |
| | | AA | EFVLTQSPGTLYLSPGERATLSCRASQSVSSSYL AWYQQKPGQAPRLLIYGASTRASGIPDRFSGSGS GTDFTLTISRLEPEYFAVYYCQQYGNSPLTFGGG TKVEIK | QVQLVQSGAEVKKPGASVKVSCKASGYTFTNYDIN WVRQATGQGLEWMGWMHPNNGNTGYAQKFQGR VTMTRNTSISTAYMELNSLRSEDTAVYYCAISSGW YWFDPWGQGTLVTVSL |
| | | | SEQ ID NO: 25988 | SEQ ID NO: 29994 |

FIGURE 50

| iPS:434959 | 21-225_87E10 | NA | GACATCGTGATGACCCAGTCTCCAGACTCCCT GGCTGTGTCTCTGGGCGAGAGGGCCACCATCA AGTGCAAGTCCAGCCAGAGTGTTTTACACAGC TCCAACAATATGAACTACTTAGCTTGGTACCA GCAGAAACCTGGACAGCCTCCTAAACTGCTCA TTTACTGGGCATCTACCCGGAAATCCGGGGTC CCTGACCGATTCAGTGGCACCGGGTCTGGGAC AGATTTCACTCTCACCATCAGCAGCCTGCAGG CTGAAGATGTGGCAGTTTATTACTGTCAGCAA TATTATAGTAGTCCGTGCAGTTTTGGCCAGGG GACCAAGCTGAAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCCTATAGCAG TGGCTGGTACTTCTTTGACTACTGGGGCCAGGGA ACCCTGGTCACCGTCTCCTCA |
| | | | SEQ ID NO: 25989 | SEQ ID NO: 29995 |
| | | AA | DIVMTQSPDSLAVSLGERATIKCKSSQSVLHSSN NMNYLAWYQQKPGQPPKLLIYWASTRKSGVPD RFSGTGSGTDFTLTISSLQAEDVAVYYCQQYYSS PCSFGQGTKLKIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAYSSGW YFFDYWGQGTLVTVSS |
| | | | SEQ ID NO: 25990 | SEQ ID NO: 29996 |
| iPS:434961 | 21-225_87A12 | NA | GAAATTGTGTTGACGCAGTCTCCAGGCACCCG GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGTCATCCCAGACAGGTTCAGT GGCAGTGGGTGTGGGACAGACTTCGCTCTCAC CATCAGCAGAGTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25991 | SEQ ID NO: 29997 |

FIGURE 50

| | | | | |
|---|---|---|---|---|
| | | AA | EIVLTQSPGTRYLSPGERATLSCRASQSVYSSYL AWYQQKPGQAPRLLIYGASSRSTVIPDRFSGSGC GTDFALTISRVEPEDCAVYYCQHSDNSPWTFGQ GTKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25992 | SEQ ID NO: 29998 |
| iPS:434965 | 21-225_88A1 | NA | AATATCGTGATGACCCAGTCTCCAGACTCCCT GGCTGTGTCTCTGGGCGCGAGGGCCACCATCA ACTGCAAGTCCAGCCAGAGTGTTTTACACAGC TCCAACAATTACAACTACTTAACTTGGTACCA GCAGAAACCAGGACAGCCTCCTAAGCTGCTCC TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTTCTGTCAGCAA TATTATAGTTCTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGTATAGCAG TGGCTGGTACTACTTTGACTACTGGGGCCAGGGA ACCCTGGTCACCGTCTCCTCA |
| | | | SEQ ID NO: 25993 | SEQ ID NO: 29999 |
| | | AA | NIVMTQSPDSLAVSLGARATINCKSSQSVLHSSN NYNYLTWYQQKPGQPPKLLLYWASTRKSGVPD RFSGSGSGTDFTLTISSLQAEDVAVYFCQQYYSS PPTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAYSSGW YYFDYWGQGTLVTVSS |
| | | | SEQ ID NO: 25994 | SEQ ID NO: 30000 |

FIGURE 50

| iPS:434969 | 21-225_88H1 | NA | GAAATTGTGTTGACGCAGTCTCCAGGCACCCT GTATTTGTCTCCAGGGGAAAGAGCCACCCTGT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACGGTCATCCCAGACAGGTTCAGT GGCAGTGGGTGTGGGACAGACTTCGCTCTCAC CATCAGCAGAGTGGAGCCTGAAGATTGTGCAG TGTATTACTGTCAGCATTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 25995 | SEQ ID NO: 30001 |
| | | AA | EIVLTQSPGTLYLSPGERATLSCRASQSVYSSYLA WYQQKPGQAPRLLIYGASSRSTVIPDRFSGSGCG TDFALTISRVEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25996 | SEQ ID NO: 30002 |
| iPS:434971 | 21-225_88G2 | NA | GACATCGTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCAA TATTATAATACTCCGTGGACGTTTGTCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTCA |
| | | | SEQ ID NO: 25997 | SEQ ID NO: 30003 |

FIGURE 50

| | | | | |
|---|---|---|---|---|
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQQYYNT PWTFVQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 25998 | SEQ ID NO: 30004 |
| iPS:434973 | 21-225_88B4 | NA | GACATCGTGATGACCCAGTCTCCAGACTCCCT GGCTGTGTCTCTGGGCGAGAGGGCCACCATCA ACTGCAAGTCCAGCCAGAGTGTTTTATACATC TCCAACAATAATAATTACTTAGCTTGGTACCA GCAGAAACCAGGACAGCCTCCTAAACTGCTCA TTTACTGGGCATCTACCCGGGAATCCGGGGTC CCTGCCCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTGACACAGGCTATGCACAGAAGTTCCAGGGCA GTGTCACCATGACCAGGAACACCTCCATAACCAC AGCCTACATGGAGCTGAGCAGCCTGAGATCTGA GGACACGGCCGTGTATTACTGTTCGTATAGCAGT GGCTGGTACTACTTTGACTACTGGGGCCAGGGAA CCCTGGTCACCGTCTCCTCA |
| | | | SEQ ID NO: 25999 | SEQ ID NO: 30005 |
| | | AA | DIVMTQSPDSLAVSLGERATINCKSSQSVLYISN NNNYLAWYQQKPGQPPKLLIYWASTRESGVPA RFSGSGSGTDFTLTISSLQAEDVAVYYCQQYYST PPTFGQGTKVEIK | QVQLVQSGAEVKKPGASVKVSCKASGYTFTNYDIN WVRQATGQGLEWMGWMNPNSGDTGYAQKFQGS VTMTRNTSITTAYMELSSLRSEDTAVYYCSYSSGW YYFDYWGQGTLVTVSS |
| | | | SEQ ID NO: 26000 | SEQ ID NO: 30006 |

FIGURE 50

| iPS:434977 | 21-225_88A5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATGATTACCCATTC ACTTTCGGCCCTGGGACCAAAGTGGAAATCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCTGGAACACCTCCATACGCAC TGCCTACATGGAGCTGAGCAGCCTGAGATCTGAG GACACGGCCGTGTATTACTGTGCGCGAGGGTTTT ACGATTTTTTGACTGGTTATTCCCCCACCTACTAC TACTACGATATGGACGTCTGGGGCCAAGGGACC ACGGTCACCGTCTCCTCA |
| | | | SEQ ID NO: 26001 | SEQ ID NO: 30007 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNDYPFTFGPGT KVEIK | QVQLVQSGAEVKKPGASVKVSCKASGYTFTSYDIN WVRQATGQGLEWMGWMNPNSGNTGYAQKFQGR VTMTWNTSIRTAYMELSSLRSEDTAVYYCARGFYD FLTGYSPTYYYYDMDVWGQGTTVTVSS |
| | | | SEQ ID NO: 26002 | SEQ ID NO: 30008 |
| iPS:434981 | 21-225_88E7 | NA | GAAATTGTGTTGACGCAGTCTCCAGGCACCCT GTCTTTGTCTCCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGAGTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |

FIGURE 50

| | | | SEQ ID NO: 26003 | SEQ ID NO: 30009 |
|---|---|---|---|---|
| | | AA | EIVLTQSPGTLSLSPGERATLSCRASQSVYSSYLA WYQQKPGQAPRLLIYGASSRSTGIPDRFSGSGSG TDFALTISRVEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26004 | SEQ ID NO: 30010 |
| iPS:434983 | 21-225_88F7 | NA | GAAATTGTGTTGACGCAGTCTCCAGGCACCCT GTCTTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26005 | SEQ ID NO: 30011 |
| | | AA | EIVLTQSPGTLSLSPGERATLSCRASQSVYSSYLA WYQQKPGQAPRLLIYGASSRSTGIPDRFSGSGSG TDFALTISRLEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26006 | SEQ ID NO: 30012 |

FIGURE 50

| | | | | |
|---|---|---|---|---|
| iPS:434995 | 21-225_88G9 | NA | GAAATTGTGTTGACGCAGTCTCCAGGCACCCG GTATTTGTCTCCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGAGTGGAGCCTGAAGATTGTGCAG TGTATTACTGTCAGCACTCTGATAACTCACCGT GGACGTTCGGCCAAGGGACCAAGGTGGAAAT CAAA<br><br>SEQ ID NO: 26007 | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA<br><br>SEQ ID NO: 30013 |
| | | AA | EIVLTQSPGTRYLSPGERATLSCRASQSVYSSYL AWYQQKPGQAPRLLIYGASSRSTGIPDRFSGSGS GTDFALTISRVEPEDCAVYYCQHSDNSPWTFGQ GTKVEIK<br><br>SEQ ID NO: 26008 | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS<br><br>SEQ ID NO: 30014 |
| iPS:434997 | 21-225_88C10 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATTGGAATTACTTAGCTTGGCACCA GCAGAAACCAGGACAGCCTCCTAAGTTGCTCA TTCACTGGGCATTTACTCGGAAATCCGGGGTC CCTGACCGATTCAGTGGCGGCGGGTCTGGGAC AAATTTCACTCTCACCATCAGCAGCCTGCAGG CTGAAGATGTGGCAGTTTATTACTGTCAGCAA TATTATAGAGCTCCTCCGACGTTCGGCCAAGG GACCAAGGTGGAAATCAAA<br><br>SEQ ID NO: 26009 | CAGGTGCAGCTGGTGCAGTCTGGGGCTGAGGTG AAGAAGCCTGGGGCCTCAGTGAGGGTCTCCTGC AAGGCTTCTGGATACACCTTCAGCAATTATGATA TCAACTGGGTGCGACAGGCCACTGGACAAGGGC TTGAGTGGATGGGATGGATGACCCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGAGTAGCAGT GGCTGGTACTACTTTGACTCCTGGGGCCAGGGAA CCCTGGTCACCGTCTCCTTA<br><br>SEQ ID NO: 30015 |

FIGURE 50

| | | | | |
|---|---|---|---|---|
| | | AA | DIVMTQSPDSLAVSLGERATINCKSSQSVLHSSN NWNYLAWHQQKPGQPPKLLIHWAFTRKSGVPD RFSGGGSGTNFTLTISSLQAEDVAVYYCQQYYR APPTFGQGTKVEIK | QVQLVQSGAEVKKPGASVRVSCKASGYTFSNYDIN WVRQATGQGLEWMGWMTPNSGNTGYAQKFQGR VTMTRNTSISTAYMELSSLRSEDTAVYYCASSSGW YYFDSWGQGTLVTVSL |
| | | | SEQ ID NO: 26010 | SEQ ID NO: 30016 |
| IPS:434999 | 21-225_75A8 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTCTGGGACAGACTTCGCTCTCAC CATCAGCAGAGTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC ATGGACGTCTGGGGCCAAGGGACCACGGTCACC GTCTCCTCA |
| | | | SEQ ID NO: 26011 | SEQ ID NO: 30017 |
| | | AA | EIVLTQSPGTLYLSPGERATLSCRASQSVYSSYLA WYQQKPGQAPRLLIYGASSRATGIPDRFSGSGSG TDFALTISRVEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26012 | SEQ ID NO: 30018 |

FIGURE 50

| iPS:435009 | 21-225_89G4 | NA | GATATTGTGATGACTCAGTCTCCACTCTCCCTG CCCGTCACCCCTGGAGAGCCGGCCTCCATCTC CTGCAGGTCTAGTCAGAGCCTCCTGCATAGTA GTGGATACAACTATTTGGATTGGTACCTGCAG AAGCCAGGGCAGTCTCCACAGCTCCTGATCTA TTTGGGTTCTAATCGGGCCTCCGGGGTCCCTG ACAGGTTCAGTGGCAGTGGTTCAGGCACAGAT TTTACACTGAAAATCAGCAGAGTGGAGGCTGA GGATATTGGGGTTTATTACTGCATGCAAGCTC TACATATTCCTCTCACTTTCGGCGGAGGGACC AAGGTGGAGATCAAA | GAGGTGCAGCTGGTGGAGTCTGGGGGAGGCTTG GTACAGCCGGGGGGGTCCCTGAGACTCTCCTGTG CAGCCTCTGGATTCACCTTCAGTAGCTACGACAT GCACTGGGTCCGCCAAGCTACAGGAAAAGGTCT GGAGTGGGTCTCAGCTATTGGTACTGCTGGTGAC ACATACTATCCAGGCTCCGTGAAGGGCCGATTCA CCATCTCCAGAGAAAATGCCAAGAACTCCTTGTA TCTTCAAATGAACAGCCTGAGAGCCGGGGACAC GGCTGTGTATTTCTGTGCAAGAGCTCTTGACTAC GGTGACTCCTTGGGCTACTACTACTACGGTATGG ACGTCTGGGGCCAAGGGACCACGGTCACCGTCTC CTCA |
| | | | SEQ ID NO: 26013 | SEQ ID NO: 30019 |
| | | AA | DIVMTQSPLSLPVTPGEPASISCRSSQSLLHSSGY NYLDWYLQKPGQSPQLLIYLGSNRASGVPDRFS GSGSGTDFTLKISRVEAEDIGVYYCMQALHIPLT FGGGTKVEIK | EVQLVESGGGLVQPGGSLRLSCAASGFTFSSYDMH WVRQATGKGLEWVSAIGTAGDTYYPGSVKGRFTIS RENAKNSLYLQMNSLRAGDTAVYFCARALDYGDS LGYYYYGMDVWGQGTTVTVSS |
| | | | SEQ ID NO: 26014 | SEQ ID NO: 30020 |
| iPS:435013 | 21-225_89D5 | NA | GAAATTGTGTTGACGCAGTCTCCAGGCACCCT GTATTTGTCTTCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26015 | SEQ ID NO: 30021 |

FIGURE 50

| | | | | | |
|---|---|---|---|---|---|
| | | AA | EIVLTQSPGTLYLSSGERATLSCRASQSVYSSYLA WYQQKPGQAPRLLIYGASSRSTGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS | |
| | | | SEQ ID NO: 26016 | SEQ ID NO: 30022 | |
| IPS:435015 | 21-225_89H5 | NA | GAAATTGTGTTGACGCAGTCTCCAGGCACCCT GTCTTTGTCTCCAGGGGAAAGAGCCACCCTCT CCTGCAGGGCCAGTCAGAGTGTTAACAGCAAC TACTTAGCCTGGTACCAGCAGAAACCTGGCCA GGCTCCCAGGCTCCTCATCTATGGTGCATTCA GCAGGGCCACTGGCATCCCAGACAGGGTCAGT GGCAGTGGGTCTGGGACAGACTTCAATCTCAT CATCAGCAGACTGGAGCCTGAAGATTTTGCAG TGTATTACTGTCAGCAGTATGAAAGCTCAGTG TGGACGTTCGGCCAAGGGACCAAGGTGGAAA TCAAA | CAGGTGCAGCTACAACAGTGGGGCGCAGGACTG TTGAAGCCTTCGGAGACCCTGTCCCTCACTTGCG CTGTCTATGGTGGGTCCTTCAGTGGTTGCTACTG GAGCTGGATCCGCCAGCCCCCAGGGAAGGGGCT GGAGTGGATTGGGGAAATCAATTATAGTGGAAG CACCAACTTCAACCCGTCCCTCAAGAGTCGAGTC ACCATATCAGCTGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA | |
| | | | SEQ ID NO: 26017 | SEQ ID NO: 30023 | |
| | | AA | EIVLTQSPGTLSLSPGERATLSCRASQSVNSNYLA WYQQKPGQAPRLLIYGAFSRATGIPDRVSGSGS GTDFNLIISRLEPEDFAVYYCQQYESSVWTFGQG TKVEIK | QVQLQQWGAGLLKPSETLSLTCAVYGGSFSGCYW SWIRQPPGKGLEWIGEINYSGSTNFNPSLKSRVTISA DTSKNQFSLKLSSVTAADTAVYYCARDYGGMDV WGQGTTVTVSS | |
| | | | SEQ ID NO: 26018 | SEQ ID NO: 30024 | |

FIGURE 50

| iPS:435025 | 21-225_89E10 | NA | GAAATTGTGTTGACGCAGTCTCCAGGCACCCT GTATTTGTCTTCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGTCATCCCAGACAGGTTCAGT GGCAGTGGGTCTGGGACAGACTTCGCTCTCAC CATCAGCAGAGTGGAGCCTGAAGATTTTGCAG TGTATTACTGTCAGCATTCTGATAACTCTCCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
|---|---|---|---|---|
| | | | SEQ ID NO: 26019 | SEQ ID NO: 30025 |
| | | AA | EIVLTQSPGTLYLSSGERATLSCRASQSVYSSYLA WYQQKPGQAPRLLIYGASSRSTVIPDRFSGSGSG TDFALTISRVEPEDFAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26020 | SEQ ID NO: 30026 |
| iPS:435029 | 21-225_89A11 | NA | GAAATTGTGTTGACGCAGTCTCCAGGCACCCT GTCTTTGTCTCCAGGGGAAAGAGCCACCCTCT CCTGCAGGGCCAGTCAGAGTGTTGACAGCAAC TTCTTAGCCTGGTACCAGCAGAAACCTGGCCA GGCTCCCAGGCTCCTCATCTATGGTGCATCTG CCAGGACCACTGGCATCCCAGACAGGTTCAGT GGCAGTGGGTCTGGGACAGACTTCACTCTCAC CATCAGCAGACTGGAGCCTGAAGATTTTGCAG TGTATTCCTGTCAGCAGTATGAAATCTCACCG TGGACGTTCGGCCAAGGGACCAAGGTGGAAA TCAAA | CAGGTGCAGCTGCAGCCGTGGGGCGCAGGACTG TTGAAGCCTTCGGAGACCCTGTCCCTCACCTGCG CTGTCTATGGTGGGTCCTTCAGTGGTTACTACTG GAGCTGGATCCGCCAGCCCCCAGGGAAGGGGCT GGAGTGGATTGGGGAAATCAATCATAGTGGACG CACCAGCTACAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTGTATTACTGTGCGAGAGACTACGGTGGT TTGGACGTCTGGGGCCAAGGGACCACGGTCACC GTCTCCTCA |
| | | | SEQ ID NO: 26021 | SEQ ID NO: 30027 |

FIGURE 50

| | | | | |
|---|---|---|---|---|
| | | AA | EIVLTQSPGTLSLSPGERATLSCRASQSVDSNFLA WYQQKPGQAPRLLIYGASARTTGIPDRFSGSGSG TDFTLTISRLEPEDFAVYSCQQYEISPWTFGQGT KVEIK | QVQLQPWGAGLLKPSETLSLTCAVYGGSFSGYYW SWIRQPPGKGLEWIGEINHSGRTSYNPSLKSRVTISV DTSKNQFSLKLSSVTAADTAVYYCARDYGGLDVW GQGTTVTVSS |
| | | | SEQ ID NO: 26022 | SEQ ID NO: 30028 |
| IPS:435039 | 21-225_90G4 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGTCATCCCAGACAGGTTCAGT GGCAGTGGGTCTGGGACAGACTTCGCTCTCAC CATCAGCAGAGTGGAGCCTGAAGATTGTGCAG TGTTACTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC ATGGACGTCTGGGGCCAAGGGACCACGGTCACC GTCTCCTCA |
| | | | SEQ ID NO: 26023 | SEQ ID NO: 30029 |
| | | AA | EIVLTQSPGTLYLSPGERATLSCRASQSVYSSYLA WYQQKPGQAPRLLIYGASSRSTVIPDRFSGSGSG TDFALTISRVEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26024 | SEQ ID NO: 30030 |

FIGURE 50

| iPS:435041 | 21-225_90A5 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTCTGGGACAGACTTCGCTCTCAC CATCAGCAGAGTGGAGCATGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26025 | SEQ ID NO: 30031 |
| | | AA | EIVLTQSPGTLYLSPGERATLSCRASQSVYSSYLA WYQQKPGQAPRLLIYGASSRATGIPDRFSGSGSG TDFALTISRVEHEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26026 | SEQ ID NO: 30032 |
| iPS:435043 | 21-225_90G5 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCATCATTTATGGTGCATCCA GCCGGGCCACTGGCATCCCAGACAGGTTCAGT GGCAGTGGGTCTGGGACAGACTTCGCTCTCAC CATCAGCAGAGTGGAGCATGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26027 | SEQ ID NO: 30033 |

FIGURE 50

| | | | | |
|---|---|---|---|---|
| | | AA | EIVLTQSPGTLYLSPGERATLSCRASQSVYSSYLA WYQQKPGQAPRLIIYGASSRATGIPDRFSGSGSG TDFALTISRVEHEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26028 | SEQ ID NO: 30034 |
| IPS:435045 | 21-225_90H5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATATTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGATC ACCTTCGGCCAAGGGACACGACTGGAGATTAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGTCTCCAGGCAAGGGGCT GGAGTGGGTGGCAGTTATATGGTATGAAGGAAG TAATACATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGTGAGAGAGATGGG GTGGTTAGATGACTACTGGGGCCAGGGAACCCT GGTCACCGTCTCCTCA |
| | | | SEQ ID NO: 26029 | SEQ ID NO: 30035 |
| | | AA | DIQMTQSPSSLSASVGDRVTITCRASQGIRNDLG WYQQKPGKAPKRLIYIASSLQSGVPSRFSGSGSG TEFTLTISSLQPEDFATYYCLQHNSYPITFGQGTR LEIK | QVQLVESGGGVVQPGRSLRLSCAASGFTFSDYGM HWVRQSPGKGLEWVAVIWYEGSNTYYADSVKGR FTISRDNSKNTLYLQMNSLRAEDTAVYYCVREMG WLDDYWGQGTLVTVSS |
| | | | SEQ ID NO: 26030 | SEQ ID NO: 30036 |

FIGURE 50

| iPS:435051 | 21-225_90D9 | NA | GACATCGTGATGACCCAGTCTCCAGACTCCCT GGCTGTGTCTCTGGGCGAGAGGGCCACCATCA TCTGCAAGTCCAGCCAGACTGTTTTACACAGC TCCAACAATTATAACTACTTAGCTTGGTACCA GCAGAAGCCAGGACAGCCCCCTAAGGTGCTC ATTTACTGGACATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTACTGTCAGCA ATATCTTAGTAGTCCTCTGACGTTCGGCCAAG GGACCAAGGTGGAAATCAAA | CAGGTGCAGCTGGTGCAGTCTGGGGGCTGAGGTG AAGAAGCCTGGGGGCCTCAGTGAAGGTCTCCTGC AAGGCTTCTGGATACACCTTCCCCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTATTGTGCGGTTTCCAG TGGCTGGCACTGGTTCGACCCCTGGGGCCAGGGA ACCCTGGTCACCGTCGCCTCA |
| | | | SEQ ID NO: 26031 | SEQ ID NO: 30037 |
| | | AA | DIVMTQSPDSLAVSLGERATIICKSSQTVLHSSNN YNYLAWYQQKPGQPPKVLIYWTSTRESGVPDRF SGSGSGTDFTLTISSLQAEDVAVYYCQQYLSSPL TFGQGTKVEIK | QVQLVQSGAEVKKPGASVKVSCKASGYTFPNYDIN WVRQATGQGLEWMGWMNPNSGNTGYAQKFQGR VTMTRNTSISTAYMELSSLRSEDTAVYYCAVSSGW HWFDPWGQGTLVTVAS |
| | | | SEQ ID NO: 26032 | SEQ ID NO: 30038 |
| iPS:435053 | 21-225_75F9 | NA | GACATCGTGATGACCCAGTCTCCAGACTCCCT GCCTGTGTCTCTGGGCGAGAGGGCCACCGTCA ACTGCAAGTCCAGCCAGAGTGTTTTACACAAC TCCAACAATAATAACTACTTGGCTTGGTACCA GCAGAAACCAGGACAGCCTCCTAAGCTGCTCA TTTACTGGGCATCTACGCGGGAGTCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAACAA TATTATAGTAGTCCTCCGACGTTCGGCCAAGG GACCAAGGTGGAAATCAAA | CAGGTGCAGCTGGTGCAGTCTGGGGGCTGAGGTG AAGAAGCCTGGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCTATAGCAGT GGCTGGTACATCTTGACTACTGGGGCCAGGGAA CCCTGGTCACCGTCTCCTCA |
| | | | SEQ ID NO: 26033 | SEQ ID NO: 30039 |

FIGURE 50

| | | | | |
|---|---|---|---|---|
| | | AA | DIVMTQSPDSLPVSLGERATVNCKSSQSVLHNSN NNNYLAWYQQKPGQPPKLLIYWASTRESGVPD RFSGSGSGTDFTLTISSLQAEDVAVYYCQQYYSS PPTFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YIFDYWGQGTLVTVSS |
| | | | SEQ ID NO: 26034 | SEQ ID NO: 30040 |
| IPS:435055 | 21-225_90F10 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26035 | SEQ ID NO: 30041 |
| | | AA | EIVLTQSPGTLYLSPGERATLSCRASQSVYSSYLA WYQQKPGQAPRLLIYGASSRSTGIPDRFSGSGSG TDFALTISRLEPEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26036 | SEQ ID NO: 30042 |

FIGURE 50

| iPS:435059 | 21-225_90C11 | NA | GATATTGTGATGACTCAGTCTCCACTCTCCCTG CCCGTCACCCCTGGAGAGCCGGCCTCCATCTC ATGCAGGTATAGTCAGAGCCTCGTGCATAGTA GTGGATACAACTATTTGGATTGGTACCTGCAG AAGCCAGGGCAGTCTCCACAGCTCGTTATCTA TTTGGGTTCTAATCGGGCCTCCGGGGTCCCTG ACAGGTTCAGTGGCAGTGGATCAGGTTCAGAT TTTACACTGAAGATCAGCAGAGTGGAGGCTGA GGATGTTGGACTTTATTACTGCATGCAAGCTC TACACCCTCCTCTCACTTTCGGCGGAGGGACC AAGGTGGAGATCAAA | GAGGTGCAGCTGGTGGAGTCTGGGGGAGGCTTG GTACAGCCGGGGGGGTCCCTGAGACTCTCCTGTG CAGCCTCTGGATTCACCTTCAGTAACTACGACAT GCACTGGGTCCGCCAAGCTACAGGAAAAGGTCT GGAGTGGGTCTCAGCTATTGGTACTGCTGGTGAC ACATACTATCCAGGCTCCGTGAAGGGCCGATTCA CCATCTCCAGAGAAAATGCCAAGAACTCCTTGTA TCTTCAAATGAACAGCCTGAGAGCCGGGGACAC GGCTGTGTATTACTGTGCAAGAGTTCTTGACTAC GGTGACTCCTTGGGCTACTACTACTACGGTATGG ACGTCTGGGGCCAAGGGACCACGGTCACCGTCTC CTCA |
| | | | SEQ ID NO: 26037 | SEQ ID NO: 30043 |
| | | AA | DIVMTQSPLSLPVTPGEPASISCRYSQSLVHSSGY NYLDWYLQKPGQSPQLVIYLGSNRASGVPDRFS GSGSGSDFTLKISRVEAEDVGLYYCMQALHPPL TFGGGTKVEIK | EVQLVESGGGLVQPGGSLRLSCAASGFTFSNYDMH WVRQATGKGLEWVSAIGTAGDTYYPGSVKGRFTIS RENAKNSLYLQMNSLRAGDTAVYYCARVLDYGDS LGYYYYGMDVWGQGTTVTVSS |
| | | | SEQ ID NO: 26038 | SEQ ID NO: 30044 |
| iPS:435071 | 21-225_91F1 | NA | GACATCGTGATGACCCAGTCTCCAGATTGCCT GGCTGTGTCTCTGGGCGAGAGGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCACT ATTATAATACTCCGTGGAAGTTTGTCCAAGGG ACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTCA |
| | | | SEQ ID NO: 26039 | SEQ ID NO: 30045 |

FIGURE 50

| | | | | |
|---|---|---|---|---|
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQHYYNT PWKFVQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 26040 | SEQ ID NO: 30046 |
| IPS:435073 | 21-225_91B2 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGAGTGGAGCCTGAAGATTGTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26041 | SEQ ID NO: 30047 |
| | | AA | EIVLTQSPGTLYLSPGERATLSCRASQSVYSSYLA WYQQKPGQAPRLLIYGASSRSTGIPDRFSGSGSG TDFALTISRVEPEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26042 | SEQ ID NO: 30048 |

FIGURE 50

| | | | | |
|---|---|---|---|---|
| iPS:435075 | 21-225_91B3 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGAGTGGAGCATGAAGATTGTGCA GTGTATTACTGTCAGCACTATGATAACTCACC GTGGACGTTCGGCCAAGGGACCAAGGTGGAA ATCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26043 | SEQ ID NO: 30049 |
| | | AA | EIVLTQSPGTLYLSPGERATLSCRASQSVYSSYLA WYQQKPGQAPRLLIYGASSRSTGIPDRFSGSGSG TDFALTISRVEHEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26044 | SEQ ID NO: 30050 |
| iPS:435077 | 21-225_91F3 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGCCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGAGTGGAGCCTGAAGATTGTGCAG TGTATTACTGTCAGCATTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26045 | SEQ ID NO: 30051 |

FIGURE 50

| | | | | |
|---|---|---|---|---|
| | | AA | EIVLTQSPGTLYLSPGERATLSCRASQSVYSSYLA WYQQKPAQAPRLLIYGASSRSTGIPDRFSGSGSG TDFALTISRVEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26046 | SEQ ID NO: 30052 |
| IPS:435079 | 21-225_91B4 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCATCATTTATGGTGCATCCA GCCGGTCCACTGGCATCCCAGACAGGTTCAGT GGCAGTGGGTCTGGGACAGACTTCGCTCTCAC CATCAGCAGAGTGGAGCCTGAAGATTGTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26047 | SEQ ID NO: 30053 |
| | | AA | EIVLTQSPGTLYLSPGERATLSCRASQSVYSSYLA WYQQKPGQAPRLLIYGASSRSTGIPDRFSGSGSG TDFALTISRVEPEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26048 | SEQ ID NO: 30054 |

FIGURE 50

| iPS:435087 | 21-225_91G8 | NA | GACATCGTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCAA TATTATACTACTCCGTGGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTCA |
| | | | SEQ ID NO: 26049 | SEQ ID NO: 30055 |
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQQYYTT PWTFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 26050 | SEQ ID NO: 30056 |
| iPS:435089 | 21-225_91E9 | NA | GAAATTGTGTTGACGCAGTCTCCAGGCACCCT GTATTTGTCTCCAGGGGAAAGAGCCACCCTGT CATGCAGGGCCAGTCAGAGTGTTTACAGCAGC TACTTAGCGTGGTACCAGCAGAAACCTGGCCA GGCTCCCAGGCTCCTCATTTATGGTGCATCCA GCCGGGCCACGGGCATCCCAGACAGGTTCAGT GGCAGTGGGTGTGGGACAGACTTCGCTCTCAC CATCAGCAGAGTGGAGCATGAAGATTGTGCA GTGTATTACTGTCAGCACTCTGATAACTCACC GTGGACGTTCGGCCAAGGGACCAAGGTGGAA ATCAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26051 | SEQ ID NO: 30057 |

FIGURE 50

| | | | | |
|---|---|---|---|---|
| | | AA | EIVLTQSPGTLYLSPGERATLSCRASQSVYSSYLA WYQQKPGQAPRLLIYGASSRATGIPDRFSGSGCG TDFALTISRVEHEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26052 | SEQ ID NO: 30058 |
| IPS:435097 | 21-225_92B1 | NA | GAAATTGTGTTGACGCAGTCTCCAGGCACCCT GTCTTTGTCTCCAGGGGAAAGAGCCACCCTCT CCTGCAGGGCCAGTCAGAGTGTTGGCAGCAAC TACTTAGCCTGGTACCAGCAGAAACGTGGCCA GGCTCCCAGGCTCCTCATCTATGGTGCATCCA GCAGGGCCACTGACATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGAAAGTTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTCCTACTGG AGCTGGATCCGCCAGCCCCCAGGGAAGGGGCTG GAGTGGATTGGGGAAATCAATTATAGGGGGAAGC ACCAACTACAACCCGTCCCTCAAGAGTCGAGTCG CCATATCAGTAGACACGTCCAAGAACCAGTTCTC CCTGAACCTGACCTCTGTGACCGCCGCGGACACG GCTGTGTATTACTGTGCGAGGGACTACGGCGGTT TGGACGTCTGGGGCCAAGGGACCACGGTCACCG TCTCCTCA |
| | | | SEQ ID NO: 26053 | SEQ ID NO: 30059 |
| | | AA | EIVLTQSPGTLSLSPGERATLSCRASQSVGSNYLA WYQQKRGQAPRLLIYGASSRATDIPDRFSGSGSG TDFTLTISRLEPEDFAVYYCQQYESSPWTFGQGT KVEIK | QVQLQQWGAGLLKPSETLSLTCAVYGGSFSGSYW SWIRQPPGKGLEWIGEINYRGSTNYNPSLKSRVAIS VDTSKNQFSLNLTSVTAADTAVYYCARDYGGLDV WGQGTTVTVSS |
| | | | SEQ ID NO: 26054 | SEQ ID NO: 30060 |

FIGURE 50

| iPS:435103 | 21-225_92B2 | NA | GATATTGTGATGACTCAGTCTCCACTCTCCCTC CCTGTCACCCCTGGAGAGCCGGCCTCCATCTC ATGCAGGTCTAGTCAGAGCCTCGTGCATAGTA GTGGATACAACTATTTGGATTGGTACCTGCAG AAGCCAGGGCAGTCTCCACAACTCGTGATCTA TTTGGGTTCTAATCGGGCCTCCGGGGTCCCTG ACAGGTTCAGTGGCAGTGGTTCAGTCACAGAT TTTACACTGAGAATCAGCAGAGTGGAGGCTGA GGATATTGGGATTTATTATTGCATGCAAGCTC TACATATTCCTCTCACTTTCGGCGGAGGGACC AAGGTGGAGATCAAA | GAGGTGCAGCTGGTGGAGTCTGGGGGAGGCTTG GTACAGCCGGGGGGGTCCCTGAGACTCTCCTGTG CAGCCTCTGGATTCACCTTCAGTAACTACGACAT GCACTGGGTCCGCCAAGCTACAGGAAAAGGTCT GGAGTGGGTCTCAGCTATTGGTACTGCTGGTGAC ACATACTATCCAGGCTCCGTGAAGGGCCGATTCA CCATCTCCAGAGAAAATGCCAAGAACTCCTTGTA TCTTCAAATGAACAGCCTGAGAGCCGGGGACAC GGCTGTGTATTTCTGTGCAAGAGCTCTTGACTAC GGTGACTCCTTGGGCTACTACTACTACGGTATGG ACGTCTGGGGCCAAGGGACCACGGTCACCGTCTC CTCA |
|---|---|---|---|---|
| | | | SEQ ID NO: 26055 | SEQ ID NO: 30061 |
| | | AA | DIVMTQSPLSLPVTPGEPASISCRSSQSLVHSSGY NYLDWYLQKPGQSPQLVIYLGSNRASGVPDRFS GSGSVTDFTLRISRVEAEDIGIYYCMQALHIPLTF GGGTKVEIK | EVQLVESGGGLVQPGGSLRLSCAASGFTFSNYDMH WVRQATGKGLEWVSAIGTAGDTYYPGSVKGRFTIS RENAKNSLYLQMNSLRAGDTAVYFCARALDYGDS LGYYYYGMDVWGQGTTVTVSS |
| | | | SEQ ID NO: 26056 | SEQ ID NO: 30062 |
| iPS:435109 | 21-225_92H5 | NA | GAAATAGTGATGACGCAGTCTCCAGCCACCCT GTCTGTGTCTCCAGGGGAAAGAGCCACCCTCT CCTGCCGGGCCAGTCAGGATGTTATCACCTAC TTAGCCTGGTACCAGCAGAAACCTGGCCAGGC TCCCAGGCTCCTCATCTATGGTGCATCCACCA GGGCCACTGGTGTCCCAGCCAGGTTCAGTGGC AGTGGGTCTGGGACAGAGTTCACTCTCACCAT CACCAGCCTGCCAGTCTGAAGATTTTGCACTTT ATTACTGTCAGGAGTATAATGACTGGCCGTGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCCCTT TATAGTGGGAGCTACTTACTTTGACTACTGGGGC CAGGGAACCCTGGTCACCGTCTCCTCA |
| | | | SEQ ID NO: 26057 | SEQ ID NO: 30063 |

FIGURE 50

| | | | | |
|---|---|---|---|---|
| | | AA | EIVMTQSPATLSVSPGERATLSCRASQDVITYLA WYQQKPGQAPRLLIYGASTRATGVPARFSGSGS GTEFTLTITSLQSEDFALYYCQEYNDWPCSFGQG TKLEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARDPFIV GATYFDYWGQGTLVTVSS |
| | | | SEQ ID NO: 26058 | SEQ ID NO: 30064 |
| IPS:435111 | 21-225_92D6 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGTCATCCCAGACAGGTTCAGT GGCAGTGGGTGTGGGACAGACTTCGCTCTCAC CATCAGCAGACTGGAGCCTGAAGATTGTGCAG TGTATTACTGTCAGCATTATGATAACTCTCCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26059 | SEQ ID NO: 30065 |
| | | AA | EIVLTQSPGTLYLSPGERATLSCRASQSVYSSYLA WYQQKPGQAPRLLIYGASSRSTVIPDRFSGSGCG TDFALTISRLEPEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26060 | SEQ ID NO: 30066 |

FIGURE 50

| iPS:435113 | 21-225_92E6 | NA | GACATCGTGATGACCCAGTCTCCAGACTCCCT GGCTGCGTCTCTGGGCGAGAGGGCCACCATCA ACTGCAAGTCCAGTCAGAATATTTTATCCAGC TCCAACAATAAGAACTACTTAACTTGGTACCA GCAGAAACCAGGACAGCCTCCTAAAATACTCA TTTACTGGACATCTACCCGGGAATCCGGGGTC CCTGACCGATTCAGTGGCAGCGGGTTTGGGAC AGATTTCACTCTCACCATCAGCAGCCTGCAGG CTGAAGATGTGGCTGTTTATTACTGTCAGCAA TATTTTAGTGTTCCTCCGACGTTCGGCCAAGG GACCAAGGTGGAAATCAAA | CAGGTGCAGCTGGTGCAGTCTGGGGCTGAGGTG AAGAAACCTGGGGCCTCAGTGAGGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATGAGCAC AGCCTACATGGAGCTGAGCAGCCTGAGATCTGA GGACACGGCCGTGTATTACTGTGCCCATAGCAGT GGCTGGTACTTTTTTGACTACTGGGGCCAGGGAA CCCTGGTCACCGTCTCCTCA |
|---|---|---|---|---|
| | | | SEQ ID NO: 26061 | SEQ ID NO: 30067 |
| | | AA | DIVMTQSPDSLAASLGERATINCKSSQNILSSSNN KNYLTWYQQKPGQPPKILIYWTSTRESGVPDRFS GSGFGTDFTLTISSLQAEDVAVYYCQQYFSVPPT FGQGTKVEIK | QVQLVQSGAEVKKPGASVRVSCKASGYTFTNYDIN WVRQATGQGLEWMGWMHPNSGNTGYAQKFQGR VTMTRNTSMSTAYMELSSLRSEDTAVYYCAHSSG WYFFDYWGQGTLVTVSS |
| | | | SEQ ID NO: 26062 | SEQ ID NO: 30068 |
| iPS:435115 | 21-225_77C5 | NA | GAAATTGTGTTGACGCAGTCTCCAGGCACCCT GTCTTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26063 | SEQ ID NO: 30069 |

FIGURE 50

| | | | | |
|---|---|---|---|---|
| | | AA | EIVLTQSPGTLSLSPGERATLSCRASQSVYSSYLA WYQQKPGQAPRLLIYGASSRSTGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26064 | SEQ ID NO: 30070 |
| iPS:435167 | 21-225_92F12 | NA | GACATCGTGATGACCCAGTCTCCAGACTCCCT GGCTGTGTCTCTGGGCGAGAGGGCCACCACCA ATTGTAAGTCCAGCCAGAGTGTTTTACACAGG TCCAACAATTACAACTACTTAGCGTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTGACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAACTGAGCAGCCTGAGATCTG AGGACACGGCCGTGTATTACCGTGGGAGTACCA GTGGCGGGAAGTTCTTCGACTACTGGGGCCAGG GAACCCTGGTCACCGTCTCCTCA |
| | | | SEQ ID NO: 26065 | SEQ ID NO: 30071 |
| | | AA | DIVMTQSPDSLAVSLGERATTNCKSSQSVLHRSN NYNYLAWYQQKPGQPPKLLIYWASTRESGVPD RFSGSGSGTDFTLTISSLQAEDVAVYYCQQYYST PPTFGQGTKVEIK | QVQLVQSGAEVKKPGASVKVSCKASGYTLTNYDI NWVRQATGQGLEWMGWMNPNSGNTGYAQKFQG RVTMTRNTSISTAYMELSSLRSEDTAVYYRGSTSG GKFFDYWGQGTLVTVSS |
| | | | SEQ ID NO: 26066 | SEQ ID NO: 30072 |

FIGURE 50

| iPS:435171 | 21-225_93C2 | NA | GAAATTGTGTTGACGCAGTCTCCAGGCACCCT GTATTTGTCTCCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATCTATGGTGCATCCA GCCGGGCCACTGGCATACCAGACAGGTTCAGT GGCAGTGGGTCTGGGACAGACTTCGCTCTCAC CATCAGCAGACTGGAGCATGAAGATTGTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26067 | SEQ ID NO: 30073 |
| | | AA | EIVLTQSPGTLYLSPGERATLSCRASQSVYSSYLA WYQQKPGQAPRLLIYGASSRATGIPDRFSGSGSG TDFALTISRLEHEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26068 | SEQ ID NO: 30074 |
| iPS:435177 | 21-225_93E4 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTGTGGGACAGACTTCGCTCTCAC CATCAGCAGAGTGGAGCCTGAAGATTTTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26069 | SEQ ID NO: 30075 |

FIGURE 50

| | | | | |
|---|---|---|---|---|
| | | AA | EIVLTQSPGTLYLSPGERATLSCRASQSVYSSYLA WYQQKPGQAPRLLIYGASSRSTGIPDRFSGSGCG TDFALTISRVEPEDFAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26070 | SEQ ID NO: 30076 |
| IPS:435183 | 21-225_93E9 | NA | GAAATTGTGTTGACGCAGTCTCCAGGCACCCT GTCTTTGTCTCCAGGGGAAAGAGCCACCCTCT CCTGCAGGGCCAGTCAGAGTGTTGACAGCAGC TACCTAGCCTGGTACCAGCAGAAAACTGGCCA GGCTCCCAGGCTCCTCATCTATGGTGCATCCA GCAGGGCCCCTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGAAAGCTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATCATAGTGGAAG GACCAACTACAACCCGTCCCTCAAGAGTCGAGTC ACCATATCAGTAGACACGTCCGAGAACAAGTTCT CCCTGAAGCTGAGCTCTGTGACCGCCGCGGACAC GGCTGTGTATTACTGTGCGAGAGACTACGGTGGG CTTGACTACTGGGGCCAGGGAACCCTGGTCACCG TCTCCTCA |
| | | | SEQ ID NO: 26071 | SEQ ID NO: 30077 |
| | | AA | EIVLTQSPGTLSLSPGERATLSCRASQSVDSSYLA WYQQKTGQAPRLLIYGASSRAPGIPDRFSGSGSG TDFTLTISRLEPEDFAVYYCQQYESSPWTFGQGT KVEIK | QVQLQQWGAGLLKPSETLSLTCAVYGGSFSGCYW SWIRQPPGKGLEWIGEINHSGRTNYNPSLKSRVTIS VDTSENKFSLKLSSVTAADTAVYYCARDYGGLDY WGQGTLVTVSS |
| | | | SEQ ID NO: 26072 | SEQ ID NO: 30078 |

FIGURE 50

| iPS:435195 | 21-225_94D3 | NA | GAAATTGTGTTGACGCAGTCTCCAGGCACCCT GTCTTTGTCTCAAGGGGAAAGAGCCACCCTCT CCTGCAGGGCCAGTCAGAGTGTTAGCAGCAGG TACTTAGCCTGGTACCAGCAGAAACCTGGCCA GGCTCCCAGACTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TATATTACTGTCAGCATTATGATAGCTCACCGT GGACGTTCGGCCAAGGGACCAAGGTGGAAAA TCAA | CAGGTGCAGCTACAGCAGGGGGGCGCAGGACCG TTGAAGCCTTCGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATTATAGTGGAAG AACCAACTACAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTGTATTACTGTGCGAGAGACTACGGCGGT ATGGACGTCTGGGGCCAAGGGACCACGGTCACC GTCTCCTCA |
| | | | SEQ ID NO: 26073 | SEQ ID NO: 30079 |
| | | AA | EIVLTQSPGTLSLSQGERATLSCRASQSVSSRYLA WYQQKPGQAPRLLIYGASSRATGIPDRFSGSGSG TDFTLTISRLEPEDFAVYYCQHYDSSPWTFGQGT KVENQ | QVQLQQGGAGPLKPSETLSLTCAVYGGSFSGCYWS WIRQPPGKGLEWIGEINYSGRTNYNPSLKSRVTISV DTSKNQFSLKLSSVTAADTAVYYCARDYGGMDV WGQGTTVTVSS |
| | | | SEQ ID NO: 26074 | SEQ ID NO: 30080 |
| iPS:435197 | 21-225_94F3 | NA | GACATCCAGATGACCCAGTCTCCATCCTCCCT GTCTGCATCTGTAGGAGACAGAGTCACCATCA CTTGCCGGGCAAGTCAGGCCATTAGAGATGAT TTAGGCTGGTATCAGCAGAAACCAGGGAAAG CCCCTCAGCGCCTGATCTTTGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTCCAGCATTATAGTTACCCTCGGA CGTTCGGCCGAGGGACCAAGGTGGCAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACGATATCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATTAACAGCCTGAGAGCCGAGG ACACGGCTGTGTATTTCTGTGCGAGAGAAAAATA TAGCAGTGGCTGGTACGACTACGGTATGGACGTC TGGGGCCAAGGGACCACGGTCACCGTCTCCTCA |
| | | | SEQ ID NO: 26075 | SEQ ID NO: 30081 |

FIGURE 50

| | | | | |
|---|---|---|---|---|
| | | AA | DIQMTQSPSSLSASVGDRVTITCRASQAIRDDLG WYQQKPGKAPQRLIFAASSLQSGVPSRFSGSGSG TEFTLTISSLQPEDFATYYCLQHYSYPRTFGRGT KVAIK | QVQLVESGGGVVQPGRSLRLSCAASGFTFSNDIMH WVRQAPGKGLEWVAVIWYDGSNKYYADSVKGRF TISRDNSKNTLYLQINSLRAEDTAVYFCAREKYSSG WYDYGMDVWGQGTTVTVSS |
| | | | SEQ ID NO: 26076 | SEQ ID NO: 30082 |
| iPS:435203 | 21-225_75A7 | NA | GACATCGTGATGACCCAGTCTCCAGACTCCCT GGCTGTGTCTCTGGGCGAGAGGGCCACCATCA ACTGCAAGTCCAGCCAGACTGTTTTACACAGC TCCAACAATTATAACTACTTAGCTTGGTACCA GCAGAAGCCAGGACAGCCCCCTAAGGTGCTC ATTTACTGGACATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTACTGTCAGCA ATATTTTAGTAGTCCTCCGACGTTCGGCCAAG GGACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCCCCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGACA GAGTCACCATGACCAGGAACACCTCCATAAGCA CAGCCTACATGGAGCTGAGCAGCCTGAGATCTG AGGACACGGCCGTGTATTATTGTGCGGTTTCCAG TGGCTGGCACTGGTTCGACCCCTGGGGGCCAGGGA ACCCTGGTCACCGTCGCCTCA |
| | | | SEQ ID NO: 26077 | SEQ ID NO: 30083 |
| | | AA | DIVMTQSPDSLAVSLGERATINCKSSQTVLHSSN NYNYLAWYQQKPGQPPKVLIYWTSTRESGVPD RFSGSGSGTDFTLTISSLQAEDVAVYYCQQYFSS PPTFGQGTKVEIK | QVQLVQSGAEVKKPGASVKVSCKASGYTFPNYDIN WVRQATGQGLEWMGWMNPNSGNTGYAQKFQDR VTMTRNTSISTAYMELSSLRSEDTAVYYCAVSSGW HWFDPWGQGTLVTVAS |
| | | | SEQ ID NO: 26078 | SEQ ID NO: 30084 |

FIGURE 50

| iPS:435209 | 21-225_75A10 | NA | AATATCGTGATGACCCAGTCTCCAGACTCCCT GGCTGTGTCTCTGGGCGCGAGGGCCACCATCA ACTGCAAGTCCAGCCAGAGTGTTTTACACAAC TCCAACAATTACAACTACTTAACTTGGTACCA GCAGAAACCAGGACAGCCTCCTAAGCTGCTCC TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTTCTGTCAGCAA TATTATAGTTCTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGTATAGCAG TGGCTGGTACTACTTTGACTACTGGGGCCAGGGA ACCCTGGTCACCGTCTCCTCA |
|---|---|---|---|---|
| | | | SEQ ID NO: 26079 | SEQ ID NO: 30085 |
| | | AA | NIVMTQSPDSLAVSLGARATINCKSSQSVLHNSN NYNYLTWYQQKPGQPPKLLLYWASTRKSGVPD RFSGSGSGTDFTLTISSLQAEDVAVYFCQQYYSS PPTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAYSSGW YYFDYWGQGTLVTVSS |
| | | | SEQ ID NO: 26080 | SEQ ID NO: 30086 |
| iPS:435211 | 21-225_94E11 | NA | GACATCGTTATGACCCAGTCTCCAGACTCCCT GGCTGTGTCTCTGGGCGAGAGGGGCCACCATCA ATTGCAAGTCCAGCCAGAGTGTTTTATTCAGC TCCAACAATTATAATTACTTAGCTTGGTACCA GCAGAGACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGCTGATGTGGCAGTTTATTACTGTCAGCAA TATCATAGTTCTCCTCTGACGTTCGGCCAAGG GACCACGGTGCAAATCAAA | CAGGTGCAGTTGGTGCAGTCTGGGGCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGAACCCTAACAGTGG TAACACAGGCTATGCACAGAAGTTCCAGGGCAG AGTCACCATGACCAGGAACACCTCCACAAGCAC AGCCCACATGGAGCTGAGCAGCCTGAGATCTGA GGACACGGCCGTGTATTACTGTGCGGTCTCCAGT GGCTGGAAGTGGTTCGACCCCTGGGGCCAGGGA ACCCTGGTCACCGTCTCCTCA |
| | | | SEQ ID NO: 26081 | SEQ ID NO: 30087 |

FIGURE 50

| | | | | |
|---|---|---|---|---|
| | | AA | DIVMTQSPDSLAVSLGERATINCKSSQSVLFSSN NYNYLAWYQQRPGQPPNLLIYWASTRESGVPD RFSGSGSGTDFTLTISSLQAADVAVYYCQQYHSS PLTFGQGTTVQIK | QVQLVQSGAEVKKPGASVKVSCKASGYTFTNYDIN WVRQATGQGLEWMGWMNPNSGNTGYAQKFQGR VTMTRNTSTSTAHMELSSLRSEDTAVYYCAVSSGW KWFDPWGQGTLVTVSS |
| | | | SEQ ID NO: 26082 | SEQ ID NO: 30088 |
| iPS:435215 | 21-225_94E12 | NA | GACATCGTGATGACCCAGTCTCCAGACTCCCT GGCTGTGTCTCTGGGCGAGAGGGCCACCACCA ATTGTAAGTCCAGCCAGAGTGTTTTACACAGG TCCAACAATTACAACTACTTAGCGTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTGACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAACTGAGCAGCCTGAGATCTG AGGACACGGCCGTGTATTACCGTGGGAGTACCA GTGGCTGGAAGTTCTTTGACTACTGGGGCCAGGG AACCCTGGTCACCGTCTCCTCA |
| | | | SEQ ID NO: 26083 | SEQ ID NO: 30089 |
| | | AA | DIVMTQSPDSLAVSLGERATINCKSSQSVLHRSN NYNYLAWYQQKPGQPPKLLIYWASTRESGVPD RFSGSGSGTDFTLTISSLQAEDVAVYYCQQYYST PPTFGQGTKVEIK | QVQLVQSGAEVKKPGASVKVSCKASGYTLTNYDI NWVRQATGQGLEWMGWMNPNSGNTGYAQKFQG RVTMTRNTSISTAYMELSSLRSEDTAVYYRGSTSG WKFFDYWGQGTLVTVSS |
| | | | SEQ ID NO: 26084 | SEQ ID NO: 30090 |

FIGURE 50

| iPS:435217 | 21-225_94F12 | NA | GAAATTGTGTTGACGCAGTCTCCAGGCACCCT GTATTTGTCTCCAGGGGAAAGAGCCACCTTCT CATGCAGGGCCAGTCAGAGTGTTTACAGCAGC TACTTAGCGTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCATTATGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26085 | SEQ ID NO: 30091 |
| | | AA | EIVLTQSPGTLYLSPGERATFSCRASQSVYSSYLA WYQQKPGQAPRLLIYGASSRSTGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26086 | SEQ ID NO: 30092 |
| iPS:435219 | 21-225_95D2 | NA | GAAATTGTGTTGACGCAGTCTCCAGGCACCCT GTCTTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATCTATGGTGCATCCA GCCGGGCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26087 | SEQ ID NO: 30093 |

FIGURE 50

| | | | | |
|---|---|---|---|---|
| | | AA | EIVLTQSPGTLSLSPGERATLSCRASQSVYSSYLA WYQQKPGQAPRLLIYGASSRATGIPDRFSGSGSG TDFALTISRLEPEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26088 | SEQ ID NO: 30094 |
| iPS:435221 | 21-225_95G2 | NA | GAAATAGTGATGACGCAGTCTCCAGCCACCCT GTCTCTGTCTCCAGGGGAAAGAGCCACCCTCT CCTGCAGGGCCAGTATGAGTGTTGTCAACAGC TTAGCCTGGTACCAGCAGAAACCTGGCCAGGC TCCCAGGCTCCTCATCTATGGTGCATCCACCA GGGCCACTGGTATCCCAGCCAGGTTCAGTGGC AGTGGGTCTGGGACAGAGTTCACTCTCACCAT CAGCAGCCTGCAGTCTGAAGATTTTGCAATTT ATTACTGTCAGCAGTATAATGACTGGCCGTGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTA CAGCGTCTGGATTCACCTTCAGTAGCTATGGCAT GCACTGGGTCCGCCAGGCTCCAGGCAAGGGGCT GGAGTGGGTGGCAGTTATTTGGTATGATGGAAGT AATAAATACTATGCAGACTCCGTGAAGGGCCGA TTCACCATCTCCAGAGACAATTCCAAGAACACGC TGTTTCTGCAAATGAACAGCCTGAGAGCCGAGG ACACGGCTGTGTATTACTGTGCGAGCGATCGCAA TATAGTGGGAGCTACTTACTTTGAGTCCTGGGGC CAGGGAACCCTGGTCACCGTCTCCTCA |
| | | | SEQ ID NO: 26089 | SEQ ID NO: 30095 |
| | | AA | EIVMTQSPATLSLSPGERATLSCRASMSVVNSLA WYQQKPGQAPRLLIYGASTRATGIPARFSGSGSG TEFTLTISSLQSEDFAIYYCQQYNDWPCSFGQGT KLEIK | QVQLVESGGGVVQPGRSLRLSCTASGFTFSSYGMH WVRQAPGKGLEWVAVIWYDGSNKYYADSVKGRF TISRDNSKNTLFLQMNSLRAEDTAVYYCASDRNIV GATYFESWGQGTLVTVSS |
| | | | SEQ ID NO: 26090 | SEQ ID NO: 30096 |

FIGURE 50

| iPS:435227 | 21-225_95G4 | NA | GACATCGTTATGACCCAGTGTCCAGACTCCCT GGCTGTGTCTCTGGGCGAGAGGGCCACCATCA ATTGCAAGTCCAGCCAGAGTGTTTTATTCAGA TCCAACAATTATAATTACTTAGCTTGGTACCA GCAGAGACCAGGACAGCCTCATAACCTGCTCA TTTACTGGGCATCTACCCGGGAATCCGGGGTC CCTGACCGATTCAGTGGCAGCGGGTATGGGAC AGATTTCACTCTCACCATCAGCAGCGTGCAGG CTGCTGATGTGGCAGTTTATTACTGTCAGCAA TATCATAGTTCTCCTCTGACGTTCGGCCAAGG GACCACGGTGCAAATCAAA | CAGGTGCAGTTGGTGCAGTCTGGGGGCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGAACCCTAACAGTGG TAACACAGGCTATGCACAGAAGTTCCAGGGCAG AGTCACCATGACCAGGAACACCTCCACAAGCAC AGCCCACATGGAGCTGAGCAGCCTGAGATCTGA GGACACGGCCGTGTATTACTGTGCGGTCTCCAGT GGCTGGAACTGGTTCGACCCCTGGGGCCAGGGA ACCCTGGTCACCGTCTCCTCA |
| | | | SEQ ID NO: 26091 | SEQ ID NO: 30097 |
| | | AA | DIVMTQCPDSLAVSLGERATINCKSSQSVLFRSN NYNYLAWYQQRPGQPHNLLIYWASTRESGVPD RFSGSGYGTDFTLTISSVQAADVAVYYCQQYHS SPLTFGQGTTVQIK | QVQLVQSGAEVKKPGASVKVSCKASGYTFTNYDIN WVRQATGQGLEWMGWMNPNSGNTGYAQKFQGR VTMTRNTSTSTAHMELSSLRSEDTAVYYCAVSSGW NWFDPWGQGTLVTVSS |
| | | | SEQ ID NO: 26092 | SEQ ID NO: 30098 |
| iPS:435235 | 21-225_95F9 | NA | GAAATTGTGTTGACGCAGTCTCCAGGCACCCT GTATTTGTCTCCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTGTGGGACAGACTTCGCTCTCAC CATCAGCAGACTGGAGCCTGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26093 | SEQ ID NO: 30099 |

FIGURE 50

| | | | | |
|---|---|---|---|---|
| | | AA | EIVLTQSPGTLYLSPGERATLSCRASQSVYSSYLA WYQQKPGQAPRLLIYGASSRSTGIPDRFSGSGCG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26094 | SEQ ID NO: 30100 |
| IPS:435237 | 21-225_95G9 | NA | GAAATTGTGTTGACGCAGTCTCCAGGCACCCT GTATTTGTCTTCAGGGGAAAGAGCCACCCTCT CATGCAGGGCCAGTCAGAGTGTTTACAGCAGC TACTTAGCGTGGTACCAGCAGAAACCTGGCCA GGCTCCCAGGCTCCTCATTTATGGTGCATCCA GCCGGTCCACTGGCATCCCAGACAGGTTCAGT GGCAGTGGGTCTGGGACAGACTTCGCTCTCAC CATCAGCAGAGTGGAGCATGAAGATTGTGCA GTGTATTACTGTCAGCACTATGATAACTCACC GTGGACGTTCGGCCAAGGGACCAAGGTGGAA ATCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26095 | SEQ ID NO: 30101 |
| | | AA | EIVLTQSPGTLYLSSGERATLSCRASQSVYSSYLA WYQQKPGQAPRLLIYGASSRSTGIPDRFSGSGSG TDFALTISRVEHEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26096 | SEQ ID NO: 30102 |

FIGURE 50

| iPS:435239 | 21-225_95H10 | NA | GAAATTGTGTTGTCGCAGTCTCCAGGCATCCT GTATTTGTCTTCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTCTGGGACAGACTTCGCTCTCAC CATCAGCAGACTGGAGCCTGAAGATTTTGCAG TGTATTACTGTCAGCACTATGATAACTCTCCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
|---|---|---|---|---|
| | | | SEQ ID NO: 26097 | SEQ ID NO: 30103 |
| | | AA | EIVLSQSPGILYLSSGERATLSCRASQSVYSSYLA WYQQKPGQAPRLLIYGASSRATGIPDRFSGSGSG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26098 | SEQ ID NO: 30104 |
| iPS:435245 | 21-225_95E12 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATGCGAACTACTTAGCTTGGTACCA GCAGAAACCAGGACAGCCTCCTAACCTGTTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGGTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGATTAGCAG TGGCTGGTACTGGTTCGACCCCTGGGGCCAGGGA ACCCTGGTCACCGTCTCCTCA |
| | | | SEQ ID NO: 26099 | SEQ ID NO: 30105 |

FIGURE 50

| | | | | |
|---|---|---|---|---|
| | | AA | DIVMTQSPDSLAVSLGERATINCKSSQSVLYSSN NANYLAWYQQKPGQPPNLFIYWASTRESGVPD RFSGSGSGTDFTLTISSLQAEDVAVYYCQQYYST PCSFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAISSGW YWFDPWGQGTLVTVSS |
| | | | SEQ ID NO: 26100 | SEQ ID NO: 30106 |
| iPS:435247 | 21-225_96G1 | NA | GAAATTGTGTTGACGCAGTCTCCAGGCACCCT GTCTTTGTCTACAGGGGAAAGAGCCACCCTCT CGTGCAGGGCCAGTCAGAGCGTTAGCAGCAG CTACTTAGCTTGGTACCAGCAGAAACCTGGCC AGCCTCCCAGGCTCCTCATTTATGGTGCATCC ACCAGGGCCTCTGGCATCCCAGACAGGTTCAG TGGCAGTGGGTCTGGGACAGACTTCACTCTCA CCATCAGCAGACTGGAGCCTGAATATTTTGCA GTTTATTACTGTCAGCAGTATGGTAACTCACC GCTCACTTTCGGCGGAGGGACCAAGGTGGAG ATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAATGG TAACACAGGCTATGCACAGAAGTTCCAGGGCAG AGTCACCATGACCAGGAACACCTCCATAAGCAC AGCCTACATGGAGCTGAACAGCCTGAGATCTGA GGACACGGCCGTGTATTACTGTGCGATTAGCAGT GGCTGGTACTGGTTCGACCCCTGGGGCCAGGGA ACCCTGGTCACCGTCTCCTTA |
| | | | SEQ ID NO: 26101 | SEQ ID NO: 30107 |
| | | AA | EIVLTQSPGTLSLSTGERATLSCRASQSVSSSYLA WYQQKPGQPPRLLIYGASTRASGIPDRFSGSGSG TDFTLTISRLEPEYFAVYYCQQYGNSPLTFGGGT KVEIK | QVQLVQSGAEVKKPGASVKVSCKASGYTFTNYDIN WVRQATGQGLEWMGWMHPNNGNTGYAQKFQGR VTMTRNTSISTAYMELNSLRSEDTAVYYCAISSGW YWFDPWGQGTLVTVSL |
| | | | SEQ ID NO: 26102 | SEQ ID NO: 30108 |

FIGURE 50

| iPS:435249 | 21-225_96E2 | NA | GACAGCGTGATGACCCAGTCTCCAGACTCCCT GGCTGTGTCTCTGGGCGAGAGGGCCACCATCA ACTGCAAGTCCAGCCAGAGTGTTTTACACAGC TCTAATAAAAAGAACTACTTAGCTTGGTACCA GCAGAAACCAGGACAGCCTCCTAAGCTGCTCA TTTACTGGGCATCTACCTGGGAATCCGGGGTC CCTGACCGATTCAGTGGCAGCGGGTCTGGGAC AGATTTCACTCTCACCATCAGCAGTCTGCAGG CTGAAGATGTGGCAGTTTATTACTGTCAGCAA TATTATAGGACTCCGTGGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAATAGTGG TAACACAGGCTATGCACAGAAGTTCCAGGGCAG AGTCACCCTGACCAGGAACACCTCCATAAGCAC AGCCTACATGGAGCTGAGCAGCCTGAGATCTGA GGACACGGCCGTGTATTACTGTGCGGTTTCCAGT GGCTGGTACTGGTTCGACCCCTGGGGCCAGGGA ACCCTGGTCACCGTCTCCTCA |
|---|---|---|---|---|
| | | | SEQ ID NO: 26103 | SEQ ID NO: 30109 |
| | | AA | DSVMTQSPDSLAVSLGERATINCKSSQSVLHSSN KKNYLAWYQQKPGQPPKLLIYWASTWESGVPD RFSGSGSGTDFTLTISSLQAEDVAVYYCQQYYRT PWTFGQGTKVEIK | QVQLVQSGAEVKKPGASVKVSCKASGYTFTNYDIN WVRQATGQGLEWMGWMHPNSGNTGYAQKFQGR VTLTRNTSISTAYMELSSLRSEDTAVYYCAVSSGW YWFDPWGQGTLVTVSS |
| | | | SEQ ID NO: 26104 | SEQ ID NO: 30110 |
| iPS:435251 | 21-225_96A3 | NA | GACATCCAGATGACCCAGTCTCCATCCTCCCT GTCTGCTTCTGTAGGAGACACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCACTT TGCAAAGTGGGGTCCCATCAAGGTTCAGCGGC AGTGGATCTGGGACAGAATTCACTCTCACAAT CAGCGGCCTGCAGCGTGAAGATTTTGCAACTT ATCACTGTCTACAGCATAGTAATTACCCGCTC ACTTTCGGCGGAGGGACCAAGGTGGAGATCA AA | CAGCTGCAGCTGCAGGAGTCGGGGCCCAGGACTG GTGAAGCCTTCGGAGACCCTGTCCCTCATCTGTA CTGTCTCTGGTGGCTCCATCAGCAGTAGTAATTA CTACTGGGGCTGGATCCGCCAGCCCCCAGGGAA GGGGCTGGAGTGGATTGGGAGTATCTATTATAGT GGATACACCTCCTACAACCCGTCCCTCAAGAGTC GAGTCACCATATCCGTAGACTCGTCCAAGAACCA CTTCTCCCTGAGGCTGAGCTCTGTGACCGCCGCA GACACGGCTGTGTATTACTGTGCGAGACTTGACT CTAACTGGGGTCTTGACTACTGGGGCCAGGGAAC CCTGGTCACCGTCTCCTCA |
| | | | SEQ ID NO: 26105 | SEQ ID NO: 30111 |

FIGURE 50

| | | | | |
|---|---|---|---|---|
| | | AA | DIQMTQSPSSLSASVGDRVTITCRASQGIRNDLG WYQQKPGKAPKRLIYAASTLQSGVPSRFSGSGS GTEFTLTISGLQREDFATYHCLQHSNYPLTFGGG TKVEIK | QLQLQESGPGLVKPSETLSLICTVSGGSISSSNYYW GWIRQPPGKGLEWIGSIYYSGYTSYNPSLKSRVTIS VDSSKNHFSLRLSSVTAADTAVYYCARLDSNWGL DYWGQGTLVTVSS |
| | | | SEQ ID NO: 26106 | SEQ ID NO: 30112 |
| iPS:435253 | 21-225_96A4 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGGGAAAG CCCCTAAGCGCCTGATCTATGGTGTATCCAGT TTGCAAAGTGGGGTCCCATCAAGGTTCAGCGG CAGTGGATCTGGGACCGAATTCACTCTCACAA TCAGCAGCCTGCAGCGTGAAGATTTTGCAACT TATTACTGTCTACAGCATAATGATTACCCATTC ACTTTCGGCCGTGGGACCAAAGTGGATATCAA A | CAGGTGCAGCTGGTGCAGTCTGGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACACGGGC TTGAGTGGATGGGATGGATGAACCCTAACAGTG GTAACACAGGCTATGCACAGAAGTTCCAGGGCA GAGTCACCATGACCTGGAACACCTCCAAAAGCA CAGCCTACATGGAGCTGAGTAGCCTGAGATCTGA GGACACGGCCGTGTATTACTGTGCGAGAGGCTTT TACGATACTTTGACTGGTTCCGGCTACTACTACG TTATGGACGTCTGGGGCCAAGGGACCACGGTCA CCGTCTCCTCA |
| | | | SEQ ID NO: 26107 | SEQ ID NO: 30113 |
| | | AA | DIQMTQSPSSLSASVGDRVTITCRASQDIRNDLG WYQQKPGKAPKRLIYGVSSLQSGVPSRFSGSGS GTEFTLTISSLQREDFATYYCLQHNDYPFTFGRG TKVDIK | QVQLVQSGAEVKKPGASVKVSCKASGYTFTSYDIN WVRQATGHGLEWMGWMNPNSGNTGYAQKFQGR VTMTWNTSKSTAYMELSSLRSEDTAVYYCARGFY DTLTGSGYYYVMDVWGQGTTVTVSS |
| | | | SEQ ID NO: 26108 | SEQ ID NO: 30114 |

FIGURE 50

| iPS:435255 | 21-225_96D5 | NA | GACATCGTGATGACCCAGTCTCCAGACTCCCT GGCTGTGTCTCTGGGCGAGAGGGCCACCATCA ACTGCAAGTCCAGCCAGAGTGTTTTGCACAGC TCCAACAATTATAATTACTTAGCTTGGTACCA GCAGAAACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGCTGATGTGGCAGTTTATTACTGTCAGCAA TATTATAGTAGTCCACCGACGTTCGGCCAAGG GACCACGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAGCACCTCCACAAGCA CAGCCCACATGGAGCTGAGCAGCCTGAGATCTG AGGACACGGCCGTGTATTACTGTGCGGTTTCCAG TGGCTGGTCCTGGTTCGACCCCTGGGGCCAGGGA ACCCTGGTCACCGTCTCCTCA |
| | | | SEQ ID NO: 26109 | SEQ ID NO: 30115 |
| | | AA | DIVMTQSPDSLAVSLGERATINCKSSQSVLHSSN NYNYLAWYQQKPGQPPNLLIYWASTRESGVPD RFSGSGSGTDFTLTISSLQAADVAVYYCQQYYSS PPTFGQGTTVEIK | QVQLVQSGAEVKKPGASVKVSCKASGYTFTNYDIN WVRQATGQGLEWMGWMNPNSGNTGYAQKFQGR VTMTRSTSTSTAHMELSSLRSEDTAVYYCAVSSGW SWFDPWGQGTLVTVSS |
| | | | SEQ ID NO: 26110 | SEQ ID NO: 30116 |
| iPS:435257 | 21-225_96H5 | NA | GACATCGTGATGACCCAGTCTCCAGACTCCCT GGCTGTGTCTCTGGGCGAGAGGGCCACCATCA ACTGCAAGTCCAGCCAGAGTGTTTTGTACAGC TCCAACAGTCACAACTACTTAGCTTGGTACCA GCAGAAACCAGGACAGCCTCCTAAACTGCTCA TTTACTGGGCATCTATCCGGGAATCCGGGGTC CCTGACCGATTCAGTGGCAGCGGGTCTGGGAC AGATTTCACTCTCTCCATCAGCAGCATGCAGG CTGAAGATGTGGCAGTTTATTACTGTCAGCAA TATTATAGTACTCCGTGCAGTTTTGGCCAGGG GACCAAGGTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGAC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACTCGGCCGTATATTACTGTGCGTATAGTAGT GGCTGGTACAAATTTGACTACTGGAGCCAGGGA ACCCTGGTCACCGTCTCCTCA |
| | | | SEQ ID NO: 26111 | SEQ ID NO: 30117 |

FIGURE 50

| | | | | |
|---|---|---|---|---|
| | | AA | DIVMTQSPDSLAVSLGERATINCKSSQSVLYSSN SHNYLAWYQQKPGQPPKLLIYWASIRESGVPDR FSGSGSGTDFTLSISSMQAEDVAVYYCQQYYSTP CSFGQGTKVEIK | QVQLVQSGAEVKKPGASVKVSCKASGYTFTNYDIN WVRQASGQGLEWMGWMHPNSGNTGYAQKFQGR VTMTRNTSISTAYMELSSLRSEDSAVYYCAYSSGW YKFDYWSQGTLVTVSS |
| | | | SEQ ID NO: 26112 | SEQ ID NO: 30118 |
| iPS:435259 | 21-225_96C6 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCTTCCAGTTT GCAAAGTGGGGTCCCCTCAAAGTTCAGCGGCA GTGGATCTGGGACAGATTTCACTCTCACCATC AACAGCCTGCAGCCTGAAGATTTTGCAACTTA TTACTGCCACCAGTATAATGATTACCCATTCA CTTTCGGCCCTGGGACCAAAGTGGATATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAGTTATGATA TCAACTGGGTGCGTCAGGCCACTGGACAAGGGC TTGAGTGGATGGGATGGATGAACCCTAACAGTCG TAACACAGGCTATGCACAGAAGTTCCAGGGCAG AGTCACCATGACCTGGAACACCTCCATAAGCACA GCCTACATGGAGCTGAGCAGCCTGAGATCTGAG GACACGGCCGTGTATTACTGTGCGAGAGGCGGCT ACGATGTTTTGCCTGGGAATAACTACTACTACGA TATGGACGTCTGGGGCCAAGGGACCACGGTCAC CGTCTCCTCA |
| | | | SEQ ID NO: 26113 | SEQ ID NO: 30119 |
| | | AA | DIQMTQSPSSLSASVGDRVTITCRASQGISNYLA WFQQKPGKAPKSLIYAASSLQSGVPSKFSGSGSG TDFTLTINSLQPEDFATYYCHQYNDYPFTFGPGT KVDIK | QVQLVQSGAEVKKPGASVKVSCKASGYTFTSYDIN WVRQATGQGLEWMGWMNPNSRNTGYAQKFQGR VTMTWNTSISTAYMELSSLRSEDTAVYYCARGGY DVLPGNNYYYDMDVWGQGTTVTVSS |
| | | | SEQ ID NO: 26114 | SEQ ID NO: 30120 |

FIGURE 50

| iPS:435267 | 21-225_96D10 | NA | GACATCGTGATGACCCAGTCTCCAGACTCCCTGGCTGTGTCTCTGGGCGAGAGGGCCACCATCAATTGCAAGTCCAGTCAGAATATTTTATCCAGCTCCAACAATAAGAACTACTTAACTTGGTACCAGCAGAAACCAGGACAGCCTCCTAAAATACTCATTTACTGGACATCTACCCGGGAATCCGGGGTCCCTGACCGATTCAGTGGCAGCGGGTTTGGGACAGATTTCACTCTCACCATCAGCAGCCTGCAGGCTGAAGATGTGGCTGTTTATTACTGTCAGCAATATTTTAGTGTTCCTCCGACGTTCGGCCAAGGGACCAAGGTGGAAATCAAA | CAGGTGCAGCTGGTGCAGTCTGGGGGCTGAGGTGAAGAAACCTGGGGCCTCAGTGAGGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATGAGCACAGCCTACATGGAGCTGAGCAGCCTGAGATCTGAGGACACGGCCGTGTATTACTGTGCCCATAGCAGTGGCTGGTACTTTTTTGACTACTGGGGCCAGGGAACCCTGGTCACCGTCTCCTCA |
| | | | SEQ ID NO: 26115 | SEQ ID NO: 30121 |
| | | AA | DIVMTQSPDSLAVSLGERATINCKSSQNILSSSNNKNYLTWYQQKPGQPPKILIYWTSTRESGVPDRFSGSGFGTDFTLTISSLQAEDVAVYYCQQYFSVPPTFGQGTKVEIK | QVQLVQSGAEVKKPGASVRVSCKASGYTFTNYDINWVRQATGQGLEWMGWMHPNSGNTGYAQKFQGRVTMTRNTSMSTAYMELSSLRSEDTAVYYCAHSSGWYFFDYWGQGTLVTVSS |
| | | | SEQ ID NO: 26116 | SEQ ID NO: 30122 |
| iPS:435273 | 21-225_97A2 | NA | GAAATTGTGTTGACGCAGTCTCCAGGCACCCTGTATTTGTCTTCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTGTGGGACAGACTTCGCTCTCACCATCAGCAGACTGGAGCCTGAAGATTGTGCAGTGTATTACTGTCAGCATTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26117 | SEQ ID NO: 30123 |

FIGURE 50

| | | | | |
|---|---|---|---|---|
| | | AA | EIVLTQSPGTLYLSSGERATLSCRASQSVYSSYLA WYQQKPGQAPRLLIYGASSRSTGIPDRFSGSGCG TDFALTISRLEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26118 | SEQ ID NO: 30124 |
| iPS:435279 | 21-225_97H4 | NA | GACATCGTGATGACCCAGTCTCCAGATTGCCT GGCTGTGTCTCTGGGCGAGAGGGCCACCATCA ACTGCAAGTCCAGCCAGAGTGTTTTATACACC TCCAACAATAACAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTTGCAGTTTATTATTGTCAGCACT ACAATGATACTCCATGGAAGTTTGTCCAAGGG ACCAAGGTGGAAATCACA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGATTAGCAGT GGCTGGTACTGGTTCGACCCCTGGGGGCCAGGGA ACCCTGGTCACCGTCTCCTCA |
| | | | SEQ ID NO: 26119 | SEQ ID NO: 30125 |
| | | AA | DIVMTQSPDCLAVSLGERATINCKSSQSVLYTSN NNNYLAWYQQKPGQPPKLLIYWASTRESGVPD RFSGSGSGTDFTLTISSLQAEDVAVYYCQHYNDT PWKFVQGTKVEIT | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 26120 | SEQ ID NO: 30126 |

FIGURE 50

| iPS:435281 | 21-225_97E5 | NA | GAAATTGTGTTGACGCAGTCTCCAGGCACCCT GTCTTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTGTGGGACAGACTTCGCTCTCAC CATCAGCAGAGTGGAGCCTGAAGATTGTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 26121 | SEQ ID NO: 30127 |
| | | AA | EIVLTQSPGTLSLSPGERATLSCRASQSVYSSYLA WYQQKPGQAPRLLIYGASSRATGIPDRFSGSGCG TDFALTISRVEPEDCAVYYCQHSDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 26122 | SEQ ID NO: 30128 |
| iPS:435291 | 21-225_146E1 | NA | GACATCAAGATGACCCAGTCTCCATCTTCCGT GTCTGCATCTGTAGGAGACAGAGTCACCATCA CTTGTCGGGCGAGTCAGGGTATTAACAACTGG TTAGTCTGGTATCAGCAGAAACCAGGGAAAGC CCCTAAGCTCCTGATCTATGCTGCATCCAGTTT GCAAAGTGGGGTCCCATCAAGGTTCCGCGGCA GTGGATCTGGGACAGATTTCACTCTCACCATC AGCAGCCTGCAGCCTGAAGATTTTGCAACTTA CTATTGTCAACAGGCTAACAGTTTCCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG AAGCGTCTGGAATCACCTTCAGTAGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTTTCTGCAAATGAACAGCCTGAGAGCCGAGG ACACGGCTGTGTATTACTGTGCGAGAGATCGTTT AGTGGGAGCTACCGCTGATGCTTTTGATATCTGG GGCCAAGGGACAATGGTCACCGTCTCTTCA |
| | | | SEQ ID NO: 26123 | SEQ ID NO: 30129 |

FIGURE 50

| | | | | |
|---|---|---|---|---|
| | | AA | DIKMTQSPSSVSASVGDRVTITCRASQGINNWLV WYQQKPGKAPKLLIYAASSLQSGVPSRFRGSGS GTDFTLTISSLQPEDFATYYCQQANSFPFTFGPGT KVDIK | QVQLVESGGGVVQPGRSLRLSCEASGITFSSYGMH WVRQAPGKGLEWVAIIWYDGSNKYYADSVKGRFT ISRDNSKNTLFLQMNSLRAEDTAVYYCARDRLVGA TADAFDIWGQGTMVTVSS |
| | | | SEQ ID NO: 26124 | SEQ ID NO: 30130 |
| iPS:435293 | 21-225_146F1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CGGTGGATCTGGGACAGAATTCACTCTCACAA TCAGCAGCCTGCAGCCTGAAGATTTTGCAACT TATTACTGTCTTCAGCATAGTACTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAGTATATATTATAGT GGGAGTACCTCCTACAACCCGTCCCTCAAGAGTC GAGTCACCATATCCGTCGACACGTCCAAGAACCA GTTCTCCCTGAAGCTGAGCTCTGTGACCGCCGCA GACACGGCTGTTTATTACTGTGCGAGACTTGATC TCCTGTGGAGTTTTGACTACTGGGGCCAGGGAAC CCTGGTCACCGTCTCCTCA |
| | | | SEQ ID NO: 26125 | SEQ ID NO: 30131 |
| | | AA | DIQMTQSPSSLSASVGDRVTITCRASQGIRNDLG WYQQKPGKAPKRLIYAASSLQSGVPSRFSGGGS GTEFTLTISSLQPEDFATYYCLQHSTYPLTFGGGT KVEIK | QLQLQESGPGLVKPSETLSLTCTVSGGSISRSSYYW GWIRQPPGKGLEWIGSIYYSGSTSYNPSLKSRVTISV DTSKNQFSLKLSSVTAADTAVYYCARLDLLWSFDY WGQGTLVTVSS |
| | | | SEQ ID NO: 26126 | SEQ ID NO: 30132 |

FIGURE 50

| iPS:435295 | 21-225_146H1 | NA | GACATCCAGATGACCCAGTCTCCATCCTCCCT GTCTGCATCTGTAGGAGACAGAGTCACCATCA CTTGCCGGGCAAGTCAGAGCATTAGCGACTAT TTAAAATTGGTATCAGCAAAAACCAGGGAAAG CCCCTAAGGTCCTGATCTATACTACATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTACCCCCACT TTCGGCGGAGGGACCAAGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACACT ATATCTGCAAATGAACAGCCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26127 | SEQ ID NO: 30133 |
| | | AA | DIQMTQSPSSLSASVGDRVTITCRASQSISDYLN WYQQKPGKAPKVLIYTTSSLQSGVPSRFSGSGSG TDFTLTISSLQPEDFATYYCQQSYSTPTFGGGTK VEIK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKRVTDYG GNDWFDPWGQGTLVTVSS |
| | | | SEQ ID NO: 26128 | SEQ ID NO: 30134 |
| iPS:435297 | 21-225_146B3 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAAGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGCTTTATCACTGCATGCAAAG TATACAGCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT TATAAATACTATGCAGACTCCGTGAAGGGCCGAT TCACCATCTCCAGAGACAATTCCAAGAACACGCT GGATCTGCAAATGAACAGCCTGAGAGCCGAGGA CACGGCTGTGTATTACTGTGTGAAAATGGGTATA GAAGTGGCTGTGGACTACTACTACGGTATGGACG TCTGGGGCCAAGGGACCACGGTCACCGTCTCCTC A |
| | | | SEQ ID NO: 26129 | SEQ ID NO: 30135 |

FIGURE 50

| | | | | |
|---|---|---|---|---|
| | | AA | DIVMTQTPLSLSVTPGQPASISCKSSQSLLHGDG KTYLYWYLQKPGQPPQLLIYEVSNRFSGVPDRFS GSGSGTDFTLKISRVEAEDVGLYHCMQSIQLPW TFGQGTKVEIK | QVQLVESGGGVVQPGRSLRLSCAASGFTFSSYGMH WVRQAPGKGLEWVAVIWYDGSYKYYADSVKGRF TISRDNSKNTLDLQMNSLRAEDTAVYYCVKMGIEV AVDYYYGMDVWGQGTTVTVSS |
| | | | SEQ ID NO: 26130 | SEQ ID NO: 30136 |
| iPS:435299 | 21-225_146D4 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATAACAACTACTTAGCTTGGTACCA GCAGAAACCAGGACAGCCTCCTAAGCTGGTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGGTGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGGGGGAGCAG TGGCTGGTACTACTTTGACTACTGGGGCCAGGGA ACCCTGGTCACCGTCTCCTCA |
| | | | SEQ ID NO: 26131 | SEQ ID NO: 30137 |
| | | AA | DIVMTQSPDSLAVSLGERATINCKSSQSVLYSSN NNNYLAWYQQKPGQPPKLVIYWASTRESGVPD RFSGSGSGTDFTLTISSLQAEDVAVYYCQQYYST PCSFGQGTKLEIK | QVQLVQSGAEVKKPGASVKVSCKASGYTFTNYDIN WVRQATGQGLEWMGWVHPNSGNTGYAQKFQGR VTMTRNTSISTAYMELSSLRSEDTAVYYCAGSSGW YYFDYWGQGTLVTVSS |
| | | | SEQ ID NO: 26132 | SEQ ID NO: 30138 |

FIGURE 50

| iPS:435301 | 21-225_146G4 | NA | GAAATTGTATTGACGCAGTCTCCAGGCACCCT GTCTTTATTTCCAGGGGAAAGAGCCACCCTCT CCTGCAGGGCCAGTCAGAATATTATCAGCAGC TATTTAGCCTGGTACCAGCAGAAACCTGGCCA GGCTCCCAGGCTCCTCATCTTTGGTGTATCTAG TCGGGCCACTGGCATCCCAGACAGGTTCAGTG GCAGTGGGTCTGGGACAGACTTCACTCTCACC ATCAGCAGACTGGAGCCTGAAGATTTTGCAGT GTATTACTGTCAACAATATGGTAGGTCACCAT TCAATTTCGGCCCTGGGACCAAAGTGGATATC AAC | CAGGTGCAGCTGCAGGAGTCGGGCCCAGGACTG GTGAAGCCTTCACAGACCCTGTCCCTCAATTGCA CTGTCTCTGGTGGCTCCATCAGCAACAGTGGTTA CTACTGGAGCTGGATCCGCCAGCACCCAGGGAA GGGGCCTGGAATGGATTGGGTACAGCTATTACAGT GGGAGCACCTACTACAACCCGTCCCTCAAGAGTC GAATTACCATATCAGTAGACACGTCTAACAACCA GTTCTCCCTGAAGCTGACCTCTGTGACTGCCGCG GACACGGCCGTGTATTACTGTGCGAGAGGGAAA TATAACTGGAACCATGCTTTTGATATCTGGGGCC AAGGGACAATGGTCACCGTCTCTTCA |
| | | | SEQ ID NO: 26133 | SEQ ID NO: 30139 |
| | | AA | EIVLTQSPGTLSLFPGERATLSCRASQNIISSYLA WYQQKPGQAPRLLIFGVSSRATGIPDRFSGSGSG TDFTLTISRLEPEDFAVYYCQQYGRSPFNFGPGT KVDIN | QVQLQESGPGLVKPSQTLSLNCTVSGGSISNSGYY WSWIRQHPGKGLEWIGYSYYSGSTYYNPSLKSRITI SVDTSNNQFSLKLTSVTAADTAVYYCARGKYNWN HAFDIWGQGTMVTVSS |
| | | | SEQ ID NO: 26134 | SEQ ID NO: 30140 |
| iPS:435303 | 21-225_146A6 | NA | GACATCCAGATGACCCAGTCTCCAGCTTCCGT GTCTGCATCTGTGGGAGACAGAGTCACCATAA CTTGTCGGGCGAGTCAGGGTATTAGCAACTGG TTAGCCTGGTATCAGCAGAAACCAGGGAAAG CCCCTAAACTCCTGATCTATGCTGCATCCAGTT TGCAAGGTGGGGTCCCATCAAGGTTCAGCGGC AGTGGATCTGGGACAGATTTCACTCTCTCCAT CAGCAGCCTGCAGCCTGAAGATTTTGCAACTT ACTATTGTCAACAGACTGACAGTTTCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAACAGCTATGCCAT GAACTGGGTCCGCCAGGCTCCAGGGAAGGGGCT GGAGTGGGTCTCAGCTATTAGTGGTAGTGGTGGT AACACATTCTACGCAGACTCCGTGAAGGGCCGCT TCACCATCTCCAGAGACAATTCCAAGAACACACT GTATCTGCAAATGAACAGCCTGAGAGCCGAGGA CACGGCCGTATATTACTGTGCGAAAAAGGATAAT GATTACGTTTGGGGGAGTCCTTACTTTGACTACT GGGGCCAGGGAACCCTGGTCACCGTCTCCTCA |
| | | | SEQ ID NO: 26135 | SEQ ID NO: 30141 |

FIGURE 50

| | | | | |
|---|---|---|---|---|
| | | AA | DIQMTQSPASVSASVGDRVTITCRASQGISNWLA WYQQKPGKAPKLLIYAASSLQGGVPSRFSGSGS GTDFTLSISSLQPEDFATYYCQQTDSFPFTFGPGT KVDIK | EVQLLESGGGLVQPGGSLRLSCAASGFTFNSYAMN WVRQAPGKGLEWVSAISGSGGNTFYADSVKGRFTI SRDNSKNTLYLQMNSLRAEDTAVYYCAKKDNDYV WGSPYFDYWGQGTLVTVSS |
| | | | SEQ ID NO: 26136 | SEQ ID NO: 30142 |
| iPS:435305 | 21-225_146C9 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATTATAATTACTTAGCTTGGTACCA GCAGAAACCAGGACAGCCTCCTAAGGTGCTCA TTTACTGGGCATCTACCCGGAAATCCGGGGTC CCTGACCGATTCAGTGGTAGCGGGTCTGGGAC AGATTTCACTCTCACCATCAGCAGCCTGCAGG CTGAAGATGTGGCAGTTTATTATTGTCAGCAA TATTATAGTACTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCTGGAACACCTCCATAAGCACA GCCTACATGGCCCTGAGCAGCCTGAGATCTGAGG ACACGGCCGTGTATTACTGTGCGTATAGCAGTGG CTGGTACTCCTTTGACTACTGGGGCCAGGGAACC CTGGTCACCGTCTCCTCA |
| | | | SEQ ID NO: 26137 | SEQ ID NO: 30143 |
| | | AA | DIVMTQSPDSLAVSLGERATINCKSSQSVLHSSN NYNYLAWYQQKPGQPPKVLIYWASTRKSGVPD RFSGSGSGTDFTLTISSLQAEDVAVYYCQQYYST PCSFGQGTKLEIK | QVQLVQSGAEVKKPGASVKVSCKASGYTFTNYDIN WVRQATGQGLEWMGWMHPNSGNTGYAQKFQGR VTMTWNTSISTAYMALSSLRSEDTAVYYCAYSSG WYSFDYWGQGTLVTVSS |
| | | | SEQ ID NO: 26138 | SEQ ID NO: 30144 |

FIGURE 50

| | | | | |
|---|---|---|---|---|
| iPS:435307 | 21-225_146E9 | NA | GACATCCAGATGACCCAGTCTCCATCCTCCCT GTCTGCATCTGTAGGAGACAGAGTCACCATCA CTTGCCGGGCAAGTCAGAGCATTAGCGACTAT TTAAATTGGTATCAGCTGAAACCAGGGAAAGC CCCTAAGGTCCTGATCTATACTACATCCAGTTT GCAAAGTGGGGTCCCATCAAGGTTCAGTGGCA GTGGATCTGGGACAGATTTCACTCTCACCATC AGCAGTCTGCAACCTGAAGATTTTGCAACTTA CTTCTGTCAACAGAGTTACAGTACCCCCACTTT CGGCGGAGGGACCAAGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAAAACACT ATATCTGCAAATGAACAGCCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26139 | SEQ ID NO: 30145 |
| | | AA | DIQMTQSPSSLSASVGDRVTITCRASQSISDYLN WYQLKPGKAPKVLIYTTSSLQSGVPSRFSGSGSG TDFTLTISSLQPEDFATYFCQQSYSTPTFGGGTKV EIK | EVQLLESGGGLVQPGGSLRLSCAASGFTFSSYAMS WVRQAPGKGLEWVSAISGRGGNTFYADSVKGRFTI SRDNSKKTLYLQMNSLRAEDTAVYYCAKRVTDYG GNDWFDPWGQGTLVTVSS |
| | | | SEQ ID NO: 26140 | SEQ ID NO: 30146 |
| iPS:435309 | 21-225_146F9 | NA | GACATCGTGATGACCCAGTCTCCAGACTCCCT GGCTGTGTCTCTGGGCGAGAGGGGCCACCATCA ACTGCAAGTCCAGCCAGAATATTTTACACAGC TCCAACAATAACAACTACTTAGCTTGGTACCA GCAGAAACCAGGACAGCCTCCTTACCTGCTCA TTTACTGGGCATCTACCCGGGAATCCGGGGTC CCTGACCGATTCAGTGGCAGCGGGTCTGGGAC AGATTTCACTCTCACCATCACCAGCCTGCAGG CTGAAGATGTGGCAGTTTATTACTGTCAGCAA TATTATACTACTCCGTGCAGTTTTGGCCAGGG GACCAAGCTGGAGATCACT | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGAGTAGCAGT GGCTGGTACTTTTTTGACTACTGGGGCCAGGGAA CCCTGGTCACCGTCTCCTCA |
| | | | SEQ ID NO: 26141 | SEQ ID NO: 30147 |

FIGURE 50

| | | | | |
|---|---|---|---|---|
| | | AA | DIVMTQSPDSLAVSLGERATINCKSSQNILHSSN NNNYLAWYQQKPGQPPYLLIYWASTRESGVPD RFSGSGSGTDFTLTITSLQAEDVAVYYCQQYYTT PCSFGQGTKLEIT | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCASSSGW YFFDYWGQGTLVTVSS |
| | | | SEQ ID NO: 26142 | SEQ ID NO: 30148 |
| IPS:435311 | 21-225_146H9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCAGCTTCAGTAGCTATGGCAT GCACTGGGTCCGCCAGGCTCCAGGCAAGGGGCT GGAGTGGGTGGCAGTGATATGGTTTGATGAAAGT AATAAACACTATGGAGACTCCGTGAAGGGCCGA TTCACCATCTCCAGGGACAATTCCAAGAACACGC TGTATCTGCAAATGAACAGCCTGAGAGCCGAGG ACACGGCTGTGTATTACTGTGCGAGAGAATTGGG ATTTCTCTCTGACTATTGGGGCCAGGGAACCCTG GTCACCGTCTCCTCA |
| | | | SEQ ID NO: 26143 | SEQ ID NO: 30149 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFSFSSYGMH WVRQAPGKGLEWVAVIWFDESNKHYGDSVKGRF TISRDNSKNTLYLQMNSLRAEDTAVYYCARELGFL SDYWGQGTLVTVSS |
| | | | SEQ ID NO: 26144 | SEQ ID NO: 30150 |

FIGURE 50

| iPS:435313 | 21-225_146G11 | NA | GACATCCAGATGACCCAGTCTCCATCCTCCCT GTCTGCATCTGTAGGAGACAGAGTCACCATCA CTTGCCGGGCAAGTCAGGACATTAGAAATAAT TTTGGCTGGTATCAGCAGAAACCAGGGAAAGC CCCTAAGCGCCTGATCTATGCTGCATCCAGTTT GCAAAGTGGGGTCCCATTAAGGTTCAGCGGCA GTGGATCTGGGACAGAATTCACTCTCACAATC AGCAGCCTGCAGCCTGAAGATTTTGCAACTTA TTACTGTCTCCAACATGATAGTTACCCGCTCAC TTTCGGCGGAGGGACCAAGGTGGAGATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTGGTAGC TACACATACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAGGTAGCAG CTCGTCCGGGTTTGACTACTGGGGCCAGGGAACC CTGGTCACCGTCTCCTCA |
|---|---|---|---|---|
| | | | SEQ ID NO: 26145 | SEQ ID NO: 30151 |
| | | AA | DIQMTQSPSSLSASVGDRVTITCRASQDIRNNFG WYQQKPGKAPKRLIYAASSLQSGVPLRFSGSGS GTEFTLTISSLQPEDFATYYCLQHDSYPLTFGGG TKVEIK | EVQLVESGGGLVKPGGSLRLSCAASGFTFSSYSMN WVRQAPGKGLEWVSSISGSGSYTYYADSVKGRFTI SRDNAKNSLYLQMNSLRAEDTAVYYCARGSSSSGF DYWGQGTLVTVSS |
| | | | SEQ ID NO: 26146 | SEQ ID NO: 30152 |
| iPS:435315 | 21-225_147B2 | NA | GATATTGTGATGACCCAGACTCCCCTCTCTCTG TCCGTCACGCCTGGACAGCCGGCCTCCATCTC CTGCAAGTCTAGTCAGAGCCTCCTGCATAGTG AAGGAAAGACCTATTTGTATTGGTACCTGCAG AAGCCAGGCCAGCCTCCACAGCTCCTGATCTA TGAAGTTTCCCACCGGGTCTCTGGAGTGCCAG ATAGGTTCAGTGGCAGCGGGTCAGGGACAGA TTTCACAGTGAAAATCAGCCGGGTGGAGGCTG AGGATGTTGGGGTTTATTACTGCATGCAAAGT ACACAGTTTCCTCCCACTTTCGGCCCTGGGAC CAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGAGGTATAG CAGCAGCTGGTCGGGGGGGTATGGACGTCTGGGG CCAAGGGACCACGGTCACCGTCTCCTCA |
| | | | SEQ ID NO: 26147 | SEQ ID NO: 30153 |

FIGURE 50

| | | | | |
|---|---|---|---|---|
| | | AA | DIVMTQTPLSLSVTPGQPASISCKSSQSLLHSEGK TYLYWYLQKPGQPPQLLIYEVSHRVSGVPDRFS GSGSGTDFTVKISRVEAEDVGVYYCMQSTQFPP TFGPGTKVDIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WSGGMDVWGQGTTVTVSS |
| | | | SEQ ID NO: 26148 | SEQ ID NO: 30154 |
| iPS:435317 | 21-225_147D2 | NA | GAAATTGTGTTGACGCAGTCTCCAGGCACCCT GTCTTTGTCTCCAGGGGAAAGAGCCACCCTCT CCTGCAGGGCCAGTCAGAGTGTTGGCAGCAGC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAGCTTATTCA CTTTCGGCCCTGGGACCAAAGTGGATATCAAA | CAGGTGCTACTGCAGGAGTCGGGCCCAGGACTG GTGAAGCCTTCACAGACCCTGTCCCTCACCTGCG CTGTCTCTGGTGGCCCCATCAGCAGTGGTGATTA CTACTGGAACTGGATCCGCCAGCGCCCAGGGAA GGGCCTGGAGTGGATTGGGTTCATCTATTACACT GGGAGCACCTACTACAACCCGTCCCTCAAGAGTC GAGTTTCCATATCGGAAGACACGTCTGAGAACCA GTTCTCCCTGAACCTGAGTTCTGTGACTGCCGCG GACACGGCCGTGTATTACTGTGCGAGAGGGGGA GCTTACTACTCCTACTACGGTATGGACGTCTGGG GCCAAGGGACCACGGTCACCGTCTCCTCA |
| | | | SEQ ID NO: 26149 | SEQ ID NO: 30155 |
| | | AA | EIVLTQSPGTLSLSPGERATLSCRASQSVGSSYLA WYQQKPGQAPRLLIYGASSRATGIPDRFSGSGSG TDFTLTISRLEPEDFAVYYCQQYGSLFTFGPGTK VDIK | QVLLQESGPGLVKPSQTLSLTCAVSGGPISSGDYYW NWIRQRPGKGLEWIGFIYYTGSTYYNPSLKSRVSIS EDTSENQFSLNLSSVTAADTAVYYCARGGAYYSYY GMDVWGQGTTVTVSS |
| | | | SEQ ID NO: 26150 | SEQ ID NO: 30156 |

FIGURE 50

| iPS:435319 | 21-225_147E3 | NA | GAAATTGTGTTGACGCAGTCTCCAGGCACCCT GTCTTTGTCTCCAGGGGAAAGAGCCACCCTCT CCTGCAGGGCCAGTCAGAGTGTTATCAGTAGC TACTTAGCCTGGTACCAGCAGAAACCTGGCCA GGCTCCCAGGCTCCTCATCTATGGTGCATCCA GCAGGGCCACTGCCATCCCAGACAGGTTCAGT GGCAGTGGGTCTGGGACAGACTTCACTCTCAC CATCAGCAGACTGGAGCCTGAAGATTTTGCAG TGTATTACTGTCAACAATATGGTAGGTCACCA TTCAATTTCGGCCCTGGGACCAAAGTGGATAT CAAA | CAGGTGCAGCTGCAGGAGTCGGGCCCAGGACTG GTGAAGCCTTCACAGACCCTGTCCCTCACCTGCA CTGTCTCTGGTGGCTCCATCACCAATAGTGGTTA CTACTATAGCTGGATCCGCCAGCACCCAGGGAA GGGCCTGGAATGGATTGGGTACATCTATTACAGT GGGGGCACCTACTACAACCCGTCCCTCAAGAGTC GAATTACCATATCAGTGGACACGTCTAACAACCA GTTCTCCCTGAAGCTGAGCTCTGTGACTGCCGCG GACACGGCCGTGTATTACTGTGCGAGAGGGGGA TATAACTGGAACCATGCTTTTGATTTCTGGGGCC AAGGGACAATGGTCACCGTCTCTTCA |
|---|---|---|---|---|
| | | | SEQ ID NO: 26151 | SEQ ID NO: 30157 |
| | | AA | EIVLTQSPGTLSLSPGERATLSCRASQSVISSYLA WYQQKPGQAPRLLIYGASSRATAIPDRFSGSGSG TDFTLTISRLEPEDFAVYYCQQYGRSPFNFGPGT KVDIK | QVQLQESGPGLVKPSQTLSLTCTVSGGSITNSGYYY SWIRQHPGKGLEWIGYIYYSGGTYYNPSLKSRITISV DTSNNQFSLKLSSVTAADTAVYYCARGGYNWNHA FDFWGQGTMVTVSS |
| | | | SEQ ID NO: 26152 | SEQ ID NO: 30158 |
| iPS:435321 | 21-225_147E4 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATTACAACTACTTAGCTTGGTACCA GCTGAAACCAAGACAGCCTCCTAAGCTGCTCA TTTACTGGGCATCTACCCGGGAATCCGGGGTC CCTGACCGATTCAGTGGCCGCGGGTCGGGGAC AGACTTCACTCTCACCATCAGCAGCCTGCAGG CTGAAGATGTGGCAATTTATTACTGTCAGCAA TATTATAGTACTCCATCCACTTTCGGCCCTGGG ACCAAAGTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGTAGCA GTGGCTGGTACTTTTTTGACTACTGGGGCCAGGG AACCCTGGTCACCGTCTCCTCA |
| | | | SEQ ID NO: 26153 | SEQ ID NO: 30159 |

FIGURE 50

| | | | | |
|---|---|---|---|---|
| | | AA | DIVMTQSPDSLAVSLGERATINCKSSQSVLHSSN NYNYLAWYQLKPRQPPKLLIYWASTRESGVPDR FSGRGSGTDFTLTISSLQAEDVAIYYCQQYYSTPS TFGPGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCASSSGW YFFDYWGQGTLVTVSS |
| | | | SEQ ID NO: 26154 | SEQ ID NO: 30160 |
| iPS:435323 | 21-225_147D5 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATAACAACTACTTAGCTTGGTACCA GCAGAAACCAGGACAGCCTCCTAAACTGCTCA TTTACTGGGCATCTACCCGGGAATCCGGGGTC CCTGACCGATTCAGTGGCAGTGGGTCTGGGAC AGATTTCACTCTCTCCATCAGCAGCCTGCAGG CTGAAGATGTGGCAGTTTATTACTGTCACCAA TATTATAGTACTCCGTGCAGTTTTGGCCAGGG GACCAAACTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGGTGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGG GGACACGGCCGTGTATTACTGTGCGGGGGAGCAG TGGCTGGTACTACTTTGACTACTGGGGCCAGGGC ACCCTGGTCACCGTCTCCTCA |
| | | | SEQ ID NO: 26155 | SEQ ID NO: 30161 |
| | | AA | DIVMTQSPDSLAVSLGERATINCKSSQSVLYSSN NNNYLAWYQQKPGQPPKLLIYWASTRESGVPD RFSGSGSGTDFTLSISSLQAEDVAVYYCHQYYST PCSFGQGTKLEIK | QVQLVQSGAEVKKPGASVKVSCKASGYTFTNYDIN WVRQATGQGLEWMGWVHPNSGNTGYAQKFQGR VTMTRNTSISTAYMELSSLRSGDTAVYYCAGSSGW YYFDYWGQGTLVTVSS |
| | | | SEQ ID NO: 26156 | SEQ ID NO: 30162 |

FIGURE 50

| iPS:435325 | 21-225_147H5 | NA | GACATCCAGATGACCCAGTCTCCATCCTCCCT GTCTGCATCTGTAGGAGACAGAGTCACCATCA CTTGCCGGGCAAGTCGGGGCATTAGAGATGAT TTAGGCTGGTATCAGCAGAAACCAGGGAAAG CCCCTAAGCGCCTGATCTATGCTGCATCCAGT TTGCAGAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTATCCTCGG ACGTTCGGCCAAGGGACCAAGGTGGAAATCA AA | CAGGTGCAGCTGGTGGAGTCTGGGGGAGGCGTG GCCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGGAAGT AATAAGTACTATGCAGACTCCGTGAAGGGCCGA CTCACCATCTCCAGAGACAATTCCAAGAACACGT TGTATCTGCAAATGAACAGCCTGAGCGCCGAGG ACACGGCTGTGTATTACTGTGCGAGAGAGAGGTA TAGCAGTGGCTGGTACGACTACGGTTTGGACGTC TGGGGCCAAGGGACCACGGTCACCGTCTCCTCA |
| | | | SEQ ID NO: 26157 | SEQ ID NO: 30163 |
| | | AA | DIQMTQSPSSLSASVGDRVTITCRASRGIRDDLG WYQQKPGKAPKRLIYAASSLQSGVPSRFSGSGS GTEFTLTISSLQPEDFATYYCLQHYSYPRTFGQG TKVEIK | QVQLVESGGGVAQPGRSLRLSCAASGFTFSDYGM HWVRQAPGKGLEWVAVIWYDGSNKYYADSVKGR LTISRDNSKNTLYLQMNSLSAEDTAVYYCARERYS SGWYDYGLDVWGQGTTVTVSS |
| | | | SEQ ID NO: 26158 | SEQ ID NO: 30164 |
| iPS:435327 | 21-225_147G6 | NA | GACATCGTGATGACCCAGTCTCCAGACTCCCT GGCTGTGTCTCTGGGCGAGAGGGGCCACCATCA ACTGCAAGTCCAGCCAGAGTGTTTTATACAGC TCCAACAGTAACAACTACTTAGCTTGGTACCA GCAGAAACCAGGCCAGCCTCCTAAGCTGCTCA TTTACTGGGCATCTGCCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATACTACTCCTCCCACTTTCGGCCCTGGG ACCAAAGTGGATATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGTTGGATGCACCCCAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGTATAGCAGT GGCTGGTACTGGTTCGACCCCTGGGGCCAGGGA ACCCTGGTCACCGTCTCCTCA |
| | | | SEQ ID NO: 26159 | SEQ ID NO: 30165 |

FIGURE 50

| | | | | |
|---|---|---|---|---|
| | | AA | DIVMTQSPDSLAVSLGERATINCKSSQSVLYSSN SNNYLAWYQQKPGQPPKLLIYWASARESGVPD RFSGSGSGTDFTLTISSLQAEDVAVYYCQQYYTT PPTFGPGTKVDIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YWFDPWGQGTLVTVSS |
| | | | SEQ ID NO: 26160 | SEQ ID NO: 30166 |
| iPS:435329 | 21-225_147A8 | NA | GATATTGTGATGACCCAGACTCCACTCTCTCT GTCCGTCACCCCTGGACAGCCGGCCTCCATCT CCTGCAAGACTAGTCAGAGCCTCCTGCATAGT GAAGGAAAGACCTATTTGTATTGGTACCTGCA GAAGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCTAATCACCTTCGGCCAAGGGACAC GACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGACGGGGGGGTATGGACGTCTGGGG CCAAGGGACCACGGTCACCGTCTCCTCA |
| | | | SEQ ID NO: 26161 | SEQ ID NO: 30167 |
| | | AA | DIVMTQTPLSLSVTPGQPASISCKTSQSLLHSEGK TYLYWYLQKPGQPPQLLIYEVSNRFSGVPDRFSG SGSGTDFTLKISRVEAEDVGVYYCMQSIQLITFG QGTRLEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WTGGMDVWGQGTTVTVSS |
| | | | SEQ ID NO: 26162 | SEQ ID NO: 30168 |

FIGURE 50

| iPS:435331 | 21-225_147G8 | NA | CAAATTGTGTTGACGCAGTCTCCAGGCACCCT GTCTTTGTCTCCAGGGGAAAGAGCCACCCTCT CCTGCAGGGCCAGTCAGAGAATTTTCAGCAAC TACTTAGCCTGGTACCAGCAGAAGCCTGGCCA GGCTCCCAGGATCCTCATCTATGGTGCATCCA GCAGGGCCACTGGCATCCCAGACAGGATCAGT GGCAGTGGGTCTGGGACAGACTTCACTCTCAC CATCACCAGACTGGAGCCTGAAGATTTTGCAG TGTATTACTGTCAGCAGTATGATAGCTCACCG TGGACGTTCGGCCAAGGGACCAAGGTGGAAA TCAAC | CAGGTGCAGCTACAGCAGTGGGGCGCAGGACTG TTGAAGCCTTCGGAGACCCTGTCCCTCACCTGCG GTGTCTATGGTGGGTCCTTCAGTGCTTACTACTG GAGCTGGATCCGCCAGCCCCCAGGGAAGGGGCT GGAGTGGATTGGGGAAATCAATCATAGTGGAAG TACCAACTACAAACCGTCCCTCAAGAGTCGAGTC ACCATATCAGTAGACACGTCCAAGAACCAGTTCT CCCTGAAGCTGAGCTCTGTGACCGCCGCGGACAC GGCTGTGTATTACTGTGCGAGAGACTACGGTGTT TTTGACTACTGGGGCCAGGGAACCCTGGTCACCG TCTCCTCA |
| | | | SEQ ID NO: 26163 | SEQ ID NO: 30169 |
| | | AA | QIVLTQSPGTLSLSPGERATLSCRASQRIFSNYLA WYQQKPGQAPRILIYGASSRATGIPDRISGSGSGT DFTLTITRLEPEDFAVYYCQQYDSSPWTFGQGTK VEIN | QVQLQQWGAGLLKPSETLSLTCGVYGGSFSAYYW SWIRQPPGKGLEWIGEINHSGSTNYKPSLKSRVTISV DTSKNQFSLKLSSVTAADTAVYYCARDYGVFDYW GQGTLVTVSS |
| | | | SEQ ID NO: 26164 | SEQ ID NO: 30170 |
| iPS:435333 | 21-225_147E9 | NA | GACATCCAGATGACCCAGTCTCCATCCTCACT GTCTGCATCTGTAGGAGACAGAGTCACCATCA CTTGTCGGGCGAGTCAGGACATTAACAATTAT TTAGCCTGGTTTCAGCAGAAACCAGGGAAAGC CCCTAAGTCCCTGATCGTTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCTCTCA CTTTCGGCGGAGGGACCAAGGTGGAGATCAA A | GAGGTGCAGCTGGTGGAGTCTGGGGGAGGCTTG GTACAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTTTCATCCATTAGTGGTAGAAACACT ACCATATACTATGCAGACTCTGTGAAGGGCCGAT TCACCATCTCCAGAGACAATGCCAAGAACTCACT GTATCTGCAAATGAACAGCCTGAGAGACGAGGA CACGGCTGTTTATTACTGTTCGAGAGATCGGGGC AGTTGCTGGGGCCAGGGAACCCTGGTCACCGTCT CCTCA |
| | | | SEQ ID NO: 26165 | SEQ ID NO: 30171 |

FIGURE 50

| iPS:435335 | 21-225_147D10 | AA | DIQMTQSPSSLSASVGDRVTITCRASQDINNYLA WFQQKPGKAPKSLIVAASSLQSGVPSKFSGSGSG TDFTLTISSLQPEDFATYYCQQYNSYPLTFGGGT KVEIK | EVQLVESGGGLVQPGGSLRLSCAASGFTFSSYSMN WVRQAPGKGLEWVSSISGRNTTIYYADSVKGRFTI SRDNAKNSLYLQMNSLRDEDTAVYYCSRDRGSCW GQGTLVTVSS |
|---|---|---|---|---|
| | | | SEQ ID NO: 26166 | SEQ ID NO: 30172 |
| | | NA | GACATCCAGATGACCCAGTCTCCAGCTTCCGT GTCTGCATCTGTAGGAGACAGAGTCACCATAA CTTGTCGGGCGAGTCAGAATATTAGCAACTGG TTAACCTGGTATCAGCAGAAACCAGGGAAAG CCCCTAAGCTCCTGATCTATGCTGCATCCAGTT TGCAAAGTGGGGTCCCATCAAGGTTCAGCGGC AGTGGATCTGGGACAGATTTCACTCTCTCCAT CAGCAGCCTGCAGCCTGAAGATTTTGCAACTT ACTATTGTCAACAGACTGACAGTTTCCCATTC ACTTTCGGCCCTGGGACCAAAGTGGATGTCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGCT TCACCATCTCCAGAGACAATTCCAAGAACACGCT CTATCTGCAAATGAACAGCCTGAGAGCCGAGGA CACGGCCGTATATTACTGTGCGAAAAAGGATTAT GATTACGTTTGGGGGGAGTCCTTACTTTGACTACT GGGGCCAGGGAACCCTGGTCACCGTCTCCTCA |
| | | | SEQ ID NO: 26167 | SEQ ID NO: 30173 |
| | | AA | DIQMTQSPASVSASVGDRVTITCRASQNISNWLT WYQQKPGKAPKLLIYAASSLQSGVPSRFSGSGS GTDFTLSISSLQPEDFATYYCQQTDSFPFTFGPGT KVDVK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKKDYDYV WGSPYFDYWGQGTLVTVSS |
| | | | SEQ ID NO: 26168 | SEQ ID NO: 30174 |

FIGURE 50

| iPS:435339 | 21-225_147D12 | NA | GACATCCAGATGACCCAGTCTCCAGCTTCCGT GTCTGCATCTGTAGGAGACAGAGTCACCATAA CTTGTCGGGCGAGTCAGGGTATTAGCAACTGG TTAGCCTGGTATCAGGAGAAACCAGGGAAAG CCCCTAAACTCCTGATCTATGCTGCATCCAGTT TGCAAAGTGGGGTCCCATCAAGGTTCAGCGGC AGTGGATCCGGGACAGATTTCACTCTCTCCAT CAGCAGCCTGCAGCCTGAAGATTTTGCAACTT ACTATTGTCAACAGACTGACAGTTTCCCATTC ACTTTCGGCCCTGGGACCAAAGTGGATGTCAA A | GAGGTGCAGCTGTTGGAGTCTGGGGGAGGCTTG GTACAGTCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGCT TCACCATCTCCAGAGACAATTCCAAGAACACGCT CTATCTGCAAATGAACAGCCTGAGAGCCGAGGA CACGGCCGTATATTACTGTGCGAAAAAGGATTAT GATTACGTTTGGGGGAGTCCTTACTTTGACTACT GGGGCCAGGGAACCCTGGTCACCGTCTCCTCA |
| | | | SEQ ID NO: 26169 | SEQ ID NO: 30175 |
| | | AA | DIQMTQSPASVSASVGDRVTITCRASQGISNWLA WYQEKPGKAPKLLIYAASSLQSGVPSRFSGSGSG TDFTLSISSLQPEDFATYYCQQTDSFPFTFGPGTK VDVK | EVQLLESGGGLVQSGGSLRLSCAASGFTFSSYAMS WVRQAPGKGLEWVSAISGRGGNTFYADSVKGRFTI SRDNSKNTLYLQMNSLRAEDTAVYYCAKKDYDYV WGSPYFDYWGQGTLVTVSS |
| | | | SEQ ID NO: 26170 | SEQ ID NO: 30176 |
| iPS:435341 | 21-225_148B2 | NA | GCTATTGTGATGACCCAGACTCCACTCTCTCTG TCCGTCACCCCTGGACAGCCGGCCTCCATCTC CTGCAAGTCTAGTCAGAGCCTCCTGCATGGTG ATGGAAAGACCTATTTTTATTGGTACCTGCAG AAGCCAGGCCAGCCTCCACAGCTCCTGATCTA TGAAGTTTCCCACCGGTTCTCTGGAGTGCCAG ATAGGTTCAGTGGCAGCGGGTCAGGGACAGA TTTCACACTGAAAATCAGCCGGGTGGAGGCTG AGGATGTTGGGGTTTATTACTGCATGCAAAGT ATACAGATTCCGTGGACGTTCGGCCAAGGGAC CAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATCTGGTATGATGGAAGT TATAAATACTATGCAGACTCCGTGAAGGGCCGAT TCACCATCTCCAGAGACAATTCCAAGAACACGCT GTATCTGCAAATGAACAGCCTGAGTGCCGAGGA CACGGCTGTGTATTACTGTGCGAGAGATCATTTC GATTTTTGGAGTGGTCACTTTGACTACTGGGGCC AGGGAACCCTGGTCACCGTCTCCTCA |
| | | | SEQ ID NO: 26171 | SEQ ID NO: 30177 |

FIGURE 50

| | | | | |
|---|---|---|---|---|
| | | AA | AIVMTQTPLSLSVTPGQPASISCKSSQSLLHGDG KTYFYWYLQKPGQPPQLLIYEVSHRFSGVPDRFS GSGSGTDFTLKISRVEAEDVGVYYCMQSIQIPWT FGQGTKVEIK | QVQLVESGGGVVQPGRSLRLSCAASGFTFSSYGMH WVRQAPGKGLEWVAIIWYDGSYKYYADSVKGRFT ISRDNSKNTLYLQMNSLSAEDTAVYYCARDHFDF WSGHFDYWGQGTLVTVSS |
| | | | SEQ ID NO: 26172 | SEQ ID NO: 30178 |
| iPS:435343 | 21-225_148E2 | NA | GACATCCAGATGACCCAGTCTCCAGCTTCCGT GTCTGCATCTGTAGGAGACAGAGTCACCATAA CTTGTCGGGCGAGTCAGGGTATTAGCAACTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AATGAATCTGGGACAGATTTCACTCTCTCCAT CAGCAGCCTGCAGCCTGAAGATTTTGCAACTT ACTATTGTCAACAGACTGACAGTTTCCCATTC ACTTTCGGCCCTGGGACCAAAGTGGATGTCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGCT TCACCATCTCCAGAGACAATTCCAAGAACACGCT CTATCTGCAAATGAACAGCCTGAGAGCCGAGGA CACGGCCGTATATTACTGTGCGAAAAAGGATTAT GATTACGTTTGGGGGGAGTCCTTACTTTGACTACT GGGGCCAGGGAACCCTGGTCACCGTCTCCTCA |
| | | | SEQ ID NO: 26173 | SEQ ID NO: 30179 |
| | | AA | DIQMTQSPASVSASVGDRVTITCRASQGISNWLA WYQQKPGKAPKLLIYAASSLQSGVPSRFSGNES GTDFTLSISSLQPEDFATYYCQQTDSFPFTFGPGT KVDVK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKKDYDYV WGSPYFDYWGQGTLVTVSS |
| | | | SEQ ID NO: 26174 | SEQ ID NO: 30180 |

FIGURE 50

| iPS:435345 | 21-225_148G3 | NA | GACATCGTGATGACCCAGTCTCCAGACTCCCT GGCTGTGTCTCTGGGCGAGAGGGCCACCATCC ACTGCAAGTCCAGCCAACGTGTTTTACACAGC TCCAACAATTATAACTACTTAGCTTGGTACCA GCAGAAACCAGGACAGCCTCCTAAGCTGCTCA TTTACTGGGCATCTACCCGGGATTCCGGGGTC CCTGACCGATTCAGTGGCAGCGGGTCTGGGGC CGATTTCACTCTCACCATCAGCAGCCTGCAGG CTGAAGATGTGGCACTTTATTACTGTCAGCAA TATTATAGTACTCCATTCACTTTCGGCCCTGGG ACCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGGC TTGAGTGGATGGGATGGATGCACCCTAACAGTGG TAACACAGGCTATGCACAGAACTTCCAGGGCAG AGTCACCATGACCAGGAACACCTCCATAAGCAC AGCCTACATGGAGCTGAGCAGCCTGAGATCTGA GGACACGGCCGTGTATTACTGTGCGAGTAGCAGT GGCTGGTACTTTTTTGACTACTGGGGCCAGGGAA CCCTGGTCACCGTCTCCTCA |
| | | | SEQ ID NO: 26175 | SEQ ID NO: 30181 |
| | | AA | DIVMTQSPDSLAVSLGERATIHCKSSQRVLHSSN NYNYLAWYQQKPGQPPKLLIYWASTRDSGVPD RFSGSGSGADFTLTISSLQAEDVALYYCQQYYST PFTFGPGTKVDIK | QVQLVQSGAEVKKPGASVKVSCKASGYTFTNYDIN WVRQASGQGLEWMGWMHPNSGNTGYAQNFQGR VTMTRNTSISTAYMELSSLRSEDTAVYYCASSSGW YFFDYWGQGTLVTVSS |
| | | | SEQ ID NO: 26176 | SEQ ID NO: 30182 |
| iPS:435347 | 21-225_148C4 | NA | GACATCCAGATGACCCAGTCTCCATCCTCCCT GTCTGCATCTGTAGGAGACAGAGTCACCATCA CTTGCCGGGCAAGTCAGAGCATTATCAACTAT TTAAATTGGTATCAGCAGAAACCAGGGAAAG CCCCTAAGGTCCTGATCTATACTGCATCCAGTT TACAGAGTGGGGTCCCATCAAGGTTCAGTGGC AGTGGATCTGGGACAGATTTCACTCTCACCAT CAGCAGTCTGCAACCTGAAGATTTTACAACTT ACTACTGTCAACAGAGTTACAGTACCCCCACT TTCGGCGGAGGGACCAAGGTAGAGATCGAA | GAGGTGCAGCTCTTGGAGTCTGGGGGAGGCTTGG TACAGCCTGGGGGGTCCCTGAGACTCTCCTGTGC AGCCTCTGGATTCACCTTTAGCAGCTATGCCATG AACTGGGTCCGCCAGGCTCCAGGGAAGGGGCTG GAGTGGGTCTCAGCTATTAGTGGTAGTGGTGGTA ACACATTCTACGCAGACTCCGTGAAGGGCCGGTT CACCATCTCCAGAGACAATTCCAAGAACACGCTG TATCTGCAAATGAACAGCCTGAGAGCCGAGGAC ACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26177 | SEQ ID NO: 30183 |

FIGURE 50

| | | | | |
|---|---|---|---|---|
| | | AA | DIQMTQSPSSLSASVGDRVTITCRASQSIINYLNW YQQKPGKAPKVLIYTASSLQSGVPSRFSGSGSGT DFTLTISSLQPEDFTTYYCQQSYSTPTFGGGTKVE IE | EVQLLESGGGLVQPGGSLRLSCAASGFTFSSYAMN WVRQAPGKGLEWVSAISGSGGNTFYADSVKGRFTI SRDNSKNTLYLQMNSLRAEDTAVYYCAKRVIDYG GNDWFDPWGQGTLVTVSS |
| | | | SEQ ID NO: 26178 | SEQ ID NO: 30184 |
| iPS:435349 | 21-225_148F5 | NA | GATATTGTGATGACCCAGACTCCACTCTCTCT GTCCGTCACCCCTGGACAGCCGGCCTCCATCT CCTGTAAGTCTAGTCAGAGCCTCCTGCATAGT GAAGGAAAGACCTATTTGTATTGGTACCTGCA GAAGCCAGGCCAGCCTCCACAGCTCCTGATCT ATGAAGTTTCCTACCGGGTCTCTGGAGTGCCA GATAGATTCAGTGGCAGCGGGTCAGGGACAG ATTTCACACTGAAAATCAGCCGGGTGGAGGCA GAGGATGTTGGGGTCTATTTCTGCATGCAAAG TATACAGCTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGAGGTATAG CAGCAGCTGGTCGGGGGGGTATGGACGTCTGGGG CCAAGGGACCACGGTCACCGTCTCCTCA |
| | | | SEQ ID NO: 26179 | SEQ ID NO: 30185 |
| | | AA | DIVMTQTPLSLSVTPGQPASISCKSSQSLLHSEGK TYLYWYLQKPGQPPQLLIYEVSYRVSGVPDRFS GSGSGTDFTLKISRVEAEDVGVYFCMQSIQLPLT FGGGTKVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WSGGMDVWGQGTTVTVSS |
| | | | SEQ ID NO: 26180 | SEQ ID NO: 30186 |

FIGURE 50

| | | | | |
|---|---|---|---|---|
| iPS:435351 | 21-225_148B6 | NA | GACATCCAGATGACCCAGTCTCCATCCTCACT GTCTGCATCTGTAGGAGACAGAGTCAGCATCA CTTGTCGGGCGAGTCAGGGCATTAGCAAATAT TTAGCCTGGTTTCAGCAGAAACCAGGGAAAGC CCCTAAGTCCCTGATCTTTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTTCCCATTCAC TTTCGGCCCTGGGACCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGTCT TGAGTGGATGGGTTGGATCCACCCTAACAATGGT GGCACAAACTATGCACAGACGTTTCAGGGCAGG GTCACCATGACCAGGGACACGTCCATCAGCACA GTCTACATGGAGCTGAGCAGGCTGAGATCGAC GACACGGCCGTGTATTACTGTGCGAGAGATCCTG TAGTAGTACCAGCTGCCCCCTTTGACTACTGGGG CCAGGGAACCCTGGTCACCGTCTCCTCA |
| | | | SEQ ID NO: 26181 | SEQ ID NO: 30187 |
| | | AA | DIQMTQSPSSLSASVGDRVSITCRASQGISKYLA WFQQKPGKAPKSLIFAASSLQSGVPSKFSGSGSG TDFTLTISSLQPEDFATYYCQQYNSFPFTFGPGTK VDIK | QVQLVQSGAEVKKPGASVKVSCKASGYTFTGYYM HWVRQAPGQGLEWMGWIHPNNGGTNYAQTFQGR VTMTRDTSISTVYMELSRLRSDDTAVYYCARDPVV VPAAPFDYWGQGTLVTVSS |
| | | | SEQ ID NO: 26182 | SEQ ID NO: 30188 |
| iPS:435353 | 21-225_148F8 | NA | GACATCGTGATGACCCAGTCTCTAGACTCCCT GGCTGTGTCTCTGGGCGAGAGGGGCCACCATCA ACTGCAAGTCCAGCCAGAGTGCTTTACACAGC TCCAACAATTACAACTACTTAGCTTGGTACCA GCAGAAACCAGGACAGCCTCCTAAACTGCTCA TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTATTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGCCAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGTAGCA GTGGCTGGTACTACTTTGACTACTGGGGCCAGGG AACCCTGGTCACCGTCTCCTCA |
| | | | SEQ ID NO: 26183 | SEQ ID NO: 30189 |

FIGURE 50

| | | | | |
|---|---|---|---|---|
| | | AA | DIVMTQSLDSLAVSLGERATINCKSSQSALHSSN NYNYLAWYQQKPGQPPKLLIYWASTRKSGVPD RFSGSGSGTDFTLTISSLQAEDVAVYYCQQYYSI PPTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCASSSGW YYFDYWGQGTLVTVSS |
| | | | SEQ ID NO: 26184 | SEQ ID NO: 30190 |
| iPS:435355 | 21-225_148H9 | NA | GACATCCAGATGACCCAGTCTCCATCCTCCCT GTCTGCATCTGTAGGAGACAGAGTCACCATCA CTTGCCGGGCAAGTCAGAGCATTAGTAACTAT TTAAAATTGGTATCAGCAGAAACCAGGGAAAG CCCCTAAGGTCCTGATCTATATTGCATCCAGTT TGCAAAGTGGGGTCCCATCTAGGTTCAGTGGC AGTGGATCTGGGACAGATTTCACTCTCACCAT CAGCAGTCTGCAACCTGAAGATTTTGCAACTT ACTACTGTCAACAGAGTTACAGTACCCCCACT TTCGGCGGAGGGACCAAGGTGGAGATCAAA | GAGGTGCAGCTGTTGGAGTCTGGGGGAGGCTTG GTACAGCCGGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACACT ATATTTGCAAATGAACAGCCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26185 | SEQ ID NO: 30191 |
| | | AA | DIQMTQSPSSLSASVGDRVTITCRASQSISNYLN WYQQKPGKAPKVLIYIASSLQSGVPSRFSGSGSG TDFTLTISSLQPEDFATYYCQQSYSTPTFGGGTK VEIK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKRVTDYG GNDWFDPWGQGTLVTVSS |
| | | | SEQ ID NO: 26186 | SEQ ID NO: 30192 |

FIGURE 50

| iPS:435357 | 21-225_148G10 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAGGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAAG ACACGGCTGTGTATTACTGTGCGAGAGATCGTTA CGATTTTTGGAGTGGTCACTTTGACTACTGGGGC CAGGGAACCCTGGTCACCGTCTCCTCA |
| | | | SEQ ID NO: 26187 | SEQ ID NO: 30193 |
| | | AA | DIVMTQTPLSLSVTPGQPASISCKSSQSLLHGDG KTYLYWYLQRPGQPPQLLIYEVSNRFSGVPDRFS GSGSGTDFTLKISRVEAEDVGVYYCMQSIQLPW TFGQGTKVEIK | QVQLVESGGGVVQPGRSLRLSCAASGFTFSSYGMH WVRQAPGKGLEWVAIIWYDGSNKYYADSVKGRFT ISRDNSKNTLYLQMNSLRAEDTAVYYCARDRYDF WSGHFDYWGQGTLVTVSS |
| | | | SEQ ID NO: 26188 | SEQ ID NO: 30194 |
| iPS:435359 | 21-225_148H10 | NA | GATATTGTGATGACCCAGACTCCACTCTCTCT GTCCGTCACTCCTGGACAGCCGGCCTCCATCT CCTGCAAGTCTAGTCAGAGCCTCCTGCATAGT GAGGGAAAGACCTATTTGTATTGGTACCTGCA GAAGCCAGGCCAGCCTCCACAGCTCCTGATCT ATGAAGTTTCCTACCGGGTCTCTGGAGTGCCA GATAGGTTCAGTGGCAGCGGGTCAGGGACAG ATTTCACACTGAAAATCAGCCGGGTGGAGGCA GAGGATGTTGGGGTCTATTACTGCATGCAGAG TATACAGCTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGGAGACTCCGTGAAGGGCCGA TTCACCATCTCCAGAGACAATTCCAAGAACACGC TGTATCTGCAAATGAACAGCCTGAGAGCCGAGG ACACGGCTGTGTATTATTGTGCGAGGAGGTATAG CAGCAGCTGGTCGGGGGGGTATGGACGTCTGGGG CCAAGGGACCACGGTCACCGTCTCCTCA |
| | | | SEQ ID NO: 26189 | SEQ ID NO: 30195 |

FIGURE 50

| | | | | |
|---|---|---|---|---|
| | | AA | DIVMTQTPLSLSVTPGQPASISCKSSQSLLHSEGK TYLYWYLQKPGQPPQLLIYEVSYRVSGVPDRFS GSGSGTDFTLKISRVEAEDVGVYYCMQSIQLPLT FGGGTKVEIK | QVQLVESGGGVVQPGRSLRLSCAASGFTFSSYGMH WVRQAPGKGLEWVAVIWYDGSNKYYGDSVKGRF TISRDNSKNTLYLQMNSLRAEDTAVYYCARRYSSS WSGGMDVWGQGTTVTVSS |
| | | | SEQ ID NO: 26190 | SEQ ID NO: 30196 |
| iPS:435361 | 21-225_148E11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTCTGCTGCATCCAGTT TGCAAAGTGGGGTCCCATCAAGGTTCACCGGC AGTGGATCTGGGACAGAATTCACTTTCACAAT CAGCAGCGTGCAGCCTGAAGATTTTGCAACTT ATTACTGTCTACAGCATCGTAATTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGTCTCCATCAGCAGAAGTAGTTA CTACTGGGGCTGGATCCGCCAGCCCCCAGGGAA GGGGCTGGAGTGGATTGGGAGTATCTATTATAGT GGGAGCACCTCCTACAACCCGTCCCTCAAGAGTC GAGTCACCATATCCGTAGACACGTCCAAGAACC AGTTCTCCCTGAAGCTGAGCTCTGTGACCGCCGC AGACACGGCTGTGTTTTACTGTGCGAGACTTGAT CCCCAGTGGAGTTTTGACTACTGGGGCCAGGGAA TCCTGGTCACCGTCTCCTCA |
| | | | SEQ ID NO: 26191 | SEQ ID NO: 30197 |
| | | AA | DIQMTQSPSSLSASVGDRVTITCRASQGIRNDLG WYQQKPGKAPKRLISAASSLQSGVPSRFTGSGSG TEFTFTISSVQPEDFATYYCLQHRNYPLTFGGGT KVEIK | QLQLQESGPGLVKPSETLSLTCTVSGVSISRSSYYW GWIRQPPGKGLEWIGSIYYSGSTSYNPSLKSRVTISV DTSKNQFSLKLSSVTAADTAVFYCARLDPQWSFDY WGQGILVTVSS |
| | | | SEQ ID NO: 26192 | SEQ ID NO: 30198 |

FIGURE 50

| iPS:435363 | 21-225_148F12 | NA | GACATCCAGATGACCCAGTCTCCTTCCTCCCT GTCTGCATCTGTAGGAGACAGAGTCACCATCA CTTGCCGGGCAAGTCAGGGCATTAGAAATGCC TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTTCTGTCTACAGCATAATAGTTACCCTCTC ATTTTCGGCGGAGGGACCAAGGTGGAGATCA AA | CAGGTGCAGCTGCAGGAGTCGGGCCCAGGACTG GTGAAGCCTTCACAGACCCTGTCCCTCACCTGCA CTGTCTCTGGTGGCTCCATCAGCAATGGTGGTTA CTACTGGAACTGGATCCGCCAGCACCCAGGGAA GGGCCTGGAGTGGATTGGGTACATCTATTACAGT GGGAGCACCTACTACAACCCGTCCCTCAAGAGTC AAATTACCATATCAGTGGACACGTCTAAGGACCA GTTCTCCCTGAGGCTGAGCTCTGTGACTGCCGCG GACACGGCCGTGTATTACTGTGCGAGGTACAGTA CCTACGACTACTACTACGGTATGGACGTCTGGGG CCAAGGGACCACGGTCACCGTCTCCTCA |
| | | | SEQ ID NO: 26193 | SEQ ID NO: 30199 |
| | | AA | DIQMTQSPSSLSASVGDRVTITCRASQGIRNALG WYQQKPGKAPKRLIYAASSLQSGVPSRFSGSGS GTEFTLTISSLQPEDFATYFCLQHNSYPLIFGGGT KVEIK | QVQLQESGPGLVKPSQTLSLTCTVSGGSISNGGYY WNWIRQHPGKGLEWIGYIYYSGSTYYNPSLKSQITI SVDTSKDQFSLRLSSVTAADTAVYYCARYSTYDYY YGMDVWGQGTTVTVSS |
| | | | SEQ ID NO: 26194 | SEQ ID NO: 30200 |
| iPS:435365 | 21-225_149F1 | NA | GATATTGTGATGACCCAGTCTCCACTCTCTCTG TTCGTCACTCCTGGACAGCCGGCCTCCATCTCC TACAAGTCTAGTCAGAGCCTCCTGCATGGTGA TGGAAAGACCTATTTTTATTGGTACCTGCAGA AGCCAGGCCAGCCTCCACAGCTCTTGATTTAT GAAGTTTCCCACCGGTTCTCTGGAGTGCCAGA TAGGTTCAGTGGCAGCGGGTCAGGGACAGATT TCACACTGAAAATCAGCCGGGTGGAGGCTGA GGATGTTGGGGTTTATTACTGCATGCAAAGTA TACAGATTCCGTGGACGTTCGGCCAAGGGACC AAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATCTGGTATGATGGAAGT TATAAATACTATGCAGACTCCGTGAAGGGCCGAT TCACCATCTCCAGAGACAATTCCAAGAACACGCT GTATCTGCAAATGAACAGCCTGAGTGCCGAGGA CACGGCTGTGTATTACTGTGCGAGAGATCATTTC GATTTTTGGAGTGGTCACTTTGACTACTGGGGCC AGGGAACCCTGGTCACCGTCTCCTCA |
| | | | SEQ ID NO: 26195 | SEQ ID NO: 30201 |

FIGURE 50

| | | | | |
|---|---|---|---|---|
| | | AA | DIVMTQSPLSLFVTPGQPASISYKSSQSLLHGDG KTYFYWYLQKPGQPPQLLIYEVSHRFSGVPDRFS GSGSGTDFTLKISRVEAEDVGVYYCMQSIQIPWT FGQGTKVEIK | QVQLVESGGGVVQPGRSLRLSCAASGFTFSSYGMH WVRQAPGKGLEWVAIIWYDGSYKYYADSVKGRFT ISRDNSKNTLYLQMNSLSAEDTAVYYCARDHFDF WSGHFDYWGQGTLVTVSS |
| | | | SEQ ID NO: 26196 | SEQ ID NO: 30202 |
| IPS:435367 | 21-225_149G1 | NA | GACATCCAGATGACCCAGTCTCCATCCTCCCT GTCTGCATCTGTAGGAGCCAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAAT TTGCAAAGTGGGGTCCCATCAAGGTTCAGTGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGAAGGAAG TAATAAATACTATGCAGACTCCGTGAAGGGCCG ATTCACCATCTCCAGAGACAATTCCAAGAACATG TTGTATCTGCAAATGAACAGCCTGAGAGCCGAG GACACGGCTGTGTATTACTGTGCGAGAGAAATAG GATTCAGTGAGGACTACTGGGGCCAGGGAACCC TGGTCACCGTCTCCTCA |
| | | | SEQ ID NO: 26197 | SEQ ID NO: 30203 |
| | | AA | DIQMTQSPSSLSASVGARVTITCRASQGIRNDLG WYQQKPGKAPKRLIYAASNLQSGVPSRFSGSGS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYEGSNKYYADSVKGR FTISRDNSKNMLYLQMNSLRAEDTAVYYCAREIGF SEDYWGQGTLVTVSS |
| | | | SEQ ID NO: 26198 | SEQ ID NO: 30204 |

FIGURE 50

| iPS:435369 | 21-225_149A2 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGT CCCAACAATAACAACTACTTAGCTTGGTACCA GCAGAAACCAGGACAGCCCCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGGTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGGTGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGGGGAGCAG TGGCTGGTACTACTTTGACTACTGGGGCCAGGGA ACCCTGGTCACCGTCTCCTCA |
| | | | SEQ ID NO: 26199 | SEQ ID NO: 30205 |
| | | AA | DIVMTQSPDSLAVSLGERATINCKSSQSVLYSPN NNNYLAWYQQKPGQPPKLLIYWASTRESGVPD RFSGSGSGTDFTLTISSLQAEDVAVYYCQQYYST PCSFGQGTKVEIK | QVQLVQSGAEVKKPGASVKVSCKASGYTFTNYDIN WVRQATGQGLEWMGWVHPNSGNTGYAQKFQGR VTMTRNTSISTAYMELSSLRSEDTAVYYCAGSSGW YYFDYWGQGTLVTVSS |
| | | | SEQ ID NO: 26200 | SEQ ID NO: 30206 |
| iPS:435371 | 21-225_149A3 | NA | GACATCCAGATGACCCAGTCTCCATCCTCCCT GTCTGCATCTGTAGGAGACAGAGTCACCATCA CTTGCCGGGCAAGTCAGAGCATTAGCAGTTAT TTAAAATTGGTATCAGCAGAAACCAGGGAAAG CCCCTAAGGTCATGATCTATACTGCATCCAGT TTGCAAAGTGGGGTCCCATCAAGGTTCAGTGG CAGTGGATCTGGGACAGATTTCACTCTCACCA TCAACAGTCTGCAACCTGAAGATTTTGCAACT TACTACTGTCAACAGAGTTACAGTATCCCCAC TTTCGGCGGAGGGACCAAGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAACTATGCCAT GACCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTACGAAACGGGTGACG GACTACGGTGGTAACGACTGGTTCGACCCCTGGG GCCAGGGAACCCTGGTCACCGTCTCCTCA |
| | | | SEQ ID NO: 26201 | SEQ ID NO: 30207 |

FIGURE 50

| | | | | |
|---|---|---|---|---|
| | | AA | DIQMTQSPSSLSASVGDRVTITCRASQSISSYLNW YQQKPGKAPKVMIYTASSLQSGVPSRFSGSGSGT DFTLTINSLQPEDFATYYCQQSYSIPTFGGGTKVE IK | EVQLLESGGGLVQPGGSLRLSCAASGFTFSNYAMT WVRQAPGKGLEWVSAISGRGGNTFYADSVKGRFTI SRDNSKNTLYLQMNSLRAEDTAVYYCTKRVTDYG GNDWFDPWGQGTLVTVSS |
| | | | SEQ ID NO: 26202 | SEQ ID NO: 30208 |
| iPS:435373 | 21-225_149E3 | NA | GACATCGTGATGACCCAGTCTCCAGACTCCCT GGCTGTGTCTCTGGGCGAGAGGGCCACCATCA GCTGCAAGTCCAGCCAGACTGTTTTACACAAC TCCAATAATCACAATTACTTTGCTTGGTACCA GCAGAAACCAGGACAGCCTCCTAAGCTGCTCA TTTACTGGGCATCTACCCTGAGATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGACATCTGA GGACACGGCCGTGTATTACTGTGCATATAGCAGT GGCTGGTACTGGTTTGACTACTGGGGCCAGGGAA CCCTGGTCACCGTCTCCTCA |
| | | | SEQ ID NO: 26203 | SEQ ID NO: 30209 |
| | | AA | DIVMTQSPDSLAVSLGERATISCKSSQTVLHNSN NHNYFAWYQQKPGQPPKLLIYWASTLRSGVPD RFSGSGSGTDFTLTISSLQAEDVAVYYCQQYYST PPTFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLTSEDTAVYYCAYSSGW YWFDYWGQGTLVTVSS |
| | | | SEQ ID NO: 26204 | SEQ ID NO: 30210 |

FIGURE 50

| iPS:435375 | 21-225_149H4 | NA | GACATCGTGATGACCCAGTCTCCAGACTCCCTGGCTGTATCTCTGGGCGAGAGGGCCACCATCAACTGCAAGTCCAGCCAGAGTGTTTTATCCAGCTCCAACGATAACAACTACTTAGCTTGGTACCAACAGAAACCAGGACGGCCTCCTAAGCTGCTCATTTACTGGTCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCACCAATATTATAGTTATCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGACTATGCACAGAAGTTCCAGGGCAGAGTCACCATGACCAGGAACACCTCCATAACCACAGTTTACATGGAGCTGAGCAGCCTGAGATCTGAGGACACGGCCGTGTATTATTGTACGTTTAGCAGTGGCTGGTACTACTTTGACTACTGGGGCCAGGGAACCCTGGTCACCGTCTCCTCA |
| | | | SEQ ID NO: 26205 | SEQ ID NO: 30211 |
| | | AA | DIVMTQSPDSLAVSLGERATINCKSSQSVLSSSNDNNYLAWYQQKPGRPPKLLIYWSSTRESGVPDRFSGSGSGTDFTLTISSLQAEDVAVYYCHQYYSYPPTFGQGTKVEIK | QVQLVQSGAEVKKPGASVKVSCKASGYTFTNYDINWVRQATGQGLEWMGWMHPNSGNTDYAQKFQGRVTMTRNTSITTVYMELSSLRSEDTAVYYCTFSSGWYYFDYWGQGTLVTVSS |
| | | | SEQ ID NO: 26206 | SEQ ID NO: 30212 |
| iPS:435377 | 21-225_149G5 | NA | GACATCCAGATGACCCAGTCTCCTTCCTCCCTGTCTGCATCTGTAGGAGACAGAGTCACCATCACTTGCCGGGCAAGTCAGGGCATTAGAAATGCCTTAGGCTGGTTTCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCTCTCACTTTCGGCGGAGGGACCAAGGTGGAGATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAATGGTGGTTACTACTGGAACTGGATCCGCCAGCACCCAGGGAAGGGCCTGGAGTGGATTGGGTACATCTATTACAGTGGGAGCACCTACTACAACCCGTCCCTCAAGAGTCGAGTTACCATATCAGTAGACACGTCTAAGGACCAGTTCTCCCTGAGGCTGAGCTCTGTGACTGCCGCGGACACGGCCGTGTATTACTGTGCGAGGTACAGTACCTACGACTACTACTACGGTATGGACGTCTGGGGCCAAGGGACCACGGTCACCGTCTCCTCA |
| | | | SEQ ID NO: 26207 | SEQ ID NO: 30213 |

FIGURE 50

| | | | | |
|---|---|---|---|---|
| | | AA | DIQMTQSPSSLSASVGDRVTITCRASQGIRNALG WFQQKPGKAPKRLIYAASSLQSGVPSRFSGSGSG TEFTLTISSLQPEDFATYYCLQHNSYPLTFGGGT KVEIK | QVQLQESGPGLVKPSQTLSLTCTVSGGSISNGGYY WNWIRQHPGKGLEWIGYIYYSGSTYYNPSLKSRVT ISVDTSKDQFSLRLSSVTAADTAVYYCARYSTYDY YYGMDVWGQGTTVTVSS |
| | | | SEQ ID NO: 26208 | SEQ ID NO: 30214 |
| IPS:435379 | 21-225_149B6 | NA | GACATCCAGATGACCCAGTCTCCATCTTCCGT GTCGGCATCTGTAGGAGACAGAGTCACCATCA CTTGTCGGGCGAGTCAGGGTATTATCAGTTGG TTAGCCTGGTATCAGCAGAAACCAGGAAAAG CCCCTAAGCTCCTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGGTAACAGTTTCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTAGTGGTGGT AGCACATTCTACGCAGACTCCGTGAAGGGCCGGT TCACCATCTCCAGAGACAATTCCAAGAACACGCT GTTTCTGCAAATGAACAGCCTGAGAGCCGAGGA CACGGCCGTATATTACTGTGCGAAACGAACTCCG GAAGATGTTTTTGATATCTGGGGCCAAGGGACAA TGGTCACCGTCTCTTCA |
| | | | SEQ ID NO: 26209 | SEQ ID NO: 30215 |
| | | AA | DIQMTQSPSSVSASVGDRVTITCRASQGIISWLA WYQQKPGKAPKLLIYAASSLQSGVPSRFSGSGS GTDFTLTISSLQPEDFATYYCQQGNSFPFTFGPGT KVDIK | EVQLLESGGGLVQPGGSLRLSCAASGFTFSSYAMS WVRQAPGKGLEWVSVISGSGGSTFYADSVKGRFTI SRDNSKNTLFLQMNSLRAEDTAVYYCAKRTPEDVF DIWGQGTMVTVSS |
| | | | SEQ ID NO: 26210 | SEQ ID NO: 30216 |

FIGURE 50

| | | | | |
|---|---|---|---|---|
| iPS:435381 | 21-225_149C6 | NA | GACATCCAGATGACCCAGTCTCCAGCTTCCGT GTCTGCATCTGTAGGAGACAGAGTCACCATAA CTTGTCGGGCGAGTCAGGGTATTAGCAACTGG TTAGCCTGGTATCAGCAGAAACCAGGGAGAG CCCCTAAGCTCCTGATCTATGCTGCATCCAGTT TGCAAGGTGGGGTCCCATCAAGGTTCAGCGGC AGTGGATCTGGGACAGATTACACTCTCTCCAT CAGCAGCCTGCAGCCTGAAGATTTTGCAACTT ACTATTGTCAACAGACTGACAGTTTCCCATTC ACTTTCGGCCCTGGGACCAAAGTGGATATCAA A | GAGGTGCAGCTGTTGGAGTCTGGGGGAGGCTTA GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AACACATTCTACGCAGACTCCGTGAAGGGCCGCT TCACCATCTCCAGAGACAATTCCAAGAACACGCT GTATCTGCACATGAACAGCCTGAGAGCCGAGGA CACGGCCGTTTATTACTGTGCGAAAAAGGATTAT GATTACGTTTGGGGGAGTCCTTACTTTGACTACT GGGGCCAGGGAACCCTGGTCACCGTCTCATCA |
| | | | SEQ ID NO: 26211 | SEQ ID NO: 30217 |
| | | AA | DIQMTQSPASVSASVGDRVTITCRASQGISNWLA WYQQKPGRAPKLLIYAASSLQGGVPSRFSGSGS GTDYTLSISSLQPEDFATYYCQQTDSFPFTFGPGT KVDIK | EVQLLESGGGLVQPGGSLRLSCAASGFTFSSYAMS WVRQAPGKGLEWVSAISGSGGNTFYADSVKGRFTI SRDNSKNTLYLHMNSLRAEDTAVYYCAKKDYDYV WGSPYFDYWGQGTLVTVSS |
| | | | SEQ ID NO: 26212 | SEQ ID NO: 30218 |
| iPS:435383 | 21-225_149D7 | NA | GAAATTGTGTTGACGCAGTCTCCAGGCACCCT GTCTTTGTTTCCAGGGGAAAGAGTCACCCTCT CCTGCAGGGCCAGTCAGAGTATTATCAGCAAC TACTTAGCCTGGTACCAGCAGAAACCTGGCCA GGCTCCCAGGCTCCTCATCTTTGGTGTATCTAG TAGGGCCACTGGCATCCCAGACAGGTTCAGTG GCAGTGGGTCTGGGACAGACTTCACTCTCACC ATCAGCAGACTGGAGCCTGAAGATTTTGCAGT GTATTACTGTCAACAATATGGTCGGTCACCAT TCAATTTCGGCCCTGGGACCAAAGTGGATATC AAA | CAGGTGCAGCTGCAGGAGTCGGGCCCAGGACTG GTGAAGCCTTCACAGACCCTGTCCCTCAATTGCA CTGTCTCTGGTGGCTCCATCAGCAACAGTGGTTA CTACTGGAGCTGGATCCGCCAGCACCCAGGGAA GGGCCTGGAATGGATTGGGTACAGCTATTACAGT GGGAGCACCTACTACAACCCGTCCCTCAAGAGTC GAATTACCATATCAGTAGACACGTCTAACAACCA GTTCTCCCTGAAGCTGAGCTCTGTGACTGCCGCG GACACGGCCGTGTATTACTGTGCGAGAGGGGGA TATAACTGGAACCATGCTTTTGATATCTGGGGCC AAGGGACAATGGTCATCGTCTCTTCA |
| | | | SEQ ID NO: 26213 | SEQ ID NO: 30219 |

FIGURE 50

| | | | | |
|---|---|---|---|---|
| | | AA | EIVLTQSPGTLSLFPGERVTLSCRASQSIISNYLA WYQQKPGQAPRLLIFGVSSRATGIPDRFSGSGSG TDFTLTISRLEPEDFAVYYCQQYGRSPFNFGPGT KVDIK | QVQLQESGPGLVKPSQTLSLNCTVSGGSISNSGYY WSWIRQHPGKGLEWIGYSYYSGSTYYNPSLKSRITI SVDTSNNQFSLKLSSVTAADTAVYYCARGGYNWN HAFDIWGQGTMVIVSS |
| | | | SEQ ID NO: 26214 | SEQ ID NO: 30220 |
| iPS:435391 | 21-225_149F8 | NA | GACATCCAGATGACCCAGTCTCCAGCTTCCGT GTCTGCATCTGTAGGAGACAGAGTCACCATAA CTTGTCGGGCGAGTCAGGGTATTAGCAACTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AATGAATCTGGGACAGATTTCACTCTCTCCAT CAGCAGCCTGCAGCCTGAAGATTTTGCAATTT ACTATTGTCAACAGACTGACAGTTTCCCATTC ACTTTCGGCCCTGGGACCAAAGTGGATGTCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGCT TCACCATCTCCAGAGACAATTCCAAGAACACGCT CTATCTGCAAATGAACAGCCTGAGAGCCGAGGA CACGGCCGTATATTACTGTGCGAAAAAGGATTAT GATTACGTTTGGGGGGAGTCCTTACTTTGACTACT GGGGCCAGGGAACCCTGGTCACCGTCTCCTCA |
| | | | SEQ ID NO: 26215 | SEQ ID NO: 30221 |
| | | AA | DIQMTQSPASVSASVGDRVTITCRASQGISNWLA WYQQKPGKAPKLLIYAASSLQSGVPSRFSGNES GTDFTLSISSLQPEDFAIYYCQQTDSFPFTFGPGT KVDVK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKKDYDYV WGSPYFDYWGQGTLVTVSS |
| | | | SEQ ID NO: 26216 | SEQ ID NO: 30222 |

FIGURE 50

| iPS:435393 | 21-225_149D10 | NA | GACATCCAGATGACCCAGTCTCCATCCTCCCTGTCTGCATCGTAGGAGACAGAGTCACCATCACTTGCCGGGCAAGTCGGGGCATTAGAAATGATTTAGGCTGGTATCAGCAGAAACCAGGGAAAGCCCCTAAGCGCCTGATCTATGCTGCATCCAGTTTGCAGAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTATCCTCGGACGTTCGGCCAAGGGACCAAGGTGGAAATCAAA | CAGGTGCAGCTGGTGGAGTCTGGGGGAGGCGTGGCCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGGAAGTAATAAGTACTATGCAGACTCCGTGAAGGGCCGACTCACCATCTCCAGAGACAATTCCAAGAACACGCTGTATCTGCAAATGAACAGCCTGAGAGCCGAGGACACGGCTGTGTATTACTGTGCGAGAGAGAGGTATAGCAGTGGCTGGTACGACTACGGTATGGACGTCTGGGGCCAAGGGACCACGGTCACCGTCTCCTCA |
|------------|---------------|----|----|----|
| | | | SEQ ID NO: 26217 | SEQ ID NO: 30223 |
| | | AA | DIQMTQSPSSLSASVGDRVTITCRASRGIRNDLGWYQQKPGKAPKRLIYAASSLQSGVPSRFSGSGSGTEFTLTISSLQPEDFATYYCLQHYSYPRTFGQGTKVEIK | QVQLVESGGGVAQPGRSLRLSCAASGFTFSDYGMHWVRQAPGKGLEWVAVIWYDGSNKYYADSVKGRLTISRDNSKNTLYLQMNSLRAEDTAVYYCARERYSSGWYDYGMDVWGQGTTVTVSS |
| | | | SEQ ID NO: 26218 | SEQ ID NO: 30224 |
| iPS:435395 | 21-225_149D11 | NA | GACATCCAGATGACCCAGTCTCCAGCTTCCGTGTCTGCATCGTAGGAGACAGAGTCACCATAACTTGTCGGGCGAGTCAGAATATTAGCAACTGGTTAACCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCTCCATCAGCAGCCTGCAGCCTGAAGATTTTGCAACTTACTATTGTCAACAGACTGACAGTTTCCCATTCACTTTCGGCCCTGGGACCAAAGTGGATGTCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGCTTCACCATCTCCAGAGACAATTCCAAGAACACGCTCTATCGGCAAATGAACAGCCTGAGAGCCGAGGACACGGCCGTATATTACTGTGCGAAAAAGGATTATGATTACGTTTGGGGGAGTCCTTACTTTGACTACTGGGGCCAGGGAACCCTGGTCACCGTCTCCTCA |
| | | | SEQ ID NO: 26219 | SEQ ID NO: 30225 |

FIGURE 50

| | | | | |
|---|---|---|---|---|
| | | AA | DIQMTQSPASVSASVGDRVTITCRASQNISNWLT WYQQKPGKAPKLLIYAASSLQSGVPSRFSGSGS GTDFTLSISSLQPEDFATYYCQQTDSFPFTFGPGT KVDVK | EVQLLESGGGLVQPGGSLRLSCAASGFTFSSYAMS WVRQAPGKGLEWVSAISGRGGNTFYADSVKGRFTI SRDNSKNTLYRQMNSLRAEDTAVYYCAKKDYDY VWGSPYFDYWGQGTLVTVSS |
| | | | SEQ ID NO: 26220 | SEQ ID NO: 30226 |
| IPS:435397 | 21-225_149F12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAAT TTGCAAAGTGGGGTCCCATCAAGGTTCAGCGG CAGTGGATCTGGGACAGAATTCACTCTCACAA TCAGCAGCCTGCAGCCTGAAGATTTTGCAATT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGAAGAAAA TAATAAATACTATGCAGACTCCGTGAAGGGCCG ATTCACCATCTCCAGAGACAATTCCAAGAACACG CTGTTTCTGCAAATGAACAGCCTGAGAGCCGAGG ACACGGCTGTGTATTACTGTGCGAGAGAAATAGG GTTCAGTGAGGACTACTGGGGCCAGGGAACCCT GGTCACCGTCTCCTCA |
| | | | SEQ ID NO: 26221 | SEQ ID NO: 30227 |
| | | AA | DIQMTQSPSSLSASVGDRVTITCRASQGIRNDLG WYQQKPGKAPKRLIYAASNLQSGVPSRFSGSGS GTEFTLTISSLQPEDFAIYYCLQHNSYPLTFGGGT KVEIK | QVQLVESGGGVVQPGRSLRLSCAASGFTFSDYGM HWVRQAPGKGLEWVAVIWYEENNKYYADSVKGR FTISRDNSKNTLFLQMNSLRAEDTAVYYCAREIGFS EDYWGQGTLVTVSS |
| | | | SEQ ID NO: 26222 | SEQ ID NO: 30228 |

FIGURE 50

| iPS:435399 | 21-225_150D2 | NA | GACATCGTGATGACCCAGTCTCCAGACTCCCT GGCTGTGTCTCTGGGCGAGAGGGCCACCATCA AGTGCAAGTCCAGCCAGAGTGTTTTATACAGA TCCAACAGTAAGAAATACTTAACTTGGTACCA GCAGAAACCAGGACAGCCTCCTAAGCTGTTCA TTTATTGGGCATCTACCCGGAAATCCGGGGTC CCTGACCGATTCAGTGGCAGCGGGTCTGGGAC AGATTTCACTCTCACCATCAGCAACCTGCAGG CTGAAGATGTGGCAGTTTATTTCTGTCAGCAA TATTTTAGTACTCCGTACAATTTTGGCCAGGG GACCAAGAGGGAGATCAAA | CAGGTGCAGCTGGTGCAATCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCTTATAGCAGT GGCTGGTACTACTTTGACTACTGGGGCCAGGGAA CCCTGGTCACCGTCTCCTCA |
| | | | SEQ ID NO: 26223 | SEQ ID NO: 30229 |
| | | AA | DIVMTQSPDSLAVSLGERATIKCKSSQSVLYRSN SKKYLTWYQQKPGQPPKLFIYWASTRKSGVPDR FSGSGSGTDFTLTISNLQAEDVAVYFCQQYFSTP YNFGQGTKREIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YYFDYWGQGTLVTVSS |
| | | | SEQ ID NO: 26224 | SEQ ID NO: 30230 |
| iPS:435401 | 21-225_150E2 | NA | GACATCCAGATGACCCAGTCTCCATCCTCCCT GTCTGCATCTGTGGGAGACAGAGTCACCATCT CTTGCCGGGCAAGTCAGGGCATTGGAAATGAT TTAGGCTGGTATCAGCAGAAACCAGGGAAAG CCCCTACGCGCCTGATCTATGCTGCATCCAGTT TGCAAAGTGGGGTCCCATCAAGGTTCAGCGGC AGTGGATCTGGGACAGAATTCACTCTCACTAT CAGCAGCCTGCAGCCTGCAGATTTTGCAACTT ATTACTGTCTACAACATTATAGTTTCCCGTACA GTTTTGGCCAGGGGACCAAGCTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGAGTA TAGCAGCAGCTGGTACGGGTACGGTATGGACGT CTGGGGCCAAGGGACCACGGTCACCGTCTCCTCA |
| | | | SEQ ID NO: 26225 | SEQ ID NO: 30231 |

FIGURE 50

| | | | | |
|---|---|---|---|---|
| | | AA | DIQMTQSPSSLSASVGDRVTISCRASQGIGNDLG WYQQKPGKAPTRLIYAASSLQSGVPSRFSGSGSG TEFTLTISSLQPADFATYYCLQHYSFPYSFGQGT KLEIK | QVQLVESGGGVVQPGRSLRLSCAASGFTFSSYVMH WVRQAPGKGLEWVAVIWYDGSNKYYADSVKGRF TISRDNSKNTLYLQMNSLRAEDTAVYYCAREEYSS SWYGYGMDVWGQGTTVTVSS |
| | | | SEQ ID NO: 26226 | SEQ ID NO: 30232 |
| iPS:435403 | 21-225_150C5 | NA | GACATCCAGATGACCCAGTCTCCAGGTTCCGT GTCTGCATCTGTAGGAGACAGAGTCACCATAA CTTGTCGGGCGAGTCAGGGTATTAACAACTGG TTAGCCTGGTATCAGCAGAAACCAGGGAAAG CCCCTAAGCTCCTGATCTATGCTGCATCCAGTT TGCAAGGTGGGGTCCCATCAAGGTTCAGCGGC AGTGGATCTGGGACAGATTTCACTCTCTCCAT CAGCAGCCTGCAGCCTGAAGATTTTGCAACTT ACTATTGTCAACAGACTGACAGTTTCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AACACATTCTACGCAGACTCCGTGAAGGGCCGCT TCACCATCTCCAGAGACAATTCCAAGAACACGCT GTATCTGCAAATGAACAGCCTGAGAGCCGAGGA CACGGCCGTATATTACTGTGCGAAAAAGGATTAT GATTACGTTTGGGGGGAGTCCTTACTTTGACTACT GGGGCCAGGGAACCCTGGTCACCGTCTCCTCA |
| | | | SEQ ID NO: 26227 | SEQ ID NO: 30233 |
| | | AA | DIQMTQSPGSVSASVGDRVTITCRASQGINNWL AWYQQKPGKAPKLLIYAASSLQGGVPSRFSGSG SGTDFTLSISSLQPEDFATYYCQQTDSFPFTFGPG TKVDIK | EVQLLESGGGLVQPGGSLRLSCAASGFTFSSYAMS WVRQAPGKGLEWVSAISGSGGNTFYADSVKGRFTI SRDNSKNTLYLQMNSLRAEDTAVYYCAKKDYDYV WGSPYFDYWGQGTLVTVSS |
| | | | SEQ ID NO: 26228 | SEQ ID NO: 30234 |

FIGURE 50

| | | | | |
|---|---|---|---|---|
| iPS:435405 | 21-225_150B7 | NA | GACATCGTGATGACCCAGTCTCCAGACTCCCT GGCTGTGTCTCTGGGCGAGAGGGCCACCATCA ACTGCAAGTCCAGCCAGAATGTTTTATACAGC TCCCACAATAACAACTACTTAGCTTGGTACCA GCAGAAACCAGGGCAGCCTCCTAAACTACTCA TTTACTGGGCATCTACCCGGAAATCCGGGGTC CCTGACCGATTCAGTGGCAGCGGGTCTGGGAC AGATTTCACTCTCACCATCACCAGCCTGCAGG CTGAAGATGTGGCAGTTTATTACTGTCAGCAA TATTATAGTACTCCATTCACTTTCGGCCCTGGG ACCAAAGTGGATATCAAA | CAGGTGCAGCTGGTGCAGTCTGGGGCTGAGGTA AAGAAGCCTGGGGCCTCAGTGACGGTCTCCTGCA AGGCTTCTGGATTCCCCTTCACCAATTATGATAT CAACTGGGTGCGACAGGCCACTGGACAAGGGCT TGAGTGGATGGGATGGATGCACCCTAACAGTGGT AACACAGGCTATGCACAGAAGTTCCAGGGCAGA GTCACCATGACCAGGAACACCTCCATAAGCACA GCCTACTTGGAGCTGAGCAGCCTGAGATCTGAGG ACACGGCCGTGTATTACTGTGCGAGTAGCAGTGG CTGGTACTTTTTTGACTACTGGGGCCAGGGAACC CTGGTCACCGTCTCCTCA |
| | | | SEQ ID NO: 26229 | SEQ ID NO: 30235 |
| | | AA | DIVMTQSPDSLAVSLGERATINCKSSQNVLYSSH NNNYLAWYQQKPGQPPKLLIYWASTRKSGVPD RFSGSGSGTDFTLTITSLQAEDVAVYYCQQYYST PFTFGPGTKVDIK | QVQLVQSGAEVKKPGASVTVSCKASGFPFTNYDIN WVRQATGQGLEWMGWMHPNSGNTGYAQKFQGR VTMTRNTSISTAYLELSSLRSEDTAVYYCASSSGWY FFDYWGQGTLVTVSS |
| | | | SEQ ID NO: 26230 | SEQ ID NO: 30236 |
| iPS:435407 | 21-225_150E7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAAT TTGCAAAGTGGGGTCCCATTAAGGTTCAGCGG CAGTGGATCTGGGACAGACTTCACTCTCACAA TCAGCAGCCTGCAGCCTGAAGATTTTGCAGCT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGAAGAAAA TAATAAATACTATGCAGACTCCGTGAAGGGCCG ATTCACCATCTCCAGAGACAATTCCAAGAACACG CTGTTTCTGCAAATGAACAGCCTGAGAGCCGAGG ACACGGCTGTGTATTACTGTGCGAGAGAAATAGG GTTCAGTGAGGACTACTGGGGCCAGGGAACCCT GGTCACCGTCTCCTCA |
| | | | SEQ ID NO: 26231 | SEQ ID NO: 30237 |

FIGURE 50

| | | | | |
|---|---|---|---|---|
| | | AA | DIQMTQSPSSLSASVGDRVTITCRASQGIRNDLG WYQQKPGKAPKRLIYAASNLQSGVPLRFSGSGS GTDFTLTISSLQPEDFAAYYCLQHNSYPLTFGGG TKVEIK | QVQLVESGGGVVQPGRSLRLSCAASGFTFSDYGM HWVRQAPGKGLEWVAVIWYEENNKYYADSVKGR FTISRDNSKNTLFLQMNSLRAEDTAVYYCAREIGFS EDYWGQGTLVTVSS |
| | | | SEQ ID NO: 26232 | SEQ ID NO: 30238 |
| IPS:435409 | 21-225_150G8 | NA | GACATCCAGATGACCCAGTCTCCATCCTCACT GTCTGCATCTGTAGGAGACAGAGTCACCATCA CTTGTCGGGCGAGTCAGGGCATTAGCCATTAT TTAGCCTGGTTTCAGCAGAAACCAGGGAAAGC CCCTAAGTCCCTGATCTATGTTGCATCCAGTTT GCAAAATGGGGTCCCATCAAGGTTCAGCGGCA GTGGATCTGGGACAGATTTCACTCTCACCATC AGCAGCCTGCAGCCTGAAGATTTTGCAACTTA TTACTGCCAACAGTATAATAATTACCCGCTCA CTTTCGGCGGAGGGACCAAGGTGGAGATCAA A | GAGGTGCAGCTGGTGGAGTCTGGGGGAGGCTTG GTGCAGCCTGGGGGGTCCCTGAGACTCTCCTGTG CAGCCTCGGGATTTACTTTCAGTACCTATAGCAT GACTTGGGTCCGCCAGGCTCCAGGGAAGGGGCT GGACTGGGTTTCATACATTAGTAGGAGTAGTAGT ACCATATACTACGCAGACTCTGTGAAGGGCCGAT TCTCCATCTCCAGAGACAATGCCAAGAACTCACT GTATCTGCAAATGAACAGCCTGAGAGACGAGGA CACGGCTCTGTATTACTGTGCGAGATCGGCATTT AGCCCTTTTGATTACTGGGGCCAGGGAACCCTGG TCACCGTCTCCTCA |
| | | | SEQ ID NO: 26233 | SEQ ID NO: 30239 |
| | | AA | DIQMTQSPSSLSASVGDRVTITCRASQGISHYLA WFQQKPGKAPKSLIYVASSLQNGVPSRFSGSGSG TDFTLTISSLQPEDFATYYCQQYNNYPLTFGGGT KVEIK | EVQLVESGGGLVQPGGSLRLSCAASGFTFSTYSMT WVRQAPGKGLDWVSYISRSSSTIYYADSVKGRFSIS RDNAKNSLYLQMNSLRDEDTALYYCARSAFSPFD YWGQGTLVTVSS |
| | | | SEQ ID NO: 26234 | SEQ ID NO: 30240 |

FIGURE 50

| | | | | |
|---|---|---|---|---|
| iPS:435413 | 21-225_150B11 | NA | GATATTGTGATGACCCAGACTCCACTCTCTCT GTCCGTCACCCCTGGACAGCCGGCCTCCATCT CCTGCAAGTCTAGTCAGAGCCTCGTGCATGGT GATGGAAAGACCTATTTGTATTGGTACCTGCA GAGGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACATTGAAAATCAGCCGGGTGGAGGCT GAGGATGTTGGGGTTTATTACTGCATGCAAAG TATACAGCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAAG ACACGGCTGTGTATTACTGTGCGAGAGATCGTTA CGATTTTTGGAGTGGTCACTTTGACTACTGGGGC CAGGGAACCCTGGTCACCGTCTCCTCA |
| | | | SEQ ID NO: 26235 | SEQ ID NO: 30241 |
| | | AA | DIVMTQTPLSLSVTPGQPASISCKSSQSLVHGDG KTYLYWYLQRPGQPPQLLIYEVSNRFSGVPDRFS GSGSGTDFTLKISRVEAEDVGVYYCMQSIQLPW TFGQGTKVEIK | QVQLVESGGGVVQPGRSLRLSCAASGFTFSSYGMH WVRQAPGKGLEWVAIIWYDGSNKYYADSVKGRFT ISRDNSKNTLYLQMNSLRAEDTAVYYCARDRYDF WSGHFDYWGQGTLVTVSS |
| | | | SEQ ID NO: 26236 | SEQ ID NO: 30242 |
| iPS:435415 | 21-225_150C11 | NA | GACATCCAGATGACCCAGTCTCCATCCTCCCT GTCTGCATCTGTAGGAGACAGAGTCACCATCA CTTGCCGGGCAAGTCAGAGCATTAGCAGCTAT TTAAATTGGTATCAGCAGAAACCAGGGAAAG CCCCTAAACTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTATTTACACT TTCGGCGGAGGGTCCAAGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGCTATTAGTGGTAGTGGTGGT AACACATTCTACGCAGACTCCGTGAAGGGCCGGT TCACCATCTCCAGAGACAATTCCAAGAACACGCT GAATCTGCAAATGAGCAGCCTGAGAGCCGAGGA CACGGCCGTATATTACTGCGCGAAACGGGTGAC GGACTACGGTGGTAACGACTGGTTCGACCCCTGG GGCCAGGGAACCCTGGTCACCGTCTCCTCA |
| | | | SEQ ID NO: 26237 | SEQ ID NO: 30243 |

FIGURE 50

| | | | | |
|---|---|---|---|---|
| | | AA | DIQMTQSPSSLSASVGDRVTITCRASQSISSYLNW YQQKPGKAPKLLIYTASSLQSGVPSRFSGSGSGT DFTLTISSLQPEDFATYYCQQSYSIYTFGGGSKVE IK | EVQLLESGGGLVQPGGSLRLSCAASGFTFSSYAMN WVRQAPGKGLEWVSAISGSGGNTFYADSVKGRFTI SRDNSKNTLNLQMSSLRAEDTAVYYCAKRVTDYG GNDWFDPWGQGTLVTVSS |
| | | | SEQ ID NO: 26238 | SEQ ID NO: 30244 |
| iPS:435417 | 21-225_150D11 | NA | GATATTGTGATGACCCAGACTCCACTCTCTCT GTCCGTCACTCCTGGACAGCCGGCCTCCATCT CCTGCAAGTCTAGTCAGAGCCTCCTGCATAGT GAGGGAAAGACCTATTTGTATTGGTACCTGCA GAAGCCAGGCCAGCCTCCACAGCTCCTGATCT ATGAAGTTTCCTACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAGG TATACAGCTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATTCTATGATGGAAGT AATAAACACTATGCAGACTCCGTGAAGGGCCGA TTCACCATCTCCAGAGACAATTCCAAGAACACGC TGTATCTGCAAATGAACAGCCTGAGAGCCGAGG ACTCGGCTGTGTATTACTGTACGAGGAGGTTTAG CAGCAGCTGGTCGGGGGGGTATGGACGTCTGGGG CCAAGGGACCACGGTCACCGTCTCCTCA |
| | | | SEQ ID NO: 26239 | SEQ ID NO: 30245 |
| | | AA | DIVMTQTPLSLSVTPGQPASISCKSSQSLLHSEGK TYLYWYLQKPGQPPQLLIYEVSYRFSGVPDRFSG SGSGTDFTLKISRVEAEDVGVYYCMQGIQLPLTF GGGTKVEIK | QVQLVESGGGVVQPGRSLRLSCAASGFTFSSYGMH WVRQAPGKGLEWVAVIFYDGSNKHYADSVKGRFT ISRDNSKNTLYLQMNSLRAEDSAVYYCTRRFSSSW SGGMDVWGQGTTVTVSS |
| | | | SEQ ID NO: 26240 | SEQ ID NO: 30246 |

FIGURE 50

| iPS:435419 | 21-225_150C12 | NA | GACATCCAGATGACCCAGTCTCCATCCTCCCT GTCTGCATCTGTAGGAGACAGAGTCACCATCA CTTGCCGGGCAAGTCAGAGCATTAGCGACTAT TTAAAATTGGTATCAGCAGAAACCAGGGAAAG CCCCTAAGGTCCTGATCTATACTACATCCAGTT TGCAAAGTGGGGTCCCATCAAGGTTCAGTGGC AGTGGATCTGGGACAGATTTCACTCTCACCAT CAGCAGTCTGCAGCCTGAAGATTTTGCAACTT ACTACTGTCAACAGAGTTTCAGTACCCCCACT TTCGGCGGAGGGACCAAGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACACT ATATCTGCAAATGAACAGCCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26241 | SEQ ID NO: 30247 |
| | | AA | DIQMTQSPSSLSASVGDRVTITCRASQSISDYLN WYQQKPGKAPKVLIYTTSSLQSGVPSRFSGSGSG TDFTLTISSLQPEDFATYYCQQSFSTPTFGGGTKV EIK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKRVTDYG GNDWFDPWGQGTLVTVSS |
| | | | SEQ ID NO: 26242 | SEQ ID NO: 30248 |
| iPS:435421 | 21-225_151F1 | NA | GAAATGGTGATGACGCAGTCTCCAGCCACCCT GTCTGTGTCTCCAGGGGAAAGAGTCACCCTCT CCTGCAGGGCCAGTCAGAGTATTAACATCAAT ATAGCCTGGTACCAGCAGAAACCTGGCCAGGC TCCCAGGCTCCTCATCTATGGTGCATCCACCA GGGCCACTGGTATCCCAGCCAGGTTCAGTGGC AGTGGGTCTGGGACAGAGTTCACTCTCACCAT CAGCAGCCTGCAGTCTGAAGATTTTGCAGTTT ATTACTGTCAGCAGTATAATGACTGGCCTCCG TGGACGTTCGGCCAAGGGACCAAGGTGGAAA TCAAA | GAGGTGCAGCTGGTGGAGTCTGGGGGAGGCCTG GTCAAGCCTGGGGGGTCCCTGAGACTCTCCTGTG CAGCCTCTGGATACACCTTCAGGAGCTTTAGCAT GAACTGGGTCCGCCAGGCTCCAGGGAAGGGGCT GGAGTGGGTCTCATCCATTAGTAGTAGTAGTTAT TACATATACTACGCAGACTCAGTGAAGGGCCGAT TCACCATCTCCAGAGACAACGCCAAGAACTCACT GTATCTGCAAATGAACAGCCTGAGAGCCGAAGA CACGGCTGTGTATTACTGTGCGAGAGATACACCA CTGGTTTACTGGGGCCAGGGAACCCTGGTCACCG TCTCCTCA |
| | | | SEQ ID NO: 26243 | SEQ ID NO: 30249 |

FIGURE 50

| | | | | |
|---|---|---|---|---|
| | | AA | EMVMTQSPATLSVSPGERVTLSCRASQSININIA WYQQKPGQAPRLLIYGASTRATGIPARFSGSGSG TEFTLTISSLQSEDFAVYYCQQYNDWPPWTFGQ GTKVEIK | EVQLVESGGGLVKPGGSLRLSCAASGYTFRSFSMN WVRQAPGKGLEWVSSISSSSYYIYYADSVKGRFTIS RDNAKNSLYLQMNSLRAEDTAVYYCARDTPLVY WGQGTLVTVSS |
| | | | SEQ ID NO: 26244 | SEQ ID NO: 30250 |
| iPS:435423 | 21-225_151G5 | NA | GATATTGTGATGACCCAGACTCCACTCTCTCT GTCCGTCACCCCTGGACAGCCGGCCTCCATCT CCTGCAAGTCTAGTCAGCGCCTCCTGCATGGT GATGGAAAGACCTATTTGTATTGGTACCTGCA GAAGCCAGGCCAGCCTCCACAGCTCCTGCTCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGGTTCCGTGGACGTTCGGCCAAGGGA CCAAGGTGGAAATCAAA | CAGGTGCAGCTGATGGAGTCTGGGGGAGGCGTG GTCCAGCCTGGGAGGTCCCTGAGACTCTCCTGTG CAGCGTCTGGATTCATCTTCAGTAGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGATA CGATTTTTGGAGTGGTCACTTTGACTACTGGGGC CAGGGAACCCTGGTCACCGTCTCCTCA |
| | | | SEQ ID NO: 26245 | SEQ ID NO: 30251 |
| | | AA | DIVMTQTPLSLSVTPGQPASISCKSSQRLLHGDG KTYLYWYLQKPGQPPQLLLYEVSNRFSGVPDRF SGSGSGTDFTLKISRVEAEDVGVYYCMQSIQVP WTFGQGTKVEIK | QVQLMESGGGVVQPGRSLRLSCAASGFIFSSYGMH WVRQAPGKGLEWVAIIWYDGSNKYYADSVKGRFT ISRDNSKNTLYLQMNSLRAEDTAVYYCARDRYDF WSGHFDYWGQGTLVTVSS |
| | | | SEQ ID NO: 26246 | SEQ ID NO: 30252 |

FIGURE 50

| iPS:435425 | 21-225_151B12 | NA | GACATACAGATGACCCAGTCTCCATCCTCCCT GTCTGCGTCTGTAGGAGACAGAGTCACCATCA CTTGCCGGGCAAGTCAGAGCATTAGCAACTTT TTAAATTGGTATCAGCAGAAACCAGGGAAAG CCCCTAAGGTCCTGATCTATACTGCATCCAGTT TGGAAAGTGGGGTCCCATCAAGGTTCAGTGGC AGTGAATCTGGGACAGATTTCACTCTCACCAT CAGCAGTCTGCAACCTGAAGATTTTGCAACTT ACTACTGTCAACAGAGTTACAGTACCCCCACT TTCGGCGGAGGGACCAGGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCACGCTCCAGGGAAGGGGCT GGAGTGGGTCTCAGCTATTAGTGGTAGTGGTAAA AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
|---|---|---|---|---|
| | | | SEQ ID NO: 26247 | SEQ ID NO: 30253 |
| | | AA | DIQMTQSPSSLSASVGDRVTITCRASQSISNFLNW YQQKPGKAPKVLIYTASSLESGVPSRFSGSESGT DFTLTISSLQPEDFATYYCQQSYSTPTFGGGTRV EIK | EVQLLESGGGLVQPGGSLRLSCAASGFTFSSYAMN WVRHAPGKGLEWVSAISGSGKNTFYADSVKGRFTI SRDNSKNTLYLQMNSLRAEDTAVYYCAKRVTDYG GNDWFDPWGQGTLVTVSS |
| | | | SEQ ID NO: 26248 | SEQ ID NO: 30254 |
| iPS:435427 | 21-225_151C9 | NA | GACATCCAGATGACCCAGTCTCCATCCTCACT GTCTGCTTCTGTAGGAGACAGAGTCACCATCA CTTGTCGGGCGAGTCAGGGCATTAGCAAGTAT TTAGCCTGGTTTCAGCAGAAACCAGGGAAAGC CCCTAAGTCTCTGATCTATGATGCATCCAGGTT GCAAAGTGGGGTCCCATCAAAGTTCAGCGGCA GTGGATCTGGGACAGATTTCACTCTCACCATC AGCAGCCTGCAGCCAGAAGATTTTGCAAGTTA TTACTGCCATCAGTATAAACATTACCCGATCA CCTTCGGCCAAGGGACACGACTGGAGATTAAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGGGGTATT ACTATGGTTCGGGGAGCTAGAAGATGACTGGTTC GACCCCTGGGGCCAGGGAACCCTGGTCACCGTCT CCTCA |
| | | | SEQ ID NO: 26249 | SEQ ID NO: 30255 |

FIGURE 50

| | | | | |
|---|---|---|---|---|
| | | AA | DIQMTQSPSSLSASVGDRVTITCRASQGISKYLA WFQQKPGKAPKSLIYDASRLQSGVPSKFSGSGSG TDFTLTISSLQPEDFASYYCHQYKHYPITFGQGT RLEIK | QVQLVESGGGVVQPGRSLRLSCAASGFTFSSYGMH WVRQAPGKGLEWVAVISYDGSNKYYADSVKGRFT ISRDNSKNTLYLQMNSLRAEDTAVYYCARGVLLW FGELEDDWFDPWGQGTLVTVSS |
| | | | SEQ ID NO: 26250 | SEQ ID NO: 30256 |
| iPS:435429 | 21-225_151A10 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTACA GAAGCCAGGCCAGCCTCCACAGCTCCTGATCT ATGAAGTTTCCCACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGATTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGTTGGCAATTATATGGTATGATGGAAGT AATAAATACTATGCAGACTCCGTGAAGGGCCGA TTCACCATCTCCAGAGACAATTCCAAGAACACGC TGTATCTACAAATGAACAGCCTGAGAGCCGAGG ACACGGCTGTGTATTACTGTGCGAGAGATCATTA CGATTTTTGGAGTGGTCACTTTGACTACTGGGGC CAGGGAACCCTGGTCACCGTCTCCTCA |
| | | | SEQ ID NO: 26251 | SEQ ID NO: 30257 |
| | | AA | DIVMTQTPLSLSVTPGQPASISCKSSQSLLHGDG KTYLYWYLQKPGQPPQLLIYEVSHRFSGVPDRFS GSGSGTDFTLKISRVEAEDVGVYYCMQSIQIPWT FGQGTKVEIK | QVQLVESGGGVVQPGRSLRLSCAASGFTFSNYGM HWVRQAPGKGLEWLAIIWYDGSNKYYADSVKGRF TISRDNSKNTLYLQMNSLRAEDTAVYYCARDHYDF WSGHFDYWGQGTLVTVSS |
| | | | SEQ ID NO: 26252 | SEQ ID NO: 30258 |

FIGURE 50

| iPS:435431 | 21-225_152D2 | NA | GACATCCAGATGACCCTGTCTCCATCCTCCCT GTCTGCATCTGTAGGAGACAGAGTCACCATCA CTTGCCGGGCAAGTCAGAGCATTAGCGACTAT TTAAATTGGTATCAGCTGAAACCAGGGAAAGC CCCTAAGGTCTTGATCTATACTACATCCAGTTT GCAAAGTGGGGTCCCATCAAGGTTCAGTGGCA GTGGATCTGGGACAGATTTCACTCTCACCATC AGCAGTCTGCAACCTGAAGATTTTGCAACTTA CTTCTGTCAACAGAGTTACAGTACCCCCACTTT CGGCGGAGGGACCAAGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAAAACACT ATATCTGCAAATGAACAGCCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26253 | SEQ ID NO: 30259 |
| | | AA | DIQMTLSPSSLSASVGDRVTITCRASQSISDYLN WYQLKPGKAPKVLIYTTSSLQSGVPSRFSGSGSG TDFTLTISSLQPEDFATYFCQQSYSTPTFGGGTKV EIK | EVQLLESGGGLVQPGGSLRLSCAASGFTFSSYAMS WVRQAPGKGLEWVSAISGRGGNTFYADSVKGRFTI SRDNSKKTLYLQMNSLRAEDTAVYYCAKRVTDYG GNDWFDPWGQGTLVTVSS |
| | | | SEQ ID NO: 26254 | SEQ ID NO: 30260 |
| iPS:435433 | 21-225_152E3 | NA | GACATCGTGATGACCCAGTCTCCAGACTCCCT GGCTGTGTCTCTGGGCGAGAGGGGCCACCATCA ACTGCAAGTCCAGCCAGAGTGTTTTACACAGC TCCAACAATTACAACTACTTAGTTTGGTACCA GCAGAAACCAGGACAGTCTCCTAAGCGGCTCA TTTACTGGGCATCTACCCGGGAATCCGGGGTC CCTGACCGATTCAGTGGCAGCGGGTCTGGGAC AGATTTCTCTCTCACCATCAGCAGCCTGCAGG CTGAAGATGTGGCAGTTTATTACTGTCAGCAA TATTACAGTACTCCATTCACTTTCGGCCCTGGG ACCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGAGTAGCAGT GGCTGGTACTTTTTTGACTACTGGGGCCAGGGAA CCCTGGTCACCGTCTCCTCA |
| | | | SEQ ID NO: 26255 | SEQ ID NO: 30261 |

FIGURE 50

| | | | | |
|---|---|---|---|---|
| | | AA | DIVMTQSPDSLAVSLGERATINCKSSQSVLHSSN NYNYLVWYQQKPGQSPKRLIYWASTRESGVPD RFSGSGSGTDFSLTISSLQAEDVAVYYCQQYYST PFTFGPGTKVDIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCASSSGW YFFDYWGQGTLVTVSS |
| | | | SEQ ID NO: 26256 | SEQ ID NO: 30262 |
| iPS:435435 | 21-225_152H3 | NA | GACATCGTGATGACCCAGTCTCCAGACTCCCT GGCTGTGTCTCTGGGCGAGAAGGCCACCATCA ACTGCAAGTCCAGCCAGAGTGTTTTGCACAGC TCCAACAATTACAACTACTTAACTTGGTACCA GCAGAAACCAGGACAGCCTCCTAAACTGCTCA TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCCCCAATTATGATA TCAACTGGGTGCGACAGGCCACTGGACAAGGCC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCATATAGCAGT GGCTGGTACTGGTTTGACTACTGGGGGCCAGGGAA CCCTGGTCACCGTCTCCTCA |
| | | | SEQ ID NO: 26257 | SEQ ID NO: 30263 |
| | | AA | DIVMTQSPDSLAVSLGEKATINCKSSQSVLHSSN NYNYLTWYQQKPGQPPKLLIYWASTRKSGVPD RFSGSGSGTDFTLTISSLQAEDVAVYYCQQYYST PCSFGQGTKLEIK | QVQLVQSGAEVKKPGASVKVSCKASGYTFPNYDIN WVRQATGQGLEWMGWMHPNSGNTGYAQKFQGR VTMTRNTSISTAYMELSSLRSEDTAVYYCAYSSGW YWFDYWGQGTLVTVSS |
| | | | SEQ ID NO: 26258 | SEQ ID NO: 30264 |

FIGURE 50

| iPS:435437 | 21-225_152F4 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATTACAACTACTTAGCTTGGTACCA GCAGAAACCAGGGCAGCCTCCTAAACTGCTCA TTTACTGGGCATCTACCCGGAAATCCGGGGTC CCTGACCGATTCAGTGGCAGCGGGTCTGGGAC AGATTTCACTCTCACCGTCAGCAGCCTGCAGG CTGAAGATGTGGCAGTTTATTACTGTCAGCAA TATTTTAATACTCCTCCCACTTTCGGCCCTGGG ACCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA CGACACGGCCGTGTATTACTGTGCGTATAGCAGT GGCTGGTACTGGTTCGACCCCTGGGGCCAGGGA ACCCTGGTCACCGTCTCCTCA |
|---|---|---|---|---|
| | | | SEQ ID NO: 26259 | SEQ ID NO: 30265 |
| | | AA | DIVMTQSPDSLAVSLGERATINCKSSQSVLYSSN NYNYLAWYQQKPGQPPKLLIYWASTRKSGVPD RFSGSGSGTDFTLTVSSLQAEDVAVYYCQQYFN TPPTFGPGTKVDIK | QVQLVQSGAEVKKPGASVKVSCKASGYTFTNYDIN WVRQATGQGLEWMGWMHPNSGNTGYAQKFQGR VTMTRNTSISTAYMELSSLRSDDTAVYYCAYSSGW YWFDPWGQGTLVTVSS |
| | | | SEQ ID NO: 26260 | SEQ ID NO: 30266 |
| iPS:435439 | 21-225_152G4 | NA | GACATCCAGATGACCCAGTCTCCATCCTCCCT GTCTGCATCTGTAGGAGACAGAGTCACCATCA CTTGCCGGGCAAGTCAGAGCATTAGCGACTAT TTAAATTGGTATCAGCAAAAACCAGGGAAAG CCCCTAAGGTCCTGATCTATACTACATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTTCTGTCAACAGAGTTACAGTACCCCCACT TTCGGCGGAGGGACCAAGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACACT ATATCTGCAAATGAACAGCCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26261 | SEQ ID NO: 30267 |

FIGURE 50

| | | | | |
|---|---|---|---|---|
| | | AA | DIQMTQSPSSLSASVGDRVTITCRASQSISDYLN WYQQKPGKAPKVLIYTTSSLQSGVPSRFSGSGSG TDFTLTISSLQPEDFATYFCQQSYSTPTFGGGTKV EIK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKRVTDYG GNDWFDPWGQGTLVTVSS |
| | | | SEQ ID NO: 26262 | SEQ ID NO: 30268 |
| iPS:435441 | 21-225_152F6 | NA | GATATTGTGATGACCCAGACTCCACTCTCTCT GTCCGTCACCCCTGGACAGCCGGCCTCCATCT CCTGCAAGTCTAGTCAGAGCCTCCGGCATGGT GATGGAAAGACCTATTTGACTTGGTACCTACA GAGGCCAGGCCAGCCTCCACAGGTCCTGATCC ATGAAATTTCCAAGCGGTTCACTGGAGTGCCA GATAGGTTCAGTGGCAGCGGGTCAGGGACAG ATTTCACACTGAACATCAGCCGGGTGGAGGCT GAGGATGTTGGCTTTTATTACTGCATGCAAAG TATACAGGTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT TATAAATACTATGCAGACTCCGTGAAGGGCCGAT TCACCATCTCCAGAGACAATTCCAAGAATACGCT GTATCTGCAAATGAACAGCCTGAGAGCCGAGGA CACGGCTGTGTATTACTGTGCGAGAGAGGGGTAC GATTTTTGGAGTGGTTACCTTGGCTACTGGGGCC AGGGAACCCTGGTCACCGTCTCCTCA |
| | | | SEQ ID NO: 26263 | SEQ ID NO: 30269 |
| | | AA | DIVMTQTPLSLSVTPGQPASISCKSSQSLRHGDG KTYLTWYLQRPGQPPQVLIHEISKRFTGVPDRFS GSGSGTDFTLNISRVEAEDVGFYYCMQSIQVPW TFGQGTKVEIK | QVQLVESGGGVVQPGRSLRLSCAASGFTFSSYGMH WVRQAPGKGLEWVAIIWYDGSYKYYADSVKGRFT ISRDNSKNTLYLQMNSLRAEDTAVYYCAREGYDF WSGYLGYWGQGTLVTVSS |
| | | | SEQ ID NO: 26264 | SEQ ID NO: 30270 |

FIGURE 50

| iPS:435443 | 21-225_152E7 | NA | GAAATTGTGTTGACGCAGTCTCCAGGCACCCT GTCTTTGTTTCCAGGGGAAAGAGCCACCCTCT CCTGCAGGGCCAGTCAGAGTGTTATCAGCAGC TACTTAGCCTGGTACCAGCAGAAACCTGGCCA GGCTCCCAGGCTCCTCATCTTTGGTGTATCTAG TAGGGCCACTGGCATCCCAGACAGGTTCAGTG GCAGTGGGTCTGGGACAGACTTCACTCTCACC ATCAGCAGACTGGAGCCTGAAGATTTTGCAGT GTATTACTGTCAACAATATGGTAGGTCACCAT TCAATTTCGGCCCTGGGACCAAAGTGGATATC AAA | CAGGTGCAGCTGCAGGAGTCGGGCCCAGGACTG GTGAAGCCTTCACAGACCCTGTCCCTCAATTGCA CTGTCTCTGGTGGCTCCATCAGCAACAGTGGTTA CTACTGGAGCTGGATCCGCCAGCACCCAGGCAA GGGCCTGGAATGGATTGGGTACAGTTATTACAGT GGGAGCACCTACTACAACCCGTCCCTCAAGAGTC GAATTACCATATCAGTAGACACGTCTAACAACCA GTTCTCCCTGAACCTGAGCTCTGTGACTGCCGCG GACACGGCCGTGTATTACTGTGCGAGAGGGGGA TATAACTGGAACCATGCTTTTGATATCTGGGGCC AAGGGACAATGGTCACCGTCTCTTCA |
| | | | SEQ ID NO: 26265 | SEQ ID NO: 30271 |
| | | AA | EIVLTQSPGTLSLFPGERATLSCRASQSVISSYLA WYQQKPGQAPRLLIFGVSSRATGIPDRFSGSGSG TDFTLTISRLEPEDFAVYYCQQYGRSPFNFGPGT KVDIK | QVQLQESGPGLVKPSQTLSLNCTVSGGSISNSGYY WSWIRQHPGKGLEWIGYSYYSGSTYYNPSLKSRITI SVDTSNNQFSLNLSSVTAADTAVYYCARGGYNWN HAFDIWGQGTMVTVSS |
| | | | SEQ ID NO: 26266 | SEQ ID NO: 30272 |
| iPS:435445 | 21-225_152F7 | NA | GACATCCAGATGACCCAGTCTCCATCCTCCCT GTCTGCATCTGTTGGAGACACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTACATCCAGT TTGCAAAGTGGGGTCCCATCAAGGTTCAGCGG CAGTGGATCTGGGACAGAATTCACTCTCACAA TCAGCAGCCTACAGCCTGAAGATTTTGCAACT TATTACTGTCTACAGCATTATAATTTCCCGTAC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATATCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGAGTA TAGCAGCAGCTGGTACGGGTACGGTATGGACGT CTGGGGCCAAGGGACCACGGTCACCGTCTCCTCA |
| | | | SEQ ID NO: 26267 | SEQ ID NO: 30273 |

FIGURE 50

| | | | | |
|---|---|---|---|---|
| | | AA | DIQMTQSPSSLSASVGDRVTITCRASQGIRNDLG WYQQKPGKAPKRLIYATSSLQSGVPSRFSGSGSG TEFTLTISSLQPEDFATYYCLQHYNFPYSFGQGT KLEIK | QVQLVESGGGVVQPGRSLRLSCAASGFTFSSYIMH WVRQAPGKGLEWVAVIWYDGSNKYYADSVKGRF TISRDNSKNTLYLQMNSLRAEDTAVYYCAREEYSS SWYGYGMDVWGQGTTVTVSS |
| | | | SEQ ID NO: 26268 | SEQ ID NO: 30274 |
| iPS:435447 | 21-225_152H7 | NA | GACATCCAGATGACCCAGTCTCCAGCTTCCGT GTCTGCATCTGTGGGAGACAGAGTCACCATAA CTTGTCGGGCGAGTCAGGATATTAGCAACTGG TTAGCCTGGTATCAGCAGAAACCAGGGAAAG CCCCTAAACTCCTGATCTATGCTGCATCCAGTT TGCAAGGTGGGGTCCCATCAAGGTTCAGCGGC AGTGGATCTGGGACAGATTTCACTCTCTCCAT CAGCACCCTGCAGCCTGAAGATTTTGCAACTT ACTATTGTCAACAGACTGACAGTTTCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAACAGCTATGCCAT GAACTGGGTCCGCCAGGCTCCAGGGAAGGGGCT GGAGTGGGTCTCAGCTATTAGTGGTAGTGGTGGT AACACATTCTACGCAGACTCCGTGAAGGGCCGCT TCACCATCTCCAGAGACAATTCCAAGAACACACT GTATCTGCAAATGAACAGCCTGAGAGCCGAGGA CACGGCCGTATATTACTGTGCGAAAAAGGATAAT GATTACGTTTGGGGGAGTCCTTACTTTGACTACT GGGGCCAGGGAACCCTGGTCACCGTCTCCTCA |
| | | | SEQ ID NO: 26269 | SEQ ID NO: 30275 |
| | | AA | DIQMTQSPASVSASVGDRVTITCRASQDISNWLA WYQQKPGKAPKLLIYAASSLQGGVPSRFSGSGS GTDFTLSISTLQPEDFATYYCQQTDSFPFTFGPGT KVDIK | EVQLLESGGGLVQPGGSLRLSCAASGFTFNSYAMN WVRQAPGKGLEWVSAISGSGGNTFYADSVKGRFTI SRDNSKNTLYLQMNSLRAEDTAVYYCAKKDNDYV WGSPYFDYWGQGTLVTVSS |
| | | | SEQ ID NO: 26270 | SEQ ID NO: 30276 |

FIGURE 50

| iPS:435449 | 21-225_152H9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCACCGG CAGTGGATCTGGGACAGAATTCACTTTCACAA TCAGCAGCCTGCAGCCTGAAGATTTTGCAACT TATTACTGTCTACAGCATAGTAATTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAGTATCTATTATAGT GGGAGCGCCTCCTACAACCCGTCCCTCAAGAGTC GAGTCACCATATCCGTAGACACGTCCAAGAACC AGTTCTCCCTGAAGCTGAGCTCTGTGACCGCCGC AGACACGGCTGTGTTTTACTGTGCGAGACTTGAT CTCCAGTGGAGTTTTGACTTCTGGGGCCAGGGAA CCCTGGTCACCGTCTCCTCA |
|---|---|---|---|---|
| | | | SEQ ID NO: 26271 | SEQ ID NO: 30277 |
| | | AA | DIQMTQSPSSLSASVGDRVTITCRASQGIRNDLG WYQQKPGKAPKRLIYAASSLQSGVPSRFTGSGS GTEFTFTISSLQPEDFATYYCLQHSNYPLTFGGGT KVEIK | QLQLQESGPGLVKPSETLSLTCTVSGGSISRSSYYW GWIRQPPGKGLEWIGSIYYSGSASYNPSLKSRVTIS VDTSKNQFSLKLSSVTAADTAVFYCARLDLQWSFD FWGQGTLVTVSS |
| | | | SEQ ID NO: 26272 | SEQ ID NO: 30278 |
| iPS:435451 | 21-225_152D10 | NA | GGCATCGTGATGACCCAGTCTCCAGACTCCCT GGCTGTGTCTCTGGGCGCGAGGGCCACCATCG ACTGCAAGTCCAGCCAGAGTGTTTTATACAGC TCCAACAATAAGAACTACTTAGCTTGGTACCA GCAGAAACCAGGACAGCCTCCTAAGTTGCTCA TTTACTGGGCATCTACCCGGGAATCCGGGGTC CCTGACCGATTCAGTGGCAGCGGATCTGGGAC AGATTTCACTCTCACCATCTACAGCCTGCAGG CTGAAGATGTGGCAGTTTATTACTGTCAGCAA TATTATCGTAGTCCTAGTTTTGGCCAGGGGAC CAAGCTGGAGATCAAA | CAGGTGCAGCTGCAGGAGTCGGGCCCAGGACTG GTGAAGCCTTCGGAGACCCTGTCCCTCACCTGCA CTGTCTCTGGTGGCTCCATCAGTAATTACTACTG GAGCTGGATCCGGCAGCCCGCCGGGAAGGGACT GGAGTGGATTGGGCGTATCGATACCAGTGGGATC ACCAACTACAACCCCTCCCTCAAGAGTCGAGTCA CCATGTCAGTCGACACGTCCAAGAACCAGTTCTC CCTGAAGCTGACCTCTGTGACCGCCGCGGACACG GCCGTGTATTACTGTGCGAGAGAGGGGGGGATTG GGAGCTACCTTCTTTGACTACTGGGGCCAGGGAA CCCTGGTCACCGTCTCCTCA |
| | | | SEQ ID NO: 26273 | SEQ ID NO: 30279 |

FIGURE 50

| | | | | |
|---|---|---|---|---|
| | | AA | GIVMTQSPDSLAVSLGARATIDCKSSQSVLYSSN NKNYLAWYQQKPGQPPKLLIYWASTRESGVPD RFSGSGSGTDFTLTIYSLQAEDVAVYYCQQYYR SPSFGQGTKLEIK | QVQLQESGPGLVKPSETLSLTCTVSGGSISNYYWS WIRQPAGKGLEWIGRIDTSGITNYNPSLKSRVTMSV DTSKNQFSLKLTSVTAADTAVYYCAREGGLGATFF DYWGQGTLVTVSS |
| | | | SEQ ID NO: 26274 | SEQ ID NO: 30280 |
| iPS:435453 | 21-225_152G10 | NA | GACATCCAGATGACCCAGTCTCCAGCTTCCGT GTCTGCATCTGTAGGAGACAGAGTCACCATAA CTTGTCGGGCGAGTCAGGGTATTAGCAACTGG TTAGCCTGGTATCAGCAGAAACCAGGGAAAG CCCCTAAGCTCCTGATCTCTGCTGCATCCAGTT TGCAAAGTGGGGTCCCATCAAGGTTCAGCGGC AATGAATCTGGGACAGATTTCACTCTCTCCAT CAGCAGCCTGCAGCCTGAAGATTTTGCAACTT ACTATTGTCAACAGACTGACAGTTTCCCATTC ACTTTCGGCCCTGGGACCAAAGTGGATGTCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGCT TCACCATCTCCAGAGACAATTCCAAGAACACGCT CTATCTGCAAATGAACAGCCTGAGAGCCGAGGA CACGGCCGTATATTACTGTGCGAAAAAGGATTAT GATTACGTTTGGGGGGAGTCCTTACTTTGACTACT GGGGCCAGGGAACCCTGGTCACCGTCTCCTCA |
| | | | SEQ ID NO: 26275 | SEQ ID NO: 30281 |
| | | AA | DIQMTQSPASVSASVGDRVTITCRASQGISNWLA WYQQKPGKAPKLLISAASSLQSGVPSRFSGNESG TDFTLSISSLQPEDFATYYCQQTDSFPFTFGPGTK VDVK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKKDYDYV WGSPYFDYWGQGTLVTVSS |
| | | | SEQ ID NO: 26276 | SEQ ID NO: 30282 |

FIGURE 50

| iPS:435455 | 21-225_152B11 | NA | GACATCCAGATGACCCAGTCTCCATCCTCCCT GTCTGCATCTGTAGGAGACAGAGTCACCATCA CTTGCCGGGCAAGTCAGAGCATTAGCGACTAT TTAAATTGGTATCAGCAGAAACCAGGGAAAG CCCCTAAGGTCCTGATCTATACTACATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTTCTGTCAACAGAGTTACAGTACCCCCACT TTCGGCGGAGGGACCAAGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACACT ATATCTGCAAATGAACAGCCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26277 | SEQ ID NO: 30283 |
| | | AA | DIQMTQSPSSLSASVGDRVTITCRASQSISDYLN WYQQKPGKAPKVLIYTTSSLQSGVPSRFSGSGSG TDFTLTISSLQPEDFATYFCQQSYSTPTFGGGTKV EIK | EVQLLESGGGLVQPGGSLRLSCAASGFTFSSYAMN WVRQAPGKGLEWVSAISGRGGNTFYADSVKGRFTI SRDNSKNTLYLQMNSLRAEDTAVYYCAKRVTDYG GNDWFDPWGQGTLVTVSS |
| | | | SEQ ID NO: 26278 | SEQ ID NO: 30284 |
| iPS:435457 | 21-225_152C11 | NA | GATATTGTGATGACCCAGGCTCCACTCTCTCT GTCCGTCACCCCTGGACAGCCGGCCTCCATCT CCTGCAAGTCTAGTCAGAGCCTCCTGCATGGT GATGGAAAGACCTATTTATATTGGTACCTGCA GAAGCCAGGCCAGCCTCCACAGATCCTGATCT ATGAAGTTTCCAACCGGTTCTCTGGAGTGCCA GATAGGTTCAGTGGCAGCGGGTCAGGGACAG ATTTCACACTGAACATCAGCCGGGTGGAGGCT GAGGATTTTGGGTTTTATTACTGCATGCAAAG TATACAGATTCCGTGGACGTTCGGCCAAGGGA CCAAGGTGGACATCAAA | CAGGTACAACTGGTGGAGTCTGGGGGAGGCGTG GTCCAGCCTGGGAGGTCCCTGAGACTCTCCTGTG CAGCGTCTGGATTCACCTTCCGTAACTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGCTTA CGATTTTTGGAGTGGTTATTTTGACTACTGGGGC CAGGGAATTCTGGTCACCGTCTCCTCA |
| | | | SEQ ID NO: 26279 | SEQ ID NO: 30285 |

FIGURE 50

| | | | | |
|---|---|---|---|---|
| | | AA | DIVMTQAPLSLSVTPGQPASISCKSSQSLLHGDG KTYLYWYLQKPGQPPQILIYEVSNRFSGVPDRFS GSGSGTDFTLNISRVEAEDFGFYYCMQSIQIPWT FGQGTKVDIK | QVQLVESGGGVVQPGRSLRLSCAASGFTFRNYGM HWVRQAPGKGLEWVAIIWYDGSNKYYADSVKGR FTISRDNSKNTLYLQMNSLRAEDTAVYYCAREAYD FWSGYFDYWGQGILVTVSS |
| | | | SEQ ID NO: 26280 | SEQ ID NO: 30286 |
| iPS:435459 | 21-225_152E12 | NA | GCCGTCGTGATGACCCAGTCTCCAGACTCCCT GGCTGTGTCTCTGGGCGAGAGGGCCACCATCA ACTGCACGTCCAGCCAGAGTATTTTACACAGC TCCAATAATTACAACTACTTAGCTTGGTACCA GCAGAAACCAGGACAGCCTCCTAAATTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAACAA TATTATAGTGGTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCTTATAGCAG TGGCTGGTACTACTTTGACTACTGGGGCCAGGGA ACCCTGGTCACCGTCTCCTCA |
| | | | SEQ ID NO: 26281 | SEQ ID NO: 30287 |
| | | AA | AVVMTQSPDSLAVSLGERATINCTSSQSILHSSN NYNYLAWYQQKPGQPPKLLIYWASTRESGVPD RFSGSGSGTDFTLTISSLQAEDVAVYYCQQYYSG PCSFGQGTKLEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAYSSGW YYFDYWGQGTLVTVSS |
| | | | SEQ ID NO: 26282 | SEQ ID NO: 30288 |

FIGURE 50

| iPS:435461 | 21-225_153A1 | NA | GACATCCAGATGACCCAGTCTCCATCCTCACT GTCTGCATCTGTAGGAGACAGAGTCACCATCA CTTGTCGGGCGAGTCAGGTCATTAGCAATTAT TTAGCCTGGTTTCAGCAGAAACCAGGGAAAGC CCCTAAGTCCCTGATCTCTGCTGCATCCAGTTT GCGAAGTGGGGTCCCATCAAACTTCAGCGGCA GTGGATCTGGGACAGATTTCACTCTCACCATC AGCAGCCTGCAGCCTGAAGATTTTGCAACTTA TTACTGCCAACAGTATCATAGTTACCCATTCA CTTTCGGCCCTGGGACCAAAGTGGATTTCAAA | GAGGTGCAGCTGTTGGAGTCTGGGGGAGGCCTG GTACAGCCTGGGGGGTCCCTGAGACTCTCCTGTG CAGCCTCTGGATTCAGCTTTAGCAGCTATGTCAT GAGTTGGGTCCGCCAGGGTCCAGGGAAGGGGCT GGAGTGGGTCTCAGCTATTAGTGGAAGTGGTGAT AGAACATACTACGCAGACTCCGTGAAGGGCCGG TTCACCATCTCCAGAGACAATTCCAAGAACACGC TGTACCTGCAAATGAACAGCCTGAGAGCCGAGG ACACGGCCGTATATTACTGTGCGCGTACGGCGAC TAAGGACTACTGGGGCCAGGGAACCCTGGTCAC CGTCTCCTCA |
| | | | SEQ ID NO: 26283 | SEQ ID NO: 30289 |
| | | AA | DIQMTQSPSSLSASVGDRVTITCRASQVISNYLA WFQQKPGKAPKSLISAASSLRSGVPSNFSGSGSG TDFTLTISSLQPEDFATYYCQQYHSYPFTFGPGT KVDFK | EVQLLESGGGLVQPGGSLRLSCAASGFSFSSYVMS WVRQGPGKGLEWVSAISGSGDRTYYADSVKGRFTI SRDNSKNTLYLQMNSLRAEDTAVYYCARTATKDY WGQGTLVTVSS |
| | | | SEQ ID NO: 26284 | SEQ ID NO: 30290 |
| iPS:435463 | 21-225_153D2 | NA | GATATTGTGATGACCCAGACTCCACTCTCTCT GTCCGTCACCCCTGGACAGCCGGCCTCCATCT CCTGCAAGTCTAGTCAGAGCCTCCGGCATGGT GATGGAAAGACCTATTTGACTTGGTACCTACA GAGGCCAGGCCAGCCTCCACAGGTCCTGATCC ATGAAGTTTCCAAGCGGTTCACTGGAGTGCCA GATAGGTTCAGTGGCAGCGGGTCAGGGACAG ATTTCACACTGAACATCAGCCGGGTGGAGGCT GAGGATGTTGGGTTTTATTACTGCATGCAAAG TATACAGGTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT TATAAATACTATGCAGACTCCGTGAAGGGCCGAT TCACCATCTCCAGAGACAATTCCAAGAACACGGT GTATCTGCAAATGAACAGCCTGAGAGCCGAGGA CACGGCTGTATATTACTGTGCGAGAGAGGGGTAC GATTTTTGGAGTGGTTACCTTGGCTACTGGGGCC AGGGAACCCTGGTCACCGTCTCCTCA |
| | | | SEQ ID NO: 26285 | SEQ ID NO: 30291 |

FIGURE 50

| | | | | |
|---|---|---|---|---|
| | | AA | DIVMTQTPLSLSVTPGQPASISCKSSQSLRHGDG KTYLTWYLQRPGQPPQVLIHEVSKRFTGVPDRFS GSGSGTDFTLNISRVEAEDVGFYYCMQSIQVPW TFGQGTKVEIK | QVQLVESGGGVVQPGRSLRLSCAASGFTFSSYGMH WVRQAPGKGLEWVAIIWYDGSYKYYADSVKGRFT ISRDNSKNTVYLQMNSLRAEDTAVYYCAREGYDF WSGYLGYWGQGTLVTVSS |
| | | | SEQ ID NO: 26286 | SEQ ID NO: 30292 |
| iPS:435465 | 21-225_153A6 | NA | GAAATTGTGTTGACGCAGTCTCCAGGCACCCT GTCTTTGTTTCCAGGGGAAAGAGCCCCCCTCT CCTGCAGGGCCAGTCAGAGTGTTATCAGCAGC TACTTAGCCTGGTACCAGCAGAAACCTGGCCA GGCTCCCAGGCTCCTCATCTTTGGTGTATCTAG TAGGGCCACTGGCATCCCAGACAGGTTCAGTG GCAGTGGGTCTGGGACAGACTTCACTCTCACC ATCAGCAGACTGGAGCCTGAAGATTTTGCAGT GTATTACTGTCAACAATATGGTAGGTCACCAT TCAATTTCGGCCCTGGGACCAAAGTGGATATC AAA | CAGGTGCAGCTGCAGGAGTCGGGCCCAGGACTG GTGAAGCCTTCACAGACCCTGTCCCTCAATTGCA CTGTCTCTGGTGGCTCCATCAGCAACAGTGGTTA CTACTGGAGCTGGATCCGCCAGCACCCAGGCAA GGGCCTGGAATGGATTGGGTACAGCTATTACAGT GGGAGCACCTACTACAACCCGTCCCTCAAGAGTC GAATTACCATATCAGTAGACACGTCTAACAACCA GTTCTCCCTGAACCTGAGCTCTGTGACTGCCGCG GACACGGCCGTGTATTACTGTGCGAGAGGGGGA TATAACTGGAACCATGCTTTTGATATCTGGGGCC AAGGGACAATGGTCACCGTCTCTTCA |
| | | | SEQ ID NO: 26287 | SEQ ID NO: 30293 |
| | | AA | EIVLTQSPGTLSLFPGERAPLSCRASQSVISSYLA WYQQKPGQAPRLLIFGVSSRATGIPDRFSGSGSG TDFTLTISRLEPEDFAVYYCQQYGRSPFNFGPGT KVDIK | QVQLQESGPGLVKPSQTLSLNCTVSGGSISNSGYY WSWIRQHPGKGLEWIGYSYYSGSTYYNPSLKSRITI SVDTSNNQFSLNLSSVTAADTAVYYCARGGYNWN HAFDIWGQGTMVTVSS |
| | | | SEQ ID NO: 26288 | SEQ ID NO: 30294 |

FIGURE 50

| iPS:435467 | 21-225_153B9 | NA | GGCATCGTGATGACCCAGTTTCCAGATTCCCT GGCTGTGTCTCTGGGCGAGAGGGCCACCATCA ACTGCAAGTCCAGCCAGAGTGTTTTATACAGC TCCAACAATAAGAACTACTTAGCTTGGTACCA GCAGAAACCAGGACAGCCTCATAAGTTGCTCA TTTACTGGGCATCTACCCGGGAATTTGGGGTC CCTGACCGATTCAGTGGCAGCGGGTGTGGGAC AGATTTCACTCTCACCATCTACAGCGTGCAGG CTGAAGATGTGGCAGTTTATTACTGTCAGCAA TATAATCGTAGTCTTAGTTTTGGCCAGGGGAC CAAGCTGGAGATCAAA | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTACTACTG GAGCTGGATCCGGCAGCCCGCCGGGAAGGGACT GGAGTGGATTGGGCGTATCGATACCAGTGGGATC ACCAACTACAACCCCTCCCTCAAGAGTCGAGTCA CCATGTCAGTCGACACGTCCAAGAACCAGTTCTC CCTGAAGCTGACCTCTGTGACCGCCGCGGACACG GCCGTGTATTACTGTGCGAGAGAGGGGGGGAGTG GGAGCTACGTACTTTGACTACTGGGGCCAGGGA ACCCTGGTCACCGTCTCCTCA |
| | | | SEQ ID NO: 26289 | SEQ ID NO: 30295 |
| | | AA | GIVMTQFPDSLAVSLGERATINCKSSQSVLYSSN NKNYLAWYQQKPGQPHKLLIYWASTREFGVPD RFSGSGCGTDFTLTIYSVQAEDVAVYYCQQYNR SLSFGQGTKLEIK | QVQLQESGPGLVKPSETLSLTCTVSGGSISSYYWSW IRQPAGKGLEWIGRIDTSGITNYNPSLKSRVTMSVD TSKNQFSLKLTSVTAADTAVYYCAREGGVGATYF DYWGQGTLVTVSS |
| | | | SEQ ID NO: 26290 | SEQ ID NO: 30296 |
| iPS:435469 | 21-225_153G9 | NA | GATATTGTGATGACCCAGACTCCCTTCTCTCTG TCCGTCACCCCTGGACAGCCGGCCTCCATCTC CTGCAAGTCTAGTCAGAGCCTCCTGCATAGTG ATGGAAAGACCTATTTGTATTGGTACCTGCAG AAGCCAGGCCAGCCTCCACAGTTCCTGATCTA TGAAGTTTCCAACCGGTTCTCTGGAGTGCCAG ATAGGTTCAGTGGCAGCGGGTCAGGGACAGA TTTCACACTGAAAATCAGCCGGGTGGAGGCTG ACGATGTTGGGGTTTATTACTGCATGCAAAAT ATAAAGTATCCGCTCACTTTCGGCGGAGGGAC CAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCACTTATATTCTATGATGGAAGT AATAAATACTATGCAGACTCCGTGAAGGGCCGA TTCACCATCTCCAGAGACAATTCCAAGAACACGC TGTATCTGCAAATAAACAGCCTGAGAGCCGAGG ACACGGCTGTGTATTTCTGTGCGCGACGCTATAG CCGCAGCTGGGCCGGGGGTATGGACGTCTGGGG CCAAGGGACCGCGGTCACCGTCTCCTCA |
| | | | SEQ ID NO: 26291 | SEQ ID NO: 30297 |

FIGURE 50

| | | | | |
|---|---|---|---|---|
| | | AA | DIVMTQTPFSLSVTPGQPASISCKSSQSLLHSDGK TYLYWYLQKPGQPPQFLIYEVSNRFSGVPDRFSG SGSGTDFTLKISRVEADDVGVYYCMQNIKYPLT FGGGTKVEIK | QVQLVESGGGVVQPGRSLRLSCAASGFTFSSYGMH WVRQAPGKGLEWVALIFYDGSNKYYADSVKGRFT ISRDNSKNTLYLQINSLRAEDTAVYFCARRYSRSW AGGMDVWGQGTAVTVSS |
| | | | SEQ ID NO: 26292 | SEQ ID NO: 30298 |
| iPS:435471 | 21-225_153F11 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATTACAAGTACTTAGCTTGGTACCA GCAGAAACCAGGACAGCCTCCTAACCTGCTCA TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGGCTGAGGTG AAGGAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAATACCTCCATAAACAC AGCCTACATGGAGCTGAGCAGCCTGAGATCTGA GGACACGGCCGTGTATTACTGTGCGTATAGCAGT GGCTGGTACTTCTTTGACAACTGGGGCCAGGGAA CCCTGGTCACCGTCTCCTCA |
| | | | SEQ ID NO: 26293 | SEQ ID NO: 30299 |
| | | AA | DIVMTQSPDSLAVSLGERATINCKSSQSVLYSSN NYKYLAWYQQKPGQPPNLLIYWASTRKSGVPD RFSGSGSGTDFTLTISSLQAEDVAVYYCQQYYST PCSFGQGTKLEIK | QVQLVQSGAEVKEPGASVKVSCKASGYTFTNYDIN WVRQATGQGLEWMGWMHPNSGNTGYAQKFQGR VTMTRNTSINTAYMELSSLRSEDTAVYYCAYSSGW YFFDNWGQGTLVTVSS |
| | | | SEQ ID NO: 26294 | SEQ ID NO: 30300 |

FIGURE 50

| iPS:435475 | 21-225_154H6 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGT TCCAACAATTACAACTATTTAGCTTGGTACCA GCAGAAACCAGGACAGCCTCCTAAGCTGCTCA TTTACTGGACATCTACCCGGAAATCCGGGGTC CCTGACCGATTCAGTGGCAGCGGGTCTGGGAC ACATTTCACTCTCTCCATCAGCAGCCTGCAGG CTGAAGATGTGGCAGTTTATTACTGTCAGCAT TATTATAGTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGTATAGCAG TGGCTGGTACATCTTTGACTACTGGGGCCAGGGA ACCCTGGTCACCGTCTCCTCA |
|---|---|---|---|---|
| | | | SEQ ID NO: 26295 | SEQ ID NO: 30301 |
| | | AA | DIVMTQSPDSLAVSLGERATINCKSSQSVLHSSN NYNYLAWYQQKPGQPPKLLIYWTSTRKSGVPD RFSGSGSGTHFTLSISSLQAEDVAVYYCQHYYST PCSFGQGTKLEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAYSSGW YIFDYWGQGTLVTVSS |
| | | | SEQ ID NO: 26296 | SEQ ID NO: 30302 |
| iPS:435479 | 21-225_154E9 | NA | GACATTCAGATGACCCTGTCTCCATCCTCCGT GTATGCATCTGTCGGGAGACAGAGTCACCATCA CTTGTCGGGCGAGTCAGGATATTAGCAACTGG TTAGCCTGGTATCAGCAGAGACCAGGGAAAG CCCCTAAGGTCCTTATCTATGCTGCATCCAGTT TGCAAAGTGGGGTCCCATCAAGATTCAGCGGC AGTGGATCTGGGACAGATTTCACTCTCACCAT CAGCAGCGTGCAGCCTGAAGATTTTGCAACTT ACTATTGTCAACAGGGTAACAGTTTCCCGCTC ACTTTCGGCGGAGGGACCAAGGTGGACATCA AA | GAGGTGAAGTTGTTGGAGTCTGGGGGAGGCTTG GTACAGCCGGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACGCT GTTTCTGCAAATGAACAGCCTGAGAGCCGAGGA CACGGCCGTATATTACTGTGCGAAAAGGGGATTT CGATTTTTGGAGTGGTTGGGGGGCTTTGACTACT GGGGCCAGGGAACCCTGGTCACCGTCTCCTCA |
| | | | SEQ ID NO: 26297 | SEQ ID NO: 30303 |

FIGURE 50

| | | | | |
|---|---|---|---|---|
| | | AA | DIQMTLSPSSVYASVGDRVTITCRASQDISNWLA WYQQRPGKAPKVLIYAASSLQSGVPSRFSGSGS GTDFTLTISSVQPEDFATYYCQQGNSFPLTFGGG TKVDIK | EVKLLESGGGLVQPGGSLRLSCAASGFTFSSYAMS WVRQAPGKGLEWVSAISGRGGNTFYADSVKGRFTI SRDNSKNTLFLQMNSLRAEDTAVYYCAKRGFRFLE WLGGFDYWGQGTLVTVSS |
| | | | SEQ ID NO: 26298 | SEQ ID NO: 30304 |
| iPS:435481 | 21-225_154A11 | NA | GACATCGTGATGACCCAGTCTCCAGACTCCCT GGCTGTGTCTCTGGGCGAGAGGGCCACCATCA ACTGCAGGTCAAGCCAGAGTGTTTTACACAGC TCCAACAATTATAACTACTTAGCTTGGTACCA GCAGAAACCAGGACAGCCTCCTAAGCTGCTCA TTTACTGGGCATCTAAACGGGATTCCGGGGTC CCTGACCGATTCAGTGGCAGCGGGTCTGGGAC AGATTTCACTCTCACCATCAGCAGCCTGCAGG CTGAGGATGTGGCAGTTTATTACTGTCAGCAA TATTTTAGTTCTCCTCGGACGTTCGGCCAAGG GACCAAGGTGGAAATCAAG | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAGGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGTAGCA GTGGCTGGTACTACTTCGACTACTGGGGCCAGGG AACCCTGGTCACCGTCTCCTCA |
| | | | SEQ ID NO: 26299 | SEQ ID NO: 30305 |
| | | AA | DIVMTQSPDSLAVSLGERATINCRSSQSVLHSSN NYNYLAWYQQKPGQPPKLLIYWASKRDSGVPD RFSGSGSGTDFTLTISSLQAEDVAVYYCQQYFSS PRTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCASSSGW YYFDYWGQGTLVTVSS |
| | | | SEQ ID NO: 26300 | SEQ ID NO: 30306 |

FIGURE 50

| iPS:435483 | 21-225_155A4 | NA | GACATCCAGATGACCCAGTCTCCAGCTTCCGT GTCTGCATCTGTAGGAGACAGAGTCACCATAA CTTGTCGGGCGAGTCAGGGTATTAGCAACTGG TTAGCCTGGTATCACCAGAAACCAGGGAAAGC CCCTAAGCTCCTGATCTATGCTGCATCCAGTTT GCAAGGTGGGGTCCCATCAAGGTTCAGCGGCA GTGGATCTGGGACAGATTTCACTCTCTCCATC AGCAGCCTGCAGCCTGAAGATTTTGCAACTTA CTATTGTCACCAGACTGACAGTTTCCCATTCAC TTTCGGCCCTGGGACCAAAGTGGATATCAAA | GAGGTGCAGCTGTTGGAGTCTGGGGGAGGCTTG GTACAGCCTGGGGGGTCCCTGAGACTCTCTTGTG CAGCCTCTGGATTCACCTTTAACAGCTATGCCAT GAGCTGGGTCCGCCAGGCTCCAGGGAAGGGGCT GGAGTGGGTCTCAGCTATTAGTGGTAGTGGTGGT AACACATTCTACGCAGACTCCGTGAAGGGCCGCT TCACCATCTCCAGAGACAATTCCAAGAACACGCT GTATCTGCAAATGAACAGCCTGAGAGCCGAGGA CACGGCCGTATATTACTGTGCGAAAAAGGATTAT GATTACGTTTGGGGGAGTCCTTACTTTGACTACT GGGGCCAGGGAACCCTGGTCACCGTCTCCTCA |
| | | | SEQ ID NO: 26301 | SEQ ID NO: 30307 |
| | | AA | DIQMTQSPASVSASVGDRVTITCRASQGISNWLA WYHQKPGKAPKLLIYAASSLQGGVPSRFSGSGS GTDFTLSISSLQPEDFATYYCHQTDSFPFTFGPGT KVDIK | EVQLLESGGGLVQPGGSLRLSCAASGFTFNSYAMS WVRQAPGKGLEWVSAISGSGGNTFYADSVKGRFTI SRDNSKNTLYLQMNSLRAEDTAVYYCAKKDYDYV WGSPYFDYWGQGTLVTVSS |
| | | | SEQ ID NO: 26302 | SEQ ID NO: 30308 |
| iPS:435485 | 21-225_155B4 | NA | GACATCCAGATGACCCAGTCTCCAGCTTCCGT GTCTGCATCTGTAGGAGACAGAGTCACCATAA CTTGTCGGGCGAGTCAGGATATTAGCAACTGG TTAGCCTGGTATCAGCAGAAACCAGGGAAAG CCCCTAAGCTCCTGATCTATGCTGCATCCAGTT TGCAAGGTGGGGTCCCATCAAGGTTCAACGGC AGTGGATCTGGGACAGATTTCACTCTCTCCAT CAGCAGCCTGCAGCCTGAAGATTTTGCAACTT ACTATTGTCACCAGACTGACAGTTTCCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AACACATTCTACGCAGACTCCGTGAAGGGCCGCT TCACCATCTCCAGAGACAATTCCAAGAACACGCT GTATCTGCAAATGAACAGCCTGAGAGCCGAGGA CACGGCCGTATATTACTGTGCGAAAAAGGATTAT GATTACGTTTGGGGGAGTCCTTACTTTGACTACT GGGGCCAGGGAACCCTGGTCACCGTCTCCTCA |
| | | | SEQ ID NO: 26303 | SEQ ID NO: 30309 |

FIGURE 50

| | | | | |
|---|---|---|---|---|
| | | AA | DIQMTQSPASVSASVGDRVTITCRASQDISNWLA WYQQKPGKAPKLLIYAASSLQGGVPSRFNGSGS GTDFTLSISSLQPEDFATYYCHQTDSFPFTFGPGT KVDIK | EVQLLESGGGLVQPGGSLRLSCAASGFTFSSYAMS WVRQAPGKGLEWVSAISGSGGNTFYADSVKGRFTI SRDNSKNTLYLQMNSLRAEDTAVYYCAKKDYDYV WGSPYFDYWGQGTLVTVSS |
| | | | SEQ ID NO: 26304 | SEQ ID NO: 30310 |
| iPS:435487 | 21-225_155C4 | NA | GACATCCAGATGACCCAGTCTCCATCCTCCCT GTCTGCATCTGTAGGAGACAGAGTCACCATCA CTTGCCGGGCAAGTCAGAGCATTAGCAGCTAT TTAAATTGGTATCAGCAGAAACCAGGGAAAG CCCCTAAGGTCCTGATCTTTACTGCATCCAGTT TGCAAAGTGGGGTCCCATCAAGATTCAGTGGC AGTGGATCTGGGACAGATTTCACTCTCACCAT CAGCAGTCTGCAACCTGAAGATTTTGCAACTT ACTACTGTCAACAGAGTTACAGTACCCCCACT TTCGGCGGAGGGACCAGGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGTCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26305 | SEQ ID NO: 30311 |
| | | AA | DIQMTQSPSSLSASVGDRVTITCRASQSISSYLNW YQQKPGKAPKVLIFTASSLQSGVPSRFSGSGSGT DFTLTISSLQPEDFATYYCQQSYSTPTFGGGTRV EIK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKRVTDYG GNDWFDPWGQGTLVTVSS |
| | | | SEQ ID NO: 26306 | SEQ ID NO: 30312 |

FIGURE 50

| | | | | |
|---|---|---|---|---|
| iPS:435489 | 21-225_155A5 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAAGCCAGGCCAGCCTCCACAGCTCCTGATTT ATGAAGTTTCCAATCGGTTCTCTGGAGTGCCA GATAGGTTCAGTGGCAGCGGTTCAGGGACAG ATTTCACACTGAAAATCAGCCGGGTGGAGGCT GAGGATGTTGGATTTTATTACTGCATGCAAAG TATACAGGTTCCGTGGACGTTCGGCCAAGGGA CCAAGGTGGAAATCAAA | CAGGTGCAGCTAGTGGAGTCTGGGGGAGACGTG GTCCAGCCTGGGAGGTCCCTGAGGCTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT AGTAAATACTATGCAGACTCCGTGAAGGGCCGA TTCACCATCTCCAGAGACAATTCCAAGAACACGC TGTATCTGCACATGAACAGCCTGAGAGCCGAGG ACACGGCAGTGTATTACTGTGCGAGAGATCGATA CGATTTTTGGAGTGGTCACTTTGACTACTGGGGC CAGGGAACCCTGGTCACCGTCTCCTCA |
| | | | SEQ ID NO: 26307 | SEQ ID NO: 30313 |
| | | AA | DIVMTQTPLSLSVTPGQPASISCKSSQSLLHGDG KTYLYWYLQKPGQPPQLLIYEVSNRFSGVPDRFS GSGSGTDFTLKISRVEAEDVGFYYCMQSIQVPW TFGQGTKVEIK | QVQLVESGGDVVQPGRSLRLSCAASGFTFSSYGMH WVRQAPGKGLEWVAIIWYDGSSKYYADSVKGRFT ISRDNSKNTLYLHMNSLRAEDTAVYYCARDRYDF WSGHFDYWGQGTLVTVSS |
| | | | SEQ ID NO: 26308 | SEQ ID NO: 30314 |
| iPS:435491 | 21-225_155E5 | NA | GACATCGTGATGACCCAGTCTCCAGACTCCCT GGCTGTGTCTCTGGGCGAGCGGGCCACCATCA ACTGCAAGTCCAGCCAGAGTGTTTTATCCAGC TCCAACAATAATAATTATTTAGCTTGGTACCA GCAGAAACCAGGACAGCCTCCTAAGCTGCTCA TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAG | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAGTACAGGCTATGCACAGAGGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGTTTAGCAGT GGCTGGTACTATTTTGACTACTGGGGCCAGGGAA CCCTGGTCACCGTCTCCTCA |
| | | | SEQ ID NO: 26309 | SEQ ID NO: 30315 |

2130

FIGURE 50

| | | | | |
|---|---|---|---|---|
| | | AA | DIVMTQSPDSLAVSLGERATINCKSSQSVLSSSN NNNYLAWYQQKPGQPPKLLIYWASTRKSGVPD RFSGSGSGTDFTLTISSLQAEDVAVYYCQQYYST PCSFGQGTKLEIK | QVQLVQSGAEVKKPGASVKVSCKASGYTFTNYDIN WVRQATGQGLEWMGWMHPNSGSTGYAQRFQGR VTMTRNTSISTAYMELSSLRSEDTAVYYCAFSSGW YYFDYWGQGTLVTVSS |
| | | | SEQ ID NO: 26310 | SEQ ID NO: 30316 |
| iPS:435495 | 21-225_155B6 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAATAATTACAACTACTTAGCTTGGTACCA GCAGAAACCAGGACAGCCCCCTAAACTGCTCA TTTACTGGACATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAACAA TATTATAGTACTCCGTGCAGTTTTGGCCAGGG GACCAAGTTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCTTATAGCAG TGGCTGGTACTACTTTGACTACTGGGGCCAGGGA ACCCTGGTCACCGTCTCCTCA |
| | | | SEQ ID NO: 26311 | SEQ ID NO: 30317 |
| | | AA | DIVMTQSPDSLAVSLGERATINCKSSQSVLHSSN NYNYLAWYQQKPGQPPKLLIYWTSTRESGVPDR FSGSGSGTDFTLTISSLQAEDVAVYYCQQYYSTP CSFGQGTKLEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAYSSGW YYFDYWGQGTLVTVSS |
| | | | SEQ ID NO: 26312 | SEQ ID NO: 30318 |

FIGURE 50

| iPS:435497 | 21-225_155H9 | NA | GAAATAGTGATGACGCAGTCTCCAGCCACCCT GTCTGTGTCTCCAGGGGAAAGAGCCACCCTCT CCTGCAGGGCCAGTCAGAGTGTTAGTAGTAAC TTAGCCTGGTACCAGCAGAAACCTGGCCAGGC TCCCAGACTCCTCATCTATGGTGCATCCACCA GGGCCACTGGTATCCCAGCCAGGTTCAGTGGC AGTGGGTCTGGGACAGAGTTCACTCTCACCAT CAGCAGCCTGCAGTCTGAAGATTTTGCAGTTT ATTACTGTCAGCAGTATGATGACTGGCCTCCG TGGACGTTCGGCCAAGGGACCAAGGTGGAAA TCAAA | GAGGTGCAACTATTGGAGTCTGGGGGAGGCTTG GTTCAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAATGGGTCTCAACTATTAGTGGTAGAGGTCTT GGCACATACTACGCAGACTCCGTGAAGGGCCGG TTCACCATCTCCAGAGACAATTCCAAGAACACGC TGTATCTGCAACTGAACAGCCTGAGAGCCGAGG ACACGGCCGTATATTACTGTGCGAAAGACCATGA CTACGGTGACTACAATATCTACTTTGACTACTGG GGCCAGGGAACCCTGGTCACCGTCTCCTCA |
| | | | SEQ ID NO: 26313 | SEQ ID NO: 30319 |
| | | AA | EIVMTQSPATLSVSPGERATLSCRASQSVSSNLA WYQQKPGQAPRLLIYGASTRATGIPARFSGSGSG TEFTLTISSLQSEDFAVYYCQQYDDWPPWTFGQ GTKVEIK | EVQLLESGGGLVQPGGSLRLSCAASGFTFSSYAMN WVRQAPGKGLEWVSTISGRGLGTYYADSVKGRFTI SRDNSKNTLYLQLNSLRAEDTAVYYCAKDHDYGD YNIYFDYWGQGTLVTVSS |
| | | | SEQ ID NO: 26314 | SEQ ID NO: 30320 |
| iPS:435499 | 21-225_156G1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTTTCACAA TCAGCAGCCTGCAGCCTGAAGATTTTGCAACT TATTACTGTCTACAGCATAGTAATTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAGTATCTATTATAGT GGGAGCGCCTCCTACAACCCGTCCCTCAAGAGTC GAGTCACCATATCCGTAGACACGTCCAAGAACC AGTTCTCCCTGAAGCTGAGCTCTGTGACCGCCGC AGACACGGCTGTGTTTTACTGTGCGAGACTTGAT CTCCAGTGGAGTTTTGACTTCTGGGGCCAGGGAA CCCTGGTCACCGTCTCCTCA |
| | | | SEQ ID NO: 26315 | SEQ ID NO: 30321 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTFTISSLQPEDFATYYCLQHSNYPLTFGGGT KVEIK | QLQLQESGPGLVKPSETLSLTCTVSGGSISRSSYYW GWIRQPPGKGLEWIGSIYYSGSASYNPSLKSRVTIS VDTSKNQFSLKLSSVTAADTAVFYCARLDLQWSFD FWGQGTLVTVSS |
| | | | SEQ ID NO: 26316 | SEQ ID NO: 30322 |
| iPS:435501 | 21-225_156H1 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATAACAACTACTTAGCTTGGTACCA GCAGAAACCAGGACAGCCTCCTAAGCTGCTCA TTTACTGGGCATCTACCCGGGAATCCGGGGTC CCTGCCCGATTCAGTGGCAGCGGGTCTGGGAC AGATTTCACTCTCACCATCAGCAGCCTGCAGG CTGAAGATGTGGCAGTTTATTACTGTCACCAA TATTATAGTACTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGGTGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGGGGGAGCAG TGGCTGGTACTACTTTGACTACTGGGGCCAGGGA ACCCTGGTCACCGTCTCCTCA |
| | | | SEQ ID NO: 26317 | SEQ ID NO: 30323 |
| | | AA | DIVMTQSPDSLAVSLGERATINCKSSQSVLYSSN NNNYLAWYQQKPGQPPKLLIYWASTRESGVPA RFSGSGSGTDFTLTISSLQAEDVAVYYCHQYYST PCSFGQGTKLEIK | QVQLVQSGAEVKKPGASVKVSCKASGYTFTNYDIN WVRQATGQGLEWMGWVHPNSGNTGYAQKFQGR VTMTRNTSISTAYMELSSLRSEDTAVYYCAGSSGW YYFDYWGQGTLVTVSS |
| | | | SEQ ID NO: 26318 | SEQ ID NO: 30324 |

FIGURE 50

| iPS:435503 | 21-225_156E4 | NA | GACATCCAGATGACCCAGTCTCCATCCTCCCT GTCTGCATCTGTAGGAGACAGAGTCACCATCA CTTGCCGGGCAAGTCAGAGCATTAGCAGCTAT TTAAAATTGGTATCAGCAGAAACCAGGGAAAG CCCCTAAGGTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTGCCCCCACT TTCGGCGGAGGGACCAAGGTGGAGATCAAA | GAGGTGCAGCTGTTGGAGTCTGGGGGAGGCTTG GTACAGCCTGGGGGGGTCCCTGAGACTCTCCTGTG CAGCCTCTGGATTCACCTTTAGCAGCTATGCCAT GAGCTGGGTCCGCCAGGCTCCAGGGAAGGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACACT ATATCTGCAAATGAACAGCCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
|---|---|---|---|---|
| | | | SEQ ID NO: 26319 | SEQ ID NO: 30325 |
| | | AA | DIQMTQSPSSLSASVGDRVTITCRASQSISSYLNW YQQKPGKAPKVLIYTASSLQSGVPSRFSGSGSGT DFTLTISSLQPEDFATYYCQQSYSAPTFGGGTKV EIK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKRVTDYG GNDWFDPWGQGTLVTVSS |
| | | | SEQ ID NO: 26320 | SEQ ID NO: 30326 |
| iPS:435505 | 21-225_157C1 | NA | GACATCCAGATGACCCAGTCTCCATCCTCACT GTCTGCATCTATAGGAGACAGAATCACCCTCA CTTGTCGGGCGAGTCAGGGCATTAGCAATTAT TTAGCCTGGTTTCAGCAGAGACCAGGGAAAGC CCCTAAGTCCCTGATCTATGCTGCATCCAGTTT GCTAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTTTCCATTCAC TTTCGGCGGAGGGACCAAGGTGGAGCTCAAA | GAGGTGCAGCTGGTGGAGTCTGGGGGAGGCCTG GTCAAGCCTGGGGGGGTCCCTGAGACTCTCCTGTG CAGCCTCTGGATTCACCTTCAGTAGCTATAGAAT GAACTGGGTCCGCCAGGCTCCAGGGAAGGGGCT GGAGTGGGTCTCATCCATGAGTAATAGTAGTAGT TCCATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGACAGGCAGCC CAGGACTACTGGGGCCAGGGAACCCTGGTCACC GTCTCCTCA |
| | | | SEQ ID NO: 26321 | SEQ ID NO: 30327 |

FIGURE 50

| | | | | |
|---|---|---|---|---|
| | | AA | DIQMTQSPSSLSASIGDRITLTCRASQGISNYLAW FQQRPGKAPKSLIYAASSLLSGVPSKFSGSGSGT DFTLTISSLQPEDFATYYCQQYNSFPFTFGGGTK VELK | EVQLVESGGGLVKPGGSLRLSCAASGFTFSSYRMN WVRQAPGKGLEWVSSMSNSSSSIYYADSVKGRFTI SRDNAKNSLYLQMNSLRAEDTAVYYCARQAAQD YWGQGTLVTVSS |
| | | | SEQ ID NO: 26322 | SEQ ID NO: 30328 |
| IPS:435509 | 21-225_157H1 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTAGTCTGGTTTCAGCAGAGACCAGGGAAAGC CCCTAGGTCCCTGATCTATGCTGCATCCAGTTT GCAAAGTGGGGTCCCATCTAAGTTCAGCGGCA GTGGATCTGGGACAGATTTCACTCTCACCATC AGCAGCCTGCAGCCTGAAGATTTTGCAACTTA TTACTGCCAACAATATCATAGTTACCCATTCA CTTTCGGCCCTGGGACCAAAGTGGATATCAAA | GAGGTGCAGCTGTTGGAGTCTGGGGGAGGGTTG GTACAGCCTGGGGGGTCCCTGAGACTCTCCTGTG CAGCCTCTGGATTCACCTTTAGCAGTTATGCCAT GAGGTGGATCCGCCAGGCTCCAGGGAAGGGGCT GCAGTGGGTCTCAGATATTAGTGGTAGTGGTGGT ACCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAGACCTACCT CTGGGGCCAGGGAACCCTGGTCACCGTCTCCTCA |
| | | | SEQ ID NO: 26323 | SEQ ID NO: 30329 |
| | | AA | DIQMTQSPSSLSASVGDRVTITCRASQDISNYLV WFQQRPGKAPRSLIYAASSLQSGVPSKFSGSGSG TDFTLTISSLQPEDFATYYCQQYHSYPFTFGPGT KVDIK | EVQLLESGGGLVQPGGSLRLSCAASGFTFSSYAMR WIRQAPGKGLQWVSDISGSGGTTYYADSVKGRFTI SRDNSKNTLYLQMNSLRAEDTAVYYCAKTYLWGQ GTLVTVSS |
| | | | SEQ ID NO: 26324 | SEQ ID NO: 30330 |

FIGURE 50

| iPS:435511 | 21-225_157C3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTCATCTATGCTGCATCCAGTT TGCAAAGTGGGGTCCCATCAAGGTTCAGCGGC AGTGGATCTGGGACAGACTTCACTCTCACAAT CAGCAGCCTGCAGCCTGATGACTTTGCAACTT ATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCTGTTATATGGTATGATGTAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGCTGGG GTTCCTCTCTGACTATTGGGGCCAGGGAACCCTG GTCACCGTCTCCTCA |
| | | | SEQ ID NO: 26325 | SEQ ID NO: 30331 |
| | | AA | DIQMTQSPSSLSASVGDRVTITCRASQGIRNDLG WYQQKPGKAPKRLIYAASSLQSGVPSRFSGSGS GTDFTLTISSLQPDDFATYYCLQHNSYPFTFGPG TKVDIK | QVQLVESGGGVVQPGRSLRLSCAASGFTFSTYGMH WVRQAPGKGLEWVAVIWYDVNNKYYADSVKGRF TISRDNSKNTLYLQMNSLRAEDTAVYYCARELGFL SDYWGQGTLVTVSS |
| | | | SEQ ID NO: 26326 | SEQ ID NO: 30332 |
| iPS:435513 | 21-225_157F3 | NA | GACATCCAGATGACCCAGTCTCCATCCTCCCT GTCTGCATCTGTAGGAGACAGAGTCACCATCA CTTGCCGGGCAAGTCAGAACATTAGCAGTTAT TTAAATTGGTATCAGCTGAAACCAGGGAAAGC CCCTAAACTCCTGATCTATACTGCATCCAGTTT GCAAAGTGGGGTCCCATCAAGGTTCAGTGGCA GTGGATCTGGGACAGATTTCACTCTCACCATC AGCAGTCTGCAACCTGAGGATTTTGCAACTTA CTACTGTCAACAGAGTTACAATACCCCCACGT GGACGTTCGGCCAAGGGACCAAGGTGGAAAT CAAA | GAGGTGCAACTGTTGGAGTCTGGGGGAGGCTTG GTACAGCCTGGGGGGGTCCCTGAGACTCTCCTGTG CAGCCTCTGAATTCACCTTTAGCACCTATGCCAT GAGCTGGGTCCGCCAGGCTCCAGGGAAGGGGCT GGAGTGGGTCTCAGTTATTAGTGGTAGTGGTGGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAAAGGAGCA GTGGCTGGTACGAGGATGCTCTTGATATCTGGGG CCAAGGGACAATGGTCACCGTCTCTTCA |
| | | | SEQ ID NO: 26327 | SEQ ID NO: 30333 |

FIGURE 50

| | | | | |
|---|---|---|---|---|
| | | AA | DIQMTQSPSSLSASVGDRVTITCRASQNISSYLN WYQLKPGKAPKLLIYTASSLQSGVPSRFSGSGSG TDFTLTISSLQPEDFATYYCQQSYNTPTWTFGQG TKVEIK | EVQLLESGGGLVQPGGSLRLSCAASEFTFSTYAMS WVRQAPGKGLEWVSVISGSGGSTYYADSVKGRFTI SRDNSKNTLYLQMNSLRAEDTAVYYCAKRSSGWY EDALDIWGQGTMVTVSS |
| | | | SEQ ID NO: 26328 | SEQ ID NO: 30334 |
| IPS:435515 | 21-225_157E4 | NA | GACATCCAGATGACCCAGTCTCCATCCTCACT GTCTGCATCTGTAGGAGACAGAGTCACCTTCA CTTGTCGGGCGAGTCAGGGCATTAGCAATTAT TTAGCCTGGTTTCAGCAGAAACCAGGGAGAGC CCCTAAGTCCCTGATCTATGCTGCATCCAGTTT GCGAAGTGGGGTCCCATCACAGTTCAGCGGCA GTGGATCTGGGACAGATTTCACTCTCACCATC AACAGCCTGCAGCCTGAAGATTTTGCAACTTA TTACTGCCAACAGTATCATAGTTATCCATTCAC TTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATACCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAAAGCCGAGGA CACGGCTGTGTATTACTGTGCGAGAGTAGCTACC TTTGACTACTGGGGCCAGGGAACCCTGGTCACCG TCTCCTCA |
| | | | SEQ ID NO: 26329 | SEQ ID NO: 30335 |
| | | AA | DIQMTQSPSSLSASVGDRVTFTCRASQGISNYLA WFQQKPGRAPKSLIYAASSLRSGVPSQFSGSGSG TDFTLTINSLQPEDFATYYCQQYHSYPFTFGPGT KVDIK | EVQLVESGGGLVKPGGSLRLSCAASGFTFSSYTMN WVRQAPGKGLEWVSSISGSSSYIYYADSVKGRFTIS RDNAKNSLYLQMNSLKAEDTAVYYCARVATFDY WGQGTLVTVSS |
| | | | SEQ ID NO: 26330 | SEQ ID NO: 30336 |

FIGURE 50

| iPS:435521 | 21-225_157H4 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATCCTGCATCCAGTTTACAAACTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGGATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATAGCATGAACTGGGTCCGCCAGGCTCCAGGGAAGGGGCTGGAGTGGGTCTCATCCATTAGTAGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATAGAGGGTCCATCTGGGGCCAGGGAACCCTGGTCACCGTCTCCTCA |
|---|---|---|---|---|
| | | | SEQ ID NO: 26331 | SEQ ID NO: 30337 |
| | | AA | DIQMTQSPSSLSASVGDRVTITCRASQGIRNDLGWYQQKPGKAPKRLIYPASSLQTGVPSRFSGSGSGTEFTLTISSLQPEDFATYYCLQDNSYPFTFGPGTKVDIK | EVQLVESGGGLVKPGGSLRLSCAASGFTFSSYSMNWVRQAPGKGLEWVSSISSSSTYIYYADSVKGRFTISRDNAKNSLYLQMNSLRAEDTAVYYCARDRGSIWGQGTLVTVSS |
| | | | SEQ ID NO: 26332 | SEQ ID NO: 30338 |
| iPS:435523 | 21-225_157G5 | NA | GACATCCAGATGACCCAGTCTCCATCCTCACTGTCTGCATCTGTAGGGGACAGAGTCACCATCACTTGTCGGGCGAGTCAGGGCATTAACAATTATTTAGCCTGGTTTCAGCAGAAACCAGGGAAAGCCCCTGAGTCCCTGATCTATGCTGCATCCAGTTTACAAAGTGGGGTCCCATCACAGTTCAGCGGCAGTGGATCTGGGACAGATTTCACTCTCACCATCAGCAGCCTGCAGCCTGAAGATTTTGCAACTTATTACTGCCAACAGTATAATAGTTATCCATTCACTTTCGGCCCTGGGACCAAAGTGGATATCAAA | GAGGTGCAGCTGTTGGAGTCTGGGGGAGGCTTGGTACAGCCGGGGGGGTCCCTGAGACTCTCCTGTGCAGCCTCTGGATTCACCTTTAGTAGCTATGTCATGAGCTGGGTCCGCCAGGCTCCAGGGAAGGGACTGGACTGGGTCTCAGCTATGAGTGGTAGTGGTGGTAGAACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTGTATTACTGTGCGAAGTATACCTGGAACGGCTACTGGGGCCAGGGAACCCTGGTCACCGTCTCCTCA |
| | | | SEQ ID NO: 26333 | SEQ ID NO: 30339 |

FIGURE 50

| | | | | |
|---|---|---|---|---|
| | | AA | DIQMTQSPSSLSASVGDRVTITCRASQGINNYLA WFQQKPGKAPESLIYAASSLQSGVPSQFSGSGSG TDFTLTISSLQPEDFATYYCQQYNSYPFTFGPGT KVDIK | EVQLLESGGGLVQPGGSLRLSCAASGFTFSSYVMS WVRQAPGKGLDWVSAMSGSGGRTYYADSVKGRF TISRDNSKNTLYLQMNSLRAEDTAVYYCAKYTWN GYWGQGTLVTVSS |
| | | | SEQ ID NO: 26334 | SEQ ID NO: 30340 |
| iPS:435525 | 21-225_157E7 | NA | GACATCCAGATGACCCAGTCTCCATCCTCCCT GTCTGCATCTGTAGGAGACAGAGTCACCATCA CTTGCCGGGCAAGTCAGAGCTTTAGCAGCTAT TTAAATTGGTATCAGCAGAAACCAGGGATAGC CCCTAAACTCCTGATCTATGCTGCATCCAGTTT GCAAAGTGGGGTCCCATCAAGGTTCAGTGGCA GTGGATCTGGGACAGATTTCACTCTCACCATC AGCAGTCTGCAACCTGAAGATTTTGCAACTTA CTACTGTCAAGAGAGTTATAGTATCCGCTTCG CCTTCGGCCAAGGGACACGACTGGAGATTAAA | CAGCTGCACCTGCAGGAGTCGGGCCCAGGACTG GTGAAGCCTTCGGAGACCCTGTCCCTCACCTGCA CTGTCTCTGGTGGCTCCATCAGCAGTGGTAGTTA CTACTGGGGCTGGATCCGCCAGCCCCCAGGGAA GGGACTGGAGTGGATTGGGAGTATCTACTATAGT GGGAGCACCTACTACAATCCGTCCCTCAAGAGTC GAGTCACCATATCCGTAGACACGTCCAAGAACC AGTTCTCCCTGAACCTGAGCTCTGTGACCGCCGC AGACACGGCTGTCTATTACTGTGCGAGACATAAA GTGGCTGGTCCCTTTGACTACTGGGGCCAGGGAA CCCTGGTCACCGTCTCCTCA |
| | | | SEQ ID NO: 26335 | SEQ ID NO: 30341 |
| | | AA | DIQMTQSPSSLSASVGDRVTITCRASQSFSSYLN WYQQKPGIAPKLLIYAASSLQSGVPSRFSGSGSG TDFTLTISSLQPEDFATYYCQESYSIRFAFGQGTR LEIK | QLHLQESGPGLVKPSETLSLTCTVSGGSISSGSYYW GWIRQPPGKGLEWIGSIYYSGSTYYNPSLKSRVTIS VDTSKNQFSLNLSSVTAADTAVYYCARHKVAGPF DYWGQGTLVTVSS |
| | | | SEQ ID NO: 26336 | SEQ ID NO: 30342 |

FIGURE 50

| iPS:435527 | 21-225_157G7 | NA | GACATTCAGATGACTCAGTCTCCATCCTCCCT GTGTGCATCAGTAGGAGACAGAGTCACCATCA CTTGCCGGGCAAGTCAGGGCATTAGAAATGAT TTAGGCTGGTATCAGCAGAAACCAGGGAAAG CCCCTAAGCGCTTGATCAATGTTGCATCCAGT TTGCAAAGTGGGGTCCCATCAAGGTTCAGCGG CAGTGGATCTGGGACAGAATTCACTCTCACAA TCAGCAGCGTGCAGCGTGAAGATTTTGCAACT TATTACTGTATACAGGATAATAGTCACCCATT CACTTTCGGCCCTGGGACCAAAGTGGAAATCA AA | GAGGTGCAACTGGTGGAGTCTGGGGGAGGCCTG GTCAAGCCTGGGGGGTCCCTGAGATTCTCCTGTG CAGCCTCTGGATTCACCTTCAGTAGTTATAGCAT GAACTGGGTCCGCCAGGCTCCAGGGAAGGGGCT GGAGTGGGTCTCATCCATTAGTGGTAGTAGTACG TACATATACTACGCAGACTCAGTGAAGGGCCGAT TCACCATCTCCAGAGACAACGCCAAGAACTCACT GTATCTGCAAATGAACAGCCTGAGAGCCGAAGA CACGGCTGTGTATTACTGTGCGAGAGATCGGGGC AGCAGCTGGGGCCAGGGAACCCTGGTCACCGTC TCCTCA |
| | | | SEQ ID NO: 26337 | SEQ ID NO: 30343 |
| | | AA | DIQMTQSPSSLCASVGDRVTITCRASQGIRNDLG WYQQKPGKAPKRLINVASSLQSGVPSRFSGSGS GTEFTLTISSVQREDFATYYCIQDNSHPFTFGPGT KVEIK | EVQLVESGGGLVKPGGSLRFSCAASGFTFSSYSMN WVRQAPGKGLEWVSSISGSSTYIYYADSVKGRFTIS RDNAKNSLYLQMNSLRAEDTAVYYCARDRGSSW GQGTLVTVSS |
| | | | SEQ ID NO: 26338 | SEQ ID NO: 30344 |
| iPS:435529 | 21-225_157H7 | NA | GACATCCAGATGACCCAGTCTCCATCCTCACT GTCTGCATCTGTAGGAGACAGAGTCACCATCA CTTGTCGGGCGAGTCAGGACATTAGCAATTTT TTAGCCTGGTTTCAGCAGAAACCAGGGAAAGC CCCTAAGTCCCTGGTCTCTACTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATCATAGTTACCCGATCA CCTTCGGCCAAGGGACACGACTGGAGATTAAA | GAGGTGCAGCTGGTGGAGTCTGGGGGAGGCCTG GTCAAGCCTGGGGGGTCCCTGAGACTCTCCTGTG CAGCCTCTGGATTCACATTCAGTAGCTATAGCAT GAACTGGGTCCGCCAGGCTCCAGGGAAGGGGCT AGAGTGGGTCTCATGCATTAGTGGTAGTAGTAGT TACATATATTATGCAGACTCAGTGAAGGGCCGAT TCACCATGTCCAGAGACAACGCCAAGAACTCACT GTATCTGCAAATGAACAGCCTGAGAGCCGAGGA CACGGCTGTGTATTACTGTGTGAGAGATCGAGGG GGCTATTGGGGCCAGGGAACCCTGGTCACCGTCT CCTCA |
| | | | SEQ ID NO: 26339 | SEQ ID NO: 30345 |

FIGURE 50

| | | | | |
|---|---|---|---|---|
| | | AA | DIQMTQSPSSLSASVGDRVTITCRASQDISNFLA WFQQKPGKAPKSLVSTASSLQSGVPSKFSGSGSG TDFTLTISSLQPEDFATYYCQQYHSYPITFGQGTR LEIK | EVQLVESGGGLVKPGGSLRLSCAASGFTFSSYSMN WVRQAPGKGLEWVSCISGSSSYIYYADSVKGRFTM SRDNAKNSLYLQMNSLRAEDTAVYYCVRDRGGY WGQGTLVTVSS |
| | | | SEQ ID NO: 26340 | SEQ ID NO: 30346 |
| iPS:435531 | 21-225_157G8 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTATATTGGTACCTGCA GAAGCCAGGCCAGTCTCCACAACTCCTGATCT ATGAAATTTCCAAGCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTACTACTGCATGCAAAG TATACAGGTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAATTATGGCAT GCACTGGGTCCGCCAGGCTCCAGGCAAGGGGCT GGAGTGGGTGGCAATTATATGGTATGATGGAAGT TATAAATACTATGCAGACTCCGTGAAGGGCCGAT TCACCGTCTCCAGAGACAATTCCAAGAACACGCT GTATCTGCAAATGAACAGCCTGAGAGCCGAGGA CACGGCTGTGTATTACTGTGCGAGAGAGGGATAC GATTTTTGGAGTGGTTTCTTTGACTCCTGGGGCCA GGGAACCCTGGTCACCGTCTCCTCA |
| | | | SEQ ID NO: 26341 | SEQ ID NO: 30347 |
| | | AA | DIVMTQTPLSLSVTPGQPASISCKSSQSLLHGDG KTYLYWYLQKPGQSPQLLIYEISKRFSGVPDRFS GSGSGTDFTLKISRVEAEDVGVYYCMQSIQVPW TFGQGTKVEIK | QVQLVESGGGVVQPGRSLRLSCAASGFTFSNYGM HWVRQAPGKGLEWVAIIWYDGSYKYYADSVKGR FTVSRDNSKNTLYLQMNSLRAEDTAVYYCAREGY DFWSGFFDSWGQGTLVTVSS |
| | | | SEQ ID NO: 26342 | SEQ ID NO: 30348 |

FIGURE 50

| | | | | |
|---|---|---|---|---|
| iPS:435533 | 21-225_157H8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGACTTCACTCTCACAA TCAGCAGCCTGCAGCCTGATGACTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCTGTTATATGGTATGATGTAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGCTGGG GTTCCTCTCTGACTACTGGGGCCAGGGAACCCTG GTCACCGTCTCCTCA |
| | | | SEQ ID NO: 26343 | SEQ ID NO: 30349 |
| | | AA | DIQMTQSPSSLSASVGDRVTITCRASQGIRNDLG WYQQKPGKAPKRLIYAASSLQSGVPSRFSGSGS GTDFTLTISSLQPDDFATYYCLQHNSYPFTFGPG TKVDIK | QVQLVESGGGVVQPGRSLRLSCAASGFTFSSYGMH WVRQAPGKGLEWVAVIWYDVNNKYYADSVKGRF TISRDNSKNTLYLQMNSLRAEDTAVYYCARELGFL SDYWGQGTLVTVSS |
| | | | SEQ ID NO: 26344 | SEQ ID NO: 30350 |
| iPS:435535 | 21-225_157H10 | NA | GACATCCAGATGACCCAGTCTCCATCCTCACT GTCTGCATCTGTAGGAGACAGAGTCACCATCA CTTGTCGGGCGAGTCAGGGCATTACCAATTAT TTAGCCTGGTTTCAGCAGAAACCAGGGAAAGC CCCTAAGTCCCTGATCTATACTGCATCCAATTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATCATAGTTACCCATTCA CTTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATACCAT GAACTGGGTCCGCCAGGCTCCAGGGAAGGGGCT GGAGTGGGTCTCATCCATTAGTGGTAGCAGTAGT TACATAAACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAGTGGCTCA CTTTGACTACTGGGGCCAGGGAACCCTGGTCACC GTCTCCTCA |
| | | | SEQ ID NO: 26345 | SEQ ID NO: 30351 |

FIGURE 50

| | | | | |
|---|---|---|---|---|
| | | AA | DIQMTQSPSSLSASVGDRVTITCRASQGITNYLA WFQQKPGKAPKSLIYTASNLQSGVPSKFSGSGSG TDFTLTISSLQPEDFATYYCQQYHSYPFTFGPGT KVDIK | EVQLVESGGGLVKPGGSLRLSCAASGFTFSSYTMN WVRQAPGKGLEWVSSISGSSSYINYADSVKGRFTIS RDNAKNSLYLQMNSLRAEDTAVYYCARVAHFDY WGQGTLVTVSS |
| | | | SEQ ID NO: 26346 | SEQ ID NO: 30352 |
| IPS:435537 | 21-225_157H12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTTGGCTGGTATCAGCAGAGACCAGGGAAAGC CCCTAAGTGCCTGATTCATGCTGCATCCAGTTT ACAGAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATTATAGTTACCCATTCAC TTTCGGCCCTGGGACCAAAGTGGATATCAAA | GAGGTGCAGTGGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTGGTAGT TACATAAACTACGCAGACTCAGTGAAGGGCCGG TTCACCATCTCCAGAGACAACGCCAAGACCTCAC TGTATCTGCAAGTGAACGGCCTGAGAGCCGAGG ACACGGCTGTGTATTACTGTGCGAGATCCAAGTT TGACTCCTGGGGCCAGGGAACCCTGGTCACCGTC TCCTCA |
| | | | SEQ ID NO: 26347 | SEQ ID NO: 30353 |
| | | AA | DIQMTQSPSSLSASVGDRVTITCRASQGIRNDFG WYQQRPGKAPKCLIHAASSLQSGVPSRFSGSGS GTEFTLTISSLQPEDFATYYCLQHYSYPFTFGPGT KVDIK | EVQWVESGGGLVKPGGSLRLSCAASGFTFSSYSMN WVRQAPGKGLEWVSSISGSGSYINYADSVKGRFTIS RDNAKTSLYLQVNGLRAEDTAVYYCARSKFDSWG QGTLVTVSS |
| | | | SEQ ID NO: 26348 | SEQ ID NO: 30354 |

FIGURE 50

| iPS:435539 | 21-225_158G1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAAT TTGCAAAGTGGGGTCCCATCAAGGTTCAGCGG CAGTGGATGTGGGACAGAATTCACTCTCACAG TCAGCAGCCTGCAGCCTGAAGATTTTGCAACT TATTACTGTCTACAGCATAATAGTTACCCGTG GACGTTCGGCCAAGGGACCAAGGTGGAAATC AAA | GAGGTGCAGCTGGTGGAATCTGGGGGAGGCCTG GTCAAGCCTGGGGGGTCCCTGAGACTCTCCTGTG CAGCCTCTGGATTCACCTTCAGTAGTTATGGCAT GAACTGGGTCCGCCAGGCTCCAGGGAAGGGGCT GGAGTGGATCTCATCCATTAGTGGTAGTGGTAGT TACATATACTACGCAGACTCAGTGAAGGGCCGAT TCACCATTTCCAGAGACAACGCCAAGAACTCACT GTATCTGCAAATGAACAGCCTGAGAGCCGAGGA CACGGCTGTGTATTACTGTGCGATTAGCAGTGGC TGGTCTTGGGGGCCAGGGAACCCTGGTCACCGTCT CCTCA |
|---|---|---|---|---|
| | | | SEQ ID NO: 26349 | SEQ ID NO: 30355 |
| | | AA | DIQMTQSPSSLSASVGDRVTITCRASQGIRNDLG WYQQKPGKAPKRLIYTASNLQSGVPSRFSGSGC GTEFTLTVSSLQPEDFATYYCLQHNSYPWTFGQ GTKVEIK | EVQLVESGGGLVKPGGSLRLSCAASGFTFSSYGMN WVRQAPGKGLEWISSISGSGSYIYYADSVKGRFTIS RDNAKNSLYLQMNSLRAEDTAVYYCAISSGWSWG QGTLVTVSS |
| | | | SEQ ID NO: 26350 | SEQ ID NO: 30356 |
| iPS:435543 | 21-225_158D4 | NA | GACATCCAGATGACCCAGTCTCCATCCTCCCT GTCTGCATCTGTAGGAGACAGAGTCACCATCA CTTGCCGGGCAAGTCAGGACATTAGAAAGGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAT TCAGCAGCCTGCAGCCTGAAGATTTTGCAACT TATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGTCAGTTATATGGTATGATGGAAGT AATAAATACTATGCAGACTCCGTGAAGGGCCGA TTCACCATCTCCAGAGACAATTCCAAGAACACAC TGTATCTGCAAATGAACAGCCTGAGAGCCGAGG ACACGGCTGTGTATTACTGTGCGAGAGAACCGTA TACTAGTGGCTGGTACGACTACGGTATGGACGTC TGGGGCCAAGGGACCACGGTCACCGTCTCCTCA |
| | | | SEQ ID NO: 26351 | SEQ ID NO: 30357 |

FIGURE 50

| | | | | | |
|---|---|---|---|---|
| | | AA | DIQMTQSPSSLSASVGDRVTITCRASQDIRKDLG WYQQKPGKAPKRLIYAASSLQSGVPSRFSGSGS GTEFTLTFSSLQPEDFATYYCLQHYSYPRTFGQG TKVEIK | QVQLVESGGGVVQPGRSLRLSCAASGFTFSDYVM HWVRQAPGKGLEWVSVIWYDGSNKYYADSVKGR FTISRDNSKNTLYLQMNSLRAEDTAVYYCAREPYT SGWYDYGMDVWGQGTTVTVSS |
| | | | SEQ ID NO: 26352 | SEQ ID NO: 30358 |
| IPS:435545 | 21-225_158F4 | NA | GACATCCAGATGACCCAGTCTCCATCCTCCCT GCCTGCATCTGTAGGAGACAGAGTCACCATCA CTTGCCGGGCAAGTCAGAACATTAGAAAGTAT TTACATTGGTATCAGTTCTTACCAGGGAAAGC CCCTAAGCTCCTGATCTATACTGCATCCACTTT ACAAAGTGGGGTCCCATCAAGGTTCAGTGGCA GCGGATCTGGGACAGATTTCACTCTCACCATC AGCAGTCTGCAACCTGAAGATTTCGCAACTTA CTACTGTCAACAGAGTTACAATATTTCATTCA CTTTCGGCCCTGGGACCAAAGTGGATATCAAA | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTCACTTCTG GAGCTGGATCCGGCAGCCCGCCGGGAAGGGACT GGAGTGGATTGGCCGTATCTATACCAGTGGGACC ACCAACTACACCCCCTCCCTCAAGAGTCGAGTCA CCATGTCAGTAGACACGTCCAAGAACCAGTTCTC CCTGAAGCTGAGCTCTGTGACCGCCGCGGACACG GCCGTGTATTACTGTGCGAGATTGAGCAGTGGCT GGTTTGACTACTGGGGCCAGGGAACCCTGGTCAC CGTCTCCTCA |
| | | | SEQ ID NO: 26353 | SEQ ID NO: 30359 |
| | | AA | DIQMTQSPSSLPASVGDRVTITCRASQNIRKYLH WYQFLPGKAPKLLIYTASTLQSGVPSRFSGSGSG TDFTLTISSLQPEDFATYYCQQSYNISFTFGPGTK VDIK | QVQLQESGPGLVKPSETLSLTCTVSGGSISSHFWSW IRQPAGKGLEWIGRIYTSGTTNYTPSLKSRVTMSVD TSKNQFSLKLSSVTAADTAVYYCARLSSGWFDYW GQGTLVTVSS |
| | | | SEQ ID NO: 26354 | SEQ ID NO: 30360 |

FIGURE 50

| iPS:435547 | 21-225_158F5 | NA | GACATTCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCAATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGGATAATAGTCACCCATT CACTTTCGGCCCTGGGACCAAAGTGGAAATCA AA<br>SEQ ID NO: 26355 | GAGGTGCAACTGGTGGAGTCTGGGGGAGGCCTG GTCAAGCCTGGGGGGTCCCTGAGATTCTCCTGTG CAGCCTCTGGATTCACCTTCAGTAGTTATAGCAT GAACTGGGTCCGCCAGGCTCCAGGGAAGGGGCT GGAGTGGGTCTCATCCATTAGTGGTAGTAGTACG TACATATACTACGCAGACTCAGTGAAGGGCCGAT TCACCATCTCCAGAGACAACGCCAAGAACTCACT GTATCTGCAAATGAACAGCCTGAGAGCCGAAGA CACGGCTGTGTATTACTGTGCGAGAGATCGGGGC AGCAGCTGGGGCCAGGGAACCCTGGTCACCGTC TCCTCA<br>SEQ ID NO: 30361 |
|---|---|---|---|---|
| | | AA | DIQMTQSPSSLSASVGDRVTITCRASQGIRNDLG WYQQKPGKAPKRLINAASSLQSGVPSRFSGSGS GTEFTLTISSLQPEDFATYYCLQDNSHPFTFGPGT KVEIK<br>SEQ ID NO: 26356 | EVQLVESGGGLVKPGGSLRFSCAASGFTFSSYSMN WVRQAPGKGLEWVSSISGSSTYIYYADSVKGRFTIS RDNAKNSLYLQMNSLRAEDTAVYYCARDRGSSW GQGTLVTVSS<br>SEQ ID NO: 30362 |
| iPS:435549 | 21-225_158H5 | NA | GACATCCAGATGATCCAGTCTCCATCCTTCCT GTTTGCATCTGTAGGAGACAGAGTTACCATCA CTTGCCGGGCAAGTCAGGGCATGAGAATTGAT TTAGGGTGGTATCAGCAGAAACCAGGAAAAG CCCCTAAGCGCCTGATTTATCGTGCATCCAGTT TGCAAAGTGGGGTCCCATCAAGGTTCAGCGGC AGTGGATGTGGGACAGAATTCACTCTCACAAT CAGCAGCGTGCAGCGTGAAGATTTTGCAAGTT ATTACTGTGTACAGCATAATAGTTACCCTCTC ACTTTCGGCGGAGGGACCAAGGTGGAGATCA AA<br>SEQ ID NO: 26357 | GAGGTGCAACTGGTGGAGTCTGGGGGAGGCCTG GTCAAGCCTGGGGGGTCCCTGAGACTCTCCTGTG CAGCCTCTGGGTTCACCTTCAGTAGCTATAGCAT GAACTGGGTCCGCCAGGCTCCAGGGAAGGGGCT GGAGTGGGTCTCATCCATCAGTGGTAGTAGTACT TACATATACTACGCAGACTCAGTGAAGGGCCGAT TCACCATCTCCAGAGACAACGCCAAGAACTCACT GCATCTGCAAATGAACAGCCTGAGAGCCGAGGA CACGGCTGTGTATTACTGTGCGAGAGATCGGGGC AGCAGCTGGGGCCAGGGAACCCTGGTCACCGTC TCCTCA<br>SEQ ID NO: 30363 |

FIGURE 50

| | | | | |
|---|---|---|---|---|
| | | AA | DIQMIQSPSFLFASVGDRVTITCRASQGMRIDLG WYQQKPGKAPKRLIYRASSLQSGVPSRFSGSGC GTEFTLTISSVQREDFASYYCVQHNSYPLTFGGG TKVEIK | EVQLVESGGGLVKPGGSLRLSCAASGFTFSSYSMN WVRQAPGKGLEWVSSISGSSTYIYYADSVKGRFTIS RDNAKNSLHLQMNSLRAEDTAVYYCARDRGSSW GQGTLVTVSS |
| | | | SEQ ID NO: 26358 | SEQ ID NO: 30364 |
| IPS:435551 | 21-225_158H6 | NA | GACATCCAGATGACCCAGTCTCCATCCTCCCT GTCTGCATCTGTAGGAGACAGAGTCACCATCA CTTGCCGGGCAAGTCAGGGCATTAGAAGTGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGACATCAA A | CAGGTGCAGCTGGTGGAGTCTGGGGGAGGCGTG GTCCAGCCTGGGAGGTCCCTGAGACTCTCCTGTG CAGTGTCTGGATTCACCTTCAGTAGCTATGGCAT GCACTGGGTCCGCCAGGCTCCAGGCAAGGGGCT GGAGTGGGTGGCAGTTATATGGTATGATGTAACT AATAAATACTATGCAGACTCCGTGAAGGGCCGA TTCACCATCTCCAGAGACAATTCCAAGAACACGC TGTATCTGCAAATGAACAGCCTGAGAGACGAGG ACACGGCTGTGTATTATTGTGTGAGAGAACTGGG ATGGGCGGAGGACTACTGGGGCCAGGGAACCCT GGTCACCGTCTCCTCA |
| | | | SEQ ID NO: 26359 | SEQ ID NO: 30365 |
| | | AA | DIQMTQSPSSLSASVGDRVTITCRASQGIRSDLG WYQQKPGKAPKRLIYTASSLQSGVPSRFSGSGSG TEFTLTISSLQPEDFATYYCLQHNSYPFTFGPGTK VDIK | QVQLVESGGGVVQPGRSLRLSCAVSGFTFSSYGMH WVRQAPGKGLEWVAVIWYDVTNKYYADSVKGRF TISRDNSKNTLYLQMNSLRDEDTAVYYCVRELGW AEDYWGQGTLVTVSS |
| | | | SEQ ID NO: 26360 | SEQ ID NO: 30366 |

FIGURE 50

| iPS:435553 | 21-225_158G8 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGGGAAAG CCCCTAAGCGCCTGATGTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | GAGGTGCAGCTGGTGGAGTCTGGGGGAGGCCTG GTCACGCCTGGGGGGTCCCTGAGACTCTCCTGTG CAGCCTCTGGATTCACCTTCAGTAGCTATACCAT GAACTGGGTCCGCCAGGCTCCAGGGAAGGGGCT GGAGTGGGTCTCATTGATTAGTGGCAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATCGAGGC AGCCTCTGGGGCCAGGGAACCCTGGTCACCGTCT CCTCA |
|---|---|---|---|---|
| | | | SEQ ID NO: 26361 | SEQ ID NO: 30367 |
| | | AA | DIQMTQSPSSLSASVGDRVTITCRASQDIRNDLG WYQQKPGKAPKRLMYTASSLQSGVPSRFSGSGS GTEFTLTISSLQPEDFATYYCLQHNSYPFTFGPGT KVDIK | EVQLVESGGGLVTPGGSLRLSCAASGFTFSSYTMN WVRQAPGKGLEWVSLISGSSSYIYYADSVKGRFTIS RDNAKNSLYLQMNSLRAEDTAVYYCARDRGSLW GQGTLVTVSS |
| | | | SEQ ID NO: 26362 | SEQ ID NO: 30368 |
| iPS:435557 | 21-225_158B12 | NA | GACATCGTGATGACCCAGTCTCCAGACTCCCC GGCTGTGTCTCTGGGCGAGAGGGCCACCATCA ACTGCAAGTCCAGCCAGAATGTTTTACACAGC TCCAACAATAACAACTACTTAACTTGGTACCA GCAGAGACCAGGACAGCCTCCTAAGTTGCTCA TTTACTGGGCATCTACCCGGGAATCCGGGGTC CCTGACCGATTCAGTGGCAGCGGGTCTGGGAC AGAATTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGCTGAGGTG AGGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG ATTCACCATGACCAGGAACACCTCCATAAGCACA GCCTACATGGAGCTGAGCAGCCTGAGATCTGAG GACACGGCCGTGTATTACTGTGCCTATAGCAGTG GCTGGTACCGCTTTGACTACTGGGGCCAGGGAAC CCTGGTCACCGTCTCCTCA |
| | | | SEQ ID NO: 26363 | SEQ ID NO: 30369 |

FIGURE 50

| | | | | |
|---|---|---|---|---|
| | | AA | DIVMTQSPDSPAVSLGERATINCKSSQNVLHSSN NNNYLTWYQQRPGQPPKLLIYWASTRESGVPDR FSGSGSGTEFTLTISSLQAEDVAVYYCQQYYSTP PTFGQGTKVEIK | QVQLVQSGAEVRKPGASVKVSCKASGYTFTNYDIN WVRQATGQGLEWMGWMHPNSGNTGYAQKFQGR FTMTRNTSISTAYMELSSLRSEDTAVYYCAYSSGW YRFDYWGQGTLVTVSS |
| | | | SEQ ID NO: 26364 | SEQ ID NO: 30370 |
| IPS:435559 | 21-225_158H12 | NA | GACATCCAGATGACCCAGTCTCCATCCTCACT GTCTGCATCTGTAGGAGACAGAGTCACCATCA CTTGTCGGGCGAGTCAGGGCATTAACAATTAT TTAGCCTGGTTTCAGCAGAAACCAGGGAAAGC CCCTAAGTCCCTGATCTCTGCTGCATCCAGTTT ACAAAGTGGGGTCCCATCAAAGTTCAGCGGCA GTGGATCTGGGACAGATTTCACTCTCAGTATC AGCAGCCTACAGCCTGAAGATTTTGCAACTTA TTACTGTCAACAGTATCATAGTTACCCATTCAC TTTCGGCCCTGGGACCAAAGTGGATATCAAA | GAGGTGCAGCTGGTGGAGTCTGGGGGAGGCTTG GTACAGCCTGGGGGGTCCCTGAGACTCTCCTGTG CAGCCTCTGGATTCACCTTTAGCAGCTATGTCAT GAGCTGGGTCCGCCAGGCTCCAGGGAAGGGGCT GGAGTGGGTCTCAGCTATTAGTGGTAGTGGTGGT AGGACAGACTACGCAGACTCCGTAAAGGGCCGG TTCACCATCTCCAGAGACAATTCCAAGAACACGC TGTATCTGCAAATGAACAGCCTGAGAGCCGAGG ACACGGCCGTATATTACTGTGCGAAAGGGGGGCT GGAACCACGACTGGGGCCAAGGGACCACGGTCA CCGTCTCCTCA |
| | | | SEQ ID NO: 26365 | SEQ ID NO: 30371 |
| | | AA | DIQMTQSPSSLSASVGDRVTITCRASQGINNYLA WFQQKPGKAPKSLISAASSLQSGVPSKFSGSGSG TDFTLSISSLQPEDFATYYCQQYHSYPFTFGPGTK VDIK | EVQLVESGGGLVQPGGSLRLSCAASGFTFSSYVMS WVRQAPGKGLEWVSAISGSGGRTDYADSVKGRFTI SRDNSKNTLYLQMNSLRAEDTAVYYCAKGGWNH DWGQGTTVTVSS |
| | | | SEQ ID NO: 26366 | SEQ ID NO: 30372 |

FIGURE 50

| iPS:435561 | 21-225_159F1 | NA | GACATCCAGATGACCCAGTCTCCATCCTCCCT GTCTGCATCGGTAGGAGACAGAGTCACCATCA CTTGCCGGGCAAGTCAGCGCGTCAGAAATGAT TTAGGCTGGTATCAGCAGAAACCAGCGAAAG CCCCTAAGCGCATCATTTTCGATGCATCCAATT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATCATAGTTTCCCGATCA CCTTCGGCCAAGGGACCCGACTGGAGATTAAA | GAGGTGCAGCTGGTGGAGTCTGGGGGAGGCCTG GTCAAGCCGGGGGGGTCCCTGAGACTCTCCTGTG CAGCCTCTGGATTCACCTTCAGTAGCTATAGAAT GAACTGGGTCCGCCAGGCTCCAGGGAAGGGGCT GGAGTGGGTCTCATCCATAAGTGGTAGTGGTAAT TACATAGACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAGGTTGGGA CGTCTGGGGCCAAGGGACCACGGTCACCGTCTCC TCA |
|---|---|---|---|---|
| | | | SEQ ID NO: 26367 | SEQ ID NO: 30373 |
| | | AA | DIQMTQSPSSLSASVGDRVTITCRASQRVRNDLG WYQQKPAKAPKRIIFDASNLQSGVPSRFSGSGSG TEFTLTISSLQPEDFATYYCLQHHSFPITFGQGTR LEIK | EVQLVESGGGLVKPGGSLRLSCAASGFTFSSYRMN WVRQAPGKGLEWVSSISGSGNYIDYADSVKGRFTI SRDNAKNSLYLQMNSLRAEDTAVYYCARGWDVW GQGTTVTVSS |
| | | | SEQ ID NO: 26368 | SEQ ID NO: 30374 |
| iPS:435563 | 21-225_159H2 | NA | GACATCCAGTTGACCCAGTCTCCATCCTCCCT GTCTGCATCTGTAGGAGACAGAGTCACCATCA CTTGCCGGGCAAGTCAGAGCATTAGCAAATAT TTAAATTGGTATCAGCAGAAACCAGGGAAAG CCCCTGAACTCCTGATCTATGCTACATCCAATT TGCAAAGTGGGGTCCCATCAAGGTTCAGTGGC AGTGGATCTGGGACAGATTTCACTCTCACCAT CAGCACTCTGCAACCTGAAGATTTTGTAACTT ACTACTGTCAACAGAGTTACAGTCTCCCGGTC ACTTTCGGCGGAGGGACCAAGGTAGAGATCA AA | CAGGTGCAGCTGGTGCAGTCTGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGATGAACCCTAACAGTG GTAACACAGGCTATGTACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGAAAGA AAACTGGGGACTACTGGGGCCAGGGAACCCTGG TCACCGTCTCCTCA |
| | | | SEQ ID NO: 26369 | SEQ ID NO: 30375 |

FIGURE 50

| | | | | |
|---|---|---|---|---|
| | | AA | DIQLTQSPSSLSASVGDRVTITCRASQSISKYLNW YQQKPGKAPELLIYATSNLQSGVPSRFSGSGSGT DFTLTISTLQPEDFVTYYCQQSYSLPVTFGGGTK VEIK | QVQLVQSGAEVKKPGASVKVSCKASGYTFTSYDIN WVRQATGQGLEWMGWMNPNSGNTGYVQKFQGR VTMTRNTSISTAYMELSSLRSEDTAVYYCARKKTG DYWGQGTLVTVSS |
| | | | SEQ ID NO: 26370 | SEQ ID NO: 30376 |
| iPS:435565 | 21-225_159C4 | NA | GACATCCAGATGACCCAGTCTCCATCCTCCCT GTCTGCATCTGTAGGAGACAGAGTCACCATCA CTTGCCAGGCGAGTCAGGACATTAGCGACTAT TTAAATTGGTATCAACAGAAACCAGGGAAAG CCCCTAAACTCCTGATCTACGATGCCTCCACTT TGGAAACAGGGGTCCCATCAAGGTTCAGTGGA AGTGGATCTGGGACAGATTTTACTTTCACCAT CAGCAGCCTGCAGCCTGAAGATATTGCAACAT ATTTCTGTCAACAATATGATAATCTCCCGATC ACCTTCGGCCAAGGGACACGACTGGAGATTAA A | CAGGTGCAGCTGGTGGAGTCTGGGGGAGGCGTG GTCCAGCCTGGGAGGTCCCTGAGACTCTCCTGTG CAGCCTCTGGATTCACCTTCAGTAACTATGGCAT GCACTGGGTCCGCCAGGCTCCAGGCAAGGGGCT GGAGTGGGTGGCAGTTATATCATATTCTGGAAAC AATAAATACTATGCAGACTCCGTGAAGGGCCGA TTCACCATCTCCAGAGACAATTCCAAGAACACGC TGTATCTGCAGTTGAACAGCCTGAGAGCTGAGGA CATGGCTGTGTATTACTGTGCGAGACGGGAGCAGC TCGTGGGGGGGCTACGGTATGGACGTCTGGGGC CACGGGACCACGGTCACCGTCTCCTCA |
| | | | SEQ ID NO: 26371 | SEQ ID NO: 30377 |
| | | AA | DIQMTQSPSSLSASVGDRVTITCQASQDISDYLN WYQQKPGKAPKLLIYDASTLETGVPSRFSGSGS GTDFTFTISSLQPEDIATYFCQQYDNLPITFGQGT RLEIK | QVQLVESGGGVVQPGRSLRLSCAASGFTFSNYGM HWVRQAPGKGLEWVAVISYSGNNKYYADSVKGR FTISRDNSKNTLYLQLNSLRAEDMAVYYCARRSSS WGGYGMDVWGHGTTVTVSS |
| | | | SEQ ID NO: 26372 | SEQ ID NO: 30378 |

FIGURE 50

| | | | | |
|---|---|---|---|---|
| iPS:435569 | 21-225_159C5 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGACTTCACTCTCACAA TCATCAGCCTGCAGCCTGATGACTTTGCAACT TATTACTGTCTACAGCATAATAGTTATCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT ACACTGGGTCCGCCAGGCTCCAGGCAAGGGGCT GGAGTGGGTGGCTGTTGTATGGTATGATGTAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGCTGGG GTTCCTCTCTGACTACTGGGGCCAGGGAACCCTG GTCACCGTCTCCTCA |
| | | | SEQ ID NO: 26373 | SEQ ID NO: 30379 |
| | | AA | DIQMTQSPSSLSASVGDRVTITCRTSQGIRNDLG WYQQKPGKAPKRLIYAASSLQSGVPSRFSGSGS GTDFTLTIISLQPDDFATYYCLQHNSYPFTFGPGT KVDIK | QVQLVESGGGVVQPGRSLRLSCAASGFTFSSYGIH WVRQAPGKGLEWVAVVWYDVNNKYYADSVKGR FTISRDNSKNTLYLQMNSLRAEDTAVYYCARELGF LSDYWGQGTLVTVSS |
| | | | SEQ ID NO: 26374 | SEQ ID NO: 30380 |
| iPS:435571 | 21-225_159C8 | NA | GACATCCAGATGACCCAGTCTCCATCCTCCCT GTCTGCATCTGTAGGAGACAGAGTCACCATCA CTTGCCGGGCAAGTCAGGACATTAGAAAGGAT TTAGGGTGGTATCAGCAGAAACCAGGGAAAG CCCCTAACCGCCTGATCTATGCTGCATCCAGTT TGCAAAGTGGGGTCCCATCAAGGTTCAGCGGC AGTGGATCTGGGACAGAATTCACTCTCACATT CAGCAGCCTGCAGCCTGAAGATTTTGCAACTT ATTACTGTCTACAGCATCATAGTTATCCTCGG ACGTTCGGCCAAGGGACCAAGGTGGAAATCA AA | CAGGTGCAACTGGTGGAGTCTGGGGGAGGCGTG GTCCAGCCTGGGAGGTCCCTGAGACTCTCCTGTG CAGCGTCTGGATTCACCTTTAGTGACTATGTCAT GCAGTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACCGTA TAATAGTGGCTGGTACGACTACGGTATGGACGTC TGGGGCCAAGGGACCACGGTCACCGTCTCCTCA |
| | | | SEQ ID NO: 26375 | SEQ ID NO: 30381 |

FIGURE 50

| | | | | |
|---|---|---|---|---|
| | | AA | DIQMTQSPSSLSASVGDRVTITCRASQDIRKDLG WYQQKPGKAPNRLIYAASSLQSGVPSRFSGSGS GTEFTLTFSSLQPEDFATYYCLQHHSYPRTFGQG TKVEIK | QVQLVESGGGVVQPGRSLRLSCAASGFTFSDYVM QWVRQAPGKGLEWVAVIWYDGSNKYYADSVKGR FTISRDNSKNTLYLQMNSLRAEDTAVYYCAREPYN SGWYDYGMDVWGQGTTVTVSS |
| | | | SEQ ID NO: 26376 | SEQ ID NO: 30382 |
| iPS:435573 | 21-225_159D8 | NA | GACATCCAGATGACCCAGTCTCCATCCTCCCT GTCTGCATCTGTTGGAGACAGAGTCACCATCA CTTGCCGGGCAAGTCGGGACATTGGAAATGAT TTAGGCTGGTATCAGCAGAAACCAGGGAAAG CCCCTAAGCGCCTGATCTCTGCTGCATCCAGTT TGCAAAGTGGGGTCCCATCAAGGTTCAGCGGC AGTGGATCTGGGACAGAATTCACTCTCACATT CAGCAGCCTGCAGCCTGAAGATTTTGCAACTT ATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGCG CAGCGTCTGGATTCACCTTCAGTAACTATGTCAT GCACTGTGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACCGTA TAGTAGTGGCTGGTACGACTACGGTATGGACGTC TGGGGCCAAGGGACCACGGTCACCGTCTCCTCA |
| | | | SEQ ID NO: 26377 | SEQ ID NO: 30383 |
| | | AA | DIQMTQSPSSLSASVGDRVTITCRASRDIGNDLG WYQQKPGKAPKRLISAASSLQSGVPSRFSGSGSG TEFTLTFSSLQPEDFATYYCLQHYSYPRTFGQGT KVEIK | QVQLVESGGGVVQPGRSLRLSCAASGFTFSNYVM HCVRQAPGKGLEWVAVIWYDGSNKYYADSVKGR FTISRDNSKNTLYLQMNSLRAEDTAVYYCAREPYS SGWYDYGMDVWGQGTTVTVSS |
| | | | SEQ ID NO: 26378 | SEQ ID NO: 30384 |

2153

FIGURE 50

| iPS:435575 | 21-225_159H11 | NA | GACATCCAGATGACCCAGTCTCCATCCTCACT GTCTGCATCTGTAGGAGACAGAGTCACCATCA CTTGTCGGGCGAGTCAGGGCATTAGCAAATAT TTAGTCTGGTTTCAGCAGAAACCAGGGAAAGC CCCTAAGTCCCTGATCTATGCTGCATCCAGTCT GCAAAGTGGGGTCCCATCAAAGTTCAGCGGCA GTGGATCTGGGACAGATTTCACTCTCACCATC AGCAACCTGCAGCCTGAAGATTTTGCAACTTA TTACTGCCAACAGTATAATAGTTACCCATTCA CTTTCGGCCCTGGGACCAAAGTGGATATCAAA | GAGGTGCAGCTGGTGGAGTCTGGGGGAGGCCTG GTCAAGCCTGGGGGGTCCCTAAGACTCTCCTGTG CAGCCTCTGGATTCACCTTCAGTAGCTATACCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTATATTACTGTGCGCGAGTGAGCTGG GCTGACTGCTGGGGCCAGGGAACCCTGGTCACC GTCTCCTCA |
| --- | --- | --- | --- | --- |
| | | | SEQ ID NO: 26379 | SEQ ID NO: 30385 |
| | | AA | DIQMTQSPSSLSASVGDRVTITCRASQGISKYLV WFQQKPGKAPKSLIYAASSLQSGVPSKFSGSGSG TDFTLTISNLQPEDFATYYCQQYNSYPFTFGPGT KVDIK | EVQLVESGGGLVKPGGSLRLSCAASGFTFSSYTMN WVRQAPGKGLEWVSSISGSSSYIYYADSVKGRFTIS RDNAKNSLYLQMNSLRAEDTAVYYCARVSWADC WGQGTLVTVSS |
| | | | SEQ ID NO: 26380 | SEQ ID NO: 30386 |
| iPS:435577 | 21-225_160B1 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGGAAGACCTATTTCTATTGGTACCTGCA GAAGCCAGGCCAGCCTCCACAGGTCCTGATCT ATGAAGTATCCAAGCGGTTCTCTGGAGTGTCA GAAAGGTTCAGTGGCAGCGGGTCAGGGACAG ATTTCACACTGAAAATCAGCCGGGTGGAGGCT GAGGATGTTGGGGTTTATTACTGCATGCAAAG TATACAGATTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT TATAAATACTATGCAGACTCCGTGAAGGGCCGAT TCACCATCTCCAGAGACAATTCCAAGAACACGCT GTATCTTCAAATGAACAGCCTGAGAGCCGAGGA CACGGCTGTGTATTACTGTGCGAGAGAGGCCTAC GATTTTTGGAGTGGTTATTATGACTACTGGGGCC AGGGAACCCTGGTCACCGTCTCCTCA |
| | | | SEQ ID NO: 26381 | SEQ ID NO: 30387 |

FIGURE 50

| | | | | |
|---|---|---|---|---|
| | | AA | DIVMTQTPLSLSVTPGQPASISCKSSQSLLHGDGKTYFYWYLQKPGQPPQVLIYEVSKRFSGVSERFSGSGSGTDFTLKISRVEAEDVGVYYCMQSIQIPWTFGQGTKVEIK | QVQLVESGGGVVQPGRSLRLSCAASGFTFSSYGMHWVRQAPGKGLEWVAVIWYDGSYKYYADSVKGRFTISRDNSKNTLYLQMNSLRAEDTAVYYCAREAYDFWSGYYDYWGQGTLVTVSS |
| | | | SEQ ID NO: 26382 | SEQ ID NO: 30388 |
| IPS:435579 | 21-225_160G1 | NA | GACATCCAGATGACCCAGTCTCCATCCTCGCTGTCTGCCTCTGTAGGAGACAGAGTCACCATCACTTGTCGGGCGAGTCAGGACATTAACAATTATTTAGCCTGGTTTCAGCAGAAACCAGGAAAAGCCCCTACGTCCCTGATCTATGCTTCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAATATCATAGTTACCCATTCACTTTCGGCCCTGGGACCAAAGTGGATATCAGA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGGCTCCAGGGAAGGGGCTGGAGTGGGTCTCAGCTATGAGTGGTAGTGGTGGTCACACATACTACGCAGACTCCGTGAAGGGCCGGTTCACCATCTCCAGAGACAATTCCAAGAACACGGTGTATCTGCAAATGAACAGCCTGAGAGCCGAGGACACGGCCGTATATTACTGTGTGAAACATGGATACAGCTGGGGGCCAGGGAACCCTGGTCACCGTCTCCTCA |
| | | | SEQ ID NO: 26383 | SEQ ID NO: 30389 |
| | | AA | DIQMTQSPSSLSASVGDRVTITCRASQDINNYLAWFQQKPGKAPTSLIYASSSLQSGVPSKFSGSGSGTDFTLTISSLQPEDFATYYCQQYHSYPFTFGPGTKVDIR | EVQLLESGGGLVQPGGSLRLSCAASGFTFSSYVMSWVRQAPGKGLEWVSAMSGSGGHTYYADSVKGRFTISRDNSKNTVYLQMNSLRAEDTAVYYCVKHGYSWGQGTLVTVSS |
| | | | SEQ ID NO: 26384 | SEQ ID NO: 30390 |

FIGURE 50

| iPS:435581 | 21-225_160H1 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGGGAAAG CCCCTAAGCGCCTGATCTATGCTGCATCCAGT TTGCAGAATGGGGTCCCATCAAGGTTCAGCGG CAGTGGATCTGGGACAGAATTCACTCTCACAA TCAGCAGCCTGCAGCCTGAAGATTTTACAACT TATTACTGTCTACAGCATAATAGTTTCCCGTGG ACGTTCGGCCAAGGGACCAAGGTGGAAATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATCGCAT GAACTGGGTCCGCCAGGCTCCAGGGAAGGGGCT GGAGTGGGTCTCATCCATTAGTAGTAGTACTGGT TACATGTACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTTCTGTGCGAGAGATAAAGAT TACTGGGGCCAGGGAACCCTGGTCACCGTCTCCT CA |
|  |  |  | SEQ ID NO: 26385 | SEQ ID NO: 30391 |
|  |  | AA | DIQMTQSPSSLSASVGDRVTITCRASQDIRNDLG WYQQKPGKAPKRLIYAASSLQNGVPSRFSGSGS GTEFTLTISSLQPEDFTTYYCLQHNSFPWTFGQG TKVEIK | EVQLVESGGGLVKPGGSLRLSCAASGFTFSSYRMN WVRQAPGKGLEWVSSISSSTGYMYYADSVKGRFTI SRDNAKNSLYLQMNSLRAEDTAVYFCARDKDYW GQGTLVTVSS |
|  |  |  | SEQ ID NO: 26386 | SEQ ID NO: 30392 |
| iPS:435583 | 21-225_160F2 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAAT TTGCAAAGTGGGGTCCCATCAAGGTTCAGCGG CAGTGGATCTGGGACAGAATTCACTCTCACAG TCAGCAGCCTGCAGCCTGAAGATTTTGCAACT TATTACTGTCTACAGCATAATAGTTACCCGTG GACGTTCGGCCAAGGGACCAAGGTGGAAATC AAA | GAGGTGCAGCTGGTGGAATCTGGGGGAGGCCTG GTCAAGCCTGGGGGGTCCCTGAGACTCTCCTGTG CAGCCTCTGGATTCACCTTCAGTAGTTATGGCAT GAACTGGGTCCGCCAGGCTCCAGGGAAGGGGCT GGAGTGGATCTCATCCATTAGTGGTAGTGGTAGT TACATATACTACGCAGACTCAGTGAAGGGCCGAT TCACCATTTCCAGAGACAACGCCAAGAACTCACT GTATCTGCAAATGAACAGCCTGAGAGCCGAGGA CACGGCTGTGTATTACTGTGCGATTAGCAGTGGC TGGTCTTGGGGCCAGGGAACCCTGGTCACCGTCT CCTCA |
|  |  |  | SEQ ID NO: 26387 | SEQ ID NO: 30393 |

FIGURE 50

| | | | | |
|---|---|---|---|---|
| | | AA | DIQMTQSPSSLSASVGDRVTITCRASQGIRNDLG WYQQKPGKAPKRLIYTASNLQSGVPSRFSGSGS GTEFTLTVSSLQPEDFATYYCLQHNSYPWTFGQ GTKVEIK | EVQLVESGGGLVKPGGSLRLSCAASGFTFSSYGMN WVRQAPGKGLEWISSISGSGSYIYYADSVKGRFTIS RDNAKNSLYLQMNSLRAEDTAVYYCAISSGWSWG QGTLVTVSS |
| | | | SEQ ID NO: 26388 | SEQ ID NO: 30394 |
| IPS:435585 | 21-225_160G3 | NA | GACATCCAGATGACCCAGTCTCCATCCTCGCT GTCTGCCTCTGTAGGAGACAGAGTCACCATCA CTTGTCGGGCGAGTCAGGACATTAACAATTAT TTAGCCTGGTTTCAGCAGAGACCAGGGAAAGC CCCTACGTCCCTGATCTATGCTTCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAATATCATAGTTACCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGTCAT GAGCTGGGTCCGCCAGACTCCAGGGAAGGGACT GGAGTGGGTCTCAGCTATGAGTGGTAGTGGTGGT CACACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCATATATTACTGTGTGAAACATGGATA CAGCTGGGGCCAGGGAACCCTGGTCACCGTCTCC TCA |
| | | | SEQ ID NO: 26389 | SEQ ID NO: 30395 |
| | | AA | DIQMTQSPSSLSASVGDRVTITCRASQDINNYLA WFQQRPGKAPTSLIYASSSLQSGVPSKFSGSGSG TDFTLTISSLQPEDFATYYCQQYHSYPFTFGPGT KVDIK | EVQLLESGGGLVQPGGSLRLSCAASGFTFSSYVMS WVRQTPGKGLEWVSAMSGSGGHTYYADSVKGRF TISRDNSKNTLYLQMNSLRAEDTAIYYCVKHGYSW GQGTLVTVSS |
| | | | SEQ ID NO: 26390 | SEQ ID NO: 30396 |

FIGURE 50

| iPS:435587 | 21-225_160H3 | NA | GACATCCAGATGACCCAGTCTCCATCCTCCCT GTCTGCATCGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATTTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAGTAATTACCCGCTC ACTTTCGGCGGAGGGACCCAGGTGGAGAGCA AA | CAGCTGCAGCTGCAGGAGTCGGGCCCAGGACTG GTGAAGCCTTCGGAGACCCTGTCCCTCACCTGCA CCGTCTCTGGTGGCTCCATCAGCAGAAGTAGTTA CTACTGGGGCTGGATCCGCCAGCCCCCAGGGAA GGGACTGGAGTGGATTGGGAGTATCTATTATAGT GGGAGTACCTCCTACAACCCGTCTCTCGAGAGTC GAGTTACCATATCCGTAGACACGTCCAAGAACCA GTTCTCCCTGAAGCTGAGCTCTGTGACCGCCGCC GACACGGCTGTGTTTTACTGTGCGAGACTCTCTC AACGGTGGGACTTTGACTACTGGGGCCAGGGAA CCCTGGTCACCGTCTCCTCA |
|---|---|---|---|---|
| | | | SEQ ID NO: 26391 | SEQ ID NO: 30397 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHSNYPLTFGGG TQVESK | QLQLQESGPGLVKPSETLSLTCTVSGGSISRSSYYW GWIRQPPGKGLEWIGSIYYSGSTSYNPSLESRVTISV DTSKNQFSLKLSSVTAADTAVFYCARLSQRWDFDY WGQGTLVTVSS |
| | | | SEQ ID NO: 26392 | SEQ ID NO: 30398 |
| iPS:435589 | 21-225_160A4 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATAATAACTACTTAGCTTGGTACCA GCAGAAACCAGGACAGCCTCCTAAGCTACTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAATAGTCCGTGCAGTTTTGGCCAGGG GACCAAGCTGGAGATCAAA | CAGGTGCAGCTGGTGCAGTCTGGGACTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGAC CTGAGTGGATGGGATGGATGCACCCTAACAGTG GTAACACAGGCTATCCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGTATAGCAG CGGCTGGTACATTTTTGACTACTGGGGCCAGGGA ACCCTGGTCACCGTCTCCTCA |
| | | | SEQ ID NO: 26393 | SEQ ID NO: 30399 |

FIGURE 50

| | | | | |
|---|---|---|---|---|
| | | AA | DIVMTQSPDSLAVSLGERATINCKSSQSVLHSSN NNNYLAWYQQKPGQPPKLLIYWASTRESGVPD RFSGSGSGTDFTLTISSLQAEDVAVYYCQQYYNS PCSFGQGTKLEIK | QVQLVQSGTEVKKPGASVKVSCKASGYTFTNYDIN WVRQATGQGPEWMGWMHPNSGNTGYPQKFQGR VTMTRNTSISTAYMELSSLRSEDTAVYYCAYSSGW YIFDYWGQGTLVTVSS |
| | | | SEQ ID NO: 26394 | SEQ ID NO: 30400 |
| iPS:435591 | 21-225_160C4 | NA | GACATCCAGATGACCCAGTCTCCATCCTCCCT GTCTGCATCTGTAGGAGACAGAGTCACCATCA CTTGCCGGGCAAGTCAGGACATTAGAAAGGAT TTAGGGTGGTATCAGCAGAAACCAGGGAAAG CCCCTAACCGCCTGATCTATGCTGCATCCAGTT TGCAAAGTGGGGTCCCATCAAGGTTCAGCGGC AGTGGATCTGGGACAGAATTCACTCTCACATT CAGCAGCCTGCAGCCTGAAGATTTTGCAACTT ATTACTGTCTACAGCATTATAGTTATCCTCGGA CGTTCGGCCAAGGGACCAAGGTGGAAATCAA A | CAGGTGCAACTGGTGGAGTCTGGGGGAGGCGTG GTCCAGCCTGGGAGGTCCCTGAGACTCTCCTGTG CAGCGTCTGGATTCACCTTTAGTGACTATGTCAT GCAGTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACCGTA TAATAGTGGCTGGTACGACTACGGTATGGACGTC TGGGGCCAAGGGACCACGGTCACCGTCTCCTCA |
| | | | SEQ ID NO: 26395 | SEQ ID NO: 30401 |
| | | AA | DIQMTQSPSSLSASVGDRVTITCRASQDIRKDLG WYQQKPGKAPNRLIYAASSLQSGVPSRFSGSGS GTEFTLTFSSLQPEDFATYYCLQHYSYPRTFGQG TKVEIK | QVQLVESGGGVVQPGRSLRLSCAASGFTFSDYVM QWVRQAPGKGLEWVAVIWYDGSNKYYADSVKGR FTISRDNSKNTLYLQMNSLRAEDTAVYYCAREPYN SGWYDYGMDVWGQGTTVTVSS |
| | | | SEQ ID NO: 26396 | SEQ ID NO: 30402 |

FIGURE 50

| iPS:435593 | 21-225_160F4 | NA | GACATTCAGATGACCCAGTCTCCATCCTCCCC GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCTTGATCAATGTTGCATCCAGT TTGCAAAGTGGGGTCCCATCAAGGTTCAGCGG CAGTGGATCTGGGACAGAATTCACTCTTACAA TCAGCAGCGTGCAGCCTGAAGATTTTGCAACT TATTACTGTATACAGGATAATAGTCACCCATT CACTTTCGGCCCTGGGACCAAAGTGGAAATCA AA | GAGGTGCAACTGGTGGAGTCTGGGGGAGGCCTG GTCAAGCCTGGGGGGTCCCTGAGATTCTCCTGTG CAGCCTCTGGATTCACCTTCAGTAGTTATAGCAT GAACTGGGTCCGCCAGGCTCCAGGGAAGGGGCT GGAGTGGGTCTCATCCATTAGTGGTAGTAGTACG TACATATACTACGCAGACTCAGTGAAGGGCCGAT TCACCATCTCCAGAGACAACGCCAAGAACTCACT GTATCTGCAAATGAACAGCCTGAGAGCCGAAGA CACGGCTGTGTATTACTGTGCGAGAGATCGGGGC AGCAGCTGGGGCCAGGGAACCCTGGTCACCGTC TCCTCA |
|---|---|---|---|---|
| | | | SEQ ID NO: 26397 | SEQ ID NO: 30403 |
| | | AA | DIQMTQSPSSPSASVGDRVTITCRASQGIRNDLG WYQQKPGKAPKRLINVASSLQSGVPSRFSGSGS GTEFTLTISSVQPEDFATYYCIQDNSHPFTFGPGT KVEIK | EVQLVESGGGLVKPGGSLRFSCAASGFTFSSYSMN WVRQAPGKGLEWVSSISGSSTYIYYADSVKGRFTIS RDNAKNSLYLQMNSLRAEDTAVYYCARDRGSSW GQGTLVTVSS |
| | | | SEQ ID NO: 26398 | SEQ ID NO: 30404 |
| iPS:435595 | 21-225_160H4 | NA | GACATCCAGATGACCCAGTCTCCATCCTCACT GTCTGCATCTGTAGGAGACAGAGTCACCATCA CTTGTCGGGCGAGTCAGGACATTAGTAATTAT TTAGTCTGGTTTCAGCAGAAATTAGGGAAAGC CCCTAAGTCCCTGATCTATGTTGCATCCAGTTT GCAAAGTGGGGTCCCATCAAGGTTCAGCGGCA GTGGATCTGGGACAGATTTCACTCTCACCATC AGCAGCCTGCAGCCTGAAGATTTTGCAACTTA TTACTGCCAACAGTATAATAGTTACCCTCTCA CTTTCGGCGGAGGGACCAAAGTAGAGATCAA A | GAGGTGCAGCTGGTGGAGTCTGGGGGAGGCCTG GTCAAGTCTGGGGGGTCCCTGAGACTCTCCTGTG CAGCCTCTGGATTCACCTTCAGTAGCTATAGGAT GAACTGGGTCCGCCAGGCTCCAGGGAAGGGGCT GGAGTGGGTCTCATCCATTAGTGGTAGTAGTAGT TACATAGACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAAAGAGTTG GTTTGACTACTGGGGCCAGGGAACCCTGGTCACC GTCTCCTCA |
| | | | SEQ ID NO: 26399 | SEQ ID NO: 30405 |

FIGURE 50

| | | | | |
|---|---|---|---|---|
| | | AA | DIQMTQSPSSLSASVGDRVTITCRASQDISNYLV WFQQKLGKAPKSLIYVASSLQSGVPSRFSGSGSG TDFTLTISSLQPEDFATYYCQQYNSYPLTFGGGT KVEIK | EVQLVESGGGLVKSGGSLRLSCAASGFTFSSYRMN WVRQAPGKGLEWVSSISGSSSYIDYADSVKGRFTIS RDNAKNSLYLQMNSLRAEDTAVYYCARKSWFDY WGQGTLVTVSS |
| | | | SEQ ID NO: 26400 | SEQ ID NO: 30406 |
| iPS:435599 | 21-225_160B10 | NA | GACATCCAGATGACCCAGTCTCCATCCTCCCC GTCTGCATCTGTAGGAGACAGAGTCACCATCA CTTGCCGGGCAAGTCAGGGCATTAGAAATGAT TTAGGCTGGTATCAGCAGAAACCAGGGACAG CCCCTAAGCGCCTTATCTATGCTGCATCCAGTT TGCAAAGTGGGGTCCCATCAAGGTTCAGCGGC AGTGGATCTGGGACAGAATTCACTCTCACAAT CAGCAACCTGCAGCCTGAGGATTTTGCAACTT ATTACTGTCTACAGCATAGTAGTTACCCGCTC ACTTTCGGCGGCGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGAAGTAGCTA CTACTGGGGCTGGATCCGCCAGTACCCAGGGAA GGGGCTGGAGTGGATTGGGAATATCTATTATAGT GGGAGCGCCTACCACATTCCGTCCCTCAAGAGTC GAGTCACCATATCCGTAGACACGTCCAAGAACC AGTTCTCCCTGAAGTTGAACTCTGTGACCGCCGC AGACACGGCTGTGTATTACTGTGTGAGACATGAC CCAAACTGGGGAGTTGACTACTGGGGCCAGGGA ACCCTGGTCACCGTCTCCTCA |
| | | | SEQ ID NO: 26401 | SEQ ID NO: 30407 |
| | | AA | DIQMTQSPSSPSASVGDRVTITCRASQGIRNDLG WYQQKPGTAPKRLIYAASSLQSGVPSRFSGSGSG TEFTLTISNLQPEDFATYYCLQHSSYPLTFGGGT KVEIK | QLQLQESGPGLVKPSETLSLTCTVSGGSISRSSYYW GWIRQYPGKGLEWIGNIYYSGSAYHIPSLKSRVTIS VDTSKNQFSLKLNSVTAADTAVYYCVRHDPNWGV DYWGQGTLVTVSS |
| | | | SEQ ID NO: 26402 | SEQ ID NO: 30408 |

FIGURE 50

| IPS:435601 | 21-225_160G10 | NA | GCTATTGTGATGACCCAGACTCCACTCTCTCTG TCCGTCACCCCTGGACAGCCGGCCTCCATCTC CTGCAAGTCTAGTCAGAGCCTCCTGCACGGTG ATGGAAAGACCTATTTGTATTGGTACCTGCAG AAGCCAGGCCAGCCTCCACACCTCCTGATCTA TGAAGTTTCCAAACGGTTCTCTGGAGTGCCAG ATAGGTTCAGTGGCAGCGGGTCAGGGACAGA TTTCACACTGAAAATCAGCCGGGTGGAGGCTG AGGATGTTGGGCTTTATTACTGCATGCAAAGT ATACAGCTTCCGTGGACGTTTGTCCAAGGGAC CAAGGTGGAAATCACA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTTTGGCAT GCACTGGGTCCGCCAGGCTCCAGGCAAGGGCCT GGAGTGGGTGGCAGTCATATGGTATGATGGAAG TTATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGTTGGTAT AGAAGTGGCTGGTGACTACTACTTCGGTATGGAA GTCTGGGGCCAAGGGACCACGGTCACCGTCTCCT CA |
| | | | SEQ ID NO: 26403 | SEQ ID NO: 30409 |
| | | AA | AIVMTQTPLSLSVTPGQPASISCKSSQSLLHGDG KTYLYWYLQKPGQPPHLLIYEVSKRFSGVPDRFS GSGSGTDFTLKISRVEAEDVGLYYCMQSIQLPW TFVQGTKVEIT | QVQLVESGGGVVQPGRSLRLSCAASGFTFSSFGMH WVRQAPGKGLEWVAVIWYDGSYKYYADSVKGRF TISRDNSKNTLYLQMNSLRAEDTAVYYCARVGIEV AGDYYFGMEVWGQGTTVTVSS |
| | | | SEQ ID NO: 26404 | SEQ ID NO: 30410 |
| IPS:435605 | 21-225_161A4 | NA | GAAATAGTGATGACGCAGTCTCCAGCCACCCT GTCTGTGTCTCCAGGGGAAAGAGCCTCCCTCT CCTGCAGGTCCAGTCAGAGTGTTAACAGCAAC TTAGCCTGGTACCAGCAGAGGCCTGGCCAGGC TCTCAGGCTCCTCATCTATGGTGCATCCATCAG GGCCACTGATATCCCAGCCAGGTTCAATGGCA GTGGGTCTGGGACAGAGTTCACTCTCACCATC AGCAGCCTGCAGTCTGAAGATTTTGCAGTTTA TTTCTGTCAGCAGTATAATAACTGGTGGACGT TCGGCCAAGGGACCACGGTGGAAATCAAA | GAGGTGCAGCTGGTGGAGTCTGGAGGAGGCTTG ATCCAGCCTGGGGGGGTCCCTGAGACTCTCCTGTG CAGCCTCTGGGTTCACCGTCAGTAGCAACTACAT GAGCTGGGTCCGCCAGGCTCCAGGGAAGGGGCT GGAGTGGGTCTCAGTTATTTATACCGGTGGTAGC ACATACAACGCAGACTCCGTGAAGGGCCGATTC ACCATCTCCAGAGACAATTCCAAGAACACGCTGT ATCTTCAAATGAACAGCCTGAGAGCCGAGGACA CGGCCGTGTATTACTGTGCGAGAAATTGGGGAAT GGCTGGCCCCTTTGACTACTGGGGCCAGGGAACC CTGGTCACCGTCTCCTCA |
| | | | SEQ ID NO: 26405 | SEQ ID NO: 30411 |

FIGURE 50

| | | | EIVMTQSPATLSVSPGERASLSCRSSQSVNSNLA WYQQRPGQALRLLIYGASIRATDIPARFNGSGSG TEFTLTISSLQSEDFAVYFCQQYNNWWTFGQGT TVEIK | EVQLVESGGGLIQPGGSLRLSCAASGFTVSSNYMS WVRQAPGKGLEWVSVIYTGGSTYNADSVKGRFTIS RDNSKNTLYLQMNSLRAEDTAVYYCARNWGMAG PFDYWGQGTLVTVSS |
| :--- | :--- | :--- | :--- | :--- |
| | | AA | SEQ ID NO: 26406 | SEQ ID NO: 30412 |
| iPS:435607 | 21-225_161G4 | NA | GACATCCAGATGACCCAGTCTCCATCCTCCCT GTCTGCATCTGTAGGAGACAGAGTCACCATCA CTTGCCAGGCGAGTCAGGACATTTACAATTAT TTAAAATTGGTATCAGCAGAAACCAGGGAAAG CCCCTAAGCTCCTGATCTACGATGCATCCAAT TTGGAAACAGGGGTCCCATCAAGGTTCAGTGG AAGTGGATCTGGGACAGATTTTACTTTCGCCA TCAGCAGCCTGCAGCCTGAAGATATTGCAACA TATTACTGTCAACAGTATGATATTCTCCCGATC ACCTTCGGCCAAGGGACACGACTGGAGATTAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATCATATGGTGGAAGT AATAAATACCATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGTGAGACGGAGCAG CTCGTCGGGGGGGCTACGGTATGGACGTCTGGGGC CAAGGGACCACGGTCACCGTCTCCTCA |
| | | | SEQ ID NO: 26407 | SEQ ID NO: 30413 |
| | | AA | DIQMTQSPSSLSASVGDRVTITCQASQDIYNYLN WYQQKPGKAPKLLIYDASNLETGVPSRFSGSGS GTDFTFAISSLQPEDIATYYCQQYDILPITFGQGT RLEIK | QVQLVESGGGVVQPGRSLRLSCAASGFTFSSYGMH WVRQAPGKGLEWVAVISYGGSNKYHADSVKGRFT ISRDNSKNTLYLQMNSLRAEDTAVYYCVRRSSSSG GYGMDVWGQGTTVTVSS |
| | | | SEQ ID NO: 26408 | SEQ ID NO: 30414 |

FIGURE 50

| iPS:435609 | 21-225_161F7 | NA | GACATCCAGATGACCCAGTCTCCATCCTCCCT GTCTGCATCTGTAGGCGACAGAGTCACCATCA CTTGCCGGGCAAGTCAGGGCATTAGAAATGAT TTGGGCTGGTATCAGCAGAAACCAGGAGAAG CCCCTAAGCGCCTGATCTATGCTGCATCCACTT TGCAAAGTGGGGTCCCATCGCGGTTCAGCGGC AGTGGATCTGGGGCAGAATTCACTGTCACAAT CGGCAGCGTGCAGCGTGAAGATTTTGCAACTT ATTACGGTCTACTATATATTCGTTACCCATTCA CTTTTGGCCGTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTTTGGCTT GCACTGGGTCCGCCAGGCTCCAGGCCAGGGACT GGAGTGGGTGGCAGTTATATGGTTTGATGGAAGT AATAAATACTATGCAGACTCCGTGAAGGGCCGA TTCACCATTTTCAGAGACAATTCCAAGAACACGC TGTATCTGCAAATGAACAGCCTGAGAGCCGAGG ACACGGCTGTGTATTACTGTGCGAGAGAGATTGG CTGGCTCTCTGACTACTGGGGCCAGGGAACCCTG GTCACCGTCTCCTCA |
|---|---|---|---|---|
| | | | SEQ ID NO: 26409 | SEQ ID NO: 30415 |
| | | AA | DIQMTQSPSSLSASVGDRVTITCRASQGIRNDLG WYQQKPGEAPKRLIYAASTLQSGVPSRFSGSGS GAEFTVTIGSVQREDFATYYGLLYIRYPFTFGRG TKVDIK | QVQLVESGGGVVQPGRSLRLSCAASGFTFSDFGLH WVRQAPGQGLEWVAVIWFDGSNKYYADSVKGRF TIFRDNSKNTLYLQMNSLRAEDTAVYYCAREIGWL SDYWGQGTLVTVSS |
| | | | SEQ ID NO: 26410 | SEQ ID NO: 30416 |
| iPS:435611 | 21-225_161F10 | NA | GACATCCAGATGACCCAGTCTCCATCCTCCCT GTCTGCATCTGTAGGAGACAGAGTCACCATCA CTTGCCAGGCGAGTCAGGACATTTACAACCAT TTAAGTTGGTATCAGCAGAAACCAGGGAAAG CCCCTAAGCTCCTGATCTACGATGCATCCAAT TGGGAAACAGGGGTCCCATCCAGGTTCAGTGG AGGTGGATCTGGGACAGATTTTACTTTCACCA TCAGCAGCCTGCAGCCTGAAGATTTTGCAACA TATTACTGTCAACAGTATGAAAATCTCCCGCT CACCTTCGGCGGAGGGACCAAGGTGGAGATC AAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAATGGGTGGCAATTATATCATATTCTGGAAGA AATGATTTCTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGCGAGACGTATAGCA GCAGCTGGTCACTACGGTATGGACGTCTGGGGCC AAGGGACCACGGTCACCGTCTCCTCA |
| | | | SEQ ID NO: 26411 | SEQ ID NO: 30417 |

FIGURE 50

| | | | | |
|---|---|---|---|---|
| | | AA | DIQMTQSPSSLSASVGDRVTITCQASQDIYNHLS WYQQKPGKAPKLLIYDASNWETGVPSRFSGGGS GTDFTFTISSLQPEDFATYYCQQYENLPLTFGGG TKVEIK | QVQLVESGGGVVQPGRSLRLSCAASGFTFSSYGMH WVRQAPGKGLEWVAIISYSGRNDFYADSVKGRFTI SRDNSKNTLYLQMNSLRAEDTAVYYCARRIAAAG HYGMDVWGQGTTVTVSS |
| | | | SEQ ID NO: 26412 | SEQ ID NO: 30418 |
| IPS:435613 | 21-225_161D11 | NA | GACATCCAGATGACCCAGTCTCCATCCTCCCT GTCTGCATCTGTAGGCGACAGAGTCACCATCA CTTGCCGGGCAAGTCAGGGCATTAGAAATGAT TTGGGCTGGTATCAGCAGAAACCAGGAGAAG CCCCTAAGCGCCTGATCTATGCTGCATCCACTT TGCAAAGTGGGGTCCCATCGCGGTTCAGCGGC AGTGGATCTGGGGCAGAATTCACTCTCACAAT CGGCAGCGTGCAGCGTGAAGATTTTGCAACTT ATTACGGTCTACAATATAATCGTTACCCATTC ACTTTTGGCCGTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTTTGGCTT GCACTGGGTCCGCCAGGCTCCAGGCCAGGGACT GGAGTGGGTGGCAGTTATATGGTTTGATGGAAGT AATAAATACTATGCAGACTCCGTGAAGGGCCGA TTCACCATTTTCAGAGACAATTCCAAGAACACGC TGTATCTGCAAATGAACAGCCTGAGAGCCGAGG ACACGGCTGTGTATTACTGTGCGAGAGAGATTGG CTGGCTCTCTGACTACTGGGGGCCAGGGAACCCTG GTCACCGTCTCCTCA |
| | | | SEQ ID NO: 26413 | SEQ ID NO: 30419 |
| | | AA | DIQMTQSPSSLSASVGDRVTITCRASQGIRNDLG WYQQKPGEAPKRLIYAASTLQSGVPSRFSGSGS GAEFTLTIGSVQREDFATYYGLQYNRYPFTGRG TKVDIK | QVQLVESGGGVVQPGRSLRLSCAASGFTFSDFGLH WVRQAPGQGLEWVAVIWFDGSNKYYADSVKGRF TIFRDNSKNTLYLQMNSLRAEDTAVYYCAREIGWL SDYWGQGTLVTVSS |
| | | | SEQ ID NO: 26414 | SEQ ID NO: 30420 |

FIGURE 50

| iPS:435615 | 21-225_161G12 | NA | GACATCCAGATGACCCAGTCTCCATCCTCCCG GTCTGCATCTGTAGGAGACAGAGTCACCATCA CTTGCCGGGCAAGTCAGGACATTAGAAAGGAT TTAGGGTGGTATCAGCAGAAACCAGGGAAAG CCCCTAACCGCCTGATCTATGCTGCATCCAGTT TGCAAAGTGGGGTCCCATCAAGGTTCAGCGGC AGTGGATCTGGGACAGAATTCACTCTCACATT CAGCAGCCTGCAGCCTGAAGATTTTGCAACTT ATTACTGTCTACAGCATCATAGTTATCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTTAGTGACTATGTCAT GCAGTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACCGTA TAATAGTGGCTGGTACGACTACGGTATGGACGTC TGGGGCCAAGGGACCACGGTCACCGTCTCCTCA |
| | | | SEQ ID NO: 26415 | SEQ ID NO: 30421 |
| | | AA | DIQMTQSPSSRSASVGDRVTITCRASQDIRKDLG WYQQKPGKAPNRLIYAASSLQSGVPSRFSGSGS GTEFTLTFSSLQPEDFATYYCLQHHSYPRTFGQG TKVEIK | QVQLVESGGGVVQPGRSLRLSCAASGFTFSDYVM QWVRQAPGKGLEWVAVIWYDGSNKYYADSVKGR FTISRDNSKNTLYLQMNSLRAEDTAVYYCAREPYN SGWYDYGMDVWGQGTTVTVSS |
| | | | SEQ ID NO: 26416 | SEQ ID NO: 30422 |
| iPS:435617 | 21-225_162F2 | NA | GACATTCAGATGACCCAGTCTCCATCCTCCCT GTGTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCTTGATCAATGCTGCATCCAGT TTGCAAAGTGGGGTCCCATCAAGGTTCAGCGG CAGTGGATCTGGGACAGAATTCACTCTCACAA TCAGCAGCGTGCAGCCTGAAGATTTTGCAACT TATTACTGTATACAGGATAATAGTCACCCATT CACTTTCGGCCCTGGGACCAAAGTGGAAATCA AA | GAGGTGCAACTGGTGGAGTCTGGGGGAGGCCTG GTCAAGCCTGGGGGGTCCCTGAGATTCTCCTGTG CAGCCTCTGGATTCACCTTCAGTAGTTATAGCAT GAACTGGGTCCGCCAGGCTCCAGGGAAGGGGCT GGAGTGGGTCTCATCCATTAGTGGTAGTAGTACG TACATATACTACGCAGACTCAGTGAAGGGCCGAT TCACCATCTCCAGAGACAACGCCAAGAACTCACT GTATCTGCAAATGAACAGCCTGAGAGCCGAAGA CACGGCTGTGTATTACTGCGCGAGAGATCGGGGC AGCAGCTGGGGCCAGGGAACCCTGGTCACCGTC TCCTCA |
| | | | SEQ ID NO: 26417 | SEQ ID NO: 30423 |

2166

FIGURE 50

| | | AA | DIQMTQSPSSLCASVGDRVTITCRASQGIRNDLG WYQQKPGKAPKRLINAASSLQSGVPSRFSGSGS GTEFTLTISSVQPEDFATYYCIQDNSHPFTFGPGT KVEIK | EVQLVESGGGLVKPGGSLRFSCAASGFTFSSYSMN WVRQAPGKGLEWVSSISGSSTYIYYADSVKGRFTIS RDNAKNSLYLQMNSLRAEDTAVYYCARDRGSSW GQGTLVTVSS |
|---|---|---|---|---|
| | | | SEQ ID NO: 26418 | SEQ ID NO: 30424 |
| IPS:435621 | 21-225_162H3 | NA | GACATTCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCTTGATCAATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGGATAATAGTCACCCATT CACTTTCGGCCCTGGGACCAAAGTGGAAATCA AA | GAGGTGCAACTGGTGGAGTCTGGGGGAGGCCTG GTCAAGCCTGGGGGGTCCTTGAGATTCTCCTGTG CAGCCTCTGGATTCACCTTCAGTAGTTATAGCAT GAACCGGGTCCGCCAGGCTCCAGGGAAGGGGCT GGAGTGGGTCTCATCCATTAGTGGTAGTAGTACG TACATATACTACGCAGACTCAGTGAAGGGCCGAT TCACCATCTCCAGAGACAACGCCAAGAACTCACT GTATCTGCAAATGAACAGCCTGAGAGCCGAAGA CACGGCTGTGTATTACTGCGCGAGAGATCGGGGC AGCAGCTGGGGCCAGGGAACCCTGGTCACCGTC TCCTCA |
| | | | SEQ ID NO: 26419 | SEQ ID NO: 30425 |
| | | AA | DIQMTQSPSSLSASVGDRVTITCRASQGIRNDLG WYQQKPGKAPKRLINAASSLQSGVPSRFSGSGS GTEFTLTISSLQPEDFATYYCLQDNSHPFTFGPGT KVEIK | EVQLVESGGGLVKPGGSLRFSCAASGFTFSSYSMN RVRQAPGKGLEWVSSISGSSTYIYYADSVKGRFTIS RDNAKNSLYLQMNSLRAEDTAVYYCARDRGSSW GQGTLVTVSS |
| | | | SEQ ID NO: 26420 | SEQ ID NO: 30426 |

FIGURE 50

| iPS:435623 | 21-225_162D5 | NA | GACATTGTGATGACCCAGTCTCCAGACTTCCG TAATGTGTCTATGGGCGAGAGGGCCATCATCA ACTTCAAGTCCAACCATAGTGTTTTATACAGG TCCAACAATAATCAATACTTAGCTTGGTACCA GCGGAAACCAGGACAGCCTCCTAAGTTGCTCA TTTACCGGACATCTATCCGGAAATCCGGGGTC CCTGACCGATTCAGTGGCAGCGGGTGTGGGAC AGATTTCACTCTCACCATCGACAGCCTGCAGG CTGAAGATGTGGCAGTTTATTACTGTCAGCAA TATTATAGTACTCCTCCCACTTTCGGCGGAGG GACCAAGGTGGAGATCAAA | CAGGTGCAGCTGGTGCAGTCTGGGTCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGCACCCTAACAGTGGT AACACAGGCTATGCACAGAAGTTCCAGGGCAGA GTCACCATGACCAGGAACACCTCCATAGACACA GCCTACATGGAACTGAGCAGCCTGAGTTCTGAGG ACACGGCCGTGTATTTCTGTGCGTTTAGCAGTGG CTGGTACTTCTTTGACTACTGGGGCCAGGGAACC CTGGTCACCGTCTCCTCA |
| | | | SEQ ID NO: 26421 | SEQ ID NO: 30427 |
| | | AA | DIVMTQSPDFRNVSMGERAIINFKSNHSVLYRSN NNQYLAWYQRKPGQPPKLLIYRTSIRKSGVPDR FSGSGCGTDFTLTIDSLQAEDVAVYYCQQYYSTP PTFGGGTKVEIK | QVQLVQSGSEVKKPGASVKVSCKASGYTFTNYDIN WVRQATGQGLEWMGWMHPNSGNTGYAQKFQGR VTMTRNTSIDTAYMELSSLSSEDTAVYFCAFSSGW YFFDYWGQGTLVTVSS |
| | | | SEQ ID NO: 26422 | SEQ ID NO: 30428 |
| iPS:435627 | 21-225_162F6 | NA | GACATCGTGATGACCCAGTCTCCAGACTCCCT GGCTGTGTCTCTGGGCGAGAGGGCCACCATCA ACTGCAAGTCCAGCCAGAATGTTTTACACAGC TCCAACAATAACAACTACTTAACTTGGTACCA GCAGAGACCAGGACAGCCTCCTAAGTTGCTCA TTTACTGGGCATCTACCCGGGAATCCGGGGTC CCTGACCGATTCAGTGGCAGCGGGTCTGGGAC AGAATTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGGCTGAGGTG AGGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG ATTCACCATGACCAGGAACACCTCCATAAGCACA GCCTACATGGAGCTGAGCAGCCTGAGATCTGAG GACACGGCCGTGTATTACTGTGCCTATAGCAGTG GCTGGTACCGCTTTGACTACTGGGGCCAGGGAAC CCTGGTCACCGTCTCCTCA |
| | | | SEQ ID NO: 26423 | SEQ ID NO: 30429 |

FIGURE 50

| | | | | |
|---|---|---|---|---|
| | | AA | DIVMTQSPDSLAVSLGERATINCKSSQNVLHSSN NNNYLTWYQQRPGQPPKLLIYWASTRESGVPDR FSGSGSGTEFTLTISSLQAEDVAVYYCQQYYSTP PTFGQGTKVEIK | QVQLVQSGAEVRKPGASVKVSCKASGYTFTNYDIN WVRQATGQGLEWMGWMHPNSGNTGYAQKFQGR FTMTRNTSISTAYMELSSLRSEDTAVYYCAYSSGW YRFDYWGQGTLVTVSS |
| | | | SEQ ID NO: 26424 | SEQ ID NO: 30430 |
| iPS:435629 | 21-225_162H6 | NA | GATATTGTGATGACCCAGACTCCACTCTCTCT GTCCGTCACCCCTGGACAGCCGGCCTCCATCT CCTGCAAGTCTACTCAGAGCCTCCTGCATGGT GATGGAAAGACCTATTTGTATTGGTACCTGCA GAAGCCAGGCCAGCCTCCACAACTCCTGATCT ATGAAGTTTCCAAGCGGTTCTCTGGAGTGCCA GAAAGGTTCAGTGGCAGCGGGTCAGGGACAG ATTTCACACTGAAAATCAGCCGGGTGGAGGCT GAGGATGTTGGGGTTTATTACTGCAAGCAAAG TATACAGCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAATAACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAAAGGTAT AGCAGCAGTTGGAGACTACTACTACGGTATGGA CGTCTGGGGCCAAGGGACCACGGTCACCGTCTCC TCA |
| | | | SEQ ID NO: 26425 | SEQ ID NO: 30431 |
| | | AA | DIVMTQTPLSLSVTPGQPASISCKSTQSLLHGDG KTYLYWYLQKPGQPPQLLIYEVSKRFSGVPERFS GSGSGTDFTLKISRVEAEDVGVYYCKQSIQLPWT FGQGTKVEIK | QVQLVESGGGVVQPGRSLRLSCAASGFTFNNYGM HWVRQAPGKGLEWVAVIWYDGSNKYYADSVKGR FTISRDNSKNTLYLQMNSLRAEDTAVYYCARKGIA AVGDYYYGMDVWGQGTTVTVSS |
| | | | SEQ ID NO: 26426 | SEQ ID NO: 30432 |

FIGURE 50

| iPS:435635 | 21-225_163F1 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAGCTTA TTACTGCCAACAGTATAATAGTTACCCATTCA CTTTCGGCCCTGGGACCCAAGTGGATGCCCAA | GAGGTGCAGCTGGTGGACTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTGGTAGT TACATATACTACGCAGACTCAGTGAAGGGCCGAT TCACCACCTCCAGAGACAACGCCAAGAACTCACT GTATCTGCAAATGAACAGCCTGAGCGCCGACGA CACGGCTGTTTATTACTGTACGCTCTATAGCAGC TCGCACTATTGGGGCCAGGGAACCCTGGTCACCG TCTCCTCA |
| | | | SEQ ID NO: 26427 | SEQ ID NO: 30433 |
| | | AA | DIQMTQSPSSLSASVGDRVTITCRASQDISNYLA WFQQKPGKAPKSLIYAASSLQSGVPSKFSGSGSG TDFTLTISSLQPEDFAAYYCQQYNSYPFTFGPGT QVDAQ | EVQLVDSGGGLVKPGGSLRLSCAASGFTFSSYSMN WVRQAPGKGLEWVSSISGSGSYIYYADSVKGRFTT SRDNAKNSLYLQMNSLSADDTAVYYCTLYSSSHY WGQGTLVTVSS |
| | | | SEQ ID NO: 26428 | SEQ ID NO: 30434 |
| iPS:435637 | 21-225_163E2 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGGGAAAG CCCCTACGCGCCTGATCTATCCTGCATCCAGTT TGCAAAGTGGGGTCCCATCAAGGTTCAGCGGC AGTGGATCTGGGACAGAATTCACTCTCTCAAT CAGTAGCCTGCAGCCTGAAGATTTTGCAACTT ATTACTGTCTACAGCATAATAGTCACCCATTC ACTTTCGGCCCTGGGACCAAAGTGGATATCAA A | GAGGTGCAACTGGTGGAGTCTGGGGGAGGCCTG GTCCAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCCTCCACTAGTGGGAGTTCTACT TACATATACTACGCAGACTCAGTGAAGGGCCGAT TCACCATCTCCAGAGACAACGCCAAGAACTTAGT GTATCTGCAAATGAACAGCCTGAGACCCGAGGA CACGGCTGTGTATTACTGTGCGAGAGATCGAGGC AGCCTCTGGGGCCAGGGAACCCTGGTCACCGTCT CCTCA |
| | | | SEQ ID NO: 26429 | SEQ ID NO: 30435 |

FIGURE 50

| | | | | |
|---|---|---|---|---|
| | | AA | DIQMTQSPSSLSASVGDRVTITCRASQDIRNDLG WYQQKPGKAPTRLIYPASSLQSGVPSRFSGSGSG TEFTLSISSLQPEDFATYYCLQHNSHPFTFGPGTK VDIK | EVQLVESGGGLVQPGGSLRLSCAASGFTFSSYSMN WVRQAPGKGLEWVSSTSGSSTYIYYADSVKGRFTI SRDNAKNLVYLQMNSLRPEDTAVYYCARDRGSLW GQGTLVTVSS |
| | | | SEQ ID NO: 26430 | SEQ ID NO: 30436 |
| IPS:435639 | 21-225_163G6 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTAGTCTGGTTTCAGCAGAAACCAGGGAAAGC CCCTAAGTCCCTGATCTATGCTGCATCCAGTTT GCTAAGTGGGGTCCCTTCAAAGTTCAGCGGCA GTGGATCTGGGACAGATTTCACTCTCACCATC AGCAGCCTGCAGCCTGAAGATTTTGCAACTTA TTACTGCCAACAGTATCATAGTTACCCGCTCA CTTTCGGCGGAGGGACCAAGGTGGCGATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGTTATAGCAT GAGCTGGGTCCGCCAGGCTCCAGGGAAGGGGCT GGAGTGGGTCTCATCCATTAGTGGTAGTAGTGCT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGATTGAGCGGT ATGGACGTCTGGGGCCAAGGGACCACGGTCACC GTCTCCTCA |
| | | | SEQ ID NO: 26431 | SEQ ID NO: 30437 |
| | | AA | DIQMTQSPSSLSASVGDRVTITCRASQDISNYLV WFQQKPGKAPKSLIYAASSLLSGVPSKFSGSGSG TDFTLTISSLQPEDFATYYCQQYHSYPLTFGGGT KVAIK | EVQLVESGGGLVKPGGSLRLSCAASGFTFSSYSMS WVRQAPGKGLEWVSSISGSSAYIYYADSVKGRFTIS RDNAKNSLYLQMNSLRAEDTAVYYCARLSGMDV WGQGTTVTVSS |
| | | | SEQ ID NO: 26432 | SEQ ID NO: 30438 |

FIGURE 50

| iPS:435641 | 21-225_163F9 | NA | GACATCCAGATGACCCAGTCTCCATCCTCCCT GTCTGCATCTGTAGGAGACAGAGTCACCATCA CTTGCCGGGCAAGTCAGGACATTAGAGATGAT TTAGGCTGGTATCAGCAGAAACCAGGGAAAG CCCCTAAGCGCCTGATCTATCCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAGCTT ATTACTGTCTACAGGATAATAGTTACCCATTC ACTTTCGGCCCTGGGACCAAAGTGGATATCAA A | GAGGTGCAGCTGGTGGAGTCTGGGGGAGGCCTG GTCCAGCCGGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTACT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATCGAGGC AGCCTCTGGGGCCAGGGAACCCTGGTCACCGTCT CCTCA |
|---|---|---|---|---|
| | | | SEQ ID NO: 26433 | SEQ ID NO: 30439 |
| | | AA | DIQMTQSPSSLSASVGDRVTITCRASQDIRDDLG WYQQKPGKAPKRLIYPASSLQSGVPSRFSGSGSG TEFTLTISSLQPEDFAAYYCLQDNSYPFTFGPGTK VDIK | EVQLVESGGGLVQPGGSLRLSCAASGFTFSSYSMN WVRQAPGKGLEWVSSISGSSTYIYYADSVKGRFTIS RDNAKNSLYLQMNSLRAEDTAVYYCARDRGSLW GQGTLVTVSS |
| | | | SEQ ID NO: 26434 | SEQ ID NO: 30440 |
| iPS:435643 | 21-225_163G10 | NA | GACATCCAGATGACCCAGTCTCCATCCTCCCT GTCTGCATCTGTAGGAGACAGAGTCACCATCA CTTGCCGGGCAAGTCAGGACATTAGAAATAAT TTAGGCTGGTATCAGCAGAAACCAGGGAAAG CCCCTAAGCGCCTGATCTATCCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGGATTATAGTTACCCATTCA CTTTCGGCCCTGGGACCAAAGTGGATATCAAA | GAGGTGCAGTTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTACT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGCCCGTATG GACGTCTGGGGCCAAGGGACCACGGTCACCGTC TCCTCA |
| | | | SEQ ID NO: 26435 | SEQ ID NO: 30441 |

FIGURE 50

| | | | | |
|---|---|---|---|---|
| | | AA | DIQMTQSPSSLSASVGDRVTITCRASQDIRNNLG WYQQKPGKAPKRLIYPASSLQSGVPSRFSGSGSG TEFTLTISSLQPEDFATYYCLQDYSYPFTFGPGTK VDIK | EVQLVESGGGLVKPGGSLRLSCAASGFTFSSYSMN WVRQAPGKGLEWVSSISGSSTYIYYADSVKGRFTIS RDNAKNSLYLQMNSLRAEDTAVYYCARARMDVW GQGTTVTVSS |
| | | | SEQ ID NO: 26436 | SEQ ID NO: 30442 |
| iPS:435649 | 21-225_165H2 | NA | GACATCGTGATGACCCAGTCTCCAGACTCCCT GACTGTGTCTCCGGGCGAGAGGGCCACCATCA ACTGCAAGTCCAGCCAGAGTGTTTTACACAGC TCCAACAATAAGAACTACTTAACTTGGTACCA GCAGAAACCAGGACAGCCTCCTAAACTGCTCA TTTACTGGGCATCTACCCGAGAATCCGGGGTC CCTGTCCGATTCAGTGGCAGCGGGTCGGGGAC AGATTTCACTGTCCCCATCAGCAGCATGCAGG ATGATGATGTGGCAGTTTATTACCGTCAGCAA TCTTATAGTATTCCTCCCACTTTCGGCCCCGGG ACCAACGTGGATATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCCATTATGATA TCAACTGGGTGCGACAGGCCACTGGACAAGGGC TTGAGTGGGTGGGGTGGATGCACCCTAACAGTCA TAAGACAGGCTATGCACAGAAGTTCCAGGGCAG AGTCACCATGACCAGGAACACCTCCAACAGCAC AGCCTACATGGACCTCAGCAGCCTGAGATCTGAG GACACGGCCGTGTATTACTGTGCTTATAGCAGTG GCTGGTACATGTTTGACTACTGGGGCCAGGGAAC CCTGGTCACCGTCTCCTCA |
| | | | SEQ ID NO: 26437 | SEQ ID NO: 30443 |
| | | AA | DIVMTQSPDSLTVSPGERATINCKSSQSVLHSSN NKNYLTWYQQKPGQPPKLLIYWASTRESGVPVR FSGSGSGTDFTVPISSMQDDDVAVYYRQQSYSIP PTFGPGTNVDIK | QVQLVQSGAEVKKPGASVKVSCKASGYTFTHYDIN WVRQATGQGLEWVGWMHPNSHKTGYAQKFQGR VTMTRNTSNSTAYMDLSSLRSEDTAVYYCAYSSG WYMFDYWGQGTLVTVSS |
| | | | SEQ ID NO: 26438 | SEQ ID NO: 30444 |

2173

FIGURE 50

| iPS:435653 | 21-225_166H12 | NA | GACATCCAGATGACCCAGTCTCCATCCTCACT GTCTACATCTGTAGGAGACAGAGTCACCATCA CTTGTCGGGCGAGTCAGGACATTAGCCATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTTCCCGCTCA CTTTCGGCGGAGGGACCAAGGTGGAGATCAC A | GAGGTGCAGCTGGTGGAGTCTGGGGGAGCCCTG GTCAAGCCTGGGGGGTCCCTGAGACTCTCCTGTG CAGCCTCTGGATTCACCTTCAGTAGCTATAGCAT GAGCTGGGTCCGCCAGGCTCCAGGGAAGGGGCT GGGGTGGGTCTCATCCATTAGTGGGAGTAGTAGT TACAGTTACTACGCAGACTCAGTGAAGGGCCGAT TCACCATCTCCAGAGACAATGCCAAGAACTCACT GTATCTGCAAATGAACAGCCTGAGAGCCGAGGA CACGGCTGTGTATTACTGTGCGAGACTAACTGGC TTTGACTACTGGGGCCAGGGAACCCTGGTCACCG TCTCCTCA |
| | | | SEQ ID NO: 26439 | SEQ ID NO: 30445 |
| | | AA | DIQMTQSPSSLSTSVGDRVTITCRASQDISHYLA WFQQKPGKAPKSLIYAASSLQSGVPSKFSGSGSG TDFTLTISSLQPEDFATYYCQQYNSFPLTFGGGT KVEIT | EVQLVESGGALVKPGGSLRLSCAASGFTFSSYSMS WVRQAPGKGLGWVSSISGSSSYSYYADSVKGRFTI SRDNAKNSLYLQMNSLRAEDTAVYYCARLTGFDY WGQGTLVTVSS |
| | | | SEQ ID NO: 26440 | SEQ ID NO: 30446 |
| iPS:435655 | 21-225_167E2 | NA | GATATTGTGATGACCCAGACTCCACTCTCTCT GTCCGTCACCCCTGGACAGCCGGCCTCCATCT CCTGTAAGTCTAGTCAGAGCCTCCTGCACGGT GATGGGAAGACCTATTTGTATTGGTACCTGCA GAAGCCAGGCCAGCCTCCACACCTCCTGATCT ATGAAGTTTCCAAACGGTTCTCTGGAGTGCCA GATAGGTTCAGTGGCAGCGGGTCAGGGACAG ATTTCACACTGAAGATCAGCCGGGTGGAGGCT GAGGATGTTGGGCTTTATCACTGCATGCAAAG CATACAGCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTTAGTAGCTTTGGCAT GCACTGGGTCCGCCAGGCTCCAGGCAAGGGCCT GGAGTGGGTGGCAGTCATATGGTATGATGGAACT TATAAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGTTGGTATT GAAGTGGCTGGTGACTACTACTACGGTATGGAAG TCTGGGGCCAAGGGACCACGGTCACCGTCTCCTC A |
| | | | SEQ ID NO: 26441 | SEQ ID NO: 30447 |

FIGURE 50

| | | | | |
|---|---|---|---|---|
| | | AA | DIVMTQTPLSLSVTPGQPASISCKSSQSLLHGDG KTYLYWYLQKPGQPPHLLIYEVSKRFSGVPDRFS GSGSGTDFTLKISRVEAEDVGLYHCMQSIQLPW TFGQGTKVEIK | QVQLVESGGGVVQPGRSLRLSCAASGFTFSSFGMH WVRQAPGKGLEWVAVIWYDGTYKYYADSVKGRF TISRDNSKNTLYLQMNSLRAEDTAVYYCARVGIEV AGDYYYGMEVWGQGTTVTVSS |
| | | | SEQ ID NO: 26442 | SEQ ID NO: 30448 |
| iPS:435657 | 21-225_167H10 | NA | GATATTGTGATGACCCAGACTCCACTCTCTCT GTCCGTCACCCCTGGACAGCCGGCCTCCATCT CCTGCAAGTCTAGTCAGAGCCTCCTGCACGGT GATGGAAAGACCTATTTGTATTGGTACCTGCA GAAGCCAGGCCAGCCTCCACACCTCCTGATCT ATGAAGTTTCCAAACGGTTCTCTGGAGTGCCA GATAGGTTCAGTGGCAGCGGGTCAGGGACAG ATTTCACACTGAAAATCAGCCGGGTGGAGGCT GAGGATGTTGGGCTTTATCACTGCATGCAAAG CATACAGCTTCCGTGGACGTTCGGCCAAGGGA CCAAGGTGGAAATCAAA | CAGGTGCAGTTGGTGGAGTCTGGGGGAGGCGTG GTCCAGCCTGGGAGGTCCCAGAGACTCTCCTGTG CAGCGTCTGGATTCACCTTTAGTAGCTTTGGCAT GCACTGGGTCCGCCAGGCTCCAGGCAAGGGCCT GGAGTGGGTGGCAGTCATATGGTATGATGGAAG TTATAAGTACCATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGTTGGTAT AGAAGTGGCTGGTGACTACTACTACGGTATGGAA GTCTGGGGCCAAGGGACCACGGTCACCGTCTCCT CA |
| | | | SEQ ID NO: 26443 | SEQ ID NO: 30449 |
| | | AA | DIVMTQTPLSLSVTPGQPASISCKSSQSLLHGDG KTYLYWYLQKPGQPPHLLIYEVSKRFSGVPDRFS GSGSGTDFTLKISRVEAEDVGLYHCMQSIQLPW TFGQGTKVEIK | QVQLVESGGGVVQPGRSQRLSCAASGFTFSSFGMH WVRQAPGKGLEWVAVIWYDGSYKYHADSVKGRF TISRDNSKNTLYLQMNSLRAEDTAVYYCARVGIEV AGDYYYGMEVWGQGTTVTVSS |
| | | | SEQ ID NO: 26444 | SEQ ID NO: 30450 |

FIGURE 50

| | | | | |
|---|---|---|---|---|
| iPS:435659 | 21-225_167D12 | NA | GACATCCAGATGACCCAGTCTCCATCCTCACT GTCTGCATCTGTAGGGGACAGAGTCACCATCA CTTGTCGGGCGAGTCAGGGCATTAACAATTAT TTAGCCTGGTTTCAGCAGAAACCAGGGAAAGC CCCTGAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTTCTGCCAACAGTATAATAGTTATCCATTCAC TTTCGGCCCTGGGACCAAAGTGGATATCAAA | GAGGTGCAGCTGTTGGAGTCTGGGGGAGGCTTG GTACAGCCGGGGGGGTCCCTGAGACTCTCCTGTG CAGCCTCTGGATTCACCTTTAGTAGCTATGTCAT GAGCTGGGTCCGCCAGGCTCCAGGGAAGGGGCT GGAGTGGGTCTCAGCTATGAGTGGTAGTGGTGGT AGAACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTGTATTACTGTGCGAAGTATACCTG GAACGGCTACTGGGGCCAGGGAACCCTGGTCAC CGTCTCCTCA |
| | | | SEQ ID NO: 26445 | SEQ ID NO: 30451 |
| | | AA | DIQMTQSPSSLSASVGDRVTITCRASQGINNYLA WFQQKPGKAPESLIYAASSLQSGVPSKFSGSGSG TDFTLTISSLQPEDFATYFCQQYNSYPFTFGPGTK VDIK | EVQLLESGGGLVQPGGSLRLSCAASGFTFSSYVMS WVRQAPGKGLEWVSAMSGSGGRTYYADSVKGRF TISRDNSKNTLYLQMNSLRAEDTAVYYCAKYTWN GYWGQGTLVTVSS |
| | | | SEQ ID NO: 26446 | SEQ ID NO: 30452 |
| iPS:435663 | 21-225_169B1 | NA | GACATCCAGATGACCCAATCTCCATCCTCCCT GTCTGCATCTGTAGGAGACAGAGTCACCATCA CTTGCCGGGCTAGTCAGGGCATTAGAAATGAT TTAGGCTGGTATCAGCAGAAACCAGGGAAAG CCCCTAAGCGCCTGATCGGTGCTGAATCCAGT TTGCAAAGTGGGGTCCCATCAAGGTTCAGCGG CAGTGGATCTGGGACAGAATTCACTCTCACAA TCAGCGGCCTGCAGCCTGAAGATTTTGCAACT TATTACTGTCTACAGCATTATAGTTACCCGCTC ACTTTCGGCGGAGGGACCAAAGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAGGGGCCGA TTCACCATCTCCAGAGACAATTCCAAGAACACGC TGTATCTGCAAATGAACAGCCTGAGAGCCGAAG ACACGGCTGTGTATTACTGTGCGAGAGATCCCTT ACGTGGATACAATGACCCGGTTATGGACTACTGG GGCCAGGGAACCCTGGTCACCGTCTCCTCA |
| | | | SEQ ID NO: 26447 | SEQ ID NO: 30453 |

FIGURE 50

| | | | | |
|---|---|---|---|---|
| | | AA | DIQMTQSPSSLSASVGDRVTITCRASQGIRNDLG WYQQKPGKAPKRLIGAESSLQSGVPSRFSGSGSG TEFTLTISGLQPEDFATYYCLQHYSYPLTFGGGT KVEIK | QVQLVESGGGVVQPGRSLRLSCAASGFTFSSYGMH WVRQAPGKGLEWVAVIWYDGSNKYYADSVRGRF TISRDNSKNTLYLQMNSLRAEDTAVYYCARDPLRG YNDPVMDYWGQGTLVTVSS |
| | | | SEQ ID NO: 26448 | SEQ ID NO: 30454 |
| iPS:435665 | 21-225_169F2 | NA | GACATCGTGATGACCCAGTCTCCAGACTCCCT GGCTGTGTCTCTGGGCGAGAGGGCCACCATCA ACTGCAAGTCCAGCCAGAGTGTTTTATACATC TCCAACAATAAAAACTACTTAGCTTGGTACCA GCAGAAACCAGGACAGCCTCCTAAACTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATCGTGCTCCCCACCTTCGGCCAAGGGAC ACGACTGGAGATTAAA | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTACTACTG GAGTTGGATCCGGCAGCCCGCCGGGAAGGGACT GGAGTGGATTGGGCGTATCGATACCAGTGGGATC ACCAACTACAACCCCTCCCTCAAGAGTCGAGTCA CCATGTCAATAGACACGTCCAAGAGCCAGATCTC CCTGAAGCTGAGCTCTGTGACCGCCGCGGACACG GCCGTGTATTACTGTGCGAGAGAGGGAGGAGTG GGAGCTACCTACTTTGACTACTGGGGCCAGGGAA CCCTGGTCACCGTCTCCTCA |
| | | | SEQ ID NO: 26449 | SEQ ID NO: 30455 |
| | | AA | DIVMTQSPDSLAVSLGERATINCKSSQSVLYISN NKNYLAWYQQKPGQPPKLLIYWASTRESGVPD RFSGSGSGTDFTLTISSLQAEDVAVYYCQQYYR APTFGQGTRLEIK | QVQLQESGPGLVKPSETLSLTCTVSGGSISSYYWSW IRQPAGKGLEWIGRIDTSGITNYNPSLKSRVTMSIDT SKSQISLKLSSVTAADTAVYYCAREGGVGATYFDY WGQGTLVTVSS |
| | | | SEQ ID NO: 26450 | SEQ ID NO: 30456 |

FIGURE 50

| iPS:435667 | 21-225_169E3 | NA | GACATTGTGATGACTCAGTCTCCACTCTCCCTG CCCGTCACCCCTGGAGAGCCGGCCTCCATCTC CTGCAGGTCTAGTCAGAGCCTCCTGCATAATA ATGGATACAAGTATTTGGATTGGTACCTGCAG AAGCCAGGGCAGTCTCCACAGCTCCTGATCTA TTTGGGTTCTAATCGGGCCTCCGGGGTCCCTG ACAGGTTCAGTGCCAGTGGATCAGGCACAGAT TTTACACTGAAAATCAGCAGAGTGGAGGCTGA GGATGTTGGGGTTTATTACTGCATGCAAGTTC TACAAACTCCGTGGACGTTCGGCCAAGGGACC AAGGTGGAAATCAAA | GAGGTGCAACTGGTGGAGTCTGGGGGAGGCTTG GTACAGCCGGGGGGGTCCCTGAGACTCTCCTGTG CAGCCTCAGGATTCACCTTCAGTGGCCATAGCAT GAACTGGGTCCGCCAGGCTCCAGGGAAGGGGCT GGAGTGGGTTGCATACATTAGCCTTAGTGGTAGT ACCATAAAGTACGCAGACTCTGTGAAGGGCCGA TTCACCATCTCCAGAGACAATGCCAGGGACTCAC TGTATCTGCAAATGAACAGCCTGAGAGACGAGG ACACGGCTGTGTATTACTGTGCGAGAAGGGGGAT TACTGTGGTTCGGAATGAGGACGGTTTGGACGTC TGGGGCCAAGGGACCACGGTCACCGTCTCCTCA |
| | | | SEQ ID NO: 26451 | SEQ ID NO: 30457 |
| | | AA | DIVMTQSPLSLPVTPGEPASISCRSSQSLLHNNGY KYLDWYLQKPGQSPQLLIYLGSNRASGVPDRFS ASGSGTDFTLKISRVEAEDVGVYYCMQVLQTPW TFGQGTKVEIK | EVQLVESGGGLVQPGGSLRLSCAASGFTFSGHSMN WVRQAPGKGLEWVAYISLSGSTIKYADSVKGRFTI SRDNARDSLYLQMNSLRDEDTAVYYCARRGITVV RNEDGLDVWGQGTTVTVSS |
| | | | SEQ ID NO: 26452 | SEQ ID NO: 30458 |
| iPS:435669 | 21-225_169F9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTTTGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATTATAGTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGTCAATTATATGGTATGATGGAACT AATAAATACTATGCAGACTCCGTGAAGGGCCGA TTCACCATCTCCAGAGACAATTCCAAGAACACGC TGTATCTGCAACTGAACAGCCTGAGAGCCGAGG ACACGGCTGTGTATTACTGTGCGCGAGATCCCTT ACGTGGATACAATGACCCGGTTATGGACTACTGG GGCCAGGGAACCCTGGTCACCGTCTCCTCA |
| | | | SEQ ID NO: 26453 | SEQ ID NO: 30459 |

FIGURE 50

| | | | | |
|---|---|---|---|---|
| | | AA | DIQMTQSPSSLSASVGDRVTITCRASQGIRNDLG WYQQKPGKAPKRLIFAASSLQSGVPSRFSGSGSG TEFTLTISSLQPEDFATYYCLQHYSYPLTFGGGT KVEIK | QVQLVESGGGVVQPGRSLRLSCAASGFTFSTYGMH WVRQAPGKGLEWVSIIWYDGTNKYYADSVKGRFT ISRDNSKNTLYLQLNSLRAEDTAVYYCARDPLRGY NDPVMDYWGQGTLVTVSS |
| | | | SEQ ID NO: 26454 | SEQ ID NO: 30460 |
| iPS:435671 | 21-225_169H5 | NA | GACATCGTGATGACCCAGTCTCCAGACTCCCT GGCTGTGTCTCTGGGCGAGAGGGCCACCATCA ACTGCAAGTCCAGCCAGAGTGTTTTATACATC TCCAACAATAAAAACTACTTAGCTTGGTACCA GCAGAAACCAGGACAGCCTCCTAAACTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATCGTGCTCCCACCTTCGGCCAAGGGAC ACGACTGGAGATTAAA | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTACTACTG GAGTTGGATCCGGCAGCCCGCCGGGAAGGGACT GGAGTGGATTGGGCGTATCGATACCAGTGGGATC ACCAACTACAACCCCTCCCTCAAGAGTCGAGTCA CCATGTCAGTAGACACGTCCAAGAGCCAGATCTC CCTGAAGCTGAGCTCTGTGACCGCCGCGGACACG GCCGTGTATTACTGTGCGAGAGAGGGAGGAGTG GGAGCTACCTACTTTGACTACTGGGGCCAGGGAA CCCTGGTCACCGTCTCCTCA |
| | | | SEQ ID NO: 26455 | SEQ ID NO: 30461 |
| | | AA | DIVMTQSPDSLAVSLGERATINCKSSQSVLYISN NKNYLAWYQQKPGQPPKLLIYWASTRESGVPD RFSGSGSGTDFTLTISSLQAEDVAVYYCQQYYR APTFGQGTRLEIK | QVQLQESGPGLVKPSETLSLTCTVSGGSISSYYWSW IRQPAGKGLEWIGRIDTSGITNYNPSLKSRVTMSVD TSKSQISLKLSSVTAADTAVYYCAREGGVGATYFD YWGQGTLVTVSS |
| | | | SEQ ID NO: 26456 | SEQ ID NO: 30462 |

FIGURE 50

| iPS:435673 | 21-225_169E6 | NA | GACATTGTGATGACTCAGTCTCCACTCTCCCTG CCCGTCACCCCTGGAGAGCCGGCCTCCATCTC CTGCAGGTCTAGTCAGAGCCTCCTGCATAATA ATGGATACAAGTATTTGGATTGGTACCTGCAG AAGCCAGGGCAGTCTCCACAGCTCCTGATCTA TTTGGGTTCTAATCGGGCCTCCGGGGTCCCTG ACAGGTTCAGTGCCAGTGGATCAGGCACAGAT TTTACACTGAAAATCAGCAGAGTGGAGGCTGA GGATGTTGGGGTTTATTACTGCATGCAAGTTC TACAAACTCCGTGGACGTTCGGCCAAGGGACC AAGGTGGAAATCAAA | GAGGTGCAACTGGTGGAGTCTGGGGGAGGCTTG GTACAGCCTGGGGGGTCCCTGAGACTCTCCTGTG CAGCCTCAGGATTCACCTTCAGTGGCCATAGCAT GAACTGGGTCCGCCAGGCTCCAGGGAAGGGGCT GGAGTGGGTTGCATACATTAGCATTAGTAGTAGT ACCATAAAGTACGCAGACTCTGTGAAGGGCCGA TTCACCATCTCCAGAGACAATGCCAGGGACTCAC TGTATCTGCAAATGAACAGCCTGAGAGACGAGG ACACGGCTGTGTATTACTGTGCGAGAAGGGGGAT TACTGTGGTTCGGAATGAGGACGGTTTGGACGTC TGGGGCCAAGGGACCACGGTCACCGTCTCCTCA |
| | | | SEQ ID NO: 26457 | SEQ ID NO: 30463 |
| | | AA | DIVMTQSPLSLPVTPGEPASISCRSSQSLLHNNGY KYLDWYLQKPGQSPQLLIYLGSNRASGVPDRFS ASGSGTDFTLKISRVEAEDVGVYYCMQVLQTPW TFGQGTKVEIK | EVQLVESGGGLVQPGGSLRLSCAASGFTFSGHSMN WVRQAPGKGLEWVAYISISSSTIKYADSVKGRFTIS RDNARDSLYLQMNSLRDEDTAVYYCARRGITVVR NEDGLDVWGQGTTVTVSS |
| | | | SEQ ID NO: 26458 | SEQ ID NO: 30464 |
| iPS:435675 | 21-225_169D7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATCATAGTTGCCCGTG GACGTTCGGCCAAGGGACCAAGGTGGAAATC AAA | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTACTACTG GACCTGGATCCGGCAGCCCGCCGGGAAGGGACT GGAGTGGATTGGGCGTATCTATACCAGTGGGAGC ACCAACTACAACCCCTCCCTCAAGAGTCGAGTCA CCATGTCAGTAGACACGTCCAAGAACCAGTTCTC CCTGAAGCTGAGCTCTGTGACCGCCGCGGACACG GCCGTGTATTTCTGTGCGAAAGTCGGGAGGTACT ACTACGGTATGGACGTCTGGGGCCAAGGGACCA CGGTCACCGTCTCCTCA |
| | | | SEQ ID NO: 26459 | SEQ ID NO: 30465 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHHSCPWTFGQG TKVEIK | QVQLQESGPGLVKPSETLSLTCTVSGGSISSYYWTW IRQPAGKGLEWIGRIYTSGSTNYNPSLKSRVTMSVD TSKNQFSLKLSSVTAADTAVYFCAKVGRYYYGMD VWGQGTTVTVSS |
| | | | SEQ ID NO: 26460 | SEQ ID NO: 30466 |
| iPS:435677 | 21-225_169C10 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTAATCTTTTCTGCATCCAGTTT GCAAAGTGGGGTCCCATCAAAATTCAGCGGCA GTGGATCTGGGACAGATTTCAATCTCACCATC AGCAGCCTGCAGCCTGAAGATTTTGCAACTTA TTACTGCCAACAATCTGATAGTTACCCTCTCAC TTTCGGCGGAGGGACCAAGGTGGAGATCAAA | CAGGTGCAGCTGGTGCAGTCTGGGGCTGAGGTG AAGAAGCCTGGGGCCTCAGTGAGGGTCTCCTGC AAGGCTTCTGGATACACCTTCACCGGCTACTTTA TGCACTGGGTGCGACAGGCCCCTGGACAAGGGC TTGAGTGGATGGGATGGATCAAGCCTAAGAGTG GTGGCACAAACTCTGCACAGAGGTTTCAGGGCA GGGTCACCATGACCAGGGACACGTCCATCAACA CAGCCTACATGGAGCTGAACAGGCTGAGATCTG ACGACACGGCCGTGTATTACTGTGCGAGAGGGG GGACTACGGTGGCTACGTGGGGGGTCTTTGACTA CTGGGGCCAGGGAACCCTGGTCACCGTCTCCTCA |
| | | | SEQ ID NO: 26461 | SEQ ID NO: 30467 |
| | | AA | DIQMTQSPSSLSASVGDRVTITCRASQDISNYLA WFQQKPGKAPKSLIFSASSLQSGVPSKFSGSGSG TDFNLTISSLQPEDFATYYCQQSDSYPLTFGGGT KVEIK | QVQLVQSGAEVKKPGASVRVSCKASGYTFTGYFM HWVRQAPGQGLEWMGWIKPKSGGTNSAQRFQGR VTMTRDTSINTAYMELNRLRSDDTAVYYCARGGT TVATWGVFDYWGQGTLVTVSS |
| | | | SEQ ID NO: 26462 | SEQ ID NO: 30468 |

FIGURE 50

| iPS:435679 | 21-225_169D10 | NA | GACATCCAGATGACCCAGTCTCCATCTTCACT GTCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATCATAGTTACCCATTCA CTTTCGGCCCTGGGACCAAAGTGGATATCAAA | GAGGTGCAGTTGTTGGAGTCTGGGGGAGGCTTGG TGCAGTCTGGGGGGGTCCCTGAGACTCTCCTGTGC AGCCTCTGGATTCACCTTTAGCAGCTATGTCATG AGTTGGGTCCGCCAGGCTCCAGGGAAGGGGCTG GAGTGGGTCTCAGCTATTAGTGGTAGTGGTAGTA GAATATACTACGCGGACTCCGTGAAGGGCCGGTT CACCATCTCCAGAGACAATTCCAAGAACACGCTG TATCTGCAAATGAACAGCCTGAGAGCCGAGGAC ACGGCCTTATATTACTGTGCGAGAGTGGCTTTCT TTGACTATTGGGGCCAGGGAACCCTGGTCACCGT CTCCTCA |
|---|---|---|---|---|
| | | | SEQ ID NO: 26463 | SEQ ID NO: 30469 |
| | | AA | DIQMTQSPSSLSASVGDRVTITCRASQDISNYLA WFQQKPGKAPKSLIYAASSLQSGVPSKFSGSGSG TDFTLTISSLQPEDFATYYCQQYHSYPFTFGPGT KVDIK | EVQLLESGGGLVQSGGGSLRLSCAASGFTFSSYVMS WVRQAPGKGLEWVSAISGSGSRIYYADSVKGRFTI SRDNSKNTLYLQMNSLRAEDTALYYCARVAFFDY WGQGTLVTVSS |
| | | | SEQ ID NO: 26464 | SEQ ID NO: 30470 |
| iPS:435681 | 21-225_169D11 | NA | GACATCCAGATGACCCAGTCTCCATCCTCCCT GTCTGCATCTGTAGGAGACAGAGTCACCATCA CTTGCCGGGCAAGTCAGGGCATTAGAGATGAT TTAGGCTGGTATCAACAGAAACCAGGGAAAG TCCCTAAGCGCCTGATATATGCTGCATCCAGT TTGCAAAGTGGGGTCCCATCAAGGTTCAGCGG CAGTGGATCTGGGACAGAATTCACTCTCACAA TCAGCAGCCTACAGCCTGAAGATTTTGCAACT TATTACTGTCTTCAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAATTGAACAGCCTGAGAGCCGAGG ACACGGCTGTGTATTACTGTGCGAGAGAAAGGTA TAGCAGTGGCTGGTACGACTACGGTATGGACGTC TGGGGCCAAGGGACCACGGTCACCGTCTCCTCA |
| | | | SEQ ID NO: 26465 | SEQ ID NO: 30471 |

FIGURE 50

| | | | | |
|---|---|---|---|---|
| | | AA | DIQMTQSPSSLSASVGDRVTITCRASQGIRDDLG WYQQKPGKVPKRLIYAASSLQSGVPSRFSGSGS GTEFTLTISSLQPEDFATYYCLQHYSYPRTFGQG TKVEIK | QVQLVESGGGVVQPGRSLRLSCAASGFTFSDYVM HWVRQAPGKGLEWVAVIWYDGSNKYYADSVKGR FTISRDNSKNTLYLQLNSLRAEDTAVYYCARERYSS GWYDYGMDVWGQGTTVTVSS |
| | | | SEQ ID NO: 26466 | SEQ ID NO: 30472 |
| iPS:435683 | 21-225_170A1 | NA | GAGATTGTGATGACCCAGACTCCACTCTTCCT GTCCGTCACCCCTGGACAGCCGGCCTCCATCT CCTGCAAGTCTAGTCAGAGCCTCCTGCATGGT GATGGAAAGACCTATTTGTTTTGGTACCTGCA GAAGCCAGGCCAGCCTCCACAGGTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATTCAGCTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT TATAAATACTATGCAGATTCCGTGAAGGGCCGAT TCACCATCTCCAGAGACAATTCCAAGAACACACT GTATCTGCAAATGAACAGCCTGAGAGCCGAGGA CACGGCTGTGTATTACTGTGCGAGAGATGCCCAC GATTTTTGGAGTGGTTACTTTGACTCCTGGGGCC AGGGAACCCTGGTCACCGTCTCCTCA |
| | | | SEQ ID NO: 26467 | SEQ ID NO: 30473 |
| | | AA | EIVMTQTPLFLSVTPGQPASISCKSSQSLLHGDGK TYLFWYLQKPGQPPQVLIYEVSNRFSGVPDRFSG SGSGTDFTLKISRVEAEDVGVYYCMQSIQLPWTF GQGTKVEIK | QVQLVESGGGVVQPGRSLRLSCAASGFTFSSYGMH WVRQAPGKGLEWVAIIWYDGSYKYYADSVKGRFT ISRDNSKNTLYLQMNSLRAEDTAVYYCARDAHDF WSGYFDSWGQGTLVTVSS |
| | | | SEQ ID NO: 26468 | SEQ ID NO: 30474 |

FIGURE 50

| iPS:435685 | 21-225_170E1 | NA | GACATCCAGATGACCCAGTCTCCATCCTCACT GTCTGCATCTGAAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATCATAGTTACCCATTCA CTTTCGGCCCTGGGACCAAAGTGGATATCAAA | GAGGTGCAGTTGTTGGAGTCTGGGGGAGGCTTGG TACAGCCTGGGGGGGTCCCTGAGACTCTCCTGTGC AGCCTCTGGATTCACCTTTAGCAGCTATGTCATG AGTTGGGTCCGCCAGGCTCCAGGGAAGGGGCTG GAGTGGGTCTCAGCTATTAGTGGTAGTGGTAATA GAATATACTACGCAGACTCCGTGAAGGGCCGGTT CACCATCTCCAGAGACAATTCCAAGAACACGCTG TATCTGCAAATGAACAGCCTGAGAGCCGAGGAC ACGGCCTTATATTACTGTGCGAGAGTGGCTTTCT TTGACTATTGGGGCCAGGGAACCCTGGTCACCGT CTCCTCA |
| | | | SEQ ID NO: 26469 | SEQ ID NO: 30475 |
| | | AA | DIQMTQSPSSLSASEGDRVTITCRASQGISNYLA WFQQKPGKAPKSLIYAASSLQSGVPSKFSGSGSG TDFTLTISSLQPEDFATYYCQQYHSYPFTFGPGT KVDIK | EVQLLESGGGLVQPGGSLRLSCAASGFTFSSYVMS WVRQAPGKGLEWVSAISGSGNRIYYADSVKGRFTI SRDNSKNTLYLQMNSLRAEDTALYYCARVAFFDY WGQGTLVTVSS |
| | | | SEQ ID NO: 26470 | SEQ ID NO: 30476 |
| iPS:435687 | 21-225_170H1 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTACATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTATTGTCTACAGCATAGTAGTAACCCGTG GACGTTCGGCCAAGGGACCAAGGTGGAAAGC AAA | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTATTACTGG AGCTGGATCCGGCAGCCCGCCGGGAAGGGACTG GAGTGGATTGGGCGTATCTATACCAGTGGGAGCA CCAACTACAACCCCTCCCTCAAGAGTCGAGTCAC CATGTCAGTAGACACGTCCAAGAACCAGTTCTCC CTGAAGCTGAGCTCTGTGACCGCCGCGGACACG GCCGTGTATTACTGTGCGAGAGTCGGGAGGTACT ACTATGGTATGGACGTCTGGGGCCAAGGGACCA CGGTCACCGTCTCCTCA |
| | | | SEQ ID NO: 26471 | SEQ ID NO: 30477 |

FIGURE 50

| | | | | |
|---|---|---|---|---|
| | | AA | DIQMTQSPSSLSASVGDRVTITCRASQGIRNDLG WYQQKPGKAPKRLIYATSSLQSGVPSRFSGSGSG TEFTLTISSLQPEDFATYYCLQHSSNPWTFGQGT KVESK | QVQLQESGPGLVKPSETLSLTCTVSGGSISSYYWSW IRQPAGKGLEWIGRIYTSGSTNYNPSLKSRVTMSVD TSKNQFSLKLSSVTAADTAVYYCARVGRYYYGMD VWGQGTTVTVSS |
| | | | SEQ ID NO: 26472 | SEQ ID NO: 30478 |
| iPS:435689 | 21-225_170F3 | NA | GACATCCAGATGACCCAGTCTCCATCCTCCCT GTCTGCTTCTGTACAAGACAGAGTCACCATCA CTTGCCGGGCAAGTCGGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAACAGCCTGCAGCCTGAAGATTTTGCAACT TATTACTGTCTACAGCATTATAGTTACCCTCGG ACGTTCGGCCAAGGGACCAAGGTGGAAATCA AA | CAGGTGCAGCTGGTGGAATCTGGGGGAGGCGTG GTCCAGCCTGGGAGGTCCCTGAGACTCTCCTGTG CAGCGTCTGGATTCTCCTTCAGTGACTATGTCAT GCACTGGGTCCGCCAGGCTCCAGGCAAGGGGCT GGAGTGGGTGGCAGTTATATGGTATGATGGAAGT AATAAATACTATGAAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGACGTA TAGCAGCAGCTGGTACGACTACGGTATGGACGTC TGGGGCCAAGGGACCACGGTCACCGTCTCCTCA |
| | | | SEQ ID NO: 26473 | SEQ ID NO: 30479 |
| | | AA | DIQMTQSPSSLSASVQDRVTITCRASRGIRNDLG WYQQKPGKAPKRLIYAASSLQSGVPSRFSGSGS GTEFTLTINSLQPEDFATYYCLQHYSYPRTFGQG TKVEIK | QVQLVESGGGVVQPGRSLRLSCAASGFSFSDYVMH WVRQAPGKGLEWVAVIWYDGSNKYYEDSVKGRF TISRDNSKNTLYLQMNSLRAEDTAVYYCARETYSS SWYDYGMDVWGQGTTVTVSS |
| | | | SEQ ID NO: 26474 | SEQ ID NO: 30480 |

FIGURE 50

| iPS:435693 | 21-225_170G4 | NA | GACATCCAGATGACCCAGTCTCCATCCTCCCT GTCTGCATCGTGTAGGAGACAGAGTCACCATCA CTTGCCGGGCAAGTCAGGGCATTAGAAATGAT TTAGGCTGGTATCACCAGAAACCGGGGAAAG CCCCTAAGCGCCTGATCTATGCTGCATCCACTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATTATAGTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTA CAGCGTCTGGATTCACCTTCAGTACCTATGGCAT GCACTGGGTCCGCCAGGCTCCAGGCAAGGGGCT GGAGTGGGTGTCAATTATATGGTATGATGGAACT AATAAATACTATGCAGACTCCGTGAAGGGCCGA TTCACCATCTCCAGAGACAATTCCAAGAACACGC TGTTTCTGCAACTGAACAGCCTGAGAGCCGAGGA CACGGCTATGTATTACTGTGCGCGAGATCCCTTA CGTGGATACAATGACCCGGTTATGGACTACTGGG GCCAGGGAACCCTGGTCACCGTCTCCTCA |
| | | | SEQ ID NO: 26475 | SEQ ID NO: 30481 |
| | | AA | DIQMTQSPSSLSASVGDRVTITCRASQGIRNDLG WYHQKPGKAPKRLIYAASTLQSGVPSRFSGSGS GTEFTLTISSLQPEDFATYYCLQHYSYPLTFGGG TKVEIK | QVQLVESGGGVVQPGRSLRLSCTASGFTFSTYGMH WVRQAPGKGLEWVSIIWYDGTNKYYADSVKGRFT ISRDNSKNTLFLQLNSLRAEDTAMYYCARDPLRGY NDPVMDYWGQGTLVTVSS |
| | | | SEQ ID NO: 26476 | SEQ ID NO: 30482 |
| iPS:435695 | 21-225_170D5 | NA | GACATCCAGATGACCCAGTCTCCATCCTCCCT GTCTGCATCGTGTCGGAGACAGAGTCACCATCA CTTGCCGGGCAAGTCAGGGCATTAGAAATGAT TTAGGCTGGTATCAGGAGAAACCAGGGAAAG CCCCTAAACACCTGATCTATGCTGCATCCAGT TTGCAAAATGGGGTCCCATCAAGGTTCAGCGG CAGTGGATCTGGGACAGAGTTCACTCTCACAA TCAGCAGCCTGCAGCCTGAAGATTTTGCAACT TATTACTGTCTACAGCATTATAGTTTCCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAACT AATAAATACTATGCAGACTCCGTGAAGGGCCGA TTCACCATCTCCAGAGACAATTCCAAGAACACGC TGTATCTGCAACTGAACAGCCTGAGAGCCGAGG ACACGGCTGTGTATTACTGTGCGCGAGATCCCTT ACGTGGATACAATGACCCGGTTATGGACTACTGG GGCCAGGGAACCCTGGTCACCGTCTCCTCA |
| | | | SEQ ID NO: 26477 | SEQ ID NO: 30483 |

FIGURE 50

| | | | | |
|---|---|---|---|---|
| | | AA | DIQMTQSPSSLSASVGDRVTITCRASQGIRNDLG WYQEKPGKAPKHLIYAASSLQNGVPSRFSGSGS GTEFTLTISSLQPEDFATYYCLQHYSFPLTFGGGT KVEIK | QVQLVESGGGVVQPGRSLRLSCAASGFTFSSYGMH WVRQAPGKGLEWVAIIWYDGTNKYYADSVKGRF TISRDNSKNTLYLQLNSLRAEDTAVYYCARDPLRG YNDPVMDYWGQGTLVTVSS |
| | | | SEQ ID NO: 26478 | SEQ ID NO: 30484 |
| iPS:435697 | 21-225_170G5 | NA | GACATCCAGATGACCCAGTCTCCATCCTCCCT GTCTGCATCTGTAGGAGACAGAGTCACCATCA CTTGCCGGGCAAGTCAGGGCATTAGAACTGAT TTAGGCTGGTTTCAGCAGAAACCAGGGAAAGC CCCTAAGCGCCTGATCTATACTGCATCCAGTTT GCAAAGTGGGGTCCCATCAAGGTTCAGCGGCA GTGGATCTGGGACAGAATTCACTCTCACGATC AGCAGCCTGCAGCCTGAAGATTTTGCAACTTA TTACTGTCTACAGCATTATAGTTACCCGCTCAC TTTCGGCGGAGGGACCAAGGTGGAGATCAAA | CAGGTGCAGCTGGTGGAGTCTGGGGGAGGCGTG GTCCAGCCTGGGGGGTCCCTGAGACTCTCCTGTG CAGCGTCTGGCTTCACCTTCAGTACCTATGGCAT GCACTGGGTCCGCCAGGCTCCAGGCAAGGGGCT GGAGTGGGTGACAATTATATGGTATGATGGGACT AATAAATACTATGCAGACTCCGTGAAGGGCCGA TTCACCATCTCCAGAGACAATTCCAAGAGCACGC TGTATCTGCAAATGAACAGCCTGAGAGCCGAGG ACACGGCTGTGTATTTCTGTGCGAGAGATCCCTT ACGTGGATACAATGACCCGGTTATGGACTACTGG GGCCAGGGAACCCTGGTCACCGTCTCCTCA |
| | | | SEQ ID NO: 26479 | SEQ ID NO: 30485 |
| | | AA | DIQMTQSPSSLSASVGDRVTITCRASQGIRTDLG WFQQKPGKAPKRLIYTASSLQSGVPSRFSGSGSG TEFTLTISSLQPEDFATYYCLQHYSYPLTFGGGT KVEIK | QVQLVESGGGVVQPGGSLRLSCAASGFTFSTYGMH WVRQAPGKGLEWVTIIWYDGTNKYYADSVKGRFT ISRDNSKSTLYLQMNSLRAEDTAVYFCARDPLRGY NDPVMDYWGQGTLVTVSS |
| | | | SEQ ID NO: 26480 | SEQ ID NO: 30486 |

FIGURE 50

| | | | | |
|---|---|---|---|---|
| iPS:435699 | 21-225_170D6 | NA | GACATCCAGATGACCCAGTCTCCATCCTCTCT GTCTGCATCTGTAGGAGACAGAGTCGCCATCA CTTGTCGGGCGAGTCAGGACATTGGCAATTGT TTAGCCTGGTTTCAGCAGAAACCAGGGAAAGC CCCTAAGTCCCTGATCTATTCTGCGTCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAATCTGATAGTTACCCTCTCAC TTTCGGCGGAGGGACCAAGGTGGAGATCAAA | CAGGTGCAGCTGGTGCAGTCTGGGGCTGAGGTG AAGAAGCCTGGGGCCTCAGTGAGGGTCTCCTGC AAGGCTTCTGGATACACCTTCACCGGCTACTTTA TACACTGGGTGCGACAGGCCCCTGGACAAGGGC TTGAGTGGATGGGATGGATCAAGCCTAACAGTG GTGGCACAAACTCTGCACAGAGGTTTCAGGGCA GGGTCACCATGACCAGGGACACGTCCATCAACA CAGCCTACATGGAGCTGAACAGGCTGAGATCTG ACGACACGGCCGTGTATTACTGTGCGAGAGGGG GGACTACGGTGGCTACGTGGGGGGGTCTTTGACTA CTGGGGCCAGGGAACCCTGGTCACCGTCTCCTCA |
| | | | SEQ ID NO: 26481 | SEQ ID NO: 30487 |
| | | AA | DIQMTQSPSSLSASVGDRVAITCRASQDIGNCLA WFQQKPGKAPKSLIYSASSLQSGVPSKFSGSGSG TDFTLTISSLQPEDFATYYCQQSDSYPLTFGGGT KVEIK | QVQLVQSGAEVKKPGASVRVSCKASGYTFTGYFIH WVRQAPGQGLEWMGWIKPNSGGTNSAQRFQGRV TMTRDTSINTAYMELNRLRSDDTAVYYCARGGTT VATWGVFDYWGQGTLVTVSS |
| | | | SEQ ID NO: 26482 | SEQ ID NO: 30488 |
| iPS:435701 | 21-225_170F6 | NA | GACATCGTGATGACCCAGTCTCCAGACTCCCT GGCTGTGTCTCTGGGCGCGAGGGGCCACCATCA ACTGCAAGTCCAGCCAGAGTGTTTTACACAGC TCCAACAATTACAACTACTTAGCTTGGTACCA GCAGAGACCAGGACAGCCTCCTAAAGTGCTCA TTCACTGGGCATCTACCCGGAAATCCGGGGTC CCTGACCGATTCAGTGGCAGCGTGTCTGGGAC AGATTTCACTCTCACCATCAACAGCCTGCAGG CTGAAGATGTGGCAGTTTATTACTGTCAGCAA TATTATAGTACTCCGTGGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGACA GAGTCACCATGACCAGGCACACCTCCATAAGCA CAGCCTACATGGAGCTGAGCAGCCTGAGATCTG AGGACACGGCCGTGTATTACTGTGCGATTAGCAG TGGCTGGTACTGGTTCGACCCCTGGGGCCAGGGA ACCCTGGTCACCGTCTCCTCA |
| | | | SEQ ID NO: 26483 | SEQ ID NO: 30489 |

FIGURE 50

| | | | | |
|---|---|---|---|---|
| | | AA | DIVMTQSPDSLAVSLGARATINCKSSQSVLHSSN NYNYLAWYQQRPGQPPKVLIHWASTRKSGVPD RFSGSVSGTDFTLTINSLQAEDVAVYYCQQYYST PWTFGQGTKVEIK | QVQLVQSGAEVKKPGASVKVSCKASGYTFTNYDIN WVRQATGQGLEWMGWMNPNSGNTGYAQKFQDR VTMTRHTSISTAYMELSSLRSEDTAVYYCAISSGW YWFDPWGQGTLVTVSS |
| | | | SEQ ID NO: 26484 | SEQ ID NO: 30490 |
| iPS:435703 | 21-225_170D11 | NA | GACATCCAGATGACCCAGTCTCCATCCTCCCT GTCTGCATCTGTCGGAGACAGAGTCACCATCA CTTGCCGGGCAAGTCAGGGCATTAGAAATGAT TTAGGCTGGTATCAGCAGAAACCAGGGAAAG CCCCTAAACACCTGATCTATGCTGCATCCAGT TTGCAAAATGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTTCCCGCTC ACTTTCGGCGGAGGGACCAAGGTGGAGATCA GA | CAGGTGCAGCTGGTGGAGTCTGGGGGAGGCGTG GTCCAGCCTGGGAGGTCCCTGAGACTCTCCTGTG TAGCGTCTGGATTCACCTTCAGTAGCTATGGCAT GCACTGGGTCCGCCAGGCTCCAGGCAAGGGGCT GGAGTGGGTGGCAATTATATGGTATGATGGAACT AATAAATACTATGCAGACTCCGTGAAGGGCCGA TTCACCATCTCCAGAGACAATTCCAAGAACACGC TGTATCTGCAACTGAACAGCCTGAGAGCCGAGG ACACGGCTGTGTATTACTGTGCGCGAGATCCCTT ACGTGGATACAATGACCCGGTTATGGACTACTGG GGCCAGGGAACCCTGGTCACCGTCTCCTCA |
| | | | SEQ ID NO: 26485 | SEQ ID NO: 30491 |
| | | AA | DIQMTQSPSSLSASVGDRVTITCRASQGIRNDLG WYQQKPGKAPKHLIYAASSLQNGVPSRFSGSGS GTEFTLTISSLQPEDFATYYCLQHYSFPLTFGGGT KVEIR | QVQLVESGGGVVQPGRSLRLSCVASGFTFSSYGMH WVRQAPGKGLEWVAIIWYDGTNKYYADSVKGRF TISRDNSKNTLYLQLNSLRAEDTAVYYCARDPLRG YNDPVMDYWGQGTLVTVSS |
| | | | SEQ ID NO: 26486 | SEQ ID NO: 30492 |

FIGURE 50

| iPS:435705 | 21-225_171C3 | NA | GACATCCAGATGACCCAGTCTCCATCCTCCCT GTCTGCATCTGTAGGAGACAGAGTCACCATCA CTTGCCGGGCAAGTCAGGGCATTAGAACTGAT TTAGGCTGGTTTCAGCAGAAACCAGGGAAAGC CCCTAAGCGCCTGATCTATACTGCATCCAGTTT GCAAAGTGGGGTCCCATCAAGGTTCAGCGGCA GTGGATCTGGGACAGAATTCACTCTCACGATC AGCAGCCTGCAGCCTGAAGATTTTGCAACTTA TTACTGTCTACAGCATTATAGTTACCCGCTCAC TTTCGGCGGAGGGACCAAGGTGGAGATCAAA | CAGGTGCAGCTGGTGGAGTCTGGGGGAGGCGTG GTCCAGCCTGGGGGGTCCCTGAGACTCTCCTGTG CAGCGTCTGGCTTCACCTTCAGTACCTATGGCAT GCACTGGGTCCGCCAGGCTCCAGGCAAGGGGCT GGAGTGGGTGACAATTATATGGTATGATGGGACT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCCCTT ACGTGGATACAATGACCCGGTTATGGACTACTGG GGCCAGGGAACCCTGGTCACCGTCTCCTCA |
| | | | SEQ ID NO: 26487 | SEQ ID NO: 30493 |
| | | AA | DIQMTQSPSSLSASVGDRVTITCRASQGIRTDLG WFQQKPGKAPKRLIYTASSLQSGVPSRFSGSGSG TEFTLTISSLQPEDFATYYCLQHYSYPLTFGGGT KVEIK | QVQLVESGGGVVQPGGSLRLSCAASGFTFSTYGMH WVRQAPGKGLEWVTIIWYDGTNKYYADSVKGRFT ISRDNSKNTLYLQMNSLRAEDTAVYYCARDPLRGY NDPVMDYWGQGTLVTVSS |
| | | | SEQ ID NO: 26488 | SEQ ID NO: 30494 |
| iPS:435709 | 21-225_171A4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACGA TCAGCAGCCTGCAGCCTGAAGATTTTGCAACT TATTACTGTCTACAGCATTATAGTTACCCGCTC ACTTTCGGCGGAGGGACCAAGGTGGAGATCA AA | CAGGTGCAACTGGTGGAGTCTGGGGGAGGCGTG GTCCAGCCTGGGGGGTCCCTGAGACTCTCCTGTG CAGCGTCTGGCTTCACCTTCAGTACCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAAG ACACGGCTGTGTATTACTGTGCGAGAGATCCCTT ACGTGGATACAATGACCCGGTTATGGACTACTGG GGCCAGGGAACCCTGGTCACCGTCTCCTCA |
| | | | SEQ ID NO: 26489 | SEQ ID NO: 30495 |

FIGURE 50

| | | | | |
|---|---|---|---|---|
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHYSYPLTFGGGT KVEIK | QVQLVESGGGVVQPGGSLRLSCAASGFTFSTYGMH WVRQAPGKGLEWVAIIWYDGSNKYYADSVKGRFT ISRDNSKNTLYLQMNSLRAEDTAVYYCARDPLRGY NDPVMDYWGQGTLVTVSS |
| | | | SEQ ID NO: 26490 | SEQ ID NO: 30496 |
| iPS:435711 | 21-225_171G4 | NA | GACATCCAGATGACCCAGTCTCCATCTTCCGT GTCTGCATCTGTAGGAGACAGAGTCACCATCA CTTGTCGGGCGAGTCAGGGTGTTAACGACTGG TTAGCCTGGTATCAGCAGAAACCAGGGAGAG CCCCTAAGCTCCTGATCTATGATGCATCAAGT TTGCAAAGTGGGGTCCCATCAAGGTTCAGCGG CAGTGGATCTGGGACAGATTTCACTCTCACCA TCAGCAGCCTGCAGCCTGAAGATTTTGCAACT TACTATTGTCAACAGGCTAACAGTTTCCCGTG GACGTTCGGCCAAGGGACCAAGGTGGAAATC AAA | GAGGTGCAGCTGTTGGAGTCTGGGGGAGACTTA GTACAGCCTGGGGGGTCCCTGAGACTCTCCTGTG CAGCCTCTGGATTCACCTTTAGCAGCTGTGCCAT GACCTGGGTCCGCCAGGCTCCAGGGAAGGGGCT GGAGTGGGTCTCAGCTATTAGTGGTCGTGGTGGT ACCACGTTCTACGCAGACTCCGTGAGGGGCCGGT TCACCATCTCCAGAGACAATTCCAAGAACACGCT GTTTCTGCAAATGAACAGCCTGAGAGCCGAGGA CACGGCCGTATATTACTGTGCGAAAGATCTTATT GGGGGAGCTACTTACTTTGACTACTGGGGCCAGG GAACCCTGGTCACCGTCTCCTCA |
| | | | SEQ ID NO: 26491 | SEQ ID NO: 30497 |
| | | AA | DIQMTQSPSSVSASVGDRVTITCRASQGVNDWL AWYQQKPGRAPKLLIYDASSLQSGVPSRFSGSGS GTDFTLTISSLQPEDFATYYCQQANSFPWTFGQG TKVEIK | EVQLLESGGDLVQPGGSLRLSCAASGFTFSSCAMT WVRQAPGKGLEWVSAISGRGGTTFYADSVRGRFTI SRDNSKNTLFLQMNSLRAEDTAVYYCAKDLIGGAT YFDYWGQGTLVTVSS |
| | | | SEQ ID NO: 26492 | SEQ ID NO: 30498 |

FIGURE 50

| | | | | |
|---|---|---|---|---|
| iPS:435713 | 21-225_171D7 | NA | GATATTGTGATGACTCAGTCTCCACTCTCCCTG CCCGTCACCCCTGGAGAGCCGGCCTCCATCTC CTGCAGGTCTAGTCAGAGCCTCCTGTATCATA ATGGATACAACTATTTGGATTGGTACCTGCAG AAGACAGGGCAGTCTCCACAGCTCCTGATCTA TGTGGGTTCTAATCGGGCCTCCGGGGTCCCTG ACAGGTTCAGTGGCAGTGGATCAGGCACAGAT TTTACACTGAAAATCAGCAGAGTGGAGGCTGA GGATGTTGGGGTTTATTACTGCATGCAAACTC TACAAACTCCGCTCACTTTCGGCGGAGGGACC AAGGTGGAGATCAAA | CAGGTGCAGCTGGTGGAGTCTAGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGACGGAAA CAATAGACACTATGCAGACTCCGTGCAGGGCCG ATTCACCATTTCCAGAGACAATTCCAAGAACACG CTGTCTCTGCAAATGAACAGCCTGGGAGCTGAGG ACACGGCTGTGTATTACTGTGCGAGAGATCGTCA CCGTTTGGACTACTACGCTTTGGACGTCTGGGGC CAAGGGACCACGGTCACCGTCTCCTCA |
| | | | SEQ ID NO: 26493 | SEQ ID NO: 30499 |
| | | AA | DIVMTQSPLSLPVTPGEPASISCRSSQSLLYHNGY NYLDWYLQKTGQSPQLLIYVGSNRASGVPDRFS GSGSGTDFTLKISRVEAEDVGVYYCMQTLQTPL TFGGGTKVEIK | QVQLVESRGGVVQPGRSLRLSCAASGFTFSSYGMH WVRQAPGKGLEWVAVISYDGNNRHYADSVQGRF TISRDNSKNTLSLQMNSLGAEDTAVYYCARDRHRL DYYALDVWGQGTTVTVSS |
| | | | SEQ ID NO: 26494 | SEQ ID NO: 30500 |
| iPS:435715 | 21-225_171A8 | NA | GCCATCCAGATGACCCAGTCTCCATCTTCCGT GTCTGCATCTGTAGGAGACAGAGTCACCATCA CTTGTCGGGCGAGTCAGGGTATTAGCAACTGG TTAGCCTGGTATCAGCAGAAACCAGGGAAAG CCCCTAACCTCATGATCCATGCTGCATTCAGTT TGCAAGGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGACTAACAGTTTCCCGTGG ACGTTCGGCCAAGGGACCAAGGTGGAAATCA AA | GAGGTGCAGTTGTTGGAGTCTGGGGGAGGCTTGG TACAGCCTGGGGGGTCCCTGAGACTCTCCTGTGC AGCCTCTGGATTCACCTTTAGGAGCTCTGCCATG AGCTGGGTCCGCCAGGCTCCAGGGAAGGGGCTG GAGTGGGTCTCAGTTATTAGTGGTAGTGGTGGTA GCACATTCTACACAGACTCCGTGAAGGGCCGGTT CACCATCTCCAGAGACAATTCCAAGAACACGCTG TATCTGCAAATGAACAGCCTGAGAGCCGAGGAC ACGGCCGTATATTACTGTGCGAAATCGAATAGCA GTGGCTGGTTTGACTACTGGGGCCAGGGAACCCT GGTCACCGTCTCCTCA |
| | | | SEQ ID NO: 26495 | SEQ ID NO: 30501 |

FIGURE 50

| | | | | |
|---|---|---|---|---|
| | | AA | AIQMTQSPSSVSASVGDRVTITCRASQGISNWLA WYQQKPGKAPNLMIHAAFSLQGGVPSRFSGSGS GTDFTLTISSLQPEDFATYYCQQTNSFPWTFGQG TKVEIK | EVQLLESGGGLVQPGGSLRLSCAASGFTFRSSAMS WVRQAPGKGLEWVSVISGSGGSTFYTDSVKGRFTI SRDNSKNTLYLQMNSLRAEDTAVYYCAKSNSSGW FDYWGQGTLVTVSS |
| | | | SEQ ID NO: 26496 | SEQ ID NO: 30502 |
| iPS:435717 | 21-225_171A9 | NA | GACATCCAGATGACCCAGTCTCCATCTTCCGT GTCTGCATCTGTAGGAGACAGAGTCACCATCA CTTGTCGGGCGAGTCAGGATATTACCACCTGG TTAGCCTGGTATCAGCAGAAACCAGGGAAAG CCCCTAAGCTCCTGATCTATGATGCATCCAGTT TGCAAAGTGCGGTCCCATCAAGGTTCAGCGGC AGTGGATCTGGGACAGATTTCACTCTCACCGT CAGCAGTTTGCAGCCTGAAGATTTTGCAACTT ACTATTGTCTACAGACTAACAGTTTCCCGTGG ACGTTCGGCCAAGGGACCAAGGTGGAGATCA AG | GAGGTGCAGTTGTTGGAGTCTGGGGGAGGCTTGG CACAGCCTGGGGGGGTCCCTGAGACTCTCCTGTGC AGCCTCTGGATTCACCTTTAGCAGCTATGCCATG ACTTGGGTCCGCCAGGCTCCAGGGAAGGGGCTG GAGTGGGTCTCAGCTATTAGTGGTAGTGGTGGTA ACACATTCAACGCAGACTCCGTGAAGGGCCGGTT CACCATCTCCAGAGACAATTCCAAGAACACGCTG TATCTGCAAATGAACAGCCTGAGAGCCGAGGAC ACGGCCGTATATTACTGTGCAAAGCTGGGGATCG ACTACTACTACTACGGTATGGACGTCTGGGGCCA AGGGACCACGGTCACCGTCTCCTCA |
| | | | SEQ ID NO: 26497 | SEQ ID NO: 30503 |
| | | AA | DIQMTQSPSSVSASVGDRVTITCRASQDITTWLA WYQQKPGKAPKLLIYDASSLQSAVPSRFSGSGS GTDFTLTVSSLQPEDFATYYCLQTNSFPWTFGQG TKVEIK | EVQLLESGGGLAQPGGSLRLSCAASGFTFSSYAMT WVRQAPGKGLEWVSAISGSGGNTFNADSVKGRFTI SRDNSKNTLYLQMNSLRAEDTAVYYCAKLGIDYY YYGMDVWGQGTTVTVSS |
| | | | SEQ ID NO: 26498 | SEQ ID NO: 30504 |

FIGURE 50

| iPS:435719 | 21-225_171A11 | NA | GACATCCAGATGACCCAGTCTCCATCCTCCCT GTCTGCATCTGTAGGAGACAGAGTCACCATCA CTTGCCGGGCAAGTCAGGGCATTAGAAATAAT TTAGGCTGGTATCAGCAGAAACCAGGGAAAG CCCCTAAGCGCCTGATCTATCCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGGATCATAGTTACCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATAGGGGC AGCTCCTGGGGCCAGGGAATCCTGGTCACCGTCT CCTCA |
|---|---|---|---|---|
| | | | SEQ ID NO: 26499 | SEQ ID NO: 30505 |
| | | AA | DIQMTQSPSSLSASVGDRVTITCRASQGIRNNLG WYQQKPGKAPKRLIYPASSLQSGVPSRFSGSGSG TEFTLTISSLQPEDFATYYCLQDHSYPFTFGPGTK VDIK | EVQLVESGGGLVKPGGSLRLSCAASGFTFSSYSMN WVRQAPGKGLEWVSSISGSSSYIYYADSVKGRFTIS RDNAKNSLYLQMNSLRAEDTAVYYCARDRGSSW GQGILVTVSS |
| | | | SEQ ID NO: 26500 | SEQ ID NO: 30506 |
| iPS:435721 | 21-225_172B3 | NA | GACATCCAGATGACCCAATCTCCATCCTCCCT GTCTGCATCTGTAGGAGACAGAGTCACCATCA CTTGCCGGGCTAGTCAGGGCATTAGAAATGAT TTAGGCTGGTATCAGCAGAAACCAGGGAAAG CCCCTAAGCGCCTGATCGGTCGGTGCTGAATCCAGT TTGCAAAGTGGGGTCCCATCAAGGTTCAGCGG CAGTGGATCTGGGACAGAATTCACTCTCACAA TCAGCGGCCTGCAGCCTGAAGATTTTGCAACT TATTACTGTCTACAGCATTATAGTTACCCGCTC ACTTTCGGCGGAGGGACCAAAGTGGAGATCA AA | CAGGTGCAGCTGGTGGAGTCTGGGGGAGGCGTG GTCCAGCCTGGGAGGCCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCCCTT ACGTGGATACAATGACCCGGTTATGGACTACTGG GGCCAGGGAACCCTGGTCACCGTCTCCTCA |
| | | | SEQ ID NO: 26501 | SEQ ID NO: 30507 |

FIGURE 50

| | | | | |
|---|---|---|---|---|
| | | AA | DIQMTQSPSSLSASVGDRVTITCRASQGIRNDLG WYQQKPGKAPKRLIGAESSLQSGVPSRFSGSGSG TEFTLTISGLQPEDFATYYCLQHYSYPLTFGGGT KVEIK | QVQLVESGGGVVQPGRPLRLSCAASGFTFSTYGMH WVRQAPGKGLEWVAVIWYDGSNKYYADSVKGRF TISRDNSKNTLYLQMNSLRAEDTAVYYCARDPLRG YNDPVMDYWGQGTLVTVSS |
| | | | SEQ ID NO: 26502 | SEQ ID NO: 30508 |
| iPS:435723 | 21-225_172B7 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTCTATTGGTACCTGCA GAAGCCAGGCCAGCCTCCACAGGTCCTGTTAT TTGAAGTTTCCCACCGGTTCTCTGGAGTGCCA GATAGGTTCAGTGGCGGCGGGTCAGGGACAG ATTTCACACTGAAGATCAGCCGGGTGGAGGCT GAGGATGTTGGGGTTTACTATTGCATGCAAAG TATACAGTTTCCGTGGACGTTCGGCCAAGGGA CCAGGGTGGACATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT AGAGTGGGTGGCAATTATATGGTATGATGGAAGT AATAAATACTATGTAGACTCCGTGAAGGGCCGAT TCACCATCTCCAGAGACAATTCCAAGAACACGCT GTATCTCCAAATGAACAGCCTGAGAGCCGAAGA CACGGCTGTGTACTATTGTGCGAGAGAGGCGTAC GATTTTTGGAGTGGTTATTGGGACTACTGGGGCC AGGGAACCCTGGTCACCGTCTCCTCA |
| | | | SEQ ID NO: 26503 | SEQ ID NO: 30509 |
| | | AA | DIVMTQTPLSLSVTPGQPASISCKSSQSLLHGDLG KTYFYWYLQKPGQPPQVLLFEVSHRFSGVPDRF SGGGSGTDFTLKISRVEAEDVGVYYCMQSIQFP WTFGQGTRVDIK | QVQLVESGGGVVQPGRSLRLSCAASGFTFSNYGM HWVRQAPGKGLEWVAIIWYDGSNKYYVDSVKGR FTISRDNSKNTLYLQMNSLRAEDTAVYYCAREAYD FWSGYWDYWGQGTLVTVSS |
| | | | SEQ ID NO: 26504 | SEQ ID NO: 30510 |

FIGURE 50

| iPS:435725 | 21-225_172G8 | NA | GACATCCAGATGACCCAGTCTCCATCCTCCCT GTCTGCATCTGTCGGAGACAGAGTCACCATCA CTTGCCGGGCAAGTCAGGGCGTTAGAAATGAT TTAGGCTGGTATCAGCAGAAACCAGGGAAAG CCCCTAAACACCTGATCTATGCTGCATCCAGT TTGCAAAATGGGGTCCCATCAAGGTTCAGCGG CAGTGGCTCTGGGACAGAATTCACTCTCACAA TCAGCAGCCTGCAGCCTGAAGATTTTGCCACT TATTACTGTCTACACCATTATAGTTTCCCGCTC ACTTTCGGCGGAGGGACCAAGGTGGAGATCA AA | CAGGTGCAGATGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAATGGATGGCAATTATATGGTATGATGGAACT AATAAATACTATGCAGACTCCGTGAAGGGCCGA TTCACCATCTCCAGAGACAATTCCAAGAACACGC TGTATCTGCAACTGAACAGCCTGAGAGCCGAGG ACACGGCTGTGTATTACTGTGCGCGAGATCCCTT ACGTGGATACAATGACCCGGTTATGGACTACTGG GGCCAGGGAACCCTGGTCACCGTCTCCTCA |
| | | | SEQ ID NO: 26505 | SEQ ID NO: 30511 |
| | | AA | DIQMTQSPSSLSASVGDRVTITCRASQGVRNDLG WYQQKPGKAPKHLIYAASSLQNGVPSRFSGSGS GTEFTLTISSLQPEDFATYYCLHHYSFPLTFGGGT KVEIK | QVQMVESGGGVVQPGRSLRLSCAASGFTFSTYGM HWVRQAPGKGLEWMAIIWYDGTNKYYADSVKGR FTISRDNSKNTLYLQLNSLRAEDTAVYYCARDPLR GYNDPVMDYWGQGTLVTVSS |
| | | | SEQ ID NO: 26506 | SEQ ID NO: 30512 |
| iPS:435727 | 21-225_172E11 | NA | GACATCGTGATGACCCAGTCTCCAGACTCCCT GGCGGTGTCTCTGGGCGAGAGGGCCACCATCA ACTGCAAGTCCAGCCAGAGTGTTTTACACAGC TCCAACAATAACAACTACTTAGCTTGGTACCA GCAGAAACCAGGACAGCCTCCTAAGCTGCTCA TTTACTGGGCATCTACTCGGGAATCCGGGGTC CCTGACCGATTCAGTGGCAGCGGGTCTGGGAC AGATTTCATTCTCACCATCAGCGGCCTGCAGG CTGAAGATGTGGCAGTTTATTACTGTCAGCAA TATTTTACTACTCCGTGCAGTTTTGGCCAGGGG ACCAAGCTGGAGATCAAA | CAGGTGCAGCTGGTGCAGTCTGGGGGCTGAGGTG AAGAAGCCTGGGGCCTCAGTGATGGTCTCCTGCA AGGCTTCTGGATACACCTTCACCAATTATGATAT CAACTGGGTGCGACAGGCCACTGGACAAGGGCT TGAGTGGATGGGATGGATGCACCCTAACAGTGGT AACACAGGCTATGCACAGAAGTTCCAGGGCAGA GTCACCATGACCAGGAACACCTCCATAAGCACA GCCTACATGGAGCTGAGCAGCCTGAGATCTGAG GACACGGCCGTGTATTACTGTGCGTATAGCAGTG GCTGGTACCGGTTTGACTACTGGGGCCAGGGAAC CCTGGTCACCGTCTCCTCA |
| | | | SEQ ID NO: 26507 | SEQ ID NO: 30513 |

FIGURE 50

| | | | | |
|---|---|---|---|---|
| | | AA | DIVMTQSPDSLAVSLGERATINCKSSQSVLHSSN NNNYLAWYQQKPGQPPKLLIYWASTRESGVPD RFSGSGSGTDFILTISGLQAEDVAVYYCQQYFTT PCSFGQGTKLEIK | QVQLVQSGAEVKKPGASVMVSCKASGYTFTNYDI NWVRQATGQGLEWMGWMHPNSGNTGYAQKFQG RVTMTRNTSISTAYMELSSLRSEDTAVYYCAYSSG WYRFDYWGQGTLVTVSS |
| | | | SEQ ID NO: 26508 | SEQ ID NO: 30514 |
| iPS:435729 | 21-225_173E7 | NA | GACATCCAGATGACCCAGTCTCCATCCTCCCG GTCTGCATGTATAGGAGACAGAGCCACCATCA CTTACCGTGCAAGTCAGACCATTAGCAACTAT TTAAATTGGTATCAGCAGAAACCAGGGAAAG CCCCTAAACTCCTTATCTATGCTGCATCCAGTT TGCAAATTGGGGTCCCATCAAGGTTCAGTGGC AGTGGATCTGGGACAGATTTCACTCTCACCAT CAGCAGTGTGCAACCTGAAGATTTTGCAACTT ACTTCTGTCAACAGAGTTACAGAACCCCTCAG TGGACGTTCGGCCAAGGGACCAAGGTGGAAA TCAAA | GAGGTGCAGTTGTTGGAGTCTGGGGGAGGCTCG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCATTTATTAGTGGTAGTGGTGGT AACACATTCTACGCAGACTCCGTGAAGGGCCGGT TCACCATCTCCAGAGACAATTCCAAGAACACGCT GTATCTGCAAATGAACAGCCTGCAAGCCGAGGA CACGGCCGTATATTACTGTACGAAAAGGGATACC TACAACGGTTGGGATGCTTTTGATATCTGGGGCC AAGGGACAATGGTCACCGTCTCTTCA |
| | | | SEQ ID NO: 26509 | SEQ ID NO: 30515 |
| | | AA | DIQMTQSPSSRSACIGDRATITYRASQTISNYLN WYQQKPGKAPKLLIYAASSLQIGVPSRFSGSGSG TDFTLTISSVQPEDFATYFCQQSYRTPQWTFGQG TKVEIK | EVQLLESGGGSVQPGGSLRLSCAASGFTFSSYAMS WVRQAPGKGLEWVSFISGSGGNTFYADSVKGRFTI SRDNSKNTLYLQMNSLQAEDTAVYYCTKRDTYNG WDAFDIWGQGTMVTVSS |
| | | | SEQ ID NO: 26510 | SEQ ID NO: 30516 |

FIGURE 50

| iPS:435731 | 21-225_173A11 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAAGCCAGGCCAGCCTCCACAGCTCCTGATCT ATGAAGTTTCCAACCGGTTCTCTGGCGTGCCA GATAGGTTCAGTGGCAGCGGGTCAGGGACAG ATTTCACACTGAAAATCAGCCGGGTGGAGGCT GAGGATGTTGGGGTTTTTTTTCTGCATGCAAAG TATACAGGTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT AGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGCCTA CGATTTTTGGAGTGGTTTCTTTGACTCCTGGGGCC AGGGAACCCTGGTCACCGTCTCCTCA |
| | | | SEQ ID NO: 26511 | SEQ ID NO: 30517 |
| | | AA | DIVMTQTPLSLSVTPGQPASISCKSSQSLLHGDG KTYLYWYLQKPGQPPQLLIYEVSNRFSGVPDRFS GSGSGTDFTLKISRVEAEDVGVFFCMQSIQVPWT FGQGTKVEIK | QVQLVESGGGVVQPGRSLRLSCAASGFTFSSYGMH WVRQAPGKGLEWVAIIWYDGSNKYYADSVKGRFT ISRDNSKNTLYLQMNSLRAEDTAVYYCAREAYDF WSGFFDSWGQGTLVTVSS |
| | | | SEQ ID NO: 26512 | SEQ ID NO: 30518 |
| iPS:435733 | 21-225_173C11 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GAGGGAAAGACCTACTTGTATTGGTACCTGCA GAAGCCAGGCCAGCCTCCACAGCTCCTGATCT ATGAAGTTTCCCACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCTTCTCACTTTCGGCGGAGGGACCA AGGTGGAGATCAAA | CAGGTGCAGCTGGTGGAGTCGGGGGGGAGGCGTG GTCCAGCCTGGGAGGTCCCTGAGACTCTCCTGTG CAGCGTCTGGATTCACCTTCAGTAGCTATGGCAT ACACTGGGTCCGCCAGGCTCCAGGCAAGGGGACT GGAGTGGGTGGCACTTATATTTTATGATGGAAGT AATAAATACTATGCAGACTCCGTGAAGGGCCGA TTCACCATCTCCAGAGACAATTCCAAGAACACGC TGTATCTGCATATGAGCAGCCTGAGAGCCGAGG ACACGGCTGTGTATTACTGTGCGAGACGGTATAG CAGCAGCTGGTCCGGTGGTATGGACGTCTGGGGC CAAGGGACCACGGTCACCGTCTCCTCA |
| | | | SEQ ID NO: 26513 | SEQ ID NO: 30519 |

FIGURE 50

| | | | | |
|---|---|---|---|---|
| | | AA | DIVMTQTPLSLSVTPGQPASISCKSSQSLLHSEGK TYLYWYLQKPGQPPQLLIYEVSHRFSGVPDRFSG SGSGTDFTLKISRVEAEDVGVYYCMQSIQLLTFG GGTKVEIK | QVQLVESGGGVVQPGRSLRLSCAASGFTFSSYGIH WVRQAPGKGLEWVALIFYDGSNKYYADSVKGRFT ISRDNSKNTLYLHMSSLRAEDTAVYYCARRYSSSW SGGMDVWGQGTTVTVSS |
| | | | SEQ ID NO: 26514 | SEQ ID NO: 30520 |
| iPS:435735 | 21-225_173H12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTACATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAAGT TATTACTGTCTACAGCATTATAGTTTCCCGAAC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAACT AACAAATACTATGCAGACTCCGTGAAGGGCCGA TTCACCATCTCCAGAGACAATTCCAAGAACACGC TGTATCTGCAACTGAACAGCCTGAGAGCCGAGG ACACGGCTGTGTATTACTGTGCGCGAGATCCCTT ACGTGGATACAATGACCCGGTTATGGACTACTGG GGCCAGGGAACCCTGGTCACCGTCTCCTCA |
| | | | SEQ ID NO: 26515 | SEQ ID NO: 30521 |
| | | AA | DIQMTQSPSSLSASVGDRVTITCRASQGIRNDLG WYQQKPGKAPKRLIYTTSSLQSGVPSRFSGSGSG TEFTLTISSLQPEDFASYYCLQHYSFPNTFGGGTK VEIK | QVQLVESGGGVVQPGRSLRLSCAASGFTFSSYGMH WVRQAPGKGLEWVAIIWYDGTNKYYADSVKGRF TISRDNSKNTLYLQLNSLRAEDTAVYYCARDPLRG YNDPVMDYWGQGTLVTVSS |
| | | | SEQ ID NO: 26516 | SEQ ID NO: 30522 |

FIGURE 50

| iPS:435737 | 21-225_174G5 | NA | GACATCGTGATGACCCAGTCTCCAGATTCCCT GGCTGTGTCTCTGGGCGCGAGGGCCACCATCA ATTGCAAGTCCAGCCAGAGTGTATTACACAGC TCCAACAATTACAACTACTTAACTTGGTACCA GCAGAAATCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATCGTACTCCGTGGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGGTTAGCAGT GGCTGGTACTGGTTCGACCCCTGGGGCCAGGGA ACCCTGGTCACCGTCTCCTCA |
| --- | --- | --- | --- | --- |
| | | | SEQ ID NO: 26517 | SEQ ID NO: 30523 |
| | | AA | DIVMTQSPDSLAVSLGARATINCKSSQSVLHSSN NYNYLTWYQQKSGQPPKLLIYWASTRESGVPDR FSGSGSGTDFTLTISSLQAEDVAVYYCQQYYRTP WTFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAVSSGW YWFDPWGQGTLVTVSS |
| | | | SEQ ID NO: 26518 | SEQ ID NO: 30524 |
| iPS:435739 | 21-225_174G7 | NA | GCCATCCAGATGACCCAGTCTCCATCTTCCGT GTCTGCATCTGTAGGAGACAGAGTCACCATCA CTTGTCGGGCGAGTCAGGGTATTAGCAACTGG TTAGCCTGGTATCAGCAGAAACCAGGGAAAG CCCCTAACCTCCTGATCCATGCTGCATTCAGTT TGCAAGGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGACTAACAGTTTCCCGTGG ACGTTCGGCCAAGGGACCAAGGTGGAAATCA AA | GAGGTGCAGTTGTTGGAGTCTGGGGGAGGCTTGG TACAGCCTGGGGGGTCCCTGAGACTCTCCTGTGC AGCCTCTGGATTCACCTTTAGGAGCTCTGCCATG AGCTGGGTCCGCCAGGCTCCAGGGAAGGGGCTG GAGTGGGTCTCAGTTATTAGTGGTAGTGGTGGTA GCACATTCTACACAGACTCCGTGAAGGGCCGGTT CACCATCTCCAGAGACAATTCCAAGAACACGCTG TATCTGCAAATGAACAGCCTGAGAGCCGAGGAC ACGGCCGTATATTACTGTGCGAAATCGAATAGCA GTGGCTGGTTTGACTACTGGGGCCAGGGAACCCT GGTCACCGTCTCCTCA |
| | | | SEQ ID NO: 26519 | SEQ ID NO: 30525 |

FIGURE 50

| | | | | |
|---|---|---|---|---|
| | | AA | AIQMTQSPSSVSASVGDRVTITCRASQGISNWLA WYQQKPGKAPNLLIHAAFSLQGGVPSRFSGSGS GTDFTLTISSLQPEDFATYYCQQTNSFPWTFGQG TKVEIK | EVQLLESGGGLVQPGGSLRLSCAASGFTFRSSAMS WVRQAPGKGLEWVSVISGSGGSTFYTDSVKGRFTI SRDNSKNTLYLQMNSLRAEDTAVYYCAKSNSSGW FDYWGQGTLVTVSS |
| | | | SEQ ID NO: 26520 | SEQ ID NO: 30526 |
| iPS:435741 | 21-225_174G10 | NA | GACATCCAGATGACCCAGTCTCCATCCTCCCT GTCTGCATCTGTAGGAGACAGAGTCACCATCA CTTGCCGGGCAAGTCAGGGCATTAGAGATGAT TTAGGCTGGTATCAACAGAAACCAGGGAAAG TCCCTAAGCGCCTGATATATGCTGCATCCAGT TTGCAAAGTGGGGTCCCATCAAGGTTCAGCGG CAGTGGATCTGGGACAGAATTCACTCTCACAA TCAGCAGCCTACAGCCTGAAGATTTTGCAACT TATTACTGTCTTCAGCATCATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAATTGAACAGCCTGAGAGCCGAGG ACACGGCTGTGTATTACTGTGCGAGAGAAAGGTA TAGCAGTGGCTGGTACGACTACGGTATGGACGTC TGGGGCCAAGGGACCACGGTCACCGTCTCCTCA |
| | | | SEQ ID NO: 26521 | SEQ ID NO: 30527 |
| | | AA | DIQMTQSPSSLSASVGDRVTITCRASQGIRDDLG WYQQKPGKVPKRLIYAASSLQSGVPSRFSGSGS GTEFTLTISSLQPEDFATYYCLQHHSYPRTFGQG TKVEIK | QVQLVESGGGVVQPGRSLRLSCAASGFTFSDYVM HWVRQAPGKGLEWVAVIWYDGSNKYYADSVKGR FTISRDNSKNTLYLQLNSLRAEDTAVYYCARERYSS GWYDYGMDVWGQGTTVTVSS |
| | | | SEQ ID NO: 26522 | SEQ ID NO: 30528 |

FIGURE 50

| | | | | |
|---|---|---|---|---|
| iPS:435743 | 21-225_175G1 | NA | GACATCCAGATGACCCAGTCTCCATCCTCCCT GTCTGCATCGTAGGAGACAGAGTCACCATCA CTTGCCGGGCAAGTCAGGGCATTAGAACTGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACGA TCAGCAGCCTGCAGCCTGAAGATTTTGCAACT TATTACTGTCTACAGCATTATAGTTACCCGCTC ACTTTCGGCGGAGGGACCAAGGTGGAGATCA AG | CAGGTGCAACTGGTGGAGTCTGGGGGAGGCGTG GTCCAGCCTGGGGGGTCCCTGAGACTCTCCTGTG CAGCGTCTGGCTTCACCTTCAGTACCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATATCCAGAGACAATTCCAAGAACACGC TGTATGTGCAAATGAACAGCCTGAGAGCCGAGG ACACGGCTGTGTATTACTGTGCGAGAGATCCCTT ACGTGGATACAATGACCCGGTTATGGACTACTGG GGCCAGGGAACCCTGGTCACCGTCTCCTCA |
| | | | SEQ ID NO: 26523 | SEQ ID NO: 30529 |
| | | AA | DIQMTQSPSSLSASVGDRVTITCRASQGIRTDLG WYQQKPGKAPKRLIYTASSLQSGVPSRFSGSGSG TEFTLTISSLQPEDFATYYCLQHYSYPLTFGGGT KVEIK | QVQLVESGGGVVQPGGSLRLSCAASGFTFSTYGMH WVRQAPGKGLEWVAVIWYDGSNKYYADSVKGRF TISRDNSKNTLYVQMNSLRAEDTAVYYCARDPLRG YNDPVMDYWGQGTLVTVSS |
| | | | SEQ ID NO: 26524 | SEQ ID NO: 30530 |
| iPS:435745 | 21-225_175G3 | NA | GACATCCAGATGACCCAGTCTCCATCCTCACT GTCTGCATCGTAGGAGACAGAGTCACCATCA CTTGTCGGGCGAGTCAGGACATTAGCAATGAT TTAGCCTGGTTTCAGCAGAAACCAGGGAAAGC CCCTAAGTCCCTGATCTTTTCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAATCTGATAGTTACCCTCTCAC TTTCGGCGGAGGGACCAAGGTTGAGATCAAA | CAGGTACAGGTGGTGCAGTCTGGGGCTGAGGTG AAGAAGCCTGGGGCCTCAGTGAAGGTCTCCTGC AAGGCTTCTGGATACACCTTCACCGGCTACTTTA TGCACTGGGTGCGACAGGCCCCTGGACAAGGGC TTGAATGGATGGGATGGATCAAGCCTAAAAGTG GTGGCACAAACTGTGCACAGAGGTTTCAGGGCA GGGTCACCATGACCAGGGACACGTCCATCACCA CAGCCTACATGGAACTGAGCAGGCTGCGATCTG ACGACACGGCCGTGTATTATTGTGTGAGAGGGGG GACTACGGTGACTACGTGGGGGGGTCTTTGACTAC TGGGGCCAGGGAACCATGGTCACCGTCTCCTCA |
| | | | SEQ ID NO: 26525 | SEQ ID NO: 30531 |

FIGURE 50

| | | | | |
|---|---|---|---|---|
| | | AA | DIQMTQSPSSLSASVGDRVTITCRASQDISNDLA WFQQKPGKAPKSLIFSASSLQSGVPSKFSGSGSG TDFTLTISSLQPEDFATYYCQQSDSYPLTFGGGT KVEIK | QVQVVQSGAEVKKPGASVKVSCKASGYTFTGYFM HWVRQAPGQGLEWMGWIKPKSGGTNCAQRFQGR VTMTRDTSITTAYMELSRLRSDDTAVYYCVRGGTT VTTWGVFDYWGQGTMVTVSS |
| | | | SEQ ID NO: 26526 | SEQ ID NO: 30532 |
| IPS:435747 | 21-225_175C4 | NA | GACATCCAGATGACCCAGTCTCCATCCTCACT GTCTGCATCTGTAGGAGACAGAGTCACCATCA CTTGTCGGGCGAGTCAGGGCATTGGGAATTAT TTAGCCTGGTTTCAGCAGAAACCAGGGAAAGC CCCTAAGTCCCTGATCTATGCTGCATCCGGTTT GCAAAGTGGGTTCCCATCAAAATTCAGCGGCA GTGGATCTGGGACAGATTTCACTCTCACCATC AGCAGCCTACAGCCTGAAGATTTTGCAACTTA TTACTGCCAACAGTATTATAGTTACCCATTCAC TTTCGGCCCTGGGACCAAAGTGGATATCAAA | GAGGTGCAGCTGTTGGAGTCTGGGGGAGGCTTG GTGCAGCCTGGGGGGTCCCTGAGACTCTCCTGTG CAGCCTCTGCATTCACCTTTAGCAGCTATGTCAT GAGCTGGGTCCGCCAGGCTCCAGGGAAGGGGCT GGAGTGGGTCTCTGCTATTAGTGGTAGTGGTGAT AGAACATACTACGCAGACTCCGTGAAGGGCCGG TTCACCATCTCCAGAGACGATTCCAATACCACGC TGTATCTGCAAATGAACAGCCTGAGAGCCGAGG ACACGGCCGTATATTACTGTGCGAGAACAGCGG GCTTTGACTACTGGGGCCAGGGAACCCTGGTCAC CGTCTCCTCA |
| | | | SEQ ID NO: 26527 | SEQ ID NO: 30533 |
| | | AA | DIQMTQSPSSLSASVGDRVTITCRASQGIGNYLA WFQQKPGKAPKSLIYAASGLQSGFPSKFSGSGSG TDFTLTISSLQPEDFATYYCQQYYSYPFTFGPGT KVDIK | EVQLLESGGGLVQPGGSLRLSCAASAFTFSSYVMS WVRQAPGKGLEWVSAISGSGDRTYYADSVKGRFTI SRDDSNTTLYLQMNSLRAEDTAVYYCARTAGFDY WGQGTLVTVSS |
| | | | SEQ ID NO: 26528 | SEQ ID NO: 30534 |

FIGURE 50

| | | | | |
|---|---|---|---|---|
| iPS:435749 | 21-225_175C10 | NA | GACATCCAGATGACCCAGTCTCCATCTTCCGT GTCTGCATCTGTAGGAGACAGAGTCACCATCA CTTGTCGGGCGAGTCAGGGTATTACCGACTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AGTGGATCTGGGACAGATTTCACTCTCACCTT CAGCAGCCTGCAGCCTGACGATTTTGCAACTT ACTATTGTCAACAGACTAACAGTTTCCCGTGG ACGTTCGGCCAAGGGACCAAGGTGGAAATCA AA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCGTCTATTAGTGGTCGTGGTGGT AGCACGTTCTACGCAGACTCCGTGAAGGGCCGGT TCACCGTCTCCAGAGACAATTCCAAGAACACGCT GTATCTGCAAATGAACAGCCTGAGAGCCGAGGA CACGGCCGTATATTACTGTGCGAAATCGAATAGC AGTGGCTGGTTTGACTACTGGGGCCAGGGAACCC TGGTCACCGTCTCCTCA |
| | | | SEQ ID NO: 26529 | SEQ ID NO: 30535 |
| | | AA | DIQMTQSPSSVSASVGDRVTITCRASQGITDWLA WYQQKPGKAPKLLIYAASSLQSGVPSRFSGSGS GTDFTLTFSSLQPDDFATYYCQQTNSFPWTFGQ GTKVEIK | EVQLLESGGGLVQPGGSLRLSCAASGFTFSSYAMS WVRQAPGKGLEWVSSISGRGGSTFYADSVKGRFT VSRDNSKNTLYLQMNSLRAEDTAVYYCAKSNSSG WFDYWGQGTLVTVSS |
| | | | SEQ ID NO: 26530 | SEQ ID NO: 30536 |
| iPS:435751 | 21-225_175D10 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATAACAACTACTTAGCTTGGTACCA GCAGAAACCAGGACAGCCTCCTAACCTGCTCA TTTACTGGACATCTACCCGGGAATCCGGGGTC CCTGACCGATTCAGTGGCAGCGGGTCTGGGAC AAATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGTTACACCTTCACCAATTATGATC TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGTTTACATGGAGCTGAGCAGCCTGAGATCTGAG GACACGGCCGTGTATTACTGTGCGTATAGCAGTG GCTGGTACTACTTTGACTACTGGGGCCAGGGAAC CCTGGTCACCGTCTCCTCA |
| | | | SEQ ID NO: 26531 | SEQ ID NO: 30537 |

FIGURE 50

| | | | | |
|---|---|---|---|---|
| | | AA | DIVMTQSPDSLAVSLGERATINCKSSQSVLYSSN NNNYLAWYQQKPGQPPNLLIYWTSTRESGVPDR FSGSGSGTNFTLTISSLQAEDVAVYYCQQYYSTP PTFGQGTKVEIK | QVQLVQSGAEVKKPGASVKVSCKASGYTFTNYDL NWVRQATGQGLEWMGWMHPNSGNTGYAQKFQG RVTMTRNTSISTVYMELSSLRSEDTAVYYCAYSSG WYYFDYWGQGTLVTVSS |
| | | | SEQ ID NO: 26532 | SEQ ID NO: 30538 |
| iPS:435753 | 21-225_175G10 | NA | GACATCCAGATGACCCAGTCTCCATCCTCCCT GTCTGCATCTGTAGGAGACAGAGTCATCATCA CTTGCCGGGCAAGTCAGACCATTGGCAACTAT TTAAATTGGTATCAGCAGAAACCAGGGAGAG CCCCTAAGCTCCTGATCTATGCTGCATCCAGTT TGCACAGTGGGGTCCCATCAGGGTTCAGTGGC AGTGGATCTGGGACAGATTTCACTCTCACCAT CAGCAGTCTGCAACCTGAAGATTTTGCAACTT ACTTCTGTCAACAGAGTTACAGAACCCCTCAG TGGACGTTCGGCCAAGGGACCAAGGTGGAAA TCAAA | GAGGTGCAGTTGTTGGAGTCTGGGGGAGGCTTGG TACAGCCTGGGGGGGTCCCTGAGACTCTCCTGTGC AGCCTCTGGATTCACCTTTAGCAGCTATGCCATG AGCTGGGTCCGCCAGACTCCAGGGAAGGGGCTG GAGTGGGTCTCAATTATTAGTGGTAGTGGTGGTA ACACATACTACGCAGACTCCGTGAAGGGCCGGTT CACCATCTCCAGAGACAATTCCAAGAACACGCTG TATCTGCAAATGAACAGCCTGAGAGCCGAGGAC ACGGCCGTATATTACTGTGCGAAAAGGGATACCT GGAACGGTTGGGATGCTTTTGATATCTGGGGCCA AGGGACAATGGTCACCGTCTCTTTA |
| | | | SEQ ID NO: 26533 | SEQ ID NO: 30539 |
| | | AA | DIQMTQSPSSLSASVGDRVIITCRASQTIGNYLN WYQQKPGRAPKLLIYAASSLHSGVPSGFSGSGS GTDFTLTISSLQPEDFATYFCQQSYRTPQWTFGQ GTKVEIK | EVQLLESGGGLVQPGGSLRLSCAASGFTFSSYAMS WVRQTPGKGLEWVSIISGSGGNTYYADSVKGRFTI SRDNSKNTLYLQMNSLRAEDTAVYYCAKRDTWN GWDAFDIWGQGTMVTVSL |
| | | | SEQ ID NO: 26534 | SEQ ID NO: 30540 |

2205

FIGURE 50

| | | | | |
|---|---|---|---|---|
| iPS:435755 | 21-225_176H4 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAAGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TATACAGATTCCGTGGACGTTCGGCCAAGGGA CCAGGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT TATAAATACTATGCAGACTCCGTGAAGGGCCGAT TCACCATCTCCAGAGACAATTCCAAGAACACGCT GTATCTGCAAATGAGCAGCCTGAGAGCCGAGGA CACGGCTGTGTATTACTGTGCGAGAGATGCCCAC GATTTTTGGAGTGGTTACTTTGCCTACTGGGGCC AGGGAGCCCTGGTCACCGTCTCCTCA |
| | | | SEQ ID NO: 26535 | SEQ ID NO: 30541 |
| | | AA | DIVMTQTPLSLSVTPGQPASISCKSSQSLLHGDG KTYLYWYLQKPGQPPQLLIYEVSNRFSGVPDRFS GSGSGTDFTLKISRVEAEDVGIYYCMQSIQIPWT FGQGTRVEIK | QVQLVESGGGVVQPGRSLRLSCAASGFTFSSYGMH WVRQAPGKGLEWVAIIWYDGSYKYYADSVKGRFT ISRDNSKNTLYLQMSSLRAEDTAVYYCARDAHDF WSGYFAYWGQGALVTVSS |
| | | | SEQ ID NO: 26536 | SEQ ID NO: 30542 |
| iPS:435759 | 21-225_176E6 | NA | GACATTGTGATGACTCAGTCTCCACTCTCCCTG CCCGTCACCCCTGGAGAGCCGGCCTCCATCTC CTGCAGGTCTAGTCAGAGCCTCCTGCATAATA ATGGATACAAGTATTTGGATTGGTACCTGCAG AAGCCAGGGCAGTCTCCACAGCTCCTGATCTA TTTGGGTTCTAATCGGGCCTCCGGGGTCCCTG ACAGGTTCAGTGCCAGTGGATCAGGCACAGAT TTTACACTGAAAATCAGCAGAGTGGAGGCTGA GGATGTTGGGGTTTATTACTGCATGCAAGTTC TACAAACTCCGTGGACGTTCGGCCAAGGGACC AAGGTGGAAATCAAA | GAGGTGCAACTGGTGGAGTCTGGGGGAGGCTTG GTACAGCCTGGGGGGTCCCTGAGACTCTCCTGTG CAGCCTCAGGATTCACCTTCAGTGGCCATAGTAT GAACTGGGTCCGCCAGGCTCCAGGGAAGGGGCT GGAGTGGGTTGCATACATTAGCATTAGTGGTAGT ACCATAAAGTACGCAGACTCTGTGAAGGGCCGA TTCATCATCTCCAGAGACAATGCCAGGGATTCAC TGTATCTGCAAATGAACAGCCTGAGAGACGAGG ACACGGCTGTGTATTACTGTGCGAGAAGGGGGAT TACTGTGGTTCGGAATGAGGACGGTTTGGACGTC TGGGGCCAAGGGACCACGGTCACCGTCTCCTCA |
| | | | SEQ ID NO: 26537 | SEQ ID NO: 30543 |

FIGURE 50

| | | | | |
|---|---|---|---|---|
| | | AA | DIVMTQSPLSLPVTPGEPASISCRSSQSLLHNNGY KYLDWYLQKPGQSPQLLIYLGSNRASGVPDRFS ASGSGTDFTLKISRVEAEDVGVYYCMQVLQTPW TFGQGTKVEIK | EVQLVESGGGLVQPGGSLRLSCAASGFTFSGHSMN WVRQAPGKGLEWVAYISISGSTIKYADSVKGRFIIS RDNARDSLYLQMNSLRDEDTAVYYCARRGITVVR NEDGLDVWGQGTTVTVSS |
| | | | SEQ ID NO: 26538 | SEQ ID NO: 30544 |
| iPS:435761 | 21-225_176B11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTTTCAGCAGAAACCAGGGAAAGC CCCTAAGCGCCTGATCTATGCTGCATCCAGTTT GCAAAGTGGGGTCCCATCAAGGTTCAGCGGCA GTGGATCTGGGACAGAATTCACTCTCACACTC AGCAGCCTGCAGCCTGAAGATTTTGCAACTTA TTATTGTCTACAGCATTATAGTTACCCGCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGCCT GGAGTGGGTGTCAATTATATGGTATGATGGAACT AATAAATACTATGCAGACTCCGTGAAGGGCCGA TTCACCATCTCCAGAGACAATTCCAAGAACACGC TGTTTCTGCAACTGAACAGCCTGAGAGCCGAGGA CACGGCTGTCTATTACTGTGCGCGAGATCCCTTA CGTGGATACAATGACCCGGTTTTGGACTACTGGG GCCAGGGAACCCTGGTCACCGTCTCCTCA |
| | | | SEQ ID NO: 26539 | SEQ ID NO: 30545 |
| | | AA | DIQMTQSPSSLSASVGDRVTITCRASQGIRNDLG WFQQKPGKAPKRLIYAASSLQSGVPSRFSGSGSG TEFTLTLSSLQPEDFATYYCLQHYSYPLTFGGGT KVEIK | QVQLVESGGGVVQPGRSLRLSCAASGFTFSTYGMH WVRQAPGKGLEWVSIIWYDGTNKYYADSVKGRFT ISRDNSKNTLFLQLNSLRAEDTAVYYCARDPLRGY NDPVLDYWGQGTLVTVSS |
| | | | SEQ ID NO: 26540 | SEQ ID NO: 30546 |

FIGURE 50

| iPS:435763 | 21-225_176H12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAACCGCCTGATCTATGCTGCATCCAGTT TGCAAAGTGGGGTCCCATCACGGTTCAGCGGC AGTGGATCTGGGACAGAATTCACTCTCACAAT CAGCAGCCTGCAGCGTGAAGATTTTGCAACTT ATTACGGTCTACAGCATAATAGTTACCCTCGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGCCGACTCCGTGAAGGGCCGAT TCACCATCTCCAGAGACAATTCCAAGAACACGCT GTATCTGCAAATGAACAGCCTGAGAGCCGAGGA CACGGCTGTATATTACTGTGCGAGAGAAAAGTAT AGCAGCAACTGGTACGACTACGGTATGGACGTCT GGGGCCAAGGGACCACGGTCACCGTCTCCTCA |
| | | | SEQ ID NO: 26541 | SEQ ID NO: 30547 |
| | | AA | DIQMTQSPSSLSASVGDRVTITCRASQGIRNDLG WYQQKPGKAPNRLIYAASSLQSGVPSRFSGSGS GTEFTLTISSLQREDFATYYGLQHNSYPRSFGQG TKLEIK | QVQLVESGGGVVQPGRSLRLSCAASGFTFSDYVM HWVRQAPGKGLEWVAVIWYDGSNKYYADSVKGR FTISRDNSKNTLYLQMNSLRAEDTAVYYCAREKYS SNWYDYGMDVWGQGTTVTVSS |
| | | | SEQ ID NO: 26542 | SEQ ID NO: 30548 |
| iPS:435765 | 21-225_177D3 | NA | GACATCCAGATGTCCCAGTCTCCATCCTCACT GTCTGCTTCTGTAGGAGACAGAGTCACCATCA CTTGTCGGGCGAGTCAGGGCATTACCAATTAT TTAGCCTGGTTTCAGCAGAAACCAGGGAAAGC CCCTAAGTCCCTGATCTATGCTGCATCCAGTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA | GAGGTGCAGTTGTTGGAGTCTGGGGGAGGCTTGG TACAGCCTGGGGGGGTCCCTGAGACTCTCCTGTGC AGCCTCTGGATTCACCTTTAGCAGCTATGTCATG AACTGGGTCCGCCAGGCTCCAGGGAAGGGGCTG GAGTGGGTCTCAGGTATGAGCGGTAGTGGTGGTA GAACATACTACGCAGACTCCGTGAAGGACCGGT TCACCATCTCCAGAGACAATTCCAAGAACACGCT GTCTCTGCAAATGAACAGCCTGAGGGCCGAGGA CACGGCCGTATATTACTGTGCGAGAGTGACTTTC TTTGACTATTGGGGCCAGGGAACCCTGGTCACCG TCTCCTCA |
| | | | SEQ ID NO: 26543 | SEQ ID NO: 30549 |

FIGURE 50

| | | | | |
|---|---|---|---|---|
| | | AA | DIQMSQSPSSLSASVGDRVTITCRASQGITNYLA WFQQKPGKAPKSLIYAASSLQSGVPSKFSGSGSG TDFTLTISSLQPEDFATYYCQQYNSYPFTFGPGT KVDIK | EVQLLESGGGLVQPGGSLRLSCAASGFTFSSYVMN WVRQAPGKGLEWVSGMSGSGGRTYYADSVKDRF TISRDNSKNTLSLQMNSLRAEDTAVYYCARVTFFD YWGQGTLVTVSS |
| | | | SEQ ID NO: 26544 | SEQ ID NO: 30550 |
| iPS:435767 | 21-225_177B4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTTCCCTCGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGATTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCATAGTCTCCAGAGACAATTCCAAGAACACGC TGTATCTGCAAATGAACAGCCTGAGAGCCGAGG ACACGGCTGTGTATTACTGTGCGAGAGAAAAGTA TAGCAGCAGCTGGTACGACTACGGTTTGGACGTC TGGGGCCAAGGGACCACGGTCACCGTCTCCTCA |
| | | | SEQ ID NO: 26545 | SEQ ID NO: 30551 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSFPRSFGQGT KLEIK | QVQLVESGGGVVQPGRSLRLSCAASGFTFSDYVM HWVRQAPGKGLEWVAVIWYDGSNKYYADSVKGR FIVSRDNSKNTLYLQMNSLRAEDTAVYYCAREKYS SSWYDYGLDVWGQGTTVTVSS |
| | | | SEQ ID NO: 26546 | SEQ ID NO: 30552 |

FIGURE 50

| iPS:435769 | 21-225_177B6 | NA | GACATCCAGATGACCCAGTCTCCATCCTCCCT GTCTGCATCGTAGGAGACAGAGTCACCATCA CTTGCCGGGCAAGTCAGGACATTAGCAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGTTCTGGGACAGAATTCACTCTCACAA TCAACAGCCTGCAGCCTGAAGATTTTGCAACT TATTACTGTCTACAGCTTAATAGTTACCCATTC ACTTTCGGCCCTGGGACCAAAGTGGATATCAA A | GAGGTGCAGCTGTTGGAGTCTGGGGGAGGCTTG GTACAGCCGGGGGGGTCCCGGAGACTCTCCTGTG AAGCCTCTGGATTCACCTTTAGCAGTTATGCCAT GAGCTGGGTCCGCCAGGCTCCAGGGAAGGGGCT GGAGTGGGTCTCAGTTATTAGTGGTAGTGGTAGT AACACATACTACGTAGACTCCGTGAAGGGCCGG TTCACCATCTCCAGAGACAATTCCAAGAACACGC TGAATCTGCAAATGAACAGCCTGAGAGCCGAGG ACTCGGCCGTATATTACTGTACGAAAGGTTACTA TGATAGTAGTGGTTATTACTACCCTTTTGACTTCT GGGGCCAGGGAACCCTGGTCACCGTCTCCTCA |
|---|---|---|---|---|
| | | | SEQ ID NO: 26547 | SEQ ID NO: 30553 |
| | | AA | DIQMTQSPSSLSASVGDRVTITCRASQDISNDLG WYQQKPGKAPKRLIYAASSLQSGVPSRFSGSGS GTEFTLTINSLQPEDFATYYCLQLNSYPFTFGPGT KVDIK | EVQLLESGGGLVQPGGSRRLSCEASGFTFSSYAMS WVRQAPGKGLEWVSVISGSGSNTYYVDSVKGRFTI SRDNSKNTLNLQMNSLRAEDSAVYYCTKGYYDSS GYYYPFDFWGQGTLVTVSS |
| | | | SEQ ID NO: 26548 | SEQ ID NO: 30554 |
| iPS:435771 | 21-225_177B11 | NA | GATATTGTGATGACCCAGACTCCACTCTCTCT GTCCGTCACTCCTGGACAGCCGGCCTCCATCT CCTGCAAGTCTAGTCAGCGCCTCCTGCATGGT GATGGAAAGACCTATTTGTATTGGTACCTGCA GAAGCCAGGCCAGCCTCCACAGATCCTGATCT ATGAAGTTTCCAACCGGTTCTCTGGAGTGCCA GATAGGTTCAGTGGCAGCGGGTCAGGGACAG AGTTCACACTTAAAATCAGCCGGGTGGAGGCT GAGGATGTTGGGGTTTATTACTGCATGCAAAG TATACAGATTCCGTGGACGTTCGGCCAAGGGA CCAAGGTGGAAATCAAA | CAGGTGCAACTGGTGGAGTCTGGGGGAGGCGTG GTCCAGCCTGGGAGGTCCCTGAGACTCACCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT TATAAATACTATACAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAGACTTAC GATTTTTGGAGTGGTTATTTTGTCTTCTGGGGCCA GGGAACCCTGGTCACCGTCTCCTCA |
| | | | SEQ ID NO: 26549 | SEQ ID NO: 30555 |

FIGURE 50

| | | | | |
|---|---|---|---|---|
| | | AA | DIVMTQTPLSLSVTPGQPASISCKSSQRLLHGDG KTYLYWYLQKPGQPPQILIYEVSNRFSGVPDRFS GSGSGTEFTLKISRVEAEDVGVYYCMQSIQIPWT FGQGTKVEIK | QVQLVESGGGVVQPGRSLRLTCAASGFTFSSYGMH WVRQAPGKGLEWVAIIWYDGSYKYYTDSVKGRFT ISRDNSKNTLYLQMNSLRAEDTAVYYCARETYDF WSGYFVFWGQGTLVTVSS |
| | | | SEQ ID NO: 26550 | SEQ ID NO: 30556 |
| iPS:435773 | 21-225_177B12 | NA | GACATCGTGATGACCCAGTCTCCAGACTCCCT GGCTGTGTCTCTGGGCGAGAGGGCTACCGTCA ACTGCAAGTCCAGCCAGAGTGTTTTACACAGC TCCAACAATAACAACTACTTAACTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTAGTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGTATAGCAGT GGCTGGTACTACTTTGACTTCTGGGGGCCAGGGAA CCCTGGTCACCGTCTCCTCA |
| | | | SEQ ID NO: 26551 | SEQ ID NO: 30557 |
| | | AA | DIVMTQSPDSLAVSLGERATVNCKSSQSVLHSSN NNNYLTWYQQKPGQPPKLLIYWASTRESGVPDR FSGSGSGTDFTLTISSLQAEDVAVYYCQQYYSSP PTFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YYFDFWGQGTLVTVSS |
| | | | SEQ ID NO: 26552 | SEQ ID NO: 30558 |

FIGURE 50

| iPS:435775 | 21-225_178A5 | NA | GACATCCAGATGACCCAGTCTCCATCTTCCGT GTCTGCATCTGTAGGAGACAGAGTCACCATCA CTTGTCGGGCGAGTCAGGGTATTAGCAACTGG TTAGCCTGGTATCAGCAGAAACCAGGGAAAG CCCCTAAGCTCCTGATCTATGCTGCTTCCAGTT TGCAAAGTGGGGTCCCATCAAGGTTCAACGGC AGTGGATCTGGGACAGATTTCACTCTCACCAT CAGCAGCCTGCAGCCTGAAGATTTTGCAACTT ACTGTTGTCAACAGGCTAACAGTTTACCGTGG ACGTTCGGCCAAGGGACCAAGGTGGAAATCA AA | GAGGTGCACCTGTTGGAGTCTGGGGGAGGCTTGG TACAGACTGGGGGGTCCCTGAGACTCTCCTGTGC AGCCTCTGGATTCACCTTTAGCAGCTATGCCATG ACCTGGGTCCGCCAGGCTCCAGGGAAGGGGCTG GAGTGGGTCTCAGTTATTAGTGGTAGTGGTGGTA ATACATTCTACGCAGACTCCGTGAAGGGCCGGTT CACCATCTCCAGAGACAATTCCAAGAATACGCTG TATCTGCAAATGAACAGCCTGAGAGCCGAGGAC ACGGCCGTATATTACTGTGCGCGCCGGGACGGTG ACTACTTTGACTACTGGGGCCAGGGAACCCTGGT CACCGTCTCCTCA |
|---|---|---|---|---|
| | | | SEQ ID NO: 26553 | SEQ ID NO: 30559 |
| | | AA | DIQMTQSPSSVSASVGDRVTITCRASQGISNWLA WYQQKPGKAPKLLIYAASSLQSGVPSRFNGSGS GTDFTLTISSLQPEDFATYCCQQANSLPWTFGQG TKVEIK | EVHLLESGGGLVQTGGSLRLSCAASGFTFSSYAMT WVRQAPGKGLEWVSVISGSGGNTFYADSVKGRFTI SRDNSKNTLYLQMNSLRAEDTAVYYCARRDGDYF DYWGQGTLVTVSS |
| | | | SEQ ID NO: 26554 | SEQ ID NO: 30560 |
| iPS:435777 | 21-225_178F7 | NA | GACATCCAGATGACCCAGTCTCCATCTTCCGT GTCTGCATCTGTAGGAGACAGAGTCACCATCA CTTGTCGGGCGAGTCAGGATATTACTGACTGG TTAGCCTGGTATCAGCAGAAACCAGGGAAAG CCCCTAAACTCCTGATCTCTGCTGCATCCAGTT TGCAGAGTGGGGTCCCATCAAGGTTCAGCGGC AGTGGATCTGGGACAGATTTCACTCTCGCCAT CAGCAGCCTGCAGCCTGAAGATTTTGCAACTT ACTATTGTCAACAGGCTAACAGTTTACCGTGG ACGTTCGGCCAAGGGACCAAGGTGGAAATCA AA | GAGGTGCACCTGTTGGAGTCTGGGGGAGGCTTGG TACAGACTGGGGGGTCCCTGAGACTCTCCTGTGC AGCCTCTGGATTCACCTTTAGCAGCTATGCCATG ACCTGGGTCCGCCAGGCTCCAGGGAAGGGGCTG GAGTGGGTCTCAGTTATTAGTGGTAGTGGTGGTA ACACATTCTACGCAGACTCCGTGAAGGGCCGGTT CACCATCTCCAGAGACAATTCCAAGAACACGCTG TATCTGCAAATGAACAGCCTGAGAGCCGAGGAC ACGGCCGTATATTACTGTGCGCGCCGGTACGGTG ACTACTTTGACTACTGGGGCCAGGGAACCCTGGT CACCGTCTCCTCA |
| | | | SEQ ID NO: 26555 | SEQ ID NO: 30561 |

FIGURE 50

| | | | | |
|---|---|---|---|---|
| | | AA | DIQMTQSPSSVSASVGDRVTITCRASQDITDWLA WYQQKPGKAPKLLISAASSLQSGVPSRFSGSGSG TDFTLAISSLQPEDFATYYCQQANSLPWTFGQGT KVEIK | EVHLLESGGGLVQTGGSLRLSCAASGFTFSSYAMT WVRQAPGKGLEWVSVISGSGGNTFYADSVKGRFTI SRDNSKNTLYLQMNSLRAEDTAVYYCARRYGDYF DYWGQGTLVTVSS |
| | | | SEQ ID NO: 26556 | SEQ ID NO: 30562 |
| iPS:435779 | 21-225_178B10 | NA | GACATCCAGATGACCCAGTCTCCATCCTCCCT GTCTGCATCTGTCGGAGACAGAGTCACCATCA CTTGCCGGGCAAGTCAGGGCATTAGAAATGAT TTAGGCTGGTATCAGCAGAAACCAGGGAAAG CCCCTAAACACCTGATCTATGCTGCATCCAGT TTGCAAAATGGGGTCCCATCAAGGTTCAGCGG CAGTGGCTCTGGGACAGAATTCACTCTCACAA TCAGCAGCCTGCAGCCTGAAGATTTTGCCACT TATTACTGTCTACACCATTATAGTTTCCCGCTC ACTTTCGGCGGAGGGACCAAGGTGGAGATCA AA | CAGGTGCAGCTGGTGGAGTCGGGGGGAGGCGTG GTCCAGCCTGGGAGGTCCCTGAGACTCTCCTGTG TAGCGTCTGGATTCACCTCCAGTACCTATGGCAT GCACTGGGTCCGCCAGGCTCCAGGCAAGGGGCT GGAATGGATGGCAATTATATGGTATGATGGAACT AATAAATACTATGCAGACTCCGTGAAGGGCCGA TTCACCATCTCCAGAGACAATTCCAAGAACACGC TGTATCTGCAACTGAACAGCCTGAGAGCCGAGG ACACGGCTGTGTATTACTGTGCGCGAGATCCCTT ACGTGGATACAATGACCCGGTTATGGACTACTGG GGCCAGGGAACCCTGGTCACCGTCTCCTCA |
| | | | SEQ ID NO: 26557 | SEQ ID NO: 30563 |
| | | AA | DIQMTQSPSSLSASVGDRVTITCRASQGIRNDLG WYQQKPGKAPKHLIYAASSLQNGVPSRFSGSGS GTEFTLTISSLQPEDFATYYCLHHYSFPLTFGGGT KVEIK | QVQLVESGGGVVQPGRSLRLSCVASGFTSSTYGMH WVRQAPGKGLEWMAIIWYDGTNKYYADSVKGRF TISRDNSKNTLYLQLNSLRAEDTAVYYCARDPLRG YNDPVMDYWGQGTLVTVSS |
| | | | SEQ ID NO: 26558 | SEQ ID NO: 30564 |

FIGURE 50

| | | | | |
|---|---|---|---|---|
| iPS:435781 | 21-225_178G10 | NA | CATATTGTGATGACCCAGACTCCACTCTCTCTG TCCGTCACCCCTGGACAGCCGGCCTCCATCTC CTGCAAGTCTAGTCAGAGCCTCCTGCATGGTG ATGGAAAGACCTATTTGTATTGGTACCTGCAG AAGCCAGGCCAGCCTCCACAGCTCCTGATCTA TGAAGTTTCCAACCGGTTTTCTGGAGTGCCAG ACAGACTCAGTGGCGGCGGGTCAGGGACAGA TTTCACACTGAAAATCAGCCGGGTGGAGGCTG AGGATGTTGGCATTTATTACTGCATGCAAAGT ATACAGGTTCCGTGGACGTTCGGCCAAGGGAC CAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAGTTATATGGTATGATGGAAGT AATAAATACTACGCAGACTCCGTGAAGGGCCGA TTCACCATCTCCAGAGACAATTTCAAGAACACGC TGTATCTGCAAATGAACAGCCTGAGAGCCGAGG ACACGGCTGTTTATTACTGTGCGAGAGAACGGTA CGATTTTTGGAGTGGTCATTTTGACTACTGGGGC CAGGGAACCCTGGTCACCGTCTCCTCA |
| | | | SEQ ID NO: 26559 | SEQ ID NO: 30565 |
| | | AA | HIVMTQTPLSLSVTPGQPASISCKSSQSLLHGDG KTYLYWYLQKPGQPPQLLIYEVSNRFSGVPDRL SGGGSGTDFTLKISRVEAEDVGIYYCMQSIQVPW TFGQGTKVEIK | QVQLVESGGGVVQPGRSLRLSCAASGFTFSTYGMH WVRQAPGKGLEWVAVIWYDGSNKYYADSVKGRF TISRDNFKNTLYLQMNSLRAEDTAVYYCARERYDF WSGHFDYWGQGTLVTVSS |
| | | | SEQ ID NO: 26560 | SEQ ID NO: 30566 |
| iPS:435783 | 21-225_179G1 | NA | GACATCCAGATGACCCAGTCTCCATCTTCCGT GTCTGCATCTGTAGGAGACAGAGTCACCATCA CTTGTCGGGCGAGTCAGGATATTAGCGACTGG TTAGCCTGGTATCAGCAAAAATCAGGGAAAGC CCCTAAACTCCTGATCTCTGCTGCATCCAGTTT GCAAAGTGGGGTCCCATCAAGGTTCGGCGGCA GTGGATCTGGGACAGATTTCACTCTCACCATC AGCAGCCTGCAGCCTGAAGATTTTGCAACTTA CTATTGTCAACAGGCTAACAGTTTACCGTGGA CGTTCGGCCAAGGGACCAAGGTGGAAATCAA A | GAGGTGCACCTGTTGGAGTCGGGGGGAGGCTTG GTACAGACTGGGGGGTCCCTGAGACTCTCCTGTG CAGCCTCTGGATTCACCTTTAGCAGCTATGCCAT GACCTGGGTCCGCCAGGCTCCAGGGAAGGGGCT GGAGTGGGTCTCAGTTATTAGTGGTTTTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGCGCGCCGGTACGGT GACTACTTTGACTACTGGGGCCAGGGAACCCTGG TCACCGTCTCCTCA |
| | | | SEQ ID NO: 26561 | SEQ ID NO: 30567 |

FIGURE 50

| | | | | |
|---|---|---|---|---|
| | | AA | DIQMTQSPSSVSASVGDRVTITCRASQDISDWLA WYQQKSGKAPKLLISAASSLQSGVPSRFGGSGS GTDFTLTISSLQPEDFATYYCQQANSLPWTFGQG TKVEIK | EVHLLESGGGLVQTGGSLRLSCAASGFTFSSYAMT WVRQAPGKGLEWVSVISGFGGNTFYADSVKGRFTI SRDNSKNTLYLQMNSLRAEDTAVYYCARRYGDYF DYWGQGTLVTVSS |
| | | | SEQ ID NO: 26562 | SEQ ID NO: 30568 |
| iPS:435785 | 21-225_179C2 | NA | GATATTGTGATGACCCAGACTCCACTCTCTCT GTCCGTCACCCCTGGACAGCCGGCCTCCATCT CCTGCAAATCTAGTCAGAGCCTCCTGCATAGT GAGGGAAAGACCTACTTGTATTGGTACCTGCA GAAGCCAGGCCAGCCTCCACAGCTCCTGATCT ATGAGGTTTCCCACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGGTTCTCACTTTCGGCGGAGGGACCA AGGTGGAGATCAAA | CAGGTGCAGCTGGTGGAGTCGGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCACTTATATTTTATGATGGAAGT AATAAATACTATGCAGACTCCGTGAAGGGCCGA TTCACCATCTCCAGAGACAATTCCAAGAACACGC TGTTTCTGCAAATGAACAGCCTGAGAGCCGAGG ACACGGCTGTGTATTACTGTGCGAGACGGTATAG CGGCAGCTGGTCCGGTGGTATGGACGTCTGGGGC CAAGGGACCACGGTCACCGTCTCCTCA |
| | | | SEQ ID NO: 26563 | SEQ ID NO: 30569 |
| | | AA | DIVMTQTPLSLSVTPGQPASISCKSSQSLLHSEGK TYLYWYLQKPGQPPQLLIYEVSHRFSGVPDRFSG SGSGTDFTLKISRVEAEDVGVYYCMQSIQVLTFG GGTKVEIK | QVQLVESGGGVVQPGRSLRLSCAASGFTFSSYGMH WVRQAPGKGLEWVALIFYDGSNKYYADSVKGRFT ISRDNSKNTLFLQMNSLRAEDTAVYYCARRYSGSW SGGMDVWGQGTTVTVSS |
| | | | SEQ ID NO: 26564 | SEQ ID NO: 30570 |

FIGURE 50

| IPS:435787 | 21-225_180A3 | NA | GACATCCAGATGACCCAGTCTCCATCTTCCGT GTCTGCATCTGTAGGAGACAGAGTCACCATCA CTTGTCGGGCGAGTCAGGATATTACCAGCTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AGTGGATCTGGGACAGATTTCATTCTCACCAT CAGCAGCCTGCAGCCTGAAGATTTTGCATTTT ACCATTGTCAACAGGCTAACAGTATCCCATTC ACTTTCGGCCCTGGGACCAAAGTGGACATCAA C | GAGGTGCAGCTGTTGGAGTCTGGGGGAGGCTTG GAACAGCCTGGGGGGTCCCTGAGACTCTCCTGTG CAGCCTCTGGATTCACCTTTAGCAGCTTTGCCAT GAACTGGGTCCGCCAGGCTCCAGGGAAGGGGCT GGAGTGGGTCTCAGTTATTAGCGGTCGCGGTGGT AACACATTCTACGCAGACTCCGTGAAGGGCCGGT TCACCATCTCCAGAGACAATTCCAAGAACACGCT GTTTCTGCAAATGAACAGCCTGAGAGCCGAGGA CACGGCCGTATATTTCTGTGCGAAACGGACTGGG GATGATGTTTTTGATGTCTGGGGCCAAGGGACAA TGGTCACCGTCTCTTCA |
| | | | SEQ ID NO: 26565 | SEQ ID NO: 30571 |
| | | AA | DIQMTQSPSSVSASVGDRVTITCRASQDITSWLA WYQQKPGKAPKLLIYAASSLQSGVPSRFSGSGS GTDFILTISSLQPEDFAFYHCQQANSIPFTFGPGT KVDIN | EVQLLESGGGLEQPGGSLRLSCAASGFTFSSFAMN WVRQAPGKGLEWVSVISGRGGNTFYADSVKGRFTI SRDNSKNTLFLQMNSLRAEDTAVYFCAKRTGDDV FDVWGQGTMVTVSS |
| | | | SEQ ID NO: 26566 | SEQ ID NO: 30572 |
| IPS:435789 | 21-225_180C4 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAAGCCAGGCCAGCCTCCACAGCTCCTGATCT ATGCAACTTCCAACCGGTTCCCTGGAGTGTCA GATAGGTTCAGTGGCAGCGGGTCAGGTACAG ACTTCACACTGAAAATCAGCCGGGTGGAGGCT GAGGATGTTGGGGTTTATTACTGCATGCAAAG TATACAGGTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGCCTATGGCAT GCACTGGGTCCGCCAGGCTCCAGGCAAGGGGCT GGAGTGGGTGACAATTATTTGGTATGATGGAAGT TATAAATACTATGCAGACTCCGTGAAGGGCCGAT TCGCCATCTCCAGAGACAATTCCAAGAATACGCT GTATCTGCAAATGAACAGCCTGAGAGCCGAGGA CACGGCTGTGTATTACTGTGCGAGAACCGGTGTG GATCCCTGGGACTACTACAACGGAATGGACGTCT GGGGCCAAGGGACCACGGTCACCGTCTCCTCA |
| | | | SEQ ID NO: 26567 | SEQ ID NO: 30573 |

FIGURE 50

| | | | | |
|---|---|---|---|---|
| | | AA | DIVMTQTPLSLSVTPGQPASISCKSSQSLLHGDG KTYLYWYLQKPGQPPQLLIYATSNRFPGVSDRFS GSGSGTDFTLKISRVEAEDVGVYYCMQSIQVPW TFGQGTKVEIK | QVQLVESGGGVVQPGRSLRLSCAASGFTFSAYGM HWVRQAPGKGLEWVTIIWYDGSYKYYADSVKGRF AISRDNSKNTLYLQMNSLRAEDTAVYYCARTGVDP WDYYNGMDVWGQGTTVTVSS |
| | | | SEQ ID NO: 26568 | SEQ ID NO: 30574 |
| IPS:435791 | 21-225_180H7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATACTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCATCT TATTACTGTCTACAGCATAATAGTTACCCATTC ACTTTCGGCCCTGGGACCAAAGTGGATTTCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGACT GGAGTGGGTGGCAGTTATATGGTATGATGAAAAT AATAAACACTATGCAGACTCCGCGAAGGGCCGA TTCACCATCTCCAGAGACAATTCCAAGAACACGC TGTATCTGCAAATGAACAGCCTGAGAGTCGAAG ACACGGCTGTGTATTACTGTGCGAGAGAGGTTGG CTGGTCCGATGACTACTGGGGCCAGGGAACCCTG GTCACCGTCTCCTCA |
| | | | SEQ ID NO: 26569 | SEQ ID NO: 30575 |
| | | AA | DIQMTQSPSSLSASVGDRVTITCRASQGIRNDLG WYQQKPGKAPKRLIYTASSLQSGVPSRFSGSGSG TEFTLTISSLQPEDFASYYCLQHNSYPFTFGPGTK VDFK | QVQLVESGGGVVQPGRSLRLSCAASGFTFSDYGM HWVRQAPGKGLEWVAVIWYDENNKHYADSAKGR FTISRDNSKNTLYLQMNSLRVEDTAVYYCAREVG WSDDYWGQGTLVTVSS |
| | | | SEQ ID NO: 26570 | SEQ ID NO: 30576 |

FIGURE 50

| | | | | |
|---|---|---|---|---|
| iPS:435793 | 21-225_180F8 | NA | GACATCCAGATGACCCAGTCTCCATCCTCCCT GTCTGCATCTGTAGGAGACGGAGTCACCATCA CTTGCCGGGCAAGTCAGACCATTCTCAGCTAT TTAAATTGGTATCAGCAGAAACCAGGGAAAG CCCCTAAACTCCTGATCTATGGTGTATCCAGTT TACAAAGTGGGGTCCCATCAAGGTTCAGTGGC AGTGGATCTGGGACAGATTTCTCTCTCACCAT CAGCAGTCTGCAACCTGAAGATTTTGCAACTT ACTACTGTCAGCAGAGTTACAGTACCCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGTCAGTTATATGGTATGATGGAAGT GATAAATACTATGAAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCATCC CCGGTGGAGCTACGGAGACTACTGGGGCCAGGG AACCCTGGTCACCGTGTCCTCA |
| | | | SEQ ID NO: 26571 | SEQ ID NO: 30577 |
| | | AA | DIQMTQSPSSLSASVGDGVTITCRASQTILSYLN WYQQKPGKAPKLLIYGVSSLQSGVPSRFSGSGS GTDFSLTISSLQPEDFATYYCQQSYSTPFTFGPGT KVDIK | QVQLVESGGGVVQPGRSLRLSCAASGFTFSSYGMH WVRQAPGKGLEWVSVIWYDGSDKYYEDSVKGRF TISRDNSKNTLYLQMNSLRAEDTAVYYCARDHPR WSYGDYWGQGTLVTVSS |
| | | | SEQ ID NO: 26572 | SEQ ID NO: 30578 |
| iPS:435795 | 21-225_181C2 | NA | GAAATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAAGCCAGGCCAGCCTCCACAGCTCCTGATCC ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGGTTCCCTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG TAGCGTCTGGATTCACCTTCAGTAGCTATGGCAT GCACTGGGTCCGCCAGGCTCCAGGCAAGGGGCT GGAATGGGTGACAATTATATGGTATGATGGAAGT TATAAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGATCATTAC GATTTTTGGAGTGGGCACTTTGACTTCTGGGGCC AGGGAACCCTGGTCACCGTCTCCTCA |
| | | | SEQ ID NO: 26573 | SEQ ID NO: 30579 |

FIGURE 50

| | | | | |
|---|---|---|---|---|
| | | AA | EIVMTQTPLSLSVTPGQPASISCKSSQSLLHGDGK TYLYWYLQKPGQPPQLLIHEVSNRFSGVPDRFSG SGSGTDFTLKISRVEAEDVGVYYCMQSIQVPWT FGQGTKVEIK | QVQLVESGGGVVQPGRSLRLSCVASGFTFSSYGMH WVRQAPGKGLEWVTIIWYDGSYKYYADSVKGRFT ISRDNSKNTLYLQMNSLRAEDTAVYYCARDHYDF WSGHFDFWGQGTLVTVSS |
| | | | SEQ ID NO: 26574 | SEQ ID NO: 30580 |
| IPS:435797 | 21-225_181G2 | NA | GACATCCAGATGACCCAGTCTCCATCCTCACT GTCTGCATCTATAGGAGAGAGAGTCACCATCA CTTGTCGGGCGAGTCAGGGCATTAGCAATTAT TTAGCCTGGATTCAGCAGAAACCAGGGACAGC CCCTAAGTCCCTGATCTATGCTGCATCCAGTTT GCAAAGTGGGGTCTCATCAAGGTTCAGCGGCA GTGGATTTGGGACAGATTTCACTCTCACCATC AGTAGTCTGCAGCCTGAAGATTTTGCAACTTA TTACTGCCAACAGTATAATGGTTACCCATTCA CTTTCGGCCCTGGGACCAAAGTGGATATCAAA | CAGGTGCAGCTGGTGCAGTCTGGGGGCTGAAGTG AAGACGCCTGGGGGCCTCAGTGAAGGTCTCCTGCA GGGCTTCTGGATACACCTTCACCAGCTACAATAT GCACTGGGTGCGACAGGTCCCTGGACAAGGGCT TGAGTGGATGGGATGGATCAACCCTAACAATGGT GGCTCAAACTATACACAGAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGAGAAAGTTTG GGGACTGGGGCCAGGGAACCCTGGTCACCGTCT CCTCA |
| | | | SEQ ID NO: 26575 | SEQ ID NO: 30581 |
| | | AA | DIQMTQSPSSLSASIGERVTITCRASQGISNYLAW IQQKPGTAPKSLIYAASSLQSGVSSRFSGSGFGTD FTLTISSLQPEDFATYYCQQYNGYPFTFGPGTKV DIK | QVQLVQSGAEVKTPGASVKVSCRASGYTFTSYNM HWVRQVPGQGLEWMGWINPNNGGSNYTQKFQGR VTMTRDTSISTAYMELSRLRSDDTAVYYCARKFGD WGQGTLVTVSS |
| | | | SEQ ID NO: 26576 | SEQ ID NO: 30582 |

FIGURE 50

| iPS:435799 | 21-225_181G3 | NA | GACATCCAGATGACCCAGTCTCCATCCTCCCT GTCTGCATCGTAGGAGACAGAGTCAACATCA CTTGCCGGGCAAGTCACAGCATTAGCAACTAT TTAAATTGGTATCAGCAGAAAGCAGGGAAAG CCCCTAACCTCTTGATCTATACTACATTGAATT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTTCTCCTCCG TGGACGTTCGGCCAAGGGACCAAGGTGGAAA TCAAA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGTTGGGTCCGCCAGGCTCCAGGGAAGGGGCT GGAGTGGGTCTCAGTTATTAGTGGTAGTGGTGGT AACACATTCTACGGAGACTCCGTGAAGGGCCGG TTCACCATCTCCAGAGACAATTCCAAGAACACGC TGTATCTGCAAATGAACAGCCTGAGAGCCGAGG ACACGGCCATATATTACTGTGCGAAACGGGAGA CCTACGACTGGGGATCCGATGCTTTTGATATCTG GGGCCAAGGGACAATGGTCACCGTCTCTTCA |
|---|---|---|---|---|
| | | | SEQ ID NO: 26577 | SEQ ID NO: 30583 |
| | | AA | DIQMTQSPSSLSASVGDRVNITCRASHSISNYLN WYQQKAGKAPNLLIYTTLNLQSGVPSRFSGSGS GTDFTLTISSLQPEDFATYYCQQSYSSPPWTFGQ GTKVEIK | EVQLLESGGGLVQPGGSLRLSCAASGFTFSSYAMS WVRQAPGKGLEWVSVISGSGGNTFYGDSVKGRFTI SRDNSKNTLYLQMNSLRAEDTAIYYCAKRETYDW GSDAFDIWGQGTMVTVSS |
| | | | SEQ ID NO: 26578 | SEQ ID NO: 30584 |
| iPS:435801 | 21-225_181E5 | NA | GACATCGTGATGACCCAGTCTCCAGACTCCCT GGCTGTGTCTCTGGGCGAGAGGGGCCACCATCA ACTGCAAGTCCAGCCAGAGTGTTTTACACAGC TCCAACAATTACAACTACTTAACTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCATCAG TATTTTATTACTCCGTGGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGTAGTAG TGGCTGGTACATCTTTGACTACTGGGGCCAGGGA ACCCTGGTCACCGTCTCCTCA |
| | | | SEQ ID NO: 26579 | SEQ ID NO: 30585 |

FIGURE 50

| | | | | |
|---|---|---|---|---|
| | | AA | DIVMTQSPDSLAVSLGERATINCKSSQSVLHSSN NYNYLTWYQQKPGQPPKLLIYWASTRESGVPDR FSGSGSGTDFTLTISSLQAEDVAVYYCHQYFITP WTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCASSSGW YIFDYWGQGTLVTVSS |
| | | | SEQ ID NO: 26580 | SEQ ID NO: 30586 |
| IPS:435805 | 21-225_181A8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGAAAAG CCCCTAAGCGCCTGATCTATACTGCATCCAGT TTGCAAAGTGGGGTCCCATCAAGGTTCAGCGG CAGTGGATCTGGGACAGATTTCACTCTCACAA TCAGCAGCCTGCAGCCTGAAGATTTTGCATCT TATTACTGTCTACAGCATAATAGTTACCCATTC ACTTTCGGCCCTGGGACCAAAGTGGATTTCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGACT GGAGTGGGTGGCAGTTATATGGTATGATGAAAAT AATAAACACTATGCAGACTCCGCGAAGGGCCGA TTCACCATCTCCAGAGACAATTCCAAGAACACGC TGTATCTGCAAATGAACAGCCTGAGAGCCGAAG ACACGGCTGTGTATTACTGTGCGAGAGAGGTTGG CTGGTCCGATGACTACTGGGGGCCAGGGAACCCTG GTCATCGTCTCCTCA |
| | | | SEQ ID NO: 26581 | SEQ ID NO: 30587 |
| | | AA | DIQMTQSPSSLSASVGDRVTITCRASQGIRNDLG WYQQKPGKAPKRLIYTASSLQSGVPSRFSGSGSG TDFTLTISSLQPEDFASYYCLQHNSYPFTFGPGTK VDFK | QVQLVESGGGVVQPGRSLRLSCAASGFTFSDYGM HWVRQAPGKGLEWVAVIWYDENNKHYADSAKGR FTISRDNSKNTLYLQMNSLRAEDTAVYYCAREVG WSDDYWGQGTLVIVSS |
| | | | SEQ ID NO: 26582 | SEQ ID NO: 30588 |

FIGURE 50

| iPS:435807 | 21-225_181C10 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAAGCCAGGCCAGCCTCCACAGCTCCTGATCT ATGAAGTTTCCAATCGGTTCTCTGGAGTGCCA GATAGGTTCAGTGGCAGCGGGTCAGGGACAG ATTTCACACTTAAAATCAGCCGGGTGGAGGCT GAGGATGTTGGGGTTTATCACTGCATGCAAAG TATACAGATTCCCTGGACGTTCGGCCAAGGGA CCAAGGTGGAAATCAAA | CAGGTGCAACTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGATGGCAATTATATGGTATGATGGAAGT TATAAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGATCATTAC GATTTTTGGAGTGGGCACTTTGACTACTGGGGCC AGGGAACCCTGGTCACCGTCTCCTCA |
| | | | SEQ ID NO: 26583 | SEQ ID NO: 30589 |
| | | AA | DIVMTQTPLSLSVTPGQPASISCKSSQSLLHGDG KTYLYWYLQKPGQPPQLLIYEVSNRFSGVPDRFS GSGSGTDFTLKISRVEAEDVGVYHCMQSIQIPWT FGQGTKVEIK | QVQLVESGGGVVQPGRSLRLSCAASGFTFSSYGMH WVRQAPGKGLEWMAIIWYDGSYKYYADSVKGRF TISRDNSKNTLYLQMNSLRAEDTAVYYCARDHYDF WSGHFDYWGQGTLVTVSS |
| | | | SEQ ID NO: 26584 | SEQ ID NO: 30590 |
| iPS:435809 | 21-225_182H5 | NA | GACATCCAGATGACCCAGTCTCCATCTTCCGT GTATGCATCTGTTGGAGACAGAGTCACCATCA CTTGTCGGGCGAGTCAGGATATTACCAGCTGG TTAGCCTGGTATCAGCAGAAACCAGGGAAAG CCCCTAAACTCCTGATCTATGCTGCATCCAGTT TACAAAGTGGGGTCCCATCAAGGTTCAGCGGC AGTGGATCTGGGACAGATTTCATTCTCACCAT CACCAGCGTGCAGCCTGATGATTTTGCAACTT ACTATTGTCAACAGGTTAACAGTTTCCCATTC ACTTTCGGCCACGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGTTGGGTCCGCCAGGCTCCAGGGAAGGGGCT GGAGTGGGTCTCAGTTATTAGTGGTAGAGGTGGT ACCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAACGGACTGGG GATGATGTTTTTGATATCTGGGGCCAAGGGACAA TGGTCACCGTCTCTTCA |
| | | | SEQ ID NO: 26585 | SEQ ID NO: 30591 |

FIGURE 50

| | | | | |
|---|---|---|---|---|
| | | AA | DIQMTQSPSSVYASVGDRVTITCRASQDITSWLA WYQQKPGKAPKLLIYAASSLQSGVPSRFSGSGS GTDFILTITSVQPDDFATYYCQQVNSFPFTFGHG TKVDIK | EVQLLESGGGLVQPGGSLRLSCAASGFTFSSYAMS WVRQAPGKGLEWVSVISGRGGTTFYADSVKGRFTI SRDNSKNTLYLQMNSLRAEDTAVYYCAKRTGDDV FDIWGQGTMVTVSS |
| | | | SEQ ID NO: 26586 | SEQ ID NO: 30592 |
| iPS:435811 | 21-225_183H6 | NA | GACATCCAGATGACCCAGTCTCCATCCTCCCT GTCTGCATCTGTAGGAGACAGAGTCACCATCG CTTGCCAGGCGAGTCAGGACATTAGCAACTAT TTAAAATTGGTATCAGCAGACACCAGGGAAAGC CCCTAAGGTCCTGATCTACGATGCATCCAATT TGGAAACAGGGGTCCCAGCAAGGTTCAGTGG AAGTGGATCTGGGACAGATTTTACTTTCACCA TCAGCAGCCTGCAGCCTGAAGATATTGCAACA TATTACTGTCAACAGTATGATAATCTCCCTCTC ACTTTCGGCGGAGGGACCAAGGTGGACATCA AA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAATTATATCATATGCTGGAAGT ACTAAATTCTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGTGAGAAGGCCCCCG CAGTGGCTGGTAGAGGGCTACGGTATGGACGTCT GGGGCCAAGGGACCACGGTCACCGTCTCCTCA |
| | | | SEQ ID NO: 26587 | SEQ ID NO: 30593 |
| | | AA | DIQMTQSPSSLSASVGDRVTIACQASQDISNYLN WYQQTPGKAPKVLIYDASNLETGVPARFSGSGS GTDFTFTISSLQPEDIATYYCQQYDNLPLTFGGG TKVDIK | QVQLVESGGGVVQPGRSLRLSCAASGFTFSSYGMH WVRQAPGKGLEWVAIISYAGSTKFYADSVKGRFTI SRDNSKNTLYLQMNSLRAEDTAVYYCVRRPPQWL VEGYGMDVWGQGTTVTVSS |
| | | | SEQ ID NO: 26588 | SEQ ID NO: 30594 |

FIGURE 50

| iPS:435813 | 21-225_183A12 | NA | GACATCCAGATGACCCAGTCTCCATCCTCCCT GTGTGCCTCTGTAGGAGACAGAGTCACCATCA CTTGCCGGGCAAGTCGGAACATCAGCAACTAT TTAAAATTGGTATCAGCAGAAACCAGGGAAAG CCCCTAAGCTCCTGATCTATGTTGTATCCAGTT TGCAAAGTGGGGTCCCATCAAGGTTCAGTGGC AGTGGATCTGGGACAGATTTCACTCTCACCAT CAGCAGTCTACAACCTGAAGATTTTGCAACTT ACTACTGTCAACAGAGTTACAGTTCCCCTCCG TGGACGTTCGGCCAAGGGACCAAGGTGGATAT CAGA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATCTGCTGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTATTGTGCGAGAAGGTATAG CAGTGGCTGGGACTGGTTCGACCCCTGGGGCCAG GGAACCCTGGTCACCGTCTCCTCA |
| | | | SEQ ID NO: 26589 | SEQ ID NO: 30595 |
| | | AA | DIQMTQSPSSLCASVGDRVTITCRASRNISNYLN WYQQKPGKAPKLLIYVVSSLQSGVPSRFSGSGS GTDFTLTISSLQPEDFATYYCQQSYSSPPWTFGQ GTKVDIR | QVQLVESGGGVVQPGRSLRLSCAASGFTFSSYGMH WVRQAPGKGLEWVAVISSAGSNKYYADSVKGRFT ISRDNSKNTLYLQMNSLRAEDTAVYYCARRYSSG WDWFDPWGQGTLVTVSS |
| | | | SEQ ID NO: 26590 | SEQ ID NO: 30596 |
| iPS:435815 | 21-225_190G10 | NA | GAAATTGTGTTGACGCAGTCTCCAGGCACCCT GTCTTTGTCTCCAGGGGAAAGAGCCACCCTCT CCTGCAGGGCCAGTCCGAGTGTTAGCAGCAGA TTCTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CAACAGCAGACTGGAGCCTGAAGATTTTGCAG TGTATTACTGTCAGCAGTATGGTAGCTCACCT CCGTGGACGTTCGGCCAAGGGACCAAGGTAG AAAATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGGGACTATAGCAT GAACTGGGTCCGCCAGGCTCCAGGGAAGGGGCT GGAGTGGGTCTCATCTATTAGTAGTGGTAGTGGT TACATACACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGCGAGCAACTAT GGCCCTTGACTACTGGGGCCAGGGAACCCTGGTC ACCGTCTCCTCA |
| | | | SEQ ID NO: 26591 | SEQ ID NO: 30597 |

FIGURE 50

| | | | | |
|---|---|---|---|---|
| | | AA | EIVLTQSPGTLSLSPGERATLSCRASPSVSSRFLA WYQQKPGQAPRLLIYGASSRATGIPDRFSGSGSG TDFTLTNSRLEPEDFAVYYCQQYGSSPPWTFGQ GTKVEIK | EVQLVESGGGLVKPGGSLRLSCAASGFTFRDYSMN WVRQAPGKGLEWVSSISSGSGYIHYADSVKGRFTIS RDNAKNSLYLQMNSLRAEDTAVYYCARATMALD YWGQGTLVTVSS |
| | | | SEQ ID NO: 26592 | SEQ ID NO: 30598 |
| iPS:435817 | 21-225_190B11 | NA | GAAATTGTGCTGACTCAGTCTCCAGACTTTCA GTCTGTGGCTCCAAAGGAGAAAGTCACCATCA CCTGCCGGGCCAGTCAGAGTATTGGTAGTAAC TTACACTGGTACCAGCAGAAACCAGATCAGTC TCCGAAACTCCTCATCAAGTCTGCTTCCCAGTC CTTCTCAGGGGTCCCCTCGAGGTTCAGTGGCA GTGGATCTGGGACAGATTTCACCCTCACCATC AATAGCCTGGAAACTGAAGATGCTGCAACGTA TTACTGTCAGCAGAGTAGTAGTTTACCGTGGA CGTTCGGCCAAGGGACCAAGGTGGAAATCAA A | CAGGTGCAGCTGCAGGAGTCGGGCCCAGGACTG GTGAAGCCTTCGGAGACCCTGTCCCTCACCTGCA CTGTCTCTGGTGGCTCCATCAATAATTACTACTG GAGCTGGATCCGGCAGCCCGCCGGGAAGGGACT GGAGTGGATTGGGCGTATCTATACCAGTGGGAGC ACCAACTACAACCCCTCCCTCAAGAGTCGAGTCA CCATGTCAGTAGACACGTCCAAGAACCAGTTCTC CCTGAAGCTGAGCTCTGTGACCGCCGCGGACACG GCCGTGCATTACTGTGCGAGAGATCGGGGGATACT ATGGCTACTACGGTATGGACGTCTGGGGCCAAG GGACCACGGTCACCGTCTCCTCA |
| | | | SEQ ID NO: 26593 | SEQ ID NO: 30599 |
| | | AA | EIVLTQSPDFQSVAPKEKVTITCRASQSIGSNLHW YQQKPDQSPKLLIKSASQSFSGVPSRFSGSGSGT DFTLTINSLETEDAATYYCQQSSSLPWTFGQGTK VEIK | QVQLQESGPGLVKPSETLSLTCTVSGGSINNYYWS WIRQPAGKGLEWIGRIYTSGSTNYNPSLKSRVTMS VDTSKNQFSLKLSSVTAADTAVHYCARDRGYYGY YGMDVWGQGTTVTVSS |
| | | | SEQ ID NO: 26594 | SEQ ID NO: 30600 |

FIGURE 50

| iPS:435819 | 21-225_190C11 | NA | GACATCCAGATGACCCAGTCTCCTTCCTCACT GTCTGCATCTGTAGGAGACAGAGTCACCATCA CTTGTCGGACGAGTCAGGGCATTGGCAATTAT TTAGCCTGGTTTCAGCAGAAACCAGGGAAAGC CCCTAAGTCCCTGCTCTATAAAACATCCAGTTT ACAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATATGACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGCCAA GGGACCACGGTCACCGTCTCCTCA |
| | | | SEQ ID NO: 26595 | SEQ ID NO: 30601 |
| | | AA | DIQMTQSPSSLSASVGDRVTITCRTSQGIGNYLA WFQQKPGKAPKSLLYKTSSLQSGVPSKFSGSGS GTDFTLTISSLQPEDFATYYCQQYMTYPLTFGGG TKVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDQGV GYDGLDVWGQGTTVTVSS |
| | | | SEQ ID NO: 26596 | SEQ ID NO: 30602 |
| iPS:435821 | 21-225_190E11 | NA | GAAATAGTGATGACGCAGTCTCCAGCCACCCT GTCTGTGTCTCCAGGGGAAAGAGCCACCCTCT CCTGCAGGGCCAGTCAGAGTTTTCGCATCAAC TTAGCCTGGTACCAGCAGAGACCTGGCCAGGC TCCCAGGCTCCTCATCTATGGTGCATCCACCA GGGCCACTGGTATCCCAGCCAGGTTCAGTGGC AGTGGGTCTGGGACAGAGTTCACTCTCACCAT CAGCAGCCTGCAGTCTGAAGATTTTGCAGTTT ATTACTGTCAGCAGTATAATAACTGGCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTA CAGCGTCTGGATTCACCTTCAGTAACTATGGCAT GCACTGGGTCCGCCAGGCTCCAGGCAAGGGGCT GGAGTGGGTGGCAATTATATGGTTTGATGGAAGT AATAAATACTATGCAGACTCCGTGAAGGGCCGA TTCACCATCTCCAGAGACAATTCCAATAACACGC TGTATCTGCAAATGAACAGCCTGCGAGCCGAGG ACACGGCTGTGTATTACTGTGCGAAAGCCCAGGG GGTCTACTACTACGTTATGGACGTCTGGGGCCAA GGGACCACGGTCACCGTCTCCTCA |
| | | | SEQ ID NO: 26597 | SEQ ID NO: 30603 |

FIGURE 50

| | | | | |
|---|---|---|---|---|
| | | AA | EIVMTQSPATLSVSPGERATLSCRASQSFRINLA WYQQRPGQAPRLLIYGASTRATGIPARFSGSGSG TEFTLTISSLQSEDFAVYYCQQYNNWPLTFGGGT KVEIK | QVQLVESGGGVVQPGRSLRLSCTASGFTFSNYGMH WVRQAPGKGLEWVAIIWFDGSNKYYADSVKGRFT ISRDNSNNTLYLQMNSLRAEDTAVYYCAKAQGVY YYVMDVWGQGTTVTVSS |
| | | | SEQ ID NO: 26598 | SEQ ID NO: 30604 |
| iPS:435823 | 21-225_190F11 | NA | GAAATTGTGCTGACTCAGTTTCCAGACTCTCA GTCTGTGACTCCAAAGGAGAAAGTCACCATCA CCTGCCGGGCCAGTCAGAACATTGGTAGTAGC TTACACTGGTACCAGCAGAAACCAGAACAGTC TCCAAAGGTCCTCATCAAGTATGCTTCCCAGT CCTTCTCAGGGGTCCCCTCGAGGTTCAGTGGC AGTGGATCTGGGACAGATTTCACCCTCACCAT CAATAGCCTGGAAGCTGAAGATGCTGCAACGT ATTACTGTCATCAGAGTAGTAGTTTCCCTCGG ACGTTCGGCCAAGGGACCAAGGTGGAAATCA AA | GAGGTGCAGCTGTTGGACTCTGGGGGAGGCTTGG GACAGCCTGGGGGGTCCCTGAGACTCTCCTGTGC AGCCTCTGGATTCACCTTTGACAGCTATGCCATG AACTGGGTCCGCCAGGCTCCAGGGAAGGGGCTG GAGTGGGTCTCAACTATTAGTGGTACTGGTCGTA GGACATACTACGCAGACTCCGTGAAGGGCCGGT TCACCATCTCCAGAGACAATTCCAAGAACACGCT GTATCTGCAAATGAATAGCCTGAGAGCCGAGGA CACGGCCGTATATTACTGTGCGAAAGAGGAGGA TTACTATGATAGTAGTGGCCCCGGGGGTTCGACCCC TGGGGCCAGGGAACCCTGGTCACCGTCTCCTCA |
| | | | SEQ ID NO: 26599 | SEQ ID NO: 30605 |
| | | AA | EIVLTQFPDSQSVTPKEKVTITCRASQNIGSSLHW YQQKPEQSPKVLIKYASQSFSGVPSRFSGSGSGT DFTLTINSLEAEDAATYYCHQSSSFPRTFGQGTK VEIK | EVQLLDSGGGLGQPGGSLRLSCAASGFTFDSYAMN WVRQAPGKGLEWVSTISGTGRRTYYADSVKGRFTI SRDNSKNTLYLQMNSLRAEDTAVYYCAKEEDYYD SSGPGFDPWGQGTLVTVSS |
| | | | SEQ ID NO: 26600 | SEQ ID NO: 30606 |

FIGURE 50

| | | | | |
|---|---|---|---|---|
| iPS:435825 | 21-225_190G11 | NA | GACATCCAGATGACCCAGTCTCCTTCCTCACT GTCTGCATCTGTAGGAGACAGAGTCACCATCA CTTGTCGGACGAGTCAGGGCATTGGCAATTAT TTAGCCTGGTTTCAGCAGAAACCAGGGAAAGC CCCTAAGTCCCTGCTCTATAAAGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATATGACTTACCCGCTCA CTTTCGGCGGAGGGACCAAGGTGGAGATCAA A<br><br>SEQ ID NO: 26601 | CAGGTGCAGCTGGTGGAGTCTGGGGGAGGCGTG GTCCAGCCTGGGAGGTCCCTGAGACTCTCCTGTG CAGCGTCTGGATTCACCTTCAGTATTTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGCCAA GGGACCACGGTCACCGTCTCCTCA<br><br>SEQ ID NO: 30607 |
| | | AA | DIQMTQSPSSLSASVGDRVTITCRTSQGIGNYLA WFQQKPGKAPKSLLYKASSLQSGVPSKFSGSGS GTDFTLTISSLQPEDFATYYCQQYMTYPLTFGGG TKVEIK<br><br>SEQ ID NO: 26602 | QVQLVESGGGVVQPGRSLRLSCAASGFTFSIYGMH WVRQAPGKGLEWVAVIWYDGSNKNYADSVKGRF TISRDNSKNTLYLQMNSLRAEDTAVYYCARDQGV GYDGLDVWGQGTTVTVSS<br><br>SEQ ID NO: 30608 |
| iPS:435827 | 21-225_190H11 | NA | GATATTGTGATGACCCAGACTCCACTCTCTCT GTCCGTCACCCCTGGACAGCCGGCCTCCATCT CCTGCAAGTCTAGTCAGAGCCTCCTCCATAGT GATGGAAGGACCTATTTGTATTGGTACCTGCA GAAGCCAGGCCAGCCTCCACAGCTCCTGATCT GTGAGGTTTCCAACCGGTTCGCTGGAGTGACA GATAGGTTCAGTGGCAGCGGGTCAGGGACAG ATTTCACACTGAAAATCAGCCGGGTGGAGGCT GGGGATGTTGGGGTTTATTACTGCATGCAAAG TATACAGTTTCCCTGGACGTTCGGCCAAGGGA CCAAGGTGGAAATCAAA<br><br>SEQ ID NO: 26603 | CAGCTGCAGCTGCAGGAGTCGGGCCCAGGACTG GTGAAGCCTTCGGAGACCCTGTCCCTCACCTGCA CTGTCTCTGGTGGCTCCATCAGAAGCTACCACTG GAGCTGGATCCGGCAGCCCGCCGGGAAGGGACT GGAGTGGATTGGGCTTATCTATACCAGTAGGAGC ACCATTTACAACCCCTCCCTCAAGAGTCGAGTCA CCCTGTCAGTAGACACGTCCAAGAACCAGTTCTC CCTGAAGCTGAGCTCTGTGACCGCCGCGGACACG GCCGTGTATTACTGTGCGAGACTCCGGTATAACT GGAACTTCCCTTACTTTGACTACTGGGGCCAGGG AACCCTGGTCACCGTCTCCTCA<br><br>SEQ ID NO: 30609 |

FIGURE 50

| | | | | |
|---|---|---|---|---|
| | | AA | DIVMTQTPLSLSVTPGQPASISCKSSQSLLHSDGR TYLYWYLQKPGQPPQLLICEVSNRFAGVTDRFS GSGSGTDFTLKISRVEAGDVGVYYCMQSIQFPW TFGQGTKVEIK | QLQLQESGPGLVKPSETLSLTCTVSGGSIRSYHWSW IRQPAGKGLEWIGLIYTSRSTIYNPSLKSRVTLSVDT SKNQFSLKLSSVTAADTAVYYCARLRYNWNFPYF DYWGQGTLVTVSS |
| | | | SEQ ID NO: 26604 | SEQ ID NO: 30610 |
| iPS:435829 | 21-225_190B12 | NA | GAAATTGTGCTGACTCAGTCTCCAGACTTTCA GTCTGTGACTCCAAAGGAGAAAGTCACCATCA CCTGCCGGGCCAGTCAGAGTATTGGTAGTAGC TTACACTGGTACCAGCAGAAACCAGATCAGTC TCCAAAGCTCCTCATCAAGTATGCTTCCCAGT CCTTCTCAGGGGACCCCTCGAGGTTCAGTGGC AGTGGATCTGGGACAGATTTCACCCTCACCAT CAATAGCCTGGAAGCTGAAGATGCTGCAACGT ATTACTGTCATCAGACTAGAAGTTTACCTCTC ACTTTCGGCGGAGGGACCAAGGTGGAGATCA AA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAACTGGATCCGCCAGCACCCAGGGAA GGGCCTGGAGTGGATTGGGTATATCTATTACAGT GGGAGCACCTACTACAACCCGTCCCTCAAGAGTC GAGTTACCATATCAGTAGACACGTCTAAGAACCA GTTTTTTCTGAAGCTGAACTCTGTGACTGCCGCG GACACGGCCGTGTATTACTGTGCGAGATCCGGGT ATAATTGGGACGCCGGGGGTCGACCCCTGGGGCC GGGGAACCCTGGTCACCGTCTCCTCA |
| | | | SEQ ID NO: 26605 | SEQ ID NO: 30611 |
| | | AA | EIVLTQSPDFQSVTPKEKVTITCRASQSIGSSLHW YQQKPDQSPKLLIKYASQSFSGDPSRFSGSGSGT DFTLTINSLEAEDAATYYCHQTRSLPLTFGGGTK VEIK | QVQLQESGPGLVKPSQTLSLTCTVSGGSISSGGYYW NWIRQHPGKGLEWIGYIYYSGSTYYNPSLKSRVTIS VDTSKNQFFLKLNSVTAADTAVYYCARSGYNWDA GVDPWGRGTLVTVSS |
| | | | SEQ ID NO: 26606 | SEQ ID NO: 30612 |

FIGURE 50

| iPS:435831 | 21-225_190C12 | NA | GACATCCAGATGACCCAGTCTCCATCCTCCCT GTCTGCATCTGTAGGAGACAGAGTCACCATCA CTTGCCGGGCAAGTCAGGGCATTAGAAAAGAT TTAGGCTGGTATCAGCAGAAACCAGGGAAAG CCCCTAAGCGCCTGATCCATACTGCATCCAGT TTGCAAAGTGGGGTCCCATTAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGCCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGATGGAGGT TATAAAAACTATGTAGACTCCGTGAAGGGCCGAT TCACCATCTCCAGAGACAATTCCAAGAACACGCT GTATCTGCAAATGAATAGCCTGAGAGCTGAGGA CACGGCTGTGTATTACTGTGCGAGAGGTACCCAC GGGTACTACTACGGTGTGGACGTCTGGGGCCAA GGGACCACGGTCACCGTCTCCTCA |
|  |  |  | SEQ ID NO: 26607 | SEQ ID NO: 30613 |
|  |  | AA | DIQMTQSPSSLSASVGDRVTITCRASQGIRKDLG WYQQKPGKAPKRLIHTASSLQSGVPLRFSGSGS GTEFTLTISSLQPEDFATYYCLQHNSYPWTFGQG TKVEIK | QVQLVESGGAVVQPGRSLRLSCAASGFTFSSYGMH WVRQAPGKGLEWVAVISYDGGYKNYVDSVKGRF TISRDNSKNTLYLQMNSLRAEDTAVYYCARGTHG YYYGVDVWGQGTTVTVSS |
|  |  |  | SEQ ID NO: 26608 | SEQ ID NO: 30614 |
| iPS:435833 | 21-225_190D12 | NA | GACATTCAGATGACCCAGTCTCCATCCTCCCT GTCTGCATCTGTAGGAGACAGAGTCACCATCA CTTGCCGGGCGAGTCAGGGCATTAGCAATTAT TTAGCCTGGTATCAGCAGAAACCAGGGAAAGT CCCTAAACTCCTGATCTATGTTGCATCCACTTT GCAATCAGGGGTCCCATCTCGGTTCAGTGGCA GTGGATCTGGGACAGATTTCACTCTCACCATC AGCAGCCTGCAGCCTGAAGATGTTGCAACTTA TTACTGTCAAAAGTATAACAGTGCCCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGACTCAGCTATTATTGGTAATGGTGGT AGGACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAAGATATGGG TAGATACAGCTATGGTTTCTTTGACTACTGGGGC CAGGGAACCCTGGTCACCGTCTCCTCA |
|  |  |  | SEQ ID NO: 26609 | SEQ ID NO: 30615 |

FIGURE 50

| | | | | |
|---|---|---|---|---|
| | | AA | DIQMTQSPSSLSASVGDRVTITCRASQGISNYLA WYQQKPGKVPKLLIYVASTLQSGVPSRFSGSGS GTDFTLTISSLQPEDVATYYCQKYNSAPFTFGPG TKVDIK | EVQLLESGGGLVQPGGSLRLSCAASGFTFSSYAMS WVRQAPGKGLEWDSAIIGNGGRTYYADSVKGRFTI SRDNSKNTLYLQMNSLRAEDTAVYYCAKDMGRYS YGFFDYWGQGTLVTVSS |
| | | | SEQ ID NO: 26610 | SEQ ID NO: 30616 |
| iPS:435835 | 21-225_190F12 | NA | GACATCCAGATGACCCAGTCTCCATCCTCACT GTCTGCATCTGTGGGAGACAGAGTCACCATCA CTTGTCGGGCGAGTCAGGGCATTGGCAAGTAT TTAGCCTGGTTTCAGCAGAAACCAGGGAAAGC CCCTAAGTCACTGATCTATGCTGCATCCAGTTT GCAAAGTGGGGTCCCATCAAAATTCAGCGGCA GTGGATCTGGGACAGATTTCACTCTCACCATC AGCAGCCTGCAGCCTGACGATTTTGCAACTTA TTACTGCCAACGATATGATACTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGGAACTATGGCAT GCACTGGGTCCGCCAGGCTCCAGGCAAGGGGCT GGAGTGGGTGGCAGTTATATGGTTTGATGGAAGT AATGACTACTATGCAGACTCCGTGAAGGGCCGAT TCACCATCTCCAGAGACAATTCCAAGAACACGCT GTCACTGCAAATGAACAGCCTGAGAGCCGAGGA CACGGCTGTGTATTACTGTGCGAGAGATAGAAGC GTCGGCTACGACGGTTTAGATGTCTGGGGCCAAG GGACCACGGTCACCGTCTCCTCA |
| | | | SEQ ID NO: 26611 | SEQ ID NO: 30617 |
| | | AA | DIQMTQSPSSLSASVGDRVTITCRASQGIGKYLA WFQQKPGKAPKSLIYAASSLQSGVPSKFSGSGSG TDFTLTISSLQPDDFATYYCQRYDTYPFTFGPGT KVDIK | QVQLVESGGGVVQPGRSLRLSCAASGFTFRNYGM HWVRQAPGKGLEWVAVIWFDGSNDYYADSVKGR FTISRDNSKNTLSLQMNSLRAEDTAVYYCARDRSV GYDGLDVWGQGTTVTVSS |
| | | | SEQ ID NO: 26612 | SEQ ID NO: 30618 |

2231

FIGURE 50

| iPS:435837 | 21-225_198G3 | NA | GACATCCAGATGGCCCAGTCTCCTTCCTCACT GTCTGCATCTGTAGGAGACAGAGTCACCATCA CTTGTCGGACGAGTCAGGGCATTGGCAAGTAT TTAGCCTGGTTTCAGCAGAAGCCAGGGAAAGC CCCTAAGTCCCTGCTCTATAAAGCATCCAGTTT GCAAGGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATATGACTTACCCGCTCA CTTTCGGCGGAGGGACCAAGGTGGAGATCAA A | CAGGTGCAGCTGGTGGAATCTGGGGGAGGCGTG GTCCAGCCTGGGAGGTCCCTGAAACTCTCCTGTG CAGCGTCTGGATTCACCTTCAGTACCTATGGCAT GCACTGGGTCCGCCAGGCTCCAGGCAAGGGACT GGAGTGGGTGGCAGTTATATGGTATGATGGAACT AATAAAAACTATGCAGACTCCGTGAAGGGCCGA TTCACCATCTCCAGAGACAATTCCAAGAACACGC TGTGTCTGCAAATGAACAGCCTGAGAGCCGAGG ACACGGCTGTGTATTACTGTGCGAGAGATCAGGG CGTGGGCTACGACGGTTTGGACGTCTGGGGCCAA GGGACCACGGTCACCGTCTCCTCA |
|---|---|---|---|---|
| | | | SEQ ID NO: 26613 | SEQ ID NO: 30619 |
| | | AA | DIQMAQSPSSLSASVGDRVTITCRTSQGIGKYLA WFQQKPGKAPKSLLYKASSLQGGVPSKFSGSGS GTDFTLTISSLQPEDFATYYCQQYMTYPLTFGGG TKVEIK | QVQLVESGGGVVQPGRSLKLSCAASGFTFSTYGMH WVRQAPGKGLEWVAVIWYDGTNKNYADSVKGRF TISRDNSKNTLCLQMNSLRAEDTAVYYCARDQGV GYDGLDVWGQGTTVTVSS |
| | | | SEQ ID NO: 26614 | SEQ ID NO: 30620 |
| iPS:435839 | 21-225_191B1 | NA | GACATTGTGATGACCCAGACTCCACTCTCTCT GTCCGTCACCCCTGGACAGCCGGCCTCCATCT CCTGTAGGTCTAGTCAGAGCCTCCTGCATAGT GATGGAAAGACCTATTTGTTTTGGTACCTGCA GAAGCCAGGCCAGCCTCCACAGGTCCTGATCT ATGAACTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TTTCCAGCTTCCCTGGACGTTCGGTCAAGGGA CCAAGGTGGAAATCAAT | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTATCACTG GAGCTGGATCCGGCAGCCCGCCGGGAAGGGACT GGAGTGGATTGGCCATATCTATACCAGTGGGAGC ACCAAGTACAACCCCTCCCTCAAGAGTCGAGTCA CCATGTCAGTAGACACGTCCAAGAACCAGTTCTC CCTGAAGCTGAGCTCTGTGACCGCCGCGGACACG GCCGTGTATTACTGTGCGAGACTCCGGTATAACT GGAACTTCCCTTTCTTTGACTATTGGGGCCAGGG AACCCTGGTCACCGTCTCCTCA |
| | | | SEQ ID NO: 26615 | SEQ ID NO: 30621 |

FIGURE 50

| | | | | |
|---|---|---|---|---|
| | | AA | DIVMTQTPLSLSVTPGQPASISCRSSQSLLHSDGK TYLFWYLQKPGQPPQVLIYELSNRFSGVPDRFSG SGSGTDFTLKISRVEAEDVGVYYCMQSFQLPWT FGQGTKVEIN | QVQLQESGPGLVKPSETLSLTCTVSGGSISSYHWSW IRQPAGKGLEWIGHIYTSGSTKYNPSLKSRVTMSVD TSKNQFSLKLSSVTAADTAVYYCARLRYNWNFPFF DYWGQGTLVTVSS |
| | | | SEQ ID NO: 26616 | SEQ ID NO: 30622 |
| iPS:435841 | 21-225_191D8 | NA | GACATCATGATGACCCAGTCTCCAGACTCCCT GGCTGTGTCTCTGGGCGAGAGGGCCATCATCA GCTGCAGGTCCAGCCAGAGTGTTTTACACAGC TCCAACAATTACAACTACTTAGCTTGGTACCA GCAGAAACCAGGACATCCTCCTAAACTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAACAA TATTATAGTACTCCTCCGACGTTCGGCCTAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCCATAGCAGT GGCTGGTACTACTTTGACTACTGGGGGCCAGGGAA CCCTGGTCACCGTCTCCTCA |
| | | | SEQ ID NO: 26617 | SEQ ID NO: 30623 |
| | | AA | DIMMTQSPDSLAVSLGERAIISCRSSQSVLHSSNN YNYLAWYQQKPGHPPKLLIYWASTRESGVPDRF SGSGSGTDFTLTISSLQAEDVAVYYCQQYYSTPP TFGLGTKVEIK | QVQLVQSGGAEVKKPGASVKVSCKASGYTFTNYDIN WVRQATGQGLEWMGWMHPNSGNTGYAQKFQGR VTMTRNTSISTAYMELSSLRSEDTAVYYCAHSSGW YYFDYWGQGTLVTVSS |
| | | | SEQ ID NO: 26618 | SEQ ID NO: 30624 |

FIGURE 50

| iPS:435843 | 21-225_191F1 | NA | GAAATTGTGTGTTGACGCAGTCTCCAGGCACCCT GTCTTTGTCTCCAGGGGAAAGAGCCGCCCTCT CCTGCAGGGCCAGTCAGAGTATTAGCCTCAAC TTCTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAATAGACTGGAGCCTGAAGATTTTGCAG TGTATTACTGTCAGCAGTATGGTAGGTCACCG TGGACGTTCGGCCAAGGGACCAAGGTGGAAG TCAAA<br><br>SEQ ID NO: 26619 | CAGGTGCAGCTGCAGGAGTCGGGCCCAGGACTG GTGAAGCCTTCACAGACCCTGTCCCTCACCTGCA CTGTTTCTGGTGGCTCCATCAACAGTGGTGGTTA CTACTGGAACTGGATCCGCCAGCACCCAGGGAA GGGCCTGGAGTGGATTGGGTACATCTTTTACAGT GGGAGCACCTACTACAACCCGTCCCTCAAGAGTC GAGTCACCATATCAGTAGACACGTCTAAGAACC AGTTCTCCCTGAAGCTGAGCTCTGTGACTGCCGC GGACACGGCCGTGTATTACTGTGCGAGAGGGGA TTACGATGGTTCGGGGAGTTATCACTACTATTAC GGTATGGACGTCTGGGGCCAAGGGACCACGGTC ACCGTCTCCTCA<br><br>SEQ ID NO: 30625 |
| | | AA | EIVLTQSPGTLSLSPGERAALSCRASQSISLNFLA WYQQKPGQAPRLLIYGASSRATGIPDRFSGSGSG TDFTLTINRLEPEDFAVYYCQQYGRSPWTFGQG TKVEVK<br><br>SEQ ID NO: 26620 | QVQLQESGPGLVKPSQTLSLTCTVSGGSINSGGYY WNWIRQHPGKGLEWIGYIFYSGSTYYNPSLKSRVTI SVDTSKNQFSLKLSSVTAADTAVYYCARGDYDGS GSYHYYYGMDVWGQGTTVTVSS<br><br>SEQ ID NO: 30626 |
| iPS:435845 | 21-225_191G1 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGAACAGATTTCACTCTCACCATC AGCAGCCTGCAGCCTGAAGATTTTGCAACTTA TTACTGCCAACATTATCTTACTTACCCTCTCAC TTTCGGCGGAGGGACCAAGGTGGAGATCAAA<br><br>SEQ ID NO: 26621 | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATAGGGG GGTGGGTTACTACGGTTTGGACGTCTGGGGCCAA GGGACCACGGTCACCGTCTCCTCA<br><br>SEQ ID NO: 30627 |

FIGURE 50

| | | | | |
|---|---|---|---|---|
| | | AA | DIQMTQSPSSLSASVGDRVTITCRASQGISNYLA WFQQKPGKAPKSLIYAASSLQSGVPSKFSGSGSG TDFTLTISSLQPEDFATYYCQHYLTYPLTFGGGT KVEIK | QVQLVESGGGVVQPGRSLRLSCAASGFTFSSYGMH WVRQAPGKGLEWVAVIWYDGSNEHYADSVKGRF TISRDNSKNTLYLQMNSLRAEDTAVYYCARDRGV GYYGLDVWGQGTTVTVSS |
| | | | SEQ ID NO: 26622 | SEQ ID NO: 30628 |
| iPS:435847 | 21-225_191A3 | NA | GAAATTGTGTTGACGCAGTCTCCAGGCACCCT GTCTTTGTCTCCAGGGGAAAGAGCCACCCTCT CCTGCAGGGCCAGTCAGAGTATTCGCAGCAGC TTCTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAATATGGTAACTCACCG TGGGCGTTCGGCCAAGGGACCAAGGTGGAAG TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGATCCGCCAGCACCCAGGGAA GGGCCTGGAGTGGATTGGGTACATCTTTTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGGGGATT ACGATGGTTCGGGGGAGTTATCACTACTACTACGG TATGGACGTCTGGGGGCCAAGGGACCACGGTCAC CGTCTCCTCA |
| | | | SEQ ID NO: 26623 | SEQ ID NO: 30629 |
| | | AA | EIVLTQSPGTLSLSPGERATLSCRASQSIRSSFLA WYQQKPGQAPRLLIYGASSRATGIPDRFSGSGSG TDFTLTISRLEPEDFAVYYCQQYGNSPWAFGQG TKVEVK | QVQLQESGPGLVKPSQTLSLTCTVSGGSISSGDYYW NWIRQHPGKGLEWIGYIFYSGSTYYNPSLKSRVTIS VDTSKNQFSLKLSSVTAADTAVYYCARGDYDGSG SYHYYYGMDVWGQGTTVTVSS |
| | | | SEQ ID NO: 26624 | SEQ ID NO: 30630 |

FIGURE 50

| | | | | |
|---|---|---|---|---|
| iPS:435849 | 21-225_191C3 | NA | GAAATTGTGTGTTGACGCAGTCTCCAGGCACCCT GTCTTTGTCTCCAGGGGAAAGAGCCACCCTCT CCTGCAGGGCCAGTCAGAGTATTCGCAGCAGC TTCTTAGCCTGGTATCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAACTCACCG TGGGCGTTCGGCCAAGGGACCAAGGTGGAAA TCAAA<br><br>SEQ ID NO: 26625 | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGATCCGCCAGCTCCCAGGGAA GGGCCTGGAGTGGATTGGGTACATCTTTTACAGT GGGAGCACCTACTACAACCCGTCCCTCAAGAGTC GACTTACCATATCAGTGGACACGTCTAAGAACCA GTTCTCCCTGAAACTGAACTCTGTGACTGCCGCG GACACGGCCGTGTATTACTGTGCGAGAGGGGATT ACGATGGTTCGGGGAGTTATCACTTCTACTACGG TATGGACGTCTGGGGCCAAGGGACCACGGTCAC CGTCTCCTCA<br><br>SEQ ID NO: 30631 |
| | | AA | EIVLTQSPGTLSLSPGERATLSCRASQSIRSSFLA WYQQKPGQAPRLLIYGASSRATGIPDRFSGSGSG TDFTLTISRLEPEDFAVYYCQQYGNSPWAFGQG TKVEIK<br><br>SEQ ID NO: 26626 | QVQLQESGPGLVKPSQTLSLTCTVSGGSISSGDYYW NWIRQLPGKGLEWIGYIFYSGSTYYNPSLKSRLTISV DTSKNQFSLKLNSVTAADTAVYYCARGDYDGSGS YHFYYGMDVWGQGTTVTVSS<br><br>SEQ ID NO: 30632 |
| iPS:435851 | 21-225_191D3 | NA | GAAATTGTGTGTTGACGCAGTCTCCAGGCACCCT GTCTTTGTCTCCAGGGGAAAGCGCCACCCTCT CCTGCAGGGCCGGTCAAAGTATTAGAACCAAC TTCTTAGCCTGGTACCAGCAGCAGCCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAACAGACTGGAGCCTGAAGATTTTGCAG TGTATTACTGTCAGCAGTATGGTAGCTCACCG TGGACGTTCGGCCAAGGGACCAAGGTGGAAA TCAAA<br><br>SEQ ID NO: 26627 | CAGGTGCAACTGAAGGAGTCGGGCCCAGGACTG GTGAAGCCTTCACAGACCCTGTCCCTCACCTGCA CTGTCTCTGGTGGCTCCATCAACAGTGGTGATTA CTACTGGAACTGGATCCGCCAGCACCCAGGGAA GGGCCTGGACTGGATTGGGTACATCTTTTACAGT GGGAGCACCTACTACAACCCGTCCCTCAAGAGTC GAGTTACCATATCAGTAGACACGTCTAAGAACCA GTTCTCCCTGAAAGCTGAGCTCTGTGACTGCCGCG GACACGGCCGTGTATTACTGTGCGAGAGGGGATT ACGATGGTTCGGGGAGTTATCACTACTATTACGG TATGGACGTCTGGGGCCAAGGGACCACGGTCAC CGTCTCCTCA<br><br>SEQ ID NO: 30633 |

FIGURE 50

| | | | | |
|---|---|---|---|---|
| | | AA | EIVLTQSPGTLSLSPGESATLSCRAGQSIRTNFLA WYQQQPGQAPRLLIYGASSRATGIPDRFSGSGSG TDFTLTINRLEPEDFAVYYCQQYGSSPWTFGQGT KVEIK | QVQLKESGPGLVKPSQTLSLTCTVSGGSINSGDYY WNWIRQHPGKGLDWIGYIFYSGSTYYNPSLKSRVT ISVDTSKNQFSLKLSSVTAADTAVYYCARGDYDGS GSYHYYYGMDVWGQGTTVTVSS |
| | | | SEQ ID NO: 26628 | SEQ ID NO: 30634 |
| iPS:435853 | 21-225_191E3 | NA | GATATTGTAATGACCCAGACTCCACTCTCTCT GTCCGTCACCCCTGGACAGCCGGCCTCCATCT CCTGCAAGTCTAGTCAGAGCCTCCTCCATAGT GATGGAAGGACCTATTTGTATTGGTACCTGCA GAAGCCAGGCCAGCCTCCACAGCTCCTGATCT GTGAGGTTTCCAACCGGTTCGCTGGAGTGACA GATAGGTTCAGTGGCAGCGGGTCAGGGACAG ATTTCACACTGAAAATCAGCCGGGTGGAGGCT GGGGATGTTGGGGTTTATTACTGCATGCAAAG TATACAACTTCCCTGGACGTTCGGCCAAGGGA CCAAGGTGGAAATCAAA | CAGCTGCAGCTGCAGGAGTCGGGCCCAGGACTG GTGAAGCCTTCGGAGACCCTGTCCCTCACCTGCA CTGTCTCTGGTGGCTCCATCAGAAGCTACCACTG GAGCTGGATCCGGCAGCCCGCCGGGAAGGGACT GGAGTGGATTGGACTTATCTATACCAGTAGGAGC ACCAATTACAACCCCTCCCTCAAGAGTCGAGTCA CCATGTCAGTTGACACGTCCAAGAACCAGTTCTC CCTGAAGCTGAACTCTGTGACCGCCGCGGACACG GCCGTGTATTACTGTGCGAGACTCCGGTATAACT GGAACTTCCCCTTACTTTGACTACTGGGGCCAGGG AACCCTGGTCACCGTCTCCTCA |
| | | | SEQ ID NO: 26629 | SEQ ID NO: 30635 |
| | | AA | DIVMTQTPLSLSVTPGQPASISCKSSQSLLHSDGR TYLYWYLQKPGQPPQLLICEVSNRFAGVTDRFS GSGSGTDFTLKISRVEAGDVGVYYCMQSIQLPW TFGQGTKVEIK | QLQLQESGPGLVKPSETLSLTCTVSGGSIRSYHWSW IRQPAGKGLEWIGLIYTSRSTNYNPSLKSRVTMSVD TSKNQFSLKLNSVTAADTAVYYCARLRYNWNFPY FDYWGQGTLVTVSS |
| | | | SEQ ID NO: 26630 | SEQ ID NO: 30636 |

FIGURE 50

| iPS:435855 | 21-225_191G3 | NA | GACATCGTGATGACCCAGTCTCCAGACTCCCT GGCTGTGTCTCTGGGCGAGAGGGCCACCATCG ACTGTAAGTCCAGCCAGAGTGTTTTACACAGC TCCAACAGTTACAACTACTTAGCTTGGTACCA GCAGAAATTAGGACAGCCTCCTAAACTGCTCA TTTACTGGGCATCTACCCGAAAATCCGGGGTC CCTGACCGATTCAGTGGCAGCGGGTCTGGGAC AGATTTCACTCTCACCATCAGCAGCCTGCAGG CTGAAGATGTGGCATTTTATTACTGTCAGCAA TATTATAGTAGTCCTCCCACTTTCGGCCCTGGG ACCAAAATGGATATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGACGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCCCATAGCAG TGGCTGGTACATCTTTGACTACTGGGGCCAGGGA ACCCTGGTCACCGTCTCCTCA |
| | | | SEQ ID NO: 26631 | SEQ ID NO: 30637 |
| | | AA | DIVMTQSPDSLAVSLGERATIDCKSSQSVLHSSN SYNYLAWYQQKLGQPPKLLIYWASTRKSGVPD RFSGSGSGTDFTLTISSLQAEDVAFYYCQQYYSS PPTFGPGTKMDIK | QVQLVQSGAEVKKPGASVKVSCKASGYTFTNYDIN WVRQATGQGLEWMGRMNPNSGNTGYAQKFQGR VTMTRNTSISTAYMELSSLRSEDTAVYYCAHSSGW YIFDYWGQGTLVTVSS |
| | | | SEQ ID NO: 26632 | SEQ ID NO: 30638 |
| iPS:435857 | 21-225_191A4 | NA | GACATCCAGATGACCCAGTCTCCATCCTCCCT GTCTGCATCTGTAGGAGACAGAGTCACCATCA CTTGCCGGGCAAGTCAGGGCATTAGAAAAGAT TTAGGCTGGTATCAGCAGAAACCAGGGAAAG CCCCTAAGCGCCTGATCCATACTGCATCCAGT TTGCAAAATGGGGTCCCATTAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGATGGAGGT TATAAAAACTATGCAGACTCCGTGAAGGGCCGA TTCACCATCTCCAGAGACAATTCCAAGAACACGC TGTATCTGCAAATGAATAGCCTGAGAGCTGAGGA CACGGCTGTGTATTACTGTGCGAGAGGTACCCAC GGGTACTACTACGGTGTGGACGTCTGGGGCCAA GGGACCACGGTCACCGTCTCCTCA |
| | | | SEQ ID NO: 26633 | SEQ ID NO: 30639 |

FIGURE 50

| | | | | |
|---|---|---|---|---|
| | | AA | DIQMTQSPSSLSASVGDRVTITCRASQGIRKDLG WYQQKPGKAPKRLIHTASSLQNGVPLRFSGSGS GTEFTLTISSLQPEDFATYYCLQHNSYPWTFGQG TKVEIK | QVQLVESGGGVVQPGRSLRLSCAASGFTFSSYGMH WVRQAPGKGLEWVAVISYDGGYKNYADSVKGRF TISRDNSKNTLYLQMNSLRAEDTAVYYCARGTHG YYYGVDVWGQGTTVTVSS |
| | | | SEQ ID NO: 26634 | SEQ ID NO: 30640 |
| iPS:435859 | 21-225_190E6 | NA | GACATCCAGATGACCCAGTCTCCTTCCTCACT GTCTGCATCTGTAGGAGACAGAGTCACCATCA CTTGTCGGACGAGTCAGGGCATTGGCAATTAT TTAGCCTGGTTTCAGCAGAAACCAGGGAAAGC CCCTAAGTCCCTGCTCTATAAAGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATATGACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGCCAA GGGACCACGGTCACCGTCTCCTCA |
| | | | SEQ ID NO: 26635 | SEQ ID NO: 30641 |
| | | AA | DIQMTQSPSSLSASVGDRVTITCRTSQGIGNYLA WFQQKPGKAPKSLLYKASSLQSGVPSKFSGSGS GTDFTLTISSLQPEDFATYYCQQYMTYPLTFGGG TKVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDQGV GYDGLDVWGQGTTVTVSS |
| | | | SEQ ID NO: 26636 | SEQ ID NO: 30642 |

FIGURE 50

| iPS:435861 | 21-225_190A5 | NA | GACATCCAGATGACCCAGTCTCCATCCTCACT GTCTGCATCTGTAGGAGACAGAGTCACCATCA CTTGTCGGGCGAGTCAGGGGATTGGCAATCAT TTAGCCTGGTTTCAGCAGAAACCAGGGAAAGC CCCTAAGTCCCTGATCCATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAGTAATTACCCAGTCA CTTTCGGCCCTGGGACCAAAGTGGATATCAAA | CAGGTGCAGTTGGTGGAGTCTGGGGGAGGCGTG GGCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATTTCTC TGTAGGGTACGACGGTATGGACGTCTGGGGCCA AGGGACCACGGTCACCGTCTCCTCA |
| | | | SEQ ID NO: 26637 | SEQ ID NO: 30643 |
| | | AA | DIQMTQSPSSLSASVGDRVTITCRASQGIGNHLA WFQQKPGKAPKSLIHAASSLQSGVPSKFSGSGSG TDFTLTISSLQPEDFATYYCQQYSNYPVTFGPGT KVDIK | QVQLVESGGGVGQPGRSLRLSCAASGFTFSSYGMH WVRQAPGKGLEWVAVIWYDGSNKNYADSVKGRF TISRDNSKNTLYLQMNSLRAEDTAVYYCARDFSVG YDGMDVWGQGTTVTVSS |
| | | | SEQ ID NO: 26638 | SEQ ID NO: 30644 |
| iPS:435863 | 21-225_191H4 | NA | GAAATTGTGCTGACTCAGTCTCCAGACTTTCA GTCTGTGACTCCAAAGGAGAAAGTCACCATCA CCTGCCGGGCCAATCAGAGCATTGGTAGTAGC TTACACTGGTACCAGCAGAAACCAGATCAGTC TCCAAAGCTCCTCATCAAGTATGCTTCCCAGT CCCTCTCAGGGGTCCCCTCGAGGTTCAGTGCC AGTGGATCTGGGACAGATTTCACCCTCACCAT CAATAGCCTGGATGCTGAAGATGCTGCAACGT ATTACTGTCATCAGACTGGTAGGTTACCTCTC ACTTTCGGCGGAGGGACCAAGGTGGAGATCA AA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAACTGGATCCGCCAGCACCCAGGGAA GGGCCTGGAGTGGATTGGGTACATCTTTTACAGT GGGAGCACCTACTACAACCCGTCCCTCAGGAGTC GACTTACCATATCAATAGACACGTCTAAGAACCA GTTCTCCCTGAAGCTGACCTCTGTGACTGCCGCG GACACGGCCGTATATTACTGTGGGAGATCCGGGT ATAACTGGGACAACGGGGTCGACCCCTGGGGCC AGGGAACCCTGGTCACCGTCTCCTCA |
| | | | SEQ ID NO: 26639 | SEQ ID NO: 30645 |

FIGURE 50

| IPS:435865 | 21-225_191A5 | AA | EIVLTQSPDFQSVTPKEKVTITCRANQSIGSSLHW YQQKPDQSPKLLIKYASQSLSGVPSRFSASGSGT DFTLTINSLDAEDAATYYCHQTGRLPLTFGGGT KVEIK | QVQLQESGPGLVKPSQTLSLTCTVSGGSISSGGYYW NWIRQHPGKGLEWIGYIFYSGSTYYNPSLRSRLTISI DTSKNQFSLKLTSVTAADTAVYYCGRSGYNWDNG VDPWGQGTLVTVSS |
|---|---|---|---|---|
| | | | SEQ ID NO: 26640 | SEQ ID NO: 30646 |
| | | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAGCAGG TTCTTAGCCTGGTACCAGCAGAAACCTGGCCA GGCTCCCAGGCTCCTCATCTATGGTGCTTCCA ACAGGGCCACTGGCATCCCCGACAGGTTCAGT GGCAGTGGGTCTGGGACAGACTTCACTCTCAC CATCAGCAGACTGGAGCCTGAAGATTTTGCAG TGTTATTACTGTCAGCAGTATGGTGGTTCACCTC CGTGGACGTTCGTCCAAGGGACCAAGGTGGA AATCAAA | GAGATACAGGTGGTGGAGTCTGGGGGAGGCCTG GTCAAGCCTGGGGGGTCCCTGAGACTCTCCTGTG CAGCCTCTGGATTCACCTTCAGAGACTATAGCAT GAACTGGGTCCGCCAGGCTCCAGGGAAGGGGCT GGAGTGGGTCTCATCCATTAGTAGTGGTAGTGGT TACATATATTATGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGCTACTATG GCCCCTTGACTACTGGGGCCAGGGAGCCCTGGTCA CCGTCTCCTCA |
| | | | SEQ ID NO: 26641 | SEQ ID NO: 30647 |
| | | AA | EIVLTQSPGTLSLSPGERATLSCRASQSVSSRFLA WYQQKPGQAPRLLIYGASNRATGIPDRFSGSGS GTDFTLTISRLEPEDFAVYYCQQYGGSPPWTFVQ GTKVEIK | EIQVVESGGGLVKPGGSLRLSCAASGFTFRDYSMN WVRQAPGKGLEWVSSISSGSGYIYYADSVKGRFTIS RDNAKNSLYLQMNSLRAEDTAVYYCARATMALD YWGQGALVTVSS |
| | | | SEQ ID NO: 26642 | SEQ ID NO: 30648 |

FIGURE 50

| iPS:435867 | 21-225_191E5 | NA | GAAATTGTGCTGACTCAGTTTCCAGACTCTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCACCCTCACCAT CAATAGTCTGGAGGCTGAAGATGCTGCAACGT ATTACTGTCATCAGAGTAGTAGTTTCCCTCGG ACGTTCGGCCAAGGGACCAAGGTGGAAATCA AA | GAGGTGCAGCTGTTGGACTCTGGGGGAGGCTTGG GACAGCCTGGGGGGTCCCTGAGACTCTCCTGTGC AGCCTCTGGATTCACCTTTGACAGCTATGCCATG AACTGGGTCCGCCAGGCTCCAGGGAAGGGGCTG GAGTGGGTCTCAACTATTAGTGGTACTGGTCGTA GGACATACTACGCAGACTCCGTGAAGGGCCGGT TCACCATCTCCAGAGACAATTCCAAGAACACGCT GTATCTGCAAATGAATAGCCTGAGAGCCGAGGA CACGGCCGTATATTACTGTGCGAAAGAGGAGGA TTACTATGATAGTAGTGGCCCGGGGTTCGACCCC TGGGGCCAGGGAACCCTGGTCACCGTCTCCTCA |
| | | | SEQ ID NO: 26643 | SEQ ID NO: 30649 |
| | | AA | EIVLTQFPDSQSVTPKEKVTITCRASQSIGSSLHW YQQKPDQSPKLLIKYASQSFSGVPSRFSGSGSGT DFTLTINSLEAEDAATYYCHQSSSFPRTFGQGTK VEIK | EVQLLDSGGGLGQPGGSLRLSCAASGFTFDSYAMN WVRQAPGKGLEWVSTISGTGRRTYYADSVKGRFTI SRDNSKNTLYLQMNSLRAEDTAVYYCAKEEDYYD SSGPGFDPWGQGTLVTVSS |
| | | | SEQ ID NO: 26644 | SEQ ID NO: 30650 |
| iPS:435869 | 21-225_190B1 | NA | GACATCCAGATGACCCAGTCTCCATCCTCACT GTCTGCATCTGTTGGAGACAGAGTCACCATCA CTTGTCGGGCGAGTCAGGGCATTAGAAATTAT TTAGCCTGGTTTCAGCAGAAACCAGGGAAAGC CCCTAAGTCCCTGATCTATGTTGCATCCAGTTT GGAAAGTGGGGTCCCATCAAAGTTCAGTGGCA GTGGATCTGGGACAGAGTTCACTCTCACCATC AGCAGCCTGCAACCTGAAGATTTTGGAACTTA TTACTGCCAACAGTATCTTAATTACCCAGTCA CTTTCGGCCCTGGGACCAAAGTGGATATCAGA | CAGGTGCAGCTGGTGGAGTCTGGGGGAGGCGTG GTCCAGCCTGGGAGGTCCCTGAGACTCTCCTGTG CAACGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAACATTATGCAGACTCCGTGAAGGGCCGA TTCACCATCTCCAGAGACAATTCCAAGAACACAC TATATCTGCAAATGAACAGCCTGAGAGCCGAGG ACACGGCTGTTTATTACTGTGCGAGAGATAGAAC AGTGGGATACTCCGGTATGGACGTCTGGGGCCA AGGGACCACGGTCACCGTCTCCTCA |
| | | | SEQ ID NO: 26645 | SEQ ID NO: 30651 |

FIGURE 50

| | | | | |
|---|---|---|---|---|
| | | AA | DIQMTQSPSSLSASVGDRVTITCRASQGIRNYLA WFQQKPGKAPKSLIYVASSLESGVPSKFSGSGSG TEFTLTISSLQPEDFGTYYCQQYLNYPVTFGPGT KVDIR | QVQLVESGGGVVQPGRSLRLSCATSGFTFSSYGMH WVRQAPGKGLEWVAVIWYDGSNKHYADSVKGRF TISRDNSKNTLYLQMNSLRAEDTAVYYCARDRTVG YSGMDVWGQGTTVTVSS |
| | | | SEQ ID NO: 26646 | SEQ ID NO: 30652 |
| iPS:435871 | 21-225_191E6 | NA | GATATTGTGATGACCCAGACTCCACTCTCTCT GTCCGTCACCCCTGGACAGCCGGCCTCCATCT CCTGCAAGTCTAGTCAGAGCCTCCTCCATAGT GATGGAAGGACCTATTTGTATTGGTACCTGCA GAAGCCAGGCCAGCCTCCACAGCTCCTGATCT GTGAGGTTTCCAACCGGTTCGCTGGAGTGACA GATAGGTTCAGTGGCAGCGGGTCAGGGACAG ATTTCACACTGAAAATCAGCCGGGTGGAGGCT GGGGATGTTGGGATTTATTACTGCATGCAAAG TATACATTTTCCCTGGACGTTCGGCCAAGGGA CCAAGGTGGAAATCAAA | CAGCTGCAGCTGCAGGAGTCGGGCCCAGGACTG GTGAAGCCTTCGGAGACCCTGTCCCTCACCTGCA CTGTCTCTGGTGGCTCCATCAGAAGCTACCACTG GAGCTGGATCCGGCAGCCCGCCGGGAAGGGACT GGAGTGGATTGGGCTTATCTATACCAGTAGGAGC ACCAATTACAACCCCTCCCTCAAGAGTCGAGTCA CCATGTCAGTAGACACGTCCAAGAACCAGTTCTC CCTGAAGCTGAGCTCTGTGACCGCCGCGGACACG GCCGTGTATTACTGTGCGAGACTCCGGTATAACT GGAACTTCCCTTACTTTGACTTCTGGGGCCAGGG AACCCTGGTCACCGTCTCCTCA |
| | | | SEQ ID NO: 26647 | SEQ ID NO: 30653 |
| | | AA | DIVMTQTPLSLSVTPGQPASISCKSSQSLLHSDGR TYLYWYLQKPGQPPQLLICEVSNRFAGVTDRFS GSGSGTDFTLKISRVEAGDVGIYYCMQSIHFPWT FGQGTKVEIK | QLQLQESGPGLVKPSETLSLTCTVSGGSIRSYHWSW IRQPAGKGLEWIGLIYTSRSTNYNPSLKSRVTMSVD TSKNQFSLKLSSVTAADTAVYYCARLRYNWNFPYF DFWGQGTLVTVSS |
| | | | SEQ ID NO: 26648 | SEQ ID NO: 30654 |

FIGURE 50

| iPS:435873 | 21-225_190G4 | NA | GACATCCAGATGACCCAGTCTCCATCCTCACT GTCTGCATCTGTAGGAGACAGAGTCACCATCA CTTGTCGGGCGAGTCAGGACATTGGCAGATAT TTAGCCTGGTTTCAGCAGAAGCCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAAATTTTGCAACTTA TTACTGCCAACAATATAGTACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTACGCAGACTCCGTGAAGGGCCGA TTCACCATCTCCAGAGACAATTCCAAGAACACGC TGTATCTGCAAATGAACAGCCTGAGAGCCGAGG ACACGGCTATGTATTACTGTGCGAGAGATCAGGG CGTGGGCTACGACGGTTTGGACGTCTGGGGGCCAA GGGACCTCGGTCACCGTCTCCTCA |
| | | | SEQ ID NO: 26649 | SEQ ID NO: 30655 |
| | | AA | DIQMTQSPSSLSASVGDRVTITCRASQDIGRYLA WFQQKPGKAPKSLIYAASSLQSGVPSKFSGSGSG TDFTLTISSLQPENFATYYCQQYSTYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAMYYCARDQGV GYDGLDVWGQGTSVTVSS |
| | | | SEQ ID NO: 26650 | SEQ ID NO: 30656 |
| iPS:435875 | 21-225_190B9 | NA | GACATCCAGATGACCCAGTCTCCATCTTCCGT GTCTGCATCTGTAGGAGACAGAGTCACCATCA CTTGTCGGGCGAGTCAGGGTATTAACAACTGG TTAGCCTGGTATCAGCAGAAACCAGGGAAAG CCCCTAAGCTCCTGATCTATGGTGTTTCCAGTT TGCAGAGTGGGGTCCCATCAAGGTTCAGCGGC AGTGGATCTGGGACAGATTTCACTCTCACCAT CAGCAGCCTGCAGCCTGAAGATTTTGCAACTT ACTCTTGTCAACAGGCTAACAGTTTCCCGTGG ACGTTCGGCCAAGGGACCAAGGTGGAAATCA GA | GAGGTGCAGCTGTTGGAGTCTGGGGGAGGCTTG GTACAGCCTGGGGGGTCCCTGAGACTCTCCTGTG CAGCCTCTGGATTCACCTTTAGCACCTATGCCAT GAGTTGGGTCCGCCAGGCTCCAGGGAAGGGGCT GGAGTGGGTCTCAGCTATTAGTCGTAGTGGTGGT AACACACACTACGCAGACTCCGTGAAGGGCCGG TTCACCATCTCCAGAGACAATTCCAAGAATACGC TGTATCTGCAAATGAACAGCCTGAGAGCCGAGG ACACGGCCGTATATTCCTGTGCGAAAGATGGATT CGGTGGGAGCTCCTACTTTGACTACTGGGGCCAG GGAACCCTGGTCACCGTCTCCTCA |
| | | | SEQ ID NO: 26651 | SEQ ID NO: 30657 |

FIGURE 50

| | | | | |
|---|---|---|---|---|
| | | AA | DIQMTQSPSSVSASVGDRVTITCRASQGINNWLA WYQQKPGKAPKLLIYGVSSLQSGVPSRFSGSGS GTDFTLTISSLQPEDFATYSCQQANSFPWTFGQG TKVEIR | EVQLLESGGGLVQPGGSLRLSCAASGFTFSTYAMS WVRQAPGKGLEWVSAISRSGGNTHYADSVKGRFTI SRDNSKNTLYLQMNSLRAEDTAVYSCAKDGFGGS SYFDYWGQGTLVTVSS |
| | | | SEQ ID NO: 26652 | SEQ ID NO: 30658 |
| IPS:435877 | 21-225_184E7 | NA | GACATCCAGATGACCCAGTCTCCATCCTCACG GTCTGCATCTATAGGAGAGAGAGTCACCATCA CTTGTCGGGCGAGTCAGGGCATTAGCAATTAT TTAGCCTGGATTCAGCAGAAACCAGGGACAGC CCCTAAGTCCCTTATTTATGCTGCATCCAGTTT GCAAAGTGGGGTTTCATCAAGGTTTAGCGGCA GTGGATTTGGGACAGATTTCACTATCACCATC AGTAGTGTGCAGCGTGAAGATTTTGCAACTTA TTACTGCCAACAGTATAATGGTTACCCATTCA CTTTCGGCCATGGGACCAAAGTGGATATCAAA | CAGGTGCAGCTGGTGCAGTCTGGGGGCTGAAGTG AAGACGCCTGGGGCCTCAGTGAAGGTCTCCTGCA GGGCTTCTGGATACACCTTCACCAGCTACAATAT GCACTGGGTGCGACAGGTCCCTGGACAAGGGCT TGAGTGGATGGGATGGATCAACCCTAACAATGGT GGCTCAAACTATACACAGAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGAGAAAGTTTG GGGACTGGGGCCAGGGAACCCTGGTCACCGTCT CCTCA |
| | | | SEQ ID NO: 26653 | SEQ ID NO: 30659 |
| | | AA | DIQMTQSPSSRSASIGERVTITCRASQGISNYLAW IQQKPGTAPKSLIYAASSLQSGVSSRFSGSGFGTD FTITISSVQREDFATYYCQQYNGYPFTFGHGTKV DIK | QVQLVQSGAEVKTPGASVKVSCRASGYTFTSYNM HWVRQVPGQGLEWMGWINPNNGGSNYTQKFQGR VTMTRDTSISTAYMELSRLRSDDTAVYYCARKFGD WGQGTLVTVSS |
| | | | SEQ ID NO: 26654 | SEQ ID NO: 30660 |

FIGURE 50

| iPS:435879 | 21-225_184H10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATATTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG TAGCGTCTGGATTCACCTTCAGTGACTATGGCAT GCACTGGGTCCGCCAGGCTCCAGGCAAGGGGCT GGAGTGGGTGGCAGTTATTTGGTATGATGAAACT AATAAACACTATGGAGACTCCGTGAAGGGCCGA TTCACCATCTCCAGAGACAATTCCAAGGACACGC TGTATCTGCAAATGAACAGCCTGAGAGCCGAGG ACACGGCTGTGTATTACTGTGCGAGAGAGGTTGG CTGGCACGATGACTATTGGGGCCAGGGAACCCT GGTCACCGTCTCCTCA |
| | | | SEQ ID NO: 26655 | SEQ ID NO: 30661 |
| | | AA | DIQMTQSPSSLSASVGDRVTITCRASQGIRNDLG WYQQKPGKAPKRLIYIASSLQSGVPSRFSGSGSG TEFTLTISSLQPEDFATYYCLQHNSYPFTFGPGTK VDIK | QVQLVESGGGVVQPGRSLRLSCVASGFTFSDYGM HWVRQAPGKGLEWVAVIWYDETNKHYGDSVKGR FTISRDNSKDTLYLQMNSLRAEDTAVYYCAREVG WHDDYWGQGTLVTVSS |
| | | | SEQ ID NO: 26656 | SEQ ID NO: 30662 |
| iPS:435881 | 21-225_184D11 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATATTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATTTGGTATGATGAAACT AATAAACACTATGGAGACTCCGTGAAGGGCCGA TTCACCATCTCCAGAGACAATTCCAAGGACACGC TGTATCTGCAAATGAACAGCCTGAGAGCCGAGG ACACGGCTGTGTATTACTGTGCGAGAGAGGTTGG CTGGCACGATGACTATTGGGGCCAGGGAACCCT GGTCACCGTCTCCTCA |
| | | | SEQ ID NO: 26657 | SEQ ID NO: 30663 |

FIGURE 50

| | | | | |
|---|---|---|---|---|
| | | AA | DIQMTQSPSSLSASVGDRVTITCRASQGIRNDLG WYQQKPGKAPKRLIYIASSLQSGVPSRFSGSGSG TEFTLTISSLQPEDFATYYCLQHNSYPFTFGPGTK VDIK | QVQLVESGGGVVQPGRSLRLSCAASGFTFSDYGM HWVRQAPGKGLEWVAVIWYDETNKHYGDSVKGR FTISRDNSKDTLYLQMNSLRAEDTAVYYCAREVG WHDDYWGQGTLVTVSS |
| | | | SEQ ID NO: 26658 | SEQ ID NO: 30664 |
| IPS:435883 | 21-225_185A1 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGACACCAGGGAAAGC CCCTAAGTCCCTGATCTCTGTTGCATCCAGTTT GCAAAGTGGGGTCCCATCAAGATTCAGCGCCA GTGGATCTGGGACAGATTTCACTCTCACCATC AGCAGCCTGCAGCCTGAAGATTTTGCAACTTA TTACTGCCGACAATATCATAGTTACCCATTCA CTTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAATAGCTATAGCAT GAACTGGGTCCGCCAGGCTCCAGGGAAGGGGCT GGAGTGGGTCTCATCCATTAGCAGTAGTGGTAGT TACATATATTACGCAGACTCAGTGAAGGGCCGAT TCACCATCTCCAGAGACAACGCCAAGAACTCACT GTATCTGCAAATGCACAGCCTGAGAGCCGAGGA CACGGCTGTGTATTACTGTGCGAGAAGCAACCTT TTTGACTGCTGGGGCCAGGGAACCCCGGTCACCG TCTCCTCA |
| | | | SEQ ID NO: 26659 | SEQ ID NO: 30665 |
| | | AA | DIQMTQSPSSLSASVGDRVTITCRASQGISNYLA WFQQTPGKAPKSLISVASSLQSGVPSRFSASGSG TDFTLTISSLQPEDFATYYCRQYHSYPFTFGPGTK VDIK | EVQLVESGGGLVKPGGSLRLSCAASGFTFNSYSMN WVRQAPGKGLEWVSSISSSGSYIYYADSVKGRFTIS RDNAKNSLYLQMHSLRAEDTAVYYCARSNLFDCW GQGTPVTVSS |
| | | | SEQ ID NO: 26660 | SEQ ID NO: 30666 |

FIGURE 50

| iPS:435885 | 21-225_185E10 | NA | GACATCCAGATGACCCAGTCTCCATCCTCACG GTCTGCATCTATAGGAGAGAGAGTCACCATCA CTTGTCGGGCGAGTCAGGGCATTAGCAATTAT TTAGCCTGGATTCAGCAGAAACCAGGGACAGC CCCTAAGTCCCTTATCTATGCTGCATCCAGTTT GCAAAGTGGGGTTTCATCAAGGTTTAGCGGCA GTGGATTTGGGACAGATTTCACTCTCACCATC AGTAGTCTGCAGCCTGAAGATTTTGCAACTTA TTACTGCCAACAGTATAATGGTTACCCATTCA CTTTCGGCCCTGGGACCAAAGTGGATATCAAA | CAGGTGCAGCTGGTGCAGTCTGGGGCTGAAGTG AAGACGCCTGGGGCCTCAGTGAAGGTCTCCTGCA GGGCTTCTGGATACACCTTCACCAGCTACAATAT GCACTGGGTGCGACAGGTCCCTGGACAAGGGCT TGAGTGGATGGGATGGATCAACCCTAACAATGGT GGCTCAAACTATACACAGAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGAGAAAGTTTG GGGACTGGGGCCAGGGAACCCTGGTCACCGTCT CCTCA |
|---|---|---|---|---|
| | | | SEQ ID NO: 26661 | SEQ ID NO: 30667 |
| | | AA | DIQMTQSPSSRSASIGERVTITCRASQGISNYLAW IQQKPGTAPKSLIYAASSLQSGVSSRFSGSGFGTD FTLTISSLQPEDFATYYCQQYNGYPFTFGPGTKV DIK | QVQLVQSGAEVKTPGASVKVSCRASGYTFTSYNM HWVRQVPGQGLEWMGWINPNNGGSNYTQKFQGR VTMTRDTSISTAYMELSRLRSDDTAVYYCARKFGD WGQGTLVTVSS |
| | | | SEQ ID NO: 26662 | SEQ ID NO: 30668 |
| iPS:435887 | 21-225_186F7 | NA | GATGTTGTGATGGCCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTGTTGGTACCTCCA GAAGCCAGGCCAGCCTCCACAGCTCCTGATCT ATGAAGTTTCCAAGCGGTTCTCTGGAGTGCCA GATAGGTTCAGTGGCAGCGGGTCAGGGACAG ATTTCACACTGAAAATCAGCCGGGTGGAGGCT GAGGATGTTGGGGTCTATTACTGCATGCAAAG TATACAGGTTCCCTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAATTATATGGTATGATGGAAGT TATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGATCATTAC GATTTTTGGAGTGGGCACTTTGACTACTGGGGCC AGGGAACCCTGGTCACCGTCTCCTCA |
| | | | SEQ ID NO: 26663 | SEQ ID NO: 30669 |

FIGURE 50

| | | | | |
|---|---|---|---|---|
| | | AA | DVVMAQTPLSLSVTPGQPASISCKSSQSLLHGDG KTYLCWYLQKPGQPPQLLIYEVSKRFSGVPDRFS GSGSGTDFTLKISRVEAEDVGVYYCMQSIQVPW TFGQGTKVEIK | QVQLVESGGGVVQPGRSLRLSCAASGFTFSTYGMH WVRQAPGKGLEWVAIIWYDGSYKYYADSVKGRFT ISRDNSKNTLYLQMNSLRAEDTAVYYCARDHYDF WSGHFDYWGQGTLVTVSS |
| | | | SEQ ID NO: 26664 | SEQ ID NO: 30670 |
| IPS:435889 | 21-225_186A11 | NA | GACATCCAGATGACCCAGTCTCCATCTTCCGT GTCTGCATCTGTTGGAGACAGAGTCACCATCA CTTGTCGGGCGAGTCAGGATATTACCAGCTGG TTAGCCTGGTATCAGCAGAAACCAGGGAAAG CCCCTAAACTCCTGATCTATGCTGCATCCAGTT TACAAAGTGGGGTCCCATCAAGGTTCAGCGGC AGTGGATCTGGGACAGATTTCATTCTCACCAT CACCAGCGTGCAGCCTGATGATTTTGCAACTT ACTATTGTCAACAGGTTAACAGTTTCCCATTC ACTTTCGGCCATGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGTTGGGTCCGCCAGGCTCCAGGGAAGGGGCT GGAGTGGGTCTCAGTTATTAGTGGTAGAGGTGGT ACCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAACGGACTGGG GATGATGTTTTTGATATCTGGGGGCCAAGGGACAA TGGTCACCGTCTCTTCA |
| | | | SEQ ID NO: 26665 | SEQ ID NO: 30671 |
| | | AA | DIQMTQSPSSVSASVGDRVTITCRASQDITSWLA WYQQKPGKAPKLLIYAASSLQSGVPSRFSGSGS GTDFILTITSVQPDDFATYYCQQVNSFPFTFGHG TKVDIK | EVQLLESGGGLVQPGGSLRLSCAASGFTFSSYAMS WVRQAPGKGLEWVSVISGRGGTTFYADSVKGRFTI SRDNSKNTLYLQMNSLRAEDTAVYYCAKRTGDDV FDIWGQGTMVTVSS |
| | | | SEQ ID NO: 26666 | SEQ ID NO: 30672 |

EP 4 435 105 A2

FIGURE 50

| iPS:435891 | 21-225_188H5 | NA | GACATCCAGATGACCCAGTCTCCATCCTCACT GTCTGCATCTATAGGAGAGAGAGTCACCATCA CTTGTCGGGCGAGTCAGGGCATTAGCAATTAT TTAGCCTGGCTTCAGCAGAAACCAGGGACAGC CCCTAAGTCCCTGATCTATGCTGCTTCCAGTTT GCAAAGTGGGGTCTCATCAAGGTTCAGCGGCA GTGGATTTGGGACAGATTTCACTCTCACCATC AGTAGTCTGCAGCCTGAAGATTTTGCAACTTA TTACTGCCAACAGTATAATAGTTATCCATTCA CTTTCGGCCCTGGGACCAAAGTGGATATCAAA | CAGGTGCAGCTGGTGCAGTCTGGGGGCTGAGGTG AAGACGCCTGGGGCCTCAGTGAAGGTCTCCTGCA GGGCTTCTGGATACACCTTCACCAGCTACAATAT GCACTGGGTACGACAGGTCCCTGGACAAGGGCT TGAGTGGATGGGATGGATCAACCCTAACAGTGGT GGCTCAAACTATACACAGAAGTTTCAGGGCAGG ATCACCATGACCAGGGACACGTCCATCAGCACA GCCTACATGGAGCTGAGCAGGCTGAGATCTGAC GACACGGCCGTATATTACTGTGCGAGAAAGTTTG GGGACTGGGGCCAGGGAACCCTGGTCACCGTCT CCTCA |
|---|---|---|---|---|
| | | | SEQ ID NO: 26667 | SEQ ID NO: 30673 |
| | | AA | DIQMTQSPSSLSASIGERVTITCRASQGISNYLAW LQQKPGTAPKSLIYAASSLQSGVSSRFSGSGFGT DFTLTISSLQPEDFATYYCQQYNSYPFTFGPGTK VDIK | QVQLVQSGAEVKTPGASVKVSCRASGYTFTSYNM HWVRQVPGQGLEWMGWINPNSGGSNYTQKFQGRI TMTRDTSISTAYMELSRLRSDDTAVYYCARKFGD WGQGTLVTVSS |
| | | | SEQ ID NO: 26668 | SEQ ID NO: 30674 |
| iPS:435895 | 21-225_188E8 | NA | GACATCCAGATGACCCAGTCTCCATCTTCCGT GTCTGCATCTGTAGGAGACAGAGTCACCATCA CTTGTCGGGCGAATCAGGATATTTCCAGCTGG TTAGCCTGGTATCAGCAGAAACCAGGGAAAG CCCCTAAGCTCCTGATCTATGCTGCATCCAATT TGCAAAGTGGGGTCCCATCAGGGTTCAGCGGC AGTGGATCTGGGACAGATTTCACTCTCACCAT CAGCAGCCTGCAGCCTGAAGATTTTGCAACTT ACTATTGTCAGCAGGCTAACAGTTTCCCGTGG ACGTTCGGCCAAGGGACCAAGGTGGAAATCA AG | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTCTGCCAT GAACTGGGTCCGCCAGGCTCCAGGGAAGGGGCT GGAGTGGGTCTCAGTTATTAGTGGTAGTGGTGGT TACACATACTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTTCTGTGCGAAAAGGAACACC GATGATGCTTTTGATATCTGGGGCCAAGGGACAA TGGTCACCGTCTCTTCA |
| | | | SEQ ID NO: 26669 | SEQ ID NO: 30675 |

2250

FIGURE 50

| | | | | |
|---|---|---|---|---|
| | | AA | DIQMTQSPSSVSASVGDRVTITCRANQDISSWLA WYQQKPGKAPKLLIYAASNLQSGVPSGFSGSGS GTDFTLTISSLQPEDFATYYCQQANSFPWTFGQG TKVEIK | EVQLLESGGGLVQPGGSLRLSCAASGFTFSSSAMN WVRQAPGKGLEWVSVISGSGGYTYYADSVKGRFT ISRDNSKNTLYLQMNSLRAEDTAVYFCAKRNTDDA FDIWGQGTMVTVSS |
| | | | SEQ ID NO: 26670 | SEQ ID NO: 30676 |
| IPS:435897 | 21-225_188B9 | NA | GACATCCAGATGACCCAGTCTCCATCCTCACT GTCTGCATCTATAGGAGAGAGAGTCACCATCA CTTGTCGGGCGAGTCAGGGCATTAGCAATTAT TTAGCCTGGCTTCAGCAGAAACCAGGGACAGC CCCTAAGTCCCTGATCTATGCTGCATCCAGTTT GCAAAGTGGGGTCTCATCAAGGTTCAGCGGCA GTGGATTTGGGACAGATTTCACTCTCACCATC AGTAGTCTGCAGCCTGAAGATTTTGCAACTTA TTACTGCCAACAGTATAATAGTTACCCATTCA CTTTCGGCCCTGGGACCAAAGTGGATATCAAA | CAGGTGCAGCTGGTGCAGTCTGGGGGCTGAGGTG AAGACGCCTGGGGCCTCAGTGAAGGTCTCCTGCA GGGCTTCTGGATACACCTTCACCAGCTACAATAT GCACTGGGTGCGACAGGTCCCTGGACAAGGGCT TGAGTGGATGGGATGGATCAACCCTAACAGTGGT GGCTCAAACTATACACAGAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGAGAAAGTTTG GGGACTGGGGCCAGGGAACCCTGGTCACCGTCT CCTCA |
| | | | SEQ ID NO: 26671 | SEQ ID NO: 30677 |
| | | AA | DIQMTQSPSSLSASIGERVTITCRASQGISNYLAW LQQKPGTAPKSLIYAASSLQSGVSSRFSGSGFGT DFTLTISSLQPEDFATYYCQQYNSYPFTFGPGTK VDIK | QVQLVQSGAEVKTPGASVKVSCRASGYTFTSYNM HWVRQVPGQGLEWMGWINPNSGGSNYTQKFQGR VTMTRDTSISTAYMELSRLRSDDTAVYYCARKFGD WGQGTLVTVSS |
| | | | SEQ ID NO: 26672 | SEQ ID NO: 30678 |

FIGURE 50

| iPS:435899 | 21-225_188G11 | NA | GATATTGTGATGACCCAGACTCCACTCTCTCT GTCCGTCACCCCTGGACAGCCGGCCTCCATCT CCTGCATGTCTAGTCAGAGCCTCCTGCATGGT GATGGAAAGACCTATTTATATTGGTACCTGCA GAAGCCCGGCCAGCCTCCACAGCTCCTGATCT ATGAAGTTTCCAACCGGTTCTCTGGAGTGCCA GATACGTTCAGTGGCAGCGGGTCAGGGACAG ATTTCACACTGAAAATCAGCCGGGTGGAGGCT GAGGATGTTGGGGTTTATTACTGCATGCAAAG TATACAGATTCCTTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGACAATTATATGGTATGATGGAAGT TATAAATACTATGCAGACTCCGTGAAGGGCCGAT TCACCATCTCCAGAGACAATTCCAAGAACACGCT GTTTCTGCAAATGAACAGCCTGAGAGCCGAAGA CACGGCTGTGTATTACTGTGCGAGAGAGAGATAC GATTTTTGGAGTGGTCATTTTGACTACTGGGGCC AGGGAACCCTGGTCACCGTCTCCTCA |
| | | | SEQ ID NO: 26673 | SEQ ID NO: 30679 |
| | | AA | DIVMTQTPLSLSVTPGQPASISCMSSQSLLHGDG KTYLYWYLQKPGQPPQLLIYEVSNRFSGVPDTFS GSGSGTDFTLKISRVEAEDVGVYYCMQSIQIPWT FGQGTKVEIK | QVQLVESGGGVVQPGRSLRLSCAASGFTFSSYGMH WVRQAPGKGLEWVTIIWYDGSYKYYADSVKGRFT ISRDNSKNTLFLQMNSLRAEDTAVYYCARERYDF WSGHFDYWGQGTLVTVSS |
| | | | SEQ ID NO: 26674 | SEQ ID NO: 30680 |
| iPS:435901 | 21-225_189G2 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTTTTGGTACCTGCA GAAGCCAGGCCAGCCTCCACAACTCCTGATCT ATGAAGTTTCCAACCGGTTCTCTGGAGTGTCA GATAGGTTCAGTGGCAGCGGGTCAGGGACAG ATTTCACACTGAAAATCAGCCGGGTGGAGGCT GAGGATGTTGGGGTTTACTACTGCATGCAAAG TATACAGATTCCGTGGACGTTCGGCCAAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTATGATGGAAGT TATAAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTATTGTGCGAGAGATCGATTC GATTTTTGGAGTGGTTATTCCGACTACTGGGGCC AGGGAACCCTGGTCACCGTCTCCTCA |
| | | | SEQ ID NO: 26675 | SEQ ID NO: 30681 |

FIGURE 50

| | | | | |
|---|---|---|---|---|
| | | AA | DIVMTQTPLSLSVTPGQPASISCKSSQSLLHGDG KTYLFWYLQKPGQPPQLLIYEVSNRFSGVSDRFS GSGSGTDFTLKISRVEAEDVGVYYCMQSIQIPWT FGQGTKVEIK | QVQLVESGGGVVQPGRSLRLSCAASGFTFSNYGM HWVRQAPGKGLEWVAIIWYDGSYKYYADSVKGR FTISRDNSKNTLYLQMNSLRAEDTAVYYCARDRFD FWSGYSDYWGQGTLVTVSS |
| | | | SEQ ID NO: 26676 | SEQ ID NO: 30682 |
| iPS:435903 | 21-225_190E2 | NA | GACATCGTGATGACCCAGTCTCCAGACTCCCT GGCTGTGTCTCTGGGCGAGAGGGCCACCATCA ACTGCAAGTCCAGCCAGAGTGTTTTATTCAAC TCCAACAATAAGAACTACTTAGCTTGGTACCA GCAGAAACCAGGTCAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCATGCAGG CTGAAGATGTGGCAGTTTATTACTGTCAGCAA TATTGTAGTCTTCCATTCACTTTCGGCCCTGGG ACCAAAGTGGATATCAGA | CAGGTGCAACTGGTGGAGTCTGGGGGAGGCTTG GTCAAGCCTGGAGGGTCCCTGAGACTCTCCTGTG CAGCCTCTGGATTCACCTTCAGTGACTACTACAT GAGCTGGATCCGCCAGGCTCCAGGGAAGGGGCT GGAGTGGCTTTCATACATTAGTAGTAGTGGTACT ACCGTATTCTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGAGAGAATGGGTG GGAGCCGACTACTGGGGCCAGGGAACCCTGGTC ACCGTCTCCTCA |
| | | | SEQ ID NO: 26677 | SEQ ID NO: 30683 |
| | | AA | DIVMTQSPDSLAVSLGERATINCKSSQSVLFNSN NKNYLAWYQQKPGQPPNLLIYWASTRESGVPD RFSGSGSGTDFTLTISSMQAEDVAVYYCQQYCS LPFTFGPGTKVDIR | QVQLVESGGGLVKPGGSLRLSCAASGFTFSDYYMS WIRQAPGKGLEWLSYISSSGTTVFYADSVKGRFTIS RDNAKNSLYLQMNSLRAEDTAVYYCAREWVGAD YWGQGTLVTVSS |
| | | | SEQ ID NO: 26678 | SEQ ID NO: 30684 |

FIGURE 50

| iPS:435905 | 21-225_190A3 | NA | GAAATTATGTTGACGCAGTCTCCAGGCACCCT GTCTTTGTCTCCAGGGGAAAGAGCCACCCTCT CCTGCAGGGCCAGTCAGAATATAAGGAGCAA CTTCTTAGCCTGGTACCAGCAGAAACCTGGCC AGGCTCCCAGGCTCCTCATCTATGGTGCATCC AGCAGGGCCACTGGCATCCCAGACAGATTCAG TGGCAGTGTGTCTGGGACAGACTTCACTCTCA CCATCAACAGACTGGAGCCTGAAGATTTTGCG GTGTATTACTGTCAGCAGTATGGTAACTCACC GTGGACGTTCGGCCAAGGGACCAAGGTGGAA ATCAAA | CAGGTGCAGCTGCAGGAGTCGGGCCCAGGACTG GTGAAGCCTTCACAGACCCTGTCCCTCACCTGCA CTGTCTCTGGTGGCTCCATCAACAGTGGTGGTTA CTACTGGAACTGGATCCGCCAGCACCCAGGGAA GGGCCTGGAGTGGATTGGGTTCATCTTTTATAGT GGGAGCACCTACTACAACCCGTCCCTCAAGAGTC GAGTTACCATATCAGTAGACACGTCTAAGAACCA GTTCTCCCTGAAGCTGAACTCTGTGACTGCCGCG GACACGGCCGTGTATTACTGTGCGAGAGGGGATT ACGATGGTTCGGGGAGTTATCACTACTATTACGG TATGGACGTCTGGGGCCAAGGGACCACGGTCAC CGTCTCCTCA |
| | | | SEQ ID NO: 26679 | SEQ ID NO: 30685 |
| | | AA | EIMLTQSPGTLSLSPGERATLSCRASQNIRSNFLA WYQQKPGQAPRLLIYGASSRATGIPDRFSGSVSG TDFTLTINRLEPEDFAVYYCQQYGNSPWTFGQG TKVEIK | QVQLQESGPGLVKPSQTLSLTCTVSGGSINSGGYY WNWIRQHPGKGLEWIGFIFYSGSTYYNPSLKSRVTI SVDTSKNQFSLKLNSVTAADTAVYYCARGDYDGS GSYHYYYGMDVWGQGTTVTVSS |
| | | | SEQ ID NO: 26680 | SEQ ID NO: 30686 |
| iPS:435907 | 21-225_190G3 | NA | GACATCCAGATGACCCAGTCTCCATCCTCCCT GTCTGCATCTGTAAGAGACAGAGTCACCATCA CTTGCCGGGCAAGTCAGGGCATTAGAAAAGAT TTAGGCTGGTATCAGCAGAAACCAGGGAAAG CCCCTAAGCGCCTGATCCATACTGCATCCAGT TTGCAAAGTGGGGTCCCATTAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGATGGAGGT TATAAAAACTATGTAGACTCCGTGAAGGGCCGAT TCACCATCTCCAGAGACAATTCCAAGAACACGCT GTATCTGCAAATGAATAGCCTGAGAGCTGAGGA CACGGCTGTGTATTACTGTGCGAGAGGTACCCAC GGGTACTACTACGGTGTGGACGTCTGGGGCCAA GGGACCACGGTCACCGTCTCCTCA |
| | | | SEQ ID NO: 26681 | SEQ ID NO: 30687 |

FIGURE 50

| | | | | |
|---|---|---|---|---|
| | | AA | DIQMTQSPSSLSASVRDRVTITCRASQGIRKDLGWYQQKPGKAPKRLIHTASSLQSGVPLRFSGSGSGTEFTLTISSLQPEDFATYYCLQHNSYPWTFGQGTKVEIK | QVQLVESGGGVVQPGRSLRLSCAASGFTFSSYGMHWVRQAPGKGLEWVAVISYDGGYKNYVDSVKGRFTISRDNSKNTLYLQMNSLRAEDTAVYYCARGTHGYYYGVDVWGQGTTVTVSS |
| | | | SEQ ID NO: 26682 | SEQ ID NO: 30688 |
| iPS:435909 | 21-225_190H3 | NA | GACATCCAGATGACCCAGTCTCCATCTTCCGTGTCTGCATCTGTAGGAGACAGAGTCACCATCACTTGTCGGGCGAGTCAGGGTCTTAACAACTGGTTAGCCTGGTATCAGCTGAAACCAGGGAAAGCCCCTAAGCTCCTGATCTATGCTGTGTCCAGTTTGCAAAGTGGGGTCCCATCAAGGTTCAGCGGCAGTGGATCTGGGTCAGAGTTCACTCTCACCATCAGCAGCCTGCAGCCTGAAGATTTTGCAACTTACCATTGTCAACAGGCTAACAGTCTCCCGTGGACGTTCGGCCAAGGGACCAAGGTGGAAATCAAA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATACTACGCAGACTCCGTGAAGGGCCGGTTCACCATCTCCAGAGACAATTCCAAGAACACGCTGTATCTGCAAATGAACAGTTTGAGAGCCGAGGACACGGCCGTATATTACTGTGCGAAAGATGGATTCGGTGGGGAGCTCCTATTTTGACTACTGGGGCCAGGGAACCCTGGTCACCGTCTCCTCA |
| | | | SEQ ID NO: 26683 | SEQ ID NO: 30689 |
| | | AA | DIQMTQSPSSVSASVGDRVTITCRASQGLNNWLAWYQLKPGKAPKLLIYAVSSLQSGVPSRFSGSGSGSEFTLTISSLQPEDFATYHCQQANSLPWTFGQGTKVEIK | EVQLLESGGGLVQPGGSLRLSCAASGFTFSSYAMSWVRQAPGKGLEWVSAISGRGGNTYYADSVKGRFTISRDNSKNTLYLQMNSLRAEDTAVYYCAKDGFGGSSYFDYWGQGTLVTVSS |
| | | | SEQ ID NO: 26684 | SEQ ID NO: 30690 |

FIGURE 50

| iPS:435911 | 21-225_190B4 | NA | GAAATTGTGTTGACGCAGTCTCCAGGCACCCT GTCTTTGTCTCCAGGGGAAAGAGCCACACTCT CCTGCAGGGCCAGTCAGAGTATTCGCAGCAGC TTCTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAACTCACCG TGGGCGTTCGGCCAAGGGACCAAGGTGGAAA TCAAA<br><br>SEQ ID NO: 26685 | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGATCCGCCAGCACCCAGGGAA GGGCCTGGAGTGGATTGGGTACATCTTTTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTATATTACTGTGCGAGAGGGGATT ACGATGGTTCGGGGAGTTATCACTACTACTACGG TATGGACGTCTGGGGCCAAGGGACCACGGTCAC CGTCTCCTCA<br><br>SEQ ID NO: 30691 |
| | | AA | EIVLTQSPGTLSLSPGERATLSCRASQSIRSSFLA WYQQKPGQAPRLLIYGASSRATGIPDRFSGSGSG TDFTLTISRLEPEDFAVYYCQQYGNSPWAFGQG TKVEIK<br><br>SEQ ID NO: 26686 | QVQLQESGPGLVKPSQTLSLTCTVSGGSISSGDYYW NWIRQHPGKGLEWIGYIFYSGSTYYNPSLKSRVTIS VDTSKNQFSLKLSSVTAADTAVYYCARGDYDGSG SYHYYYGMDVWGQGTTVTVSS<br><br>SEQ ID NO: 30692 |
| iPS:435913 | 21-225_190A7 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGAAGCAAC TTCTTAGCCTGGCACCAGCAGAAACCTGGCCA GGCTCCCAGGCTCCTCATCTATGGTGCATACC GCAGGGCCACTGGCATCCCAGACAGGTTCAGT GGCAGTGGGTCTGGGACAGACTTCACTCTCAC CATCAACAGACTGGAGCCTGAAGATTTTGCAG TGTATTACTGTCAGCAGTATGGTAACTCACCG TGGACGTTCGGCCAAGGGACCAAGGTGGAAA TCAAA<br><br>SEQ ID NO: 26687 | CAGGTGCAGCTGCAGGAGTCGGGCCCAGGACTG GTGAACCCTTCACAGACCCTGTCCCTCACCTGCA CTGTCTCTGGTGGCTCCATCAGCAGTGGTGTTTA CTACTGGAGCTGGATCCGCCAGCACCCAGGGAA GGGCCTGGAGTGGATTGGGAACATCTATTACAGT GGGAGCACCTACAACAACCCGTCCCTCAAGAGT CGAATTATCATATCAGTAGACACGTCTAAGAACC AGTTCTCCCTGAAGCTGAGCTCTGTGACTGCCGC GGACACGGCCGTGTATTACTGTGCGAGAGGGGA TTACGATGGTTCGGGGAGTTATCACTACTACTAC GGTTTGGACGTCTGGGGCCAAGGGACCACGGTC ACCGTCTCCTCA<br><br>SEQ ID NO: 30693 |

FIGURE 50

| | | | | |
|---|---|---|---|---|
| | | AA | EIVLTQSPGTLSLSPGERATLSCRASQSVRSNFLA WHQQKPGQAPRLLIYGAYRRATGIPDRFSGSGS GTDFTLTINRLEPEDFAVYYCQQYGNSPWTFGQ GTKVEIK | QVQLQESGPGLVNPSQTLSLTCTVSGGSISSGVYYW SWIRQHPGKGLEWIGNIYYSGSTYNNPSLKSRIIISV DTSKNQFSLKLSSVTAADTAVYYCARGDYDGSGS YHYYYGLDVWGQGTTVTVSS |
| | | | SEQ ID NO: 26688 | SEQ ID NO: 30694 |
| iPS:435915 | 21-225_190H4 | NA | GCCAACGTGATGACCCAGTCTCCAGACTCCCT GGCTGTGTCTCTGGGCGAGAGGACCACCATCA ACTGCAAGTCCAGCCAGAGTGTTTTACACAGC TCCAACAATTACAACTACTTAGCTTGGTACCG GCAGAAACCAGGACAGCCTCCTAAGCTGCTCA TTTACTGGGCATCTACCCGGGAATCCGGGGTC CCTGACCGATTCAGTGGCAGCGGGTCTGGTAC AGATTTCACTCTCACCATCAGCAGCCTGCAGG CTGAAGATGTGGCAGTTTATTACTGTCAGCAA TATTATAGTATTCCTCCCACTTTCGGCCCTGGG ACCAAAGTGGATATCAAA | CAGGTGCAGCTGGTGCAGTCTGGGGGCTGAGGTG AAGAAGCCTGGGGCCTCAGTGAAGGTCTCCTGC AAGGCTTCTGGAAACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCCCATAGCAG TGGCTGGTACATCTTTGACTACTGGGGCCAGGGA ACCCTGGTCACCGTCTCCTCA |
| | | | SEQ ID NO: 26689 | SEQ ID NO: 30695 |
| | | AA | ANVMTQSPDSLAVSLGERTTINCKSSQSVLHSSN NYNYLAWYRQKPGQPPKLLIYWASTRESGVPD RFSGSGSGTDFTLTISSLQAEDVAVYYCQQYYSI PPTFGPGTKVDIK | QVQLVQSGAEVKKPGASVKVSCKASGNTFTNYDIN WVRQATGQGLEWMGWMNPNSGNTGYAQKFQGR VTMTRNTSISTAYMELSSLRSEDTAVYYCAHSSGW YIFDYWGQGTLVTVSS |
| | | | SEQ ID NO: 26690 | SEQ ID NO: 30696 |

FIGURE 50

| iPS:435917 | 21-225_190D5 | NA | GAAATTGTGCTGACTCAGTCTCCAGACTTTCAGTCTGTGGCTCCAAAGGAGAAAGTCACCATCACCTGCCGGGCCAGTCAGAGTATTGGTAGTAACTTACACTGGTACCAGCAGAAACCTGATCAGTCTCCAAAGCTCCTCATCAAGTCTGCTTCCCAGTCCTTCTCAGGGGTCCCCTCGAGGTTCAGTGGCAGTGGATCTGGGACAGATTTCACCCTCACCATCAATAGCCTGGAAACTGAAGATGCTGCAACGTATTACTGTCAGCAGAGTAGTAGTTTACCGTGGACGTTCGGCCAAGGGACCAAGGTGGAAATCAAA | CAGGTGCAGCTGCAGGAGTCGGGCCCAGGACTGGTGAAGCCTTCGGAGACCCTGTCCCTCACCTGCACTGTCTCTGGTGGCTCCATCAATAATTACTACTGGAGCTGGATCCGGCAGCCCGCCGGGAAGGGACTGGAGTGGATTGGGCGTATCTATGCCAGTGGGAGCACCAACTACAACCCCTCCCTCAAGAGTCGAGTCACCATGTCAATAGACACGTCCAAGAACCAGTTCTCCCTGAAGCTGAGCTCTGTGACCGCCGCGGACACGGCCGTGTATTACTGTGCGAGAGATCGGGGGATACTATGGCTACTACGGTATGGACGTCTGGGGCCAAGGGACCACGGTCACCATCTCCTCA |
| | | | SEQ ID NO: 26691 | SEQ ID NO: 30697 |
| | | AA | EIVLTQSPDFQSVAPKEKVTITCRASQSIGSNLHWYQQKPDQSPKLLIKSASQSFSGVPSRFSGSGSGTDFTLTINSLETEDAATYYCQQSSSLPWTFGQGTKVEIK | QVQLQESGPGLVKPSETLSLTCTVSGGSINNYYWSWIRQPAGKGLEWIGRIYASGSTNYNPSLKSRVTMSIDTSKNQFSLKLSSVTAADTAVYYCARDRGYYGYYGMDVWGQGTTVTISS |
| | | | SEQ ID NO: 26692 | SEQ ID NO: 30698 |
| iPS:435919 | 21-225_190H5 | NA | GACATCCAGATGACCCAGTCTCCATCCTCCCTGTCTGCATCTGTAGGAGACAGAGTCACCATCACTTGCCGGGCAAGTCAGGGCATTAGAAAAGATTTAGGCTGGTATCAGCAGAAACCAGGGAAAGCCCCTAAGCGCCTGATCCATACTGCATCCAGTTTGCAAAGTGGGGTCCCATTAAGGTTCAGCGGCAGTGGATCTGGGACAGAATTCACTCTCACAATCAGCAGCCTGCAGCCTGAAGATTTTGCAACTTATTACTGTCTACAGCATAATAATTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGATGGAGGTTATAAAAACTATGCAGACTCCGTGAAGGGCCGATTCACCATCTCCAGAGACAATTCCAAGAACACGCTGTATCTGCAAATGAATAGCCTGAGAGCTGAGGACACGGCTGTGTATTACTGTGCGAGAGGTACCCACGGGTACTACTACGGTGTGGACGTCTGGGGCCAAGGGACCACGGTCACCGTCTCCTCA |
| | | | SEQ ID NO: 26693 | SEQ ID NO: 30699 |

2258

FIGURE 50

| | | | | |
|---|---|---|---|---|
| | | AA | DIQMTQSPSSLSASVGDRVTITCRASQGIRKDLG WYQQKPGKAPKRLIHTASSLQSGVPLRFSGSGS GTEFTLTISSLQPEDFATYYCLQHNNYPWTFGQG TKVEIK | QVQLVESGGGVVQPGRSLRLSCAASGFTFSSYGMH WVRQAPGKGLEWVAVISYDGGYKNYADSVKGRF TISRDNSKNTLYLQMNSLRAEDTAVYYCARGTHG YYYGVDVWGQGTTVTVSS |
| | | | SEQ ID NO: 26694 | SEQ ID NO: 30700 |
| iPS:435921 | 21-225_190D6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCTGAAACCAGGGAAAGC CCCTAAGCGCCTGATTTATGCTGCATCCAGTTT GCAAAGTGGGGTCCCATCAAGGTTCAGCGGCA GTGGATCTGGGCCAGAATTCACTCTCACAATC AGCAGCCTGCAGCCTGAAGATTTTGCAACTTA TTACTGTCTACAGCATTATAGTTTCCCATTCAC TTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTTTATCAT GCACTGGGTCCGCCAGGCTCCAGGCAGGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGAGTA TAGCAGTGGCTGGTTCGGGTACGGTATGGACGTC TGGGGCCAAGGGACCACGGTCACCGTCTCCTCA |
| | | | SEQ ID NO: 26695 | SEQ ID NO: 30701 |
| | | AA | DIQMTQSPSSLSASVGDRVTITCRASQGIRNDLG WYQLKPGKAPKRLIYAASSLQSGVPSRFSGSGSG PEFTLTISSLQPEDFATYYCLQHYSFPFTFGPGTK VDIK | QVQLVESGGGVVQPGRSLRLSCAASGFTFSSFIMH WVRQAPGRGLEWVAVIWYDGSNKYYADSVKGRF TISRDNSKNTLYLQMNSLRAEDTAVYYCAREEYSS GWFGYGMDVWGQGTTVTVSS |
| | | | SEQ ID NO: 26696 | SEQ ID NO: 30702 |

FIGURE 50

| iPS:435923 | 21-225_190H6 | NA | GACATCGTGATGACCCAGTCTCCAGACTCCCT GGCTGTGTCTCTGGGCGAGAGGGCCACCATCA ACTGCAAGTCCAGCCAGAGTGTTTTATTCAAC TCCAACAATAAGAACTACTTAGCTTGGTACCA GCAGAAACCAGGTCAGCCTCCTAACCTGCTCA TTTACTGGGCATCTACCCGGGAATCCGGGGTC CCTGACCGATTCAGTGGCAGCGGGTGTGGGAC AGATTTCACTCTCACCATCAGCAGCCTGCAGG CTGAAGATGTGGCAGTTTATTACTGTCAGCAA TATTGTAGTCTTCCATTCACTTTCGGCCCTGGG ACCAAAGTGGATATCAGA | CAGGTGCAACTGGTGGAGTCTGGGGGAGGCTTG GTCAAGCCTGGAGGGTCCCTGAGACTCTCCTGTG CAGCCTCTGGATTCACCTTCAGTGACTACTACAT GAGCTGGATCCGCCAGGCTCCAGGGAAGGGGCT GGAGTGGCTTTCATACATTAGTAGTAGTGGTACT ACCGTATTCTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGAGAGAATGGGTG GGAGCCGACTACTGGGGCCAGGGAACCCTGGTC ACCGTCTCCTCA |
| | | | SEQ ID NO: 26697 | SEQ ID NO: 30703 |
| | | AA | DIVMTQSPDSLAVSLGERATINCKSSQSVLFNSN NKNYLAWYQQKPGQPPNLLIYWASTRESGVPD RFSGSGCGTDFTLTISSLQAEDVAVYYCQQYCSL PFTFGPGTKVDIR | QVQLVESGGGLVKPGGSLRLSCAASGFTFSDYYMS WIRQAPGKGLEWLSYISSSGTTVFYADSVKGRFTIS RDNAKNSLYLQMNSLRAEDTAVYYCAREWVGAD YWGQGTLVTVSS |
| | | | SEQ ID NO: 26698 | SEQ ID NO: 30704 |
| iPS:435925 | 21-225_190D7 | NA | GACATCGTGATGACCCAGTCTCCAGACTCCCT GGCTGTGTCTCTGGGCGAGAGGGCCACCATCA ACTGCAAGTCCAGCCAGAGTGTTTTATCCAGC TCCAACAATTACAACTATTTAGTTTGGTATCA GCAGAAACCAGGACAGCCTCCTAAGCTGCTCA TTTACTGGGCATCTACCCGGAAATCCGGGGTC CCTGACCGATTCAGTGGCAGCGGGTCTGGGAC AGATTTTACTCTCACCATCAGCAGCCTGCAGG CTGATGACGTGGCAGTTTATTACTGTCAACAA TATTATCGTACTCCGTGGACGTTCGGCCAAGG GACCAAGGTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAATAGTGG TAATACAGGCTATGCACAGAAGTTCCAGGGCAG AGTCACCATGACCAGGAATACCTCCATAAGCAC AGCCTACATGGAGCTGAGCAGCCTGAGATCTGA GGACACGGCCGTGTATTACTGTGCGAGTAGCAGT GGCTGGTACTTCTTTGACTACTGGGGCCAGGGAA CCCTGGTCACCGTCTCCTCA |
| | | | SEQ ID NO: 26699 | SEQ ID NO: 30705 |

FIGURE 50

| | | | | |
|---|---|---|---|---|
| | | AA | DIVMTQSPDSLAVSLGERATINCKSSQSVLSSSN NYNYLVWYQQKPGQPPKLLIYWASTRKSGVPD RFSGSGSGTDFTLTISSLQADDVAVYYCQQYYR TPWTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCASSSGW YFFDYWGQGTLVTVSS |
| | | | SEQ ID NO: 26700 | SEQ ID NO: 30706 |
| iPS:435927 | 21-225_190E7 | NA | GATATTGTGTTGACCCAGACTCCACTCTCTCTG TCCGTCACCCCTGGACAGCCGGCCTCCATCTC CTGCAAGTCTAGTCAGAGCCTCCTCCATAGTG ATGGAAGGACCTATTTGTATTGGTACCTGCAG AAACCAGGCCAGCCTCCACAGGTCCTGATCTG TGAGGTTTCCAACCGGTTCTCTGGAGTGCCAG ATAGGTTCAGTGGCAGCGGGTCAGGGACAGA TTTCACACTGAAAATCAGCCGGGTGGAGGCTG GAGATGTTGGGGTTTATTACTGCATGCAAAGT ATACAGCTTCCCTGGACGTTCGGCCAAGGGAC CAAGGTGGAAATCAAA | CAGCTGCAGCTGCAGGAGTCGGGCCCAGGACTG GTGAAGCCTTCGGAGACCCTGTCCCTCACCTGCA CTGTCTCTGGTGGCTCCATCAGAAGTTACCACTG GAGTTGGATCCGGCAGCCCGCCGGGAAGGGACT GGAGTGGATTGGGCATATCTATACCAGTAGGAGC ACCAACTACAACCCCTCCCTCAAGAGTCGAGTCA CCATTTCAGTAGACACGTCCAAGAACCAGTTCTC CCTGAAGCTGAGCTCTGTGACCGCCACGGACACG GCCGTGTATTACTGTGCGAGACTCCGGTATAACT GGAACTTCCCTTACTTTGACTACTGGGGCCAGGG AACCCTGGTCACCGTCTCCTCA |
| | | | SEQ ID NO: 26701 | SEQ ID NO: 30707 |
| | | AA | DIVLTQTPLSLSVTPGQPASISCKSSQSLLHSDGR TYLYWYLQKPGQPPQVLICEVSNRFSGVPDRFS GSGSGTDFTLKISRVEAGDVGVYYCMQSIQLPW TFGQGTKVEIK | QLQLQESGPGLVKPSETLSLTCTVSGGSIRSYHWSW IRQPAGKGLEWIGHIYTSRSTNYNPSLKSRVTISVDT SKNQFSLKLSSVTATDTAVYYCARLRYNWNFPYFD YWGQGTLVTVSS |
| | | | SEQ ID NO: 26702 | SEQ ID NO: 30708 |

FIGURE 50

| iPS:435929 | 21-225_190D9 | NA | GAAATTGTGCTGACTCAGTTTCCAGACTCTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCACCCTCACCAT CAATAGCCTGGAAGCTGAAGATGCTGCAACAT ATTACTGTCATCAGAGTAGTAGTTTCCCTCGG ACGTTCGGCCAAGGGACCAAGGTGGAAATCA AA | GAGGTGCAGCTGTTGGACTCTGGGGGAGGCTTGG GACAGCCTGGGGGGTCCCTGAGACTCTCCTGTGC AGCCTCTGGATTCACCTTTGACAGCTATGCCATG AGTTGGGTCCGCCAGGCTCCAGGGAAGGGGCTG GAGTGGGTCTCAACTATTAGTGGTACTGGTCGTA GGACATACTACGCAGACTCCGTGAAGGGCCGGT TCACCATCTCCAGAGACAATTCCAAGAACACGCT GTATCTGCAAATGAATAGCCTGAGAGCCGAGGA CACGGCCGTATATTACTGTGCGAAAGAGGAGGA TTACTATGATAGTAGTGGCCCGGGGTTCGACCCC TGGGGCCAGGGAACCCTGGTCACCGTCTCCTCA |
| | | | SEQ ID NO: 26703 | SEQ ID NO: 30709 |
| | | AA | EIVLTQFPDSQSVTPKEKVTITCRASQSIGSSLHW YQQKPDQSPKLLIKYASQSFSGVPSRFSGSGSGT DFTLTINSLEAEDAATYYCHQSSSFPRTFGQGTK VEIK | EVQLLDSGGGLGQPGGSLRLSCAASGFTFDSYAMS WVRQAPGKGLEWVSTISGTGRRTYYADSVKGRFTI SRDNSKNTLYLQMNSLRAEDTAVYYCAKEEDYYD SSGPGFDPWGQGTLVTVSS |
| | | | SEQ ID NO: 26704 | SEQ ID NO: 30710 |
| iPS:435933 | 21-225_190F8 | NA | GACATCCAGATGACCCAGTCTCCTTCCTCACT GTCTGCATCTGTAGGAGACAGAGTCACCATCA CTTGTCGGACGAGTCAGGGCATTGGCAATTAT TTAGCCTGGTTTCAGCAGAAACCAGGGAAAGC CCCTAAGTCCCTACTCTATAAAGCATCCAGTTT ACAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATATGACTTACCCACTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTACTTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGGCCAA GGGACCACGGTCACCGTCTCCTCA |
| | | | SEQ ID NO: 26705 | SEQ ID NO: 30711 |

FIGURE 50

| | | | | |
|---|---|---|---|---|
| | | AA | DIQMTQSPSSLSASVGDRVTITCRTSQGIGNYLA WFQQKPGKAPKSLLYKASSLQSGVPSKFSGSGS GTDFTLTISSLQPEDFATYYCQQYMTYPLTFGGG TKVEIK | QVQLVESGGGVVQPGRSLRLSCAASGFTFSTYGMH WVRQAPGKGLEWVAVIWYDGSNKNYADSVKGRF TISRDNSKNTLYLQMNSLRAEDTAVYYCARDQGV GYDGLDVWGQGTTVTVSS |
| | | | SEQ ID NO: 26706 | SEQ ID NO: 30712 |
| iPS:435935 | 21-225_190H8 | NA | GAAATTGTGCTGACTCAGTTTCCAGACTCTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCACCCTCACCAT CAATAGCCTGGAAGCTGAAGATGCTGCAACGT ATTACTGTCATCAGAGTAGTAGTTTCCCTCGG ACGTTCGGCCAAGGGACCAAGGTGGAAATCA AA | GGGGTACAACTGTTGGACTCTGGGGGAGGCTTGG GACAGCCTGGGGGGGTCCCTGAGACTCTCCTGTGC AGCCTCTGGATTCACCTTTGACAGCTATGCCATG AGCTGGGTCCGCCAGGCTCCAGGGAAGGGGCTG GAGTGGGTCTCAACTATTAGTGGTACTGGTCGTA GGACATATTACGCAGACTCCGTGAAGGGCCGGTT CACCATCTCCAGAGACAATTCCAAGAACACACTG TATCTGCAGATGAATAGCCTGAGAGCCGAGGAC ACGGCCGTATATTACTGTGCGAAAGAGGAGGATT ACTATGATAGTAGTGGCCCCGGGGGTTCGACCCCTG GGGCCAGGGAACCCTGGTCACCGTCTCCTCA |
| | | | SEQ ID NO: 26707 | SEQ ID NO: 30713 |
| | | AA | EIVLTQFPDSQSVTPKEKVTITCRASQSIGSSLHW YQQKPDQSPKLLIKYASQSFSGVPSRFSGSGSGT DFTLTINSLEAEDAATYYCHQSSSFPRTFGQGTK VEIK | GVQLLDSGGGLGQPGGSLRLSCAASGFTFDSYAMS WVRQAPGKGLEWVSTISGTGRRTYYADSVKGRFTI SRDNSKNTLYLQMNSLRAEDTAVYYCAKEEDYYD SSGPGFDPWGQGTLVTVSS |
| | | | SEQ ID NO: 26708 | SEQ ID NO: 30714 |

FIGURE 50

| iPS:435937 | 21-225_190H9 | NA | GACATCCAGATGACCCAGTCTCCATCCTCACT GTCTGCATCTGTGGGAGACAGAGTCACCATCA CTTGTCGGGCGAGTCAGGGCATTGGCAAGTAT TTAGCCTGGTTTCAGCAGAAACCAGGGAAAGC CCTTAAGTCACTGATCTATGCTGCATCCAGTTT GCAAAGTGGGGTCCCATCAAAATTCAGCGGCA GTGGATCTGGGACAGATTTCACTCTCACCATC AGCAGCCTGCAGCCTGACGATTTTGCAACTTA TTACTGCCAACGATATGATACTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGGAACTATGGCAT GCACTGGGTCCGCCAGGCTCCAGGCAAGGGGCT GGAGTGGGTGGCAGTTATATGGTTTGATGGAAGT AATGACTACTATGCAGACTCCGTGAAGGGCCGAT TCACCATCTCCAGAGACAATTCCAAGAACACGCT GTCACTGCAAATGAACAGCCTGAGAGCCGAGGA CACGGCTGTGTATTACTGTGCGAGAGATAGAAGC GTCGGCTACGACGGTTTAGATGTCTGGGGCCAAG GGACCACGGTCACCGTCTCCTCA |
| | | | SEQ ID NO: 26709 | SEQ ID NO: 30715 |
| | | AA | DIQMTQSPSSLSASVGDRVTITCRASQGIGKYLA WFQQKPGKALKSLIYAASSLQSGVPSKFSGSGSG TDFTLTISSLQPDDFATYYCQRYDTYPFTFGPGT KVDIK | QVQLVESGGGVVQPGRSLRLSCAASGFTFRNYGM HWVRQAPGKGLEWVAVIWFDGSNDYYADSVKGR FTISRDNSKNTLSLQMNSLRAEDTAVYYCARDRSV GYDGLDVWGQGTTVTVSS |
| | | | SEQ ID NO: 26710 | SEQ ID NO: 30716 |
| iPS:435939 | 21-225_191H7 | NA | GAAATTGTGTTGACGCAGTCTCCAGGCACCCT GTCTTTGTCTCCAGGGGAAAGCGCCACCCTCT CCTGCAGGGCCGGTCAAAGTATTAGAACCGAC TTCTTAGCCTGGTACCAGCAGCAACCTGGCCA GGCTCCCAGGCTCCTCATCTATGGTCCATCCA GCAGGGCCACTGGCATCCCAGACAGGTTCAGT GGCAGTGGGTCTGGGACAGACTTCACTCTCAC CATCAACAGACTGGAGCCTGAAGATTTTGCAG TGTATTTCTGTCAGCAGTATGGTAGCTCACCGT GGACGTTCGGCCAAGGGACCAAGGTGGAAAT CAAT | CAGGTGCAACTGCAGGAGTCGGGGCCCAGGACTG GTGAAGCCTTCACAGACCCTGTCCCTCACCTGCA CTGTCTCTGGTGGCTCCATCAACAGTGGTGATTA CTACTGGAACTGGATCCGCCAGCACCCAGGGAA GGGGCCTGGAGTGGATTGGGTACATCTTTTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGGGGATT ACGATGGTTCGGGGAGTTATCACTACTATTACGG TATGGACGTCTGGGGCCAAGGGACCACGGTCAC CGTCTCCTCA |
| | | | SEQ ID NO: 26711 | SEQ ID NO: 30717 |

FIGURE 50

| | | | | |
|---|---|---|---|---|
| | | AA | EIVLTQSPGTLSLSPGESATLSCRAGQSIRTDFLA WYQQQPGQAPRLLIYGPSSRATGIPDRFSGSGSG TDFTLTINRLEPEDFAVYFCQQYGSSPWTFGQGT KVEIN | QVQLQESGPGLVKPSQTLSLTCTVSGGSINSGDYY WNWIRQHPGKGLEWIGYIFYSGSTYYNPSLKSRVTI SVDTSKNQFSLKLSSVTAADTAVYYCARGDYDGS GSYHYYYGMDVWGQGTTVTVSS |
| | | | SEQ ID NO: 26712 | SEQ ID NO: 30718 |
| iPS:435941 | 21-225_191E8 | NA | GAAATAGTGATGACGCAGTCTCCAGCCACCCT GTCTGTGTCTCCAGGGGAAAGAGCCACCCTCT CCTGCAGGCCCAGTCAGAGTTTTAGCAGAAAC TTAGCCTGGTACCAGCAGAAACCTGGCCAGGC TCCCAGGCTCCTCATCTATGGTGCATCCACTA GGGCCACTGGTATCCCATCCAGGTTCAGTGGC AGTGGGTCTGGGACAGAGTTCACTCTCACCAT CAGCAGCCTGGAGTCTGAAGATTTTGCAGTTT ACTACTGTCAGCAGTATAATAACTGGCCGCTC ACTTTCGGCGGAGGGATCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTTTGATGGAAGT AATCAATACTATGCCGACTCCGTGAAGGGCCGAT TCACCATCTCCAGAGACAATTCCAAGAACACGCT GTATCTGCAATTGAACAGCCTGAGAGCCGAGGA CACGGCTGTCTATTACTGTGCGAGAGCCCACGGG GTCTACTACTACGCTATGGACGTCTGGGGCCAAG GGACCACGGTCACCGTCTCCTCA |
| | | | SEQ ID NO: 26713 | SEQ ID NO: 30719 |
| | | AA | EIVMTQSPATLSVSPGERATLSCRPSQSFSRNLA WYQQKPGQAPRLLIYGASTRATGIPSRFSGSGSG TEFTLTISSLESEDFAVYYCQQYNNWPLTFGGGI KVEIK | QVQLVESGGGVVQPGRSLRLSCAASGFTFSNYGM HWVRQAPGKGLEWVAIIWFDGSNQYYADSVKGRF TISRDNSKNTLYLQLNSLRAEDTAVYYCARAHGVY YYAMDVWGQGTTVTVSS |
| | | | SEQ ID NO: 26714 | SEQ ID NO: 30720 |

FIGURE 50

| | | | | |
|---|---|---|---|---|
| iPS:435943 | 21-225_191C9 | NA | GAAATTGTGCTGACTCAGTCTCCAGACTTTCA GTCTGTGACTCCAAAGGAGAAAGTCACCATCA CCTGCCGGGCCAGTCAGAGTATTGGTAGTAGT TTACACTGGTACCAGCAGAAACCAGATCAGTC TCCAAAGCTCCTCATCAAGTATGCTTCCCAGT CCTTCTCAGGGGACCCCTCGAGGTTCAGTGGC AGTGGATCTGGGACAGATTTCACCCTCACCAT CAATAGCCTGGAAGCTGAAGATGCTGCAACGT ATTACTGTCATCAGACTAGAAGTTTACCTCTC ACTTTCGGCGGAGGGACCAAGGTGGAGATCA AA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAACTGGATCCGCCAGCACCCAGGGAA GGGCCTGGATTGGATTGGATATATCTATTACAGT GGGAGCACCTACTACAACCCGTCCCTCAAGAGTC GAGTTACCATATCAGTAGACACGTCTAAGAACCA GTTCTCCCTGAAGCTGAACTCTGTGACTGCCGCG GACACGGCCGTGTATTACTGTGCGAGATCCGGGT ATAATTGGGACGCCGGGGTCGACCCCTGGGGCC AGGGAACCCTGGTCACCGTCTCCTCA |
| | | | SEQ ID NO: 26715 | SEQ ID NO: 30721 |
| | | AA | EIVLTQSPDFQSVTPKEKVTITCRASQSIGSSLHW YQQKPDQSPKLLIKYASQSFSGDPSRFSGSGSGT DFTLTINSLEAEDAATYYCHQTRSLPLTFGGGTK VEIK | QVQLQESGPGLVKPSQTLSLTCTVSGGSISSGGYYW NWIRQHPGKGLDWIGYIYYSGSTYYNPSLKSRVTIS VDTSKNQFSLKLNSVTAADTAVYYCARSGYNWDA GVDPWGQGTLVTVSS |
| | | | SEQ ID NO: 26716 | SEQ ID NO: 30722 |
| iPS:435945 | 21-225_191A10 | NA | GACATCCAGATGACCCAGTCTCCATCCTCACT GTCTGCATCTGTAGGAGACAGAGTCACCATCA CTTGTCGGGCGAGTCAGGGCATTGGCAAGTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATATGGGACAGATTTCACTCTCACCATC AGCAGCCTGCAGCCTGAAAATTTTGCAATTTA TTACTGCCAACAGTATAGTACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTACGCAGACTCCGTGAAGGGCCGA TTCACCATCTCCAGAGACAATTCCAAGAACACGC TGTATCTGCAAATGAACAGCCTGAGAGCCGAGG ACACGGCTATGTATTACTGTGCGAGAGATCAGGG CGTGGGCTACGACGGTTTGGACGTCTGGGGCCAA GGGACCTCGGTCACCGTCTCCTCA |
| | | | SEQ ID NO: 26717 | SEQ ID NO: 30723 |

FIGURE 50

| | | | | |
|---|---|---|---|---|
| | | AA | DIQMTQSPSSLSASVGDRVTITCRASQGIGKYLA WFQQKPGKAPKSLIYAASSLQSGVPSKFSGSGY GTDFTLTISSLQPENFAIYYCQQYSTYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAMYYCARDQGV GYDGLDVWGQGTSVTVSS |
| | | | SEQ ID NO: 26718 | SEQ ID NO: 30724 |
| iPS:435947 | 21-225_191E10 | NA | GACATCCAGATGACCCAGTCTCCATCCTCACT GTCTGCATCTGTAGGAGACAGAGTCACCATCA CTTGTCGGGCGAGTCAGGGCATTGGCAAGTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAAATTTTGCAACTTA TTACTGCCAACAGTATAGTACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTACGCAGACTCCGTGAAGGGCCGA TTCACCATCTCCAGAGACAATTCCAAGAACACGC TGTATCTGCAAATGAACAGCCTGAGAGCCGAGG ACACGGCTATGTATTACTGTGCGAGAGATCAGGG CGTGGGCTACGACGGTTTGGACGTCTGGGGCCAA GGGACCTCGGTCACCGTCTCCTCA |
| | | | SEQ ID NO: 26719 | SEQ ID NO: 30725 |
| | | AA | DIQMTQSPSSLSASVGDRVTITCRASQGIGKYLA WFQQKPGKAPKSLIYAASSLQSGVPSKFSGSGSG TDFTLTISSLQPENFATYYCQQYSTYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAMYYCARDQGV GYDGLDVWGQGTSVTVSS |
| | | | SEQ ID NO: 26720 | SEQ ID NO: 30726 |

FIGURE 50

| iPS:435953 | 21-225_191B12 | NA | GACATCGTGATGACCCAGTCTCCAGACTCCCT GGCTGTGTCTCTGGGCGAGAGGGCCACCATCA ACTGCAAGTCCAGCCAGAGTGTTTTATTCAAC TCCAACAATAAGAACTACTTAGCTTGGTACCA GCAGAAACCAGGTCAGCCTCCTAAACTGCTCA TTTACTGGGCCTCTACCCGGGAATCCGGGGTC CCTGACCGATTCAGTGGCAGCGGGTCTGGGAC AGATTTCACTCTCACCATCAGCAACCTGCAGG CTGAAGATGTGGCAATTTATTACTGTCAGCAA TATTCTAGTCTTCCATTCACTTTCGGCCCTGGG ACCAAAGTGGATATCAAA | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTACAT GAGCTGGATCCGCCAGGCTCCAGGGAAGGGGCT GGAGTGGGTTTCATACATTAGTAGTAGTGGTACT ACCGTATTCTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGAGAGAATGGGTG GGAGCCGACTACTGGGGCCAGGGAACCCTGGTC ACCGTCTCCTCA |
| | | | SEQ ID NO: 26721 | SEQ ID NO: 30727 |
| | | AA | DIVMTQSPDSLAVSLGERATINCKSSQSVLFNSN NKNYLAWYQQKPGQPPKLLIYWASTRESGVPD RFSGSGSGTDFTLTISNLQAEDVAIYYCQQYSSLP FTFGPGTKVDIK | QVQLVESGGGLVKPGGSLRLSCAASGFTFSDYYMS WIRQAPGKGLEWVSYISSSGTTVFYADSVKGRFTIS RDNAKNSLYLQMNSLRAEDTAVYYCAREWVGAD YWGQGTLVTVSS |
| | | | SEQ ID NO: 26722 | SEQ ID NO: 30728 |
| iPS:435957 | 21-225_191G12 | NA | GACATCCAGATGACCCAGTCTCCTTCCTCACT GTCTGCATCTGTAGGAGACAGAGTCACCATCA CTTGTCGGACGAGTCAGGGCATTGGCAATTAT TTAGCCTGGTTTCAGCAGAAACCAGGGAAAGC CCCTAAGTCCCTGCTCTATAAAGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATATCACTTACCCGCTCA CTTTCGGCGGAGGGTCCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGGCCAA GGGACCACGGTCACCGTCTCCTCA |
| | | | SEQ ID NO: 26723 | SEQ ID NO: 30729 |

FIGURE 50

| | | | | |
|---|---|---|---|---|
| | | AA | DIQMTQSPSSLSASVGDRVTITCRTSQGIGNYLA WFQQKPGKAPKSLLYKASSLQSGVPSKFSGSGS GTDFTLTISSLQPEDFATYYCQQYITYPLTFGGGS KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDQGV GYDGLDVWGQGTTVTVSS |
| | | | SEQ ID NO: 26724 | SEQ ID NO: 30730 |
| iPS:435961 | 21-225_192A2 | NA | GACATCCAGATGACCCAGTCTCCATCCTCACT GTCTGCATCTGTAGGAGACAGAGTCACCATCA CTTGTCGGGCGAATCAGGGCATTAACAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGCTGGCAGTTATATGGTATGATGGAAGT TATAAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAGGATTCC CCTTATAGTGGCTACGCCTTGGACTACTTCTACG GTATGGACGTCTGGGGCCAAGGGACCACGGTCA CCGTCTCCTCA |
| | | | SEQ ID NO: 26725 | SEQ ID NO: 30731 |
| | | AA | DIQMTQSPSSLSASVGDRVTITCRANQGINNYLA WFQQKPGKAPKSLIYAASSLQSGVPSKFSGSGSG TDFTLTISSLQPEDFATYYCQQYNSYPFTFGPGT KVDIK | QVQLVESGGGVVQPGRSLRLSCAASGFTFSSYGMH WVRQAPGKGLEWLAVIWYDGSYKYYADSVKGRF TISRDNSKNTLYLQMNSLRAEDTAVYYCAREDSPY SGYALDYFYGMDVWGQGTTVTVSS |
| | | | SEQ ID NO: 26726 | SEQ ID NO: 30732 |

FIGURE 50

| iPS:435963 | 21-225_192D2 | NA | GACATCCAGATGATTCAGTCTCCATCCTCACT GTCTGCATCGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACATTATGTTACTTACCCGAACA CTTTCGGCGGAGGGACCAAGGTGGAGATCAA A<br><br>SEQ ID NO: 26727 | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACATGT TGTATCTGCAAATGAACAGCCTGAGAGCCGAGG AAACGGCTGTGTATTACTGTGCGAGAGATCGTGG GGTTGGCTACTACGGTATGGACGTCTGGGGCCAA GGGACCACGGTCACCGTCTCCTCA<br><br>SEQ ID NO: 30733 |
|---|---|---|---|---|
| | | AA | DIQMIQSPSSLSASVGDRVTITCRASQGISNYLA WFQQKPGKAPKSLIYAASSLQSGVPSKFSGSGSG TDFTLTISSLQPEDFATYYCQHYVTYPNTFGGGT KVEIK<br><br>SEQ ID NO: 26728 | QVQLVESGGGVVQPGRSLRLSCAASGFTFSSYGMH WVRQAPGKGLEWVAVIWYDGSNKNYADSVKGRF TISRDNSKNMLYLQMNSLRAEETAVYYCARDRGV GYYGMDVWGQGTTVTVSS<br><br>SEQ ID NO: 30734 |
| iPS:435965 | 21-225_192H2 | NA | GACATCCAGATGACCCAGTCTCCATCTTCCGT GTCTGCATCGTAGGAGACAGAGTCACCATCA CTTGTCGGGCGAGTCAGGGTATAAGTAGTTGG ATAGCCTGGTATCAGCAGAAACCAGGGAAAG CCCCTAAGCTCCTGATCTATGGTGCATCCAGTT TGCAAAGTGGGGTCCCATCTAGGTTCAGCGGC AGTGGATCTGGGACAGATTTCACTCTCACCAT CAGCAGCCTGCAGCCTGAAGATTTTGCAACTT ACTATTGTCAACAGTCTAACAGTTTCCCATTCA CTTTCGGCCCTGGGACCAAAGTGGATGTCAAA<br><br>SEQ ID NO: 26729 | GAGGTGCAGCTGTTGGAATCTGGGGGAGACTTA ATACAGCCTGGGGGGTCCCTGAGACTCTCCTGTG CAGCCTCTGGATTCACCTTTAGCAGCTCTGCCAT GAGCTGGGTCCGCCAGGCTCCAGGGAAGGGGCT GGAGTGGGTCTCAGCCATTAGTGGTAGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAAGA CACGGCCGTTTATTACTGTGCGAAACTCATAGCA GTAGTTGGGTCCCACTACTTTGACTACTGGGGCC AGGGAACCCTGGTCACCGTCTCCTCA<br><br>SEQ ID NO: 30735 |

FIGURE 50

| | | | | |
|---|---|---|---|---|
| | | AA | DIQMTQSPSSVSASVGDRVTITCRASQGISSWIA WYQQKPGKAPKLLIYGASSLQSGVPSRFSGSGS GTDFTLTISSLQPEDFATYYCQQSNSFPFTFGPGT KVDVK | EVQLLESGGDLIQPGGSLRLSCAASGFTFSSSAMSW VRQAPGKGLEWVSAISGSGGNTFYADSVKGRFTIS RDNSKNTLYLQMNSLRAEDTAVYYCAKLIAVVGS HYFDYWGQGTLVTVSS |
| | | | SEQ ID NO: 26730 | SEQ ID NO: 30736 |
| iPS:435967 | 21-225_192B3 | NA | GAAATTGTGTTGACGCAGTCTCCAGGCACCCT GTCTTTGTCTCCAGGGGAAAGAGCCACCCTCT CCTGCAGGGCCAGTCAGAGTGTTCGCAGCAGC TTCCTTGCCTGGTACCAGCAGAAACCTGGCCA GGCTCCCAGGCTCCTCATCTATGGTGCATCTA GCAGGGCCACTGGCATCCCAGACAGGTTCAGT GGCAGTGGGTCTGGGACAGACTTCACTCTCAC CATCAGCAGACTGGAGCCTGAAGATTTTGCTG TGTATTACTGTCAGCAGTATGGTAACTCACCG TGGGCGTTCGGCCAAGGGACCAAGGTGGAAA TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGATCCGCCAGCACCCAGGGAA GGGCCTGGAATGGATTGGGTTCATCTTTTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGTCGCG GACACGGCCGTGTATTACTGTGCGAGAGGGGATT ACGATGGTTCGGGGAGTTATCACCACTACTACGG TATGGACGTCTGGGGCCAAGGGACCACGGTCAC CGTCTCCTCA |
| | | | SEQ ID NO: 26731 | SEQ ID NO: 30737 |
| | | AA | EIVLTQSPGTLSLSPGERATLSCRASQSVRSSFLA WYQQKPGQAPRLLIYGASSRATGIPDRFSGSGSG TDFTLTISRLEPEDFAVYYCQQYGNSPWAFGQG TKVEIK | QVQLQESGPGLVKPSQTLSLTCTVSGGSISSGDYYW NWIRQHPGKGLEWIGFIFYSGSTYYNPSLKSRVTIS VDTSKNQFSLKLSSVTVADTAVYYCARGDYDGSG SYHHYYGMDVWGQGTTVTVSS |
| | | | SEQ ID NO: 26732 | SEQ ID NO: 30738 |

FIGURE 50

| iPS:435971 | 21-225_192D3 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGAACAGATTTCACTCTCACCATC AGCAGCCTGCAGCCTGAAGATTTTGCAACTTA TTACTGCCTACATTATCTTACTTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATAGGGG GGTGGGTTACTACGGTTTGGACGTCTGGGGCCAA GGGACCACGGTCACCGTCTCCTCA |
| | | | SEQ ID NO: 26733 | SEQ ID NO: 30739 |
| | | AA | DIQMTQSPSSLSASVGDRVTITCRASQGISNYLA WFQQKPGKAPKSLIYAASSLQSGVPSKFSGSGSG TDFTLTISSLQPEDFATYYCLHYLTYPLTFGGGT KVEIK | QVQLVESGGGVVQPGRSLRLSCAASGFTFSSYGMH WVRQAPGKGLEWVAVIWYDGSNEHYADSVKGRF TISRDNSKNTLYLQMNSLRAEDTAVYYCARDRGV GYYGLDVWGQGTTVTVSS |
| | | | SEQ ID NO: 26734 | SEQ ID NO: 30740 |
| iPS:435973 | 21-225_192H3 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAGCAAC TTCTTAGCCTGGTACCAGCAGAAACCTGGCCA GGCTCCCAGGCTCCTCATCTTTGGTGCATCCA GCAGGGCCACTGGCATCCCAGACAGGTTCAGT GGCAGTGGGTCTGGGACAGACTTCACTCTCAC CATCAACAGACTGGAGCCTGAAGATTTTGCAG TGTATTACTGTCAGCAATATGGTATCTCACCGT GGACGTTCGGCCAAGGGACCAAGGTGGAAAT CAAA | CAGGTGCAGCTGCAGGAGTCGGGGCCCAGGACTG GTGAAGCCTTCACAGACCCTGTCCCTCACCTGCA CTGTCTCTGGTGGCTCCATCAGCAGTGTTAGTTA CTACTGGAGCTGGATCCGCCAGCGCCCAGGGAA GGGCCTGGAGTGGATTGGGAACCTCTATTACAGT GGGAGCACCTACTACAACCCGTCCCTCAGGAGTC GAGCTACCATATCAGTAGACACGTCTAAGAACC AGTTCTCCCTGAAGCTGAGTTCTGTGACTGCCGC GGACACGGCCGTGTATTACTGTACGAGAGGGGA TTACGATGGTTCGGGGAGTTATCACTACTACCAC GGTATGGACGTCTGGGGCCAAGGGACCACGGTC ACCGTCTCCTCA |
| | | | SEQ ID NO: 26735 | SEQ ID NO: 30741 |

FIGURE 50

| | | | | |
|---|---|---|---|---|
| | | AA | EIVLTQSPGTLSLSPGERATLSCRASQSVSSNFLA WYQQKPGQAPRLLIFGASSRATGIPDRFSGSGSG TDFTLTINRLEPEDFAVYYCQQYGISPWTFGQGT KVEIK | QVQLQESGPGLVKPSQTLSLTCTVSGGSISSVSYYW SWIRQRPGKGLEWIGNLYYSGSTYYNPSLRSRATIS VDTSKNQFSLKLSSVTAADTAVYYCTRGDYDGSGS YHYYHGMDVWGQGTTVTVSS |
| | | | SEQ ID NO: 26736 | SEQ ID NO: 30742 |
| iPS:435977 | 21-225_192E4 | NA | GACATCCAGATGACCCAGTCTCCATCCTCACT GTCTGCATCTGTAGGAGACAGAGTCACCATCA CTTGTCGGGCGAGTCAGGGCATTGGCAATTAT TTAGCCTGGTTTCAGCAGAAACCAGGGAAAGC CCCTAAGTCCCTGATCTATGTTGTATCCAGTTT ACAAAGTGGGGTCCCATCAAGGTTCAGCGGCA GTGGATCTGGGACAGATTTCACTCTCACCATC AGCAGCCTGCAGCCTGAAGATTTTGCGACTTA TTACTGCCAACGGTATGATACTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCAGTTATATGGTATGATGGAAGT AACAAAAACTATGTAGACTCCGTGAGGGGCCGA TTCACCATCTCCAGAGACAACTCCAAGAACACGC TGTATCTGCAAATGAACAGCCTGAGAGCCGAGG ACACGGCTGTGTATTATTGTGCGAGAGATAGAAG CGTCGGCTACGACGGTATGGACGTCTGGGGCCA AGGGACCACGGTCACCGTCTCCTCA |
| | | | SEQ ID NO: 26737 | SEQ ID NO: 30743 |
| | | AA | DIQMTQSPSSLSASVGDRVTITCRASQGIGNYLA WFQQKPGKAPKSLIYVVSSLQSGVPSRFSGSGSG TDFTLTISSLQPEDFATYYCQRYDTYPFTFGPGT KVDIK | QVQLVESGGGVVQPGRSLRLSCAASGFTFSSYGMH WVRQAPGKGLEWVAVIWYDGSNKNYVDSVRGRF TISRDNSKNTLYLQMNSLRAEDTAVYYCARDRSVG YDGMDVWGQGTTVTVSS |
| | | | SEQ ID NO: 26738 | SEQ ID NO: 30744 |

FIGURE 50

| iPS:435979 | 21-225_192H4 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTCTGCTGCATCCAGTTT GCAAAGTGGGGTCCCATCAAAGTTCAGCGGCA GTAGATCTGGGACAGATTTCACTCTCACCATC AGCAGCCTGCAGCCTGAAGATTTTGCAACTTA TTACGGCCTACATTATCTCAATTACCCGCTCAC TTTCGGCGGAGGGACCAGGGTGGAGATCAGA<br><br>SEQ ID NO: 26739 | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAG CAATAAAAAACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGATCAAG GTGTGGGGTACTACGGTATGGACGTCTGGGGCCA AGGGACCACGGTCACCGTCTCCTCA<br><br>SEQ ID NO: 30745 |
|---|---|---|---|---|
| | | AA | DIQMTQSPSSLSASVGDRVTITCRASQDISNYLA WFQQKPGKAPKSLISAASSLQSGVPSKFSGSRSG TDFTLTISSLQPEDFATYYGLHYLNYPLTFGGGT RVEIR<br><br>SEQ ID NO: 26740 | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDQGV GYYGMDVWGQGTTVTVSS<br><br>SEQ ID NO: 30746 |
| iPS:435983 | 21-225_192E5 | NA | GAAATTGTTCTGACTCAGTCTCCAGATTTTCAG TCTGTGACTCCAAAGGAGAAAGTCACCATCAC CTGCCGGGCCAGTCAGAGCATTGGTAGGAGTT TACACTGGTACCAGCAGAAACCAGATCAGTCT CCAAAGCTCCTCATCAAGTATGCTTCCCAGTC ATTCTCAGGGGTCCCCTCGAGGTTCAGTGGCA GTGGATCTGGGACAGATTTCACCCTCACCATC AATAGCCTGGAGGCTGAAGATGCTGCAACGTA TTACTGTCATCAGAGTAGTCGTTTACCGCTCAC TTTCGGCGGAGGGACCAAGGTGGAGATCAAA<br><br>SEQ ID NO: 26741 | CAGGTGCAGCTGCAGGAGTCGGGCCCAGGACTG GTGAAGCCTTCACAGACCCTGTCCCTCACCTGCA CTGTCTCTGGTGACTCCATCAATAATGGTGGATA CTACTGGAGCTGGATCCGCCAGCACCCAGGGAA GGGCCTGGAGTGGATTGGATACATCTTTTACAGC GGGAGCACCTACTACAACCCGTCCCTCAAGAGTC GAGTTACCATATCAGTTGACACGTCTAAGAATCA GTTCTCCCTGAAACTGAACTCTGTGACTGCCGCG GACACGGCCGTGTATTTTTGTGCGAGAGCGGGAT ATAACTGGGACAACGGGTTTGACTACTGGGGCC AGGGAACCCTGGTCACCGTCTCCTCA<br><br>SEQ ID NO: 30747 |

FIGURE 50

| | | | | |
|---|---|---|---|---|
| | | AA | EIVLTQSPDFQSVTPKEKVTITCRASQSIGRSLHW YQQKPDQSPKLLIKYASQSFSGVPSRFSGSGSGT DFTLTINSLEAEDAATYYCHQSSRLPLTFGGGTK VEIK | QVQLQESGPGLVKPSQTLSLTCTVSGDSINNGGYY WSWIRQHPGKGLEWIGYIFYSGSTYYNPSLKSRVTI SVDTSKNQFSLKLNSVTAADTAVYFCARAGYNWD NGFDYWGQGTLVTVSS |
| | | | SEQ ID NO: 26742 | SEQ ID NO: 30748 |
| iPS:435985 | 21-225_192F6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCTGAAACCAGGGAAAGC CCCTAAGCGCCTGATCTATGCTGCATCCAGTTT GCAAAGTGGGGTCCCATCAAGGTTCAGCGGCA GTGGATCTGGGCCAGAATTCACTCTCACAATC AGCAGCCTGCAGCCTGAAGATTTTGCAACTTA TTACTGTCTACAGCATTATAGTTTCCCATTCAC TTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTTTATCAT GCACTGGGTCCGCCAGGCTCCAGGCAGGGGGCT GGAGTGGGTGGCAGTTATATGGTATGATGGAAGT AATAAATACTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTACTACTGTGCGAGAGAGGAGTAT AGTAGCGGCTGGTTCGGGTACGGTATGGACGTCT GGGGCCAAGGGACCACGGTCACCGTCTCCTCA |
| | | | SEQ ID NO: 26743 | SEQ ID NO: 30749 |
| | | AA | DIQMTQSPSSLSASVGDRVTITCRASQGIRNDLG WYQLKPGKAPKRLIYAASSLQSGVPSRFSGSGSG PEFTLTISSLQPEDFATYYCLQHYSFPFTFGPGTK VDIK | QVQLVESGGGVVQPGRSLRLSCAASGFTFSSFIMH WVRQAPGRGLEWVAVIWYDGSNKYYVDSVKGRF TISRDNSKNTLYLQMNSLRAEDTAVYYCAREEYSS GWFGYGMDVWGQGTTVTVSS |
| | | | SEQ ID NO: 26744 | SEQ ID NO: 30750 |

FIGURE 50

| iPS:435987 | 21-225_192G6 | NA | GACATCAAGATGACCCAGTCTCCTTCCTCACT GTCTGCATCTGTAGGAGACAGAGTCACCATCA CTTGTCGGACGAGTCAGGGCATTGGCAATTAT TTAGCCTGGTTTCAGCAGAAACCAGGGAAAGC CCCTAAGTCCCTGCTCTATAAAGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATATGACTTACCCGCTCA CTTTCGGCGGGGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTTTATGGTATGATGGAACT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGCCAA GGGACCACGGTCACCGTCTCCTCA |
|---|---|---|---|---|
| | | | SEQ ID NO: 26745 | SEQ ID NO: 30751 |
| | | AA | DIKMTQSPSSLSASVGDRVTITCRTSQGIGNYLA WFQQKPGKAPKSLLYKASSLQSGVPSKFSGSGS GTDFTLTISSLQPEDFATYYCQQYMTYPLTFGGG TKVEIK | QVQLVESGGGVVQPGRSLRLSCAASGFTFSSYGMH WVRQAPGKGLEWVAVLWYDGTNKNYADSVKGR FTISRDNSKNTLYLQMNSLRAEDTAVYYCARDQGV GYDGLDVWGQGTTVTVSS |
| | | | SEQ ID NO: 26746 | SEQ ID NO: 30752 |
| iPS:435989 | 21-225_192F7 | NA | GACATCCAGATGACCCAGTCTCCATCCTCCCT GTCTGCGTCTGTAGGAGACAGAGTCACCATCA CTTGCCGGGCAAGTCAGGGCATTAGAAAAGAT TTAGGCTGGTATCAACAGAAACCAGGGAAAG CCCCTAAGCGCCTAATCCATACTGCATCCAGT TTGCAAAGTGGGGTCCCATTAAGGTTCAGCGG CAGTGGATCTGGGACAGAATTCACTCTCACAA TCAGCAGCCTGCAGCCTGAAGATTTTGCAACT TATTACTGTCTACAGCATACTAGTTACCCGTG GACGTTCGGCCAAGGGACCAAGGTGGAAATC TAC | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGACAGTTATATCATATGATGGAGGT TATAAAAACTATGCAGACTCCGTGAAGGGCCGA TTCACCATCTCCAGAGACAATTCCAAGAACACGC TGTATCTGCAAATGAATAGCCTGAGAGCTGAGGA CACGGCTGTGTATTACTGTGCGAGAGGTACCCAC GGGTACTACTACGGTGTGGACGTCTGGGGCCAA GGGACCACGGTCACCGTCTCCTCA |
| | | | SEQ ID NO: 26747 | SEQ ID NO: 30753 |

FIGURE 50

| | | | | |
|---|---|---|---|---|
| | | AA | DIQMTQSPSSLSASVGDRVTITCRASQGIRKDLG WYQQKPGKAPKRLIHTASSLQSGVPLRFSGSGS GTEFTLTISSLQPEDFATYYCLQHTSYPWTFGQG TKVEIY | QVQLVESGGGVVQPGRSLRLSCAASGFTFSSYGMH WVRQAPGKGLEWVTVISYDGGYKNYADSVKGRF TISRDNSKNTLYLQMNSLRAEDTAVYYCARGTHG YYYGVDVWGQGTTVTVSS |
| | | | SEQ ID NO: 26748 | SEQ ID NO: 30754 |
| iPS:435993 | 21-225_192C8 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGAACAGATTTCACTCTCACCATC AGCAGCCTGCAGCCTGAGGATTTTGCTACTTA TTACTGCCAACATTATCTTACTTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCATGATCTCCAGAGACAATTCCAAGAACACGC TGTATCTGCAAATGAACAGCCTGAGAGCCGAGG ACACGGCTGTGTATTACTGTGCGAGAGATAGGGG AGTGGGTTACTACGGTATGGACGTCTGGGGCCAA GGGACCACGGTCACCGTCTCCTCA |
| | | | SEQ ID NO: 26749 | SEQ ID NO: 30755 |
| | | AA | DIQMTQSPSSLSASVGDRVTITCRASQGISNYLA WFQQKPGKAPKSLIYAASSLQSGVPSKFSGSGSG TDFTLTISSLQPEDFATYYCQHYLTYPLTFGGGT KVEIK | QVQLVESGGGVVQPGRSLRLSCAASGFTFSSYGMH WVRQAPGKGLEWVAVIWYDGSNEHYADSVKGRF MISRDNSKNTLYLQMNSLRAEDTAVYYCARDRGV GYYGMDVWGQGTTVTVSS |
| | | | SEQ ID NO: 26750 | SEQ ID NO: 30756 |

FIGURE 50

| iPS:435995 | 21-225_192F8 | NA | GAAATTGTGTTGACGCAGTCTCCAGGCACCCT GTCTTTGTCTCCAGGGGAAAGAGCCACCCTCT CCTGCAGGGCCAGTCAGAGTATTAGCAGCAGC TACTTAGCCTGGTACCAGCAGAAACCTGGCCA GGCTCCCCGGCTCCTCATCTATGGTGCATCCA GCAGGGCCACTGGCATCCCAGCCAGGTTCAGT GGCAGTGGGTCTGGGACAGACTTCACTCTCAC CATCAGCAGACTGGAGCCTGAAGATTTTGCAG TGTATTACTGTCAGCAGTATGAGAGTTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATCAAAGTGGAAG GTCCAACTACAACCCGTCCCTCAAGAGTCGAGTC ACCATTTCAGTAGACACGTCCACGAACCAGTTCT CCCTGAAGCTGAGATCTGTGACCGCCGCGGACAC GGCTGTGTATTACTGTGCGAGAGACTACGGTGTC TTTGACTACTGGGGCCAGGGCACCCTGGTCACCG TCTCCTCA |
| | | | SEQ ID NO: 26751 | SEQ ID NO: 30757 |
| | | AA | EIVLTQSPGTLSLSPGERATLSCRASQSISSSYLA WYQQKPGQAPRLLIYGASSRATGIPARFSGSGSG TDFTLTISRLEPEDFAVYYCQQYESSPWTFGQGT KVEIK | QVQLQQWGAGLLKPSETLSLTCAVYGGSFSGCYW SWIRQPPGKGLEWIGEINQSGRSNYNPSLKSRVTIS VDTSTNQFSLKLRSVTAADTAVYYCARDYGVFDY WGQGTLVTVSS |
| | | | SEQ ID NO: 26752 | SEQ ID NO: 30758 |
| iPS:435997 | 21-225_192G8 | NA | GACATCCAGATGACCCAGTCTCCTTCCTCACT GTCTGCATCTGTAGGAGACAGAGTCACCATCA CTTGTCGGACGAGTCAGGGCATTGGCAATTAT TTAGCCTGGTTTCAGCAGAAACCAGGGAAAGC CCCTAAGTCCCTGCTCTATAAAGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATATCACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGCCAA GGGACCACGGTCACCGTCTCCTCA |
| | | | SEQ ID NO: 26753 | SEQ ID NO: 30759 |

FIGURE 50

| | | | | |
|---|---|---|---|---|
| | | AA | DIQMTQSPSSLSASVGDRVTITCRTSQGIGNYLA WFQQKPGKAPKSLLYKASSLQSGVPSKFSGSGS GTDFTLTISSLQPEDFATYYCQQYITYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDQGV GYDGLDVWGQGTTVTVSS |
| | | | SEQ ID NO: 26754 | SEQ ID NO: 30760 |
| iPS:435999 | 21-225_192F9 | NA | GATATTGTAATGACCCAGACTCCACTCTCTCT GTCCGTCACCCCTGGACAGCCGGCCTCCATCT CCTGCAAGTCTAGTCAGAGCCTCCTCCATAGT GATGGAAGGACCTATTTGTATTGGTACCTGCA GAGGCCAGGCCAGCCTCCACAGCTCCTGATCT GTGAGGTTTCCAACCGGTTCGCTGGAGTGACA GATAGGTTCAGTGGCAGCGGGTCAGGGACAG ATTTCACACTGAAAATCAGCCGGGTGGAGGCT GGGGATGTTGGGGTTTATTACTGCATGCAAAG TATACAGCTTCCCTGGACGTTCGGCCAAGGGA CCAAGGTGGAAATCAAA | CAGCTGCAGCTGCAGGAGTCGGGCCCAGGACTG GTGAAGCCTTCGGAGACCCTGTCCCTCACCTGCA CTGTCTCTGGTGGCTCCATCAGAAGCTACCACTG GAGCTGGATCCGGCAGCCCGCCGGGAAGGGACT GGAGTGGATTGGGCTTATCTATACCAGTAGGAGC ACCAATTACAACCCCTCCCTCAAGAGTCGAGTCA CCATGTCAGTTGACACGTCCAAGAACCAGTTCTC CCTGAAGCTGAACTCTGTGACCGCCGCGGACACG GCCGTGTATTACTGTGCGAGACTCCGGTATAACT GGAACTTCCCTTACTTTGACTACTGGGGCCAGGG AACCCTGGTCACCGTCTCCTCA |
| | | | SEQ ID NO: 26755 | SEQ ID NO: 30761 |
| | | AA | DIVMTQTPLSLSVTPGQPASISCKSSQSLLHSDGR TYLYWYLQRPGQPPQLLICEVSNRFAGVTDRFS GSGSGTDFTLKISRVEAGDVGVYYCMQSIQLPW TFGQGTKVEIK | QLQLQESGPGLVKPSETLSLTCTVSGGSIRSYHWSW IRQPAGKGLEWIGLIYTSRSTNYNPSLKSRVTMSVD TSKNQFSLKLNSVTAADTAVYYCARLRYNWNFPY FDYWGQGTLVTVSS |
| | | | SEQ ID NO: 26756 | SEQ ID NO: 30762 |

FIGURE 50

| iPS:436001 | 21-225_192C10 | NA | GACATCCAGATGACCCAGTCTCCATCCTCACT GTCTGCATCTGTGGGAGACAGAGTCACCATCA CTTGTCGGGCGAGTCAGGGCATTGGCAAGTAT TTAGCCTGGTTTCAGCAGAAACCAGGGAAAGC CCCTAAGTCACTGATCTATGCTGCATCCAGTTT GCAAAGTGGGGTCCCATCAAAGTTCAGCGGCA GTGGGTCTGGGACAGATTTCACTCTCACCATC AGCAGCCTGCAGCCTGACGATTTTGCAACTTA TTACTGCCAACGATATGATACTTACCCATTCA CTTTCGGCCCTGGGACCAAAGTGGATTTCAAA | CAGGTGCAGCTGGTGGAGTCTGGGGGAGGCGTG GTCCAGCCTGGGAGGTCCCTGAGACTCTCCTGTG CATCGTCTGGATTCACCTTCAGGAACTATGGCAT GCACTGGGTCCGCCAGGCTCCAGGCAAGGGGCT GAAATGGGTGGCAGTTATATGGTTTGATGGAAGT AATGACTACTATGCAGACTCCGTGAAGGGCCGAT TCACCATCTCCAGAGACAATTCCAAGAACACGCT GTCACTGCAAATGAACAGCCTGAGAGCCGAGGA CACGGCTGTGTATTACTGTGCGAGAGATAGAAGC GTCGGCTACGACGGTTTAGATGTCTGGGGCCAAG GGACCACGGTCACCGTCTCCTCA |
| | | | SEQ ID NO: 26757 | SEQ ID NO: 30763 |
| | | AA | DIQMTQSPSSLSASVGDRVTITCRASQGIGKYLA WFQQKPGKAPKSLIYAASSLQSGVPSKFSGSGSG TDFTLTISSLQPDDFATYYCQRYDTYPFTFGPGT KVDFK | QVQLVESGGGVVQPGRSLRLSCASSGFTFRNYGMH WVRQAPGKGLKWVAVIWFDGSNDYYADSVKGRF TISRDNSKNTLSLQMNSLRAEDTAVYYCARDRSVG YDGLDVWGQGTTVTVSS |
| | | | SEQ ID NO: 26758 | SEQ ID NO: 30764 |
| iPS:436003 | 21-225_192G10 | NA | GACATCCAGATGACCCAGTCTCCATCCTCCCT GTCTGCATCTGTAGGAGACAGAGTCACCATCA CTTGCCGGGCAAGTCAGAGCATTAGCAACTAT TTAAATTGGTATCAGCAGACACCAGGAAAAGC CCCTAAGCTCCTGATCTATGCTGAATCCAGTTT ACAAAGTGGGGTCCCATCAAGGTTCAGTGGCA GTGGATCTGGGACAGATTTCACTCTCACCATC AGCAGTCTGCAACCTGAAGATTTTGCAACTTA CTCCTGTCAACAGAGTTACAGTTCCCCTCCGT GGACGTTCGGCCAAGGGACCAAGGTGGAAAT CAAA | GAGGTGCAGCTGTTGGAGTCTGGGGGAGGCTTG GTACAGCCTGGGGGGTCCCTGAGACTCTCCTGTT CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGAGGCGGT AGTACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTCTATTACTGTGCGCGACGTTTAGCA CTGGATGGCTATGATGCTTTTGATATCTGGGGCC AAGGGACAATGGTCACCGTCTCTTCA |
| | | | SEQ ID NO: 26759 | SEQ ID NO: 30765 |

FIGURE 50

| | | | | |
|---|---|---|---|---|
| | | AA | DIQMTQSPSSLSASVGDRVTITCRASQSISNYLN WYQQTPGKAPKLLIYAESSLQSGVPSRFSGSGSG TDFTLTISSLQPEDFATYSCQQSYSSPPWTFGQGT KVEIK | EVQLLESGGGLVQPGGSLRLSCSASGFTFSSYAMS WVRQAPGKGLEWVSAISGRGGSTFYADSVKGRFTI SRDNSKNTLYLQMNSLRAEDTAVYYCARRLALDG YDAFDIWGQGTMVTVSS |
| | | | SEQ ID NO: 26760 | SEQ ID NO: 30766 |
| iPS:436005 | 21-225_192H10 | NA | GACATCCAGATGACCCAGTCTCCATCCTCACT GTCTGCATCTGTAGGAGACAGAGTCACCATCA CTTGTCGGGCGAGTCAGGGCATTGGCAAGTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATATGGGACAGATTTCACTCTCACCATC AGCAGCCTGCAGCCTGAAAATTTTGCAACTTA TTACTGCCAACAGTATAGTACTTACCCGCTCA CTTTCGGCGGAGGGACCAAGGTGGAGATCAA G | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTACGCAGACTCCGTGAAGGGCCGA TTCACCATCTCCAGAGACAATTCCAAGAACACGC TGTATCTGCAAATGAACAGCCTGAGAGCCGAGG ACACGGCTATGTATTACTGTGCGAGAGATCAGGG CGTGGGCTACGACGGTTTGGACGTCTGGGGCCAA GGGACCTCGGTCACCGTCTCCTCA |
| | | | SEQ ID NO: 26761 | SEQ ID NO: 30767 |
| | | AA | DIQMTQSPSSLSASVGDRVTITCRASQGIGKYLA WFQQKPGKAPKSLIYAASSLQSGVPSKFSGSGY GTDFTLTISSLQPENFATYYCQQYSTYPLTFGGG TKVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAMYYCARDQGV GYDGLDVWGQGTSVTVSS |
| | | | SEQ ID NO: 26762 | SEQ ID NO: 30768 |

FIGURE 50

| iPS:436007 | 21-225_192G12 | NA | GAAATTGTGTTGACGCAGTCTCCAGGCACCCT GTCTTTGTCTCCAGGGGAAAGAGCCACCCTCT CCTGCAGGGCCAGTCAGAGTGTCAGAAGCGA CTTCTTAGCCTGGCTCCAGCAGAAACCTGGCC AGGCTCCCAGGCTCCTCATCTATGGTGTATCC CGCAGGGCCACTGGCATCCCAGACAGGTTCAG TGGCAGTGGGTCTGGGACAGACTTCACTCTCA CCATCAACAGACTGGAGCCTGAAGATTTTGCA GTGTATTACTGTCAGCAGTATGGTAACTCACC GTGGACGTTCGGCCAAGGGACCAAGGTGGAA ATCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGTTTA CCACTGGAGCTGGATCCGCCAGCACCCAGGGAA GGGCCTGGAGTGGATTGGGAACATCCATTACAG CGGGAGCACCTACAACAACCCGTCCCTCAAGAG TCGAGTTACCATATCAGTAGACACGTCTAAGAAC CAGTTCTCCCTGAAGCTGAGCTCTGTGACTGCCG CGGACACGGCCGTGTATTACTGTGCGAGAGGGG ATTACGATGGTTCGGGGGAGTTATCACTACTACTA CGGTATGGACGTCTGGGGCCAAGGGACCACGGT CACCGTCTCCTCA |
| | | | SEQ ID NO: 26763 | SEQ ID NO: 30769 |
| | | AA | EIVLTQSPGTLSLSPGERATLSCRASQSVRSDFLA WLQQKPGQAPRLLIYGVSRRATGIPDRFSGSGSG TDFTLTINRLEPEDFAVYYCQQYGNSPWTFGQG TKVEIK | QVQLQESGPGLVKPSQTLSLTCTVSGGSISSGVYHW SWIRQHPGKGLEWIGNIHYSGSTYNNPSLKSRVTIS VDTSKNQFSLKLSSVTAADTAVYYCARGDYDGSG SYHYYYGMDVWGQGTTVTVSS |
| | | | SEQ ID NO: 26764 | SEQ ID NO: 30770 |
| iPS:436009 | 21-225_193A1 | NA | GAAATTGTGTTGACGCAGTCTCCAGGCACCCT GTCTTTGTCTCCAGGGGAAAGAGCCACCCTCT CTTGTAGGGCCAGTCAGAGTGTTAGAAGCAAC TTCTTAGCCTGGCACCAGCAGAAACCTGGCCA GGCTCCCAGGCTCTTCATCTATGGTGCATCCC GCAGGGCCACTGGCATCCCAGACAGGTTCAGT GGCAGTGGGTCTGGGACAGACTTCACTCTCAC CATCAACAGACTGGAGCCTGAAGATTTTGCAG TGTATTACTGTCAGCAGTATGGTAACTCACCG TGGACGTTCGGCCAAGGGACCAAGGTGGAAA TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGTTTA CTACTGGAGCTGGATCCGCCAGCACCCAGGGAA GGGCCTGGAGTGGATTGGGAACATCTATTACAGT GGGAGCACCTACAACAACCCGTCCCTCAAGAGT CGACTTACCATATCAGCAGACACGTCTAAGAACC AGTTCTCCCTGAAGCTGAGCTCTGTGACTGCCGC GGACACGGCCGTGTATTACTGTGCGAGAGGGGA TTACGATGGTTCGGGGGAGTTATCACTACTACTAC GGTATGGACGTCTGGGGCCAAGGGACCACGGTC ACCGTCTCCTCA |
| | | | SEQ ID NO: 26765 | SEQ ID NO: 30771 |

FIGURE 50

| | | | | |
|---|---|---|---|---|
| | | AA | EIVLTQSPGTLSLSPGERATLSCRASQSVRSNFLA WHQQKPGQAPRLFIYGASRRATGIPDRFSGSGSG TDFTLTINRLEPEDFAVYYCQQYGNSPWTFGQG TKVEIK | QVQLQESGPGLVKPSQTLSLTCTVSGGSISSGVYYW SWIRQHPGKGLEWIGNIYYSGSTYNNPSLKSRLTIS ADTSKNQFSLKLSSVTAADTAVYYCARGDYDGSG SYHYYYGMDVWGQGTTVTVSS |
| | | | SEQ ID NO: 26766 | SEQ ID NO: 30772 |
| iPS:436011 | 21-225_193B1 | NA | GAAATTGTGTTGACGCAGTCTCCAGGCACCCT GTCTTTGTCTCCAGGGGACAGAGCCACCCTCT CTTGCAGGGCCAGTCAGAGTGTTAGAAGCAAC TTCTTAGCCTGGCACCAGCAGAAACCTGGCCA GGCTCCCAGGCTCCTCATCTATGGTGCATCCC GCAGGGCCACTGGCATCCCAGACAGGTTCAGT GGCAGTGGGTCTGGGACAGACTTCACTCTCAC CATCAACAGACTGGAACCTGAAGATTTTGCAG TGTATTTCTGTCAGCAGTATGGTAACTCACCGT GGACGTTCGGCCAAGGGACCAAGGTGGAAAT CAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGTTTA CTACTGGAGCTGGATCCGCCAGCACCCAGGGAA GGGCCTGGAGTGGATTGGGAACATCTATTACAGT GGGAGCACCTACAACAACCCGTCCCTCAAGAGT CGAGTTACCATATCAGTAGACACGTCTAAGAACC AGTTCTCCCTGAAGCTGAGCTCTGTGACTGCCGC GGACACGGCCGTGTATTACTGTGCGAGAGGGGA TTACGATGGTTCGGGGAGTTATCACTACTACTAC GGTATGGACGTCTGGGGCCAAGGGACCACGGTC ACCGTCTCCTCA |
| | | | SEQ ID NO: 26767 | SEQ ID NO: 30773 |
| | | AA | EIVLTQSPGTLSLSPGDRATLSCRASQSVRSNFLA WHQQKPGQAPRLLIYGASRRATGIPDRFSGSGSG TDFTLTINRLEPEDFAVYFCQQYGNSPWTFGQGT KVEIK | QVQLQESGPGLVKPSQTLSLTCTVSGGSISSGVYYW SWIRQHPGKGLEWIGNIYYSGSTYNNPSLKSRVTIS VDTSKNQFSLKLSSVTAADTAVYYCARGDYDGSG SYHYYYGMDVWGQGTTVTVSS |
| | | | SEQ ID NO: 26768 | SEQ ID NO: 30774 |

FIGURE 50

| | | | | |
|---|---|---|---|---|
| iPS:436013 | 21-225_193F2 | NA | GACATCCAGATGACCCAGTCTCCATCTTCCGT GTCTGCATCTGTAGGAGACAGAGTCACCATCA CTTGTCGGGCGAGTCAGGGTATTAACAACTGG TTAGCCTGGTATCAGCAGAAACCAGGGAAAG CCCCTAAGCTCCTGATCTATGGTGTTTCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTCTTGTCAACAGGCTAACAGTTTCCCGTGG ACGTTCGGCCAAGGGACCAAGGTGGAAATCA GA | GAGGTGCAGCTGTTGGAGTCTGGGGGAGGCTTG GTACAGCCTGGGGGGTCCCTGAGACTCTCCTGTG CAGCCTCTGGATTCACCTTTAGCACCTTTGCCAT GAGTTGGGTCCGCCAGGCTCCAGGGAAGGGGCT GGAGTGGGTCTCAGCTATTAGTCGTAGTGGTGGT AACACACACTACGCAGACTCCGTGAAGGGCCGG TTCACCATCTCCAGAGACAATTCCAAGAATACGC TGTATCTGCAAATGAACAGCCTGAGAGCCGAGG ACACGGCCGTATATTCCTGTGCGAAAGATGGATT CGGTGGGAGCTCCTACTTTGACTACTGGGGCCAG GGAACCCTGGTCACCGTCTCCTCA |
| | | | SEQ ID NO: 26769 | SEQ ID NO: 30775 |
| | | AA | DIQMTQSPSSVSASVGDRVTITCRASQGINNWLA WYQQKPGKAPKLLIYGVSSLQSGVPSRFSGSGS GTDFTLTISSLQPEDFATYSCQQANSFPWTFGQG TKVEIR | EVQLLESGGGLVQPGGSLRLSCAASGFTFSTFAMS WVRQAPGKGLEWVSAISRSGGNTHYADSVKGRFTI SRDNSKNTLYLQMNSLRAEDTAVYSCAKDGFGGS SYFDYWGQGTLVTVSS |
| | | | SEQ ID NO: 26770 | SEQ ID NO: 30776 |
| iPS:436015 | 21-225_193D3 | NA | GAAATTGTGTTGACGCAGTCTCCAGGCACCCT GTCTTTGTCTCCAGGGGAAAGAGCCACCCTCT CCTGCAGGGCCAGTCAGAGTATTCGCAGCAGC TTCTTAGCCTGGTATCAGCAGAAACCTGGCCA GGCTCCCAGGCTCCTCATCTATGGTGCATCCA ACAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAACTCACCG TGGGCGTTCGGCCAAGGGACCAAGGTGGAAA TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGATCCGCCAGCTCCCAGGGAA GGGCCTGGAGTGGATTGGGTACATCTTTTACAGT GGGAGCACCTACTACAACCCGTCCCTCAAGAGTC GACTTACCATATCAGTGGACACGTCTAAGAACCA GTTCTCCCTGAAGCTGAGCTCTGTGACTGCCGCG GACACGGCCGTGTATTACTGTACAAGGGGGGATT ACGATGGTTCGGGGAGTTATCACTTCTACTACGG TATGGACGTCTGGGGCCAAGGGACCACGGTCAC CGTCTCCTCA |
| | | | SEQ ID NO: 26771 | SEQ ID NO: 30777 |

FIGURE 50

| | | | | |
|---|---|---|---|---|
| | | AA | EIVLTQSPGTLSLSPGERATLSCRASQSIRSSFLA WYQQKPGQAPRLLIYGASNRATGIPDRFSGSGS GTDFTLTISRLEPEDFAVYYCQQYGNSPWAFGQ GTKVEIK | QVQLQESGPGLVKPSQTLSLTCTVSGGSISSGDYYW NWIRQLPGKGLEWIGYIFYSGSTYYNPSLKSRLTISV DTSKNQFSLKLSSVTAADTAVYYCTRGDYDGSGSY HFYYGMDVWGQGTTVTVSS |
| | | | SEQ ID NO: 26772 | SEQ ID NO: 30778 |
| iPS:436017 | 21-225_193F3 | NA | GAAATTGTGTTGACGCAGTCTCCAGGCACCCT GTCTTTGTCTCCAGGGGAAAGCGCCACCCTCT CCTGCAGGGCCGGTCAAAGTATTAGAACCGAC TTCTTAGTCTGGTACCAGCAGCAACCTGGCCA GGCTCCCAGGCTCCTCATCTATGGTGCATCCA GCAGGGCCACTGGCTTCCCAGAGAGGTTCAGT GGCAGTGGGTCTGGGACAGACTTCACTCTCAC CATCAACAGACTGGAGCCTGAAGATTTTGCAG TGTATTACTGTCAGCAGTATGGAAGCTCACCG TGGACGTTCGGCCAAGGGACCAAGGTGGAAA TCAAT | CAGGTGCAACTGCAGGAGTCGGGCCCAGGACTG GTGAAGCCTTCACAGACCCTGTCCCTCACCTGCA CTGTCTCTGGTGGCTCCATCAACAGTGGTGATTA CTACTGGAACTGGATCCGCCAGCACCCAGGGAA GGGCCTGGAGTGGATTGGGTACATCTTTTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGGGGATT ACGATGGTTCGGGGAGTTATCACTACTATTACGG TATGGACGTCTGGGGCCAAGGGACCACGGTCAC CGTCTCCTCA |
| | | | SEQ ID NO: 26773 | SEQ ID NO: 30779 |
| | | AA | EIVLTQSPGTLSLSPGESATLSCRAGQSIRTDFLV WYQQQPGQAPRLLIYGASSRATGFPERFSGSGSG TDFTLTINRLEPEDFAVYYCQQYGSSPWTFGQGT KVEIN | QVQLQESGPGLVKPSQTLSLTCTVSGGSINSGDYY WNWIRQHPGKGLEWIGYIFYSGSTYYNPSLKSRVTI SVDTSKNQFSLKLSSVTAADTAVYYCARGDYDGS GSYHYYYGMDVWGQGTTVTVSS |
| | | | SEQ ID NO: 26774 | SEQ ID NO: 30780 |

FIGURE 50

| iPS:436019 | 21-225_193C4 | NA | GACATCCAGATGACCCAGTCTCCATCCTCCCT GTCTGCATCTGTTGGAGACAGAGTCACCATCA CTTGCCGGCCGAGTCAGGGCATTAGCATTTAT TTAGCCTGGTATCAGCAGAAACCAGGGAATGT TCCTAAGCTCCTGATCTATGCTGCATCCACTTT ACAATCAGGGGTCCCATCTCGGTTCAGTGGCA GTGGATCTGGGACAGATTTCACTCTCACCATC AGCAGCCTGCAGCCTGAAGATGTTGCAACTTA TTACTGTCAAAAGTATAACAGTGCCCCATTCA CTTTCGGCCCTGGGACCAAAGTGGATATCAAA | GAGGTGCAGCTGTTGGAGTCTGGGGGAGGCTTG GCACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGCTATTATTGGTAATGGTGGT AGAACATACTACGCAGACTCCGTGAAGGGCCGG TTCTCCATCTCCAGAGACAATTCCAAGAACACGC TGTTTCTGCAAATGAACAGCCTGAGAGCCGAGG ACACGGCCGTATATTACTGTGCGAAAGATCTGGG TAGATACAGCTATGGTTTCTTTGACTACTGGGGC CAGGGAACCCTGGTCACCGTCTCCTCA |
| | | | SEQ ID NO: 26775 | SEQ ID NO: 30781 |
| | | AA | DIQMTQSPSSLSASVGDRVTITCRPSQGISIYLAW YQQKPGNVPKLLIYAASTLQSGVPSRFSGSGSGT DFTLTISSLQPEDVATYYCQKYNSAPFTFGPGTK VDIK | EVQLLESGGGLAQPGGSLRLSCAASGFTFSSYAMN WVRQAPGKGLEWVSAIIGNGGRTYYADSVKGRFSI SRDNSKNTLFLQMNSLRAEDTAVYYCAKDLGRYS YGFFDYWGQGTLVTVSS |
| | | | SEQ ID NO: 26776 | SEQ ID NO: 30782 |
| iPS:436021 | 21-225_193G4 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATTATAACTACTTGACTTGGTACCA GCAGAAACCAGGACAGCCTCCTAAGCTGCTCA TTTACTGGGCATCTACCCGAAAATCCGGGGTC CCTGACCGATTCAGTGGCAGCGGGTCTGGGAC AGATTTCACTCTCACCATCAGCAGCCTGCAGG CTGACGATGTGGCAGTTTATTACTGTCAGCAA TATTATATTACTCCGTGGACGTTCGGCCAAGG GACCAAGGTGGACATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGAA CAGCCTACATGGAGCTGAACAGCCTGAGATCTG AGGACACGGCCGTGTATTACTGTGCGAGTAGCA GTGGCTGGTACTTCTTTGACTACTGGGGCCAGGG AACCCTGGTCACCGTCTCCTCA |
| | | | SEQ ID NO: 26777 | SEQ ID NO: 30783 |

FIGURE 50

| | | | | |
|---|---|---|---|---|
| | | AA | DIVMTQSPDSLAVSLGERATINCKSSQSVLYSSN NYNYLTWYQQKPGQPPKLLIYWASTRKSGVPD RFSGSGSGTDFTLTISSLQADDVAVYYCQQYYIT PWTFGQGTKVDIK | QVQLVQSGAEVKKPGASVKVSCKASGYTFTNYDIN WVRQATGQGLEWMGWMNPNSGNTGYAQKFQGR VTMTRNTSIRTAYMELNSLRSEDTAVYYCASSSGW YFFDYWGQGTLVTVSS |
| | | | SEQ ID NO: 26778 | SEQ ID NO: 30784 |
| iPS:436023 | 21-225_193A5 | NA | GACATCCAGATGACCCAGTCTCCATCCTCCCT GTTTGCATCTGTAGGAGACAGAGTCATCATCT CTTGCCGGGCAAGTCAGGGCATTAGAAATGAT TTAGGGTGGTATCAGCAGTATCCAGGGAAAGC CCCTAAGCGCGTGATTTATGCTGCATCCAGTTT GCAAAGTGGGGTCCCATCAAGGTTCAGCGGCA GTGGATTTGGGACAGAATTCACTATCACAATC AGCAGCGTGCAGCCTGAAGATTTTGAAACTTA TTACTGTCTACAGCATAATGATTTCCCGTTCAC TTTCGGCGGAGGGACCAAGGTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCATCAGTTATGATA TCAACTGGGTGCGACAGGCCACTGGACAAGGGC TTGAGTGGATGGGATGGATGAACCCTAAAAGGG GTAACACAGGCTATGCACAGAAGTTCCAGGGCA GAGTCACCATGACCAGGGACACCTCCATAAGCA CAGCCTACATGGAGCTGAGCAGCCTGAGATCTG AGGACACGGCCGTTTATTACTGTGCGAGAGGAG ACCCGTATAACTGGAACTCCTACGCTATGGACGT CTGGGGCCAAGGGGCCACGGTCACCGTCTCCTCA |
| | | | SEQ ID NO: 26779 | SEQ ID NO: 30785 |
| | | AA | DIQMTQSPSSLFASVGDRVIISCRASQGIRNDLG WYQQYPGKAPKRVIYAASSLQSGVPSRFSGSGF GTEFTITISSVQPEDFETYYCLQHNDFPFTGGGT KVEIK | QVQLVQSGAEVKKPGASVKVSCKASGYTFISYDIN WVRQATGQGLEWMGWMNPKRGNTGYAQKFQGR VTMTRDTSISTAYMELSSLRSEDTAVYYCARGDPY NWNSYAMDVWGQGATVTVSS |
| | | | SEQ ID NO: 26780 | SEQ ID NO: 30786 |

FIGURE 50

| | | | | |
|---|---|---|---|---|
| iPS:436025 | 21-225_193B5 | NA | GAAATTGTGCTGACTCAGTCTCCAGACTTTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCACCCTCACCAT CAATAGCCTGGAAGCTGAAGATGCTGCAACGT ATTACTGTCATCAGAGTAGTCGTTTACCATTCA CTTTCGGCCCTGGGACCAAAGTGGATATCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGTAGACACGTCTAAGAACCA GTTCTCCCTGAAGCTGAGCTCTGTGGCTGCCGCG GACACGGCCGTGTATTATTGTGCGAGAGGAGAGT ATAACTGGAACCACGGTATGGACGTCTGGGGCC AAGGGACCACGGTCACCGTCTCCTCA |
| | | | SEQ ID NO: 26781 | SEQ ID NO: 30787 |
| | | AA | EIVLTQSPDFQSVTPKEKVTITCRASQSIGSSLHW YQQKPDQSPKLLIKYASQSFSGVPSRFSGSGSGT DFTLTINSLEAEDAATYYCHQSSRLPFTFGPGTK VDIK | QVQLQESGPGLVKPSQTLSLTCTVSGGSISSGGYYW SWIRQHPGKGLEWIGYIYYSGSTYYNPSLKSRVTIS VDTSKNQFSLKLSSVAAADTAVYYCARGEYNWNH GMDVWGQGTTVTVSS |
| | | | SEQ ID NO: 26782 | SEQ ID NO: 30788 |
| iPS:436027 | 21-225_193E6 | NA | GAAATTGTGTTGGCGCAGTCTCCAGGCACCCT GTCTTTGTCTCCAGGGGAAAGAGCCACCCTCT CCTGCAGGGCCAGTCAGAGTGTTAGGAGCGGT TACTTAGCCTGGTACCAGCAGAAACCTGGCCA GGCTCCCAGGCTCCTCATCTATGGTGCATCCA GCAGGGCCACTGGCATCCCAGACAGGTTCGGT GGCAGTGGGTCTGGGACAGACTTCACTCTCAC CATCAGCAGACTGGAGCCTGAAGATTTTTGCAG TGTATTACTGTCAGCAGTATGAGAGTTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCTTTGGTGGGTCCTTCAGTGGTCCCTACTGG AGTTGGATCCGCCAGCCCCCAGGGAAGGGGCTG GAGTGGATTGGGGAATCCAATCATAGTGGACGC ACCAACTACAACCCGTCCCTCAAGAGTCGAGTCA CCATATCAGTGGACACGTCCAAGAACCAGTTCTC CCTGAGGCTGAGCTCTGTGACCGCCGCGGACACG GCTGTGTATTACTGTGCGAGAGACTACGGTGGTT TGGACTACTGGGGCCAGGGAACCCTGGTCACCGT CTCCTCA |
| | | | SEQ ID NO: 26783 | SEQ ID NO: 30789 |

2288

FIGURE 50

| | | | | |
|---|---|---|---|---|
| | | AA | EIVLAQSPGTLSLSPGERATLSCRASQSVRSGYL AWYQQKPGQAPRLLIYGASSRATGIPDRFGGSG SGTDFTLTISRLEPEDFAVYYCQQYESSPWTFGQ GTKVEIK | QVQLQQWGAGLLKPSETLSLTCAVFGGSFSGPYWS WIRQPPGKGLEWIGESNHSGRTNYNPSLKSRVTISV DTSKNQFSLRLSSVTAADTAVYYCARDYGGLDYW GQGTLVTVSS |
| | | | SEQ ID NO: 26784 | SEQ ID NO: 30790 |
| iPS:436029 | 21-225_193H6 | NA | GAAATTGTGTTGACGCAGTCTCCAGGCACCCT GTCTTTGTCTCCAGGGGAAAGCGCCACCCTCT CCTGCAGGGCCGGTCAAAGTATTAGAACCAAC TTCTTAGCCTGGTACCAGCAGCAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAACAGACTGGAGCCTGAAGATTTTGCAG TGTATTTCTGTCAGCAGTATGGTAGCTCACCGT GGACGTTCGGCCAAGGGACCAAGGTGGAAAT CAAT | CAGGTGCAACTGCAGGAGTCGGGCCCAGGACTG GTGAAGCCTTCACAGACCCTGTCCCTCACCTGCA CTGTCTCTGGTGGCTCCATCAACAGTGGTGATTA CTACTGGAACTGGATCCGCCAGCACCCAGGGAA GGGCCTGGAGTGGATTGGGTACATCTTTTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGGGGATT ACGATGGTTCGGGGAGTTATCACTACTATTACGG TATGGACGTCTGGGGGCCAAGGGACCACGGTCAC CGTCTCCTCA |
| | | | SEQ ID NO: 26785 | SEQ ID NO: 30791 |
| | | AA | EIVLTQSPGTLSLSPGESATLSCRAGQSIRTNFLA WYQQQPGQAPRLLIYGASSRATGIPDRFSGSGSG TDFTLTINRLEPEDFAVYFCQQYGSSPWTFGQGT KVEIN | QVQLQESGPGLVKPSQTLSLTCTVSGGSINSGDYY WNWIRQHPGKGLEWIGYIFYSGSTYYNPSLKSRVTI SVDTSKNQFSLKLSSVTAADTAVYYCARGDYDGS GSYHYYYGMDVWGQGTTVTVSS |
| | | | SEQ ID NO: 26786 | SEQ ID NO: 30792 |

FIGURE 50

| iPS:436031 | 21-225_193C7 | NA | GACATCCAGATGACCCAGTCTCCATCCTCACT GTCTGCATCTGTAGGAGACAGAGTCACCATCA CTTGTCGGGCGAGTCAGGGCATTGGCAAGTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAGTACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTACGCAGACTCCGTGAAGGGCCGA TTCACCATCTCCAGAGACAATTCCAAGAACACGC TGTATCTGCAAATGAACAGCCTGAGAGCCGAGG ACACGGCTATGTATTACTGTGCGAGAGATCAGGG CGTGGGCTACGACGGCTTGGACGTCTGGGGCCA AGGGACCTCGGTCACCGTCTCCTCA |
| | | | SEQ ID NO: 26787 | SEQ ID NO: 30793 |
| | | AA | DIQMTQSPSSLSASVGDRVTITCRASQGIGKYLA WFQQKPGKAPKSLIYAASSLQSGVPSKFSGSGSG TDFTLTISSLQPEDFATYYCQQYSTYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAMYYCARDQGV GYDGLDVWGQGTSVTVSS |
| | | | SEQ ID NO: 26788 | SEQ ID NO: 30794 |
| iPS:436033 | 21-225_193E7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTAATCTATTCTGCATCCAGTT TGCAAAGGGGGGTCCCATCAAGATTCAGCGGC AGTGGATCTGGGACAGAATTCACTCTCACAAT CAGCAGCCTGCAGCCTGAAGATTTTGCAACTT ATTACTGTCTACAGCATAAAAGGTACCCGCTC ACTTTCGGCGGAGGGACCAAGGTGGAGATCA AA | CAGGTGCAGCTGCTACAGCAGTGGGGCGCAGGACTG TTGAAGCCTTCGGGAGACCCTGTCCCTCACCTGCG CTGTCTATGGTGGGTCCTTCAGTGGTTACTTCTGG ACCTGGATCCGCCAGCCCCCAGGGAAGGGGCTG GAGTGGATTGGGGAAATCAATCATAGTGGAAGC ACCAACTACAACCCGTCCCTCAAGAGTCGCGTCA CCATATCAGTAGACACGTCCAAGAACCAGTTCTC CCTGAAGCTGAGCTCTGTGACCGCCGCGGACACG GCTGTGTATTACTGTGCGAGAGACTACGGTGCTG ACTACTGGGGCCAGGGAACCCTGGTCGCCGTCTC CGCA |
| | | | SEQ ID NO: 26789 | SEQ ID NO: 30795 |

FIGURE 50

| | | | | |
|---|---|---|---|---|
| | | AA | DIQMTQSPSSLSASVGDRVTITCRASQGIRNDLG WYQQKPGKAPKRLIYSASSLQRGVPSRFSGSGS GTEFTLTISSLQPEDFATYYCLQHKRYPLTFGGG TKVEIK | QVQLQQWGAGLLKPSETLSLTCAVYGGSFSGYFW TWIRQPPGKGLEWIGEINHSGSTNYNPSLKSRVTISV DTSKNQFSLKLSSVTAADTAVYYCARDYGADYWG QGTLVAVSA |
| | | | SEQ ID NO: 26790 | SEQ ID NO: 30796 |
| iPS:436035 | 21-225_193C8 | NA | GAAATTGTGTTGACGCAGTCTCCAGGCACCCT GTTTTTGTCTCCAGGGGAAAGAGCCACCCTCT CCTGCAGGGCCAGTCAGAGTATTCGCAGCAGC TTCTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAATATGGTAACTCACCG TGGGCGTTCGGCCAAGGGATCAAGGTGGAAG TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGATCCGCCAGCACCCAGGGAA GGGCCTGGAGTGGATTGGGTACATCTTTTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGGGGATT ACGATGGTTCGGGGAGTTATCACTACTACTACGG TATGGACGTCTGGGGGCCAAGGGACCACGGTCAC CGTCTCCTCA |
| | | | SEQ ID NO: 26791 | SEQ ID NO: 30797 |
| | | AA | EIVLTQSPGTLFLSPGERATLSCRASQSIRSSFLA WYQQKPGQAPRLLIYGASSRATGIPDRFSGSGSG TDFTLTISRLEPEDFAVYYCQQYGNSPWAFGQGI KVEVK | QVQLQESGPGLVKPSQTLSLTCTVSGGSISSGDYYW NWIRQHPGKGLEWIGYIFYSGSTYYNPSLKSRVTIS VDTSKNQFSLKLSSVTAADTAVYYCARGDYDGSG SYHYYYGMDVWGQGTTVTVSS |
| | | | SEQ ID NO: 26792 | SEQ ID NO: 30798 |

FIGURE 50

| iPS:436037 | 21-225_193D8 | NA | GAAATTGTGTTGAAGCAGTCTCCAGGCACCCT GTTTTTGTCTCCAGGGGAGAGAGCCACCCTCT CCTGCAGGGCCAGTCAGAGTATAAGGACCAA CTTCTTAGCCTGGTACCAGCAGAAACCTGGCC AGGCTCCCAGGCTCCTCATCTATGGTGCATCC AGCAGGGCCACTGGCATCCCAGACAGGTTCAG TGGCAGTGGGTCTGGGACAGACTTCACTCTCA CCATCAACAGACTGGAGCCTGAAGATTTTGCA GTGTATTACTGTCAGCAGTATGGTAACTCACC GTGGACGTTCGGCCAAGGGACCAAGGTGGAA ATCAAA | CAGGTGCAGCTGCAGGAGTCGGGCCCAGGACTG GTGAAGCCTTCACAGACCCTGTCCCTCACCTGCA CTGTCTCTGGTGGCTCCATCAACAGTGGTGGTTA CTACTGGAACTGGATCCGCCAGCACCCAGGGAA GGGCCTGGAGTGGATTGGGTTCATCTTTTACAGT GGGAGCACCTACTACAACCCGTCCCTCAAGAGTC GAGTTTCCATATCAGTAGACACGTCTAAGAACCA GTTCTCCCTGAAGCTGAGCTCTGTGACTGCCGCG GACACGGCCGTGTATTACTGTGCGAGAGGGGATT ACGATGGTTCGGGGAGTTATCACTACTATTACGG TATGGACGTCTGGGGCCAGGGGACCACGGTCAC CGTCTCCTCA |
| | | | SEQ ID NO: 26793 | SEQ ID NO: 30799 |
| | | AA | EIVLKQSPGTLFLSPGERATLSCRASQSIRTNFLA WYQQKPGQAPRLLIYGASSRATGIPDRFSGSGSG TDFTLTINRLEPEDFAVYYCQQYGNSPWTFGQG TKVEIK | QVQLQESGPGLVKPSQTLSLTCTVSGGSINSGGYY WNWIRQHPGKGLEWIGFIFYSGSTYYNPSLKSRVSI SVDTSKNQFSLKLSSVTAADTAVYYCARGDYDGS GSYHYYYGMDVWGQGTTVTVSS |
| | | | SEQ ID NO: 26794 | SEQ ID NO: 30800 |
| iPS:436039 | 21-225_193F8 | NA | GACATCCAGATGATCCAGTCTCCATCCTCACT GTCTGCATCTGTAGGAGACAGAGTCACCATCA CTTGTCGGGCGAGTCAGGGCGTTAGCAATCAT TTAGCCTGGTTTCAGCAGAAACCAGGGAGAGC CCCTAAGTCCCTGATCTATGCTGCATCCAGTTT GCAAAGTGGGGTCCCATCAAAGTTCAGCGGCA GTGGATCTGGGGCAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTATCTATGGCAT GGACTGGGTCCGCCAGGCTCCAGGCAAGGGGCT GGAGTGGGTGGCAGTTATATGGTATGATGGAAGT TATAAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTATTGTGTGAGAGAGGATTCC CCTTATAGTGGCTACGGCTTGGACTACTACTACG GTATGGACGTCTGGGGCCAAGGGACCACGGTCA CCGTCTCCTCA |
| | | | SEQ ID NO: 26795 | SEQ ID NO: 30801 |

FIGURE 50

| | | | | |
|---|---|---|---|---|
| | | AA | DIQMIQSPSSLSASVGDRVTITCRASQGVSNHLA WFQQKPGRAPKSLIYAASSLQSGVPSKFSGSGSG ADFTLTISSLQPEDFATYYCQQYNSYPFTFGPGT KVDIK | QVQLVESGGGVVQPGRSLRLSCAASGFTFSIYGMD WVRQAPGKGLEWVAVIWYDGSYKYYADSVKGRF TISRDNSKNTLYLQMNSLRAEDTAVYYCVREDSPY SGYGLDYYYGMDVWGQGTTVTVSS |
| | | | SEQ ID NO: 26796 | SEQ ID NO: 30802 |
| iPS:436041 | 21-225_193G8 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGAACCAAC TTCTTAGCCTGGCACCAGCAGAAACCTGGCCA GGCTCCCAGGCTCCTCATCTATGGTGCATCCC GCAGGGCCACTGGCATCCCAGACAGGTTCAGT GGCAGTGGGTCTGGGACAGACTTCACTCTCAC CATCAACAGACTGGAGCCTGAAGATTTTGCAC TGTATTACTGTCAGCAGTATGGTAACTCACCG TGGACGTTCGGCCAAGGGACCAAGGTGGAAA TCAAA | CAGGTGCAGCTGCAGGAGAAAGGCCCAGGACTG GTGAAGCCTTCACAGACCCTGTCCCTCACCTGCA CTGTCTCTGGTGGCTCCGTCAGCAGTGGTGTTTA CTACTGGAGCTGGATCCGCCAGCACCCAGGGAA GGGCCTGGAGTGGATTGGGAACATCTATTACAGT GGGAGCACCTACAACAACCCGTCCCTCAAGAGT CGAGTTACCATATCAGTAGACACGTCTAAGAACC AGTTCTCCCTGAAGCTGAACTCTGTGACTGCCGC GGACACGGCCGTCTATTACTGTGCGCGAGGGGAT TACGATGGTTCGGGGGAGTTATCACTTCTACTACG GTTTGGACGTCTGGGGCCATGGGACCACGGTCAC CGTCTCCTCA |
| | | | SEQ ID NO: 26797 | SEQ ID NO: 30803 |
| | | AA | EIVLTQSPGTLSLSPGERATLSCRASQSVRTNFLA WHQQKPGQAPRLLIYGASRRATGIPDRFSGSGSG TDFTLTINRLEPEDFALYYCQQYGNSPWTFGQG TKVEIK | QVQLQEKGPGLVKPSQTLSLTCTVSGGSVSSGVYY WSWIRQHPGKGLEWIGNIYYSGSTYNNPSLKSRVTI SVDTSKNQFSLKLNSVTAADTAVYYCARGDYDGS GSYHFYYGLDVWGHGTTVTVSS |
| | | | SEQ ID NO: 26798 | SEQ ID NO: 30804 |

FIGURE 50

| iPS:436043 | 21-225_193G9 | NA | GAAATTGTACTGACTCAGTCTCCAGATTTTCA GTCTGTGACTCCAAAGGAGAAAGTCACCATCA CCTGCCGGGCCAGTCAGAGCATTGGTAGGAGT TTACACTGGTACCAGCAGAGACCAGATCAGTC TCTAAAGCTCCTCATCAAGTATGCTTCCCAGTC ATTCTCAGGGGTCCCCTCGAGGTTCAGTGGCA GTGGATCTGGGACAGATTTCACCCTCACCATC AATAGCCTGGAGGCTGAAGATGCTGCAACGTA TTTCTGTCATCAGAGTAGTCGTTTACCGCTCAC TTTCGGCGGCGGGACCAAGGTGGAGATCAAA | CAGGTGCAGCTGCAGGAGTCGGGCCCAGGACTG GTGAAGCCTTCACAGACCCTGTCCCTCACCTGCA CTGTCTCTGGTGACTCCATCAATAATGGTGGATA CTACTGGAGCTGGATCCGCCAGCACCCAGGGAA GGGCCTGGAGTGGATTGGGTACATCTTTTACAGC GGGAGCACCTACTACAACCCGTCCCTCAAGAGTC GAGTTACCATATCAGTTGACACGTCTAAGAATCA GTTCTCCCTGAAGCTGAACTCTGTGACTGCCGCG GACACGGCCGTGTATTTTTGTGCGAGAGCGGGAT ATAACTGGGACAACGGGTTTGACTACTGGGGCC AGGGAACCCTGGTCACCGTCTCCTCA |
|---|---|---|---|---|
| | | | SEQ ID NO: 26799 | SEQ ID NO: 30805 |
| | | AA | EIVLTQSPDFQSVTPKEKVTITCRASQSIGRSLHW YQQRPDQSLKLLIKYASQSFSGVPSRFSGSGSGT DFTLTINSLEAEDAATYFCHQSSRLPLTFGGGTK VEIK | QVQLQESGPGLVKPSQTLSLTCTVSGDSINNGGYY WSWIRQHPGKGLEWIGYIFYSGSTYYNPSLKSRVTI SVDTSKNQFSLKLNSVTAADTAVYFCARAGYNWD NGFDYWGQGTLVTVSS |
| | | | SEQ ID NO: 26800 | SEQ ID NO: 30806 |
| iPS:436045 | 21-225_193A10 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGAACAGATTTCACTCTCACCATC AGCAGCCTGCAGCCTGAAGATTTTGCAACTTA TTTCTGCCAACATTATCTTACTTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATAGGGG GGTGGGTTACTACGGTTTGGACGTCTGGGGCCAA GGGACCACGGTCACCGTCTCCTCA |
| | | | SEQ ID NO: 26801 | SEQ ID NO: 30807 |

FIGURE 50

| | | | | |
|---|---|---|---|---|
| | | AA | DIQMTQSPSSLSASVGDRVTITCRASQGISNYLA WFQQKPGKAPKSLIYAASSLQSGVPSKFSGSGSG TDFTLTISSLQPEDFATYFCQHYLTYPLTFGGGT KVEIK | QVQLVESGGGVVQPGRSLRLSCAASGFTFSSYGMH WVRQAPGKGLEWVAVIWYDGSNEHYADSVKGRF TISRDNSKNTLYLQMNSLRAEDTAVYYCARDRGV GYYGLDVWGQGTTVTVSS |
| | | | SEQ ID NO: 26802 | SEQ ID NO: 30808 |
| IPS:436047 | 21-225_193B10 | NA | GAAATTGTGTTGACGCAGTCTCCAGGCACCCT GTCTTTGTCTCCAGGGGAAAGAGCCACCCTCT CCTGCAGGGCCAGTCCGAGTGTTAGCAGCAGC TACTTAGCCTGGTACCAGCAAAAACCTGGCCA GGCTCCCAGGCTCGTCATCTATGGTGCATCCA GGAGGGCCACTGGCATCCCAGACAGGTTCAG AGGCAGTGGGTCTGGGACAGACTTCACTCTCA CCATCAGTAGACTGGAGCCTGAGGATTTTGCA GTGTATTACTGTCAGCAGTATGGTAGCTCACC TCCGTGGACGTTCGGCCAAGGGACCAAGGTGG AAATCAAA | GAGGTGCAGTTGGTGGAGTCTGGGGGAGGCCTG GTCAAGCCTGGGGGGTCCCTGAGACTCTCCTGTG CAGCCTCTGGATTCACCTTCAGGGACTATAGCAT GAACTGGGTCCGCCAGGCTCCAGGGAAGGGGCT GGAGTGGGTCTCATCCATTAGTAGTGCTGGTGGT TACATATACTACGCAGACTCACTGAAGGGCCGAT TCACCATCTCCAGAGACAACGCCAAAAACTCACT GTATCTGCAAATGAACAGCCTGAGAGCCGAGGA CACGGCTGTGTATTACTGTGCGAGAGCAACTATG GCCCTTGACTACTGGGGCCAGGGAACCCTGGTCA CCGTCTCCTCA |
| | | | SEQ ID NO: 26803 | SEQ ID NO: 30809 |
| | | AA | EIVLTQSPGTLSLSPGERATLSCRASPSVSSSYLA WYQQKPGQAPRLVIYGASRRATGIPDRFRGSGS GTDFTLTISRLEPEDFAVYYCQQYGSSPPWTFGQ GTKVEIK | EVQLVESGGGLVKPGGSLRLSCAASGFTFRDYSMN WVRQAPGKGLEWVSSISSAGGYIYYADSLKGRFTI SRDNAKNSLYLQMNSLRAEDTAVYYCARATMALD YWGQGTLVTVSS |
| | | | SEQ ID NO: 26804 | SEQ ID NO: 30810 |

FIGURE 50

| iPS:436049 | 21-225_193B12 | NA | GAAATTGTGTTGACGCAGTCTCCAGGCACCCT GTCTTTGTCTCCAGGGGAAAGAGCCACCCTCT CCTGCAGGGCCAGTCAGAGTATTCGCAGCAGC TTCTTAGCCTGGTATCAGCAGAAACCTGGCCA GGCTCCCAGGCTCCTCATCTATGATGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAACTCACCG TGGGCGTTCGGCCAAGGGACCAAGGTGGAAA TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGCTGATTA CTACTGGAACTGGATCCGCCAGCTCCCAGGGAA GGGCCTGGAGTGGATTGGGTACATCTTTTACAGT GGGAGCACCTACTACAACCCGTCCCTCAAGAGTC GACTTACCATATCAGTGGACACGTCTAAGAACCA GTTCTCCCTGAAGCTGAGCTCTGTGACTGCCGCG GACACGGCCGTGTATTACTGTGCGAGAGGGGATT ACGATGGTTCGGGGAGTTATCACTTCTACTACGG TATGGACGTCTGGGGCCAAGGGACCACGGTCAC CGTCTCCTCA |
| | | | SEQ ID NO: 26805 | SEQ ID NO: 30811 |
| | | AA | EIVLTQSPGTLSLSPGERATLSCRASQSIRSSFLA WYQQKPGQAPRLLIYDASSRATGIPDRFSGSGSG TDFTLTISRLEPEDFAVYYCQQYGNSPWAFGQG TKVEIK | QVQLQESGPGLVKPSQTLSLTCTVSGGSISSADYYW NWIRQLPGKGLEWIGYIFYSGSTYYNPSLKSRLTISV DTSKNQFSLKLSSVTAADTAVYYCARGDYDGSGS YHFYYGMDVWGQGTTVTVSS |
| | | | SEQ ID NO: 26806 | SEQ ID NO: 30812 |
| iPS:436051 | 21-225_193G12 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCAGC TTAGCCTGGTATCAGCAGAAACCTGGCCAGGC TCCCAGGATCCTCATCTCTGGTGCATCCACCA GGGCCACTGGTATCCCAGCCAGGTTCAGTGGC AGTGGGTCTGGGACAGAGTTCACTCTCACCAT CAGCAGCCTGCAGTCTGCAGATTTTGCAGTTT ATTACTGCCAGCAGTATAATAACTGGCCGTGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG GAGCGTCTGGATTCACCTTCAGTAGCTATGCCAT GCACTGGGTCCGCCAGGCTCCAGGCAAGGGGCT GGAGTGGGTGGCAGTTATATGGTATGATGGAACT AATAAATACTATGGAGACTCCGTGAAGGGCCGA TTCACCATCTCCAGAGACAATTCCAAGAACACGC TGAATCTGCAAATGAACAGCCTGAGAGCCGAGG ACACGGCTGTCTATTACTGTGCGAGAGATTTCAC TATAACTGGAGCTACATATTTTGACTACTGGGGC CAGGGAACCCTGGTCACCGTCTCCTCA |
| | | | SEQ ID NO: 26807 | SEQ ID NO: 30813 |

FIGURE 50

| | | | | |
|---|---|---|---|---|
| | | AA | EIVMTQSPATLSVSPGERATLSCRASQSVSSSLA WYQQKPGQAPRILISGASTRATGIPARFSGSGSG TEFTLTISSLQSADFAVYYCQQYNNWPCSFGQG TKLEIK | QVQLVESGGGVVQPGRSLRLSCGASGFTFSSYAMH WVRQAPGKGLEWVAVIWYDGTNKYYGDSVKGRF TISRDNSKNTLNLQMNSLRAEDTAVYYCARDFTIT GATYFDYWGQGTLVTVSS |
| | | | SEQ ID NO: 26808 | SEQ ID NO: 30814 |
| iPS:436054 | 21-225_194C1 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGAACAGATTTCACTCTCACCATC AGCAGCCTGCAGCCTGAAGATTTTGCAACTTA TTACTGCCTACATTATCTTACTTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAATAGGGG GGTGGGTTACTACGGTTTGGACGTCTGGGGCCAA GGGACCACGGTCACCGTCTCCTCA |
| | | | SEQ ID NO: 26809 | SEQ ID NO: 30815 |
| | | AA | DIQMTQSPSSLSASVGDRVTITCRASQGISNYLA WFQQKPGKAPKSLIYAASSLQSGVPSKFSGSGSG TDFTLTISSLQPEDFATYYCLHYLTYPLTFGGGT KVEIK | QVQLVESGGGVVQPGRSLRLSCAASGFTFSSYGMH WVRQAPGKGLEWVAVIWYDGSNEHYADSVKGRF TISRDNSKNTLYLQMNSLRAEDTAVYYCARNRGV GYYGLDVWGQGTTVTVSS |
| | | | SEQ ID NO: 26810 | SEQ ID NO: 30816 |

FIGURE 50

| iPS:436056 | 21-225_194C3 | NA | GAAATTGTGCTGACTCAGTCTCCAGACTTTCA GTCTGTGGCTCCAAAGGAGAAAGTCACCATCA CCTGCCGGGCCAGTCAGAGTATTGGTAGTAAC TTACACTGGTACCAGCAGAAACCTGATCAGTC TCCAAAGCTCCTCATCAAGTCTGCTTCCCAGTC CTTCTCAGGGGTCCCCTCGAGGTTCAGTGGCA GTGGATCTGGGACAGATTTCACCCTCACCATC AATAGCCTGGAAACTGAAGATGCTGCAACGTA TTACTGTCAGCAGAGTAGTAGTTTACCGTGGA CGTTCGGCCAAGGGACCAAGGTGGAAATCAA A | CAGGTGCAGCTGCAGGAGTCGGGCCCAGGACTG GTGAAGCCTTCGGAGACCCTGTCCCTCACCTGCA CTGTCTCTGGTGGCTCCATCAATAATTACTACTG GAGCTGGATCCGGCAGCCCGCCGGGAAGGGACT GGAGTGGATTGGGCGTATCTATGCCAGTGGGAGC ACCAACTACAACCCCTCCCTCAAGAGTCGAGTCA CCATGTCAATAGACACGTCCAAGAACCAGTTCTC CCTGAAGCTGAGCTCTGTGACCGCCGCGGACACG GCCGTGCATTACTGTGCGAGAGATCGGGGATACT ATGGCTACTACGGTATGGACGTCTGGGGCCAAG GGACCACGGTCACCATCTCCTCA |
|---|---|---|---|---|
| | | | SEQ ID NO: 26811 | SEQ ID NO: 30817 |
| | | AA | EIVLTQSPDFQSVAPKEKVTITCRASQSIGSNLHW YQQKPDQSPKLLIKSASQSFSGVPSRFSGSGSGT DFTLTINSLETEDAATYYCQQSSSLPWTFGQGTK VEIK | QVQLQESGPGLVKPSETLSLTCTVSGGSINNYYWS WIRQPAGKGLEWIGRIYASGSTNYNPSLKSRVTMSI DTSKNQFSLKLSSVTAADTAVHYCARDRGYYGYY GMDVWGQGTTVTISS |
| | | | SEQ ID NO: 26812 | SEQ ID NO: 30818 |
| iPS:436058 | 21-225_194A4 | NA | GAAATTGTGTTGACGCAGTCTCCAGGCACCCT GTCTTTGTCTCCAGGGGAAAGAGCCACCCTCT CCTGCAGGGCCAGTCGGGGTGTTAGCAACATC TACTTAGCCTGGTACCAACAGAAACCTGGCCA GGCTCCCAGGCTCCTCATCTATGGTGCTTCCA ACAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCACAATGATTACTCAATG TTCACTTTCGGCCCTGGGACCAAAGTGGATAT CAAA | CAGGTGCAGTTGGTGCAATCTGGGACTGAGGTGA AGAAGCCTGGGGCCTCTTTGAAGGTCTCCTGCAA GGCTTCTGGATACACCTTCACCGTCTACTATTTG AACTGGGTGCGACAGGCCCCTGGACAAGGGCTT GAGTGGATGGGATGGATCAACCCTAACAGTGGT GGCACAAACTATGCACAGAAGTTTCAGGGCAGG GTCACCATGACCAGGGACACGTCCATCAGCACA GCCTACATGGAACTGAGCAGGCTGAGATCTGAC GACACGGCCGTGTATTACTGTGCGAGAGGCTACG ATATTTTGACTGGTTGGGGCCAGGGAACCCTGGT CACCGTCTCCTCA |
| | | | SEQ ID NO: 26813 | SEQ ID NO: 30819 |

2298

FIGURE 50

| | | | | |
|---|---|---|---|---|
| | | AA | EIVLTQSPGTLSLSPGERATLSCRASRGVSNIYLA WYQQKPGQAPRLLIYGASNRATGIPDRFSGSGS GTDFTLTISRLEPEDFAVYYCQHNDYSMFTFGPG TKVDIK | QVQLVQSGTEVKKPGASLKVSCKASGYTFTVYYL NWVRQAPGQGLEWMGWINPNSGGTNYAQKFQGR VTMTRDTSISTAYMELSRLRSDDTAVYYCARGYDI LTGWGQGTLVTVSS |
| | | | SEQ ID NO: 26814 | SEQ ID NO: 30820 |
| iPS:436060 | 21-225_194F4 | NA | GATATTGTGATGACCCAGACTCCACTCTCTCT GTCCGTCACCCCTGGACAGCCGGCCTCCATCT CCTGCAAGTCTAGTCAGAGCCTCCTCCATAGT GATGGAAGGACCTATTTGTATTGGTACCTGCA GAAGCCAGGCCAGCCTCCACAGCTCCTGATCT GTGAGGTTTCCAACCGGTTCTCTGGAGTGCCA GATAGGTTCAGTGGCAGCGGGTCAGGGACAG ATTTCACACTGAAAATCAGCCGGGTGGAGGCT GGGGATGTTGGGGTTTATTACTGCATGCAAAG TATACAGCTTCCCTGGACGTTCGGCCAAGGGA CCAAGGTGGAAATCAAA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGTTACCACTG GAGCTGGATCCGGCAGCCCGCCGGGAAGGGACT GGAGTGGATTGGACTTATCTATACCAGTAGGAGC ACCAACTACAACCCCTCCCTCAAGAGTCGAGTCA CCATGTCAGTAGACAGGTCCAAGAGCCAGTTCTC CCTGAAGCTGAGCTCTGTGACCGCCGCGGACACG GCCGTGTATTACTGTGCGAGACTCCGGTATAACT GGAACTTCCCTTACTTTGACTACTGGGGCCAGGG AACCCTGGTCACCGTCTCCTCA |
| | | | SEQ ID NO: 26815 | SEQ ID NO: 30821 |
| | | AA | DIVMTQTPLSLSVTPGQPASISCKSSQSLLHSDGR TYLYWYLQKPGQPPQLLICEVSNRFSGVPDRFSG SGSGTDFTLKISRVEAGDVGVYYCMQSIQLPWT FGQGTKVEIK | QLQLQESGPGLVKPSETLSLTCTVSGGSISSYHWSW IRQPAGKGLEWIGLIYTSRSTNYNPSLKSRVTMSVD RSKSQFSLKLSSVTAADTAVYYCARLRYNWNFPYF DYWGQGTLVTVSS |
| | | | SEQ ID NO: 26816 | SEQ ID NO: 30822 |

FIGURE 50

| | | | | |
|---|---|---|---|---|
| iPS:436062 | 21-225_194E5 | NA | GAAATTGTGTTGACGCAGTCTCCAGGCACCCT GTCTTTGTCTCCAGGGGAAAGCGCCACCCTCT CCTGCAGGGCCGGTCAAAGTATTAGAACCAAC TTCTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAACAGACTGGAGCCTAAAGATTTTGCAG TGTATTACTGTCAGCAGTATGGTAGTTCACCG TGGACGTTCGGCCAAGGGACCAAGGTGGAAA TCAAA<br><br>SEQ ID NO: 26817 | CAGGTGCAACTGCAGGAGTCGGGCCCAGGACTG GTGAAGCCTTCACAGACCCTGTCCCTCACCTGCA CTGTCTCTGGTGGCTCCATCAACAGTGGTGATTA CTACTGGAACTGGATCCGCCACCACCCAGGGAA GGGCCTGGAGTGGATTGGGTACATCTTTTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTATATTACTGTGCGAGAGGGGATT ACGATGGTTCGGGGAGTTATCACTACTATTACGG TATGGACGTCTGGGGCCAAGGGACCACGGTCAC CGTCTCCTCA<br><br>SEQ ID NO: 30823 |
| | | AA | EIVLTQSPGTLSLSPGESATLSCRAGQSIRTNFLA WYQQKPGQAPRLLIYGASSRATGIPDRFSGSGSG TDFTLTINRLEPKDFAVYYCQQYGSSPWTFGQG TKVEIK<br><br>SEQ ID NO: 26818 | QVQLQESGPGLVKPSQTLSLTCTVSGGSINSGDYY WNWIRHHPGKGLEWIGYIFYSGSTYYNPSLKSRVTI SVDTSKNQFSLKLSSVTAADTAVYYCARGDYDGS GSYHYYYGMDVWGQGTTVTVSS<br><br>SEQ ID NO: 30824 |
| iPS:436064 | 21-225_194E6 | NA | GAAATTGTGTTGACGCAGTCTCCAGGCACCCT GTCTTTGTCTCCAGGGGAAAGAGCCACCCTCT CCTGCAGGGCCAGTCAGAGTATTAGAAGCAAC TTCTTAGCCTGGTATCAGCAGAAACCTGGCCA GGCTCCCAGGCTCCTCATCTATGGTGCATCCA GCAGGGCCACTGGCATCCCAGACAGGTTCAGT GGCAGTGTGTCTGGGACAGACTTCACTCTCAC CATCAACAGACTGGAGCCTGAAGATTTTGCAG TGTATTACTGTCAGCAGTATGGTAGCTCACCG TGGACGTTCGGCCAAGGGACCAAGGTGGAAA TCAAA<br><br>SEQ ID NO: 26819 | CAGGTGCAGCTGCAGGAGTCGGGCCCAGGACTG GTGAAGCCTTCACAGACCCTGTCCCTCACCTGCA CTGTCTCTGGTGACTCCATCAACAGTGGTGATTA CTACTGGAACTGGATCCGCCAGCACCCAGGGAA GGGCCTGGAGTGGATTGGATTCATCTTTTACAGT GGGAGCACCTACTACAACCCGTCCCTCAAGAGTC GAGTTACCATATCAATAGACACGTCTAAGAACCA GTTCTCCCTGAAGCTGAGCTCTGTGAATGTCGCG GACACGGCCGTGTATTACTGTGCGAGAGGGGATT ACGATGGTTCGGGGAGTTATCACTACTATTACGG TATGGACGTCTGGGGCCAAGGGACCACGGTCAC CGTCTCCTCA<br><br>SEQ ID NO: 30825 |

FIGURE 50

| | | | | |
|---|---|---|---|---|
| | | AA | EIVLTQSPGTLSLSPGERATLSCRASQSIRSNFLA WYQQKPGQAPRLLIYGASSRATGIPDRFSGSVSG TDFTLTINRLEPEDFAVYYCQQYGSSPWTFGQGT KVEIK | QVQLQESGPGLVKPSQTLSLTCTVSGDSINSGDYY WNWIRQHPGKGLEWIGFIFYSGSTYYNPSLKSRVTI SIDTSKNQFSLKLSSVNVADTAVYYCARGDYDGSG SYHYYYGMDVWGQGTTVTVSS |
| | | | SEQ ID NO: 26820 | SEQ ID NO: 30826 |
| iPS:436066 | 21-225_194B7 | NA | GACATCCAGATGACCCAGTCTCCATCCTCACT GTCTGCATCTGTAGGAGACAGAGTCACCATCA CTTGTCGGGCGAGTCAGGGCATTAGCAAATAT TTAGCCTGGTTTCAGCAGAAACCAGGGAAAGC CCCTAAGTCCCTGATCTATGGTGCATCCAGGT TGCAAAGTGGGGTCCCATCAAAGTTCAGCGGC AGTGGATCTGGGACAGATTTCACTCTCACCAT CAGCAGCCTGCAGCCTGAAGATTTTGCAACTT ATTACTGCCAACATTATCTTAATTACCCTCTCA CCTTCGGCCAAGGGACACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTATTGTGCGAGAGATCGGTC TAAGGGTTACGACGGTATGGACGTCTGGGGCCA AGGGACCACGGTCACCGTCTCCTCA |
| | | | SEQ ID NO: 26821 | SEQ ID NO: 30827 |
| | | AA | DIQMTQSPSSLSASVGDRVTITCRASQGISKYLA WFQQKPGKAPKSLIYGASRLQSGVPSKFSGSGSG TDFTLTISSLQPEDFATYYCQHYLNYPLTFGQGT RLEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDRSKG YDGMDVWGQGTTVTVSS |
| | | | SEQ ID NO: 26822 | SEQ ID NO: 30828 |

FIGURE 50

| iPS:436068 | 21-225_194F7 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTTTCAGCAGAAACCAGGGAAAGC CCCTAAGATCCTGATCTATGCTGCATCCAGTTT GCAAAGTGGGGTCCCATCAAGGTTCAGCGGCA GTGGATCTGGGACAGATTTCACTCTCACCATC AGCAGCCTGCAGCCTGAAGATTTTGCAACTTA CTATTGTCAACAGGCTAACAGTTCCCGTGGA CGTTCGGCCAAGGGACCAAGGTGGAAATCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGACTACTACA TACACTGGGTGCGACAGGCCCCTGGACAAGGGC TTGAGTGGATGGGATGGATCAACCCTAACAATGG TGGCACAAACTATGCACAGAAATTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGAACCC CTTGGTTACTATGGTTCGGGGAGTTATGGGGCCT ACGGTATGGACGTCTGGGGCCAAGGGACCACGG TCACCGTCTCCTCA |
| | | | SEQ ID NO: 26823 | SEQ ID NO: 30829 |
| | | AA | DIQMTQSPSSVSASVGDRVTITCRASQGISRWLA WFQQKPGKAPKILIYAASSLQSGVPSRFSGSGSG TDFTLTISSLQPEDFATYYCQQANSFPWTFGQGT KVEIK | QVQLVQSGAEVKKPGASVKVSCKASGYTFTDYYIH WVRQAPGQGLEWMGWINPNNGGTNYAQKFQGRV TMTRDTSISTAYMELSRLRSDDTAVYYCAREPLGY YGSGSYGAYGMDVWGQGTTVTVSS |
| | | | SEQ ID NO: 26824 | SEQ ID NO: 30830 |
| iPS:436072 | 21-225_194C10 | NA | GAAATTGTGTTGACGCAGTCTCCAGGCACCCT GTCTTTGTCTCCAGGGGAAAGAGCCACCCTCT CCTGCAGGGCCAGTCCGAGTGTTAACAGCGGC TACTTAGCCTGGTACCAGCAGAAGCCTGGCCA GACTCCCAGGCTCCTCATCTTTGGTGCATCCA GCAGGGCCACTGGCATCCCCGACAGGTTCAGT GCCAGTGGGTCTGGGGCAGACTTCACTCTCAC CATCAGTAGACTGGAGCCTGAAGATTTTGCAG TGTATTTCTGTCAGCAGTATGAAAGCTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCTCTGGTGGGTCCTTCAGATATTACTACTGG AGCTGGATCCGCCAGCCCCCCGGGAAGGGGCTG GAGTGGTTTGGGGAAATCAATCATAGTGGAAGC ACCAACTACAACCCGTCCCTCAAGAGTCGAGTCA CCATATCAATAGACACGTCCAAGAACCAGTTCTC CCTGAAGCTGAGGTCTGTGACCGCCGCGGGACAC GGCTGTGTATTACTGTGCGAGAGACTACGGTGCT TTTGATATCTGGGGCCAAGGGACAATGGTCACCG TCTCTTCA |
| | | | SEQ ID NO: 26825 | SEQ ID NO: 30831 |

FIGURE 50

| | | | | |
|---|---|---|---|---|
| | | AA | EIVLTQSPGTLSLSPGERATLSCRASPSVNSGYLA WYQQKPGQTPRLLIFGASSRATGIPDRFSASGSG ADFTLTISRLEPEDFAVYFCQQYESSPWTFGQGT KVEIK | QVQLQQWGAGLLKPSETLSLTCAVSGGSFRYYYW SWIRQPPGKGLEWFGEINHSGSTNYNPSLKSRVTISI DTSKNQFSLKLRSVTAADTAVYYCARDYGAFDIW GQGTMVTVSS |
| | | | SEQ ID NO: 26826 | SEQ ID NO: 30832 |
| iPS:436074 | 21-225_194F10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCTGAAACCAGGGAAAGC CCCTAAGCGCCTGATCTATGCTGCATCCAGTTT ACAAAGTGGGGTCCCATCAAGGTTCAGCGGCA GTGGATCTGGGCCAGAATTCACTCTCACAATC AGCAGCCTGCAGCCTGAAGATTTTGCAACTTA TTACTGTCTACAGCATTATAGTTTCCCATTCAC TTTCGGCCCTGGGACCAAAGTGGATATCAAA | CAGGTGCAGCTGGTGGAGTCGGGGGGAGGCGTG GTCCAGCCTGGGAGGTCCCTGAGACTCTCCTGTG CAGCGTCTGGATTCACCTTCAGTAGCTTTATCAT GCACTGGGTCCGCCAGGCTCCAGGCAGGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGGAAATGAACAGCCTGAGAGTCGAGG ACACGGCTGTGTATTACTGTGCGAGAGAGGAGTA TAGCAGTGGCTGGTTCGGGTACGGTATGGACGTC TGGGGCCAAGGGACCACGGTCACCGTCTCCTCA |
| | | | SEQ ID NO: 26827 | SEQ ID NO: 30833 |
| | | AA | DIQMTQSPSSLSASVGDRVTITCRASQGIRNDLG WYQLKPGKAPKRLIYAASSLQSGVPSRFSGSGSG PEFTLTISSLQPEDFATYYCLQHYSFPFTFGPGTK VDIK | QVQLVESGGGVVQPGRSLRLSCAASGFTFSSFIMH WVRQAPGRGLEWVAVIWYDGSNKYYADSVKGRF TISRDNSKNTLYLEMNSLRVEDTAVYYCAREEYSS GWFGYGMDVWGQGTTVTVSS |
| | | | SEQ ID NO: 26828 | SEQ ID NO: 30834 |

FIGURE 50

| | | | | |
|---|---|---|---|---|
| iPS:436076 | 21-225_194H11 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT ACAAAGTGGGGTCCCATCAAAGTTCAGCGGCA GTGGATCTGGAACAGATTTCACTCTCACCATC AGCAGCCTGCAGCCTGAAGATTTTGCAACTTA TTACTGCCAACATTATCTTACTTACCCTCTCAC TTTCGGCGGAGGGACCAAGGTGGAGATCAAA<br><br>SEQ ID NO: 26829 | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAACACGC TGTCTCTGCAAATGAACAGCCTGAGAGCCGAGG ACACGGCTGTGTATTACTGTGCGAGAGATAGGGG GGGTGGGTTATTACGGTTTGGACGTCTGGGGCCAA GGGACCACGGTCACCGTCTCCTCA<br><br>SEQ ID NO: 30835 |
| | | AA | DIQMTQSPSSLSASVGDRVTITCRASQGISNYLA WFQQKPGKAPKSLIYAASSLQSGVPSKFSGSGSG TDFTLTISSLQPEDFATYYCQHYLTYPLTFGGGT KVEIK<br><br>SEQ ID NO: 26830 | QVQLVESGGGVVQPGRSLRLSCAASGFTFSSYGMH WVRQAPGKGLEWVAVIWYDGSNEHYADSVKGRF TISRDNSKNTLSLQMNSLRAEDTAVYYCARDRGVG YYGLDVWGQGTTVTVSS<br><br>SEQ ID NO: 30836 |
| iPS:436078 | 21-225_194H12 | NA | GACATCCAGATGACCCAGTCTCCATCCTCACT GTCTGCATCTGTGGGAGACAGAGTCACCATCA CTCGTCGGGCGAGTCAGGGCATTGGCAAGTAT TTAGCATGGTTTCAGCAGAAACCAGGGAAAGC CCTTAAGTCACTGATATATGCTGCATCCAGTTT GCAAAGTGGGGTCCCATCAAAATTCAGCGGCA GTGGATCTGGGACAGATTTCACTCTCACCATC AGCAGCCTGCAGCCTGACGATTTTGCAACTTA TTACTGCCAACGATATGATACTTACCCATTCA CTTTCGGCCCTGGGACCAAAGTGGATATCAAA<br><br>SEQ ID NO: 26831 | CAGGTGCAGCTGGTGGAGTCTGGGGGAGGCGTG GTCCAGCCTGGGAGGTCCCTGAGACTCTCCTGTG CAGCGTCTGGATTCACCTTCAGGAACTATGGCAT GCACTGGGTCCGCCAGGCTCCAGGCAAGGGGCT GGAGTGGGTGGCAGTTATATGGTTTGATGGAAGT AATGACTACTATGCAGACTCCGTGAAGGGCCGAT TCACCATCTCCAGAGACAATTCCAAGAACACGCT GTCACTGCAAATGAACAGCCTGAGAGCCGAGGA CACGGCTGTGTATTACTGTGCGAGAGATAGAAGC GTCGGCTACGACGGTTTAGATGTCTGGGGCCAAG GGACCACGGTCACCGTCTCCTCA<br><br>SEQ ID NO: 30837 |

EP 4 435 105 A2

FIGURE 50

| | | | DIQMTQSPSSLSASVGDRVTITRRASQGIGKYLA WFQQKPGKALKSLIYAASSLQSGVPSKFSGSGSG TDFTLTISSLQPDDFATYYCQRYDTYPFTFGPGT KVDIK | QVQLVESGGGVVQPGRSLRLSCAASGFTFRNYGM HWVRQAPGKGLEWVAVIWFDGSNDYYADSVKGR FTISRDNSKNTLSLQMNSLRAEDTAVYYCARDRSV GYDGLDVWGQGTTVTVSS |
|---|---|---|---|---|
| | | AA | SEQ ID NO: 26832 | SEQ ID NO: 30838 |
| IPS:436080 | 21-225_195B1 | NA | GAAATTGTGTTGACGCAGTCTCCAGGCACCCT GTCTTTGTCTTCAGGGGAAAGAGCCACCCTCT CCTGCAGGGCCAGTCCGAGTGTTAACAGTAAC TACTTAGCCTGGTATCAGCAGAAACCTGGCCA GACTCCCAGGCTCCTCATCTATGGTGCATCCA ACAGGGCCACTGGCGTCCCAGACAGGTTCAGT GCCAGTGGGTCTGGGACAGACTTCACTCTCAC CATCAGAAGACTGGAGCCTGAAGATTTTGCAG TGTATTTCTGTCAGCAGTATGAAAGCTCACCG TGGACGTTCGGCCAAGGGACCAAGGTGGAAA TCAAA | CAGGTGCAGTTACAGCAGTGGGGCGCAGGACTG TTGAAGCCTTCGGAGACCCTGTCCCTCACCTGCG CTGTCTCTGGTGGGTCCTTCAGATATTACTTCTGG AGCTGGATCCGCCAGTCCCCCGGGAAGGGGCTG GAGTGGTTTGGGGAAATCAATCATAGTGGACGC ACCAACTACAACCCGTCCCTCAAGAGTCGAGTCA CCATATCAGTAGACACGTCCAAGAACCAGTTCTC CCTGAAGCTGAGGTCTGTGACCGCCGCGGACAC GGCTGTGTATTACTGTGCGAGAGACTACGGTGCT TTTGATATCTGGGGCCAAGGGCACATTGGTCACCG TCTCTTCA |
| | | | SEQ ID NO: 26833 | SEQ ID NO: 30839 |
| | | AA | EIVLTQSPGTLSLSSGERATLSCRASPSVNSNYLA WYQQKPGQTPRLLIYGASNRATGVPDRFSASGS GTDFTLTIRRLEPEDFAVYFCQQYESSPWTFGQG TKVEIK | QVQLQQWGAGLLKPSETLSLTCAVSGGSFRYYFW SWIRQSPGKGLEWFGEINHSGRTNYNPSLKSRVTIS VDTSKNQFSLKLRSVTAADTAVYYCARDYGAFDI WGQGTLVTVSS |
| | | | SEQ ID NO: 26834 | SEQ ID NO: 30840 |

2305

FIGURE 50

| iPS:436082 | 21-225_195D9 | NA | GACATCCAGATGACCCAGTCTCCATCTTCCGT GTCTGCATCGTAGGAGACAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGCTGCATCCAGTT TGCTAGGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACGGGCTAACAGTTTCCCGTGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTATGTCAT GCACTGGGTCCGCCAGGCTCCAGGCAAGGGGCT GGAGTGGGTGGCAGTTATTTGGTATGATGGAACT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATTGGTT CGGGGAGGGGAACTACTACGGTATGGACGTCTG GGGCCAAGGGACCACGGTCACCGTCTCCTCA |
|---|---|---|---|---|
| | | | SEQ ID NO: 26835 | SEQ ID NO: 30841 |
| | | AA | DIQMTQSPSSVSASVGDRVTITCRASQGISRWLA WYQQKPGKAPKLLIYAASSLLGGVPSRFSGSGS GTDFTLTISSLQPEDFATYYCQRANSFPCSFGQG TKLEIK | QVQLVESGGGVVQPGRSLRLSCAASGFTFSHYVM HWVRQAPGKGLEWVAVIWYDGTNKYYADSVKGR FTISRDNSKNTLYLQMNSLRAEDTAVYYCARDWF GEGNYYGMDVWGQGTTVTVSS |
| | | | SEQ ID NO: 26836 | SEQ ID NO: 30842 |
| iPS:436084 | 21-225_195F2 | NA | GAAATTGTGCTGACTCAGTCTCCAGACTTTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCGCCCTCACCAT CAGTAGCCTGGAAGCTGAAGATGCTGCAACGT ATTACTGTCATCAGAGTAGAACTTTACCGCTC ACTTTCGGCGGAGGGACCAAGGTGGAGATCA AA | CAGGTGCAGCTGCAGGAGTCGGGGCCCAGGACTG GTGAAGCCTTCACAGACCCTGTCCCTCACCTGCA CTGTCTCTGGTGACTCCATCAGCAGCGGTGGTTA CTACTGGAGCTGGATCCGCCAGCACCCAGGGAA GGGCCTGGAGTGGATTGGGTACAGCTATTACAGT GGGAGCACCAACTATAACCCGTCCCTCAAGAGTC GAGTTACCATATCAGTAGACATGTCTAAGAACCA GTTCTCCCTGAAGCTGAGCTCTGTGACTGACGCG GACACGGCCGTGTATTATTGTGCGAGAGGGGGGT ATAACTGGAACAACGGGTTTGACTACTGGGGCC AGGGAGCCCTGGTCACCGTCTCCTCA |
| | | | SEQ ID NO: 26837 | SEQ ID NO: 30843 |

FIGURE 50

| | | | | |
|---|---|---|---|---|
| | | AA | EIVLTQSPDFQSVTPKEKVTITCRASQSIGSSLHW YQQKPDQSPKLLIKYASQSFSGVPSRFSGSGSGT DFALTISSLEAEDAATYYCHQSRTLPLTFGGGTK VEIK | QVQLQESGPGLVKPSQTLSLTCTVSGDSISSGGYYW SWIRQHPGKGLEWIGYSYYSGSTNYNPSLKSRVTIS VDMSKNQFSLKLSSVTDADTAVYYCARGGYNWN NGFDYWGQGALVTVSS |
| | | | SEQ ID NO: 26838 | SEQ ID NO: 30844 |
| iPS:436086 | 21-225_191G10 | NA | GACATCCAGATGACCCAGTCTCCTTCCTCACT GTCTGCATCTGTAGGAGACAGAGTCACCATCA CTTGTCGGACGAGTCAGGGCATTGGCAAGTAT TTAGCCTGGTTTCAGCAGAAACCAGGGAAAGC CCCTAAGTCCCTGCTCTATAAAGTATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATATGACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGCCAA GGGACCACGGTCACCGTCTCCTCA |
| | | | SEQ ID NO: 26839 | SEQ ID NO: 30845 |
| | | AA | DIQMTQSPSSLSASVGDRVTITCRTSQGIGKYLA WFQQKPGKAPKSLLYKVSSLQSGVPSKFSGSGS GTDFTLTISSLQPEDFATYYCQQYMTYPLTFGGG TKVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDQGV GYDGLDVWGQGTTVTVSS |
| | | | SEQ ID NO: 26840 | SEQ ID NO: 30846 |

FIGURE 50

| iPS:436088 | 21-225_195C8 | NA | GAAATTGTGTGTTGACGCAGTCTCCAGGCACCCT GTCTTTGTCTCCAGGGGAAAGAGCCACCCTCT CCTGCAGGGCCAGTCAGAGTATTCGCAGCAGC TTCTTAGCCTGGTACCAACAGAAACCTGGCCA GGCTCCCAGGCTCCTCATCTATGGTGCATTTA GTAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAACTCACCG TGGGCGTTCGGCCAAGGGACCAAGGTGGAAA TCAAA <br> SEQ ID NO: 26841 | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGATCCGCCAGCTCCCAGGGAA GGGCCTGGAGTGGATTGGGTACATCTTTTACAGT GGGAGCACCTACTACAACCCGTCCCTCAAGAGTC GACTTACCATATCAGTGGACACGTCTAAGAACCA GTTCTCCCTGAAGCTGAGCTCTGTGACTGCCGCG GACACGGCCGTGTATTACTGTGCGAGAGGGGATT ACGATGGTTCGGGGAGTTATCACTTCTACTACGG TATGGACGTCTGGGGCCAAGGGACCACGGTCAC CGTCTCCTCA <br> SEQ ID NO: 30847 |
|  |  | AA | EIVLTQSPGTLSLSPGERATLSCRASQSIRSSFLA WYQQKPGQAPRLLIYGAFSRATGIPDRFSGSGSG TDFTLTISRLEPEDFAVYYCQQYGNSPWAFGQG TKVEIK <br> SEQ ID NO: 26842 | QVQLQESGPGLVKPSQTLSLTCTVSGGSISSGDYYW NWIRQLPGKGLEWIGYIFYSGSTYYNPSLKSRLTISV DTSKNQFSLKLSSVTAADTAVYYCARGDYDGSGS YHFYYGMDVWGQGTTVTVSS <br> SEQ ID NO: 30848 |
| iPS:436090 | 21-225_195A9 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGAACAGATTTCACTCTCACCATC AGCAGCCTGCAGCCTGAAGATTTTGCAACTTA TTACTGCCAACATTATCTTACTTACCCTCTCAC TTTCGGCGGAGGGACCAAGGTGGAGATCAAA <br> SEQ ID NO: 26843 | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GCAGTGGGTGGCAGTTATATGGTATGATGGAAGT AATGAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATAGGGG GGTGGGTTACTACGGTTTGGACGTCTGGGGGCCAA GGGACCACGGTCACCGTCTCCTCA <br> SEQ ID NO: 30849 |

FIGURE 50

| | | | DIQMTQSPSSLSASVGDRVTITCRASQGISNYLA WFQQKPGKAPKSLIYAASSLQSGVPSKFSGSGSG TDFTLTISSLQPEDFATYYCQHYLTYPLTFGGGT KVEIK | QVQLVESGGGVVQPGRSLRLSCAASGFTFSSYGMH WVRQAPGKGLQWVAVIWYDGSNEHYADSVKGRF TISRDNSKNTLYLQMNSLRAEDTAVYYCARDRGV GYYGLDVWGQGTTVTVSS |
|---|---|---|---|---|
| | | AA | SEQ ID NO: 26844 | SEQ ID NO: 30850 |
| iPS:436092 | 21-225_195B9 | NA | GACATCCAGATGACCCAGTCTCCATCCTCCCT GTCTGCATCTGTAGGAGACAGAGTCACCATCA CTTGCCGGGCAAGTCAGGGCATAAGAAAAGA TTTAGGCTGGTATCAGCAGAAACCAGGGAAA GCCCCTAAGCGCCTGATCTATGCTGCATCCGA TTTGCAAAGTGGGGTCCCATCAAGGTTCAGCG GCAGTGGATCTGGGACAGAATTCACTCTCACA ATCAGCAGCCTGCAGCCTGAAGATTTTGCAAC TTATTGCTGTCTACAGCATTATCGTTACCCATT CACTTTCGGCCCTGGGACCAAAGTGGATTTCA AA | CAGGTGCAACTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAATGGCT ACAATTCAGGTACTACTACGGTATGGACGTCTGG GGCCAAGGGACCACGGTCACCGTCTCCTCA |
| | | | SEQ ID NO: 26845 | SEQ ID NO: 30851 |
| | | AA | DIQMTQSPSSLSASVGDRVTITCRASQGIRKDLG WYQQKPGKAPKRLIYAASDLQSGVPSRFSGSGS GTEFTLTISSLQPEDFATYCCLQHYRYPFTFGPGT KVDFK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCAREWLQF RYYYGMDVWGQGTTVTVSS |
| | | | SEQ ID NO: 26846 | SEQ ID NO: 30852 |

FIGURE 50

| iPS:436094 | 21-225_195B10 | NA | GAAATTGTGCTGACTCAGTCTCCAGACTTTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCACCCTCACCAT CAATAGCCTGGAAGCTGAAGATGCTGCAACGT ATTACTGTCATCAGAGTGGTCGTTTACCGCTC ACTTTCGGCGGAGGGACCAAGGTGGAGATCA AA | CAGGTGCAGCTGCAGGAGTCGGGCCCAGGACTG GTGAAGCCTTCACAGACCCTGTCCCTCACCTGCA CTGTCTCTGGTGGCTCCATCAGCAATAGTGGTTA CTACTGGAGCTGGATCCGCCAGCACCCAGGGAA GGGCCTGGAATGGATTGGGTACATGTATTACAGT GGGAGCACCTACTACAACCCGTCCCTCAAGAGTC GGGTTACCATATCTGTAGACACGTCTAAGAACCA GTTTTATCTGAAGCTGAGCGCTGTGACTGCCGCG GACACGGCCGTGTATTACTGTGCGAAAGGGGGG TATAACTGGAACAATGGGTTTGACTGTTGGGGCC AGGGAACCCTGGTCACCGTCTCCTCA |
| | | | SEQ ID NO: 26847 | SEQ ID NO: 30853 |
| | | AA | EIVLTQSPDFQSVTPKEKVTITCRASQSIGSSLHW YQQKPDQSPKLLIKYASQSFSGVPSRFSGSGSGT DFTLTINSLEAEDAATYYCHQSGRLPLTFGGGTK VEIK | QVQLQESGPGLVKPSQTLSLTCTVSGGSISNSGYYW SWIRQHPGKGLEWIGYMYYSGSTYYNPSLKSRVTI SVDTSKNQFYLKLSAVTAADTAVYYCAKGGYNW NNGFDCWGQGTLVTVSS |
| | | | SEQ ID NO: 26848 | SEQ ID NO: 30854 |
| iPS:436096 | 21-225_195E10 | NA | GAAATTGTGCTGACTCAGTCTCCAGACTTTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCACCCTCACCAT CAATAGCCTGGAAGCTGAAGATGCTGCAACGT ATTACTGTCATCAGAGTAGTCGTTTACCATTCA CTTTCGGCCCTGGGACCAAAGTGGATATCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGTAGACACGTCTAAGAACCA GTTCTCCCTGAAGCTGAGCTCTGTGGCTGCCGCG GACACGGCCGTGTATTATTGTGCGAGAGGGGGGT ATAACTGGAACCACGGTATGGACGTCTGGGGCC AAGGGACCACGGTCACCGTCTCCTCA |
| | | | SEQ ID NO: 26849 | SEQ ID NO: 30855 |

FIGURE 50

| | | | | |
|---|---|---|---|---|
| | | AA | EIVLTQSPDFQSVTPKEKVTITCRASQSIGSSLHW YQQKPDQSPKLLIKYASQSFSGVPSRFSGSGSGT DFTLTINSLEAEDAATYYCHQSSRLPFTFGPGTK VDIK | QVQLQESGPGLVKPSQTLSLTCTVSGGSISSGGYYW SWIRQHPGKGLEWIGYIYYSGSTYYNPSLKSRVTIS VDTSKNQFSLKLSSVAAADTAVYYCARGGYNWN HGMDVWGQGTTVTVSS |
| | | | SEQ ID NO: 26850 | SEQ ID NO: 30856 |
| iPS:436098 | 21-225_195G11 | NA | GACATCGTGATGACCCAGTCTCCAGACTCCCT GGCTGTGTCTCTGGGCGAGAGGGCCACCATCA ACTGCAAGTCCAGCCAGAGTGTTTTATTCAAC TCCAACAATAAGAACTACTTAGCTTGGTACCA GCAGAAACCCGGTCAGCCTCCTAACCTGCTCA TTTACTGGGCATCTACCCTGGAATCTGGGGTC CCTGACCGATTCAGTGGCAGCGGGTGTGGGAC AGATTTCACTCTCACCATCAGCAGCATGCAGG CTGAAGATGTGGCAGTTTATTACTGTCAGCAA TATTGTAGTTTTCCATTCACTTTCGGCCCTGGG ACCAAAGTGGATATCAGA | CAGGTGCAACTGGTGGAGTCTGGGGGAGGCTTG GTCAAGCCTGGAGGGTCCCTGAGACTCTCCTGTG CAGCCTCTGGATTCACCTTCAGTGACTACTACAT GAGCTGGATCCGCCAGGCTCCAGGGAAGGGGCT GGAGTGGCTTTCATACATTAGTAGTAGTGGTACT ACCGTATTCTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGAGAGAATGGGTG GGAGCCGACTACTGGGGCCAGGGAACCCTGGTC ACCGTCTCCTCA |
| | | | SEQ ID NO: 26851 | SEQ ID NO: 30857 |
| | | AA | DIVMTQSPDSLAVSLGERATINCKSSQSVLFNSN NKNYLAWYQQKPGQPPNLLIYWASTLESGVPD RFSGSGCGTDFTLTISSMQAEDVAVYYCQQYCS FPFTFGPGTKVDIR | QVQLVESGGGLVKPGGSLRLSCAASGFTFSDYYMS WIRQAPGKGLEWLSYISSSGTTVFYADSVKGRFTIS RDNAKNSLYLQMNSLRAEDTAVYYCAREWVGAD YWGQGTLVTVSS |
| | | | SEQ ID NO: 26852 | SEQ ID NO: 30858 |

FIGURE 50

| iPS:436100 | 21-225_195G12 | NA | GACATCCAGATGACCCAGTCTCCATCTTCCGT GTCTGCATCTGTAGGAGACAGAGTCACCATCA CTTGTCGGGCGAGTCAGGGTATTAACAACTGG TTAGCCTGGTATCAGCAGAAACCAGGGAAAG CCCCTAAGCTCCTGATCTATGGTGTTTCCAGTT TGCAGAGTGGGGTCCCATCAAGGTTCAGCGGC AGTGGATCTGGGACAGATTTCACTCTCACCAT CAGCAGCCTGCAGCCTGAAGATTTTGCAACTT ACTCTTGTCAACAGGCTAACAGTTTCCCGTGG ACGTTCGGCCGAGGGACCAAGGTGGAAAATC AG | GAGGTGCAGCTGTTGGAGTCTGGGGGAGGCTTG GTACAGCCTGGGGGGTCCCTGAGACTCTCCTGTG CAGCCTCTGGATTCACCTTTAGCACCTATGCCAT GAGTTGGGTCCGCCAGGCTCCAGGGAAGGGGCT GGAGTGGGTCTCAGCTATTAGTCGTAGTGGTGGT AACACACACTACGCAGACTCCGTGAAGGGCCGG TTCACCATCTCCAGAGACAATTCCAAGAATACGC TGTATCTGCAAATGAACAGCCTGAGAGCCGAGG ACACGGCCGTATATTCCTGTGCGAAAGATGGATT CGGTGGGAGCTCCTACTTTGACTACTGGGGCCAG GGAACCCTGGTCACCGTCTCCTCA |
| | | | SEQ ID NO: 26853 | SEQ ID NO: 30859 |
| | | AA | DIQMTQSPSSVSASVGDRVTITCRASQGINNWLA WYQQKPGKAPKLLIYGVSSLQSGVPSRFSGSGS GTDFTLTISSLQPEDFATYSCQQANSFPWTFGRG TKVENQ | EVQLLESGGGLVQPGGSLRLSCAASGFTFSTYAMS WVRQAPGKGLEWVSAISRSGGNTHYADSVKGRFTI SRDNSKNTLYLQMNSLRAEDTAVYSCAKDGFGGS SYFDYWGQGTLVTVSS |
| | | | SEQ ID NO: 26854 | SEQ ID NO: 30860 |
| iPS:436102 | 21-225_196B1 | NA | GACATCGTGATGACCCAGTCTCCAGACTCCCT GGCTGTGTTTCTGGGCGAGAGGGCCACCATCA ACTGCAAGTCCAGCCAGAGTATTTTATTCAGC TCCAACAATAAGAGGTACTTAGCTTGGTATCA GCAGAAACCAGGACAGCCTCCTAAGCTGCTCA TTTACTGGGCATCTATCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTCTAGTCTTCCATTCACTTTCGGCCCTGGG ACCAAAGTGGATATCAAA | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTACAT GAGCTGGATCCGCCAGGCTCCAGGGAAGGGGCT GGAGTGGATTTCATACATTAGTAGTAGTGGTATT ACCATGTACTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGAGAGAATGGGTG GGAGCCGACTACTGGGGCCAGGGAACCCTGGTC ACCGTCTCCTCA |
| | | | SEQ ID NO: 26855 | SEQ ID NO: 30861 |

FIGURE 50

| | | | | |
|---|---|---|---|---|
| | | AA | DIVMTQSPDSLAVFLGERATINCKSSQSILFSSNN KRYLAWYQQKPGQPPKLLIYWASIRESGVPDRF SGSGSGTDFTLTISSLQAEDVAVYYCQQYSSLPF TFGPGTKVDIK | QVQLVESGGGLVKPGGSLRLSCAASGFTFSDYYMS WIRQAPGKGLEWISYISSSGITMYYADSVKGRFTIS RDNAKNSLYLQMNSLRAEDTAVYYCAREWVGAD YWGQGTLVTVSS |
| | | | SEQ ID NO: 26856 | SEQ ID NO: 30862 |
| iPS:436104 | 21-225_196C1 | NA | GACATCGTGATGACCCAGTCTCCAGACTCCCT GGCTGTGTCTCTGGGCGAGAGGGCCACCATCA ACTGCAAGTCCAGCCAGAGTGTTTTATTCAAC TCCAACAATAAGAACTACTTAGCTTGGTACCA GCAGAAACCCGGTCAGCTTCCTAACCTGCTCA TTTACTGGGCATCTACCCTGGAATCTGGGGTC CCTGACCGATTCAGTGGCAGCGGCTGTGGGAC AGATTTCACTCTCACCATCAGCAGCATGCAGG CTGAAGATGTGGCAGTTTATTACTGTCAGCAA TATTGTAGTTTTCCATTCACTTTCGGCCCTGGG ACCAAAGTGGATATCAGA | CAGGTGCAACTGGTGGAGTCTGGGGGAGGCTTG GTCAAGCCTGGAGGGTCCCTGAGACTCTCCTGTG CAGCCTCTGGATTCACCTTCAGTGACTACTACAT GAGCTGGATCCGCCAGGCTCCAGGGAAGGGGCT GGAGTGGCTTTCATACATTAGTAGTAGTGGTACT ACCGTATTCTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGAGAGAATGGGTG GGAGCCGACTACTGGGGCCAGGGAACCCTGGTC ACCGTCTCCTCA |
| | | | SEQ ID NO: 26857 | SEQ ID NO: 30863 |
| | | AA | DIVMTQSPDSLAVSLGERATINCKSSQSVLFNSN NKNYLAWYQQKPGQLPNLLIYWASTLESGVPD RFSGSGSCGTDFTLTISSMQAEDVAVYYCQQYCS FPFTFGPGTKVDIR | QVQLVESGGGLVKPGGSLRLSCAASGFTFSDYYMS WIRQAPGKGLEWLSYISSSGTTVFYADSVKGRFTIS RDNAKNSLYLQMNSLRAEDTAVYYCAREWVGAD YWGQGTLVTVSS |
| | | | SEQ ID NO: 26858 | SEQ ID NO: 30864 |

FIGURE 50

| iPS:436106 | 21-225_196F2 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAACTCCTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGACTAACAGTGTCCCATTC ACTTTCGGCCCTGGGACCAAAGTAGATATCAA A | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTTTGGCAT GCACTGGGTCCGCCAGGCTCCAGGCAAGGGGCT GGAGTGGGTGGCAGTTATATTAAATGATGGAAGT AATAAAAAGTGTGCAGACTCCGTGAAGGGCCGA TGCACCATTTCCAGAGACAACTCCAAGAACACGC TGTATCTGCAAATGAACAGCCTGAGAGCTGAGG ACACGGCTGTTTATTACTGTGCGAGAGGACAGCA GTGGCTGGTAAACGGTGTGGACGTCTGGGGCCA GGGGACCACGGTCACCGTCTCCTCA |
| | | | SEQ ID NO: 26859 | SEQ ID NO: 30865 |
| | | AA | DIQMTQSPSSVSASVGDRVTITCRASQGISSWLA WYQQKPGKAPKLLIYAASSLQSGVPSRFSGSGS GTDFTLTISSLQPEDFATYYCQQTNSVPFTFGPGT KVDIK | QVQLVESGGGVVQPGRSLRLSCAASGFTFSSFGMH WVRQAPGKGLEWVAVILNDGSNKKCADSVKGRC TISRDNSKNTLYLQMNSLRAEDTAVYYCARGQQW LVNGVDVWGQGTTVTVSS |
| | | | SEQ ID NO: 26860 | SEQ ID NO: 30866 |
| iPS:436110 | 21-225_196F4 | NA | GACTTTCAGATGATCCAGTCTCCATCCTCCCTG TCTGCATCTGTAGGCGACAGAGTCACCATCAC TTGCCGGGCAAGTCAGCGCATTCACAGCTATT TAAATTGGTATCAGCAGAAACCAGGGAAAGC TCCTAAGCTCCTGATCTATACTGCATCCAGTTT GCAAGGTGGGGTCCCATCAAGGTTCAGTGGCA GTGGATCTGGGACAGATTTCACTCTCACCATC AGCAGTCTGCAGCCTGAAGATTTTGCAACTTA CTACTGTCAACAGAGCTACGGTTCCCCTCTCA CTTTCGGCGGAGGGACCAAGGTGGAGATCAA A | GAGGTGCAGCTGTTGGAGTCTGGGGGAGGCTTG GTACAGCCTGGGGGGTCCCTGAGATTCTCCTGTG CAGCCTCTGGATTCACCTTTAGCAGCTGTGCCAT GACCTGGGTCCGCCAGGCTCCAGGGAAGGGGCT GGAGTGGGTCTCAGCTATTAGTGGTAGTGGTGGT AGCACATACTACGCAGACTCCGTGAAGGGCCGG TTCACCATTTCCAGAGACAATTCCAAGAACACGC TGTATCTGCAAATGAACAGCCTGAGAGCCGAGG ACACGGCCGTATATTACTGTGCGAAAGTGGGGG GTTTGACTGGCTCCTACTACTACTACGGTATGGA CGTCTGGGGGCCAAGGGACCACGGTCACCGTCTCC TCA |
| | | | SEQ ID NO: 26861 | SEQ ID NO: 30867 |

FIGURE 50

| | | | | |
|---|---|---|---|---|
| | | AA | DFQMIQSPSSLSASVGDRVTITCRASQRIHSYLN WYQQKPGKAPKLLIYTASSLQGGVPSRFSGSGS GTDFTLTISSLQPEDFATYYCQQSYGSPLTFGGG TKVEIK | EVQLLESGGGLVQPGGSLRFSCAASGFTFSSCAMT WVRQAPGKGLEWVSAISGSGGSTYYADSVKGRFTI SRDNSKNTLYLQMNSLRAEDTAVYYCAKVGGLTG SYYYYGMDVWGQGTTVTVSS |
| | | | SEQ ID NO: 26862 | SEQ ID NO: 30868 |
| iPS:436112 | 21-225_196C7 | NA | GACATCCAGATGACCCAGTCTCCATCCTCACT GTCTGCATCTGTAGGAGACAGAGTCACCATCA CTTGTCGGGCGAATCAGGCCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGAACAGATTTCACTCTCACCATC AGCAGCCTGCAGCCTGAAGATTTTGCAACTTA TTACTGCCAACATTATCTCACTTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATAGGGG GGTGGGTTACTACGGTTTGGACGTCTGGGGCCAA GGGACCACGGTCACCGTCTCCTCA |
| | | | SEQ ID NO: 26863 | SEQ ID NO: 30869 |
| | | AA | DIQMTQSPSSLSASVGDRVTITCRANQAISNYLA WFQQKPGKAPKSLIYAASSLQSGVPSKFSGSGSG TDFTLTISSLQPEDFATYYCQHYLTYPLTFGGGT KVEIK | QVQLVESGGGVVQPGRSLRLSCAASGFTFSSYGMH WVRQAPGKGLEWVAVIWYDGSNEHYADSVKGRF TISRDNSKNTLYLQMNSLRAEDTAVYYCARDRGV GYYGLDVWGQGTTVTVSS |
| | | | SEQ ID NO: 26864 | SEQ ID NO: 30870 |

FIGURE 50

| iPS:436114 | 21-225_196G8 | NA | GACATCGTGATGACCCAGTCTCCAGACTCCCT GACTGTGTCTCTGGGCGAGAGGGCCACCGTCA ACTGCAAGTCCAGCCAGAGTGTTTTACACAGC TCCAACAATAACAACTACTTAGCTTGGTACCA GCAGAAACCAGGACAGCCTCCTAAGCTGCTCA TTTACTGGGCATCTACCCGGGAATCCGGAGTC CCTGACCGATTCAGTGGCAGCGGGTCTGGGAC AGATTTCACTCTCGCCATCAGCAGCCTGCAGG CTGAAGATGTGGCGGTTTATTACTGTCAGCAA TATTATAATACTCCTCCGACATTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGATGCGGCAGGCCACTGGTCAAGGGC TTGAGTGGATGGGATGGATGCACCTTAACAGTGG TAACACAGGCTATGCACCGAAGTTCCAGGGCAG AGTCACCATGACCAGGGACACCTCCATAAGCAC AGCCTTCATGGAGCTGAGCAGCCTGAGATCTGAG GACACGGCCGTGTATTACTGTGCCTATAGCGGTG GCTGGTACGTGTTCGACCCCTGGGGCCAGGGAAC CCTGGTCACCGTCTCCTCA |
|---|---|---|---|---|
| | | | SEQ ID NO: 26865 | SEQ ID NO: 30871 |
| | | AA | DIVMTQSPDSLTVSLGERATVNCKSSQSVLHSSN NNNYLAWYQQKPGQPPKLLIYWASTRESGVPD RFSGSGSGTDFTLAISSLQAEDVAVYYCQQYYN TPPTFGQGTKVEIK | QVQLVQSGAEVKKPGASVKVSCKASGYTFTNYDIN WMRQATGQGLEWMGWMHLNSGNTGYAPKFQGR VTMTRDTSISTAFMELSSLRSEDTAVYYCAYSGGW YVFDPWGQGTLVTVSS |
| | | | SEQ ID NO: 26866 | SEQ ID NO: 30872 |
| iPS:436116 | 21-225_196B9 | NA | GACATCCAGATGACCCAGTCTCCATCTTCCGT GTCTGCATCTGTAGGAGACAGAGTCACCATCA CTTGTCGGGCGAGTCAGGGTATTAGCAACTGC TTAGCCTGGTATCAGCAGAAACCAGGGAAAG CCCCTAAGTTCCTGATCTGTGCTGCATCCAGTT TGCAAAGTGCGGTCCCATCAAGGTTCAGCGGC AGTGGATCTGGGACAGATTTCACTCTCACCAT CAGCAGCCTGCAGCCTGAAGATTTTGCATCTT ACTATTGTCAACAGGGTGACAGTTTCCCTCCG ACGTTCGGCCAAGGGACCAAGGTGGAATTCA GA | CAGGTGCAGCTGGTGCAGTCTGGGGCTGAGGTG AAGAAGCCTGGGGCCTCAATGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTTTGCACAGAAGTTTCGGGGCAG GGTCACCATGACCAGGGACACGTCCATCAGCAC AGCCTACATGGAGCTGAGCAGGCTGAGCTCTGA CGACACGGCCGTGTATTACTGTGCGAGAGGGGG GGTTCGGGGAGTTCCCAACTACTACTACGTTATG GACGTCTGGGGCCAAGGGACCACGGTCACCGTC TCCTCA |
| | | | SEQ ID NO: 26867 | SEQ ID NO: 30873 |

FIGURE 50

| | | | | |
|---|---|---|---|---|
| | | AA | DIQMTQSPSSVSASVGDRVTITCRASQGISNCLA WYQQKPGKAPKFLICAASSLQSAVPSRFSGSGSG TDFTLTISSLQPEDFASYYCQQGDSFPPTFGQGTK VEFR | QVQLVQSGAEVKKPGASMKVSCKASGYTFTGYYM HWVRQAPGQGLEWMGWINPNSGGTNFAQKFRGR VTMTRDTSISTAYMELSRLSSDDTAVYYCARGGVR GVPNYYYVMDVWGQGTTVTVSS |
| | | | SEQ ID NO: 26868 | SEQ ID NO: 30874 |
| iPS:436118 | 21-225_196A10 | NA | GACATCCAGATGACCCAGTATCCATCTTACGT GTCTGCATCTGTAGGAGACAGAGTCAGCATCA CTTGTCGGGCGAGTCAGGGTATTAGCAGGTGG TTAGCCTGGTATCAGCAGAAGCCAGGGAAAG CCGCCAAGTTCCTGATCTATGCTGCATCCAGTT TGCTAGGTGGGGTCTCATCAAGGTTCAGCGGC AGTGGATCTGGGACAGATTTCACTCTCACCAT CAGCAGCCTGCAGCCTGAAGATTTTGCAATTT ACTATTGTCAACGGGATAACAGTTTACCGTGC AGTTTTGGCCAGGGGACCAAGCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTATGTCAT GCACTGGGTCCGCCAGGCTCCAGGCAAGGGGCT GGAGTGGGTGGCAGTTATTTGGTATGATGGAACT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATTGGTT CGGGGAGGGGAACTACTACGGTATGGACGTCTG GGGCCAAGGGACCACGGTCACCGTCTCCTCA |
| | | | SEQ ID NO: 26869 | SEQ ID NO: 30875 |
| | | AA | DIQMTQYPSYVSASVGDRVSITCRASQGISRWLA WYQQKPGKAAKFLIYAASSLLGGVSSRFSGSGS GTDFTLTISSLQPEDFAIYYCQRDNSLPCSFGQGT KLEIK | QVQLVESGGGVVQPGRSLRLSCAASGFTFSHYVM HWVRQAPGKGLEWVAVIWYDGTNKYYADSVKGR FTISRDNSKNTLYLQMNSLRAEDTAVYYCARDWF GEGNYYGMDVWGQGTTVTVSS |
| | | | SEQ ID NO: 26870 | SEQ ID NO: 30876 |

FIGURE 50

| iPS:436120 | 21-225_196C10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGTTCTGGGACAGAATTCACTCTCACAA TCAGCAGCCTGCAGCCTGAAGATTTTGCAACT TATTACTGTCTACAATATAATAGTTACCCTCTC ACTTTCGGCGGAGGGACCAAGGTGGAGATCA AA | CAGGTGCAGCTGCAGGAGTCGGGCCCAGGACTG GTGAAGCCTTCACAGACCCTGTCCCTCACCTGCA CTGTCTCTGGTGGCTCCATGAGGAGTGGTGGTGA CTACTGGAGCTGGATCCGCCAGCACCCAGGGAA GGGCCTGGAGTGGTTTGGGTTCATCTATTACAGT GGGAGCACCTACTACAATCCGTCCCTCAAGAGTC GAGTTACCTTATCAGTAGACACGTCTAAGAACCA GTTCTCCCTGAAGCTGAGCTCTGTGACTGCCGCG GACACGGCCGTGTATTACTGTGCGAGAATGGACT ACAGTAACTACTACTACGGTATGGACGTCTGGGG CCAAGGGACCACGGTCACCGTCTCCTCA |
| | | | SEQ ID NO: 26871 | SEQ ID NO: 30877 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQYNSYPLTFGGG TKVEIK | QVQLQESGPGLVKPSQTLSLTCTVSGGSMRSGGDY WSWIRQHPGKGLEWFGFIYYSGSTYYNPSLKSRVT LSVDTSKNQFSLKLSSVTAADTAVYYCARMDYSN YYYGMDVWGQGTTVTVSS |
| | | | SEQ ID NO: 26872 | SEQ ID NO: 30878 |
| iPS:436122 | 21-225_196G10 | NA | GAAATTGTGTTGACGCAGTCTCCAGGCACCCT GTTATTGTCTCCCGGGGAAAGAGCCACCCTCT CCTGCAGGGCCAGTCCGAGTGTTAGCAACAGC TTCTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CAACAGCAGACTGGAGCCTGAAGATTTTGCAG TGTATTACTGTCAGCAGTATGGTAGCTCACCT CCGTGGACGTTCGGCCAAGGGACCAAGGTAG AACTCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGGGACTATAGCAT GAACTGGGTCCGCCAGGCTCCAGGGAAGGGGCT GGAGTGGGTCTCATCTATTAGTAGTGGTAGTGGT TACATACACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGCGAGCAACTAT GGCCCTTGACTACTGGGGCCAGGGAACCCTGGTC ACCGTCTCCTCA |
| | | | SEQ ID NO: 26873 | SEQ ID NO: 30879 |

FIGURE 50

| | | | | |
|---|---|---|---|---|
| | | AA | EIVLTQSPGTLLLSPGERATLSCRASPSVSNSFLA WYQQKPGQAPRLLIYGASSRATGIPDRFSGSGSG TDFTLTNSRLEPEDFAVYYCQQYGSSPPWTFGQ GTKVELK | EVQLVESGGGLVKPGGSLRLSCAASGFTFRDYSMN WVRQAPGKGLEWVSSISSGSGYIHYADSVKGRFTIS RDNAKNSLYLQMNSLRAEDTAVYYCARATMALD YWGQGTLVTVSS |
| | | | SEQ ID NO: 26874 | SEQ ID NO: 30880 |
| iPS:436132 | 21-225_196C12 | NA | GACATCCAGATGACCCTGTCTCCATCGTCCCT GTTTGCATGTGTAGGAGACAGAGTCATCATCA CTTGCCGGGCAAGTCAGGGCATTAGAAATGAT CTAGGCTGGTCTCAGCAGAATCCAGGGAAAGC CCCTAAGCGCCTGATCTATGCTGCATCCAGTTT GCAAAGTGGGGTCCCATCAAGGTTCAGCGGCA GTGGATCTGGGACAGAATTCACTATCACAATC AGCAGCCTGCAGCCTGAAGATTTTGAAACTTA TTACTGTCTACAGCATAATGATTTCCCGTTCAC TTTCGGCCGAGGGACCAAGGTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCATCAGTTATGATA TCAACTGGGTGCGACAGGCCACTGGACAAGGGC TTGAGTGGATGGGATGGATGAACCCTAAAAGGG GTAACACAGGCTATGCACAGAAGTTCCAGGGCA GAGTCACCATGACCAGGGACACCTCCATAAGCA CAGCCTACATGGAGCTGAGCAGCCTGAGATCTG AGGACACGGCCGTTTATTACTGTGCGAGAGGAG ACCCGTATAACTGGAACTCCTACGCTATGGACGT CTGGGGCCAAGGGGCCACGGTCACCGTCTCCTCA |
| | | | SEQ ID NO: 26875 | SEQ ID NO: 30881 |
| | | AA | DIQMTLSPSSLFACVGDRVIITCRASQGIRNDLG WSQQNPGKAPKRLIYAASSLQSGVPSRFSGSGSG TEFTITISSLQPEDFETYYCLQHNDFPFTFGRGTK VEIK | QVQLVQSGAEVKKPGASVKVSCKASGYTFISYDIN WVRQATGQGLEWMGWMNPKRGNTGYAQKFQGR VTMTRDTSISTAYMELSSLRSEDTAVYYCARGDPY NWNSYAMDVWGQGATVTVSS |
| | | | SEQ ID NO: 26876 | SEQ ID NO: 30882 |

FIGURE 50

| iPS:436134 | 21-225_196H12 | NA | GAAATTGTGTTGACGCAGTCTCCAGGCACCCT GTTTTTGTCTCCAGGGGAAAGAGCCACCCTCT CCTGCAGGGCCAGTCAGAGTGTTAGAAGCAAC TTCTTAGCCTGGCACCAGCAGAAACCTGGCCA GGCTCCCAGGCTCCTCATCTATGGTGCATACC GCAGGGCCACTGGCATCCCAGACAGGTTCAGT GGCAGTGGGTCTGGGACAGACTTCACTCTCAC CATCAACAGACTGGAGCCTGAAGATTTTGCAG TGTATTACTGTCAGCAGTATGGTAACTCACCG TGGACGTTCGGCCAAGGGACCAAGGTGGAAA TCAAA | CAGGTGCAGCTGCAGGAGTCGGGCCCAGGACTG GTGAACCCTTCACAGACCCTGTCCCTCACCTGCA CTGTCTCTGGTGGCTCCATCAGCAGTGGTGTTTA CTACTGGAGCTGGATCCGCCAGCACCCAGGGAA GGGCCTGGAGTGGATTGGGAACATCTATTACAGT GGGAGCACCTACAACAACCCGTCCCTCAAGAGT CGAATTATCATATCAGTAGACACGTCTAAGAACC AGTTCTCCCTGAAGCTGAGCTCTGTGACTGCCGC GGACACGGCCGTGTATTACTGTGCGAGAGGGGA TTACGATGGTTCGGGGAGTTATCACTACTACTAC GGTTTGGACGTCTGGGGCCAAGGGACCACGGTC ACCGTCTCCTCA |
| | | | SEQ ID NO: 26877 | SEQ ID NO: 30883 |
| | | AA | EIVLTQSPGTLFLSPGERATLSCRASQSVRSNFLA WHQQKPGQAPRLLIYGAYRRATGIPDRFSGSGS GTDFTLTINRLEPEDFAVYYCQQYGNSPWTFGQ GTKVEIK | QVQLQESGPGLVNPSQTLSLTCTVSGGSISSGVYYW SWIRQHPGKGLEWIGNIYYSGSTYNNPSLKSRIIISV DTSKNQFSLKLSSVTAADTAVYYCARGDYDGSGS YHYYYGLDVWGQGTTVTVSS |
| | | | SEQ ID NO: 26878 | SEQ ID NO: 30884 |
| iPS:436138 | 21-225_197F2 | NA | GACATCCAGATGATCCAGTCTCCTTCCTCACT GTCTGCATCTGTAGGAGACAGAGTCACCATCA CTTGTCGGACGAGTCAGGGCATTGGCAATTAT TTAGCCTGGTTTCAGCAGAAACCAGGGAAAGC CCCTAAGTCCCTGCTCTATAAAACATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTTCCAACAATATATCACTTACCCGCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGACT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGCCAA GGGACCACGGTCACCGTCTCCTCA |
| | | | SEQ ID NO: 26879 | SEQ ID NO: 30885 |

FIGURE 50

| | | | | |
|---|---|---|---|---|
| | | AA | DIQMIQSPSSLSASVGDRVTITCRTSQGIGNYLA WFQQKPGKAPKSLLYKTSSLQSGVPSKFSGSGS GTDFTLTISSLQPEDFATYYFQQYITYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDQGV GYDGLDVWGQGTTVTVSS |
| | | | SEQ ID NO: 26880 | SEQ ID NO: 30886 |
| iPS:436140 | 21-225_197G3 | NA | GACATCCAGATGACCCAGTCTCCGTCCTCACT GTCTGCATCTGTAGGAGACAGAGTCACCATCA CTTGTCGGGCGAGTCAGGGCATTGGCAATCAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTAGATCTGGGACAGATTTCTCTCTCACCATC AGCAGCCTGCAGCCTGAAGATTTTGCAACTTA TTACTGCCAACAGTATAGTAATTACCCGGTCA CTTTCGGCCCTGGGACCAAAGTGGATATCAAG | CAGGTGCAACTGGTGGAGTCTGGGGGAGGCGTG GTCCAGCCTGGGAGGTCCCTGAGACTCTCCTGTG TAGCGTCTGGGTTCACCTTCAGTAGCCATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCCCTC TGTAGGGTACGACGGTATGGACGTCTGGGGCCA AGGGACCACGGTCACCGTCTCCTCA |
| | | | SEQ ID NO: 26881 | SEQ ID NO: 30887 |
| | | AA | DIQMTQSPSSLSASVGDRVTITCRASQGIGNHLA WFQQKPGKAPKSLIYAASSLQSGVPSKFSGSRSG TDFSLTISSLQPEDFATYYCQQYSNYPVTFGPGT KVDIK | QVQLVESGGGVVQPGRSLRLSCVASGFTFSSHGMH WVRQAPGKGLEWVAVIWYDGSNKNYADSVKGRF TISRDNSKNTLYLQMNSLRAEDTAVYYCARDPSVG YDGMDVWGQGTTVTVSS |
| | | | SEQ ID NO: 26882 | SEQ ID NO: 30888 |

FIGURE 50

| iPS:436146 | 21-225_197F4 | NA | GAAATTGTGTTGACGCAGTCTCCAGGCACCCT GTCTTTGTCTCCAGGGGAAGGAGCCACCCTCT CCTGCAGGGCCAGTCAGAGTATTCGCAGCAGC TTCTTAGCCTGGTACCTGCAGAAGCCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGAAACTCACCG TGGGCGTTCGGCCAAGGGACCAAGGTGGAAA TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGATCCGCCAGCACCCAGGGAA GGGCCTGGAGTGGATTGGGTACATCTTTTACAGT GGGAGCACCTACTACAACCCGTCCCTCAAGAGTC GAATTACCATATCAGTAGACACGTCTAAGAACCA GTTCTCCCTGAAGCTGAGCTCTGTGACTGCCGCG GACACGGCCGTGTATTACTGTGCGAGAGGGGATT ACGATGGTTCGGGGAGTTATCACTACTACTACGG TTTGGACGTCTGGGGCCAAGGGACCACGGTCACC GTCTCCTCA |
| | | | SEQ ID NO: 26883 | SEQ ID NO: 30889 |
| | | AA | EIVLTQSPGTLSLSPGEGATLSCRASQSIRSSFLA WYLQKPGQAPRLLIYGASSRATGIPDRFSGSGSG TDFTLTISRLEPEDFAVYYCQQYGNSPWAFGQG TKVEIK | QVQLQESGPGLVKPSQTLSLTCTVSGGSISSGDYYW NWIRQHPGKGLEWIGYIFYSGSTYYNPSLKSRITISV DTSKNQFSLKLSSVTAADTAVYYCARGDYDGSGS YHYYYGLDVWGQGTTVTVSS |
| | | | SEQ ID NO: 26884 | SEQ ID NO: 30890 |
| iPS:436150 | 21-225_197H4 | NA | GACATCATGATGACCCAGTCTTCAGACTCCCT GACTGTGTCTCTGGGCGAGAGGGGCCATCATCA GCTGCAGGTCCAGCCAGAGTGTTTTACACAGC TTCAACAATTACAACTACTTAGCTTGGTACCA GCAGAAAGCAGGACATCCTCCTAACCTGCTCA TTTACTGGGCATCTACCCGGGAATCCGGGGTC CCTGACCGATTCAGTGGCAGCGGGTCTGGGAC AGATTTCACTCTCCCCATCAGCAGCCTGCAGG CTGAAGATGTGGCAGTTTATTACTGTCAACAA TATTATAGTACTCCTCCGACGTTCGGCCTAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCCATAGCAGT GGCTGGTACTACTTTGACTACTGGGGCCAGGGAA CCCTGGTCACCGTCTCCTCA |
| | | | SEQ ID NO: 26885 | SEQ ID NO: 30891 |

FIGURE 50

| | | | | |
|---|---|---|---|---|
| | | AA | DIMMTQSSDSLTVSLGERAIISCRSSQSVLHSFNN YNYLAWYQQKAGHPPNLLIYWASTRESGVPDR FSGSGSGTDFTLPISSLQAEDVAVYYCQQYYSTP PTFGLGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAHSSGW YYFDYWGQGTLVTVSS |
| | | | SEQ ID NO: 26886 | SEQ ID NO: 30892 |
| iPS:436152 | 21-225_197B6 | NA | GACATCCAGATGACCCAGTCTCCATCCTCACT GTCTGCATCTGTAGGAGACAGAGTCACCATCA CTTGTCGGGCGAGTCAGGGCATTGGCAAATAT TTAGCCTGGTTTCAGCAGAAACCAGGGAAAGC CCCTAAGTCCCTGATCTATGGTGCATCCAGTTT GCAAAGTGGGGTCCCATCAAAGTTCAGCGGCA GTGGATCTGGGACAGATTTCACTCTCACCATC AGCAGCCTGCAGCCTGAAAATTTTGCAACTTA TTACTGCCAACAATATAGTACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTACGCAGACTCCGTGAAGGGCCGA TTCACCATCTCCAGAGACAATTCCAAGAACATGT TGTATCTGCAAATGAACAGCCTGAGAGCCGAGG ACACGGCTATGTATTACTGTGCGAGAGATCAGGG CGTGGGCTACGACGGTTTGGACGTCTGGGGCCAA GGGACCTCGGTCACCGTCTCCTCA |
| | | | SEQ ID NO: 26887 | SEQ ID NO: 30893 |
| | | AA | DIQMTQSPSSLSASVGDRVTITCRASQGIGKYLA WFQQKPGKAPKSLIYGASSLQSGVPSKFSGSGSG TDFTLTISSLQPENFATYYCQQYSTYPLTFGGGT KVEIK | QVQLVESGGGVVQPGRSLRLSCAASGFTFSSYGMH WVRQAPGKGLEWVAVIWYDGSNKNYADSVKGRF TISRDNSKNMLYLQMNSLRAEDTAMYYCARDQGV GYDGLDVWGQGTSVTVSS |
| | | | SEQ ID NO: 26888 | SEQ ID NO: 30894 |

FIGURE 50

| iPS:436154 | 21-225_197C6 | NA | GACATCATGATGACCCAGTCTCCAGACTCCCT GGCTGTGTCTCTGGGCGAGAGGGCCATCATCA GCTGCAGGTCCAGCCAGAGTGTTTTACACAGC TCCAACAATTACAACTACTTAGCTTGGTACCA GCAGAAACCAGGACATCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAACAA TATTATAGTACTCCTCCGACGTTCGGCCTAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCCATAGCAGT GGCTGGTACTACTTTGACTACTGGGGCCAGGGAA CCCTGGTCACCGTCTCCTCA |
| | | | SEQ ID NO: 26889 | SEQ ID NO: 30895 |
| | | AA | DIMMTQSPDSLAVSLGERAIISCRSSQSVLHSSNN YNYLAWYQQKPGHPPNLLIYWASTRESGVPDRF SGSGSGTDFTLTISSLQAEDVAVYYCQQYYSTPP TFGLGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAHSSGW YYFDYWGQGTLVTVSS |
| | | | SEQ ID NO: 26890 | SEQ ID NO: 30896 |
| iPS:436156 | 21-225_197C8 | NA | GACATCGTGATGACCCCGTCTCCAGACTCCCT GGCTGTGTCTCTGGGCGAGAGGGCCACCATCA ACTGCAAGTCCAGCCAGAGTGTTTTACACAGT TCCAACAATAAGAACTACTTAGCTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCAGCATCAGCAGCCTGCAGG CTGAAGATGTGGCAGTTTATTACTGTCAGCAG TCTTATACTATTCCATTCACTTTCGGCCCTGGG ACCAAAGTGGATAACAAA | GAGGTGCAACTATTGGAGTCTGGGGGAGGCTTG GTGCAGCCTGGGGGGTCCCTGAGACTCTCCTGTG CAGCCTCTGGATTCACCTTTAGCAGCTCTGCCAT GACCTGGGTCCGCCAGGCTCCAGGGAAGGGACT GGAGTGGGTCTCAGCTATCATTGGTAATGGTGGT AGAGCATACTACGCAGACTCCGTGAAGGGCCGG TTCACCATCTCCAGAGACAATTCCAAGAACACGC TGTATCTGCAAATGAACAGCCTGAGAACCGAGG ACACGGCCGTATATTACTGTGCGAAAGATCGGG GATATAGCAGGATAGCAGTGGCTGGTACCTTTGA CTACTGGGGCCAGGGAACCCTGGTCACCGTCTCC TCA |
| | | | SEQ ID NO: 26891 | SEQ ID NO: 30897 |

FIGURE 50

| | | | | |
|---|---|---|---|---|
| | | AA | DIVMTPSPDSLAVSLGERATINCKSSQSVLHSSN NKNYLAWYQQKPGQPPKLLIYWASTRESGVPD RFSGSGSGTDFTLSISSLQAEDVAVYYCQQSYTIP FTFGPGTKVDNK | EVQLLESGGGLVQPGGSLRLSCAASGFTFSSSAMT WVRQAPGKGLEWVSAIIGNGGRAYYADSVKGRFT ISRDNSKNTLYLQMNSLRTEDTAVYYCAKDRGYSR IAVAGTFDYWGQGTLVTVSS |
| | | | SEQ ID NO: 26892 | SEQ ID NO: 30898 |
| iPS:436158 | 21-225_197G8 | NA | GAGATTGTGATGACCCAGACTCCACTTTCTCT GTCCGTCATCCCTGGACAGCCGGCCTCCATCT CCTGCAAGTCTAGTCAGAACCTCCTGCATAGT GATGGAAAGACCTATTTGTATTGGTACCTGCA GAAGCCAGGCCAGCCTCCACAGGTCCTGATCT ATGAAGTTTCCAACCGGTTCTCTGGGGTGCCA GATAGGTTCAGTGGCAGCGGGTCAGGGACAG ATTTCACACTGAAAATTAGCCGGGTGGAGGCT GAGGATGTTGGGGTTTATTCCTGCATGCAAAG TATACAGCTTCCCTGGACGTTCGCCCAAGGGT CCAAGGTGGAAATCAAA | CAGGTGCATCTGCAGGAGTCGGGCCCAGGACTG GTGAAGCCTTCGGAGACCCTGTCCCTCACCTGCA CTGTCTCTGGTGGCTCCATCAGTGCTTACTCCTGG AGCTGGATCCGGCAGCCCGCCGGGAAGGGACTG GAGTGGATTGGGCGTCTCTCTCCTGGTGGGAGCA CCAACTTCAACCCCTCCCTCAAGAGTCGAGTCAC CATGTCAGTAGACACGTCCAAGAACCAGTTCTCC CTGAGGCTGAGCTCTGTGACCGCCGCGGACACG GCCGTTTATTACTGTGCGAGACTCCGGTATAACT GGAACTTCCCTTACTTTGACTACTGGGGCCAGGG AGCCCTGGTCACCGTCTCCTCA |
| | | | SEQ ID NO: 26893 | SEQ ID NO: 30899 |
| | | AA | EIVMTQTPLSLSVIPGQPASISCKSSQNLLHSDGK TYLYWYLQKPGQPPQVLIYEVSNRFSGVPDRFS GSGSGTDFTLKISRVEAEDVGVYSCMQSIQLPWT FAQGSKVEIK | QVHLQESGPGLVKPSETLSLTCTVSGGSISAYSWSW IRQPAGKGLEWIGRLSPGGSTNFNPSLKSRVTMSVD TSKNQFSLRLSSVTAADTAVYYCARLRYNWNFPYF DYWGQGALVTVSS |
| | | | SEQ ID NO: 26894 | SEQ ID NO: 30900 |

FIGURE 50

| iPS:436160 | 21-225_197C9 | NA | GACATCCAGATGACCCAGTCTCCATCTTCCGT GTCTGCATCTGTAGGAGACAGAGTCACCATCA CTTGTCGGGCGAGTCAGGGTATTAGCAACTGG TTAGCCTGGTTTCAGCAGAAACCAGGGAAAGC CCCTCAGCTCCTGATCTATGCTGCATCCAGTTT GCAAAGTGGGGTCCCATCAAGGTTCAGCGGCA GTGGATCTGGGACAGACTTCAGTCTCACCATC AGCAGCCTGCAGCCTGAAGATTTTGCAACTTA CTATTGTCAACAGGCTAACAGTTTCCCGTGGA CGTTCGGGCAAGGGACCAAGGTGGAAATCAA A | GAGGTGCAGCTGTTGGAGTCTGGGGGAGGCTTG GCACAGCCGGGGGGGTCCCTGAGACTCTCCTGTG CAGCCTCTGGATTCACCTTTAGGAGCTATGCCAT GAGCTGGGTCCGCCAGGCTCCAGGGAAGGGGCT GGAGTGGGTCTCAGTTATTAGTGGTAGAGGTGGT AACACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAAGGCATAGC AGTGGCTGGCTCGCACTACTTTGACTACTGGGGC CAGGGAACCCTGGTCACCGTCTCCTCA |
| | | | SEQ ID NO: 26895 | SEQ ID NO: 30901 |
| | | AA | DIQMTQSPSSVSASVGDRVTITCRASQGISNWLA WFQQKPGKAPQLLIYAASSLQSGVPSRFSGSGSG TDFSLTISSLQPEDFATYYCQQANSFPWTFGQGT KVEIK | EVQLLESGGGLAQPGGSLRLSCAASGFTFRSYAMS WVRQAPGKGLEWVSVISGRGGNTYYADSVKGRFT ISRDNSKNTLYLQMNSLRAEDTAVYYCAKGIAVAG SHYFDYWGQGTLVTVSS |
| | | | SEQ ID NO: 26896 | SEQ ID NO: 30902 |
| iPS:436164 | 21-225_197G10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGGTGCATCCAGT TTACAAAGTGGGGTCCCATCGAGGTTCAGCGG CAGTGGATCTGGGACAGAATTCACTCTCACAA TCAGCAGCCTGCAGCCTGAAGATTTTGCAACT TATTACTGTCTACAGCATTATAGGTACCCATTC ACTTTCGGCCCTGGAACCAAAGTGGATATCAA A | CAGGTGCAGCTGGTGGAGTCTGGGGGAGGCGTG GTCCAGCCTGGGAGGTCCCTGAGACTCTCCTGTG TAGCGTCTGGATTCACCTTCAGTAGCTATGGCAT GCACTGGGTCCGCCAGGCTCCAGGCAAGGGGCT GGAGTGGGTGGCAGTTACATGGTATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGAATGGC TACAATTTAGGTACTACTACGGGTATAGACGTCTG GGGCCAAGGGACCACGGTCACCGTCTCCTCA |
| | | | SEQ ID NO: 26897 | SEQ ID NO: 30903 |

FIGURE 50

| | | | | |
|---|---|---|---|---|
| | | AA | DIQMTQSPSSLSASVGDRVTITCRASQGIRNDLG WYQQKPGKAPKRLIYGASSLQSGVPSRFSGSGS GTEFTLTISSLQPEDFATYYCLQHYRYPFTFGPGT KVDIK | QVQLVESGGGVVQPGRSLRLSCVASGFTFSSYGMH WVRQAPGKGLEWVAVTWYDGSNKYYADSVKGR FTISRDNSKNTLYLQMNSLRAEDTAVYYCAREWLQ FRYYYGIDVWGQGTTVTVSS |
| | | | SEQ ID NO: 26898 | SEQ ID NO: 30904 |
| iPS:436167 | 21-225_197E11 | NA | GACATCCAGATGACCCAGTCTCCATCCTCGCT GTCTGCATCTGTGGGAGACAGAGTCACCATCA CTTGTCGGGCGAGTCAGGGCATTAACAAGTAT TTATCCTGGTTTCAGCAGAAACCAGGGAAAGC CCCTAAGTCATTGATCTATGCTGCATCCAGTGT GCAAAGTGGGGTCCCATCAAAATTCAGCGGCA GTGGATCTGGGACAGATTTCACTCTCACCATC AGCCGCCTGCAGCCTGACGATTTTGCAACTTA TTACGGCCAACGATATGACACTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGGAACTATGGCAT GCACTGGGTCCGCCAGGCTCCAGGCAAGGGGCT GGAGTGGGTGGCAGTTATATGGTTTGATGGAAGT AATGACTACTATGCAGACTCCGTGAAGGGCCGAT TCACCATCTCCAGAGACAATTCCAAGAACACGCT GTCACTGCAAATGAACAGCCTGAGAGCCGAGGA CACGGCTGTGTATTACTGTGCGAGAGATAGAAGC GTCGGCTACGACGGTTTAGATGTCTGGGGCCAAG GGACCACGGTCACCGTCTCCTCA |
| | | | SEQ ID NO: 26899 | SEQ ID NO: 30905 |
| | | AA | DIQMTQSPSSLSASVGDRVTITCRASQGINKYLS WFQQKPGKAPKSLIYAASSVQSGVPSKFSGSGSG TDFTLTISRLQPDDFATYYGQRYDTYPFTFGPGT KVDIK | QVQLVESGGGVVQPGRSLRLSCAASGFTFRNYGM HWVRQAPGKGLEWVAVIWFDGSNDYYADSVKGR FTISRDNSKNTLSLQMNSLRAEDTAVYYCARDRSV GYDGLDVWGQGTTVTVSS |
| | | | SEQ ID NO: 26900 | SEQ ID NO: 30906 |

FIGURE 50

| iPS:436173 | 21-225_197G12 | NA | GACATCCAGATGATCCAGTCTCCTTCCTCACT GTCTGCATCTGTAGGAGACAGAGTCACCATCA CTTGTCGGACGAGTCAGGGCATTGGCAATTAT TTAGCCTGGTTTCAGCAGAAACCAGGGAAAGC CCCTAAGTCCCTGCTCTATAAAACATCCAGTTT GCAAAGTGGGTTCCCATCAAAGTTCAGCGGCA GTGGATCTGGGACAGATTTCACTCTCACCATC AGCAGCCTGCAGCCTGAAGATTTTGCAACTTA TTACTTCCAACAATATATGACTTACCCGCTCAC TTTCGGCGGAGGGACCAAGGTGGAGATCAAA <br><br>SEQ ID NO: 26901 | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGACT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACGACGGTTTGGACGTCTGGGGCCAA GGGACCACGGTCACCGTCTCCTCA <br><br>SEQ ID NO: 30907 |
| | | AA | DIQMIQSPSSLSASVGDRVTITCRTSQGIGNYLA WFQQKPGKAPKSLLYKTSSLQSGFPSKFSGSGSG TDFTLTISSLQPEDFATYYFQQYMTYPLTFGGGT KVEIK <br><br>SEQ ID NO: 26902 | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDQGV GYDGLDVWGQGTTVTVSS <br><br>SEQ ID NO: 30908 |
| iPS:436177 | 21-225_198B1 | NA | GAAATTGTGTTGACGCAGTCTCCAGGCACCCT GTCTTTGTCTCCAGGGGAAAGCGCCACCCTCT CCTGCAGGGCCGGTCAAAGTATTAGAACCAAC TTCTTAGCCTGGTACCAGCAGCAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAACAGACTGGAGCCTGAAGATTTTGCAG TGTATTACTGTCAGCAGTATGGTAGCTCACCG TGGACGTTCGGCCAAGGGACCAAGGTGGAAA TCAAA <br><br>SEQ ID NO: 26903 | CAGGTGCAACTGAAGGAGTCGGGCCCAGGACTG GTGAAGCCTTCACAGACCCTGTCCCTCACCTGCA CTGTCTCTGGTGGCTCCATCAACAGTGGTGATTA CTACTGGAACTGGTTCCGCCAGCACCCAGGGAA GGGCCTGGAGTGGATTGGGTACATCTTCCACAGT GGGAGCACCTACTACAACCCGTCCCTCAAGAGTC GAGTAACCGTATCAGTAGACACGTCTAAGAACC AGTTCTCCCTGAAGCTGAGTTCTGTGACTGCCGC GGACACGGCCGTGTATTACTGTGCGAGAGGGGA TTACGATGGTTCGGGGGAGTTATCACTACTATTAC GGTATGGACGTCTGGGGCCGAGGGACCACGGTC ACCGTCTCCTCA <br><br>SEQ ID NO: 30909 |

FIGURE 50

| | | | | |
|---|---|---|---|---|
| | | AA | EIVLTQSPGTLSLSPGESATLSCRAGQSIRTNFLA WYQQQPGQAPRLLIYGASSRATGIPDRFSGSGSG TDFTLTINRLEPEDFAVYYCQQYGSSPWTFGQGT KVEIK | QVQLKESGPGLVKPSQTLSLTCTVSGGSINSGDYY WNWFRQHPGKGLEWIGYIFHSGSTYYNPSLKSRVT VSVDTSKNQFSLKLSSVTAADTAVYYCARGDYDG SGSYHYYYGMDVWGRGTTVTVSS |
| | | | SEQ ID NO: 26904 | SEQ ID NO: 30910 |
| iPS:436179 | 21-225_198E1 | NA | GAAATTGTGTTGACGCAGTCTCCAGGCACCCT GTCTTTGTCTCCAGGGGAAAGAGCCACCCTCT CCTGCAGGGCCAGTCAGAGTATAAGGAGCAA CTTCTTAGCCTGGTACCAGCAGAAACCTGGCC AGGCTCCCAGGCTCCTCATCTATGGTGCATTC AGTAGGGCCACTGGCATCCCAGACAGGTTCAG TGGCAGTGGGTCTGGGACAGACTTCACTCTCA CCATCAACAGACTGGAGCCTGAAGATTTTGCA GTGTATTACTGTCAGCAGTATGGTAATTCACC GTGGACGTTCGGCCAAGGGACCAAGGTGGAA ATCAAA | CAGGTGCAGCTGCAGGAGTCGGGCCCAGGACTG GTGAAGCCTTCACAGACCCTGTCCCTCACCTGCA CTGTCTCTGGTGGCTCCATCAACAGTGGTGGTTA CTACTGGAACTGGATCCGCCAGCACCCAGGGAA GGGCCTGGAGTGGATTGGGTTCATCTTTTACAGT GGGAGCACCTACTACAACCCGTCCCTCAAGAGTC GACTTTCCATATCAGTAGACACGTCTAAGAACCA GTTCTCCCTGAAGCTGAGCTCTGTGACTGCCGCG GACACGGCCGTGTATTACTGTGCGAGAGGGGATT ACGATGGTTCGGGGGAGTTATCACTACTATTACGG TATGGACGTCTGGGGGCCAAGGGACCACGGTCAC CGTCTCCTCA |
| | | | SEQ ID NO: 26905 | SEQ ID NO: 30911 |
| | | AA | EIVLTQSPGTLSLSPGERATLSCRASQSIRSNFLA WYQQKPGQAPRLLIYGAFSRATGIPDRFSGSGSG TDFTLTINRLEPEDFAVYYCQQYGNSPWTFGQG TKVEIK | QVQLQESGPGLVKPSQTLSLTCTVSGGSINSGGYY WNWIRQHPGKGLEWIGFIFYSGSTYYNPSLKSRLSI SVDTSKNQFSLKLSSVTAADTAVYYCARGDYDGS GSYHYYYGMDVWGQGTTVTVSS |
| | | | SEQ ID NO: 26906 | SEQ ID NO: 30912 |

FIGURE 50

| iPS:436181 | 21-225_198C2 | NA | GAAATTGTGGTGACGCAGTCTCCAGGCACCCT GTTATTGTATTCCGAGGAGAGATCCACCCTCT CCTGCAGGGCCAGTCAGAGTGTTAGAAGCAGC TACTTAGCCTGGTACCAGCAGAAACCTGGCCA GGCTCCCAGGCTCCTCATCTGTGGTGCATTCA GCAGGGCCAGTGGCATCCCAGACAGGTTCAGT GGCAGTGGGTCTGGGACAGACTTCACTCTCAC CATCAGCAGACTGGAGCCTGAAGATTTCGCAG TGTACTACTGTCAGCAGTATGGTAACTCACCG TGGACGTTGGGCCACGCGACCAAGGTGGAAA TCAAA | CAGGTGCAGCTGCAGGAGTCGGGCCCAGGACTG GTGAAGCCCTCACAGACCCTATCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGAACATCTATTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGGGGATT ACTATGGTTCGGGGAGTTATCACAACTACTACGG TTTGGACGTCTGGGGCCAAGGGACCACGGTCACC GTCTCCTCA |
| | | | SEQ ID NO: 26907 | SEQ ID NO: 30913 |
| | | AA | EIVVTQSPGTLLLYSEERSTLSCRASQSVRSSYLA WYQQKPGQAPRLLICGAFSRASGIPDRFSGSGSG TDFTLTISRLEPEDFAVYYCQQYGNSPWTLGHA TKVEIK | QVQLQESGPGLVKPSQTLSLTCTVSGGSISSGGYYW SWIRQHPGKGLEWIGNIYYSGSTYYNPSLKSRVTIS VDTSKNQFSLKLSSVTAADTAVYYCARGDYYGSG SYHNYYGLDVWGQGTTVTVSS |
| | | | SEQ ID NO: 26908 | SEQ ID NO: 30914 |
| iPS:436189 | 21-225_198B6 | NA | GACATCCAGATGACCCAGTCTCCATCCTCACT GTCTGCATCTGTAGGAGACAGAGTCACCATCA CTTGTCGGGCGAGTCAGGGCATTGGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA ATAGATCTGGGACAGATTTCACTCTCACCATC AGCAGCCTGCAGCCTGAAGATTTTGCAACTTA TTACTGCCAACAATATAGTACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACTACGGTATGGACGTCTGGGGCCAA GGGACCACGGTCACCGTCTCCTCA |
| | | | SEQ ID NO: 26909 | SEQ ID NO: 30915 |

FIGURE 50

| | | | | |
|---|---|---|---|---|
| | | AA | DIQMTQSPSSLSASVGDRVTITCRASQGIGNYLA WFQQKPGKAPKSLIYAASSLQSGVPSKFSGNRSG TDFTLTISSLQPEDFATYYCQQYSTYPLTFGGGT KVEIK | QVQLVESGGGVVQPGRSLRLSCAASGFTFSSYGMH WVRQAPGKGLEWVAVIWYDGSNKHYADSVKGRF TISRDNSKNTLYLQMNSLRAEDTAVYYCARDQGV GYYGMDVWGQGTTVTVSS |
| | | | SEQ ID NO: 26910 | SEQ ID NO: 30916 |
| iPS:436191 | 21-225_198B9 | NA | GACATCCAGATGACCCAGTCTCCATCCTCCCT GTCTGCATCTGTAGGAGACAGAGTCACCATCA CTTGCCGGGCAAGTCAGGGCATAAGAAAAGA TTTAGGCTGGTATCAGCAGAAACCAGGGAAA GCCCCTAAGCGCCTGATCTATGCTGCATCCAG TTTGCAAAGTGGGGTCCCATCAAGGTTCAGCG GCAGTGGATCTGGGACAGAATTCACTCTCACA ATCAGCAGCCTGCAGCCTGAAGATTTTGCAAC TTATGTCTGTCTACAACATTATCGTTACCCTTT CACTTTCGGCCCTGGGACCAAAGTGGATTTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTTTGATGGAAGT AATAAATACTATGTAGACTCCGTGAAGGGCCGAT TCACCATCTCCAGAGACAATTCCAAGAACACGCT GTTTCTGCAAATGAGCAGCCTGAGAGCCGAGGA CACGGCTGTGTATTATTGCGCGAGAGAATGGCTA CAATTCAGGTACTACTACGGTATGGACGTCTGGG GCCAAGGGACCACGGTCACCGTCTCCTCA |
| | | | SEQ ID NO: 26911 | SEQ ID NO: 30917 |
| | | AA | DIQMTQSPSSLSASVGDRVTITCRASQGIRKDLG WYQQKPGKAPKRLIYAASSLQSGVPSRFSGSGS GTEFTLTISSLQPEDFATYVCLQHYRYPFTFGPGT KVDFK | QVQLVESGGGVVQPGRSLRLSCAASGFTFSNYGM HWVRQAPGKGLEWVAIIWFDGSNKYYVDSVKGRF TISRDNSKNTLFLQMSSLRAEDTAVYYCAREWLQF RYYYGMDVWGQGTTVTVSS |
| | | | SEQ ID NO: 26912 | SEQ ID NO: 30918 |

FIGURE 50

| iPS:436193 | 21-225_198A10 | NA | GATATTGTGTTGACCCAGACTCCACTCTCTCTG TCCGTCACCCCTGGACAGCCGGCCTCCATATC GTGCAAGTCTAGTCAGAGCCTCCTCTATAGTG ATGGAAGGACCTATTTGTATTGGTACCTGCAG AAACCAGGCCAGCCTCCACAGGTCCTGATCTG TGAGGTTTCCAACCGGTTCTCTGGAGTGCCAG ATAGGTTCAGTGGCAGCGGGTCAGGGACAGA TTTCACACTGAAAATCAGCCGGGTGGAGGCAG GAGATGTTGGGGTTTATTACTGCATGCAAAGT ATACAGCTTCCCTGGACGTTCGGCCAAGGGAC CAAGGTGGAAATCAAA | CAGCTGCAGCTGCAGGAGTCGGGCCCAGGACTG GTGAAGCCTTCGGAGACCCTGTCCCTCACCTGCA CTGTCTCTGGTGGCTCCATCAGAAGTTACCACTG GAGTTGGATCCGGCAGCCCGCCGGGAAGGGACT GGAGTGGATTGGGCATATCTATACCAGTAGGAGC ACCAACTACAACCCCTCCCTCAAGAGTCGAGTCA CCATTTCAGTAGACACGTCCAAGAACCAGTTCTC CCTGAAGCTGAGCTCTGTGACCGCCACGGACACG GCCGTGTATTACTGTGCGAGACTCCGGTATAACT GGAACTTCCCTTACTTTGACTACTGGGGCCAGGG AACCCTGGTCACCGTCTCCTCA |
| | | | SEQ ID NO: 26913 | SEQ ID NO: 30919 |
| | | AA | DIVLTQTPLSLSVTPGQPASISCKSSQSLLYSDGR TYLYWYLQKPGQPPQVLICEVSNRFSGVPDRFS GSGSGTDFTLKISRVEAGDVGVYYCMQSIQLPW TFGQGTKVEIK | QLQLQESGPGLVKPSETLSLTCTVSGGSIRSYHWSW IRQPAGKGLEWIGHIYTSRSTNYNPSLKSRVTISVDT SKNQFSLKLSSVTATDTAVYYCARLRYNWNFPYFD YWGQGTLVTVSS |
| | | | SEQ ID NO: 26914 | SEQ ID NO: 30920 |
| iPS:436195 | 21-225_198G10 | NA | GAAATTGTGTTGACGCAGTCTCCAGGCACCCT GTCTTTGTCTCCAGGGGAAAGAGCCACCCTCT CCTGCAGGGCCAGTCAGAGTATTCGCAGCAGC TTCTTAGCCTGGTATCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAACTCACCG TGGGCGTTCGGCCAAGGGACCAAGGTGGAAA TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGATCCGCCAGCTCCCAGGGAA GGGCCTGGAGTGGATTGGGTACATCTTTTACAGT GGGAGCACCTACTACAACCCGTCCCTCAAGAGTC GACTTACCATATCAGTGGACACGTCTAAGAACCA GTTCTCCCTGAAGCTGAGTTCTGTGACTGCCGCG GACACGGCCGTGTATTACTGTGCGAGAGGGGATT ACGATGGTTCGGGGAGTTATCACTTCTACTACGG TATGGACGTCTGGGGCCAAGGGACCACGGTCAC CGTCTCCTCA |
| | | | SEQ ID NO: 26915 | SEQ ID NO: 30921 |

FIGURE 50

| | | | | |
|---|---|---|---|---|
| | | AA | EIVLTQSPGTLSLSPGERATLSCRASQSIRSSFLA WYQQKPGQAPRLLIYGASSRATGIPDRFSGSGSG TDFTLTISRLEPEDFAVYYCQQYGNSPWAFGQG TKVEIK | QVQLQESGPGLVKPSQTLSLTCTVSGGSISSGDYYW NWIRQLPGKGLEWIGYIFYSGSTYYNPSLKSRLTISV DTSKNQFSLKLSSVTAADTAVYYCARGDYDGSGS YHFYYGMDVWGQGTTVTVSS |
| | | | SEQ ID NO: 26916 | SEQ ID NO: 30922 |
| iPS:436197 | 21-225_199C2 | NA | GAAATTGTGTTGACGCAGTCTCCAGGCACCCT GTCTTTGTCTCCAGGGGAAAGAGCCACCCTCT CCTGCAGGGCCAGTCAGAGTATTCGCAGCAGC TTCTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAACAGACTGGAGCCTGAAGATTTTGCAG TGTATTACTGTCAGCAGTATGGTAACTCACCG TGGGCGTTCGGCCAAGGGACCAAGGTGGAAA TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGCTCCGCCAGCACCCAGAGAA GGGCCTGGAGTGGATTGGGTACATCTTTTACAGT GGGAGCACCTACTACAACCCGTCCCTCAAGAGTC GAGTTACCATATCAGTGGACACGTCTATGACCCA GTTCTCCCTGAAGCTGACCTCTGTGACTGCCGCG GACACGGCCGTGTATTACTGTGCGAGAGGGGATT ACGATGGTTCGGGGGAGTTATCACTACTACTACGG TATGGACGTCTGGGGGCCAAGGGACCACGGTCAC CGTCTCCTCA |
| | | | SEQ ID NO: 26917 | SEQ ID NO: 30923 |
| | | AA | EIVLTQSPGTLSLSPGERATLSCRASQSIRSSFLA WYQQKPGQAPRLLIYGASSRATGIPDRFSGSGSG TDFTLTINRLEPEDFAVYYCQQYGNSPWAFGQG TKVEIK | QVQLQESGPGLVKPSQTLSLTCTVSGGSISSGDYYW NWLRQHPEKGLEWIGYIFYSGSTYYNPSLKSRVTIS VDTSMTQFSLKLTSVTAADTAVYYCARGDYDGSG SYHYYYGMDVWGQGTTVTVSS |
| | | | SEQ ID NO: 26918 | SEQ ID NO: 30924 |

FIGURE 50

| iPS:436199 | 21-225_199E3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATTTCTTCTGCATCCAGTT TGCAAAGGGGGGTCCCATCAAGATTCAGCGGC AGTGGATCTGGGACAGAATTCACTCTCACAAT CAGCAGCCTGCAGCCTGAAGATTTTGCAACTT ATTACTGTCTACAGCATAAAAGGTACCCGCTC ACTTTCGGCGGAGGGACCAAGGTGGAGATCA AA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTACTTCTGG ACCTGGATCCGCCAGCCCCCAGGGAAGGGGCTG GAGTGGATTGGGGAAATCAATCATAGTGGAAGC ACCAACTACAACCCGTCCCTCAAGAGTCGCGTCA CCATATCAGTAGACACGTCCAAGAACCAGTTCTC CCTGAAGCTGAGCTCTGTGACCGCCGCGGACACG GCTGTGTATTACTGTGCGAGAGACTACGGTGCTG ACTACTGGGGCCAGGGAACCCTGGTCGCCGTCTC CGCA |
|  |  |  | SEQ ID NO: 26919 | SEQ ID NO: 30925 |
|  |  | AA | DIQMTQSPSSLSASVGDRVTITCRASQGIRNDLG WYQQKPGKAPKRLISSASSLQRGVPSRFSGSGSG TEFTLTISSLQPEDFATYYCLQHKRYPLTFGGGT KVEIK | QVQLQQWGAGLLKPSETLSLTCAVYGGSFSGYFW TWIRQPPGKGLEWIGEINHSGSTNYNPSLKSRVTISV DTSKNQFSLKLSSVTAADTAVYYCARDYGADYWG QGTLVAVSA |
|  |  |  | SEQ ID NO: 26920 | SEQ ID NO: 30926 |
| iPS:436201 | 21-225_199C5 | NA | GACATCCAGATGACACAGTCTCCATCCTCACT GTCTGCATCTGTAGGAGACAGAGTCACCATCA CTTGTCGGGCGAGTCAGGGCATTAGCAAGTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAGCTTA TTACTGCCAACAGTATCTTACTTACCCGCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCAGGG CGTGGGCTACTACGGTATGGACGTCTGGGGCCAA GGGACCACGGTCACCGTCTCCTCA |
|  |  |  | SEQ ID NO: 26921 | SEQ ID NO: 30927 |

FIGURE 50

| | | | | |
|---|---|---|---|---|
| | | AA | DIQMTQSPSSLSASVGDRVTITCRASQGISKYLA WFQQKPGKAPKSLIYAASSLQSGVPSKFSGSGSG TDFTLTISSLQPEDFAAYYCQQYLTYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDQGV GYYGMDVWGQGTTVTVSS |
| | | | SEQ ID NO: 26922 | SEQ ID NO: 30928 |
| iPS:436203 | 21-225_199A6 | NA | GACATAGTGATGACGCAGTCTCCAGCCACCCT GTCTGTGTCTCCAGGGGATAGAGCCACCCTCT CCTGCAGGCCCAGTCAGAGTTTTAGCAGAAAC TTAGCCTGGTACCAGCAGAAACCTGGCCAGGC TCCCAGGCTCCTCATCTATGGTGCATCCACTA GGGCCACTGGTATCCCAGCCAGGTTCAGTGGC AGTGGGTCTGGGACAGAGTTCACTCTCACCAT CAGCAGCCTGGAGTGTGAAGATTTTGCAGTTT ACTACTGTCAGCAGTATAATAACTGGCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTTTGATGGAAGT AATCAATACTATGCCGACTCCGTGAAGGGCCGAT TCACCATCTCCAGAGACAATTCCAAGAACACGCT GTATCTGCAATTGAACAGCCTGAGAGCCGAGGA CACGGCTGTCTATTACTGTGCGAGAGCCCACGGG GTCTACTACTACGCTATGGACGTCTGGGGCCAAG GGACCACGGTCACCGTCTCCTCA |
| | | | SEQ ID NO: 26923 | SEQ ID NO: 30929 |
| | | AA | DIVMTQSPATLSVSPGDRATLSCRPSQSFSRNLA WYQQKPGQAPRLLIYGASTRATGIPARFSGSGSG TEFTLTISSLECEDFAVYYCQQYNNWPLTFGGGT KVEIK | QVQLVESGGGVVQPGRSLRLSCAASGFTFSNYGM HWVRQAPGKGLEWVAIIWFDGSNQYYADSVKGRF TISRDNSKNTLYLQLNSLRAEDTAVYYCARAHGVY YYAMDVWGQGTTVTVSS |
| | | | SEQ ID NO: 26924 | SEQ ID NO: 30930 |

FIGURE 50

| iPS:436205 | 21-225_199A7 | NA | GACATCCAGATGACCCAGTCTCCATCCTCCCT GTCTGCATCTGTAGGAGACAGAGTCACCATCA CTTGCCGGGCAAGTCAGGGCATAAGAAAAGA TTTAGGCTGGTATCAGCAGAAACCAGGGAAA GCCCCTAAGCGCCTGATCTATGCTGCATCCAG TTTGCAAAGTGGGGTCCCATCAAGGTTCAGCG GCAGTGGATCTGGGACAGAATTCACTCTCACA ATCAGCAGCCTGCAGCCTGAAGATTTTGCAAC TTATGTCTGTCTACAACATTATCGTTACCCTTT CACTTTCGGCCCTGGGACCAAAGTGGATTTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTTTGATGGAAGT AATAAATACTATGCAGACTCCGTGAAGGGCCGA TTCACCATCTCCAGAGACAATTCCAAGAACACGC TGTTTCTGCAAATGAGCAGCCTGAGAGCCGAGG ACACGGCTGTGTATTACTGTGCGAGAGAATGGCT ACAATTCAGGTACTACTACGGTATGGACGTCTGG GGCCAAGGGACCACGGTCACCGTCTCCTCA |
|---|---|---|---|---|
| | | | SEQ ID NO: 26925 | SEQ ID NO: 30931 |
| | | AA | DIQMTQSPSSLSASVGDRVTITCRASQGIRKDLG WYQQKPGKAPKRLIYAASSLQSGVPSRFSGSGS GTEFTLTISSLQPEDFATYVCLQHYRYPFTFGPGT KVDFK | QVQLVESGGGVVQPGRSLRLSCAASGFTFSNYGM HWVRQAPGKGLEWVAIIWFDGSNKYYADSVKGRF TISRDNSKNTLFLQMSSLRAEDTAVYYCAREWLQF RYYYGMDVWGQGTTVTVSS |
| | | | SEQ ID NO: 26926 | SEQ ID NO: 30932 |
| iPS:436207 | 21-225_199C7 | NA | GAAATTGTGTTGACGCAGTCTCCAGGCACCCT GTCTTTGTCTCCAGGGGAAAGAGCCACCCTCT CCTGCAGGGCCAGTCAGAGTATAAGGACCAA CTTCTTAGCCTGGTACCAGCAGAAACCTGGCC AGGCTCCCAGGCTCCTCATCTATGGTGCATCC AGCAGGGCCACTGGCATCCCAGACAGGTTCAG TGGCAGTGGGTCTGGGACAGACTTCACTCTCA CCATCAACAGACTGGAGCCTGAAGATTTTGCA GTGTATTACTGTCAGCAGTATGGTAACTCACC GTGGACGTTCGGCCAAGGGACCAAGGTGGAA ATCAAA | CAGGTGCAGCTGCAGGAGTCGGGCCCAGGACTG GTGAAGCCTTCACAGACCCTGTCCCTCACCTGCA CTGTCTCTGGTGGCTCCATCAACAGTGGTGGTTA CTACTGGAACTGGATCCGCCAGCACCCAGGGAA GGGCCTGGAGTGGATTGGGTTCATCTTTTACAGT GGGAGCACCTACTACAACCCGTCCCTCAAGAGTC GAGTTTCCATATCAGTAGACACGTCTAAGAACCA GTTCTCCCTGAAGCTGAGCTCTGTGACTGCCGCG GACACGGCCGTGTATTACTGTGCGAGAGGGGATT ACGATGGTTCGGGGGAGTTATCACTACTATTACGG TATGGACGTCTGGGGGCCAGGGGACCACGGTCAC CGTCTCCTCA |
| | | | SEQ ID NO: 26927 | SEQ ID NO: 30933 |

FIGURE 50

| | | | | |
|---|---|---|---|---|
| | | AA | EIVLTQSPGTLSLSPGERATLSCRASQSIRTNFLA WYQQKPGQAPRLLIYGASSRATGIPDRFSGSGSG TDFTLTINRLEPEDFAVYYCQQYGNSPWTFGQG TKVEIK | QVQLQESGPGLVKPSQTLSLTCTVSGGSINSGGYY WNWIRQHPGKGLEWIGFIFYSGSTYYNPSLKSRVSI SVDTSKNQFSLKLSSVTAADTAVYYCARGDYDGS GSYHYYYGMDVWGQGTTVTVSS |
| | | | SEQ ID NO: 26928 | SEQ ID NO: 30934 |
| iPS:436210 | 21-225_199G11 | NA | GAAATTGTGGTGACGCAGTCTCCAGGCACCCT GTCTTTGTCTCCAGGGGAAAGAGCCACCCTCT CCTGCAGGGCCAGTCAGAGTGTTAGAAGCAGC TACTTAGCCTGGTACCAGCAGAAACCTGGCCA GGCTCCCAGGCTCCTCATCTATGGTGCATTCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTACTACTGTCAGCAGTATGGTAACTCACCG TGGACGTTCGGCCACGGGACCAAGGTGGAAA TCAAA | CAGGTGCAGCTGCAGGAGTCGGGCCCAGGACTG GTGAAGCCCTCACAGACCCTATCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGAACATCTATTACAGT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGGGGATT ACTATGGTTCGGGGGAGTTATCACAACTACTACGG TTTGGACGTCTGGGGCCAAGGGACCACGGTCACC GTCTCCTCA |
| | | | SEQ ID NO: 26929 | SEQ ID NO: 30935 |
| | | AA | EIVVTQSPGTLSLSPGERATLSCRASQSVRSSYLA WYQQKPGQAPRLLIYGAFSRATGIPDRFSGSGSG TDFTLTISRLEPEDFAVYYCQQYGNSPWTFGHGT KVEIK | QVQLQESGPGLVKPSQTLSLTCTVSGGSISSGGYYW SWIRQHPGKGLEWIGNIYYSGSTYYNPSLKSRVTIS VDTSKNQFSLKLSSVTAADTAVYYCARGDYYGSG SYHNYYGLDVWGQGTTVTVSS |
| | | | SEQ ID NO: 26930 | SEQ ID NO: 30936 |

FIGURE 50

| iPS:436212 | 21-225_200G1 | NA | GACATCCAGATGACCCAGTCTCCATCCTCCCT GTCTGCATCTGTAGGAGACAGAGTCACCATCA CTTGCCGGGCAAGTCAGAGCATTAGCAGCTAT TTAAATTGGTTTCAGCAGAAACCAGGGAAAGC CCCTAAGCTCCTGATCTATGCTGAGTCCAGTTT ACAAAGTGGGGTCCCATCAAGGTTCAGTGGCA GTGGATCTGGGACAGATTTCACTCTCACCATC AGCAGTCTGCAACCTGAAGATTTTGCAACTTA TTCCTGTCAACAGAGTTACAGTTCCCCTCCGTG GACGTTCGGCCAAGGGACCAAGGTGGAGTTC AAA | GAGGTGCAACTGTTGGAGTCTGGGGGAGGCTTG GTGCAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGAGGCGGT AATACATTCTACGCAGACTCCGTGAGGGGCCGGT TCACCATCTCCAGAGACAATTCCAAGAACACGCT GTATCTGCAAATGAACAGCCTGAGAGCCGAGGA CACGGCCGTATATTACTGTGCGCGACGTATAGCA GTGGATGGCTATGATGCTTTTGATGTCTGGGGCC AAGGGACAATGGTCACCGTCTCTTCA |
|---|---|---|---|---|
| | | | SEQ ID NO: 26931 | SEQ ID NO: 30937 |
| | | AA | DIQMTQSPSSLSASVGDRVTITCRASQSISSYLNW FQQKPGKAPKLLIYAESSLQSGVPSRFSGSGSGT DFTLTISSLQPEDFATYSCQQSYSSPPWTFGQGT KVEFK | EVQLLESGGGLVQPGGSLRLSCAASGFTFSSYAMS WVRQAPGKGLEWVSAISGRGGNTFYADSVRGRFTI SRDNSKNTLYLQMNSLRAEDTAVYYCARRIAVDG YDAFDVWGQGTMVTVSS |
| | | | SEQ ID NO: 26932 | SEQ ID NO: 30938 |
| iPS:436214 | 21-225_200F6 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATCACTGTCTACAGCATTATCGTTACCCATTC ACTTTCGGCCCTGGGACCAAAGTGGATATCAA A | CAGGTGCAGCTGGTGGAGTCTGGGGGAGGCGTG GTCCAGCCTGGAAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAATGGCT ACAATTTAGGTATTACTACGGTATGGACGTCTGG GGCCAAGGGACCACGGTCACCGTCTCCTCA |
| | | | SEQ ID NO: 26933 | SEQ ID NO: 30939 |

FIGURE 50

| | | | | |
|---|---|---|---|---|
| | | AA | DIQMTQSPSSLSASVGDRVTITCRASQDIRNDLG WYQQKPGKAPKRLIYAASSLQSGVPSRFSGSGS GTEFTLTISSLQPEDFATYHCLQHYRYPFTFGPGT KVDIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCTREWLQF RYYYGMDVWGQGTTVTVSS |
| | | | SEQ ID NO: 26934 | SEQ ID NO: 30940 |
| iPS:436216 | 21-225_200B7 | NA | GAAATTGTGCTGACTCAGTCTCCAGACTTTCA GTCTGTGACTCCAAAGGAGAAAGTCACCATCA CCTGCCGGGCCAGTCAGAACATTGGTAATACC TTGCACTGGTACCAGCAGAAACCAGATCAGTC TCCTAAGCTCCTCATCAAGTATGCTTCCCAGTC CTTCTCAGGGGTCCCCTCGAGGTTCAGTGGCA GTGGGTCTGGGACAGATTTCATCCTCACCATC AATAGCCTGGAAGCTGAAGATGCTGCAACGTA TTACTGTCATCAGAGTGGTAGTTTACCTCAGA CGTTCGGCCAAGGGACCAAGGTGGAAATCAA A | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGTACATCTTTTACAGT GGGAGCACCAACTACAACCCGTCCCTCAGGAGT CGAGTTACCATATCAGTAGACACGTCTAAGAACC AGTTCTCCCTGAAACTGAGTTCTGTGACTGCCGC GGACACGGCCGTGTATTACTGTGCGAGAGCCGG GTATAACTGGAACAACGGTATGGACGTCTGGGG CCAAGGGACCACGGTCACCGTCTCCTCA |
| | | | SEQ ID NO: 26935 | SEQ ID NO: 30941 |
| | | AA | EIVLTQSPDFQSVTPKEKVTITCRASQNIGNTLH WYQQKPDQSPKLLIKYASQSFSGVPSRFSGSGSG TDFILTINSLEAEDAATYYCHQSGSLPQTFGQGT KVEIK | QVQLQESGPGLVKPSQTLSLTCTVSGGSISSGGYYW SWIRQHPGKGLEWIGYIFYSGSTNYNPSLRSRVTIS VDTSKNQFSLKLSSVTAADTAVYYCARAGYNWNN GMDVWGQGTTVTVSS |
| | | | SEQ ID NO: 26936 | SEQ ID NO: 30942 |

FIGURE 50

| iPS:436218 | 21-225_200G7 | NA | GACATCGTGATGACCCAGTCTCCAGACTCCCC GACTGCGTCTCTGGGCGAGAGGGCCACCGTCA AATGCAAGTCCAGCCAGAGTGTTTTACACAGC TCCAACAATAACAACTACTTAGCTTGGTACCA GCAGAAACCAGGACAGCCTCCTAAGCTGCTCA TTTACTGGGCATCTACCCGGGAATCCGGAGTC CCTGACCGATTCAGTGGCAGCGGGTCTGGGAC AGATTTCACTCTCGCCATCAGCAGCCTGCAGG CTGAAGATGTGGCGGTTTATTACTGTCAGCAA TATTATAATACTCCTCCGACATTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGATGCGGCAGGCCACTGGTCAAGGGC TTGAGTGGATGGGATGGATGCACCTTAACAGTGG TAACACAGGCTATGCACCGAAGTTCCAGGGCAG AGTCACCATGACCAGGGACACCTCCATAAGCAC AGCCTTCATGGAGCTGAGCAGCCTGAGATCTGAG GACACGGCCGTGTATTACTGTGCCTATAGCGGTG GCTGGTACGTGTTCGACCCCTGGGGCCAGGGAAC CCTGGTCACCGTCTCCTCA |
|---|---|---|---|---|
| | | | SEQ ID NO: 26937 | SEQ ID NO: 30943 |
| | | AA | DIVMTQSPDSPTASLGERATVKCKSSQSVLHSSN NNNYLAWYQQKPGQPPKLLIYWASTRESGVPD RFSGSGSGTDFTLAISSLQAEDVAVYYCQQYYN TPPTFGQGTKVEIK | QVQLVQSGAEVKKPGASVKVSCKASGYTFTNYDIN WMRQATGQGLEWMGWMHLNSGNTGYAPKFQGR VTMTRDTSISTAFMELSSLRSEDTAVYYCAYSGGW YVFDPWGQGTLVTVSS |
| | | | SEQ ID NO: 26938 | SEQ ID NO: 30944 |
| iPS:436220 | 21-225_200F8 | NA | GAAATTGTGCTGACTCAGTCTCCAGACTTTCA GTCTGTGGCTCCAAAGGAGAAAGTCACCATCA CCTGCCGGGCCAGTCAGAGTATTGGTAGTAAC TTACACTGGTACCAGCAGAAACCAGATCAGTC TCCGAAACTCCTCATCAAGTCTGCTTCCCAGTC CTTCTCAGGGGTCCCCTCGAGGTTCAGTGGCA GTGGATCTGGGACAGATTTCACCCTCACCATC AATAGCCTGGAAACTGAAGATGCTGCAACGTA TTACTGTCAGCAGAGTAGTAGTTTACCGTGGA CGTTCGGCCAAGGGACCAAGGTGGAAATCAA A | CAGGTGCAGCTGCAGGAGTCGGGCCCAGGACTG GTGAAGCCTTCGGAGACCCTGTCCCTCACCTGCA CTGTCTCTGGTGGCTCCATCAATAATTACTACTG GAGCTGGATCCGGCAGCCCGCCGGGAAGGGACT GGAGTGGATTGGGCGTATCTATACCAGTGGGAGC ACCAACTACAACCCCTCCCTCAAGAGTCGAGTCA CCATGTCAGTAGACACGTCCAAGAACCAGTTCTC CCTGAAGCTGAGCTCTGTGACCGCCGCGGACACG GCCGTGTATTACTGTGCGAGAGATCGGGGATACT ATGGCTACTACGGTATGGACGTCTGGGGCCAAG GGACCACGGTCACCGTCTCCTCA |
| | | | SEQ ID NO: 26939 | SEQ ID NO: 30945 |

EP 4 435 105 A2

FIGURE 50

| | | | | |
|---|---|---|---|---|
| | | AA | EIVLTQSPDFQSVAPKEKVTITCRASQSIGSNLHW YQQKPDQSPKLLIKSASQSFSGVPSRFSGSGSGT DFTLTINSLETEDAATYYCQQSSSLPWTFGQGTK VEIK | QVQLQESGPGLVKPSETLSLTCTVSGGSINNYYWS WIRQPAGKGLEWIGRIYTSGSTNYNPSLKSRVTMS VDTSKNQFSLKLSSVTAADTAVYYCARDRGYYGY YGMDVWGQGTTVTVSS |
| | | | SEQ ID NO: 26940 | SEQ ID NO: 30946 |
| iPS:436222 | 21-225_200C9 | NA | GACATCCAGATGACCCAGTCTCCATCCTCCCT GTCTGCATCTGTTGGAGACAGAGTCACCATCA CTTGCCGGGCAACTCAGGGCATTAGAAAAGAT TTAGGCTGGTATCAGCAGAAACCAGGGAAAG CCCCTAAGCGCCTGATCCATACTGCATCCAGT TTGCAAAGTGGGGTCCCATTAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGTG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGATGGAGGT TATAAAAACTATATAGACTCCGTGAAGGGCCGAT TCACCATCTCCAGAGACAATTCCAAGAACACGCT GTATCTGCAAATGAACAGCCTGAGAGCTGAGGA CACGGCTGTGTTTTACTGTGCGAGAGGTACCCAC GGGTACTACTACGGTGTGGACGTCTGGGGGCCAA GGGACCACGGTCACCGTCTCCTCA |
| | | | SEQ ID NO: 26941 | SEQ ID NO: 30947 |
| | | AA | DIQMTQSPSSLSASVGDRVTITCRATQGIRKDLG WYQQKPGKAPKRLIHTASSLQSGVPLRFSGSGS GTEFTLTISSLQPEDFATYYCLQHNSYPWTFGQG TKVEIK | QVQLVESGGGVVQPGRSLRLSCAASGFTFSSYGMH WVRQAPGKGLEWVAVISYDGGYKNYIDSVKGRFT ISRDNSKNTLYLQMNSLRAEDTAVFYCARGTHGYY YGVDVWGQGTTVTVSS |
| | | | SEQ ID NO: 26942 | SEQ ID NO: 30948 |

2341

FIGURE 50

| iPS:436226 | 21-225_200F10 | NA | GAAATTGTGTTGACGCAGTCTCCAGGCACCCT GTCTTTGTCTCCAGGGGAAAGAGCCACCCTCT CCTGCAGGGCCAGTCAGAATATTCGCAGCAGC TTCTTAGCCTGGTACCAACAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAACAGACTGGAGCCTGAAGATTTTTGCAG TGTATTACTGTCAGCAGTATGGTAACTCACCG TGGGCGTTCGGCCAAGGGACCAAGGTGGAAA TCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGATTA CTACTGGAACTGGCTCCGCCAGCACCCAGAGAA GGGCCTGGAGTGGATTGGGTACATCTTTTACAGT GGGAGCACCTACTACAACCCGTCCCTCAAGAGTC GAATTACCATATCAGTGGACACGTCTATGACCCA GTTCTCCCTGAAGCTGACCTCTGTGACTGCCGCG GACACGGCCGTGTATTACTGTGCGAGAGGGGATT ACGATGGTTCGGGGAGTTATCACTACTACTACGG TATGGACGTCTGGGGCCAAGGGACCACGGTCAC CGTCTCCTCA |
| | | | SEQ ID NO: 26943 | SEQ ID NO: 30949 |
| | | AA | EIVLTQSPGTLSLSPGERATLSCRASQNIRSSFLA WYQQKPGQAPRLLIYGASSRATGIPDRFSGSGSG TDFTLTINRLEPEDFAVYYCQQYGNSPWAFGQG TKVEIK | QVQLQESGPGLVKPSQTLSLTCTVSGGSISSGDYYW NWLRQHPEKGLEWIGYIFYSGSTYYNPSLKSRITISV DTSMTQFSLKLTSVTAADTAVYYCARGDYDGSGS YHYYYGMDVWGQGTTVTSS |
| | | | SEQ ID NO: 26944 | SEQ ID NO: 30950 |
| iPS:436228 | 21-225_200F12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAGAG CCCCTAAGCGCCTGATCTATTCTGCATCCAGTT TGCATACTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAAGAGTTACCCGCTC ACTTTCGGCGGAGGGACCAAGGTGGAGATCA AA | CAGGTGCAGCTACAGCAGTGGGGCGCAGGACTG TTGAAGCCTTCGGGAGACCCTGTCCCTCACCTGCG CTGTCTATGGTGGGTCCTTCAGTGGTTACTTCTGG ACCTGGATACGCCAGCCCCCAGGGAAGGGACTG GAGTGGATTGGGGAAATCAGTCATAGTGGAAGC ACCAACTACAACCCGTCCCTCAAGAGTCGAGTCA CCATATCAGTAGACACGTCCAAGAACCAGTTCTC CCTGAAGGTGAGCTCTGTGACCGCCGCGGACAC GGCTGTGTATTACTGTGCGAGGGACTACGGGGCG GACTACTGGGGCCAGGGAACCCTGGTCACCGTCT CCTCA |
| | | | SEQ ID NO: 26945 | SEQ ID NO: 30951 |

FIGURE 50

| | | | | |
|---|---|---|---|---|
| | | AA | DIQMTQSPSSLSASVGDRVTITCRASQGIRNDLG WYQQKPGRAPKRLIYSASSLHTGVPSRFSGSGSG TEFTLTISSLQPEDFATYYCLQHKSYPLTFGGGT KVEIK | QVQLQQWGAGLLKPSETLSLTCAVYGGSFSGYFW TWIRQPPGKGLEWIGEISHSGSTNYNPSLKSRVTISV DTSKNQFSLKVSSVTAADTAVYYCARDYGADYW GQGTLVTVSS |
| | | | SEQ ID NO: 26946 | SEQ ID NO: 30952 |
| IPS:436230 | 21-225_201A1 | NA | GACATCCAGATGACCCAGTCTCCATCCTCCCT GTCTGCATCTGTAGGAGACAGAGTCACCATCA CTTGCCGGGCAAGTCAGGGCATTAGGAATGAT TTAGGCTGGTATCAGCAGAAACCAGGGAAAG CCCCTAAGCGCCTGATCTATTCTGCATCCATTT TACAAAGGGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAAAAGTTACCCTCTC ACTTTCGGCGGAGGGACCAAGGTGGAGGTCA AA | CAGGTGCAGCTACAGCAGTGGGGCGCAGGACTG TTGAAACCTTCGGAGACCCTGTCCCTCACCTGCG CTGTCACTGGTGGGTCCTTCAGTGGTTACTTCTGG ACCTGGATCCGCCAGCCCCCAGGGAAGGGGCTG GAGTGGATTGGGGAAATCAGTCATAGTGGACGC ACCAACTACAACCCGTCCCTCAAGAGTCGAGTCA CCATATCAGGAGACACGTCCAAGAACCAGTTCTC CCTGAAGCTGAGCTCTGTGACCGCCGCGGACACG GCTGTGTATTACTGTGCGAGGGACTACGGGGCGG ACTACTGGGGCCAGGGAACCCTGGTCACCGTCTC CTCA |
| | | | SEQ ID NO: 26947 | SEQ ID NO: 30953 |
| | | AA | DIQMTQSPSSLSASVGDRVTITCRASQGIRNDLG WYQQKPGKAPKRLIYSASILQRGVPSRFSGSGSG TEFTLTISSLQPEDFATYYCLQHKSYPLTFGGGT KVEVK | QVQLQQWGAGLLKPSETLSLTCAVTGGSFSGYFWT WIRQPPGKGLEWIGEISHSGRTNYNPSLKSRVTISG DTSKNQFSLKLSSVTAADTAVYYCARDYGADYWG QGTLVTVSS |
| | | | SEQ ID NO: 26948 | SEQ ID NO: 30954 |

FIGURE 50

| iPS:436232 | 21-225_201E1 | NA | GAAATTGTGTTGACGCAGTCTCCAGACACCCT GTCTTTGTCTCCAGGGGAAAGAGCCACCCTCT CCTGCAGGGCCAGTCCGAGTATTAACAGCGGC TTCTTAGCCTGGTACCAGCAGAAACCTGGCCA AGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAA TGTTTCACTGTCACCAGTATGAGACCTCACCG TGGACGTTCGGCCAAGGGACCAAGGTGGAGA TCAAA | CAGGTGCAGCTACAGCAGTGGGGCGCAGGACTG TTGAAGCCTTCGGAGACCCTGTCCCTCACCTGCG CTGTCTTTGATGGGTCCTTCAGTCCTTACTACTGG AGCTGGATCCGCCAGCCCCCAGGGAAGGGGCTG GAGTGGATTGGGGAAGTCAATCATAGTGGAAGC ACCAACTACAACCCGTCCCTCAAGAGTCGAGTCA CCATATCAGTAGACACGTCCAAGAACCAGTTCTC CCTGAAGCTGAGCTCTGTGACCGCCGCGGACACG GCTGTGTATTACTGTGCGAGAGACTACGGGGGTT TAGACTACTGGGGCCAGGGGAGCCCTGGTCACCGT CTCCTCA |
| | | | SEQ ID NO: 26949 | SEQ ID NO: 30955 |
| | | AA | EIVLTQSPDTLSLSPGERATLSCRASPSINSGFLA WYQQKPGQAPRLLIYGASSRATGIPDRFSGSGSG TDFTLTISRLEPEDFAMFHCHQYETSPWTFGQGT KVEIK | QVQLQQWGAGLLKPSETLSLTCAVFDGSFSPYYWS WIRQPPGKGLEWIGEVNHSGSTNYNPSLKSRVTISV DTSKNQFSLKLSSVTAADTAVYYCARDYGGLDYW GQGALVTVSS |
| | | | SEQ ID NO: 26950 | SEQ ID NO: 30956 |
| iPS:436234 | 21-225_51E3 | NA | CAGTCTGTGCTGACTCAGCCACCCTCAGCGTC TGGGACCCCCGGGCAGAGGGTCACCATCTCTT GTTCTGGAAGCAACTCCAACATCGGAAGTAAT ATTGTAACCTGGTACCAGCAGCTCCCAGGAAC GGCCCCCAAACTCCTCATCTATAGTAATGATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTACAGCATGGGATGACAGCCTG AATGGTTGGGTGTTCGGCGGAGGTACCACGCT GACCGTCCTA | CAGGTTCAGCTGGTGCAGTCTGGAGCTGAGGTGA AGAAGCCTGGGGCCTCAGTGAAGGTCTCCTGCA AGGCTTCAGGTTACACCTTTAACAGCTATGGTAT CAGCTGGGTGCGACTGGCCCCTGGACAAGGGCTT GAGTGGATGGGATGGATCAGCGCTTATAATGGTA ACACAAAGAATGCACAGAGGTTCCAGGGCAGAG TCACCATGACCACAGACACATCCACGAGCACGG CCTACATGGAGCTGAGGAGCCTGAGATCTGACG ACACGGCCGTTTATTACTGTGCGAGACACGATTT TTGGAGTGGTTATTATAAGGGTATGGACGTCTGG GGCCAAGGGACCACGGTCACCGTCTCCTCA |
| | | | SEQ ID NO: 26951 | SEQ ID NO: 30957 |

FIGURE 50

| | | | | |
|---|---|---|---|---|
| | | AA | QSVLTQPPSASGTPGQRVTISCSGSNSNIGSNIVT WYQQLPGTAPKLLIYSNDQRPSGVPDRFSGSKS GTSASLAISGLQSEDEADYYCTAWDDSLNGWVF GGGTTLTVL | QVQLVQSGAEVKKPGASVKVSCKASGYTFNSYGIS WVRLAPGQGLEWMGWISAYNGNTKNAQRFQGRV TMTTDTSTSTAYMELRSLRSDDTAVYYCARHDFW SGYYKGMDVWGQGTTVTVSS |
| | | | SEQ ID NO: 26952 | SEQ ID NO: 30958 |
| iPS:436236 | 21-225_201F7 | NA | GAAATAGTGATGACGCAGTCTCCAGCCACCCT GTCTGTGTCTCCAGGGGAAAGAGCCACCCTCT CCTGCAGGGCCAGTCAGAATATTAAAAACAAC TTAGCCTGGTACCAGCAGAAACCTGGCCAGGC TCCCAGGCTCCTCATCTTTGGTGCATCCACCAG GGCCACTGGTATCCCAGCCAGGTTCAGTGGCA GTGGGTCTGGGACAGAGTTCACTCTCACCATC AGCAGCCTGCAGTCTGAAGATTTTGCAGTTTA TTACTGTCAGCAGTTTTATAACTGGCTGTGCA GTTTTGGCCAGGGGACCAAGCTGGAGCTCAAA | CAGGTACAGCTGCAGCAGTCAGGTCCAGGACTG GTGAAGCCCTCGCAGACCCTCTCACTCACCTGTG CCATCTCCGGGGACAGTGTCTCTAGCAACAGTGC TGCTTGGAACTGGATCAGGCAGTCCCCATCGAGA GGCCTTGAGTGGCTGGGAAGGACATACTACAGG TCCAAGTGGTATAATTATTATGAAGTATCTGTGA GAAGTCGAATAACCATCAACCCAGACACATCCA AGAACCAGTTCTCCCTGCAGCTGAACTCTGTGAC TCCCGAGGACACGGCTGTATATTTCTGTGCGAGA GATCAACGGTACTACGGTATGGACGTCTGGGGCC AAGGGACCACGGTCACCGTCTCCTCA |
| | | | SEQ ID NO: 26953 | SEQ ID NO: 30959 |
| | | AA | EIVMTQSPATLSVSPGERATLSCRASQNIKNNLA WYQQKPGQAPRLLIFGASTRATGIPARFSGSGSG TEFTLTISSLQSEDFAVYYCQQFYNWLCSFGQGT KLELK | QVQLQQSGPGLVKPSQTLSLTCAISGDSVSSNSAA WNWIRQSPSRGLEWLGRTYYRSKWYNYYEVSVRS RITINPDTSKNQFSLQLNSVTPEDTAVYFCARDQRY YGMDVWGQGTTVTVSS |
| | | | SEQ ID NO: 26954 | SEQ ID NO: 30960 |

FIGURE 50

| iPS:436238 | 21-225_201B2 | NA | GAAATTGTTTTGACGCAGTCTCCAGGCACCCT GTCTTTGTCTCCAGGGGAAAGAGCCACCCTCT CCTGCAGGGCCAGTCAGAGTGTTAGCAGCAAC TACTTAGCCTGGTATCAGCAGAGACCTGGCCA GGCTCCCAGACTCCTCATCTATGGTGCATCCA GCAGGGCCACTGGCATCCCAGACAGATTCAGT GGCAGTGGGTCTGGGACAGACTTCACTCTCAC CATCAGCAGACTGGAGCCTGAAGATTTTGCAG TGTATTTCTGTCAGCAGTATGAAAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTTTGGTGGGTCCATCAGTGTTTACTACTGG ACCTGGATCCGCCAGCCCCCAGGGAAGGGGCTG GAGTGGATTGGGGAAATCAATCATAGTGGAAGC ACCAACTACAACCCGTCCCTCAAGAGTCGAGTCA CCATATCAGTAGACACGTCCAAGAACCAGTTCTC CCTGAAGCTGAGCTCTGTGACCGCCGCGGACACG GCTGTGTATTACTGTGCGAGGGACTATGGTGTCT TTGATTACTGGGGCCAGGGAACCCTGGTCACCGT CTCCTCA |
| | | | SEQ ID NO: 26955 | SEQ ID NO: 30961 |
| | | AA | EIVLTQSPGTLSLSPGERATLSCRASQSVSSNYLA WYQQRPGQAPRLLIYGASSRATGIPDRFSGSGSG TDFTLTISRLEPEDFAVYFCQQYENSPWTFGQGT KVEIK | QVQLQQWGAGLLKPSETLSLTCAVFGGSISVYYWT WIRQPPGKGLEWIGEINHSGSTNYNPSLKSRVTISV DTSKNQFSLKLSSVTAADTAVYYCARDYGVFDYW GQGTLVTVSS |
| | | | SEQ ID NO: 26956 | SEQ ID NO: 30962 |
| iPS:436240 | 21-225_201E8 | NA | GAAATTGTGCTGACTCAGTCTCCAGCCTTTCA GTCTGTGACTCCAAAGGAGAAAGTCACCATCA CCTGCCGGGCCAGTCAGAACATTGGTCGTAGT TTACACTGGTACCAGCAGAAACCAGATCAGTC TCCAAAACTCCTCATCAAGTATGCTTCCCAGT CCTTCTCAGGGGTCCCCTCGAGGTTCAGTGGC AGTGGATCTGGGACAAATTTCACCCTCACCAT CAATAGCCTGGAAGCTGAAGATGCTGTAACGT ATTACTGTCATCAGAGTCGAAGTTTACCGCTC ACTTTCGGCGGAGGGACCAAGGTAGAGATCA GA | CAGGTGCAGCTGGTACAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCGACCCTAACAGTGG TGGCACAAACTATCCACAGAAGTTTCAGGGCAG GGTCACCATGACCAGGGACACGTCCATCAGCAC AGCCTACATGGAACTGAGCAGGCTGAGATCTGA CGACACGGCCGTGTGTTTTACTGTGCGAAAGATCAA GGGTATAACTGGAACTCTTTTGACTACTGGGGCC AGGGAACCCTGGTCACCGTCTCCTCA |
| | | | SEQ ID NO: 26957 | SEQ ID NO: 30963 |

FIGURE 50

| | | | | |
|---|---|---|---|---|
| | | AA | EIVLTQSPAFQSVTPKEKVTITCRASQNIGRSLHW YQQKPDQSPKLLIKYASQSFSGVPSRFSGSGSGT NFTLTINSLEAEDAVTYYCHQSRSLPLTFGGGTK VEIR | QVQLVQSGAEVKKPGASVKVSCKASGYTFTGYYM HWVRQAPGQGLEWMGWIDPNSGGTNYPQKFQGR VTMTRDTSISTAYMELSRLRSDDTAVFYCAKDQGY NWNSFDYWGQGTLVTVSS |
| | | | SEQ ID NO: 26958 | SEQ ID NO: 30964 |
| IPS:436242 | 21-225_201A10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAGAG CCCCTAAGCGCCTGATCTATTCTACATCCAGTT TGCATTCTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGCTC ACTTTCGGCGGAGGGACCAAGGTGGAGATCA AA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTACTTCTGG ACCTGGATACGCCAGCCCCCAGGGAAGGGACTG GAGTGGATTGGGGAAATCAGTCATAGTGGAAGC ACCAACTACAACCCGTCCCTCAAGAGTCGAGTCA CCATATCAGTAGACACGTCCAAGAACCAGTTCTC CCTGAAGGTGAACTCTGTGACCGCCGCGGACAC GGCTGTGTATTACTGTGCGAGGGACTACGGGGCG GACTACTGGGGCCAGGGAACCCTGGTCACCGTCT CCTCA |
| | | | SEQ ID NO: 26959 | SEQ ID NO: 30965 |
| | | AA | DIQMTQSPSSLSASVGDRVTITCRASQGIRNDLG WYQQKPGRAPKRLIYSTSSLHSGVPSRFSGSGSG TEFTLTISSLQPEDFATYYCLQHNSYPLTFGGGT KVEIK | QVQLQQWGAGLLKPSETLSLTCAVYGGSFSGYFW TWIRQPPGKGLEWIGEISHSGSTNYNPSLKSRVTISV DTSKNQFSLKVNSVTAADTAVYYCARDYGADYW GQGTLVTVSS |
| | | | SEQ ID NO: 26960 | SEQ ID NO: 30966 |

FIGURE 50

| | | | | |
|---|---|---|---|---|
| iPS:436244 | 21-225_201H10 | NA | GACATCCAGATGACCCAGTCTCCATCCTCCCT GTCTGCATCTGTTGGAGACAGAGTCACCATCA CTTGCCGGGCAAGTCACAACATTAACAGCTAT TTAAATTGGTATCAGCAGAAATCAGGGAAAGC CCCTAAACTCCTAATCTATGCTGCATCCAGTTT GCAAAGTGGGGTCCCGTCAAGGTTCAGTGGCA GTGGATCTGGGACAGATTTCACTCTCACCTTC AGTAGTCTACAACCTGAAGATTTTGCAACTTA CTACTGTCAACAGAGTTACAGTTTCCCGCTCA CTTTCGGCGGAGGGACCAAGGTGGAGATGAG G | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TCCACTGGGTGCGACAGGCCCCTGGACAAGGGC TTGAGTGGATGGCATGGATCAACCCTAATAGTGG TGGCACAAACTATGCACAGAAGTTTCAGGGCAG GGTCACCATGACCAGGGACACGTCCATCACCAC AACCTACATGGAGCTGAGCAGGCTGAGATCTGA CGACACGGCCGTGTATTACTGTGCGAGAGGATAC AGCTATGGTTACAACTGGTTCGACCCCTGGGGCC AGGGAACCCTGGTCACCGTCTCCTCA |
| | | | SEQ ID NO: 26961 | SEQ ID NO: 30967 |
| | | AA | DIQMTQSPSSLSASVGDRVTITCRASHNINSYLN WYQQKSGKAPKLLIYAASSLQSGVPSRFSGSGS GTDFTLTFSSLQPEDFATYYCQQSYSFPLTFGGG TKVEMR | QVQLVQSGAEVKKPGASVKVSCKASGYTFTGYYIH WVRQAPGQGLEWMAWINPNSGGTNYAQKFQGRV TMTRDTSITTTYMELSRLRSDDTAVYYCARGYSYG YNWFDPWGQGTLVTVSS |
| | | | SEQ ID NO: 26962 | SEQ ID NO: 30968 |
| iPS:436246 | 21-225_201G6 | NA | GATATTGTGATGACTCAGTCTCCACTCTCCCTG CCCGTCACCCCTGGAGAGCCGGCCTCCATCTC CTGCAGGTCTAGTCAGAGCCTCCTCCATAATA ATAGATACAACCATTTGGATTGGTACCTGCAG AAGCCAGGACAGTCTCCACAGCTCCTGATCTA TTTGGGTTCTAATCGGGCCTCCGGGGTCCCTG ACAGGTTCAGTGGCAGTGGATCAGGCACAGAT TTTACACTGAAAATCAGCAGAGTGGAGGCTGA GGATGTTGGGGTTTATTACTGCATGCAAGCTC TACAAACTCCCACTTTCGGCGGAGGGACCAAG GTGGAGATCAAA | GAGGTGCAGCTGTTGGAGTCTGGGGGAGGCTTG GTACAGCCTGGGGGGTCCCTGAGACTCTCCTGTG TAGCCTCTGGATTCACCTTTAGCAGCTATGCCAT GAGCTGGGTCCGCCAGGCTCCAGGGAAGGGGCT GGAGTGGGTCTCAACTATTAGTGGTAGTGGTGTT AGAACATACTACGCAGACTCCGTGAAGGGCCGG TTCACCATCTCCAGAGACAATTCCAAGAACACGC TGTTTCTGCAAATGAACAGCCTGAGAGCCGAGG ACACGGCCGTATATTACTGTGCGAAAGGGGGAG CTAGGAGCAGTGGCTGGTTCCACTTTGACTACTG GGGCCAGGGAACCCTGGTCACCGTCTCCTCA |
| | | | SEQ ID NO: 26963 | SEQ ID NO: 30969 |

EP 4 435 105 A2

FIGURE 50

| | | | | |
|---|---|---|---|---|
| | | AA | DIVMTQSPLSLPVTPGEPASISCRSSQSLLHNNRY NHLDWYLQKPGQSPQLLIYLGSNRASGVPDRFS GSGSGTDFTLKISRVEAEDVGVYYCMQALQTPT FGGGTKVEIK | EVQLLESGGGLVQPGGSLRLSCVASGFTFSSYAMS WVRQAPGKGLEWVSTISGSGVRTYYADSVKGRFTI SRDNSKNTLFLQMNSLRAEDTAVYYCAKGGARSS GWFHFDYWGQGTLVTVSS |
| | | | SEQ ID NO: 26964 | SEQ ID NO: 30970 |
| iPS:436248 | 21-225_202A3 | NA | GACATCCAGATGTCCCAATCTCCATCCTCCCT GTCTGCATCTGTTGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTGTGCTGCATCCAGG TTGCAAAGTGGGGTCCCATCAAGGTTCAGCGG CAGTGGATCTGGGACAGAATTCACTCTCACAA TCAGCAGCCTGCAGCCTGAAGATTTTGCAATT TATTACTGTCTACAGCATCATGACTACCCATTC ACTTTCGGCCCTGGGACCAAAGTGGATATCAA A | CAGGTGCAGCTGGAGCAGTCTGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACAGGACAAGGGC TTGAGTGGCTGGGATGGATGAACCCTAAGAGAG GTAACACAGGCTATGCACAGAAGTTCCAGGGCA GAGTCACCATGACCAGGAATACCTCCATAAGCA CAGCCCACATGGAGCTGAGCAGCCTGAGATCTG AGGACACGGCCGTGTATTACTGTGCGAGAGGAA GGTATAGCAGGGAGGATTACTACTACTATTATGA TATGGACGTCTGGGGCCAAGGGACCACGGTCAC CGTCTCCTCA |
| | | | SEQ ID NO: 26965 | SEQ ID NO: 30971 |
| | | AA | DIQMSQSPSSLSASVGDRVTITCRASQGIRNDLG WYQQKPGKAPKRLICAASRLQSGVPSRFSGSGS GTEFTLTISSLQPEDFAIYYCLQHHDYPFTFGPGT KVDIK | QVQLEQSGAEVKKPGASVKVSCKASGYTFTSYDIN WVRQATGQGLEWLGWMNPKRGNTGYAQKFQGR VTMTRNTSISTAHMELSSLRSEDTAVYYCARGRYS REDYYYYYDMDVWGQGTTVTVSS |
| | | | SEQ ID NO: 26966 | SEQ ID NO: 30972 |

2349

FIGURE 50

| | | | | |
|---|---|---|---|---|
| iPS:436250 | 21-225_201A4 | NA | GAAATAGTGATGACGCAGTCTCCAGCCACCCT GTCTGTGTCTCCAGGGGAAAGTGCCACCCTCT CCTGCAGGTCCAGTCAGAATATTAAAAGCAAC TTAGCCTGGTACCAGCAGAAACCTGGCCAGGC TCCCAGGCTCCTCATCTTTGGTGCATCCACCAG GGCCACTGGTATCCCAGCCAGGTTCAGTGGCA GTGGGTCTGGGACAGAGTTCACTCTCACCATC AGCAGCCTGCAGTCTGAAGATTTTGCAGTTTA TTACTGTCAGCAGTTTTATAACTGGCTGTGCA GTTTTGGCCAGGGGACCAAGCTGGAGCTCAAA | CAGGTACAGCTGCAGCAGTCAGGTCCAGGACTG GTGAAGCCCTCGCAGACCCTCTCACTCACCTGTG CCATCTCCGGGGACAGTGTCTCTAGCAACAGTGC TGCTTGGAACTGGATCAGGCAGTCCCCATCGAGA GGCCTTGAGTGGCTGGGAAGGACATACTACAGG TCCAAGTGGTATAATTATTATGAAGTATCTGTGA AAAGTCGAATAACCATCAACCCAGACACATCCA AGAACCAGTTCTCCCTGCAGCTGAACTCTGTGAC TCCCGAGGACACGGCTGTATATTTCTGTGCGAGA GATCAACGGTACTACGGTATGGACGTCTGGGGCC AAGGGACCACGGTCACCGTCTCCTCA |
| | | | SEQ ID NO: 26967 | SEQ ID NO: 30973 |
| | | AA | EIVMTQSPATLSVSPGESATLSCRSSQNIKSNLA WYQQKPGQAPRLLIFGASTRATGIPARFSGSGSG TEFTLTISSLQSEDFAVYYCQQFYNWLCSFGQGT KLELK | QVQLQQSGPGLVKPSQTLSLTCAISGDSVSSNSAA WNWIRQSPSRGLEWLGRTYYRSKWYNYYEVSVKS RITINPDTSKNQFSLQLNSVTPEDTAVYFCARDQRY YGMDVWGQGTTVTVSS |
| | | | SEQ ID NO: 26968 | SEQ ID NO: 30974 |
| iPS:436252 | 21-225_202A8 | NA | GAAATAGTGATGACGCAGTCACCAGCCACCCT GTCTGTGTCTCCAGGGGAAAGAGCCACCCTCT CCTGCAGGGCCAGTCAGAGAATTAACAACAA CTTAGCCTGGTACCAGCAGAACCCTGGCCAGG CTCCCAGGCTCCTCATTTATGGTGCATCCACCA GGGCCACTGGTGTCCCGGCCAGGTTCAGTGGC AGTGGGTCTGGGACAGAGTTCACTCTCACCAT CAGCAGCCTGCAGTCTGAAGACTTTACAGTTT ATTACTGTCAGCAGTATTATAACTGGCTGTGC AGTTTTGGCCAGGGGACCAAGCTGGAGATCAG A | CAGGTACAGCTGCAGCAGTCAGGTCCAGGACTG GTGAAGCCCTCGCAGACCCTCTCACTCACCTGTG CCATCTCCGGGGACAGTGTCTCTAGCAACAGTGC TGCTTGGAACTGGATCAGGCAGTCCCCATCGAGA GGCCTTGAGTGGCTGGGAAGGACATACTACAGG TCCAAGTGGTATAATGAGTATGCAGTATCTGTGA GAAGTCGAATAACCATCAACCCAGACACATCCA AGAACCAGTTCTCCCTGCAGCTGAACTCTGTGAC TCCCGAGGACACGGCTCTGTATTACTGTACAAGA GATCAACGGTACTACGGTATGGACGTCTGGGGCC AAGGGACCCCGGTCACCGTCTCCTCA |
| | | | SEQ ID NO: 26969 | SEQ ID NO: 30975 |

FIGURE 50

| | | | | |
|---|---|---|---|---|
| | | AA | EIVMTQSPATLSVSPGERATLSCRASQRINNNLA WYQQNPGQAPRLLIYGASTRATGVPARFSGSGS GTEFTLTISSLQSEDFTVYYCQQYYNWLCSFGQG TKLEIR | QVQLQQSGPGLVKPSQTLSLTCAISGDSVSSNSAA WNWIRQSPSRGLEWLGRTYYRSKWYNEYAVSVRS RITINPDTSKNQFSLQLNSVTPEDTALYYCTRDQRY YGMDVWGQGTPVTVSS |
| | | | SEQ ID NO: 26970 | SEQ ID NO: 30976 |
| iPS:436254 | 21-225_202C12 | NA | GATATTGTGCTGACTCAGTCTCCACTCTCCCTG CCCGTCACCCCTGGAGAGCCGGCCTCCATCTC CTGCAGGTCTAGTCAGAGCCTCCTGCATAATA ATAAATACAACCATTTGGATTGGTACCTGCAG AAGCCAGGGCAGTCTCCACAGCTCCTGATCTA TTTGGGTTCTAATCGGGCCTCCGGGGTCCCTG ACAGGTTCAGTGGCAGTGGATCAGGCACAGAT TTTACACTGAAAATCAGCAGAGTGGAGGCTGA GGATGTTGGGGTTTATTACTGCATGCAAGCTC TACAAACTCCCACTTTCGGCGGAGGGACCAAG GTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAACTATTAGTGGTAGTGGTGTT AGAACATACTACGCAGACTCCGTGAAGGGCCGG TCCACCATCTCCAGAGACAATTCCAAGAATACGC TGTTTCTGCAAATGAACAGCCTGAGAGCCGAGG ACACGGCCGTATATTACTGTGCGAAAGGGGGAG CTAGGAGCAGTGGCTGGTTCCACTTTGACTACTG GGGCCAGGGAACCCTGGTCACCGTCTCCTCA |
| | | | SEQ ID NO: 26971 | SEQ ID NO: 30977 |
| | | AA | DIVLTQSPLSLPVTPGEPASISCRSSQSLLHNNKY NHLDWYLQKPGQSPQLLIYLGSNRASGVPDRFS GSGSGTDFTLKISRVEAEDVGVYYCMQALQTPT FGGGTKVEIK | EVQLLESGGGLVQPGGSLRLSCAASGFTFSSYAMS WVRQAPGKGLEWVSTISGSGVRTYYADSVKGRSTI SRDNSKNTLFLQMNSLRAEDTAVYYCAKGGARSS GWFHFDYWGQGTLVTVSS |
| | | | SEQ ID NO: 26972 | SEQ ID NO: 30978 |

FIGURE 50

| | | | | |
|---|---|---|---|---|
| iPS:436256 | 21-225_202D9 | NA | GAAATTGTGTTGACGCAGTCTCCAGACACCCT GTCTTTGTCTCCAGGGGAAAGAGCCACCCTCT CCTGCAGGGCCAGTCAGAGTGTTAACAGCGGC TACTTAGCCTGGTACCAGCAGAAACCTGGCCA GTCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGCATTACTGTCAACAATATGAGACCTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGATGGGTCCTTCAGTCCTTACTACTGG AGCTGGATCCGCCAGCCCCCAGGGAAGGGGCTG GAGTGGATTGGGGAAATCAATCATAGTGGAAGC ACCAACTACAATCCGTCCCTCAAGAGTCGAGTCA CCATATCAGTAGACACGTCCAAGAACCAGTTCTC CCTGAAGCTGAGCTCTGTGACCGCCGCGGACACG GCTGTATATTACTGTGCGAGAGACTACGGGGGTT TAGACTACTGGGGCCAGGGAACCCTGGTCACCGT CTCCTCA |
| | | | SEQ ID NO: 26973 | SEQ ID NO: 30979 |
| | | AA | EIVLTQSPDTLSLSPGERATLSCRASQSVNSGYLA WYQQKPGQSPRLLIYGASSRATGIPDRFSGSGSG TDFTLTISRLEPEDFAVHYCQQYETSPWTFGQGT KVEIK | QVQLQQWGAGLLKPSETLSLTCAVYDGSFSPYYW SWIRQPPGKGLEWIGEINHSGSTNYNPSLKSRVTISV DTSKNQFSLKLSSVTAADTAVYYCARDYGGLDYW GQGTLVTVSS |
| | | | SEQ ID NO: 26974 | SEQ ID NO: 30980 |
| iPS:436258 | 21-225_202F12 | NA | GAAATAGTGATGACGCAGTCTCCAGCCACCCT GTCTGTGTCTCCAGGGGAAAGAGCCACCCTCT CCTGCAGGGCCAGTCAGAGTGTTCTGAACAAC TTAGCCTGGTACCAGCAGAGACCTGGCCAGGC TCCCAGGCTCCTCATCTATGGTGCATCCACTA GGGCCACTGGTATCCCAGCCAGGTTCAGTGGC AGTGGGTCTGGGACAGAGTTCACTCTCACCAT CAGCAGCCTGCAGTCTGAAGATTTTGCAGTTT ATTACTGTCAGCAGTATGATAACTGGCCTCCG TGCAGTTTTGGCCAGGGGACCAAGCTGGAGAT CAAA | CAGGTGCAGCTGGTGGAGTCTGGGGGAGGCGTG GTCCAGCCTGGGAGGTCCCTGAGACTCTCCTGTG AAGCGTCTGGATTCACCTTCAGTTACTATGGCAT GCACTGGGTCCGCCAGGCTCCAGGCAAGGGACT GGAGTGGGTGGCAATTATATGGTATGATGGAAGT AATAAATTCTATGCAGACTCCGTGAAGGGCCGAT TCACCATCTCCAGAGACAGTTCCAAGAACACGCT GTATCTGCAAATGAACAGCCTGAGAGCCGAGGA CACGGCTGTGTATTACTGTGCGAGTAATATAGCA GCAGCTGCCCCTTACTTTGACTACTGGGGCCAGG GAACCCTGGTCACCGTCTCCTCA |
| | | | SEQ ID NO: 26975 | SEQ ID NO: 30981 |

FIGURE 50

| | | | | |
|---|---|---|---|---|
| | | AA | EIVMTQSPATLSVSPGERATLSCRASQSVLNNLA WYQQRPGQAPRLLIYGASTRATGIPARFSGSGSG TEFTLTISSLQSEDFAVYYCQQYDNWPPCSFGQG TKLEIK | QVQLVESGGGVVQPGRSLRLSCEASGFTFSYYGMH WVRQAPGKGLEWVAIIWYDGSNKFYADSVKGRFT ISRDSSKNTLYLQMNSLRAEDTAVYYCASNIAAAA PYFDYWGQGTLVTVSS |
| | | | SEQ ID NO: 26976 | SEQ ID NO: 30982 |
| iPS:436260 | 21-225_203H1 | NA | GACATCCAGATGACCCAGTCTCCATCCTCACT GTCTGCATCTGTCGGAGACAGAGTCACCATCA CTTGTCGGGCGAGTCAGGGCATTGGCAATTAT TTAGCCTGGTTTCAGCAGAAACCAGGGAAAGC CCCTAAGTCCCTGATCTATGTTGCATCCAGGTT GCAAAGTGGGGTCCCATCAAAGTTCAGCGGCA CTGGATCTGGGTCAGATTTCACTCTCACCATC AGCAGCCTGCAGCCTGACGATTTTGCGACTTA TTACTGCCAACGGTATCATACTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATAGAAC AGTTGGCTACAACGGTATGGACGTCTGGGGCCA AGGGACCACGGTCACCGTCTCCTCA |
| | | | SEQ ID NO: 26977 | SEQ ID NO: 30983 |
| | | AA | DIQMTQSPSSLSASVGDRVTITCRASQGIGNYLA WFQQKPGKAPKSLIYVASRLQSGVPSKFSGTGS GSDFTLTISSLQPDDFATYYCQRYHTYPLTFGGG TKVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDRTVG YNGMDVWGQGTTVTVSS |
| | | | SEQ ID NO: 26978 | SEQ ID NO: 30984 |

FIGURE 50

| iPS:436262 | 21-225_203E3 | NA | GACATCCAGATGACCCAGTCTCCATCCTCCCC GTCTGCATCTGTTGGAGACAGAGTCACCATCA CTCGCCGGGCAAGTCACAACATTAACAGCTAT TTAAATTGGTATCAGCAGAAATCAGGGAAAGC CCCTAAACTCCTTATCTATGCTGCATCCAGTTT GCAAAGTGGGGTCCCGTCAAGGTTCAGTGGCA GTGGATCTGGGACAGATTTCACTCTCACTTTC AGTAGTCTACAACCTGAAGATTTTGCAACTTA CTACTGTCAACAGAGTTACAGTTTCCCGCTCA CTTTCGGCGGAGGGACCAAGGTGGAGATGAG G | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TCCACTGGGTGCGACAGGCCCCTGGACAAGGGC TTGAGTGGATGGCATGGATCAACCCTAATAGTGG TGGCACAAACTATGCACAGAAGTTTCAGGGCAG GGTCACCATGACCAGGGACACGTCCATCACCAC AGCCTACATGGAGCTGAGCAGGCTGAGATCTGA CGACACGGCCGTGTATTACTGTGCGAGAGGATAC AGCTATGGTTACAACTGGTTCGACCCCTGGGGCC AGGGAACCCTGGTCACCGTCTCCTCA |
|---|---|---|---|---|
| | | | SEQ ID NO: 26979 | SEQ ID NO: 30985 |
| | | AA | DIQMTQSPSSPSASVGDRVTITRRASHNINSYLN WYQQKSGKAPKLLIYAASSLQSGVPSRFSGSGS GTDFTLTFSSLQPEDFATYYCQQSYSFPLTFGGG TKVEMR | QVQLVQSGAEVKKPGASVKVSCKASGYTFTGYYIH WVRQAPGQGLEWMAWINPNSGGTNYAQKFQGRV TMTRDTSITTAYMELSRLRSDDTAVYYCARGYSYG YNWFDPWGQGTLVTVSS |
| | | | SEQ ID NO: 26980 | SEQ ID NO: 30986 |
| iPS:436264 | 21-225_203F7 | NA | GACATCCAGATGACCCAGTCTCCATCCTCCCG TTTTGCATCTGTAGGAGACAGAGTCACCATCA CTTGCCGGGCAAGTCAGGGCATTAGACATGAT TTAGGCTGGTATCAGCAGAAACCAGGGAAAG CCCTTAAGCGCTTGATATATGCTGCATCCAGTT TGCAAAGTGGGGTCCCATCAAGGTTCAGCGGC AGTGGATGTGGGACAGAATTCACTCTCACAAT CAGCAGCGTGCAGCCTGAAGATTTTGCAAATT ATTACTGTCTACAGCATTATAGTTTCCCTCGGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAGGAACACGCT GTATCTGCAAATGAACAGCCTGAGAGCCGAGGA CACGGCTGTGTATTACTGTGCGAGAGAACGGTAT AGCAGTGGCTTGTACGACTACGGTATGGACGTCT GGGGCCAAGGGACCACGGTCACCGTCTCCTCA |
| | | | SEQ ID NO: 26981 | SEQ ID NO: 30987 |

2354

FIGURE 50

| | | | | |
|---|---|---|---|---|
| | | AA | DIQMTQSPSSRFASVGDRVTITCRASQGIRHDLG WYQQKPGKALKRLIYAASSLQSGVPSRFSGSGC GTEFTLTISSVQPEDFANYYCLQHYSFPRTFGQG TKVEIK | QVQLVESGGGVVQPGRSLRLSCAASGFTFSDYVM HWVRQAPGKGLEWVAVIWYDGSNKYYVDSVKGR FTISRDNSRNTLYLQMNSLRAEDTAVYYCARERYS SGLYDYGMDVWGQGTTVTVSS |
| | | | SEQ ID NO: 26982 | SEQ ID NO: 30988 |
| IPS:436268 | 21-225_203B9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATAGGGCATCCAGT GTGCAAAATGGGGTCCCATCAAGGTTCAGCGG CAGTGGATCTGGGACAGAATTCACTCTCACAA TCAGCAGCCTGCAGCCTGAAGATTTTGCAACT TATCACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCAGGATTCACCCTCAGTAGTTTTGGCAT GCACTGGGTCCGCCAGGCTCCAGGCAAGGGGCT GGAGTGGGTGGCAGTTATATGGTATGATGTAAAT AATAAATACTATGCAGACTCCGTGAAGGGCCGA TTCACAATCTCCAGAGACAATTCCAAGAACACGC TGTATCTCCAAATGAACAGCCTGAGACCCGAGG ACACGGCTGTGTATTACTGTGCGAGAGAACTGGG GTTCCTCTCTGACTACTGGGGGCCAGGGAATCCTG GTCACCGTCTCCTCA |
| | | | SEQ ID NO: 26983 | SEQ ID NO: 30989 |
| | | AA | DIQMTQSPSSLSASVGDRVTITCRASQGIRNDLG WYQQKPGKAPKRLIYRASSVQNGVPSRFSGSGS GTEFTLTISSLQPEDFATYHCLQHNSYPFTFGPGT KVDIK | QVQLVESGGGVVQPGRSLRLSCAASGFTLSSFGMH WVRQAPGKGLEWVAVIWYDVNNKYYADSVKGRF TISRDNSKNTLYLQMNSLRPEDTAVYYCARELGFL SDYWGQGILVTVSS |
| | | | SEQ ID NO: 26984 | SEQ ID NO: 30990 |

FIGURE 50

| iPS:436270 | 21-225_203F10 | NA | GACATCGTGATGACCCAGTCTCCAGACTCCCT GGCTGTGTCTCTGGGCGAGAGGGCCACCATCA ACTGCAAGTCCAGCCAGAGTGTTTTTTTCCACT CGAACAATAAGAACTACTTAGCTTGGTACCAG CAGAAACCAGGACAGCCTCCTAAGTTGCTCAT TTACTGGGCATCTACCCGGGAATCCGGGGTCC CTGACCGATTCAGTGGCAGCGGGTCTGGGACA GATTTCACTCTCACCATCAGCAGCCTGCAGGC TGAAGATGTGACAGTTTATTACTGTCAACAAT ATTTTAGTCTTCCATTCACTTTCGGCCCTGGGA CCAAAGTGGATATCACA | CAGGTGCAACTGGTGGAGTCTGGGGGAGGCTTG GTCAAGCCTGGAGGGTCCCTGAGACTCTCCTGTG CAGCCTCTGGATTCACCTTCAGTGACTACTACAT GAGCTGGATCCGCCAGGCTCCAGGGAAGGGGCT GGAGTGGGTTTTATACATTAGTGGTAGTGGTACT ACCACATACTACGCAGACTCTGTGAAGGGCCGAT TCACCATCTCCAGGGACAACGCCAAGAACTCACT GTATCTGCAAATGAACAGCCTGAGAGCCGAGGA CACGGCCGTGTATTACTGTGCGAGAGATAGGGG GGGTTTGGACGTCTGGGGCCAAGGGACCACGGT CACCGTCTCCTCA |
|---|---|---|---|---|
| | | | SEQ ID NO: 26985 | SEQ ID NO: 30991 |
| | | AA | DIVMTQSPDSLAVSLGERATINCKSSQSVFFHSN NKNYLAWYQQKPGQPPKLLIYWASTRESGVPD RFSGSGSGTDFTLTISSLQAEDVTVYYCQQYFSL PFTFGPGTKVDIT | QVQLVESGGGLVKPGGSLRLSCAASGFTFSDYYMS WIRQAPGKGLEWVLYISGSGTTTYYADSVKGRFTIS RDNAKNSLYLQMNSLRAEDTAVYYCARDRGGLD VWGQGTTVTVSS |
| | | | SEQ ID NO: 26986 | SEQ ID NO: 30992 |
| iPS:436272 | 21-225_201F5 | NA | GACATCGTGATGACCCAGTCTCCAGAGTCCCT GGCTGTGTCTCTGGGCGAGAGGGCCACCATCA ACTGCAAGTCCAGCCAGAGTGTTTTTATACAGT TCCAACAATAAGAACTACTTAGTTTGGTACCA GCAGAAACCAGGACAGCCTCCTAAACTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGGCTGCGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCTATAGCAGT GGCTGGTACTACTTTGACTACTGGGGCCAGGGAA CCCTGGTCACCGTCTCCTCA |
| | | | SEQ ID NO: 26987 | SEQ ID NO: 30993 |

FIGURE 50

| | | | | |
|---|---|---|---|---|
| | | AA | DIVMTQSPESLAVSLGERATINCKSSQSVLYSSN NKNYLVWYQQKPGQPPKLLIYWASTRESGVPD RFSGSGSGTDFTLTISSLQAEDVAVYYCQQYYST PPTFGQGTKVEIK | QVQLVQSGAAVKKPGASVKVSCKASGYTFTNYDI NWVRQATGQGLEWMGWMHPNSGNTGYAQKFQG RVTMTRNTSISTAYMELSSLRSEDTAVYYCAYSSG WYYFDYWGQGTLVTVSS |
| | | | SEQ ID NO: 26988 | SEQ ID NO: 30994 |
| iPS:436274 | 21-225_204H3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTGAACTCCTGATCTATGCTGCAGCCAGT TTGCAAGGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAACTCC AA | CAGGTGCAGCTGGTGGAGTCTGGGGGAGGCGTG GGCCAGCCTGGGAGGTCCCTGAGACTCTCCTGTA CAGCGTCTGGATTCACCTTCAGTAGCTATGTCAT GCACTGGGTCCGCCAGGCTCCAGGCAAGGGGCT GGAGTGGGTGGCAGTTATATGGTATGATGGAAGT AATAAATATAATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACCGTA TAGCAGCAGCTGGTACGACTACGGTATGGACGTC TCGGGCCAAGGGACCACGGTCACCGTCTCCTCA |
| | | | SEQ ID NO: 26989 | SEQ ID NO: 30995 |
| | | AA | DIQMTQSPSSLSASVGDRVTITCRASQGIRNDLG WYQQKPGKAPELLIYAAASLQGGVPSRFSGSGS GTEFTLTISSLQPEDFATYYCLQHYSYPRTFGQG TKVELQ | QVQLVESGGGVGQPGRSLRLSCTASGFTFSSYVMH WVRQAPGKGLEWVAVIWYDGSNKYNADSVKGRF TISRDNSKNTLYLQMNSLRAEDTAVYYCAREPYSS SWYDYGMDVSGQGTTVTVSS |
| | | | SEQ ID NO: 26990 | SEQ ID NO: 30996 |

FIGURE 50

| | | | | |
|---|---|---|---|---|
| iPS:436276 | 21-225_204H4 | NA | GACATCCAGATGACCCTGTCTCCATCCTCCCC GTCTGCATTTGTTGGAGACAGAGTCACCATCA CTCGCCGGGCAAGTCACAACATTAACAGCTAT TTAAATTGGTATCAGCAGAAATCAGGGAAAGC CCCTAAACTCCTTATCTATGCTGCATCCAGTTT GCAAAGTGGGGTCCCGTCAAGGTTCAGTGGCA GTGGATCTGGGACAGATTTCACTCTCACTTTC AGTAGTCTACAACCTGAAGATTTTGCAACTTA CTACTGTCAACAGAGTTACAGTTTCCCGCTCA CTTTCGGCGGAGGGACCAAGGTGGAGATGAG G | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TCCACTGGGTGCGACAGGCCCCTGGACAAGGGC TTGAGTGGATGGCATGGATCAACCCTAATAGTGG TGGCACAAACTATGCACAGAAGTTTCAGGGCAG GGTCACCATGACCAGGGACACGTCCATCACCAC AGCCTACATGGAGCTGAGCAGGCTGAGATCTGA CGACACGGCCGTGTATTACTGTGCGAGAGGATAC AGCTATGGTTACAACTGGTTCGACCCCTGGGGCC AGGGAACCCTGGTCACCGTCTCCTCA |
| | | | SEQ ID NO: 26991 | SEQ ID NO: 30997 |
| | | AA | DIQMTLSPSSPSAFVGDRVTITRRASHNINSYLN WYQQKSGKAPKLLIYAASSLQSGVPSRFSGSGS GTDFTLTFSSLQPEDFATYYCQQSYSFPLTFGGG TKVEMR | QVQLVQSGAEVKKPGASVKVSCKASGYTFTGYYIH WVRQAPGQGLEWMAWINPNSGGTNYAQKFQGRV TMTRDTSITTAYMELSRLRSDDTAVYYCARGYSYG YNWFDPWGQGTLVTVSS |
| | | | SEQ ID NO: 26992 | SEQ ID NO: 30998 |
| iPS:436278 | 21-225_201F2 | NA | GAGATAGTGATGACGCAGTCTCCAGCCACCCT GTCTGTGTCTCCAGGGGAAAGAGCCACCCTCT CCTGCAGGGCCAGTCAGAATATTAAAAGCAAC TTAGCCTGGTACCAGCAGAAACCTGGCCAGGC TCCCAGGCTCCTCATCTTTGGTGCATCCACCAG GGCCACTGGTATCCCAGCCAGGTTCAGTGGCA GTGGGTCTGGTACAGAGTTCACTCTCACCATC AGCAGCCTGCAGTCTGAAGATTTTGCAGTGTA TTACTGTCAGCAGTTTTATAACTGGCTGTGCA GTTTTGGCCAGGGGACCAAGCTGGAGCTCAAG | CAGGTACAGCTGCAGCAGTCAGGTCCAGGACTG GTGAAGCCCTCGCAGACCCTCTCACTCACCTGTG CCATCTCCGGGGACAGTGTCTCTAGCAACAGTGC TGCTTGGAACTGGATCAGGCAGTCCCCATCGAGA GGCCTTGAGTGGCTGGGAAGGACATACTACAGG TCCAAGTGGTATAATTATTATGAAGTATCTGTGA GAAGTCGAGTAACCATCAACCCAGACACATCCA AGAACCAGTTCTCCCTGCAACTGAACTCTGTGAC TCCCGAGGACACGGCTGTGTATTTCTGTGCGAGA GATCAACGGTACTACGGTATGGACGTCTGGGGCC AAGGGACCACGGTCACCGTCTCCTCA |
| | | | SEQ ID NO: 26993 | SEQ ID NO: 30999 |

EP 4 435 105 A2

FIGURE 50

| | | | EIVMTQSPATLSVSPGERATLSCRASQNIKSNLA WYQQKPGQAPRLLIFGASTRATGIPARFSGSGSG TEFTLTISSLQSEDFAVYYCQQFYNWLCSFGQGT KLELK | QVQLQQSGPGLVKPSQTLSLTCAISGDSVSSNSAA WNWIRQSPSRGLEWLGRTYYRSKWYNYYEVSVRS RVTINPDTSKNQFSLQLNSVTPEDTAVYFCARDQR YYGMDVWGQGTTVTVSS |
|---|---|---|---|---|
| | | AA | SEQ ID NO: 26994 | SEQ ID NO: 31000 |
| iPS:436280 | 21-225_204D6 | NA | GACAGCCAGATGACCCAGTCTCCATCCTCCCT GTCTGCATCTGTAGGAGACAGAGTCACCATCA CTTGCCGGGCAAGTCGGAGCGTTCACACCTAT TTAAAATTGGTATCAACAGAAGCCAGGGAAAG CCCCTAAGGTCCTGATCTATGGTGCATCCAGT TTGCAACGTGGGGTCCCATCAAGGTTCAGTGG CAGTGGATCTGGGACAGATTTCACTCTCACCA TCAGCAGTCTGCAACCTGAAGATGTTGCAACT TACTACTGTCAACAGAGTTACAGTTCCCCGCT CACTTTCGGCGGGGGGACCAAGGTGGAGATCC AA | GAGGTGCAGGTGTTGGAGTCTGGGGGAGGCTTG GTGCAGCCTGGGGGGTCCCTGCGACTCTCCTGTG CAGCCTCTGGATTCACCTTTAGCACCTATGCCAT GAGCTGGGTCCGCCAGGCTCCAGGGAAGGGGCT GGAGTGGGTCTCAGCTATTAGTGGTAGTGGTGGT AGCACATATTACGCAGACTCCGTGAAGGGCCGG TTCACCATCTCCAGAGACAATTCCAAGAACACGC TGTATCTGCAAATGGACAGCCTGAGAGCCGAGG ACACGGCCGTATATTACTGTGCGAAAGGGATAA GTGGAACCGGCTCCTACTACTACTACGGTGTGGA CGTCTGGGGCCAAGGGACCACGGTCACCGTCTCC TCA |
| | | | SEQ ID NO: 26995 | SEQ ID NO: 31001 |
| | | AA | DSQMTQSPSSLSASVGDRVTITCRASRSVHTYLN WYQQKPGKAPKVLIYGASSLQRGVPSRFSGSGS GTDFTLTISSLQPEDVATYYCQQSYSSPLTFGGG TKVEIQ | EVQVLESGGGLVQPGGSLRLSCAASGFTFSTYAMS WVRQAPGKGLEWVSAISGSGGSTYYADSVKGRFTI SRDNSKNTLYLQMDSLRAEDTAVYYCAKGISGTGS YYYYGVDVWGQGTTVTVSS |
| | | | SEQ ID NO: 26996 | SEQ ID NO: 31002 |

2359

FIGURE 50

| iPS:436282 | 21-225_204G6 | NA | GACATCCGGATGACCCAGTCTCCATCCTCACT GTCTGCATCTGTAGGAGACAGAATCACCATCA CTTGTCGGACGAGTCAGGGCATTGGCAATTAT TTAGCCTGGTTTCAGCAGAAACCAGGGAAAGC CCCTAAGTCCCTGATCTATGCTGCATCCAGTTT GCAAAGTGGGGTCCCATCAAGGTTCAGCGGCA GTGGATCTGGGACAGATTTCACTCTCACCATC AGCAGCCTGCAGCCTGAAGATTTTGCAACTTA TTACTGCCAACACTATCTTAGTTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTA CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGAGG TGTCGGCTACGACGGTATGGACGTCTGGGGCCAA GGGACCACGGTCACCGTCTCCTCA |
| | | | SEQ ID NO: 26997 | SEQ ID NO: 31003 |
| | | AA | DIRMTQSPSSLSASVGDRITITCRTSQGIGNYLAW FQQKPGKAPKSLIYAASSLQSGVPSRFSGSGSGT DFTLTISSLQPEDFATYYCQHYLSYPLTFGGGTK VEIK | QVQLVESGGGVVQPGRSLRLSCTASGFTFSSYGMH WVRQAPGKGLEWVAVIWYDGSNKNYADSVKGRF TISRDNSKNTLYLQMNSLRAEDTAVYYCARDRGV GYDGMDVWGQGTTVTVSS |
| | | | SEQ ID NO: 26998 | SEQ ID NO: 31004 |
| iPS:436284 | 21-225_204G8 | NA | GACATCCAGATGACCCAGTCTCCATCCTCACT GTCTGCATCTGTAGGAGACAGAGTCACCATCA CTTGTCGGGCGAGTCAGGGCATAAGTAATCAT TTAGCCTGGTTTCAGCAGAAACCAGGGAAAGC CCCTAAGTCCCTGATCTTTGCTGCATCCAGTTT GCAAAGTGGGGTCCCATCACAGTTCAGCGGCA GTGGATCTGGGACCGAATTCACTCTCACCATC AGCAGCCTGCAGCCTGAAGATTTTGCAACTTA TTACTGCCAACAGTATAGTAATTACCCGGTCA CTTTCGGCGGAGGGACCAAGGTGGAGATCAA A | CAGGTGCAGCTGGTGGAGTCTGGGGGAGACGTG GTCCAGCCTGGGAGGTCCCTGAGACTCTCATGTG CAGCGTCTGGATTCACCTTCAGTAGCTATGGCAT GCACTGGGGCCGCCAGGCTCCAGGCAAGGGGCT GGAGTGGGTGGCAGTTATATGGTATGATGGTAGC AATGAAAATTATGTAGCCTCCGTGAAGGGCCGAT TCACCATCTTCAGAGACAATTCCAAGAACACGCT GTATCTGCAAATGAACAGCCTGAGAGCCGAGGA CACGGCTGTGTATTACTGTGCGAGAGATCTGGGG ATAGGGTATTACGGTATGGACGTCTGGGGCCAAG GGACCACGGTCACCGTCTCCTCA |
| | | | SEQ ID NO: 26999 | SEQ ID NO: 31005 |

FIGURE 50

| | | | | |
|---|---|---|---|---|
| | | AA | DIQMTQSPSSLSASVGDRVTITCRASQGISNHLA WFQQKPGKAPKSLIFAASSLQSGVPSQFSGSGSG TEFTLTISSLQPEDFATYYCQQYSNYPVTFGGGT KVEIK | QVQLVESGGDVVQPGRSLRLSCAASGFTFSSYGMH WGRQAPGKGLEWVAVIWYDGSNENYVASVKGRF TIFRDNSKNTLYLQMNSLRAEDTAVYYCARDLGIG YYGMDVWGQGTTVTVSS |
| | | | SEQ ID NO: 27000 | SEQ ID NO: 31006 |
| IPS:436286 | 21-225_204H8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATTCTGCATCCAGTT TGCAAAGTGGGGTCCCATCAAGGTTCAGCGGC CGTGGATCTGGGACAGAATTCACTCTCACAGT CAGCAGCCTGCAGCCTGAAGATTTTGCAACTT ATTACTGTCTACAACATAATAGTTACCCTCTCA CTTTCGGCGGAGGGACCAAGGTGGAGATCAA A | CAGGTGCAGCTACAGCAGTGGGGCGCAGGACTG TTGAAGCCTTCGGAGACCCTGTTCCTCACCTGCG CTGTCTATGGTGGGTCCTTCAGTGGTTACTTCTGG ACCTGGGTCCGCCAGCCCCCAGGGAAGGGACTG GAGTGGATTGGGGAAATCAGTCATAGTGGAAGC ACCAGTTACAACCCGTCCCTCAAGAGTCGAGTCA CCATATCAGTAGACAAGTCCAAGAACCAGTTCTC CCTGAAGCTGAGCTCTGTGACCGCCGCGGACACG GCTGTGTATTACTGTGCGAGGGACTACGGGGCCG ACTACTGGGGCCAGGGAACCCTGGTCACCGTCTC CTCA |
| | | | SEQ ID NO: 27001 | SEQ ID NO: 31007 |
| | | AA | DIQMTQSPSSLSASVGDRVTITCRASQGIRNDLG WYQQKPGKAPKRLIYSASSLQSGVPSRFSGRGS GTEFTLTVSSLQPEDFATYYCLQHNSYPLTFGGG TKVEIK | QVQLQQWGAGLLKPSETLFLTCAVYGGSFSGYFW TWVRQPPGKGLEWIGEISHSGSTSYNPSLKSRVTIS VDKSKNQFSLKLSSVTAADTAVYYCARDYGADY WGQGTLVTVSS |
| | | | SEQ ID NO: 27002 | SEQ ID NO: 31008 |

FIGURE 50

| iPS:436290 | 21-225_205G3 | NA | GAAATCGTGTTGACGCAGTCTCCAGGCACCCT GTCTTTGTCTCCAGGGGAAAGAGCCACCCTCT CCTGCAGGGCCAGTCAGAATGTTAGTTACAGC TACTTAGCCTGGTACCAGCAGAAACCTGGCCA GGCTCCCAGGCTCCTCATCTATGGTGCATCCA GGAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAGCTCACCG TGCAGTTTTGGCCAGGGGACCAAGCTGGAGAT CAAA | CAGGTGCAGCTACAGCAGTGGGGCGCAGGACTG TTGAAGCCTTCGGAGACCCTGTCCCTCACCTGCG CTGTCTTTGGTGGGTCCTTCAGTGGTCACTACTGG AGCTGGATCCGCCAGCCCCCAGGGAAGGGGCTG GAGTGGATTGGGGAAATGTATCATTTTGGAAACA CCAACTACAACCCGTCCCTCAAGAGTCGAGTCAC CATGTCAGTAGACACGTCCAAGAAACAGTTCTCC CTGAAGCTGAGCTCTGTGACCGCCGCGGACACG GCTGTGTATTACTGTGCGAGAGTGGGGCAGTGGC TGGCTTTTGATATCTGGGGCCAAGGGACAATGGT CACCGTCTCTTCA |
| | | | SEQ ID NO: 27003 | SEQ ID NO: 31009 |
| | | AA | EIVLTQSPGTLSLSPGERATLSCRASQNVSYSYLA WYQQKPGQAPRLLIYGASRRATGIPDRFSGSGSG TDFTLTISRLEPEDFAVYYCQQYGSSPCSFGQGT KLEIK | QVQLQQWGAGLLKPSETLSLTCAVFGGSFSGHYW SWIRQPPGKGLEWIGEMYHFGNTNYNPSLKSRVTM SVDTSKKQFSLKLSSVTAADTAVYYCARVGQWLA FDIWGQGTMVTVSS |
| | | | SEQ ID NO: 27004 | SEQ ID NO: 31010 |
| iPS:436292 | 21-225_205H3 | NA | GACATCCCGATGACCCAGTCTCCATCCTCACT GTCTGCATCTGTAGGAGACAGAGTCACCATCA CTTGTCGGGCGAGTCAGGCCATTAGTAATCAT TTAGCCTGGTTTCAGCTGAAACCAGGGAAAGC CCCTAAGTCCCTGATCTATGCTGCATCCAGTTT GCAAAGTGGGGTCCCATCAAAGTTCAGCGGCA GTGGATCTGGGTCAGATTTCACTCTCACCATC AGCAGCCTGCAGCCTGAAGATTTTGCAACTTA TTACTGCCAACAATATAGTAATTACCCACTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGCGAGAGATAGATC AGTTGGCTACGACGGTACGGACGTCTGGGGCCA AGGGACCACGGTCACCGTCTCCTCA |
| | | | SEQ ID NO: 27005 | SEQ ID NO: 31011 |

2362

FIGURE 50

| | | | | |
|---|---|---|---|---|
| | | AA | DIPMTQSPSSLSASVGDRVTITCRASQAISNHLA WFQLKPGKAPKSLIYAASSLQSGVPSKFSGSGSG SDFTLTISSLQPEDFATYYCQQYSNYPLTFGGGT KVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARDRSVG YDGTDVWGQGTTVTVSS |
| | | | SEQ ID NO: 27006 | SEQ ID NO: 31012 |
| iPS:436294 | 21-225_205G4 | NA | GAAATAGTGATGACGCAGTCTCCAGCCACCCT GTCTGTGTCTCCAGGGGAAAGAGCCACCCTCT CCTGCAGGGCCAGTCAGAATATTAAAAGCAAC TTAGCCTGGTACCAGCAGAAACCTGGCCAGGC TCCCAGGCTCCTCATCTTTGGTGCATCCACCAG GGCCACTGGTATCCCAGCCAGGTTCAGTGGCA GTGGGTCTGGGACAGAGTTCACTCTCACCATC AGCAGCCTGCAGTCTGAAGATTTTGCAGTTTA TTACTGTCAGCAGTTTTATAACTGGCTGTGCA GTTTTGGCCAGGGGACCAAGCTGGAGCTCAAA | CAGGTACAGCTGCAGCAGTCAGGTCCAGGACTG GTGAAGCCCTCGCAGACCCTCTCACTCACCTGTG CCATCTCCGGGGACAGTGTCTCTAGCAACAGTGC TGCTTGGAACTGGATCAGGCAGTCCCCATCGAGA GGCCTTGAGTGGCTGGGAAGGACATATTACAGGT CCAAGTGGTATAATTATTATGAAGTATCTGTGAG AAGTCGAATAACCATCAACCCAGACACATCCAA GAACCAGTTCTCCCTGCAGCTGAATTCTGTGACT CCCGAGGACACGGCTGTGTATTTCTGTGCGAGAG ATCAACGGTACTACGGTATGGACGTCTGGGGCCA AGGGACCACGGTCACCGTCTCCTCA |
| | | | SEQ ID NO: 27007 | SEQ ID NO: 31013 |
| | | AA | EIVMTQSPATLSVSPGERATLSCRASQNIKSNLA WYQQKPGQAPRLLIFGASTRATGIPARFSGSGSG TEFTLTISSLQSEDFAVYYCQQFYNWLCSFGQGT KLELK | QVQLQQSGPGLVKPSQTLSLTCAISGDSVSSNSAA WNWIRQSPSRGLEWLGRTYYRSKWYNYYEVSVRS RITINPDTSKNQFSLQLNSVTPEDTAVYFCARDQRY YGMDVWGQGTTVTVSS |
| | | | SEQ ID NO: 27008 | SEQ ID NO: 31014 |

FIGURE 50

| iPS:436296 | 21-225_205F5 | NA | GACATCCAGATGACCCAGTCTCCATCCTCACT GTCTGCATCTGTAGGAGACAGAGTCACCATCA CTTGTCGGGCGAGTCAGGGCATTGGCAATTAT TTAGCCTGGTTTCAGCAGAAACCAGGGAAAGC CCCTAAGTCCCTGATCTATGGTGTCTCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TACCTGCCAACAATATAGTAATTACCCTCTCA CTTTCGGCGGAGGGACCAAGGTGGACATCAG A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGATATGGCAT GCACTGGGTCCGCCAGGCTCCAGGCAAGGGACT GGAGTGGGTGGCAGTTATATGGTATGATGGAAGT AATGAGAATTATGTAGACTCCGTGAAGGGCCGAT TCACCATCTCCAGAGACACTTCCAAGAAAATGCT GTTTCTGCAAATGAACAGCCTGAGAACCGATGAC ACGGCTGTGTATTACTGTGCGAGAGATATGGGGA TAGGGTATTATGGTATGGACGTCTGGGGCCAAGG GACCACGGTCACCGTCTCCTCA |
|---|---|---|---|---|
| | | | SEQ ID NO: 27009 | SEQ ID NO: 31015 |
| | | AA | DIQMTQSPSSLSASVGDRVTITCRASQGIGNYLA WFQQKPGKAPKSLIYGVSSLQSGVPSKFSGSGSG TDFTLTISSLQPEDFATYTCQQYSNYPLTFGGGT KVDIR | QVQLVESGGGVVQPGRSLRLSCAASGFTFSRYGMH WVRQAPGKGLEWVAVIWYDGSNENYVDSVKGRF TISRDTSKKMLFLQMNSLRTDDTAVYYCARDMGIG YYGMDVWGQGTTVTVSS |
| | | | SEQ ID NO: 27010 | SEQ ID NO: 31016 |
| iPS:436302 | 21-225_205G7 | NA | GAAATTGTGTTGACGCAGTCTCCAGGCACCCT GTCTTTGTCTCCAGGGGAAAGAGCCACCCTCT CCTGCAGGGCCAGTCAGAGTGTTTTCAGCAAC TACTTAGCCTGGTACCAGCAGAAACCTGGCCA GGCTCCCAGGCTCCTCATCTATGGTGCATCCA GCAGGGCCGCTGGCATCCCCGACAGGTTCAGT GGCAGTGGGTCTGGGACAGACTTCACTCTCAC CATCAGCAGACTGGAGCCTGAAAATTTTGCAG TGTATTACTGTCAGCAGTATGAAAGTTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCTATGGTGGGTCCTTCAGTGTTTATTACTGG AGCTGGATCCGCCAGCCCCCAGGGAAGGGGCTG GAGTGGATTGGGGAAAGCAATCAGAGTGGACGC ACCACCTACAACCCGTCCCTCAAGAGTCGAGTCA CCATATCAGTAGACACGTCCAAGAACCAGTTCTC CCTGAATCTGATCTCTGTGACCGCCGCGGACACG GCTGTGTATTACTGTGCGAGGGACTACGGTGTCT TTGACTACTGGGGCCAGGGAACCCTGGTCACCGT CTCCTCA |
| | | | SEQ ID NO: 27011 | SEQ ID NO: 31017 |

FIGURE 50

| | | | | |
|---|---|---|---|---|
| | | AA | EIVLTQSPGTLSLSPGERATLSCRASQSVFSNYLA WYQQKPGQAPRLLIYGASSRAAGIPDRFSGSGSG TDFTLTISRLEPENFAVYYCQQYESSPWTFGQGT KVEIK | QVQLQQWGAGLLKPSETLSLTCAVYGGSFSVYYW SWIRQPPGKGLEWIGESNQSGRTTYNPSLKSRVTIS VDTSKNQFSLNLISVTAADTAVYYCARDYGVFDY WGQGTLVTVSS |
| | | | SEQ ID NO: 27012 | SEQ ID NO: 31018 |
| iPS:436304 | 21-225_201F3 | NA | GATATTGTGATGACTCAGTCTCCACTCTCCCTG CCCGTCACCCCTGGAGAGCCGGCCTCCATCTC CTGCAGGTCTAGTCAGAGCCTCCTGCATAATA ATAGATACAACCATTTGGATTGGTACCTGCAG AAGCCAGGGCAGTCTCCACAGCTCCTGATCTA TTTGGGTTCTAATCGGGCCTCCGGGGTCCCTG ACAGGTTCAGTGGCAGTGGATCAGGCACAGAT TTTACACTGAAAATCAGCAGAGTGGAGGCTGA GGATGTTGGGGTTTATTACTGCATGCAAGCTC TACAAACTCCCACTTTCGGCGGAGGGACCAAG GTGGAGATCAAA | GAGGTGCAACTGTTGGAGTCTGGGGGAGGCTTG GTACAGCCTGGGGGGTCCCAGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAACTATTAGTGGTAGTGGTGTT AGAACATACTACGCAGACTCCGTGAAGGGCCGG TTCACCATCTCCAGAGACAATTCCAATAACACGC TGTTTCTGCAAATGAACAGCCTGAGAGCCGAGG ACACGGCCGTATATTACTGTGCGAAAGGGGGAG CTAGGAGCAGTGGCTCGGTTCCACTTTGACTACTG GGGCCAGGGAACCCTGGTCACCGTCTCCTCA |
| | | | SEQ ID NO: 27013 | SEQ ID NO: 31019 |
| | | AA | DIVMTQSPLSLPVTPGEPASISCRSSQSLLHNNRY NHLDWYLQKPGQSPQLLIYLGSNRASGVPDRFS GSGSGTDFTLKISRVEAEDVGVYYCMQALQTPT FGGGTKVEIK | EVQLLESGGGLVQPGGSQRLSCAASGFTFSSYAMS WVRQAPGKGLEWVSTISGSGVRTYYADSVKGRFTI SRDNSNNTLFLQMNSLRAEDTAVYYCAKGGARSS GWFHFDYWGQGTLVTVSS |
| | | | SEQ ID NO: 27014 | SEQ ID NO: 31020 |

FIGURE 50

| | | | | |
|---|---|---|---|---|
| iPS:436306 | 21-225_201H4 | NA | GAAATAGTGATGACGCAGTCTCCAGCCACCCT GTCTGTGTCTCCAGGGGAAAGAGCCACCCTCT CCTGCAGGGCCAGTCAGAGTGTTAATAGCTAC TTAGCCTGGTACCAGCAGAAACCTGGCCAGGC TCCCAGGCTCCTCATCTATGGTGCATCCACCA GGGCCACTGGTATCCCAGCCAGGTTCAGTGGC AGTGGGTCTGGGACAGAATTCACTCTCACCAT CAGCAGCCTGCAGTCTGAAGATTTTGCAGTTT ATTACTGTCAAGAGTATAATGACTGGCCGTGC AGTTTTGGCCAGGGGACCAACCTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGCCAT GCACTGGGTCCGCCAGGCTCCAGGCAAGGGGCT GGAGTGGGTGGCAGCTATATGGTATGATGGAAG TAATAAATACAATGCAGACTCCGTGAAGGGCCG ATTCACCATCTCCAGAGACAATTCCAAGAACACG CTGTATATGCAAATGAACAGCCTGAGAGCCGAG GACACGGCTGTATACTACTGTGCGAGAGATGTGG GTACAGTGGGAGCTACCTACTTTGACTGCTGGGG CCCGGGAACCCTGGTCACCGTCTCCTCA |
| | | | SEQ ID NO: 27015 | SEQ ID NO: 31021 |
| | | AA | EIVMTQSPATLSVSPGERATLSCRASQSVNSYLA WYQQKPGQAPRLLIYGASTRATGIPARFSGSGSG TEFTLTISSLQSEDFAVYYCQEYNDWPCSFGQGT NLEIK | QVQLVESGGGVVQPGRSLRLSCAASGFTFSSYAMH WVRQAPGKGLEWVAAIWYDGSNKYNADSVKGRF TISRDNSKNTLYMQMNSLRAEDTAVYYCARDVGT VGATYFDCWGPGTLVTVSS |
| | | | SEQ ID NO: 27016 | SEQ ID NO: 31022 |
| iPS:436308 | 21-225_205H8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACAAGGGAAAG CCCCTAAGCTCCTGATCTATTCTGCATCCTTTT TGCAAAGAGGGGTCCCATCAAGATTCAGCGGC AGTGGATCTGGGACAGAATTCACTCTCACAAT CAGCAGCCTGCAGCCTGAAGATTCTGCAGCTT ATTACTGTCTACAGCATAATAGTTACCCGCTC ACTTTCGGCGGAGGGACCACGGTGAAGATCA AA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTTACTTCTGG AGCTGGATCCGCCAGCCCCCAGGGAAGGGGCTG GAGTGGATTGGGGAAATCAGTCATAGTGGACGC ACCAACTACAACCCGTCCCTCAAGAGCCGAGTCA CCATATCAGTAGACACGTCCAAGAACCAGTTCTC CCTGAAGGTGAGCTCTGTGACCGCCGCGGACAC GGCTGTGTATTACTGTGCGAGGGACTACGGGGCG GACTACTGGGGCCAGGGAACCCTGGTCACCGTCT CCTCA |
| | | | SEQ ID NO: 27017 | SEQ ID NO: 31023 |

FIGURE 50

| | | | | |
|---|---|---|---|---|
| | | AA | DIQMTQSPSSLSASVGDRVTITCRASQGIRNDLG WYQQKQGKAPKLLIYSASFLQRGVPSRFSGSGS GTEFTLTISSLQPEDSAAYYCLQHNSYPLTFGGG TTVKIK | QVQLQQWGAGLLKPSETLSLTCAVYGGSFSGYFW SWIRQPPGKGLEWIGEISHSGRTNYNPSLKSRVTISV DTSKNQFSLKVSSVTAADTAVYYCARDYGADYW GQGTLVTVSS |
| | | | SEQ ID NO: 27018 | SEQ ID NO: 31024 |
| IPS:436310 | 21-225_202D11 | NA | GAAATTGTGTTGACGCAGTCTCCAGGCACCCT GTCTTTGTCTCCAGGGGAAAGAGCCACCCTCT CCTGCAGGGCCAGTCAGAGTATTAACAGCAAC TACTTAGCCTGGTACCAGCGGAAGCCTGGCCA GGCTCCCAGGGTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTTTTACTGTCAGCAGTATGAAAACTCACCG TGGACGTTCGGCCAAGGGACCAAGGTGGAAA TCAAA | CAGGTGCAGCTACAGCAGCGGGGCGCAGGACTG TTGAAGCCTTCGGAGACCCTGTCCCTCACCTGCG CTGCCTATGGTGGGTCCTTCAGTGGTCCCTACTG GAGCTGGATCCGCCAGCCCCCAGGGAAGGGCCT GGAGTGGATTGGGGAAATCAATCATAGTGGAAG CACCAACTACAACCCGTCCCTCAAGAGTCGAGTC ACCATATCAGTAGACACGTCCAAGAACCAGTTCT CCCTGAAGCTGAGCTCTGTGACCGCCGCGGACAC GGCTGTTTACTACTGTGCGAGGGACTACGGTGTC CTTGACTACTGGGGCCAGGGAACCCTGGTCACCG TCTCCTCA |
| | | | SEQ ID NO: 27019 | SEQ ID NO: 31025 |
| | | AA | EIVLTQSPGTLSLSPGERATLSCRASQSINSNYLA WYQRKPGQAPRVLIYGASSRATGIPDRFSGSGSG TDFTLTISRLEPEDFAVFYCQQYENSPWTFGQGT KVEIK | QVQLQQRGAGLLKPSETLSLTCAAYGGSFSGPYWS WIRQPPGKGLEWIGEINHSGSTNYNPSLKSRVTISV DTSKNQFSLKLSSVTAADTAVYYCARDYGVLDYW GQGTLVTVSS |
| | | | SEQ ID NO: 27020 | SEQ ID NO: 31026 |

FIGURE 50

| iPS:436312 | 21-225_206A4 | NA | GACATCCAGATGACCCTGTCTCCATCCTCCCC GTCTGCATTTGTTGGAGACAGAGTCACCATCA CTCGCCGGGCAAGTCACAACATTAACAGCTAT TTAAATTGGTATCAGCAGAAATCAGGGAAAGC CCCTAAACTCCTTATCTGTGCTGCATCCAGTTT GCAAAGTGGGGTCCCGTCAAGGTTCAGTGGCA GTGGATCTGGGACAGATTTCACTCTCACTTTC AGTAGTCTACAACCTGAAGATTTTGCAACTTA CTACTGTCAACAGAGTTACAGTTTCCCGCTCA CTTTCGGCGGAGGGACCAAGGTGGAGATGAG G | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TCCACTGGGTGCGACAGGCCCCTGGACAAGGGC TTGAGTGGATGGCATGGATCAACCCTAATAGTGG TGGCACAAACTATGCACAGAAGTTTCAGGGCAG GGTCACCATGACCAGGGACACGTCCATCACCAC AGCCTACATGGAGCTGAGCAGGCTGAGATCTGA CGACACGGCCGTGTATTACTGTGCGAGAGGATAC AGCTATGGTTACAACTGGTTCGACCCCTGGGGCC AGGGAACCCTGGTCACCGTCTCCTCA |
| | | | SEQ ID NO: 27021 | SEQ ID NO: 31027 |
| | | AA | DIQMTLSPSSPSAFVGDRVTITRRASHNINSYLN WYQQKSGKAPKLLICAASSLQSGVPSRFSGSGSG TDFTLTFSSLQPEDFATYYCQQSYSFPLTFGGGT KVEMR | QVQLVQSGAEVKKPGASVKVSCKASGYTFTGYYIH WVRQAPGQGLEWMAWINPNSGGTNYAQKFQGRV TMTRDTSITTAYMELSRLRSDDTAVYYCARGYSYG YNWFDPWGQGTLVTVSS |
| | | | SEQ ID NO: 27022 | SEQ ID NO: 31028 |
| iPS:436314 | 21-225_206G4 | NA | GAAATTGTGCTGACTCAGTCTCCAGACTTTCA GTCTGTGACTCCAAAGGAGAAAGTCACCATCA CCTGCCGGGCCAGTCAGAGCATTGGTCGTAGC TTACACTGGTACCAGCAGAAACCAGATCAGTC TCCAAAACTCCTCATCAAGTATGCTTCCCAGT CCTTCTCAGGGGTCCCCTCGAGGTTCAGTGGC AGTGGATCTGGGACAGATTTCACCCTCACCAT CAATAGCCTGGAAGCTGAAGATGCTGCAACGT ATTACTGTCATCAGAGTAGAAGTTTACCGCTC ACTTTCGGCGGAGGGACCAAGGTGGAGATCA AA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCGACCCTAATAGTGG TGGCACAAACTATGCACAGAAGTTTCAGGGCAG AATCACCATGACCAGGGACACGTCCATCAGTAC AGCCTACATGGAACTGAGCAGGCTGAGATCTGA CGACACGGCCGTGTTTTACTGTGCGAAAGATCAA GGGTATAACTGGAACTCTTTTGACTACTGGGGCC AGGGAACCCTGGTCACCGTCTCCTCA |
| | | | SEQ ID NO: 27023 | SEQ ID NO: 31029 |

FIGURE 50

| | | | | |
|---|---|---|---|---|
| | | AA | EIVLTQSPDFQSVTPKEKVTITCRASQSIGRSLHW YQQKPDQSPKLLIKYASQSFSGVPSRFSGSGSGT DFTLTINSLEAEDAATYYCHQSRSLPLTFGGGTK VEIK | QVQLVQSGAEVKKPGASVKVSCKASGYTFTGYYIH WVRQAPGQGLEWMGWIDPNSGGTNYAQKFQGRI TMTRDTSISTAYMELSRLRSDDTAVFYCAKDQGYN WNSFDYWGQGTLVTVSS |
| | | | SEQ ID NO: 27024 | SEQ ID NO: 31030 |
| iPS:436316 | 21-225_206A5 | NA | GACATCCAGATGACCCAGTCTCCATCCTCCCC GTCTGCATCTGTTGGAGACAGAGTCACCATCA CTCGCCGGGCAAGTCACAACATTAACAGCTAT TTAAATTGGTATCAGCAGAAATCAGGGAAAGC CCCTAAACTCCTTATCTGTGCTGCATCCAGTTT GCAAAGTGGGGTCCCGTCAAGGTTCAGTGGCA GTGGATCTGGGACAGATTTCACTCTCACTTTC AGTAGTCTACAACCTGAAGATTTTGCAACTTA CTACTGTCAACAGAGTTACAGTTTCCCGCTCA CTTTCGGCGGAGGGACCAAGGTGGAGATGAG G | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TCCACTGGGTGCGACAGGCCCCTGGACAAGGGC TTGAGTGGATGGCATGGATCAACCCTAATAGTGG TGGCACAAACTATGCACAGAAGTTTCAGGGCAG GGTCACCATGACCAGGGACACGTCCATCACCAC AGCCTACATGGAGCTGAGCAGGCTGAGATCTGA CGACACGGCCGTGTATTACTGTGCGAGAGGATAC AGCTATGGTTACAACTGGTTCGACCCCTGGGGCC AGGGAACCCTGGTCACCGTCTCCTCA |
| | | | SEQ ID NO: 27025 | SEQ ID NO: 31031 |
| | | AA | DIQMTQSPSSPSASVGDRVTITRRASHNINSYLN WYQQKSGKAPKLLICAASSLQSGVPSRFSGSGSG TDFTLTFSSLQPEDFATYYCQQSYSFPLTFGGGT KVEMR | QVQLVQSGAEVKKPGASVKVSCKASGYTFTGYYIH WVRQAPGQGLEWMAWINPNSGGTNYAQKFQGRV TMTRDTSITTAYMELSRLRSDDTAVYYCARGYSYG YNWFDPWGQGTLVTVSS |
| | | | SEQ ID NO: 27026 | SEQ ID NO: 31032 |

2369

FIGURE 50

| iPS:436324 | 21-225_207G6 | NA | GATATCCAGATGACCCAGTCTCCATCCTCACT GTCTGCATCTGTAGGAGACAGAGTCACCATCA CTTGTCGGGCGAGTCAGGGCATTAGAAATTAT TTAGCCTGGCTTCAGCAGAAACCAGGGAAGGC CCCTAAGTCCCTGATCCATGCTGCATCCAGTTT GCAAAGTGGGGTCCCATCAAAGTTCAGCGGCA ATAGATCTGGGACAGATTTCACTCTCACCATC AGCAGCCTGCAGCCTGAAGATTTTGCAACTTA TTACTGCCAACAGTACAGTAATTACCCTCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGAGTCCGTGAAGGGCCGA TTCACCATCTCCAGAGACAATTCCAAGAACACGC TGTATCTGCAAATGAACAGCCTGAGAGCCGAGG ACACGGCTGTGTATTATTGTGCGAGAGATGCGGG TATTGGATACTACGGTATAGACGTCTGGGGCCAA GGGACCACGGTCACCGTCTCCTCA |
|  |  |  | SEQ ID NO: 27027 | SEQ ID NO: 31033 |
|  |  | AA | DIQMTQSPSSLSASVGDRVTITCRASQGIRNYLA WLQQKPGKAPKSLIHAASSLQSGVPSKFSGNRS GTDFTLTISSLQPEDFATYYCQQYSNYPLTFGGG TKVEIK | QVQLVESGGGVVQPGRSLRLSCAASGFTFSSYGMH WVRQAPGKGLEWVAVIWYDGSNKNYAESVKGRF TISRDNSKNTLYLQMNSLRAEDTAVYYCARDAGIG YYGIDVWGQGTTVTVSS |
|  |  |  | SEQ ID NO: 27028 | SEQ ID NO: 31034 |
| iPS:436328 | 21-225_207F12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTTCTGTCTACAGCATAATAGTTACCCTCTC ACCTTCGGCCAAGGGACACGACTGGAAATTAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATAGAAAT AATAAATACTATGGTGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCTCTTTGACTACTGGGGCCAGGGAACCCTGG TCACCGTCTCCTCA |
|  |  |  | SEQ ID NO: 27029 | SEQ ID NO: 31035 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYFCLQHNSYPLTFGQGT RLEIK | QVQLVESGGGVVQPGRSLRLSCAASGFTFSNYGM HWVRQAPGKGLEWVAVIWYDRNNKYYGDSVKG RFTISRDNSKNTLYLQMNSLRAEDTAVYYCARELG FLFDYWGQGTLVTVSS |
| | | | SEQ ID NO: 27030 | SEQ ID NO: 31036 |
| iPS:436332 | 21-225_208B2 | NA | GACATCCAGATGACCCAGTCTCCATCCTCCCT GTCTGCATCTGTAGGAGACAGAGTCACCATCA CTTGCCGGGCAAGTCAGGGCATTAGACATGAT TTAGGCTGGTATCAACAGAAACCAGGGAAAG CCCCTAAGCGCCTGATCTATGCTGCATCCAGT TTGCAAAGTGGGGTCCCATCAGGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTGTGTCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAGGAACACGCT GTATCTGCAAATGAACAGCCTGAGAGCCGAGGA CACGGCTGTGTATTACTGTGCGAGAGAACGGTAT AGCAGTGGCTTGTACGACTACGGTTTGGACGTCT GGGGCCAAGGGACCACGGTCACCGTCTCCTCA |
| | | | SEQ ID NO: 27031 | SEQ ID NO: 31037 |
| | | AA | DIQMTQSPSSLSASVGDRVTITCRASQGIRHDLG WYQQKPGKAPKRLIYAASSLQSGVPSGFSGSGS GTEFTLTISSLQPEDFATYYCLQHSYPRTFGQG TKVEIK | QVQLVESGGGVVQPGRSLRLSCAASGFTFSDCVMH WVRQAPGKGLEWVAVIWYDGSNKYYVDSVKGRF TISRDNSRNTLYLQMNSLRAEDTAVYYCARERYSS GLYDYGLDVWGQGTTVTVSS |
| | | | SEQ ID NO: 27032 | SEQ ID NO: 31038 |

FIGURE 50

| iPS:436334 | 21-225_208G3 | NA | GATATTGTGCTGACTCAGTCTCCACTCTCCCTG CCCGTCACCCCTGGAGAGCCGGCCTCCATCTC CTGCAGGTCTAGTCAGAGCCTCCTGCATAATA ATAAATACAACCATTTGGATTGGTACCTGCAG AAGCCAGGGCAGTCTCCACAGCTCCTGATCTA TTTGGGTTCTAATCGGGCCTCCGGGGTCCCTG ACAGGTTCAGTGGCAGTGGATCAGGCACAGAT TTTACACTGAAAATCAGCAGAGTGGAGGCTGA GGATGTTGGGGTTTATTACTGCATGCAAGCTC TACAAACTCCCACTTTCGGCGGAGGGACCAAG GTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAACTATTAGTGGTAGTGGTGTT AGAACATACTACGCAGACTCCGTGAAGGGCCGG TCCACCATCTCCAGAGACAATTCCAAGAATACGC TGTTTCTGCAAATGAACAGCCTGAGAGCCGAGG ACAAGGCCGTATATTACTGTGCGAAAGGGGGAG CTAGGAGCAGTGGCTGGTTCCACTTTGACTACTG GGGCCAGGGAACCCTGGTCACCGTCTCCTCA |
| | | | SEQ ID NO: 27033 | SEQ ID NO: 31039 |
| | | AA | DIVLTQSPLSLPVTPGEPASISCRSSQSLLHNNKY NHLDWYLQKPGQSPQLLIYLGSNRASGVPDRFS GSGSGTDFTLKISRVEAEDVGVYYCMQALQTPT FGGGTKVEIK | EVQLLESGGGLVQPGGSLRLSCAASGFTFSSYAMS WVRQAPGKGLEWVSTISGSGVRTYYADSVKGRSTI SRDNSKNTLFLQMNSLRAEDKAVYYCAKGGARSS GWFHFDYWGQGTLVTVSS |
| | | | SEQ ID NO: 27034 | SEQ ID NO: 31040 |
| iPS:436336 | 21-225_208B5 | NA | GAAATTGTTTTGACGCAGTCTCCAGGCACCCT GTCTTTGTCTCCAGGGGAAAGAGCCACCCTCT CCTGCAGGGCCAGTCAGAGTGTTAGCAGCAAC TACTTAGCCTGGTATCAGCAGAGACCTGGCCA GGCTCCCAGACTCCTCATCTATGGTGCATCCA GCAGGGCCACTGGCATCCCAGACAGATTCAGT GGCAGTGGGTCTGGGACAGACTTCACTCTCAC CATCAGCAGACTGGAGCCTGAAGATTTTGCAG TGTATTTCTGTCAGCACTACGAAAACTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCTTTGGTGGGTCCATCAGTGTTTACTACTGG ACCTGGATCCGCCAGCCCCCAGGGAAGGGGCTG GAGTGGATTGGGGAAATCAATCATAGTGGAAGC ACCAACTACAACCCGTCCCTCAAGAGTCGAGTCA CCATATCAGTAGACACGTCCAAGAACCAGTTCTC CCTGAAGCTGAGCTCTGTGACCGCCGCGGACACG GCTGTGTATTACTGTGCGAGGGACTATGGTGTCT TTGATTACTGGGGCCAGGGAACCCTGGTCACCGT CTCCTCA |
| | | | SEQ ID NO: 27035 | SEQ ID NO: 31041 |

FIGURE 50

| | | | | |
|---|---|---|---|---|
| | | AA | EIVLTQSPGTLSLSPGERATLSCRASQSVSSNYLA WYQQRPGQAPRLLIYGASSRATGIPDRFSGSGSG TDFTLTISRLEPEDFAVYFCQHYENSPWTFGQGT KVEIK | QVQLQQWGAGLLKPSETLSLTCAVFGGSISVYYWT WIRQPPGKGLEWIGEINHSGSTNYNPSLKSRVTISV DTSKNQFSLKLSSVTAADTAVYYCARDYGVFDYW GQGTLVTVSS |
| | | | SEQ ID NO: 27036 | SEQ ID NO: 31042 |
| iPS:436338 | 21-225_208E8 | NA | GACATCCAGATGACCCAGTCTCCATCCTCCCC GTCTGCATTTGTTGGAGACAGAGTCACCATCA CTCGCCGGGCAAGTCACAACATTAACAGCTAT TTAAAATTGGTATCAGCAGAAATCAGGGAAAGC CCCTAAACTCCTTATCTATGCTGCATCCAGTTT GCAAAGTGGGGTCCCGTCAAGGTTCAGTGGCA GTGGATCTGGGACAGATTTCACTCTCACTTTC AGTAGTCTACAACCTGAAGATTTTGCAACTTA CTACTGTCAACAGAGTTACAGTTTCCCGCTCA CTTTCGGCGGAGGGACCAAGGTGGAGATGAG G | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TCCACTGGGTGCGACAGGCCCCTGGACAAGGGC TTGAGTGGATGGCATGGATCAACCCTAATAGTGG TGGCACAAACTATGCACAGAAGTTTCAGGGCAG GGTCACCATGACCAGGGACACGTCCATCACCAC AGCCTACATGGAGCTGAGCAGGCTGAGATCTGA CGACACGGCCGTGTATTACTGTGCGAGAGGATAC AGCTATGGTTACAACTGGTTCGACCCCTGGGGCC AGGGAACCCTGGTCACCGTCTCCTCA |
| | | | SEQ ID NO: 27037 | SEQ ID NO: 31043 |
| | | AA | DIQMTQSPSSPSAFVGDRVTITRRASHNINSYLN WYQQKSGKAPKLLIYAASSLQSGVPSRFSGSGS GTDFTLTFSSLQPEDFATYYCQQSYSFPLTFGGG TKVEMR | QVQLVQSGAEVKKPGASVKVSCKASGYTFTGYYIH WVRQAPGQGLEWMAWINPNSGGTNYAQKFQGRV TMTRDTSITTAYMELSRLRSDDTAVYYCARGYSYG YNWFDPWGQGTLVTVSS |
| | | | SEQ ID NO: 27038 | SEQ ID NO: 31044 |

FIGURE 50

| iPS:436340 | 21-225_208A9 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAACAAC TACTTAGCCTGGTACCAGCAGAAACCTGGCCA GGCTCCCAGGGTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCACTATCATAGCTCACCGT GGACGTTCGGCCAAGGGACCAAGGTGGAAAT CAAA | CAGGTGCAGCTACAGCAGTGGGGCGCAGGACTG TTGAAGCCTTCGGAGCCCCTGTCCCTCACCTGCG CTGTCTATGGTGGGTCCTTCAGTGTTTCCTACTGG AGCTGGATCCGCCAGCCCCCAGGGAAGGGGCTG GAGTGGATTGGGGAAATCAATCATAGTGGACGC GCCAACTACAACCCGTCCCTCAAGAGTCGAGTCA CCATATCAATAGACACGTCCAAGAACCAGTTCTC CCTGAAGCTGAGCTCTGTGACCGCCGCGGACACG GCTGTGTATTACTGTGCGAGGGACTACGGTGTCC TTGACTACTGGGGCCAGGGAACCCTGGTCACCGT CTCCTCA |
| | | | SEQ ID NO: 27039 | SEQ ID NO: 31045 |
| | | AA | EIVLTQSPGTLSLSPGERATLSCRASQSVSNNYLA WYQQKPGQAPRVLIYGASSRATGIPDRFSGSGSG TDFTLTISRLEPEDFAVYYCQHYHSSPWTFGQGT KVEIK | QVQLQQWGAGLLKPSEPLSLTCAVYGGSFSVSYW SWIRQPPGKGLEWIGEINHSGRANYNPSLKSRVTISI DTSKNQFSLKLSSVTAADTAVYYCARDYGVLDYW GQGTLVTVSS |
| | | | SEQ ID NO: 27040 | SEQ ID NO: 31046 |
| iPS:436344 | 21-225_208B11 | NA | GACATCCAGATGACCCAGTCTCCATCCTCCCC GTCTGCATCTGTTGGAGACAGAGTCACCATCA CTTGCCGGGCAAGTCACAACATTAACAGCTAT TTAAATTGGTATCAGCAGAAATCAGGGAAAGC CCCTAAACTCCTTATCTATGCTGCATCCAGTTT GCAAAGTGGGGTCCCGTCAAGGTTCAGTGGCA GTGGATCTGGGACAGATTTCACTCTCACTTTC AGTAGTCTACAACCTGAAGATTTTGCAACTTA CTACTGTCAACAGAGTTACAGTTTCCCGCTCA CTTTCGGCGGAGGGACCAAGGTGGAGATGAG G | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TCCACTGGGTGCGACAGGCCCCTGGACAAGGGC TTGAGTGGATGGCATGGATCAACCCTAATAGTGG TGGCACAAACTATGCACAGAAGTTTCAGGGCAG GGTCACCATGACCAGGGACACGTCCATCACCAC AGCCTACATGGAGCTGAGCAGGCTGAGATCTGA CGACACGGCCGTGTATTACTGTGCGAGAGGATAC AGCTATGGTTACAACTGGTTCGACCCCTGGGGCC AGGGAACCCTGGTCACCGTCTCCTCA |
| | | | SEQ ID NO: 27041 | SEQ ID NO: 31047 |

FIGURE 50

| | | | | |
|---|---|---|---|---|
| | | AA | DIQMTQSPSSPSASVGDRVTITCRASHNINSYLN WYQQKSGKAPKLLIYAASSLQSGVPSRFSGSGS GTDFTLTFSSLQPEDFATYYCQQSYSFPLTFGGG TKVEMR | QVQLVQSGAEVKKPGASVKVSCKASGYTFTGYYIH WVRQAPGQGLEWMAWINPNSGGTNYAQKFQGRV TMTRDTSITTAYMELSRLRSDDTAVYYCARGYSYG YNWFDPWGQGTLVTVSS |
| | | | SEQ ID NO: 27042 | SEQ ID NO: 31048 |
| iPS:436350 | 21-225_210E4 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCCTCATTAACTATGGCAT GCACTGGGTCCGCCAGGCTCCAGGCAAGGGGCT GGAGTGGGTGGCAGTTATATGGTATGATGAAAAT AATAAATACTATGTAGACTCCGTGAAGGGCCGAT TCACCATCTCCAGAGACGATTCCAAGAACACGCT GTATCTGCAAATGAACAGCCTGAGAGCCGAGGA CTCGGCTGTGTATTACTGTGCGAGAGAGACGGGT TTCTTGAGCGACTACTGGGGCCAGGGAACCCTGG TCACCGTCTCCTCA |
| | | | SEQ ID NO: 27043 | SEQ ID NO: 31049 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTLINYGMH WVRQAPGKGLEWVAVIWYDENNKYYVDSVKGRF TISRDDSKNTLYLQMNSLRAEDSAVYYCARETGFL SDYWGQGTLVTVSS |
| | | | SEQ ID NO: 27044 | SEQ ID NO: 31050 |

FIGURE 50

| iPS:436352 | 21-225_210G5 | NA | GACATCCAGATGACCCAGTCTCCATCCTCCCT GTCTGCATCTGTAGGAGACAGAGTCACCATCA CTTGCCGGGCAAGTCAGGGCATTAGACATGAT TTAGGCTGGTATCAACAGAAACCAGGGAAAG CCCCTAAGCGCCTGATCTATGCTGCATCCAGT TTGCAAAGTGGGGTCCCATCAGGGTTCAGCGG CAGTGGATCTGGGACAGAATTCACTCTCACAA TCAGCAGCCTGCAGCCTGAAGATTTTGCAACT TATTACTGTCTACACCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTGTGTCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAGGAACACGCT GTATCTGCAAATGAACAGCCTGAGAGCCGAGGA CACGGCTGTGTATTACTGTGCGAGAGAACGGTAT AGCAGTGGCTTGTACGACTACGGTTTGGACGTCT GGGGCCAAGGGACCACGGTCACCGTCTCCTCA |
| | | | SEQ ID NO: 27045 | SEQ ID NO: 31051 |
| | | AA | DIQMTQSPSSLSASVGDRVTITCRASQGIRHDLG WYQQKPGKAPKRLIYAASSLQSGVPSGFSGSGS GTEFTLTISSLQPEDFATYYCLHHYSYPRTFGQG TKVEIK | QVQLVESGGGVVQPGRSLRLSCAASGFTFSDCVMH WVRQAPGKGLEWVAVIWYDGSNKYYVDSVKGRF TISRDNSRNTLYLQMNSLRAEDTAVYYCARERYSS GLYDYGLDVWGQGTTVTVSS |
| | | | SEQ ID NO: 27046 | SEQ ID NO: 31052 |
| iPS:436354 | 21-225_210G10 | NA | GACATCCAGATGACCCAGTCTCCATCCTCCCT GTCTGCATCTGTAGGAGACAGAGTCACCATCA CTTTCCGGACAAGTCAGGGCATTAGAAATGAT TTAGGCTGGTATCAGCAGCAACCAGGGAAAA CCCCTAAGCGCATGATTTATGCTGCATCCAGT TTGTTTAGTGGGGTCCCATCAAGGTTCAGCGG CAGTAGATCTGGGACAGATTTCACTCTCACAA TCAGCAGCCTGCAGCCTGAAGATTTTGCAACT TATTACTGTCTACAGTATAATAGTTACCCTCCC ACCTTCGGCCAAGGGACACGACTGGAGATTAA A | CAGGTGCAGCTGCAGGAGTCGGGCCCAGGACTG GTGAAGCCTTCGGAGACCCTGTCCCTCAACTGCA CTGTCTCTGGTGGCTCCATCAGGAGTTACTACTG GAGCTGGATCCGGCAGCCCGCCGGGAAGGGACT GGAGTGGATTGGGCGTATCTATACCAGTGGGAGC ACCGACTACAACCCCTCCCTCAAGAGTCGAATCA CCATGTCAGTAGACACGTCCAAGAACCAGTTCTC TTTGAAGCTGAGCTCTGTGACCGCCGCGGACACG GCCGTGTATTACTGTGCGAGAGGGGTTCGGTGACT GGGACTACTGGGGCCAGGGAACCCTGGTCACCG TCTCCTCA |
| | | | SEQ ID NO: 27047 | SEQ ID NO: 31053 |

FIGURE 50

| | | | | |
|---|---|---|---|---|
| | | AA | DIQMTQSPSSLSASVGDRVTITFRTSQGIRNDLG WYQQQPGKTPKRMIYAASSLFSGVPSRFSGSRS GTDFTLTISSLQPEDFATYYCLQYNSYPPTFGQG TRLEIK | QVQLQESGPGLVKPSETLSLNCTVSGGSIRSYYWS WIRQPAGKGLEWIGRIYTSGSTDYNPSLKSRITMSV DTSKNQFSLKLSSVTAADTAVYYCARGFGDWDYW GQGTLVTVSS |
| | | | SEQ ID NO: 27048 | SEQ ID NO: 31054 |
| iPS:436356 | 21-225_210H10 | NA | GAAATAGTGATGACGCAGTCTCCAGCCACCCT GTCTGTGTCTCCAGGGGAAAGAGCCACCCTCT CCTGCAGGGCCAGTCAGAGTGTTAAAAGCAAC TTAGCCTGGTACCAGCAGAAACCTGGCCAGGC TCCCAGGCTCCTCATCTATGGTGCATCCACCA GGGCCACTGGTATCCCAGCCAGGTTCAGTGGC AGTGGGTCTGGGACAGAGTTCACTCTCACCAT CAGCAGCCTGCAGTCTGAAGATTTTGCAGTTT ATTCCTGTCAGCAGTATTATAACTGGCTGTGC AGTTTTGGCCAGGGGACCAAGCTGGAGATCAA A | CAGGTACAGCTGCAGCAGTCAGGTCCAGGACTG GTGAAGCCCTCGCAGACCCTCTCACTCACCTGTG CCATCTCCGGGGACAGTGTCTCTAGCAACAGTGC TGCTTGGAACTGGATCAGGCAGTCCCCATCGAGA GGCCTTGAGTGGCTGGGAAGGACATACTACAGG TCCAAGTGGTATAATTATTATCCAGTATCTGTGA GAAGTCGAATAACCATCAACCCAGACACATCCA AGAACCAGTTCTCCCTGCTGCTGAACTCTGTGAC TCCCGAGGACACGGCTGTGTATTACTGTGCAAGA GATCAACGGTACTACGGTATGGACGTCTGGGGCC AAGGGACCACGGTCACCGTCTCCTCA |
| | | | SEQ ID NO: 27049 | SEQ ID NO: 31055 |
| | | AA | EIVMTQSPATLSVSPGERATLSCRASQSVKSNLA WYQQKPGQAPRLLIYGASTRATGIPARFSGSGSG TEFTLTISSLQSEDFAVYSCQQYYNWLCSFGQGT KLEIK | QVQLQQSGPGLVKPSQTLSLTCAISGDSVSSNSAA WNWIRQSPSRGLEWLGRTYYRSKWYNYYPVSVRS RITINPDTSKNQFSLLLNSVTPEDTAVYYCARDQRY YGMDVWGQGTTVTVSS |
| | | | SEQ ID NO: 27050 | SEQ ID NO: 31056 |

FIGURE 50

| iPS:436358 | 21-225_210D11 | NA | GACATCCAGATGACCCAGTCTCCATCCTCCCT GTCTGCATCTGTTGGAGACAGAGTCACCATCA CTTGCCGGGCAAGTCACAACATTAACAGCTAT TTAAATTGGTATCAGCAGAAATCAGGGAAAGC CCCTAAACTCCTAATCTATGCTGCATCCAGTTT GCAAAGTGGGGTCCCGTCAAGGTTCAGTGGCA GTGGATCTGGGACAGATTTCACTCTCACCTTC AGTAGTCTACAACCTGAAGATTTTGCAACTTA CTACTGTCAACAGAGTTACAGTTTCCCGCTCA CTTTCGGCGGAGGGACCAAGGTGGAGATGAG G | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TCCACTGGGTGCGACAGGCCCCTGGACAAGGGC TTGAGTGGATGGCATGGATCAACCCTAATAGTGG TGGCACAAACTATGCACAGAAGTTTCAGGGCAG GGTCACCATGACCAGGGACACGTCCATCACCAC AGCCTACATGGAGCTGAGCAGGCTGAGATCTGA CGACACGGCCGTGTATTACTGTGCGAGAGGATAC AGCTATGGTTACAACTGGTTCGACCCCTGGGGCC AGGGAACCCTGGTCACCGTCTCCTCA |
| | | | SEQ ID NO: 27051 | SEQ ID NO: 31057 |
| | | AA | DIQMTQSPSSLSASVGDRVTITCRASHNINSYLN WYQQKSGKAPKLLIYAASSLQSGVPSRFSGSGS GTDFTLTFSSLQPEDFATYYCQQSYSFPLTFGGG TKVEMR | QVQLVQSGAEVKKPGASVKVSCKASGYTFTGYYIH WVRQAPGQGLEWMAWINPNSGGTNYAQKFQGRV TMTRDTSITTAYMELSRLRSDDTAVYYCARGYSYG YNWFDPWGQGTLVTVSS |
| | | | SEQ ID NO: 27052 | SEQ ID NO: 31058 |
| iPS:436360 | 21-225_210H11 | NA | GACATCCAGATGACCCAGTCTCCATCTTCCGT GTCTGCTTCTGTAGGAGACACAGAGTCACCATCA CTTGTCGGGCGAGTCAGGGTATTAGCATCTGG TTAGCCTGGTATCAGCAGAAACCAGGGAAAG CCCCTAACCTCCTGATCTATGCTGCATCCAGTT TGCAAAGTGGAGTCCCATCAAGGTTCAGCGGC AGAGGGATCTGGGACAGATTTCACTCTCACCAT CAGCAGCCTGCAGCCTGAAGATTTTGCAACTT ACTATTGTCAACAGGCTAAAAGTTTCCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCCATGGCAT GCACTGGGTCCGCCAGGCTCCAGGCAAGGGGCT GGAGTGGGTGGCAGTTACATGGTATGATGGAAG TGATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTATATTACTGTGCGCGAGACCGGC TAGTGGGAGCTACTACCGATGCTTTTGATATCTG GGGCCAAGGGACAATGGTCACCGTCTCTTCA |
| | | | SEQ ID NO: 27053 | SEQ ID NO: 31059 |

FIGURE 50

| | | | | |
|---|---|---|---|---|
| | | AA | DIQMTQSPSSVSASVGDRVTITCRASQGISIWLA WYQQKPGKAPNLLIYAASSLQSGVPSRFSGRGS GTDFTLTISSLQPEDFATYYCQQAKSFPFTFGPGT KVDIK | QVQLVESGGGVVQPGRSLRLSCAASGFTFSSHGMH WVRQAPGKGLEWVAVTWYDGSDKYYADSVKGR FTISRDNSKNTLYLQMNSLRAEDTAVYYCARDRLV GATTDAFDIWGQGTMVTVSS |
| | | | SEQ ID NO: 27054 | SEQ ID NO: 31060 |
| iPS:436362 | 21-225_210C12 | NA | GATATTGTGATGACTCAGTCTCCACTCTCCCTG CCCGTCACCCCTGGAGAGCCGGCCTCCATCTC CTGCAGGTCTAGTCAGAGCCTCCTGCATTATA ATGGACACAACTTTTTGGATTGGTACCTGCAG AAGCCAGGGCAGTCTCCACAGCTCCTGATCTA TTTGGTTTCTAATCGGGCCTCCGGGGTCCCTGA CAGGTTCAGTGGCAGTGGATCAGGCACAGATT TTACACTGAAAATCAGCAGAGTGGAGGCTGA GGATGTTGGGGTTTATTACTGCATGCAAGCTC TACAAACTCCCATGTGCAGTTTTGGCCAGGGG ACCAAGTTGGAGATCAAA | CAGGTTCAGCTGGTGCAGTCTGGAGCTGAGGTGA CGAAGCCTGGGGCCTCAGTGAAGGTCTCCTGCAA GGCTTCTGGTTACACCTTTACCAACAATGGTATC AGCTGGGTGCGACAGGCCCCTGGACAAGGGCTT GAGTGGATGGGATGGATCAACGCTTACAATGGTC ACACAAACTATGCACAGAAGTTCCAGGGCAGAG TCACCATGACCACAGACACATCCACGAGCACAG CCTACATGGAGCTGAGGAGCCTGAGATCTGACG ACACGGCCGTGTATTACTGTGCGAGAGATCCTAC GGTGACCCACTACTATTACTACGGTATGGACGTC TGGGGCCAAGGGACCACGGTCACCGTCTCCTCA |
| | | | SEQ ID NO: 27055 | SEQ ID NO: 31061 |
| | | AA | DIVMTQSPLSLPVTPGEPASISCRSSQSLLHYNGH NFLDWYLQKPGQSPQLLIYLVSNRASGVPDRFS GSGSGTDFTLKISRVEAEDVGVYYCMQALQTPM CSFGQGTKLEIK | QVQLVQSGAEVTKPGASVKVSCKASGYTFTNNGIS WVRQAPGQGLEWMGWINAYNGHTNYAQKFQGR VTMTTDTSTSTAYMELRSLRSDDTAVYYCARDPTV THYYYYGMDVWGQGTTVTVSS |
| | | | SEQ ID NO: 27056 | SEQ ID NO: 31062 |

FIGURE 50

| iPS:436364 | 21-225_211A11 | NA | GACATCCAGATGACCCAGTCTCCATCCTCACT GTCTGCATCTGTAGGAGACAGAGTCACCATCA CTTGTCGGGCGAGTCAGGGCATTGGCAATTAT TTAGCCTGGTTTCAGCAGAAACCAGGGAAAGC CCCTAGGTCCCTGATCTATGATGCATCCAGTTT GGAAAGTGGGGTCCCATCAAAGTTCAGCGGC AGTAGGTCTGGGACAGATTTCACTCTCACCAT CGGCAGCCTGCAGCCTGAAGATTTTGCAACTT ATTACTGCCAACACTATATGACTTACCCGCTC ACTTTCGGCGCAGGGACCAAGGTGGAGATCA AA | CAGGTGCAGCTGGTGGGGGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAATGGGTGGCAGTTCTTTGGTTTGATGGAAGT AATAGAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGG AGTGGGCTACTACGGTACGGACGTCTGGGGCCA AGGGACCACGGTCACCGTCTCCTCA |
| | | | SEQ ID NO: 27057 | SEQ ID NO: 31063 |
| | | AA | DIQMTQSPSSLSASVGDRVTITCRASQGIGNYLA WFQQKPGKAPRSLIYDASSLESGVPSKFSGSRSG TDFTLTIGSLQPEDFATYYCQHYMTYPLTFGAGT KVEIK | QVQLVGSGGGVVQPGRSLRLSCAASGFTFSSYGMH WVRQAPGKGLEWVAVLWFDGSNRNYADSVKGRF TISRDNSKNTLYLQMNSLRAEDTAVYYCARDRGV GYYGTDVWGQGTTVTVSS |
| | | | SEQ ID NO: 27058 | SEQ ID NO: 31064 |
| iPS:436366 | 21-225_211A3 | NA | GACATCCGGATGACCCAGTCTCCATCCTCACT GTCTGCATCTGTAGGAGACAGAGTCACCATCA CTTGTCGGGCGAGTCAGGCCATTGGGAAACAT TTAGCCTGGTTTCAGCAGAGGCCTGGGAAAGC CCCTAAGTCCCTGATCTATGCTGCATCCAGATT GCAAAGTGGGGTCCCATCAAAGTTCAGCGGCA GTGGATCTGGGACAGATTTCACTCTCACCATC AGCAGCCTGCAGCCTGAAGATCTTGCAACTTA TTACTGTCAACAGTATAGTAATTATCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGTGTCTGGAATCACCTTCAGTAGTTATGGCAT GCACTGGGCCCGCCAGGCTCCAGGCAAGGGGCT GGAGTGGGTGGCAGTTATATGGTATGATGGAAGT AATAAAAACTATGAAGACTCCGTGAAGGGCCGA TTCACCATCTCCAGAGACAACTCCAAGAACACGC TGTATCTGCAAATGAACAGCCTGAGAGCCGAGG ACACGGCTGTGTATTACTGTGCGAGAGATGGGAG TTATGGTTACGACGGTATGGACGTCTGGGGCCAA GGGACCACGGTCACCGTCTCCTCA |
| | | | SEQ ID NO: 27059 | SEQ ID NO: 31065 |

FIGURE 50

| | | | | |
|---|---|---|---|---|
| | | AA | DIRMTQSPSSLSASVGDRVTITCRASQAIGKHLA WFQQRPGKAPKSLIYAASRLQSGVPSKFSGSGSG TDFTLTISSLQPEDLATYYCQQYSNYPLTFGGGT KVEIK | QVQLVESGGGVVQPGRSLRLSCAVSGITFSSYGMH WARQAPGKGLEWVAVIWYDGSNKNYEDSVKGRF TISRDNSKNTLYLQMNSLRAEDTAVYYCARDGSYG YDGMDVWGQGTTVTVSS |
| | | | SEQ ID NO: 27060 | SEQ ID NO: 31066 |
| iPS:436368 | 21-225_211G3 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAGCAGC TTCTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGTTAGCTCACCG CTCACTTTCGGCGGAGGGACCAAGGTGGAGAT CAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGCATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGGTTAGACT ACAGTAACTACGGGTGGTTCGACCCCTGGGGCCA GGGAACCCTGGTCACCGTCTCCTCA |
| | | | SEQ ID NO: 27061 | SEQ ID NO: 31067 |
| | | AA | EIVLTQSPGTLSLSPGERATLSCRASQSVSSSFLA WYQQKPGQAPRLLIYGASSRATGIPDRFSGSGSG TDFTLTISRLEPEDFAVYYCQQYVSSPLTFGGGT KVEIK | QVQLVESGGGVVQPGRSLRLSCAASGFTFSNYGM HWVRQAPGKGLEWVAVIWHDGSNKYYADSVKGR FTISRDNSKNTLYLQMNSLRAEDTAVYYCARLDYS NYGWFDPWGQGTLVTVSS |
| | | | SEQ ID NO: 27062 | SEQ ID NO: 31068 |

FIGURE 50

| iPS:436370 | 21-225_211A6 | NA | GACATCCAGATGACCCAGTCTCCATCCTCACT GTCTGCATCTGTAGGAGACAGCGTCACCATCA CTTGTCGGGCGAGTCAGGGCATTGGCAAGTAT TTAGCCTGGTTTCAGCAGAAACCAGGGAAAGC CCCTAAGTCCCTGATCTATGCTGCATCCAGTTT GCTAAGTGGGGTCCCATCAAAGTTCAGCGGCA GTGGATCTGGGACAGATTTCACTCTCACCATC AGCAGCCTTCAGCCTGAAGATTTTGCCACTTA TTACTGCCAAAAGTATGATACTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAACTCCAAGAACACGC TGTATCTGCAAATGAACAGCCTGAGAGCCGAGG ACACGGCTGTGTTTTACTGTGCGAGAGATAGGAC GGTGGGCTATGATGGTTTTGATATCTGGGGCCAA GGGACAATGGTCACCGTCTCTTCA |
|---|---|---|---|---|
| | | | SEQ ID NO: 27063 | SEQ ID NO: 31069 |
| | | AA | DIQMTQSPSSLSASVGDSVTITCRASQGIGKYLA WFQQKPGKAPKSLIYAASSLLSGVPSKFSGSGSG TDFTLTISSLQPEDFATYYCQKYDTYPFTFGPGT KVDIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVFYCARDRTVG YDGFDIWGQGTMVTVSS |
| | | | SEQ ID NO: 27064 | SEQ ID NO: 31070 |
| iPS:436372 | 21-225_211A8 | NA | GACATCGAGATGACCCAGTCTCCACCCTCACT GTCTGCATTTGTAGGAGACAGAGTCACCATCA CTTGTCGGGCGAGTCAGGGCATTAGCAGATAT TTAGCCTGGGTTCAGCAGAAACCAGGGAAAG CCCCTAAGTCCCTGATCTATGCTGCATCCAGTT TGCAAAGTGGGGTCTCATCAAGGTTCAGCGGC AGTGGATCTGGGACAGATTTCACTCTCACCAT CAGCAGCCTGCAGCCTGAAGATTTCGCAACTT ATTACTGCCTACGGTATGATACTTACCCTCTCA TTTTCGGCGGAGGGACCAAGGTGGAGATCAA G | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAAAACGC TGTATCTGCAAATGAACAGCCTGAGAGCCGAGG ACACGGCTGTGTATTACTGTGCGAGAGACCACGG TGTCGGGTACTACGGTATGGACGTCTGGGGCCAA GGGACCACGGTCACCGTCTCCTCA |
| | | | SEQ ID NO: 27065 | SEQ ID NO: 31071 |

FIGURE 50

| | | | | |
|---|---|---|---|---|
| | | AA | DIEMTQSPPSLSAFVGDRVTITCRASQGISRYLA WVQQKPGKAPKSLIYAASSLQSGVSSRFSGSGSG TDFTLTISSLQPEDFATYYCLRYDTYPLIFGGGTK VEIK | QVQLVESGGGVVQPGRSLRLSCAASGFTFSSYGMH WVRQAPGKGLEWVAVIWYDGSNEHYADSVKGRF TISRDNSKKTLYLQMNSLRAEDTAVYYCARDHGV GYYGMDVWGQGTTVTVSS |
| | | | SEQ ID NO: 27066 | SEQ ID NO: 31072 |
| iPS:436374 | 21-225_211C10 | NA | GATATTGTGATGACTCAGTCTCCACTCTCCCTG CCCGTCACCCCTGGAGAGCCGGCCTCCATCTC CTGCAGGTCTAGTCAGAGCCTCCTCCATAGTA ATGGATACAACTATTTGGATTGGTACCTGCTG AAGCCAGGGCAGTCTCCACAGCTCCTGATCTA TTTGGGTTCTAATCGGGCCTCCGGGGTCCCTG ACAGGTTCAGTGGCAGTGGATCAGGCACAGAT TTTACACTGAAAATCAGCAGAATGGAGGCTGA GGATGTTGGGATTTATTACTGCATGCAAGCTC TACTAACTCCCGTGTGCAGTTTTGGCCAGGGG ACCAAGCTGGAGATCAAA | CAGGTTCAGCTGGTGCAGTCTGGAGCTGAGGTGA AGAAGCCTGGGGCCTCAGTGAAGGTCTCCTGCA AGGCTTCTGGTTACACCTTTACCAGGCATGGTAT CAGCTGGGTGCGACTGGCCCCTGGACAAGGGCTT GAGTGGATGGGATGGATCAGCGCTTACAATGGTC TCACAAACTATGCACAGAAGTTCCAGGGCAGAG TCACCATGACCACAGACACATCCACGAGCACAG GCTACATGGAGCTGCGGGAGCCTGAGATCTGACG ACACGGCCGTGTATTACTGTGCGAGAGATCCTAC GGTGACCCACTACTACTACTACGGTATGGACGTC TGGGGCCAAGGGACCACGGTCACCGTCTCCTCA |
| | | | SEQ ID NO: 27067 | SEQ ID NO: 31073 |
| | | AA | DIVMTQSPLSLPVTPGEPASISCRSSQSLLHSNGY NYLDWYLLKPGQSPQLLIYLGSNRASGVPDRFS GSGSGTDFTLKISRMEAEDVGIYYCMQALLTPV CSFGQGTKLEIK | QVQLVQSGAEVKKPGASVKVSCKASGYTFTRHGIS WVRLAPGQGLEWMGWISAYNGLTNYAQKFQGRV TMTTDTSTSTGYMELRSLRSDDTAVYYCARDPTVT HYYYYGMDVWGQGTTVTVSS |
| | | | SEQ ID NO: 27068 | SEQ ID NO: 31074 |

FIGURE 50

| iPS:436376 | 21-225_212E6 | NA | GACATCCAGATGACCCAGTCTCCATCCTCACT GTCTGCATCTGTAGGAGACAGAGTCACCATCA CTTGTCGGGCGAGTCAGGGCATTAGCAATCAT TTAGCCTGGTTTCAGCAGAAACCAGGGCAAGC CCCTAAGTCCCTGATCTATGCTGCATCCAGTTT GCAAAGTGGGGTCCCATCAAAGTTCAGCGGCA GTAGATCTGGGACAGATTTCACTCTCACCATC AGCAGCCTGCAGCCTGAAGATTTTGCAACTTA TTACTGCCAACAGTATGTTACCTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGTAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGACTACGG TGTCGGGTACTACGGTACGGACGTCTGGGGCCAA GGGACCACGGTCACCGTCTCCTCA |
|---|---|---|---|---|
|  |  |  | SEQ ID NO: 27069 | SEQ ID NO: 31075 |
|  |  | AA | DIQMTQSPSSLSASVGDRVTITCRASQGISNHLA WFQQKPGQAPKSLIYAASSLQSGVPSKFSGSRSG TDFTLTISSLQPEDFATYYCQQYVTYPLTFGGGT KVEIK | QVQLVESGGGVVQPGRSLRLSCAASGFTFSSYGMH WVRQAPGKGLEWVAVIWYDGSNKNYVDSVKGRF TISRDNSKNTLYLQMNSLRAEDTAVYYCARDYGV GYYGTDVWGQGTTVTVSS |
|  |  |  | SEQ ID NO: 27070 | SEQ ID NO: 31076 |
| iPS:436378 | 21-225_212D7 | NA | GACATCCAGATGACCCAGTCTCCATCCTCACT GTCTGCATCTGTAGGAGACAGAGTCACCATCA CTTGTCGGGCGAGTCAGGGCATTAGCAATCAT TTAGCCTGGTTTCAGCAGAAACCAGGGAAAGC CCCTAAGTCCCTGATCCATGCTGCATCCAGTTT GCAAAGTGGGGTCCCATCAAAATTCAGCGGCA ATAGATCTGGGACAGATTTCACTCTCACCATC AACAACCTGCAGCCTGAAGATTTTGTAACTTA TTACTGCCAGCAGTATAGTAATTACCCTCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAATTATGCAGACTCCGTGAAGGGCCGA TTCACCATCTCCAGAGACAATTCCAAGAACACGC TGTCTCTGCAAATGAACAGCCTGAGAGCCGAGG ACACGGCTGTGTATTACTGTGCGAGAGACTACGG TGTCGGGTACTACGGTACGGACGTCTGGGGCCAA GGGACCACGGTCACCGTCTCCTCA |
|  |  |  | SEQ ID NO: 27071 | SEQ ID NO: 31077 |

FIGURE 50

| | | | | |
|---|---|---|---|---|
| | | AA | DIQMTQSPSSLSASVGDRVTITCRASQGISNHLAWFQQKPGKAPKSLIHAASSLQSGVPSKFSGNRSGTDFTLTINNLQPEDFVTYYCQQYSNYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLSLQMNSLRAEDTAVYYCARDYGVGYYGTDVWGQGTTVTVSS |
| | | | SEQ ID NO: 27072 | SEQ ID NO: 31078 |
| iPS:436380 | 21-225_212H9 | NA | GACATCGAGATGACCCAGTCTCCATCCTCACTGTCTGCATCTGTAGGAGACAGAGTCACCATCACTTGTCGGGCGAGTCAGGGCATTAGCAGTTATTTAGCCTGGCTTCAGCAGAAACCAGGGAAAGCCCCTAAGTCCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTCGCAACTTATTATTGCCTACGGTATGATACTTACCCTCTCACTTTCGGCGGAGGGACCAAGGTGGAGATCAAG | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATGAACACTATGCAGACTCCGTGAAGGGCCGATTCACCATCTCCAGAGACAATTCCAAGAAAACGCTGTATCTGCAAATGAACAGCCTGAGAGCCGAGGACACGGCTGTGTATTACTGTGCGAGAGACCACGGTGTCGGGTACTACGGTATGGACGTCTGGGGCCAAGGGACCACGGTCACCGTCTCCTCA |
| | | | SEQ ID NO: 27073 | SEQ ID NO: 31079 |
| | | AA | DIEMTQSPSSLSASVGDRVTITCRASQGISSYLAWLQQKPGKAPKSLIYAASSLQSGVPSRFSGSGSGTDFTLTISSLQPEDFATYYCLRYDTYPLTFGGGTKVEIK | QVQLVESGGGVVQPGRSLRLSCAASGFTFSSYGMHWVRQAPGKGLEWVAVIWYDGSNEHYADSVKGRFTISRDNSKKTLYLQMNSLRAEDTAVYYCARDHGVGYYGMDVWGQGTTVTVSS |
| | | | SEQ ID NO: 27074 | SEQ ID NO: 31080 |

FIGURE 50

| iPS:436382 | 21-225_212C10 | NA | GAAATAGTGATGACGCAGTCTCCAGCCACCCT GTCTGTGTCTCCAGGGGAAAGAGCCACCCTCT CCTGCAGGGCCAGTCAGAGTGTTGCCAGCAGC TTAGCCTGGTACCAGCAGAAGCCTGGCCAGGC TCCCAGGCTCCTCATCCATGGTACATCCACCA GGGCCACTGATGTCCCAGCCAGGTTCAGTGGC TTTGGGTCTGGGTCGGACTTCACTCTCACCATC AGCAGCCTGCAGTCTGAAGATTTTGCAGTTTA TTCCTGTCAGCAGTATAATGACTGGCCGTGCA GTTTTGGCCAGGGGACCAAGCTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAATGGGTGACAGCTATATGGTATGATGGAAG TCATAAATACTATACAGATTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGAG TATAGTGGGAGCTACCTACTTTGACTACTGGGGC CAGGGAACCCTGGTCACCGTCTCCTCA |
| | | | SEQ ID NO: 27075 | SEQ ID NO: 31081 |
| | | AA | EIVMTQSPATLSVSPGERATLSCRASQSVASSLA WYQQKPGQAPRLLIHGTSTRATDVPARFSGFGS GSDFTLTISSLQSEDFAVYSCQQYNDWPCSFGQG TKLEIK | QVQLVESGGGVVQPGRSLRLSCAASGFTFSSYGMH WVRQAPGKGLEWVTAIWYDGSHKYYTDSVKGRF TISRDNSKNTLYLQMNSLRAEDTAVYYCARDRSIV GATYFDYWGQGTLVTVSS |
| | | | SEQ ID NO: 27076 | SEQ ID NO: 31082 |
| iPS:436384 | 21-225_212F10 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGACTGGTTTCAGCAGAAACCAGGGAAAGC CCCTAAGTCCCTGATCTATTCTGCATCCAATTT GCAAAGTGGGGTCCCATCAAAGTTCAGCGGCA GTAGATCTGGGACAGATTTCACTCTCACCATC AGCAGCCTGCAGCCTGAAGATTTTGCAACTTA TTACTGCCAACACTATAGTAATTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGATATGGCAT GCACTGGGTCCGCCAGGCTCCAGGCAAGGGGCT GGAGTGGGTGGCAGTTATATGGTATGATGGAAGT AATAAACACTATGCAGACTCCGTGAAGGGCCGA TTCACCATCTCCAGAGACAATTCCAAGAATACGT TGTATCTGCAAATGAACAGTCTGAGAGGCGAGG ACACGGCTGTGTATTACTGTGCGAGAGATCGGGG AGTGGGCTACAACGGTATGGACGTCTGGGGCCA AGGGACCACGGTCACCGTCTCCTCA |
| | | | SEQ ID NO: 27077 | SEQ ID NO: 31083 |

FIGURE 50

| | | | | |
|---|---|---|---|---|
| | | AA | DIQMTQSPSSLSASVGDRVTITCRASQGISNYLD WFQQKPGKAPKSLIYSASNLQSGVPSKFSGSRSG TDFTLTISSLQPEDFATYYCQHYSNYPLTFGGGT KVEIK | QVQLVESGGGVVQPGRSLRLSCAASGFTFSRYGMH WVRQAPGKGLEWVAVIWYDGSNKHYADSVKGRF TISRDNSKNTLYLQMNSLRGEDTAVYYCARDRGV GYNGMDVWGQGTTVTVSS |
| | | | SEQ ID NO: 27078 | SEQ ID NO: 31084 |
| iPS:436386 | 21-225_212B11 | NA | GACATCCAGATGACCCAGTCTCCATCTTCCCT GCCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCATTCTCACAA TCAGCAGCCTGCAGCCTGAAGATTTTGCAACT TATTACTGTCTACTGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27079 | SEQ ID NO: 31085 |
| | | AA | DIQMTQSPSSLPASVGDRVTITCRASQGIRNDLG WYQQKPGKAPKRLIYAASSLQSGVPSRFSGSGS GTEFILTISSLQPEDFATYYCLLHYSYPRTFGQGT KVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27080 | SEQ ID NO: 31086 |

FIGURE 50

| | | | | |
|---|---|---|---|---|
| iPS:436388 | 21-225_212H11 | NA | GACATCCGGATGACCCAGTCTCCATCCTCACT GTCTGCATCTGTAGGAGACAGAGTCACCATCA CTTGTCGGGCGAGTCAGGCCATTGGGAAACAT TTAGCCTGGTTTCAGCAGAGGCCTGGGAAAGC CCCTAAGTCCCTGATCTATGCTGCATCCAGATT GCAAAGTGGGGTCCCATCAAAGTTCAGCGGCA GTGGATCTGGGACAGATTTCACTCTCACCATC AGCAGCCTGCAGCCTGAAGATCTTGCAACTTA TTACTGTCAACACTATAGTAATTATCCGCTCAC TTTTGTCGGAGGGACCAAGGTGGAGATCACA | CAGGTGCAGCTGGTGGAGTCTGGGGGAGGCGTG GTCCAGCCTGGGAGGTCCCTGAGACTCTCCTGTG CAGTGTCTGGAATCACCTTCAGTAGTTATGGCAT GCACTGGGCCCGCCAGGCTCCAGGCAAGGGGCT GGAGTGGGTGGCAGTTATATGGTATGATGGAAGT AATAAAAACTATGAAGACTCCGTGAAGGGCCGA TTCACCATCTCCAGAGACAACTCCAAGAACACGC TGTATCTGCAAATGAACAGCCTGAGAGCCGAGG ACACGGCTGTGTATTACTGTGCGAGAGATGGGAG TTATGGTTACGACGGTATGGACGTCTGGGGCCAA GGGACCACGGTCACCGTCTCCTCA |
| | | | SEQ ID NO: 27081 | SEQ ID NO: 31087 |
| | | AA | DIRMTQSPSSLSASVGDRVTITCRASQAIGKHLA WFQQRPGKAPKSLIYAASRLQSGVPSKFSGSGSG TDFTLTISSLQPEDLATYYCQHYSNYPLTFVGGT KVEIT | QVQLVESGGGVVQPGRSLRLSCAVSGITFSSYGMH WARQAPGKGLEWVAVIWYDGSNKNYEDSVKGRF TISRDNSKNTLYLQMNSLRAEDTAVYYCARDGSYG YDGMDVWGQGTTVTVSS |
| | | | SEQ ID NO: 27082 | SEQ ID NO: 31088 |
| iPS:436390 | 21-225_213D2 | NA | GACATCCAGATGACCCAGTCTCCATCCTCACT GTCTGCATCTGTAGGAGACAGAGTCACCATCA CTTGTCGGGCGAGTCAGGGCATTAGCAATCAT TTAGCCTGGTTTCAGCAGAAACCAGGGAAAGC CCCTAAGTCCCTGATCCATGCTGCATCCAGTTT GCAAAGTGGGGTCCCATCAAAATTCAGCGGCA ATAGATCTGGGACAGATTTCACTCTCACCATC AACAACCTGCAGCCTGAAGATTTTGTAACTTA TTACTGCCACCAGTATAGTAATTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAATTATGCAGACTCCGTGAAGGGCCGA TTCACCATCTCCAGAGACAATTCCAAGAACACGC TGTCTCTGCAAATGAACAGCCTGAGAGCCGAGG ACACGGCTGTGTATTACTGTGCGAGAGACTACGG TGTCGGGTACTACGGTACGGACGTCTGGGGCCAA GGGACCACGGTCACCGTCTCCTCA |
| | | | SEQ ID NO: 27083 | SEQ ID NO: 31089 |

FIGURE 50

| | | | | |
|---|---|---|---|---|
| | | AA | DIQMTQSPSSLSASVGDRVTITCRASQGISNHLA WFQQKPGKAPKSLIHAASSLQSGVPSKFSGNRSG TDFTLTINNLQPEDFVTYYCHQYSNYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLSLQMNSLRAEDTAVYYCARDYGVG YYGTDVWGQGTTVTVSS |
| | | | SEQ ID NO: 27084 | SEQ ID NO: 31090 |
| iPS:436392 | 21-225_213B3 | NA | GACATCCAGATGACCCAGTCTCCATCCTCACT GTCTGCATCTGTAGGAGACAGAGTCACCATCA CTTGTCGGGCGAGTCAGGGCATTGGCAAGTAT TTAGCCTGGTTTCAGCAGAAACCAGGGAAAGC CCCTAAGTCCCTGATCTCTGCTGCATCCAGTGT GCTAAGTGGGGTCCCATCAAAGTTCAGCGGCA GTGGATCTGGGACAGATTTCACTCTCACCATC AGCAGCCTTCAGCCTGAAGATTTTGCCACTTA TTACTGCCAAAAGTATGATACTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAACTCCAAGAACACGC TGTATCTGCAAATGAACAGCCTGAGAGCCGAGG ACACGGCTGTGTATTACTGTGCGAGAGATAGGAC GGTGGGCTATGATGGTTTTGATATCTGGGGCCAA GGGACAATGGTCACCGTCTCTTCA |
| | | | SEQ ID NO: 27085 | SEQ ID NO: 31091 |
| | | AA | DIQMTQSPSSLSASVGDRVTITCRASQGIGKYLA WFQQKPGKAPKSLISAASSVLSGVPSKFSGSGSG TDFTLTISSLQPEDFATYYCQKYDTYPFTGPGT KVDIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDRTVG YDGFDIWGQGTMVTVSS |
| | | | SEQ ID NO: 27086 | SEQ ID NO: 31092 |

FIGURE 50

| | | | | |
|---|---|---|---|---|
| iPS:436394 | 21-225_213C4 | NA | GACATCCATATGACCCAGTCTCCATCCTCACT GTCTGCATCTGTAGGAGACAGAGTCACCATCA CTTGTCGGGCGAGTCAGGCCATTAGGAATTAT TTAGCCTGGTGTCAGCAGAAACCAGGGAAAG CCCCTAAGACCCTGATCTATGCTGCATCCAGT TTGCAAAGTGGGGTCCCATCAAAGTTCAGCGG CAGTGGATCTGGGACAGATTTCACTCTCACCA TCAGCAGCCTGCAGCCTGAAGATTTTGCAACT TATTACTGCCAACAGTATAGTAATTACCCTCTC ACTTTCGGCGGAGGGACCAAGGTGGAGATCA AA | CAGGTGCAGTTGGTGGAGTCTGGGGGAGGCGTG GTCCAGCCTGGGAGGTCCCTGAGACTCTCCTGTG CAACGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGACTACGG TGTCGGGTACGACGGTATGGACGTCTGGGGCCA AGGGACCACGGTCACCGTCTCCTCA |
| | | | SEQ ID NO: 27087 | SEQ ID NO: 31093 |
| | | AA | DIHMTQSPSSLSASVGDRVTITCRASQAIRNYLA WCQQKPGKAPKTLIYAASSLQSGVPSKFSGSGS GTDFTLTISSLQPEDFATYYCQQYSNYPLTFGGG TKVEIK | QVQLVESGGGVVQPGRSLRLSCATSGFTFSSYGMH WVRQAPGKGLEWVAVIWYDGSNKHYADSVKGRF TISRDNSKNTLYLQMNSLRAEDTAVYYCARDYGV GYDGMDVWGQGTTVTVSS |
| | | | SEQ ID NO: 27088 | SEQ ID NO: 31094 |
| iPS:436396 | 21-225_213E5 | NA | GACATCCGGATGACCCAGTCTCCATCCTCACT GTCTGCATCTGTAGGAGACAGAGTCACCATCA CTTGTCGGGCGAGTCAGGCCATTGGGAAACAT TTAGCCTGGTTTCAGCAGAGGCCTGGGAAAGC CCCTAAGTCCCTGATCTATGCTGCATCCAGATT GCAAAGTGGGGTCCCATCAAAGTTCAGCGGCA GTGGATCTGGGACAGATTTCACTCTCACCATC AGCAGCCTGCAGCCTGAAGATCTTGCAACTTA TTACTGTCAACACTATAGTAATTATCCGCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGTGTCTGGAATCACCTTCAGTAGTTATGGCAT GCACTGGGCCCGCCAGGCTCCAGGCAAGGGGCT GGAGTGGGTGGCAGTTATATGGTATGATGGAAGT AATAAAAACTATGAAGACTCCGTGAAGGGCCGA TTCACCATCTCCAGAGACAACTCCAAGAACACGC TGTATCTGCAAATGAACAGCCTGAGAGCCGAGG ACACGGCTGTGTATTACTGTGCGAGAGATGGGAG TTATGGTTACGACGGTATGGACGTCTGGGGCCAA GGGACCACGGTCACCGTCTCCTCA |
| | | | SEQ ID NO: 27089 | SEQ ID NO: 31095 |

FIGURE 50

| | | | | |
|---|---|---|---|---|
| | | AA | DIRMTQSPSSLSASVGDRVTITCRASQAIGKHLA WFQQRPGKAPKSLIYAASRLQSGVPSKFSGSGSG TDFTLTISSLQPEDLATYYCQHYSNYPLTFGGGT KVEIK | QVQLVESGGGVVQPGRSLRLSCAVSGITFSSYGMH WARQAPGKGLEWVAVIWYDGSNKNYEDSVKGRF TISRDNSKNTLYLQMNSLRAEDTAVYYCARDGSYG YDGMDVWGQGTTVTVSS |
| | | | SEQ ID NO: 27090 | SEQ ID NO: 31096 |
| iPS:436398 | 21-225_213B8 | NA | GACATCCAGATGACCCAGTCTCCATCCTCACT GTCTGCATCTGTAGGAGACAGAGTCACCATCA CTTGTCGGGCGAGTCAGGGCATTAGCAATCAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAGTAATTACCCTCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGACTACGG TGTCGGGTACTACGGTACGGACGTCTGGGGCCAA GGGACCACGGTCACCGTCTCCTCA |
| | | | SEQ ID NO: 27091 | SEQ ID NO: 31097 |
| | | AA | DIQMTQSPSSLSASVGDRVTITCRASQGISNHLA WFQQKPGKAPKSLIYAASSLQSGVPSKFSGSGSG TDFTLTISSLQPEDFATYYCQQYSNYPLTFGGGT KVEIK | QVQLVESGGGVVQPGRSLRLSCAASGFTFSSYGMH WVRQAPGKGLEWVAVIWYDGSNKHYADSVKGRF TISRDNSKNTLYLQMNSLRAEDTAVYYCARDYGV GYYGTDVWGQGTTVTVSS |
| | | | SEQ ID NO: 27092 | SEQ ID NO: 31098 |

FIGURE 50

| | | | | |
|---|---|---|---|---|
| iPS:436400 | 21-225_213H7 | NA | GACATCGTGATGACCCAGTCTCCAGACTCCCT GGCTGTGTCTCTGGGCGAGAGGGCCACCATCA ACTGCAAGTCCAGCCAGAATGTTTTAGACATC TCCAACAATAAGAATTCCTTAGGTTGGTTCCA GCAGAAACCAGGTCAGCCTCCCAAGCTGCTCA TTAACTGGGCATCTACCCGGGAATCCGGGGTC CCTGACCGCTTCAGTGGCAGCGGGTCTGGGAC AGATTTCACTCTCACCATCAGCAGCCTGCAGA CTGAAGATGTGGCAGTTTATCACTGTCAGCAA TATTATAACATTCCTCCGACGTTCGGCCGAGG GACCAAGGTGGAAATCAAA<br><br>SEQ ID NO: 27093 | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACCATA TGCACTGGGTGCGACAGGCCCCTGGACAAGGGC TTGAGTGGATGGGATGGATCAATCCTAAGAGTGA TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGAAAA GCCTGGGAGCTACTACAAATACTGGGGCCAGGG AACCCTGGTCACCGTCTCCTCA<br><br>SEQ ID NO: 31099 |
| | | AA | DIVMTQSPDSLAVSLGERATINCKSSQNVLDISN NKNSLGWFQQKPGQPPKLLINWASTRESGVPDR FSGSGSGTDFTLTISSLQTEDVAVYHCQQYYNIP PTFGRGTKVEIK<br><br>SEQ ID NO: 27094 | QVQLVQSGAEVKKPGASVKVSCKASGYTFTGYHM HWVRQAPGQGLEWMGWINPKSDGTNYAQKFQGR VTMTRDTSISTAYMELSRLRSDDTAVYYCAREKPG SYYKYWGQGTLVTVSS<br><br>SEQ ID NO: 31100 |
| iPS:436402 | 21-225_213H12 | NA | GACATCGTGATGACCCAGTCTCCAGACTCCCT GGCTGTGTCTCTGGGCGAGAGGGCCACCATCA CCTGCAAGTCCAGCCAGAATGTTTTAAAGACC TCCAACAATAGGAACTACTTAGCTTGGTACCA GCAGAAACCAGGACAGCCTCCTAAGGTGCTCA TTTACTGGGCATCTACCCGGGAATCCGGGGTC CCTGACCGATTCAGTGGCAGCGGGTCTGGGAC AGATTTCACTCTCATCATCAGCAGCCTGCAGG CTGAAGATGTGGCAGTTTATTACTGTCACCAA TATTATAGTATTCCGTGGACCTTCGGCCAAGG GACCAAGGTGGAAATCAAG<br><br>SEQ ID NO: 27095 | CAGGTTCAGCTGGTGCAGTCTGGAGCTGAGGTGA AGAAGCCTGGGGCCTCAGTGAAGGTCTCCTGCA AGGCTTCTGGTTTCACCTTTACCAGCTATGGTATC AACTGGGTGCGACAGGCCCCTGGACAAGGGCTT GAGTGGATGGGATGGATCAGCGTTCACAATGGT AACACAGACTATGCACAGAAGTTCCAGGGCAGA GTCACCATGACCACAGACACATCCACGAGCACA GCCTACATGGAACTGAGGAGCCTGAGATCTGAC GACACGGCCGTGTATTACTGTGCGAGAGACTACT ACTACGGTATGGACGTCTGGGGCCAAGGGACCA AGGTCACCGTCTCCTCA<br><br>SEQ ID NO: 31101 |

FIGURE 50

| | | | | |
|---|---|---|---|---|
| | | AA | DIVMTQSPDSLAVSLGERATITCKSSQNVLKTSN NRNYLAWYQQKPGQPPKVLIYWASTRESGVPD RFSGSGSGTDFTLIISSLQAEDVAVYYCHQYYSIP WTFGQGTKVEIK | QVQLVQSGAEVKKPGASVKVSCKASGFTFTSYGIN WVRQAPGQGLEWMGWISVHNGNTDYAQKFQGRV TMTTDTSTSTAYMELRSLRSDDTAVYYCARDYYY GMDVWGQGTKVTVSS |
| | | | SEQ ID NO: 27096 | SEQ ID NO: 31102 |
| iPS:436404 | 21-225_214C3 | NA | GACATCCAGATGACCCAGTCTCCATCCTCACT GTCTGCATCTGTTGGAGACAGAGTCACCATAA CTTGTCGGGCGAGTCAGGGCATTGGCAATTAT TTAGCCTGGTTTCAGCAGAAACCAGGTAAAGT CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTACAACTTA TTACTGCCAACAATATATGACTTACCCAATCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG AAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGGAGACTCCGTGAAGGGCCGA TTCACCATCTCCAGAGACAATTCCAAGAATACGC TGTATCTGCAAATGAACAGCCTGAGAGCCGAGG ACACGGCTGTGTATTACTGTGCGAGAGATCGGGG AGTGGGCTACGACGGAATGGACGTCTGGGGCCA AGGGACCACGGTCACCGTCTCCTCA |
| | | | SEQ ID NO: 27097 | SEQ ID NO: 31103 |
| | | AA | DIQMTQSPSSLSASVGDRVTITCRASQGIGNYLA WFQQKPGKVPKSLIYAASSLQSGVPSKFSGSGSG TDFTLTISSLQPEDFTTYYCQQYMTYPITFGPGTK VDIK | QVQLVESGGGVVQPGRSLRLSCEASGFTFSSYGMH WVRQAPGKGLEWVAVIWYDGSNKNYGDSVKGRF TISRDNSKNTLYLQMNSLRAEDTAVYYCARDRGV GYDGMDVWGQGTTVTVSS |
| | | | SEQ ID NO: 27098 | SEQ ID NO: 31104 |

FIGURE 50

| iPS:436406 | 21-225_214E4 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATCTTACTTACCCATTCAC TTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAAAACTATGCAGACTCCGTGAAGGGCCGA ATCACCATCTCCAGAGACAATTCCAAGAACACGC TGTATCTGCAAATGAACAGCCTGAGAGCCGAGG ACACGGCTGTGTATTACTGTGCGAGAGATAGGAC GGTGGGCTATGATGGTTGTGATATCTGGGGCCAA GGGGCAATGGTCACCGTCTCTTCA |
|---|---|---|---|---|
|  |  |  | SEQ ID NO: 27099 | SEQ ID NO: 31105 |
|  |  | AA | DIQMTQSPSSLSASVGDRVTITCRASQGISNYLA WFQQKPGKAPKSLIYAASSLQSGVPSKFSGSGSG TDFTLTISSLQPEDFATYYCQQYLTYPFTFGPGTK VDIK | QVQLVESGGGVVQPGRSLRLSCAASGFTFSSYGMH WVRQAPGKGLEWVAVIWYDGSNENYADSVKGRI TISRDNSKNTLYLQMNSLRAEDTAVYYCARDRTVG YDGCDIWGQGAMVTVSS |
|  |  |  | SEQ ID NO: 27100 | SEQ ID NO: 31106 |
| IPS:436408 | 21-225_214H8 | NA | GAAATTGTGCTGACTCAGTCTCCAGACTTTCA GTCTGTGACTCCAAAGGAGAAAGTCACCATCA CCTGCCGGGCCAGTCAGAGCATTGGTGTTAGC TTACACTGGTACCAGCAGAAACCAGATCAGTC TCCACAACTCCTCATCAAGTATGCTTCCCAGTC CTTCTCAGGGGTCCCCTCGAGGTTCAGTGGCA GTGGATCTGGGACAGATTTCACCCTCACCATC AATAGCCTGGAAGCTGAAGATGCTGCAACGTA TTACTGTCATCAGAGTCGTAGTTTACCATTCAC TTTCGGCCCTGGGTCCAAAGTGGATATCAAA | CAGGTCCAGCTGGTGCAGTCTGGGGGCTGAGGTG AAGAAGCCTGGGGCCTCAGTGAAGGTCTCCTGC AAGGCTTCTGGATACACCTTCACCGGCCACTATA TACACTGGGTGCGACAGGCCCCTGGACAAGGGC TTGAGTGGATGGGATGGATCAACTCTAACAGTGG TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTATATTACTGTGCGAAAGACGG GAGATACAGCTATGGTTACGACTGGTTCGACCCC TGGGGCCAGGGAACCCTGGTCACCGTCTCCTCA |
|  |  |  | SEQ ID NO: 27101 | SEQ ID NO: 31107 |

FIGURE 50

| | | | | |
|---|---|---|---|---|
| | | AA | EIVLTQSPDFQSVTPKEKVTITCRASQSIGVSLHW YQQKPDQSPQLLIKYASQSFSGVPSRFSGSGSGT DFTLTINSLEAEDAATYYCHQSRSLPFTFGPGSK VDIK | QVQLVQSGAEVKKPGASVKVSCKASGYTFTGHYIH WVRQAPGQGLEWMGWINSNSGGTNYAQKFQGRV TMTRDTSISTAYMELSRLRSDDTAVYYCAKDGRYS YGYDWFDPWGQGTLVTVSS |
| | | | SEQ ID NO: 27102 | SEQ ID NO: 31108 |
| iPS:436410 | 21-225_212E10 | NA | GACATCCAGATGACCCAGTCTCCATCCTCACT GTCTGCATCTGTAGGAGACAGAGTCACCATCA CTTGTCGGGCGAGTCAGGGCATTAGCAATCAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAGTAATTACCCTCTCA CTTTCGGCGGAGGGACCAAGGTGGAGATCAA A | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTCTCTGCAAATGAACAGCCTGAGAGCCGAGG ACACGGCTGTGTATTACTGTGCGAGAGACTACGG TGTCGGGTACTACGGTACGGACGTCTGGGGCCAA GGGACCACGGTCACCGTCTCCTCA |
| | | | SEQ ID NO: 27103 | SEQ ID NO: 31109 |
| | | AA | DIQMTQSPSSLSASVGDRVTITCRASQGISNHLA WFQQKPGKAPKSLIYAASSLQSGVPSKFSGSGSG TDFTLTISSLQPEDFATYYCQQYSNYPLTFGGGT KVEIK | QVQLVESGGGVVQPGRSLRLSCAASGFTFSSYGMH WVRQAPGKGLEWVAVIWYDGSNKYYADSVKGRF TISRDNSKNTLSLQMNSLRAEDTAVYYCARDYGVG YYGTDVWGQGTTVTVSS |
| | | | SEQ ID NO: 27104 | SEQ ID NO: 31110 |

FIGURE 50

| iPS:436412 | 21-225_214H9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTATTTTACTGTGCGAGAGAGAGGTA TACCAGCAGCTGGTACGACTACGGTATGGACGTC TGGGGCCAAGGGACCACGGTCACCGTCTCCTCA |
| | | | SEQ ID NO: 27105 | SEQ ID NO: 31111 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEIK | QVQLVESGGGVVQPGRSLRLSCAASGFTFSSYVMH WVRQAPGKGLEWVAVIWYDGSNKYYADSVKGRF TISRDNSKNTLYLQMNSLRAEDTAVFYCARERYTS SWYDYGMDVWGQGTTVTVSS |
| | | | SEQ ID NO: 27106 | SEQ ID NO: 31112 |
| iPS:436414 | 21-225_214G10 | NA | GACATCCAGATGACCCTGTGTCCATCTTCCCC GCCTGCATCTGTTGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCATTCTCACAA TCAGCAGCCTGCAGCCTGAAGATTTTGCAACT TATTACTGTCTACTGCATAATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27107 | SEQ ID NO: 31113 |

FIGURE 50

| | | | | |
|---|---|---|---|---|
| | | AA | DIQMTLCPSSPPASVGDRVTITCRASQGIRNDLG WYQQKPGKAPKRLIYAASSLQSGVPSRFSGSGS GTEFILTISSLQPEDFATYYCLLHNSYPRTFGQGT KVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27108 | SEQ ID NO: 31114 |
| iPS:436416 | 21-225_214G12 | NA | GACATCCAGATGACCCAGTCTCCATCTTCCCC GCCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCATTCTCACAAT CAGCAGCCTGCAGCCTGAAGATTTTGCAACTT ATTACTGTGTAATGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27109 | SEQ ID NO: 31115 |
| | | AA | DIQMTQSPSSPPASVGDRVTITCRASQGIRNDLG WYQQKPGKAPKRLIYAASSLQSGVPSRFSGSGS GTEFILTISSLQPEDFATYYCVMHYSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27110 | SEQ ID NO: 31116 |

FIGURE 50

| iPS:436418 | 21-225_215E3 | NA | GACATCCAGATGACCCAGTCTCCATCCTCCCC GCCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTGTAATGCATAATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT ACACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGTGTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
|---|---|---|---|---|
| | | | SEQ ID NO: 27111 | SEQ ID NO: 31117 |
| | | AA | DIQMTQSPSSPPASVGDRVTITCRASQGIRNDLG WYQQKPGKAPKRLIYAASSLQSGVPSRFSGSGS GTEFTLTISSLQPEDFATYYCVMHNSYPRTFGQG TKVEIK | QVQLVESGGGVVQPGRSLRLSCAASGFTFSDYVIH WVRQAPGKGLEWVTVIWYDGSNKYYADSVKGRF TISRDNSKNTLYLQMNSVRAEDTAVYYCARERYSS GWYDYGMDVWGQGTTVTVSS |
| | | | SEQ ID NO: 27112 | SEQ ID NO: 31118 |
| iPS:436420 | 21-225_215B5 | NA | GACATCCAGATGACCCAGTCTCCATCCTCACT GTCTGCATCTGTAGGAGACAGAGTCACCATCA CTTGTCGGGCGAGTCAGGGCATTAGCAATCAT TTAGCCTGGTTTCAGCAGAAACCAGGGAAAGC CCCTAAGTCCCTGATCCATGCTGCATCCAGTTT GCAAAGTGGGGTCCCATCAAAATTCAGCGGCA ATAGATCTGGGACAGATTTCACTCTCACCATC AACAACCTGCAGCCTGAAGATTTTGTAACTTA TTACTGCCAGCAGTATATTAATTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAATTATGCAGACTCCGTGAAGGGCCGA TTCACCATCTCCAGAGACAATTCCAAGAACACGC TGTCTCTGCAAATGAACAGCCTGAGAGCCGAGG ACACGGCTGTGTATTACTGTGCGAGAGACTACGG TGTCGGGTACTACGGTACGGACGTCTGGGGCCAA GGGACCACGGTCACCGTCTCCTCA |
| | | | SEQ ID NO: 27113 | SEQ ID NO: 31119 |

FIGURE 50

| | | | | |
|---|---|---|---|---|
| | | AA | DIQMTQSPSSLSASVGDRVTITCRASQGISNHLA WFQQKPGKAPKSLIHAASSLQSGVPSKFSGNRSG TDFTLTINNLQPEDFVTYYCQQYINYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLSLQMNSLRAEDTAVYYCARDYGVG YYGTDVWGQGTTVTVSS |
| | | | SEQ ID NO: 27114 | SEQ ID NO: 31120 |
| iPS:436422 | 21-225_215D6 | NA | GACATCCAGATGACCCAGTCTCCATCCTCACT GTCTGCATCTGTAGGAGACAGAGTCACCATCA CTTGTCGGGCGAGTCAGGGCATTAGCAATCAT TTAGCCTGGTTTCAGCAGAAACCAGGGAAAGC CCCTAAGTCCCTGATCTATGCTGCATCCAGTTT GCATAGTGGGGTCCCATCAAAGTTCAGCGGCA GTAGATCTGGGACAGATTTCACTCTCACCATC AGCAGCCTGCAGCCTGAAGATTTTGCAACTTA TTACTGCCAACAGTATGTTACTTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGATTCCGTGAAGGGCCGA TTCACCATCTCCAGAGACAATTCCAAGAACACAC TGTATCTGCAAATGAACAGCCTGAGAGCCGAGG ACACGGCTGTGTATTACTGTGCGAGAGACTGCGG TGTCGGATACTACGGTACGGACGTCTGGGGCCAA GGGACCACGGTCACCGTCTCCTCA |
| | | | SEQ ID NO: 27115 | SEQ ID NO: 31121 |
| | | AA | DIQMTQSPSSLSASVGDRVTITCRASQGISNHLA WFQQKPGKAPKSLIYAASSLHSGVPSKFSGSRSG TDFTLTISSLQPEDFATYYCQQYVTYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDCGV GYYGTDVWGQGTTVTVSS |
| | | | SEQ ID NO: 27116 | SEQ ID NO: 31122 |

FIGURE 50

| iPS:436424 | 21-225_215H6 | NA | GAAATTGTGCTGACTCAGTCTCCAGACTTTCA GTCTGTGACTCCAAAGGAGAAAGTCACCATCA CCTGCCGGGCCAGTCAGAGCATCGGTGTTAGC TTACACTGGTACCAGCAGAAACCAGATCAGTC TCCACAGCTCCTCATCAAGTATGCTTCCCAGTC CCTCTCAGGGGTCCCCTCGAGGTTCAGTGGCA GTGGATCTGGGACAGATTTCACCCTCACCATC AATAGCCTGGAAGCTGAAGATGCTGCAACGTA TTACTGTCATCAGAGTCGCAGTTTACCATTCAC TTTCGGCCCTGGGTCCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCCACTATA TACACTGGGTGCGACAGGCCCCTGGACAAGGGC TTGAGTGGATGGGATGGATCAACTCTAACAGTGG TGGCACAAATTATGCAGAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAAAGACGG GAGATACAGCTATGGTCACGACTGGTTCGACCCC TGGGGCCAGGGAACCCTGGTCACCGTCTCCTCA |
| | | | SEQ ID NO: 27117 | SEQ ID NO: 31123 |
| | | AA | EIVLTQSPDFQSVTPKEKVTITCRASQSIGVSLHW YQQKPDQSPQLLIKYASQSLSGVPSRFSGSGSGT DFTLTINSLEAEDAATYYCHQSRSLPFTFGPGSK VDIK | QVQLVQSGAEVKKPGASVKVSCKASGYTFTGHYIH WVRQAPGQGLEWMGWINSNSGGTNYAEKFQGRV TMTRDTSISTAYMELSRLRSDDTAVYYCAKDGRYS YGHDWFDPWGQGTLVTVSS |
| | | | SEQ ID NO: 27118 | SEQ ID NO: 31124 |
| iPS:436426 | 21-225_215C7 | NA | GAAATTGTGTTGACGCAGTCTCCAGGCACCCT GTCTTTGTCTCCAGGGGAAAGAGTCACCCTCT CCTGCAGGGCCAGTCAGAGGATTACCACCAAC TTCTTAGCTTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGTCTGAAGATTTTGCAG TTTATTACTGTCAGCAGTATGTTAGTTCATTGC TCACTTTCGGCGGAGGGACCAAGGTGGAGATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTTACTATGGCAT GCACTGGGTCCGCCAGGCTCCAGGCAAGGGGCT GGAGTGGGTGGCAGTTATATGGCATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGGTTAGACT ACAGTAATTACGGGTGGTTCGACCCCTGGGGCCA GGGAACCCTGGTCACCGTCTCCTCA |
| | | | SEQ ID NO: 27119 | SEQ ID NO: 31125 |

FIGURE 50

| | | | | |
|---|---|---|---|---|
| | | AA | EIVLTQSPGTLSLSPGERVTLSCRASQRITTNFLA WYQQKPGQAPRLLIYGASSRATGIPDRFSGSGSG TDFTLTISRLESEDFAVYYCQQYVSSLLTFGGGT KVEIK | QVQLVESGGGVVQPGRSLRLSCAASGFTFSYYGM HWVRQAPGKGLEWVAVIWHDGSNKYYADSVKGR FTISRDNSKNTLYLQMNSLRAEDTAVYYCARLDYS NYGWFDPWGQGTLVTVSS |
| | | | SEQ ID NO: 27120 | SEQ ID NO: 31126 |
| iPS:436428 | 21-225_215E11 | NA | GACATCCAGATGACCCAGTGTCCATCTTCCCC GCCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCATTCTCACAAT CAGCAGCGTGCAGCGTGAAGATTTTGCAACTT ATTACTGTGTAATGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGGCCAAGGGACCACGGTCACCGTCTCCTCA |
| | | | SEQ ID NO: 27121 | SEQ ID NO: 31127 |
| | | AA | DIQMTQCPSSPPASVGDRVTITCRASQGIRNDLG WYQQKPGKAPKRLIYAASSLQSGVPSRFSGSGS GTEFILTISSVQREDFATYYCVMHYSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27122 | SEQ ID NO: 31128 |

FIGURE 50

| iPS:436430 | 21-225_215A12 | NA | GACATCCAGATGACCCAGTCTCCATCCTCACT GTCTGCATCTGTAGGAGACAGAATCACCATCA CTTGTCGGACGAGTCAGGACATTGGCAATTAT TTAGCCTGGTTTCAGCAGAAACCAGGGAAAGC CCCTAAGTCCCTGATCTATGCTGCATCCAGTTT ACAGAGTGGGGTCCCATCAAAGTTCAGCGGCA GTAGATCTGGGACAGATTTCACTCTCACCATC AGCAGCCTGCAGTCTGAAGATTTTGCAACTTA TTACTGCCAACAGTATGTTACTTACCCGCTCAC TTTCGGCGGAGGGACCAAGGTGGAGATCAAA | CAGGTGCAACTGGTGGAGTCTGGGGGAGGCGTG GTCCAGCCTGGGAGGTCCCTGAGACTCTCCTGTG CAGCGTCTGGACTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGG AGTGGGCTACTACGGTATGGACGTCTGGGGCCA AGGGACCACGGTCACCGTCTCCTCA |
| | | | SEQ ID NO: 27123 | SEQ ID NO: 31129 |
| | | AA | DIQMTQSPSSLSASVGDRITITCRTSQDIGNYLAW FQQKPGKAPKSLIYAASSLQSGVPSKFSGSRSGT DFTLTISSLQSEDFATYYCQQYVTYPLTFGGGTK VEIK | QVQLVESGGGVVQPGRSLRLSCAASGLTFSSYGMH WVRQAPGKGLEWVAVIWYDGSNEHYADSVKGRF TISRDNSKNTLYLQMNSLRAEDTAVYYCARDRGV GYYGMDVWGQGTTVTVSS |
| | | | SEQ ID NO: 27124 | SEQ ID NO: 31130 |
| iPS:436432 | 21-225_215H12 | NA | GAAATTGTGTTGACGCAGTCTCCAGGCACCCT GTCTTTGTCTCCAGGGGAAAGAGCCACCCTCT CCTGCAGGGCCAGTCAGAGTGTTAGCAGCAGC TTCTTAGCTTGGTACCAGCAGAAACCTGGCCA GGCTCCCAGGCTCCTCATGTATGGTGCATCCA GCAGGGCCATTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGTTAGCTCACCG CTCACTTTCGGCGGAGGGACCAAGGTGGAGAT CAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGCATGATGGAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGGTTAGACT ACAGTAACTACGGGTGGTTCGACCCCTGGGGCCA GGGAACCCTGGTCACCGTCTCCTCA |
| | | | SEQ ID NO: 27125 | SEQ ID NO: 31131 |

FIGURE 50

| | | | | |
|---|---|---|---|---|
| | | AA | EIVLTQSPGTLSLSPGERATLSCRASQSVSSSFLA WYQQKPGQAPRLLMYGASSRAIGIPDRFSGSGS GTDFTLTISRLEPEDFAVYYCQQYVSSPLTFGGG TKVEIK | QVQLVESGGGVVQPGRSLRLSCAASGFTFSNYGM HWVRQAPGKGLEWVAVIWHDGSNKYYADSVKGR FTISRDNSKNTLYLQMNSLRAEDTAVYYCARLDYS NYGWFDPWGQGTLVTVSS |
| | | | SEQ ID NO: 27126 | SEQ ID NO: 31132 |
| iPS:436434 | 21-225_216B10 | NA | GAAATAGTGATGACGGAGTCTCCAGCCACCCT GTCTGTGTCTCCAGGGGAAAGAGCCACCCTCT CCTGCAGGGCCAGTCAGAGTGTTAACAACAAC TTAGCCTGGTACCGGCAGAAACCTGGCCAGGC TCCCAGGCTCCTCATCTATGGTGCATCCACCA GGGCCACTGGTATCCCACCCAGGTTCAGTGGC AGTGGGTCTGGGACAGAGTTCACTCTCTCCAT CAGCAGCCTGCAGTCTGAAGATTTTGCAGTTT ATTACTGTCAGCAGTATAATGACTGGCCGTGC AGTTTTGGCCAGGGGACCAAACTGGAGATCAG A | CAGGTGCAGCTGGTGGAGTCGGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGCTATATGGTATGATGGAAG TAATAAATACTATGCAGACTCCGTGAAGGGCCG ATTCACCATCTCCAGAGACAATTCCAAGCGCACG CTGTATCTGCAGATGAACAGCCTGAGAGCCGAG GACACGGCTGTGTATTACTGTGCGAGAGATCCCA ACATAGTGGGAGCTACTTGGTTTGACTACTGGGG CCAGGGAACCCTGGTCACCGTCTCCTCA |
| | | | SEQ ID NO: 27127 | SEQ ID NO: 31133 |
| | | AA | EIVMTESPATLSVSPGERATLSCRASQSVNNNLA WYRQKPGQAPRLLIYGASTRATGIPPRFSGSGSG TEFTLSISSLQSEDFAVYYCQQYNDWPCSFGQGT KLEIR | QVQLVESGGGVVQPGRSLRLSCAASGFTFSSYGMH WVRQAPGKGLEWVAAIWYDGSNKYYADSVKGRF TISRDNSKRTLYLQMNSLRAEDTAVYYCARDPNIV GATWFDYWGQGTLVTVSS |
| | | | SEQ ID NO: 27128 | SEQ ID NO: 31134 |

FIGURE 50

| | | | | |
|---|---|---|---|---|
| iPS:436436 | 21-225_216F10 | NA | GAAGTTGTGTTGACGCAGTCTCCAGGCACCCT GTCTTTGTCTCCAGGGGAAAGAGCCACCCTCT CCTGCAGGGCCAGTCAGAGTGTTAGCAGCAGC TTCTTAGCCTGGTACCAGCAGAAACCTGGCCA GGCTCCCAGGCTCCTCATCTATGGTACATCCA CCAGGGCCACTGGCATCCCTGACAGGTTCAGT GGCAGTGGGTCTGGGACAGACTTCATTCTCAC CATCAGCAGACTGGAGCCTGAAGATTTTGCAG TATATTACTGTCAACAGTATGATAGGTCACCA TTCACTTTCGGCCCTGGGACCAAAGTGGATAT CAAA | GAGGTACAGCTGGTGGAGTCTGGGGGAGGCTTG GTACAGCCTGGGGGGTCCCTGAGACTCTCCTGTG CAGCCTCTGGATTCTCCTTCAGAAGCTATAGCAT GAACTGGGTCCGCCAGGCTCCAGGGAAGGGGCT GGAGTGGGTTTCATACATTACTGGTAGTAGTAGT ACCATATACTACGCAGACTCTGTGAAGGGCCGAT TCACCATCTCCAGAGACAATGCCAAGAACTCACT GTATCTGCAAATGAACAGCCTGAGAGACGAGGA CACGGCTGTGTATTACTGTGCGAGATCGGGTTTA GCAGTGGAGGACTACTGGGGCCAGGGAACCCTG GTCACCGTCTCCTCA |
| | | | SEQ ID NO: 27129 | SEQ ID NO: 31135 |
| | | AA | EVVLTQSPGTLSLSPGERATLSCRASQSVSSSFLA WYQQKPGQAPRLLIYGTSTRATGIPDRFSGSGSG TDFILTISRLEPEDFAVYYCQQYDRSPFTFGPGTK VDIK | EVQLVESGGGLVQPGGSLRLSCAASGFSFRSYSMN WVRQAPGKGLEWVSYITGSSSTIYYADSVKGRFTIS RDNAKNSLYLQMNSLRDEDTAVYYCARSGLAVED YWGQGTLVTVSS |
| | | | SEQ ID NO: 27130 | SEQ ID NO: 31136 |
| iPS:436438 | 21-225_216E8 | NA | GACATCCAGATGACCCAGTCTCCATCTTCCCT GCCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCATTCTCACAAT CAGCAGCCTGCAGCCTGAAGATTTTGCAACTT ATTACTGTCTAATGCATTATAGTTACCCTCGGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27131 | SEQ ID NO: 31137 |

FIGURE 50

| | | | | |
|---|---|---|---|---|
| | | AA | DIQMTQSPSSLPASVGDRVTITCRASQGIRNDLG WYQQKPGKAPKRLIYAASSLQSGVPSRFSGSGS GTEFILTISSLQPEDFATYYCLMHYSYPRTFGQGT KVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27132 | SEQ ID NO: 31138 |
| iPS:436440 | 21-225_216H12 | NA | GACATCCAGATGACCCTGTCTCCATCTTCCCTG CCTGCATCTGTAGGAGACAGAGTCACCATCAC TTGCCGGGCAAGTCAGGGCATTAGAAATGATT TAGGCTGGTATCAGCAGAAACCAGGGAAAGC CCCTAAGCGCCTTATCTATGGTGCATCCAGTTT GCAAAGTGGGGTCCCATCAAGGTTCAGCGGCA GTGGATCTGGGACAGAATTCATTCTCACAATC AGCAGCCTGCAGCCTGAAGATTTTGCAACTTA TTACTGTGTAATGCATAATAGTTACCCTCGGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27133 | SEQ ID NO: 31139 |
| | | AA | DIQMTLSPSSLPASVGDRVTITCRASQGIRNDLG WYQQKPGKAPKRLIYGASSLQSGVPSRFSGSGS GTEFILTISSLQPEDFATYYCVMHNSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27134 | SEQ ID NO: 31140 |

FIGURE 50

| iPS:436448 | 21-225_217A3 | NA | GAAATTGTGCTGACTCAGTCTCCAGACTTTAAGTCTGTGACTCCAAAGGAGAAAGTCACCATCACCTGCCGGGCCAGTCAGAGCATTGGTAGTAGCTTACACTGGTACCAGCAGAAACCAGATCAGTCTCCAAAGCTCCTCGTCAAGTATGCTTCCCAGTCCTTCTCAGGGGTCCCCTCGAGGTTCAGTGGCAGTGGATCTGGGACAGATTTCACCCTCACCATCAACAGCCTGGAGGCTGAAGATGGTGCAACGTATTACTGTCATCAGAGTAGAAGTTTACCGTGGACGTTCGGCCAAGGGACCAAGGTGGAAATCAAA | GAGGTGCAACTGGTGGAGTCGGGGGGAGGCTTGGTACAGCCTGGGGGGTCCCTGAGACTCTCCTGTGCAGCCTCTGGATTCACCTTCAGTAGCTATAATATGAACTGGGTCCGCCAGGCTCCAGGGAAGGGCCTGGAGTGGGTTTCATACATTAGTAGTAGTCGTAATATCATATATTACGCAGACTCTGTGAAGGGCCGATTCACCATCTCCAGAGAAAATGCCAAGAACTCACTGTCTCTGCAAATGGACAGCCTGAGAGACGAGGACACGGCTGTGTATTACTGTGCGCGAGATGGCTCTTATAGCAGTGGCTGGTACTGGGGTTTTGACTACTGGGGCCAGGGCACCCTGGTCACCGTCTCCTCA |
| | | | SEQ ID NO: 27135 | SEQ ID NO: 31141 |
| | | AA | EIVLTQSPDFKSVTPKEKVTITCRASQSIGSSLHWYQQKPDQSPKLLVKYASQSFSGVPSRFSGSGSGTDFTLTINSLEAEDGATYYCHQSRSLPWTFGQGTKVEIK | EVQLVESGGGLVQPGGSLRLSCAASGFTFSSYNMNWVRQAPGKGLEWVSYISSSRNIIYYADSVKGRFTISRENAKNSLSLQMDSLRDEDTAVYYCARDGSYSSGWYWGFDYWGQGTLVTVSS |
| | | | SEQ ID NO: 27136 | SEQ ID NO: 31142 |
| iPS:436450 | 21-225_217E5 | NA | GACATCCAGATGACCCAGTGTCCATCTTCCCCGCCTGCATTTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTGTGCTGCATCCAGTTTGCAAAGTGGGGTCCCATCAAGGTTCAGCGGCAGTGGATCTGGGACAGAATTCATTCTCACAATCAGCAGCCTGCAGCCTGAAGATTTTGCAACTTATTACTGTGTAATGCATAATAGTTACCCTCGGACGTTCGGCCAAGGGACCAAGGTGGAAATCAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27137 | SEQ ID NO: 31143 |

FIGURE 50

| | | | | |
|---|---|---|---|---|
| | | AA | DIQMTQCPSSPPAFVGDRVTITCRASQGIRNDLG WYQQKPGKAPKRLICAASSLQSGVPSRFSGSGS GTEFILTISSLQPEDFATYYCVMHNSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27138 | SEQ ID NO: 31144 |
| iPS:436452 | 21-225_217G5 | NA | GACATCCTGATGACCCAGTCTCCATCCTCACT GTCTGCATCTGTAGGAGACAGAGTCACCATCA CTTGTCGGGCGAGTCAGGGCATTGGCAATTAT TTAGCCTGGTTTCAGCAGAGACCAGGGAAAGC CCCTAAGTCCCTGATCTATGCTGCATCCAGTTT GCAAAGTGGGGTCCCATCAAAGTTCAGCGGCA CTCGATCTGGGACAGATTTCACTCTCACCATC AGCAGCCTGCAGCCTGACGATTTTGCAACTTA TTACTGCCAACAGTATGTTAATTACCCTCTCAC TTTCGGCGGAGGGACCAAGGTGGAGATCAAC | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCAATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGACTACGG TGTCGGGTACTACGGTCTGGACGTCTGGGGCCAA GGGACCACGGTCACCGTCTCCTCA |
| | | | SEQ ID NO: 27139 | SEQ ID NO: 31145 |
| | | AA | DILMTQSPSSLSASVGDRVTITCRASQGIGNYLA WFQQRPGKAPKSLIYAASSLQSGVPSKFSGTRSG TDFTLTISSLQPDDFATYYCQQYVNYPLTFGGGT KVEIN | QVQLVESGGGVVQPGRSLRLSCAASGFTFSSNGMH WVRQAPGKGLEWVAVIWYDGSNKNYADSVKGRF TISRDNSKNTLYLQMNSLRAEDTAVYYCARDYGV GYYGLDVWGQGTTVTVSS |
| | | | SEQ ID NO: 27140 | SEQ ID NO: 31146 |

FIGURE 50

| iPS:436454 | 21-225_217B10 | NA | GACATCCGGATGACCCAGTCTCCATCCTCACT GTCTGCATCGTAGGAGACAGAGTCACCATCA CTTGTCGGGCGAGTCAGGCCATTGGGAAACAT TTAGCCTGGTTTCAGCAGAGGCCTGGGAAAGC CCCTAAGTCCCTGATCTATGCTGCATCCAGATT GCAAAGTGGGGTCCCATCAAAGTTCAGCGGCA GTGGATCTGGGACAGATTTCACTCTCACCATC AGCAGCGTGCAGCCTGAAGATCTTGCAACTTA TTACCGTCAACACACCAGTAAATCTCCAGTGC AGCTTGTCGGAGGCACCAAGGTGGAGATCAC A | CAGGTGCAGCTGGTGGAGTCTGGGGGAGGCGTG GTCCAGCCTGGGAGGTCCCTGAGACTCTCCTGTG CAGTGTCTGGAATCACCTTCAGTAGTTATGGCAT GCACTGGGCCCGCCAGGCTCCAGGCAAGGGGCT GGAGTGGGTGGCAGTTATATGGTATGATGGAAGT AATAAAAACTATGAAGACTCCGTGAAGGGCCGA TTCACCATCTCCAGAGACAACTCCAAGAACACGC TGTATCTGCAAATGAACAGCCTGAGAGCCGAGG ACACGGCTGTGTATTACTGTGCGAGAGATGGGAG TTATGGTTACGACGGTATGGACGTCTGGGGCCAA GGGACCACGGTCACCGTCTCCTCA |
|---|---|---|---|---|
| | | | SEQ ID NO: 27141 | SEQ ID NO: 31147 |
| | | AA | DIRMTQSPSSLSASVGDRVTITCRASQAIGKHLA WFQQRPGKAPKSLIYAASRLQSGVPSKFSGSGSG TDFTLTISSVQPEDLATYYRQHTSKSPVQLVGGT KVEIT | QVQLVESGGGVVQPGRSLRLSCAVSGITFSSYGMH WARQAPGKGLEWVAVIWYDGSNKNYEDSVKGRF TISRDNSKNTLYLQMNSLRAEDTAVYYCARDGSYG YDGMDVWGQGTTVTVSS |
| | | | SEQ ID NO: 27142 | SEQ ID NO: 31148 |
| iPS:436456 | 21-225_217G10 | NA | GACATCCAGATGACCCAGTCTCCATCTTCCCC GCCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCATTCTCACTAT CAGCAGCCTGCAGCCTGAAGATTTTGCAACTT ATTACTGTGTAATGCATAATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27143 | SEQ ID NO: 31149 |

FIGURE 50

| | | | | |
|---|---|---|---|---|
| | | AA | DIQMTQSPSSPPASVGDRVTITCRASQGIRNDLG WYQQKPGKAPKRLIYAASSLQSGVPSRFSGSGS GTEFILTISSLQPEDFATYYCVMHNSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27144 | SEQ ID NO: 31150 |
| iPS:436458 | 21-225_217H12 | NA | GACATCCAGATGACCCAGTCTCCATCTTCCCC GCCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCATTCTCACAAT CAGCAGCCTGCAGCCTGAAGATTTTGCAACTT ATTACTGTCTAATGCATTATAGTTACCCTCGGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGCT GGGGGCCAAGGGACCACGGTCACCGTCTCCTCA |
| | | | SEQ ID NO: 27145 | SEQ ID NO: 31151 |
| | | AA | DIQMTQSPSSPPASVGDRVTITCRASQGIRNDLG WYQQKPGKAPKRLIYAASSLQSGVPSRFSGSGS GTEFILTISSLQPEDFATYYCLMHYSYPRTFGQGT KVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27146 | SEQ ID NO: 31152 |

FIGURE 50

| iPS:436462 | 21-225_218C4 | NA | GACATCCAGATGACCCAGTGTCCATCTTCCCC GCCTGCATTTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCATTCTCACAAT CAGCAGCGTGCAGCGTGAAGATTTTGCAACTT ATTACTGTGTAATGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27147 | SEQ ID NO: 31153 |
| | | AA | DIQMTQCPSSPPAFVGDRVTITCRASQGIRNDLG WYQQKPGKAPKRLIYAASSLQSGVPSRFSGSGS GTEFILTISSVQREDFATYYCVMHYSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27148 | SEQ ID NO: 31154 |
| iPS:436464 | 21-225_219H1 | NA | GACATCCAGATGACCCAGTCTCCATCCTCACA GTCTGCATCTGTAGGAGACAGAGTCACCATCA CTTGTCGGGCGAGTCAGGGCATTAGCAATTAT TTAGACTGGTTTCAGCAGAAACCAGGGAAAGC CCCTAAGTCCCTGATCTATTCTGCATCCAATTT GCAAAGTGGGGTCCCATCAAAGTTCAGCGGCA GTAGATCTGGGACAGATTTCACTCTCACCATC AGCAGCCTGCAGCCTGAAGATTTTGCAACTTA TTACTGCCAACACTATAGTAATTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGATATGGCAT GCACTGGGTCCGCCAGGCTCCAGGCAAGGGGCT GGAGTGGGTGGCAGTTATATGGTATGATGGAAGT AATAAACACTATGCAGACTCCGTGAAGGGCCGA TTCACCATCTCCAGAGACAATTCCAAGAATACGT TGTATCTGCAAATGAACAGTCTGAGAGGCGAGG ACACGGCTGTGTATTACTGTGCGAGAGATCGGGG AGTGGGCTACAACGGTATGGACGTCTGGGGCCA AGGGACCACGGTCACCGTCTCCTCA |
| | | | SEQ ID NO: 27149 | SEQ ID NO: 31155 |

FIGURE 50

| | | | | |
|---|---|---|---|---|
| | | AA | DIQMTQSPSSQSASVGDRVTITCRASQGISNYLD WFQQKPGKAPKSLIYSASNLQSGVPSKFSGSRSG TDFTLTISSLQPEDFATYYCQHYSNYPLTFGGGT KVEIK | QVQLVESGGGVVQPGRSLRLSCAASGFTFSRYGMH WVRQAPGKGLEWVAVIWYDGSNKHYADSVKGRF TISRDNSKNTLYLQMNSLRGEDTAVYYCARDRGV GYNGMDVWGQGTTVTVSS |
| | | | SEQ ID NO: 27150 | SEQ ID NO: 31156 |
| iPS:436472 | 21-225_220E1 | NA | GAAATTGTGTTGACGCAGTCTCCAGGCACCCT GTCTTTGTCTCCAGGGGAAAGAGCCACCCTCT CCTGCAGGGCCAGTCAGAGTATTAGCCGCAGC CACTTAGTCTGGTACCAGCAGAAACCTAACCA GGCTCCCAGGCTCCTCCTCTATGTTACATCCAG CAGGGCCACTGGCATCCCAGACAGGTTTAGTG GCAGTGGGTCTGGGACAGACTTCACTCTCACC ATCAGAAGTCTGGAGCCTGAAGATTTTGCAAT GTATTACTGTCAGCAGTATGGTAGCTCACCGT GGACGTTCGGCCAAGGGACCAAGGTGGAAAT CAAA | CAGGTGCAGCTGCAGGAGTCGGGCCCAGGACTG GTGAAGCCTTCGGAGACCCTGTCCCTCACCTGCA CTGTCTCTGGTGGCTCCATCAGTACTTACTACTG GAGCTGGATCCGGCAGCCCCCAGGGAAGGGACT GGAGTGGATTGGGTATATCTATTACAGTGGGACC ACCAACTACAACCCCTCCCTCAAGAGTCGAGTCA CCATATCAGTAGACACGTCCAAGAACCAGTTCTC CCTGAAGCTGAGCTCTGTGACCGCTGCGGACACG GCCGTGTATTACTGTGCGAGAGACCAGCAGTGGC TGGTACGTGGGAGGGACAACTACTACTACGGTAT GGACGTCTGGGGCCAAGGGACCACGGTCACCGT CTCCTCA |
| | | | SEQ ID NO: 27151 | SEQ ID NO: 31157 |
| | | AA | EIVLTQSPGTLSLSPGERATLSCRASQSISRSHLV WYQQKPNQAPRLLLYVTSSRATGIPDRFSGSGS GTDFTLTIRSLEPEDFAMYYCQQYGSSPWTFGQ GTKVEIK | QVQLQESGPGLVKPSETLSLTCTVSGGSISTYYWSW IRQPPGKGLEWIGYIYYSGTTNYNPSLKSRVTISVDT SKNQFSLKLSSVTAADTAVYYCARDQQWLVRGRD NYYYGMDVWGQGTTVTVSS |
| | | | SEQ ID NO: 27152 | SEQ ID NO: 31158 |

FIGURE 50

| iPS:436480 | 21-225_220F8 | NA | GACATCCAGATGACCCTGTCTCCATCTTCCCC GCCTGCATTTGTAGGAGACAGAGTCACCATCA CTCGCCGGGCAAGTCAGGGCATTAGAAATGAT TTAGGCTGGTATCAGCAGAAACCAGGGAAAG CCCCTAAGCGCCTTATATGTGGTGCATCCAGT TTGCAAAGTGGGGTCCCATCAAGGTTCAGCGG CAGTGGATCTGGGACAGAATTCATTCTCACAA TCAGCAGCCTGCAGCGTGAAGATTTTGCAACT TATTACTGTGTAATGCATAATAGTTACCCTCG GACGTTCGGCCAAGGGACCAAGGTGGAAATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27153 | SEQ ID NO: 31159 |
| | | AA | DIQMTLSPSSPPAFVGDRVTITRRASQGIRNDLG WYQQKPGKAPKRLICGASSLQSGVPSRFSGSGS GTEFILTISSLQREDFATYYCVMHNSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27154 | SEQ ID NO: 31160 |
| iPS:436488 | 21-225_221A6 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGCTCCTGATCTATACTGCATCCAATT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGGCTGAGGTG AAGAAGCCTGGGGCCTCAGTGAAGGTCTCCTGC AAGACTTCTGGATACACCTTCACCGGCTACTATA TGCACTGGGTGCGACAGGCCCCTGGACAAGGGC TTGAGTGGATGGGATGGATCCACCCTAACAGTGG TGGCACAAACTATGCACAGAAATTTCAGGGCAG GGTCACCCTGACCAGGGACACGTCCATCAGCAC AGCCTACATGGACCTGAGCAGGCTGAGATCTGA CGACACGGCCGTGTATTACTGTGCGAGAGATGG GACCAGCTCGTTTGACTACTGGGGCCAGGGAACC CTGGTCACCGTCTCCTCA |
| | | | SEQ ID NO: 27155 | SEQ ID NO: 31161 |

FIGURE 50

| | | | | |
|---|---|---|---|---|
| | | AA | DIQMTQSPSSVSASVGDRVTITCRASQGISSWLA WYQQKPGKAPKLLIYTASNLQSGVPSRFSGSGS GTDFTLTISSLQPEDFATYYCQQANSFPFTFGPGT KVDIK | QVQLVQSGAEVKKPGASVKVSCKTSGYTFTGYYM HWVRQAPGQGLEWMGWIHPNSGGTNYAQKFQGR VTLTRDTSISTAYMDLSRLRSDDTAVYYCARDGTS SFDYWGQGTLVTVSS |
| | | | SEQ ID NO: 27156 | SEQ ID NO: 31162 |
| iPS:436490 | 21-225_221F6 | NA | GACATCCAGATGACCCAGTCTCCATCCTCACT GTCTGGATCTGTAGGAGACAGAGTCACCATCA CTTGTCGGGCGAGTCAGGGCATTAGCAATTAT TTAGCCTGGTTTCAGCAGAAACCAGGGAAAGC CCCTAAATCCCTGATCTATGCTGCATCCAATTT ACAAAGTGGGGTCCCATCAAAGTTCAGCGGCA GTAGATCTGGGACAGATTTCACTCTCACCATC AGCAGCCTGCAGCCTGAAGATTTTGCAACTTA TTACTGCCAACAGTATATGACTTACCCGCTCA CTTTCGGCGGAGGGACCAGGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCAATGGCAT GCACTGGGTCCGCCAGGCTCCAGGCAAGGGGCT GGAGTGGGTGGCAGTTATATGGTACGATGGAAG TAATGAAAACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGATCGGA CAGTGGGCTACAACGGTATGGACGTCTGGGGCC AAGGGACCACGGTCACCGTCTCCTCA |
| | | | SEQ ID NO: 27157 | SEQ ID NO: 31163 |
| | | AA | DIQMTQSPSSLSGSVGDRVTITCRASQGISNYLA WFQQKPGKAPKSLIYAASNLQSGVPSKFSGSRSG TDFTLTISSLQPEDFATYYCQQYMTYPLTFGGGT RVEIK | QVQLVESGGGVVQPGRSLRLSCAASGFTFSSNGMH WVRQAPGKGLEWVAVIWYDGSNENYADSVKGRF TISRDNSKNTLYLQMNSLRAEDTAVYYCARDRTVG YNGMDVWGQGTTVTVSS |
| | | | SEQ ID NO: 27158 | SEQ ID NO: 31164 |

FIGURE 50

| | | | | |
|---|---|---|---|---|
| iPS:436494 | 21-225_221F12 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGCTCCTGATCTATACTGCATCCAATT TGCAAAGTGGGGTCCCATCAAGGTTCAGCGGC AGTGGATCTGGGACAGATTTCACTCTCACCAT CAGCAGCCTGCAGCGTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGGCTGAGGTG AAGAAGCCTGGGGCCTCAGTGAAGGTCTCCTGC AAGACTTCTGGATACACCTTCACCGGCTACTATA TGCACTGGGTGCGACAGGCCCCTGGACAAGGGC TTGAGTGGATGGGATGGATCCACCCTAACAGTGG TGGCACAAACTATGCACAGAAATTTCAGGGCAG GGTCACCCTGACCAGGGACACGTCCATCAGCAC AGCCTACATGGACCTGAGCAGGCTGAGATCTGA CGACACGGCCGTGTATTACTGTGCGAGAGATGG GACCAGCTCGTTTGACTACTGGGGCCAGGGAACC CTGGTCACCGTCTCCTCA |
| | | | SEQ ID NO: 27159 | SEQ ID NO: 31165 |
| | | AA | DIQMTQSPSSVSASVGDRVTITCRASQGISSWLA WYQQKPGKAPKLLIYTASNLQSGVPSRFSGSGS GTDFTLTISSLQREDFATYYCQQANSFPFTFGPGT KVDIK | QVQLVQSGAEVKKPGASVKVSCKTSGYTFTGYYM HWVRQAPGQGLEWMGWIHPNSGGTNYAQKFQGR VTLTRDTSISTAYMDLSRLRSDDTAVYYCARDGTS SFDYWGQGTLVTVSS |
| | | | SEQ ID NO: 27160 | SEQ ID NO: 31166 |
| iPS:436496 | 21-225_222E1 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGCTCTTGATCTATACTGCATCCAATT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCGTGGGACCAAAGTGGATATCAA A | CAGGTGCAGCTGGTGCAGTCTGGGGGCTGAGGTG AAGAAGCCTGGGGCCTCAGTGAAGGTCTCCTGC AAGACTTCTGGATACACCTTCACCGGCTACTATA TGCACTGGGTGCGACAGGCCCCTGGACAAGGGC TTGAGTGGATGGGATGGATCCACCCTAACAGTGG TGGCACAAACTATGCACAGAAATTTCAGGGCAG GGTCACCCTGACCAGGGACACGTCCATCAGCAC AGCCTACATGGACCTGAGCAGGCTGAGATCTGA CGACACGGCCGTGTATTACTGTGCGAGAGATGG GACCAGCTCGTTTGACTACTGGGGCCAGGGAACC CTGGTCACCGTCTCCTCA |
| | | | SEQ ID NO: 27161 | SEQ ID NO: 31167 |

FIGURE 50

| | | | | |
|---|---|---|---|---|
| | | AA | DIQMTQSPSSVSASVGDRVTITCRASQGISSWLA WYQQKPGKAPKLLIYTASNLQSGVPSRFSGSGS GTDFTLTISSLQPEDFATYYCQQANSFPFTFGRGT KVDIK | QVQLVQSGAEVKKPGASVKVSCKTSGYTFTGYYM HWVRQAPGQGLEWMGWIHPNSGGTNYAQKFQGR VTLTRDTSISTAYMDLSRLRSDDTAVYYCARDGTS SFDYWGQGTLVTVSS |
| | | | SEQ ID NO: 27162 | SEQ ID NO: 31168 |
| iPS:436500 | 21-225_222H3 | NA | GACATCGTGATGACCCAGTCTCCAGACTCCCT GGCTGTGTCTCTGGGCGAGAGGGCCACCATCA ACTGCAAGTCCAGCCAGAGTGTTTTGAAAAGT TCCAACCATAGGAACTACTTAGCTTGGTACCA ACAGAAACCAGGGCAGCCTCCTCAGCTTCTCA TTTACTGGGCATCTACCCGGGAAACCGGGGTC CCTGACCGATTCAGTGGCAGCGGGTCTGGGAC AGATTTCACTCTCACCATCAGCAGCCTTCAGG CTGAAGATGTGTCAGTTTATTCCTGTCAGCAA TATTCTTCTATTCCGTGGACGTTCGGCCAAGG GACCAAGGTGGAAATCAAT | CAGGTTCAGCTGGTGCAGTCTGGAGCTGAGGTGA AGAAGCCTGGGGCCTCAGTGAAGGTCTCCTGCA AGGCTTCTGGTTTCACCTTTACCAGCTATGGTATC AACTGGGTGCGACAGGCCCCTGGACAAGGGCTT GAGTGGATGGGATGGATCAGCGTTTACAATGGTA ACACAAACTATGCACAGAAGCTCCAGGGCAGAG TCACCATGACCACAGACACATCCACGAGCACAG CCTACATGGAGCTGAGGAGCCTGAGATCTGACG ACACGGCCGTGTATTACTGTGCGAGAGACTACTA CTACGGTTTTGACGTCTGGGGGCCAAGGGACCACG GTCACCGTCTCCTCA |
| | | | SEQ ID NO: 27163 | SEQ ID NO: 31169 |
| | | AA | DIVMTQSPDSLAVSLGERATINCKSSQSVLKSSN HRNYLAWYQQKPGQPPQLLIYWASTRETGVPD RFSGSGSGTDFTLTISSLQAEDVSVYSCQQYSSIP WTFGQGTKVEIN | QVQLVQSGAEVKKPGASVKVSCKASGFTFTSYGIN WVRQAPGQGLEWMGWISVYNGNTNYAQKLQGR VTMTTDTSTSTAYMELRSLRSDDTAVYYCARDYY YGFDVWGQGTTVTVSS |
| | | | SEQ ID NO: 27164 | SEQ ID NO: 31170 |

FIGURE 50

| iPS:436502 | 21-225_222A11 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTGGCCTGGTTTCAGCAGAAACCAGGGAAAGC CCCTAAGTCCCTGATCTACGCTGCATCCAGTTT GCAAAGTGGGGTCCCATCAAAGTTCAGCGGCA GTGGATCTGGGACAGATTTCACTCTCACCATC AGTAGCCTGCAGCCTGAAGATTTTGCAACTTA TTACTGCCTATATTATCTTAATTATCCGCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAATGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGCGAGAGATCGGGA TGTCGGGTACAACGGTATGGACGTCTGGGGCCA AGGGACCACGGTCACCGTCTCCTCA |
|---|---|---|---|---|
| | | | SEQ ID NO: 27165 | SEQ ID NO: 31171 |
| | | AA | DIQMTQSPSSLSASVGDRVTITCRASQGISNYLA WFQQKPGKAPKSLIYAASSLQSGVPSKFSGSGSG TDFTLTISSLQPEDFATYYCLYYLNYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDRDV GYNGMDVWGQGTTVTVSS |
| | | | SEQ ID NO: 27166 | SEQ ID NO: 31172 |
| iPS:436504 | 21-225_222H4 | NA | GACATCCAGATGACCCATTCTCCATCCTCCCT GTCTGCATCTGTAGGAGACAGAGTCACCATCA CTTGCCGGGCAAGTCAGAACATTAGTAATTAT GTTAATTGGTATCAGCAGAAACCAGGGAAAG CCCCTAAGTTCCTGATCTATACTGCATCGAGTT TGCAAAGTGGGGTCTCGTCACGGTTCAGTGGC AGTGGATCTGGGACAGATTTCACTCTCACCAT CAGCAGTGTGCACCGTGACGATTTTGCAATTT ACTATTGTCAGCAGTATTACTTTACCCCATTCA CTTTCGGCCGTGGGACCAAAGTGGATATCAAA | GAGGTGCAGCTATTGGAGTCTGGGGGAGGCTTG GTACAGCCTGGGGGGTCCCTGAGACTCTCCTGTG CAGCCTCTGGCTTCACCTTTAGCAACTTTGCCAT GAGTTGGGTCCGCCAGGCTCCAGGGAAGGGACT GGAGTGGGTCTCAAGTATTGTTGGTAGTGGTGGT CGCACGTACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTATTGTGCGAAAGACCCTTA TCGTGTAGCAGTGGCTGGGGCCTTTGACTACTGG GGCCAGGGAACCCTGATCACCGTCTCCTCA |
| | | | SEQ ID NO: 27167 | SEQ ID NO: 31173 |

FIGURE 50

| | | | | |
|---|---|---|---|---|
| | | AA | DIQMTHSPSSLSASVGDRVTITCRASQNISNYVN WYQQKPGKAPKFLIYTASSLQSGVSSRFSGSGSG TDFTLTISSVHRDDFAIYYCQQYYFTPFTFGRGT KVDIK | EVQLLESGGGLVQPGGSLRLSCAASGFTFSNFAMS WVRQAPGKGLEWVSSIVGSGGRTYYADSVKGRFTI SRDNSKNTLYLQMNSLRAEDTAVYYCAKDPYRVA VAGAFDYWGQGTLITVSS |
| | | | SEQ ID NO: 27168 | SEQ ID NO: 31174 |
| IPS:436506 | 21-225_222C7 | NA | GAAATTGTGTTGACGCAGTCTCCAGGCACCCT GTCTTTGTCTCCAGGGGAAAGAGCCACCCTCT CCTGCAGGGCCAGTCAGAGTGTTTACAGCAAC TACTTAGCCTGGTACCAGCAGAAACCTGGCCA GGCTCCCAGGCTCCTCATCTATGGTGCATCAA GCAGGGCCACTGGCATCCCAGACAGGTTCGGT GGCAGTGGGTCTGGGACAGACTTCACTCTCGC CATAAGCAGACTGGAGCCTGAAGACTTTACAA TATATTACTGTCAGCAGTATGAAGACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGGTCGCTACTG GAGCTGGATCCGCCAGCCCCCAGGGAAGGGGCT GGAGTGGATTGGGGAAATCAATCATAGTGGAAG CGCCAACTACAACCCGTCCCTCAAGAGTCGAGTC ACCATATCAGTAGACACGTCCAAGAACCAGTTCT CCCTGAAGCTGAGTTCTGTGACCGCCGCGGACAC GGCTGTATATTACTGTGCGAGAGACTACGGCGCC CTTGATTTCTGGGGGCCAAGGGACAATGGTCACCG TCTCTTCA |
| | | | SEQ ID NO: 27169 | SEQ ID NO: 31175 |
| | | AA | EIVLTQSPGTLSLSPGERATLSCRASQSVYSNYLA WYQQKPGQAPRLLIYGASSRATGIPDRFGGSGS GTDFTLAISRLEPEDFTIYYCQQYEDSPWTFGQG TKVEIK | QVQLQQWGAGLLKPSETLSLTCAVYGGSFSGRYW SWIRQPPGKGLEWIGEINHSGSANYNPSLKSRVTIS VDTSKNQFSLKLSSVTAADTAVYYCARDYGALDF WGQGTMVTVSS |
| | | | SEQ ID NO: 27170 | SEQ ID NO: 31176 |

FIGURE 50

| iPS:436508 | 21-225_222F7 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGCTCCTGATCTATACTGCATCCAATT TGCAAAGTGGGGTCCCATCAAGGTTCAGCGGC AGTGGATCTGGGACAGATTTCACTCTCACCAT CAGCAGCGTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGC AAGACTTCTGGATACACCTTCACCGGCTACTATA TGCACTGGGTGCGACAGGCCCCTGGACAAGGGC TTGAGTGGATGGGATGGATCCACCCTAACAGTGG TGGCACAAACTATGCACAGAAATTTCAGGGCAG GGTCACCCTGACCAGGGACACGTCCATCAGCAC AGCCTACATGGACCTGAGCAGGCTGAGATCTGA CGACACGGCCGTGTATTACTGTGCGAGAGATGG GACCAGCTCGTTTGACTACTGGGGCCAGGGAACC CTGGTCACCGTCTCCTCA |
|  |  |  | SEQ ID NO: 27171 | SEQ ID NO: 31177 |
|  |  | AA | DIQMTQSPSSVSASVGDRVTITCRASQGISSWLA WYQQKPGKAPKLLIYTASNLQSGVPSRFSGSGS GTDFTLTISSVQPEDFATYYCQQANSFPFTFGPGT KVDIK | QVQLVQSGAEVKKPGASVKVSCKTSGYTFTGYYM HWVRQAPGQGLEWMGWIHPNSGGTNYAQKFQGR VTLTRDTSISTAYMDLSRLRSDDTAVYYCARDGTS SFDYWGQGTLVTVSS |
|  |  |  | SEQ ID NO: 27172 | SEQ ID NO: 31178 |
| iPS:436510 | 21-225_222H8 | NA | GACATCCAGATGACCCATTCTCCATCTTCCCTG TCTGCATCTGTAGGAGACAGAGTCACCATCAC TTGCCGGGCAAGTCAGAACATTAGTAATTATG TTAATTGGTATCAGCAGAAACCAGGGAAAGCC CCTAAGTTCCTGATCTATATTGCATCGAGTTTG CAAAGTGGGGTCTCGTCACGGTTCAGTGGCAG TGGATCTGGGACAGATTTCACTCTCACCATCA GCAGTGTGCACCGTGACGATTTTGCAATTTAC TACTGTCAGCAGTATTACTTTACCCCATTCACT TTCGGCCGTGGGACCAAAGTGGATATCAAA | GAGGTGCAGCTATTGGAGTCTGGGGGAGGCTTG GTACAGCCTGGGGGGTCCCTGAGACTCTCCTGTG CAGCCTCTGGCTTCACCTTTAGCAACTTTGCCAT GAGTTGGGTCCGCCAGGCTCCAGGGAAGGGACT GGAGTGGGTCTCAAGTATTGTTGGTAGTGGTGGT CGCACGTACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTATTGTGCGAAAGACCCTTA TCGTGTAGCAGTGGCTGGGGCCTTTGACTACTGG GGCCAGGGAACCCTGATCACCGTCTCCTCA |
|  |  |  | SEQ ID NO: 27173 | SEQ ID NO: 31179 |

FIGURE 50

| | | | | |
|---|---|---|---|---|
| | | AA | DIQMTHSPSSLSASVGDRVTITCRASQNISNYVN WYQQKPGKAPKFLIYIASSLQSGVSSRFSGSGSG TDFTLTISSVHRDDFAIYYCQQYYFTPFTFGRGT KVDIK | EVQLLESGGGLVQPGGSLRLSCAASGFTFSNFAMS WVRQAPGKGLEWVSSIVGSGGRTYYADSVKGRFTI SRDNSKNTLYLQMNSLRAEDTAVYYCAKDPYRVA VAGAFDYWGQGTLITVSS |
| | | | SEQ ID NO: 27174 | SEQ ID NO: 31180 |
| iPS:436514 | 21-225_222D10 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTGGCCTGGTTTCAGCAGAAACCAGGGAAAGC CCCTAAGTCCCTGATCTACGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCTACATTATCTTAATTACCCGCTCAC TTTCGGCGGAGGGACCAGGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGA TGTCGGGTACAACGGTATGGACGTCTGGGGCCA AGGGACCACGGTCACCGTCTCCTCA |
| | | | SEQ ID NO: 27175 | SEQ ID NO: 31181 |
| | | AA | DIQMTQSPSSLSASVGDRVTITCRASQGISNYLA WFQQKPGKAPKSLIYAASSLQSGVPSKFSGSGSG TDFTLTISSLQPEDFATYYCLHYLNYPLTFGGGT RVEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDRDV GYNGMDVWGQGTTVTVSS |
| | | | SEQ ID NO: 27176 | SEQ ID NO: 31182 |

FIGURE 50

| iPS:436516 | 21-225_222C12 | NA | GACATCCAGATGACTCAGTGTCCATCTTCCGT GTCTGCATCTGTAGGAGACAGAGTCACCATCA CTTGTCGGGTGAGTCAGGGTATTAGCAGCTGG TTAGCCTGGTATCAGCAGAAACCAGGGAAAG CCCTTAAGCTCGTGATCTATACTGCATCCAATT TGCAAAGTGGGGTCCCATCAAGGTTCAGCGGC AGTGGATCTGGGACAGATTTCACTCTCACCAT CAGCAGCGTGCAGCGTGAAGATTTTGCAACTT ACTATTGTCAACAGGATAACAGTTTCCCATTC ACTTTCGGCCGAGGGACCAAAGTGGATATCAA A | CAGGTGCAGCTGGTGCAGTCTGGGGCTGAGGTG AAGAAGCCTGGGGCCTCAGTGAAGGTCTCCTGC AAGACTTCTGGATACACCTTCACCGGCTACTATA TGCACTGGGTGCGACAGGCCCCTGGACAAGGGC TTGAGTGGATGGGATGGATCCACCCTAACAGTGG TGGCACAAACTATGCACAGAAATTTCAGGGCAG GGTCACCCTGACCAGGGACACGTCCATCAGCAC AGCCTACATGGACCTGAGCAGGCTGAGATCTGA CGACACGGCCGTGTATTACTGTGCGAGAGATGG GACCAGCTCGTTTGACTACTGGGGCCAGGGAACC CTGGTCACCGTCTCCTCA |
| | | | SEQ ID NO: 27177 | SEQ ID NO: 31183 |
| | | AA | DIQMTQCPSSVSASVGDRVTITCRVSQGISSWLA WYQQKPGKALKLVIYTASNLQSGVPSRFSGSGS GTDFTLTISSVQREDFATYYCQQDNSFPFTFGRG TKVDIK | QVQLVQSGAEVKKPGASVKVSCKTSGYTFTGYYM HWVRQAPGQGLEWMGWIHPNSGGTNYAQKFQGR VTLTRDTSISTAYMDLSRLRSDDTAVYYCARDGTS SFDYWGQGTLVTVSS |
| | | | SEQ ID NO: 27178 | SEQ ID NO: 31184 |
| iPS:436520 | 21-225_223G10 | NA | GACATCGTGATGACCCAGTCTCCAGACTCCCT GGCTGTGTCTCTGGGCGAGAGGGCCACCATCA ACTGCAAGTCCAGCCAGAGTATTTTACTCAGC TCCAACAATAAGAACTACTTAGCTTGGCACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCTGCAA TATTTTAGTACTCCGTGGACGTTCGGCCAAGG GACCAAGGTGGAAATCAAA | CAGGTTCAGCTGGTGCAGTCTGGAGCTGAGGTGA AGAAGCCTGGGGCCTCAGTGAAGGTCTCCTGCA AGGCTTCTGGTTTCACCTTTACCAGCTATGGTATC AACTGGGTGCGACAGGCCCCTGGACAAGGACTT GAGTGGATGGGATGGATCAGCGTTTACAGTGGTA ACACAAACTATGCACAGAAGCTCCAGGGCAGAG TCACCATGACCACAGATACATCCACGAGCACAG CCTACATGGAGCTGAGGAGCCTGAGATCTGACG ACACGGCCGTGTATTACTGTGCGAGAGACTACTA CTACGGTATGGACGTCTGGGGCCAAGGGACCAC GGTCACCGTCTCCTCA |
| | | | SEQ ID NO: 27179 | SEQ ID NO: 31185 |

FIGURE 50

| | | | | |
|---|---|---|---|---|
| | | AA | DIVMTQSPDSLAVSLGERATINCKSSQSILLSSNN KNYLAWHQQKPGQPPKLLIYWASTRESGVPDRF SGSGSGTDFTLTISSLQAEDVAVYYCLQYFSTPW TFGQGTKVEIK | QVQLVQSGAEVKKPGASVKVSCKASGFTFTSYGIN WVRQAPGQGLEWMGWISVYSGNTNYAQKLQGRV TMTTDTSTSTAYMELRSLRSDDTAVYYCARDYYY GMDVWGQGTTVTVSS |
| | | | SEQ ID NO: 27180 | SEQ ID NO: 31186 |
| iPS:436522 | 21-225_223H10 | NA | GACATCCAGATGACCCAGTCTCCATCCTCACT GTCTGCATCTGTAGGAGACAGAGTCACCATCA CTTGTCGGGCGAGTCAGGGCATTAGTAATTAT TTGGCCTGGTTTCAGCAGAAACCAGGGAAAGC CCCTAAGTCCCTGATCTACGCTGCATCCAATTT GCAAAGTGGGGTCCCATCAAAGTTCAGCGGCA GTGGGTCTGGGACAGATTTCACTCTCACCATC AGCAGCCTGCAGCCTGAAGATTTTGCAACTTA TTACTGCCTACATTATCTTAATTACCCACTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACCATTCCAAGAACACGC TGTATCTGCAAATGAACAGCCTGAGAGCCGAGG ACACGGCTGTGTATTACTGTGCGAGAGATCGGGA TGTCGGGTACAACGGTATGGACGTCTGGGGCCA AGGGACCACGGTCACCGTCTCCTCA |
| | | | SEQ ID NO: 27181 | SEQ ID NO: 31187 |
| | | AA | DIQMTQSPSSLSASVGDRVTITCRASQGISNYLA WFQQKPGKAPKSLIYAASNLQSGVPSKFSGSGS GTDFTLTISSLQPEDFATYYCLHYLNYPLTFGGG TKVEIK | QVQLVESGGGVVQPGRSLRLSCAASGFTFSSYGMH WVRQAPGKGLEWVAVIWYDGSNKNYADSVKGRF TISRDHSKNTLYLQMNSLRAEDTAVYYCARDRDV GYNGMDVWGQGTTVTVSS |
| | | | SEQ ID NO: 27182 | SEQ ID NO: 31188 |

FIGURE 50

| iPS:436526 | 21-225_224A1 | NA | GACATCCAGATGACCCAGTCTCCATCCTCCCT GTCTGCATCTGTAGGAGACAGAGTCACCATCA CTTGCCGGGCAAGTCAGGGCATTGAAAATGAT TTAGGCTGGTATCAGCAGAAGCCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGTGGGACCAAGGTGGAGATCAA A | GAGGTGCAGGTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGCAGAGGCGG CAGCACATACTACGCAGACGCCGTGAAGGGCCG GTTCACCATCTCCAGAGACAATTCCAAGAACACG CTGTATCTGCAAATGAACAGCCTGAGAGCCGAG GACACGGCCGTATATTACTGCGCGAAAGGCTCCT ACGATAGTAGTGGTTATTACCACTACTTAGACCA CTGGGGCCAGGGAACCCTGGTCACCGTCTCCTCA |
| | | | SEQ ID NO: 27183 | SEQ ID NO: 31189 |
| | | AA | DIQMTQSPSSLSASVGDRVTITCRASQGIENDLG WYQQKPGKAPKRLIYAASSLQSGVPSRFSGSGS GTEFTLTISSLQPEDFATYYCLQHNSYPLTFGGG TKVEIK | EVQVLESGGGLVQPGGSLRLSCAASGFTFSSYAMS WVRQAPGKGLEWVSAISGRGGSTYYADAVKGRFT ISRDNSKNTLYLQMNSLRAEDTAVYYCAKGSYDSS GYYHYLDHWGQGTLVTVSS |
| | | | SEQ ID NO: 27184 | SEQ ID NO: 31190 |
| iPS:436528 | 21-225_224B1 | NA | GACATCCAGATGACCCAGTCTCCATCCTCCCT GTCTGCATCTGTGGGAGACAGAGTCACCATCA CTTGCCGGGCAAGTCAGGGCATTAGTAATGAT TTAGGCTGGTATCAGCAGAAGCCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATCACTGTCTACAGCATAATAGTTATCCTCCT ACTTTCGGCGGAGGGACCAAGGTGGAGATCA AA | GAGGTGCAGGTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AACACATACTACGCAGACTCCGTGAAGGGCCGG TTCACCATCTCCAGAGACAATTCCAAGAACACGC TGTATCTGCACATGAACAGCCTGAGAGCCGAGG ACACGGCCGTATATTACTGTGCGGGAGAGGGGG GCTACTACTATTACTACGGTGTGGACGTCTGGGG CCAAGGGACCACGGTCACCGTCTCCTCA |
| | | | SEQ ID NO: 27185 | SEQ ID NO: 31191 |

FIGURE 50

| | | | | |
|---|---|---|---|---|
| | | AA | DIQMTQSPSSLSASVGDRVTITCRASQGISNDLG WYQQKPGKAPKRLIYAASSLQSGVPSRFSGSGS GTEFTLTISSLQPEDFATYHCLQHNSYPPTFGGGT KVEIK | EVQVLESGGGLVQPGGSLRLSCAASGFTFSSYAMS WVRQAPGKGLEWVSAISGSGGNTYYADSVKGRFT ISRDNSKNTLYLHMNSLRAEDTAVYYCAGEGGYY YYYGVDVWGQGTTVTVSS |
| | | | SEQ ID NO: 27186 | SEQ ID NO: 31192 |
| iPS:436534 | 21-225_224F1 | NA | GCCATCCAGATGACCCAGTCTCCATCCTCCCT GTCTGCATCTGTAGGAGACAGAGTCACCATCA CTTGCCGGGCAAGTCAGGGCATTAGAGATGAT TTAGGCTGGTATCAGCAGAAACCAGGGAAAG CCCCTAAGCGCCTGATCTATGCTGCATCCAGT TTGCAAAGTGGGGTCCCGTCAAGGATCAGCGG CAGTGGATCTGGGACAGAATTCACTCTCACAA TCAGCAGCCTGCAGCCTGAAGATTTTGCAATT TATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATATCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAGCAGCAACTGGTACGACTACGGTATGGACGTC TGGGGCCAAGGGACCACGGTCACCGTCTCCTCA |
| | | | SEQ ID NO: 27187 | SEQ ID NO: 31193 |
| | | AA | AIQMTQSPSSLSASVGDRVTITCRASQGIRDDLG WYQQKPGKAPKRLIYAASSLQSGVPSRISGSGSG TEFTLTISSLQPEDFAIYYCLQHYSYPRTFGQGTK VEIK | QVQLVESGGGVVQPGRSLRLSCAASGFTFSSYIMH WVRQAPGKGLEWVAVIWYDGSNKYYADSVKGRF TISRDNSKNTLYLQMNSLRAEDTAVYYCARERYSS NWYDYGMDVWGQGTTVTVSS |
| | | | SEQ ID NO: 27188 | SEQ ID NO: 31194 |

FIGURE 50

| iPS:436536 | 21-225_224G1 | NA | GATATTGTGATGACCCAGACTCCACTCTCTCT GTCCGTCACCCCTGGACAGCCGGCCTCCATCT CCTGCAAGTCTGGTCAGAGCCTCCTGCATAGT GATGGAAAGACCTTTTTGTCTTGGTACCTGCA GAAGCCAGGCCAGCCTCCACAGCTCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTTTTACTGCATGCAAAG TACACAGCTTCCTCGGACGTTCGGCCAAGGGA CCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAACTATGCACAGAAGTTTCAGGGCAG AGTCACCATGACCAGGGACACGTCCATCAGCAC AGCCTACATGGAACTGAGCAGGCTGAGATTTGA CGACACGGCCGTGTTTTACTGTGCGAGAGATTGG GGTGGCTACAGTTCTTACTACTACGGTATGGACG TCTGGGGCCAAGGGACCACGGTCACCGTCTCTTC A |
| | | | SEQ ID NO: 27189 | SEQ ID NO: 31195 |
| | | AA | DIVMTQTPLSLSVTPGQPASISCKSGQSLLHSDG KTFLSWYLQKPGQPPQLLIYEISNRFSGVPDRFSG SGSGTDFTLKISRVEAEDVGIFYCMQSTQLPRTF GQGTKVEIK | QVQLVQSGAEVKKPGASVKVSCKASGYTFTGYYIH WVRQAPGQGLEWMGWINPYSGDTNYAQKFQGRV TMTRDTSISTAYMELSRLRFDDTAVFYCARDWGG YSSYYYGMDVWGQGTTVTVSS |
| | | | SEQ ID NO: 27190 | SEQ ID NO: 31196 |
| iPS:436538 | 21-225_224C3 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGGGAAAG CCCCTAAGCGCCTGATCTATGCTACATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGCTGCAGCTGCAGGAGTCGGGCCCAGGACTG GTGAAGTCTTCGGAGACCCTGTCCCTCACCTGCA CTGTCTCTGGCGGCTCCATCAGCAGAAGTAGTTA CTACTGGGGCTGGATCCGCCAGCCCCCAGGGAA GGGGCCTGGAGTGGATTGGGAATATCTATTATAGT GGGAGCACCTACTACAACCCGTCCCTCAAGAGTC GAGTCACCATATCCGTAGACACGTCCAAGAACC AGTTCTCCCTGAAGCTGAGCTCTGTGACCGCCGC AGACACGGCTGTGTATTACTGTGCGAGACAGGGT CGGGACTGGGGTGTTGACTACGGGGGCCAGGGA ACCCTAGTCACCGTCTCCTCA |
| | | | SEQ ID NO: 27191 | SEQ ID NO: 31197 |

FIGURE 50

|  |  | AA | DIQMTQSPSSLSASVGDRVTITCRASQDIRNDLG WYQQKPGKAPKRLIYATSSLQSGVPSRFSGSGSG TEFTLTISSLQPEDFATYYCLQHNSYPLTFGGGT KVEIK | QLQLQESGPGLVKSSETLSLTCTVSGGSISRSSYYW GWIRQPPGKGLEWIGNIYYSGSTYYNPSLKSRVTIS VDTSKNQFSLKLSSVTAADTAVYYCARQGRDWGV DYGGQGTLVTVSS |
|  |  |  | SEQ ID NO: 27192 | SEQ ID NO: 31198 |
| iPS:436540 | 21-225_224F3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAACAGAAACCAGGGAAAG CCCCTGAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTTCAGCATTATAATTACCCTCGG GCGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCAGCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAGCAGCAGCTGGTACGACTACGGTATGGACGTC TGGGGCCAAGGGACCACGGTCACCGTCTCCTCA |
|  |  |  | SEQ ID NO: 27193 | SEQ ID NO: 31199 |
|  |  | AA | DIQMTQSPSSLSASVGDRVTITCRASQGIRNDLG WYQQKPGKAPERLIYAASSLQSGVPSRFSGSGSG TEFTLTISSLQPEDFATYYCLQHYNYPRAFGQGT KVEIK | QVQLVESGGGVVQPGRSLRLSCAASGFSFSSYGMH WVRQAPGKGLEWVAVIWYDGSNKYYADSVKGRF TISRDNSKNTLYLQMNSLRAEDTAVYYCARERYSS SWYDYGMDVWGQGTTVTVSS |
|  |  |  | SEQ ID NO: 27194 | SEQ ID NO: 31200 |

FIGURE 50

| iPS:436544 | 21-225_224H5 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATTTCAACTACTTAACTTGGTATCA GCAGAAACCAGGACAGCCTCCTAAGTTGCTCA TTTACTGGGCATCTACCCGGGAATCCGGGGTC CCTGACCGATTCAGTGGCAGCGGGTCTGGGAC AGATTTCACTCTCACCATCAACAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGTTCCAG TGGCTGGAACTGGTTCGACCCCTGGGGCCAGGG AACCCTGGTCACCGTCTCCTCA |
|---|---|---|---|---|
| | | | SEQ ID NO: 27195 | SEQ ID NO: 31201 |
| | | AA | DIVMTQSPDSLAVSLGERATINCKSSQSVLYSSN NFNYLTWYQQKPGQPPKLLIYWASTRESGVPDR FSGSGSGTDFTLTINSLQAEDVAVYYCQQYYSTP PTFGQGTKVEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCASSSGW NWFDPWGQGTLVTVSS |
| | | | SEQ ID NO: 27196 | SEQ ID NO: 31202 |
| iPS:436546 | 21-225_224D6 | NA | GACATCCAGATGACCCAGTCTCCATCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCGTGG ACGTTCGGCCCAGGGACCAAGGTGGAAATCA AA | GAGGTGCAGGTGTTGGAGTCTGGGGGAGGCTTG GTACAGCCTGGGGGGTCCCTGAGACTCTCCTGTG CAGCCTCTGGATCCACCTTTAGCAGCGATGCCAT GAGCTGGGTCCGCCAGGCTCCAGGGAAGGGGCT GGAGTGGGTCTCAGCTATTAGTGGTAGTGGTGAT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAAGTCTATAGT GCCTACGATTCTCACTGGTTCGACCCCTGGGGCC AGGGAACCCTGGTCACCGTCTCCTCA |
| | | | SEQ ID NO: 27197 | SEQ ID NO: 31203 |

FIGURE 50

| | | | | |
|---|---|---|---|---|
| | | AA | DIQMTQSPSSVSASVGDRVTITCRASQGISSWLA WYQQKPGKAPKLLIYAASSLQSGVPSRFSGSGS GTDFTLTISSLQPEDFATYYCQQANSFPWTFGPG TKVEIK | EVQVLESGGGLVQPGGSLRLSCAASGSTFSSDAMS WVRQAPGKGLEWVSAISGSGDNTFYADSVKGRFTI SRDNSKNTLYLQMNSLRAEDTAVYYCAKVYSAYD SHWFDPWGQGTLVTVSS |
| | | | SEQ ID NO: 27198 | SEQ ID NO: 31204 |
| iPS:436548 | 21-225_224A7 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTGTATTGGTTCCTGCA GAAGCCAGGCCAGCCTCCACAGCTCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAACTATGCACAGAAGTTTCAGGGCAG GGTCACCATGACCAGGGACACGTCCATCACCAC AGCCTACATGGAACTGAGCAGGCTGAGATTTGA CGACACGGCCGTGTGTTTTACTGTGCGAGAGATTGG GGTGGCTACAGTTCTTACTACTACGGTATGGACG TCTGGGGGCCAAGGGACCACGGTCACCGTCTCTTC A |
| | | | SEQ ID NO: 27199 | SEQ ID NO: 31205 |
| | | AA | DIVMTQTPLSLSVTPGQPASISCKSSQSLLHSDGK TFLYWFLQKPGQPPQLLIYEISNRFSGVPDRFSGS GSGTDFTLKISRVEAEDVGIYYCMQSTQLPRTFG QGTKVEIK | QVQLVQSGAEVKKPGASVKVSCKASGYTFTGYYIH WVRQAPGQGLEWMGWINPYSGDTNYAQKFQGRV TMTRDTSITTAYMELSRLRFDDTAVFYCARDWGG YSSYYYGMDVWGQGTTVTVSS |
| | | | SEQ ID NO: 27200 | SEQ ID NO: 31206 |

FIGURE 50

| iPS:436550 | 21-225_224D8 | NA | GACATCGTGATGACCCAGTCTCCAGACTCCCT GGCTGTTTCTCTGGGCGAGAGGGCCGCCATCA ACTGCAAGTCCAGCCAGAGTGTTTTATACAGC TCCAACAATAAGAACTACTTAGCTTGGTACCA GCAGAAACCAGGACAGCCTCCTAAGCTGCTCA TTTATTGGTCGTCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTTTAGTACTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGTTGTACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCTTATAGCAGT GGCTGGTACTACTTTGACTACTGGGGCCAGGGAA CCCTGGTCATCGTCTCCTCA |
|  |  |  | SEQ ID NO: 27201 | SEQ ID NO: 31207 |
|  |  | AA | DIVMTQSPDSLAVSLGERAAINCKSSQSVLYSSN NKNYLAWYQQKPGQPPKLLIYWSSTRKSGVPD RFSGSGSGTDFTLTISSLQAEDVAVYYCQQYFST PPTFGQGTKVEIK | QVQLVQSGAEVKKPGASVKVSCKASGYTFTNYDIN WVRQATGQGLEWMGWLYPNSGNTGYAQKFQGR VTMTRNTSISTAYMELSSLRSEDTAVYYCAYSSGW YYFDYWGQGTLVIVSS |
|  |  |  | SEQ ID NO: 27202 | SEQ ID NO: 31208 |
| iPS:436554 | 21-225_224C10 | NA | GACATCGTGATGACCCAGTCTCCAGACTCCCT GGCTGCGTCTCTGGGCGAGAGGGCCACCATCA CCTGCAAGTCCAGCCAGAGTGTTTTATACAAT TCCAACAATAAGAACTACTTAGCTTGGTACCA GCAGAAACCAGGACAGCCTCCTAAGCTGCTCA TTTACTGGGCATCTACCCGGGAGTCCGGGGTC CCTGACCGATTCAGTGGCAGCGGGTCTGGGAC AGATTTCACTCTCACCATCAGCAGCCTGCAGG CTGAAGATGTGGCAATTTATTACTGTCAACAA TATTATATTAATCCGTGCAGTTTTGGCCAGGG GACCAGGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATCCACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCTATAGCAGT GGCTGGTACAAGTTTGACTACTGGGGCCAGGGA ACCCTGGTCACCGTCTCCTCA |
|  |  |  | SEQ ID NO: 27203 | SEQ ID NO: 31209 |

FIGURE 50

| | | | | |
|---|---|---|---|---|
| | | AA | DIVMTQSPDSLAASLGERATITCKSSQSVLYNSN NKNYLAWYQQKPGQPPKLLIYWASTRESGVPD RFSGSGSGTDFTLTISSLQAEDVAIYYCQQYYINP CSFGQGTRLEIK | QVQLVQSGAEVKKPGASVKVSCKASGSTFTNYDIN WVRQATGQGLEWMGWMHPNSGNTGYAQKFQGR VTMTRNTSISTAYMELSSLRSEDTAVYYCAYSSGW YKFDYWGQGTLVTVSS |
| | | | SEQ ID NO: 27204 | SEQ ID NO: 31210 |
| IPS:436556 | 21-225_224D10 | NA | GACATCCTGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCGCGGTTCAGCGG CAGTGGATCTGGGACAGAATTCACTCTCACAA TCAGCAGCCTGCAGCCTGAAGATTTTGCAACT TATTACTGTCTACAACATAATAGTTACCCGCTC ACTTTTGGCGGAGGGACCAAGGTGGAGATCA AA | CAGGTGCAGCTGGTGGAGTCTGGGGGAGGCCTG GTCCAGCCTGGAAAGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGAGGGAAG TAATAAATACTATGTAGACTCCGTGAGGGGCCGA TTCACCATCTCCAGAGACAATTCCAAGAGTACGC TGTATCTGCAAATGAACAGCCTGCGAGCCGAGG ACACGGCTGTGTATTACTGTGTGAGAGAGCTAGG CTTCCAGTCTGACTACTGGGGCCAGGGAACCCCG GTCACCGTCTCCTCA |
| | | | SEQ ID NO: 27205 | SEQ ID NO: 31211 |
| | | AA | DILMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGLVQPGKSLRLSCAASGFTFSSYGMH WVRQAPGKGLEWVAVIWYEGSNKYYVDSVRGRF TISRDNSKSTLYLQMNSLRAEDTAVYYCVRELGFQ SDYWGQGTPVTVSS |
| | | | SEQ ID NO: 27206 | SEQ ID NO: 31212 |

FIGURE 50

| iPS:436558 | 21-225_224C11 | NA | GATTTTGTGATGACCCAGACTCCACTCTCTCTG TCCGTCACCCCTGGACAGCCGGCCTCCATCTC CTGCAAGTCTAGTCAGAGCCTCCTGCATAGTG ATGGAAAGACCTTTTTGTATTGGTACCTGCAG AAGCCAGGCCAGCCTCCACAGCTCCTGATCTA TGAAATTTCCAACCGGTTCTCTGGAGTGCCAG ATAGGTTCAGTGGCAGCGGGTCAGGGACAGA TTTCACACTGAAAATCAGCCGGGTGGAGGCTG AGGATGTTGGGATTTATTACTGCATGCAAAGT ACACAGCTTCCTCGGACGTTCGGCCAAGGGAC CAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAACTATGCACAGAAGTTTCAGGGCAG GGTCACCATGACCAGGGACACGTCCATCAGCAC AGCCTACATGGAACTGAGGAGGCTGAGATTTGA CGACACGGCCGTGTTTTACTGTGCGAGAGATTGG GGTGGCTACAGTTCTTACTACTTCGGTATGGACG TCTGGGGCCAAGGGACCACGGTCACCGTCTCTTC A |
| | | | SEQ ID NO: 27207 | SEQ ID NO: 31213 |
| | | AA | DFVMTQTPLSLSVTPGQPASISCKSSQSLLHSDG KTFLYWYLQKPGQPPQLLIYEISNRFSGVPDRFS GSGSGTDFTLKISRVEAEDVGIYYCMQSTQLPRT FGQGTKVEIK | QVQLVQSGAEVKKPGASVKVSCKASGYTFTGYYIH WVRQAPGQGLEWMGWINPYSGDTNYAQKFQGRV TMTRDTSISTAYMELRRLRFDDTAVFYCARDWGG YSSYYFGMDVWGQGTTVTVSS |
| | | | SEQ ID NO: 27208 | SEQ ID NO: 31214 |
| iPS:436560 | 21-225_224F11 | NA | AACATCGTGATGACCCAGTCTCCAGACTCCCT GGCTGTGTCTCTGGACGAGAGGGGCCACCATCA ACTGCAAGTCCAGCCAGAGTGTTTTATCCAGC TCCAACAATCACAACTACTTAGCTTGGTACCA GCAGAAACCAGGACAGCCTCCTAAGATGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATACTACTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCCTATAGCAG TGGCTGGTACAAGTTTGACTACTGGGGCCAGGGA ACCCTGGTCACCGTCTCCTCA |
| | | | SEQ ID NO: 27209 | SEQ ID NO: 31215 |

FIGURE 50

| | | | | |
|---|---|---|---|---|
| | | AA | NIVMTQSPDSLAVSLDERATINCKSSQSVLSSSN NHNYLAWYQQKPGQPPKMLIYWASTRESGVPD RFSGSGSGTDFTLTISSLQAEDVAVYYCQQYYTT PCSFGQGTKLEIK | QVQLVQSGAEVKKPGASVKVSCKASGYTFTNYDIN WVRQATGQGLEWMGWMNPNSGNTGYAQKFQGR VTMTRNTSISTAYMELSSLRSEDTAVYYCAYSSGW YKFDYWGQGTLVTVSS |
| | | | SEQ ID NO: 27210 | SEQ ID NO: 31216 |
| iPS:436562 | 21-225_224H11 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTGTATTGGTACCTGCA GAAGCCAGGCCAGCCTCCACAGCTCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGACCCCTGGACAAGGGC TTGAGTGGATGGGATGGATCAACCCTTACAGTGG TGACACAAACTATGCACAGAAGTTTCAGGGCAG GGTCACCATGACCAGGGACACGTCCATCAGCAC AGCCTACATGGAACTGCGCAGGCTGAGATTTGAC GTGGCTACAGTTCTTACTACTACGGTATGGACGT CTGGGGCCAAGGGACCACGGTCACCGTCTCTTCA |
| | | | SEQ ID NO: 27211 | SEQ ID NO: 31217 |
| | | AA | DIVMTQTPLSLSVTPGQPASISCKSSQSLLHSDGK TFLYWYLQKPGQPPQLLIYEISNRFSGVPDRFSGS GSGTDFTLKISRVEAEDVGIYYCMQSTQLPRTFG QGTKVEIK | QVQLVQSGAEVKKPGASVKVSCKASGYTFTGYYIH WVRQTPGQGLEWMGWINPYSGDTNYAQKFQGRV TMTRDTSISTAYMELRRLRFDDTAVFYCARDWGG YSSYYYGMDVWGQGTTVTVSS |
| | | | SEQ ID NO: 27212 | SEQ ID NO: 31218 |

FIGURE 50

| iPS:436564 | 21-225_225A1 | NA | GACATCCAGATGACCCAGTCTCCATCCTCCCT GTCTGCATCTGTAGGAGACAGAGTCACCATCA CTTGCCGGGCAAGTCAGGGCATTAGAGATGAT TTAGGCTGGTATCAGCAGATACCAGGGAAAGC CCCTAAGCGCCTGATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTGCAGCATTATAGTTACCCTCGGAC GTTCGGCCAAGGGACCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT CCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAGCAGTGGCTGGTACGACTACGGTATGGACGTC TGGGGCCAAGGGACCACGGTCACCGTCTCCTCA |
| | | | SEQ ID NO: 27213 | SEQ ID NO: 31219 |
| | | AA | DIQMTQSPSSLSASVGDRVTITCRASQGIRDDLG WYQQIPGKAPKRLIYAASSLQSGVPSRFSGSGSG TEFTLTISSLQPEDFATYYCLQHYSYPRTFGQGT KVEIK | QVQLVESGGGVVQPGRSLRLSCAASGFTFSDYVIH WVRQAPGKGLEWVAVIWYDGSNKYYADSVKGRF TISRDNSKNTLYLQMNSLRAEDTAVYYCARERYSS GWYDYGMDVWGQGTTVTVSS |
| | | | SEQ ID NO: 27214 | SEQ ID NO: 31220 |
| iPS:436568 | 21-225_225B3 | NA | GAAATTGTGCTGACGCAGTCTCCAGGCACCCT GTCTTTGTCTCCAGGGGAAAGAGCCACCCTCT CCTGCAGGGCCAGTCAGAATCTTAGCAGCAGC TACTTAGGCTGGTACCAGCAGAAACCTGGCCA GGCTCCCAGACTCCTCATCTATGATACATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGGAGTATGGTAGCTCACTC ATGTGCAGTTTTGGCCAGGGGACCAAGCTGGA GATCAAA | CAGGTGCAACTGGTGCAGTCTGGGGCTGAGGTG AAGAAGCCTGGGGCCTCAGTGAAGGTTTCCTGCA AGGCATCTGGATACACCTTCACCAGCTACTATAT GCACTGGGTGCGACAGGCCCCTGGACAAGGGGCT TGAGTGGATGGGAATAATCAACCCTAGTGGTGGT AGCACAAGCTACGCACAGAAGTTCCAGGGCAGA GTCACCATGACCAGGGACACGTCCACGAGCACA GTCTACATGGAGCTGAGCAGCCTGAGATCTGCGG ACACGGCCGTGTATTACTGTGCGAGGGATTTAGC AGCTCGTTCTTACTACTACTACTTCGGTATGGAC GTCTGGGGCCAAGGGGCCACGGTCACCGTCTCCT CA |
| | | | SEQ ID NO: 27215 | SEQ ID NO: 31221 |

FIGURE 50

| | | | | |
|---|---|---|---|---|
| | | AA | EIVLTQSPGTLSLSPGERATLSCRASQNLSSSYLG WYQQKPGQAPRLLIYDTSSRATGIPDRFSGSGSG TDFTLTISRLEPEDFAVYYCQEYGSSLMCSFGQG TKLEIK | QVQLVQSGAEVKKPGASVKVSCKASGYTFTSYYM HWVRQAPGQGLEWMGIINPSGGSTSYAQKFQGRV TMTRDTSTSTVYMELSSLRSADTAVYYCARDLAAR SYYYYFGMDVWGQGATVTVSS |
| | | | SEQ ID NO: 27216 | SEQ ID NO: 31222 |
| iPS:436570 | 21-225_225F4 | NA | GACATCGTGATGACCCAGTCTCCAGACTCCCT GGCTGTGTCTCTGGGCGAGAGGGCCACCATCA ACTGCAAGTCCAGCCAGAGTGTTTTATATAGC TCCAACAATAAGAACTACTTAGCTTGGTACCA GCAGAAACCAGGACAGCCTCCTAAACTACTCA TTTACTGGGCATCTACCCGGGAATCCGGGGTC CCTGACCGATTCACTGGCAGCGGGTCTGGGAC AGATTTCACTCTCACCATCAGCTGCGTGCAGC CGGAAGATGTGGCAGTTTATTACTGTCACCAA TATCATAATTCTCCTCCCACTTTCGGCCACGGG ACCGAAGTGGATATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAGCACAGGCTATGCACAGAAGTTCCAGGGCAG ACTCACCATGACCAGGAACACCTCCATAAGCAC AGTCTACATGGAACTGAACAGCCTGAGATCTGA GGACACGGCCGTGTATTATTGTGCGAGTAGCAGT GGCTGGTACTGGTTCGACCCCTGGGGCCAGGGA ACCCTGGTCACCGTCTCCTCA |
| | | | SEQ ID NO: 27217 | SEQ ID NO: 31223 |
| | | AA | DIVMTQSPDSLAVSLGERATINCKSSQSVLYSSN NKNYLAWYQQKPGQPPKLLIYWASTRESGVPD RFTGSGSGTDFTLTISCVQPEDVAVYYCHQYHN SPPTFGHGTEVDIK | QVQLVQSGAEVKKPGASVKVSCKASGYTFTNYDIN WVRQATGQGLEWMGWMHPNSGSTGYAQKFQGR LTMTRNTSISTVYMELNSLRSEDTAVYYCASSSGW YWFDPWGQGTLVTVSS |
| | | | SEQ ID NO: 27218 | SEQ ID NO: 31224 |

FIGURE 50

| iPS:436572 | 21-225_225G4 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTGTATTGGTACCTGCA GAAGCCAGGCCAGCCTCCACAGCTCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAACTATGCACAGAAGTTTCAGGGCAG GGTCACCATGACCAGGGACACGTCCATCAGCAC AGCCTACATGGAACTGCGCAGGCTGAGATTTGAC GACACGGCCGTCTTTTACTGTGCGAGAGATTGGG GTGGCTACAGTTCTTACTACTACGGTATGGACGT CTGGGGGCCAAGGGACCACGGTCACCGTCTCTTCA |
| | | | SEQ ID NO: 27219 | SEQ ID NO: 31225 |
| | | AA | DIVMTQTPLSLSVTPGQPASISCKSSQSLLHSDGK TFLYWYLQKPGQPPQLLIYEISNRFSGVPDRFSGS GSGTDFTLKISRVEAEDVGIYYCMQSTQLPRTFG QGTKVEIK | QVQLVQSGAEVKKPGASVKVSCKASGYTFTGYYIH WVRQAPGQGLEWMGWINPYSGDTNYAQKFQGRV TMTRDTSISTAYMELRRLRFDDTAVFYCARDWGG YSSYYYGMDVWGQGTTVTVSS |
| | | | SEQ ID NO: 27220 | SEQ ID NO: 31226 |
| iPS:436574 | 21-225_225F5 | NA | GACATCGTGATGACCCAGTCTCCAGACTCCCT GGCTGTGTCTCTGGGCGAGAGGGGCCACCATCA ACTGCAAGTCCAGCCAGAATGTTTTATACAAC TCCAACAATAACAACTACTTAGCTTGGTACCA GCAGAAACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTTCTGTCAGCAA TATTATAGTTCTCCTCCGACGTTCGGCCAAGG GACCAAGGTGGAAATCAAC | CAGGTGCAGCTGGTGCAGTCTGGGGGCTGAGGTG AAGAAGCCTAGGGCCTCAGTGAAGGTCTCCTGC AAGGCTTCTGGACACACCTTCACCAATTATGATA TCAACTGGGTGCGACAGGCCACTGGACAAGGGC TTGAGTGGATGGGATGGATGCATCCTAACAGTGG TAACACAGGCTTTGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCATATAGCAGT GGCTGGTACCGCTTTGACTACTGGGGCCAGGGAA CCCTGGTCACCGTCTCCTCA |
| | | | SEQ ID NO: 27221 | SEQ ID NO: 31227 |

FIGURE 50

| | | | | |
|---|---|---|---|---|
| | | AA | DIVMTQSPDSLAVSLGERATINCKSSQNVLYNSN NNNYLAWYQQKPGQPPNLLIYWASTRESGVPD RFSGSGSGTDFTLTISSLQAEDVAVYFCQQYYSS PPTFGQGTKVEIN | QVQLVQSGAEVKKPRASVKVSCKASGHTFTNYDIN WVRQATGQGLEWMGWMHPNSGNTGFAQKFQGR VTMTRNTSISTAYMELSSLRSEDTAVYYCAYSSGW YRFDYWGQGTLVTVSS |
| | | | SEQ ID NO: 27222 | SEQ ID NO: 31228 |
| IPS:436576 | 21-225_225B6 | NA | GACATCCAGATGACCCAGTCTCCATCCTCCCT GTCTGCATCTGTAGGAGACAGAGTCATCATCA CTTGCCGGGCAAGTCAGGGCATGAGAAAAGA TTTAGGCTGGTATCAGCAGAAACCAGGGAAA GCCCCTAAGCGCCTGATCTATGCTGCAACCAG TTTGCAAAGTGGGGTCCCATCAAGGTTCAGCG GCAGTGGATCTGGGACAGAATTCACTCTCACA ATCAGCAGCCTGCAGCCTGAAGATTTTGCAAC TTATTACTGTCTACAGCATAATAGTTATCCATT CACTTTCGGCCCTGGGACCAAAGTGGATATCA AA | CAGGTGCGGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGAAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAAGTGGG ATTCACTGAGGACTACTGGGGCCAGGGAACCCT GGTCACCGTCTCCTCA |
| | | | SEQ ID NO: 27223 | SEQ ID NO: 31229 |
| | | AA | DIQMTQSPSSLSASVGDRVIITCRASQGMRKDLG WYQQKPGKAPKRLIYAATSLQSGVPSRFSGSGS GTEFTLTISSLQPEDFATYYCLQHNSYPFTFGPGT KVDIK | QVRLVESGGGVVQPGRSLRLSCAASGFTFSDYGMH WVRQAPGKGLEWVAVIWYDENNKYYADSVKGRF TISRDNSKNTLYLQMNSLRAEDTAVYYCAREVGFT EDYWGQGTLVTVSS |
| | | | SEQ ID NO: 27224 | SEQ ID NO: 31230 |

2435

FIGURE 50

| iPS:436578 | 21-225_225D6 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTGCTGTCTTCAGCATAATACTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGAAAAT AATAAATACTATGCAGACTCCGTGAAGGGCCGA TTCACCATCTCCAGAGACAATTCCCAGAACACGC TGTATCTGCAAATGACCAGCCTGAGAGCCGAGG ACACGGCTGTGTATTACTGTGCGAGAGAAGTGGG ATTCACTGAGGACTACTGGGGCCAGGGAACCCT GGTCACCGTCTCCTCA |
| | | | SEQ ID NO: 27225 | SEQ ID NO: 31231 |
| | | AA | DIQMTQSPSSLSASVGDRVTITCRASQGIRNDLG WYQQKPGKAPKRLIYAASSLQSGVPSRFSGSGS GTEFTLTISSLQPEDFATYCCLQHNTYPFTFGPGT KVDIK | QVQLVESGGGVVQPGRSLRLSCAASGFTFSNYGM HWVRQAPGKGLEWVAVIWYDENNKYYADSVKGR FTISRDNSQNTLYLQMTSLRAEDTAVYYCAREVGF TEDYWGQGTLVTVSS |
| | | | SEQ ID NO: 27226 | SEQ ID NO: 31232 |
| iPS:436580 | 21-225_225E7 | NA | GAAATTGTGTTGACGCAGTCTCCAGGCACCCT GTCTTTGTCTCCAGGGGAAAGAGCCACCCTCT CCTGCAGGGCCAGTCAGAGTGTTTACAGCAGC TACTTAGCCTGGTACCAGCAGAAACCTGGCCA GGCTCCCAGGCTCCTCATCTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TATATTACTGTCAGCAGTATGGTACCTCACCTC GGACGTTCGGCCAAGGGACCAAGGTGGAAAT CAAA | CAGGTGCAGCTGCAGGAGTCGGGCCCAGGACTG GTGAAGCCTTCACAGACCCTGTCCCTCACCTGCA CTGTCTCTGGTAACTCCATCAGCAGTGGTCATTA CTACTGGAGCTGGATCCGCCAGCACCCAGGGAA GGGGCCTTGAGTGGATTGGGTTCATCTATTACACT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGAGGCC GGTGACTACGGCTACTACGGTATGGACGTCTGGG GCCAAGGGACCACGGTCACCGTCTCCTCA |
| | | | SEQ ID NO: 27227 | SEQ ID NO: 31233 |

FIGURE 50

| | | | | |
|---|---|---|---|---|
| | | AA | EIVLTQSPGTLSLSPGERATLSCRASQSVYSSYLA WYQQKPGQAPRLLIYGASSRATGIPDRFSGSGSG TDFTLTISRLEPEDFAVYYCQQYGTSPRTFGQGT KVEIK | QVQLQESGPGLVKPSQTLSLTCTVSGNSISSGHYYW SWIRQHPGKGLEWIGFIYYTGSTYYNPSLKSRVTIS VDTSKNQFSLKLSSVTAADTAVYYCAREAGDYGY YGMDVWGQGTTVTVSS |
| | | | SEQ ID NO: 27228 | SEQ ID NO: 31234 |
| IPS:436582 | 21-225_225F8 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATATATGCTGCATCCAGT TTGCTAGGTGGTGTCCCATCAAGATTCAGCGG CAGTGGATCTGGGACAGAATTCACTCTCACAA TCAGCAGCCTGCAGCCTGAAGATTTTGCAACT TATTACTGTCTACAACATAATAGTTACCCATTC ACTTTCGGCCCTGGGACCAAGGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGAAAAT AATAAATACTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAAGTGGGA TTTACTGAGGACTACTGGGGCCAGGGAACCCTGG TCACCGTCTCCTCA |
| | | | SEQ ID NO: 27229 | SEQ ID NO: 31235 |
| | | AA | DIQMTQSPSSLSASVGDRVTITCRASQGIRNDLG WYQQKPGKAPKRLIYAASSLLGGVPSRFSGSGS GTEFTLTISSLQPEDFATYYCLQHNSYPFTFGPGT KVDIK | QVQLVESGGGVVQPGRSLRLSCAASGFTFSSYGMH WVRQAPGKGLEWVAVIWYDENNKYYVDSVKGRF TISRDNSKNTLYLQMNSLRAEDTAVYYCAREVGFT EDYWGQGTLVTVSS |
| | | | SEQ ID NO: 27230 | SEQ ID NO: 31236 |

FIGURE 50

| iPS:436584 | 21-225_225B9 | NA | GACATCGTGATGACCCAGTCTCCAGATTCCCT GGCTGTGTCTCTGGGCGAGAGGGCCACCATCA ACTGCAAGTCCAGCCAGAGTGTTTTATACAGC TCCAACAATAACAACTACTTAGCTTGGTACCA ACAAAAACCAGGACAGCCTCCTAAGCTGCTCA TTTTCTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAACAC TCCAAGAGTATTCCTGGTAAGTTTGGGCAGGG GATCAAACTGGAGATCCAA | CAGGTGCAGCTGGTGCAGTCTGGGACTGAGGTG AGGAAGCCTGGGGCCTCAGTGAAGGTCTCCTGC AAGGCTTCTGGATACACCTTCACCAATTATGATA TCAACTGGGTACGACAGGCCACTGGACAAAGGC TTGAGTGGATGGGATGGATGCACCCTAACAGTGG TAACACAGGCTATGCACAGAAGTTCCGGGGCAG AGTCACCATGACCAGGAACACCTCCATAAACAC AGCCTACATGGAGCTGAACAGCCTGAGATCTGA GGACACGGCCGTATATTATTGTGCATATAGCAGT GGCTGGACCCTTTTTGACTACTGGGGCCAGGGAA CCCTGGTCACCGTCTCCTCA |
| | | | SEQ ID NO: 27231 | SEQ ID NO: 31237 |
| | | AA | DIVMTQSPDSLAVSLGERATINCKSSQSVLYSSN NNNYLAWYQQKPGQPPKLLIFWASTRESGVPDR FSGSGSGTDFTLTISSLQAEDVAVYYCQHSKSIPG KFGQGIKLEIQ | QVQLVQSGTEVRKPGASVKVSCKASGYTFTNYDIN WVRQATGQRLEWMGWMHPNSGNTGYAQKFRGR VTMTRNTSINTAYMELNSLRSEDTAVYYCAYSSG WTLFDYWGQGTLVTVSS |
| | | | SEQ ID NO: 27232 | SEQ ID NO: 31238 |
| iPS:436586 | 21-225_225F11 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATTACAACTACTTAGCTTGGTACCA GCAGAGACCAGGACAGCCTCCTAAGCTGCTCA TTTACTGGGCTTCTACCCGGGAATCCGGGGTC CCTGACCGATTCAGTGGCAGCGGGTCTGGGCC AGATTTCACTCTCACCATCAGCAGCCTGCAGG CTGAAGATGTGGCAGTTTATTACTGTCAGCAA TATTATACTACTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAACAC AGCCTACATGGAGCTGAGCAGCCTGAGATCTGA GGACACGGCCGTGTATTACTGTGCGTATAGCAGT GGCTGGTACCGCTTTGACTACTGGGGCCAGGGAA CCCTGGTCACCGTCTCCTCA |
| | | | SEQ ID NO: 27233 | SEQ ID NO: 31239 |

FIGURE 50

| | | | | |
|---|---|---|---|---|
| | | AA | DIVMTQSPDSLAVSLGERATINCKSSQSVLYSSN NYNYLAWYQQRPGQPPKLLIYWASTRESGVPD RFSGSGSGPDFTLTISSLQAEDVAVYYCQQYYTT PPTFGQGTKVEIK | QVQLVQSGAEVKKPGASVKVSCKASGYTFTNYDIN WVRQATGQGLEWMGWMHPNSGNTGYAQKFQGR VTMTRNTSINTAYMELSSLRSEDTAVYYCAYSSGW YRFDYWGQGTLVTVSS |
| | | | SEQ ID NO: 27234 | SEQ ID NO: 31240 |
| iPS:436588 | 21-225_225F12 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATCAGAACTACTTAGCTTGGTACCA GCAGAAACCAGGACAGCCTCCTAGGCTGCTCA TTTACTGGACATCTACCCGGGAATCCGGGGTC CCTGACCGATTCAGTGGCAGCGGGTCTGGGAC AGATTTCACTCTCACCATCAGCAACCTGCAGG CTGAAGATGTGGCTGTTTATTACTGTCAGCAA TATTATATTACTCCGTGCAGTTTTGGCCAGGG GACCAAACTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCCATTATGATA TCAACTGGGTGCGACAGGCCACTGGACAAGGGC TTGAGTGGATGGGATGGATGCACCCAAACAGTG GTAACACAGGCTATGCACAGAAGTTCCAGGGCA GAGTCACCATGGCCAGGAACACCTCCATAAGCA CAGCCTACATGGAGCTGAGCAGCCTGAGATCTG AGGACACGGCCGTGTATTACTGTGCCTATAGCAG TGGCTGGTACAAGTTTGACTACTGGGGCCAGGGA ACCCTGGTCACCGTCTCCTCA |
| | | | SEQ ID NO: 27235 | SEQ ID NO: 31241 |
| | | AA | DIVMTQSPDSLAVSLGERATINCKSSQSVLYSSN NQNYLAWYQQKPGQPPRLLIYWTSTRESGVPDR FSGSGSGTDFTLTISNLQAEDVAVYYCQQYYITP CSFGQGTKLEIK | QVQLVQSGAEVKKPGASVKVSCKASGYTFTHYDIN WVRQATGQGLEWMGWMHPNSGNTGYAQKFQGR VTMARNTSISTAYMELSSLRSEDTAVYYCAYSSGW YKFDYWGQGTLVTVSS |
| | | | SEQ ID NO: 27236 | SEQ ID NO: 31242 |

FIGURE 50

| iPS:436590 | 21-225_225H12 | NA | GACATCGTGATGACCCAGTCTCCAGACTCCCT GGCTGTGTCTCTGGGCGAGAGGGCCACCATCA ACTGCAAGTCCAGCCAGAGTGTTTTATACAAC TCCAACAATAACAACTACTTAGCTTGGTACCA GCAGAAACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGA CTGAAGATGTGGCAGTTTATTACTGTCAGCAA TATTATATTACTCCGTGCAGTTTTGGCCAGGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCTATAGCAGT GGCTGGTACAAGTTTGACTACTGGGGCCAGGGA ACCCTGGTCACCGTCTCCTCA |
| | | | SEQ ID NO: 27237 | SEQ ID NO: 31243 |
| | | AA | DIVMTQSPDSLAVSLGERATINCKSSQSVLYNSN NNNYLAWYQQKPGQPPNLLIYWASTRESGVPD RFSGSGSGTDFTLTISSLQTEDVAVYYCQQYYIT PCSFGQGTKLEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YKFDYWGQGTLVTVSS |
| | | | SEQ ID NO: 27238 | SEQ ID NO: 31244 |
| iPS:436592 | 21-225_226B1 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAGGCCAGGCCAGCCTCCACAGCTCCTGATCA ATGAAGTTTCCATCCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTACTACTGCATGCAAAG TATACAGATTCCGTGGACGTTCGGCCAGGGGA CCAAGGTGGACATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAATTATATGGTATGATGGAGGT TATAAATACTATGCAGACTCCGTGAAGGGCCGAT TCACCATCTCCAGAGACAATTCCAAGAACACGCT GTTTCTGCAAATGAACAGCCTGAGAGCCGAGGA CACGGCTGTGTATTACTGTGCGAGAGATCACTAC GATTTTTGGAGTGGTTATCTTACCCACTGGGGCC AGGGAACCCTGGTCACCGTCTCCTCA |
| | | | SEQ ID NO: 27239 | SEQ ID NO: 31245 |

FIGURE 50

| | | | | |
|---|---|---|---|---|
| | | AA | DIVMTQTPLSLSVTPGQPASISCKSSQSLLHGDG KTYLYWYLQRPGQPPQLLINEVSIRFSGVPDRFS GSGSGTDFTLKISRVEAEDVGVYYCMQSIQIPWT FGQGTKVDIK | QVQLVESGGGVVQPGRSLRLSCAASGFTFSTYGMH WVRQAPGKGLEWVAIIWYDGGYKYYADSVKGRF TISRDNSKNTLFLQMNSLRAEDTAVYYCARDHYDF WSGYLTHWGQGTLVTVSS |
| | | | SEQ ID NO: 27240 | SEQ ID NO: 31246 |
| iPS:436594 | 21-225_226A5 | NA | GATATTGTGATGACCCAGACTCCACTCTCTCT GTCCGTCACCCCTGGACAGCCGGCCTCCATCT CCTGCAAGTCTAGTCAGAGCCTCCTACATGGT GATGGAAAGACCTATTTGTATTGGTACCTGCA GAAGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGATTCCGTGGACGTTCGGCCAAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTATGATGGAACT AATAAATACTATACAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGGTCA CGATTTTTGGAGTGGCTTTTTTTGTTACTGGGGCC AGGGAACCCTGGTCACCGTCTCCTCA |
| | | | SEQ ID NO: 27241 | SEQ ID NO: 31247 |
| | | AA | DIVMTQTPLSLSVTPGQPASISCKSSQSLLHGDG KTYLYWYLQKPGQPPQLLIYEVSNRFSGVPDRFS GSGSGTDFTLKISRVEAEDVGVYYCMQSIQIPWT FGQGTKVEIK | QVQLVESGGGVVQPGRSLRLSCAASGFTFSNYGM HWVRQAPGKGLEWVAIIWYDGTNKYYTDSVKGR FTISRDNSKNTLYLQMNSLRAEDTAVYYCAREGHD FWSGFFCYWGQGTLVTVSS |
| | | | SEQ ID NO: 27242 | SEQ ID NO: 31248 |

FIGURE 50

| iPS:436596 | 21-225_226C6 | NA | GACATCCAGATGACCCAGTCTCCATCCTCCCT GTCTGCATCTATAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGCTCCCATCAAGGTTCAGCGG CAGTGGATCTGGGACAGAATTCACTCTCACAA TCAGCAACCTGCAGCCTGAAGATTTTGCAACT TATTACTGTCTACAGCATTATAATTACCCTCGG GCGTTCGGCCAAGGGACCAAGGTGGAAATCC AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAACGAACAGCCTGAGAGCCGAGG ACACGGCTGTGTATTACTGTGCGAGAGAGAGGTA TAGCAGCAGCTGGTACGACTACGGTATGGACGTC TGGGGCCAAGGGACCACGGTCACCGTCTCCTCA |
| | | | SEQ ID NO: 27243 | SEQ ID NO: 31249 |
| | | AA | DIQMTQSPSSLSASIGDRVTITCRASQGIRNDLG WYQQKPGKAPKRLIYAASSLQSGLPSRFSGSGSG TEFTLTISNLQPEDFATYYCLQHYNYPRAFGQGT KVEIQ | QVQLVESGGGVVQPGRSLRLSCAASGFTFSDYGM HWVRQAPGKGLEWVAVIWYDGSNKYYADSVKGR FTISRDNSKNTLYLQTNSLRAEDTAVYYCARERYSS SWYDYGMDVWGQGTTVTVSS |
| | | | SEQ ID NO: 27244 | SEQ ID NO: 31250 |
| iPS:436598 | 21-225_226D6 | NA | GACATCGTGATGACCCAGTCTCCAGACTCCCT GGCTGTGTCTCTGGGCGAGAGGGCCACCATCA ACTGCAGGTCCAGCCAGAGTATTTTATACATC TCCAACAATAAGAACTACTTAGCTTGGTACCA GCAGAAACCAGGACAGCCTCCTAAGATGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTTCTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCTATAGCAGT GGCTGGTACAAGTTTGACTACTGGGGCCAGGGA ACCCTGGTCACCGTCTCCTCA |
| | | | SEQ ID NO: 27245 | SEQ ID NO: 31251 |

FIGURE 50

| | | | | |
|---|---|---|---|---|
| | | AA | DIVMTQSPDSLAVSLGERATINCRSSQSILYISNN KNYLAWYQQKPGQPPKMLIYWASTRESGVPDR FSGSGSGTDFTLTISSLQAEDVAVYYCQQYYSSP CSFGQGTKLEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YKFDYWGQGTLVTVSS |
| | | | SEQ ID NO: 27246 | SEQ ID NO: 31252 |
| iPS:436600 | 21-225_226F6 | NA | GACATCGTGATGACCCAGTCTCCAGACTCCCT GGCTGTGTCTCTGGGCGAGAGGGCCACCATCA ACTGCAAGTCCAGCCAGAGTATTTTATACAGC TCCAACAATTACAACTACTTAGCTTGGTACCA GCAGAAGCCAGGACAGCCTCCTAAGCTGCTCA TTTACTGGGCTTCTACCCGGGAATCCGGGGTC CCTGACCGATTCAGTGGCAGCGGGTCTGGGCC AGATTTCACTCTCACCATCAGCAGCCTGCAGG CTGAAGATGTGGCAGTTTATTACTGTCAGCAA TATTATACTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAACAC AGCCTACATGGAGCTGAGCAGCCTGAGATCTGA GGACACGGCCGTGTATTACTGTGCGTATAGCAGT GGCTGGTACCGCTTTGACTACTGGGGCCAGGGAA CCCTGGTCACCGTCTCCTCA |
| | | | SEQ ID NO: 27247 | SEQ ID NO: 31253 |
| | | AA | DIVMTQSPDSLAVSLGERATINCKSSQSILYSSNN YNYLAWYQQKPGQPPKLLIYWASTRESGVPDRF SGSGSGPDFTLTISSLQAEDVAVYYCQQYYTTPP TFGQGTKVEIK | QVQLVQSGAEVKKPGASVKVSCKASGYTFTNYDIN WVRQATGQGLEWMGWMHPNSGNTGYAQKFQGR VTMTRNTSINTAYMELSSLRSEDTAVYYCAYSSGW YRFDYWGQGTLVTVSS |
| | | | SEQ ID NO: 27248 | SEQ ID NO: 31254 |

FIGURE 50

| iPS:436602 | 21-225_226E7 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCAGCA GAAGCCAGGCCAGCCTCCACAGATCCTGATCT ATGAAGTTTCCAACCGGTTCTCTGGAGTGCCA GATAGGTTCAGTGGCAGCGGGTCAGGGACAG ACTTCACACTGAAAATCAGCCGGGTGGAGGCT GAGGATGTTGGGGTTTATTACTGCATGCAAAG TATACAGGTTCCGTGGACGTTCGGCCAAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAATGGGTGGCAATTATATGGTATGATGGAAGT AATAAATACTATGCAGACTCCGTGAAGGGCCGA TTCACCATCTCCAGAGACAATTTCAAGAACACGC TGTATCTGCAAATGCACAGCCTGAGAGCCGACG ACACGGCTGTGTATTACTGTGCGAGAGAGAATTA CGATTTTTGGAGTGGTTATTATGGCTACTGGGGC CAGGGAACCCTGGTCACCGTCTCCTCA |
| | | | SEQ ID NO: 27249 | SEQ ID NO: 31255 |
| | | AA | DIVMTQTPLSLSVTPGQPASISCKSSQSLLHGDG KTYLYWYQQKPGQPPQILIYEVSNRFSGVPDRFS GSGSGTDFTLKISRVEAEDVGVYYCMQSIQVPW TFGQGTKVEIK | QVQLVESGGGVVQPGRSLRLSCAASGFTFSTYGMH WVRQAPGKGLEWVAIIWYDGSNKYYADSVKGRFT ISRDNFKNTLYLQMHSLRADDTAVYYCARENYDF WSGYYGYWGQGTLVTVSS |
| | | | SEQ ID NO: 27250 | SEQ ID NO: 31256 |
| iPS:436604 | 21-225_226F7 | NA | GACATCCAGATGACCCAGTCTCCATCCTCCCT GTCTGCATCTGTTGGAGACACAGAGTCACCATCA CTTGCCGGGCAAGTCAGGGCATTGGGAATGAT TTAGGCTGGTATCAGCAGAAACCAGGGAAAG CCCCTAAGCGCCTGATCTATGCTGCCTCCAGTT TGCAAAGTGGGGTCCCATCAAGGTTCAGCGGC AGTGTATCTGGGACAGAATTCATTCTCACAAT CAGCAGCCTGCAGCCTGAAGATTTTGCAACTT ATTACTGTCTACATCATTATAGTTACCCTCGGA CGTTCGGCCAGGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAACAGCGGCTGGTACGACTACGGTTTGGACGTC TGGGGCCAAGGGACCACGGTCACCGTCTCCTCA |
| | | | SEQ ID NO: 27251 | SEQ ID NO: 31257 |

FIGURE 50

| | | | | |
|---|---|---|---|---|
| | | AA | DIQMTQSPSSLSASVGDRVTITCRASQGIGNDLG WYQQKPGKAPKRLIYAASSLQSGVPSRFSGSVS GTEFILTISSLQPEDFATYYCLHHYSYPRTFGQGT KVEIK | QVQLVESGGGVVQPGRSLRLSCAASGFTFSSYGMH WVRQAPGKGLEWVAIIWYDGSNKYYADSVKGRFT ISRDNSKNTLYLQMNSLRAEDTAVYYCARERYNSG WYDYGLDVWGQGTTVTVSS |
| | | | SEQ ID NO: 27252 | SEQ ID NO: 31258 |
| iPS:436606 | 21-225_226G8 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTGTATTGGTACCTGCA GAAGCCAGGCCAGCCTCCACAGCTCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAAGTATGCACAGAAGTTTCAGGGCAG GGTCACCATGACCAGGGACACGTCCATCAGCAC AGCCTACATGGAACTGAGCAGGCTGAGATTTGA CGACACGGCCGTGTGTTTTACTGTGCGAGAGATTGG GGTGGCTACAGTTCTTACTACTACGGTATGGACG TCTGGGGCCAAGGGACCACGGTCACCGTCTCTTC A |
| | | | SEQ ID NO: 27253 | SEQ ID NO: 31259 |
| | | AA | DIVMTQTPLSLSVTPGQPASISCKSSQSLLHSDGK TFLYWYLQKPGQPPQLLIYEISNRFSGVPDRFSGS GSGTDFTLKISRVEAEDVGIYYCMQSTQLPRTFG QGTKVEIK | QVQLVQSGAEVKKPGASVKVSCKASGYTFTGYYIH WVRQAPGQGLEWMGWINPYSGDTKYAQKFQGRV TMTRDTSISTAYMELSRLRFDDTAVFYCARDWGG YSSYYYGMDVWGQGTTVTVSS |
| | | | SEQ ID NO: 27254 | SEQ ID NO: 31260 |

FIGURE 50

| iPS:436608 | 21-225_226A9 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGAGGAAAG TAATAAATACTATACAGACTCCGTGAAGGGCCG ATTCACCATCTCCAGAGACAATTCCAAGAACACG CTGTTTCTGCAAATGAACAGCCTGAGAGCCGAGG ACACGGCTGTGTATTACTGTGCGAGAGAAGTGGG ATTCACTGAGGACTACTGGGGCCAGGGAACCCT GGTCACCGTCTCCTCA |
| | | | SEQ ID NO: 27255 | SEQ ID NO: 31261 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSNYGM HWVRQAPGKGLEWVAVIWYEESNKYYTDSVKGR FTISRDNSKNTLFLQMNSLRAEDTAVYYCAREVGF TEDYWGQGTLVTVSS |
| | | | SEQ ID NO: 27256 | SEQ ID NO: 31262 |
| iPS:436610 | 21-225_226F9 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTGTATTGGTACCTGCA GAAGCCAGGCCAGCCTCCACAGCTCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAAGTATGCACAGAAGTTTCAGGGCAG GGTCACCATGACCAGGGCCACGTCCATCAGCAC AGCCTACATGGAACTGAGCAGGCTGAGATTTGA CGACACGGCCGTGTTTTACTGTGCGAGAGATTGG GGTGGCTACAGTTCTTACTACTACGGTATGGACG TCTGGGGCCAAGGGACCACGGTCACCGTCTCTTC A |
| | | | SEQ ID NO: 27257 | SEQ ID NO: 31263 |

FIGURE 50

| | | | | |
|---|---|---|---|---|
| | | AA | DIVMTQTPLSLSVTPGQPASISCKSSQSLLHSDGK TFLYWYLQKPGQPPQLLIYEISNRFSGVPDRFSGS GSGTDFTLKISRVEAEDVGIYYCMQSTQLPRTFG QGTKVEIK | QVQLVQSGAEVKKPGASVKVSCKASGYTFTGYYIH WVRQAPGQGLEWMGWINPYSGDTKYAQKFQGRV TMTRATSISTAYMELSRLRFDDTAVFYCARDWGG YSSYYYGMDVWGQGTTVTVSS |
| | | | SEQ ID NO: 27258 | SEQ ID NO: 31264 |
| iPS:436612 | 21-225_226H9 | NA | GATATTGTGATGACCCAGACTCCACTCTCTCT GTCCGTCATCCCTGGACAGCCGGCCTCCATCT CCTGCAAGTCTAGTCAGAGCCTCCTGCATAGT GATGGAAAGACCTTTTTGTATTGGTACCTGCA GAGGCCAGGCCAGCCTCCACAGCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAACTCTGCACAGAAGTTTCAGGGCCG GGTCACCATGACCAGGGACACGTCCATCAGCAC AGCCTACATGGAACTGAGCAGGCTGAGATTTGA CGACACGGCCGTGTTTTACTGTGCGAGAGATTGG GGTGGCTACAGTTCTTACTACTACGGTATGGACG TCTGGGGCCAAGGGACCACGGTCACCGTCTCTTC A |
| | | | SEQ ID NO: 27259 | SEQ ID NO: 31265 |
| | | AA | DIVMTQTPLSLSVIPGQPASISCKSSQSLLHSDGK TFLYWYLQRPGQPPQLLIYEVSNRFSGVPDRFSG SGSGTDFTLKISRVEAEDVGIYYCMQSTQLPRTF GQGTKVEIK | QVQLVQSGAEVKKPGASVKVSCKASGYTFTGYYIH WVRQAPGQGLEWMGWINPYSGDTNSAQKFQGRV TMTRDTSISTAYMELSRLRFDDTAVFYCARDWGG YSSYYYGMDVWGQGTTVTVSS |
| | | | SEQ ID NO: 27260 | SEQ ID NO: 31266 |

FIGURE 50

| iPS:436614 | 21-225_226F10 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTGTATTGGTACCTGCA GAAGCCAGGCCAGCCTCCACAGCTCCTGATCT ATGAAATTTCCAACCGGTTCTCTGGGGTGCCA GATAGGTTCAGTGGCAGCGGGTCAGGGACAG ATTTCACACTGAAAATCAGCCGAGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGGTGAGGTG AAGAAGCTGAGGGCCTCAGTGAAGGTCTCCTGC AAGGCTTCTGGATACACCTTCACCGGCTATTATA TACACTGGGTGCGACAGGCCCCTGGACAAGGGC TTGAGTGGATGGGATGGATCAACCCATACAGTGG TGACACAAACTATGCACAGAAGTTTCAGGGCAG GGTCACCATGACCAGGGACACGTCCGTCAGCAC AGCCTACATGGAACTGAGCAGGTTGAGATTGGA CGACACGGCCGTGTTTTACTGTGCGAGAGATTGG GGTGGCTACAGTTCTTACTACTACGGTATGGACG TCTGGGGCCAAGGGACCCCGGTCACCGTCTCTTC A |
| | | | SEQ ID NO: 27261 | SEQ ID NO: 31267 |
| | | AA | DIVMTQTPLSLSVTPGQPASISCKSSQSLLHSDGK TFLYWYLQKPGQPPQLLIYEISNRFSGVPDRFSGS GSGTDFTLKISRVEAEDVGIYYCMQSTQLPRTFG QGTKVEIK | QVQLVQSGGEVKKLRASVKVSCKASGYTFTGYYIH WVRQAPGQGLEWMGWINPYSGDTNYAQKFQGRV TMTRDTSVSTAYMELSRLRLDDTAVFYCARDWGG YSSYYYGMDVWGQGTPVTVSS |
| | | | SEQ ID NO: 27262 | SEQ ID NO: 31268 |
| iPS:436616 | 21-225_226D11 | NA | GACATCGTGATGACCCAGTCTCCAGACTCCCT GGCTGTGTCTCTGGGCGAGAGGGGCCACCATCA ACTGCAAGTCCAGCCAGAATGTTTTACACAGC TCCAACAGTAATAACTACTTAGTTTGGTACCA GCAGAAACCAGGACAGCCTCCTAAACTGCTCA TTTACTGGGCATCTACCCGGGAATCCGGGGTC CCTGACCGATTCAGAGGCAGCGGGTCTGGGAC AGATTTCACTCTCACCATCAGCAGCCTGCAGG CTGAAGATGTGGCAGTTTATTACTGTCAGCAA TATTATAAAACTCCGTGGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AGGTCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCTATAGCAGT GGCTGGTACTACTTTGACTACTGGGGCCAGGGAA CCCTGGTCACCGTCTCCTCA |
| | | | SEQ ID NO: 27263 | SEQ ID NO: 31269 |

FIGURE 50

| | | | | |
|---|---|---|---|---|
| | | AA | DIVMTQSPDSLAVSLGERATINCKSSQNVLHSSN SNNYLVWYQQKPGQPPKLLIYWASTRESGVPDR FRGSGSGTDFTLTISSLQAEDVAVYYCQQYYKTP WTFGQGTKVEIK | QVQLVQSGAEVKKPGASVKVSCRSSGYTFTNYDIN WVRQATGQGLEWMGWMHPNSGNTGYAQKFQGR VTMTRNTSISTAYMELSSLRSEDTAVYYCAYSSGW YYFDYWGQGTLVTVSS |
| | | | SEQ ID NO: 27264 | SEQ ID NO: 31270 |
| iPS:436618 | 21-225_226E11 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTGTATTGGTACCTGCA CAAGCCAGGCCAGCCTCCACACCTCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAACTATGCACAGAAGTTTCAGGGCAG GGTCACCATGACCAGGGACACGTCCATCAGCAC AGCCTACATGGAACTGAGCAGGCTGAGATTTGA CGACACGGCCGTGTTTTACTGTGCGAGAGATTGG GGTGGCTACAGTTCTTACTACTACGGTATGGACG TCTGGGGCCAAGGGACCACGGTCACCGTCTCTTC A |
| | | | SEQ ID NO: 27265 | SEQ ID NO: 31271 |
| | | AA | DIVMTQTPLSLSVTPGQPASISCKSSQSLLHSDGK TFLYWYLHKPGQPPHLLIYEISNRFSGVPDRFSGS GSGTDFTLKISRVEAEDVGIYYCMQSTQLPRTFG QGTKVEIK | QVQLVQSGAEVKKPGASVKVSCKASGYTFTGYYIH WVRQAPGQGLEWMGWINPYSGDTNYAQKFQGRV TMTRDTSISTAYMELSRLRFDDTAVFYCARDWGG YSSYYYGMDVWGQGTTVTVSS |
| | | | SEQ ID NO: 27266 | SEQ ID NO: 31272 |

FIGURE 50

| iPS:436620 | 21-225_226H11 | NA | GACATCCAGATGACCCAGTCTCCATCCTCCCT GTCTGCATCTGTAGGAGACAGAGTCACCATCA CTTGCCGGACAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGTGG CAGTGGATCTGGGACAGATTTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAATCA AA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAACTGTGGCAT GCACTGGGTCCGCCAGGCTCCAGGCAAGGGGCT GGAGTGGGTGACAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGCTGTA TAGCAGCAGCTGGTACGACTACGGTTTGGACGTC TGGGGCCAAGGGACCACGGTCACCGTCTCCTCA |
| | | | SEQ ID NO: 27267 | SEQ ID NO: 31273 |
| | | AA | DIQMTQSPSSLSASVGDRVTITCRTSQGIRNDLG WYQQKPGKAPKRLIYAASSLQSGVPSRFSGSGS GTDFTLTISSLQPEDFATYYCLQHYSYPRTFGQG TKVEIK | QVQLVESGGGVVQPGRSLRLSCAASGFTFSNCGMH WVRQAPGKGLEWVTVIWYDGSNKYYADSVKGRF TISRDNSKNTLYLQMNSLRAEDTAVYYCARELYSS SWYDYGLDVWGQGTTVTVSS |
| | | | SEQ ID NO: 27268 | SEQ ID NO: 31274 |
| iPS:436622 | 21-225_226A12 | NA | GACATCGTGATGACCCAGTCTCCAGACTCCCT GGCTGTGTCTCTCGGCGAGAGGGCCACCATCA ACTGCAAGTCCAGCCAGAATGTTTTATACAGT TCCAACAATAACAACTACTTAGCTTGGTACCA GCAGACACCTGGACAGCCTCCTAAGCTGCTCT TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTAGTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCTTATAGCAGT GGCTGGTACTACTTTGACTACTGGGGCCAGGGAA CCCTGGTCACCGTCTCCTCA |
| | | | SEQ ID NO: 27269 | SEQ ID NO: 31275 |

FIGURE 50

| | | | | |
|---|---|---|---|---|
| | | AA | DIVMTQSPDSLAVSLGERATINCKSSQNVLYSSN NNNYLAWYQQTPGQPPKLLFYWASTRKSGVPD RFSGSGSGTDFTLTISSLQAEDVAVYYCQQYYSS PPTFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YYFDYWGQGTLVTVSS |
| | | | SEQ ID NO: 27270 | SEQ ID NO: 31276 |
| iPS:436624 | 21-225_226H12 | NA | GATATTGTGATGACCCAGACTCCACTCTCTCT GTCCGTCACCCCTGGACAGCCGGCCTCCATCT CCTGCAAGTCTAGTAAGACCCTCCTGCATAGT GATGGAAAGACCTTTTTGTATTGGTACCTGCA GAAGCCAGGCCAGCCTCCACAGCCCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAACTATGCACAGAAGTTTCAGGGCAG GGTCACCATGACCAGGGACACGTCCATCAGCAC AGCCTACATGGAACTGAGGAGGCTGAGATTTGA CGACACGGCCGTGTGTTTTACTGTGCGAGAGATTGG GGTGGCTACAGTTCTTACTACTACGGTATGGACG TCTGGGGCCAAGGGACCACGGTCACCGTCTCTTC A |
| | | | SEQ ID NO: 27271 | SEQ ID NO: 31277 |
| | | AA | DIVMTQTPLSLSVTPGQPASISCKSSSKTLLHSDGK TFLYWYLQKPGQPPQPLIYEISNRFSGVPDRFSGS GSGTDFTLKISRVEAEDVGIYYCMQSTQLPRTFG QGTKVEIK | QVQLVQSGAEVKKPGASVKVSCKASGYTFTGYYIH WVRQAPGQGLEWMGWINPYSGDTNYAQKFQGRV TMTRDTSISTAYMELRRLRFDDTAVFYCARDWGG YSSYYYGMDVWGQGTTVTVSS |
| | | | SEQ ID NO: 27272 | SEQ ID NO: 31278 |

FIGURE 50

| | | | | |
|---|---|---|---|---|
| iPS:436626 | 21-225_227C1 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTGTATTGGTACCTGCA GAAGCCAGGCCAGCCTCCACAGCTCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA CACACTGGGTGCGACAGGCCCCTGGACAAGGGC TTGAGTGGATGGGATGGATCAACCCTTACAGTGG TGGCACAAACTATGCACAGAAGTTTCAGGGCAG GGTCACCATGACCAGGGACACGTCCATCAGCAC AGCCTACATGGAACTGAGCAGGCTGAGATTTGA CGACACGGCCGTGTATTACTGTGCGAGAGATTGG GGTGGCTACAGTTCTTACTACTACGGTATGGACG TCTGGGGCCAAGGGACCACGGTCACCGTCTCTTC A |
| | | | SEQ ID NO: 27273 | SEQ ID NO: 31279 |
| | | AA | DIVMTQTPLSLSVTPGQPASISCKSSQSLLHSDGK TFLYWYLQKPGQPPQLLIYEISNRFSGVPDRFSGS GSGTDFTLKISRVEAEDVGIYYCMQSTQLPRTFG QGTKVEIK | QVQLVQSGAEVKKPGASVKVSCKASGYTFTGYYT HWVRQAPGQGLEWMGWINPYSGGTNYAQKFQGR VTMTRDTSISTAYMELSRLRFDDTAVYYCARDWG GYSSYYYGMDVWGQGTTVTVSS |
| | | | SEQ ID NO: 27274 | SEQ ID NO: 31280 |
| iPS:436628 | 21-225_227F2 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTGTATTGGTACCTGCA GAAGCCAGGCCAGCCTCCACAGCTCCTGATCT ATGAAATTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TACACAGCTTCCTCGGACGTTCGGCCAAGGGA CCAAGGTGGAAATCAAA | CAGGTGCACCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAAGTATGCACAGAAGTTTCAGGGCAG GGTCACCATGACCAGGGACACGTCCATCAGCAC AGCCTACATGGAACTGAGCAGGCTGAGATTTGA CGACACGGCCGTGTTTTACTGTGCGAGAGATTGG GGTGGCTACAGTTCTTACTACTACGGTATGGACG TCTGGGGCCAAGGGACCACGGTCACCGTCTCTTC A |
| | | | SEQ ID NO: 27275 | SEQ ID NO: 31281 |

FIGURE 50

| | | | | |
|---|---|---|---|---|
| | | AA | DIVMTQTPLSLSVTPGQPASISCKSSQSLLHSDGK TFLYWYLQKPGQPPQLLIYEISNRFSGVPDRFSGS GSGTDFTLKISRVEAEDVGIYYCMQSTQLPRTFG QGTKVEIK | QVHLVQSGAEVKKPGASVKVSCKASGYTFTGYYIH WVRQAPGQGLEWMGWINPYSGDTKYAQKFQGRV TMTRDTSISTAYMELSRLRFDDTAVFYCARDWGG YSSYYYGMDVWGQGTTVTVSS |
| | | | SEQ ID NO: 27276 | SEQ ID NO: 31282 |
| IPS:436630 | 21-225_227G3 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGCAGATTTTGCAACT TATTACTGTCTACACCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGTTGGAAGT AATCAATACTATGCAGACTCCGTGAAGGGCCGAT TCACCATCTCCAGAGACAATTCCAAGAACACGCT GTATCTGCAAATGAGCAGCCTGAGAGCCGAGGA CACGGCTGTGTATTACTGTGCGAGAGAAGTGGGA TTCACTGAGGACTACTGGGGCCAGGGAACCCTG GTCACCGTCTCCTCC |
| | | | SEQ ID NO: 27277 | SEQ ID NO: 31283 |
| | | AA | DIQMTQSPSSLSASVGDRVTITCRASQGIRNDLG WYQQKPGKAPKRLIYAASSLQSGVPSRFSGSGS GTEFTLTISSLQPADFATYYCLHHNSYPFTFGPGT KVDIK | QVQLVESGGGVVQPGRSLRLSCAASGFTFSSYGMH WVRQAPGKGLEWVAVIWYVGSNQYYADSVKGRF TISRDNSKNTLYLQMSSLRAEDTAVYYCAREVGFT EDYWGQGTLVTVSS |
| | | | SEQ ID NO: 27278 | SEQ ID NO: 31284 |

FIGURE 50

| iPS:436632 | 21-225_227E4 | NA | GACATCCTGATGACCCAGTCTCCATCTTCCGT GTCTGCATCTGTAGGAGACAGAGTCACCATCA CTTGTCGGGCGAGTCAGGGTATTATCAACTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAATTT ACTATTGTCAACAGGCTAACAGTTTCCCGTGG ACGTTCGGCCAAGGGACCAAGGTGGAAATCA AA | GAGGGGCAGCTATTGGAGTCTGGGGGAGGCTTG GTACAGCCTGGGGGGTCACTGAGACTCTCCTGTA CAGCCTCTGGATTCACCTTTAGCACTTTTGCCATG ACCTGGGTCCGCCAGGCTCCAGGGAGGGGGCTG GAGTGGGTCTCAGTTATTAGTGGTAGAGGTGGTA GCTCATTCTACGCAGACTCCGTGAAGGGCCGGTT CACCATCTCCAGAGACAATACCAAGAACACGCT GTATCTGCAAATGAACAGCCTGAGAGCCGAGGA CACGGCCGTATATTACTGTGCGAAAGATCAACTA TGGTTTGACTACTGGGGCCAGGGAACCCTGGTCA CCGTCTCCTCA |
| | | | SEQ ID NO: 27279 | SEQ ID NO: 31285 |
| | | AA | DILMTQSPSSVSASVGDRVTITCRASQGIINWLA WYQQKPGKAPKLLIYAASSLQSGVPSRFSGSGS GTDFTLTISSLQPEDFAIYYCQQANSFPWTFGQG TKVEIK | EGQLLESGGGLVQPGGSLRLSCTASGFTFSTFAMT WVRQAPGRGLEWVSVISGRGGSSFYADSVKGRFTI SRDNTKNTLYLQMNSLRAEDTAVYYCAKDQLWFD YWGQGTLVTVSS |
| | | | SEQ ID NO: 27280 | SEQ ID NO: 31286 |
| iPS:436634 | 21-225_227H5 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGACTGGGTGGCAGTTATATGGTATGAAGAAAG TAATAAATACTATGCAGACTCCGTGAAGGGCCG ATTCACCATCTCCAGAGACAATTCCAAGAACACG CTGTATCTGCAAATGAACAGCCTGAGAGCCGAG GACACGGCTATGTATTACTGTGCGAGAGAAGTGG GATTCACTGAGGACTACTGGGGCCAGGGAACCC TGGTCACCGTCTCCTCA |
| | | | SEQ ID NO: 27281 | SEQ ID NO: 31287 |

FIGURE 50

| | | | | |
|---|---|---|---|---|
| | | AA | DIQMTQSPSSLSASVGDRVTITCRASQDIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSNYGM HWVRQAPGKGLDWVAVIWYEESNKYYADSVKGR FTISRDNSKNTLYLQMNSLRAEDTAMYYCAREVGF TEDYWGQGTLVTVSS |
| | | | SEQ ID NO: 27282 | SEQ ID NO: 31288 |
| iPS:436636 | 21-225_227E6 | NA | GACATCGTGATGACCCAGTCTCCAGACTCCCT GACTGTGTCTCTGGGCGAGAGGGCCACCATCA ACTGCAAGTCCAGCCAGAGTGTTTTATACAGC TCCAACGATAAGAACTACTTAGCTTGGTACCA GCAGAAACCAGGACAGCCTCCTAAGATGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATATTACTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCTATAGCAGT GGCTGGTACAAGTTTGACTACTGGGGCCAGGGA ACCCTGGTCACCGTCTCCTCA |
| | | | SEQ ID NO: 27283 | SEQ ID NO: 31289 |
| | | AA | DIVMTQSPDSLTVSLGERATINCKSSQSVLYSSN DKNYLAWYQQKPGQPPKMLIYWASTRESGVPD RFSGSGSGTDFTLTISSLQAEDVAVYYCQQYYIT PCSFGQGTKLEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YKFDYWGQGTLVTVSS |
| | | | SEQ ID NO: 27284 | SEQ ID NO: 31290 |

FIGURE 50

| iPS:436638 | 21-225_227C7 | NA | GACATCGTGATGACCCAGTCTCCAGACTCCCT GGCTGTGTCTCTGGGCGAGAGGGCCACCATCA ACTGCAGGTCCAGCCAGATTGTTTTATCCGAC TCCAACAATAACAACTACTTAGCTTGGTACCA GCAGAAACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTTCTGTCAGCAA TATTATAGTTCTCCTCCGACGTTCGGCCAAGG GACCAAGGTGGAAATCAAA | CAGGTGCAGCTGGTGCAGTCTGGGGCTGAGGTG AAGAAGCCTAGGGCCTCAGTGAAGGTCTCCTGC AAGGCTTCTGGACACACCTTCACCAATTATGATA TCAACTGGGTGCGACAGGCCACTGGACAAGGGC TTGAGTGGATGGGATGGATGCATCCTAACAGTGG TAACACAGGCTATGCACAGAAGTTCCAGGGCAG AGTCACCATGACCAGGAACACCTCCATAAGCAC CGCCTACATGGAGCTGAGCAGCCTGAGATCTGA GGACACGGCCGTGTATTACTGTGCATATAGCAGT GGCTGGTACCGCTTTGACTACTGGGGCCAGGGAA CCCTGGTCACCGTCTCCTCA |
|  |  |  | SEQ ID NO: 27285 | SEQ ID NO: 31291 |
|  |  | AA | DIVMTQSPDSLAVSLGERATINCRSSQIVLSDSNN NNYLAWYQQKPGQPPNLLIYWASTRESGVPDRF SGSGSGTDFTLTISSLQAEDVAVYFCQQYYSSPP TFGQGTKVEIK | QVQLVQSGAEVKKPRASVKVSCKASGHTFTNYDIN WVRQATGQGLEWMGWMHPNSGNTGYAQKFQGR VTMTRNTSISTAYMELSSLRSEDTAVYYCAYSSGW YRFDYWGQGTLVTVSS |
|  |  |  | SEQ ID NO: 27286 | SEQ ID NO: 31292 |
| iPS:436640 | 21-225_227A8 | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GATGGAAAGACCTTTTTTGTATTGGTATCTGCA GAAGCCAGGCCAGCCCCCACAGCTCCTGATCT ATGAAATTTCCAACCGGTTCTCTGGGGTGCCA GATAGGTTCAGTGGCAGCGGGTCAGGGACAG ATTTCACACTGAAAATCAGTCGAGTGGAGGCT GAGGATGTTGGGATTTATTACTGCATGCAAAG TACACAGCTTCCTCGGACGTTCGGCCAAGGGA CCAGGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTTACAGTGG TGACACAAACTATGCACAGAAGTTTCAGGGCAG GGTCACCATGACCAGGGACACGTCCGTCAGCAC AGCCTACATGGAACTGAGCAGGCTGAGATTTGA CGACACGGCCGTGTTTTACTGTGCGAGAGATTGG GGTGGCTACAGTTCTTACTACTACGGTATGGACG TCTGGGGCCAAGGGACCACGGTCACCGTCTCTTC A |
|  |  |  | SEQ ID NO: 27287 | SEQ ID NO: 31293 |

FIGURE 50

| | | | | |
|---|---|---|---|---|
| | | AA | DIVMTQTPLSLSVTPGQPASISCKSSQSLLHSDGK TFLYWYLQKPGQPPQLLIYEISNRFSGVPDRFSGS GSGTDFTLKISRVEAEDVGIYYCMQSTQLPRTFG QGTRVEIK | QVQLVQSGAEVKKPGASVKVSCKASGYTFTGYYIH WVRQAPGQGLEWMGWINPYSGDTNYAQKFQGRV TMTRDTSVSTAYMELSRLRFDDTAVFYCARDWGG YSSYYYGMDVWGQGTTVTVSS |
| | | | SEQ ID NO: 27288 | SEQ ID NO: 31294 |
| iPS:436644 | 21-225_227G9 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATAACAACTACTTAGCTTGGTATCA GCAGAAACCAGGACAGCCTCCTAAGCTGCTCA TTTACTGGGGATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTGCTCCGTACAGTTTTGGCCAGGG GACCAAGTTGGAGATCAAA | CAGGTGCAGCTGGTGCAGTCTGGGGGCTGAGGTG AAGAAGCCTGGGGCCTCAGTGAAGGTCTCCTGC AGGGCTTCTGGATACACCTTCACCAATTATGATA TCAACTGGGTGCGACAGGCCACTGGACGAGGGC TTGAGTGGATGGGATGGATGTACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGCTTAGCAGT GGCTGGTACTGGTTCGACCCCTGGGGGCCAGGGA ACCCTGGTCACCGTCTCCTCA |
| | | | SEQ ID NO: 27289 | SEQ ID NO: 31295 |
| | | AA | DIVMTQSPDSLAVSLGERATINCKSSQSVLHSSN NNNYLAWYQQKPGQPPKLLIYWGSTRESGVPD RFSGSGSGTDFTLTISSLQAEDVAVYYCQQYYSA PYSFGQGTIKLEIK | QVQLVQSGAEVKKPGASVKVSCRASGYTFTNYDIN WVRQATGRGLEWMGWMYPNSGNTGYAQKFQGR VTMTRNTSISTAYMELSSLRSEDTAVYYCALSSGW YWFDPWGQGTLVTVSS |
| | | | SEQ ID NO: 27290 | SEQ ID NO: 31296 |

FIGURE 50

| iPS:436646 | 21-225_227D11 | NA | GACATCGTGATGACCCAGTCTCCAGACTCCCT GGCTGTGTCTCTGGACGAGAGGGCCACCATCA ACTGCAAGTCCAGCCAGAGTGTTTTACACAGT TCCAACAATAATAACTACTTAGCTTGGTACCA GCAGAAGCCAGGACAGCCTCCTAAGCTGCTCA TTTACTGGGCATCTACCCGGGAATCCGGGATC CCTGACCGATTCAGTGGCAGCGGGTCTGGGAC AGATTTCACTCTCACCATCAGCAGCCTGCAGG CTGAGGATGTGGCAGTTTATTACTGTCAGCAA TATTATAATACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCCG AGTCACCATGACCAGGAACACCTCCATAAGCAC AGCCTACATGGAACTGAGCAGCCTGAGATCTGA GGACACGGCCGTGTATTACTGTGCGTATAGCAGT GGCTGGTACTACTTTGACTACTGGGGCCAGGGAA CCCTGGTCACCGTCTCCTCA |
| | | | SEQ ID NO: 27291 | SEQ ID NO: 31297 |
| | | AA | DIVMTQSPDSLAVSLDERATINCKSSQSVLHSSN NNNYLAWYQQKPGQPPKLLIYWASTRESGIPDR FSGSGSGTDFTLTISSLQAEDVAVYYCQQYYNTP CSFGQGTKLEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YYFDYWGQGTLVTVSS |
| | | | SEQ ID NO: 27292 | SEQ ID NO: 31298 |
| iPS:436648 | 21-225_227F11 | NA | GATGTTGTGATGACTCAGTCTCCACTCTCCCTG CCCGTCACCCCTGGAGAGCCGGCCTCCATCTC CTGCTGGTCTAGTCAGAGCCTCCTGCATAGTA ATGGATACAACTATTTGGATTGGTACCTGCAG AAGCCAGGGCAGTCTCCACAGGTCCTGATCTA TTTGGGTTCTAATCGGGCCTCCGGGGTCCCTG ACAGGTTCAGTGGCAGTGGATCAGGCACAGAT TTTACACTGAAAATCAGCAGAGTGGAGGCTGA GGATGTTGGGGTTTATTACTGCATGCAAGCTC TACAAACTCCTCTCACCTTCGGCCAAGGGACA CGACTGGAGATTAAA | GAGGTGCAGCTGGTGGAGTCTGGGGGAGGCCTG GTCAAGCCTGGGGGGTCCCTGAGACTCTCCTGTG CAGTCTCTGGATTCACCTTCAGTACCTATAGCAT GAACTGGGTCCGCCAGGCTCCAGGGAAGGGGCT GGAGTGGGTCTCATCCATTAGTAGTAGTATTAAT TACATGTACTACGCAGACTCAGTGAAGGGCCGAT TCACCATCTCCAGAGACAACGCCAAGAACTCACT GTATCTGCAAATGAACAGCCTGAGAGCCGAGGA CTCGGCTGTGTATTACTGTGCGAGATTAGGGGTC TACTGGGGCCAGGGAACCCTGGTCACCGTCTCCT CA |
| | | | SEQ ID NO: 27293 | SEQ ID NO: 31299 |

EP 4 435 105 A2

FIGURE 50

| | | AA | DVVMTQSPLSLPVTPGEPASISCWSSQSLLHSNG YNYLDWYLQKPGQSPQVLIYLGSNRASGVPDRF SGSGSGTDFTLKISRVEAEDVGVYYCMQALQTP LTFGQGTRLEIK | EVQLVESGGGLVKPGGSLRLSCAVSGFTFSTYSMN WVRQAPGKGLEWVSSISSSINYMYYADSVKGRFTI SRDNAKNSLYLQMNSLRAEDSAVYYCARLGVYW GQGTLVTVSS |
|---|---|---|---|---|
| | | | SEQ ID NO: 27294 | SEQ ID NO: 31300 |
| IPS:436650 | 21-225_227C12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCATTC ACTTTCGGCCCTGGGACCAAAGTGGATATCAA A | CAGGTGCAGCTGGTGGAGTCTGGGGGAGGCGTG GTCCAGCCTGGGAAGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATATTGGAAGT AATCAATACTATGCGGACTCCGTGAAGGGCCGAT TCACCATCTCCAGAGACAATTCCAAGAACACGCT GTATCTGCAAATGAGCAGCCTGAGAGCCGAGGA CACGGCTGTGTATTACTGTGCGAGAGAAGTGGGA TTCACTGAGGACTACTGGGGCCAGGGAACCCTG GTCACCGTCTCCTCC |
| | | | SEQ ID NO: 27295 | SEQ ID NO: 31301 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGKSLRLSCAASGFTFSNYGM HWVRQAPGKGLEWVAVIWYIGSNQYYADSVKGR FTISRDNSKNTLYLQMSSLRAEDTAVYYCAREVGF TEDYWGQGTLVTVSS |
| | | | SEQ ID NO: 27296 | SEQ ID NO: 31302 |

2459

FIGURE 50

| iPS:436652 | 21-225_146B11 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA<br><br>SEQ ID NO: 27297 | GAGGTGCAACTGTTGGAGTCTGGGGGAGGCTTG GTACAGCCTGGGGGGTCCCTGAGACTCTCCTGTG CAGCTTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA<br><br>SEQ ID NO: 31303 |
| | | AA | SYELTQPPSVSVSPGQTASITCSGDKLGDKYASW YQQKPGQSPVLVIYQDSKRPSGIPERFSGSNSGN TATLTISGIQAMDEADYYCQAWDSSTVVFGGGT KLTVL<br><br>SEQ ID NO: 27298 | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS<br><br>SEQ ID NO: 31304 |
| iPS:436654 | 21-225_146C11 | NA | TCCTATGAACTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA<br><br>SEQ ID NO: 27299 | GAGGTGCAACTGTTGGAGTCTGGGGGAGGCTTG GTACAGCCGGGGGGGTCCCTGAGACTCTCCTGTG CAGCCTCTGGAATCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA<br><br>SEQ ID NO: 31305 |

FIGURE 50

| | | | | |
|---|---|---|---|---|
| | | AA | SYELTQPPSVSVSPGQTASITCSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGITFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27300 | SEQ ID NO: 31306 |
| iPS:436658 | 21-225_146A2 | NA | TCCTATGAGCTGACTCAGCCACCCTCAGTGTC CGTGTCCCCAGGACAGACAGCCAGCATCACCT GCTCTGGAGATAAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCCTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGCATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27301 | SEQ ID NO: 31307 |
| | | AA | SYELTQPPSVSVSPGQTASITCSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLHLQMNSLRAEDTAVYYCAKWRGNPT DYGMDVWGQGTTVTVSS |
| | | | SEQ ID NO: 27302 | SEQ ID NO: 31308 |

FIGURE 50

| | | | | |
|---|---|---|---|---|
| iPS:436660 | 21-225_146D8 | NA | CAGTCTGTACTGACTCAGCCACCCTCAACGTC TGGGACCCCCGGGCAGAGGGTCACCATCTCTT GTTCTGGAAGCAGCTCCTACATCGGAAGTAAT ACTGTAGACTGGTACCAGCAGCTCCCAGGAAC GGCCCCCAAACTCCTCATCTATAGTAATAATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTT AATGGCGTGGTATTCGGCGGAGGGACCAAACT GACCGTCCTA | GAGGTGCAGCTGGTGGAGTCTGGGGGAGGCTTG GTACAGCCTGGGGGGTCCCTGAGACTCTCCTGTG CAGCCTCTGGATTCACCTTCAGTAACTATAACAT GAACTGGGTCCGCCAGGCTCCAGGGAAGGGGCT GGAGTGGGTTTCATACATTAGTAGAAGTAGTAAT ACCAAATACTATGTAGACTCTGTGAAGGGCCGAT TCACCATCTCCAGAGACAATGCCAAGAACTCATT GTATCTGCAAATGAACAGCCTGAGAGACGAGGA CACGGCTGTGTATTACTGTGCGAGAGATAGGAGT GGGGAGCTACGGGTACTTCTACTACTACGGTTTGG ACGTCTGGGGCCAAGGGACCACGGTCACCGTCTC CTCA |
| | | | SEQ ID NO: 27303 | SEQ ID NO: 31309 |
| | | AA | QSVLTQPPSTSGTPGQRVTISCSGSSSYIGSNTVD WYQQLPGTAPKLLIYSNNQRPSGVPDRFSGSKS GTSASLAISGLQSEDEADYYCAAWDDSLNGVVF GGGTKLTVL | EVQLVESGGGLVQPGGSLRLSCAASGFTFSNYNMN WVRQAPGKGLEWVSYISRSSNTKYYVDSVKGRFTI SRDNAKNSLYLQMNSLRDEDTAVYYCARDRSGSY GYFYYYGLDVWGQGTTVTVSS |
| | | | SEQ ID NO: 27304 | SEQ ID NO: 31310 |
| iPS:436662 | 21-225_147D7 | NA | TCCTATGAGTTGACTCAGCCACCCTCAGTGTC CGTGTCCCCAGGACAGACAGCCAGCATCACCT GCTCTGGAGATAAATTGGGGGATAAATTTGCT TGCTGGTATCAGCAGAAACCAGGCCAGTCCCC TGTACTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGGAACACCGCTGTC TTCGGAACTGGGACCAAGGTCACCGTCCTA | CAGGTGCAGCTGGTGCAGTCTGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGATGAACCCTAATAGTGG TAACACAGGCTATGCACAGAAGTTCCAGGGCAG AGTCACCATGACCAGGAACACCTCCATAAGCAC AGCCTATATGGAGCTGAGAAGCCTGAGATCTGA GGACACGGCCGTGTATTACTGTGCGAGAGCGGA TATTGTATTAGTACCAGCTGCTATCCCTTATAATT ACTACTTCGCTATGGACGTCTGGGGCCAAGGGAC CACGGTCACCGTCTCCTCA |

FIGURE 50

|  |  |  | SEQ ID NO: 27305 | SEQ ID NO: 31311 |
|---|---|---|---|---|
|  |  | AA | SYELTQPPSVSVSPGQTASITCSGDKLGDKFACW YQQKPGQSPVLVIYQDRKRPSGIPERFSGSNSGN TATLTISGTQAMDEADYYCQAWDRNTAVFGTG TKVTVL | QVQLVQSGAEVKKPGASVKVSCKASGYTFTSYDIN WVRQATGQGLEWMGWMNPNSGNTGYAQKFQGR VTMTRNTSISTAYMELRSLRSEDTAVYYCARADIV LVPAAIPYNYYFAMDVWGQGTTVTVSS |
|  |  |  | SEQ ID NO: 27306 | SEQ ID NO: 31312 |
| iPS:436664 | 21-225_147E7 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGCC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGTACATACTACGCAGACTCCGTGAAGGGCCGG TTCACCCTCTCCAGAGACAATTCCAAGAACACGC TGTATCTGCAAATGAACAGCCTGAGAGCCGAGG ACACGGCCGTTTTTTACTGTGCGAAATGGCGAGG TAACCCCACTGACTACGGTATGGACGTCTGGGGC CAAGGGACCACGGTCACCGTCTCCTCA |
|  |  |  | SEQ ID NO: 27307 | SEQ ID NO: 31313 |
|  |  | AA | SYELTQPPSVSVSPGQTASITCSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFT LSRDNSKNTLYLQMNSLRAEDTAVFYCAKWRGNP TDYGMDVWGQGTTVTVSS |
|  |  |  | SEQ ID NO: 27308 | SEQ ID NO: 31314 |

FIGURE 50

| | | | | |
|---|---|---|---|---|
| iPS:436666 | 21-225_147B8 | NA | TCCTATGAACTGACTCAGCCACCCTCAGTGTC CGTGTCCCCAGGACAGACAGCCAGTATCACCT GCTCTGGAGATAAATTGGGGGATAAATATGTT TGCTGGTATCAGCAGAAGCCAGGCCAGTCCCC TGAGCTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGGCAGTAACACTGCTGTG GTATTCGGCGGAGGGACCAAGCTGACCGTCCT A | CAGGTGCAGCTGGTGCAGTCTGGGGCTGAGGTG AAGAAGCCTGGGGCCTCAGTGAAGGTCTCCTGC AAGGCTTCTGGATACACCTTCACCGACTACTATT TGCACTGGGTGCGACAGGCCCCTGGACAAGGGC TTGAGTGGATGGGATGGATCAACCCTAACAGTGG TGACACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGATCGG GACTCTGGTTCGGGGAGTTACCCCTACTACTACT ACTACGGTATGGACGTCTGGGGGCCAAGGGACCA CGGTCACCGTCTCCTCA |
| | | | SEQ ID NO: 27309 | SEQ ID NO: 31315 |
| | | AA | SYELTQPPSVSVSPGQTASITCSGDKLGDKYVCW YQQKPGQSPELVIYQDRKRPSGIPERFSGSNSGN TATLTISGTQAMDEADYYCQAWGSNTAVVFGG GTKLTVL | QVQLVQSGAEVKKPGASVKVSCKASGYTFTDYYL HWVRQAPGQGLEWMGWINPNSGDTNYAQKFQGR VTMTRDTSISTAYMELSRLRSDDTAVYYCARDRDS GSGSYPYYYYYGMDVWGQGTTVTVSS |
| | | | SEQ ID NO: 27310 | SEQ ID NO: 31316 |
| iPS:436668 | 21-225_147B9 | NA | TCCTATGAGCTGACTCAGCCGCCCTCAGTGTC CGTGTCCCCAGGACAGACAGCCAGCATCACCT GCTCTGGAGATAAATTGGGGGATAAATATGTT TCCTGGTATCAGCAGAGGCCAGGCCAGTCCCC TGTGCTGGTCATCTATCAAGATAGGAAGCGGC CCTCAGGGATCCCTGAGCGATTCTCTGGCTCC AACTCTGGGAACACAGCCACTCTGACCATCAG CGGGACCCTGGCTGTGGATGAGGCTGACTATT ACTGTCTGGCGTGGGACAGCAGCACTTTTGTG GTATTCGGCGGAGGGACCAAGTTGACCGTCCT A | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGTGA CTACGGTGACCCCCCCTACTACTACTACTACGGT ATGGACGTCTGGGGCCAAGGGACCACGGTCACC GTCTCCTCA |

FIGURE 50

| | | | SEQ ID NO: 27311 | SEQ ID NO: 31317 |
|---|---|---|---|---|
| | | AA | SYELTQPPSVSVSPGQTASITCSGDKLGDKYVSW YQQRPGQSPVLVIYQDRKRPSGIPERFSGSNSGN TATLTISGTLAVDEADYYCLAWDSSTFVVFGGG TKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARDRDY GDPPYYYYYGMDVWGQGTTVTVSS |
| | | | SEQ ID NO: 27312 | SEQ ID NO: 31318 |
| iPS:436670 | 21-225_147D9 | NA | TCCTATGAGCTGACTCAGCCACCCTCAGTGTC CGTGTCCCCAGGACAGACAGCCAGCATCACCT CCTCTGGAGATAAATTGGGTAATAAATATGTT TGCTGGTATCAGCAGAGGCCAGGCCAGTCCCC TGTGCTGGTCATCTATCAAGATAGCAAGCGGC CCTCAGGGATCCCTGAGCGATTCTCTGGCTCC AACTCCGGGAACACAGCCACTCTGACCATCAG CGGGACCCAGGCTATGGATGAGGCTGACTATT ACTGTCAGGCGTGGGACAGGAACACTTATGTG GTGTTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGATATATGGTTTGATGGCAGT AATAAATACTATGTAGACTCCGTGAAGGACCGAT TCACCATCTCCAGAGACAATTCCAAGAACACGCT GTATCTGCAAATGAACAGCCTGAGAGCCGAGGA CACGGCTGTTTATTACTGTGCGAGAGATCGGGTG GAGGGTTCGGGGACTCCCTACTACTACTACGGTA TGGACGTCTGGGGCCAAGGGACCACGGTCACCG TCTCCTCA |
| | | | SEQ ID NO: 27313 | SEQ ID NO: 31319 |
| | | AA | SYELTQPPSVSVSPGQTASITSSGDKLGNKYVCW YQQRPGQSPVLVIYQDSKRPSGIPERFSGSNSGN TATLTISGTQAMDEADYYCQAWDRNTYVVFGG GTKLTVL | QVQLVESGGGVVQPGRSLRLSCAASGFTFSSYGMH WVRQAPGKGLEWVADIWFDGSNKYYVDSVKDRF TISRDNSKNTLYLQMNSLRAEDTAVYYCARDRVEG SGTPYYYYGMDVWGQGTTVTVSS |
| | | | SEQ ID NO: 27314 | SEQ ID NO: 31320 |

FIGURE 50

| iPS:436672 | 21-225_147F9 | NA | TCCTATGAGCTGACTCAGCCACCCTCAGTGTC CGTGTCCCCAGGACAGACAGCCAGCATCACCT GTTCTGGAGATGAATTGGGGAATAAATATGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGCACAGCAGCACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAGTTATATGGTATGGTGGAAGT GATAAAGACTATGCAGACTCCGTGAAGGGCCGA TTCACCATCTCCAGAGACATTTCCAAGAACACGC TGTATCTGCAAATGAACAGCCTGAGAGCCGAGG ACACGGCTGTGTATTACTGTGCGAGAGATCGGGA TTATTGTAGTGGTGGTACTTGTCCTTACTACTACT ACTACGGTATGGACGTCTGGGGCCAAGGGACCA CGGTCACCGTCTCCTCA |
| | | | SEQ ID NO: 27315 | SEQ ID NO: 31321 |
| | | AA | SYELTQPPSVSVSPGQTASITCSGDELGNKYACW YQQKPGQSPVLVIYQDSKRPSGIPERFSGSNSGN TATLTISGTQAMDEADYYCQAWHSSTVVFGGG TKLTVL | QVQLVESGGGVVQPGRSLRLSCAASGFTFSTYGMH WVRQAPGKGLEWVAVIWYGGSDKDYADSVKGRF TISRDISKNTLYLQMNSLRAEDTAVYYCARDRDYC SGGTCPYYYYYGMDVWGQGTTVTVSS |
| | | | SEQ ID NO: 27316 | SEQ ID NO: 31322 |

FIGURE 50

| iPS:436674 | 21-225_147G9 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAGGAAGCGGC CCTCAGGGATCCCTGAGCGATTCTCTGGCTCC AACTCTGGGAACACAGCCACTCTGACCATTAG CGGGACCCAGGCTATGGATGAGGCTGACTATT ACTGTCAGGCGTGGCACAGCAGTACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAGTTATATGGTATGGTGGAAGT GATAAAGACTATGCAGACTCCGTGAAGGGCCGA TTCACCATCTCCAGAGACATTTCCAAGAACACGC TGTATCTGCAAATGAACAGCCTGAGAGCCGAGG ACACGGCTGTGTATTACTGTGCGAGAGATCGGGA TTATTGTAGTGGTGGTAGCTGCCCTTACTACTACT ACTACGGTATGGACGTCTGGGGCCAAGGGACCA CGGTCACCGTCTCCTCA |
|---|---|---|---|---|
| | | | SEQ ID NO: 27317 | SEQ ID NO: 31323 |
| | | AA | SYELTQPPSVSVSPGQTASITCSGDKLGDKYACW YQQKPGQSPVLVIYQDRKRPSGIPERFSGSNSGN TATLTISGTQAMDEADYYCQAWHSSTVVFGGG TKLTVL | QVQLVESGGGVVQPGRSLRLSCAASGFTFSTYGMH WVRQAPGKGLEWVAVIWYGGSDKDYADSVKGRF TISRDISKNTLYLQMNSLRAEDTAVYYCARDRDYC SGGSCPYYYYYGMDVWGQGTTVTVSS |
| | | | SEQ ID NO: 27318 | SEQ ID NO: 31324 |

FIGURE 50

| iPS:436676 | 21-225_147E11 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGTTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAACTATGTCAT GAGCTGGGTCCGCCAGGCTCCAGGGAAGGGGCT GGAGTGGGTCTCAGTTATTAGTGGTGGTGGTAGT AGTACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27319 | SEQ ID NO: 31325 |
| | | AA | SYELTQPPSVSVSPGQTASITCSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSNYVMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27320 | SEQ ID NO: 31326 |
| iPS:436678 | 21-225_147B12 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACGCAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27321 | SEQ ID NO: 31327 |

FIGURE 50

| | | | | |
|---|---|---|---|---|
| | | AA | SYELTQPPSVSVSPGQTASITCSGDKLGDKYASW YQQKPGQSPVLVIYQDSKRPSGIPERFSGSNSGN AATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27322 | SEQ ID NO: 31328 |
| iPS:436680 | 21-225_147H12 | NA | CAGTCTGTGTTGACTCAGCCACCCTCAGCGTC TGGGACCCCCGGGCAGAGGGTCACCATCTCTT GTTCTGGAAGCAGCTCCAACATCGGAAGTTAT GCTGTAAACTGGTATCAGCAGCTCCCAGGAAC GGCCCCCAAACTCCTCATTTATAGTAATAATC ACCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGAAGCATGGGATGACAGCCTG AATGGTCCGGTATTCGGCGGAGGGACCAAGCT GACCGTCCTA | CAGGTGCAGCTGCAGGAGTCGGGCCCAGGACTG GTGAATCCTTCACAGACCCTGTCCCTCACCTGCG CTGTCTCTGGTGGCTCCATCAGCAGTGGTTATTA CCACTGGAGCTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGTTGACACGTCTAAGAACCA GTTCTCCCTGAAGCTGACCTCTGTGACTGCCGCG GACACGGCCGTGTATTACTGTGCGAGAGATTGGG GTGGCTACGATTCGAGTGGCTGGTTCGACCCCTG GGGCCAGGGAACCCTGGTCACCGTCTCCTCA |
| | | | SEQ ID NO: 27323 | SEQ ID NO: 31329 |
| | | AA | QSVLTQPPSASGTPGQRVTISCSGSSSNIGSYAVN WYQQLPGTAPKLLIYSNNHRPSGVPDRFSGSKS GTSASLAISGLQSEDEADYYCEAWDDSLNGPVF GGGTKLTVL | QVQLQESGPGLVNPSQTLSLTCAVSGGSISSGYYH WSWIRQHPGKGLEWIGYIYYSGSTYYNPSLKSRVTI SVDTSKNQFSLKLTSVTAADTAVYYCARDWGGYD SSGWFDPWGQGTLVTVSS |
| | | | SEQ ID NO: 27324 | SEQ ID NO: 31330 |

FIGURE 50

| iPS:436682 | 21-225_146A8 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTAAT TCTATAAACTGGTACCAGCAACTCCCAAGAAC GGCCCCCAAACTCCTCATCTATAGTAATGATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AACGGCGTGGTATTCGGCGGAGGGACCAAGC TGACCGTCCTA  SEQ ID NO: 27325 | GAGGTGAAGCTGGTGGAGTCTGGGGGAGGCTTG GTACAGCCTGGGGAGTCCCTGAGACTCTCCTGTG TAGCCTCTGGATTCACCTTCAGTAACTATAACAT GAACTGGGTCCGCCAGGCTCCAGGGAAGGGACT GGAGTGGGTTTCATACATTAGTAGAAGTAGTAAT ACCAAATACTACGCAGACTCTGTGAGGGGCCGA TTCACCATCTCCAGAGACAATGCCAAGAATTCAC TATATCTGCAAATGAACAGCCTGAGAGACGAGG ACACGGCTGTGTATTACTGTGCGAGAGATAGGAG TGGGAGCTACGGGTACTTCTACTACTACGGTTTG GACGTCTGGGGCCAAGGGACCACGGTCACCGTC TCCTCA  SEQ ID NO: 31331 |
| | | AA | QSVLTQPPSASGTPGQRVTISCSGSSSNIGSNSIN WYQQLPRTAPKLLIYSNDQRPSGVPDRFSGSKSG TSASLAISGLQSEDEADYYCAAWDDSLNGVVFG GGTKLTVL  SEQ ID NO: 27326 | EVKLVESGGGLVQPGESLRLSCVASGFTFSNYNMN WVRQAPGKGLEWVSYISRSSNTKYYADSVRGRFTI SRDNAKNSLYLQMNSLRDEDTAVYYCARDRSGSY GYFYYYGLDVWGQGTTVTVSS  SEQ ID NO: 31332 |
| iPS:436684 | 21-225_146B6 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTAAT GCTGTAAACTGGTACCAGCAGCTCCCAGGAAC GGCCCCCAAACTCCTCATCTATAGTAATAATC AGCGGCCCTCAGGGGTCCCTGACCGGTTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AATGGCGTGGTATTCGGCGGAGGGACCAAGCT GACCGTCCTA  SEQ ID NO: 27327 | GAGGTGCAGCTGGTGGAGTCTGGGGGAGGCTTG GTACAGCCTGGGGGGGTCCCTGAGACTCTCCTGTG CAGCCTCTGGATTCACCTTCAGTAGCTATAACAT GAACTGGGTCCGCCAGGCTCCAGGGAAGGGGCT GGAGTGGGTTTCATACATTAGTAGAAGTAGTAAT ACCAAACACTACGCAGACTCTGTGAAGGGCCGA TTCACCATCTCCAGAGACAATGCCAAGAACTCAC TGTATCTGCAAATGGACAGCCTGAGAGACGAGG ACACGGCTGTGTATTACTGTGCGAGAGATAGGAG TGGGAGCTACGGGTACTTCTACTACTACGGTTTG GACGTCTGGGGCCAAGGGACCACGGTCACCGTC TCCTCA  SEQ ID NO: 31333 |

FIGURE 50

| | | | | |
|---|---|---|---|---|
| | | AA | QSVLTQPPSASGTPGQRVTISCSGSSSNIGSNAVN WYQQLPGTAPKLLIYSNNQRPSGVPDRFSGSKS GTSASLAISGLQSEDEADYYCAAWDDSLNGVVF GGGTKLTVL | EVQLVESGGGLVQPGGSLRLSCAASGFTFSSYNMN WVRQAPGKGLEWVSYISRSSNTKHYADSVKGRFTI SRDNAKNSLYLQMDSLRDEDTAVYYCARDRSGSY GYFYYYGLDVWGQGTTVTVSS |
| | | | SEQ ID NO: 27328 | SEQ ID NO: 31334 |
| iPS:436686 | 21-225_148G6 | NA | TCCTATGAGCTGACTCAGCCACCCTCAGTGTC CGTGTCCCCAGGACAGACAGCCAGCATCACCT GCTCTGGAGATAAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACATGC TGCATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27329 | SEQ ID NO: 31335 |
| | | AA | SYELTQPPSVSVSPGQTASITCSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNMLHLQMNSLRAEDTAVYYCAKWRGNP TDYGMDVWGQGTTVTVSS |
| | | | SEQ ID NO: 27330 | SEQ ID NO: 31336 |

FIGURE 50

| iPS:436688 | 21-225_148C8 | NA | TCCTATGAGCTGACTCAGCCGCCCTCAGTGTC CGTGTCCCCAGGACAGACAGCCAGCATCACCT GCTCTGGAGATAAATTGGGGGATAAATATGTT TCCTGGTATCAGCAGAAGCCAGGCCAGTCCCC TATACTGGTCATCTATCAAGATAGGAAGCGGC CCTCAGGGACCCCTGAGCGATTCTCTGGCTCC AACTCTGGGAACACAGCCACTCTGACCATCAG CGGGACCCAGGCTATGGATGAGGCTGATTATT ACTGTCTGGCGTGGGACAGCAGCACTTTTGTG GTATTCGGCGGAGGGACCAAGTTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGTGA CTACGGTGACCCCCCCTACTACTACTACTACGGT ATGGACGTCTGGGGCCAAGGGACCACGGTCACC GTCTCCTCA |
| | | | SEQ ID NO: 27331 | SEQ ID NO: 31337 |
| | | AA | SYELTQPPSVSVSPGQTASITCSGDKLGDKYVSW YQQKPGQSPILVIYQDRKRPSGTPERFSGSNSGN TATLTISGTQAMDEADYYCLAWDSSTFVVFGGG TKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARDRDY GDPPYYYYYGMDVWGQGTTVTVSS |
| | | | SEQ ID NO: 27332 | SEQ ID NO: 31338 |
| iPS:436690 | 21-225_148A9 | NA | TCCTATGAGCTGACTCAGCCACCCTCAGTGTC CGTGTCCCCAGGACAGACAGCCAGCATCACCT GTTCTGGAGATAAATTGGGGGAATAAATATGTT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGCACAGCAGCACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAGTTATATGGTATGGTGGAAGT GATAAAGACTATGCAGACTCCGTGAAGGGCCGA TTCACCATCTCCAGAGACAATTTCCAAGAACACGC TGTATCTGCAAATGAACAGCCTGAGAGCCGAGG ACACGGCTGTGTATTACTGTGCGAGAGATCGGGA TTATTGTAGTGGTGGTACTTGTCCTTACTACTACT ACTACGGTATGGACGTCTGGGGCCAAGGGACCA CGGTCACCGTCTCCTCA |
| | | | SEQ ID NO: 27333 | SEQ ID NO: 31339 |

FIGURE 50

| | | | | |
|---|---|---|---|---|
| | | AA | SYELTQPPSVSVSPGQTASITCSGDKLGNKYVCW YQQKPGQSPVLVIYQDSKRPSGIPERFSGSNSGN TATLTISGTQAMDEADYYCQAWHSSTVVFGGG TKLTVL | QVQLVESGGGVVQPGRSLRLSCAASGFTFSTYGMH WVRQAPGKGLEWVAVIWYGGSDKDYADSVKGRF TISRDISKNTLYLQMNSLRAEDTAVYYCARDRDYC SGGTCPYYYYYGMDVWGQGTTVTVSS |
| | | | SEQ ID NO: 27334 | SEQ ID NO: 31340 |
| iPS:436694 | 21-225_148G11 | NA | TCCTATGAGCTGACTCAGCCACCCTCAGTGTC CGTGTCCCCAGGACAGACAGCCAGCATCACCT GCTCTGGAGATAAATTGGGGGATAAATTTGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATCCCAT GAGCTGGGTCCGCCAGGCTCCCGGGAAGGGGCT GGAGTGGGTCTCAGTTATTAGTGGTGGTGGTAGT AGTGCATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27335 | SEQ ID NO: 31341 |
| | | AA | SYELTQPPSVSVSPGQTASITCSGDKLGDKFASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYPMS WVRQAPGKGLEWVSVISGGGSSAYYADSVKGRFTI SRDNSKNTLYLQMNSLRAEDTAVYYCAKWRGNPT DYGMDVWGQGTTVTVSS |
| | | | SEQ ID NO: 27336 | SEQ ID NO: 31342 |

2473

FIGURE 50

| iPS:436696 | 21-225_149A1 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTAAT GCTGTAAACTGGTACCAGCAGCTCCCAGGAAC GGCCCCCAAACTCCTCATCTATAGTAATAATC AGCGGCCCTCAGGGGTCCCTGACCGGTTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AATGGCGTGGTATTCGGCGGAGGGACCAAGCT GACCGTCCTA | GAGGTGCAGCTGGTGGAGTCTGGGGGAGGCTTG GTACAGCCTGGGGGGTCCCTGAGACTCTCCTGTG CAGCCTCTGGATTCACCTTCAGTAGCTATAACAT GAACTGGGTCCGCCAGGCTCCAGGGAAGGGGCT GGAGTGGGTTTCATACATTAGTAGAAGTAGTAAT ACCAAACACTACGCAGACTCTGTGAAGGGCCGA TTCACCATCTCCAGAGACAATGCCAAGAACTCAC TGTATCTGCAAATGAACAGCCTGAGAGACGAGG ACACGGCTGTGTATTACTGTGCGAGAGATAGGAG TGGGAGCTACGGGTACTTCTACTACTACGGTTTG GACGTCTGGGGCCAAGGGACCACGGTCACCGTC TCCTCA |
| | | | SEQ ID NO: 27337 | SEQ ID NO: 31343 |
| | | AA | QSVLTQPPSASGTPGQRVTISCSGSSSNIGSNAVN WYQQLPGTAPKLLIYSNNQRPSGVPDRFSGSKS GTSASLAISGLQSEDEADYYCAAWDDSLNGVVF GGGTKLTVL | EVQLVESGGGLVQPGGSLRLSCAASGFTFSSYNMN WVRQAPGKGLEWVSYISRSSNTKHYADSVKGRFTI SRDNAKNSLYLQMNSLRDEDTAVYYCARDRSGSY GYFYYYGLDVWGQGTTVTVSS |
| | | | SEQ ID NO: 27338 | SEQ ID NO: 31344 |
| iPS:436698 | 21-225_149B5 | NA | TCCTATGAACTGACTCAGCCACCCTCAGTGTC CGTGTCCCCAGGACAGACAGCCAGCATCACCT GCTCTGGATATAAATTGGGGTATAAATATGTT TGCTGGTATCAGCAGAAGCCAGGCCAGTCCCC TGTACTGGTCATCTTTCAAAATAACCAGCGGC CCTCAGGGATCCCTGAGCGATTCTCTGGCTCC AACTCTGGGAACACAGCCTCTCTGACCATCAG CGGGACCCAGGCTATGGATGAGGCTGACTATT ACTGTCAGGCGTGGGACAGCAGCACTGTGGTA TTCGGCGGAGGGACCAAGCTGACCGTCCTA | GAGGTGCAGCTGGTGGAGTCTGGGGGAGGCTTG GTCCAGCCTGGGGGGTCCCTGAGACTCTCCTGTG CAGCCTCTGGATTCACCTTTAGTGGCTATTGGAT GAACTGGGTCCGCCAGGCTCCAGGGAAGGGGCT GGAGTGGGTGGCCAACATAAAGCAAGATGGAAG TGAGAAATACTATGTGGACTCTGTGAAGGGCCGA TTCACCATCTCCAGAGACAACGCCAAGAATTCAC TGTATCTGCAAATGAACAGCCTGAGAGCCGAGG ACACGGCTGTGTATTACTGTGCGAGAGGGATGTA TAGCAGTGGCTGGTACGTCTTTGACTACTGGGGC CAGGGAACCCTGGTCACCGTCTCCTCA |
| | | | SEQ ID NO: 27339 | SEQ ID NO: 31345 |

FIGURE 50

| | | | | |
|---|---|---|---|---|
| | | AA | SYELTQPPSVSVSPGQTASITCSGYKLGYKYVCW YQQKPGQSPVLVIFQNNQRPSGIPERFSGSNSGN TASLTISGTQAMDEADYYCQAWDSSTVVFGGG TKLTVL | EVQLVESGGGLVQPGGSLRLSCAASGFTFSGYWM NWVRQAPGKGLEWVANIKQDGSEKYYVDSVKGR FTISRDNAKNSLYLQMNSLRAEDTAVYYCARGMY SSGWYVFDYWGQGTLVTVSS |
| | | | SEQ ID NO: 27340 | SEQ ID NO: 31346 |
| iPS:436700 | 21-225_149C7 | NA | TCCTATGAGCTGACTCAGCCACCCTCAGTGTC CGTGTCCCCAGGACAGACAGCCAGCATCACCT GCTCTGGAAATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AAGTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCCA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27341 | SEQ ID NO: 31347 |
| | | AA | SYELTQPPSVSVSPGQTASITCSGNKLGDKYASW YQQKPGQSPVLVTYQDSKRPSGIPERFSGSKSGN TATLTISGIQAMDEADYYCQAWDSSTVVFGGGT KLTVP | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27342 | SEQ ID NO: 31348 |

FIGURE 50

| iPS:436702 | 21-225_149E8 | NA | CAGGCTGTGTCGACTCAGCCGTCTTCCCTCTCTGCATCTCCTGGAGCATCAGCCAGTCTCACCTGCACCTTACGCAGTGGCATCACTGTTACTACCTATAGGATATACTGGTACCAGCAGAAGCCAGGGAGTCCTCCCCAGTTTCTCCTGCGGTACACATCAGACTCAGATAAACACCAGGGCTCTGGAGTCCCCAGCCGCTTCTCTGGATCCAAAGATGCTTCGGCCAATGCAGGGATTTTATTCATCTCTGGGCTCCAGTCTGAGGATGAGGCTGACTATTACTGTATGATTTGGCACAGCAGCGCTTGGGTGTTCGGCGGAGGGACCAAGCTGACCGTCCTA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATAGCATGAACTGGGTCCGCCGGGCTCCAGGGAAGGGGCTGGAGTGGGTCTCAGCCATTAGTAGTACTGGTAGTTACATATATTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGGACGGCAGTGGCTGGTACTGGGTGGTTCGACCCCTGGGGCCAGGGAACCCTGGTCACCGTCTCCTCA |
|---|---|---|---|---|
| | | | SEQ ID NO: 27343 | SEQ ID NO: 31349 |
| | | AA | QAVSTQPSSLSASPGASASLTCTLRSGITVTTYRIYWYQQKPGSPPQFLLRYTSDSDKHQGSGVPSRFSGSKDASANAGILFISGLQSEDEADYYCMIWHSSAWVFGGGTKLTVL | EVQLVESGGGLVKPGGSLRLSCAASGFTFSSYSMNWVRRAPGKGLEWVSAISSTGSYIYYADSVKGRFTISRDNAKNSLYLQMNSLRAEDTAVYYCARTAVAGTGWFDPWGQGTLVTVSS |
| | | | SEQ ID NO: 27344 | SEQ ID NO: 31350 |
| iPS:436704 | 21-225_149C10 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAACAAGCGGCCCTCAGGGATCCCTGAGCGATTCTCTGGCTCCAACTCTGGGAACACAGCCACTCTGACCATCGGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGATTCTCCTGTGCAGCCTCTGGATTCACCTTTAGCAGCCACGCCATGAGCTGGGTCCGCCAGGCTCCAGGGAAGGGGCTGGAGTGGGTCTCAGTTATAAGTGGAGGTGGTAGTAGCACATATTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27345 | SEQ ID NO: 31351 |

FIGURE 50

| | | | | |
|---|---|---|---|---|
| | | AA | SYELTQPPSVSVSPGQTASITCSGDKLGDKYASW YQQKPGQSPVLVIYQDNKRPSGIPERFSGSNSGN TATLTIGGIQAMDEADYYCQAWDSSTVVFGGGT KLTVL | EVQLLESGGGLVQPGGSLRFSCAASGFTFSSHAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27346 | SEQ ID NO: 31352 |
| iPS:436706 | 21-225_149A11 | NA | TCCTATGAACTGACTCAGCCGCCCTCAGTGTC CGTGTCCCCAGGACAGACAGCCAGCATCACCT GCTCTGGAGATAAATTGGGGGAATAAATATGTT TCCTGGTATCAGCAGAAGCCAGGCCAGTCCCC TGTGCTGGTCATCTATCAAGATAGCAGGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCTGGCGTGGGACAGCAGCACTTTTGTG GTCTTCGGCGGAGGGACCAAGTTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGTGA CTACGGTGACCCCCCCTACTACTACTACTACGGT ATGGACGTCTGGGGCCAAGGGACCACGGTCACC GTCTCCTCA |
| | | | SEQ ID NO: 27347 | SEQ ID NO: 31353 |
| | | AA | SYELTQPPSVSVSPGQTASITCSGDKLGNKYVSW YQQKPGQSPVLVIYQDSRRPSGIPERFSGSNSGN TATLTISGTQAMDEADYYCLAWDSSTFVVFGGG TKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARDRDY GDPPYYYYYGMDVWGQGTTVTVSS |
| | | | SEQ ID NO: 27348 | SEQ ID NO: 31354 |

FIGURE 50

| iPS:436708 | 21-225_150D3 | NA | TCCTATGAGCTGACTCAGCCACCCTCAGTGTC CGTGTCCCCAGGACAGACAGCCAGCATCACCT GCTCTGGAGATGAATTGGGGAATAAATATGCT TGCTGGTATCAGCAGAAGCCAGGCCAGTCCCC TGTGCTGGTCATCTATCAAGATAACAAGCGGC CCTCAGGGATCCCTGAGCGATTCTCTGGCTCC AGCTCTGGGAACACAGCCACTCTGACCATCAG CGGGACCCAGGCTATGGATGAGGCTGACTATT ACTGTCAGGCGTGGCACAGCAGCACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAGTTATATGGTATGGTGGAAGT AATAAAGACTATGCAGACTCCGTGAAGGGCCGA TTCACCATCTCCAGAGACAATTCCAAGAACACGC TGTATCTGCAAATGAACAGTTTGAGAGCCGAGGA CACGGCTGTGTATTACTGTGCGAGAGATCGGGAT TATTGTAGTGGTGGTACCTGCCCCTTACTACTACTA CTACGGTATGGACGTCTGGGGGCCAAGGGACCAC GGTCACCGTCTCCTCA |
| | | | SEQ ID NO: 27349 | SEQ ID NO: 31355 |
| | | AA | SYELTQPPSVSVSPGQTASITCSGDELGNKYACW YQQKPGQSPVLVIYQDNKRPSGIPERFSGSSSGN TATLTISGTQAMDEADYYCQAWHSSTVVFGGG TKLTVL | QVQLVESGGGVVQPGRSLRLSCAASGFTFSTYGMH WVRQAPGKGLEWVAVIWYGGSNKDYADSVKGRF TISRDNSKNTLYLQMNSLRAEDTAVYYCARDRDY CSGGTCPYYYYYGMDVWGQGTTVTVSS |
| | | | SEQ ID NO: 27350 | SEQ ID NO: 31356 |

FIGURE 50

| | | | | |
|---|---|---|---|---|
| IPS:436710 | 21-225_150F6 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTGTCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT GCTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27351 | SEQ ID NO: 31357 |
| | | AA | SYELTQPPSVSVSPGQTASITCSGDKLGDKYASW YQQKPGQSPVLVICQDSKRPSGIPERFSGSNSGN TATLTISGIQAMDEADYCCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27352 | SEQ ID NO: 31358 |
| IPS:436712 | 21-225_150F9 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTAAT GCTGTAAACTGGTACCAGCAGCTCCCAGGAAC GGCCCCCAAAACTCCTCATCTATAGTAATAGTC AGCGGCCCTCAGGGGTCCCTGACCGGTTCTCT GGCTCCAAGTCTGGCACCTCAGCCTCCCTGGC CATCAGTGGGCTCCAGTCTGAGGATGAGGCTG ATTATTTCTGTGCAGCATGGGATGACAGCCTG AATGGCGTGGTATTCGGCGGAGGGACCAAGCT GACCGTCCTA | GAGATGCAGCTGGTGGAGTCTGGGGGAGGCTTG GTACAGCCTGGGGGGTCCCTGAGACTCTCCTGTG CAGCCTCTGGATTCACCTTCAGTAGCTATAACAT GAACTGGGTCCGCCAGGTTCCAGGGAAGGGGCT GGAGTGGGTTTCATACATTAGTAGAAGTAGTAAT ACCAAACACTACGCAGACTCTGTGAAGGGCCGA TTCACCATCTCCAGAGACAATGCCAAGAACTCAC TGTATCTGCAAATGAACAGCCTGAGAGACGAGG ACACGGCTGTGTATTACTGTGCGAGAGATAGGAG TGGGAGCTACGGGTACTTCTACTACTACGGTTTG GACGTCTGGGGCCAAGGGACCACGGTCACCGTC TCCTCA |
| | | | SEQ ID NO: 27353 | SEQ ID NO: 31359 |

## EP 4 435 105 A2

FIGURE 50

| | | | | |
|---|---|---|---|---|
| | | AA | QSVLTQPPSASGTPGQRVTISCSGSSSNIGSNAVN WYQQLPGTAPKLLIYSNSQRPSGVPDRFSGSKSG TSASLAISGLQSEDEADYFCAAWDDSLNGVVFG GGTKLTVL | EMQLVESGGGLVQPGGSLRLSCAASGFTFSSYNMN WVRQVPGKGLEWVSYISRSSNTKHYADSVKGRFTI SRDNAKNSLYLQMNSLRDEDTAVYYCARDRSGSY GYFYYYGLDVWGQGTTVTVSS |
| | | | SEQ ID NO: 27354 | SEQ ID NO: 31360 |
| iPS:436714 | 21-225_150H11 | NA | TCCTATGAGCTGACTCAGCCACCCTCAGTGTC CGTGTCCCCAGGACAGACAGCCAGCATCACCT GCTCTGGAGATAAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTTTT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCACCTATGCCAT GAGCTGGGTCCGCCAGGCTCCAGGGAAGGGGCT GGAGTGGGTCTCAATTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27355 | SEQ ID NO: 31361 |
| | | AA | SYELTQPPSVSVSPGQTASITCSGDKLGDKYASW YQQKPGQSPVLVIYQDSKRPSGIPERFSGSNSGN TATLTISGIQAMDEADFYCQAWDSSTVVFGGGT KLTVL | EVQLLESGGGLVQPGGSLRLSCAASGFTFSTYAMS WVRQAPGKGLEWVSIISGGGSSTYYADSVKGRFTI SRDNSKNTLYLQMNSLRAEDTAVYYCAKWRGNPT DYGMDVWGQGTTVTVSS |
| | | | SEQ ID NO: 27356 | SEQ ID NO: 31362 |

2480

FIGURE 50

| iPS:436716 | 21-225_151F3 | NA | TCTTATGAGCTGACTCAGCCACCCTCAGTGTC CGTGTCCCCAGGACAGACAGCCAGCATCACCT GCTCTGGAGATAAATTGGGGGATAAATATGTT TGCTGGTATCAGCAGAAGCCAGGCCAGTCCCC TGTGCTGGTCATCTATCAAGATCGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGCACAGCAGCACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAGTTATATGGTATGGTGGAAGT AATACAGACTATGCAGACTCCGTGAAGGGCCGA TTCACCATCTCCAGAGACATTTCCAAGAACACGC TGTATCTGCAAATGAACAGCCTGAGAGCCGAGG ACACGGCTGTGTATTACTGTGCGAGAGATCGGGA TTATTGTAGTGGTACTAGCTGCCCTTACTACTACT ACTACGGTATGGACGTCTGGGGCCAAGGGACCA CGGTCACCGTCTCCTCA |
| | | | SEQ ID NO: 27357 | SEQ ID NO: 31363 |
| | | AA | SYELTQPPSVSVSPGQTASITCSGDKLGDKYVCW YQQKPGQSPVLVIYQDRKRPSGIPERFSGSNSGN TATLTISGTQAMDEADYYCQAWHSSTVVFGGG TKLTVL | QVQLVESGGGVVQPGRSLRLSCAASGFTFSTYGMH WVRQAPGKGLEWVAVIWYGGSNTDYADSVKGRF TISRDISKNTLYLQMNSLRAEDTAVYYCARDRDYC SGTSCPYYYYYGMDVWGQGTTVTVSS |
| | | | SEQ ID NO: 27358 | SEQ ID NO: 31364 |

FIGURE 50

| iPS:436718 | 21-225_151H5 | NA | TCCTATGAGCTGACTCAGCCACCCTCAGTGTC CGTGTCCCCAGGACAGACAGCCAGCATCGCCT GCTCTGGAGATAAATTGGGGGATAAATATGCT TCCTGGTATCAACAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTCAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
|---|---|---|---|---|
| | | | SEQ ID NO: 27359 | SEQ ID NO: 31365 |
| | | AA | SYELTQPPSVSVSPGQTASIACSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27360 | SEQ ID NO: 31366 |
| iPS:436720 | 21-225_151H6 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATACCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTTATGTC TTCGGAACTGGGACCAAGGTCACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCACTTATATGGTATGATGGAAGT AATAAATACTATGCAGACTCCGTGAAGGGCCGA TTCACCATCTCCAGAGACAATTCCAAGAACACGC TGTATCTACAAATGAACAGCCTGAGAGCCGAGG ACACGGCTGTGTATTACTGTGCGAGAGATGACCG ATCTTGTAGTAGAACCAGCTGCCCTTACTACTAC TACTACGGTTTGGACGTCTGGGGCCAAGGGACCA CGGTCATCGTCTCCTCA |
| | | | SEQ ID NO: 27361 | SEQ ID NO: 31367 |

FIGURE 50

| | | | | |
|---|---|---|---|---|
| | | AA | SYELTQPPSVSVSPGQTASITCSGDKLGDKYACW YQQKPGQSPVLVIYQDTKRPSGIPERFSGSNSGN TATLTISGTQAMDEADYYCQAWDSSTYVFGTGT KVTVL | QVQLVESGGGVVQPGRSLRLSCAASGFTFSDYGM HWVRQAPGKGLEWVALIWYDGSNKYYADSVKGR FTISRDNSKNTLYLQMNSLRAEDTAVYYCARDDRS CSRTSCPYYYYYGLDVWGQGTTVIVSS |
| | | | SEQ ID NO: 27362 | SEQ ID NO: 31368 |
| iPS:436722 | 21-225_151H7 | NA | TCCTATGACCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27363 | SEQ ID NO: 31369 |
| | | AA | SYDLTQPPSVSVSPGQTASITCSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27364 | SEQ ID NO: 31370 |

FIGURE 50

| iPS:436724 | 21-225_151B9 | NA | TCCTATGAGCTGACTCAGCCACCCTCAGTGTC CGTGTCCCCAGGACAGACAGCCAGCATCGCCT GCTCTGGAGATAATTTGGGGGATAAATATGCT TCCTGGTATCAACAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTCAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27365 | SEQ ID NO: 31371 |
| | | AA | SYELTQPPSVSVSPGQTASIACSGDNLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27366 | SEQ ID NO: 31372 |
| iPS:436726 | 21-225_152G5 | NA | TCCTATGAGATGACTCAGCCACCCTCAGTGTC CGTGTCCCCAGGACAGACAGCCATCATCACCT GCTCTGGAGATAAATTGGGGGATAAATATGCT TGCTGGTATCAACAGAAGCCAGGCCAGTCCCC TGTGCTGGTCATCTATCAAGATTCCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTTATGTC TTCGGAACTGGGACCAAGGTCACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCACTTATATGGTATGATGGAAGT AATAAATACTATGCAGACTCCGTGAAGGGCCGA TTCACCATCTCCAGAGACAATTCCAAGAACACGC TGTATCTACAAATGAACAGCCTGAGAGCCGAGG ACACGGCTGTGTATTACTGTGCGAGAGATGACCG ATCTTGTAGTAGAACCAGCTGCCCTTACTACTAC TACTACGGTTTGGACGTCTGGGGGCCAAGGGACCA CGGTCATCGTCTCCTCA |
| | | | SEQ ID NO: 27367 | SEQ ID NO: 31373 |

FIGURE 50

| | | | | |
|---|---|---|---|---|
| | | AA | SYEMTQPPSVSVSPGQTAIITCSGDKLGDKYAC WYQQKPGQSPVLVIYQDSKRPSGIPERFSGSNSG NTATLTISGTQAMDEADYYCQAWDSSTYVFGT GTKVTVL | QVQLVESGGGVVQPGRSLRLSCAASGFTFSDYGM HWVRQAPGKGLEWVALIWYDGSNKYYADSVKGR FTISRDNSKNTLYLQMNSLRAEDTAVYYCARDDRS CSRTSCPYYYYYGLDVWGQGTTVIVSS |
| | | | SEQ ID NO: 27368 | SEQ ID NO: 31374 |
| iPS:436728 | 21-225_152G6 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCCAGGCTCTGGATGAGGCTGACTATT ACTGTCAGGCGTGGGACAACAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27369 | SEQ ID NO: 31375 |
| | | AA | SYELTQPPSVSVSPGQTASITCSGDKLGDKYASW YQQKPGQSPVLVIYQDSKRPSGIPERFSGSNSGN TATLTISGIQALDEADYYCQAWDNSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27370 | SEQ ID NO: 31376 |

FIGURE 50

| | | | | |
|---|---|---|---|---|
| iPS:436730 | 21-225_152D7 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTCCGGTATGGACGTCTGGGG CCAAGGGACCACGGTCACCGTCTCCTCA |
| | | | SEQ ID NO: 27371 | SEQ ID NO: 31377 |
| | | AA | SYELTQPPSVSVSPGQTASITCSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DSGMDVWGQGTTVTVSS |
| | | | SEQ ID NO: 27372 | SEQ ID NO: 31378 |
| iPS:436732 | 21-225_152B12 | NA | TCCTATGAGCTGACTCAGCCACCCTCAGTGTC CGTGTCCCCAGGACAGACAGCCAGCATCACCT GCTCTGGCGATAAATTGGGGAAATAAATATGCT TGCTGGTATCAGCAGAAGCCCAGGCCAGTCCCC TGTGCTGGTCATCTATCAAGATACCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTTATGTC TTCGGAACTGGGACCAAGGTCACCGTCCTA | CAGGTGCAGGTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTACAAATGAACAGCCTGAGAGCCGAGG ACACGGCTGTGTATTACTGTGCGAGAGATGACCG ATCTTGTAGTAGTACCAGCTGCCCTTACTACTAC TACTACGGTTTGGACGTCTGGGGCCAAGGGACCA CGGTCACCGTCTCCTCA |
| | | | SEQ ID NO: 27373 | SEQ ID NO: 31379 |

FIGURE 50

| | | | | | |
|---|---|---|---|---|---|
| | | AA | SYELTQPPSVSVSPGQTASITCSGDKLGNKYACW YQQKPGQSPVLVIYQDTKRPSGIPERFSGSNSGN TATLTISGTQAMDEADYYCQAWDSSTYVFGTGT KVTVL | QVQVVESGGGVVQPGRSLRLSCAASGFTFSDYGM HWVRQAPGKGLEWVAVIWYDGSNKYYADSVKGR FTISRDNSKNTLYLQMNSLRAEDTAVYYCARDDRS CSSTSCPYYYYYGLDVWGQGTTVTVSS | |
| | | | SEQ ID NO: 27374 | SEQ ID NO: 31380 | |
| iPS:436734 | 21-225_153A8 | NA | TCCTATGAGCTGACTCAGCCACCCTCAGTGTC CGTGTCCCCAGGGCAGACAGCCAGCATCACCT GCTCTGGAGATAAATTGGGGGGATAAATATGCT TGCTGGTATCAGCAGAAGCCAGGCCAGTCCCC TGTGCTGGTCATCTATCAAGATACCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTTATGTC TTCGGAACTGGGACCAAGGTCACCGTCCTA | CAGGTGCAGCTGATGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCACTTATATGGTATGATGGAAGT AATAAATACTATGCAGACTCCGTGAAGGGCCGA TTCACCATCTCCAGAGACAATTCCAAGAACACGC TTTATCTACAAATGAACAGCCTGAGAGCCGAGG ACACGGCTGTGTATTACTGTGCGAGAGATGACCG ATCTTGTAGTAGAACCAGCTGCCCTTACTACTAC TACTACGGTTTGGACGTCTGGGGGCCAAGGGACCA CGGTCATCGTCTCCTCA | |
| | | | SEQ ID NO: 27375 | SEQ ID NO: 31381 | |
| | | AA | SYELTQPPSVSVSPGQTASITCSGDKLGDKYACW YQQKPGQSPVLVIYQDTKRPSGIPERFSGSNSGN TATLTISGTQAMDEADYYCQAWDSSTYVFGTGT KVTVL | QVQLMESGGGVVQPGRSLRLSCAASGFTFSDYGM HWVRQAPGKGLEWVALIWYDGSNKYYADSVKGR FTISRDNSKNTLYLQMNSLRAEDTAVYYCARDDRS CSRTSCPYYYYYGLDVWGQGTTVIVSS | |
| | | | SEQ ID NO: 27376 | SEQ ID NO: 31382 | |

FIGURE 50

| iPS:436736 | 21-225_153E8 | | | | |
|---|---|---|---|---|---|
| | | NA | TCCTATGAGCTGACTCAGCCACCCTCAGTGTCCGTGTCCCCAGGACAGACAGCCAGCATCACCTGCTCTGGAAGTAAATTGGGTAATAAATATGTTTGCTGGTATCAGCAGAGGCCAGGCCAGTCCCCTGTACTGGTCATCTATCAAGATAACAAGCGGCCCTCAGGGATCCCTGAGCGATTCTCTGGCTCCAACTCCGGAAACACAGCCACTCTGACCATCAGCGGGACCCAGGCTATGGATGAGGCTGGCTATTACTGTCAGGCGTGGGACAGCAGCACTTATGTGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCCTCAGTAACTATGGCATGCACTGGGTCCGCCAGGCTCCAGGCAAGGGGCTGGAGTGGGTGGCAGTTATATGGTTTGATGGCAGTAATAAATACTATGTTGACTCCGTGAAGGACCGATTCACCATCTCCAGAGACAATTCCAAGAACACGCTATATCTGCAAATGAACAGCCTGAGAGCCGAAGACACGGCTGTGTATTACTGTGCGAGAGATCGGGTGGAGGGTTCGGGGACTCCCTACTACTACTACGGTATGGACGTCTGGGGCCAAGGGACCACGGTCACCGTCTCCTCA |
| | | | SEQ ID NO: 27377 | SEQ ID NO: 31383 |
| | | AA | SYELTQPPSVSVSPGQTASITCSGSKLGNKYVCWYQQRPGQSPVLVIYQDNKRPSGIPERFSGSNSGNTATLTISGTQAMDEAGYYCQAWDSSTYVIFGGGTKLTVL | QVQLVESGGGVVQPGRSLRLSCAASGFTLSNYGMHWVRQAPGKGLEWVAVIWFDGSNKYYVDSVKDRFTISRDNSKNTLYLQMNSLRAEDTAVYYCARDRVEGSGTPYYYYGMDVWGQGTTVTVSS |
| | | | SEQ ID NO: 27378 | SEQ ID NO: 31384 |
| iPS:436738 | 21-225_153D9 | | | | |
| | | NA | TCCTATGAGCTGACTCAGCCACCCTCAGTGTCCGTGTCCCCAGGACAGACAGCGAGCATCACCTGCTCTGGAGATAAATTGGGGGATAAATATGCTTGCTGGTATCAGCAGAAGCCAGGCCAGTCCCCTGTACTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGCACAGCAGTACTGTGGTATTCGGCGGAGGGACCAAGGTGACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCGTCAGTACCTATGGCATGCACTGGGTCCGCCAGGCTCCAGGCAAGGGGCTGGAGTGGGTGGCAGTTATATGGTATGGTGGAAGTAATAAAGACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGATTATTGTAGTGGTGGTAGCTGTCCTTACTACTACTACTACGGTATGGACGTCTGGGGCCAAGGGACCACGGTCACCGTCTCCTCA |

2488

FIGURE 50

| | | | | SEQ ID NO: 27379 | SEQ ID NO: 31385 |
|---|---|---|---|---|---|
| | | | AA | SYELTQPPSVSVSPGQTASITCSGDKLGDKYACW YQQKPGQSPVLVIYQDRKRPSGIPERFSGSNSGN TATLTISGTQAMDEADYYCQAWHSSTVVFGGG TKVTVL | QVQLVESGGGVVQPGRSLRLSCAASGFTVSTYGM HWVRQAPGKGLEWVAVIWYGGSNKDYADSVKGR FTISRDISKNTLYLQMNSLRAEDTAVYYCARDRDY CSGGSCPYYYYYGMDVWGQGTTVTVSS |
| | | | | SEQ ID NO: 27380 | SEQ ID NO: 31386 |
| iPS:436740 | 21-225_154C3 | | NA | TCCTATGAGCTGACTCAGCCACCCTCAGTGTC CGTGTCCCCAGGACAGACAGCCAGCATCACCT GCTCTGGAGATAAATTGGGGGATAAATATGTT TGCTGGTATCAGCAGAAGCCAGGCCAGTCCCC TGTGTTGGTCATCTATCAAGATAGGAAGCGGC CCTCAGGGATCCCTGAGCGATTCTCTGGCTCC AACTCTGGGAACACAGCCACTCTGACCATCAC CGGGACCCAGGCTATGGATGAGGCTGACTATT ACTGTCAGGCGTGGCACAGCAGCACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTA CAGCGTCTGGATTCACCTTCAGTACCTATGGCAT GCACTGGGTCCGCCAGGCTCCAGGCAAGGGGCT GGAGTGGGTGGCAGTTGTATGGTATGGTGGAAAT AATAAAGACTATGCAGACTCCGTGAAGGGCCGA TTCACCATCTCCAGAGACATTTCCAAGAACACAC TGTATCTGCAAATGAACAGCCTGAGAGCCGAGG ACACGGCTGTGTATTACTGTGCGAGAGATCGGGA TTATTGTAGTGGTGGTAGCTGCCCCTTACTACTACT ACTACGGTATGGACGTCTGGGGCCAAGGGACCA CGGTCACCGTCTCCTCA |
| | | | | SEQ ID NO: 27381 | SEQ ID NO: 31387 |
| | | | AA | SYELTQPPSVSVSPGQTASITCSGDKLGDKYVCW YQQKPGQSPVLVIYQDRKRPSGIPERFSGSNSGN TATLTITGTQAMDEADYYCQAWHSSTVVFGGG TKLTVL | QVQLVESGGGVVQPGRSLRLSCTASGFTFSTYGMH WVRQAPGKGLEWVAVVWYGGNNKDYADSVKGR FTISRDISKNTLYLQMNSLRAEDTAVYYCARDRDY CSGGSCPYYYYYGMDVWGQGTTVTVSS |
| | | | | SEQ ID NO: 27382 | SEQ ID NO: 31388 |

FIGURE 50

| iPS:436742 | 21-225_154C4 | NA | TCCTATGAGCTGACTCAGCCACCCTCAGTGTC CGTGTCCCCAGGACAGACAGCCAGAATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG ATACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27383 | SEQ ID NO: 31389 |
| | | AA | SYELTQPPSVSVSPGQTARITCSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLIQPGGSLRLSCAASGFTFSSYAMSW VRQAPGKGLEWVSVISGGGSSTYYADSVKGRFTIS RDNSKNTLYLQMNSLRAEDTAVYYCAKWRGNPT DYGMDVWGQGTTVTVSS |
| | | | SEQ ID NO: 27384 | SEQ ID NO: 31390 |
| iPS:436744 | 21-225_154F4 | NA | TCCTATGAGCTGACTCAGCCACCCTCAGTGTC CGTGTCCCCAGGACAGACAGCCAGCATCACCT GCTCTGGAGATAAATTGGGAAATAAATATGTT TGTTGGTATCAGCAGAAGCCAGGCCAGTCCCC TGTGGAAGTCATCTATAAAGATAGTAAGCGGC CCTCAGGGATCCCTGAGCGATTCTCTGGCTCC AATTCTGGGAACACAGGCACTCTGACCATCAG CGGGACCCAGGCTATGGATGAGGCTGACTATT ACTGTCAGGCGTGGGACAACAGTACTTTAGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGATTC CTATTGTAGTGGTACCAGCTGCCCCTACTACTAC TACTACGGTATGGACGTCTGGGGCCAAGGGACC ACGGTCACCGTCTCCTCA |

FIGURE 50

| | | | SEQ ID NO: 27385 | SEQ ID NO: 31391 |
|---|---|---|---|---|
| | | AA | SYELTQPPSVSVSPGQTASITCSGDKLGNKYVCW YQQKPGQSPVEVIYKDSKRPSGIPERFSGSNSGN TGTLTISGTQAMDEADYYCQAWDNSTLVFGGG TKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCAREDSYC SGTSCPYYYYYGMDVWGQGTTVTVSS |
| | | | SEQ ID NO: 27386 | SEQ ID NO: 31392 |
| iPS:436746 | 21-225_154E10 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGTCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAACAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27387 | SEQ ID NO: 31393 |
| | | AA | SYELTQPPSVSVSPGQTASITCSGDKLGDKYASW YQQKPGQSPVLVIYQDSKRPSGIPERFSGSNSGN TATLTISGIQAMDEADYYCQAWDNSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27388 | SEQ ID NO: 31394 |

FIGURE 50

| iPS:436748 | 21-225_154D11 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAAGAAGCGGC CCTCAGGGATCCCTGAGCGATTCTCTGGCTCC AACTCTGGGAACATAGCCACTCTGACCATCAG CGGGACCCAGGCTATGGATGAGGCTGACTATT ACTGTCAGGCGTGGCACAGCAGTATTGTGGTA TTCGGCGGAGGGACCAAGCTGACCGTCCTA | CAGGTGCAGCTGGTGGAGTCTGGGGGAGGGGTG GTCCAGCCTGGGAGGTCCCTGAGACTCTCCTGTG CAGCGTCTGGATTCAACGTCAGTACCTATGGCAT GCACTGGGTCCGCCAGGCTCCAGGCAAGGGGCT GGAGTGGGTGGCAGTTATATGGTATGGTGGAAGT AATAAAGACTATGCAGACTCTGTGAAGGGCCGA TTCACCATCTCCAGAGACAGTTCCAAGAACACGC TGTATCTGCAAATGAACAGCCTGAGAGCCGAGG ACACGGCTGTGTATTACTGTGCGAGAGATCGGGA TTATTGTAGTGGTGGTAGTTGCCCCTTACTACTACT ACTACGGTATGGACGTCTGGGGCCAAGGGACCA CGGTCACCGTCTCCTCA |
|---|---|---|---|---|
| | | | SEQ ID NO: 27389 | SEQ ID NO: 31395 |
| | | AA | SYELTQPPSVSVSPGQTASITCSGDKLGDKYACW YQQKPGQSPVLVIYQDKKRPSGIPERFSGSNSGNI ATLTISGTQAMDEADYYCQAWHSSIVVFGGGTK LTVL | QVQLVESGGGVVQPGRSLRLSCAASGFNVSTYGM HWVRQAPGKGLEWVAVIWYGGSNKDYADSVKGR FTISRDSSKNTLYLQMNSLRAEDTAVYYCARDRDY CSGGSCPYYYYYGMDVWGQGTTVTVSS |
| | | | SEQ ID NO: 27390 | SEQ ID NO: 31396 |

FIGURE 50

| iPS:436750 | 21-225_154G12 | NA | CAGTCTGTACTGACTCAGCCACCCTCAGCGTC TGGGACCCCCGGGCAGAGGGTCACCATCTCTT GTTCTGGAAGCAGCTCCAACATCGGAAATAAT GCTGTAAGCTGGTATCAGCAGCTCCCAGGAAC GGCCCCCAAACTCCTCATCTATAGTAATGATC ACCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AAGGGGTCCGGTATTCGGCGGAGGGACCAAGC TGACCGTCCTA | CAGGTGCAGCTGCAGGAGTCGGGCCCAGGACTG GTGAATCCTTCACAGACCCTGTCCCTCACCTGCG GTGTCTCTGGTGGCTCCATCAGCAGTGGTTATTA CTACTGGAGCTGGATCCGCCAGCACCCAGGGAA GGGCCTGGAGTGGATTGGGTACATCTATTATAGT GGGAGCACCTACTACAACCCGTCCCTCAAGAGTC GAGTTTCCATATCATTAGACACGCCTAAGAACCA GTTCTCCCTGAAGCTGACCTCTGTGACTGCCGCG GACACGGCCGTGTATTACTGTGCGAGAGATTGGG GTGGCTACGATTCGAGTGGCTGGTTCGACCCCTG GGGCCAGGGAACCCTGGTCACCGTCTCCTCA |
| | | | SEQ ID NO: 27391 | SEQ ID NO: 31397 |
| | | AA | QSVLTQPPSASGTPGQRVTISCSGSSSNIGNNAVS WYQQLPGTAPKLLIYSNDHRPSGVPDRFSGSKS GTSASLAISGLQSEDEADYYCAAWDDSLKGPVF GGGTKLTVL | QVQLQESGPGLVNPSQTLSLTCGVSGGSISSGYYY WSWIRQHPGKGLEWIGYIYYSGSTYYNPSLKSRVSI SLDTPKNQFSLKLTSVTAADTAVYYCARDWGGYD SSGWFDPWGQGTLVTVSS |
| | | | SEQ ID NO: 27392 | SEQ ID NO: 31398 |
| iPS:436752 | 21-225_155H1 | NA | CAGTCTGTGCTGACGCAGCCGCCCTCAGTGTC TGGGGCCCCAGGGCAGAGGGTCACCATCTCCT GCACTGGGAGCAGCTCCAATATCGGGGCAGGT TATGATGTACACTGGTACCAGCAGCTTCCAGG AACAGCCCCCAAACTCCTCATCTATGGTAACA GCAATCGGCCCTCAGGGGTCCCTGACCGATTC TCTGGCTCCAAGTCTGGCACCTCAGCCTCCCT GGCCATCACTGGCCTCCAGGCTGAGGATGAGG CTGATTATCACTGCCAGTCCTATGACAGCAGC CTGAGTGGTCCTGTGATATTCGGCGGAGGGAC CAAGCTGACCGTCCTA | GAGGTGCAGCTAGTGCAGTCTGGAGCAGAGGTG AAAAAGCCCGGGGAGTCTCTGAAGATCTCCTGTA AGGGTTCTGGATACAGCTTTACCAGCTACTGGAT CGGCTGGGTGCGCCAGATGCCCGGGAAAGGCCT GGAGTGGATGGGGCTCATCTATCCTGGTGCCTCT GATACCAGATACAGCCCGTCCTTCCAAGGCCAGG TCACCATCTCAGCCGACAAGTCCATCAGCACCGC CTACCTGCAGTGGAGCAGCCTGAAGGCCTCGGA CACCGCCATGTATTACTGTGCGAGACAGGCCATA GCAAGTCGAGGGAGGTACTACTACTACGGTATG GACGTCTGGGGCCAAGGGACCACGGTCACCGTC TCCTCA |
| | | | SEQ ID NO: 27393 | SEQ ID NO: 31399 |

FIGURE 50

| | | | | |
|---|---|---|---|---|
| | | AA | QSVLTQPPSVSGAPGQRVTISCTGSSSNIGAGYD VHWYQQLPGTAPKLLIYGNSNRPSGVPDRFSGS KSGTSASLAITGLQAEDEADYHCQSYDSSLSGPV IFGGGTKLTVL | EVQLVQSGAEVKKPGESLKISCKGSGYSFTSYWIG WVRQMPGKGLEWMGLIYPGASDTRYSPSFQGQVT ISADKSISTAYLQWSSLKASDTAMYYCARQAIASR GRYYYYGMDVWGQGTTVTVSS |
| | | | SEQ ID NO: 27394 | SEQ ID NO: 31400 |
| iPS:436754 | 21-225_155G3 | NA | TCCTATGAGCTGACTCAGCCACCCTCAGTGTC CGTGTCCCCAGGACAGACAGCCAGCATCACCT GCTCTGGAGATAAGTTGGGGGATAAATATGTT TGCTGGTATCAGCAGAAGCCAGGCCAGTCCCC TATGTTGGTCATCTATCAAGATAGTAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAATAGTATTTATGTC TTCGGAACTGGGACCAAGGTCACCGTCCTA | CAGGTGCAGCTGGTGGAGTCTGGGGGAGGCGTG GTCCAGCCTGGGAGGTCCCTGAGACTCTCCTGTA CAGCCTCTGGATTCACCTTCAGTAGCTATGGCAT GCACTGGGTCCGCCAGGCTCCAGGCAAGGGGCT GGAGTGGGTGGCAGTTATATCATATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGCGAGAGATACGGA GAGATGGCTACCATACTCCTACGGTATGGACGTC TGGGGCCAAGGGACCACGGTCACCGTCTCCTCA |
| | | | SEQ ID NO: 27395 | SEQ ID NO: 31401 |
| | | AA | SYELTQPPSVSVSPGQTASITCSGDKLGDKYVCW YQQKPGQSPMLVIYQDSKRPSGIPERFSGSNSGN TATLTISGTQAMDEADYYCQAWDNSIYVFGTGT KVTVL | QVQLVESGGGVVQPGRSLRLSCTASGFTFSSYGMH WVRQAPGKGLEWVAVISYDGSNKYYADSVKGRFT ISRDNSKNTLYLQMNSLRAEDTAVYYCARDTERW LPYSYGMDVWGQGTTVTVSS |
| | | | SEQ ID NO: 27396 | SEQ ID NO: 31402 |

FIGURE 50

| iPS:436756 | 21-225_146A10 | NA | TCCTATGAGCTGACTCAGCCACCCTCAGTGTC CGTGTCCCCAGGACAGACAGCCAGCATCACCT GCTCTGGAGATAAATTGGGGGATAAATATGTT TGCTGGTATCAGCAGAAGCCAGGCCAGTCCCC TGTGCTGGTCATCTTTCAAGATAGAAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTGGTG TTCGGCGGAGGGACCAAAGTTACCGTCCTA | CAGGTGCAGCTGGAGGAGTCTGGGGGAGGCGTG GTCCAGCCTGGGAGGTCCCTGAGACTCTCCTGTG CAGCGTCTGGATTCACCTTCAGTGGCTATGGCAT GCACTGGGTCCGCCAGGCTCCAGGCAAGGGGCT GGAGTGGATGACACTTATACGGTATGATGGAAG CGATAAAAACTATGCAGACTCCGTGAAGGGCCG ATTCACCATCTCCAGAGACAACTCCAAGAACACG CTGTATCTGCAAATGAACAGCCTGAGAGCCGAG GACACGGCTGTGTATTACTGTGCGAGAGATCGGG TTTTTTGTAGTAGTACCAGCTGCCTCTCTTACTAC TACTACTACGGTATGGACGTCTGGGGCCAAGGG ACCACGGTCACCGTCTCCTCA |
|---|---|---|---|---|
| | | | SEQ ID NO: 27397 | SEQ ID NO: 31403 |
| | | AA | SYELTQPPSVSVSPGQTASITCSGDKLGDKYVCW YQQKPGQSPVLVIFQDRKRPSGIPERFSGSNSGN TATLTISGTQAMDEADYYCQAWDSSTVVFGGG TKVTVL | QVQLEESGGGVVQPGRSLRLSCAASGFTFSGYGMH WVRQAPGKGLEWMTLIRYDGSDKNYADSVKGRF TISRDNSKNTLYLQMNSLRAEDTAVYYCARDRVFC SSTSCLSYYYYYGMDVWGQGTTVTVSS |
| | | | SEQ ID NO: 27398 | SEQ ID NO: 31404 |

FIGURE 50

| iPS:436758 | 21-225_155C10 | NA | TCCTATGAGCTGACTCAGCCACCCTCAGTGTC CGTGTCCCCAGGACAGACAGTCAGCATCACCT GCTCTGGAGATAAAATTGGGGGATAAATATGTT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
|  |  |  | SEQ ID NO: 27399 | SEQ ID NO: 31405 |
|  |  | AA | SYELTQPPSVSVSPGQTVSITCSGDKLGDKYVSW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
|  |  |  | SEQ ID NO: 27400 | SEQ ID NO: 31406 |
| iPS:436760 | 21-225_155E10 | NA | TCCTATGAGCTGACTCAGCCGCCCTCAGTGTC CGTGTCCCCAGGACAGACAGCCAGCATCACCT GCTCTGGAGATAAAATTGGGGGATAAATATGTT TCCTGGTATCAGCAGAAGCCAGGCCAGTCCCC TGTGCTGGTCATCTATCAAGATAGGAAGCGGC CCTCAGGGACCCCTGAGCGATTCTCTGGCTCC AACTCTGGGAACACAGCCACTCTGACCATCAG CGGGACCCAGGCTATGGATGAGGCTGACTATT ACTGTCTGGCGTGGGACAGCAGCACTTTTGTG GTATTCGGCGGAGGGACCAAGTTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGTGA CTACGGTGACCCCCCCTACTACTACTACTACGGT ATGGACGTCTGGGGCCAAGGGACCACGGTCACC GTCTCCTCA |
|  |  |  | SEQ ID NO: 27401 | SEQ ID NO: 31407 |

FIGURE 50

| | | | | |
|---|---|---|---|---|
| | | AA | SYELTQPPSVSVSPGQTASITCSGDKLGDKYVSW YQQKPGQSPVLVIYQDRKRPSGTPERFSGSNSGN TATLTISGTQAMDEADYYCLAWDSSTFVVFGGG TKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARDRDY GDPPYYYYYGMDVWGQGTTVTVSS |
| | | | SEQ ID NO: 27402 | SEQ ID NO: 31408 |
| iPS:436762 | 21-225_156H2 | NA | CAGTCTGTGGTGACTCAGCCACCCTCAGCGTC TGGGACCCCCGGGCAGAGGGTCACCATCTCTT GTTCTGGAAGCAGCTCCAACATCGGAAGTAAT ACTGTAAATTGGTACCAGCAGCTCCCAGGAAC GGCCCCCAAACTCCTCATCTATAGTAGTAATC AGCGGCCCTCAGGGGTCCCTGACCGATTGTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AATGGCGTGGTATTCGGCGGAGGGACCAAGG TGACCGTCCTA | GAGGTGCAGCTGGTGGAGTCTGGGGGAGGCTTG ATACAGCCTGGGGGGTCCCTGAGACTCTCCTGTG CAGCCTCTGGATTCACCTTCAGTAACTATAACAT GAACTGGGTCCGCCAGGCTCCAGGGAAGGGGCT GGAGTGGGTTTCATACATTAGTAGAAGTAGTAAT ACCAAATACTACGCAGACTCTGTGAAGGGCCGA TTCACCATCTCCAGAGACAATGCCAAGAACTCAC TGTATCTGCAAATGAACAGCCTGAGAGACGAGG ACACGGCTGTGTATTACTGTGCGAGAGATAGGAG TGGGAGCTACGGGTACTTCTACTACTACGGTATG GACGTCTGGGGCCAAGGGACCACGGTCACCGTC TCCTCA |
| | | | SEQ ID NO: 27403 | SEQ ID NO: 31409 |
| | | AA | QSVVTQPPSASGTPGQRVTISCSGSSSNIGSNTVN WYQQLPGTAPKLLIYSSNQRPSGVPDRLSGSKSG TSASLAISGLQSEDEADYYCAAWDDSLNGVVFG GGTKVTVL | EVQLVESGGGLIQPGGSLRLSCAASGFTFSNYNMN WVRQAPGKGLEWVSYISRSSNTKYYADSVKGRFTI SRDNAKNSLYLQMNSLRDEDTAVYYCARDRSGSY GYFYYYGMDVWGQGTTVTVSS |
| | | | SEQ ID NO: 27404 | SEQ ID NO: 31410 |

FIGURE 50

| iPS:436764 | 21-225_158E9 | NA | TCCTATGACCTGACTCAGCCACCCTCAGTGTC CGTGTCCCCAGGACAGACAGCCAGCATCACCT GCTCTGGAGATAAATTGGGGGATAAATATGTT TGCTGGTATCAGCAGAAGCCAGGCCAGTCCCC TGTGCTGGTCATCTATCAAGATAGTAAGCGGC CCTCAGGGATCCCTGAGCGATTCTCTGGCTCC AACTCTGGGAACACAGCCACTCTGACCATCAC CGGGACCCAGGCTATGGATGAGGCTAACTATT ACTGTCAGGCGTGGGACAACAGCAGCTTTGTG CTATTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AGTAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGT TTTTTGTAGTGGTACCAGCTGCCCTTACTACTACT ACTACGGTATGGACGTCTGGGGCCAAGGGACCA CGGTCACCGTCTCCTCA |
| | | | SEQ ID NO: 27405 | SEQ ID NO: 31411 |
| | | AA | SYDLTQPPSVSVSPGQTASITCSGDKLGDKYVC WYQQKPGQSPVLVIYQDSKRPSGIPERFSGSNSG NTATLTITGTQAMDEANYYCQAWDNSSFVLFG GGTKLTVL | QVQLVESGGGVVQPGRSLRLSCAASGFTFSSYGMH WVRQAPGKGLEWVAVIWYDGSSKYYADSVKGRF TISRDNSKNTLYLQMNSLRAEDTAVYYCARDRVFC SGTSCPYYYYYGMDVWGQGTTVTVSS |
| | | | SEQ ID NO: 27406 | SEQ ID NO: 31412 |

FIGURE 50

| iPS:436766 | 21-225_158D10 | NA | TCCTATGAGCTGACTCAGCCACCCTCAGTGAC CGTGTCCCCAGGACAGACAGCCAGCATCACCT GCTCTGGAGATAAATTGGGGGATAAATATGTT TGCTGGTATCAGCAGAAGCCAGGCCAGTCCCC TGTGCTGGTCATCTATCAAGATCGCAAGCGGC CCTCAGGGATCCCTGAGCGATTGTCTGGCTCC AACTCTGGGAACACAGCCACTCTGACCATCAG CGGGACCCAGGCTCTGGATGAGGCTGACTATT ACTGTCAGGCGTGGGGCAACAGCAGCTTTGTG GTATTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGT TTCTTGTAGTAGTACCAGCTGCCCTTACTACTACT ACTACGGTATGGACGTCTGGGGCCAAGGGACCA CGGTCACCGTCTCCTCA |
|---|---|---|---|---|
| | | | SEQ ID NO: 27407 | SEQ ID NO: 31413 |
| | | AA | SYELTQPPSVTVSPGQTASITCSGDKLGDKYVC WYQQKPGQSPVLVIYQDRKRPSGIPERLSGSNSG NTATLTISGTQALDEADYYCQAWGNSSFVVFGG GTKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARDRVSC SSTSCPYYYYYGMDVWGQGTTVTVSS |
| | | | SEQ ID NO: 27408 | SEQ ID NO: 31414 |

FIGURE 50

| iPS:436768 | 21-225_159H8 | NA | TCCTATGAGCTGACTCAGCCACCCTCAGTGTC CGTGTCCCCAGGACAGACAGCCAGCATCACCT GCTCTGGAGATAAATTGGGGGATAAATATGTT TGCTGGTATCAGCAGAAGCCAGGCCAGTCCCC TGTGTTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTCTGGATGAGGCTGACTATT ACTGTCAGGCGTGGGGCAACAGCAGCTTTGTG GTATTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGT TTCTTGTAGTAGTACCAGCTGCCCTTACTACTACT ACTACGGTATGGACGTCTGGGGCCAAGGGACCA CGGTCACCGTCTCCTCA |
| | | | SEQ ID NO: 27409 | SEQ ID NO: 31415 |
| | | AA | SYELTQPPSVSVSPGQTASITCSGDKLGDKYVCW YQQKPGQSPVLVIYQDRKRPSGIPERFSGSNSGN TATLTISGTQALDEADYYCQAWGNSSFVVFGGG TKLTVL | QVQLVESGGGVVQPGRSLRLSCAASGFTFSTYGMH WVRQAPGKGLEWVAVIWYDGSNKYYADSVKGRF TISRDNSKNTLYLQMNSLRAEDTAVYYCARDRVSC SSTSCPYYYYYGMDVWGQGTTVTVSS |
| | | | SEQ ID NO: 27410 | SEQ ID NO: 31416 |

FIGURE 50

| iPS:436770 | 21-225_160B12 | NA | TCCGATGAGCTGACTCAGTCACCCTCAGTGTC CGTGTCCCCAGGACAGACAGCCAGCATCACCT GCTCTGGAGATAAATTGGGGGATAAATATGTT TGCTGGTATCAGCAGAAGCCAGGCCAGTCCCC TGTGTTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTCTGGATGAGGCTGACTATT ACTGTCAGGCGTGGGGCAACAGCAGCTTTGTG GTATTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGT TTCTTGTAGTAGTACCAGCTGCCCTTACTACTACT ACTACGGTATGGACGTCTGGGGCCAAGGGACCA CGGTCACCGTCTCCTCA |
|---|---|---|---|---|
| | | | SEQ ID NO: 27411 | SEQ ID NO: 31417 |
| | | AA | SDELTQSPSVSVSPGQTASITCSGDKLGDKYVCW YQQKPGQSPVLVIYQDSKRPSGIPERFSGSNSGN TATLTISGTQALDEADYYCQAWGNSSFVVFGGG TKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARDRVSC SSTSCPYYYYYGMDVWGQGTTVTVSS |
| | | | SEQ ID NO: 27412 | SEQ ID NO: 31418 |

FIGURE 50

| iPS:436772 | 21-225_161H3 | NA | TCCTATGAGCTGACTCAGCCACCCTCAGTGTC CGTGTCCCCAGGACAGACAGCCAGCATCACCT GCTCTGGAGATAGATTGGGGGATAAATATGTT TGCTGGTATCAGCAGAAGCCAGGCCAGTCCCC TGTACTGGTCATCTTTCAAGATAACAAGCGGC CCTCAGGGATCCCTGAGCGATTCTCTGGCTCC AACTCTGGGAACACAGCCACTCTGACCATCAG CGGGACCCAGGCTCTGGATGAGGCTGACTATT ACTGTCAGGCGTGGGTCAACAACACTGCAGTG GTTTTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGTCGGGTA TAGCGGTGGCTGGTACATCTTTGACTACTGGGGC CAGGGAACCCTGGTCACCGTCTCCTCA |
| | | | SEQ ID NO: 27413 | SEQ ID NO: 31419 |
| | | AA | SYELTQPPSVSVSPGQTASITCSGDRLGDKYVCW YQQKPGQSPVLVIFQDNKRPSGIPERFSGSNSGN TATLTISGTQALDEADYYCQAWVNNTAVVFGG GTKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARVGYSG GWYIFDYWGQGTLVTVSS |
| | | | SEQ ID NO: 27414 | SEQ ID NO: 31420 |
| iPS:436774 | 21-225_161E10 | NA | TCCTTTGACCTGACTCAGCCACCCTCAGTGTCC GTGTCCCCAGGACAGACAGCCAGCATCACCTG CTCTGGAGATAAATTGGGGGATAAATATGTTT GCTGGTATCAGCAGAAGCCAGGCCAGTCCCCT GTGCTGGTCATCTATCAAGATAGCAAGCGGCC CTCAGGGATCCCTGAGCGAATCTCTGGCTCCA ACTCTGGGAACACAGCCACTCTGACCATCAGC GGGACCCAGGCTATGGATGAGGCTGACTATTA CTGTCAGACGTGGGACAACAGTAGTTTTGCGC TTTTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGATCGGGTT TTTGTAGTGGTACCAGCTGCCCTTACTACTACTA CTACGGTATGGACGTCTGGGGCCAAGGGACCAC GGTCACCGTCTCCTCA |
| | | | SEQ ID NO: 27415 | SEQ ID NO: 31421 |

FIGURE 50

| | | | | |
|---|---|---|---|---|
| | | AA | SFDLTQPPSVSVSPGQTASITCSGDKLGDKYVCW YQQKPGQSPVLVIYQDSKRPSGIPERISGSNSGNT ATLTISGTQAMDEADYYCQTWDNSSFALFGGGT KLTVL | QVQLVESGGGVVQPGRSLRLSCAASGFTFSSYGMH WVRQAPGKGLEWVAVIWYDGSNKYYVDSVKGRF TISRDNSKNTLYLQMNSLRAEDTAVYYCARDRVFC SGTSCPYYYYYGMDVWGQGTTVTVSS |
| | | | SEQ ID NO: 27416 | SEQ ID NO: 31422 |
| iPS:436776 | 21-225_161F12 | NA | TCCTATGAGCTGACTCAGCCACCCTCAGTGTC CGTGTCCCCAGGACAGACAGCCAGCATCACCT GCTCTGGAGATAAATTGGGTGATAAATATGCT TGCTGGTATCAGCAGAAGCCAGGCCAGTCCCC TGTGCTGGTCATCTATCAAGATACCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCACCACTCTGGTT TTCGGCGGAGGGACCAAGCTGACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGTACATCTATTACAGT GGGAGCCCCTACTACAACCCGTCCCTCAAGAGTC GAATTACCATATCAATAGACACGTCTAAGAACCA GTTCTCCCTGAAGCTGAACTCTGTGACTGCCGCG GACACGGCCGTGTATTACTGTGCGAGATCGAATT GTAGTAGTGCCAACTGCTATACGGTGGGGTTCTA CTACTACGGTTTGGACGTCTGGGGCCGAGGGACC ACGGTCACCGTCTCCTCA |
| | | | SEQ ID NO: 27417 | SEQ ID NO: 31423 |
| | | AA | SYELTQPPSVSVSPGQTASITCSGDKLGDKYACW YQQKPGQSPVLVIYQDTKRPSGIPERFSGSNSGN TATLTISGTQAMDEADYYCQAWDSTTLVFGGG TKLTVL | QVQLQESGPGLVKPSQTLSLTCTVSGGSISSGGYYW SWIRQHPGKGLEWIGYIYYSGSPYYNPSLKSRITISI DTSKNQFSLKLNSVTAADTAVYYCARSNCSSANCY TVGFYYYGLDVWGRGTTVTVSS |
| | | | SEQ ID NO: 27418 | SEQ ID NO: 31424 |

FIGURE 50

| iPS:436780 | 21-225_165H3 | NA | TCCTATGAGCTGACTCAGCCACCCTCAGTGTC CGTGTCCCCAGGACAGACAGCCAGCATCACCT GCTCTGGAGATAAATTGGGTGATAAATATGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCACCACTCTGGTT TTCGGCGGAGGGACCAAGCTGACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGGCCTGGAGTGGATTGGGTACATCTATTACAGT GGGAGCCCCTACTACAATCCGTCCCTCAAGAGTC GAATTACCATATCAATAGACACGTCTAAGAACCA GTTCTCCCTGAAGCTGAACTCTGTGACTGCCGCG GACACGGCCGTGTATTACTGTGCGAGATCGAATT GTAGTAGTGCCCAACTGCTATACGGTGGGGTTCTA CTACTACGGTATGGACGTCTGGGGCCAAGGGAC CACGGTCACCGTCTCCTCA |
|---|---|---|---|---|
| | | | SEQ ID NO: 27419 | SEQ ID NO: 31425 |
| | | AA | SYELTQPPSVSVSPGQTASITCSGDKLGDKYACW YQQKPGQSPVLVIYQDSKRPSGIPERFSGSNSGN TATLTISGTQAMDEADYYCQAWDSTTLVFGGG TKLTVL | QVQLQESGPGLVKPSQTLSLTCTVSGGSISSGGYYW SWIRQHPGKGLEWIGYIYYSGSPYYNPSLKSRITISI DTSKNQFSLKLNSVTAADTAVYYCARSNCSSANCY TVGFYYYGMDVWGQGTTVTVSS |
| | | | SEQ ID NO: 27420 | SEQ ID NO: 31426 |

2504

FIGURE 50

| iPS:436782 | 21-225_166G11 | NA | TCCTATGAGCTGAGTCAGCCACCCTCAGTGTC CGTGTCCCCAGGACAGACAGCCAGCATCACCT GCTCTGGAGATAAATTGGGGGATAAATATGTT CACTGGTATCAGCAGAAGCCAGGCCAGTCCCC TGTGTTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGATTATT ACTGTCAGGCGTGGGACAACAGCACTGCGGTA TTCGGCGGAGGGACCAAGTTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAATGGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACACTTCCAAGAACACGC TGTTTCTGCAAATGAACAGCCTGACAGCCGAGGA CACGGCTGTGTATTACTGTGCGAGAGATGATAGA TATTGTAGTAGTCCCACCTGCCATCCTTACTACTA CTACTACGGTCTGGACGTCTGGGGCCAAGGGACC ACGGTCACCGTCTCCTCA |
| | | | SEQ ID NO: 27421 | SEQ ID NO: 31427 |
| | | AA | SYELSQPPSVSVSPGQTASITCSGDKLGDKYVHW YQQKPGQSPVLVIYQDSKRPSGIPERFSGSNSGN TATLTISGTQAMDEADYYCQAWDNSTAVFGGG TKLTVL | QVQLVESGGGVVQPGRSLRLSCAASGFTFNGYGM HWVRQAPGKGLEWVAVIWYDGSNKYYADSVKGR FTISRDTSKNTLFLQMNSLTAEDTAVYYCARDDRY CSSPTCHPYYYYYGLDVWGQGTTVTVSS |
| | | | SEQ ID NO: 27422 | SEQ ID NO: 31428 |

FIGURE 50

| iPS:436784 | 21-225_169C1 | NA | TCCTATGAGCTGACTCAGCCACCCTCAGTGTC CGTGTCCCCAGGACAGACAGCCAGCATCACCT GCTCTGGAGATAAATTGGGGGATAAATATGTT TGTTGGTATCAGCAGAAGCCAGGCCAGTCCCC TTTGCTGGTCATCTATAAAGATATCAAGCGGC CCTCAGGGATCCCTGAGCGATTCTCTGGCTCC AACTCTGGTAACACAGCCACTCTGACCATCAG CGGGACCCAGGCTATGGATGAGGCTGACTATT ACTGTCAGGCGTGGGACACCAACACTGTGATA TTCGGCGGAGGGACCAAGCTGACCGTCCTA | CAGGTGCAGTTGGTGGAGTCTGGGGGAGGCGTG GTCCAGCCTGGGAGGTCCCTGAGACTCTCCTGTG CAGCGTCTGGATTCACTTTGAGTAGCAATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACACTTCCAAGAACACGC TGTATCTGCAAATGAACAGCCTGAGAGCCGAGG ACACGGCTGTGTATTACTGTGCGAGAGATCAGTA CAACCGGAACGACGGACCACCAGCTTACTACTA CTACTACGGTTTGGACGTCTGGGGCCAAGGGACC ACGGTCACCGTCTCCTCA |
|---|---|---|---|---|
| | | | SEQ ID NO: 27423 | SEQ ID NO: 31429 |
| | | AA | SYELTQPPSVSVSPGQTASITCSGDKLGDKYVCW YQQKPGQSPLLVIYKDIKRPSGIPERFSGSNSGNT ATLTISGTQAMDEADYYCQAWDTNTVIFGGGT KLTVL | QVQLVESGGGVVQPGRSLRLSCAASGFTLSSNGMH WVRQAPGKGLEWVAVIWYDGSNKYYADSVKGRF TISRDTSKNTLYLQMNSLRAEDTAVYYCARDQYNR NDGPPAYYYYYGLDVWGQGTTVTVSS |
| | | | SEQ ID NO: 27424 | SEQ ID NO: 31430 |

FIGURE 50

| iPS:436786 | 21-225_169A6 | NA | TCCTATGAGCTGACTCAGCCACCCTCAGTGTC CGTGTCCCCAGGACAGACAGCCAGCATCACCT GCTCTGGAGATAAATTGGGGGATAAATATGTT TGTTGGTATCAGCGGAAGCCAGGCCAGTCCCC TGTTCTGGTCATCTATCAGGATTACAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACACCAACACTGTGCTT TTCGGCGGAGGGACCAAGCTGACCGTCCTG | CAGGTGCAGCTGGTGGAGTCTGGGGGAGGCGTG GTCCAGCCTGGGAGGTCCCTGAGACTCTCCTGTG CAGCGTCTGGATTCACTTTGAGCAGCAATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTCTCTGCAAATGAACAGCCTGAGAGCCGAGG ACACGGCTGTGTATTACTGTGCGAGAGATCAGTA CAACCGGAACGACGGACCACCAGCTTACTACTA CTACTACGGTATGGACGTCTGGGGCCAAGGGAC CACGGTCACCGTCTCCTCA |
|---|---|---|---|---|
| | | | SEQ ID NO: 27425 | SEQ ID NO: 31431 |
| | | AA | SYELTQPPSVSVSPGQTASITCSGDKLGDKYVCW YQRKPGQSPVLVIYQDYKRPSGIPERFSGSNSGN TATLTISGTQAMDEADYYCQAWDTNTVLFGGG TKLTVL | QVQLVESGGGVVQPGRSLRLSCAASGFTLSSNGMH WVRQAPGKGLEWVAVIWYDGSNKYYADSVKGRF TISRDNSKNTLSLQMNSLRAEDTAVYYCARDQYNR NDGPPAYYYYYGMDVWGQGTTVTVSS |
| | | | SEQ ID NO: 27426 | SEQ ID NO: 31432 |

FIGURE 50

| iPS:436788 | 21-225_169B7 | NA | GCCTATGACCTGACTCAGCCACCCTCAGTGTC CGTGTCCCCAGGACAGACAGCCAGAATCACCT GCTCTGGAGATAAATTGGGGGGGAAAATATGCT TCCTGGTATCAACAGAAGCCAGGCCAGTCCCC TGTGCTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAAGAACACTGTGGTA TTCGGCGGAGGGACCAAGCTGACCGTCCTA | GAGGTGCAGCTGGTGGAGTCTGGGGGAGGCTTG GTACAGCCTGGGGGGTCCCTGAGACTCTCCTGTG CAGCCTCTGGATTCACCTTCAGTAGCTATAGCTT GAACTGGGTCCGCCAGGCTCCAGGGAAGGGGCT GGAGTGGGTTTCATACATTGGTAGTAGTGGCAGT ATCATATTCTACGCAGACTCTGTGAAGGGCCGAT TCACCATCTCCAGAGACAATGCCAAGAACTCACT GTATCTGCAAATGAACAGCCTGAGAGACGAGGA CACGGCTGTGTATTACTGTGCGAGAGGGGATACA GCTGGGGGTTACCTATTACTACGGTATGGACGTCT GGGGCCAAGGGACCACGGTCACCGTCTCCTCA |
| | | | SEQ ID NO: 27427 | SEQ ID NO: 31433 |
| | | AA | AYDLTQPPSVSVSPGQTARITCSGDKLGGKYAS WYQQKPGQSPVLVIYQDRKRPSGIPERFSGSNSG NTATLTISGTQAMDEADYYCQAWDKNTVVFGG GTKLTVL | EVQLVESGGGLVQPGGSLRLSCAASGFTFSSYSLN WVRQAPGKGLEWVSYIGSSGSIIFYADSVKGRFTIS RDNAKNSLYLQMNSLRDEDTAVYYCARGDTAGVT YYYGMDVWGQGTTVTVSS |
| | | | SEQ ID NO: 27428 | SEQ ID NO: 31434 |
| iPS:436790 | 21-225_169G11 | NA | TCCTATGAGCTGACTCAGCCACCCTCAGTGTC CGTGTCCCCAGGACAGACAGCCAGCATCACCT GCTCTGGAGATAAATTGGGGGGATAAATATGCT TGCTGGTATCAGCAGAAGCCAGGCCAGTCCCC TGTGCTGGTCATCTATCAAGATAGTAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAGCACTGCGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGGTGTGTATTACTGTGCGAGAGAGGGGG CTACGTATTACCATGGTTCGGGGAGTTATTATCC GGCTACTAACTACGGTATGGACGTCTGGGGGCCAA GGGACCACGGTCACCGTCTCCTCA |

EP 4 435 105 A2

FIGURE 50

| | | | SEQ ID NO: 27429 | SEQ ID NO: 31435 |
|---|---|---|---|---|
| | | AA | SYELTQPPSVSVSPGQTASITCSGDKLGDKYACW YQQKPGQSPVLVIYQDSKRPSGIPERFSGSNSGN TATLTISGTQAMDEADYYCQAWDNSTAVFGGG TKLTVL | QVQLVESGGGVVQPGRSLRLSCAASGFTFSSYGMH WVRQAPGKGLEWVAIIWYDGSNKYYADSVKGRFT ISRDNSKNTLYLQMNSLRAEDTGVYYCAREGATY YHGSGSYYPATNYGMDVWGQGTTVTVSS |
| | | | SEQ ID NO: 27430 | SEQ ID NO: 31436 |
| iPS:436792 | 21-225_169D12 | NA | AATTTTATGCTGACTCAGCCCCACTCTGTGTCG GAGTCTCCGGGGAAGACGGTAACCATCTCCTG CACCCGCAGCAGTGGCAGCATTACCGGCAACT ATGTGCAGTGGCACCAGCAGCGCCCGGGCAAT TCCCCCACCACTTTGATCTATGAGGATAAAAA AAGACCCTCTGGGGTCCCTGATCGGTTCTCTG GCTCCATCGACAGCTCCTCCAACTCTGCCTCC CTCACCATCTCTGGACTGAAGACTGAGGACGA GGCTGACTATTACTGTCAGTCTTATTATAGCG GCAATTGGGTGTTCGGCGGAGGGACCAAGCTG ACCGTCCTG | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGTCCCCTTTAC GATATGGGACTCTACTACGATATGGACGTCTGGG GCCAAGGGACCACGGTCACCGTCTCCTCA |
| | | | SEQ ID NO: 27431 | SEQ ID NO: 31437 |
| | | AA | NFMLTQPHSVSESPGKTVTISCTRSSGSITGNYVQ WHQQRPGNSPTTLIYEDKKRPSGVPDRFSGSIDS SSNSASLTISGLKTEDEADYYCQSYYSGNWVFG GGTKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCASPLYDM GLYYDMDVWGQGTTVTVSS |
| | | | SEQ ID NO: 27432 | SEQ ID NO: 31438 |

2509

FIGURE 50

| iPS:436794 | 21-225_170F1 | NA | TCCTATGAGTTGAGTCAGCCACCCTCAGTGTC CGTGTCCCCAGGACAGACAGCCAGCATCACCT GCTCTGGAGATAAATTGGGGGATAAATATTCT TGCTGGTATCAGCAGAAGCCAGGCCAGTCCCC TGTGCTGGTCATCTATCAAGATAGTAAGCGGC CCTCAGGGATCCCTGCACGATTCTCTGGCTCC AACTCTGGGAACACAGCCACTCTGACCATCAG CGGGACCCAGGCTATGGATGAGGCTGATTATT ACTGTCAGGCGTGGGACAGCAACACTGCGGTA TTCGGCGGAGGGACCAAACTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGGCTATGGCAT GAACTGGGTCCGCCAGGCTCCAGGCAAGGGGCT GGAGTGGGTGGCAATTATATGGTATGATGGAAAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGT TTATTGTAGTAGTACCAGCTGCCATCCCTATTACT ACTACTACGCTATGGACGTCTGGGGGCCAAGGGA CCACGGTCACCGTCTCCTCA |
|---|---|---|---|---|
| | | | SEQ ID NO: 27433 | SEQ ID NO: 31439 |
| | | AA | SYELSQPPSVSVSPGQTASITCSGDKLGDKYSCW YQQKPGQSPVLVIYQDSKRPSGIPARFSGSNSGN TATLTISGTQAMDEADYYCQAWDSNTAVFGGG TKLTVL | QVQLVESGGGVVQPGRSLRLSCAASGFTFSGYGM NWVRQAPGKGLEWVAIIWYDGNNKYYADSVKGR FTISRDNSKNTLYLQMNSLRAEDTAVYYCARDRVY CSSTSCHPYYYYYAMDVWGQGTTVTVSS |
| | | | SEQ ID NO: 27434 | SEQ ID NO: 31440 |

FIGURE 50

| iPS:436796 | 21-225_170A5 | NA | TCCTATGACCTGACTCAGCCACCCTCAGTGTC CGTGTCCCCAGGACAGACAGCCAGCATCACCT GCTCTGGAGATAAATTGGGGGATAAATATGCT TGTTGGTATCAGCAGAAGCCAGGCCAGTCCCC TATACTGGTCATCTATCAAGATTACAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ATTGTCAGGCGTGGGACAACAGCACTATGGTA TTCGGCGGAGGGACCAGGCTGACCGTCCTA | CAGGTGCAGCTGGTGGAGTCTGGGGGAGGCGTG GTCCAGCCTGGGAGGTCCCTGAGACTCTCCTGTG CAGCGTCTGGACTCACCTTCAGTAACTGTGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGGATCTGCAAATGAACAGCCTGAGAGCCGAGG ACACGGCTGTGTATTACTGTGCGAGAGATCAGTA CAACAGGAACGACGGACCACCAGCTTACTACTA CTACTACGGTTTGGACGTCTGGGGCCAAGGGACC ACGGTCACCGTCTCCTCA |
|---|---|---|---|---|
| | | | SEQ ID NO: 27435 | SEQ ID NO: 31441 |
| | | AA | SYDLTQPPSVSVSPGQTASITCSGDKLGDKYAC WYQQKPGQSPILVIYQDYKRPSGIPERFSGSNSG NTATLTISGTQAMDEADYYCQAWDNSTMVFGG GTRLTVL | QVQLVESGGGVVQPGRSLRLSCAASGLTFSNCGM HWVRQAPGKGLEWVAIIWYDGSNKYYADSVKGR FTISRDNSKNTLDLQMNSLRAEDTAVYYCARDQYN RNDGPPAYYYYYGLDVWGQGTTVTVSS |
| | | | SEQ ID NO: 27436 | SEQ ID NO: 31442 |

FIGURE 50

| iPS:436798 | 21-225_171F5 | NA | GCCTATGACCTGACTCAGCCACCCTCAGTGTC CGTGTCCCCAGGACAGACAGCCAGCATCACCT GCTCTGGAGATAAATTGGGGGGGAAAATATGCT TCCTGGTATCAACAGAAGCCAGGCCAGTCCCC TGTGCTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAAGAACACTGTGGTA TTCGGCGGAGGGACCAAGCTGACCGTCCTA | GAGGTGCAGCTGGTGGAGTCTGGGGGAGGCTTG GTACAGCCTGGGGGGTCCCTGAGACTCTCCTGTG CAGCCTCTGGATTCACCTTCAGTAGCTATAGCTT GAACTGGGTCCGCCAGGCTCCAGGGAAGGGGCT GGAGTGGGTTTCATACATTGGTAGTAGTGGCAGT ATCATATTCTACGCAGACTCTGTGAAGGGCCGAT TCACCATCTCCAGAGACAATGCCAAGAACTCACT GTATCTGCAAATGAACAGCCTGAGAGACGAGGA CACGGCTGTGTATTACTGTGCGAGAGGGGATACA GCTGGGGTTACCTATTACTACGGTATGGACGTCT GGGGCCAAGGGACCACGGTCACCGTCTCCTCA |
|---|---|---|---|---|
|  |  |  | SEQ ID NO: 27437 | SEQ ID NO: 31443 |
|  |  | AA | AYDLTQPPSVSVSPGQTASITCSGDKLGGKYAS WYQQKPGQSPVLVIYQDRKRPSGIPERFSGSNSG NTATLTISGTQAMDEADYYCQAWDKNTVVFGG GTKLTVL | EVQLVESGGGLVQPGGSLRLSCAASGFTFSSYSLN WVRQAPGKGLEWVSYIGSSGSIIFYADSVKGRFTIS RDNAKNSLYLQMNSLRDEDTAVYYCARGDTAGVT YYYGMDVWGQGTTVTVSS |
|  |  |  | SEQ ID NO: 27438 | SEQ ID NO: 31444 |
| iPS:436800 | 21-225_171D12 | NA | TCTTCTGAGCTGACTCAGGACCCTGCTGTGTCT GTGGCCTTGGGACAGACAGTCAGGATCACATG CCAAGGAGACAGCCTCAGAAGCTATTATGCAA GCTGGTACCAGCAGAAGCCAGGACAGGCCCC TATACTTGTCATCTATGCTAAAAACAACCGGC CCTCAGGGATCCCAGACCGATTCTCTGGCTCC AACTCAGGAAACACAGCTTCCTTGACCATCAC TGGGGCTCAGGCGGAAGATGAGGCTGACTATT ACTGTAACTCCCGGGACAGCAGTGGCAGCCAT GTGGTATTCGGCGGAGGGACCAAACTGACCGT CCTA | CAGGTGCAGCTGGTGCAGTCTGGGGGCTGAGGTG AAGAAGCCTGGGGCCTCAGTGAAGGTTTCCTGCA AGGCATCTGGATACACCTTCAACAGTTACTATAT GTATTGGGTGCGACAGGCCCCTGGACAAGGGCTT GAGTGGATGGGAATAATCAACCCTAGTGGTGGT AGCACAAACTACGCACAGAAGTTCCAGGGCAGA GTCACCATGACCAGGGACACGTCCACGAGCACA CTCTACATGGAGCTGAACAGCCTGAGATCTGAGG ACACGGCCGTGTATTACTGTGCGAGTGGTTGGGA GTTAAACTACTGGGGCCAGGGAACCCTGGTCACC GTCTCCTCA |
|  |  |  | SEQ ID NO: 27439 | SEQ ID NO: 31445 |

FIGURE 50

| | | | | |
|---|---|---|---|---|
| | | AA | SSELTQDPAVSVALGQTVRITCQGDSLRSYYAS WYQQKPGQAPILVIYAKNNRPSGIPDRFSGSNSG NTASLTITGAQAEDEADYYCNSRDSSGSHVVFG GGTKLTVL | QVQLVQSGAEVKKPGASVKVSCKASGYTFNSYYM YWVRQAPGQGLEWMGIINPSGGSTNYAQKFQGRV TMTRDTSTSTLYMELNSLRSEDTAVYYCASGWELN YWGQGTLVTVSS |
| | | | SEQ ID NO: 27440 | SEQ ID NO: 31446 |
| IPS:436802 | 21-225_171E12 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATCGCAAGCGGC CCTCAGGGATCCCTGAGCGATTCTCTGGCTCC AACTCTGGGAACACAGCCACCCTGACCATCAG CGGGACCCAGGCTATGGATGAGGCTGACTATT ACTGTCAGGCGTGGGACATCAGCACTTATGTG GTATTCGGCGGAGGGACCAAACTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCAGTTATATGGAATGATGGAGG TAATAAATATAATGGAGACTCCGTGAAGGGCCG ATTCACCATCTCCAGAGACAATTCCAAGAACACG CTGTATCTGCAAATGAACAGTCTGAGAGCCGAG GACACGGCTGTGTATTACTGTGCGAGAGACCGTA CGTATTACTCTGGTTCGGGGAGCCCCCCCTACTA CTACTACTACGGTATGGACGTCTGGGGCCAAGGG ACCACGGTCACCGTCTCCTCA |
| | | | SEQ ID NO: 27441 | SEQ ID NO: 31447 |
| | | AA | SYELTQPPSVSVSPGQTASITCSGDKLGDKYACW YQQKPGQSPVLVIYQDRKRPSGIPERFSGSNSGN TATLTISGTQAMDEADYYCQAWDISTYVVFGGG TKLTVL | QVQLVESGGGVVQPGRSLRLSCAASGFTFSSYGMH WVRQAPGKGLEWVAVIWNDGGNKYNGDSVKGRF TISRDNSKNTLYLQMNSLRAEDTAVYYCARDRTYY SGSGSPPYYYYYGMDVWGQGTTVTVSS |
| | | | SEQ ID NO: 27442 | SEQ ID NO: 31448 |

FIGURE 50

| iPS:436804 | 21-225_172C3 | NA | TCTTCTGAGCTGACTCAGGACCCTGCTGTGTCT GTGGCCTTGGGACAGACAGTCAGGATCACATG CCAAGGAGACAGCCTCAGAAACTATTATGTAA GCTGGTACCAGCAGAAGCCAGGACAGGCCCC TATACTTGTCATCTATACTAAAAACAGCCGGC CCTCAGGGATCCCAGACCGATTCTCTGGCTCC ACCTCAGGAAACACAGCTTCCTTGACCATCAC TGGGACTCAGGCGGAAGATGAGGCTGACTATT ACTGTAACTCCCGGGACAGCAGTGGCAACCAT GTGGTATTCGGCGGAGGGACCAAGCTGACCGT CCTA | CAGGTGCAGCTGGTGCAGTCTGGGGGCTGAGGTG AAGAAGCCTGGGGCCTCAGTGAAGGTTTCCTGCA AGGCATCTGGATACACCTTCAGAAGTTACTATAT GTATTGGGTGCGACAGGCCCCTGGACAAGGGCTT GAGTGGGTGGGAACAATCAACCCTAGTGGTGGT AGCACAAACTACGCACAGAAGTTCCAGGGCAGA GTCACCATGACCAGGGACACGTCCACGAGCACA CTCTACATGGAGCTGAACAGCCTGAGATCTGAGG ACACGGCCGTGTATTACTGTGCGAGTGGCTGGGA GTTAAACTACTGGGGCCAGGGAACCCTGGTCACC GTCTCCTCA |
| | | | SEQ ID NO: 27443 | SEQ ID NO: 31449 |
| | | AA | SSELTQDPAVSVALGQTVRITCQGDSLRNYYVS WYQQKPGQAPILVIYTKNSRPSGIPDRFSGSTSG NTASLTITGTQAEDEADYYCNSRDSSGNHVVFG GGTKLTVL | QVQLVQSGAEVKKPGASVKVSCKASGYTFRSYYM YWVRQAPGQGLEWVGTINPSGGSTNYAQKFQGRV TMTRDTSTSTLYMELNSLRSEDTAVYYCASGWELN YWGQGTLVTVSS |
| | | | SEQ ID NO: 27444 | SEQ ID NO: 31450 |
| iPS:436806 | 21-225_172B12 | NA | TCTTCTGAGCTGACTCAGGACCCTGCTGTGTCT GTGGCCTTGGGACAGACAGTCAGGATCACATG CCAAGGAGACAGCCTCAGAAACTATTATGCAA GCTGGTACCAGCAGAAGCCAGGACAGGCCCC TATACTTGTCATCTATACTAAAAACAGCCGGC CCTCAGGGATCCCAGACCGATTCTCTGGCTCC ACCTCAGGAAACACAGCTTCCTTGACCATCAC TGGGGCTCAGGCGGAAGATGAGGCTGACTATT ACTGTAACTCCCGGGACAGCAGTGGCAACCAT GTGGTATTCGGCGGAGGGACCAAGCTGACCGT CCTA | CAGGTGCAGCTGGTGCAGTCTGGGGGCTGAGGTG AAGAAGCCTGGGGCCTCAGTGACGGTTTCCTGCA AGGCATCTGGATACACCTTCAGAAGTTACTATAT GTATTGGGTGCGACAGGCCCCTGGACAAGGGCTT GAGTGGGTGGGAACAATCAACCCTAGTGGTGGT AGCACAGACTACGCACAGAAGTTCCAGGGCAGA GTCACCATGACCAGGGACACGTCCACGAGCACA CTCTACATGGAGCTGAACAGCCTGAGATCTGAGG ACACGGCCGTGTATTACTGTGCGAGTGGCTGGGA ATTAAACTACTGGGGCCAGGGAACCCTGGTCACC GTCTCCTCA |
| | | | SEQ ID NO: 27445 | SEQ ID NO: 31451 |

FIGURE 50

| | | | | |
|---|---|---|---|---|
| | | AA | SSELTQDPAVSVALGQTVRITCQGDSLRNYYAS WYQQKPGQAPILVIYTKNSRPSGIPDRFSGSTSG NTASLTITGAQAEDEADYYCNSRDSSGNHVVFG GGTKLTVL | QVQLVQSGAEVKKPGASVTVSCKASGYTFRSYYM YWVRQAPGQGLEWVGTINPSGGSTDYAQKFQGRV TMTRDTSTSTLYMELNSLRSEDTAVYYCASGWELN YWGQGTLVTVSS |
| | | | SEQ ID NO: 27446 | SEQ ID NO: 31452 |
| iPS:436808 | 21-225_173F8 | NA | TCCTATGAGCTGACTCAGCCACCCTCAGTGTC CGTGTCCCCAGGACAGACAGCCAGCATCACCT GCTCTGGAAATAAATTGGGGAATAAATATGTT TGCTGGTATCAGCAGAGGCCAGGCCAGTCCCC TGTGCTGGTCATCTCTCAAGATAGCAGGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCTTCACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAATGGGTGGCAGTTATATCATATGATGGAAGT CCTAAATACTGTGCAGACTCCGTGAAGGGCCGAT TCACCATCTCCAGAGACAATTCCAAGAACACGCT GTTTCTCCAAATGAACAGCCTGAGAGCTGAGGAC ACGGCTGTGTATTATTGTGCGAGAGATGAAAGGC AGTGGCTGCCGGCCCCCTACGGTATGGACGTCTG GGGCCAAGGGACCACGGTCACCGTCTCCTCA |
| | | | SEQ ID NO: 27447 | SEQ ID NO: 31453 |
| | | AA | SYELTQPPSVSVSPGQTASITCSGNKLGNKYVCW YQQRPGQSPVLVISQDSRRPSGIPERFSGSNSGNT ATLTISGTQAMDEADYYCQAWDSFTVVFGGGT KLTVL | QVQLVESGGGVVQPGRSLRLSCAASGFTFSSYGMH WVRQAPGKGLEWVAVISYDGSPKYCADSVKGRFT ISRDNSKNTLFLQMNSLRAEDTAVYYCARDERQW LPAPYGMDVWGQGTTVTVSS |
| | | | SEQ ID NO: 27448 | SEQ ID NO: 31454 |

FIGURE 50

| iPS:436810 | 21-225_175F4 | NA | CAGTCTGCCCTGACTCAGCCTGCCTCCGTGTCT GGGTCTCCTGGACAGTCGATCACCATCTCCTG CACTGGAACCAGCAGTGATGTTGGACGTTTTA ACCTTGTCTCCTGGTACCAACAGCACCCAGGC TACGCCCCCAAACTCATGATTTATGAGGTCAG TAAGCGGCCCTCAGGGGTTTCTAATCGCTTCT CTGGCTCCAAGTCTGGCAACACGGCCTCCCTG ACAATCTCTGGGCTCCAGGCTGAGGACGAGGC TGATTATTACTGCTGCTCATATGCAGGTAGTA GCACCTATGTGGTATTCGGCGGAGGGACCAAG CTGACCGTCCTG | CAGGTACAGCTGCAGCAGTCAGGTCCAGGACTG GTGAAGCCCTCGCAGACCCTCTCACTCACCTGTG CCATCTCCGGGGACAGTGTCTCTAGCAACAGTGC TGCTTGGAACTGGATCAGGCAGTCCCCATCGAGA GGCCTTGAGTGGCTGGGAAGGACATACTACAGG TCCAAGTGGTATAATGCTTATCCAGTATCTATGG AAAGTCGAATATCCATCAACCCAGACACATCCA AGAACCAGTTCTCCCTGCAGCTGAACTCTGTGAC TCCCGAGGACACGGCTGTTTATTACTGTGCAAGA GATAAGGCAGCTGGGAGGAATGACTTCTACTACT ACGGTATGGACGTCTGGGGCCAAGGGACCACGG TCACCGTCTCCTCA |
| | | | SEQ ID NO: 27449 | SEQ ID NO: 31455 |
| | | AA | QSALTQPASVSGSPGQSITISCTGTSSDVGRFNLV SWYQQHPGYAPKLMIYEVSKRPSGVSNRFSGSK SGNTASLTISGLQAEDEADYYCCSYAGSSTYVVF GGGTKLTVL | QVQLQQSGPGLVKPSQTLSLTCAISGDSVSSNSAA WNWIRQSPSRGLEWLGRTYYRSKWYNAYPVSMES RISINPDTSKNQFSLQLNSVTPEDTAVYYCARDKAA GRNDFYYYGMDVWGQGTTVTVSS |
| | | | SEQ ID NO: 27450 | SEQ ID NO: 31456 |
| iPS:436812 | 21-225_175C6 | NA | TCCTATGACCTGACTCAGCCACCCTCAGTGTC CGTGTCCCCAGGACAGACAGCCAGCATCACCT GCTCTGGAGATAAATTGGGGGATAAATATGCT TGTTGGTATCAGCAGAAGCCAGGCCAGTCCCC TATACTGGTCATCTATCAAGATTACAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ATTGTCAGGCGTGGGACAACAGCACTATGGTA TTCGGCGGAGGGACCAGGCTGACCGTCCTA | CAGGTGCAGCTGGTGGAGTCTGGGGGAGGCGTG GTCCAGCCTGGGAGGTCCCTGAGACTCTCCTGTG CAGCGTCTGGACTCACCTTCAGTAACTGTGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCGTCTCCAGAGACAATTCCAAGAACACGC TGGATCTGCAAATGAACAGCCTGAGAGCCGAGG ACACGGCTGTGTATTACTGTGCGAGAGATCAGTA CAACAGGAACGACGGACCACCAGCTTACTACTA CTACTACGGTTTGGACGTCTGGGGCCAAGGGACC ACGGTCACCGTCTCCTCA |

FIGURE 50

| | | | SEQ ID NO: 27451 | SEQ ID NO: 31457 |
|---|---|---|---|---|
| | | AA | SYDLTQPPSVSVSPGQTASITCSGDKLGDKYAC WYQQKPGQSPILVIYQDYKRPSGIPERFSGSNSG NTATLTISGTQAMDEADYYCQAWDNSTMVFGG GTRLTVL | QVQLVESGGGVVQPGRSLRLSCAASGLTFSNCGM HWVRQAPGKGLEWVAIIWYDGSNKYYADSVKGR FTVSRDNSKNTLDLQMNSLRAEDTAVYYCARDQY NRNDGPPAYYYYYGLDVWGQGTTVTVSS |
| | | | SEQ ID NO: 27452 | SEQ ID NO: 31458 |
| iPS:436814 | 21-225_178H10 | NA | CAGTCTGCCCTGACTCAGCCTGCCTCCGTGTCT GGGTCTCCTGGACAGTCGATCACCATCTCCTG CACTGGAACCAGCAGTGATGTTGGACGTTTTA ACCTTGTCTCCTGGTACCAACACCACCCAGGC AACGCCCCCAAACTCATGATTTATGAAGTCAG TAAGCGGCCCTCAGGGGTTTCTAATCGCTTCT CTGGCTCCAAGTCTGGCAACACGGCCTCCCTG ACAATCTCTGGGCTCCAGGCTGAGGACGAGGC TGATTATTACTGCTGCTCATATGCAGGTAGTA GCACCTTTGTAGTATTCGGCGGAGGGACCAAG CTGACCGTCCTA | CAGGTACAGCTGCAGCAGTCAGGTCCAGGACTG GTGAAGCCCTCGCAGACCCTCTCACTCACCTGTG CCATCTCCGGGGACAGTGTCTCTAGCAACAGTGC TGCTTGGAACTGGATCAGGCAGTCCCCATCGAGA GGCCTTGAGTGGCTGGGAAGGACATACTACAGG TCCAAGTGGTATAGTGCTTATCCAGTATCTATGG AAAGTCGAGTATCCATCAACCCAGACACATCCA AGAACCAGTTCTCCCTGCAGCTGAACTCTGTGAC TCCCGAGGACACGGCTGTTTATTACTGTGCAAGA GATAAGGCAGCTGGGAGGAATGACTTCTACTACT ACGGTATGGACGTCTGGGGCCAAGGGACCACGG TCACCGTCTCCTCA |
| | | | SEQ ID NO: 27453 | SEQ ID NO: 31459 |
| | | AA | QSALTQPASVSGSPGQSITISCTGTSSDVGRFNLV SWYQHHPGNAPKLMIYEVSKRPSGVSNRFSGSK SGNTASLTISGLQAEDEADYYCCSYAGSSTFVVF GGGTKLTVL | QVQLQQSGPGLVKPSQTLSLTCAISGDSVSSNSAA WNWIRQSPSRGLEWLGRTYYRSKWYSAYPVSMES RVSINPDTSKNQFSLQLNSVTPEDTAVYYCARDKA AGRNDFYYYGMDVWGQGTTVTVSS |
| | | | SEQ ID NO: 27454 | SEQ ID NO: 31460 |

FIGURE 50

| iPS:436816 | 21-225_179H5 | NA | TCTTCTGAGCTGACTCAGGACCCTGCTGTGTCT GTGGCCTTGGGACAGACAGTCAGGATCACATG CCAAGGAGACAGTCTCAGAAACTATTATGCAA GCTGGTACCAGCAGAAGCCAGGACAGGCCCC TGTATTTGTCATCTATGGTAAAAACAACCGGC CCTCAGGGATCCCAGACCGATTCTCTGGCTCC AGGTCAGGAAACACAGCTTCCTTGACCATCAC TGGGGCTCAGGCGGAAGATGAGGCTGACTATT ACTGTAACTCCCGGGACAGCAGTGGTAACCAT TGGGTGTTCGGCGGAGGGACCAAACTGACCGT CCTA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATATCCG GAACTACTACTACGGTTTGGACGTCTGGGGCCAA GGGACCACGGTCACCGTCTCCTCA |
| | | | SEQ ID NO: 27455 | SEQ ID NO: 31461 |
| | | AA | SSELTQDPAVSVALGQTVRITCQGDSLRNYYAS WYQQKPGQAPVFVIYGKNNRPSGIPDRFSGSRS GNTASLTITGAQAEDEADYYCNSRDSSGNHWVF GGGTKLTVL | QVQLVESGGGVVQPGRSLRLSCAASGFTFSSYGMH WVRQAPGKGLEWVAVIWYDGSNEYYADSVKGRF TISRDNSKNTLYLQMNSLRAEDTAVYYCARDIRNY YYGLDVWGQGTTVTVSS |
| | | | SEQ ID NO: 27456 | SEQ ID NO: 31462 |
| iPS:436818 | 21-225_179C7 | NA | TCCTATGAGCTGACTCAGCCACCCTCAGTGTC CGTGTCCCCAGGACAGACAGCCAGTATCACCT GCTCTGGAGATAAATTGGGGGATAAATATGTT TGCTGGTATCAACAGAGGCCGGGCCAGTCCCC TGTGCTGGTCATCTATCAGGATAGTAAGCGGC CCTCAGGGATCCCTGAGCGATTCTCTGGCTCC AACTCTGGGAACACAGCCACTCTGACCATCCG CGGGACCCAGGCTATGGATGAGGCTGACTATT ACTGTCAGGCGTGGGACAGCAACACTGCAGTG GTATTCGGCGGAGGGACCAAGCTGACCGTCCT A | CAGGCGCAGCTGGTGGAGTCTGGGGGAGGCGTG GTCCAGCCTGGGAGGTCCCTGAGACTCTCCTGTG CAGCGTCTGGATTCACCTTCAGTAACTCTGGCAT GCACTGGGTCCGCCAGGGTCCAGGCAAGGGGCT GGAGTGGGTGGCGATTATATATTATGATGGAAGT TATAAATACAATGCAGACTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGCGAGAGACCGTCA TTACGATTTCCACGTTCCCTACTATTACTATTACG GTATGGACGTCTGGGGCCAAGGGACCACGGTCA CCGTCTCCTCA |
| | | | SEQ ID NO: 27457 | SEQ ID NO: 31463 |

FIGURE 50

| | | | | |
|---|---|---|---|---|
| | | AA | SYELTQPPSVSVSPGQTASITCSGDKLGDKYVCW YQQRPGQSPVLVIYQDSKRPSGIPERFSGSNSGN TATLTIRGTQAMDEADYYCQAWDSNTAVVFGG GTKLTVL | QAQLVESGGGVVQPGRSLRLSCAASGFTFSNSGMH WVRQGPGKGLEWVAIIYYDGSYKYNADSVKGRFT ISRDNSKNTLYLQMNSLRAEDTAVYYCARDRHYD FHVPYYYYYGMDVWGQGTTVTVSS |
| | | | SEQ ID NO: 27458 | SEQ ID NO: 31464 |
| iPS:436820 | 21-225_179D10 | NA | CAGTCTGTGTTGACGCAGCCGCCCTCAGTGTC TGGGGCCCCAGGGCAGAGGGTCACCATCTCCT GCACTGGGAGCAGCTCCAACTTCGGGACAGAT TATGATGTACACTGGTACCAGCAATTTCCAGG AACAGCCCCCAAACTCCTCATCTATGGTCACA GCAACCGGCCCTCAGGGGTCCCTGACCGATTT TCTGGCTCCAAGTCTGGCACCTCAGCCTCCCT GGCCATCACTGGGCTCCAGGCTGAGGATGAGG CTGATTATTACTGCCAGTCCTATGATAGAAGC CTGAATGTGGTCTTCGGCGGAGGGACCAAGCT GACCGTCCTA | GAGGTGCAGCTGGTGGAGTCTGGGGGAGGCTTG GTACAGCCTGGGGGGTCCCTGAGATTCTCCTGTG CAGCCTCTGGATTCACCTTCAGTAGCTATAGCAT GAACTGGGTCCGCCAGGCTCCAGGGAAGGGGCT GGAGTGGGTTGCATACATTAGTAGTAGTGGAAGT ACCACATACTACGCAGACTCTGTGCAGGGCCGAT TCACCATCTCCAGAGACAATGCCAAGAACTCACT GTATCTGCAAATGAACAGCCTGAGAGACGAGGA CACGGCTGTGTATCGCTGTGCGAGAGATAGTAGG AAGGGGTTCTACTACGGTCTGGACGTCTGGGGCC AAGGGATCACGGTCACCGTCTCCTCA |
| | | | SEQ ID NO: 27459 | SEQ ID NO: 31465 |
| | | AA | QSVLTQPPSVSGAPGQRVTISCTGSSSNFGTDYD VHWYQQFPGTAPKLLIYGHSNRPSGVPDRFSGS KSGTSASLAITGLQAEDEADYYCQSYDRSLNVV FGGGTKLTVL | EVQLVESGGGLVQPGGSLRFSCAASGFTFSSYSMN WVRQAPGKGLEWVAYISSSGSTTYYADSVQGRFTI SRDNAKNSLYLQMNSLRDEDTAVYRCARDSRKGF YYGLDVWGQGITVTVSS |
| | | | SEQ ID NO: 27460 | SEQ ID NO: 31466 |

FIGURE 50

| iPS:436822 | 21-225_180D4 | NA | TCCTATGAGCTGACTCAGACACCCTCAGTGTC CGTGTCCCCAGGACAGACAGCCAGCATCACCT GCTCTGGAGATAGAATTGGGGGATAAAATATGCT TGCTGGTATCAGCAGAAGCCAGGCCAGTCCCC TGTGCTGGTCATCTATGAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGGTGACTATT ACTGTCAGGCGTGGGACAGTAGGAAAGTGGT ATTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTTTGGCAT GCACTGGGTCCGCCAGGCTCCAGGCAAGGGGCT GGAGTGGGTGGCAATTATATGGTATGATGGAAGT GATAAATACTATGCAGACTCCGTGAAGGGCCGA TTCACCATCTCCAGAGACAATTCCAAGAACACGC TGTATCTGCAAATGAACACCCTGAGAGCCGAAG ACACGGCTGTGTATTTCTGTGCGAGAGGGGGGCC CCCGTTCTCTACGGTGACTATGTACTTTGACTACT GGGGCCAGGGAACCCTGGTCACCGTCTCCTCA |
|---|---|---|---|---|
| | | | SEQ ID NO: 27461 | SEQ ID NO: 31467 |
| | | AA | SYELTQTPSVSVSPGQTASITCSGDRLGDKYACW YQQKPGQSPVLVIYEDRKRPSGIPERFSGSNSGN TATLTISGTQAMDEGDYYCQAWDSRKVVFGGG TKLTVL | QVQLVESGGGVVQPGRSLRLSCAASGFTFSNFGMH WVRQAPGKGLEWVAIIWYDGSDKYYADSVKGRFT ISRDNSKNTLYLQMNTLRAEDTAVYFCARGGPPFS TVTMYFDYWGQGTLVTVSS |
| | | | SEQ ID NO: 27462 | SEQ ID NO: 31468 |
| IPS:436824 | 21-225_180C5 | NA | TCCTATGAACTGACTCAGCCACCCTCAGTGTC CGTGTCCCCAGGACAGACAGCCAGCATCACCT GCTCTGGAGATAAAATTGGGGGAAAAATATGCT TGCTGGTATCAGCAGAAGCCAGGCCAGTCCCC TGTACTGGTCATCTATCAAGATAGAAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGGATTCATTTCTTGGAATG ATGATAAGCGCTACAGCCCATCTCTGAAGAGCA GCCTCACCATCACCAAGGACACCTCCAAAAACC AGGTGGTTCTTATAATGACCAACATGGACCCTGT GGACACAGCCACATATTACTGTGCACACAAAGC AGCAGCTGTTGCTTTTGATATCTGGGGCCAAGGG ACAATGGTCACCGTCTCTTCA |
| | | | SEQ ID NO: 27463 | SEQ ID NO: 31469 |

FIGURE 50

| IPS:436826 | 21-225_180G5 | AA | SYELTQPPSVSVSPGQTASITCSGDKLGEKYACW YQQKPGQSPVLVIYQDRKRPSGIPERFSGSNSGN TATLTISGTQAMDEADYYCQAWDSSTAVFGGG TKLTVL | QITLKESGPTLVKPTQTLTLTCTFSGFSLSTSGVGVG WIRQPPGKALEWLGFISWNDDKRYSPSLKSSLTITK DTSKNQVVLIMTNMDPVDTATYYCAHKAAAVAFD IWGQGTMVTVSS |
|---|---|---|---|---|
| | | | SEQ ID NO: 27464 | SEQ ID NO: 31470 |
| | | NA | TCCTATGAGCTGACTCAGCCACCCTCAGTGTC CGTGTACCCAGGACAGACAGCCAGCATCACCT GCTCTGGAGATAAATTGGGGGATAAATATGTT AGCTGGTATCAACAGAAGCCAGGCCAGTCCCC TGTTCTGGTCATCTATCAAGATAGCAAGCGGC CCTCAGGGATCCCTGAGCGATTCTCTGGCTCC AACTCTGGGAACCCCAGCCTCTCTGACCATCAG CGGGGACCCAGGCTATGGATGAGGCTGACTATT ACTGTCAGGCGTGGGACATCACCACTGCGGTA TTCGGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGAGGGTT TTATTACTATGGTTCGGGGAGTCATGTCCCTTACC ACTACTACTACGGTTTGGACGTCTGGGGCCAAGG GACCACGGTCACCGTCTCCTCA |
| | | | SEQ ID NO: 27465 | SEQ ID NO: 31471 |
| | | AA | SYELTQPPSVSVYPGQTASITCSGDKLGDKYVS WYQQKPGQSPVLVIYQDSKRPSGIPERFSGSNSG NPASLTISGTQAMDEADYYCQAWDITTAVFGGG TKLTVL | QVQLVQSGAEVKKPGASVKVSCKASGYTFTSYDIN WVRQATGQGLEWMGWMNPNSGNTGYAQKFQGR VTMTRNTSISTAYMELSSLRSEDTAVYYCARGFYY YGSGSHVPYHYYYGLDVWGQGTTVTVSS |
| | | | SEQ ID NO: 27466 | SEQ ID NO: 31472 |

FIGURE 50

| iPS:436828 | 21-225_181H1 | NA | TCCTATGAGCTGACTCAGACACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATGAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGGAAAGTGGT ATTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTATGATGGAAGT GATAAATACTATGCAGACTCCGTGAAGGGCCGA ATCACCATCTCCAGAGACAATTCCAAGAACACGC TGTATCTGCAAATGAACACCCTGAGAGCCGAAG ACACGGCTGTGTATTTCTGTGCGAGAGGGGGGCC CCCGTTTTCTACGGTGACTATGTACTTCGACTACT GGGGCCAGGGAACCCTGGTCACCGTCTCCTCA |
| | | | SEQ ID NO: 27467 | SEQ ID NO: 31473 |
| | | AA | SYELTQTPSVSVSPGQTASITCSGDKLGDKYAC WYQQKPGQSPVLVIYEDRKRPSGIPERFSGSNSG NTATLTISGTQAMDEADYYCQAWDSRKVVFGG GTKLTVL | QVQLVESGGGVVQPGRSLRLSCAASGFTFSNYGM HWVRQAPGKGLEWVAIIWYDGSDKYYADSVKGRI TISRDNSKNTLYLQMNTLRAEDTAVYFCARGGPPF STVTMYFDYWGQGTLVTVSS |
| | | | SEQ ID NO: 27468 | SEQ ID NO: 31474 |
| iPS:436830 | 21-225_51F4 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTAAT ATTGTGACCTGGTACCAGCAGCTCCCAGGAAC GGCCCCCAAACTCCTCATCTATAGTAATGATC AGCGGCCCTCAGGGGTCCCTGACCGATTCTCT GGCTCCAAGTCTGGCACCTCAGCCTCCCTGGC CATCAGTGGGCTCCAGTCTGAGGATGAGACTG ATTATTACTGTACAGCATGGGATGACAGCCTG AATGGTTGGGTGTTCGGCGGAGGGACCACGCT GACCGTCCTA | CAGGTTCAGCTGGTGCAGTCTGGAGCTGAGGTGA AGAAGCCTGGGGCCTCAGTGAAGGTCTCCTGCA AGGCTTCTGGTTACACCTTTAACAGCTATGGTAT CAGCTGGGTGCGACAGGCCCCTGGACAAGGGCT TGAGTGGATGGGATGGATCAGCGCTTATAATGGT AACACAAAGTATGCACAGAAGCTCCAGGGCAGA GTCACCATGACCACAGACACATCCACGAGCACA GCCTACATGGAGCTGAGGAGCCTGAGATCTGAC GACACGGCCGTGTATTACTGTGCGAGACACGATT TTTGGAGTGGTTATTATAAGGGTATGGACGTCTG GGGCCAAGGGACCACGGTCACCGTCTCCTCA |
| | | | SEQ ID NO: 27469 | SEQ ID NO: 31475 |

FIGURE 50

| | | | | |
|---|---|---|---|---|
| | | AA | QSVLTQPPSASGTPGQRVTISCSGSSSNIGSNIVT WYQQLPGTAPKLLIYSNDQRPSGVPDRFSGSKS GTSASLAISGLQSEDETDYYCTAWDDSLNGWVF GGGTTLTVL | QVQLVQSGAEVKKPGASVKVSCKASGYTFNSYGIS WVRQAPGQGLEWMGWISAYNGNTKYAQKLQGR VTMTTDTSTSTAYMELRSLRSDDTAVYYCARHDF WSGYYKGMDVWGQGTTVTVSS |
| | | | SEQ ID NO: 27470 | SEQ ID NO: 31476 |
| iPS:436832 | 21-225_51D8 | NA | CAGTCTGTGCTGACGCAGCCGCCCTCAGTGTC TGGGGCCCCAGGGCAGAGGGTCACCATCTCCT GCACTGGGAGCAGCTCCAACATCGGGGGCAGG TTTTGAAGTACACTGGTACCAGCAGCTTCCAG GAACAGCCCCCAAACTCCTCATCTATGGTAAC AGCAATCGGCCCTCAGGGGTCCCTGACCGATT CTCTGGCTCCAAGTCTGGCACCTCAGCCTCCCT GGCCATCACTGGGCTCCAGGCTGAGGATGAGG CTGATTATTACTGCCAGTCCTATGACAGCAGC CTGAGTGGTTATGTCTTCGGAACTGGGACCAA GGTCACCGTCCTA | CAGGTACAGCTGCAGCAGTCAGGTCCAGGACTG GTGAAGCCCTCGCAGACCCTCTCACTCACCTGTG CCATCTCCGGGGACAGTGTCTCTAGTAACAGTGC TGCTTGGAACTGGATCAGGCAGTCCCCATCGAGA GGCCTTGAGTGGCTGGGAAGGACATACTACAGG TCCAAGTGGTATAATGATTATGCAGTATCTGTGA AAAGTCGAATAACCTTCAACCCGGACACATCCA AGAACCAGTTCTCCCTGCGGCTGAACTCTGTGAC TCCCGAGGACACGGCTGTGTATTACTGTGCAAGA GACCGCTATAACTGGAACTACCCCTACTGGTACT TCGATCTCTGGGGCCGTGGCACCCTGGTCACTGT CTCCTCA |
| | | | SEQ ID NO: 27471 | SEQ ID NO: 31477 |
| | | AA | QSVLTQPPSVSGAPGQRVTISCTGSSSNIGAGFEV HWYQQLPGTAPKLLIYGNSNRPSGVPDRFSGSK SGTSASLAITGLQAEDEADYYCQSYDSSLSGYVF GTGTKVTVL | QVQLQQSGPGLVKPSQTLSLTCAISGDSVSSNSAA WNWIRQSPSRGLEWLGRTYYRSKWYNDYAVSVK SRITFNPDTSKNQFSLRLNSVTPEDTAVYYCARDRY NWNYPYWYFDLWGRGTLVTVSS |
| | | | SEQ ID NO: 27472 | SEQ ID NO: 31478 |

FIGURE 50

| iPS:436834 | 21-225_52F1 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTAAT ATTGTGACCTGGTACCAGCAGCTCCCAGGAAC GGCCCCCAAACTCCTCATCTATAGTAATGATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AATGGTTGGGTGTTCGGCGGAGGGACCACGCT GACCGTCCTA | CAGGTTCAGCTGGTGCAGTCTGGAGCTGAGGTGA AGAAGCCTGGGGCCTCAGTGAAGGTCTCCTGCA AGGCTTCTGGTTACACCTTTAACAGCTATGGTGT CAGCTGGGTGCGACAGGCCCCTGGACAAGGGCT TGAGTGGATGGGATGGATCAGCGCTTATAATGGT AACAGAAAGTATGCACAGAAGCTCCAGGGCAGA GTCTCCATGACCACAGACACATCCACGAGCACA GCCTACATGGAGCTGAGGAGCCTGAGATCTGAC GACACGGCCGTGTATTACTGTGCGAGACACGATT TTTGGAGTGGTTATTATAAGGGTATGGACGTCTG GGGCCAAGGGACCACGGTCACCGTCTCCTCA |
| | | | SEQ ID NO: 27473 | SEQ ID NO: 31479 |
| | | AA | QSVLTQPPSASGTPGQRVTISCSGSSSNIGSNIVT WYQQLPGTAPKLLIYSNDQRPSGVPDRFSGSKS GTSASLAISGLQSEDEADYYCAAWDDSLNGWV FGGGTTLTVL | QVQLVQSGAEVKKPGASVKVSCKASGYTFNSYGV SWVRQAPGQGLEWMGWISAYNGNRKYAQKLQGR VSMTTDTSTSTAYMELRSLRSDDTAVYYCARHDF WSGYYKGMDVWGQGTTVTVSS |
| | | | SEQ ID NO: 27474 | SEQ ID NO: 31480 |
| iPS:436836 | 21-225_52H1 | NA | TCCTATGAGCTGACTCAGCCACCCTCAGTGTT CGTGTCCCCAGGACAGACAGCCAGCATCACCT CCTCTGGAGATAAATTGGGGGATAAATATGTT TCCTGGTATCAGCAGAAGCCAGGCCAGTCCCC TGTGCTGGTCATCTATCAAGATAGAAAGCGGC CCTCAGGGATCCCTGAGCGATTCTCTGGCTCC AATTCTGGGAATACAGCCACTCTGACCATCAG CGGGACCCAGGCTATGGATGAGGCTGACTATT ATTGTCAGGCGTGGGACAACAGCACTGTGGTA TTCGGCGGAGGGACCAAGCTGACCGTCCTA | CAGGTGCAGCTGGTGGAGTCTGGGGGAGGCGTG GTCCAGCCCGGGAGGTCCCTGAGACTCTCCTGTG CAGCGTCTGGATTCACCTTCAGTGGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTTTATTACTGTGCGAGAGATCGGGT CTATTGTAGTAGTTCCAGCTGCTCATATTACTACT ACTACTACGGTATGGACGTCTGGGGGCCAAGGGA CCACGGTCACCGTCTCCTCA |

FIGURE 50

| | | | SEQ ID NO: 27475 | SEQ ID NO: 31481 |
|---|---|---|---|---|
| | | AA | SYELTQPPSVFVSPGQTASITSSGDKLGDKYVSW YQQKPGQSPVLVIYQDRKRPSGIPERFSGSNSGN TATLTISGTQAMDEADYYCQAWDNSTVVFGGG TKLTVL | QVQLVESGGGVVQPGRSLRLSCAASGFTFSGYGM HWVRQAPGKGLEWVAVIWYDGSNKYYADSVKGR FTISRDNSKNTLYLQMNSLRAEDTAVYYCARDRVY CSSSSCSYYYYYGMDVWGQGTTVTVSS |
| | | | SEQ ID NO: 27476 | SEQ ID NO: 31482 |
| iPS:436838 | 21-225_52H4 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAGCACCCAGGC AAAGCCCCCAAACTCATGATTTATGAGGTCAG TAATCGGCCCTCAGGGGTTTCTAATCGCTTCTC TGGCTCCAAGTCTGGCAATACGGCCTCCCTGA CCATCTCTGGGCTCCAGGCTGAGGACGAGGCT GATTATTACTGCAACTCATATACAAGCAACAT CACTTGGGTGTTCGGCGGAGGGACCAAGCTGA CCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTTTGGCAT GCACTGGGTCCGCCAGGCTCCAGGCAAGGGGCT GAAATGGGTGGCAGTTATTTGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGGGGACTG GAACTACGAGGGTTTTGACTACTGGGGCCAGGG AACCCTGGTCACCGTCTCCTCA |
| | | | SEQ ID NO: 27477 | SEQ ID NO: 31483 |
| | | AA | QSALTQPASVSGSPGQSITISCTGTSSDVGGYNY VSWYQQHPGKAPKLMIYEVSNRPSGVSNRFSGS KSGNTASLTISGLQAEDEADYYCNSYTSNITWVF GGGTKLTVL | QVQLVESGGGVVQPGRSLRLSCAASGFTFSHFGMH WVRQAPGKGLKWVAVIWYDGSNKYYADSVKGRF TISRDNSKNTLYLQMNSLRAEDTAVYYCARGDWN YEGFDYWGQGTLVTVSS |
| | | | SEQ ID NO: 27478 | SEQ ID NO: 31484 |

FIGURE 50

| iPS:436840 | 21-225_53E9 | NA | TCCTATGAGCTGACTCAGCCACCCTCAGTGTC CGTGTCCCCAGGACAGACAGCCAGCATCACCT GCTCTGGAACTAAATTGGGGGATAAATATGTT TGCTGGTATCAACAGAAGCCAGGCCAGTCCCC TGTGTTGGTCATCAATCAAGATACAATGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGACGTGGGACAGCAGCACTGCGGTT TTCGGCGGAGGGACCACGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGAAACACCTTCACCGGCTACTATA TGCACTGGGTGCGACAGGCCCCTGGACAAGGGC TTGAGTGGATGGGATGGATCATCCCTAACAGTGG TGACACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTCTATTACTGTGCGAGAGATGGG TATAGCAGTGGCTGGTTCAACTGGTTCGACCCCT GGGGCCAGGGAACCCTGGTCACCGTCTCCTCA |
|---|---|---|---|---|
| | | | SEQ ID NO: 27479 | SEQ ID NO: 31485 |
| | | AA | SYELTQPPSVSVSPGQTASITCSGTKLGDKYVCW YQQKPGQSPVLVINQDTMRPSGIPERFSGSNSGN TATLTISGTQAMDEADYYCQTWDSSTAVFGGGT TLTVL | QVQLVQSGAEVKKPGASVKVSCKASGNTFTGYYM HWVRQAPGQGLEWMGWIIPNSGDTNYAQKFQGR VTMTRDTSISTAYMELSRLRSDDTAVYYCARDGYS SGWFNWFDPWGQGTLVTVSS |
| | | | SEQ ID NO: 27480 | SEQ ID NO: 31486 |
| iPS:436842 | 21-225_54E9 | NA | CAGTCTGTGCTGACTCAGCCACCCTCAGCGTC TGGGACCCCCGGGCAGAGGGTCACCATCTCTT GTTCTGGAAGCAACTCCAACATCGGAAATAAT ATTGTCACCTGGTACCAGCAGCTCCCAGGAAC GGCCCCCAAACTCCTCATCTATGTTAATGATC AGCGGCCCTCAGGGGTCCCTGACCGATTCTCT GGCTCCAAGTCTGGCACCTCTGCCTCCCTGGC CATCAGTGGGCTCCAGTCTGAGGATGAGGCTG ATTATTACTGTGCAGCATGGGATGACAGCCTG AATGGTTGGGTGTTCGGCGGGGGGACCACGCT GACCGTCCTA | CAGGTTCAGCTGGTGCAGTCTGGAGCTGAGGTGA AGAAGCCTGGGGCCTCAGTGAAGGTCTCCTGCA AGGCTTCTGGTTACACCTTTAACAGCTATGGTAT CAGCTGGGTGCGACTGGCCCCTGGACAAGGGCTT GAGTGGATGGGATGGATTAGTGCTTATAATGGTA ACACAAAGAATGCACAGAAGCTCCAGGGCAGAG TCACCATGACCACAGACACATCCACGAGCACAG CCTACATGGAGCTGAGGAGCCTGAGATCTGACG ACACGGCCGTTTATTACTGTGCGAGACACGATTT TTGGAGTGGTTATTATAAGGGTATGGACGTCTGG GGCCAAGGGACCACGGTCACCGTCTCCTCA |
| | | | SEQ ID NO: 27481 | SEQ ID NO: 31487 |

EP 4 435 105 A2

FIGURE 50

| | | | QSVLTQPPSASGTPGQRVTISCSGSNSNIGNNIVT WYQQLPGTAPKLLIYVNDQRPSGVPDRFSGSKS GTSASLAISGLQSEDEADYYCAAWDDSLNGWV FGGGTTLTVL | QVQLVQSGAEVKKPGASVKVSCKASGYTFNSYGIS WVRLAPGQGLEWMGWISAYNGNTKNAQKLQGRV TMTTDTSTSTAYMELRSLRSDDTAVYYCARHDFW SGYYKGMDVWGQGTTVTVSS |
|---|---|---|---|---|
| | | AA | SEQ ID NO: 27482 | SEQ ID NO: 31488 |
| iPS:436844 | 21-225_56G1 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTCAT ATTGTTACCTGGTACCAGCAGCTCCCAGGAAC GGCCCCCAAACTCCTCATCTACAGTAATGATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGTATGGGATGACAGCCTG ATTGGTTGGGTGTTCGGCGGAGGGACCACGCT GACCGTCCTA | CAGGTTCAGCTGGTGCAGTCTGGAGCTGAGGTGA AGAAGCCTGGGGCCTCAGTGAAGGTCTCCTGCA AGGCTTCTGGCTACACCTTTAACAGCTATGGTAT CAGCTGGGTGCGACAGGCCCCTGGACAAGGGCT TGAGTGGATGGGATGGATCAGCGCTTATAATGGT AACACAAAGTATGCACAGAAGTTCCAGGGCAGA GTCACCATGACCACAGACACATCCACGAGCACA GCCTACATGGAGCTGAGGAGCCTGAGATCTGAC GACACGGCCGTGTATTACTGTGCGAGACACGATT TTTGGAGTGGTTATTATAAGGGTATGGACGTCTG GGGCCAAGGGACCACGGTCACCGTCTCCTCA |
| | | | SEQ ID NO: 27483 | SEQ ID NO: 31489 |
| | | AA | QSVLTQPPSASGTPGQRVTISCSGSSSNIGSHIVT WYQQLPGTAPKLLIYSNDQRPSGVPDRFSGSKS GTSASLAISGLQSEDEADYYCAVWDDSLIGWVF GGGTTLTVL | QVQLVQSGAEVKKPGASVKVSCKASGYTFNSYGIS WVRQAPGQGLEWMGWISAYNGNTKYAQKFQGRV TMTTDTSTSTAYMELRSLRSDDTAVYYCARHDFW SGYYKGMDVWGQGTTVTVSS |
| | | | SEQ ID NO: 27484 | SEQ ID NO: 31490 |

2527

FIGURE 50

| iPS:436846 | 21-225_56E3 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTAAT ATTGTTACCTGGTACCAGCAGCTCCCAGGAAC GGCCCCCAAACTCCTCATCTATAGTAATAATC AGCGGCCCTCAGGGGTCCCTGACCGATTCTCT GGCTCCAAGTCTGGCACCTCAGCCTCCCTGGC CATCAGTGGACTCCAGTCTGAGGATGAGGCTG ATTATTGCTGTGCAGCATGGGATGACAGCCTG AATGGTTGGGTGTTCGGCGGAGGGACCACGCT GACCGTCCTA | CAGGTTCAACTGGTGCAGTCTGGAGCTGAGGTGA AGAAGCCTGGGGCCTCAGTGAAGGTCTCCTGCA AGGCTTCTGGTTACACCTTTAACAGCTATGGTTTC AGCTGGGTGCGACAGGCCCCTGGACAAGGGCTT GAGTGGATGGGATGGATCAGCGCTTATAATGGTA ACACAAAGGAAGCACAGAAGTTCCAGGGCAGAG TCACCATGACCACAGACACATCCACGAGCACAG CCTACATGGAGCTGAGGAGCCTGAGAGCTGACG ACACGGCCGTGTATTACTGTGCGAGACACGATTT TTGGAGTGGTTATTATAAGGGTATGGACGTCTGG GGCCAAGGGACCACGGTCACCGTCTCCTCA |
| | | | SEQ ID NO: 27485 | SEQ ID NO: 31491 |
| | | AA | QSVLTQPPSASGTPGQRVTISCSGSSSNIGSNIVT WYQQLPGTAPKLLIYSNNQRPSGVPDRFSGSKS GTSASLAISGLQSEDEADYCCAAWDDSLNGWVF GGGTTLTVL | QVQLVQSGAEVKKPGASVKVSCKASGYTFNSYGF SWVRQAPGQGLEWMGWISAYNGNTKEAQKFQGR VTMTTDTSTSTAYMELRSLRADDTAVYYCARHDF WSGYYKGMDVWGQGTTVTVSS |
| | | | SEQ ID NO: 27486 | SEQ ID NO: 31492 |
| iPS:436848 | 21-225_57F1 | NA | TCCTATGAGCTGACTCAGCCACCCTCAGCGTC CGTGTCCCCAGGACAGACAGCCAGCATCACCT GCTCTGGAGATAAACTGGGGGAAAAATATGCT TGCTGGTATCAGCAGAAGCCAGGCCAGTCCCC TGTGTTGGTCATCTATCAAGATAGGAAGCGGC CCTCAGGGATCCCTGAGCGATTCTCTGGCTCC AATTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAACTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGTACTAGTGGAGTG GGTGTGGGCTGGATCCGTCAGCCCCCAGGAAAG GCCCTGGAGTGGCTTGCACTCATTTATTGGCATG AAGATAAGCGCTACAGCCCATCTCTGAAGAGCA GGCTCACCATCACTGAGGACACCTCCAAAAACC AGGTGGACCTTACAATGACCAACATGGCCCCTGT GGACACAGCCACATATTACTGTGCACACGTCACA GGTATAGCAGCTCCCTACTGGGGCCAGGGAACC CTGGTCACCGTCTCCTCA |
| | | | SEQ ID NO: 27487 | SEQ ID NO: 31493 |

FIGURE 50

| | | | | |
|---|---|---|---|---|
| | | AA | SYELTQPPSASVSPGQTASITCSGDKLGEKYACW YQQKPGQSPVLVIYQDRKRPSGIPERFSGSNSGN TATLTISGTQAMDEADYYCQAWDSSTVVFGGG TKLTVL | QITLKESGPTLVKPTQTLTLTCTFSGFSLSTSGVGVG WIRQPPGKALEWLALIYWHEDKRYSPSLKSRLTITE DTSKNQVDLTMTNMAPVDTATYYCAHVTGIAAPY WGQGTLVTVSS |
| | | | SEQ ID NO: 27488 | SEQ ID NO: 31494 |
| iPS:436850 | 21-225_57D9 | NA | TCCTATGAGCTGACTCAGCCACCCTCAGTGTC CGTGTCCCCAGGACAGACAGCCAGCATCACCT GCTCTGGAGAGAAATTGGGGGAAAAATTTGCT TGCTGGTCTCAGCAGAAGCCAGGCCAGTCCCC TGTGTTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAGCTGACCGTCCTA | CAGATCACCTTGAAGGAGTCTGGTCCTATGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTTATTGGAATG ATGATAAGCGCTACAGTCCATCTCTGAAGAGCAG GCTCACCATCACCGAGGACACCTCCAAAAACCA GGTGGTCCTTACAATGACCAACATGGACCCTGTG GACACAGCCACATATTACTGTGCACATGCAGTGG CTGTCTCCTTTGACTACTGGGGCCAGGGAACCCT GGTCACCGTCTCCTCA |
| | | | SEQ ID NO: 27489 | SEQ ID NO: 31495 |
| | | AA | SYELTQPPSVSVSPGQTASITCSGEKLGEKFACW SQQKPGQSPVLVIYQDSKRPSGIPERFSGSNSGNT ATLTISGTQAMDEADYYCQAWDSSTVVFGGGT KLTVL | QITLKESGPMLVKPTQTLTLTCTFSGFSLSTSGVGV GWIRQPPGKALEWLALIYWNDDKRYSPSLKSRLTI TEDTSKNQVVLTMTNMDPVDTATYYCAHAVAVSF DYWGQGTLVTVSS |
| | | | SEQ ID NO: 27490 | SEQ ID NO: 31496 |

FIGURE 50

| iPS:436852 | 21-225_57H11 | NA | TCCTATGCGCTGACTCAGCCACCCTCAGCGTC CGTGTCCCCAGGACAGACAGCCAGCATCACCT GCTCTGGAGATAAACTGGGGGAAAAATATGCT TGCTGGTATCAGCAGAAGCCAGGCCAGTCCCC TGTGTTGGTCATCTATCAAGATAGGAAGCGGC CCTCAGGGATCCCTGAGCGATTCTCTGGCTCC AATTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAACTGACCGTCCTA | CAGATCACCTTGAAGGAGTCTGGTCCTACGCTGG TGAAACCCACACAGACCCTCACGCTGACCTGCAC CTTCTCTGGGTTCTCACTCACTACTAGTGGAGTG GGTGTGGGCTGGATCCGTCAGCCCCCAGGAAAG GCCCTGGAGTGGCTTGCACTCATTTATTGGCATG AAGATAGGCGCTACAGCCCATCTCTGAAGAGCA GGCTCACCATCACTGAGGACACCTCCAAAAACC AGGTGGACCTTACAATGACCAACATGGCCCCTGT GGACACAGCCACATATTACTGTGCACACGTCACA GGTATAGCAGCTCCCTACTGGGGCCAGGGAACC CTGGTCACCGTCTCCTCA |
| | | | SEQ ID NO: 27491 | SEQ ID NO: 31497 |
| | | AA | SYALTQPPSASVSPGQTASITCSGDKLGEKYACW YQQKPGQSPVLVIYQDRKRPSGIPERFSGSNSGN TATLTISGTQAMDEADYYCQAWDSSTVVFGGG TKLTVL | QITLKESGPTLVKPTQTLTLTCTFSGFSLTTSGVGVG WIRQPPGKALEWLALIYWHEDRRYSPSLKSRLTITE DTSKNQVDLTMTNMAPVDTATYYCAHVTGIAAPY WGQGTLVTVSS |
| | | | SEQ ID NO: 27492 | SEQ ID NO: 31498 |
| IPS:436854 | 21-225_58C1 | NA | TCCTATGAGCTGACTCAGCCACCCTCAGTGTC CGTGTCCCCAGGACAGACAGCCAGCCAACATCACCT GCTCTGGAGATAAAATTGGGGAATAAATATGCT TGCTGGTATCAGCAGAAGCCAGGCCAGTCCCC TGTATTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGCATTC GGCGGAGGGACCAAGCTGACCGTCCTC | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTTATTGGGATG ATGATAAGCGCTACAGCCCATCTCTGAAGAGCA GGCTCACCATCACCGAGGACACCTCCAAAAACC AGGTGGTCCTTACAATGACCAACATGGACCCTGT GGACACAGCCACATATTACTGTGCACACCTTATA GCAGTGGCCTTTGACTCCTGGGGCCAGGGAACCC TGGTCACCGTCTCCTCA |
| | | | SEQ ID NO: 27493 | SEQ ID NO: 31499 |

FIGURE 50

| | | | | |
|---|---|---|---|---|
| | | AA | SYELTQPPSVSVSPGQTANITCSGDKLGNKYAC WYQQKPGQSPVLVIYQDRKRPSGIPERFSGSNSG NTATLTISGTQAMDEADYYCQAWDSSTAFGGG TKLTVL | QITLKESGPTLVKPTQTLTLTCTFSGFSLSTSGVGVG WIRQPPGKALEWLALIYWDDDKRYSPSLKSRLTITE DTSKNQVVLTMTNMDPVDTATYYCAHLIAVAFDS WGQGTLVTVSS |
| | | | SEQ ID NO: 27494 | SEQ ID NO: 31500 |
| IPS:436856 | 21-225_58C5 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAATAAT TATGTATCCTGGTACCAGCAGCTCCCAGGAAC AGCCCCCAAACTCCTCATTTATGACAATAATA AGCGACCCTCAGGGATTCCTGACCGATTTTCT GGCTCCAAGTCTGGCACGTCAGCCACCCTGGG CATCACCGGACTCCAGACTGGGGACGAGGCC GATTATTACTGCGGAACATGGGATATCAGTCT GAGTGTTGGGGGTATTCGGCGGAGGGACCAAG CTGACCGTCCTA | GAGGTGCAGTTGGTGGAGTCTGGGGGAGGCCTG GTCAAGCCTGGGGGGTCCCTGAGACTCTCCTGTG CAGCCTCTGGATTCACCTTCAATAGCTATAGCAT GAACTGGGTCCGCCAGGCTCCAGGGAAGGGGCT GGAGTGGATCTCATCCATTAGTAGTAGTAGTTAT TACTTATACTACGCAGACTCAGTGAAGGGCCGAT TCACCATCTCCAGAGACAGCGCCAAGAATTCACT GTATCTGCAAATGAACAGCCTGAGAGCCGAGGA CACGGCTGTATATTACTGTGCGAGGACCTATAGT GGGAGTTTTGACTACTGGGGCCAGGGAACCCTG GTCACCGTCTCCTCA |
| | | | SEQ ID NO: 27495 | SEQ ID NO: 31501 |
| | | AA | QSVLTQPPSVSAAPGQKVTISCSGSSSNIGNNYVS WYQQLPGTAPKLLIYDNNKRPSGIPDRFSGSKSG TSATLGITGLQTGDEADYYCGTWDISLSVGVFG GGTKLTVL | EVQLVESGGGLVKPGGSLRLSCAASGFTFNSYSMN WVRQAPGKGLEWISSISSSSYYLYYADSVKGRFTIS RDSAKNSLYLQMNSLRAEDTAVYYCARTYSGSFD YWGQGTLVTVSS |
| | | | SEQ ID NO: 27496 | SEQ ID NO: 31502 |

FIGURE 50

| iPS:436858 | 21-225_58E7 | NA | TCCTATGAACTGACTCAGTCACCCTCGGTGTC CGTGTCCCCAGGACAGACAGCCAGCATCACCT GTTCTGGAGATAAATTGGGGGATAAATATACT TGCTGGTATCAGAAGAAGCCAGGCCAGTCCCC TGTGCTGGTCATCTATCAAGATAACAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT TCTGTCAGGCGTGGAACAACTACACTGTGGTA TTCGGCGGAGGGACCAAGCTGACCGCTCTA | CAGGTGCAGTTGGTGGAGTCTGGGGGAGGCGTG GTCCAGCCTGGGAGGTCCCTGAGACTCTCCTGTG CAGCCTCTGGATTCACCTTCAGTTTCTATGGCAT GCACTGGGTCCGCCAGGCTCCAGGCAAGGGGCT GGAGTGGGTGGCAGTTACATCATATGATGGAAGT GATAAATACTATGCAGACTCCGTGAAGGGCCGA TTCTCCATCTCCAGAGACAATTCCAAGAACACGC TGTATCTGCAAATGAGCAGCCTGAGAGCTGAGG ACACGGCTATGTATTACTGTGCGAGAGATGACTA TGGTTCGGGGAGTCCCCTATACTACGGTATGGAC GTCTGGGGCCAAGGGACCACGGTCACCGTCTCCT CA |
|---|---|---|---|---|
| | | | SEQ ID NO: 27497 | SEQ ID NO: 31503 |
| | | AA | SYELTQSPSVSVSPGQTASITCSGDKLGDKYTCW YQKKPGQSPVLVIYQDNKRPSGIPERFSGSNSGN TATLTISGTQAMDEADYFCQAWNNYTVVFGGG TKLTAL | QVQLVESGGGVVQPGRSLRLSCAASGFTFSFYGMH WVRQAPGKGLEWVAVTSYDGSDKYYADSVKGRF SISRDNSKNTLYLQMSSLRAEDTAMYYCARDDYGS GSPLYYGMDVWGQGTTVTVSS |
| | | | SEQ ID NO: 27498 | SEQ ID NO: 31504 |
| iPS:436860 | 21-225_58F7 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAGCTGACCGTCCTA | GAGGTGCAGCTGGTGGAGTCTGGGGGAGGCTTG GTCCAGCCTGGGGGGTCCCTGAGACTCTCCTGTG CAGCCTCTGGATTCACCTTTAGTAGCTTTTGGATG AGCTGGGTCCGCCAGGCTCCAGGGAAGGGGCTG GAGTGGGTGGCCCACATAAAGCAAGATGGAAGT GAGAAATACTATGTGGACTCTGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGGGGACCTC CCATACAGCTCGGGCTACTACTACGGTATGGACG TCTGGGGCCAAGGGACCACGGTCACCGTCTCCTC A |
| | | | SEQ ID NO: 27499 | SEQ ID NO: 31505 |

FIGURE 50

| | | | | |
|---|---|---|---|---|
| | | AA | SYELTQPPSVSVSPGQTASITCSGDKLGDKYACW YQQKPGQSPVLVIYQDRKRPSGIPERFSGSNSGN TATLTISGTQAMDEADYYCQAWDSSTVVFGGG TKLTVL | EVQLVESGGGLVQPGGSLRLSCAASGFTFSSFWMS WVRQAPGKGLEWVAHIKQDGSEKYYVDSVKGRF TISRDNAKNSLYLQMNSLRAEDTAVYYCARGDLPY SSGYYYGMDVWGQGTTVTVSS |
| | | | SEQ ID NO: 27500 | SEQ ID NO: 31506 |
| iPS:436862 | 21-225_58F8 | NA | TCCTATGAGCTGACTCAGCCACCCTCAGTGTC CGTGTCCCCAGGACAGACAGCCAGCATCACCT GCTCTGGAGATAAATTGGGAAATAAATATGCT TGCTGGTATCAGCAGAAGCCAGGCCAGTCCCC TGTACTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAACTGACCGTCCTA | CAGGTGCAACTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGATGGAAGT AATAAATACTATGCAGACTCCGTGAAGGGCCGA TTCACCATCTCCAGAGACAATTCCAAGAACACGT TGTATCTGCAAATGAACAGCCTGAGAGCTGAGG ACACGGCTGTGTATTACTGTGCGAGAGATGAGGG ACGTGGATATGGTGGCTACGAGAGGGGATATTA CTACTACTACTACGGTATGGACGTCTGGGGCCAA GGGACCACGGTCACCGTCTCCTCA |
| | | | SEQ ID NO: 27501 | SEQ ID NO: 31507 |
| | | AA | SYELTQPPSVSVSPGQTASITCSGDKLGNKYACW YQQKPGQSPVLVIYQDSKRPSGIPERFSGSNSGN TATLTISGTQAMDEADYYCQAWDSSTVVFGGG TKLTVL | QVQLVESGGGVVQPGRSLRLSCAASGFTFSSYGMH WVRQAPGKGLEWVAVISYDGSNKYYADSVKGRFT ISRDNSKNTLYLQMNSLRAEDTAVYYCARDEGRG YGGYERGYYYYYYGMDVWGQGTTVTVSS |
| | | | SEQ ID NO: 27502 | SEQ ID NO: 31508 |

FIGURE 50

| | | | | |
|---|---|---|---|---|
| iPS:436864 | 21-225_58G11 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAACAAGCGGC CCTCAGGGATCCCTGAGCGCTTCTCTGGCTCC AACTCTGGGAACACAGCCACTCTGACCATCAG CGGGACCCAGGCTATGGATGAGGCTGACTATT ACTGTCAGGCGTGGAACAACAACACTGTAATG TTCGGCGGAGGGACCAAGCTGACCGTCCTA | GAGGTGCAGCTGGTGGAATCTGGGGGAGGCTTG GTACAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGATTTCATACATTAGTACTAGTAGTAGT ACCATATTCTACGCAGACTCTGTGAAGGGCCGAT TCACCATCTCCAGAGACAGTGCCAAGAACTCACT GTATCTGCAAATGAACAGCCTGAGAGACGAGGA CACGGCTGTGTATTACTGTGCGAGGGGGGATACA GCTATGGTCCTCTACTACTACGGTATGGACGTCT GGGGCCAAGGGACCACGGTCACCGTCTCCTCA |
| | | | SEQ ID NO: 27503 | SEQ ID NO: 31509 |
| | | AA | SYELTQPPSVSVSPGQTASITCSGDKLGDKYASW YQQKPGQSPVLVIYQDNKRPSGIPERFSGSNSGN TATLTISGTQAMDEADYYCQAWNNNTVMFGGG TKLTVL | EVQLVESGGGLVQPGGSLRLSCAASGFTFSSYSMN WVRQAPGKGLEWISYISTSSSTIFYADSVKGRFTISR DSAKNSLYLQMNSLRDEDTAVYYCARGDTAMVL YYYGMDVWGQGTTVTVSS |
| | | | SEQ ID NO: 27504 | SEQ ID NO: 31510 |
| iPS:436866 | 21-225_59F2 | NA | TCCTATGAACTGACTCAGCCACCCTCAGTGTC CGTGTCCCCAGGACAGACAGCCAGCATCACCT GCTCTGGAGATAAATTGGGGGATAAATATGCT TCTTGGTATCAGCAGAGGCCAGGCCAGTCCCC TGTGTTGGTCATCTATCAAGATAACAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAACACTGTGGTC TTCGGCGGAGGGACCAAGCTGACCGTCCTA | GAGGTGCAGCTGGTGGAGTCTGGGGGGAGGCTTG GTACAGCCTGGGGGGGTCCCTGAGACTCTCCTGTG GAGCCTCTGGATTCACCTTCAGTAGCTATAGCAT GAACTGGGTCCGCCAGGCTCCAGGGAAGGGGACT GGAGTGGGTTTCATACATTAGTGGGAGTAGTAAT ATCATATACTACACAGACTCTGTGAAGGGCCGAT TCACCATCTCCAGAGACAATGCCAAGAACTCACT GTATCTGCAAATGAACAGCCTGAGAGACGAGGA CACGGCTGTGTATTACTGTGCGAGAGCGGATACA CCTATGGTCCTTTACTTCTACGGTATGGACGTCTG GGGCCAAGGGACCACGGTCACCGTCTCCTCA |
| | | | SEQ ID NO: 27505 | SEQ ID NO: 31511 |

FIGURE 50

| | | | | |
|---|---|---|---|---|
| | | AA | SYELTQPPSVSVSPGQTASITCSGDKLGDKYASW YQQRPGQSPVLVIYQDNKRPSGIPERFSGSNSGN TATLTISGTQAMDEADYYCQAWDNNTVVFGGG TKLTVL | EVQLVESGGGLVQPGGSLRLSCGASGFTFSSYSMN WVRQAPGKGLEWVSYISGSSNIIYYTDSVKGRFTIS RDNAKNSLYLQMNSLRDEDTAVYYCARADTPMVL YFYGMDVWGQGTTVTVSS |
| | | | SEQ ID NO: 27506 | SEQ ID NO: 31512 |
| iPS:436868 | 21-225_59B11 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAGCAAGCGGC CCTCAGGGATCCTTGAGCGATTCTCTGGCTCC AATTCTGGGAACACAGCCACTCTGACCATCAG CGGAACCCAGGCTATGGATGAGGCTGACTATT ACTGTCAGGCGTGGGACAGCAGCACTTATGTG GTATTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCGT GCACTGGGTCCGCCAGGCTCCAGGCAAGGGGCT GGAGTGGGTGGCAGCTATATGGTATGATGGAAG TAATAAATACTATGCAGACTCCGTGAAGGGCCG ATTCACCATCTCCAGAGACAATTCCAAGAACACG CTGTATCTGCTAATGAACAGCCTGAGAGCCGAGG ACACGGCTGTGTATTACTGTGCGAGAGATAGGGA CTATTGTAGTAGTTCCAGCTGCCCTTACTACTACT ACTACGGTATGGACGTCTGGGGCCAAGGGACCA CGGTCACCGTCTCCTCA |
| | | | SEQ ID NO: 27507 | SEQ ID NO: 31513 |
| | | AA | SYELTQPPSVSVSPGQTASITCSGDKLGDKYACW YQQKPGQSPVLVIYQDSKRPSGILERFSGSNSGN TATLTISGTQAMDEADYYCQAWDSSTYVVFGG GTKLTVL | QVQLVESGGGVVQPGRSLRLSCAASGFTFSSYGVH WVRQAPGKGLEWVAAIWYDGSNKYYADSVKGRF TISRDNSKNTLYLLMNSLRAEDTAVYYCARDRDYC SSSSCPYYYYYGMDVWGQGTTVTVSS |
| | | | SEQ ID NO: 27508 | SEQ ID NO: 31514 |

FIGURE 50

| iPS:436870 | 21-225_60B1 | NA | TCCTATGAGCTGACTCAGCCACCCTCAGCGTC CGTGTCCCCAGGACAGACAGCCAGCATCACCT GCTCTGGAGATAAACTGGGGGAAAAATATGCT TGCTGGTATCAGCAGAAGCCAGGCCAGTCCCC TGTGTTGGTCATCTATCAAGATAGGAAGCGGC CCTCAGGGATCCCTGAGCGATTCTCTGGCTCC AATTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAACTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGTACTAGTGGAGTG GGTGTGGGCTGGATCCGTCAGCCCCCAGGAAAG GCCCTGGAGTGGCTTGCACTCATTTATTGGCATG AAGATAAGCGCTACAGCCCATCTCTGAAGAGCA GGCTCACCATCACTGAGGACACCTCCAAAAACC AGGTGGACCTTACAATGACCAACATGGCCCCTGT GGACACAGCCACATATTACTGTGCACACGTCACA TATATAGCAGCTCCCTACTGGGGCCAGGGAACCC TGGTCACCGTCTCCTCA |
|---|---|---|---|---|
| | | | SEQ ID NO: 27509 | SEQ ID NO: 31515 |
| | | AA | SYELTQPPSASVSPGQTASITCSGDKLGEKYACW YQQKPGQSPVLVIYQDRKRPSGIPERFSGSNSGN TATLTISGTQAMDEADYYCQAWDSSTVVFGGG TKLTVL | QITLKESGPTLVKPTQTLTLTCTFSGFSLSTSGVGVG WIRQPPGKALEWLALIYWHEDKRYSPSLKSRLTITE DTSKNQVDLTMTNMAPVDTATYYCAHVTYIAAPY WGQGTLVTVSS |
| | | | SEQ ID NO: 27510 | SEQ ID NO: 31516 |
| iPS:436872 | 21-225_60D2 | NA | TCCTATGAACTGACTCAGCCACCCTCAGTGTC CGTGTCCCCAGGACAGACAGCCAGCATCACCT GCTCTGGAAATAAATTGGGGGATAAATATGCT TCTTGGTATCAGCAGAGGCCAGGCCAGTCCCC TGTATTAGTCATCTATCAAGATAACAAGCGGC CCTCAGGGATCCCTGAGCGATTCTCTGGCTCC AACTCTGGAAACACAGCCACTCTGACCATCAG CGGGACCCAGACTATGGATGAGGCTGACTATT ACTGTCAGGCGTGGGACAACAACACTGTGGTC TTCGGCGGAGGGACCAAGCTGACCGTCCTA | GAGGTGCAGCTGGTGGAGTCTGGGGGAGGCTTG GTACAGCCTGGGGGGTCCCTGAGACTCTCCTGTG GAGCCTCTGGATTCACCTTCAGTAGCTATAGCAT GAACTGGGTCCGCCAGGCTCCAGGAAAGGGACT GGAGTGGGTTTCATACATTAGTGAGAGTAGTAAT ATCATATACTACACAGACTCTGTGAAGGGCCGAT TCACCATCTCCAGAGACAATGCCATGAACTCACT GTATCTGCAAATGAACAGCCTGAGAGACGAGGA CACGGCTGTGTATTACTGTGCGAGAGCGGATACA CCTATGGTCCTTTACTTCTACGGTATGGACGTCTG GGGCCAAGGGACCACGGTCACCGTCTCCTCA |
| | | | SEQ ID NO: 27511 | SEQ ID NO: 31517 |

FIGURE 50

| | | | | |
|---|---|---|---|---|
| | | AA | SYELTQPPSVSVSPGQTASITCSGNKLGDKYASW YQQRPGQSPVLVIYQDNKRPSGIPERFSGSNSGN TATLTISGTQTMDEADYYCQAWDNNTVVFGGG TKLTVL | EVQLVESGGGLVQPGGSLRLSCGASGFTFSSYSMN WVRQAPGKGLEWVSYISESSNIIYYTDSVKGRFTIS RDNAMNSLYLQMNSLRDEDTAVYYCARADTPMV LYFYGMDVWGQGTTVTVSS |
| | | | SEQ ID NO: 27512 | SEQ ID NO: 31518 |
| iPS:436874 | 21-225_60A12 | NA | TCCTATGAGCTGACTCAGCCACCCTCAGTGTC CGTGTCCCCAGGACAGACAGCCAACATCACCT GCTCTGGAGATAAAATTGGGGAATAAATATGCT TGCTGGTATCAGCAGAAGCCAGGCCAGTCCCC TGTATTGGTCATTTATCAAGATAGGAAGCGGC CCTCAGGGATCCCTGAGCGATTCTCTGGCTCC AACTCTGGGAACACAGCCATTCTGACCATCAG CGGGACCCAGGCTATGGATGAGGCTGACTATT ACTGTCAGGCGTGGGACAGCAGCACTGCTTTC GGCGGAGGGACCAAGCTGACCGTCCTC | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTTATTGGGATG ATGATAAGCGCTACAGCCCATCTCTGAAGAGCA GGCTCACCATCACCGAGGACACCTCCAAAAACC AGGTGGTCCTTACAATGACCAACATGGACCCTGT GGACACAGCCACATATTACTGTGCACACCTTATA GCAGTGGCCTTTGACTCCTGGGGCCAGGGAACCC TGGTCACCGTCTCCTCA |
| | | | SEQ ID NO: 27513 | SEQ ID NO: 31519 |
| | | AA | SYELTQPPSVSVSPGQTANITCSGDKLGNKYAC WYQQKPGQSPVLVIYQDRKRPSGIPERFSGSNSG NTAILTISGTQAMDEADYYCQAWDSSTAFGGGT KLTVL | QITLKESGPTLVKPTQTLTLTCTFSGFSLSTSGVGVG WIRQPPGKALEWLALIYWDDDKRYSPSLKSRLTITE DTSKNQVVLTMTNMDPVDTATYYCAHLIAVAFDS WGQGTLVTVSS |
| | | | SEQ ID NO: 27514 | SEQ ID NO: 31520 |

FIGURE 50

| iPS:436876 | 21-225_61F5 | NA | TCCTATGAGCTGACTCAGCCACCCTCAGTGTC CGTGTCCCCAGGACAGACAGCCAGCATCACCT GCTCTGGAGATAAATTGGGGGAAAAAATATGCT TGCTGGTATCAGCAGAAGCCAGGCCAGTCCCC TGTGTTGGTCATCTATCAAGATAGCAAGCGGC CCTCAGGGATCCCTGAGCGATTCTCTGGCTCC AATTCTGGGAACACAGCCACTCTGACCATCAG CGGGACCCAGGCTATGGATGAGGCTGACTATT ACTGTCAGGCGTGGGACAGCAGCACTGTGGTT TTCGGCGGAGGGACCAAACTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGTACTAGTGGGTTGG GTGTGGGCTGGATCCGTCAGCCCCCAGGAAAGG CCCTGGAGTGGCTTGCACTCATTTATTCACATGA AGATAAGCGCTACAGCCCATCTCTGAAGAGCAG GCTCACCATCACTGAGGACACCTCCAAAAAACCA GGTGGACCTTACAATGACCAACATGGCCCCTGTG GACACAGCCACATATTACTGTGCACACGTCACAG GTATAGCAGCTCCCTACTGGGGCCAGGGAACCCT GGTCACCGTCTCCTCA |
| | | | SEQ ID NO: 27515 | SEQ ID NO: 31521 |
| | | AA | SYELTQPPSVSVSPGQTASITCSGDKLGEKYACW YQQKPGQSPVLVIYQDSKRPSGIPERFSGSNSGN TATLTISGTQAMDEADYYCQAWDSSTVVFGGG TKLTVL | QITLKESGPTLVKPTQTLTLTCTFSGFSLSTSGLGVG WIRQPPGKALEWLALIYSHEDKRYSPSLKSRLTITE DTSKNQVDLTMTNMAPVDTATYYCAHVTGIAAPY WGQGTLVTVSS |
| | | | SEQ ID NO: 27516 | SEQ ID NO: 31522 |
| IPS:436878 | 21-225_62E3 | NA | TCCTATGACCTGACTCAGCCACCCTCAGTGTC CGTGTCCCCAGGACAGACAGCCAGCATCACCT GCTCTGGAGATAAATTGGGGGAATAAATATGCT TGCTGGTATCAGCAGAAGCCAGGCCAGTCCCC TGTGGTGGTCATCTATCAAGATAGGAAGCGGC CCTCAGGGATCCCTGAGCGATTCTCTGGCTCC AATTCTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCGCTCAGCACTAGTGGAGTG GGTGTGGGCTGGATCCGTCAGCCCCCAGGAAAG GCCCTGGAGTGGCTTGCACTCATTAATTGGAATG ATGATAAGCGCTACAGCCCATCTCTGAAGAGCA GGTTCACCATCACCAGGGACACCTCCAAAGACC AGGTGGTCCTTACAATGACCAACATGGACCCTGT GGACACAGCCACATATTACTGTGCACATAAAGCT ACCTGGGTGGCTTTTGATATCTGGGGCCAAGGGA CAATGGTCACCGTCTCTTCA |
| | | | SEQ ID NO: 27517 | SEQ ID NO: 31523 |

FIGURE 50

| | | | | |
|---|---|---|---|---|
| | | AA | SYDLTQPPSVSVSPGQTASITCSGDKLGNKYAC WYQQKPGQSPVVVIYQDRKRPSGIPERFSGSNSG NTATLTISGTQAMDEADYYCQAWDSSTAVFGG GTKLTVL | QITLKESGPTLVKPTQTLTLTCTFSGFSLSTSGVGVG WIRQPPGKALEWLALINWNDDKRYSPSLKSRFTITR DTSKDQVVLTMTNMDPVDTATYYCAHKATWVAF DIWGQGTMVTVSS |
| | | | SEQ ID NO: 27518 | SEQ ID NO: 31524 |
| iPS:436880 | 21-225_62E8 | NA | TCCTATGACCTGACTCAGCCACCCTCAGTGTC CGTGTCCCCAGGACAGACAGCCAGCATCACCT GCTCTGGAGATAGATTGGGGAATAAATATGCT TCCTGGTATCAGCAGAAGCCAGGCCAGTCCCC TGTGGTGGTCATCTATCAGGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | CAGATCACCTTGAAGGAGTCTGGTCCTACGCTGG TGAAACCCACACAGACCCTCACGCTGACCTGTAC CTTCTCTGGGTTCTCACTCAGCACTAGTGGAGTG GGTGTGGGCTGGATCCGTCAGCCCCCAGGAAAG GCCCTGGAGTGGCTTGCACTCATTAATTGGAATG ATGATAAGCGCTACAGCCCATCTCTGAAGAGCA GGTTCACCATCACCAGGGACACCTCCAAAGACC AGGTGGTCCTTACAATGACCAACATGGACCCTGT GGACACAGCCACATATTACTGTGCACATAAAGCT ACCTGGGTGGCTTTTGATATCTGGGGCCAAGGGA CAATGGTCACCGTCTCTTCA |
| | | | SEQ ID NO: 27519 | SEQ ID NO: 31525 |
| | | AA | SYDLTQPPSVSVSPGQTASITCSGDRLGNKYASW YQQKPGQSPVVVIYQDRKRPSGIPERFSGSNSGN TATLTISGTQAMDEADYYCQAWDSSTAVFGGG TKLTVL | QITLKESGPTLVKPTQTLTLTCTFSGFSLSTSGVGVG WIRQPPGKALEWLALINWNDDKRYSPSLKSRFTITR DTSKDQVVLTMTNMDPVDTATYYCAHKATWVAF DIWGQGTMVTVSS |
| | | | SEQ ID NO: 27520 | SEQ ID NO: 31526 |

FIGURE 50

| iPS:436882 | 21-225_62D10 | NA | TCCTATGACCTGACTCAGCCACCCTCAGTGTC CGTGTCCCCAGGACAGACAGCCAGCATCACCT GCTCTGGAGATAAAATTGGGGAATAAAATATGCT TGCTGGTATCAGCAGAAGCCAGGCCAGTCCCC TGTGGTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | CAGATCACCTTGAAGGAGTCTGGTCCTACGCTGG TGAAATCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTAATTGGAATG ATGATAAGCGCTACAGCCCATCTCTGAAGAGCA GGTTCACCATCACCAGGGACACCTCCAAAGACC AGGTGGTCCTTACAATGACCAACATGGACCCTGT GGACACAGCCACATATTACTGTGCACATAAAGCT ACCTGGGTGGCTTTTGATATCTGGGGCCAAGGGA CAATGGTCACCGTCTCTTCA |
| | | | SEQ ID NO: 27521 | SEQ ID NO: 31527 |
| | | AA | SYDLTQPPSVSVSPGQTASITCSGDKLGNKYAC WYQQKPGQSPVVVIYQDRKRPSGIPERFSGSNSG NTATLTISGTQAMDEADYYCQAWDSSTAVFGG GTKLTVL | QITLKESGPTLVKSTQTLTLTCTFSGFSLSTSGVGVG WIRQPPGKALEWLALINWNDDKRYSPSLKSRFTITR DTSKDQVVLTMTNMDPVDTATYYCAHKATWVAF DIWGQGTMVTVSS |
| | | | SEQ ID NO: 27522 | SEQ ID NO: 31528 |
| iPS:436884 | 21-225_62A12 | NA | TCCTATGACCTGACTCAGCCACCCTCAGTGTC CGTGTCCCCAGGACAGACAGCCAGCATCACCT GCTCTGGAGATAAAATTGGGGAATAAAATATGCT TGCTGGTATCAGCAGAAGCCAGGCCAGTCCCC TGTGGTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTAATTGGAATG ATGATAAACGCTACAGCCCATCTCTGAAGAGCA GGTTCACCATCACCAGGGACACCTCCAAAGACC AGGTGGTCCTTACAATGACCAACATGGACCCTCT GGACACAGCCACATATTACTGTGCACATAAAACT ACCTGGGTGGCTTTTGATATCTGGGGCCAAGGGA CAATGGTCACCGTCTCTTCA |
| | | | SEQ ID NO: 27523 | SEQ ID NO: 31529 |

FIGURE 50

| | | | | |
|---|---|---|---|---|
| | | AA | SYDLTQPPSVSVSPGQTASITCSGDKLGNKYAC WYQQKPGQSPVVVIYQDRKRPSGIPERFSGSNSG NTATLTISGTQAMDEADYYCQAWDSSTAVFGG GTKLTVL | QITLKESGPTLVKPTQTLTLTCTFSGFSLSTSGVGVG WIRQPPGKALEWLALINWNDDKRYSPSLKSRFTITR DTSKDQVVLTMTNMDPLDTATYYCAHKTTWVAF DIWGQGTMVTVSS |
| | | | SEQ ID NO: 27524 | SEQ ID NO: 31530 |
| iPS:436886 | 21-225_62B12 | NA | TCCTATGACCTGACTCAGCCACCCTCAGTGTC CGTGTCCCCAGGACAGACAGCCAGCATCACCT GCTCTGGAGATAAAATTGGGGAATAAATATACT TGCTGGTATCAGCAGAAGCCAGGCCAGTCCCC TGTGGTGGTCATCTATCAAGATAGGAAGCGGC CCTCAGGGATCCCTGAGCGATTCTCTGGCTCC AATTCTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAACACTAGTGGAGTG GGTGTGGGCTGGATCCGTCAGCCCCCAGGAAAG GCCCTGGAGTGGCTTGCACTCATTAATTGGAATG ATGATAAGCGCTACAGCCCGTCTCTGAAGAGCA GGTTCACCATCACCAGGGACACCTCCAAAGACC AGGTGGTCCTTACAATGACCAACATGGACCCTGT GGACACAGCCACATATTACTGTGCACATAAAGCT ACCTGGGTGGCTTTTGATATCTGGGGCCAAGGGA CAATGGTCACCGTCTCTTCA |
| | | | SEQ ID NO: 27525 | SEQ ID NO: 31531 |
| | | AA | SYDLTQPPSVSVSPGQTASITCSGDKLGNKYTCW YQQKPGQSPVVVIYQDRKRPSGIPERFSGSNSGN TATLTISGTQAMDEADYYCQAWDSSTAVFGGG TKLTVL | QITLKESGPTLVKPTQTLTLTCTFSGFSLNTSGVGVG WIRQPPGKALEWLALINWNDDKRYSPSLKSRFTITR DTSKDQVVLTMTNMDPVDTATYYCAHKATWVAF DIWGQGTMVTVSS |
| | | | SEQ ID NO: 27526 | SEQ ID NO: 31532 |

FIGURE 50

| iPS:436888 | 21-225_63G7 | NA | AATTTTATGCTGACTCAGCCCCACTCTGTGTCG GAGTCTCCGGGGAAGACGGTAACCATCTCCTG CACCCGCAGCAATGGCAGCATTGTCAGCAACT ATGTGCAGTGGTACCAGCAGCGCCCGGGCAGT TCCCCCACCACTATGATCTATGAGGATAGCCG AAGACCCTCTGGGGTCCCTGATCGGTTCTCTG GCTCCATCGACAGCTCCTCCAACTCTGCCTCC CTCACCATCTCTGGACTGAAGACTGAGGACGA GGCTGACTACTCCTGTCAGTCTTATGATGGCA TCAATGTGGTATTCGGCGGAGGGACCAAGCTG ACCGTCCTA | GAGGTGCAGCTGGTGGAGTCTGGGGGAGGCTTG GTACAGCCTGGGGGGTCCCTGAGACTCTCCTGTG CAGCCTCTGGATTCACCTTCAGTAGCTATAGCAT GAACTGGGTCCGCCAGGCTCCGGGGAAGGGGCT GGAGTGGGTTTCATACATTAGTAGTAGTACTAGT ACCATATACTACGCAGCCTCTGTGAAGGGCCGAT TCACCATCTCCAGAGACAATGCCAAGAACTCACT GTATCTGCAAATGAACAGCCTGAGAGACGAGGA CACGGCTGTGTATTACTGTGCGAGAGATCACCGT TACTATGATAGTAGTGGTTATTACTCTGATGCTTT TGATATCTGGGGCCAAGGGACAATGGTCACCGTC TCTTCA |
| | | | SEQ ID NO: 27527 | SEQ ID NO: 31533 |
| | | AA | NFMLTQPHSVSESPGKTVTISCTRSNGSIVSNYV QWYQQRPGSSPTTMIYEDSRRPSGVPDRFSGSID SSSNSASLTISGLKTEDEADYSCQSYDGINVVFG GGTKLTVL | EVQLVESGGGLVQPGGSLRLSCAASGFTFSSYSMN WVRQAPGKGLEWVSYISSSTSTIYYAASVKGRFTIS RDNAKNSLYLQMNSLRDEDTAVYYCARDHRYYD SSGYYSDAFDIWGQGTMVTVSS |
| | | | SEQ ID NO: 27528 | SEQ ID NO: 31534 |
| iPS:436890 | 21-225_63A10 | NA | AATTTTATGCTGACTCAGCCCCACTCTGTGTCG GAGTCTCCGGGGAAGACGGTAACCATCTCCTG CACCCGCAGCAATGGCAGCATTGTCAGCAACT ATGTGCAGTGGTACCAGCAGCGCCCGGGCAGT TCCCCCACCACTGTGATCTATGAGGATAAAAG AAGACCCTCAGGGGTCCCTGATCGGTTCTCTG GCTCCATCGACAGCTCCTCCAACTCTGCCTCC CTCACCATCTCTGGACTGAAGACTGAGGACGA GGCTGACTACTACTGTCAGTCTTATGATAGCA TCAATGTGGTATTCGGCGGAGGGACCAAGCTG ACCGTCCTA | GAGGTGCAGCTGGTGGAGTCTGGGGGAGGCTTG GTACAGCCTGGGGGGTCCCTGAGACTCTCCTGTG CAGCCTCTGGATTCACCTTCAGTAGCTATAGCAT GAACTGGGTCCGCCAGGCTCCGGGGAAGGGGCT GGAGTGGGTTTCATACATTAGTAGTAGTACTAGT ACCATATACTACGCAGCCTCTGTGAAGGGCCGAT TCACCATCTCCAGAGACAATGCCAAGAATTCACT GTATCTGCAAATGAACAGCCTGAGAGACGAGGA CACGGCTGTGTATTACTGTGCGAGAGATCACCGT TACTATGATAGTAGTGGTTATTACTCTGATGCTTT TGATATCTGGGGCCAAGGGACAATGGTCACCGTC TCTTCA |
| | | | SEQ ID NO: 27529 | SEQ ID NO: 31535 |

FIGURE 50

| | | | | |
|---|---|---|---|---|
| | | AA | NFMLTQPHSVSESPGKTVTISCTRSNGSIVSNYV QWYQQRPGSSPTTVIYEDKRRPSGVPDRFSGSID SSSNSASLTISGLKTEDEADYYCQSYDSINVVFG GGTKLTVL | EVQLVESGGGLVQPGGSLRLSCAASGFTFSSYSMN WVRQAPGKGLEWVSYISSSTSTIYYAASVKGRFTIS RDNAKNSLYLQMNSLRDEDTAVYYCARDHRYYD SSGYYSDAFDIWGQGTMVTVSS |
| | | | SEQ ID NO: 27530 | SEQ ID NO: 31536 |
| iPS:436892 | 21-225_65E9 | NA | TCCTATGAGCTGACTCAGCCACCCTCAGTGTC CGTGTCCCCAGGACAGACAGCCAGCATCACCT GCTCTGGAGATAAATTGGGGAATAAATATGAT TACTGGTATCAGCAGAAGCCAGGCCAGTCCCC TGTGCTGGTCATCTATCAAGATAGAAAGCGGC CCTCAGGGATCCCTGAGCGATTCTCTGGCTCC AACTCTGGGAACACAGCCACCCTGACCATCAG CGGGACCCAGGCTATGGATGAGGCTGACTATT ACTGTCAGGCGTGGGACAACAGCACTGTGGTA TTCGGCGGAGGGACCAAACTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAATTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGCGTAT TATTATGGTTCGGGGGAGTTATTATAATGAATTTG ATATGTGGGGCCAAGGGACAATGGTCACCGTCTC TTCA |
| | | | SEQ ID NO: 27531 | SEQ ID NO: 31537 |
| | | AA | SYELTQPPSVSVSPGQTASITCSGDKLGNKYDY WYQQKPGQSPVLVIYQDRKRPSGIPERFSGSNSG NTATLTISGTQAMDEADYYCQAWDNSTVVFGG GTKLTVL | QVQLVQSGAEVKKPGASVKVSCKASGYTFTGYYM HWVRQAPGQGLEWMGWINPNSGGTNYAQKFQGR VTMTRDTSISTAYMELSRLRSDDTAVYYCARAYYY GSGSYYNEFDMWGQGTMVTVSS |
| | | | SEQ ID NO: 27532 | SEQ ID NO: 31538 |

FIGURE 50

| iPS:436894 | 21-225_66G9 | NA | TCCTATGACCTGACTCAGCCACCCTCAGTGAC CGTGTCCCCAGGACAGACAGCCAGCATCACCT GCTCTGGAGATAAATTGGGGAATAAATATGCT TGCTGGTATCAGCAGAAGCCAGGCCAGTCCCC TGTGGTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACATCAACACTGCGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTAATTGGAATG ATGATAAGCGCTTCAGCCCATCTCTGAAGAGCAG GTTCACCATCACCAGGGACACCTCCAAAGACCA GGTGGTCCTTACAATGACCAACATGGACCCTGTG GACACAGCCACATATTACTGTGCACATAAAGCTA CCTGGGTGGCTTTTGATATCTGGGGCCAAGGGAC AATGGTCACCGTCTCTTCA |
|---|---|---|---|---|
| | | | SEQ ID NO: 27533 | SEQ ID NO: 31539 |
| | | AA | SYDLTQPPSVTVSPGQTASITCSGDKLGNKYAC WYQQKPGQSPVVVIYQDRKRPSGIPERFSGSNSG NTATLTISGTQAMDEADYYCQAWDINTAVFGG GTKLTVL | QITLKESGPTLVKPTQTLTLTCTFSGFSLSTSGVGVG WIRQPPGKALEWLALINWNDDKRFSPSLKSRFTITR DTSKDQVVLTMTNMDPVDTATYYCAHKATWVAF DIWGQGTMVTVSS |
| | | | SEQ ID NO: 27534 | SEQ ID NO: 31540 |
| iPS:436896 | 21-225_67F10 | NA | TCCTATGAACTGACTCAGCCACCCTCAGTTTCC GTGTCCCCAGGACAGACAGCCAGCATCACCTG CTCTGGAGATAAATTGGGGTATAAATATGCTT GGTGGTATCAGCAGAAGCCAGGCCAGTCCCCT GTGCTGGTCATCTTTGAAGATAGGAAGCGGC CTCAGGGATCCCTGAGCGATTCTCTGGCTCCA ACTCTGGGAACACAGCCACTCTGACCATCAGC GGGACCCAGGCTATGGATGAGGCTGACTATTA CTGTCAGGCGTGGGACAACAGCACTGTGGTAT TCGGCGGAGGGACCAGGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTATGGACAGAAGTTTCAGGGCAG GGTCACCATGACCAGGGACACGTCCATCAGCAC AGCCTACATGGAGCTGAGCAGGCTGAGATCTGA CGACACGGCCGTATATTACTGTGCGAGAACGTAT TTCTATGGTTCGGGGAGTTATTATAACGGCTTTG ACTACTGGGGCCAGGGAACCCTGGTCACCGTCTC CTCA |
| | | | SEQ ID NO: 27535 | SEQ ID NO: 31541 |

FIGURE 50

| | | | | |
|---|---|---|---|---|
| | | AA | SYELTQPPSVSVSPGQTASITCSGDKLGYKYAW WYQQKPGQSPVLVIFEDRKRPSGIPERFSGSNSG NTATLTISGTQAMDEADYYCQAWDNSTVVFGG GTRLTVL | QVQLVQSGAEVKKPGASVKVSCKASGYTFTGYYM HWVRQAPGQGLEWMGWINPNSGGTNYGQKFQGR VTMTRDTSISTAYMELSRLRSDDTAVYYCARTYFY GSGSYYNGFDYWGQGTLVTVSS |
| | | | SEQ ID NO: 27536 | SEQ ID NO: 31542 |
| iPS:436898 | 21-225_68D8 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGTTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAGCACTGTGGTA TTCGGCGGAGGGACCAAGCTGACCGTCCTA | CAGGTACAGCTGCAGCAGTCAGGTCCAGGACTG GTGAAGCCCTCGCAGACCCTCTCACTCACCTGTG CCATCTCCGGGGACAGTGTCTCTAGCAACAGTGC TGCTTGGAACTGGATCAGGCAGTCCCCATCGAGA GGCCTTGAGTGGCTGGGAAGGACATACTACAGG TCCGAGTGCTATAATGATTATGCAGTATCTGTGC AGAGTCGAATAACCATCAACCCAGACACATCCA AGAACCAGTTCTCCCTGCACCTGAACTCTGTGAC TCCCGAGGACACGGCTGTGTATTTCTGTGCAAGA GATAGAGGGCATAGAGGGTTCTACGGTATGGAC GTCTGGGGCCAAGGGACCACGGTCACCGTCTCCT CA |
| | | | SEQ ID NO: 27537 | SEQ ID NO: 31543 |
| | | AA | SYELTQPPSVSVSPGQTASITCSGDKLGDKYACW YQQKPGQSPVLVTYQDSKRPSGIPERFSGSNSGN TATLTISGTQAMDEADYYCQAWDNSTVVFGGG TKLTVL | QVQLQQSGPGLVKPSQTLSLTCAISGDSVSSNSAA WNWIRQSPSRGLEWLGRTYYRSECYNDYAVSVQS RITINPDTSKNQFSLHLNSVTPEDTAVYFCARDRGH RGFYGMDVWGQGTTVTVSS |
| | | | SEQ ID NO: 27538 | SEQ ID NO: 31544 |

FIGURE 50

| iPS:436900 | 21-225_69B9 | NA | TCCTATGAGCTGACTCAGCCACCCTCAGTGTC CGTGTCCCCAGGACAGACAGCCAGCATCACCT GCTCTGGAGATAAATTGGGGGAATAAATATGAT TACTGGTATCAGCAGAAGCCAGGCCAGTCCCC TGTGCTGGTCATCTATCAAGATAGAAAGCGGC CCTCAGGGATCCCTGAGCGATTCTCTGGCTCC AACTCTGGGAACACAGCCACCCTGACCATCAG CGGGACCCAGGCTATGGATGAGGCTGACTATT ACTGTCAGGCGTGGGACAACAGCACTGTGGTA TTCGGCGGAGGGACCAAACTGACCGTCCTA | CAGGTGCAGATGGTGCAGTCTGGGGGATGAGGTG AAGAAGCCTGGGGCCTCAGTGAAGGTCTCGTGC AAGGCTTCGGGATACACCTTCACCGGCTACCATA TGCACTGGGTGCGACAGGCCCCTGGACAAGGGC TTGAGTGGATGGGATGGATCAACCCTAACAGTGG TGGCACAAATTATGCACAGAAGTTTCAGGGCAG GGTCACCATGACCAGGGACACGTCCATCAGCAC AGCCTACATGGAGCTGAGCAGGATGAGATCTGA CGACACGGCCGTGTATTACTGTGCGAGAGCGTAT TATTATGGTTCGGGGGAGTTATTATAATGAATCTG ATATGTGGGGCCAAGGGACAATGGTCACCGTCTC TTCA |
| | | | SEQ ID NO: 27539 | SEQ ID NO: 31545 |
| | | AA | SYELTQPPSVSVSPGQTASITCSGDKLGNKYDY WYQQKPGQSPVLVIYQDRKRPSGIPERFSGSNSG NTATLTISGTQAMDEADYYCQAWDNSTVVFGG GTKLTVL | QVQMVQSGDEVKKPGASVKVSCKASGYTFTGYH MHWVRQAPGQGLEWMGWINPNSGGTNYAQKFQ GRVTMTRDTSISTAYMELSRMRSDDTAVYYCARA YYYGSGSYYNESDMWGQGTMVTVSS |
| | | | SEQ ID NO: 27540 | SEQ ID NO: 31546 |
| iPS:436902 | 21-225_69B11 | NA | TCCTATGAGCTGACTCAGCCACCCTCAGTGTC CGTGTCCCCAGGACAGGCAGCCAGCATCACCT GCTCTGGAGATAAATTGGGGGGATAAATATGCT TGGTGGTATCAGCAGAAGCCAGGCCAGTCCCC TGTGCTGGTCATCTATGAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAGCACTGTGGTA TTCGGCGGAGGGACCAAGCTGACCGTCCTA | CAGGTGCAGCTGGTGCAGTCCGGGGGCTGAGGTG AAGAAGCCTGGGGCCTCAGTGAGGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTATGGACAGAAGTTTCAGGACAG GGTCACCATGACCAGGGACACGTCCATCAGCAC AGCCTTCATGGAGCTGAGCAGGCTGAGATCTGAC GACACGGCCGCATATTACTGTGCGAGAACGTATT ACTATGGGTCGGGGGAGTTATTATAACGGCTTTGA CTACTGGGGCCAGGGAACCCTGGTCACCGTCTCC TCA |
| | | | SEQ ID NO: 27541 | SEQ ID NO: 31547 |

FIGURE 50

| | | | | |
|---|---|---|---|---|
| | | AA | SYELTQPPSVSVSPGQAASITCSGDKLGDKYAW WYQQKPGQSPVLVIYEDRKRPSGIPERFSGSNSG NTATLTISGTQAMDEADYYCQAWDNSTVVFGG GTKLTVL | QVQLVQSGAEVKKPGASVRVSCKASGYTFTGYYM HWVRQAPGQGLEWMGWINPNSGGTNYGQKFQDR VTMTRDTSISTAFMELSRLRSDDTAAYYCARTYYY GSGSYYNGFDYWGQGTLVTVSS |
| | | | SEQ ID NO: 27542 | SEQ ID NO: 31548 |
| iPS:436904 | 21-225_71D4 | NA | TCCTATGAGCTGACTCAGCCACCCTCAGTGTC CGTGTCTCCAGGACAGACAGCCAGCATCACCT GCTCTGGAGATAAATTGGGGGATAAATATGCT TACTGGTATCAGCAGAAACCAGGCCAGTCCCC TGTGGTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGTCAACAGCACTGTGGTA TTCGGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTGTA TGCACTGGGTGCGACAGGCCCCTGGACAAGGGC TTGAGTGGATGGGATGGATCAACCCTAACAGTGG TGGCACAAACTATGCACAGAAGTTTCAGGTCAG GGTCACCATGACCAGGGACACGTCCGTCAGCAC AGTCTACATGGACCTGAGCAGGCTGAGATCTGAC GACACGGCCGTGTATTACTGTGCGAGAGCGTATT ACTATGGTTCGGGGACTTATCATAACGAATTTGA CTACTGGGGGCCAGGGAAGTTTGGTCACCGTCTCC TCA |
| | | | SEQ ID NO: 27543 | SEQ ID NO: 31549 |
| | | AA | SYELTQPPSVSVSPGQTASITCSGDKLGDKYAY WYQQKPGQSPVVVIYQDRKRPSGIPERFSGSNSG NTATLTISGTQAMDEADYYCQAWVNSTVVFGG GTKLTVL | QVQLVQSGAEVKKPGASVKVSCKASGYTFTGYCM HWVRQAPGQGLEWMGWINPNSGGTNYAQKFQVR VTMTRDTSVSTVYMDLSRLRSDDTAVYYCARAYY YGSGTYHNEFDYWGQGSLVTVSS |
| | | | SEQ ID NO: 27544 | SEQ ID NO: 31550 |

FIGURE 50

| iPS:436906 | 21-225_72B4 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGGTGGTATCAGCAGAGGCCAGGCCAGTCCCC TGTGCTGGTCATCTATGAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAACAGCACTGTGGTA TTCGGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTATGGACAGAAGTTTCAGGGCAG GGTCACCATGACCAGGGACACGTCCATCAGCAC AGCCTACATGGAGCTGAGCAGGCTGAGATCTGA CGACACGGCCGTATATTACTGTGCGAGAACGTAT TACTATGGTTCGGGGGAGTTATTATAACGGCTTTG ACTACTGGGGCCAGGGAACCCTGGTCACCGTCTC CTCA |
| | | | SEQ ID NO: 27545 | SEQ ID NO: 31551 |
| | | AA | SYELTQPPSVSVSPGQTASITCSGDKLGDKYAW WYQQRPGQSPVLVIYEDRKRPSGIPERFSGSNSG NTATLTISGTQAMDEADYYCQAWDNSTVVFGG GTKLTVL | QVQLVQSGAEVKKPGASVKVSCKASGYTFTGYYM HWVRQAPGQGLEWMGWINPNSGGTNYGQKFQGR VTMTRDTSISTAYMELSRLRSDDTAVYYCARTYYY GSGSYYNGFDYWGQGTLVTVSS |
| | | | SEQ ID NO: 27546 | SEQ ID NO: 31552 |
| iPS:436908 | 21-225_72D5 | NA | TCCTATGACCTGACTCAGCCACCCTCAGTGTC CGTGTCCCCAGGACAGACAGCCAGCATCACCT GCTCTGGAGATAAATTGGGGGAATAAATATGCT TGCTGGTATCAGCAGAAGCCAGGCCAGTCCCC TGTGGTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTAATTGGAATG ATGATAAGCGCTACAGCCCATCTCTGAAGAGCA GGTTCACCATCACCAGGGACACCTCCAAAGACC AGGTGGTCTTTACAATGACCAACATGGACCCTGT GGACACAGGCACATATTACTGTGCACATAAAGCT ACCTGGGTGGCTTTTGATATCTGGGGCCAAGGGA CAATGGTCACCGTCTCTTCA |
| | | | SEQ ID NO: 27547 | SEQ ID NO: 31553 |

FIGURE 50

| | | | | |
|---|---|---|---|---|
| | | AA | SYDLTQPPSVSVSPGQTASITCSGDKLGNKYAC WYQQKPGQSPVVVIYQDRKRPSGIPERFSGSNSG NTATLTISGTQAMDEADYYCQAWDSSTAVFGG GTKLTVL | QITLKESGPTLVKPTQTLTLTCTFSGFSLSTSGVGVG WIRQPPGKALEWLALINWNDDKRYSPSLKSRFTITR DTSKDQVVFTMTNMDPVDTGTYYCAHKATWVAF DIWGQGTMVTVSS |
| | | | SEQ ID NO: 27548 | SEQ ID NO: 31554 |
| iPS:436910 | 21-225_73G1 | NA | CAGACTGTGGTGACCCAGGAGCCATCGTTCTC AGTGTCCCCTGGAGGGACAGTCACACTCACTT GTGGCTTGAGCTCTGGCTCAGTCTCTACTAGTT ACTACCCCAGCTGGTACCAGCAGACCCCAGGC CAGGCTCCACGCACGCTCATCTACAACACAAA CACTCGCTCTTCTGGGGTCCCTGATCGCTTCTC TGGCTCCATCCTTGGGAACAAAGCTGCCCTCA CCATCACGGGGGGCCCAGGCAGATGATGAATCT GATTATTACTGTGTTCTATATATGGGTAGTGCC ATTTGGGTGTTCGGCGGAGGGACCAAGTTGAC CGTCCTA | CAGGTGCAGCTGGTGGAGTCTGGGGGAGGCGTG GTCCAGCCTGGGAGGTCCCTGAGACTCTCCTGTG CAGGCACTGGATTCACCTTCAGTTACTATGGCAT GCACTGGGTCCGCCAGGCTCCAGGCAAGGGGCT GGAGTGGGTGGCAGTTACAACATATGATGGAAG TAATAAATACTATGCAGACTCCGTGAAGGGCCG ATTCACCAGCTCCAGAGACAATTCCAAGAACAC GCTGTATCTGCAAATGAATAGCCTGAGAGCTGAG GACACGGCTGTGTATCACTGTGCGAGAGAGACT GGAACCTGGGCTTTTGATATCTGGGGCCAAGGGA CAATGGTCACCGTCTCTTTA |
| | | | SEQ ID NO: 27549 | SEQ ID NO: 31555 |
| | | AA | QTVVTQEPSFSVSPGGTVTLTCGLSSGSVSTSYY PSWYQQTPGQAPRTLIYNTNTRSSGVPDRFSGSI LGNKAALTITGAQADDESDYYCVLYMGSAIWV FGGGTKLTVL | QVQLVESGGGVVQPGRSLRLSCAGTGFTFSYYGM HWVRQAPGKGLEWVAVTTYDGSNKYYADSVKGR FTSSRDNSKNTLYLQMNSLRAEDTAVYHCARETGT WAFDIWGQGTMVTVSL |
| | | | SEQ ID NO: 27550 | SEQ ID NO: 31556 |

FIGURE 50

| iPS:436912 | 21-225_73C4 | NA | TCCTATGACCTGACTCAGCCACCCTCAGTGTC CGTGTCCCCAGGACAGACAGCCAGCATCACCT GCTCTGGAGATAAATTGGGGAATAAATATGCT TGCTGGTATCAGCAGAAGCCAGGCCAGTCCCC TGTGGTGGTCATCTATCAAGATATGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTAATTGGAATG ATGATAAGCGCTACAGCCCATCTCTGAAGAGCA GGTTCACCATCACCAGGGACACCTCCAAAGACC AGGTGGTCCTTACAATGACCAACATGGACCCTGT GGACACAGCCACATATTACTGTGCACATAAAACT ACCTGGGTGGCTTTTGATATCTGGGGCCAAGGGA CAATGGTCACCGTCTCTTCA |
|---|---|---|---|---|
| | | | SEQ ID NO: 27551 | SEQ ID NO: 31557 |
| | | AA | SYDLTQPPSVSVSPGQTASITCSGDKLGNKYAC WYQQKPGQSPVVVIYQDMKRPSGIPERFSGSNS GNTATLTISGTQAMDEADYYCQAWDSSTAVFG GGTKLTVL | QITLKESGPTLVKPTQTLTLTCTFSGFSLSTSGVGVG WIRQPPGKALEWLALINWNDDKRYSPSLKSRFTITR DTSKDQVVLTMTNMDPVDTATYYCAHKTTWVAF DIWGQGTMVTVSS |
| | | | SEQ ID NO: 27552 | SEQ ID NO: 31558 |
| iPS:436914 | 21-225_76B4 | NA | CCCTATGAGCTGAATCAGACACCCTCAGTGTC CGTGTCCCCAGGACAGACAGCCAGCATCACCT GCTCTGGAGATAGATTGGGGACTAAATTTGCT TGCTGGTATCAGCAGAAGCCAGGCCAGTCCCC TGTGCTGGTCATTTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTATTC GGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTGGTGGAGTG GGTGTGGGCTGGATCCGTCAGCCCCCAGGAAAG GCCCTGGAGTGGCTTGCACTCATTTATTGGGATG ATGATAAGCGCTACAGCCCATCTCTGAAGAGCA GGCTCACCATCACCAAGGACACCCCCAAAAACC AGGTGGTCCTTACAATGACCAACATGGACCCTGT GGACACAGCCACATATTACTGTGCACACCTTATA GCAGTGGCCTTTGACTACTGGGGCCAGGGAACCC TGGTCACCGTCTCCTCA |
| | | | SEQ ID NO: 27553 | SEQ ID NO: 31559 |

FIGURE 50

| | | | | |
|---|---|---|---|---|
| | | AA | PYELNQTPSVSVSPGQTASITCSGDRLGTKFACW YQQKPGQSPVLVIYQDSKRPSGIPERFSGSNSGN TATLTISGTQAMDEADYYCQAWDSSTVFGGGT KLTVL | QITLKESGPTLVKPTQTLTLTCTFSGFSLSTGGVGVG WIRQPPGKALEWLALIYWDDDKRYSPSLKSRLTIT KDTPKNQVVLTMTNMDPVDTATYYCAHLIAVAFD YWGQGTLVTVSS |
| | | | SEQ ID NO: 27554 | SEQ ID NO: 31560 |
| iPS:436916 | 21-225_74A9 | NA | TCCTATGAGCTGACTCAGCCACCCTCAGTGTC CGTGTCCCCAGGACAGACAGCCAGCATCACCT GCTCTGGAGATAAATTGGGTAATAAATATGTT TGTTGGTATCAGCAGAAGCCAGGCCAGTCCCC TGTGCTGGTCATCTATCAAGATAACAGGCGGC CCTCAGGGATCCCTGAGCGATTCTCTGGCTCC AACTCTGGGAACACAGCCACTCTGACCATCAG CGGGGACCCAGGCTATGGATGAGGCTGACTATT ACTGTCAGGCGTGGGACAGCAGTCCTGTGATA TTCGGCGGAGGGACCAAACTGACCGTCCTA | CAGGTGCAACTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAAT AATAAATCCTATGCAGACTCCGTGAAGGGCCGAT TCACCATCTCCAGAGACATTTCCAAGAACACGCT GTTTCTGCAAATGAACAGCCTGAGAGCCGATGAC ACGGCTGTGTATTACTGTGCGAGAGATCGAGATT ATTGTAGTGGTACCAGCTGCCCTTATTATTACTAC TACGGTATGGACGTCTGGGGCCAAGGGACCACG GTCACCGTCTCCTCA |
| | | | SEQ ID NO: 27555 | SEQ ID NO: 31561 |
| | | AA | SYELTQPPSVSVSPGQTASITCSGDKLGNKYVCW YQQKPGQSPVLVIYQDNRRPSGIPERFSGSNSGN TATLTISGTQAMDEADYYCQAWDSSPVIFGGGT KLTVL | QVQLVESGGGVVQPGRSLRLSCAASGFTFSSYGMH WVRQAPGKGLEWVAVIWYDGNNKSYADSVKGRF TISRDISKNTLFLQMNSLRADDTAVYYCARDRDYC SGTSCPYYYYYGMDVWGQGTTVTVSS |
| | | | SEQ ID NO: 27556 | SEQ ID NO: 31562 |

FIGURE 50

| iPS:436918 | 21-225_77A2 | NA | TCCTATGAGCTGACTCAGCCACCCTCAGTGTC CGTGTCCCCAGGACAGACAGCCAGCATCACCT GCTCTGGAGATAGATTGGGGGATAAATATGCT TGCTGGTATCAGCAGAAGCCAGGCCAGTCCCC TGTACTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGTACTGCGGTA TTCGGCGGAGGGACCAAGCTGACCGTCCTA | CAGATCACCTTGAAGGAGTCTGGTCCTTCGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGTATTCATTTATTGGGATG ATGATAAGCGCTACAGCCCATCTCTGAAGAGCA GGCTCACCATCACCAAGGACACCTCCAAAAACC AGGTGGTCCTTACAATGACCAACATGGACCCTGT GGACACAGCCACATATTACTGTGCACACCTTATA GCAGTGGCCTTTGACTACTGGGGCCAGGGAACCC TGGTCACCGTCTCCTCA |
|---|---|---|---|---|
| | | | SEQ ID NO: 27557 | SEQ ID NO: 31563 |
| | | AA | SYELTQPPSVSVSPGQTASITCSGDRLGDKYACW YQQKPGQSPVLVIYQDRKRPSGIPERFSGSNSGN TATLTISGTQAMDEADYYCQAWDSSTAVFGGG TKLTVL | QITLKESGPSLVKPTQTLTLTCTFSGFSLSTSGVGVG WIRQPPGKALEWLVFIYWDDDKRYSPSLKSRLTIT KDTSKNQVVLTMTNMDPVDTATYYCAHLIAVAFD YWGQGTLVTVSS |
| | | | SEQ ID NO: 27558 | SEQ ID NO: 31564 |
| iPS:436920 | 21-225_74E5 | NA | CAGACTGTGGTGACTCAGGAGCCCTCACTGAC TGTGTCCCCAGGAGGGACAGTCACTCTCACCT GTGCTTCCAGCACTGAAACAGTCACCAGTGGT TCTTATCCGAACTGGTTCCAGCAGAAACCTGG ACAAGCACCCAGGGCACTGATTTATAGTACAA GCAACAAACACTCCTGGACCCCTGCCCGGTTC TCAGGCTCCCTCCTTGGGGGCAAAGCTGCCCT GACACTGTCAGATGTGCAGCCTGAGGACGAG GCTGAGTATTACTGCCTGCTCTACTATGGTGGT GCTCAACTGGTATTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGAAGTGGTGGTTA CTACTGGAGCTGGATCCGCCAGCACCCAGGGAA GGGGCCTGGAGTGGATTGGATACATCTATTACAGT GGGAGCACCTACTACAACCCGTCCCTCAGGAGTC GAGCTTCCATATCAGTAGACACGTCTAAGAACCA GTTCTCCCTGAAGCTGAGCTCTGTGACTGCCGCG GACACGGCCGTGTATTACTGTGCGAGAGATTCAC CAGTGGCTGGTACTGACTACTGGGGCCAGGGAA CCCTGGTCACCGTCTCCTCA |
| | | | SEQ ID NO: 27559 | SEQ ID NO: 31565 |

FIGURE 50

| | | | | | |
|---|---|---|---|---|---|
| | | AA | QTVVTQEPSLTVSPGGTVTLTCASSTETVTSGSY PNWFQQKPGQAPRALIYSTSNKHSWTPARFSGS LLGGKAALTLSDVQPEDEAEYYCLLYYGGAQL VFGGGTKLTVL | QVQLQESGPGLVKPSQTLSLTCTVSGGSIRSGGYY WSWIRQHPGKGLEWIGYIYYSGSTYYNPSLRSRASI SVDTSKNQFSLKLSSVTAADTAVYYCARDSPVAGT DYWGQGTLVTVSS | |
| | | | SEQ ID NO: 27560 | SEQ ID NO: 31566 | |
| iPS:436922 | 21-225_78E9 | NA | TCTTATGAGTTGACTCAGCCACCCTCAGAGTC TGTGTCCCCAGGACAGACAGCCAGCATCACGT GCTCAGGAGATAAATTGGGGAATAAATATGTT TCCTGGTATCAGCAGAAGCCAGGCCAGTCCCC TGTGCTGGTCATCTATCAAGATAACAGGCGGC CGTCAGGGATCCCTGAGCGATTTTCTGGCTCC AACTCTGGGAGCACAGCCACTTTGACCATCAG CGGGACCCAGGCTATGGATGAGGCTGACTATT ACTGTCAGGCGTGGGACAGCAGCCCTGTGATA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAAT AATAAATCCTATGCAGACTCCGTGAAGGGCCGAT TCACCATCTCCAGAGACATTTCCAAAAACACGCT GTATCTGCAAATGAACAGCCTGAGAGCCGAGGA CACGGCTGTGTATTACTGTGCGAGGGATCGAGAT TATTGTAGTAGTACCAGCTGCCCTTATTATTACTA CTACGGTATGGACGTCTGGGGCCAAGGGACCAC GGTCACCGTCTCCTCA | |
| | | | SEQ ID NO: 27561 | SEQ ID NO: 31567 | |
| | | AA | SYELTQPPSESVSPGQTASITCSGDKLGNKYVSW YQQKPGQSPVLVIYQDNRRPSGIPERFSGSNSGS TATLTISGTQAMDEADYYCQAWDSSPVIFGGGT KLTVL | QVQLVESGGGVVQPGRSLRLSCAASGFTFSSYGMH WVRQAPGKGLEWVAVIWYDGNNKSYADSVKGRF TISRDISKNTLYLQMNSLRAEDTAVYYCARDRDYC SSTSCPYYYYYGMDVWGQGTTVTVSS | |
| | | | SEQ ID NO: 27562 | SEQ ID NO: 31568 | |

FIGURE 50

| iPS:436924 | 21-225_74B3 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCACCACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCGGGATTCACCTTCAGTCGATATGGCAT GCACTGGGTCCGCCAGGCTCCAGGCAAGGGGCT GGAGTGGGTGGCAGTTTTTTGGTATGATGGAAGT AATAAAGACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGAGA TTATTGTAGTAGTACCAGCTGCCCCTTACTACTACT ACTACGGTATGGACGTCTGGGGCCAAGGGACCA CGGTCACCGTCTCCTCA |
|---|---|---|---|---|
| | | | SEQ ID NO: 27563 | SEQ ID NO: 31569 |
| | | AA | SYELTQPPSVSVSPGQTASITCSGDKLGDKYACW YQQKPGQSPVLVIYQDSKRPSGIPERFSGSNSGN TATLTISGTQAMDEADYYCQAWDSTTVVFGGG TKLTVL | QVQLVESGGGVVQPGRSLRLSCAASGFTFSRYGMH WVRQAPGKGLEWVAVFWYDGSNKDYADSVKGRF TISRDNSKNTLYLQMNSLRAEDTAVYYCARDRDY CSSTSCPYYYYYGMDVWGQGTTVTVSS |
| | | | SEQ ID NO: 27564 | SEQ ID NO: 31570 |

FIGURE 50

| | | | | |
|---|---|---|---|---|
| iPS:436926 | 21-225_78D10 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT TACTTTCCAAACTGGTTCCAGCAGAAACCTGG ACAAGCACCCAGGGCACTGATTTATAGTACAG ACAACAAACACTCCTGGACCCCTGCCCGGTTC TCAGGCTCCCTCCTTGGGGGCAAAGCTGCCCT GACACTGTCAGGTGTGCAGCCTGAGGACGAG GCTGAGTATTACTGCCTCCTCTACTATGGTGGT GCTCAGCTGATGTTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGTACATCTATTACATT GGGAGTGTTTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTACTACTGTGCGAGAGATGCCC CCGACTTCGGTATGGACGTCTGGGGCCAAGGGA CCACGGTCACCGTCTCCTCA |
| | | | SEQ ID NO: 27565 | SEQ ID NO: 31571 |
| | | AA | QTVVTQEPSLTVSPGGTVTLTCASSTGAVTSGYF PNWFQQKPGQAPRALIYSTDNKHSWTPARFSGS LLGGKAALTLSGVQPEDEAEYYCLLYYGGAQL MFGGGTKLTVL | QVQLQESGPGLVKPSQTLSLTCTVSGGSISSGGYYW SWIRQHPGKGLEWIGYIYYIGSVYYNPSLKSRVTIS VDTSKNQFSLKLSSVTAADTAVYYCARDAPDFGM DVWGQGTTVTVSS |
| | | | SEQ ID NO: 27566 | SEQ ID NO: 31572 |
| iPS:436928 | 21-225_79E7 | NA | TCCTATGAGCTGACTCAGCCACCCTCAGTGTC CGTGTCCCCAGGACAGACAGCCAGCATCACCT GCTCAGGAGATAAAATTGGGGAATAAATATGTT TCCTGGTATCAGCAGAAGCCAGGCCAGTCCCC TGTGCTGGTCATCTATCAAGATAACAGGCGGC CCTCAGGGATCCCTGAGCGATTCTCTGGCTCC AACTCTGGGAGCACAGCCACTCTGACCATCAG CGGGACCCAGGCTATGGATGAGGCTGACTATT ACTGTCAGGCGTGGGACAGCAGCCCTGTGATA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAAT AATAAATCCTATGCAGACTCCGTGAAGGGCCGAT TCACCATCTCCAGAGACATTTCCAAAAACACGCT GTATCTGCAAATGAACAGCCTGAGAGCCGAGGA CACGGCTGTGTATTACTGTGCGAGGGATCGAGAT TATTGTAGTAGTACCAGCTGCCCTTATTATTACTA CTACGGTATGGACGTCTGGGGCCAAGGGACCAC GGTCACCGTCTCCTCA |
| | | | SEQ ID NO: 27567 | SEQ ID NO: 31573 |

FIGURE 50

| | | | | |
|---|---|---|---|---|
| | | AA | SYELTQPPSVSVSPGQTASITCSGDKLGNKYVSW YQQKPGQSPVLVIYQDNRRPSGIPERFSGSNSGS TATLTISGTQAMDEADYYCQAWDSSPVIFGGGT KLTVL | QVQLVESGGGVVQPGRSLRLSCAASGFTFSSYGMH WVRQAPGKGLEWVAVIWYDGNNKSYADSVKGRF TISRDISKNTLYLQMNSLRAEDTAVYYCARDRDYC SSTSCPYYYYYGMDVWGQGTTVTVSS |
| | | | SEQ ID NO: 27568 | SEQ ID NO: 31574 |
| iPS:436932 | 21-225_92A4 | NA | TCCTATGAGCTGACTCAGCCACCCTCAGTGTC CGTGTCCCCAGGACAGACAGCCAACATCACCT GCTCTGGAGATAAATTGGGGAATAAATATGTT TGCTGGTATCAACAGAAGCCAGGCCAGTCCCC TGTGTTGGTCATCTATCAAGATAACAGGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCCCTGTGATA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAAT AATAAATCCTATGCAGACTCCGTGAAGGGCCGAT TCACCATCTACAGAGACATTTCCAAGAACACGCT GTATCTGCAAATGAACAGCCTGAGAGCCGAGGA CACGGCTGTGTATTACTGTGCGAGAGATCGAGAT TATTGTAGTAGTACCAGCTGCCCTTATTATTACTA CTACGGTATGGACGTCTGGGGCCAAGGGACCAC GGTCACCGTCTCCTCA |
| | | | SEQ ID NO: 27569 | SEQ ID NO: 31575 |
| | | AA | SYELTQPPSVSVSPGQTANITCSGDKLGNKYVC WYQQKPGQSPVLVIYQDNRRPSGIPERFSGSNSG NTATLTISGTQAMDEADYYCQAWDSSPVIFGGG TKLTVL | QVQLVESGGGVVQPGRSLRLSCAASGFTFSSYGMH WVRQAPGKGLEWVAVIWYDGNNKSYADSVKGRF TIYRDISKNTLYLQMNSLRAEDTAVYYCARDRDYC SSTSCPYYYYYGMDVWGQGTTVTVSS |
| | | | SEQ ID NO: 27570 | SEQ ID NO: 31576 |

FIGURE 50

| iPS:436934 | 21-225_96B5 | NA | CCCTATGAGCTGAATCAGACACCCTCAGTGTC CGTGTCCCCAGGACAGACAGCCAGCATCACCT GCTCTGGAGATAGATTGGGGACTAAATTTGCT TGCTGGTATCAGCAGAAGCCAGGCCAGTCCCC TGTGCTGGTCATTTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTATTC GGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTGGTGGAGTG GGTGTGGGCTGGATCCGTCAGCCCCCAGGAAAG GCCCTGGAGTGGCTTGCACTCATTTATTGGGATG ATGATAAGCGCTACAGCCCATCTCTGAAGAGCA GGCTCACCATCACCAAGGACACCCCCAAAAACC AGGTGGTCCTTACAATGACCAACATGGACCCTGT GGACACAGCCACATATTACTGTGCACACCTTATA GCAGTGGCCTGTGACTACTGGGGCCAGGGAACC CTGGTCACCGTCTCCTCA |
|---|---|---|---|---|
| | | | SEQ ID NO: 27571 | SEQ ID NO: 31577 |
| | | AA | PYELNQTPSVSVSPGQTASITCSGDRLGTKFACW YQQKPGQSPVLVIYQDSKRPSGIPERFSGSNSGN TATLTISGTQAMDEADYYCQAWDSSTVFGGGT KLTVL | QITLKESGPTLVKPTQTLTLTCTFSGFSLSTGGVGVG WIRQPPGKALEWLALIYWDDDKRYSPSLKSRLTIT KDTPKNQVVLTMTNMDPVDTATYYCAHLIAVACD YWGQGTLVTVSS |
| | | | SEQ ID NO: 27572 | SEQ ID NO: 31578 |
| iPS:436936 | 21-225_97E6 | NA | TCCTATGAGCTGACTCAGCCACCCTCAGTGTC TGTGTCCCCAGGACAGACAGTCAGCATCACCT GCTCTGGAGATAAATTGGGGGAATAAATATGTT TCCTGGTATCAGCAGAAGCCAGGCCAGTCCCC TGTGCTGGTCATCTATCAAGATAACAGGCGGC CGTCAGGGATCCCTGAGCGATTCTCTGGCTCC AACTCTGGGAGCACAGCCACTCTGACCATCAG CGGGACCCAGGCTATGGATGAGGCTGACTATT ACTGTCAGGCGTGGGACAGCACCCCTGTGATA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAAT AATAAATCCTATGCAGACTCCGTGAAGGGCCGAT TCACCATCTCCAGAGACATTTCCAAAAACACGCT GTATCTGCAAATGAACAGCCTGAGAGCCGAGGA CACGGCTGTGTATTACTGTGCGAGGGATCGAGAT TATTGTAGTAGTACCAGCTGCCCTTATTATTACTA CTACGGTATGGACGTCTGGGGCCAAGGGACCAC GGTCACCGTCTCCTCA |
| | | | SEQ ID NO: 27573 | SEQ ID NO: 31579 |

FIGURE 50

| | | AA | SYELTQPPSVSVSPGQTVSITCSGDKLGNKYVSW YQQKPGQSPVLVIYQDNRRPSGIPERFSGSNSGS TATLTISGTQAMDEADYYCQAWDSTPVIFGGGT KLTVL | QVQLVESGGGVVQPGRSLRLSCAASGFTFSSYGMH WVRQAPGKGLEWVAVIWYDGNNKSYADSVKGRF TISRDISKNTLYLQMNSLRAEDTAVYYCARDRDYC SSTSCPYYYYYGMDVWGQGTTVTVSS |
| | | | SEQ ID NO: 27574 | SEQ ID NO: 31580 |
| iPS:436938 | 21-225_146A3 | NA | TCCTATGCGATGACTCAGCCACCCTCAATGTC CGTGTCCCCAGGACAGACAGCCAGCATCACCT GCTCTGGAAATAAATTGGGGAATAGATATGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACATAGCCACTCTGACCATCAG CGGGACCCAGGCTATGGATGAGGCTGACTATT ACTGTCAGGCGTGGGACAGCAGCACTGTGGTA TTCGGCGGAGGGACCAAGCTGACCGTCCTA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTACT ACCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27575 | SEQ ID NO: 31581 |
| | | AA | SYAMTQPPSMSVSPGQTASITCSGNKLGNRYAC WYQQKPGQSPVLVIYQDSKRPSGIPERFSGSNSG NIATLTISGTQAMDEADYYCQAWDSSTVVFGGG TKLTVL | EVQLLESGGGLVQPGGSLRLSCAASGFTFSSYAMS WVRQAPGKGLEWVSVISGGGTTTYYADSVKGRFTI SRDNSKNTLYLQMNSLRAEDTAVYYCAKWRGNPT DYGMDVWGQGTTVTVSS |
| | | | SEQ ID NO: 27576 | SEQ ID NO: 31582 |

FIGURE 50

| iPS:436940 | 21-225_146B8 | NA | TCCTATGAGCTGACTCAGCCACCCTCAGTGTC CGTGTCCCCAGGACAGACAGCCAGCATCACCT GCTCTGGAGATAAATTGGGGGATAAATATGTT TGCTGGTATCAGCAGAAGCCAGGCCAGTCCCC TGTGTTGGTCATCTATCAAGATAGGAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGCACAGCAGCACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGGCAGTTGTATGGTATGGTGGAAAT GATAAAGACTTTGCAGACTCCGTGACGGGCCGAT TCACCATCTCCAGAGACATTTCCAAGAACACACT GTATCTGCAAATGAACAGCCTGAGAGCCGAGGA CACGGCTGTGTATTACTGTGCGAGAGATCGGGAT TATTGTAGTGGTGGTAGCTGCCCTTACTACTACT ACTACGGTATGGACGTCTGGGGCCAAGGGACCA CGGTCACCGTCTCCTCA |
| | | | SEQ ID NO: 27577 | SEQ ID NO: 31583 |
| | | AA | SYELTQPPSVSVSPGQTASITCSGDKLGDKYVCW YQQKPGQSPVLVIYQDRKRPSGIPERFSGSNSGN TATLTISGTQAMDEADYYCQAWHSSTVVFGGG TKLTVL | QVQLVESGGGVVQPGRSLRLSCAASGFTFSTYGMH WVRQAPGKGLEWVAVVWYGGNDKDFADSVTGR FTISRDISKNTLYLQMNSLRAEDTAVYYCARDRDY CSGGSCPYYYYYGMDVWGQGTTVTVSS |
| | | | SEQ ID NO: 27578 | SEQ ID NO: 31584 |

FIGURE 50

| iPS:436942 | 21-225_146H8 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAAGAAGCGGC CCTCAGGGATCCCTGAGCGATTCTCTGGCTCC AACTCTGGGAACACAGCCACTCTGACCATCAG CGGGACCCAGGTTATGGATGAGGCTGACTATT ACTGTCAGGCGTGGGACATCAGAACTGTGGTA TTCGGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCATCAGTTATGATA TCAATTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCAAAAGCA CAGCCTACATGGAGCTGAGCAGCCTGAGATCTG AGGACACGGCCGTGTATTACTGTGCGAGAGGAG ATTATTACTATGATAGTAGTGGTCACCAGCCTTA CTACTACTACTACGGTATGGACGTCTGGGGCCAA GGGACCACGGTCACCGTCTCCTCA |
| | | | SEQ ID NO: 27579 | SEQ ID NO: 31585 |
| | | AA | SYELTQPPSVSVSPGQTASITCSGDKLGDKYACW YQQKPGQSPVLVTYQDKKRPSGIPERFSGSNSGN TATLTISGTQVMDEADYYCQAWDIRTVVFGGGT KLTVL | QVQLVQSGAEVKKPGASVKVSCKASGYTFISYDIN WVRQATGQGLEWMGWMNPNSGNTGYAQKFQGR VTMTRNTSKSTAYMELSSLRSEDTAVYYCARGDY YYDSSGHQPYYYYYGMDVWGQGTTVTVSS |
| | | | SEQ ID NO: 27580 | SEQ ID NO: 31586 |

FIGURE 50

| | | | | |
|---|---|---|---|---|
| iPS:436944 | 21-225_182D12 | NA | TCCTATGAGCTGACTCAGCCACCCTCAGTGTCCGTGTCCCCAGGACAGACAGCCAGCATCACCTGCTCTGGAGATAAATTGGGGGAGAAATATGCTTGCTGGTATCAGCAGAAGTCAGGCCAGTCCCCTGTGTTGGTCATCTATCAAGATAGAAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGTAGAACTGCGGTATTCGGCGGAGGGACCAAGCTGACCGTCCTA | CAGATCACCTTGAAGGAGTCTGGTCCTACGCTGGTGAAACCCACACAGCCCCTCACGCTGACCTGTACCTTCTCTGGGTTCTCACTCAGCACTACTGGAGTGGGTGTGGGCTGGATCCGTCAGCCCCCAGGAAAGGCCCTGGAGTGGCTTGGAATCCTTTTTTGGAATGATGATGAGCGCTACAGCCCATCTCTGAAGAGCAGGCTCACCATCACCAAGGACACCTCCAAAAACCAGGTGGTCCTTACAATGACCAACATGGACCCTGTGGACACAGCCACATATTACTGTGCACACAAATCGCAGCTCGTCTACTTTGACTACTGGGGCCAGGGAACCCTGGTCACCGTCTCCTCA |
| | | | SEQ ID NO: 27581 | SEQ ID NO: 31587 |
| | | AA | SYELTQPPSVSVSPGQTASITCSGDKLGEKYACWYQQKSGQSPVLVIYQDRKRPSGIPERFSGSNSGNTATLTISGTQAMDEADYYCQAWDSRTAVFGGGTKLTVL | QITLKESGPTLVKPTQPLTLTCTFSGFSLSTTGVGVGWIRQPPGKALEWLGILFWNDDERYSPSLKSRLTITKDTSKNQVVLTMTNMDPVDTATYYCAHKSQLVYFDYWGQGTLVTVSS |
| | | | SEQ ID NO: 27582 | SEQ ID NO: 31588 |
| iPS:436946 | 21-225_183F4 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGTTGGTATCAGCAGAAGCCAGGCCAGTCCCCTGTGTTGGTCATCTATCAAGATAAGAAACGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAGCACTGCTGTGGTATTCGGCGGAGGGACCAAACTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAGGACGTATTGTAGTGGTACCACCTGCCCCTACTACTACTACTACGGTCTGGGCGTCTGGGGCCAAGGGACCACGGTCACCGTCTCCTCA |
| | | | SEQ ID NO: 27583 | SEQ ID NO: 31589 |

FIGURE 50

| | | | | | |
|---|---|---|---|---|---|
| | | | AA | SYELTQPPSVSVSPGQTASITCSGDKLGDKYACW YQQKPGQSPVLVIYQDKKRPSGIPERFSGSNSGN TATLTISGTQAMDEADYYCQAWDNSTAVVFGG GTKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARERTYC SGTTCPYYYYYGLGVWGQGTTVTVSS |
| | | | | SEQ ID NO: 27584 | SEQ ID NO: 31590 |
| iPS:436948 | 21-225_183F5 | | NA | CAGACTGTGGTGACCCAGGAGCCATCGTTCTC AGTGTCCCCTGGAGGGACAGTCACACTCACTT GTGGCTTGAGCTCTGGCTCAGTCTCTACTACTT TCTACCCCAGCTGGTACCAGCAGACCCCAGGC CAGGCTCCACGCACGCTCATCTACAACACAAA CACTCGCTCTTCTGGGGTCCCTGATCGCTTCTC TGGCTCCATCCTTGGGAACAAAGCTGCCCTCA CCATCACGGGGGGCCCAGGCAGATGATGAATCT GATTATTACTGTGTGCTTTATATGGGTAGTGGC ATTTGGGTGTTCGGCGGAGGGACCAAGCTGAC CGTCCTA | CAGGTACAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCTCTGGGTCCGCCAGGCTCCAGGCAAGGGGCT GGAGTGGGTGACAGTTATATGGTATGATGGAAGT GGTAAATACTATGCAGACTCCGTGAAGGGCCGA TTCACCATCTCCAGAGACAATTCCAAGAACACAC TGTATCTGCAAATGAACAGCCTGAGAGCCGAGG ACACGGCTGTTTATTACTGTGCGAGAGAGAATTT TTGGAGTGGTGACTACTGGGGGCCAGGGAACCCT GGTCACCGTCTCCTCA |
| | | | | SEQ ID NO: 27585 | SEQ ID NO: 31591 |
| | | | AA | QTVVTQEPSFSVSPGGTVTLTCGLSSGSVSTTFYP SWYQQTPGQAPRTLIYNTNTRSSGVPDRFSGSIL GNKAALTITGAQADDESDYYCVLYMGSGIWVF GGGTKLTVL | QVQLVESGGGVVQPGRSLRLSCAASGFTFSSYGML WVRQAPGKGLEWVTVIWYDGSGKYYADSVKGRF TISRDNSKNTLYLQMNSLRAEDTAVYYCARENFWS GDYWGQGTLVTVSS |
| | | | | SEQ ID NO: 27586 | SEQ ID NO: 31592 |

FIGURE 50

| iPS:436950 | 21-225_184G4 | NA | TCCTATGAGCTGACTCAGCCACCCTCAGTGTC CGTGTCCCCAGGACAGACAGCCAGCATCACCT GCTCTGGAGATAAAATTGGGGGATAAAATTTGCT TGCTGGTATCAGCAGAAGCCAGGCCAGTCCCC TGTGCTGGTCATCTATGAAGATAGGAAGCGGC CCTCAGGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCCGCACTGTGGTA TTCGGCGGAGGGACCCAGCTGACCGTCCTA | CAGGTGCAGCTGGTGGAGTCTGGGGGAGGCGTG GTCCAGCCTGGGAGGTCCCTGAGACTCTCCTGTG CAGTGTCTGGATTCACCTTTAGTAGCTATGGCAT GCACTGGGTCCGCCAGGCTCCAGGCAAGGGGCT GGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGGGGGGC CCCCGTTCTCTACGGTGACTATGTACTTTGACTAC TGGGGCCAGGGAACCCTGGTCACCGTCTCCTCA |
| | | | SEQ ID NO: 27587 | SEQ ID NO: 31593 |
| | | AA | SYELTQPPSVSVSPGQTASITCSGDKLGDKFACW YQQKPGQSPVLVIYEDRKRPSGIPERFSGSNSGN TATLTISGTQAMDEADYYCQAWDSRTVVFGGG TQLTVL | QVQLVESGGGVVQPGRSLRLSCAVSGFTFSSYGMH WVRQAPGKGLEWVAIIWYDGSNKYYADSVKGRFT ISRDNSKNTLYLQMNSLRAEDTAVYYCARGGPPFS TVTMYFDYWGQGTLVTVSS |
| | | | SEQ ID NO: 27588 | SEQ ID NO: 31594 |
| iPS:436952 | 21-225_185D2 | NA | TCCTATGAGCTGACTCAGACACCCTCAGTGTC CGTGTCCCCAGGACAGACAGCCAGCATCACCT GCTCTGGAGATAAAATTGGGGGATAAAATATGCT TGCTGGTATCAGCAGAAGCCAGGCCAGTCCCC TGTGCTGGTCATCTATGAAGATAGGAAGCGGC CCTCAGGGGATCCCTGACCGATTCTCTGGCTCC AACTCTGGGAACACAGCCACTCTGACCATCAG CGGGACCCAGGCTATGGATGAGGCTGACTATT ACTGTCAGGCGTGGGACAGCAGGAAAGTGGT ATTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTATGATGGAAGT GATAAATACTATGCAGACTCCGTGAAGGGCCGA TTCACCATCTCCAGAGACAATTCCAAGAACACGC TGTATCTGCAAATGAACACCCTGAGAGCCGAAG ACACGGCTGTGTATTTCTGTGCGAGAGGGGGGCC CCCGTTCTCTACGGTGACTATGTACTTTGACTACT GGGGCCAGGGAACCCTGGTCACCGTCTCCTCA |
| | | | SEQ ID NO: 27589 | SEQ ID NO: 31595 |

FIGURE 50

| | | | | |
|---|---|---|---|---|
| | | AA | SYELTQTPSVSVSPGQTASITCSGDKLGDKYAC WYQQKPGQSPVLVIYEDRKRPSGIPDRFSGSNSG NTATLTISGTQAMDEADYYCQAWDSRKVVFGG GTKLTVL | QVQLVESGGGVVQPGRSLRLSCAASGFTFSNYGM HWVRQAPGKGLEWVAIIWYDGSDKYYADSVKGR FTISRDNSKNTLYLQMNTLRAEDTAVYFCARGGPP FSTVTMYFDYWGQGTLVTVSS |
| | | | SEQ ID NO: 27590 | SEQ ID NO: 31596 |
| iPS:436954 | 21-225_185G7 | NA | TCCTATGAGCTGACTCAGCCACCCTCAGTGTC CGTGTCCCCAGGACAGACAGCCAGCATCACCT GCTCTGGAGATAAATTGGGGGCATAAATTTGTT TGCTGGTATCAGCAGAAGCCAGGCCAGTCCCC TGTGTTGGTCATCTATCAAGATCGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACGGTATTC GGCGGAGGGACCAAGCTGACCGTCCTA | CAGATCACCCTGAAGGAGTCTGGTCCTACGCTGG TGAAACCCACACAGACCCTCACGTTGACCTGCAC CTTCTCTGGGTTCTCACTCACCACTGGTGGAGTG GGTGTGGGCTGGATCCGTCAGCCCCCAGGAAAG GCCCTGGAGTGGCTTGCACTCATTTATTGGAATG ATGATGAGCGCTACAGCCCATCTCTGAAGAGCA GGCTCACCATCACCAAGGACACCTCCAAAAACC AGGTGGTCCTTACAATGACCAACATGGACCCTGT GGACACAGCCACATATTACTGTGCACACATTATA GCAGTGGCCTTCCAGCATTGGGGGCCAGGGCACCC TGGTCACCGTCTCCTCA |
| | | | SEQ ID NO: 27591 | SEQ ID NO: 31597 |
| | | AA | SYELTQPPSVSVSPGQTASITCSGDKLGHKFVCW YQQKPGQSPVLVTYQDRKRPSGIPERFSGSNSGN TATLTISGTQAMDEADYYCQAWDSSTVFGGGT KLTVL | QITLKESGPTLVKPTQTLTLTCTFSGFSLTTGGVGV GWIRQPPGKALEWLALIYWNDDERYSPSLKSRLTIT KDTSKNQVVLTMTNMDPVDTATYYCAHIIAVAFQ HWGQGTLVTVSS |
| | | | SEQ ID NO: 27592 | SEQ ID NO: 31598 |

FIGURE 50

| iPS:436956 | 21-225_186H6 | NA | TCCTATGAGCTGACTCAGCCACCCTCAGTGTC CGTGTCCCCAGGACAGACAGCCAGCATCACCT GCTCTGGAGATAAAATGGGGGAAAAATATGC TTGCTGGTATCAGCAGAAGCCAGGCCAGTCCC CTGTGCTGGTCATTTATCAAGATAGAAAGCGG CCCTCAGGGATCCCTGAGCGATTCTCTGGCTC CAACTCTGGGAACACAGCCACTCTGACCATCA GCGGGACCCAGGCTATGGATGAGGCTGACTAT TACTGTCAGGCGTGGGACAGCAGCACTGCGGT ATTCGGCGGAGGGACCAAGCTGACCGTCCTA | CAGATCACCTTGAAGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGGATTCATTTCTTGGAATG ATGATAAGCGCTACAGCCCATCTCTGAAGAGCA GCCTCACCATCACCAAGGACACCTCCAAAAACC AGGTGGTCCTTACAATGACCAACATGGACCCTGT GGACACAGCCACATATTACTGTGCACACAAAGC AGCAGCTGTTGCTTTTGATATCTGGGGCCAAGGG ACAATGGTCACCGTCTCTTCA |
|---|---|---|---|---|
| | | | SEQ ID NO: 27593 | SEQ ID NO: 31599 |
| | | AA | SYELTQPPSVSVSPGQTASITCSGDKMGEKYAC WYQQKPGQSPVLVIYQDRKRPSGIPERFSGSNSG NTATLTISGTQAMDEADYYCQAWDSSTAVFGG GTKLTVL | QITLKESGPTLVKPTQTLTLTCTFSGFSLSTSGVGVG WIRQPPGKALEWLGFISWNDDKRYSPSLKSSLTITK DTSKNQVVLTMTNMDPVDTATYYCAHKAAAVAF DIWGQGTMVTVSS |
| | | | SEQ ID NO: 27594 | SEQ ID NO: 31600 |
| iPS:436958 | 21-225_190D1 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT TCCTATCCAAACTGGTTCCAGCAGAAACCTGG ACAAGCACCCAGGGCACTGATTTACAGTACAA GTAACAAACACTCCTGGACCCCTGCCCGGTTC TCAGGCTCCCTCCTTGGGGGCAAAGCTGCCCT GACACTGTCAGGTGTGCAGCCTGAGGACGAG GCTGACTATTACTGCCTGCTCTACTATGGTGGT GCTCAGGTGGCATTCGGCGGAGGGACCAAGTT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGATACATCTATTACAGT GGGAGCACCTACTACAACCCGTCCCTCAAGAGTC GAGTCACCATATCAGTAGACACGTCTAAGAACC AGTTCTCCCTGAAGCTGAGCTCTGTGACTGCCGC GGACACGGCCGTTTATTACTGTGCGAGAGATTCC CCACTACGAGGCTTTGACTACTGGGGCCAGGGA ACCCTGGTCACCGTCTCCTCA |
| | | | SEQ ID NO: 27595 | SEQ ID NO: 31601 |

FIGURE 50

| | | | | |
|---|---|---|---|---|
| | | AA | QTVVTQEPSLTVSPGGTVTLTCASSTGAVTSGSY PNWFQQKPGQAPRALIYSTSNKHSWTPARFSGS LLGGKAALTLSGVQPEDEADYYCLLYYGGAQV AFGGGTKLTVL | QVQLQESGPGLVKPSQTLSLTCTVSGGSISSGGYYW SWIRQHPGKGLEWIGYIYYSGSTYYNPSLKSRVTIS VDTSKNQFSLKLSSVTAADTAVYYCARDSPLRGFD YWGQGTLVTVSS |
| | | | SEQ ID NO: 27596 | SEQ ID NO: 31602 |
| IPS:436960 | 21-225_198D2 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAGTAAT TATGTTTCCTGGTACCAACAGCTCCCAGGAAC AGCCCCCAAAGTCCTCATTTATGACAATAATA AGCGACCCTCAGGGATTCCTGACCGATTCTCT GGCTCCAAGTCTGGCACGTCAGCCACCCTGGG CATCACCGGACTCCAGACTGGGGACGAGGCC GATTATTACTGCGGAACATGGGATAGCAGACT GAATGTTGGGGGTATTCGGCGGAGGGACCAAG CTGACCGTCCTA | CAGGTGCAGCTGGTGGAGTCTGGGGGAGGCGTG GTCCAGCCTGGGAGGTCCCTGAGACTCTCCTGTG CAGCCTCTGGATTCACCTTCAGAAGCTATGGCAT GCATTGGGTCCGCCAGGCTCCAGGCAAGGGGCT GGAGTGGGTGGCAGTTATAATATATGATGGAAGT TATAAGTACTATGCAGACTCCGTGAAGGGCCGAT TCACCATCTCCAGAGACAATTCCAAGAACACGCT GTATCTGCAAATGAACAGCCTGAGAGCTGAGGA GGGGGGTATGGACGTCTGGGGGCCAAGGGACCACG GTCACCGTCTCCTCA |
| | | | SEQ ID NO: 27597 | SEQ ID NO: 31603 |
| | | AA | QSVLTQPPSVSAAPGQKVTISCSGSSSNIGSNYVS WYQQLPGTAPKVLIYDNNKRPSGIPDRFSGSKSG TSATLGITGLQTGDEADYYCGTWDSRLNVGVFG GGTKLTVL | QVQLVESGGGVVQPGRSLRLSCAASGFTFRSYGMH WVRQAPGKGLEWVAVIIYDGSYKYYADSVKGRFT ISRDNSKNTLYLQMNSLRAEDTAVYYCARTYSGG MDVWGQGTTVTVSS |
| | | | SEQ ID NO: 27598 | SEQ ID NO: 31604 |

FIGURE 50

| iPS:436962 | 21-225_190H1 | NA | TCCTATGAGTTGACTCAGCCACCCTCAGTGTC CGTGTCCCCAGGACAGACAGCCAGCATCACCT GCTCTGGAGATAAATTGGGGGATAGATTTGCT TACTGGTATCAGCAGAAGCCAGGCCAGTCCCC TGTACTGGTCATCTATCAAGATAGCAAGCGGC CCTCAGGGGATCCCTGAGCGATTCTCTGGCTCC AACTCTGGGAACACAGCCACTCTGACCATCAG CGGGACCCAGGCTATGGATGAGGCTGACTATT ACTGTAAGGCGTGGGACAGCAGCACTGTGGTA TTCGGCGGAGGGACCAAGCTGACCGTCCTA | CAGGTACAGCTGCAGCAGTCAGGTCCAGGACTG GTGAAGCCCTCGCAGACCCTCTCACTCACCTGTG ACATCTCCGGGGACAGTGTCTCTAGGAAAAGTGC TACTTGGAACTGGATCAGGCAGTCCCCATCGAGA GGGCCTTGAGTGGCTGGGAAAGACATACTACAGG TCCAAGTGGTATAATGATTATGCAGTATCTGTGA AAAGTCGAATAACCATCAATCCAGACACATCCA AGAACCAGTTCTCCCTGCAATTGAACTCTGTGAC TCCCGAGGACACGGCTGTGTATTACTGTGCAAGA GATCCGGGTGGCCTCTTTGACTACTGGGGCCAGG GAACCCTGGTCACCGTCTCCTCA |
|---|---|---|---|---|
| | | | SEQ ID NO: 27599 | SEQ ID NO: 31605 |
| | | AA | SYELTQPPSVSVSPGQTASITCSGDKLGDRFAYW YQQKPGQSPVLVIYQDSKRPSGIPERFSGSNSGN TATLTISGTQAMDEADYYCKAWDSSTVVFGGG TKLTVL | QVQLQQSGPGLVKPSQTLSLTCDISGDSVSRKSAT WNWIRQSPSRGLEWLGKTYYRSKWYNDYAVSVK SRITINPDTSKNQFSLQLNSVTPEDTAVYYCARDPG GLFDYWGQGTLVTVSS |
| | | | SEQ ID NO: 27600 | SEQ ID NO: 31606 |
| iPS:436964 | 21-225_190B3 | NA | TCTTCTGAGCTGACTCAGGACCCTGCTGTGTCT GTGGCCTTGGGACAGACAGTCAGGATCACATG CCAAGGAGACAAACTCAGAACCTATTATGCAA GCTGGTACCAGCAGAAGCCAGGACAGGCCCC TGTACTTGTCGTCTATGGAAAAAAACAACCGGC CCTCAGGGGATCCCAGACCGATTCTCTGGCTCC AGCTCAGGAAACACAGCTTCCTTGACCATCAC TGGGGCTCAGGCGGAAGATGAGGCTGACTATT ACTGTAACTCCCGGGACAGCAGTGGTAACCAT CTTGTACTATTCGGCGGAGGGACCAAGCTGAC CGTCCTA | CAGGTGCAGCTGGTGGAATCTGGGGGAGGCGTG GTCCAGCCTGGGAGGTCCCTGAGACTCTCCTGTG CAGCGTCTGGATTCACCTTCAATAACTATGGCAT ACACTGGGTCCGCCAGGCTCCAGGCAAGGGGCT GGAGTGGGTGGCAGTTATATGGTTTGATGGAGAT AATAAATACTATGCAGACTCCGTGAAGGGCCGA TTCACCATCTCCAGAGACAATTCCAAGAACACGC TGTATCTGCAAATGAACAGCCTGAGAGCCGAAG ACACGGCTGTGTATTACTGTGCGAGAGATAACTG GAACTACGGCGATCACTACTACTACTTCGGTATG GACGTCTGGGGCCAAGGGACCACGGTCACCGTC TCCTCA |
| | | | SEQ ID NO: 27601 | SEQ ID NO: 31607 |

FIGURE 50

| | | | | |
|---|---|---|---|---|
| | | AA | SSELTQDPAVSVALGQTVRITCQGDKLRTYYAS WYQQKPGQAPVLVVYGKNNRPSGIPDRFSGSSS GNTASLTITGAQAEDEADYYCNSRDSSGNHLVL FGGGTKLTVL | QVQLVESGGGVVQPGRSLRLSCAASGFTFNNYGIH WVRQAPGKGLEWVAVIWFDGDNKYYADSVKGRF TISRDNSKNTLYLQMNSLRAEDTAVYYCARDNWN YGDHYYYFGMDVWGQGTTVTVSS |
| | | | SEQ ID NO: 27602 | SEQ ID NO: 31608 |
| iPS:436966 | 21-225_190C3 | NA | CAGTCTGTGTTGACGCAGCCGCCCTCAGTGTC TGCGGCCCCAGGACAGAAGGTCACCATCTCCT GCTCTGGAAGCAGCTCCAACATTGGAAATAAT TATGTATCCTGGTACCAGCAGCTCCCAGGAAC AGCCCCCAAACTCCTCCTTTATGACAGTAATA AGCGACCCTCAGGGATTCCTGGCCGATTCTCT GGCTCCAAGTCTGGCACGTCAGCCACCCTGGG CATCACCGGCCTCCAGACTGGGGACGAGGCCG ATTATTACTGCGGAACATGGGATAGCAGCCTG AGTACTGTGGTATTCGGCGGAGGGACCAAGCT GACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAACTGGTACTA CTACTACTACGGTATGGACGTCTGGGGCCAAGGG ACCACGGTCACCGTCTCCTCA |
| | | | SEQ ID NO: 27603 | SEQ ID NO: 31609 |
| | | AA | QSVLTQPPSVSAAPGQKVTISCSGSSSNIGNNYVS WYQQLPGTAPKLLLYDSNKRPSGIPGRFSGSKSG TSATLGITGLQTGDEADYYCGTWDSSLSTVVFG GGTKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCANWYYY YYGMDVWGQGTTVTVSS |
| | | | SEQ ID NO: 27604 | SEQ ID NO: 31610 |

2568

FIGURE 50

| iPS:436968 | 21-225_190B10 | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAATTATGTATCCTGGTACCAGCATCTCCCAGGAACAGCCCCCAAACTCCTCATTTATGACAATAATAAGCGACCCTCAGGGATTCCTGACCGATTCTCTGGCTCCAAGTCTGGCACGTCAGCCACCCTGGGCATCACCGGACTCCAGACTGGGGACGAGGCCGATTATTACTGCGGAACATGGGATAGCAGCCTGAGTGCTGGGGTTTTCGGCGGAGGGACCAAGCTGACCGTCCTA<br><br>SEQ ID NO: 27605 | CAGGTGCAGCTGGTGGAGTCTGGGGGAGGCGTGGTCCAGCCTGGGAGGTCCCTGAGACTCTCCTGTGTAGCGTCTGGATTCACCTTCAGTAGCTATGGCATGCACTGGGTCCGCCAGGCTCCAGGCAAGGGGCTGGAGTGGGTGGCAGTTATATGGAATGATGGAAGTAAAAAATACCATGTAGACTCCGTGAAGGGCCGATTTACCATCTCCAGAGACAATTCCAAGAACACGCTGTATCTGCAAATGAACAGCCTGAGAGCCGAGGACACGGCTCTGTATTACTGTGCGAGAGATCTGGATAAGAGGAACTTTCCTTATTACTACTACTACGGTATGGACGTCTGGGGCCAAGGGACCACGGTCACCGTCTCCTCA<br><br>SEQ ID NO: 31611 |
| | | AA | QSVLTQPPSVSAAPGQKVTISCSGSSSNIGNNYVSWYQHLPGTAPKLLIYDNNKRPSGIPDRFSGSKSGTSATLGITGLQTGDEADYYCGTWDSSLSAGVFGGGTKLTVL<br><br>SEQ ID NO: 27606 | QVQLVESGGGVVQPGRSLRLSCVASGFTFSSYGMHWVRQAPGKGLEWVAVIWNDGSKKYHVDSVKGRFTISRDNSKNTLYLQMNSLRAEDTALYYCARDLDKRNFPYYYYYGMDVWGQGTTVTVSS<br><br>SEQ ID NO: 31612 |
| iPS:436970 | 21-225_190B8 | NA | TCTTCTGAGCTGACTCAGGACCCTGCTGTGTCTGTGGCCTTGGGACAGACAGTCAGGATCACATGCCAAGGAGACACCCTCAGACCCTATTATGTAAGCTGGTACCAGCAGAAGCCAGGACAGGCCCCTGTACTTGTCATCTATGGTAAAAACAACCGGCCCTCAGGGATCCCAGACCGATTCTCTGGCTCCAGCTCAGGAAACACAGCTTCCTTGACCATCACTGGGGCTCAGGCGGAAGATGAGGCTGACTATTACTGTAACTCCCGGGACAGCAGTGGTAACCATCTTGTGGTATTCGGCGGAGGGACCAAGCTGACCGTCCTA<br><br>SEQ ID NO: 27607 | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTTTGATGGAAGTAATAAATACTATACAGACTCCGTGAAGGGCCGATTCACCATCTCCAGAGACAATTCCAAGAACACGCTGTATCTGCAAATGAACAGCCTGAGAGCCGAGGACACGGCTGTATATTATTGTGCGAGAGATAACTGGAACTACGGCGATTACTACTACTACTACGGTATGGACGTCTGGGGCCAAGGGACCACGGTCACCGTCTCCTCA<br><br>SEQ ID NO: 31613 |

FIGURE 50

| | | | | |
|---|---|---|---|---|
| | | AA | SSELTQDPAVSVALGQTVRITCQGDTLRPYYVS WYQQKPGQAPVLVIYGKNNRPSGIPDRFSGSSSG NTASLTITGAQAEDEADYYCNSRDSSGNHLVVF GGGTKLTVL | QVQLVESGGGVVQPGRSLRLSCAASGFTFSSYGMH WVRQAPGKGLEWVAVIWFDGSNKYYTDSVKGRF TISRDNSKNTLYLQMNSLRAEDTAVYYCARDNWN YGDYYYYYGMDVWGQGTTVTVSS |
| | | | SEQ ID NO: 27608 | SEQ ID NO: 31614 |
| iPS:436972 | 21-225_190C7 | NA | CAGTCTGTGTTGACGCAGCCGCCCTCAGTGTC TGCGGCCCCAGGACAGAAGGTCACCATCTCCT GCTCTGGAGGCAGCTCCAACATTGGGAATAAT TATGTATCCTGGTTTCAGCAGTTCCCAGGAAC AGCCCCCAAATTCCTCATTTATGACAATAATA AGCGACCCTCAGGGATTCCTGACCGATTCTCT GGCTCCAAGTCTGGCACGTCAGCCACCCTGGG CATCACCGGACTCCAGACTGGTGACGAGGCCG ATTATTACTGCGGAACATGGGATCGCACCCTG AGTGATTGGGTATTCGGCGGAGGGACCAAGCT GACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAACTGAGCAGGCTGAGATCTGA CGACACGGCCGTGTATTACTGTGCGAGAGATAG AGCAGTGGCTGGAAACTACTTCTACTACGGTATG GACGTCTGGGGCCAAGGGACCACGGTCACCGTC TCCTCA |
| | | | SEQ ID NO: 27609 | SEQ ID NO: 31615 |
| | | AA | QSVLTQPPSVSAAPGQKVTISCSGGSSNIGNNYV SWFQQFPGTAPKFLIYDNNKRPSGIPDRFSGSKS GTSATLGITGLQTGDEADYYCGTWDRTLSDWV FGGGTKLTVL | QVQLVQSGAEVKKPGASVKVSCKASGYTFTGYYM HWVRQAPGQGLEWMGWINPNSGGTNYAQKFQGR VTMTRDTSISTAYMELSRLRSDDTAVYYCARDRAV AGNYFYYGMDVWGQGTTVTVSS |
| | | | SEQ ID NO: 27610 | SEQ ID NO: 31616 |

FIGURE 50

| iPS:436974 | 21-225_190H7 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAGTAAT TATGTTTCCTGGTACCAACAGCTCCCAGGAAC AGCCCCCAAAGTCCTCATTTATGACAATAATA AGCGACCCTCAGGGATTCCTGACCGATTCTCT GGCTCCAAGTCTGGCACGTCAGCCACCCTGGG CATCACCGGACTCCAGACTGGGGACGAGGCC GATTATTACTGCGGAACATGGGATGGCAGACT GAATGTTGGGGTATTCGGCGGAGGGACCAAG CTGACCGTCCTA | CAGGTGCAGCTGGTGGAGTCTGGGGGAGGCGTG GTCCAGCCTGGGAGGTCCCTGAGACTCTCCTGTG CAGCCTCTGGATTCAACTTCAGAAGCTATGGCAT GCATTGGGTCCGCCAGGCTCCAGGCAAGGGGCT GGAGTGGGTGGCAGTTATAATATATGATGGAAGT TATAAGTACTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGCGAGAACGTATAGC GGGGGTATGGACGTCTGGGGCCAAGGGACCACG GTCACCGTCTCCTCA |
| | | | SEQ ID NO: 27611 | SEQ ID NO: 31617 |
| | | AA | QSVLTQPPSVSAAPGQKVTISCSGSSSNIGSNYVS WYQQLPGTAPKVLIYDNNKRPSGIPDRFSGSKSG TSATLGITGLQTGDEADYYCGTWDGRLNVGVF GGGTKLTVL | QVQLVESGGGVVQPGRSLRLSCAASGFNFRSYGM HWVRQAPGKGLEWVAVIIYDGSYKYYADSVKGRF TISRDNSKNTLYLQMNSLRAEDTAVYYCARTYSGG MDVWGQGTTVTVSS |
| | | | SEQ ID NO: 27612 | SEQ ID NO: 31618 |
| iPS:436976 | 21-225_190D8 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAATCAT TATGTCTCCTGGTACCAGCAGCTTCCAGGAAC AGCCCCCAAACTCCTCATTTATGACAGTAGTA AGCGACCCTCAGAGATTCCTGACCGATTCTCT GGCTCCAAGTCTGGCACGTCCGCCACCCTGGG CATCACCGGACTCCAGACTGGGGACGAGGCC GATTATTACTGCGGAACATGGGATAGTAGTCT GAGTACTGTGGTATTCGGCGGAGGGACCAAGC TGACCGTCCTA | CAGGTGCAGCTGGTGGAGTCTGGGGGAGACGTG GTCCAGCCTGGAAGGTCCCTGAGACTCTCCTGTG AAGCGTCTGGATTCACCTTCAGTAGCTATGGCCT GCACTGGGTCCGCCAGGCTCCCGGCAAGGGGACT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAACTGGTACTA CTACTACTACGGTATGGACGTCTGGGGCCAAGGG ACCACGGTCACCGTCTCCTCA |
| | | | SEQ ID NO: 27613 | SEQ ID NO: 31619 |

FIGURE 50

| | | | | |
|---|---|---|---|---|
| | | AA | QSVLTQPPSVSAAPGQKVTISCSGSSSNIGNHYVS WYQQLPGTAPKLLIYDSSKRPSEIPDRFSGSKSGT SATLGITGLQTGDEADYYCGTWDSSLSTVVFGG GTKLTVL | QVQLVESGGDVVQPGRSLRLSCEASGFTFSSYGLH WVRQAPGKGLEWVAVIWYDGSNKYYADSVKGRF TISRDNSKNTLYLQMNSLRAEDTAVYYCANWYYY YYGMDVWGQGTTVTVSS |
| | | | SEQ ID NO: 27614 | SEQ ID NO: 31620 |
| iPS:436978 | 21-225_190G9 | NA | TCCTATGAGCTGACTCAGCCACCCTCAGTGTC CGTGTCCCCAGGACAGACAGCCAGCATCACCT GCTCTGGAGATAAAATTGGGGGATAGATTTGCT TACTGGTATCAGCAGAAGCCAGGCCAGTCCCC TGTGCTGGTCATCTATCAAGATAACAAGCGGC CCTCAGGGATCCCTGAGCGATTCTCTGGCTCC AACTCTGGGAACACAGCCTCTCTGACCATCAG CGGGACCCAGGCTATGGATGAGGCTGACTATT ACTGTCAGGCGTGGGACAGCAGCACTGTGGTA TTCGGCGGAGGGACCAGGCTGACCGTCCTA | CAGGTACAGTTGCAGCAGTCAGGTCCAGGACTG GTGAAGCCCTCGCAGACCCTCTCACTCACCTGTG CCATCTCCGGGGACAGTGTCTCTAGGAAAAGTGC TACTTGGAACTGGATCAGGCAGTCCCCATCGAGA GGCCTTGAGTGGCTGGGAAGGACATACTACAGG TCCAAGTGGTATAATGATTATGCAGTATCTGTGA AAAGTCGAATAATCATCAATCCAGACACATCCA AGAACCAGTTCTCCCTGCAGCTGAACTCTGTGAC TCCCGAGGACACGGCTGTGTATTACTGTGCAAGA GATCCGGGTGGCCTCTTTGACTACTGGGGCCAGG GAACCCTGGTCACCGTCTCCTCA |
| | | | SEQ ID NO: 27615 | SEQ ID NO: 31621 |
| | | AA | SYELTQPPSVSVSPGQTASITCSGDKLGDRFAYW YQQKPGQSPVLVIYQDNKRPSGIPERFSGSNSGN TASLTISGTQAMDEADYYCQAWDSSTVVFGGG TRLTVL | QVQLQQSGPGLVKPSQTLSLTCAISGDSVSRKSAT WNWIRQSPSRGLEWLGRTYYRSKWYNDYAVSVK SRIIINPDTSKNQFSLQLNSVTPEDTAVYYCARDPGG LFDYWGQGTLVTVSS |
| | | | SEQ ID NO: 27616 | SEQ ID NO: 31622 |

FIGURE 50

| iPS:436980 | 21-225_190C10 | NA | TCTTCTGAGCTGACTCAGGACCCTGCTGTGTCT GTGGCCTTGGGACAGACAGTCAGGATCACATG CCAAGGAGACAGCCTCAGACCCTATTATGCAA GCTGGTACCAGCAGAAGCCAGGACAGGCCCC TGTACTTGTCATCTATGGTAAAAACAACCGGC CCTCAGGGATCCCAGACCGATTCTCTGGCTCC AGTTCAGGAAACACAGCTTCCTTGACCATCAC TGAGGCTCAGGCGGAAGATGAGGCTGACTATT ACTGTAACTCCCGGGACAGCAGTGGTAACCAT CTTGTGGTATTCGGCGGAGGGACCAAGCTGAC CGTCCTA | CAGGTGCAGCTGGTGGAGTCTGGGGGAGGCGTG GTCCAGCCTGGGAGGTCCCTGAGACTCTCCTGTG CAGCGTCTGGATTCACCCTCAGTAACTATGGCAT GCACTGGGTCCGCCAGGCTCCAGGCAAGGGGCT GGAGTGGGTGGCAGTTATATGGTTTGGTGGAGAT AATAAATACTATGCAGACTCCGTGAGGGGCCGA TTCACCATCTCCAGAGACAATTCCAAGAACACGC TGTATCTGCAAATGAACAGCCTGAGAGCCGAGG ACACGGCTGTGTTTTACTGTGCGAGAGATAACTG GAACTACGGCGATCACTACTACTATTACGGAATG GACGTCTGGGGGCCAAGGGACCACGGTCACCGTC TCCTCA |
| | | | SEQ ID NO: 27617 | SEQ ID NO: 31623 |
| | | AA | SSELTQDPAVSVALGQTVRITCQGDSLRPYYAS WYQQKPGQAPVLVIYGKNNRPSGIPDRFSGSSSG NTASLTITEAQAEDEADYYCNSRDSSGNHLVVF GGGTKLTVL | QVQLVESGGGVVQPGRSLRLSCAASGFTLSNYGM HWVRQAPGKGLEWVAVIWFGGDNKYYADSVRGR FTISRDNSKNTLYLQMNSLRAEDTAVFYCARDNW NYGDHYYYYGMDVWGQGTTVTVSS |
| | | | SEQ ID NO: 27618 | SEQ ID NO: 31624 |
| iPS:436982 | 21-225_190D10 | NA | CAGTCTGTGTTGACGCAGCCGCCCTCAGTGTG TGCGGCCCCAGGACAGAAGGTCACCATCTCCT GCTCTGGAAGCAGCTCCAACATTGGGAGTAAT TATGTTTCCTGGTACCAACAGCTCCCAGGAAC AGTCCCCAAAGTCCTCATTTATGACAATAATA AGCGACCCTCAGGGATTCCTGACCGATTCTCT GGCTCCAAGTCTGGCACGTCAGCCACCCTGGG CATCACCGGACTCCAGACTGGGGACGAGGCC GATTATTACTGCGGAACATGGGATAGCAGACT GAATGTTGGGGGTATTCGGCGGAGGGACCAAG CTGACCGTCCTA | CAGGTGCAGCTGGTGGAGTCTGGGGGAGGCGTG GTCCAGCCTGGGAGGTCCCTGAGACTCTCCTGTG CAGCCTCTGGATTCACCTTCAGAAGCTATGGCAT GCATTGGGTCCGCCAGGCTCCAGGCAAGGGGCT GGAGTGGGTGGCAGTTATAATATATGATGGAAGT TATAAGTACTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGCGAGAACGTATAGC GGGGGGTATGGACGTCTGGGGGCCAAGGGACCACG GTCACCGTCTCCTCA |
| | | | SEQ ID NO: 27619 | SEQ ID NO: 31625 |

FIGURE 50

| | | | | |
|---|---|---|---|---|
| | | AA | QSVLTQPPSVSAAPGQKVTISCSGSSSNIGSNYVS WYQQLPGTVPKVLIYDNNKRPSGIPDRFSGSKSG TSATLGITGLQTGDEADYYCGTWDSRLNVGVFG GGTKLTVL | QVQLVESGGGVVQPGRSLRLSCAASGFTFRSYGMH WVRQAPGKGLEWVAVIIYDGSYKYYADSVKGRFT ISRDNSKNTLYLQMNSLRAEDTAVYYCARTYSGG MDVWGQGTTVTVSS |
| | | | SEQ ID NO: 27620 | SEQ ID NO: 31626 |
| iPS:436984 | 21-225_190F10 | NA | CAGACTGTGGTGACTCAGGAGCCCTCACTGAC TGTGTCCCCAGGAGGGACAGTCACTCTCACTT GTGTTTTTAGCACTGGAGCAGTCACCAGTGGT TCCTTTCCAAACTGGTTCCAGCAGAAACCTGG ACAAGCACCCAGGGCACTGATTTATAGTACAA GCAACAAACACTCCTGGACCCCTGCCCGGTTC TCAGGCTCCCTCCTTGGGGGCAAAGCTGCCCT GACACTGTCAGGTGTGCAGCCTGAGGACGAG GCTGACTATTACTGCCTGCTCTACTGTGGTGGT GCTCAGCTGGTGTTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGAAGTGGTGGTGA CTACTGGAGCTGGATCCGCCAGCACCCAGGGGA GGGCCTGGAGTGGATTGGGTACATCTATTACAGT GGGATCACCTACTACAATCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGATAGC AGCTCGCGGGGTATGGACGTCTGGGGCCAAGGG ACCACGGTCACCGTCTCCTCA |
| | | | SEQ ID NO: 27621 | SEQ ID NO: 31627 |
| | | AA | QTVVTQEPSLTVSPGGTVTLTCVFSTGAVTSGSF PNWFQQKPGQAPRALIYSTSNKHSWTPARFSGS LLGGKAALTLSGVQPEDEADYYCLLYCGGAQL VFGGGTKLTVL | QVQLQESGPGLVKPSQTLSLTCTVSGGSIRSGGDY WSWIRQHPGEGLEWIGYIYYSGITYYNPSLKSRVTI SVDTSKNQFSLKLSSVTAADTAVYYCARDSSSRGM DVWGQGTTVTVSS |
| | | | SEQ ID NO: 27622 | SEQ ID NO: 31628 |

FIGURE 50

| iPS:436986 | 21-225_191A1 | NA | CAGTCTGTGTTGACGCAGCCGCCCTCAGTGTC TGCGGCCCCAGGACAGAGGGTCACCATCTCCT GCTCTGGAAGCAGCTCCAACCTTGGAAATAAT TTTGTATCCTGGTACCAGCAGTTCCCAGGAAC AGCCCCCAAACTCCTCATTTATGACAATTATA AGCGACCCTCAGGGATTCCTGACCGATTCTCT GTCTCCAAGTCTGGCACGTCAGCCACCCTGGG CATCACCGGACTCCAGACTGGGGACGAGGCC GATTATTACTGCGGAACATGGGATAGCAGCCT GAATACTGGGGTATTCGGCGGAGGGACCAAG CTGACCGTCCTA | CAGGTGCAGCTGCAGGAGTCGGGCCCAGGACTG GTGAAGCCTTCGGAGACCCTGTCCCTCACCTGCA CTGTCTCTGGTGGCTCCATCAGAAGTTACTACTG GATCTGGATCCGGCAGCCCCCAGGGAAGGGACT GGAGTGGATTGGGTATATCTATTACAGTGGGAGT ACTAAGTACAACCCCTCCCTCAAGAGTCGAGTCA CCATATCAGTAGACACGTCCAAGAACCAGTTCTC CCTGAAACTGAGCTCTGTGACCGCTGCGGACACG GCCGTGTATTACTGTGCGAGAAAGGGAGTGGGA ACCATCCACTTTGACTACTGGGGCCAGGGAACCC TGGTCACCGTCTCCTCA |
| | | | SEQ ID NO: 27623 | SEQ ID NO: 31629 |
| | | AA | QSVLTQPPSVSAAPGQRVTISCSGSSSNLGNNFV SWYQQFPGTAPKLLIYDNYKRPSGIPDRFSVSKS GTSATLGITGLQTGDEADYYCGTWDSSLNTGVF GGGTKLTVL | QVQLQESGPGLVKPSETLSLTCTVSGGSIRSYYWIW IRQPPGKGLEWIGYIYYSGSTKYNPSLKSRVTISVDT SKNQFSLKLSSVTAADTAVYYCARKGVGTIHFDY WGQGTLVTVSS |
| | | | SEQ ID NO: 27624 | SEQ ID NO: 31630 |
| iPS:436988 | 21-225_191A2 | NA | CAGACTGTGGTGACTCAGGAGCCCTCACTGAC TGTGTCCCCAGGAGGGACAGTCACTCTCACTT GTGTTCTTAGCACTGGAGCAGTCACCAGTGGT TCCTTTCCAAACTGGTTCCAGCAGAAACCTGG ACAAGCACCCAGGGCACTGATTTATAGTACAA GCAACAAACACTCCTGGACCCCTGCCCGGTTT TCAGGCTCCCTCCTGGGGGGGCAAAGCTGCCCT GACACTGTCAGGTGTGCAGCCTGAGGACGAG GCTGACTATTACTGCATGCTCTACTGTGGTGGT GCTCAGCTGGTGTTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGAAGTGGTGGTGA CTACTGGAGCTGGATCCGCCAGCACCCAGGGGA GGGCCTGGAGTGGATTGGGTACATCTATTACAGT GGGATCACCTACTACAATCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGATAGC AGCTCGCGGGGTATGGACGTCTGGGGCCAAGGG ACCACGGTCACCGTCTCCTCA |
| | | | SEQ ID NO: 27625 | SEQ ID NO: 31631 |

FIGURE 50

| | | | | |
|---|---|---|---|---|
| | | AA | QTVVTQEPSLTVSPGGTVTLTCVLSTGAVTSGSF PNWFQQKPGQAPRALIYSTSNKHSWTPARFSGS LLGGKAALTLSGVQPEDEADYYCMLYCGGAQL VFGGGTKLTVL | QVQLQESGPGLVKPSQTLSLTCTVSGGSIRSGGDY WSWIRQHPGEGLEWIGYIYYSGITYYNPSLKSRVTI SVDTSKNQFSLKLSSVTAADTAVYYCARDSSSRGM DVWGQGTTVTVSS |
| | | | SEQ ID NO: 27626 | SEQ ID NO: 31632 |
| iPS:436992 | 21-225_191B8 | NA | TCTTCTGAGCTGACTCAGGACCCTGCTGTGTCT GTGGCCTTGGGACAGACAGTCAGGATCACATG CCAAGGAGACACCCTCAGACCCTATTATGCAA GTTGGTACCAGCAGAAGCCAGGACAGGCCCCT GTACTTGTCATCTATGGTAAAAACAACCGGCC CTCAGGGATCTCAGACCGATTCTCTGGCTCCA GCTCAGGAAACACAGCTTCCTTGACCATCACT GGGGCTCAGGCGGAAGATGAGGCTGACTATT ACTGTAACTCCCGGGACAGCAGTGGTAACCAT CTTGTGGTATTCGGCGGAGGGACCAAGCTGAC CGTCCTA | CAGGTGCAGCTGGTGGAGTCTGGGGGAGGCGTG GTCCAGCCTGGGAGGTCCCTGAGACTCTCCTGTG CAGCGTCTGGATTCACCCTCAGTAGCTATGGCAT GCACTGGGTCCGCCAGGCTCCAGGCAAGGGGCT GGAGTGGGTGGCAGTTATATGGTTTGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATAACTG GAACTACGGCGATCACTACTACTACTACGGTATG GACGTCTGGGGCCAAGGGACCACGGTCACCGTC TCCTCA |
| | | | SEQ ID NO: 27627 | SEQ ID NO: 31633 |
| | | AA | SSELTQDPAVSVALGQTVRITCQGDTLRPYYAS WYQQKPGQAPVLVIYGKNNRPSGISDRFSGSSSG NTASLTITGAQAEDEADYYCNSRDSSGNHLVVF GGGTKLTVL | QVQLVESGGGVVQPGRSLRLSCAASGFTLSSYGMH WVRQAPGKGLEWVAVIWFDGSNKYYADSVKGRF TISRDNSKNTLYLQMNSLRAEDTAVYYCARDNWN YGDHYYYYGMDVWGQGTTVTVSS |
| | | | SEQ ID NO: 27628 | SEQ ID NO: 31634 |

FIGURE 50

| iPS:436994 | 21-225_191A9 | NA | TCTTCTGAGCTGACTCAGGACCCTGCTGTGTCT GTGGCCTTGGGACAGACAGTCAGGATCACATG CCAAGGAGACAGCCTCAGACCCTATTATGCAA GCTGGTACCAGCAGAAGCCAGGACAGGCCCC TGTACTTGTCATCTATGGTAAAAACAACCGGC CCTCAGGGATCCCAGACCGATTCTCTGGCTCC AGCTCAGGAAACACAGCTTCCTTGACCATCAC TGAGGCTCAGGCGGAAGATGAGGCTGACTATT ACTGTAACTCCCGGGACAGCTGTGGTAACCAT CTTGTGGTATTCGGCGGAGGGACCAAGCTGAC CGTCCTA | CAGGTGCAGCTGGTGGAGTCTGGGGGAGGCGTG GTCCAGCCTGGGAGGTCCCTGAGACTCTCCTGTG CAGCGTCTGGATTCACCCTCAGTAATTATGGCAT GCACTGGGTCCGCCAGGCTCCAGGCAAGGGGCT GGAGTGGGTGGCAGTTATATGGTTTGGTGGAGAT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTTTTACTGTGCGAGAGATAACTG GAACTACGGCGATCACTACTACTATTACGGTATG GACGTCTGGGGCCAAGGGACCACGGTCACCGTC TCCTCA |
| | | | SEQ ID NO: 27629 | SEQ ID NO: 31635 |
| | | AA | SSELTQDPAVSVALGQTVRITCQGDSLRPYYAS WYQQKPGQAPVLVIYGKNNRPSGIPDRFSGSSSG NTASLTITEAQAEDEADYYCNSRDSCGNHLVVF GGGTKLTVL | QVQLVESGGGVVQPGRSLRLSCAASGFTLSNYGM HWVRQAPGKGLEWVAVIWFGGDNKYYADSVKGR FTISRDNSKNTLYLQMNSLRAEDTAVFYCARDNW NYGDHYYYYGMDVWGQGTTVTVSS |
| | | | SEQ ID NO: 27630 | SEQ ID NO: 31636 |
| iPS:436996 | 21-225_191B9 | NA | CAGTCTGTGTTGACGCAGCCGCCCTCAGTGTC TGCGGCCCCAGGACAGAAGGTCACCATCTCCT GCTCTGGAAGCAGCTCCAACATCGGGAATAAT TATGTATCCTGGTACCAGCAGCTCCCAGGAAC AGCCCCCAAACTCCTCATTTATGACAATAAAA AGCGACCCTCAGGGATTCCTGACCGATTCTCT GGCTCCAAGTCTGGCACGTCAGCCACCCTGGG CATCACCGGACTCCAGACTGGGGACGAGGCC GATTATTACTGCGGAACATGGGATAGCAGCCT GAGTGTTTGTGTCTTCGGAACTGGGACCAAGG TCACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTTTCCATGGCAT GCACTGGGTCCGCCAGGCTCCAGGCAAGGGGCT GGAGTGGGTGGCAGTTATATGGTATGATGGAAGT AAAAAATACTATGCAGACTCCGTGAAGGGCCGA TTCACCATCTCCAGAGACAATTCCAAGAACACGC TGCATCTGCAAATGAACAGTCTGAGAGCCGAGG ACACGGCTGTGTATTACTGTGCGAAAGAAGGGTA TAGCAGTGGCTTTTACAGGGGGGTTTGACAACTGG GGCCAGGGAACCCTGGTCACCGTCTCCTCA |
| | | | SEQ ID NO: 27631 | SEQ ID NO: 31637 |

FIGURE 50

| | | | | |
|---|---|---|---|---|
| | | AA | QSVLTQPPSVSAAPGQKVTISCSGSSSNIGNNYVS WYQQLPGTAPKLLIYDNKKRPSGIPDRFSGSKSG TSATLGITGLQTGDEADYYCGTWDSSLSVCVFG TGTKVTVL | QVQLVESGGGVVQPGRSLRLSCAASGFTFSFHGMH WVRQAPGKGLEWVAVIWYDGSKKYYADSVKGRF TISRDNSKNTLHLQMNSLRAEDTAVYYCAKEGYSS GFYRGFDNWGQGTLVTVSS |
| | | | SEQ ID NO: 27632 | SEQ ID NO: 31638 |
| iPS:437000 | 21-225_191G9 | NA | CAGGCTGTGTCGACTCGGCCGTCTTCCCTCTCT GCATCTCCTGGAGCATCAGCCAGTCTCACCTG CACCTTACGCAGTGGCATCAATGTTGGTACCT ACAGGATATACTGGTACCAGCAGAAGCCAGG GAGTCCTCCCCAGTATCTCCTGAGGTACAAAT CAGACTCAGATAAGCAGCAGGGCTCTGGAGTC CCCAGCCGCTTCTCTGGATCCAAAGATGCTTC GGCCAATGCAGGGATTTTACTCATCTCTGGGC TCCAGTCTGAGGATGAGGCTGACTATTACTGT ATGATTTGGCACAGCAGCGCTGTGGTATTCGG CGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGACACTTATATGGTTTGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGT GGGAGGTACTAGTCCTCCTTACTACTACTACTAC GGTATGGACGTCTGGGGGCCAAGGGACCACGGTC ACCGTCTCCTCA |
| | | | SEQ ID NO: 27633 | SEQ ID NO: 31639 |
| | | AA | QAVSTRPSSLSASPGASASLTCTLRSGINVGTYRI YWYQQKPGSPPQYLLRYKSDSDKQQGSGVPSRF SGSKDASANAGILLISGLQSEDEADYYCMIWHSS AVVFGGGTKLTVL | QVQLVESGGGVVQPGRSLRLSCAASGFTFSTYGMH WVRQAPGKGLEWVTLIWFDGSNKYYADSVKGRFT ISRDNSKNTLYLQMNSLRAEDTAVYYCARDRVGG TSPPYYYYYGMDVWGQGTTVTVSS |
| | | | SEQ ID NO: 27634 | SEQ ID NO: 31640 |

FIGURE 50

| iPS:437002 | 21-225_191H9 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGCT TACTATCCAAACTGGTTGCAGCAGAAACCTGG ACAAGCACCCAGGACACTGATTTATAGTACAA ACAACAAACACTCCTGGACCCCTGCCCGGTTC TCAGGCTCCCTCCTTGGGGGCAAAGCTGCCCT GACACTGTCAGATGTGCAGCCTGAGGACGAG GCTGAGTATTACTGCCTGATCTTCTATGGTGGT GTACATGTGATATTTGGCGGAGGGACCAAGCT GACCGTCCTA | CAGGTGCAGCTGGTGCAGTCTGGGGCTGAGGTG AAGAAGCCTGGGGCCTCAGTGAAGGTCTCCTGTA AGGTTTCTGGATATACCTTCACCGGCTACAATAT GCACTGGGTGCGACAGGCCCCTGGACAAGGGCT TGAGTGGATGGGATGGATCAACCCTAATAGTGGT GGCACAAACTATGCACACAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGAGAGATTTCT ATGATAGTGGTGGAGAAGGGTGGTTCGACCCCTG GGGCCAGGGAACCCTGGTCACCGTCTCCTCA |
| | | | SEQ ID NO: 27635 | SEQ ID NO: 31641 |
| | | AA | QTVVTQEPSLTVSPGGTVTLTCASSTGAVTSAYY PNWLQQKPGQAPRTLIYSTNNKHSWTPARFSGS LLGGKAALTLSDVQPEDEAEYYCLIFYGGVHVIF GGGTKLTVL | QVQLVQSGAEVKKPGASVKVSCKVSGYTFTGYNM HWVRQAPGQGLEWMGWINPNSGGTNYAHKFQGR VTMTRDTSISTAYMELSRLRSDDTAVYYCARDFYD SGGEGWFDPWGQGTLVTVSS |
| | | | SEQ ID NO: 27636 | SEQ ID NO: 31642 |
| iPS:437006 | 21-225_192G2 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAATAAT TATGTATCCTGGTACCAGCAGCTCCCAGGAAC AGCCCCCAAACTCCTCATTTATGACAATAATA AGCGACCCTCAAGGATTCCTGACCGATTCTCT GGCTCCAAGTCTGGCACGTCAGCCACCCTGGG CATCACCGGACTCCAGACTGGGGACGAGGCC GATTATTACTGCGGAACATGGGATAGCAGCCT GAGTGCTGGGGTATTCGGCGGAGGGACCAAG CTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGACAGTTATATGGAATGATGGAAG TAATAAATACTATGCAGACTCCGTGAAGGGCCG ATTTACCATCTCCAGAGACAATTCCAAGAACACG CTGTATCTGCAAATGAACAGCCTGAGAGCCGAG GACACGGCTCTGTATTACTGTGCGAGAGATCTGG ATAAGAGGAACTTTCCTTATTACTACTACTACGG TATGGACGTCTGGGGGCCAAGGGACCACGGTCAC CGTCTCCTCA |
| | | | SEQ ID NO: 27637 | SEQ ID NO: 31643 |

FIGURE 50

| | | | | |
|---|---|---|---|---|
| | | AA | QSVLTQPPSVSAAPGQKVTISCSGSSSNIGNNYVS WYQQLPGTAPKLLIYDNNKRPSRIPDRFSGSKSG TSATLGITGLQTGDEADYYCGTWDSSLSAGVFG GGTKLTVL | QVQLVESGGGVVQPGRSLRLSCAASGFTFSSYGMH WVRQAPGKGLEWVTVIWNDGSNKYYADSVKGRF TISRDNSKNTLYLQMNSLRAEDTALYYCARDLDKR NFPYYYYYGMDVWGQGTTVTVSS |
| | | | SEQ ID NO: 27638 | SEQ ID NO: 31644 |
| iPS:437008 | 21-225_192E3 | NA | CAGACTGTGGTGACTCAGGAGCCCTCACTGAC TGTGTCCCCAGGAGGGACAGTCACTCTCACCT GTGCTTTCAGCACTGGATCAGTCACCAGTGGT TCCTATCCAAACTGGTTCCAGCAGAAACCTGG ACAAGCACCCAGGGCACTGATTTATAGTACAA ACAACAAACACTCCTGGACCCCTGCCCGGTTC TCAGGCTCCCTCCTTGGGGGCAAAGCTGCCCT GACACTGTCAGGTGTGCAGCGTGAGGACGAG GCTGAGTATTACTGCCTGCTATACTATGGTGG TGCTCAGCTGGTGTTCGGCGGAGGGACCAAGC TGACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCCCCCAGGGAA GGGCCTGGAGTGGATTGGGTACATCTATTACACT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGACGATC CCCTCTACGGAATGGACGTCTGGGGCCAAGGGA CCACGGTCACCGTCTCCTCA |
| | | | SEQ ID NO: 27639 | SEQ ID NO: 31645 |
| | | AA | QTVVTQEPSLTVSPGGTVTLTCAFSTGSVTSGSY PNWFQQKPGQAPRALIYSTNNKHSWTPARFSGS LLGGKAALTLSGVQREDEAEYYCLLYYGGAQL VFGGGTKLTVL | QVQLQESGPGLVKPSQTLSLTCTVSGGSISSGGYYW SWIRQPPGKGLEWIGYIYYTGSTYYNPSLKSRVTIS VDTSKNQFSLKLSSVTAADTAVYYCARDDPLYGM DVWGQGTTVTVSS |
| | | | SEQ ID NO: 27640 | SEQ ID NO: 31646 |

FIGURE 50

| iPS:437010 | 21-225_192G3 | NA | CAGTCTGTGCTGACTCAGCCACCCTCAGCGTC TGGGACCCCCGGACAGAGGGTCACCATGTCTT GTTCTGGAAGCAGCTCCAACATCGGAAGTAAT ACTGTAAACTGGTACCAACAATTCCCAGGAAC GGCCCCCAAACTCCTCATCTATGGTAATAAGC AGCGGCCCTCAAGGGTCCCTGACCGATTCTCT GGCTCCAAGTCTGGCACCTCAGCCTCCCTGGC CATCAGTGGGCTCCAGTCTGAGGATGAGACTG ATTATTACTGTGCAGCGTGGGATGACAGCCTG AATGGTTGGGTGTTCGGCGGAGGGACCAAGCT GACCGTCCTA | CAGGTGCAGCTGCAGGAGTCGGGCCCAGGACTG GTGAAGCCGTCGGAGACCCTGTCCCTCACCTGCA CTGTCTCTGGTGGCTCCATCAGTAATTACTACTG GAGCTGGATCCGGCAGCCCGCCGGGAAGGGACT GGAGTGGATTGGGCGGATCTATTCCAGTGGGAGC ACCAACTACAACCCCTCCCTCAAGAGTCGAGTCA CCATGTCAGTAGACACGTCCAAGAACCAGTTCTC CCTGAAGCTGAACTCTGTGACCGCCGCGGACACG GCCGTGTATTACTGTGCGAAAGGGTGGGAGCTAA ACTACTGGGGCCAGGGAACCCTGGTCACCGTCTC CTCA |
| | | | SEQ ID NO: 27641 | SEQ ID NO: 31647 |
| | | AA | QSVLTQPPSASGTPGQRVTMSCSGSSSNIGSNTV NWYQQFPGTAPKLLIYGNKQRPSRVPDRFSGSK SGTSASLAISGLQSEDETDYYCAAWDDSLNGWV FGGGTKLTVL | QVQLQESGPGLVKPSETLSLTCTVSGGSISNYYWS WIRQPAGKGLEWIGRIYSSGSTNYNPSLKSRVTMS VDTSKNQFSLKLNSVTAADTAVYYCAKGWELNY WGQGTLVTVSS |
| | | | SEQ ID NO: 27642 | SEQ ID NO: 31648 |
| iPS:437012 | 21-225_192G7 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT AACTATCCACAGTGGTTCCAGCAGAAACCTGG ACAAGCACCCAGGGCACTGATTTACAGTACAA CCAACAGACATTCCTGGACCCCTGCCCGGTTT TCAGGCTCCCTCCTGGGGGGCAAAGCTGCCCT GACACTGTCAGGTGTGCAGCCTGAGGACGAG GCTGAGTATTACTGCCTGTTCTACTATGGTGGT GCTCAGGTGATATTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGTACATCTATTACAGA GGGAGTACCTACTACAATCCGTCCCTCAAGAGTC GAGTTACCATATCAGTGGACACGTCTAAGAACCA GTTCTCCCTGAAACTGAACTCTGTGACTGCCGCG GACACGGCCGTGTATTACTGTGCGAGAGACTCCC CGGTGACAGGATTTGACTATTGGGGCCAGGGAAT CCTGGTCACCGTCTCCTCA |
| | | | SEQ ID NO: 27643 | SEQ ID NO: 31649 |

FIGURE 50

| | | | | |
|---|---|---|---|---|
| | | AA | QTVVTQEPSLTVSPGGTVTLTCASSTGAVTSGNY PQWFQQKPGQAPRALIYSTTNRHSWTPARFSGS LLGGKAALTLSGVQPEDEAEYYCLFYYGGAQVI FGGGTKLTVL | QVQLQESGPGLVKPSQTLSLTCTVSGGSISSGGYYW SWIRQHPGKGLEWIGIYYRGSTYYNPSLKSRVTIS VDTSKNQFSLKLNSVTAADTAVYYCARDSPVTGFD YWGQGILVTVSS |
| | | | SEQ ID NO: 27644 | SEQ ID NO: 31650 |
| iPS:437014 | 21-225_192H8 | NA | CAGACTGTGGTGACTCAGGAACCCTCACTGAC TGTGTCCCCAGGAGGGACAGTCACTCTCACCT GTGCTTTCAGCACTGGAACAGTCACCAGTGGT TTCTATCCAAACTGGTTCCAGCAGAAACCTGG ACAAGCACCCAGGGCATTGATTTATAATACAA GCAACAGACACTCCTGGACCCCTGCCCGGTTC TCAGGCTCCCTCCTTGGGGGGCATGGCTGCCCT GACACTGTCAGGTGTGCAGCCTGAGGACGAG GCTGATTATTACTGCCTGCTGTACTATGGTGGT GCTCAGCTGATGTTCGGCGGAGGGACCAAGCT GACCGTCCTA | CAGGTGCAGCTGCAGGAGTCGGGCCCAGGAGTG GTGAAGCCTTCACAGACCCTGTCCCTCACCTGCA CTGTCTCTGGTGGCTCCATCAGCAGTGGTGGTGA CTACTGGAGCTGGATCCGCCAGCACCCAGGGAA GGGCCTGGAGTGGATTGGGTACATCTATTACAGT GGGCCCACCTACTACAACCCGTCCCTCAAGAGTC GACTTACCATGTCAGCAGACACGTCTAAGAACCA GTTCTCCCTGAAGCTGAGCTCTGTGACTGCCGCG GACACGGCCGTGTATTACTGTGCGAGAGATAGCT CCCTCTACGGTATGGACGTCTGGGGGCCAAGGGAC AACGGTCACCGTCTCCTCA |
| | | | SEQ ID NO: 27645 | SEQ ID NO: 31651 |
| | | AA | QTVVTQEPSLTVSPGGTVTLTCAFSTGTVTSGFY PNWFQQKPGQAPRALIYNTSNRHSWTPARFSGS LLGGMAALTLSGVQPEDEADYYCLLYYGGAQL MFGGGTKLTVL | QVQLQESGPGVVKPSQTLSLTCTVSGGSISSGGDY WSWIRQHPGKGLEWIGIYYSGPTYYNPSLKSRLT MSADTSKNQFSLKLSSVTAADTAVYYCARDSSLYG MDVWGQGTTVTVSS |
| | | | SEQ ID NO: 27646 | SEQ ID NO: 31652 |

2582

FIGURE 50

| iPS:437016 | 21-225_193A6 | NA | TCTTCTGAGCTGACTCAGGACCCTGCTGTGTCT GTGGCCTTGGGACAGACAGTCAGGATCACATG CCAAGGAGACAGCCTCAGAAGCTATTATGCAA ACTGGTACCAGCAGAAGCCAGGACAGGCCCC TGTACTTTTCATCTATGCTAAGAACAACCGGC CCTCAGGGATCCCAGACCGATTCTCTGGCTCC AACTCAGGAAACACAGCTTCCTTGACCATCAC TGGGGCTCAGGCGGAAGATGAGGCTGACTATT ACTGTAATTCCCGGGACAGCAGTGGTAACCAT CTGGTATTCGGCGGAGGGACCAAGCTGACCGT CCTA | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTACTACTG GAGCTGGATCCGGCAGCCCCCAGGGAAGGGACT GGAGTGGATTGGGTATATCTATTACAGTGGGAGC ACCAACTACAACCCCTCCCTCAAGAGTCGAGTCA CCATATCAGTAGACACGTCCAAGAACCAGTTCTC CCTGAAGCTGAGCTCTGTGACCGCTGCGGACACG GCCGTGTATTACTGTGCGGGAGGATGGGAGCTAA ACTACTGGGGCCAGGGAACCCTGGTCACCGTCTC CTCA |
|---|---|---|---|---|
| | | | SEQ ID NO: 27647 | SEQ ID NO: 31653 |
| | | AA | SSELTQDPAVSVALGQTVRITCQGDSLRSYYAN WYQQKPGQAPVLFIYAKNNRPSGIPDRFSGSNSG NTASLTITGAQAEDEADYYCNSRDSSGNHLVFG GGTKLTVL | QVQLQESGPGLVKPSETLSLTCTVSGGSISSYYWSW IRQPPGKGLEWIGYIYYSGSTNYNPSLKSRVTISVDT SKNQFSLKLSSVTAADTAVYYCAGGWELNYWGQ GTLVTVSS |
| | | | SEQ ID NO: 27648 | SEQ ID NO: 31654 |
| iPS:437018 | 21-225_193H5 | NA | TCCTATGAACTGACTCAGCCATCCTCAGTGTC CGTGTCCCCAGGACAGACAGCCAGCATCACCT GCTCTGGAGATAAATTGGGGGGATAGATTTGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGCGGTA TTCGGCGGAGGGACCAAGCTGACCGTCCTA | GAGGTGCAGCTGGTGGAGTCTGGGGGAGGCTTG GTAATGCCTGGGGGGTCCCTTAGCCTCTCCTGTG CAGCCTCTGGATTCACTTTCAGTAACGCCTACAT GACCTGGGTCCGCCAGGCTCCAGGGAAGGGGCT GGAGTGGGTTGGCCGTATTAAAAGCAAAACTGA TGGTGGGACAACAGACTACGCTGCACCCGTGAA AGGCAGATTCACCATCTCAAGAGATGATTCAAA AAACACGCTGTATCTGCAAATGAACAGCCTGAA AACCGAGGACACAGCCGTGTATTACTGTACCACA GATCCCGGTGGTATCTTTGACTACTGGGGCCAGG GAACCCTGGTCACCGTCTCCTCA |
| | | | SEQ ID NO: 27649 | SEQ ID NO: 31655 |

FIGURE 50

| | | | | |
|---|---|---|---|---|
| | | AA | SYELTQPSSVSVSPGQTASITCSGDKLGDRFACW YQQKPGQSPVLVIYQDSKRPSGIPERFSGSNSGN TATLTISGTQAMDEADYYCQAWDSSTAVFGGG TKLTVL | EVQLVESGGGLVMPGGSLSLSCAASGFTFSNAYMT WVRQAPGKGLEWVGRIKSKTDGGTTDYAAPVKG RFTISRDDSKNTLYLQMNSLKTEDTAVYYCTTDPG GIFDYWGQGTLVTVSS |
| | | | SEQ ID NO: 27650 | SEQ ID NO: 31656 |
| iPS:437020 | 21-225_193F11 | NA | CAGTATGTGTTGACGCAGCCGCCATCAGTGTC TGCGGCCCCAGGACAGAAGGTCACCATCTCCT GCTTCGGAGGCAGCTCCAACATTGGGAATAAT TATGTATCCTGGTTCCAGCAGTTCCCAGGAAC AGCCCCCAAATTCCTCATTTATGACAATAATA AGCGACCCTCAGGGATTCTTGACCGATTATCT GGCTCCAAGTCTGGCACGTCAGCCACCCTGGA CATCACCGGACTCCAGAATGGGGACGAGGCC GATTATTACTGCGGAACATGGGATCGCACCAT GAGTGATTGGGTATTCGGCGGAGGGACCAAG CTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGAC TTGAGTGGATGGGATGGATCAACCCTTACAGTGG TGGCACAAACTATGCACAGAAGTTTCAGGGCAG GGTCACCATGACCAGGGACACGTCCATCAGCAC AGCCTACATGGAACTGAGCAGGCTGAGATCTGA CGACACGGCCGTGTATTACTGTGCGAGAGATAG AGCAGTGGCTGGAAACTACTTCTACTACGGTATG GACGTCTGGGGCCAAGGGACCACGGTCACCGTC TCCTCA |
| | | | SEQ ID NO: 27651 | SEQ ID NO: 31657 |
| | | AA | QYVLTQPPSVSAAPGQKVTISCFGGSSNIGNNYV SWFQQFPGTAPKFLIYDNNKRPSGILDRLSGSKS GTSATLDITGLQNGDEADYYCGTWDRTMSDWV FGGGTKLTVL | QVQLVQSGAEVKKPGASVKVSCKASGYTFTGYYM HWVRQAPGQGLEWMGWINPYSGGTNYAQKFQGR VTMTRDTSISTAYMELSRLRSDDTAVYYCARDRAV AGNYFYYGMDVWGQGTTVTVSS |
| | | | SEQ ID NO: 27652 | SEQ ID NO: 31658 |

FIGURE 50

| iPS:437022 | 21-225_194G5 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT AACTATCCAAACTGGTTCCAGCAGAAACCTGG ACAAACACCCAGGGCACTGATTTATAGTACAA GCAACAAACACTCCTGGACCCCTGCCCGGTTC TCAGGCTCCCTCCTTGGGGGCAAAGGTGCCCT GACACTGTCAGGTGTGCAGCCTGAGGACGAG GCTGAGTATTACTGCCTGATCTACTATGGTGG TGCTCAGCTGATGTTCGGCGGAGGGACCAAGC TGACCGTCCTA | CAGGTGCAGCTGCAGGAGTCGGGCCCAGGACTG GTGAAGCCTTCACAGACCCTGTCCCTCATCTGCA CTGTCTCTGGTGGCTCCATCAGAAGTGGTGGTGA CTACTGGAGCTGGATCCGCCAGCACCCAGGGAA GGGCCTGGAGTGGATTGGGTACATCTATTACAGT GGGAGCACCTACTACAACCCGTCTCTCAAGAGTC GAGTTACCATATCAGTAGACACGTCTAAGAACCA GTTCTCCCTGAAGCTGAGCTCTGTGACTGCCGCG GACACGGCCGTTTATTACTGTGCGAGAGATCACT CCCTCTACGGTATGGACGTCTGGGGCCAAGGGAC CACGGTCACCGTCTCCTCA |
| | | | SEQ ID NO: 27653 | SEQ ID NO: 31659 |
| | | AA | QTVVTQEPSLTVSPGGTVTLTCASSTGAVTSGNY PNWFQQKPGQTPRALIYSTSNKHSWTPARFSGSL LGGKGALTLSGVQPEDEAEYYCLIYYGGAQLMF GGGTKLTVL | QVQLQESGPGLVKPSQTLSLICTVSGGSIRSGGDYW SWIRQHPGKGLEWIGYIYYSGSTYYNPSLKSRVTIS VDTSKNQFSLKLSSVTAADTAVYYCARDHSLYGM DVWGQGTTVTVSS |
| | | | SEQ ID NO: 27654 | SEQ ID NO: 31660 |
| iPS:437024 | 21-225_194F11 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAATAAT TATGTATCCTGGTACCAGCAGCTCCCAGGAAC AGCCCCCAAACTCCTCATTTATGACAATAATA AGCGACCCTCAGGGATTCCTGACCGATTCTCT GGCTCCAAGTCTGGCACGTCAGCCACCCTGGG CATCACCGGACTCCAGACTGGGGACGAGGCC GATTATTACTGCGGAACATGGGATAGCAGCCT GAGTGCTGGGGTTTTCGGCGGAGGGACCAAGC TGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGAATGATGGAAG TAAAAAATACCATGTAGACTCCGTGAAGGGCCG ATTTACCATCTCCAGAGACAATTCCAAGAACACG CTGTATCTGCAAATGAACAGCCTGAGAGCCGAG GACACGGCTCTGTATTACTGTGCGAGAGATCTGG ATAAGAGGAACTTTCCTTATTACTACTACTACGG TATGGACGTCTGGGGCCAAGGGACCACGGTCAC CGTCTCCTCA |
| | | | SEQ ID NO: 27655 | SEQ ID NO: 31661 |

# EP 4 435 105 A2

FIGURE 50

| | | | | |
|---|---|---|---|---|
| | | AA | QSVLTQPPSVSAAPGQKVTISCSGSSSNIGNNYVS WYQQLPGTAPKLLIYDNNKRPSGIPDRFSGSKSG TSATLGITGLQTGDEADYYCGTWDSSLSAGVFG GGTKLTVL | QVQLVESGGGVVQPGRSLRLSCAASGFTFSSYGMH WVRQAPGKGLEWVAVIWNDGSKKYHVDSVKGRF TISRDNSKNTLYLQMNSLRAEDTALYYCARDLDKR NFPYYYYYGMDVWGQGTTVTVSS |
| | | | SEQ ID NO: 27656 | SEQ ID NO: 31662 |
| iPS:437026 | 21-225_194D12 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT TCCTTTCCAAGCTGGTTCCAGCAGAAACCTGG ACAAGCACCCAGGGCACTGATTTATAGTACAA GCAACAGACACTCCTCGACCCCTGCCCGGTTC TCAGGCTCCCTCCTTGGGGGCAAAGCAGCCCT GACACTGTCAGGTGTGCAGCCTGAGGACGAG GCTGACTATTACTGCCTGATCTACTATGGTGGT GCTCAGCTGGCATTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTGA CTACTGGAGCTGGATCCGCCAGCACCCAGGGAA GGGCCTGGAGTGGATTGGGTACATCTATTACAGT GGGAGTACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGATGGG GCTCGGCACGGTATGGACGTCTGGGGCCAAGGG ACCACGGTCACCGTCTCCTCA |
| | | | SEQ ID NO: 27657 | SEQ ID NO: 31663 |
| | | AA | QTVVTQEPSLTVSPGGTVTLTCASSTGAVTSGSF PSWFQQKPGQAPRALIYSTSNRHSSTPARFSGSL LGGKAALTLSGVQPEDEADYYCLIYYGGAQLAF GGGTKLTVL | QVQLQESGPGLVKPSQTLSLTCTVSGGSISSGGDYW SWIRQHPGKGLEWIGYIYYSGSTYYNPSLKSRVTIS VDTSKNQFSLKLSSVTAADTAVYYCARDGARHGM DVWGQGTTVTVSS |
| | | | SEQ ID NO: 27658 | SEQ ID NO: 31664 |

2586

FIGURE 50

| | | | | |
|---|---|---|---|---|
| iPS:437028 | 21-225_194G12 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAATAAT TATGTATCCTGGTACCAGCAGCTCCCAGGAAC AGCCCCCAAACTCCTCATTTATGACAATAATA AGCGACCCTCAAGGATTCCTGACCGATTCTCT GGCTCCAAGTCTGGCACGTCAGCCACCCTGGG CATCACCGGACTCCAGACTGGGGAGGAGGCC GATTATTACTGCGGAACATGGGATAGCAGCCT GAGTGTTGGGGTATTCGGCGGAGGGACCAAG CTGACCGTCTTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGACAGTTATATGGAATGATGGAAG TAATAAATACTATGCAGACTCCGTGAAGGGCCG ATTTACCATCTCCAGAGACAATTCCAAGAACACG CTGTATCTGCAAATGAACAGCCTGAGAGCCGAG GACACGGCTCTGTATTACTGTGCGAGAGATCTGG ATAAGAGGAACTTTCCTTATTACTACTACTACGG TATGGACGTCTGGGGCCAAGGGACCACGGTCAC CGTCTCCTCA |
| | | | SEQ ID NO: 27659 | SEQ ID NO: 31665 |
| | | AA | QSVLTQPPSVSAAPGQKVTISCSGSSSNIGNNYVS WYQQLPGTAPKLLIYDNNKRPSRIPDRFSGSKSG TSATLGITGLQTGEEADYYCGTWDSSLSVGVFG GGTKLTVL | QVQLVESGGGVVQPGRSLRLSCAASGFTFSSYGMH WVRQAPGKGLEWVTVIWNDGSNKYYADSVKGRF TISRDNSKNTLYLQMNSLRAEDTALYYCARDLDKR NFPYYYYYGMDVWGQGTTVTVSS |
| | | | SEQ ID NO: 27660 | SEQ ID NO: 31666 |
| iPS:437030 | 21-225_195E3 | NA | TCCTATGAGCTGACTCAGCCACCCTCAGTGTC CGTGTCCCCAGGACAGACAGCCAGCATCACCT GCTCTGGAGATAAATTGGGGTATAGATCTGTT TGCTGGTATCAGCAGAAGCCAGGCCAGTCCCC TGTGCTGGTCATCTATGAAGATAGCAAGCGAC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGTGTCACTGTGGTA TTCGGCGGAGGGACCAAGCTGATCGTCCTA | CAGGTGCAGCTGGTGGAGTCTGGGGGAGGCTTG GTCAAGCCTGGAGGGTCCCTGAGACTCTCCTGTG CAGCCTCTGGATTCACCTTCAGTGACTACTACAT GAGCTGGATCCGCCAGGCTCCAGGGAAGGGGCT GGAGTGGGTTTCATATATTACTAGTAGTGGTAAT ACCATATACTACGCAGACTCTGTGAAGGGCCGAT TCACCATCTCCAGGGACAACGCCAAGAACTCACT GTATCTGCAAATGAACAGCCTGAGAGCCGAGGA CACGGCCGTATATTACTGTGCGAGAGATAGTCGA TATTTTGACTGGTTTGACTACTGGGGCCAGGGAA CCCTGGTCACCGTCTCTTCA |
| | | | SEQ ID NO: 27661 | SEQ ID NO: 31667 |

FIGURE 50

| | | | | |
|---|---|---|---|---|
| | | AA | SYELTQPPSVSVSPGQTASITCSGDKLGYRSVCW YQQKPGQSPVLVIYEDSKRPSGIPERFSGSNSGNT ATLTISGTQAMDEADYYCQAWDSVTVVFGGGT KLIVL | QVQLVESGGGLVKPGGSLRLSCAASGFTFSDYYMS WIRQAPGKGLEWVSYITSSGNTIYYADSVKGRFTIS RDNAKNSLYLQMNSLRAEDTAVYYCARDSRYFD WFDYWGQGTLVTVSS |
| | | | SEQ ID NO: 27662 | SEQ ID NO: 31668 |
| IPS:437032 | 21-225_195H6 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTCAT ACTGTAAACTGGTACCAGCAACTCCCAGGAAC GGCCCCCAAACTCCTCATCTATAATAATTATC AGCGGCCCTCAGGGGTCCCTGACCGATTCTCT GGCTCCAAGTCTGGCACCTCAGCCTCCCTGAC CATCAGTGGGCTCCAGTCTGAGGATGAGGCTG ATTATTACTGTGCAACATGGGATGACAGCCTG AGTGTTTGGGTGTTCGGCGGAGGGACCAAGGT GACCGTCCTA | CAGGTGCAGCTGCAGGAGTCGGGCCCAGGACTG GTGAAGCCTTCGGAGACCCTGTCCCTCACCTGCA CTGTCTCTGGTGGCTCCATCAGGAATTACTACTG GAGCTGGATCCGGCAGCCCGCCGGGAAGGGACT GGAGTGGATTGGGCGTATCTATAGCAGTGGGAG CACCAACTACAACCCCTCCCTCAAGAGTCGAGTC TCCATGTCAGTAGACACGTCCAAGAACCAGTTCT CCCTGAAGCTGAGTTCTGTGACCGCCGCGGACAC GGCCGTGTATTACTGTACGAGAGGGTGGGAGCTA AACAACTGGGGCCAGGGAACCCTGGTCACCGTC TCCTCA |
| | | | SEQ ID NO: 27663 | SEQ ID NO: 31669 |
| | | AA | QSVLTQPPSASGTPGQRVTISCSGSSSNIGSHTVN WYQQLPGTAPKLLIYNNYQRPSGVPDRFSGSKS GTSASLTISGLQSEDEADYYCATWDDSLSVWVF GGGTKVTVL | QVQLQESGPGLVKPSETLSLTCTVSGGSIRNYYWS WIRQPAGKGLEWIGRIYSSGSTNYNPSLKSRVSMSV DTSKNQFSLKLSSVTAADTAVYYCTRGWELNNWG QGTLVTVSS |
| | | | SEQ ID NO: 27664 | SEQ ID NO: 31670 |

FIGURE 50

| iPS:437034 | 21-225_195E9 | NA | TCCTATGAGCTGACTCAGCCACCCTCAGTGTC CGTGTCCCCAGGACAGACAGCCAGCATCACCT GCTCAGGAGATAAATTGGGGAATAAATATGCT TACTGGTATCAGCAGAAGCCAGGCCAGTCCCC TGTGCTGGTCATCTATCAAGATAGGAAGCGGC CCTCAGGGATCCCTGAGCGATTCTCTGGCTCC AATTCTGGGAACACAGGCACTTTGACCATCAG CGGGACCCAGGGTATGGATGAGGCTGACTATT ACTGTCAGGCGTGGGACAGAGGAATTGTGGTA TTCGGCGGAGGGACCAAGCTGACCGTCCTA | CAGGTGCAGGTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAGTGG TGCCACAAACTATGCACAGAAGTTTCAGGGCAG GGTCACCATGACCAGGGACACGTCCATCAGCAC AGCCTACATGGAGCTGAACAGGCTGAGATCTGA CGACACGGCCGTGTATTACTGTGCGAGAGCCTAT TACTATGGTTCGGGGACTTATTATAACGAGTTCG ACTACTGGGGCCAGGGAACCCTGGTCACCGTCTC CTCA |
| | | | SEQ ID NO: 27665 | SEQ ID NO: 31671 |
| | | AA | SYELTQPPSVSVSPGQTASITCSGDKLGNKYAY WYQQKPGQSPVLVIYQDRKRPSGIPERFSGSNSG NTGTLTISGTQGMDEADYYCQAWDRGIVVFGG GTKLTVL | QVQVVQSGAEVKKPGASVKVSCKASGYTFTGYYM HWVRQAPGQGLEWMGWINPNSGATNYAQKFQGR VTMTRDTSISTAYMELNRLRSDDTAVYYCARAYY YGSGTYYNEFDYWGQGTLVTVSS |
| | | | SEQ ID NO: 27666 | SEQ ID NO: 31672 |
| iPS:437036 | 21-225_195H9 | NA | CAGTATGTGTTGACGCAGCCGCCCTCAGTGTC TGCGGCCCCAGGACAGAAGGTCACCATCTCCT GTTCTGGAGGCAGCTCCAACATTGGGAATAAT TATGTATCCTGGTTCCAGCAGTTCCCAGGAAC AGCCCCCAAATTCCTCATTTATGACAATAATA AGCGACCCTCAGGGATTCTTGACCGATTCTCT GGCTCCAAGTCTGGCACGTCAGCCACCCTGGG CATCACCGGACTCCAGACTGGGGACGAGGCC GATTATTACTGCGGAACATGGGATCGCACCAT GAGTGATTGGGTATTCGGCGGAGGGACCAAGT TGACCGTCCTA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTTACAGTGG TGGCACAAACTATGCACAGAAGTTTCAGGACAG GGTCACCATGACCAGGGACACGTCCATCACCAC AGCCTACATGGAACTGAGCAGGCTGAGATCTGA CGACACGGCCGTGTATTACTGTGCGAGAGATAG AGCAGTGGCTGGAAACTACTTCTACTACGGTATG GACGTCTGGGGCCAGGGGACCACGGTCACCGTC TCCTCA |
| | | | SEQ ID NO: 27667 | SEQ ID NO: 31673 |

FIGURE 50

| | | | | |
|---|---|---|---|---|
| | | AA | QYVLTQPPSVSAAPGQKVTISCSGGSSNIGNNYV SWFQQFPGTAPKFLIYDNNKRPSGILDRFSGSKS GTSATLGITGLQTGDEADYYCGTWDRTMSDWV FGGGTKLTVL | QVQLVQSGAEVKKPGASVKVSCKASGYTFTGYYM HWVRQAPGQGLEWMGWINPYSGGTNYAQKFQDR VTMTRDTSITTAYMELSRLRSDDTAVYYCARDRAV AGNYFYYGMDVWGQGTTVTVSS |
| | | | SEQ ID NO: 27668 | SEQ ID NO: 31674 |
| iPS:437040 | 21-225_196E7 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGCT TACTATCCAAACTGGTTGCAGCAGAAACCTGG ACAAGCACCCAGGACACTGATTTATAGTACAA ACAACAAACACTCCTGGACCCCTGCCCGGTTC TCAGGCTCCCTCCTTGGGGGCAAAGCTGCCCT GACACTGTCAGATGTGCAGCCTGAGGACGAG GCTGAGTATTACTGCCTGATTTTCTATGGTGGT GTACATGTGATATTTGGCGGAGGGACCAAGCT GACCGTCCTA | CAGGTGCAGCTGGTGCAGTCTGGGGCTGAGGTG AAGAAGCCTGGGGCCTCAGTGAAGGTCTCCTGTA AGGTTTCTGGATATACCTTCACCGGCTACAATAT GCACTGGGTGCGACAGGCCCCTGGACAAGGGCT TGAGTGGATGGGATGGATCAACCCTAATAGTGGT GGCACAAACTATGCACACAAGTTTCAGGGCAGG GTCACCATGACCAGGGACACGTCCATCAGCACA GCCTACATGGAACTGAGCAGGCTGAGATCCGAC GACACGGCCGTGTATTACTGTGCGAGAGATTACT ATGATACTAGTGGAGAAGGGTGGTTCGACCCCTG GGGCCAGGGAACCCTGGTCACCGTCTCCTCA |
| | | | SEQ ID NO: 27669 | SEQ ID NO: 31675 |
| | | AA | QTVVTQEPSLTVSPGGTVTLTCASSTGAVTSAYY PNWLQQKPGQAPRTLIYSTNNKHSWTPARFSGS LLGGKAALTLSDVQPEDEAEYYCLIFYGGVHVIF GGGTKLTVL | QVQLVQSGAEVKKPGASVKVSCKVSGYTFTGYNM HWVRQAPGQGLEWMGWINPNSGGTNYAHKFQGR VTMTRDTSISTAYMELSRLRSDDTAVYYCARDYYD TSGEGWFDPWGQGTLVTVSS |
| | | | SEQ ID NO: 27670 | SEQ ID NO: 31676 |

FIGURE 50

| iPS:437042 | 21-225_197E8 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAATAAA TATGTATCCTGGTACCAGCAGTTCCCAGGAAC AGCCCCCAAACTCCTCATTTATGACAATAATA AGCGACCCTCAAAGATTCCTGACCGATTCTCT GGCTCCAAATCTGGCACGTCAGCCACCCTGGG CATCACCGGACTCCTGACTGGGGACGAGGCCG ATTATTACTGCGGAATATGGGATCGCAGTCTG AGTGTTATGGTGTTCGGCGGAGGGACCAAGCT GACCGTCCTA<br><br>SEQ ID NO: 27671 | CAGGTGCAGCTGCTGCAGTCTGGGGCTGAGGTG AGGAAGCCTGGGGCCTCAGTGAGGGTCTCCTGC AAGGCTTCTGGATACACCTTCACCGGCTACTATA TACACTGGGTGCGACAGGCCCCTGGACAAGGGC TTGAGTGGATGGGATGGATCAACCCTAACAGTGG TGGCACAAACTATGCACAGAGGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGAGAT AGCAGTGGCTGGGAACTACTTCTACTACGGTATG GGCGTCTGGGGCCAAGGGACCACGGTCGCCGTC TCCTCA<br><br>SEQ ID NO: 31677 |
| | | AA | QSVLTQPPSVSAAPGQKVTISCSGSSSNIGNKYVS WYQQFPGTAPKLLIYDNNKRPSKIPDRFSGSKSG TSATLGITGLLTGDEADYYCGIWDRSLSVMVFG GGTKLTVL<br><br>SEQ ID NO: 27672 | QVQLLQSGAEVRKPGASVRVSCKASGYTFTGYYIH WVRQAPGQGLEWMGWINPNSGGTNYAQRFQGRV TMTRDTSISTAYMELSRLRSDDTAVYYCAREIAVA GNYFYYGMGVWGQGTTVAVSS<br><br>SEQ ID NO: 31678 |
| iPS:437044 | 21-225_197F9 | NA | CAGTCTGTGTTGACTCAGCCACCCTCAGCGTC TGGGACCCCCGGGCAGAGGGTCACCATCTCTT GTTCTGGAAGCAGCTCCAACATCGGAAGTAAT ACTGTAAACTGGTACCAGCAACTCCCAGGAAC GGCCCCCAAACTCCTCATTTATAGTAATAATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGTATG AATGGTCCGGTGTTCGGCGGAGGGACCAAGCT GACCGTCCTA<br><br>SEQ ID NO: 27673 | CAGGTGCAGCTGCAGGAGTCGGGGCCCAGGACTG GTGAAGCCTTCGGAGACCCTGTCCCTCACCTGCA CTGTCTCTGGTGGCTCCATCAGAATTTACTACTG GAGCTGGATCCGGCAGCCCCCAGGGAAGGGACT GGAGTGGATTGGGTATGTCTATTACAGTGGGAGC ACCACCTACAACCCCTCCCTCAAGAGTCGAGTCA CCATATCAGTAGACACGTCCAAGAACCAGTTCTC CCTGAAGCTGAACTCTGTGACCGCTGCGGACACG GCCGTGTATTACTGTGTGAGAGAAAGGGGGAGT AGCCACAGATGGGGGGGACTACTACGGAATGGAC GTCTGGGGCCGAGGGACCACGGTCACCGTCTCCT CA<br><br>SEQ ID NO: 31679 |

FIGURE 50

| | | | | |
|---|---|---|---|---|
| | | AA | QSVLTQPPSASGTPGQRVTISCSGSSSNIGSNTVN WYQQLPGTAPKLLIYSNNQRPSGVPDRFSGSKS GTSASLAISGLQSEDEADYYCAAWDDSMNGPVF GGGTKLTVL | QVQLQESGPGLVKPSETLSLTCTVSGGSIRIYYWSW IRQPPGKGLEWIGYVYYSGSTTYNPSLKSRVTISVD TSKNQFSLKLNSVTAADTAVYYCVRERGSSHRWG DYYGMDVWGRGTTVTVSS |
| | | | SEQ ID NO: 27674 | SEQ ID NO: 31680 |
| iPS:437048 | 21-225_197B11 | NA | CAGACTGTGGTGACTCAGGAGCCCTCACTGAC TGTGTCCCCAGGAGGGACAGTCACTCTCACCT GTGCTTTCAGCACTGGATCAGTCACCAGTGGT TCCTATCCAAACTGGTTCCAGCAGAAACCTGG ACAAGCACCCAGGGCACTGATTTATAGTACAA ACAACAAACACTCCTGGACCCCTGCCCGGTTC TCAGGCTCCCTCCTTGGGGGCAAAGCTGCCCT GACACTGTCAGGTGTGCAGCCTGAGGACGAG GCTGAGTATTACTGCCTGCTCTACTATGGTGGT GCTCAGCTGGTGTTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCCCCCAGGGAA GGGCCTGGAGTGGATTGGGTACATCTATTACACT GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGACGATC CCCTCTACGGAATGGACGTCTGGGGCCAAGGGA CCACGGTCACCGTCTCCTCA |
| | | | SEQ ID NO: 27675 | SEQ ID NO: 31681 |
| | | AA | QTVVTQEPSLTVSPGGTVTLTCAFSTGSVTSGSY PNWFQQKPGQAPRALIYSTNNKHSWTPARFSGS LLGGKAALTLSGVQPEDEAEYYCLLYYGGAQL VFGGGTKLTVL | QVQLQESGPGLVKPSQTLSLTCTVSGGSISSGGYYW SWIRQPPGKGLEWIGYIYYTGSTYYNPSLKSRVTIS VDTSKNQFSLKLSSVTAADTAVYYCARDDPLYGM DVWGQGTTVTVSS |
| | | | SEQ ID NO: 27676 | SEQ ID NO: 31682 |

FIGURE 50

| iPS:437050 | 21-225_197C11 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGCT TACTATCCAAACTGGTTGCAGCAGAAACCTGG ACAAGCACCCAGGACACTGATTTATAGTACAA GCAACAAACACTCCTGGACCCCTGCCCGGTTC TCAGGCTCCCTCCTTGGGGGCAAAGCTGCCCT GACACTGTCAGATGTGCAGCCTGAGGACGAG GCTGAGTATTACTGCCTGATCTTCTATGGTGGT GTACATGTGATATTTGGCGGAGGGACCAAGCT GACCGTCCTA | CAGGTGCAGCTGGTGCAGTCTGGGGCTGAGGTG AAGAAGCCTGGGGCCTCAGTGAAGGTCTCCTGTA AGGTTTCTGGATATACCTTCACCGGCTACAATAT GCACTGGGTGCGACAGGCCCCTGGACAAGGGCT TGAGTGGATGGGATGGATCAACCCTAATAGTGGT GGCACAAACTATGCACACAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGAGAGATTACT ATGATAGTAGTGGAGAAGGGTGGTTCGACCCCTG GGGCCAGGGAACCCTGGTCACCGTCTCCTCA |
|  |  |  | SEQ ID NO: 27677 | SEQ ID NO: 31683 |
|  |  | AA | QTVVTQEPSLTVSPGGTVTLTCASSTGAVTSAYY PNWLQQKPGQAPRTLIYSTSNKHSWTPARFSGS LLGGKAALTLSDVQPEDEAEYYCLIFYGGVHVIF GGGTKLTVL | QVQLVQSGAEVKKPGASVKVSCKVSGYTFTGYNM HWVRQAPGQGLEWMGWINPNSGGTNYAHKFQGR VTMTRDTSISTAYMELSRLRSDDTAVYYCARDYYD SSGEGWFDPWGQGTLVTVSS |
|  |  |  | SEQ ID NO: 27678 | SEQ ID NO: 31684 |
| iPS:437054 | 21-225_194G3 | NA | CAGTCTGTGTTGACGCAGCCGCCCTCAGTGTC TGCGGCCCCAGGACAGAAGGTCACCATCTCCT GCTCTGGAAGCAGCTCCAACATCGGGAATAAT TATATATCCTGGTACCAGCAGCTCCCAGGAAC AGCCCCCAAACTCCTCATTTATGACAATAAAA AGCGACCCTCAGGGATTCCTGACCGATTCTCT GGCTCCAAGTCTGGCACGTCAGCCACCCTGGG CATCACCGGACTCCAGACTGGGGACGAGGCC GATTATTACTGCGGAACATGGGATAGCAGCCT GAGTGTTTGTGTCTTCGGAACTGGGACCAAGG TCACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTTAGTTTCCATGGCAT GCACTGGGTCCGCCAGGCTCCAGGCAAGGGGCT GGAGTGGGTGGCAGTTATATGGTATGATGGAAGT AAAAAATACTATGCAGACTCCGTGAAGGGCCGA TTCACCATCTCCAGAGACAATTCCAAGAACACGC TGCATCTGCAAATGAACAGTCTGAGAGCCGAGG ACACGGCTGTGTATTACTGTGCGAAAGAAGGGTT TAGCAGTGGCTTTTACAGGGGGGTTTGACAACTGG GGCCAGGGAACCCTGGTCACCGTCTCCTCA |
|  |  |  | SEQ ID NO: 27679 | SEQ ID NO: 31685 |

FIGURE 50

| | | | | |
|---|---|---|---|---|
| | | AA | QSVLTQPPSVSAAPGQKVTISCSGSSSNIGNNYIS WYQQLPGTAPKLLIYDNKKRPSGIPDRFSGSKSG TSATLGITGLQTGDEADYYCGTWDSSLSVCVFG TGTKVTVL | QVQLVESGGGVVQPGRSLRLSCAASGFTFSFHGMH WVRQAPGKGLEWVAVIWYDGSKKYYADSVKGRF TISRDNSKNTLHLQMNSLRAEDTAVYYCAKEGFSS GFYRGFDNWGQGTLVTVSS |
| | | | SEQ ID NO: 27680 | SEQ ID NO: 31686 |
| iPS:437056 | 21-225_198B8 | NA | CAGACCGTGGTGACTCAGGAGTCCTCACTGAC TGTGTCCCCAGGAGGGACAGTCACTCTCACTT GTGTTCTTAGCACTGGAGCAGTCACCAGTGGT TCCTTTCCAAACTGGTTCCAGCAGAAACCTGG ACAAGCACCCAGGGCTCTGATTTATAGTACAA GCAACAAACATTCCTGGACCCCTGCCCGGTTT TCAGGCTCCCTCCTTGGGGGCAAAGCTGCCCT GACATTGTCAGGTGTGCAGCCTGAGGACGAGG CTGATTATTACTTCATGCTTTACAGTGGTGGAG CTCAGATGGTGTTCGGCGGAGGGACCAAGCTG ACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGAAGTGGTGGTGA CTACTGGAGCTGGATCCGCCAGCACCCAGGGGA GGGCCTGGAGTGGATTGGGTACATCTATTACAGT GGGATCACCTACTACAATCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGATAGC AGCTCGCGGGGTATGGACGTCTGGGGCCAAGGG ACCACGGTCACCGTCTCCTCA |
| | | | SEQ ID NO: 27681 | SEQ ID NO: 31687 |
| | | AA | QTVVTQESSLTVSPGGTVTLTCVLSTGAVTSGSF PNWFQQKPGQAPRALIYSTSNKHSWTPARFSGS LLGGKAALTLSGVQPEDEADYYFMLYSGGAQM VFGGGTKLTVL | QVQLQESGPGLVKPSQTLSLTCTVSGGSIRSGGDY WSWIRQHPGEGLEWIGYIYYSGITYYNPSLKSRVTI SVDTSKNQFSLKLSSVTAADTAVYYCARDSSSRGM DVWGQGTTVTVSS |
| | | | SEQ ID NO: 27682 | SEQ ID NO: 31688 |

FIGURE 50

| | | | | |
|---|---|---|---|---|
| iPS:437058 | 21-225_199F3 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAATAAT TATGTATCCTGGTACCAGCAACTCCCAGGAAC AGCCCCCAAACTCCTCATTTATGACAATAATA AGCGCCCCTCAGGGATTCCTGACCGATTCTCT GGCTCCAAGTCTGGCACGTCAGCCACCCTGGG CATCACCGGCCTCCAGACTGGGGACGAGGCCG ATTATTACTGCGGAACATGGGATAGCAGCCTG AGTGCTTGTGTCTTCGGAACTGGGACCAAGGT CACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTTTCTATGGCAT GCACTGGGTCCGCCAGGCTCCAGGCAAGGGGCT GGAGTGGGTGGCAGTTATTTGGTATGATGGAAGT AGTAAATACTATGCAGACTCCGTGAAGGGCCGA TTCACCGTCTCCAGAGACAATTCCAAGAACACGC TGTATCTGCAAATGAACAGCCTGAGAGCCGAGG ACACGGCTGTGTATTATTGTGCGAAAGAAGGGTA TAGCAGTGGCTTTTACAGGGGATTTGCCAACTGG GGCCAGGGAACCCTGGTCACCGTCTCCTCA |
| | | | SEQ ID NO: 27683 | SEQ ID NO: 31689 |
| | | AA | QSVLTQPPSVSAAPGQKVTISCSGSSSNIGNNYVS WYQQLPGTAPKLLIYDNNKRPSGIPDRFSGSKSG TSATLGITGLQTGDEADYYCGTWDSSLSACVFG TGTKVTVL | QVQLVESGGGVVQPGRSLRLSCAASGFTFSFYGMH WVRQAPGKGLEWVAVIWYDGSSKYYADSVKGRF TVSRDNSKNTLYLQMNSLRAEDTAVYYCAKEGYS SGFYRGFANWGQGTLVTVSS |
| | | | SEQ ID NO: 27684 | SEQ ID NO: 31690 |
| iPS:437060 | 21-225_199C3 | NA | CAGTCTGTGTTGACTCAGCCACCCTCAGCGTC TGGGACCCCCGGGCAGAGGGTCACCATCTCTT GTTCTGGAAGCAGCTCCAACATCGGAAGTAAT ACTGTAAACTGGTACCAGCAGCTCCCAGGAAC GGCCCCCAAACTCCTCATTTATAGTAATAATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AATGGTCCGGTGTTCGGCGGAGGGACCAAGCT GACCGTCCTA | CAGGTGCAGCTGCAGGAGTCGGGCCCAGGACTG GTGAAGCCTTCGGAGACCCTGTCCCTCACCTGCA CTGTCTCTGGTGGCTCCATCAGAATTTACTACTG GAGCTGGATCCGGCAGACCCCAGGGAAGGGACT GGAGTGGATTGGATATATATCTATTACAGTGGGAGC ACCACCTACAACCCCTCCCTCAAGAGTCGAGTCA CCATATCAGTAGACACGTCCAAGAACCAGTTCTC CCTGAAGCTGAACTCTGTGACCGCTGCGGACACG GCCGTGTATTACTGTGTGAGAGAAAGGGGGAGT AGCCACAGATGGGGGGACTACTACGGAATGGAC GTCTGGGGCCGAGGGACCACGGTCACCGTCTCCT CA |
| | | | SEQ ID NO: 27685 | SEQ ID NO: 31691 |

FIGURE 50

| | | | | |
|---|---|---|---|---|
| | | AA | QSVLTQPPSASGTPGQRVTISCSGSSSNIGSNTVN WYQQLPGTAPKLLIYSNNQRPSGVPDRFSGSKS GTSASLAISGLQSEDEADYYCAAWDDSLNGPVF GGGTKLTVL | QVQLQESGPGLVKPSETLSLTCTVSGGSIRIYYWSW IRQTPGKGLEWIGYIYYSGSTTYNPSLKSRVTISVDT SKNQFSLKLNSVTAADTAVYYCVRERGSSHRWGD YYGMDVWGRGTTVTVSS |
| | | | SEQ ID NO: 27686 | SEQ ID NO: 31692 |
| iPS:437062 | 21-225_200H1 | NA | CAGACTGTGGTGACTCAGGAGCCCTCACTGAC TGTGTCCCCAGGAGGGACAGTCACTCTCACCT GTGCTTCCAACACTGGAGCAGTCACCAGTGGT TCCTATCCAAACTGGTTCCAGCAGAAACCTGG ACAGGCACCCAGGGCACTGATTTATCATACAA ACAACAAACACTCCTGGACCCCTGCCCGGTTC TCAGGCTCCCTCCTTGGGGGCAAAGCTGCCCT GACACTGTCAGGTGTGCAGCCTGAGGACGAG GCTGAATATTACTGTCTGATCTACTATGGTGGT GCTCAGCTGGTGTTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTGA CTATTGGAGCTGGATCCGCCAGCACCCAGGGAA GGGCCTGGAGTGGATTGGGTACATCTATTATAGT GGGAGCACCTACTACAACCCGTCCCTCAAGAGTC GAGTTACCATATCAGTAGACACGTCTAAGAACCA GTTCTCCCTGAAGCTGAACTCTGTGACTGCCGCG GACACGGCCGTGTATTACTGTGCGAGAGATGGA GCAGCTCTGGGTATGGACGTCTGGGGCCAAGGG ACCACGGTCACCGTCTCCTCA |
| | | | SEQ ID NO: 27687 | SEQ ID NO: 31693 |
| | | AA | QTVVTQEPSLTVSPGGTVTLTCASNTGAVTSGSY PNWFQQKPGQAPRALIYHTNNKHSWTPARFSGS LLGGKAALTLSGVQPEDEAEYYCLIYYGGAQLV FGGGTKLTVL | QVQLQESGPGLVKPSQTLSLTCTVSGGSISSGGDYW SWIRQHPGKGLEWIGYIYYSGSTYYNPSLKSRVTIS VDTSKNQFSLKLNSVTAADTAVYYCARDGAALGM DVWGQGTTVTVSS |
| | | | SEQ ID NO: 27688 | SEQ ID NO: 31694 |

FIGURE 50

| iPS:437064 | 21-225_200G8 | NA | CAGTCTGTGTTGACGCAGCCGCCCTCAGTGTC TGCGGCCCCAGGACAGAGGGTCACCATCTCCT GCTCTGGAAGCAGCTCCAACCTTGGAAATAAT TTTGTATCCTGGTACCAGCAGTTCCCAGGAAC AGCCCCCAAACTCCTCATTTATGACAATTATA AGCGACCCTCAGGGATTCCTGACCGATTCTCT GTCTCCAAGTCTGGCACGTCAGCCACCCTGGG CATCACCGGACTCCAGACTGGGGACGAGGCC GATTATTACTGCGGAACTTGGGATAGCAGCCT GAATACTGGGGTATTCGGCGGAGGGACCAAG CTGACCGTCCTA | CAGGTGCAGCTGCAGGAGTCGGGCCCAGGACTG GTGAAGCCTTCGGAGACCCTGTCCCTCACCTGCA CTGTCTCTGGTGGCTCCATCAGAAGTTACTACTG GAGCTGGATCCGGCAGCCCCCAGGGAAGGGACT GGAGTGGATTGGGTATATCTATTACAGTGGGAGT ACTAAGTACAACCCCTCCCTCAAGAGTCGAGTCA CCATATCAGTAGACACGTCCAAGAACCAGTTCTC CCTGAAACTGAGCTCTGTGACCGCTGCGGACACG GCCGTGTACTACTGTGCGAGAAAGGGAGTGGGA ACCATCCACTTTGACTACTGGGGCCAGGGAACCC TGGTCACCGTCTCCTCA |
|---|---|---|---|---|
| | | | SEQ ID NO: 27689 | SEQ ID NO: 31695 |
| | | AA | QSVLTQPPSVSAAPGQRVTISCSGSSSNLGNNFV SWYQQFPGTAPKLLIYDNYKRPSGIPDRFSVSKS GTSATLGITGLQTGDEADYYCGTWDSSLNTGVF GGGTKLTVL | QVQLQESGPGLVKPSETLSLTCTVSGGSIRSYYWS WIRQPPGKGLEWIGYIYYSGSTKYNPSLKSRVTISV DTSKNQFSLKLSSVTAADTAVYYCARKGVGTIHFD YWGQGTLVTVSS |
| | | | SEQ ID NO: 27690 | SEQ ID NO: 31696 |
| iPS:437066 | 21-225_200G9 | NA | CAGACTGTGGTGACTCAGGAGCCCTCACTGAC TGTGTCCCCAGGAGGGACAGTCACTCTCACCT GTGCTTCCAACACTGGAGCAGTCACCAGTGGT TCCTATCCAAATTGGTTACAGCAGAAACCTGG ACAAGCACCCAGGGCACTGATTTATCATACAG ACAACAAACACTCCTGGACCCCTGCCCGGTTC TCAGGCTCCCTCCTTGGGGGGCAAAGCTGCCCT GACACTGTCAGGTGCGCAGCCTGAGGACGAG GCTGAATATTACTGTCTGATCTACTATGGTGGT GCTCAGCTGGTGTTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTGA CTACTGGAGCTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGTAGACACGTCTAAGAACCA GTTCTCCCTGAAGCTGAACTCTGTGACTGCCGCG GACACGGCCGTGTATTACTGTGCGAGAGATGGA GCAGCTCTGGGTATGGACGTCTGGGGGCCAAGGG ACCACGGTCACCGTCTCCTCA |
| | | | SEQ ID NO: 27691 | SEQ ID NO: 31697 |

FIGURE 50

| | | | | |
|---|---|---|---|---|
| | | AA | QTVVTQEPSLTVSPGGTVTLTCASNTGAVTSGSY PNWLQQKPGQAPRALIYHTDNKHSWTPARFSGS LLGGKAALTLSGAQPEDEAEYYCLIYYGGAQLV FGGGTKLTVL | QVQLQESGPGLVKPSQTLSLTCTVSGGSISSGGDYW SWIRQHPGKGLEWIGYIYYSGSTYYNPSLKSRVTIS VDTSKNQFSLKLNSVTAADTAVYYCARDGAALGM DVWGQGTTVTVSS |
| | | | SEQ ID NO: 27692 | SEQ ID NO: 31698 |
| iPS:437068 | 21-225_200A11 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT TACTATCCAAACTGGTTCCAGCAGAAACCTGG ACAAGTACCCAGGGCACTGATTTATAGTACAA ACAACAAACACTCCTGGACCCCTGCCCGGTTC TCAGGCTCCCTCCTTGGGGGCAAAGCTGCCCT GACACTGTCAGGTGTGCAGCCTGAGGACGAG GCTGACTATTACTGCTGCTCTATTATGGTGGT GCTCACCTGGCATTCGGCGGAGGGACCAAGCT GACCGTCCTG | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTGA CTACTGGAGCTGGATCCGCCAGCACCCAGGGAA GGGCCTGGAGTGGATTGGGTACATCTATTACAGA GGGAGCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTACTGTGCGAGAGATGCA GCAGCCCACGGCATGGACGTCTGGGGCCAAGGG ACCACGGTCACCGTCTCCTCA |
| | | | SEQ ID NO: 27693 | SEQ ID NO: 31699 |
| | | AA | QTVVTQEPSLTVSPGGTVTLTCASSTGAVTSGYY PNWFQQKPGQVPRALIYSTNNKHSWTPARFSGS LLGGKAALTLSGVQPEDEADYYCLLYYGGAHL AFGGGTKLTVL | QVQLQESGPGLVKPSQTLSLTCTVSGGSISSGGDYW SWIRQHPGKGLEWIGYIYYRGSTYYNPSLKSRVTIS VDTSKNQFSLKLSSVTAADTAVYYCARDAAAHGM DVWGQGTTVTVSS |
| | | | SEQ ID NO: 27694 | SEQ ID NO: 31700 |

FIGURE 50

| iPS:437070 | 21-225_201G11 | NA | TCCTATGAGCTGACTCAGCCACCCTCAGTGTC CGTGTCCCCAGGACAGACAGCCAGCATCACCT GTTCTGGAGATAAATTGGGGGGATAGATTTGCT TGCTGGTATCAACAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAGCTGACCGTCCTA | CAGGTACAGCTGCAGCAGTCAGGTCCAGGACTG GTGAAGCCCTCGCAGACCCTCTCACTCACCTGTG CCATCTCCGGGGACAGTGTCTCTCGCATCAATCC TACTTGGAACTGGATCAGGCAGTCCCCATCGAGA GGGCCTTGAGTGGCTGGGAAGGACATACTACAGG TCCAAGTGGTATCATGTTTATGCAGTATCTGTGA AAAGTCGAATAACCATCAACCCAGACACATCCA AGAACCAGTTCTCCCTGCAGCTGAATTCTGTGAC TCCCGAGGACACGGCAGTGTATTACTGTGCAAGA GATCCTGGGGGGCTCTTTGACTACTGGGGCCAGG GAACCCTGGTCACCGTCTCCTCA |
|---|---|---|---|---|
| | | | SEQ ID NO: 27695 | SEQ ID NO: 31701 |
| | | AA | SYELTQPPSVSVSPGQTASITCSGDKLGDRFACW YQQKPGQSPVLVIYQDSKRPSGIPERFSGSNSGN TATLTISGTQAMDEADYYCQAWDSSTVVFGGG TKLTVL | QVQLQQSGPGLVKPSQTLSLTCAISGDSVSRINPTW NWIRQSPSRGLEWLGRTYYRSKWYHVYAVSVKSR ITINPDTSKNQFSLQLNSVTPEDTAVYYCARDPGGL FDYWGQGTLVTVSS |
| | | | SEQ ID NO: 27696 | SEQ ID NO: 31702 |
| iPS:437074 | 21-225_203B2 | NA | TCCTATGAGCTGACTCAGCCACTCTCAGTGTC AGTGGCCCTGGGACAGACGGCCAGGATTACCT GTGGGGGAAACAACATTGGAAGAAAAAAATGT GCACTGGTACCAGCAGAAGCCAGGCCAGTCCC CTGTGTTGATCATCCATAGGGATAGCGACCGG CCCTCTGGGATCCCTGAGCGATTCTCTGGCTCC AACTCGGGGAACACGGCCACCCTGACCATCAG CAGAGCCCAAGCCGGGGATGAGGCTGACTATT ACTGTCAGGTGTGGGACAGCGGCACTGCGGTA TTCGGCGGAGGGACCAAGCTGCCCGTCCTA | CAGGTGCAGCTGGTGGAGTCTGGGGGAGGCGTG GTCCAGCCGGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTTTGATGGAAGT AATGAATACTATGCAGACTCCGTGAAGGGCCGA TTCACCATCTCCAGAGACAATTCCATGAGCACGC TGTATCTGCAAATGAACAGCCTGAGAGCCGAGG ACACGGCTGTGTATTACTGTGCGAGAGAAAGTGG GAGCTATGCTCTTTATATCTGGGGCCAAGGGACA ATGGTCACCGTCTCTTCA |
| | | | SEQ ID NO: 27697 | SEQ ID NO: 31703 |

FIGURE 50

| | | | | |
|---|---|---|---|---|
| | | AA | SYELTQPLSVSVALGQTARITCGGNNIGRKNVH WYQQKPGQSPVLIIHRDSDRPSGIPERFSGSNSGN TATLTISRAQAGDEADYYCQVWDSGTAVFGGG TKLPVL | QVQLVESGGGVVQPGRSLRLSCAASGFTFSNYGM HWVRQAPGKGLEWVAIIWFDGSNEYYADSVKGRF TISRDNSMSTLYLQMNSLRAEDTAVYYCARESGSY ALYIWGQGTMVTVSS |
| | | | SEQ ID NO: 27698 | SEQ ID NO: 31704 |
| iPS:437076 | 21-225_203G6 | NA | TCCTATGAACTGACTCAGCCACCCTCAGTGTC CGTGTCCCCAGGACAGACAGCCAGCATCACCT GTTCTGGAGATAAAATTGGGGGATAGATTTGCT TGCTGGTATCAACAGAAGCCAGGCCAGTCCCC TGTACTGGTCATCTATCAAGATAACAAGCGGC CCTCAGGGATCCCTGAGCGATTCTCTGGCTCC AACTCTGGGAACACAGCCACTCTGACCATCAG CGGGACCCAGTCTATGGATGAGGCTGACTATT ACTGTCAGGCGTGGGACAGCAGCACTGTGGTA TTCGGCGGAGGGACCAAGCTGACCGTCCTG | CAGGTTCAGCTGCAGCAGTCAGGTCCAGGACTG GTGAAGCCCTCGCAGACCCTCTCACTCACCTGTG CCATCTCCGGGGACAGTGTCTCTCGCACCAATCC TACTTGGAACTGGATCAGGCAGTCCCCATCGAGA GGCCTTGAGTGGCTGGGAAGGACATACTACAGG TCCAAGTGGTATCATGTTTATGCACTATCTGTGA AAAGTCGAATAACCATCACCCCAGACACATCCA AGAACCAGTTCTCCCTGCAGCTGAACTCTGTGAC TCCCGAGGACACGGCTGTGTATTACTGTGCAAGA GATCCTGGGGGCCTCTTTGACTACTGGGGCCAGG GAACCCTGGTCACCGTCTCCTCA |
| | | | SEQ ID NO: 27699 | SEQ ID NO: 31705 |
| | | AA | SYELTQPPSVSVSPGQTASITCSGDKLGDRFACW YQQKPGQSPVLVIYQDNKRPSGIPERFSGSNSGN TATLTISGTQSMDEADYYCQAWDSSTVVFGGGT KLTVL | QVQLQQSGPGLVKPSQTLSLTCAISGDSVSRTNPTW NWIRQSPSRGLEWLGRTYYRSKWYHVYALSVKSRI TITPDTSKNQFSLQLNSVTPEDTAVYYCARDPGGLF DYWGQGTLVTVSS |
| | | | SEQ ID NO: 27700 | SEQ ID NO: 31706 |

FIGURE 50

| | | | | |
|---|---|---|---|---|
| iPS:437082 | 21-225_205E12 | NA | TCCTATGAGCTGACTCAGCCACTCTCAGTGTC AGCGGCCCTGGGACAGACGGCCAGGATTACCT GTGGGGGAAACAACATTGGAAGAAAAAATGT GCACTGGTACCAGCAGAAGCCAGGCCAGTCCC CTGTGTTGATCATCCATAGGGATAGCGACCGG CCCTCTGGGATCCCTGAGCGATTCTCTGGCTCC AACTCGGGGAACACGGCCACCCTGACCATCAG CAGAGCCCAAGCCGGGGATGAGGCTGACTATT ACTGTCAGGTGTGGGACAGCGGCACTGCGGTA TTCGGCGGAGGGACCAAGCTGCCCGTCCTA | CAGGTGCAGCTGGTGGAGTCTGGGGGAGGCGTG GTCCAGCCGGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTTTGATGGAAGT AATGAATACTATGCAGACTCCGTGAAGGGCCGA TTCACCATCTCCAGAGACAATTCCATGAGCACGC TGTATCTGCAAATGAACAGCCTGAGAGCCGAGG ACACGGCTGTGTATTACTGTGCGAGAGAAAGTGG GAGCTATGCTCTTTATATCTGGGGCCAAGGGACA ATGGTCACCGTCTCTTCA |
| | | | SEQ ID NO: 27701 | SEQ ID NO: 31707 |
| | | AA | SYELTQPLSVSAALGQTARITCGGNNIGRKNVH WYQQKPGQSPVLIIHRDSDRPSGIPERFSGSNSGN TATLTISRAQAGDEADYYCQVWDSGTAVFGGG TKLPVL | QVQLVESGGGVVQPGRSLRLSCAASGFTFSNYGM HWVRQAPGKGLEWVAIIWFDGSNEYYADSVKGRF TISRDNSMSTLYLQMNSLRAEDTAVYYCARESGSY ALYIWGQGTMVTVSS |
| | | | SEQ ID NO: 27702 | SEQ ID NO: 31708 |
| iPS:437084 | 21-225_206B5 | NA | TCCTATGAATTGACTCAGCCACTCTCAGTGTC AGTGGCCCTGGGACAGACGGCCAGGATTGCCT GTGGGGGGAAACAACATTGGAAGAAAAAATGT GCACTGGTACCAGCAGAAGCCAGGCCTGGCCC CTGTGCCGGTCATCCNTAGGGATAGCTACCGA TCTTCTGGGATCCCTGACAGATTCTCTGGCTCC AACTGCGGGAACACGACCACCGTGACCATCA GCAGAGCCCAAGCCGGGGAGGAGGCAGAGTA TTATTGTCAGGATTGGGACAGCAGCACTGTGG TGTTCGGCGGAGGGACCAAACTGACCGTCCTA | CAGGTGCAGCTGGTGGAGTCCGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATAACTGTGCGAGAGAGGGTG GGAGCTACCACCTTGACTACTGGGGCCAGGGAA TCCTGGTCACCGTCTCCTCA |
| | | | SEQ ID NO: 27703 | SEQ ID NO: 31709 |

FIGURE 50

| | | | | |
|---|---|---|---|---|
| | | AA | SYELTQPLSVSVALGQTARIACGGNNIGRKNVH WYQQKPGLAPVPVIXRDSYRSSGIPDRFSGSNCG NTTTVTISRAQAGEEAEYYCQDWDSSTVVFGGG TKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYNCAREGGSY HLDYWGQGILVTVSS |
| | | | SEQ ID NO: 27704 | SEQ ID NO: 31710 |
| IPS:437086 | 21-225_209A8 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAGTAAT TTTTTATCCTGGTACCAGCAGCTCCCAGGAAC AGCCCCCAAACTCCTCATTTATGACAATAATA AGCGACCCTCAGGGATTCCTGACCGATTCTCT GGCTCCAAGTCTGGCACGTCAGCCACCCTGGG CATCACCGGACTCCAGACTGGGGACGAGGCC GATTATTACTGCGGAACATGGGATAGCAGCCT GAGTGCTGGGGTATTCGGCGGAGGGACCAAG CTGACCGTCCTA | GAGGTGCAGCTGGTGGAGTCTGGGGGAGGCTTG GTACAGCCTGGGGGGTCCCTGAGACTCTCCTGTG CAGCCTCTGGATTCACTTTTAGAAGTTATAGCAT GAACTGGGTCCGCCAGGCTCCAGGGAAGGGGCT GGAGTGGGTTTTATACATTAGTAGTAGTAGTAGT ATCAAAAAGTACGCAGACTCTGTGAAGGGCCGA TTCACCATCTCCAGAGACAATGCCAAGAACTCAC TGTATCTGCAAATGAACAGCCTGAGAGACGAGG ACACGGCTGTGTATTACTGTGTGAGAGATGATGG GAGCTACTACTTTGACTACTGGGGCCAGGGAACC CTGGTCACCGTCTCCTCA |
| | | | SEQ ID NO: 27705 | SEQ ID NO: 31711 |
| | | AA | QSVLTQPPSVSAAPGQKVTISCSGSSSNIGSNFLS WYQQLPGTAPKLLIYDNNKRPSGIPDRFSGSKSG TSATLGITGLQTGDEADYYCGTWDSSLSAGVFG GGTKLTVL | EVQLVESGGGLVQPGGSLRLSCAASGFTFRSYSMN WVRQAPGKGLEWVLYISSSSSIKKYADSVKGRFTIS RDNAKNSLYLQMNSLRDEDTAVYYCVRDDGSYYF DYWGQGTLVTVSS |
| | | | SEQ ID NO: 27706 | SEQ ID NO: 31712 |

FIGURE 50

| | | | | |
|---|---|---|---|---|
| iPS:437088 | 21-225_209H10 | NA | TCCTATGAATTGACTCAGCCACTCTCAGTGTC AGTGGCCCTGGGACAGACGGCCAGGATTGCCT GTGGGGGAAACAACATTGGAAGAAAAAATGT GCACTGGTACCAGCAGAAGCCAGGCCTGGCCC CTGTGCCGGTCATCCTTAGGGATAGCTACCGG TCTTCTGGGATCCCTGACAGATTCTCTGGCTCC AACTGGGGGAACACGGCCACCGTGACCATCA GCAGAGCCCAAGCCGGGGAGGAGGCAGAGTA TTATTGTCAGGATTGGGACAGCAGCACTGTGG TGTTCGGCGGAGGGACCAAACTGACCGTCCTA | CAGGTGCAGCTGGTGGAGTCCGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATAACTGTGCGAGAGAGGGTG GGAGCTACCACCTTGACTACTGGGGCCAGGGAA TCCTGGTCACCGTCTCCTCA |
| | | | SEQ ID NO: 27707 | SEQ ID NO: 31713 |
| | | AA | SYELTQPLSVSVALGQTARIACGGNNIGRKNVH WYQQKPGLAPVPVILRDSYRSSGIPDRFSGSNW GNTATVTISRAQAGEEAEYYCQDWDSSTVVFGG GTKLTVL | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYNCAREGGSY HLDYWGQGILVTVSS |
| | | | SEQ ID NO: 27708 | SEQ ID NO: 31714 |
| iPS:437090 | 21-225_210F11 | NA | CAGACTGTGGTGACTCAGGAGCCCTCACTGAC TGTGTCCCCAGGAGGGACAGTCACTCTCACCT GTGCTTTCAGCACTGGAGCAGTCACCAGTGGT AATTATCCAAACTGGTTCCAGCAGAAACCTGG ACAAGCACCCAGGGCACTGATTTACAGTACAA GCAACAAACACTCCTGGACCCCTGCCCGGTTC TCAGGCTCCCTCCTTGGGGGCAAAGCTGCCCT GACACTGTCAGGTGTGCAGCCTGAGGACGAG GCTGAATATTACTGCCTGCTCTACTATGGTGGT GCTCAGCTGGTGTTCGGCGGAGGGACCAAGCT GACCGTCCTA | CAGGTGCAGCTGCAGGAGTCGGGCCCAGGACTG GTGAAGCCGTCACAGACCCTGTCCCTCACCTGCA CTGTCTCTGGTGGCTCCATCAGCAGTGGTGGTTC CTACTGGAGCTGGATCCGCCAGCACCCAGGGAA GGGCCTGGAGTGGATTGGGTACATCTATTACATT GGGACCACCTACTACAACCCGTCCCTCAAGAGTC GAGTTACCATATCAGTAGACACGTCTACGAACCA CTTCTCCCTGAAACTGAGCTCTGTGACTGCCGCG GACACGGCCGTGTATTACTGTGCGAGAGATGAG CCATTGACCGGTATGGACGTCTGGGGCCAAGGG ACCACGGTCACCGTCTCCTCA |
| | | | SEQ ID NO: 27709 | SEQ ID NO: 31715 |

FIGURE 50

| | | | | |
|---|---|---|---|---|
| | | AA | QTVVTQEPSLTVSPGGTVTLTCAFSTGAVTSGNY PNWFQQKPGQAPRALIYSTSNKHSWTPARFSGS LLGGKAALTLSGVQPEDEAEYYCLLYYGGAQL VFGGGTKLTVL | QVQLQESGPGLVKPSQTLSLTCTVSGGSISSGGSYW SWIRQHPGKGLEWIGYIYYIGTTYYNPSLKSRVTIS VDTSTNHFSLKLSSVTAADTAVYYCARDEPLTGMD VWGQGTTVTVSS |
| | | | SEQ ID NO: 27710 | SEQ ID NO: 31716 |
| IPS:437092 | 21-225_210B12 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAACACCCAGGC AAAGCCCCCAAATTCATGATTTATGAGGTCAG GAATCGGCCCTCAGGGGTTTCTAATCGCTTCT CTGGCTCCAAGTCTGGCAACACGGCCTCCCTG ACCATCTCTGGGCTCCAGGCTGAGGACGAGGC TGATTATTACTGCAGCTCATATACCAGCAGCC GCACTCTGGTATTCGGCGGAGGGACCAAGTTG ACCGTCCTA | CAGGTGCAGCTGGTGCAGTCTGGGGGCTGAGGTG AAGAAGCCTGGGGCCTCAGTGAAGATCTCCTGC AAGGCTTCTGGATTCACCTTCACCGACTACTATA TGAACTGGGTGCGACAGGCCCCTGGACAAGGGC TTGAGTGGATGGGATGGATCAACCCTAACAGTGG TGGCACAAACTATGCACAGAAGTTTCAGGGCAG GGTCACCATGACCAGGGACACGTCCATCAGCAC AGCCTACATGGAGCTGAGTAGGCTGAGATCTGA CGACACGGCCGTGTATTACTGTGCGAGAGGGTAT GACTCGTTCGCCCCCTGGGGCCAGGGAACCCTGG TCACCGTCTCCTCA |
| | | | SEQ ID NO: 27711 | SEQ ID NO: 31717 |
| | | AA | QSALTQPASVSGSPGQSITISCTGTSSDVGGYNY VSWYQQHPGKAPKFMIYEVRNRPSGVSNRFSGS KSGNTASLTISGLQAEDEADYYCSSYTSSRTLVF GGGTKLTVL | QVQLVQSGAEVKKPGASVKISCKASGFTFTDYYMN WVRQAPGQGLEWMGWINPNSGGTNYAQKFQGRV TMTRDTSISTAYMELSRLRSDDTAVYYCARGYDSF APWGQGTLVTVSS |
| | | | SEQ ID NO: 27712 | SEQ ID NO: 31718 |

FIGURE 50

| | | | | |
|---|---|---|---|---|
| iPS:437094 | 21-225_210D12 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ATTATGTCTCCTGGTACCAACAACACCCAGTC AAAGCCCCCAAACTCTTGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAACTCATATACAAGCAGCAT CACTTGGGTGTTCGGCGGAGGGACCAAGCTGA CCGTCCTA | CAGGTGCAGTTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGGGGGACTG GAACCCCGAGGGTTTGGACGTCTGGGGCCAAGG GACCACGGTCACCGTCTCCTCA |
| | | | SEQ ID NO: 27713 | SEQ ID NO: 31719 |
| | | AA | QSALTQPASVSGSPGQSITISCTGTSSDVGGYNY VSWYQQHPVKAPKLLIYEVSNRPSGVSNRFSGS KSGNTASLTISGLQAEDEADYYCNSYTSSITWVF GGGTKLTVL | QVQLVESGGGVVQPGRSLRLSCAASGFTFSHYGM HWVRQAPGKGLEWVAVIWYDGSNKYYADSVKGR FTISRDNSKNTLYLQMNSLRAEDTAVYYCARGDW NPEGLDVWGQGTTVTVSS |
| | | | SEQ ID NO: 27714 | SEQ ID NO: 31720 |
| iPS:437096 | 21-225_210E12 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAACACCCAGGC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCGGCTCATATGTAAAAGGCAT CACTTGGGTGTTCGGCGGAGGGACCAGTCTAA CCGTCCTC | CAGGTGCACCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGGGGGACTG GAACCCCGAGGGTATGGACGTCTGGGGCCAAGG GACCACGGTCATCGTCTCCTCA |
| | | | SEQ ID NO: 27715 | SEQ ID NO: 31721 |

FIGURE 50

| | | | | |
|---|---|---|---|---|
| | | AA | QSALTQPASVSGSPGQSITISCTGTSSDVGGYNY VSWYQQHPGKAPKLMIYEVSNRPSGVSNRFSGS KSGNTASLTISGLQAEDEADYYCGSYVKGITWV FGGGTSLTVL | QVHLVESGGGVVQPGRSLRLSCAASGFTFSNYGM HWVRQAPGKGLEWVAVIWYDGSNKYYADSVKGR FTISRDNSKNTLYLQMNSLRAEDTAVYYCARGDW NPEGMDVWGQGTTVIVSS |
| | | | SEQ ID NO: 27716 | SEQ ID NO: 31722 |
| iPS:437098 | 21-225_211C1 | NA | CAGTCTGCCCTGACTCAGCCTGCCTCCGTGTCT GGGTCTCCTGGACAGTCGATCACCATCTCCTG CACTGGAACCAGTAGTGACGTTGGTAGTTATA ACTATGTCTCCTGGTACCAACAGTACCCAGGC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAACTCATATACAAGCAGCAT CACTTGGGTGTTCGGCGGAGGGACCAAGCTGA CCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACACCCTGAGAGCCGAGG ACACGGCTATCTATTACTGTGCGAGGGGGGGACTG GAACCCCGAGGGTTTGGACGTCTGGGGGCCAAGG GACCACGGTCACCGTCTCCTCA |
| | | | SEQ ID NO: 27717 | SEQ ID NO: 31723 |
| | | AA | QSALTQPASVSGSPGQSITISCTGTSSDVGSYNYV SWYQQYPGKAPKLMIYEVSNRPSGVSNRFSGSK SGNTASLTISGLQAEDEADYYCNSYTSSITWVFG GGTKLTVL | QVQLVESGGGVVQPGRSLRLSCAASGFTFSHYGM HWVRQAPGKGLEWVAVIWYDGSNKYYADSVKGR FTISRDNSKNTLYLQMNTLRAEDTAIYYCARGDWN PEGLDVWGQGTTVIVSS |
| | | | SEQ ID NO: 27718 | SEQ ID NO: 31724 |

FIGURE 50

| iPS:437100 | 21-225_211H2 | NA | TCCTATGAACTGACTCAGCCACTCTCAGTGTCAGTGGCCCTGGGACAGACGGCCAGGATTACCTGTGGGGGAAACAACATTGGACGTAGAAATGTGCACTGGTACCAACAGAAGCCAGGCCAGGCCCCTATACTGGTCATCTATAGAGATCGCGACCGGCCCTCTGGGATCCCTGAGCGATTCTCTGGCTCCAACTCGGGGAACACGGCCACCCTGACCATCAGCAGAGCCCAAGCCGGGGATGAGGCTGACTATTTCTGTCAGGTGTGGGACAGCAGTACTGCGGTGTTCGGCGGAGGGACCAAACTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGCCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCGCCAGAGACAATTCCAAGAACACGCTGTATCTGCAAATGAACAGCCTGAGAGCCGAGGACACGGCTGTGTATTACTGTGCGAGAGATCCTGGGAGCTACGGGTTCGACCCCTGGGGCCAGGGAACCCTGGTCACCGTCTCCTCA |
| --- | --- | --- | --- | --- |
| | | | SEQ ID NO: 27719 | SEQ ID NO: 31725 |
| | | AA | SYELTQPLSVSVALGQTARITCGGNNIGRRNVHWYQQKPGQAPILVIYRDRDRPSGIPERFSGSNSGNTATLTISRAQAGDEADYFCQVWDSSTAVFGGGTKLTVL | QVQLVESGGGVVQPGRSLRLSCAASGFTFSSYAMHWVRQAPGKGLEWVAVIWYDGSNKYYADSVKGRFTIARDNSKNTLYLQMNSLRAEDTAVYYCARDPGSYGFDPWGQGTLVTVSS |
| | | | SEQ ID NO: 27720 | SEQ ID NO: 31726 |
| iPS:437102 | 21-225_211E5 | NA | TCCTATGAGCTGACACAGCCACCCTCGGTGTCAGTGTCCCCAGGACAGACGGCCAGGATCACCTGTTCTGGAGATGCATTGCCAAAGCAATATGCTTATTGGTACCAGCAGAAGCCAGGCCAGGCCCCAGTGCTGGTGATATATAAAGACAGTGCGAGGCCCTCAGGGATCCCTGAGCGATTCTCTGGCTCCCGCTCAGGGACAACAGTCACGTTGACCGTCAGTGGAGTCCAGGCAGAAGACGAGGCTGACTATTACTGTCAATTAGTGTACAGCAGTGATACTTATGTCTTCGGAACTGGGACCATGCTCACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGGAACTATGGCATGCACTGGGTCCGCCAGGCTCCAGGCAAGGGGCTGGAGTGGGTGTCAATTATATGGTTTGATGGAAGTGATCAATACTATGCAGACTCCGTGAAGGGCCGATTCACCATCTCCAGAGACAACTCCAAGAACACGCTGTACCTGCAAATGAACAGCCTGAGAGCCGAGGACACGGCTGTTTATTACTGTGCGAGAGGCCTCTCTGTCTACTACTACGGTATGGGCGTCTGGGGCCAAGGGACCACGGTCACCGTCTCCTCA |
| | | | SEQ ID NO: 27721 | SEQ ID NO: 31727 |

FIGURE 50

| | | | | |
|---|---|---|---|---|
| | | AA | SYELTQPPSVSVSPGQTARITCSGDALPKQYAYW YQQKPGQAPVLVIYKDSARPSGIPERFSGSRSGT TVTLTVSGVQAEDEADYYCQLVYSSDTYVFGTG TMLTVL | QVQLVESGGGVVQPGRSLRLSCAASGFTFRNYGM HWVRQAPGKGLEWVSIIWFDGSDQYYADSVKGRF TISRDNSKNTLYLQMNSLRAEDTAVYYCARGLSVY YYGMGVWGQGTTVTVSS |
| | | | SEQ ID NO: 27722 | SEQ ID NO: 31728 |
| iPS:437104 | 21-225_211G5 | NA | CAGTCTGCCCTGACTCAGCCTGCCTCCGTGTCT GGGTCTCCTGGACAGTCGATAACCATCTCCTG CACTGGAACCAGCAGTGACGTTGGTGGTTATA ACTATGTCTCCTGGTACCAACAGCACCCAGGC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAACTCATATACAAGAAGCAT CACTTGGGTGTTCGGCGGAGGGACCAAGCTGA CCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTACAAATGAACAGCCTGAGAGCCGAGG ACACGGCTGTGTATTACTGTGCGAGGGGGGGACTG GAACCCCGAGGGTTTGGACGTCTGGGGGCCAAGG GACCACGGTCACCGTCTCCTCA |
| | | | SEQ ID NO: 27723 | SEQ ID NO: 31729 |
| | | AA | QSALTQPASVSGSPGQSITISCTGTSSDVGGYNY VSWYQQHPGKAPKLMIYEVSNRPSGVSNRFSGS KSGNTASLTISGLQAEDEADYYCNSYTRSITWVF GGGTKLTVL | QVQLVESGGGVVQPGRSLRLSCAASGFTFSHYGM HWVRQAPGKGLEWVAVIWYDGSNKYYADSVKGR FTISRDNSKNTLYLQMNSLRAEDTAVYYCARGDW NPEGLDVWGQGTTVTVSS |
| | | | SEQ ID NO: 27724 | SEQ ID NO: 31730 |

FIGURE 50

| | | | | |
|---|---|---|---|---|
| iPS:437106 | 21-225_211H7 | NA | CAGACTGTGGTGACTCAGGAGCCCTCACTGAC TGTGTCCCCAGGAGGGACAGTCACTCTCACCT GTGCTTTCAGCACTGGAGCAGTCACCAGTGGT AACTATCCAAGTTGGTTCCAGCAGAAACCTGG ACAAGTTCCCAGGGCACTGATTTATAGTACAA GCAACAGACACTCCTGGACCCCTGCCCGGTTT TCTGGCTCCCTCCTTGGGGGCAAAGCTGCCCT GACACTGTCAGGTGTGCAGCCTGAGGACGAG GCTGAATATTACTGCCTGCTCTACTATGGTGGT GCTCAGCTGGTGTTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGACCCGCCAGCACCCAGGGAA GGGCCTGGAGTGGATTGGGTACATCTATTACGTT GGGAGCACCTACTACAACCCGTCCCTCAAGAGTC GAGTTACCATATCAGTAGACACGTCTAAGAACCA GTTCTCCCTGAAGCTGAGTTCTGTGACTGCCGCG GACACGGCCGTGTATTACTGTGCGAGAGATGGG CCATTGAGCGGTATGGACGTCTGGGGCCAAGGG ACCACGGTCACCGTCTCCTCA |
| | | | SEQ ID NO: 27725 | SEQ ID NO: 31731 |
| | | AA | QTVVTQEPSLTVSPGGTVTLTCAFSTGAVTSGNY PSWFQQKPGQVPRALIYSTSNRHSWTPARFSGSL LGGKAALTLSGVQPEDEAEYYCLLYYGGAQLV FGGGTKLTVL | QVQLQESGPGLVKPSQTLSLTCTVSGGSISSGGYYW SWTRQHPGKGLEWIGYIYYVGSTYYNPSLKSRVTIS VDTSKNQFSLKLSSVTAADTAVYYCARDGPLSGM DVWGQGTTVTVSS |
| | | | SEQ ID NO: 27726 | SEQ ID NO: 31732 |
| iPS:437108 | 21-225_211C9 | NA | CAGACTGTGGTGACTCAGGAGCCCTCACTGAC TGTGTCCCCAGGAGGGACAGTCACTCTCACCT GTGGTTCCAGCACTGGATCAGTCACCAGTGGT TACTTTCCAAACTGGTTCCAGCAGAAACCTGG ACAAGCACCCAGGCCACTGATTTATAGTACAA ACAACAAGCACTCCTGGACCCCTGCCCGGTTC TCAGGCTCCCTCCTTGGGGGCAAAGCTGCCCT GACACTGTCAGATGTGCAGCCTGAGGACGAG GCTGACTATTACTGCCTGCTCTACTATGGTGGT GCTCAGCTGGCATTCGGCGGAGGGACCAAACT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGAAGTGGTGGTGA CTACTGGAGCTGGATCCGCCAGCACCCAGGGAA GGGCCTGGAGTGGATTGGATACATCTATTATAGT GGGAGCACCTACTACAACCCGTCCCTCAAGAGTC GAGTTACCATATTACTAGACACGTCTAAGAACCA GTTCTCCCTGAAGCTGAGCTCTGTGACTGTCGCG GACACGGCCGTGTATTACTGTGCGAGAGATTCAG CAGTGTACAATATGGACGTCTGGGGCCAAGGGA CCACGGTCACCGTCTCCTCA |
| | | | SEQ ID NO: 27727 | SEQ ID NO: 31733 |

FIGURE 50

| | | | | |
|---|---|---|---|---|
| | | AA | QTVVTQEPSLTVSPGGTVTLTCGSSTGSVTSGYF PNWFQQKPGQAPRPLIYSTNNKHSWTPARFSGS LLGGKAALTLSDVQPEDEADYYCLLYYGGAQL AFGGGTKLTVL | QVQLQESGPGLVKPSQTLSLTCTVSGGSIRSGGDY WSWIRQHPGKGLEWIGYIYYSGSTYYNPSLKSRVTI LLDTSKNQFSLKLSSVTVADTAVYYCARDSAVYN MDVWGQGTTVTVSS |
| | | | SEQ ID NO: 27728 | SEQ ID NO: 31734 |
| iPS:437110 | 21-225_211E9 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT AACTATCCAAACTGGTTCCAGCAGAAACCTGG ACAAGCACCCAGGGCACTGATTTATAGTACAA TCAACAAACACTCCGGGACCCCTGCCCGGTTT ACAGGCTTCCTCCTTGGGGGGCAAAGCTGCCCT GACACTGTCAGGTGTACAGCCTGAGGACGAG GCTGAGTATTACTGCCTGCTCTACTATGGTGGT GCTCAGCTGGCATTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTGA CTACTGGAGCTGGATCCGCCAGCACCCAGGGAA GGGCCTGGAGTGGATTGGGTACATCTATTACACT GGGAGCAACTACTACAACCCGTCCCTCAAGAGTC GAGTTACCATATCAGTAGACACGTCTAAGAATCA GTTCTCCCTGAACCTGATCTCTGTGACTGCCGCG GACACGGCCGTGTATTACTGTGCGAGAGATTCAG CAGTGTACGGTATGGACGTCTGGGGCCAAGGGA CCACGGTCACCGTCTCCTCA |
| | | | SEQ ID NO: 27729 | SEQ ID NO: 31735 |
| | | AA | QTVVTQEPSLTVSPGGTVTLTCASSTGAVTSGNY PNWFQQKPGQAPRALIYSTINKHSGTPARFTGFL LGGKAALTLSGVQPEDEAEYYCLLYYGGAQLA FGGGTKLTVL | QVQLQESGPGLVKPSQTLSLTCTVSGGSISSGGDYW SWIRQHPGKGLEWIGYIYYTGSNYYNPSLKSRVTIS VDTSKNQFSLNLISVTAADTAVYYCARDSAVYGM DVWGQGTTVTVSS |
| | | | SEQ ID NO: 27730 | SEQ ID NO: 31736 |

FIGURE 50

| iPS:437112 | 21-225_212C2 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAACACCCAGGC AAAGCCCCCAAACTCATGATTTATGAGGTCAG AAATCGGCCCTCAGGAGTTTCTAATCGCTTCT CTGGCTCCAAGTCTGGCAACACGGCCTCCCTG ACCATCTCTGGGCTCCAGGCTGAGGACGAGGC TGATTATTACTGCAGCTCATATACACGCAGCA TCACTTGGGTGTTCGGCGGAGGGACCAAGCTG ACCGTCCTA | CAGGTGCAACTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGGGGGACTG GAACCCCGAGGGTATGGACGTCTGGGGCCAAGG GACCTCGGTCACCGTCTCCTCA |
| | | | SEQ ID NO: 27731 | SEQ ID NO: 31737 |
| | | AA | QSALTQPASVSGSPGQSITISCTGTSSDVGGYNY VSWYQQHPGKAPKLMIYEVRNRPSGVSNRFSGS KSGNTASLTISGLQAEDEADYYCSSYTRSITWVF GGGTKLTVL | QVQLVESGGGVVQPGRSLRLSCAASGFTFSHYGM HWVRQAPGKGLEWVAVIWYDGSNKYYADSVKGR FTISRDNSKNTLYLQMNSLRAEDTAVYYCARGDW NPEGMDVWGQGTSVTVSS |
| | | | SEQ ID NO: 27732 | SEQ ID NO: 31738 |
| iPS:437114 | 21-225_212A4 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAACACCCAGGC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCGGCTCATATGTAAAAGGCAT CACTTGGGTGTTCGGCGGAGGGACCAGTCTAA CCGTCCTC | CAGGTGCACCTGGTGGAGTCTGGGGGAGGCGTG GTCCAGGCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGGGGGACTG GAACCCCGAGGGTATGGACGTCTGGGGCCAAGG GACCACGGTCATCGTCTCCTCA |
| | | | SEQ ID NO: 27733 | SEQ ID NO: 31739 |

2611

FIGURE 50

| | | | | |
|---|---|---|---|---|
| | | AA | QSALTQPASVSGSPGQSITISCTGTSSDVGGYNY VSWYQQHPGKAPKLMIYEVSNRPSGVSNRFSGS KSGNTASLTISGLQAEDEADYYCGSYVKGITWV FGGGTSLTVL | QVHLVESGGGVVQAGRSLRLSCAASGFTFSNYGM HWVRQAPGKGLEWVAVIWYDGSNKYYADSVKGR FTISRDNSKNTLYLQMNSLRAEDTAVYYCARGDW NPEGMDVWGQGTTVIVSS |
| | | | SEQ ID NO: 27734 | SEQ ID NO: 31740 |
| iPS:437116 | 21-225_212F6 | NA | CAGTCTGCCCTGACTCAGCCTGCCTCCGTGTCT GGGTCTCCTGGACAGTCGATCACCATCTCCTG CACTGGAACCAGTAGTGACGTTGGTGGTTATA ACTATGTCTCCTGGTACCAACAGTACCCAGGC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAACTCATATACAAGCAGCAT CACTTGGGTGTTCGGCGGAGGGACCAAGCTGA CCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCGGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGGGGGACTG GAACCCCGAGGGTTTGGACGTCTGGGGCCAAGG GACCACGGTCACCGTCTCCTCA |
| | | | SEQ ID NO: 27735 | SEQ ID NO: 31741 |
| | | AA | QSALTQPASVSGSPGQSITISCTGTSSDVGGYNY VSWYQQYPGKAPKLMIYEVSNRPSGVSNRFSGS KSGNTASLTISGLQAEDEADYYCNSYTSSITWVF GGGTKLTVL | QVQLVESGGGVVQPGRSLRLSCAASGFTFSHYGM HWVRQAPGKGLEWVAVIWYDGSNKYYADSVKGR FTISRDNSKNTLYLQMNSLRAEDTAVYYCARGDW NPEGLDVWGQGTTVTVSS |
| | | | SEQ ID NO: 27736 | SEQ ID NO: 31742 |

FIGURE 50

| iPS:437118 | 21-225_212G7 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ATTATGTCTCCTGGTACCAACAGCACCCAGGC AAAACCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAACTCATATACAAGCAGCAT CACTTGGGTGTTCGGCGGAGGGACCAAGCTGA CCGTCCTA | CAGGTGCAGTTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTGTGCAGACTCCGTGAAGGGCCGA TTCACCATCTCCAGAGACAATTCCACGAACACGC TGTATCTGCAAATGAACAGCCTGAGAGCCGAGG ACACGGCTGTGTATTACTGTGCGAGGGGAGACTG GAACCCCGAGGGTTTGGACGTCTGGGGCCAAGG GACCACGGTCACCGTCTCCTCA |
| | | | SEQ ID NO: 27737 | SEQ ID NO: 31743 |
| | | AA | QSALTQPASVSGSPGQSITISCTGTSSDVGGYNY VSWYQQHPGKTPKLMIYEVSNRPSGVSNRFSGS KSGNTASLTISGLQAEDEADYYCNSYTSSITWVF GGGTKLTVL | QVQLVESGGGVVQPGRSLRLSCAASGFTFSHYGM HWVRQAPGKGLEWVAVIWYDGSNKYCADSVKGR FTISRDNSTNTLYLQMNSLRAEDTAVYYCARGDW NPEGLDVWGQGTTVTVSS |
| | | | SEQ ID NO: 27738 | SEQ ID NO: 31744 |
| iPS:437120 | 21-225_212A9 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT TACTATCCAAACTGGTTCCAGCAGAAACCTGG ACAAGCACCCAGGGCACTGATTTATAGTACAA ACAACAAACACTCCTGGACCCCTGCCCGGTTC TCAGGCTCCCTCCTTGGGGGCAAAGCTGCCCT GACACTGTCAGGTGTACAGCCTGACGACGAGG CTGACTATTACTGCCTGCTCTACTATGGTGGTG CTCAGGTGGGATTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA GTGTCTCTGGTGGCTCCATCAGAAGTGGTGGTGA CTACTGGAGCTGGATCCGCCAGCACCCAGGGAA GGGCCTGGAGTGGATTGGGTATATGTATTACAGT GGGAGCACCTACTACAACCCGTCCCTCAAGAGTC GAGTTACCATATCAGTAGACACGTCTAAGAACCA GTTCTCCCTGAAGCTGAGTTCTGTGACTGTCGCG GACACGGCCGTGTATTACTGTGCGCGAGATTCAG CAGTGTACGGTATGGACGTCTGGGGCCAAGGGA CCACGGTCACCGTCTCCTCA |
| | | | SEQ ID NO: 27739 | SEQ ID NO: 31745 |

FIGURE 50

| | | | | |
|---|---|---|---|---|
| | | AA | QTVVTQEPSLTVSPGGTVTLTCASSTGAVTSGYY PNWFQQKPGQAPRALIYSTNNKHSWTPARFSGS LLGGKAALTLSGVQPDDEADYYCLLYYGGAQV GFGGGTKLTVL | QVQLQESGPGLVKPSQTLSLTCSVSGGSIRSGGDY WSWIRQHPGKGLEWIGYMYYSGSTYYNPSLKSRV TISVDTSKNQFSLKLSSVTVADTAVYYCARDSAVY GMDVWGQGTTVTVSS |
| | | | SEQ ID NO: 27740 | SEQ ID NO: 31746 |
| iPS:437124 | 21-225_212H12 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT TACTATCCAAACTGGTTCCAGCAGAAACCTGG ACAAGCACCCAGGGCACTGATTTATAGTACAA GCAACAAACACTCCTGGACCCCTGCCCGGTTC TCAGGCTCCCTCCTTGGGGGCAAAGCTGCCCT GACACTGTCAGGTGTGCAGCCTGAGGACGAG GCTGAGTATTACTGCCTGCTCTACTATGGTGGT GCTCATGTGGTATTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGAAGTGGTGGTGA CTACTGGAGTTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGGAGACACGTCTAAGAACC AGTTCTCCCTGAAGCTGAGCTCTGTGACTGCCGC GGACACGGCCGTGTATTACTGTGCGAGGGATAG CAGCTCCTACGGTATGGACGTCTGGGGCCAAGG GACCACGGTCACCGTCTCCTCA |
| | | | SEQ ID NO: 27741 | SEQ ID NO: 31747 |
| | | AA | QTVVTQEPSLTVSPGGTVTLTCASSTGAVTSGYY PNWFQQKPGQAPRALIYSTNKHSWTPARFSGS LLGGKAALTLSGVQPEDEAEYYCLLYYGGAHV VFGGGTKLTVL | QVQLQESGPGLVKPSQTLSLTCTVSGGSIRSGGDY WSWIRQHPGKGLEWIGYIYYSGSTYYNPSLKSRVTI SGDTSKNQFSLKLSSVTAADTAVYYCARDSSSYGM DVWGQGTTVTVSS |
| | | | SEQ ID NO: 27742 | SEQ ID NO: 31748 |

FIGURE 50

| iPS:437128 | 21-225_213G3 | NA | CTGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAACACCCAGGC AAAGCCCCCAAACTCATGATTTCTGAGGTCAG GAATCGGCCCTCAGGGGTTTCTAATCGCTTCT CTGGCTCCAAGTCTGGCAACACGGCCTCCCTG ACCATCTCTGGGCTCCAGGCTGAGGACGAGGC TGATTATTACTGCAACTCATATACACGCAGCA TCACTTGGGTGTTCGGCGGAGGGACCAAGCTG ACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGCATCTGCAAATGAACAGCCTGAGAGCCGAGG ACACGGCTGTGTATTACTGTGCGAGGGGGGGACTG GAACCCCGAGGGTATGGACGTCTGGGGCCAAGG GACCTCGGTCACCGTCTCCTCA |
|---|---|---|---|---|
| | | | SEQ ID NO: 27743 | SEQ ID NO: 31749 |
| | | AA | LSALTQPASVSGSPGQSITISCTGTSSDVGGYNYV SWYQQHPGKAPKLMISEVRNRPSGVSNRFSGSK SGNTASLTISGLQAEDEADYYCNSYTRSITWVFG GGTKLTVL | QVQLVESGGGVVQPGRSLRLSCAASGFTFSHYGM HWVRQAPGKGLEWVAVIWYDGSNKYYADSVKGR FTISRDNSKNTLHLQMNSLRAEDTAVYYCARGDW NPEGMDVWGQGTSVTVSS |
| | | | SEQ ID NO: 27744 | SEQ ID NO: 31750 |
| iPS:437130 | 21-225_213D5 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAACACCCAGGC AAAGCCCCCAAACTCGTGATTTATGAGGTCCG TAATCGGCCCTCAGGGGTTTCTACTCGCTTCTC TGGCTCCAAGTCTGGCAACAAGGCCTCCCTGA CCATCTCTGGGCTCCAGGCTGAGGACGAGGCT GATTATTACTGCTGCTCATATACAAGAAGAAT CACTTGGGTGTTCGGCGGAGGGACCAAGCTGA CCGTCCTG | CAGGTGCAGCTGGTGGAATCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTTTCTGCAAATGAACAGCCTGAGAGTCGAGGA CACGGCTGTGTATTATTGTGCGAGGGGGGGACTGG AACCCCGAGGGTATGGACGTCTGGGGCCAAGGG ACCACGGTCACCGTCTCCTCA |
| | | | SEQ ID NO: 27745 | SEQ ID NO: 31751 |

FIGURE 50

| | | | | |
|---|---|---|---|---|
| | | AA | QSALTQPASVSGSPGQSITISCTGTSSDVGGYNY VSWYQQHPGKAPKLVIYEVRNRPSGVSTRFSGS KSGNKASLTISGLQAEDEADYYCCSYTRRITWV FGGGTKLTVL | QVQLVESGGGVVQPGRSLRLSCAASGFTFSHYGM HWVRQAPGKGLEWVAVIWYDGSNKYYADSVKGR FTISRDNSKNTLFLQMNSLRVEDTAVYYCARGDW NPEGMDVWGQGTTVTVSS |
| | | | SEQ ID NO: 27746 | SEQ ID NO: 31752 |
| iPS:437132 | 21-225_213F5 | NA | CAGACTGTGGTGACTCAGGAGCCCTCACTGAC TGTGTCCCCAGGAGGGACAGTCACTCTCACCT GTGGTTCCAGCACTGGATCAGTCACCAGTGGT TACTTTCCAAACTGGTTCCAGCAGAAACCTGG ACAAACACCCAGGCCACTGATTTATAGTACAA ACAACAAGCACTCCTGGACCCCTGCCCGGTTC TCAGGCTCCCTCCTTGGGGGCAAAACTGCCCT GACACTGTCAGATGTGCAGCCTGAGGACGAG GCTGACTATTACTGCCTGCTCTACTTTGGTGGT GCTCAGCTGGCATTCGGCGGAGGGACCAAACT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGAAGTGGTGGTGA CTACTGGAGCTGGATCCGCCAGCACCCAGGGAA GGGCCTGGAGTGGATTGGGTACATCTATTATAGT GGGAGCACCTACTACAACCCGTCCCTCAAGAGTC GAGTTACCATATCACTAGACACGTCTAAGAACCA GTTCTCCCTGAAGCTGAGCTCTGTGACTGTCGCG GACACGGCCGTGTATTACTGTGCGAGAGATTCAG CAGTGTACAATATGGACGTCTGGGGCCAAGGGA CCACGGTCACCGTCTCCTCA |
| | | | SEQ ID NO: 27747 | SEQ ID NO: 31753 |
| | | AA | QTVVTQEPSLTVSPGGTVTLTCGSSTGSVTSGYF PNWFQQKPGQTPRPLIYSTNNKHSWTPARFSGSL LGGKTALTLSDVQPEDEADYYCLLYFGGAQLAF GGGTKLTVL | QVQLQESGPGLVKPSQTLSLTCTVSGGSIRSGGDY WSWIRQHPGKGLEWIGYIYYSGSTYYNPSLKSRVTI SLDTSKNQFSLKLSSVTVADTAVYYCARDSAVYN MDVWGQGTTVTVSS |
| | | | SEQ ID NO: 27748 | SEQ ID NO: 31754 |

FIGURE 50

| | | | | |
|---|---|---|---|---|
| iPS:437134 | 21-225_213A7 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAGCACCCAGGC AAAGCCCCCAAATTCATGATTTATGAGGTCAG GAATCGGCCCTCAGGGGTTTCTAATCGCTTCT CTGGCTCCAAGTCTGGCAACACGGCCTCCCTG ACCATCTCTGGGCTCCAGGCTGAGGACGAGGC TGATTATTACTGCAGCTCATATACCAGCAGCC GCACTCTGGTATTCGGCGGAGGGACCAAGTTG ACCGTCCTA | CAGGTGCAGCTGGTGCAGTCTGGGGGCTGAGGTG AAGAAGCCTGGGGCCTCCGTGAAGATCTCCTGCA GGGCTTCTGGATTCACCTTCACCGACTACTATAT GAACTGGGTGCGACAGGCCCCTGGACAAGGGCT TGAGTGGATGGGATGGATCAACCCTAAGAATGG TGGCACAAACTATGCACAGAAGTTTCAGGGCAG GGTCACCATGACCAGGGACACGTCCCTCAGCAG AGCCTACATGGAGCTGAGTAGGCTGAGATCTGA CGACACGGCCGTGTATTACTGTGCGAAAGGGTAT GATTCGTTCGCCCCCTGGGGCCAGGGAACCCTGG TCACCGTCTCCTCA |
| | | | SEQ ID NO: 27749 | SEQ ID NO: 31755 |
| | | AA | QSALTQPASVSGSPGQSITISCTGTSSDVGGYNY VSWYQQHPGKAPKFMIYEVRNRPSGVSNRFSGS KSGNTASLTISGLQAEDEADYYCSSYTSSRTLVF GGGTKLTVL | QVQLVQSGAEVKKPGASVKISCRASGFTFTDYYMN WVRQAPGQGLEWMGWINPKNGGTNYAQKFQGRV TMTRDTSLSRAYMELSRLRSDDTAVYYCAKGYDS FAPWGQGTLVTVSS |
| | | | SEQ ID NO: 27750 | SEQ ID NO: 31756 |
| iPS:437136 | 21-225_214H3 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT TACTATCCAAACTGGTTCCAGCAGAAACCTGG ACAAGCACCCAGGGCACTGATTTATAGTACAA GCAACAAACACTCCTGTACCCCTGCCCGGTTC TCAGGCTCCCTCCTTGGGGGCAAAGCTGCCCT GACACTGTCAGGTGTGCAGCCTGAGGACGAG GCTGAGTATTACTGCCTGCTCTACTATGGTGGT GCTCATGTGGTATTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGAAGTGGTGGTGA CTACTGGAGTTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGGAGACACGTCTAAGAACC AGTTCTCCCTGAAGCTGAGCTCTGTGACTGCCGC GGACACGGCCGTGTATTACTGTGCGAGGGATAG CAGCTCCTACGGTATGGACGTCTGGGGCCAAGG GACCACGGTCACCGTCTCCTCA |
| | | | SEQ ID NO: 27751 | SEQ ID NO: 31757 |

FIGURE 50

| | | | | |
|---|---|---|---|---|
| | | AA | QTVVTQEPSLTVSPGGTVTLTCASSTGAVTSGYY PNWFQQKPGQAPRALIYSTSNKHSCTPARFSGSL LGGKAALTLSGVQPEDEAEYYCLLYYGGAHVV FGGGTKLTVL | QVQLQESGPGLVKPSQTLSLTCTVSGGSIRSGGDY WSWIRQHPGKGLEWIGYIYYSGSTYYNPSLKSRVTI SGDTSKNQFSLKLSSVTAADTAVYYCARDSSSYGM DVWGQGTTVTVSS |
| | | | SEQ ID NO: 27752 | SEQ ID NO: 31758 |
| iPS:437138 | 21-225_214D8 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAATAAT TATGTATCCTGGTACCAGCAGTTCCCAGGAAC AGCCCCCAAACTCCTCATTCATGACAATAATA AGCGACCCTCAGGGATTCCTGACCGATTCTCT GGCTCCAAGTCTGGCACGTCAGCCACCCTGGG CATCACCGGACTCCAGACTGGGGACGAGGCC GATTATTACTGCGGAGCATGGGATAGCAGCCT GAGTGCTGTGTGGTAATCGGCGGAGGGAGCAAG CTGACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGTACTGCTTTTTAC TACTGGAGCTGGATCCGCCAGCACCCAGGGAAG GGCCTGGAGTGGATTGGGTACATCTATTTCAGTG GGAGCACCTACTACAACCCGTCCCTCAAGAGTCG AGTTACCATATCAGTAGACACGTCTAAGAACCAG TTCTCCCTGAACCTGAGCTCTGTGACTGCCGCGG ACACGGCCGTGTATTACTGTGCGAGAGCAAGGG GATATCACTACAGTATCTTTGACTACTGGGGCCA GGGAACCCTGGTCACCGTCTCCTCA |
| | | | SEQ ID NO: 27753 | SEQ ID NO: 31759 |
| | | AA | QSVLTQPPSVSAAPGQKVTISCSGSSSNIGNNYVS WYQQFPGTAPKLLIHDNNKRPSGIPDRFSGSKSG TSATLGITGLQTGDEADYYCGAWDSSLSAVVIG GGSKLTVL | QVQLQESGPGLVKPSQTLSLTCTVSGGSISTAFYYW SWIRQHPGKGLEWIGYTYFSGSTYYNPSLKSRVTIS VDTSKNQFSLNLSSVTAADTAVYYCARARGYHYSI FDYWGQGTLVTVSS |
| | | | SEQ ID NO: 27754 | SEQ ID NO: 31760 |

FIGURE 50

| iPS:437140 | 21-225_214E12 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGC TACTATCCAAACTGGTTCCAACAGAAACCTGG ACAAGCACCCAGGGCACTGATTTATAGTACAA GCAATAAACACTCCTGGACCCCTGCCCGGTTC TCAGGCTCCCTCCTTGGGGGCAAAGCTGCCCT GACACTGTCAGATGTGCAGCCTGAGGACGAG GCTGAGTATTACTGCCTGCTCTACTGTGATGGT GCCCAGCTGGTGTTCGGCGGAGGGACCAAACT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTGA TTACTGGAGCTGGATCCGCCAGCACCCAGGGAA GGGCCTGGAGTGGATTGGGTACATCTATTACAGT GGGCCCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTATTGTGCGAGAGATGGGG CTGCGGAGGGTATGGACGTCTGGGGCCAAGGGA CCACGGTCACCGTCTCCTCA |
|  |  |  | SEQ ID NO: 27755 | SEQ ID NO: 31761 |
|  |  | AA | QTVVTQEPSLTVSPGGTVTLTCASSTGAVTSGYY PNWFQQKPGQAPRALIYSTSNKHSWTPARFSGS LLGGKAALTLSDVQPEDEAEYYCLLYCDGAQL VFGGGTKLTVL | QVQLQESGPGLVKPSQTLSLTCTVSGGSISSGGDYW SWIRQHPGKGLEWIGYIYYSGPTYYNPSLKSRVTIS VDTSKNQFSLKLSSVTAADTAVYYCARDGAAEGM DVWGQGTTVTVSS |
|  |  |  | SEQ ID NO: 27756 | SEQ ID NO: 31762 |
| iPS:437142 | 21-225_215A3 | NA | CAGACTGTGGTGACTCAGGAGCCCTCACTGAC GGTGTCCCCAGGAGGGACAGTCACTCTCACCT GTGCTTCCAGCACTGAAGCCGTCACCAGTGGT AACTATCCAAGCTGGTTCCAGCAGAAACCTGG ACAAGCACCCAGGGCACTGATTTATAGTACAA GCAACAAACACTCCGGTACCCCTGCCCGGTTT ACAGGCTCCCTCCTTGGGGGCAAAGCTGCCCT GACACTGTCAGGTGTACAGCCTGAGGACGAG GCTGAGTATTACTGCCTGCTCTACTATGGTGG CGCTCAGCTGGCATTCGGCGGAGGGACCAAGC TGGCCGTCCTA | CAGGTGCAGTTGCAGGAGTCGGGCCCAGGACTG GTGAAGCCTTCACAGACCCTGTCCCTCACCTGCA CTGTCTCTGGTGGCTCCATCAGCAGTGGTGGTGA CTACTGGAGCTGGATCCGCCAGCACCCAGGGAA GGGCCTGGAGTGGATTGGGTACATCTATTACACT GGGAGCAACTACTACAACCCGTCCCTCAAGAGTC GAGTTACCATATCAGTAGACACGTCTAAGAATCA GTTCTCCCTGAAGGTGATCTCTGTGACTGCCGCG GACACGGCCGTGTATTACTGTGCGAGAGATTCAG CAGTGTACGGTATGGACGTCTGGGGCCAAGGGA CCACGGTCACCGTCTCCTCA |
|  |  |  | SEQ ID NO: 27757 | SEQ ID NO: 31763 |

FIGURE 50

| | | | | |
|---|---|---|---|---|
| | | AA | QTVVTQEPSLTVSPGGTVTLTCASSTEAVTSGNY PSWFQQKPGQAPRALIYSTSNKHSGTPARFTGSL LGGKAALTLSGVQPEDEAEYYCLLYYGGAQLA FGGGTKLAVL | QVQLQESGPGLVKPSQTLSLTCTVSGGSISSGGDYW SWIRQHPGKGLEWIGYIYYTGSNYYNPSLKSRVTIS VDTSKNQFSLKVISVTAADTAVYYCARDSAVYGM DVWGQGTTVTVSS |
| | | | SEQ ID NO: 27758 | SEQ ID NO: 31764 |
| iPS:437144 | 21-225_215B3 | NA | TCCTATGAGCTGACTCAGCCACCCTCAGTGTC CGTGTCCCCAGGACAGACAGCCAGCATCACCT GCTCTGGAGATAAATTGGGGGATAAATTTGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAGCTGACCGTCCTA | GAGGTGCAGCTGGTGGAGTCTGGGGGAGGCTTG GTAAAGCCTGGGGGGTCCCTTAGACTCTCCTGTG CAGCCTCTGGATTCACTTTCAGTAACGCCTGGAT GCACTGGGTCCGCCAGGCTCCAGGGAAGGGGCT GGAGTGGGTTGGCCGTATTAAAAGCAAAACTAA TGGTGGGACAACAGACTACGCTGCACCCGTGAA AGGCAGATTCACCATTTCAAGAGATGATTCAAAA AACACGCTGTATCTGCAAATGAACAGCCTGAAA ACCGAGGACACAGCCGTGTATTACTGTACCACAG ATCCGGGGGGGATCTTTGACTACTGGGGCCAGG GAACCCTGGTCACCGTCTCCTCA |
| | | | SEQ ID NO: 27759 | SEQ ID NO: 31765 |
| | | AA | SYELTQPPSVSVSPGQTASITCSGDKLGDKFACW YQQKPGQSPVLVIYQDSKRPSGIPERFSGSNSGN TATLTISGTQAMDEADYYCQAWDSSTVVFGGG TKLTVL | EVQLVESGGGLVKPGGSLRLSCAASGFTFSNAWM HWVRQAPGKGLEWVGRIKSKTNGGTTDYAAPVK GRFTISRDDSKNTLYLQMNSLKTEDTAVYYCTTDP GGIFDYWGQGTLVTVSS |
| | | | SEQ ID NO: 27760 | SEQ ID NO: 31766 |

FIGURE 50

| | | | | |
|---|---|---|---|---|
| iPS:437146 | 21-225_215D3 | NA | CAGTCTGCCCTGACTCAGCCTGCCTCCGTGTCT GGGTCTCCTGGACAGTCGATCACCATCTCCTG CACTGGAACCAGCAGTGACATTGGTGGTTATA ACTATGTCTCCTGGTACCAACAACACCCAGGC AAAGCCCCCACACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAACTCATATAAAAGGGGCAG CACTTGGGTGTTCGGCGGAGGGACCAAGGTGA CCGTCCTA | CAGACGCAGCTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAATGGGTGGCAGTTATATGGTATGATGGAAGT AATGAGTACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGGGGGACTG GAACCCCGAGGGTATGGACGTCTGGGGCCAAGG GACCACGGTCACCGTCTCCTCA |
| | | | SEQ ID NO: 27761 | SEQ ID NO: 31767 |
| | | AA | QSALTQPASVSGSPGQSITISCTGTSSDIGGYNYV SWYQQHPGKAPTLMIYEVSNRPSGVSNRFSGSK SGNTASLTISGLQAEDEADYYCNSYKRGSTWVF GGGTKVTVL | QTQLVESGGGVVQPGRSLRLSCAASGFTFSHYGMH WVRQAPGKGLEWVAVIWYDGSNEYYADSVKGRF TISRDNSKNTLYLQMNSLRAEDTAVYYCARGDWN PEGMDVWGQGTTVTVSS |
| | | | SEQ ID NO: 27762 | SEQ ID NO: 31768 |
| iPS:437148 | 21-225_215H3 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT TACTATCCAAACTGGTTCCAGCAGAAACCTGG ACAAGCACCCAGGGCACTGATTTATAGTACAA ACAACAAACACTCCTGTGGACCTGCCCGGTTT TCAGGCTCCCTCCTTGGGGGGCAAAGCTGCCCT GACACTGTCAGGTGTACAGCCTGACGACGAGG CTGACTATTACTGCCTGCTCTACTATGGTGGTG CTCAGGTGGGATTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA GTGTCTCTGGTGGCTCCATCAGAAGTGGTGGTGA CTACTGGAGCTGGATCCGCCAGCACCCAGGGAA GGGCCTGGAGTGGATTGGGTATATGTATTACAGT GGGAGCACCTACTACAACCCGTCCCTCAAGAGTC GAGTTACCATATCAGTAGACACGTCTAAGAACCA GTTCTCCCTGAAGCTGAGTTCTGTGACTGTCGCG GACACGGCCGTGTATTACTGTGCGCGAGATTCAG CAGTGTACGGTATGGACGTCTGGGGCCAAGGGA CCACGGTCACCGTCTCCTCA |
| | | | SEQ ID NO: 27763 | SEQ ID NO: 31769 |

FIGURE 50

| | | | | |
|---|---|---|---|---|
| | | AA | QTVVTQEPSLTVSPGGTVTLTCASSTGAVTSGYY PNWFQQKPGQAPRALIYSTNNKHSCGPARFSGS LLGGKAALTLSGVQPDDEADYYCLLYYGGAQV GFGGGTKLTVL | QVQLQESGPGLVKPSQTLSLTCSVSGGSIRSGGDY WSWIRQHPGKGLEWIGYMYYSGSTYYNPSLKSRV TISVDTSKNQFSLKLSSVTVADTAVYYCARDSAVY GMDVWGQGTTVTVSS |
| | | | SEQ ID NO: 27764 | SEQ ID NO: 31770 |
| iPS:437150 | 21-225_216A3 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ATTATGTCTCCTGGTACCAACAACACCCAGGC AAAGCCCCCAAACTCATGATTTATGAGGTCAG TAATCGGCCCTCAGGGGTTTCTAATCGCTTCTC TGGCTCCAAGTCTGGCAACACGGCCTCCCTGA CCATCTCTGGGCTCCAGGCTGAGGACGAGGCT GATTATTACTGCAACTCATATACAAGCAGCAT CACTTGGGTGTTCGGCGGAGGGACCAAGCTGA CCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGGGGGACTG GAACCCCGAGGGTTTGGACGTCTGGGGGCCAAGG GACCACGGTCACCGTCTCCTCA |
| | | | SEQ ID NO: 27765 | SEQ ID NO: 31771 |
| | | AA | QSALTQPASVSGSPGQSITISCTGTSSDVGGYNY VSWYQQHPGKAPKLMIYEVSNRPSGVSNRFSGS KSGNTASLTISGLQAEDEADYYCNSYTSSITWVF GGGTKLTVL | QVQLVESGGGVVQPGRSLRLSCAASGFTFSHYGM HWVRQAPGKGLEWVAVIWYDGSNKYYADSVKGR FTISRDNSKNTLYLQMNSLRAEDTAVYYCARGDW NPEGLDVWGQGTTVTVSS |
| | | | SEQ ID NO: 27766 | SEQ ID NO: 31772 |

FIGURE 50

| iPS:437154 | 21-225_216A7 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGT TACTATCCAAACTGGTTCCAGCAGAAACCTGG ACAAGCACCCAGGGCACTGATTTATAGTACAA ACAACAAACACTCCTGTACCCCTGCCCGGTTC TCAGGCTCCCTCCTTGGGGGCAAAGCTGCCCT GACACTGTCAGGTGTACAGCCTGACGACGAGG CTGACTATTACTGCCTGCTCTACTATGGTGGTG CTCAGGTGGGATTCGGCGGAGGGACCAAGCT GACCGTCCTA | CAGGTGCAGCTGCAGGAGTCGGGCCCAGGACTG GTGAAGCCTTCACAGACCCTGTCCCTCACCTGCA GTGTCTCTGGTGGCTCCATCAGAAGTGGTGGTGA CTACTGGAGCTGGATCCGCCAGCACCCAGGGAA GGGCCTGGAGTGGATTGGGTATATGTATTACAGT GGGAGCACCTACTACAACCCGTCCCTCAAGAGTC GAGTTACCATATCAGTAGACACGTCTAAGAACCA GTTCTCCCTGAAGCTGAGTTCTGTGACTGTCGCG GACACGGCCGTGTATTACTGTGCGCGAGATTCAG CAGTGTACGGTATGGACGTCTGGGGCCAAGGGA CCACGGTCACCGTCTCCTCA |
|---|---|---|---|---|
| | | | SEQ ID NO: 27767 | SEQ ID NO: 31773 |
| | | AA | QTVVTQEPSLTVSPGGTVTLTCASSTGAVTSGYY PNWFQQKPGQAPRALIYSTNNKHSCTPARFSGSL LGGKAALTLSGVQPDDEADYYCLLYYGGAQVG FGGGTKLTVL | QVQLQESGPGLVKPSQTLSLTCSVSGGSIRSGGDY WSWIRQHPGKGLEWIGYMYYSGSTYYNPSLKSRV TISVDTSKNQFSLKLSSVTVADTAVYYCARDSAVY GMDVWGQGTTVTVSS |
| | | | SEQ ID NO: 27768 | SEQ ID NO: 31774 |
| iPS:437158 | 21-225_216H11 | NA | CAGACTGTGGTGACTCAGGAGCCCTCACTGAC TGTGTCCCCAGGAGGGACAGTCACTCTCACCT GTGCTTCCAGCACTGGAGCAGTCACCAGTGGC TACTATCCAAACTGGTTCCAACAGAAACCTGG ACAAGCACCCAGGGCACTGATTTATAGTACAA GCAATAAACACTCCTGGACCCCTGCCCGGTTC TCAGGCTCCCTCCTTGGGGGCAAAGCTGCCCT GACACTGTCAGATGTGCAGCCTGAGGACGAG GCTGAGTATTACTGCCTGCTCTACTGTGATGGT GCTCAGCTGGTGTTCGGCGGAGGGACCAAACT GACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTGA TTACTGGAGCTGGATCCGCCAGCACCCAGGGAA GGGCCTGGAGTGGATTGGGTACATCTATTACAGT GGGCCCACCTACTACAACCCGTCCCTCAAGAGTC GAGTTACCATATCAGTAGACACGTCTAAGAACCA GTTCTCCCTGAAGCTGAGCTCTGTGACTGCCGCG GACACGGCCGTGTATTATTGTGCGAGAGATGGGG CTGCGGAGGGTTTGGACGTCTGGGGCCAAGGGA CCACGGTCACCGTCTCCTCA |
| | | | SEQ ID NO: 27769 | SEQ ID NO: 31775 |

FIGURE 50

| | | | | |
|---|---|---|---|---|
| | | AA | QTVVTQEPSLTVSPGGTVTLTCASSTGAVTSGYY PNWFQQKPGQAPRALIYSTSNKHSWTPARFSGS LLGGKAALTLSDVQPEDEAEYYCLLYCDGAQL VFGGGTKLTVL | QVQLQESGPGLVKPSQTLSLTCTVSGGSISSGGDYW SWIRQHPGKGLEWIGYIYYSGPTYYNPSLKSRVTIS VDTSKNQFSLKLSSVTAADTAVYYCARDGAAEGL DVWGQGTTVTVSS |
| | | | SEQ ID NO: 27770 | SEQ ID NO: 31776 |
| iPS:437160 | 21-225_216B12 | NA | TCCTATGAGCTGACTCAGCCACTCTCAGTGTC AGTGGCCCTGGGACAGACGGCCAGGATTACCT GTGGGGGAGACAACATTAGAAGAAGAAATGT GCACTGGTACCAGCAGAAGCCAGGCCAGGCC CCTGTGCTGGTCATCTATAGGGATAGCAACCG GCCCTCTGGGATCCCTGAGCGATTCTCTGGCT CCAACTCGGGGAACACGGCCACCCTGACCATC AGCAGAGCCCAAGCCGGGGATGAGGCTGACT ATTACTGTCAGGTGTGGGACAGCAGCACTGGG GTGTTCGGCGGAGGGACCAAGCTGACCGTCCT A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGCCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCTGGG ACTGGGATACTTCTTTGACTACTGGGGCCAGGGA ACCCTAGTCACCGTCTCCTCA |
| | | | SEQ ID NO: 27771 | SEQ ID NO: 31777 |
| | | AA | SYELTQPLSVSVALGQTARITCGGDNIRRRNVH WYQQKPGQAPVLVIYRDSNRPSGIPERFSGSNSG NTATLTISRAQAGDEADYYCQVWDSSTGVFGG GTKLTVL | QVQLVESGGGVVQPGRSLRLSCAASGFTFSSYAMH WVRQAPGKGLEWVAVIWYDGSNKYYADSVKGRF TISRDNSKNTLYLQMNSLRAEDTAVYYCARDLGLG YFFDYWGQGTLVTVSS |
| | | | SEQ ID NO: 27772 | SEQ ID NO: 31778 |

FIGURE 50

| iPS:437162 | 21-225_217B2 | NA | CAGTCTGCCCTGACTCAGCCTGCCTCCGTGTCT GGGGTCTCCTGGACAGTCGATCACCATCTCCTG CACTGGAACCAGCAGTGACGTTGGTGGTTATA ACTATGTCTCCTGGTACCAACAACACCCAGGC AAAGCCCCCAAACTCTTGATTTATGAGGTCAG TAATCGGCCCTCAGGGGTTTATAATCGCTTCTC TGGCTCCAAGTCTGGCAACACGGCCTCCCTGA CCATCTCTGGGCTCCAGGCTGAGGACGAGGCT GATTATTACTGCGGCTCATATGTAAAAGGCAT CACTTGGGTGTTCGGCGGAGGGACCAGTCTGA CCGTCCTC | CAGGTGCACCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTATTGTGCGAGGGGGGGACTG GAACCCCGAGGGTATGGACGTCTGGGGCCAAGG GACCACGGTCATCGTCTCCTCA |
| | | | SEQ ID NO: 27773 | SEQ ID NO: 31779 |
| | | AA | QSALTQPASVSGSPGQSITISCTGTSSDVGGYNY VSWYQQHPGKAPKLLIYEVSNRPSGVYNRFSGS KSGNTASLTISGLQAEDEADYYCGSYVKGITWV FGGGTSLTVL | QVHLVESGGGVVQPGRSLRLSCAASGFTFSNYGM HWVRQAPGKGLEWVAVIWYDGSNKYYADSVKGR FTISRDNSKNTLYLQMNSLRAEDTAVYYCARGDW NPEGMDVWGQGTTVIVSS |
| | | | SEQ ID NO: 27774 | SEQ ID NO: 31780 |
| iPS:437164 | 21-225_217C6 | NA | TCCTATGAGCTGACACAGCCACCCTCGGTGTC AGTGTCCCCAGGACAGACGGCCAGGATCACCT GCTCTGGAGATGCATTGCCAAAGCAATATGCT TATTGGTACCAGCAGAAGCCAGGCCAGGCCCC AGTGCTGGTGATATATAAAGACAGTGAGAGG CCCTCAGGGATTCCTGAGCGATTCTCTGGCTC CCGCTCAGGGACAACAGTCACGTTGACCATCA GAGGAGTCCAGGCAGAAGACGAGGCTGACTA TTACTGTCAATTAATAGTCAGCAGTGATACTT ATGTCTTCGGAACTGGGACCAAGGTCACCGTC CTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGATGGCAATTATATGGTTTGATGGAAGT GATGAATACTATGCAGACTCCGTGAAGGGCCGA TTCACCATCTCCAGAGACAATTCCAAGAACACGA TGTATCTGCAAATGAACAGCCTGAGAGCCGAAG ACACGGCTGTGTATTACTGTGCGAGAGGCCTATC TGTCTACTACTACGGTATGGACGTCTGGGGCCAA GGGACCACGGTCACCGTCTCCTCA |
| | | | SEQ ID NO: 27775 | SEQ ID NO: 31781 |

FIGURE 50

| | | | | |
|---|---|---|---|---|
| | | AA | SYELTQPPSVSVSPGQTARITCSGDALPKQYAYW YQQKPGQAPVLVIYKDSERPSGIPERFSGSRSGTT VTLTIRGVQAEDEADYYCQLIVSSDTYVFGTGT KVTVL | QVQLVESGGGVVQPGRSLRLSCAASGFTFSNYGM HWVRQAPGKGLEWMAIIWFDGSDEYYADSVKGRF TISRDNSKNTMYLQMNSLRAEDTAVYYCARGLSV YYYGMDVWGQGTTVTVSS |
| | | | SEQ ID NO: 27776 | SEQ ID NO: 31782 |
| iPS:437166 | 21-225_217G11 | NA | TCCTATGAGCTGACACAGCCACCCTCGGTGTC AGTGTCCCCAGGACAGACGGCCAGGATCACCT GCTCTGGAGATGCATTGCCAAAACAATATGCT TATTGGTACCAGCAGAAGCCAGGCCAGGCCCC AGTACTGGTGATATATAAAGACAGTGAGAGG CCCTCAGGGATCCCTGAGCGATTCTCTGGCTC CCGCTCAGGGACAACAGTCACGTTGACCGTCA GTGGAGTCCAGGCAGAAGACGAGGCTGACTA TTACTGTCAATTAGTGTACAGCAGTGATACTT ATGTCTTCGGAACTGGGACCAAGGTCACCGTC CTA | CAGGTGCAGTTGGTGGAGTCTGGGGGAGGCGTG GTCCAGCCTGGGAGGTCCCTGAGACTCTCCTGTG CAGCGTCTGGATTCACCTTCAGGAACTATGGCAT GCACTGGGTCCGCCAGGCTCCAGGCAAGGGGCT GGAGTGGGTGTCAATTATATGGTTTGATGGAAGT GATCAGTACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTATATTACTGTGCGAGAGGCCTCTCT GTCTACTACTACGGTATGGACGTCTGGGGCCAAG GGACCACGGTCACCGTCTCCTCA |
| | | | SEQ ID NO: 27777 | SEQ ID NO: 31783 |
| | | AA | SYELTQPPSVSVSPGQTARITCSGDALPKQYAYW YQQKPGQAPVLVIYKDSERPSGIPERFSGSRSGTT VTLTVSGVQAEDEADYYCQLVYSSDTYVFGTGT KVTVL | QVQLVESGGGVVQPGRSLRLSCAASGFTFRNYGM HWVRQAPGKGLEWVSIIWFDGSDQYYADSVKGRF TISRDNSKNTLYLQMNSLRAEDTAVYYCARGLSVY YYGMDVWGQGTTVTVSS |
| | | | SEQ ID NO: 27778 | SEQ ID NO: 31784 |

2626

FIGURE 50

| | | | | |
|---|---|---|---|---|
| iPS:437168 | 21-225_218G4 | NA | CAGTCTGTGTTGACGCAGCCGCCCTCAGTGTC TGCGGCCCCAGGACAGAAGGTCACCATCTCCT GCTCTGGAAGCAGCTCCAACATTGGGAATAAT TATGTATCCTGGTACCAGCAGCTCCCAGGAAC AGCCCCCAAACTCCTCCTTTATGACAGTAATA AGCGACCCTCAGGGATTCCTGCCCGATTCTCT GGCTCCAAGTCTGGCACGTCAGCCACCCTGGG CATCACCGGACTCCAGACTGGGGACGAGGCC GATTATTACTGCGGAACATGGGATAGCAGCCT GAATACTGTGGTATTCGGCGGAGGGACCAAGC TGACCGTCCTA | CAGGTGCAGCTGGTGGAGTCTGGGGGAGGCGTA GTCCAGCCTGGGAGGTCCCTGAGACTCTCCTGTG CAGCGTCTGGATTCACCTTCAGTAGCTATGGCTT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGTCGAGG ACACGGCTGTGTACTACTGTGCGAACTGGTACTA CTATTACTACGGTATGGACGTCTGGGGCCAAGGG ACCACGGTCACCGTCTCCTCA |
| | | | SEQ ID NO: 27779 | SEQ ID NO: 31785 |
| | | AA | QSVLTQPPSVSAAPGQKVTISCSGSSSNIGNNYVS WYQQLPGTAPKLLLYDSNKRPSGIPARFSGSKSG TSATLGITGLQTGDEADYYCGTWDSSLNTVVFG GGTKLTVL | QVQLVESGGGVVQPGRSLRLSCAASGFTFSSYGLH WVRQAPGKGLEWVAVIWYDGSNKYYADSVKGRF TISRDNSKNTLYLQMNSLRVEDTAVYYCANWYYY YYGMDVWGQGTTVTVSS |
| | | | SEQ ID NO: 27780 | SEQ ID NO: 31786 |
| iPS:437170 | 21-225_218E5 | NA | TCCTATGAGCTGACACAGCCACCCTCGGTGTC AGTGTCCCCAGGACAGACGGCCAGGATCACCT GCTCTAGAGATGTATTGCCGAAGCAATATGCT TATTGGTACCAGCAGAAGCCAGGCCAGGCCCC AGTACTGGTGATATATAAAGACAGTGAGAGG CCCTCAGGGATCCCTGAGCGATTCTCTGGCTC CCGCTCAGGGACAACAGTCACGTTGACCATCA GAGGAGTCCAGGCAGAAGACGAGGCTGACTA TTACTGTCAATTAGTTGTCAGCAGTGATACTTA TGTCTTCGGAACTGGGACCAAGGTCACCGTCC TA | CAGGTGCAGTTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGATGGCAATTATATGGTTTGATGGAAGT GATGAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTATATTACTGTGCGAGAGGCCTATC TGTCTACTACTACGGTATGGACGTCTGGGGCCAA GGGACCACGGTCACCGTCTCCTCA |
| | | | SEQ ID NO: 27781 | SEQ ID NO: 31787 |

FIGURE 50

| | | | | |
|---|---|---|---|---|
| | | AA | SYELTQPPSVSVSPGQTARITCSRDVLPKQYAYW YQQKPGQAPVLVIYKDSERPSGIPERFSGSRSGTT VTLTIRGVQAEDEADYYCQLVVSSDTYVFGTGT KVTVL | QVQLVESGGGVVQPGRSLRLSCAASGFTFSNYGM HWVRQAPGKGLEWMAIIWFDGSDEYYADSVKGRF TISRDNSKNTLYLQMNSLRAEDTAVYYCARGLSVY YYGMDVWGQGTTVTVSS |
| | | | SEQ ID NO: 27782 | SEQ ID NO: 31788 |
| iPS:437172 | 21-225_219A7 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ATTATGTCTCCTGGTACCAACAGCACCCAGGC AAAGCCCCCAAACTCATGATTTATGAGGTCAG AAATCGGCCCTCAGGGGTTTCTAACCGCTTCT CTGGCTCCAAGTCTGGCAACACGGCCTCCCTG ACCATCTCTGGGCTCCAGGCTGAGGACGAGGC TGATTATTACTGCTGCTCATATACAAGGAGCA TCACTTGGGTGTTCGGCGGAGGGACCAAGTTG ACCGTCCTA | CAGGTGCAGCTGGTGGAGTCTGGGGGAGGCGTG GTCCAGCCTGGGAGGTCCCTGAGACTCTCCTGTC CAGCGTCTGGATTCACCTTCAGTCACTATGGCAT GCACTGGGTCCGCCAGGCTCCAGGCAAGGGGCT GGAGTGGGTGGCAGTCATATGGTATGATGGAAG TAATAAATACTATGCAGACTCCGTGAAGGGCCG ATTCACCATCTCCAGAGACAATTCCAAGAATATG CTGTATCTGCAAATGAACAGCCTGAGAGCCGAG GACACGGCTGTGTATTACTGTGCGAGGGGGGGACT GGAACCCCGAGGGTATGGACGTCTGGGGCCAAG GGACCACGGTCACCGTCTCCTCA |
| | | | SEQ ID NO: 27783 | SEQ ID NO: 31789 |
| | | AA | QSALTQPASVSGSPGQSITISCTGTSSDVGGYNY VSWYQQHPGKAPKLMIYEVRNRPSGVSNRFSGS KSGNTASLTISGLQAEDEADYYCCSYTRSITWVF GGGTKLTVL | QVQLVESGGGVVQPGRSLRLSCPASGFTFSHYGMH WVRQAPGKGLEWVAVIWYDGSNKYYADSVKGRF TISRDNSKNMLYLQMNSLRAEDTAVYYCARGDWN PEGMDVWGQGTTVTVSS |
| | | | SEQ ID NO: 27784 | SEQ ID NO: 31790 |

FIGURE 50

| | | | | |
|---|---|---|---|---|
| iPS:437182 | 21-225_221H2 | NA | CTGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAACACCCAGGC AAAGCCCCCAAACTCATGATTTCTGAGGTCAG GAATCGGCCCTCAGGGGTTTCTAATCGCTTCT CTGGCTCCAAGTCTGGCAACACGGCCTCCCTG ACCATCTCTGGGCTCCAGGCTGAGGACGAGGC TGATTATTACTGCAACTCATATACACGCAGCA TCACTTGGGTGTTCGGCGGAGGGACCAAGCTG ACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGCATCTGCAAATGAACAGCCTGAGAGCCGAGG ACACGGCTGTGTATTACTGTGCGAGGGGGGGACTG GAACCCCGAGGGTATGGACGTCTGGGGCCAAGG GACCACGGTCACCGTCTCCTCA |
| | | | SEQ ID NO: 27785 | SEQ ID NO: 31791 |
| | | AA | LSALTQPASVSGSPGQSITISCTGTSSDVGGYNYV SWYQQHPGKAPKLMISEVRNRPSGVSNRFSGSK SGNTASLTISGLQAEDEADYYCNSYTRSITWVFG GGTKLTVL | QVQLVESGGGVVQPGRSLRLSCAASGFTFSHYGM HWVRQAPGKGLEWVAVIWYDGSNKYYADSVKGR FTISRDNSKNTLHLQMNSLRAEDTAVYYCARGDW NPEGMDVWGQGTTVTVSS |
| | | | SEQ ID NO: 27786 | SEQ ID NO: 31792 |
| iPS:437184 | 21-225_221G4 | NA | CAGTCTGCCCTGACTCAGCCTGCCTCCGTGTCT GGGTCTCCTGGACAGTCGATCACCATCTCCTG CACTGGAACCAGCAGTGACGTTGGTGGTTATA ACTATGTCTCCTGGTACCAACAACACCCAGGC AAAGCCCCCAAACTCATGATTTATGAGGTCAG GAATCGGCCCTCAGGGGTTTCTAATCGCTTCT CTGGCTCCAAGTCTGGCAACACGGCCTCCCTG ACCATCTCTGGGCTCCAGGCTGAGGACGAGGC TGATTATTACTGCAACTCATATACACGCAGCA TCACTTGGGTGTTCGGCGGAGGGACCAAGCTG ACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTCACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGCATCTGCAAATGAACAGCCTGAGAGCCGAGG ACACGGCTGTGTATTACTGTGCGAGGGGGGGACTG GAACCCCGAGGGTATGGACGTCTGGGGCCAAGG GACCTCGGTCACCGTCTCCTCA |
| | | | SEQ ID NO: 27787 | SEQ ID NO: 31793 |

FIGURE 50

| | | | | | |
|---|---|---|---|---|---|
| | | AA | QSALTQPASVSGSPGQSITISCTGTSSDVGGYNY VSWYQQHPGKAPKLMIYEVRNRPSGVSNRFSGS KSGNTASLTISGLQAEDEADYYCNSYTRSITWVF GGGTKLTVL | QVQLVESGGGVVQPGRSLRLSCAASGFTFSHYGM HWVRQAPGKGLEWVAVIWYDGSNKYYADSVKGR FTISRDNSKNTLHLQMNSLRAEDTAVYYCARGDW NPEGMDVWGQGTSVTVSS |
| | | | SEQ ID NO: 27788 | SEQ ID NO: 31794 |
| iPS:437186 | 21-225_224H2 | NA | TCCTATGAGCTGACTCAGCCATCCTCAGTGTC CGTGTCCCCAGGACAGACAGCCAGCATCACCT GCTCTGGAGATAATTTGGGGGTTAAATATACT TACTGGTATCAGCAGAAGCCAGGCCAGTCCCC TGTGCTAGTCGTCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAGCTGACCGTCCTA | CAGGTACAGCTGCAGCAGTCAGGTCCAGGACTG GTGAAGCCCTCGCAGACCCTCTCACTCACCTGTG ACATCTCCGGGGACAGTGTCTCTAGCAACAGTGC TGCTTGGAACTGGATCAGGCAGTCCCCATCGCGA GGCCTTGAGTGGCTGGGAAGGACATACTACAGG TCCAAGTGGTATAATGATTATGCAGTATCTGTGA AAAGTCGAGTAACCATCAACCCAGACACATCCA AGAACCAGTTCTCCCTGCAGCTGAACTCTGTGAC TCCCGAGGACACGGCTGTGTATTACTGTGCAAGA GAGGGGGGGCCTAGGATATTGTAGTAGTACCAGCT GCTATGGAGGCTGGTTCGACCCCTGGGGCCAGG GAACCCTGGTCACCGTCTCCTCA |
| | | | SEQ ID NO: 27789 | SEQ ID NO: 31795 |
| | | AA | SYELTQPSSVSVSPGQTASITCSGDNLGVKYTYW YQQKPGQSPVLVVYQDSKRPSGIPERFSGSNSGN TATLTISGTQAMDEADYYCQAWDSSTVVFGGG TKLTVL | QVQLQQSGPGLVKPSQTLSLTCDISGDSVSSNSAA WNWIRQSPSRGLEWLGRTYYRSKWYNDYAVSVK SRVTINPDTSKNQFSLQLNSVTPEDTAVYYCAREGG LGYCSSTSCYGGWFDPWGQGTLVTVSS |
| | | | SEQ ID NO: 27790 | SEQ ID NO: 31796 |

FIGURE 50

| iPS:437188 | 21-225_224B11 | NA | CAGTCTGTGCTGACGCAGCCGCCCTCAGTGTC TGGGGCCCCAGGGCAGAGGGTCACCATCTCCT GCACTGGGAGCAGCTCCAACATCGGGGCAGG TTATGATGTACACTGGTACCAGCAGCTTCCAG GAACAGCCCCCAAACTCCTCATCTTTGGTAAC AGCAATCGGCCCTCAGGGGTCCCTGACCGATT CTCTGGCTCCAAGTCTGGCACCTCAGCCTCCCT GGCCATCACTGGGCTCCAGGCTGAGGATGAGG CTGATTATCACTGCCAGTCCTATGACAACAGC CTGAGTGGTGTGTTCGGCGGAGGGACCAAGCT GACCGTCCTA | CAGGTACACCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGGC TTGAGTGGATGGGATGGATCAACCCTAAAAATG GTGGCACAAACTATGCACAGAAGTTTCAGGGCA GGGTCACCATGACCAGGGACGCGTCCATCAGCA CAACCTACATGGAGCTGAGCAGGCTGAGATCTG ACGACACGGCCGTGTATTACTGTGCGAGAGGAG CGTTTGATTACTTCTACTACTACGCTATGGACGTC TGGGGCCACGGGACCACGGTCACCGTCTCCTCA |
| | | | SEQ ID NO: 27791 | SEQ ID NO: 31797 |
| | | AA | QSVLTQPPSVSGAPGQRVTISCTGSSSNIGAGYD VHWYQQLPGTAPKLLIFGNSNRPSGVPDRFSGS KSGTSASLAITGLQAEDEADYHCQSYDNSLSGV FGGGTKLTVL | QVHLVQSGAEVKKPGASVKVSCKASGYTFTGYYIH WVRQAPGQGLEWMGWINPKNGGTNYAQKFQGRV TMTRDASISTTYMELSRLRSDDTAVYYCARGAFDY FYYYAMDVWGHGTTVTVSS |
| | | | SEQ ID NO: 27792 | SEQ ID NO: 31798 |
| iPS:437190 | 21-225_225A9 | NA | TCCTATGAGCTGACTCAGCCACCCTCAGTGTC CGTGTCCCCAGGACAGACAGCCAGCATCACCT GCTCTGGAGATAAATTGGGGAATAAATATGCT TGCTGGTATCAGCAGAAGCCAGGCCAGTCCCC TGTGTTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAACACTGCATGT GTCTTCGGAACTGGGACCAAGGTCACCGTCCT A | CAGGTGCAGCTGGTGGAGTCTGGGGGAGGCGTG GTCCAGCCTGGGGGGTCCCTGAGACTCTCCTGTG CAGCGTCTGGATTCACCTTCAGTACCTATGGCAT GCACTGGGTCCGCCTGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACATTTCCCAGAACACGC TGTATCTGCAAATGAACAGCCTGAGAGCCGAGG ACACGGCTGTGTATTACTGTGCGAGAGATAACCA CTATTGTAGTAGTACCAGCTGCTCCCCATACTAC TACTACTTCGGTATGGACGTCTGGGGCCAAGGGA CCACGGTCACCGTCTCCTCA |

FIGURE 50

| | | | | SEQ ID NO: 27793 | SEQ ID NO: 31799 |
|---|---|---|---|---|---|
| | | AA | | SYELTQPPSVSVSPGQTASITCSGDKLGNKYACW YQQKPGQSPVLVIYQDSKRPSGIPERFSGSNSGN TATLTISGTQAMDEADYYCQAWDSNTACVFGT GTKVTVL | QVQLVESGGGVVQPGGSLRLSCAASGFTFSTYGMH WVRLAPGKGLEWVAVIWYDGSNKYYADSVKGRF TISRDISQNTLYLQMNSLRAEDTAVYYCARDNHYC SSTSCSPYYYYFGMDVWGQGTTVTVSS |
| | | | | SEQ ID NO: 27794 | SEQ ID NO: 31800 |
| iPS:437192 | 21-225_225E9 | NA | | TCCTATGACCTGACTCAGCCACCCTCAGTGTC CGTGTCCCCAGGACAGACAGCCAGCATCACCT GCTCTGGAGATAATTTGGGGAATAGATATGCT TGCTGGTATCAGCAGAAGCCAGGCCAGTCCCC TGTACTGGTCATGTATCAAGATCGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGAACTGCTGTG GTATTCGGCGGAGGGACCAAGCTGACCGTCCT A | CAGGTGCAGTTGGTGGAGTCTGGGGGAGGCGTG GTCCAGCCTGGGAGGTCCCTGAGACTCTCCTGTG AAGCGTCTGGATTCATCTTCAGTAGCTATGGCAT GCACTGGGTCCGCCAGGCTCCAGGCAAGGGGCT GGAGTGGGTGGCAGTTATGTGGTATGATGGAGGT AATAAAGACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGGA ATATTGTACTAGTACCAGCTGCCCTTACTACTAC TACTACGGTATGGACGTCTGGGGGCCAAGGGACC ACGGTCACCGTCTCCTCA |
| | | | | SEQ ID NO: 27795 | SEQ ID NO: 31801 |
| | | AA | | SYDLTQPPSVSVSPGQTASITCSGDNLGNRYAC WYQQKPGQSPVLVMYQDRKRPSGIPERFSGSNS GNTATLTISGTQAMDEADYYCQAWDSRTAVVF GGGTKLTVL | QVQLVESGGGVVQPGRSLRLSCEASGFIFSSYGMH WVRQAPGKGLEWVAVMWYDGGNKDYADSVKGR FTISRDNSKNTLYLQMNSLRAEDTAVYYCARDREY CTSTSCPYYYYYGMDVWGQGTTVTVSS |
| | | | | SEQ ID NO: 27796 | SEQ ID NO: 31802 |

FIGURE 50

| iPS:437194 | 21-225_226B2 | NA | TCCTATGAGCTGACTCAGCCACCCTCAGTGTC CGTGTCCCCAGGACAGACAGCCAGCATCACCT GCTCTGGAGATACATTGGGGGGTAAATATGCT TGGTGGTATCAGCAGAGGCCAGGCCAGTCCCC TGTGCTGGTCATCTATCAAGATCGCAAGCGGC CCTCAGGGATCCCTGAGCGATTCTCTGGCTCC AGCTCTGGGAACACAGCCACTCTGACCATCAG CGGGACCCAGGCTATGGATGAGGCTGACTATT ACTGTCAGGCGTGGGACAACGGCGCTGCGGTT TTCGGCGGAGGGACCAAGCTGACCGTCCTA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTTTA TGCACTGGGTGCGACAGGCCCCTGGACAAGGGC TTGAGTGGATGGGATGGATCAACCCTAACAGTGG TGACACAAACTATGCACAGAAGTTTCAGGGCAG GGTCACCATGACCAGGGACACGTCCCTCAACAC AGCCTACATGGAGCTGAGCAGGCTGAGATCTGA CGACACGGCCATTTATTACTGTGCGAGAGGGACT TACTATGGTTCGGGGGAGTTATTTTAACGAACTTG ACTCCTGGGGCCAGGGAACCCTGGTCACCGTCTC CTCA |
|---|---|---|---|---|
| | | | SEQ ID NO: 27797 | SEQ ID NO: 31803 |
| | | AA | SYELTQPPSVSVSPGQTASITCSGDTLGGKYAW WYQQRPGQSPVLVIYQDRKRPSGIPERFSGSSSG NTATLTISGTQAMDEADYYCQAWDNGAAVFGG GTKLTVL | QVQLVQSGAEVKKPGASVKVSCKASGYTFTGYFM HWVRQAPGQGLEWMGWINPNSGDTNYAQKFQGR VTMTRDTSLNTAYMELSRLRSDDTAIYYCARGTYY GSGSYFNELDSWGQGTLVTVSS |
| | | | SEQ ID NO: 27798 | SEQ ID NO: 31804 |
| iPS:437196 | 21-225_226B7 | NA | TCCTTTGAGCTGACACAGCCACCCTCGGTGTC AGTGTCCCCAGGACAGACGGCCAGGATCACCT GCTCTGGAGATGCATTGCCAAGGCATTATGTT TATTGGTACCAGCAGAACCCAGGCCAGGCCCC TGTGCTGGTGATATATAAAGACAGTGAGAGGC CCTCAGGGATCCCTGAGCGATTCTCTGGCTCC AGCTCAGGGACAACAGTCACGTTGACCATCAG TGGAGTCCAGGCCAGAAGACGAGGCTGACTATT ACTGTCAATCAGCAGACAGCAGTGGTACTTAT GTCTTCGGAACTGGGACCAAGGTCACCGTCCT A | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGAC TTGAGTGGATGGGATGGATCAACCCTAACAGTGG AGGCACAAACTATGCACAGAAGTTTCAGGACAG GGTCACCATGACCAGGGACACGTCCATCAGCAC AGCCCACATGGAGCTGAGCAGGCTGAGATCTGA CGACACGGCCGTGTATTATTGTGCGAGAGGATAT TACTATGGTTCGGGGGAGTTATTATAACTGGTTCG ACTCCTGGGGCCAGGGAACCCTGGTCACCGTCTC CTCA |
| | | | SEQ ID NO: 27799 | SEQ ID NO: 31805 |

2633

FIGURE 50

| | | | | |
|---|---|---|---|---|
| | | AA | SFELTQPPSVSVSPGQTARITCSGDALPRHYVYW YQQNPGQAPVLVIYKDSERPSGIPERFSGSSSGTT VTLTISGVQAEDEADYYCQSADSSGTYVFGTGT KVTVL | QVQLVQSGAEVKKPGASVKVSCKASGYTFTGYYM HWVRQAPGQGLEWMGWINPNSGGTNYAQKFQDR VTMTRDTSISTAHMELSRLRSDDTAVYYCARGYYY GSGSYYNWFDSWGQGTLVTVSS |
| | | | SEQ ID NO: 27800 | SEQ ID NO: 31806 |
| iPS:437198 | 21-225_226F8 | NA | CAGTCTGTGCTGACGCAGCCGCCCTCAGTGTC TGGGGCCCCAGGGCAGAGGGTCACCATCTCCT GCACTGGGAGCAGCTCCAACATCGGGGCAGG TTATGATGTACACTGGTACCAGCAGCTTCCAG GAACAGCCCCCAAACTCCTCATCTATGGTAAC AGCAATCGGCCCTCAGGGGTCCCTGACCGATT CTCTGGCTCCAAGTCTGACACCTCAGCCTCCCT GGCCATCACTGGGCTCCAGGCTGAGGATGAGG CTGATTATTACTGCCAGTCCTATGACAACAGC CTGAGTGGTGTGTTCGGCGGAGGGACCAAGCT GACCGTCCTA | CAGGTGCAACTGGTGCAGTCTGGGGGCTGAGGTG AAGAAGCCTGGGGCCTCAGTGAAAGTCTCCTGC AAGGCTTCTGGATACACCTTCACCGGCTACTATA TACACTGGGTGCGACAGGCCCCTGGACAAGGGC TTGAGTGGATGGGATGGATCAACCCTAACAGTGG TGGCACAAACTATGCACGGAAGTTTCAGGGCAG GGTCACCATGACCAGGGACACGTCCATCAGCAC AGCCTACATGGAGCTGAGCAGACTGAGATCTGA CGACACGGCCGTGTATTACTGTGCGAGAGGAGC GTTTGATTACTACTACTACTACGCTTTGGACGTCT GGGGCCAAGGGACCACGGTCACCGTCTCCTCA |
| | | | SEQ ID NO: 27801 | SEQ ID NO: 31807 |
| | | AA | QSVLTQPPSVSGAPGQRVTISCTGSSSNIGAGYD VHWYQQLPGTAPKLLIYGNSNRPSGVPDRFSGS KSDTSASLAITGLQAEDEADYYCQSYDNSLSGV FGGGTKLTVL | QVQLVQSGAEVKKPGASVKVSCKASGYTFTGYYIH WVRQAPGQGLEWMGWINPNSGGTNYARKFQGRV TMTRDTSISTAYMELSRLRSDDTAVYYCARGAFDY YYYYALDVWGQGTTVIVSS |
| | | | SEQ ID NO: 27802 | SEQ ID NO: 31808 |

FIGURE 50

| | | | | |
|---|---|---|---|---|
| iPS:437200 | 21-225_226A10 | NA | TCCTATGAGCTGACTCAGCCACCCTCAGTGTC CGTGTCCCCAGGACAGACAGCCAGCATCACCT GCTCTGGAGATACATTGGGGGGTAAATATGCT TGGTGGTATCAGCAGAAGCCAGGCCAGTCCCC TGTGCTGGTCATCTATCAAGATCGCAAGCGGC CCTCAGGGATCCCTGAGCGATTCTCTGGCTCC AGCTCTGGGAACACAGCCACTCTGACCATCAG CGGGACCCAGGCTATGGATGAGGCTGACTATT ACTGTCAGGCGTGGGACAACGGCGCTGCGGTT TTCGGCGGAGGGACCAAGCTGACCGTCCTA | CAGGTGCAACTGGTGCAGTCTGGGGGCTGAGGTG AAGAAGCCTGGGGCCTCAGTGAAGGTCTCCTGC AAGGCTTCTGGATACACCTTCACCGGCTACTTTA TGCACTGGGTGCGACAGGCCCCTGGACAAGGGC TTGAGTGGATGGGTTGGATCAACCCTAACAGTGG TGACACAAACTATGCACAGAAGTTTCAGGGCAG GGTCACCATGACCAGGGACACGTCCCTCAGCAC AGCCTACATGGAGCTGAGCAGGCTGAGATCTGA CGACACGGCCATTTATTACTGTGCGAGAGGGACT TACTATGGTTCGGGGGAGTTATTTTAACGAACTTG ACTCCTGGGGCCAGGGAACCCTGGTCACCGTCTC CTCA |
| | | | SEQ ID NO: 27803 | SEQ ID NO: 31809 |
| | | AA | SYELTQPPSVSVSPGQTASITCSGDTLGGKYAW WYQQKPGQSPVLVIYQDRKRPSGIPERFSGSSSG NTATLTISGTQAMDEADYYCQAWDNGAAVFGG GTKLTVL | QVQLVQSGAEVKKPGASVKVSCKASGYTFTGYFM HWVRQAPGQGLEWMGWINPNSGDTNYAQKFQGR VTMTRDTSLSTAYMELSRLRSDDTAIYYCARGTYY GSGSYFNELDSWGQGTLVTVSS |
| | | | SEQ ID NO: 27804 | SEQ ID NO: 31810 |
| iPS:437202 | 21-225_227D3 | NA | CAGTCTGTGCTGACGCAGCCGCCCTCAGTGTC TGGGGCCCCAGGGCAGAGGGTCACCATCTCCT GCACTGGGAGCAGCTCCAACATCGGGGCAGG TTATGATGTACACTGGTACCAGCAGCTTCCAG GAACAGCCCCCAAACTCCTCATCTATGGTAAC AGCAATCGGCCCTCAGGGGTCCCTGACCGATT CTCTGGCTCCAAGTCTGACACCTCAGCCTCCCT GGCCATCACTGGGCTCCAGGCTGAGGATGAGG CTGATTATTACTGCCAGTCCTATGACAACAGC CTGAGTGGTGTGTTCGGCGGAGGGACCAAGCT GACCGTCCTA | CAGGTGCAATTGGTGCAGTCTGGGGGCTGAGGTGA AGAAGCCTGGGGCCTCAGTGAAGGTCTCCTGCA AGGCTTCTGGATACACCTTCACCGGCTACTATAT GCACTGGGTGCGACAGGCCCCTGGACAAGGGCT TGAGCGGATGGGATGGATCAACCCTAAGAGTGG TGGCACAAACTTTGCACAGAAGTTTCAGGGCAG GGTCACCATGACCAGGGACACGTCCATCAGCAC AGCCTACATGGAACTGAGCAGGCTGAGATCTGA CGACACGGCCGTGTATTACTGCGCGAGAGGAGC GTTTGATTACTTCTACTACTACGGTATGGACGTCT GGGGCCAAGGGACCACGGTCACCGTCTCCTCA |
| | | | SEQ ID NO: 27805 | SEQ ID NO: 31811 |

FIGURE 50

| | | | | |
|---|---|---|---|---|
| | | AA | QSVLTQPPSVSGAPGQRVTISCTGSSSNIGAGYD VHWYQQLPGTAPKLLIYGNSNRPSGVPDRFSGS KSDTSASLAITGLQAEDEADYYCQSYDNSLSGV FGGGTKLTVL | QVQLVQSGAEVKKPGASVKVSCKASGYTFTGYYM HWVRQAPGQGLERMGWINPKSGGTNFAQKFQGR VTMTRDTSISTAYMELSRLRSDDTAVYYCARGAFD YFYYYGMDVWGQGTTVTVSS |
| | | | SEQ ID NO: 27806 | SEQ ID NO: 31812 |
| iPS:437204 | 21-225_227E5 | NA | TCCTATGAGCTGAGTCAGCCACCCTCAGTGTC CGTGTCCCCAGGACAGACAGCCAGCATCACCT GCTCTGGAGATAAATTGGGGGAAAAATATGCT TGCTGGTATCAGCAGAAGCCAGGCCAGTCCCC TGTGCTGGTCATCTATCAAGATAGGAAGCGGC CCTCAGGGATCCCTGAGCGATTCTCTGGCTCC AACTCTGGGAACAAAGCCACTCTGACCATCAG CGGGACCCAGGCTATGGATGAGGCTGACTATT ACTGTCAGGCGTGGGTCAACAACACTATGATA TTCGGCGGAGGGACCAAGCTGACCGTCCTA | GAGGTGCAGCTGTTGGAGTCTGGGGGAGGCTTG GTACAGCCTGAGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AGCACATACTACGCAGACTCCGTGAAGGGCCGG TTCACCATCTCCAGAGACAATTCCAAGAACACGC TGTGTCTGCAAATGAACAGCCTGAGAGCCGAGG ACACGGCCGTATATTACTGTGCGAAAGAATATTG TGGTGGTGACTGCTATTCCCCTTACTACTACTACT ACGGTATGGACGTCTGGGGCCAAGGGACCACGG TCACCGTCTCCTCA |
| | | | SEQ ID NO: 27807 | SEQ ID NO: 31813 |
| | | AA | SYELSQPPSVSVSPGQTASITCSGDKLGEKYACW YQQKPGQSPVLVIYQDRKRPSGIPERFSGSNSGN KATLTISGTQAMDEADYYCQAWVNNTMIFGGG TKLTVL | EVQLLESGGGLVQPEGSLRLSCAASGFTFSSYAMS WVRQAPGKGLEWVSAISGSGGSTYYADSVKGRFTI SRDNSKNTLCLQMNSLRAEDTAVYYCAKEYCGGD CYSPYYYYGMDVWGQGTTVTVSS |
| | | | SEQ ID NO: 27808 | SEQ ID NO: 31814 |

FIGURE 50

| iPS:437208 | 21-225_227C10 | NA | CAGTCTGTGCTGACGCAGCCGCCCTCAGTGTCTGGGGCCCCAGGGCAGAGGGTCACCATCTCCTGCACTGGGAGCAGCTCCAACATCGGGGCAGGTTATGATGTACACTGGTACCAGCAGCTTCCAGGAACAGCCCCCAAACTCCTCATCTATGGTAACAGCAATCGGCCCTCAGGGGTCCCTGACCGATTCTCTGGCTCCAAGTCTGACACCTCAGCCTCCCTGGCCATCACTGGGCTCCAGGCTGAGGATGAGGCTGATTATTACTGCCAGTCCTATGACAACAACCTGAGTGGTGTGTTCGGCGGAGGGACCAAGCTGACCGTCCTA | CAGGTGCAATTGGTGCAGTCTGGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGCGGATGGGATGGATCAACCCTAAGAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGCGCGAGAGGAGCGTTTGATTACTTCTACTACTACGGTATGGACGTCTGGGGGCCAAGGGACCACGGTCACCGTCTCCTCA |
| | | | SEQ ID NO: 27809 | SEQ ID NO: 31815 |
| | | AA | QSVLTQPPSVSGAPGQRVTISCTGSSSNIGAGYDVHWYQQLPGTAPKLLIYGNSNRPSGVPDRFSGSKSDTSASLAITGLQAEDEADYYCQSYDNNLSGVFGGGTKLTVL | QVQLVQSGAEVKKPGASVKVSCKASGYTFTGYYMHWVRQAPGQGLERMGWINPKSGGTNYAQKFQGRVTMTRDTSISTAYMELSRLRSDDTAVYYCARGAFDYFYYYGMDVWGQGTTVTVSS |
| | | | SEQ ID NO: 27810 | SEQ ID NO: 31816 |
| iPS:437210 | 21-225_227E12 | NA | TCCTATGAACTGACTCAGCCACCCTCAGTGTCCGTGTCCCCAGGACAGACAGCCAGCATCACCTGCTCTGGAGATAAAATTGGGGGGATAAATATGTTTGTTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGAACAGCAGCAATGTGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGCCTTTCACTCATTTATTGGAATGATGATAAGGTCTACAGCCCATCTCTGAAGAGCAGGCTCACCATCACCAAGTACACCTCCAAAAACCAGGTGGTCCTTACAATGACCAACATGGACCCTGTGGACACAGCCACATATTACTGTGCACACAGGGGACAGCAGCTGGCCCTCGACTACTGGGGCCAGGGAACCCTGGTCACCGTCTCCTCA |
| | | | SEQ ID NO: 27811 | SEQ ID NO: 31817 |

FIGURE 50

| | | | | |
|---|---|---|---|---|
| | | AA | SYELTQPPSVSVSPGQTASITCSGDKLGDKYVCW YQQKPGQSPVLVIYQDSKRPSGIPERFSGSNSGN TATLTISGTQAMDEADYYCQAWNSSNVVFGGG TKLTVL | QITLKESGPTLVKPTQTLTLTCTFSGFSLSTSGVGVG WIRQPPGKALECLSLIYWNDDKVYSPSLKSRLTITK YTSKNQVVLTMTNMDPVDTATYYCAHRGQQLAL DYWGQGTLVTVSS |
| | | | SEQ ID NO: 27812 | SEQ ID NO: 31818 |
| iPS:437214 | 21-225_48B12 | NA | TCCTATGAGCTGACACAGCCACCCTCGGTGTC AGTGTCCCCAGGACAAACGGCCAGGATCACCT GCTCTGGAGATGCATTGCCAAAAAAATATGCT TATTGGTACCAGCAGAAGTCAGGCCAGGCCCC TGTGCTGGTCATCTATGAGGACAGCAAACGAC CCTCCGGGATCCCTGAGAGATTCTCTGGCTCC AGCTCAGGGACAATGGCCACCTTGACTATCAG TGGGGCCCAGGTGGAGGATGAAGCTGACTACT ACTGTAACTCAACAGACAGCAGTGGTAATCAT GTGGTATTCGGCGGAGGGACCAAGCTGACCGT CCTA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAACTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAAAGAGAGAC GTATAACTGGAACTACGAAGGGTTTGACTACTGG GGCCAGGGAACCCTGGTCACCGTCTCCTCA |
| | | | SEQ ID NO: 27813 | SEQ ID NO: 31819 |
| | | AA | SYELTQPPSVSVSPGQTARITCSGDALPKKYAYW YQQKSGQAPVLVIYEDSKRPSGIPERFSGSSSGT MATLTISGAQVEDEADYYCNSTDSSGNHVVFGG GTKLTVL | EVQLLESGGGLVQPGGSLRLSCAASGFTFSSYAMN WVRQAPGKGLEWVSAISGRGGNTFYADSVKGRFTI SRDNSKNTLYLQMNSLRAEDTAVYYCAKRETYNW NYEGFDYWGQGTLVTVSS |
| | | | SEQ ID NO: 27814 | SEQ ID NO: 31820 |

FIGURE 50

| iPS:437216 | 21-225_51D5 | NA | GACATCCAGATGACCCAGTCTCCATCCTCGCT GTCTGCATCTGTAGGAGACAGAGTCACCATCA CTTGTCGGGCGAGTCAGGGCATTAACAATTAT TTAGCCTGGTTTCAGCAGAAACCAGGGGAAGC CCCTAAGTCCCTGATCTATGCTGCATCCAGTTT GCGAAGTGGGGTCCCATCACAGTTCAGCGGCA GTGGATCTGGAACAGATTTCATTCTCACCATC AGCAGCCTGCAGCCTGAAGATTTTGCAACTTA TTACTGCCAACAGTATTATAGTTACCCATTCAC TTTCGGCCCTGGGACCAAAGTGGATATCAAA | GAGGTGCAGTTGTTGGAGTCTGGGGGAGGCTTGG TACAGCCTGGGGGGTCCCTGAGACTCTCCTGTGC AGCCTCTGGATTCACCTTTAGCAGCTATGTCATG AGCTGGGTCCGCCAGACTCCAGGGAAGGGGCTG GAGTGGGTCTCAACTATGAGTGGTAGTGGTGGTC GCACATACTACGCAGACTCCGTGAACGGCCGATT CACCGTCTCCAGAGACAATTCCAAGAACACGCTG TATTTGCAAATGAGCAGCCTGAGAGCCGAGGAC ACGGCCGTATATTACTGTGCGCGGGTGACTGCTT TTGACTACTGGGGCCAGGGAACCCTGGTCACCGT CTCCTCA |
| | | | SEQ ID NO: 27815 | SEQ ID NO: 31821 |
| | | AA | DIQMTQSPSSLSASVGDRVTITCRASQGINNYLA WFQQKPGEAPKSLIYAASSLRSGVPSQFSGSGSG TDFILTISSLQPEDFATYYCQQYYSYPFTFGPGTK VDIK | EVQLLESGGGLVQPGGSLRLSCAASGFTFSSYVMS WVRQTPGKGLEWVSTMSGSGGRTYYADSVNGRFT VSRDNSKNTLYLQMSSLRAEDTAVYYCARVTAFD YWGQGTLVTVSS |
| | | | SEQ ID NO: 27816 | SEQ ID NO: 31822 |
| iPS:437220 | 21-225_55H6 | NA | GACATCCAGATGACCCAGTCTCCATCCTCCCT GTCTGCATCTGTAGGAGACAGAGTCACCATCA CTTGCCGGGCAAGTCAGGGCATTAGAAACGAT TTAGGCTGGTATCAGCAGAAACCAGGGAAAG CCCCTAAGCGCCTGATCTATGGTGCATCCAGT TTGCAAAGTGGGGTCCCATCAAGATTCAGCGG CAGTGGATCTGGGACAGAATTCACTCTCACAA TCAGCAGCCTGCAGCCTGAAGATTTTGCAGCT TATTACTGTCTACAGCGTGATAGTTACCCGTTC ACTTTCGGCGGAGGGACCAAGGTGGAGATCA AA | GAGGTGCACCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTACT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAACTGGGGTC TTTGACTACTGGGGCCAGGGAACCCTGGTCACCG TCTCCTCA |
| | | | SEQ ID NO: 27817 | SEQ ID NO: 31823 |

FIGURE 50

| | | | | |
|---|---|---|---|---|
| | | AA | DIQMTQSPSSLSASVGDRVTITCRASQGIRNDLG WYQQKPGKAPKRLIYGASSLQSGVPSRFSGSGS GTEFTLTISSLQPEDFAAYYCLQRDSYPFTFGGG TKVEIK | EVHLVESGGGLVKPGGSLRLSCAASGFTFSSYSMN WVRQAPGKGLEWVSSISGSSTYIYYADSVKGRFTIS RDNAKNSLYLQMNSLRAEDTAVYYCARTGVFDY WGQGTLVTVSS |
| | | | SEQ ID NO: 27818 | SEQ ID NO: 31824 |
| IPS:437224 | 21-225_56H1 | NA | GACATCCAGATGACCCAGTCTCCATCCTCACT GTCTGCATCTGTAGGAGACAGAGTCACCATCA CTTGTCGGGCGAGTCAGGGCATTAGCCATTAT TTAGCCTGGTTTCAGCAGAAACCAGGGAAAGC CCCTCAGTCCCTGATGTCTGCTGCATCCGGTTT GCAAAGTGGGGTCCCTTCAAAGTTCAGCGGCA GTGGATCTGGGACAGATTTCACTCTCACCATC AGCAGCCTGCAGCCTGAAGATTTTGCAACTTA CTACTGTCAACAATATCAGAATTACCCCTTCA CTTTCGGCCCTGGGACCAAAGTGGATATCAAA | GAGGTGCAGCTGGTGGAATCTGGGGGAGGCCTG GTCAAGCCGGGGGGGGTCCCTGAGACTCTCCTGTG CAGCCTCTGGATTCACCTTCAGTAACTATAGAAT GAACTGGGTCCGCCAGGGTCCAGGGAAGGGGCT GGAGTGGATCTCATCCATTAGTGGTAGTAGTACT GACATATACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAGTGGCCTC CTTTGACTACTGGGGCCAGGGAACCCTGGTCACC GTCTCCTCA |
| | | | SEQ ID NO: 27819 | SEQ ID NO: 31825 |
| | | AA | DIQMTQSPSSLSASVGDRVTITCRASQGISHYLA WFQQKPGKAPQSLMSAASGLQSGVPSKFSGSGS GTDFTLTISSLQPEDFATYYCQQYQNYPFTFGPG TKVDIK | EVQLVESGGGLVKPGGSLRLSCAASGFTFSNYRMN WVRQGPGKGLEWISSISGSSTDIYYADSVKGRFTIS RDNAKNSLYLQMNSLRAEDTAVYYCARVASFDY WGQGTLVTVSS |
| | | | SEQ ID NO: 27820 | SEQ ID NO: 31826 |

FIGURE 50

| iPS:437226 | 21-225_57C2 | NA | GATGTTGTGATGACTCAGTCTCCACTCTCCCTG CCCGTCACCCTTGGACAGCCGGCCTCCATCTC CTGCAGGTCTAGTCAAAGCCTCGTATACAGTG ATGGAAACACCTACTTGAATTGGTTTCAGCAG AGGCCAGGCCAATCTCCAAGGCGCCTAATTTA TGAGGTTTCTAACTGGGACTCTGGGGTCCCAA ACAGATTCAGCGGCAGTGGGTCAGGCACTGAT TTCACACTGAAAATCAGTGCGGTGGAGGCTGA GGATGTTGGGGTTTATTACTGCGTGCAAGGTA CACACTGGCCTCGGACGTTCGGCCAAGGGACC AAGGTGGAAATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTTTGGCAT GAACTGGGTCCGCCAGGCTCCAGGGAAGGGGCT GGAGTGGGTCTCATCTATTAGTAGTAGTACTGGT TACATATACAACGCAGACTCAGTGAAGGGCCGA TTCACCATCTCCAGAGACAACGCCAAGAACTCAC TGTATCTGCAAATGAACAGCCTGAGAGCCGAAG ACACGGCTGTGTATTACTGTGCGAGAACCTATAG TGGGAGCCTGGACGTCTGGGGCCAAGGGACCAC GGTCACCGTCTCCTCA |
| | | | SEQ ID NO: 27821 | SEQ ID NO: 31827 |
| | | AA | DVVMTQSPLSLPVTLGQPASISCRSSQSLVYSDG NTYLNWFQQRPGQSPRRLIYEVSNWDSGVPNRF SGSGSGTDFTLKISAVEAEDVGVYYCVQGTHWP RTFGQGTKVEIK | EVQLVESGGGLVKPGGSLRLSCAASGFTFSSFGMN WVRQAPGKGLEWVSSISSSTGYIYNADSVKGRFTIS RDNAKNSLYLQMNSLRAEDTAVYYCARTYSGSLD VWGQGTTVTVSS |
| | | | SEQ ID NO: 27822 | SEQ ID NO: 31828 |
| iPS:437228 | 21-225_60C11 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAACGAC TTAGCCTGGTACCAGCAGAAACCTGGCCAGGC TCCCAGGCTCCTCATCTATGGTGCATCCACCA GGGCCACTGGTATCCCAGCCAGGTTCAGTGGC GGTGGGTCTGGGACAGAGTTCACTCTCACCAT CAGCGCCCTGCAGTCTGAACATTTTGCAGTTT ATTACTGTCAGCAGTATAGTAACTGGCCATTC ACTTTCGGCCCTGGGACCAAAGTGGATATCAA A | GAGGTGCAGCTGTTGGAGTCTGGGGGAGGCTTG GTACAGCCTGGGGGGTCCCTGAGACTCTCCTGTG CAGCCTCTGGATTCCCCTTTAGCAGCTATGCCAT GAGCTGGGTCCGCCAGGCTCCAGGGAAGGGGCT GGAGTGGGTCTCAGCTATTAGTGGTAGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGAAATTTTTCGGT GTAGTGGGAGTCGGGTGCTTTGACTACTGGGGCC AGGGAACCCTGGTCACCGTCTCCTCA |
| | | | SEQ ID NO: 27823 | SEQ ID NO: 31829 |

FIGURE 50

| | | | | |
|---|---|---|---|---|
| | | AA | EIVMTQSPATLSVSPGERATLSCRASQSVSNDLA WYQQKPGQAPRLLIYGASTRATGIPARFSGGGS GTEFTLTISALQSEHFAVYYCQQYSNWPFTFGPG TKVDIK | EVQLLESGGGLVQPGGSLRLSCAASGFPFSSYAMS WVRQAPGKGLEWVSAISGSGGNTFYADSVKGRFTI SRDNSKNTLYLQMNSLRAEDTAVYYCAKFFGVVG VGCFDYWGQGTLVTVSS |
| | | | SEQ ID NO: 27824 | SEQ ID NO: 31830 |
| iPS:437230 | 21-225_62H10 | NA | GACATCCAGATGACCCAGTCTCCATCCTCCCT GTCTGCATCTGTAGGAGACAGAGTCACCATCA CTTGCCGGGCAAGTCAGAGCATTACCAGCTAT TTAAATTGGTATCAGCAGAAACCAGGGAAAGT CCCTAAGCTCCTGATCTCTACTGCATCCAGTTT GCAAAGTGGGGTCCCATCAAGGTTCAGTGGCA GTGGATCTGGGACAGATTTCACTCTCACCATC AGCAGTCTGCAACCTGAAGATTTTGCAACTTA CTACTGTCAACAGAGTCACAGTTTCCCATTCA CTTTCGGCCCTGGGACCAATGTGGATTTCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGGGGGTTCG AGGGGGTTCGACCCCTGGGGGCCAGGGAACCCTG GTCACCGTCTCCTCA |
| | | | SEQ ID NO: 27825 | SEQ ID NO: 31831 |
| | | AA | DIQMTQSPSSLSASVGDRVTITCRASQSITSYLN WYQQKPGKVPKLLISTASSLQSGVPSRFSGSGSG TDFTLTISSLQPEDFATYYCQQSHSFPFTFGPGTN VDFK | EVQLVESGGGLVKPGGSLRLSCAASGFTFSSYSMN WVRQAPGKGLEWVSSISSSSSYIYYADSVKGRFTIS RDNAKNSLYLQMNSLRAEDTAVYYCARGGSRGFD PWGQGTLVTVSS |
| | | | SEQ ID NO: 27826 | SEQ ID NO: 31832 |

FIGURE 50

| iPS:437232 | 21-225_63E1 | NA | GACATTCAGATGACCCAGTCTCCATCTTCCGT GTATGCATCTGTAGGAGACAGAGTCACCATCA CTTGTCGTGCGAGTCAGGGTATTAGCAGCTAC TTAGCCTGGTATCAGCAGAAACCAGGGAAAG CCCCTAAGCTCCTGATCTATGCTGCATCCAGTT TGCAAAGTGGGGTCCCATCAAGATTCAGCGGC AGTGGGTCTGGGACAGATTTCACTCTCACCAT CAGCAGCCTGCAGCCTGAAGATTTTGCAACTT ACTATTGTCAACAGGCTAACAGTTTCCCGCTC ACTTTCGGCGGAGGGACCAAGGTGGAGATCA AA | GAGGTGCAGATGTTGGAGTCTGGGGGAGGCTTG GGACAGTCGGGGGGGTCCCTGAGACTCTCCTGTA CAGCCTCTGGATTCACCTTCACCACTTCTGCCAT GAGCTGGGTCCGCCAGGCTCCAGGGAAGGGGCT GGAGTGGGTCTCAGCTATTAGTGGTAGTGGTGCT AACACATTCTACGCAGACTCCGTGAAGGGCCGGT TCACCGTCACCAGAGACAATTCCAAGAACACGCT GTATCTGCAAATGAACAGCCTGACAGCCGAGGA CACGGCCGTTTATTATTGTGTGAAAGTTATAGCA GTGGCTGGAGGGCACTTTTTCGACCCCTGGGGCC AGGGAACCCTGGTCACCGTCTCCTCA |
|---|---|---|---|---|
| | | | SEQ ID NO: 27827 | SEQ ID NO: 31833 |
| | | AA | DIQMTQSPSSVYASVGDRVTITCRASQGISSYLA WYQQKPGKAPKLLIYAASSLQSGVPSRFSGSGS GTDFTLTISSLQPEDFATYYCQQANSFPLTFGGG TKVEIK | EVQMLESGGGLGQSGGGSLRLSCTASGFTFTTSAMS WVRQAPGKGLEWVSAISGSGANTFYADSVKGRFT VTRDNSKNTLYLQMNSLTAEDTAVYYCVKVIAVA GGHFFDPWGQGTLVTVSS |
| | | | SEQ ID NO: 27828 | SEQ ID NO: 31834 |
| iPS:437234 | 21-225_64E2 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGGC TTGAGTGGATGGGATGGATCAACCCTAACAATAA TGGCACAAACTATGCACAGAAGTTTCAGGGCAG GGTCACCATGACCAGGGACACGTCCATCAGCAC AGCCTACATGGAGCTGAGCAGGCTGAGATCTGA CGACACGGCCGTGTATTACTGTGCGAGAGATGG GAGCAGTGGCTTTGACTACTGGGGCCAGGGAAC CCTGGTCACCGTCTCCTCA |
| | | | SEQ ID NO: 27829 | SEQ ID NO: 31835 |

FIGURE 50

| | | | | |
|---|---|---|---|---|
| | | AA | DIQMTQSPSSVSASVGDRVTITCRASQGISRWLA WYQQKPGKAPKLLIYAASSLQSGVPSRFSGSGS GTDFTLTISSLQPEDFATYYCQQANSFPFTFGPGT KVDIK | QVQLVQSGAEVKKPGASVKVSCKASGYTFTGYYM HWVRQAPGQGLEWMGWINPNNNGTNYAQKFQGR VTMTRDTSISTAYMELSRLRSDDTAVYYCARDGSS GFDYWGQGTLVTVSS |
| | | | SEQ ID NO: 27830 | SEQ ID NO: 31836 |
| iPS:437248 | 21-225_97H3 | NA | GATATTGTGATGATTCAGTCTCCACTCTCCCTG CCCGTCACCCCTGGAGAGCCGGCCTCCATCTC CTGCAGGTCTAGTCAGAGCCTCCTGCATAGTA ATGGACACAACTATTTGGATTGGTACCTACAG AAGCCAGGGCGGTCTCCACAGCTCTTGATCTA TTTGGGTTCTAATCGGGCCTCCGGGGTCCCTG AGAGGTTCAGTGGCAGTGGATCAGGCACAGA TTTTACACTGAAAATCAGCAGAGTGGAGGCTG AGGATGTTGGGGTTTATTACTGCATGCAACCT CTACAAACTCCGTTCACTTTCGGCGGAGGGAC CAAGGTGGAGATCAAA | CAGGTGCAGGTACAGCAGTGGGGCGCAGGACTG TTGAAGCCTTCGGAGACCCTGTCCCTCACCTGCG CTGTCTATGGTGGGTCCTTCACTGATTACTACTGG AGCTGGATCCGCCAGCCCCCAGGGAAGGGGCTG GAGTGGATTGGGGAAATCAATCATAGTGGAGAC ACCAACTACAACCCGTCCCTCAAGAGTCGAGTCA CCATATCAGTAGACACGTCCAAGAACCAGTTCTC CCTGAAGCTGAGTTCTGTGACCGCCGCGGACACG GTGGAAGCTACCTCTACTACTACGGTATGGACGT CTGGGGCCAAGGGACCACGGTCACCGTCTCCTCA |
| | | | SEQ ID NO: 27831 | SEQ ID NO: 31837 |
| | | AA | DIVMIQSPLSLPVTPGEPASISCRSSQSLLHSNGH NYLDWYLQKPGRSPQLLIYLGSNRASGVPERFS GSGSGTDFTLKISRVEAEDVGVYYCMQPLQTPF TFGGGTKVEIK | QVQVQQWGAGLLKPSETLSLTCAVYGGSFTDYYW SWIRQPPGKGLEWIGEINHSGDTNYNPSLKSRVTIS VDTSKNQFSLKLSSVTAADTAVYYCAREFPYSGSY LYYYGMDVWGQGTTVTVSS |
| | | | SEQ ID NO: 27832 | SEQ ID NO: 31838 |

FIGURE 50

| iPS:437250 | 21-225_148C6 | NA | GATGTTGTGATGACTCAGTCTCCACTCTCCCTG CCCGTCACCCTTGGACAGCCGGCCTCCATCTC CTGCAGGTCTAGTCAAAGCCTCGTATACAGTG ATGGAAACACCTACTTGAATTGGTTTCAGCAG AGGCCAGGCCAATCTCCAAGGCGCCTAATTTA TAAGGTTTCTAACTGGGACTCTGGGGTCCCAG ACAGATTCAGCGGCAGTGGGTCAGGCACTGAT TTCACACTGAAAATCAGCAGGGTGGAGGCTGA GGATGTTGGGGTTTATTACTGCATGCAAGGTA CACACTGGTCGCTCACTTTCGGCGGAGGGACC AAGGTGGAGATCAAA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGTACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27833 | SEQ ID NO: 31839 |
| | | AA | DVVMTQSPLSLPVTLGQPASISCRSSQSLVYSDG NTYLNWFQQRPGQSPRRLIYKVSNWDSGVPDRF SGSGSGTDFTLKISRVEAEDVGVYYCMQGTHWS LTFGGGTKVEIK | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27834 | SEQ ID NO: 31840 |
| iPS:437252 | 21-225_148H11 | NA | GATGTTGTGATGACTCAGTCTCCACTCTCCCTG CCCGTCACCCTTGGACAGCCGGCCTCCATCTC CTGCAGGTCTAGTCAAAGCCTCGTATACAGTG ATGGAAACACCTACTTGAATTGGTTTCAGCAG AGGCCAGGCCAATCTCCAAGGCGCCTAATTTA TAAGGTTTCTAACTGGGACTCTGGGGTCCCAG ACAGATTCAGCGGCAGTGGGTCAGGCACTGAT TTCACACTGAAAATCAGCAGGGTGGAGGCTGA GGATGTTGGGGTTTATTACTGCATGCAAGGTA CACACTGGTTGCTCACTTTCGGCGGAGGGACC AAGGTGGAGATCAAA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27835 | SEQ ID NO: 31841 |

FIGURE 50

| | | | | |
|---|---|---|---|---|
| | | AA | DVVMTQSPLSLPVTLGQPASISCRSSQSLVYSDG NTYLNWFQQRPGQSPRRLIYKVSNWDSGVPDRF SGSGSGTDFTLKISRVEAEDVGVYYCMQGTHWL LTFGGGTKVEIK | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27836 | SEQ ID NO: 31842 |
| iPS:437254 | 21-225_149F2 | NA | GATGTTGTGATGACTCAGTCTCCACTCTCCCTG CCCGTCACCCTTGGACAGCCGGCCTCCATCTC CTGCAGGTCTAGTCAAAGCCTCGTATACAGTG ATGGAAACACCTCCTTGAATTGGTTTCAGCAG AGGCCAGGCCAATCTCCAAGGCGCCTAATTTA TAAGGTTTCTAACTGGGACTCTGGGGTCCCAG TCAGATTCAGCGGCAGTGGGTCAGGCACTGAT TTCACACTGAAAATCAGCAGGGTGGAGGCTGA GGATGTTGGGATTTATTACTGCATGCAAGGTA CACACTGGCCTCCCACTTTCGGCGGAGGGACC AAGGTGGAGATCAAA | CAGGTGCAGCTGGGGGAGGCTGGGGGAGGCGTG GTCCAGCCTGGGAGGTCCCTGAGACTCTCCTGTG CAGCGTCTGGATTCACCTTCAGTCGCTATGGCAT GCACTGGGTCCGCCAGGCTCCAGGCAAGGGGCT GGAGTGGGTGGCTTTTATATGGTATGATGGAAGT GAGAACTACTATGCAGACTCCGTGAAGGGCCGA TTCACCATCTCCAGAGTCAATTCCAGGAACACGC TGTATCTGCAAATGAACAGCCTGAGAGCCGAGG ACACGGCTGTGTATTACTGTGCGAGAGATCGGGT GGAGGGTTCGGGGACTCCCTACTACTACTACGGT ATGGACGTCTGGGGCCAAGGGACCACGGTCACC GTCTCCTCA |
| | | | SEQ ID NO: 27837 | SEQ ID NO: 31843 |
| | | AA | DVVMTQSPLSLPVTLGQPASISCRSSQSLVYSDG NTSLNWFQQRPGQSPRRLIYKVSNWDSGVPVRF SGSGSGTDFTLKISRVEAEDVGIYYCMQGTHWP PTFGGGTKVEIK | QVQLGEAGGGVVQPGRSLRLSCAASGFTFSRYGM HWVRQAPGKGLEWVAFIWYDGSENYYADSVKGR FTISRVNSRNTLYLQMNSLRAEDTAVYYCARDRVE GSGTPYYYYGMDVWGQGTTVTVSS |
| | | | SEQ ID NO: 27838 | SEQ ID NO: 31844 |

FIGURE 50

| | | | | |
|---|---|---|---|---|
| IPS:437256 | 21-225_150F11 | NA | GATGTTGTGATGAGTCAGTATCCACTCTCCCT GCCCGTCACCTTTGGACAGCCGGCCTCCATCT CATGCAGGTCTAGTCAAAGCCTCGTATACAGT GATGGAAACACCTCCTTGAATTGGTTTCAGCA GAGGCCAGGCCAATATCCAAGGCGCTTAATTT ATAAGGTTTCTAACTGGGACTATGGGGTCCCA GTCAGATTCAGCGGCAGTGGGTCAGGCACTGA TTTCACACTGAAAATCAGCAGGGTGGAGGCTG AGGATGTTGGGATTTATTACTGCATGCAAGGT ACACACTGGCCTCCCACTTTCGGCGGAGGGAC CAAGGTGGAGATCAAA<br><br>SEQ ID NO: 27839 | CAGGTGCAGCTGGGGGAGGCTGGGGGAGGCGTG GTCCAGCCTGGGAGGTCCCTGAGACTCTCCTGTG CAGCGTCTGGATTCACCTTCAGTCGCTATGGCAT GCACTGGGTCCGCCAGGCTCCAGGCAAGGGGCT GGAGTGGGTGGCTTTTATATGGTATGATGGAAGT GAGAACTACTATGCAGACTCCGTGAAGGGCCGA TTCACCATCTCCAGAGTCAATTCCAGGAACACGC TGTATCTGCAAATGAACAGCCTGAGAGCCGAGG ACACGGCTGTGTATTACTGTGCGAGAGATCGGGT GGAGGGTTCGGGGACTCCCTACTACTACTACGGT ATGGACGTCTGGGGGCCAAGGGACCACGGTCACC GTCTCCTCA<br><br>SEQ ID NO: 31845 |
| | | AA | DVVMSQYPLSLPVTFGQPASISCRSSQSLVYSDG NTSLNWFQQRPGQYPRRLIYKVSNWDYGVPVR FSGSGSGTDFTLKISRVEAEDVGIYYCMQGTHW PPTFGGGTKVEIK<br><br>SEQ ID NO: 27840 | QVQLGEAGGGVVQPGRSLRLSCAASGFTFSRYGM HWVRQAPGKGLEWVAFIWYDGSENYYADSVKGR FTISRVNSRNTLYLQMNSLRAEDTAVYYCARDRVE GSGTPYYYYGMDVWGQGTTVTVSS<br><br>SEQ ID NO: 31846 |
| IPS:437258 | 21-225_153F9 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAGCCAGGGAAAGC CCCTAAGTCCCTGATCTCTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCGCTCA GTTTCGGCGGAGGGACCAAGGTGGAGATCAA A | CAGGTACACCTGGTGGAGTCTGGGGGAGGCGTG GTCCAGCCTGGGAGGTCCCTGAGACTCTCCTGTG CAGCGTCTGGATTCACCATCAGTACCTATGGCAT GCACTGGGTCCGCCAGGGTCCAGGCAAGGGGCT GGAGTGGGTGGCAGTTATATGGTATGGTGGAAGT GATACAGACTATGCAGACTCCGTGAGGGGCCGA TTCACCATCTCCAGAGACATTTCCAAGAACACGC TGTATCTGCAAATGAACAGCCTGAGAGCCGAGG ACACGGCTGTGTATTACTGTGCGAGAGATCGGGA TTATTGTAGTGGTGGTAACTGCCCTTACTACTACT ACTACGGTATGGACGTCTGGGGGCCAAGGGACCA CGGTCACCGTCTCCTCA |

FIGURE 50

| | | | SEQ ID NO: 27841 | SEQ ID NO: 31847 |
|---|---|---|---|---|
| | | AA | DIQMTQSPSSLSASVGDRVTITCRASQGISNYLA WFQQKPGKAPKSLISAASSLQSGVPSKFSGSGSG TDFTLTISSLQPEDFATYYCQQYNSYPLSFGGGT KVEIK | QVHLVESGGGVVQPGRSLRLSCAASGFTISTYGMH WVRQGPGKGLEWVAVIWYGGSDTDYADSVRGRF TISRDISKNTLYLQMNSLRAEDTAVYYCARDRDYC SGGNCPYYYYYGMDVWGQGTTVTVSS |
| | | | SEQ ID NO: 27842 | SEQ ID NO: 31848 |
| iPS:437260 | 21-225_170D1 | NA | GACATCCAGATGACCCAGTCTCCATCCTCACT GGCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAGGTTCAGCGGCA GTGGATCTGGGACAGATTTCACTCTCACCATC AGCAGCCTGCAGCCTGAAGATTTTGCAACTTA TTACTGCCAACAGTGTGATAGTTTCCCTCTCAC TTTCGGCGGAGGGACCAAGGTGGAGATCAAA | CAGGTGCAGTTGGTGCAGTCTGGGGGCTGAGGTGA AGAAGCCTGGGGCCTCAGTGAAGGTCTCCTGCA AGGCTTCTGGATACACCTTCACCGGCTACTTTAT GCACTGGGTGCGACAGGCCCCTGGACAAGGGCT TGAGTGGATGGGATGGATCAAGCCTAAAAGCGG TGGCACAAACTGTGCACAGAAGTTTCAGGGCAG GGTCACCATGACCAGGGACACGTCCAGCAGCAC AGCCTACATGGAGCTGAGCAGGCTGACATCTGA CGACACGGCCGTGTATTACTGTGCGAGAGGGGG GGCTACGGTGACTACGTGGGGGGGTCTTTGACTAC TGGGGCCAGGGAACCCTGGTCACCGTCTCCTCA |
| | | | SEQ ID NO: 27843 | SEQ ID NO: 31849 |
| | | AA | DIQMTQSPSSLAASVGDRVTITCRASQDISNYLA WFQQKPGKAPKSLIYAASSLQSGVPSRFSGSGSG TDFTLTISSLQPEDFATYYCQQCDSFPLTFGGGT KVEIK | QVQLVQSGAEVKKPGASVKVSCKASGYTFTGYFM HWVRQAPGQGLEWMGWIKPKSGGTNCAQKFQGR VTMTRDTSSSTAYMELSRLTSDDTAVYYCARGGA TVTTWGVFDYWGQGTLVTVSS |
| | | | SEQ ID NO: 27844 | SEQ ID NO: 31850 |

FIGURE 50

| | | | | |
|---|---|---|---|---|
| iPS:437262 | 21-225_170E4 | NA | GACATCCAGATGACCCAGTCTCCATCCTCCCT GTCTGCATCGTAGGAGACAGAGTCACCATCA CTTGCCGGGCAAGTCAGGGCATTAGAAATGAT TTAGGCTATTATCAGCAGAAACCAGGGAAAGC CCCTAAGCGCCTGATCTATGTTGCATCCGGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCACAATAGTTACCCTCCGT GGACGTTCGGCCAAGGGACCAAGGTGGATAT CAAA | GAGGTGCAACTGGTGGAGTCTGGGGGAGGCTCG GTACAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTTTCATACATTAGCAGTAGTGGTAGT ACCAAATACTACGCAGACTCTGTGGAGGGCCGA TTCACCATCTCCAGAGACAATGCCAAGAATTCAC TGGATCTGCAAATGAACAGCCTGAGAGACGAGG ACACGGCTGTGTATCGCTGTGCGAGAGATAGTAG GAAGGGGTTCTACTACGGTCTGGACGTCTGGGGC CAAGGGACCACGGTCACCGTCTCCTCA |
| | | | SEQ ID NO: 27845 | SEQ ID NO: 31851 |
| | | AA | DIQMTQSPSSLSASVGDRVTITCRASQGIRNDLG YYQQKPGKAPKRLIYVASGLQSGVPSRFSGSGS GTEFTLTISSLQPEDFATYYCLQHNSYPPWTFGQ GTKVDIK | EVQLVESGGGSVQPGGSLRLSCAASGFTFSSYSMN WVRQAPGKGLEWVSYISSSGSTKYYADSVEGRFTI SRDNAKNSLDLQMNSLRDEDTAVYRCARDSRKGF YYGLDVWGQGTTVTVSS |
| | | | SEQ ID NO: 27846 | SEQ ID NO: 31852 |
| iPS:437264 | 21-225_171H12 | NA | GACATCCAGATGACCCAGTCTCCATCCTCACT GTCTGCATCGTAGGAGACAGAGTCACCATCA CTTGTCGGGCGAGTCAGGACATTAGCAATTAT TTAGCCTGGTTTCAGCAGAAGCCAGGGAAAGC CCCTAAGTCCCTGATCTATTCTGCATCCAGTTT GCAAAGTGGGGTCCCATCAAGGTTCAGCGGCA GTGGATCTGGGACAGATTTCACTCTCACCATC AGCAGCCTGCAGCCTGAAGATTTTGCAACTTA TTACTGCCAACAATCTGATAGTTACCCTCTCAC TTTCGGCGGAGGGACCAAGGTGGAGATCAAA | CAGGTGCAGCTGGTGCAGTCTGGGGCTGAGGTG AAGAAGCCTGGGGCCTCAGTGAGGGTCTCCTGC AAGGCTTCTGGATACACCTTCACCGGCTACTTTA TGCACTGGGTGCGACAGGCCCCTGGACAAGGGC TTGAGTGGATGGGATGGATCAAGCCTAAGAGTG GTGGCACAAACTCTGCACAGAGGTTTCAGGGCA GGGTCACCATGACCAGGGACACGTCCATCAACA CAGCCTACATGGAGCTGAACAGGCTGAGATCTG ACGACACGGCCGTATATTACTGTGCGAGAGGGG GGACTACGGTGGCTACGTGGGGGGGTCTTTGACTA CTGGGGCCAGGGAACCCTGGTCACCGTCTCCTCA |
| | | | SEQ ID NO: 27847 | SEQ ID NO: 31853 |

FIGURE 50

| | | | | |
|---|---|---|---|---|
| | | AA | DIQMTQSPSSLSASVGDRVTITCRASQDISNYLA WFQQKPGKAPKSLIYSASSLQSGVPSKFSGSGSG TDFTLTISSLQPEDFATYYCQQSDSYPLTFGGGT KVEIK | QVQLVQSGAEVKKPGASVRVSCKASGYTFTGYFM HWVRQAPGQGLEWMGWIKPKSGGTNSAQRFQGR VTMTRDTSINTAYMELNRLRSDDTAVYYCARGGT TVATWGVFDYWGQGTLVTVSS |
| | | | SEQ ID NO: 27848 | SEQ ID NO: 31854 |
| iPS:437266 | 21-225_177A5 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTAGCCTGGTTTCAGCAGAAGCCAGGGAAAGC CCCTAAGTCCCTGATCTATTCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAATCTGATAGTTACCCTCTCAC TTTCGGCGGAGGGACCAAGGTGGAGATCAAA | CAGGTGCAGCTGGTACAGTCTGGGGGCTGAGGTG AAGAAGCCTGGGGCCTCAGTGCGGGTCTCCTGCA AGGCTTCTGGATACACCTTCACCGGCTACTTTAT GCACTGGGTGCGACAGGCCCCTGGACAAGGGCT TGAGTGGATGGGATGGATCAAGCCTAAGAGTGG TGGCACAAACTCTGCACAGAGGTTTCAGGGCAG GGTCACCATGACCAGGGACACGTCCATCAACAC AGCCTACATGGAGCTGAACTGGCTGAGATCTGAC GACACGGCCGTATATTACTGTGCGAGAGGGGGG ACTACGGTGGCTACGTGGGGGGTCTTTGACTACT GGGGCCAGGGAACCCTGGTCACCGTCTCCTCA |
| | | | SEQ ID NO: 27849 | SEQ ID NO: 31855 |
| | | AA | DIQMTQSPSSLSASVGDRVTITCRASQDISNYLA WFQQKPGKAPKSLIYSASSLQSGVPSKFSGSGSG TDFTLTISSLQPEDFATYYCQQSDSYPLTFGGGT KVEIK | QVQLVQSGAEVKKPGASVRVSCKASGYTFTGYFM HWVRQAPGQGLEWMGWIKPKSGGTNSAQRFQGR VTMTRDTSINTAYMELNWLRSDDTAVYYCARGGT TVATWGVFDYWGQGTLVTVSS |
| | | | SEQ ID NO: 27850 | SEQ ID NO: 31856 |

FIGURE 50

| iPS:437268 | 21-225_177D2 | NA | GATGTTGTGATGACTCAGTCTCCACTCTCCCTG CCCGTCACCCTTGGACAGCCGGCCTCCATCTC CTGCAGGTCTAGTCAAAGCCTCGTATACAGTG ATGGAAACACCTACTTGAATTGGTTTCAGCAG AGGCCAGGCCAATCTCCAAGGCGCCTAATTTA TAAGGTTTCTAACTGGGACTCTGGGGTCCCAG ACAGATTCAGCGGCAGTGGGTCAGGCACTGAT TTCACACTGAAAATCAGCAGGGTGGAGGCTGA GGATGTTGGGGTTTATTACTGCATGCAAGGTA CACACTGGCCTCTCACTTTCGGCGGAGGGACC AAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GGACTGGGTCCGCCAGGCTCCAGGCAAGGGGCT GGAGTGGGTGGCAATTATATGGTTTGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGCATATTG TGGTGGTGACTGCTATTTCCCCCATCTCCATTACT ACGGTATGGACGTCTGGGGCCAAGGGACCACGG TCACCGTCTCCTCA |
| | | | SEQ ID NO: 27851 | SEQ ID NO: 31857 |
| | | AA | DVVMTQSPLSLPVTLGQPASISCRSSQSLVYSDG NTYLNWFQQRPGQSPRRLIYKVSNWDSGVPDRF SGSGSGTDFTLKISRVEAEDVGVYYCMQGTHWP LTFGGGTKVEIK | QVQLVESGGGVVQPGRSLRLSCAASGFTFSSYGMD WVRQAPGKGLEWVAIIWFDGSNKYYADSVKGRFT ISRDNSKNTLYLQMNSLRAEDTAVYYCARAYCGG DCYFPHLHYYGMDVWGQGTTVTVSS |
| | | | SEQ ID NO: 27852 | SEQ ID NO: 31858 |
| iPS:437270 | 21-225_178H4 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTTTTCTGCATCCAGTTT GCAAAGTGGGGTCCCATCAAAGTTCAGCGGCA GTGGATCTGGGACAGATTTCACTCTCACCATC AGCAACCTGCAGCCTGAAGATTTTGCAACTTA TTACTGCCAACAATCTAATAGTTACCCTCTCAC TTTCGGCGGAGGGACCAAGGTGGAGATCAAA | CAGGTACAGCTGGTGCAGTCTGGGGGCTGAGGTG AAGAAGCCTGGGGCCTCAGTGAAGGTCTCCTGC AAGGCTTCTGGATACACCTTCACCGGCTACTTTA TGCACTGGGTGCGACAGGCCCCTGGACAAGGGC TTGAGTGGATGGGATGGATCAAGCCTAAAAGTG GTGGCACAAACTGTGCACAGAAGTTTCAGGGCA GGGTCACCATGACCAGGGACACGTCCATCAGCA CAGCCTACATGGAACTGAGCAGGCTGAGATCTG ACGACACGGCCGTGTATTATTGTGTGAGAGGGGG GACTACGGTGACTACGTGGGGGGTCTTTGACTAC TGGGGCCAGGGAACCATGGTCACCGTCTCCTCA |

FIGURE 50

| | | | SEQ ID NO: 27853 | SEQ ID NO: 31859 |
|---|---|---|---|---|
| | | AA | DIQMTQSPSSLSASVGDRVTITCRASQDISNYLA WFQQKPGKAPKSLIFSASSLQSGVPSKFSGSGSG TDFTLTISNLQPEDFATYYCQQSNSYPLTFGGGT KVEIK | QVQLVQSGAEVKKPGASVKVSCKASGYTFTGYFM HWVRQAPGQGLEWMGWIKPKSGGTNCAQKFQGR VTMTRDTSISTAYMELSRLRSDDTAVYYCVRGGTT VTTWGVFDYWGQGTMVTVSS |
| | | | SEQ ID NO: 27854 | SEQ ID NO: 31860 |
| iPS:437274 | 21-225_196D4 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAACCCAGGGAAAGC CCCTAAGTCCCTTATCTATGCTGCATCCAGTTT GCAAAGTGGGGTCCCATCAAAGTTCAGCGGCA GTGGATGTGGGACAGATTTCACTCTCACCATC AGCAGCCCGCAGCCTGAAGATGTTGCAACCTA TTACTGCCAACATTATCTTAATTACCCTCTCAC CTTCGGCCAAGGGACACGACTGGAGATTAAA | CAGGTGCAGGTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAGAAACTATGCAGACTCCGTGAAGGGCCGA TTCACCATCTCCAGAGACAATTCCAAGAACACGC TGTATCTGCAAATGAACAGCCTGAGAGTCGAGG ACACGGCTGTGTATTACTGTGCGAGAGATCGGTC TAAGGGTTACGACGGTATGGACGTCTGGGGCCA AGGGACCACGGTCACCGTCTCCTCA |
| | | | SEQ ID NO: 27855 | SEQ ID NO: 31861 |
| | | AA | DIQMTQSPSSLSASVGDRVTITCRASQGISNYLA WFQQNPGKAPKSLIYAASSLQSGVPSKFSGSGCG TDFTLTISSPQPEDVATYYCQHYLNYPLTFGQGT RLEIK | QVQVVESGGGVVQPGRSLRLSCAASGFTFSSYGMH WVRQAPGKGLEWVAVIWYDGSNRNYADSVKGRF TISRDNSKNTLYLQMNSLRVEDTAVYYCARDRSKG YDGMDVWGQGTTVTVSS |
| | | | SEQ ID NO: 27856 | SEQ ID NO: 31862 |

FIGURE 50

| | | | | |
|---|---|---|---|---|
| iPS:437280 | 21-225_203C10 | NA | GACATCCAGATGACCCAGTCTCCATCCTCCCT GTCTGCATCTGTAGGAGACAGAGTCACCATCA CTTGCCGGGCAAGTCAGGACATTAGAAATGAT TTAGGCTGGTATCAGCAGAAACCAGCGAAAG CCCCTAAGCGCCTGATCTATAGAGCATCCAGT TTGCAAAGTGGGGTCCCATCACGCTTCAGCGG CAGTGGATCTGGGACAGAATTCACTCTCACAA TCAGCAGCCTGCAGCCTGAAGATTTTGCCGCT TATTACTGTCTACAGCATAATAGTTACCCGTG GACGTTCGGCCAAGGGACCAAGGTAGAGATC AAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGGCAT GCACTGGGTCCGCCAGGCTCCAGGCAAGGGGCT GGAGTGGGTGGCAGTTATATGGTATGATGGAGGT AATACACATTATACAGACTCCGTGAAGGGCCGAT TCACCATCTCCAGAGACAATTCCAAGAACACGCT GTATCTGCAAATGAACAGCCTGAGAGCCGAGGA CACGGCTGTATATTACTGTGCGAGAGAAGTGGGT TGGCTTGATGACTACTGGGGCCAGGGAACCCTGG TCACCGTCTCCTCA |
| | | | SEQ ID NO: 27857 | SEQ ID NO: 31863 |
| | | AA | DIQMTQSPSSLSASVGDRVTITCRASQDIRNDLG WYQQKPAKAPKRLIYRASSLQSGVPSRFSGSGS GTEFTLTISSLQPEDFAAYYCLQHNSYPWTFGQG TKVEIK | QVQLVESGGGVVQPGRSLRLSCAASGFTFSDYGM HWVRQAPGKGLEWVAVIWYDGGNTHYTDSVKGR FTISRDNSKNTLYLQMNSLRAEDTAVYYCAREVG WLDDYWGQGTLVTVSS |
| | | | SEQ ID NO: 27858 | SEQ ID NO: 31864 |
| iPS:437282 | 21-225_207C9 | NA | GACAACCAGATGACCCAGTCTCCATCCTCCCT GTCTGCATCTGTAGGAGACAGAGTCACCATCA CTTGCCGGGCAAGTCAGAGGTTTAGTAACTAT TTAAATTGGTATCAGCAGAAACCAGGGAAAG CCCCTAAGCTCCTGATCTATACTGCATCCAGTT TGCAAAGTGGGGTCCCATCAAGGTTCAGTGCC AGTGTCTCTGGGACAGACTTCACTCTCACCAT CAGCAGTCTGCAACCTGAAGATTTTGCAACTT ATTACTGTCAACAGAGTTACAGTATTCCGCTC ACTTTCGGCGGAGGGACCAAGGTGGAGATC AA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAAGCTGGTGG AACTACGGGGAGCTACTACTACAACGGTATGGA CGTCTGGGGCCAAGGGACCACGGTCACCGTCTCC TCA |
| | | | SEQ ID NO: 27859 | SEQ ID NO: 31865 |

FIGURE 50

| | | | | |
|---|---|---|---|---|
| | | AA | DNQMTQSPSSLSASVGDRVTITCRASQRFSNYLN WYQQKPGKAPKLLIYTASSLQSGVPSRFSASVSG TDFTLTISSLQPEDFATYYCQQSYSIPLTFGGGTK VEIK | EVQLLESGGGLVQPGGSLRLSCAASGFTFSSYAMS WVRQAPGKGLEWVSAISGSGGSTYYADSVKGRFTI SRDNSKNTLYLQMNSLRAEDTAVYYCAKAGGTTG SYYYNGMDVWGQGTTVTVSS |
| | | | SEQ ID NO: 27860 | SEQ ID NO: 31866 |
| iPS:437286 | 21-225_208F1 | NA | GACATCCAGATGACCCAGTCTCCATCCTCCCT GTCTGCATCTGTAGGAGACAGAGTCACCATCA CTTGCCGGGCAAGTCAGGGCATTAGACATGAT TTAGGCTGGTATCAGCAGAAACCAGGGAAAG CCCCTAAGCGCCTGATCTTTGCTGCATCCAGTT TGCAAAGTGGGGTCCCATCTAGATTCAGCGGC AGTGGATCTGGGACAGAATTCACTCTCACAAT CAGCAGCCTGCAGCCTGAAGATTTTGCAACTT ATTACTGTCTACAGCATTATAGTTTCCCTCGGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTAGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAGGAACACGCT GTATCTGCAAATGAACAGCCTGAGAGCCGAGGA CACGGCTGTATATTACTGTGCGAGAGAACGGTAT AGCAGTGGCTTGTACGACTACGGTATGGACGTCT GGGGCCAAGGGACCACGGTCACCGTCTCCTCA |
| | | | SEQ ID NO: 27861 | SEQ ID NO: 31867 |
| | | AA | DIQMTQSPSSLSASVGDRVTITCRASQGIRHDLG WYQQKPGKAPKRLIFAASSLQSGVPSRFSGSGSG TEFTLTISSLQPEDFATYYCLQHYSFPRTFGQGTK VEIK | QVQLVESGGGVVQPGRSLRLSCAASRFTFSDYVMH WVRQAPGKGLEWVAVIWYDGSNKYYVDSVKGRF TISRDNSRNTLYLQMNSLRAEDTAVYYCARERYSS GLYDYGMDVWGQGTTVTVSS |
| | | | SEQ ID NO: 27862 | SEQ ID NO: 31868 |

FIGURE 50

| iPS:437290 | 21-225_210G6 | NA | GACATCCAGATGACCCAGTCTCCATCCTCCCG TTTTGCATTTGTAGGAGACAGAGTCACCATCA CTTGCCGGGCAAGTCAGGGCATTAGACATGAT TTAGGCTGGTATCAGCAGAAACCAGGGAAAG CCCTTAAGCGCTTGATATATGCTGCATCCAGTT CGCAAAGTGGGGTCCCATCAAGGTTCAGCGGC AGTGGATGTGGGACAGAATTCACTCTCACAAT CAGCAGCGTGCAGCGCGAAGATTTTGCAAATT ATTACTGTGTACAGCATTATAGTTTCCCTCGGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAGGAACACGCT GTATCTGCAAATGAACAGCCTGAGAGCCGAGGA CACGGCTGTGTATTACTGTGCGAGAGAACGGTAT AGCAGTGGCTTGTACGACTACGGTATGGACGTCT GGGGCCAAGGGACCACGGTCACCGTCTCCTCA |
| | | | SEQ ID NO: 27863 | SEQ ID NO: 31869 |
| | | AA | DIQMTQSPSSRFAFVGDRVTITCRASQGIRHDLG WYQQKPGKALKRLIYAASSSQSGVPSRFSGSGC GTEFTLTISSVQREDFANYYCVQHYSFPRTFGQG TKVEIK | QVQLVESGGGVVQPGRSLRLSCAASGFTFSDYVM HWVRQAPGKGLEWVAVIWYDGSNKYYVDSVKGR FTISRDNSRNTLYLQMNSLRAEDTAVYYCARERYS SGLYDYGMDVWGQGTTVTVSS |
| | | | SEQ ID NO: 27864 | SEQ ID NO: 31870 |
| iPS:437294 | 21-225_216D5 | NA | GATGTTGTGATGACTCAGTCTCCACTCTCCCTG CCCGTCACCCTTGGACAGCCGGCCTCCATCTC CTGCAGGTCTAGTCAAAGCCTCGTATACAGTG ATGGAAACACCTACTTGAATTGGTTTCAGCAG AGGCCAGGCCAATCTCCAAGGCGCCTCATTTA TAAGGTTTCTAACTGGGACTCTGGGGTCCCAG ACAGAATCAGCGGCAGTGGGTCAGGCACTGA TTTCACACTGAAAATCAGCAGGGTGGAGGCTG AGGATGTTGGGATTTATTACTGCATGCAAGGT GCACACTGGTTCACCTTCGGCCAAGGGACACG ACTGGAGATTAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGTAGACACGTCTAAGAACCA ATTCTCCCTGAAACTGAACTCTGTGACTGCCGCG GACACGGCCGTGTATTTCTGTGCGAGAGATTCCC CTGACAGGGGGTTTGACTACTGGGGCCAGGGAA CCCTGGTCACCGTCTCCTCA |
| | | | SEQ ID NO: 27865 | SEQ ID NO: 31871 |

FIGURE 50

| | | | | |
|---|---|---|---|---|
| | | AA | DVVMTQSPLSLPVTLGQPASISCRSSQSLVYSDG NTYLNWFQQRPGQSPRRLIYKVSNWDSGVPDRI SGSGSGTDFTLKISRVEAEDVGIYYCMQGAHWF TFGQGTRLEIK | QVQLQESGPGLVKPSQTLSLTCTVSGGSISSGGYYW SWIRQHPGKGLEWIGYIYYSGSTYYNPSLKSRVTIS VDTSKNQFSLKLNSVTAADTAVYFCARDSPDRGFD YWGQGTLVTVSS |
| | | | SEQ ID NO: 27866 | SEQ ID NO: 31872 |
| iPS:437302 | 21-225_225B11 | NA | GACATCCAGATGACCCAGTCTCCATCCTCCCT GTCTGCATCTATAGGAGACAGAGTCACCATCA CTTGCCAGGCGAGTCAGGACATTTTCAACTAT TTAAATTGGTATCAGCAGAAACCAGGGAAAG CCCCTAAGCTCCTGATCTACGATGCATCCACTT TGGAAACAGGGGTCCCATCAAGGTTCAGTGGA AGTGGATCTGGGACAGATTTTACTTTCACCAT CAGCAGCCTGCAGCCTGAAGATATTGCAACAT ATTACTGTCAACAGTATGATAATCTCCCGATC ACCTTCGGCCAAGGGACACGACTGGAGATTAA A | CAGGTGCAGCTGGTGGAATCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGACAATTATATCATATAGTGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGCGAGACGGGGATA CAGCTATGGCGGGTACGGTATGGACGTCTGGGG CCAAGGGACCACGGTCACCGTCTCCTCA |
| | | | SEQ ID NO: 27867 | SEQ ID NO: 31873 |
| | | AA | DIQMTQSPSSLSASIGDRVTITCQASQDIFNYLNW YQQKPGKAPKLLIYDASTLETGVPSRFSGSGSGT DFTFTISSLQPEDIATYYCQQYDNLPITFGQGTRL EIK | QVQLVESGGGVVQPGRSLRLSCAASGFTFSSYGMH WVRQAPGKGLEWVTIISYSGSNKYYADSVKGRFTI SRDNSKNTLYLQMNSLRAEDTAVYYCARRGYSYG GYGMDVWGQGTTVTVSS |
| | | | SEQ ID NO: 27868 | SEQ ID NO: 31874 |

FIGURE 50

| iPS:437320 | 21-225_75A1 | NA | GATATTGTGATGACTCAGTCTCCACTCTCCCTG CCCGTCACCCCTGGAGAGCCGGCCTCCATCTC CTGCAGGTCTAGTCAGAGCCTCCTGCATAGTA ATGGACACAACTATTTGGATTGGTACCTACAG AAGCCAGGGCGGTCTCCACAGCTCCTGATCTA TTTGGGTTCTAATCGGGCCTCCGGGGTCCCTG AGAGGTTCAGTGGCAGTGGATCAGGCACAGA TTTTACACTGAAAATCAGCAGAGTGGAGGCTG AGGATGTTGGGGTTTATTACTGCATGCAACCT CTACAAACTCCGTTCACTTTCGGCGGAGGGAC CAAGGTGGAGATCAAA | CAGGTGCAGGTACAGCAGTGGGGCGCAGGACTG TTGAAGCATTCGGAGACCCTGTCCCTCACCTGCG CTGTCTATGGTGGGTCCTTCACTGATTACTACTGG AGCTGGATCCGCCAGCCCCCAGGGAAGGGGCTG GAGTGGATTGGGGAAATCAATCATAGTGGAGAC ACCAACTACAACCCGTCCCTCAAGAGTCGAGTCA CCATATCAGTAGACACGTCCAAGAACCAGTTCTC CCTGAAGCTGAGTTCTGTGACCGCCGCGGACACG GCTGTGTATTACTGTGCGAGAGAGTTTCCATATA GTGGAAGCTACCTCTACTACTACGGTATGGACGT CTGGGGCCAAGGGACCACGGTCACCGTCTCCTCA |
| | | | SEQ ID NO: 27869 | SEQ ID NO: 31875 |
| | | AA | DIVMTQSPLSLPVTPGEPASISCRSSQSLLHSNGH NYLDWYLQKPGRSPQLLIYLGSNRASGVPERFS GSGSGTDFTLKISRVEAEDVGVYYCMQPLQTPF TFGGGTKVEIK | QVQVQQWGAGLLKHSETLSLTCAVYGGSFTDYYW SWIRQPPGKGLEWIGEINHSGDTNYNPSLKSRVTIS VDTSKNQFSLKLSSVTAADTAVYYCAREFPYSGSY LYYYGMDVWGQGTTVTVSS |
| | | | SEQ ID NO: 27870 | SEQ ID NO: 31876 |
| iPS:437322 | 21-225_75B1 | NA | GAATTTATGTTGACGCAGTCTCCAGGCACCCT GTATTGGTCTCCAGGGGAAAGAGCCACCATCT CGAGCAGGGCCAGTCAGAGTGTTAGCAGCAG CTACTTAGTCTGGTATCAGCAGAAACCTGGCC AGGCTCCCAGGCTCCTCATCTATGGTGCATCC ACCAGGGCCACTGGCATCCCAGACAGGTTCAG TGGCAGTGGGTCTGGGACAGACTTCACTCTCA CCATCAGCAGACTGGAGCCTGAATATTTTGCA GTTTATTACTGTCAGCAGTATGGTTGCTCACCG CTCACTTTCGGCGGAGGGACCAAGGTGGAGAT CACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
| | | | SEQ ID NO: 27871 | SEQ ID NO: 31877 |

FIGURE 50

| | | | | |
|---|---|---|---|---|
| | | AA | EFMLTQSPGTLYWSPGERATISSRASQSVSSSYL VWYQQKPGQAPRLLIYGASTRATGIPDRFSGSGS GTDFTLTISRLEPEYFAVYYCQQYGCSPLTFGGG TKVEIT | QVQLVQSGAEVKKPGASVKVSCKASGYTFTNYDIN WVRQVPGQGLEWMGWMHPNSGNTGYAQKFQGR VTMTRNTSISTAYMELSSLRSEDTAVYYCAISSGW YWFDPWGQGTLVTVSS |
| | | | SEQ ID NO: 27872 | SEQ ID NO: 31878 |
| IPS:437324 | 21-225_75C2 | NA | GAAATTGTGTTGACGCAGTCTCCAGGCACCCG GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATCTATGGTGCATCCA GCCGGGCCACTGGCATCCCAGACAGGTTCAGT GGCAGTGGGTCTGGGACAGACTTCGCTCTCAC CATCAGCAGACTGGAGCCTGAAGATTGTGCAG TGTATTACTGTCAGCACTATGATAACTCACCG TGGACGTTCGGCCGAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 27873 | SEQ ID NO: 31879 |
| | | AA | EIVLTQSPGTRYLSPGERATLSCRASQSVYSSYL AWYQQKPGQAPRLLIYGASSRATGIPDRFSGSGS GTDFALTISRLEPEDCAVYYCQHYDNSPWTFGR GTKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 27874 | SEQ ID NO: 31880 |

FIGURE 50

| | | | | |
|---|---|---|---|---|
| iPS:437326 | 21-225_75C10 | NA | GACATCCAGATGACCCAGTCTCCGTCTTCCGT GTCTGCTTCTGTAGGAGACAGAGTCATCATCA CTTGTCGGGCGAGTCAGGGCATTAGCATCTGG TTAGCCTGGTATCAGCAGAAACCAGGGAAAG CCCCTAAGCTCCTGATCTATGCTGCATCCAGTT TGCAAAGTGGGGTCCCATTAAGGTTCAGCGGC AGTGGATCTGGGACAGATTTCACTCTCACCAT CAGCAGCCTGCAGCCTGAAGATTTTGCAACTT ACTATTGTCAACAGGCTAAAAGTTTCCCGCTC ACTTTCGGCGGAGGGACCAAGGTGGAGATCA AA | CAGGTGCAACTGGTGGAGTCTGGGGGAGGCGTG GTCCAGCCTGGGAGGTCCCTGAGACTCTCCTGTG CAGCGTCTGGATTCACCTTCAGTAGTTATGGCAT GCATTGGGTCCGCCAGGCTCCAGGCAAGGGGCT GGAGTGGGTGGCAGTTATATGGTATGATGGAAGT GATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATCGGTT AGTGGGAGCTACGGTTGATGCTTTTGATATCTGG GGCCAAGGGACAATGGTCACCGTCTCTTCA |
| | | | SEQ ID NO: 27875 | SEQ ID NO: 31881 |
| | | AA | DIQMTQSPSSVSASVGDRVIITCRASQGISIWLAW YQQKPGKAPKLLIYAASSLQSGVPLRFSGSGSGT DFTLTISSLQPEDFATYYCQQAKSFPLTFGGGTK VEIK | QVQLVESGGGVVQPGRSLRLSCAASGFTFSSYGMH WVRQAPGKGLEWVAVIWYDGSDKYYADSVKGRF TISRDNSKNTLYLQMNSLRAEDTAVYYCARDRLVG ATVDAFDIWGQGTMVTVSS |
| | | | SEQ ID NO: 27876 | SEQ ID NO: 31882 |
| iPS:437328 | 21-225_75D3 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCATCATTTATGGTGCATCCA GCCGGTCCACTGGCATACCAGACAGGTTCAGT GGCAGTGGGTGTGGGACAGACTTCGCTCTCAC CATCAGCAGAGTGGAGCATGAAGATTGTGCA GTGTATTACTGTCAGCACTATGATAACTCACC GTGGACGTTCGGCCAAGGGACCAAGGTGGAA ATCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 27877 | SEQ ID NO: 31883 |

FIGURE 50

| | | | | |
|---|---|---|---|---|
| | | AA | EIVLTQSPGTLYLSPGERATLSCRASQSVYSSYLA WYQQKPGQAPRLIIYGASSRSTGIPDRFSGSGCG TDFALTISRVEHEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 27878 | SEQ ID NO: 31884 |
| IPS:437332 | 21-225_75F3 | NA | GAAATTGTGTTGACGCAGTCTCCAGGCACCCT GTATTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTGTGGGACAGACTTCGCTCTCAC CATCAGCAGACTGGAGCATGAAGATTTTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 27879 | SEQ ID NO: 31885 |
| | | AA | EIVLTQSPGTLYLSPGERATLSCRASQSVYSSYLA WYQQKPGQAPRLLIYGASSRATGIPDRFSGSGCG TDFALTISRLEHEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 27880 | SEQ ID NO: 31886 |

FIGURE 50

| iPS:437334 | 21-225_75F11 | NA | GAAATAGTGATGACGCAGTCTCCAGCCACCCT GTTTGTGTCTCCAGGGGAAAGAGCCACCCTCT CCTGCAGGGCCAGTCAGAGTGTTAGCAGAAAT TTAGCCTGGTTCCAGCAGAAACCTGGCCAGGC TCCCAGGCTCCTCTTTTATGGTGCATCCATCAG GGCCACTGGTATCCCAGCCAGGTTCAGTGGCA GTGGGTCTGGGACAGAGTTCACTCTCACCATC TACAGCCTGCAGTATGAAGATTTTGCAGTTTA TTACTGTCAGCAGTATAATAACTGGCCTCCGC TCACTTTCGGCGGAGGGACCAAGGTGGAGATC AAA | CAGATCACCTTGAAGGAGTCTGGTCCTACGCTGG TGAAACCCACACAGACCCTCACGCTGACCTGCAC CTTCTCTGGGTTCTCACTCAGCACTGGTGGAGTG GGTGTGGGCTGGATCCGTCAGCCCCCAGGAAAG GCCCTGGAGTGGCTTGCACTCATTTATTGGGATG ATGATAAGCGCTACAGCCCATCTCTGAAGAGCA GGCTCACCATCACCAAGGACACCCCCAAAAACC AGGTGGTCCTTACAATGACCAACATGGACCCTGT GGACACAGCCACATATTACTGTGCACACCTTATA GCAGTGGCCTTTGACTACTGGGGCCAGGGAACCC TGGTCACCGTCTCCTCA |
| | | | SEQ ID NO: 27881 | SEQ ID NO: 31887 |
| | | AA | EIVMTQSPATLFVSPGERATLSCRASQSVSRNLA WFQQKPGQAPRLLFYGASIRATGIPARFSGSGSG TEFTLTIYSLQYEDFAVYYCQQYNNWPPLTFGG GTKVEIK | QITLKESGPTLVKPTQTLTLTCTFSGFSLSTGGVGVG WIRQPPGKALEWLALIYWDDDKRYSPSLKSRLTIT KDTPKNQVVLTMTNMDPVDTATYYCAHLIAVAFD YWGQGTLVTVSS |
| | | | SEQ ID NO: 27882 | SEQ ID NO: 31888 |
| iPS:437340 | 21-225_75G9 | NA | GAAATTGCGTTGACGCAGTCTCCAGGCACCCT GTCTTTGTCTCCAGGGGAAAGAGCCACCCTCT CCTGCAGGGCCAGTCCGAGTGTTGACAGCAGC TACTTAGCCTGGTACCAGCAGAAACCTGGCCA GGCTCCCAGGCTCCTCATCTATGGTGCATCCA GCAGGGCCCCTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGAAAGTTCACCG TGGACGTTCGGCCAAGGGACCAAGGTGGAAA TCAAA | CAGGTGCAGCTACAGCAGTGGGGCGCAGGACTG TTGAAGCCTTCGGGAGACCCTGTCCCTCACCTGCG CTGTCTATGGTGGGTCCTTCAGTGGTTGCTACTG GAGCTGGATCCGCCAGCCCCCAGGGAAGGGGCT GGAGTGGATTGGGGAAATCAATCATAGTGGAAG GACCAACTACAACCCGTCCCTCAAGAGTCGAGTC ACCATATCAGTAGACACGTCCGAGAACCAGTTCT CCCTGAAGCTGAGCTCTGTGACCGCCGCGGACAC GGCTGTGTATTACTGTGCGAGAGACTACGGTGGG CTTGACTACTGGGGCCAGGGAACCCTGGTCACCG TCTCCTCA |
| | | | SEQ ID NO: 27883 | SEQ ID NO: 31889 |

FIGURE 50

| | | | | |
|---|---|---|---|---|
| | | AA | EIALTQSPGTLSLSPGERATLSCRASPSVDSSYLA WYQQKPGQAPRLLIYGASSRAPGIPDRFSGSGSG TDFTLTISRLEPEDFAVYYCQQYESSPWTFGQGT KVEIK | QVQLQQWGAGLLKPSETLSLTCAVYGGSFSGCYW SWIRQPPGKGLEWIGEINHSGRTNYNPSLKSRVTIS VDTSENQFSLKLSSVTAADTAVYYCARDYGGLDY WGQGTLVTVSS |
| | | | SEQ ID NO: 27884 | SEQ ID NO: 31890 |
| IPS:437344 | 21-225_75G12 | NA | GAAATTGTGTTGACGCAGTCTCCAGGCACCCT GTATTTGTCTCCAGGGGAAAGAGCCACCCTGT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTCTGGGACAGACTTCGCTCTCAC CATCAGCAGAGTGGAGCCTGAAGATTGTGCAG TGTTATTACTGCCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 27885 | SEQ ID NO: 31891 |
| | | AA | EIVLTQSPGTLYLSPGERATLSCRASQSVYSSYLA WYQQKPGQAPRLLIYGASSRATGIPDRFSGSGSG TDFALTISRVEPEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 27886 | SEQ ID NO: 31892 |

FIGURE 50

| | | | | |
|---|---|---|---|---|
| iPS:437346 | 21-225_75H7 | NA | GACATTCAGATGACCCAATCTCCATCCTCCCG GTATGCATCTGTAGGAGACAGAGTCACCATCA ATAGCCGGGCAAGTCAGGGCATAAGAAATGA TTTAGGCTGGTATCAACAGAAACCAGGGAAAT CCCCTCAGCGCCTGATTTATGATGCATCCAGTT TGCAAAGTGGGGTCCCATCAAGGTTCAGCGGC AGTGGATCTGGGACAGAATTCACTCTCACAAT CAGCAGCGTGCAGCCTGAAGATTTTGGTGTTT ATTACTGTATACAGCATAGTAATTACCCGCTC ACTTTCGGCGGAGGGACCAAGGTGGAGATCA AA | CAGCTGCAGCTGCAGGAGTCGGGCCCAGGACTG GTGACGCCTTCGGAGACCCTGTCCCTCACCTGCA CTGTCTCTGGCGGCTCCATCAGCAGGAGTAGTTA CTACTGGGGCTGGATCCGCCAGCCCCCAGGGAA GGGGCTGGAGTGGATTGGGAGTATCTATTATAGT GGGAGCGCCTACTCCAACCCGTCCCTCAAGAGTC GAGTCACCATATCCGTAGACACGTCCAAGAACC AGTTCTCCCTGAAGCTGAGCTCTGTGACCGCCGC GGACACGGCTCTGTTTTACTGTGCGAGACTTGAC TCTAACTGGGGTCTTGACTACTGGGGCCAGGGAA CCCTGGTCACCGTCTCCTCA |
| | | | SEQ ID NO: 27887 | SEQ ID NO: 31893 |
| | | AA | DIQMTQSPSSRYASVGDRVTINSRASQGIRNDLG WYQQKPGKSPQRLIYDASSLQSGVPSRFSGSGSG TEFTLTISSVQPEDFGVYYCIQHSNYPLTFGGGTK VEIK | QLQLQESGPGLVTPSETLSLTCTVSGGSISRSSYYW GWIRQPPGKGLEWIGSIYYSGSAYSNPSLKSRVTIS VDTSKNQFSLKLSSVTAADTALFYCARLDSNWGLD YWGQGTLVTVSS |
| | | | SEQ ID NO: 27888 | SEQ ID NO: 31894 |
| iPS:437350 | 21-225_74A3 | NA | GAAATTGTGTTGACGCAGTCTCCAGGCACCCT GTCTTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGTCCACTGGCATCCCAGACAGGTTCAGT GGCAGTGGGTGTGGGACAGACTTCGCTCTCAC CATCAGCAGACTGGAGCCTGAAGATTTTGCAG TGTATTACTGTCAGCACTCTGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 27889 | SEQ ID NO: 31895 |

FIGURE 50

| | | | | |
|---|---|---|---|---|
| | | AA | EIVLTQSPGTLSLSPGERATLSCRASQSVYSSYLA WYQQKPGQAPRLLIYGASSRSTGIPDRFSGSGCG TDFALTISRLEPEDFAVYYCQHSDNSPWTFGQGT KVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 27890 | SEQ ID NO: 31896 |
| iPS:437356 | 21-225_74B1 | NA | GACATCGTGATGACCCAGTCTCCAGACTTCCT GGCTGTGTCTCTGGGCGAGAGGGCCACCACCA ATTGTAAGTCCAGCCAGAGTGTTTTACACAGG TCCAACAATTACAACTACTTAGCGTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTGACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAACTGAGCAGCCTGAGATCTG AGGACACGGCCGTGTATTACTGTGGGAGTACCA GTGGCTGGAACTTCTTTGACTACTGGGGCCAGGG AACCCTGGTCACCGTCTCCTCA |
| | | | SEQ ID NO: 27891 | SEQ ID NO: 31897 |
| | | AA | DIVMTQSPDFLAVSLGERATTNCKSSQSVLHRSN NYNYLAWYQQKPGQPPKLLIYWASTRESGVPD RFSGSGSGTDFTLTISSLQAEDVAVYYCQQYYST PPTFGQGTKVEIK | QVQLVQSGAEVKKPGASVKVSCKASGYTLTNYDI NWVRQATGQGLEWMGWMNPNSGNTGYAQKFQG RVTMTRNTSISTAYMELSSLRSEDTAVYYCGSTSG WNFFDYWGQGTLVTVSS |
| | | | SEQ ID NO: 27892 | SEQ ID NO: 31898 |

FIGURE 50

| iPS:437361 | 21-225_74C1 | NA | GACATCGTGATGACCCAGTGTCCAGACTCCCC GGCTGTGTCTCTGGGCGAGAGGGCCTCCATCA ATTGCAAGTCCAGCCAGAGTATTTTACACAGC TCCAACAATTACAATTACTTAGCTTGGTACCA GCAGAAACCAGGACATCCTCATAAGCTGCTCA TTTACTGGGCATCTACCCGGGAATCCGGGGTC CCTGACCGATTCAGTGGCAGCGGGTATGGGAC AGATTTCACTCTAACCATCAGCAGCCTGCAGG CTGAAGATGTGGCAGTTTATTACTGTCAGCAA TATTATAGTACTCCGTGGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTGACAGTG GTAACACAGGCTTTGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGGTTTCCAG TGGCTGGTACTGGTTCGACCCCTGGGGCCAGGGA ACCCTGGTCACCGTCTCCTCA |
| | | | SEQ ID NO: 27893 | SEQ ID NO: 31899 |
| | | AA | DIVMTQCPDSPAVSLGERASINCKSSQSILHSSNN YNYLAWYQQKPGHPHKLLIYWASTRESGVPDR FSGSGYGTDFTLTISSLQAEDVAVYYCQQYYSTP WTFGQGTKVEIK | QVQLVQSGAEVKKPGASVKVSCKASGYTFTNYDIN WVRQATGQGLEWMGWMNPDSGNTGFAQKFQGR VTMTRNTSISTAYMELSSLRSEDTAVYYCAVSSGW YWFDPWGQGTLVTVSS |
| | | | SEQ ID NO: 27894 | SEQ ID NO: 31900 |
| iPS:437363 | 21-225_74C10 | NA | GACATCGTGATGACCCAGTCTCCAGACTCCCT GGCTGTGTCTCTGGGCGAGAGGGGCCACCATCA ACTGCAAGTCCAGCCAGAGTGTTTTATACAGC TCCAACAATGCGAACTACTTAGCTTGGTACCA GCAGAAACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACATAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGATTAGCAG TGGCTGGTACTGGTTCGACCCCTGGGGCCAGGGA ACCCTGGTCACCGTCTCCTCA |
| | | | SEQ ID NO: 27895 | SEQ ID NO: 31901 |

EP 4 435 105 A2

FIGURE 50

| | | AA | DIVMTQSPDSLAVSLGERATINCKSSQSVLYSSN NANYLAWYQQKPGQPPNLLIYWASTRESGVPD RFSGSGSGTDFTLTISSLQAEDVAVYYCQQYYST PCSFGQGTKLEIK | QVQLVQSGAEVKKPGASVKVSCKASGYTFTNYDIN WVRQATGQGLEWMGWMNPNSGNIGYAQKFQGR VTMTRNTSISTAYMELSSLRSEDTAVYYCAISSGW YWFDPWGQGTLVTVSS |
|---|---|---|---|---|
| | | | SEQ ID NO: 27896 | SEQ ID NO: 31902 |
| IPS:437369 | 21-225_74D6 | NA | GAAATTGTGTTGACGCAGTCTCCAGGCACCCT GTCTTTGTCTCCAGGGGAAAGAGCCACCCTCT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCCGGGCCACTGGCATCCCAGACAGGTTCAGT GGCAGTGGGTGTGGGACAGACTTCGCTCTCAC CATCAGCAGACTGGAGCCTGAAGATTTTGCAG TGTATTACTGTCAGCATTATGATAACTCACCGT GGACGTTCGGCCAAGGGACCAAGGTGGAAAT CAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC ATGGACGTCTGGGGCCAAGGGACCACGGTCACC GTCTCCTCA |
| | | | SEQ ID NO: 27897 | SEQ ID NO: 31903 |
| | | AA | EIVLTQSPGTLSLSPGERATLSCRASQSVYSSYLA WYQQKPGQAPRLLIYGASSRATGIPDRFSGSGCG TDFALTISRLEPEDFAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 27898 | SEQ ID NO: 31904 |

2666

FIGURE 50

| iPS:437371 | 21-225_74D8 | NA | GATATTGTGATGACTCAGTCTCCACTCTCCCTG CCCGTCACCCCTGGAGAGCCGGCCTCCATCTC ATGCAGGTCTAGTCAGAGCCTCGTGCATAGTA GTGGATACAACTATTTGGATTGGTACCTGCAG AAGCCAGGGCAGTCTCCACAGCTCGTTATCTA TTTGGGTTCTAATCGGGCCTCCGGGGTCCCTG ACAGGTTCAGTGGCAGTGGATCAGGCTCAGAT TTTACACTGAAGATCAGCAGAGTGGAGGCTGA GGATGTTGGACTTTATTACTGCATGCAAGCTC TACACCCTCCTCTCACTTTCGGCGGAGGGACC AAGGTGGAGATCAAA | GAGGTGCAGCTGGTGGAGTCTGGGGGAGGCTTG GTACAGCCGGGGGGGTCCCTGAGACTCTCCTGTG CAGCCTCTGGATTCACCTTCAGTAACTACGACAT GCACTGGGTCCGCCAAGCTACAGGAAAAGGTCT GGAGTGGGTCTCAGCTATTGGTACTGCTGGTGAC ACATACTATCCAGGCTCCGTGAAGGGCCGATTCA CCATCTCCAGAGAAAATGCCAAGAACTCCTTGTA TCTTCAAATGAACAGCCTGAGAGCCGGGGACAC GGCTGTGTATTACTGTGCAAGAGTTCTTGACTAC GGTGACTCCTTGGGCTACTACTACTACGGTATGG ACGTCTGGGGCCAAGGGACCACGGTCACCGTCTC CTCA |
|---|---|---|---|---|
| | | | SEQ ID NO: 27899 | SEQ ID NO: 31905 |
| | | AA | DIVMTQSPLSLPVTPGEPASISCRSSQSLVHSSGY NYLDWYLQKPGQSPQLVIYLGSNRASGVPDRFS GSGSGSDFTLKISRVEAEDVGLYYCMQALHPPL TFGGGTKVEIK | EVQLVESGGGLVQPGGSLRLSCAASGFTFSNYDMH WVRQATGKGLEWVSAIGTAGDTYYPGSVKGRFTIS RENAKNSLYLQMNSLRAGDTAVYYCARVLDYGDS LGYYYYGMDVWGQGTTVTVSS |
| | | | SEQ ID NO: 27900 | SEQ ID NO: 31906 |
| iPS:437377 | 21-225_74G9 | NA | GAATTTATGTTGACGCAGTCTCCAGGCACCCT GTCTTTGTCTCCAGGGGAAAGAGCCACCCTCT CCTGCAGGGCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGATCACCTTGAAGGAGTCTGGTCCTACGCTGG TGAAACCCACACAGACCCTCACGCTGACCTGCAC CTTCTCTGGGTTCTCACTCAGCACTGGTGGAGTG GGTGTGGGCTGGATCCGTCAGCCCCCAGGAAAG GCCCTGGAGTGGCTTGCACTCATTTATTGGGATG ATGATAAGCGCTACAGCCCATCTCTGAAGAGCA GGCTCACCATCACCAAGGACACCCCCAAAAACC AGGTGGTCCTTACAATGACCAACATGGACCCTGT GGACACAGCCACATATTACTGTGCACACCTTATA GCAGTGGCCTTTGACTACTGGGGCCAGGGAACCC TGGTCACCGTCTCCTCA |
| | | | SEQ ID NO: 27901 | SEQ ID NO: 31907 |

FIGURE 50

| | | | | |
|---|---|---|---|---|
| | | AA | EFMLTQSPGTLSLSPGERATLSCRASQSVSSSYL VWYQQKPGQAPRLLIYGASTRATGIPDRFSGSGS GTDFTLTISRLEPEYFAVYYCQQYGCSPLTFGGG TKVEIT | QITLKESGPTLVKPTQTLTLTCTFSGFSLSTGGVGVG WIRQPPGKALEWLALIYWDDDKRYSPSLKSRLTIT KDTPKNQVVLTMTNMDPVDTATYYCAHLIAVAFD YWGQGTLVTVSS |
| | | | SEQ ID NO: 27902 | SEQ ID NO: 31908 |
| iPS:437379 | 21-225_74H2 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATAAGAACTACTTAACTTGGTACCA GCAGAAACCAGGACAGCCTCATAAGCTGCTCA TTTACTGGGCATCTACTCGGGAATCCGGGGTC CCTGACCGATTCAGTGGCAGCGGGTCTGGGAC AGATTTCAGTCTCACGATCGGCAGCCTGCAGG CTGAAGATGTGGCAGTTTATTACTGTCAGCAA TATTATAGTATTCCTCCGACGTTCGGCCAAGG GACCAAGGTGGAAATCAAA | CAGGTGCAGCTGGTGCAGTCTGGGGGCTGAGGTG AAGAAGCCTGGGGGCCTCAGTGAAGGTCTCCTGC AAGGCTTCTGGATTCACCTTCACCAATTATGATA TCAACTGGGTGCGACAGGCCACTGGACAAGGGC TTGAGTGGATGGGATGGATGCACCCTAACAGTGG TAACACAGGCTTTGCACAGAAGTTCCAGGGCAG AGTCACCATGACCAGGAACACCTCCATAAGCAC AGCCTACATGGAACTGAGCAGCCTGAGATCTGA GGACACGGCCGTGTATTACTGTGCGGTTTCCAGT GGCTGGTACTGGTTCGACCCCTGGGGCCAGGGA ACCCTGGTCACCGTCTCCTCA |
| | | | SEQ ID NO: 27903 | SEQ ID NO: 31909 |
| | | AA | DIVMTQSPDSLAVSLGERATINCKSSQSVLHSSN NKNYLTWYQQKPGQPHKLLIYWASTRESGVPD RFSGSGSGTDFSLTIGSLQAEDVAVYYCQQYYSI PPTFGQGTKVEIK | QVQLVQSGAEVKKPGASVKVSCKASGFTFTNYDIN WVRQATGQGLEWMGWMHPNSGNTGFAQKFQGR VTMTRNTSISTAYMELSSLRSEDTAVYYCAVSSGW YWFDPWGQGTLVTVSS |
| | | | SEQ ID NO: 27904 | SEQ ID NO: 31910 |

FIGURE 50

| | | | | |
|---|---|---|---|---|
| iPS:437383 | 21-225_74H8 | NA | GAAATTGTGTTGACGCAGTCTCCAGGCACCCT GTCTTTGTCTCCAGGGGAAAGAGCCACCCTCT CCTGCAGGGCCAGTCAGAGTTTTAGCAGCAGC TACTTAGCCTGGTACCAGCAGAAACCTGGCCA GGCTCCCAGGCTCCTCATTTATGGTGCATCCA GCAGGGCCACTGGCATCCCAGACAGGTTCAGT GGCAGTGGGTCTGGGACAGACTTCACTCTCAC CATCAGCAGACTGGAGCCTGAAGATTTTGCAG TGTATTACTGTCAGCAGTATGGTAGCTCAAGG ACGTTCGGCCAAGGGACCAAGGTGGAAATCA AA | GAGGTGCACCTGGTGGAGTCTGGGGGAGGCTTG GTAAAGCCTGGGGGGTCCCTTAGACTCTCCTGTG CAGCCTCTGGATTCACTTTCAGTAACGCCTGGAT GAACTGGGTCCGCCAGGCTCCAGGGAAGGGGCT GGAGTGGGTTGGCCGTATTAAAAGCAAAACTGA TGGTGGGACAACAGACTACGCTGCACCCGTGAA AGGCAGATTCACCATCTCAAGAGATGATTCAAA AAACACGCTGTATCTGCAAATGAACAGCCTGAA AACCGAGGACACAGCCGTGTATTACTGTACCACA GTGGGAGCTACTACGGACTACTGGGGCCAGGGA ACCCTGGTCACCGTCTCCTCA |
| | | | SEQ ID NO: 27905 | SEQ ID NO: 31911 |
| | | AA | EIVLTQSPGTLSLSPGERATLSCRASQSFSSSYLA WYQQKPGQAPRLLIYGASSRATGIPDRFSGSGSG TDFTLTISRLEPEDFAVYYCQQYGSSRTFGQGTK VEIK | EVHLVESGGGLVKPGGSLRLSCAASGFTFSNAWM NWVRQAPGKGLEWVGRIKSKTDGGTTDYAAPVK GRFTISRDDSKNTLYLQMNSLKTEDTAVYYCTTVG ATTDYWGQGTLVTVSS |
| | | | SEQ ID NO: 27906 | SEQ ID NO: 31912 |
| iPS:438664 | 21-225_216G1 | NA | GACATCGAGATGACCCAGTCTCCATCCTCACT GTCTGCATCTGTAGGAGACAGAGTCACCATCA CTTGTCGGGCGAGTCAGGGCATTAGCAGTTAT TTAGCCTGGCTTCAGCAGAAACCAGGGAAAGC CCCTAAGTCCCTGATCTATGCTGCATCCAGTTT GCAAAGTGGGGTCCCATCAAGGTTCAGCGGCA GTGGATCTGGGACAGATTTCACTCTCACCATC AGCAGCCTGCAGCCTGAAGATTTCGCAACTTA TTATTGCCTACGGTATGATACTTACCCTCTCAC TTTCGGCGGAGGGACCAAGGTGGAGATCAAG | CAGGTGCACCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGGGGGGACTG GAACCCCGAGGGTATGGACGTCTGGGGCCAAGG GACCACGGTCATCGTCTCCTCA |
| | | | SEQ ID NO: 27907 | SEQ ID NO: 31913 |

FIGURE 50

| | | | | |
|---|---|---|---|---|
| | | AA | DIEMTQSPSSLSASVGDRVTITCRASQGISSYLA WLQQKPGKAPKSLIYAASSLQSGVPSRFSGSGSG TDFTLTISSLQPEDFATYYCLRYDTYPLTFGGGT KVEIK | QVHLVESGGGVVQPGRSLRLSCAASGFTFSNYGM HWVRQAPGKGLEWVAVIWYDGSNKYYADSVKGR FTISRDNSKNTLYLQMNSLRAEDTAVYYCARGDW NPEGMDVWGQGTTVIVSS |
| | | | SEQ ID NO: 27908 | SEQ ID NO: 31914 |
| iPS:441468 | 21-225_25A4.001.001 | NA | GACATCGTGATGACCCAGTTTCCAGACTCCCT GGCTGTGTCTCTGGGCGAGAGGGCCACCATCA AGTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGGAGG CTGAAGATGTGGCAGTTTATTACTGTCAGCAG TATTATAGTACTCCTCCGACGTTCGGCCAAGG GACCAAGGTGGAAATCAAA | CAGGTGCTCCTGGTGCAGTCTGGGGGCTGAGGTGA AGAGGCCTGGGGCCTCAGTGAAGGTCAGCTGCA AGGCTTCTGGATACACCTTCACCAATTATGATAT TAATTGGGTGCGACAGGCCACTGGACAAGGGCT TGAGTGGATGGGATGGATGTACCCTAACAGTGGT AGCACAGGCTATGCACAGAAATTCCAGGGCAGA GTCACCATGACCAGGGACACCTCCATCAGCACA GCCTACATGGAGCTGAGCAGCCTGAGATCTGAG GACACGGCCGTCTATTACTGTGCGAGTAGCAGTG GCTGGTACTACTTTGACTACTGGGGGCCAGGGAAC CCTGGTCACCGTGTCCTCA |
| | | | SEQ ID NO: 27909 | SEQ ID NO: 31915 |
| | | AA | DIVMTQFPDSLAVSLGERATIKCKSSQSVLYSSH NNNYLAWYQQKPGQPPKLLLYWASTRESGVPD RFSGSGSGTEFTLTISSLEAEDVAVYYCQQYYST PPTFGQGTKVEIK | QVLLVQSGAEVKRPGASVKVSCKASGYTFTNYDIN WVRQATGQGLEWMGWMYPNSGSTGYAQKFQGR VTMTRDTSISTAYMELSSLRSEDTAVYYCASSSGW YYFDYWGQGTLVTVSS |
| | | | SEQ ID NO: 27910 | SEQ ID NO: 31916 |

FIGURE 50

| iPS:441475 | 21-225_25A4.001.002 | NA | GACATCGTGATGACCCAGTTTCCAGACTCCCT GGCTGTGTCTCTGGGCGAGAGGGCCACCATCA AGTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGGAGG CTGAAGATGTGGCAGTTTATTACTGTCAGCAG TATTATAGTACTCCTCCGACGTTCGGCCAAGG GACCAAGGTGGAAATCAAA | CAGGTGCTCCTGGTGCAGTCTGGGGGCTGAGGTGA AGAGGCCTGGGGCCTCAGTGAAGGTCAGCTGCA AGGCTTCTGGATACACCTTCACCAATTATGATAT TAATTGGGTGCGACAGGCCACTGGACAAGGGCT TGAGTGGATGGGATGGATGTACCCTAACAGTGGT AACGCAGGCTATGCACAGAAATTCCAGGGCAGA GTCACCATGACCAGGGACACCTCCATCAGCACA GCCTACATGGAGCTGAGCAGCCTGAGATCTGAG GACACGGCCGTCTATTACTGTGCGAGTAGCAGTG GCTGGTACTACTTTGACTACTGGGGCCAGGGAAC CCTGGTCACCGTGTCCTCA |
| | | | SEQ ID NO: 27911 | SEQ ID NO: 31917 |
| | | AA | DIVMTQFPDSLAVSLGERATIKCKSSQSVLYSSH NNNYLAWYQQKPGQPPKLLLYWASTRESGVPD RFSGSGSGTEFTLTISSLEAEDVAVYYCQQYYST PPTFGQGTKVEIK | QVLLVQSGAEVKRPGASVKVSCKASGYTFTNYDIN WVRQATGQGLEWMGWMYPNSGNAGYAQKFQGR VTMTRDTSISTAYMELSSLRSEDTAVYYCASSSGW YYFDYWGQGTLVTVSS |
| | | | SEQ ID NO: 27912 | SEQ ID NO: 31918 |
| iPS:441482 | 21-225_25A4.001.003 | NA | GACATCGTGATGACCCAGTTTCCAGACTCCCT GGCTGTGTCTCTGGGCGAGAGGGCCACCATCA AGTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGGAGG CTGAAGATGTGGCAGTTTATTACTGTCAGCAG TATTATAGTACTCCTCCGACGTTCGGCCAAGG GACCAAGGTGGAAATCAAA | CAGGTGCTCCTGGTGCAGTCTGGGGGCTGAGGTGA AGAGGCCTGGGGCCTCAGTGAAGGTCAGCTGCA AGGCTTCTGGATACACCTTCACCAATTATGATAT TAATTGGGTGCGACAGGCCACTGGACAAGGGCT TGAGTGGATGGGATGGATGTACCCTAACAGTGGT AACGTAGGCTATGCACAGAAATTCCAGGGCAGA GTCACCATGACCAGGGACACCTCCATCAGCACA GCCTACATGGAGCTGAGCAGCCTGAGATCTGAG GACACGGCCGTCTATTACTGTGCGAGTAGCAGTG GCTGGTACTACTTTGACTACTGGGGCCAGGGAAC CCTGGTCACCGTGTCCTCA |
| | | | SEQ ID NO: 27913 | SEQ ID NO: 31919 |

FIGURE 50

| | | | | |
|---|---|---|---|---|
| | | AA | DIVMTQFPDSLAVSLGERATIKCKSSQSVLYSSH NNNYLAWYQQKPGQPPKLLLYWASTRESGVPD RFSGSGSGTEFTLTISSLEAEDVAVYYCQQYYST PPTFGQGTKVEIK | QVLLVQSGAEVKRPGASVKVSCKASGYTFTNYDIN WVRQATGQGLEWMGWMYPNSGNVGYAQKFQGR VTMTRDTSISTAYMELSSLRSEDTAVYYCASSSGW YYFDYWGQGTLVTVSS |
| | | | SEQ ID NO: 27914 | SEQ ID NO: 31920 |
| iPS:441489 | 21-225_25A4.001.004 | NA | GACATCGTGATGACCCAGTTTCCAGACTCCCT GGCTGTGTCTCTGGGCGAGAGGGCCACCATCA AGTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGGAGG CTGAAGATGTGGCAGTTTATTACTGTCAGCAG TATTATAGTACTCCTCCGACGTTCGGCCAAGG GACCAAGGTGGAAATCAAA | CAGGTGCTCCTGGTGCAGTCTGGGGGCTGAGGTGA AGAGGCCTGGGGCCTCAGTGAAGGTCAGCTGCA AGGCTTCTGGATACACCTTCACCAATTATGATAT TAATTGGGTGCGACAGGCCACTGGACAAGGGCT TGAGTGGATGGGATGGATGTACCCTAACAGTGGT CAAACAGGCTATGCACAGAAATTCCAGGGCAGA GTCACCATGACCAGGGACACCTCCATCAGCACA GCCTACATGGAGCTGAGCAGCCTGAGATCTGAG GACACGGCCGTCTATTACTGTGCGAGTAGCAGTG GCTGGTACTACTTTGACTACTGGGGGCCAGGGAAC CCTGGTCACCGTGTCCTCA |
| | | | SEQ ID NO: 27915 | SEQ ID NO: 31921 |
| | | AA | DIVMTQFPDSLAVSLGERATIKCKSSQSVLYSSH NNNYLAWYQQKPGQPPKLLLYWASTRESGVPD RFSGSGSGTEFTLTISSLEAEDVAVYYCQQYYST PPTFGQGTKVEIK | QVLLVQSGAEVKRPGASVKVSCKASGYTFTNYDIN WVRQATGQGLEWMGWMYPNSGQTGYAQKFQGR VTMTRDTSISTAYMELSSLRSEDTAVYYCASSSGW YYFDYWGQGTLVTVSS |
| | | | SEQ ID NO: 27916 | SEQ ID NO: 31922 |

FIGURE 50

| iPS:441496 | 21-225_25A4.001.005 | NA | GACATCGTGATGACCCAGTCTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCAACTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAGCACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
|---|---|---|---|---|
| | | | SEQ ID NO: 27917 | SEQ ID NO: 31923 |
| | | AA | DIVMTQSPDSLAVSLGERATIKCKSSQSVLYSSHNNNYLAWYQQKPGQPPKLLLYWASTRESGVPDRFSGSGSGTEFTLTISSLQAEDVAVYYCQQYYSTPPTFGQGTKVEIK | QVQLVQSGAEVKRPGASVKVSCKASGYTFTNYDINWVRQATGQGLEWMGWMYPNSGSTGYAQKFQGRVTMTRDTSISTAYMELSSLRSEDTAVYYCASSSGWYYFDYWGQGTLVTVSS |
| | | | SEQ ID NO: 27918 | SEQ ID NO: 31924 |
| iPS:441505 | 21-225_25A4.001.006 | NA | GACATCGTGATGACCCAGTCTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCAACTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAACGCAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27919 | SEQ ID NO: 31925 |

FIGURE 50

| | | | | |
|---|---|---|---|---|
| | | AA | DIVMTQSPDSLAVSLGERATIKCKSSQSVLYSSH NNNYLAWYQQKPGQPPKLLLYWASTRESGVPD RFSGSGSGTEFTLTISSLQAEDVAVYYCQQYYST PPTFGQGTKVEIK | QVQLVQSGAEVKRPGASVKVSCKASGYTFTNYDIN WVRQATGQGLEWMGWMYPNSGNAGYAQKFQGR VTMTRDTSISTAYMELSSLRSEDTAVYYCASSSGW YYFDYWGQGTLVTVSS |
| | | | SEQ ID NO: 27920 | SEQ ID NO: 31926 |
| iPS:441512 | 21-225_25A4.001.007 | NA | GACATCGTGATGACCCAGTCTCCAGACTCCCT GGCTGTGTCTCTGGGCGAGAGGGCCACCATCA AGTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGCAGG CTGAAGATGTGGCAGTTTATTACTGTCAGCAG TATTATAGTACTCCTCCGACGTTCGGCCAAGG GACCAAGGTGGAAATCAAA | CAGGTGCAACTGGTGCAGTCTGGGGGCTGAGGTG AAGAGGCCTGGGGCCTCAGTGAAGGTCAGCTGC AAGGCTTCTGGATACACCTTCACCAATTATGATA TTAATTGGGTGCGACAGGCCACTGGACAAGGGC TTGAGTGGATGGGATGGATGTACCCTAACAGTGG TAACGTAGGCTATGCACAGAAATTCCAGGGCAG AGTCACCATGACCAGGGACACCTCCATCAGCAC AGCCTACATGGAGCTGAGCAGCCTGAGATCTGA GGACACGGCCGTCTATTACTGTGCGAGTAGCAGT GGCTGGTACTACTTTGACTACTGGGGCCAGGGAA CCCTGGTCACCGTGTCCTCA |
| | | | SEQ ID NO: 27921 | SEQ ID NO: 31927 |
| | | AA | DIVMTQSPDSLAVSLGERATIKCKSSQSVLYSSH NNNYLAWYQQKPGQPPKLLLYWASTRESGVPD RFSGSGSGTEFTLTISSLQAEDVAVYYCQQYYST PPTFGQGTKVEIK | QVQLVQSGAEVKRPGASVKVSCKASGYTFTNYDIN WVRQATGQGLEWMGWMYPNSGNVGYAQKFQGR VTMTRDTSISTAYMELSSLRSEDTAVYYCASSSGW YYFDYWGQGTLVTVSS |
| | | | SEQ ID NO: 27922 | SEQ ID NO: 31928 |

FIGURE 50

| iPS:441519 | 21-225_25A4.001.008 | NA | GACATCGTGATGACCCAGTCTCCAGACTCCCT GGCTGTGTCTCTGGGCGAGAGGGCCACCATCA AGTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGCAGG CTGAAGATGTGGCAGTTTATTACTGTCAGCAG TATTATAGTACTCCTCCGACGTTCGGCCAAGG GACCAAGGTGGAAATCAAA | CAGGTGCAACTGGTGCAGTCTGGGGCTGAGGTG AAGAGGCCTGGGGCCTCAGTGAAGGTCAGCTGC AAGGCTTCTGGATACACCTTCACCAATTATGATA TTAATTGGGTGCGACAGGCCACTGGACAAGGGC TTGAGTGGATGGGATGGATGTACCCTAACAGTGG TCAAACAGGCTATGCACAGAAATTCCAGGGCAG AGTCACCATGACCAGGGACACCTCCATCAGCAC AGCCTACATGGAGCTGAGCAGCCTGAGATCTGA GGACACGGCCGTCTATTACTGTGCGAGTAGCAGT GGCTGGTACTACTTTGACTACTGGGGCCAGGGAA CCCTGGTCACCGTGTCCTCA |
| | | | SEQ ID NO: 27923 | SEQ ID NO: 31929 |
| | | AA | DIVMTQSPDSLAVSLGERATIKCKSSQSVLYSSH NNNYLAWYQQKPGQPPKLLLYWASTRESGVPD RFSGSGSGTEFTLTISSLQAEDVAVYYCQQYYST PPTFGQGTKVEIK | QVQLVQSGAEVKRPGASVKVSCKASGYTFTNYDIN WVRQATGQGLEWMGWMYPNSGQTGYAQKFQGR VTMTRDTSISTAYMELSSLRSEDTAVYYCASSSGW YYFDYWGQGTLVTVSS |
| | | | SEQ ID NO: 27924 | SEQ ID NO: 31930 |
| iPS:441554 | 21-225_25A4.001.013 | NA | GACATCGTGATGACCCAGTTTCCAGACTCCCT GGCTGTGTCTCTGGGCGAGAGGGCCACCATCA AGTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGCAGG CTGAAGATGTGGCAGTTTATTACTGTCAGCAG TATTATAGTACTCCTCCGACGTTCGGCCAAGG GACCAAGGTGGAAATCAAA | CAGGTGCTCCTGGTGCAGTCTGGGGCTGAGGTGA AGAGGCCTGGGGCCTCAGTGAAGGTCAGCTGCA AGGCTTCTGGATACACCTTCACCAATTATGATAT TAATTGGGTGCGACAGGCCACTGGACAAGGGCT TGAGTGGATGGGATGGATGTACCCTAACAGTGGT AGCACAGGCTATGCACAGAAATTCCAGGGCAGA GTCACCATGACCAGGGACACCTCCATCAGCACA GCCTACATGGAGCTGAGCAGCCTGAGATCTGAG GACACGGCCGTCTATTACTGTGCGAGTAGCAGTG GCTGGTACTACTTTGACTACTGGGGCCAGGGAAC CCTGGTCACCGTGTCCTCA |
| | | | SEQ ID NO: 27925 | SEQ ID NO: 31931 |

FIGURE 50

| | | | | |
|---|---|---|---|---|
| | | AA | DIVMTQFPDSLAVSLGERATIKCKSSQSVLYSSH NNNYLAWYQQKPGQPPKLLLYWASTRESGVPD RFSGSGSGTEFTLTISSLQAEDVAVYYCQQYYST PPTFGQGTKVEIK | QVLLVQSGAEVKRPGASVKVSCKASGYTFTNYDIN WVRQATGQGLEWMGWMYPNSGSTGYAQKFQGR VTMTRDTSISTAYMELSSLRSEDTAVYYCASSSGW YYFDYWGQGTLVTVSS |
| | | | SEQ ID NO: 27926 | SEQ ID NO: 31932 |
| iPS:441595 | 21-225_25A4.001.019 | NA | GACATCGTGATGACCCAGTCTCCAGACTCCCT GGCTGTGTCTCTGGGCGAGAGGGCCACCATCA ACTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGGAGG CTGAAGATGTGGCAGTTTATTACTGTCAGCAG TATTATAGTACTCCTCCGACGTTCGGCCAAGG GACCAAGGTGGAAATCAAA | CAGGTGCAACTGGTGCAGTCTGGGGCTGAGGTG AAGAGGCCTGGGGCCTCAGTGAAGGTCAGCTGC AAGGCTTCTGGATACACCTTCACCAATTATGATA TTAATTGGGTGCGACAGGCCCCTGGACAAGGGCT TGAGTGGATGGGATGGATGTACCCTAACAGTGGT AGCACAGGCTATGCACAGAAATTCCAGGGCAGA GTCACCATGACCAGGGACACCTCCATCAGCACA GCCTACATGGAGCTGAGCAGCCTGAGATCTGAG GACACGGCCGTCTATTACTGTGCGAGTAGCAGTG GCTGGTACTACTTTGACTACTGGGGGCCAGGGAAC CCTGGTCACCGTGTCCTCA |
| | | | SEQ ID NO: 27927 | SEQ ID NO: 31933 |
| | | AA | DIVMTQSPDSLAVSLGERATINCKSSQSVLYSSH NNNYLAWYQQKPGQPPKLLLYWASTRESGVPD RFSGSGSGTEFTLTISSLEAEDVAVYYCQQYYST PPTFGQGTKVEIK | QVQLVQSGAEVKRPGASVKVSCKASGYTFTNYDIN WVRQAPGQGLEWMGWMYPNSGSTGYAQKFQGR VTMTRDTSISTAYMELSSLRSEDTAVYYCASSSGW YYFDYWGQGTLVTVSS |
| | | | SEQ ID NO: 27928 | SEQ ID NO: 31934 |

FIGURE 50

| iPS:441604 | 21-225_25A4.001.020 | NA | GACATCGTGATGACCCAGTCTCCAGACTCCCT GGCTGTGTCTCTGGGCGAGAGGGCCACCATCA ACTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGCAGG CTGAAGATGTGGCAGTTTATTACTGTCAGCAG TATTATAGTACTCCTCCGACGTTCGGCCAAGG GACCAAGGTGGAAATCAAA | CAGGTGCTCCTGGTGCAGTCTGGGGCTGAGGTGA AGAGGCCTGGGGCCTCAGTGAAGGTCAGCTGCA AGGCTTCTGGATACACCTTCACCAATTATGATAT TAATTGGGTGCGACAGGCCCCTGGACAAGGGCTT GAGTGGATGGGATGGATGTACCCTAACAGTGGTC AAACAGGCTATGCACAGAAATTCCAGGGCAGAG TCACCATGACCAGGGACACCTCCATCAGCACAGC CTACATGGAGCTGAGCAGCCTGAGATCTGAGGA CACGGCCGTCTATTACTGTGCGAGTAGCAGTGGC TGGTACTACTTTGACTACTGGGGCCAGGGAACCC TGGTCACCGTGTCCTCA |
| | | | SEQ ID NO: 27929 | SEQ ID NO: 31935 |
| | | AA | DIVMTQSPDSLAVSLGERATINCKSSQSVLYSSH NNNYLAWYQQKPGQPPKLLLYWASTRESGVPD RFSGSGSGTEFTLTISSLQAEDVAVYYCQQYYST PPTFGQGTKVEIK | QVLLVQSGAEVKRPGASVKVSCKASGYTFTNYDIN WVRQAPGQGLEWMGWMYPNSGQTGYAQKFQGR VTMTRDTSISTAYMELSSLRSEDTAVYYCASSSGW YYFDYWGQGTLVTVSS |
| | | | SEQ ID NO: 27930 | SEQ ID NO: 31936 |
| iPS:441613 | 21-225_25A4.001.021 | NA | GACATCGTGATGACCCAGTCTCCAGACTCCCT GGCTGTGTCTCTGGGCGAGAGGGCCACCATCA ACTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGCAGG CTGAAGATGTGGCAGTTTATTACTGTCAGCAG TATTATAGTACTCCTCCGACGTTCGGCCAAGG GACCAAGGTGGAAATCAAA | CAGGTGCAACTGGTGCAGTCTGGGGCTGAGGTG AAGAGGCCTGGGGCCTCAGTGAAGGTCAGCTGC AAGGCTTCTGGATACACCTTCACCAATTATGATA TTAATTGGGTGCGACAGGCCACTGGACAAGGGC TTGAGTGGATGGGATGGATGTACCCTAACAGTGG TAACGCAGGCTATGCACAGAAATTCCAGGGCAG AGTCACCATGACCAGGGACACCTCCATCAGCAC AGCCTACATGGAGCTGAGCAGCCTGAGATCTGA GGACACGGCCGTCTATTACTGTGCGAGTAGCAGT GGCTGGTACTACTTTGACTACTGGGGCCAGGGAA CCCTGGTCACCGTGTCCTCA |
| | | | SEQ ID NO: 27931 | SEQ ID NO: 31937 |

FIGURE 50

| | | | | |
|---|---|---|---|---|
| | | AA | DIVMTQSPDSLAVSLGERATINCKSSQSVLYSSH NNNYLAWYQQKPGQPPKLLLYWASTRESGVPD RFSGSGSGTEFTLTISSLQAEDVAVYYCQQYYST PPTFGQGTKVEIK | QVQLVQSGAEVKRPGASVKVSCKASGYTFTNYDIN WVRQATGQGLEWMGWMYPNSGNAGYAQKFQGR VTMTRDTSISTAYMELSSLRSEDTAVYYCASSSGW YYFDYWGQGTLVTVSS |
| | | | SEQ ID NO: 27932 | SEQ ID NO: 31938 |
| iPS:441841 | 21-225_4A2.001.001 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAGTACTCCAGTCACTTTCGGCCCTGGG ACCAAAGTGGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGGCCAGGGAAC CCTGGTCACCGTGTCCTCA |
| | | | SEQ ID NO: 27933 | SEQ ID NO: 31939 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYSTP VTFGPGTKVGIK | QVQLVQSGTEVKKPGASVKVSCKASGYTFTNYDIN WVRQATGQGLEWMGWMHPNSGNTGYAQKFQGR VTLTRDTSISTAYMELSSLRSEDTAVYYCASSSGWY YFDYWGQGTLVTVSS |
| | | | SEQ ID NO: 27934 | SEQ ID NO: 31940 |

FIGURE 50

| | | | | |
|---|---|---|---|---|
| iPS:441847 | 21-225_4A2.001.002 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAATGCTCCAGTCACTTTCGGCCCTGGGACCAAAGTGGGTATCAAA<br>SEQ ID NO: 27935 | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA<br>SEQ ID NO: 31941 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYNAPVTFGPGTKVGIK<br>SEQ ID NO: 27936 | QVQLVQSGTEVKKPGASVKVSCKASGYTFTNYDINWVRQATGQGLEWMGWMHPNSGNTGYAQKFQGRVTLTRDTSISTAYMELSSLRSEDTAVYYCASSSGWYYFDYWGQGTLVTVSS<br>SEQ ID NO: 31942 |
| iPS:441853 | 21-225_4A2.001.003 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATCAAACTCCAGTCACTTTCGGCCCTGGGACCAAAGTGGGTATCAAA<br>SEQ ID NO: 27937 | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA<br>SEQ ID NO: 31943 |

FIGURE 50

| | | | | |
|---|---|---|---|---|
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYQTP VTFGPGTKVGIK | QVQLVQSGTEVKKPGASVKVSCKASGYTFTNYDIN WVRQATGQGLEWMGWMHPNSGNTGYAQKFQGR VTLTRDTSISTAYMELSSLRSEDTAVYYCASSSGWY YFDYWGQGTLVTVSS |
| | | | SEQ ID NO: 27938 | SEQ ID NO: 31944 |
| iPS:441859 | 21-225_4A2.001.004 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAATACTCCAGTCACTTTCGGCCCTGGG ACCAAAGTGGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACGCAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGGCCAGGGAAC CCTGGTCACCGTGTCCTCA |
| | | | SEQ ID NO: 27939 | SEQ ID NO: 31945 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYNTP VTFGPGTKVGIK | QVQLVQSGTEVKKPGASVKVSCKASGYTFTNYDIN WVRQATGQGLEWMGWMHPNSGNAGYAQKFQGR VTLTRDTSISTAYMELSSLRSEDTAVYYCASSSGWY YFDYWGQGTLVTVSS |
| | | | SEQ ID NO: 27940 | SEQ ID NO: 31946 |

FIGURE 50

| iPS:441866 | 21-225_4A2.001.005 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAATACTCCAGTCACTTTCGGCCCTGGG ACCAAAGTGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAGCACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
|---|---|---|---|---|
| | | | SEQ ID NO: 27941 | SEQ ID NO: 31947 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYNTP VTFGPGTKVGIK | QVQLVQSGTEVKKPGASVKVSCKASGYTFTNYDIN WVRQATGQGLEWMGWMHPNSGSTGYAQKFQGR VTLTRDTSISTAYMELSSLRSEDTAVYYCASSSGWY YFDYWGQGTLVTVSS |
| | | | SEQ ID NO: 27942 | SEQ ID NO: 31948 |
| iPS:441873 | 21-225_4A2.001.006 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAATACTCCAGTCACTTTCGGCCCTGGG ACCAAAGTGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TCAAACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27943 | SEQ ID NO: 31949 |

FIGURE 50

| | | | | |
|---|---|---|---|---|
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYNTP VTFGPGTKVGIK | QVQLVQSGTEVKKPGASVKVSCKASGYTFTNYDIN WVRQATGQGLEWMGWMHPNSGQTGYAQKFQGR VTLTRDTSISTAYMELSSLRSEDTAVYYCASSSGWY YFDYWGQGTLVTVSS |
| | | | SEQ ID NO: 27944 | SEQ ID NO: 31950 |
| iPS:441880 | 21-225_4A2.001.007 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAGTACTCCAGTCACTTTCGGCCCTGGG ACCAAAGTGGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TCAAACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGGCCAGGGAAC CCTGGTCACCGTGTCCTCA |
| | | | SEQ ID NO: 27945 | SEQ ID NO: 31951 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYSTP VTFGPGTKVGIK | QVQLVQSGTEVKKPGASVKVSCKASGYTFTNYDIN WVRQATGQGLEWMGWMHPNSGQTGYAQKFQGR VTLTRDTSISTAYMELSSLRSEDTAVYYCASSSGWY YFDYWGQGTLVTVSS |
| | | | SEQ ID NO: 27946 | SEQ ID NO: 31952 |

FIGURE 50

| | | | | |
|---|---|---|---|---|
| iPS:441884 | 21-225_4A2.001.008 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAGTACTCCAGTCACTTTCGGCCCTGGG ACCAAAGTGGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACGCAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27947 | SEQ ID NO: 31953 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYSTP VTFGPGTKVGIK | QVQLVQSGTEVKKPGASVKVSCKASGYTFTNYDIN WVRQATGQGLEWMGWMHPNSGNAGYAQKFQGR VTLTRDTSISTAYMELSSLRSEDTAVYYCASSSGWY YFDYWGQGTLVTVSS |
| | | | SEQ ID NO: 27948 | SEQ ID NO: 31954 |
| iPS:441888 | 21-225_4A2.001.009 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAATGCTCCAGTCACTTTCGGCCCTGGG ACCAAAGTGGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TCAAACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27949 | SEQ ID NO: 31955 |

FIGURE 50

| | | | | |
|---|---|---|---|---|
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYNAP VTFGPGTKVGIK | QVQLVQSGTEVKKPGASVKVSCKASGYTFTNYDIN WVRQATGQGLEWMGWMHPNSGQTGYAQKFQGR VTLTRDTSISTAYMELSSLRSEDTAVYYCASSSGWY YFDYWGQGTLVTVSS |
| | | | SEQ ID NO: 27950 | SEQ ID NO: 31956 |
| iPS:441892 | 21-225_4A2.001.010 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATCAAACTCCAGTCACTTTCGGCCCTGG GACCAAAGTGGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACGCAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGGCCAGGGAAC CCTGGTCACCGTGTCCTCA |
| | | | SEQ ID NO: 27951 | SEQ ID NO: 31957 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYQTP VTFGPGTKVGIK | QVQLVQSGTEVKKPGASVKVSCKASGYTFTNYDIN WVRQATGQGLEWMGWMHPNSGNAGYAQKFQGR VTLTRDTSISTAYMELSSLRSEDTAVYYCASSSGWY YFDYWGQGTLVTVSS |
| | | | SEQ ID NO: 27952 | SEQ ID NO: 31958 |

FIGURE 50

| iPS:441896 | 21-225_4A2.001.011 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATCAAACTCCAGTCACTTTCGGCCCTGG GACCAAAGTGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TCAAACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27953 | SEQ ID NO: 31959 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYQTP VTFGPGTKVGIK | QVQLVQSGTEVKKPGASVKVSCKASGYTFTNYDIN WVRQATGQGLEWMGWMHPNSGQTGYAQKFQGR VTLTRDTSISTAYMELSSLRSEDTAVYYCASSSGWY YFDYWGQGTLVTVSS |
| | | | SEQ ID NO: 27954 | SEQ ID NO: 31960 |
| iPS:441900 | 21-225_4A2.001.012 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATCAAACTCCAGTCACTTTCGGCCCTGG GACCAAAGTGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAGCACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27955 | SEQ ID NO: 31961 |

FIGURE 50

| | | | | |
|---|---|---|---|---|
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYQTP VTFGPGTKVGIK | QVQLVQSGTEVKKPGASVKVSCKASGYTFTNYDIN WVRQATGQGLEWMGWMHPNSGSTGYAQKFQGR VTLTRDTSISTAYMELSSLRSEDTAVYYCASSSGWY YFDYWGQGTLVTVSS |
| | | | SEQ ID NO: 27956 | SEQ ID NO: 31962 |
| iPS:441955 | 21-225_4A2.001.022 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAGTACTCCAGTCACTTTCGGCCCTGGG ACCAAAGTGGGGTATCAAA | CAGGTGCAGCTGGTGCAGTCTGGGGGCTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27957 | SEQ ID NO: 31963 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYSTP VTFGPGTKVGIK | QVQLVQSGAEVKKPGASVKVSCKASGYTFTNYDIN WVRQATGQGLEWMGWMHPNSGNTGYAQKFQGR VTLTRDTSISTAYMELSSLRSEDTAVYYCASSSGWY YFDYWGQGTLVTVSS |
| | | | SEQ ID NO: 27958 | SEQ ID NO: 31964 |

2686

FIGURE 50

| iPS:441962 | 21-225_4A2.001.023 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAGTACTCCAGTCACTTTCGGCCAAGG GACCAAAGTGGAAATCAAA | CAGGTGCAGCTGGTGCAGTCTGGGGCTGAGGTG AAGAAGCCTGGGGCCTCAGTGAAGGTGTCCTGC AAGGCTTCTGGATACACCTTCACCAATTATGATA TCAACTGGGTGCGACAGGCCCCTGGACAAGGGC TTGAGTGGATGGGATGGATGCACCCTAACAGTGG TAACACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27959 | SEQ ID NO: 31965 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYSTP VTFGQGTKVEIK | QVQLVQSGAEVKKPGASVKVSCKASGYTFTNYDIN WVRQAPGQGLEWMGWMHPNSGNTGYAQKFQGR VTLTRDTSISTAYMELSSLRSEDTAVYYCASSSGWY YFDYWGQGTLVTVSS |
| | | | SEQ ID NO: 27960 | SEQ ID NO: 31966 |
| iPS:441971 | 21-225_4A2.001.024 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAGTACTCCAGTCACTTTCGGCCCTGGG ACCAAAGTGGGGTATCAAA | CAGGTGCAGCTGGTGCAGTCTGGGGCTGAGGTG AAGAAGCCTGGGGCCTCAGTGAAGGTGTCCTGC AAGGCTTCTGGATACACCTTCACCAATTATGATA TCAACTGGGTGCGACAGGCCCACTGGACAAGGGC TCAAACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27961 | SEQ ID NO: 31967 |

FIGURE 50

| | | | | |
|---|---|---|---|---|
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYSTP VTFGPGTKVGIK | QVQLVQSGAEVKKPGASVKVSCKASGYTFTNYDIN WVRQATGQGLEWMGWMHPNSGQTGYAQKFQGR VTLTRDTSISTAYMELSSLRSEDTAVYYCASSSGWY YFDYWGQGTLVTVSS |
| | | | SEQ ID NO: 27962 | SEQ ID NO: 31968 |
| iPS:441999 | 21-225_4A2.001.028 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAGTACTCCAGTCACTTTCGGCCAAGG GACCAAAGTGGAAATCAAA | CAGGTGCAGCTGGTGCAGTCTGGGGGCTGAGGTG AAGAAGCCTGGGGCCTCAGTGAAGGTGTCCTGC AAGGCTTCTGGATACACCTTCACCAATTATGATA TCAACTGGGTGCGACAGGCCCCTGGACAAGGGC TTGAGTGGATGGGATGGATGCACCCTAACAGTGG TCAAACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGGCCAGGGAAC CCTGGTCACCGTGTCCTCA |
| | | | SEQ ID NO: 27963 | SEQ ID NO: 31969 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYSTP VTFGQGTKVEIK | QVQLVQSGAEVKKPGASVKVSCKASGYTFTNYDIN WVRQAPGQGLEWMGWMHPNSGQTGYAQKFQGR VTLTRDTSISTAYMELSSLRSEDTAVYYCASSSGWY YFDYWGQGTLVTVSS |
| | | | SEQ ID NO: 27964 | SEQ ID NO: 31970 |

FIGURE 50

| iPS:442006 | 21-225_4A2.001.029 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAGTACTCCAGTCACTTTCGGCCAAGG GACCAAAGTGGAAATCAAA | CAGGTGCAGCTGGTGCAGTCTGGGGGCTGAGGTG AAGAAGCCTGGGGCCTCAGTGAAGGTGTCCTGC AAGGCTTCTGGATACACCTTCACCAATTATGATA TCAACTGGGTGCGACAGGCCCCTGGACAAGGGC TTGAGTGGATGGGATGGATGCACCCTAACAGTGG TAGCACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
|---|---|---|---|---|
| | | | SEQ ID NO: 27965 | SEQ ID NO: 31971 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYSTP VTFGQGTKVEIK | QVQLVQSGAEVKKPGASVKVSCKASGYTFTNYDIN WVRQAPGQGLEWMGWMHPNSGSTGYAQKFQGR VTLTRDTSISTAYMELSSLRSEDTAVYYCASSSGWY YFDYWGQGTLVTVSS |
| | | | SEQ ID NO: 27966 | SEQ ID NO: 31972 |
| iPS:442020 | 21-225_4A2.001.031 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAATACTCCAGTCACTTTCGGCCCTGGG ACCAAAGTGGAAATCAAA | CAGGTGCAGCTGGTGCAGTCTGGGGGTGAGGTG AAGAAGCCTGGGGCCTCAGTGAAGGTGTCCTGC AAGGCTTCTGGATACACCTTCACCAATTATGATA TCAACTGGGTGCGACAGGCCGAAGGACAAGGGC TTGAGTGGATGGGATGGATGCACCCTAACAGTGG TAACGAAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27967 | SEQ ID NO: 31973 |

FIGURE 50

| | | | | |
|---|---|---|---|---|
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYNTP VTFGPGTKVEIK | QVQLVQSGGEVKKPGASVKVSCKASGYTFTNYDIN WVRQAEGQGLEWMGWMHPNSGNEGYAQKFQGR VTLTRDTSISTAYMELSSLRSEDTAVYYCASSSGWY YFDYWGQGTLVTVSS |
| | | | SEQ ID NO: 27968 | SEQ ID NO: 31974 |
| IPS:442050 | 21-225_4H6.004 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAATTT ATTATTGTCAACAGGCTAACAGTTTCCCATTC ACTTTCGGCCCTGGGACCAAAGTGGATATCAA A | CAGGTGCAGCTGGTACAGTCTGGGGCTGAGGTG AAGAAGCCTGGGGCCTCAGTGAAGGTCAGCTGC AAGGCTTCTGGATACACCTTCACCGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGTCTGAGATCTGACG ACACGGCCGTGTATTACTGTGCGAGAGATGGTAC CAGCTCGTTTGACTACTGGGGCCAGGGAACCCTG GTCACCGTGTCCTCA |
| | | | SEQ ID NO: 27969 | SEQ ID NO: 31975 |
| | | AA | DIQMTQSPSSVSASVGDRVTITCRASQGISRWLA WYQQKPGKAPKLLIYGASSLQSGVPSRFSGSGS GTDFTLTISSLQPEDFAIYYCQQANSFPFTFGPGT KVDIK | QVQLVQSGAEVKKPGASVKVSCKASGYTFTDYYL HWVRQAPGQGLEWMGWIHPNSGGTNYAQKFQGR VTMTRDTSISTAYMELSSLRSDDTAVYYCARDGTS SFDYWGQGTLVTVSS |
| | | | SEQ ID NO: 27970 | SEQ ID NO: 31976 |

FIGURE 50

| iPS:442059 | 21-225_4H6.005 | NA | GACATCCAGATGACCCAGTCTCCATCTTCCGT GTCTGCATCTGTAGGAGATAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAATTT ATTATTGTCAACAGGCTAACAGTTTCCCATTC ACTTTCGGCCAAGGGACCAAAGTGGATATCAA A | CAGGTGCAGCTGGTACAGTCTGGGGGCTGAGGTG AAGAAGCCTGGGGCCTCAGTGAAGGTCAGCTGC AAGGCTTCTGGATACACCTTCACCGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGTCTGAGATCTGACG ACACGGCCGTGTATTACTGTGCGAGAGATGGTAC CAGCTCGTTTGACTACTGGGGCCAGGGAACCCTG GTCACCGTGTCCTCA |
| | | | SEQ ID NO: 27971 | SEQ ID NO: 31977 |
| | | AA | DIQMTQSPSSVSASVGDRVTITCRASQGISRWLA WYQQKPGKAPKLLIYGASSLQSGVPSRFSGSGS GTDFTLTISSLQPEDFAIYYCQQANSFPFTFGQGT KVDIK | QVQLVQSGAEVKKPGASVKVSCKASGYTFTDYYL HWVRQAPGQGLEWMGWIHPNSGGTNYAQKFQGR VTMTRDTSISTAYMELSSLRSDDTAVYYCARDGTS SFDYWGQGTLVTVSS |
| | | | SEQ ID NO: 27972 | SEQ ID NO: 31978 |
| iPS:442065 | 21-225_4H6.006 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ATTATTGTCAACAGGCTAACAGTTTCCCATTC ACTTTCGGCCAAGGGACCAAAGTGGATATCAA A | CAGGTGCAGCTGGTACAGTCTGGGGGCTGAGGTG AAGAAGCCTGGGGCCTCAGTGAAGGTCAGCTGC AAGGCTTCTGGATACACCTTCACCGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGTCTGAGATCTGACG ACACGGCCGTGTATTACTGTGCGAGAGATGGTAC CAGCTCGTTTGACTACTGGGGCCAGGGAACCCTG GTCACCGTGTCCTCA |
| | | | SEQ ID NO: 27973 | SEQ ID NO: 31979 |

FIGURE 50

| | | | | |
|---|---|---|---|---|
| | | AA | DIQMTQSPSSVSASVGDRVTITCRASQGISRWLA WYQQKPGKAPKLLIYGASSLQSGVPSRFSGSGS GTDFTLTISSLQPEDFATYYCQQANSFPFTFGQG TKVDIK | QVQLVQSGAEVKKPGASVKVSCKASGYTFTDYYL HWVRQAPGQGLEWMGWIHPNSGGTNYAQKFQGR VTMTRDTSISTAYMELSSLRSDDTAVYYCARDGTS SFDYWGQGTLVTVSS |
| | | | SEQ ID NO: 27974 | SEQ ID NO: 31980 |
| IPS:442071 | 21-225_4H6.007 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAATTT ATTATTGTCAACAGGCTAACAGTTTCCCATTC ACTTTCGGCCCTGGGACCAAAGTGGATATCAA A | CAGGTGCAGCTGGTACAGTCTGGGGGCTGAGGTG AAGAAGCCTGGGGCCTCAGTGAAGGTCAGCTGC AAGGCTTCTGGATACACCTTCACCGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGTCTGAGATCTGACG ACACGGCCGTGTATTACTGTGCGAGAGATGCTAC CAGCTCGTTTGACTACTGGGGCCAGGGAACCCTG GTCACCGTGTCCTCA |
| | | | SEQ ID NO: 27975 | SEQ ID NO: 31981 |
| | | AA | DIQMTQSPSSVSASVGDRVTITCRASQGISRWLA WYQQKPGKAPKLLIYGASSLQSGVPSRFSGSGS GTDFTLTISSLQPEDFAIYYCQQANSFPFTFGPGT KVDIK | QVQLVQSGAEVKKPGASVKVSCKASGYTFTDYYL HWVRQAPGQGLEWMGWIHPNSGGTNYAQKFQGR VTMTRDTSISTAYMELSSLRSDDTAVYYCARDATS SFDYWGQGTLVTVSS |
| | | | SEQ ID NO: 27976 | SEQ ID NO: 31982 |

FIGURE 50

| iPS:442078 | 21-225_4H6.008 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAATTT ATTATTGTCAACAGGCTAACAGTTTCCCATTC ACTTTCGGCCCTGGGACCAAAGTGGATATCAA A | CAGGTGCAGCTGGTACAGTCTGGGGGCTGAGGTG AAGAAGCCTGGGGCCTCAGTGAAGGTCAGCTGC AAGGCTTCTGGATACACCTTCACCGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGTCTGAGATCTGACG ACACGGCCGTGTATTACTGTGCGAGAAGTGGTAC CAGCTCGTTTGACTACTGGGGCCAGGGAACCCTG GTCACCGTGTCCTCA |
| | | | SEQ ID NO: 27977 | SEQ ID NO: 31983 |
| | | AA | DIQMTQSPSSVSASVGDRVTITCRASQGISRWLA WYQQKPGKAPKLLIYGASSLQSGVPSRFSGSGS GTDFTLTISSLQPEDFAIYYCQQANSFPFTFGPGT KVDIK | QVQLVQSGAEVKKPGASVKVSCKASGYTFTDYYL HWVRQAPGQGLEWMGWIHPNSGGTNYAQKFQGR VTMTRDTSISTAYMELSSLRSDDTAVYYCARSGTSS FDYWGQGTLVTVSS |
| | | | SEQ ID NO: 27978 | SEQ ID NO: 31984 |
| iPS:442085 | 21-225_4H6.009 | NA | GACATCCAGATGACCCAGTCTCCATCTTCCGT GTCTGCATCTGTAGGAGATAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAATTT ATTATTGTCAACAGGCTAACAGTTTCCCATTC ACTTTCGGCCAAGGGACCAAAGTGGATATCAA A | CAGGTGCAGCTGGTACAGTCTGGGGGCTGAGGTG AAGAAGCCTGGGGCCTCAGTGAAGGTCAGCTGC AAGGCTTCTGGATACACCTTCACCGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGTCTGAGATCTGACG ACACGGCCGTGTATTACTGTGCGAGAAGTGGTAC CAGCTCGTTTGACTACTGGGGCCAGGGAACCCTG GTCACCGTGTCCTCA |
| | | | SEQ ID NO: 27979 | SEQ ID NO: 31985 |

FIGURE 50

| | | | | |
|---|---|---|---|---|
| | | AA | DIQMTQSPSSVSASVGDRVTITCRASQGISRWLA WYQQKPGKAPKLLIYGASSLQSGVPSRFSGSGS GTDFTLTISSLQPEDFAIYYCQQANSFPFTFGQGT KVDIK | QVQLVQSGAEVKKPGASVKVSCKASGYTFTDYYL HWVRQAPGQGLEWMGWIHPNSGGTNYAQKFQGR VTMTRDTSISTAYMELSSLRSDDTAVYYCARSGTSS FDYWGQGTLVTVSS |
| | | | SEQ ID NO: 27980 | SEQ ID NO: 31986 |
| IPS:442089 | 21-225_4H6.010 | NA | GACATCCAGATGACCCAGTCTCCATCTTCCGT GTCTGCATCTGTAGGAGATAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAATTT ATTATTGTCAACAGGCTAACAGTTTCCCATTC ACTTTCGGCCAAGGGACCAAAGTGGATATCAA A | CAGGTGCAGCTGGTACAGTCTGGGGGCTGAGGTG AAGAAGCCTGGGGCCTCAGTGAAGGTCAGCTGC AAGGCTTCTGGATACACCTTCACCGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGTCTGAGATCTGACG ACACGGCCGTGTATTACTGTGCGAGAGATGCTAC CAGCTCGTTTGACTACTGGGGCCAGGGAACCCTG GTCACCGTGTCCTCA |
| | | | SEQ ID NO: 27981 | SEQ ID NO: 31987 |
| | | AA | DIQMTQSPSSVSASVGDRVTITCRASQGISRWLA WYQQKPGKAPKLLIYGASSLQSGVPSRFSGSGS GTDFTLTISSLQPEDFAIYYCQQANSFPFTFGQGT KVDIK | QVQLVQSGAEVKKPGASVKVSCKASGYTFTDYYL HWVRQAPGQGLEWMGWIHPNSGGTNYAQKFQGR VTMTRDTSISTAYMELSSLRSDDTAVYYCARDATS SFDYWGQGTLVTVSS |
| | | | SEQ ID NO: 27982 | SEQ ID NO: 31988 |

FIGURE 50

| iPS:442093 | 21-225_4H6.011 | NA | GACATCCAGATGACCCAGTCTCCATCTTCCGT GTCTGCATCTGTAGGAGACAGAGTCACCATCA CTTGTCGGGCGAGTCAGGGTATTAGCAGGTGG TTAGCCTGGTATCAGCAGAAACCAGGGAAAG CCCCTAAGCTCCTGATCTATGGTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ATTATTGTCAACAGGCTAACAGTTTCCCATTC ACTTTCGGCCAAGGGACCAAAGTGGATATCAA A | CAGGTGCAGCTGGTACAGTCTGGGGGCTGAGGTG AAGAAGCCTGGGGCCTCAGTGAAGGTCAGCTGC AAGGCTTCTGGATACACCTTCACCGACTACTATT TGCACTGGGTGCGACAGGCCCCTGGACAAGGTCT TGAGTGGATGGGATGGATCCACCCTAACAGTGGT GGCACAAACTATGCACAGAAGTTTCAGGGCAGG GTCACCATGACCAGGGACACGTCCATCAGCACA GCCTACATGGAGCTGAGCAGTCTGAGATCTGACG ACACGGCCGTGTATTACTGTGCGAGAGATGCTAC CAGCTCGTTTGACTACTGGGGCCAGGGAACCCTG GTCACCGTGTCCTCA |
| | | | SEQ ID NO: 27983 | SEQ ID NO: 31989 |
| | | AA | DIQMTQSPSSVSASVGDRVTITCRASQGISRWLA WYQQKPGKAPKLLIYGASSLQSGVPSRFSGSGS GTDFTLTISSLQPEDFATYYCQQANSFPFTFGQG TKVDIK | QVQLVQSGAEVKKPGASVKVSCKASGYTFTDYYL HWVRQAPGQGLEWMGWIHPNSGGTNYAQKFQGR VTMTRDTSISTAYMELSSLRSDDTAVYYCARDATS SFDYWGQGTLVTVSS |
| | | | SEQ ID NO: 27984 | SEQ ID NO: 31990 |
| iPS:442115 | 21-225_5E5.003 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGCAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTGGTACGACTACGGTATGGACGTC TGGGGCCAAGGGACCACGGTCACCGTGTCCTCA |
| | | | SEQ ID NO: 27985 | SEQ ID NO: 31991 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDASNKYYADSVKGR FTISRDNSKNTLYLQMNSLRAEDTAVYYCTREVYS SGWYDYGMDVWGQGTTVTVSS |
| | | | SEQ ID NO: 27986 | SEQ ID NO: 31992 |
| iPS:442122 | 21-225_5E5.004 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGGAAGT AATAAATACTATGCAGAATCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTGGTACGACTACGGTATGGACGTC TGGGGCCAAGGGACCACGGTCACCGTGTCCTCA |
| | | | SEQ ID NO: 27987 | SEQ ID NO: 31993 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDGSNKYYAESVKGR FTISRDNSKNTLYLQMNSLRAEDTAVYYCTREVYS SGWYDYGMDVWGQGTTVTVSS |
| | | | SEQ ID NO: 27988 | SEQ ID NO: 31994 |

FIGURE 50

| iPS:442129 | 21-225_5E5.005 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGGAAGT AATAAATACTATGCAGGCTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTGGTACGACTACGGTATGGACGTC TGGGGCCAAGGGACCACGGTCACCGTGTCCTCA |
| | | | SEQ ID NO: 27989 | SEQ ID NO: 31995 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDGSNKYYAGSVKGR FTISRDNSKNTLYLQMNSLRAEDTAVYYCTREVYS SGWYDYGMDVWGQGTTVTVSS |
| | | | SEQ ID NO: 27990 | SEQ ID NO: 31996 |
| iPS:442136 | 21-225_5E5.006 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGGAAGT AATAAATACTATGCAGACGCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTGGTACGACTACGGTATGGACGTC TGGGGCCAAGGGACCACGGTCACCGTGTCCTCA |
| | | | SEQ ID NO: 27991 | SEQ ID NO: 31997 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDGSNKYYADAVKG RFTISRDNSKNTLYLQMNSLRAEDTAVYYCTREVY SSGWYDYGMDVWGQGTTVTVSS |
| | | | SEQ ID NO: 27992 | SEQ ID NO: 31998 |
| iPS:442171 | 21-225_5E5.011 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGCAAGT AATAAATACTATGCAGAATCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTGGTACGACTACGGTATGGACGTC TGGGGCCAAGGGACCACGGTCACCGTGTCCTCA |
| | | | SEQ ID NO: 27993 | SEQ ID NO: 31999 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDASNKYYAESVKGR FTISRDNSKNTLYLQMNSLRAEDTAVYYCTREVYS SGWYDYGMDVWGQGTTVTVSS |
| | | | SEQ ID NO: 27994 | SEQ ID NO: 32000 |

FIGURE 50

| iPS:442178 | 21-225_5E5.012 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGCAAGT AATAAATACTATGCAGACGCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTGGTACGACTACGGTATGGACGTC TGGGGCCAAGGGACCACGGTCACCGTGTCCTCA |
|---|---|---|---|---|
| | | | SEQ ID NO: 27995 | SEQ ID NO: 32001 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDASNKYYADAVKG RFTISRDNSKNTLYLQMNSLRAEDTAVYYCTREVY SSGWYDYGMDVWGQGTTVTVSS |
| | | | SEQ ID NO: 27996 | SEQ ID NO: 32002 |
| iPS:442199 | 21-225_5E5.015 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGCAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTATTACGACTACGGTATGGACGTC TGGGGCCAAGGGACCACGGTCACCGTGTCCTCA |
| | | | SEQ ID NO: 27997 | SEQ ID NO: 32003 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDASNKYYADSVKGR FTISRDNSKNTLYLQMNSLRAEDTAVYYCTREVYS SGYYDYGMDVWGQGTTVTVSS |
| | | | SEQ ID NO: 27998 | SEQ ID NO: 32004 |
| iPS:442206 | 21-225_5E5.016 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGCAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTTCTACGACTACGGTATGGACGTC TGGGGCCAAGGGACCACGGTCACCGTGTCCTCA |
| | | | SEQ ID NO: 27999 | SEQ ID NO: 32005 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDASNKYYADSVKGR FTISRDNSKNTLYLQMNSLRAEDTAVYYCTREVYS SGFYDYGMDVWGQGTTVTVSS |
| | | | SEQ ID NO: 28000 | SEQ ID NO: 32006 |

FIGURE 50

| iPS:442213 | 21-225_5E5.017 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGGAAGT AATAAATACTATGCAGAATCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTATTACGACTACGGTATGGACGTC TGGGGCCAAGGGACCACGGTCACCGTGTCCTCA |
|---|---|---|---|---|
| | | | SEQ ID NO: 28001 | SEQ ID NO: 32007 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDGSNKYYAESVKGR FTISRDNSKNTLYLQMNSLRAEDTAVYYCTREVYS SGYYDYGMDVWGQGTTVTVSS |
| | | | SEQ ID NO: 28002 | SEQ ID NO: 32008 |
| iPS:442220 | 21-225_5E5.018 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTATTACGACTACGGTATGGACGTC TGGGGCCAAGGGACCACGGTCACCGTGTCCTCA |
| | | | SEQ ID NO: 28003 | SEQ ID NO: 32009 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDGSNKYYADSVKGR FTISRDNSKNTLYLQMNSLRAEDTAVYYCTREVYS SGYYDYGMDVWGQGTTVTVSS |
| | | | SEQ ID NO: 28004 | SEQ ID NO: 32010 |
| iPS:442227 | 21-225_5E5.019 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTTCTACGACTACGGTATGGACGTC TGGGGCCAAGGGACCACGGTCACCGTGTCCTCA |
| | | | SEQ ID NO: 28005 | SEQ ID NO: 32011 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDGSNKYYADSVKGR FTISRDNSKNTLYLQMNSLRAEDTAVYYCTREVYS SGFYDYGMDVWGQGTTVTVSS |
| | | | SEQ ID NO: 28006 | SEQ ID NO: 32012 |

FIGURE 50

| iPS:442255 | 21-225_5E5.023 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGCAAGT AATAAATACTATGCAGAATCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTATTACGACTACGGTATGGACGTC TGGGGCCAAGGGACCACGGTCACCGTGTCCTCA |
| | | | SEQ ID NO: 28007 | SEQ ID NO: 32013 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDASNKYYAESVKGR FTISRDNSKNTLYLQMNSLRAEDTAVYYCTREVYS SGYYDYGMDVWGQGTTVTVSS |
| | | | SEQ ID NO: 28008 | SEQ ID NO: 32014 |
| iPS:442262 | 21-225_5E5.024 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGCAAGT AATAAATACTATGCAGACGCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTATTACGACTACGGTATGGACGTC TGGGGCCAAGGGACCACGGTCACCGTGTCCTCA |
| | | | SEQ ID NO: 28009 | SEQ ID NO: 32015 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDASNKYYADAVKG RFTISRDNSKNTLYLQMNSLRAEDTAVYYCTREVY SSGYYDYGMDVWGQGTTVTVSS |
| | | | SEQ ID NO: 28010 | SEQ ID NO: 32016 |
| iPS:442269 | 21-225_5E5.025 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | GAGGTGCAGCTGGTGGAGTCTGGGGGAGGCGTG GTCCAGCCTGGGGGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGGAAGT AATAAATACTATGCAGAATCCGTGAAGGGCCGA TTCACCATCTCCAGAGACAATGCCAAGAACACGC TGTATCTGCAAATGAACAGCCTGAGAGCCGAGG ACACGGCTGTGTATTACTGTACGAGAGAGGTATA TAGCAGTGGCTGGTACGACTACGGTATGGACGTC TGGGGCCAAGGGACCACGGTCACCGTGTCCTCA |
| | | | SEQ ID NO: 28011 | SEQ ID NO: 32017 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | EVQLVESGGGVVQPGGSLRLSCAASGFTFSNYVMH WVRQAPGKGLEWVAVIWYDGSNKYYAESVKGRF TISRDNAKNTLYLQMNSLRAEDTAVYYCTREVYSS GWYDYGMDVWGQGTTVTVSS |
| | | | SEQ ID NO: 28012 | SEQ ID NO: 32018 |

FIGURE 50

| | | | | |
|---|---|---|---|---|
| iPS:442311 | 21-225_7E11.001.001 | NA | GACATCCAGATGACCCAGTCTCCATCCTCCCT GTCTGCATCTGTAGGAGACAGAGTCACCATTA CTTGCCGGGCAAGTCAAAACATTATCAGCTAT TTAAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGATGGAAGT AATAAATACTATGCAGACTCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGCCAAGGGAC CACGGTCACCGTGTCCTCA |
| | | | SEQ ID NO: 28013 | SEQ ID NO: 32019 |
| | | AA | DIQMTQSPSSLSASVGDRVTITCRASQNIISYLNW YQQKPGKAPKFLIYTASSLQSGVPSRFSGSGSGT DFTLTISSLQPEDFAIYYCQQTYSTPLTFGGGTKV EIK | QVQLVESGGGVVQPGRSLRLSCAASGFTFSSFGMH WVRQAPGKGLEWVAIIWHDGSNKYYADSVKGRFT ISRDNSKNTLYLQMSSLRAEDTAVYYCARDLSMGG MDVWGQGTTVTVSS |
| | | | SEQ ID NO: 28014 | SEQ ID NO: 32020 |
| iPS:442317 | 21-225_7E11.001.002 | NA | GACATCCAGATGACCCAGTCTCCATCCTCCCT GTCTGCATCTGTAGGAGACAGAGTCACCATTA CTTGCCGGGCAAGTCAAAACATTATCAGCTAT TTAAAATTGGTATCAACAGAAACCAGGGAAAG CCCCTAAACTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGATGGAAGT AATAAATACTATGCAGACTCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGCCAAGGGAC CACGGTCACCGTGTCCTCA |
| | | | SEQ ID NO: 28015 | SEQ ID NO: 32021 |

FIGURE 50

| | | | | |
|---|---|---|---|---|
| | | AA | DIQMTQSPSSLSASVGDRVTITCRASQNIISYLNW YQQKPGKAPKLLIYTASSLQSGVPSRFSGSGSGT DFTLTISSLQPEDFATYYCQQTYSTPLTFGGGTK VEIK | QVQLVESGGGVVQPGRSLRLSCAASGFTFSSFGMH WVRQAPGKGLEWVAIIWHDGSNKYYADSVKGRFT ISRDNSKNTLYLQMSSLRAEDTAVYYCARDLSMGG MDVWGQGTTVTVSS |
| | | | SEQ ID NO: 28016 | SEQ ID NO: 32022 |
| IPS:442323 | 21-225_7E11.001.003 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATAGTGGAAGT AATAAATACTATGCAGACTCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGGCCAAGGGAC CACGGTCACCGTGTCCTCA |
| | | | SEQ ID NO: 28017 | SEQ ID NO: 32023 |
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHSGSNKYYADSVKGRFT ISRDNSKNTLYLQMSSLRAEDTAVYYCARDLSMGG MDVWGQGTTVTVSS |
| | | | SEQ ID NO: 28018 | SEQ ID NO: 32024 |

FIGURE 50

| | | | | |
|---|---|---|---|---|
| iPS:442330 | 21-225_7E11.001.004 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGAAGGAAG TAATAAATACTATGCAGACTCCGTGAAGGGCCG ATTCACCATCTCCAGAGACAATTCCAAAAACACG CTGTATCTGCAAATGAGCAGCCTGCGAGCCGAG GACACGGCTGTGTATTACTGTGCGAGAGATCTGA GTATGGGCGGTATGGACGTCTGGGGCCAAGGGA CCACGGTCACCGTGTCCTCA |
| | | | SEQ ID NO: 28019 | SEQ ID NO: 32025 |
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHEGSNKYYADSVKGRFT ISRDNSKNTLYLQMSSLRAEDTAVYYCARDLSMGG MDVWGQGTTVTVSS |
| | | | SEQ ID NO: 28020 | SEQ ID NO: 32026 |
| iPS:442337 | 21-225_7E11.001.005 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGATGCAAGT AATAAATACTATGCAGACTCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGCCAAGGGAC CACGGTCACCGTGTCCTCA |
| | | | SEQ ID NO: 28021 | SEQ ID NO: 32027 |

FIGURE 50

| | | | | |
|---|---|---|---|---|
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHDASNKYYADSVKGRFT ISRDNSKNTLYLQMSSLRAEDTAVYYCARDLSMGG MDVWGQGTTVTVSS |
| | | | SEQ ID NO: 28022 | SEQ ID NO: 32028 |
| iPS:442344 | 21-225_7E11.001.006 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGATGGAAGT AATAAATACTATGCAGAATCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGCCAAGGGAC CACGGTCACCGTGTCCTCA |
| | | | SEQ ID NO: 28023 | SEQ ID NO: 32029 |
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHDGSNKYYAESVKGRFT ISRDNSKNTLYLQMSSLRAEDTAVYYCARDLSMGG MDVWGQGTTVTVSS |
| | | | SEQ ID NO: 28024 | SEQ ID NO: 32030 |

FIGURE 50

| iPS:442351 | 21-225_7E11.001.007 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGATGGAAGT AATAAATACTATGCAGACGCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGCCAAGGGAC CACGGTCACCGTGTCCTCA |
|---|---|---|---|---|
| | | | SEQ ID NO: 28025 | SEQ ID NO: 32031 |
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHDGSNKYYADAVKGRF TISRDNSKNTLYLQMSSLRAEDTAVYYCARDLSMG GMDVWGQGTTVTVSS |
| | | | SEQ ID NO: 28026 | SEQ ID NO: 32032 |
| iPS:442358 | 21-225_7E11.001.008 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGAAGGAAG TAATAAATACTATGCAGACGCCGTGAAGGGCCG ATTCACCATCTCCAGAGACAATTCCAAAAACACG CTGTATCTGCAAATGAGCAGCCTGCGAGCCGAG GACACGGCTGTGTATTACTGTGCGAGAGATCTGA GTATGGGCGGTATGGACGTCTGGGGCCAAGGGA CCACGGTCACCGTGTCCTCA |
| | | | SEQ ID NO: 28027 | SEQ ID NO: 32033 |

FIGURE 50

| | | | | |
|---|---|---|---|---|
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHEGSNKYYADAVKGRF TISRDNSKNTLYLQMSSLRAEDTAVYYCARDLSMG GMDVWGQGTTVTVSS |
| | | | SEQ ID NO: 28028 | SEQ ID NO: 32034 |
| iPS:442365 | 21-225_7E11.001.009 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGAAGGAAG TAATAAATACTATGCAGAATCCGTGAAGGGCCG ATTCACCATCTCCAGAGACAATTCCAAAAACACG CTGTATCTGCAAATGAGCAGCCTGCGAGCCGAG GACACGGCTGTGTATTACTGTGCGAGAGATCTGA GTATGGGCGGTATGGACGTCTGGGGCCAAGGGA CCACGGTCACCGTGTCCTCA |
| | | | SEQ ID NO: 28029 | SEQ ID NO: 32035 |
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHEGSNKYYAESVKGRFT ISRDNSKNTLYLQMSSLRAEDTAVYYCARDLSMGG MDVWGQGTTVTVSS |
| | | | SEQ ID NO: 28030 | SEQ ID NO: 32036 |

FIGURE 50

| iPS:442372 | 21-225_7E11.001.010 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATAGTGGAAGT AATAAATACTATGCAGAATCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGGCCAAGGGAC CACGGTCACCGTGTCCTCA |
| | | | SEQ ID NO: 28031 | SEQ ID NO: 32037 |
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHSGSNKYYAESVKGRFT ISRDNSKNTLYLQMSSLRAEDTAVYYCARDLSMGG MDVWGQGTTVTVSS |
| | | | SEQ ID NO: 28032 | SEQ ID NO: 32038 |
| iPS:442379 | 21-225_7E11.001.011 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATAGTGGAAGT AATAAATACTATGCAGACGCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGGCCAAGGGAC CACGGTCACCGTGTCCTCA |
| | | | SEQ ID NO: 28033 | SEQ ID NO: 32039 |

FIGURE 50

| | | | | |
|---|---|---|---|---|
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHSGSNKYYADAVKGRFT ISRDNSKNTLYLQMSSLRAEDTAVYYCARDLSMGG MDVWGQGTTVTVSS |
| | | | SEQ ID NO: 28034 | SEQ ID NO: 32040 |
| IPS:442386 | 21-225_7E11.001.012 | NA | GACATCCAGATGACCCAGTCTCCATCCTCCCT GTCTGCATCTGTAGGAGACAGAGTCACCATTA CTTGCCGGGCAAGTCAAAACATTATCAGCTAT TTAAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATAGTGGAAGT AATAAATACTATGCAGACTCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGCCAAGGGAC CACGGTCACCGTGTCCTCA |
| | | | SEQ ID NO: 28035 | SEQ ID NO: 32041 |
| | | AA | DIQMTQSPSSLSASVGDRVTITCRASQNIISYLNW YQQKPGKAPKFLIYTASSLQSGVPSRFSGSGSGT DFTLTISSLQPEDFAIYYCQQTYSTPLTFGGGTKV EIK | QVQLVESGGGVVQPGRSLRLSCAASGFTFSSFGMH WVRQAPGKGLEWVAIIWHSGSNKYYADSVKGRFT ISRDNSKNTLYLQMSSLRAEDTAVYYCARDLSMGG MDVWGQGTTVTVSS |
| | | | SEQ ID NO: 28036 | SEQ ID NO: 32042 |

FIGURE 50

| iPS:442390 | 21-225_7E11.001.013 | NA | GACATCCAGATGACCCAGTCTCCATCCTCCCT GTCTGCATCTGTAGGAGACAGAGTCACCATTA CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGATGGAAGT AATAAATACTATGCAGAATCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGGCCAAGGGAC CACGGTCACCGTGTCCTCA |
| | | | SEQ ID NO: 28037 | SEQ ID NO: 32043 |
| | | AA | DIQMTQSPSSLSASVGDRVTITCRASQNIISYLNW YQQKPGKAPKFLIYTASSLQSGVPSRFSGSGSGT DFTLTISSLQPEDFAIYYCQQTYSTPLTFGGGTKV EIK | QVQLVESGGGVVQPGRSLRLSCAASGFTFSSFGMH WVRQAPGKGLEWVAIIWHDGSNKYYAESVKGRFT ISRDNSKNTLYLQMSSLRAEDTAVYYCARDLSMGG MDVWGQGTTVTVSS |
| | | | SEQ ID NO: 28038 | SEQ ID NO: 32044 |
| iPS:442394 | 21-225_7E11.001.014 | NA | GACATCCAGATGACCCAGTCTCCATCCTCCCT GTCTGCATCTGTAGGAGACAGAGTCACCATTA CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATAGTGGAAGT AATAAATACTATGCAGAATCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGGCCAAGGGAC CACGGTCACCGTGTCCTCA |
| | | | SEQ ID NO: 28039 | SEQ ID NO: 32045 |

FIGURE 50

| | | | | |
|---|---|---|---|---|
| | | AA | DIQMTQSPSSLSASVGDRVTITCRASQNIISYLNW YQQKPGKAPKFLIYTASSLQSGVPSRFSGSGSGT DFTLTISSLQPEDFAIYYCQQTYSTPLTFGGGTKV EIK | QVQLVESGGGVVQPGRSLRLSCAASGFTFSSFGMH WVRQAPGKGLEWVAIIWHSGSNKYYAESVKGRFT ISRDNSKNTLYLQMSSLRAEDTAVYYCARDLSMGG MDVWGQGTTVTVSS |
| | | | SEQ ID NO: 28040 | SEQ ID NO: 32046 |
| IPS:442398 | 21-225_7E11.001.015 | NA | GACATCCAGATGACCCAGTCTCCATCCTCCCT GTCTGCATCTGTAGGAGACAGAGTCACCATTA CTTGCCGGGCAAGTCAAAACATTATCAGCTAT TTAAATTGGTATCAACAGAAACCAGGGAAAG CCCCTAAACTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATAGTGGAAGT AATAAATACTATGCAGACTCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGGCCAAGGGAC CACGGTCACCGTGTCCTCA |
| | | | SEQ ID NO: 28041 | SEQ ID NO: 32047 |
| | | AA | DIQMTQSPSSLSASVGDRVTITCRASQNIISYLNW YQQKPGKAPKLLIYTASSLQSGVPSRFSGSGSGT DFTLTISSLQPEDFATYYCQQTYSTPLTFGGGTK VEIK | QVQLVESGGGVVQPGRSLRLSCAASGFTFSSFGMH WVRQAPGKGLEWVAIIWHSGSNKYYADSVKGRFT ISRDNSKNTLYLQMSSLRAEDTAVYYCARDLSMGG MDVWGQGTTVTVSS |
| | | | SEQ ID NO: 28042 | SEQ ID NO: 32048 |

FIGURE 50

| iPS:442402 | 21-225_7E11.001.016 | NA | GACATCCAGATGACCCAGTCTCCATCCTCCCT GTCTGCATCGTAGGAGACAGAGTCACCATTA CTTGCCGGGCAAGTCAAAACATTATCAGCTAT TTAAATTGGTATCAACAGAAACCAGGGAAAG CCCCTAAACTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATAGTGGAAGT AATAAATACTATGCAGAATCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGCCAAGGGAC CACGGTCACCGTGTCCTCA |
|---|---|---|---|---|
| | | | SEQ ID NO: 28043 | SEQ ID NO: 32049 |
| | | AA | DIQMTQSPSSLSASVGDRVTITCRASQNIISYLNW YQQKPGKAPKLLIYTASSLQSGVPSRFSGSGSGT DFTLTISSLQPEDFATYYCQQTYSTPLTFGGGTK VEIK | QVQLVESGGGVVQPGRSLRLSCAASGFTFSSFGMH WVRQAPGKGLEWVAIIWHSGSNKYYAESVKGRFT ISRDNSKNTLYLQMSSLRAEDTAVYYCARDLSMGG MDVWGQGTTVTVSS |
| | | | SEQ ID NO: 28044 | SEQ ID NO: 32050 |
| iPS:442406 | 21-225_7E11.001.017 | NA | GACATCCAGATGACCCAGTCTCCATCCTCCCT GTCTGCATCGTAGGAGACAGAGTCACCATTA CTTGCCGGGCAAGTCAAAACATTATCAGCTAT TTAAATTGGTATCAACAGAAACCAGGGAAAG CCCCTAAACTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGAAGGAAG TAATAAATACTATGCAGACGCCGTGAAGGGCCG ATTCACCATCTCCAGAGACAATTCCAAAAACACG CTGTATCTGCAAATGAGCAGCCTGCGAGCCGAG GACACGGCTGTGTATTACTGTGCGAGAGATCTGA GTATGGGCGGTATGGACGTCTGGGGCCAAGGGA CCACGGTCACCGTGTCCTCA |
| | | | SEQ ID NO: 28045 | SEQ ID NO: 32051 |

FIGURE 50

| | | | | |
|---|---|---|---|---|
| | | AA | DIQMTQSPSSLSASVGDRVTITCRASQNIISYLNWYQQKPGKAPKLLIYTASSLQSGVPSRFSGSGSGTDFTLTISSLQPEDFATYYCQQTYSTPLTFGGGTKVEIK | QVQLVESGGGVVQPGRSLRLSCAASGFTFSSFGMHWVRQAPGKGLEWVAIIWHEGSNKYYADAVKGRFTISRDNSKNTLYLQMSSLRAEDTAVYYCARDLSMGGMDVWGQGTTVTVSS |
| | | | SEQ ID NO: 28046 | SEQ ID NO: 32052 |
| iPS:442410 | 21-225_7E11.001.018 | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGCAAGTAATAAATACTATGCAGAATCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28047 | SEQ ID NO: 32053 |
| | | AA | DIQMTQSPSSLSASVGDRVTITCRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDASNKYYAESVKGRFTISRDNSKNTLYLQMSSLRAEDTAVYYCARDLSMGGMDVWGQGTTVTVSS |
| | | | SEQ ID NO: 28048 | SEQ ID NO: 32054 |

FIGURE 50

| iPS:442417 | 21-225_7E11.001.019 | NA | GACATCCAGATGACCCAGTCTCCATCCTCCCTGTCTGCATCGTAGGAGACAGAGTCACCATTGCTTGCCGGGCAAGTCAAAACATTATCAGCTATTTAAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | GAGGTGCAGCTGGTGGAGTCTGGGGGAGGCGTGGTCCAGCCTGGGGGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGGAAGTAATAAATACTATGCAGAATCCGTGAAGGGCCGATTCACCATCTCCAGAGACAATGCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
|---|---|---|---|---|
| | | | SEQ ID NO: 28049 | SEQ ID NO: 32055 |
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | EVQLVESGGGVVQPGGSLRLSCAASGFTFSSFGMHWVRQAPGKGLEWVAIIWHDGSNKYYAESVKGRFTISRDNAKNTLYLQMSSLRAEDTAVYYCARDLSMGGMDVWGQGTTVTVSS |
| | | | SEQ ID NO: 28050 | SEQ ID NO: 32056 |
| iPS:442431 | 21-225_7E11.001.021 | NA | GACATCCAGATGACCCAGTCTCCATCCTCCCTGTCTGCATCGTAGGAGACAGAGTCACCATTGCTTGCCGGGCAAGTCAAAACATTATCAGCTATTTAAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | GAGGTGCAGCTGGTGGAGTCTGGGGGAGGCGTGGTCCAGCCTGGGGGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATAGTGGAAGTAATAAATACTATGCAGAATCCGTGAAGGGCCGATTCACCATCTCCAGAGACAATGCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28051 | SEQ ID NO: 32057 |

FIGURE 50

| | | | | |
|---|---|---|---|---|
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | EVQLVESGGGVVQPGGSLRLSCAASGFTFSSFGMH WVRQAPGKGLEWVAIIWHSGSNKYYAESVKGRFT ISRDNAKNTLYLQMSSLRAEDTAVYYCARDLSMG GMDVWGQGTTVTVSS |
| | | | SEQ ID NO: 28052 | SEQ ID NO: 32058 |
| iPS:442438 | 21-225_7E11.001.022 | NA | GACATCCAGATGACCCAGTCTCCATCCTCCCT GTCTGCATCTGTAGGAGACAGAGTCACCATTA CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | GAGGTGCAGCTGGTGGAGTCTGGGGGAGGCGTG GTCCAGCCTGGGGGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGAAGGAAG TAATAAATACTATGCAGAATCCGTGAAGGGCCG ATTCACCATCTCCAGAGACAATGCCAAAAACAC GCTGTATCTGCAAATGAGCAGCCTGCGAGCCGA GGACACGGCTGTGTATTACTGTGCGAGAGATCTG AGTATGGGCGGTATGGACGTCTGGGGCCAAGGG ACCACGGTCACCGTGTCCTCA |
| | | | SEQ ID NO: 28053 | SEQ ID NO: 32059 |
| | | AA | DIQMTQSPSSLSASVGDRVTITCRASQNIISYLNW YQQKPGKAPKFLIYTASSLQSGVPSRFSGSGSGT DFTLTISSLQPEDFAIYYCQQTYSTPLTFGGGTKV EIK | EVQLVESGGGVVQPGGSLRLSCAASGFTFSSFGMH WVRQAPGKGLEWVAIIWHEGSNKYYAESVKGRFT ISRDNAKNTLYLQMSSLRAEDTAVYYCARDLSMG GMDVWGQGTTVTVSS |
| | | | SEQ ID NO: 28054 | SEQ ID NO: 32060 |

FIGURE 50

| iPS:442568 | 21-225_149D8 | NA | GAAATTGTGTTGACGCAGTCTCCAGGCACCCT GTCTTTGTTTCCAGGGGAAAGAGCCACCCTCT CCTGCAGGGCCAGTCAGAGTGTGATCAGCAGC TACTTAGCCTGGTACCAGCAGAAACCTGGCCA GGCTCCCAGGCTCCTCATCTTTGGTGTATCTAG TTGGGCCACTGGCATCCCAGACAGGTTCAGTG GCAGTGGGTCTGGGACAGACTTCACTCTCACC ATCAGCAGACTGGAGCCTGAAGATTTTGCAGT GTATTACTGTCAACAATATGGTAGGTCACCAT TCAATTTCGGCCCTGGGACCAAAGTGGATATC AAA | CAGGTGCAGCTGCAGGAGTCGGGCCCAGGACTG GTGAAGCCTTCACAGACCCTGTCCCTCAATTGCA CTGTCTCTGGTGGCTCCATCAGCAACAGTGGTTA CTACTGGAGCTGGATCCGCCAGCACCCAGGGAA GGGCCTGGAATGGATTGGGTACAGCTATTACAGT GGGAGCACCTACTACAACCCGTCCCTCAAGAGTC GAATTACCATATCAGTAGACACGTCTAACAACCA GTTCTCCCTGAAGCTGAGCTCTGTGACTGCCGCG GACACGGCCGTGTATTACTGTGCGAGAGGGGGA TATAACTGGAACCATGCTTTTGATATCTGGGGCC AAGGGACAATGGTCACCGTCTCTTCA |
|  |  |  | SEQ ID NO: 28055 | SEQ ID NO: 32061 |
|  |  | AA | EIVLTQSPGTLSLFPGERATLSCRASQSVISSYLA WYQQKPGQAPRLLIFGVSSWATGIPDRFSGSGSG TDFTLTISRLEPEDFAVYYCQQYGRSPFNFGPGT KVDIK | QVQLQESGPGLVKPSQTLSLNCTVSGGSISNSGYY WSWIRQHPGKGLEWIGYSYYSGSTYYNPSLKSRITI SVDTSNNQFSLKLSSVTAADTAVYYCARGGYNWN HAFDIWGQGTMVTVSS |
|  |  |  | SEQ ID NO: 28056 | SEQ ID NO: 32062 |
| iPS:443003 | 21-225_43F11_LC2 | NA | CAGTCTGTGCTGACGCAGCCGCCCTCAGTGTC TGGGGCCCCAGGGCAGAGGGTCACCATCTCCT GCACTGGGAGCAGCTCCAACATCGGGGCAGG TTATGATGTACACTGGTACCAGCAGCTTCCAG GAACAGCCCCCAAACTCCTCATCTATGGTAAC AGCAATCGGCCCTCAGGGGTCCCTGACCGATT CTCTGGCTCCAAGTCTGGCACCTCAGCCTCCCT GGCCATCACTGGGCTCCAGGCTGAGGATGAGG CTGATTATTACTGCCAGTCCTATGACAACAGC CTGAGTGGTTCGGTATTCGGCGGAGGGACCAA GCTGACCGTCCTA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGCC TTGAGTGGATGGGATGGATCAACCCTAACAGTGG TGGCACAAACTATGCACAGAAGTTTCAGGGCGG GGTCACCATGACCAGGCTCACGTCCATCAACACA GCCTACATGGACCTGAGCAGGCTGAGATCTGAC GACACGGCCGTGTATTACTGTGCGAGAGGAGGG AATTACTTCTACAACCACGTTATGGACGTCTGGG GCCAAGGGACCCCGGTCACCGTCTCCTCA |
|  |  |  | SEQ ID NO: 28057 | SEQ ID NO: 32063 |

FIGURE 50

| | | | | |
|---|---|---|---|---|
| | | AA | QSVLTQPPSVSGAPGQRVTISCTGSSSNIGAGYD VHWYQQLPGTAPKLLIYGNSNRPSGVPDRFSGS KSGTSASLAITGLQAEDEADYYCQSYDNSLSGS VFGGGTKLTVL | QVQLVQSGAEVKKPGASVKVSCKASGYTFTGYYIH WVRQAPGQGLEWMGWINPNSGGTNYAQKFQGGV TMTRLTSINTAYMDLSRLRSDDTAVYYCARGGNYF YNHVMDVWGQGTPVTVSS |
| | | | SEQ ID NO: 28058 | SEQ ID NO: 32064 |
| iPS:443005 | 21-225_43F11_LC1 | NA | GATGTTGTGATGACTCAGTCTCCACTCTCCCTG CCCGTCACCCTTGGACAGCCGGCCTCCATCTC CTGCAGGTCTAGTCAAAGCCTCGTATACAGTG ATGGAAACACCTACTTGAATTGGTTTCAGCAG AGGCCAGGCCAATCTCCAAGGCGCCTAATTTA TAAGGTTTCTAACTGGGACTCTGGGGTCCCAG ACAGATTCAGCGGCAGTGGGTCAGGCACTGAT TTCACACTGAAAATCAGCAGGGTGGAGGCTGA GGATGTTGGGGTTTATTACTGCATGCAAGGTA CACACTGGCCGCTCACTTTCGGCGGAGGGACC AAGGTGGAGATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCGGCTACTATA TACACTGGGTGCGACAGGCCCCTGGACAAGGCC TTGAGTGGATGGGATGGATCAACCCTAACAGTGG TGGCACAAACTATGCACAGAAGTTTCAGGGCGG GGTCACCATGACCAGGCTCACGTCCATCAACACA GCCTACATGGACCTGAGCAGGCTGAGATCTGAC GACACGGCCGTGTATTACTGTGCGAGAGGAGGG AATTACTTCTACAACCACGTTATGGACGTCTGGG GCCAAGGGACCCCGGTCACCGTCTCCTCA |
| | | | SEQ ID NO: 28059 | SEQ ID NO: 32065 |
| | | AA | DVVMTQSPLSLPVTLGQPASISCRSSQSLVYSDG NTYLNWFQQRPGQSPRRLIYKVSNWDSGVPDRF SGSGSGTDFTLKISRVEAEDVGVYYCMQGTHWP LTFGGGTKVEIK | QVQLVQSGAEVKKPGASVKVSCKASGYTFTGYYIH WVRQAPGQGLEWMGWINPNSGGTNYAQKFQGGV TMTRLTSINTAYMDLSRLRSDDTAVYYCARGGNYF YNHVMDVWGQGTPVTVSS |
| | | | SEQ ID NO: 28060 | SEQ ID NO: 32066 |

2720

FIGURE 50

| iPS:443006 | 21-225_25A4.001.029 | NA | GACATCGTGATGACCCAGTTTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTCAAACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 28061 | SEQ ID NO: 32067 |
| | | AA | DIVMTQFPDSLAVSLGERATIKCKSSQSVLYSSHNNNYLAWYQQKPGQPPKLLLYWASTRESGVPDRFSGSGSGTEFTLTISSLQAEDVAVYYCQQYYSTPPTFGQGTKVEIK | QVLLVQSGAEVKRPGASVKVSCKASGYTFTNYDINWVRQATGQGLEWMGWMYPNSGQTGYAQKFQGRVTMTRDTSISTAYMELSSLRSEDTAVYYCASSSGWYYFDYWGQGTLVTVSS |
| | | | SEQ ID NO: 28062 | SEQ ID NO: 32068 |
| iPS:443016 | 21-225_4H6.014 | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTATTATTGTCAACAGGCTAACAGTTTCCCATTCACTTTCGGCCCTGGGACCAAAGTGGATATCAAA | CAGGTGCAGCTGGTACAGTCTGGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGGGCTGAGCAGTCTGAGATCTGACGACACGGCCGTGTATTACTGTGCGAGAGATGCTACCAGCTCGTTTGACTACTGGGGCCAGGGAACCCTGGTCACCGTGTCCTCA |
| | | | SEQ ID NO: 28063 | SEQ ID NO: 32069 |

FIGURE 50

| | | | | |
|---|---|---|---|---|
| | | AA | DIQMTQSPSSVSASVGDRVTITCRASQGISRWLA WYQQKPGKAPKLLIYGASSLQSGVPSRFSGSGS GTDFTLTISSLQPEDFATYYCQQANSFPFTFGPGT KVDIK | QVQLVQSGAEVKKPGASVKVSCKASGYTFTDYYL HWVRQAPGQGLEWMGWIHPNSGGTNYAQKFQGR VTMTRDTSISTAYMGLSSLRSDDTAVYYCARDATS SFDYWGQGTLVTVSS |
| | | | SEQ ID NO: 28064 | SEQ ID NO: 32070 |
| iPS:443027 | 21-225_7E11.001.023 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGATGCAAGT AATAAATACTATGCAGACGCCGTGAAGGGCCGA TTCACCATCTCCAGAGACAATTCCAAAAACACGC TGTATCTGCAAATGAGCAGCCTGCGAGCCGAGG ACACGGCTGTGTATTACTGTGCGAGAGATCTGAG TATGGGCGGTATGGACGTCTGGGGCCAAGGGAC CACGGTCACCGTGTCCTCA |
| | | | SEQ ID NO: 28065 | SEQ ID NO: 32071 |
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHDASNKYYADAVKGRF TISRDNSKNTLYLQMSSLRAEDTAVYYCARDLSMG GMDVWGQGTTVTVSS |
| | | | SEQ ID NO: 28066 | SEQ ID NO: 32072 |

FIGURE 50

| | | | | |
|---|---|---|---|---|
| iPS:446086 | 21-225_94D8 | NA | GACATCGTGTTGACCCAGTCGCCAGACTCCCT GGCTGTGTCTCTGGGCGAGAGGGCCACCATCA ACTGCAAGTCCAGACAGAGTGTTTTATACAGC TCCAACAATTACAACTACTTAACTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGACGTGGCAGTTTATTACTGTCAGCAA TATTATAGTTCTCCTCCTACTTTCGGCGGAGGG ACCAAGGTGGAGATCAAA | CAGGTGCAGCTGGTGCAGTCTGGGGCTGAGGTG AAGAAGCCTGGGGCCTCAGTGAAGGTCTCCTGC AAGACTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGTCA GAGTCACCATGACCAGGAACACCTCCATAAGCA CAGCCTACATGGAGGTGAGCAGCCTGAGATCTG AGGACACGGCCGTCTATTACTGTGCGTATAGCAG TGGCTGGTACATCTTTGACTACTGGGGCCAGGGA ACCCTGGTCACCGTCTCTTCA |
| | | | SEQ ID NO: 28067 | SEQ ID NO: 32073 |
| | | AA | DIVLTQSPDSLAVSLGERATINCKSRQSVLYSSN NYNYLTWYQQKPGQPPKLLIYWASTRESGVPDR FSGSGSGTDFTLTISSLQAEDVAVYYCQQYYSSP PTFGGGTKVEIK | QVQLVQSGAEVKKPGASVKVSCKTSGYTFTNYDIN WVRQATGQGLEWMGWMNPNSGNTGYAQKFQVR VTMTRNTSISTAYMEVSSLRSEDTAVYYCAYSSGW YIFDYWGQGTLVTVSS |
| | | | SEQ ID NO: 28068 | SEQ ID NO: 32074 |
| iPS:446094 | 21-225_77E1 | NA | GACATCGTGATGACCCAGTCTCCAGACTCCCT GGCTGTGTCTCTGGGCGAGAGGGCCACCATCA ACTGCAAGTCCAGCCAGACTGTCTTACACAGC TCCAACAATTATAACTACTTAGCTTGGTACCA GCAGAAGCCAGGACAGCCCCCTAAGGTGCTC ATTTACTGGACATCTACCCGGGAATCCGGGGT CCATGACCGATTCAGTGGCAGCGGGTCTGGGA CAGATTTCACTCTCACCATCAGCAGCCTGCAG GCTGAAGATGTGGCAGTTTATTACTGTCACCA ATATCTTAGTAGTCCTCTGACGTTCGGCCAAG GGACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCCCCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTATTGTGCGGTTTCCAG TGGCTGGCACTGGTTCGACCCCTGGGGCCAGGGA ACCCTGGTCACCGTCGCCTCA |
| | | | SEQ ID NO: 28069 | SEQ ID NO: 32075 |

FIGURE 50

| | | | | |
|---|---|---|---|---|
| | | AA | DIVMTQSPDSLAVSLGERATINCKSSQTVLHSSN NYNYLAWYQQKPGQPPKVLIYWTSTRESGVHD RFSGSGSGTDFTLTISSLQAEDVAVYYCHQYLSS PLTFGQGTKVEIK | QVQLVQSGAEVKKPGASVKVSCKASGYTFPNYDIN WVRQATGQGLEWMGWMNPNSGNTGYAQKFQGR VTMTRNTSISTAYMELSSLRSEDTAVYYCAVSSGW HWFDPWGQGTLVTVAS |
| | | | SEQ ID NO: 28070 | SEQ ID NO: 32076 |
| IPS:448904 | 21-225_65C12 | NA | GAGATAGTGATGACGCAGTCTCCAGCCACCCT GTCTGTGTTTCCAGGGGAAGGAGCCACCCTCT CCTGCAGGGCCAGTCAGAGTGTTAGCATCAAC TTAGCCTGGTACCAGCAGAAACCTGGCCAGGC TCCCAGACTCCTCATCTATGGTGCATCCACCA GGGCCACTGGTATCCCAGCCAGGTTCAATGCC AGTGGGTCTGGGACAGAGTTCACTCTCTCCAT CAGCAGCCTGCAGTCTGAAAATTTTGCAGTTT ATTACTGTCAGCAGTATAATACCTGGCCTCTC ACTTTCGGCGGAGGGACCAAGGTGGAAATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGGAGCTTTAGCTT GAACTGGGTCCGCCAGGCTCCAGGGAAGGGGCT GGAGTGGGTCTCATCCATTAGTAGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGATGCGTAT AGCCACTACTGGGGCCAGGGAACCCTGGTCACC GTCTCCTCA |
| | | | SEQ ID NO: 28071 | SEQ ID NO: 32077 |
| | | AA | EIVMTQSPATLSVFPGEGATLSCRASQSVSINLA WYQQKPGQAPRLLIYGASTRATGIPARFNASGS GTEFTLSISSLQSENFAVYYCQQYNTWPLTFGGG TKVEIK | EVQLVESGGGLVKPGGSLRLSCAASGFTFRSFSLN WVRQAPGKGLEWVSSISSSSSYIYYADSVKGRFTIS RDNAKNSLYLQMNSLRAEDTAVYYCARDAYSHY WGQGTLVTVSS |
| | | | SEQ ID NO: 28072 | SEQ ID NO: 32078 |

FIGURE 50

| iPS:448906 | 21-225_72G9 | NA | GACATCCAGATGACCCAGTCTCCATCCTCCCT GTCTGCATCTGTAGGAGACAGAGTCACCATCA CTTGCCGGGCAAGTCAGAGCATTACCAGCTAT TTAAATTGGTATCAGCAGAAACCAGGGAAAG CCCCTAAGCTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTCACAGTTTCCCATTC ACTTTCGGCCCTGGGACCAAAGTGGATATCAA A | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGTTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTTCTGTGCGAGAGGGGGTTCG AGGGGGTTCGACCCCTGGGGCCAGGGAACCCTG GTCACCGTCTCCTCA |
| | | | SEQ ID NO: 28073 | SEQ ID NO: 32079 |
| | | AA | DIQMTQSPSSLSASVGDRVTITCRASQSITSYLN WYQQKPGKAPKLLIYTASSLQSGVPSRFSGSGSG TDFTLTISSLQPEDFATYYCQQSHSFPFTFGPGTK VDIK | EVQLVESGGGLVKPGGSLRLSCAASGFTFSSYSMN WVRQAPGKGLEWVSSISGSSSYIYYADSVKGRFTIS RDNAKNSLYLQMNSLRAEDTAVYFCARGGSRGFD PWGQGTLVTVSS |
| | | | SEQ ID NO: 28074 | SEQ ID NO: 32080 |
| iPS:448908 | 21-225_50G9 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TGCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGACCCAGGCTAGGGATGAGGCTGAATATT ACTGTCAGGCGCGGAACAGCCGCAGAGGGGT ATTCGGCGGAGGGACCAGGCTGACCGTCCTA | CAGGTGCAGCTGGTGGAGTCTGGGGGAGGCGTG GTCCAGCCTGGGAGGTCCCTGAGACTCTCCTGTG CAGCCTCCGGATTCATCTTCAGTAGCTATGGCAT GCACTGGGTCCGCCAGGCTCCAGGCAAGGGGCT GGAGTGGGTGGCAGTTATATCACAAGATGGAATT ATTAGATACTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGCGAGAGATGTGAAG CAGTGGCTGGTACGGACCTACGGTATGGACGTCT GGGGCCAAGGGACCACGGTCACCGTCTCCTCA |
| | | | SEQ ID NO: 28075 | SEQ ID NO: 32081 |

FIGURE 50

| | | | | |
|---|---|---|---|---|
| | | AA | SYELTQPPSVSVSPGQTASITCSGDKLGDKYACW YQQKPGQSPVLVIYQDSKRPSGIPERFSGSNSGN TATLTISGTQARDEAEYYCQARNSRRGVFGGGT RLTVL | QVQLVESGGGVVQPGRSLRLSCAASGFIFSSYGMH WVRQAPGKGLEWVAVISQDGIIRYYADSVKGRFTI SRDNSKNTLYLQMNSLRAEDTAVYYCARDVKQW LVRTYGMDVWGQGTTVTVSS |
| | | | SEQ ID NO: 28076 | SEQ ID NO: 32082 |
| iPS:451102 | 21-225_45F6 | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGTAAGCGGC CCTCAGGGATCCCTGAGCGATTCTCTGGCTCC AACTCTGGAAACACAGCCACTCTGACCATCAG CGGGACCCAGGCTATGGATGAGGCTGACTATT ACTGTCAGGCGTGGGACAACAGAACTATGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCCTCTGGATTCACCTTCAGTTACTATGGCTT GCACTGGGTCCGCCAGGCTCCAGGCAAGGGGCT GGAGTGGGTGGCAGTTATATCATATGATGGAAGT AATAAATATTATGCAGACTCCGTGAAGGGCCGAT TCACCATCTCCAGAGACAATTCCAAGAACACGCT GTATCTGCAAATGAACAACCTGAGAGCTGAGGA CACGGCTGTGTTTTACTGTGCGAGAGAGGATCGA TATTGTAGTGGTACCAGCTGCCCCTACTACTACT ACTACGGTATGGACGTCTGGGGCCAAGGGACCA CGGTCACCGTCTCCTCA |
| | | | SEQ ID NO: 28077 | SEQ ID NO: 32083 |
| | | AA | SYELTQPPSVSVSPGQTASITCSGDKLGDKYASW YQQKPGQSPVLVIYQDSKRPSGIPERFSGSNSGN TATLTISGTQAMDEADYYCQAWDNRTMVFGGG TKLTVL | QVQLVESGGGVVQPGRSLRLSCAASGFTFSYYGLH WVRQAPGKGLEWVAVISYDGSNKYYADSVKGRFT ISRDNSKNTLYLQMNNLRAEDTAVFYCAREDRYCS GTSCPYYYYYGMDVWGQGTTVTVSS |
| | | | SEQ ID NO: 28078 | SEQ ID NO: 32084 |

FIGURE 50

| iPS:451104 | 21-225_49C5 | NA | CAGTCTGTGCTGACTCAGCCACCCTCAGCGTC TGGGACCCCCGGGCAGAGGGTCACCATCTCTT GTTCTGGAAGCAGCTCCAACATCGGAAGTAAT ATTGTGACCTGGTACCAGCAGCTCCCAGGAAC GGCCCCCAAACTCCTCATCTATAGTAATGATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTACAGCATGGGATGACAGCCTG AATGGTTGGGTGTTCGGCGGAGGGACCACGCT GACCGTCCTA | CAGGTTCAGCTGGTGCAGTCTGGAGCTGAGGTGA AGAAGCCTGGGGCCTCAGTGAAGGTCTCCTGCA AGGCTTCTGGTTACACCTTTAACAGCTATGGTAT CAGCTGGGTGCGACAGGCCCCTGGACAAGGGCT TGAGTGGATGGGATGGATCAGCGCTTATAATGGT AACACAAAGTATGCACAGAAGCTCCAGGGCAGA GTCACCATGACCACAGACACATCCACGAGCACA GCCTACATGGAGCTGAGGAGCCTGAGATCTGAC GACACGGCCGTGTATTACTGTGCGAGACACGATT TTTGGAGTGGTTATTATAAGGGTATGGACGTCTG GGGCCAAGGGACCACGGTCACCGTCTCCTCA |
| | | | SEQ ID NO: 28079 | SEQ ID NO: 32085 |
| | | AA | QSVLTQPPSASGTPGQRVTISCSGSSSNIGSNIVT WYQQLPGTAPKLLIYSNDQRPSGVPDRFSGSKS GTSASLAISGLQSEDEADYYCTAWDDSLNGWVF GGGTTLTVL | QVQLVQSGAEVKKPGASVKVSCKASGYTFNSYGIS WVRQAPGQGLEWMGWISAYNGNTKYAQKLQGR VTMTTDTSTSTAYMELRSLRSDDTAVYYCARHDF WSGYYKGMDVWGQGTTVTVSS |
| | | | SEQ ID NO: 28080 | SEQ ID NO: 32086 |
| iPS:451106 | 21-225_49D10 | NA | CAGTCTGTGCTGACTCAGCCACCCTCAGCGTC TGGGACCCCCGGGCAGAGGGTCACCATCTCTT GTTCTGGAAGCAACTCCAACATCGGAAGTAAT ATTGTAACCTGGTACCAGCAGCTCCCAGGAAC GGCCCCCAAACTCCTCATCTATAGTAATGATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTGCAGCATGGGATGACAGCCTG AATGGTTGGGTGTTCGGCGGAGGGACCACGCT GACCGTCCTA | CAGGTTCAGCTGGTGCAGTCTGGAGCTGAGGTGA AGAAGCCTGGGGCCTCAGTGAAGGTCTCCTGCA AGGCTTCTGGTTACACCTTTAACAGCTATGGTAT CAGCTGGGTGCGACTGGCCCCTGGACAAGGGTTT GAGTGGATGGGATGGATCAGCGCTTATAATGGTA ACACAAAGAATGCACAGAAGCTCCAGGGCAGAG TCACCATGACCACAGACACATCCACGAGCACAG CCTACATGGAGCTGAGGAGCCTGAGATCTGACG ACACGGCCGTTTATTACTGTGCGAGACACGATTT TTGGAGTGGTTATTATAAGGGTATGGACGTCTGG GGGCCAAGGGACCACGGTCACCGTCTCCTCA |
| | | | SEQ ID NO: 28081 | SEQ ID NO: 32087 |

FIGURE 50

| | | | | |
|---|---|---|---|---|
| | | AA | QSVLTQPPSASGTPGQRVTISCSGSNSNIGSNIVT WYQQLPGTAPKLLIYSNDQRPSGVPDRFSGSKS GTSASLAISGLQSEDEADYYCAAWDDSLNGWV FGGGTTLTVL | QVQLVQSGAEVKKPGASVKVSCKASGYTFNSYGIS WVRLAPGQGFEWMGWISAYNGNTKNAQKLQGRV TMTTDTSTSTAYMELRSLRSDDTAVYYCARHDFW SGYYKGMDVWGQGTTVTVSS |
| | | | SEQ ID NO: 28082 | SEQ ID NO: 32088 |
| iPS:451108 | 21-225_53E8 | NA | CAGTCTGTGCTGACTCAGCCACCCTCAGCGTC TGGGACCCCCGGGCAGAGGGTCACCATCTCTT GTTCTGGAAGCTGCTCCAACATCGGAAGTAAT ATTGTGACCTGGTACCAGCAGCTCCCAGGAAC GGCCCCCAAACTCCTCATCTATAGTAATGATC AGCGGCCCTCAGGGGTCCCTGACCGATTCTCT GGCTCCAAGTCTGGCACCTCAGCCTCCCTGGC CATCAGTGGGCTCCAGTCTGAGGATGAGGCTG ATTATTACTGTACAGCATGGGATGACAGCCTG AATGATTGGGTGTTCGGCGGAGGGACCACGCT GACCGTCCTA | CAGGTTCAGCTGGTGCAGTCTGGAGCTGAGGTGA AGAAGCCTGGGGCCTCAGTGAAGGTCTCCTGCA AGGCTTCTGGTTACACCTTTAACAGCTATGGTAT CAGCTGGGTGCGACAGGCCCCTGGACAAGGGCT TGAGTGGATGGGATGGATCAGCGCTTATAATGGT AACACAAAGTTTGCACAGAAGCTCCAGGGCAGA GTCACCATGACCACAGACACATCCACGAGCACA GCCTACATGGAACTGAGGAGCCTGAGATCTGAC GACACGGCCGTGTATTACTGTGCGAGACACGATT TTTGGAGTGGTTATTATAAGGGTATGGACGTCTG GGGCCAAGGGACCACGGTCACCGTCTCCTCA |
| | | | SEQ ID NO: 28083 | SEQ ID NO: 32089 |
| | | AA | QSVLTQPPSASGTPGQRVTISCSGSCSNIGSNIVT WYQQLPGTAPKLLIYSNDQRPSGVPDRFSGSKS GTSASLAISGLQSEDEADYYCTAWDDSLNDWVF GGGTTLTVL | QVQLVQSGAEVKKPGASVKVSCKASGYTFNSYGIS WVRQAPGQGLEWMGWISAYNGNTKFAQKLQGRV TMTTDTSTSTAYMELRSLRSDDTAVYYCARHDFW SGYYKGMDVWGQGTTVTVSS |
| | | | SEQ ID NO: 28084 | SEQ ID NO: 32090 |

FIGURE 50

| iPS:451110 | 21-225_74C9 | NA | TCTTATGAGCTGACTCAGCCACCCTCAGAGTC TGTGTCCCCAGGACAGACAGCCAGCATCACCT GCTCAGGAGATAAATCGGGGAATAAATATGTT TCCTGGTATCAGCAGAAGCCAGGCCAGTCCCC TGTGCTGGTCATCTATCAAGATAACAGGCGGC CGTCAGGGATCCCTGAGCGATTTTCTGGCTCC AACTCTGGGAGCACAGCCACTTTGACCATCAG CGGGACCCAGGCTATGGATGAGGCTGACTATT ACTGTCAGGCGTGGGACAGCACCCCTGTGATA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAAT AATAAATCCTATGCAGACTCCGTGAAGGGCCGAT TCACCATCTCCAGAGACATTTCCAAAAACACGCT GTATCTGCAAATGAACAGCCTGAGAGCCGAGGA CACGGCTGTGTATTACTGTGCGAGGGATCGAGAT TATTGTAGTAGTACCAGCTGCCCTTATTATTACTA CTACGGTATGGACGTCTGGGGCCAAGGGACCAC GGTCACCGTCTCCTCA |
|---|---|---|---|---|
| | | | SEQ ID NO: 28085 | SEQ ID NO: 32091 |
| | | AA | SYELTQPPSESVSPGQTASITCSGDKSGNKYVSW YQQKPGQSPVLVIYQDNRRPSGIPERFSGSNSGS TATLTISGTQAMDEADYYCQAWDSTPVIFGGGT KLTVL | QVQLVESGGGVVQPGRSLRLSCAASGFTFSSYGMH WVRQAPGKGLEWVAVIWYDGNNKSYADSVKGRF TISRDISKNTLYLQMNSLRAEDTAVYYCARDRDYC SSTSCPYYYYYGMDVWGQGTTVTVSS |
| | | | SEQ ID NO: 28086 | SEQ ID NO: 32092 |
| iPS:451112 | 21-225_53D10 | NA | TCCTATGAGCTGACTCAGCCACCCTCAGTGTC CGTGTCCCCAGGACAGACAGCCAGCATCACCT GCTCTGGAGATAAATTGGGGAATAAATATGCT TGCTGGTATCAGCAGAGGCCAGGCCAGTCCCC TGTGCTGGTCATCTATCAAGATCGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAGCTGACCGTCCTA | CAGGTGCAGTTGGTGCAGTCTGGGGGCTGAGGTGA AGAAGCCTGGGGCCTCAGTGAAGGTCTCCTGCA AGGCTTCTGGATACATTTTCACCGGCTACTATAT ACACTGGGTGCGACAGGCCCCTGGACAAGGGCT TGAGTGGATGGGATGGATCAACCCTAACAGTGGT GGCACAAACTATGCACAGAAGTTTCAGGGCAGG GTCACCATGACCAGGGACACGTCCATCAGCACA GCCTACATGGAGCTGATCAGGCTGAGATCTGACG ACACGGCCGTGTATTATTGTGCGAGAGAAAACG AAAGTCTAGCAACTCGTCCTTTCTACGACTACTA CGGTATGGACGTCTGGGGCCAAGGGACCACGGT CACCGTCTCCTCA |

FIGURE 50

| | | | SEQ ID NO: 28087 | SEQ ID NO: 32093 |
|---|---|---|---|---|
| | | AA | SYELTQPPSVSVSPGQTASITCSGDKLGNKYACW YQQRPGQSPVLVIYQDRKRPSGIPERFSGSNSGN TATLTISGTQAMDEADYYCQAWDSSTVVFGGG TKLTVL | QVQLVQSGAEVKKPGASVKVSCKASGYIFTGYYIH WVRQAPGQGLEWMGWINPNSGGTNYAQKFQGRV TMTRDTSISTAYMELIRLRSDDTAVYYCARENESLA TRPFYDYYGMDVWGQGTTVTVSS |
| | | | SEQ ID NO: 28088 | SEQ ID NO: 32094 |
| iPS:451114 | 21-225_159A3 | NA | GACATCCAGATGACCCAGTCTCCATCCTCCCT GTCTGCATCTGTAGGAGACAGAGTCACCATCA CTTGCCGGGCAAGTCAGGACATTAGAAAGGAT TTAGGCTGGTATCAGCAGAAACCAGGGAAAG CCCCTAACCGCCTGATCTATGCTGCATCCAGTT TGCAAAGTGGGGTCCCATCAAGGTTCAGCGGC AGTGGATCTGGGACAGAATTCACTCTCACATT CAGCAGCCTGCAGCCTGAAGATTTTGCAACTT ATTACTGTCTACAGCATCATAGTTATCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTTAGTGACTATGTCAT GCAGTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACATTTCCAAGAACACGC TGTATCTGCAAATGAACAGCCTGAGAGCCGAGG ACACGGCTGTGTATTACTGTGCGAGAGAACCGTA TAATAGTGGCTGGTACGACTACGGTATGGACGTC TGGGGCCAAGGGACCACGGTCACCGTCTCCTCA |
| | | | SEQ ID NO: 28089 | SEQ ID NO: 32095 |
| | | AA | DIQMTQSPSSLSASVGDRVTITCRASQDIRKDLG WYQQKPGKAPNRLIYAASSLQSGVPSRFSGSGS GTEFTLTFSSLQPEDFATYYCLQHHSYPRTFGQG TKVEIK | QVQLVESGGGVVQPGRSLRLSCAASGFTFSDYVM QWVRQAPGKGLEWVAVIWYDGSNKYYADSVKGR FTISRDISKNTLYLQMNSLRAEDTAVYYCAREPYNS GWYDYGMDVWGQGTTVTVSS |
| | | | SEQ ID NO: 28090 | SEQ ID NO: 32096 |

FIGURE 50

| | | | | |
|---|---|---|---|---|
| iPS:451116 | 21-225_164A4 | NA | GACATCGTGATGACCCAGTATCCAGACTCCCGGGCTGTGTCTCTGGGCGAGAGGGCCACCATCAAGTGCAAGTCCAGCCAGAGTGTTTTATACAGCTCCAACAATAAGAACTACTTAACTTGGTACCAGCAGAAACCAGGACAGCCTCCCAAACTGTTCATTTACTGGGCATCTACCCGGGAATCCGGGGTTCCTGACCGATTCAGTGGCAGCGGGTGTGGGACAGATTTCACTCTCACCATCAGCAGCGTGCAGGCCGAAGATGTGGCAGTTTATTACTGTCAGCAATATTTTAGTACTCCGTGGACGTTCGGCCAAGGGACCAAGGTGGAAATCAAA<br><br>SEQ ID NO: 28091 | CAGGTGCAGCTGGTGCAGTCTGGGGGCTGAGGTGAAGAAGCCTGGGGCCTCAGTGAAGGTCTCCTGCAAGGCTTCTGGATTCACCTTCCCCAATTATGATATCAACTGGGTGCGACAGGCCACTGGACAAGGCCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTAGCAGTGGCTGGTACTTCTTTGACTACTGGGGCCAGGGAACCCTGGTCACCGTCTCCTCA<br><br>SEQ ID NO: 32097 |
| | | AA | DIVMTQYPDSRAVSLGERATIKCKSSQSVLYSSNNKNYLTWYQQKPGQPPKLFIYWASTRESGVPDRFSGSGCGTDFTLTISSVQAEDVAVYYCQQYFSTPWTFGQGTKVEIK<br><br>SEQ ID NO: 28092 | QVQLVQSGAEVKKPGASVKVSCKASGFTFPNYDINWVRQATGQGLEWMGWMHPNSGNTGYAQKFQGRVTMTRNTSISTAYMELSSLRSEDTAVYYCASSSGWYFFDYWGQGTLVTVSS<br><br>SEQ ID NO: 32098 |
| iPS:451118 | 21-225_191C8 | NA | GAAATAGTGATGACGCAGTCTCCAGCCACCCTGTCTGTGTCTCCAGGGGAAAGAGCCACCCTCTCCTGCAGGGCCAGTCAGAGTGTTCGCAGTAACTTAGCCTGGTACCAGCAGGAACCTGGCCAGGCTCCCAGGCTCCTCATCTATGGTGCATCCACCAGGGCCACTGGTATCCCAGCCAGGTTCAGTGGCAGTGGGTCTGGGACAGAATTCACTCTCACCATCAGCAGCTTGCAGTCTGAAGATTTTGCAGTTTATTACTGTCAGCAGTCTTTTACCTGGCTCCGGACGTTCGGCCAAGGGACCAAGGTGGAAATCAAA<br><br>SEQ ID NO: 28093 | CAGGTGCAGCTGCAGGAGTCGGGCCCAGGACTGGTGAAGCCTTCGGAGACCCTGTCCCTCACCTGCACTGTCTCTAGTGGCTCCGTCAGCAGTGGTGGTTACTACTGGAGCTGGATCCGGCAGCCCCCAGGGAAGGGACTGGAGTGGATTGGGTATATCTATTACAGTGGGACCACCATTTACAACCCCTCCCTCAAGAGTCGAGTCACCATATCAGTAGACACGTCCAAGAACCAGTTCTCCCTGAAGCTGACCTCTGTGACCGTTGCGGACACGGCCGTGTATTACTGTGCGCGAGACACGTTTTGCTTTGATGGTTGTGGTTATTTCTTTGACTCCTGGGGCCAGGGAACCCTGGTCACCGTCTCCTCA<br><br>SEQ ID NO: 32099 |

FIGURE 50

| | | | | |
|---|---|---|---|---|
| | | AA | EIVMTQSPATLSVSPGERATLSCRASQSVRSNLA WYQQEPGQAPRLLIYGASTRATGIPARFSGSGSG TEFTLTISSLQSEDFAVYYCQQSFTWLRTFGQGT KVEIK | QVQLQESGPGLVKPSETLSLTCTVSSGSVSSGGYY WSWIRQPPGKGLEWIGYIYYSGTTIYNPSLKSRVTIS VDTSKNQFSLKLTSVTVADTAVYYCARDTFCFDGC GYFFDSWGQGTLVTVSS |
| | | | SEQ ID NO: 28094 | SEQ ID NO: 32100 |
| iPS:451120 | 21-225_197D3 | NA | GACATCCAGATGACCCAGTCTCCATCCTCACT GTCTGCATCTGTAGGAGACAGAGTCACCATCA CTTGTCGGGCGAGTCAGGGCATTAGAAATTAT TTAGCCTGGTTTCAGCAGAAACCAGGGAAAGC CCCTAAGTCCCTGATCTATGCTGCATCCAGTTT GCAAAGTGGGGTCCCCTCAAAGTTCAGCGGCA GTGGATCTGGGACAGATTTCACTCTCACCATC AGCAGCCTGCAGCCTGAAGATTTTGCAACTTA TTACTGCCAACATTATCTTACTTACCCGCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCATCTTCAGTAGCCATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTTTATTACTGTGCGAGAGATCAAGG TGTGGGGTACTACGGTATGGACGTCTGGGGCCAA GGGACCACGGTCACCGTCTCCTCA |
| | | | SEQ ID NO: 28095 | SEQ ID NO: 32101 |
| | | AA | DIQMTQSPSSLSASVGDRVTITCRASQGIRNYLA WFQQKPGKAPKSLIYAASSLQSGVPSKFSGSGSG TDFTLTISSLQPEDFATYYCQHYLTYPLTFGGGT KVEIK | QVQLVESGGGVVQPGRSLRLSCAASGFIFSSHGMH WVRQAPGKGLEWVAVIWYDGSNEHYADSVKGRF TISRDNSKNTLYLQMNSLRAEDTAVYYCARDQGV GYYGMDVWGQGTTVTVSS |
| | | | SEQ ID NO: 28096 | SEQ ID NO: 32102 |

FIGURE 50

| iPS:451122 | 21-225_200A1 | NA | GAAATTGTGTTGACGCAGTCTCCAGGCATCCT GTCTTTGTATCCAGGGGAAAGAGCCACCCTCT CCTGTAGGGCCAGTCAGAGTGTTAACAGCAAC TATTTAGCCTGGTACCAGCAGAGACCTGGCCA GGCTCCCAGTCTCCTCATTTATGGGGCATCCA GCAGGGCCACTTGCATCCTGGACAGGTTCAGT GGCAGTGGGTGTGGGACAGACTTCACTCTCAC GATCAGCAGACTGGAGCCTGAAGATTTTGCAG TGTATTGCTGTCAGCAGTATGAGATCTCACCG TGGACGTTCGGCCAAGGGACCAAGGTGGAAA GCAAA | CAGGTGCAGCTACAGCAGTGGGGCGCAGGACTG TTGAAGCCTTCGGAGACCCTGTCCCTCACCTGCG CTGTCTATGGTGGGTCCTTCAGTGTTACTATTGG AGCTGGATCCGCCAGCCCCCAGGGAAGGGGCTG GAGTGGATTGGGGAAATCAATCATAGTGGAAGC ACCAACTACAACCCGTCCCTCAAGAGTCGAGTCA CCATTTCACTAGACACGTCCAAGAACCAGTTCTC CCTGAAGCTGAGCTCTGTGACCGCCGCGGACACG GCTGTGTATTACTGTGCGAGAGACTACGGTGTCT TTGACTACTGGGGCCAGGGAACCCTGGTCACCGT CTCCTCA |
| | | | SEQ ID NO: 28097 | SEQ ID NO: 32103 |
| | | AA | EIVLTQSPGILSLYPGERATLSCRASQSVNSNYLA WYQQRPGQAPSLLIYGASSRATCILDRFSGSGCG TDFTLTISRLEPEDFAVYCCQQYEISPWTFGQGT KVESK | QVQLQQWGAGLLKPSETLSLTCAVYGGSFSVYYW SWIRQPPGKGLEWIGEINHSGSTNYNPSLKSRVTISL DTSKNQFSLKLSSVTAADTAVYYCARDYGVFDYW GQGTLVTVSS |
| | | | SEQ ID NO: 28098 | SEQ ID NO: 32104 |
| iPS:451124 | 21-225_74F6 | NA | GACATCGTGATGACCCAGTCTCCAGACTCCCT GGCTGTGTCTCTGGGCGAGAGGGCCACCATCA ATTGCAAGTCCAGTCAGAATATTTTTATCCAGC TCCAACAATAAGAACTACTTAACTTGGTACCA GCAGAAACCAGGACAGCCTCCTAAAATACTCA TTTACTGGACATCTACCCGGGAATCCGGGGTC CCTGACCGATTCAGTGGCAGCGGGTTTGGGAC AGATTTCACTCTCACCATCAGCAGCCTGCAGG CTGAAGATGTGGCTGTTTATTACTGTCAGCAA TATTTTAGTGTTCCTCTGACGTTCGGCCAAGGG ACCAAGGTGGAAATCAAA | CAGGTGCAGCTGGTGCAGTCTGGGGCTGAGGTG AAGAAACCTGGGGCCTCAGTGAGGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAAACACAGGCTATGCACAGAAGTTCCAGGGCAG AGTCACCATGACCAGGAACACCTCCATGAGCAC AGCCTACATGGAGCTGAGCAGCCTGAGATCTGA GGACACGGCCGTGTATTACTGTGCCCATAGCAGT GGCTGGTACTTTTTTGACTACTGGGGCCAGGGAA CCCTGGTCACCGTCTCCTCA |
| | | | SEQ ID NO: 28099 | SEQ ID NO: 32105 |

FIGURE 50

| | | | | |
|---|---|---|---|---|
| | | AA | DIVMTQSPDSLAVSLGERATINCKSSQNILSSSNN KNYLTWYQQKPGQPPKILIYWTSTRESGVPDRFS GSGFGTDFTLTISSLQAEDVAVYYCQQYFSVPLT FGQGTKVEIK | QVQLVQSGAEVKKPGASVRVSCKASGYTFTNYDIN WVRQATGQGLEWMGWMHPNSGNTGYAQKFQGR VTMTRNTSMSTAYMELSSLRSEDTAVYYCAHSSG WYFFDYWGQGTLVTVSS |
| | | | SEQ ID NO: 28100 | SEQ ID NO: 32106 |
| iPS:451127 | 21-225_164A7 | NA | GACATCGTGATGACCCAGTGTCCAGACTCCCT GGCTGTGTCTCTGGGCGAGAGGGCCACCATCA ATTGCAAGTCCAGCCAGAGTGTTTTACACAGC TCCAACAATAACAACTACTTAGCTTGGTACCA GCAGAAACCAGGACAGCCTCATAAGTTGCTCA TTTACTGGACATCTACCCGGGAATCCGGGGTC CCTGACCGATTCAGTGGCAGCGGGTATGGGAC AGATTTCTCTCTCACCATCGCCAGCCTGCAGG CTGAAGATGTGGCAGTTTATTACTGTCAGCAA TATTATAGTATTCCTCTGACGTTCGGCCAAGG GACCAAGGTGGAAATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAATTATGATG TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCACAAGCAC AGCCTACATGGAGCTGAGCAGCCTGAGATCTGA GGACTCGGCCGTGTATTACTGTGCGAGTAGCAGT GGCTGGTACCTCTTTGACTACTGGGGCCAGGGAA CCCTGGTCACCGTCTCCTCA |
| | | | SEQ ID NO: 28101 | SEQ ID NO: 32107 |
| | | AA | DIVMTQCPDSLAVSLGERATINCKSSQSVLHSSN NNNYLAWYQQKPGQPHKLLIYWTSTRESGVPD RFSGSGYGTDFSLTIASLQAEDVAVYYCQQYYSI PLTFGQGTKVEIK | QVQLVQSGAEVKKPGASVKVSCKASGYTFTNYDV NWVRQATGQGLEWMGWMHPNSGNTGYAQKFQG RVTMTRNTSTSTAYMELSSLRSEDSAVYYCASSSG WYLFDYWGQGTLVTVSS |
| | | | SEQ ID NO: 28102 | SEQ ID NO: 32108 |

FIGURE 50

| iPS:451129 | 21-225_94D2 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATAAGAACTACTTAACTTGGTACCA GCAGAAACCAGGACAGCCTCATAAGCTGCTCA TTTACTGGGCATCTACTCGGGAATCCGGGGTC CCTGACCGATTCAGCGGCAGCGGGTCTGGGAC AGATTTCAGTCTCACGATCGGCAGCCTGCAGC ATGAAGATGTGGCAGTTTATTACTGTCAGCAA TATCATAGTATTCCTCCGACGTTCGGCCACGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATTCACCTTCACCAATTATGATA TCAACTGGGTGCGACAGGCCACTGGACAAGGGC TTGAGTGGATGGGATGGATGCACCCTAACAGTGG TAACACAGGCTTTGCACAGAAGTTCCAGGGCAG AGTCACAATGACCAGGAACACCTCCATAAGCAC AGCCTACATGGAACTGAGCAGCCTGAGATCTGA GGACACGGCCGTGTATTACTGTGCGGTTTCCAGT GGCTGGTACTGGTTCGACCCCTGGGGCCAGGGA ACCCTGGTCACCGTCTCCTCA |
| | | | SEQ ID NO: 28103 | SEQ ID NO: 32109 |
| | | AA | DIVMTQSPDSLAVSLGERATINCKSSQSVLHSSN NKNYLTWYQQKPGQPHKLLIYWASTRESGVPD RFSGSGSGTDFSLTIGSLQHEDVAVYYCQQYHSI PPTFGHGTKVEIK | QVQLVQSGAEVKKPGASVKVSCKASGFTFTNYDIN WVRQATGQGLEWMGWMHPNSGNTGFAQKFQGR VTMTRNTSISTAYMELSSLRSEDTAVYYCAVSSGW YWFDPWGQGTLVTVSS |
| | | | SEQ ID NO: 28104 | SEQ ID NO: 32110 |
| iPS:451131 | 21-225_160A7 | NA | GACATCGTGCTGACCCAGTCTCCAGACTCCCC GGCTGTGTCTCTGGGCGAGAGGGCCACCATCA ATTGCAAGTCCAGCCAGAGTGTTTTATCCAAC TCCCACAATAACAACTACTTAGCTTGGTACCA GCAGAGACCAGGACATCCTCATAAACTCCTCA TTTTCTGGGCATCTACCCGGGAATCCGGGGTC CCTGACCGATTCAGTGGCAGCGGGTATGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGCTGAAGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTCACAGTGG TAACACAGGCTATGCACAGAAGTTCCAGGGCAG AGTCACCATGACCAGGAACACCTCCATAAACAC AGCCTACATGGAGCTGAGCAGCCTGAGATCTGA GGACACGGCCGTGTATTACTGTGCCCATAGCAGT GGCTGGTACTACTTTGACTACTGGGGCCAGGGAA CCCTGGTCACCGTCTCCTCA |
| | | | SEQ ID NO: 28105 | SEQ ID NO: 32111 |

FIGURE 50

| | | | | |
|---|---|---|---|---|
| | | AA | DIVLTQSPDSPAVSLGERATINCKSSQSVLSNSHN NNYLAWYQQRPGHPHKLLIFWASTRESGVPDRF SGSGYGTDFTLTISSLQAEDVAVYYCQQYYSTPC SFGQGTKLEIK | QVQLVQSGAEVKKPGASVKVSCKASGYTFTNYDIN WVRQATGQGLEWMGWMHPHSGNTGYAQKFQGR VTMTRNTSINTAYMELSSLRSEDTAVYYCAHSSGW YYFDYWGQGTLVTVSS |
| | | | SEQ ID NO: 28106 | SEQ ID NO: 32112 |
| iPS:451133 | 21-225_95H4 | NA | GACATCGTTATGACCCAGTGTCCAGACTCCCT GGCTGTGTCTCTGGGCGAGAGGGCCACCATCA ATTGCAAGTCCAGCCAGAGTGTTTTATTCAGC TCCAACAATTATAATTACTTAGCTTGGTACCA GCAGAGACCAGGACAGCCTCATAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGCTGATGTGGCAGTTTATTACTGTCAGCAA TATCATAGTTCTCCTCTGACGTTCGGCCAAGG GACCACGGTGCAAATCAAA | CAGGTGCAGTTGGTGCAGTCTGGGGGCTGAGGTGA AGAAGCCTGGGGCCTCAGTGAAGGTCTCCTGCA AGGCTTCTGGATACACCTTCACCAATTATGATAT CAACTGGGTGCGACAGGCCACTGGACAAGGGCT TGAGTGGATGGGATGGATGAACCCTAACAGTGG TAACACAGGCTATGCACAGAAGTTCCAGGGCAG AGTCACCATGACCAGGAACACCTCCACAAGCAC AGCCCACATGGAGCTGAGCAGCCTGAGATCTGA GGACACGGCCGTGTATTACTGTGCGGTCTCCAGT GGCTGGAACTGGTTCGACCCCTGGGGGCCAGGGA ACCCTGGTCACCGTCTCCTCA |
| | | | SEQ ID NO: 28107 | SEQ ID NO: 32113 |
| | | AA | DIVMTQCPDSLAVSLGERATINCKSSQSVLFSSN NYNYLAWYQQRPGQPHNLLIYWASTRESGVPD RFSGSGSGTDFTLTISSLQAADVAVYYCQQYHSS PLTFGQGTTVQIK | QVQLVQSGAEVKKPGASVKVSCKASGYTFTNYDIN WVRQATGQGLEWMGWMNPNSGNTGYAQKFQGR VTMTRNTSTSTAHMELSSLRSEDTAVYYCAVSSGW NWFDPWGQGTLVTVSS |
| | | | SEQ ID NO: 28108 | SEQ ID NO: 32114 |

FIGURE 50

| iPS:437240 | 21-225_84H12 | NA | GACATCCAGATGACCCAGTCTCCATCCTACCA GTCTGCATCTGTGGGAGACAGAGTCACCATCA CTTGCCGGGCAAGTCAGGGCATTAGAAATGAT TTAGGCTGGTTTCAGCAGAAACCAGGGAAAGC CCCTAAGCGCCTGATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTTTACAGCATAATGATTACCCATTCAC TTTCGGCCCTGGGACCAAAGTGGATATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGCTGAACCCTCACAGTGG TAACACAGGCTATGCACAGAAGTTCCAGGGCAG AATCACCATGACCTGGAACACCTCCATACGCACT GCCTACATGGAGCTGAGCAGCCTGAGATCTGAG GACACGGCCGTGTATTACTGTGCGAGAGGGTTTT ACGATATTTTGACTGGTTATTCCCCCACCTACTAC TACTACGATATGGACGTCTGGGGCCAAGGGACC ACGGTCACCGTCTCCTCA |
|---|---|---|---|---|
| | | | SEQ ID NO: 28109 | SEQ ID NO: 32115 |
| | | AA | DIQMTQSPSYQSASVGDRVTITCRASQGIRNDLG WFQQKPGKAPKRLIYAASSLQSGVPSRFSGSGSG TEFTLTISSLQPEDFATYYCLQHNDYPFTFGPGTK VDIK | QVQLVQSGAEVKKPGASVKVSCKASGYTFTSYDIN WVRQATGQGLEWMGWLNPHSGNTGYAQKFQGRI TMTWNTSIRTAYMELSSLRSEDTAVYYCARGFYDI LTGYSPTYYYYDMDVWGQGTTVTVSS |
| | | | SEQ ID NO: 28110 | SEQ ID NO: 32116 |

FIGURE 50

| iPS:434577 | 21-225_75C11 | NA | GACATCCAGATGACCCAGTCTCCATCCTCCCT GTCTGCATCTGTGGGAGACAGAGTCACCATCA CTTGCCGGGCAAGTCAGGGCATTAGAAATGAT TTAGGCTGGTTTCAGCAGAAACCAGGGAAAGC CCCTAAGCGCCTGATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATAATGATTACCCATTCAC TTTCGGCCCTGGGACCAAAGTGGATATCAAA | CAGGTGCAGCTGGTGCAGTCTGGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGCTGAACCCTCACAGTGG TAACACAGGCTATGCACAGAAGTTCCAGGGCAG AATCACCATGACCTGGAACACCTCCATACGCACT GCCTACATGGAGCTGAGCAGCCTGAGATCTGAG GACACGGCCGTGTATTACTGTGCGAGAGGGTTTT ACGATATTTTGACTGGTTATTCCCCCACCTACTAC TACTACGATATGGACGTCTGGGGCCAAGGGACC ACGGTCACCGTCTCCTCA |
| | | | SEQ ID NO: 28111 | SEQ ID NO: 32117 |
| | | AA | DIQMTQSPSSLSASVGDRVTITCRASQGIRNDLG WFQQKPGKAPKRLIYAASSLQSGVPSRFSGSGSG TEFTLTISSLQPEDFATYYCLQHNDYPFTFGPGTK VDIK | QVQLVQSGAEVKKPGASVKVSCKASGYTFTSYDIN WVRQATGQGLEWMGWLNPHSGNTGYAQKFQGRI TMTWNTSIRTAYMELSSLRSEDTAVYYCARGFYDI LTGYSPTYYYYDMDVWGQGTTVTVSS |
| | | | SEQ ID NO: 28112 | SEQ ID NO: 32118 |

FIGURE 50

| iPS:435477 | 21-225_154E8 | NA | GACATCCAGATGACCCAGTCTCCATCTTCCGT GTCTGCATCTATAGGAGACAGAGTCACCATCA CTTGTCGGGCGAGTCAGTTTATTAGCAGCTGG TTAGCCTGGTATCAGCAGAAACCAGGGAAAG CCCCTAAGCTCCTGATCTATACTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTGTTGTCAACAGGCTAACAGTTTCCCGTGG ACGTTCGGCCAAGGGACCAAGGTGGAATTCA AC | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAGGGGGCT GGAGTGGGTCTCAGCTATTAGTGGTAGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGATCCACGGTATA GCAGTGGCTGGTACTGGGGCTCACTACTTTGACT ACTGGGGCCAGGGAACCCTGGCCACCGTCTCCTC A |
| | | | SEQ ID NO: 28113 | SEQ ID NO: 32119 |
| | | AA | DIQMTQSPSSVSASIGDRVTITCRASQFISSWLAW YQQKPGKAPKLLIYTASSLQSGVPSRFSGSGSGT DFTLTISSLQPEDFATYCCQQANSFPWTFGQGTK VEFN | EVQLLESGGGLVQPGGSLRLSCAASGFTFSSYAMS WVRQAPGRGLEWVSAISGSGGNTFYADSVKGRFTI SRDNSKNTLYLQMNSLRAEDTAVYYCAIHGIAVAG TGAHYFDYWGQGTLATVSS |
| | | | SEQ ID NO: 28114 | SEQ ID NO: 32120 |
| iPS:434553 | 21-225_76H12 | NA | GACATCCAGATGACCCAGTCTCCATCCTCCCT GTCTGCATCTGTGGGAGACAGAGTCACCATCA CTTGCCGGGCAAGTCAGGGCATTAGAAATGAT TTAGGCTGGTTTCAGCAGAAACCAGGGAAAGC CCCCAAGCGCCTGATCTATGCTGCATCCAGAT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATGATTACCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGCTGAACCCTCACAGTGG TAACACAGGCTATGCACAGAAGTTCCAGGGCAG AATCACCATGACCTGGAACACCTCCATACGCACT GCCTACATGGAGCTGAGCAGCCTGAGATCTGAG GACACGGCCGTGTATTACTGTGCGAGAGGGTTTT ACGATATTTTGACTGGTTATTCCCCCACCTACTAC TACTACGATATGGACGTCTGGGGCCAAGGGACC ACGGTCACCGTCTCCTCA |

FIGURE 50

| | | | SEQ ID NO: 28115 | SEQ ID NO: 32121 |
|---|---|---|---|---|
| | | AA | DIQMTQSPSSLSASVGDRVTITCRASQGIRNDLG WFQQKPGKAPKRLIYAASRLQSGVPSRFSGSGS GTEFTLTISSLQPEDFATYYCLQHNDYPFTFGPGT KVDIK | QVQLVQSGAEVKKPGASVKVSCKASGYTFTSYDIN WVRQATGQGLEWMGWLNPHSGNTGYAQKFQGRI TMTWNTSIRTAYMELSSLRSEDTAVYYCARGFYDI LTGYSPTYYYYDMDVWGQGTTVTVSS |
| | | | SEQ ID NO: 28116 | SEQ ID NO: 32122 |
| iPS:434927 | 21-225_86E5 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATCACTGTCTACAGCATAATGATTACCCATTC ACTTTCGGCCCTGGGACCAAAGTGGAAATCAA A | CAGGTGCAGCTGGTGCAGTCTGGGGGCTGAGGTG AAGAAGCCTGGGGCCTCAGTGAAGGTCTCCTGC AAGGCTTCTGGATACACCTTCACCAGTTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCTGGAACACCTCCATACGCAC TGCCTACATGGAGCTGAGCAGCCTGAGATCTGAG GACACGGCCGTGTATTACTGTGCGAGAGGGTTTT ACGATATTTTGACTGGTTATTCCCCCACCTACTAC TACTACGATATGGACGTCTGGGGCCAAGGGACC ACGGTCACCGTCTCCTCA |
| | | | SEQ ID NO: 28117 | SEQ ID NO: 32123 |
| | | AA | DIQMTQSPSSLSASVGDRVTITCRASQGIRNDLG WYQQKPGKAPKRLIYAASSLQSGVPSRFSGSGS GTEFTLTISSLQPEDFATYHCLQHNDYPFTFGPGT KVEIK | QVQLVQSGAEVKKPGASVKVSCKASGYTFTSYDIN WVRQATGQGLEWMGWMNPNSGNTGYAQKFQGR VTMTWNTSIRTAYMELSSLRSEDTAVYYCARGFYD ILTGYSPTYYYYDMDVWGQGTTVTVSS |
| | | | SEQ ID NO: 28118 | SEQ ID NO: 32124 |

FIGURE 50

| iPS:435385 | 21-225_149G7 | NA | GACATCCAGATGACCCAGTCTCCATCTTCCGT GTCTGCATCTGTAGGAGACAGAGTCACCATCA CTTGTCGGGCGAGTCAGTTTATTAGCAGCTGG TTAGCCTGGTATCAGCAGAAACCAGGGAAAG CCCCTAAGTTCCTGATCTATGCTGCATCCAGTT TGCAAAGTGGGGTCCCATCAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCGTGG ACGTTCGGCCAAGGGACCAAGGTGGAAATCA AC | GAGGTGCAGCTGTTGGAGTCTGGGGGAGGCTTG GTACAGCCTGGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AACACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGATCCACGGTATA GCAGTGGCTGGTACTGGGGCTCACTACTTTGACT ACTGGGGCCAGGGAACCCTGGCCACCGTCTCCTC A |
|---|---|---|---|---|
| | | | SEQ ID NO: 28119 | SEQ ID NO: 32125 |
| | | AA | DIQMTQSPSSVSASVGDRVTITCRASQFISSWLA WYQQKPGKAPKFLIYAASSLQSGVPSRFSGSGSG TDFTLTISSLQPEDFATYYCQQANSFPWTFGQGT KVEIN | EVQLLESGGGLVQPGGSLRLSCAASGFTFSSYAMS WVRQAPGKGLEWVSAISGSGGNTFYADSVKGRFTI SRDNSKNTLYLQMNSLRAEDTAVYYCAIHGIAVAG TGAHYFDYWGQGTLATVSS |
| | | | SEQ ID NO: 28120 | SEQ ID NO: 32126 |

FIGURE 51 (Table 4)
Standard IgG Antibody Variable Region Protein Alignment
CDRs defined by Kabat
Alignment numbering defined by Amgen Reference (AHo)

| KAPPA_VARIABLE | | Germline | SEQ ID NO: | K_FR1 | K_CDR1 | K_FR2 | K_CDR2 | K_FR3 | K_CDR3 | K_FR4 |
|---|---|---|---|---|---|---|---|---|---|---|
| | VK4\|B3/JK3 | | 32127 | DIVMTQSPDSLAV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------TPFT | FGPGT KVDIK |
| IPS:426 126 | 21-225_6G6 | VK4\|B3/J K3 | | DIVMTQSPDSLAV SLGERATINC | KSS-- QSVLHNSNNYN YLA | WYQQKPGQ PPNLLIF | W-------- ASTRES | GVPDRFSGSGFG-- TDFTLNISSLQAEDVAVYYC | QQYYD----------- ------------TPFT | FGHGT KVDIK |
| IPS:412 232 | 21-225_4A2 | VK4\|B3/J K3 | | DIVMTQSPDSLAV SLGERATINC | KSS-- QSILHSSNNNN YLA | WFQQKPGQ PPKLLLY | W-------- ASTRES | GVPDRFSGSGSG-- TDFTLTISSLQPEDVAVYYC | QQYYN----------- ------------TPVT | FGPGT KVGIK |
| IPS:451 141 | 21-225_164B1 1 | VK4\|B3/J K3 | | DIVMTQSPDSLAV SLGERATINC | KSS-- QSLLKSSNNKS YLA | SYQQKPGQ LPKLLIY | W-------- ASSRES | GVPDRFSGSGSG-- TDFTLTISSLQAEDVALYYC | QQYYS----------- ------------IPPT | FGHGT NVDIT |
| IPS:423 314 | 21-225_12F11 | VK4\|B3/J K3 | | DIVMTQSPDSLAV SLGERATINC | KSS-- QSVLHSSNNYN YLA | WYQQKPGQ PPNLLIF | W-------- ASTRES | GVPDRFSGSGFG-- TDFTLNISSLQAEDVAVYYC | QQYYD----------- ------------TPFT | FGPGT KVDIK |
| IPS:435 327 | 21-225_147G6 | VK4\|B3/J K3 | | DIVMTQSPDSLAV SLGERATINC | KSS-- QSVLYSSNSNN YLA | WYQQKPGQ PPKLLIY | W-------- ASARES | GVPDRFSGSGSG-- TDFTLTISSLQAEDVAVYYC | QQYYT----------- ------------TPPT | FGPGT KVDIK |
| IPS:435 345 | 21-225_148G3 | VK4\|B3/J K3 | | DIVMTQSPDSLAV SLGERATIHC | KSS-- QRVLHSSNNYN YLA | WYQQKPGQ PPKLLIY | W-------- ASTRDS | GVPDRFSGSGSG-- ADFTLTISSLQAEDVALYYC | QQYYS----------- ------------TPFT | FGPGT KVDIK |
| IPS:435 405 | 21-225_150B7 | VK4\|B3/J K3 | | DIVMTQSPDSLAV SLGERATINC | KSS-- QNVLYSSHNNN YLA | WYQQKPGQ PPKLLIY | W-------- ASTRKS | GVPDRFSGSGSG-- TDFTLTITSLQAEDVAVYYC | QQYYS----------- ------------TPFT | FGPGT KVDIK |
| IPS:435 433 | 21-225_152E3 | VK4\|B3/J K3 | | DIVMTQSPDSLAV SLGERATINC | KSS-- QSVLHSSNNYN YLV | WYQQKPGQ SPKRLIY | W-------- ASTRES | GVPDRFSGSGSG-- TDFSLTISSLQAEDVAVYYC | QQYYS----------- ------------TPFT | FGPGT KVDIK |
| IPS:435 437 | 21-225_152F4 | VK4\|B3/J K3 | | DIVMTQSPDSLAV SLGERATINC | KSS-- QSVLYSSNNYN YLA | WYQQKPGQ PPKLLIY | W-------- ASTRKS | GVPDRFSGSGSG-- TDFTLTVSSLQAEDVAVYYC | QQYFN----------- ------------TPPT | FGPGT KVDIK |
| IPS:435 649 | 21-225_165H2 | VK4\|B3/J K3 | | DIVMTQSPDSLTV SPGERATINC | KSS-- QSVLHSSNNKN YLT | WYQQKPGQ PPKLLIY | W-------- ASTRES | GVPVRFSGSGSG-- TDFTVPISSMQDDDVAVYYR | QQSYS----------- ------------IPPT | FGPGT NVDIK |
| IPS:435 855 | 21-225_191G3 | VK4\|B3/J K3 | | DIVMTQSPDSLAV SLGERATIDC | KSS-- QSVLHSSNSYN YLA | WYQQKLGQ PPKLLIY | W-------- ASTRKS | GVPDRFSGSGSG-- TDFTLTISSLQAEDVAFYYC | QQYYS----------- ------------SPPT | FGPGT KMDIK |

| ID | Clone | Germline | | K_FR1 | K_CDR1 | K_FR2 | K_CDR2 | K_FR3 | K_CDR3 | K_FR4 |
|---|---|---|---|---|---|---|---|---|---|---|
| iPS:435 903 | 21-225_190E2 | VK4|B3/JK3 | | DIVMTQSPDSLAV SLGERATINC | KSS-- QSVLFNSNNKN YLA | WYQQKPGQ PPNLLIY | W-------- ASTRES | GVPDRFSGSGSG-- TDFTLTISSMQAEDVAVYYC | QQYCS----------- ------------LPFT | FGPGT KVDIR |
| IPS:435 915 | 21-225_190H4 | VK4|B3/JK3 | | ANVMTQSPDSLAV SLGERTTINC | KSS-- QSVLHSSNNYN YLA | WYRQKPGQ PPKLLIY | W-------- ASTRES | GVPDRFSGSGSG-- TDFTLTISSLQAEDVAVYYC | QQYYS----------- ------------IPPT | FGPGT KVDIK |
| IPS:435 923 | 21-225_190H6 | VK4|B3/JK3 | | DIVMTQSPDSLAV SLGERATINC | KSS-- QSVLFNSNNKN YLA | WYQQKPGQ PPNLLIY | W-------- ASTRES | GVPDRFSGSGCG-- TDFTLTISSLQAEDVAVYYC | QQYCS----------- ------------LPFT | FGPGT KVDIR |
| IPS:435 953 | 21-225_191B12 | VK4|B3/JK3 | | DIVMTQSPDSLAV SLGERATINC | KSS-- QSVLFNSNNKN YLA | WYQQKPGQ PPKLLIY | W-------- ASTRES | GVPDRFSGSGSG-- TDFTLTISNLQAEDVAIYYC | QQYSS----------- ------------LPFT | FGPGT KVDIK |
| IPS:436 098 | 21-225_195G11 | VK4|B3/JK3 | | DIVMTQSPDSLAV SLGERATINC | KSS-- QSVLFNSNNKN YLA | WYQQKPGQ PPNLLIY | W-------- ASTLES | GVPDRFSGSGCG-- TDFTLTISSMQAEDVAVYYC | QQYCS----------- ------------FPFT | FGPGT KVDIR |
| IPS:436 102 | 21-225_196B1 | VK4|B3/JK3 | | DIVMTQSPDSLAV FLGERATINC | KSS-- QSILFSSNNKR YLA | WYQQKPGQ PPKLLIY | W-------- ASIRES | GVPDRFSGSGSG-- TDFTLTISSLQAEDVAVYYC | QQYSS----------- ------------LPFT | FGPGT KVDIK |
| IPS:436 104 | 21-225_196C1 | VK4|B3/JK3 | | DIVMTQSPDSLAV SLGERATINC | KSS-- QSVLFNSNNKN YLA | WYQQKPGQ LPNLLIY | W-------- ASTLES | GVPDRFSGSGCG-- TDFTLTISSMQAEDVAVYYC | QQYCS----------- ------------FPFT | FGPGT KVDIR |
| IPS:436 156 | 21-225_197C8 | VK4|B3/JK3 | | DIVMTPSPDSLAV SLGERATINC | KSS-- QSVLHSSNNKN YLA | WYQQKPGQ PPKLLIY | W-------- ASTRES | GVPDRFSGSGSG-- TDFTLSISSLQAEDVAVYYC | QQSYT----------- ------------IPFT | FGPGT KVDNK |
| IPS:436 270 | 21-225_203F10 | VK4|B3/JK3 | | DIVMTQSPDSLAV SLGERATINC | KSS-- QSVFFHSNNKN YLA | WYQQKPGQ PPKLLIY | W-------- ASTRES | GVPDRFSGSGSG-- TDFTLTISSLQAEDVTVYYC | QQYFS----------- ------------LPFT | FGPGT KVDIT |
| IPS:436 570 | 21-225_225F4 | VK4|B3/JK3 | | DIVMTQSPDSLAV SLGERATINC | KSS-- QSVLYSSNNKN YLA | WYQQKPGQ PPKLLIY | W-------- ASTRES | GVPDRFTGSGSG-- TDFTLTISCVQPEDVAVYYC | HQYHN----------- ------------SPPT | FGHGT EVDIK |
| IPS:394 065 | 21-225_11E2 | VK4|B3/JK3 | | DIVMTQSPDSLAV SLGERATINC | KSN-- QRVLSSSNNHN YLA | WYQQRPGQ PPKLLIY | W-------- ASTRES | GVPDRFSGSGSG-- TDFTLTISSLQAEDVAVYYC | QQYFS----------- ------------TPFT | FGPGT KVDIK |
| | **Germline** | | | **K_FR1** | **K_CDR1** | **K_FR2** | **K_CDR2** | **K_FR3** | **K_CDR3** | **K_FR4** |
| | VK1|A30/JK5 | | 32128 | DIQMTQSPSSLSA SVGDRVTITC | RAS--QGIR-- ------NDLG | WYQQKPGK APKRLIY | A-------- ASSLQS | GVPSRFSGSGSG-- TEFTLTISSLQPEDFATYYC | LQHNS----------- ------------YPIT | FGQGT RLEIK |
| IPS:473 253 | 21-225_7C3_LC1 | VK1|A30/JK5 | | DIQMTQSPSSLSA SVGDRVTITC | RAS--QGIR-- ----SDLG | WYQQNPVK APKRLIY | A-------- ASSLQS | GVPSRFSGSGSG-- TEFTLTVSSLQPEDFAFYYC | LQHNS----------- -----------YLPIT | FGQGT RLEIK |
| IPS:473 256 | 21-225_9F12_LC2 | VK1|A30/JK5 | | DIQMTQSPSSLSA SVGDRVTITC | RAS--QGIR-- ----NDLG | WYQQKPVK APKRLIY | A-------- ASSLQS | GVPSRFSGSGSG-- TEFTLTISSLQPEDFATYYC | LQHNS----------- -----------YLPIT | FGQGT RLEIK |
| IPS:453 449 | 21-225_208A2 | VK1|A30/JK5 | | DIQMTQSPSSLSA SVGDRVTITC | RTS--QGIR-- ----NDLG | WYQQQPGK TPKRLIY | A-------- ASSLLS | GVPSRFSGSRSG-- TDFTLTISSLQPEDFATYYC | LQYNS----------- ------------YPPT | FGQGT RLEIK |
| IPS:434 467 | 21-225_73H8 | VK1|A30/JK5 | | DIQMTQSPSSLYA SVGDRVTITR | RAS--QDIR-- ----NDLG | WYQQKPGK ALKRVIY | A-------- ASSLQS | GVPSSFSGSGSG-- TEFTLTISSLQPEDFATYYG | IQHNS----------- -----------YPPIT | VGQGT RLEIK |
| IPS:435 045 | 21-225_90H5 | VK1|A30/JK5 | | DIQMTQSPSSLSA SVGDRVTITC | RAS--QGIR-- ----NDLG | WYQQKPGK APKRLIY | I-------- ASSLQS | GVPSRFSGSGSG-- TEFTLTISSLQPEDFATYYC | LQHNS----------- ------------YPIT | FGQGT RLEIK |

| ID | Clone | V-gene | No. | K_FR1 | K_CDR1 | K_FR2 | K_CDR2 | K_FR3 | K_CDR3 | K_FR4 |
|---|---|---|---|---|---|---|---|---|---|---|
| iPS:435 561 | 21-225_159F1 | VK1\|A30/JK5 | | DIQMTQSPSSLSA SVGDRVTITC | RAS--QRVR-- ----NDLG | WYQQKPAK APKRIIF | D-------- ASNLQS | GVPSRFSGSGSG-- TEFTLTISSLQPEDFATYYC | LQHHS----------- ------------FPIT | FGQGT RLEIK |
| iPS:436 328 | 21-225_207F12 | VK1\|A30/JK5 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPLT | FGQGT RLEIK |
| iPS:436 3540 | 21-225_210G10 | VK1\|A30/JK5 | | DIQMTQSPSSLSA SVGDRVTITF | RTS--QGIR-- ----NDLG | WYQQQPGK TPKRMIY | A-------- ASSLFS | GVPSRFSGSRSG-- TDFTLTISSLQPEDFATYYC | LQYNS----------- ------------YPPT | FGQGT RLEIK |
| iPS:393 094 | 21-225_34C4 | VK1\|A30/JK5 | | DIQMTQSPSSLSA SVGDRVTITC | RAS--QGIR-- ----NDLG | WYQQKPGK APKRLIY | T-------- ASNLQS | GVPSRFSGSGSG-- TEFTLTISSLQPEDFATYYC | LQHSS----------- ------------YPIT | FGQGT RLEIK |
| iPS:398 484 | 21-225_18D4 | VK1\|A30/JK5 | | DIQMTQSPSSLSA SVGDRVTITC | RAS--QGIR-- ----NDLG | WYQQKPGK APKRLIY | A-------- ASSLES | GVPSRFSGSGSG-- TEFTLTVSSLQPEDFATYYC | LHHNN----------- -----------YLPIT | FGQGT RLEIK |
| | Germline | | | **K_FR1** | **K_CDR1** | **K_FR2** | **K_CDR2** | **K_FR3** | **K_CDR3** | **K_FR4** |
| | VK1\|L5/JK3 | | 32129 | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQANS---------- -----------FPFT | FGPGT KVDIK |
| iPS:473 254 | 21-225_7C3_LC2 | VK1\|L5/JK3 | | DIQMTQSPSSVSA SLGDRVTITC | RAS--QGIS-- ----SWLA | WYQQKQGK APKLLIF | A-------- ASRLQS | GAPSRFSGSGSG-- TDFTLTISSLQPEDFATYYC | QQANS----------- ------------FPFT | FGPGT KVDIK |
| iPS:473 255 | 21-225_9F12_LC1 | VK1\|L5/JK3 | | DIQMTQSPSSVSA SVGDRVTITC | RAS--QGIS-- ----RWLA | WYQQKPGK APKLLIY | A-------- ASRLQS | GVPSRFSGSGSG-- TDFTLTISGLLPEDFAIYYC | QQANS----------- ------------FPFT | FGPGT KVDFK |
| iPS:426 108 | 21-225_10G6 | VK1\|L5/JK3 | | DIQMTQSPSSVSA SVGDRVTITC | RAS--QGIS-- ----KWLA | WYQQKPGK APKILIY | A-------- AYSLQS | GVPARFSGSGSG-- TDFTLTIRSLQPEDFATYYC | QQANS----------- ------------FPFT | FGPGT KVDIK |
| iPS:426 110 | 21-225_12E9 | VK1\|L5/JK3 | | DIQMTQSPSSVSA SVRDRVTITC | RAS--QGIS-- ----SWLA | WYQQKPGE APKLLIY | A-------- ASRLQS | GVPSRFSGSGSG-- TDFTLTISSLQPEDFATYYC | QQANS----------- ------------FPFT | FGPGT KVDIK |
| iPS:453 447 | 21-225_65F10 | VK1\|L5/JK3 | | DIQMTQSPSSVSA SVGDRVTITC | RAS--QGIS-- ----TWLA | WYQQKPGK APKLLIY | A-------- ASILQS | GVPSRFRGRGSG-- TDFTLTISSLQPEDFATYYC | QQGNI----------- ------------FPFT | FGRGT KVDIK |
| iPS:453 451 | 21-225_52G11 | VK1\|L5/JK3 | | DIQMTQSPSSVSA SVGDRVTITC | RAS--QGIS-- ----KWLA | WYQQKPGK APKLLLY | A-------- ASSLQS | GVPSRFSASGSG-- TDFTLTISSLQPEDFATYYC | QQANS----------- ------------FPFT | FGPGT KVDVK |
| iPS:453 453 | 21-225_53F2 | VK1\|L5/JK3 | | DIQMTQSPSSVSA SVGDRVTITC | RAS--QGIS-- ----KWLA | WYQQKPGK APNLLLY | A-------- ASSLQS | GVPSKFSGSGSG-- TDFTLTISSLQPEDFATYYC | QQANS----------- ------------FPFT | FGPGT KVDVK |
| iPS:433 915 | 21-225_43H9 | VK1\|L5/JK3 | | DIQMTQSPSSVSA SVGDRVTITC | RAS--QDIS-- ----SWLA | WYQKKPGK APKLLIY | D-------- ASSLQS | GVPSRFSGSGSG-- TDFTLTISSLQPEDFATYFC | QQANS----------- -----------LPFT | FGPGT KVDIK |
| iPS:433 925 | 21-225_44F3 | VK1\|L5/JK3 | | DIQMTQSPSSVSA SVGDRVTITC | RAS--QGIS-- ----DWLA | WYQQKPGK APKLLIY | A-------- ASSLQS | GVPSRFSGSGFG-- TDFTLTISSLQPEDFATYYC | QQANS----------- ------------FPFT | FGPGT KVDIK |
| iPS:433 953 | 21-225_45H4 | VK1\|L5/JK3 | | DIQMTQSPSSVSA SVGDRVTITC | RAS--QDIS-- ----SWLA | WYQKKPGK APKYLIY | D-------- ASSLQS | GVPSRFSGSGSG-- TDFTLTISSLQPEDFAIYFC | QQANS----------- -----------LPFT | FGPGT KVDIK |
| iPS:433 959 | 21-225_45C9 | VK1\|L5/JK3 | | DIQMTQSPSSVSV SVGDRVTITC | RAS--QDIS-- ----DWLA | WYQQRPGK APKLLIY | A-------- ASSLES | GVPSRFSGSGSG-- TDFTLTISSLQPEDFATYYC | QQANS----------- ------------FPFT | FGPGT KVDIK |
| iPS:434 023 | 21-225_49F1 | VK1\|L5/JK3 | | DIQMTQSPSSVSA SVGDRVTITC | RAS--RDIN-- ----GWLA | WYQQKPGK APKLLIY | T-------- VSSLQS | GVPSRFSGSGSG-- TDFTLTISSLQPEDFATYYC | QQSNS----------- ------------FPFT | FGPGT KVDIK |
| iPS:434 027 | 21-225_49H5 | VK1\|L5/JK3 | | DIQMTQSPSSVSA SVGDRVTITC | RAS--QGFS-- ----TWLA | WFQQKPGK APKLLIY | A-------- ASSLQD | GVPSRFSGSGSG-- TDFTLTISSLQPEDFATYYC | QQTNS----------- ------------FPFT | FGPGT KVDIK |
| iPS:434 035 | 21-225_49F10 | VK1\|L5/JK3 | | DIQMTQSPSSVSA SVGDRVTITC | RAS--QGIS-- ----RWLA | WYQQKPGK APKVLIY | A-------- ASTLQS | GVPSRFSGSGSG-- TDFTLTISSLQPEDFATYYC | QQANS----------- ------------FPFT | FGPGT KVDIK |
| iPS:434 061 | 21-225_51C7 | VK1\|L5/JK3 | | DIQMTQSPSSVSA SVGDRVTITC | RAS--QDVN-- ----NYLA | WFQQKPGK APKLLIY | A-------- ASSLQN | GVPSRFSGSGSG-- TDFTLTISSLLPEDFATYYC | QQTNS----------- ------------FPFT | FGPGT KVDIK |

| | | | | | | | | | |
|---|---|---|---|---|---|---|---|---|---|
| iPS:434 065 | 21-225_50D4 | VK1\|L5/JK3 | | DIQMTQSPSSVSA SVGDRLTITC | RAS--QGIS-- ----RWLA | WYQQKPGK APKVLIY | A-------- ASTLQS | GVPSRFSGSGSG-- TDFTLTISSLQPEDFATYYC | QQANS----------- -----------FPFT | FGPGT KVDIR |
| iPS:434 069 | 21-225_51E9 | VK1\|L5/JK3 | | DIQMTQSPSSVSA SVGDRVTITC | RAS--QGIS-- ----SWLA | WYQQKPGK APKLLIY | V-------- ASSLQS | GVPSRFSGSGSG-- TDFTLTIRSLQPEDFATYYC | QQAKS----------- -----------FPFT | FGPGT KVDIK |
| iPS:434 079 | 21-225_52B1 | VK1\|L5/JK3 | | DIQMTQSPSSVST FVGDRITITC | RAS--QDIR-- ----TWLA | WYQQKPGK APKLLIY | A-------- ASSLQN | GVPSRFSGSGSG-- TDFTLTISSLQPEDFATYFC | QQAKS----------- -----------FPFT | FGPGT KVDIK |
| iPS:434 097 | 21-225_52H10 | VK1\|L5/JK3 | | DIQMTQSPSSVSA SVGDRVTITC | RAS--QDIN-- ----SWLA | WYQQKPGK APKLLIY | V-------- ASSLQS | GVPSRFSGSGSG-- TDFTLTIRSLQPEDFATYYC | QQAKS----------- -----------FPFT | FGPGT KVDIK |
| iPS:434 123 | 21-225_53F7 | VK1\|L5/JK3 | | DIQMSQSPSSVSA SVGDRVTITC | RAS--QGIS-- ----RWLA | WYQQKPGK APNLLIY | A-------- ASSLQS | GVPSRFSGSGSG-- TDFTLTISSLQPEDFAIYYC | QQANS----------- -----------FPFT | FGPGT KVDIK |
| iPS:434 145 | 21-225_55B1 | VK1\|L5/JK3 | | DIQMTQSPSSVSA SVGDRVTITC | RAS--QVIS-- ----RWLA | WYQQKPGK APNLLIY | A-------- ASSLQS | GVPSRFSGSGSG-- TDFTLTISSLQPEDFATYYC | QQANS----------- -----------FPFT | FGPGT KVDLK |
| iPS:434 167 | 21-225_50F3 | VK1\|L5/JK3 | | DIQMTQSPSSVSA SVGDRVTITC | RAS--QDIS-- ----SWLA | WFQQKPGK APKLLIY | A-------- ASSLQS | GVPSRFSGSGSG-- TDFTLTISSLQPEDFATYYC | QQINS----------- -----------FPFT | FGPGT KVDIK |
| iPS:434 189 | 21-225_56E5 | VK1\|L5/JK3 | | DIQMTQSPSSVSA SVGDRVTITC | RAS--QGIR-- ----KWLA | WYQQKPGK APKLLIY | A-------- ASSLQS | GVPSRFSGSGSG-- TDFTLTISSLQPEDFATYYC | QQANS----------- -----------FPFT | FGPGT KVDIK |
| iPS:434 193 | 21-225_56C6 | VK1\|L5/JK3 | | DIQMTQSPSSVSA SVGDRVTITC | RAS--QGIS-- ----SWLA | WYQQKSGN APKLLIY | A-------- ASRLQS | GVPSRFSGSGSG-- TYFTLIISSLQSEDFATYYC | QQANS----------- -----------FPFT | FGPGT KVDIK |
| iPS:434 195 | 21-225_56F6 | VK1\|L5/JK3 | | DIQMTQSPSSVCA YVGDRVTITC | RVS--QDIS-- ----KWLA | WFQQKPGK APKFLIY | V-------- ASGLQS | GVPSRFSGSGSG-- TDFTFTISSLQPEDFATYYC | QQANS----------- -----------FPFT | FGPGT KVDIK |
| iPS:434 273 | 21-225_57E4 | VK1\|L5/JK3 | | DIQMTQSPSSVSA SVGDRVSITC | RAS--QDIS-- ----NWLA | WYQQKPGK APKLLIY | A-------- ASSLQS | GVPSRFSGSGSG-- TDFTLTISSLQPEDFATYSC | QQGNS----------- -----------FPFT | FGPGT KVDIK |
| iPS:434 277 | 21-225_57A7 | VK1\|L5/JK3 | | DIQMTQSPSSVSA SVGDRVTITC | RAS--QGIS-- ----RWLA | WYQQKPGK APNLLIY | A-------- ASNLQS | GVPSRFSGSGSG-- TDFTLTISSLQPEDFATYYC | QQANS----------- -----------FPFT | FGPGT KVDIK |
| iPS:434 355 | 21-225_64G12 | VK1\|L5/JK3 | | DIQMTQSPSSVSA SVGDRVTITC | RAS--QNIT-- ----TWLA | WYQQKPGK APKLLIS | A-------- ASSLQS | GVPSRFSGSGSG-- TDFTLTISSLQPEDFATYIC | QQANS----------- -----------FPFT | FGPGT KLDIK |
| iPS:434 389 | 21-225_66F11 | VK1\|L5/JK3 | | DIQMTQSPSSVCA SVGDRVTITC | RES--QGIS-- ----IWLA | WYQQKPGK APKLLIY | A-------- ASSLQS | GVPSRFSGSGYG-- TDFTLTISSVQPEDFATYYC | QQANS----------- -----------FPFT | FGPGT KVDIK |
| iPS:434 423 | 21-225_70D1 | VK1\|L5/JK3 | | DIQMTQSPSSVSA SVGDRVTITC | RAS--QGVS-- ----RWLA | WYQQKPGK APKLLIY | A-------- ASSLQS | GVPSRFSGSGSG-- TDFTVTISSLQPEDFATYYC | QQANS----------- -----------FPFT | FGPGT KVDIK |
| iPS:435 291 | 21-225_146E1 | VK1\|L5/JK3 | | DIKMTQSPSSVSA SVGDRVTITC | RAS--QGIN-- ----NWLV | WYQQKPGK APKLLIY | A-------- ASSLQS | GVPSRFRGSGSG-- TDFTLTISSLQPEDFATYYC | QQANS----------- -----------FPFT | FGPGT KVDIK |
| iPS:435 303 | 21-225_146A6 | VK1\|L5/JK3 | | DIQMTQSPASVSA SVGDRVTITC | RAS--QGIS-- ----NWLA | WYQQKPGK APKLLIY | A-------- ASSLQG | GVPSRFSGSGSG-- TDFTLSISSLQPEDFATYYC | QQTDS----------- -----------FPFT | FGPGT KVDIK |
| iPS:435 335 | 21-225_147D10 | VK1\|L5/JK3 | | DIQMTQSPASVSA SVGDRVTITC | RAS--QNIS-- ----NWLT | WYQQKPGK APKLLIY | A-------- ASSLQS | GVPSRFSGSGSG-- TDFTLSISSLQPEDFATYYC | QQTDS----------- -----------FPFT | FGPGT KVDVK |
| iPS:435 339 | 21-225_147D12 | VK1\|L5/JK3 | | DIQMTQSPASVSA SVGDRVTITC | RAS--QGIS-- ----NWLA | WYQEKPGK APKLLIY | A-------- ASSLQS | GVPSRFSGSGSG-- TDFTLSISSLQPEDFATYYC | QQTDS----------- -----------FPFT | FGPGT KVDVK |
| iPS:435 343 | 21-225_148E2 | VK1\|L5/JK3 | | DIQMTQSPASVSA SVGDRVTITC | RAS--QGIS-- ----NWLA | WYQQKPGK APKLLIY | A-------- ASSLQS | GVPSRFSGNESG-- TDFTLSISSLQPEDFATYYC | QQTDS----------- -----------FPFT | FGPGT KVDVK |
| iPS:435 379 | 21-225_149B6 | VK1\|L5/JK3 | | DIQMTQSPSSVSA SVGDRVTITC | RAS--QGII-- ----SWLA | WYQQKPGK APKLLIY | A-------- ASSLQS | GVPSRFSGSGSG-- TDFTLTISSLQPEDFATYYC | QQGNS----------- -----------FPFT | FGPGT KVDIK |
| iPS:435 381 | 21-225_149C6 | VK1\|L5/JK3 | | DIQMTQSPASVSA SVGDRVTITC | RAS--QGIS-- ----NWLA | WYQQKPGR APKLLIY | A-------- ASSLQG | GVPSRFSGSGSG-- TDYTLSISSLQPEDFATYYC | QQTDS----------- -----------FPFT | FGPGT KVDIK |

| | | | | | | | | | | |
|---|---|---|---|---|---|---|---|---|---|---|
| iPS:435 391 | 21-225_149F8 | VK1\|L5/JK3 | | DIQMTQSPASVSA SVGDRVTITC | RAS--QGIS-- ----NWLA | WYQQKPGK APKLLIY | A-------- ASSLQS | GVPSRFSGNESG-- TDFTLSISSLQPEDFAIYYC | QQTDS----------- ------------FPFT | FGPGT KVDVK |
| IPS:435 395 | 21-225_149D1 1 | VK1\|L5/JK3 | | DIQMTQSPASVSA SVGDRVTITC | RAS--QNIS-- ----NWLI | WYQQKPGK APKLLIY | A-------- ASSLQS | GVPSRFSGSGSG-- TDFTLSISSLQPEDFATYYC | QQTDS----------- ------------FPFT | FGPGT KVDVK |
| iPS:435 403 | 21-225_150C5 | VK1\|L5/JK3 | | DIQMTQSPGSVSA SVGDRVTITC | RAS--QGIN-- ----NWLA | WYQQKPGK APKLLIY | A-------- ASSLQG | GVPSRFSGSGSG-- TDFTLSISSLQPEDFATYYC | QQTDS----------- ------------FPFT | FGPGT KVDIK |
| iPS:435 447 | 21-225_152H7 | VK1\|L5/JK3 | | DIQMTQSPASVSA SVGDRVTITC | RAS--QDIS-- ----NWLA | WYQQKPGK APKLLIY | A-------- ASSLQG | GVPSRFSGSGSG-- TDFTLSISTLQPEDFATYYC | QQTDS----------- ------------FPFT | FGPGT KVDIK |
| iPS:435 453 | 21-225_152G1 0 | VK1\|L5/JK3 | | DIQMTQSPASVSA SVGDRVTITC | RAS--QGIS-- ----NWLA | WYQQKPGK APKLLIS | A-------- ASSLQS | GVPSRFSGNESG-- TDFTLSISSLQPEDFATYYC | QQTDS----------- ------------FPFT | FGPGT KVDVK |
| iPS:435 483 | 21-225_155A4 | VK1\|L5/JK3 | | DIQMTQSPASVSA SVGDRVTITC | RAS--QGIS-- ----NWLA | WYHQKPGK APKLLIY | A-------- ASSLQG | GVPSRFSGSGSG-- TDFTLSISSLQPEDFATYYC | HQTDS----------- ------------FPFT | FGPGT KVDIK |
| iPS:435 485 | 21-225_155B4 | VK1\|L5/JK3 | | DIQMTQSPASVSA SVGDRVTITC | RAS--QDIS-- ----NWLA | WYQQKPGK APKLLIY | A-------- ASSLQG | GVPSRFNGSGSG-- TDFTLSISSLQPEDFATYYC | HQTDS----------- ------------FPFT | FGPGT KVDIK |
| iPS:435 787 | 21-225_180A3 | VK1\|L5/JK3 | | DIQMTQSPSSVSA SVGDRVTITC | RAS--QDIT-- ----SWLA | WYQQKPGK APKLLIY | A-------- ASSLQS | GVPSRFSGSGSG-- TDFILTISSLQPEDFAFYHC | QQANS----------- ------------IPFT | FGPGT KVDIN |
| iPS:435 809 | 21-225_182H5 | VK1\|L5/JK3 | | DIQMTQSPSSVYA SVGDRVTITC | RAS--QDIT-- ----SWLA | WYQQKPGK APKLLIY | A-------- ASSLQS | GVPSRFSGSGSG-- TDFILTIISVQPDDFATYYC | QQVNS----------- ------------FPFT | FGHGT KVDIK |
| iPS:435 889 | 21-225_186A1 1 | VK1\|L5/JK3 | | DIQMTQSPSSVSA SVGDRVTITC | RAS--QDIT-- ----SWLA | WYQQKPGK APKLLIY | A-------- ASSLQS | GVPSRFSGSGSG-- TDFILTIITSVQPDDFATYYC | QQVNS----------- ------------FPFT | FGHGT KVDIK |
| iPS:435 965 | 21-225_192H2 | VK1\|L5/JK3 | | DIQMTQSPSSVSA SVGDRVTITC | RAS--QGIS-- ----SWIA | WYQQKPGK APKLLIY | G-------- ASSLQS | GVPSRFSGSGSG-- TDFTLTISSLQPEDFATYYC | QQSNS----------- ------------FPFT | FGPGT KVDVK |
| iPS:436 106 | 21-225_196F2 | VK1\|L5/JK3 | | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQTNS----------- ------------VPFT | FGPGT KVDIK |
| IPS:436 360 | 21-225_210H1 1 | VK1\|L5/JK3 | | DIQMTQSPSSVSA SVGDRVTITC | RAS--QGIS-- ----IWLA | WYQQKPGK APNLLIY | A-------- ASSLQS | GVPSRFSGRGSG-- TDFTLTISSLQPEDFATYYC | QQAKS----------- ------------FPFT | FGPGT KVDIK |
| iPS:436 488 | 21-225_221A6 | VK1\|L5/JK3 | | DIQMTQSPSSVSA SVGDRVTITC | RAS--QGIS-- ----SWLA | WYQQKPGK APKLLIY | T-------- ASNLQS | GVPSRFSGSGSG-- TDFTLTISSLQPEDFATYYC | QQANS----------- ------------FPFT | FGPGT KVDIK |
| iPS:436 496 | 21-225_222E1 | VK1\|L5/JK3 | | DIQMTQSPSSVSA SVGDRVTITC | RAS--QGIS-- ----SWLA | WYQQKPGK APKLLIY | T-------- ASNLQS | GVPSRFSGSGSG-- TDFTLTISSLQPEDFATYYC | QQANS----------- ------------FPFT | FGRGT KVDIK |
| iPS:436 508 | 21-225_222F7 | VK1\|L5/JK3 | | DIQMTQSPSSVSA SVGDRVTITC | RAS--QGIS-- ----SWLA | WYQQKPGK APKLLIY | T-------- ASNLQS | GVPSRFSGSGSG-- TDFTLTISSVQPEDFATYYC | QQANS----------- ------------FPFT | FGPGT KVDIK |
| iPS:436 516 | 21-225_222C1 2 | VK1\|L5/JK3 | | DIQMTQCPSSVSA SVGDRVTITC | RVS--QGIS-- ----SWLA | WYQQKPGK ALKLVIY | T-------- ASNLQS | GVPSRFSGSGSG-- TDFTLTISSVQREDFATYYC | QQDNS----------- ------------FPFT | FGRGT KVDIK |
| IPS:437 234 | 21-225_64E2 | VK1\|L5/JK3 | | DIQMTQSPSSVSA SVGDRVTITC | RAS--QGIS-- ----RWLA | WYQQKPGK APKLLIY | A-------- ASSLQS | GVPSRFSGSGSG-- TDFTLTISSLQPEDFATYYC | QQANS----------- ------------FPFT | FGPGT KVDIK |
| iPS:392 996 | 21-225_28B1 | VK1\|L5/JK3 | | DIQMTQSPSSVSA SVGDRVTITC | RAS--QAIN-- ----DWLA | WYQQKPGK APKLLIY | A-------- ASSFQS | GVPSRFSGSGSG-- TDFTLTITSLQPEDFATYYC | QQASS----------- ------------FPFT | FGPGT KVDIK |
| iPS:393 010 | 21-225_25E11 | VK1\|L5/JK3 | | DIQMTQSPSSVSA SVGDRVTITC | RAS--QGIS-- ----NWLA | WYQQKPGK APKLLIY | T-------- ASSLQG | GVPSRFSGSGSG-- TDFTISISSLQPEDFATYYC | QQANS----------- ------------FPIT | FGPGT KVDIK |
| iPS:393 016 | 21-225_28F11 | VK1\|L5/JK3 | | DIQMTQSPSSVSA SVGDRVTITC | RAS--QGIS-- ----NWLA | WYQQKPGK APKLLIS | A-------- ASNLQS | GVPSRFRGSGSG-- TDFTLTITSLQPEDFATYCC | QQANS----------- ------------LPFT | FGPGT KVDIK |

| ID | Clone | Gene | | K_FR1 | K_CDR1 | K_FR2 | K_CDR2 | K_FR3 | K_CDR3 | K_FR4 |
|---|---|---|---|---|---|---|---|---|---|---|
| iPS:393 024 | 21-225_31H9 | VK1\|L5/JK3 | | DIQMTQSPSSVSA SVGDRVTITC | RAS--QGIT-- ----SWLI | WYQQRPGK APKLLIY | D-------- TSSLQS | GVPSRFSGSGSG-- TDFIFTISSLQPEDFATYYC | QQGNS----------- -----------FPFT | FGQGT KVDIK |
| iPS:393 080 | 21-225_34F3 | VK1\|L5/JK3 | | DIQMTQSPSSVSA TVGDRVTSTC | RAS--QGIS-- ----KWLA | WYQQKPGK APKLLIY | A-------- ASSLQS | GVPSRFSGSGSG-- TDFTLTISSLQPEDSATYYC | QQANS----------- ----------FPFT | FGPGT KVDIK |
| iPS:393 084 | 21-225_35C6 | VK1\|L5/JK3 | | DIQMTQSPSSVSA SVGDRVTITC | RAS--QGIS-- ----KWLA | WYQQKPGK APKPLIY | A-------- ASSLQS | GVPTRFSGSGSG-- TDFTLTISSLQPEDFAIYYC | QQANS----------- ----------FPFT | FGPGT KVDIK |
| iPS:393 086 | 21-225_36H5 | VK1\|L5/JK3 | | DIQMTQSPSSVSA SVGDRVTITC | RAS--QGIS-- ----RWLA | WYQQKPGK APELLIY | A-------- ASRLQS | GIPSRFSGSGSG-- TDFTLTISSLQPEDFATYYC | QQANS----------- ----------FPFT | FGPGT KVDIK |
| iPS:393 098 | 21-225_35G6 | VK1\|L5/JK3 | | DIQMTQSPSSVSA SVGDRLTITC | RAS--QGIS-- ----RWLA | WYQQKVGK VPKLLIY | A-------- ASRLQS | GVPSRFSGSGSG-- TAFTLTIGSLQPEDFATYYC | QQANS----------- ----------FPFT | FGPGT KVDLK |
| iPS:393 112 | 21-225_33G1 | VK1\|L5/JK3 | | DIQMTQSPSSVSV SVGDRVTITC | RAS--QGIS-- ----KWLA | WYQQKPGK APKLLIY | G-------- AYSLQS | GVPSRFSGSGSG-- TDFTLTISSLQPEDFATYYC | QQANS----------- ----------FPFT | FGPGT KVDIK |
| iPS:393 116 | 21-225_34G7 | VK1\|L5/JK3 | | DIQMTQSPSSVSA SVGDRVTITC | RAS--QLIS-- ----KWLA | WYQQKPGK APKLLIY | A-------- ASSLQS | GVPLRFSGSGSG-- TDFTLTISSLQPEDFATYYC | QQANS----------- ----------FPFT | FGPGT KVDIK |
| iPS:393 132 | 21-225_33H7 | VK1\|L5/JK3 | | DIQMTQSPSSVSA SVGDRVTITC | RAS--QGIS-- ----RWLA | WYQQKVGK VPKLLIY | A-------- ASRLQS | GVPSRFSGSGSG-- TDFTLTISSLQPEDFATYYC | QQANI----------- ----------FPFT | FGPGT KVDLK |
| iPS:393 140 | 21-225_35H12 | VK1\|L5/JK3 | | DIQMTQSPSSVSA SVGDRVTITC | RAS--QGIS-- ----RWLA | WYQQKPGK APELLIY | A-------- ASRLQS | GIPSRFSGSGSG-- TDFTLTISSLQPEDFATYYC | QQANS----------- ----------FPFT | FGPGT KVDIK |
| iPS:393 954 | 21-225_4H6 | VK1\|L5/JK3 | | DIQMTQSPSSVSA SVGDRVTITC | RAS--QGIS-- ----RWLA | WYQQKPGK APKLLIY | G-------- ASSLQS | GVPSRFSGSGSG-- TDFTLTISSLQPEDFAIYYC | QQANS----------- ----------FPFT | FGPGT KVDIK |
| iPS:398 502 | 21-225_23B11 | VK1\|L5/JK3 | | DIQMTQSPSSVSA SVGDRVTITC | RAS--QGIT-- ----KWLA | WYQQKPGK APKVLIY | A-------- ASSLQS | RVPSRFSGSRSG-- TDFTLTISSLQPEDFATYYC | QQANS----------- ----------FPFT | FGPGT KVDIK |
| iPS:398 520 | 21-225_31C4 | VK1\|L5/JK3 | | DIQMTQSPSSVSA SVGDRVTITC | RAS--QGIS-- ----KWLA | WYQQKPGK APKPLIY | A-------- ASSLQS | GVPTRFSGSGSG-- TDFTLTISSLQPEDFAIYYC | QQANS----------- ----------FPFT | FGPGT KVDIK |
| iPS:402 223 | 21-225_30A11 | VK1\|L5/JK3 | | DIQMTQSPSSVSA SVGDRVTITC | RAS--QGIS-- ----RWLA | WYQQKPGR APELLIY | A-------- ASRLQS | GIPSRFSGSGSG-- TDFTLTISSLQPEDFATYYC | QQANS---------- -----------FPFT | FGPGT KVDNK |
| | Germline | | | **K_FR1** | **K_CDR1** | **K_FR2** | **K_CDR2** | **K_FR3** | **K_CDR3** | **K_FR4** |
| | VK4\|B3/JK1 | | 32130 | DIVMTQSPDSLAV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- -----------TPWT | FGQGT KVEIK |
| iPS:426 112 | 21-225_12F12 | VK4\|B3/JK1 | | DIVMTQSPDSLAV SLGERATINC | KSS-- QTVLFSSNNNH YLA | WYQQKPGQ PPNLLIY | W-------- ASTRAS | GVPDRFSGSGSG-- TDFTLTISSLQAEDVAVYYC | QQYYS----------- -----------SPWT | FGQGT KVEIK |
| iPS:451 137 | 21-225_74A7 | VK4\|B3/JK1 | | DIVMTQSPDSLAV SLGERATINC | KSS-- QSVLFSSNNYN YLA | WYQQRPGQ PPNLLIY | W-------- ASTRES | GVPDRFSGSGSG-- TDFTLTISSLQAADVAVYYC | QQYHS----------- -----------SPPT | FGQGT TVQIK |
| iPS:433 909 | 21-225_43D8 | VK4\|B3/JK1 | | DIVMTQSPDSLAV SLGERATINC | KSS-- QSVLMTSNDKN YLT | WYQQRPGQ PPKLLIY | W-------- ASTRES | GVPDRFSGGGSG-- TDFTLTISGLQAEDVAVYYC | QQYYS----------- -----------TPPT | FGQGT KVEIK |
| iPS:434 177 | 21-225_56A1 | VK4\|B3/JK1 | | DIVMTQSPDSLAV SLGERATINC | KSS-- QSVLHSSNNKN YLV | WYQQRPGQ PPNLLIY | W-------- ASTRES | GVPDRFSGSGSG-- TDFTLTISSLQAEDVAVYYC | QQYYS----------- -----------TPPT | FGQGT KVEIK |
| iPS:434 237 | 21-225_61B5 | VK4\|B3/JK1 | | DIVMTQSPDSLAV SLGERATINC | KSS-- QSVLYSSNNNN SLT | WYQLKPGQ PPKKLIY | W-------- ASTRES | GVPDRFSGSGSG-- TDFTLTISSLQAEDVAVYYC | QQYYS----------- -----------TPPT | FGQGS KVEIK |

| | | | | | | | | | | |
|---|---|---|---|---|---|---|---|---|---|---|
| iPS:434 285 | 21-225_57A11 | VK4\|B3/JK1 | | DIVMTQSPDSLIV SLGERATINC | KSS-- QSVLHSSNNYN YLA | WYQQRPGQ PPKLVIY | W-------- ASTRAS | GVPDRFSGSGSG-- TDFTLTISSLQAEDMAVYYC | QQYYS----------- -----------TPWT | FGQGT KVEFK |
| iPS:434 295 | 21-225_58B9 | VK4\|B3/JK1 | | DIVMTQSPDSLAV SLGERATINC | KSG-- QSILYSSNNNN YLA | WYQQKPGQ PPKKLIY | W-------- ASTRDS | GVPARFSGSGSG-- TDFTLTISSLQAEDVAVYYC | QQYYS----------- -----------TPPT | FGQGS KVEIK |
| IPS:434 321 | 21-225_59F10 | VK4\|B3/JK1 | | DIVMTQFPDSLAV SLGERATINC | KSS-- QTVLYRSNNYN YLA | WYQQKPGQ PPKLLIY | W-------- ASTRES | GVPDRFSGSGSG-- TDFTLTISSLQAEDVAVYYC | QQYFS----------- -----------TPPT | FGQGT KVEIK |
| IPS:434 431 | 21-225_70E7 | VK4\|B3/JK1 | | DIVMTQSPDSLAV SLGERATLNC | KSS-- QSVLYSSNNNN YLA | WYQQKPGQ PPKLLIY | W-------- ASTRES | GVPDRFSGSGSG-- TDFTLTISSLQAEDVAVYYC | QQYYN----------- -----------IPPT | FGQGT KVEIK |
| IPS:434 475 | 21-225_74F9 | VK4\|B3/JK1 | | DIVMTQSPDSLAV SLGERATINC | KSS-- QSVLYSSNNYN YLA | WYQQKPGQ PPKKLIY | W-------- ASTRES | GVPDRFSGSGSG-- TDFTLTISSLQAEDVAVYSC | QQYYS----------- -----------SPPT | FGQGT KVEIK |
| IPS:434 477 | 21-225_74A6 | VK4\|B3/JK1 | | DIVMTQSPDSLAV SPGERATINC | KSS-- QSVLHSSNNNN YLA | WYQQKPGQ PPDLLIY | W-------- ASTRES | GVPDRFSGSGSG-- TDFTLTISSLQAEDVAVYYC | QQYFS----------- -----------TPWT | FGQGT QVEIK |
| IPS:434 481 | 21-225_74B10 | VK4\|B3/JK1 | | DIVMTQSPDSLAV SLGERATINC | KSS-- QSVLHSSNNKN YLT | WYQQKPGQ PPKLLIY | W-------- ASTRES | GVPDRFSGSGSG-- TDFSLTIGSLQAEDVAVYYC | QQYYS----------- -----------IPPT | FGQGT KVEIK |
| IPS:434 487 | 21-225_76G2 | VK4\|B3/JK1 | | DIVMTQSPDSLAV SLGERATINC | KSS-- QSVLHSSNNYN YLA | WYQQRPGQ PPRLLIY | W-------- ASTRES | GVPDRFSGSGSG-- TDFTLTISSLQVEDVAVYYC | QQYYS----------- -----------SPPT | FGQGT KVEIK |
| IPS:434 493 | 21-225_76F3 | VK4\|B3/JK1 | | DIVMTQCPDSPAV SLGERATINC | KSS-- QSVLFSSNNYN YLA | WYQQRPGQ PHDLLIY | W-------- ASTRES | GVPDRFSGSGSG-- TDFTLTISSLQAADVAVYYC | QQYHS----------- -----------SPLT | FGQGT TVQIK |
| IPS:434 509 | 21-225_76F5 | VK4\|B3/JK1 | | VIVLTQSPDSLAV SLGERATINC | KSS-- QSVLHSSNSYN YLA | WYQQKPGQ SPKVLIY | W-------- TSTRES | GVPDRFSGSGSG-- TDFTLTISSLQAEDVAVYYC | QQYYS----------- -----------SPPT | FGQGT KVEIK |
| IPS:434 525 | 21-225_76E8 | VK4\|B3/JK1 | | DIVMTQSPDSLAV SLGERATINC | KSS-- QTVLHSSNNYN YLA | WYQQKPGQ PPKVLIY | W-------- TSTRES | GVPDRFSGSGSG-- TDFTLTISSLQAEDVAVYYC | QQYFS----------- -----------SPLT | FGQGT KVEIK |
| IPS:434 549 | 21-225_76E11 | VK4\|B3/JK1 | | DIVMTQSPDSLAV SLGEGATINC | KSR-- QSVLHSSNNYN YLA | WYQQKAGQ PPKLLIY | W-------- ASTRES | GVPDRFSGSGSG-- TDFTLTISSLQAEDVAVYYC | QQYYS----------- -----------TPPT | FGQGT KVEIR |
| IPS:434 551 | 21-225_75C4 | VK4\|B3/JK1 | | DIVMTQSPDSLAV SLGERATINC | KSS-- QSILYSSNNNN YLA | WFQQKPGQ PPKLLIY | W-------- ASTRES | GVPDRFSGSGSG-- TDFTLTISSLQAEDVAVYYC | QQYYI----------- -----------TPPT | FGQGT KVEIK |
| IPS:434 575 | 21-225_77C7 | VK4\|B3/JK1 | | DIVMTQSPDSLAV SLGERATINC | KSS-- QTVLHSSNNYN YLA | WYQQKPGQ PPKVLLY | W-------- TSTRES | GVPDRFSGSGSG-- TDFTLTISSLQAEDVAVYYC | QQYFS----------- -----------SPPT | FGQGT RVEIK |
| IPS:434 597 | 21-225_77C10 | VK4\|B3/JK1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QHYYN----------- -----------TPWK | FVQGT KVEIT |
| IPS:434 617 | 21-225_74B8 | VK4\|B3/JK1 | | DSVMTQSPDSLAV SLGERATINC | KSS-- QSVLHSSNKKN YLA | WYQQKPGQ PPKLLIY | W-------- ASTRES | GVPDRFSGSGSG-- TDFTLTISSLQAEDVAVYYC | QQYYR----------- -----------TPWT | FGQGT KVEIK |

| | | | | | | | | | | |
|---|---|---|---|---|---|---|---|---|---|---|
| iPS:434 619 | 21-225_78C1 | VK4|B3/J K1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QHYYN----------- ------------TPWK | FVQGT KVEIK |
| iPS:434 639 | 21-225_74B7 | VK4|B3/J K1 | | DIVMTQSPDSLAV SLGERATINC | KSS-- QTVLHSSNNYN YLA | WYQQKPGQ PPKVLIY | W-------- TSTRES | GVPDRFSGSGSG-- TDFTLTISSLQAEDVAVYYC | QQYFS----------- ------------SPPT | FGQGT KVEIK |
| iPS:434 649 | 21-225_78E11 | VK4|B3/J K1 | | DIVMTQSPDSLAV SLGERATINC | KSS-- QSVLYSFNNYN YLA | WYQQKPGQ PPKKLIY | W-------- ASTRES | GVPDRFSGSGSG-- TDFTLTISSLQAEDVAVYSC | QQYYS----------- ------------SPPT | FGQGT KVEIK |
| iPS:434 653 | 21-225_74B5 | VK4|B3/J K1 | | DIVMTQSPDSLAV SLGERATINC | KSS-- QSVLFSSNNYN YLA | WYQQRPGQ PPNLLIY | W-------- ASTRES | GVPDRFSGSGSG-- TDFTLTISSLQAADVAVYYC | QQYYS----------- ------------SPPT | FGQGT TVQIK |
| iPS:434 655 | 21-225_78H12 | VK4|B3/J K1 | | DIVMTQSPDSLAV SLGERATINC | KSS-- QTVLHSFNNYN YLA | WYQQKPGQ PPKVLIY | W-------- TSTRES | GVPDRFSGSGSG-- TDFTLTISSLQAEDVAVYYC | QQYFS----------- ------------SPPT | FGQGT KVEIK |
| iPS:434 665 | 21-225_74G4 | VK4|B3/J K1 | | DIVMTQSPDSLAV SLGERATINC | KSS-- QSVLYSSNNNN YLA | WYQQKAGQ PPKLLIY | W-------- ASTRES | GVPDRFSGSGSG-- TDFTLTISSLQAEDVAVYYC | QQYYS----------- ------------IPPT | FGQGT KVEIK |
| iPS:434 675 | 21-225_79G6 | VK4|B3/J K1 | | DIVMTQSPDCLAV SLGERATISC | MSS-- QSVLHSFNNKN YLT | WYQQKPGQ PPKLLIY | W-------- ASTWES | GVPDRFSGSGSG-- TDFSLPIGSLQAEDVAVYYC | QQYYS----------- ------------IPPT | FGQGT KVEIK |
| iPS:434 685 | 21-225_79E9 | VK4|B3/J K1 | | DIVMTQSPDSLAV SLGERATINC | KSS-- QSILYSSNNNN YLA | WFQQKPGQ PPKLLIY | W-------- ASTRES | GVPDRFSGSGCG-- TDFTLTISSLQAEDVAVYYC | QQYYI----------- ------------TPPT | FGQGT KVEIK |
| iPS:434 689 | 21-225_79G10 | VK4|B3/J K1 | | DIVMTQSPDSLAV SLGERATINC | KSS-- QSVLFSSNNYN YLA | WYQQRPGQ PHNLLIY | W-------- ASTRES | GVPDRFSGSGSG-- TDFTLTISSLQAADVAVYYC | QQYHS----------- ------------SPLT | FGQGT TVQIK |
| iPS:434 697 | 21-225_79F12 | VK4|B3/J K1 | | DIVMTQSPDSLAV SLGERATINC | KSS-- QSILYSSNNYN YLA | WYQQKPGQ PPKLLIY | W-------- ASTRES | GVPDRFSGSGSG-- TDFTLTISSLQAEDVAVYYC | QQYYS----------- ------------TPWT | FGQGT KVEIK |
| iPS:434 707 | 21-225_80D3 | VK4|B3/J K1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QHYNE----------- ------------TPGK | FVQVT KVEIT |
| iPS:434 711 | 21-225_80H3 | VK4|B3/J K1 | | DIVMTQSPDSLAV SLGERATTNC | KSS-- QSVLHRSNNYN YLA | WYQQKPGQ PPKLLIY | W-------- ASTRES | GVPDRFSGSGSG-- TDFTLTISSLQAEDVAVYYC | QQYYS----------- ------------TPPT | FGQGT KVEIK |
| iPS:434 731 | 21-225_80E9 | VK4|B3/J K1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QQYYN----------- ------------TPWT | FVQGT KVEIK |
| iPS:434 761 | 21-225_81E5 | VK4|B3/J K1 | | DIVMTQSPDSLAV SLGERATINC | KSS-- QSVLFSSNNYN YLA | WYQQRPGQ PPNLLIY | W-------- ASTRES | GVPDRFSGSGSG-- TDFTLTISSLQAADVAVYYC | QQYYS----------- ------------SPLT | FGQGT TVQIK |
| iPS:434 771 | 21-225_81F9 | VK4|B3/J K1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYR | QHYND----------- ------------TPGK | FVQGI MVEIT |
| iPS:434 827 | 21-225_83F3 | VK4|B3/J K1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QHYND----------- ------------TPWK | FVQGI KVEIK |

| | | | | | | | | | | |
|---|---|---|---|---|---|---|---|---|---|---|
| iPS:434 829 | 21-225_83G3 | VK4|B3/JK1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QHYYN----------- ------------TPWT | FVQGT KVEIK |
| iPS:434 841 | 21-225_83G7 | VK4|B3/JK1 | | DIVMTQSPDSLAV SLGERATIIC | KSS-- QTVLHSSNNYN YLA | WYQQKPGQ PPKVLIY | W-------- TSTRES | GVPDRFSGSGSG-- TDFTLTISSLQAEDVAVYYC | QQYFS----------- ------------SPLT | FGQGT KVEIK |
| iPS:434 863 | 21-225_84G7 | VK4|B3/JK1 | | DIVMTQSPDSPAV SLGERATIIC | KSS-- QTVLHSSNNYN YLA | WYQQKPGQ PPKVLIY | W-------- TSTRES | GVPDRFSGSGSG-- TDFTLTISSLQAEDVAVYYC | QQYFS----------- ------------SPPT | FGQGT KVEIK |
| iPS:434 877 | 21-225_85H2 | VK4|B3/JK1 | | DSMMTQSPDSLAV SLGERATINC | KSS-- QSVLHSSNKKN YLA | WYQQKPGQ PPKLLIY | W-------- ASTRES | GVPDRFSGSGSG-- TDFTLTISSLQAEDVAVYYC | QQYYR----------- ------------TPWT | FGQGT KVEIK |
| iPS:434 901 | 21-225_85H9 | VK4|B3/JK1 | | DIVMTQSPDSLAV SLGERATTNC | KSS-- QSVLHRSNNYN YLA | WYQQKPGQ PPKLLIY | W-------- ASTRES | GVPDRFSGSGSG-- TDFTLTISSLQAEDVAVYYC | QQYYS----------- ------------TPPT | FGQGT KVEIK |
| iPS:434 935 | 21-225_86E9 | VK4|B3/JK1 | | DIVMTQCPDSPAV SLGERATINC | KSS-- QSVLHRSNNYN YLA | WYQQKPGQ PPNLLIY | W-------- ASTRES | GVPDRFSGSGSG-- TDFTLTISSLQAADVAVYYC | QQYHS----------- ------------SPLT | FGQGT TVEIK |
| iPS:434 965 | 21-225_88A1 | VK4|B3/JK1 | | NIVMTQSPDSLAV SLGARATINC | KSS-- QSVLHSSNNYN YLT | WYQQKPGQ PPKLLLY | W-------- ASTRKS | GVPDRFSGSGSG-- TDFTLTISSLQAEDVAVYFC | QQYYS----------- ------------SPPT | FGQGT KVEIK |
| iPS:434 971 | 21-225_88G2 | VK4|B3/JK1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QQYYN----------- ------------TPWT | FVQGT KVEIK |
| iPS:434 973 | 21-225_88B4 | VK4|B3/JK1 | | DIVMTQSPDSLAV SLGERATINC | KSS-- QSVLYISNNNN YLA | WYQQKPGQ PPKLLIY | W-------- ASTRES | GVPARFSGSGSG-- TDFTLTISSLQAEDVAVYYC | QQYYS----------- ------------TPPT | FGQGT KVEIK |
| iPS:434 997 | 21-225_88C10 | VK4|B3/JK1 | | DIVMTQSPDSLAV SLGERATINC | KSS-- QSVLHSSNNWN YLA | WHQQKPGQ PPKLLIH | W-------- AFTRKS | GVPDRFSGGGSG-- TNFTLTISSLQAEDVAVYYC | QQYYR----------- ------------APPT | FGQGT KVEIK |
| IPS:435 051 | 21-225_90D9 | VK4|B3/JK1 | | DIVMTQSPDSLAV SLGERATIIC | KSS-- QTVLHSSNNYN YLA | WYQQKPGQ PPKVLIY | W-------- TSTRES | GVPDRFSGSGSG-- TDFTLTISSLQAEDVAVYYC | QQYLS----------- ------------SPLT | FGQGT KVEIK |
| IPS:435 053 | 21-225_75F9 | VK4|B3/JK1 | | DIVMTQSPDSLPV SLGERATVNC | KSS-- QSVLHNSNNNN YLA | WYQQKPGQ PPKLLIY | W-------- ASTRES | GVPDRFSGSGSG-- TDFTLTISSLQAEDVAVYYC | QQYYS----------- ------------SPPT | FGQGT KVEIK |
| IPS:435 071 | 21-225_91F1 | VK4|B3/JK1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QHYYN----------- ------------TPWK | FVQGT KVEIK |
| IPS:435 087 | 21-225_91G8 | VK4|B3/JK1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QQYYT----------- ------------TPWT | FGQGT KVEIK |
| IPS:435 113 | 21-225_92E6 | VK4|B3/JK1 | | DIVMTQSPDSLAA SLGERATINC | KSS-- QNILSSSNNKN YLT | WYQQKPGQ PPKILIY | W-------- TSTRES | GVPDRFSGSGFG-- TDFTLTISSLQAEDVAVYYC | QQYFS----------- ------------VPPT | FGQGT KVEIK |
| IPS:435 167 | 21-225_92F12 | VK4|B3/JK1 | | DIVMTQSPDSLAV SLGERATTNC | KSS-- QSVLHRSNNYN YLA | WYQQKPGQ PPKLLIY | W-------- ASTRES | GVPDRFSGSGSG-- TDFTLTISSLQAEDVAVYYC | QQYYS----------- ------------TPPT | FGQGT KVEIK |

| iPS:435 203 | 21-225_75A7 | VK4\|B3/JK1 | | DIVMTQSPDSLAV SLGERATINC | KSS-- QTVLHSSNNYN YLA | WYQQKPGQ PPKVLIY | W-------- TSTRES | GVPDRFSGSGSG-- TDFTLTISSLQAEDVAVYYC | QQYFS----------- -----------SPPT | FGQGT KVEIK |
| iPS:435 209 | 21-225_75A10 | VK4\|B3/JK1 | | NIVMTQSPDSLAV SLGARATINC | KSS-- QSVLHNSNNYN YLT | WYQQKPGQ PPKLLLY | W-------- ASTRKS | GVPDRFSGSGSG-- TDFTLTISSLQAEDVAVYFC | QQYYS----------- -----------SPPT | FGQGT KVEIK |
| iPS:435 211 | 21-225_94E11 | VK4\|B3/JK1 | | DIVMTQSPDSLAV SLGERATINC | KSS-- QSVLFSSNNYN YLA | WYQQRPGQ PPNLLIY | W-------- ASTRES | GVPDRFSGSGSG-- TDFTLTISSLQAADVAVYYC | QQYHS----------- -----------SPLT | TVQIK |
| iPS:435 215 | 21-225_94E12 | VK4\|B3/JK1 | | DIVMTQSPDSLAV SLGERATTNC | KSS-- QSVLHRSNNYN YLA | WYQQKPGQ PPKLLIY | W-------- ASTRES | GVPDRFSGSGSG-- TDFTLTISSLQAEDVAVYYC | QQYYS----------- -----------TPPT | FGQGT KVEIK |
| iPS:435 227 | 21-225_95G4 | VK4\|B3/JK1 | | DIVMTQCPDSLAV SLGERATINC | KSS-- QSVLFRSNNYN YLA | WYQQRPGQ PHNLLIY | W-------- ASTRES | GVPDRFSGSGYG-- TDFTLTISSVQAADVAVYYC | QQYHS----------- -----------SPLT | TVQIK |
| iPS:435 245 | 21-225_95E12 | VK4\|B3/JK1 | | DIVMTQSPDSLAV SLGERATINC | KSS-- QSVLYSSNNAN YLA | WYQQKPGQ PPNLFIY | W-------- ASTRES | GVPDRFSGSGSG-- TDFTLTISSLQAEDVAVYYC | QQYYS----------- -----------TPCS | FGQGT KVEIK |
| iPS:435 249 | 21-225_96E2 | VK4\|B3/JK1 | | DSVMTQSPDSLAV SLGERATINC | KSS-- QSVLHSSNKKN YLA | WYQQKPGQ PPKLLIY | W-------- ASTWES | GVPDRFSGSGSG-- TDFTLTISSLQAEDVAVYYC | QQYYR----------- -----------TPWT | FGQGT KVEIK |
| iPS:435 255 | 21-225_96D5 | VK4\|B3/JK1 | | DIVMTQSPDSLAV SLGERATINC | KSS-- QSVLHSSNNYN YLA | WYQQKPGQ PPNLLIY | W-------- ASTRES | GVPDRFSGSGSG-- TDFTLTISSLQAADVAVYYC | QQYYS----------- -----------SPPT | FGQGT TVEIK |
| iPS:435 257 | 21-225_96H5 | VK4\|B3/JK1 | | DIVMTQSPDSLAV SLGERATINC | KSS-- QSVLYSSNSHN YLA | WYQQKPGQ PPKLLIY | W-------- ASIRES | GVPDRFSGSGSG-- TDFTLSISSMQAEDVAVYYC | QQYYS----------- -----------TPCS | FGQGT KVEIK |
| iPS:435 267 | 21-225_96D10 | VK4\|B3/JK1 | | DIVMTQSPDSLAV SLGERATINC | KSS-- QNILSSSNNKN YLT | WYQQKPGQ PPKILIY | W-------- TSTRES | GVPDRFSGSGFG-- TDFTLTISSLQAEDVAVYYC | QQYFS----------- -----------VPPT | FGQGT KVEIK |
| iPS:435 279 | 21-225_97H4 | VK4\|B3/JK1 | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QHYND----------- -----------TPWK | FVQGT KVEIT |
| iPS:435 321 | 21-225_147E4 | VK4\|B3/JK1 | | DIVMTQSPDSLAV SLGERATINC | KSS-- QSVLHSSNNYN YLA | WYQLKPRQ PPKLLIY | W-------- ASTRES | GVPDRFSGRGSG-- TDFTLTISSLQAEDVAIYYC | QQYYS----------- -----------TPST | FGPGT KVEIK |
| iPS:435 353 | 21-225_148F8 | VK4\|B3/JK1 | | DIVMTQSLDSLAV SLGERATINC | KSS-- QSALHSSNNYN YLA | WYQQKPGQ PPKLLIY | W-------- ASTRKS | GVPDRFSGSGSG-- TDFTLTISSLQAEDVAVYYC | QQYYS----------- -----------IPPT | FGQGT KVEIK |
| iPS:435 369 | 21-225_149A2 | VK4\|B3/JK1 | | DIVMTQSPDSLAV SLGERATINC | KSS-- QSVLYSPNNNN YLA | WYQQKPGQ PPKLLIY | W-------- ASTRES | GVPDRFSGSGSG-- TDFTLTISSLQAEDVAVYYC | QQYYS----------- -----------TPCS | FGQGT KVEIK |
| iPS:435 373 | 21-225_149E3 | VK4\|B3/JK1 | | DIVMTQSPDSLAV SLGERATISC | KSS-- QTVLHNSNNHN YFA | WYQQKPGQ PPKLLIY | W-------- ASTLRS | GVPDRFSGSGSG-- TDFTLTISSLQAEDVAVYYC | QQYYS----------- -----------TPPT | FGQGT KVEIK |
| iPS:435 375 | 21-225_149H4 | VK4\|B3/JK1 | | DIVMTQSPDSLAV SLGERATINC | KSS-- QSVLSSSNDNN YLA | WYQQKPGR PPKLLIY | W-------- SSTRES | GVPDRFSGSGSG-- TDFTLTISSLQAEDVAVYYC | HQYYS----------- -----------YPPT | FGQGT KVEIK |

| | | | | | | | | | | |
|---|---|---|---|---|---|---|---|---|---|---|
| iPS:435 481 | 21-225_154A11 | VK4\|B3/JK1 | | DIVMTQSPDSLAV SLGERATINC | RSS-- QSVLHSSNNYN YLA | WYQQKPGQ PPKLLIY | W-------- ASKRDS | GVPDRFSGSGSG-- TDFTLTISSLQAEDVAVYYC | QQYFS----------- -----------SPRT | FGQGT KVEIK |
| iPS:435 557 | 21-225_158B12 | VK4\|B3/JK1 | | DIVMTQSPDSPAV SLGERATINC | KSS-- QNVLHSSNNNN YLT | WYQQRPGQ PPKLLIY | W-------- ASTRES | GVPDRFSGSGSG-- TEFTLTISSLQAEDVAVYYC | QQYYS----------- -----------TPPT | FGQGT KVEIK |
| iPS:435 627 | 21-225_162F6 | VK4\|B3/JK1 | | DIVMTQSPDSLAV SLGERATINC | KSS-- QNVLHSSNNNN YLT | WYQQRPGQ PPKLLIY | W-------- ASTRES | GVPDRFSGSGSG-- TEFTLTISSLQAEDVAVYYC | QQYYS----------- -----------TPPT | FGQGT KVEIK |
| iPS:435 701 | 21-225_170F6 | VK4\|B3/JK1 | | DIVMTQSPDSLAV SLGARATINC | KSS-- QSVLHSSNNYN YLA | WYQQRPGQ PPKVLIH | W-------- ASTRKS | GVPDRFSGSVSG-- TDFTLTINSLQAEDVAVYYC | QQYYS----------- -----------TPWT | FGQGT KVEIK |
| iPS:435 737 | 21-225_174G5 | VK4\|B3/JK1 | | DIVMTQSPDSLAV SLGARATINC | KSS-- QSVLHSSNNYN YLT | WYQQKSGQ PPKLLIY | W-------- ASTRES | GVPDRFSGSGSG-- TDFTLTISSLQAEDVAVYYC | QQYYR----------- -----------TPWT | FGQGT KVEIK |
| iPS:435 751 | 21-225_175D10 | VK4\|B3/JK1 | | DIVMTQSPDSLAV SLGERATINC | KSS-- QSVLYSSNNNN YLA | WYQQKPGQ PPNLLIY | W-------- TSTRES | GVPDRFSGSGSG-- TNFTLTISSLQAEDVAVYYC | QQYYS----------- -----------TPPT | FGQGT KVEIK |
| iPS:435 773 | 21-225_177B12 | VK4\|B3/JK1 | | DIVMTQSPDSLAV SLGERATVNC | KSS-- QSVLHSSNNNN YLT | WYQQKPGQ PPKLLIY | W-------- ASTRES | GVPDRFSGSGSG-- TDFTLTISSLQAEDVAVYYC | QQYYS----------- -----------SPPT | FGQGT KVEIK |
| iPS:435 801 | 21-225_181E5 | VK4\|B3/JK1 | | DIVMTQSPDSLAV SLGERATINC | KSS-- QSVLHSSNNYN YLT | WYQQKPGQ PPKLLIY | W-------- ASTRES | GVPDRFSGSGSG-- TDFTLTISSLQAEDVAVYYC | HQYFI----------- -----------TPWT | FGQGT KVEIK |
| iPS:435 841 | 21-225_191D8 | VK4\|B3/JK1 | | DIMMTQSPDSLAV SLGERAIISC | RSS-- QSVLHSSNNYN YLA | WYQQKPGH PPKLLIY | W-------- ASTRES | GVPDRFSGSGSG-- TDFTLTISSLQAEDVAVYYC | QQYYS----------- -----------TPPT | FGLGT KVEIK |
| iPS:435 925 | 21-225_190D7 | VK4\|B3/JK1 | | DIVMTQSPDSLAV SLGERATINC | KSS-- QSVLSSSNNYN YLV | WYQQKPGQ PPKLLIY | W-------- ASTRKS | GVPDRFSGSGSG-- TDFTLTISSLQADDVAVYYC | QQYYR----------- -----------TPWT | FGQGT KVEIK |
| iPS:436 021 | 21-225_193G4 | VK4\|B3/JK1 | | DIVMTQSPDSLAV SLGERATINC | KSS-- QSVLYSSNNYN YLT | WYQQKPGQ PPKLLIY | W-------- ASTRKS | GVPDRFSGSGSG-- TDFTLTISSLQADDVAVYYC | QQYYI----------- -----------TPWT | FGQGT KVDIK |
| iPS:436 114 | 21-225_196G8 | VK4\|B3/JK1 | | DIVMTQSPDSLIV SLGERATVNC | KSS-- QSVLHSSNNNN YLA | WYQQKPGQ PPKLLIY | W-------- ASTRES | GVPDRFSGSGSG-- TDFTLAISSLQAEDVAVYYC | QQYYN----------- -----------TPPT | FGQGT KVEIK |
| iPS:436 150 | 21-225_197H4 | VK4\|B3/JK1 | | DIMMTQSSDSLTV SLGERAIISC | RSS-- QSVLHSFNNYN YLA | WYQQKAGH PPNLLIY | W-------- ASTRES | GVPDRFSGSGSG-- TDFTLPISSLQAEDVAVYYC | QQYYS----------- -----------TPPT | FGLGT KVEIK |
| iPS:436 154 | 21-225_197C6 | VK4\|B3/JK1 | | DIMMTQSPDSLAV SLGERAIISC | RSS-- QSVLHSSNNYN YLA | WYQQKPGH PPNLLIY | W-------- ASTRES | GVPDRFSGSGSG-- TDFTLTISSLQAEDVAVYYC | QQYYS----------- -----------TPPT | FGLGT KVEIK |
| iPS:436 218 | 21-225_200G7 | VK4\|B3/JK1 | | DIVMTQSPDSPTA SLGERATVKC | KSS-- QSVLHSSNNNN YLA | WYQQKPGQ PPKLLIY | W-------- ASTRES | GVPDRFSGSGSG-- TDFTLAISSLQAEDVAVYYC | QQYYN----------- -----------TPPT | FGQGT KVEIK |
| iPS:436 272 | 21-225_201F5 | VK4\|B3/JK1 | | DIVMTQSPESLAV SLGERATINC | KSS-- QSVLYSSNNKN YLV | WYQQKPGQ PPKLLIY | W-------- ASTRES | GVPDRFSGSGSG-- TDFTLTISSLQAEDVAVYYC | QQYYS----------- -----------TPPT | FGQGT KVEIK |

| | | | | | | | | | | |
|---|---|---|---|---|---|---|---|---|---|---|
| iPS:436 400 | 21-225_213H7 | VK4\|B3\|JK1 | | DIVMTQSPDSLAV SLGERATINC | KSS-- QNVLDISNNKN SLG | WFQQKPGQ PPKLLIN | W-------- ASTRES | GVPDRFSGSGSG-- TDFTLTISSLQTEDVAVYHC | QQYYN----------- ------------IPPT | FGRGT KVEIK |
| iPS:436 402 | 21-225_213H12 | VK4\|B3\|JK1 | | DIVMTQSPDSLAV SLGERATITC | KSS-- QNVLKTSNNRN YLA | WYQQKPGQ PPKVLIY | W-------- ASTRES | GVPDRFSGSGSG-- TDFTLIISSLQAEDVAVYYC | HQYYS----------- ------------IPWT | FGQGT KVEIK |
| iPS:436 500 | 21-225_222H3 | VK4\|B3\|JK1 | | DIVMTQSPDSLAV SLGERATINC | KSS-- QSVLKSSNHRN YLA | WYQQKPGQ PPQLLIY | W-------- ASTRET | GVPDRFSGSGSG-- TDFTLTISSLQAEDVSVYSC | QQYSS----------- ------------IPWT | FGQGT KVEIN |
| iPS:436 520 | 21-225_223G10 | VK4\|B3\|JK1 | | DIVMTQSPDSLAV SLGERATINC | KSS-- QSILLSSNNKN YLA | WHQQKPGQ PPKLLIY | W-------- ASTRES | GVPDRFSGSGSG-- TDFTLTISSLQAEDVAVYYC | LQYFS----------- ------------TPWT | FGQGT KVEIK |
| iPS:436 544 | 21-225_224H5 | VK4\|B3\|JK1 | | DIVMTQSPDSLAV SLGERATINC | KSS-- QSVLYSSNNFN YLT | WYQQKPGQ PPKLLIY | W-------- ASTRES | GVPDRFSGSGSG-- TDFTLTINSLQAEDVAVYYC | QQYYS----------- ------------TPPT | FGQGT KVEIK |
| iPS:436 550 | 21-225_224D8 | VK4\|B3\|JK1 | | DIVMTQSPDSLAV SLGERAAINC | KSS-- QSVLYSSNNKN YLA | WYQQKPGQ PPKLLIY | SSTRKS | GVPDRFSGSGSG-- TDFTLTISSLQAEDVAVYYC | QQYFS----------- ------------TPPT | FGQGT KVEIK |
| iPS:436 574 | 21-225_225F5 | VK4\|B3\|JK1 | | DIVMTQSPDSLAV SLGERATINC | KSS-- QNVLYNSNNNN YLA | WYQQKPGQ PPNLLIY | W-------- ASTRES | GVPDRFSGSGSG-- TDFTLTISSLQAEDVAVYFC | QQYYS----------- ------------SPPT | FGQGT KVEIN |
| iPS:436 586 | 21-225_225F11 | VK4\|B3\|JK1 | | DIVMTQSPDSLAV SLGERATINC | KSS-- QSVLYSSNNYN YLA | WYQQRPGQ PPKLLIY | W-------- ASTRES | GVPDRFSGSGSG-- PDFTLTISSLQAEDVAVYYC | QQYYT----------- ------------TPPT | FGQGT KVEIK |
| iPS:436 600 | 21-225_226F6 | VK4\|B3\|JK1 | | DIVMTQSPDSLAV SLGERATINC | KSS-- QSILYSSNNYN YLA | WYQQKPGQ PPKLLIY | W-------- ASTRES | GVPDRFSGSGSG-- PDFTLTISSLQAEDVAVYYC | QQYYT----------- ------------TPPT | FGQGT KVEIK |
| iPS:436 616 | 21-225_226D11 | VK4\|B3\|JK1 | | DIVMTQSPDSLAV SLGERATINC | KSS-- QNVLHSSNSNN YLV | WYQQKPGQ PPKLLIY | W-------- ASTRES | GVPDRFRGSGSG-- TDFTLTISSLQAEDVAVYYC | QQYYK----------- ------------TPWT | FGQGT KVEIK |
| iPS:436 622 | 21-225_226A12 | VK4\|B3\|JK1 | | DIVMTQSPDSLAV SLGERATINC | KSS-- QNVLYSSNNNN YLA | WYQQTPGQ PPKLLFY | W-------- ASTRKS | GVPDRFSGSGSG-- TDFTLTISSLQAEDVAVYYC | QQYYS----------- ------------SPPT | FGQGT KVEIK |
| iPS:436 638 | 21-225_227C7 | VK4\|B3\|JK1 | | DIVMTQSPDSLAV SLGERATINC | RSS-- QIVLSDSNNNN YLA | WYQQKPGQ PPNLLIY | W-------- ASTRES | GVPDRFSGSGSG-- TDFTLTISSLQAEDVAVYFC | QQYYS----------- ------------SPPT | FGQGT KVEIK |
| iPS:437 356 | 21-225_74B1 | VK4\|B3\|JK1 | | DIVMTQSPDFLAV SLGERATTNC | KSS-- QSVLHRSNNYN YLA | WYQQKPGQ PPKLLIY | W-------- ASTRES | GVPDRFSGSGSG-- TDFTLTISSLQAEDVAVYYC | QQYYS----------- ------------TPPT | FGQGT KVEIK |
| iPS:437 361 | 21-225_74C1 | VK4\|B3\|JK1 | | DIVMTQCPDSPAV SLGERASINC | KSS-- QSILHSSNNYN YLA | WYQQKPGH PHKLLIY | W-------- ASTRES | GVPDRFSGSGYG-- TDFTLTISSLQAEDVAVYYC | QQYYS----------- ------------TPWT | FGQGT KVEIK |
| iPS:437 379 | 21-225_74H2 | VK4\|B3\|JK1 | | DIVMTQSPDSLAV SLGERATINC | KSS-- QSVLHSSNNKN YLT | WYQQKPGQ PHKLLIY | W-------- ASTRES | GVPDRFSGSGSG-- TDFSLTIGSLQAEDVAVYYC | QQYYS----------- ------------IPPT | FGQGT KVEIK |
| iPS:446 094 | 21-225_77E1 | VK4\|B3\|JK1 | | DIVMTQSPDSLAV SLGERATINC | KSS-- QTVLHSSNNYN YLA | WYQQKPGQ PPKVLIY | TSTRES | GVHDRFSGSGSG-- TDFTLTISSLQAEDVAVYYC | HQYLS----------- ------------SPLT | FGQGT KVEIK |

| iPS:451 116 | 21-225_164A4 | VK4\|B3/JK1 | | DIVMTQYPDSRAV SLGERATIKC | KSS-- QSVLYSSNNKN YLT | WYQQKPGQ PPKLFIY | W-------- ASTRES | GVPDRFSGSGCG-- TDFTLTISSVQAEDVAVYYC | QQYFS----------- -----------TPWT | FGQGT KVEIK |
| iPS:451 124 | 21-225_74F6 | VK4\|B3/JK1 | | DIVMTQSPDSLAV SLGERATINC | KSS-- QNILSSSNNKN YLT | WYQQKPGQ PPKILIY | W-------- TSTRES | GVPDRFSGSGFG-- TDFTLTISSLQAEDVAVYYC | QQYFS----------- -----------VPLT | FGQGT KVEIK |
| iPS:451 127 | 21-225_164A7 | VK4\|B3/JK1 | | DIVMTQCPDSLAV SLGERATINC | KSS-- QSVLHSSNNNN YLA | WYQQKPGQ PHKLLIY | W-------- TSTRES | GVPDRFSGSGYG-- TDFSLTIASLQAEDVAVYYC | QQYYS----------- -----------IPLT | FGQGT KVEIK |
| iPS:451 129 | 21-225_94D2 | VK4\|B3/JK1 | | DIVMTQSPDSLAV SLGERATINC | KSS-- QSVLHSSNNKN YLT | WYQQKPGQ PHKLLIY | W-------- ASTRES | GVPDRFSGSGSG-- TDFSLTIGSLQHEDVAVYYC | QQYHS----------- -----------IPPT | FGHGT KVEIK |
| iPS:451 133 | 21-225_95H4 | VK4\|B3/JK1 | | DIVMTQCPDSLAV SLGERATINC | KSS-- QSVLFSSNNYN YLA | WYQQRPGQ PHNLLIY | W-------- ASTRES | GVPDRFSGSGSG-- TDFTLTISSLQAADVAVYYC | QQYHS----------- -----------SPLT | TVQIK |
| iPS:392 786 | 21-225_24E1 | VK4\|B3/JK1 | | DIVMTQSPDSLAV SLGERATINC | KSS-- QSVLYTSNNNN YLT | WYQQKPGQ RPNLLIY | W-------- ASTRES | GVPDRFSGSGSG-- TDFTLTISSLQAEDVAVYYC | QQFYS----------- -----------TPPT | FGQGT KVEIK |
| iPS:392 886 | 21-225_23A12 | VK4\|B3/JK1 | | DIVMTQSPDSLAV SLGERATINC | KSS-- QSVLYSSNNNN YLA | WYQQKPGQ PPKLLIY | W-------- TSTRES | GVPDRFSGSGSG-- TDFTLTISSLQAEDVAVYYC | QQYYD----------- -----------TPPT | FGQGT KVEIK |
| iPS:392 928 | 21-225_25A4 | VK4\|B3/JK1 | | DIVMTQFPDSLAV SLGERATIKC | KSS-- QSVLYSSHNNN YLA | WYQQKPGQ PPKLLLY | W-------- ASTRES | GVPDRFSGSGSG-- TEFTLTISSLEAEDVAVYYC | QQYYS----------- -----------TPPT | FGQGT KVEIK |
| iPS:392 960 | 21-225_29E6 | VK4\|B3/JK1 | | DIVMTQFPDSLAV SLGERATINC | KSS-- QSVLYSSHNNY YLT | WYQQKPGQ PHKLLLY | W-------- ASSRES | GVPDRFSGSGSG-- TEFTLTISSLQAEDVAVYYC | QQYYS----------- -----------TPPT | FGQGT KVEIK |
| iPS:392 992 | 21-225_26C4 | VK4\|B3/JK1 | | DIVMTQSPDSLAV SLGERATINC | KSS-- QSVLYRSNNYN YLA | WYQHKPGQ PPKLLIY | W-------- ASTRES | GVPDRFSGSGSG-- TDFTLTISSLQAEDVAVYYC | QQYYS----------- -----------TPPT | FGQGT KVEFK |
| iPS:393 368 | 21-225_29H8 | VK4\|B3/JK1 | | DIVMTQSPDSLAV SLGERATINC | RSS-- QTILHSSNNYN YLA | WYQQKPGQ PPKLLIY | W-------- ASTRES | GVPDRFSGSGSG-- TDFTLTISSLQAEDVAVYYC | QQYYC----------- -----------TPPT | FGQGT KVEIK |
| iPS:393 942 | 21-225_11E5 | VK4\|B3/JK1 | | DIVMTQSPDSLAV SLGERATLNC | KSS-- QSVLYSSNNNN YLT | WYQQKPGQ RPKLLIY | W-------- ASTRES | GVPDRFSGSGSG-- TNFTLTISSLQAEDVAVYYC | QQYYS----------- -----------TPPT | FGQGT KVEIK |
| iPS:398 506 | 21-225_23G12 | VK4\|B3/JK1 | | DIVMTQSPDSLAV SLGERATINC | KSS-- QSILFSSNNNN YLA | WYQQKPGQ PPKLLIY | W-------- ASTRES | GVPDRFSGSGSG-- TDFTLTISSLQAEDVAVYFC | QQYSS----------- -----------TPWT | FGQGT KVEIK |
| | | Germline | | K_FR1 | K_CDR1 | K_FR2 | K_CDR2 | K_FR3 | K_CDR3 | K_FR4 |
| | VK1\|A30/JK2 | | 32131 | DIQMTQSPSSLSA SVGDRVTITC | RAS--QGIR-- -----NDLG | WYQQKPGK APKRLIY | A-------- ASSLQS | GVPSRFSGSGSG-- TEFTLTISSLQPEDFATYYC | LQHNS----------- -----------YPYT | FGQGT KLEIK |
| iPS:426 114 | 21-225_28H2 | VK1\|A30/JK2 | | DIQMTQSPSSLSA SIGDRVTITC | RAS--QGIR-- -----NDLG | WYQQKPGK APKRLIY | A-------- ASSLQS | GVPSRFSGSGSG-- TEFTLTISSLQPEDFATYYC | LQHYS----------- -----------YPRS | FGQGT KLEIK |
| iPS:426 116 | 21-225_29E2 | VK1\|A30/JK2 | | DIQMTQSPSSLSA SVGDRVTITC | RAS--QAIR-- -----NDLG | WYQQRPGK APKRLIY | A-------- ASSLQS | GVPSRFSGSGSG-- TEFILTISSLQPEDFATYYC | LQHYN----------- -----------YPRS | FGQGT KLEIK |
| iPS:434 231 | 21-225_61F2 | VK1\|A30/JK2 | | DIQMTQSPSSLSA SVGDRVTVTC | RAS--QGIR-- -----DDLG | WYQQKPGK APERLIY | A-------- ASSLQS | GVPSRFSGSGSG-- TEFTLTISSLQPEDFATYYC | LQHYS----------- -----------YPRS | FGQGT KLEIK |

| ID | ID2 | Germline | | K_FR1 | K_CDR1 | K_FR2 | K_CDR2 | K_FR3 | K_CDR3 | K_FR4 |
|---|---|---|---|---|---|---|---|---|---|---|
| iPS:435 401 | 21-225_150E2 | VK1\|A30/ JK2 | | DIQMTQSPSSLSA<br>SVGDRVTISC | RAS--QGIG--<br>----NDLG | WYQQKPGK<br>APTRLIY | A--------<br>ASSLQS | GVPSRFSGSGSG--<br>TEFTLTISSLQPADFATYYC | LQHYS-----------<br>------------FPYS | FGQGT<br>KLEIK |
| iPS:435 445 | 21-225_152F7 | VK1\|A30/ JK2 | | DIQMTQSPSSLSA<br>SVGDRVTITC | RAS--QGIR--<br>----NDLG | WYQQKPGK<br>APKRLIY | A--------<br>TSSLQS | GVPSRFSGSGSG--<br>TEFTLTISSLQPEDFATYYC | LQHYN-----------<br>------------FPYS | FGQGT<br>KLEIK |
| iPS:435 763 | 21-225_176H1 2 | VK1\|A30/ JK2 | | DIQMTQSPSSLSA<br>SVGDRVTITC | RAS--QGIR--<br>----NDLG | WYQQKPGK<br>APNRLIY | A--------<br>ASSLQS | GVPSRFSGSGSG--<br>TEFTLTISSLQREDFATYYG | LQHYS-----------<br>------------YPRS | FGQGT<br>KLEIK |
| iPS:435 767 | 21-225_177B4 | VK1\|A30/ JK2 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHYS-----------<br>------------FPRS | FGQGT<br>KLEIK |
| iPS:392 974 | 21-225_26A11 | VK1\|A30/ JK2 | | DIQMTQSPISLSA<br>SVGDRVTITC | RAS--QAIR--<br>----NDLG | WYQQRPGK<br>APKRLIY | A--------<br>ASSLQS | GVPSRFSGSGSG--<br>TEFILTISSLQPEDFATYYC | LQHYN-----------<br>------------YPRS | FGQGT<br>KLEIR |
| iPS:393 046 | 21-225_25A12 | VK1\|A30/ JK2 | | DIQMTQSPSSLSA<br>SVGDRVTVTC | RAS--QAIR--<br>----DDLG | WYQQKPGK<br>APKRLIY | A--------<br>ASSLQS | GVPSRFSGSGSG--<br>TEFILTISSLQPEDFATYYG | LQHYN-----------<br>------------YPRS | FGQGT<br>KLEIK |
| | | Germline | | K_FR1 | K_CDR1 | K_FR2 | K_CDR2 | K_FR3 | K_CDR3 | K_FR4 |
| | VK1\|O12/J K4 | | 32132 | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFATYYC | QQSYS-----------<br>------------TPLT | FGGGT<br>KVEIK |
| iPS:426 118 | 21-225_7A10 | VK1\|O12/ JK4 | | DIQMTQSPSSLSA<br>SVGDRVTITC | RAS--QNIY--<br>----SYLN | WYQQKPGR<br>APKLVIY | S--------<br>TSSLQS | GVPSRFSGSGSG--<br>TDFSLTISNLQPEDFSTYYC | QQSYS-----------<br>------------PPLT | FGGGT<br>KVEIR |
| iPS:426 124 | 21-225_32D6 | VK1\|O12/ JK4 | | DIQMTQSPSSLST<br>SVGDRVTITC | RAS--QNII--<br>----SYLN | WYQQKPGK<br>APKLLMY | V--------<br>ASRLQS | GVPSRFSGSGSG--<br>TDFTLTISSLQAEDFATYYC | QQSYS-----------<br>------------TPYT | FGGGT<br>KVAIK |
| iPS:451 135 | 21-225_64A11 | VK1\|O12/ JK4 | | DIQMTQSPSSLSA<br>SVGDRVTITC | RAS--RSVS--<br>----RYLN | WYQQTLGK<br>ALKLLIS | V--------<br>ASRLQS | GVPSRFSGSGSG--<br>TDFTLTISSVQREDFATYFC | QQSDS-----------<br>------------FPLT | FGGGT<br>KVEIK |
| iPS:434 011 | 21-225_48B10 | VK1\|O12/ JK4 | | DIQMTQSPSSLSA<br>SVGDRVTITC | RAS--QSIR--<br>----KYLN | WYQKTPGK<br>APKLLIY | A--------<br>ASSLQS | GVPSRFSGSGSG--<br>TDFTLTISSVQPEDFATYYC | QQTYS-----------<br>------------NPLT | FGGGT<br>KVEFT |
| iPS:434 015 | 21-225_48F12 | VK1\|O12/ JK4 | | DIQMTQSPSSLSA<br>SVGDRVTITC | RAS--QSIR--<br>----KYLN | WYQKTPGK<br>APKLLIY | A--------<br>ASSLQS | GVPSRFSGSGSG--<br>TDFTLTISSLQPEDFANYYC | QQTYS-----------<br>------------NPLT | FGGGT<br>EVEIT |
| iPS:434 017 | 21-225_48G12 | VK1\|O12/ JK4 | | DIQMTQSPSSLSA<br>SVGDRVTITC | RAS--QSIR--<br>----KYLN | WYQKTPGK<br>APKLLIY | A--------<br>ASSLQS | GVPSRFSGSGSG--<br>TDFTLTISSVQPEDFANYYC | QQTYS-----------<br>------------NPLT | FGGGT<br>EVEIT |
| iPS:434 165 | 21-225_50F2 | VK1\|O12/ JK4 | | DIQMTQSPSSLSA<br>SVGDRITITC | RAS--QSIL--<br>----SYLN | WYQQKPGK<br>APKLLIY | V--------<br>ASSFQS | GVPSRFSGSGSG--<br>TDFTLTISSLQPDDFATYYC | QQSYS-----------<br>------------PPLT | FGGGT<br>KVEIK |
| iPS:434 191 | 21-225_56B6 | VK1\|O12/ JK4 | | DIQMTQSPSSLSV<br>SVGDRVTITC | RAS--QSIF--<br>----RYLN | WYQQKPGR<br>APKLLIF | A--------<br>ASSFQS | GVPSRFSGSGSG--<br>TDFTLTISSLQPEDFATYYC | QQTYS-----------<br>------------PPLT | FGGGT<br>KVEIK |
| iPS:434 247 | 21-225_62D2 | VK1\|O12/ JK4 | | DIQMTQSPSSLSA<br>SVGDRVTIIC | RAS--QSII--<br>----SYLN | WYQQKPGK<br>APKLLIY | A--------<br>TSSLQS | GVPSRFSGSGSG--<br>TDFTLTISSLQPEDFATYYC | QQTYS-----------<br>------------PPLT | FGGGT<br>KVEIK |
| iPS:434 335 | 21-225_63C10 | VK1\|O12/ JK4 | | DIQMTQSPSSLST<br>SVGDRVTITC | RAS--QSIF--<br>----SYLH | WYQQKPGK<br>APKLLIS | A--------<br>ASSLQS | GVPSRFSGSGSG--<br>TDFTLTISSLQPEDFATFYC | QQTYS-----------<br>------------PPLT | FGGGT<br>KVEIK |
| iPS:434 341 | 21-225_64F7 | VK1\|O12/ JK4 | | DIQMTQSPSSLSA<br>SVGDRVTITC | RAS--QNIK--<br>----KYLN | WYQQKPGK<br>APKFLIY | G--------<br>ASSLQS | GVPSRFSGSGSG--<br>TDFTLTISSLQPEDFAAYYC | QQSYN-----------<br>------------ISFT | FGGGT<br>KVELK |
| iPS:435 295 | 21-225_146H1 | VK1\|O12/ JK4 | | DIQMTQSPSSLSA<br>SVGDRVTITC | RAS--QSIS--<br>----DYLN | WYQQKPGK<br>APKVLIY | T--------<br>TSSLQS | GVPSRFSGSGSG--<br>TDFTLTISSLQPEDFATYYC | QQSY------------<br>------------STPT | FGGGT<br>KVEIK |
| iPS:435 307 | 21-225_146E9 | VK1\|O12/ JK4 | | DIQMTQSPSSLSA<br>SVGDRVTITC | RAS--QSIS--<br>----DYLN | WYQLKPGK<br>APKVLIY | T--------<br>TSSLQS | GVPSRFSGSGSG--<br>TDFTLTISSLQPEDFATYFC | QQSY------------<br>------------STPT | FGGGT<br>KVEIK |
| iPS:435 347 | 21-225_148C4 | VK1\|O12/ JK4 | | DIQMTQSPSSLSA<br>SVGDRVTITC | RAS--QSII--<br>----NYLN | WYQQKPGK<br>APKVLIY | T--------<br>ASSLQS | GVPSRFSGSGSG--<br>TDFTLTISSLQPEDFTTYYC | QQSY------------<br>------------STPT | FGGGT<br>KVEIE |
| iPS:435 355 | 21-225_148H9 | VK1\|O12/ JK4 | | DIQMTQSPSSLSA<br>SVGDRVTITC | RAS--QSIS--<br>----NYLN | WYQQKPGK<br>APKVLIY | I--------<br>ASSLQS | GVPSRFSGSGSG--<br>TDFTLTISSLQPEDFATYYC | QQSY------------<br>------------STPT | FGGGT<br>KVEIK |

| | | | | | | | | | | |
|---|---|---|---|---|---|---|---|---|---|---|
| iPS:435 371 | 21-225_149A3 | VK1|O12/ JK4 | | DIQMIQSPSSLSA SVGDRVTITC | RAS--QSIS-- ----SYLN | WYQQKPGK APKVMIY | ASSLQS | GVPSRFSGSGSG-- TDFTLTINSLQPEDFATYYC | QQSY------------ ------------SIPT | FGGGT KVEIK |
| iPS:435 415 | 21-225_150C1 1 | VK1|O12/ JK4 | | DIQMIQSPSSLSA SVGDRVTITC | RAS--QSIS-- ----SYLN | WYQQKPGK APKLLIY | ASSLQS | GVPSRFSGSGSG-- TDFTLTISSLQPEDFATYYC | QQSY------------ ------------SIYT | FGGGS KVEIK |
| iPS:435 419 | 21-225_150C1 2 | VK1|O12/ JK4 | | DIQMIQSPSSLSA SVGDRVTITC | RAS--QSIS-- ----DYLN | WYQQKPGK APKVLIY | TSSLQS | GVPSRFSGSGSG-- TDFTLTISSLQPEDFATYYC | QQSF------------ ------------STPT | FGGGT KVEIK |
| iPS:435 425 | 21-225_151B1 2 | VK1|O12/ JK4 | | DIQMIQSPSSLSA SVGDRVTITC | RAS--QSIS-- ----NFLN | WYQQKPGK APKVLIY | ASSLES | GVPSRFSGSESG-- TDFTLTISSLQPEDFATYYC | QQSY------------ ------------STPT | FGGGT RVEIK |
| iPS:435 431 | 21-225_152D2 | VK1|O12/ JK4 | | DIQMILSPSSLSA SVGDRVTITC | RAS--QSIS-- ----DYLN | WYQLKPGK APKVLIY | ASSLQS | GVPSRFSGSGSG-- TDFTLTISSLQPEDFATYFC | QQSY------------ ------------STPT | FGGGT KVEIK |
| iPS:435 439 | 21-225_152G4 | VK1|O12/ JK4 | | DIQMIQSPSSLSA SVGDRVTITC | RAS--QSIS-- ----DYLN | WYQQKPGK APKVLIY | TSSLQS | GVPSRFSGSGSG-- TDFTLTISSLQPEDFATYFC | QQSY------------ ------------STPT | FGGGT KVEIK |
| iPS:435 455 | 21-225_152B1 1 | VK1|O12/ JK4 | | DIQMIQSPSSLSA SVGDRVTITC | RAS--QSIS-- ----DYLN | WYQQKPGK APKVLIY | TSSLQS | GVPSRFSGSGSG-- TDFTLTISSLQPEDFATYFC | QQSY------------ ------------STPT | FGGGT KVEIK |
| iPS:435 487 | 21-225_155C4 | VK1|O12/ JK4 | | DIQMIQSPSSLSA SVGDRVTITC | RAS--QSIS-- ----SYLN | WYQQKPGK APKVLIF | ASSLQS | GVPSRFSGSGSG-- TDFTLTISSLQPEDFATYYC | QQSY------------ ------------STPT | FGGGT RVEIK |
| iPS:435 503 | 21-225_156E4 | VK1|O12/ JK4 | | DIQMIQSPSSLSA SVGDRVTITC | RAS--QSIS-- ----SYLN | WYQQKPGK APKVLIY | ASSLQS | GVPSRFSGSGSG-- TDFTLTISSLQPEDFATYYC | QQSY------------ ------------SAPT | FGGGT KVEIK |
| iPS:435 563 | 21-225_159H2 | VK1|O12/ JK4 | | DIQLIQSPSSLSA SVGDRVTITC | RAS--QSIS-- ----KYLN | WYQQKPGK APELLIY | TSNLQS | GVPSRFSGSGSG-- TDFTLTISTLQPEDFVIYYC | QQSYS----------- ------------LPVT | FGGGT KVEIK |
| iPS:436 110 | 21-225_196F4 | VK1|O12/ JK4 | | DFQMIQSPSSLSA SVGDRVTITC | RAS--QRIH-- ----SYLN | WYQQKPGK APKLLIY | ASSLQG | GVPSRFSGSGSG-- TDFTLTISSLQPEDFATYYC | QQSYG---------- ------------SPLT | FGGGT KVEIK |
| iPS:436 244 | 21-225_201H1 0 | VK1|O12/ JK4 | | DIQMIQSPSSLSA SVGDRVTITC | RAS--HNIN-- ----SYLN | WYQQKSGK APKLLIY | ASSLQS | GVPSRFSGSGSG-- TDFTLTFSSLQPEDFATYYC | QQSYS----------- ------------FPLT | FGGGT KVEMR |
| iPS:436 262 | 21-225_203E3 | VK1|O12/ JK4 | | DIQMIQSPSSPSA SVGDRVTITC | RAS--HNIN-- ----SYLN | WYQQKSGK APKLLIY | ASSLQS | GVPSRFSGSGSG-- TDFTLTFSSLQPEDFATYYC | QQSYS----------- ------------FPLT | FGGGT KVEMR |
| iPS:436 276 | 21-225_204H4 | VK1|O12/ JK4 | | DIQMILSPSSPSA FVGDRVTITR | RAS--HNIN-- ----SYLN | WYQQKSGK APKLLIY | ASSLQS | GVPSRFSGSGSG-- TDFTLTFSSLQPEDFATYYC | QQSYS----------- ------------FPLT | FGGGT KVEMR |
| iPS:436 280 | 21-225_204D6 | VK1|O12/ JK4 | | DSQMIQSPSSLSA SVGDRVTITC | RAS--RSVH-- ----TYLN | WYQQKPGK APKVLIY | ASSLQR | GVPSRFSGSGSG-- TDFTLTIISSLQPEDVAIYYC | QQSYS----------- ------------SPLT | FGGGT KVEIQ |
| iPS:436 312 | 21-225_206A4 | VK1|O12/ JK4 | | DIQMILSPSSPSA FVGDRVTITR | RAS--HNIN-- ----SYLN | WYQQKSGK APKLLIC | ASSLQS | GVPSRFSGSGSG-- TDFTLTFSSLQPEDFATYYC | QQSYS----------- ------------FPLT | FGGGT KVEMR |
| iPS:436 316 | 21-225_206A5 | VK1|O12/ JK4 | | DIQMIQSPSSPSA SVGDRVTITR | RAS--HNIN-- ----SYLN | WYQQKSGK APKLLIC | ASSLQS | GVPSRFSGSGSG-- TDFTLTFSSLQPEDFATYYC | QQSYS----------- ------------FPLT | FGGGT KVEMR |
| iPS:436 338 | 21-225_208E8 | VK1|O12/ JK4 | | DIQMIQSPSSPSA FVGDRVTITR | RAS--HNIN-- ----SYLN | WYQQKSGK APKLLIY | ASSLQS | GVPSRFSGSGSG-- TDFTLTFSSLQPEDFATYYC | QQSYS----------- ------------FPLT | FGGGT KVEMR |
| iPS:436 344 | 21-225_208B1 1 | VK1|O12/ JK4 | | DIQMIQSPSSPSA SVGDRVTITC | RAS--HNIN-- ----SYLN | WYQQKSGK APKLLIY | ASSLQS | GVPSRFSGSGSG-- TDFTLTFSSLQPEDFATYYC | QQSYS----------- ------------FPLT | FGGGT KVEMR |
| iPS:436 358 | 21-225_210D1 1 | VK1|O12/ JK4 | | DIQMTQSPSSLSA SVGDRVTITC | RAS--HNIN-- ----SYLN | WYQQKSGK APKLLIY | ASSLQS | GVPSRFSGSGSG-- TDFTLTFSSLQPEDFATYYC | QQSYS----------- ------------FPLT | FGGGT KVEMR |

| ID | Clone | Germline | | FR1/FR2 | CDR1 | FR2/FR3 | CDR2 | FR3 | CDR3 | FR4 |
|---|---|---|---|---|---|---|---|---|---|---|
| iPS:437 282 | 21-225_207C9 | VK1\|O12/ JK4 | | DNQMTQSPSSLSA SVGDRVTITC | RAS--QRFS-- ----NYLN | WYQQKPGK APKLLIY | T-------- ASSLQS | GVPSRFSASVSG-- TDFTLTIISSLQPEDFATYYC | QQSYS---------- -----------IPLT | FGGGT KVEIK |
| iPS:392 636 | 21-225_17A6 | VK1\|O12/ JK4 | | DIQMTQSPSSLSA SVGDRVTITC | RAS--QTIS-- ----NYLN | WYHQKPGK APKLLIY | A-------- ASSLQS | GVPSRFSGSGSG-- TDFTLTIISSLQPEDFATYYC | QQSHT---------- -----------SPLT | FGGGT KVEIK |
| iPS:392 648 | 21-225_16D11 | VK1\|O12/ JK4 | | DIQMTQSPSSLSA SVGDRVTITC | RAS--QTIS-- ----NYLN | WYHQKPGK APKLLIY | A-------- ASSLQS | GVPSRFSGSGSG-- TDFTLTIISSLQPEDFATYYC | QQSHS---------- -----------SPLT | FGGGT KVEIK |
| iPS:392 664 | 21-225_20F6 | VK1\|O12/ JK4 | | DIQMTQSPSSLSA SVGDRVTITC | RAS--QSII-- ----IYLN | WYQQKPGK APKVLIH | A-------- ASSLQS | GVPSRFSGSGSG-- TDFTLTIISSLQPEDFATYYC | QQTYS---------- -----------PPLT | FGGGT KVEIK |
| iPS:392 738 | 21-225_18G4 | VK1\|O12/ JK4 | | DIHMTQSPSSLSA SVGDRVTITC | RAS--QSII-- ----SYLN | WYQQKPGK APKVLIY | T-------- ASSLQT | GVPSGFSGSGSG-- IDFTLTIISSLQPEDFATYYC | QQTYS---------- -----------PPLT | FGGGT KVEIK |
| iPS:392 798 | 21-225_22C7 | VK1\|O12/ JK4 | | DIQMTQSPSSLSA SVGDRVTITC | RAS--QNII-- ----SYLN | WYQQKPEK APKLLIH | I-------- ASSLQS | CVPSRFSGSGSG-- IDFTLTISSLQPEDFATYYC | QQTYS---------- -----------TPLT | FGGGT KVEIK |
| iPS:392 922 | 21-225_30G4 | VK1\|O12/ JK4 | | DIQMTQSPPSLST SVGDRVTITC | RAT--QNIF-- ----SYLN | WHQQKPGK APKLLIH | T-------- ASSLQG | GVPSRFSGSGSG-- IDFTLTIISMQPEDFSTYYC | QLSYS---------- -----------PPYT | FGGGT KVEIK |
| iPS:393 002 | 21-225_30G1 | VK1\|O12/ JK4 | | DIQMTQSPSSLSA SVGDRVTITC | RAS--QNIY-- ----SYLN | WYQQKPGK DPKLLIY | A-------- ASSLHS | GVPSRFSGSGSG-- TDFTLTIISSLQPEDFATYYC | QQSYS---------- -----------TPLT | FGGGT KVEIK |
| iPS:393 042 | 21-225_31F1 | VK1\|O12/ JK4 | | DIQMTQSPSSLSA SVGDRVTITC | RAS--QRIS-- ----SYLN | WYQQKPGK APKLLIF | A-------- ASSSQS | GVPSRFSGSGSG-- TDFTLTIISSLQPEDFATYYC | QQSYI---------- -----------TPLT | FGGGT IVEIR |
| iPS:393 066 | 21-225_34D3 | VK1\|O12/ JK4 | | DIQMTQSPSSLSA SVGDRVTITC | RAS--QNIY-- ----SYLN | WYQQKPGK DPKLLIY | A-------- ASSLHS | GVPSRFSGSGSG-- TDFTLTIISSLQPEDFATFYC | QQSYS---------- -----------TPLT | FGGGT KVEIK |
| iPS:393 082 | 21-225_34C11 | VK1\|O12/ JK4 | | DIQMTQFPSSLST SVGDRVTSTC | RAS--QNIR-- ----NFLN | WYQQKPEK DPKLQIY | G-------- ASTLQS | GVPSRFSGSGSG-- TDFTLTIISSLQPKDFATYYC | QQTCS---------- -----------TPLT | FGGGT KVEIK |
| iPS:393 092 | 21-225_33C12 | VK1\|O12/ JK4 | | DIQMTQSPSSLST SVGDRVTITC | RAS--QSII-- ----SYLN | WYQQKPGK APKLLIY | V-------- ASSLQG | GVPSRFNGSGSG-- TDFTLTIISSLQPEDFATYYC | QQSYS---------- -----------TPYT | FGGGT KVEIK |
| iPS:393 100 | 21-225_36B8 | VK1\|O12/ JK4 | | DIQMTQSPSSLST SVGDRVTITC | RAS--QSII-- ----SYLN | WYQQKPGK APKLLIY | V-------- ASSLQS | GVPSRFSGSGSG-- TDFTLTIISSLQPEDFATYYC | QQSYS---------- -----------TPYT | FGGGT KMEIK |
| iPS:393 108 | 21-225_34G11 | VK1\|O12/ JK4 | | DIQMTQSPSSLSA SVGDRVTITC | RAS--QNIN-- ----RYLN | WYQQKPGK APKLLIY | G-------- ASSLQS | GVPSRFSGSGSG-- TDFTLTISTLQPEDFATYYC | QQTYI---------- -----------TPLT | FGGGT KVEIK |
| iPS:393 122 | 21-225_33B2 | VK1\|O12/ JK4 | | DIQMTQSPSSLST SVGDKVTITC | RAS--QSII-- ----SYLN | WYQQKPGK APKLLIY | V-------- ASSLQS | GVPSRFSGSGSG-- TDFTLTIISSLQPEDFATYYC | QQSYS---------- -----------TPYT | FGGGT KVEIK |
| iPS:393 134 | 21-225_34C2 | VK1\|O12/ JK4 | | DIQMTQSPSSLST FVGDRVTITC | RAS--QRII-- ----SYLN | WTQQKPGK APKLLIY | V-------- ASSLQS | GVPSRFSGSGSG-- TYFTLTIISSLQPEDFATYYC | QQSYS---------- -----------TPYT | FGGGT KMEIK |
| iPS:393 136 | 21-225_34D8 | VK1\|O12/ JK4 | | DIQMIQSPSSLST SVGDRVTITC | RAS--QIII-- ----SYLN | WYQQKPGK APKLLIF | V-------- ASSLQS | GVPSRFSGSGSG-- TDFTLTIISSLQPEDFATYYC | QQSYS---------- -----------TPYT | FGGGT KVEIK |
| iPS:393 840 | 21-225_3F8 | VK1\|O12/ JK4 | | DFQMTQSPSSLSA SVGDRVTITC | RAS--QSIL-- ----SYLN | WYRQKPGR APQVLIH | T-------- TSSLQS | GVPSRFSGSGSG-- IVFTLTISSLQPEDFATYYC | QQIYS---------- -----------TPLT | FGGGT RVEIN |
| iPS:393 844 | 21-225_3G7 | VK1\|O12/ JK4 | | DIQMTQSPSSLSA SVGDRVTVTC | RAS--QNIY-- ----RYLN | WYQGRPGR APKLMIY | A-------- ASSSQS | GVPSRFSGSGSG-- TDFTVTISSLQPEDFATYYC | QQSYS---------- -----------PPFT | FGGGA KVEID |
| iPS:393 852 | 21-225_12A10 | VK1\|O12/ JK4 | | DVQMTQSPSSLSA SAGDRVTITC | RAS--QNIY-- ----SYLN | WYQQKPGR APKVLIH | T-------- ASSLQS | GVPSRFSGSGSG-- ADFTLTINSLQPEDFATYYC | QQSYS---------- -----------PPLT | FGGGT KVEIK |
| iPS:393 900 | 21-225_10E12 | VK1\|O12/ JK4 | | DIQMTQSPSSLSA SVGDRVTITC | RAS--QNIY-- ----SYLN | WYQEKPGR APKLLIY | A-------- TSSLQS | GVPSRFSGSGSG-- TDFTLTIISSLQPEDFATYYC | QQNYS---------- -----------PPLT | FGGGT KVEIE |
| iPS:393 920 | 21-225_1H12 | VK1\|O12/ JK4 | | DIQMTQSPSSLSA SVGDRVTITC | RAS--QNIY-- ----RYLN | WYQEKPGR APKLLIY | T-------- ASSLQS | GVPSRFSGSDSG-- TDFTLTIISSLQPEDFATYYC | QQSYS---------- -----------PPLT | FGGGT KVEIK |
| iPS:393 926 | 21-225_4G4 | VK1\|O12/ JK4 | | DIQMTQSPSSLAA SVGDRVTITC | RAS--QTII-- ----SYLN | WYQQKPGK APKLLIH | T-------- ASSLQS | GVPSRFSGSGSG-- TDFTLSISSLQPEDFATYYC | QQTYS---------- -----------TPLT | FGGGT KVEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:393 930 | 21-225_7E11 | VK1|O12/JK4 | | DIQMTQSPSSLSA SVGDRVTIAC | RAS--QNII-- ----SYLN | WYQQKPGK APKFLIY | T-------- ASSLQS | GVPSRFSGSGSG-- TDFTLTISSLQPEDFAIYYC | QQTYS----------- ------------TPLT | FGGGT KVEIK |
| iPS:393 932 | 21-225_10F5 | VK1|O12/JK4 | | DIQMTQSPSSLSA SVGDRVTITC | RAS--QNIY-- ----RYLN | WYQFKPGR APKLLIY | T-------- ASSLQS | GVPSRFSGSDSG-- TDFTLTISSLQPEDFATYYC | QQSYS----------- ------------PPLT | FGGGT KVEIK |
| iPS:393 964 | 21-225_6G1 | VK1|O12/JK4 | | DIQMTQSPSSLSA SVGDRVTITC | RTS--QNII-- ----SYLN | WYQQKPGK APKVLIY | T-------- ASNLQT | GVPSGFSGSGSG-- TDFTLTISSLQPEDFATYYC | QQPHS----------- ------------PPLT | FGGGT KVEIK |
| iPS:394 012 | 21-225_15A3 | VK1|O12/JK4 | | DIQMTQSPSSLSA SVGDRVTITC | RAS--QSII-- ----SYLN | WYLQKPGK APKFLIY | T-------- ASSLQS | GVPSRFSGSGSG-- TDFTLTISSLQPEDFATYYC | QQTYS----------- ------------TPLT | FGGGT KVEIK |
| iPS:394 016 | 21-225_13D4 | VK1|O12/JK4 | | DLQMTQSPSSLSA SVGDRVTITC | RAS--QSIF-- ----SYLN | WYQQKPGK APKLLIC | T-------- ASSLQN | GVPSRFSGSGSG-- TDFTLTISSLQPEDFATYYC | QQTYS----------- ------------LPLT | FGGGT KVEIK |
| iPS:394 083 | 21-225_16E6 | VK1|O12/JK4 | | DIQMTQSPSSLSA SVGDRVTITC | RAS--QSII-- ----SYLN | WYQQKPGK APKFLIY | T-------- TSSLQS | GVPSRFSGSGSG-- TDFTLTISSLQPEDFATYYC | QQTYS----------- ------------TPLT | FGGGT KVEIK |
| iPS:398 480 | 21-225_17G4 | VK1|O12/JK4 | | DIQMTQSPSSLSA SAGDRVTITC | RTS--QNIS-- ----NYLN | WYQQKPGK APKLLIY | V-------- ASSFPS | GVPSRFSGSGSG-- TEFTLTISSLQPEDFATYYC | QQSNF----------- ------------FPLT | FGGGT KVEII |
| iPS:398 486 | 21-225_19A1 | VK1|O12/JK4 | | DIQMTQSPSSLSA SVGDRVTITC | RAS--HTII-- ----SYLN | WYQQKPGK APKFLIY | A-------- TSNLQS | GVPSRFSGSGSG-- TDFTFTISSLQPEDFAIYYC | QQSYN----------- ------------FPLT | FGGGT KVEIK |
| | VK2|A18/JK4 | Germline | | K_FR1 | K_CDR1 | K_FR2 | K_CDR2 | K_FR3 | K_CDR3 | K_FR4 |
| | VK2|A18/JK4 | | 32133 | DIVMTQTPLSLSV TPGQPASISC | KSS QSLLHSD-GKTYLY | WYLQKPGQ SPQLLIY | E-------- VSSRFS | GVPDRFSGSGSG-- TDFTLKISRVEAEDVGVYYC | MQGIH----------- ------------LPLT | FGGGT KVEIK |
| iPS:451 139 | 21-225_71A6 | VK2|A18/JK4 | | DIVMTQTPLSLSV TPGQPASISC | KSS QSLLRSD-GKTHLY | WYLQKPGQ PPQLLIY | E-------- VSNRFS | GVSDRFSGSGSG-- TDFTLKISRVEAEDVGVYYC | MQSKQ----------- ------------LPLT | FGGGT KVEFK |
| iPS:433 937 | 21-225_44B10 | VK2|A18/JK4 | | EIVMTQTPLSLSV TPGQPASISC | KSS QSLLHSE-GRTYLY | WYLQKPGQ PPQLLIY | E-------- ISHRFS | GVPDRFSGSGSG-- TDFTLKISRVEAEDVGVYYC | MQSIH----------- ------------LPFT | FGGGT KVEIK |
| iPS:433 979 | 21-225_46B9 | VK2|A18/JK4 | | DIVMTQTPLSLSV TPGQPASISC | KSS-- QSLLHSE-GKTYLY | WYLQKPGQ PPQLLIY | E-------- VSYRFS | GVPDRFSGSGSG-- TDFTLKISRMEAEDVGVYYC | MHSIQ----------- ------------YPLT | FGGGT KVEIQ |
| iPS:434 201 | 21-225_59A12 | VK2|A18/JK4 | | DIVMTQTPLSLSV TPGQPASISC | KSS-- QSLQHGE-GKTYLY | WYVQKPGQ PPQLLIY | E-------- VSYRFS | GVPDRFSGSGSG-- TDFTLKISRVEVEDVGVYYC | MQSTQ----------- ------------LPLT | FGGGT KVEIK |
| iPS:434 205 | 21-225_60G2 | VK2|A18/JK4 | | DIVMTQTPLSLSV TPGQSASISC | KSS-- QSLLHSE-GKTYLY | WYLQKPGQ PPQLLIY | E-------- VSNRIS | GVPDRFSGSGSG-- TDFTLKISRVEAEDVGVYYC | MQSIQ----------- ------------LPLT | FGGGT KVEIK |
| iPS:434 223 | 21-225_60C12 | VK2|A18/JK4 | | DIVMTQTPLSLSV TPGQPASISC | KSS-- QSLLHSE-GKTYLY | WYLQKPGQ PPQFLIY | E-------- VSNRFS | GVPDRFSGSGSG-- TDFTLKISRVEAEDVGIYYC | MQSIK----------- ------------YPLT | FGGGT KVEIK |
| iPS:434 233 | 21-225_61B3 | VK2|A18/JK4 | | DIVMTQTPLSLSV TPGQPASISC | KSS-- QSLLHSE-GKTYLY | WYLQKPGQ PPQLLIY | E-------- VSNRIS | GVPDRFSGSGSG-- TDFTLKISRVEAEDVGVYYC | MQSIQ----------- ------------LPLT | FGGGT KVEIK |
| iPS:434 303 | 21-225_58H11 | VK2|A18/JK4 | | DIVMTQTPLSLSV TPGQPASISC | KSS-- QSLLHSE-GKTYLY | WYVQKPGQ PPQLLIY | E-------- VSYRFS | GVPDRFSGSGSG-- TDFTLKISRVEAEDVGVYYC | MQSTQ----------- ------------LPLT | FGGGT KVEIK |
| iPS:435 349 | 21-225_148F5 | VK2|A18/JK4 | | DIVMTQTPLSLSV TPGQPASISC | KSS-- QSLLHSE-GKTYLY | WYLQKPGQ PPQLLIY | E-------- VSYRVS | GVPDRFSGSGSG-- TDFTLKISRVEAEDVGVYFC | MQSIQ----------- ------------LPLT | FGGGT KVEIK |

| ID | Clone | Germline | K_FR1 | K_CDR1 | K_FR2 | K_CDR2 | K_FR3 | K_CDR3 | K_FR4 |
|---|---|---|---|---|---|---|---|---|---|
| iPS:435 359 | 21-225_148H10 | VK2\|A18/JK4 | DIVMTQTPLSLSV TPGQPASISC | KSS-- QSLLHSE- GKTYLY | WYLQKPGQ PPQLLIY | E-------- VSYRVS | GVPDRFSGSGSG-- TDFTLKISRVEAEDVGVYYC | MQSIQ----------- -----------LPLT | FGGGT KVEIK |
| iPS:435 417 | 21-225_150D11 | VK2\|A18/JK4 | DIVMTQTPLSLSV TPGQPASISC | KSS-- QSLLHSE- GKTYLY | WYLQKPGQ PPQLLIY | E-------- VSYRFS | GVPDRFSGSGSG-- TDFTLKISRVEAEDVGVYYC | MQGIQ----------- -----------LPLT | FGGGT KVEIK |
| iPS:435 469 | 21-225_153G9 | VK2\|A18/JK4 | DIVMTQTPFSLSV TPGQPASISC | KSS-- QSLLHSD- GKTYLY | WYLQKPGQ PPQFLIY | E-------- VSNRFS | GVPDRFSGSGSG-- TDFTLKISRVEADDVGVYYC | MQNIK----------- -----------YPLT | FGGGT KVEIK |
| iPS:435 733 | 21-225_173C11 | VK2\|A18/JK4 | DIVMTQTPLSLSV TPGQPASISC | KSS-- QSLLHSE- GKTYLY | WYLQKPGQ PPQLLIY | E-------- VSHRFS | GVPDRFSGSGSG-- TDFTLKISRVEAEDVGVYYC | MQSI----------- -----------QLLT | FGGGT KVEIK |
| iPS:435 785 | 21-225_179C2 | VK2\|A18/JK4 | DIVMTQTPLSLSV TPGQPASISC | KSS-- QSLLHSE- GKTYLY | WYLQKPGQ PPQLLIY | E-------- VSHRFS | GVPDRFSGSGSG-- TDFTLKISRVEAEDVGVYYC | MQSI----------- -----------QVLT | FGGGT KVEIK |
| iPS:392 618 | 21-225_16F10 | VK2\|A18/JK4 | DIVMTQTPLSLSV IPGQPASISC | KSS-- QSLLHSD- GKTHLN | WYLQKPGQ PPQLLIY | E-------- VSYRFS | GVPDRFSGSGSG-- TVFTLEISRVEAADVGVYYC | FQSIQ----------- -----------LPLT | FGGGT KVEIK |
| iPS:392 860 | 21-225_22H8 | VK2\|A18/JK4 | DIVMTQTPLSLSV TPGQPASISC | KSS-- QSLLHSD- GKTFLY | WYLQKPGH PPQLLIY | E-------- VSNRFS | GVPDRFSGSGSG-- TDFTLKISRVEAEDVGIYYC | MQSIQ----------- -----------LPLS | FGGGT KVEIN |
| iPS:392 888 | 21-225_25A2 | VK2\|A18/JK4 | DIVMNQTPLSLSV TPGQPASISC | KSS-- QSLLHSE- GKTYLY | WYLQKPGQ PPQLLIY | I-------- ISNRFS | GVPARLSGSGSG-- TDFTLKISRVEAEDVGVYYC | MQSIQ----------- -----------FPLT | FGGGT KVEIK |
| iPS:392 938 | 21-225_29H4 | VK2\|A18/JK4 | DIVMTQTPLSLSV TPGQPASFSC | KSS-- QSLLHSD- GKTYLY | WYLQKPGQ PPQLLIF | E-------- VSHRFS | GLPDRFSGSGSG-- TDFTLKISRVEAEDVGVYYC | MQSIQ----------- -----------HPFT | FGGGT RVEIK |
| iPS:392 994 | 21-225_26G11 | VK2\|A18/JK4 | DIVMTQTPLSLSV TPGQPASISC | KSS-- QTLLHGE- GKTYLY | WYLQKPGQ PPHLLIY | E-------- VSNRFS | GVPDRFSGSGSG-- TDFTLKISRVEAEDVGIYYC | MQSIK----------- -----------LPLT | FGGGT KVEIK |
| iPS:393 012 | 21-225_26G7 | VK2\|A18/JK4 | DILMTQTPLSLSV TPGQPASISC | KSS-- QSLLHSE- GKTYLY | WYLQKPGQ PPQFLIY | E-------- VSHRLS | GVPDRFSGSGSG-- TDFTLKISRVEAEDVGVYYC | MQSIQ----------- -----------LPLT | FGGGT KVEIK |
| iPS:393 144 | 21-225_34D2 | VK2\|A18/JK4 | DIVMTQTPLSLSV TPGQPASISC | KSS-- QSLLHSD- GKTYLY | WYLQKPGQ PPQLLIY | E-------- VSNRFS | GVPDRFSGSGSG-- TDFTLKISRVEAEDVGVYYC | MQSK----------- -----------QLPP | FGGGT KVEIR |
| | Germline | | K_FR1 | K_CDR1 | K_FR2 | K_CDR2 | K_FR3 | K_CDR3 | K_FR4 |
| VK1\|L1/JK3 | 32134 | | DIQMTQSPSSLSA SVGDRVTITC | RAS--QGIS-- ----NYLA | WFQQKPGK APKSLIY | A-------- ASSLQS | GVPSRFSGSGSG-- TDFTLTISSLQPEDFATYYC | QQYNS----------- -----------YPFT | FGPGT KVDIK |
| iPS:451 143 | 21-225_66H11 | VK1\|L1/JK3 | DIQMTQFPSSLFA FVGDRVTITC | PAS--QGIS-- ----NYLA | WFQQKPGK APKSLIY | G-------- AFNLHS | GVPSKFSGSGFG-- TDFTLTINSLQPEDFANYYC | QQYSC----------- -----------YPFT | FGHGT KVDIK |
| iPS:468 814 | 21-225_223D11 | VK1\|L1/JK3 | DIQMTQSPSSLSA SVGDRVTITC | RAS--QGIS-- ----NYLA | WFQQKPGK APKSLIY | A-------- ASTLQS | GVPSKFSGSRSG-- TDFNLIISNLQPEDFATYYC | QQYSG----------- -----------YPFT | FGPGT KVDTK |
| iPS:433 901 | 21-225_43A4 | VK1\|L1/JK3 | DIQMTQSPSSLSA SVGDRVTITC | RAS--QGIN-- ----NYLA | WFQQKPGK APKSLIN | A-------- ASSLQS | GVPSRFSGSGSG-- TDFTLAISSLQPEDFATYYC | QQYYS----------- -----------YPFT | FGPGT KVDIK |
| iPS:433 961 | 21-225_45D9 | VK1\|L1/JK3 | DIQMTQSPSSLSA SVGDRVTITC | RAS--QGIN-- ----NYLA | WFQQKPGK APKSLIN | A-------- ASSLQS | GVPSRFSGSGSG-- TDFTLAISSLQPEDFATYYC | QHYYS----------- -----------YPFT | FGRGT KVDIK |

| iPS ID | 21-225 | Germline | FR1 | CDR1 | FR2 | CDR2 | FR3 | CDR3 | FR4 |
|---|---|---|---|---|---|---|---|---|---|
| iPS:434 059 | 21-225_51C5 | VK1\|L1\|JK3 | DIQMTQSPSSLSA SVGDRVTITC | RAS--QGIS-- ----NYLA | WFQQKPGE APKSLIY | A-------- ASSLRS | GVPSQFSGSGSG-- TDFILTISSLQPEDFATYYC | QQYYS----------- ------------YPFT | FGPGT KVDIK |
| IPS:434 085 | 21-225_52E3 | VK1\|L1\|JK3 | DIQMTQSPSSLSA SVGDRVTITC | RAS--QGIS-- ----NYLA | WFQQKPGK APKSLIN | A-------- ASSLQS | GVPSKFSGSGSG-- TDFTLTISSLQPEDFATYYC | QQYNS----------- ------------FPFT | FGPGT KVDIK |
| iPS:434 115 | 21-225_53E4 | VK1\|L1\|JK3 | DIQMTQSPSSLSA SVGDRVTITC | RAS--QVIS-- ----NYLA | WFQQKPGK APKSLIS | A-------- ASSLQS | GVPSKFSGSGSG-- TDFTLTISSLQPEDFATYYC | QQYHS----------- ------------YPLT | FCRGT KVDIK |
| iPS:434 213 | 21-225_60A4 | VK1\|L1\|JK3 | DIQMTQSPSSLSA SVGDRVTITC | RAS--QGIS-- ----NYLA | WFQQKPGK APKSLIY | A-------- ASSLQS | GVPSKFSGSGSG-- TDFTLTISSLQSEDFATYYC | QQYKS----------- ------------HPFT | FGPGT KVDIK |
| iPS:434 215 | 21-225_60F7 | VK1\|L1\|JK3 | DIQMTQSPSSLSA SVGDRVTISC | RAS--QVIK-- ----NYLV | WVQQKPGK APKSLIY | A-------- ASSLQS | GVPSTFSGSGSG-- TDFTLTISSLQPEDFATYYC | LQFHS----------- ------------YPFT | FGPGT KMDIK |
| iPS:434 261 | 21-225_56F7 | VK1\|L1\|JK3 | DILMIQSPSSLSA SVGDRVTITC | RAS--QGIS-- ----TYLA | WFQQTPGT APKSLIY | A-------- ASSLQG | GVPSKFSGSGSG-- TDFTLTISSLQPEDFATYYC | HQYNS----------- ------------FPFK | FGRGT KVDIT |
| iPS:434 331 | 21-225_63H8 | VK1\|L1\|JK3 | DIQMTQSPSSLSA SVGDRVTITC | RAS--QGIS-- ----NYLA | WFQQKPGK AHKSLIY | A-------- ASSLQS | GVPSKFSGSGSG-- TDFILTISSLQPEDFATYYC | QQYHS----------- ------------YPFT | FGPGT KVDIR |
| iPS:434 361 | 21-225_65D5 | VK1\|L1\|JK3 | DIQMTQSPSSLSA SVGDRVTITC | RAS--QDIN-- ----NYLA | WFQQKPGK APRSLIY | A-------- ASSLHS | GVPSQFSASGSG-- SDFILTISSLQPEDFATYYC | PLYKS----------- ------------YPLT | FGPGT KVDIK |
| iPS:434 405 | 21-225_68E6 | VK1\|L1\|JK3 | DIQMTQSPSSLSA SVGDRVTITC | RAS--QGIS-- ----YYLA | WFQQKPGR APKSLIY | V-------- ASSLQS | GVPSRFSGSGSG-- TDFTLTISSLQPEDFATYYC | QQYDS----------- ------------YPFT | FGPGT KVDIR |
| iPS:434 259 | 21-225_86C6 | VK1\|L1\|JK3 | DIQMTQSPSSLSA SVGDRVTITC | RAS--QGIS-- ----NYLA | WFQQKPGK APKSLIY | A-------- ASSLQS | GVPSKFSGSGSG-- TDFTLTINSLQPEDFATYYC | HQYND----------- ------------YPFT | FGPGT KVDIK |
| iPS:435 351 | 21-225_148B6 | VK1\|L1\|JK3 | DIQMTQSPSSLSA SVGDRVSITC | RAS--QGIS-- ----KYLA | WFQQKPGK APKSLIF | A-------- ASSLQS | GVPSKFSGSGSG-- TDFTLTISSLQPEDFATYYC | QQYNS----------- ------------FPFT | FGPGT KVDIK |
| iPS:435 461 | 21-225_153A1 | VK1\|L1\|JK3 | DIQMTQSPSSLSA SVGDRVTITC | RAS--QVIS-- ----NYLA | WFQQKPGK APKSLIS | A-------- ASSLRS | GVPSNFSGSGSG-- TDFTLTISSLQPEDFATYYC | QQYHS----------- ------------YPFT | FGPGT KVDFK |
| iPS:435 509 | 21-225_157H1 | VK1\|L1\|JK3 | DIQMTQSPSSLSA SVGDRVTITC | RAS--QDIS-- ----NYLV | WFQQRPGK APRSLIY | A-------- ASSLQS | GVPSKFSGSGSG-- TDFTLTISSLQPEDFATYYC | QQYHS----------- ------------YPFT | FGPGT KVDIK |
| iPS:435 515 | 21-225_157E4 | VK1\|L1\|JK3 | DIQMTQSPSSLSA SVGDRVTFTC | RAS--QGIS-- ----NYLA | WFQQKPGR APKSLIY | A-------- ASSLRS | GVPSQFSGSGSG-- TDFTLTINSLQPEDFATYYC | QQYHS----------- ------------YPFT | FGPGT KVDIK |
| iPS:435 523 | 21-225_157G5 | VK1\|L1\|JK3 | DIQMTQSPSSLSA SVGDRVTITC | RAS--QGIN-- ----NYLA | WFQQKPGK APESLIY | A-------- ASSLQS | GVPSQFSGSGSG-- TDFTLTISSLQPEDFATYYC | QQYNS----------- ------------YPFT | FGPGT KVDIK |
| iPS:435 535 | 21-225_157H10 | VK1\|L1\|JK3 | DIQMTQSPSSLSA SVGDRVTITC | RAS--QGIT-- ----NYLA | WFQQKPGK APKSLIY | T-------- ASNLQS | GVPSKFSGSGSG-- TDFTLIISSLQPEDFATYYC | QQYHS----------- ------------YPFT | FGPGT KVDIK |
| IPS:435 559 | 21-225_158H12 | VK1\|L1\|JK3 | DIQMTQSPSSLSA SVGDRVTITC | RAS--QGIN-- ----NYLA | WFQQKPGK APKSLIS | A-------- ASSLQS | GVPSKFSGSGSG-- TDFTLSISSLQPEDFATYYC | QQYHS----------- ------------YPFT | FGPGT KVDIK |
| iPS:435 575 | 21-225_159H11 | VK1\|L1\|JK3 | DIQMTQSPSSLSA SVGDRVTITC | RAS--QGIS-- ----KYLV | WFQQKPGK APKSLIY | A-------- ASSLQS | GVPSKFSGSGSG-- TDFTLTISNLQPEDFATYYC | QQYNS----------- ------------YPFT | FGPGT KVDIK |
| iPS:435 579 | 21-225_160G1 | VK1\|L1\|JK3 | DIQMTQSPSSLSA SVGDRVTITC | RAS--QDIN-- ----NYLA | WFQQKPGK APTSLIY | A-------- SSSLQS | GVPSKFSGSGSG-- TDFTLTISSLQPEDFATYYC | QQYHS----------- ------------YPFT | FGPGT KVDIR |
| iPS:435 585 | 21-225_160G3 | VK1\|L1\|JK3 | DIQMTQSPSSLSA SVGDRVTITC | RAS--QDIN-- ----NYLA | WFQQRPGK APTSLIY | A-------- SSSLQS | GVPSKFSGSGSG-- TDFTLTISSLQPEDFATYYC | QQYHS----------- ------------YPFT | FGPGT KVDIK |
| iPS:435 635 | 21-225_163F1 | VK1\|L1\|JK3 | DIQMTQSPSSLSA SVGDRVTITC | RAS--QDIS-- ----NYLA | WFQQKPGK APKSLIY | A-------- ASSLQS | GVPSKFSGSGSG-- TDFTLTISSLQPEDFAAYYC | QQYNS----------- ------------YPFT | FGPGT QVDAQ |

| | | | | | | | | | |
|---|---|---|---|---|---|---|---|---|---|
| iPS:435 659 | 21-225_167D1 2 | VK1\|L1/J K3 | DIQMTQSPSSLSA SVGDRVTITC | RAS--QGIN-- ----NYLA | WFQQKPGK APESLIY | A-------- ASSLQS | GVPSKFSGSGSG-- TDFTLTISSLQPEDFATYFC | QQYNS----------- -----------YPFT | FGPGT KVDIK |
| iPS:435 679 | 21-225_169D1 0 | VK1\|L1/J K3 | DIQMTQSPSSLSA SVGDRVTITC | RAS--QDIS-- ----NYLA | WFQQKPGK APKSLIY | A-------- ASSLQS | GVPSKFSGSGSG-- TDFTLTISSLQPEDFATYYC | QQYHS----------- -----------YPFT | FGPGT KVDIK |
| iPS:435 685 | 21-225_170E1 | VK1\|L1/J K3 | DIQMTQSPSSLSA SEGDRVTITC | RAS--QGIS-- ----NYLA | WFQQKPGK APKSLIY | A-------- ASSLQS | GVPSKFSGSGSG-- TDFTLTISSLQPEDFATYYC | QQYHS----------- -----------YPFT | FGPGT KVDIK |
| iPS:435 747 | 21-225_175C4 | VK1\|L1/J K3 | DIQMTQSPSSLSA SVGDRVTITC | RAS--QGIG-- ----NYLA | WFQQKPGK APKSLIY | A-------- ASGLQS | GVPSKFSGSGSG-- TDFTLTISSLQPEDFATYYC | QQYYS----------- -----------YPFT | FGPGT KVDIK |
| iPS:435 765 | 21-225_177D3 | VK1\|L1/J K3 | DIQMSQSPSSLSA SVGDRVTITC | RAS--QGIT-- ----NYLA | WFQQKPGK APKSLIY | A-------- ASSLQS | GVPSKFSGSGSG-- TDFTLTISSLQPEDFATYYC | QQYNS----------- -----------YPFT | FGPGT KVDIK |
| iPS:435 797 | 21-225_181G2 | VK1\|L1/J K3 | DIQMTQSPSSLSA SIGERVTITC | RAS--QGIS-- ----NYLA | WIQQKPGT APKSLIY | A-------- ASSLQS | GVSSRFSGSGFG-- TDFTLTISSLQPEDFATYYC | QQYNG----------- -----------YPFT | FGPGT KVDIK |
| iPS:435 835 | 21-225_190F12 | VK1\|L1/J K3 | DIQMTQSPSSLSA SVGDRVTITC | RAS--QGIG-- ----KYLA | WFQQKPGK APKSLIY | A-------- ASSLQS | GVPSKFSGSGSG-- TDFTLTISSLQPDDFATYYC | QRYDT----------- -----------YPFT | FGPGT KVDIK |
| iPS:435 861 | 21-225_190A5 | VK1\|L1/J K3 | DIQMTQSPSSLSA SVGDRVTITC | RAS--QGIG-- ----NHLA | WFQQKPGK APKSLIH | A-------- ASSLQS | GVPSKFSGSGSG-- TDFTLTISSLQPEDFATYYC | QQYSN----------- -----------YPVT | FGPGT KVDIK |
| iPS:435 869 | 21-225_190B1 | VK1\|L1/J K3 | DIQMTQSPSSLSA SVGDRVTITC | RAS--QGIR-- ----NYLA | WFQQKPGK APKSLIY | V-------- ASSLES | GVPSKFSGSGSG-- TEFTLTISSLQPEDFGTYYC | QQYLN----------- -----------YPVT | FGPGT KVDIR |
| iPS:435 877 | 21-225_184E7 | VK1\|L1/J K3 | DIQMTQSPSSRSA SIGERVTITC | RAS--QGIS-- ----NYLA | WIQQKPGT APKSLIY | A-------- ASSLQS | GVSSRFSGSGFG-- TDFTITISSVQREDFATYYC | QQYNG----------- -----------YPFT | FGHGT KVDIK |
| iPS:435 883 | 21-225_185A1 | VK1\|L1/J K3 | DIQMTQSPSSLSA SVGDRVTITC | RAS--QGIS-- ----NYLA | WFQQTPGK APKSLIS | V-------- ASSLQS | GVPSRFSASGSG-- TDFTLTISSLQPEDFATYYC | RQYHS----------- -----------YPFT | FGPGT KVDIK |
| iPS:435 885 | 21-225_185E1 0 | VK1\|L1/J K3 | DIQMTQSPSSRSA SIGERVTITC | RAS--QGIS-- ----NYLA | WIQQKPGT APKSLIY | A-------- ASSLQS | GVSSRFSGSGFG-- TDFTLTISSLQPEDFATYYC | QQYNG----------- -----------YPFT | FGPGT KVDIK |
| iPS:435 891 | 21-225_188H5 | VK1\|L1/J K3 | DIQMTQSPSSLSA SIGERVTITC | RAS--QGIS-- ----NYLA | WLQQKPGT APKSLIY | A-------- ASSLQS | GVSSRFSGSGFG-- TDFTLTISSLQPEDFATYYC | QQYNS----------- -----------YPFT | FGPGT KVDIK |
| iPS:435 897 | 21-225_188B9 | VK1\|L1/J K3 | DIQMTQSPSSLSA SIGERVTITC | RAS--QGIS-- ----NYLA | WLQQKPGT APKSLIY | A-------- ASSLQS | GVSSRFSGSGFG-- TDFTLTISSLQPEDFATYYC | QQYNS----------- -----------YPFT | FGPGT KVDIK |
| iPS:435 937 | 21-225_190H9 | VK1\|L1/J K3 | DIQMTQSPSSLSA SVGDRVTITC | RAS--QGIG-- ----KYLA | WFQQKPGK ALKSLIY | A-------- ASSLQS | GVPSKFSGSGSG-- TDFTLTISSLQPDDFATYYC | QRYDI----------- -----------YPFT | FGPGT KVDIK |
| iPS:435 961 | 21-225_192A2 | VK1\|L1/J K3 | DIQMTQSPSSLSA SVGDRVTITC | RAN--QGIN-- ----NYLA | WFQQKPGK APKSLIY | A-------- ASSLQS | GVPSKFSGSGSG-- TDFTLTISSLQPEDFATYYC | QQYNS----------- -----------YPFT | FGPGT KVDIK |
| iPS:435 977 | 21-225_192E4 | VK1\|L1/J K3 | DIQMTQSPSSLSA SVGDRVTITC | RAS--QGIG-- ----NYLA | WFQQKPGK APKSLIY | V-------- VSSLQS | GVPSRFSGSGSG-- TDFTLTISSLQPEDFATYYC | QRYDT----------- -----------YPFT | FGPGT KVDIK |
| iPS:436 001 | 21-225_192C1 0 | VK1\|L1/J K3 | DIQMTQSPSSLSA SVGDRVTITC | RAS--QGIG-- ----KYLA | WFQQKPGK APKSLIY | A-------- ASSLQS | GVPSKFSGSGSG-- TDFTLTISSLQPDDFATYYC | QRYDT----------- -----------YPFT | FGPGT KVDFK |
| iPS:436 039 | 21-225_193F8 | VK1\|L1/J K3 | DIQMTQSPSSLSA SVGDRVTITC | RAS--QGVS-- ----NHLA | WFQQKPGR APKSLIY | A-------- ASSLQS | GVPSKFSGSGSG-- ADFTLTISSLQPEDFATYYC | QQYNS----------- -----------YPFT | FGPGT KVDIK |
| iPS:436 078 | 21-225_194H1 2 | VK1\|L1/J K3 | DIQMTQSPSSLSA SVGDRVTITR | RAS--QGIG-- ----KYLA | WFQQKPGK ALKSLIY | A-------- ASSLQS | GVPSKFSGSGSG-- TDFTLTISSLQPDDFATYYC | QRYDT----------- -----------YPFT | FGPGT KVDIK |
| iPS:436 140 | 21-225_197G3 | VK1\|L1/J K3 | DIQMTQSPSSLSA SVGDRVTITC | RAS--QGIG-- ----NHLA | WFQQKPGK APKSLIY | A-------- ASSLQS | GVPSKFSGSRSG-- TDFSLTISSLQPEDFATYYC | QQYSN----------- -----------YPVT | FGPGT KVDIK |

| | | | | | | | | | |
|---|---|---|---|---|---|---|---|---|---|
| iPS:436 167 | 21-225_197E1 1 | VK1\|L1/JK3 | | DIQMTQSPSSLSA SVGDRVTITC | RAS--QGIN-- ----KYLS | WFQQKPGK APKSLIY | A-------- ASSVQS | GVPSKFSGSGSG-- TDFTLTISRLQPDDFATYYG | QRYDT----------- -----------YPFT | FGPGT KVDIK |
| iPS:436 370 | 21-225_211A6 | VK1\|L1/JK3 | | DIQMTQSPSSLSA SVGDSVTITC | RAS--QGIG-- ----KYLA | WFQQKPGK APKSLIY | A-------- ASSLLS | GVPSKFSGSGSG-- TDFTLTISSLQPEDFATYYC | QKYDT----------- -----------YPFT | FGPGT KVDIK |
| iPS:436 392 | 21-225_213B3 | VK1\|L1/JK3 | | DIQMTQSPSSLSA SVGDRVTITC | RAS--QGIG-- ----KYLA | WFQQKPGK APKSLIS | A-------- ASSVLS | GVPSKFSGSGSG-- TDFTLTISSLQPEDFATYYC | QKYDT----------- -----------YPFT | FGPGT KVDIK |
| iPS:436 404 | 21-225_214C3 | VK1\|L1/JK3 | | DIQMTQSPSSLSA SVGDRVTITC | RAS--QGIG-- ----NYLA | WFQQKPGK VPKSLIY | A-------- ASSLQS | GVPSKFSGSGSG-- TDFTLTISSLQPEDFTTYYC | QQYMT----------- -----------YPIT | FGPGT KVDIK |
| iPS:436 406 | 21-225_214E4 | VK1\|L1/JK3 | | DIQMTQSPSSLSA SVGDRVTITC | RAS--QGIS-- ----NYLA | WFQQKPGK APKSLIY | A-------- ASSLQS | GVPSKFSGSGSG-- TDFTLTISSLQPEDFATYYC | QQYLT----------- -----------YPFT | FGPGT KVDIK |
| iPS:437 216 | 21-225_51D5 | VK1\|L1/JK3 | | DIQMTQSPSSLSA SVGDRVTITC | RAS--QGIN-- ----NYLA | WFQQKPGE APKSLIY | A-------- ASSLRS | GVPSQFSGSGSG-- TDFTLTISSLQPEDFATYYC | QQYYS----------- -----------YPFT | FGPGT KVDIK |
| iPS:437 224 | 21-225_56H1 | VK1\|L1/JK3 | | DIQMTQSPSSLSA SVGDRVTITC | RAS--QGIS-- ----HYLA | WFQQKPGK APQSLMS | A-------- ASGLQS | GVPSKFSGSGSG-- TDFTLTISSLQPEDFATYYC | QQYQN----------- -----------YPFT | FGPGT KVDIK |
| iPS:392 620 | 21-225_17H5 | VK1\|L1/JK3 | | DIQMTQSPSSLSA SVGDRVTITC | RAS--QGIS-- ----NYLA | WFQQKPGK APKSLIN | A-------- ASSLQS | GVPSKFSGSGSG-- TDFTLTISSLQPEDFATYYC | QQYHS----------- -----------YPFT | FGPGT KVDIK |
| iPS:392 692 | 21-225_18G10 | VK1\|L1/JK3 | | DIQMTQSPSSLSA SVGDRVTITC | RAS--QDIS-- ----YYLA | WFQQKPGK APKSLIY | V-------- ASSLQS | GVPSKFGGSGFG-- TDFTLTISSLQPEDFATYYC | LQYNS----------- -----------YPFT | FGPGT KVDIK |
| iPS:392 708 | 21-225_18D11 | VK1\|L1/JK3 | | DIQMTQSPSSLFA FVGDRVTITC | RAS--QGIS-- ----YYLA | WFQQKPGK APKSLIY | V-------- ASSLQS | GVPSKFSGSGFG-- TDFTLTISSLQPEDFATYYC | QQYNT----------- -----------YPFT | FGPGT TVDIK |
| iPS:392 714 | 21-225_16G12 | VK1\|L1/JK3 | | DIQMTQSPSSLSA SVGDRVTITC | RAS--QDIS-- ----NYLA | WFQQKPGK APKSLIY | A-------- ASSLQS | GVPSKFRGSGSG-- TDFTLTISNLQPEDFASYYC | QQYHS----------- -----------FPFT | FGPGT KVDIK |
| iPS:392 746 | 21-225_20H7 | VK1\|L1/JK3 | | DIQMTQSPSSLSA SVGDRVTITC | RTS--QGIN-- ----NYLV | WFQQKPGK APKRLIY | A-------- ASSLQS | GVPSKFSGSGSG-- TDFTLTISSLQPEDFATYYC | QQYYS----------- -----------YPFT | FGPGT KMDFK |
| iPS:392 782 | 21-225_22B12 | VK1\|L1/JK3 | | DIQMTQSPSSLSA SVGDRVTITC | RAS--QDIS-- ----NYLA | WFQEKPGK AHKSLIY | G-------- ASSLRS | GVPSKFSGSGSG-- TDFNLTISSLQPEDLATYYC | QQYHS----------- -----------YPFT | FGPGT KVDFK |
| iPS:392 784 | 21-225_23C7 | VK1\|L1/JK3 | | DIQMTQSPSSLSA SVGDRVTITC | RAS--QGIG-- ----IYLA | WFQQKPGK APKSLIS | A-------- ASSLQS | GVPSKFSGSGSG-- TEFTLTISSLQPEDFATYYC | QQYNS----------- -----------YPFT | FGPGT KVDIK |
| iPS:392 802 | 21-225_23E7 | VK1\|L1/JK3 | | DIQMTQSPSSLSA SVGDRVTITC | RAS--QGIS-- ----NYLA | WFQQIPGK APKSLIY | A-------- ASSLQS | GVPSKFSGSGSG-- TDFTLTISSLQPEDFATYYC | QQFYS----------- -----------YPFT | FGPGT KVDIN |
| iPS:392 826 | 21-225_20B9 | VK1\|L1/JK3 | | DIQMTQSPSSLSA SVGDRVTITC | RAS--QGIS-- ----NYLA | WFQQKPGK APKSLIY | V-------- ASSLQS | GVPSKFSGSGSG-- TDFTLTISSLQPEDFATYYC | QQYNT----------- -----------YPFT | FGPGT KVDIK |
| iPS:392 840 | 21-225_23G1 | VK1\|L1/JK3 | | DIQMTQSPSSLSA SVGDRVTITC | RAS--QGIS-- ----NYLA | WFQQKPGK APKSLIS | A-------- ASSLQS | GVPSQFSGSGFG-- TDFTLTISSLQPEDFATYYC | QQYNS----------- -----------YPFT | FGPGT KVDIK |
| iPS:392 842 | 21-225_23G8 | VK1\|L1/JK3 | | DIQMTQSPSSLSA SVGDRVTITC | RAS--QGIR-- ----NYLA | WFQQKPGK APKSLIY | A-------- ASSLQS | GVPSNFSGSGSG-- TDFTLTISSLQPEDFATYYC | QQYNS----------- -----------YPFT | IGPGT KVDIK |
| iPS:392 890 | 21-225_20H9 | VK1\|L1/JK3 | | DIQMTQSPSSLSA SVGDRVTITC | RAS--QGIS-- ----NYLA | WFQQKPGK APKSLIS | A-------- ASSLQS | GVPSKFSGSGSG-- TDFTLTISSLQPEDFATYYC | QQYNS----------- -----------YPFT | FGPGT KVDIK |
| iPS:392 892 | 21-225_20C11 | VK1\|L1/JK3 | | DIQMTQSPSSLSA SVGDRVTITC | RAS--QDIS-- ----NYLA | WFQQKPGK APKSLIY | A-------- ASSLQS | GVPSKFRGSGSG-- TDFTLTISSLQPEDFATYYC | QQYHS----------- -----------FPFT | FGPGT KVDVK |
| iPS:392 950 | 21-225_25C10 | VK1\|L1/JK3 | | DIQMTQSPSSLSA SVGDRVTITC | RAS--QDIS-- ----NYLA | WFQQKPGK APKSLIY | A-------- ASSLQS | GFPSKFSGSGSG-- TDFTLTISSLQPEDFATYYC | QQYHS----------- -----------YPFT | FGPGT KVDIK |
| iPS:392 952 | 21-225_26G1 | VK1\|L1/JK3 | | DIQMTQSPSSLSA SVGDRVTITC | RAS--QDIS-- ----NYLA | WFQQKPGK APKSLIY | A-------- ASSLRS | GVPSNFSGSGSG-- TDFTLTISSLQPENFATYYC | QQYHS----------- -----------YPFT | FGPGT KVDIK |
| iPS:392 962 | 21-225_30A1 | VK1\|L1/JK3 | | DIQMTQSPSSLSA SVGDRVTITC | RAS--QGIS-- ----NYLA | WFQQKPGK APKSLIS | A-------- ASSLQT | GVPSKFSGSGSG-- TDFTLTISSLQPEDFATYYC | QQYNS----------- -----------YPFT | FGPGT KVDIK |

| ID | Clone | VK | # | K_FR1 | K_CDR1 | K_FR2 | K_CDR2 | K_FR3 | K_CDR3 | K_FR4 |
|---|---|---|---|---|---|---|---|---|---|---|
| IPS:392 976 | 21-225_27H12 | VK1\|L1/J K3 | | DIQMTQSPSSLSA<br>SVGDRVTITC | RAS--QGIS--<br>----NYLA | WFQQKPGK<br>APKSLIN | ASSLQS | GVPSKFSGSGSG--<br>TDFTLTISSLQPEDFATYYC | QQYYS-----------<br>------------YPFT | FGPGT<br>KVNIN |
| IPS:393 090 | 21-225_33A5 | VK1\|L1/J K3 | | DIQMTQSPSSLSA<br>SVGDRVTITC | RAS--QGIS--<br>----NYLA | WFQQKPGK<br>APKSLIS | ASSLQS | GVPSKFSGSGSG--<br>TDFTLTISSLQPEDFATYYC | QQYNS-----------<br>------------YPFT | FGPGT<br>NVDIK |
| IPS:393 120 | 21-225_35H8 | VK1\|L1/J K3 | | DIQMTQSPSSLSA<br>SVGDRVTITC | RAS--QAIS--<br>----NYLA | WFQQKPGK<br>APKSLIY | ASGLQS | GVPSKFSCSGSG--<br>TDFTLTISSLQPEDFATYYC | QQYNS-----------<br>------------YPFT | FGPGT<br>KVDFK |
| IPS:393 836 | 21-225_15A2 | VK1\|L1/J K3 | | DIQMTQSPSSLFA<br>FIGDRVTITC | RAS--QGIS--<br>----NYLA | WFQQKPGK<br>APKSLIF | ASSLQS | GVPSKFSGSGFG--<br>TDFTFPISSLQPEDFANYYC | QQYYS-----------<br>------------YPFT | FGPGT<br>QVDVK |
| IPS:393 870 | 21-225_7B1 | VK1\|L1/J K3 | | DIQMTQAPSSLSA<br>SVGDRVTITC | RAS--QDIS--<br>----NHLV | WFQQKPGK<br>APKSLIF | ASSLQS | GVPSQFSGSGSG--<br>TDFTLTISILQPEDFATYYC | HQYNS-----------<br>------------YPFT | FGPGT<br>KVDFK |
| IPS:393 894 | 21-225_5E11 | VK1\|L1/J K3 | | VIQMTQSPSSLSA<br>SVGDRVTITC | RAS--QGIS--<br>----NYLA | WFQQKPGK<br>APKSLIN | ASSVQS | GVPSKFSGNGSG--<br>TDFTLTISSLQPEDFATYYC | HQYHS-----------<br>------------YPFT | FGPGT<br>KVDIK |
| IPS:393 896 | 21-225_2A4 | VK1\|L1/J K3 | | DIQMTQSPSSLFA<br>SVGDRVTITC | RAS--QGIS--<br>----NYLA | WFQQKPGK<br>APKRLIY | ASSLQS | GVPSKFSGSGFG--<br>TDFTLTISSLQPEDFATYYC | QQYNS-----------<br>------------YPFT | FGPGT<br>KVDIK |
| IPS:393 914 | 21-225_16B8 | VK1\|L1/J K3 | | DIQMTQSPSSLSA<br>SVGDRVTITC | RAS--QGIN--<br>----NYLA | WFQQKPGK<br>ALKSLIN | ASSVQS | GVPSKFSGSGSG--<br>TDFTLTISSLQPEDFATYYC | HQYHS-----------<br>------------YPFT | FGPGT<br>KVDIV |
| IPS:393 968 | 21-225_5A5 | VK1\|L1/J K3 | | DIQMTQSPSSLSA<br>SVGDRVTITC | RAS--QGIS--<br>----NYLA | WFQQKPGK<br>APKSLIS | ASSLQS | GVPSKFSGSGSG--<br>TDFTLTISSLQPEDFATYYC | QQYNS-----------<br>------------YPFT | FGPGT<br>KVDIK |
| IPS:393 992 | 21-225_14H8 | VK1\|L1/J K3 | | DIQMTQSPSSLSA<br>SVGDRVTITC | RAS--QGIS--<br>----YYLA | WFQQKPGK<br>APKSLIY | ASSLQS | GVPSKFSGSGSG--<br>TDFTLTISSLQPEDFATYYC | QQYNS-----------<br>------------YPFT | FGPGT<br>KVDIN |
| IPS:394 018 | 21-225_15B1 | VK1\|L1/J K3 | | DIQMTQSPSSLSA<br>SVGDRVTITC | RAS--QGIS--<br>----NYLA | WFQQKPGK<br>APKSLIS | ASSLQS | GVPSNFSGSGSG--<br>TDFTLTISSLQPEDFATYYC | QQYHS-----------<br>------------YPFT | FGPGT<br>KVDIK |
| IPS:394 026 | 21-225_16C7 | VK1\|L1/J K3 | | DIQMTQSPSSLSA<br>SVGDRVTITC | RAS--QGIS--<br>----NYLA | WFQQKPGK<br>APKSLIS | ASSLQS | GVPSKFSGSGSG--<br>TDFTLTISSLQPEDFATYYC | QQYNS-----------<br>------------YPFT | FGPGT<br>KVDIK |
| IPS:394 055 | 21-225_9C8 | VK1\|L1/J K3 | | DIQMTQSPSSLSA<br>SVGDRVTITC | RAS--QGIS--<br>----YYLA | WFQQKPGK<br>APKSLIY | ASSLQS | GVPSKFSGSGSG--<br>TDFTLTISSLQPEDFATYYC | QQYDS-----------<br>------------YPFT | FGPGT<br>KVDIK |
| IPS:398 482 | 21-225_17H6 | VK1\|L1/J K3 | | DIQMTQSPSSLSA<br>SVGDRVTITC | RAS--RDIS--<br>----NYLA | WFQQKPGK<br>APKSLIS | ASSLQS | GVPSKFSGSGSG--<br>TDFTLTISSLQPEDFAIYYC | QQYHS-----------<br>------------YPFT | FGPGT<br>KVDIQ |
| IPS:398 500 | 21-225_23A11 | VK1\|L1/J K3 | | DIQMTQSPSSLST<br>SVGDRVTITC | RAS--QDIS--<br>----NYLA | WFQQKPGK<br>APKRLIY | ASTLQS | GVPSKFSGSGSG--<br>TDFTLTISSLQPEDFATYYC | QQYNS-----------<br>------------YPFT | FGPGT<br>KVDLK |
| IPS:398 526 | 21-225_32B3 | VK1\|L1/J K3 | | DIQMTQSPSSLSA<br>SIGDRVTITC | RAS--QGIS--<br>----NYLA | WFQQKPGK<br>APRSLIY | ASSLQS | GVPSTFSGSGSG--<br>TDFTLTISSLQPEDFATYYC | QQYNS-----------<br>------------YPFT | FGPGT<br>KVDIK |
| IPS:402 235 | 21-225_20F10 | VK1\|L1/J K3 | | DIQLTQSPSSLSA<br>SVGDRVTITC | RAS--QGIN--<br>----NYLA | WFQQKPGK<br>APKSLIY | ASSLQS | GVPSRFSGSGFG--<br>TDFTLTISSLQPEDFATFYC | QQYNS-----------<br>------------YPFT | FGPGT<br>KVDNK |
| | Germline | | | K_FR1 | K_CDR1 | K_FR2 | K_CDR2 | K_FR3 | K_CDR3 | K_FR4 |
| | VK3\|A27/JK 1 | | 32135 | EIVLTQSPGTLSL<br>SPGERATLSC | RAS--QSVSS--<br>----SYLA | WYQQKPGQ<br>APRLLIY | ASSRAT | GIPDRFSGSGSG--<br>TDFTLTISRLEPEDFAVYYC | QQYGS-----------<br>------------SPWT | FGQGT<br>KVEIK |
| IPS:468 810 | 21-225_74D5 | VK3\|A27/ JK1 | | EIVLTQSPGILSL<br>SPGERATLSC | RAS--QSVNS--<br>----NYLA | WYRQKPGQ<br>APRLLIY | ASSRAT | GIPDRFSGSGSG--<br>TDFTLTISRLEPEDFAVYYC | QQYES-----------<br>----------SPWT | FGQGT<br>KVEIK |
| IPS:468 834 | 21-225_94G10 | VK3\|A27/ JK1 | | EIVLTQSPGILSL<br>SPGERATLSC | RAS--QSVNS--<br>----NYLA | WYRQKPGQ<br>APRLLIY | ASSRAT | GIPDRFSGSGSG--<br>TDFILIISRLEPEDFAVYYC | QQYES-----------<br>------------SPWT | FGQGT<br>KVEIK |
| IPS:468 838 | 21-225_80E12 | VK3\|A27/ JK1 | | EIVLTQCPGILSL<br>SPGERATVSC | RAS--QSVNS--<br>----NYLA | WYRQKPDQ<br>APRLLIY | ASSRAT | GIPDRFSGSGSG--<br>TDFILIISRLEPEDFAVYYC | QQYES-----------<br>------------SPWT | FGQGT<br>KVEIK |
| IPS:468 820 | 21-225_76E10 | VK3\|A27/ JK1 | | EIVLTQSPGILSL<br>SPGERATLSC | RAS--QSVNS--<br>----NYLA | WYRQKPGQ<br>APRLLIY | ASSRAT | GIPDRFSGSGSG--<br>TDFILIISRLEPEDFAVYYC | QQYES-----------<br>------------SPWT | FGQGT<br>KVEIK |
| IPS:433 931 | 21-225_44F6 | VK3\|A27/ JK1 | | EIVLTQSPGTLSL<br>SPGERATLSC | RAS--QSVSG-<br>----SYLA | WYQQKPGQ<br>APRLLIY | ASSRAT | GIPDRFSGSGSG--<br>TDFTLTISRLEPEDFAVYYC | QQYGS-----------<br>------------SPWT | FGQGT<br>KVEIK |

| iPS ID | Clone | Germline | | | | | | | | |
|---|---|---|---|---|---|---|---|---|---|---|
| iPS:434 307 | 21-225_59B2 | VK3\|A27/JK1 | | EIVLTQSPGTLSL SPGERATLSC | WAS--QSVYS- ----SFLA | WFQQKSGQ APRLLIY | G-------- ASSRAT | GIPDRFSGSGSG-- TDFTLTISRLEPEDFAVFYC | QQYGT----------- ------------SPWT | FGQGT KVEIK |
| iPS:434 471 | 21-225_75G3 | VK3\|A27/JK1 | | EIVLTQSPGTLSL SPGERATLSC | RAR--QNVDS- ----SYLA | WYQQKPGQ APRLLIY | G-------- ASSRAT | GIPDRFSGSGSG-- TDFTLTISRLEPEDFAVFYC | QQYER----------- ------------SPWT | FGQGT KVEIK |
| iPS:434 473 | 21-225_76D1 | VK3\|A27/JK1 | | EIVLTQSPGTLSL SPGERATLSC | RAS--QNIYS- ----NYLA | WYQFKPGQ APRLLIY | G-------- ASSRAT | GIPDRFSGSGSG-- TDFTLTISRLEPEDFVVYYC | QQYES----------- ------------SPWT | FGQGT KVEIK |
| iPS:434 495 | 21-225_74B2 | VK3\|A27/JK1 | | ELVLTQSPGTLSL SPGERATLSC | RAS--QNIYS- ----SYLA | WYQQKPGQ APRLLIY | G-------- ASSRAT | GIPDRFSGSGSG-- TDFTLTISRLEPEDFAVCC | QQYES----------- ------------SPWT | FGQGT KVEIK |
| iPS:434 497 | 21-225_76A4 | VK3\|A27/JK1 | | EIVLTQSPGTLYL SPGERATLSC | RAS--QSVYS- ----SYLA | WYQQKPGQ APRLLIY | G-------- ASSRAT | GIPDRFSGSGSG-- TDFALTISRLEPEDFAVYYC | QHSDN----------- ------------SPWT | FGQGT KVEIK |
| iPS:434 501 | 21-225_76G4 | VK3\|A27/JK1 | | EIVLTQSPGTLSL SPGERATLSC | RAS--QSVYS- ----SYLA | WYQQKPGQ APRLLIY | G-------- ASSRAT | GIPDRFSGSGSG-- TDFALTISRLEPEDCAVYYC | QHSDN----------- ------------SPWT | FGQGT KVEIK |
| iPS:434 507 | 21-225_74C5 | VK3\|A27/JK1 | | EIVLTQSPGTLSL SPGERATLSC | RAS--QSVNS- ----NYLA | WYQQKPGQ APRLLIY | G-------- ASSRAT | GIPDRVSGSGSG-- TDFNLIISRLEPEDFAVYYC | QQYES----------- ------------SPWT | FGQGT KVEIK |
| iPS:434 517 | 21-225_76A7 | VK3\|A27/JK1 | | EIALTQSPGTLSL SPGERATLSC | RAS--PSVDS- ----SYLA | WYQQRPGQ APRLLIY | G-------- ASSRAP | GIPDRFSGSGSG-- TDFTLTISRLEPEDFAVYYC | QQYES----------- ------------SPWT | FGQGT KVEIK |
| iPS:434 519 | 21-225_74C7 | VK3\|A27/JK1 | | EIVLTQSPGTLSL SPGERATLSC | RTS--PNVDS- ----SYLA | WYQQKPGQ APRLLIY | G-------- ASSRAT | GIPDRFSGSGSG-- TDFTLTISRLEPEDFAVFYC | QQYER----------- ------------SPWT | FGQGT KVEIK |
| iPS:434 523 | 21-225_75C3 | VK3\|A27/JK1 | | EIVLTQSPGTLSL SQGERATLSC | RAS--QSVSS- ----RYLA | WYQQKPGQ APRLLIY | G-------- ASSRAT | GIPDRFSGSGSG-- TDFTLTISRLEPEDFAVYYC | QHYDS----------- ------------SPWT | FGQGT KVEIK |
| iPS:434 531 | 21-225_76C9 | VK3\|A27/JK1 | | EIVLTQSPGTLSL SPGERATLSC | RAS--QSVSS- ----SYLS | WYQQKPGQ APRLLIY | G-------- ASSRAT | GIPDRFSGSGSG-- TDFTLTISRLEPEDFAVYYC | QQYG------------ ------------RSRT | FGQGT KVEIR |
| iPS:434 533 | 21-225_85F7 | VK3\|A27/JK1 | | EPVLTQSPGTLSL SPGERATLSC | RAS--QNIYS- ----NYLA | WYQQKPGQ APRLLIY | G-------- ASSRAT | GIPDRFSGSGSG-- TDFTLTISRLEPEDFAVCC | QQYES----------- ------------SPWT | FGQGT KVEIK |
| iPS:434 547 | 21-225_74H5 | VK3\|A27/JK1 | | EIVLTQSPGTLSL SPGERATLSC | RAS--QSVNS- ----NYLA | WYRQKPGQ APRLLIY | G-------- ASSRAT | GIPDRFSGSGSG-- TDFILIISRLEPEDFAVYYC | QQYES----------- ------------SPWT | FGQGT KVEIK |
| iPS:434 559 | 21-225_74D11 | VK3\|A27/JK1 | | EIVLTQSPGTLSL SPGERATLSC | RAS--QSVYS- ----SYLA | WYQQKPGQ APRLLIY | G-------- ASSRAT | GIPDRFSGSGSG-- TDFALTISRLEPEDFAVYYC | QHYDN----------- ------------SPWT | FGQGT KVEIK |
| iPS:434 561 | 21-225_77G1 | VK3\|A27/JK1 | | EIVLTQSPGTLYL SPGERATLSC | RAS--QSVYS- ----SYLA | WYQQKPGQ APRLLIY | G-------- ASSRAT | GIPDRFSGSGSG-- TDFALTISRLEPEDCAVYYC | QHYDN----------- ------------SPWT | FGQGT KVEIK |
| iPS:434 565 | 21-225_75B10 | VK3\|A27/JK1 | | EIVLTQSPGTLSL SPGERATLSC | RAS--PSVNS- ----YYLA | WYQQKPGQ APRLLIY | G-------- ATSRAT | GIPDRFSGSGSG-- TDFTLTISRLEPEDFAVYFC | QQYED----------- ------------SPWT | FGQGT KVEIK |
| iPS:434 571 | 21-225_74D2 | VK3\|A27/JK1 | | EIVLTQSPGTLSL SPGERATLSC | RAS--QSVDS- ----NYLA | WYQQKPGQ APRLLIY | G-------- ASSRAP | GIPDRFSGSGSG-- TDFTLTINRLEPEDFAVFC | QQYES----------- ------------SPWT | FGQGT KVEIK |
| iPS:434 579 | 21-225_77F7 | VK3\|A27/JK1 | | EIVLTQSPGTLYL SPGERATLSC | RAS--QSVYS- ----SYLA | WYQQKPGQ APRLIIY | G-------- ASSRAT | GIPDRFSGSGCG-- TDFALTISRVEHEDCAVYYC | QHYDN----------- ------------SPWT | FGQGT KVEIK |
| iPS:434 581 | 21-225_74B12 | VK3\|A27/JK1 | | EIVLTQSPGTLYL SPGERATLSC | RAS--QSVYS- ----SYLA | WYQQKPGQ APRLLIY | G-------- ASSRST | GIPDRFSGSGSG-- TDFALTISRLEPEDFAVYYC | QHYDN----------- ------------SPWT | FGQGT KVEIK |
| iPS:434 585 | 21-225_75A12 | VK3\|A27/JK1 | | EIVLTQSPGTLSL SPGERATLSC | RAS--QSVSS- ----RYLA | WYQQKPGQ APRLLIY | G-------- ASSRAT | GIPDRFSGSGSG-- TDFTLTISRLEPADFAVYYC | QHYDS----------- ------------SPWT | FGQGT KVEIK |
| iPS:434 595 | 21-225_77A10 | VK3\|A27/JK1 | | EIVLTQSPGTLSL SPGERATLSC | RAS--QSVHS- ----RYLA | WYQQKPGQ APKLLIF | G-------- ASSRAT | GIPDRFSGSGSG-- TDFTLTISRLEPEDFAVYYC | QHYDS----------- ------------SPWT | FGQGT KVEIK |
| iPS:434 611 | 21-225_77C12 | VK3\|A27/JK1 | | EIVLTQSPGTLSL SPGERATLSC | RAR--QSVDS- ----SYLA | WYQQKRGQ APRLLIY | G-------- ASSRAT | GIPDRFSGSGSG-- TDFTLTISRLEPEDFAVYYC | QQYES----------- ------------SPWT | FGQGT KVEIK |
| iPS:434 633 | 21-225_74G8 | VK3\|A27/JK1 | | EIVLTQSPGTLSL SPGERATLSC | RAS--QSFSS- ----AYLA | WYQQKPGQ APRLLIY | G-------- TSSRAT | GIPDRFSGSGSG-- TDFTLTIGRLEPEDFAVYYC | QQYG------------ ------------NSRT | FGQGT KVEIK |

| | | | | | | | | | | |
|---|---|---|---|---|---|---|---|---|---|---|
| iPS:434 637 | 21-225_78E7 | VK3\|A27/JK1 | | EIVLTQSPGTLSL SPGERATLSC | RAS--QNVDS- ----NYLA | WYQQKPGQ APRLLIY | G-------- ASSRAT | GIPDRFSGSGSG-- TDFTLTISRLEPEDFAVFYC | QQYER----------- -----------SPWT | FGQGT KVEIK |
| iPS:434 657 | 21-225_79G1 | VK3\|A27/JK1 | | EIVLTQSPGTLYL SSGERATLSC | RAS--QSVYS- ----SYLA | WYQQKPAQ APRLLIY | G-------- ASSRST | GIPDRFSGSGSG-- TDFALTISRLEPEDFAVYYC | QHYDN----------- -----------SPWT | FGQGT KVEIK |
| iPS:434 663 | 21-225_79F3 | VK3\|A27/JK1 | | EIVLTQSPGTLYL SPGERATLSC | RAS--QSVYS- ----SYLA | WYQQKPAQ APRLLIY | G-------- ASSRST | GIPDRFSGSGSG-- TDFALTISRLEPEDFAVYYC | QHYDN----------- -----------SPWT | FGQGT KVEIK |
| iPS:434 671 | 21-225_74F4 | VK3\|A27/JK1 | | EIVLTQSPGTLSL SPGERATLSC | RAS--QIFSS- ----SYLA | WYQQKPGQ SPRLLIY | G-------- ASSRAT | GIPDRFSGSGSG-- TDFTLTISRLEPEDFAVYYC | QQYG------------ ---------SSRT | FGQGT KVEIK |
| iPS:434 687 | 21-225_75A5 | VK3\|A27/JK1 | | EIVLTQSPGTLYL SPGERATLSC | RAS--QSVYS- ----SYLA | WYQQKPGQ APRLLIY | G-------- ASSRAT | GIPDRFSGSGSG-- TDFALTISRLEPEDFAVYYC | QHYDN----------- -----------SPWT | FGQGT KVEIK |
| iPS:434 691 | 21-225_75G7 | VK3\|A27/JK1 | | EIVLTQSPGTLSL SPGERATLSC | RAR--QSVDS- ----SYLA | WYQQKRGQ APRLLIY | G-------- ASSRAT | GIPDRFSGSGSG-- TDFTLTISRLEPEDFAVYYC | QQYES----------- -----------SPWT | FGQGT KVEIK |
| iPS:434 693 | 21-225_79F11 | VK3\|A27/JK1 | | EIVLTQSPGTLSL SPGERATLSC | RAS--QSVYS- ----SYLA | WYQQKPGQ APRLLIY | G-------- ASSRAT | GIPDRFSGSGSG-- TDFALTISRLEPEDFAVYYC | QHSDN----------- -----------SPWT | FGQGT KVEIK |
| iPS:434 699 | 21-225_79G12 | VK3\|A27/JK1 | | EIVLTQSPGTLYL SPGERATLSC | RAS--QSVYS- ----SYLA | WYQQKPGQ APRLLIY | G-------- ASSRST | GIPDRFSGSGSG-- TDFALTISRLEPEDCAVYYC | QHSDN----------- -----------SPWT | FGQGT KVEIK |
| iPS:434 701 | 21-225_80A1 | VK3\|A27/JK1 | | EIVLTQSPGTRYL SPGERATLSC | RAS--QSVYS- ----SYLA | WYQQKPGQ APRLLIY | G-------- ASSRST | GIPDRFSGSGCG-- TDFALTISRLEPEDCAVYYC | QHSDN----------- -----------SPWT | FGQGT KVEIK |
| iPS:434 703 | 21-225_80C1 | VK3\|A27/JK1 | | EIVLTQSPGTLSL SPGERATLSC | RAS--QSVYS- ----SYLA | WYQQKPGQ APRLLIY | G-------- ASSRST | GIPDRFSGSGSG-- TDFALTISRVEPEDCAVYYC | QHSDN----------- -----------SPWT | FGQGT KVEIK |
| iPS:434 709 | 21-225_80E3 | VK3\|A27/JK1 | | EIVLTQSPGTLYL SSGERATLSC | RAS--QSVYS- ----SYLA | WYQQKPGQ APRLLIY | G-------- ASSRST | GIPDRFSGSGSG-- TDFALTISRVEPEDFAVYYC | QHSDN----------- -----------SPWT | FGQGT KVEIK |
| iPS:434 715 | 21-225_80D5 | VK3\|A27/JK1 | | ELVLTQSPGTLSL SPGKRVTLSC | RAS--QNIYS- ----SYLA | WYQQKPGQ APRLLIY | G-------- ASSRAT | GIPDRFSGSGSG-- TDFTLTITRLEPEDFAVYCC | QQYES----------- -----------SPWT | FGQGT KVEIK |
| iPS:434 717 | 21-225_80A6 | VK3\|A27/JK1 | | EIVLTQSPGTLSL SPGEIPTLSC | RAS--QSVDS- ----GYLA | WYQQKPGQ APRLLIY | G-------- ASSRAP | GIPDRFSGSGSG-- TDFTLTISRLEPEDFAVYYC | QQYES----------- -----------SPWT | FGQGT KVEIK |
| iPS:434 725 | 21-225_80H7 | VK3\|A27/JK1 | | EIVLTQSPGTLSL SPGERATLSC | RAS--QSINS- ----NYLA | WYQQKPGQ APRLLIY | G-------- ASSRAT | GIPDRFSGSGSG-- TDFTLTISRLEPEDFAVFYC | QQYES----------- -----------SPWT | FGQGT KVEIK |
| iPS:434 735 | 21-225_80B10 | VK3\|A27/JK1 | | EIVLTQSPGTLSL SPGERATLSC | RAS--QSVDS- ----SYLA | WYQQKPGQ APRLLIY | G-------- ASSRAP | GIPDRFSGSGSG-- TDFTLTISRLEPEDFAVYYC | QQYES----------- -----------SPWT | FGQGT KVEIK |
| iPS:434 743 | 21-225_74A4 | VK3\|A27/JK1 | | EIVLTQSPGTLYL SPGERATLSC | RAS--QSVYS- ----SYLA | WYQQKPGQ APRLLIY | G-------- ASSRST | GIPDRFSGSGCG-- TDFALTISRLEPEDCAVYYC | QHSDN----------- -----------SPWT | FGQGT KVEIK |
| iPS:434 751 | 21-225_80H12 | VK3\|A27/JK1 | | EIVLTQSPGTLYL SPGERATLSC | RAS--QSVYS- ----SYLA | WYQQKPGQ APRLLIY | G-------- ASSRST | GIPDRFSGSGSG-- TDFALTISRLEPEDFAVYYC | QHSDN----------- -----------SPWT | FGQGT KVEIK |
| iPS:434 759 | 21-225_81C5 | VK3\|A27/JK1 | | EIVLTQSPGTLYL SSGERATLSC | RAS--QSVYS- ----SYLA | WYQQKPGQ APRLLIY | G-------- ASSRST | GIPDRFSGSGCG-- TDFALTISRLEPEDCAVYYC | QHSDN----------- -----------SPWT | FGQGT KVEIK |
| iPS:434 773 | 21-225_75D9 | VK3\|A27/JK1 | | EIVLTQSPGTLSL SPGERATLSC | RAS--QSVSS- ----SYLA | WYQQKRGQ APRLLIY | G-------- ASSRAT | GIPDRFSGSGSG-- TDFALTISRLEPEDFAVYYC | QQYES----------- -----------SPWT | FGQGT KVEIK |
| iPS:434 777 | 21-225_81C11 | VK3\|A27/JK1 | | EIVLTQSPGTRYL SPVERATLSC | RAS--QSVYS- ----SYLA | WYQQKPGQ APRLLIY | G-------- ASSRST | GLPDRFSGSGCG-- TDFALTISRLEPEDCAVYYC | QHSDN----------- -----------SPWT | FGQGT KVEIK |
| iPS:434 809 | 21-225_74F5 | VK3\|A27/JK1 | | EIVLTQSPGTLYL SPGERATLSC | RAS--QSVYS- ----SYLA | WYQQKPGQ APRLLIY | G-------- ASSRST | GIPDRFSGSGSG-- TDFALTISRLEPEDFAVYYC | QHSDN----------- -----------SPWT | FGQGT KVEIK |
| iPS:434 821 | 21-225_83G1 | VK3\|A27/JK1 | | EIVLTQSPGTRYL SPGERATLSC | RAS--QSVYS- ----SYLA | WYQQKPGQ APRLLIY | G-------- ASSRST | VLPDRFSGSGSG-- TDFALTISRVEPEDCAVYYC | QHSDN----------- -----------SPWT | FGQGT KVEIK |
| iPS:434 835 | 21-225_83B6 | VK3\|A27/JK1 | | EIVLTQSPGTLSL SPGERATLSC | RAS--QSVDS- ----GYLA | WYQQKPGQ APRLLIY | G-------- ASSRTP | GIPDRFSGSGSG-- TDFTLTISRLEPEDFAVYYC | QQYES----------- -----------SPWT | FGQGT KVEIK |

| iPS:434 839 | 21-225_83B7 | VK3\|A27/ JK1 | | EIVLTQSPGTRYL SSVERATLSC | RAS--QSVYS- ----SYLA | WYQQKPGQ APRLLIY | G-------- ASSRST | GIPDRFSGSGSG-- TDFALTISRLEPEDCAVYYC | QHSDN----------- -----------SPWT | FGQGT KVEIK |
| iPS:434 849 | 21-225_83C10 | VK3\|A27/ JK1 | | EIVLTQSPGTLSL SPGERATLSC | RAS--PSVHS- ----NYLA | WYQQKPGQ APRLLIY | G-------- ASSRAT | GIPDRFSGSGSG-- TDFILTISRLEPEDFAVYYC | QQYES----------- -----------SPWT | FGQGT KVEIK |
| iPS:434 869 | 21-225_84E12 | VK3\|A27/ JK1 | | EIVLTQSPGTLSL SPGERATLSC | RAS--QSINS- ----NYLA | WYQQKPGQ APRLLIY | G-------- ASSRAT | GIPDRFSGSGSG-- TDFTLTISRLEPDDFAVFYC | QQYES----------- -----------SPWT | FGQGT KVEIK |
| iPS:434 879 | 21-225_85A3 | VK3\|A27/ JK1 | | EIVLTQSPGTLFL SPGERATLSC | RAS--QSVYS- ----SYLA | WYQQKPAQ APRLLIY | G-------- ASSRAS | GIPDRFSGSGSG-- TDFALTISRLEPEDFAVYYC | QHYDN----------- -----------SPWT | FGQGT KVEIK |
| iPS:434 881 | 21-225_85B4 | VK3\|A27/ JK1 | | EIVLTQSPGTLFL SPGERATLSC | RAS--QSVYS- ----SYLA | WYQQKPGQ APRLLIY | G-------- ASSRAT | GIPDRFSGSGSG-- TDFALTISRLEPEDFAVYYC | QHYDN----------- -----------SPWT | FGQGT KVEIK |
| iPS:434 887 | 21-225_85D6 | VK3\|A27/ JK1 | | EIVLTQSPGTLFL SQGERATLSC | RAS--QSVSS- ----RYLA | WYQQKPGQ APRLLIY | G-------- ASSRAT | GIPDRFSGSGSG-- TDFTLTISRLEPEDFAVYYC | QHYDS----------- -----------SPWT | FGQGT KVEIK |
| iPS:434 891 | 21-225_85G6 | VK3\|A27/ JK1 | | EIALTQSPGTLSL SPGERATLSC | RAS--PSVDS- ----SYLA | WYQQKPGQ APRLLIY | G-------- AASRAP | GIPDRFSGSGSG-- TDFTLTISRLEPEDFVVYYC | QQYES----------- -----------SPWT | FGQGT KVEIK |
| iPS:434 895 | 21-225_74H7 | VK3\|A27/ JK1 | | ELVLTQSPGTLSL SPGERATLSC | RAS--QNIYS- ----SYLA | WYQQKPGQ APRLLIY | G-------- ASSRAT | GIPDRFSGSGSG-- TDFTLTISRLEPEDFAVYCC | QQYES----------- -----------SPWT | FGQGT KVEIK |
| iPS:434 899 | 21-225_85B9 | VK3\|A27/ JK1 | | EIVLTQSPGTLFL SPGERATLSC | RAS--QSVNS- ----NYLA | WYRQKPGQ APRLLIY | G-------- ASSRAT | GIPDRFSGSGSG-- TDFILTISRLEPEDFAVYYC | QQYES----------- -----------SPWT | FGQGT KVEIK |
| iPS:434 907 | 21-225_85G10 | VK3\|A27/ JK1 | | EIVLTQSPGSLSL SPGERATLSC | RAS--QSVWS- ----GYLA | WYQQKPGQ APRLLIY | G-------- ASSRAT | GIPDRFSGSGSG-- TDFTLTISRLEPEDFAVYFC | QQYES----------- -----------SPWT | FGQGT KVEIK |
| iPS:434 913 | 21-225_86C1 | VK3\|A27/ JK1 | | EIVLTQSPGTLYL SPGERATLSC | RAS--QSVYS- ----SYLA | WYQQKPAQ APRLLIY | G-------- ASSRAT | GIPDRFSGSGSG-- TDFALTISRLEPEDCAVYYC | QHYDN----------- -----------SPWT | FGQGT KVEIK |
| iPS:434 921 | 21-225_86E4 | VK3\|A27/ JK1 | | EIVLTQSPGTRYL SPGERATLSC | RAS--QSVYS- ----SYLA | WYQQKPGQ APRLLIY | G-------- ASSRST | GIPDRFSGSGSG-- TDFALTISRLEPEDCAVYYC | QHSDN----------- -----------SPWT | FGQGT KVEIK |
| iPS:434 939 | 21-225_86C11 | VK3\|A27/ JK1 | | EIVLTQSPGTLYL SPGERATLSC | RAS--QSVYS- ----SYLA | WYQQKPGQ APRLLIY | G-------- ASSRST | GLPDRFSGSGSG-- TDFALTISRLEPEDCAVYYC | QHSDN----------- -----------SPWT | FGQGT KVEIK |
| iPS:434 943 | 21-225_87H1 | VK3\|A27/ JK1 | | EIVLTQSPGTLSL SPGERATLSC | RAS--QSVDS- ----NYLA | WYQQKPGQ APRLLIY | G-------- ASARTT | GIPDRFSGSGSG-- TDFTLTISRLEPEDFAVYSC | QQYES----------- -----------SPWT | FGQGT KVEIK |
| iPS:434 945 | 21-225_87E5 | VK3\|A27/ JK1 | | EFVLTQSPGTLYL SPGERATLSC | RAS--QSVYS- ----SYLA | WYQQKPGQ APRLLIY | G-------- ASSRST | GIPDRFSGSGSG-- TDFALTISRVEPEDCAVYYC | QHSDN----------- -----------SPWT | FGQGT KVEIK |
| iPS:434 955 | 21-225_87C9 | VK3\|A27/ JK1 | | EIVLTQSPGTLYL SPVERATLSC | RAS--QSVYS- ----SYLA | WYQQKPGQ APRLLIY | G-------- ASSRST | GIPDRFSGSGSG-- TDFALTISRLEPEDCAVYYC | QHSDN----------- -----------SPWT | FGQGT KVEIK |
| iPS:434 961 | 21-225_87A12 | VK3\|A27/ JK1 | | EIVLTQSPGTRYL SPGERATLSC | RAS--QSVYS- ----SYLA | WYQQKPGQ APRLLIY | G-------- ASSRST | VIPDRFSGSGCG-- TDFALTISRLEPEDCAVYYC | QHSDN----------- -----------SPWT | FGQGT KVEIK |
| iPS:434 969 | 21-225_88H1 | VK3\|A27/ JK1 | | EIVLTQSPGTLYL SPGERATLSC | RAS--QSVYS- ----SYLA | WYQQKPGQ APRLLIY | G-------- ASSRST | VIPDRFSGSGCG-- TDFALTISRLEPEDCAVYYC | QHSDN----------- -----------SPWT | FGQGT KVEIK |
| iPS:434 981 | 21-225_88E7 | VK3\|A27/ JK1 | | EIVLTQSPGTLSL SPGERATLSC | RAS--QSVYS- ----SYLA | WYQQKPGQ APRLLIY | G-------- ASSRST | GIPDRFSGSGSG-- TDFALTISRVEPEDCAVYYC | QHSDN----------- -----------SPWT | FGQGT KVEIK |
| iPS:434 983 | 21-225_88F7 | VK3\|A27/ JK1 | | EIVLTQSPGTLSL SPGERATLSC | RAS--QSVYS- ----SYLA | WYQQKPGQ APRLLIY | G-------- ASSRST | GIPDRFSGSGSG-- TDFALTISRLEPEDCAVYYC | QHSDN----------- ----------- | FGQGT KVEIK |
| iPS:434 995 | 21-225_88G9 | VK3\|A27/ JK1 | | EIVLTQSPGTRYL SPGERATLSC | RAS--QSVYS- ----SYLA | WYQQKPGQ APRLLIY | G-------- ASSRST | GIPDRFSGSGSG-- TDFALTISRVEPEDCAVYYC | QHSDN----------- -----------SPWT | FGQGT KVEIK |
| iPS:434 999 | 21-225_75A8 | VK3\|A27/ JK1 | | EIVLTQSPGTLYL SPGERATLSC | RAS--QSVYS- ----SYLA | WYQQKPGQ APRLLIY | G-------- ASSRAT | GIPDRFSGSGSG-- TDFALTISRVEPEDCAVYYC | QHSDN----------- -----------SPWT | FGQGT KVEIK |
| iPS:435 013 | 21-225_89D5 | VK3\|A27/ JK1 | | EIVLTQSPGTLYL SSGERATLSC | RAS--QSVYS- ----SYLA | WYQQKPGQ APRLLIY | G-------- ASSRST | GIPDRFSGSGSG-- TDFALTISRLEPEDFAVYYC | QHYDN----------- -----------SPWT | FGQGT KVEIK |

| | | | | | | | | | |
|---|---|---|---|---|---|---|---|---|---|
| iPS:435 015 | 21-225_89H5 | VK3\|A27/JK1 | EIVLTQSPGTLSL<br>SPGERATLSC | RAS--QSVNS-<br>----NYLA | WYQQKPGQ<br>APRLLIY | G--------<br>AFSRAT | GIPDRVSGSGSG--<br>TDFNLIISRLEPEDFAVYYC | QQYES-----------<br>------------SVWT | FGQGT<br>KVEIK |
| IPS:435 025 | 21-225_89E10 | VK3\|A27/JK1 | EIVLTQSPGTLYL<br>SSGERATLSC | RAS--QSVYS-<br>----SYLA | WYQQKPGQ<br>APRLLIY | G--------<br>ASSRST | VIPDRFSGSGSG--<br>TDFALTISRVEPEDFAVYYC | QHSDN-----------<br>------------SPWT | FGQGT<br>KVEIK |
| IPS:435 029 | 21-225_89A11 | VK3\|A27/JK1 | EIVLTQSPGTLSL<br>SPGERATLSC | RAS--QSVDS-<br>----NFLA | WYQQKPGQ<br>APRLLIY | G--------<br>ASARTT | GIPDRFSGSGSG--<br>TDFTLTISRLEPEDFAVYSC | QQYEI-----------<br>------------SPWT | FGQGT<br>KVEIK |
| IPS:435 039 | 21-225_90G4 | VK3\|A27/JK1 | EIVLTQSPGTLYL<br>SPGERATLSC | RAS--QSVYS-<br>----SYLA | WYQQKPGQ<br>APRLLIY | G--------<br>ASSRST | VIPDRFSGSGSG--<br>TDFALTISRVEPEDCAVYYC | QHSDN-----------<br>------------SPWT | FGQGT<br>KVEIK |
| IPS:435 041 | 21-225_90A5 | VK3\|A27/JK1 | EIVLTQSPGTLYL<br>SPGERATLSC | RAS--QSVYS-<br>----SYLA | WYQQKPGQ<br>APRLLIY | G--------<br>ASSRAT | GIPDRFSGSGSG--<br>TDFALTISRVEHEDFAVYYC | QHYDN-----------<br>------------SPWT | FGQGT<br>KVEIK |
| IPS:435 043 | 21-225_90G5 | VK3\|A27/JK1 | EIVLTQSPGTLYL<br>SPGERATLSC | RAS--QSVYS-<br>----SYLA | WYQQKPGQ<br>APRLIIY | G--------<br>ASSRAT | GIPDRFSGSGSG--<br>TDFALTISRVEHEDFAVYYC | QHYDN-----------<br>------------SPWT | FGQGT<br>KVEIK |
| IPS:435 055 | 21-225_90F10 | VK3\|A27/JK1 | EIVLTQSPGTLYL<br>SPGERATLSC | RAS--QSVYS-<br>----SYLA | WYQQKPGQ<br>APRLLIY | G--------<br>ASSRST | GIPDRFSGSGSG--<br>TDFALTISRVEPEDCAVYYC | QHYDN-----------<br>------------SPWT | FGQGT<br>KVEIK |
| IPS:435 073 | 21-225_91B2 | VK3\|A27/JK1 | EIVLTQSPGTLYL<br>SPGERATLSC | RAS--QSVYS-<br>----SYLA | WYQQKPGQ<br>APRLLIY | G--------<br>ASSRST | GIPDRFSGSGSG--<br>TDFALTISRVEPEDCAVYYC | QHYDN-----------<br>------------SPWT | FGQGT<br>KVEIK |
| IPS:435 075 | 21-225_91B3 | VK3\|A27/JK1 | EIVLTQSPGTLYL<br>SPGERATLSC | RAS--QSVYS-<br>----SYLA | WYQQKPGQ<br>APRLLIY | G--------<br>ASSRST | GIPDRFSGSGSG--<br>TDFALTISRVEHEDCAVYYC | QHYDN-----------<br>------------SPWT | FGQGT<br>KVEIK |
| IPS:435 077 | 21-225_91F3 | VK3\|A27/JK1 | EIVLTQSPGTLYL<br>SPGERATLSC | RAS--QSVYS-<br>----SYLA | WYQQKPAQ<br>APRLLIY | G--------<br>ASSRST | GIPDRFSGSGSG--<br>TDFALTISRVEPEDCAVYYC | QHSDN-----------<br>------------SPWT | FGQGT<br>KVEIK |
| IPS:435 079 | 21-225_91B4 | VK3\|A27/JK1 | EIVLTQSPGTLYL<br>SPGERATLSC | RAS--QSVYS-<br>----SYLA | WYQQKPGQ<br>APRLIIY | G--------<br>ASSRST | GIPDRFSGSGSG--<br>TDFALTISRVEPEDCAVYYC | QHYDN-----------<br>------------SPWT | FGQGT<br>KVEIK |
| IPS:435 089 | 21-225_91E9 | VK3\|A27/JK1 | EIVLTQSPGTLYL<br>SPGERATLSC | RAS--QSVYS-<br>----SYLA | WYQQKPGQ<br>APRLLIY | G--------<br>ASSRAT | GIPDRFSGSGCG--<br>TDFALTISRVEHEDCAVYYC | QHSDN-----------<br>------------SPWT | FGQGT<br>KVEIK |
| IPS:435 097 | 21-225_92B1 | VK3\|A27/JK1 | EIVLTQSPGTLSL<br>SPGERATLSC | RAS--QSVGS-<br>----NYLA | WYQQKRGQ<br>APRLLIY | G--------<br>ASSRAT | DIPDRFSGSGSG--<br>TDFTLTISRLEPEDFAVYYC | QQYES-----------<br>------------SPWT | FGQGT<br>KVEIK |
| IPS:435 111 | 21-225_92D6 | VK3\|A27/JK1 | EIVLTQSPGTLYL<br>SPGERATLSC | RAS--QSVYS-<br>----SYLA | WYQQKPGQ<br>APRLLIY | G--------<br>ASSRST | VIPDRFSGSGCG--<br>TDFALTISRLEPEDCAVYYC | QHYDN-----------<br>------------SPWT | FGQGT<br>KVEIK |
| IPS:435 115 | 21-225_77C5 | VK3\|A27/JK1 | EIVLTQSPGTLSL<br>SPGERATLSC | RAS--QSVYS-<br>----SYLA | WYQQKPGQ<br>APRLLIY | G--------<br>ASSRST | GIPDRFSGSGSG--<br>TDFALTISRLEPEDCAVYYC | QHYDN-----------<br>------------SPWT | FGQGT<br>KVEIK |
| IPS:435 171 | 21-225_93C2 | VK3\|A27/JK1 | EIVLTQSPGTLYL<br>SPGERATLSC | RAS--QSVYS-<br>----SYLA | WYQQKPGQ<br>APRLLIY | G--------<br>ASSRAT | GIPDRFSGSGSG--<br>TDFALTISRLEHEDCAVYYC | QHYDN-----------<br>------------SPWT | FGQGT<br>KVEIK |
| IPS:435 177 | 21-225_93E4 | VK3\|A27/JK1 | EIVLTQSPGTLYL<br>SPGERATLSC | RAS--QSVYS-<br>----SYLA | WYQQKPGQ<br>APRLLIY | G--------<br>ASSRST | GIPDRFSGSGCG--<br>TDFALTISRVEPEDFAVYYC | QHSDN-----------<br>------------SPWT | FGQGT<br>KVEIK |
| IPS:435 183 | 21-225_93E9 | VK3\|A27/JK1 | EIVLTQSPGTLSL<br>SPGERATLSC | RAS--QSVDS-<br>----SYLA | WYQQKTGQ<br>APRLLIY | G--------<br>ASSRAP | GIPDRFSGSGSG--<br>TDFTLTISRLEPEDFAVYYC | QQYES-----------<br>------------SPWT | FGQGT<br>KVEIK |
| IPS:435 195 | 21-225_94D3 | VK3\|A27/JK1 | EIVLTQSPGTLSL<br>SQGERATLSC | RAS--QSVSS-<br>----RYLA | WYQQKPGQ<br>APRLLIY | G--------<br>ASSRAT | GIPDRFSGSGSG--<br>TDFTLTISRLEPEDFAVYYC | QHYDS-----------<br>------------SPWT | FGQGT<br>KVENQ |
| IPS:435 217 | 21-225_94F12 | VK3\|A27/JK1 | EIVLTQSPGTLYL<br>SPGERATFSC | RAS--QSVYS-<br>----SYLA | WYQQKPGQ<br>APRLLIY | G--------<br>ASSRST | GIPDRFSGSGSG--<br>TDFALTISRLEPEDFAVYYC | QHYDN-----------<br>------------SPWT | FGQGT<br>KVEIK |
| IPS:435 219 | 21-225_95D2 | VK3\|A27/JK1 | EIVLTQSPGTLSL<br>SPGERATLSC | RAS--QSVYS-<br>----SYLA | WYQQKPGQ<br>APRLLIY | G--------<br>ASSRAT | GIPDRFSGSGSG--<br>TDFALTISRLEPEDCAVYYC | QHYDN-----------<br>------------SPWT | FGQGT<br>KVEIK |
| IPS:435 235 | 21-225_95F9 | VK3\|A27/JK1 | EIVLTQSPGTLYL<br>SPGERATLSC | RAS--QSVYS-<br>----SYLA | WYQQKPGQ<br>APRLLIY | G--------<br>ASSRST | GIPDRFSGSGCG--<br>TDFALTISRLEPEDFAVYYC | QHYDN-----------<br>------------SPWT | FGQGT<br>KVEIK |
| IPS:435 237 | 21-225_95G9 | VK3\|A27/JK1 | EIVLTQSPGTLYL<br>SSGERATLSC | RAS--QSVYS-<br>----SYLA | WYQQKPGQ<br>APRLLIY | G--------<br>ASSRST | GIPDRFSGSGSG--<br>TDFALTISRVEHEDCAVYYC | QHYDN-----------<br>------------SPWT | FGQGT<br>KVEIK |

| iPS:435 239 | 21-225_95H10 | VK3|A27/ JK1 | | EIVLSQSPGILYL SSGERATLSC | RAS--QSVYS- ----SYLA | WYQQKPGQ APRLLIY | G-------- ASSRAT | GIPDRFSGSGSG-- TDFALTISRLEPEDFAVYYC | QHYDN----------- ------------SPWT | FGQGT KVEIK |
| iPS:435 273 | 21-225_97A2 | VK3|A27/ JK1 | | EIVLTQSPGTLYL SSGERATLSC | RAS--QSVYS- ----SYLA | WYQQKPGQ APRLLIY | G-------- ASSRST | GIPDRFSGSGCG-- TDFALTISRLEPEDCAVYYC | QHSDN----------- ------------SPWT | FGQGT KVEIK |
| iPS:435 261 | 21-225_97E5 | VK3|A27/ JK1 | | EIVLTQSPGTLSL SPGERATLSC | RAS--QSVYS- ----SYLA | WYQQKPGQ APRLLIY | G-------- ASSRAT | GIPDRFSGSGCG-- TDFALTISRVEPEDCAVYYC | QHSDN----------- -------------SPWT | FGQGT KVEIK |
| iPS:435 331 | 21-225_147G8 | VK3|A27/ JK1 | | QIVLTQSPGTLSL SPGERATLSC | RAS--QRIFS- ----NYLA | WYQQKPGQ APRILIY | G-------- ASSRAT | GIPDRISGSGSG-- TDFTLTIITRLEPEDFAVYYC | QQYDS----------- ------------SPWT | FGQGT KVEIN |
| iPS:435 815 | 21-225_190G1 0 | VK3|A27/ JK1 | | EIVLTQSPGTLSL SPGERATLSC | RAS--PSVSS- ----RFLA | WYQQKPGQ APRLLIY | G-------- ASSRAT | GIPDRFSGSGSG-- TDFTLTNSRLEPEDFAVYYC | QQYGS----------- -----------SPPWT | FGQGT KVEIK |
| iPS:435 843 | 21-225_191F1 | VK3|A27/ JK1 | | EIVLTQSPGTLSL SPGERAALSC | RAS--QSISL- ----NFLA | WYQQKPGQ APRLLIY | G-------- ASSRAT | GIPDRFSGSGSG-- TDFTLTINRLEPEDFAVYYC | QQYGR----------- ------------SPWT | FGQGT KVEVK |
| iPS:435 847 | 21-225_191A3 | VK3|A27/ JK1 | | EIVLTQSPGTLSL SPGERATLSC | RAS--QSIRS- ----SFLA | WYQQKPGQ APRLLIY | G-------- ASSRAT | GIPDRFSGSGSG-- TDFTLTISRLEPEDFAVYYC | QQYGN----------- ------------SPWA | FGQGT KVEVK |
| iPS:435 849 | 21-225_191C3 | VK3|A27/ JK1 | | EIVLTQSPGTLSL SPGERATLSC | RAS--QSIRS- ----SFLA | WYQQKPGQ APRLLIY | G-------- ASSRAT | GIPDRFSGSGSG-- TDFTLTISRLEPEDFAVYYC | QQYGN----------- ------------SPWA | FGQGT KVEIK |
| iPS:435 851 | 21-225_191D3 | VK3|A27/ JK1 | | EIVLTQSPGTILSL SPGESATLSC | RAG--QSIRT- ----NFLA | WYQQQPGQ APRLLIY | G-------- ASSRAT | GIPDRFSGSGSG-- TDFTLTINRLEPEDFAVYYC | QQYGS----------- ------------SPWT | FGQGT KVEIK |
| iPS:435 865 | 21-225_191A5 | VK3|A27/ JK1 | | EIVLTQSPGTLSL SPGERATLSC | RAS--QSVSS- ----RFLA | WYQQKPGQ APRLLIY | G-------- ASNRAT | GIPDRFSGSGSG-- TDFTLTINRLEPEDFAVYYC | QQYGG----------- -----------SPPWT | FVQGT KVEIK |
| iPS:435 905 | 21-225_190A3 | VK3|A27/ JK1 | | EIMLTQSPGTLSL SPGERATLSC | RAS--QNIRS- ----NFLA | WYQQKPGQ APRLLIY | G-------- ASSRAT | GIPDRFSGSVSG-- TDFTLTINRLEPEDFAVYYC | QQYGN----------- ------------SPWT | FGQGT KVEIK |
| iPS:435 911 | 21-225_190B4 | VK3|A27/ JK1 | | EIVLTQSPGTLSL SPGERATLSC | RAS--QSIRS- ----SFLA | WYQQKPGQ APRLLIY | G-------- ASSRAT | GIPDRFSGSGSG-- TDFTLTISRLEPEDFAVYYC | QQYGN----------- ------------SPWA | FGQGT KVEIK |
| iPS:435 913 | 21-225_190A7 | VK3|A27/ JK1 | | EIVLTQSPGTLSL SPGERATLSC | RAS--QSVRS- ----NFLA | WHQQKPGQ APRLLIY | G-------- AYRRAT | GIPDRFSGSGSG-- TDFTLTINRLEPEDFAVYYC | QQYGN----------- ------------SPWT | FGQGT KVEIK |
| iPS:435 939 | 21-225_191H7 | VK3|A27/ JK1 | | EIVLTQSPGTLSL SPGESATLSC | RAG--QSIRT- ----DFLA | WYQQQPGQ APRLLIY | G-------- PSSRAT | GIPDRFSGSGSG-- TDFTLTINRLEPEDFAVYFC | QQYGS----------- ------------SPWT | FGQGT KVEIN |
| iPS:435 967 | 21-225_192B3 | VK3|A27/ JK1 | | EIVLTQSPGTLSL SPGERATLSC | RAS--QSVRS- ----SFLA | WYQQKPGQ APRLLIY | G-------- ASSRAT | GIPDRFSGSGSG-- TDFTLTISRLEPEDFAVYYC | QQYGN----------- ------------SPWA | FGQGT KVEIK |
| iPS:435 973 | 21-225_192H3 | VK3|A27/ JK1 | | EIVLTQSPGTLSL SPGERATLSC | RAS--QSVSS- ----NFLA | WYQQKPGQ APRLLIF | G-------- ASSRAT | GIPDRFSGSGSG-- TDFTLTINRLEPEDFAVYYC | QQYGI----------- ------------SPWT | FGQGT KVEIK |
| iPS:435 995 | 21-225_192F8 | VK3|A27/ JK1 | | EIVLTQSPGTLSL SPGERATLSC | RAS--QSISS- ----SYLA | WYQQKPGQ APRLLIY | G-------- ASSRAT | GIPARFSGSGSG-- TDFTLTISRLEPEDFAVYYC | QQYES----------- ------------SPWT | FGQGT KVEIK |
| iPS:436 007 | 21-225_192G1 2 | VK3|A27/ JK1 | | EIVLTQSPGTLSL SPGERATLSC | RAS--QSVRS- ----DFLA | WLQQKPGQ APRLLIY | G-------- VSRRAT | GIPDRFSGSGSG-- TDFTLTINRLEPEDFAVYYC | QQYGN----------- ------------SPWT | FGQGT KVEIK |
| iPS:436 009 | 21-225_193A1 | VK3|A27/ JK1 | | EIVLTQSPGTLSL SPGERATLSC | RAS--QSVRS- ----NFLA | WHQQKPGQ APRLFIY | G-------- ASRRAT | GIPDRFSGSGSG-- TDFTLTINRLEPEDFAVYYC | QQYGN----------- ------------SPWT | FGQGT KVEIK |
| iPS:436 011 | 21-225_193B1 | VK3|A27/ JK1 | | EIVLTQSPGTLSL SPGDRATLSC | RAS--QSVRS- ----NFLA | WHQQKPGQ APRLLIY | G-------- ASRRAT | GIPDRFSGSGSG-- TDFTLTINRLEPEDFAVYFC | QQYGN----------- ------------SPWT | FGQGT KVEIK |
| iPS:436 015 | 21-225_193D3 | VK3|A27/ JK1 | | EIVLTQSPGTLSL SPGERATLSC | RAS--QSIRS- ----SFLA | WYQQKPGQ APRLLIY | G-------- ASNRAT | GIPDRFSGSGSG-- TDFTLTISRLEPEDFAVYYC | QQYGN----------- ------------SPWA | FGQGT KVEIK |
| iPS:436 017 | 21-225_193F3 | VK3|A27/ JK1 | | EIVLTQSPGTLSL SPGESATLSC | RAG--QSIRT- ----DFLV | WYQQQPGQ APRLLIY | G-------- ASSRAT | GFPERFSGSGSG-- TDFTLTINRLEPEDFAVYYC | QQYGS----------- ------------SPWT | FGQGT KVEIN |

| iPS:436 027 | 21-225_193E6 | VK3|A27/JK1 | | EIVLAQSPGTLSL SPGERATLSC | RAS--QSVRS- ----GYLA | WYQQKPGQ APRLLIY | G-------- ASSRAT | GIPDRFGGSGSG-- TDFTLTISRLEPEDFAVYYC | QQYES----------- ------------SPWT | FGQGT KVEIK |
| iPS:436 029 | 21-225_193H6 | VK3|A27/JK1 | | EIVLTQSPGTLSL SPGESATLSC | RAG--QSIRT- ----NFLA | WYQQQPGQ APRLLIY | G-------- ASSRAT | GIPDRFSGSGSG-- TDFTLTINRLEPEDFAVYFC | QQYGS----------- ------------SPWT | FGQGT KVEIN |
| iPS:436 035 | 21-225_193C8 | VK3|A27/JK1 | | EIVLTQSPGTLFL SPGERATLSC | RAS--QSIRS- ----SFLA | WYQQKPGQ APRLLIY | G-------- ASSRAT | GIPDRFSGSGSG-- TDFTLTISRLEPEDFAVYYC | QQYGN----------- ------------SPWA | FGQGI KVEVK |
| iPS:436 037 | 21-225_193D8 | VK3|A27/JK1 | | EIVLKQSPGTLFL SPGERATLSC | RAS--QSIRT- ----NFLA | WYQQKPGQ APRLLIY | G-------- ASSRAT | GIPDRFSGSGSG-- TDFTLTINRLEPEDFAVYYC | QQYGN----------- ------------SPWT | FGQGT KVEIK |
| iPS:436 041 | 21-225_193G8 | VK3|A27/JK1 | | EIVLTQSPGTLSL SPGERATLSC | RAS--QSVRT- ----NFLA | WHQQKPGQ APRLLIY | G-------- ASRRAT | GIPDRFSGSGSG-- TDFTLTINRLEPEDFALYYC | QQYGN----------- ------------SPWT | FGQGT KVEIK |
| iPS:436 047 | 21-225_193B10 | VK3|A27/JK1 | | EIVLTQSPGTLSL SPGERATLSC | RAS--PSVSS- ----SYLA | WYQQKPGQ APRLVIY | G-------- ASRRAT | GIPDRFRGSGSG-- TDFTLTISRLEPEDFAVYYC | QQYGS----------- -----------SPPWT | FGQGT KVEIK |
| iPS:436 049 | 21-225_193B12 | VK3|A27/JK1 | | EIVLTQSPGTLSL SPGERATLSC | RAS--QSIRS- ----SFLA | WYQQKPGQ APRLLIY | D-------- ASSRAT | GIPDRFSGSGSG-- TDFTLTISRLEPEDFAVYYC | QQYGN----------- ------------SPWA | FGQGT KVEIK |
| iPS:436 062 | 21-225_194E5 | VK3|A27/JK1 | | EIVLTQSPGTLSL SPGESATLSC | RAG--QSIRT- ----NFLA | WYQQKPGQ APRLLIY | G-------- ASSRAT | GIPDRFSGSGSG-- TDFTLTINRLEPKDFAVYYC | QQYGS----------- ------------SPWT | FGQGT KVEIK |
| iPS:436 064 | 21-225_194E6 | VK3|A27/JK1 | | EIVLTQSPGTLSL SPGERATLSC | RAS--QSIRS- ----NFLA | WYQQKPGQ APRLLIY | G-------- ASSRAT | GIPDRFSGSVSG-- TDFTLTINRLEPEDFAVYYC | QQYGS----------- ------------SPWT | FGQGT KVEIK |
| iPS:436 072 | 21-225_194C10 | VK3|A27/JK1 | | EIVLTQSPGTLSL SPGERATLSC | RAS--PSVNS- ----GYLA | WYQQKPGQ TPRLLIF | G-------- ASSRAT | GIPDRFSASGSG-- ADFTLTISRLEPEDFAVYFC | QQYES----------- ------------SPWT | FGQGT KVEIK |
| iPS:436 080 | 21-225_195B1 | VK3|A27/JK1 | | EIVLTQSPGTLSL SSGERATLSC | RAS--PSVNS- ----NYLA | WYQQKPGQ TPRLLIY | G-------- ASNRAT | GVPDRFSASGSG-- TDFTLTIRRLEPEDFAVYYC | QQYES----------- ------------SPWT | FGQGT KVEIK |
| iPS:436 088 | 21-225_195C8 | VK3|A27/JK1 | | EIVLTQSPGTLSL SPGERATLSC | RAS--QSIRS- ----SFLA | WYQQKPGQ APRLLIY | G-------- AFSRAT | GIPDRFSGSGSG-- TDFTLTISRLEPEDFAVYYC | QQYGN----------- ------------SPWA | FGQGT KVEIK |
| iPS:436 122 | 21-225_196G10 | VK3|A27/JK1 | | EIVLTQSPGTLLL SPGERATLSC | RAS--PSVSN- ----SFLA | WYQQKPGQ APRLLIY | G-------- ASSRAT | GIPDRFSGSGSG-- TDFTLTNSRLEPEDFAVYYC | QQYGS----------- -----------SPPWT | FGQGT KVELK |
| iPS:436 134 | 21-225_196H12 | VK3|A27/JK1 | | EIVLTQSPGTLFL SPGERATLSC | RAS--QSVRS- ----NFLA | WHQQKPGQ APRLLIY | G-------- AYRRAT | GIPDRFSGSGSG-- TDFTLTINRLEPEDFAVYYC | QQYGN----------- ------------SPWT | FGQGT KVEIK |
| iPS:436 146 | 21-225_197F4 | VK3|A27/JK1 | | EIVLTQSPGTLSL SPGEGATLSC | RAS--QSIRS- ----SFLA | WYLQKPGQ APRLLIY | G-------- ASSRAT | GIPDRFSGSGSG-- TDFTLTISRLEPEDFAVYYC | QQYGN----------- ------------SPWA | FGQGT KVEIK |
| iPS:436 177 | 21-225_198B1 | VK3|A27/JK1 | | EIVLTQSPGTLSL SPGESATLSC | RAS--QSIRT- ----NFLA | WYQQQPGQ APRLLIY | G-------- ASSRAT | GIPDRFSGSGSG-- TDFTLTINRLEPEDFAVYYC | QQYGS----------- ------------SPWT | FGQGT KVEIK |
| iPS:436 179 | 21-225_198E1 | VK3|A27/JK1 | | EIVLTQSPGTLSL SPGERATLSC | RAS--QSIRS- ----NFLA | WYQQKPGQ APRLLIY | G-------- AFSRAT | GIPDRFSGSGSG-- TDFTLTISRLEPEDFAVYYC | QQYGN----------- ------------SPWT | FGQGT KVEIK |
| iPS:436 181 | 21-225_198C2 | VK3|A27/JK1 | | EIVVTQSPGTLLL YSEERSTLSC | RAS--QSVRS- ----SYLA | WYQQKPGQ APRLLIC | G-------- AFSRAS | GIPDRFSGSGSG-- TDFTLTISRLEPEDFAVYYC | QQYGN----------- ------------SPWT | LGHAT KVEIK |
| iPS:436 195 | 21-225_198G10 | VK3|A27/JK1 | | EIVLTQSPGTLSL SPGERATLSC | RAS--QSIRS- ----SFLA | WYQQKPGQ APRLLIY | G-------- ASSRAT | GIPDRFSGSGSG-- TDFTLTISRLEPEDFAVYYC | QQYGN----------- ------------SPWA | FGQGT KVEIK |
| iPS:436 197 | 21-225_199C2 | VK3|A27/JK1 | | EIVLTQSPGTLSL SPGERATLSC | RAS--QSIRS- ----SFLA | WYQQKPGQ APRLLIY | G-------- ASSRAT | GIPDRFSGSGSG-- TDFTLTINRLEPEDFAVYYC | QQYGN----------- ------------SPWA | FGQGT KVEIK |

| | | | | | | | | | | |
|---|---|---|---|---|---|---|---|---|---|---|
| iPS:436 207 | 21-225_199C7 | VK3\|A27/ JK1 | | EIVLTQSPGTLSL SPGERATLSC | RAS--QSIRT- ----NFLA | WYQQKPGQ APRLLIY | G-------- ASSRAT | GIPDRFSGSGSG-- TDFTLTINRLEPEDFAVYYC | QQYGN---------- ------------SPWT | FGQGT KVEIK |
| iPS:436 210 | 21-225_199G1 1 | VK3\|A27/ JK1 | | EIVVTQSPGTLSL SPGERATLSC | RAS--QSVRS- ----SYLA | WYQQKPGQ APRLLIY | G-------- AFSRAT | GIPDRFSGSGSG-- TDFTLTISRLEPEDFAVYYC | QQYGN---------- ------------SPWT | FGHGT KVEIK |
| iPS:436 226 | 21-225_200F10 | VK3\|A27/ JK1 | | EIVLTQSPGTLSL SPGERATLSC | RAS--QNIRS- ----SFLA | WYQQKPGQ APRLLIY | G-------- ASSRAT | GIPDRFSGSGSG-- TDFTLTINRLEPEDFAVYYC | QQYGN---------- ------------SPWA | FGQGT KVEIK |
| iPS:436 232 | 21-225_201E1 | VK3\|A27/ JK1 | | EIVLTQSPDTLSL SPGERATLSC | RAS--PSINS- ----GFLA | WYQQKPGQ APRLLIY | G-------- ASSRAT | GIPDRFSGSGSG-- TDFTLTISRLEPEDFAMFHC | HQYET---------- ------------SPWT | FGQGT KVEIK |
| iPS:436 238 | 21-225_201B2 | VK3\|A27/ JK1 | | EIVLTQSPGTLSL SPGERATLSC | RAS--QSVSS- ----NYLA | WYQQRPGQ APRLLIY | G-------- ASSRAT | GIPDRFSGSGSG-- TDFTLTISRLEPEDFAVYFC | QQYEN---------- ------------SPWT | FGQGT KVEIK |
| iPS:436 256 | 21-225_202D9 | VK3\|A27/ JK1 | | EIVLTQSPDTLSL SPGERATLSC | RAS--QSVNS- ----GYLA | WYQQKPGQ SPRLLIY | G-------- ASSRAT | GIPDRFSGSGSG-- TDFTLTISRLEPEDFAVHYC | QQYET---------- ------------SPWT | FGQGT KVEIK |
| iPS:436 302 | 21-225_205G7 | VK3\|A27/ JK1 | | EIVLTQSPGTLSL SPGERATLSC | RAS--QSVFS- ----NYLA | WYQQKPGQ APRLLIY | G-------- ASSRAA | GIPDRFSGSGSG-- TDFTLTISRLEPENFAVYYC | QQYES---------- ------------SPWT | FGQGT KVEIK |
| iPS:436 310 | 21-225_202D1 1 | VK3\|A27/ JK1 | | EIVLTQSPGTLSL SPGERATLSC | RAS--QSINS- ----NYLA | WYQRKPGQ APRVLIY | G-------- ASSRAT | GIPDRFSGSGSG-- TDFTLTISRLEPEDFAVFYC | QQYEN---------- ------------SPWT | FGQGT KVEIK |
| iPS:436 336 | 21-225_208B5 | VK3\|A27/ JK1 | | EIVLTQSPGTLSL SPGERATLSC | RAS--QSVSS- ----NYLA | WYQQRPGQ APRLLIY | G-------- ASSRAT | GIPDRFSGSGSG-- TDFTLTISRLEPEDFAVYFC | QHYEN---------- ------------SPWT | FGQGT KVEIK |
| iPS:436 340 | 21-225_208A9 | VK3\|A27/ JK1 | | EIVLTQSPGTLSL SPGERATLSC | RAS--QSVSN- ----NYLA | WYQQKPGQ APRVLIY | G-------- ASSRAT | GIPDRFSGSGSG-- TDFTLTISRLEPEDFAVYYC | QHYHS---------- ------------SPWT | FGQGT KVEIK |
| iPS:436 472 | 21-225_220E1 | VK3\|A27/ JK1 | | EIVLTQSPGTLSL SPGERATLSC | RAS--QSISR- ----SHLV | WYQQKPNQ APRLLLY | V-------- TSSRAT | GIPDRFSGSGSG-- TDFTLTIRSLEPEDFAMYYC | QQYGS---------- ------------SPWT | FGQGT KVEIK |
| iPS:436 506 | 21-225_222C7 | VK3\|A27/ JK1 | | EIVLTQSPGTLSL SPGERATLSC | RAS--QSVYS- ----NYLA | WYQQKPGQ APRLLIY | G-------- ASSRAT | GIPDRFSGSGSG-- TDFTLAISRLEPEDFTIYYC | QQYED---------- ------------SPWT | FGQGT KVEIK |
| iPS:436 580 | 21-225_225E7 | VK3\|A27/ JK1 | | EIVLTQSPGTLSL SPGERATLSC | RAS--QSVYS- ----SYLA | WYQQKPGQ APRLLIY | G-------- ASSRAT | GIPDRFSGSGSG-- TDFTLTISRLEPEDFAVYYC | QQYGT---------- ------------SPRT | FGQGT KVEIK |
| iPS:437 324 | 21-225_75C2 | VK3\|A27/ JK1 | | EIVLTQSPGTRYL SPGERATLSC | RAS--QSVYS- ----SYLA | WYQQKPGQ APRLLIY | G-------- ASSRAT | GIPDRFSGSGSG-- TDFALTISRLEPEDCAVYYC | QHYDN---------- ------------SPWT | FGRGT KVEIK |
| iPS:437 328 | 21-225_75D3 | VK3\|A27/ JK1 | | EIVLTQSPGTLYL SPGERATLSC | RAS--QSVYS- ----SYLA | WYQQKPGQ APRLIIY | G-------- ASSRST | GIPDRFSGSGCG-- TDFALTISRVEHEDCAVYYC | QHYDN---------- ------------SPWT | FGQGT KVEIK |
| iPS:437 332 | 21-225_75F3 | VK3\|A27/ JK1 | | EIVLTQSPGTLYL SPGERATLSC | RAS--QSVYS- ----SYLA | WYQQKPGQ APRLLIY | G-------- ASSRAT | GIPDRFSGSGCG-- TDFALTISRLEHEDFAVYYC | QHYDN---------- ------------SPWT | FGQGT KVEIK |
| iPS:437 340 | 21-225_75G9 | VK3\|A27/ JK1 | | EIALTQSPGTLSL SPGERATLSC | RAS--PSVDS- ----SYLA | WYQQKPGQ APRLLIY | G-------- ASSRAP | GIPDRFSGSGSG-- TDFTLTISRLEPEDFAVYYC | QQYES---------- ------------SPWT | FGQGT KVEIK |
| iPS:437 344 | 21-225_75G12 | VK3\|A27/ JK1 | | EIVLTQSPGTLYL SPGERATLSC | RAS--QSVYS- ----SYLA | WYQQKPGQ APRLLIY | G-------- ASSRAT | GIPDRFSGSGSG-- TDFALTISRVEPEDCAVYYC | QHYDN---------- ------------SPWT | FGQGT KVEIK |
| iPS:437 350 | 21-225_74A3 | VK3\|A27/ JK1 | | EIVLTQSPGTLSL SPGERATLSC | RAS--QSVYS- ----SYLA | WYQQKPGQ APRLLIY | G-------- ASSRST | GIPDRFSGSGCG-- TDFALTISRLEPEDFAVYYC | QHSDN---------- ------------SPWT | FGQGT KVEIK |
| iPS:437 369 | 21-225_74D6 | VK3\|A27/ JK1 | | EIVLTQSPGTLSL SPGERATLSC | RAS--QSVYS- ----SYLA | WYQQKPGQ APRLLIY | G-------- ASSRAT | GIPDRFSGSGCG-- TDFALTISRLEPEDFAVYYC | QHYDN---------- ------------SPWT | FGQGT KVEIK |
| iPS:437 383 | 21-225_74H8 | VK3\|A27/ JK1 | | EIVLTQSPGTLSL SPGERATLSC | RAS--QSFSS- ----SYLA | WYQQKPGQ APRLLIY | G-------- ASSRAT | GIPDRFSGSGSG-- TDFTLTISRLEPEDFAVYYC | QQYG----------- ----------SSRT | FGQGT KVEIK |
| iPS:451 122 | 21-225_200A1 | VK3\|A27/ JK1 | | EIVLTQSPGILSL YPGERATLSC | RAS--QSVNS- ----NYLA | WYQQRPGQ APSLLIY | G-------- ASSRAT | CILDRFSGSGCG-- TDFTLTISRLEPEDFAVYCC | QQYEI---------- ------------SPWT | FGQGT KVESK |

| | | Germline | | K_FR1 | K_CDR1 | K_FR2 | K_CDR2 | K_FR3 | K_CDR3 | K_FR4 |
|---|---|---|---|---|---|---|---|---|---|---|
| iPS:392 864 | 21-225_23B9 | VK3\|A27/ JK1 | | EIVLIQSPGTLSL SPGERATLSC | RAS--QNVYS- ----SYLA | WYQQKPGQ TPRLLIY | G-------- ASSRAS | GIPDRFSGSGSG-- TDFTLTISRLEPEDFAVYYC | QQYGS----------- -----------SPRT | FGQGT KVEIK |
| | VK1\|A30/JK 4 | | 32136 | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:468 812 | 21-225_48H4 | VK1\|A30/ JK4 | | DIQMIQFPSSLSA SVGDRVTITC | RAS--RDIR-- ----NDLG | WYQQKPGK APKRLIY | A-------- ASSLQS | GVPSRFSGSGSG-- TEFTLTISSLQPEDFATYYC | LQHYS----------- -----------YPLT | FGGGT KVEIK |
| iPS:468 824 | 21-225_73G6 | VK1\|A30/ JK4 | | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:468 818 | 21-225_190C8 | VK1\|A30/ JK4 | | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFETYYC | LQHND----------- -----------YPFT | FGGGT KVEIK |
| iPS:468 840 | 21-225_200H9 | VK1\|A30/ JK4 | | DIQMIQSPSSLSA SVGDRVTITC | RAS--QGIR-- ----NDLG | WYQQKPGK APKRLIY | A-------- ASSLQS | GIPSRFSGSGSG-- TEFTLTISSLQPEDFATYYC | LQHNS----------- -----------YPLT | FGGGT KVEIK |
| iPS:468 868 | 21-225_74A1 | VK1\|A30/ JK4 | | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHDS----------- -----------YPLT | FGGGA KVEIK |
| iPS:392 920 | 21-225_29G4 | VK1\|A30/ JK4 | | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIT |
| iPS:433 899 | 21-225_43C3 | VK1\|A30/ JK4 | | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVGIT |
| iPS:433 921 | 21-225_44C3 | VK1\|A30/ JK4 | | DIQMIQSPSSLSA SVGDRVTITC | RAS--QGIR-- ----NDLG | WYQQKPGK APKRLIY | A-------- ASSLQS | GVPSRFSGGGSG-- TEFTLTISSLQPEDFATYYC | LQHSS----------- -----------YPLT | FGGGT KVEIK |
| iPS:433 947 | 21-225_44E12 | VK1\|A30/ JK4 | | DIQMIQSPSSLSA SVGDRVTITC | RTS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:433 963 | 21-225_46B1 | VK1\|A30/ JK4 | | DIQMIQSPSSLSA SVGDRVTITC | RAS--QGIR-- ----NDLG | WYQQKPGK APKRLIY | A-------- ASSLQG | GVPSRFSGSGSG-- TEFTLTISSLQPEDFATYYC | LQHNS----------- -----------YPLT | FGGGT KVEIK |
| iPS:433 969 | 21-225_46F3 | VK1\|A30/ JK4 | | DIQMIQSPSSLSA SVGDRVTITC | RAS--QGIR-- ----KDLG | WYQQKPGK APKRLIY | A-------- ASSLQS | GVPSRFSGSGSG-- TEFSLTISSLQPEDFATYYC | LQHNS----------- -----------YPLT | FGGGT KVEIK |
| iPS:433 975 | 21-225_46C6 | VK1\|A30/ JK4 | | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIT |
| iPS:433 977 | 21-225_46D8 | VK1\|A30/ JK4 | | DIQMIQSPSSLSA SVGDRVTITC | RAS--QGIR-- ----KDLG | WYQQKPGK APKRLIY | A-------- ASSLQS | GVPSRFSGSGSG-- TEFSLTISSLQPEDFATYYC | LQHNS----------- -----------YPLT | FGGGT KVEIK |
| iPS:433 983 | 21-225_47A1 | VK1\|A30/ JK4 | | DIQMIQSPSSLSA SVGDRVTITC | RAS--QDIR-- ----NDLG | WYQQKPGK APKRLIY | A-------- AFSLQS | GVPSRFSGSRSG-- TEFTLTISSLQPEDFATYYC | LQHNS----------- -----------YPLT | FGGGT KVEIK |
| iPS:433 987 | 21-225_47A5 | VK1\|A30/ JK4 | | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:434 013 | 21-225_48D12 | VK1\|A30/ JK4 | | DIQMIQSPSSLSA SVGDRVTITC | RAS--QGIR-- ----NDLG | WYQQKPGK APKRLIY | A-------- ASTLQS | GVPSRISGSGSG-- TEFTLTISSLQPEDFATYYC | LQHNS----------- -----------YPLT | FGGGT KVEIK |
| iPS:434 019 | 21-225_49A1 | VK1\|A30/ JK4 | | DIQMIQSPSSLSA SVGDRVTITC | RAS--QGIR-- ----NDLD | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:434 029 | 21-225_49C6 | VK1\|A30/ JK4 | | DIQMIQSPSSLSA SVGDRVTITC | RAS--QGIR-- ----NDLG | WYQQKPGK APKRLIY | A-------- ASSLQS | GFPSRFSGSGSG-- TEFTLTISSLQPEDFATYYC | LQHNS----------- -----------YPLT | FGGGT KVEIK |
| iPS:434 043 | 21-225_50G10 | VK1\|A30/ JK4 | | DIQMIQSPSSLSA SVGDRVTITC | RAS--QGIR-- ----NNLG | WYQQKPGK VPKRLIY | A-------- ASSLQS | GVPSRFSGSGSG-- TEFTLTISSLQPEDFATYYC | LQYNS----------- -----------YPFT | FGGGT KVESK |
| iPS:434 077 | 21-225_51F11 | VK1\|A30/ JK4 | | DIQMIQSPSALSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPFT | FGGGT KVEIK |
| iPS:434 081 | 21-225_52B2 | VK1\|A30/ JK4 | | DIQMIQSPSSLSA SVGDRVTITC | RAS--QGIR-- ----NDLG | WYQQKPGK APRRLIY | A-------- ASFLQS | GVPSTFSGSGSG-- TEFTLTISSLQPEDFATYYC | LQHNS----------- -----------YPLT | FGGGT KVEIK |

| | | | | | | | | | |
|---|---|---|---|---|---|---|---|---|---|
| iPS:434 105 | 21-225_53D2 | VK1|A30/ JK4 | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNR---------- ------------YPLT | FGGGT KVEIK |
| iPS:434 119 | 21-225_53E6 | VK1|A30/ JK4 | DIQMIQSPSSLSA SVGARVTITC | RAS--QGIR-- ----NDLG | WYQQNPGK APKRLIY | A-------- ASNLQS | GVPSRFSGSGSG-- TEFTLTISSLQPEDFATYYC | LQHNR---------- ------------YPLT | FGGGT KVEIK |
| iPS:434 141 | 21-225_54C6 | VK1|A30/ JK4 | DIQMIQSPSSLSA SVGARVTITC | RAS--QGIR-- ----NDLG | WYQQNPGK APKRLIY | A-------- ASNLQS | GVPSRFSGSGSG-- TEFTLTISSLQPEDFATYFC | LQHNR---------- ------------YPLT | FGGGT KVEIK |
| iPS:434 159 | 21-225_55B8 | VK1|A30/ JK4 | DIQMIQSPSSLSA SVGDRVTITC | RAS--QAIR-- ----NDLG | WYQQKPGK APKRLIH | A-------- AFRLQS | GVPSRFSGSGSG-- TEFTLTISSLQPEDFATYYC | LQHNS---------- ------------YPLT | FGGGT KVGIK |
| iPS:434 179 | 21-225_56F1 | VK1|A30/ JK4 | DIQMIQSPSSLSA SVGDRVTITC | RAS--QDIR-- ----NNLG | WYQQKPGK APKRLIN | A-------- ASSLQS | GVPSRFSGSGSG-- TEFTLTISSLQPEDFATYYC | LQDNS---------- ------------HPFT | FGGGT KVEIK |
| iPS:434 217 | 21-225_60E8 | VK1|A30/ JK4 | DIQMIQSPSSLSA SVGDRVTFTC | RAS--QGIR-- ----NDLG | WFQQKPGK APKRLIY | A-------- ASSLQS | GVPSRFSGSGSG-- TEFTLTISSLQPEDFATYYC | LQHSS---------- ------------YPLT | FGGGT KVEIK |
| iPS:434 249 | 21-225_62E2 | VK1|A30/ JK4 | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHSN---------- ------------YPLT | FGGGT RVEIK |
| iPS:434 253 | 21-225_62E4 | VK1|A30/ JK4 | DIQMIQSPSSLSA SVGDRVTITC | RAS--QGIR-- ----NDLG | WYQQKPGK APKRLIY | A-------- AFSLQS | GVPSRFSGSGSG-- TEFTLTISSLQPEDFATYYC | LQHNS---------- ------------YPLT | FGGGT KVEIK |
| iPS:434 313 | 21-225_59E6 | VK1|A30/ JK4 | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS---------- ------------YPLT | FGGGT RVEIK |
| iPS:434 337 | 21-225_64E1 | VK1|A30/ JK4 | DIQMIQSPSSLSA SVGDRVTITC | RAS--QGIR-- ----NDLG | WYQQKPGK APKRLIY | A-------- ASSLQS | GVPSRFSGSKSG-- TEFTLTISSLQPEDLATYYC | LQHNS---------- ------------YPLT | FGGGT KVEIK |
| iPS:434 411 | 21-225_68F11 | VK1|A30/ JK4 | DIQMIQSPSSLSA SVGDRVTITC | RAS--QGIR-- ----NDLG | WYQQKPGK APKRLIY | A-------- ASSLQS | GVPSRFSGSGSG-- PEFTLSISSLQPEDFATYYC | LQHNS---------- ------------YPFT | FGGGT KVEIK |
| iPS:434 413 | 21-225_68D12 | VK1|A30/ JK4 | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHST---------- ------------YPLT | FGGGT KVEIK |
| iPS:434 433 | 21-225_70E8 | VK1|A30/ JK4 | DIQMIQSPSSLST SVGDRVTITC | RAS--QGIR-- ----KDLG | WYQQKPGK APKRLIY | A-------- ASSLQS | GVPSRFSGSGSG-- TEFTLTISSLQPEDFATYYC | LQHNR---------- ------------YPLT | FGGGT KVEIK |
| iPS:434 439 | 21-225_70E12 | VK1|A30/ JK4 | DIQMIQSPSSLSA SVGDRVTITC | RAS--QGIN-- ----NNLN | WFQQKPGK APKRLIY | A-------- ASSLQS | GVPSRFSGSGSG-- TEFTLTISSLQPEDFATYYC | LQHNS---------- ------------YPLT | FGGGS KVEIK |
| iPS:434 469 | 21-225_74E4 | VK1|A30/ JK4 | DIQMIQSPSSLSA SVGDRVTITC | RAS--QGIR-- ----NDLG | WYQQKPGK APKRLIY | A-------- ASTLQS | GVPSRFSGSGSG-- TEFTLTISGLQPEDFATYHC | LQHSN---------- ------------YPLT | FGGGT KVEIK |
| iPS:434 503 | 21-225_74D7 | VK1|A30/ JK4 | DIQMIQSPSSLSA SVGDRVTITC | RAS--QGIR-- ----NDLG | WYQQKPGK APKRLIY | A-------- ASSLQS | GVPSRFSGSGFG-- TEFTLTISSLQPEDFATYYC | LQHSN---------- ------------YPLT | FGGGT KVEIK |
| iPS:435 251 | 21-225_96A3 | VK1|A30/ JK4 | DIQMIQSPSSLSA SVGDRVTITC | RAS--QGIR-- ----NDLG | WYQQKPGK APKRLIY | A-------- ASTLQS | GVPSRFSGSGSG-- TEFTLTISGLQREDFATYHC | LQHSN---------- ------------YPLT | FGGGT KVEIK |
| iPS:435 293 | 21-225_146F1 | VK1|A30/ JK4 | DIQMIQSPSSLSA SVGDRVTITC | RAS--QGIR-- ----NDLG | WYQQKPGK APKRLIY | A-------- ASSLQS | GVPSRFSGGGSG-- TEFTLTISGLQREDFATYYC | LQHST---------- ------------YPLT | FGGGT KVEIK |
| iPS:435 311 | 21-225_146H9 | VK1|A30/ JK4 | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS---------- ------------YPLT | FGGGT KVEIK |
| iPS:435 313 | 21-225_146G1 1 | VK1|A30/ JK4 | DIQMIQSPSSLSA SVGDRVTITC | RAS--QDIR-- ----NNFG | WYQQKPGK APKRLIY | A-------- ASSLQS | GVPLRFSGSGSG-- TEFTLTISSLQPEDFATYYC | LQHDS---------- ------------YPLT | FGGGT |
| iPS:435 361 | 21-225_148E1 1 | VK1|A30/ JK4 | DIQMIQSPSSLSA SVGDRVTITC | RAS--QGIR-- ----NDLG | WYQQKPGK APKRLIS | A-------- ASSLQS | GVPSRFTGSGSG-- TEFTFTISSVQPEDFATYYC | LQHRN---------- ------------YPLT | FGGGT KVEIK |
| iPS:435 363 | 21-225_148F12 | VK1|A30/ JK4 | DIQMIQSPSSLSA SVGDRVTITC | RAS--QGIR-- ----NALG | WYQQKPGK APKRLIY | A-------- ASSLQS | GVPSRFSGSGSG-- TEFTLTISSLQPEDFATYFC | LQHNS---------- ------------YPLT | FGGGT KVEIK |

| | | | | | | | | | |
|---|---|---|---|---|---|---|---|---|---|
| iPS:435 367 | 21-225_149G1 | VK1\|A30/ JK4 | DIQMIQSPSSLSA SVGARVTITC | RAS--QGIR-- ----NDLG | WYQQKPGK APKRLIY | A-------- ASNLQS | GVPSRFSGSGSG-- TEFTLTISSLQPEDFATYYC | LQHNS----------- ------------YPLT | FGGGT KVEIK |
| iPS:435 377 | 21-225_149G5 | VK1\|A30/ JK4 | DIQMIQSPSSLSA SVGDRVTITC | RAS--QGIR-- ----NALG | WFQQKPGK APKRLIY | A-------- ASSLQS | GVPSRFSGSGSG-- TEFTLTISSLQPEDFATYYC | LQHNS----------- ------------YPLT | FGGGT KVEIK |
| iPS:435 397 | 21-225_149F12 | VK1\|A30/ JK4 | DIQMIQSPSSLSA SVGDRVTITC | RAS--QGIR-- ----NDLG | WYQQKPGK APKRLIY | A-------- ASNLQS | GVPSRFSGSGSG-- TEFTLTISSLQPEDFAIYYC | LQHNS----------- ------------YPLT | FGGGT KVEIK |
| iPS:435 407 | 21-225_150E7 | VK1\|A30/ JK4 | DIQMIQSPSSLSA SVGDRVTITC | RAS--QGIR-- ----NDLG | WYQQKPGK APKRLIY | A-------- ASNLQS | GVPLRFSGSGSG-- TDFTLTISSLQPEDFAAYYC | LQHNS----------- ------------YPLT | FGGGT KVEIK |
| iPS:435 449 | 21-225_152H9 | VK1\|A30/ JK4 | DIQMIQSPSSLSA SVGDRVTITC | RAS--QGIR-- ----NDLG | WYQQKPGK APKRLIY | A-------- ASSLQS | GVPSRFTGSGSG-- TEFTFTISSLQPEDFATYYC | LQHSN----------- ------------YPLT | FGGGT KVEIK |
| iPS:435 499 | 21-225_156G1 | VK1\|A30/ JK4 | DIQMIQSPSSLSA SVGDRVTITC | RAS--QGIR-- ----NDLG | WYQQKPGK APKRLIY | A-------- ASSLQS | GVPSRFSGSGSG-- TEFTFTISSLQPEDFATYYC | LQHSN----------- ------------YPLT | FGGGT KVEIK |
| iPS:435 549 | 21-225_158H5 | VK1\|A30/ JK4 | DIQMIQSPSFLFA SVGDRVTITC | RAS--QGMR-- ----IDLG | WYQQKPGK APKRLIY | R-------- ASSLQS | GVPSRFSGSGCG-- TEFTLTISSVQREDFASYYC | VQHNS----------- ------------YPLT | FGGGT KVEIK |
| iPS:435 587 | 21-225_160H3 | VK1\|A30/ JK4 | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHSN----------- ------------YPLT | FGGGT QVESK |
| iPS:435 599 | 21-225_160B10 | VK1\|A30/ JK4 | DIQMIQSPSSPSA SVGDRVTITC | RAS--QGIR-- ----NDLG | WYQQKPGT APKRLIY | A-------- ASSLQS | GVPSRFSGSGSG-- TEFTLTISNLQPEDFATYYC | LQHSS----------- ------------YPLT | FGGGT KVEIK |
| iPS:435 663 | 21-225_169B1 | VK1\|A30/ JK4 | DIQMIQSPSSLSA SVGDRVTITC | RAS--QGIR-- ----NDLG | WYQQKPGK APKRLIG | A-------- ESSLQS | GVPSRFSGSGSG-- TEFTLTISGLQPEDFATYYC | LQHYS----------- ------------YPLT | FGGGT KVEIK |
| iPS:435 669 | 21-225_169F9 | VK1\|A30/ JK4 | DIQMIQSPSSLSA SVGDRVTITC | RAS--QGIR-- ----NDLG | WYQQKPGK APKRLIF | A-------- ASSLQS | GVPSRFSGSGSG-- TEFTLTISSLQPEDFAIYYC | LQHYS----------- ------------YPLT | FGGGT KVEIK |
| iPS:435 693 | 21-225_170G4 | VK1\|A30/ JK4 | DIQMIQSPSSLSA SVGDRVTITC | RAS--QGIR-- ----NDLG | WYHQKPGK APKRLIY | A-------- ASTLQS | GVPSRFSGSGSG-- TEFTLTISSLQPEDFATYYC | LQHYS----------- ------------YPLT | FGGGT KVEIK |
| iPS:435 695 | 21-225_170D5 | VK1\|A30/ JK4 | DIQMIQSPSSLSA SVGDRVTITC | RAS--QGIR-- ----NDLG | WYQEKPGK APKRLIY | A-------- ASSLQN | GVPSRFSGSGSG-- TEFTLTISSLQPEDFATYYC | LQHYS----------- -----------FPLT | FGGGT KVEIK |
| iPS:435 697 | 21-225_170G5 | VK1\|A30/ JK4 | DIQMIQSPSSLSA SVGDRVTITC | RAS--QGIR-- ----TDLG | WFQQKPGK APKRLIY | T-------- ASSLQS | GVPSRFSGSGSG-- TEFTLTISSLQPEDFATYYC | LQHYS----------- ------------YPLT | FGGGT KVEIK |
| iPS:435 703 | 21-225_170D11 | VK1\|A30/ JK4 | DIQMIQSPSSLSA SVGDRVTITC | RAS--QGIR-- ----NDLG | WYQQKPGK APKHLIY | A-------- ASSLQN | GVPSRFSGSGSG-- TEFTLTISSLQPEDFATYYC | LQHYS----------- -----------FPLT | FGGGT KVEIR |
| iPS:435 705 | 21-225_171C3 | VK1\|A30/ JK4 | DIQMIQSPSSLSA SVGDRVTITC | RAS--QGIR-- ----TDLG | WFQQKPGK APKRLIY | T-------- ASSLQS | GVPSRFSGSGSG-- TEFTLTISSLQPEDFATYYC | LQHYS----------- ------------YPLT | FGGGT KVEIK |
| iPS:435 709 | 21-225_171A4 | VK1\|A30/ JK4 | DIQMIQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHYS----------- ------------YPLT | FGGGT KVEIK |
| iPS:435 721 | 21-225_172B3 | VK1\|A30/ JK4 | DIQMIQSPSSLSA SVGDRVTITC | RAS--QGIR-- ----NDLG | WYQQKPGK APKRLIG | A-------- ESSLQS | GVPSRFSGSGSG-- TEFTLTISSLQPEDFATYYC | LQHYS----------- ------------YPLT | FGGGT KVEIK |
| iPS:435 725 | 21-225_172G8 | VK1\|A30/ JK4 | DIQMIQSPSSLSA SVGDRVTITC | RAS--QGVR-- ----NDLG | WYQQKPGK APKHLIY | A-------- ASSLQN | GVPSRFSGSGSG-- TEFTLTISSLQPEDFATYYC | LHHYS----------- -----------FPLT | FGGGT KVEIK |
| iPS:435 735 | 21-225_173H12 | VK1\|A30/ JK4 | DIQMIQSPSSLSA SVGDRVTITC | RAS--QGIR-- ----NDLG | WYQQKPGK APKRLIY | T-------- TSSLQS | GVPSRFSGSGSG-- TEFTLTISSLQPEDFASYYC | LQHYS----------- -----------FPNT | FGGGT KVEIK |
| iPS:435 743 | 21-225_175G1 | VK1\|A30/ JK4 | DIQMIQSPSSLSA SVGDRVTITC | RAS--QGIR-- ----TDLG | WYQQKPGK APKRLIY | T-------- ASSLQS | GVPSRFSGSGSG-- TEFTLTISSLQPEDFATYYC | LQHYS----------- ------------YPLT | FGGGT KVEIK |

| ID | Clone | Germline | FR1 | CDR1 | FR2 | CDR2 | FR3 | CDR3 | FR4 |
|---|---|---|---|---|---|---|---|---|---|
| iPS:435 761 | 21-225_176B1 1 | VK1\|A30/ JK4 | DIQMIQSPSSLSA SVGDRVTITC | RAS--QGIR-- ----NDLG | WFQQKPGK APKRLIY | A-------- ASSLQS | GVPSRFSGSGSG-- TEFTLTLSSLQPEDFATYYC | LQHYS----------- -----------YPLT | FGGGT KVEIK |
| iPS:435 779 | 21-225_178B1 0 | VK1\|A30/ JK4 | DIQMIQSPSSLSA SVGDRVTITC | RAS--QGIR-- ----NDLG | WYQQKPGK APKHLIY | A-------- ASSLQN | GVPSRFSGSGSG-- TEFTLTISSLQPEDFATYYC | LHHYS----------- -----------FPLT | FGGGT KVEIK |
| IPS:436 023 | 21-225_193A5 | VK1\|A30/ JK4 | DIQMIQSPSSLFA SVGDRVIISC | RAS--QGIR-- ----NDLG | WYQQYPGK APKRVIY | A-------- ASSLQS | GVPSRFSGSGFG-- TEFTITISSVQPEDFETYYC | LQHND----------- -----------FPFT | FGGGT KVEIK |
| IPS:436 033 | 21-225_193E7 | VK1\|A30/ JK4 | DIQMIQSPSSLSA SVGDRVTITC | RAS--QGIR-- ----NDLG | WYQQKPGK APKRLIY | S-------- ASSLQR | GVPSRFSGSGSG-- TEFTLTISSLQPEDFATYYC | LQHKR----------- -----------YPLT | FGGGT KVEIK |
| IPS:436 120 | 21-225_196C1 0 | VK1\|A30/ JK4 | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQYNS----------- -----------YPLT | FGGGT KVEIK |
| IPS:436 199 | 21-225_199E3 | VK1\|A30/ JK4 | DIQMIQSPSSLSA SVGDRVTITC | RAS--QGIR-- ----NDLG | WYQQKPGK APKRLIS | S-------- ASSLQR | GVPSRFSGSGSG-- TEFTLTISSLQPEDFATYYC | LQHKR----------- -----------YPLT | FGGGT KVEIK |
| IPS:436 228 | 21-225_200F12 | VK1\|A30/ JK4 | DIQMIQSPSSLSA SVGDRVTITC | RAS--QGIR-- ----NDLG | WYQQKPGR APKRLIY | S-------- ASSLHT | GVPSRFSGSGSG-- TEFTLTISSLQPEDFATYYC | LQHKS----------- -----------YPLT | FGGGT KVEIK |
| IPS:436 230 | 21-225_201A1 | VK1\|A30/ JK4 | DIQMIQSPSSLSA SVGDRVTITC | RAS--QGIR-- ----NDLG | WYQQKPGK APKRLIY | S-------- ASILQR | GVPSRFSGSGSG-- TEFTLTISSLQPEDFATYYC | LQHKS----------- -----------YPLT | FGGGT KVEVK |
| IPS:436 242 | 21-225_201A1 0 | VK1\|A30/ JK4 | DIQMIQSPSSLSA SVGDRVTITC | RAS--QGIR-- ----NDLG | WYQQKPGR APKRLIY | S-------- TSSLHS | GVPSRFSGSGSG-- TEFTLTISSLQPEDFATYYC | LQHNS----------- -----------YPLT | FGGGT KVEIK |
| IPS:436 286 | 21-225_204H8 | VK1\|A30/ JK4 | DIQMIQSPSSLSA SVGDRVTITC | RAS--QGIR-- ----NDLG | WYQQKPGK APKRLIY | S-------- ASSLQS | GVPSRFSGRGSG-- TEFTLTVSSLQPEDFATYYC | LQHNS----------- -----------YPLT | FGGGT KVEIK |
| IPS:436 308 | 21-225_205H8 | VK1\|A30/ JK4 | DIQMIQSPSSLSA SVGDRVTITC | RAS--QGIR-- ----NDLG | WYQQKQGK APKLLIY | S-------- ASFLQR | GVPSRFSGSGSG-- TEFTLTISSLQPEDSAAYYC | LQHNS----------- -----------YPLT | FGGGT TVKIK |
| IPS:436 526 | 21-225_224A1 | VK1\|A30/ JK4 | DIQMIQSPSSLSA SVGDRVTITC | RAS--QGIE-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| IPS:436 528 | 21-225_224B1 | VK1\|A30/ JK4 | DIQMIQSPSSLSA SVGDRVTITC | RAS--QGIS-- ----NDLG | WYQQKPGK APKRLIY | A-------- ASSLQS | GVPSRFSGSGSG-- TEFTLTISSLQPEDFATYHC | LQHNS----------- -----------YPPT | FGGGT KVEIK |
| IPS:436 538 | 21-225_224C3 | VK1\|A30/ JK4 | DIQMIQSPSSLSA SVGDRVTITC | RAS--QDIR-- ----NDLG | WYQQKPGK APKRLIY | A-------- TSSLQS | GVPSRFSGSGSG-- TEFTLTISSLQPEDFATYYC | LQHNS----------- -----------YPLT | FGGGT KVEIK |
| IPS:436 556 | 21-225_224D1 0 | VK1\|A30/ JK4 | DILMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| IPS:437 220 | 21-225_55H6 | VK1\|A30/ JK4 | DIQMIQSPSSLSA SVGDRVTITC | RAS--QGIR-- ----NDLG | WYQQKPGK APKRLIY | G-------- ASSLQS | GVPSRFSGSGSG-- TEFTLTISSLQPEDFAAYYC | LQRDS----------- -----------YPFT | FGGGT KVEIK |
| IPS:437 346 | 21-225_75H7 | VK1\|A30/ JK4 | DIQMIQSPSSRYA SVGDRVTINS | RAS--QGIR-- ----NDLG | WYQQKPGK SPQRLIY | D-------- ASSLQS | GVPSRFSGSGSG-- TEFTLTISSVQPEDFGVYYC | IQHSN----------- -----------YPLT | FGGGT KVEIK |
| IPS:472 730 | 21-225_14B1_ LC1 | VK1\|A30/ JK4 | DIQMIQSPSYLSA SVGDRVTITC | RAS--QDIR-- ----DNLG | WYQQKPGK APKRLIY | T-------- AYSLQS | GVPSRFSGSGSG-- TEFTLTISSLQPEDFATYYC | LQHYN----------- -----------YPLT | FGGGT KVEIK |
| iPS:392 622 | 21-225_17H8 | VK1\|A30/ JK4 | DIQMIQSPSSLSA SVGDRVTITC | RAS--QGIR-- ----NDLG | WYQQKPGK APKRLIY | G-------- ASSLQS | GVPSRFSGSGSG-- TEFTLTISSLQPEDFTTYYC | LQHNS----------- -----------YPLT | FGGGT KVEIK |
| IPS:392 624 | 21-225_17H12 | VK1\|A30/ JK4 | DIQMIQSPSSLSA SVGDRVTITC | RAS--QDIR-- ----NDLG | WYQQKAGK APKRLIN | A-------- ASSLQS | GVPSRFSGIGSG-- TEFTLTITGLQPEDFATYYC | LQHYS----------- -----------YMFT | FGGGT KVEIK |

| | | | FR1 | CDR1 | FR2 | CDR2 | FR3 | CDR3 | FR4 |
|---|---|---|---|---|---|---|---|---|---|
| iPS:392 628 | 21-225_20C2 | VK1\|A30/JK4 | DIQMTQSPSSLSA SVGDRVTITC | RAS--QGIR-- ----NDLG | WYQQKPGK APKRLIF | A-------- ASSLQS | GVPSRFSGSGSG-- TEFTLTISSLQPEDFATYYC | LQHAS----------- -----------YPLT | FGGGT KVEIE |
| iPS:392 630 | 21-225_20E5 | VK1\|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPLT | FGGGT KVEIK |
| iPS:392 638 | 21-225_17F9 | VK1\|A30/JK4 | DIQMTQSPSSLSA SVGDRVTITC | RAS--QVIR-- ----NDLG | WYQQKPGK APKRLIY | A-------- VSSLQS | GVPSRFSGSGSG-- TEFTLIINSLQPEDFATYYC | LQHNT----------- -----------YPLT | FGGGT KVEFK |
| iPS:392 640 | 21-225_18A1 | VK1\|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLIISSLQPEDFATYYC | LQHNS----------- ------------YPLT | FGGGT KVEIK |
| iPS:392 642 | 21-225_18C6 | VK1\|A30/JK4 | DIQMTQSPSSLSA SVGDRVTITC | RTS--QGIR-- ----NDLG | WYQQKPGK APKRLIY | A-------- ASSLQS | GVPSRFSGSGSG-- IEFTLIISSLQPEDFATYYC | LQHSS----------- -----------YPLT | FGGGT KVEIK |
| iPS:392 644 | 21-225_19E1 | VK1\|A30/JK4 | DIQMTQSPSSLSA SVGDRVTITC | RAS--QGIR-- ----NDLG | WYQQKPGK APKRLIY | A-------- ASNLQS | CVPSRFSGSGSG-- IEFTLTISSLQPEDFATYYC | LQHNS----------- -----------FPLT | FGGGT KVEIK |
| iPS:392 646 | 21-225_20G2 | VK1\|A30/JK4 | DIQMTQSPSSLSA SVGDRVTITC | RAS--QGIR-- ----NDLG | WYQQKPGK APKRLIY | A-------- ASSFQS | GVPSRFSGSGSG-- IEFTLTISSLQPEDFASYYC | LQHNS----------- -----------YPLT | FGGGT KVEIK |
| iPS:392 654 | 21-225_17A10 | VK1\|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:392 656 | 21-225_1F2 | VK1\|A30/JK4 | DIQMTQSPSSLSA SVGDRVTITC | RAS--QGIR-- ----NDLG | WYQQKPGK APKRLIY | A-------- ASSVQS | GVPSRFSGSGSG-- TEFTLTISSLQPEDFAIYYC | LQHNS----------- -----------YPLT | FGGGT KVEIK |
| iPS:392 658 | 21-225_18E8 | VK1\|A30/JK4 | DIQMTQAPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:392 666 | 21-225_16F11 | VK1\|A30/JK4 | DIQMTQSPSSLSA SVGDRVTITC | RAS--QGIR-- ----NDLG | WYQQKPGK APKRLIY | A-------- ASSVQS | GVPSRFSGSGSG-- TEFTLTISSLQPEDFATYYC | LQHNS----------- -----------YPLT | FGGGT KVEIK |
| iPS:392 676 | 21-225_19F3 | VK1\|A30/JK4 | DIQMTQSPSSLSA SVRDRVTITC | RAS--QGIR-- ----NDLG | WYQQKPGK APKRLIY | A-------- VSSLQS | GVPSRFSGSGSG-- TEFTLTISSLQPEDFATYYC | LQHAS----------- -----------YPLT | FGGGT KVEIK |
| iPS:392 680 | 21-225_20A7 | VK1\|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFSLTISSLQPEDFATYYC | LQHNS----------- -----------YPLT | FGGGT KVEIK |
| iPS:392 700 | 21-225_16E12 | VK1\|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:392 706 | 21-225_18A3 | VK1\|A30/JK4 | DIQMTQSPSSLSA SVGDRVTITC | RAS--QGIR-- ----NDLG | WYQQKPGK APKRLIY | V-------- ASSLQS | GVPSRFNGSGSG-- TEFTLTISSLQPEDFATYYC | LQHSS----------- -----------YPLT | FGGGT KVEIK |
| iPS:392 716 | 21-225_17B5 | VK1\|A30/JK4 | DIQMTQSPSSLSA SVGDRVTITC | RAS--QGIR-- ----NDLG | WYQQKPGK APKRLIY | A-------- ASNLQS | GVPSRFSGSGSG-- TEFSFTISSLQPEDFATYYC | LQHNS----------- -----------YPLT | FGGGT KVEIK |
| iPS:392 744 | 21-225_20D5 | VK1\|A30/JK4 | DIQMIQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPFT | FGGGT KVEIK |
| iPS:392 750 | 21-225_20A10 | VK1\|A30/JK4 | DIQMTQSPSSLSA SVGDRVTITC | RAS--QGIR-- ----NDLG | WYQQKQGK APKRLIY | A-------- ASSLQS | GVPSRFSGSGSG-- TEFTLTISSLQPEDFATYYC | LQHNR----------- -----------YPLT | FGGGT KVEIK |
| iPS:392 772 | 21-225_20E12 | VK1\|A30/JK4 | DIQMTQSPSSLSA SVGDRVTITC | RAS--QGIR-- ----NDLG | WYQQKPGK APKRLIF | A-------- ASSLQS | GVPSRFSGSGSG-- TEFTLTISS-QPEDFATYYC | LQHNS----------- -----------YPFT | FGGGT KVEIK |
| iPS:392 774 | 21-225_21F3 | VK1\|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TDFILTISS-QPEDFATYYC | LQHSS----------- -----------YPLT | FGGGT KVEIK |
| iPS:392 780 | 21-225_22B7 | VK1\|A30/JK4 | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNT----------- -----------YPLT | FGGGT KVEIK |
| iPS:392 788 | 21-225_20C8 | VK1\|A30/JK4 | DIQMTQSPSSLSA SVGDRVTITC | RAS--QGIR-- ----NDLG | WYQKKPGK APKRLIN | A-------- ASSLQS | GVPSRFSGSGSG-- TEFTLTISSLQPEDFATYYC | LQDNS----------- -----------YPFT | FGGGT KVEIT |
| iPS:392 794 | 21-225_21H3 | VK1\|A30/JK4 | DIQMTQSPSSLSA SVGDRVTITC | RAS--QGIR-- ----NDLG | WYQQKPGK APKRLIY | A-------- ASSVQT | GVPSRFSGSGSG-- TEFTLTISSLQAEDLAIYYC | LQHNS----------- -----------YPLT | FGGGT KVEIK |

| | | | | | | | | | |
|---|---|---|---|---|---|---|---|---|---|
| iPS:392 800 | 21-225_22D12 | VK1|A30/ JK4 | | DIQMIQSPSSLSA SVGDRVTITC | RAS--QGIR-- ----NDLG | WYQQKPGK APKRLIY | A-------- ASSLQS | GVPSRFSGSGSG-- TDFTLTISSLQPEDFATYYC | LQHST----------- ------------YPLT | FGGGT KVEIK |
| iPS:392 810 | 21-225_20H12 | VK1|A30/ JK4 | | DIQMTQSPSSLSA SVGDRVTITC | RAS--QGIR-- ----NDLD | WYQQKPGK APKRLIY | A-------- ASSLQS | GVPSRFSGSGSG-- TEFTLTISSLQPEDFATYYC | LQHNS----------- ------------YPLT | FGGGT KVEIK |
| iPS:392 820 | 21-225_23D1 | VK1|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTVSSLQPEDFATYYC | LQHSS----------- ------------YPLT | FGGGT KVEIK |
| iPS:392 822 | 21-225_23C8 | VK1|A30/ JK4 | | DIQMTQSPSSRSA SVGDRVTITC | RAS--QDIR-- ----NDLG | WYQQKPGR APKRLIN | A-------- ASSVQS | GVPSRFSGSGSG-- TEFTLTISSLQPEDFVIYYC | LQHNS----------- ------------YPLT | FGGGT KVEIK |
| iPS:392 824 | 21-225_24E5 | VK1|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHSN----------- ------------YPLT | FGGGT KVEIK |
| iPS:392 834 | 21-225_22C1 | VK1|A30/ JK4 | | DIQMTQSPSSLSA SVGDRVTITC | RTS--QGIR-- ----NDLG | WYQQKPGK APKRLIY | A-------- ASSLQS | GVPSRFSGSGSG-- TEFTLTISSLQPEDFATYYC | LQHST----------- ------------YPLT | FGGGT KVEIK |
| iPS:392 838 | 21-225_22G8 | VK1|A30/ JK4 | | DIQMTQSPSSLFA SVGDRVTITC | RTS--QGIR-- ----NDLG | WYQQKPGK APKRLIY | A-------- ASSLQS | GVPSKFSGSGSG-- TEFTLSISSLQPEDFATYYC | LQHNS----------- ------------YPLT | FGGGT KVEIK |
| iPS:392 850 | 21-225_20H10 | VK1|A30/ JK4 | | GIQMTQSPSSLSA SVGDRVTITC | RAS--QGIK-- ----NNLG | WYQQKPGK GPKCLIY | A-------- ASSLQS | GVPSRFSGSGSG-- TEFTLTISSLQPEDFATYYC | LQHNS----------- ------------YPLT | FGGGT KVEIK |
| iPS:392 854 | 21-225_21E5 | VK1|A30/ JK4 | | DIQMTQSPSSLSA SVGDRVTITC | RAS--QGIR-- ----NDLG | WYQQKPGK APKRLIY | A-------- TSSLQS | GVPSRFSGSGSG-- TEFTLTISSLQPEDFATYYC | LQHNS----------- ------------YPLT | FGGGT KVEIK |
| iPS:392 858 | 21-225_22H4 | VK1|A30/ JK4 | | DIQMTQSPSSLSA SVGDRVTITC | RAS--QGIR-- ----NDLG | WYQQKPGK APKRLIY | A-------- ASSVQS | GVPSRFSGSGSG-- TEFTLTISSLQPEDFALYYC | LQHNS----------- ------------YPLT | FGGGT KVEIK |
| iPS:392 866 | 21-225_23H11 | VK1|A30/ JK4 | | DIQMTQSPSSLSA SVGDRVTITC | RAS--QGIR-- ----NDLD | WYQQKPGK APKRLIY | A-------- ASSLQS | GVPSRFSGSGSG-- TEFTLTISSLQPEDFATYYC | LQHNR----------- ------------YPLT | FGGGT KVEIK |
| iPS:392 870 | 21-225_20G9 | VK1|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHST----------- ------------YPLT | FGGGT KVEIK |
| iPS:392 880 | 21-225_22F9 | VK1|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPLT | FGGGT KVEIK |
| iPS:392 882 | 21-225_23A3 | VK1|A30/ JK4 | | DIQMTQSPSSLSA SVGDRVTITC | RAS--QGIR-- ----NDLG | WYQQKPGK APKRLIY | A-------- ASSVQS | GVPSRFSGSGSG-- TEFTLTISSLQPEDFAIYFC | LQHNS----------- ------------YPLT | FGGGT KVEIK |
| iPS:392 896 | 21-225_21G7 | VK1|A30/ JK4 | | DIQMTQSPSSLSA SVGDRVTITC | RAS--QGVR-- ----NDLG | WYQQKPGK APQRLIY | A-------- ASSLQS | GVPSRFNGSGSG-- TEFTLTISSLQPEDFATYYC | LQHSS----------- ------------YPLT | FGGGT KVEIK |
| iPS:392 900 | 21-225_22F2 | VK1|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPLT | FGGGT KVEIK |
| iPS:392 904 | 21-225_22G9 | VK1|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHAS----------- ------------YPLT | FGGGT KVEIK |
| iPS:392 942 | 21-225_30E9 | VK1|A30/ JK4 | | DIQMTQSPSSLSA SVGDRVTITC | RAS--QDIR-- ----DDLG | WYQQKPGK APRRLIY | G-------- ASSLQS | GVPSRFSGSGSG-- TEFTLTISSLQPEDFATYYC | LQHTS----------- ------------YPPT | FGGGT KVEIK |
| iPS:392 944 | 21-225_31H5 | VK1|A30/ JK4 | | DIQMTQSPSSLSA SVGDRFTISF | RAS--QDIR-- ----SDLG | WYQQKPGK AHKRIIY | A-------- ASSLQS | GVPSRFSVSGSG-- TEFTFTISSMQPDDFSNYYC | IQHII----------- ------------YPPT | FGGGT KVEIK |
| iPS:392 964 | 21-225_31A8 | VK1|A30/ JK4 | | DIQMTQSPSSLSA SVGDRVTITC | RAS--QDIR-- ----SDLG | WYQQKPGK APKRLIY | A-------- VSSLQS | GVPSRFSGSGSG-- TEFTLTISSLQPEDFATYYC | LQHTI----------- ------------YPPT | FGGGT KVEIK |
| iPS:392 980 | 21-225_29H6 | VK1|A30/ JK4 | | DIQMTQSPSSLSA SVGDRVTITC | RAS--QGIR-- ----NDLG | WYQQKSGK TPKRLIY | A-------- ASSLQS | GVPSRFSGSGSG-- TEFTLTISSLQPEDFATYYC | LQHNS----------- ------------YPLT | FGGGT KVEIK |
| iPS:392 982 | 21-225_30D1 | VK1|A30/ JK4 | | DIQMTQSPSSLSA SVGDRVTITC | RAS--QGIR-- ----SDLG | WYQQKPGK APERLIY | A-------- ASSLQS | GVPSRFSGSGSG-- TEFTLTISSLQPEDFTTYYC | LQHTI----------- ------------YPPT | FGGGT KVEIK |
| iPS:392 986 | 21-225_31B8 | VK1|A30/ JK4 | | DIQMTQSPSSLSA SVGDRVTITC | RAS--QDIR-- ----SDLG | WYQQKPGK APKRLIY | G-------- ASSLQS | GVPSRFSGSGSG-- TEFTLTISSLQPEDFATYYC | LQHII----------- ------------YPPT | FGGGT KVEIK |

| | | | | | | | | |
|---|---|---|---|---|---|---|---|---|
| iPS:392 988 | 21- 225_25E6 | VK1\|A30/ JK4 | DIQMTQSPSSLSA SVGDRVTITC | RAS--QGIR-- ----NDLG | WYQRKPGK APKRLIY | A-------- ASSLQS | GVPSRFRGSGSG-- TEFTLTINSLQPEDFATYYC | LQHNS----------- ------------YPLT | FGGGT KVEIK |
| iPS:392 990 | 21- 225_25H10 | VK1\|A30/ JK4 | DIQMTQSPSSLSA SVGDRVTITC | RAS--RGIR-- ----NDLG | WYQQKPGK APKRLIY | A-------- AFSLQS | GVPSRFSGSGSG-- TEFTLTIISLQPEDFATYYC | LQHNS----------- ------------YPLT | FGGGT KVEIK |
| iPS:393 004 | 21- 225_30G11 | VK1\|A30/ JK4 | DIQMTQSPSSLSA SVGDRVTITC | RAS--QDIR-- ----SDLG | WYQQKPGK APKRLIY | A-------- ASSLQS | GVPSRFSGSGSG-- TEFTLTIGSLQPEDFATYFC | LQHTI----------- ------------YPPT | FGGGT KVEIK |
| iPS:393 018 | 21- 225_29B8 | VK1\|A30/ JK4 | DIQMTQSPSSLSA SVGDRVTITC | RAS--QGIR-- ----NDLG | WYQQKPGK APKRLIY | A-------- ASSLQS | GVPSRFSGSRSG-- TEFTLTIISLQPEDFATYYC | LQHNS----------- ------------YPLT | FGGGT KVEIK |
| iPS:393 030 | 21- 225_25H11 | VK1\|A30/ JK4 | DIQMTQSPSSLSA SVGDRVTITC | RAS--QGIR-- ----TDLG | WYQQKPGK APKRLIY | A-------- ASSLQS | GVPSRFSGSGSG-- TEFTLTIISLQPEDFATYYC | LQHNS----------- ------------YPLT | FGGGT KVEIK |
| iPS:393 034 | 21- 225_27F2 | VK1\|A30/ JK4 | DIQMTQSPSSLSA SVGDRVTITC | RAS--QGIR-- ----NDLG | WYQQKPGK APKRLIY | V-------- ASSLQS | GVPSRFSGSGSG-- TEFTLTIISLQPEDFATYYC | LQHNS----------- ------------YPLT | FGGGT KVEIK |
| iPS:393 040 | 21- 225_30E3 | VK1\|A30/ JK4 | DIQMTQSPSSLSA SVGDRVTITC | RAS--QDIR-- ----DDLG | WYQQKPGK APRRLIY | A-------- AFSLQS | GVPSRFSGSGSG-- TEFTLTIISLQPEDFATYYC | LQHTS----------- ------------YPPT | FGGGT KVEIK |
| iPS:393 048 | 21- 225_27C3 | VK1\|A30/ JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTIISLQPEDFATYYC | LQHNR----------- ------------YPLT | FGGGT KVEIK |
| iPS:393 054 | 21- 225_29G8 | VK1\|A30/ JK4 | DIQMTQSPSSLSA SVGDRVTITC | RAS--QGIR-- ----NDLG | WYQLKPGK APKRLIY | A-------- ASSLQS | GVPSRFSGSGSG-- TEFTLTIISLQPEDFATYNC | LQHNS----------- ------------YPLT | FGGGT KVEIK |
| iPS:393 056 | 21- 225_30F3 | VK1\|A30/ JK4 | DIQMTQSPSSLSA SVGDRVTITC | RAS--QGIR-- ----NDLG | WYQQKPGK APKRLIY | T-------- ASSLQS | GVPSRFSGSGSG-- TDFTLTIISLQPEDFASYYC | LQHNS----------- ------------YPFT | FGGGT KVEIK |
| iPS:393 058 | 21- 225_31H3 | VK1\|A30/ JK4 | DIQMTQSPSSLSA SVGDRVTITC | RAS--QGIR-- ----DDLG | WYQQKPGK APRRLIY | A-------- AFSLQS | GVPSRFSGSGSG-- TEFTLTIISLQPEDFATYYC | LQHTS----------- ------------YPPT | FGGGT KVEIK |
| iPS:393 060 | 21- 225_32G12 | VK1\|A30/ JK4 | DIQMTQSPSSLSA SVGDRVTITC | RAS--QDIR-- ----SDLG | WYQQRPGK APKRLIY | A-------- ASSLQS | GVPSRFSGSGSG-- TEFTLTIISLQPEDFATYYC | LQHTI----------- ------------YPPT | FGGGT KVEIK |
| iPS:393 068 | 21- 225_34G9 | VK1\|A30/ JK4 | DIQMTQSPSSLSA SVGDRVTITC | RAS--QDIR-- ----SDLG | WYQQKPGK APKRLIY | A-------- ASSLQS | GVPSRFTGSGSG-- TEFTLTIISLQPEAFAIYYC | LQHTI----------- ------------YPPT | FGGGT KVNIK |
| iPS:393 072 | 21- 225_36C5 | VK1\|A30/ JK4 | DIQMTQSPSSLSA SVGDRVTITC | RAS--QDIR-- ----SDLG | WYQQKPGK APKRLIY | A-------- ASSLQS | GVPSRFTGSGSG-- TEFTLTISSVQPEDFATYYC | LHHPI----------- ------------YPPT | FGGGT KVNIK |
| iPS:393 074 | 21- 225_33B1 | VK1\|A30/ JK4 | DIQMTQSPSSLSA SVGDRVTITC | RTS--QGIR-- ----NDLG | WFQQKPGK APKRLIY | T-------- ASSLQS | GVPSRFSGSGSG-- TEFTLTIISLQPEDFATYYC | LQHNS----------- ------------YPLT | FGGGT KVEVK |
| iPS:393 076 | 21- 225_33A4 | VK1\|A30/ JK4 | DIQMTQSPSSLSA SVGDRVTITC | RAS--QDIR-- ----NDVG | WYQQKPGK APERLIY | A-------- ASSLQR | GVPSRFSGSGSG-- TEFTLTIISLQPEDFARYYC | LQHYS----------- ------------YPPT | FGGGT KVEIK |
| iPS:393 096 | 21- 225_34D11 | VK1\|A30/ JK4 | DIQMTQSPSSLSA SVGDRVTITC | RAS--QDIR-- ----SDLG | WYQQKPGK APKRLIY | A-------- ASSLQS | GVPSRFSGSGSG-- TEFTLTVISLQPEDFATYYC | LQHTI----------- ------------YPPT | FGGGT KVGIK |
| iPS:393 102 | 21- 225_33F1 | VK1\|A30/ JK4 | DIQMTQSPSSLSA SVGDRVTITC | RTS--QDIR-- ----SDLG | WYQQKPGK APKRLIY | A-------- ASSLQS | GVPSRFSGSGSG-- TEFTLTIISLQPEDFAIYYC | LQHTI----------- ------------YPPT | FGGGT KVEIK |
| iPS:393 104 | 21- 225_33A7 | VK1\|A30/ JK4 | DIQMTQSPSSLSA SVGDRVTITC | RAS--QDIR-- ----SDLG | WYQQKPGK APKRLIY | V-------- ASSLQS | GVPSRFSGSGSG-- TEFTLTIISLQPEDFAAYYC | LQHTI----------- ------------YPPT | FGGGT KVEIK |
| iPS:393 106 | 21- 225_34A6 | VK1\|A30/ JK4 | DIQMTQSPSSLSA SVGDRVTITC | RTS--QDIR-- ----NDLG | WYQQKPGK APKRLIY | A-------- ASSLQS | GVPSRFSGSGSG-- TEFTLTVSSLQPEDFATYYC | LQHNS----------- ------------YPPT | FGGGT KVEIK |
| iPS:393 110 | 21- 225_35B7 | VK1\|A30/ JK4 | DIQMTQSPSSLSA SVGDRITITC | RAS--QDIR-- ----SDLG | WYQQKPGK APERLIY | A-------- ASSLQS | GVPSRFSGSGSG-- TEFTLTIISLQPEDFATYYC | LQHTI----------- ------------YPPT | FGGGT KVEIK |
| iPS:393 118 | 21- 225_34H11 | VK1\|A30/ JK4 | DIQMTQSPSSLSA SVGDRVTITC | RAS--QDIR-- ----NDLG | WYQQKPGK APKRLIY | A-------- TSSLQS | GVPSRFSGSGSG-- TEFTLTIISLQPEDFATYYC | LQHNS----------- ------------YPPT | FGGGT KVEIK |
| iPS:393 124 | 21- 225_33G7 | VK1\|A30/ JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTIISLQPEDFATYYC | LQHYS----------- ------------YPPT | FGGGT KVEIK |

| | | | | | | | | | |
|---|---|---|---|---|---|---|---|---|---|
| iPS:393 126 | 21-225_35D1 | VK1\|A30/ JK4 | DIQMTQSPSSLSA SVGDRVTITC | RAS--QDIR-- ----SDLG | WYQQKPGK APKRLIY | A-------- ASSLQS | GVPSRFSGSGSG-- TEFTLTISSLQPEDFATYYC | LQHTI----------- -----------YPPT | FGGGT KVEIK |
| iPS:393 128 | 21-225_35F11 | VK1\|A30/ JK4 | DIQMTQSPSSLSA SVGDRVTITC | RAS--QDIR-- ----SDLG | WYQQKPGK APKRLIY | A-------- ASSLQS | GVPSRFSGSGSG-- TEFTLTISSLQPEDFATYYC | LQHTV----------- -----------YPPT | FGGGT KVEIK |
| iPS:393 146 | 21-225_34G8 | VK1\|A30/ JK4 | DIQMTQSPSSLSA SVGDRVTITC | RAS--QDIR-- ----SDLG | WYQQKPGK APKRLIY | A-------- ASSLQS | GVPSRFSGSGSG-- TEFTLTISSLQPEDFATYYC | LQHTI----------- -----------YPPT | FGGGT KVEIK |
| iPS:393 150 | 21-225_36A5 | VK1\|A30/ JK4 | DIQMTQSPSSLSA SVGDRVTITC | RAS--QGIR-- ----NDLG | WYQQKPGK APKRLIF | A-------- ASSLQS | GVPSRFSGSGSG-- TEFTLTISSLQPEDFATYYC | LHHNS----------- -----------YPPK | FGGGI KVEIT |
| iPS:393 804 | 21-225_5H7 | VK1\|A30/ JK4 | DIQMTQSPSSLSA SVGDRVTITC | RAS--QGIR-- ----NDLG | WYQQKPGK APKRLIY | V-------- TSSLQG | GVPSRFSGSGSG-- TEFTLTISSLQPEDFATYYC | LQHNS----------- -----------YPLT | FGGGT KVEIK |
| iPS:393 806 | 21-225_6A6 | VK1\|A30/ JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHSS----------- -----------YPLT | FGGGT KVEIK |
| iPS:393 808 | 21-225_1A2 | VK1\|A30/ JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------HPLT | FGGGT KVEIK |
| iPS:393 814 | 21-225_7F4 | VK1\|A30/ JK4 | DIQMTQSPSSLSA SVGDRVTITC | RTS--QGIR-- ----NDLG | WYQQKPGK APKRLIY | A-------- ASNLQS | GVPSRFSGSGSG-- TEFTLTISSLQPEDFATYYC | LQHSA----------- -----------YPLT | FGGGT KVEIK |
| iPS:393 816 | 21-225_6D4 | VK1\|A30/ JK4 | DIQMTQSPSSLSA SVGDRVTITC | RAS--QAIR-- ----NDLG | WYQQKPGK APKRLIY | A-------- ASSLQS | GVPSRFSGSGSG-- TEFTLTISSLQPEDFATYYC | LQHSS----------- -----------YPLT | FGGGT KVEIK |
| iPS:393 818 | 21-225_6G12 | VK1\|A30/ JK4 | DIQMTQSPSSLSA SVGDRVTITC | RAS--QGIR-- ----NDLG | WYQQKPGK APKRLIY | A-------- ASTLQS | GVPSRFSGSGSG-- TEFTLTISSLQPEDFATYYC | LQHNS----------- -----------YPLT | FGGGT KVEIK |
| iPS:393 820 | 21-225_8H7 | VK1\|A30/ JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPFT | FGGGT KVEIK |
| iPS:393 826 | 21-225_10G5 | VK1\|A30/ JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:393 828 | 21-225_10H12 | VK1\|A30/ JK4 | DIQMTQSPSSLSA SVGDRVTITC | RAS--QGIR-- ----NDLG | WYQQKPGK APKRLIY | G-------- ASSLQS | GVPSRFSGSGSG-- TEFTLTISSLQPEDFATYYC | LQHNS----------- -----------YPLT | FGGGT KVEIK |
| iPS:393 830 | 21-225_12A1 | VK1\|A30/ JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPLT | FGGGT KVEIK |
| iPS:393 832 | 21-225_14B2 | VK1\|A30/ JK4 | DIQMTQSPSSLSA SVGDRVTITC | RAS--QGIR-- ----NDLG | WYQQKPGK APKRLIY | V-------- TSSLQG | GVPSRFSGSGSG-- TEFTLTISSLQPEDFATYYC | LQHNS----------- -----------YPLT | FGGGT KVEIK |
| iPS:393 854 | 21-225_7H11 | VK1\|A30/ JK4 | DIQMTQSPSSASA SVGVRVTIIC | LAS--QGIR-- ----NDLG | WYQQKPGK APKRIIY | V-------- ACSFQS | GVPSRFSGSGYG-- TEFTLTISIMQPEDFATYYC | LQHNL----------- -----------YPLT | FGGGT KVEIK |
| iPS:393 856 | 21-225_14C2 | VK1\|A30/ JK4 | DIQMTQSPSSLSA CVGDRVIITC | RAS--QGIR-- ----NDLD | WYQQKPGK APKRLIY | A-------- ASSLQS | GVPSRFSGSGSG-- TEFTLTISSVQREDFATYYC | VQHNS----------- -----------YPLT | FGGGT KVEIR |
| iPS:393 866 | 21-225_14E3 | VK1\|A30/ JK4 | DIQMTQSPSSLSA SVGDRVTITC | RAS--QGIR-- ----NDLG | WYQQKPGK APKRIIY | S-------- ASSLQS | GVPSRFIGSGCG-- TEFTLTISSLQPEDFAAYYS | VQHYS----------- -----------YPFT | FGGGT KVEIK |
| iPS:393 872 | 21-225_2A11 | VK1\|A30/ JK4 | DIQMTQSPSSLSP SVGDRVTITC | RAS--QGIR-- ----NDLG | WYQQKPGK AFQRLIS | A-------- ASSLQS | GVPSRFSGSGSG-- TDFTLTISSLQPEDFATYYC | LQHNS----------- -----------YPLT | FGGGT KMEIK |
| iPS:393 874 | 21-225_4C8 | VK1\|A30/ JK4 | DIQMIQSPSFLFA CVGDRVTITC | RAS--QGIR-- ----NDLG | WYQQKPGK APKRLIY | A-------- ASSLQS | GVPSRFSGSVSG-- TEFTLTISSLQPEDFATYYC | LQHNS----------- -----------YPLT | FGGGT KVEIK |
| iPS:393 880 | 21-225_15A1 | VK1\|A30/ JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLVSSLQPEDFASYYC | LHNSS----------- -----------YPVK | FGGGI KVEIT |
| iPS:393 882 | 21-225_15E3 | VK1\|A30/ JK4 | DIQMTQSPSSLSA SVGDRVTITC | RAS--QGIR-- ----NDLG | WYQQKPGK APKRLIY | A-------- ASSSQS | GVPSRFSGSRSG-- TEFTLTISSLQPEDLAAYYC | LQHHS----------- -----------YPLT | FGGGT EVEIY |
| iPS:393 884 | 21-225_16F4 | VK1\|A30/ JK4 | DIQMIQSPSSPSA SVGDRVTITC | RAS--QGIR-- ----NDLG | WYQQKSGK APKRLIY | V-------- ASSLQS | GVPSRFSGSGSG-- TEFTITISSVQPEDFATYYC | IQHNS----------- -----------YPFT | FGGGT KVEIK |

| ID | Clone | Germline | | | | | | | |
|---|---|---|---|---|---|---|---|---|---|
| iPS:393 886 | 21-225_2G9 | VK1|A30/JK4 | DIQMTQSPSSLFA SVGDRVTITC | RAS--QGIR-- ----NDLG | WYQQKPGK APQRLIY | A-------- ASSLQS | GVPSRFSGSGFG-- TEFTLTIISSLQLEDFATYYC | LQHES----------- ------------YPLT | FGGGT KVEIK |
| iPS:393 922 | 21-225_2B2 | VK1|A30/JK4 | DIQMTQSPSSLSA SVGDRVTITC | RAS--QGIR-- ----NDLG | WYQQKPGQ APKRLIY | A-------- ASSLQS | GVPSRFSGSGSG-- TEFTLTIISSLQPEDLATYHC | LQHNS----------- ------------YPLT | FGGGT KVEIK |
| iPS:393 928 | 21-225_4E10 | VK1|A30/JK4 | DIQMTQSPSSLFA SVGDRVTITC | RAS--QGIR-- ----NDLG | WYQQKPGK APKRLIY | A-------- ASSLQS | GVPSRFSGSGFG-- TEFTLTIISSLQLEDFATYYC | LQHDN----------- -----------YPLT | FGGGT KVEIK |
| iPS:393 934 | 21-225_13E6 | VK1|A30/JK4 | DIQVTQSPSSLSA SVGDRVTITS | RAS--QGIR-- ----NDLG | WYQQKPGK APKRLIY | A-------- ASSLQS | GVPSRFSGSGSG-- TEFTLTIISSLQPEDFATYYC | VQHNS----------- -----------YPLT | FGGGT KVAIK |
| iPS:393 958 | 21-225_5H2 | VK1|A30/JK4 | DIQMTQSPSSLSA SVTDRVTITC | RAS--QGIR-- ----NDLG | WYQQKPGK APKRLIY | A-------- ASSLQS | GVPSRFSGSGSG-- IEFSLTISSLQPEDFATYYC | LQHNS----------- -----------YPLT | FGGGT KVEIK |
| iPS:393 962 | 21-225_7H7 | VK1|A30/JK4 | DIQMTQSPSSLFA SVGDRVTITC | RAS--QGIR-- ----NDLG | WYQQKPGK APKRLIY | A-------- ASSLQS | CVTSRFSGSGSG-- IEFTLTIISSLQPEDFATYYC | LQHSS----------- -----------YPFT | FVGGT KVEIK |
| iPS:393 974 | 21-225_7C4 | VK1|A30/JK4 | DIQMTQSPSSLFA SVGDRVTITC | RAS--QGIR-- ----NDLG | WYQQKPGK AHKRLIY | A-------- ASSLQS | GVPSRFSGSGSG-- IEFTLTIISSLQPEDFATYYC | LQHNS----------- -----------YPLT | FGGGT KVEIK |
| iPS:393 976 | 21-225_7E9 | VK1|A30/JK4 | DIQMTQSPSSLFA SVGDRVTITC | RAS--QGIR-- ----NDLG | WYEQKPGK APKRLIY | T-------- ASSLQS | GVPSRFSGSGSG-- TEFTLTIISSLQPEDFATYYC | LQHNS----------- -----------YPLT | FGGGT KVEIK |
| iPS:393 982 | 21-225_6C12 | VK1|A30/JK4 | DIQMTQSPSSLSA SVGDRGTITC | RAS--QGIR-- ----SNLG | WYQQKPGK APKRLIY | A-------- ASSLES | GVPSRFSGSGSG-- TEFTLTIISSLQPEDFATYFC | LQDNS----------- -----------YPFT | FGGGT KVEIK |
| iPS:393 984 | 21-225_4F12 | VK1|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TIFTLTISSLQPEDFATYYC | LQHNS----------- -----------YALT | FGGGT KVEIK |
| iPS:393 990 | 21-225_11G7 | VK1|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPEDFATYYC | LQHSN----------- -----------YPLT | FGGGT MVEIR |
| iPS:393 994 | 21-225_8C9 | VK1|A30/JK4 | DIQMTQSPSSLSA SIGDRVTITC | RAS--QAIR-- ----NDLD | WYQQKPGK APKRLIY | A-------- ASSLQS | GVPSRFSGSGSG-- TEFTLTIISSLQTEDFATYYC | LQHNS----------- ------------YPLT | FGGGT KVEIK |
| iPS:394 002 | 21-225_15G7 | VK1|A30/JK4 | DIQMTQSPSSLSA SVGDRVTITC | RAS--QGIR-- ----NDLG | WYQQKPGK APKRLIY | A-------- ASSLQS | GIPSRFSGSGFG-- TEFTLTIISSLQPEDFATYYC | LQHSN----------- ------------YPLT | FGGGT KVEIK |
| iPS:394 008 | 21-225_15H8 | VK1|A30/JK4 | DIQMTQSPSSLSA SVGDRVTITC | RAS--QGIR-- ----NDLG | WYQQKPGK APKRLIF | A-------- ASSLQS | GVPSRFSGSGSG-- TEFTLTIISSLQPEDFATYYC | LQHNS----------- ------------YPLT | FGGGT KVEIR |
| iPS:394 020 | 21-225_15H10 | VK1|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPEDFASYYC | LQHNS----------- ------------YPLT | FGGGT KVEIN |
| iPS:394 024 | 21-225_16B7 | VK1|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPEDFATYYC | LQHNS----------- -----------YPLT | FGGGT KVEFN |
| iPS:394 037 | 21-225_4F4 | VK1|A30/JK4 | DIQMIQSPSSLSA SVGDRVTITC | RAS--QGIR-- ----NDLG | WYQQKPGK APKRLIY | A-------- ASSVQT | GVPSRFSGSGSG-- TEFTLTIISSLQPEDFAIYYC | LQHNS----------- ------------YPLT | FGGGT KVEIK |
| iPS:394 045 | 21-225_4H4 | VK1|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPEDFAIYYC | LQHNS----------- -----------YPLT | FGGGT KVEIK |
| iPS:394 049 | 21-225_13H5 | VK1|A30/JK4 | DIQMTQSPSSLSA SVGDRVTITC | RAS--QGIR-- ----NDLG | WYQQKPGK APKRLIY | A-------- ASSLQT | GVPSRFSGSGSG-- TEFILTISSLQPEDFATYYC | LQHNS----------- -----------YPLT | FGGGT KVEIK |
| iPS:394 053 | 21-225_11F10 | VK1|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLVSSLQPEDFATYYC | LQHSS----------- -----------YPLT | FGGGT KVEIK |
| iPS:394 057 | 21-225_15H1 | VK1|A30/JK4 | DIQMTQSPSSLSA SVGDRVTITC | RAS--QGIR-- ----NDLG | WYQQKPGK APKRLIY | T-------- ASSLQS | GVPSRFNGSGSG-- TEFTLTIISSLQPEDFATYYC | LQHSS----------- -----------YPLT | FGGGT KVEIK |
| iPS:394 059 | 21-225_9E8 | VK1|A30/JK4 | DIQMTQSPSSLFA SVGDRVTITC | RAS--QGIR-- ----NDLG | WYQQKPGK APKRLIY | A-------- ASSLQS | GVPSRFSGSGSG-- TEFTLTIISSLQPEDFATYYC | LQHNS----------- -----------YPLT | FGGGT KVEIK |
| iPS:394 063 | 21-225_16A1 | VK1|A30/JK4 | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPEDFATYYC | LHHSN----------- -----------YPLT | FGGGT KVEIE |

| | | Germline | | K_FR1 | K_CDR1 | K_FR2 | K_CDR2 | K_FR3 | K_CDR3 | K_FR4 |
|---|---|---|---|---|---|---|---|---|---|---|
| iPS:394 073 | 21-225_15C9 | VK1|A30/JK4 | | DIQMTQSPSSLSA SVGDRVTITC | RAS--QGIR-- ----NDLG | WYQQKPGK APKRLIY | A-------- ASSLQS | GVPSRFSCSGSG-- TEFTLTVSSLQPEDFATYYC | LQHIS----------- ------------YPLT | FGGGT KVEIK |
| iPS:394 075 | 21-225_8D12 | VK1|A30/JK4 | | DIQMTQSPSSLSA SVGDRVTITC | RAS--QGIR-- ----NDLG | WYQQKPGK APKRLIY | A-------- ASSLQS | GVPSRFNGSGSG-- TEFTLTISSLQPEDFATYYC | LQHSS----------- ------------YPLT | FGGGT KVEIK |
| iPS:394 079 | 21-225_11F5 | VK1|A30/JK4 | | DIQMTQSPSSLSA SVGDRVTITC | RAS--QGIR-- ----NDLG | WYQQKPGK APKRLIY | A-------- ASSVQS | GVPSRFSGSGSG-- TEFTLTISSLQPEDFAIYYC | LQHNS----------- ------------YPLT | FGGGT KVEIK |
| iPS:394 091 | 21-225_13H3 | VK1|A30/JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPLT | FGGGT KVEIK |
| iPS:398 528 | 21-225_32G1 | VK1|A30/JK4 | | DIQMTQSPSSLSA SVGDRVTITC | RAS--QDMR-- ----SDLG | WYQQKPGK APKRLIY | A-------- ASSLQS | GVPSRFSGSGSG-- TEFTLTISSLQPEDFATYYC | LQHTI----------- -----------SPPT | FGGGT KVEIK |
| iPS:398 534 | 21-225_33B8 | VK1|A30/JK4 | | DIQMTQSPSSLSA SVGDRVTITC | RAS--QDIR-- ----SDLG | WYQQKPGK APKRLIY | A-------- ASSLQS | GVPSRFSGSGSG-- TEFTLTISSLQPEDFATYFC | LQHTI----------- ------------YPPT | FGGGT KVEIK |
| iPS:398 540 | 21-225_35A6 | VK1|A30/JK4 | | DIQMTQSPSSLSA SVGDRVTITC | RAS--QDIR-- ----SDLG | WYQQKPGK APKRLIY | A-------- TSSLQS | GVPSRFSGSGSG-- TEFTLTISSLQPEDFATYYC | LQHTI----------- ------------YPPT | FGGGT KVEIK |
| iPS:402 219 | 21-225_1C12 | VK1|A30/JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPLT | FGGGT KVAIK |
| iPS:403 868 | 21-225_19D11 | VK1|A30/JK4 | | DIQMTQSPSSLSA SVGDRVTITC | RAS--QGIR-- ----NDLG | WYQQKPGK APKRLIY | A-------- TSSLQS | GVPSRFSGSGSG-- TEFTLTISSLQPEDFATYYC | LQYYS----------- ------------YPLT | FGGGT EVEIK |
| iPS:403 872 | 21-225_8F11 | VK1|A30/JK4 | | DIQMTQSPSSLSA SVGDRVTITC | RAS--QGIR-- ----SDLG | WFQQKPGK APKRLIF | D-------- ASSVQS | GVPSRFSGSGSG-- TEFTLTISSLQPEDFAIYYC | LQHYT----------- ------------YPLT | FGGGT KVEIK |
| | VK2|A18/JK5 | | 32137 | DIVMTQTPLSLSV TPGQPASISC | KSS-- QSLLHSD- GKTYLY | WYLQKPGQ SPQLLIY | E-------- VSSRFS | GVPDRFSGSGSG-- CDFTLKISRVEAEDVGVYYC | MQGIE----------- -----------LPIT | FGGGT RLEIK |
| iPS:468 816 | 21-225_52G8 | VK2|A18/JK5 | | DIVMTQTPLSLSV TPGQPASMSC | KSS-- QSLLHSE- GKTYLY | WYLQKTGQ PPHLLIY | E-------- VSKRLS | GVPDRFSGSGSG-- TDFTLKISRMEAEDVGVYYC | MQSMQ----------- -----------LPII | FGQGT RLEIK |
| iPS:434 021 | 21-225_49C1 | VK2|A18/JK5 | | DIVMTQTPLSLSV TPGQPASISC | KSS-- QSLLHRE- GKTYLY | WYLQKPGQ APQFLIF | E-------- VSHRFS | GVPDRFSGSGSG-- TDFTLKISRVEAEDVGVYYC | MQSIQ----------- -----------IPIT | LGQGT RLEIK |
| iPS:434 025 | 21-225_49G3 | VK2|A18/JK5 | | DIVMTQTPLSLSV TPGQPASMSC | KSS-- QSLLHSE- GKTYLY | WYLQKTGQ PPHLLIY | E-------- VSNRLS | GVPDRFSGSGSG-- TDFTLKISRMEAEDVGVYFC | MQSMQ----------- -----------LPIT | FGQGT RLEIK |
| iPS:434 031 | 21-225_49E7 | VK2|A18/JK5 | | DIVMTQTPLSLSV TPGQPAFMSC | KSS-- QIFLHSE- GKTYLY | WYLQKTGQ PPHLLIY | E-------- VSKRLS | GVPDRFSGSGSG-- TDFTLKISRMEAEDVGVYYC | MQSMQ----------- -----------LPII | FGQGT RLEIK |
| iPS:434 033 | 21-225_49F9 | VK2|A18/JK5 | | DIVMTQTPLSLSV TPGQPASISC | KSN-- QSLVHNE- GKTYLY | WYLQKPGQ PPQLLIF | E-------- VSNRFS | GVPDRFSGSGSG-- TDFTLKISRVEAEDVGVYYC | MQSIQ----------- ------------YPIT | FGQGT RLEIK |
| iPS:434 093 | 21-225_52D10 | VK2|A18/JK5 | | DVMMTQIPLSLSV TPGQPASISC | KSS-- QSLLHSE- GKTYLY | WYLQKPGQ PPQLLIF | E-------- VSNRVS | GVPDRFSGRGSG-- TDFTLKISRVEAEDVGVYYC | MQSIQ----------- ------------YPIT | FGQGT RLEIK |
| iPS:434 151 | 21-225_55C2 | VK2|A18/JK5 | | DVMMTQIPLSLSV TPGQPASISC | KSS-- QSLVHSE- GKTYLY | WYLQKPGQ PPQLLIF | E-------- VSNRVS | GVPDRFSGRGSG-- TDFTLKISRVEAEDVGVYYC | MQSIL----------- ------------YPIT | FGQGT RLEIK |
| iPS:434 161 | 21-225_55F9 | VK2|A18/JK5 | | DIVMTQTPLSLSV TPGQSASISC | KSS-- QSLLHSE- GKTYLY | WYLQKPGQ PPQLLIY | E-------- VSNRFS | GVPDRFSGSGSG-- TDFTLKISRVEAEDVGVYYC | IQSIQ----------- -----------LPIT | FGQGT RLEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:435 329 | 21-225_147A8 | VK2\|A18/ JK5 | | DIVMTQTPLSLSV TPGQPASISC | KTS-- QSLLHSE- GKTYLY | WYLQKPGQ PPQLLIY | E-------- VSNRFS | GVPDRFSGSGSG-- TDFTLKISRVEAEDVGVYYC | MQSI------------ ------------QLIT | FGQGT RLEIK |
| iPS:392 924 | 21-225_32H2 | VK2\|A18/ JK5 | | DIVMTQTPLSLSV TPGQPASISC | RSS-- QSLLHSD- GRTYLY | WYLQKPGQ PPQLLIY | E-------- LSNRFS | GVPDRFSGSGSG-- TDFTLKISRVEAEDVGIYSC | LQSIQ---------- ------------YPIT | FGQGT RLEIK |
| | Germline | | | K_FR1 | K_CDR1 | K_FR2 | K_CDR2 | K_FR3 | K_CDR3 | K_FR4 |
| | VK2\|A18/JK 1 | | 32138 | DIVMTQTPLSLSV TPGQPASISC | KSS-- QSLLHSD- GKTYLY | WYLQKPGQ SPQLLIY | E-------- VSSRFS | GVPDRFSGSGSG-- TDFTLKISRVEAEDVGVYYC | MQGIH---------- -----------LPWT | FGQGT KVEIK |
| iPS:468 822 | 21-225_147E10 | VK2\|A18/ JK1 | | AIVMTQTPLSLSV TPGQPASISC | KSS-- QRLLHGD- GKTYLY | WYLQKPGQ PPHLLIS | E-------- VSNRFS | GVPDRFSGSGSG-- TDFTLKISRVEAEDVGVYYC | MQSIQ---------- -----------VPWT | FGQGT KVEIK |
| iPS:433 917 | 21-225_43E11 | VK2\|A18/ JK1 | | DIVMTQTPLSLSV TPGQPASISC | KSS-- QSLLHSD- GRTYLY | WYLQKPGQ PPQLLIY | E-------- VSNRFS | GVPDRFSGSGSG-- TDFTLKISRVEAEDVGVYYC | MQSIQ---------- -----------LPWT | FGQGT KVEIK |
| iPS:433 965 | 21-225_46F2 | VK2\|A18/ JK1 | | DIVMTQTPLSLIV TPGQPASISC | KSS-- QSLLHGD- GKTYLY | WYLQKPGQ PPQVLIY | E-------- VSNRFS | GVPDRFSGSGSG-- TDFTLKLSRVEAEDVGVYYC | MQSIQ---------- -----------LPWT | FGQGT KVEIK |
| iPS:433 985 | 21-225_47C1 | VK2\|A18/ JK1 | | DIVMTQTPLSLSV TPGQPASISC | TSS-- QSLLHSE- GKTYLY | WYLQKPGQ PPQLLIY | E-------- VSNRFS | GVPDRFSGSGSG-- TDFTLKISRVEAEDVGVFYC | MQSIQ---------- -----------LPWT | FGQGT KVEIK |
| iPS:433 991 | 21-225_47E7 | VK2\|A18/ JK1 | | DIVMTQTPLSLSV TPGQPASISC | KSS-- QSLLHSD- GRTYLY | WYLQKPGQ PPQLLIY | E-------- VSSRFS | GVPDRFSGSGSG-- TDFALKISRVEAEDVGVYYC | MQSTQ---------- -----------LPWT | FGQGT KAEIK |
| iPS:434 345 | 21-225_64H9 | VK2\|A18/ JK1 | | EIVMTQTPLSLSV TPGQPASISC | KSS-- QSLLHGD- GKTYLF | WYLQKPGQ PPQVLIY | E-------- VSNRLC | GVPDRFSGSGSG-- TDFSLKISRVEAEDVGVYYC | MQSIQ---------- -----------VPWT | FGQGT KVEIT |
| iPS:435 297 | 21-225_146B3 | VK2\|A18/ JK1 | | DIVMTQTPLSLSV TPGQPASISC | KSS-- QSLLHGD- GKTYLY | WYLQKPGQ PPQLLIY | E-------- VSNRFS | GVPDRFSGSGSG-- TDFTLKISRVEAEDVGLYHC | MQSIQ---------- -----------LPWT | FGQGT KVEIK |
| iPS:435 341 | 21-225_148B2 | VK2\|A18/ JK1 | | AIVMTQTPLSLSV TPGQPASISC | KSS-- QSLLHGD- GKTYFY | WYLQKPGQ PPQLLIY | E-------- VSHRFS | GVPDRFSGSGSG-- TDFTLKISRVEAEDVGVYYC | MQSIQ---------- -----------IPWT | FGQGT KVEIK |
| iPS:435 357 | 21-225_148G10 | VK2\|A18/ JK1 | | DIVMTQTPLSLSV TPGQPASISC | KSS-- QSLLHGD- GKTYLY | WYLQRPGQ PPQLLIY | E-------- VSNRFS | GVPDRFSGSGSG-- TDFTLKISRVEAEDVGVYYC | MQSIQ---------- -----------LPWT | FGQGT KVEIK |
| iPS:435 365 | 21-225_149F1 | VK2\|A18/ JK1 | | DIVMTQSPLSLFV TPGQPASISY | KSS-- QSLLHGD- GKTYFY | WYLQKPGQ PPQLLIY | E-------- VSHRFS | GVPDRFSGSGSG-- TDFTLKISRVEAEDVGVYYC | MQSIQ---------- -----------IPWT | FGQGT KVEIK |
| iPS:435 413 | 21-225_150B11 | VK2\|A18/ JK1 | | DIVMTQTPLSLSV TPGQPASISC | KSS-- QSLVHGD- GKTYLY | WYLQKPGQ PPQLLIY | E-------- VSNRFS | GVPDRFSGSGSG-- TDFTLKISRVEAEDVGVYYC | MQSIQ---------- -----------LPWT | FGQGT KVEIK |
| iPS:435 423 | 21-225_151G5 | VK2\|A18/ JK1 | | DIVMTQTPLSLSV TPGQPASISC | KSS-- QRLLHGD- GKTYLY | WYLQKPGQ PPQLLLY | E-------- VSNRFS | GVPDRFSGSGSG-- TDFTLKISRVEAEDVGVYYC | MQSIQ---------- -----------VPWT | FGQGT KVEIK |

| | | | | | | | | | | |
|---|---|---|---|---|---|---|---|---|---|---|
| iPS:435 429 | 21-225_151A1 0 | VK2\|A18/ JK1 | | DIVMTQTPLSLSV TPGQPASISC | KSS-- QSLLHGD-GKTYLY | WYLQKPGQ PPQLLIY | E-------- VSHRFS | GVPDRFSGSGSG-- TDFTLKISRVEAEDVGVYYC | MQSIQ----------- ------------IPWT | FGQGT KVEIK |
| iPS:435 441 | 21-225_152F6 | VK2\|A18/ JK1 | | DIVMTQTPLSLSV TPGQPASISC | KSS-- QSLRHGD-GKTYLT | WYLQRPGQ PPQVLIH | E-------- ISKRFT | GVPDRFSGSGSG-- TDFTLNISRVEAEDVGFYYC | MQSIQ----------- ------------VPWT | FGQGT KVEIK |
| iPS:435 457 | 21-225_152C1 1 | VK2\|A18/ JK1 | | DIVMTQAPLSLSV TPGQPASISC | KSS-- QSLLHGD-GKTYLY | WYLQKPGQ PPQILIY | E-------- VSNRFS | GVPDRFSGSGSG-- TDFTLNISRVEAEDFGFYYC | MQSIQ----------- ------------IPWT | FGQGT KVDIK |
| iPS:435 483 | 21-225_153D2 | VK2\|A18/ JK1 | | DIVMTQTPLSLSV TPGQPASISC | KSS-- QSLRHGD-GKTYLT | WYLQRPGQ PPQVLIH | E-------- VSKRFT | GVPDRFSGSGSG-- TDFTLNISRVEAEDVGFYYC | MQSIQ----------- ------------VPWT | FGQGT KVEIK |
| iPS:435 489 | 21-225_155A5 | VK2\|A18/ JK1 | | DIVMTQTPLSLSV TPGQPASISC | KSS-- QSLLHGD-GKTYLY | WYLQKPGQ PPQLLIY | E-------- VSNRFS | GVPDRFSGSGSG-- TDFTLKISRVEAEDVGFYYC | MQSIQ----------- ------------VPWT | FGQGT KVEIK |
| iPS:435 531 | 21-225_157G8 | VK2\|A18/ JK1 | | DIVMTQTPLSLSV TPGQPASISC | KSS-- QSLLHGD-GKTYLY | WYLQKPGQ SPQLLIY | E-------- ISKRFS | GVPDRFSGSGSG-- TDFTLKISRVEAEDVGVYYC | MQSIQ----------- ------------VPWT | FGQGT KVEIK |
| iPS:435 577 | 21-225_160B1 | VK2\|A18/ JK1 | | DIVMTQTPLSLSV TPGQPASISC | KSS-- QSLLHGD-GKTYFY | WYLQKPGQ PPQVLIY | E-------- VSKRFS | GVSERFSGSGSG-- TDFTLKISRVEAEDVGVYYC | MQSIQ----------- -----------IPWT | FGQGT KVEIK |
| iPS:435 601 | 21-225_160G1 0 | VK2\|A18/ JK1 | | AIVMTQTPLSLSV TPGQPASISC | KSS-- QSLLHGD-GKTYLY | WYLQKPGQ PPHLLIY | E-------- VSKRFS | GVPDRFSGSGSG-- TDFTLKISRVEAEDVGLYYC | MQSIQ----------- ------------LPWT | FVQGT KVEIT |
| iPS:435 629 | 21-225_162H6 | VK2\|A18/ JK1 | | DIVMTQTPLSLSV TPGQPASISC | KST-- QSLLHGD-GKTYLY | WYLQKPGQ PPQLLIY | E-------- VSKRFS | GVPERFSGSGSG-- TDFTLKISRVEAEDVGVYYC | KQSIQ----------- ------------LPWT | FGQGT KVEIK |
| iPS:435 655 | 21-225_167E2 | VK2\|A18/ JK1 | | DIVMTQTPLSLSV TPGQPASISC | KSS-- QSLLHGD-GKTYLY | WYLQKPGQ PPHLLIY | E-------- VSKRFS | GVPDRFSGSGSG-- TDFTLKISRVEAEDVGLYHC | MQSIQ----------- ------------LPWT | FGQGT KVEIK |
| iPS:435 657 | 21-225_167H1 0 | VK2\|A18/ JK1 | | DIVMTQTPLSLSV TPGQPASISC | KSS-- QSLLHGD-GKTYLY | WYLQKPGQ PPHLLIY | E-------- VSKRFS | GVPDRFSGSGSG-- TDFTLKISRVEAEDVGLYHC | MQSIQ----------- ------------LPWT | FGQGT KVEIK |
| iPS:435 683 | 21-225_170A1 | VK2\|A18/ JK1 | | EIVMTQTPLFLSV TPGQPASISC | KSS-- QSLLHGD-GKTYLF | WYLQKPGQ PPQVLIY | E-------- VSNRFS | GVPDRFSGSGSG-- TDFTLKISRVEAEDVGVYYC | MQSIQ----------- ------------LPWT | FGQGT KVEIK |
| iPS:435 723 | 21-225_172B7 | VK2\|A18/ JK1 | | DIVMTQTPLSLSV TPGQPASISC | KSS-- QSLLHGD-GKTYFY | WYLQKPGQ PPQVLLF | E-------- VSHRFS | GVPDRFSGGGSG-- TDFTLKISRVEAEDVGVYYC | MQSIQ----------- ----------FPWT | FGQGT RVDIK |
| iPS:435 731 | 21-225_173A1 1 | VK2\|A18/ JK1 | | DIVMTQTPLSLSV TPGQPASISC | KSS-- QSLLHGD-GKTYLY | WYLQKPGQ PPQLLIY | E-------- VSNRFS | GVPDRFSGSGSG-- TDFTLKISRVEAEDVGVFFC | MQSIQ----------- ------------VPWT | FGQGT KVEIK |
| iPS:435 755 | 21-225_176H4 | VK2\|A18/ JK1 | | DIVMTQTPLSLSV TPGQPASISC | KSS-- QSLLHGD-GKTYLY | WYLQKPGQ PPQLLIY | E-------- VSNRFS | GVPDRFSGSGSG-- TDFTLKISRVEAEDVGIYYC | MQSIQ----------- ------------IPWT | FGQGT RVEIK |
| iPS:435 771 | 21-225_177B1 1 | VK2\|A18/ JK1 | | DIVMTQTPLSLSV TPGQPASISC | KSS-- QRLLHGD-GKTYLY | WYLQKPGQ PPQILIY | E-------- VSNRFS | GVPDRFSGSGSG-- TEFTLKISRVEAEDVGVYYC | MQSIQ----------- ------------IPWT | FGQGT KVEIK |

| | | | | | | | | | | |
|---|---|---|---|---|---|---|---|---|---|---|
| iPS:435 781 | 21-225_178G10 | VK2\|A18/JK1 | | EIVMTQTPLSLSV TPGQPASISC | KSS-- QSLLHGD- GKTYLY | WYLQKPGQ PPQLLIY | E-------- VSNRFS | GVPDRLSGGGSG-- TDFTLKISRVEAEDVGIYYC | MQSIQ----------- -----------VPWT | FGQGT KVEIK |
| iPS:435 789 | 21-225_180C4 | VK2\|A18/JK1 | | DIVMTQTPLSLSV TPGQPASISC | KSS-- QSLLHGD- GKTYLY | WYLQKPGQ PPQLLIY | A-------- TSNRFP | GVSDRFSGSGSG-- TDFTLKISRVEAEDVGVYYC | MQSIQ----------- -----------VPWT | FGQGT KVEIK |
| iPS:435 795 | 21-225_181C2 | VK2\|A18/JK1 | | EIVMTQTPLSLSV TPGQPASISC | KSS-- QSLLHGD- GKTYLY | WYLQKPGQ PPQLLIH | E-------- VSNRFS | GVPDRFSGSGSG-- TDFTLKISRVEAEDVGVYYC | MQSIQ----------- -----------VPWT | FGQGT KVEIK |
| iPS:435 807 | 21-225_181C10 | VK2\|A18/JK1 | | DIVMTQTPLSLSV TPGQPASISC | KSS-- QSLLHGD- GKTYLY | WYLQKPGQ PPQLLIY | E-------- VSNRFS | GVPDRFSGSGSG-- TDFTLKISRVEAEDVGVYHC | MQSIQ----------- -----------IPWT | FGQGT KVEIK |
| iPS:435 827 | 21-225_190H11 | VK2\|A18/JK1 | | DIVMTQTPLSLSV TPGQPASISC | KSS-- QSLLHSD- GRTYLY | WYLQKPGQ PPQLLIC | E-------- VSNRFA | GVTDRFSGSGSG-- TDFTLKISRVEAGDVGVYYC | MQSIQ----------- -----------FPWT | FGQGT KVEIK |
| iPS:435 839 | 21-225_191B1 | VK2\|A18/JK1 | | DIVMTQTPLSLSV TPGQPASISC | RSS-- QSLLHSD- GKTYLF | WYLQKPGQ PPQVLIY | E-------- LSNRFS | GVPDRFSGSGSG-- TDFTLKISRVEAEDVGVYYC | MQSFQ----------- -----------LPWT | FGQGT KVEIN |
| iPS:435 853 | 21-225_191E3 | VK2\|A18/JK1 | | DIVMTQTPLSLSV TPGQPASISC | KSS-- QSLLHSD- GRTYLY | WYLQKPGQ PPQLLIC | E-------- VSNRFA | GVTDRFSGSGSG-- TDFTLKISRVEAGDVGVYYC | MQSIQ----------- -----------LPWT | FGQGT KVEIK |
| iPS:435 871 | 21-225_191E6 | VK2\|A18/JK1 | | DIVMTQTPLSLSV TPGQPASISC | KSS-- QSLLHSD- GRTYLY | WYLQKPGQ PPQLLIC | E-------- VSNRFA | GVTDRFSGSGSG-- TDFTLKISRVEAGDVGIYYC | MQSIH----------- -----------FPWT | FGQGT KVEIK |
| iPS:435 887 | 21-225_186F7 | VK2\|A18/JK1 | | DVVMAQTPLSLSV TPGQPASISC | KSS-- QSLLHGD- GKTYLC | WYLQKPGQ PPQLLIY | E-------- VSKRFS | GVPDRFSGSGSG-- TDFTLKISRVEAEDVGVYYC | MQSIQ----------- -----------VPWT | FGQGT KVEIK |
| iPS:435 899 | 21-225_188G11 | VK2\|A18/JK1 | | DIVMTQTPLSLSV TPGQPASISC | MSS-- QSLLHGD- GKTYLY | WYLQKPGQ PPQLLIY | E-------- VSNRFS | GVPDTFSGSGSG-- TDFTLKISRVEAEDVGVYYC | MQSIQ----------- -----------IPWT | FGQGT KVEIK |
| iPS:435 901 | 21-225_189G2 | VK2\|A18/JK1 | | DIVMTQTPLSLSV TPGQPASISC | KSS-- QSLLHGD- GKTYLF | WYLQKPGQ PPQLLIY | E-------- VSNRFS | GVSDRFSGSGSG-- TDFTLKISRVEAEDVGVYYC | MQSIQ----------- -----------IPWT | FGQGT KVEIK |
| iPS:435 927 | 21-225_190E7 | VK2\|A18/JK1 | | DIVLTQTPLSLSV TPGQPASISC | KSS-- QSLLHSD- GRTYLY | WYLQKPGQ PPQVLIC | E-------- VSNRFS | GVPDRFSGSGSG-- TDFTLKISRVEAGDVGVYYC | MQSIQ----------- -----------LPWT | FGQGT KVEIK |
| iPS:435 999 | 21-225_192F9 | VK2\|A18/JK1 | | DIVMTQTPLSLSV TPGQPASISC | KSS-- QSLLHSD- GRTYLY | WYLQRPGQ PPQLLIC | E-------- VSNRFA | GVTDRFSGSGSG-- TDFTLKISRVEAGDVGVYYC | MQSIQ----------- -----------LPWT | FGQGT KVEIK |
| iPS:436 060 | 21-225_194F4 | VK2\|A18/JK1 | | DIVMTQTPLSLSV TPGQPASISC | KSS-- QSLLHSD- GRTYLY | WYLQKPGQ PPQLLIC | E-------- VSNRFS | GVPDRFSGSGSG-- TDFTLKISRVEAGDVGVYYC | MQSIQ----------- -----------LPWT | FGQGT KVEIK |
| iPS:436 158 | 21-225_197G8 | VK2\|A18/JK1 | | EIVMTQTPLSLSV IPGQPASISC | KSS-- QNLLHSD- GKTYLY | WYLQKPGQ PPQVLIY | E-------- VSNRFS | GVPDRFSGSGSG-- TDFTLKISRVEAEDVGVYSC | MQSIQ----------- -----------LPWT | FAQGS KVEIK |
| iPS:436 193 | 21-225_198A10 | VK2\|A18/JK1 | | DIVLTQTPLSLSV TPGQPASISC | KSS-- QSLLYSD- GRTYLY | WYLQKPGQ PPQVLIC | E-------- VSNRFS | GVPDRFSGSGSG-- TDFTLKISRVEAGDVGVYYC | MQSIQ----------- -----------LPWT | FGQGT KVEIK |

| | | | | | | | | | | | |
|---|---|---|---|---|---|---|---|---|---|---|---|
| iPS:436 536 | 21-225_224G1 | VK2\|A18/ JK1 | | DIVMIQTPLSLSV TPGQPASISC | KSG-- QSLLHSD- GKTFLS | WYLQKPGQ PPQLLIY | E-------- ISNRFS | GVPDRFSGSGSG-- TDFTLKISRVEAEDVGIFYC | MQSTQ----------- -----------LPRT | FGQGT KVEIK |
| iPS:436 548 | 21-225_224A7 | VK2\|A18/ JK1 | | DIVMIQTPLSLSV TPGQPASISC | KSS-- QSLLHSD- GKTFLY | WFLQKPGQ PPQLLIY | E-------- ISNRFS | GVPDRFSGSGSG-- TDFTLKISRVEAEDVGIYYC | MQSTQ----------- -----------LPRT | FGQGT KVEIK |
| iPS:436 558 | 21-225_224C1 1 | VK2\|A18/ JK1 | | DFVMIQTPLSLSV TPGQPASISC | KSS-- QSLLHSD- GKTFLY | WYLQKPGQ PPQLLIY | E-------- ISNRFS | GVPDRFSGSGSG-- TDFTLKISRVEAEDVGIYYC | MQSTQ----------- -----------LPRT | FGQGT KVEIK |
| iPS:436 562 | 21-225_224H1 1 | VK2\|A18/ JK1 | | DIVMIQTPLSLSV TPGQPASISC | KSS-- QSLLHSD- GKTFLY | WYLQKPGQ PPQLLIY | E-------- ISNRFS | GVPDRFSGSGSG-- TDFTLKISRVEAEDVGIYYC | MQSTQ----------- -----------LPRT | FGQGT KVEIK |
| iPS:436 572 | 21-225_225G4 | VK2\|A18/ JK1 | | DIVMIQTPLSLSV TPGQPASISC | KSS-- QSLLHSD- GKTFLY | WYLQKPGQ PPQLLIY | E-------- ISNRFS | GVPDRFSGSGSG-- TDFTLKISRVEAEDVGIYYC | MQSTQ----------- -----------LPRT | FGQGT KVEIK |
| iPS:436 592 | 21-225_226B1 | VK2\|A18/ JK1 | | DIVMIQTPLSLSV TPGQPASISC | KSS-- QSLLHGD- GKTYLY | WYLQRPGQ PPQLLIN | E-------- VSIRFS | GVPDRFSGSGSG-- TDFTLKISRVEAEDVGVYYC | MQSIQ----------- -----------IPWT | FGQGT KVDIK |
| iPS:436 594 | 21-225_226A5 | VK2\|A18/ JK1 | | DIVMIQTPLSLSV TPGQPASISC | KSS-- QSLLHGD- GKTYLY | WYLQKPGQ PPQLLIY | E-------- VSNRFS | GVPDRFSGSGSG-- TDFTLKISRVEAEDVGVYYC | MQSIQ----------- -----------IPWT | FGQGT KVEIK |
| iPS:436 602 | 21-225_226E7 | VK2\|A18/ JK1 | | DIVMIQTPLSLSV TPGQPASISC | KSS-- QSLLHGD- GKTYLY | WYQQKPGQ PPQILIY | E-------- VSNRFS | GVPDRFSGSGSG-- TDFTLKISRVEAEDVGVYYC | MQSIQ----------- -----------VPWT | FGQGT KVEIK |
| iPS:436 606 | 21-225_226G8 | VK2\|A18/ JK1 | | DIVMIQTPLSLSV TPGQPASISC | KSS-- QSLLHSD- GKTFLY | WYLQKPGQ PPQLLIY | E-------- ISNRFS | GVPDRFSGSGSG-- TDFTLKISRVEAEDVGIYYC | MQSTQ----------- -----------LPRT | FGQGT KVEIK |
| iPS:436 610 | 21-225_226F9 | VK2\|A18/ JK1 | | DIVMIQTPLSLSV TPGQPASISC | KSS-- QSLLHSD- GKTFLY | WYLQKPGQ PPQLLIY | E-------- ISNRFS | GVPDRFSGSGSG-- TDFTLKISRVEAEDVGIYYC | MQSTQ----------- -----------LPRT | FGQGT KVEIK |
| iPS:436 612 | 21-225_226H9 | VK2\|A18/ JK1 | | DIVMIQTPLSLSV IPGQPASISC | KSS-- QSLLHSD- GKTFLY | WYLQRPGQ PPQLLIY | E-------- VSNRFS | GVPDRFSGSGSG-- TDFTLKISRVEAEDVGIYYC | MQSTQ----------- -----------LPRT | FGQGT KVEIK |
| iPS:436 614 | 21-225_226F10 | VK2\|A18/ JK1 | | DIVMIQTPLSLSV TPGQPASISC | KSS-- QSLLHSD- GKTFLY | WYLQKPGQ PPQLLIY | E-------- ISNRFS | GVPDRFSGSGSG-- TDFTLKISRVEAEDVGIYYC | MQSTQ----------- -----------LPRT | FGQGT KVEIK |
| iPS:436 618 | 21-225_226E1 1 | VK2\|A18/ JK1 | | DIVMIQTPLSLSV TPGQPASISC | KSS-- QSLLHSD- GKTFLY | WYLHKPGQ PPHLLIY | E-------- ISNRFS | GVPDRFSGSGSG-- TDFTLKISRVEAEDVGIYYC | MQSTQ----------- -----------LPRT | FGQGT KVEIK |
| iPS:436 624 | 21-225_226H1 2 | VK2\|A18/ JK1 | | DIVMIQTPLSLSV TPGQPASISC | KSS-- KTLLHSD- GKTFLY | WYLQKPGQ PPQPLIY | E-------- ISNRFS | GVPDRFSGSGSG-- TDFTLKISRVEAEDVGIYYC | MQSTQ----------- -----------LPRT | FGQGT KVEIK |
| iPS:436 626 | 21-225_227C1 | VK2\|A18/ JK1 | | DIVMIQTPLSLSV TPGQPASISC | KSS-- QSLLHSD- GKTFLY | WYLQKPGQ PPQLLIY | E-------- ISNRFS | GVPDRFSGSGSG-- TDFTLKISRVEAEDVGIYYC | MQSTQ----------- -----------LPRT | FGQGT KVEIK |
| iPS:436 628 | 21-225_227F2 | VK2\|A18/ JK1 | | DIVMIQTPLSLSV TPGQPASISC | KSS-- QSLLHSD- GKTFLY | WYLQKPGQ PPQLLIY | E-------- ISNRFS | GVPDRFSGSGSG-- TDFTLKISRVEAEDVGIYYC | MQSTQ----------- -----------LPRT | FGQGT KVEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:436 640 | 21-225_227A8 | VK2\|A18/JK1 | | DIVMTQTPLSLSV TPGQPASISC | KSS--QSLLHSD-GKTFLY | WYLQKPGQ PPQLLIY | E--------ISNRFS | GVPDRFSGSGSG-- TDFTLKISRVEAEDVGIYYC | MQSIQ-----------------------LPRT | FGQGT RVEIK |
| iPS:392 814 | 21-225_22A1 | VK2\|A18/JK1 | | DIVMTQTPLSLSV TPGQPASISC | KSS--QSLLHSG-GKTYLY | WYLQKPGQ PPQLLIY | E--------VSNRFS | GLPDRFSGSGSG-- TDFTLKISRVEAADVGVYYC | MQTLH-----------------------LPWT | FGQGT KVEIK |
| iPS:392 930 | 21-225_25H9 | VK2\|A18/JK1 | | DIVMTQTPLSLSV TPGQPASISC | KSS--QSLLHGD-GKTYLF | WYLQKPGQ PPQLLIY | E--------VSNRFS | GVPDRFSGSGSG-- TDFTLKISRVEAEDVGVYYC | MQSIQ-----------------------LPWT | FGQGT KVEIK |
| iPS:393 032 | 21-225_26F8 | VK2\|A18/JK1 | | DIVMTQTPLSLSV TPGQPASISC | KSS--QSLLHGD-GKTYLY | WYLQKPGQ PPQLLIY | E--------VSNRFS | GVPDRFSGSGSG-- TDFTLKISRVEAEDVGVYYC | MQSIQ-----------------------LPWT | FGQGT KVEIK |
| iPS:393 036 | 21-225_28G3 | VK2\|A18/JK1 | | EIVMTQTPLSLSV TPGQPASISC | KSS--QSLLHGD-GKTYLY | WYLQKPGQ PPQLLIY | E--------VSNRFS | GVPERFSGSGSG-- TDFTLKISRVEAEDVGVYYC | MQSIQ-----------------------IPWT | FGQGT KVEIK |
| | Germline | | | K_FR1 | K_CDR1 | K_FR2 | K_CDR2 | K_FR3 | K_CDR3 | K_FR4 |
| | VK1\|A30/JK1 | | 32139 | DIQMTQSPSSLSA SVGDRVTITC | RAS--QGIR-------NDLG | WYQQKPGK APKRLIY | A--------ASSLQS | GVPSRFSGSGSG-- TEFTLTISSLQPEDFATYYC | LQHYS-----------------------YPWT | FGQGT KVEIK |
| iPS:468 826 | 21-225_201C5 | VK1\|A30/JK1 | | DIQMTQSPSSLSA SVGDRVTITC | RAS--QGIR-------HDLG | WYQQKPGK APKRLIY | A--------ASSLQS | GVPSRFSGSGSG-- TEFTLTISSLQPEDFATYYC | LQHYS-----------------------FPRT | FGQGT KVEIK |
| iPS:468 842 | 21-225_50H4 | VK1\|A30/JK1 | | DIQMTQSPSSLSA SVGDRVTITC | RAS--QGIR-------NDLG | WYQQKPGK APKRLIY | A--------ASSLQS | GVPSRFSGSGSG-- TEFTLTISSLQPEDFATYYC | LQHYS-----------------------YPRT | FGQGT KVEIK |
| iPS:468 858 | 21-225_148C9 | VK1\|A30/JK1 | | DIQMTQSPSSLSA SVGDRVTITC | RAS--RGIR-------DDLG | WYQQKPGK APKRLIY | A--------ASSLQS | GVPSRFSGSGSG-- TEFTLTISSLQPEDFATYYC | LQHYS-----------------------YPRT | FGQGT KVEIK |
| iPS:468 860 | 21-225_224E7 | VK1\|A30/JK1 | | DIQMTQSPSSLSA SVGDRVTITC | RAS--QGIR-------NDLG | WYQQKPGK APTRLIY | A--------ASSLQS | GVPSRFSGSGSG-- TEFTLTISSLQPEDFATYYC | LQHYS-----------------------YPRT | FGQGT KVEIK |
| iPS:433 919 | 21-225_44B3 | VK1\|A30/JK1 | | DIQMTQSPSSLSA SVGDRVTITC | RAS--QGIR-------NDLG | WYQQKPGK APKRLIY | A--------ASSLQS | GVPSRFSGSGSG-- TEFTLTISSLQPEDFATYYC | LLHYN-----------------------YPRT | FGQGT KVEIK |
| iPS:433 923 | 21-225_44D3 | VK1\|A30/JK1 | | DIQMTQSPSSLSA SVGDRVTFTC | RAS--QGIR-------DDLG | WYQQKPGK APKRLIY | A--------ASSLQS | GVPSRFSGSRSG-- REFTLTISSLQPEDFATYYC | LQHYS-----------------------YPRT | FGQGT KVEIK |
| iPS:433 929 | 21-225_44D5 | VK1\|A30/JK1 | | DIQMTQSPSSLSA SVGDRVTITC | RAS--QDIR-------KDLG | WYQQKPGK APKRLIY | A--------ASTLES | GVPSRFSGSGSG-- TEFTLTISSLQPEDFATYYC | LQHYS-----------------------FPWT | FGQGT KVEIK |
| iPS:433 935 | 21-225_44F9 | VK1\|A30/JK1 | | DVQMTQSPSSLSA SVGDRVTITC | RAS--QGIR-------NDLG | WYQQKPGK APKRLIY | A--------ASSLQS | GVPSRFSGSGSG-- TEFTLTISSLQPEDFATYYC | LHHYN-----------------------YPRT | FGQGT KVEIT |
| iPS:433 939 | 21-225_44C10 | VK1\|A30/JK1 | | DIQMTQSPSSLSA SVGDRVTITC | RAS--QGIR-------DDLG | WYQQKPGK APKRLIY | A--------TSSLQS | GVPSRFSGSGSG-- TEFTLTISSLQPEDFATYYC | LQHYS-----------------------YPRT | FGQGT KVEIK |
| iPS:433 951 | 21-225_45B4 | VK1\|A30/JK1 | | DIQMTQSPSSLSA SVGDRVTFTC | RAS--QGIR-------DDLG | WYQQKPGK APKRLIY | A--------ASSLQS | GVPSRFSGSGSG-- TEFTLTISSLQPEDFATYYC | LQHYS-----------------------YPRT | FGQGT KVEIK |
| iPS:433 955 | 21-225_45B8 | VK1\|A30/JK1 | | DIQMTQSPSSLSA SVGDRVTFTC | RAS--QGIR-------DDLG | WYQQKPGK APKRLIY | A--------ASSLQS | GVPSRFSGSGSG-- TEFTLTISSLQPEDFATYYC | LQHYN-----------------------YPRT | FGQGT KVEIK |
| iPS:433 967 | 21-225_46C3 | VK1\|A30/JK1 | | DIQMTQSPSSLSA SVGDRVTFTC | RAS--QGIR-------DDLG | WYQQKPGK APKRLIY | A--------ASSLQS | GVPSRFSGSRSG-- TEFTLTISSLQPEDFATYYC | LQHYS-----------------------YPRT | FGQGT KVEIK |
| iPS:433 971 | 21-225_46D4 | VK1\|A30/JK1 | | DIQMTQSPSSLSA SVGDRVTITC | RTS--QGIR-------KDLG | WYQQKVGK APKRLIY | A--------ASSLES | GVPSRFSGSGSG-- TEFTLTISSLQPEDFATYYC | LQHYS-----------------------FPWT | FGQGT KVEIK |
| iPS:433 997 | 21-225_48C1 | VK1\|A30/JK1 | | DIQMTQSPSSLSA SVGDRVTITC | RTS--QGIR-------NDLG | WYQQKPGK APKRLIY | R--------ASSLQS | GVPARFSGSGSG-- TEFTLTISSLQPEDFAIYYC | LQHNF-----------------------YPWT | FGQGT KVEIK |

| | | | | | | | | | | |
|---|---|---|---|---|---|---|---|---|---|---|
| iPS:434 001 | 21-225_48F2 | VK1\|A30/ JK1 | | DIQMTQSPSSLSA SVGDRVTITC | RAS--RGIR-- ----DDLG | WYQQKPGK PPKRLIY | A-------- ASSLQS | GVPSRFNGSGSG-- TEFTLTISSLQSEDLATYYC | LQQYS----------- -----------YPRT | FGQGT KVEIK |
| iPS:434 009 | 21-225_48A9 | VK1\|A30/ JK1 | | DIQMTQSPSSLSA SVGDRVTITC | RAS--QGIR-- ----NDLG | WYQQKPGK APKRLIY | I-------- ASSLQS | GVPSRFSGSGSG-- TEFTLTISGLQPEDFAIYYC | LQHNR----------- ------------YPWT | FGQGT KVEIK |
| iPS:434 047 | 21-225_50A12 | VK1\|A30/ JK1 | | DIQMTQSPSSLSA SVGDRVTITC | RAS--QGIR-- ----NDLG | WYQQKPGK APKRLIY | I-------- ASNLQS | GVPSRFSGSGSG-- TEFTLTISSLQPEDFATYYC | LQHNS----------- ------------YPWT | FGQGT KVEIK |
| iPS:434 067 | 21-225_51H8 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGGGSG-- THFTLTISSLQPEDFATYYC | LQHNS----------- ------------YPWT | FGQGT KVEIK |
| iPS:434 135 | 21-225_54H3 | VK1\|A30/ JK1 | | DIQMTQSPSSLSA SVGDRVTITC | RAS--QDIR-- ----NILG | WYQQKPGK APKRLIY | A-------- ASNLQS | GVPSRFSGSGSG-- TEFTLTISSLQPEDFATYYC | LQYNS----------- ------------YPWT | FGQGT KVEIK |
| iPS:434 197 | 21-225_56C7 | VK1\|A30/ JK1 | | DIQMTQSPSSLSA SVGDRVTITC | RAS--QGIR-- ----NDLG | WYQQKPGK APKRLIY | T-------- ASNLQS | GVPSRFSGSGSG-- TEFTLTISSLQPEDFATYYC | LQHNS----------- ------------YPWT | FGQGT KVEIK |
| iPS:434 203 | 21-225_60E2 | VK1\|A30/ JK1 | | DIQMTQSPSSLSA SVGDRVTITC | RAS--QGIR-- ----KDLG | WYQQKPGK APKRLIY | A-------- ASSLQS | GVPSRFSGSGSG-- TEFTLTISSLQPEDFATYYC | LQHNS----------- ------------YPWT | FGQGT KVEIK |
| iPS:434 209 | 21-225_60C3 | VK1\|A30/ JK1 | | DIQMTQSPSSLSA SVGDRVTITC | RAS--QGIR-- ----NDLG | WYQQKPGK APKRLIY | S-------- ASSLQS | GVPSRFSGSGSG-- TEFTLTISSLQPEDFATYYC | LQHNS----------- ------------YPWT | FGLGT KVEIK |
| iPS:434 229 | 21-225_61H1 | VK1\|A30/ JK1 | | DIQMTQSPSSLSA SVGDRVTITC | RAS--QGIR-- ----KDLG | WYQQKPGK APKRLIF | A-------- ASSLQS | GVPSRFSGSGSG-- TEFTLTISSLQPEDFATYYC | LQHNS----------- ------------YPWT | FGQGT KVEIK |
| iPS:434 241 | 21-225_61E6 | VK1\|A30/ JK1 | | DIQMTQSPSSLSA SVGDRVTITC | RAS--QGIG-- ----APERLIY | WYQQKPGK | A-------- ASSLQS | GVPSRFSGSGSG-- TEFTLTISSLQPEDFATYYC | LQHNS----------- -----------FPPWT | FGQGT KVEIK |
| iPS:434 257 | 21-225_62F7 | VK1\|A30/ JK1 | | DIQMTQSPSSLSA SVGDRVTITC | RAS--QAIR-- ----NDLG | WYQQKPGK APKRLIY | P-------- ASRLQS | GVPSRFSGSGSG-- TEFTLTISSLQPEDFATYYC | LQYNS----------- -----------YPPWT | FGQGS KVEIK |
| iPS:434 281 | 21-225_57B8 | VK1\|A30/ JK1 | | DIQMTQSPSSLSA SVGDRITITC | RAS--QGIG-- ----APERLIY | WYQQKPGK | A-------- ASSLQS | GVPSRFSGSGSG-- TEFTLTISSLQPEDFATYYC | LQHNS----------- -----------FPPWT | FGQGT KVEIK |
| iPS:434 315 | 21-225_59G7 | VK1\|A30/ JK1 | | DIQMTQSPSSLSA SVGDRVTITC | RAS--QGIR-- ----NDLG | WYQQKPGK APKRLIY | S-------- ASSLQS | GVPSRFSGSGSG-- TEFTLTISSLQPEDFATYYC | LQHNS----------- ------------YPWT | FGQGT KVEIK |
| iPS:434 319 | 21-225_59B9 | VK1\|A30/ JK1 | | DIQMTQSPSSLSA SVGDRVTITC | RAS--QDIR-- ----NDLG | WYQQHPGK APKRLIY | A-------- ASSLQS | GVPSRFSGTRSG-- TEFTLTISSLQPEDFATYYC | LQHNS----------- ------------YPWT | FGQGT KVEIK |
| iPS:434 339 | 21-225_64A4 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDLATYYC | LQHYS----------- ------------YPRT | FGQGT KVEIK |
| iPS:434 343 | 21-225_64C8 | VK1\|A30/ JK1 | | DIQMTQSPSSLSA AVGDRVTITC | RAS--QGIR-- ----NDLG | WYQQKPGK APKCLIY | A-------- ASSLQS | GVPSRFSGSGSG-- TEFTLTISSLQPEDFATYYC | LHHYS----------- ------------YPRT | FGQGT KVEIK |
| iPS:434 385 | 21-225_66C10 | VK1\|A30/ JK1 | | DIQMTQSPSSLSA SVGDRVTITC | RAS--QAIR-- ----NDLG | WYQQKPGK APKRLIY | P-------- ASSLQS | GVPSRFSGSGSG-- TEFTLTISSLQPEDFATYYC | LQYNS----------- -----------YPPWT | FGQGS KVEIK |
| iPS:434 387 | 21-225_66D11 | VK1\|A30/ JK1 | | DIQMTQFPSSQSA SVGDRVTITC | RAS--QGIR-- ----NDLG | WYQQKPGK AHKRLIY | A-------- ASSCQS | GVPSRFSGSGSG-- TEFTISISSMQREDFATYYC | IVHNS----------- ------------YPRT | FGQGT KVEIK |
| iPS:434 441 | 21-225_71A2 | VK1\|A30/ JK1 | | DIQMTQSPSSRSA SVGDRVTITC | RAS--QGIR-- ----NDLG | WYQQKPGK APKRLIY | I-------- AFRLQI | GVPSRFSGSGSG-- TEFTLTISSLQPEDFATYYC | IHHNS----------- ------------YPWT | FGQGT KVEIK |
| iPS:434 469 | 21-225_73C9 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHYS----------- ------------YPRT | FGQGT KVEIK |
| iPS:435 197 | 21-225_94F3 | VK1\|A30/ JK1 | | DIQMTQSPSSLSA SVGDRVTITC | RAS--QAIR-- ----DDLG | WYQQKPGK APQRLIF | A-------- ASSLQS | GVPSRFSGSGSG-- TEFTLTISSLQPEDFATYYC | LQHYS----------- ------------YPRT | FGRGT KVAIK |
| iPS:435 325 | 21-225_147H5 | VK1\|A30/ JK1 | | DIQMTQSPSSLSA SVGDRVTITC | RAS--RGIR-- ----DDLG | WYQQKPGK APKRLIY | A-------- ASSLQS | GVPSRFSGSGSG-- TEFTLTISSLQPEDFATYYC | LQHYS----------- ------------YPRT | FGQGT KVEIK |
| iPS:435 393 | 21-225_149D1 0 | VK1\|A30/ JK1 | | DIQMTQSPSSLSA SVGDRVTITC | RAS--RGIR-- ----NDLG | WYQQKPGK APKRLIY | A-------- ASSLQS | GVPSRFSGSGSG-- TEFTLTISSLQPEDFATYYC | LQHYS----------- ------------YPRT | FGQGT KVEIK |

| | | | | | | | | | | |
|---|---|---|---|---|---|---|---|---|---|---|
| iPS:435 539 | 21- 225_158G1 | VK1|A30/ JK1 | | DIQMTQSPSSLSA SVGDRVTITC | RAS--QGIR-- ----NDLG | WYQQKPGK APKRLIY | T-------- ASNLQS | GVPSRFSGSGCG-- TEFTLTVSSLQPEDFATYYC | LQHNS----------- ------------YPWT | FGQGT KVEIK |
| IPS:435 543 | 21- 225_158D4 | VK1|A30/ JK1 | | DIQMTQSPSSLSA SVGDRVTITC | RAS--QDIR-- ----KDLG | WYQQKPGK APKRLIY | A-------- ASSLQS | GVPSRFSGSGSG-- TEFTLTFSSLQPEDFATYYC | LQHYS----------- ------------YPRT | FGQGT KVEIK |
| iPS:435 571 | 21- 225_159C8 | VK1|A30/ JK1 | | DIQMTQSPSSLSA SVGDRVTITC | RAS--QDIR-- ----KDLG | WYQQKPGK APNRLIY | A-------- ASSLQS | GVPSRFSGSGSG-- TEFTLTFSSLQPEDFATYYC | LQHHS----------- ------------YPRT | FGQGT KVEIK |
| iPS:435 573 | 21- 225_159D8 | VK1|A30/ JK1 | | DIQMTQSPSSLSA SVGDRVTITC | RAS--RDIG-- ----NDLG | WYQQKPGK APKRLIS | A-------- ASSLQS | GVPSRFSGSGSG-- TEFTLTFSSLQPEDFATYYC | LQHYS----------- ------------YPRT | FGQGT KVEIK |
| iPS:435 581 | 21- 225_160H1 | VK1|A30/ JK1 | | DIQMTQSPSSLSA SVGDRVTITC | RAS--QDIR-- ----NDLG | WYQQKPGK APKRLIY | A-------- ASSLQN | GVPSRFSGSGSG-- TEFTLTISSLQPEDFTTYYC | LQHNS----------- ------------FPWT | FGQGT KVEIK |
| iPS:435 583 | 21- 225_160F2 | VK1|A30/ JK1 | | DIQMTQSPSSLSA SVGDRVTITC | RAS--QGIR-- ----NDLG | WYQQKPGK APKRLIY | T-------- ASNLQS | GVPSRFSGSGSG-- TEFTLTVSSLQPEDFATYYC | LQHNS----------- ------------YPWT | FGQGT KVEIK |
| iPS:435 591 | 21- 225_160C4 | VK1|A30/ JK1 | | DIQMTQSPSSLSA SVGDRVTITC | RAS--QDIR-- ----KDLG | WYQQKPGK APNRLIY | A-------- ASSLQS | GVPSRFSGSGSG-- TEFTLTFSSLQPEDFATYYC | LQHYS----------- ------------YPRT | FGQGT KVEIK |
| iPS:435 615 | 21- 225_161G1 2 | VK1|A30/ JK1 | | DIQMTQSPSSRSA SVGDRVTITC | RAS--QDIR-- ----KDLG | WYQQKPGK APNRLIY | A-------- ASSLQS | GVPSRFSGSGSG-- TEFTLTFSSLQPEDFATYYC | LQHHS----------- ------------YPRT | FGQGT KVEIK |
| iPS:435 675 | 21- 225_169D7 | VK1|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHHS----------- ------------CPWT | FGQGT KVEIK |
| iPS:435 681 | 21- 225_169D1 1 | VK1|A30/ JK1 | | DIQMTQSPSSLSA SVGDRVTITC | RAS--QGIR-- ----DDLG | WYQQKPGK VPKRLIY | A-------- ASSLQS | GVPSRFSGSGSG-- TEFTLTISSLQPEDFATYYC | LQHYS----------- ------------YPRT | FGQGT KVEIK |
| iPS:435 687 | 21- 225_170H1 | VK1|A30/ JK1 | | DIQMTQSPSSLSA SVGDRVTITC | RAS--QGIR-- ----KDLG | WYQQKPGK APKRLIY | A-------- TSSLQS | GVPSRFSGSGSG-- TEFTLTFSSLQPEDFATYYC | LQHSS----------- ------------NPWT | FGQGT KVESK |
| iPS:435 689 | 21- 225_170F3 | VK1|A30/ JK1 | | DIQMTQSPSSLSA SVGDRVTITC | RAS--RGIR-- ----NDLG | WYQQKPGK APKRLIY | A-------- ASSLQS | GVPSRFSGSGSG-- TEFTLTINSLQPEDFATYYC | LQHYS----------- ------------YPRT | FGQGT KVEIK |
| iPS:435 741 | 21- 225_174G1 0 | VK1|A30/ JK1 | | DIQMTQSPSSLSA SVGDRVTITC | RAS--QGIR-- ----DDLG | WYQQKPGK VPKRLIY | A-------- ASSLQS | GVPSRFSGSGSG-- TEFTLTISSLQPEDFATYYC | LQHHS----------- ------------YPRT | FGQGT KVEIK |
| iPS:435 831 | 21- 225_190C1 2 | VK1|A30/ JK1 | | DIQMTQSPSSLSA SVGDRVTITC | RAS--QGIR-- ----KDLG | WYQQKPGK APKRLIH | T-------- ASSLQS | GVPLRFSGSGSG-- TEFTLTISSLQPEDFATYYC | LQHNS----------- ------------YPWT | FGQGT KVEIK |
| iPS:435 857 | 21- 225_191A4 | VK1|A30/ JK1 | | DIQMTQSPSSLSA SVGDRVTITC | RAS--QGIR-- ----KDLG | WYQQKPGK APKRLIH | T-------- ASSLQN | GVPLRFSGSGSG-- TEFTLTISSLQPEDFATYYC | LQHNS----------- ------------YPWT | FGQGT KVEIK |
| iPS:435 907 | 21- 225_190G3 | VK1|A30/ JK1 | | DIQMTQSPSSLSA SVRDRVTITC | RAS--QGIR-- ----KDLG | WYQQKPGK APKRLIH | T-------- ASSLQS | GVPLRFSGSGSG-- TEFTLTISSLQPEDFATYYC | LQHNS----------- ------------YPWT | FGQGT KVEIK |
| iPS:435 919 | 21- 225_190H5 | VK1|A30/ JK1 | | DIQMTQSPSSLSA SVGDRVTITC | RAS--QGIR-- ----KDLG | WYQQKPGK APKRLIH | T-------- ASSLQS | GVPLRFSGSGSG-- TEFTLTISSLQPEDFATYYC | LQHNN----------- ------------YPWT | FGQGT KVEIK |
| iPS:435 989 | 21- 225_192F7 | VK1|A30/ JK1 | | DIQMTQSPSSLSA SVGDRVTITC | RAS--QGIR-- ----KDLG | WYQQKPGK APKRLIH | T-------- ASSLQS | GVPLRFSGSGSG-- TEFTLTISSLQPEDFATYYC | LQHTS----------- ------------YPWT | FGQGT KVEIY |
| iPS:436 132 | 21- 225_196C1 2 | VK1|A30/ JK1 | | DIQMTLSPSSLFA CVGDRVIITC | RAS--QGIR-- ----NDLG | WSQQNPGK APKRLIY | A-------- ASSLQS | GVPSRFSGSGSG-- TEFTITISSLQPEDFETYYC | LQHND----------- ------------FPFT | FGRGT KVEIK |
| iPS:436 222 | 21- 225_200C9 | VK1|A30/ JK1 | | DIQMTQSPSSLSA SVGDRVTITC | RAT--QGIR-- ----KDLG | WYQQKPGK APKRLIH | T-------- ASSLQS | GVPLRFSGSGSG-- TEFTLTISSLQPEDFATYYC | LQHNS----------- ------------YPWT | FGQGT KVEIK |
| iPS:436 264 | 21- 225_203F7 | VK1|A30/ JK1 | | DIQMTQSPSSRFA SVGDRVTITC | RAS--QGIR-- ----HDLG | WYQQKPGK ALKRLIY | A-------- ASSLQS | GVPSRFSGSGCG-- TEFTLTISSVQPEDFANYYC | LQHYS----------- ------------FPRT | FGQGT KVEIK |

| | | | | | | | | | |
|---|---|---|---|---|---|---|---|---|---|
| iPS:436 274 | 21-225_204H3 | VK1\|A30/ JK1 | DIQMTQSPSSLSA SVGDRVTITC | RAS--QGIR-- ----NDLG | WYQQKPGK APELLIY | A-------- AASLQG | GVPSRFSGSGSG-- TEFTLTISSLQPEDFATYYC | LQHYS----------- ------------YPRT | FGQGT KVELQ |
| iPS:436 332 | 21-225_208B2 | VK1\|A30/ JK1 | DIQMTQSPSSLSA SVGDRVTITC | RAS--QGIR-- ----HDLG | WYQQKPGK APKRLIY | A-------- ASSLQS | GVPSGFSGSGSG-- TEFTLTISSLQPEDFATYYC | LQHYS----------- ------------YPRT | FGQGT KVEIK |
| iPS:436 352 | 21-225_210G5 | VK1\|A30/ JK1 | DIQMTQSPSSLSA SVGDRVTITC | RAS--QGIR-- ----HDLG | WYQQKPGK APKRLIY | A-------- ASSLQS | GVPSGFSGSGSG-- TEFTLTISSLQPEDFATYYC | LHHYS----------- ------------YPRT | FGQGT KVEIK |
| iPS:436 386 | 21-225_212B1 1 | VK1\|A30/ JK1 | DIQMTQSPSSLPA SVGDRVTITC | RAS--QGIR-- ----NDLG | WYQQKPGK APKRLIY | A-------- ASSLQS | GVPSRFSGSGSG-- TEFILTISSLQPEDFATYYC | LLHYS----------- ------------YPRT | FGQGT KVEIK |
| iPS:436 412 | 21-225_214H9 | VK1\|A30/ JK1 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHYS----------- ------------YPRT | FGQGT KVEIK |
| iPS:436 414 | 21-225_214G1 0 | VK1\|A30/ JK1 | DIQMTLCPSSPPA SVGDRVTITC | RAS--QGIR-- ----NDLG | WYQQKPGK APKRLIY | A-------- ASSLQS | GVPSRFSGSGSG-- TEFILTISSLQPEDFATYYC | LLHNS----------- ------------YPRT | FGQGT KVEIK |
| iPS:436 416 | 21-225_214G1 2 | VK1\|A30/ JK1 | DIQMTQSPSSPPA SVGDRVTITC | RAS--QGIR-- ----NDLG | WYQQKPGK APKRLIY | A-------- ASSLQS | GVPSRFSGSGSG-- TEFILTISSLQPEDFATYYC | VMHYS----------- ------------YPRT | FGQGT KVEIK |
| iPS:436 418 | 21-225_215E3 | VK1\|A30/ JK1 | DIQMTQSPSSPPA SVGDRVTITC | RAS--QGIR-- ----NDLG | WYQQKPGK APKRLIY | A-------- ASSLQS | GVPSRFSGSGSG-- TEFTLTISSLQPEDFATYYC | VMHNS----------- ------------YPRT | FGQGT KVEIK |
| iPS:436 428 | 21-225_215E1 1 | VK1\|A30/ JK1 | DIQMTQCPSSPPA SVGDRVTITC | RAS--QGIR-- ----NDLG | WYQQKPGK APKRLIY | A-------- ASSLQS | GVPSRFSGSGSG-- TEFILTISSVQREDFATYYC | VMHYS----------- ------------YPRT | FGQGT KVEIK |
| iPS:436 438 | 21-225_216E8 | VK1\|A30/ JK1 | DIQMTQSPSSLPA SVGDRVTITC | RAS--QGIR-- ----NDLG | WYQQKPGK APKRLIY | A-------- ASSLQS | GVPSRFSGSGSG-- TEFILTISSLQPEDFATYYC | LMHYS----------- ------------YPRT | FGQGT KVEIK |
| iPS:436 440 | 21-225_216H1 2 | VK1\|A30/ JK1 | DIQMTLSPSSPPA SVGDRVTITC | RAS--QGIR-- ----NDLG | WYQQKPGK APKRLIY | G-------- ASSLQS | GVPSRFSGSGSG-- TEFILTISSLQPEDFATYYC | VMHNS----------- ------------YPRT | FGQGT KVEIK |
| iPS:436 450 | 21-225_217E5 | VK1\|A30/ JK1 | DIQMTQCPSSPPA FVGDRVTITC | RAS--QGIR-- ----NDLG | WYQQKPGK APKRLIC | A-------- ASSLQS | GVPSRFSGSGSG-- TEFILTISSLQPEDFATYYC | VMHNS----------- ------------YPRT | FGQGT KVEIK |
| iPS:436 456 | 21-225_217G1 0 | VK1\|A30/ JK1 | DIQMTQSPSSPPA SVGDRVTITC | RAS--QGIR-- ----NDLG | WYQQKPGK APKRLIY | A-------- ASSLQS | GVPSRFSGSGSG-- TEFILTISSLQPEDFATYYC | VMHNS----------- ------------YPRT | FGQGT KVEIK |
| iPS:436 458 | 21-225_217H1 2 | VK1\|A30/ JK1 | DIQMTQSPSSPPA SVGDRVTITC | RAS--QGIR-- ----NDLG | WYQQKPGK APKRLIY | A-------- ASSLQS | GVPSRFSGSGSG-- TEFILTISSLQPEDFATYYC | LMHYS----------- ------------YPRT | FGQGT KVEIK |
| iPS:436 462 | 21-225_218C4 | VK1\|A30/ JK1 | DIQMTQCPSSPPA FVGDRVTITC | RAS--QGIR-- ----NDLG | WYQQKPGK APKRLIY | A-------- ASSLQS | GVPSRFSGSGSG-- TEFILTISSVQREDFATYYC | VMHYS----------- ------------YPRT | FGQGT KVEIK |
| iPS:436 480 | 21-225_220F8 | VK1\|A30/ JK1 | DIQMTLSPSSPPA FVGDRVTITR | RAS--QGIR-- ----NDLG | WYQQKPGK APKRLIC | G-------- ASSLQS | GVPSRFSGSGSG-- TEFILTISSLQREDFATYYC | VMHNS----------- ------------YPRT | FGQGT KVEIK |
| iPS:436 534 | 21-225_224F1 | VK1\|A30/ JK1 | AIQMTQSPSSLSA SVGDRVTITC | RAS--QGIR-- ----DDLG | WYQQKPGK APKRLIY | A-------- ASSLQS | GVPSRISGSGSG-- TEFTLTISSLQPEDFAIYYC | LQHYS----------- ------------YPRT | FGQGT KVEIK |
| iPS:436 540 | 21-225_224F3 | VK1\|A30/ JK1 | DIQMTQSPSSLSA SVGDRVTITC | RAS--QGIR-- ----NDLG | WYQQKPGK APERLIY | A-------- ASSLQS | GVPSRFSGSGSG-- TEFTLTISSLQPEDFATYYC | LQHYN----------- ------------YPRA | FGQGT KVEIK |
| iPS:436 564 | 21-225_225A1 | VK1\|A30/ JK1 | DIQMTQSPSSLSA SVGDRVTITC | RAS--QGIR-- ----DDLG | WYQQIPGK APKRLIY | A-------- ASSLQS | GVPSRFSGSGSG-- TEFTLTISSLQPEDFATYYC | LQHYS----------- ------------YPRT | FGQGT KVEIK |
| iPS:436 596 | 21-225_226C6 | VK1\|A30/ JK1 | DIQMTQSPSSLSA SIGDRVTITC | RAS--QGIR-- ----NDLG | WYQQKPGK APKRLIY | A-------- ASSLQS | GLPSRFSGSGSG-- TEFTLTISNLQPEDFATYYC | LQHYN----------- ------------YPRA | FGQGT KVEIQ |

| | | | | | | | | | | |
|---|---|---|---|---|---|---|---|---|---|---|
| iPS:436 604 | 21-225_226F7 | VK1\|A30/ JK1 | | DIQMTQSPSSLSA SVGDRVTITC | RAS--QGIG-- ----NDLG | WYQQKPGK APKRLIY | A-------- ASSLQS | GVPSRFSGSVSG-- TEFILTISSLQPEDFATYYC | LHHYS----------- -----------YPRT | FGQGT KVEIK |
| iPS:436 620 | 21-225_226H11 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TDFTLTISSLQPEDFATYYC | LQHYS----------- -----------YPRT | FGQGT KVEIK |
| iPS:437 262 | 21-225_170E4 | VK1\|A30/ JK1 | | DIQMTQSPSSLSA SVGDRVTITC | RAS--QGIR-- ----NDLG | YYQQKPGK APKRLIY | V-------- ASGLQS | GVPSRFSGSGSG-- TEFTLTISSLQPEDFATYYC | LQHNS----------- -------YPPWT | FGQGT KVDIK |
| iPS:437 280 | 21-225_203C10 | VK1\|A30/ JK1 | | DIQMTQSPSSLSA SVGDRVTITC | RAS--QDIR-- ----NDLG | WYQQKPAK APKRLIY | R-------- ASSLQS | GVPSRFSGSGSG-- TEFTLTISSLQPEDFAAYYC | LQHNS----------- -----------YPWT | FGQGT KVEIK |
| iPS:437 286 | 21-225_208F1 | VK1\|A30/ JK1 | | DIQMTQSPSSLSA SVGDRVTITC | RAS--QGIR-- ----HDLG | WYQQKPGK APKRLIF | A-------- ASSLQS | GVPSRFSGSGSG-- TEFTLTISSLQPEDFATYYC | LQHYS----------- -----------FPRT | FGQGT KVEIK |
| iPS:437 290 | 21-225_210G6 | VK1\|A30/ JK1 | | DIQMTQSPSSRFA FVGDRVTITC | RAS--QGIR-- ----HDLG | WYQQKPGK ALKRLIY | A-------- ASSSQS | GVPSRFSGSGCG-- TEFTLTISSVQREDFANYYC | VQHYS----------- -----------FPRT | FGQGT KVEIK |
| iPS:451 114 | 21-225_159A3 | VK1\|A30/ JK1 | | DIQMTQSPSSLSA SVGDRVTITC | RAS--QDIR-- ----KDLG | WYQQKPGK APNRLIY | A-------- ASSLQS | GVPSRFSGSGSG-- TEFTLTFSSLQPEDFATYYC | LQHHS----------- -----------YPRT | FGQGT KVEIK |
| iPS:392 626 | 21-225_18A5 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPWT | FGQGT KVEIK |
| iPS:392 634 | 21-225_17H3 | VK1\|A30/ JK1 | | DIQMTQSPSSLSA SVGDRVTFTC | RAS--QGIR-- ----SDLG | WYQQKPGK APKRLIY | A-------- ASSLQS | GVPSRFSGSGSG-- TEFTLTISSLQPEDFATYYC | LQQYS----------- -----------YPRT | FGQGT KVEIK |
| iPS:392 674 | 21-225_18C2 | VK1\|A30/ JK1 | | DIQMTQSPSSLSA SVGDRVTITC | RAS--QGIR-- ----NDLG | WYQQKPGK APKRLIY | T-------- ASSLQS | GVPSRFSGSGFG-- TEFTLTISSLQPEDFATYYC | LQHNS----------- -----------YPWT | FGLGT KVVIK |
| iPS:392 686 | 21-225_17C7 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPWT | FGQGT KVEIK |
| iPS:392 690 | 21-225_18F2 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPWT | FGQGT KVEIK |
| iPS:392 710 | 21-225_19A10 | VK1\|A30/ JK1 | | DIQMTQSPSSLSA SVGDRVTITC | RAS--QGIR-- ----TDLG | WYQQRPGK APKRLIY | T-------- ASSLQS | GVPSRFSGSGSG-- TEFTLTISRLQPEDFATYYC | LQHNG----------- -----------YPWT | FGQGT KVEIK |
| iPS:392 740 | 21-225_18H12 | VK1\|A30/ JK1 | | DIQMTQSPSSLSA SVGDRVTITC | RAS--QGIR-- ----NDLG | WYQQKPGK APKRLIY | T-------- ASNLQS | GVPSRFSGSGSG-- TEFTLTISSLQPEDFATYYC | LQHNN----------- -----------YPWT | FGLGT KVVIK |
| iPS:392 742 | 21-225_20B2 | VK1\|A30/ JK1 | | DIQMTQSPSSLSA SVGDRVNITC | RAS--QDIR-- ----NDLG | WYQQKPGK APKRLIY | A-------- ASSLQS | GVPSRFSGSGSG-- TEFTLTISSLQPEDFATYYC | LQHYN----------- -------YPRA | FGQGT KVDIK |
| iPS:392 758 | 21-225_21G11 | VK1\|A30/ JK1 | | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNN----------- -----------YPWT | FGLGT KVVIK |
| iPS:392 790 | 21-225_20D10 | VK1\|A30/ JK1 | | DIQMTQSPSSLSA FVGDRVTITC | RAS--QGIR-- ----NDLG | WYQQKPGK ARKRLIY | V-------- AYSLQS | GVPSRFSGSGYG-- TEFTLTISSLQPEDFATYYC | IQQNS----------- -----------YPWT | FGQGT KVEIK |
| iPS:392 796 | 21-225_22A4 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPWT | FGQGT KVEIK |
| iPS:392 832 | 21-225_21H8 | VK1\|A30/ JK1 | | DIQMTQSPSSLSA SVGDRVTITC | RAS--QGIR-- ----NDLG | WYQQKPGK APKRLIY | A-------- ASSLQS | GVPSRFRGSGSG-- TEFTLTISSLQPEDFATYYC | LQHNS----------- -----------YPWT | FGQGT KVEIK |
| iPS:392 836 | 21-225_22F4 | VK1\|A30/ JK1 | | DIQMTQSPSSLSA SVGDRVTITC | RAS--QVIR-- ----DDLG | WYQQKPGK APKRLIY | A-------- ASSLQS | GVPSRFSGSRSG-- TDFTLTISSLQPEDFATYYC | LHHYS----------- -----------YPRT | FGQGT KVEIK |
| iPS:392 844 | 21-225_23E11 | VK1\|A30/ JK1 | | DIQMTQSPSSLSA SVGDRITITC | RAS--QGIR-- ----NDLG | WYQQKPGK APKRLIY | P-------- ASSLQS | GVPSRFSGSGSG-- TEFTLTISSLQPEDFATYYC | LQHNS----------- -----------YPWT | FGQGT KVEIK |
| iPS:392 846 | 21-225_24B6 | VK1\|A30/ JK1 | | DIQMTQSPSSLSA SVGDRVTITC | RAS--QDIR-- ----NDLG | WYQQKPGK APKRLIY | A-------- ASSLHS | GVPSRFSGSGSG-- TEFTLTISSLQTEDFATYYC | LQHYS----------- -----------YPRT | FGQGT KVEVK |

| iPS | 21-225 clone | VK1\|A30/JK1 | | Seq 1 | Seq 2 | Seq 3 | Seq 4 | Seq 5 | Seq 6 | Seq 7 |
|---|---|---|---|---|---|---|---|---|---|---|
| iPS:392 872 | 21-225_20B11 | VK1\|A30/ JK1 | | DIQMTQSPSSLSA SVGDRVTITC | RAS--QGIG-- ----NDLG | WYQQKPEK APKRLIY | A-------- ASSLHS | GVPSRFSGSGSG-- TEFTLTISSLQPEDFATYYC | LQHYS----------- -----------YPRT | FGQGT KVEIK |
| iPS:392 876 | 21-225_21F7 | VK1\|A30/ JK1 | | DIQMTQSPSSLSA SVGDRVTITC | RAS--QDIR-- ----NDLG | WYQQKPGK APKRLIY | A-------- ASNFQS | GVPSRFSGSGSG-- TEFTLTISSLQPEDFATYYC | LQHNN----------- -----------YPWT | FGQGT KVEIK |
| iPS:392 884 | 21-225_23A10 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTIGSLQPEDFATYYC | LQHYS----------- -----------YPRT | FGLGT KVEIK |
| iPS:392 894 | 21-225_21G2 | VK1\|A30/ JK1 | | DIQMTQSPSSLSA SVGDRVTITC | RAS--QGIR-- ----NDLG | WYQQKPGK APKRLIY | T-------- SSSLQS | GVPSRFSGSGSG-- TEFTLTISSLQPEDFATYYC | LQHNS----------- -----------YPWT | FGLGT KVVIK |
| iPS:392 908 | 21-225_23F12 | VK1\|A30/ JK1 | | DIQMTQSPSSLSA SVGDRVTITC | RAS--QGIR-- ----NDLG | WYQQKPGK APKRLIY | V-------- ASSLQS | GVPSRFSGSGSG-- TEFTLTISSLQPEDFASYYC | LQHNS----------- -----------YPWT | FGQGT KVEIK |
| iPS:392 914 | 21-225_25D12 | VK1\|A30/ JK1 | | DIQMTQSPSSLSA SVGDRVTITC | RTS--QGIR-- ----NDLG | WYQQKPGK APKRLIY | A-------- ASSLQS | GVPSRFSGSGSG-- TEFTLTISSLQPEDFATYYC | LQHYS----------- -----------FPRT | FGQGT KVEIK |
| iPS:392 918 | 21-225_28F5 | VK1\|A30/ JK1 | | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNT----------- -----------YPWT | FGQGT KVEVK |
| iPS:392 958 | 21-225_28C7 | VK1\|A30/ JK1 | | DIQMTQSPSSLSA SVGDRVTITC | RAS--QGIR-- ----NDLG | WYQQKPGK APKRLIY | I-------- ASSLQS | GVPSRFSGSGSG-- TEFTLTISSLQPEDFATYYC | LQHNT----------- -----------YPWT | FGQGT KVEIK |
| iPS:392 972 | 21-225_26A2 | VK1\|A30/ JK1 | | DIQMTQSPSSLSA SVGDRVTITC | RAS--QGIR-- ----SDLG | WYQQKPGK APKRLIY | T-------- ASSLQS | GVPSRFSGSGSG-- TEFTLTFSSLQPEDFATYYC | LQHNR----------- -----------YPWT | FGQGT KVEIK |
| iPS:393 026 | 21-225_32B6 | VK1\|A30/ JK1 | | DIQMTQSPSSLSA SVGDRVTITC | RAS--QGIR-- ----NDLG | WYQQKPGK APKRLIY | I-------- ASSLQS | GVPSRFSGSGSG-- TEFTLTISSLQPEDFATYYC | LQHNS----------- -----------YPWT | FGQGT KVEIK |
| iPS:393 130 | 21-225_33C2 | VK1\|A30/ JK1 | | DIQMTQSPSSLSA SVGDRVTITC | RAS--QGIR-- ----NDLG | WYQQKPVK APKRLIY | A-------- APSLQS | GVPSRFSGSGSG-- TEFTLTISSLQPEHFATYYC | LQHNS----------- -----------YPWT | FGQGT KVEIK |
| iPS:393 812 | 21-225_6A11 | VK1\|A30/ JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPWT | FGQGT KVEIK |
| iPS:393 838 | 21-225_6G2 | VK1\|A30/ JK1 | | DIQMTQSPSSRSA FVGDRVTITY | RAS--QGIR-- ----NDLG | WYQQKPGK APQRLIY | A-------- ASSLQS | GVPSRFSGSGYG-- TEFNITISSLQPEDFAIYYC | IQHNS----------- -----------YLWT | FGQGT KVEIT |
| iPS:393 864 | 21-225_4C5 | VK1\|A30/ JK1 | | DIQMTQSPSSLSA SVGHRVHLTC | RAS--RGIR-- ----GDLG | WYRQKPGK APKRLIY | A-------- ASNLQS | GVPSRFSGSGYG-- TEFTLTIGSLQPEDFATYYC | LQHYS----------- -----------YPRT | FGQGT KVEIK |
| iPS:393 868 | 21-225_9C11 | VK1\|A30/ JK1 | | DIQMTQSPSSLSA SVGDRVTITC | RAS--QNIR-- ----NYLN | WYQQKSGR APKLLIY | V-------- ASSLQS | GVPSRFSGSGSG-- TEFTLTINSLQPEDFAIYHC | HQSNS----------- -----------TPLT | FGQGT KVEIK |
| iPS:393 876 | 21-225_9A1 | VK1\|A30/ JK1 | | DIQMTQSPSSRSA FVGDRVTITC | RAS--QGIR-- ----NDLG | WYQQKPGK ARKRLIY | T-------- ASSLQS | GVPSRFSGSGYG-- TEFTITISSLQPEDFATYYC | IQHNS----------- -----------YLWT | FGQGT KVEIK |
| iPS:393 902 | 21-225_14E10 | VK1\|A30/ JK1 | | DIQMTQSPSSLSA SVGDRVTITC | RTS--QGIR-- ----NDLG | WYQHKPGQ APKRLIY | A-------- ASSLQS | GVPSRFSGSGSG-- TEFTLTISSLQPEDFTAYYC | LQHYS----------- -----------YPRT | FGQGT KVEIK |
| iPS:393 908 | 21-225_10E9 | VK1\|A30/ JK1 | | DIQMTQSPSSLSA SVGDRVTFTC | RAS--QDIR-- ----SDLG | WYQQKPGK APTRLIF | A-------- ASSLQS | GVPSRFSGSGSG-- TEFTLTINSLQPEDFATYYC | LQHYS----------- -----------YPRT | FGQGT KVEIK |
| iPS:393 916 | 21-225_2G4 | VK1\|A30/ JK1 | | DIQMTQSPSSLSA SVGDRVTITC | RAS--QGIR-- ----NDLG | WYQQKPGK AFTRLIY | A-------- ASSLHS | GVPSRFSGSGSG-- TEFTLTISSLQPEDLATYYC | LQHYS----------- -----------FPRT | FGRGT KVEIK |
| iPS:393 948 | 21-225_16A5 | VK1\|A30/ JK1 | | DIQMTQSPSSLSA SVGDRVTITC | RAS--QGIR-- ----NDLG | CYQQKPGK APKRLIY | A-------- ASSLQS | GVPSRFSGNGYG-- TEFTLTISSLQPEDFATYYC | LQHNS----------- -----------YPWT | FGQGT KVEIK |
| iPS:393 960 | 21-225_7G2 | VK1\|A30/ JK1 | | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNS----------- -----------YPWT | FGLGT KVVIK |
| iPS:393 966 | 21-225_7F8 | VK1\|A30/ JK1 | | DIQMTQSPSSLSA SVGDRVTITC | RAS--QGIG-- ----NDLG | WYQQKPGK APKRLIY | A-------- ASSLQS | GVPSRFSGSVSG-- TEFTLPISSLQPEDFATYYC | LQHYT----------- -----------YPRT | FGQGT KVEIK |
| iPS:393 972 | 21-225_7C9 | VK1\|A30/ JK1 | | DIQMTQSPSSLSA SGGDRVTITC | RAS--QGIR-- ----NDLG | WYQQKPGK APKRLIY | A-------- ASSLQS | GVPSRFSGSGSG-- TEFTLTISSLQPEDFATYYC | LQLYS----------- -----------YPRT | FGQGT KVDIK |

| ID | Clone | Germline | | K_FR1 | K_CDR1 | K_FR2 | K_CDR2 | K_FR3 | K_CDR3 | K_FR4 |
|---|---|---|---|---|---|---|---|---|---|---|
| iPS:393 978 | 21-225_4C12 | VK1|A30/JK1 | | DIQMTQSPSSLSA SVGDRVTITC | RAS--QGIR-- ----NDLG | WYQQKPGK APTRLIY | A-------- ASSLHS | GVPSRFSGSGSG-- TEFTLTISSLQPEDLATYYC | LQHYS----------- ------------FPRT | FGQGT KVEIK |
| iPS:393 986 | 21-225_7G4 | VK1|A30/JK1 | | DIQMTQSPSSLSA SVGDRVTITC | RAS--QGIR-- ----NDLG | WYQQRPGK APKRLIY | A-------- ASSLQS | GVPSRFSGSGSG-- TEFTLTISSLQPEDSATYYC | LHQYS----------- ------------YPRT | FGQGT KVEIK |
| iPS:393 996 | 21-225_15C11 | VK1|A30/JK1 | | DIQMTQSPSSLSA SVGDRVTITC | RAS--QGIR-- ----NDLG | WYQQKPGK VPKRLIY | A-------- ASSLQS | GVPSRFSGSGSG-- TEFTLTISSLQPEDFATYYC | LLHYS----------- ------------YPRT | FGRGT KVEIK |
| iPS:393 998 | 21-225_12B12 | VK1|A30/JK1 | | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNS----------- ------------YPWT | FGLGT KVVIK |
| iPS:394 041 | 21-225_5E5 | VK1|A30/JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHYS----------- ------------YPRT | FGQGT KVEVK |
| iPS:394 067 | 21-225_12F2 | VK1|A30/JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDVTYYC | LQHNS----------- ------------YPWT | FGQGT KVEIK |
| iPS:394 089 | 21-225_12E6 | VK1|A30/JK1 | | DIQMTQSPSSLSA SVGDRVTITC | RAS--QGIR-- ----SDLG | WYQQKPGK APKRLIY | A-------- ASSLQS | GVPSRFSGSGSG-- TEFTLTISSLQPEDFATYYC | LQHNS----------- ------------YPWT | FGQGT KVEIK |
| iPS:394 093 | 21-225_9D12 | VK1|A30/JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPWT | FGQGT KVEIK |
| iPS:394 095 | 21-225_16H4 | VK1|A30/JK1 | | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNS----------- ------------YPWT | FGQGT KVEIK |
| iPS:394 097 | 21-225_16G7 | VK1|A30/JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPWT | FGQGT KVEIK |
| | | Germline | K_FR1 | K_CDR1 | K_FR2 | K_CDR2 | K_FR3 | K_CDR3 | K_FR4 | |
| | VK3|L2/JK2 | | 32140 | EIVMTQSPATLSV SPGERATLSC | RAS--QSVS-- ----SNLA | WYQQKPGQ APRLLIY | G-------- ASTRAT | GIPARFSGSGSG-- TEFTLTISSLQSEDFAVYYC | QQYNN----------- ----------WPYT | FGQGT KLEIK |
| iPS:468 828 | 21-225_162A10 | VK3|L2/JK2 | | EIVMTQSPATLSV SPGERATLSC | RAS--QTVN-- ----SNLA | WYQQKSGQ APRLLIF | G-------- ASTRAT | VIPARINGSGSG-- TEFTLTISSLRSEDFAVYFC | QQYND----------- ----------WPCS | FGQGT KLEIK |
| iPS:434 255 | 21-225_62E6 | VK3|L2/JK2 | | EIVMTQSPATLSV SPGERATLSC | RAS--QSVN-- ----SNLA | WYQQKPGQ APRLLIS | V-------- ASTRAT | GIPARFNGSGSG-- TEFTLTISSLQSEDFAVYYC | QQYND----------- ----------WPCS | FGQGT KLEIK |
| iPS:434 269 | 21-225_57H3 | VK3|L2/JK2 | | EIVMTQSPATLSV SPGERATLSC | RAS--QSVS-- ----SSLA | WYQQKPGQ APRLLIY | G-------- ASTRAT | GFPARFNGSGSW-- TEFTLTISSLQSEDFAIYYC | QQYND----------- ----------WPCS | FGQGT KLEIK |
| iPS:434 363 | 21-225_65A6 | VK3|L2/JK2 | | EIVMTQSPVTLFV SPGERATLSC | RAS--QSVN-- ----SNLA | WYQQKPGQ APRLLIY | G-------- ASTRAT | GIPARFNGSGSG-- TEFTLTISSLQSEDFAVYYC | QQYND----------- ----------WPCS | FGLET KLEIK |
| iPS:434 393 | 21-225_67C3 | VK3|L2/JK2 | | EIVMTQSPATLSV SPGERATLSC | RAS--QSVN-- ----SNLA | WYQQKPGQ APRLLIS | I-------- ASTRAT | GIPARFNGSGSG-- TEFTLTISSLQSEDFAVYYC | QQYND----------- ----------WPCS | FGQGT KLEIK |
| iPS:434 425 | 21-225_70A5 | VK3|L2/JK2 | | EIVMTQSPATLSV SPGERATLSC | RAS--QSVN-- ----SNLA | WYQLKPGQ APRLLIS | I-------- ASTRAT | GIPPRFNGSGSG-- TEFTLTISSLQSEDFAVYYC | QQYND----------- ----------WPCS | FGQGT KLEIK |
| iPS:434 485 | 21-225_76D2 | VK3|L2/JK2 | | EIVMTQSPATPSV SPGERATLSC | RAS--VSVV-- ----NSLA | WYQQKPGQ APRLLIH | G-------- ASTRAT | GIPARFSGSGSG-- TEFTLTISSVQSEDFAIYYC | QQYND----------- ----------WPCS | FGQGT KLEIK |
| iPS:434 537 | 21-225_74E11 | VK3|L2/JK2 | | EIVMTQSPATLSV SPGERATLSC | RAS--LSVV-- ----NSLA | WYQQKPGQ APRLLIH | G-------- ASTRAT | GIPARFSGSGSG-- TEFTLTISSLQSEDFAIYYC | QQYND----------- ----------WPCS | FGQGT KLEIK |
| iPS:434 569 | 21-225_77H5 | VK3|L2/JK2 | | EIVMTQSPVTLSV SPGERATLSC | RAS--QSVS-- ----SSLA | WYQQKPGL APRLLIY | G-------- ASTRAT | GIPARFSGSGSG-- TEFSFTISSLQSEDFAVYFC | QQYND----------- ----------WPCS | FGQGS KLEIQ |
| iPS:434 629 | 21-225_74C3 | VK3|L2/JK2 | | EIVMTQSPATLSV SPGERATLSC | RAS--QSVA-- ----SSLA | WYQQKPGQ APRLLIF | G-------- TSTRAT | GIPARFSGSGSG-- TEFTLIISSLQSEDFAVYYC | QQYND----------- ----------WPCS | FGLGT KLEIK |
| iPS:434 673 | 21-225_74E3 | VK3|L2/JK2 | | EIVMTQSPATLSL SPGERATLSC | RAS--LSVV-- ----NSLA | WYQQKPGQ APRLLIY | G-------- ASTRAT | GIPARFSGSGSG-- TEFTLTISSLQSEDFAIYYC | QQYND----------- ----------WPCS | FGQGT KLEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:435 109 | 21-225_92H5 | VK3\|L2\|J K2 | | EIVMTQSPATLSV SPGERATLSC | RAS--QDVI-- ----TYLA | WYQQKPGQ APRLLIY | G-------- ASTRAT | GVPARFSGSGSG-- TEFTLTITSLQSEDFALYYC | QEYND----------- ------------WPCS | FGQGT KLEIK |
| IPS:435 221 | 21-225_95G2 | VK3\|L2\|J K2 | | EIVMTQSPATLSL SPGERATLSC | RAS--MSVV-- ----NSLA | WYQQKPGQ APRLLIY | G-------- ASTRAT | GIPARFSGSGSG-- TEFTLTISSLQSEDFAIYYC | QQYND----------- ------------WPCS | FGQGT KLEIK |
| iPS:436 051 | 21-225_193G1 2 | VK3\|L2\|J K2 | | EIVMTQSPATLSV SPGERATLSC | RAS--QSVS-- ----SSLA | WYQQKPGQ APRILIS | G-------- ASTRAT | GIPARFSGSGSG-- TEFTLTISSLQSADFAVYYC | QQYNN----------- ------------WPCS | FGQGT KLEIK |
| iPS:436 236 | 21-225_201F7 | VK3\|L2\|J K2 | | EIVMTQSPATLSV SPGERATLSC | RAS--QNIK-- ----NNLA | WYQQKPGQ APRLLIF | G-------- ASTRAT | GIPARFSGSGSG-- TEFTLTISSLQSEDFAVYYC | QQFYN----------- ------------WLCS | FGQGT KLELK |
| iPS:436 250 | 21-225_201A4 | VK3\|L2\|J K2 | | EIVMTQSPATLSV SPCESATLSC | RSS--QNIK-- ----SNLA | WYQQKPGQ APRLLIF | G-------- ASTRAT | GIPARFSGSGSG-- TEFTLTISSLQSEDFAVYYC | QQFYN----------- ------------WLCS | FGQGT KLELK |
| iPS:436 252 | 21-225_202A8 | VK3\|L2\|J K2 | | EIVMTQSPATLSV SPGERATLSC | RAS--QRIN-- ----NNLA | WYQQNPGQ APRLLIY | G-------- ASTRAT | GVPARFSGSGSG-- TEFTLTISSLQSEDFTVYYC | QQYYN----------- ------------WLCS | FGQGT KLEIR |
| iPS:436 258 | 21-225_202F12 | VK3\|L2\|J K2 | | EIVMTQSPATLSV SPGERATLSC | RAS--QSVL-- ----NNLA | WYQQRPGQ APRLLIY | G-------- ASTRAT | GIPARFSGSGSG-- TEFTLTISSLQSEDFAVYYC | QQYDN----------- -----------WPPCS | FGQGT KLEIK |
| iPS:436 278 | 21-225_201F2 | VK3\|L2\|J K2 | | EIVMTQSPATLSV SPGERATLSC | RAS--QNIK-- ----SNLA | WYQQKPGQ APRLLIF | G-------- ASTRAT | GIPARFSGSGSG-- TEFTLTISSLQSEDFAVYYC | QQFYN----------- ------------WLCS | FGQGT KLELK |
| iPS:436 294 | 21-225_205G4 | VK3\|L2\|J K2 | | EIVMTQSPATLSV SPGERATLSC | RAS--QNIK-- ----SNLA | WYQQKPGQ APRLLIF | G-------- ASTRAT | GIPARFSGSGSG-- TEFTLTISSLQSEDFAVYYC | QQFYN----------- ------------WLCS | FGQGT KLELK |
| iPS:436 306 | 21-225_201H4 | VK3\|L2\|J K2 | | EIVMTQSPATLSV SPGERATLSC | RAS--QSVN-- ----SYLA | WYQQKPGQ APRLLIY | G-------- ASTRAT | GIPARFSGSGSG-- TEFTLTISSLQSEDFAVYYC | QEYND----------- ------------WPCS | FGQGT NLEIK |
| iPS:436 356 | 21-225_210H1 0 | VK3\|L2\|J K2 | | EIVMTQSPATLSV SPGERATLSC | RAS--QSVK-- ----SNLA | WYQQKPGQ APRLLIY | G-------- ASTRAT | GIPARFSGSGSG-- TEFTLTISSLQSEDFAVYSC | QQYYN----------- ------------WLCS | FGQGT KLEIK |
| iPS:436 362 | 21-225_212C1 0 | VK3\|L2\|J K2 | | EIVMTQSPATLSV SPGERATLSC | RAS--QSVA-- ----SSLA | WYQQKPGQ APRLLIH | G-------- TSTRAT | DVPARFSCFGSG-- SDFTLTISSLQSEDFAVYSC | QQYND----------- ------------WPCS | FGQGT KLEIK |
| iPS:436 434 | 21-225_216B1 0 | VK3\|L2\|J K2 | | EIVMTESPATLSV SPGERATLSC | RAS--QSVN-- ----NNLA | WYRQKPGQ APRLLIY | G-------- ASTRAT | GIPPRFSGSGSG-- TEFTLSISSLQSEDFAVYYC | QQYND----------- ------------WPCS | FGQGT KLEIR |
| iPS:392 806 | 21-225_24H3 | VK3\|L2\|J K2 | | EIVMTQSPATLSV SPGERATLSC | RAS--QSVS-- ----SNLA | WYQQKPGQ APRLLIY | F-------- ASIRAT | GIPARFSGSGSG-- TEFTLTISSLQSEDFAVYYC | QQYNN----------- -----------WPMCS | FGQGT KLEIK |
| Germline | | | | K_FR1 | K_CDR1 | K_FR2 | K_CDR2 | K_FR3 | K_CDR3 | K_FR4 |
| | VK3\|L2\|JK4 | | 32141 | EIVMTQSPATLSV SPGERATLSC | RAS--QSVS-- ----SNLA | WYQQKPGQ APRLLIY | G-------- ASTRAT | GIPARFSGSGSG-- TEFTLTISSLQSEDFAVYYC | QQYNN----------- ------------WPLT | FGGGT KVEIK |
| IPS:468 830 | 21-225_191G1 1 | VK3\|L2\|J K4 | | EIVMTQSPATLSV SPGERANLSC | RTS--QSVW-- ----ISVA | WYHQKPGQ APRLLIY | G-------- AATRAT | GIPARFSGSGSG-- TEFTLTISSLQSEDFAVYYC | QQYNY----------- ------------WPLT | FGGGT KVEIK |
| iPS:434 147 | 21-225_55E1 | VK3\|L2\|J K4 | | EIVMTQSPATLSV SPGERATLSC | RAS--QSVS-- ----SDLA | WYQLKPGQ APRLLIY | D-------- ASARAT | GIPARFSGSGSG-- TEFTLTISSLQSEDFAFYYC | QQYYN----------- ------------WPLT | FGGGT KVEIK |
| iPS:434 621 | 21-225_74D1 | VK3\|L2\|J K4 | | EIVMTQSPATLSV SPGERATLSC | RAS--QSVS-- ----RNLA | WFQQKPGQ APRLLIY | G-------- ASTRAT | GIPARFSGSGSG-- TEFTLTIYSLQSEDFAVYYC | QQYNN----------- -----------WPPLT | FGGGT KVEIK |
| iPS:435 821 | 21-225_190E1 1 | VK3\|L2\|J K4 | | EIVMTQSPATLSV SPGERATLSC | RAS--QSFR-- ----INLA | WYQQRPGQ APRLLIY | G-------- ASTRAT | GIPARFSGSGSG-- TEFTLTISSLQSEDFAVYYC | QQYNN----------- ------------WPLT | FGGGT KVEIK |
| iPS:435 941 | 21-225_191E8 | VK3\|L2\|J K4 | | EIVMTQSPATLSV SPGERATLSC | RPS--QSFS-- ----RNLA | WYQQKPGQ APRLLIY | G-------- ASTRAT | GIPSRFSGSGSG-- TEFTLTISSLESEDFAVYYC | QQYNN----------- ------------WPLT | FGGGI KVEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:436 203 | 21-225_199A6 | VK3\|L2/JK4 | | DIVMTQSPAILSV SPGDRATLSC | RPS--QSFS-- ----RNLA | WYQQKPGQ APRLLIY | G-------- ASTRAT | GIPARFSGSGSG-- TEFTLTISSLECEDFAVYYC | QQYNN----------- -----------WPLT | FGGGT KVEIK |
| iPS:437 334 | 21-225_75F11 | VK3\|L2/JK4 | | EIVMTQSPAILFV SPGERATLSC | RAS--QSVS-- ----RNLA | WFQQKPGQ APRLLFY | G-------- ASIRAT | GIPARFSGSGSG-- TEFTLTIYSLQYEDFAVYYC | QQYNN----------- ----------WPPLT | FGGGT KVEIK |
| iPS:448 904 | 21-225_65C12 | VK3\|L2/JK4 | | EIVMTQSPAILSV FPGEGATLSC | RAS--QSVS-- ----INLA | WYQQKPGQ APRLLIY | G-------- ASTRAT | GIPARFNASGSG-- TEFTLSISSLQSENFAVYYC | QQYNT----------- -----------WPLT | FGGGT KVEIK |
| | Germline | | | K_FR1 | K_CDR1 | K_FR2 | K_CDR2 | K_FR3 | K_CDR3 | K_FR4 |
| | VK1\|A30/JK3 | | 32142 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPFT | FGPGT KVDIK |
| iPS:468 832 | 21-225_76H10 | VK1\|A30/ JK3 | | DIQMTQSPSSLSA SAGDRVTITC | RAS--QDIR-- ----NYLG | WYQQKPGK APKRLIY | G-------- ASSLQS | GVPSRFSGSGSG-- TEFTLTISSLQPEDFATFYC | LQYNS----------- -----------YPFT | FGPGT KVDIK |
| iPS:468 836 | 21-225_198E3 | VK1\|A30/ JK3 | | DIQMTQSPSSLSA SVGDRVTITC | RAS--QGIR-- ----KDLG | WYQQKPGK APKRLIY | A-------- ASSLQS | GVPSRFSGSGSG-- TEFTLTISSLQPEDFATYVC | LQHYR----------- -----------YPFT | FGPGT KVDFK |
| iPS:468 844 | 21-225_48E10 | VK1\|A30/ JK3 | | DIQMTQSPSSLSA SVGDRVTITC | RAS--QGIR-- ----SDLC | WYQQKPGK APKRLIY | T-------- ASSLQS | GVPSRFSGSGSG-- TEFTLTISSLQPEDFATYYC | LQYNS----------- -----------YPFT | FGPGT KVDIK |
| iPS:468 846 | 21-225_53B10 | VK1\|A30/ JK3 | | DIQMTQSPSSLSA SAGDRVTITC | RAS--QDIR-- ----NYLG | WYQQKPGK APKRLIY | G-------- ASSLQS | GVPSRFSGSGSG-- TEFTLTISSLQPEDFATFYC | LQYNS----------- -----------YPFT | FGPGT KVDIK |
| iPS:433 895 | 21-225_43E1 | VK1\|A30/ JK3 | | DIQMTQSPSSLSA SVGDRVTITC | RAS--QGIR-- ----NDLG | WYQKKPGK APKRLIN | A-------- ASSLQS | GVPSRFSGSGSG-- TEFTLTISSLQPEDFATYYC | LQHNS----------- -----------YPFT | FGPGT KVDIK |
| iPS:433 905 | 21-225_43E5 | VK1\|A30/ JK3 | | DIQMTQSPSSLSA SVGDRVTITC | RAS--QGIR-- ----NDLG | WYQQKPGK APKRLIY | G-------- ASNLQS | GVPSRFSGSGSG-- TEFTLTVSSLQPEDFATYFC | LQHTS----------- -----------FPFT | FGPGT KVDIK |
| iPS:433 913 | 21-225_43H8 | VK1\|A30/ JK3 | | DIQMTQSPSSLSA SVGDRVTITC | RAS--QGIR-- ----NDLG | WHQQKPGK APKRLIY | A-------- ASSLQS | GVPSRFSGSGSG-- TEFTLSISSLQPEDFATYYC | LQHNS----------- -----------FPFT | FGPGT KVDIK |
| iPS:433 933 | 21-225_44C8 | VK1\|A30/ JK3 | | DIQMTQSPSSLSA SVGDRVTITC | RAS--QGIR-- ----NDLG | WYQQKPGK APKRLIY | A-------- ASNLQS | GVPSRFSGSRSG-- TEFTLTISSLQPEDFATYYC | LQHNS----------- -----------YPFT | FGPGT KVDIK |
| iPS:433 949 | 21-225_45H2 | VK1\|A30/ JK3 | | DIQMTQSPSSLSA SVGDRVTITC | RAS--QGIR-- ----NDLC | WYQQKPGK APKRLIY | G-------- ASNLQS | GVPSRFSGSGSG-- TEFTLTVSSLQPEDFATYFC | LQHTS----------- -----------FPFT | FGPGT KVDIK |
| iPS:433 981 | 21-225_46E9 | VK1\|A30/ JK3 | | DIQMTQSPSSLSA SVGDRVTITC | RAS--QGIR-- ----NDLG | WYQQKPGK APKRLIY | A-------- ASSLQS | GVPSRFSCSGSG-- TEFTLTVSSLQPEDFATYFC | LQHTS----------- -----------FPFT | FGPGT KVDIK |
| iPS:433 995 | 21-225_47H7 | VK1\|A30/ JK3 | | DIQMTQSPSSLSA SVGDRVTITC | RTS--QGIR-- ----NDLG | WYQKKPGK APKRLIY | A-------- ASSLQS | GVPSRFSGSGSG-- TEFTLTVSSLQPEDFATYFC | LQHTS----------- -----------FPFT | FGPGT KVDIK |
| iPS:434 039 | 21-225_43B1 | VK1\|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTVSSLQPEDFATYFC | LQHTS----------- -----------FPFT | FGPGT KVDIK |
| iPS:434 057 | 21-225_51E4 | VK1\|A30/ JK3 | | DIQMTQSPSSLSA SVGDRVTITC | RAS--QGIR-- ----NDLG | WYQQKPGK APQRLIY | A-------- ASSLQS | GVPSRFSGSGSG-- TEFTLTISSLQPEDFAAYYC | LQHNS----------- -----------YPFT | FGPGT KVDIK |
| iPS:434 071 | 21-225_51F9 | VK1\|A30/ JK3 | | DIQMTQSPSSLSA SVGDRVTITC | RAS--QGIR-- ----TDLG | WYQQKPRK APQRLIY | A-------- ASSLQR | GVPSRFSGSGSG-- TDFTLTISSLQPEDFASYYC | LQHNS----------- -----------YPFT | FGPGT KVDIK |
| iPS:434 075 | 21-225_51B11 | VK1\|A30/ JK3 | | AIQMTQSPSSLSA SVGDRVTITC | RTS--QGIR-- ----NDLG | WYQQKPRK APKRLIY | A-------- ASSLQS | GVPSRFSGSGSG-- TEFTLTISSLQPEDFATYYC | LQHNS----------- YPFT | FGPGT KVDIK |
| iPS:434 091 | 21-225_52B9 | VK1\|A30/ JK3 | | AIQMTQSPSSLSA SVGDRVTITC | RAS--QGIR-- ----NDLG | WYQQKPRK APKRLIY | A-------- ASSLQS | GVPLRFSGSGSG-- TEFTLTIRSLQPEDFATYYC | LQHNS----------- -----------YPFT | FGPGT KVDIK |
| iPS:434 101 | 21-225_52H12 | VK1\|A30/ JK3 | | DIQMTQSPSSLSA SVGDRVTITC | RAS--QGIR-- ----NNLG | WYQQKPGK APKRLIY | G-------- ASSLQS | GVPSRFSGSGSG-- TEFTLTISSLQPEDFATYYC | LQYNS----------- -----------YPFT | FGPGT KVDIK |
| iPS:434 103 | 21-225_53G1 | VK1\|A30/ JK3 | | DIQMTQSPSSLSA SVGDRVTITC | RAS--QGIR-- ----NDLG | WYQQKPGK APKRLIY | P-------- ASSLQS | GVPSRFSGSGSG-- TEFSLTISSLQPEDFATYYC | LQDNS----------- -----------YPFT | FGPGT KVDIK |
| iPS:434 129 | 21-225_53B12 | VK1\|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPFT | FGPGT KVDIK |

| | | | | | | | | | |
|---|---|---|---|---|---|---|---|---|---|
| iPS:434 131 | 21-225_54D3 | VK1\|A30/JK3 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPFT | FGPGT KVDIK |
| iPS:434 143 | 21-225_54G7 | VK1\|A30/JK3 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LHHNT----------- -----------YPFT | FGPGT KVDIK |
| iPS:434 155 | 21-225_55B3 | VK1\|A30/JK3 | DIQMTQSPSSLSA SVGDRVTITC | RAS--QGIR-- ----NDLG | WYQQKPGK APKRLIY | A-------- ASSLQS | GVPSRFSGSRSG-- TEFTLTISSLQPEDFASYYC | LQHNS----------- -----------YPFT | FGPGT KVDIK |
| iPS:434 169 | 21-225_50C4 | VK1\|A30/JK3 | DIQMTQSPSSLSA SVGDRVTLTC | RAS--QGIR-- ----NDLG | WYQQKPGI APKRLIY | A-------- ASSLQS | GVPSRFSGSGSG-- TEFTLTISSLQPEDFATYYC | LQHNR----------- -----------YPFT | FGPGT KVDIK |
| iPS:434 187 | 21-225_56A5 | VK1\|A30/JK3 | DIQMTQSPSSLSA SVGDRVTITC | RAS--QDIR-- ----NLLG | WYQQKPGK APKRLIY | A-------- ASSLQS | GVPSRFSGSGSG-- TEFTLTISSLQPEDFATYYC | LQYNS----------- -----------YPFT | FGPGT KVDIR |
| iPS:434 199 | 21-225_59F11 | VK1\|A30/JK3 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNR----------- -----------YPFT | FGPGT KVDFK |
| iPS:434 207 | 21-225_60A3 | VK1\|A30/JK3 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- -----------YPFT | FGPGT KVDIK |
| iPS:434 251 | 21-225_62G3 | VK1\|A30/JK3 | DIHMTQSPSSLSA SVGDRVTITC | RAS--QDIR-- ----NNLG | WYQQKPGK APKRLIY | T-------- ASSLQS | GVPSRFSGSGSG-- TEFTLTISSLQPEDFATYYC | LQYNS----------- -----------YPFT | FGPGT KVDIK |
| iPS:434 263 | 21-225_56H7 | VK1\|A30/JK3 | DIQLTQSPSSLSA SVGDRVTITC | RAS--QDIR-- ----NDLG | WYQQRPGK APKRLIY | P-------- ASSLQS | GVPSRFSGSGSG-- TEFTLTISSLQPEDFATYYC | LQDNS----------- -----------YPFT | FGPGT KVDSK |
| iPS:434 265 | 21-225_57B2 | VK1\|A30/JK3 | DIQMTQSPSSLSV SVGDRVTITC | RAS--QGIR-- ----NALG | WYQQKPGK APKRLIY | A-------- ASSLQS | GVPSRFSGSGSG-- TEFTLTISSLQPEDFATYYC | LQHNS----------- -----------YPFT | FGPGT KVDIK |
| iPS:434 271 | 21-225_57A4 | VK1\|A30/JK3 | DIQMTQSPSSLSA SVGDRVTITC | RAS--QGIR-- ----NDLG | WYLQKPGK APKRLIY | A-------- ASSLLS | GVPSRFSGSGSG-- TEFTLTISSLQPEDFATYYC | LQHNS----------- -----------YPFT | FGPGT KVDIK |
| iPS:434 275 | 21-225_57F4 | VK1\|A30/JK3 | DIQMTQSPSSLSA SVGDRVTITC | RAS--QVIR-- ----NDLG | WYQQKPGK APKRLIY | A-------- ASSLQS | GVPSRFSGSGSG-- TEFTLTISSLQPEDFATYHC | LQYGS----------- -----------FPFT | FGPGT KVDIK |
| iPS:434 293 | 21-225_58F5 | VK1\|A30/JK3 | DIQMTQSPSSLSA SVGDRVTITC | RAS--QGIR-- ----NDLG | WFLQTPGK APKRLIY | A-------- ASSLLS | GVPSRFGGSGSG-- TEFTLTISSLQPEDFATYYC | LQHNS----------- -----------YPFT | FGPGT KVEIK |
| iPS:434 299 | 21-225_58D11 | VK1\|A30/JK3 | DIQMTQSPSSLSA SVGDRVTITC | RAS--QGIR-- ----SDLD | WYQQKPGK APKRLIY | A-------- ASSLQS | GVPSRFSGSGSG-- TEFTLAISSLRPEDFATYYC | LQHNN----------- -----------FPFT | FGPGT KVDIK |
| iPS:434 351 | 21-225_64A12 | VK1\|A30/JK3 | DIQMTQSPSSLSA SVGDRVTITC | RAS--QGIR-- ----NDLG | WYQQKPGK APTRLIY | T-------- ASTLQS | GVPSRFSGSGSG-- TEFTLTISSLQPEDFATYYC | LQHNG----------- -----------YPFT | FGPGT KVDIK |
| iPS:434 383 | 21-225_66F9 | VK1\|A30/JK3 | DIQMTQSPSSLSA SVGDRVTITC | RAS--QDIR-- ----NVLG | WYQQKPGK APKRLIY | T-------- ASSLQS | GVPSGFSGSGSG-- TEFTLTISSLQPEDFATYYC | LQYNS----------- -----------YPFT | FGPGT KVDIK |
| iPS:434 399 | 21-225_67B7 | VK1\|A30/JK3 | DIQMTQSPSSLSA SVGDRVTITC | RAS--QGIR-- ----NDLG | WFQQKPGK APKRLIY | A-------- ASSLQS | GVPSRFSGSGSG-- TEFSLTISSLQPEDFATYYC | LHHNS----------- -----------YPFK | FGPGT KVDIK |
| iPS:434 407 | 21-225_68G8 | VK1\|A30/JK3 | DIQMTQSPSSLSA SVGDRVTITC | RAS--QGIR-- ----NNLG | WYQQRPGK APKRLIY | A-------- ASSVQS | GVPSRFSGSGSG-- TEFTLTISSLQPEDFATYYC | LQYNS----------- -----------YPFT | FGPGT KVDIK |
| iPS:434 447 | 21-225_71B6 | VK1\|A30/JK3 | DIQMTQSPSSPSA SVGDRVTITC | RAS--QGIR-- ----NDLD | WYQQKPGK AFQRLIY | A-------- ASSLQS | GVPSRFSGSGSG-- TEFTLTISSLQPDDFATYYC | LQHNS----------- -----------YPFT | FGPGT KVDIK |
| iPS:434 449 | 21-225_71H6 | VK1\|A30/JK3 | DIQMTQSPSSLSA SVGDRVTITC | RAS--QGIR-- ----NVLG | WYQQKPGK APKRLIY | A-------- ASSLQS | GVPSRFSGSGSG-- TEFTLTISSLQPEDFATYYC | LQYNS----------- -----------YPFT | FGPGT KVDIK |
| iPS:434 453 | 21-225_71B11 | VK1\|A30/JK3 | DIQMTQSPSSLSA SVGDRVTITC | RAS--QGIR-- ----NDLD | WYQQTPGK APKRLIY | A-------- ASSLQS | GVPSRFSGGGSG-- TEFSLTISSLQPEDFTTYYC | LQHNT----------- -----------YPFT | FGPGT KVDVK |
| iPS:434 463 | 21-225_73A6 | VK1\|A30/JK3 | DIQMTQSPSSLSA SVGDRVTITC | RAS--QGIR-- ----NDLG | WFQQKPGK APKRLIY | A-------- ASNLQS | GVPSRFSGSGSG-- TEFTLTISSLQPEDFATYYC | LQHNS----------- -----------YPFT | FGPGT KVDIK |
| iPS:434 615 | 21-225_74A11 | VK1\|A30/JK3 | DIQMTQSPSSLSA SVGDRVTITC | RAS--QDIR-- ----NDLG | WYQQKPGK APKRLIC | A-------- ASSLQS | GVPSRFSGSGSG-- TEFTLTISSLQPEDFATYFC | LQHND----------- -----------YPFT | FGPGT KVDIK |

| iPS:434 977 | 21-225_88A5 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPEDFATYYC | LQHND----------- ------------YPFT | FGPGT KVEIK |
|---|---|---|---|---|---|---|---|---|---|---|
| iPS:435 253 | 21-225_96A4 | VK1|A30/ JK3 | | DIQMTQSPSSLSA SVGDRVTITC | RAS--QDIR-- ----NDLG | WYQQKPGK APKRLIY | G-------- VSSLQS | GVPSRFSGSGSG-- TEFTLTIISSLQREDFATYYC | LQHND----------- ------------YPFT | FGRGT KVDIK |
| iPS:435 511 | 21-225_157C3 | VK1|A30/ JK3 | | DIQMTQSPSSLSA SVGDRVTITC | RAS--QGIR-- ----NDLC | WYQQKPGK APKRLIY | A-------- ASSLQS | GVPSRFSGSGSG-- TDFTLTIISSLQPDDFATYYC | LQHNS----------- -----------YPFT | FGPGT KVDIK |
| iPS:435 521 | 21-225_157H4 | VK1|A30/ JK3 | | DIQMTQSPSSLSA SVGDRVTITC | RAS--QGIR-- ----NDLG | WYQQKPGK APKRLIY | P-------- ASSLQT | GVPSRFSGSGSG-- TEFTLTIISSLQPEDFATYYC | LQDNS----------- ------------YPFT | FGPGT KVDIK |
| iPS:435 527 | 21-225_157G7 | VK1|A30/ JK3 | | DIQMTQSPSSLCA SVGDRVTITC | RAS--QGIR-- ----NDLG | WYQQKPGK APKRLIN | V-------- ASSLQS | GVPSRFSGSGSG-- TEFTLTIISSVQREDFATYYC | IQDNS----------- ------------HPFT | FGPGT KVEIK |
| iPS:435 533 | 21-225_157H8 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TDFTLTIISSLQPDDFATYYC | LQHNS----------- ------------YPFT | FGPGT KVDIK |
| iPS:435 537 | 21-225_157H1 2 | VK1|A30/ JK3 | | DIQMTQSPSSLSA SVGDRVTITC | RAS--QGIR-- ----NDFG | WYQQRPGK APKCLIH | A-------- ASSLQS | GVPSRFSGSGSG-- TEFTLTIISSLQPEDFATYYC | LQHYS----------- ------------YPFT | FGPGT KVDIK |
| iPS:435 547 | 21-225_158F5 | VK1|A30/ JK3 | | DIQMTQSPSSLSA SVGDRVTITC | RAS--QGIR-- ----NDLG | WYQQKPGK APKRLIN | A-------- ASSLQS | GVPSRFSGSGSG-- TEFTLTIISSLQPEDFATYYC | LQDNS----------- ------------HPFT | FGPGT KVEIK |
| iPS:435 551 | 21-225_158H6 | VK1|A30/ JK3 | | DIQMTQSPSSLSA SVGDRVTITC | RAS--QGIR-- ----SDLC | WYQQKPGK APKRLIY | T-------- ASSLQS | GVPSRFSGSGSG-- TEFTLTIISSLQPEDFATYYC | LQHNS----------- ------------YPFT | FGPGT KVDIK |
| iPS:435 553 | 21-225_158G8 | VK1|A30/ JK3 | | DIQMTQSPSSLSA SVGDRVTITC | RAS--QDIR-- ----NDLG | WYQQKPGK APKRLMY | T-------- ASSLQS | GVPSRFSGSGSG-- TEFTLTIISSLQPEDFATYYC | LQHNS----------- ------------YPFT | FGPGT KVDIK |
| iPS:435 569 | 21-225_159C5 | VK1|A30/ JK3 | | DIQMTQSPSSLSA SVGDRVTITC | RTS--QGIR-- ----NDLG | WYQQKPGK APKRLIY | A-------- ASSLQS | GVPSRFSGSGSG-- TDFTLTIISSLQPDDFATYYC | LQHNS----------- ------------YPFT | FGPGT KVDIK |
| iPS:435 593 | 21-225_160F4 | VK1|A30/ JK3 | | DIQMTQSPSSPSA SVGDRVTITC | RAS--QGIR-- ----NDLG | WYQQKPGK APKRLIN | V-------- ASSLQS | GVPSRFSGSGSG-- TEFTLTIISSVQREDFATYYC | IQDNS----------- ------------HPFT | FGPGT KVEIK |
| iPS:435 609 | 21-225_161F7 | VK1|A30/ JK3 | | DIQMTQSPSSLSA SVGDRVTITC | RAS--QGIR-- ----NDLG | WYQQKPGE APKRLIY | A-------- ASTLQS | GVPSRFSGSGSG-- AEFTVTIGSVQREDFATYYG | LLYIR----------- ------------YPFT | FGRGT KVDIK |
| iPS:435 613 | 21-225_161D1 1 | VK1|A30/ JK3 | | DIQMTQSPSSLSA SVGDRVTITC | RAS--QGIR-- ----NDLG | WYQQKPGE APKRLIY | A-------- ASTLQS | GVPSRFSGSGSG-- AEFTLTIGSVQREDFATYYG | LQYNR----------- ------------YPFT | FGRGT KVDIK |
| iPS:435 617 | 21-225_162F2 | VK1|A30/ JK3 | | DIQMTQSPSSLCA SVGDRVTITC | RAS--QGIR-- ----NDLG | WYQQKPGK APKRLIN | A-------- ASSLQS | GVPSRFSGSGSG-- TEFTLTIISSVQPEDFATYYC | IQDNS----------- -----------HPFT | FGPGT KVEIK |
| iPS:435 621 | 21-225_162H3 | VK1|A30/ JK3 | | DIQMTQSPSSLSA SVGDRVTITC | RAS--QGIR-- ----NDLG | WYQQKPGK APKRLIN | A-------- ASSLQS | GVPSRFSGSGSG-- TEFTLTIISSLQPEDFATYYC | LQDNS----------- ------------HPFT | FGPGT KVEIK |
| iPS:435 637 | 21-225_163E2 | VK1|A30/ JK3 | | DIQMTQSPSSLSA SVGDRVTITC | RAS--QDIR-- ----NDLG | WYQQKPGK APTRLIY | P-------- ASSLQS | GVPSRFSGSGSG-- TEFTLSIISSLQPEDFATYYC | LQHNS----------- -----------HPFT | FGPGT KVDIK |
| iPS:435 641 | 21-225_163F9 | VK1|A30/ JK3 | | DIQMTQSPSSLSA SVGDRVTITC | RAS--QDIR-- ----DDLG | WYQQKPGK APKRLIY | P-------- ASSLQS | GVPSRFSGSGSG-- TEFTLTIISSLQPEDFAAYYC | LQDNS----------- ------------YPFT | FGPGT KVDIK |
| iPS:435 643 | 21-225_163G1 0 | VK1|A30/ JK3 | | DIQMTQSPSSLSA SVGDRVTITC | RAS--QDIR-- ----NNLG | WYQQKPGK APKRLIY | P-------- ASSLQS | GVPSRFSGSGSG-- TEFTLTIISSLQPEDFATYYC | LQDYS----------- ------------YPFT | FGPGT KVDIK |
| iPS:435 719 | 21-225_171A1 1 | VK1|A30/ JK3 | | DIQMTQSPSSLSA SVGDRVTITC | RAS--QGIR-- ----NNLG | WYQQKPGK APKRLIY | P-------- ASSLQS | GVPSRFSGSGSG-- TEFTLTIISSLQPEDFATYYC | LQDHS----------- ------------YPFT | FGPGT KVDIK |
| iPS:435 769 | 21-225_177B6 | VK1|A30/ JK3 | | DIQMTQSPSSLSA SVGDRVTITC | RAS--QDIS-- ----NDLG | WYQQKPGK APKRLIY | A-------- ASSLQS | GVPSRFSGSGSG-- TEFTLTINSLQPEDFATYYC | LQLNS----------- ------------YPFT | FGPGT KVDIK |

| | | | | | | | | | | |
|---|---|---|---|---|---|---|---|---|---|---|
| iPS:435 791 | 21-225_180H7 | VK1|A30/ JK3 | | DIQMTQSPSSLSA SVGDRVTITC | RAS--QGIR-- ----NDLG | WYQQKPGK APKRLIY | T-------- ASSLQS | GVPSRFSGSGSG-- TEFTLTIISSLQPEDFASYYC | LQHNS----------- ------------YPFT | FGPGT KVDFK |
| iPS:435 805 | 21-225_181A8 | VK1|A30/ JK3 | | DIQMTQSPSSLSA SVGDRVTITC | RAS--QGIR-- ----NDLG | WYQQKPGK APKRLIY | T-------- ASSLQS | GVPSRFSGSGSG-- TDFTLTIISSLQPEDFASYYC | LQHNS----------- ------------YPFT | FGPGT KVDFK |
| iPS:435 879 | 21-225_184H1 0 | VK1|A30/ JK3 | | DIQMTQSPSSLSA SVGDRVTITC | RAS--QGIR-- ----NDLG | WYQQKPGK APKRLIY | I-------- ASSLQS | GVPSRFSGSGSG-- TEFTLTIISSLQPEDFATYYC | LQHNS----------- ------------YPFT | FGPGT KVDIK |
| iPS:435 881 | 21-225_184D1 1 | VK1|A30/ JK3 | | DIQMTQSPSSLSA SVGDRVTITC | RAS--QGIR-- ----NDLG | WYQQKPGK APKRLIY | I-------- ASSLQS | GVPSRFSGSGSG-- TEFTLTIISSLQPEDFATYYC | LQHNS----------- ------------YPFT | FGPGT KVDIK |
| iPS:435 921 | 21-225_190D6 | VK1|A30/ JK3 | | DIQMTQSPSSLSA SVGDRVTITC | RAS--QGIR-- ----NDLG | WYQLKPGK APKRLIY | A-------- ASSLQS | GVPSRFSGSGSG-- PEFTLTIISSLQPEDFATYYC | LQHYS----------- ------------FPFT | FGPGT KVDIK |
| iPS:435 985 | 21-225_192F6 | VK1|A30/ JK3 | | DIQMTQSPSSLSA SVGDRVTITC | RAS--QGIR-- ----NDLG | WYQLKPGK APKRLIY | A-------- ASSLQS | GVPSRFSGSGSG-- PEFTLTIISSLQPEDFATYYC | LQHYS----------- ------------FPFT | FGPGT KVDIK |
| iPS:436 074 | 21-225_194F10 | VK1|A30/ JK3 | | DIQMTQSPSSLSA SVGDRVTITC | RAS--QGIR-- ----NDLG | WYQLKPGK APKRLIY | A-------- ASSLQS | GVPSRFSGSGSG-- PEFTLTIISSLQPEDFATYYC | LQHYS----------- ------------FPFT | FGPGT KVDIK |
| iPS:436 092 | 21-225_195B9 | VK1|A30/ JK3 | | DIQMTQSPSSLSA SVGDRVTITC | RAS--QGIR-- ----KDLG | WYQQKPGK APKRLIY | A-------- ASDLQS | GVPSRFSGSGSG-- TEFTLTIISSLQPEDFATYCC | LQHYR----------- ------------YPFT | FGPGT KVDFK |
| iPS:436 164 | 21-225_197G1 0 | VK1|A30/ JK3 | | DIQMTQSPSSLSA SVGDRVTITC | RAS--QGIR-- ----NDLG | WYQQKPGK APKRLIY | G-------- ASSLQS | GVPSRFSGSGSG-- TEFTLTIISSLQPEDFATYYC | LQHYR----------- ------------YPFT | FGPGT KVDIK |
| iPS:436 191 | 21-225_198B9 | VK1|A30/ JK3 | | DIQMTQSPSSLSA SVGDRVTITC | RAS--QGIR-- ----KDLG | WYQQKPGK APKRLIY | A-------- ASSLQS | GVPSRFSGSGSG-- TEFTLTIISSLQPEDFATYVC | LQHYR----------- ------------YPFT | FGPGT KVDFK |
| iPS:436 205 | 21-225_199A7 | VK1|A30/ JK3 | | DIQMTQSPSSLSA SVGDRVTITC | RAS--QGIR-- ----KDLG | WYQQKPGK APKRLIY | A-------- ASSLQS | GVPSRFSGSGSG-- TEFTLTIISSLQPEDFATYVC | LQHYR----------- ------------YPFT | FGPGT KVDFK |
| iPS:436 214 | 21-225_200F6 | VK1|A30/ JK3 | | DIQMTQSPSSLSA SVGDRVTITC | RAS--QDIR-- ----NDLG | WYQQKPGK APKRLIY | A-------- ASSLQS | GVPSRFSGSGSG-- TEFTLTIISSLQPEDFATYHC | LQHYR----------- ------------YPFT | FGPGT KVDIK |
| iPS:436 248 | 21-225_202A3 | VK1|A30/ JK3 | | DIQMSQSPSSLSA SVGDRVTITC | RAS--QGIR-- ----NDLG | WYQQKPGK APKRLIC | A-------- ASRLQS | GVPSRFSGSGSG-- TEFTLTIISSLQPEDFAIYYC | LQHHD----------- ------------YPFT | FGPGT KVDIK |
| iPS:436 268 | 21-225_203B9 | VK1|A30/ JK3 | | DIQMTQSPSSLSA SVGDRVTITC | RAS--QGIR-- ----NDLG | WYQQKPGK APKRLIY | R-------- ASSVQN | GVPSRFSGSGSG-- TEFTLTIISSLQPEDFATYHC | LQHNS----------- ------------YPFT | FGPGT KVDIK |
| iPS:436 350 | 21-225_210E4 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPEDFATYYC | LQHNS----------- ------------YPFT | FGPGT KVDIK |
| iPS:436 576 | 21-225_225B6 | VK1|A30/ JK3 | | DIQMTQSPSSLSA SVGDRVIITC | RAS--QGMR-- ----KDLG | WYQQKPGK APKRLIY | A-------- ATSLQS | GVPSRFSGSGSG-- TEFTLTIISSLQPEDFATYYC | LQHNS----------- ------------YPFT | FGPGT KVDIK |
| iPS:436 578 | 21-225_225D6 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPEDFATYCC | LQHNT----------- ------------YPFT | FGPGT KVDIK |
| iPS:436 582 | 21-225_225F8 | VK1|A30/ JK3 | | DIQMTQSPSSLSA SVGDRVTITC | RAS--QGIR-- ----NDLG | WYQQKPGK APKRLIY | A-------- ASSLLG | GVPSRFSGSGSG-- TEFTLTIISSLQPEDFATYYC | LQHNS----------- ------------YPFT | FGPGT KVDIK |
| iPS:436 608 | 21-225_226A9 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPEDFATYYC | LQHNS----------- ------------YPFT | FGPGT KVDIK |
| iPS:436 630 | 21-225_227G3 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTIISSLQPADFATYYC | LHHNS----------- ------------YPFT | FGPGT KVDIK |
| iPS:436 634 | 21-225_227H5 | VK1|A30/ JK3 | | DIQMTQSPSSLSA SVGDRVTITC | RAS--QDIR-- ----NDLG | WYQQKPGK APKRLIY | A-------- ASSLQS | GVPSRFSGSGSG-- TEFTLTIISSLQPEDFATYYC | LQHNS----------- ------------YPFT | FGPGT KVDIK |

| | | | | | | | | | | |
|---|---|---|---|---|---|---|---|---|---|---|
| iPS:436 650 | 21-225_227C1 2 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:392 632 | 21-225_16A11 | VK1|A30/ JK3 | | DIQMTQSPSSLSA SVGDSVTISC | RAS--QDIR-- ----NHLG | WYQRNPGK APKRLIY | A-------- ASSLQS | GVPSRFSGSGSG-- TEFTLTISSLQPEDFATYYC | LQYNS----------- ------------YPFT | FGPGT KVDIK |
| iPS:392 684 | 21-225_17F4 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:392 732 | 21-225_17E5 | VK1|A30/ JK3 | | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNS----------- ------------YPLT | FGPGT KVVIK |
| iPS:392 778 | 21-225_22H3 | VK1|A30/ JK3 | | DIQMTQSPSSLSA SVGDRVTITC | RAS--QDIR-- ----NNLG | WYQQKPGK APKRLIY | P-------- ASSLQT | GVPSRFSGSGSG-- TEFTLTISSLQPEDFATYFC | LQDNS----------- ------------YPFT | FGPGT KVDIK |
| iPS:392 912 | 21-225_25A9 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:392 934 | 21-225_27D5 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:392 940 | 21-225_29D9 | VK1|A30/ JK3 | | DIQMTQSPSSLSA SVGDRVTITC | RAS--QGIR-- ----NDLG | WFQQKPGK APKRLIY | A-------- ASSLQS | GVPSRFSGSGSG-- TEFTLTISSLQPEDFATYYC | LQHNT----------- ------------YPFT | FGPGT KVDFK |
| iPS:392 948 | 21-225_25G5 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTVSSLQPEDFATYYC | LQHNS----------- ------------YPFT | FGPGT KVDIK |
| iPS:392 968 | 21-225_25B6 | VK1|A30/ JK3 | | DIQMTQSPSSLSA SVGDRVTITC | RAS--QGIR-- ----NDLG | WYQQKPGK APKRLIY | R-------- ASSLQS | GVPSRFSGSGSG-- TEFTLTISSLQPEDFATYYC | LQHNS----------- ------------YPFT | FGPGT KVDIK |
| iPS:392 978 | 21-225_28B8 | VK1|A30/ JK3 | | DIQMTQSPSSLSA SVGDRVTITC | RAS--QGIR-- ----NDLG | WYQHKPGK APKRLIY | A-------- ASSLQS | GVPSRFSGSGSG-- TEFTLTISSLQPEDFATYYC | LQHNS----------- ------------YPFT | FGPGT KVDIN |
| iPS:392 998 | 21-225_28A9 | VK1|A30/ JK3 | | DIQMTQSPSSLSA SVGDRVTITC | RAS--QGIR-- ----NDLG | WYQQKPGK APKRLIY | A-------- ASSLQN | GVPSRFSGSGSG-- TEFTLTISSLQPEDFATYYC | LQHNR----------- ------------YPFT | FGPGT KVDIK |
| iPS:393 000 | 21-225_29D7 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:393 006 | 21-225_31G9 | VK1|A30/ JK3 | | DIQMTQSPSSLSA SIGDRVTITC | RAS--QGIR-- ----NDLG | WYQQKPGK APKRLIY | P-------- ASSLQS | GVPSRFSGSGSG-- TEFTLTISSLQPEDFATYYC | LQDNS----------- ------------YPFT | FGPGT KVDIK |
| iPS:393 022 | 21-225_30H11 | VK1|A30/ JK3 | | DIQMTQSPSSLSA SVGDRVTITC | RAS--QGIR-- ----NDLG | WYQQKPGK APKRLVY | P-------- ASSLQS | GVPSRFSGSGSG-- TEFTLTISSLQPEDFATYYC | LQDNS----------- ------------HPFT | FGPGT KVDIT |
| iPS:393 038 | 21-225_29D8 | VK1|A30/ JK3 | | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNS----------- ------------YPFT | FGPGT KVDIK |
| iPS:393 822 | 21-225_15B11 | VK1|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:393 944 | 21-225_14D6 | VK1|A30/ JK3 | | DIQMTQSPSSLSA SVGDSVTITC | RAS--QGIR-- ----NHLG | WYQHKPGK APKRLIY | A-------- ASSLQS | GVPSRFSGSGSG-- TEFTLTISSLQPEDFATYYC | LQYNS----------- ------------YPFT | FGPGT KVDIN |
| iPS:394 033 | 21-225_5F4 | VK1|A30/ JK3 | | DIQMTQSPSSLSA SVGDRVTITC | RAS--QGIR-- ----NHLG | WYQQKPGK APKRLIY | A-------- ASSLQS | GVPSRFSGSGSG-- TEFTLTVSSLQPEDFATYYC | LQYNG----------- ------------YPFT | FGPGT KVDIK |
| iPS:394 069 | 21-225_16H1 | VK1|A30/ JK3 | | DIQMTQSPSSLSA SVGDRVTITC | RAS--QGIR-- ----DILG | WYQQKPGK APKRLIY | A-------- ASSLQN | GVPSRFSGSGSG-- TEFTLTISSLQPEDFATYYC | LQYHS----------- ------------YPFT | FGPGT KVDVK |
| iPS:402 229 | 21-225_16H9 | VK1|A30/ JK3 | | DIQMTQSPSSLSA SVGDRVTITC | RAS--QGIR-- ----NYLG | WFQQKPGK APKRLIY | G-------- ASSLQS | GVPSRISGSGSG-- TEFTLTISSLQPEDFATYYC | LQYHS----------- ------------YLFT | FGPGT KVDIK |
| | | Germline | | K_FR1 | K_CDR1 | K_FR2 | K_CDR2 | K_FR3 | K_CDR3 | K_FR4 |
| | VK1|O12/J K1 | | 32143 | DIQMTQSPSSLSA SVGDRVTITC | RAS--QSIS-- ----SYLN | WYQQKPGK APKLLIY | A-------- ASSLQS | GVPSRFSGSGSG-- TDFTLTISSLQPEDFATYYC | QQSYS----------- ------------TPWT | FGQGT KVEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:468 848 | 21-225_54B1 | VK1|O12/JK1 | | DIQMTQSPSSLSA SIGDRVTITC | RAS--QNIS-- ----SYLN | WYQQKPGK APKFLIY | A-------- ASSLHS | GVPPRFSGSGSG-- TDFTLTISSLQPEDFAIYYC | QQSYR----------- -----------TPLWT | FGQGT KVEIK |
| iPS:434 239 | 21-225_58F1 | VK1|O12/JK1 | | DIQMTQSPSSLSA SVGDRVTITC | RAS--QSIT-- ----NFLN | WYQQKPGK APKLLIF | A-------- ASSLQS | GIPSRFSGSGSG-- TDFTLTISSLQPEDFATYYC | QQSYS----------- ------------IPWT | FGQGT KVEIK |
| iPS:435 513 | 21-225_157F3 | VK1|O12/JK1 | | DIQMTQSPSSLSA SVGDRVTITC | RAS--QNIS-- ----SYLN | WYQLKPGK APKLLIY | T-------- ASSLQS | GVPSRFSGSGSG-- TDFTLTISSLQPEDFATYYC | QQSYN----------- -----------TPTWT | FGQGT KVEIK |
| iPS:435 729 | 21-225_173E7 | VK1|O12/JK1 | | DIQMTQSPSSRSA CIGDRATITY | RAS--QTIS-- ----NYLN | WYQQKPGK APKLLIY | A-------- ASSLQI | GVPSRFSGSGSG-- TDFTLTISSVQPEDFATYFC | QQSYR----------- -----------TPQWT | FGQGT KVEIK |
| iPS:435 753 | 21-225_175G10 | VK1|O12/JK1 | | DIQMTQSPSSLSA SVGDRVIITC | RAS--QTIG-- ----NYLN | WYQQKPGR APKLLIY | A-------- ASSLHS | GVPSGFSGSGSG-- TDFTLTISSLQPEDFATYFC | QQSYR----------- -----------TPQWT | FGQGT KVEIK |
| iPS:435 799 | 21-225_181G3 | VK1|O12/JK1 | | DIQMTQSPSSLSA SVGDRVNITC | RAS--HSIS-- ----NYLN | WYQQKAGK APNLLIY | T-------- TLNLQS | GVPSRFSGSGSG-- TDFTLTISSLQPEDFATYYC | QQSYS----------- -----------SPPWT | FGQGT KVEIK |
| iPS:435 813 | 21-225_183A12 | VK1|O12/JK1 | | DIQMTQSPSSLSA SVGDRVTITC | RAS--RNIS-- ----NYLN | WYQQKPGK APKLLIY | V-------- VSSLQS | GVPSRFSGSGSG-- TDFTLTISSLQPEDFATYYC | QQSYS----------- -----------SPPWT | FGQGT KVDIR |
| iPS:436 003 | 21-225_192G10 | VK1|O12/JK1 | | DIQMTQSPSSLSA SVGDRVTITC | RAS--QSIS-- ----NYLN | WYQQTPGK APKLLIY | A-------- ESSLQS | GVPSRFSGSGSG-- TDFTLTISSLQPEDFATYSC | QQSYS----------- -----------SPPWT | FGQGT KVEIK |
| iPS:436 212 | 21-225_200G1 | VK1|O12/JK1 | | DIQMTQSPSSLSA SVGDRVTITC | RAS--QSIS-- ----SYLN | WFQQKPGK APKLLIY | A-------- ESSLQS | GVPSRFSGSGSG-- TDFTLTISSLQPEDFATYSC | QQSYS----------- -----------SPPWT | FGQGT KVEFK |
| iPS:392 730 | 21-225_17A1 | VK1|O12/JK1 | | DIQMTQSPSSLSA SVGDRVTITC | RAS--QNIN-- ----NYLN | WYQQKPGK GPKVLIF | T-------- TSSLQS | GVPSRFSGSGSG-- TDFTLTISSLQPEDFATYYC | QQSYT----------- -----------TPTWT | FGQGT KVEIK |
| iPS:392 736 | 21-225_17B12 | VK1|O12/JK1 | | DIQMTQSPSSLSA SVGDRVSITC | RAS--QNIN-- ----NYLN | WYQQKPGK GPKVLIL | T-------- ASSLQS | GVPSRFSGSGSG-- TDFTLTISSLQPEDFATYYC | QQTYT----------- -----------TPTWT | FGQGT KVEIK |
| iPS:392 766 | 21-225_23H4 | VK1|O12/JK1 | | DIQMTQSPSSLSA SVGDRVTITC | RAS--QSIS-- ----RYLN | WYQQKPGR APKLLIC | S-------- TSSLQS | GVPSRFSGSGSG-- TDFTLTISSLQPEDFATYYC | QQSYS----------- -----------TPTWT | FGQGT KVEIR |
| iPS:392 770 | 21-225_20C10 | VK1|O12/JK1 | | DIHMTQSPSSLSA SVGDKVSITC | RAS--HHIS-- ----NYLN | WYQQKPGK GPKVLIL | T-------- ASSLQS | GVPSRFSGSGSG-- TDFTLTISSLQPEDFATYYC | QQSYT----------- -----------TPTWT | FGQGT KVEIK |
| iPS:392 808 | 21-225_20F8 | VK1|O12/JK1 | | DIQMTQSPSSLSA SVGDRVTITC | RAS--QSIS-- ----RYLN | WYQQKPGK APELLIY | A-------- ASSLQS | GVPSRFSGSGSG-- TDFILTISSLQPEDFATYYC | QQSYN----------- -----------TPTWT | FGQGT KVEIK |
| iPS:392 954 | 21-225_26A10 | VK1|O12/JK1 | | DIQMTQSPSSLSA SVGDRITITC | RAS--QSIS-- ----SYLN | WYQQKPGK APKVLIY | A-------- ASSLQS | GVPSRFSGSGSG-- TDFTLTISSLQPEDFATYYC | QQSYS----------- -----------TPTWT | FGQGT KVEIK |
| iPS:393 878 | 21-225_7G12 | VK1|O12/JK1 | | DIQMTQSPSSLSA SVGDRVSITC | RAS--QNIN-- ----NYLN | WYQQKPGK GPKVLIL | T-------- ASSLQS | GVPSRFSGSGSG-- TDFTLTINSLQPEDFATYYC | QQSYT----------- -----------TPTWT | FGQGT KVEIK |
| iPS:398 474 | 21-225_17B10 | VK1|O12/JK1 | | DIQMTQSPSSLSA SVGDRVTITC | RSS--QSIN-- ----SYLN | WYQQKPGK APKLLIF | A-------- ASSLHS | GVPSRFSGSGSG-- TDFTLTISSLQPEDFATYYC | QQGYN----------- -----------TPTWT | FGQGT KVEIN |
| | | Germline | | K_FR1 | K_CDR1 | K_FR2 | K_CDR2 | K_FR3 | K_CDR3 | K_FR4 |
| | VK4|B3/JK2 | | 32144 | DIVMTQSPDSLAV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------TPYT | FGQGT KLEIK |
| iPS:468 850 | 21-225_63F4 | VK4|B3/JK2 | | DIVMTQSPDSLAV SLGERATINC | KSS-- QSVLSSSNNNN YLA | WYQQKPGQ PPKLLIY | W-------- ASTRES | GIPDRFSGSGSG-- TGFTLTISSLQAEDVAVYYC | QQYYT----------- -----------TPCS | FGQGT KLEIN |
| iPS:468 852 | 21-225_71F3 | VK4|B3/JK2 | | DIVMTQSPDSLAV SLGARATINC | KSS-- QSVLSNSNNNN YLA | WYQQKPGQ PPKLLIY | W-------- ASTRES | GIPDRFSGSGSG-- TDFTLTINSLQAEDVAVYYC | QQYYT----------- -----------TPCS | FGQGT KLEIK |

| | | | | | | | | | | |
|---|---|---|---|---|---|---|---|---|---|---|
| iPS:468 870 | 21-225_74A8 | VK4|B3/J K2 | | DIVMIQSPDSLAV SLGERATINC | KSS-- QSVLYSSNSHN YLA | WYQQKPGQ PPKLLIY | W-------- ASTRES | GVPDRFSGSGSG-- TDFTLTISSLQAEDVAVYYC | QQYYS----------- -----------TPCS | FGQGT KLEIK |
| iPS:434 211 | 21-225_60F3 | VK4|B3/J K2 | | DIVMIQSPDSLTV SLGERATINC | KSS-- QSVLYSSNNKN YLA | WYQQKPGQ PPKLLIY | W-------- ASTRES | GVPDRFSGSGSG-- TDFTLTISSLQAEDVAIYYC | QQYYS----------- -----------TPCS | FGQGT KLEIN |
| iPS:434 235 | 21-225_61E3 | VK4|B3/J K2 | | DIVMIQSPDSLAV SLGERATINC | KSS-- QSVLHISNNNN YLA | WYQQQPGQ PPKLLIY | W-------- ASIRES | GVPDRFSGSGSG-- TDFTLTISSLQAEDVAVYYC | QQYYS----------- -----------IPCS | FGQGT KLEIK |
| iPS:434 287 | 21-225_57F12 | VK4|B3/J K2 | | DIVMIQSPDSLAV SLGERATINC | KSS-- QSVLFSSNNYN YLA | WYQQKTGQ PPKLIIY | W-------- ASTRES | GVPDRFSGSGSG-- TDFTLTISSLQAEDVAIYYC | QQYYS----------- -----------NPCS | FGQGT KLEIK |
| iPS:434 305 | 21-225_59E1 | VK4|B3/J K2 | | DIVMIQSPDSLAV SLGERATINC | KSS-- QSVLYSSNNNN YLA | WYQQRPGQ PPKLLIY | W-------- SSTRES | GVPDRFSGSGSG-- TDFTLTISSLQAEDVAVYYC | QQYFS----------- -----------IPCS | FGQGT KLEIK |
| iPS:434 443 | 21-225_71G3 | VK4|B3/J K2 | | DIVMIQSPDSLAV SLGERVAINC | KSS-- QSVLHSSNNNN YLD | WYQQKPGQ LPKLLIF | W-------- ASTREF | GVPDRFSGSGFG-- TDFTLTISSLQAEDVADYYC | QQYYI----------- -----------TPCS | FGQGT KLEIK |
| iPS:434 483 | 21-225_74C12 | VK4|B3/J K2 | | DIVMIQSPDSLAV SLGERATINC | KSS-- QSVLYSSNNAN YLA | WYQQKPGQ PPNLLIY | W-------- ASTRES | GVPDRFSGSGSG-- TDFTLTISSLQAEDVAVYYC | QQYYS----------- -----------TPCS | FGQGT KLEIK |
| iPS:434 613 | 21-225_77D12 | VK4|B3/J K2 | | DIVMIQSPDSLAV SLGARATINC | RSS-- QSVLYSSNKYN YLA | WYQQKPGQ PPKLLIY | W-------- ASTRDS | GVPDRFSGSGSG-- TDFTLTISSLQAEDVAVYYC | QQYYT----------- -----------TPCS | FGQGT KLEIK |
| iPS:434 635 | 21-225_78E6 | VK4|B3/J K2 | | DIVMIQSPDSLAV SLGERATINC | KSS-- QSVLYSSNSHN YLA | WYQQKPGQ PPKLLIY | W-------- ASIRES | GVPDRFSGSGSG-- TDFTLSISSLQAEDVAVYYC | QQYYS----------- -----------TPCS | FGQGT KLEIK |
| iPS:434 679 | 21-225_79G7 | VK4|B3/J K2 | | DIVMIQSPDSLAV SLGERATINC | KSS-- QSVLYSSNSHN YLA | WYQQKPGQ PPKLLIF | W-------- ASIRES | GVPDRFSGSGSG-- TDFTLSISSMQAEDVAVYYC | QQYYS----------- -----------TPCS | FGQGT KLEIK |
| iPS:434 909 | 21-225_85C11 | VK4|B3/J K2 | | DIVMIQSPDSLAV SLGERATINC | KSS-- QSVLYSSNSHN FLA | WYQQNPGQ PPKLLIF | W-------- AFIRES | GVPEGFSGSGSG-- ADFTLSISGLQAEDVAVYYC | QQYYS----------- -----------TPCS | FGQGT KLEIK |
| iPS:434 959 | 21-225_87E10 | VK4|B3/J K2 | | DIVMIQSPDSLAV SLGERATIKC | KSS-- QSVLHSSNNMN YLA | WYQQKPGQ PPKLLIY | W-------- ASTRKS | GVPDRFSGTGSG-- TDFTLTISSLQAEDVAVYYC | QQYYS----------- -----------SPCS | FGQGT KLKIK |
| iPS:435 299 | 21-225_146D4 | VK4|B3/J K2 | | DIVMIQSPDSLAV SLGERATINC | KSS-- QSVLYSSNNNN YLA | WYQQKPGQ PPKLVIY | W-------- ASTRES | GVPDRFSGSGSG-- TDFTLTISSLQAEDVAVYYC | QQYYS----------- -----------TPCS | FGQGT KLEIK |
| iPS:435 305 | 21-225_146C9 | VK4|B3/J K2 | | DIVMIQSPDSLAV SLGERATINC | KSS-- QSVLHSSNNYN YLA | WYQQKPGQ PPKVLIY | W-------- ASTRKS | GVPDRFSGSGSG-- TDFTLTISSLQAEDVAVYYC | QQYYS----------- -----------TPCS | FGQGT KLEIK |
| iPS:435 309 | 21-225_146F9 | VK4|B3/J K2 | | DIVMIQSPDSLAV SLGERATINC | KSS-- QNILHSSNNNN YLA | WYQQKPGQ PPYLLIY | W-------- ASTRES | GVPDRFSGSGSG-- TDFTLIITSLQAEDVAVYYC | QQYYT----------- -----------TPCS | FGQGT KLEIT |
| iPS:435 323 | 21-225_147D5 | VK4|B3/J K2 | | DIVMIQSPDSLAV SLGERATINC | KSS-- QSVLYSSNNNN YLA | WYQQKPGQ PPKLLIY | W-------- ASTRES | GVPDRFSGSGSG-- TDFTLSISSLQAEDVAVYYC | HQYYS----------- -----------TPCS | FGQGT KLEIK |

| | | | | | | | | | | |
|---|---|---|---|---|---|---|---|---|---|---|
| iPS:435 399 | 21- 225_150D2 | VK4\|B3/J K2 | | DIVMIQSPDSLAV SLGERATIKC | KSS-- QSVLYRSNSKK YLT | WYQQKPGQ PPKLFIY | W-------- ASTRKS | GVPDRFSGSGSG-- TDFTLTISNLQAEDVAVYFC | QQYFS----------- -----------TPYN | FGQGT KREIK |
| iPS:435 435 | 21- 225_152H3 | VK4\|B3/J K2 | | DIVMIQSPDSLAV SLGEKATINC | KSS-- QSVLHSSNNYN YLT | WYQQKPGQ PPKLLIY | W-------- ASTRKS | GVPDRFSGSGSG-- TDFTLTISSLQAEDVAVYYC | QQYYS----------- -----------TPCS | FGQGT KLEIK |
| iPS:435 451 | 21- 225_152D1 0 | VK4\|B3/J K2 | | GIVMIQSPDSLAV SLGARATIDC | KSS-- QSVLYSSNNKN YLA | WYQQKPGQ PPKLLIY | W-------- ASTRES | GVPDRFSGSGSG-- TDFTLTIYSLQAEDVAVYYC | QQYY----------- -----------RSPS | FGQGT KLEIK |
| iPS:435 459 | 21- 225_152E1 2 | VK4\|B3/J K2 | | AVVMIQSPDSLAV SLGERATINC | TSS-- QSILHSSNNYN YLA | WYQQKPGQ PPKLLIY | W-------- ASTRES | GVPDRFSGSGSG-- TDFTLTISSLQAEDVAVYYC | QQYYS----------- -----------GPCS | FGQGT KLEIK |
| iPS:435 467 | 21- 225_153B9 | VK4\|B3/J K2 | | GIVMIQFPDSLAV SLGERATINC | KSS-- QSVLYSSNNKN YLA | WYQQKPGQ PHKLLIY | W-------- ASTREF | GVPDRFSGSGCG-- TDFTLTIYSVQAEDVAVYYC | QQYN----------- -----------RSLS | FGQGT KLEIK |
| iPS:435 471 | 21- 225_153F11 | VK4\|B3/J K2 | | DIVMIQSPDSLAV SLGERATINC | KSS-- QSVLYSSNNYK YLA | WYQQKPGQ PPNLLIY | W-------- ASTRKS | GVPDRFSGSGSG-- TDFTLTISSLQAEDVAVYYC | QQYYS----------- -----------TPCS | FGQGT KLEIK |
| iPS:435 475 | 21- 225_154H6 | VK4\|B3/J K2 | | DIVMIQSPDSLAV SLGERATINC | KSS-- QSVLHSSNNYN YLA | WYQQKPGQ PPKLLIY | T-------- TSTRKS | THFTLSISSLQAEDVAVYYC | QHYYS----------- -----------TPCS | FGQGT KLEIK |
| iPS:435 491 | 21- 225_155E5 | VK4\|B3/J K2 | | DIVMIQSPDSLAV SLGERATINC | KSS-- QSVLSSSNNNN YLA | WYQQKPGQ PPKLLIY | W-------- ASTRKS | GVPDRFSGSGSG-- TDFTLTISSLQAEDVAVYYC | QQYYS----------- -----------TPCS | FGQGT KLEIK |
| iPS:435 495 | 21- 225_155B6 | VK4\|B3/J K2 | | DIVMIQSPDSLAV SLGERATINC | KSS-- QSVLHSSNNYN YLA | WYQQKPGQ PPKLLIY | T-------- TSTRES | GVPDRFSGSGSG-- TDFTLTISSLQAEDVAVYYC | QQYYS----------- -----------TPCS | FGQGT KLEIK |
| iPS:435 501 | 21- 225_156H1 | VK4\|B3/J K2 | | DIVMIQSPDSLAV SLGERATINC | KSS-- QSVLYSSNNNN YLA | WYQQKPGQ PPKLLIY | W-------- ASTRES | GVPARFSGSGSG-- TDFTLTISSLQAEDVAVYYC | HQYYS----------- -----------TPCS | FGQGT KLEIK |
| iPS:435 589 | 21- 225_160A4 | VK4\|B3/J K2 | | DIVMIQSPDSLAV SLGERATINC | KSS-- QSVLHSSNNNN YLA | WYQQKPGQ PPKLLIY | W-------- ASTRES | GVPDRFSGSGSG-- TDFTLTISSLQAEDVAVYYC | QQYYN----------- -----------SPCS | FGQGT KLEIK |
| iPS:435 727 | 21- 225_172E1 1 | VK4\|B3/J K2 | | DIVMIQSPDSLAV SLGERATINC | KSS-- QSVLHSSNNNN YLA | WYQQKPGQ PPKLLIY | W-------- ASTRES | GVPDRFSGSGSG-- TDFILTISGLQAEDVAVYYC | QQYFT----------- -----------TPCS | FGQGT KLEIK |
| iPS:436 560 | 21- 225_224F11 | VK4\|B3/J K2 | | NIVMIQSPDSLAV SLDERATINC | KSS-- QSVLSSSNNHN YLA | WYQQKPGQ PPKMLIY | W-------- ASTRES | GVPDRFSGSGSG-- TDFTLTISSLQAEDVAVYYC | QQYYT----------- -----------TPCS | FGQGT KLEIK |
| iPS:436 584 | 21- 225_225B9 | VK4\|B3/J K2 | | DIVMIQSPDSLAV SLGERATINC | KSS-- QSVLYSSNNNN YLA | WYQQKPGQ PPKLLIF | W-------- ASTRES | GVPDRFSGSGSG-- TDFTLTISSLQAEDVAVYYC | QHSKS----------- -----------IPGK | FGQGI KLEIQ |
| iPS:436 588 | 21- 225_225F12 | VK4\|B3/J K2 | | DIVMIQSPDSLAV SLGERATINC | KSS-- QSVLYSSNNQN YLA | WYQQKPGQ PPRLLIY | T-------- TSTRES | GVPDRFSGSGSG-- TDFTLTISNLQAEDVAVYYC | QQYYI----------- -----------TPCS | FGQGT KLEIK |
| iPS:436 590 | 21- 225_225H1 2 | VK4\|B3/J K2 | | DIVMIQSPDSLAV SLGERATINC | KSS-- QSVLYNSNNNN YLA | WYQQKPGQ PPNLLIY | W-------- ASTRES | GVPDRFSGSGSG-- TDFTLTISSLQTEDVAVYYC | QQYYI----------- -----------TPCS | FGQGT KLEIK |

| | | Germline | | K_FR1 | K_CDR1 | K_FR2 | K_CDR2 | K_FR3 | K_CDR3 | K_FR4 |
|---|---|---|---|---|---|---|---|---|---|---|
| iPS:436 598 | 21-225_226D6 | VK4\|B3/JK2 | | DIVMTQSPDSLAV SLGERATINC | RSS-- QSILYISNNKN YLA | WYQQKPGQ PPKMLIY | W-------- ASTRES | GVPDRFSGSGSG-- TDFTLTISSLQAEDVAVYYC | QQYYS----------- ------------SPCS | FGQGT KLEIK |
| iPS:436 636 | 21-225_227E6 | VK4\|B3/JK2 | | DIVMTQSPDSLTV SLGERATINC | RSS-- QSVLYSSNDKN YLA | WYQQKPGQ PPKMLIY | W-------- ASTRES | GVPDRFSGSGSG-- TDFTLTISSLQAEDVAVYYC | QQYYI----------- ------------TPCS | FGQGT KLEIK |
| iPS:436 644 | 21-225_227G9 | VK4\|B3/JK2 | | DIVMTQSPDSLAV SLGERATINC | KSS-- QSVLHSSNNNN YLA | WYQQKPGQ PPKLLIY | W-------- GSTRES | GVPDRFSGSGSG-- TDFTLTISSLQAEDVAVYYC | QQYYS----------- ------------APYS | FGQGT KLEIK |
| iPS:436 646 | 21-225_227D11 | VK4\|B3/JK2 | | DIVMTQSPDSLAV SLDERATINC | KSS-- QSVLHSSNNNN YLA | WYQQKPGQ PPKLLIY | W-------- ASTRES | GIPDRFSGSGSG-- TDFTLTISSLQAEDVAVYYC | QQYYN----------- ------------TPCS | FGQGT KLEIK |
| iPS:437 363 | 21-225_74C10 | VK4\|B3/JK2 | | DIVMTQSPDSLAV SLGERATINC | KSS-- QSVLYSSNNAN YLA | WYQQKPGQ PPNLLIY | W-------- ASTRES | GVPDRFSGSGSG-- TDFTLTISSLQAEDVAVYYC | QQYYS----------- ------------TPCS | FGQGT KLEIK |
| iPS:451 131 | 21-225_160A7 | VK4\|B3/JK2 | | DIVLTQSPDSPAV SLGERATINC | KSS-- QSVLSNSHNNN YLA | WYQQRPGH PHKLLIF | W-------- ASTRES | GVPDRFSGSGYG-- TDFTLTISSLQAEDVAVYYC | QQYYS----------- ------------TPCS | FGQGT KLEIK |
| iPS:393 088 | 21-225_33D1 | VK4\|B3/JK2 | | DIVMTQSPDSLSV SLGERATINC | KSI-- QSVLYRSNNKN YLT | WYQQKPGQ PRKLFIY | W-------- ASTRES | GVLDRFSGSGCG-- TDFTLTIISLQAEDVALYYC | QQYYS----------- ------------SPCS | FGQGT KLEIK |
| iPS:394 085 | 21-225_8B11 | VK4\|B3/JK2 | | DIVMTQSPDSLAV SLGERATINC | KSS-- QNVLYNSNNNN YLA | WYQQKPGQ PPKLLIY | W-------- ASTRKS | GVPDRFSGSGSG-- TDFTLTISSLQAEDVAVYYC | QQYYT----------- ------------TPCS | FGQGT KLEIK |
| iPS:398 496 | 21-225_22D2 | VK4\|B3/JK2 | | DIVMTQSPDSLAV SLGERATITC | KSS-- QSVLHSSNNNN YLA | WYQQKPGQ PPKLLIY | W-------- ASTRKS | GVPDRFSGSGSG-- TDFTLTISSLQAEDVAVYFC | QQYYS----------- ------------TPCS | FGQGT KLEIK |
| iPS:398 512 | 21-225_25E12 | VK4\|B3/JK2 | | DIVLTQSPDFLAM SLGERATINC | KSS-- QSVLYHSNNYN YLA | WYQQKPKQ PPKLLIY | W-------- ASTRES | GVPDRFSGSGSG-- TYFTLTISSLQAEDVAVYYC | QQYYS----------- ------------TPCS | FGQGT NLEIK |
| iPS:398 522 | 21-225_32A1 | VK4\|B3/JK2 | | DIVMTQSPDSLAV SLGERATINC | KSS-- QSVLYSSNNYN YLA | WYQLKPGQ PPKLLIY | W-------- ASTRKS | GVPDRFSGSGSG-- TDFTLTISSLQTEDVALYYC | QQYYT----------- ------------SPCS | FGQGT KLEIK |
| iPS:398 524 | 21-225_32A5 | VK4\|B3/JK2 | | DIVMTQSPDSLAV SLGERATINC | KSS-- QSVLHSSNNKN YLA | WYQQKPGQ PPKLLIY | W-------- ASTRES | GVPDRFSGSGSG-- TDFTLAISSLQAEDVALYHC | QQYYS----------- ------------SPCS | FGQGT GLEIK |
| iPS:398 538 | 21-225_34H7 | VK4\|B3/JK2 | | DIVMTQSPDSLAV SLGERATINC | KSS-- QSVLYSSNNYN YLA | WYQLKPGQ PPKLLIY | W-------- ASTRKS | GVPDRFSGSGSG-- TDFTLTISSLQTEDVALYYC | QQYYT----------- ------------SPCS | FGQGT KLEIK |
| | VK2\|A17/JK4 | | 32145 | DVVMTQSPLSLPV TLGQPASISC | RSS-- QSLVYSD- GNTYLN | WFQQRPGQ SPRRLIY | K-------- VSNRDS | GVPDRFSGSGSG-- TDFTLKISRVEAEDVGVYYC | MQGTH----------- ------------WPLT | FGGGT KVEIK |
| iPS:468 854 | 21-225_72C4 | VK2\|A17/JK4 | | DVVMTQSPLSLPV TLGQPASISC | RSG-- QSLVYSD- GNTYLN | WFQQRPGQ SPRRLIY | E-------- VSKWDS | GVPDRFSGSGSG-- TNFTLKISRVEAEDVGVFYC | MQGTH----------- ------------WPLT | FGGGT KVEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:437 250 | 21-225_148C6 | VK2\|A17/JK4 | | DVVMTQSPLSLPV TLGQPASISC | RSS--QSLVYSD-GNTYLN | WFQQRPGQ SPRRLIY | K--------VSNWDS | GVPDRFSGSGSG--TDFTLKISRVEAEDVGVYYC | MQGTH----------------------WSLT | FGGGT KVEIK |
| iPS:437 252 | 21-225_148H11 | VK2\|A17/JK4 | | DVVMTQSPLSLPV TLGQPASISC | RSS--QSLVYSD-GNTYLN | WFQQRPGQ SPRRLIY | K--------VSNWDS | GVPDRFSGSGSG--TDFTLKISRVEAEDVGVYYC | MQGTH----------------------WLLT | FGGGT KVEIK |
| iPS:437 254 | 21-225_149F2 | VK2\|A17/JK4 | | DVVMTQSPLSLPV TLGQPASISC | RSS--QSLVYSD-GNTSLN | WFQQRPGQ SPRRLIY | K--------VSNWDS | GVPVRFSGSGSG--TDFTLKISRVEAEDVGIYYC | MQGTH----------------------WPPT | FGGGT KVEIK |
| iPS:437 256 | 21-225_150F11 | VK2\|A17/JK4 | | DVVMSQYPLSLPV TFGQPASISC | RSS--QSLVYSD-GNTSLN | WFQQRPGQ YPRRLIY | K--------VSNWDY | GVPVRFSGSGSG--TDFTLKISRVEAEDVGIYYC | MQGTH----------------------WPPT | FGGGT KVEIK |
| iPS:437 268 | 21-225_177D2 | VK2\|A17/JK4 | | DVVMTQSPLSLPV TLGQPASISC | RSS--QSLVYSD-GNTYLN | WFQQRPGQ SPRRLIY | K--------VSNWDS | GVPDRFSGSGSG--TDFTLKISRVEAEDVGVYYC | MQGTH----------------------WPLT | FGGGT KVEIK |
| iPS:443 005 | 21-225_43F11_LC1 | VK2\|A17/JK4 | | DVVMTQSPLSLPV TLGQPASISC | RSS--QSLVYSD-GNTYLN | WFQQRPGQ SPRRLIY | K--------VSNWDS | GVPDRFSGSGSG--TDFTLKISRVEAEDVGVYYC | MQGTH----------------------WPLT | FGGGT KVEIK |
| iPS:398 530 | 21-225_32G4 | VK2\|A17/JK4 | | DVVMTQSPLSLSV TLGQPASISC | RSS--QSLVYSD-GNTYLN | WFQQRPGQ SPRRLIY | K--------VSNWDS | GVPDRFSGSGSG--TDFTLKISRVEAEDVGVYYC | MQGI-----------------------HWLT | FGGGT KVEIK |
| | Germline | | | K_FR1 | K_CDR1 | K_FR2 | K_CDR2 | K_FR3 | K_CDR3 | K_FR4 |
| | VK2\|A17/JK3 | | 32146 | DVVMTQSPLSLPV TLGQPASISC | RSS--QSLVYSD-GNTYLN | WFQQRPGQ SPRRLIY | K--------VSNRDS | GVPDRFSGSGSG--TDFTLKISRVEAEDVGVYYC | MQGTH----------------------WPFT | FGPGT KVDIK |
| iPS:468 856 | 21-225_77C9 | VK2\|A17/JK3 | | DVVMTQSPLSLPV TLGQPASISC | RSS--QSLVYSV-GNTSLS | WFQQRPGQ SPRRLIY | K--------VSNWDS | GVPDRFSGSGSG--TDFTLKISRVEAEDVGVYYR | MQGTH----------------------WPFT | FGPGT KVDIK |
| iPS:392 936 | 21-225_28B6 | VK2\|A17/JK3 | | DVVMTQSPLSLPV TLGQPASISC | RSS--QSLVYSD-GDTYLN | WFQQRPGQ SPRRQIY | K--------VSNWDS | GVPDRFSGSGSG--TDFTLNISRVEAEDVGIYFC | MHCT-----------------------HWLL | FGPGT KVDIK |
| | Germline | | | K_FR1 | K_CDR1 | K_FR2 | K_CDR2 | K_FR3 | K_CDR3 | K_FR4 |
| | VK2\|A17/JK5 | | 32147 | DVVMTQSPLSLPV TLGQPASISC | RSS--QSLVYSD-GNTYLN | WFQQRPGQ SPRRLIY | K--------VSNRDS | GVPDRFSGSGSG--TDFTLKISRVEAEDVGVYYC | MQGTH----------------------WPIT | FGQGT RLEIK |
| iPS:472 741 | 21-225_30D9_LC1 | VK2\|A17/JK5 | | DVVMTQSPLSLPV TLGQPASISC | RSS--QSLVSSD-GNTFLN | WFQQRPGQ SPRRLIY | K--------VSNWDS | GVPDRFSGSGSG--TDFTLKISRLEAEDVGVYYC | LQGTH----------------------WPLT | FGQGT RLEIK |
| iPS:437 294 | 21-225_216D5 | VK2\|A17/JK5 | | DVVMTQSPLSLPV TLGQPASISC | RSS--QSLVYSD-GNTYLN | WFQQRPGQ SPRRLIY | K--------VSNWDS | GVPDRISGSGSG--TDFTLKISRVEAEDVGIYYC | MQGA-----------------------HWFT | FGQGT RLEIK |
| iPS:472 732 | 21-225_2B10_LC1 | VK2\|A17/JK5 | | DVVMTQSPLSLPV TLGQPASISC | RSS--QSLVYSD-GNTFLN | WFQQRPGQ SPRRLIY | K--------VSNWDS | GVPDRFSGSGSG--TGFTLKISRVEAEDVGVYYC | IQGTH----------------------WPFP | FGQGT RLEIK |
| iPS:398 508 | 21-225_24B1 | VK2\|A17/JK5 | | DVVMTQSPLSLPV TLGQPASISC | RSS--QSLVYSD-GNTYLN | WFQQRPGQ SPRRLIY | K--------VSNWDS | GVPDRFSGSGSG--TDFTLKISWVEAEDVGVCYC | MQGAH---------------------WPPIT | FGQGT RLEIK |

| | | Germline | | K_FR1 | K_CDR1 | K_FR2 | K_CDR2 | K_FR3 | K_CDR3 | K_FR4 |
|---|---|---|---|---|---|---|---|---|---|---|
| iPS:423 019 | 21-225_31D12 _LC1 | VK2\|A17/ JK5 | | DVVMTQSPLSLPV TLGQPASISC | RSS-- QSLIYSD- GNTFLN | WFQQRPGQ SPRRLIY | K-------- VSNWDS | GVPDRFSGSGSG-- TDFTLKISRLEAEDVGIYYC | MQGTH----------- -----------WPLT | FGQGT RLEIK |
| | VK1\|L5/JK1 | | 32148 | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQANS----------- ------------FPWT | FGQGT KVEIK |
| iPS:433 897 | 21-225_43C2 | VK1\|L5/J K1 | | DIQMTQSPSSVSA SVGDRVTITC | RAS--QGIS-- ----DWLA | WYQQKPGK APRLLIY | A-------- ASSLQS | GVPSRFSGSGSG-- TDFTLTISNLQPEDFATYYC | QQTNS----------- ------------FPWT | FGQGT KVEIK |
| iPS:433 903 | 21-225_43H4 | VK1\|L5/J K1 | | DIQMTQSPSSVSA SVGDRVTITC | RAS--QGII-- ----NWLA | WYQQKPGK APKLLIY | A-------- ASSLQS | GVPSRFSGSGSG-- TDFTLTISNLQPEDFATYYC | QQTNS----------- ------------FPWT | FGQGT KVEIK |
| iPS:433 911 | 21-225_43E8 | VK1\|L5/J K1 | | DIQMNQSPSSVSA SVGDRVTITC | RAS--QGIS-- ----NWLA | WYQQKPGR APKLLIY | A-------- ASSLQS | GVPSRFSGSGSG-- TDFTLTISNLQPEDFATYYC | QQTNS----------- ------------FPWT | FGQGT KVEIK |
| iPS:433 941 | 21-225_44D10 | VK1\|L5/J K1 | | DIQMTQSPSSVSA SVGDRVTITC | RAS--QGIS-- ----DWLA | WYQQKPGK APRLLIY | A-------- ASSLQS | GVPSKFSGSGSG-- TDFTLTISNLQPEDFATYYC | QQTNS----------- ------------FPWT | FGQGT KVEIK |
| iPS:433 945 | 21-225_44C12 | VK1\|L5/J K1 | | DIQMTQSPSSVSA SVGDRVTITC | RAS--QGIS-- ----NWLA | WYQQKPGK APKLLIS | A-------- ASSLQS | GVPSRFSGSGSG-- TDFTLSISSLQPEDFATYYC | QQSNS----------- ------------FPWT | FGQGT KVEIK |
| iPS:433 957 | 21-225_45F8 | VK1\|L5/J K1 | | DIQMTQSPSSVSA SVGDRVTITC | RAS--QGIS-- ----DWLA | WYQQKPGK APRLLIY | G-------- ASSLQS | GVPSRFSGSGSG-- TDFTLTISNLQPEDFATYYC | QQTNS----------- ------------FPWT | FGQGT KVEIK |
| iPS:433 973 | 21-225_46A6 | VK1\|L5/J K1 | | DIQMNQSPSSVSA SVGDRVNITC | RAS--QGIS-- ----NWLA | WYQQKPGK VPKLLIY | A-------- ASSLQS | GVPSRFSGSGSG-- TDFTLTISNLQPEDFATYYC | QQTNS----------- ------------FPWT | FGQGT KVEIK |
| iPS:433 993 | 21-225_47G7 | VK1\|L5/J K1 | | DIQMTQSPSSVSA SVGDRVTITC | RAS--QGIS-- ----NWLA | WYQQKPGK APKLLIF | A-------- ASNLQS | GVPSRFSGSGSG-- TDFTLTISSLQPADFATYCC | QQVNS----------- ------------FPWT | FGQGT KVEIK |
| iPS:434 007 | 21-225_48D7 | VK1\|L5/J K1 | | DIQMTQSPSSVSA SVGDRVTITC | RAS--QNIT-- ----SWLA | WYQQKPGK APKLLIY | S-------- ASSLQN | GVPSRFSGSGSG-- TDFTLTISSLQPEDFVTYCC | QQANS----------- ------------FPWT | FGQGT KVEIK |
| iPS:434 063 | 21-225_51G7 | VK1\|L5/J K1 | | DIQMTQSPSSVSA SVGDRVTITC | RAS--QDIS-- ----SWLA | WYQQKPGK APKVLIY | A-------- PSNLQS | AVPSRFSGSGSG-- TDFTLTISSLQPEDFATYYC | QQAHS----------- ------------FPWT | FGQGT KVEIK |
| iPS:434 083 | 21-225_52H2 | VK1\|L5/J K1 | | DIQMTQSPSSVSA SVGDRVTITC | RAS--QNIT-- ----NWLA | WFQQKPGR APKLLIY | T-------- TSSLQS | GVPSRFSGSGSG-- TDFTLTISSLQPEDFATYCC | QQTNS----------- ------------FPWT | FGHGT KVEVK |
| iPS:434 133 | 21-225_54G3 | VK1\|L5/J K1 | | DIQMTQSPSSVSA SVGDRVTITC | RAS--QGIS-- ----SWLA | WYQQKPGK APKLLIY | D-------- ASSLQS | GVPSRFSGSGSE-- TDFTLTISSLQPEDFATYYC | QQANS----------- ------------FPWT | FGQGT KVEIK |
| iPS:434 221 | 21-225_60A11 | VK1\|L5/J K1 | | DIQMTQSPSSVSA SVGDRVTITC | RAS--QVIS-- ----NWLA | WYQLKPGK APKLLIY | T-------- ASSLQS | GVPSRFSGNESG-- TDFTLTISSLQPEDFATYYC | QQANS----------- ------------FPWT | FGQGT KVEIK |
| iPS:434 283 | 21-225_57F8 | VK1\|L5/J K1 | | DIQMTQSPSSVSA SVGDRVTITC | RAS--QGIS-- ----NWLA | WYQQKPGK APKLLIY | T-------- ASSLQS | GVPSRFSGNESG-- TDFTLTISSLQPEDFATYYC | QQANS----------- ------------FPWT | FGQGT KVEIK |
| iPS:435 711 | 21-225_171G4 | VK1\|L5/J K1 | | DIQMTQSPSSVSA SVGDRVTITC | RAS--QGVN-- ----DWLA | WYQQKPGK APKLLIY | D-------- ASSLQS | GVPSRFSGSGSG-- TDFTLTISSLQPEDFATYYC | QQANS----------- ------------FPWT | FGQGT KVEIK |
| iPS:435 715 | 21-225_171A8 | VK1\|L5/J K1 | | AIQMTQSPSSVSA SVGDRVTITC | RAS--QGIS-- ----NWLA | WYQQKPGK APNLMIH | A-------- AFSLQG | GVPSRFSGSGSG-- TDFTLTISSLQPEDFATYYC | QQTNS----------- ------------FPWT | FGQGT KVEIK |
| iPS:435 717 | 21-225_171A9 | VK1\|L5/J K1 | | DIQMTQSPSSVSA SVGDRVTITC | RAS--QDIT-- ----TWLA | WYQQKPGK APKLLIY | D-------- ASSLQS | AVPSRFSGSGSG-- TDFTLTVSSLQPEDFATYYC | LQTNS----------- ------------FPWT | FGQGT KVEIK |
| iPS:435 739 | 21-225_174G7 | VK1\|L5/J K1 | | AIQMTQSPSSVSA SVGDRVTITC | RAS--QGIS-- ----NWLA | WYQQKPGK APNLLIH | A-------- AFSLQG | GVPSRFSGSGSG-- TDFTLTISSLQPEDFATYYC | QQTNS----------- ------------FPWT | FGQGT KVEIK |
| iPS:435 749 | 21-225_175C1 0 | VK1\|L5/J K1 | | DIQMTQSPSSVSA SVGDRVTITC | RAS--QGIT-- ----DWLA | WYQQKPGK APKLLIY | A-------- ASSLQS | GVPSRFSGSGSG-- TDFTLTFSSLQPDDFATYYC | QQTNS----------- ------------FPWT | FGQGT KVEIK |
| iPS:435 775 | 21-225_178A5 | VK1\|L5/J K1 | | DIQMTQSPSSVSA SVGDRVTITC | RAS--QGIS-- ----NWLA | WYQQKPGK APKLLIY | A-------- ASSLQS | GVPSRFNGSGSG-- TDFTLTISSLQPEDFATYCC | QQANS----------- ------------LPWT | FGQGT KVEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:435 777 | 21-225_178F7 | VK1\|L5/J K1 | | DIQMTQSPSSVSA SVGDRVTITC | RAS--QDIT-- ----DWLA | WYQQKPGK APKLLIS | A-------- ASSLQS | GVPSRFSGSGSG-- TDFTLAISSLQPEDFATYYC | QQANS----------- ------------LPWT | FGQGT KVEIK |
| iPS:435 783 | 21-225_179G1 | VK1\|L5/J K1 | | DIQMTQSPSSVSA SVGDRVTITC | RAS--QDIS-- ----DWLA | WYQQKSGK APKLLIS | A-------- ASSLQS | GVPSRFGGSGSG-- TDFTLTISSLQPEDFATYYC | QQANS----------- ------------LPWT | FGQGT KVEIK |
| iPS:435 875 | 21-225_190B9 | VK1\|L5/J K1 | | DIQMTQSPSSVSA SVGDRVTITC | RAS--QGIN-- ----NWLA | WYQQKPGK APKLLIY | G-------- VSSLQS | GVPSRFSGSGSG-- TDFTLTISSLQPEDFATYSC | QQANS----------- ------------FPWT | FGQGT KVEIR |
| iPS:435 895 | 21-225_188E8 | VK1\|L5/J K1 | | DIQMTQSPSSVSA SVGDRVTITC | RAN--QDIS-- ----SWLA | WYQQKPGK APKLLIY | A-------- ASNLQS | GVPSGFSGSGSG-- TDFTLTISSLQPEDFATYYC | QQANS----------- ------------FPWT | FGQGT KVEIK |
| iPS:435 909 | 21-225_190H3 | VK1\|L5/J K1 | | DIQMTQSPSSVSA SVGDRVTITC | RAS--QGLN-- ----NWLA | WYQLKPGK APKLLIY | A-------- VSSLQS | GVPSRFSGSGSG-- SEFTLTISSLQPEDFATYHC | QQANS----------- ------------LPWT | FGQGT KVEIK |
| iPS:436 013 | 21-225_193F2 | VK1\|L5/J K1 | | DIQMTQSPSSVSA SVGDRVTITC | RAS--QGIN-- ----NWLA | WYQQKPGK APKLLIY | G-------- VSSLQS | GVPSRFSGSGSG-- TDFTLTISSLQPEDFATYSC | QQANS----------- ------------FPWT | FGQGT KVEIR |
| iPS:436 068 | 21-225_194F7 | VK1\|L5/J K1 | | DIQMTQSPSSVSA SVGDRVTITC | RAS--QGIS-- ----RWLA | WFQQKPGK APKILIY | A-------- ASSLQS | GVPSRFSGSGSG-- TDFTLTISSLQPEDFATYYC | QQANS----------- ------------FPWT | FGQGT KVEIK |
| iPS:436 100 | 21-225_195G1 2 | VK1\|L5/J K1 | | DIQMTQSPSSVSA SVGDRVTITC | RAS--QGIN-- ----NWLA | WYQQKPGK APKLLIY | G-------- VSSLQS | GVPSRFSGSGSG-- TDFTLTISSLQPEDFATYSC | QQANS----------- ------------FPWT | FGRGT KVENQ |
| iPS:436 116 | 21-225_196B9 | VK1\|L5/J K1 | | DIQMTQSPSSVSA SVGDRVTITC | RAS--QGIS-- ----NCLA | WYQQKPGK APKFLIC | A-------- ASSLQS | AVPSRFSGSGSG-- TDFTLTISSLQPEDFASYYC | QQGDS----------- ------------FPPT | FGQGT KVEFR |
| iPS:436 160 | 21-225_197C9 | VK1\|L5/J K1 | | DIQMTQSPSSVSA SVGDRVTITC | RAS--QGIS-- ----NWLA | WFQQKPGK APQLLIY | A-------- ASSLQS | GVPSRFSGSGSG-- TDFSLTISSLQPEDFATYYC | QQANS----------- ------------FPWT | FGQGT KVEIK |
| iPS:436 546 | 21-225_224D6 | VK1\|L5/J K1 | | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQANS----------- ------------FPWT | FGPGT KVEIK |
| iPS:436 632 | 21-225_227E4 | VK1\|L5/J K1 | | DILMTQSPSSVSA SVGDRVTITC | RAS--QGII-- ----NWLA | WYQQKPGK APKLLIY | A-------- ASSLQS | GVPSRFSGSGSG-- TDFTLTISSLQPEDFAIYYC | QQANS----------- ------------FPWT | FGQGT KVEIK |
| iPS:392 650 | 21-225_17A4 | VK1\|L5/J K1 | | DIQMTQSPSSVSA SVGDRVTITC | RAS--QGIG-- ----NWLA | WYQQKPGK APKLLIF | A-------- ASSLQS | GVPSRFSGSGSG-- TDFTLTISSLQPEDFVTYYC | QQANS----------- ------------FPRT | FGQGT KVEIK |
| iPS:392 728 | 21-225_20F7 | VK1\|L5/J K1 | | DIQMTQSPSSVSA SVGDGVTITC | RAS--QGIS-- ----SWLA | WYQQKPGK APKLLIY | A-------- ASSLQS | GVPSRFSGSGSG-- TDFTLTISSLQPEDFATYYC | QQANS----------- ------------FPRT | FGQGT KVEIK |
| iPS:392 916 | 21-225_27C5 | VK1\|L5/J K1 | | DIQMTQSPSSVSA SVGDRVTITC | RAS--QGIS-- ----SWLA | WYQQKPGK APKFLIY | G-------- ASSLQS | GVPSRFSASGSG-- TEFTLTISSLQPEDFATYYC | QQYDS----------- ------------FPRT | FGQGT KVEIK |
| iPS:392 956 | 21-225_27A11 | VK1\|L5/J K1 | | DIQMTQSPSSVSA SVGDRVTITC | RAS--QGIS-- ----SWLA | WYQQKPGK APKLLIY | G-------- ASSLQS | GVPSRFSASGSG-- TDFTLTISSLQPEDFATYCC | QQSDS----------- -----------FPRT | FGQGT KVEIK |
| iPS:393 014 | 21-225_26D12 | VK1\|L5/J K1 | | DIQMTQSPSSVSA SVGDRVTITC | RAS--QGIS-- ----SWLA | WYQQKPGK APKLLIY | G-------- ASSLQS | GVPSRFSASGSG-- TDFTLTISSLQPEDFATYCC | QQSDS----------- -----------FPRT | FGQGT KVEIK |
| iPS:393 028 | 21-225_25D7 | VK1\|L5/J K1 | | DIQMTQSPSSVSA SVGDRVTFTC | RAS--QDIF-- ----DWLA | WYQQKPGT APKLLIY | A-------- ASSLQS | GVPSRFSGSGSG-- TNFTLTVSGLQPEDFATYYC | QQAYS----------- ------------FPWT | FGQGT KVEIK |
| iPS:393 152 | 21-225_25B3 | VK1\|L5/J K1 | | DIQMTQSPSSVSA SVGDRVTITC | RAS--QGIS-- ----SWLA | WYQQKPGK APKLLIY | G-------- ASSLQS | GVPSRFSGSGSG-- TDFTLTISSLQPEDFATYCC | QQSDS----------- -----------FPRT | FGQGT KVEIK |
| iPS:393 810 | 21-225_5A4 | VK1\|L5/J K1 | | DIQMTQSPSSVSA SVGDRVTITC | RAS--QGIS-- ----TWLA | WYQQKPGK APKLLIY | D-------- ASSLQS | GVPSRFSGSGSG-- TDFTLTISSLQPEDFATYYC | QQANS----------- ------------FPWT | FGQGT KVEIK |
| | | Germline | | K_FR1 | K_CDR1 | K_FR2 | K_CDR2 | K_FR3 | K_CDR3 | K_FR4 |
| | VK2\|A19/JK 3 | | 32149 | DIVMTQSPLSLPV TPGEPASISC | RSS-- QSLLHSN- GYNYLD | WYLQKPGQ SPQLLIY | L-------- GSNRAS | GVPDRFSGSGSG-- TDFTLKISRVEAEDVGVYYC | MQALQ----------- -----------TPFT | FGPGT KVDIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:433 943 | 21-225_44E10 | VK2|A19/ JK3 | | DIVMIQSPLSLPV TPGEPASISC | RSS-- QSLLHSN- GYSYLE | WYLQKPGQ SPQLLIY | L-------- GSNRAS | GVPDRFSGSGSG-- TDFTLKISRVEAEDVGVYYC | MQTLQ----------- -------------TPFT | FGPGT KVDIK |
| iPS:433 989 | 21-225_47C7 | VK2|A19/ JK3 | | DIVMIQSPLSPPV TPGEPASISC | RSS-- QSLLHSN- GYNYLE | WYLQKSGQ SPQFLIY | L-------- GFNRAS | GVPDRFTGSGSG-- TDFTLKISRVEAEDVGVYYC | MQVLQ----------- -------------TPFT | FGPGT KVDIK |
| | Germline | | | K_FR1 | K_CDR1 | K_FR2 | K_CDR2 | K_FR3 | K_CDR3 | K_FR4 |
| | VK1|O12/J K3 | | 32150 | DIQMIQSPSSLSA SVGDRVTITC | RAS--QSIS-- ----SYLN | WYQQKPGK APKLLIY | A-------- ASSLQS | GVPSRFSGSGSG-- TDFTLTISSLQPEDFATYYC | QQSYS----------- -------------IPFT | FGPGT KVDIK |
| iPS:433 999 | 21-225_48D1 | VK1|O12/ JK3 | | DIQMIQSPSSLSA SVGDRVNITC | RAS--QSIS-- ----SYLI | WYQQKPGK APKLLIY | A-------- ASSLQS | GVPSRFSASGSG-- TDFTLTISSLQPEDFASYYC | QQSNS----------- -------------IPFT | FGPGT KVDIK |
| iPS:434 003 | 21-225_48C3 | VK1|O12/ JK3 | | DIQMIQSPSSLSA SVGDRVNITC | RAS--QSII-- ----SYLI | WYQQKPGK APRLLIY | A-------- ASSLQS | GVPSRFSASGSG-- TDFTLTISSLQPEDFASYYC | QQTNS----------- -------------IPFT | FGPGT KVDIK |
| iPS:434 037 | 21-225_49G12 | VK1|O12/ JK3 | | DIQMIQSPSSLSA SVGDRVTITC | RSS--QSIS-- ----TYLM | WYQQKPGK APKLLIY | A-------- ASSLQI | GVPSEFSASGSG-- TDFTLTISSLQPEDFATYYC | QQSYS----------- -------------IPFT | FGPGT KVDIK |
| iPS:434 041 | 21-225_50H8 | VK1|O12/ JK3 | | DIQMIQSPSSLSA SVGDRVNITC | RAS--QSIS-- ----SYLI | WYQQKPGK APKLLIY | A-------- ASSLQS | GVPSRFSASGSG-- TDFTLTISSLQPEDFASYYC | QQSNS----------- -------------LPFT | FGPGT KVDIK |
| iPS:434 045 | 21-225_50H10 | VK1|O12/ JK3 | | DIQMIQSPSSLSA SVGDRVNITC | RAS--QSIY-- ----SYLI | WYQQKPGK APKLLIY | A-------- ASSLQS | GVPSRFSASGSG-- TDFTLTISSLQPEDFASYYC | QQSNS----------- -------------IPFT | FGPGT KVDIK |
| iPS:434 049 | 21-225_50B12 | VK1|O12/ JK3 | | DIQMIQSPSSLSA SVGDRVTITC | RAS--QSIS-- ----SYLN | WYQHKPGK APRLLIY | A-------- ASSLQS | GVPSRFSGSESG-- TDFILTISSLQPEDFTTYYC | QQSYI----------- -------------APFT | FGPGT KVDIK |
| iPS:434 073 | 21-225_51H10 | VK1|O12/ JK3 | | DIQMIQSPSSLSA SVGDRVTITC | RAS--QSIS-- ----TYLM | WYQQKPGK APKLLIY | A-------- ASSLQS | GVPSEFSASGSG-- TDFTLTISSLQPEDFATYYC | QQSYS----------- -------------IPFT | FGPGT KVDIK |
| iPS:434 107 | 21-225_53E2 | VK1|O12/ JK3 | | DIQMIQSPSSLSA SVGDRVTITC | RAS--QSFS-- ----HYLN | WYQQKPGK APNLLIF | A-------- VSSLQS | GVPSRFSGSGSG-- SDFTLPISSLQPEDFAIYFC | QQSFS----------- -------------TPFT | FGPGT KVDIK |
| iPS:434 161 | 21-225_56B2 | VK1|O12/ JK3 | | DIQMIQSPSSLSA SVGDRVTITC | RAS--QSFS-- ----HYLN | WYQQKPGK APNLLIF | A-------- VSSLQS | GVPSRFSGSGSG-- TDFTLTISSLQPEDFAIYFC | QQSYS----------- -------------TPFT | FGPGT KVDIK |
| iPS:434 225 | 21-225_60E12 | VK1|O12/ JK3 | | DIQMIQSPSSLSA SVGDRVTITC | RAS--QSIS-- ----SYLN | WYQQKPGK APKLLIY | A-------- ASSLQS | GVPSRFSGSGSG-- TDFTLTISSLQPEDFAPYYC | QQSYN----------- -------------ISFT | FGPGT KVDIK |
| iPS:434 227 | 21-225_61A1 | VK1|O12/ JK3 | | DIQMIQSPSSLSA SVGDRVTITC | RAS--QSIS-- ----SYLN | WYQQKPGK APKLLIY | A-------- ASSLQS | GVPSRFSGSGSG-- TDFTLTISSLQPEDFAPYYC | QQSYN----------- -------------ISFT | FGPGT KVDIK |
| iPS:434 245 | 21-225_62H1 | VK1|O12/ JK3 | | DIQMIQSPSSLSA YVGDRVTITC | RAS--QNIF-- ----SYLN | WYQQKPGK APKVLIY | V-------- VFSLQS | GVPSRFSGSGSG-- TDFTLTISSLQPEDFATYYC | QQSYN----------- -------------TPFT | FGPGT KVDIK |
| iPS:434 267 | 21-225_57F2 | VK1|O12/ JK3 | | DIQMIQSPSSLSA SVGDRVTITC | RAS--QSIS-- ----SYLN | WYQQKPGK APKLLIY | A-------- ASSLQS | GVPSRFSGSGSG-- TDFTLTISSLQPEDFAPYYC | QQSYN----------- -------------ISFT | FGPGT KVDIK |
| iPS:434 323 | 21-225_62H8 | VK1|O12/ JK3 | | DIQMIQSPSSLSA SVGDRVTITC | RAS--QSIF-- ----SYLN | WYQQNPGK APKLLIY | A-------- SSSLQS | GVPSRLSGNGSG-- TDFILTISSLQPEDFATYYC | QQSYS----------- -------------TPFT | FGPGT RVDIK |
| iPS:434 379 | 21-225_66A9 | VK1|O12/ JK3 | | DIQMIQSPSSLSA SVGDRVTITC | RAS--QNIF-- ----SYLN | WYQQKPGK APKLLIF | A-------- ASSLQS | GVPSRFSGSGSG-- TDFTLTISSLQPEDFATYYC | QQTYS----------- -------------VPFT | FGPGT KVDFK |
| iPS:434 417 | 21-225_69C8 | VK1|O12/ JK3 | | DIQMIQSPSSLSA SVGDRVTFTC | RAG--QTIY-- ----NYLN | WYQQKPGK APKLLIH | V-------- ASSLQS | GVPSRFSGSGSG-- TDFTLVISSLQPEDFATYYC | QQSYS----------- -------------TPFT | FGPGT KVDIK |
| iPS:435 545 | 21-225_158F4 | VK1|O12/ JK3 | | DIQMIQSPSSLPA SVGDRVTITC | RAS--QNIR-- ----KYLH | WYQFLPGK APKLLIY | T-------- ASTLQS | GVPSRFSGSGSG-- TDFTLTISSLQPEDFATYYC | QQSYN----------- -------------ISFT | FGPGT KVDIK |
| iPS:435 793 | 21-225_180F8 | VK1|O12/ JK3 | | DIQMIQSPSSLSA SVGDGVTITC | RAS--QIIL-- ----SYLN | WYQQKPGK APKLLIY | G-------- VSSLQS | GVPSRFSGSGSG-- TDFSLTISSLQPEDFATYYC | QQSYS----------- -------------TPFT | FGPGT KVDIK |
| iPS:436 504 | 21-225_222H4 | VK1|O12/ JK3 | | DIQMIHSPSSLSA SVGDRVTITC | RAS--QNIS-- ----NYVN | WYQQKPGK APKFLIY | T-------- ASSLQS | GVSSRFSGSGSG-- TDFTLTISSVHRDDFAIYYC | QQYYF----------- -------------TPFT | FGRGT KVDIK |

| | | | | | | | | | |
|---|---|---|---|---|---|---|---|---|---|
| iPS:436 510 | 21-225_222H8 | VK1\|O12/ JK3 | DIQMTHSPSSLSA SVGDRVTITC | RAS--QNIS-- ----NYVN | WYQQKPGK APKFLIY | I-------- ASSLQS | GVSSRFSGSGSG-- TDFTLTISSVHRDDFAIYYC | QQYYF----------- ------------TPFT | FGRGT KVDIK |
| iPS:437 230 | 21-225_62H10 | VK1\|O12/ JK3 | DIQMTQSPSSLSA SVGDRVTITC | RAS--QSIT-- ----SYLN | WYQQKPGK VPKLLIS | T-------- ASSLQS | GVPSRFSGSGSG-- TDFTLTISSLQPEDFATYYC | QQSHS----------- ------------FPFT | FGPGT NVDFK |
| iPS:448 906 | 21-225_72G9 | VK1\|O12/ JK3 | DIQMTQSPSSLSA SVGDRVTITC | RAS--QSIT-- ----SYLN | WYQQKPGK APKLLIY | T-------- ASSLQS | GVPSRFSGSGSG-- TDFTLTISSLQPEDFATYYC | QQSHS----------- ------------FPFT | FGPGT KVDIK |
| iPS:392 652 | 21-225_17C6 | VK1\|O12/ JK3 | DIQMTQSPSSLSA SVGDRVTITC | RAS--QSIN-- ----TYLN | WYQQKPGK APKLLIY | A-------- ASSLQS | GVPSRFSGSGSG-- TDFTLTISSLQPEDFATYFC | QQSYR----------- -----------TPFFT | FGPGT KVDIK |
| iPS:392 660 | 21-225_19B3 | VK1\|O12/ JK3 | DIQMTQSPSSLSA SVGDRITITC | RAG--QNII-- ----NYLN | WYQQKPGK APNLLIY | V-------- ASSLQS | GVPSRFNGSGSG-- TDFTLTISSLQPEDFATYYC | QQSYS----------- ------------TPFT | FGPGT KVDIK |
| iPS:392 668 | 21-225_17B4 | VK1\|O12/ JK3 | DIQMTQSPSSLSA SVGDRVTITC | RAS--QNIS-- ----SYLN | WYQQKPGK APKLLIF | G-------- ASSLQT | GVPSRFSGSGSG-- TDFTLTINSLQPEDFATYFC | QQSYR----------- -----------TPFFT | FGPGT KVDIK |
| iPS:392 678 | 21-225_20F3 | VK1\|O12/ JK3 | DIQMTQSPSSLSA SVGDRVTITC | RAS--QSIS-- ----SYLY | WYQQKPGK APKLLIY | A-------- ASSLQS | GVPSRFSGSGSG-- TDFTLTISSLQPEDFATYYC | QQSYS----------- -----------APPFT | FGPGT KVDIK |
| iPS:392 694 | 21-225_19A5 | VK1\|O12/ JK3 | DIQMTQSPSSLSA PVGDRVSITC | RAS--QNII-- ----NYLN | WYQQKPGK APKLLID | V-------- ASNLQG | GVPSRFSGSGSG-- TDFTLTISSLQPEDFATYYC | QQSYS----------- ------------TPFT | FGPGT KVDIK |
| iPS:392 696 | 21-225_20A4 | VK1\|O12/ JK3 | DIQMTQSPASLSA SVGDRVTITC | RAS--QSI-- ----NYLN | WYQQRPGK SPKLLIY | A-------ASSLHS | GVPSRFSGRGSG-- TDFTLTISSLQPEDFATYFC | QQSYR----------- -----------TPLFT | FGPGT KVDFK |
| iPS:392 702 | 21-225_17F7 | VK1\|O12/ JK3 | DIQMTQSPSSLSA SVGDRVTITC | RAS--QTIS-- ----SFLN | WYQQKPGK APKLLIY | A-------- ASSLQS | GVPSRFSGSGSG-- TDFTLTISGLQPEDFATYFC | QQSYR----------- -----------TPFFT | FGPGT KVDIK |
| iPS:392 704 | 21-225_17F11 | VK1\|O12/ JK3 | DIQMTQSPSSLSA SIGDRVSITC | RAS--RTIN-- ----NYLN | WYQQKPGK APKLLIF | TSSLQS | GVPSRFSGSGSG-- TDFTLTISSLQPEDFATYYC | QQTYS----------- -----------TPLFA | FGPGT KVDIK |
| iPS:392 720 | 21-225_17A12 | VK1\|O12/ JK3 | DIQMTQSPSSLSA SVGDRVTITC | RAS--QSIS-- ----SYLN | WYHQKPGK APKLLIY | A-------- ASSLQS | GVPSRFSGSGSG-- TDFTLTISNLQPEDFATYYC | QQSYN----------- -----------TPLFT | FGPGT KVDIK |
| iPS:392 722 | 21-225_18E12 | VK1\|O12/ JK3 | DIQMTQSPSSLSA SVGDRVTITC | RAS--QSIS-- ----SYLN | WYQQKPGK APTLLIY | A-------- ASSLQS | GVPSRFSGSGSG-- TDFTLTINSLQPEDFATYYC | QQSYR----------- -----------TPFFT | FGPGT KVDIK |
| iPS:392 760 | 21-225_22G3 | VK1\|O12/ JK3 | DIQMTQSPSSLSA SVGDRVTITC | RAS--QSIS-- ----NYLN | WYQQKPGK APKLLIF | A-------- ASSLQS | GVPSRFSGSGSG-- TDFTLTISGLQPEDFATYFC | QQSFR----------- -----------TPFFT | FGPGT KVDIK |
| iPS:392 762 | 21-225_22G5 | VK1\|O12/ JK3 | DIQMTQSPSSLSA SVGDRVTITC | RAS--QNIS-- ----SYLN | WYQQKQGK APKLLIY | A-------- ASSLQN | GVPSRFSGRGSG-- TDFTLTISSLQPEDFATYYC | QQSYR----------- -----------TPLFT | FGPGT KVDFK |
| iPS:392 764 | 21-225_22G10 | VK1\|O12/ JK3 | DIQMTQSPSSLSA SVGDRVTITC | RAS--QSIF-- ----SYLN | WYQQKPGK APKLLIY | A-------- ASSLQS | GVPSRFSGSGSG-- TDFTLTISSLQPEDFATYFC | QQSFR----------- -----------TPLFT | FGPGT KVDFK |
| iPS:392 812 | 21-225_21F4 | VK1\|O12/ JK3 | DIQMTQSPSSLSA SVGDRVTITC | RAS--QNIG-- ----SYLN | WYQQKPGK APKLLIF | A-------- ASSLQS | GVPSRFSGSGSG-- TDFTLTISSLQPEDFATFFC | QQSYR----------- -----------TPFFT | FGPGT KVDFK |
| iPS:392 816 | 21-225_22E4 | VK1\|O12/ JK3 | DIQMTQSPSSLSA SVGDRVTITC | RAS--QNIS-- ----SYLN | WYQQKPGK APKLLIY | A-------- ASSLQS | GVPSRFSGSGSG-- TDFTLTISSLQPEDFATYYC | QQSYN----------- -----------TPLFT | FGPGT KVDIK |
| iPS:392 830 | 21-225_21A5 | VK1\|O12/ JK3 | DIQMTQSPSSLCA SVGDRVTITC | RAS--QTIS-- ----SHLN | WYQRKPGK APKLLIY | A-------- ASSLQS | GVPSRFSGSGSG-- TDFTLTISSVQPEDFATYYC | QQSYN----------- ------------ISFT | FGPGT KVDIK |
| iPS:392 852 | 21-225_21A2 | VK1\|O12/ JK3 | DIQMTQSPSSLSA SVGDRVTITC | RAS--QSIS-- ----SYLN | WYQQKPGK APKLLIY | A-------- ASSLQS | GVPSRFSGSGSG-- TDFTLTISSLQPEDFATYFC | QQSYR----------- -----------TPFFT | FGPGT KVDIK |
| iPS:392 878 | 21-225_22C5 | VK1\|O12/ JK3 | DIQMTQSPASLSA SVGDRVTITC | RAS--QNIS-- ----SYLN | WYQQKPGK APKLLIY | A-------- ASVLQH | GIPSRFSGRGSG-- TDFTLIISSLQPEDFATYYC | QQSYR----------- -----------TPLFT | FGPGT KVDFK |
| iPS:392 902 | 21-225_22D5 | VK1\|O12/ JK3 | DIQMTQSPSSLSA SVGDRVTITC | RAS--QNIF-- ----SYLN | WYHQKPGK APKLLIY | A-------- ASSLQS | GVPSRFSGSGSG-- TDFTLTISSLQPEDFATYYC | QQSYS----------- -----------TPLFT | FGPGT KVDIK |
| iPS:392 984 | 21-225_30E11 | VK1\|O12/ JK3 | DIQMTQSPSSLSA SVGDRVTITC | RAS--QSIS-- ----NYLN | WYQQQTGK APKFLIY | A-------- ASSLQS | GVPSRFSGSGSG-- TDFTLTISSLQPEDFATYYC | QQSYS----------- -----------TPFT | FGPGT KVDIK |

| | | Germline | | K_FR1 | K_CDR1 | K_FR2 | K_CDR2 | K_FR3 | K_CDR3 | K_FR4 |
|---|---|---|---|---|---|---|---|---|---|---|
| iPS:393 114 | 21-225_33G12 | VK1\|O12/ JK3 | | DIQMTQSPSSLSA SVGDRVTITC | RAS--QSIS-- ----NYLN | WYQQQTGK APKFLIY | A-------- ASSLQS | GVPSRFSGSGSG-- TDFTLTIISSLQPEDFATYYC | QQSYS----------- ------------TPFT | FGPGT KVDIK |
| iPS:393 824 | 21-225_10F12 | VK1\|O12/ JK3 | | DIQMTQSPSSLSA SVGDRVTITC | RAS--QNIS-- ----SYLN | WYQQKPGK APKLLIY | A-------- ASSLQS | GVPSRFSCSGSG-- TDFTLTISSLQPEDFATYYC | QQSYN----------- ----------TPFFT | FGPGT KVDIK |
| iPS:393 848 | 21-225_4H2 | VK1\|O12/ JK3 | | DIQMTLSPSSLSA SVGDRVTITC | RAI--QNIS-- ----SYLN | WYQQKPGK APKLVIY | A-------- ASSLQS | GVPSRFSGRGSG-- TDFTLTIGCVQREDFATYYC | QQSYR----------- ----------TPLFT | FGPGT KVDIK |
| iPS:393 862 | 21-225_5G2 | VK1\|O12/ JK3 | | DIQMTQSPSSPSA SVGDRVTITC | RAS--QNII-- ----SYLN | WYQQKPGK ARKLVIY | G-------- ASSLQS | GVPSRFSGSGSG-- TDFTLNIRSLQPEDFATYYC | QQSYS----------- ----------TPLFT | FGPGT KVDIK |
| iPS:393 888 | 21-225_3E3 | VK1\|O12/ JK3 | | DIQMTQSPSSPSA SVGDRVTITF | RAS--QSIR-- ----SYLN | WYQQKPGK AHKLVIY | G-------- TSSLQS | GVPSRFSCSGSG-- TDFTLTISSLQPEDFATYYC | QQSYS----------- ----------TPLFT | FGPGT KVDIK |
| iPS:393 890 | 21-225_4B1 | VK1\|O12/ JK3 | | DIQMTQSPSSLSA SVGDRVTITC | RAS--HTIR-- ----TYLN | WYQQKPGK APKLLIY | A-------- ASSLQS | GVPSRINGSGSG-- TDFTLTIINLQPEDFATYYC | QQSYN----------- ------------ISFT | FGPGT KVDIK |
| iPS:393 898 | 21-225_5F7 | VK1\|O12/ JK3 | | DIQMTQSPSSPSA SVGDRVTITF | RAS--QTIS-- ----SYLN | WYQQKPGK APKLLIS | A-------- ASSLQS | GVPSRFSGSGSG-- TDFTLTISSLQPEDFATYYC | QQSYN----------- ----------TPLFT | FGPGT KVVIK |
| iPS:393 904 | 21-225_8H11 | VK1\|O12/ JK3 | | DIQMTQSPSSLSA FVGDRVTITC | RAS--QNII-- ----SYLN | WYQQKPGK APNLMIY | V-------- TSSLHS | GVPSRFSGSGSG-- TDFSLTISSLQPEDFATYYC | QQSYS----------- -----------TPFT | FGPGT KVDIK |
| iPS:393 936 | 21-225_14A11 | VK1\|O12/ JK3 | | DIQMTQSPSSLSA SIGDRVTITC | RAS--QSIS-- ----SYLN | WYQQKPGK APKLLIF | A-------- ASSLQN | GVPSRFSGSGSG-- TDFTLTISSLQPEDFATYYC | QQTYS----------- ----------SPPFT | FAPGT KVDIK |
| iPS:393 980 | 21-225_6D3 | VK1\|O12/ JK3 | | DIQMTQSPSSLSA SVGDRVTITC | RTS--QSIS-- ----TYLN | WYQQKPGK APKLLIF | A-------- ASSLQS | GVPSRFSGSGSG-- TDFTLTISSLQPEDFATYFC | QQSYR----------- ----------TPFFT | FGPGT KVDIK |
| iPS:394 014 | 21-225_8G6 | VK1\|O12/ JK3 | | DIQMTQSPSSLSA SVGDRVTITC | RAS--QSIS-- ----SYLN | WYQQKPGK APKLLIF | A-------- ASSLQS | GVPSRFSGSGSG-- TDFTLTISSLQPEDFATYFC | QQSYR----------- ----------TPFFT | FGPGT KVDIK |
| iPS:394 022 | 21-225_16H6 | VK1\|O12/ JK3 | | DIQMTQSPSSLSA SVGDRVTITC | RAS--QNIS-- ----SYLN | WYQQKPGK APKLLIY | A-------- ASSLQS | GVPSRFSGRGSG-- TDFTLTISSLQPEDFATYYC | QQSYS----------- ----------TPLFT | FGPGT KVDFK |
| iPS:394 043 | 21-225_3B1 | VK1\|O12/ JK3 | | DIQMTQSPSSLSA SVGDRVTITC | RAS--QSIN-- ----NYLN | WYQQKPGK APKLLIY | A-------- TSSLQN | GVPSRFSGSGSG-- TDFTLTISSLQPEDFATYYC | QQSYS----------- ----------TPLFT | FGPGT KVDIN |
| iPS:394 051 | 21-225_9E5 | VK1\|O12/ JK3 | | DIQMTQSPSSLSA SVGDRVTITC | RAS--QSIA-- ----SYLN | WYQQKPGK APKLLIY | G-------- ASSLQS | GVPSRFSGSGSG-- TDFTLTISSLQPEDFATYYC | QQSYS----------- ----------TPLFS | FGPGT KVDIK |
| iPS:394 077 | 21-225_8E12 | VK1\|O12/ JK3 | | DIQMTQSPSSLSA SVGDRVTITC | RAS--QSIS-- ----SYLN | WYQQKPGK APKLLIY | A-------- ASSLQS | GVPSRFSGSGSG-- TDFTLTISSLQPEDFATYFC | QQSYR----------- ----------TPFFT | FGPGT KVDIK |
| iPS:394 087 | 21-225_11A5 | VK1\|O12/ JK3 | | DIQMTQSPSSLSA SVGDRVTITC | RAS--QNIY-- ----SYLN | WYQQKPGK APKLLIY | A-------- ASSLQS | GVPSRFSGSGSG-- TDFTLTISSLQPEDFATYYC | QQSYN----------- ----------TPLFT | FGPGT KVDIK |
| | | Germline | | K_FR1 | K_CDR1 | K_FR2 | K_CDR2 | K_FR3 | K_CDR3 | K_FR4 |
| | VK2\|A18/JK 3 | | 32151 | DIVMTQTPLSLSV TPGQPASISC | KSS-- QSLLHSD- GKTYLY | SPQLLIY | VSSRFS | GVPDRFSGSGSG-- TDFTLKISRVEAEDVGVYYC | MQGIH----------- ------------LPFT | FGPGT KVDIK |
| iPS:434 053 | 21-225_51E1 | VK2\|A18/ JK3 | | DIVMTQTPLSLSV TPGQPASMSC | KSS-- QSLLHSE- GKTYLY | WYLRKPGQ PPQFLIF | E-------- VSNRFS | GVPDRFSGSGSG-- TEFTLKISRVEAEDVGVYYC | MQSIQ----------- ------------LPFT | FGPGT KVDIK |
| iPS:434 137 | 21-225_54D4 | VK2\|A18/ JK3 | | DIVMTQTPLSLSV TPGQPASMSC | KSS-- QSLLHSE- GKTYLY | WYLRKPGQ PPQFLIF | E-------- VSNRFS | GVPDRFSGSGSG-- TEFTLKISRVEAEDVGIYYC | MQSIQ----------- ------------FPFT | FGPGT KVDIK |
| iPS:434 149 | 21-225_55H1 | VK2\|A18/ JK3 | | DIVMTQTPLSLSV TPGQPASISC | KSS-- QSLLHSE- GKTYLY | WYLQKPGQ PPQFLIF | E-------- VSHRFS | GVPDRFSCSGSG-- TDFTLKISRVEAEDVGVYYC | MQSIQ----------- ------------LPFT | FGPGT KVDIK |

| ID | Clone | VK/JK | No. | K_FR1 | K_CDR1 | K_FR2 | K_CDR2 | K_FR3 | K_CDR3 | K_FR4 |
|---|---|---|---|---|---|---|---|---|---|---|
| iPS:435 315 | 21-225_147B2 | VK2\|A18/ JK3 | | DIVMTQTPLSLSV TPGQPASISC | XSS-- QSLLHSE- GKTYLY | WYLQKPGQ PPQLLIY | E-------- VSHRVS | GVPDRFSGSGSG-- TDFTVKISRVEAEDVGVYYC | MQSTQ----------- -----------FPPT | FCPGT KVDIK |
| | | Germline | | K_FR1 | K_CDR1 | K_FR2 | K_CDR2 | K_FR3 | K_CDR3 | K_FR4 |
| | VK1\|O18/J K3 | | 32152 | DIQMTQSPSSLSA SVGDRVTITC | QAS--QDIS-- ----NYLN | WYQQKPGK APKLLIY | D-------- ASNLET | GVPSRFSGSGSG-- TDFTFTISSLQPEDIATYYC | QQYDN----------- -----------LPFT | FCPGT KVDIK |
| iPS:434 055 | 21-225_51B4 | VK1\|O18/ JK3 | | DIQMTQSPSSLSA SVGDRVTITC | QAS--RDIT-- ----FYLN | WYQQKPGK APKLLIY | D-------- ASNLET | GVPSRFSGSGSG-- TDFTFTISCVHPEDIATYLC | QQYDN----------- -----------LPFT | FCPGT TVDIK |
| | | Germline | | K_FR1 | K_CDR1 | K_FR2 | K_CDR2 | K_FR3 | K_CDR3 | K_FR4 |
| | VK1\|O18/J K4 | | 32153 | DIQMTQSPSSLSA SVGDRVTITC | QAS--QDIS-- ----NYLN | WYQQKPGK APKLLIY | D-------- ASNLET | GVPSRFSGSGSG TDFTFTISSLQPEDIATYYC | QQYDN -----------LPLT | FGGGT KVEIK |
| iPS:434 087 | 21-225_52F6 | VK1\|O18/ JK4 | | DIQMTQSPSSLSA SVGDRVTITC | QAS--QDIS-- ----NYLH | WYQQKPGK APKLLIY | D-------- ASTLGT | GVPLRFSGSGSG-- TEFTFTINSLQPEDIATYSC | QQCDN----------- -----------LPLT | FGGGT KVEIK |
| iPS:434 111 | 21-225_53H2 | VK1\|O18/ JK4 | | DIQMTQSPSSLSA SVGDRVTITC | QAS--QDIS-- ----NYLH | WYQQKPGK APQLLIY | D-------- ASNLET | GVPSRFGSGSGSG-- TDFTFTISSLQPEDIATYYC | HQYDN----------- -----------LPLT | FGGGT KVEIK |
| iPS:434 121 | 21-225_53F6 | VK1\|O18/ JK4 | | DIQMTQSPSSLSA SVGDRVTITC | QAS--QDIT-- ----NYLD | WYQQKPGK APKLLIY | D-------- ASNLGT | GVPSRFSGSGSG-- TDFTFTISSLQPEDIATYYC | QQCDN----------- -----------LPLT | FGGGT KVEIK |
| iPS:434 163 | 21-225_50H1 | VK1\|O18/ JK4 | | DIQMTQSPSSPSA SVGDRVTITC | QAS--QDIS-- ----NYLD | WYQQKPGK APKLLIY | D-------- ASNLET | GVPSRFSGSGSG-- TDFAFTISSLQPEDIATYYC | QQCDN----------- -----------LPLT | FGGGT KVEIK |
| iPS:435 611 | 21-225_161F10 | VK1\|O18/ JK4 | | DIQMTQSPSSLSA SVGDRVTITC | QAS--QDIY-- ----NHLS | WYQQKPGK APKLLIY | D-------- ASNWET | GVPSRFSGSGSG-- TDFTFTISSLQPEDFATYYC | QQYEN----------- -----------LPLT | FGGGT KVEIK |
| iPS:435 811 | 21-225_183H6 | VK1\|O18/ JK4 | | DIQMTQSPSSLSA SVGDRVTIAC | QAS--QDIS-- ----NYLN | WYQQTPGK APKVLIY | D-------- ASNLET | GVPARFSGSGSG-- TDFTFTISSLQPEDIATYYC | QQYDN----------- -----------LPLT | FGGGT KVDIK |
| iPS:394 035 | 21-225_5G9 | VK1\|O18/ JK4 | | DIQMTQSPSSLSA SVGDRVTITC | QAS--QGIS-- ----NSLN | WYQQKPGK APKLLIY | D-------- ASNLET | GVPSRFSGSGAG-- TDFTFTISSLQPEDIATYYC | QQYDN----------- -----------LPLT | FGGGT KVEIK |
| | | Germline | | K_FR1 | K_CDR1 | K_FR2 | K_CDR2 | K_FR3 | K_CDR3 | K_FR4 |
| | VK1\|L1/JK1 | | 32154 | DIQMTQSPSSLSA SVGDRVTITC | RAS--QGIS-- ----NYLA | WFQQKPGK APKSLIY | A-------- ASSLQS | GVPSRFSGSGSG-- TDFTLTISSLQPEDFATYYC | QQYNS----------- -----------YPWT | FGGGT KVEIK |
| iPS:434 095 | 21-225_52F10 | VK1\|L1/J K1 | | DIQMTQSPSSLSV SVGDRVTITC | RAS--QGIS-- ----NYLG | WFQQKPGK APKSLIY | A-------- ASSLQS | GVPSRFSGSGSG-- TDFTLTISSLQPEDFATYYC | QQYNS----------- -----------YPPT | FGGGT KVEIK |
| iPS:392 848 | 21-225_20F9 | VK1\|L1/J K1 | | DIQMTQSPSSLSA SVGDRITITC | RAS--QGIS-- ----NYLA | WFQQKPGK APKSLIS | A-------- ASSLQS | GVPSRFSGSGSG-- TDFTLTISSLQPEDFATYCC | QQYFS----------- -----------YPWT | FGGGT KVEIK |
| iPS:393 078 | 21-225_33H11 | VK1\|L1/J K1 | | DIQMTQSPSSLSA FVGDRVTITC | WAS--QGIN-- ----SYLA | WFQQKPGK AHKSLIY | A-------- ASSLQG | GVPSRFSGSGSG-- TDFILTISSLQREDFATYYC | QQFNS----------- -----------YPLT | FGGGT KVEIK |
| iPS:393 142 | 21-225_33A3 | VK1\|L1/J K1 | | DIQMTQSPSSLSA SVGDRVTITC | RAS--QGIN-- ----NYLA | WFQQKPGK APKSLIY | A-------- ASSLQS | GVPSRFSGSGSG-- TDFTLTISSLQPEDFATYYC | QQFNS----------- -----------YPPT | FGGGT KVEIK |
| iPS:393 946 | 21-225_16A4 | VK1\|L1/J K1 | | DIQMTQSPSSLSA SVGDRVTITC | RAS--QDIS-- ----NYLA | WFQQKPGK APKSLIS | A-------- ASSLQS | GVPSRFSGSGSG-- TDFTLTISSLQPEDFATYYC | QQYFS----------- -----------YPWT | FGGGT KVEIN |
| | | Germline | | K_FR1 | K_CDR1 | K_FR2 | K_CDR2 | K_FR3 | K_CDR3 | K_FR4 |
| | VK1\|O12/J K5 | | 32155 | DIQMTQSPSSLSA SVGDRVTITC | RAS--QSIS-- ----SYLN | WYQQKPGK APKLLIY | A-------- ASSLQS | GVPSRFSGSGSG-- TDFTLTISSLQPEDFATYYC | QQSYS----------- -----------TPIT | FGGGT RLEIK |
| iPS:434 117 | 21-225_53C6 | VK1\|O12/ JK5 | | DIQMTQSPSSLSA SVGDRVTITC | RAS--QSIS-- ----DYLN | WYQQKPGK APKVLIF | A-------- ASSLKS | GVPSRFSGSGSG-- TDFTLTISSLEPEDFATYFC | QQSYS----------- -----------TPFT | FGGGT RLEIK |
| iPS:434 317 | 21-225_59E8 | VK1\|O12/ JK5 | | DIQMTQSPSSLSA SVGDRVTITC | RAS--QSIS-- ----SYLN | WYQQKPGK APKLLIY | A-------- ASSLQS | GVPSRFSGSGSG-- TDFTLTISSLQPEDFATYFC | QQSFS----------- -----------NSIT | FGGGT RLEIK |
| iPS:434 327 | 21-225_63G6 | VK1\|O12/ JK5 | | DIQMTQSPSSLSA SVGDRVIISC | RAS--QSIF-- ----SYLN | WYQVKPGK APKLLIY | D-------- TSTLQT | GVPSRFSGSGSG-- TDFTLTINSLQPEDFATYYC | QQSYG----------- -----------IPIT | FGGGT RLEIQ |

| ID | Clone | Germline | # | K_FR1 | K_CDR1 | K_FR2 | K_CDR2 | K_FR3 | K_CDR3 | K_FR4 |
|---|---|---|---|---|---|---|---|---|---|---|
| iPS:434 455 | 21-225_72F5 | VK1\|O12/JK5 | | DIQMTQSPSSLSA SVGDRVIIPC | RAS--QNIS-- ----SYLN | WYQQKPGK APKLLIY | A-------- ASSLQS | GVPSRFSGSGSG-- TDFTLTISSLQPEDFATYSC | QQTY------------ ------------STPT | FGQGT RLDIN |
| iPS:435 525 | 21-225_157E7 | VK1\|O12/JK5 | | DIQMTQSPSSLSA SVGDRVTITC | RAS--QSFS-- ----SYLN | WYQQKPGI APKLLIY | A-------- ASSLQS | GVPSRFSGSGSG-- TDFTLTISSLQPEDFATYYC | QESYS----------- -------------IRFA | FGQGT RLEIK |
| iPS:392 754 | 21-225_21D3 | VK1\|O12/JK5 | | DIQMTQSPSSLSA SVGDRVTITC | RAS--QSIT-- ----GYSN | WYQQKPGK TPKLLIF | A-------- TYSLES | GVPSRFSGSGFG-- TNFTLTIISLQPEDFATYYC | QQSYS----------- ------------TSIT | FGQGT RLEIK |
| iPS:392 818 | 21-225_22D8 | VK1\|O12/JK5 | | DIQMTQSPSSLSA SVGDRVTITC | RTS--QNSN-- ----SYLN | WYQQKPGK APKLQIF | A-------- AYSLES | GVPSRFSGNRSG-- TEFTLTISSLQPEDFATYYC | QQTYG----------- ------------TSIT | FGQGT RLEIK |
| iPS:393 064 | 21-225_33A9 | VK1\|O12/JK5 | | DIQMTQSPSSLSA SVGDRVTITC | RAS--QSIS-- ----RYLS | WYQQKPGR APNLQIY | A-------- ASSLQS | GVPSRFSGSGSG-- TDFTLTISSLQPEDFATYYC | QQSYN----------- ------------IPIT | FGQGT RLEIK |
| iPS:393 148 | 21-225_35E5 | VK1\|O12/JK5 | | DIQMIQSPSSLSA SVGDRVTITY | RAS--QSIS-- ----SYLN | WYQQKPAK APKLHIY | G-------- ASSFQS | WVPSRFSGSGSS-- TDFTLTIISMQPGDYATYYC | HQSYN----------- ------------LPIT | FGQGT RLEIK |
| iPS:398 536 | 21-225_33D12 | VK1\|O12/JK5 | | DVQMTQSPSSLSA SLGDRVTITC | RAS--QSIR-- ----SYLN | WYQQKPGK APNLLIY | S-------- ASSLQS | GVPSRFSGSGSG-- TDFTLTISSLQPEDFANYYC | QQSYS----------- ------------IPIT | FGQGT RLEIK |
| | | **Germline** | | **K_FR1** | **K_CDR1** | **K_FR2** | **K_CDR2** | **K_FR3** | **K_CDR3** | **K_FR4** |
| | VK3\|A27/JK2 | | 32156 | EIVLTQSPGTLSL SPGERATLSC | RAS--QSVSS-- ----SYLA | WYQQKPGQ APRLLIY | G-------- ASSRAT | GIPDRFSGSGSG-- TDFTLTISRLEPEDFAVYYC | QQYGS----------- ------------SPYT | FGQGT KLEIK |
| iPS:434 127 | 21-225_53H8 | VK3\|A27/JK2 | | EIVLTQSPGTLSL SPGERATLSC | RAS--QSITS-- ----SYLA | WYQQKPGQ APRLLIY | G-------- ASGRAT | GIPDRFSGSGSG-- TDFTLTISRLEPEDFAVYYC | QQFES----------- -----------SPMCS | FGQGT NLEIK |
| iPS:436 290 | 21-225_205G3 | VK3\|A27/JK2 | | EIVLTQSPGTLSL SPGERATLSC | RAS--QNVSY-- ----SYLA | WYQQKPGQ APRLLIY | G-------- ASRRAT | GIPDRFSGSGSG-- TDFTLTISRLEPEDFAVYYC | QQYGS----------- ------------SPCS | FGQGT KLEIK |
| iPS:436 568 | 21-225_225B3 | VK3\|A27/JK2 | | EIVLTQSPGTLSL SPGERATLSC | RAS--QNLSS-- ----SYLG | WYQQKPGQ APRLLIY | D-------- TSSRAT | GIPDRFSGSGSG-- TDFTLTISRLEPEDFAVYYC | QEYGS----------- -----------SLMCS | FGQGT KLEIK |
| iPS:392 898 | 21-225_21H10 | VK3\|A27/JK2 | | EIVLTQSPGTLSL SPGERATLSC | RAS--QSFSS-- ----SYLA | WYQQKPGQ APRLLIY | G-------- ASSRAT | GIPDRFSGSGSG-- TDFTLTISRLEPEDFAVYYC | QQYG------------ -----------SSRS | FGQGT KLEIK |
| iPS:393 802 | 21-225_3D12 | VK3\|A27/JK2 | | EIVLTQSPGILSL SPGERATLSC | RAS--QSVSS-- ----SYLA | WYQQKPGQ APRLLIY | G-------- TSSRAT | GIPDRFSGSGSG-- TDFTLTISRLEPEDFAVYYC | QQYG------------ -----------SSRS | FGQGT KLEIK |
| | | **Germline** | | **K_FR1** | **K_CDR1** | **K_FR2** | **K_CDR2** | **K_FR3** | **K_CDR3** | **K_FR4** |
| | VK1\|L1/JK4 | | 32157 | DIQMTQSPSSLSA SVGDRVTITC | RAS--QGIS-- ----NYLA | WFQQKPGK APKSLIY | A-------- ASSLQS | GVPSRFSGSGSG-- TDFTLTISSLQPEDFATYYC | QQYNS----------- ------------YPLT | FGGGT KVEIK |
| iPS:434 157 | 21-225_55D4 | VK1\|L1/JK4 | | DIQMTQSPSSLFA SVGDRVTITC | RAS--QDIS-- ----NYLI | WFQQKPGK APKSLIY | T-------- ASSLQS | GVPSKFSGSGFG-- TDFTLTISNLQPEDFATYYC | QQHS----------- ------------FPLT | FGGGT RVEIR |
| iPS:434 175 | 21-225_55A11 | VK1\|L1/JK4 | | DIQMTQSPSSLSA SVGDRVTITC | RAS--QDIN-- ----IYLA | WFQQKPGK APKSLIY | A-------- ASSLQS | GVPSKFSGSGSG-- TDFTLTISSLQPEDFATYYC | QQYNS----------- ------------YPLT | FGGGT KVEIK |
| iPS:434 367 | 21-225_65H11 | VK1\|L1/JK4 | | DIQMTQSPSSLSA SVGDRVTITC | RAS--QDIS-- ----TYLA | WFQQKPGK APKSLIY | A-------- ASSLQS | GVPSKFSGSGSG-- TDFTLTISSLQPEDFATYYC | QQYNS----------- ------------FPLT | FGGGT KVEIK |
| iPS:434 429 | 21-225_70H6 | VK1\|L1/JK4 | | DIQMTQSPSSLSA SLGDRVTITC | RTS--QSIF-- ----NYLN | WFQRKPGK APKVLIY | T-------- ASSLQS | GIPSRFSGSGSG-- TDFTLTISSLQPEDSATYYC | QQSYS----------- ------------IPLT | FGGGT KVEIK |
| iPS:434 535 | 21-225_74C8 | VK1\|L1/JK4 | | DIQMTQSPSSLSA SVGDRVTITC | RAS--QGIG-- ----KYLA | WFQQKPGK APKSLIY | T-------- TSNLQS | GAPSKFSGSGSG-- TDFTLTISSLQYEDFATYYC | QQYSN----------- ------------YPLT | FGGGT KVEIN |
| iPS:434 573 | 21-225_77E6 | VK1\|L1/JK4 | | DIQMTQSPSSLSA SVGDRVTITC | RAS--QGIS-- ----KYLA | WFQQKPGK APKSLIY | A-------- ASSLQG | GAPSKFSGSGSG-- TDFTLTISSLQPEDFATYYC | QQYSN----------- ------------YPLT | FGGGT RVEIK |
| iPS:434 615 | 21-225_76C5 | VK1\|L1/JK4 | | DIQMTQSPSSLSA SVGDRVTITC | RAS--QVIS-- ----KYLA | WFQQKPGK APKSLIY | A-------- ASSLQS | GAPSKFSGSGSG-- TDFTLTISSLQPEDFATYYC | QQYSN----------- ------------YPLT | FGGGT KVEIK |
| iPS:434 669 | 21-225_79F4 | VK1\|L1/JK4 | | DIQMTQSPSSLSA SVGDRVTITC | RAS--QGIS-- ----KYLA | WFQQKPGK APKSLIY | A-------- ASSLQG | GAPSKFSGSGSG-- TDFTLTISSLQPEDFATYYC | QQYSN----------- ------------YPLT | FGGGT RVEIK |

| ID | Clone | VK | | | | | | | |
|---|---|---|---|---|---|---|---|---|---|
| iPS:434 737 | 21-225_74G6 | VK1\|L1\|JK4 | DIQMTQSPSSLSA SVGDRVTITC | RAS--QGIG-- ----KYLA | WFQQKPGK APKSLIY | T-------- TSSLQS | GAPSKFSGSGSG-- TDFTLTIISSLQYEDFATYYC | QQYSN----------- ------------YPLT | FGGGT KVEIN |
| iPS:434 741 | 21-225_80C11 | VK1\|L1\|JK4 | DIQMTQSPSSLSA SVGDRVTITC | RAS--QGIG-- ----RYLA | WFQQKPGR APKSLIY | T-------- ASSLQS | GAPSKFSGSGSG-- TDFTLTIISSLQPEDFATYYC | QQYSN----------- ------------YPLT | FGGGT KVEIN |
| iPS:434 867 | 21-225_79A12 | VK1\|L1\|JK4 | DIQMTQSPSSLSA SVGDRVTITC | RAS--QVIS-- ----KYLA | WFQQKPGK APKSLIY | A-------- ASSLQS | GAPSKFSGSGSG-- TDFTLTIISSLQPEDFATYYC | QQYSN----------- ------------YPLT | FGGGT KVEIK |
| iPS:435 333 | 21-225_147E9 | VK1\|L1\|JK4 | DIQMTQSPSSLSA SVGDRVTITC | RAS--QDIN-- ----NYLA | WFQQKPGK APKSLIV | A-------- ASSLQS | GVPSKFSGSGSG-- TDFTLTIISSLQPEDFATYYC | QQYNS----------- ------------YPLT | FGGGT KVEIK |
| iPS:435 409 | 21-225_150G8 | VK1\|L1\|JK4 | DIQMTQSPSSLSA SVGDRVTITC | RAS--QGIS-- ----HYLA | WFQQKPGK APKSLIY | V-------- ASSLQN | GVPSRFSGSGSG-- IDFTLTIISSLQPEDFATYYC | QQYNN----------- ------------YPLT | FGGGT KVEIK |
| iPS:435 505 | 21-225_157C1 | VK1\|L1\|JK4 | DIQMTQSPSSLSA SIGDRITLTC | RAS--QGIS-- ----NYLA | WFQQRPGK APKSLIY | A-------- ASSLLS | GVPSKFSGSGSG-- IDFTLTIISSLQPEDFATYYC | QQYNS----------- ------------FPFT | FGGGT KVEIK |
| iPS:435 595 | 21-225_160H4 | VK1\|L1\|JK4 | DIQMTQSPSSLSA SVGDRVTITC | RAS--QDIS-- ----NYLV | WFQQKLCK APKSLIY | V-------- ASSLQS | GVPSRFSGSGSG-- IDFTLTIISSLQPEDFATYYC | QQYNS----------- ------------YPLT | FGGGT KVEIK |
| iPS:435 639 | 21-225_163G6 | VK1\|L1\|JK4 | DIQMTQSPSSLSA SVGDRVTITC | RAS--QDIS-- ----NYLV | WFQQKPGK APKSLIY | A-------- ASSLLS | GVPSKFSGSGSG-- TDFTLTIISSLQPEDFATYYC | QQYHS----------- ------------YPLT | FGGGT KVAIK |
| iPS:435 653 | 21-225_166H12 | VK1\|L1\|JK4 | DIQMTQSPSSLST SVGDRVTITC | RAS--QDIS-- ----HYLA | WFQQKPGK APKSLIY | A-------- ASSLQS | GVPSKFSGSGSG-- TDFTLTIISSLQPEDFATYYC | QQYNS----------- ------------FPLT | FGGGT KVEIT |
| iPS:435 677 | 21-225_169C10 | VK1\|L1\|JK4 | DIQMTQSPSSLSA SVGDRVTITC | RAS--QDIS-- ----NYLA | WFQQKPGK APKSLIF | S-------- ASSLQS | GVPSKFSGSGSG-- TDFNLTIISSLQPEDFATYYC | QQSDS----------- ------------YPLT | FGGGT KVEIK |
| iPS:435 699 | 21-225_170D6 | VK1\|L1\|JK4 | DIQMTQSPSSLSA SVGDRVAITC | RAS--QDIG-- ----NCLA | WFQQKPGK APKSLIY | S-------- ASSLQS | GVPSKFSGSGSG-- TDFTLTIISSLQPEDFATYYC | QQSDS----------- ------------YPLT | FGGGT KVEIK |
| iPS:435 745 | 21-225_175G3 | VK1\|L1\|JK4 | DIQMTQSPSSLSA SVGDRVTITC | RAS--QDIS-- ----NDLA | WFQQKPGK APKSLIY | S-------- ASSLQS | GVPSKFSGSGSG-- TDFTLTIISSLQPEDFATYYC | QQSDS----------- ------------YPLT | FGGGT KVEIK |
| iPS:435 819 | 21-225_190C11 | VK1\|L1\|JK4 | DIQMTQSPSSLSA SVGDRVTITC | RTS--QGIG-- ----NYLA | WFQQKPGK APKSLLY | K-------- TSSLQS | GVPSKFSGSGSG-- TDFTLTIISSLQPEDFATYYC | QQYMT----------- ------------YPLT | FGGGT KVEIK |
| iPS:435 825 | 21-225_190G11 | VK1\|L1\|JK4 | DIQMTQSPSSLSA SVGDRVTITC | RTS--QGIG-- ----NYLA | WFQQKPGK APKSLLY | K-------- ASSLQS | GVPSKFSGSGSG-- TDFTLTIISSLQPEDFATYYC | QQYMT----------- ------------YPLT | FGGGT KVEIK |
| iPS:435 837 | 21-225_198G3 | VK1\|L1\|JK4 | DIQMAQSPSSLSA SVGDRVTITC | RTS--QGIG-- ----KYLA | WFQQKPGK APKSLLY | K-------- ASSLQG | GVPSKFSGSGSG-- TDFTLTIISSLQPEDFATYYC | QQYMT----------- ------------YPLT | FGGGT KVEIK |
| iPS:435 845 | 21-225_191G1 | VK1\|L1\|JK4 | DIQMTQSPSSLSA SVGDRVTITC | RAS--QGIS-- ----NYLA | WFQQKPGK APKSLIY | A-------- ASSLQS | GVPSKFSGSGSG-- TDFTLTIISSLQPEDFATYYC | QHYLT----------- ------------YPLT | FGGGT KVEIK |
| iPS:435 859 | 21-225_190E6 | VK1\|L1\|JK4 | DIQMTQSPSSLSA SVGDRVTITC | RTS--QGIG-- ----NYLA | WFQQKPGK APKSLLY | K-------- ASSLQS | GVPSKFSGSGSG-- TDFTLTIISSLQPEDFATYYC | QQYMT----------- ------------YPLT | FGGGT KVEIK |
| iPS:435 873 | 21-225_190G4 | VK1\|L1\|JK4 | DIQMTQSPSSLSA SVGDRVTITC | RAS--QDIG-- ----RYLA | WFQQKPGK APKSLIY | A-------- ASSLQS | GVPSKFSGSGSG-- TDFTLTIISSLQPENFATYYC | QQYST----------- ------------YPLT | FGGGT KVEIK |
| iPS:435 933 | 21-225_190F8 | VK1\|L1\|JK4 | DIQMTQSPSSLSA SVGDRVTITC | RTS--QGIG-- ----NYLA | WFQQKPGK APKSLLY | K-------- ASSLQS | GVPSKFSGSGSG-- TDFTLTIISSLQPEDFATYYC | QQYMT----------- ------------YPLT | FGGGT KVEIK |
| iPS:435 945 | 21-225_191A10 | VK1\|L1\|JK4 | DIQMTQSPSSLSA SVGDRVTITC | RAS--QGIG-- ----KYLA | WFQQKPGK APKSLIY | A-------- ASSLQS | GVPSKFSGSGYG-- TDFTLTIISSLQPENFAIYYC | QQYST----------- ------------YPLT | FGGGT KVEIK |

| | | | | | | | | | | |
|---|---|---|---|---|---|---|---|---|---|---|
| iPS:435 947 | 21-225_191E1 0 | VK1|L1/J K4 | | DIQMTQSPSSLSA SVGDRVTITC | RAS--QGIG-- ----KYLA | WFQQKPGK APKSLIY | A-------- ASSLQS | GVPSKFSCSGSG-- TDFTLTISSLQPENFATYYC | QQYST----------- ------------YPLT | FGGGT KVEIK |
| IPS:435 957 | 21-225_191G1 2 | VK1|L1/J K4 | | DIQMTQSPSSLSA SVGDRVTITC | RTS--QGIG-- ----NYLA | WFQQKPGK APKSLLY | K-------- ASSLQS | GVPSKFSGSGSG-- TDFTLTISSLQPEDFATYYC | QQYIT----------- ------------YPLT | FGGGS KVEIK |
| IPS:435 963 | 21-225_192D2 | VK1|L1/J K4 | | DIQMTQSPSSLSA SVGDRVTITC | RAS--QGIS-- ----NYLA | WFQQKPGK APKSLIY | A-------- ASSLQS | GVPSKFSGSGSG-- TDFTLTISSLQPEDFATYYC | QHYVT----------- ------------YPNT | FGGGT KVEIK |
| IPS:435 971 | 21-225_192D3 | VK1|L1/J K4 | | DIQMTQSPSSLSA SVGDRVTITC | RAS--QGIS-- ----NYLA | WFQQKPGK APKSLIY | A-------- ASSLQS | GVPSKFSGSGSG-- TDFTLTISSLQPEDFATYYC | LHYLT----------- ------------YPLT | FGGGT KVEIK |
| IPS:435 979 | 21-225_192H4 | VK1|L1/J K4 | | DIQMTQSPSSLSA SVGDRVTITC | RAS--QDIS-- ----NYLA | WFQQKPGK APKSLIS | A-------- ASSLQS | GVPSKFSGSRSG-- TDFTLTISSLQPEDFATYYG | LHYLN----------- ------------YPLT | FGGGT RVEIR |
| IPS:435 987 | 21-225_192G6 | VK1|L1/J K4 | | DIKMTQSPSSLSA SVGDRVTITC | RTS--QGIG-- ----NYLA | WFQQKPGK APKSLLY | K-------- ASSLQS | GVPSKFSGSGSG-- TDFTLTISSLQPEDFATYYC | QQYMT----------- ------------YPLT | FGGGT KVEIK |
| IPS:435 993 | 21-225_192C8 | VK1|L1/J K4 | | DIQMTQSPSSLSA SVGDRVTITC | RAS--QGIS-- ----NYLA | WFQQKPGK APKSLIY | A-------- ASSLQS | GVPSKFSGSGSG-- TDFTLTISSLQPEDFATYYC | QHYLT----------- ------------YPLT | FGGGT KVEIK |
| IPS:435 997 | 21-225_192G8 | VK1|L1/J K4 | | DIQMTQSPSSLSA SVGDRVTITC | RTS--QGIG-- ----NYLA | WFQQKPGK APKSLLY | K-------- ASSLQS | GVPSKFSGSGSG-- TDFTLTISSLQPEDFATYYC | QQYIT----------- ------------YPLT | FGGGT KVEIK |
| IPS:436 005 | 21-225_192H1 0 | VK1|L1/J K4 | | DIQMTQSPSSLSA SVGDRVTITC | RAS--QGIG-- ----KYLA | WFQQKPGK APKSLIY | A-------- ASSLQS | GVPSKFSGSGYG-- TDFTLTISSLQPENFATYYC | QQYST----------- ------------YPLT | FGGGT KVEIK |
| iPS:436 031 | 21-225_193C7 | VK1|L1/J K4 | | DIQMTQSPSSLSA SVGDRVTITC | RAS--QGIG-- ----KYLA | WFQQKPGK APKSLIY | A-------- ASSLQS | GVPSKFSGSGSG-- CDFTLTISSLQPEDFATYYC | QQYST----------- ------------YPLT | FGGGT KVEIK |
| IPS:436 045 | 21-225_193A1 0 | VK1|L1/J K4 | | DIQMTQSPSSLSA SVGDRVTITC | RAS--QGIS-- ----NYLA | WFQQKPGK APKSLIY | A-------- ASSLQS | GVPSKFSGSGSG-- CDFTLTISSLQPEDFATYFC | QHYLT----------- ------------YPLT | FGGGT KVEIK |
| IPS:436 054 | 21-225_194C1 | VK1|L1/J K4 | | DIQMTQSPSSLSA SVGDRVTITC | RAS--QGIS-- ----NYLA | WFQQKPGK APKSLIY | A-------- ASSLQS | GVPSKFSGSGSG-- CDFTLTISSLQPEDFATYYC | LHYLT----------- ------------YPLT | FGGGT KVEIK |
| IPS:436 076 | 21-225_194H1 1 | VK1|L1/J K4 | | DIQMTQSPSSLSA SVGDRVTITC | RAS--QGIS-- ----NYLA | WFQQKPGK APKSLIY | A-------- ASSLQS | GVPSKFSGSGSG-- TDFTLTISSLQPEDFATYYC | QHYLI----------- ------------YPLT | FGGGT KVEIK |
| IPS:436 086 | 21-225_191G1 0 | VK1|L1/J K4 | | DIQMTQSPSSLSA SVGDRVTITC | RTS--QGIG-- ----KYLA | WFQQKPGK APKSLLY | K-------- VSSLQS | GVPSKFSGSGSG-- TDFTLTISSLQPEDFATYYC | QQYMT----------- ------------YPLT | FGGGT KVEIK |
| IPS:436 090 | 21-225_195A9 | VK1|L1/J K4 | | DIQMTQSPSSLSA SVGDRVTITC | RAS--QGIS-- ----NYLA | WFQQKPGK APKSLIY | A-------- ASSLQS | GVPSKFSGSGSG-- TDFLTISSLQPEDFATYYC | QHYLT----------- ------------YPLT | FGGGT KVEIK |
| IPS:436 112 | 21-225_196C7 | VK1|L1/J K4 | | DIQMTQSPSSLSA SVGDRVTITC | RAN--QAIS-- ----NYLA | WFQQKPGK APKSLIY | A-------- ASSLQS | GVPSKFSGSGSG-- TDFTLTISSLQPEDFATYYC | QHYLT----------- ------------YPLT | FGGGT KVEIK |
| IPS:436 138 | 21-225_197F2 | VK1|L1/J K4 | | DIQMTQSPSSLSA SVGDRVTITC | RTS--QGIG-- ----NYLA | WFQQKPGK APKSLLY | K-------- TSSLQS | GVPSKFSGSGSG-- TDFTLTISSLQPEDFATYYF | QQYIT----------- ------------YPLT | FGGGT KVEIK |
| IPS:436 152 | 21-225_197B6 | VK1|L1/J K4 | | DIQMTQSPSSLSA SVGDRVTITC | RAS--QGIG-- ----KYLA | WFQQKPGK APKSLIY | G-------- ASSLQS | GVPSKFSGSGSG-- TDFTLTISSLQPENFATYYC | QQYST----------- ------------YPLT | FGGGT KVEIK |
| IPS:436 173 | 21-225_197G1 2 | VK1|L1/J K4 | | DIQMTQSPSSLSA SVGDRVTITC | RTS--QGIG-- ----NYLA | WFQQKPGK APKSLLY | K-------- TSSLQS | GFPSKFSGSGSG-- TDFTLTISSLQPEDFATYYF | QQYMT----------- ------------YPLT | FGGGT KVEIK |
| IPS:436 189 | 21-225_198B6 | VK1|L1/J K4 | | DIQMTQSPSSLSA SVGDRVTITC | RAS--QGIG-- ----NYLA | WFQQKPGK APKSLIY | A-------- ASSLQS | GVPSKFSGNRSG-- TDFTLTISSLQPEDFATYYC | QQYST----------- ------------YPLT | FGGGT KVEIK |

| ID | Clone | Germline | | CDR / FR 1 | CDR / FR 2 | FR | CDR | FR / CDR | CDR / FR | FR |
|---|---|---|---|---|---|---|---|---|---|---|
| iPS:436 201 | 21-225_199C5 | VK1\|L1\|JK4 | | DIQMTQSPSSLSA SVGDRVIITC | RAS--QGIS-- ----KYLA | WFQQKPGK APKSLIY | A-------- ASSLQS | GVPSKFSGSGSG-- TDFTLTISSLQPEDFAAYYC | QQYLT----------- ------------YPLT | FGGGT KVEIK |
| iPS:436 260 | 21-225_203H1 | VK1\|L1\|JK4 | | DIQMTQSPSSLSA SVGDRVIITC | RAS--QGIG-- ----NYLA | WFQQKPGK APKSLIY | V-------- ASRLQS | GVPSKFSGTGSG-- SDFTLTISSLQPDDFATYYC | QRYHT----------- ------------YPLT | FGGGT KVEIK |
| iPS:436 282 | 21-225_204G6 | VK1\|L1\|JK4 | | DIRMTQSPSSLSA SVGDRITITC | RTS--QGIG-- ----NYLA | WFQQKPGK APKSLIY | A-------- ASSLQS | GVPSRFSGSGSG-- TDFTLTISSLQPEDFATYYC | QHYLS----------- ------------YPLT | FGGGT KVEIK |
| iPS:436 284 | 21-225_204G8 | VK1\|L1\|JK4 | | DIQMTQSPSSLSA SVGDRVTITC | RAS--QGIS-- ----NHLA | WFQQKPGK APKSLIF | A-------- ASSLQS | GVPSQFSGSGSG-- TEFTLTISSLQPEDFATYYC | QQYSN----------- ------------YPVT | FGGGT KVEIK |
| iPS:436 292 | 21-225_205H3 | VK1\|L1\|JK4 | | DIPMTQSPSSLSA SVGDRVTITC | RAS--QAIS-- ----NHLA | WFQLKPGK APKSLIY | A-------- ASSLQS | GVPSKFSGSGSG-- SDFTLTISSLQPEDFATYYC | QQYSN----------- ------------YPLT | FGGGT KVEIK |
| iPS:436 296 | 21-225_205F5 | VK1\|L1\|JK4 | | DIQMTQSPSSLSA SVGDRVTITC | RAS--QGIG-- ----NYLA | WFQQKPGK APKSLIY | G-------- VSSLQS | GVPSKFSGSGSG-- TDFTLTISSLQPEDFATYTC | QQYSN----------- ------------YPLT | FGGGT KVDIR |
| iPS:436 324 | 21-225_207G6 | VK1\|L1\|JK4 | | DIQMTQSPSSLSA SVGDRVTITC | RAS--QGIR-- ----NYLA | WLQQKPGK APKSLIH | A-------- ASSLQS | GVPSRFSGNRSG-- TDFTLTISSLQPEDFATYYC | QQYSN----------- ------------YPLT | FGGGT KVEIK |
| iPS:436 364 | 21-225_211A1 1 | VK1\|L1\|JK4 | | DIQMTQSPSSLSA SVGDRVTITC | RAS--QGIG-- ----NYLA | WFQQKPGK APRSLIY | D-------- ASSLES | GVPSKFSGSRSG-- TDFTLTIGSLQPEDFATYYC | QHYMT----------- ------------YPLT | FGAGT KVEIK |
| iPS:436 366 | 21-225_211A3 | VK1\|L1\|JK4 | | DIRMTQSPSSLSA SVGDRVTITC | RAS--QAIG-- ----KHLA | WFQQRPGK APKSLIY | A-------- ASRLQS | GVPSKFSGSGSG-- TDFTLTISSLQPEDLATYYC | QQYSN----------- ------------YPLT | FGGGT KVEIK |
| iPS:436 372 | 21-225_211A8 | VK1\|L1\|JK4 | | DIEMTQSPSSLSA FVGDRVTITC | RAS--QGIS-- ----RYLA | WVQQKPGK APKSLIY | A-------- ASSLQS | GVSSRFSGSGSG-- TDFTLTISSLQPEDFATYYC | LRYDT----------- ------------YPLI | FGGGT KVEIK |
| iPS:436 376 | 21-225_212E6 | VK1\|L1\|JK4 | | DIQMTQSPSSLSA SVGDRVTITC | RAS--QGIS-- ----NHLA | WFQQKPGQ APKSLIY | A-------- ASSLQS | GVPSKFSGSRSG-- TDFTLTISSLQPEDFATYYC | QQYVT----------- ------------YPLT | FGGGT KVEIK |
| iPS:436 378 | 21-225_212D7 | VK1\|L1\|JK4 | | DIQMTQSPSSLSA SVGDRVTITC | RAS--QGIS-- ----NHLA | WFQQKPGK APKSLIH | A-------- ASSLQS | GVPSKFSGNRSG-- TDFTLTINNLQPEDFVTYYC | QQYSN----------- ------------YPLT | FGGGT KVEIK |
| iPS:436 380 | 21-225_212H9 | VK1\|L1\|JK4 | | DIEMTQSPSSLSA SVGDRVTITC | RAS--QGIS-- ----SYLA | WLQQKPGK APKSLIY | A-------- ASSLQS | GVPSRFSGSGSG-- TDFTLTISSLQPEDFATYYC | LRYDT----------- ------------YPLT | FGGGT KVEIK |
| iPS:436 384 | 21-225_212F10 | VK1\|L1\|JK4 | | DIQMTQSPSSLSA SVGDRVTITC | RAS--QGIS-- ----NYLD | WFQQKPGK APKSLIY | S-------- ASNLQS | GVPSKFSGSGSG-- TDFTLTISSLQPEDFATYYC | QHYSN----------- ------------YPLT | FGGGT KVEIK |
| iPS:436 388 | 21-225_212H1 1 | VK1\|L1\|JK4 | | DIRMTQSPSSLSA SVGDRVTITC | RAS--QAIG-- ----KHLA | WFQQRPGK APKSLIY | A-------- ASRLQS | GVPSKFSGSGSG-- TDFTLTISSLQPEDLATYYC | QHYSN----------- ------------YPLT | FVGGT KVEIT |
| iPS:436 390 | 21-225_213D2 | VK1\|L1\|JK4 | | DIQMTQSPSSLSA SVGDRVTITC | RAS--QGIS-- ----NHLA | WFQQKPGK APKSLIH | A-------- ASSLQS | GVPSKFSGNRSG-- TDFTLTINNLQPEDFVTYYC | HQYSN----------- ------------YPLT | FGGGT KVEIK |
| iPS:436 394 | 21-225_213C4 | VK1\|L1\|JK4 | | DIEMTQSPSSLSA SVGDRVTITC | RAS--QAIR-- ----NYLA | WCQQKPGK APKTLIY | A-------- ASSLQS | GVPSKFSGSGSG-- TDFTLTISSLQPEDFATYYC | QQYSN----------- ------------YPLT | FGGGT KVEIK |
| iPS:436 396 | 21-225_213E5 | VK1\|L1\|JK4 | | DIRMTQSPSSLSA SVGDRVTITC | RAS--QAIG-- ----KHLA | WFQQRPGK APKSLIY | A-------- ASRLQS | GVPSKFSGSGSG-- TDFTLTISSLQPEDLATYYC | QHYSN----------- ------------YPLT | FGGGT KVEIK |
| iPS:436 398 | 21-225_213B8 | VK1\|L1\|JK4 | | DIQMTQSPSSLSA SVGDRVTITC | RAS--QGIS-- ----NHLA | WFQQKPGK APKSLIY | A-------- ASSLQS | GVPSKFSGSGSG-- TDFTLTISSLQPEDFATYYC | QQYSN----------- ------------YPLT | FGGGT KVEIK |
| iPS:436 410 | 21-225_212E1 0 | VK1\|L1\|JK4 | | DIQMTQSPSSLSA SVGDRVTITC | RAS--QGIS-- ----NHLA | WFQQKPGK APKSLIY | A-------- ASSLQS | GVPSKFSGSGSG-- TDFTLTISSLQPEDFATYYC | QQYSN----------- ------------YPLT | FGGGT KVEIK |
| iPS:436 420 | 21-225_215B5 | VK1\|L1\|JK4 | | DIQMTQSPSSLSA SVGDRVTITC | RAS--QGIS-- ----NHLA | WFQQKPGK APKSLIH | A-------- ASSLQS | GVPSKFSGNRSG-- TDFTLTINNLQPEDFVTYYC | QQYIN----------- ------------YPLT | FGGGT KVEIK |
| iPS:436 422 | 21-225_215D6 | VK1\|L1\|JK4 | | DIQMTQSPSSLSA SVGDRVTITC | RAS--QGIS-- ----NHLA | WFQQKPGK APKSLIY | A-------- ASSLHS | GVPSKFSGSRSG-- TDFTLTIISSLQPEDFATYYC | QQYVT----------- ------------YPLT | FGGGT KVEIK |

| | | | | | | | | | |
|---|---|---|---|---|---|---|---|---|---|
| iPS:436 430 | 21-225_215A12 | VK1\|L1/JK4 | DIQMTQSPSSLSA SVGDRITITC | RTS--QDIG-- ----NYLA | WFQQKPGK APKSLIY | A-------- ASSLQS | GVPSKFSGSRSG-- TDFTLTISSLQSEDFATYYC | QQYVT----------- ------------YPLT | FGGGT KVEIK |
| iPS:436 452 | 21-225_217G5 | VK1\|L1/JK4 | DILMTQSPSSLSA SVGDRVTITC | RAS--QGIG-- ----NYLA | WFQQRPGK APKSLIY | A-------- ASSLQS | GVPSKFSGTRSG-- TDFTLTISSLQPDDFATYYC | QQYVN----------- ------------YPLT | FGGGT KVEIN |
| iPS:436 454 | 21-225_217B10 | VK1\|L1/JK4 | DIRMTQSPSSLSA SVGDRVTITC | RAS--QAIG-- ----KHLA | WFQQRPGK APKSLIY | A-------- ASRLQS | GVPSKFSGSGSG-- TDFTLTISSVQPEDLATYYR | QHTSK----------- ------------SPVQ | LVGGT KVEIT |
| iPS:436 464 | 21-225_219H1 | VK1\|L1/JK4 | DIQMTQSPSSQSA SVGDRVTITC | RAS--QGIS-- ----NYLD | WFQQKPGK APKSLIY | S-------- ASNLQS | GVPSKFSGSRSG-- IDFTLTISSLQPEDFATYYC | QHYSN----------- ------------YPLT | FGGGT KVEIK |
| iPS:436 490 | 21-225_221F6 | VK1\|L1/JK4 | DIQMTQSPSSLSG SVGDRVTITC | RAS--QGIS-- ----NYLA | WFQQKPGK APKSLIY | A-------- ASNLQS | GVPSKFSGSRSG-- IDFTLTISSLQPEDFATYYC | QQYMT----------- ------------YPLT | FGGGT RVEIK |
| iPS:436 502 | 21-225_222A11 | VK1\|L1/JK4 | DIQMTQSPSSLSA SVGDRVTITC | RAS--QGIS-- ----NYLA | WFQQKPGK APKSLIY | A-------- ASSLQS | GVPSKFSGSGSG-- IDFTLTISSLQPEDFATYYC | LYYLN----------- ------------YPLT | FGGGT KVEIK |
| iPS:436 514 | 21-225_222D10 | VK1\|L1/JK4 | DIQMTQSPSSLSA SVGDRVTITC | RAS--QGIS-- ----NYLA | WFQQKPGK APKSLIY | A-------- ASSLQS | GVPSKFSGSGSG-- TDFTLTISSLQPEDFATYYC | LHYLN----------- ------------YPLT | FGGGT RVEIK |
| iPS:436 522 | 21-225_223H10 | VK1\|L1/JK4 | DIQMTQSPSSLSA SVGDRVTITC | RAS--QGIS-- ----NYLA | WFQQKPGK APKSLIY | A-------- ASNLQS | GVPSKFSGSGSG-- TDFTLTISSLQPEDFATYYC | LHYLN----------- ------------YPLT | FGGGT KVEIK |
| iPS:437 258 | 21-225_153F9 | VK1\|L1/JK4 | DIQMTQSPSSLSA SVGDRVTITC | RAS--QGIS-- ----NYLA | WFQQKPGK APKSLIS | A-------- ASSLQS | GVPSKFSGSGSG-- TDFTLTISSLQPEDFATYYC | QQYNS----------- ------------YPLS | FGGGT KVEIK |
| iPS:437 260 | 21-225_170D1 | VK1\|L1/JK4 | DIQMTQSPSSLAA SVGDRVTITC | RAS--QDIS-- ----NYLA | WFQQKPGK APKSLIY | A-------- ASSLQS | GVPSRFSGSGSG-- TDFTLTISSLQPEDFATYYC | QQCDS----------- ------------FPLT | FGGGT KVEIK |
| iPS:437 264 | 21-225_171H12 | VK1\|L1/JK4 | DIQMTQSPSSLSA SVGDRVTITC | RAS--QDIS-- ----NYLA | WFQQKPGK APKSLIY | S-------- ASSLQS | GVPSKFSGSGSG-- TDFTLTISSLQPEDFATYYC | QQSDS----------- ------------YPLT | FGGGT KVEIK |
| iPS:437 266 | 21-225_177A5 | VK1\|L1/JK4 | DIQMTQSPSSLSA SVGDRVTITC | RAS--QDIS-- ----NYLA | WFQQKPGK APKSLIY | S-------- ASSLQS | GVPSKFSGSGSG-- TDFTLTISSLQPEDFATYYC | QQSDS----------- ------------YPLT | FGGGT KVEIK |
| iPS:437 270 | 21-225_178H4 | VK1\|L1/JK4 | DIQMTQSPSSLSA SVGDRVTITC | RAS--QDIS-- ----NYLA | WFQQKPGK APKSLIF | S-------- ASSLQS | GVPSKFSGSGSG-- TDFTLTISSLQPEDFATYYC | QQSNS----------- ------------YPLT | FGGGT KVEIK |
| iPS:438 664 | 21-225_216G1 | VK1\|L1/JK4 | DIEMIQSPSSLSA SVGDRVTITC | RAS--QGIS-- ----SYLA | WLQQKPGK APKSLIY | A-------- ASSLQS | GVPSRFSGSGSG-- TDFTLTISSLQPEDFATYYC | LRYDT----------- ------------YPLT | FGGGT KVEIK |
| iPS:451 120 | 21-225_197D3 | VK1\|L1/JK4 | DIQMTQSPSSLSA SVGDRVTITC | RAS--QGIR-- ----NYLA | WFQQKPGK APKSLIY | A------- ASSLQS | GVPSKFSGSGSG-- TDFTLTISSLQPEDFATYYC | QHYLT----------- ------------YPLT | FGGGT KVEIK |
| iPS:392 682 | 21-225_16A12 | VK1\|L1/JK4 | DIQMTQSPSSLSA SVGDRVTITC | RAS--QAIN-- ----TYLA | WFQQKPGK APKSLIS | A-------- ASSLQS | GVPSKFSGSGSG-- TEFTLTISSLQPEDFATYYC | QQYYS----------- ------------YPLT | FGGGT KVEIK |
| iPS:392 856 | 21-225_22A2 | VK1\|L1/JK4 | DIQMTQSPSSLSA SVGDRVTITC | RAS--QDIS-- ----NYLA | WVQQKPGK APKSLIY | A------- ASSLQS | GVPSKFSGSGSG-- TDFTLTISSLQPEDFASYYC | QQYNS----------- ------------FPLT | FGGGT KVEIK |
| iPS:392 966 | 21-225_32G3 | VK1\|L1/JK4 | DIQMTQSPTSLSA SVGDRVTITC | RAS--QAIS-- ----NYLA | WFQQKPGK APKSLIY | D-------- TSSLQS | GVPSKFSGSGSG-- TDFTLTISTLQPEDFATYYC | QQYHS----------- ------------YPLT | FGGGT KVEIK |
| iPS:393 952 | 21-225_1F1 | VK1\|L1/JK4 | DIQMTQSPSSLSA SVGDRVTITC | RAS--QGIN-- ----NYLA | WFQQKSGK APKSLIS | V-------- ASSLQT | GVPSKFSGSGSG-- TDFTLTISSLQPEDFATYYC | QQYNS----------- ------------YPLT | FGGGT KVEIK |
| iPS:393 988 | 21-225_7F10 | VK1\|L1/JK4 | DIQMTQSPSALSA SVGDRVTITC | RAS--QDIR-- ----NYLA | WFQQKPGK APKSLIS | V-------- ASSLQS | GVPSRFSGSGSG-- TDFTLTISSLQPEDFATYYC | QQYNS----------- ------------YPLT | FGGGT KVEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:402 233 | 21-225_16D10 | VK1\|L1/JK4 | | DIQMTQSPSSLSA SVGDRVTITC | RAS--QDIS-- ----NYLA | WFQQKPGK APKSLIY | A-------- TPSLQS | GVPSKFSGSGSG-- TEFTLTISSLQPEDFATYYC | QQYNS----------- -----------YPLT | FGGGT KVEIK |
| iPS:402 237 | 21-225_23D11 | VK1\|L1/JK4 | | DIQMTQSPSSLSA SVGDRVTITC | RAS--QGIA-- ----NYLA | WFQQRPGK APKSLIS | A-------- ASSLQS | GVPSKFSGSGSG-- TEFTLTISSLQPEDFATYYC | QQYHS----------- -----------YPLT | FGGGS KVEIK |
| | Germline | | | K_FR1 | K_CDR1 | K_FR2 | K_CDR2 | K_FR3 | K_CDR3 | K_FR4 |
| | VK1\|O18/JK5 | | 32158 | DIQMTQSPSSLSA SVGDRVTITC | QAS--QDIS-- ----NYLN | WYQQKPGK APKLLIY | D-------- ASNLET | GVPSRFSGSGSG TDFTFTISSLQPEDIATYYC | QQYDN----------- -----------LPIT | FGQGT RLEIK |
| iPS:434 171 | 21-225_50G4 | VK1\|O18/JK5 | | DIQMTQSPTSLSA SVGDRVTITC | QAS--QDIT-- ----NFLN | WYQQKPGK APKLLIY | D-------- ASTLET | GVPSRFSGSGSG-- TDFTFTISSLQPEDIATYYC | QQYD------------ -----------NLIT | FGQGT RLEIK |
| iPS:435 565 | 21-225_159C4 | VK1\|O18/JK5 | | DIQMTQSPSSLSA SVGDRVTITC | QAS--QDIS-- ----DYLN | WYQQKPGK APKLLIY | D-------- ASTLET | GVPSRFSGSGSG-- TDFTFTISSLQPEDIATYFC | QQYDN----------- -----------LPIT | FGQGT RLEIK |
| iPS:435 607 | 21-225_161G4 | VK1\|O18/JK5 | | DIQMTQSPSSLSA SVGDRVTITC | QAS--QDIY-- ----NYLN | WYQQKPGK APKLLIY | D-------- ASTLET | GVPSRFSGSGSG-- TDFTFAISSLQPEDIATYYC | QQYDI----------- -----------LPIT | FGQGT RLEIK |
| iPS:437 302 | 21-225_225B11 | VK1\|O18/JK5 | | DIQMTQSPSSLSA SIGDRVTITC | QAS--QDIF-- ----NYLN | WYQQKPGK APKLLIY | D-------- ASTLET | GVPSRFSGSGSG-- TDFTFTISSLQPEDIATYYC | QQYDN----------- -----------LPIT | FGQGT RLEIK |
| iPS:392 868 | 21-225_24D6 | VK1\|O18/JK5 | | DIQMTQSPSSLSA SVGDRVTITC | QAS--QDIN-- ----NYLN | WYQQKPGK ALKLLIY | D-------- ASDLET | GVPSRFSGSGSG-- TDFTFTISSLQPEDIAIYYC | QQYEN----------- -----------LPIT | FGQGT RLEIK |
| iPS:393 020 | 21-225_30E2 | VK1\|O18/JK5 | | DIQMTQSPHSLSA SVGDRVTITC | QAS--QYIS-- ----NYLN | WYQQKSGK APKLLIY | D-------- ASDLET | GVPSRFSGSGSG-- TDFTFTISSLQPEDLATYYC | QQYDN----------- -----------LPIT | FGQGT RLEIK |
| iPS:393 138 | 21-225_35E3 | VK1\|O18/JK5 | | DIQMTQSPSSLSA SVGDRVTITC | QAS--QDIF-- ----NYLN | WYQQKPGK APNLLIY | D-------- ASNLET | GVPSRFSGSGSG-- TDFTFTISSLQPEDIATYFC | QQYDN----------- -----------LPIT | FGQGT RLDIR |
| iPS:393 892 | 21-225_6G7 | VK1\|O18/JK5 | | DIQMTQSPSSRSA SVGDRVTITC | QAS--QDIS-- ----NYLN | WCQQKPGK ALKLLIY | D-------- ASTLET | GVPSRFSGSGSG-- TDFTFTISSVQPEDIATYYC | QQYDN----------- -----------VPIT | FGQGT RLEIK |
| iPS:393 910 | 21-225_15F10 | VK1\|O18/JK5 | | DIQMTQSPSSLSA SVGDRVTITC | QAN--QDIT-- ----NFLN | WYQLKPGK APNLLIS | D-------- ASNLET | GVPSRFSGSGSG-- TDFTFTISSLQPEDVATYYC | QQYDN----------- -----------LPIT | FGQGT RLEIK |
| iPS:393 912 | 21-225_16F6 | VK1\|O18/JK5 | | DIQMTQSPSSLSA SVGDRVTITC | QAN--QDIN-- ----NFLN | WYQLKPGK APNLLIS | D-------- ASNLET | GVPSRFSGSGSG-- TDFTFTISSLQPEDVATYYC | QQYDN----------- -----------LPIT | FGQGT RLEIK |
| iPS:394 000 | 21-225_11A2 | VK1\|O18/JK5 | | DIQMTQSPSSLSA SVGDRVTITC | QAS--QDIS-- ----NYLN | WYQQKPGK APKLLIY | D-------- ASNLET | GVPSRFSGSGSG-- TDFTFTISSLQPEDVATYYC | QQYDN----------- -----------LPIT | FGQGT RLDIK |
| iPS:394 004 | 21-225_13A1 | VK1\|O18/JK5 | | DIQMTQSPSSLSA SVGDRVTITC | QAS--QDIN-- ----NYLN | WYQQKLGT APKLLIY | D-------- GSNLET | GVPSRFSGSGSG-- TDFTFTISSLQPEDIATYYC | QQYEN----------- -----------LPIT | FGQGT RLEIK |
| iPS:394 006 | 21-225_15C2 | VK1\|O18/JK5 | | DIQMTQSPSSLSA SVGDRVTITC | QAS--QDIT-- ----NYLN | WYQQKPGK APNLLIY | D-------- ASNLET | GVPSRFSGSGSG-- TDFTFTISSLQPEDIATYYC | QQYDN----------- -----------LPIT | FAQGT RLEIK |
| iPS:394 029 | 21-225_1B12 | VK1\|O18/JK5 | | DIQMTQSPSSLSA SVGDRVTITC | QAS--QDIN-- ----NYLN | WYQQKPGK APKLLIY | D-------- ASNLET | GVPSRFSGSGSG-- TDFTFTISSLQPEDITTYYC | QQYEN----------- -----------LPIT | FGQGT RLEIK |
| iPS:394 047 | 21-225_5E6 | VK1\|O18/JK5 | | DIQMTQSPSSLSA SVGDRVTITC | QAS--QDIN-- ----NYLN | WCQQKPGK APKLLIY | D-------- ASNLET | GVPSRFSGSGSG-- TDFTFTISSLQPEDIATYYC | QQYDN----------- -----------LPIT | FGQGT RLEIK |
| iPS:394 081 | 21-225_16B3 | VK1\|O18/JK5 | | DIQMTQSPSSLSA SVGDRVTITC | QAS--QDIN-- ----NYLN | WYQQKPGR APKLLIY | D-------- ASNLET | GVPSRFSGSGSG-- TDFTFTISSLQPEDIATYYC | QQFDN----------- -----------LPIT | FGQGT RLEIK |
| | Germline | | | K_FR1 | K_CDR1 | K_FR2 | K_CDR2 | K_FR3 | K_CDR3 | K_FR4 |
| | VK3\|L2/JK3 | | 32159 | EIVMTQSPATLSV SPGERATLSC | RAS--QSVS-- ----SNLA | WYQQKPGQ APRLLIY | G-------- ASTRAT | GIPARFSGSGSG-- TEFTLTISSLQSEDFAVYYC | QQYNN----------- -----------WPFT | FGPGT KVDIK |
| iPS:434 219 | 21-225_60E9 | VK3\|L2/JK3 | | EIVMTQSPATLSV SPGERATLSC | RAS--QSVS-- ----SSLA | WYRQKPGQ APRLLIY | G-------- ASTRAT | GIPARFSGSGSG-- TEFTLTISSLQSEDFAVYCC | QQYNN----------- -----------WPFT | FGPGT KIDIK |
| iPS:434 279 | 21-225_57F7 | VK3\|L2/JK3 | | EIVMTQSPATLSV FPGERATLSC | RAS--QSVS-- ----SDLA | WYQQKPGQ APRLLIY | G-------- ASTRAT | GMPARFSGGGSG-- TEFTLTISSLQSEHFAVYYC | QQYSN----------- -----------WPFT | FGPGT KVDIK |

| | | Germline | | K_FR1 | K_CDR1 | K_FR2 | K_CDR2 | K_FR3 | K_CDR3 | K_FR4 |
|---|---|---|---|---|---|---|---|---|---|---|
| iPS:434 289 | 21-225_57H12 | VK3\|L2\|JK3 | | EIVMTQSPATLSV SPGERATLSC | RAS--QSVS-- ----SDLA | WYQQKPGQ APRLLIY | A-------- ASTRAT | GIPARFSGSGSG-- TEFTLTISSLQSEDFAVYYC | QQYDN----------- ------------WPFT | FGPGT KVDNK |
| iPS:434 291 | 21-225_58A4 | VK3\|L2\|JK3 | | EIVMTQSPATLSV SPGERATLSC | RAS--QSVS-- ----SDLA | WYQQRPGQ APRLLIY | A-------- ASTRAT | GIPARFSGSGSG-- TEFTLTISSLQSEDFAVYYC | QQFNN----------- ------------WPFT | FGPGT KVDIK |
| iPS:434 297 | 21-225_58A10 | VK3\|L2\|JK3 | | EIVMTQSPATLSV CPGERATLSC | RAS--QSVS-- ----SSLA | WYQQKPGQ APRLLIY | G-------- ASTRAT | GIPARFSGSGSG-- TEFTLTISSLQSEDFAVYYC | QQYNN----------- ------------WPFT | FGPGT KVDIK |
| iPS:434 301 | 21-225_58F11 | VK3\|L2\|JK3 | | EIVMTQSPATLSV SPGERATLSC | RAS--QSVS-- ----SDLV | WYQQKPGQ APRLLIY | G-------- VSTRAT | GIPARFSGSGSG-- TEFTLTISSLQSEDFAVYYC | QQYNN----------- ------------WPFT | FGPGT KVDIK |
| iPS:437 228 | 21-225_60C11 | VK3\|L2\|JK3 | | EIVMTQSPATLSV SPGERATLSC | RAS--QSVS-- ----NDLA | WYQQKPGQ APRLLIY | G-------- ASTRAT | GIPARFSGGGSG-- TEFTLTISALQSEHFAVYYC | QQYSN----------- ------------WPFT | FGPGT KVDIK |
| | | Germline | | K_FR1 | K_CDR1 | K_FR2 | K_CDR2 | K_FR3 | K_CDR3 | K_FR4 |
| | VK2\|A19/JK5 | | 32160 | DIVMTQSPLSLPV TPGEPASISC | RSS-- QSLLHSN- GYNYLD | WYLQKPGQ SPQLLIY | L-------- GSNRAS | GVPDRFSGSGSG-- TDFTLKISRVEAEDVGVYYC | MQALQ----------- -----------TPIT | FGQGT RLEIK |
| iPS:434 243 | 21-225_62C1 | VK2\|A19/JK5 | | DIVMTQSPLSLPV TPGEPASISC | RSS-- QSLLHSN- GYNYLD | WYLQKPGQ SPQLLIY | L-------- VSNRAS | GVPDRFSGSGSG-- TDFTLKISRVGAEDVGVYFC | LQALQ----------- -----------TPLT | FGQGT RLEIK |
| iPS:436 648 | 21-225_227F11 | VK2\|A19/JK5 | | DVVMTQSPLSLPV TPGEPASISC | WSS-- QSLLHSN- GYNYLD | WYLQKPGQ SPQVLIY | L-------- GSNRAS | GVPDRFSGSGSG-- TDFTLKISRVEAEDVGVYYC | MQALQ----------- -----------TPLT | FGQGT RLEIK |
| iPS:394 061 | 21-225_12D2 | VK2\|A19/JK5 | | DIVMTQSPLSLPV TPGEPASISC | RSS-- QSLLHSN- GYNYLD | WYLQKPGQ SPQVLIY | L-------- GSNRAS | GVPDRFSGSGSG-- TDFTLKISRVEAEDVGVYYC | MQALQ----------- -----------TPIT | FGQGT RLEIK |
| iPS:394 071 | 21-225_10C7 | VK2\|A19/JK5 | | DIVMTQSPLSLPV TPGEPASISC | RSS-- QSLLHSK- GYNYLD | WYLQKPGQ SPQVLIY | L-------- GSNRAS | GVPDRFSGSGSG-- TDFTLKISRVEAEDVGVYYC | MQALQ----------- -----------TPLT | FGQGT RLEIK |
| | | Germline | | K_FR1 | K_CDR1 | K_FR2 | K_CDR2 | K_FR3 | K_CDR3 | K_FR4 |
| | VK1\|L5/JK4 | | 32161 | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQANS----------- -----------FPLT | FGGGT KVEIK |
| iPS:434 259 | 21-225_62G7 | VK1\|L5/JK4 | | DIQMTQSPSSVSA SVGDRVTITC | RAS--QDIS-- ----SWLA | WYQQNPGK APKLLIY | A-------- ASSLQS | GVPSRFSGSGSG-- TNFTLTISSLQPEDFATYYC | QQTNS----------- -----------FPLT | FGGGT KVEIK |
| iPS:434 333 | 21-225_63C9 | VK1\|L5/JK4 | | DIQMTQSPSSVSA SVGDRVTITC | RAS--QGIS-- ----SWLA | WYQQKPGK APNLLIY | A-------- ASSLQS | GVPSRFSGSGSG-- TDFTLTISGLQPEDFATYFC | QQINS----------- -----------FPLT | FGGGT KVAIK |
| iPS:434 347 | 21-225_64H10 | VK1\|L5/JK4 | | DIQMTQSPSSVSA SVGDRVTITC | RAS--QGIS-- ----SWLA | WYQQKPGK ALKLLIY | A-------- ASSLQS | GVPSRFSGSGSG-- TDFTLTISSLQPEDFATYYC | QQINS----------- -----------FPLT | FGGGT KVEIK |
| iPS:434 359 | 21-225_65G3 | VK1\|L5/JK4 | | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATFYC | QQVNS----------- -----------FPLT | FGGGT KVEIK |
| iPS:434 369 | 21-225_66B1 | VK1\|L5/JK4 | | DIQMTQSPSSVSA SVGDRVTITC | RAS--QGIS-- ----SWLA | WYQQKPGK ALKLLIY | A-------- ASSLQS | GVPSRFSGSGSG-- TDFTLTISSLQPEDFATYYC | QQTNS----------- -----------FPLT | FGGGT KVEIK |
| iPS:434 373 | 21-225_66A7 | VK1\|L5/JK4 | | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQINS----------- -----------FPLT | FGGGT KVEIK |
| iPS:434 397 | 21-225_67H4 | VK1\|L5/JK4 | | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQTNS----------- -----------FPLT | FGGGT KVEIK |
| iPS:434 427 | 21-225_70D6 | VK1\|L5/JK4 | | DIQMTQSPSSVSA SVGDRVTITC | RAS--QGIS-- ----KWLA | WYQQNPGK ALKLLIF | A-------- ASSLQS | GVPSRFSGSGSG-- TDFTLTISSLQPEDFANYYC | QQTNS----------- -----------FPLT | FGGGT KVEIK |
| iPS:434 435 | 21-225_70G9 | VK1\|L5/JK4 | | DIQMTQSPSSVSA SVGDRVTITC | RAS--QDIS-- ----SWLA | WYQQNPGK APKLLIY | A-------- ASSLQS | GVPSRFSGSGSG-- TDFTLTISSLQPEDFATYYC | QQTNS----------- -----------FPLT | FGGGT KVEIK |

| iPS ID | 21-225 ID | V/J | No. | K_FR1 | K_CDR1 | K_FR2 | K_CDR2 | K_FR3 | K_CDR3 | K_FR4 |
|---|---|---|---|---|---|---|---|---|---|---|
| iPS:434 437 | 21-225_70A12 | VK1\|L5/JK4 | | DIQMTQSPSSVSA<br>SVGDRVTITC | RAS--QGIS--<br>----SWLA | WYQQKPGK<br>ALKLLIY | A--------<br>ASSLQS | GVPSRFSGSGSG--<br>TDFTLTISSVQPEDFATYYC | QQINS-----------<br>------------FPLT | FGGGT<br>KVEIK |
| iPS:434 451 | 21-225_71B7 | VK1\|L5/JK4 | | DIQMTQSPSSVSA<br>SVGDRVTITC | RAS--QGIS--<br>----SWLA | WYQQNPGE<br>APKLLIY | A--------<br>ASSLQG | GVPSRFSGSGSG--<br>TDFTLTISSLQPEDFANYYC | QQTNS-----------<br>------------FPLT | FGGGT<br>KVEIK |
| iPS:434 459 | 21-225_71A7 | VK1\|L5/JK4 | | DIQMTQSPSSVSA<br>SVGDRVTITC | RAS--QGIS--<br>----SWLA | WYQQKPGK<br>APKLLIY | A--------<br>ASSLQS | GVPSRFSGSGSG--<br>TDFTLTISSLQPEDFATYYC | QQVNS-----------<br>------------FPLT | FGGGT<br>KVELK |
| iPS:434 461 | 21-225_73A3 | VK1\|L5/JK4 | | DIQMTQSPSSVSA<br>SIGDRVTITC | RAS--QGIS--<br>----NWLA | WYQQKPGK<br>APKLLIY | A--------<br>ASSLQS | GVPSRFSGSGSG--<br>TDFTLTISSLQPEDFATYYC | QQVNS-----------<br>------------FPLT | FGGGT<br>KVEIK |
| iPS:435 479 | 21-225_154E9 | VK1\|L5/JK4 | | DIQMTLSPSSVYA<br>SVGDRVTITC | RAS--QDIS--<br>----NWLA | WYQQRPGK<br>APKVLIY | A--------<br>ASSLQS | GVPSRFSGSGSG--<br>TDFTLTISSLQPEDFATYYC | QQGNS-----------<br>------------FPLT | FGGGT<br>KVDIK |
| iPS:437 232 | 21-225_63E1 | VK1\|L5/JK4 | | DIQMTQSPSSVYA<br>SVGDRVTITC | RAS--QGIS--<br>----SYLA | WYQQKPGK<br>APKLLIY | A--------<br>ASSLQS | GVPSRFSGSGSG--<br>TDFTLTISSLQPEDFATYYC | QQANS-----------<br>------------FPLT | FGGGT<br>KVEIK |
| iPS:437 326 | 21-225_75C10 | VK1\|L5/JK4 | | DIQMTQSPSSVSA<br>SVGDRVIITC | RAS--QGIS--<br>----IWLA | WYQQKPGK<br>APKLLIY | A--------<br>ASSLQS | GVPLRFSGSGSG--<br>TDFTLTISSLQPEDFATYYC | QQAKS-----------<br>------------FPLT | FGGGT<br>KVEIK |
| Germline | | | K_FR1 | K_CDR1 | K_FR2 | K_CDR2 | K_FR3 | K_CDR3 | K_FR4 | |
| VK1\|O12/JK2 | | 32162 | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFATYYC | QQSYS-----------<br>------------TPYT | FGQGT<br>KLEIK | |
| iPS:434 309 | 21-225_59B5 | VK1\|O12/JK2 | | DIQMTQSPSSLSA<br>SVGDRVTITC | RAS--QSII--<br>----SYLN | WYQQKPGK<br>APKLLIY | G--------<br>ASSLQS | GVPSRFSGSGSG--<br>TDFTLTISSLQPEDFATYYC | QQSYS-----------<br>-----------TPMFS | FGQGT<br>KLEIK |
| iPS:392 874 | 21-225_21D2 | VK1\|O12/JK2 | | DIQMTQSPSSLFA<br>SVGDRVTITC | RAS--QSIS--<br>----DYLN | WYQQKPGR<br>APKLLIY | D--------<br>TSSLQS | GVPSRFSGSGSG--<br>TDFTLTINSLQPEDFATYYC | QQTYNI----------<br>------------LPERS | FGRGT<br>KLEIK |
| iPS:393 940 | 21-225_16B2 | VK1\|O12/JK2 | | GVQMTQSPSSLSA<br>SVGDRVTITC | RAS--QSIS--<br>----GYLN | WYQQKPGK<br>APKLLIY | A--------<br>ASSLQS | GVPSRFSGGGSG--<br>TDFTLTISSLQPEDFATYYC | QQTYNI----------<br>------------PPERS | FGQGT<br>KLEIK |
| iPS:393 956 | 21-225_4D7 | VK1\|O12/JK2 | | DIQMTQSPSSLSA<br>SVGDRVTITC | RAS--QSIS--<br>----DYLN | WYQQKPGK<br>APKLIF | D--------<br>TTSLQS | GVPSRFSGSGSG--<br>TDFTLTINSLQPEDFATYYC | QQTYNI----------<br>------------PPERS | FGQGT<br>KLEIK |
| iPS:398 476 | 21-225_17C1 | VK1\|O12/JK2 | | DIQMTQSPSSLSA<br>SVGDRVTITC | RAS--QNIN--<br>----DYLN | WYQQKPGK<br>APKLLIY | A--------<br>ASNLQS | GVPARFSGSRSG--<br>TDFTLTISSLQPEDFATYYC | QQTYNI----------<br>------------PPERS | FGQGT<br>KLEIK |
| iPS:403 870 | 21-225_23G4 | VK1\|O12/JK2 | | DIQMTQSPSSLSA<br>SVGDRVTITC | RAS--QNIY--<br>----SYLN | WYQQKPGK<br>APKLLIY | A--------<br>ASSLQS | GVPSRFSGSGSG--<br>TEFTLTISILQPEDFATYYC | QQSYNT----------<br>------------PPECN | FGQGT<br>KLEIK |
| Germline | | | K_FR1 | K_CDR1 | K_FR2 | K_CDR2 | K_FR3 | K_CDR3 | K_FR4 | |
| VK3\|A27/JK3 | | 32163 | EIVLTQSPGTILSL<br>SPGERATLSC | RAS--QSVSS--<br>----SYLA | WYQQKPGQ<br>APRLLIY | G--------<br>ASSRAT | GIPDRFSGSGSG--<br>TDFTLTISRLEPEDFAVYYC | QQYGS-----------<br>------------SPFT | FGPGT<br>KVDIK | |
| iPS:434 311 | 21-225_59H5 | VK3\|A27/JK3 | | EIVLTQSPGTILSL<br>SPGERATLSC | RAS--QSVSS--<br>----IYLA | WFLQKPGQ<br>APRLLIY | G--------<br>ASSRAT | GIPDRFSGSGSG--<br>TDFTLTISRLEPEDFAVYYC | HQYGN-----------<br>------------SPFT | FGPGT<br>KVDFK |
| iPS:435 301 | 21-225_146G4 | VK3\|A27/JK3 | | EIVLTQSPGTILSL<br>FPGERATLSC | RAS--QNIIS--<br>----SYLA | WYQQKPGQ<br>APRLLIF | G--------<br>VSSRAT | GIPDRFSGSGSG--<br>TDFTLTISRLEPEDFAVYYC | QQYGR-----------<br>------------SPFN | FGPGT<br>KVDIN |
| iPS:435 317 | 21-225_147D2 | VK3\|A27/JK3 | | EIVLTQSPGTILSL<br>SPGERATLSC | RAS--QSVGS--<br>----SYLA | WYQQKPGQ<br>APRLLIY | G--------<br>ASSRAT | GIPDRFSGSGSG--<br>TDFTLTISRLEPEDFAVYYC | QQYG------------<br>------------SLFT | FGPGT<br>KVDIK |
| iPS:435 319 | 21-225_147E3 | VK3\|A27/JK3 | | EIVLTQSPGTILSL<br>SPGERATLSC | RAS--QSVIS--<br>----SYLA | WYQQKPGQ<br>APRLLIY | G--------<br>ASSRAT | AIPDRFSGSGSG--<br>TDFTLTISRLEPEDFAVYYC | QQYGR-----------<br>------------SPFN | FGPGT<br>KVDIK |
| iPS:435 383 | 21-225_148D7 | VK3\|A27/JK3 | | EIVLTQSPGTILSL<br>FPGERVTLSC | RAS--QSIIS--<br>----NYLA | WYQQKPGQ<br>APRLLIF | G--------<br>VSSRAT | GIPDRFSGSGSG--<br>TDFTLTISRLEPEDFAVYYC | QQYGR-----------<br>------------SPFN | FGPGT<br>KVDIK |
| iPS:435 443 | 21-225_152E7 | VK3\|A27/JK3 | | EIVLTQSPGTILSL<br>FPGERATLSC | RAS--QSVIS--<br>----SYLA | WYQQKPGQ<br>APRLLIF | G--------<br>VSSRAT | GIPDRFSGSGSG--<br>TDFTLTISRLEPEDFAVYYC | QQYGR-----------<br>------------SPFN | FGPGT<br>KVDIK |
| iPS:435 465 | 21-225_153A6 | VK3\|A27/JK3 | | EIVLTQSPGTILSL<br>FPGERAPLSC | RAS--QSVIS--<br>----SYLA | WYQQKPGQ<br>APRLLIF | G--------<br>VSSRAT | GIPDRFSGSGSG--<br>TDFTLTISRLEPEDFAVYYC | QQYGR-----------<br>------------SPFN | FGPGT<br>KVDIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:436 058 | 21-225_194A4 | VK3\|A27/ JK3 | | EIVLTQSPGTLSL SPGERATLSC | RAS--RGVSN- ----IYLA | WYQQKPGQ APRLLIY | G-------- ASNRAT | GIPDRFSGSGSG-- TDFTLTISRLEPEDFAVYYC | QHNDY---------- -----------SMFT | FGPGT KVDIK |
| iPS:436 436 | 21-225_216F10 | VK3\|A27/ JK3 | | EVVLTQSPGTLSL SPGERATLSC | RAS--QSVSS- ----SFLA | WYQQKPGQ APRLLIY | G-------- TSTRAT | GIPDRFSGSGSG-- TDFILTISRLEPEDFAVYYC | QQYDR---------- -----------SPFT | FGPGT KVDIK |
| iPS:442 568 | 21-225_149D8 | VK3\|A27/ JK3 | | EIVLTQSPGTLSL FPGERATLSC | RAS--QSVIS- ----SYLA | WYQQKPGQ APRLLIF | G-------- VSSWAT | GIPDRFSGSGSG-- TDFTLTISRLEPEDFAVYYC | QQYGR---------- -----------SPFN | FGPGT KVDIK |
| Germline | | | | K_FR1 | K_CDR1 | K_FR2 | K_CDR2 | K_FR3 | K_CDR3 | K_FR4 |
| VK1\|O18/ JK1 | | | 32164 | DIQMTQSPSSLSA SVCDRVTITC | QAS--QDIS-- ----NYLN | WYQQKPGK APRLLIY | D-------- ASNLET | GVPSRFSGSGSG-- TDFTFTISSLQPEDIATYYC | QQYDN---------- -----------LPWT | FGQGT KVEIK |
| iPS:434 353 | 21-225_64B12 | VK1\|O18/ JK1 | | DIQMTQSPSSLSA SVCDRVTITC | RAS--QDIS-- ----NYLN | WYQQKPGT APNLLIS | D-------- ASILET | GVPSTFSGSGSG-- TDFTFTISSLQPEDIATYYC | QQSDN---------- -----------LPCS | FGQGT KVEIK |
| Germline | | | | K_FR1 | K_CDR1 | K_FR2 | K_CDR2 | K_FR3 | K_CDR3 | K_FR4 |
| VK1\|A20/JK4 | | | 32165 | DIQMTQSPSSLSA SVCDRVTITC | RAS--QGIS-- ----NYLA | WYQQKPGK VPKLLIY | A-------- ASTLQS | GVPSRFSGSGSG-- TDFTLTISSLQPEDVATYYC | QKYNS---------- -----------APLT | FGGGT KVEIK |
| iPS:434 357 | 21-225_65C1 | VK1\|A20/ JK4 | | DIQLTQSPSSLSA SVGDRVTITC | RAS--QVIS-- ----SYLH | WYQQKPGK VPKVLIY | S-------- ASNLQC | GVPSRFSGSGSG-- TDFTLTISSLQPEDVATYYG | QRPYN---------- -----------APLT | FGGGT KVEIK |
| iPS:434 375 | 21-225_66C7 | VK1\|A20/ JK4 | | DIQLTQSPSSLSA SVGDRVTITR | RAS--QGIS-- ----NYLH | WYQQKPGK APRLLIY | C-------- ASNLQC | GVPSRFSGSGSG-- TDFTLTISSLQPEDVATYYC | QQHNN---------- -----------SPLT | FGGGT KVEIK |
| iPS:434 457 | 21-225_72G12 | VK1\|A20/ JK4 | | DIQLTQSPSSLSA SVGDRVTITC | RAS--QGIS-- ----SYLN | WSQQKPGK VPKLLIC | G-------- ASNLQS | GVPSRFSGSASG-- TEFILTISSLQPEDVTTYYG | QQNYN---------- -----------APLT | FGGGT KVEIK |
| Germline | | | | K_FR1 | K_CDR1 | K_FR2 | K_CDR2 | K_FR3 | K_CDR3 | K_FR4 |
| VK3\|A27/JK4 | | | 32166 | EIVLTQSPGTLSL SPGERATLSC | RAS--QSVSS- ----SYLA | WYQQKPGQ APRLLIY | G-------- ASSRAT | GIPDRFSGSGSG-- TDFTLTISRLEPEDFAVYYC | QQYGS---------- -----------SPLT | FGGGT KVEIK |
| iPS:434 479 | 21-225_76H1 | VK3\|A27/ JK4 | | EFMLTQSPGTLYW SPGERATLSC | RAS--QSVSS- ----SYLV | WYQQKPGQ APRLLIY | G-------- ASTRAT | GIPDRFSGSGSG-- TDFTLTISRLEPEYFAVYYC | QQYGC---------- -----------SPLT | FGGGT KVEIT |
| iPS:434 513 | 21-225_76A6 | VK3\|A27/ JK4 | | EIVLTQSPGTRSW SPGERATLSC | RAS--QSVSS- ----SYLA | WYQQKPGQ APRLLIY | G-------- ASTRAT | GIPDRFSGSGSG-- TDFTLTISRLEPEYFAVYYC | QQYGN---------- -----------SPLT | FGGGT KVEIK |
| iPS:434 515 | 21-225_74A5 | VK3\|A27/ JK4 | | EFMLTQSPGTLSL SPGERATLSC | RAS--QSVSS- ----SYLV | WYQQKPGQ APRLLIY | G-------- ASTRAT | GIPDRFSGSGSG-- TDFTLTISRLEPEYFAVYYC | QQYGC---------- -----------SPLT | FGGGT KVEIT |
| iPS:434 529 | 21-225_76B9 | VK3\|A27/ JK4 | | EFMLTQSPGTLSW SPGERATLSC | RAS--QSVSS- ----SYLV | WYQQKPGQ APRLLIY | G-------- ASTRAT | GIPDRFSGSGSG-- TDFTLTISRLEPEYFAVYYC | QQYGC---------- -----------SPLT | FGGGT KVEIT |
| iPS:434 583 | 21-225_74B6 | VK3\|A27/ JK4 | | EIVLTQSPGTLSL SPGERATLSC | RAS--QSVSS- ----SYLA | WYQQKPGQ APRLLIY | G-------- ASTRAT | GIPDRFSGSGSG-- TDFTLTISRLEPEYFAVYYC | QQYGN---------- -----------SPLT | FGGGT KVEIK |
| iPS:434 587 | 21-225_74G3 | VK3\|A27/ JK4 | | EFMLTQSPGTLCW SPGERATLSC | RAS--QSVSS- ----SYLV | WYQQKPGQ APRLLIY | G-------- ASTRAT | GIPDRFSGSGSG-- TDFTLTISRLEPEYFAVYYC | QQYGC---------- -----------SPLT | FGGGT KVEIT |
| iPS:434 603 | 21-225_77D11 | VK3\|A27/ JK4 | | EFMLTQSPGTLYW SPGERATISC | RAS--QSVSS- ----SYLV | WYQQKPGQ APRLLIY | G-------- ASTRAT | GIPDRFSGSGSG-- TDFTLTISRLEPEYFAVYYC | QQYGC---------- -----------SPLT | FGGGT KVEIT |
| iPS:434 705 | 21-225_80A2 | VK3\|A27/ JK4 | | EFMLTQSPGTLYL SPGERATLSC | RAS--QSVSS- ----SYLV | WYQQKPGQ APRLLIY | G-------- ASTRAT | GIPDRFSGSGSG-- TDFTLTISRLEPEYFAVYYC | QQYGC---------- -----------SPLT | FGGGT KVEIT |
| iPS:434 747 | 21-225_80C12 | VK3\|A27/ JK4 | | EIVLTQSPGTLYL SPGERATLSC | RAS--QSVSS- ----SYLA | WYQQKPGQ APRLLIY | G-------- ASTRAT | GIPDRFSGSGSG-- TDFTLTISRLEPEYFAVYYC | QQYGN---------- -----------SPLT | FGGGT KVEIK |
| iPS:434 793 | 21-225_82A5 | VK3\|A27/ JK4 | | EIVLTQSPGTLSW SPGERATLSC | RAS--QSVSS- ----SYLA | WYQQKPGQ APRLLIY | G-------- ASTRAT | GIPDRFSGSGSG-- TDFTLTISRLEPEYFAVYYC | QQYGN---------- -----------SPLT | FGGGT KVEIK |
| iPS:434 797 | 21-225_82G5 | VK3\|A27/ JK4 | | EFMLTQSPGTLYW SPGERATLSS | RAS--ESVSS- ----SYLV | WYQQKPGQ APRLLIY | G-------- ASTRAT | GIPDRFSGSGSG-- TDFTLTISRLEPEYFAVYYC | QQYGC---------- -----------SPLT | FGGGT KVEIT |
| iPS:434 805 | 21-225_82D9 | VK3\|A27/ JK4 | | EFMLTQSPGTLSW SPGERATLSC | RAS--ESVSS- ----SYLV | WYQQKPGQ APRLLIY | G-------- ASTRAT | GIPDRFSGSGSG-- TDFTLTISRLEPEYFAVYYC | QQYGC---------- -----------SPLT | FGGGT KVEIT |

| | | | | K_FR1 | K_CDR1 | K_FR2 | K_CDR2 | K_FR3 | K_CDR3 | K_FR4 |
|---|---|---|---|---|---|---|---|---|---|---|
| iPS:434 813 | 21-225_62C12 | VK3\|A27/ JK4 | | EIVLTQSPGTLSW SPGERATLSC | RAS--QSVSS- ----SYLA | WYQQKPGQ APRLLIY | G-------- ASTRAS | GIPDRFSGSGSG-- TDFTLTISRLEPEYFAVYYC | QQYGN----------- ------------SPLT | FGGGT KVEIK |
| iPS:434 825 | 21-225_83C2 | VK3\|A27/ JK4 | | EFMLTQSPGTLCL SPGERATLSC | RAS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:434 833 | 21-225_83C5 | VK3\|A27/ JK4 | | EFMLTQSPGTLCW SPGERATLSC | RAS--ESVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:434 883 | 21-225_85B5 | VK3\|A27/ JK4 | | EFMLTQSPGTILSL SPGERATLSC | RSS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:434 911 | 21-225_85D11 | VK3\|A27/ JK4 | | EFMLTQSPGTILSL STGERATLSC | RSS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:434 957 | 21-225_87A10 | VK3\|A27/ JK4 | | EFVLTQSPGTILYL SPGERATLSC | RAS--QSVSS- ----SYLA | WYQQKPGQ APRLLIY | G-------- ASTRAT | GIPDRFSGSGSG-- TDFTLTISRLEPEYFAVYYC | QQYGN----------- ------------SPLT | FGGGT KVEIK |
| iPS:435 247 | 21-225_96G1 | VK3\|A27/ JK4 | | EIVLTQSPGTILSL STGERATLSC | RAS--QSVSS- ----SYLA | WYQQKPGQ PPRLLIY | G-------- ASTRAS | GIPDRFSGSGSG-- TDFTLTISRLEPEYFAVYYC | QQYGN----------- ------------SPLT | FGGGT KVEIK |
| iPS:436 368 | 21-225_211G3 | VK3\|A27/ JK4 | | EIVLTQSPGTILSL SPGERATLSC | RAS--QSVSS- ----SFLA | WYQQKPGQ APRLLIY | G-------- ASSRAT | GIPDRFSGSGSG-- TDFTLTISRLEPEDFAVYYC | QQYVS----------- ------------SPLT | FGGGT KVEIK |
| iPS:436 426 | 21-225_215C7 | VK3\|A27/ JK4 | | EIVLTQSPGTILSL SPGERVTLSC | RAS--QRITT- ----NFLA | WYQQKPGQ APRLLIY | G-------- ASSRAT | GIPDRFSGSGSG-- TDFTLTISRLESEDFAVYYC | QQYVS----------- ------------SLLT | FGGGT KVEIK |
| iPS:436 432 | 21-225_215H1 2 | VK3\|A27/ JK4 | | EIVLTQSPGTILSL SPGERATLSC | RAS--QSVSS- ----SFLA | WYQQKPGQ APRLLMY | G-------- ASSRAI | GIPDRFSGSGSG-- TDFTLTISRLEPEDFAVYYC | QQYVS----------- ------------SPLT | FGGGT KVEIK |
| iPS:437 322 | 21-225_75B1 | VK3\|A27/ JK4 | | EFMLTQSPGTILYW SPGERATISS | RAS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:437 377 | 21-225_74G9 | VK3\|A27/ JK4 | | EFMLTQSPGTILSL SPGERATLSC | RAS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| | | Germline | | **K_FR1** | **K_CDR1** | **K_FR2** | **K_CDR2** | **K_FR3** | **K_CDR3** | **K_FR4** |
| | VK4\|B3/JK4 | | 32167 | DIVMTQSPDSLAV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------TPLT | FGGGT KVEIK |
| IPS:434 511 | 21-225_74B11 | VK4\|B3/ K4 | | DIVMTQSPDSLTV SLGERATINC | KSS-- QSILYNSNNNN YLA | WYQQKPGQ PPKLLIY | W-------- ASTRES | GVPDRFSGSGSG-- TDFILTISSLQAEDVAVYYC | QQYYS----------- ------------TPPT | FGGGT KVEIK |
| IPS:434 729 | 21-225_80B12 | VK4\|B3/ K4 | | DIVLTQSPDSLAV SLGERATINC | KSR-- QSVLYSSNNYN YLT | WYQQKPGQ PPKLLIY | W-------- ASTRES | GVPDRFSGSGSG-- TDFTLTISSLQAEDVAVYYC | QQYYS----------- ------------SPPT | FGGGT KVEIK |
| IPS:434 851 | 21-225_75A6 | VK4\|B3/ K4 | | DIVMTQSPDSLAV SLGERATINC | KSR-- QSVLHSSNNYN YLA | WYQQKPGQ PPELLIY | W-------- ASTRES | GVPDRFSGSGSG-- TDFTLTISSLQAEDVAVYYC | QQYYS----------- ------------TPPT | FGGGT KVEIK |
| IPS:435 623 | 21-225_162D5 | VK4\|B3/ K4 | | DIVMTQSPDFRNV SMGERAIINF | KSN-- HSVLYRSNNNQ YLA | WYQRKPGQ PPKLLIY | R-------- TSIRKS | GVPDRFSGSGCG-- TDFTLTIDSLQAEDVAVYYC | QQYYS----------- ------------TPPT | FGGGT KVEIK |
| IPS:446 086 | 21-225_94D8 | VK4\|B3/ K4 | | DIVLTQSPDSLAV SLGERATINC | KSR-- QSVLYSSNNYN YLT | WYQQKPGQ PPKLLIY | W-------- ASTRES | GVPDRFSGSGSG-- TDFTLTISSLQAEDVAVYYC | QQYYS----------- ------------SPPT | FGGGT KVEIK |
| | | Germline | | **K_FR1** | **K_CDR1** | **K_FR2** | **K_CDR2** | **K_FR3** | **K_CDR3** | **K_FR4** |

| | | | | K_FR1 | K_CDR1 | K_FR2 | K_CDR2 | K_FR3 | K_CDR3 | K_FR4 |
|---|---|---|---|---|---|---|---|---|---|---|
| VK2\|A19/JK4 | | | 32168 | DIVMTQSPLSLPV TPGEPASISC | RSS-- QSLLHSN- GYNYLD | WYLQKPGQ SPQLLIY | L-------- GSNRAS | GVPDRFSGSGSG-- TDFTLKISRVEAEDVGVYYC | MQALQ----------- ------------TPLT | FGGGT KVEIK |
| iPS:434 539 | 21-225_74A2 | VK2\|A19/ JK4 | | DIVMTQSPLSLPV TPGEPASISC | RSS-- QSLLHSN- GHNYLD | WYLQKPGR SPQLLIY | L-------- GSNRAS | GVPERFSGSGSG-- TDFTLKISRVEAEDVGVYYC | MQPLQ----------- -----------TPFT | FGGGT KVEIK |
| iPS:434 563 | 21-225_75D8 | VK2\|A19/ JK4 | | DIVMTQSPLSLPV TPGEPASISC | RSS-- QSLLHSS- GYNYLD | WYLQKPGQ SPQLLIY | L-------- GSNRAS | GVPDRFSGSGSG-- SDFTLKISRVEAEDVGLYYC | MQALH----------- -----------PPLT | FGGGT KVEIK |
| iPS:435 009 | 21-225_89G4 | VK2\|A19/ JK4 | | DIVMTQSPLSLPV TPGEPASISC | RSS-- QSLLHSS- GYNYLD | WYLQKPGQ SPQLLIY | L-------- GSNRAS | GVPDRFSGSGSG-- TDFTLKISRVEAEDIGVYYC | MQALH----------- -----------IPLT | FGGGT KVEIK |
| iPS:435 059 | 21-225_90C11 | VK2\|A19/ JK4 | | DIVMTQSPLSLPV TPGEPASISC | RYS-- QSLVHSS- GYNYLD | WYLQKPGQ SPQLVIY | L-------- GSNRAS | GVPDRFSGSGSG-- SDFTLKISRVEAEDVGLYYC | MQALH----------- -----------PPLT | FGGGT KVEIK |
| iPS:435 103 | 21-225_92B2 | VK2\|A19/ JK4 | | DIVMTQSPLSLPV TPGEPASISC | RSS-- QSLVHSS- GYNYLD | WYLQKPGQ SPQLVIY | L-------- GSNRAS | GVPDRFSGSGSV-- TDFTLRISRVEAEDIGIYYC | MQALH----------- -----------IPLT | FGGGT KVEIK |
| iPS:435 713 | 21-225_171D7 | VK2\|A19/ JK4 | | DIVMTQSPLSLPV TPGEPASISC | RSS-- QSLLYHN- GYNYLD | WYLQKTGQ SPQLLIY | V-------- GSNRAS | GVPDRFSGSGSG-- TDFTLKISRVEAEDVGVYYC | MQTLQ----------- -----------TPLT | FGGGT KVEIK |
| iPS:436 246 | 21-225_201G6 | VK2\|A19/ JK4 | | DIVMTQSPLSLPV TPGEPASISC | RSS-- QSLLHNN- RYNHLD | WYLQKPGQ SPQLLIY | L-------- GSNRAS | GVPDRFSGSGSG-- TDFTLKISRVEAEDVGVYYC | MQAL------------ -----------QTPT | FGGGT KVEIK |
| iPS:436 254 | 21-225_202C12 | VK2\|A19/ JK4 | | DIVLTQSPLSLPV TPGEPASISC | RSS-- QSLLHNN- KYNHLD | WYLQKPGQ SPQLLIY | L-------- GSNRAS | GVPDRFSGSGSG-- TDFTLKISRVEAEDVGVYYC | MQAL------------ -----------QTPT | FGGGT KVEIK |
| iPS:436 304 | 21-225_201F3 | VK2\|A19/ JK4 | | DIVMTQSPLSLPV TPGEPASISC | RSS-- QSLLHNN- RYNHLD | WYLQKPGQ SPQLLIY | L-------- GSNRAS | GVPDRFSGSGSG-- TDFTLKISRVEAEDVGVYYC | MQAL------------ -----------QTPT | FGGGT KVEIK |
| iPS:436 334 | 21-225_208G3 | VK2\|A19/ JK4 | | DIVLTQSPLSLPV TPGEPASISC | RSS-- QSLLHNN- KYNHLD | WYLQKPGQ SPQLLIY | L-------- GSNRAS | GVPDRFSGSGSG-- TDFTLKISRVEAEDVGVYYC | MQAL------------ -----------QTPT | FGGGT KVEIK |
| iPS:437 248 | 21-225_97H3 | VK2\|A19/ JK4 | | DIVMIQSPLSLPV TPGEPASISC | RSS-- QSLLHSN- GHNYLD | WYLQKPGR SPQLLIY | L-------- GSNRAS | GVPERFSGSGSG-- TDFTLKISRVEAEDVGVYYC | MQPLQ----------- -----------TPFT | FGGGT KVEIK |
| iPS:437 320 | 21-225_75A1 | VK2\|A19/ JK4 | | DIVMTQSPLSLPV TPGEPASISC | RSS-- QSLLHSN- GHNYLD | WYLQKPGR SPQLLIY | L-------- GSNRAS | GVPERFSGSGSG-- TDFTLKISRVEAEDVGVYYC | MQPLQ----------- -----------TPFT | FGGGT KVEIK |
| iPS:437 371 | 21-225_74D8 | VK2\|A19/ JK4 | | DIVMTQSPLSLPV TPGEPASISC | RSS-- QSLVHSS- GYNYLD | WYLQKPGQ SPQLVIY | L-------- GSNRAS | GVPDRFSGSGSG-- SDFTLKISRVEAEDVGLYYC | MQALH----------- -----------PPLT | FGGGT KVEIK |
| iPS:392 718 | 21-225_17B8 | VK2\|A19/ JK4 | | DIVMTQSPLSLPV TPGEPASISC | RSS-- QSLLHSN- GNNSLD | WYLQKPGQ SPQLLIY | L-------- GSHRAS | GVPDRFSDSGSG-- TDFTLKISRVEAEDVGVYYC | MQVLQ----------- ----------TPPLT | FGGGT KVEIK |
| | Germline | | | **K_FR1** | **K_CDR1** | **K_FR2** | **K_CDR2** | **K_FR3** | **K_CDR3** | **K_FR4** |
| VK3\|L2/JK1 | | | 32169 | EIVMTQSPATLSV SPGERATLSC | RAS--QSVS-- ----SNLA | WYQQKPGQ APRLLIY | G-------- ASTRAT | GIPARFSGSGSG-- TEFTLTISSLQSEDFAVYYC | QQYNN----------- ------------WPWT | FGQGT KVEIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:434 871 | 21-225_85H1 | VK3\|L2\|J K1 | | EIVMTQSPATLSV SPGERATLSC | RAS--QDVI-- ----TYLA | WYQQKPGQ APRLLIY | G-------- ASTRAT | GVPARFSGSGSG-- TEFTLTISSLQSEDFALYYC | QEYND----------- ------------WPCS | FGQGT KVEIK |
| iPS:435 421 | 21-225_151F1 | VK3\|L2\|J K1 | | EMVMTQSPATLSV SPGERVTLSC | RAS--QSIN-- ----INIA | WYQQKPGQ APRLLIY | G-------- ASTRAT | GIPARFSGSGSG-- TEFTLTISSLQSEDFAVYYC | QQYND----------- -----------WPPWT | FGQGT KVEIK |
| iPS:435 497 | 21-225_155H9 | VK3\|L2\|J K1 | | EIVMTQSPATLSV SPGERATLSC | RAS--QSVS-- ----SNLA | WYQQKPGQ APRLLIY | G-------- ASTRAT | GIPARFSGSGSG-- TEFTLTISSLQSEDFAVYYC | QQYDD----------- -----------WPPWT | FGQGT KVEIK |
| iPS:435 605 | 21-225_161A4 | VK3\|L2\|J K1 | | EIVMTQSPATLSV SPGERASLSC | RSS--QSVN-- ----SNLA | WYQQRPGQ ALRLLIY | G-------- ASIRAT | DIPARFNGSGSG-- TEFTLTISSLQSEDFAVYFC | QQYN------------ ------------NWWI | FGQGT IVEIK |
| iPS:451 118 | 21-225_191C8 | VK3\|L2\|J K1 | | EIVMTQSPATLSV SPGERATLSC | RAS--QSVR-- ----SNLA | WYQQEPGQ APRLLIY | G-------- ASIRAI | GIPARFSGSGSG-- TEFTLTISSLQSEDFAVYYC | QQSFT----------- ------------WLRI | FGQGT KVEIK |
| iPS:392 734 | 21-225_17D8 | VK3\|L2\|J K1 | | EIVMTQSPSTLSV SPGERATLSC | RAS--QSVS-- ----SNLA | WFQQKPGQ APRLLIN | G-------- ASTRAS | GIPARFSGSGSG-- TEFTLTISSLQSEDFAVYYC | QQYNN----------- ------------WPLT | FGQGT KVEIK |
| iPS:392 768 | 21-225_20B8 | VK3\|L2\|J K1 | | EIVMTQSPSTLSV SPGERVTLSC | RAS--QSVS-- ----SNLA | WFQQKPGQ APRLLIN | G-------- ASTRAS | GIPARFSGSGSG-- TEFTLTISSLQSEDFAVYYC | QQYNN----------- ------------CPLT | FGQGT KVEIK |
| iPS:393 044 | 21-225_25B8 | VK3\|L2\|J K1 | | EIVMTQSPATLSV SPGERATLSC | RAS--QSVR-- ----SNLA | WYQQKPGQ APRLLIY | G-------- ASTRAT | GIPARFSGSGSG-- TEFTLTISSLQSEDFALYYC | QQYNN----------- -----------WPPWP | FGQGT KVEIK |
| iPS:393 050 | 21-225_28C5 | VK3\|L2\|J K1 | | EIVMTQSPATLSV SPGERATLSC | RAS--QSVS-- ----SNLA | WYHQKPGQ APRLLIY | G-------- ASTRAT | GIPARFSGSGSG-- TEFTLTISSLQSEDFALYYC | QQYNN----------- -----------WPPWP | FGQGT KVEIK |
| iPS:393 906 | 21-225_13D3 | VK3\|L2\|J K1 | | EIVMTQSPATLSV SPGESATLSC | RAS--QTVS-- ----SNLA | WFQQKPGQ APRLLIN | G-------- ASTRAT | GIPARFSGSGSG-- TEFTLTISSLQSEDFAVYFC | QQYHD----------- ------------WPPT | FGQGT KVEIK |
| | | **Germline** | | **K_FR1** | **K_CDR1** | **K_FR2** | **K_CDR2** | **K_FR3** | **K_CDR3** | **K_FR4** |
| | **VK1\|L1\|JK5** | | 32170 | DIQMTQSPSSLSA SVGDRVTITC | RAS--QGIS-- ----NYLA | WFQQKPGK APKSLIY | A-------- ASSLQS | GVPSRFSGSGSG-- TDFTLTISSLQPEDFATYYC | QQYNS----------- ------------YPIT | FGQGT RLEIK |
| iPS:434 947 | 21-225_87B7 | VK1\|L1\|J K5 | | DIQMTQSPSSLSA SVGDRVIITC | RAS--QGIS-- ----NYLA | WFQQKPGK APKSLIY | A-------- ASSLHS | GVPSKFSGSGSG-- TDFTLTISSLQPEDSATYFC | LLYLT----------- ------------YPLT | FGQGT RLEIK |
| iPS:435 427 | 21-225_151C9 | VK1\|L1\|J K5 | | DIQMTQSPSSLSA SVGDRVTITC | RAS--QGIS-- ----KYLA | WFQQKPGK APKSLIY | D-------- ASRLQS | GVPSKFSGSGSG-- TDFTLTISSLQPEDFASYYC | HQYKH----------- ------------YPIT | FGQGT RLEIK |
| iPS:435 529 | 21-225_157H7 | VK1\|L1\|J K5 | | DIQMTQSPSSLSA SVGDRVTITC | RAS--QDIS-- ----NFLA | WFQQKPGK APKSLVS | T-------- ASSLQS | GVPSKFSGSGSG-- TDFTLTISSLQPEDFATYYC | QQYHS----------- ------------YPIT | FGQGT RLEIK |
| iPS:436 066 | 21-225_194B7 | VK1\|L1\|J K5 | | DIQMTQSPSSLSA SVGDRVTITC | RAS--QGIS-- ----KYLA | WFQQKPGK APKSLIY | G-------- ASRLQS | GVPSKFSGSGSG-- TDFTLTISSLQPEDFATYYC | QHYLN----------- ------------YPLT | FGQGT RLEIK |
| iPS:437 274 | 21-225_196D4 | VK1\|L1\|J K5 | | DIQMTQSPSSLSA SVGDRVTITC | RAS--QGIS-- ----NYLA | WFQQNPGK APKSLIY | A-------- ASSLQS | GVPSKFSGSGCG-- TDFTLIISSPQPEDVATYYC | QHYLN----------- ------------YPLT | FGQGT RLEIK |
| iPS:392 748 | 21-225_20A8 | VK1\|L1\|J K5 | | DIQMTQSPSSLSA SVGDRVTITC | RAS--QGIN-- ----NYLV | WFQQKPGK APKSLIY | A-------- ASSLLS | GVPSKFSGSGSG-- TDFTLIISSLQPEDFATYYC | QQYNS----------- ------------YPIT | FGQGT RLEIK |
| iPS:393 062 | 21-225_33H3 | VK1\|L1\|J K5 | | DIQMTQSPSSLSA SVGDRVTITC | QAS--QDIS-- ----NFLN | WFRQKPGK APNSLIY | D-------- ASNLVT | GVPSRFSGRGSG-- TDFTLTISSLQPEDFATYYC | QQYDN----------- ------------LPIT | FGQGT RLEIK |
| iPS:398 532 | 21-225_33B7 | VK1\|L1\|J K5 | | DIQMTQSPSSLSA SVGDRVTITC | RAS--QDIS-- ----NYLA | WFQQKPGK APTSLIY | A-------- ASSLQS | GVPSKFSGSGSG-- TDFTLTISSLQPEDFATFYC | QQYHS----------- ------------YPLT | FGQGT RLEIK |
| iPS:402 221 | 21-225_2C12 | VK1\|L1\|J K5 | | DIQMTQSPSSLSA SVGDRVTITC | RAS--QGIS-- ----NYLA | WFQQKSGK APKSLIS | A-------- ATSLQS | GVPSQFSGSGSG-- TDFTLTISSLQPEDFATYYC | QQYYS----------- ------------YPIT | FGQGT RLEIK |
| | | **Germline** | | **K_FR1** | **K_CDR1** | **K_FR2** | **K_CDR2** | **K_FR3** | **K_CDR3** | **K_FR4** |
| | **VK4\|B3\|JK5** | | 32171 | DIVMTQSPDSLAV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------TPIT | FGQGT RLEIK |

| ID | Clone | Germline | No. | K_FR1 | K_CDR1 | K_FR2 | K_CDR2 | K_FR3 | K_CDR3 | K_FR4 |
|---|---|---|---|---|---|---|---|---|---|---|
| iPS:435 665 | 21-225_169F2 | VK4\|B3/JK5 | | DIVMTQSPDSLAV SLGERATINC | KSS-- QSVLYISNNKN YLA | WYQQKPGQ PPKLLIY | W-------- ASTRES | GVPDRFSGSGSG-- TDFTLTISSLQAEDVAVYYC | QQYY------------ ------------RAPT | FGQGT RLEIK |
| iPS:435 671 | 21-225_169H5 | VK4\|B3/JK5 | | DIVMTQSPDSLAV SLGERATINC | KSS-- QSVLYISNNKN YLA | WYQQKPGQ PPKLLIY | W-------- ASTRES | GVPDRFSGSGSG-- TDFTLTISSLQAEDVAVYYC | QQYY------------ ------------RAPT | FGQGT RLEIK |
| iPS:436 554 | 21-225_224C10 | VK4\|B3/JK5 | | DIVMTQSPDSLAA SLGERATITC | KSS-- QSVLYNSNNKN YLA | WYQQKPGQ PPKLLIY | W-------- ASTRES | GVPDRFSGSGSG-- TDFTLTISSLQAEDVAIYYC | QQYYI---------- ------------NPCS | FGQGT RLEIK |
| iPS:398 510 | 21-225_25A3 | VK4\|B3/JK5 | | DIVMTQSPDSLIV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------TPCS | FGQGT RLEIK |
| iPS:398 516 | 21-225_26A9 | VK4\|B3/JK5 | | DIVMTQSPDSLAV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------SPCS | FGQGT RLEIK |
| | | Germline | | K_FR1 | K_CDR1 | K_FR2 | K_CDR2 | K_FR3 | K_CDR3 | K_FR4 |
| | VK2\|A19/JK1 | | 32172 | DIVMTQSPLSLPV TPGEPASISC | RSS-- QSLLHSN- GYNYLD | WYLQKPGQ SPQLLIY | L-------- GSNRAS | GVPDRFSGSGSG-- TDFTLKISRVEAEDVGVYYC | MQALQ----------- -----------TPWT | FGQGT KVEIK |
| iPS:435 667 | 21-225_169E3 | VK2\|A19/JK1 | | DIVMTQSPLSLPV TPGEPASISC | RSS-- QSLLHNN- GYKYLD | WYLQKPGQ SPQLLIY | L-------- GSNRAS | GVPDRFSASGSG-- TDFTLKISRVEAEDVGVYYC | MQVLQ----------- -----------TPWT | FGQGT KVEIK |
| iPS:435 673 | 21-225_169E6 | VK2\|A19/JK1 | | DIVMTQSPLSLPV TPGEPASISC | RSS-- QSLLHNN- GYKYLD | WYLQKPGQ SPQLLIY | L-------- GSNRAS | GVPDRFSASGSG-- TDFTLKISRVEAEDVGVYYC | MQVLQ----------- -----------TPWT | FGQGT KVEIK |
| iPS:435 759 | 21-225_176E6 | VK2\|A19/JK1 | | DIVMTQSPLSLPV TPGEPASISC | RSS-- QSLLHNN- GYKYLD | WYLQKPGQ SPQLLIY | L-------- GSNRAS | GVPDRFSASGSG-- TDFTLKISRVEAEDVGVYYC | MQVLQ----------- -----------TPWT | FGQGT KVEIK |
| | | Germline | | K_FR1 | K_CDR1 | K_FR2 | K_CDR2 | K_FR3 | K_CDR3 | K_FR4 |
| | VK6\|A26/JK1 | | 32173 | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------LPWT | FGQGT KVEIK |
| iPS:435 817 | 21-225_190B11 | VK6\|A26/JK1 | | EIVLTQSPDFQSV APKEKVTITC | RAS--QSIG-- ----SNLH | WYQQKPDQ SPKLLIK | S-------- ASQSFS | GVPSRFSGSGSG-- TDFTLTINSLETEDAATYYC | QQSSS----------- ------------LPWT | FGQGT KVEIK |
| iPS:435 823 | 21-225_190F11 | VK6\|A26/JK1 | | EIVLTQFPDSQSV TPKEKVTITC | RAS--QNIG-- ----SSLH | WYQQKPEQ SPKVLIK | Y-------- ASQSFS | GVPSRFSGSGSG-- TDFTLTINSLEAEDAATYYC | HQSSS----------- ------------FPRT | FGQGT KVEIK |
| iPS:435 867 | 21-225_191E5 | VK6\|A26/JK1 | | EIVLTQFPDSQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------FPRT | FGQGT KVEIK |
| iPS:435 917 | 21-225_190D5 | VK6\|A26/JK1 | | EIVLTQSPDFQSV APKEKVTITC | RAS--QSIG-- ----SNLH | WYQQKPDQ SPKLLIK | S-------- ASQSFS | GVPSRFSGSGSG-- TDFTLTINSLETEDAATYYC | QQSSS----------- ------------LPWT | FGQGT KVEIK |
| iPS:435 929 | 21-225_190D9 | VK6\|A26/JK1 | | EIVLTQFPDSQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------FPRT | FGQGT KVEIK |
| iPS:435 935 | 21-225_190H8 | VK6\|A26/JK1 | | EIVLTQFPDSQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------FPRT | FGQGT KVEIK |
| iPS:436 056 | 21-225_194C3 | VK6\|A26/JK1 | | EIVLTQSPDFQSV APKEKVTITC | RAS--QSIG-- ----SNLH | WYQQKPDQ SPKLLIK | S-------- ASQSFS | GVPSRFSGSGSG-- TDFTLTINSLETEDAATYYC | QQSSS----------- ------------LPWT | FGQGT KVEIK |
| iPS:436 216 | 21-225_200B7 | VK6\|A26/JK1 | | EIVLTQSPDFQSV TPKEKVTITC | RAS--QNIG-- ----NTLH | WYQQKPDQ SPKLLIK | Y-------- ASQSFS | GVPSRFSGSGSG-- TDFILTINSLEAEDAATYYC | HQSGS----------- ------------LPQT | FGQGT KVEIK |

| ID | Clone | Gene | No. | K_FR1 | K_CDR1 | K_FR2 | K_CDR2 | K_FR3 | K_CDR3 | K_FR4 |
|---|---|---|---|---|---|---|---|---|---|---|
| iPS:436 220 | 21-225_200F8 | VK6\|A26/JK1 | | EIVLTQSPDFQSV APKEKVTITC | RAS--QSIG-- ----SNLH | WYQQKPDQ SPKLLIK | S-------- ASQSFS | GVPSRFSGSGSG-- TDFTLTINSLETEDAATYYC | QQSSS----------- -----------LPWT | FGQGT KVEIK |
| iPS:436 448 | 21-225_217A3 | VK6\|A26/JK1 | | EIVLTQSPDFKSV TPKEKVTITC | RAS--QSIG-- ----SSLH | WYQQKPDQ SPKLLVK | Y-------- ASQSFS | GVPSRFSGSGSG-- TDFTLTINSLEAEDCATYYC | HQSRS----------- -----------LPWT | FGQGT KVEIK |
| | Germline | | | **K_FR1** | **K_CDR1** | **K_FR2** | **K_CDR2** | **K_FR3** | **K_CDR3** | **K_FR4** |
| | VK6\|A26/JK4 | | 32174 | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- -----------LPLT | FGGGT KVEIK |
| iPS:435 829 | 21-225_190B12 | VK6\|A26/JK4 | | EIVLTQSPDFQSV TPKEKVTITC | RAS--QSIG-- ----SSLH | WYQQKPDQ SPKLLIK | Y-------- ASQSFS | GDPSRFSGSGSG-- TDFTLTINSLEAEDAATYYC | HQTRS----------- -----------LPLT | FGGGT KVEIK |
| iPS:435 863 | 21-225_191H4 | VK6\|A26/JK4 | | EIVLTQSPDFQSV TPKEKVTITC | RAN--QSIG-- ----SSLH | WYQQKPDQ SPKLLIK | Y-------- ASQSLS | GVPSRFSASGSG-- TDFTLTINSLDAEDAATYYC | HQTGR----------- -----------LPLT | FGGGT KVEIK |
| iPS:435 943 | 21-225_191C9 | VK6\|A26/JK4 | | EIVLTQSPDFQSV TPKEKVTITC | RAS--QSIG-- ----SSLH | WYQQKPDQ SPKLLIK | Y-------- ASQSFS | GDPSRFSGSGSG-- TDFTLTINSLEAEDAATYYC | HQTRS----------- -----------LPLT | FGGGT KVEIK |
| iPS:435 983 | 21-225_192E5 | VK6\|A26/JK4 | | EIVLTQSPDFQSV TPKEKVTITC | RAS--QSIG-- ----RSLH | WYQQKPDQ SPKLLIK | Y-------- ASQSFS | GVPSRFSGSGSG-- TDFTLTINSLEAEDAATYYC | HQSSR----------- -----------LPLT | FGGGT KVEIK |
| iPS:436 043 | 21-225_193G9 | VK6\|A26/JK4 | | EIVLTQSPDFQSV TPKEKVTITC | RAS--QSIG-- ----RSLH | WYQQRPDQ SLKLLIK | Y-------- ASQSFS | GVPSRFSGSGSG-- TDFTLTINSLEAEDAATYFC | HQSSR----------- -----------LPLT | FGGGT KVEIK |
| iPS:436 084 | 21-225_195F2 | VK6\|A26/JK4 | | EIVLTQSPDFQSV TPKEKVTITC | RAS--QSIG-- ----SSLH | WYQQKPDQ SPKLLIK | Y-------- ASQSFS | GVPSRFSGSGSG-- TDFALTISSLEAEDAATYYC | HQSRT----------- -----------LPLT | FGGGT KVEIK |
| iPS:436 094 | 21-225_195B10 | VK6\|A26/JK4 | | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSGR----------- -----------LPLT | FGGGT KVEIK |
| iPS:436 240 | 21-225_201E8 | VK6\|A26/JK4 | | EIVLTQSPAFQSV TPKEKVTITC | RAS--QNIG-- ----RSLH | WYQQKPDQ SPKLLIK | Y-------- ASQSFS | GVPSRFSGSGSG-- TNFTLTINSLEAEDAVTYYC | HQSRS----------- -----------LPLT | FGGGT KVEIR |
| iPS:436 314 | 21-225_206G4 | VK6\|A26/JK4 | | EIVLTQSPDFQSV TPKEKVTITC | RAS--QSIG-- ----RSLH | WYQQKPDQ SPKLLIK | Y-------- ASQSFS | GVPSRFSGSGSG-- TDFTLTINSLEAEDAATYYC | HQSRS----------- -----------LPLT | FGGGT KVEIK |
| | Germline | | | **K_FR1** | **K_CDR1** | **K_FR2** | **K_CDR2** | **K_FR3** | **K_CDR3** | **K_FR4** |
| | VK1\|A20/JK3 | | 32175 | DIQMTQSPSSLSA SVGDRVTITC | RAS--QGIS-- ----NYLA | WYQQKPGK VPKLLIY | A-------- ASTLQS | GVPSRFSGSGSG-- TDFTLTISSLQPEDVATYYC | QKYNS----------- -----------APFT | FGPGT KVDIK |
| iPS:435 833 | 21-225_190D12 | VK1\|A20/JK3 | | DIQMTQSPSSLSA SVGDRVTITC | RAS--QGIS-- ----NYLA | WYQQKPGK VPKLLIY | V-------- ASTLQS | GVPSRFSGSGSG-- TDFTLTISSLQPEDVATYYC | QKYNS----------- -----------APFT | FGPGT KVDIK |
| iPS:436 019 | 21-225_193C4 | VK1\|A20/JK3 | | DIQMTQSPSSLSA SVGDRVTITC | RPS--QGIS-- ----IYLA | WYQQKPGN VPKLLIY | A-------- ASTLQS | GVPSRFSGSGSG-- TDFTLTISSLQPEDVATYYC | QKYNS----------- -----------APFT | FGPGT KVDIK |
| iPS:394 010 | 21-225_12G5 | VK1\|A20/JK3 | | DIQMTQSPSSLSA SVGDRVTIPC | RAS--QDIS-- ----NYLA | WYQQKPGK VPKLLIY | A-------- AYILQS | GVPSRFSGSGSG-- TDFTLTISSLQPEDVAAYYC | QKYDS----------- -----------APFT | FGPGT KVDIK |
| | Germline | | | **K_FR1** | **K_CDR1** | **K_FR2** | **K_CDR2** | **K_FR3** | **K_CDR3** | **K_FR4** |
| | VK6\|A26/JK3 | | 32176 | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- -----------LPFT | FGPGT KVDIK |
| iPS:436 025 | 21-225_193B5 | VK6\|A26/JK3 | | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSR----------- -----------LPFT | FGPGT KVDIK |
| iPS:436 096 | 21-225_195E10 | VK6\|A26/JK3 | | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSR----------- -----------LPFT | FGPGT KVDIK |
| iPS:436 408 | 21-225_214H8 | VK6\|A26/JK3 | | EIVLTQSPDFQSV TPKEKVTITC | RAS--QSIG-- ----VSLH | WYQQKPDQ SPQLLIK | Y-------- ASQSFS | GVPSRFSGSGSG-- TDFTLTINSLEAEDAATYYC | HQSRS----------- -----------LPFT | FGPGS KVDIK |

| | | | | K_FR1 | K_CDR1 | K_FR2 | K_CDR2 | K_FR3 | K_CDR3 | K_FR4 |
|---|---|---|---|---|---|---|---|---|---|---|
| iPS:436 424 | 21-225_215H6 | VK6\|A26/ JK3 | | EIVLTQSPDFQSV TPKEKVTITC | RAS--QSIG-----VSLH | WYQQKPDQ SPQLLIK | Y-------- ASQSLS | GVPSRFSGSGSG-- TDFTLTINSLEAEDAATYYC | HQSRS------------------------LPFT | FGPGS KVDIK |
| | | Germline | | K_FR1 | K_CDR1 | K_FR2 | K_CDR2 | K_FR3 | K_CDR3 | K_FR4 |
| | VK1\|L5/JK2 | | 32177 | DIQMTQSPSSVSA SVGDRVTITC | RAS--QGIS-----SWLA | WYQQKPGK APKLLIY | A-------- ASSLQS | GVPSRFSGSGSG TDFTLTISSLQPEDFATYYC | QQANS----------------FPYT | FGQGT KLEIK |
| iPS:436 082 | 21-225_195D9 | VK1\|L5/J K2 | | DIQMTQSPSSVSA SVGDRVTITC | RAS--QGIS-----RWLA | WYQQKPGK APKLLIY | A-------- ASSLLG | GVPSRFSGSGSG-- TDFTLTISSLQPEDFATYYC | QRANS-----------FPCS | FGQGT KLEIK |
| iPS:436 118 | 21-225_196A1 0 | VK1\|L5/J K2 | | DIQMTQYPSYVSA SVGDRVSITC | RAS--QGIS-----RWLA | WYQQKPGK AAKFLIY | A-------- ASSLLG | GVSSRFSGSGSG-- TDFTLTISSLQPEDFAIYYC | QRDNS-----------LPCS | FGQGT KLEIK |
| | | Germline | | K_FR1 | K_CDR1 | K_FR2 | K_CDR2 | K_FR3 | K_CDR3 | K_FR4 |
| | VK2\|A19/JK 2 | | 32178 | DIVMTQSPLSLPV TPGEPASISC | RSS-- QSLLHSN-GYNYLD | WYLQKPGQ SPQLLIY | L-------- GSNRAS | GVPDRFSGSGSG-- TDFTLKISRVEAEDVGVYYC | MQALQ-----------TPYT | FGQGT KLEIK |
| iPS:436 362 | 21-225_210C1 2 | VK2\|A19/ JK2 | | DIVMTQSPLSLPV TPGEPASISC | RSS-- QSLLHYN-GHNFLD | WYLQKPGQ SPQLLIY | L-------- VSNRAS | GVPDRFSGSGSG-- TDFTLKISRVEAEDVGVYYC | MQALQ-----------TPMCS | FGQGT KLEIK |
| iPS:436 374 | 21-225_211C1 0 | VK2\|A19/ JK2 | | DIVMTQSPLSLPV TPGEPASISC | RSS-- QSLLHSN-GYNYLD | WYLLKPGQ SPQLLIY | L-------- GSNRAS | GVPDRFSGSGSG-- TDFTLKISRMEAEDVGIYYC | MQALL-----------TPVCS | FGQGT KLEIK |
| | | Germline | | K_FR1 | K_CDR1 | K_FR2 | K_CDR2 | K_FR3 | K_CDR3 | K_FR4 |
| | VK2\|A17/JK 1 | | 32179 | DVVMTQSPLSLPV TLGQPASISC | RSS-- QSLVYSD-GNTYLN | WFQQRPGQ SPRRLIY | K-------- VSNRDS | GVPDRFSGSGSG-- TDFTLKISRVEAEDVGVYYC | MQGTH-----------WPWT | FGQGT KVEIK |
| iPS:437 226 | 21-225_57C2 | VK2\|A17/ JK1 | | DVVMTQSPLSLPV TLGQPASISC | RSS-- QSLVYSD-GNTYLN | WFQQRPGQ SPRRLIY | E-------- VSNWDS | GVPNRFSGSGSG-- TDFTLKISAVEAEDVGVYYC | VQGTH-----------WPRT | FGQGT KVEIK |
| | | Germline | | K_FR1 | K_CDR1 | K_FR2 | K_CDR2 | K_FR3 | K_CDR3 | K_FR4 |
| | VK1\|A20/JK 5 | | 32180 | DIQMTQSPSSLSA SVGDRVTITC | RAS--QGTS-----NYLA | WYQQKPGK VPKLLIY | A-------- ASTLQS | GVPSRFSGSGSG-- TDFTLTISSLQPEDVATYYC | QKYNS-----------APIT | FGQGT RLEIK |
| iPS:392 726 | 21-225_20B5 | VK1\|A20/ JK5 | | DIQMTQSPSSLSA SVGDRVTITC | RAS--QGIN-----NYLA | WYQQKPGK IPKLLIY | A-------- ASTLQS | GVPSRFSGSGSG-- TDFTLTISSLQPEDVATYCC | QKYNS-----------APPIT | FGQGT RLEIK |
| iPS:392 792 | 21-225_20G12 | VK1\|A20/ JK5 | | DIQMTQSPSSLSA SVGDRVTITC | RAS--QGIS-----NYLA | WYQQKPGK VPKVLIY | T-------- ASTLQS | GVPSRFSGSGSG-- TDFTLTVSSLQPEDVATYYC | QKYNS-----------APPIT | FGQGT RLEIK |
| iPS:398 478 | 21-225_17C10 | VK1\|A20/ JK5 | | DIQMTQSPSSQSA SVGDRVTITC | RAS--QGIS-----NYLA | WYQQKPGR VPKLLIY | A-------- ASTLQS | GVPSRFSGSGSG-- TDFTLTISSLQPDDVATYYC | QKYNS-----------APPLT | FGQGT RLEIK |
| | | Germline | | K_FR1 | K_CDR1 | K_FR2 | K_CDR2 | K_FR3 | K_CDR3 | K_FR4 |
| | VK1\|L1/JK2 | | 32181 | DIQMTQSPSSLSA SVGDRVTITC | RAS--QGIS-----NYLA | WFQQKPGK APKSLIY | A-------- ASSLQS | GVPSRFSGSGSG-- TDFTLTISSLQPEDFATYYC | QQYNS-----------YPYT | FGQGT KLEIK |
| iPS:392 776 | 21-225_21A12 | VK1\|L1/J K2 | | DIQMTQSPSSLSA SVGDRVTITC | RAS--QGIS-----KYLA | WFQQKPGK APKSLIY | A-------- ASSLQS | GVPSKFSGSGSG-- TDFTLTISSLQPEDFATYYC | QQYNS-----------YPFR | FGQGT KLEIK |
| | | Germline | | K_FR1 | K_CDR1 | K_FR2 | K_CDR2 | K_FR3 | K_CDR3 | K_FR4 |
| | VK1\|A30/JK 3 | | 32182 | DIQMTQSPSSLSA SVGDRVTITC | RAS--QGIR-----NDLG | WYQQKPGK APKRLIY | A-------- ASSLQS | GVPSRFSGSGSG-- TEFTLTISSLQPEDFATYYC | LQHNS-----------YPFT | FGPGT KVDIK |
| iPS:437 240 | 21-225_84H12 | VK1\|A30/ JK3 | | DIQMTQSPSYQSA SVGDRVTITC | RAS--QGIR-----NDLG | WFQQKPGK APKRLIY | A-------- ASSLQS | GVPSRFSGSGSG-- TEFTLTISSLQPEDFATYYC | LQHND-----------YPFT | FGPGT KVDIK |

| ID | Clone | Germline | No. | FR1 | CDR1 | FR2 | CDR2 | FR3 | CDR3 | FR4 |
|---|---|---|---|---|---|---|---|---|---|---|
| iPS:434 577 | 21-225_75C11 | VK1\|A30/JK3 | | DIQMTQSPSSLSA SVGDRVTITC | RAS--QGIR-- ----NDLG | WFQQKPGK APKRLIY | A-------- ASSLQS | GVPSRFSGSGSG-- TEFTLTISSLQPEDFATYYC | LQHND----------- ------------YPFT | FGPGT KVDIK |
| iPS:434 553 | 21-225_76H12 | VK1\|A30/JK3 | | DIQMTQSPSSLSA SVGDRVTITC | RAS--QGIR-- ----NDLG | WFQQKPGK APKRLIY | A-------- ASRLQS | GVPSRFSGSGSG-- TEFTLTISSLQPEDFATYYC | LQHND----------- ------------YPFT | FGPGT KVDIK |
| iPS:434 927 | 21-225_86E5 | VK1\|A30/JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYHC | LQHND----------- ------------YPFT | FGPGT KVEIK |
| | Germline | | | K_FR1 | K_CDR1 | K_FR2 | K_CDR2 | K_FR3 | K_CDR3 | K_FR4 |
| | VK1\|L5/JK1 | | 32183 | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQANS----------- -----------FPWT | FGQGT KVEIK |
| iPS:435 477 | 21-225_154E8 | VK1\|L5/JK1 | | DIQMTQSPSSVSA SIGDRVTITC | RAS--QFIS-- ----SWLA | WYQQKPGK APKLLIY | T-------- ASSLQS | GVPSRFSGSGSG-- TDFTLTISSLQPEDFATYCC | QQANS----------- -----------FPWT | FGQGT KVEFN |
| iPS:435 385 | 21-225_149G7 | VK1\|L5/JK1 | | DIQMTQSPSSVSA SVGDRVTITC | RAS--QFIS-- ----SWLA | WYQQKPGK APKFLIY | A-------- ASSLQS | GVPSRFSGSGSG-- TDFTLTISSLQPEDFATYYC | QQANS----------- -----------FPWT | FGQGT KVEIN |

**LAMBDA_VARIABLE**

| ID | Clone | Germline | No. | L_FR1 | L_CDR1 | L_FR2 | L_CDR2 | L_FR3 | L_CDR3 | L_FR4 |
|---|---|---|---|---|---|---|---|---|---|---|
| | Germline | | | L_FR1 | L_CDR1 | L_FR2 | L_CDR2 | L_FR3 | L_CDR3 | L_FR4 |
| | VL3\|3r/JL2 | | 32184 | SYELTQP- PSVSVSPGQTASI TC | SGD---KLGD- ----KYAC | WYQQKPGQ SPVLVIY | Q-------- DSKRPS | GIPERFSGSNSG-- NTATLTISGTQAMDEADYYC | QAWDS----------- -----------STAVV | FGGGT KLTVL |
| iPS:4 53445 | 21-225_148E10 | VL3\|3r/JL2 | | SYELTQP- PSVSVSPGQIASI TC | SGD---KLGN- ----KYVC | WYQQRPGH AAVLIIY | Q-------- DSKRPS | GIPERFSGSNSG-- NTATLTISGTQAMDEADYFC | QAWDR----------- -----------NTYVV | FGGGT KLTVL |
| iPS:4 72742 | 21-225_30D9_LC 2 | VL3\|3r/JL2 | | SYELTQP- PSVSVSPGQTASI TC | SGD---KLGD- ----KYVY | WFQQKPGQ SPVLVIY | Q-------- DRKRPS | GIPERFSGSNSG-- NTATLTISGTQALDEADYYC | QAWDN----------- -----------STAV | FGGGT KLTVL |
| iPS:4 72743 | 21-225_68G6 | VL3\|3r/JL2 | | SYEVTQP- PSVSVSPGQTASI TC | SGD---KLGD- ----KYTY | WYQQKAGQ SPFLVIY | Q-------- DRKRPS | GIPDRFSGSNSG-- NTATLTISGTQAMDAADFYC | QAWDN----------- -----------STAV | FGGGT KLTVL |
| iPS:4 36652 | 21-225_146B11 | VL3\|3r/JL2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- -----------STVV | FGGGT KLTVL |
| iPS:4 36654 | 21-225_146C11 | VL3\|3r/JL2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- -----------STVV | FGGGT KLTVL |
| iPS:4 36658 | 21-225_146A2 | VL3\|3r/JL2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- -----------STVV | FGGGT KLTVL |
| iPS:4 36664 | 21-225_147E7 | VL3\|3r/JL2 | | SYELTQP- PSVSVSPGQTASI TC | SGD----KLGD- ----KYAS | WYQQKPGQ SPVLVIY | Q-------- DSKRPS | GIPERFSGSNSG-- NTATLTISGIQAMDEADYYC | QAWDS----------- -----------STVV | FGGGT KLTVL |
| iPS:4 36666 | 21-225_147B8 | VL3\|3r/JL2 | | SYELTQP- PSVSVSPGQTASI TC | SGD---KLGD- ----KYVC | WYQQKPGQ SPELVIY | Q-------- DRKRPS | GIPERFSGSNSG-- NTATLTISGTQAMDEADYYC | QAWGS----------- -----------NTAVV | FGGGT KLTVL |
| iPS:4 36668 | 21-225_147B9 | VL3\|3r/JL2 | | SYELTQP- PSVSVSPGQTASI TC | SGD---KLGD- ----KYVS | WYQQRPGQ SPVLVIY | Q-------- DRKRPS | GIPERFSGSNSG-- NTATLTISGILAVDEADYYC | LAWDS----------- -----------STFVV | FGGGT KLTVL |
| iPS:4 36670 | 21-225_147D9 | VL3\|3r/JL2 | | SYELTQP- PSVSVSPGQTASI TS | SGD---KLGN- ----KYVC | WYQQRPGQ SPVLVIY | Q-------- DSKRPS | GIPERFSGSNSG-- NTATLTISGTQAMDEADYYC | QAWDR----------- -----------NTYVV | FGGGT KLTVL |

| | | | | | | | | | | |
|---|---|---|---|---|---|---|---|---|---|---|
| iPS:4 36672 | 21-225_147F9 | VL3\|3r/JL 2 | | SYELTQP- PSVSVSPGQTASI TC | SGD---ELGN- ----KYAC | WYQQKPGQ SPVLVIY | Q-------- DSKRPS | GIPERFSGSNSG-- NTATLTISGTQAMDEADYYC | QAWHS----------- -----------STVV | FGGGT KLTVL |
| iPS:4 36674 | 21-225_147G9 | VL3\|3r/JL 2 | | SYELTQP- PSVSVSPGQTASI TC | SGD---KLGD- ----KYAC | WYQQKPGQ SPVLVIY | Q-------- DRKRPS | GIPERFSGSNSG-- NTATLTISGTQAMDEADYYC | QAWHS----------- -----------STVV | FGGGT KLTVL |
| iPS:4 36676 | 21- 225_147E11 | VL3\|3r/JL 2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- -----------STVV | FGGGT KLTVL |
| iPS:4 36678 | 21- 225_147B12 | VL3\|3r/JL 2 | | SYELTQP- PSVSVSPGQTASI TC | SGD---KLGD- ----KYAS | WYQQKPGQ SPVLVIY | Q-------- DSKRPS | GIPERFSGSNSG-- NAATLTISGIQAMDEADYYC | QAWDS----------- -----------STVV | FGGGT KLTVL |
| iPS:4 36686 | 21-225_148G6 | VL3\|3r/JL 2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- -----------STVV | FGGGT KLTVL |
| iPS:4 36688 | 21-225_148C8 | VL3\|3r/JL 2 | | SYELTQP- PSVSVSPGQTASI TC | SGD---KLGD- ----KYVS | WYQQKPGQ SPILVIY | Q-------- DRKRPS | GTPERFSGSNSG-- NTATLTISGIQAMDEADYYC | LAWDS----------- -----------STFVV | FGGGT KLTVL |
| iPS:4 36690 | 21-225_148A9 | VL3\|3r/JL 2 | | SYELTQP- PSVSVSPGQTASI TC | SGD---KLGN- ----KYVC | WYQQKPGQ SPVLVIY | Q-------- DSKRPS | GIPERFSGSNSG-- NTATLTISGIQAMDEADYYC | QAWHS----------- -----------STVV | FGGGT KLTVL |
| iPS:4 36694 | 21- 225_148G11 | VL3\|3r/JL 2 | | SYELTQP- PSVSVSPGQTASI TC | SGD---KLGD- ----KFAS | WYQQKPGQ SPVLVIY | Q-------- DSKRPS | GIPERFSGSNSG-- NTATLTISGIQAMDEADYYC | QAWDS----------- -----------STVV | FGGGT KLTVL |
| iPS:4 36698 | 21-225_149B5 | VL3\|3r/JL 2 | | SYELTQP- PSVSVSPGQTASI TC | SGY---KLGY- ----KYVC | WYQQKPGQ SPVLVIF | Q-------- NNQRPS | GIPERFSGSNSG-- NTASLTISGTQAMDEADYYC | QAWDS----------- -----------STVV | FGGGT KLTVL |
| iPS:4 36700 | 21-225_149C7 | VL3\|3r/JL 2 | | SYELTQP- PSVSVSPGQTASI TC | SGN---KLGD- ----KYAS | WYQQKPGQ SPVLVIY | Q-------- DSKRPS | GIPERFSGSKSG-- NTATLTISGIQAMDEADYYC | QAWDS----------- -----------STVV | FGGGT KLTVP |
| iPS:4 36704 | 21- 225_149C10 | VL3\|3r/JL 2 | | SYELTQP- PSVSVSPGQTASI TC | SGD---KLGD- ----KYAS | WYQQKPGQ SPVLVIY | Q-------- DNKRPS | GIPERFSGSNSG-- NTATLTIGGIQAMDEADYYC | QAWDS----------- -----------STVV | FGGGT KLTVL |
| iPS:4 36706 | 21- 225_149A11 | VL3\|3r/JL 2 | | SYELTQP- PSVSVSPGQTASI TC | SGD---KLGN- ----KYVS | WYQQKPGQ SPVLVIY | Q-------- DSRRPS | GIPERFSGSNSG-- NTATLTISGIQAMDEADYYC | LAWDS----------- -----------STFVV | FGGGT KLTVL |
| iPS:4 36708 | 21-225_150D3 | VL3\|3r/JL 2 | | SYELTQP- PSVSVSPGQTASI TC | SGD---ELGN- ----KYAC | WYQQKPGQ SPVLVIY | Q-------- DNKRPS | GIPERFSGSSSG-- NTATLTISGIQAMDEADYYC | QAWHS----------- -----------STVV | FGGGT KLTVL |
| iPS:4 36710 | 21-225_150F6 | VL3\|3r/JL 2 | | SYELTQP- PSVSVSPGQTASI TC | SGD---KLGD- ----KYAS | WYQQKPGQ SPVLVIC | Q-------- DSKRPS | GIPERFSGSNSG-- NTATLTISGIQAMDEADYCC | QAWDS----------- -----------STVV | FGGGT KLTVL |
| iPS:4 36714 | 21- 225_150H11 | VL3\|3r/JL 2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADFYC | QAWDS----------- -----------STVV | FGGGT KLTVL |
| iPS:4 36716 | 21-225_151F3 | VL3\|3r/JL 2 | | SYELTQP- PSVSVSPGQTASI TC | SGD---KLGD- ----KYVC | WYQQKPGQ SPVLVIY | Q-------- DRKRPS | GIPERFSGSNSG-- NTATLTISGTQAMDEADYYC | QAWHS----------- -----------STVV | FGGGT KLTVL |

| iPS:4 36718 | 21-225_151H5 | VL3\|3r/JL 2 | | SYELTQP-PSVSVSPGQTASIAC | SGD---KLGD-----KYAS | WYQQKPGQ SPVLVIY | Q--------DSKRPS | GIPERFSGSNSG--NTATLTISGIQAMDEADYYC | QAWDS----------------------STVV | FGGGT KLTVL |
|---|---|---|---|---|---|---|---|---|---|---|
| iPS:4 36722 | 21-225_151H7 | VL3\|3r/JL 2 | | SYDLTQP-PSVSVSPGQTASITC | SGD---KLGD-----KYAS | WYQQKPGQ SPVLVIY | Q--------DSKRPS | GIPERFSGSNSG--NTATLTISGIQAMDEADYYC | QAWDS----------------------STVV | FGGGT KLTVL |
| iPS:4 36724 | 21-225_151B9 | VL3\|3r/JL 2 | | SYELTQP-PSVSVSPGQTASIAC | SGD---NLGD-----KYAS | WYQQKPGQ SPVLVIY | Q--------DSKRPS | GIPERFSGSNSG--NTATLTISGIQAMDEADYYC | QAWDS----------------------STVV | FGGGT KLTVL |
| iPS:4 36728 | 21-225_152G6 | VL3\|3r/JL 2 | | SYELTQP-PSVSVSPGQTASITC | SGD---KLGD-----KYAS | WYQQKPGQ SPVLVIY | Q--------DSKRPS | GIPERFSGSNSG--NTATLTISGIQALDEADYYC | QAWDN----------------------STVV | FGGGT KLTVL |
| iPS:4 36730 | 21-225_152D7 | VL3\|3r/JL 2 | | SYELTQP-PSVSVSPGQTASITC | SGD---KLGD-----KYAS | WYQQKPGQ SPVLVIY | Q--------DSKRPS | GIPERFSGSNSG--NTATLTISGIQAMDEADYYC | QAWDS----------------------STVV | FGGGT KLTVL |
| iPS:4 36736 | 21-225_153E8 | VL3\|3r/JL 2 | | SYELTQP-PSVSVSPGQTASITC | SGS---KLGN-----KYVC | WYQQRPGQ SPVLVIY | Q--------DNKRPS | GIPERFSGSNSG--NTATLTISGTQAMDEAGYYC | QAWDS---------------------STYVI | FGGGT KLTVL |
| iPS:4 36738 | 21-225_153D9 | VL3\|3r/JL 2 | | SYELTQP-PSVSVSPGQTASITC | SGD---KLGD-----KYAC | WYQQKPGQ SPVLVIY | Q--------DRKRPS | GIPERFSGSNSG--NTATLTISGTQAMDEADYYC | QAWHS----------------------STVV | FGGGT KVTVL |
| iPS:4 36740 | 21-225_154C3 | VL3\|3r/JL 2 | | SYELTQP-PSVSVSPGQTASITC | SGD---KLGD-----KYVC | WYQQKPGQ SPVLVIY | Q--------DRKRPS | GIPERFSGSNSG--NTATLTITGTQAMDEADYYC | QAWHS----------------------STVV | FGGGT KLTVL |
| iPS:4 36742 | 21-225_154C4 | VL3\|3r/JL 2 | | SYELTQP-PSVSVSPGQTARITC | SGD---KLGD-----KYAS | WYQQKPGQ SPVLVIY | Q--------DSKRPS | GIPERFSGSNSG--NTATLTISGIQAMDEADYYC | QAWDS----------------------STVV | FGGGT KLTVL |
| iPS:4 36744 | 21-225_154F4 | VL3\|3r/JL 2 | | SYELTQP-PSVSVSPGQTASITC | SGD---KLGN-----KYVC | WYQQKPGQ SPVEVIY | K--------DSKRPS | GIPERFSGSNSG--NTGTLTISGTQAMDEADYYC | QAWDN----------------------STLV | FGGGT KLTVL |
| iPS:4 36746 | 21-225_154E10 | VL3\|3r/JL 2 | | SYELTQP-PSVSVSPGQTASITC | SGD---KLGD-----KYAS | WYQQKPGQ SPVLVIY | Q--------DSKRPS | GIPERFSGSNSG--NTATLTISGIQAMDEADYYC | QAWDN----------------------STVV | FGGGT KLTVL |
| iPS:4 36748 | 21-225_154D11 | VL3\|3r/JL 2 | | SYELTQP-PSVSVSPGQTASITC | SGD---KLGD-----KYAC | WYQQKPGQ SPVLVIY | Q--------DKKRPS | GIPERFSGSNSG--NIATLTISGIQAMDEADYYC | QAWHS----------------------SIVV | FGGGT KLTVL |
| iPS:4 36756 | 21-225_148A10 | VL3\|3r/JL 2 | | SYELTQP-PSVSVSPGQTASITC | SGD---KLGD-----KYVC | WYQQKPGQ SPVLVIF | Q--------DRKRPS | GIPERFSGSNSG--NTATLTISGIQAMDEADYYC | QAWDS----------------------STVV | FGGGT KVTVL |
| iPS:4 36758 | 21-225_155C10 | VL3\|3r/JL 2 | | SYELTQP-PSVSVSPGQTVSITC | SGD---KLGD-----KYVS | WYQQKPGQ SPVLVIY | Q--------DSKRPS | GIPERFSGSNSG--NTATLTISGIQAMDEADYYC | QAWDS----------------------STVV | FGGGT KLTVL |
| iPS:4 36760 | 21-225_155E10 | VL3\|3r/JL 2 | | SYELTQP-PSVSVSPGQTASITC | SGD---KLGD-----KYVS | WYQQKPGQ SPVLVIY | Q--------DRKRPS | GTPERFSGSNSG--NTATLTISGIQAMDEADYYC | LAWDS---------------------STFVV | FGGGT KLTVL |
| iPS:4 36764 | 21-225_158E9 | VL3\|3r/JL 2 | | SYDLTQP-PSVSVSPGQTASITC | SGD---KLGD-----KYVC | WYQQKPGQ SPVLVIY | Q--------DSKRPS | GIPERFSGSNSG--NTATLTITGTQAMDEANYYC | QAWDN---------------------SSFVL | FGGGT KLTVL |

| | | | | | | | | | | |
|---|---|---|---|---|---|---|---|---|---|---|
| iPS:4 36766 | 21-225_158D10 | VL3\|3r/JL2 | | SYELTQP-PSVTVSPGQTASITC | SGD---KLGD-----KYVC | WYQQKPGQSPVLVIY | Q--------DRKRPS | GIPERLSGSNSG--NTATLTISGTQALDEADYYC | QAWGN-----------------------SSFVV | FGGGTKLTVL |
| iPS:4 36768 | 21-225_159H8 | VL3\|3r/JL2 | | SYELTQP-PSVSVSPGQTASITC | SGD---KLGD-----KYVC | WYQQKPGQSPVLVIY | Q--------DRKRPS | GIPERFSGSNSG--NTATLTISGTQALDEADYYC | QAWGN-----------------------SSFVV | FGGGTKLTVL |
| iPS:4 36770 | 21-225_160B12 | VL3\|3r/JL2 | | SDELTQS-PSVSVSPGQTASITC | SGD---KLGD-----KYVC | WYQQKPGQSPVLVIY | Q--------DSKRPS | GIPERFSGSNSG--NTATLTISGTQALDEADYYC | QAWGN-----------------------SSFVV | FGGGTKLTVL |
| iPS:4 36772 | 21-225_161H3 | VL3\|3r/JL2 | | SYELTQP-PSVSVSPGQTASITC | SGD---RLGD-----KYVC | WYQQKPGQSPVLVIF | Q--------DNKRPS | GIPERFSGSNSG--NTATLTISGTQALDEADYYC | QAWVN-----------------------NTAVV | FGGGTKLTVL |
| iPS:4 36774 | 21-225_161E10 | VL3\|3r/JL2 | | SFDLTQP-PSVSVSPGQTASITC | SGD---KLGD-----KYVC | WYQQKPGQSPVLVIY | Q--------DSKRPS | GIPERISGSNSG--NTATLTISGTQAMDEADYYC | QTWDN-----------------------SSFAL | FGGGTKLTVL |
| iPS:4 36776 | 21-225_161F12 | VL3\|3r/JL2 | | SYELTQP-PSVSVSPGQTASITC | SGD---KLGD-----KYAC | WYQQKPGQSPVLVIY | Q--------DTKRPS | GIPERFSGSNSG--NTATLTISGTQAMDEADYYC | QAWDS----------------------TTLV | FGGGTKLTVL |
| iPS:4 36780 | 21-225_165H3 | VL3\|3r/JL2 | | SYELTQP-PSVSVSPGQTASITC | SGD---KLGD-----KYAC | WYQQKPGQSPVLVIY | Q--------DSKRPS | GIPERFSGSNSG--NTATLTISGTQAMDEADYYC | QAWDS----------------------TTLV | FGGGTKLTVL |
| iPS:4 36782 | 21-225_166G11 | VL3\|3r/JL2 | | SYELSQP-PSVSVSPGQTASITC | SGD---KLGD-----KYVH | WYQQKPGQSPVLVIY | Q--------DSKRPS | GIPERFSGSNSG--NTATLTISGTQAMDEADYYC | QAWDN---------------------STAV | FGGGTKLTVL |
| iPS:4 36784 | 21-225_169C1 | VL3\|3r/JL2 | | SYELTQP-PSVSVSPGQTASITC | SGD---KLGD-----KYVC | WYQQKPGQSPLLVIY | K--------DIKRPS | GIPERFSGSNSG--NTATLTISGTQAMDEADYYC | QAWDT---------------------NTVI | FGGGTKLTVL |
| iPS:4 36786 | 21-225_169A6 | VL3\|3r/JL2 | | SYELTQP-PSVSVSPGQTASITC | SGD---KLGD-----KYVC | WYQRKPGQSPVLVIY | Q--------DYKRPS | GIPERFSGSNSG--NTATLTISGTQAMDEADYYC | QAWDT----------------------NTVL | FGGGTKLTVL |
| iPS:4 36788 | 21-225_169B7 | VL3\|3r/JL2 | | AYDLTQP-PSVSVSPGQTARITC | SGD---KLGG-----KYAS | WYQQKPGQSPVLVIY | Q--------DRKRPS | GIPERFSGSNSG--NTATLTISGTQAMDEADYYC | QAWDK----------------------NTVV | FGGGTKLTVL |
| IPS:4 36790 | 21-225_169G11 | VL3\|3r/JL2 | | SYELTQP-PSVSVSPGQTASITC | SGD---KLGD-----KYAC | WYQQKPGQSPVLVIY | Q--------DSKRPS | GIPERFSGSNSG--NTATLTISGTQAMDEADYYC | QAWDN----------------------STAV | FGGGTKLTVL |
| iPS:4 36794 | 21-225_170F1 | VL3\|3r/JL2 | | SYELSQP-PSVSVSPGQTASITC | SGD---KLGD-----KYSC | WYQQKPGQSPVLVIY | Q--------DSKRPS | GIPARFSGSNSG--NTATLTISGTQAMDEADYYC | QAWDS----------------------NTAV | FGGGTKLTVL |
| iPS:4 36796 | 21-225_170A5 | VL3\|3r/JL2 | | SYDLTQP-PSVSVSPGQTASITC | SGD---KLGD-----KYAC | WYQQKPGQSPILVIY | Q--------DYKRPS | GIPERFSGSNSG--NTATLTISGTQAMDEADYYC | QAWDN-----------------------STMV | FGGGTRLTVL |
| iPS:4 36798 | 21-225_171F5 | VL3\|3r/JL2 | | AYDLTQP-PSVSVSPGQTASITC | SGD---KLGG-----KYAS | WYQQKPGQSPVLVIY | Q--------DRKRPS | GIPERFSGSNSG--NTATLTISGTQAMDEADYYC | QAWDK----------------------NTVV | FGGGTKLTVL |
| iPS:4 36802 | 21-225_171E12 | VL3\|3r/JL2 | | SYELTQP-PSVSVSPGQTASITC | SGD---KLGD-----KYAC | WYQQKPGQSPVLVIY | Q--------DRKRPS | GIPERFSGSNSG--NTATLTISGTQAMDEADYYC | QAWDI----------------------STYVV | FGGGTKLTVL |

| | | | | | | | | | | |
|---|---|---|---|---|---|---|---|---|---|---|
| iPS:4 36808 | 21-225_173F8 | VL3\|3r/JL 2 | | SYELTQP-PSVSVSPGQTASITC | SGN---KLGN-----KYVC | WYQQRPGQ SPVLVIS | Q--------DSRRPS | GIPERFSGSNSG--NTATLTISGTQAMDEADYYC | QAWDS----------------------FTVV | FGGGT KLTVL |
| iPS:4 36812 | 21-225_175C6 | VL3\|3r/JL 2 | | SYDLTQP-PSVSVSPGQTASITC | SGD---KLGD-----KYAC | WYQQKPGQ SPILVIY | Q--------DYKRPS | GIPERFSGSNSG--NTATLTISGTQAMDEADYYC | QAWDN----------------------STMV | FGGGT RLTVL |
| iPS:4 36818 | 21-225_179C7 | VL3\|3r/JL 2 | | SYELTQP-PSVSVSPGQTASITC | SGD---KLGD-----KYVC | WYQQRPGQ SPVLVIY | Q--------DSKRPS | GIPERFSGSNSG--NTATLTIRGTQAMDEADYYC | QAWDS----------------------NTAVV | FGGGT KLTVL |
| iPS:4 36822 | 21-225_180D4 | VL3\|3r/JL 2 | | SYELTQT-PSVSVSPGQTASITC | SGD---RLGD-----KYAC | WYQQKPGQ SPVLVIY | E--------DRKRPS | GIPERFSGSNSG--NTATLTISGTQAMDEGDYYC | QAWDS----------------------RKVV | FGGGT KLTVL |
| iPS:4 36824 | 21-225_180C5 | VL3\|3r/JL 2 | | SYELTQP-PSVSVSPGQTASITC | SGD---KLGE-----KYAC | WYQQKPGQ SPVLVIY | Q--------DRKRPS | GIPERFSGSNSG--NTATLTISGTQAMDEADYYC | QAWDS----------------------STAV | FGGGT KLTVL |
| iPS:4 36826 | 21-225_180G5 | VL3\|3r/JL 2 | | SYELTQP-PSVSVYPGQTASITC | SGD---KLGD-----KYVS | WYQQKPGQ SPVLVIY | Q--------DSKRPS | GIPERFSGSNSG--NPASLTISGTQAMDEADYYC | QAWDI----------------------TTAV | FGGGT KLTVL |
| iPS:4 36828 | 21-225_181H1 | VL3\|3r/JL 2 | | SYELTQT-PSVSVSPGQTASITC | SGD---KLGD-----KYAC | WYQQKPGQ SPVLVIY | E--------DRKRPS | GIPERFSGSNSG--NTATLTISGTQAMDEADYYC | QAWDS----------------------RKVV | FGGGT KLTVL |
| iPS:4 36836 | 21-225_52H1 | VL3\|3r/JL 2 | | SYELTQP-PSVFVSPGQTASITS | SGD---KLGD-----KYVS | WYQQKPGQ SPVLVIY | Q--------DRKRPS | GIPERFSGSNSG--NTATLTISGTQAMDEADYYC | QAWDN----------------------STVV | FGGGT KLTVL |
| iPS:4 36848 | 21-225_57F1 | VL3\|3r/JL 2 | | SYELTQP-PSASVSPGQTASITC | SGD---KLGE-----KYAC | WYQQKPGQ SPVLVIY | Q--------DRKRPS | GIPERFSGSNSG--NTATLTISGTQAMDEADYYC | QAWDS----------------------STVV | FGGGT KLTVL |
| iPS:4 36850 | 21-225_57D9 | VL3\|3r/JL 2 | | SYELTQP-PSVSVSPGQTASITC | SGE---KLGE-----KFAC | WSQQKPGQ SPVLVIY | Q--------DSKRPS | GIPERFSGSNSG--NTATLTISGTQAMDEADYYC | QAWDS----------------------STVV | FGGGT KLTVL |
| iPS:4 36852 | 21-225_57H11 | VL3\|3r/JL 2 | | SYALTQP-PSASVSPGQTASITC | SGD---KLGE-----KYAC | WYQQKPGQ SPVLVIY | Q--------DRKRPS | GIPERFSGSNSG--NTATLTISGTQAMDEADYYC | QAWDS----------------------STVV | FGGGT KLTVL |
| iPS:4 36854 | 21-225_58C1 | VL3\|3r/JL 2 | | SYELTQP-PSVSVSPGQTANITC | SGD---KLGN-----KYAC | WYQQKPGQ SPVLVIY | Q--------DRKRPS | GIPERFSGSNSG--NTATLTISGTQAMDEADYYC | QAWD-----------------------SSTA | FGGGT KLTVL |
| iPS:4 36858 | 21-225_58E7 | VL3\|3r/JL 2 | | SYELTQS-PSVSVSPGQTASITC | SGD---KLGD-----KYTC | WYQKKPGQ SPVLVIY | Q--------DNKRPS | GIPERFSGSNSG--NTATLTISGTQAMDEADYFC | QAWNN----------------------YTVV | FGGGT KLTAL |
| iPS:4 36860 | 21-225_58F7 | VL3\|3r/JL 2 | | SYELTQP-PSVSVSPGQTASITC | SGD---KLGD-----KYAC | WYQQKPGQ SPVLVIY | Q--------DRKRPS | GIPERFSGSNSG--NTATLTISGTQAMDEADYYC | QAWDS----------------------STVV | FGGGT KLTVL |
| iPS:4 36862 | 21-225_58F8 | VL3\|3r/JL 2 | | SYELTQP-PSVSVSPGQTASITC | SGD---KLGN-----KYAC | WYQQKPGQ SPVLVIY | Q--------DSKRPS | GIPERFSGSNSG--NTATLTISGTQAMDEADYYC | QAWDS----------------------STVV | FGGGT KLTVL |
| iPS:4 36864 | 21-225_58G11 | VL3\|3r/JL 2 | | SYELTQP-PSVSVSPGQTASITC | SGD---KLGD-----KYAS | WYQQKPGQ SPVLVIY | Q--------DNKRPS | GIPERFSGSNSG--NTATLTISGTQAMDEADYYC | QAWNN----------------------NTVM | FGGGT KLTVL |

| iPS:4 36866 | 21-225_59F2 | VL3\|3r/JL 2 | | SYELTQP-PSVSVSPGQTASITC | SGD---KLGD-----KYAS | WYQQRPGQ SPVLVIY | Q--------DNKRPS | GIPERFSGSNSG--NTATLTISGTQAMDEADYYC | QAWDN---------------------NTVV | FGGGT KLTVL |
| iPS:4 36868 | 21-225_59B11 | VL3\|3r/JL 2 | | SYELTQP-PSVSVSPGQTASITC | SGD---KLGD-----KYAC | WYQQKPGQ SPVLVIY | Q--------DSKRPS | GILERFSGSNSG--NTATLTISGTQAMDEADYYC | QAWDS---------------------STYVV | FGGGT KLTVL |
| iPS:4 36870 | 21-225_60B1 | VL3\|3r/JL 2 | | SYELTQP-PSASVSPGQTASITC | SGD---KLGE-----KYAC | WYQQKPGQ SPVLVIY | Q--------DRKRPS | GIPERFSGSNSG--NTATLTISGTQAMDEADYYC | QAWDS---------------------STVV | FGGGT KLTVL |
| iPS:4 36872 | 21-225_60D2 | VL3\|3r/JL 2 | | SYELTQP-PSVSVSPGQTASITC | SGN---KLGD-----KYAS | WYQQRPGQ SPVLVIY | Q--------DNKRPS | GIPERFSGSNSG--NTATLTISGTQTMDEADYYC | QAWDN---------------------NTVV | FGGGT KLTVL |
| iPS:4 36874 | 21-225_60A12 | VL3\|3r/JL 2 | | SYELTQP-PSVSVSPGQTANITC | SGD---KLGN-----KYAC | WYQQKPGQ SPVLVIY | Q--------DRKRPS | GIPERFSGSNSG--NTAILTISGTQAMDEADYYC | QAWD---------------------SSTA | FGGGT KLTVL |
| iPS:4 36876 | 21-225_61F5 | VL3\|3r/JL 2 | | SYELTQP-PSVSVSPGQTASITC | SGD---KLGE-----KYAC | WYQQKPGQ SPVLVIY | Q--------DSKRPS | GIPERFSGSNSG--NTATLTISGTQAMDEADYYC | QAWDS---------------------STVV | FGGGT KLTVL |
| iPS:4 36878 | 21-225_62E3 | VL3\|3r/JL 2 | | SYDLTQP-PSVSVSPGQTASITC | SGD---KLGN-----KYAC | WYQQKPGQ SPVVVIY | Q--------DRKRPS | GIPERFSGSNSG--NTATLTISGTQAMDEADYYC | QAWDS---------------------STAV | FGGGT KLTVL |
| iPS:4 36880 | 21-225_62E8 | VL3\|3r/JL 2 | | SYDLTQP-PSVSVSPGQTASITC | SGD---RLGN-----KYAS | WYQQKPGQ SPVVVIY | Q--------DRKRPS | GIPERFSGSNSG--NTATLTISGTQAMDEADYYC | QAWDS---------------------STAV | FGGGT KLTVL |
| iPS:4 36882 | 21-225_62D10 | VL3\|3r/JL 2 | | SYDLTQP-PSVSVSPGQTASITC | SGD---KLGN-----KYAC | WYQQKPGQ SPVVVIY | Q--------DRKRPS | GIPERFSGSNSG--NTATLTISGTQAMDEADYYC | QAWDS---------------------STAV | FGGGT KLTVL |
| iPS:4 36884 | 21-225_62A12 | VL3\|3r/JL 2 | | SYDLTQP-PSVSVSPGQTASITC | SGD---KLGN-----KYAC | WYQQKPGQ SPVVVIY | Q--------DRKRPS | GIPERFSGSNSG--NTATLTISGTQAMDEADYYC | QAWDS---------------------STAV | FGGGT KLTVL |
| iPS:4 36886 | 21-225_62B12 | VL3\|3r/JL 2 | | SYDLTQP-PSVSVSPGQTASITC | SGD---KLGN-----KYTC | WYQQKPGQ SPVVVIY | Q--------DRKRPS | GIPERFSGSNSG--NTATLTISGTQAMDEADYYC | QAWDS---------------------STAV | FGGGT KLTVL |
| iPS:4 36892 | 21-225_65E9 | VL3\|3r/JL 2 | | SYELTQP-PSVSVSPGQTASITC | SGD---KLGN-----KYDY | WYQQKPGQ SPVLVIY | Q--------DRKRPS | GIPERFSGSNSG--NTATLTISGTQAMDEADYYC | QAWDN---------------------STVV | FGGGT KLTVL |
| iPS:4 36894 | 21-225_66G9 | VL3\|3r/JL 2 | | SYDLTQP-PSVTVSPGQTASITC | SGD---KLGN-----KYAC | WYQQKPGQ SPVVVIY | Q--------DRKRPS | GIPERFSGSNSG--NTATLTISGTQAMDEADYYC | QAWDI---------------------NTAV | FGGGT KLTVL |
| iPS:4 36896 | 21-225_67F10 | VL3\|3r/JL 2 | | SYELTQP-PSVSVSPGQTASITC | SGD---KLGY-----KYAW | WYQQKPGQ SPVLVIF | E--------DRKRPS | GIPERFSGSNSG--NTATLTISGTQAMDEADYYC | QAWDN---------------------STVV | FGGGT RLTVL |
| iPS:4 36898 | 21-225_68D8 | VL3\|3r/JL 2 | | SYELTQP-PSVSVSPGQTASITC | SGD---KLGD-----KYAC | WYQQKPGQ SPVLVIY | Q--------DSKRPS | GIPERFSGSNSG--NTATLTISGTQAMDEADYYC | QAWDN---------------------STVV | FGGGT KLTVL |
| iPS:4 36900 | 21-225_69B9 | VL3\|3r/JL 2 | | SYELTQP-PSVSVSPGQTASITC | SGD---KLGN-----KYDY | WYQQKPGQ SPVLVIY | Q--------DRKRPS | GIPERFSGSNSG--NTATLTISGTQAMDEADYYC | QAWDN---------------------STVV | FGGGT KLTVL |

| | | | | | | | | | | |
|---|---|---|---|---|---|---|---|---|---|---|
| iPS:4 36902 | 21-225_69B11 | VL3l3r/JL 2 | | SYELTQP-PSVSVSPGQAASITC | SGD---KLGD-----KYAW | WYQQKPGQ SPVLVIY | E--------DRKRPS | GIPERFSGSNSG--NTATLTISGTQAMDEADYYC | QAWDN----------------------STVV | FGGGT KLTVL |
| iPS:4 36904 | 21-225_71D4 | VL3l3r/JL 2 | | SYELTQP-PSVSVSPGQIASITC | SGD---KLGD-----KYAY | WYQQKPGQ SPVVVIY | Q--------DRKRPS | GIPERFSGSNSG--NTATLTISGTQAMDEADYYC | QAWVN----------------------STVV | FGGGT KLTVL |
| iPS:4 36906 | 21-225_72B4 | VL3l3r/JL 2 | | SYELTQP-PSVSVSPGQTASITC | SGD---KLGD-----KYAW | WYQQRPGQ SPVLVIY | E--------DRKRPS | GIPERFSGSNSG--NTATLTISGTQAMDEADYYC | QAWDN----------------------STVV | FGGGT KLTVL |
| iPS:4 36908 | 21-225_72D5 | VL3l3r/JL 2 | | SYDLTQP-PSVSVSPGQTASITC | SGD---KLGN-----KYAC | WYQQKPGQ SPVVVIY | Q--------DRKRPS | GIPERFSGSNSG--NTATLTISGTQAMDEADYYC | QAWDS----------------------STAV | FGGGT KLTVL |
| iPS:4 36912 | 21-225_73C4 | VL3l3r/JL 2 | | SYDLTQP-PSVSVSPGQTASITC | SGD---KLGN-----KYAC | WYQQKPGQ SPVVVIY | Q--------DMKRPS | GIPERFSGSNSG--NTATLTISGTQAMDEADYYC | QAWDS----------------------STAV | FGGGT KLTVL |
| iPS:4 36914 | 21-225_76B4 | VL3l3r/JL 2 | | PYELNQT-PSVSVSPGQTASITC | SGD---RLGT-----KFAC | WYQQKPGQ SPVLVIY | Q--------DSKRPS | GIPERFSGSNSG--NTATLTISGTQAMDEADYYC | QAWD----------------------SSTV | FGGGT KLTVL |
| iPS:4 36916 | 21-225_74A9 | VL3l3r/JL 2 | | SYELTQP-PSVSVSPGQTASITC | SGD---KLGN-----KYVC | WYQQKPGQ SPVLVIY | Q--------DNRRPS | GIPERFSGSNSG--NTATLTISGTQAMDEADYYC | QAWDS----------------------SPVI | FGGGT KLTVL |
| iPS:4 36918 | 21-225_77A2 | VL3l3r/JL 2 | | SYELTQP-PSVSVSPGQTASITC | SGD---RLGD-----KYAC | WYQQKPGQ SPVLVIY | Q--------DRKRPS | GIPERFSGSNSG--NTATLTISGTQAMDEADYYC | QAWDS----------------------STAV | FGGGT KLTVL |
| iPS:4 36922 | 21-225_78E9 | VL3l3r/JL 2 | | SYELTQP-PSESVSPGQTASITC | SGD---KLGN-----KYVS | WYQQKPGQ SPVLVIY | Q--------DNRRPS | GIPERFSGSNSG--STATLTISGTQAMDEADYYC | QAWDS----------------------SPVI | FGGGT KLTVL |
| iPS:4 36924 | 21-225_74B3 | VL3l3r/JL 2 | | SYELTQP-PSVSVSPGQTASITC | SGD---KLGD-----KYAC | WYQQKPGQ SPVLVIY | Q--------DSKRPS | GIPERFSGSNSG--NTATLTISGTQAMDEADYYC | QAWDS----------------------TTVV | FGGGT KLTVL |
| iPS:4 36928 | 21-225_79E7 | VL3l3r/JL 2 | | SYELTQP-PSVSVSPGQTASITC | SGD---KLGN-----KYVS | WYQQKPGQ SPVLVIY | Q--------DNRRPS | GIPERFSGSNSG--STATLTISGTQAMDEADYYC | QAWDS----------------------SPVI | FGGGT KLTVL |
| iPS:4 36932 | 21-225_92A4 | VL3l3r/JL 2 | | SYELTQP-PSVSVSPGQTANITC | SGD---KLGN-----KYVC | WYQQKPGQ SPVLVIY | Q--------DNRRPS | GIPERFSGSNSG--NTATLTISGTQAMDEADYYC | QAWDS----------------------SPVI | FGGGT KLTVL |
| iPS:4 36934 | 21-225_96B5 | VL3l3r/JL 2 | | PYELNQT-PSVSVSPGQTASITC | SGD---RLGT-----KFAC | WYQQKPGQ SPVLVIY | Q--------DSKRPS | GIPERFSGSNSG--NTATLTISGTQAMDEADYYC | QAWD----------------------SSTV | FGGGT KLTVL |
| iPS:4 36936 | 21-225_97E6 | VL3l3r/JL 2 | | SYELTQP-PSVSVSPGQTVSITC | SGD---KLGN-----KYVS | WYQQKPGQ SPVLVIY | Q--------DNRRPS | GIPERFSGSNSG--STATLTISGTQAMDEADYYC | QAWDS----------------------TPVI | FGGGT KLTVL |
| iPS:4 36938 | 21-225_146A3 | VL3l3r/JL 2 | | SYAMTQP-PSMSVSPGQTASITC | SGN---KLGN-----RYAC | WYQQKPGQ SPVLVIY | Q--------DSKRPS | GIPERFSGSNSG--NIATLTISGTQAMDEADYYC | QAWDS----------------------STVV | FGGGT KLTVL |
| iPS:4 36940 | 21-225_146B8 | VL3l3r/JL 2 | | SYELTQP-PSVSVSPGQTASITC | SGD---KLGD-----KYVC | WYQQKPGQ SPVLVIY | Q--------DRKRPS | GIPERFSGSNSG--NTATLTISGTQAMDEADYYC | QAWHS----------------------STVV | FGGGT KLTVL |

| | | | | | | | | | | |
|---|---|---|---|---|---|---|---|---|---|---|
| iPS:4 36942 | 21-225_146H8 | VL3\|3r/JL 2 | | SYELTQP-PSVSVSPGQTASITC | SGD---KLGD-----KYAC | WYQQKPGQSPVLVIY | Q--------DKKRPS | GIPERFSGSNSG--NTATLTISGTQVMDEADYYC | QAWDI----------------------RTVV | FGGGTKLTVL |
| iPS:4 36944 | 21-225_182D12 | VL3\|3r/JL 2 | | SYELTQP-PSVSVSPGQTASITC | SGD---KLGE-----KYAC | WYQQKSGQSPVLVIY | Q--------DRKRPS | GIPERFSGSNSG--NTATLTISGTQAMDEADYYC | QAWDS----------------------RTAV | FGGGTKLTVL |
| iPS:4 36946 | 21-225_183F4 | VL3\|3r/JL 2 | | SYELTQP-PSVSVSPGQTASITC | SGD---KLGD-----KYAC | WYQQKPGQSPVLVIY | Q--------DKKRPS | GIPERFSGSNSG--NTATLTISGTQAMDEADYYC | QAWDN----------------------STAVV | FGGGTKLTVL |
| iPS:4 36952 | 21-225_185D2 | VL3\|3r/JL 2 | | SYELTQT-PSVSVSPGQTASITC | SGD---KLGD-----KYAC | WYQQKPGQSPVLVIY | E--------DRKRPS | GIPDRFSGSNSG--NTATLTISGTQAMDEADYYC | QAWDS----------------------RKVV | FGGGTKLTVL |
| iPS:4 36954 | 21-225_185G7 | VL3\|3r/JL 2 | | SYELTQP-PSVSVSPGQTASITC | SGD---KLGH-----KFVC | WYQQKPGQSPVLVIY | Q--------DRKRPS | GIPERFSGSNSG--NTATLTISGTQAMDEADYYC | QAWD----------------------SSTV | FGGGTKLTVL |
| iPS:4 36956 | 21-225_186H6 | VL3\|3r/JL 2 | | SYELTQP-PSVSVSPGQTASITC | SGD---KMGE-----KYAC | WYQQKPGQSPVLVIY | Q--------DRKRPS | GIPERFSGSNSG--NTATLTISGTQAMDEADYYC | QAWDS----------------------STAV | FGGGTKLTVL |
| iPS:4 36962 | 21-225_190H1 | VL3\|3r/JL 2 | | SYELTQP-PSVSVSPGQTASITC | SGD---KLGD-----RFAY | WYQQKPGQSPVLVIY | Q--------DSKRPS | GIPERFSGSNSG--NTATLTISGTQAMDEADYYC | KAWDS----------------------STVV | FGGGTKLTVL |
| iPS:4 36978 | 21-225_190G9 | VL3\|3r/JL 2 | | SYELTQP-PSVSVSPGQTASITC | SGD---KLGD-----RFAY | WYQQKPGQSPVLVIY | Q--------DNKRPS | GIPERFSGSNSG--NTASLTISGTQAMDEADYYC | QAWDS----------------------STVV | FGGGTRLTVL |
| iPS:4 37018 | 21-225_193H5 | VL3\|3r/JL 2 | | SYELTQP-SSVSVSPGQTASITC | SGD---KLGD-----RFAC | WYQQKPGQSPVLVIY | Q--------DSKRPS | GIPERFSGSNSG--NTATLTISGTQAMDEADYYC | QAWDS----------------------STAV | FGGGTKLTVL |
| iPS:4 37030 | 21-225_195E3 | VL3\|3r/JL 2 | | SYELTQP-PSVSVSPGQTASITC | SGD---KLGY-----RSVC | WYQQKPGQSPVLVIY | E--------DSKRPS | GIPERFSGSNSG--NTATLTISGTQAMDEADYYC | QAWDS----------------------VTVV | FGGGTKLIVL |
| iPS:4 37034 | 21-225_195E9 | VL3\|3r/JL 2 | | SYELTQP-PSVSVSPGQTASITC | SGD---KLGN-----KYAY | WYQQKPGQSPVLVIY | Q--------DRKRPS | GIPERFSGSNSG--NTGTLTISGTQGMDEADYYC | QAWDR----------------------GIVV | FGGGTKLTVL |
| iPS:4 37070 | 21-225_201G11 | VL3\|3r/JL 2 | | SYELTQP-PSVSVSPGQTASITC | SGD---KLGD-----RFAC | WYQQKPGQSPVLVIY | Q--------DSKRPS | GIPERFSGSNSG--NTATLTISGTQAMDEADYYC | QAWDS----------------------STVV | FGGGTKLTVL |
| iPS:4 37076 | 21-225_203G6 | VL3\|3r/JL 2 | | SYELTQP-PSVSVSPGQTASITC | SGD---KLGD-----RFAC | WYQQKPGQSPVLVIY | Q--------DNKRPS | GIPERFSGSNSG--NTATLTISGTQSMDEADYYC | QAWDS----------------------STVV | FGGGTKLTVL |
| iPS:4 37144 | 21-225_215B3 | VL3\|3r/JL 2 | | SYELTQP-PSVSVSPGQTASITC | SGD---KLGD-----KFAC | WYQQKPGQSPVLVIY | Q--------DSKRPS | GIPERFSGSNSG--NTATLTISGTQAMDEADYYC | QAWDS----------------------STVV | FGGGTKLTVL |
| iPS:4 37186 | 21-225_224H2 | VL3\|3r/JL 2 | | SYELTQP-SSVSVSPGQTASITC | SGD---NLGV-----KYTY | WYQQKPGQSPVLVVY | Q--------DSKRPS | GIPERFSGSNSG--NTATLTISGTQAMDEADYYC | QAWDS----------------------STVV | FGGGTKLTVL |
| iPS:4 37192 | 21-225_225E9 | VL3\|3r/JL 2 | | SYDLTQP-PSVSVSPGQTASITC | SGD---NLGN-----RYAC | WYQQKPGQSPVLVMY | Q--------DRKRPS | GIPERFSGSNSG--NTATLTISGTQAMDEADYYC | QAWDS----------------------RTAVV | FGGGTKLTVL |

| | | | | | | | | | | |
|---|---|---|---|---|---|---|---|---|---|---|
| iPS:4 37194 | 21-225_226B2 | VL3I3r/JL 2 | | SYELTQP-PSVSVSPGQTASI TC | SGD---TLGG-----KYAW | WYQQRPGQ SPVLVIY | Q--------DRKRPS | GIPERFSGSSSG--NTATLTISGTQAMDEADYYC | QAWDN--------------------GAAV | FGGGT KLTVL |
| iPS:4 37200 | 21-225_226A10 | VL3I3r/JL 2 | | SYELTQP-PSVSVSPGQTASI TC | SGD---TLGG-----KYAW | WYQQKPGQ SPVLVIY | Q--------DRKRPS | GIPERFSGSSSG--NTATLTISGTQAMDEADYYC | QAWDN--------------------GAAV | FGGGT KLTVL |
| iPS:4 37204 | 21-225_227E5 | VL3I3r/JL 2 | | SYELSQP-PSVSVSPGQTASI TC | SGD---KLGE-----KYAC | WYQQKPGQ SPVLVIY | Q--------DRKRPS | GIPERFSGSNSG--NKATLTISGTQAMDEADYYC | QAWVN--------------------NTMI | FGGGT KLTVL |
| iPS:4 37210 | 21-225_227E12 | VL3I3r/JL 2 | | SYELTQP-PSVSVSPGQTASI TC | SGD---KLGD-----KYVC | WYQQKPGQ SPVLVIY | Q--------DSKRPS | GIPERFSGSNSG--NTATLTISGTQAMDEADYYC | QAWNS--------------------SNVV | FGGGT KLTVL |
| iPS:4 48908 | 21-225_50G9 | VL3I3r/JL 2 | | SYELTQP-PSVSVSPGQTASI TC | SGD---KLGD-----KYAC | WYQQKPGQ SPVLVIY | Q--------DSKRPS | GIPERFSGSNSG--NTATLTISGTQARDEAEYYC | QARNS--------------------RRGV | FGGGT RLTVL |
| iPS:4 51102 | 21-225_45F6 | VL3I3r/JL 2 | | SYELTQP-PSVSVSPGQTASI TC | SGD---KLGD-----KYAS | WYQQKPGQ SPVLVIY | Q--------DSKRPS | GIPERFSGSNSG--NTATLTISGTQAMDEADYYC | QAWDN--------------------RTMV | FGGGT KLTVL |
| iPS:4 51110 | 21-225_74C9 | VL3I3r/JL 2 | | SYELTQP-PSESVSPGQTASI TC | SGD---KSGN-----KYVS | WYQQKPGQ SPVLVIY | Q--------DNRRPS | GIPERFSGSNSG--STATLTISGTQAMDEADYYC | QAWDS--------------------TPVI | FGGGT KLTVL |
| iPS:4 51112 | 21-225_53D10 | VL3I3r/JL 2 | | SYELTQP-PSVSVSPGQTASI TC | SGD---KLGN-----KYAC | WYQQRPGQ SPVLVIY | Q--------DRKRPS | GIPERFSGSNSG--NTATLTISGTQAMDEADYYC | QAWDS--------------------STVV | FGGGT KLTVL |
| iPS:4 72731 | 21-225_14B1_LC 2 | VL3I3r/JL 2 | | SFELTQP-PSVSVSPGQTASI TC | SGD---KLGD-----KYAY | WYQQKPGQ SPVLVIY | Q--------DRKRPS | GIPERFSGSNSG--NTATLTISGTQAMDEADYYC | QAWDN--------------------STVV | FGGGT KLTVL |
| iPS:3 92583 | 21-225_10B10 | VL3I3r/JL 2 | | SYELTQP-PSVSVSPGQTASI TC | SGD---KLGN-----KYAW | WYQQKPGQ SPVLVIY | Q--------DRKRPS | GIPERFSGSNSG--NTATLTISGTQAMDEADYYC | QAWDN--------------------STVV | FGGGT KLTVL |
| iPS:3 92585 | 21-225_14H11 | VL3I3r/JL 2 | | TYELTQP-SSVSVSPGQTASI TC | SGD---KLGE-----KYVC | WYQQKPGQ SPVLVIY | Q--------DTKRPS | GIPERFSGSNSG--NTATLTISGTQAMDEADYYC | QAWD--------------------SSTI | FGGGT KLTVL |
| iPS:3 92587 | 21-225_18G5 | VL3I3r/JL 2 | | SYELTQP-PSVSVSPGQTASI TC | SGE---KLGD-----KYVC | WYQQKPGQ SPVLVIY | Q--------DSKRPS | GIPERFSGSNSG--NTATLTISGTQAMDEADYYC | QAWNS--------------------SNVV | FGGGT KLTVL |
| iPS:3 92589 | 21-225_27H2 | VL3I3r/JL 2 | | SYELTQP-PSVSVSPGQTASI TC | SGD---KLGD-----KYAS | WYQQKPGQ SPVLVIY | Q--------DGKRPS | GIPERFSGSNSG--NTATLTLSGTQAMDEADYYC | QAWDS--------------------STYVV | FGGGT KLTVL |
| iPS:3 92598 | 21-225_18E10 | VL3I3r/JL 2 | | SYELTQP-PSVSVSPGQTASI TC | SGD---RLGD-----KYAW | WYQQKPGQ SPVLVIY | Q--------DRKRPS | GIPERFSGSNSG--NTATLTISGTQAMDEADYYC | QAWDS--------------------STVV | FGGGT KLTVL |
| iPS:3 93166 | 21-225_27G6 | VL3I3r/JL 2 | | SYELTQP-PSVSVSPGQTASI TC | SGD---KLGD-----KYAC | WYQQKPGQ SPVLVIY | Q--------DRKRPS | GIPERFSGSNSG--NTATLTISGTQTMDEADYYC | QAWDS--------------------SSYVV | FGGGT KLTVL |
| iPS:3 93168 | 21-225_32B11 | VL3I3r/JL 2 | | SYELTQP-PSVSVSPGQTASI TC | SGD---KLGD-----KYAY | WYQQKPGQ SPVLVIY | Q--------DSKRSS | GIPERFSGSNSG--NTATLTISGTQAMDEADYYC | QAWDN--------------------STVV | FGGGT KLTVL |

| | | | | | | | | | | |
|---|---|---|---|---|---|---|---|---|---|---|
| iPS:3 93172 | 21-225_3B12 | VL3\|3r/JL 2 | | SYELSQP-PSVSVSPGQTASI TC | SGD---KLGE-----KYAC | WYQQKPGQ SPVLVIY | Q--------DRKRPS | GIPERFSGSNSG--NKATLTISGTQAMDEADYYC | QAWVN------------------NTMI | FGGGT KLTVL |
| iPS:3 93176 | 21-225_27E7 | VL3\|3r/JL 2 | | SYELTQP-PSVSVSPGQTASI TC | SGD---KLGN-----KYAC | WYQQKPGQ SPVEVIY | Q--------DSKRPL | GIPERFSGSNSG--NTATLTISGTQAMDEADYYC | QAWDS------------------STVV | FGGGT KLTVL |
| iPS:3 93178 | 21-225_34D7 | VL3\|3r/JL 2 | | SYELTQP-PSVSVSPGQTASI TC | SGD---KLGE-----KYAY | WYQQKPGQ SPVLVLY | Q--------DSKRPS | GIPERFSGSNSG--NTATLTISGTQIMDEADFYC | QAWDN------------------TTVV | FGGGT KLTVL |
| iPS:3 93182 | 21-225_4B3 | VL3\|3r/JL 2 | | SYELTQP-PSVSVSPRQTVSI TC | SGD---KLGD-----KYAC | WYQQKPGQ SPVLVIY | Q--------DRKRPS | GIPERFSGSNSG--NTATLTISGTQAMDEADYYC | QAWDN------------------NTVI | FGGGT KLTVL |
| iPS:3 93184 | 21-225_15H11 | VL3\|3r/JL 2 | | SYELTQP-PSVSVSPGQTASI TC | SGD---KLGE-----KYAC | WYQQKPGQ SPVVVIY | Q--------DRKRPS | GIPERFSGSNSG--NTATLTISGTQAIDEADYYC | QAWDS------------------STAV | FGGGT KLTVL |
| iPS:3 93186 | 21-225_27D9 | VL3\|3r/JL 2 | | SYELTQP-PSMSVSPGQTASI TC | SGY---KLGD-----KYAC | WFQQKPGQ SPVLVIY | Q--------DSKRPS | GIPERFSGSNSG--NTATLTISGTQAMDEADYYC | QAWV-------------------NNTV | FGGGT KLTVL |
| iPS:3 93188 | 21-225_34B9 | VL3\|3r/JL 2 | | SYELTQA-PSVSVSPGQTASI TC | SGD---KLGE-----KYVS | WYQEKPGQ SPVLVIY | Q--------DSKRPS | GIPERFSGSNSG--NTATLTISGTQAMDEADYYC | QAWD-------------------SSTV | FGGGT KLTVL |
| iPS:3 93192 | 21-225_12B1 | VL3\|3r/JL 2 | | SYELTQP-PSVSVSPRQTVSI TC | SGD---KLGD-----KYAC | WYQQKPGQ SPVLVIY | Q--------DRKRPS | GIPERFSGSNSG--NTATLTISGTQAMDEADYYC | QAWDN------------------NTVI | FGGGT KLTVL |
| iPS:3 93194 | 21-225_16D2 | VL3\|3r/JL 2 | | SYELTQP-PSVSVSPGQTASI TC | SGD---KLGD-----KYAC | WYQQKPGQ SPVLVIY | Q--------DSKRPS | GIPERFSGSNSG--NTATLTISGTQAMDEADYYC | QAWDS------------------STYVV | FGGGT KLTVL |
| iPS:3 93196 | 21-225_16G8 | VL3\|3r/JL 2 | | SYELSQP-PSVSVSPGQTASI TC | SGD---KLGE-----KYAC | WYQQKPGQ SPVLVIY | Q--------DRKRPS | GIPERFSGSNSG--NKATLTISGTQAMDEADYYC | QAWVN------------------NTMI | FGGGT KLTVL |
| iPS:3 93198 | 21-225_28A11 | VL3\|3r/JL 2 | | SYELTQP-PSVSVSPGQTASI TC | SGD---KLGD-----KYAC | WYQQKPGQ SPVLVIY | Q--------DSKRPS | GIPERFSGSNSG--NTATLTISGTQAMDEADYYC | QAWDS------------------STYVV | FGGGT KLTVL |
| iPS:3 93200 | 21-225_35E1 | VL3\|3r/JL 2 | | SYELTQP-PSVSVSPGQTASI TC | SGD---KLGE-----KYAY | WFQQKPGQ SPVIVIY | Q--------DRKRPS | GIPERFSGSNSG--NTATLTISGTQAMDEADYYC | QAWDN------------------STAV | FGGGT KLTVL |
| iPS:3 93202 | 21-225_6B4 | VL3\|3r/JL 2 | | SYELTQP-PSVSVSPRQTVSI TC | SGD---KLGD-----KYAC | WYQQKPGQ SPVLVIY | Q--------DRKRPS | GIPERFSGSNSG--NTATLTISGTQAMDEADYYC | QAWDN------------------NTVI | FGGGT KLTVL |
| iPS:3 93206 | 21-225_13F6 | VL3\|3r/JL 2 | | SYELTQP-PSVSVSPGQTASI TC | SGD---KLGD-----KYAC | WYQQKPGQ SPVLVIY | Q--------DSKRPS | GIPERFSGSNSG--NTATLTISGTQAMDEADYYC | QAWGN------------------STAVV | FGGGT KLTVL |
| iPS:3 93210 | 21-225_17D3 | VL3\|3r/JL 2 | | SYELTQS-PSVSVSPGQTASI TC | SGD---KLGD-----KYVY | WYQQKPGQ SPVVVIY | Q--------DRKRPS | GIPERFSGSNSG--NTATLTISGTQAMDEADYYC | QAWDS------------------ITAV | FGGGT KLTVR |
| iPS:3 93212 | 21-225_30H6 | VL3\|3r/JL 2 | | SYELTQP-PSVSVSPGQTASI TC | SGD---KLGN-----KYAC | WYQQKPGQ SPVLVIY | Q--------DSKRPS | GIPERFSGSNSG--NTATLTISGTQAMDEADYYC | QAWD-------------------SSTV | FGGGT KLTVL |

| | | | | | | | | | | |
|---|---|---|---|---|---|---|---|---|---|---|
| iPS:3 93214 | 21-225_33A1 | VL3\|3r/JL 2 | | SYELTQP-PSVSVSPGQTASI TC | SGD---KLGD-----KFVY | WYQQKPGQ SPVLVIY | Q--------DSKRPS | GIPERFSGSNSG--NTATLTISGTQAMDEADYYC | QAWDS-----------------------TTVV | FGGGT KLTVL |
| iPS:3 93218 | 21-225_14G3 | VL3\|3r/JL 2 | | SYELTQP-PSVSVSPGQTASI TC | SGD---KLGD-----KYVC | WYQQKPGQ SPVLVIY | Q--------DRKRPS | GIPERFSGSNSG--NTATLTISGTQAMDEADYYC | QAWGN---------------------STAVV | FGGGT KLTVL |
| iPS:3 93222 | 21-225_17F5 | VL3\|3r/JL 2 | | SYELTQP-PSVSVSPGQTASI TC | SGD---KLGE-----KYAC | WYQQKPGQ SPVLVIY | Q--------DRKRPS | GIPERFSGSNSG--NTATLTISGTQAMDEADYYC | QAWD------------------------SSTV | FGGGT KLTVL |
| iPS:3 93224 | 21-225_31C2 | VL3\|3r/JL 2 | | SYELTQP-PSVSVSPGQTASI TC | SGD---KLGN-----KYAC | WYQQKPGQ SPVLVIY | Q--------DSKRPS | GIPERFSGSNSG--NTATLTISGTQAMDEADYYC | QAWD------------------------SSTV | FGGGT KLTVL |
| iPS:3 93226 | 21-225_33E6 | VL3\|3r/JL 2 | | SYELTQP-PSVSVSPGQTASI TC | SGD---KLGD-----KYAY | WFQQKPGQ SPVIVIY | Q--------DRKRPS | GIPERFSGSNSG--NTATLTISGTQAMDEADYYC | QAWDN-----------------------STAV | FGGGT KLTVL |
| iPS:3 93234 | 21-225_26C10 | VL3\|3r/JL 2 | | SYEVTQP-PSMSVSPGQTASI TC | SGD---KLGD-----KYVC | WFQQKPGQ SPVLVIY | Q--------DSKRPS | GIPERFSGSNSG--NTATLTISGTQAMDEADYYC | QAWV------------------------NNTV | FGGGT KLTVL |
| iPS:3 93345 | 21-225_5G7 | VL3\|3r/JL 2 | | SYELTQP-PSVSVSPGQTASI TC | SGD---KLGN-----KYAW | WYQQKPGQ SPVLVIY | Q--------DRKRPS | GIPERFSGSNSG--NTATLTISGTQAMDEADYYC | QAWDN-----------------------STVV | FGGGT KLTVL |
| iPS:3 93565 | 21-225_34B11 | VL3\|3r/JL 2 | | SYELTQP-PSVSVSPGQTASI TC | SGD---KLGD-----KYAC | WYQQKPGQ SPVVVIY | Q--------DMKRPS | GIPERFSGSNSG--NTATLTISGTQAMDEADYYC | QAWDN-----------------------STAV | FGGGT KLTVL |
| iPS:3 93950 | 21-225_3H10 | VL3\|3r/JL 2 | | SYELSQP-PSVSVSPGQTASI TC | SGD---KLGE-----KYAC | WYQQKPGQ SPVLVIY | Q--------DRKRPS | GIPERFSGSNSG--NKATLTISGTQAMDEADYYC | QAWVN-----------------------NTMI | FGGGT KLTVL |
| iPS:3 98470 | 21-225_14B7 | VL3\|3r/JL 2 | | SYELTQP-PSVSVSPGRTASI TC | SGD---KLGN-----KYAY | WYQQKPGQ SPVLVIY | Q--------DRKRPS | GIPERFSGSNSG--NTATLTISGTQTMDEADYYC | QAWNN-----------------------STVV | FGGGT KLTVL |
| iPS:3 98472 | 21-225_16E4 | VL3\|3r/JL 2 | | PYELTQP-PSVSVSPGQTASI TC | SGD---KLGD-----KYVY | WYQQKSGQ SPVLVIY | Q--------DSKRPS | GIPERFSGSNSG--NTAALTISGTQAMDEADYYC | QAWDS-----------------------STVV | FGGGT KLTVL |
| iPS:3 98488 | 21-225_19F6 | VL3\|3r/JL 2 | | SYELTQP-PSVSVSPGQTVSI TC | SGD---KLGD-----KYAC | WYQQKPGQ SPVLVIY | Q--------DSKRPS | GTPERFSGSNSG--NTATLTISGTQAMDEADYYC | QAWDN-----------------------NTVV | FGGGT KLTVL |
| iPS:3 98490 | 21-225_21D12 | VL3\|3r/JL 2 | | SYELTQP-PSVSVSPGQTASI TC | SGD---KLGN-----KYAY | WYQQKPGQ SPVLVIY | Q--------DRKRPS | GIPERFSGSNSG--NTATLTISGTQAMDEADFYC | QAWDN-----------------------STVV | FGGGT RLTVL |
| iPS:3 98498 | 21-225_22E6 | VL3\|3r/JL 2 | | SYELTQP-PSVSVSPGQTASI TC | SGD---KLGE-----KYAC | WYQQKPGQ SPVLVIY | Q--------DRKRPS | GIPERYSGSNTG--NTATLTISGTQAMDEADYYC | QAWDS-----------------------STAV | FGGGT KLTVL |
| iPS:3 98504 | 21-225_23D7 | VL3\|3r/JL 2 | | SYELTQP-PSVSVSPGQTASI TC | SGE---KLGD-----KYVC | WYQQKPGQ SPVVVIY | Q--------DSKRPS | GIPERFSGSNSG--NTATLTISGTQAMDEADYYC | QAWNS-----------------------SNVV | FGGGT KLTVL |
| iPS:3 98546 | 21-225_9H10 | VL3\|3r/JL 2 | | SYELTQP-PSVSVSPGQTASI TC | SGD---KLGD-----KYAC | WYQQKPGQ SPVLVIY | Q--------DSKRPS | GIPERFSGSNSG--NTATLTISGTQAMDEADYYC | QAWDS----------------------STYVV | FGGGT KLTVL |

| iPS:4 ID | Clone | Germline | | L_FR1 | L_CDR1 | L_FR2 | L_CDR2 | L_FR3 | L_CDR3 | L_FR4 |
|---|---|---|---|---|---|---|---|---|---|---|
| iPS:4 02225 | 21-225_2B1 | VL3|3r/JL2 | | SYELTQP-PSVSVSPGQIVSITC | SGD---KLGD-----KYAC | WYQQKPGQ SPVLVIY | Q-------- DRKRPS | GIPERFSGSNSG--NTATLTISGTQAMDEADYYC | QAWDN------------NTVV | FGGGT KLTVL |
| iPS:4 02231 | 21-225_6D9 | VL3|3r/JL2 | | SYELTQP-PSVSVSPGQIASITC | SGD---KLGE-----KYAC | WYQQKPGQ SPVLVIY | Q-------- DKKRPS | GIPERFSGSNSG--NTATLTISGTQAMDEADYYC | QAWD------------SSTV | FGGGT KLTVL |
| iPS:4 04090 | 21-225_8D8 | VL3|3r/JL2 | | SYELTQP-PSVSVSPGQIASITC | SGD---KLGE-----KYAC | WYQQKPGQ SPVVVIY | Q-------- DRKRPS | GIPERFSGSNSG--NTATLTISGTQAIDEADYYC | QAWDS------------STAV | FGGGT KLTVL |
| iPS:4 23018 | 21-225_31D12_LC2 | VL3|3r/JL2 | | SYELTQP-PSVSVSPGQTASITC | SGD---KLGD-----KYAY | WFQQKPGQ SPVIVIY | Q-------- DRKRPS | GIPERFSGSNSG--NTATLTISGTQAMDEADYYC | QAWDN------------STAV | FGGGT KLTVL |
| | | Germline | | L_FR1 | L_CDR1 | L_FR2 | L_CDR2 | L_FR3 | L_CDR3 | L_FR4 |
| | VL2|2a2/JL3b | | 32185 | QSALTQP-ASVSGSPGQSITISC | TGTS-SDVGGY----NYVS | WYQQHPGK APKLMIY | E-------- VSNRPS | GVSNRFSGSKSG--NTASLTISGLQAEDEADYYC | SSYTSS------------STLWV | FGGGT KLTVL |
| iPS:4 68862 | 21-225_178H8 | VL2|2a2/JL3b | | QSALTQS-ASVSGSPGQSITISC | TGTS-SDVGGY----NFVS | WYQQHPGK VPKFMIY | E-------- VSNRPS | GVPNRFSGSKSG--NTASLTISGLQAEDEADYYC | SSYTS------------SYTWV | FGGGT KLTVL |
| iPS:4 36838 | 21-225_52H4 | VL2|2a2/JL3b | | QSALTQP-ASVSGSPGQSITISC | TGTS-SDVGGY----NYVS | WYQQHPGK APKLMIY | E-------- VSNRPS | GVSNRFSGSKSG--NTASLTISGLQAEDEADYYC | NSYTS------------NITWV | FGGGT KLTVL |
| iPS:4 37094 | 21-225_210D12 | VL2|2a2/JL3b | | QSALTQP-ASVSGSPGQSITISC | TGTS-SDVGGY----NYVS | WYQQHPVK APKLLIY | E-------- VSNRPS | GVSNRFSGSKSG--NTASLTISGLQAEDEADYYC | NSYTS------------SITWV | FGGGT KLTVL |
| iPS:4 37096 | 21-225_210E12 | VL2|2a2/JL3b | | QSALTQP-ASVSGSPGQSITISC | TGTS-SDVGGY----NYVS | WYQQHPGK APKLMIY | E-------- VSNRPS | GVSNRFSGSKSG--NTASLTISGLQAEDEADYYC | GSYVK------------GITWV | FGGGT SLTVL |
| iPS:4 37098 | 21-225_211C1 | VL2|2a2/JL3b | | QSALTQP-ASVSGSPGQSITISC | TGTS-SDVGSY----NYVS | WYQQYPGK APKLMIY | E-------- VSNRPS | GVSNRFSGSKSG--NTASLTISGLQAEDEADYYC | NSYTS------------SITWV | FGGGT KLTVL |
| iPS:4 37104 | 21-225_211G5 | VL2|2a2/JL3b | | QSALTQP-ASVSGSPGQSITISC | TGTS-SDVGGY----NYVS | WYQQHPGK APKLMIY | E-------- VSNRPS | GVSNRFSGSKSG--NTASLTISGLQAEDEADYYC | NSYTR------------SITWV | FGGGT KLTVL |
| iPS:4 37112 | 21-225_212C2 | VL2|2a2/JL3b | | QSALTQP-ASVSGSPGQSITISC | TGTS-SDVGGY----NYVS | WYQQHPGK APKLMIY | E-------- VRNRPS | GVSNRFSGSKSG--NTASLTISGLQAEDEADYYC | SSYTR------------SITWV | FGGGT KLTVL |
| iPS:4 37114 | 21-225_212A4 | VL2|2a2/JL3b | | QSALTQP-ASVSGSPGQSITISC | TGTS-SDVGGY----NYVS | WYQQHPGK APKLMIY | E-------- VSNRPS | GVSNRFSGSKSG--NTASLTISGLQAEDEADYYC | GSYVK------------GITWV | FGGGT SLTVL |
| iPS:4 37116 | 21-225_212F6 | VL2|2a2/JL3b | | QSALTQP-ASVSGSPGQSITISC | TGTS-SDVGGY----NYVS | WYQQYPGK APKLMIY | E-------- VSNRPS | GVSNRFSGSKSG--NTASLTISGLQAEDEADYYC | NSYTS------------SITWV | FGGGT KLTVL |
| iPS:4 37118 | 21-225_212G7 | VL2|2a2/JL3b | | QSALTQP-ASVSGSPGQSITISC | TGTS-SDVGGY----NYVS | WYQQHPGK TPKLMIY | E-------- VSNRPS | GVSNRFSGSKSG--NTASLTISGLQAEDEADYYC | NSYTS------------SITWV | FGGGT KLTVL |

| ID | Clone | Germline | No. | L_FR1 | L_CDR1 | L_FR2 | L_CDR2 | L_FR3 | L_CDR3 | L_FR4 |
|---|---|---|---|---|---|---|---|---|---|---|
| iPS:4 37128 | 21-225_213G3 | VL2\|2a2/JL3b | | LSALTQP-ASVSGSPGQSITISC | TGTS-SDVGGY----NYVS | WYQQHPGK APKLMIS | E--------VRNRPS | GVSNRFSGSKSG--NTASLTISGLQAEDEADYYC | NSYTR----------------SITWV | FGGGT KLTVL |
| iPS:4 37130 | 21-225_213D5 | VL2\|2a2/JL3b | | QSALTQP-ASVSGSPGQSITISC | TGTS-SDIGGY----NYVS | WYQQHPGK APKLVIY | E--------VRNRPS | GVSTRFSGSKSG--NKASLTISGLQAEDEADYYC | CSYTR----------------RITWV | FGGGT KLTVL |
| iPS:4 37146 | 21-225_215D3 | VL2\|2a2/JL3b | | QSALTQP-ASVSGSPGQSITISC | TGTS-SDVGGY----NYVS | WYQQHPGK APTLMIY | E--------VSNRPS | GVSNRFSGSKSG--NTASLTISGLQAEDEADYYC | NSYKR----------------GSTWV | FGGGT KVTVL |
| iPS:4 37150 | 21-225_216A3 | VL2\|2a2/JL3b | | QSALTQP-ASVSGSPGQSITISC | TGTS-SDVGGY----NYVS | WYQQHPGK APKLMIY | E--------VSNRPS | GVSNRFSGSKSG--NTASLTISGLQAEDEADYYC | NSYTS----------------SITWV | FGGGT KLTVL |
| iPS:4 37162 | 21-225_217B2 | VL2\|2a2/JL3b | | QSALTQP-ASVSGSPGQSITISC | TGTS-SDVGGY----NYVS | WYQQHPGK APKLLIY | E--------VSNRPS | GVYNRFSGSKSG--NTASLTISGLQAEDEADYYC | GSYVK----------------GITWV | FGGGT SLTVL |
| iPS:4 37172 | 21-225_219A7 | VL2\|2a2/JL3b | | QSALTQP-ASVSGSPGQSITISC | TGTS-SDVGGY----NYVS | WYQQHPGK APKLMIY | E--------VRNRPS | GVSNRFSGSKSG--NTASLTISGLQAEDEADYYC | CSYTR----------------SITWV | FGGGT KLTVL |
| iPS:4 37182 | 21-225_221H2 | VL2\|2a2/JL3b | | LSALTQP-ASVSGSPGQSITISC | TGTS-SDVGGY----NYVS | WYQQHPGK APKLMIS | E--------VRNRPS | GVSNRFSGSKSG--NTASLTISGLQAEDEADYYC | NSYTR----------------SITWV | FGGGT KLTVL |
| iPS:4 37184 | 21-225_221G4 | VL2\|2a2/JL3b | | QSALTQP-ASVSGSPGQSITISC | TGTS-SDVGGY----NYVS | WYQQHPGK APKLMIY | E--------VRNRPS | GVSNRFSGSKSG--NTASLTISGLQAEDEADYYC | NSYTR----------------SITWV | FGGGT KLTVL |
| | | **Germline** | | **L_FR1** | **L_CDR1** | **L_FR2** | **L_CDR2** | **L_FR3** | **L_CDR3** | **L_FR4** |
| | VL1\|1c/JL2 | | 32186 | QSVLTQP-PSASGTPGQRVTISC | SGSS-SNIGS-----NTVN | WYQQLPGT APKLLIY | S--------NNQRPS | GVPDRFSGSKSG--TSASLAISGLQSEDEADYYC | AAWDDS----------------LNGVV | FGGGT KLTVL |
| iPS:4 68864 | 21-225_60D6 | VL1\|1c/JL2 | | QSVLTQP-PSASGTPGQRVTISC | SGSS-SNIGS-----NTVN | WYQQLPGT APKLLIY | S--------NNQRPS | GVPDRFSGSKSG--TSASLAISGLQSEDEADYYC | AAWDD----------------SLNGP | VGGGT KLTVL |
| iPS:4 36660 | 21-225_146D8 | VL1\|1c/JL2 | | QSVLTQP-PSTSGTPGQRVTISC | SGSS-SYIGS-----NTVD | WYQQLPGT APKLLIY | S--------NNQRPS | GVPDRFSGSKSG--TSASLAISGLQSEDEADYYC | AAWDDS----------------LNGVV | FGGGT KLTVL |
| iPS:4 36680 | 21-225_147H12 | VL1\|1c/JL2 | | QSVLTQP-PSASGTPGQRVTISC | SGSS-SNIGS-----YAVN | WYQQLPGT APKLLIY | S--------NNHRPS | GVPDRFSGSKSG--TSASLAISGLQSEDEADYYC | EAWDDS----------------LNGPV | FGGGT KLTVL |
| iPS:4 36682 | 21-225_146A8 | VL1\|1c/JL2 | | QSVLTQP-PSASGTPGQRVTISC | SGSS-SNIGS-----NSIN | WYQQLPRT APKLLIY | S--------NDQRPS | GVPDRFSGSKSG--TSASLAISGLQSEDEADYYC | AAWDDS----------------LNGVV | FGGGT KLTVL |
| iPS:4 36684 | 21-225_146B6 | VL1\|1c/JL2 | | QSVLTQP-PSASGTPGQRVTISC | SGSS-SNIGS-----NAVN | WYQQLPGT APKLLIY | S--------NNQRPS | GVPDRFSGSKSG--TSASLAISGLQSEDEADYYC | AAWDDS----------------LNGVV | FGGGT KLTVL |
| iPS:4 36696 | 21-225_149A1 | VL1\|1c/JL2 | | QSVLTQP-PSASGTPGQRVTISC | SGSS-SNIGS-----NAVN | WYQQLPGT APKLLIY | S--------NNQRPS | GVPDRFSGSKSG--TSASLAISGLQSEDEADYYC | AAWDDS----------------LNGVV | FGGGT KLTVL |

| | | | | L_FR1 | L_CDR1 | L_FR2 | L_CDR2 | L_FR3 | L_CDR3 | L_FR4 |
|---|---|---|---|---|---|---|---|---|---|---|
| iPS:4 36712 | 21-225_150F9 | VL1\|1c/JL2 | | QSVLTQP-PSASGTPGQRVTISC | SGSS-SNIGS-----NAVN | WYQQLPGT APKLLIY | S--------NSQRPS | GVPDRFSGSKSG--ISASLAISGLQSEDEADYFC | AAWDDS-------------------LNGVV | FGGGT KLIVL |
| iPS:4 36750 | 21-225_154G12 | VL1\|1c/JL2 | | QSVLTQP-PSASGTPGQRVTISC | SGSS-SNIGN-----NAVS | WYQQLPGT APKLLIY | S--------NDHRPS | GVPDRFSGSKSG--ISASLAISGLQSEDEADYYC | AAWDDS-------------------LKGPV | FGGGT XLIVL |
| iPS:4 36762 | 21-225_156H2 | VL1\|1c/JL2 | | QSVVTQP-PSASGTPGQRVTISC | SGSS-SNIGS-----NTVN | WYQQLPGT APKLLIY | S--------SNQRPS | GVPDRLSGSKSG--TSASLAISGLQSEDEADYYC | AAWDDS-------------------LNGVV | FGGGT KVIVL |
| iPS:4 37044 | 21-225_197F9 | VL1\|1c/JL2 | | QSVLTQP-PSASGTPGQRVTISC | SGSS-SNIGS-----NTVN | WYQQLPGT APKLLIY | S--------NNQRPS | GVPDRFSGSKSG--TSASLAISGLQSEDEADYYC | AAWDDS-------------------MNGPV | FGGGT KLIVL |
| iPS:4 37060 | 21-225_199C3 | VL1\|1c/JL2 | | QSVLTQP-PSASGTPGQRVTISC | SGSS-SNIGS-----NTVN | WYQQLPGT APKLLIY | S--------NNQRPS | GVPDRFSGSKSG--TSASLAISGLQSEDEADYYC | AAWDDS-------------------LNGPV | FGGGT KLIVL |
| iPS:3 93180 | 21-225_4G12 | VL1\|1c/JL2 | | QSVLTQP-PSASGTPGQRVNMSC | SGTN-SNIGS-----YTVN | WYQQLPGT APKLLIY | I--------NNQRPS | GVPDRFSGSKSG--TSASLAISGLQSEDEADYYC | AAWDDS-------------------LNGHVV | FGGRT KLTVL |
| iPS:3 93230 | 21-225_9G9 | VL1\|1c/JL2 | | QSVLTQP-PSASGTPGQRVNMSC | SGTN-SNIGS-----YTVN | WYQQLPGT APKLLIY | I--------NNQRPS | GVPDRFSGSKSG--TSASLAISGLQSEDEADYYC | AAWDDS-------------------LNGHVV | FGGRT KLTVL |
| | | Germline | | L_FR1 | L_CDR1 | L_FR2 | L_CDR2 | L_FR3 | L_CDR3 | L_FR4 |
| | VL3\|3p/JL2 | | 32187 | SYELTQP-PSVSVSPGQTARITC | SGD---ALPK-----KYAY | WYQQKSGQ APVLVIY | E--------DSKRPS | GIPERFSGSSSG--TMATLTISGAQVEDEADYYC | YSTDSS-------------------GNHVV | FGGGT KLTVL |
| iPS:4 68866 | 21-225_190C1 | VL3\|3p/JL2 | | SYELTQP-PSVSVSPGQTARITC | TGD---AMPK-----KYAY | WDQQKSGQ APVLVIS | E--------DSKRPS | GIPERFSGSSSG--TMAPLTISGAQVEDETDYDC | NSTDS-------------------SGNRV | FGGGT KLTVL |
| iPS:4 37214 | 21-225_48B12 | VL3\|3p/JL2 | | SYELTQP-PSVSVSPGQTARITC | SGD---ALPK-----KYAY | WYQQKSGQ APVLVIY | E--------DSKRPS | GIPERFSGSSSG--TMATLTISGAQVEDEADYYC | NSTDSS-------------------GNHVV | FGGGT KLTVL |
| | | Germline | | L_FR1 | L_CDR1 | L_FR2 | L_CDR2 | L_FR3 | L_CDR3 | L_FR4 |
| | VL1\|1c/JL3b | | 32188 | QSVLTQP-PSASGTPGQRVTISC | SGSS-SNIGS-----NTVN | WYQQLPGT APKLLIY | S--------NNQRPS | GVPDRFSGSKSG--TSASLAISGLQSEDEADYYC | AAWDDS-------------------LNGWV | FGGGT KLTVL |
| iPS:4 36234 | 21-225_51E3 | VL1\|1c/JL3b | | QSVLTQP-PSASGTPGQRVTISC | SGSN-SNIGS-----NIVT | WYQQLPGT APKLLIY | S--------NDQRPS | GVPDRFSGSKSG--TSASLAISGLQSEDEADYYC | TAWDDS-------------------LNGWV | FGGGT TLTVL |
| iPS:4 36830 | 21-225_51F4 | VL1\|1c/JL3b | | QSVLTQP-PSASGTPGQRVTISC | SGSS-SNIGS-----NIVT | WYQQLPGT APKLLIY | S--------NDQRPS | GVPDRFSGSKSG--TSASLAISGLQSEDETDYYC | TAWDDS-------------------LNGWV | FGGGT TLTVL |
| iPS:4 36834 | 21-225_52F1 | VL1\|1c/JL3b | | QSVLTQP-PSASGTPGQRVTISC | SGSS-SNIGS-----NIVT | WYQQLPGT APKLLIY | S--------NDQRPS | GVPDRFSGSKSG--TSASLAISGLQSEDEADYYC | AAWDDS-------------------LNGWV | FGGGT TLTVL |
| iPS:4 36842 | 21-225_54E9 | VL1\|1c/JL3b | | QSVLTQP-PSASGTPGQRVTISC | SGSN-SNIGN-----NIVT | WYQQLPGT APKLLIY | V--------NDQRPS | GVPDRFSGSKSG--TSASLAISGLQSEDEADYYC | AAWDDS-------------------LNGWV | FGGGT TLTVL |

| ID | Clone | VL | | L_FR1 | L_CDR1 | L_FR2 | L_CDR2 | L_FR3 | L_CDR3 | L_FR4 |
|---|---|---|---|---|---|---|---|---|---|---|
| iPS:4 36844 | 21-225_56G1 | VL1\|1c/JL3b | | QSVLTQP-PSASGTPGQRVTISC | SGSS-SNIGS-----HIVI | WYQQLPGTAPKLLIY | S--------NDQRPS | GVPDRFSGSKSG--TSASLAISGLQSEDEADYYC | AVWDDS-----------------LIGWV | FGGGTTLTVL |
| iPS:4 36846 | 21-225_56E3 | VL1\|1c/JL3b | | QSVLTQP-PSASGTPGQRVTISC | SGSS-SNIGS-----NIVI | WYQQLPGTAPKLLIY | S--------NNQRPS | GVPDRFSGSKSG--TSASLAISGLQSEDEADYCC | AAWDDS-----------LNGWV | FGGGTTLTVL |
| iPS:4 37010 | 21-225_192G3 | VL1\|1c/JL3b | | QSVLTQP-PSASGTPGQRVTMSC | SGSS-SNIGS-----NTVN | WYQQFPGTAPKLLIY | G--------NKQRPS | RVPDRFSGSKSG--TSASLAISGLQSEDETDYYC | AAWDDS-----------LNGWV | FGGGTKLTVL |
| iPS:4 37032 | 21-225_195H6 | VL1\|1c/JL3b | | QSVLTQP-PSASGTPGQRVTISC | SGSS-SNIGS-----HTVN | WYQQLPGTAPKLLIY | N--------NYQRPS | GVPDRFSGSKSG--TSASLTISGLQSEDEADYYC | AIWDDS-----------LSVWV | FGGGTKVTVL |
| iPS:4 51104 | 21-225_49C5 | VL1\|1c/JL3b | | QSVLTQP-PSASGTPGQRVTISC | SGSS-SNIGS-----NIVI | WYQQLPGTAPKLLIY | S--------NDQRPS | GVPDRFSGSKSG--TSASLAISGLQSEDEADYYC | TAWDDS-----------LNGWV | FGGGTTLTVL |
| iPS:4 51106 | 21-225_49D10 | VL1\|1c/JL3b | | QSVLTQP-PSASGTPGQRVTISC | SGSN-SNIGS-----NIVI | WYQQLPGTAPKLLIY | S--------NDQRPS | GVPDRFSGSKSG--TSASLAISGLQSEDEADYYC | AAWDDS-----------LNGWV | FGGGTTLTVL |
| iPS:4 51108 | 21-225_53E8 | VL1\|1c/JL3b | | QSVLTQP-PSASGTPGQRVTISC | SGSC-SNIGS-----NIVI | WYQQLPGTAPKLLIY | S--------NDQRPS | GVPDRFSGSKSG--TSASLAISGLQSEDEADYYC | TAWDDS-----------LNDWV | FGGGTTLTVL |
| | Germline | | | L_FR1 | L_CDR1 | L_FR2 | L_CDR2 | L_FR3 | L_CDR3 | L_FR4 |
| | VL3\|3r/JL1 | | 32189 | SYELTQP-PSVSVSPGQTASITC | SGD---KLGD-----KYAC | WYQQKPGQSPVLVIY | Q--------DSKRPS | GIPERFSGSNSG--NTATLTISGTQAMDEADYYC | QAWDS----------------STAYV | FGTGTKVTVL |
| iPS:4 36662 | 21-225_147D7 | VL3\|3r/JL1 | | SYELTQP-PSVSVSPGQIASITC | SGD---KLGD-----KFAC | WYQQKPGQSPVLVIY | Q--------DRKRPS | GIPERFSGSNSG--NTATLTISGTQAMDEADYYC | QAWDR-----------NTAV | FGTGTKVTVL |
| iPS:4 36720 | 21-225_151H6 | VL3\|3r/JL1 | | SYELTQP-PSVSVSPGQIASITC | SGD---KLGD-----KYAC | WYQQKPGQSPVLVIY | Q--------DTKRPS | GIPERFSGSNSG--NTATLTISGTQAMDEADYYC | QAWDS-----------STYV | FGTGTKVTVL |
| iPS:4 36726 | 21-225_152G5 | VL3\|3r/JL1 | | SYEMTQP-PSVSVSPGQTAIITC | SGD---KLGD-----KYAC | WYQQKPGQSPVLVIY | Q--------DSKRPS | GIPERFSGSNSG--NTATLTISGTQAMDEADYYC | QAWDS-----------STYV | FGTGTKVTVL |
| iPS:4 36732 | 21-225_152B12 | VL3\|3r/JL1 | | SYELTQP-PSVSVSPGQTASITC | SGD---KLGN-----KYAC | WYQQKPGQSPVLVIY | Q--------DTKRPS | GIPERFSGSNSG--NTATLTISGTQAMDEADYYC | QAWDS-----------STYV | FGTGTKVTVL |
| iPS:4 36734 | 21-225_153A8 | VL3\|3r/JL1 | | SYELTQP-PSVSVSPGQTASITC | SGD---KLGD-----KYAC | WYQQKPGQSPVLVIY | Q--------DTKRPS | GIPERFSGSNSG--NTATLTISGTQAMDEADYYC | QAWDS-----------STYV | FGTGTKVTVL |
| iPS:4 36754 | 21-225_155G3 | VL3\|3r/JL1 | | SYELTQP-PSVSVSPGQTASITC | SGD---KLGD-----KYVC | WYQQKPGQSPMLVIY | Q--------DSKRPS | GIPERFSGSNSG--NTATLTISGTQAMDEADYYC | QAWDN-----------SIYV | FGTGTKVTVL |
| iPS:4 37190 | 21-225_225A9 | VL3\|3r/JL1 | | SYELTQP-PSVSVSPGQTASITC | SGD---KLGN-----KYAC | WYQQKPGQSPVLVIY | Q--------DSKRPS | GIPERFSGSNSG--NTATLTISGTQAMDEADYYC | QAWDS-----------NTACV | FGTGTKVTVL |
| | Germline | | | L_FR1 | L_CDR1 | L_FR2 | L_CDR2 | L_FR3 | L_CDR3 | L_FR4 |

| | | | | L_FR1 | L_CDR1 | L_FR2 | L_CDR2 | L_FR3 | L_CDR3 | L_FR4 |
|---|---|---|---|---|---|---|---|---|---|---|
| **VL5\|5c/JL3b** | | | 32190 | QAVLTQP-ASLSASPGASASLTC | TLRS-GINVGI----YRIY | WYQQKPGS PPQYLLR | YKS---- DSDKQQGS | GVPSRFSGSKDASANAGILL ISGLQSEDEADYYC | MIWHS----------------SASWV | FGGGT KLTVL |
| iPS:4 36702 | 21-225_149E8 | VL5\|5c/JL3b | | QAVSTQP-SSLSASPGASASLTC | TLRS-GITVTT----YRIY | WYQQKPGS PPQFLLR | YTS---- DSDKHQGS | GVPSRFSGSKDASANAGILF ISGLQSEDEADYYC | MIWHS--------------SAWV | FGGGT KLTVL |
| Germline | | | | L_FR1 | L_CDR1 | L_FR2 | L_CDR2 | L_FR3 | L_CDR3 | L_FR4 |
| **VL1\|1e/JL2** | | | 32191 | QSVLTQP-PSVSGAPGQRVTISC | TGSS-SNIGAG----YDVH | WYQQLPGT APKLLIY | G-------- NSNRPS | GVPDRFSGSKSG--TSASLAIIGLQAEDEADYYC | QSYDSS-----------LSGVV | FGGGT KLTVL |
| iPS:4 36752 | 21-225_155H1 | VL1\|1e/JL2 | | QSVLTQP-PSVSGAPGQRVTISC | TGSS-SNIGAG----YDVH | WYQQLPGT APKLLIY | G-------- NSNRPS | GVPDRFSGSKSG--TSASLAIIGLQAEDEADYHC | QSYDSS-----------LSGPVI | FGGGT KLTVL |
| iPS:4 36820 | 21-225_179D10 | VL1\|1e/JL2 | | QSVLTQP-PSVSGAPGQRVTISC | TGSS-SNFGTD----YDVH | WYQQFPGT APKLLIY | G-------- HSNRPS | GVPDRFSGSKSG--TSASLAIIGLQAEDEADYYC | QSYDR-----------SLNVV | FGGGT KLTVL |
| iPS:4 37188 | 21-225_224B11 | VL1\|1e/JL2 | | QSVLTQP-PSVSGAPGQRVTISC | TGSS-SNIGAG----YDVH | WYQQLPGT APKLLIF | G-------- NSNRPS | GVPDRFSGSKSG--TSASLAIIGLQAEDEADYHC | QSYDN-----------SLSCV | FGGGT KLTVL |
| iPS:4 37198 | 21-225_226F8 | VL1\|1e/JL2 | | QSVLTQP-PSVSGAPGQRVTISC | TGSS-SNIGAG----YDVH | WYQQLPGT APKLLIY | G-------- NSNRPS | GVPDRFSGSKSD--TSASLAIIGLQAEDEADYYC | QSYDN-----------SLSGV | FGGGT KLTVL |
| iPS:4 37202 | 21-225_227D3 | VL1\|1e/JL2 | | QSVLTQP-PSVSGAPGQRVTISC | TGSS-SNIGAG----YDVH | WYQQLPGT APKLLIY | G-------- NSNRPS | GVPDRFSGSKSD--TSASLAITGLQAEDEADYYC | QSYDN-----------SLSGV | FGGGT KLTVL |
| iPS:4 37208 | 21-225_227C10 | VL1\|1e/JL2 | | QSVLTQP-PSVSGAPGQRVTISC | TGSS-SNIGAG----YDVH | WYQQLPGT APKLLIY | G-------- NSNRPS | GVPDRFSGSKSD--TSASLAITGLQAEDEADYYC | QSYDN-----------NLSGV | FGGGT KLTVL |
| iPS:4 43003 | 21-225_43F11_LC2 | VL1\|1e/JL2 | | QSVLTQP-PSVSGAPGQRVTISC | TGSS-SNIGAG----YDVH | WYQQLPGT APKLLIY | G-------- NSNRPS | GVPDRFSGSKSG--TSASLAIIGLQAEDEADYYC | QSYDNS-----------LSGSV | FGGGT KLTVL |
| Germline | | | | L_FR1 | L_CDR1 | L_FR2 | L_CDR2 | L_FR3 | L_CDR3 | L_FR4 |
| **VL6\|6a/JL3b** | | | 32192 | NFMLTQP-HSVSESPGKTVTISC | TRSS-GSIAS-----NYVQ | WYQQRPGS SPTIVIY | E-------- DNQRPS | GVPDRFSGSIDSSSNSASLT ISGLKTEDEADYYC | QSYDS-----------SNWV | FGGGT KLTVL |
| iPS:4 36792 | 21-225_169D12 | VL6\|6a/JL3b | | NFMLTQP-HSVSESPGKTVTISC | TRSS-GSITG-----NYVQ | WHQQRPGN SPTILIY | E-------- DKKRPS | GVPDRFSGSIDSSSNSASLT ISGLKTEDEADYYC | QSYYS-----------GNWV | FGGGT KLTVL |
| Germline | | | | L_FR1 | L_CDR1 | L_FR2 | L_CDR2 | L_FR3 | L_CDR3 | L_FR4 |
| **VL3\|3l/JL2** | | | 32193 | SSELTQD-PAVSVALGQTVRITC | QGD---SLRS-----YYAS | WYQQKPGQ APVLVIY | G-------- KNNRPS | GIPDRFSGSSSG--NTASLTITGAQAEDEADYYC | NSRDSS-----------GNHVV | FGGGT KLTVL |
| iPS:4 36800 | 21-225_171D12 | VL3\|3l/JL2 | | SSELTQD-PAVSVALGQTVRITC | QGD---SLRS-----YYAS | WYQQKPGQ APILVIY | A-------- KNNRPS | GIPDRFSGSNSG--NTASLIITGAQAEDEADYYC | NSRDSS-----------GSHVV | FGGGT KLTVL |
| iPS:4 36804 | 21-225_172C3 | VL3\|3l/JL2 | | SSELTQD-PAVSVALGQTVRITC | QGD---SLRN-----YYVS | WYQQKPGQ APILVIY | T-------- KNSRPS | GIPDRFSGSTSG--NTASLIITGIQAEDEADYYC | NSRDSS-----------GNHVV | FGGGT KLTVL |

| | | | | | | | | | | |
|---|---|---|---|---|---|---|---|---|---|---|
| iPS:4 36806 | 21-225_172B12 | VL3\|3I/JL2 | | SSELTQD-PAVSVALGQIVRITC | QGD---SLRN-----YYAS | WYQQKPGQ APILVIY | T--------KNSRPS | GIPDRFSGSTSG--NTASLTIITGAQAEDEADYYC | NSRDSS--------------------GNHVV | FGGGT KLTVL |
| iPS:4 36964 | 21-225_190B3 | VL3\|3I/JL2 | | SSELTQD-PAVSVALGQIVRITC | QGD---KLRT-----YYAS | WYQQKPGQ APVLVVY | G--------KNNRPS | GIPDRFSGSSSG--NTASLTIITGAQAEDEADYYC | NSRDSS-------------------GNHLVL | FGGGT KLTVL |
| iPS:4 36970 | 21-225_190B8 | VL3\|3I/JL2 | | SSELTQD-PAVSVALGQIVRITC | QGD---TLRP-----YYVS | WYQQKPGQ APVLVIY | G--------KNNRPS | GIPDRFSGSSSG--NTASLTIITGAQAEDEADYYC | NSRDSS-------------------GNHLVV | FGGGT KLTVL |
| iPS:4 36980 | 21-225_190C10 | VL3\|3I/JL2 | | SSELTQD-PAVSVALGQIVRITC | QGD---SLRP-----YYAS | WYQQKPGQ APVLVIY | G--------KNNRPS | GIPDRFSCSSSG--NTASLTIIEAQAEDEADYYC | NSRDSS-------------------GNHLVV | FGGGT KLTVL |
| iPS:4 36992 | 21-225_191B8 | VL3\|3I/JL2 | | SSELTQD-PAVSVALGQIVRITC | QGD---TLRP-----YYAS | WYQQKPGQ APVLVIY | G--------KNNRPS | GISDRFSGSSSG--NTASLTIITGAQAEDEADYYC | NSRDSS-------------------GNHLVV | FGGGT KLTVL |
| iPS:4 36994 | 21-225_191A9 | VL3\|3I/JL2 | | SSELTQD-PAVSVALGQIVRITC | QGD---SLRP-----YYAS | WYQQKPGQ APVLVIY | G--------KNNRPS | GIPDRFSGSSSG--NTASLTIITEAQAEDEADYYC | NSRDSC-------------------GNHLVV | FGGGT KLTVL |
| iPS:4 37016 | 21-225_193A6 | VL3\|3I/JL2 | | SSELTQD-PAVSVALGQIVRITC | QGD---SLRS-----YYAN | WYQQKPGQ APVLFIY | A--------KNNRPS | GIPDRFSGSNSG--NTASLTIITGAQAEDEADYYC | NSRDSS--------------------GNHLV | FGGGT KLTVL |
| | | Germline | | **L_FR1** | **L_CDR1** | **L_FR2** | **L_CDR2** | **L_FR3** | **L_CDR3** | **L_FR4** |
| **VL2\|2b2/JL2** | | | 32194 | QSALTQP-ASVSGSPGQSITISC | TGTS-SDVGSY----NLVS | WYQQHPGK APKLMIY | E--------VSKRPS | GVSNRFSGSKSG--NTASLTISGLQAEDEADYYC | CSYAGS-------------------------STFVV | FGGGT KLTVL |
| iPS:4 36810 | 21-225_175F4 | VL2\|2b2/JL2 | | QSALTQP-ASVSGSPGQSITISC | TGTS-SDVGRF----NLVS | WYQQHPGY APKLMIY | E--------VSKRPS | GVSNRFSGSKSG--NTASLTISGLQAEDEADYYC | CSYAGS-------------------STYVV | FGGGT KLTVL |
| iPS:4 36814 | 21-225_178H10 | VL2\|2b2/JL2 | | QSALTQP-ASVSGSPGQSITISC | TGTS-SDVGRF----NLVS | WYQHHPGN APKLMIY | E--------VSKRPS | GVSNRFSGSKSG--NTASLTISGLQAEDEADYYC | CSYAGS-------------------STFVV | FGGGT KLTVL |
| | | Germline | | **L_FR1** | **L_CDR1** | **L_FR2** | **L_CDR2** | **L_FR3** | **L_CDR3** | **L_FR4** |
| **VL3\|3I/JL3b** | | | 32195 | SSELTQD-PAVSVALGQIVRITC | QGD---SLRS-----YYAS | WYQQKPGQ APVLVIY | G--------KNNRPS | GIPDRFSGSSSG--NTASLTIITGAQAEDEADYYC | NSRDSS-------------------GNHWV | FGGGT KLTVL |
| iPS:4 36816 | 21-225_179H5 | VL3\|3I/JL3b | | SSELTQD-PAVSVALGQIVRITC | QGD---SLRN-----YYAS | WYQQKPGQ APVFVIY | G--------KNNRPS | GIPDRFSGSRSG--NTASLTIITGAQAEDEADYYC | NSRDSS-------------------GNHWV | FGGGT KLTVL |
| | | Germline | | **L_FR1** | **L_CDR1** | **L_FR2** | **L_CDR2** | **L_FR3** | **L_CDR3** | **L_FR4** |
| **VL1\|1e/JL1** | | | 32196 | QSVLTQP-PSVSGAPGQRVTISC | TGSS-SNIGAG----YDVH | WYQQLPGT APKLLIY | G--------NSNRPS | GVPDRFSGSKSG--TSASLAITGLQAEDEADYYC | QSYDSS-------------------LSGYV | FGTGT KVTVL |
| iPS:4 36832 | 21-225_51D8 | VL1\|1e/JL1 | | QSVLTQP-PSVSGAPGQRVTISC | TGSS-SNIGAG----FEVH | WYQQLPGT APKLLIY | G--------NSNRPS | GVPDRFSGSKSG--TSASLAITGLQAEDEADYYC | QSYDSS-------------------LSGYV | FGTGT KVTVL |
| | | Germline | | **L_FR1** | **L_CDR1** | **L_FR2** | **L_CDR2** | **L_FR3** | **L_CDR3** | **L_FR4** |

| Clone ID | Name | VL/JL | Germline | L_FR1 | L_CDR1 | L_FR2 | L_CDR2 | L_FR3 | L_CDR3 | L_FR4 |
|---|---|---|---|---|---|---|---|---|---|---|
| | VL3\|3r/JL7 | | 32197 | SYELTQP-PSVSVSPGQIASITC | SGD---KLGD-----KYAC | WYQQKPGQ SPVLVIY | Q--------DSKRPS | GIPERFSGSNSG--NTATLTISGTQAMDEADYYC | QAWDS-----------STAAV | FGGGT QLTVL |
| iPS:4 36840 | 21-225_53E9 | VL3\|3r/JL7 | | SYELTQP-PSVSVSPGQIASITC | SGT---KLGD-----KYVC | WYQQKPGQ SPVLVIN | Q--------DTMRPS | GIPERFSGSNSG--NTATLTISGTQAMDEADYYC | QIWDS-----------STAV | FGGGT TLTVL |
| iPS:4 36950 | 21-225_184G4 | VL3\|3r/JL7 | | SYELTQP-PSVSVSPGQIASITC | SGD---KLGD-----KFAC | WYQQKPGQ SPVLVIY | E--------DRKRPS | GIPERFSGSNSG--NTATLTISGTQAMDEADYYC | QAWDS-----------RTVV | FGGGT QLTVL |
| | Germline | | | L_FR1 | L_CDR1 | L_FR2 | L_CDR2 | L_FR3 | L_CDR3 | L_FR4 |
| | VL1\|1b/JL2 | | 32198 | QSVLTQP-PSVSAAPGQKVTISC | SGSS-SNIGN-----NYVS | WYQQLPGT APKLLIY | D--------NNKRPS | GIPDRFSGSKSG--TSATLGITGLQTGDEADYYC | GTWDSS-----------LSAVV | FGGGT KLTVL |
| iPS:4 36856 | 21-225_58C5 | VL1\|1b/JL2 | | QSVLTQP-PSVSAAPGQKVTISC | SGSS-SNIGN-----NYVS | WYQQLPGT APKLLIY | D--------NNKRPS | GIPDRFSGSKSG--TSATLGITGLQTGDEADYYC | GTWDIS-----------LSVGV | FGGGT KLTVL |
| iPS:4 36960 | 21-225_198D2 | VL1\|1b/JL2 | | QSVLTQP-PSVSAAPGQKVTISC | SGSS-SNIGS-----NYVS | WYQQLPGT APKVLIY | D--------NNKRPS | GIPDRFSGSKSG--TSATLGITGLQTGDEADYYC | GTWDSR-----------LNVGV | FGGGT KLTVL |
| iPS:4 36966 | 21-225_190C3 | VL1\|1b/JL2 | | QSVLTQP-PSVSAAPGQKVTISC | SGSS-SNIGN-----NYVS | WYQQLPGT APKLLLY | D--------SNKRPS | GIPGRFSGSKSG--TSATLGITGLQTGDEADYYC | GTWDSS-----------LSTVV | FGGGT KLTVL |
| iPS:4 36968 | 21-225_190B10 | VL1\|1b/JL2 | | QSVLTQP-PSVSAAPGQKVTISC | SGSS-SNIGN-----NYVS | WYQHLPGT APKLLIY | D--------NNKRPS | GIPDRFSGSKSG--TSATLGITGLQTGDEADYYC | GTWDSS-----------LSAGV | FGGGT KLTVL |
| iPS:4 36974 | 21-225_190H7 | VL1\|1b/JL2 | | QSVLTQP-PSVSAAPGQKVTISC | SGSS-SNIGS-----NYVS | WYQQLPGT APKVLIY | D--------NNKRPS | GIPDRFSGSKSG--TSATLGITGLQTGDEADYYC | GTWDGR-----------LNVGV | FGGGT KLTVL |
| iPS:4 36976 | 21-225_190D8 | VL1\|1b/JL2 | | QSVLTQP-PSVSAAPGQKVTISC | SGSS-SNIGN-----HYVS | WYQQLPGT APKLLIY | D--------SSKRPS | EIPDRFSGSKSG--TSATLGITGLQTGDEADYYC | GTWDSS-----------LSTVV | FGGGT KLTVL |
| iPS:4 36982 | 21-225_190D10 | VL1\|1b/JL2 | | QSVLTQP-PSVSAAPGQKVTISC | SGSS-SNIGS-----NYVS | WYQQLPGT VPKVLIY | D--------NNKRPS | GIPDRFSGSKSG--TSATLGITGLQTGDEADYYC | GTWDSR-----------LNVGV | FGGGT KLTVL |
| iPS:4 36986 | 21-225_191A1 | VL1\|1b/JL2 | | QSVLTQP-PSVSAAPGQRVTISC | SGSS-SNLGN-----NFVS | WYQQFPGT APKLLIY | D--------NYKRPS | GIPDRFSVSKSG--TSATLGITGLQTGDEADYYC | GTWDSS-----------LNTGV | FGGGT KLTVL |
| iPS:4 37006 | 21-225_192G2 | VL1\|1b/JL2 | | QSVLTQP-PSVSAAPGQKVTISC | SGSS-SNIGN-----NYVS | WYQQLPGT APKLLIY | D--------NNKRPS | RIPDRFSGSKSG--TSATLGITGLQTGDEADYYC | GTWDSS-----------LSAGV | FGGGT KLTVL |
| iPS:4 37024 | 21-225_194F11 | VL1\|1b/JL2 | | QSVLTQP-PSVSAAPGQKVTISC | SGSS-SNIGN-----NYVS | WYQQLPGT APKLLIY | D--------NNKRPS | GIPDRFSGSKSG--TSATLGITGLQTGDEADYYC | GTWDSS-----------LSAGV | FGGGT KLTVL |
| iPS:4 37028 | 21-225_194G12 | VL1\|1b/JL2 | | QSVLTQP-PSVSAAPGQKVTISC | SGSS-SNIGN-----NYVS | WYQQLPGT APKLLIY | D--------NNKRPS | RIPDRFSGSKSG--TSATLGITGLQTGEEADYYC | GTWDSS-----------LSVGV | FGGGT KLTVL |

| | | | | L_FR1 | L_CDR1 | L_FR2 | L_CDR2 | L_FR3 | L_CDR3 | L_FR4 |
|---|---|---|---|---|---|---|---|---|---|---|
| iPS:4 37042 | 21-225_197E8 | VL1\|1b/JL2 | | QSVLTQP-PSVSAAPGQKVTISC | SGSS-SNIGN-----KYVS | WYQQFPGTAPKLLIY | D---------NNKRPS | KIPDRFSGSKSG--TSATLGIITGLLTGDEADYYC | GIWDRS--------------------LSVMV | FGGGTKLTVL |
| iPS:4 37064 | 21-225_200G8 | VL1\|1b/JL2 | | QSVLTQP-PSVSAAPGQRVTISC | SGSS-SNLGN-----NFVS | WYQQFPGTAPKLLIY | D---------NYKRPS | GIPDRFSVSKSG--TSATLGIITGLQTGDEADYYC | GIWDSS--------------------LNTGV | FGGGTKLTVL |
| iPS:4 37086 | 21-225_209A8 | VL1\|1b/JL2 | | QSVLTQP-PSVSAAPGQKVTISC | SGSS-SNIGS-----NFLS | WYQQLPGTAPKLLIY | D---------NNKRPS | GIPDRFSGSKSG--TSATLGIITGLQTGDEADYYC | GIWDSS--------------------LSAGV | FGGGTKLTVL |
| iPS:4 37138 | 21-225_214D8 | VL1\|1b/JL2 | | QSVLTQP-PSVSAAPGQKVTISC | SGSS-SNIGN-----NYVS | WYQQFPGTAPKLLIH | D---------NNKRPS | GIPDRFSGSKSG--TSATLGIITGLQTGDEADYYC | GAWDSS--------------------LSAVV | IGGGSKLTVL |
| iPS:4 37168 | 21-225_218G4 | VL1\|1b/JL2 | | QSVLTQP-PSVSAAPGQKVTISC | SGSS-SNIGN-----NYVS | WYQQLPGTAPKLLLY | D---------SNKRPS | GIPARFSGSKSG--TSATLGIITGLQTGDEADYYC | GTWDSS--------------------LNTVV | FGGGTKLTVL |
| | | Germline | | L_FR1 | L_CDR1 | L_FR2 | L_CDR2 | L_FR3 | L_CDR3 | L_FR4 |
| | VL6\|6a/JL2 | | 32199 | NFMLTQP-HSVSESPGKTVTISC | TRSS-GSIAS-----NYVQ | WYQQRPGSSPTTVIY | E---------DNQRPS | GVPDRFSGSIDSSSNSASLTISGLKTEDEADYYC | QSYDS--------------------SNVV | FGGGTKLTVL |
| iPS:4 36888 | 21-225_63G7 | VL6\|6a/JL2 | | NFMLTQP-HSVSESPGKTVTISC | TRSN-GSIVS-----NYVQ | WYQQRPGSSPTIMIY | E---------DSRRPS | GVPDRFSGSIDSSSNSASLTISGLKTEDEADYSC | QSYDG--------------------INVV | FGGGTKLTVL |
| iPS:4 36890 | 21-225_63A10 | VL6\|6a/JL2 | | NFMLTQP-HSVSESPGKTVTISC | TRSN-GSIVS-----NYVQ | WYQQRPGSSPTTVIY | E---------DKRRPS | GVPDRFSGSIDSSSNSASLTISGLKTEDEADYYC | QSYDS--------------------INVV | FGGGTKLTVL |
| | | Germline | | L_FR1 | L_CDR1 | L_FR2 | L_CDR2 | L_FR3 | L_CDR3 | L_FR4 |
| | VL8\|8a/JL3b | | 32200 | QTVVTQE-PSFSVSPGGTVTLTC | GLSS-GSVSTS----YYPS | WYQQTPGQAPRILIY | S---------TNTRSS | GVPDRFSGSILG--NKAALTITGAQADDESDYYC | VLYMG--------------------SGIWV | FGGGTKLTVL |
| iPS:4 36910 | 21-225_73G1 | VL8\|8a/JL3b | | QTVVTQE-PSFSVSPGGTVTLTC | GLSS-GSVSTS----YYPS | WYQQTPGQAPRILIY | N---------TNTRSS | GVPDRFSGSILG--NKAALTITGAQADDESDYYC | VLYMG--------------------SAIWV | FGGGTKLTVL |
| iPS:4 36948 | 21-225_183F5 | VL8\|8a/JL3b | | QTVVTQE-PSFSVSPGGTVTLTC | GLSS-GSVSTT----FYPS | WYQQTPGQAPRILIY | N---------TNTRSS | GVPDRFSGSILG--NKAALTITGAQADDESDYYC | VLYMG--------------------SGIWV | FGGGTKLTVL |
| | | Germline | | L_FR1 | L_CDR1 | L_FR2 | L_CDR2 | L_FR3 | L_CDR3 | L_FR4 |
| | VL7\|7a/JL2 | | 32201 | QTVVTQE-PSLTVSPGGTVTLTC | ASST-GAVTSG----YYPN | WFQQKPGQAPRALIY | S---------TSNKHS | WTPARFSGSLLG--GKAALTLSGVQPEDEAEYYC | LLYYG--------------------GAQVV | FGGGTKLTVL |
| iPS:4 36920 | 21-225_74E5 | VL7\|7a/JL2 | | QTVVTQE-PSLTVSPGGTVTLTC | ASST-ETVTSG----SYPN | WFQQKPGQAPRALIY | S---------TSNKHS | WTPARFSGSLLG--GKAALTLSDVQPEDEAEYYC | LLYYG--------------------GAQLV | FGGGTKLTVL |
| iPS:4 36926 | 21-225_78D10 | VL7\|7a/JL2 | | QTVVTQE-PSLTVSPGGTVTLTC | ASST-GAVTSG----YFPN | WFQQKPGQAPRALIY | S---------TDNKHS | WTPARFSGSLLG--GKAALTLSGVQPEDEAEYYC | LLYYG--------------------GAQLM | FGGGTKLTVL |
| iPS:4 36958 | 21-225_190D1 | VL7\|7a/JL2 | | QTVVTQE-PSLTVSPGGTVTLTC | ASST-GAVTSG----SYPN | WFQQKPGQAPRALIY | S---------TSNKHS | WTPARFSGSLLG--GKAALTLSGVQPEDEADYYC | LLYYG--------------------GAQVA | FGGGTKLTVL |

| iPS | name | VL7|7a/JL2 | | FR1 | CDR1 | FR2 | CDR2 | FR3 | CDR3 | FR4 |
|---|---|---|---|---|---|---|---|---|---|---|
| iPS:4 36984 | 21-225_190F10 | VL7|7a/JL 2 | | QTVVTQE-PSLTVSPGGTVTL TC | VFST-GAVTSG----SFPN | WFQQKPGQ APRALIY | S--------TSNXHS | WTPARFSGSLLG--GKAALTLSGVQPEDEADYYC | LLYCG----------------------GAQLV | FGGGT KLTVL |
| iPS:4 36988 | 21-225_191A2 | VL7|7a/JL 2 | | QTVVTQE-PSLTVSPGGTVTL TC | VLST-GAVTSG----SFPN | WFQQKPGQ APRALIY | S--------TSNXHS | WTPARFSGSLLG--GKAALTLSGVQPEDEADYYC | MLYCG----------------------GAQLV | FGGGT KLTVL |
| iPS:4 37002 | 21-225_191H9 | VL7|7a/JL 2 | | QTVVTQE-PSLTVSPGGTVTL TC | ASST-GAVTSA----YYPN | WLQQKPGQ APRTLIY | S--------TNNXHS | WTPARFSGSLLG--GKAALTLSDVQPEDEAEYYC | LIFYG----------------------GVHVI | FGGGT KLTVL |
| iPS:4 37008 | 21-225_192E3 | VL7|7a/JL 2 | | QTVVTQE-PSLTVSPGGTVTL TC | AFST-GSVTSG----SYPN | WFQQKPGQ APRALIY | S--------TNNXHS | WTPARFSGSLLG--GKAALTLSGVQREDEAEYYC | LLYYG----------------------GAQLV | FGGGT KLTVL |
| iPS:4 37012 | 21-225_192G7 | VL7|7a/JL 2 | | QTVVTQE-PSLTVSPGGTVTL TC | ASST-GAVTSG----NYPQ | WFQQKPGQ APRALIY | S--------TTNRHS | WTPARFSGSLLG--GKAALTLSGVQPEDEAEYYC | LFYYG----------------------GAQVI | FGGGT KLTVL |
| iPS:4 37014 | 21-225_192H8 | VL7|7a/JL 2 | | QTVVTQE-PSLTVSPGGTVTL TC | AFST-GTVTSG----FYPN | WFQQKPGQ APRALIY | N--------TSNRHS | WTPARFSGSLLG--GMAALTLSGVQPEDEADYYC | LLYYG----------------------GAQLM | FGGGT KLTVL |
| iPS:4 37022 | 21-225_194G5 | VL7|7a/JL 2 | | QTVVTQE-PSLTVSPGGTVTL TC | ASST-GAVTSG----NYPN | WFQQKPGQ TPRALIY | S--------TSNXHS | WTPARFSGSLLG--GKGALTLSGVQPEDEAEYYC | LIYYG----------------------GAQLM | FGGGT KLTVL |
| iPS:4 37026 | 21-225_194D12 | VL7|7a/JL 2 | | QTVVTQE-PSLTVSPGGTVTL TC | ASST-GAVTSG----SFPS | WFQQKPGQ APRALIY | S--------TSNRHS | STPARFSGSLLG--GKAALTLSGVQPEDEADYYC | LIYYG----------------------CAQLA | FGGGT KLTVL |
| iPS:4 37040 | 21-225_196E7 | VL7|7a/JL 2 | | QTVVTQE-PSLTVSPGGTVTL TC | ASST-GAVTSA----YYPN | WLQQKPGQ APRTLIY | S--------TNNXHS | WTPARFSGSLLG--GKAALTLSDVQPEDEAEYYC | LIFYG----------------------GVHVI | FGGGT KLTVL |
| iPS:4 37048 | 21-225_197B11 | VL7|7a/JL 2 | | QTVVTQE-PSLTVSPGGTVTL TC | AFST-GSVTSG----SYPN | WFQQKPGQ APRALIY | S--------TNNXHS | WTPARFSGSLLG--GKAALTLSGVQPEDEAEYYC | LLYYG----------------------GAQLV | FGGGT KLTVL |
| iPS:4 37050 | 21-225_197C11 | VL7|7a/JL 2 | | QTVVTQE-PSLTVSPGGTVTL TC | ASST-GAVTSA----YYPN | WLQQKPGQ APRTLIY | S--------TSNXHS | WTPARFSGSLLG--GKAALTLSDVQPEDEAEYYC | LIFYG----------------------GVHVI | FGGGT KLTVL |
| iPS:4 37056 | 21-225_198B8 | VL7|7a/JL 2 | | QTVVTQE-SSLTVSPGGTVTL TC | VLST-GAVTSG----SFPN | WFQQKPGQ APRALIY | S--------TSNXHS | WTPARFSGSLLG--GKAALTLSGVQPEDEADYYF | MLYSG----------------------GAQMV | FGGGT KLTVL |
| iPS:4 37062 | 21-225_200H1 | VL7|7a/JL 2 | | QTVVTQE-PSLTVSPGGTVTL TC | ASNT-GAVTSG----SYPN | WFQQKPGQ APRALIY | H--------TNNXHS | WTPARFSGSLLG--GKAALTLSGVQPEDEAEYYC | LIYYG----------------------GAQLV | FGGGT KLTVL |
| iPS:4 37066 | 21-225_200G9 | VL7|7a/JL 2 | | QTVVTQE-PSLTVSPGGTVTL TC | ASNT-GAVTSG----SYPN | WLQQKPGQ APRALIY | H--------TDNXHS | WTPARFSGSLLG--GKAALTLSGAQPEDEAEYYC | LIYYG----------------------GAQLV | FGGGT KLTVL |
| iPS:4 37068 | 21-225_200A11 | VL7|7a/JL 2 | | QTVVTQE-PSLTVSPGGTVTL TC | ASST-GAVTSG----YYPN | WFQQKPGQ VPRALIY | S--------TNNXHS | WTPARFSGSLLG--GKAALTLSGVQPEDEADYYC | LLYYG----------------------GAHLA | FGGGT KLTVL |
| iPS:4 37090 | 21-225_210F11 | VL7|7a/JL 2 | | QTVVTQE-PSLTVSPGGTVTL TC | AFST-GAVTSG----NYPN | WFQQKPGQ APRALIY | S--------TSNXHS | WTPARFSGSLLG--GKAALTLSGVQPEDEAEYYC | LLYYG----------------------GAQLV | FGGGT KLTVL |

| ID | Name | Germline | | L_FR1 | L_CDR1 | L_FR2 | L_CDR2 | L_FR3 | L_CDR3 | L_FR4 |
|---|---|---|---|---|---|---|---|---|---|---|
| iPS:4 37106 | 21-225_211H7 | VL7\|7a/JL2 | | QTVVTQE-PSLTVSPGGTVTLTC | AFST-GAVTSG----NYPS | WFQQKPGQVPRALIY | S--------TSNRHS | WTPARFSGSLLG--GKAALTLSGVQPEDEAEYYC | LLYYG---------------------GAQLV | FGGGTKLTVL |
| iPS:4 37108 | 21-225_211C9 | VL7\|7a/JL2 | | QTVVTQE-PSLTVSPGGTVTLTC | GSST-GSVTSG----YFPN | WFQQKPGQAPRPLIY | S--------TNNKHS | WTPARFSGSLLG--GKAALTLSDVQPEDEADYYC | LLYYG---------------------GAQLA | FGGGTKLTVL |
| iPS:4 37110 | 21-225_211E9 | VL7\|7a/JL2 | | QTVVTQE-PSLTVSPGGTVTLTC | ASST-GAVTSG----NYPN | WFQQKPGQAPRALIY | S--------TINKHS | GTPARFTGFLLG--GKAALTLSGVQPEDEAEYYC | LLYYG---------------------GAQLA | FGGGTKLTVL |
| iPS:4 37120 | 21-225_212A9 | VL7\|7a/JL2 | | QTVVTQE-PSLTVSPGGTVTLTC | ASST-GAVTSG----YYPN | WFQQKPGQAPRALIY | S--------TNNKHS | WTPARFSGSLLG--GKAALTLSGVQPDDEADYYC | LLYYG---------------------GAQVG | FGGGTKLTVL |
| iPS:4 37124 | 21-225_212H12 | VL7\|7a/JL2 | | QTVVTQE-PSLTVSPGGTVTLTC | ASST-GAVTSG----YYPN | WFQQKPGQAPRALIY | S--------TSNKHS | WTPARFSGSLLG--GKAALTLSGVQPEDEAEYYC | LLYYG---------------------GAHVV | FGGGTKLTVL |
| iPS:4 37132 | 21-225_213F5 | VL7\|7a/JL2 | | QTVVTQE-PSLTVSPGGTVTLTC | GSST-GSVTSG----YFPN | WFQQKPGQTPRPLIY | S--------TNNKHS | WTPARFSGSLLG--GKTALTLSDVQPEDEADYYC | LLYFG---------------------GAQLA | FGGGTKLTVL |
| iPS:4 37136 | 21-225_214H3 | VL7\|7a/JL2 | | QTVVTQE-PSLTVSPGGTVTLTC | ASST-GAVTSG----YYPN | WFQQKPGQAPRALIY | S--------TSNKHS | CTPARFSGSLLG--GKAALTLSGVQPEDEAEYYC | LLYYG---------------------GAHVV | FGGGTKLTVL |
| iPS:4 37140 | 21-225_214E12 | VL7\|7a/JL2 | | QTVVTQE-PSLTVSPGGTVTLTC | ASST-GAVTSG----YYPN | WFQQKPGQAPRALIY | S--------TSNKHS | WTPARFSGSLLG--GKAALTLSDVQPEDEADYYC | LLYCD---------------------GAQLV | FGGGTKLTVL |
| iPS:4 37142 | 21-225_215A3 | VL7\|7a/JL2 | | QTVVTQE-PSLTVSPGGTVTLTC | ASST-EAVTSG----NYPS | WFQQKPGQAPRALIY | S--------TSNKHS | GTPARFTGSLLG--GKAALTLSGVQPEDEAEYYC | LLYYG---------------------GAQLA | FGGGTKLAVL |
| iPS:4 37148 | 21-225_215H3 | VL7\|7a/JL2 | | QTVVTQE-PSLTVSPGGTVTLTC | ASST-GAVTSG----YYPN | WFQQKPGQAPRALIY | S--------TNNKHS | CGPARFSGSLLG--GKAALTLSGVQPDDEADYYC | LLYYG---------------------GAQVG | FGGGTKLTVL |
| iPS:4 37154 | 21-225_216A7 | VL7\|7a/JL2 | | QTVVTQE-PSLTVSPGGTVTLTC | ASST-GAVTSG----YYPN | WFQQKPGQAPRALIY | S--------TNNKHS | CTPARFSGSLLG--GKAALTLSGVQPDDEADYYC | LLYYG---------------------GAQVG | FGGGTKLTVL |
| iPS:4 37158 | 21-225_216H11 | VL7\|7a/JL2 | | QTVVTQE-PSLTVSPGGTVTLTC | ASST-GAVTSG----YYPN | WFQQKPGQAPRALIY | S--------TSNKHS | WTPARFSGSLLG--GKAALTLSDVQPEDEADYYC | LLYCD---------------------GAQLV | FGGGTKLTVL |
| | | **Germline** | | **L_FR1** | **L_CDR1** | **L_FR2** | **L_CDR2** | **L_FR3** | **L_CDR3** | **L_FR4** |
| | VL1\|1b/JL3b | | 32202 | QSVLTQP-PSVSAAPGQKVTISC | SGSS-SNIGN-----NYVS | WYQQLPGTAPKLLIY | D--------NNKRPS | GIPDRFSGSKSG--TSATLGITGLQTGDEADYYC | GTWDSS---------------------LSAWV | FGGGTKLTVL |
| iPS:4 36972 | 21-225_190C7 | VL1\|1b/JL3b | | QSVLTQP-PSVSAAPGQKVTISC | SGGS-SNIGN-----NYVS | WFQQFPGTAPKFLIY | D--------NNKRPS | GIPDRFSGSKSG--TSATLGITGLQTGDEADYYC | GTWDRT---------------------LSDWV | FGGGTKLTVL |
| iPS:4 37020 | 21-225_193F11 | VL1\|1b/JL3b | | QYVLTQP-PSVSAAPGQKVTISC | FGGS-SNIGN-----NYVS | WFQQFPGTAPKFLIY | D--------NNKRPS | GILDRLSGSKSG--TSATLDITGLQNGDEADYYC | GTWDRT---------------------MSDWV | FGGGTKLTVL |

| | | | | | | | | | | |
|---|---|---|---|---|---|---|---|---|---|---|
| iPS:4 37036 | 21-225_195H9 | VL1\|1b/JL3b | | QYVLTQP-PSVSAAPGQKVTISC | SGSS-SNIGN-----NYVS | WFQQFPGT APKFLIY | D-------- NNKRPS | GILDRFSGSKSG-- TSATLGIIGLQTGDEADYYC | GIWDRI--------------------MSDWV | FGGGT KLTVL |
| | | Germline | | L_FR1 | L_CDR1 | L_FR2 | L_CDR2 | L_FR3 | L_CDR3 | L_FR4 |
| | VL1\|1b/JL1 | | 32203 | QSVLTQP-PSVSAAPGQKVTISC | SGSS-SNIGN-----NYVS | WYQQLPGT APKLLIY | D-------- NNKRPS | GIPDRFSGSKSG-- TSATLGIIGLQTGDEADYYC | GIWDSS-----------LSAYV | FGTGT KVTVL |
| iPS:4 36996 | 21-225_191B9 | VL1\|1b/JL1 | | QSVLTQP-PSVSAAPGQKVTISC | SGSS-SNIGN-----NYVS | WYQQLPGT APKLLIY | D-------- NKKRPS | GIPDRFSGSKSG-- TSATLGIIGLQTGDEADYYC | GIWDSS-----------LSVCV | FGTGT KVTVL |
| iPS:4 37054 | 21-225_194G3 | VL1\|1b/JL1 | | QSVLTQP-PSVSAAPGQKVTISC | SGSS-SNIGN-----NYIS | WYQQLPGT APKLLIY | D-------- NKKRPS | GIPDRFSGSKSG-- TSATLGIIGLQTGDEADYYC | GIWDSS-----------LSVCV | FGTGT KVTVL |
| iPS:4 37058 | 21-225_199F3 | VL1\|1b/JL1 | | QSVLTQP-PSVSAAPGQKVTISC | SGSS-SNIGN-----NYVS | WYQQLPGT APKLLIY | D-------- NNKRPS | GIPDRFSGSKSG-- TSATLGIIGLQTGDEADYYC | GIWDSS-----------LSACV | FGTGT KVTVL |
| | | Germline | | L_FR1 | L_CDR1 | L_FR2 | L_CDR2 | L_FR3 | L_CDR3 | L_FR4 |
| | VL5\|5c/JL2 | | 32204 | QAVLTQP-ASLSASPGASASLTC | TLRS-GINVGT----YRIY | WYQQKPGS PPQYLLR | YKS---- DSDKQQGS | GVPSRFSGSKDASANAGILL ISGLQSEDEADYYC | MIWHS-----------SASVV | FGGGT KLTVL |
| iPS:4 37000 | 21-225_191G9 | VL5\|5c/JL2 | | QAVSTRP-SSLSASPGASASLTC | TLRS-GINVGT----YRIY | WYQQKPGS PPQYLLR | YKS---- DSDKQQGS | GVPSRFSGSKDASANAGILL ISGLQSEDEADYYC | MIWHS-----------SAVV | FGGGT KLTVL |
| | | Germline | | L_FR1 | L_CDR1 | L_FR2 | L_CDR2 | L_FR3 | L_CDR3 | L_FR4 |
| | VL3\|3j/JL2 | | 32205 | SYELTQP-LSVSVALGQTARITC | GGN---NIGS-----KNVH | WYQQKPGQ APVLVIY | R-------- DSNRPS | GIPERFSGSNSG-- NTATLTISRAQAGDEADYYC | QVWDS-----------STAVV | FGGGT KLTVL |
| iPS:4 37074 | 21-225_203B2 | VL3\|3j/JL2 | | SYELTQP-LSVSVALGQTARITC | GGN---NIGR-----KNVH | WYQQKPGQ SPVLIIH | R-------- DSDRPS | GIPERFSGSNSG-- NTATLTISRAQAGDEADYYC | QVWDS-----------GTAV | FGGGT KLPVL |
| iPS:4 37082 | 21-225_205E12 | VL3\|3j/JL2 | | SYELTQP-LSVSAALGQTARITC | GGN---NIGR-----KNVH | WYQQKPGQ SPVLIIH | R-------- DSDRPS | GIPERFSGSNSG-- NTATLTISRAQAGDEADYYC | QVWDS-----------GTAV | FGGGT KLPVL |
| iPS:4 37084 | 21-225_206B5 | VL3\|3j/JL2 | | SYELTQP-LSVSVALGQTARIAC | GGN---NIGR-----KNVH | WYQQKPGL APVPVIX | R-------- DSYRSS | GIPDRFSGSNCG-- NTTTVTISRAQAGEEAEYYC | QDWDS-----------STVV | FGGGT KLTVL |
| iPS:4 37088 | 21-225_209H10 | VL3\|3j/JL2 | | SYELTQP-LSVSVALGQTARIAC | GGN---NIGR-----KNVH | WYQQKPGL APVPVIL | R-------- DSYRSS | GIPDRFSGSNWG-- NTATVTISRAQAGEEAEYYC | QDWDS-----------STVV | FGGGT KLTVL |
| iPS:4 37100 | 21-225_211H2 | VL3\|3j/JL2 | | SYELTQP-LSVSVALGQTARITC | GGN---NIGR-----RNVH | WYQQKPGQ APILVIY | DRDRPS | GIPERFSGSNSG-- NTATLTISRAQAGDEADYFC | QVWDS-----------STAV | FGGGT KLTVL |
| iPS:4 37160 | 21-225_216B12 | VL3\|3j/JL2 | | SYELTQP-LSVSVALGQTARITC | GGD---NIRR-----RNVH | WYQQKPGQ APVLVIY | R-------- DSNRPS | GIPERFSGSNSG-- NTATLTISRAQAGDEADYYC | QVWDS-----------STGV | FGGGT KLTVL |
| iPS:3 92593 | 21-225_3E10 | VL3\|3j/JL2 | | SYELTQP-HSVSVATAQMARITC | GGN---NIGS-----KAVH | WYQQKPGQ DPVLVIY | S-------- DSNRPS | GIPERFSGSNPG-- NTATLTISRIEAGDEADYYC | QVWDSS-----------SDHVV | FGGGT KLTVL |

| iPS:3 93204 | 21-225_8C12 | VL3\|3j/JL2 | | SYELTQP-HSVSVATAQMARITC | GGN---NIGS-----KAVH | WYQQKPGQ DPVLVIY | S--------DSNRPS | GIPERFSGSNPG--NTATLTISRIEAGDEADYYC | QVWDSS--------------------SDHVV | FGGGT KLTVL |
|---|---|---|---|---|---|---|---|---|---|---|
| | | Germline | | L_FR1 | L_CDR1 | L_FR2 | L_CDR2 | L_FR3 | L_CDR3 | L_FR4 |
| | VL2\|2a2/JL2 | | 32206 | QSALTQP-ASVSGSPGQSITISC | TGTS-SDVGGY----NYVS | WYQQHPGK APKLMIY | E--------VSNRPS | GVSNRFSGSKSG--NTASLTISGLQAEDEADYYC | SSYISS--------------STLVV | FGGGT KLTVL |
| iPS:4 37092 | 21-225_210B12 | VL2\|2a2/JL2 | | QSALTQP-ASVSGSPGQSITISC | TGTS-SDVGGY----NYVS | WYQQHPGK APKFMIY | E--------VRNRPS | GVSNRFSGSKSG--NTASLTISGLQAEDEADYYC | SSYTS--------------SRTLV | FGGGT KLTVL |
| iPS:4 37134 | 21-225_213A7 | VL2\|2a2/JL2 | | QSALTQP-ASVSGSPGQSITISC | TGTS-SDVGGY----NYVS | WYQQHPGK APKFMIY | E--------VRNRPS | GVSNRFSGSKSG--NTASLTISGLQAEDEADYYC | SSYTS--------------SRTLV | FGGGT KLTVL |
| iPS:4 72733 | 21-225_2B10_LC2 | VL2\|2a2/JL2 | | QSALTQP-ASVSGSPGQSITISC | TGTS-SDVGGY----NFVS | WYQQHPDK APKLMIY | E--------VSNRPS | GVSNRFSGSKSG--NTASLTISGLQAEDEADYYC | SSYTS--------------TGTVV | IGGGT KLTVL |
| iPS:3 92573 | 21-225_15G2 | VL2\|2a2/JL2 | | QSALTQP-ASVSGSPGQSITISC | TGTS-SDVGGY----NYVS | WYQQHPGK APKLMIY | E--------VSNRPS | GVSNRFSGSKSG--NTASLTISGLQAEDEADYYC | TSYTS--------------TSTVV | FGGGT KLTVL |
| iPS:3 93232 | 21-225_17F12 | VL2\|2a2/JL2 | | QSALTQP-ASVSGSPGQSITISC | TGAS-SDVGDY----NSVS | WYQQHPGK APKLMIY | E--------VSNRPS | GVSNRFSGSKSG--NTASLTISGLQAEDEADYYC | SSYTS--------------SITVV | FGGGT KLTVL |
| iPS:3 98494 | 21-225_21H4 | VL2\|2a2/JL2 | | QSALTQP-ASVSGSPGQSITISC | TGTS-SDVGGY----NSVS | WYQQHPDK APKLMIY | E--------VSNRPS | GVSNRFSGSKSG--NTASLTISGLQAEDEADYYC | SSYTR--------------SSTVV | FGGGT KLTVL |
| | | Germline | | L_FR1 | L_CDR1 | L_FR2 | L_CDR2 | L_FR3 | L_CDR3 | L_FR4 |
| | VL3\|3m/JL1 | | 32207 | SYELMQP-PSVSVSPGQTARITC | SGD----ALPK-----QYAY | WYQQKPGQ APVLVIY | K--------DSERPS | GIPERFSGSSSG--TTVTLTISGVQAEDEADYYC | QSADSS--------------GTYYV | FGTGT KVTVL |
| iPS:4 37102 | 21-225_211E5 | VL3\|3m/JL1 | | SYELTQP-PSVSVSPGQTARITC | SGD---ALPK-----QYAY | WYQQKPGQ APVLVIY | K--------DSARPS | GIPERFSGSRSG--TTVTLTVSGVQAEDEADYYC | QLVYS--------------SDTYV | FGTGT MLTVL |
| iPS:4 37164 | 21-225_217C6 | VL3\|3m/JL1 | | SYELTQP-PSVSVSPGQTARITC | SGD---ALPK-----QYAY | WYQQKPGQ APVLVIY | K--------DSERPS | GIPERFSGSRSG--TTVTLTIRGVQAEDEADYYC | QLIVS--------------SDTYV | FGTGT KVTVL |
| iPS:4 37166 | 21-225_217G11 | VL3\|3m/JL1 | | SYELTQP-PSVSVSPGQTARITC | SGD---ALPK-----QYAY | WYQQKPGQ APVLVIY | K--------DSERPS | GIPERFSGSRSG--TTVTLTVSGVQAEDEADYYC | QLVYS--------------SDTYV | FGTGT KVTVL |
| iPS:4 37170 | 21-225_218E5 | VL3\|3m/JL1 | | SYELTQP-PSVSVSPGQTARITC | SRD---VLPK-----QYAY | WYQQKPGQ APVLVIY | K--------DSERPS | GIPERFSGSRSG--TTVTLTIRGVQAEDEADYYC | QLVVS--------------SDTYV | FGTGT KVTVL |
| iPS:4 37196 | 21-225_226B7 | VL3\|3m/JL1 | | SFELTQP-PSVSVSPGQTARITC | SGD---ALPR-----HYVY | WYQQNPGQ APVLVIY | K--------DSERPS | GIPERFSGSSSG--TTVTLTISGVQAEDEADYYC | QSADS--------------SGTYV | FGTGT KVTVL |
| | | Germline | | L_FR1 | L_CDR1 | L_FR2 | L_CDR2 | L_FR3 | L_CDR3 | L_FR4 |
| | VL4\|4c/JL2 | | 32208 | LPVLTQP-PSASALLGASIKLTC | TLSS-EHSTY-------TIE | WYQQRPGR SPQYIMK | VKS----DGSHSKGD | GIPDRFMGSSSG--ADRYLTFSNLQSDDEAEYHC | GESHTID--------------GQVGVV | FGGGT KLTVL |

| | | Germline | | L_FR1 | L_CDR1 | L_FR2 | L_CDR2 | L_FR3 | L_CDR3 | L_FR4 |
|---|---|---|---|---|---|---|---|---|---|---|
| iPS:3 92596 | 21-225_12D8 | VL4|4c/JL2 | | LPVLTQP-PSASALLGASIKLTC | TLSS-EHSTY------TIE | WYQQRPGRSPQYIMK | VKS----DGSHSKGD | GIPDRFMGSSSG--ADRYLTFSNLQSDDEDEYHC | GESHTID-------------------GQVGVV | FGGGTKLTVL |
| iPS:3 93174 | 21-225_15D8 | VL4|4c/JL2 | | LPVLTQP-PSASALLGASIKLTC | TLSS-EHSTY------TIE | WYQQRPGRSPQYIMK | VKS----DGSHSKGD | GIPDRFMGSSSG--ADRYITFSNLQSDDEEYHC | GESHTID-------------------GQVGVV | FGGGTKLTVL |
| iPS:3 98544 | 21-225_7C8 | VL4|4c/JL2 | | LPVLTQP-PSASALLGASIKLTC | TLSS-EHSTY------TIE | WYQQRPGRSPQYIMK | VKS----DGSHSKGD | GIPDRFMGSSSG--GDRYLTFSNLQSDDEDEYHC | GESHPID-------------------GQVGVV | FGGGTKLTVL |
| | | Germline | | L_FR1 | L_CDR1 | L_FR2 | L_CDR2 | L_FR3 | L_CDR3 | L_FR4 |
| | VL3|3h/JL2 | | 32209 | SYVLTQP-PSVSVAPGKIARITC | GGN---NIGS-----KSVH | WYQQKPGQAPVLVIY | Y--------DSDRPS | GIPERFSGSNSG--NTATLTISRVEAGDEADYYC | QVWDSS-------------SDHVV | FGGGTKLTVL |
| iPS:3 93208 | 21-225_16F3 | VL3|3h/JL2 | | SYVLTQP-PSVSVAPGQIARITC | GGN---NIGS----KSVH | WYQQKPGQAPVLVVY | D--------DTDRPS | GIPERFSGSNSG--NTATLTISRVEAGDEADYYC | QVWDSS-------------SDHVV | FGGGTKLTVL |

**HEAVY_VARIABLE**

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH1|1-08/D6|6-19|RF1/JH4 | | 32210 | QVQLVQS-GAEVKKPGASVKVSCKASG-YTFT | S-----YDIN | WVRQATGQGLEWMG | WMHPN---SGNTGYAQKFQG | RVTMTRNISISTAYMELSSLRSEDTAVYYCAR | GYSSGW-------------YYFDY | WGQGTLVTVSS |
| iPS:4 26126 | 21-225_6G6 | VH1|1-08/D6|6-19|RF1/JH4 | | QVQLVQS-GAEVRKPGASVKVSCKASG-YTFT | N-----YDIN | WVRQATGQGPEWMG | WMHPN---SGNTGYAKKFQG | RVTMTRNTSISAAYMVLSSLRSEDTAVYYCAL | SSGWY-------------YFDY | WGQGTLVTVSS |
| iPS:4 12232 | 21-225_4A2 | VH1|1-08/D6|6-19|RF1/JH4 | | QVQLVQS-GTEVKKPGASVKVSCKASG-YTFT | N-----YDIN | WVRQATGQGLEWMG | WMHPN---SGNTGYAQKFQG | RVTLTRNTSISTAYMELSSLRSEDTAVYYCAS | SSGWY-------------YFDY | WGQGTLVTVSS |
| iPS:4 26112 | 21-225_12F12 | VH1|1-08/D6|6-19|RF1/JH4 | | QVQLLQS-GAEVKKPGASVKVSCKASG-YTFT | N-----YDIN | WVRQATGQGLEWMG | WMYPN---SGNTGYAQKFQG | RVTMTMNTSISTAYMELSSLRSEDTAVYYCAM | SSGWY-------------YFDF | WGQGTLVTVSS |
| iPS:4 51141 | 21-225_164B11 | VH1|1-08/D6|6-19|RF1/JH4 | | QVQLVQS-GAEVKKPGASVKVSCKASG-YTFT | N-----YDIN | WVRQATGQGLEWMG | WMTPN---SGNTGYAQKFQG | RVTMTRNTSMSTAYMELSSLRSEDSAVYYCSY | SSGWY-------------MFDY | WGQGTLVTVSS |
| iPS:4 68850 | 21-225_63F4 | VH1|1-08/D6|6-19|RF1/JH4 | | QVQLVQS-GAEVKKPGASVKVSCKASG-YTFT | N-----YDVN | WVRQATGQGLEWMG | WMHPN---SGNTGYAQKFRG | RVTMTRNTSLSTVYMELSSLRSEDTAVYYCAY | SSGWY-------------VFDY | WGQGTLVTVSS |
| iPS:4 68852 | 21-225_71F3 | VH1|1-08/D6|6-19|RF1/JH4 | | QVQLVQS-GAEVKKPGASVKVSCKASG-YTFT | N-----YDVN | WVRQATGQGLEWMG | WMHPN---SGNTGYAQKFQG | RVTMTRNTSISTAYMELSSLRSEDTAVYYCAY | SSGWY-------------VFDS | WGQGTLVTVSS |

| iPS:4 68854 | 21-225_72C4 | VH1|1-08/D6|6-19|RF1/JH4 | | QVQLVQS-GAEVKKPGASVKVSCKASG-YTFT | N-----YDIN | WVRQVTGQGLEWMG | WMHPN---SGNTGYAQKFQG | RVTMTRNTSISTAYMELNSLRSEDTAVYYCSH | SSGWY---------------------LFDY | WGQGTLVTVSS |
| iPS:4 68870 | 21-225_74A8 | VH1|1-08/D6|6-19|RF1/JH4 | | QVQLVQS-GAEVKKPGASVKVSCKASG-YTFT | N-----YDIN | WVRQASGQGLEWMG | WMHPN---SGNTGYAQKFQG | RVTMTRNTSISTAYMELSSLRSEDSAVYYCAY | SSGWY---------------------KFDY | WSQGTLVTVSS |
| iPS:4 23314 | 21-225_12F11 | VH1|1-08/D6|6-19|RF1/JH4 | | QVQLVQS-GAEVKKPGASVKVSCKASG-YTFT | N-----YDIN | WVRQATGQGPEWMG | WMHPN---SGNTGYAKKFQG | RVTMTRNTSISAAYMVLSSLRSEDTAVYYCAL | SSGWY---------------------YFDY | WGQGTLVTVSS |
| iPS:4 33909 | 21-225_43D8 | VH1|1-08/D6|6-19|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLTSEDTAVYYCAH | SSGWT---------------------LFDY | WGQGTLVTVSA |
| iPS:4 34177 | 21-225_56A1 | VH1|1-08/D6|6-19|RF1/JH4 | | QVQLVQS-GAEVKKPGASVKVSCKASG-YTFT | N-----YDIN | WVRQATGQGLEWLG | WMHPN---SGNTGYAQKFQG | RVTMTRNTSISTAYMELSSLRSEDTAVYYCAY | SSGWY---------------------VFDY | WGQGTLVTVSS |
| iPS:4 34211 | 21-225_60F3 | VH1|1-08/D6|6-19|RF1/JH4 | | QVLLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------FFDY | WGQGTLVTVSS |
| iPS:4 34235 | 21-225_61E3 | VH1|1-08/D6|6-19|RF1/JH4 | | QVQLVQS-GAEVKKPGASVKVSCKASG-YTFT | N-----YDIN | WVRQATGQGLEWMG | WMTPN---SGNTGYAQKFQG | RVTMTRNTSISTAYMELSSLKSEDTAVYYCAY | SSGWY---------------------RFDY | WGQGTLVTVSS |
| iPS:4 34237 | 21-225_61B5 | VH1|1-08/D6|6-19|RF1/JH4 | | QVQLVQS-GAEVKKPGASVKVSCKASG-YTFT | N-----YDIN | WVRQATGQGLEWMG | WMHPN---SGSTGYAQKFQG | RVTMTRNTSISTAYMELSSLRSEDTAVYYCAY | SSGWY---------------------YFDY | WGQGTLVTVSS |
| iPS:4 34295 | 21-225_58B9 | VH1|1-08/D6|6-19|RF1/JH4 | | QVQLVQS-GAEVKKPGASVKVSCKASG-YTFT | N-----YDIN | WVRQATGQGLEWMG | WMNPN---SGSTGYAQKFQG | RVTMTRNTSISTAYMELSSLRSEDTAVYYCAY | SSGWY---------------------YFDY | WGQGTLVTVSS |
| iPS:4 34305 | 21-225_59E1 | VH1|1-08/D6|6-19|RF1/JH4 | | QVQLVQS-GAEVKKPGASVKVSCKASG-YTFT | N-----YDIN | WVRQATGQGLEWMG | WMTPN---SGNTGYAQKFQG | RVTMTRTTSISTAYMELSSLRSEDTAVYYCAF | SSGWY---------------------FFDY | WGQGTLVTVSS |
| iPS:4 34321 | 21-225_59F10 | VH1|1-08/D6|6-19|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYNCAV | SSGWY---------------------YFDY | WGQGTLVTVSS |
| iPS:4 34431 | 21-225_70E7 | VH1|1-08/D6|6-19|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------VFDY | WGQGTLVTVSS |

EP 4 435 105 A2

| | | | | | | | | | | |
|---|---|---|---|---|---|---|---|---|---|---|
| iPS:4 34443 | 21-225_71G3 | VH1|1-08/D6|6-19|RF1/JH4 | | QVQLVQS-GAEVKKPGASVKVSCKASG-YTFT | N-----YDIN | WVRQATGQGLEWMG | WMHPN---SGSTGYAQKFQG | RVTMTRDISVSTAYMELSSLRSEDTAVYYCAY | SSGWY---------------------YFDY | WGQGTLVTVSS |
| iPS:4 34475 | 21-225_74F9 | VH1|1-08/D6|6-19|RF1/JH4 | | QVQLVQS-GAEVKKPGASVKVSCKASG-YTFT | N-----YDIN | WVRQATGQGLEWMG | WMNPN---SGNTGCAQKRSEDTAVYYCAS | RVTMTRNTSISTAYMELSSL | SSGWN---------------------FFDY | WGQGTLVTVSS |
| iPS:4 34477 | 21-225_74A6 | VH1|1-08/D6|6-19|RF1/JH4 | | QVQLVQS-GAEVKKPGASVKVSCKASG-YTFT | N-----YDIN | WVRQATGQGLEWMG | WMHPN---SGNTGYAQKFRG | RVTMTWNTSISTAYMELSSLRSEDTAVYYCAS | SSGWY---------------------YFDY | WGQGTLVTVSS |
| iPS:4 34487 | 21-225_76G2 | VH1|1-08/D6|6-19|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAG | SSGWY---------------------MFDY | WGQGTLVTVSS |
| iPS:4 34511 | 21-225_74B11 | VH1|1-08/D6|6-19|RF1/JH4 | | QVQLVQS-GAEVKKPGASVRVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------YFDY | WGQGTLVTVSS |
| iPS:4 34549 | 21-225_76E11 | VH1|1-08/D6|6-19|RF1/JH4 | | QVQLVQS-GAEVKKPGASVKVSCKASG-YTFT | N-----YDIN | WVRQATGQGLEWMG | WMNPN---SGNTGYAQKFQG | RLTMTRNTSISTAYMELSSLRSEDTAVFYCAY | SSGWY---------------------YFDY | WGQGTLVTVSS |
| iPS:4 34551 | 21-225_75C4 | VH1|1-08/D6|6-19|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAS | SSGWY---------------------YFDY | WGQGTLVTVSS |
| iPS:4 34635 | 21-225_78E6 | VH1|1-08/D6|6-19|RF1/JH4 | | QVQLVQS-GAEVKKPGASVKVSCKASG-YTFT | N-----YDIN | WVRQASGQGLEWMG | WMHPN---SGNTGYAQKFQG | RVTMTRNTSISTAYMELSSLRSEDSAVYYCAY | SSGWY---------------------KFDY | WSQGTLVTVSS |
| iPS:4 34649 | 21-225_78E11 | VH1|1-08/D6|6-19|RF1/JH4 | | QVQLVQS-GAEVKKPGASVKVSCKASG-YTFT | N-----YDIN | WVRQATGQGLEWMG | WMNPN---SGNTGCAQKFQG | RVTMTRNTSISTAYMELSSLRSEDTAVYYCAS | SSGWN---------------------FFDY | WGQGTLVTVSS |
| iPS:4 34665 | 21-225_74G4 | VH1|1-08/D6|6-19|RF1/JH4 | | QVQLVQS-GAEVKKPGASVKVSCKASG-YTFT | N-----YDVN | WVRQATGQGLEWMG | WMHPN---SGNTGYAQKFQG | RVTMTRNTSIRTAYMELSSLRSEDTAVYYCAS | SSGWY---------------------HFDY | WGQGTLVTVSS |
| iPS:4 34679 | 21-225_79G7 | VH1|1-08/D6|6-19|RF1/JH4 | | QVQLVQS-GAEVKKPGASVKVSCKASG-YTFT | N-----YDIN | WVRQASGQGLEWMG | WMHPN---SGNTGYAQKFQG | RVTMTRNTSISTAYMELSSLRSEDSAVYYCAY | SSGWY---------------------KFDY | WSQGTLVTVSS |
| iPS:4 34685 | 21-225_79E9 | VH1|1-08/D6|6-19|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAS | SSGWY---------------------YFDY | WGQGTLVTVSS |

| | | | | | | | | | | |
|---|---|---|---|---|---|---|---|---|---|---|
| iPS:4 34697 | 21-225_79F12 | VH1\|1-08\|D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY---------------------FFDY | WGQGTLVTLS S |
| iPS:4 34729 | 21-225_80B12 | VH1\|1-08\|D6\|6-19\|RF1/JH4 | | QVQLVQS-GAEVKKPGASVKVSCKTSG-YTFT | N-----YDIN | WVRQATGQGLEWMG | WMNPN---SGNTGYAQKFQV | RVTMTRNTSISTAYMELSSLRSEDTAVYYCAY | SSGWY---------------------IFDY | WGQGTLVTVS S |
| iPS:4 34851 | 21-225_75A6 | VH1\|1-08\|D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY---------------------IFDY | WGQGTLVTVS S |
| iPS:4 34909 | 21-225_85C11 | VH1\|1-08\|D6\|6-19\|RF1/JH4 | | QVQLVQS-GAEVKKPGASVKVSCKASG-YTFT | N-----YDIN | WVRQASGQGLEWMG | WMHPN---SGNTGYAQKFQG | RVTMTRNTSISTAYMELSSLRSEDSAVYYCAY | SSGWY---------------------KFDY | WSQGTLVTVS S |
| iPS:4 34959 | 21-225_87E10 | VH1\|1-08\|D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY---------------------FFDY | WGQGTLVTVS S |
| iPS:4 34965 | 21-225_88A1 | VH1\|1-08\|D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY---------------------YFDY | WGQGTLVTVS S |
| iPS:4 34973 | 21-225_88B4 | VH1\|1-08\|D6\|6-19\|RF1/JH4 | | QVQLVQS-GAEVKKPGASVKVSCKASG-YTFT | N-----YDIN | WVRQATGQGLEWMG | WMNPN---SGDTGYAQKFQG | SVTMTRNTSITTAYMELSSLRSEDTAVYYCSY | SSGWY---------------------YFDY | WGQGTLVTVS S |
| iPS:4 34997 | 21-225_88C10 | VH1\|1-08\|D6\|6-19\|RF1/JH4 | | QVQLVQS-GAEVKKPGASVRVSCKASG-YTFS | N-----YDIN | WVRQATGQGLEWMG | WMTPN---SGNTGYAQKFQG | RVTMTRNTSISTAYMELSSLRSEDTAVYYCAS | SSGWY---------------------YFDS | WGQGTLVTVS L |
| iPS:4 35053 | 21-225_75F9 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------IFDY | WGQGTLVTVS S |
| iPS:4 35113 | 21-225_92E6 | VH1\|1-08\|D6\|6-19\|RF1/JH4 | | QVQLVQS-GAEVKKPGASVRVSCKASG-YTFT | N-----YDIN | WVRQATGQGLEWMG | WMHPN---SGNTGYAQKFQG | RVTMTRNTSMSTAYMELSSLRSEDTAVYYCAH | SSGWY---------------------FFDY | WGQGTLVTVS S |
| iPS:4 35209 | 21-225_75A10 | VH1\|1-08\|D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY---------------------YFDY | WGQGTLVTVS S |
| iPS:4 35257 | 21-225_96H5 | VH1\|1-08\|D6\|6-19\|RF1/JH4 | | QVQLVQS-GAEVKKPGASVKVSCKASG-YTFT | N-----YDIN | WVRQASGQGLEWMG | WMHPN---SGNTGYAQKFQG | RVTMTRNTSISTAYMELSSLRSEDSAVYYCAY | SSGWY---------------------KFDY | WSQGTLVTVS S |

| | | | | | | | | | |
|---|---|---|---|---|---|---|---|---|---|
| iPS:4 35267 | 21-225_96D10 | VH1\|1-08\|D6\|6-19\|RF1/JH4 | | QVQLVQS-GAEVKKPGASVRVSCKASG-YTFT | N-----YDIN | WVRQATGQGLEWMG | WMHPN---SGNTGYAQKFQG | RVTMTRNISMSTAYMELSSLRSEDTAVYYCAH | SSGWY--------------------FFDY | WGQGTLVTVSS |
| iPS:4 35299 | 21-225_146D4 | VH1\|1-08\|D6\|6-19\|RF1/JH4 | | QVQLVQS-GAEVKKPGASVKVSCKASG-YTFT | N-----YDIN | WVRQATGQGLEWMG | WVHPN---SGNTGYAQKFQG | RVTMTRNISISTAYMELSSLRSEDTAVYYCAG | SSGWY--------------------YFDY | WGQGTLVTVSS |
| iPS:4 35305 | 21-225_146C9 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTWNTSISTAYMALSSLRSEDTAVYYCAY | SSGWY--------------------SFDY | WGQGTLVTVSS |
| iPS:4 35309 | 21-225_146F9 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAS | SSGWY--------------------FFDY | WGQGTLVTVSS |
| iPS:4 35321 | 21-225_147E4 | VH1\|1-08\|D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY--------------------FFDY | WGQGTLVTVSS |
| iPS:4 35323 | 21-225_147D5 | VH1\|1-08\|D6\|6-19\|RF1/JH4 | | QVQLVQS-GAEVKKPGASVKVSCKASG-YTFT | N-----YDIN | WVRQATGQGLEWMG | WVHPN---SGNTGYAQKFQG | RVTMTRNTSISTAYMELSSLRSGDTAVYYCAG | SSGWY--------------------YFDY | WGQGTLVTVSS |
| iPS:4 35345 | 21-225_148G3 | VH1\|1-08\|D6\|6-19\|RF1/JH4 | | QVQLVQS-GAEVKKPGASVKVSCKASG-YTFT | N-----YDIN | WVRQASGQGLEWMG | WMHPN---SGNTGYAQNRSEDTAVYYCAS | RVTMTRNTSISTAYMELSSL | SSGWY--------------------FFDY | WGQGTLVTVSS |
| iPS:4 35353 | 21-225_148F8 | VH1\|1-08\|D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY--------------------YFDY | WGQGTLVTVSS |
| iPS:4 35369 | 21-225_149A2 | VH1\|1-08\|D6\|6-19\|RF1/JH4 | | QVQLVQS-GAEVKKPGASVKVSCKASG-YTFT | N-----YDIN | WVRQATGQGLEWMG | WVHPN---SGNTGYAQKFQG | RVTMTRNTSISTAYMELSSLRSEDTAVYYCAG | SSGWY--------------------YFDY | WGQGTLVTVSS |
| iPS:4 35373 | 21-225_149E3 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLTSEDTAVYYCAY | SSGWY--------------------WFDY | WGQGTLVTVSS |
| iPS:4 35375 | 21-225_149H4 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTDYAQKFQG | RVTMTRNTSITTVYMELSSLRSEDTAVYYCTF | SSGWY--------------------YFDY | WGQGTLVTVSS |
| iPS:4 35399 | 21-225_150D2 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY--------------------YFDY | WGQGTLVTVSS |

| | | | | | | | | | | |
|---|---|---|---|---|---|---|---|---|---|---|
| iPS:4 35405 | 21-225_150B7 | VH1\|1-08/D6\|6-19\|RF1/J H4 | | QVQLVQS-GAEVKKPGASVTV SCKASG-FPFT | N-----YDIN | WVRQATGQ GLEWMG | WMHPN--- SGNTGYAQK FQG | RVTMTRNISISTAYLELSSL RSEDTAVYYCAS | SSGWY----------- ----------FFDY | WGQGT LVTVS S |
| iPS:4 35433 | 21-225_152E3 | VH1\|1-08/D6\|6-19\|RF1/J H4 | | QVQLVQS-GAEVKKPGASVKV SCKASG-YTFT | N-----YDIN | WVRQATGQ GLEWMG | WMHPN--- SGNTGYAQK FQG | RVTMTRNISISTAYMELSSL RSEDTAVYYCAS | SSGWY----------- ----------FFDY | WGQGT LVTVS S |
| iPS:4 35435 | 21-225_152H3 | VH1\|1-08/D6\|6-19\|RF1/J H4 | | QVQLVQS-GAEVKKPGASVKV SCKASG-YTFP | N-----YDIN | WVRQATGQ GLEWMG | WMHPN--- SGNTGYAQK FQG | RVTMTRNISISTAYMELSSL RSEDTAVYYCAY | SSGWY----------- ----------WFDY | WGQGT LVTVS S |
| iPS:4 35459 | 21-225_152E12 | VH1\|1-08/D6\|6-19\|RF1/J H4 | | QVQLVQS-GAEVKKPGASVKV SCKASG-YTFT | N-----YDIN | WVRQATGQ GLEWMG | WMNPN--- SGNTGYAQK FQG | RVTMTRNISISTAYMELSSL RSEDTAVYYCAY | SSGWY----------- ----------YFDY | WGQGT LVTVS S |
| iPS:4 35471 | 21-225_153F11 | VH1\|1-08/D6\|6-19\|RF1/J H4 | | QVQLVQS-GAEVKEPGASVKV SCKASG-YTFT | N-----YDIN | WVRQATGQ GLEWMG | WMNPN--- SGNTGYAQK FQG | RVTMTRNISINTAYMELSSL RSEDTAVYYCAY | SSGWY----------- ----------FFDN | WGQGT LVTVS S |
| iPS:4 35475 | 21-225_154H6 | VH1\|1-08/D6\|6-19\|RF1/J H4 | | QVQLVQS-GAEVKKPGASVKV SCKASG-YTFT | N-----YDIN | WVRQATGQ GLEWMG | WMNPN--- SGNTGYAQK FQG | RVTMTRNISISTAYMELSSL RSEDTAVYYCAY | SSGWY----------- ----------IFDY | WGQGT LVTVS S |
| iPS:4 35481 | 21-225_154A11 | VH1\|1-08/D6\|6-19\|RF1/J H4 | | QVQLVQS-GAEVKKPGASVKV SCKASG-YTFT | N-----YDIN | WVRQATGQ GLEWMG | WMNPN--- SGNTGYAQK FQG | RVTMTRNISISTAYMELSSL RSEDTAVYYCAS | SSGWY----------- ----------YFDY | WGQGT LVTVS S |
| iPS:4 35491 | 21-225_155E5 | VH1\|1-08/D6\|6-19\|RF1/J H4 | | QVQLVQS-GAEVKKPGASVKV SCKASG-YTFT | N-----YDIN | WVRQATGQ GLEWMG | WMNPN--- SGSTGYAQK FQG | RVTMTRNISISTAYMELSSL RSEDTAVYYCAF | SSGWY----------- ----------YFDY | WGQGT LVTVS S |
| iPS:4 35495 | 21-225_155B6 | VH1\|1-08/D6\|6-19\|RF1/J H4 | | QVQLVQS-GAEVKKPGASVKV SCKASG-YTFT | N-----YDIN | WVRQATGQ GLEWMG | WMNPN--- SGNTGYAQK FQG | RVTMTRNISISTAYMELSSL RSEDTAVYYCAY | SSGWY----------- ----------YFDY | WGQGT LVTVS S |
| iPS:4 35501 | 21-225_156H1 | VH1\|1-08/D6\|6-19\|RF1/J H4 | | QVQLVQS-GAEVKKPGASVKV SCKASG-YTFT | N-----YDIN | WVRQATGQ GLEWMG | WVHPN--- SGNTGYAQK FQG | RVTMTRNISISTAYMELSSL RSEDTAVYYCAG | SSGWY----------- ----------YFDY | WGQGT LVTVS S |
| iPS:4 35557 | 21-225_158B12 | VH1\|1-08/D6\|6-19\|RF1/J H4 | | QVQLVQS-GAEVKKPGASVKV SCKASG-YTFT | N-----YDIN | WVRQATGQ GLEWMG | WMHPN--- SGNTGYAQK FQG | RFTMTRNISISTAYMELSSL RSEDTAVYYCAY | SSGWY----------- ----------RFDY | WGQGT LVTVS S |
| iPS:4 35589 | 21-225_160A4 | VH1\|1-08/D6\|6-19\|RF1/J H4 | | QVQLVQS-GTEVKKPGASVKV SCKASG-YTFT | N-----YDIN | WVRQATGQ GPEWMG | WMHPN--- SGNTGYPQK FQG | RVTMTRNISISTAYMELSSL RSEDTAVYYCAY | SSGWY----------- ----------IFDY | WGQGT LVTVS S |

| | | | | | | | | | | |
|---|---|---|---|---|---|---|---|---|---|---|
| iPS:4 35623 | 21-225_162D5 | VH1\|1-08/D6\|6-19\|RF1/JH4 | | QVQLVQS-GSEVKKPGASVKVSCKASG-YTFT | N-----YDIN | WVRQATGQGLEWMG | WMHPN---SGNTGYAQKFQG | RVTMTRNISIDTAYMELSSLSSEDTAVYFCAF | SSGWY---------------------FFDY | WGQGTLVTVSS |
| iPS:4 35627 | 21-225_162F6 | VH1\|1-08/D6\|6-19\|RF1/JH4 | | QVQLVQS-GAEVRKPGASVKVSCKASG-YTFT | N-----YDIN | WVRQATGQGLEWMG | WMHPN---SGNTGYAQKFQG | RFTMTRNTSISTAYMELSSLRSEDTAVYYCAY | SSGWY---------------------RFDY | WGQGTLVTVSS |
| iPS:4 35649 | 21-225_165H2 | VH1\|1-08/D6\|6-19\|RF1/JH4 | | QVQLVQS-GAEVKKPGASVKVSCKASG-YTFT | H-----YDIN | WVRQATGQGLEWVG | WMHPN---SHKTGYAQKFQG | RVTMTRNTSNSTAYMDLSSLRSEDTAVYYCAY | SSGWY---------------------MFDY | WGQGTLVTVSS |
| iPS:4 35727 | 21-225_172E11 | VH1\|1-08/D6\|6-19\|RF1/JH4 | | QVQLVQS-GAEVKKPGASVMVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------RFDY | WGQGTLVTVSS |
| iPS:4 35751 | 21-225_175D10 | VH1\|1-08/D6\|6-19\|RF1/JH4 | | QVQLVQS-GAEVKKPGASVKVSCKASG-YTFT | N-----YDLN | WVRQATGQGLEWMG | WMHPN---SGNTGYAQKFQG | RVTMTRNTSISTVYMELSSLRSEDTAVYYCAY | SSGWY---------------------YFDY | WGQGTLVTVSS |
| iPS:4 35773 | 21-225_177B12 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------YFDF | WGQGTLVTVSS |
| iPS:4 35801 | 21-225_181E5 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY---------------------IFDY | WGQGTLVTVSS |
| iPS:4 35841 | 21-225_191D8 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAH | SSGWY---------------------YFDY | WGQGTLVTVSS |
| iPS:4 35855 | 21-225_191G3 | VH1\|1-08/D6\|6-19\|RF1/JH4 | | QVQLVQS-GAEVKKPGASVKVSCKASG-YTFT | N-----YDIN | WVRQATGQGLEWMG | RMNPN---SGNTGYAQKFQG | RVTMTRNTSISTAYMELSSLRSEDTAVYYCAH | SSGWY---------------------IFDY | WGQGTLVTVSS |
| iPS:4 35915 | 21-225_190H4 | VH1\|1-08/D6\|6-19\|RF1/JH4 | | QVQLVQS-GAEVKKPGASVKVSCKASG-NTFT | N-----YDIN | WVRQATGQGLEWMG | WMNPN---SGNTGYAQKFQG | RVTMTRNTSISTAYMELSSLRSEDTAVYYCAH | SSGWY---------------------IFDY | WGQGTLVTVSS |
| iPS:4 35925 | 21-225_190D7 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY---------------------FFDY | WGQGTLVTVSS |
| iPS:4 36021 | 21-225_193G4 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSIRTAYMELNSLRSEDTAVYYCAS | SSGWY---------------------FFDY | WGQGTLVTVSS |

| | | | | | | | | | | |
|---|---|---|---|---|---|---|---|---|---|---|
| iPS:4 36150 | 21-225_197H4 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAH | SSGWY---------------------YFDY | WGQGTLVTVSS |
| iPS:4 36154 | 21-225_197C6 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAH | SSGWY---------------------YFDY | WGQGTLVTVSS |
| iPS:4 36272 | 21-225_201F5 | VH1\|1-08/D6\|6-19\|RF1/JH4 | | QVQLVQS-GAAVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------YFDY | WGQGTLVTVSS |
| iPS:4 36550 | 21-225_224D8 | VH1\|1-08/D6\|6-19\|RF1/JH4 | | QVQLVQS-GAEVKKPGASVKVSCKASG-YTFT | N-----YDIN | WVRQATGQGLEWMG | WLYPN---SGNTGYAQKFQG | RVTMTRNTSISTAYMELSSLRSEDTAVYYCAY | SSGWY---------------------YFDY | WGQGTLVIVSS |
| iPS:4 36554 | 21-225_224C10 | VH1\|1-08/D6\|6-19\|RF1/JH4 | | QVQLVQS-GAEVKKPGASVKVSCKASG-STFT | N-----YDIN | WVRQATGQGLEWMG | WMHPN---SGNTGYAQKFQG | RVTMTRNTSISTAYMELSSLRSEDTAVYYCAY | SSGWY---------------------KFDY | WGQGTLVTVSS |
| iPS:4 36560 | 21-225_224F11 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY---------------------KFDY | WGQGTLVTVSS |
| iPS:4 36574 | 21-225_225F5 | VH1\|1-08/D6\|6-19\|RF1/JH4 | | QVQLVQS-GAEVKKPRASVKVSCKASG-HTFT | N-----YDIN | WVRQATGQGLEWMG | WMHPN---SGNTGFAQKFQG | RVTMTRNTSISTAYMELSSLRSEDTAVYYCAY | SSGWY---------------------RFDY | WGQGTLVTVSS |
| iPS:4 36584 | 21-225_225B9 | VH1\|1-08/D6\|6-19\|RF1/JH4 | | QVQLVQS-GTEVRKPGASVKVSCKASG-YTFT | N-----YDIN | WVRQATGQRLEWMG | WMHPN---SGNTGYAQKFRG | RVTMTRNTSINTAYMELNSLRSEDTAVYYCAY | SSGWT---------------------LFDY | WGQGTLVTVSS |
| iPS:4 36586 | 21-225_225F11 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSINTAYMELSSLRSEDTAVYYCAY | SSGWY---------------------RFDY | WGQGTLVTVSS |
| iPS:4 36588 | 21-225_225F12 | VH1\|1-08/D6\|6-19\|RF1/JH4 | | QVQLVQS-GAEVKKPGASVKVSCKASG-YTFT | H-----YDIN | WVRQATGQGLEWMG | WMHPN---SGNTGYAQKFQG | RVTMARNTSISTAYMELSSLRSEDTAVYYCAY | SSGWY---------------------KFDY | WGQGTLVTVSS |
| iPS:4 36590 | 21-225_225H12 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------KFDY | WGQGTLVTVSS |
| iPS:4 36598 | 21-225_226D6 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------KFDY | WGQGTLVTVSS |

| iPS:4 36600 | 21-225_226F6 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSINTAYMELSSLRSEDTAVYYCAY | SSGWY---------------RFDY | WGQGTLVTVSS |
| iPS:4 36616 | 21-225_226D11 | VH1\|1-08\|D6\|6-19\|RF1/JH4 | | QVQLVQS-GAEVKKPGASVKVSCRSSG-YTFT | N-----YDIN | WVRQATGQGLEWMG | WMHPN---SGNTGYAQKFQG | RVTMTRNTSISTAYMELSSLRSEDTAVYYCAY | SSGWY---------------YFDY | WGQGTLVTVSS |
| iPS:4 36622 | 21-225_226A12 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------YFDY | WGQGTLVTVSS |
| iPS:4 36636 | 21-225_227E6 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------KFDY | WGQGTLVTVSS |
| iPS:4 36638 | 21-225_227C7 | VH1\|1-08\|D6\|6-19\|RF1/JH4 | | QVQLVQS-GAEVKKPRASVKVSCKASG-HTFT | N-----YDIN | WVRQATGQGLEWMG | WMHPN---SGNTGYAQKFQG | RVTMTRNTSISTAYMELSSLRSEDTAVYYCAY | SSGWY---------------RFDY | WGQGTLVTVSS |
| iPS:4 36646 | 21-225_227D11 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------YFDY | WGQGTLVTVSS |
| iPS:4 46086 | 21-225_94D8 | VH1\|1-08\|D6\|6-19\|RF1/JH4 | | QVQLVQS-GAEVKKPGASVKVSCKTSG-YTFT | N-----YDIN | WVRQATGQGLEWMG | WMNPN---SGNTGYAQKFQV | RVTMTRNTSISTAYMEVSSLRSEDTAVYYCAY | SSGWY---------------IFDY | WGQGTLVTVSS |
| iPS:4 51116 | 21-225_164A4 | VH1\|1-08\|D6\|6-19\|RF1/JH4 | | QVQLVQS-GAEVKKPGASVKVSCKASG-FTFP | N-----YDIN | WVRQATGQGLEWMG | WMHPN---SGNTGYAQKFQG | RVTMTRNTSISTAYMELSSLRSEDTAVYYCAS | SSGWY---------------FFDY | WGQGTLVTVSS |
| iPS:4 51124 | 21-225_74F6 | VH1\|1-08\|D6\|6-19\|RF1/JH4 | | QVQLVQS-GAEVKKPGASVRVSCKASG-YTFT | N-----YDIN | WVRQATGQGLEWMG | WMHPN---SGNTGYAQKFQG | RVTMTRNTSMSTAYMELSSLRSEDTAVYYCAH | SSGWY---------------FFDY | WGQGTLVTVSS |
| iPS:4 51127 | 21-225_164A7 | VH1\|1-08\|D6\|6-19\|RF1/JH4 | | QVQLVQS-GAEVKKPGASVKVSCKASG-YTFT | N-----YDVN | WVRQATGQGLEWMG | WMHPN---SGNTGYAQKFQG | RVTMTRNTSTSTAYMELSSLRSEDSAVYYCAS | SSGWY---------------LFDY | WGQGTLVTVSS |
| iPS:4 51131 | 21-225_160A7 | VH1\|1-08\|D6\|6-19\|RF1/JH4 | | QVQLVQS-GAEVKKPGASVKVSCKASG-YTFT | N-----YDIN | WVRQATGQGLEWMG | WMHPH---SGNTGYAQKFQG | RVTMTRNTSINTAYMELSSLRSEDTAVYYCAH | SSGWY---------------YFDY | WGQGTLVTVSS |
| iPS:3 92786 | 21-225_24E1 | VH1\|1-08\|D6\|6-19\|RF1/JH4 | | QVQLVQS-GAEVKKPGASVKVSCKASG-YTFT | N-----YDIN | WVRQATGQGLEWMG | WMHPN---SGNTGYAQRFQG | RVTMTRNTSISTAYMELSSLRSEDTAVYYCAT | SSGWE---------------VFDY | WGQGTLVTVSS |

| | | | | | | | | | |
|---|---|---|---|---|---|---|---|---|---|
| iPS:3 92886 | 21-225_23A12 | VH1\|1-08/D6\|6-19\|RF1/JH4 | | QVQLVQS-GAEVKKPGASVKVSCKASG-YPFT | N-----YDIN | WVRQATGQGLEWMG | WMHPN---SGNTGYAQKFQG | RVTMTRNISINTAYMELSSLRSEDTAVYYCAG | SSGWY---------------------YFDY | WGQGTLVTVSS |
| iPS:3 92928 | 21-225_25A4 | VH1\|1-08/D6\|6-19\|RF1/JH4 | | QVLLVQS-GAEVKRPGASVKVSCKASG-YTFT | N-----YDIN | WVRQATGQGLEWMG | WMYPN---SGNTGYAQKFQG | RVTMTRNISISTAYMELSSLRSEDTAVYYCAS | SSGWY---------------------YFDY | WGQGTLVTVSS |
| iPS:3 92936 | 21-225_28B6 | VH1\|1-08/D6\|6-19\|RF1/JH4 | | QVLLVQS-GAEVKRPGASVKVSCKASG-YTFT | N-----YDIN | WVRQATGQGLEWMG | WMHPD---SGNTGYAQKFQG | RVTMTRNISISTAYMELSSLRSEDTAVYYCAS | SSGWY---------------------YFDY | WGQGTLVTVSS |
| iPS:3 92960 | 21-225_29E6 | VH1\|1-08/D6\|6-19\|RF1/JH4 | | QVLLVQS-GAEVKRPGASVKVSCKASG-YTFT | N-----YDIN | WVRQATGQGLEWMG | WMHPN---SGNTGYAQKFQG | RVTMTRNISISTAYMELSSLRSEDTAVYYCAS | SSGWY---------------------YFDY | WGQGTLVTVSS |
| iPS:3 92992 | 21-225_26C4 | VH1\|1-08/D6\|6-19\|RF1/JH4 | | QVQLVQS-GAEVKKPGASVKVSCKASG-YTFT | N-----YDIN | WVRQASGQGLEWMG | WMNPN---SGNTGYAQRFQG | RVTMTRNISINTAYMELSSLRSEDTAVYYCAS | SSGWY---------------------YFDY | WGQGTLVTVSS |
| iPS:3 93088 | 21-225_33D1 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGFAQKFRG | RVTMTRNISISTAYMELSSLRSEDTAVYYCAS | SSGWY---------------------FFDY | WGQGTLVTVSS |
| iPS:3 93144 | 21-225_34D2 | VH1\|1-08/D6\|6-19\|RF1/JH4 | | QVQLVQS-GAEVKKPGASVKVSCKASG-YTFT | N-----YDIN | WVRQATGQGLEWVG | WLHPN---SGTTGFAQKFRG | RVTMTRNISISTAYLELSSLRSEDTAVYYCAS | SSGWY---------------------FFDY | WGQGTLVTVSS |
| iPS:3 93368 | 21-225_29H8 | VH1\|1-08/D6\|6-19\|RF1/JH4 | | QVQLVQS-GAEVQKPGASVKVSCKASG-YTFT | N-----YDIN | WVRQATGQGLEWMG | WMNPN---SGNTGYAQRFQG | RVTMTRNISISAAYMELSSLRSEDTAVYYCAS | SSGWY---------------------YFDY | WGQGTLVTVSS |
| iPS:3 93942 | 21-225_11E5 | VH1\|1-08/D6\|6-19\|RF1/JH4 | | QVQLVQS-GPEVKKPGASVKVSCKASG-YTFT | N-----YDIN | WVRQATGQGLEWMG | WMHPN---SGATGYAQRFQG | RVTMTRNISISTAYMELSSLRSEDTAVYYCAT | SSGWE---------------------VFDY | WGQGTLVTVSS |
| iPS:3 94085 | 21-225_8B11 | VH1\|1-08/D6\|6-19\|RF1/JH4 | | QVQLVQS-GAEVKKPGASVKVSCKASG-YTFT | N-----YDIN | WVRQAAGQGLEWMG | WMHPN---SGNTGYAQKFQG | RVTMTRNISRSTAYMELSSLRSEDTAVYYCAY | SSGWY---------------------FFDY | WGQGTLVTVSS |
| iPS:3 98496 | 21-225_22D2 | VH1\|1-08/D6\|6-19\|RF1/JH4 | | QVQLVQS-GAEVKKPGASVKVSCKASG-YTFI | N-----YDIN | WVRQATGQGLEWMG | WMHPD---SGNTGYAQKFQG | RVTMTRNISISTAYMELSSLRSEDTAVYYCAY | SSGWY---------------------YFDY | WGQGTLVTVSS |
| iPS:3 98522 | 21-225_32A1 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNISISTAYMELSSLRSEDTAVYYCAS | SSGWY---------------------FFDY | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 98524 | 21-225_32A5 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGFAQKPRG | RVTMTRNISISTAYMELSSLRSEDTAVYYCSS | SSGWY--------------------FFDY | WGQGTLVTVSS |
| iPS:3 98538 | 21-225_34H7 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNISISTAYMELNSLRSEDTAVYYCAS | SSGWY--------------------FFDY | WGQGTLVTVSS |
| | VH1\|1-02/D1\|1-1\|RF1/JH4 | Germline | 32?1 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GTTGT--------------------YFDY | WGQGTLVTVSS |
| iPS:4 73253 | 21-225_7C3_LC1 | VH1\|1-02/D1\|1-1\|RF1/JH4 | | QVQLVQS-GAEVKKPGASVKVSCKASG-YTFN | D-----YYLH | WVRQAPGQGLEWMG | WIHPN---SGGTNYAQKFQG | RVTMTRDTSISTAYMELSSLRSDDTAVYSCAR | DGTS--------------------SFDY | WGQGTLVTVSS |
| iPS:4 73254 | 21-225_7C3_LC2 | VH1\|1-02/D1\|1-1\|RF1/JH4 | | QVQLVQS-GAEVKKPGASVKVSCKASG-YTFN | D-----YYLH | WVRQAPGQGLEWMG | WIHPN---SGGTNYAQKFQG | RVTMTRDTSISTAYMELSSLRSDDTAVYSCAR | DGTS--------------------SFDY | WGQGTLVTVSS |
| iPS:4 73255 | 21-225_9F12_LC1 | VH1\|1-02/D1\|1-1\|RF1/JH4 | | QVQLVQS-GAEVKKPGASVKVSCKASG-YTFA | D-----YYLH | WVRQAPGQGLEWMG | WIHPN---SGGTNFAQKFQG | RVTMTRDTSISTAYLELSSLRSDDTAFYYCAR | DGTS--------------------SFDY | WGQGTLVTVSS |
| iPS:4 73256 | 21-225_9F12_LC2 | VH1\|1-02/D1\|1-1\|RF1/JH4 | | QVQLVQS-GAEVKKPGASVKVSCKASG-YTFA | D-----YYLH | WVRQAPGQGLEWMG | WIHPN---SGGTNFAQKFQG | RVTMTRDTSISTAYLELSSLRSDDTAFYYCAR | DGTS--------------------SFDY | WGQGTLVTVSS |
| iPS:4 26108 | 21-225_10G6 | VH1\|1-02/D1\|1-1\|RF1/JH4 | | QVQLVQS-GAEVKKPGASVKVSCKASG-YTFT | A-----YHMH | WVRQAPGQGLEWMG | WINPN---NNGTNYAQKFQG | RVTMTRDTSISTAYMELSRLRSDDTAVYYCGR | DVTS--------------------SFDY | WGQGTLVTVSS |
| iPS:4 26110 | 21-225_12E9 | VH1\|1-02/D1\|1-1\|RF1/JH4 | | QVQLVQS-GAEVKKPGASVKVSCKASG-YTFT | D-----YYLH | WVRQAPGQGLEWMG | WVHPN---SGGTNFAQKFQD | RVTMTRDTSISTAYMELSSLRSDDTAIYSCAR | DGTS--------------------SFDY | WGQGTLVTVSS |
| iPS:4 53451 | 21-225 52G11 | VH1\|1-02/D1\|1-1\|RF1/JH4 | | QVQLVQS-GAEVKKPGASVKVSCKASG-YTFT | G-----YYLH | WVRQAPGQGLEWMG | WINPN---RNGTNYAQKFQG | RVTMTRDTSISTAFMELSRLRSDDTAVYYCAR | DGTS--------------------SFDY | WGQGTLVTVSS |
| iPS:4 53453 | 21-225_53F2 | VH1\|1-02/D1\|1-1\|RF1/JH4 | | QVQLVQS-GAEVKKPGASVKVSCKASG-YTFT | G-----YYLH | WVRQAPGQGLEWMG | WINPN---RNGTNYAQNFQG | RVTMTRDTSISTAYMELSRLKSDDTAVYYCAR | DGTS--------------------SFDY | WGQGTLVTVSS |
| iPS:4 34035 | 21-225_49F10 | VH1\|1-02/D1\|1-1\|RF1/JH4 | | QVQLVQS-GAEVKKPGASVKVSCKASG-YTFT | G-----YHMH | WVRQAPGQGLEWMG | WINPN---NNATNYAQNFQG | RVTLTRDTSISTAYMELSRLRSDDTAVYYCAR | DGTS--------------------SFDF | WGQGTLVTVSS |

| iPS:4 | clone | VH | | FR1 | CDR1 | FR2 | CDR2 | FR3 | CDR3 | FR4 |
|---|---|---|---|---|---|---|---|---|---|---|
| iPS:4 34065 | 21-225_50D4 | VH1|1-02/D1|1-1|RF1/JH4 | | QVQLVQS-GAEVKKPGASVKVSCKASG-YTFT | G-----YHMH | WVRQAPGQ GLEWMG | WINPN---NNATNYAQS FQG | RVTLTRDISISTAYMELSRL RSDDTAVYYCAR | DGTS----------------------SFDF | WGQGT LVTVS S |
| iPS:4 34069 | 21-225_51E9 | VH1|1-02/D1|1-1|RF1/JH4 | | QVQLVQS-GAEVKKPGASVKVSCKASR-YTFT | G-----YHIH | WVRQAPGQ GLEWMG | WINPN---TNGTQYAQK FQG | RVTMTRDISISTAYMELSRL RSDDTAVYYCAR | DGTS----------------------SFDY | WGQGT LVTVS S |
| iPS:4 34079 | 21-225_52B1 | VH1|1-02/D1|1-1|RF1/JH4 | | QVQLVQS-GAEVKKPGASVKVSCKASG-YTFT | G-----YHMQ | WVRQAPGQ GLEWMG | WINPN---SCATNYAQN FQG | RVTMTRDISISTAYLDLSRL RSDDTAVYYCAR | DGTS----------------------SFDY | WGQGT LVTVS S |
| iPS:4 34097 | 21-225_52H10 | VH1|1-02/D1|1-1|RF1/JH4 | | QVQLVQS-GAEVKKPGASVKVSCKASG-YTFT | G-----YHMQ | WVRQAPGQ GLEWMG | WINPN---NGGTQYAQK FQG | RVTMTRDISISTAYMELSRL RSDDTAVYYCAR | DGTS----------------------SFDY | WGQGT LVTVS S |
| iPS:4 34123 | 21-225_53F7 | VH1|1-02/D1|1-1|RF1/JH4 | | QVQLVQS-GAEVKKPGASVKVSCKASG-YTFT | G-----YHMH | WVRQAPGQ GLEWMG | WINPN---NNGTNYAQK FQG | RVTMTRDISISTAYMELNRL TSDDTAVYYCAR | DGTS----------------------SFDY | WGQGT LVTVS S |
| iPS:4 34189 | 21-225_56E5 | VH1|1-02/D1|1-1|RF1/JH4 | | QVQLVQS-GAEVKKPGASVKVSCQASG-YTFT | G-----YHMH | WVRQAPGQ GLEWMG | WINPN---NNATNYAQK FQG | RVTMTRDISISTAYMELRRL RSDDTAVYHCAR | DGTS----------------------SFDY | WGQGT LVTVS S |
| iPS:4 35677 | 21-225_169C10 | VH1|1-02/D1|1-1|RF1/JH4 | | QVQLVQS-GAEVKKPGASVRVSCKASG-YTFT | G-----YFMH | WVRQAPGQ GLEWMG | WIKPK---SGGTNSAQR FQG | RVTMTRDISINTAYMELNRL RSDDTAVYYCAR | GGTTVAT----------------WGVFDY | WGQGT LVTVS S |
| iPS:4 35699 | 21-225_170D6 | VH1|1-02/D1|1-1|RF1/JH4 | | QVQLVQS-GAEVKKPGASVRVSCKASG-YTFT | G-----YFIH | WVRQAPGQ GLEWMG | WIKPN---SGGTNSAQR FQG | RVTMTRDISINTAYMELNRL RSDDTAVYYCAR | GGTTVAT----------------WGVFDY | WGQGT LVTVS S |
| iPS:4 35797 | 21-225_181G2 | VH1|1-02/D1|1-1|RF1/JH4 | | QVQLVQS-GAEVKTPGASVKVSCRASG-YTFT | S-----YNMH | WVRQVPGQ GLEWMG | WINPN---NGGSNYTQK FQG | RVTMTRDISISTAYMELSRL RSDDTAVYYCAR | KF------------------------GD | WGQGT LVTVS S |
| iPS:4 35877 | 21-225_184E7 | VH1|1-02/D1|1-1|RF1/JH4 | | QVQLVQS-GAEVKTPGASVKVSCRASG-YTFT | S-----YNMH | WVRQVPGQ GLEWMG | WINPN---NGGSNYTQK FQG | RVTMTRDISISTAYMELSRL RSDDTAVYYCAR | KF------------------------GD | WGQGT LVTVS S |
| iPS:4 35685 | 21-225_185E10 | VH1|1-02/D1|1-1|RF1/JH4 | | QVQLVQS-GAEVKTPGASVKVSCRASG-YTFT | S-----YNMH | WVRQVPGQ GLEWMG | WINPN---NGGSNYTQK FQG | RVTMTRDISISTAYMELSRL RSDDTAVYYCAR | KF------------------------GD | WGQGT LVTVS S |
| iPS:4 35891 | 21-225_188H5 | VH1|1-02/D1|1-1|RF1/JH4 | | QVQLVQS-GAEVKTPGASVKVSCRASG-YTFT | S-----YNMH | WVRQVPGQ GLEWMG | WINPN---SGGSNYTQK FQG | RITMTRDISISTAYMELSRL RSDDTAVYYCAR | KF------------------------GD | WGQGT LVTVS S |

| iPS ID | Clone | VH gene | | FR1/CDR1 | CDR1 | FR2 | CDR2 | FR3 | CDR3 | FR4 |
|---|---|---|---|---|---|---|---|---|---|---|
| iPS:4 35897 | 21-225_188B9 | VH1\|1-02/D1\|1-1\|RF1/JH4 | | QVQLVQS-GAEVKTPGASVKV SCRASG-YTFT | S-----YNMH | WVRQVPGQ GLEWMG | WINPN--- SGGSNYTQK FQG | RVTMTRDTSISTAYMELSRL RSDDTAVYYCAR | KF------------------------GD | WGQGT LVTVS S |
| iPS:4 36400 | 21-225_213H7 | VH1\|1-02/D1\|1-1\|RF1/JH4 | | QVQLVQS-GAEVKKPGASVKV SCKASG-YTFT | G-----YHMH | WVRQAPGQ GLEWMG | WINPK--- SDGTNYAQK FQG | RVTMTRDTSISTAYMELSRL RSDDTAVYYCAR | EXPGS--------------------YYKY | WGQGT LVTVS S |
| iPS:4 36488 | 21-225_221A6 | VH1\|1-02/D1\|1-1\|RF1/JH4 | | QVQLVQS-GAEVKKPGASVKV SCKTSG-YTFT | G-----YYMH | WVRQAPGQ GLEWMG | WIHPN--- SGGTNYAQK FQG | RVTLTRDTSISTAYMDLSRL RSDDTAVYYCAR | DGTS--------------------SFDY | WGQGT LVTVS S |
| iPS:4 36494 | 21-225_221F12 | VH1\|1-02/D1\|1-1\|RF1/JH4 | | QVQLVQS-GAEVKKPGASVKV SCKTSG-YTFT | G-----YYMH | WVRQAPGQ GLEWMG | WIHPN--- SGGTNYAQK FQG | RVTLTRDTSISTAYMDLSRL RSDDTAVYYCAR | DGTS--------------------SFDY | WGQGT LVTVS S |
| iPS:4 36496 | 21-225_222E1 | VH1\|1-02/D1\|1-1\|RF1/JH4 | | QVQLVQS-GAEVKKPGASVKV SCKTSG-YTFT | G-----YYMH | WVRQAPGQ GLEWMG | WIHPN--- SGGTNYAQK FQG | RVTLTRDTSISTAYMDLSRL RSDDTAVYYCAR | DGTS--------------------SFDY | WGQGT LVTVS S |
| iPS:4 36508 | 21-225_222F7 | VH1\|1-02/D1\|1-1\|RF1/JH4 | | QVQLVQS-GAEVKKPGASVKV SCKTSG-YTFT | G-----YYMH | WVRQAPGQ GLEWMG | WIHPN--- SGGTNYAQK FQG | RVTLTRDTSISTAYMDLSRL RSDDTAVYYCAR | DGTS--------------------SFDY | WGQGT LVTVS S |
| iPS:4 36516 | 21-225_222C12 | VH1\|1-02/D1\|1-1\|RF1/JH4 | | QVQLVQS-GAEVKKPGASVKV SCKTSG-YTFT | G-----YYMH | WVRQAPGQ GLEWMG | WIHPN--- SGGTNYAQK FQG | RVTLTRDTSISTAYMDLSRL RSDDTAVYYCAR | DGTS--------------------SFDY | WGQGT LVTVS S |
| iPS:4 37264 | 21-225_171H12 | VH1\|1-02/D1\|1-1\|RF1/JH4 | | QVQLVQS-GAEVKKPGASVRV SCKASG-YTFT | G-----YFMH | WVRQAPGQ GLEWMG | WIKPK--- SGGTNSAQR FQG | RVTMTRDTSINTAYMELNRL RSDDTAVYYCAR | GGTTVAT----------------WGVFDY | WGQGT LVTVS S |
| iPS:4 37266 | 21-225_177A5 | VH1\|1-02/D1\|1-1\|RF1/JH4 | | QVQLVQS-GAEVKKPGASVRV SCKASG-YTFT | G-----YFMH | WVRQAPGQ GLEWMG | WIKPK--- SGGTNSAQR FQG | RVTMTRDTSINTAYMELNWL RSDDTAVYYCAR | GGTTVAT----------------WGVFDY | WGQGT LVTVS S |
| iPS:3 93080 | 21-225_34F3 | VH1\|1-02/D1\|1-1\|RF1/JH4 | | QVQLVQS-GAEVKKPGASVKV SCKASG-YTFT | G-----YHMH | WVRQAPGQ GLDWMG | WINPN-- SGGTNYAQK FQG | RVTMTRDTSISTAYMELSRL RSDDTAVYYCAR | DGTS--------------------SFDY | WGQGT LVTVS S |
| iPS:3 93084 | 21-225_35C6 | VH1\|1-02/D1\|1-1\|RF1/JH4 | | QVQLVQS-GTEVKKPGASVKV SCKASG-YTFT | G-----DYMH | WVRQAPGQ GLEWMG | WISPK--- NGGTNYAQK FQG | RVTMTRDTSISTAYMELNRL RSDDTAVYYCAR | DGTG--------------------SFDY | WGQGT LVTVS S |
| iPS:3 93086 | 21-225_36H5 | VH1\|1-02/D1\|1-1\|RF1/JH4 | | QVQLVQS-GAEVKKPGASMKV SCKASG-YTFT | D-----YHMH | WVRQAPGQ GLEWMG | WINPN--- RGGTNYAQK FQD | RVTMTRDTSISTAYMELSRL RSDDTAVYFCAR | DGTG--------------------SFDY | WGQGT LVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:3 93098 | 21-225_35G6 | VH1\|1-02/D1\|1-1\|RF1/JH4 | | QVQLVQS-GADVKKPGASVKVSCKASG-YTFT | D-----YHIH | WVRQAPGQGLEWMG | WINPN---NGGTHYAQEFQG | RVTMTRDISISTAYMELSSLRSDDTAVYYCAR | DGTG----------SFDY | WGQGTLVTVSS |
| iPS:3 93112 | 21-225_33G1 | VH1\|1-02/D1\|1-1\|RF1/JH4 | | QVQLAQS-GAEVKKPGASVKVSCKASG-YTFT | G-----YYMH | WVRQAPGQGLEWMG | WISPN---NGGTNYAQKFQG | RVTMTRDISISTAYMELSRLRSDDTAVYYCAR | DGTG----------SFDY | WGQGTLVTVSS |
| iPS:3 93116 | 21-225_34G7 | VH1\|1-02/D1\|1-1\|RF1/JH4 | | QVQLVQS-GAEVKKPGASVKVSCRASG-YTFT | D-----YHIH | WVRQAPGQGLEWMG | WINPN---NGGTHYAQKFQG | RVTMTRDISISTAYMELSRLRSDDTAVYYCAR | DGTG----------SFDY | WGQGNLVTVSS |
| iPS:3 93132 | 21-225_33H7 | VH1\|1-02/D1\|1-1\|RF1/JH4 | | QVQLVQS-GADVKKPGASVKVSCKASG-YTFT | D-----YHIH | WVRQAPGQGLEWMG | WINPN---NGGTHYAQEFQG | RVTMTRDISISTAYMELSSLRSDDTAVYHCAR | DGTG----------SFDY | WGQGTLVTVSS |
| iPS:3 93140 | 21-225_35H12 | VH1\|1-02/D1\|1-1\|RF1/JH4 | | QVQLVQS-GAEVKKPGASVKVSCKASG-YTFT | D-----YYIH | WVRQAPGQGLEWMG | WINPN---RGGTNYAQKFQG | RVTMTRDISISTAYMELSRLRSDDTAVYYCAR | DGTG----------SFDY | WGQGTLVTVSS |
| iPS:3 93954 | 21-225_4H6 | VH1\|1-02/D1\|1-1\|RF1/JH4 | | QVQLVQS-GAEVKKPGASVKVSCKASG-YTFT | D-----YYLH | WVRQAPGQGLEWMG | WIHPN---SGGTNYAQKFQG | RVTMTRDISISTAYMGLSSLRSDDTAVYYCAR | DGTS----------SFDY | WGQGTLVTVSS |
| iPS:3 98484 | 21-225_18D4 | VH1\|1-02/D1\|1-1\|RF1/JH4 | | QVQLVQS-GAEVKKPGASVKVSCKASG-YTFT | G-----YYLH | WVRQAPGQGLEWLG | WINPN---SNGTISAQKFQG | RVTMTRDISISTAYMELSRLISDDTAVYYCAR | DGTS----------SLDY | WGQGTLVTVSS |
| iPS:3 98502 | 21-225_23B11 | VH1\|1-02/D1\|1-1\|RF1/JH4 | | QVQLVQS-GAEVKKPGASVKVSCKASG-YTFT | G-----YYLH | WVRQAPGQGLEWMG | WINPN---NNGTNYAQKFQG | RVTMTRDISISTAYMELSRLRSDDTAVYYCAR | DGTS----------SFDY | WGQGTLVTVSS |
| iPS:3 98520 | 21-225_31C4 | VH1\|1-02/D1\|1-1\|RF1/JH4 | | QVQLVQS-GTEVKKPGASVKVSCKASG-YTFT | G------DYMH | WVRQAPGQGLEWMG | WISPK---NGGTNYAQKFQG | RVTMTRDISISTVYMELNRLRSDDTAVYYCAR | DGTG----------SFDY | WGQGTLVTVSS |
| iPS:4 02223 | 21-225_30A11 | VH1\|1-02/D1\|1-1\|RF1/JH4 | | QVQLVQS-GAEVKKPGASVKVSCKASG-YTFT | D-----YHMH | WVRQAPGQGLEWMG | WINPN---RGGTNYAQKFQD | RVTMTRDISISTAYMELSRLRSDDTAVYFCAR | DGTG----------SFDY | WGQGTLVTVSS |
| | Germline | | | **H_FR1** | **H_CDR1** | **H_FR2** | **H_CDR2** | **H_FR3** | **H_CDR3** | **H_FR4** |
| | VH3\|3-33/D6\|6-6\|RF1/JH6 | | 32212 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYCAR | EYSSSSYY--------YYYGMDV | WGQGTTVTVSS |
| iPS:4 26114 | 21-225_28H2 | VH3\|3-33/D6\|6-6\|RF1/JH6 | | QVQLVES-GGGVVQPGRSLRLSCAASG-FTFS | N-----YVMH | WVRQAPGKGLEWVA | VIWYD---GSNKYYADSVKG | RFTISRDNSKNTLYLQMNSLRAVDTAVYYCAR | EEYSSGW---------YDYGMDV | WGQGTTVTVSS |

| | | | | | | | | | |
|---|---|---|---|---|---|---|---|---|---|
| iPS:4 26116 | 21-225_29E2 | VH3\|3-33/D6\|6-6\|RF1/JH6 | | QVQLVES-GGGVVQPGRSLRLSCAASG-FTFS | N-----CVMH | WVRQAPGKGLEWVA | VIWYD---GSNKYYADSVKG | RITISRDNSKNTLYLQMNSLRAEDTAVYYCAR | EEYSSGW----------------YDYGMDV | WGQGTTVTVSS |
| iPS:4 68812 | 21-225_48H4 | VH3\|3-33/D6\|6-6\|RF1/JH6 | | QVQLVES-GGGVVQPGRSLRLSCAASG-FTFS | D-----SLMH | WVRQAPGKGLEWVA | VIWYD---GSNKYYTDSVKG | RFTISRDNSKNTLYLQMNSLRAEDTAVYYCAR | ENYSSGW----------------YGYGMDV | WGQGTTVTVSS |
| iPS:4 68816 | 21-225_52G8 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTALYYCAR | RYSSSW-------------------SGGMDV | WGQGTTVTVSS |
| iPS:4 68826 | 21-225_201C5 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYVDSVKG | RFTISRDNSRNTLYLQMNSLRAEDTAVYYCAR | ERYSSGL----------------YDYGMDV | WGQGTTVTVSS |
| iPS:4 68842 | 21-225_50H4 | VH3\|3-33/D6\|6-6\|RF1/JH6 | | QVQLVES-GGGVVQPGRSLRLSCAASG-FTFS | S-----YVMH | WVRQAPGKGLEWVA | AIWYD---GSNKYYADSVKG | RFTISRDNSKNTLDLQMNSLRAEDTAVYYCAR | ELYSSNW----------------YDYGMDV | WGQGTTVTVSS |
| iPS:4 68858 | 21-225_148C9 | VH3\|3-33/D6\|6-6\|RF1/JH6 | | QVQLVES-GGGVAQPGRSLRLSCAASG-FTFS | D-----YGMH | WVRQAPGKGLEWVA | VIWYD---GSNKYYADSVKG | RLTISRDNSKNTLYLQMNSLRAEDTAVYYCAR | ERYSSGW----------------YDYGLDV | WGQGTTVTVSS |
| iPS:4 68860 | 21-225_224E7 | VH3\|3-33/D6\|6-6\|RF1/JH6 | | QVQLVES-GGGVVQPGRSLSLSCAASG-FTFS | S-----YVMH | WVRQAPGKGLEWVA | VIWYD---GSNKYYADSVKG | RFTISRDNSKNTLYLQMNSLRAEDTAVYYCAR | EQYSSSW----------------YDFGLDV | WGQGTTVTVSS |
| iPS:4 33917 | 21-225_43E11 | VH3\|3-33/D6\|6-6\|RF1/JH6 | | QVQLVES-GGGVVQPGRSLRLSCAASG-FSFS | D-----YGMH | WVRQAPGKGLEWVA | VIWYD---GSNKYYADSVKG | RLTISRDNSKNTLYLQMNSLRAEDTAVYYCAR | RYVRSW-------------------VGGMDV | WGQGTTVTVSS |
| iPS:4 33919 | 21-225_44B3 | VH3\|3-33/D6\|6-6\|RF1/JH6 | | QVQLVES-GGGVVQPGRSLRLSCTASG-FTFS | D-----YGMH | WVRQAPGKGLEWVA | VIWYD---GSNKYYADSVKG | RFTISRDNSKNTLYLQMNSLRAEDTAVYYCAR | ERYSSGW----------------YDYGMDV | WGQGTTVTVSS |
| iPS:4 33923 | 21-225_44D3 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | ERYSSGL----------------YDYGMDV | WGQGTTVTVSS |
| iPS:4 33929 | 21-225_44D5 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | VPYSSSW----------------YDYGMDV | WGQGTTVTVSS |
| iPS:4 33935 | 21-225_44F9 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | EPYSSSW----------------YDYGMDV | GGQGTTVTVSS |

| iPS:4 33937 | 21-225_44B10 | VH3\|3-33/D6\|6-6\|RF1/JH6 | | QVQLVES-GGGVVQPGRSLRLSCVASG-FTFS | S-----YGMH | WVRQAPGKGLEWVA | VIWYD---GSNKYYADSVKG | RFTISRDNSKNTLYLQMNSLRAEDTAVYYCAR | RYSSSW-------------------VGGMDV | WGQGTTVTVSS |
| iPS:4 33939 | 21-225_44C10 | VH3\|3-33/D6\|6-6\|RF1/JH6 | | QVQLVES-GGGVVQPGRSLRLSCAASG-FTFS | D-----CVMH | WVRQAPGKGLEWVA | VIWYD---GSNKYYADSVKG | RFTISRDNSKNTLYLQMNSLRAEDTAVYYCAR | ERYSSGL-------------------YDYGMDV | WGQGTTVTVSS |
| iPS:4 33951 | 21-225_45B4 | VH3\|3-33/D6\|6-6\|RF1/JH6 | | QVQLVES-GGGVVQPGRSLRLSCAASG-FTFS | D-----CVMH | WVRQAPGKGLEWVA | VIWYD---GSNKYYADSVKG | RFTISRDNSKNTLYLQMNSLRAEDTAVYYCAR | ERYSSGL-------------------YDYGMDV | WGQGTTVTVSS |
| iPS:4 33955 | 21-225_45B8 | VH3\|3-33/D6\|6-6\|RF1/JH6 | | QVQLVES-GGGVVQPGRSLRLSCAASG-FTFS | D-----CVMH | WVRQAPGKGLEWVA | VIWYD---GSNKYYADSVKG | RFTISRDNSKNTLYLQMNSLRAEDTAVYYCAR | ERYSSGL-------------------YDYGMDV | WGQGTTVTVSS |
| iPS:4 33967 | 21-225_46C3 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | ERYSSGL-------------------YDYGMDV | WGQGTTVTVSS |
| iPS:4 33971 | 21-225_46D4 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | VRYSSSW-------------------YDYGMDV | WGQGTTVTVSS |
| iPS:4 33979 | 21-225_46B9 | VH3\|3-33/D6\|6-6\|RF1/JH6 | | QVQLVES-GGGVVQPGRSLRLSCSASG-FTFS | S-----YGMH | WVRQAPGKGLEWVA | VIWYD---GRNKYYADSVKG | RITISRDNSKNTLYLQMNSLRAEDTAVYYCAR | RYSSSW-------------------MGGMDV | WGQGTTVTVSS |
| iPS:4 33985 | 21-225_47C1 | VH3\|3-33/D6\|6-6\|RF1/JH6 | | QVQLVES-GGGVVQPGRSLRLSCAASG-FTFS | D-----YGMH | WVRQTPGKGLEWVA | VIWYD---GSNKYYADSVKG | RFTISRDNSKNTLFLQMNSLRDEDTAVYYCAR | RYSRSW-------------------VGGMDV | WGQGTTVTVSS |
| iPS:4 33991 | 21-225_47E7 | VH3\|3-33/D6\|6-6\|RF1/JH6 | | QVQLVES-GGGVVQPGRSLRLSCAASG-FTFS | I-----YGMH | WVRQAPGKGLEWVA | VIWYD---GSNKYYADSVKG | RFTISRDNSKNTLYLQMNSLRAEDTAVYYCAR | RYSRSW-------------------VGGMDV | WGQGTTVTVSS |
| iPS:4 34001 | 21-225_48F2 | VH3\|3-33/D6\|6-6\|RF1/JH6 | | QVQLVES-GGGVVQPGRSLRLSCAASG-FTFS | N-----YVMH | WVRQAPGKGLEWVA | VIWYD---GSNKYYADSVKG | RFTISRDNSKNTLYLQMNSLRAEDTAVYYCAR | ERYSSSW-------------------YDYGLDV | WGQGTTVTVSS |
| iPS:4 34021 | 21-225_49C1 | VH3\|3-33/D6\|6-6\|RF1/JH6 | | QVQLVES-GGGVVQPGRSLRLSCTASG-FTFS | S-----YGMH | WVRQAPGKGLEWVA | VIWYD---GSNKYYADSVKG | RFTISRDNSKNTLYLQMNSLRAEDTAVYYCAR | RYSSSW-------------------SGGMDV | WGQGTTVTVSS |
| iPS:4 34025 | 21-225_49G3 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-------------------SGGMDV | WGQGTTVTVSS |

| | | | | | | | | | | |
|---|---|---|---|---|---|---|---|---|---|---|
| iPS:4 34031 | 21-225_49E7 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTALYYCAR | RYSSSW-------------------SGGMDV | WGQGTTVTVSS |
| iPS:4 34033 | 21-225_49F9 | VH3\|3-33/D6\|6-6\|RF1/JH6 | | QVQLVES-GGGVVQPGRSLRLSCAASG-FTFS | S-----YGMH | WVRQAPGKGLEWVA | LIWYD---GRNKYYADSVKG | RFTISRDNPKNTLYLQMNSLRAEDTAVYHCAR | RYSSSW-------------------SGGMDV | WGQGTTVTVSS |
| iPS:4 34053 | 21-225_51E1 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSSKYYADSVKG | RFTISRDNSKNTLYLQMNSLRAEDTAVYYCAR | RYSSSW-------------------SGGMDV | WGQGTTVTVSS |
| iPS:4 34093 | 21-225_52D10 | VH3\|3-33/D6\|6-6\|RF1/JH6 | | QVQLVES-GGGVVQPGRSLRLSCAASG-FTFS | S-----YGMH | WVRQAPGKGLEWVA | LIWYD---GSNKYHADSVKG | RFTISRDNSKNTLYLQMNSLRAEDTAVYYCAR | RYSSSW-------------------SGGMDV | WGQGTTVTVSS |
| iPS:4 34137 | 21-225_54D4 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-------------------SGGMDV | WGQGTTVTVSS |
| iPS:4 34149 | 21-225_55H1 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-------------------SGGMDV | WGQGTTVTVSS |
| iPS:4 34151 | 21-225_55C2 | VH3\|3-33/D6\|6-6\|RF1/JH6 | | QVQLVES-GGGVVQPGRSLRLSCAASG-FTFS | S-----YGMH | WVRQAPGKGLEWVA | LIWYD---GSNKYHADSVKG | RFTISRDNSKNTLYLQMNSLRAEDTAVYYCAR | RYSSSW-------------------SGGMDV | WGQGTTVTVSS |
| iPS:4 34161 | 21-225_55F9 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GNGKYYADSVKG | RFTISRDNSKNTLYLQMNSLRAEDTAVYYCAR | RYSSSW-------------------SGGMDV | WGQGTTVTVSS |
| iPS:4 34201 | 21-225_59A12 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-------------------DGGMDV | WGQGTTVTVSS |
| iPS:4 34205 | 21-225_60G2 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSRSW-------------------TGGMDV | WGQGTTVTVSS |
| iPS:4 34223 | 21-225_60C12 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYVDSVKG | RFTISRDNSKNTLYLQMNSLRAEDTAVYYCAR | RYSRSW-------------------TGGMDV | WGQGTTVTVSS |
| iPS:4 34231 | 21-225_61F2 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | ERYSSGW-------------------YDYGMDV | WGQGTTVTVSS |

| | | | | | | | | | | |
|---|---|---|---|---|---|---|---|---|---|---|
| iPS:4 34233 | 21-225_61B3 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSRSW-----------------AGGMDV | WGQGTTVTVSS |
| iPS:4 34303 | 21-225_58H11 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYHADSVKG | RFTISRDNSKNTLYLQMNSLRAEDTAVYYCAR | RYSSSW-----------------DGGMDV | WGQGTTVTVSS |
| iPS:4 34339 | 21-225_64A4 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYADSVKG | RFTISRDNSQNTLYLQMNSLRAEDTAVYYCAR | ERYSSSW---------------YDYGMDV | WGQGTTVTVSS |
| iPS:4 34343 | 21-225_64C8 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYADSMKG | RFTISRDNSKNTLYLQMNSLRAEDTAVYYCAR | ERYSSGW---------------YDYGMDV | WGQGTTVTVSS |
| iPS:4 34387 | 21-225_66D11 | VH3\|3-33/D6\|6-6\|RF1/JH6 | | QVQLVES-GGGVVQPGRSLRLSCGASG-FTFS | S-----YGMH | WVRQAPGKGLEWVA | VIWYD---GSNKYYADSVKG | RFTISRDNSKNTLSLQMNSLRAEDTALYYCAR | EMYSSNW---------------YDYGLDV | WGQGTTVTVSS |
| iPS:4 34469 | 21-225_73C9 | VH3\|3-33/D6\|6-6\|RF1/JH6 | | QVQLVES-GGGVVQPGRSLRLSCAASG-FTFS | N-----YVMH | WVRQAPGKGLEWVA | VIWYD---GSNKYYADSVKG | RFTISRDNSKNTLYLQMNSLRAEDTAVYYCAR | ERYSSSW---------------FDYGMDV | WGQGTTVTVSS |
| iPS:4 35197 | 21-225_94F3 | VH3\|3-33/D6\|6-6\|RF1/JH6 | | QVQLVES-GGGVVQPGRSLRLSCAASG-FTFS | N-----DIMH | WVRQAPGKGLEWVA | VIWYD---GSNKYYADSVKG | RFTISRDNSKNTLYLQINSLRAEDTAVYFCAR | EKYSSGW---------------YDYGMDV | WGQGTTVTVSS |
| iPS:4 35315 | 21-225_147B2 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-----------------SGGMDV | WGQGTTVTVSS |
| iPS:4 35325 | 21-225_147H5 | VH3\|3-33/D6\|6-6\|RF1/JH6 | | QVQLVES-GGGVAQPGRSLRLSCAASG-FTFS | D-----YGMH | WVRQAPGKGLEWVA | VIWYD---GSNKYYADSVKG | RLTISRDNSKNTLYLQMNSLSAEDTAVYYCAR | ERYSSGW---------------YDYGLDV | WGQGTTVTVSS |
| iPS:4 35329 | 21-225_147A8 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-----------------TGGMDV | WGQGTTVTVSS |
| iPS:4 35349 | 21-225_148F5 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-----------------SGGMDV | WGQGTTVTVSS |
| iPS:4 35359 | 21-225_148H10 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYGDSVKG | RFTISRDNSKNTLYLQMNSLRAEDTAVYYCAR | RYSSSW-----------------SGGMDV | WGQGTTVTVSS |

| ID | Name | VH gene | FR1 | CDR1 | FR2 | CDR2 | FR3 | CDR3 | FR4 |
|---|---|---|---|---|---|---|---|---|---|
| iPS:4 35393 | 21-225_149D10 | VH3\|3-33/D6\|6-6\|RF1/JH6 | QVQLVES-GGGVAQPGRSLRLSCAASG-FTFS | D-----YGMH | WVRQAPGK GLEWVA | VIWYD--- GSNKYYADS VKG | RLTISRDNSKNTLYLQMNSL RAEDTAVYYCAR | ERYSSGW--------- -------YDYGMDV | WGQGT TVTVS S |
| iPS:4 35401 | 21-225_150E2 | VH3\|3-33/D6\|6-6\|RF1/JH6 | QVQLVES-GGGVVQPGRSLRLSCAASG-FTFS | S-----YVMH | WVRQAPGK GLEWVA | VIWYD--- GSNKYYADS VKG | RFTISRDNSKNTLYLQMNSL RAEDTAVYYCAR | EEYSSSW--------- -------YGYGMDV | WGQGT TVTVS S |
| iPS:4 35417 | 21-225_150D11 | VH3\|3-33/D6\|6-6\|RF1/JH6 | QVQLVES-GGGVVQPGRSLRLSCAASG-FTFS | S-----YGMH | WVRQAPGK GLEWVA | VIFYD--- GSNKHYADS VKG | RFTISRDNSKNTLYLQMNSL RAEDSAVYYCTR | RFSSSW--------- -------SGGMDV | WGQGT TVTVS S |
| iPS:4 35445 | 21-225_152F7 | VH3\|3-33/D6\|6-6\|RF1/JH6 | QVQLVES-GGGVVQPGRSLRLSCAASG-FTFS | S-----YIMH | WVRQAPGK GLEWVA | VIWYD--- GSNKYYADS VKG | RFTISRDNSKNTLYLQMNSL RAEDTAVYYCAR | EEYSSSW--------- -------YGYGMDV | WGQGT TVTVS S |
| iPS:4 35469 | 21-225_153G9 | VH3\|3-33/D6\|6-6\|RF1/JH6 | QVQLVES-GGGVVQPGRSLRLSCAASG-FTFS | S-----YGMH | WVRQAPGK GLEWVA | LIFYD--- GSNKYYADS VKG | RFTISRDNSKNTLYLQINSL RAEDTAVYFCAR | RYSRSW--------- -------AGGMDV | WGQGT AVTVS S |
| iPS:4 35573 | 21-225_159D8 | VH3\|3-33/D6\|6-6\|RF1/JH6 | QVQLVES-GGGVVQPGRSLRLSCAASG-FTFS | N-----YVMH | CVRQAPGK GLEWVA | VIWYD--- GSNKYYADS VKG | RFTISRDNSKNTLYLQMNSL RAEDTAVYYCAR | EFYSSGW--------- -------YDYGMDV | WGQGT TVTVS S |
| iPS:4 35681 | 21-225_169D11 | VH3\|3-33/D6\|6-6\|RF1/JH6 | QVQLVES-GGGVVQPGRSLRLSCAASG-FTFS | D-----YVMH | WVRQAPGK GLEWVA | VIWYD--- GSNKYYADS VKG | RFTISRDNSKNTLYLQLNSL RAEDTAVYYCAR | ERYSSGW--------- -------YDYGMDV | WGQGT TVTVS S |
| iPS:4 35689 | 21-225_170F3 | VH3\|3-33/D6\|6-6\|RF1/JH6 | QVQLVES-GGGVVQPGRSLRLSCAASG-FSFS | D-----YVMH | WVRQAPGK GLEWVA | VIWYD--- GSNKYYEDS VKG | RFTISRDNSKNTLYLQMNSL RAEDTAVYYCAR | ETYSSSW--------- -------YDYGMDV | WGQGT TVTVS S |
| iPS:4 35733 | 21-225_173C11 | VH3\|3-33/D6\|6-6\|RF1/JH6 | QVQLVES-GGGVVQPGRSLRLSCAASG-FTFS | S-----YGIH | WVRQAPGK GLEWVA | LIFYD--- GSNKYYADS VKG | RFTISRDNSKNTLYLHMSSL RAEDTAVYYCAR | RYSSSW--------- -------SGGMDV | WGQGT TVTVS S |
| iPS:4 35741 | 21-225_174G10 | VH3\|3-33/D6\|6-6\|RF1/JH6 | QVQLVES-GGGVVQPGRSLRLSCAASG-FTFS | D------YVMH | WVRQAPGK GLEWVA | VIWYD--- GSNKYYADS VKG | RFTISRDNSKNTLYLQLNSL RAEDTAVYYCAR | ERYSSGW--------- -------YDYGMDV | WGQGT TVTVS S |
| iPS:4 35763 | 21-225_176H12 | VH3\|3-33/D6\|6-6\|RF1/JH6 | QVQLVES-GGGVVQPGRSLRLSCAASG-FTFS | D-----YVMH | WVRQAPGK GLEWVA | VIWYD--- GSNKYYADS VKG | RFTISRDNSKNTLYLQMNSL RAEDTAVYYCAR | EKYSSNW--------- -------YDYGMDV | WGQGT TVTVS S |
| iPS:4 35767 | 21-225_177B4 | VH3\|3-33/D6\|6-6\|RF1/JH6 | QVQLVES-GGGVVQPGRSLRLSCAASG-FTFS | D-----YVMH | WVRQAPGK GLEWVA | VIWYD--- GSNKYYADS VKG | RFIVSRDNSKNTLYLQMNSL RAEDTAVYYCAR | EKYSSSW--------- -------YDYGLDV | WGQGT TVTVS S |

| | | | | | | | | | |
|---|---|---|---|---|---|---|---|---|---|
| iPS:4 35785 | 21-225_179C2 | VH3\|3-33/D6\|6-6\|RF1/JH6 | QVQLVES-GGGVVQPGRSLRLSCAASG-FIFS | S-----YGMH | WVRQAPGKGLEWVA | LIFYD---GSNKYYADSVKG | RFTISRDNSKNTLFLQMNSLRAEDTAVYYCAR | RYSGSW-------------------SGGMDV | WGQGTTVTVS |
| iPS:4 35921 | 21-225_190D6 | VH3\|3-33/D6\|6-6\|RF1/JH6 | QVQLVES-GGGVVQPGRSLRLSCAASG-FIFS | S-----FIMH | WVRQAPGRGLEWVA | VIWYD---GSNKYYADSVKG | RFTISRDNSKNTLYLQMNSLRAEDTAVYYCAR | EEYSSGW----------------FGYGMDV | WGQGTTVTVS |
| iPS:4 35961 | 21-225_192A2 | VH3\|3-33/D6\|6-6\|RF1/JH6 | QVQLVES-GGGVVQPGRSLRLSCAASG-FIFS | S-----YGMH | WVRQAPGKGLEWLA | VIWYD---GSYKYYADSVKG | RFTISRDNSKNTLYLQMNSLRAEDTAVYYCAR | EDSPYSGYA-----------LDYFYGMDV | WGQGTTVTVS |
| iPS:4 35985 | 21-225_192F6 | VH3\|3-33/D6\|6-6\|RF1/JH6 | QVQLVES-GGGVVQPGRSLRLSCAASG-FIFS | S-----FIMH | WVRQAPGRGLEWVA | VIWYD---GSNKYYVDSVKG | RFTISRDNSKNTLYLQMNSLRAEDTAVYYCAR | EEYSSGW----------------FGYGMDV | WGQGTTVTVS |
| iPS:4 36039 | 21-225_193F8 | VH3\|3-33/D6\|6-6\|RF1/JH6 | QVQLVES-GGGVVQPGRSLRLSCAASG-FIFS | I-----YGMD | WVRQAPGKGLEWVA | VIWYD---GSYKYYADSVKG | RFTISRDNSKNTLYLQMNSLRAEDTAVYYCVR | EDSPYSGYG-----------LDYYYGMDV | WGQGTTVTVS |
| iPS:4 36074 | 21-225_194F10 | VH3\|3-33/D6\|6-6\|RF1/JH6 | QVQLVES-GGGVVQPGRSLRLSCAASG-FIFS | S-----FIMH | WVRQAPGRGLEWVA | VIWYD---GSNKYYADSVKG | RFTISRDNSKNTLYLEMNSLRVEDTAVYYCAR | EEYSSGW----------------FGYGMDV | WGQGTTVTVS |
| iPS:4 36264 | 21-225_203F7 | VH3\|3-33/D6\|6-6\|RF1/JH6 | QVQLVES-GGGVVQPGRSLRLSCAASG-FIFS | D-----YVMH | WVRQAPGKGLEWVA | VIWYD---GSNKYYVDSVKG | RFTISRDNSRNTLYLQMNSLRAEDTAVYYCAR | ERYSSGL----------------YDYGMDV | WGQGTTVTVS |
| iPS:4 36274 | 21-225_204H3 | VH3\|3-33/D6\|6-6\|RF1/JH6 | QVQLVES-GGGVCQPGRSLRLSCTASG-FIFS | S-----YVMH | WVRQAPGKGLEWVA | VIWYD---GSNKYNADSVKG | RFTISRDNSKNTLYLQMNSLRAEDTAVYYCAR | EPYSSSW----------------YDYGMDV | SGQGTTVTVS |
| iPS:4 36332 | 21-225_208B2 | VH3\|3-33/D6\|6-6\|RF1/JH6 | QVQLVES-GGGVVQPGRSLRLSCAASG-FIFS | D-----CVMH | WVRQAPGKGLEWVA | VIWYD---GSNKYYVDSVKG | RFTISRDNSRNTLYLQMNSLRAEDTAVYYCAR | ERYSSGL----------------YDYGLDV | WGQGTTVTVS |
| iPS:4 36352 | 21-225_210G5 | VH3\|3-33/D6\|6-6\|RF1/JH6 | QVQLVES-GGGVVQPGRSLRLSCAASG-FIFS | D-----CVMH | WVRQAPGKGLEWVA | VIWYD---GSNKYYVDSVKG | RFTISRDNSRNTLYLQMNSLRAEDTAVYYCAR | ERYSSGL----------------YDYGLDV | WGQGTTVTVS |
| iPS:4 36386 | 21-225_212B11 | VH3\|3-33/D6\|6-6\|RF1/JH6 | QVQLVES-GGGVVQPGRSLRLSCAASG-FIFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVS |
| iPS:4 36412 | 21-225_214H9 | VH3\|3-33/D6\|6-6\|RF1/JH6 | QVQLVES-GGGVVQPGRSLRLSCAASG-FIFS | S-----YVMH | WVRQAPGKGLEWVA | VIWYD---GSNKYYADSVKG | RFTISRDNSKNTLYLQMNSLRAEDTAVFYCAR | ERYTSSW----------------YDYGMDV | WGQGTTVTVS |

| | | | | | | | | | | |
|---|---|---|---|---|---|---|---|---|---|---|
| iPS:4 36414 | 21-225_214G10 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36416 | 21-225_214G12 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36418 | 21-225_215E3 | VH3\|3-33/D6\|6-6\|RF1/JH6 | | QVQLVES-GGGVVQPGRSLRLSCAASG-FTFS | D-----YVIH | WVRQAPGKGLEWVT | VIWYD---GSNKYYADSVKG | RFTISRDNSKNTLYLQMNSVRAEDTAVYYCAR | ERYSSGW----------------YDYGMDV | WGQGTTVTVSS |
| iPS:4 36428 | 21-225_215E11 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36438 | 21-225_216E8 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36440 | 21-225_216H12 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36450 | 21-225_217E5 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36456 | 21-225_217G10 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36458 | 21-225_217H12 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36462 | 21-225_218C4 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36480 | 21-225_220F8 | VH3\|3-33/D6\|6-6\|RF1/JH6 | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36534 | 21-225_224F1 | VH3\|3-33/D6\|6-6\|RF1/JH6 | | QVQLVES-GGGVVQPGRSLRLSCAASG-FTFS | S-----YIMH | WVRQAPGKGLEWVA | VIWYD---GSNKYYADSVKG | RFTISRDNSKNTLYLQMNSLRAEDTAVYYCAR | ERYSSNW----------------YDYGMDV | WGQGTTVTVSS |

| | | | | | | | | | | |
|---|---|---|---|---|---|---|---|---|---|---|
| iPS:4 36540 | 21-225_224F3 | VH3\|3-33/D6\|6-6\|RF1/JH6 | | QVQLVES-GGGVVQPGRSLRLSCAASG-FSFS | S-----YGMH | WVRQAPGKGLEWVA | VIWYD---GSNKYYADSVKG | RFTISRDNSKNTLYLQMNSLRAEDTAVYYCAR | ERYSSSW----------------YDYGMDV | WGQGTTVTVSS |
| iPS:4 36564 | 21-225_225A1 | VH3\|3-33/D6\|6-6\|RF1/JH6 | | QVQLVES-GGGVVQPGRSLRLSCAASG-FIFS | D-----YVIH | WVRQAPGKGLEWVA | VIWYD---GSNKYYADSVKG | RFTISRDNSKNTLYLQMNSLRAEDTAVYYCAR | ERYSSGW----------------YDYGMDV | WGQGTTVTVSS |
| iPS:4 36596 | 21-225_226C6 | VH3\|3-33/D6\|6-6\|RF1/JH6 | | QVQLVES-GGGVVQPGRSLRLSCAASG-FIFS | D-----YGMH | WVRQAPGKGLEWVA | VIWYD---GSNKYYADSVKG | RFTISRDNSKNTLYLQTINSLRAEDTAVYYCAR | ERYSSSW----------------YDYGMDV | WGQGTTVTVSS |
| iPS:4 36620 | 21-225_226H11 | VH3\|3-33/D6\|6-6\|RF1/JH6 | | QVQLVES-GGGVVQPGRSLRLSCAASG-FIFS | N-----CGMH | WVRQAPGKGLEWVT | VIWYD---GSNKYYADSVKG | RFTISRDNSKNTLYLQMNSLRAEDTAVYYCAR | ELYSSSW----------------YDYGLDV | WGQGTTVTVSS |
| iPS:4 36744 | 21-225_154F4 | VH3\|3-33/D6\|6-6\|RF1/JH6 | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | EDSYCSGTSC---------PYYYYYGMDV | WGQGTTVTVSS |
| iPS:4 36946 | 21-225_183F4 | VH3\|3-33/D6\|6-6\|RF1/JH6 | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | ERTYCSGTTC---------PYYYYYGLGV | WGQGTTVTVSS |
| iPS:4 37286 | 21-225_208F1 | VH3\|3-33/D6\|6-6\|RF1/JH6 | | QVQLVES-GGGVVQPGRSLRLSCAASR-FIFS | D-----YVMH | WVRQAPGKGLEWVA | VIWYD---GSNKYYVDSVKG | RFTISRDNSRNTLYLQMNSLRAEDTAVYYCAR | ERYSSGL----------------YDYGMDV | WGQGTTVTVSS |
| iPS:4 37290 | 21-225_210G6 | VH3\|3-33/D6\|6-6\|RF1/JH6 | | QVQLVES-GGGVVQPGRSLRLSCAASG-FIFS | D-----YVMH | WVRQAPGKGLEWVA | VIWYD---GSNKYYVDSVKG | RFTISRDNSRNTLYLQMNSLRAEDTAVYYCAR | ERYSSGL----------------YDYGMDV | WGQGTTVTVSS |
| iPS:3 92634 | 21-225_17H3 | VH3\|3-33/D6\|6-6\|RF1/JH6 | | QVQLVES-GGGVVQPGRSLRLSCAASG-FIFS | N-----CVMH | WVRQAPGKGLEWVA | VIWYD---GSNKYYADSVKG | RFTISRDNSKNTLYLQMNSLRAEDTAVYYCAR | EKYSSSW----------------YDYGMDV | WGQGTTVTVSS |
| iPS:3 92742 | 21-225_20B2 | VH3\|3-33/D6\|6-6\|RF1/JH6 | | QVQLVES-GGGVVQPGRSLRLSCAASG-FIFS | N-----YVIH | WVRQAPGKGLEWVA | VIWYD---GSNKYYADSVKG | RFTISRDNSKNTLYLQMNSLRAEDTAVYSCAR | EKYSSSW----------------YDYGMDV | WGQGTTVTVSS |
| iPS:3 92836 | 21-225_22F4 | VH3\|3-33/D6\|6-6\|RF1/JH6 | | QVQLVES-GGGVVQPGRSLRLSCAASG-FIFS | D-----YGMH | WVRQAPGKGLEWVA | VIWYD---GSNKYYVDSVKG | RFTISRDNSKNTLYLQMNSLRAEDTAVYYCAR | EKYSSSW----------------YDYGMDV | WGQGTTVTVSS |
| iPS:3 92846 | 21-225_24B6 | VH3\|3-33/D6\|6-6\|RF1/JH6 | | QVQLVES-GGGVVQPGRSPRLSCAASG-FIFS | N-----YVMH | WVRQAPGKGLEWVA | VIWYD---GSNKYYADSVKG | RFTISRDNSKNTLYLQMNSLRAEDTAVYYCAR | EEYSSGW----------------YDYGMDV | WGQGTTVTVSS |

| iPS:3 92884 | 21-225_23A10 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | ERYSSGW----------------HDYGMDV | WGQGTTVTVSS |
|---|---|---|---|---|---|---|---|---|---|---|
| iPS:3 92888 | 21-225_25A2 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW----------------SGGMDV | WGQGTTVTVSS |
| iPS:3 92914 | 21-225_25D12 | VH3\|3-33/D6\|6-6\|RF1/JH6 | | QVQLVES-GGGVVQPGRSLRLSCAASG-FTFS | S-----DGMH | WVRQAPGKGLEWVA | VIWYD---GSNKYYADSVKG | RFTISRDNSKNTLYLQMNSLRAEDTAVYYCAR | ERYSSSW----------------YDYGMDV | WGQGTTVTVSS |
| iPS:3 92924 | 21-225_32H2 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLNLQMNSLRAEDTGVYYCAR | RYSSSW----------------TGGMDV | WGQGTTVTVSS |
| iPS:3 92938 | 21-225_29H4 | VH3\|3-33/D6\|6-6\|RF1/JH6 | | QVQLVES-GGGVVQPGRSLRLSCAASG-FTFS | S-----YGIH | WVRQAPGKGLEWVA | VIWYD---GSNKYYADSVKG | RFTISRDNSKNTLYLQMNSLRAEDTAVYYCAR | RYSSSW----------------SGGMDV | WGQGTTVTVSS |
| iPS:3 92974 | 21-225_26A11 | VH3\|3-33/D6\|6-6\|RF1/JH6 | | QVQLVES-GGGVVQPGRSLRLSCAASG-FTFS | N-----CVMH | WVRQAPGKGLEWVA | VIWYD---GSNKYYADSVKG | RFTISRDNSKNTLYLQMNSLRAEDTAVYYCAR | EEYSSGW----------------YDYGMDV | WGQGTTVTVSS |
| iPS:3 93012 | 21-225_26G7 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSXNTLYLQMNSLRAEDTAVYYCAR | RYSSSW----------------SGGMDV | WGQGTTVTVSS |
| iPS:3 93176 | 21-225_27E7 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | EDSYCSSTSC----------PYYYYYGMDV | WGQGTTVTVSS |
| iPS:3 93864 | 21-225_4C5 | VH3\|3-33/D6\|6-6\|RF1/JH6 | | QVQLVES-GGGVVQPGRSLRLSCAASG-FTFS | S-----YVLH | WVRQAPGKGLEWVA | VIWYD---GSNKYYADSVKG | RFTISRDNSKNTLYLQMNSLRAEDTAVYYCAR | EKYTSSW----------------YDYGMDV | WGQGTTVTVSS |
| iPS:3 93902 | 21-225_14E10 | VH3\|3-33/D6\|6-6\|RF1/JH6 | | QVQLVES-GGGVVQPGRSLRLSCAASG-FTFS | D-----YGMH | WVRQAPGKGLEWVA | VIWYD---GSNKYYADSVKG | RFTISRDNSKNTLYLQMNSLRAEDTAVYYCAR | EKYSSSW----------------YDYGMDV | WGQGTTVTVSS |
| iPS:3 93908 | 21-225_10E9 | VH3\|3-33/D6\|6-6\|RF1/JH6 | | QVQLVES-GGGVVQPGRSLRLSCAASG-FTFS | N-----YVIH | WVRQAPGKGLEWVA | VIWYD---GSNKYYADSVKG | RFTISRDNSKNTLYLQMNSLRAEDTAVYYCAR | EKYSSSW----------------YDYGMDV | WGQGTTVTVSS |
| iPS:3 93916 | 21-225_2G4 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | EKYSSSW----------------YDYGMDV | WGHGTTVTVSS |

| ID | Clone | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 93950 | 21-225_3H10 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | ERYSSGW----------------YDYGLDV | WGQGTTVTVSS |
| iPS:3 93972 | 21-225_7C9 | VH3\|3-33/D6\|6-6\|RF1/JH6 | | QVQLVES-GGGVVQPGRSLRLSCAASG-LTFS | N-----YVMH | WVRQAPGKGLEWVA | VIWYD---GSNKYYADSVKG | RFTISRDNSKNTLYLQMNSLRAEDTAVYYCAR | EXYSSSW----------------YDYGMDV | WGQGTTVTVSS |
| iPS:3 93978 | 21-225_4C12 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | EXYSSSW----------------YDYGMDV | WGHGTTVTVSS |
| iPS:3 93986 | 21-225_7G4 | VH3\|3-33/D6\|6-6\|RF1/JH6 | | QVQLVES-GGGVVQPGRSLRLSCAASG-FTFN | N-----YVMH | WVRQAPGKGLEWVA | VIWYD---GSNKYYADSVKG | RFTISRDNSKNTLYLQMTSLRAEDTAVYYCAR | EXYSSNW----------------YDYGMDV | WGQGTTVTVSS |
| iPS:3 93996 | 21-225_15C11 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYADSVKG | RFTISRDNSKNTLYLQMNSLRAEDTAVYYCAR | EXYSSSW----------------YDYGLDV | WGQGTTVTVSS |
| iPS:3 94041 | 21-225_5E5 | VH3\|3-33/D6\|6-6\|RF1/JH6 | | QVQLVES-GGGVVQPGRSLRLSCAASG-FTFS | N-----YVMH | WVRQAPGKGLEWVA | VIWYD---GSNKYYADSVKG | RFTISRDNSKNTLYLQMNSLRAEDTAVYYCTR | EVYSSGW----------------YDYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D2\|2-8\|RF3/JH4 | | 322 3 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DIVLMVY----------------AIYFDY | WGQGTLVTVSS |
| iPS:4 26118 | 21-225_7A10 | VH3\|3-33/D2\|2-8\|RF3/JH4 | | QVQLVES-GGGVVQPGRSLRLSCAASG-FNFS | S-----YGMH | WVRQAPGKGLEWMA | VIWYD---GSNKYYADSVKG | RFTISRDNSKNTLYLQMHSLRAEDTAVYYCAR | DERLG--------------------IFDY | WGQGTLVTVSS |
| iPS:3 93844 | 21-225_3G7 | VH3\|3-33/D2\|2-8\|RF3/JH4 | | QVQLVES-GGGVVQPGRSLRLSCAASG-FNFS | N-----YGMH | WVRQAPGKGLEWVA | VIWHD---GSNKYYVDSVKG | RFTISRDNSKNTVYLQMNSLRAEDTAVYYCAR | DERLG--------------------IFDY | WGQGTLVTVSS |
| iPS:3 93852 | 21-225_12A10 | VH3\|3-33/D2\|2-8\|RF3/JH4 | | QVQLVES-GGGVVQPGRSLRLSCAASG-FTFS | S-----YGMH | WVRQAPGKGLEWVA | VIWHD---ESNKYYTDSVKG | RFTISRDNSKNTLYLQMNSLRAEDTAVYYCAR | DERLG--------------------IFDY | WGQGTLVTVSS |
| iPS:3 93868 | 21-225_9C11 | VH3\|3-33/D2\|2-8\|RF3/JH4 | | QVQLVES-GGGVVQPGRSLRLSCAASG-FTFS | S-----YGMH | WVRQAPGKGLEWVA | VIWHD---ETNKYYADSVKG | RFTISRDNSKNTLSLQMNSLRAEDTAVYYCAR | DERLG--------------------IFDY | WGQGTLVTVSS |
| iPS:3 93900 | 21-225_10E12 | VH3\|3-33/D2\|2-8\|RF3/JH4 | | QVQLVES-GGGVVQPGRSLRLSCAASG-FNFS | N-----YGMH | WVRQVPGKGLEWVA | VIWHD---GSNKYYVDSVKG | RFTISRDNSKNTLSLQMNSLRAEDTAVYCCAR | DERLG--------------------IFDY | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 93920 | 21-225_1H12 | VH3\|3-33/D2\|2-8\|RF3/JH4 | | QVQLVES-GGGVVQSGRSLRLSCAASG-FNFS | S-----YGMH | WVRQAPGK GLEWVA | IIWHD---GSNKYYVDS VKG | RFTISRDNSKNTLYLQMNSL RAEDTAVYYCAR | DERLG---------------------IFDY | WGQGT LVTVS S |
| iPS:3 93932 | 21-225_10F5 | VH3\|3-33/D2\|2-8\|RF3/JH4 | | QVQLVES-GGGVVQPGRSLRLSCAASG-FNFS | S-----YGMH | WVRQAPGK GLEWVS | IIWHD---GSNKYYVDS VKG | RFTISRDNSKNTLYLQMNSL RAEDTAVYYCAR | DERLG---------------------IFDY | WGQGT LVTVS S |
| | | VH3\|3-33/D6\|6-6\|RF1/JH4 | 32214 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAR | EYSSS---------------SYFDY | WGQGT LVTVS S |
| iPS:4 26124 | 21-225_32D6 | VH3\|3-33/D6\|6-6\|RF1/JH4 | | QVQLVES-GGGVVQPGRSLRLSCAASG-FTFS | S-----YGMH | WVRQAPGK GLEWVT | VIWHD---GSNAYYADS VKG | RFTISRDNSKNTLYLQMNSL RAEDTAVYYCAR | ENSSS-----------------YYFDY | WGQGT LVTVS S |
| iPS:3 92922 | 21-225_30G4 | VH3\|3-33/D6\|6-6\|RF1/JH4 | | QVQLVES-GGGVVQPGRSLRLSCAASG-FTFS | S-----YGMH | WVRQAPGK GLEWVA | VIWYD---GTDKYYVDS VKG | RFTISRDNSKNTLYLQMNSL RAEDSAVYYCAR | ENSSS-----------------YYFDY | WGQGT LVTVS S |
| iPS:3 93002 | 21-225_30G1 | VH3\|3-33/D6\|6-6\|RF1/JH4 | | QVQLVES-GGGVVQPGRSLRLSCAASG-FTFS | Y-----YGMH | WVRQAPGK GLEWVA | VIWHD---GSNKYYVDS VKG | RFTISRDNSKNTLYLQMNSL RAEDTAVYYCAR | ENSSS-----------------YYFDY | WGQGT LVTVS S |
| iPS:3 93066 | 21-225_34D3 | VH3\|3-33/D6\|6-6\|RF1/JH4 | | QVQLVES-GGGVVQPGRSLRLSCAASG-FTFS | Y-----YGMH | WVRQAPGK GLEWVA | VIWHD---GSNKYYVDS VKG | RFTISRDNSKNTLYLQMNSL RAEDTAVYYCAR | ENSSS-----------------FYFDY | WGQGT LVTVS S |
| iPS:3 93092 | 21-225_33C12 | VH3\|3-33/D6\|6-6\|RF1/JH4 | | QVQLVES-GGGVVQPGRSLRLSCAASG-FTFS | H-----YGMH | WVRQAPGK GLEWVA | VIWYD---GSNKYYADS VKG | RFTISRDNSKNTLYLQMNSL RAEDTAVYYCAR | ENSSS-----------------YYFDY | WGQGT LVTVS S |
| iPS:3 93100 | 21-225_36B8 | VH3\|3-33/D6\|6-6\|RF1/JH4 | | QVQLVES-GGGVVQPGRSLRLSCAASG-FTFS | N-----YGMH | WVRQAPGK GLEWVA | VIWHD---GSNKYYGDS VKG | RFTISRDNSKNTLYLQMNSL RAEDTAVYYCAR | ENSSS-----------------YYFDY | WGQGT LVTVS S |
| iPS:3 93122 | 21-225_33B2 | VH3\|3-33/D6\|6-6\|RF1/JH4 | | QVQLVES-GGGVVQPGRSLRLSCAASG-FTFS | N-----YGMH | WVRQAPGK GLEWVA | VIWHD---GSNKYYADS VKG | RFTISRDNSKNTLYLQMNSL RAEDTAVYYCAR | ENSSS-----------------YYFDY | WGQGT LVTVS S |
| iPS:3 93134 | 21-225_34C2 | VH3\|3-33/D6\|6-6\|RF1/JH4 | | QVQLVES-GGGVVQPGRSLRLSCAASG-FTFS | N-----YVMH | WVRQAPGK GLEWVA | LIWYD---GSNKYYADS VKG | RFTISRDNSKNTLYLQMNSL RAEDTAVYYCAR | ENSSS-----------------YYFDY | WGQGT LVTVS S |
| iPS:3 93136 | 21-225_34D8 | VH3\|3-33/D6\|6-6\|RF1/JH4 | | QVQLVES-GGGVVQPGRSLRLSCAASG-FTFS | N-----YGMH | WVRQAPGK GLEWVA | VIWYD---GSNKYYADS VKG | RFTISRDNSKNTLYLQMNSL RAEDTAVYYCAR | ENSSS-----------------YYFDY | WGQGT LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-30.3/D6\|6-19\|RF2/JH6 | | 32215 | QVQLVES-GGGVVQPGRSLRL SCAASG-FTFS | S-----YAMH | WVRQAPGK GLEWVA | VISYD--- GSNKYYADS VKG | RFTISRDNSKNTLYLQMNSL RAEDTAVYYCAR | GIAVAGYY--------- --------YYYGMDV | WGQGT TVTVS S |
| iPS:4 51135 | 21-225_64A11 | VH3\|3-30.3/D6\|6-19\|RF2/JH6 | | QVQLVES-GGGVVQPGRSLRL SCAASG-FTFS | N-----YGMH | WVRQAPGK GLEWVA | VISYV--- GSNKYYADS VKG | RFTISRDNSKNTLYLQMNTL RAEDTAVYYCAR | RGAVAP---------- --------YYGMDV | WGQGT TVTVS S |
| | | Germline | | H_FR1 | H_CDR1 | H_FR2 | H_CDR2 | H_FR3 | H_CDR3 | H_FR4 |
| | VH1\|1-08/D6\|6-19\|RF1/JH5 | | 32216 | QVQLVQS-GAEVKKPGASVKV SCKASG-YTFT | S-----YDIN | WVRQATGQ GLEWMG | WMNPN--- SGNTGYAQK FQG | RVTMTRNISISTAYMELSSL RSEDTAVYYCAR | GYSSGW---------- --------YNWFDP | WGQGT LVTVS S |
| iPS:4 51137 | 21-225_74A7 | VH1\|1-08/D6\|6-19\|RF1/JH5 | | QVQLVQS-GAEVKKPGASVKV SCKASG-YTFT | N-----YDIN | WVRQATGQ GLEWMG | WMNPN--- SGNTGYAQK FQG | RVTMTRNISTSTAHMELSSL RSEDTAVYYCAV | SSGWN----------- ----------WFDP | WGQGT LVTVS S |
| iPS:4 34285 | 21-225_57A11 | VH1\|1-08/D6\|6-19\|RF1/JH5 | | QVQLVQS-GAEVKKPGASVKV SCKASG-YTFT | N-----YDIN | WVRQATGQ GLEWMG | WMNPN--- SVNTGYAQK FQG | RVTMTRDTSISTAYMELSSL RSEDTAVYYCAI | SSGWN----------- ----------WFDP | WGQGT LVTVS S |
| iPS:4 34287 | 21-225_57F12 | VH1\|1-08/D6\|6-19\|RF1/JH5 | | QVQLVQS-GAEVKKPGASVKV SCKASG-YTFT | N-----YDIN | WVRQATGQ GLEWMG | WMNPN--- SGNTGYAQK FQG | RVTMTRNTSISTAYMELSSL RSEDTAVYYCAI | SSGWY----------- ----------RFDP | WGQGT LVTVS S |
| iPS:4 34479 | 21-225_76H1 | VH1\|1-08/D6\|6-19\|RF1/JH5 | | QVQLVQS-GAEVKKPGASVKV SCKASG-YTFT | N-----YDIN | WVRQVPGQ GLEWMG | WMHPN--- SGNTGYAQK FQG | RVTMTRNTSISTAYMELSSL RSEDTAVYYCAI | SSGWY----------- ----------WFDP | WGQGT LVTVS S |
| iPS:4 34481 | 21-225_74B10 | VH1\|1-08/D6\|6-19\|RF1/JH5 | | QVQLVQS-GAEVKKPGASVKV SCKASG-FTFT | N-----YDIN | WVRQATGQ GLEWMG | WMHPN--- SGNTGFAQK FQG | RVTMTRNTSISTAYMELSSL RSEDTAVYYCAV | SSGWY----------- ----------WFDP | WGQGT LVTVS S |
| iPS:4 34483 | 21-225_74C12 | VH1\|1-08/D6\|6-19\|RF1/JH5 | | QVQLVQS-GAEVKKPGASVKV SCKASG-YTFT | N-----YDIN | WVRQATGQ GLEWMG | WMNPN--- SGNTGYAQK FQG | RVTMTRNTSISTAYMELSSL RSEDTAVYYCAI | SSGWY----------- ----------WFDP | WGQGT LVTVS S |
| iPS:4 34493 | 21-225_76F3 | VH1\|1-08/D6\|6-19\|RF1/JH5 | | QVQLVQS-GAEVKKPGASVKV SCKASG-YTFT | N-----YDIN | WVRQATGQ GLEWMG | WMNPN--- SGNTGYAQK FQG | RVTMTRNTSTSTAHMELSSL RSEDTAVYYCAV | SSGWN----------- ----------WFDP | WGQGT LVTVS S |
| iPS:4 34509 | 21-225_76F5 | VH1\|1-08/D6\|6-19\|RF1/JH5 | | QVQLVQS-GAEVKKPGASVKV SCKASG-YTFT | N-----YDIN | WVRQATGQ GLEWMG | WMNPN--- SGNTGYAQK FQG | RVTMTRNTSISTAYMELSSL RSEDTAVYYCAS | SSGWY----------- ----------WFDP | WGQGT LVTVS S |
| iPS:4 34513 | 21-225_76A6 | VH1\|1-08/D6\|6-19\|RF1/JH5 | | QVQLVQS-GAEVKKPGASVKV SCKASG-YTFT | N-----YDIN | WVRQATGQ GLEWMG | WMHPN--- NGNTGYAQK FQG | RVTMTRNTSISTAYMELNSL RSEDTAVYYCAI | SSGWY----------- ----------WFDP | WGQGT LVTVS L |

| ID | Clone | V gene | FR1/CDR1 | CDR1 | FR2 | CDR2 | FR3 | CDR3 | FR4 |
|---|---|---|---|---|---|---|---|---|---|
| iPS:4 34515 | 21-225_74A5 | VH1\|1-08\|D6\|6-19\|RF1/JH5 | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVS |
| iPS:4 34525 | 21-225_76E8 | VH1\|1-08\|D6\|6-19\|RF1/JH5 | QVQLVQS-GAEVKKPGASVKVSCKASG-YTFP | N-----YDIN | WVRQATGQGLEWMG | WMNPN---SGNTGYAQKFQG | RVTMTRNTSISTAYMELSSLRSEDTAVYYCAV | SSGWH---------------------WFDP | WGQGTLVTVAS |
| iPS:4 34529 | 21-225_76B9 | VH1\|1-08\|D6\|6-19\|RF1/JH5 | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVS |
| iPS:4 34575 | 21-225_77C7 | VH1\|1-08\|D6\|6-19\|RF1/JH5 | QVQLVQS-GAEVKKPGASVKVSCKASG-YTFP | N-----YDIN | WVRQATGQGLEWMG | WMNPN---SGNTGYAQKFQG | RVTMTRNTSISTAYMELSSLRSEDTAVYYCAV | SSGWH---------------------WFDP | WGQGTLVTVAS |
| iPS:4 34583 | 21-225_74B6 | VH1\|1-08\|D6\|6-19\|RF1/JH5 | QVQLVQS-GAEVKKPGASVKVSCKASG-YTFT | N-----YDIN | WVRQATGQGLEWMG | WMHPN---NGNTGYAQKFQG | RVTMTRNTSISTAYMELNSLRSEDTAVYYCAI | SSGWY---------------------WFDP | WGQGTLVTVSL |
| iPS:4 34587 | 21-225_74G3 | VH1\|1-08\|D6\|6-19\|RF1/JH5 | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVS |
| iPS:4 34597 | 21-225_77C10 | VH1\|1-08\|D6\|6-19\|RF1/JH5 | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY---------------------WFDP | WGQGTLVTVS |
| iPS:4 34603 | 21-225_77D11 | VH1\|1-08\|D6\|6-19\|RF1/JH5 | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVS |
| iPS:4 34613 | 21-225_77D12 | VH1\|1-08\|D6\|6-19\|RF1/JH5 | QVQLVQS-GAEVKKPGASVKVSCKASG-YTFT | N-----YDIN | WVRQATGQGLEWMG | WMNPN---SGNTGYAQKFQG | RVTMTRNTSINTAYMELSSLRSEDTAVYYCAI | SSGWY---------------------WFDP | WGQGTLVTVS |
| iPS:4 34617 | 21-225_74B8 | VH1\|1-08\|D6\|6-19\|RF1/JH5 | QVQLVQS-GAEVKKPGASVKVSCKASG-YTFT | N-----YDIN | WVRQATGQGLEWMG | WMHPN---SGNTGYAQKFQG | RVTLTRNTSISTAYMELSSLRSEDTAVYYCAV | SSGWY---------------------WFDP | WGQGTLVTVS |
| iPS:4 34619 | 21-225_78C1 | VH1\|1-08\|D6\|6-19\|RF1/JH5 | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY---------------------WFDP | WGQGTLVTVS |
| iPS:4 34639 | 21-225_74B7 | VH1\|1-08\|D6\|6-19\|RF1/JH5 | QVQLVQS-GAEVKKPGASVKVSCKASG-YTFP | N-----YDIN | WVRQATGQGLEWMG | WMNPN---SGNTGYAQKFQG | RVTMTRNTSISTAYMELSSLRSEDTAVYYCAV | SSGWH---------------------WFDP | WGQGTLVTVAS |

| | | | | | | | | | | |
|---|---|---|---|---|---|---|---|---|---|---|
| iPS:4 34653 | 21-225_74B5 | VH1\|1-08\|D6\|6-19\|RF1/J H5 | | QVQLVQS-GAEVKKPGASVKV SCKASG-YTFT | N-----YDIN | WVRQATGQ GLEWMG | WMNPN---SGNTGYAQK FQG | RVTMTRNTSTSTAHMELSSL RSEDTAVYYCAV | SSGWN--------------------WFDP | WGQGT LVTVS S |
| iPS:4 34655 | 21-225_78H12 | VH1\|1-08\|D6\|6-19\|RF1/J H5 | | QVQLVQS-GAEVKKPGASVKV SCKASG-YTFP | N-----YDIN | WVRQATGQ GLEWMG | WMNPN---SGNTGYAQK FQG | RVTMTRNTSISTAYMELSSL RSEDTAVYYCAV | SSGWH--------------------WFDP | WGQGT LVTVA S |
| iPS:4 34675 | 21-225_79G6 | VH1\|1-08\|D6\|6-19\|RF1/J H5 | | QVQLVQS-GAEVKKPGASVKV SCKASG-FTFT | N-----YDIN | WVRQATGQ GLEWMG | WMHPN---SGNTGFAQK FQG | RVTMTRNTSISTAYMELSSL RSEDTAVYYCAV | SSGWY--------------------WFDP | WGQGT LVTVS S |
| iPS:4 34689 | 21-225_79G10 | VH1\|1-08\|D6\|6-19\|RF1/J H5 | | QVQLVQS-GAEVKKPGASVKV SCKASG-YTFT | N-----YDIN | WVRQATGQ GLEWMG | WMNPN---SGNTGYAQK FQG | RVTMTRNTSTSTAHMELSSL RSEDTAVYYCAV | SSGWN--------------------WFDP | WGQGT LVTVS S |
| iPS:4 34705 | 21-225_80A2 | VH1\|1-08\|D6\|6-19\|RF1/J H5 | | QVQLVQS-GAEVKKPGASVKV SCKASG-YTFT | N-----YDIN | WVRQVPGQ GLEWMG | WMHPN---SGNTGYAQK FQG | RVTMTRNTSISTAYMELSSL RSEDTAVYYCAI | SSGWY--------------------WFDP | WGQGT LVTVS S |
| iPS:4 34707 | 21-225_80D3 | VH1\|1-08\|D6\|6-19\|RF1/J H5 | | QVQLVQS-GAEVKKPGASVKV SCKASG-YTFT | N-----YDIN | WVRQATGQ GLEWMG | WMHPN---SGNTGYAQK FQG | RVTMTRNTSISTAYMELSSL RSEDTAVYYCAI | SSGWY--------------------WFDP | WGQGT LVTVS S |
| iPS:4 34731 | 21-225_80E9 | VH1\|1-08\|D6\|6-19\|RF1/J H5 | | QVQLVQS-GAEVKKPGASVKV SCKASG-YTFT | N-----YDIN | WVRQATGQ GLEWMG | WMHPN---SGNTGYAQK FQG | RVTMTRNTSISTAYMELSSL RSEDTAVYYCAI | SSGWY--------------------WFDP | WGQGT LVTVS S |
| iPS:4 34747 | 21-225_80C12 | VH1\|1-08\|D6\|6-19\|RF1/J H5 | | QVQLVQS-GAEVKKPGASVKV SCKASG-YTFT | N-----YDIN | WVRQATGQ GLEWMG | WMHPN---NGNTGYAQK FQG | RVTMTRNTSISTAYMELNSL RSEDTAVYYCAI | SSGWY--------------------WFDP | WGQGT LVTVS L |
| iPS:4 34761 | 21-225_81E5 | VH1\|1-08\|D6\|6-19\|RF1/J H5 | | QVQLVQS-GAEVKKPGASVKV SCKASG-YTFT | N-----YDIN | WVRQATGQ GLEWMG | WMNPN---SGNTGYAQK FQG | RVTMTRNTSTSTAHMELSSL RSEDTAVYYCAV | SSGWN--------------------WFDP | WGQGT LVTVS S |
| iPS:4 34771 | 21-225_81F9 | VH1\|1-08\|D6\|6-19\|RF1/J H5 | | QVQLVQS-GAEVKKPGASVKV SCKASG-YTFT | N-----YDIN | WVRQATGQ GLEWMG | WMHPN---SGNTGYAQK FQG | RVTMTRNTSISTAYMELSSL RSEDTAVYYCAI | SSGWY--------------------WFDP | WGQGT LVTVS S |
| iPS:4 34793 | 21-225_82A5 | VH1\|1-08\|D6\|6-19\|RF1/J H5 | | QVQLVQS-GAEVKKPGASVKV SCKASG-YTFT | N-----YDIN | WVRQATGQ GLEWMG | WMHPN---NGNTGYAQK FQG | RVTMTRNTSISTAYMELNSL RSEDTAVYYCAI | SSGWY--------------------WFDP | WGQGT LVTVS L |
| iPS:4 34797 | 21-225_82G5 | VH1\|1-08\|D6\|6-19\|RF1/J H5 | | QVQLVQS-GAEVKKPGASVKV SCKASG-YTFT | N-----YDIN | WVRQVPGQ GLEWMG | WMHPN---SGNTGYAQK FQG | RVTMTRNTSISTAYMELSSL RSEDTAVYYCAV | SSGWY--------------------WFDP | WGQGT LVTVS S |

| | | | | | | | | | |
|---|---|---|---|---|---|---|---|---|---|
| iPS:4 34805 | 21-225_82D9 | VH1\|1-08/D6\|6-19\|RF1/JH5 | | QVQLVQS-GAEVKKPGASVKVSCKASG-YTFT | N-----YDIN | WVRQVPGQGLEWMG | WMHPN---SGNTGYAQKFQG | RVTMTRNISISTAYMELSSLRSEDTAVYYCAI | SSGWY--------------------WFDP | WGQGTLVTVS |
| iPS:4 34813 | 21-225_82C12 | VH1\|1-08/D6\|6-19\|RF1/JH5 | | QVQLVQS-GAEVKKPGASVKVSCKASG-YTFT | N-----YDIN | WVRQATGQGLEWMG | WMHPN---NGNTGYAQKFQG | RVTMTRNISISTAYMELNSLRSEDTAVYYCAI | SSGWY--------------------WFDP | WGQGTLVTVSL |
| iPS:4 34825 | 21-225_83C2 | VH1\|1-08/D6\|6-19\|RF1/JH5 | | QVQLVQS-GAEVKKPGASVKVSCKASG-YTFT | N-----YDIN | WVRQVPGQGLEWMG | WMHPN---SGNTGYAQKFQG | RVTMTRNISISTAYMELSSLRSEDTAVYYCAI | SSGWY--------------------WFDP | WGQGTLVTVS |
| iPS:4 34827 | 21-225_83F3 | VH1\|1-08/D6\|6-19\|RF1/JH5 | | QVQLVQS-GAEVKKPGASVKVSCKASG-YTFT | N-----YDIN | WVRQATGQGLEWMG | WMHPN---SGNTGYAQKFQG | RVTMTRNISISTAYMELSSLRSEDTAVYYCAI | SSGWY--------------------WFDP | WGQGTLVTVS |
| iPS:4 34829 | 21-225_83G3 | VH1\|1-08/D6\|6-19\|RF1/JH5 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY--------------------WFDP | WGQGTLVTVS |
| iPS:4 34833 | 21-225_83C5 | VH1\|1-08/D6\|6-19\|RF1/JH5 | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY--------------------WFDP | WGQGTLVTVS |
| iPS:4 34841 | 21-225_83G7 | VH1\|1-08/D6\|6-19\|RF1/JH5 | | QVQLVQS-GAEVKKPGASVKVSCKASG-YTFP | N-----YDIN | WVRQATGQGLEWMG | WMNPN---SGNTGYAQKFQG | RVTMTRNTSISTAYMELSSLRSEDTAVYYCAV | SSGWH--------------------WFDP | WGQGTLVTVA |
| iPS:4 34863 | 21-225_84G7 | VH1\|1-08/D6\|6-19\|RF1/JH5 | | QVQLVQS-GAEVKKPGASVKVSCKASG-YTFP | N-----YDIN | WVRQATGQGLEWMG | WMNPN---SGNTGYAQKFQD | RVTMTRNTSISTAYMELSSLRSEDTAVYYCAV | SSGWH--------------------WFDP | WGQGTLVTVA |
| iPS:4 34877 | 21-225_85H2 | VH1\|1-08/D6\|6-19\|RF1/JH5 | | QVQLVQS-GAEVKKPGASVKVSCKASG-YTFT | N-----YDIN | WVRQATGQGLEWMG | WMHPN---SGNTGYAQKFQG | RVTLTRNTSISTAYMELSSLRSEDTAVYYCAV | SSGWY--------------------WFDP | WGQGTLVTVS |
| iPS:4 34883 | 21-225_85B5 | VH1\|1-08/D6\|6-19\|RF1/JH5 | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY--------------------WFDP | WGQGTLVTVS |
| iPS:4 34911 | 21-225_85D11 | VH1\|1-08/D6\|6-19\|RF1/JH5 | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY--------------------WFDP | WGQGTLVTVS |
| iPS:4 34935 | 21-225_86E9 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRSTSTSTAHMELSSLRSEDTAVYYCAV | SSGWS--------------------WFDP | WGQGTLVTVS |

| ID | Clone | VH | | FR1 | CDR1 | FR2 | CDR2 | FR3 | CDR3 | FR4 |
|---|---|---|---|---|---|---|---|---|---|---|
| iPS:4 34957 | 21-225_87A10 | VH1\|1-08/D6\|6-19\|RF1/JH5 | | QVQLVQS-GAEVKKPGASVKVSCKASG-YTFT | N-----YDIN | WVRQATGQGLEWMG | WMHPN---NGNTGYAQKFQG | RVTMTRNISISTAYMELNSLRSEDTAVYYCAI | SSGWY---------------------WFDP | WGQGTLVTVSL |
| iPS:4 34971 | 21-225_88G2 | VH1\|1-08/D6\|6-19\|RF1/JH5 | | QVQLVQS-GAEVKKPGASVKVSCKASG-YTFT | N-----YDIN | WVRQATGQGLEWMG | WMHPN---SGNTGYAQKFQG | RVTMTRNISISTAYMELSSLRSEDTAVYYCAI | SSGWY---------------------WFDP | WGQGTLVTVS |
| iPS:4 35051 | 21-225_90D9 | VH1\|1-08/D6\|6-19\|RF1/JH5 | | QVQLVQS-GAEVKKPGASVKVSCKASG-YTFP | N-----YDIN | WVRQATGQGLEWMG | WMPN---SGNTGYAQKFQG | RVTMTRNISISTAYMELSSLRSEDTAVYYCAV | SSGWH---------------------WFDP | WGQGTLVTVAS |
| iPS:4 35071 | 21-225_91F1 | VH1\|1-08/D6\|6-19\|RF1/JH5 | | QVQLVQS-GAEVKKPGASVKVSCKASG-YTFT | N-----YDIN | WVRQATGQGLEWMG | WMHPN---SGNTGYAQKFQG | RVTMTRNISISTAYMELSSLRSEDTAVYYCAI | SSGWY---------------------WFDP | WGQGTLVTVS |
| iPS:4 35087 | 21-225_91G8 | VH1\|1-08/D6\|6-19\|RF1/JH5 | | QVQLVQS-GAEVKKPGASVKVSCKASG-YTFT | N-----YDIN | WVRQATGQGLEWMG | WMHPN---SGNTGYAQKFQG | RVTMTRNISISTAYMELSSLRSEDTAVYYCAI | SSGWY---------------------WFDP | WGQGTLVTVS |
| iPS:4 35203 | 21-225_75A7 | VH1\|1-08/D6\|6-19\|RF1/JH5 | | QVQLVQS-GAEVKKPGASVKVSCKASG-YTFP | N-----YDIN | WVRQATGQGLEWMG | WMNPN---SGNTGYAQKFQD | RVTMTRNTSISTAYMELSSLRSEDTAVYYCAV | SSGWH---------------------WFDP | WGQGTLVTVAS |
| iPS:4 35211 | 21-225_94E11 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWK---------------------WFDP | WGQGTLVTVS |
| iPS:4 35227 | 21-225_95G4 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWN---------------------WFDP | WGQGTLVTVS |
| iPS:4 35245 | 21-225_95E12 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAI | SSGWY---------------------WFDP | WGQGTLVTVS |
| iPS:4 35247 | 21-225_96G1 | VH1\|1-08/D6\|6-19\|RF1/JH5 | | QVQLVQS-GAEVKKPGASVKVSCKASG-YTFT | N-----YDIN | WVRQATGQGLEWMG | WMHPN---NGNTGYAQKFQG | RVTMTRNTSISTAYMELNSLRSEDTAVYYCAI | SSGWY---------------------WFDP | WGQGTLVTVSL |
| iPS:4 35249 | 21-225_96E2 | VH1\|1-08/D6\|6-19\|RF1/JH5 | | QVQLVQS-GAEVKKPGASVKVSCKASG-YTFT | N-----YDIN | WVRQATGQGLEWMG | WMHPN---SGNTGYAQKFQG | RVTLTRNTSISTAYMELSSLRSEDTAVYYCAV | SSGWY---------------------WFDP | WGQGTLVTVS |
| iPS:4 35255 | 21-225_96D5 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRSTSTSTAHMELSSLRSEDTAVYYCAV | SSGWS---------------------WFDP | WGQGTLVTVS |

| iPS:4 35279 | 21-225_97H4 | VH1\|1-08/D6\|6-19\|RF1/JH5 | | QVQLVQS-GAEVKKPGASVKVSCKASG-YTFT | N-----YDIN | WVRQATGQ GLEWMG | WMHPN--- SGNTGYAQK FQG | RVTMTRNISISTAYMELSSL RSEDTAVYYCAI | SSGWY--------------WFDP | WGQGT LVTVS S |
|---|---|---|---|---|---|---|---|---|---|---|
| iPS:4 35327 | 21-225_147G6 | VH1\|1-08/D6\|6-19\|RF1/JH5 | | QVQLVQS-GAEVKKPGASVKVSCKASG-YTFT | N-----YDIN | WVRQATGQ GLEWMG | WMHPN--- SGNTGYAQK FQG | RVTMTRNISISTAYMELSSL RSEDTAVYYCAY | SSGWY--------------WFDP | WGQGT LVTVS S |
| iPS:4 35437 | 21-225_152F4 | VH1\|1-08/D6\|6-19\|RF1/JH5 | | QVQLVQS-GAEVKKPGASVKVSCKASG-YTFT | N-----YDIN | WVRQATGQ GLEWMG | WMHPN--- SGNTGYAQK FQG | RVTMTRNISISTAYMELSSL RSDDTAVYYCAY | SSGWY--------------WFDP | WGQGT LVTVS S |
| iPS:4 35701 | 21-225_170F6 | VH1\|1-08/D6\|6-19\|RF1/JH5 | | QVQLVQS-GAEVKKPGASVKVSCKASG-YTFT | N-----YDIN | WVRQATGQ GLEWMG | WMNPN--- SGNTGYAQK FQD | RVTMTRHTSISTAYMELSSL RSEDTAVYYCAI | SSGWY--------------WFDP | WGQGT LVTVS S |
| iPS:4 35737 | 21-225_174G5 | VH1\|1-08/D6\|6-19\|RF1/JH5 | | QVQLVQS-GAEVKKPGASVKVSCKASG-YTFT | N-----YDIN | WVRQATGQ GLEWMG | WMHPN--- SGNTGYAQK FQG | RVTMTRNTSISTAYMELSSL RSEDTAVYYCAV | SSGWY--------------WFDP | WGQGT LVTVS S |
| iPS:4 36544 | 21-225_224H5 | VH1\|1-08/D6\|6-19\|RF1/JH5 | | QVQLVQS-GAEVKKPGASVKVSCKASG-YTFT | N-----YDIN | WVRQATGQ GLEWMG | WMNPN--- SGNTGYAQK FQG | RVTMTRNTSISTAYMELSSL RSEDTAVYYCAS | SSGWN--------------WFDP | WGQGT LVTVS S |
| iPS:4 36570 | 21-225_225F4 | VH1\|1-08/D6\|6-19\|RF1/JH5 | | QVQLVQS-GAEVKKPGASVKVSCKASG-YTFT | N-----YDIN | WVRQATGQ GLEWMG | WMHPN--- SGSTGYAQK FQG | RLTMTRNTSISTVYMELNSL RSEDTAVYYCAS | SSGWY--------------WFDP | WGQGT LVTVS S |
| iPS:4 36644 | 21-225_227G9 | VH1\|1-08/D6\|6-19\|RF1/JH5 | | QVQLVQS-GAEVKKPGASVKVSCRASG-YTFT | N-----YDIN | WVRQATGR GLEWMG | WMYPN--- SGNTGYAQK FQG | RVTMTRNTSISTAYMELSSL RSEDTAVYYCAL | SSGWY--------------WFDP | WGQGT LVTVS S |
| iPS:4 37322 | 21-225_75B1 | VH1\|1-08/D6\|6-19\|RF1/JH5 | | QVQLVQS-GAEVKKPGASVKVSCKASG-YTFT | N-----YDIN | WVRQVPGQ GLEWMG | WMHPN--- SGNTGYAQK FQG | RVTMTRNTSISTAYMELSSL RSEDTAVYYCAI | SSGWY--------------WFDP | WGQGT LVTVS S |
| iPS:4 37361 | 21-225_74C1 | VH1\|1-08/D6\|6-19\|RF1/JH5 | | QVQLVQS-GAEVKKPGASVKVSCKASG-YTFT | N-----YDIN | WVRQATGQ GLEWMG | WMNPD--- SGNTGFAQK FQG | RVTMTRNTSISTAYMELSSL RSEDTAVYYCAV | SSGWY--------------WFDP | WGQGT LVTVS S |
| iPS:4 37363 | 21-225_74C10 | VH1\|1-08/D6\|6-19\|RF1/JH5 | | QVQLVQS-GAEVKKPGASVKVSCKASG-YTFT | N-----YDIN | WVRQATGQ GLEWMG | WMNPN--- SGNIGYAQK FQG | RVTMTRNTSISTAYMELSSL RSEDTAVYYCAI | SSGWY--------------WFDP | WGQGT LVTVS S |
| iPS:4 37379 | 21-225_74H2 | VH1\|1-08/D6\|6-19\|RF1/JH5 | | QVQLVQS-GAEVKKPGASVKVSCKASG-FTFT | N-----YDIN | WVRQATGQ GLEWMG | WMHPN--- SGNTGFAQK FQG | RVTMTRNTSISTAYMELSSL RSEDTAVYYCAV | SSGWY--------------WFDP | WGQGT LVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 46094 | 21-225_77E1 | VH1\|1-08/D6\|6-19\|RF1/JH5 | | QVQLVQS-GAEVKKPGASVKVSCKASG-YTFP | N-----YDIN | WVRQATGQGLEWMG | WMNPN---SGNTGYAQKFQG | RVTMTRNISISTAYMELSSLRSEDTAVYYCAV | SSGWH---------------------WFDP | WGQGTLVTVAS |
| iPS:4 51129 | 21-225_94D2 | VH1\|1-08/D6\|6-19\|RF1/JH5 | | QVQLVQS-GAEVKKPGASVKVSCKASG-FTFT | N-----YDIN | WVRQATGQGLEWMG | WMHPN---SGNTGFAQKFQG | RVTMTRNISISTAYMELSSLRSEDTAVYYCAV | SSGWY---------------------WFDP | WGQGTLVTVS |
| iPS:4 51133 | 21-225_95H4 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SCNTGYAQKFQG | RVTMTRNISTSTAHMELSSLRSEDTAVYYCAV | SSGWN---------------------WFDP | WGQGTLVTVS |
| iPS:3 98510 | 21-225_25A3 | VH1\|1-08/D6\|6-19\|RF1/JH5 | | QVQLVQS-GAEVKKPGASVKVSCKASG-YIFT | N-----YDIN | WVRQATGQGLEWMG | WMHPN---SGNTGYAQKFQG | RVTMTWNTSISTANMELSSLRSEDTAVYYCAS | SSGWY---------------------WFDP | WGQGTLVTVS |
| iPS:3 98516 | 21-225_26A9 | VH1\|1-08/D6\|6-19\|RF1/JH5 | | QVQLVQS-GAEVKKPGASVKVSCKASG-YTFT | N-----YDIN | WVRQATGQGLEWMG | WMHPN---SGNTGCAQKFQG | RVTMTWNMSISTAYMELSSLRSEDTAVYYCAS | SSGWY---------------------WFDP | WGQGTLVTVS |
| | | Germline | | H_FR1 | H_CDR1 | H_FR2 | H_CDR2 | H_FR3 | H_CDR3 | H_FR4 |
| | VH3\|3-30.3/D5\|5-18\|RF3/JH4 | | 32217 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | GYSYG---------------------YYFDY | WGQGTLVTVS |
| iPS:4 51139 | 21-225_71A6 | VH3\|3-30.3/D5\|5-18\|RF3/JH4 | | QVQLVES-GGGVVQPGRSLRLSCAASG-FTFS | N-----YGMH | WVRQAPGKGLEWVA | VISYD---GSNEYYADSVKG | RFTISRDNSKNTLYLQMNSLRAEDTAVYYCAR | DHRYGV--------------------RGGFDY | WGQGTLVTVS |
| | | Germline | | H_FR1 | H_CDR1 | H_FR2 | H_CDR2 | H_FR3 | H_CDR3 | H_FR4 |
| | VH1\|1-18/D6\|6-19\|RF2/JH5 | | 32218 | QVQLVQS-GAEVKKPGASVKVSCKASG-YTFT | S-----YGIS | WVRQAPGQGLEWMG | WISAY---NGNTNYAQKLQG | RVTMTTDTSTSTAYMELRSLRSDDTAVYYCAR | GIAVAG--------------------NWFDP | WGQGTLVTVS |
| iPS:4 51143 | 21-225_66H11 | VH1\|1-18/D6\|6-19\|RF2/JH5 | | QVQLVQS-GAEVKKPGASVKVSCKASG-YTFA | T-----YGIS | WVRQAPGQGLEWMG | WISAY---NGNTNYAQKLQG | RVTMTTDTSTSTAYMELRSLRSDDTAVYYCAR | GEAVA---------------------VFDP | WGQGTLVTVS |
| iPS:4 34361 | 21-225_65D5 | VH1\|1-18/D6\|6-19\|RF2/JH5 | | QVQLVQS-GAEVKKPGASVKVSCKASG-YTFP | S-----YGIS | WVRQAPGQGLEWMG | WISAY---SGNTNYAQKLQG | RVTMTTDTSTSTAYMELRSLRSDDTAVYFCAR | GEAVA---------------------VFDP | WGQGTLVTVS |
| | | Germline | | H_FR1 | H_CDR1 | H_FR2 | H_CDR2 | H_FR3 | H_CDR3 | H_FR4 |
| | VH3\|3-33/D4\|4-23\|RF2/JH6 | | 32219 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYGGNSYY------------YYYGMDV | WGQGTTVTVS |
| iPS:4 53445 | 21-225_148E10 | VH3\|3-33/D4\|4-23\|RF2/JH6 | | QVQLVES-GGGVVQPGRSLRLSCAASG-FTFS | S-----YGMH | WVRQAPGKGLEWVA | VIWFD---GSNKYYVDSVKD | RFTISRDNSKNTLYLQMNSLRAEDTAVYYCAR | DRVEGSGTP-------YYYYGMDV | WGQGTTVTVS |

| iPS:4 36082 | 21-225_195D9 | VH3\|3-33/D4\|4-23\|RF2/J H6 | | QVQLVES-GGGVVQPGRSLRL SCAASG-FTFS | H-----YVMH | WVRQAPGK GLEWVA | VIWYD--- GTNKYYADS VKG | RFTISRDNSKNTLYLQMNSL RAEDTAVYYCAR | DWFGEGN--------- --------YYGMDV | WGQGT TVTVS S |
| iPS:4 36118 | 21-225_196A10 | VH3\|3-33/D4\|4-23\|RF2/J H6 | | QVQLVES-GGGVVQPGRSLRL SCAASG-FTFS | H-----YVMH | WVRQAPGK GLEWVA | VIWYD--- GTNKYYADS VKG | RFTISRDNSKNTLYLQMNSL RAEDTAVYYCAR | DWFGEGN--------- --------YYGMDV | WGQGT TVTVS S |
| iPS:4 36670 | 21-225_147D9 | VH3\|3-33/D4\|4-23\|RF2/J H6 | | QVQLVES-GGGVVQPGRSLRL SCAASG-FTFS | S-----YGMH | WVRQAPGK GLEWVA | DIWFD--- GSNKYYVDS VKD | RFTISRDNSKNTLYLQMNSL RAEDTAVYYCAR | DRVEGSGTP------- ------YYYYGMDV | WGQGT TVTVS S |
| iPS:4 36720 | 21-225_151H6 | VH3\|3-33/D4\|4-23\|RF2/J H6 | | QVQLVES-GGGVVQPGRSLRL SCAASG-FTFS | D-----YGMH | WVRQAPGK GLEWVA | LIWYD--- GSNKYYADS VKG | RFTISRDNSKNTLYLQMNSL RAEDTAVYYCAR | DDRSCSRTSC------ ----PYYYYYGLDV | WGQGT TVIVS S |
| iPS:4 36726 | 21-225_152G5 | VH3\|3-33/D4\|4-23\|RF2/J H6 | | QVQLVES-GGGVVQPGRSLRL SCAASG-FTFS | D-----YGMH | WVRQAPGK GLEWVA | LIWYD--- GSNKYYADS VKG | RFTISRDNSKNTLYLQMNSL RAEDTAVYYCAR | DDRSCSRTSC------ ----PYYYYYGLDV | WGQGT TVIVS S |
| iPS:4 36732 | 21-225_152B12 | VH3\|3-33/D4\|4-23\|RF2/J H6 | | QVQVVES-GGGVVQPGRSLRL SCAASG-FTFS | D-----YGMH | WVRQAPGK GLEWVA | VIWYD--- GSNKYYADS VKG | RFTISRDNSKNTLYLQMNSL RAEDTAVYYCAR | DDRSCSSTSC------ ----PYYYYYGLDV | WGQGT TVTVS S |
| iPS:4 36734 | 21-225_153A8 | VH3\|3-33/D4\|4-23\|RF2/J H6 | | QVQLMES-GGGVVQPGRSLRL SCAASG-FTFS | D-----YGMH | WVRQAPGK GLEWVA | LIWYD--- GSNKYYADS VKG | RFTISRDNSKNTLYLQMNSL RAEDTAVYYCAR | DDRSCSRTSC------ ----PYYYYYGLDV | WGQGT TVIVS S |
| iPS:4 36736 | 21-225_153E8 | VH3\|3-33/D4\|4-23\|RF2/J H6 | | QVQLVES-GGGVVQPGRSLRL SCAASG-FTLS | N-----YGMH | WVRQAPGK GLEWVA | VIWFD--- GSNKYYVDS VKD | RFTISRDNSKNTLYLQMNSL RAEDTAVYYCAR | DRVEGSGTP------- ------YYYYGMDV | WGQGT TVTVS S |
| iPS:4 36756 | 21-225_145A10 | VH3\|3-33/D4\|4-23\|RF2/J H6 | | QVQLEES-GGGVVQPGRSLRL SCAASG-FTFS | G-----YGMH | WVRQAPGK GLEWMT | LIRYD--- GSDKNYADS VKG | RFTISRDNSKNTLYLQMNSL RAEDTAVYYCAR | DRVFCSSTSCL----- ----SYYYYYGMDV | WGQGT TVTVS S |
| iPS:4 36766 | 21-225_158D10 | VH3\|3-33/D4\|4-23\|RF2/J H6 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | DRVSCSSTSC------ ----PYYYYYGMDV | WGQGT TVTVS S |
| iPS:4 36768 | 21-225_159H8 | VH3\|3-33/D4\|4-23\|RF2/J H6 | | QVQLVES-GGGVVQPGRSLRL SCAASG-FTFS | T-----YGMH | WVRQAPGK GLEWVA | VIWYD--- GSNKYYADS VKG | RFTISRDNSKNTLYLQMNSL RAEDTAVYYCAR | DRVSCSSTSC------ ----PYYYYYGMDV | WGQGT TVTVS S |
| iPS:4 36770 | 21-225_160B12 | VH3\|3-33/D4\|4-23\|RF2/J H6 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | DRVSCSSTSC------ ----PYYYYYGMDV | WGQGT TVTVS S |

| iPS:4 36782 | 21-225_166G11 | VH3\|3-33/D4\|4-23\|RF2/JH6 | | QVQLVES-GGGVVQPGRSLRLSCAASG-FTFN | G-----YGMH | WVRQAPGKGLEWVA | VIWYD---GSNKYYADSVKG | RFTISRDISKNTLFLQMNSLTAEDTAVYYCAR | DDRYCSSPTCH---------PYYYYYGLDV | WGQGTTVTVSS |
| iPS:4 36794 | 21-225_170F1 | VH3\|3-33/D4\|4-23\|RF2/JH6 | | QVQLVES-GGGVVQPGRSLRLSCAASG-FTFS | G-----YGMN | WVRQAPGKGLEWVA | IIWYD---GNNKYYADSVKG | RFTISRDNSKNTLYLQMNSLRAEDTAVYYCAR | DRVYCSSTSCH---------PYYYYYAMDV | WGQGTTVTVSS |
| iPS:4 36836 | 21-225_52H1 | VH3\|3-33/D4\|4-23\|RF2/JH6 | | QVQLVES-GGGVVQPGRSLRLSCAASG-FTFS | G-----YGMH | WVRQAPGKGLEWVA | VIWYD---GSNKYYADSVKG | RFTISRDNSKNTLYLQMNSLRAEDTAVYYCAR | DRVYCSSSSCS---------YYYYYYGMDV | WGQGTTVTVSS |
| iPS:4 36922 | 21-225_78E9 | VH3\|3-33/D4\|4-23\|RF2/JH6 | | QVQLVES-GGGVVQPGRSLRLSCAASG-FTFS | S-----YGMH | WVRQAPGKGLEWVA | VIWYD---GNNKSYADSVKG | RFTISRDISKNTLYLQMNSLRAEDTAVYYCAR | DRDYCSSTSC----------PYYYYYGMDV | WGQGTTVTVSS |
| iPS:4 36924 | 21-225_74B3 | VH3\|3-33/D4\|4-23\|RF2/JH6 | | QVQLVES-GGGVVQPGRSLRLSCAASG-FTFS | R-----YGMH | WVRQAPGKGLEWVA | VFWYD---GSNKDYADSVKG | RFTISRDNSKNTLYLQMNSLRAEDTAVYYCAR | DRDYCSSTSC----------PYYYYYGMDV | WGQGTTVTVSS |
| iPS:4 36928 | 21-225_79E7 | VH3\|3-33/D4\|4-23\|RF2/JH6 | | QVQLVES-GGGVVQPGRSLRLSCAASG-FTFS | S-----YGMH | WVRQAPGKGLEWVA | VIWYD---GNNKSYADSVKG | RFTISRDISKNTLYLQMNSLRAEDTAVYYCAR | DRDYCSSTSC----------PYYYYYGMDV | WGQGTTVTVSS |
| iPS:4 36932 | 21-225_92A4 | VH3\|3-33/D4\|4-23\|RF2/JH6 | | QVQLVES-GGGVVQPGRSLRLSCAASG-FTFS | S-----YGMH | WVRQAPGKGLEWVA | VIWYD---GNNKSYADSVKG | RFTIYRDISKNTLYLQMNSLRAEDTAVYYCAR | DRDYCSSTSC----------PYYYYYGMDV | WGQGTTVTVSS |
| iPS:4 36936 | 21-225_97E6 | VH3\|3-33/D4\|4-23\|RF2/JH6 | | QVQLVES-GGGVVQPGRSLRLSCAASG-FTFS | S-----YGMH | WVRQAPGKGLEWVA | VIWYD---GNNKSYADSVKG | RFTISRDISKNTLYLQMNSLRAEDTAVYYCAR | DRDYCSSTSC----------PYYYYYGMDV | WGQGTTVTVSS |
| iPS:4 37190 | 21-225_225A9 | VH3\|3-33/D4\|4-23\|RF2/JH6 | | QVQLVES-GGGVVQPGGSLRLSCAASG-FTFS | T-----YGMH | WVRLAPGKGLEWVA | VIWYD---GSNKYYADSVKG | RFTISRDISQNTLYLQMNSLRAEDTAVYYCAR | DNHYCSSTSCS---------PYYYYFGMDV | WGQGTTVTVSS |
| iPS:4 37254 | 21-225_149F2 | VH3\|3-33/D4\|4-23\|RF2/JH6 | | QVQLGEA-GGGVVQPGRSLRLSCAASG-FTFS | R-----YGMH | WVRQAPGKGLEWVA | FIWYD---GSENYYADSVKG | RFTISRVNSRNTLYLQMNSLRAEDTAVYYCAR | DRVEGSGTP-----------YYYYGMDV | WGQGTTVTVSS |
| iPS:4 37256 | 21-225_150F11 | VH3\|3-33/D4\|4-23\|RF2/JH6 | | QVQLGEA-GGGVVQPGRSLRLSCAASG-FTFS | R-----YGMH | WVRQAPGKGLEWVA | FIWYD---GSENYYADSVKG | RFTISRVNSRNTLYLQMNSLRAEDTAVYYCAR | DRVEGSGTP-----------YYYYGMDV | WGQGTTVTVSS |
| iPS:4 51110 | 21-225_74C9 | VH3\|3-33/D4\|4-23\|RF2/JH6 | | QVQLVES-GGGVVQPGRSLRLSCAASG-FTFS | S-----YGMH | WVRQAPGKGLEWVA | VIWYD---GNNKSYADSVKG | RFTISRDISKNTLYLQMNSLRAEDTAVYYCAR | DRDYCSSTSC----------PYYYYYGMDV | WGQGTTVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 92589 | 21-225_27H2 | VH3\|3-33/D4\|4-23\|RF2/JH6 | | QVQLVES-GGGVVQPGRSLRL SCAASG-FTFS | G-----YGMH | WVRQAPGK GLEWVA | VIWYD---GSNKYYADS VKG | RFTISRDNSKNTLFLQMNSL RAEDTAVYYCAR | DRVYCSSTSCS--------PYYYYYGMDV | WGQGT TVTVS S |
| iPS:3 93166 | 21-225_27G6 | VH3\|3-33/D4\|4-23\|RF2/JH6 | | QVQLVES-GGGVVQPGRSLRL SCAASG-FTFS | G-----YGMH | WVRQAPGK GLEWVA | IIWYD---GSKKYNADS VKG | RFTISRDNSKNTLYLQMNSL RAEDTAVYYCAR | DRVYCSSTSCS--------PYYYYYGMDV | WGQGT TVTVS S |
| iPS:3 93198 | 21-225_28A11 | VH3\|3-33/D4\|4-23\|RF2/JH6 | | QVQLVES-GGGVVQPGRSLRL SCAASG-FTFS | G-----YGLH | WVRQAPGK GLEWVA | LIWYD---GNNTYYADS VKG | RFTISRDNSKNTLYLQMNSL RAEDTAVYYCAR | DRVYCSSTSCS--------PYYYYYGMDV | WGQGT TVTVS S |
| iPS:3 93204 | 21-225_8C12 | VH3\|3-33/D4\|4-23\|RF2/JH6 | | QVQLVES-GGGVVQPGRSLRL SCAASG-FTFG | S-----YGMH | WVRQAPGK GLEWVA | LIWYD---GSNKYYADS VKG | RFTISRDNSKNTLYLQMNSL RAEDTAVYYCAR | DRVSCSSSSCY--------PYYYYYGMDV | WGQGT TVTVS S |
| | | Germline | | H_FR1 | H_CDR1 | H_FR2 | H_CDR2 | H_FR3 | H_CDR3 | H_FR4 |
| | VH1\|1-02/D4\|4-11\|RF2/JH4 | | 32220 | QVQLVQS-GAEVKKPGASVKV SCKASG-YTFT | G-----YYMH | WVRQAPGQ GLEWMG | WINPN---SGGTNYAQK FQG | RVTMTRDTSISTAYMELSRL RSDDTAVYYCAR | DYSNY-----------------YFDY | WGQGT LVTVS S |
| iPS:4 53447 | 21-225_65F10 | VH1\|1-02/D4\|4-11\|RF2/JH4 | | QVQLVQS-GAEVKKPGASVKV SCKASG-YTFT | G-----YHMH | WVRQAPGQ GLEWMG | WINPN---NGGTSYAQK FQD | RVNMTRDTSISTAYMELSRL RSDDTAVYYCAR | DSRS----------------SWDY | WGQGT LVTVS S |
| iPS:4 34145 | 21-225_55B1 | VH1\|1-02/D4\|4-11\|RF2/JH4 | | QVQLVQS-GAEVKKPGASVKV SCKASG-YTFT | G-----YYFH | WVRQAPGQ GLEWMG | WIHPN---NNATNYAQK FQG | RVTMTRDTSISTAYMELSRL RSDDTAVYYCAK | DGRS----------------SFDY | WGQGT LVTVS S |
| iPS:4 34277 | 21-225_57A7 | VH1\|1-02/D4\|4-11\|RF2/JH4 | | QVQLVQS-GAEVKKPGASVKV SCKASG-YTFT | G-----YHIH | WVRQAPGQ DLEWMG | WINPN---NNGTNYAQK FQG | RVTMTRDTSISTAYMELSRL RSDDTAVYYCAR | DGRS----------------GFDY | WGQGT LVTVS S |
| iPS:4 34389 | 21-225_66F11 | VH1\|1-02/D4\|4-11\|RF2/JH4 | | QVQLVQS-GAEVKKPGASVKV SCKASG-YTFT | G-----YHMH | WVRQAPGQ GLEWMG | WINPN---NGGTHYAQK FQD | WVTMTRDTSISTAYMELSRL RSDDTAVYYCAR | DSRS----------------SWDY | WGQGT LVSVS S |
| iPS:4 34423 | 21-225_70D1 | VH1\|1-02/D4\|4-11\|RF2/JH4 | | QVQLVQS-GAEVKKPGASVKV SCKASG-YTFT | G-----YHMH | WVRQAPGQ GLEWMG | WINPN---SNATNYAQK FQG | RVTMTRDTSISTAYMELSRL RSDDTAVYYCAR | DSIS----------------SWDY | WGQGT LVTVS S |
| iPS:4 37234 | 21-225_64E2 | VH1\|1-02/D4\|4-11\|RF2/JH4 | | QVQLVQS-GAEVKKPGASVKV SCKASG-YTFT | G-----YYMH | WVRQAPGQ GLEWMG | WINPN---NNGTNYAQK FQG | RVTMTRDTSISTAYMELSRL RSDDTAVYYCAR | DGSS----------------GFDY | WGQGT LVTVS S |
| | | Germline | | H_FR1 | H_CDR1 | H_FR2 | H_CDR2 | H_FR3 | H_CDR3 | H_FR4 |
| | VH4\|4-59/D7\|7-27\|RF1/JH4 | | 32221 | QVQLQES-GPGLVKPSETLSL TCTVSG-GSIS | S-----YYWS | WIRQPPGK GLEWIG | YIYY----SGSTNYNPS LKS | RVTISVDTSKNQFSLKLSSV TAADTAVYYCAR | LTGY----------------FDY | WGQGT LVTVS S |

EP 4 435 105 A2

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 53449 | 21-225_208A2 | VH4\|4-59/D7\|7-27\|RF1/JH4 | | QVQLQES-GPGLVKPSETLSLNCTVSG-GSIR | S-----YYWS | WIRQPAGKGLEWIG | RIYT----SGSTDYNPSLKS | RITMSVDISKNQFSLKLSSVTAADTAVYYCAR | GFGD----------------------WDY | WGQGTLVTVSS |
| iPS:4 35451 | 21-225_152D10 | VH4\|4-59/D7\|7-27\|RF1/JH4 | | QVQLQES-GPGLVKPSETLSLTCTVSG-GSIS | N-----YYWS | WIRQPAGKGLEWIG | RIDT----SGITNYNPSLKS | RVTMSVDISKNQFSLKLTSVTAADTAVYYCAR | EGGLGA-------------------TFFDY | WGQGTLVTVSS |
| iPS:4 35467 | 21-225_153B9 | VH4\|4-59/D7\|7-27\|RF1/JH4 | | QVQLQES-GPGLVKPSETLSLTCTVSG-GSIS | S-----YYWS | WIRQPAGKGLEWIG | RIDT----SGITNYNPSLKS | RVTMSVDISKNQFSLKLTSVTAADTAVYYCAR | EGGVGA-------------------TYFDY | WGQGTLVTVSS |
| iPS:4 35545 | 21-225_158F4 | VH4\|4-59/D7\|7-27\|RF1/JH4 | | QVQLQES-GPGLVKPSETLSLTCTVSG-GSIS | S-----HFWS | WIRQPAGKGLEWIG | RIYT----SGTTNYTPSLKS | RVTMSVDISKNQFSLKLSSVTAADTAVYYCAR | LSSG--------------------WFDY | WGQGTLVTVSS |
| iPS:4 35665 | 21-225_169F2 | VH4\|4-59/D7\|7-27\|RF1/JH4 | | QVQLQES-GPGLVKPSETLSLTCTVSG-GSIS | S-----YYWS | WIRQPAGKGLEWIG | RIDT----SGITNYNPSLKS | RVTMSIDTSKSQISLKLSSVTAADTAVYYCAR | EGGVGA-------------------TYFDY | WGQGTLVTVSS |
| iPS:4 35671 | 21-225_169H5 | VH4\|4-59/D7\|7-27\|RF1/JH4 | | QVQLQES-GPGLVKPSETLSLTCTVSG-GSTS | S-----YYWS | WIRQPAGKGLEWIG | RIDT----SGITNYNPSLKS | RVTMSVDTSKSQISLKLSSVTAADTAVYYCAR | EGGVGA-------------------TYFDY | WGQGTLVTVSS |
| iPS:4 36354 | 21-225_210G10 | VH4\|4-59/D7\|7-27\|RF1/JH4 | | QVQLQES-GPGLVKPSETLSLNCTVSG-GSIR | S-----YYWS | WIRQPAGKGLEWIG | RIYT----SGSTDYNPSLKS | RITMSVDISKNQFSLKLSSVTAADTAVYYCAR | GFGD----------------------WDY | WGQGTLVTVSS |
| | Germline | | | H_FR1 | H_CDR1 | H_FR2 | H_CDR2 | H_FR3 | H_CDR3 | H_FR4 |
| | VH4\|4-34/D4\|4-17\|RF2/JH6 | | 32222 | QVQLQQW-GAGLLKPSETLSLTCAVYG-GSFS | G------YYWS | WIRQPPGKGLEWIG | EINH----SGSTNYNPSLKS | RVTISVDTSKNQFSLKLSSVTAADTAVYYCAR | DYGDYYY-----------------YYYGMDV | WGQGTTVTVSS |
| iPS:4 68810 | 21-225_74D5 | VH4\|4-34/D4\|4-17\|RF2/JH6 | | QVQLQQW-GAGLLKPSETLSLTCAVNG-GPFS | G------CYWS | WIRQPPGKGLEWIG | EINY----SGRTNFNPSLKS | RVTISVDTSKNQFSLKLSSVTAADTAVYYCAR | DYGG----------------------MDV | WGQGTTVTVSS |
| iPS:4 68832 | 21-225_76H10 | VH4\|4-34/D4\|4-17\|RF2/JH6 | | QVQLQQW-GAGLLKPSETLSLTCAVNG-GPFS | G------CYWS | WIRQPPGKGLEWIG | EINY----SGRTNFNPSLKS | RVTISVDTSKNQFSLKLSSVTAADTAVYYCAR | DYGG----------------------MDV | WGQGTTVTVSS |
| iPS:4 68834 | 21-225_94G10 | VH4\|4-34/D4\|4-17\|RF2/JH6 | | QVQLQQW-GAGLLKPSETLSLTCAVNG-GPFS | G------CYWS | GIRQPPGKGREWIG | EINY----SGRTNFNPSLKS | RVTISVDTSKNQFSLKLSSVTAADTAVYYCAR | DYGG----------------------MDV | WGQGTTVTVSS |
| iPS:4 68838 | 21-225_80E12 | VH4\|4-34/D4\|4-17\|RF2/JH6 | | QVQLQQW-GAGLLKPSETLSLTCAVNG-GPFS | G------CYWS | WIRQPPGKGLEWIG | EINY----SGRTNFNPSLKS | RVTISVDTSKNQFSLKLSSVTAADTAVYYCAR | DYGG----------------------MDV | WGQGTTVTVSS |

2882

| | | | | | | | | | | |
|---|---|---|---|---|---|---|---|---|---|---|
| iPS:4 68820 | 21-225_76E10 | VH4\|4-34/D4\|4-17\|RF2/JH6 | | QVQLQQW-GAGLLKPSETLSL TCAVNG-GPFS | G-----SYWS | WIRQPPGK GLEWIG | EINY---- SGRTNFNPS LKS | RVTISVDTSKNQFSLKLSSV TAADTAVYYCAR | DYGG----------- ----------MDV | WGQGT TVTVS S |
| iPS:4 34473 | 21-225_76D1 | VH4\|4-34/D4\|4-17\|RF2/JH6 | | QVQLQQW-GAGLLKPSETLSL TCAVYS-GSFS | G-----CYWS | WIRQPPGK GLEWIG | EINH---- SGRTNYNPS LKS | RVTISVDTSKNQFSLKLSSV TAADTAVYYCAR | DYGG----------- ----------MDV | WGQGT TVTVS S |
| iPS:4 34495 | 21-225_74B2 | VH4\|4-34/D4\|4-17\|RF2/JH6 | | QVQLQQW-GAGLLKPSEPLSL TCAVYG-GSFS | G-----PYWS | WIRQPPGK GLEWIG | EINH---- SGSTNYNPS LTS | RVTISVDTSKNQFSLKLTSV TAADSAVYYCAR | DYGG----------- ----------LDV | WGQGT TVTVS S |
| iPS:4 34497 | 21-225_76A4 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG----------- ----------MDV | WGQGT TVTVS S |
| iPS:4 34501 | 21-225_76G4 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG----------- ----------MDV | WGQGT TVTVS S |
| iPS:4 34507 | 21-225_74C5 | VH4\|4-34/D4\|4-17\|RF2/JH6 | | QVQLQQW-GAGLLKPSETLSL TCAVYG-GSFS | G-----CYWS | WIRQPPGK GLEWIG | EINH---- SGCTNFNPS LKS | RVTISVDTSKNQFSLKLSSV TAADTAVYYCAR | DYGG----------- ----------MDV | WGQGT TVTVS S |
| iPS:4 34523 | 21-225_75C3 | VH4\|4-34/D4\|4-17\|RF2/JH6 | | QVQLQQG-GAGPLKPSETLSL TCAVYG-GSFS | G-----CYWS | WIRQPPGK GLEWIG | EINY---- SGRTNYNPS LKS | RVTISVDTSKNQFSLKLSSV TAADTAVYYCAR | DYGG----------- ----------MDV | WGQGT TVTVS S |
| iPS:4 34533 | 21-225_85F7 | VH4\|4-34/D4\|4-17\|RF2/JH6 | | QVQLQQW-GAGLLKPSEPLSL TCAVYG-GSFS | G-----PYWS | WIRQPPGK GLEWIG | EINH---- SGSTNYNPS LKS | RVTISVDTSKNQFSLKLTSV TAADTAVYYCAR | DYGG----------- ----------LDV | WGQGT TVTVS S |
| iPS:4 34547 | 21-225_74H5 | VH4\|4-34/D4\|4-17\|RF2/JH6 | | QVQLQQW-GAGLLKPSETLSL TCAVNG-GPFS | G-----CYWS | WIRQPPGK GLEWIG | EINH---- SGRTNFNPS LKS | RVTISVDTSKNQFSLKLSSV TAADTAVYYCAR | DYGG----------- ----------MDV | WGQGT TVTVS S |
| iPS:4 34559 | 21-225_74D11 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG----------- ----------MDV | WGQGT TVTVS S |
| iPS:4 34561 | 21-225_77G1 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG----------- ----------MDV | WGQGT TVTVS S |
| iPS:4 34565 | 21-225_75B10 | VH4\|4-34/D4\|4-17\|RF2/JH6 | | QVQLQQW-GAGLLKPSETLSL KCDVYG-GSFS | G-----YYWS | WIRQPPGK GLEWIG | EINH---- SGSTNYNPS LKS | RVTISVDTSKNQFSLKLSSV TAADTAVYYCAR | DYGG----------- ----------LDV | WGQGT TVTVS S |

| | | | | | | | | | | |
|---|---|---|---|---|---|---|---|---|---|---|
| iPS:4 34579 | 21-225_77F7 | VH4|4-34/D4|4-17|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:4 34581 | 21-225_74B12 | VH4|4-34/D4|4-17|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:4 34585 | 21-225_75A12 | VH4|4-34/D4|4-17|RF2/JH6 | | QVQLQQG-GAGPLKPSETLSLTCAVYG-GSFS | G-----CYWS | WIRQPPGKGLEWIG | EINY----SGRTNYNPSLKS | RVTISVDTSKNQFSLKLSSVTAADTAVYYCAR | DYGG-----------------------MDV | WGQGTTVTVSS |
| iPS:4 34595 | 21-225_77A10 | VH4|4-34/D4|4-17|RF2/JH6 | | QVQLQQG-GAGPLKPSETLSLTCAVYG-GSFS | G-----CYWS | WIRQPPGKGLEWIG | EINY----SGRTNYNPSLKS | RVTISVDTSKNQFSLKLSSVTAADTAVYYCAR | DYGG-----------------------MDV | WGQGTTVTVSS |
| iPS:4 34611 | 21-225_77C12 | VH4|4-34/D4|4-17|RF2/JH6 | | QVQLQQW-GAGLLKPSETLSLTCAVYG-GAFS | G-----SYWS | WIRQSPGKGLEWIG | EINY----RGSTNYNPSLKS | RVTISVDTSKNQFSLNLSSVTAADTAVYYCAR | DYGG-----------------------MDV | WGQGTTVTVSS |
| iPS:4 34657 | 21-225_79G1 | VH4|4-34/D4|4-17|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:4 34663 | 21-225_79F3 | VH4|4-34/D4|4-17|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:4 34687 | 21-225_75A5 | VH4|4-34/D4|4-17|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:4 34691 | 21-225_75G7 | VH4|4-34/D4|4-17|RF2/JH6 | | QVQLQQW-GAGLLKPSETLSLTCTVYG-GAFS | G-----SYWS | WIRQSPGKGLEWIG | EINY----RGSTNYNPSLKS | RVTISVDTSKNQFSLNLSSVTAADTAVYYCAR | DYGG-----------------------MDV | WGQGTTVTVSS |
| iPS:4 34693 | 21-225_79F11 | VH4|4-34/D4|4-17|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:4 34699 | 21-225_79G12 | VH4|4-34/D4|4-17|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:4 34701 | 21-225_80A1 | VH4|4-34/D4|4-17|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |

| iPS:4 34703 | 21-225_80C1 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
|---|---|---|---|---|---|---|---|---|---|---|
| iPS:4 34709 | 21-225_80E3 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34715 | 21-225_80D5 | VH4\|4-34/D4\|4-17\|RF2/J H6 | | QVQLQQW-GAGLLKPSEPLSL TCAVYG-GSFS | G-----PYWS | WIRQPPGK GLEWIG | EINH---- SGSTNYNPS LKS | RVTISVDTSKNQFSLKLTSV TAADMAVYYCAR | DYGG------------ ----------LDV | WGQGT TVTVS S |
| iPS:4 34725 | 21-225_80H7 | VH4\|4-34/D4\|4-17\|RF2/J H6 | | QVQLQQW-GAGLLKPSETLSL TCAVYV-GSFS | G-----SYWS | WIRQPPGK GLEWIG | EINQ---- SGRTNYNPS LKS | RVTISVDTSKNQFSLKLSSV TAADTAVYYCAR | DYGG------------ ----------IDV | WGQGT TVTVS S |
| iPS:4 34743 | 21-225_74A4 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34751 | 21-225_80H12 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34759 | 21-225_81C5 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34773 | 21-225_75D9 | VH4\|4-34/D4\|4-17\|RF2/J H6 | | QVQLQQW-GAGLLKPSETLSL TCAVYG-GSFS | G-----PYWS | WIRQPPGK GLEWIG | EINY---- RGSTNYNPS LKS | RVTISVDTSKNQFSLNLSSV TAADTAVYYCAR | DYGG------------ ----------MDV | WGQGT TVTVS S |
| iPS:4 34777 | 21-225_81C11 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34809 | 21-225_74F5 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34821 | 21-225_83G1 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34839 | 21-225_83B7 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |

| | | | | | | | | | | |
|---|---|---|---|---|---|---|---|---|---|---|
| iPS:4 34869 | 21-225_84E12 | VH4\|4-34/D4\|4-17\|RF2/J H6 | | QVQLQQW-GAGLLKPSETLSL TCAVYG-GSFS | G-----SYWS | WIRQPPGK GLEWIG | EINQ---- SGRTNYNPS LKS | RVTISVDTSKNQFSLKLSSV TAADTAVYYCAR | DYGG------------ -----------IDV | WGQGT TVTVS S |
| iPS:4 34879 | 21-225_85A3 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ -----------MDV | WGQGT TVTVS S |
| iPS:4 34881 | 21-225_85B4 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ -----------MDV | WGQGT TVTVS S |
| iPS:4 34887 | 21-225_85D6 | VH4\|4-34/D4\|4-17\|RF2/J H6 | | QVQLQQG-GAGPLKPSETLSL TCAVYG-GSFS | G-----CYWS | WIRQPPGK GLEWIG | EINY---- SGRTNYNPS LKS | RVTISVDTSKNQFSLKLSSV TAADTAVYYCAR | DYGG------------ -----------MDV | WGQGT TVTVS S |
| iPS:4 34895 | 21-225_74H7 | VH4\|4-34/D4\|4-17\|RF2/J H6 | | QVQLQQW-GAGLLKPSEPLSL TCAVYG-GSFS | G-----PYWS | WIRQPPGK GLEWIG | EINH---- SGSTNYNPS LKS | RVTISVDTSKNQFSLKLTSV TAADTAVYYCAR | DYGG------------ -----------LDV | WGQGT TVTVS S |
| iPS:4 34899 | 21-225_85B9 | VH4\|4-34/D4\|4-17\|RF2/J H6 | | QVQLQQW-GAGLLKPSETLSL TCAVNG-GPFS | G-----CYWS | WIRQPPGK GLEWIG | EINH---- SGRTNFNPS LKS | RVTISVDTSKNQFSLKLSSV TAADTAVYYCAR | DYGG------------ -----------MDV | WGQGT TVTVS S |
| iPS:4 34907 | 21-225_85G10 | VH4\|4-34/D4\|4-17\|RF2/J H6 | | QVQLQQR-GAGLLKPSETLSL TCAVYG-GSFS | G-----CYWS | WIRQPPGK GLEWIG | EINH---- SGITNYNPS LKS | RVTISVDTSKNQFSLKLTSV TAADTAVYYCAR | DYGG------------ -----------LDV | WGQGT TVTVS S |
| iPS:4 34913 | 21-225_86C1 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ -----------MDV | WGQGT TVTVS S |
| iPS:4 34921 | 21-225_86E4 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ -----------MDV | WGQGT TVTVS S |
| iPS:4 34939 | 21-225_86C11 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ -----------MDV | WGQGT TVTVS S |
| iPS:4 34943 | 21-225_87H1 | VH4\|4-34/D4\|4-17\|RF2/J H6 | | QVQLQPW-GAGLLKPSETLSL TCAVYG-GSFS | G-----YYWS | WIRQPPGK GLEWIG | EINH---- SGRTNYNPS LKS | RVTISVDTSKDQFSLKLSSV TAADTAVYYCAR | DYGG------------ -----------LDV | WGQGT TVTVS S |
| iPS:4 34945 | 21-225_87E5 | VH4\|4-34/D4\|4-17\|RF2/J H6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ -----------MDV | WGQGT TVTVS S |

| | | | | | | | | | | |
|---|---|---|---|---|---|---|---|---|---|---|
| iPS:4 34955 | 21-225_87C9 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34961 | 21-225_87A12 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34969 | 21-225_88H1 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34981 | 21-225_88E7 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34983 | 21-225_88F7 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34995 | 21-225_88G9 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 34999 | 21-225_75A8 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35013 | 21-225_89D5 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35015 | 21-225_89H5 | VH4\|4-34/D4\|4-17\|RF2/JH6 | | QVQLQQW-GAGLLKPSETLSL TCAVYG-GSFS | G-----CYWS | WIRQPPGK GLEWIG | EINY---- SGSTNFNPS LKS | RVTISADTSKNQFSLKLSSV TAADTAVYYCAR | DYGG------------ ----------MDV | WGQGT TVTVS S |
| iPS:4 35025 | 21-225_89E10 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35029 | 21-225_89A11 | VH4\|4-34/D4\|4-17\|RF2/JH6 | | QVQLQPW-GAGLLKPSETLSL TCAVYG-GSFS | G-----YYWS | WIRQPPGK GLEWIG | EINH---- SGRTSYNPS LKS | RVTISVDTSKNQFSLKLSSV TAADTAVYYCAR | DYGG------------ ----------LDV | WGQGT TVTVS S |
| iPS:4 35039 | 21-225_90G4 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |

| | | | | | | | | | | |
|---|---|---|---|---|---|---|---|---|---|---|
| iPS:4 35041 | 21-225_90A5 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35043 | 21-225_90G5 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35055 | 21-225_90F10 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35073 | 21-225_91B2 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35075 | 21-225_91B3 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35077 | 21-225_91F3 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35079 | 21-225_91B4 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35089 | 21-225_91E9 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35097 | 21-225_92B1 | VH4\|4-34/D4\|4-17\|RF2/JH6 | | QVQLQQW-GAGLLKPSETLSL TCAVYG-GSFS | G-----SYWS | WIRQPPGK GLEWIG | EINY---- RGSTNYNPS LKS | RVAISVDTSKNQFSLNLTSV TAADTAVYYCAR | DYGG------------ ----------LDV | WGQGT TVTVS S |
| iPS:4 35111 | 21-225_92D6 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35115 | 21-225_77C5 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |
| iPS:4 35171 | 21-225_93C2 | VH4\|4-34/D4\|4-17\|RF2/JH6 | | QVQLQQW-GAGLLKPSETLSL TCAVHG-GSFS | G-----CYWS | WIRQPPGK GLEWIG | EINH---- SGSTNYNPS LKS | RVTISVDTSKNQFSLKLSSV TAADTAVYYCAR | DYGG------------ ----------MDV | WGQGT TVTVS S |

| | | | | | | | | | |
|---|---|---|---|---|---|---|---|---|---|
| iPS:4 35177 | 21-225_93E4 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 35195 | 21-225_94D3 | VH4|4-34/D4|4-17|RF2/J H6 | | QVQLQQG-GAGPLKPSETLSL TCAVYG-GSFS | G-----CYWS | WIRQPPGK GLEWIG | EINY----SGRTNYNPS LKS | RVTISVDTSKNQFSLKLSSV TAADTAVYYCAR | DYGG------------MDV | WGQGT TVTVS S |
| iPS:4 35217 | 21-225_94F12 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 35219 | 21-225_95D2 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 35235 | 21-225_95F9 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 35237 | 21-225_95G9 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 35239 | 21-225_95H10 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 35273 | 21-225_97A2 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 35281 | 21-225_97E5 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 37324 | 21-225_75C2 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 37328 | 21-225_75D3 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |
| iPS:4 37332 | 21-225_75F3 | VH4|4-34/D4|4-17|RF2/J H6 | | QVQLQQW-GAGLLKPSETLSL TCAVHG-GSFS | G-----CYWS | WIRQPPGK GLEWIG | EINH----SGSTNYNPS LKS | RVTISVDTSKNQFSLKLSSV TAADTAVYYCAR | DYGG------------MDV | WGQGT TVTVS S |

| iPS ID | Name | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 37344 | 21-225_75G12 | VH4\|4-34/D4\|4-17\|RF2/JH6 | | QVQLQQW-GAGLLKPSETLSLTCAVHG-GSFS | G-----CYWS | WIRQPPGK GLEWIG | EINH----SGSTNYNPS LKS | RVTISVDISKNQFSLKLSSV TAADTAVYYCAR | DYGG----------------------MDV | WGQGT TVTVS S |
| iPS:4 37350 | 21-225_74A3 | VH4\|4-34/D4\|4-17\|RF2/JH6 | | QVQLQQW-GAGLLKPSETLSLTCAVHG-GSFS | G-----CYWS | WIRQPPGK GLEWIG | EINH----SGSTNYNPS LKS | RVTISVDISKNQFSLKLSSV TAADTAVYYCAR | DYGG----------------------MDV | WGQGT TVTVS S |
| iPS:4 37369 | 21-225_74D6 | VH4\|4-34/D4\|4-17\|RF2/JH6 | | QVQLQQW-GAGLLKPSETLSLTCAVHG-GSFS | G-----CYWS | WIRQPPGK GLEWIG | EINH----SGSTNYNPS LKS | RVTISVDISKNQFSLKLSSV TAADTAVYYCAR | DYGG----------------------MDV | WGQGT TVTVS S |
| | | Germline | | H_FR1 | H_CDR1 | H_FR2 | H_CDR2 | H_FR3 | H_CDR3 | H_FR4 |
| | VH3\|3-33/D4\|4-17\|RF2/JH6 | | 32223 | QVQLVES-GGGVVQPGRSLRLSCAASG-FTFS | S      YCMH | WVRQAPGK GLEWVA | VIWYD-GSNKYYADS VKG | RFTISRDNSKNTLYLQMNSL RAEDTAVYYCAR | DYCDYYY-------YYYGMDV | WCQGT TVTVS S |
| iPS:4 68814 | 21-225_223D11 | VH3\|3-33/D4\|4-17\|RF2/JH6 | | QVQLVES-GGGVVQPGRSLRLSCAASG-FTFS | N-----YGMD | WVRQAPGK GLEWVA | VIWYD---GSNDYYADS VKG | RFTISRDNSKNTLFLQMNSL RAEDTAVYYCAR | DRGIGY------------------NDMDV | WGQGT TVTVS S |
| iPS:4 34621 | 21-225_74D1 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKYHADS VKG | RFTISRDNSKNTLYLQMNSL RAEDTAMYYCAR | DEGFGEFD-------------YYNYGMDV | WGQGT TVTVS S |
| iPS:4 34947 | 21-225_87B7 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DFGVGY---------------YGMDV | WGQGT TVTVS S |
| iPS:4 35819 | 21-225_190C11 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DQGVGY---------------DGLDV | WGQGT TVTVS S |
| iPS:4 35825 | 21-225_190G11 | VH3\|3-33/D4\|4-17\|RF2/JH6 | | QVQLVES-GGGVVQPGRSLRLSCAASG-FTFS | I-----YGMH | WVRQAPGK GLEWVA | VIWYD---GSNKNYADS VKG | RFTISRDNSKNTLYLQMNSL RAEDTAVYYCAR | DQGVGY---------------DGLDV | WGQGT TVTVS S |
| iPS:4 35837 | 21-225_198G3 | VH3\|3-33/D4\|4-17\|RF2/JH6 | | QVQLVES-GGGVVQPGRSLKLSCAASG-FTFS | T-----YGMH | WVRQAPGK GLEWVA | VIWYD---GTNKNYADS VKG | RFTISRDNSKNTLCLQMNSL RAEDTAVYYCAR | DQGVGY---------------DGLDV | WGQGT TVTVS S |
| iPS:4 35845 | 21-225_191G1 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNEHYADS VKG | RFTISRDNSKNTLYLQMNSL RAEDTAVYYCAR | DRGVGY---------------YGLDV | WGQGT TVTVS S |
| iPS:4 35859 | 21-225_190E6 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DQGVGY---------------DGLDV | WGQGT TVTVS S |

| | | | | | | | | | | |
|---|---|---|---|---|---|---|---|---|---|---|
| iPS:4 35873 | 21-225_190G4 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAMYYCAR | DQGVGY-------------------DGLDV | WGQGT SVTVS S |
| iPS:4 35933 | 21-225_190F8 | VH3\|3-33/D4\|4-17\|RF2/JH6 | | QVQLVES-GGGVVQPGRSLRLSCAASG-FTFS | T-----YGMH | WVRQAPGK GLEWVA | VIWYD---GSNKNYADS VKG | RFTISRDNSKNTLYLQMNSL RAEDTAVYYCAR | DQGVGY-------------------DGLDV | WGQGT TVTVS S |
| iPS:4 35941 | 21-225_191E8 | VH3\|3-33/D4\|4-17\|RF2/JH6 | | QVQLVES-GGGVVQPGRSLRLSCAASG-FTFS | N-----YGMH | WVRQAPGK GLEWVA | IIWFD---GSNQYYADS VKG | RFTISRDNSKNTLYLQLNSL RAEDTAVYYCAR | AHGVYY-------------------YAMDV | WGQGT TVTVS S |
| iPS:4 35945 | 21-225_191A10 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAMYYCAR | DQGVGY-------------------DGLDV | WGQGT SVTVS S |
| iPS:4 35947 | 21-225_191E10 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAMYYCAR | DQGVGY-------------------DGLDV | WGQGT SVTVS S |
| iPS:4 35957 | 21-225_191G12 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DQGVGY-------------------DGLDV | WGQGT TVTVS S |
| iPS:4 35963 | 21-225_192D2 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNMLYLQMNSL RAEETAVYYCAR | DRGVGY-------------------YGMDV | WGQGT TVTVS S |
| iPS:4 35971 | 21-225_192D3 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNEHYADS VKG | RFTISRDNSKNTLYLQMNSL RAEDTAVYYCAR | DRGVGY-------------------YGLDV | WGQGT TVTVS S |
| iPS:4 35979 | 21-225_192H4 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DQGVGY-------------------YGMDV | WGQGT TVTVS S |
| iPS:4 35987 | 21-225_192G6 | VH3\|3-33/D4\|4-17\|RF2/JH6 | | QVQLVES-GGGVVQPGRSLRLSCAASG-FTFS | S-----YGMH | WVRQAPGK GLEWVA | VLWYD---GTNKNYADS VKG | RFTISRDNSKNTLYLQMNSL RAEDTAVYYCAR | DQGVGY-------------------DGLDV | WGQGT TVTVS S |
| iPS:4 35993 | 21-225_192C8 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNEHYADS VKG | RFMISRDNSKNTLYLQMNSL RAEDTAVYYCAR | DRGVGY-------------------YGMDV | WGQGT TVTVS S |
| iPS:4 35997 | 21-225_192G8 | VH3\|3-33/D4\|4-17\|RF2/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DQGVGY-------------------DGLDV | WGQGT TVTVS S |

| iPS:4 36005 | 21-225_192H10 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAMYYCAR | DQGVGY-------------------DGLDV | WGQGTSVTVSS |
| iPS:4 36031 | 21-225_193C7 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAMYYCAR | DQGVGY-------------------DGLDV | WGQGTSVTVSS |
| iPS:4 36045 | 21-225_193A10 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNEHYADSVKG | RFTISRDNSKNTLYLQMNSLRAEDTAVYYCAR | DRGVGY-------------------YGLDV | WGQGTTVTVSS |
| iPS:4 36076 | 21-225_194H11 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNEHYADSVKG | RFTISRDNSKNTLSLQMNSLRAEDTAVYYCAR | DRGVGY-------------------YGLDV | WGQGTTVTVSS |
| iPS:4 36086 | 21-225_191G10 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DQGVGY-------------------DGLDV | WGQGTTVTVSS |
| iPS:4 36090 | 21-225_195A9 | VH3|3-33/D4|4-17|RF2/JH6 | | QVQLVES-GGGVVQPGRSLRLSCAASG-FTFS | S-----YGMH | WVRQAPGKGLQWVA | VIWYD---GSNEHYADSVKG | RFTISRDNSKNTLYLQMNSLRAEDTAVYYCAR | DRGVGY-------------------YGLDV | WGQGTTVTVSS |
| iPS:4 36112 | 21-225_196C7 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNEHYADSVKG | RFTISRDNSKNTLYLQMNSLRAEDTAVYYCAR | DRGVGY-------------------YGLDV | WGQGTTVTVSS |
| iPS:4 36138 | 21-225_197F2 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DQGVGY-------------------DGLDV | WGQGTTVTVSS |
| iPS:4 36152 | 21-225_197B6 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNMLYLQMNSLRAEDTAMYYCAR | DQGVGY-------------------DGLDV | WGQGTSVTVSS |
| iPS:4 36173 | 21-225_197G12 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DQGVGY-------------------DGLDV | WGQGTTVTVSS |
| iPS:4 36189 | 21-225_198B6 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNKHYADSVKG | RFTISRDNSKNTLYLQMNSLRAEDTAVYYCAR | DQGVGY-------------------YGMDV | WGQGTTVTVSS |
| iPS:4 36201 | 21-225_199C5 | VH3|3-33/D4|4-17|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DQGVGY-------------------YGMDV | WGQGTTVTVSS |

| | | | | | | | | | | |
|---|---|---|---|---|---|---|---|---|---|---|
| iPS:4 36203 | 21-225_199A6 | VH3\|3-33/D4\|4-17\|RF2/JH6 | | QVQLVES-GGGVVQPGRSLRL SCAASG-FTFS | N-----YGMH | WVRQAPGK GLEWVA | IIWFD--- GSNQYYADS VKG | RFTISRDNSKNTLYLQLNSL RAEDTAVYYCAR | AHGVYY---------- ---------YAMDV | WGQGT TVTVS S |
| iPS:4 36282 | 21-225_204G6 | VH3\|3-33/D4\|4-17\|RF2/JH6 | | QVQLVES-GGGVVQPGRSLRL SCTASG-FTFS | S-----YGMH | WVRQAPGK GLEWVA | VIWYD--- GSNKNYADS VKG | RFTISRDNSKNTLYLQMNSL RAEDTAVYYCAR | DRGVGY---------- ---------DGMDV | WGQGT TVTVS S |
| iPS:4 36296 | 21-225_205F5 | VH3\|3-33/D4\|4-17\|RF2/JH6 | | QVQLVES-GGGVVQPGRSLRL SCAASG-FTFS | R-----YGMH | WVRQAPGK GLEWVA | VIWYD--- GSNENYVDS VKG | RFTISRDISKKMLFLQMNSL RTDDTAVYYCAR | DMGIGY---------- ---------YGMDV | WGQGT TVTVS S |
| iPS:4 36324 | 21-225_207G6 | VH3\|3-33/D4\|4-17\|RF2/JH6 | | QVQLVES-GGGVVQPGRSLRL SCAASG-FTFS | S-----YGMH | WVRQAPGK GLEWVA | VIWYD--- GSNKNYAES VKG | RFTISRDNSKNTLYLQMNSL RAEDTAVYYCAR | DAGIGY---------- ---------YGIDV | WGQGT TVTVS S |
| iPS:4 36364 | 21-225_211A11 | VH3\|3-33/D4\|4-17\|RF2/JH6 | | QVQLVGS-GGGVVQPGRSLRL SCAASG-FTFS | S-----YGMH | WVRQAPGK GLEWVA | VLWFD--- GSNRNYADS VKG | RFTISRDNSKNTLYLQMNSL RAEDTAVYYCAR | DRGVGY---------- ---------YGTDV | WGQGT TVTVS S |
| iPS:4 36372 | 21-225_211A8 | VH3\|3-33/D4\|4-17\|RF2/JH6 | | QVQLVGS-GGGVVQPGRSLRL SCAASG-FTFS | S-----YGMH | WVRQAPGK GLEWVA | VIWYD--- GSNEHYADS VKG | RFTISRDNSKKTLYLQMNSL RAEDTAVYYCAR | DHGVGY---------- ---------YGMDV | WGQGT TVTVS S |
| iPS:4 36376 | 21-225_212E6 | VH3\|3-33/D4\|4-17\|RF2/JH6 | | QVQLVES-GGGVVQPGRSLRL SCAASG-FTFS | S-----YGMH | WVRQAPGK GLEWVA | VIWYD--- GSNKNYVDS VKG | RFTISRDNSKNTLYLQMNSL RAEDTAVYYCAR | DYGVGY---------- ---------YGTDV | WGQGT TVTVS S |
| iPS:4 36378 | 21-225_212D7 | VH3\|3-33/D4\|4-17\|RF2/JH6 | | QVQLVES-GGGVVQPGRSLRL SCAASG-FTFS | S-----YGMH | WVRQAPGK GLEWVA | VIWYD--- GSNKNYADS VKG | RFTISRDNSKNTLSLQMNSL RAEDTAVYYCAR | DYGVGY---------- ---------YGTDV | WGQGT TVTVS S |
| iPS:4 36380 | 21-225_212H9 | VH3\|3-33/D4\|4-17\|RF2/JH6 | | QVQLVES-GGGVVQPGRSLRL SCAASG-FTFS | S-----YGMH | WVRQAPGK GLEWVA | VIWYD--- GSNEHYADS VKG | RFTISRDNSKKTLYLQMNSL RAEDTAVYYCAR | DHGVGY---------- ---------YGMDV | WGQGT TVTVS S |
| iPS:4 36384 | 21-225_212F10 | VH3\|3-33/D4\|4-17\|RF2/JH6 | | QVQLVES-GGGVVQPGRSLRL SCAASG-FTFS | R-----YGMH | WVRQAPGK GLEWVA | VIWYD--- GSNKHYADS VKG | RFTISRDNSKNTLYLQMNSL RGEDTAVYYCAR | DRGVGY---------- ---------NGMDV | WGQGT TVTVS S |
| iPS:4 36390 | 21-225_213D2 | VH3\|3-33/D4\|4-17\|RF2/JH6 | | QVQLVES-GGGVVQPGRSLRL SCAASG-FTFS | S-----YGMH | WVRQAPGK GLEWVA | VIWYD--- GSNKNYADS VKG | RFTISRDNSKNTLSLQMNSL RAEDTAVYYCAR | DYGVGY---------- ---------YGTDV | WGQGT TVTVS S |
| iPS:4 36394 | 21-225_213C4 | VH3\|3-33/D4\|4-17\|RF2/JH6 | | QVQLVES-GGGVVQPGRSLRL SCATSG-FTFS | S-----YGMH | WVRQAPGK GLEWVA | VIWYD--- GSNKHYADS VKG | RFTISRDNSKNTLYLQMNSL RAEDTAVYYCAR | DYGVGY---------- ---------DGMDV | WGQGT TVTVS S |

| ID | Name | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 36398 | 21-225_213B8 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKHYADSVKG | RFTISRDNSKNTLYLQMNSLRAEDTAVYYCAR | DYGVGY-------------------YGTDV | WGQGTTVTVSS |
| iPS:4 36404 | 21-225_214C3 | VH3\|3-33/D4\|4-17\|RF2/JH6 | | QVQLVES-GGGVVQPGRSLRLSCEASG-FTFS | S-----YGMH | WVRQAPGKGLEWVA | VIWYD---GSNKNYGDSVKG | RFTISRDNSKNTLYLQMNSLRAEDTAVYYCAR | DRGVGY-------------------DGMDV | WGQGTTVTVSS |
| iPS:4 36410 | 21-225_212E10 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLSLQMNSLRAEDTAVYYCAR | DYGVGY-------------------YGTDV | WGQGTTVTVSS |
| iPS:4 36420 | 21-225_215B5 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLSLQMNSLRAEDTAVYYCAR | DYGVGY-------------------YGTDV | WGQGTTVTVSS |
| iPS:4 36422 | 21-225_215D6 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DCGVGY-------------------YGTDV | WGQGTTVTVSS |
| iPS:4 36430 | 21-225_215A12 | VH3\|3-33/D4\|4-17\|RF2/JH6 | | QVQLVES-GGGVVQPGRSLRLSCAASG-LTFS | S-----YGMH | WVRQAPGKGLEWVA | VIWYD---GSNEHYADSVKG | RFTISRDNSKNTLYLQMNSLRAEDTAVYYCAR | DRGVGY-------------------YGMDV | WGQGTTVTVSS |
| iPS:4 36452 | 21-225_217G5 | VH3\|3-33/D4\|4-17\|RF2/JH6 | | QVQLVES-GGGVVQPGRSLRLSCAASG-FTFS | S-----NGMH | WVRQAPGKGLEWVA | VIWYD---GSNKNYADSVKG | RFTISRDNSKNTLYLQMNSLRAEDTAVYYCAR | DYGVGY-------------------YGLDV | WGQGTTVTVSS |
| iPS:4 36464 | 21-225_219H1 | VH3\|3-33/D4\|4-17\|RF2/JH6 | | QVQLVES-GGGVVQPGRSLRLSCAASG-FTFS | R-----YGMH | WVRQAPGKGLEWVA | VIWYD---GSNKHYADSVKG | RFTISRDNSKNTLYLQMNSLRGEDTAVYYCAR | DRGVGY-------------------NGMDV | WGQGTTVTVSS |
| iPS:4 51120 | 21-225_197D3 | VH3\|3-33/D4\|4-17\|RF2/JH6 | | QVQLVES-GGGVVQPGRSLRLSCAASG-FIFS | S-----HGMH | WVRQAPGKGLEWVA | VIWYD---GSNEHYADSVKG | RFTISRDNSKNTLYLQMNSLRAEDTAVYYCAR | DQGVGY-------------------YGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D4\|4-17\|RF2/JH4 | | 32224 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYGDY-------------YFDY | WGQGTLVTVSS |
| iPS:4 68822 | 21-225_147E10 | VH3\|3-33/D4\|4-17\|RF2/JH4 | | QVQLVES-GGGVVQPGKSLRLSCAASG-FTFS | N-----YGLH | WVRQAPGKGLEWVA | IIWYD---GSNKYYADSVKG | RFTISRDNSKNTLYLQMNSLRAEDTAVYYCAR | DHYDFW----------SGHFDY | WGQGTLVTVSS |
| iPS:4 33965 | 21-225_46F2 | VH3\|3-33/D4\|4-17\|RF2/JH4 | | QVQLVES-GGGVVQPGRSLRLSCAASG-FTFS | S-----YGMH | WVRQAPGKGLEWVA | IIWYD---GSNKYYVDSVKG | RFTISRDNSKNTLYLQMNSLRAEDTAVYYCAR | DRYDFW----------SGYFDY | WGQGTLVTVSS |

| | | | | | | | | | | |
|---|---|---|---|---|---|---|---|---|---|---|
| iPS:4 34255 | 21-225_62E6 | VH3\|3-33/D4\|4-17\|RF2/JH4 | | QVQLVES-GGGVVQPGRSLRL SCAASG-FTFS | S-----YGMH | WVRQAPGK GLEWVA | AIWYD---GSNKYYGDS VKG | RVTISRDNSKNSLHLQMNSL RAEDTAVYYCAR | DQGIVG------------------ATWFDY | WGQGT LVTVS S |
| iPS:4 34269 | 21-225_57H3 | VH3\|3-33/D4\|4-17\|RF2/JH4 | | QVQLVES-GGGVVQPGRSLRL SCAASG-FTFS | S-----YGMH | WVRQAPGK GLEWVA | AIWYD---GSNKYYADS VKG | RFTISRDNSKNTLYLQMNSL RAEDTAVYYCAR | DYGIVG------------------ATYFDY | WGQGT LVTVS S |
| iPS:4 34345 | 21-225_64H9 | VH3\|3-33/D4\|4-17\|RF2/JH4 | | QVQLVES-GGGVVQPGRSLRL SCAASG-FTFS | T-----YGMH | WVRQAPGK GLEWVA | IIWYD---GSNKYYADS VKG | RFTISRDNSKNTLYLQMNSL RAEDTAVYYCAR | DIYDFW------------------SGYLGY | WGQGT LVTVS S |
| iPS:4 34363 | 21-225_65A6 | VH3\|3-33/D4\|4-17\|RF2/JH4 | | QVQLVES-GGGVVQPGRSLRL SCAASG-FTFS | N-----YGMH | WVRQAPGK GLEWVA | VIWYD---GSNKYYGDS VKG | RFTISRDNSKNTLYLQMNSL RAEDTAVYYCAR | DQGIVG------------------ATWFDY | WGQGT LVTVS S |
| iPS:4 34393 | 21-225_67C3 | VH3\|3-33/D4\|4-17\|RF2/JH4 | | QVQLVES-GGGVVQPGRSLRL SCAASG-FTFS | S-----YGMH | WVRQAPGK GLEWVA | AIWYD---GSNKYYGDS VKG | RVTISRDNSKNSLHLQMNSL RAEDTAVYYCAR | DQGIVG------------------ATWFDY | WGQGT LVTVS S |
| iPS:4 34425 | 21-225_70A5 | VH3\|3-33/D4\|4-17\|RF2/JH4 | | QVQLVES-GGGVVQPGRSLRL SCAASG-FTFS | S-----YGMH | WVRQAPGK GLEWVA | VIWYD---GSNKYYADS VKG | RFTISRDNSKNSLYLQMNSL SAEDTAVYYCAR | DQGIVG------------------ATWFDY | WGQGT LVTVS S |
| iPS:4 35341 | 21-225_148B2 | VH3\|3-33/D4\|4-17\|RF2/JH4 | | QVQLVES-GGGVVQPGRSLRL SCAASG-FTFS | S-----YGMH | WVRQAPGK GLEWVA | IIWYD---GSYKYYADS VKG | RFTISRDNSKNTLYLQMNSL SAEDTAVYYCAR | DHFDFW------------------SGHFDY | WGQGT LVTVS S |
| iPS:4 35357 | 21-225_148G10 | VH3\|3-33/D4\|4-17\|RF2/JH4 | | QVQLVES-GGGVVQPGRSLRL SCAASG-FTFS | S-----YGMH | WVRQAPGK GLEWVA | IIWYD---GSNKYYADS VKG | RFTISRDNSKNTLYLQMNSL RAEDTAVYYCAR | DRYDFW------------------SGHFDY | WGQGT LVTVS S |
| iPS:4 35365 | 21-225_149F1 | VH3\|3-33/D4\|4-17\|RF2/JH4 | | QVQLVES-GGGVVQPGRSLRL SCAASG-FTFS | S-----YGMH | WVRQAPGK GLEWVA | IIWYD---GSYKYYADS VKG | RFTISRDNSKNTLYLQMNSL SAEDTAVYYCAR | DHFDFW------------------SGHFDY | WGQGT LVTVS S |
| iPS:4 35413 | 21-225_150B11 | VH3\|3-33/D4\|4-17\|RF2/JH4 | | QVQLVES-GGGVVQPGRSLRL SCAASG-FTFS | S-----YGMH | WVRQAPGK GLEWVA | IIWYD---GSNKYYADS VKG | RFTISRDNSKNTLYLQMNSL RAEDTAVYYCAR | DRYDFW------------------SGHFDY | WGQGT LVTVS S |
| iPS:4 35423 | 21-225_151G5 | VH3\|3-33/D4\|4-17\|RF2/JH4 | | QVQLMES-GGGVVQPGRSLRL SCAASG-FIFS | S-----YGMH | WVRQAPGK GLEWVA | IIWYD---GSNKYYADS VKG | RFTISRDNSKNTLYLQMNSL RAEDTAVYYCAR | DRYDFW------------------SGHFDY | WGQGT LVTVS S |
| iPS:4 35429 | 21-225_151A10 | VH3\|3-33/D4\|4-17\|RF2/JH4 | | QVQLVES-GGGVVQPGRSLRL SCAASG-FTFS | N-----YGMH | WVRQAPGK GLEWLA | IIWYD---GSNKYYADS VKG | RFTISRDNSKNTLYLQMNSL RAEDTAVYYCAR | DHYDFW------------------SGHFDY | WGQGT LVTVS S |

| ID | Clone | Germline | # | H_FR1 | H_CDR1 | H_FR2 | H_CDR2 | H_FR3 | H_CDR3 | H_FR4 |
|---|---|---|---|---|---|---|---|---|---|---|
| iPS:4 35489 | 21-225_155A5 | VH3\|3-33/D4\|4-17\|RF2/JH4 | | QVQLVES-GGDVVQPGRSLRLSCAASG-FTFS | S-----YGMH | WVRQAPGKGLEWVA | IIWYD---GSSKYYADSVKG | RFTISRDNSKNTLYLHMNSLRAEDTAVYYCAR | DRYDFW-------------------SGHFDY | WGQGTLVTVS S |
| iPS:4 35683 | 21-225_170A1 | VH3\|3-33/D4\|4-17\|RF2/JH4 | | QVQLVES-GGGVVQPGRSLRLSCAASG-FTFS | S-----YGMH | WVRQAPGKGLEWVA | IIWYD---GSYKYYADSVKG | RFTISRDNSKNTLYLQMNSLRAEDTAVYYCAR | DAHDFW-------------------SGYFDS | WGQGTLVTVS S |
| iPS:4 35755 | 21-225_176H4 | VH3\|3-33/D4\|4-17\|RF2/JH4 | | QVQLVES-GGGVVQPGRSLRLSCAASG-FTFS | S-----YGMH | WVRQAPGKGLEWVA | IIWYD---GSYKYYADSVKG | RFTISRDNSKNTLYLQMSSLRAEDTAVYYCAR | DAHDFW-------------------SGYFAY | WGQGALVTVS S |
| iPS:4 35795 | 21-225_181C2 | VH3\|3-33/D4\|4-17\|RF2/JH4 | | QVQLVES-GGGVVQPGRSLRLSCVASG-FTFS | S-----YGMH | WVRQAPGKGLEWVT | IIWYD---GSYKYYADSVKG | RFTISRDNSKNTLYLQMNSLRAEDTAVYYCAR | DHYDFW-------------------SGHFDF | WGQGTLVTVS S |
| iPS:4 35807 | 21-225_181C10 | VH3\|3-33/D4\|4-17\|RF2/JH4 | | QVQLVES-GGGVVQPGRSLRLSCAASG-FTFS | S-----YGMH | WVRQAPGKGLEWMA | IIWYD---GSYKYYADSVKG | RFTISRDNSKNTLYLQMNSLRAEDTAVYYCAR | DHYDFW-------------------SGHFDY | WGQGTLVTVS S |
| iPS:4 35887 | 21-225_186F7 | VH3\|3-33/D4\|4-17\|RF2/JH4 | | QVQLVES-GGGVVQPGRSLRLSCAASG-FTFS | T-----YGMH | WVRQAPGKGLEWVA | IIWYD---GSYKYYADSVKG | RFTISRDNSKNTLYLQMNSLRAEDTAVYYCAR | DHYDFW-------------------SGHFDY | WGQGTLVTVS S |
| iPS:4 35901 | 21-225_189G2 | VH3\|3-33/D4\|4-17\|RF2/JH4 | | QVQLVES-GGGVVQPGRSLRLSCAASG-FTFS | N-----YGMH | WVRQAPGKGLEWVA | IIWYD---GSYKYYADSVKG | RFTISRDNSKNTLYLQMNSLRAEDTAVYYCAR | DRFDFW-------------------SGYSDY | WGQGTLVTVS S |
| iPS:4 36594 | 21-225_226A5 | VH3\|3-33/D4\|4-17\|RF2/JH4 | | QVQLVES-GGGVVQPGRSLRLSCAASG--FTFS | N-----YGMH | WVRQAPGKGLEWVA | IIWYD---GTNKYYTDSVKG | RFTISRDNSKNTLYLQMNSLRAEDTAVYYCAR | EGHDFW-------------------SGFFCY | WGQGTLVTVS S |
| iPS:3 92814 | 21-225_22A1 | VH3\|3-33/D4\|4-17\|RF2/JH4 | | QVQLVES-GGGVVQPGRSLRLSCAASG-FTFS | S------YGMH | WVRQAPGKGLEWVA | VMWYD---GSNKYYADSVKG | RFTISRDNSKNTLYLQMNSLRAEDTAVYYCAR | DGGFL---------------------EWLDY | WGQGTLVTVS S |
| iPS:3 93036 | 21-225_28G3 | VH3\|3-33/D4\|4-17\|RF2/JH4 | | QVQLVES-GGGVVQPGRSLRLSCAASG-FIFS | T-----YGMH | WVRQAPGKGLEWVA | VIWYD---GSNKYYADSVKG | RFTISRDNSKNTLYLQMNSLRAEDTAVYYCAR | DRYDFW-------------------SGYFDY | WGQGTLVTVS S |
| **Germline** | | | | **H_FR1** | **H_CDR1** | **H_FR2** | **H_CDR2** | **H_FR3** | **H_CDR3** | **H_FR4** |
| | VH3\|3-33/D7\|7-27\|RF1/JH4 | | 32225 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | LTGY------------------------EDY | WGQGTLVTVS S |
| iPS:4 68824 | 21-225_73G6 | VH3\|3-33/D7\|7-27\|RF1/JH4 | | QVQLVES-GGGVVQPGRSLRLSCAASG-FTFS | S-----YGMH | WVRQAPGKGLEWVA | VIWYD---VSNKYYGDSVKG | RFTISRDNSKNTLYLQMNSLRAEDTAVYYCAR | EVGM----------------------TSDY | WGQGTLVTVS S |

| iPS:4 34169 | 21-225_50C4 | VH3\|3-33/D7\|7-27\|RF1/JH4 | | QVQLVES-GGGVVQPGRSLRLSCAASG-FTFS | D-----YGMH | WVRQAPGK GLEWVA | VIWYE---ETNKYYADS VKG | RFTISRDNSKNTLYLQMNSL RGEDTAVYYCAR | EVGF--------------------LNDY | WGQGI LVTVS S |
|---|---|---|---|---|---|---|---|---|---|---|
| iPS:4 35045 | 21-225_90H5 | VH3\|3-33/D7\|7-27\|RF1/JH4 | | QVQLVES-GGGVVQPGRSLRLSCAASG-FTFS | D-----YGMH | WVRQSPGK GLEWVA | VIWYE---GSNTYYADS VKG | RFTISRDNSKNTLYLQMNSL RAEDTAVYYCVR | EMGW--------------------LDDY | WGQGT LVTVS S |
| iPS:4 35367 | 21-225_149G1 | VH3\|3-33/D7\|7-27\|RF1/JH4 | | QVQLVES-GGGVVQPGRSLRLSCAASG-FTFS | D-----YGMH | WVRQAPGK GLEWVA | VIWYE---GSNKYYADS VKG | RFTISRDNSKNMLYLQMNSL RAEDTAVYYCAR | EIGF--------------------SEDY | WGQGT LVTVS S |
| iPS:4 35397 | 21-225_149F12 | VH3\|3-33/D7\|7-27\|RF1/JH4 | | QVQLVES-GGGVVQPGRSLRLSCAASG-FTFS | D-----YGMH | WVRQAPGK GLEWVA | VIWYE---ENNKYYADS VKG | RFTISRDNSKNTLFLQMNSL RAEDTAVYYCAR | EIGF--------------------SEDY | WGQGT LVTVS S |
| iPS:4 35407 | 21-225_150E7 | VH3\|3-33/D7\|7-27\|RF1/JH4 | | QVQLVES-GGGVVQPGRSLRLSCAASG-FTFS | D-----YGMH | WVRQAPGK GLEWVA | VIWYE---ENNKYYADS VKG | RFTISRDNSKNTLFLQMNSL RAEDTAVYYCAR | EIGF--------------------SEDY | WGQGT LVTVS S |
| iPS:4 35609 | 21-225_161F7 | VH3\|3-33/D7\|7-27\|RF1/JH4 | | QVQLVES-GGGVVQPGRSLRLSCAASG-FTFS | D-----FGLH | WVRQAPGQ GLEWVA | VIWFD---GSNKYYADS VKG | RFTIFRDNSKNTLYLQMNSL RAEDTAVYYCAR | EIGW--------------------LSDY | WGQGT LVTVS S |
| iPS:4 35613 | 21-225_161D11 | VH3\|3-33/D7\|7-27\|RF1/JH4 | | QVQLVES-GGGVVQPGRSLRLSCAASG-FTFS | D-----FGLH | WVRQAPGQ GLEWVA | VIWFD---GSNKYYADS VKG | RFTIFRDNSKNTLYLQMNSL RAEDTAVYYCAR | EIGW--------------------LSDY | WGQGT LVTVS S |
| iPS:4 35791 | 21-225_180H7 | VH3\|3-33/D7\|7-27\|RF1/JH4 | | QVQLVES-GGGVVQPGRSLRLSCAASG-FTFS | D-----YGMH | WVRQAPGK GLEWVA | VIWYD---ENNKHYADS AKG | RFTISRDNSKNTLYLQMNSL RVEDTAVYYCAR | EVGW--------------------SDDY | WGQGT LVTVS S |
| iPS:4 35805 | 21-225_181A8 | VH3\|3-33/D7\|7-27\|RF1/JH4 | | QVQLVES-GGGVVQPGRSLRLSCAASG-FTFS | D-----YGMH | WVRQAPGK GLEWVA | VIWYD---ENNKHYADS AKG | RFTISRDNSKNTLYLQMNSL RAEDTAVYYCAR | EVGW--------------------SDDY | WGQGT LVIVS S |
| iPS:4 35879 | 21-225_184H10 | VH3\|3-33/D7\|7-27\|RF1/JH4 | | QVQLVES-GGGVVQPGRSLRLSCVASG-FTFS | D-----YGMH | WVRQAPGK GLEWVA | VIWYD---ETNKHYGDS VKG | RFTISRDNSKDTLYLQMNSL RAEDTAVYYCAR | EVGW--------------------HDDY | WGQGT LVTVS S |
| iPS:4 35881 | 21-225_184D11 | VH3\|3-33/D7\|7-27\|RF1/JH4 | | QVQLVES-GGGVVQPGRSLRLSCAASG-FTFS | D-----YGMH | WVRQAPGK GLEWVA | VIWYD---ETNKHYGDS VKG | RFTISRDNSKDTLYLQMNSL RAEDTAVYYCAR | EVGW--------------------HDDY | WGQGT LVTVS S |
| iPS:4 36350 | 21-225_210E4 | VH3\|3-33/D7\|7-27\|RF1/JH4 | | QVQLVES-GGGVVQPGRSLRLSCAASG-FTLI | N-----YGMH | WVRQAPGK GLEWVA | VIWYD---ENNKYYVDS VKG | RFTISRDDSKNTLYLQMNSL RAEDSAVYYCAR | ETGF--------------------LSDY | WGQGT LVTVS S |

| iPS:4 36576 | 21-225_225B6 | VH3\|3-33/D7\|7-27\|RF1/J H4 | | QVRLVES-GGGVVQPGRSLRL SCAASG-FTFS | D-----YGMH | WVRQAPGK GLEWVA | VIWYD--- ENNKYYADS VKG | RFTISRDNSKNTLYLQMNSL RAEDTAVYYCAR | EVGF------------ ----------TEDY | WGQGT LVTVS S |
| iPS:4 36578 | 21-225_225D6 | VH3\|3-33/D7\|7-27\|RF1/J H4 | | QVQLVES-GGGVVQPGRSLRL SCAASG-FTFS | N-----YGMH | WVRQAPGK GLEWVA | VIWYD--- ENNKYYADS VKG | RFTISRDNSQNTLYLQMTSL RAEDTAVYYCAR | EVGF------------ ----------TEDY | WGQGT LVTVS S |
| iPS:4 36582 | 21-225_225F8 | VH3\|3-33/D7\|7-27\|RF1/J H4 | | QVQLVES-GGGVVQPGRSLRL SCAASG-FTFS | S-----YGMH | WVRQAPGK GLEWVA | VIWYD--- ENNKYYVDS VKG | RFTISRDNSKNTLYLQMNSL RAEDTAVYYCAR | EVGF------------ ----------TEDY | WGQGT LVTVS S |
| iPS:4 36608 | 21-225_226A9 | VH3\|3-33/D7\|7-27\|RF1/J H4 | | QVQLVES-GGGVVQPGRSLRL SCAASG-FTFS | N-----YGMH | WVRQAPGK GLEWVA | VIWYE--- ESNKYYTDS VKG | RFTISRDNSKNTLFLQMNSL RAEDTAVYYCAR | EVGF------------ ----------TEDY | WGQGT LVTVS S |
| iPS:4 36630 | 21-225_227G3 | VH3\|3-33/D7\|7-27\|RF1/J H4 | | QVQLVES-GGGVVQPGRSLRL SCAASG-FTFS | S-----YGMH | WVRQAPGK GLEWVA | VIWYV--- GSNQYYADS VKG | RFTISRDNSKNTLYLQMSSL RAEDTAVYYCAR | EVGF------------ ----------TEDY | WGQGT LVTVS S |
| iPS:4 36634 | 21-225_227H5 | VH3\|3-33/D7\|7-27\|RF1/J H4 | | QVQLVES-GGGVVQPGRSLRL SCAASG-FTFS | N-----YGMH | WVRQAPGK GLDWVA | VIWYE--- ESNKYYADS VKG | RFTISRDNSKNTLYLQMNSL RAEDTAMYYCAR | EVGF------------ ----------TEDY | WGQGT LVTVS S |
| iPS:4 36650 | 21-225_227C12 | VH3\|3-33/D7\|7-27\|RF1/J H4 | | QVQLVES-GGGVVQPGKSLRL SCAASG-FTFS | N-----YGMH | WVRQAPGK GLEWVA | VIWYI--- GSNQYYADS VKG | RFTISRDNSKNTLYLQMSSL RAEDTAVYYCAR | EVGF------------ ----------TEDY | WGQGT LVTVS S |
| iPS:4 37280 | 21-225_203C10 | VH3\|3-33/D7\|7-27\|RF1/J H4 | | QVQLVES-GGGVVQPGRSLRL SCAASG-FTFS | D-----YGMH | WVRQAPGK GLEWVA | VIWYD--- GGNTHYTDS VKG | RFTISRDNSKNTLYLQMNSL RAEDTAVYYCAR | EVGW------------ ----------LDDY | WGQGT LVTVS S |
| iPS:3 92740 | 21-225_18H12 | VH3\|3-33/D7\|7-27\|RF1/J H4 | | QVQLVES-GGGVVQPGRSLRL SCAASG-FTFS | D-----YGMH | WVRQAPGK GLEWMA | VIWYD--- VTNKYYADS VKG | RFTISRDNSKNTLYLQMNSL RAEDTAVYYCAR | EVGW------------ ----------YEDY | WGQGT LVTVS S |
| iPS:3 92780 | 21-225_22B7 | VH3\|3-33/D7\|7-27\|RF1/J H4 | | QVQLVES-GGGVVQPGRSLRL SCAASG-FTFS | S-----YGMH | WVRQAPGK GLEWVA | VIWYE--- ENNQYYADS VKG | RFTISRDNSKNTLYLQMNSL RAEDTAVYYCAR | EVGF------------ ----------RSDY | WGQGT LVTVS S |
| iPS:3 92912 | 21-225_25A9 | VH3\|3-33/D7\|7-27\|RF1/J H4 | | QVQLVES-GGGVVQPGRSLRL SCAASG-FTFS | S-----YGMH | WVRQAPGK GLEWVA | VIWYD--- VTNKYYTGS VKG | RFTISRDNSKNTLYLQMNSL RAEDTAVYYCAR | EIGW------------ ----------LDDY | WGQGT LVTVS S |
| iPS:3 92940 | 21-225_29D9 | VH3\|3-33/D7\|7-27\|RF1/J H4 | | QVQLVES-GGGVVQPGRSLKL SCSASG-FTFS | D-----YGIH | WVRQAPGK GLEWVA | VIWYD--- ESNNYYADS VKG | RFTISRDNSKNTLYLQMNSL RAEDTAVYYCAR | EIGW------------ ----------LDDY | WGQGT QVTVS S |

| | | | | | | | | | |
|---|---|---|---|---|---|---|---|---|---|
| iPS:3 92948 | 21-225_25G5 | VH3|3-33/D7|7-27|RF1/JH4 | | QVQLVES-GGGVVQPGRSLRLSCAASG-FTFS | D-----YGIH | WVRQAPGKGLEWVA | VIWYD---GNNKYYADSVKG | RFTISRDNSKNTLYLQMNNLRAEDTAVYYCAR | EIGW-----------------------LDDY | WGQGTLVTVSS |
| iPS:3 92978 | 21-225_28B8 | VH3|3-33/D7|7-27|RF1/JH4 | | QVQLVES-GGGVVQPGRSLRLSCAASG-FTFS | D-----YGMH | WVRQAPGKGLEWVT | VIWYD---ANNKYYADSVKG | RFTISRDNFKNTVYLQMNSLRAEDTAVYYCAR | EIGW-----------------------LDDY | WGQGTLVTVSS |
| iPS:3 92998 | 21-225_28A9 | VH3|3-33/D7|7-27|RF1/JH4 | | QVQLVES-GGGVVQPGRSLRLSCAASG-FTFS | S-----YGIH | WVRQAPGKGLEWVA | VIWFD---GSNKYYADSVKG | RFTISRDNSKNTLFLQMNSLRVEDTAVYYCAR | EIGW-----------------------LDDY | WGQGTLVTVSS |
| iPS:3 93038 | 21-225_29D8 | VH3|3-33/D7|7-27|RF1/JH4 | | QVQLVES-GGGVVQPGRSLRLSCAASG-FTFR | D-----YGIH | WVRQAPGKGLEWVA | VIWFD---GTNKYYADSVKG | RFTISRDNSKNTLYLQMNSLRAEDTAVYYCGR | EIGW-----------------------LDDY | WGQGTLVTVSS |
| iPS:3 93056 | 21-225_30F3 | VH3|3-33/D7|7-27|RF1/JH4 | | QVQLVES-GGGVVQPGRSLRLSCAASG-FTFS | S-----YGMH | WVRQAPGKGLEWVA | VIWYD---VSNKYYADSVKG | RFTISRDNSKNTLYLQMNSLRAEDTAVYYCAR | EMGW-----------------------YDDY | WGQGTLVTVSS |
| iPS:3 93074 | 21-225_33B1 | VH3|3-33/D7|7-27|RF1/JH4 | | QVQLVES-GGGVVQPGRSLRLSCAASG-FTFS | D-----YGMH | WVRQAPAKGLEWVA | VIWYD---RNNKYYADSVKG | RFTISRDNSKNTLYLQMNSLRAEDTAVYYCAR | EMGW-----------------------YDDY | WGQGTLVTVSS |
| iPS:3 93822 | 21-225_15B11 | VH3|3-33/D7|7-27|RF1/JH4 | | QVQLVES-GGGVVQPGRSLRLSCAASG-FTFS | N-----YGMH | WVRQAPGKGLEWVA | VIWYE---ESNKYYTDSVKG | RFTISRDNSKNTLYLQMNSLRPEDTAVYYCAR | EVGF-----------------------TEDY | WGQGTLVTVSS |
| iPS:3 93856 | 21-225_14C2 | VH3|3-33/D7|7-27|RF1/JH4 | | QVQLVES-GGGVVQPGRSLRLSCAASG-FTFS | D-----YGMH | WVRQAPGKGLEWVA | VIWYD---ESNKYYEDSVKG | RFTISRDNSKNTLYLQMKSLRAEDTGVYYCAR | EVGF-----------------------RSDY | WGQGTLVTVSS |
| iPS:3 93874 | 21-225_4C8 | VH3|3-33/D7|7-27|RF1/JH4 | | QVQLVES-GGGVVQPGRSLRLSCAASG-FTFS | N-----YGMH | WVRQAPGKGLEWVA | VIWYE---ENNQYYADSVKG | RFTISRDNSKNTLYLQMNSLTAEDTAVYYSPR | EMGF-----------------------LSDY | WGQGTLVTVSS |
| iPS:3 93984 | 21-225_4F12 | VH3|3-33/D7|7-27|RF1/JH4 | | QVQLVES-GGGVVQPGRSLRLSCAASG-FTFS | S-----YGMH | WVRQAPGKGLEWVA | VIWYD---VTNKKYADSVKG | RFTISRDNSKNTLYLQMNSLTPENTGGYENQR | EKGG-----------------------LFDY | WGQGTLVTVSS |
| iPS:3 94020 | 21-225_15H10 | VH3|3-33/D7|7-27|RF1/JH4 | | QVQLVES-GGGVVQPGRSLRLSCAASG-FTFS | N-----YGMH | WVRQAPGKGLEWVA | VIWYD---ESNKYYEDSVKG | RFTISRDNSKNTLYLQMNSLRAEDTAVYYCVR | EVGF-----------------------LSDY | WGQGILVTVSS |
| iPS:3 94095 | 21-225_16H4 | VH3|3-33/D7|7-27|RF1/JH4 | | QVQLVES-GGGVVQPGRSLRLSCAASG-FTFS | N-----YGMH | WVRQAPGKGLEWVA | VIWYD---VSNKYYADSVKG | RFTISRDNSKNMLYLQMNSLRAEDTAVYYCAR | EMGW-----------------------TDDC | WGQGTLVTVSS |

| | | Germline | H_FR1 | H_CDR1 | H_FR2 | H_CDR2 | H_FR3 | H_CDR3 | H_FR4 |
|---|---|---|---|---|---|---|---|---|---|
| | VH1\|1-08/D5\|5-24\|RF3/JH6 | 32226 | QVQLVQS-GAEVKKPGASVKV SCKASG-YTFI | S-----YDIN | WVRQATGQ GLEWMG | WMNPN--- SGNTGYAQK FQG | RVTMTRNISISTAYMELSSL RSEDTAVYYCAR | RIGYNYYY--------- -------YYYGMDV | WGQGT TVTVS S |
| iPS:4 68818 | 21-225_190C8 | VH1\|1-08/D5\|5-24\|RF3/J H6 | QVQLVQS-GAEVKKPGASVKV SCKASG-YTFI | S-----YDIN | WVRQATGQ GLEWMG | WMNPK--- RGNTGYAQK FQG | RVTMTRDISISTAYMELSSL RSEDTAVYYCAR | GDPYNWN--------- --------SYAMDV | WGQGA TVTVS S |
| iPS:4 36023 | 21-225_193A5 | VH1\|1-08/D5\|5-24\|RF3/J H6 | QVQLVQS-GAEVKKPGASVKV SCKASG-YTFI | S-----YDIN | WVRQATGQ GLEWMG | WMNPK--- RGNTGYAQK FQG | RVTMTRDISISTAYMELSSL RSEDTAVYYCAR | GDPYNWN--------- --------SYAMDV | WGQGA TVTVS S |
| iPS:4 36132 | 21-225_196C12 | VH1\|1-08/D5\|5-24\|RF3/J H6 | QVQLVQS-GAEVKKPGASVKV SCKASG-YTFI | S-----YDIN | WVRQATGQ GLEWMG | WMNPK--- RGNTGYAQK FQG | RVTMTRDISISTAYMELSSL RSEDTAVYYCAR | GDPYNWN--------- --------SYAMDV | WGQGA TVTVS S |
| | | Germline | H_FR1 | H_CDR1 | H_FR2 | H_CDR2 | H_FR3 | H_CDR3 | H_FR4 |
| | VH3\|3-33/D2\|2-8\|RF3/JH5 | 32227 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | DIVLMVY--------- -------AINWFDP | WGQGT LVTVS S |
| iPS:4 68828 | 21-225_162A10 | VH3\|3-33/D2\|2-8\|RF3/JH 5 | QVQLVES-GGGVVQPGRSLRL SCAASG-FTFS | S-----CGMH | WVRQAPGK GLEWVA | AIWYD--- GSNKYYADS VKG | RFTISRDNSKNTLFLQMNSL RAEDTAVYYCAR | DKNIMG--------- --------DTWFDF | WGQGT LVTVS S |
| | | Germline | H_FR1 | H_CDR1 | H_FR2 | H_CDR2 | H_FR3 | H_CDR3 | H_FR4 |
| | VH1\|1-02/D5\|5-18\|RF3/JH4 | 32228 | QVQLVQS-GAEVKKPGASVKV SCKASG-YTFT | G-----YYMH | WVRQAPGQ GLEWMG | WINPN--- SGGTNYAQK FQG | RVTMTRDTISTAYMELSRL RSDDTAVYYCAR | GYSYG---------- ---------YYFDY | WGQGT LVTVS S |
| iPS:4 68830 | 21-225_191G11 | VH1\|1-02/D5\|5-18\|RF3/J H4 | QVQLVQS-GAEVKKPGASVKV SCKASG-YTFT | G-----YYMH | WVRQAPGQ GLEWMG | WINPN--- SGGTNFAQK FQG | RVTLTRDTSINTAYMELSWL RSDDTAVYYCAR | GKNYG----------- --------SYFDY | WGQGT LVTVS S |
| iPS:4 36896 | 21-225_67F10 | VH1\|1-02/D5\|5-18\|RF3/J H4 | QVQLVQS-GAEVKKPGASVKV SCKASG-YTFT | G-----YYMH | WVRQAPGQ GLEWMG | WINPN--- SGGTNYGQK FQG | RVTMTRDTISTAYMELSRL RSDDTAVYYCAR | TYFYGSGS-------- -------YYNGFDY | WGQGT LVTVS S |
| iPS:3 93218 | 21-225_14G3 | VH1\|1-02/D5\|5-18\|RF3/J H4 | QVQLVQS-GAEVKKPGASVKV SCKASG-YTFT | G-----YYMY | WVRQAPGQ GLEWMG | WINPN--- SGGTNYAQK FQG | RVTMTRDTISTAYMELSRL RSDDTAVYYCAR | SYFYGSGS-------- -------YYNEFDY | WGQGT LVTVS S |
| iPS:3 93565 | 21-225_34B11 | VH1\|1-02/D5\|5-18\|RF3/J H4 | QVKLVQS-GAEVKKPGASVKV SCKASG-YTFT | G-----YYMH | WVRQAPGQ GLEWMG | WINPN--- SGGTNYAQK FQG | RVTMTRDTISTAYMELSRL RSDDTAVYYCAR | VYFYGSGS-------- -------YYNEFDY | WGQGT LVTVS S |
| iPS:3 98470 | 21-225_14B7 | VH1\|1-02/D5\|5-18\|RF3/J H4 | QVQLVQS-GAEVKKPGASVKV SCKASG-YTFT | G-----YYMH | WVRQAPGQ GLEWMG | WINPN--- SGGTNYVQK FQG | RVTMTRDTISTACMELSRL KSDDTAVYFCAR | SFFYGSGS-------- -------YYNEFDY | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-30.3/D1\|1-1\|RF1/JH6 | 32229 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | GTHGTYY----------------YYGMDV | WGQGTTVTVSS |
| iPS:4 68836 | 21-225_198E3 | VH3\|3-30.3/D1\|1-1\|RF1/JH6 | QVQLVES-GGGVVQPGRSLRLSCAASG-FTFS | S-----YGMH | WVRQAPGKGLEWVA | VISYD---GGYKNYADSVKG | RFTISRDNSKNTLYLQMNSLRAEDTAVYYCAR | GTHGYY-------------------YGVDV | WGQGTTVTVSS |
| iPS:4 35831 | 21-225_190C12 | VH3\|3-30.3/D1\|1-1\|RF1/JH6 | QVQLVES-GGAVVQPGRSLRLSCAASG-FTFS | S-----YGMH | WVRQAPGKGLEWVA | VISYD---GGYKNYVDSVKG | RFTISRDNSKNTLYLQMNSLRAEDTAVYYCAR | GTHGYY-------------------YGVDV | WGQGTTVTVSS |
| iPS:4 35857 | 21-225_191A4 | VH3\|3-30.3/D1\|1-1\|RF1/JH6 | QVQLVES-GGGVVQPGRSLRLSCAASG-FTFS | S-----YGMH | WVRQAPGKGLEWVA | VISYD---GGYKNYADSVKG | RFTISRDNSKNTLYLQMNSLRAEDTAVYYCAR | GTHGYY-------------------YGVDV | WGQGTTVTVSS |
| iPS:4 35907 | 21-225_190G3 | VH3\|3-30.3/D1\|1-1\|RF1/JH6 | QVQLVES-GGGVVQPGRSLRLSCAASG-FTFS | S-----YGMH | WVRQAPGKGLEWVA | VISYD---GGYKNYVDSVKG | RFTISRDNSKNTLYLQMNSLRAEDTAVYYCAR | GTHGYY-------------------YGVDV | WGQGTTVTVSS |
| iPS:4 35919 | 21-225_190H5 | VH3\|3-30.3/D1\|1-1\|RF1/JH6 | QVQLVES-GGGVVQPGRSLRLSCAASG-FTFS | S-----YGMH | WVRQAPGKGLEWVA | VISYD---GGYKNYADSVKG | RFTISRDNSKNTLYLQMNSLRAEDTAVYYCAR | GTHGYY-------------------YGVDV | WGQGTTVTVSS |
| iPS:4 35989 | 21-225_192F7 | VH3\|3-30.3/D1\|1-1\|RF1/JH6 | QVQLVES-GGGVVQPGRSLRLSCAASG-FTFS | S-----YGMH | WVRQAPGKGLEWVT | VISYD---GGYKNYADSVKG | RFTISRDNSKNTLYLQMNSLRAEDTAVYYCAR | GTHGYY-------------------YGVDV | WGQGTTVTVSS |
| iPS:4 36222 | 21-225_200C9 | VH3\|3-30.3/D1\|1-1\|RF1/JH6 | QVQLVES-GGGVVQPGRSLRLSCAASG-FTFS | S-----YGMH | WVRQAPGKGLEWVA | VISYD---GGYKNYIDSVKG | RFTISRDNSKNTLYLQMNSLRAEDTAVFYCAR | GTHGYY-------------------YGVDV | WGQGTTVTVSS |
| | | Germline | H_FR1 | H_CDR1 | H_FR2 | H_CDR2 | H_FR3 | H_CDR3 | H_FR4 |
| | VH4\|4-30.1/D5\|5-24\|RF3/JH6 | 32200 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | RDGYNYYY---------------YYGMDV | WGQGTTVTVSS |
| iPS:4 68840 | 21-225_200H9 | VH4\|4-30.1/D5\|5-24\|RF3/JH6 | QVQLQES-GPGLVKPSQTLSLTCIVSG-GSMR | SG---GDYWS | WIRQHPGKGLEWFG | YIYY----SGSTYYNPSLKS | RVTLSVDTSKNQFSLKLSSVTAADTAVYYCAR | MDYSNY-------------------YYGMDV | WGQGTSVTVSS |
| iPS:4 36096 | 21-225_195E10 | VH4\|4-30.1/D5\|5-24\|RF3/JH6 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVAAADTAVYYCAR | GGYNWN-------------------HGMDV | WGQGTTVTVSS |
| iPS:4 36120 | 21-225_196C10 | VH4\|4-30.1/D5\|5-24\|RF3/JH6 | QVQLQES-GPGLVKPSQTLSLTCTVSG-GSMR | SG---GDYWS | WIRQHPGKGLEWFG | FIYY----SGSTYYNPSLKS | RVTLSVDTSKNQFSLKLSSVTAADTAVYYCAR | MDYSNY-------------------YYGMDV | WGQGTTVTVSS |

| iPS:4 | | VH | Germline | H_FR1 | H_CDR1 | H_FR2 | H_CDR2 | H_FR3 | H_CDR3 | H_FR4 |
|---|---|---|---|---|---|---|---|---|---|---|
| iPS:4 36216 | 21-225_200B7 | VH4\|4-30.1/D5\|5-24\|RF3/JH6 | | QVQLQES-GPGLVKPSQTLSL TCTVSG-GSIS | SG---GYYWS | WIRQHPGK GLEWIG | YIFY---- SGSTNYNPS LRS | RVTISVDISKNQFSLKLSSV TAADTAVYYCAR | AGYNWN------------------NGMDV | WGQGT TVTVS S |
| | **Germline** | | | **H_FR1** | **H_CDR1** | **H_FR2** | **H_CDR2** | **H_FR3** | **H_CDR3** | **H_FR4** |
| | VH3\|3-21/D6\|6-6\|RF2/JH4 | | 32231 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | SIAAR------------------YFDY | WGQGT LVTVS S |
| iPS:4 68844 | 21-225_48E10 | VH3\|3-21\|D6\|6-6\|RF2/JH4 | | EVQLVES-GGGLVKPGGSLRL SCAASG-FTFS | S-----YNMN | WVRQAPGK GLEWVS | SISGS--- SSYIYYADS VKG | RFTISRDNAKNSLYLQMNSL RAEDTAVYYCAR | SL----------------DL | WGQGT LVTVS S |
| iPS:4 35537 | 21-225_157H12 | VH3\|3-21\|D6\|6-6\|RF2/JH4 | | EVQWVES-GGGLVKPGGSLRL SCAASG-FTFS | S-----YSMN | WVRQAPGK GLEWVS | SISGS--- GSYINYADS VKG | RFTISRDNAKTSLYLQVNGL RAEDTAVYYCAR | SXF----------------DS | WGQGT LVTVS S |
| iPS:4 35539 | 21-225_158G1 | VH3\|3-21\|D6\|6-6\|RF2/JH4 | | EVQLVES-GGGLVKPGGSLRL SCAASG-FTFS | S-----YGMN | WVRQAPGK GLEWIS | SISGS--- GSYIYYADS VKG | RFTISRDNAKNSLYLQMNSL RAEDTAVYYCAI | SSG----------------WS | WGQGT LVTVS S |
| iPS:4 35583 | 21-225_160F2 | VH3\|3-21\|D6\|6-6\|RF2/JH4 | | EVQLVES-GGGLVKPGGSLRL SCAASG-FTFS | S-----YGMN | WVRQAPGK GLEWIS | SISGS--- GSYIYYADS VKG | RFTISRDNAKNSLYLQMNSL RAEDTAVYYCAI | SSG----------------WS | WGQGT LVTVS S |
| | **Germline** | | | **H_FR1** | **H_CDR1** | **H_FR2** | **H_CDR2** | **H_FR3** | **H_CDR3** | **H_FR4** |
| | VH3\|3-21/D1\|1-1\|RF2/JH5 | | 32232 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | VQLER----------------NWFDP | WGQGT LVTVS S |
| iPS:4 68846 | 21-225_53B10 | VH3\|3-21\|D1\|1-1\|RF2/JH5 | | EVQLVES-GGGLVKPGGSLRL SCAASG-FTFS | S-----YSMN | WVRQAPGK GLEWVS | SISSS--- SSYIYYVDS VKG | RFTISRDNAKNSLYLQMNSL RAEDTAVYYCAR | VNS----------------FDS | WGQGT LVTVS S |
| iPS:4 34251 | 21-225_62G3 | VH3\|3-21\|D1\|1-1\|RF2/JH5 | | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | VNS----------------FDS | WGQGT LVTVS S |
| iPS:4 34407 | 21-225_68G8 | VH3\|3-21\|D1\|1-1\|RF2/JH5 | | EVQLVES-GGGLVKPGGSLRL SCAASG-FTFS | S-----YSMN | WVRQAPGK GLEWVS | SISGS--- SSYIYYADS VMG | RFTISRDNAKNSLYLQMNSL RAEDTAVYYCAR | VNS----------------FDS | WGQGT LVTVS S |
| iPS:4 35575 | 21-225_159H11 | VH3\|3-21\|D1\|1-1\|RF2/JH5 | | EVQLVES-GGGLVKPGGSLRL SCAASG-FTFS | S-----YTMN | WVRQAPGK GLEWVS | SISGS--- SSYIYYADS VKG | RFTISRDNAKNSLYLQMNSL RAEDTAVYYCAR | VSW----------------ADC | WGQGT LVTVS S |
| | **Germline** | | | **H_FR1** | **H_CDR1** | **H_FR2** | **H_CDR2** | **H_FR3** | **H_CDR3** | **H_FR4** |
| | VH3\|3-23/D1\|1-1\|RF2/JH4 | | 32233 | EVQLLES-GGGLVQPGGSLRL SCAASG-FTFS | S-----YAMS | WVRQAPGK GLEWVS | ATSGS--- GGSTYYADS VKG | RFTISRDNSKNTLYLQMNSL RAEDTAVYYCAK | VQLER------------------YFDY | WGQGT LVTVS S |

| iPS | Name | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 68848 | 21-225_54B1 | VH3\|3-23\|D1\|1-1\|RF2/JH4 | | EVQLLES-GGGLVQPGGSLRLSCAASG-FTFS | S-----YAMS | WVRQAPGKGLEWVS | VLSGS---GGSTFYADSVKG | RFTISRENSKNTLYLQMSSLRAEDTAVYYCAR | RGREYSG-----------------YDYFDY | WGQGTLVTVSS |
| iPS:4 33993 | 21-225_47G7 | VH3\|3-23\|D1\|1-1\|RF2/JH4 | | EVQLLDS-GGGLVQPGGSLRLSCAASG-FTFS | S-----YAMS | WVRQAPGKGLEWVS | AISGR---GGNTFYAESVRG | RFTISRDNSKNTLYLQMNSLRAEDTAVYYCAK | IIREQ----------------WAFDY | WGQGTLVTVSS |
| iPS:4 34007 | 21-225_48D7 | VH3\|3-23\|D1\|1-1\|RF2/JH4 | | EVQLLES-GGGLVQPGGSLRLSCAASG-FTFR | N-----SAMN | WVRQAPGKGLEWVS | AISGS---GGTTFYADSVKG | RFTISRDNSKNTLYLQINSLRAEDTAVYYCAK | CCREQ----------------WLDY | WGQGTLVTVSS |
| iPS:4 34115 | 21-225_53E4 | VH3\|3-23\|D1\|1-1\|RF2/JH4 | | EVQLLES-GGGLVQPGGSLRLSCAASG-FTFS | S-----YVMS | WVRQAPGKGLEWVS | GISGS---GGRTYYADSVKG | RFNISRDNSKNTLYLQMNSLRAEDTAVYYCAR | VAL----------------FDY | WGQGTLVTVSS |
| iPS:4 35679 | 21-225_169D10 | VH3\|3-23\|D1\|1-1\|RF2/JH4 | | EVQLLES-GGGLVQSGGSLRLSCAASG-FTFS | S-----YVMS | WVRQAPGKGLEWVS | AISGS---GSRIYYADSVKG | RFTISRDNSKNTLYLQMNSLRAEDTALYYCAR | VAF----------------FDY | WGQGTLVTVSS |
| iPS:4 35685 | 21-225_170E1 | VH3\|3-23\|D1\|1-1\|RF2/JH4 | | EVQLLES-GGGLVQPGGSLRLSCAASG-FTFS | S-----YVMS | WVRQAPGKGLEWVS | AISGS---GNRIYYADSVKG | RFTISRDNSKNTLYLQMNSLRAEDTALYYCAR | VAF----------------FDY | WGQGTLVTVSS |
| iPS:4 36632 | 21-225_227E4 | VH3\|3-23\|D1\|1-1\|RF2/JH4 | | EGQLLES-GGGLVQPGGSLRLSCTASG-FTFS | T-----FAMT | WVRQAPGRGLEWVS | VISGR---GGSSFYADSVKG | RFTISRDNTKNTLYLQMNSLRAEDTAVYYCAK | DQLW----------------FDY | WGQGTLVTVSS |
| | Germline | | | H_FR1 | H_CDR1 | H_FR2 | H_CDR2 | H_FR3 | H_CDR3 | H_FR4 |
| | VH4\|4-39\|D4\|4-11\|RF2/JH5 | | 32234 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | DYSNY----------------NWFDP | WGQGTLVTVSS |
| iPS:4 68856 | 21-225_77C9 | VH4\|4-39\|D4\|4-11\|RF2/JH5 | | QLQLQES-GPGLVTPSETLSLTCTVSG-GSIS | RS---SYYWG | WIRQPPGKGLEWIG | SIYY----SGSAYSNPSLKS | RVTISVDTSKNQFSLKLSSVTAADTALFYCAR | LDSNW----------------GLDY | WGQGTLVTVSS |
| iPS:4 34489 | 21-225_74E4 | VH4\|4-39\|D4\|4-11\|RF2/JH5 | | QLQLQES-GPGLVKPSETLSLICTVSG-GSIS | SS---NYYWG | WIRQPPGKGLEWIG | SIYY----SGYTSYNPSLKS | RVTISVDSSKNHFSLRLSSVTAADTAVYYCAR | LDSNW----------------GLDY | WGQGTLVTVSS |
| iPS:4 35251 | 21-225_96A3 | VH4\|4-39\|D4\|4-11\|RF2/JH5 | | QLQLQES-GPGLVKPSETLSLICTVSG-GSIS | SS---NYYWG | WIRQPPGKGLEWIG | SIYY----SGYTSYNPSLKS | RVTISVDSSKNHFSLRLSSVTAADTAVYYCAR | LDSNW----------------GLDY | WGQGTLVTVSS |
| iPS:4 37346 | 21-225_75H7 | VH4\|4-39\|D4\|4-11\|RF2/JH5 | | QLQLQES-GPGLVTPSETLSLTCTVSG-GSIS | RS---SYYWG | WIRQPPGKGLEWIG | SIYY----SGSAYSNPSLKS | RVTISVDTSKNQFSLKLSSVTAADTALFYCAR | LDSNW----------------GLDY | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 93886 | 21-225_2G9 | VH4\|4-39/D4\|4-11\|RF2/JH5 | | QLQLQES-GPGLVKPSETLSL TCTVSG-GSIR | PN----YYWG | WIRQPPGK GLEWIG | SIYY---- SGSTSYNPS LNS | RVTISVDISKNQFSLKLNSV TAADTAVYYCAR | LSSNW---------- ----------DFDN | WGQGT LVTVS S |
| iPS:3 93928 | 21-225_4E10 | VH4\|4-39/D4\|4-11\|RF2/JH5 | | QLQLQES-GPGLVKPSETLSL TCTVSG-GSIS | RS---SYYWG | WIRQPPGK GLEWIG | SVYY---- SGATSYNPS LKS | RVTISVDISKNQFSLKLNSV TAADTALYYCVR | LSSNW---------- ----------DFDY | WGQGT LFTVS S |
| | | **Germline** | | **H_FR1** | **H_CDR1** | **H_FR2** | **H_CDR2** | **H_FR3** | **H_CDR3** | **H_FR4** |
| | VH1\|1-02/D6\|6-6\|RF1/JH6 | | 32235 | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | EYSSSSYY--------YYYGMDV | WGQGT TVTVS S |
| iPS:4 68862 | 21-225_178H8 | VH1\|1-02/D6\|6-6\|RF1/JH6 | | QVQLVQS-GAEVRTPGASVKV SCKASG-YTFT | D-----YYMH | WVRQAPGQ GLEWMG | WINPN--- RGGTNYAQK FQG | RVTMTRDTSISTAYMELSRL RSDDTAVYYCAR | EEDRSGWY-------- -------YYYGMDV | WGQGT TVTVS S |
| iPS:4 51112 | 21-225_53D10 | VH1\|1-02/D6\|6-6\|RF1/JH6 | | QVQLVQS-GAEVKKPGASVKV SCKASG-YIFT | G-----YYIH | WVRQAPGQ GLEWMG | WINPN--- SGGTNYAQK FQG | RVTMTRDTSISTAYMELIRL RSDDTAVYYCAR | ENESLATRP------- -----FYDYYGMDV | WGQGT TVTVS S |
| | | **Germline** | | **H_FR1** | **H_CDR1** | **H_FR2** | **H_CDR2** | **H_FR3** | **H_CDR3** | **H_FR4** |
| | VH2\|2-05/D6\|6-6\|RF2/JH4 | | 32236 | QITLKES-GPTLVKPTQTLTL TCTFSG-FSLS | TS---GVGVG | WIRQPPGK ALEWLA | LIYW---- NDDKRYSPS LKS | RLTITKDTSKNQVVLTMTNM DPVDTATYYCAH | SIAAR----------- ----------YFDY | WGQGT LVTVS S |
| iPS:4 68864 | 21-225_60D6 | VH2\|2-05/D6\|6-6\|RF2/JH4 | | QTTLKES-GPTLVKPTQTLTL TCTFSG-FSLS | TS---GVGVG | WIRQPPGK ALEWLA | LIYW---- KDDERYSPS LKS | RLTITKDTSKNQVVLTMTNM DPVDTATYYCAH | AVAV------------ ----------SFDY | WGQGT LVTVS S |
| iPS:4 36850 | 21-225_57D9 | VH2\|2-05/D6\|6-6\|RF2/JH4 | | QITLKES-GPMLVKPTQTLTL TCTFSG-FSLS | TS---GVGVG | WIRQPPGK ALEWLA | LIYW---- NDDKRYSPS LKS | RLTITEDTSKNQVVLTMTNM DPVDTATYYCAH | AVAV------------ ----------SFDY | WGQGT LVTVS S |
| iPS:4 36914 | 21-225_76B4 | VH2\|2-05/D6\|6-6\|RF2/JH4 | | QITLKES-GPTLVKPTQTLTL TCTFSG-FSLS | TG---GVGVG | WIRQPPGK ALEWLA | LIYW---- DDDKRYSPS LKS | RLTITKDTPKNQVVLTMTNM DPVDTATYYCAH | LIAV------------ ----------AFDY | WGQGT LVTVS S |
| iPS:4 36918 | 21-225_77A2 | VH2\|2-05/D6\|6-6\|RF2/JH4 | | QITLKES-GPSLVKPTQTLTL TCTFSG-FSLS | TS---GVGVG | WIRQPPGK ALEWLV | FIYW---- DDDKRYSPS LKS | RLTITKDTSKNQVVLTMTNM DPVDTATYYCAH | LIAV------------ ----------AFDY | WGQGT LVTVS S |
| iPS:4 36934 | 21-225_96B5 | VH2\|2-05/D6\|6-6\|RF2/JH4 | | QITLKES-GPTLVKPTQTLTL TCTFSG-FSLS | TG---GVGVG | WIRQPPGK ALEWLA | LIYW---- DDDKRYSPS LKS | RLTITKDTPKNQVVLTMTNM DPVDTATYYCAH | LIAV------------ ----------ACDY | WGQGT LVTVS S |
| iPS:4 37334 | 21-225_75F11 | VH2\|2-05/D6\|6-6\|RF2/JH4 | | QITLKES-GPTLVKPTQTLTL TCTFSG-FSLS | TG---GVGVG | WIRQPPGK ALEWLA | LIYW---- DDDKRYSPS LKS | RLTITKDTPKNQVVLTMTNM DPVDTATYYCAH | LIAV------------ ----------AFDY | WGQGT LVTVS S |

| iPS | Name | Germline | No. | H_FR1 | H_CDR1 | H_FR2 | H_CDR2 | H_FR3 | H_CDR3 | H_FR4 |
|---|---|---|---|---|---|---|---|---|---|---|
| iPS:4 37377 | 21-225_74G9 | VH2\|2-05/D6\|6-6\|RF2/JH4 | | QITLKES-GPTLVKPTQILTL TCTFSG-FSLS | TG---GVGVG | WIRQPPGK ALEWLA | LIYW---- DDDKRYSPS LKS | RLTITKDIPKNQVVLTMTNM DPVDTATYYCAH | LIAV------------ ----------AFDY | WGQGT LVTVS S |
| iPS:3 92583 | 21-225_10B10 | VH2\|2-05/D6\|6-6\|RF2/JH4 | | QITLKES-GPTLVKPTQILTL TCTFSG-FSLS | TG---GVGVG | WIRQPPGK ALEWLV | FIYW---- SDDKRYSPS LKS | RLSITKDISKNQVVLTMTNM DPVDTATYYCAR | IAAV------------ ----------AFDY | WGQGT LVTVS S |
| iPS:3 93184 | 21-225_15H11 | VH2\|2-05/D6\|6-6\|RF2/JH4 | | QITLKES-GLTLMKPTQTLTL TCTFSG-FSLS | TG---GVGVG | WIRQPPGK ALEWLA | LIYW---- HDDKRYSPS LRS | RLTITKDTSKNQVVLTMTNM DPVDTATYYCAR | IVAV------------ ----------AFDY | WGQGT LITVS S |
| iPS:3 93212 | 21-225_30H6 | VH2\|2-05/D6\|6-6\|RF2/JH4 | | QITLKES-GPTLVKPTQTLTL TCTFSG-FSLS | TG---GVGVG | WIRQPPGK ALEWLA | LIYW---- HDDKRYSPS LKS | RLAITKDTSKNQVVLTITNM DPVDTATYYCAH | LIAV------------ ----------AFDY | WGQGT LVTVS S |
| iPS:3 93222 | 21-225_17F5 | VH2\|2-05/D6\|6-6\|RF2/JH4 | | QITLKES-GPSLVKPTQTLTL TCTFSG-FSLS | TS---GVGVG | WIRQPPGK ALEWLA | LIYW---- DDDKRYSPS LKS | RLTITKDTSKNQVVLTMTNM DPVDTATYSCAH | IIAV------------ ----------AFDY | WGQGT LVTVS S |
| iPS:3 93224 | 21-225_31C2 | VH2\|2-05/D6\|6-6\|RF2/JH4 | | QITLKES-GPTLVKPTQTLTL TCTFSG-FSLN | TG---GVGVG | WIRQPPGK ALEWLA | LIYW---- NDDERYSPS LKS | RLTITKDTSKNQVVLTMTNM DPLDTASYYCAH | LIAV------------ ----------SFDY | WGQGA LVTVS S |
| | | Germline | | H_FR1 | H_CDR1 | H_FR2 | H_CDR2 | H_FR3 | H_CDR3 | H_FR4 |
| | VH1\|1-02/D6\|6-19\|RF2/JH6 | | 32237 | QVQLVQS-GAEVKKPGASVKV SCKASG-YTFT | G-----YYMH | WVRQAPGQ GLEWMG | WINPN---- SGGTNYAQK FQG | RVTMTRDTSISTAYMELSRL RSDDTAVYYCAR | GIAVAGYY-------- ---------YYYGMDV | WGQGT TVTVS S |
| iPS:4 68866 | 21-225_190C1 | VH1\|1-02/D6\|6-19\|RF2/JH6 | | QVQLVQS-GAEVKKPGASVKV SCKASG-YTFT | G-----YYMH | WVRQAPGQ GLEWMG | WINPY--- SGGTNYAQK FQG | RVTMTRDTSISTAYMELSRL RSDDTAVYYCAR | DRAVAGNY-------- -------FYYGMDV | WGQGT TVTVS S |
| iPS:4 36972 | 21-225_190C7 | VH1\|1-02/D6\|6-19\|RF2/JH6 | | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | DRAVAGNY-------- -------FYYGMDV | WGQGT TVTVS S |
| iPS:4 37020 | 21-225_193F11 | VH1\|1-02/D6\|6-19\|RF2/JH6 | | QVQLVQS-GAEVKKPGASVKV SCKASG-YTFT | G-----YYMH | WVRQAPGQ GLEWMG | WINPY--- SGGTNYAQK FQG | RVTMTRDTSISTAYMELSRL RSDDTAVYYCAR | DRAVAGNY-------- -------FYYGMDV | WGQGT TVTVS S |
| iPS:4 37036 | 21-225_195H9 | VH1\|1-02/D6\|6-19\|RF2/JH6 | | QVQLVQS-GAEVKKPGASVKV SCKASG-YTFT | G-----YYMH | WVRQAPGQ GLEWMG | WINPY--- SGGTNYAQK FQD | RVTMTRDTSITTAYMELSRL RSDDTAVYYCAR | DRAVAGNY-------- -------FYYGMDV | WGQGT TVTVS S |
| IPS:4 37042 | 21-225_197E8 | VH1\|1-02/D6\|6-19\|RF2/JH6 | | QVQLLQS-GAEVRKPGASVRV SCKASG-YTFT | G-----YYIH | WVRQAPGQ GLEWMG | WINPN--- SGGTNYAQR FQG | RVTMTRDTSISTAYMELSRL RSDDTAVYYCAR | EIAVAGNY-------- -------FYYGMGV | WGQGT TVAVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-39/D1\|1-1\|RF2/JH4 | | 32238 | QLQLQES-GPGLVKPSETLSL TCTVSG-GSIS | SS---SYYWG | WIRQPPGK GLEWIG | SIYY---- SGSTYYNPS LKS | RVTISVDTSKNQFSLKLSSV TAADTAVYYCAR | VQLER--------- ----------YFDY | WGQGT LVTVS S |
| iPS:4 68868 | 21-225_74A1 | VH4\|4-39/D1\|1-1\|RF2/JH4 | | QLQLQES-GPGLVKPSETLSL TCTVSG-GSIS | GS---SYYWG | WIRQPPGK GLEWIG | NIYY---- SGSTYHNPS LKS | RVTISVDTSKNQFSLKLTSV TAADTAVYYCAR | HDLLW------------ ---------SLDF | WGQGI LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH1\|1-02/D3\|3-22\|RF2/JH5 | | 32239 | QVQLVQS-GAEVKKPGASVKV SCKASG-YTFT | G------YYMH | WVRQAPGQ GLEWMG | WINPN--- SGGTNYAQK FQG | RVTMTRDTSISTAYMELSRL RSDDTAVYYCAR | YYYDSSGY----- --------YYNWFDP | WGQGT LVTVS S |
| iPS:4 72742 | 21-225_30D9_LC 2 | VH1\|1-02/D3\|3-22\|RF2/JH5 | | QVKLVQS-GAEVEKPGASVKV SCKASG-YTFT | G-----YYLH | WVRQAPGQ GLEWMG | WINPN--- SGGTNYAQK FQG | RVTMTRDTSISTAYMELSRL RSDDTAVYYCAR | VYYYGSGS-------- -------YYNEFDN | WGQGT LVTVS S |
| iPS:4 72741 | 21-225_30D9_LC 1 | VH1\|1-02/D3\|3-22\|RF2/JH5 | | QVKLVQS-GAEVEKPGASVKV SCKASG-YTFT | G-----YYLH | WVRQAPGQ GLEWMG | WINPN--- SGGTNYAQK FQG | RVTMTRDTSISTAYMELSRL RSDDTAVYYCAR | VYYYGSGS-------- -------YYNEFDN | WGQGT LVTVS S |
| iPS:4 37040 | 21-225_196E7 | VH1\|1-02/D3\|3-22\|RF2/JH5 | | QVQLVQS-GAEVKKPGASVKV SCKVSG-YTFT | G-----YNMH | WVRQAPGQ GLEWMG | WINPN--- SGGTNYAHK FQG | RVTMTRDTSISTAYMELSRL RSDDTAVYYCAR | DYYDTSG--------- --------EGWFDP | WGQGT LVTVS S |
| iPS:4 37050 | 21-225_197C11 | VH1\|1-02/D3\|3-22\|RF2/JH5 | | QVQLVQS-GAEVKKPGASVKV SCKVSG-YTFT | G-----YNMH | WVRQAPGQ GLEWMG | WINPN--- SGGTNYAHK FQG | RVTMTRDTSISTAYMELSRL RSDDTAVYYCAR | DYYDSSG--------- --------EGWFDP | WGQGT LVTVS S |
| iPS:3 93214 | 21-225_33A1 | VH1\|1-02/D3\|3-22\|RF2/JH5 | | QVQLVQS-GAEVKKPGASVKV SCKASG-YTFS | G-----YYMH | WVRQAPGQ GLEWMG | WINPN--- NGGTHYAQK FQG | RVTMTRDTSIRTASMELSRL RSDDTAVYFCAR | GYYYASGS-------- -------YYNDLDP | WGQGT LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH1\|1-02/D3\|3-22\|RF2/JH4 | | 32240 | QVQLVQS-GAEVKKPGASVKV SCKASG-YTFT | G------YYMH | WVRQAPGQ GLEWMG | WINPN--- SGGTNYAQK FQG | RVTMTRDTSISTAYMELSRL RSDDTAVYYCAR | YYYDSSG---------- ------YYYYFDY | WGQGT LVTVS S |
| iPS:4 72743 | 21-225_68G6 | VH1\|1-02/D3\|3-22\|RF2/JH4 | | QVQLVQF-GGEVKKPGSSVKV SCKASG-YTFT | G-----YYMH | WVRQAPGQ GLEWMG | SIYRN--- SGGTNYAQK FQG | RVTMTRDKSISTAYMEKSRI RSDDTAVYYCAR | AFYYGSGT-------- -------YYNEFDY | WGQGT LVTVS S |
| iPS:4 36902 | 21-225_69B11 | VH1\|1-02/D3\|3-22\|RF2/JH4 | | QVQLVQS-GAEVKKPGASVRV SCKASG-YTFT | G-----YYMH | WVRQAPGQ GLEWMG | WINPN--- SGGTNYGQK FQD | RVTMTRDTSISTAFMELSRL RSDDTAAYYCAR | TYYYGSGS-------- -------YYNGFDY | WGQGT LVTVS S |
| iPS:4 36904 | 21-225_71D4 | VH1\|1-02/D3\|3-22\|RF2/JH4 | | QVQLVQS-GAEVKKPGASVKV SCKASG-YTFT | G-----YCMH | WVRQAPGQ GLEWMG | WINPN--- SGGTNYAQK FQV | RVTMTRDTSVSTVYMDLSRL RSDDTAVYYCAR | AYYYGSGT-------- -------YHNEFDY | WGQGS LVTVS S |

| iPS ID | Clone | VH gene | | FR1 | CDR1 | FR2 | CDR2 | FR3 | CDR3 | FR4 |
|---|---|---|---|---|---|---|---|---|---|---|
| iPS:4 36906 | 21-225_72B4 | VH1\|1-02\|D3\|3-22\|RF2\|JH4 | | QVQLVQS-GAEVKKPGASVKVSCKASG-YTFT | G-----YYMH | WVRQAPGQGLEWMG | WINPN---SGGTNYGQKFQG | RVTMTRDISISTAYMELSRLRSDDTAVYYCAR | TYYYGSGS---------------YYNGFDY | WGQGTLVTVSS |
| iPS:4 37034 | 21-225_195E9 | VH1\|1-02\|D3\|3-22\|RF2\|JH4 | | QVQVVQS-GAEVKKPGASVKVSCKASG-YTFT | G-----YYMH | WVRQAPGQGLEWMG | WINPN---SGATNYAQKFQG | RVTMTRDISISTAYMELNRLRSDDTAVYYCAR | AYYYGSGT---------------YYNEFDY | WGQGTLVTVSS |
| iPS:3 92598 | 21-225_18E10 | VH1\|1-02\|D3\|3-22\|RF2\|JH4 | | QVQLVQS-GAEVKKPGASVKVSCKASG-YTFT | G-----YYMH | WVRQAPGQGLEWMG | WINPN---SGGTNYAQKFQG | RVTMTRDSSINTAYMELSRLRSDDTAVYYCAR | SYYYGSGS---------------YYNEFDY | WGQGTLVTVSS |
| iPS:3 93182 | 21-225_4B3 | VH1\|1-02\|D3\|3-22\|RF2\|JH4 | | QVQLVQS-GAEVKKPGASVKVSCKASG-YTFT | G-----YYMH | WVRQAPGQGLEWMG | WINPN---SGGTNSAQKFQG | RVTMTRDTSISTAYMELSRLRSDDTAVYYCAR | SYYYGSGS---------------YYNEFDY | WGQGTLVTVSS |
| iPS:3 93200 | 21-225_35E1 | VH1\|1-02\|D3\|3-22\|RF2\|JH4 | | QVKLVQS-GAEVKKPGASVKVSCKASG-YTFT | G-----YYMH | WVRQAPGQGLEWMG | WINPK---SGGTNYAQKFQG | RVTMTRDTSISTVYMEPSRLRSDDTAVYYCAR | VYYHGSGS---------------YYNEFDY | WGQGTLVTVSS |
| iPS:3 93206 | 21-225_13F6 | VH1\|1-02\|D3\|3-22\|RF2\|JH4 | | QVQLVQS-GAEVKKPGASVKVSCKASG-YTFT | G-----YYMH | WVRQAPGQGLEWMG | WINPN---SGGANYAQKFQG | RVTMTRDTSISTAYMELSRLRSDDTAVYFCAR | SFYYGSGT---------------YYNEFDY | WGQGTLVTVSS |
| iPS:3 93208 | 21-225_16F3 | VH1\|1-02\|D3\|3-22\|RF2\|JH4 | | QVQLVQS-GAEVKKPGASVKVSCKASG-YTFT | G-----HYMH | WVRQAPGQGLEWMG | WINPN---SGGTNYAQKFQG | RVTMTRDTSISTAYMELSRLRSDDTAVYYCAR | SYYYGSGS---------------YYNEFDY | WGQGTLVTVSS |
| iPS:3 93210 | 21-225_17D3 | VH1\|1-02\|D3\|3-22\|RF2\|JH4 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | ANYYGSGS---------------YYNDFDY | WGQGTLVTVSS |
| iPS:3 93226 | 21-225_33E6 | VH1\|1-02\|D3\|3-22\|RF2\|JH4 | | QVKLVQS-GAEVKKPGASVKVSCKASG-YTFT | G------YYMH | WVRQAPGQGLEWMG | WINPN---SGGTNYAQKFQG | RVTMTRDTSISTAYMELSRLRSDDTAVYYCAR | VYYYGSGS---------------YYNEFDY | WGQGTLVTVSS |
| iPS:3 93230 | 21-225_9G9 | VH1\|1-02\|D3\|3-22\|RF2\|JH4 | | QVQLVQS-GAEVKKPGASVKVSCKASG-YTFT | G-----YYIH | WVRQAPGQGLEWMG | WINPN---SGGTDYAQKFQG | RVTMTRDTSISTAYMELSRLRSDDTAVYCAR | SYYYGSGT---------------YYNEFDY | WGQGTLVTVSS |
| iPS:3 98490 | 21-225_21D12 | VH1\|1-02\|D3\|3-22\|RF2\|JH4 | | QVQLVQS-GAEVKKPGASVKVSCKASG-YTFT | D-----YYIH | WVRQAPGQGLEWMG | WINPN---SGGTNNAQKFQG | RVTMTRDTSISTAYMELSRLRSDDTAVYSCAR | SYYYGSGT---------------YYNEFDY | WGQGTLVTVSS |
| iPS:4 23018 | 21-225_31D12_LC2 | VH1\|1-02\|D3\|3-22\|RF2\|JH4 | | QVKLVQS-GAEVKKPGASVKVSCKASG-YTFT | G-----YYMH | WVRQAPGQGLEWMG | WINPN---SGGTNYVQKFQG | RVTMTRDTSISTAYMELSRLRSDDTAVYYCAR | VYYYGSGS---------------YYNEFDY | WGQGTLVTVSS |

| iPS:4 23019 | 21-225_31D12_LC1 | VH1\|1-02/D3\|3-22\|RF2\|JH4 | | QVKLVQS-GAEVKKPGASVKVSCKASG-YTFT | G-----YYMH | WVRQAPGQGLEWMG | WINPN---SGGTNYVQKFQG | RVTMTRDISISTAYMELSRLRSDDTAVYYCAR | VYYYGSGS--------------YYNEFDY | WGQGTLVTVS |
|---|---|---|---|---|---|---|---|---|---|---|
| | | Germline | | H_FR1 | H_CDR1 | H_FR2 | H_CDR2 | H_FR3 | H_CDR3 | H_FR4 |
| | VH3\|3-33/D7\|7-27\|RF2\|JH4 | | 32241 & 32242 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LGY-------------FDY | WGQGTLVTVS |
| iPS:3 92920 | 21-225_29G4 | VH3\|3-33/D7\|7-27\|RF2\|JH4 | | QVQLVES-GGGVVQPGRSLRLTCAASG-FTFS | D-----YGIH | WVRQAPGKGLEWVA | VIWYD---ESNKYYADSMKG | RFTISRDNSKNTLYLQMNSLRAEDTAVYYCAR | ELGM-----------TGDY | WGQGTLVTVS |
| iPS:4 33899 | 21-225_43C3 | VH3\|3-33/D7\|7-27\|RF2\|JH4 | | QVQLVES-GGGVVQPGRSLRLSCAASG-FTFS | S-----YGIH | WVRQAPGKGLEWVA | VIWYD---ENNKYYADSVKG | RFTISRDNSKNTLYLQMNSLRAEDTAVYYCAR | ELGF-----------SNDY | WGQGTLVTVS |
| iPS:4 33921 | 21-225_44C3 | VH3\|3-33/D7\|7-27\|RF2\|JH4 | | QVQLVES-GGGVVQPGRSLRLSCAASG-FTFS | S-----YGMH | WVRQAPGKGLEWVA | VIWFE---GSNKYYADSVKG | RFTISRDNSKNTLYLQMSSLRAEDTAVYYCVR | ELGF-----------STDY | WGQGTLVTVS |
| iPS:4 33933 | 21-225_44C8 | VH3\|3-33/D7\|7-27\|RF2\|JH4 | | QVQLVES-GGGVVQPGRSLRLSCAASG-FTFN | N-----YGMH | WVRQAPGKGLEWVA | VIWYE---GSNKYYADSVKG | RFTISRDNSKNTLYLQMNSLRAEDTAVYYCVR | ELGF-----------LSDY | WGQGTLVTVS |
| iPS:4 33969 | 21-225_46F3 | VH3\|3-33/D7\|7-27\|RF2\|JH4 | | QVQLVES-GGGVVQPGRSLRLSCAASG-FTFS | D-----YGMH | WVRQAPGKGLEWVA | VIWFE---GSNKYYADSVKG | RFTISRDNSKNTLYVQMNSLRAEDTAVYYCVR | ELGF-----------SNDY | WGQGTLVTVS |
| iPS:4 33975 | 21-225_46C6 | VH3\|3-33/D7\|7-27\|RF2\|JH4 | | QVQLVES-GGGVVQPGRSLRLSCAASG-FTFS | S-----YGIH | WVRQAPGKGLEWVA | VIWYD---ENNKYYADSVKG | RFTISRDNSKNTLYLQMNSLRAEDTAVYYCAR | ELGF-----------SNDY | WGQGTLVTVS |
| iPS:4 33977 | 21-225_46D8 | VH3\|3-33/D7\|7-27\|RF2\|JH4 | | QVQLVES-GGGVVQPGRSLRLSCAASG-FTFS | D-----YGIH | WVRQAPGKGLEWVA | VIWFE---GSNKYYADSVKG | RFTISRDNSKNTLYVQMNSLRAEDTAVYYCVR | ELGF-----------SNDY | WGQGTLVTVS |
| iPS:4 33983 | 21-225_47A1 | VH3\|3-33/D7\|7-27\|RF2\|JH4 | | QVQLVES-GGGVVQPGRSLRLSCAASG-FTFS | D-----YGMH | WVRQAPGKGLEWVA | VIWYD---DYNKKYYADSVKG | RFTISRDNAKNTLYLQVNSLRVEDTAVYYCAT | ELGM-----------LFDY | WGQGTLVTVS |
| iPS:4 33997 | 21-225_48C1 | VH3\|3-33/D7\|7-27\|RF2\|JH4 | | QVQLVES-GGGVVQPGRSLRLSCAASG-FTFS | S-----YGMH | WVRQAPGKGLEWVA | VVWYD---EINKKYYADSVKG | RVTISRDNSKNTLYLQMNSLRAEDTAMYYCAR | ELGW-----------EADY | WGQGTLVTVS |
| iPS:4 34009 | 21-225_48A9 | VH3\|3-33/D7\|7-27\|RF2\|JH4 | | QVQLVES-GGGVVQPGRSLRLSCAASG-FTFS | S-----YGMH | WVRQAPGKGLEWVA | VIWYE---ENKKYYADSVKG | RFTISRDNSKNTLFLQMNSLRAEDTAMYFCAR | ELAW-----------YEDY | WGQGTLVTVS |

| | | | | | | | | | |
|---|---|---|---|---|---|---|---|---|---|
| iPS:4 34013 | 21-225_48D12 | VH3\|3-33\|D7\|7-27\|RF2\|JH4 | | QVQLVES-GGGVVQPGRSLRLSCAASG-FTFS | D-----YGMH | WVRQAPGRGLEWVA | VIWYD---VSNKYYVDSVKG | RFTISRDNSKNTLYLQMNSLRAEDTAVYYCAR | ELGM------------------------RSDY | WGQGTLVTVSS |
| iPS:4 34019 | 21-225_49A1 | VH3\|3-33\|D7\|7-27\|RF2\|JH4 | | QVQLVES-GGGVVQPGRSLRLSCAASG-FTFS | D-----YGMH | WVRQAPGKGLEWVA | VIWYD---EDNXYYVDSVKG | RFTISRDNSKNTLYLQMNSLRAEDTAVYYCAR | ELGF------------------------LSDY | WGQGTLVTVSS |
| iPS:4 34029 | 21-225_49C6 | VH3\|3-33\|D7\|7-27\|RF2\|JH4 | | QVQLVES-GGGVVQPGRSLRLSCAASG-FTFS | N-----YGMH | WVRQAPGKGLEWVA | VIWFD---VSNKKYVDSVKG | RFTISRDNSKNTLYLQMSSLRAEDTAVYYCAR | DLGM------------------------IEDY | WGQGTLVTVSS |
| iPS:4 34057 | 21-225_51E4 | VH3\|3-33\|D7\|7-27\|RF2\|JH4 | | QVQLVES-GGGVVQPGRSLRLSCAASG-FTFS | S-----YGMH | WVRQAPGKGLEWVA | VIWYD---ESNKYYADSVKG | RFTISRDNSKNTLYLQMNSLRAEDTAVYYCAR | ELGF------------------------LSDY | WGQGTLVTVSS |
| iPS:4 34071 | 21-225_51F9 | VH3\|3-33\|D7\|7-27\|RF2\|JH4 | | QVQLVES-GGGVVQPGRSLRLSCAASG-FTFS | D-----YGMH | WVRQAPGKGLEWVA | VIWFD---GNNKYYADSVKG | RFTISRDNSKNTLYLQMNSLSAEDTAVYYCAR | ELGF------------------------LSDY | WGQGTLVTVSS |
| iPS:4 34075 | 21-225_51B11 | VH3\|3-33\|D7\|7-27\|RF2\|JH4 | | QVQLVES-GGGVVQPGRSLRLSCAASG-FTFS | D-----YGMH | WVRQAPGKGLEWVA | VIWFG---GNNKYYGDSVKG | RFTISRDNSKNTLYLQMNSLSAEDTAVYYCAR | ELGF------------------------LSDY | WGQGTLVTVSS |
| iPS:4 34077 | 21-225_51F11 | VH3\|3-33\|D7\|7-27\|RF2\|JH4 | | QVQLVES-GGGVVQPGRSLRLSCAASG-FTFS | N-----FGMH | WVRQAPGKGLEWVA | VIWYE---ESNKYYADSVKG | RFTISRDNSKNTLYLQMNSLRAEDTAVYYCAR | ELGF------------------------LSDF | WGQGTLVTVSS |
| iPS:4 34081 | 21-225_52B2 | VH3\|3-33\|D7\|7-27\|RF2\|JH4 | | QVQLVES-GGGVVQPGRSLRLSCAASG-FTFS | N-----YGMH | WVRQAPGKGLEWVA | VTWFD---GSNQRYADSVKG | RFTISRDISKNTLYLQMNSLSAEDTAVYYCAR | DLGM------------------------IEDF | WGQGTLVTVSS |
| iPS:4 34091 | 21-225_52B9 | VH3\|3-33\|D7\|7-27\|RF2\|JH4 | | QVQLVES-GGGVVQPGRSLRLSCAASG-FTFS | D-----YGMH | WVRQAPGKGLEWVA | VIWFD---GNNKYYADSVKG | RFTISRDNSKNTLYLQMNSLSAEDTAVYYCAR | ELGF------------------------LSDY | WGQGTLVTVSS |
| iPS:4 34105 | 21-225_53D2 | VH3\|3-33\|D7\|7-27\|RF2\|JH4 | | QVQLVES-GGGVVQPGRSLRLSCAASG-FTFS | N-----YGMH | WVRQAPGKGLEWVT | VVWDD---GSNKYYADSVKG | RFTISRDNSKNTLYLQMNSLRAEDTAVYYCTR | GLGF------------------------TGDY | WGQGALVTVSS |
| iPS:4 34119 | 21-225_53E6 | VH3\|3-33\|D7\|7-27\|RF2\|JH4 | | QVQLVES-GGGVVQPGRSLRLSCTTSG-FTFS | D-----YGIH | WVRQAPGKGLEWVA | VIWYD---ESNKYYGDSVKG | RFAISRDNSKNTLYLQMNSLRAEDTAVYYCVR | ELGM------------------------TSDY | WGQGTLVTVSS |
| iPS:4 34129 | 21-225_53B12 | VH3\|3-33\|D7\|7-27\|RF2\|JH4 | | QVQLVES-GGGVVQPGRSLRLSCAASG-FTFS | N-----FGMH | WVRQAPGKGLEWVA | VVWYD---GNNRYYADSVKG | RFTISRDNSKNTLYLQMHSLRAEDTAVYYCAR | ELGF------------------------LSDF | WGQGTLVTVSS |

| iPS:4 34131 | 21-225_54D3 | VH3\|3-33\|D7\|7-27\|RF2\|JH4 | | QVQLVES-GGGVVQPGRSLRL SCAASG-FTFS | N-----YGMH | WVRQAPGK GLEWVA | VTWFD---GNNNYYADS VKG | RFTISRDNSKNTLYLQMNSL RAEDTAVYYCAR | ELGF------------ ----------LSDY | WGQGT LVTVS S |
| iPS:4 34141 | 21-225_54C6 | VH3\|3-33\|D7\|7-27\|RF2\|JH4 | | QVQLVES-GGGVVQPGRSLRL SCTTSG-FTFS | D-----YGIH | WVRQAPGK GLDWVA | VIWYD---ENNKYYADS VKG | RFAISRDNSKNTLYLQMNSL RAEDTAVYYCVR | ELGM------------ ----------TSDY | WGQGT LVTVS S |
| iPS:4 34143 | 21-225_54G7 | VH3\|3-33\|D7\|7-27\|RF2\|JH4 | | QVQLVES-GGGVVQPGRSLRL SCAASG-FTFS | N-----YGMH | WVRQAPGK GLEWVA | VIWYE---ESNKYYGDS VKG | RFTISRDNSKNTLYLQMNSL RAEDTAVYYCAR | ELGF------------ ----------LSDY | WGQGT LVTVS S |
| iPS:4 34155 | 21-225_55B3 | VH3\|3-33\|D7\|7-27\|RF2\|JH4 | | QVQLVES-GGGVVQPGRSLRL SCAASG-FTFS | S-----YGMH | WVRQAPGK GLEWVA | VIWFD---GNNKYYEDS VKG | RFTISRDNSKNTLYLQMNSL RAEDTAVYYCVR | ELGF------------ ----------LSDY | WGQGT LVTVS S |
| iPS:4 34199 | 21-225_59F11 | VH3\|3-33\|D7\|7-27\|RF2\|JH4 | | QVQLVES-GGGVVQPGRSLRL SCAASG-FTFS | N-----YGMH | WVRQAPGK GLEWVA | VIWYD---ESNKHYADS VKG | RFTISRDNSKTTLYLQMSSL RAEDTAVYYCSR | ELGM------------ ----------NGDY | WGQGT LVTVS S |
| iPS:4 34207 | 21-225_60A3 | VH3\|3-33\|D7\|7-27\|RF2\|JH4 | | QVQLVES-GGGVVQPGRSLRL SCAASG-FTFS | S-----YGMH | WVRQAPGK GLEWVA | VIWYE---ESNKYYADS VKG | RFTISRDNSKNTLYLQMNSL RAEDTAVYYCAR | ELGM------------ ----------TGDY | WGQGT LVTVS S |
| iPS:4 34253 | 21-225_62E4 | VH3\|3-33\|D7\|7-27\|RF2\|JH4 | | QVQLVES-GGGVVQPGRSLRL SCAASG-FTFS | D-----YGMH | WVRQAPGK GLEWVA | VIWYD---RSNKYYADS VKG | RFTISRDNSKNTLYLQMNSL RVEDTAVYHCAR | ELGF------------ ----------SSDY | WGQGT LVTVS S |
| iPS:4 34271 | 21-225_57A4 | VH3\|3-33\|D7\|7-27\|RF2\|JH4 | | QVQLVES-GGGVVQPGRSLRL SCVASG-FTFS | D-----YGMH | WVRQAPGK GLEWVA | VIWYA---GSNKYYVDS VKG | RFTISRDNSKNTLYLQMNSL RAEDTAVYYCAR | ELGM------------ ----------RSDY | WGQGT LVTVS S |
| iPS:4 34293 | 21-225_58F5 | VH3\|3-33\|D7\|7-27\|RF2\|JH4 | | QVQLVES-GGGVVQPGRSLRL SCVASG-FTFS | D-----YGMH | WVRQAPGK GLEWVA | VIWYA---GSNKYHVDS VKG | RFTISRDNSKNTLYLQMNSL RAEDTAVYYCAR | ELGM------------ ----------RSDY | WGQGT LVTVS S |
| iPS:4 34337 | 21-225_64E1 | VH3\|3-33\|D7\|7-27\|RF2\|JH4 | | QVQLVES-GGGVVQPGRSLRL SCVASG-FTFS | S-----YGMH | WVRQAPGK GLEWVA | VIWFD---ETNKYYGDS VKG | RFTISRDNSKNTLYLQMNSL RAEDTAVYYCAR | ELGF------------ ----------SSDY | WGQGT LVTVS S |
| iPS:4 34357 | 21-225_65C1 | VH3\|3-33\|D7\|7-27\|RF2\|JH4 | | QVQLVES-GGGVVQPGRSLRL SCAASG-FTFS | D-----YGMH | WVRQAPGK GLEWVA | VIWFE---GSNKHYTDS VKG | RFTISRDNSKNTLYLQMNSL RAEDTAVYYCAR | ELGF------------ ----------SSDY | WGQGT LVTVS S |
| iPS:4 34375 | 21-225_66C7 | VH3\|3-33\|D7\|7-27\|RF2\|JH4 | | QVQLVES-GGGVVQPGRSLRL SCVASG-FTFS | D-----YGMH | WVRQAPGK GLEWVA | VIWFE---GSHKYYTDS VKG | RFTISRDNSKNTLYLQMNSL RAEDTAVYYCAR | ELGF------------ ----------SSDY | WGQGT LVTVS S |

| iPS:4 34411 | 21-225_68F11 | VH3\|3-33\|D7\|7-27\|RF2\|JH4 | | QVQLVES-GGGVVQPGRSLRLSCAASG-FTFS | D-----YGMH | WVRQAPGKGLEWVA | VIWYD---VSNKYYADSVKG | RFTISRDNSKNTLYLQMNSLRAEDTAVYYCVR | ELGM----------------------TSDC | WGQGTLVTVSS |
| iPS:4 34441 | 21-225_71A2 | VH3\|3-33\|D7\|7-27\|RF2\|JH4 | | QVQLVES-GGGVVQPGRSLRLSCATSG-FTFS | D-----YGMH | WVRQAPGKGLEWVA | VIWYD---ESNKYYADSVKG | RFTISRDNSKNTLYLQMNSLRAEDTAVYYCAR | ELGW----------------------QDDY | WGQGTLVTVSS |
| iPS:4 34447 | 21-225_71B6 | VH3\|3-33\|D7\|7-27\|RF2\|JH4 | | QVQLVES-GGGVVQPGRSLRLSCAASG-FTFS | N-----YGMH | WVRQAPGKGLEWVA | VIWYD---RTNKYYADSVKG | RFTISRDNSKNTLHLQMNSLRAEDTAVYYCAR | ELGM----------------------LSDY | WGQGTLVTVSS |
| iPS:4 34453 | 21-225_71B11 | VH3\|3-33\|D7\|7-27\|RF2\|JH4 | | QVQLVES-GGGVVQPGRSLRLSCAASG-FTFS | D-----YGMH | WVRQAPGKGLEWVA | VIWYD---RNNKYYGDSVKG | RFTISRDNSKNTLYLQMNSLRAEDTAVYYCAR | ELGM----------------------LSDY | WGQGTLVTVSS |
| iPS:4 34457 | 21-225_72G12 | VH3\|3-33\|D7\|7-27\|RF2\|JH4 | | QVQLVES-GGGVVQPGRSLRLSCAASG-FTFS | S-----YGMH | WVRQAPDTGLEWVA | VIWFD---ESNKYYADSVKG | RFTISRDNSKNTLYLQMNSLRAEDTAVYYCAR | ELGF----------------------SSDY | WGQGTLVTVSS |
| iPS:4 35311 | 21-225_146H9 | VH3\|3-33\|D7\|7-27\|RF2\|JH4 | | QVQLVES-GGGVVQPGRSLRLSCAASG-FSFS | S-----YGMH | WVRQAPGKGLEWVA | VIWFD---ESNKHYGDSVKG | RFTISRDNSKNTLYLQMNSLRAEDTAVYYCAR | ELGF----------------------LSDY | WGQGTLVTVSS |
| iPS:4 35511 | 21-225_157C3 | VH3\|3-33\|D7\|7-27\|RF2\|JH4 | | QVQLVES-GGGVVQPGRSLRLSCAASG-FTFS | T-----YGMH | WVRQAPGKGLEWVA | VIWYD---VNNKYYADSVKG | RFTISRDNSKNTLYLQMNSLRAEDTAVYYCAR | ELGF----------------------LSDY | WGQGTLVTVSS |
| iPS:4 35533 | 21-225_157H8 | VH3\|3-33\|D7\|7-27\|RF2\|JH4 | | QVQLVES-GGGVVQPGRSLRLSCAASG-FTFS | S-----YGMH | WVRQAPGKGLEWVA | VIWYD---VNNKYYADSVKG | RFTISRDNSKNTLYLQMNSLRAEDTAVYYCAR | ELGF----------------------LSDY | WGQGTLVTVSS |
| iPS:4 35551 | 21-225_158H6 | VH3\|3-33\|D7\|7-27\|RF2\|JH4 | | QVQLVES-GGGVVQPGRSLRLSCAVSG-FTFS | S-----YGMH | WVRQAPGKGLEWVA | VIWYD---VTNKYYADSVKG | RFTISRDNSKNTLYLQMNSLRDEDTAVYYCVR | ELGW----------------------AEDY | WGQGTLVTVSS |
| iPS:4 35569 | 21-225_159C5 | VH3\|3-33\|D7\|7-27\|RF2\|JH4 | | QVQLVES-GGGVVQPGRSLRLSCAASG-FTFS | S-----YGIH | WVRQAPGKGLEWVA | VVWYD---VNNKYYADSVKG | RFTISRDNSKNTLYLQMNSLRAEDTAVYYCAR | ELGF----------------------LSDY | WGQGTLVTVSS |
| iPS:4 36268 | 21-225_203B9 | VH3\|3-33\|D7\|7-27\|RF2\|JH4 | | QVQLVES-GGGVVQPGRSLRLSCAASG-FTLS | S-----FGMH | WVRQAPGKGLEWVA | VIWYD---VNNKYYADSVKG | RFTISRDNSKNTLYLQMNSLRPEDTAVYYCAR | ELGF----------------------LSDY | WGQGILVTVSS |
| iPS:4 36328 | 21-225_207F12 | VH3\|3-33\|D7\|7-27\|RF2\|JH4 | | QVQLVES-GGGVVQPGRSLRLSCAASG-FTFS | N-----YGMH | WVRQAPGKGLEWVA | VIWYD---RNNKYYGDSVKG | RFTISRDNSKNTLYLQMNSLRAEDTAVYYCAR | ELGF----------------------LFDY | WGQGTLVTVSS |

| iPS:4 36556 | 21-225_224D10 | VH3\|3-33\|D7\|7-27\|RF2\|JH4 | | QVQLVES-GGGLVQPGKSLRLSCAASG-FTFS | S-----YGMH | WVRQAPGKGLEWVA | VIWYE---GSNKYYVDSVRG | RFTISRDNSKSTLYLQMNSLRAEDTAVYYCVR | ELGF----------------------QSDY | WGQGTPVTVSS |
|---|---|---|---|---|---|---|---|---|---|---|
| iPS:3 92618 | 21-225_16F10 | VH3\|3-33\|D7\|7-27\|RF2\|JH4 | | QVQLVES-GGGVVQPGRSLRLSCAASG-FTFS | D-----YGMH | WVRQAPGKGLEWVA | VIWYD---GNNKYYVDSVKG | RFTISRDNSKNTLYLQMNSLRAEDTAVYYCAR | ELAW----------------------YEDY | WGQGTLVTVSS |
| iPS:3 92626 | 21-225_18A5 | VH3\|3-33\|D7\|7-27\|RF2\|JH4 | | QVQLVES-GGGVVQPGRSLRLSYTASG-FTFS | D-----YGMH | WVRQAPGKGLEWVA | VIWFD---GSNKYYADSVKG | RFTISRDNSKNTLYLQMNSLRAEDTAVYYCAR | DLGW----------------------TEEY | WGQGTLVTVSS |
| iPS:3 92630 | 21-225_20E5 | VH3\|3-33\|D7\|7-27\|RF2\|JH4 | | QVQLVES-GGGVVQPGRSLRLSCAASG-FTFS | D-----YGMH | WVRQAPGKGLEWVA | VIWYE---ENNQYYADSVKG | RFTISRDNSKNTLYLQMNSLRAEDTAVYYCAR | ELGF----------------------RSDY | WGQGTLVTVSS |
| iPS:3 92640 | 21-225_18A1 | VH3\|3-33\|D7\|7-27\|RF2\|JH4 | | QVQLVES-GGGVVQPGRSLRLSCAVSG-FTFS | S-----YGMH | WVRQAPGKGLEWVA | VIWYE---ENNKYYVDSVKG | RFTISRDNSKSTLYLQMNSLRAEDTAVYYCVR | ELGF----------------------QSDY | WGQGTPVTVSS |
| iPS:3 92644 | 21-225_19E1 | VH3\|3-33\|D7\|7-27\|RF2\|JH4 | | QVQLVES-GGGVVQPGRSLRLSCAASG-FTFS | N-----YGMH | WVRQAPGKGLEWVA | VIWYE---ENNQYYADSVKG | RFTISRDNSKNTLYLQMNSLRAEDTAVYYCAR | ELGF----------------------RSDY | WGQGTLVTVSS |
| iPS:3 92654 | 21-225_17A10 | VH3\|3-33\|D7\|7-27\|RF2\|JH4 | | QVQLVES-GGGVVQPGRSLRLSCAASG-FTFS | N-----YGMH | WVRQAPAKGLEWVA | VIWYE---ENNQYYADSVKG | RFTISRDNSKNTLYLQMNSLRAEDTAVYYCAR | ELGF----------------------RSDY | WGQGTLVTVSS |
| iPS:3 92658 | 21-225_18E8 | VH3\|3-33\|D7\|7-27\|RF2\|JH4 | | QVQLVES-GGGVVQPGRSLRLSCAASG-FTFS | N-----YGMH | WVRQAPGKGLEWVA | VIWYE---ENNQYYADSVKG | RFTVSRDNSKNTLFLQMNSLRAEDTAVYYCAR | ELGF----------------------RSDY | WGQGTLVTVSS |
| iPS:3 92666 | 21-225_16F11 | VH3\|3-33\|D7\|7-27\|RF2\|JH4 | | QVQMVES-GGGVVQPGRSLRLSCEASG-FTFS | S-----YGMH | WVRQAPGKGLEWVA | VIWYE---ENNKYYVDSVKG | RFTISRDNSKSTLYLQMNSLRAEDTAVYYCVR | ELGF----------------------QSDY | WGQGTPVTVSS |
| iPS:3 92674 | 21-225_18C2 | VH3\|3-33\|D7\|7-27\|RF2\|JH4 | | QVQLVES-GGGVVQPGRSLRLSCVASG-FTFS | D-----YGMH | WVRQAPGKGLEWMA | VIWYD---VTNKYYADSVKG | RFTISRDNSKNTLYLQMNSLSAEDTAVYYCAR | ELGW----------------------YEDY | WGQGTLVTVSS |
| iPS:3 92680 | 21-225_20A7 | VH3\|3-33\|D7\|7-27\|RF2\|JH4 | | QVQLVES-GGGVVQPGRSLRLSCAASG-FTFS | N-----YGMH | WVRQAPGKGLEWVA | VIWYE---ENNQYYADSVKG | RFTISRDNSKNTLYLQMNSLRAEDTAIYYCAR | ELGF----------------------RSDY | WGQGTLVTVSS |
| iPS:3 92686 | 21-225_17C7 | VH3\|3-33\|D7\|7-27\|RF2\|JH4 | | QVQLVES-GGGVVQPGKSLRLSCAASG-FTFS | D-----YGMH | WVRQAPGKGLEWVA | VIWFD---GSNKYYADSVKG | RFTISRDNSKNTLYLQMNSLRAEGTAVYYCAR | DLGW----------------------TEEY | WGQGTLVTVSS |

| iPS:3 92690 | 21-225_18F2 | VH3\|3-33/D7\|7-27\|RF2/JH4 | | QVQLVES-GGGVVQPGRSLRLSCAASG-FTFS | D-----YGMH | WVRQAPGKGLEWVA | VIWFD---GSNKYYVDSVKG | RFTISRDNSKNTLYLQMNSLRVEDTAVYYCAR | DLGW----------------------TEEY | WGQGTLVTVSS |
|---|---|---|---|---|---|---|---|---|---|---|
| iPS:3 92716 | 21-225_17B5 | VH3\|3-33/D7\|7-27\|RF2/JH4 | | QVQLVES-GGGVVQPGRSLRLSCAASG-FTFS | D-----YGMH | WVRQAPGKGLEWVA | VIWYD---ESNKHYIDSVKG | RFTISRDNSKNTLYLQMNSLRAEDTAVYYCVR | ELGF----------------------RFDY | WGQGTLVTVSS |
| iPS:3 92732 | 21-225_17E5 | VH3\|3-33/D7\|7-27\|RF2/JH4 | | QVQLVES-GGGVVQPGRSLRLSCAASG-FTFS | D-----YGMH | WVRQAPGKGLEWVA | LIWYD---VTNKYYGDSVKG | RFTISRDNSQNTLYLQLNSLRAEDTAVYYCAR | ELGW----------------------YEDY | WGQGTLVTVSS |
| iPS:3 92744 | 21-225_20D5 | VH3\|3-33/D7\|7-27\|RF2/JH4 | | QVQLVES-GGGVVQPGRSLRLSCAASG-FTFS | S-----YGMH | WVRQAPGKGLEWVA | VIWYE---ENNQYYADSVKG | RFTISRDNSKNTLYLQMNSLRADDTAVYYCAR | ELGF----------------------RSDY | WGQGTLVTVSS |
| iPS:3 92758 | 21-225_21G11 | VH3\|3-33/D7\|7-27\|RF2/JH4 | | QVQLVES-GGGVVQPGRSLRLSCAASG-FTFS | D-----YGMH | WVRQAPGKGLEWMA | VIWYD---VTNEYYADSVKG | RFTISRDNSKNTLYLQMNSLRAEDTAVYYCAR | ELGW----------------------YEDY | WGQGTLVTVSS |
| iPS:3 92772 | 21-225_20E12 | VH3\|3-33/D7\|7-27\|RF2/JH4 | | QVQLVES-GGGVVQPGRSLRLSCAASG-FTFS | S-----YGMH | WVRQAPGKGLEWVA | VMWYD---ESNKHYADSVKG | RFTISRDNSRNTLYLQMNSLRAEDTAVYYCAR | ELGF----------------------RFDY | WGQGTLVTVSS |
| iPS:3 92790 | 21-225_20D10 | VH3\|3-33/D7\|7-27\|RF2/JH4 | | QVQLVES-GGGVVQPGRSLRLSCAASG-FTFS | D-----YGMH | WVRQAPGKGLEWVA | VIWFD---GSNKYYVDSVKG | RFTISRDDSKNTLYLQMNSLRAEDTAVYYCAR | DLGW----------------------TEEY | WGQGTLVTVSS |
| iPS:3 92796 | 21-225_22A4 | VH3\|3-33/D7\|7-27\|RF2/JH4 | | QVQLVES-GGGVVQPGRSLRLSCAASG-FTFS | D-----YGIH | WVRQAPGKGLEWVA | VIWFD---GSNKYYADSVKG | RITISRDNSKNTLYLQMNSLRAEDTAVYYCAR | DLGW----------------------TEEY | WGQGTLVTVSS |
| iPS:3 92810 | 21-225_20H12 | VH3\|3-33/D7\|7-27\|RF2/JH4 | | QVQLVES-GGGVVQPGRSLRLSCAASG-FTFS | N-----YGMH | WVRQAPGKGLEWVA | VIWYD---ENNKYYVDSVKG | RFTISRDNSKNTLYLQMNSLRAEDTAVYYCAR | ELGF----------------------RSDY | WGQGTLVTVSS |
| iPS:3 92832 | 21-225_21H8 | VH3\|3-33/D7\|7-27\|RF2/JH4 | | QVQLVES-GGGVVQSGRSLRLSCAASG-FTFS | D-----YGMH | WVRQAPGKGLEWVA | VIWFD---GSNKYYADSVKG | RFTISRDNSKNTLYLQMNSLRAEDTAVYYCAR | DLGW----------------------TEEY | WGQGTLVTVSS |
| iPS:3 92854 | 21-225_21E5 | VH3\|3-33/D7\|7-27\|RF2/JH4 | | QVQLVES-GGGVVQPGRSLRLSCSASG-FTFS | D-----YGMH | WVRQAPGKGLEWVA | VIWYD---ESNKYYADSVKG | RFTISRDNSKNTLYLQMNSLRAEDTAMYYCTR | ELGF----------------------RSDY | WGQGTLVTVSS |
| iPS:3 92860 | 21-225_22H8 | VH3\|3-33/D7\|7-27\|RF2/JH4 | | QVQLVES-GGGVVQPGRSLRLSCAASG-FTFS | S-----YGMH | WVRQAPGKGLEWVA | VIWYD---GNNKYYADSVKG | RFTISRDNSKNTLYLQMNSLRAEDTAVYYCAR | ELAW----------------------YEDY | WGQGTLVTVSS |

| | | | | | | | | | | |
|---|---|---|---|---|---|---|---|---|---|---|
| iPS:3 92866 | 21-225_23H11 | VH3\|3-33/D7\|7-27\|RF2/JH4 | | QEQLVES-GGGVVQPGRSLRLSCAASG-FTFS | S-----YGMH | WVRQAPGKGLEWVA | VIWYD---ENNKYYVDSVKG | RFTISRDNSKNTLYLQMNSLRAEDTAVYYCAR | ELGF-----------------------RSDY | WGQGTLVTVSS |
| iPS:3 92876 | 21-225_21F7 | VH3\|3-33/D7\|7-27\|RF2/JH4 | | QVQLVES-GGGVVQPGRSLRLSCAASG-FTFS | D-----YGMH | WVRQAPGKGLEWVA | VIWFD---GNNKYYVDSVKG | RFTISRDNSKNTLYLQMNSLRAEDTAVYYCAR | DLGW-----------------------TEEY | WGQGTLVTVSS |
| iPS:3 92880 | 21-225_22F9 | VH3\|3-33/D7\|7-27\|RF2/JH4 | | QVQMVES-GGGVVQPGRSLRLSCAASG-FTFS | S-----YGMH | WVRQAPGKGLEWVA | VIWYE---ENNKDYVDSVKG | RFTISRDNSKSTLYLQMNSLRAEDTAVYYCVR | ELGF-----------------------QSDY | WGQGTPVTVSS |
| iPS:3 92894 | 21-225_21G2 | VH3\|3-33/D7\|7-27\|RF2/JH4 | | QVQLVES-GGGVVQPGRSLRLSCAASG-FTFS | D-----YGMH | WVRQAPGKGLEWMA | VIWYD---VTNKYYADSVKG | RFTISRDNSKNTLYLEMNSLRAEDTAVYYCAR | ELGW-----------------------YEDY | WGQGTLVTVSS |
| iPS:3 92900 | 21-225_22F2 | VH3\|3-33/D7\|7-27\|RF2/JH4 | | QVQLVES-GGGVVQPGRSLRLSCAASG-FTFS | D-----YGMH | WVRQAPGKGLEWVA | VIWYE---GSNKYYVDSVRG | RFTISRDNSKSTLYLQMNSLRAEDTAVYYCVR | ELGF-----------------------QSDY | WGQGTPVTVSS |
| iPS:3 92908 | 21-225_23F12 | VH3\|3-33/D7\|7-27\|RF2/JH4 | | QVQLVES-GGGVVQPGRSLRLSCVASG-FTFS | S-----YGMH | WVRQAPGKGLEWVA | VIWYD---ETNKYYADSVKG | RFTISRDNSKNTLYLQMNSLRAEDTAIYYCAR | ELAW-----------------------YEDY | WGQGSLVTVSS |
| iPS:3 92918 | 21-225_28F5 | VH3\|3-33/D7\|7-27\|RF2/JH4 | | QVQLVES-GGGVVQPGRSLRLSCAASG-FTFS | S-----YGMH | WVRQAPGKGLEWVA | VIWYD---ENNKYYADSVKG | RFTISRDNSKNTLYLQMNSLRAEDTAVYYCAR | ELGW-----------------------YDDY | WGQGTLVTVSS |
| iPS:3 92934 | 21-225_27D5 | VH3\|3-33/D7\|7-27\|RF2/JH4 | | QVQLVES-GGGVVQPGRSLRLSCAASG-FTFS | S-----YGMH | WVRQAPGKGLEWVA | VIWYD---ESNKYYGDSVKG | RFTISRDNSKNMLYLQMNSLRGEDTALYYCAR | ELGF-----------------------LSDY | WGQGTLVTVSS |
| iPS:3 92958 | 21-225_28C7 | VH3\|3-33/D7\|7-27\|RF2/JH4 | | QVQLVES-GGGVVQPGRSLRLSCAASG-FTFS | S-----YGMH | WVRQAPGKGLEWVA | VIWYD---ESNKYYADSVKG | RFTISRDNSKNTLYLQMNSLRAEDTAVYYCAR | ELGW-----------------------YDDY | WGQGTLVTVSS |
| iPS:3 92968 | 21-225_25B6 | VH3\|3-33/D7\|7-27\|RF2/JH4 | | QVQLVES-GGGVVQPGRSLRVSCTASG-FTLR | N-----YGMH | WVRQAPGKGLEWVA | VIWYE---ESNKYYTESVKG | RFTISRDNSKNTLYLQMNSLRAEDTAVYYCAR | ELGF-----------------------LSDY | WGQGTLVTVSS |
| iPS:3 92972 | 21-225_26A2 | VH3\|3-33/D7\|7-27\|RF2/JH4 | | QVQLVES-GGGVVQPGRSLRLSCAASG-FTFS | S-----YGMH | WVRQAPGKGLEWVA | VIWYE---GSNKYYVDSVKG | RFTISRDNSKNTLYLQMNSLRAEDTAVYYCAR | ELGW-----------------------YDDY | WGQGTLVTVSS |
| iPS:3 92980 | 21-225_29H6 | VH3\|3-33/D7\|7-27\|RF2/JH4 | | QVQLVES-GGGVVQPGRSLRLSCAASG-FTFS | D-----YGMH | WVRQAPGKGLEWVT | VIWYN---ENNKYYADSVKG | RFTISRDNSKNTLYLQMNSLRAEDTAVYYCAR | ELGM-----------------------TGDS | WGQGTLVTVSS |

| iPS:3 92988 | 21-225_25E6 | VH3\|3-33/D7\|7-27\|RF2\|JH4 | | QVQLVES-GGGVVQPGRSLRLSCAASG-FTFS | D-----YGMH | WVRQAPGKGLEWVA | VIWYD---ENNKYYADSVKG | RFTISRDNSKNTLYLQMNSLRAEDTALYYCAT | ELGM----------------------TGDS | WGQGTLVTVSS |
| iPS:3 92990 | 21-225_25H10 | VH3\|3-33/D7\|7-27\|RF2\|JH4 | | QVQLVES-GGGVVQPGRSLRLSCAASG-FTFS | D-----YGMH | WVRQAPGKGLEWVA | VIWYD---ESNKYYADSMKG | RFTISRDNSKNTLYLQMNSLRAEDTAVYYCAR | ELGM----------------------TGDY | WGQGTLVTVSS |
| iPS:3 93000 | 21-225_29D7 | VH3\|3-33/D7\|7-27\|RF2\|JH4 | | QVQLVES-GGGVVQPGRSLRLSCAASG-FTFS | S-----YGMH | WVRQAPGKGLEWVA | VIWYD---ESNKYYGDSVKG | RFTISRDNSKNTLYLQMNSLRGEDTALYYCAR | ELGF----------------------LSDY | WGQGTLVTVSS |
| iPS:3 93018 | 21-225_29B8 | VH3\|3-33/D7\|7-27\|RF2\|JH4 | | QVQLVES-GGGVVQPGRSLRLSCAASG-FTFS | D-----YGMH | WVRQAPGKGLEWVA | VIWYD---ENNKYYADSVKG | RFTISRDNSKNTLYLQMNSLRAEDTAVYYCAR | ELGM----------------------TGDS | WGQGTLVTVSS |
| iPS:3 93030 | 21-225_25H11 | VH3\|3-33/D7\|7-27\|RF2\|JH4 | | QVQLVES-GGGVVQPGRSLRLSCAASG-FTFS | D-----YGMH | WVRQAPGKGLEWVA | VIWYD---ENNEYYADSVKG | RFTISRDNSKNTLYLQMNSLRAEDTAVYYCAR | ELGM----------------------TGDS | WGQGTLVTVSS |
| iPS:3 93034 | 21-225_27F2 | VH3\|3-33/D7\|7-27\|RF2\|JH4 | | QEQLVES-GGGVVQPGRSLRLSCAASG-FIFS | D-----YGMH | WVRQAPGKGLEWVA | VIWYD---ENNKYYVDSVRG | RFTISRDNSKNTLYLQMNSLRAEDTAVYYCAR | ELGM----------------------TGDS | WGQGTLVTVSS |
| iPS:3 93048 | 21-225_27C3 | VH3\|3-33/D7\|7-27\|RF2\|JH4 | | QVQLVES-GGGVVQPGRSLRLSCAASG-FTFS | D-----YGMH | WVRQAPGKGLEWVA | VIWYD---ENNKSYADSVKG | RFTISRDNSKNTLYLQMNSLRAEDTAVYYCAR | ELGM----------------------TGDS | WGQGTLVTVSS |
| iPS:3 93054 | 21-225_29G8 | VH3\|3-33/D7\|7-27\|RF2\|JH4 | | QVQLVES-GGGVVQPGRSLRLSCAASG-FTFS | D-----YGMH | WVRQAPGKGLEWVA | VIWYD---ETNKYYADSVKG | RFTISRDNSKNTLYLQMNSLRAEDTAVYYCAR | ELGM----------------------TSDY | WGQGTLVTVSS |
| iPS:3 93812 | 21-225_6A11 | VH3\|3-33/D7\|7-27\|RF2\|JH4 | | QVQLVES-GGGVVQPGRSLRLSCAASG-FTFS | D-----YGMH | WVRQAPGKGLEWVA | VIWFD---GSNKYYADSVKG | RFTISRDNSKNTLYLQMNSLRAEDTAVYDCAR | DLGW----------------------TEEY | WGQGTLVTVSS |
| iPS:3 93818 | 21-225_6G12 | VH3\|3-33/D7\|7-27\|RF2\|JH4 | | QVQLVES-GGGVVQPGRSLRLSCAASG-FTFS | S-----YGMH | WVRQAPGKGLEWVA | VIWYD---RSNNYYADSVKG | RFTISRDNSKNTLYLQMNSLRAEDTAVYYCAR | ELGF----------------------RSDY | WGQGTLVTVSS |
| iPS:3 93820 | 21-225_8H7 | VH3\|3-33/D7\|7-27\|RF2\|JH4 | | QVQLVES-GGGVVQPGRSLRLSCPASG-FTFS | D-----FGMH | WVRQAPGKGLEWVA | VIWYE---ENNQYYADSVKG | RFTISRDNSKNTLYLQMNSLRAEDTAVYYCAR | ELGF----------------------RSDY | WGQGTLVTVSS |
| iPS:3 93826 | 21-225_10G5 | VH3\|3-33/D7\|7-27\|RF2\|JH4 | | QVQLVES-GGGVVQPGRSLRLSCAASG-FTFS | N-----YGMH | WVRQAPGKGLEWVA | VIWYE---ENNQYYADSVKG | RFTISRDNSKNTLYLQMNSLRAEDTAVYYCTR | ELGF----------------------RSDY | WGQGTLVTVSS |

| | | | | | | | | | | |
|---|---|---|---|---|---|---|---|---|---|---|
| iPS:3 93828 | 21-225_10H12 | VH3\|3-33/D7\|7-27\|RF2\|JH4 | | QVQLVES-GGGVVQPGRSLRLSCAASG-FTFS | D-----YGMH | WVRQAPGK GLEWVA | VIWYE---DNNQYYADS VKG | RFTISRDNSKNTLYLQMNSL RAEDTAVYYCAR | ELGF------------ ----------RSDY | WGQGT LVTVS S |
| iPS:3 93830 | 21-225_12A1 | VH3\|3-33/D7\|7-27\|RF2\|JH4 | | QVQLVES-GGGVVQPGRSLRLSCAASG-FTFS | S-----YGMH | WVRQAPGK GLEWVA | VIWYE---ENNQYYADS VKG | RFTISRDNSKNTLYLQMNSL RAEDTAVYYCAR | ELGF------------ ----------RSDY | WGQGT LVTVS S |
| iPS:3 93838 | 21-225_6G2 | VH3\|3-33/D7\|7-27\|RF2\|JH4 | | LVQLVES-GGGVVQPGKSLRLSCAASG-FTFS | D-----YGIH | WVRQAPGK GLEWVA | VIWFD---GSNKYYADS VKG | RFTISSDNSKNTLYLQMNSL RAEDTAVYYCAR | DLGW------------ ----------TEEY | WGQGT LVTVS S |
| iPS:3 93854 | 21-225_7H11 | VH3\|3-33/D7\|7-27\|RF2\|JH4 | | QVQLVES-GGGVVQPGRSLRLSCSASG-FTFS | D-----YGMH | WVRQAPGK GLEWVA | VIWYD---ENNKYYADS VKG | RFTISRDNSKNTLYLQMNSL RAEDTAVYYCAR | ELGF------------ ----------RSDY | WGQGT LVTVS S |
| iPS:3 93866 | 21-225_14E3 | VH3\|3-33/D7\|7-27\|RF2\|JH4 | | QVQLVES-GGGVVQPGRSLRLSCAASG-FTFS | D-----FGMH | WVRQAPGK GLEWVA | VIWYE---ENNQYYADS VKG | RFTISRDNSKNTLYLQMNSL RAEDTAFYYCAR | ELGF------------ ----------RSDY | WGQGT LVTVS S |
| iPS:3 93876 | 21-225_9A1 | VH3\|3-33/D7\|7-27\|RF2\|JH4 | | QVQVVES-GGGVVQPGRSLRLSCAASG-FTFS | D-----YGMH | WVRQAPGK GLEWVA | VIWFD---GSNKYYADS VKG | RFTISRDNSKNTLYLQMNSL RAEDTAVYYCAR | DLGW------------ ----------TEEY | WGQGT PVTVS S |
| iPS:3 93882 | 21-225_15E3 | VH3\|3-33/D7\|7-27\|RF2\|JH4 | | QVQLVES-GGTVVQPGRSLRLSCAASG-FTFS | S-----YGMH | WVRQAPGK GLEWVA | VIWYE---ENNKHYADS VKG | RFTISRDNSKNTLYLQMNSL RAEDTAVYYCAR | ELGF------------ ----------LSDY | WGQGT LVTVS S |
| iPS:3 93884 | 21-225_16F4 | VH3\|3-33/D7\|7-27\|RF2\|JH4 | | QVQLVES-GGGVVQPGRSLRLSCAASG-FTFS | N-----YGMH | WVRQAPGK GLEWVA | VIWYE---GSNQYYGDS VKG | RFTISRDNSKNTVYLQMHSL RAEDTAVYYCAR | ELGF------------ ----------LSDY | WGQGT LVTVS S |
| iPS:3 93912 | 21-225_16F6 | VH3\|3-33/D7\|7-27\|RF2\|JH4 | | QVQLVES-GGGVVQPGRSLRLSCAASG-FTFS | N-----YGMH | RVRQAPGK GLEWVA | VIWYE---GSNQYYGDS VKG | RFTISRDNSKNTVYLQMHSL RAEDTAVYYCAR | ELGF------------ ----------LSDY | WGQGT LVTVS S |
| iPS:3 93922 | 21-225_2B2 | VH3\|3-33/D7\|7-27\|RF2\|JH4 | | QVQLVES-GGGVVQPGRSLRLSCAASG-FTFS | S-----YGMH | WVRQAPGK GLEWVA | VIWYE---ENNKYYVDS VKG | RFTISRDNSKSTLYLQMNSL RAEDTAVYYCVR | ELGF------------ ----------QSDY | WGQGT PVTVS S |
| iPS:3 93934 | 21-225_13E6 | VH3\|3-33/D7\|7-27\|RF2\|JH4 | | QVQLVES-GGGVVQPGRSLRLSCAASG-FTFS | N-----YGMH | WVRQAPGK GLEWVA | VIWYD---ENNKYYIDS VKG | RFTISRDNSKNTLYLQMNSL RAEDTAVYYCAR | ELGF------------ ----------RSDY | WGQGT LVTVS S |
| iPS:3 93948 | 21-225_16A5 | VH3\|3-33/D7\|7-27\|RF2\|JH4 | | QVQLVES-GGGVVQPGRSLRLSCAASG-FTFS | D-----YGMH | WVRQAPGK GLEWVA | VIWFD---GSNKYYVDS VKG | RFTISRDNSKNTLYLQMSSL RAEDTAVYYCAR | DLGW------------ ----------TEEY | WGQGT LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 93960 | 21-225_7G2 | VH3|3-33/D7|7-27|RF2/JH4 | | QVQLVES-GGGVVQPGRSLRLSCAASG-FTFS | D-----YGMH | WVRQAPGKGLEWMA | VIWYD---VTNKYYADSVKG | RFTISRDNSKNTLYLQLNSLRAEDTAVYYCAR | ELGW----------------------YEDY | WGQGTLVTVSS |
| iPS:3 93974 | 21-225_7C4 | VH3|3-33/D7|7-27|RF2/JH4 | | QVQLVES-GGGVVQPGKSLRLSCAASG-FTFS | N-----YGMH | WVRQAPGKGLEWVA | VIWYE---ENNQYYADSVKG | RFTISRDNSKNTLYLQMNSLRAEDTAVYYCAR | ELGF----------------------RSDY | WGQGTLVTVSS |
| iPS:3 93976 | 21-225_7E9 | VH3|3-33/D7|7-27|RF2/JH4 | | QVQLVES-GGGVVQPGRSLRLSCAASG-FTFS | D-----YGMH | WVRQAPGKGLEWVA | VIWYD---ENNKYYVDSVKG | RFTISRDNSKNTLYLQMNSLRAEDTAVYYCAR | ELGF----------------------RSDY | WGQGTLVTVSS |
| iPS:3 93994 | 21-225_8C9 | VH3|3-33/D7|7-27|RF2/JH4 | | QVQLVES-GGGVVQPGRSLRLSCAASG-FTFS | S-----YGMH | WVRQAPGKGLEWVA | VIWYD---ENNKYYVDSVKG | RFTISRDNSKNTLYLQMNSLRAEDTAVYYCAR | ELGF----------------------RSDY | WGQGTLVTVSS |
| iPS:3 93998 | 21-225_12B12 | VH3|3-33/D7|7-27|RF2/JH4 | | QVQLVES-GGGVVQPGRSLRLSCAASG-FTFS | D-----YGMH | WVRQAPGKGLEWMA | VIWYD---VTNKYYADSVKG | RFTISRDNSKNTLYLQMNSLRAEDTAVYYCAR | ELGW----------------------YEDY | WGQGTLVTVSS |
| iPS:3 94024 | 21-225_16B7 | VH3|3-33/D7|7-27|RF2/JH4 | | QVQLVES-GGGVVQPGRSLRLSCAASG-FTFS | S-----YGMH | WVRQAPGKGLEWVA | VIWYD---ESNKYYADSVKG | RFTISRDNSKNTLYLQMNSLRAEDTAVYYCAR | ELGF----------------------LSDY | WGQGTLVTVSS |
| iPS:3 94059 | 21-225_9E8 | VH3|3-33/D7|7-27|RF2/JH4 | | QVQLVES-GGGVVQPGRSLRLSCEASG-FTFS | S-----YGMH | WVRQAPGKGLEWVA | VIWYE---ENNQYYADSVKG | RFTISRDNSKNTLYLQMNSLRAEDTAVYYCAR | ELGF----------------------RSDY | WGQGTLVTVSS |
| iPS:3 94067 | 21-225_12F2 | VH3|3-33/D7|7-27|RF2/JH4 | | QVQLVES-GGGVVQPGRSLRLSCAASG-FTFS | D-----YGMH | WVRQAPGKGLEWVA | VIWFD---GNNKYYVDSVKG | RFTISRDNSKNTLFLQMNSLRAEDTAVYYCVR | ELAW----------------------SEDY | WGQGTLVTVSS |
| iPS:3 94089 | 21-225_12E6 | VH3|3-33/D7|7-27|RF2/JH4 | | QVQLVES-GGGVVQPGRSLRLSCAASG-FTFS | D-----YGMH | WVRQAPGKGLEWVT | VIWYD---ESNKYYADSVKG | RFTISRDDSKNTLYLQMNSLRAEDTAVYYCAR | ELAW----------------------YEDY | WGQGTLVTVSS |
| iPS:3 94097 | 21-225_16G7 | VH3|3-33/D7|7-27|RF2/JH4 | | QVQLVES-GGGVVQPGRSLRLSCAASG-FTFT | D------YGMH | WVRQAPGKGLEWVA | VIWYD---ENNEYYADSVKG | RFTISRANSKNTLYLQMNSLRAEDTAVYYCAR | ELAW----------------------YEDY | WGQGTLVTVSS |
| iPS:4 02219 | 21-225_1C12 | VH3|3-33/D7|7-27|RF2/JH4 | | QVQLVES-GGGVVQPGRSLRLSCAASG-FTFS | N-----YGMH | WVRQAPGKGLEWVA | VIWYD---ENNKYYVDSVKG | RFTISRDNSKNTLYLQMNSLRAEDTAVYYCAR | ELGF----------------------RSDY | WGQGTLVTVSS |
| | | VH3|3-21/D4|4-11|RF2/JH4 | 32243 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DYSNY----------------------YFDY | WGQGTLVTVSS |

| | | | | | | | | | |
|---|---|---|---|---|---|---|---|---|---|
| iPS:4 33895 | 21-225_43E1 | VH3\|3-21\|D4\|4-11\|RF2/J H4 | | EVQLVES-GGGLVKPGGSLRL SCAASG-FTFS | S-----YSMN | WVRQAPGK GLEWVS | AISGN---STYIYYADS LKG | RFTISRDNAKNSLFLQLNSL RAEDTAVYYCAR | DRG------------------------SE | WGQGT LVTVS S |
| iPS:4 34103 | 21-225_53G1 | VH3\|3-21\|D4\|4-11\|RF2/J H4 | | EVQLVES-GGGLVKPGESLRL SCAASG-FTFS | S-----YSMN | WVRQAPGK GLEWVS | SISGS---SSYIYYADS VKG | RFTISRDNAKNSLYLQMNSL RAEDTAVYYCAR | DRG------------------------ST | WGQGT LVTVS S |
| IPS:4 34179 | 21-225_56F1 | VH3\|3-21\|D4\|4-11\|RF2/J H4 | | EVQLVES-GGGLVKFGGSLRL SCAASG-FTFS | S-----YSMN | WVRQAPGK GLEWVS | SISSS---STYIYYGDS VKG | RFTISRDNAKNSLYLQMNSL RAEDTAVYYCAR | DRG------------------------SS | WGQGT LVTVS S |
| iPS:4 34263 | 21-225_56H7 | VH3\|3-21\|D4\|4-11\|RF2/J H4 | | EVQLVES-GGGLVKPGGSLRL SCAASG-FSFS | S-----YSMN | WVRQAPGK GLEWVS | SISSS---STYIYYGDS VKG | RFTISRDNAKNSLYLQMNSL RAEDTAVYYCAR | DRG------------------------SS | WGQGT LVTVS S |
| iPS:4 35521 | 21-225_157H4 | VH3\|3-21\|D4\|4-11\|RF2/J H4 | | EVQLVES-GGGLVKPGGSLRL SCAASG-FTFS | S-----YSMN | WVRQAPGK GLEWVS | SISSS---STYIYYADS VKG | RFTISRDNAKNSLYLQMNSL RAEDTAVYYCAR | DRG------------------------SI | WGQGT LVTVS S |
| IPS:4 35527 | 21-225_157G7 | VH3\|3-21\|D4\|4-11\|RF2/J H4 | | EVQLVES-GGGLVKPGGSLRF SCAASG-FTFS | S-----YSMN | WVRQAPGK GLEWVS | SISGS---STYIYYADS VKG | RFTISRDNAKNSLYLQMNSL RAEDTAVYYCAR | DRG------------------------SS | WGQGT LVTVS S |
| IPS:4 35529 | 21-225_157H7 | VH3\|3-21\|D4\|4-11\|RF2/J H4 | | EVQLVES-GGGLVKPGGSLRL SCAASG-FTFS | S-----YSMN | WVRQAPGK GLEWVS | CISGS---SSYIYYADS VKG | RFTMSRDNAKNSLYLQMNSL RAEDTAVYYCVR | DRG------------------------GY | WGQGT LVTVS S |
| IPS:4 35547 | 21-225_158F5 | VH3\|3-21\|D4\|4-11\|RF2/J H4 | | EVQLVES-GGGLVKPGGSLRF SCAASG-FTFS | S-----YSMN | WVRQAPGK GLEWVS | SISGS---STYIYYADS VKG | RFTISRDNAKNSLYLQMNSL RAEDTAVYYCAR | DRG------------------------SS | WGQGT LVTVS S |
| iPS:4 35549 | 21-225_158H5 | VH3\|3-21\|D4\|4-11\|RF2/J H4 | | EVQLVES-GGGLVKPGGSLRL SCAASG-FTFS | S-----YSMN | WVRQAPGK GLEWVS | SISGS---STYIYYADS VKG | RFTISRDNAKNSLHLQMNSL RAEDTAVYYCAR | DRG------------------------SS | WGQGT LVTVS S |
| iPS:4 35553 | 21-225_158G8 | VH3\|3-21\|D4\|4-11\|RF2/J H4 | | EVQLVES-GGGLVTPGGSLRL SCAASG-FTFS | S-----YTMN | WVRQAPGK GLEWVS | LISGS---SSYIYYADS VKG | RFTISRDNAKNSLYLQMNSL RAEDTAVYYCAR | DRG------------------------SL | WGQGT LVTVS S |
| iPS:4 35581 | 21-225_160H1 | VH3\|3-21\|D4\|4-11\|RF2/J H4 | | EVQLVES-GGGLVKPGGSLRL SCAASG-FTFS | S-----YRMN | WVRQAPGK GLEWVS | SISSS---TGYMYYADS VKG | RFTISRDNAKNSLYLQMNSL RAEDTAVYFCAR | DK------------------------DY | WGQGT LVTVS S |
| iPS:4 35593 | 21-225_160F4 | VH3\|3-21\|D4\|4-11\|RF2/J H4 | | EVQLVES-GGGLVKPGGSLRF SCAASG-FTFS | S-----YSMN | WVRQAPGK GLEWVS | SISGS---STYIYYADS VKG | RFTISRDNAKNSLYLQMNSL RAEDTAVYYCAR | DRG------------------------SS | WGQGT LVTVS S |

| iPS:4 35617 | 21-225_162F2 | VH3\|3-21/D4\|4-11\|RF2/JH4 | | EVQLVES-GGGLVKPGGSLRFSCAASG-FTFS | S-----YSMN | WVRQAPGKGLEWVS | SISGS---STYIYYADSVKG | RFTISRDNAKNSLYLQMNSLRAEDTAVYYCAR | DRG-------------------------SS | WGQGTLVTVSS |
| iPS:4 35621 | 21-225_162H3 | VH3\|3-21/D4\|4-11\|RF2/JH4 | | EVQLVES-GGGLVKPGGSLRFSCAASG-FTFS | S-----YSMN | RVRQAPGKGLEWVS | SISGS---STYIYYADSVKG | RFTISRDNAKNSLYLQMNSLRAEDTAVYYCAR | DRG-------------------------SS | WGQGTLVTVSS |
| IPS:4 35641 | 21-225_163F9 | VH3\|3-21/D4\|4-11\|RF2/JH4 | | EVQLVES-GGGLVQPGGSLRLSCAASG-FTFS | S-----YSMN | WVRQAPGKGLEWVS | SISGS---STYIYYADSVKG | RFTISRDNAKNSLYLQMNSLRAEDTAVYYCAR | DRG-------------------------SL | WGQGTLVTVSS |
| iPS:4 35719 | 21-225_171A11 | VH3\|3-21/D4\|4-11\|RF2/JH4 | | EVQLVES-GGGLVKPGGSLRLSCAASG-FTFS | S-----YSMN | WVRQAPGKGLEWVS | SISGS---SSYIYYADSVKG | RFTISRDNAKNSLYLQMNSLRAEDTAVYYCAR | DRG-------------------------SS | WGQGILVTVSS |
| iPS:4 36856 | 21-225_58C5 | VH3\|3-21/D4\|4-11\|RF2/JH4 | | EVQLVES-GGGLVKPGGSLRLSCAASG-FTFN | S-----YSMN | WVRQAPGKGLEWIS | SISSS---SYYLYYADSVKG | RFTISRDSAKNSLYLQMNSLRAEDTAVYYCAR | TYSG----------------------SFDY | WGQGTLVTVSS |
| IPS:4 48904 | 21-225_65C12 | VH3\|3-21/D4\|4-11\|RF2/JH4 | | EVQLVES-GGGLVKPGGSLRLSCAASG-FTFR | S-----FSLN | WVRQAPGKGLEWVS | SISSS---SSYIYYADSVKG | RFTISRDNAKNSLYLQMNSLRAEDTAVYYCAR | DAY----------------------SHY | WGQGTLVTVSS |
| iPS:4 72730 | 21-225_14B1_LC1 | VH3\|3-21/D4\|4-11\|RF2/JH4 | | EVQLVES-GGGLVKPGGSLRLSCAASG-FTFS | S-----YTMN | WVRQAPGKGLEWVS | SISGS---SSYLYYPDSVKG | RFTISRDNAKNSLYLQMNSLRAEDTAVYYCAR | DRG-------------------------SS | WGQGTLVTVSS |
| iPS:4 72731 | 21-225_14B1_LC2 | VH3\|3-21/D4\|4-11\|RF2/JH4 | | EVQLVES-GGGLVKPGGSLRLSCAASG-FTFS | S-----YTMN | WVRQAPGKGLEWVS | SISGS---SSYLYYPDSVKG | RFTISRDNAKNSLYLQMNSLRAEDTAVYYCAR | DRG-------------------------SS | WGQGTLVTVSS |
| iPS:3 92726 | 21-225_20B5 | VH3\|3-21/D4\|4-11\|RF2/JH4 | | EVQLVEA-GGGLVKPGGSLRLSCAASG-FTFT | S-----YSMN | WVRQAPGKGLEWVS | SISGS---SSYIYYADSVKG | RFTISRDNAKNSLYLQMNSLRAEDTAVYYCAR | DRG----------------------SY | WGQGTLVTVSS |
| iPS:3 92734 | 21-225_17D8 | VH3\|3-21/D4\|4-11\|RF2/JH4 | | EVQLVES-GGGLVKPGGSLRLSCAASG-FTFS | S------YGLN | WVRQAPGKGLEWVS | SISGS---GSHISYADSVKG | RFTISRDNAKNSLYLQLNSLRAEDTAVYYCAR | DRG-------------------------SG | WGQGTLVTVSS |
| iPS:3 92768 | 21-225_20B8 | VH3\|3-21/D4\|4-11\|RF2/JH4 | | EVQLVES-GGGLVKPGGSLRLSCAASG-FTFS | S-----YSMN | WVRQAPGKGLEWVS | SISGS---GSHIYYADSVKG | RFTISRDNAKNSLYLQMNSLRAEDTAVYYCAR | DRG-------------------------SG | WGQGTLVTVSS |
| iPS:3 92778 | 21-225_22H3 | VH3\|3-21/D4\|4-11\|RF2/JH4 | | AVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVFYCAR | DRG-------------------------SL | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 92788 | 21-225_20C8 | VH3\|3-21\|D4\|4-11\|RF2\|JH4 | | EVQLVES-GGGLVKPGGSLRLSCAASG-FTFS | S-----YSMN | WVRQAPGKGLEWVS | SISGS---SSYIYYADSVKA | RFTISRDNAKNSLYLQMNSLRAEDTAVYYCAR | DRG-------------------------SL | WGQGTLVTVSS |
| iPS:3 92792 | 21-225_20G12 | VH3\|3-21\|D4\|4-11\|RF2\|JH4 | | EVQLVES-GGGLVKPGGSLRLSCAASG-FTFS | S-----YSMN | WVRQAPGKGLEWVS | SISGS---SSYIYYADSLKG | RFTISRDNAKNSLYLQMNSLRAEDTAVYYCAR | DRG-------------SY | WGQGTLVTVSS |
| iPS:3 92844 | 21-225_23E11 | VH3\|3-21\|D4\|4-11\|RF2\|JH4 | | EVQLVES-GGGLVKPGGSLRLSCAASG-FTFS | S-----YIMN | WVRQAPGKGLEWVS | SISGS---SSYIWYVDSVKG | RFTISRDNAKNSLYLQMNSLRAEDTAVYYCAR | DRG-------------SL | WGQGTLVTVSS |
| iPS:3 92848 | 21-225_20F9 | VH3\|3-21\|D4\|4-11\|RF2\|JH4 | | EVQLVES-GGGLVKPGGSLRLSCAASG-FTFS | S-----YSMN | WVRQAPCKGLEWVS | SISSS---SSYIYYADSVKG | RFTISRDNAKNSLYLQMNSLRAEDTAVYYCAR | DRG-------------SC | WGQGTLVTISS |
| iPS:3 92850 | 21-225_20H10 | VH3\|3-21\|D4\|4-11\|RF2\|JH4 | | EVQLVES-GGGLVKPGGSLRLSCAASG-FTFS | T-----YSMN | WVRQAPGKGLEWVS | SISSS---SSYIYYADSVKG | RFTISRDNAKNSLYLQMNSLRAEDTAIYYCAR | DRG-------------SL | WGQGTLVTVSS |
| iPS:3 93006 | 21-225_31G9 | VH3\|3-21\|D4\|4-11\|RF2\|JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DRG-------------SS | WGQGTLVTVSS |
| iPS:3 93022 | 21-225_30H11 | VH3\|3-21\|D4\|4-11\|RF2\|JH4 | | EVQLVES-GGGLVKPGGSLRLSCAASG-FTFS | S-----YSMN | WVRQAPGKGLEWVS | SISGS---SSYIYYADSVKG | RFTISRDNAKNSLYLQMNSLRAEDTAVYYCAR | DRG-------------SL | WGQGTLVTVSS |
| iPS:3 93130 | 21-225_33C2 | VH3\|3-21\|D4\|4-11\|RF2\|JH4 | | EVQLVES-GGGLVKPGGSLRLSCAASG-FTFS | S-----YTMN | WVRQAPGKGLQWVS | SISGS---SSYIYYADSVKG | RFTISRDNAKNSLFLQMNSLRAEDTAIFYCAR | DRG-------------GT | WGQGTLVTVSS |
| iPS:3 93906 | 21-225_13D3 | VH3\|3-21\|D4\|4-11\|RF2\|JH4 | | EVQLVES-GGGLVKPGGSLRLSCAASG-FTFS | S-----YTMN | WVRQAPGKGLDWVS | SISGS---SSYIYYADSVKG | RFTISRDNAKNSLYLQMNSLRAEDTAVYYCAR | DRG-------------SG | WGQGTLVTVSS |
| iPS:3 93982 | 21-225_6C12 | VH3\|3-21\|D4\|4-11\|RF2\|JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DRG-------------SL | WGQGTLVTVSS |
| iPS:3 98478 | 21-225_17C10 | VH3\|3-21\|D4\|4-11\|RF2\|JH4 | | EVQLVES-GGGLVKPGGSLRLSCAASG-FTFS | S-----YSMN | WVRQAPGKGLEWVS | SISGS---SSYMYYADSVKG | RFTISRDNAKNSLYLQMNSLRAEDTALYYCAR | DRG-------------SS | WGQGTLVTVSS |
| | VH3\|3-23/D6\|6-6\|RF1/JH4 | | 32244 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | EYSSS----------SYFDY | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 33897 | 21-225_43C2 | VH3\|3-23/D6\|6-6\|RF1/JH4 | | EVQLLES-GGGLVQPGGSLRLSCAASG-FTFS | S-----YAMS | WVRQAPGK GLEWVS | AISGR---GVNTFDADS VKG | RFTISRDNSKNTLYLQMNSL RAEDTAVYYCAK | ERSGS-------------------YFDY | WGQGT LVTVS S |
| iPS:4 33903 | 21-225_43H4 | VH3\|3-23/D6\|6-6\|RF1/JH4 | | EVQLLES-GGGLVQPGGSLRLSCAASG-FTFS | S-----YAMS | WVRQAPGK GLEWVS | AISGR---GINTFDADS VKG | RFTISRDNSKNTLYLQMNSL RAEDTAVYYCAK | ERSGS-------------------YFDY | WGQGT LVTVS S |
| iPS:4 33911 | 21-225_43E8 | VH3\|3-23/D6\|6-6\|RF1/JH4 | | EVQLLES-GGGLVQPGGSLRLSCAASG-FTFS | S-----YAMS | WVRQAPGK GLEWVS | AISGR---GINTFDADS VKG | RFTISRDNSKNTLYLQMNSL RAEDTAVYYCAK | ERSGS-------------------YFDY | WGQGT LVTVS S |
| iPS:4 33941 | 21-225_44D10 | VH3\|3-23/D6\|6-6\|RF1/JH4 | | EVQLLES-GGGLVQPGGSLRLSCAASG-FTFS | S-----YAMS | WVRQAPGK GLEWVS | AISGR---GVNTFDADS VKG | RFTISRDNSKNTLYLQMNSL RAEDTAVYYCAK | ERSGS-------------------YFDY | WGQGT LVTVS S |
| iPS:4 33957 | 21-225_45F8 | VH3\|3-23/D6\|6-6\|RF1/JH4 | | EVQLLES-GGGLVQPGGSLRLSCAASG-FTFS | S-----YAMS | WVRQAPGK GLEWVS | AISGR---GVNTFDADS VKG | RFTISRDNSKNTLYLQMNSL RAEDTAVYYCAK | ERSGS-------------------YFDY | WGQGT LVTVS S |
| iPS:4 33973 | 21-225_46A6 | VH3\|3-23/D6\|6-6\|RF1/JH4 | | EVQLLES-GGGLVQPGGSLRLSCAASG-FTFS | S-----YAMS | WVRQAPGK GLEWVS | AISGR---GINTFDADS VKG | RFTISRDNSKNTLYLQMNSL RAEDTAVYYCAK | ERSGS-------------------YFDY | WGQGT LVTVS S |
| iPS:4 35715 | 21-225_171A8 | VH3\|3-23/D6\|6-6\|RF1/JH4 | | EVQLLES-GGGLVQPGGSLRLSCAASG-FTFR | S-----SAMS | WVRQAPGK GLEWVS | VISGS---GGSTFYTDS VKG | RFTISRDNSKNTLYLQMNSL RAEDTAVYYCAK | SNSSG-------------------WFDY | WGQGT LVTVS S |
| iPS:4 35739 | 21-225_174G7 | VH3\|3-23/D6\|6-6\|RF1/JH4 | | EVQLLES-GGGLVQPGGSLRLSCAASG-FTFR | S-----SAMS | WVRQAPGK GLEWVS | VISGS---GGSTFYTDS VKG | RFTISRDNSKNTLYLQMNSL RAEDTAVYYCAK | SNSSG-------------------WFDY | WGQGT LVTVS S |
| iPS:4 35749 | 21-225_175C10 | VH3\|3-23/D6\|6-6\|RF1/JH4 | | EVQLLES-GGGLVQPGGSLRLSCAASG-FTFS | S-----YAMS | WVRQAPGK GLEWVS | SISGR---GGSTFYADS VKG | RFTVSRDNSKNTLYLQMNSL RAEDTAVYYCAK | SNSSG-------------------WFDY | WGQGT LVTVS S |
| | VH3\|3-21/D7\|7-27\|RF1/JH4 | | 32245 | EVQLVES-GGCLVKPGGSLRLSCAASG-FTFS | S------YSMN | WVRQAPGK GLEWVS | SISSS-SSYIYYADS VKG | RFTISRDNAKNSLYLQMNSL RAEDTAVYYCAR | LTGY-------------FDY | WGQGT LVTVS S |
| iPS:4 33901 | 21-225_43A4 | VH3\|3-21/D7\|7-27\|RF1/JH4 | | EVQLVES-GGGLVKPGGSLRLSCAASG-FTFS | S-----YTMN | WVRQVPGK GLEWVS | SISGS---STYIYYADS VKG | RFTISRDDAQNSLYLQMNSL RGEDTAVYYCAR | VTS-------------------FDY | WGQGA LVTVS S |
| iPS:4 33961 | 21-225_45D9 | VH3\|3-21/D7\|7-27\|RF1/JH4 | | EVQLVES-GGGLVKPGGSLRLSCAASG-FTFS | S-----YTMN | WVRQVPGK GLEWVS | SISGS---STYIYYADS VKG | RFTISRDDAQNSLYLQMNSL RGEDTAVYYCAR | VTS-------------------FDY | WGQGA LVTVS S |

| | | | | | | | | | | |
|---|---|---|---|---|---|---|---|---|---|---|
| iPS:4 34135 | 21-225_54H3 | VH3\|3-21\|D7\|7-27\|RF1\|J H4 | | EVQLVES-GGGLVKPGGSLRL SCAASG-FTFS | S-----YSMI | WVRQAPGK GLEWVS | SISGT---SSYIYYADS VKG | RFTISRDNAKNSLYLQMNSL RAEDTAVYYCAG | MIT-------------------------VI | WGQGT LVTVS S |
| iPS:4 34331 | 21-225_63H8 | VH3\|3-21\|D7\|7-27\|RF1\|J H4 | | EVQLVES-GGGLVKPGGSLRL SCAASG-FTFS | S-----YNMN | WVRQAPGK GLEWVS | SISGS---STYMNYTDS VKG | RFTISRDNAKNSLYLQMNSL RAEDTAVYYCAR | LRN-------------------------FDY | WGQGT LVTVS S |
| iPS:4 35421 | 21-225_151F1 | VH3\|3-21\|D7\|7-27\|RF1\|J H4 | | EVQLVES-GGGLVKPGGSLRL SCAASG-YTFR | S-----FSMN | WVRQAPGK GLEWVS | SISSS---SYYIYYADS VKG | RFTISRDNAKNSLYLQMNSL RAEDTAVYYCAR | DIP-------------------------LVY | WGQGT LVTVS S |
| iPS:4 35653 | 21-225_166H12 | VH3\|3-21\|D7\|7-27\|RF1\|J H4 | | EVQLVES-GGALVKPGGSLRL SCAASG-FTFS | S-----YSMS | WVRQAPGK GLGWVS | SISGS---SSYSYYADS VKG | RFTISRDNAKNSLYLQMNSL RAEDTAVYYCAR | LTG-------------------------FDY | WGQGT LVTVS S |
| iPS:4 36648 | 21-225_227F11 | VH3\|3-21\|D7\|7-27\|RF1\|J H4 | | EVQLVES-GGGLVKPGGSLRL SCAVSG-FTFS | T-----YSMN | WVRQAPGK GLEWVS | SISSS---INYMYYADS VKG | RFTISRDNAKNSLYLQMNSL RAEDSAVYYCAR | LG-----------------------VY | WGQGT LVTVS S |
| iPS:3 92952 | 21-225_26G1 | VH3\|3-21\|D7\|7-27\|RF1\|J H4 | | EVQLVES-GGGLVKPGGSLRL SCAASG-FTFS | S-----YGMN | WVRQAPGK GLEWVS | SISGS---SSYIYYADS VKG | RFTISRDNAKNSLYLQMNSL RAEDTAVYYCAR | LTT-------------------------FDF | WGQGT LVTVS S |
| iPS:3 93082 | 21-225_34C11 | VH3\|3-21\|D7\|7-27\|RF1\|J H4 | | EVQLVES-GGGLVKPGGSLRL SCAASG-FTFS | S-----YTMS | WVRQAPGK GLEWVS | SISGS---SNYIYYADS VKG | RFTISRDNAKNSLYLQMNSL RAEDTAVYYCAR | LTG-------------------------FDY | WGQGT LVTVS S |
| iPS:3 94061 | 21-225_12D2 | VH3\|3-21\|D7\|7-27\|RF1\|J H4 | | EVQLVES-GGGLVKPGGSLRL SCAASG-FTFS | S-----YSMN | WVRQAPGK GLEWVS | SISSS---SSYIYYADS VKG | RFTISRDNAKNSLYLQMNSL RAEDTAVYYCAR | LG-----------------------DY | WGQGT LVAVS S |
| iPS:3 94071 | 21-225_10C7 | VH3\|3-21\|D7\|7-27\|RF1\|J H4 | | EVQLVES-GGGLVKPGGSLRL SCAASG-FTFS | S-----YSMN | WVRQAPGK GLEWVS | SISSS---NNYIYYADS VKG | RFTISRDNAKNSLYLQMNSL RAEDSAVYYCAR | LG-----------------------VY | WGQGT LVTVS S |
| | | | | | | | | | | |
| iPS:3 98532 | 21-225_33B7 | VH3\|3-21\|D7\|7-27\|RF1\|J H4 | | EVQLVES-GGGLVKPGGSLRL SCAASG-FTFS | S-----YNMN | WVRQAPGK GLEWVS | SISGS---SSYIYYADS VKG | RFTISRDNAKNSLYLQMNSL RAEDTAVYYCAR | LNG-------------------------FDY | WGQGT LVTVS S |
| iPS:4 02225 | 21-225_2B1 | VH3\|3-21\|D7\|7-27\|RF1\|J H4 | | EVQLVES-GGGLVKPGGSLRL SCAASG-FTFS | S-----YSMN | WVRQAPGK GLEWVS | SISSS---SSYIYYADS VKG | RFTISRDNAKNSLYLQMNSL RAEDTAVYYCAR | LG-----------------------NY | WGQGT LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 04090 | 21-225_8D8 | VH3\|3-21\|D7\|7-27\|RF1\|JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | LG------------------------NY | WGQGTLVTVS |
| | VH3\|3-11/D4\|4-11\|RF2\|JH4 | | 32246 | QVQLVES-GGGLVKPGGSLRLSCAASG-FTFS | D-----YYMS | WIRQAPGKGLEWVS | YISSS---GSTIYYADSVKG | RFTISRDNAKNSLYLQMNSLRAEDTAVYYCAR | DYSNY-----------------YFDY | WGQGTLVTVS |
| iPS:4 33905 | 21-225_43E5 | VH3\|3-11/D4\|4-11\|RF2\|JH4 | | QVQLVES-GGGLVKPGGSLRLSCAASG-FTFS | D-----YYMI | WIRQAPGKGLEWVS | YISSS---GITKYYADSMKG | RFTISRDNAKNSLYLQMNSLRAEDTAVYYCAA | DI--------------------IY | WGQGTLVTVS |
| iPS:4 33913 | 21-225_43H8 | VH3\|3-11/D4\|4-11\|RF2\|JH4 | | QVQLVES-GGGLVKPGGSLRLSCAASG-ITFS | D-----YYMN | WIRQAPGKGLEWVS | YISSS---GRTIFYADSLKG | RFTISRDNAKNSLYLQMNSLRAEDTAVYYCAA | DI--------------------IY | WGQGTLVTVS |
| iPS:4 33949 | 21-225_45H2 | VH3\|3-11/D4\|4-11\|RF2\|JH4 | | QVQLVES-GGGLVKPGGSLRLSCAASG-FTFS | D-----YYMN | WIRQAPGKGLEWVS | YISSS---GITKYYADSVKG | RFTISRDNAKNSLYLQMNSLRAEDTAVYYCAA | DI--------------------IY | WGQGTLVTVS |
| iPS:4 33981 | 21-225_46E9 | VH3\|3-11/D4\|4-11\|RF2\|JH4 | | QVQLVES-GGGLVKPGGSLRLSCAASG-FTFS | D-----YYMN | WIRQAPGKGLEWVS | YINSN---GFTIYYADSVKG | RFTISRDNAKNSLYLQMNSLRAEDTAVYYCAA | DI--------------------IY | WGQGTLVTVS |
| iPS:4 33995 | 21-225_47H7 | VH3\|3-11/D4\|4-11\|RF2\|JH4 | | QVQLVES-GGGLVKPGGSLRLSCAASG-FTFS | D-----YYMI | WIRQAPGKGLEWVS | YINSN---GFTKYYADSVKG | RFTISRDNAKNSLYLQMNSLRAEDTAVYYCAA | DI--------------------VY | WGQGTLVTVS |
| iPS:4 34039 | 21-225_43B1 | VH3\|3-11/D4\|4-11\|RF2\|JH4 | | QVQLVES-GGGLVKPGGSLRLSCAASG-FTFS | D-----YYMN | WIRQAPGKGLEWVS | YINSN---GFTIYYADSVKG | RFTISRDNAKNSLYLQMNSLRAEDTAVYYCAA | DI--------------------IY | WGQGTRVTVS |
| iPS:4 34275 | 21-225_57F4 | VH3\|3-11/D4\|4-11\|RF2\|JH4 | | QVQLVES-GGGLVKPGGSLRLSCAASG-FIFS | D-----YYMN | WIRQAPGKGLEWVS | YISSS---GSTIYYADSVKG | RFTISRDNAKNSLYLQMNSLRAEDTAVYYCAR | DM--------------------IT | WGQGTLVTVS |
| | | Germline | | H_FR1 | H_CDR1 | H_FR2 | H_CDR2 | H_FR3 | H_CDR3 | H_FR4 |
| | VH3\|3-23\|D7\|7-27\|RF1\|JH3 | | 32247 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | LTGD---------------AFDI | WGQGTMVTVS |
| iPS:4 33915 | 21-225_43H9 | VH3\|3-23\|D7\|7-27\|RF1\|JH3 | | EVQLLES-GGGLVQPGGSLRLSCAASG-FTFS | S-----YAMS | WVRQAPGMGLEWVS | AISGS---GSNTFYADSVKG | RFTISRDNSKNTLYLHMNSLRAEDTAVYFCAK | RTPSD----------------VFDI | WGQGTMVTVS |
| iPS:4 33925 | 21-225_44F3 | VH3\|3-23\|D7\|7-27\|RF1\|JH3 | | EVHLLES-GGGLVQPGGSLRLSCAASG-FTFS | S-----YAMS | WVRQAPGKGLEWVS | ILSGG---GKTTYYADSVKG | RFTISRDNSKNTLFLQMNSLRAEDTAVYYCAK | RTPSD----------------AFDI | WGQGTMVTVS |

| ID | Clone | Germline | No. | H_FR1 | H_CDR1 | H_FR2 | H_CDR2 | H_FR3 | H_CDR3 | H_FR4 |
|---|---|---|---|---|---|---|---|---|---|---|
| iPS:4 33953 | 21-225_45H4 | VH3\|3-23/D7\|7-27\|RF1/JH3 | | EVQLLES-GGGLVQPGGSLRLSCAASG-FTFS | S-----YAMS | WVRQAPGM GLEWVS | AISGS---GSNTFYADS VKG | RFTISRDNSKNTLYLHMNSL RAEDTAVYFCAK | RIPSD---------------VFDI | WGQGT MVTVS S |
| iPS:4 33959 | 21-225_45C9 | VH3\|3-23/D7\|7-27\|RF1/JH3 | | EVQLLES-GGGLVQPGGSLRLSCAASG-FTFN | S-----YAMS | WVRQAPGK GLEWVS | VISGR---GGTTYYADS VKG | RFTISRDNSKNTLYLQMNSL RAEDTAVYYCAK | RIPSD---------------AFDI | WGQGT MVTVS S |
| iPS:4 35379 | 21-225_149B6 | VH3\|3-23/D7\|7-27\|RF1/JH3 | | EVQLLES-GGGLVQPGGSLRLSCAASG-FTFS | S-----YAMS | WVRQAPCK GLEWVS | VISGS---GGSTFYADS VKG | RFTISRDNSKNTLFLQMNSL RAEDTAVYYCAK | RIPED---------------VFDI | WGQGT MVTVS S |
| iPS:4 35787 | 21-225_180A3 | VH3\|3-23/D7\|7-27\|RF1/JH3 | | EVQLLES-GGGLEQPGGSLRLSCAASG-FTFS | S-----FAMN | WVRQAPCK GLEWVS | VISGR---GGNTFYADS VKG | RFTISRDNSKNTLFLQMNSL RAEDTAVYFCAK | RIGDD---------------VFDV | WGQGT MVTVS S |
| iPS:4 35809 | 21-225_182H5 | VH3\|3-23/D7\|7-27\|RF1/JH3 | | EVQLLES-GGGLVQPGGSLRLSCAASG-FTFS | S-----YAMS | WVRQAPGK GLEWVS | VISGR---GGTTFYADS VKG | RFTISRDNSKNTLYLQMNSL RAEDTAVYYCAK | RIGDD---------------VFDI | WGQGT MVTVS S |
| iPS:4 35889 | 21-225_186A11 | VH3\|3-23/D7\|7-27\|RF1/JH3 | | EVQLLES-GGGLVQPGGSLRLSCAASG-FTFS | S-----YAMS | WVRQAPGK GLEWVS | VISGR---GGTTFYADS VKG | RFTISRDNSKNTLYLQMNSL RAEDTAVYYCAK | RIGDD---------------VFDI | WGQGT MVTVS S |
| | Germline | | | H_FR1 | H_CDR1 | H_FR2 | H_CDR2 | H_FR3 | H_CDR3 | H_FR4 |
| | VH4\|4-59/D6\|6-13\|RF2/JH4 | | 32248 | QVQLQES-GPGLVKPSETLSLTCTVSG-GSIS | S-----YYWS | WIRQPPGK GLEWIG | YIYY----SGSTNYNPS LKS | RVTISVDTSKNQFSLKLSSV TAADTAVYYCAR | GIAAA---------------GYFDY | WGQGT LVTVS S |
| iPS:4 33931 | 21-225_44F6 | VH4\|4-59/D6\|6-13\|RF2/JH4 | | QVHLQES-GPGLVKPSETLSLTCTVSG-GSIS | S-----YYWS | WIRQPPGK GLEWIG | YIYY----SGNTNYNPS LKS | RVTISVDTSKNQFSLKLSSV TAADTAVYYCVR | GVAI---------------KNY | WGQGI LVTVS S |
| | Germline | | | H_FR1 | H_CDR1 | H_FR2 | H_CDR2 | H_FR3 | H_CDR3 | H_FR4 |
| | VH3\|3-23/D4\|4-17\|RF2/JH6 | | 32249 | EVQLLES-GGGLVQPGGSLRLSCAASG-FTFS | S-----YAMS | WVRQAPGK GLEWVS | AISGS---GGSTYYADS VKG | RFTISRDNSKNTLYLQMNSL RAEDTAVYYCAK | DYGDYYY---------YYYGMDV | WGQGT TVTVS S |
| iPS:4 33943 | 21-225_44E10 | VH3\|3-23/D4\|4-17\|RF2/JH6 | | EVQLLES-GGGLVQSGGSLRLSCAASG-FTFS | S-----YAMN | WVRQAPGK GLEWVS | GVVGS---GGRTYYADS VKG | RFIISRDNSKNTLYLQMNSL RAEDTAVYYCAK | DRGQWL----------LGGMDV | WGQGT TVTVS S |
| iPS:4 33989 | 21-225_47C7 | VH3\|3-23/D4\|4-17\|RF2/JH6 | | EVQLLES-GGGLVQPGGSLRLSCAASG-FTFS | N-----YAMS | WVRQAPGK GLEWVS | GISGS---GSRTYYADS VKG | RFTISRDNSKNTLYLQMNSL RAEDTAVYYCAK | DRGQWL----------IGGMDV | WGQGT TVTVS S |
| iPS:4 34133 | 21-225_54G3 | VH3\|3-23/D4\|4-17\|RF2/JH6 | | EVQLLES-GGGLVQPGGSLRLSCAASG-FTFS | T-----YAMS | WVRQAPGK GLEWVS | AISGS---GVNTFYADS VKG | RFTISRDNSKNTLFLQMNSL RAEDTAVYYCAK | LGKDYY----------YYGMDV | WGQGT TVTVS S |

| ID | Name | VH | No. | H_FR1 | H_CDR1 | H_FR2 | H_CDR2 | H_FR3 | H_CDR3 | H_FR4 |
|---|---|---|---|---|---|---|---|---|---|---|
| iPS:4 34221 | 21-225_60A11 | VH3\|3-23/D4\|4-17\|RF2/JH6 | | EVQLLES-GGGLVQPGGSLRLSCAASG-FTFS | N-----YAMT | WVRQAPGK GLEWVS | AISGS---GGNTFYADS VTG | RVTISRDNSKNTLYLQMNSL RAEDTAVYYCAK | LGKDYH----------------YYGMDV | WGQGT TVTVS S |
| iPS:4 34257 | 21-225_62F7 | VH3\|3-23/D4\|4-17\|RF2/JH6 | | EVQLLES-GGGLVQPGGSLRLSCAASG-FTFS | S-----YVMS | WVRQAPGK GLEWVS | GISGS---GAKTYYADS VKG | RFTISRDNSKNTLYLQMNSL RAEDTAVYYCAE | LGIDYY----------------YGMDV | WGQGT TVTVS S |
| iPS:4 34283 | 21-225_57F8 | VH3\|3-23/D4\|4-17\|RF2/JH6 | | EVQLLES-GGGLVQPGGSLRLSCVVSG-FTFS | N-----YAMS | WVRQAPGK GLEWVS | ASSGS---GGNTFYADS VTG | RVTISRDNSKNTLYLQMNSL RAEDTAVYYCAK | LGKDYH----------------YYGMDV | WGQGT TVTVS S |
| iPS:4 34385 | 21-225_66C10 | VH3\|3-23/D4\|4-17\|RF2/JH6 | | EVQLLES-GGGLVQPGGSLRLSCAASG-FTFS | S-----YVMS | WVRQAPGK GLEWVS | GISGS---GARTYYADS VKG | RFTISRDNSKNTLYLQMNSL RAEDTAVYYCAE | LGIDYY----------------YGMDV | WGQGT TVTVS S |
| iPS:4 35717 | 21-225_171A9 | VH3\|3-23/D4\|4-17\|RF2/JH6 | | EVQLLES-GGGLAQPGGSLRLSCAASG-FTFS | S-----YAMT | WVRQAPGK GLEWVS | AISGS---GGNTFNADS VKG | RFTISRDNSKNTLYLQMNSL RAEDTAVYYCAK | LGIDYY----------------YYGMDV | WGQGT TVTVS S |
| iPS:4 36528 | 21-225_224B1 | VH3\|3-23/D4\|4-17\|RF2/JH6 | | EVQVLES-GGGLVQPGGSLRLSCAASG-FTFS | S-----YAMS | WVRQAPGK GLEWVS | AISGS---GGNTYYADS VKG | RFTISRDNSKNTLYLHMNSL RAEDTAVYYCAG | EGGYYY----------------YYGVDV | WGQGT TVTVS S |
| iPS:3 93810 | 21-225_5A4 | VH3\|3-23/D4\|4-17\|RF2/JH6 | | EVQVLES-GGGLVQPGGSLRLSCAASG-LTFS | S-----SAMS | WVRQAPGK GLEWVS | AISGR---GGNTFYTDS VKG | RFTISRDNSKNTLYLQMNSL RAEDTAVYYCAK | LGKDYY----------------YYGMDV | WGQGT TVTVS S |
| | Germline | | | H_FR1 | H_CDR1 | H_FR2 | H_CDR2 | H_FR3 | H_CDR3 | H_FR4 |
| | VH3\|3-48/D5\|5-24\|RF3/JH6 | | 32250 | EVQLVES-GGGLVQPGGSLRLSCAASG-FTFS | S-----YSMN | WVRQAPGK GLEWVS | YISSS---SSTIYYADS VKG | RFTISRDNAKNSLYLQMNSL RDEDTAVYYCAR | RDGYNYYY----------------YYGMDV | WGQGT TVTVS S |
| iPS:4 33945 | 21-225_44C12 | VH3\|3-48/D5\|5-24\|RF3/JH6 | | EVQLVES-GGGLVQPGGSLRLSCAASG-FTFS | S-----YSVN | WVRQAPGK GLEWVS | YISSS---SSTIYYADS VKG | RFTISRDNAKNSLYLQMNSL RDEDTAVYYCAR | SGYSYAYY----------------YYYGMDV | WGQGT TVTVS S |
| | Germline | | | H_FR1 | H_CDR1 | H_FR2 | H_CDR2 | H_FR3 | H_CDR3 | H_FR4 |
| | VH3\|3-33/D6\|6-13\|RF2/JH4 | | 32251 | QVQLVES-GGGVVQPCRSLRLSCAASG-FTFS | S------YGMH | WVRQAPGK GLEWVA | VIWYD---GSNKYYADS VKG | RFTISRDNSKNTLYLQMNSL RAEDTAVYYCAR | GIAAA------------GYFDY | WGQGT LVTVS S |
| iPS:4 33947 | 21-225_44E12 | VH3\|3-33/D6\|6-13\|RF2/JH4 | | QVQLAES-GGGVVQPGRSLRLSCEASG-FTLS | S-----DDTH | WVRQPPGK GLEWVA | VIWFD---EYNKYYADS VKG | RFTISRDNSKNTLYLQMNSL RAEDAAVYYCAR | DLIAAA----------------GTGDY | WGQGT LVTVS S |
| iPS:4 33963 | 21-225_46B1 | VH3\|3-33/D6\|6-13\|RF2/JH4 | | QVHLAES-GGGVVQPGRSLRLSCEASG-FTLS | S-----DDSH | WVRQPPGK GLEWVA | VIWFD---EYTKYYADS VKG | RFTISRDNSKNTLYLQMNNL RAEDAAVYYCAR | DLIAAT----------------GTGDY | WGQGT LVTVS S |

EP 4 435 105 A2

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 33987 | 21-225_47A5 | VH3\|3-33/D6\|6-13\|RF2/JH4 | | QVQLVES-GGGVVQPGRSLRL SCAASG-FTFS | D-----DDTH | WVRQAPGK GLEWVA | VIWFD---GSNKYYADS VKG | RFTISRDNSKNTLYLQMNSL SAEDTAVYYCAR | DLIAAA-------------------GTVDY | WGQGT LVTVS S |
| iPS:4 36258 | 21-225_202F12 | VH3\|3-33/D6\|6-13\|RF2/JH4 | | QVQLVES-GGGVVQPGRSLRL SCEASG-FTFS | Y-----YGMH | WVRQAPGK GLEWVA | IIWYD---GSNXFYADS VKG | RFTISRDSSKNTLYLQMNSL RAEDTAVYYCAS | NIAAAA-------------------PYFDY | WGQGT LVTVS S |
| iPS:3 92646 | 21-225_20G2 | VH3\|3-33/D6\|6-13\|RF2/JH4 | | QVQLVES-GGGVVQPGRSLRL SCAASG-FTLS | S-----DDMH | WVRQEPGK GLEWVA | VIWFD---GSNKYYADS VKG | RFIMSRDNSKNTLYLQMNSL RACDTAVYYCAR | DLIAAA-------------------GTVDY | WGQGT LVTVS S |
| iPS:3 92750 | 21-225_20A10 | VH3\|3-33/D6\|6-13\|RF2/JH4 | | QVQLVES-GGGVVQPGRSLRL SCAASG-FTLS | S-----DDMH | WVRQAPGK GLEWVA | VIWFD---GSNKYYADS VKG | RFIISRDNSKNTLYLQMNSL RAEDTAMYYCAR | DLIAAA-------------------GTVDY | WGQGT LVTVS S |
| | **Germline** | | | **H_FR1** | **H_CDR1** | **H_FR2** | **H_CDR2** | **H_FR3** | **H_CDR3** | **H_FR4** |
| | **VH3\|3-23/D6\|6-19\|RF2/JH3** | | 32252 | EVQLLES-GGGLVQPGGSLRL SCAASG-FTFS | S-----YAMS | WVRQAPGK GLEWVS | AISGS---GGSTYYADS VKG | RFTISRDNSKNTLYLQMNSL RAEDTAVYYCAK | GIAVAG-------------------DAFDI | WGQGT MVTVS S |
| iPS:4 33999 | 21-225_48D1 | VH3\|3-23/D6\|6-19\|RF2/JH3 | | EVQLLES-GGGLVQPGGSLRL SCAASR-FTFS | S-----YAMS | WVRQAPGK GLEWVS | VISGR---GGSTFYADS VKG | RFTISRDNSKNTLYLQMNSL RAEDTAVYYCAR | RIAVAG-----------------NEAFDI | WGQGT MVTVS S |
| iPS:4 34003 | 21-225_48C3 | VH3\|3-23/D6\|6-19\|RF2/JH3 | | EVQLLES-GGGLVQPGGSLRL SCAASR-FTFS | S-----YAMS | WVRQAPGK GLEWVS | VISGR---GGSTFYADS VKG | RFTISRDNSKNTLYLQMNSL RAEDTAVYYCAR | RIAVAG-----------------NEAFDI | WGQGT MVTVS S |
| iPS:4 34037 | 21-225_49G12 | VH3\|3-23/D6\|6-19\|RF2/JH3 | | EVQLLES-GGGLVQPGGSLRL SCTASG-FTFS | S-----YAMS | WVRQAPGK GLEWVS | VISGS---GGTTFYADS VKG | RLTISRDNSKNTLYLQMNSL RAEDTAVYYCAR | RIAVAG-----------------NDAFDI | WGQGT MVTVS S |
| iPS:4 34041 | 21-225_50H8 | VH3\|3-23/D6\|6-19\|RF2/JH3 | | EVQLLES-GGGLVQPGGSLRL SCAASR-FTFS | S-----YAMS | WVRQAPGK GLEWVS | VISGR---GGTTFYADS VKG | RFTISRDNSKNTLYLQMNSL RAEDTAVYYCAR | RIAVAG-----------------NEAFDI | WGQGT MVTVS S |
| iPS:4 34045 | 21-225_50H10 | VH3\|3-23/D6\|6-19\|RF2/JH3 | | EGQLLES-GGGLVQPGGSLRL SCAASR-FTFS | S-----YAMS | WVRQAPGK GLEWVS | VISGR---GGSTFYADS VKG | RFTISRDNSKNTLYLQMNSL RAEDTAVYYCAR | RIAVAG-----------------NEAFDI | WGQGT MVTVS S |
| iPS:4 34073 | 21-225_51H10 | VH3\|3-23/D6\|6-19\|RF2/JH3 | | EVQLLES-GGGLVQPGGSLRL SCTASG-FTFS | S-----YAMS | WVRQAPGK GLEWVS | VISGS---GGTTFYADS VKG | RLTISRDNSKNTLYLQMNSL RAEDTAVYYCAR | RIAVAG-----------------NDAFDI | WGQGT MVTVS S |
| iPS:4 36212 | 21-225_200G1 | VH3\|3-23/D6\|6-19\|RF2/JH3 | | EVQLLES-GGGLVQPGGSLRL SCAASG-FTFS | S-----YAMS | WVRQAPGK GLEWVS | AISGR---GGNTFYADS VRG | RFTISRDNSKNTLYLQMNSL RAEDTAVYYCAR | RIAVDG-----------------YDAFDV | WGQGT MVTVS S |

| | | | | | | | | | | |
|---|---|---|---|---|---|---|---|---|---|---|
| iPS:3 92652 | 21-225_17C6 | VH3\|3-23/D6\|6-19\|RF2/JH3 | | EVQLLES-GGGLVQPGGSLRLSCAASE-FTFS | S-----YAMN | WVRQAPGKGLEWVS | VISGR---GGNTFYADSVKG | RFTISRDNSKNTLFLQMNSLRAEDTAVYYCAS | RLAVAG-------------------SEAFDI | WGQGTMVTVSS |
| iPS:3 92668 | 21-225_17B4 | VH3\|3-23/D6\|6-19\|RF2/JH3 | | EVQLLES-GGGLVQPGGSLRLSCAASE-FTFS | S-----YAMN | WVRQAPGKGLEWVS | VISGR---GGNTFYADSVKG | RFTISRDNSKNTLYLQMNSLRAEDTAVYYCAS | RLAVAG-------------------SEAFDI | WGQGTMVTVSS |
| iPS:3 92696 | 21-225_20A4 | VH3\|3-23/D6\|6-19\|RF2/JH3 | | EVQLLES-GGGLVQPGGSLRLSCAASG-FTFS | S-----YAMT | WVRQGPGMGLEWVS | VISGS---GGYTYNADSVKG | RFTISRDNSKNTLYLQMNSLRVEDTAVYYCAS | RIAVAG-------------------SEAFDI | WGQGTMVTVSS |
| iPS:3 92702 | 21-225_17F7 | VH3\|3-23/D6\|6-19\|RF2/JH3 | | EVQLLES-GGGLVQPGGSLRLSCAASE-FTFS | S-----YAMN | WVRQAPGKGLEWVS | IISGR---GGNTFYADSVKG | RFTISRDNSKNTLYLQMNSLRAEDTAVYYCAS | RLAVAG-------------------SEAFDI | WGQGTMVTVSS |
| iPS:3 92704 | 21-225_17F11 | VH3\|3-23/D6\|6-19\|RF2/JH3 | | EAQLLES-GGGLEQPGGSLRLSCAASG-FTFS | S-----YAMS | WVRQAPGKGLEWVS | VISGR---GGNTYSADSVKG | RFTISRDNSKNTLYLQMNSLRAEDTAVYYCAS | RLAVAG-------------------SEAFHI | WGQGTMVTVSS |
| iPS:3 92720 | 21-225_17A12 | VH3\|3-23/D6\|6-19\|RF2/JH3 | | EVQLLES-GGGLIQPGGSLRLSCAASE-FTFS | S-----YAMS | WVRQDPGKGLEWVS | IISGR---GGNAFYADSVKG | RFTISRDNSKNTLYLQMNSLRAEDTAVYYCAS | RIAVAG-------------------SEAFDI | WGQGTMVTVSS |
| iPS:3 92722 | 21-225_18E12 | VH3\|3-23/D6\|6-19\|RF2/JH3 | | EVQLLES-GGGLVQPGGSLRLSCAASE-FTFS | S-----YAMS | WVRQAPGTGLEWVS | IISGR---GGNTFYADSVKG | RFTISRDNSKNTLYLQMNSLRAEDTAVYYCAS | RLAVAG-------------------SEAFDI | WGQGTMVTVSS |
| iPS:3 92760 | 21-225_22G3 | VH3\|3-23/D6\|6-19\|RF2/JH3 | | EVQLLES-GGGLVQPGGSLRLSCAASE-FTFS | S-----YAMN | WVRQAPGKGLEWVS | IISGR---GVNTFYADSVKG | RFTISRDNSKNTLYLQMNSLRAEDTAVYYCAS | RLAVAG-------------------SEAFDI | WGQGTMVTVSS |
| iPS:3 92764 | 21-225_22G10 | VH3\|3-23/D6\|6-19\|RF2/JH3 | | EVQLLES-GGDLVQPGGSLRLSCTASG-FTFS | S-----YAMN | WVRQAPGKGLEWVS | VISGS---GGNTFYADSVKG | RFTISRDNSKNTLFLHMNSLRAEDTAVYYCAS | RMAVAG-------------------SEAFDI | WGQGTMVTVSS |
| iPS:3 92812 | 21-225_21F4 | VH3\|3-23/D6\|6-19\|RF2/JH3 | | EVQLLES-GGGLVQPGGSLRLSCAASE-FTFS | S-----YAMN | WVRQAPGKGLEWVS | VISGR---GGNTFYADSVKG | RFTISRDNSKNTLYLQMNSLRAEDTAVYYCAS | RLAVAG-------------------SEACDI | WGQGTMVTVSS |
| iPS:3 92816 | 21-225_22E4 | VH3\|3-23/D6\|6-19\|RF2/JH3 | | EVQLLES-GGGLVQPGGSLRLSCAASE-FTFS | S-----YAMS | WVRQAPGKGLEWVS | IISGR---GTNTFYADSVKG | RFTISRVNSKNTLYLQMNSLRAEDTAVYYCAS | RIAVAG-------------------SEAFDI | WGQGTMVTVSS |
| iPS:3 92852 | 21-225_21A2 | VH3\|3-23/D6\|6-19\|RF2/JH3 | | EVQLLES-GGGLVQPGGSLRLSCAASK-FTFS | S-----YAMN | WVRQAPGKGLEWIS | IISGR---GGNTFYADSVKG | RFTISRDNSKNTLYLQMNSLRAEDTAVYYCAS | RLAVAG-------------------SEAFDI | WGQGTMVTVSS |

| iPS:3 92878 | 21-225_22C5 | VH3\|3-23/D6\|6-19\|RF2/J H3 | | EVQLLES-GGGLVQVGGSLRL SCAASG-FTFS | S-----YAMS | WVRQAPGM GLEWVS | IISGS--- GGYTYYADS VKG | RFTISRDNSKNTLYLQMNSL RAEDTAVYYCAS | RIAVAG---------- --------SEAFDI | WGQGT MVTVS S |
| iPS:3 92902 | 21-225_22D5 | VH3\|3-23/D6\|6-19\|RF2/J H3 | | EVQLLES-GGGLVQPGGSLRL SCAASE-FTFS | S-----YAMS | WVRQAPGK GLEWVS | VISGR--- GGNTFYADS VKG | RFTISRDNSKNTLYLQMNSL RAEDTAVYYCAS | RIAVAG---------- --------SEAFDI | WGQGT MVTVS S |
| iPS:3 93824 | 21-225_10F12 | VH3\|3-23/D6\|6-19\|RF2/J H3 | | EVQLLES-GGGLVQPGGSLRL SCAASE-FTFS | S-----YAMS | WVRQAPGK GLEWVS | IISGR--- GGNTFYADS VKG | RFTISRDNSKNTLYLQMNSL RAEDTAVYYCAS | RVAVAG---------- --------SEAFAI | WGQGT MVTVS S |
| iPS:3 93848 | 21-225_4H2 | VH3\|3-23/D6\|6-19\|RF2/J H3 | | EVQLLES-GGGLVQPGGSLRL SCAASG-FTFS | G-----YAMN | WVRQAPGM GLEWVS | VISRS--- GGYTYYADS VKG | RFTISRDNSKNTLYLQMNSL RAEDTAVYYCAS | RLAVAG---------- --------SEAFDI | WGQGT MVTVS S |
| iPS:3 93862 | 21-225_5G2 | VH3\|3-23/D6\|6-19\|RF2/J H3 | | EVQLLES-GGGLVQPGGSLRL SCAASE-FTFS | S-----YAMS | WVRQAPGK GLEWVS | VISGR--- GVNTFYADS VKG | RFTISRDNSKNTLYLQMNSL RAEDTAVYYCAS | RIAVAG---------- --------SEAFDI | WGQGT MVTVS S |
| iPS:3 93888 | 21-225_3E3 | VH3\|3-23/D6\|6-19\|RF2/J H3 | | EVQLLES-GGTLVQPGGSLRL SCAASE-FTFS | S-----YVMS | WVRQAPGK GLEWVS | IISGR--- GGNTFYADS VKG | RFTISRDNSKNTLYLQMNSL RAEDTALYYCAS | RLAVAG---------- --------SEAFDI | WGQGT MVTVS S |
| iPS:3 93898 | 21-225_5F7 | VH3\|3-23/D6\|6-19\|RF2/J H3 | | EVQLLES-GGGLVQPGGSLRL SCAASE-FTFS | S-----YAMS | WVRQAPGK GLEWVS | IISGR--- GGNTFYADS VKG | RFTISRDNSKNTLYLQMNSL RAEDTAVYYCAS | RIAVAG---------- --------SEAFAI | WGQGT MVTVS S |
| iPS:3 93980 | 21-225_6D3 | VH3\|3-23/D6\|6-19\|RF2/J H3 | | EVQLLES-GGGLVQPGGSLRL SCAASE-FTFS | S-----YAMN | WVRQAPGK GLEWVS | VISGR--- GGNTFYADS VKG | RFTISRDNSKNTLYLQMNSL RAEDTAVYFCAS | RLAVAG---------- --------SEAFDI | WGQGT MVTVS S |
| iPS:3 94014 | 21-225_8G6 | VH3\|3-23/D6\|6-19\|RF2/J H3 | | EVQLLES-GGGLVQPGGSLRL SCVASE-FTFS | S-----YVMN | WVRQAPGK GLEWVS | VISGR--- GGNTFYADS VKG | RFTISRDNSKNTLYLQMNSL RAEDTAVYYCAS | RLAVAG---------- --------SEAFDI | WGQGT MVSVS S |
| iPS:3 94022 | 21-225_16H6 | VH3\|3-23/D6\|6-19\|RF2/J H3 | | EVQLLES-GGGLVQPGGSLRL SCAASG-FTFS | S-----YAMN | WVRQAPGM GLEWVS | VISRS--- GGYTYYADS VKG | RFTISRDNSKNTLYLQMNSL RAEDTAVYYCAS | RLAVAG---------- --------SEAFDI | WGQGT MVTVS S |
| iPS:3 94043 | 21-225_3B1 | VH3\|3-23/D6\|6-19\|RF2/J H3 | | EVQLLES-GGGLVQPGGSLRL SCAASE-FTFS | S-----YAMN | WVRQAPGK GLEWVS | VISGR--- GINTFYADS VKG | RFTISRDNSKNTLYLQMNSL RAEDTAVYYCAS | RLAVAG---------- --------SEAFDI | WGQGT MVTVS S |
| iPS:3 94077 | 21-225_8E12 | VH3\|3-23/D6\|6-19\|RF2/J H3 | | EVQLLES-GGGLVQPGGSLRL SCAASE-FTFS | S-----YAMS | WVRQAPGK GLEWVS | IISGR--- GGNTFYADS VKG | RFTISRDNSKNTLYLQMNSL RAEDTAVYYCAS | RMAVAG---------- --------SEAFDI | WGQGT MVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:3 94087 | 21-225_11A5 | VH3\|3-23/D6\|6-19\|RF2/JH3 | | EVQLLES-GGDLVQPGGSLRLSCAASE-FTFS | S-----YAMS | WVRQAPGK GLEWIS | VISGR--- GVNTFYADS VKG | RFTISRDNSKNTLYLQMNSL RAEDTAVYYCAS | RIAVAG--------------------SEAFDI | WGQGT MVTVS S |
| | Germline | | | H_FR1 | H_CDR1 | H_FR2 | H_CDR2 | H_FR3 | H_CDR3 | H_FR4 |
| | VH3\|3-11/D6\|6-6\|RF2/JH3 | | 32253 | QVQLVES-GGGLVKPGGSLRLSCAASG-FTFS | D-----YYMS | WIRQAPGK GLEWVS | YISSS--- GSTIYYADS VKG | RFTISRDNSKNSLYLQMNSL RAEDTAVYYCAR | STAAR--------------------DAFDI | WGQGT MVTVS S |
| iPS:4 34011 | 21-225_48B10 | VH3\|3-11/D6\|6-6\|RF2/JH3 | | QVQLVES-GGGLVKPGGSLRLSCAASG-FTFS | D-----YFMT | WIRQAPGQ GLEWVS | YISSA--- GGAIYYADS VKG | RFTISRDNAKNSLYLQMNSL RAEDTAVYYCAI | AVAAP--------------------GVFDI | WGQGT MVTVS S |
| | Germline | | | H_FR1 | H_CDR1 | H_FR2 | H_CDR2 | H_FR3 | H_CDR3 | H_FR4 |
| | VH3\|3-11/D6\|6-6\|RF2/JH4 | | 32254 | QVQLVES-GGGLVKPGGSLRLSCAASG-FTFS | D-----YYMS | WIRQAPCK GLEWVS | YISSS--- GSTIYYADS VKG | RFTISRDNAKNSLYLQMNSL RAEDTAVYYCAR | STAAR--------------------YFDY | WGQGT LVTVS S |
| iPS:4 34015 | 21-225_48F12 | VH3\|3-11/D6\|6-6\|RF2/JH4 | | QVQLVES-GGGLVKPGGSLRLSCAASG-FTFS | D-----YFMT | WIRQAPGQ GLEWVS | YISSA--- GGAIYYADS VKG | RFTISRDNAKNSLFLQMNSL RAEDTAVYYCAI | AVAAP--------------------GAFDI | WGQGT LVTVS S |
| iPS:4 34017 | 21-225_48G12 | VH3\|3-11/D6\|6-6\|RF2/JH4 | | QVQLVES-GGGLVKPGGSLRLSCAASG-FTFS | D-----YFMT | WIRQAPGQ GLEWVS | YISSA--- GGAIYYADS VKG | RFTISRDNAKNSLFLQMNSL RAEDTAVYYCAI | AVAAP--------------------GAFDI | WGQGT LVTVS S |
| | Germline | | | H_FR1 | H_CDR1 | H_FR2 | H_CDR2 | H_FR3 | H_CDR3 | H_FR4 |
| | VH3\|3-23/D6\|6-13\|RF2/JH4 | | 32255 | EVQLLES-GGGLVQPGGSLRLSCAASG-FTFS | S-----YAMS | WVRQAPGK GLEWVS | AISGS--- GGSTYYADS VKG | RFTISRDNSKNTLYLQMNSL RAEDTAVYYCAK | GIAAA--------------------GYFDY | WGQGT LVTVS S |
| iPS:4 34023 | 21-225_49F1 | VH3\|3-23/D6\|6-13\|RF2/JH4 | | EVQLLES-GGGLVQPGGSLRLSCAASG-FTFS | S-----YAMS | WVRQAPGK GLDWVS | VISGS--- GGSTFYADS VKG | RFTISRDNSKSTLYLQMNSL RAEDTAVYYCAS | AIAAAG--------------------AHYFDY | WGQGT LVTVS S |
| iPS:4 36246 | 21-225_201G6 | VH3\|3-23/D6\|6-13\|RF2/JH4 | | EVQLLES-GGGLVQPGGSLRLSCVASG-FTFS | S-----YAMS | WVRQAPGK GLEWVS | TISGS--- GVRTYYADS VKG | RFTISRDNSKNTLFLQMNSL RAEDTAVYYCAK | GGARSSG--------------------WFHFDY | WGQGT LVTVS S |
| iPS:4 36254 | 21-225_202C12 | VH3\|3-23/D6\|6-13\|RF2/JH4 | | EVQLLES-GGGLVQPGGSLRLSCAASG-FTFS | S-----YAMS | WVRQAPGK GLEWVS | TISGS--- GVRTYYADS VKG | RSTISRDNSKNTLFLQMNSL RAEDTAVYYCAK | GGARSSG--------------------WFHFDY | WGQGT LVTVS S |
| iPS:4 36304 | 21-225_201F3 | VH3\|3-23/D6\|6-13\|RF2/JH4 | | EVQLLES-GGGLVQPGGSQRLSCAASG-FTFS | S-----YAMS | WVRQAPGK GLEWVS | TISGS--- GVRTYYADS VKG | RFTISRDNSNNTLFLQMNSL RAEDTAVYYCAK | GGARSSG--------------------WFHFDY | WGQGT LVTVS S |
| iPS:4 36334 | 21-225_208G3 | VH3\|3-23/D6\|6-13\|RF2/JH4 | | EVQLLES-GGGLVQPGGSLRLSCAASG-FTFS | S-----YAMS | WVRQAPGK GLEWVS | TISGS--- GVRTYYADS VKG | RSTISRDNSKNTLFLQMNSL RAEDKAVYYCAK | GGARSSG--------------------WFHFDY | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-23/D6\|6-19\|RF2/JH5 | | 32256 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | GIAVAG---------- ---------NWFDP | WGQGT LVTVS S |
| iPS:4 34027 | 21-225_49H5 | VH3\|3-23/D6\|6-19\|RF2/J H5 | | EVQLLES-GGGLVQPGGSLRL SCAASG-FTFS | S-----YAMT | WVRQTPGK GLEWVS | AISGS--- GGNSFYADS VKG | RFTISRDNSENTLYLQMNSL RAEDTAVYYCAK | ARAVAG---------- --------SHWFDP | WGQGT LVTVS S |
| iPS:4 34061 | 21-225_51C7 | VH3\|3-23/D6\|6-19\|RF2/J H5 | | EVQLLES-GGGLVQPGGSLRL SCAASG-FTIS | N-----YAMT | WVRQTPGK GLEWVS | VISAS--- GGNSFYADS VKG | RFTISRDNSENTFYLQMNSL RAEDTAVYYCAK | ARAVAG---------- --------SHWFDP | WGQGT LVTVS S |
| iPS:4 34167 | 21-225_50F3 | VH3\|3-23/D6\|6-19\|RF2/J H5 | | EVQLLES-GGGLVQPGGSLRL SCAASG-FTFR | T-----YAMT | WVRQAPGK GLEWVS | AISGS--- GVNSFYADS VKG | RFTISRDNSENTLYLQMNSL RAEDTAVYYCAK | ARAVAG---------- --------SHWFDP | WGQGT LVTVS S |
| iPS:4 34455 | 21-225_72F5 | VH3\|3-23/D6\|6-19\|RF2/J H5 | | EVQLLES-GGGLVQPGGSLRL SCAASG-FTFS | S-----YAMI | WVRQAPGK GLEWVS | TISGS--- GGYTYSADS VKG | RFTISRDNSKNTLYLQMNSL RAEDTAVYYCAR | RIAVTG---------- --------TEWYDP | WGQGT LVTVS S |
| iPS:4 37232 | 21-225_63E1 | VH3\|3-23/D6\|6-19\|RF2/J H5 | | EVQMLES-GGGLGQSGGSLRL SCTASG-FTFT | T-----SAMS | WVRQAPGK GLEWVS | AISGS--- GANTFYADS VKG | RFTVTRDNSKNTLYLQMNSL TAEDTAVYYCVK | VIAVAG---------- --------GHFFDP | WGQGT LVTVS S |
| | VH3\|3-21/D1\|1-1\|RF2/JH4 | | 32257 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | VQLER----------- --------YFDY | WGQGT LVTVS S |
| iPS:4 34043 | 21-225_50G10 | VH3\|3-21/D1\|1-1\|RF2/JH 4 | | EVQLVES-GGGLVKPGGSLRL SCAASG-FTFS | S-----YSMN | WVRQAPGK GLEWVS | SISGS--- SSYIYYADS VKG | RFTISRDNAKNSLYLQMNSL RAEDTAVYYCAR | VAT------------- ----------FDY | WGQGT LVTVS S |
| iPS:4 34085 | 21-225_52E3 | VH3\|3-21/D1\|1-1\|RF2/JH 4 | | EVQLVES-GGGLVKPGGSLRL SCAASG-FTFS | S-----YKMN | WVRQAPGK GLEWVS | SISSG--- NSSIYYADS VKG | RFTISRDNAENSLYLQMNSL RAEDTAVYYCAR | VSS------------- ----------NDY | WGQGT LVTVS S |
| iPS:4 34101 | 21-225_52H12 | VH3\|3-21/D1\|1-1\|RF2/JH 4 | | EVQLVES-GGGLVKPGGSLRL SCAASG-FTFS | S-----YSMN | WVRQAPGK GLEWVS | SISGS--- STYIYYADS VKG | RFTVSRDNAKNSLYLQVNSL RAEDTAVYYCAR | VNS------------- ----------FDY | WGQGT LVTVS S |
| iPS:4 34187 | 21-225_56A5 | VH3\|3-21/D1\|1-1\|RF2/JH 4 | | EVQLVES-GGGLVKPGGSLRL SCAASG-FTFS | S-----YSMN | WVRQAPGK GLEWVS | SISGS--- SSYIYYADS VKG | RFTISRDNAKNSLYLQMNSL RAEDTAVYYCAR | VAT------------- ----------FDY | WGQGT LVTVS S |
| iPS:4 34265 | 21-225_57B2 | VH3\|3-21/D1\|1-1\|RF2/JH 4 | | EVQLVES-GGGLVKPGGSLRL SCAASG-FTFS | S-----YSMN | WVRQAPGK GLEWVS | SISGS--- SSYINYTDS VKG | RFTISRDNAKNSLYLQMNSL RAEDTAVYYCAR | VAG------------- ----------FDY | WGQGT LVTVS S |

| iPS:4 34439 | 21-225_70E12 | VH3\|3-21/D1\|1-1\|RF2/JH4 | | EVQLVES-GGGLVKPGGSLRL SCAASG-FTFS | S-----YSMN | WVRQAPGK GLEWVS | SISGN--- STYIYYTDS VKG | RFTISRDNAKNSLYLQMDSL TAEDTAVYYCAR | VAA------------------------FDC | WGQGT LVTVS S |
| iPS:4 35515 | 21-225_157E4 | VH3\|3-21/D1\|1-1\|RF2/JH4 | | EVQLVES-GGGLVKPGGSLRL SCAASG-FTFS | S-----YIMN | WVRQAPGK GLEWVS | SISGS--- SSYIYYADS VKG | RFTISRDNAKNSLYLQMNSL KAEDTAVYYCAR | VAT------------------------FDY | WGQGT LVTVS S |
| iPS:4 35535 | 21-225_157H10 | VH3\|3-21/D1\|1-1\|RF2/JH4 | | EVQLVES-GGGLVKPGGSLRL SCAASG-FTFS | S-----YIMN | WVRQAPGK GLEWVS | SISGS--- SSYINYADS VKG | RFTISRDNAKNSLYLQMNSL RAEDTAVYYCAR | VAH------------------------FDY | WGQGT LVTVS S |
| iPS:4 37224 | 21-225_56H1 | VH3\|3-21/D1\|1-1\|RF2/JH4 | | EVQLVES-GGGLVKPGGSLRL SCAASG-FTFS | N-----YRMN | WVRQGPCK GLEWIS | SISGS--- STDIYYADS VKG | RFTISRDNAKNSLYLQMNSL RAEDTAVYYCAR | VAS------------------------FDY | WGQGT LVTVS S |
| iPS:3 92620 | 21-225_17H5 | VH3\|3-21/D1\|1-1\|RF2/JH4 | | EVQLVES-GGGLVKPGGSLRL SCAASG-FTFS | S-----YSMN | WVRQAPGK GLEWVS | SISSS--- STYIYYADS VKG | RFTISRDNAKNSLYLQMNSL RAEDTAVYYCAR | VAS------------------------FDY | WGQGT LVTVS S |
| iPS:3 92632 | 21-225_16A11 | VH3\|3-21/D1\|1-1\|RF2/JH4 | | EVQLVES-GGGLVKPGGSLRL SCAASG-FTFS | S-----YSMN | WVRQAPGK GLEWVS | SISGS--- SSLIYYADS VKG | RFTISRDNAKNSLYLQMNSL RAEDTAVYYCAR | VAA------------------------FDY | WGQGT LVTVS S |
| iPS:3 92746 | 21-225_20H7 | VH3\|3-21/D1\|1-1\|RF2/JH4 | | EVHLVES-GGGLVKPGGSLRL SCAASG-FTFS | S-----YSMN | WVRQAPGK GLEWVS | SISGS--- SSFIYYADS VKG | RFTISRDNAKNSLYLQMNSL RAEDTAVYYCAR | VAA------------------------LDY | WGQGT LVTVS S |
| iPS:3 92782 | 21-225_22B12 | VH3\|3-21/D1\|1-1\|RF2/JH4 | | EVQLVES-GGGLVKPGGSLRL SCAASG-FTFS | S-----YSMN | WVRQAPGK GLEWVS | SISGS--- SSYTYYADS VKG | RFTISRDNAKNSLYLQMNSL RAEDTAVYYCAR | VAA------------------------LDS | WGQGT LVTVS S |
| iPS:3 92916 | 21-225_27C5 | VH3\|3-21/D1\|1-1\|RF2/JH4 | | EVQLVES-GGGLVKPGGSLRL SCAASG-FTFS | S-----YSMN | WVRQAPGK GLEWVS | STSSS--- DSYIYYADS VKG | RFTISRDNAKNSLYLQMNSL RAEDTAVYYCAR | VAS----------------FDC | WGQGT LVTVS S |
| iPS:3 92976 | 21-225_27H12 | VH3\|3-21/D1\|1-1\|RF2/JH4 | | EVQLVES-GGGLVKPGGSLRL SCAASG-FTFS | S-----YSLN | WVRQAPGK GLEWVS | SISGS--- SSNIYYTDS VKG | RFTISRDNAKNSLFLQMNSL RAEDTAVYYCAR | VAS------------------------FDY | WGQGT LVTVS S |
| iPS:3 93120 | 21-225_35H8 | VH3\|3-21/D1\|1-1\|RF2/JH4 | | EVQLVES-GGGLVKPGGSLRL SCAASG-FTFS | S-----YSMN | WVRQAPGK GLEWVS | SISGT--- GSFIYYADS VKG | RFTISRDNAKKSVYLQMNSL RAEDTAVYYCAR | VSG------------------------FDY | WGQGT LVTVS S |
| iPS:3 93836 | 21-225_15A2 | VH3\|3-21/D1\|1-1\|RF2/JH4 | | EVQLVES-GGGLVKPGGSLRL SCAASG-FTFS | S-----YTMN | WVRQAPGK GLEWVS | SISGS--- GSYIYYADS VKG | RFTISRDNAKNSLYLQMNAL RAEDTAVYYCAR | VAS------------------------FDY | WGQGT LVTVS S |

| iPS:3 93894 | 21-225_5E11 | VH3\|3-21/D1\|1-1\|RF2/JH4 | | EVQLVES-GGGLVKPGGSLRLSCAASG-FTFS | S-----YSMN | WVRQAPGKGLEWVS | SISGS---STYIYYADSVKG | RFTISRDNAKNSLYLQMNSLRAEDTAVFYCAR | VAS-------------------------FDY | WGQGTLVTVSS |
| iPS:3 93896 | 21-225_2A4 | VH3\|3-21/D1\|1-1\|RF2/JH4 | | EVQLVES-GGGLVKPGGSLRLSCAASG-FTFS | S-----YSMN | WVRQAPGKGLEWVS | SISGS---STYIYYADSVKG | RIAISRDNAKNSLYLQMNSLRAEDTAVYYCAR | VAS-------------------------FDY | WGQGTLVTVSS |
| iPS:3 93914 | 21-225_16B8 | VH3\|3-21/D1\|1-1\|RF2/JH4 | | EVQLVES-GGGLVKPGGSLRLSCAASG-FTFS | S-----YSMN | WVRQAPGKGLEWIS | SISGS---STYIYYADSVKG | RFTISRDNAKNSLYLQMNSLRAEDTAVYYCAR | VAS-------------------------FDY | WGQGTLVTVSS |
| iPS:3 93944 | 21-225_14D6 | VH3\|3-21/D1\|1-1\|RF2/JH4 | | EVQLVES-GGGLVKPGGSLRLSCAASG-FTFS | S-----YTMN | WVRQAPGKGLEWVS | SISGS---GSYIYYADSVKG | RFTMSRDNAKNSLYLQMNSLRAEDTAVYYCAR | VAA-------------------------FDS | WGQGTLVSVSS |
| iPS:3 93952 | 21-225_1F1 | VH3\|3-21/D1\|1-1\|RF2/JH4 | | EVQLVES-GGGLVKPGGSLRLSCAASG-FTFS | S-----YNMN | WVRQAPGKGLEWVS | SISSS---SSYIYYADSVKG | RFTISRDNAKNSLYLQMNSLRAEDTAVYYCAR | VNL-------------------------FDY | WGQGTLVTVSS |
| iPS:3 94033 | 21-225_5F4 | VH3\|3-21/D1\|1-1\|RF2/JH4 | | EVQLVES-GGGLVKPGGSLRLSCVASG-FTFS | S-----YSMN | WVRQAPGKGLEWVS | SISGS---SSYIYYADSVKG | RFTISRDNAKNSLFLQMNSLRAEDTAVYYCAR | VNN-------------------------FDY | WGQGTLVTVSS |
| iPS:3 94069 | 21-225_16H1 | VH3\|3-21/D1\|1-1\|RF2/JH4 | | EVQLVES-GGGLVKPGGSLRLSCAASG-FTFS | S-----YSMN | WVRQAPGKGLEWVS | SISGS---STYIYYADSVKG | RFTISRDNAKNSLYLQMNSLRAEDTAVYYCAR | VAA-------------------------FDY | WGQGTLVTVSS |
| iPS:3 98482 | 21-225_17H6 | VH3\|3-21/D1\|1-1\|RF2/JH4 | | EVQLVES-GGGLVKPGGSLRLSCAASG-FTFS | S-----YSMN | WVRQAPGRGLEWVS | SISGS---SSYIYYADSVKG | RFTIFRDNAKNSLYLQMNSLRAEDTAVYYCAR | VAS-------------------------FDY | WGQGTLVTVSS |
| iPS:3 98492 | 21-225_21F12 | VH3\|3-21/D1\|1-1\|RF2/JH4 | | EVQLVES-GGGLVKPGGSLRLSCAASG-FTFS | S-----YRMN | WVRQAPGKGLEWVS | SINSY---SSYIYYADSVKG | RFTISRDNAKNSLYLQMNSLRAEDTAVYYCAR | VAS-------------------------FDY | WGQGTLVTVSS |
| iPS:3 98500 | 21-225_23A11 | VH3\|3-21/D1\|1-1\|RF2/JH4 | | EVQLVES-GGGLVKPGGSLRLSCAASG-FTFS | S-----YSMN | WIRQAPGKGLEWVS | SISGS---STYIYYADSVKG | RIAISRDNAKNSLYLQMNSLRAEDTAVYYCAR | VAS-------------------------FDY | WGQGTLVTVSS |
| iPS:3 98526 | 21-225_32B3 | VH3\|3-21/D1\|1-1\|RF2/JH4 | | EVQLVES-GGGLVKPGGSLRLSCAASG-FTFS | S-----YSMN | WVRQAPGKGLEWVS | SISGS---STYIYYADSVKG | RFTISRDNAKNSLYLQMNSLRAEDTAVYYCAR | VAG-------------------------FDY | WGQGTLVTVSS |
| iPS:4 02221 | 21-225_2C12 | VH3\|3-21/D1\|1-1\|RF2/JH4 | | EVQLVES-GGGLVKPGGSLRLSCAASG-FTFS | S-----YSMN | WVRQAPGKGLEWVS | SISGS---SSYMYYADSVKG | RFTISRDNAKDSLYLQMNSLRAEDTAVYYCAR | VNL-------------------------FDY | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH1\|1-02/D2\|2-15\|RF2/JH6 | | 32258 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GYCSGGSCYS-----------YYYYYGMDV | WGQGTTVTVSS |
| iPS:4 34047 | 21-225_50A12 | VH1\|1-02/D2\|2-15\|RF2/JH6 | | QVQLVQS-GAEVKKPGASVKVSCKASG-YTFT | G-----HYIN | WVRQAPGQGPEWMA | WVNPN---SGGTNSAQKFQG | RVTMTRDTSISIVYMELSRLRSDDTAVYYCAR | GGQLGGFN----------------YYYYGMDV | WGQGTTVTVSS |
| iPS:4 34067 | 21-225_51H8 | VH1\|1-02/D2\|2-15\|RF2/JH6 | | QVQLVQS-GAEVKKPGASVKVSCKASG-YTFT | G-----HYMN | WVRQAPGQGLEWMG | WVNPN---SGCSNSAQQFQG | RVTMTRDTSISIVYMELSRLSSDDTAVYYCAR | GGQLGGFN----------------FYYYGMDV | WGQGTTVTVSS |
| iPS:4 34197 | 21-225_56C7 | VH1\|1-02/D2\|2-15\|RF2/JH6 | | QVQLVQS-GAEVKKPGASVKVSCKASG-YTFT | G-----HYIN | WVRQAPGQGLEWMA | WVNPN---SGGTNSAQKFQG | RVTMTRDTSISIVYMELSRLRSDDTAVYYCAR | GGQLGGFN----------------YYYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-21/D2\|2-15\|RF3/JH6 | | 32259 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DIVVVVAAT-----------YYYYYGMDV | WGQGTTVTVSS |
| iPS:4 34049 | 21-225_50B12 | VH3\|3-21/D2\|2-15\|RF3/JH6 | | EVQLVES-GGGLVKPGGSLRLSCAASG-FTFS | S-----HSMN | WVRQAPGKGLEWVS | SISSS---SNYIYYADSVKG | RFTISRDYAKNSLYLQMNSLRAEDTAVYYCAR | DRSIVVAGP----------------WDYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D1\|1-20\|RF1/JH3 | | 32260 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAR | GITGT-------------------DAFDI | WGQGTMVTVSS |
| iPS:4 34055 | 21-225_51B4 | VH3\|3-23/D1\|1-20\|RF1/JH3 | | EVQLLES-GGGLVQPGGSLSLSCAASG-FTFR | S-----YVMS | WVRQAPGKGLEWVS | AISGR---GSNTFYTDSVKG | RFTISRDNSKNTLYLQMNSLRAEDTAVYYCAK | GITGSH----------------GAFDI | WGQGTMVTVSS |
| | | Germline | | H_FR1 | H_CDR1 | H_FR2 | H_CDR2 | H_FR3 | H_CDR3 | H_FR4 |
| | VH3\|3-23/D7\|7-27\|RF1/JH4 | | 32261 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | LTGY-------------FDY | WGQGTLVTVSS |
| iPS:4 34059 | 21-225_51C5 | VH3\|3-23/D7\|7-27\|RF1/JH4 | | EVQLLES-GGGLVQPGGSLRLSCAASG-FTFS | S-----YVMS | WVRQTPGKGLEWVS | TMSGS---GGRTYYADSVNG | RFTVSRDNSKNTLYLQMSSLRAEDTAVYYCAR | VTA-----------------FDY | WGQGTLVTVSS |
| iPS:4 34213 | 21-225_60A4 | VH3\|3-23/D7\|7-27\|RF1/JH4 | | EVQLLES-GGGLVQPGGSLRLSCAASG-FTFS | S-----YVMS | WVRQAPGKGLEWVS | SISGS---GGWTNYADSVKG | RFTTSRDNSKNTLYLQMNSLRAEDTAVYYCAR | LTG-----------------FDY | WGQGTLVTVSS |
| iPS:4 34215 | 21-225_60F7 | VH3\|3-23/D7\|7-27\|RF1/JH4 | | EVQLLES-GGGLVQPGGSLRLSCAASG-FTFS | S-----YVMS | WVRQAPGKGLEWVS | GISGS---GNRTYYADSVKG | RFTISRDNSKNTLYLQMNSLRAEDSAVYYCGS | LG-----------------ID | WGQGTLVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 34241 | 21-225_61E6 | VH3\|3-23/D7\|7-27\|RF1/JH4 | | EVQLLES-GGGLVQPGGSLRL SCAASG-FTFR | S-----YAMS | WVRQAPGK GLEWVS | ATSGS--- GVNTFYADS VKG | RFTISRDNSKNTLYLQMNSL RAEDTAVYYCAK | LELG------------ ----------IFDY | WGQGT LVTVS S |
| iPS:4 34281 | 21-225_57B8 | VH3\|3-23/D7\|7-27\|RF1/JH4 | | EVQLLES-GGGLVQPGGSLRL SCAASG-FTFR | S-----YAMS | WVRQAPGK GLEWVS | AISGS--- GGNTFYADS VKG | RFTISRDNSKNTLYLQMNSL RAEDTAVYYCAK | LELG------------ ----------IFDY | WGQGT LVTVS S |
| iPS:4 34301 | 21-225_58F11 | VH3\|3-23/D7\|7-27\|RF1/JH4 | | EVQLLES-GGGLVQPGGSLRL SCAASG-FTFS | S-----YAMS | WVRQAPGK GLEWVS | AISGS--- GGNTFYADS VKG | RFTISRYNSKNTLYLQMNSL RAEDTAVYYCAK | FFGMVG---------- --------AGFFDY | WGQGT LVTVS S |
| iPS:4 35523 | 21-225_157G5 | VH3\|3-23/D7\|7-27\|RF1/JH4 | | EVQLLES-GGGLVQPGGSLRL SCAASG-FTFS | S-----YVMS | WVRQAPGK GLDWVS | AMSGS--- GGRTYYADS VKG | RFTISRDNSKNTLYLQMNSL RAEDTAVYYCAK | YIW------------- -----------NGY | WGQGT LVTVS S |
| iPS:4 35659 | 21-225_167D12 | VH3\|3-23/D7\|7-27\|RF1/JH4 | | EVQLLES-GGGLVQPGGSLRL SCAASG-FTFS | S-----YVMS | WVRQAPGK GLEWVS | AMSGS--- GGRTYYADS VKG | RFTISRDNSKNTLYLQMNSL RAEDTAVYYCAK | YIW------------- -----------NGY | WGQGT LVTVS S |
| iPS:4 35765 | 21-225_177D3 | VH3\|3-23/D7\|7-27\|RF1/JH4 | | EVQLLES-GGGLVQPGGSLRL SCAASG-FTFS | S-----YVMN | WVRQAPGK GLEWVS | GMSGS--- GGRTYYADS VKD | RFTISRDNSKNTLSLQMNSL RAEDTAVYYCAR | VIF------------- -----------FDY | WGQGT LVTVS S |
| iPS:4 37216 | 21-225_51D5 | VH3\|3-23/D7\|7-27\|RF1/JH4 | | EVQLLES-GGGLVQPGGSLRL SCAASG-FTFS | S-----YVMS | WVRQTPGK GLEWVS | TMSGS--- GGRTYYADS VNG | RFTVSRDNSKNTLYLQMSSL RAEDTAVYYCAR | VTA------------- -----------FDY | WGQGT LVTVS S |
| | Germline | | | H_FR1 | H_CDR1 | H_FR2 | H_CDR2 | H_FR3 | H_CDR3 | H_FR4 |
| | VH3\|3-21/D5\|5-24\|RF2/JH4 | | 32262 & 32263 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | *RWLQ---------- ----------LYFDY | WGQGT LVTVS S |
| iPS:4 34063 | 21-225_51G7 | VH3\|3-21/D5\|5-24\|RF2/JH4 | | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | ARL------------- -----------DY | WGQGT LVTVS S |
| | Germline | | | H_FR1 | H_CDR1 | H_FR2 | H_CDR2 | H_FR3 | H_CDR3 | H_FR4 |
| | VH3\|3-23/D7\|7-27\|RF3/JH4 | | 32264 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | NWGY------------ -----------FDY | WGQGT LVTVS S |
| iPS:4 34083 | 21-225_52H2 | VH3\|3-23/D7\|7-27\|RF3/JH4 | | EVQLLES-GGGLVQPGGSLRL SCAASG-FTFS | R-----NAMS | WVRQAPGM GLEWVS | AISGR--- GGNTFYADS VKG | RFTVSRDNSKNTLFLQMNSL RAEDTAVYYCAK | NGREQ----------- ----------WLDY | WGQGT LVTVS S |
| | Germline | | | H_FR1 | H_CDR1 | H_FR2 | H_CDR2 | H_FR3 | H_CDR3 | H_FR4 |
| | VH3\|3-30.3/D6\|6-6\|RF2/JH6 | | 32265 | QVQLVES-GGGVVQPGRSLRL SCAASG-FTFS | S-----YAMH | WVRQAPGK GLEWVA | VISYD--- GSNKYYADS VKG | RFTISRDNSKNTLYLQMNSL RAEDTAVYYCAR | SIAARYY--------- --------YYYGMDV | WGQGT TVTVS S |

2934

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 34087 | 21-225_52F6 | VH3\|3-30.3\|D6\|6-6\|RF2/JH6 | | QVQLVES-GGGVVQPGRSLRLSCAASG-FTFS | S-----YGMH | WVRQAPGK GLEWVA | IISYG---GSNKYYADS VKG | RFTISRDNSKNTLYLQMNSL RADDTAVYYCAR | RSAARP------------------GYGMDV | WGQGT TVTVS S |
| iPS:4 34111 | 21-225_53H2 | VH3\|3-30.3\|D6\|6-6\|RF2/JH6 | | QVQLVES-GGGVVQPGRSLRLSCAASG-FTFS | S-----YGMH | WVRQAPGK GPEWVA | VISYG---GSNKYHADS VKG | RFTISRDNSKNTLYLQMNSL RAEDTAVYYCAR | RGAARP------------------GYGMDV | WGQGT TVTVS S |
| iPS:4 34121 | 21-225_53F6 | VH3\|3-30.3\|D6\|6-6\|RF2/JH6 | | QVQLVES-GGGVVQPGRSLRLSCAASG-FTFS | S-----YGMH | WVRQAPGK GLEWVA | VISYG---GSNKYDADS VKG | RFTISRDNSKNTLYLQMTSL RAEDTAVYYCAR | RRAARP------------------GYGMDV | WGQGT TVTVS S |
| iPS:4 34163 | 21-225_50H1 | VH3\|3-30.3\|D6\|6-6\|RF2/JH6 | | QVQLVES-GGGVVQPGRSLRLSCAASG-FTFS | S-----YGMH | WVRQAPGK GLEWVS | IISYG---GSNKYDADS VKG | RFTISRDNSKNTLYLQMTSL RAEDTAVYYCAR | RRAARP------------------GYGMDV | WGQGI TVTVS S |
| iPS:3 94035 | 21-225_5G9 | VH3\|3-30.3\|D6\|6-6\|RF2/JH6 | | QVQLVES-GGGVVQPGRSLRLSCAASG-FTFS | N-----YGMH | WVRQAPGK GLEWVA | IISYA---GSNKYYADS VKG | RFTISRDNSKNTLYLQMNSL RAEDTAVYYCVR | RITARL------------------YYGMDV | WGQGT TVTVS S |
| | Germline | | | H_FR1 | H_CDR1 | H_FR2 | H_CDR2 | H_FR3 | H_CDR3 | H_FR4 |
| VH3\|3-33/D2\|2-8\|RF1/JH4 | | | 32266 & 32267 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAR | RILY*WC---------------MLYYFDY | WGQGT LVTVS S |
| iPS:4 34095 | 21-225_52F10 | VH3\|3-33/D2\|2-8\|RF1/JH4 | | QVQLVES-GGGVVQPGRSLRLSCAASG-FTFS | F-----YGMH | WVRQAPGK GLEWVA | VIWDD---GSNKYYADS VKG | RFTISRDNSKNTLFLQMSL RAEDTAVYYCAR | DSLYSS---------------SWLFDY | WGQGT LVTVS S |
| | Germline | | | H_FR1 | H_CDR1 | H_FR2 | H_CDR2 | H_FR3 | H_CDR3 | H_FR4 |
| VH3\|3-23-23/D4\|4-23\|RF3/JH4 | | | 32268 | EVQLLES-GGGLVQPGGSLRLSCAASG-FTFS | S-----YAMS | WVRQAPGK GLEWVS | AISGS---GGSTYYADS VKG | RFTISRDNSKNTLYLQMNSL RAEDTAVYYCAK | TTVVT---------------YFDY | WGQGT LVTVS S |
| iPS:4 34107 | 21-225_53E2 | VH3\|3-23/D4\|4-23\|RF3/JH4 | | EVQLLES-GGGLIQPGGSLRLSCAASG-FTFR | S-----YAMS | WVRQAPGK GLEWVS | AISGS---GGNTFYADS VKG | RFTISRDNSKNTLYLQMNTL RADDTAVYYCAK | KVVDTA---------------MALDY | WGQGT LVTVS S |
| iPS:4 34181 | 21-225_56B2 | VH3\|3-23/D4\|4-23\|RF3/JH4 | | EVQLLES-GGGLIQPGGSLRLSCAASG-FTFR | S-----YAMS | WVRQAPGK GLEWVS | AISGS---GGNTFYADS VKG | RFTISRDNSKNTLYLQMNTL RADDTAVYYCAK | KVVDTA---------------MALDY | WGQGT LVTVS S |
| | Germline | | | H_FR1 | H_CDR1 | H_FR2 | H_CDR2 | H_FR3 | H_CDR3 | H_FR4 |
| VH3\|3-23/D1\|1-26\|RF1/JH3 | | | 32269 | EVQLLES-GGGLVQPGGSLRLSCAASG-FTFS | S-----YAMS | WVRQAPGK GLEWVS | AISGS---GGSTYYADS VKG | RFTISRDNSKNTLYLQMNSL RAEDTAVYYCAK | GIVGAT---------------DAFDI | WGQGT MVTVS S |
| iPS:4 34117 | 21-225_53C6 | VH3\|3-23/D1\|1-26\|RF1/JH3 | | EVQLLES-GGGLVQPGGSLRLSCAASG-FTFS | S-----YAMN | WVRQAPGK GLEWVS | AISGS---GGATYYADS VKG | RFTISRDNSKNTLYLQMNSL RAEDTAVYYCAK | PLVGAH---------------DAFEI | WGQGT MVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| iPS:3 92984 | 21-225_30E11 | VH3\|3-23/D1\|1-26\|RF1/JH3 | EVQLLES-GGDMVQPGGSLRLSCAASG-FTFS | I-----YAMS | WVRQAPGKGLEWVS | VISGS---GGSSFYADSVKG | RFTISRDNSKNTLYLQMNSLRAEDTAVYYCTK | DRVKAH-------------------DGFDI | WGQGTMVTVSS |
| iPS:3 93114 | 21-225_33G12 | VH3\|3-23/D1\|1-26\|RF1/JH3 | EVQLLES-GGDMVQPGGSLRLSCAASG-FTFS | S-----YAMS | WVRQAPGKGLEWVS | VISGS---GGSSFYADSVKG | RFTISRDNSKNTLYLQMNSLRAEDTAVYCAK | DRVRAH-------------------DGFDI | WGQGTMVTVSS |
| | | Germline | H_FR1 | H_CDR1 | H_FR2 | H_CDR2 | H_FR3 | H_CDR3 | H_FR4 |
| | VH3\|3-33/D5\|5-24\|RF2/JH4 | 32270 & 32271 | QVQLVES-GGGVVQPGRSLRLSCAASG-FTFS | S------YGMH | WVRQAPGKGLEWVA | VIWYD---GSNKYYADSVKG | RFTISRDNSKNTLYLQMNSLRAEDTAVYYCAR | *RWLQ------------------LYFDY | WGQGTLVTVSS |
| iPS:4 34127 | 21-225_53H8 | VH3\|3-33/D5\|5-24\|RF2/JH4 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | ARIG--------------------YFDS | WGQGTLVTVSS |
| | | Germline | H_FR1 | H_CDR1 | H_FR2 | H_CDR2 | H_FR3 | H_CDR3 | H_FR4 |
| | VH3\|3-23/D4\|4-17\|RF2/JH4 | 32272 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | DYGDY-------------------YFDY | WGQGTLVTVSS |
| iPS:4 34147 | 21-225_55E1 | VH3\|3-23/D4\|4-17\|RF2/JH4 | EVQLLES-GGGLVQPGGSLRLSCAASG-FTFS | S-----YAMS | WVRQAPGKGLEWVS | AISGR---GSSTFYADSVKG | RFTISRDNSKNTLYLQMNSLRAEDTAVFYCAK | DHGIVG-----------------TIYFDY | WGQGTLVTVSS |
| iPS:4 35303 | 21-225_146A6 | VH3\|3-23/D4\|4-17\|RF2/JH4 | EVQLLES-GGGLVQPGGSLRLSCAASG-FTFN | S-----YAMN | WVRQAPGKGLEWVS | AISGS---GGNTFYADSVKG | RFTISRDNSKNTLYLQMNSLRAEDTAVYYCAK | KDNDYVW---------------GSPYFDY | WGQGTLVTVSS |
| iPS:4 35335 | 21-225_147D10 | VH3\|3-23/D4\|4-17\|RF2/JH4 | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | KDYDYVW---------------GSPYFDY | WGQGTLVTVSS |
| iPS:4 35339 | 21-225_147D12 | VH3\|3-23/D4\|4-17\|RF2/JH4 | EVQLLES-GGGLVQSGGSLRLSCAASG-FTFS | S-----YAMS | WVRQAPGKGLEWVS | AISGR---GGNTFYADSVKG | RFTISRDNSKNTLYLQMNSLRAEDTAVYYCAK | KDYDYVW---------------GSPYFDY | WGQGTLVTVSS |
| iPS:4 35343 | 21-225_148E2 | VH3\|3-23/D4\|4-17\|RF2/JH4 | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | KDYDYVW---------------GSPYFDY | WGQGTLVTVSS |
| iPS:4 35381 | 21-225_149C6 | VH3\|3-23/D4\|4-17\|RF2/JH4 | EVQLLES-GGGLVQPGGSLRLSCAASG-FTFS | S-----YAMS | WVRQAPGKGLEWVS | AISGS---GGNTFYADSVKG | RFTISRDNSKNTLYLHMNSLRAEDTAVYYCAK | KDYDYVW---------------GSPYFDY | WGQGTLVTVSS |
| iPS:4 35391 | 21-225_149F8 | VH3\|3-23/D4\|4-17\|RF2/JH4 | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | KDYDYVW---------------GSPYFDY | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 35395 | 21-225_149D11 | VH3\|3-23/D4\|4-17\|RF2/JH4 | | EVQLLES-GGGLVQPGGSLRLSCAASG-FTFS | S-----YAMS | WVRQAPGKGLEWVS | AISGR---GGNTFYADSVKG | RFTISRDNSKNTLYRQMNSLRAEDTAVYYCAK | KƆYDYVW----------------GSPYFDY | WGQGTLVTVSS |
| iPS:4 35403 | 21-225_150C5 | VH3\|3-23/D4\|4-17\|RF2/JH4 | | EVQLLES-GGGLVQPGGSLRLSCAASG-FTFS | S-----YAMS | WVRQAPGKGLEWVS | AISGS---GGNTFYADSVKG | RFTISRDNSKNTLYLQMNSLRAEDTAVYYCAK | KƆYDYVW----------------GSPYFDY | WGQGTLVTVSS |
| iPS:4 35447 | 21-225_152H7 | VH3\|3-23/D4\|4-17\|RF2/JH4 | | EVQLLES-GGGLVQPGGSLRLSCAASG-FTFN | S-----YAMN | WVRQAPGKGLEWVS | AISGS---GGNTFYADSVKG | RFTISRDNSKNTLYLQMNSLRAEDTAVYYCAK | KƆNDYVW----------------GSPYFDY | WGQGTLVTVSS |
| iPS:4 35453 | 21-225_152G10 | VH3\|3-23/D4\|4-17\|RF2/JH4 | | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | KƆYDYVW----------------GSPYFDY | WGQGTLVTVSS |
| iPS:4 35483 | 21-225_155A4 | VH3\|3-23/D4\|4-17\|RF2/JH4 | | EVQLLES-GGGLVQPGGSLRLSCAASG-FTFN | S-----YAMS | WVRQAPGKGLEWVS | AISGS---GGNTFYADSVKG | RFTISRDNSKNTLYLQMNSLRAEDTAVYYCAK | KƆYDYVW----------------GSPYFDY | WGQGTLVTVSS |
| iPS:4 35485 | 21-225_155B4 | VH3\|3-23/D4\|4-17\|RF2/JH4 | | EVQLLES-GGGLVQPGGSLRLSCAASG-FTFS | S-----YAMS | WVRQAPGKGLEWVS | AISGS---GGNTFYADSVKG | RFTISRDNSKNTLYLQMNSLRAEDTAVYYCAK | KƆYDYVW----------------GSPYFDY | WGQGTLVTVSS |
| iPS:4 35777 | 21-225_178F7 | VH3\|3-23/D4\|4-17\|RF2/JH4 | | EVHLLES-GGGLVQTGGSLRLSCAASG-FTFS | S-----YAMT | WVRQAPGKGLEWVS | VISGS---GGNTFYADSVKG | RFTISRDNSKNTLYLQMNSLRAEDTAVYYCAR | RYGD--------------------YFDY | WGQGTLVTVSS |
| iPS:4 35783 | 21-225_179G1 | VH3\|3-23/D4\|4-17\|RF2/JH4 | | EVHLLES-GGGLVQTGGSLRLSCAASG-FTFS | S-----YAMT | WVRQAPGKGLEWVS | VISGF---GGNTFYADSVKG | RFTISRDNSKNTLYLQMNSLRAEDTAVYYCAR | RYGD--------------------YFDY | WGQGTLVTVSS |
| iPS:4 35833 | 21-225_190D12 | VH3\|3-23/D4\|4-17\|RF2/JH4 | | EVQLLES-GGGLVQPGGSLRLSCAASG-FTFS | S-----YAMS | WVRQAPGKGLEWDS | AIIGN---GGRTYYADSVKG | RFTISRDNSKNTLYLQMNSLRAEDTAVYYCAK | DMGRYS----------------YGFFDY | WGQGTLVTVSS |
| iPS:4 36156 | 21-225_197C8 | VH3\|3-23/D4\|4-17\|RF2/JH4 | | EVQLLES-GGGLVQPGGSLRLSCAASG-FTFS | S-----SAMT | WVRQAPGKGLEWVS | AIIGN---GGRAYYADSVKG | RFTISRDNSKNTLYLQMNSLRTEDTAVYYCAK | DRGYSRIA----------------VAGTFDY | WGQGTLVTVSS |
| | VH3\|3-21/D1\|1-1\|RF1/JH4 | | 32273 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GTTGT----------------YFDY | WGQGTLVTVSS |
| iPS:4 34157 | 21-225_55D4 | VH3\|3-21/D1\|1-1\|RF1/JH4 | | EVQLVES-GGGLVKPGGSLRLSCAASG-FTFN | S-----YRMN | WVRQAPGKGLEWVS | SISSS---SNHIDYADSVKG | RFTISRDNAKNSLYLQMNSLRAEDTALYYCAR | GT----------------DY | WGQGTLVSVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 34243 | 21-225_62C1 | VH3\|3-21/D1\|1-1\|RF1/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAI | FG----------------------VD | WGQGTLVTVSS |
| iPS:4 35505 | 21-225_157C1 | VH3\|3-21/D1\|1-1\|RF1/JH4 | | EVQLVES-GGGLVKPGGSLRLSCAASG-FTFS | S-----YRMN | WVRQAPGKGLEWVS | SMSNS---SSSIYYADSVKG | RFTISRDNAKNSLYLQMNSLRAEDTAVYYCAR | QAA-------------------QDY | WGQGTLVTVSS |
| iPS:3 92966 | 21-225_32G3 | VH3\|3-21/D1\|1-1\|RF1/JH4 | | EVQLVES-GGGLVKPGGSLRLSCAASG-FTFS | S-----YSMN | WVRQAPGKGLEWVS | SISGS---SSYIYYADSVKG | RFTISRDNAKNSLYLQINSLRAEDTAVYYCAR | GNIA------------------RDY | WGQGTLVTVSS |
| | Germline | | | H_FR1 | H_CDR1 | H_FR2 | H_CDR2 | H_FR3 | H_CDR3 | H_FR4 |
| | VH3\|3-33/D7\|7-27\|RF3/JH4 | | 32274 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | NWCY----------------FDY | WGQGTLVTVSS |
| iPS:4 34159 | 21-225_55B8 | VH3\|3-33/D7\|7-27\|RF3/JH4 | | QVQLVES-GGGVVQPGRSLRLSCAASG-FTFS | D-----YGMH | WVRQAPGKGLEWVA | VIWYD---ENNKYYADSVKG | RFTISRDNSKNTLYLQMNSLRAEDTAVYYCTR | EWF-------------------DY | WGQGTLVTVSS |
| iPS:3 93026 | 21-225_32B6 | VH3\|3-33/D7\|7-27\|RF3/JH4 | | QVQLVES-GGGVVQPGRSLRLSCAASG-FTFS | D-----YGMH | WVRQAPDKGLEWVA | VIWYD---ENTKYYADSVKG | RFTISRDNSKNTLYLQMNSLRAEDTAVYYCAR | EWG-------------------DY | WGQGTLVTVSS |
| | Germline | | | H_FR1 | H_CDR1 | H_FR2 | H_CDR2 | H_FR3 | H_CDR3 | H_FR4 |
| | VH3\|3-33/D6\|6-6\|RF3/JH4 | | 32275 & 32276 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | V*QLV-----------YFDY | WGQGTLVTVSS |
| iPS:4 34165 | 21-225_50F2 | VH3\|3-33/D6\|6-6\|RF3/JH4 | | QVQLVES-GGGVVQPGRSLRLSCAASA-FTFS | S-----YGMH | WVRQTPGKGLEWVA | VIWHD---GSNKYYADSVKG | RFTISRDNSKNTLYLQMNSLRAEDTAVYYCAR | DEQLG------------TFDY | WGQGTLVTVSS |
| iPS:4 34191 | 21-225_56B6 | VH3\|3-33/D6\|6-6\|RF3/JH4 | | QVQLVES-GGGVVQPGRSLRLSCAASG-FTFS | S-----YGMH | WVRQAPGKGLEWVA | VIWHD---GSNKYYVDSVKG | RFTISRDNSKNTLYLQMNSLRAEDTAVYYCAR | DEQLG------------TFDY | WGQGTLVTVSS |
| | Germline | | | H_FR1 | H_CDR1 | H_FR2 | H_CDR2 | H_FR3 | H_CDR3 | H_FR4 |
| | VH1\|1-46/D4\|4-17\|RF2/JH6 | | 32277 | QVQLVQS-GAEVKKPGASVKVSCKASG-YTFT | S-----YYMH | WVRQAPGQGLEWMG | IINPSGGSTSYAQKFQG | RVTMTRDTSTSTVYMELSSLRSEDTAVYYCAR | DYGDYYY--------YYYGMDV | WGQGTTVTVSS |
| iPS:4 34171 | 21-225_50G4 | VH1\|1-46/D4\|4-17\|RF2/JH6 | | QVQLVQS-GAEVKEPGASVKVSCKASG-YTFT | S-----YYIH | WVRQAPGQGLEWMG | VINPS---NGRTSYAQKFQG | RVTMTRDTSTSTVYMELSSLRSEDTAVYYCAR | DRGDGYY-------FYYGMDV | WGQGTTVTVSS |
| | Germline | | | H_FR1 | H_CDR1 | H_FR2 | H_CDR2 | H_FR3 | H_CDR3 | H_FR4 |
| | VH3\|3-30.3/D4\|4-17\|RF2/JH3 | | 32278 | QVQLVES-GGGVVQPGRSLRLSCAASG-FTFS | S-----YAMH | WVRQAPGKGLEWVA | VLSYD---GSNKYYADSVKG | RFTISRDNSKNTLYLQMNSLRAEDTAVYYCAR | DYGDY----------DAFDI | WGQGTMVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 34175 | 21-225_55A11 | VH3\|3-30.3/D4\|4-17\|RF2/JH3 | | QVQLVES-GGGVVQPGRSLRL SCAASG-FTFS | S-----YGMH | WVRQTPGK GLEWVA | VISYV---GSTKYYADS VRG | RFTISRDNSKNTLYLQMNSL RTEDTAVYYCAR | GRGRYSDY--------------GHDAFDI | WGQGT MVTVS S |
| | Germline | H_FR1 | H_CDR1 | H_FR2 | H_CDR2 | H_FR3 | | H_CDR3 | H_FR4 | |
| | VH1\|1-02/D1\|1-1\|RF1/JH2 | | 32279 | QVQLVQS-GAEVKKPGASVKV SCKASG-YTFT | G-----YYMH | WVRQAPGQ GLEWMG | WINPN---SGGTNYAQK FQG | RVTMTRDTSISTAYMELSRL RSDDTAVYYCAR | GITGTY-------------WYFDL | WGRGT LVTVS S |
| iPS:4 34193 | 21-225_56C6 | VH1\|1-02/D1\|1-1\|RF1/JH2 | | QVQLVQS-GAEVKKPGASVKV SCKASG-YTFT | D-----YHMH | WVRQAPGQ GLEWMG | WINPN---RGGTNYVQK FQG | RVAMTNDTSISTAYMELSGL RSDDTAVYYCAR | DGTS-------------SFDY | WGRGT LVTVS S |
| | Germline | H_FR1 | H_CDR1 | H_FR2 | H_CDR2 | H_FR3 | | H_CDR3 | H_FR4 | |
| | VH1\|1-02/D5\|5-24\|RF1/JH4 | | 32280 | QVQLVQS-GAEVKKPGASVKV SCKASG-YTFT | G-----YYMH | WVRQAPGQ GLEWMG | WINPN---SGGTNYAQK | RVTMTRDTSISTAYMELSRL RSDDTAVYYCAR | VEMAT-----------IYFDY | WGQGT LVTVS S |
| iPS:4 34195 | 21-225_56F6 | VH1\|1-02/D5\|5-24\|RF1/JH4 | | QVQLVQS-GAEVKKPGASVKV SCKASG-FTFT | D-----YYMH | WVRQAPGQ GLEWMG | WINPN---SGGTNYAQR FQG | RVTMTRDTSISTAYMELSRL RSDDTAVYYCTR | EGATRP----------TGFDY | WGQGT LVTVS S |
| | Germline | H_FR1 | H_CDR1 | H_FR2 | H_CDR2 | H_FR3 | | H_CDR3 | H_FR4 | |
| | VH3\|3-33/D1\|1-1\|RF2/JH4 | | 32281 | QVQLVES-GGGVVQPGRSLRL SCAASG-FTFS | S-----YGMH | WVRQAPGK GLEWVA | VIWYD---GSNKYYADS VKG | RFTISRDNSKNTLYLQMNSL RAEDTAVYYCAR | VQLER-----------YFDY | WGQGT LVTVS S |
| iPS:4 34203 | 21-225_60E2 | VH3\|3-33/D1\|1-1\|RF2/JH4 | | QVQLVES-GGGVVQPGRSLRL SCAASG-FTFN | D-----YGMH | WVRQAPGK GLEWVA | IIWYD---ENNKYYADS VKG | RFTISRDNSKNTLYLQMSSL RAEDTAVYYCAR | DVLD----------PFDY | WGQGT LVTVS S |
| iPS:4 34229 | 21-225_61H1 | VH3\|3-33/D1\|1-1\|RF2/JH4 | | QVQLVES-GGGVVQPGRSLRL SCAASG-FTFN | D-----YGMH | WVRQAPGK GLEWVA | IIWYD---ESNKYYADS VKG | RFTISRDNSKNTLYLQMNSL RAEDTAVYYCAR | DVLD----------PFDY | WGQGT LVTVS S |
| | Germline | H_FR1 | H_CDR1 | H_FR2 | H_CDR2 | H_FR3 | | H_CDR3 | H_FR4 | |
| | VH1\|1-02/D5\|5-18\|RF3/JH6 | | 32282 | QVQLVQS-GAEVKKPGASVKV SCKASG-YTFT | G-----YYMH | WVRQAPGQ GLEWMG | WINPN---SGGTNYAQK FQG | RVTMTRDTSISTAYMELSRL RSDDTAVYYCAR | GYSYGYYY----------YYYGMDV | WGQGT TVTVS S |
| iPS:4 34209 | 21-225_60C3 | VH1\|1-02/D5\|5-18\|RF3/JH6 | | QVQLVQS-GAEVKKPGASVKV SCKASG-YSFT | G-----HYIH | WVRQAPGQ GLEYMG | WINPN---SGGTNYVQK FQG | RVTMTRDTSISTAYMELSRL RSDDTAIYYCSR | GGLLGATN--------YYYGMDV | WGQGT TVTVS S |
| iPS:4 34315 | 21-225_59G7 | VH1\|1-02/D5\|5-18\|RF3/JH6 | | QVQLVQS-GAEVKKPGASVKV SCKASG-YSFT | G-----HYIH | WVRQAPGQ GLEYMG | WINPN---SGGTNYVQK FQG | RVTMTRDTSISTAYMELSRL RSDDTAIYYCSR | GGLLGATN--------YYYGMDV | WGQGT TVTVT S |
| iPS:4 34319 | 21-225_59B9 | VH1\|1-02/D5\|5-18\|RF3/JH6 | | QVQLVQS-GPEVKKPGASVKV SCKASG-YIFT | G-----NYIH | WVRQAPGQ GLEYMG | WINPN---SGGTNYVQK FQG | RVTMTRDTSISTANMELTSL RSDDTAVYYCSR | GGLLGATY--------YYYGMDV | WGQGT TVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 43003 | 21-225_43F11_LC2 | VH1\|1-02/D5\|5-18\|RF3/JH6 | | QVQLVQS-GAEVKKPGASVKV SCKASG-YTFT | G-----YYIH | WVRQAPGQ GLEWMG | WINPN--- SGGTNYAQK FQG | GVTMTRLISINTAYMDLSRL RSDDTAVYYCAR | GGNYFY-------------------NHVMDV | WGQGT PVTVS S |
| iPS:4 43005 | 21-225_43F11_LC1 | VH1\|1-02/D5\|5-18\|RF3/JH6 | | QVQLVQS-GAEVKKPGASVKV SCKASG-YTFT | G-----YYIH | WVRQAPGQ GLEWMG | WINPN--- SGGTNYAQK FQG | GVTMTRLISINTAYMDLSRL RSDDTAVYYCAR | GGNYFY-------------------NHVMDV | WGQGT PVTVS S |
| **Germline** | | | | **H_FR1** | **H_CDR1** | **H_FR2** | **H_CDR2** | **H_FR3** | **H_CDR3** | **H_FR4** |
| VH4\|4-30.4/D5\|5-12\|RF3/JH4 | | | 32283 | QVQLQES-GPGLVKPSQTLSL TCTVSG-GSIS | SG----DYYWS | WIRQPPGK GLEWIG | YIYY---- SGSTYYNPS LKS | RVTISVDTSKNQFSLKLSSV TAADTAVYYCAR | GYSGYD----------------YYFDY | WGQGT LVTVS S |
| iPS:4 34217 | 21-225_60E8 | VH4\|4-30.4/D5\|5-12\|RF3/JH4 | | QVQLQES-GPGLVRPSATLSL TCTVSG-GSIS | RS----SYYWG | WIRQPPGK GLEWIG | SIYY---- SGSASYNPS LKS | RVTISVDTSENQFSLRLSSV TAADTAVYYCAR | LDSGW---------------SFDY | WGQGT LVTVS S |
| **Germline** | | | | **H_FR1** | **H_CDR1** | **H_FR2** | **H_CDR2** | **H_FR3** | **H_CDR3** | **H_FR4** |
| VH3\|3-23/D3\|3-22\|RF3/JH4 | | | 32284 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | ITMIVVV--------------ITYFDY | WGQGT LVTVS S |
| iPS:4 34219 | 21-225_60E9 | VH3\|3-23/D3\|3-22\|RF3/JH4 | | EVQLLES-GGGLVQPGGSLRL SCAASG-FTFS | S-----YAMS | WVRQAPGK GLEWVS | AISGS--- GGNTFYADS VKG | RFTISRDNSKNTLFLQMNSL RAEDTAVYYCAK | FFGIVG---------------AGYFDY | WGQGT LVTVS S |
| iPS:4 34289 | 21-225_57H12 | VH3\|3-23/D3\|3-22\|RF3/JH4 | | EVQLLES-GGGLVQPGGSLRL SCAASG-LTFS | S-----YAMS | WVRQDPGK GLEWVS | AISGS--- GGNTFYGDS VKG | RFTISRDNSKKTLYLQMNSL RAEDTAVYYCAK | FFGIVG---------------AGYFDY | WGQGT LVTVS S |
| iPS:4 34297 | 21-225_58A10 | VH3\|3-23/D3\|3-22\|RF3/JH4 | | EVQLLES-GGGLVQPGGSLRL SCAASG-FTFR | S-----YAMS | WVRQAPGK GLEWVS | AISGS--- GGNTFYADS VKG | RFTISRDNSKNTLFLQMNSL RAEDTAVYYCAK | FFGIVG---------------AGYFDY | WGQGT LVTVS S |
| iPS:3 92996 | 21-225_28B1 | VH3\|3-23/D3\|3-22\|RF3/JH4 | | EVQLLES-GGGLVQPGGSLRL SCAASG-FTFS | S-----YAMS | WVRQAPGK GLEWVS | AISGS--- GGNTFYADS VKG | RFTISRDNSKNTLYLQMNSL RAEDTAVYYCAK | LGRIAVT--------------GPYFDY | WGQGT LVTVS S |
| **Germline** | | | | **H_FR1** | **H_CDR1** | **H_FR2** | **H_CDR2** | **H_FR3** | **H_CDR3** | **H_FR4** |
| VH4\|4-59/D4\|4-11\|RF3/JH4 | | | 32285 | QVQLQES-GPGLVKPSETLSL TCTVSG-GSIS | S-----YYWS | WIRQPPGK GLEWIG | YIYY---- SGSTNYNPS LKS | RVTISVDTSKNQFSLKLSSV TAADTAVYYCAR | TTVT----------------YFDY | WGQGT LVTVS S |
| iPS:4 34225 | 21-225_60E12 | VH4\|4-59/D4\|4-11\|RF3/JH4 | | QVQLQES-GPGLVKPSETLSL TCTVSG-GSIS | S-----YFWS | WIRQPAGK GLEWIG | RIYT---- RGSTNYNPS LKS | RVTMSVDTSKNQFSLKLSSV TAADTAVYYCAR | EGKTGG---------------VSYFDY | WGQGT LVTVS S |
| iPS:4 34227 | 21-225_61A1 | VH4\|4-59/D4\|4-11\|RF3/JH4 | | QVQLQES-GPGLVKPSETLSL TCTVSG-GSIS | S-----HFWS | WIRQPAGK GLEWIG | RIYI---- RGSTNYNPS LKS | RVTMSVDTSKNQFSLKLSSV TAADTAVYYCAR | EGKTGG---------------VSYFDY | WGQGT LVTVS S |

| iPS | clone | VH germline | H_FR1 | H_CDR1 | H_FR2 | H_CDR2 | H_FR3 | H_CDR3 | H_FR4 |
|---|---|---|---|---|---|---|---|---|---|
| iPS:4 34267 | 21-225_57F2 | VH4\|4-59/D4\|4-11\|RF3/JH4 | QVQLQES-GPGLVKPSEILSL TCTVSG-GSIS | S-----YYWS | WIRQPAGK GLEWIG | RIYT----RGSTNYNPS LKS | RVTMSIDISKNQFSLKLSSV TAADTAVYYCAR | EGKTGG------------------VSYFDY | WGQGT LVTVS S |
| | Germline | | H_FR1 | H_CDR1 | H_FR2 | H_CDR2 | H_FR3 | H_CDR3 | H_FR4 |
| | VH3\|3-23/D5\|5-18\|RF3/JH3 | 32286 | EVQLLES-GGGLVQPGGSLRL SCAASG-FTFS | S-----YAMS | WVRQAPGK GLEWVS | AISGS---GGSTYYADS VKG | RFTISRDNSKNTLYLQMNSL RAEDTAVYYCAK | GYSYGY-------------DAFDI | WGQGT MVTVS S |
| iPS:4 34239 | 21-225_58F1 | VH3\|3-23/D5\|5-18\|RF3/JH3 | EVQLLES-GGGLVQPGGSLRL SCAASG-FTFS | S-----YAMS | WVRQAPGK GLEWVS | AISTG---GGNTYYADS VKG | RFTISRDNSKNTLYLQMNSL RAEDTAVYYCAK | RGVYGD----------FDAFDI | WGQGT MVTVS S |
| iPS:4 34309 | 21-225_59B5 | VH3\|3-23/D5\|5-18\|RF3/JH3 | EVQLLES-GGGLVQPGGSLRL SCAASG-FTFS | S-----YAMN | WVRQAPGK GLEWVS | AISGS---GGNTFYADS VKG | RFTISRDNSKNTLYLQMNSL RAEDTAVYYCAK | RGVYGD----------YEAFDI | WGQGT MVTVS S |
| | Germline | | H_FR1 | H_CDR1 | H_FR2 | H_CDR2 | H_FR3 | H_CDR3 | H_FR4 |
| | VH3\|3-33/D5\|5-18\|RF1/JH4 | 32287 | QVQLVES-GGGVVQPGRSLRL SCAASG-FTFS | S-----YGMH | WVRQAPGK GLEWVA | VIWYD---GSNKYYADS VKG | RFTISRDNSKNTLYLQMNSL RAEDTAVYYCAR | VDTAM------------------VYFDY | WGQGT LVTVS S |
| iPS:4 34245 | 21-225_62H1 | VH3\|3-33/D5\|5-18\|RF1/JH4 | QVQLVES-GGGVVQPGRSLRL SCAASG-FTFS | S-----YGMH | WVRQAPGQ GLEWMA | IIWYD---GSNKYYADS VKG | RFTISRDNSKNTLYLQINSL RAEDTAVYYCAR | EDPRT----------------SCSDY | WGQGT LVTVS S |
| iPS:4 34323 | 21-225_62H8 | VH3\|3-33/D5\|5-18\|RF1/JH4 | QVQLVES-GGGVVQPGRSLRL SCAASG-FTFS | T-----YGMH | WVRQAPGK GLEWVA | VIWHD---GSDKYYVDS VKG | RFTISRDNSKNTLYLQMNSL RAEDTAVYYCAR | EDPRT----------------SCSDY | WGQGT LVTVS S |
| iPS:4 34379 | 21-225_66A9 | VH3\|3-33/D5\|5-18\|RF1/JH4 | QVQLVES-GGGVVQPGRSLRL SCAASG-FTFS | S-----YGMH | WVRQAPGK GLEWVA | VIWHD---GSDKYYADS VKG | RFTISRDNSKNTLYLQMNSL RAEDTAVYYCAR | EDPRT----------------SCSDY | WGQGT LVTVS S |
| | Germline | | H_FR1 | H_CDR1 | H_FR2 | H_CDR2 | H_FR3 | H_CDR3 | H_FR4 |
| | VH3\|3-33/D1\|1-1\|RF3/JH4 | 32288 | QVQLVES-GGGVVQPGRSLRL SCAASG-FTFS | S-----YGMH | WVRQAPGK GLEWVA | VIWYD---GSNKYYADS VKG | RFTISRDNSKNTLYLQMNSL RAEDTAVYYCAR | YNWND------------YFDY | WGQGT LVTVS S |
| iPS:4 34247 | 21-225_62D2 | VH3\|3-33/D1\|1-1\|RF3/JH4 | QVYLVES-GGGVVQPGRSLRL SCAASG-FTFS | S-----YGMH | WVRQAPGK GLEWVA | VIWYD---GSNKYSADS VKG | RFTISRDNSKNTLDLQMNSL RAEDTAVYYCAR | DNGNW----------------NYLDY | WGQGT LVTVS S |
| iPS:4 36838 | 21-225_52H4 | VH3\|3-33/D1\|1-1\|RF3/JH4 | QVQLVES-GGGVVQPGRSLRL SCAASG-FTFS | H-----FGMH | WVRQAPGK GLKWVA | VIWYD---GSNKYYADS VKG | RFTISRDNSKNTLYLQMNSL RAEDTAVYYCAR | GDWNY----------------EGFDY | WGQGT LVTVS S |
| iPS:4 36948 | 21-225_183F5 | VH3\|3-33/D1\|1-1\|RF3/JH4 | QVQLVES-GGGVVQPGRSLRL SCAASG-FTFS | S-----YGML | WVRQAPGK GLEWVT | VIWYD---GSGKYYADS VKG | RFTISRDNSKNTLYLQMNSL RAEDTAVYYCAR | ENFW----------------SGDY | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| VH4\|4-39\|D5\|5-12\|RF3/JH4 | | 32289 | QLQLQES-GPGLVKPSETLSL TCTVSG-GSIS | SS---SYYWG | WIRQPPGK GLEWIG | SIYY---- SGSTYYNPS LKS | RVTISVDTSKNQFSLKLSSV TAADTAVYYCAR | GYSGYD------------YYFDY | WGQGT LVTVS S |
| iPS:4 34249 | 21-225_62E2 | VH4\|4-39/D5\|5-12\|RF3/JH4 | QLQLQES-GPGLVKPSETLSL TCIVSG-GSIS | RS---SYYWG | WIRQPPGK GLEWIG | SIYY---- SGIASYNPS LKS | RVTISVDTSKNQFSLKLNSV TATDTAVYYCAR | LSSGW-------------SFDY | WGQGT LVTVS S |
| iPS:4 34353 | 21-225_64B12 | VH4\|4-39/D5\|5-12\|RF3/JH4 | QLQLQES-GPGLVKPSETLSL TCTVSG-VSIS | RS---SYYWG | WIRQPPGK GLEWIG | SIYY---- SGSTSYNPS LKS | RVTISVDTSKNQFSLKLSSV TAADTAVYYCAR | LDSGW-------------SFDY | WGQGT LVTVS S |
| iPS:3 94073 | 21-225_15C9 | VH4\|4-39/D5\|5-12\|RF3/JH4 | QLQLQES-GPGLVKPSETLSL TCTVSG-GSIS | RS---SYYWG | WIRQPPGK GLEWIG | NIYY---- SGSTYNNPS LKS | RVTISVDTSKNQFSLKLSSV TAADTAVYYCAR | QGSGW-------------EVDY | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| VH1\|1-02\|D7\|7-27\|RF1/JH4 | | 32290 | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | LIGY---------------FDY | WGQGT LVTVS S |
| iPS:4 34259 | 21-225_62G7 | VH1\|1-02\|D7\|7-27\|RF1/JH4 | QVQLVQS-GAEVKKPGASVKV SCKASG-YTFT | G-----YYMH | WVRQAPGQ GLEWMG | WIKPK--- SGGTNQAQK FQG | RVTMTRDTSISTAYMELSSL RSDDTAVYYCAR | APGIAAAG--------TWGYFDY | WGQGT LVTVS S |
| iPS:4 34347 | 21-225_64H10 | VH1\|1-02\|D7\|7-27\|RF1/JH4 | QVQLVQS-GAEVKKPGASVKV SCKASG-YTFT | G-----YYMH | WVRQAPGQ GLEWMG | WIKPN--- SGGTNQAQK FQG | RVTMTRDTSISTAYMELSGL RSDDTAVYYCAR | APGTAATG--------TWGYFDY | WGQGT LVTVS S |
| iPS:4 34359 | 21-225_65G3 | VH1\|1-02\|D7\|7-27\|RF1/JH4 | QVQLVQS-GAEVKKPGASVKV SCKASG-YTFT | G-----YYIH | WVRQAPGQ GLEWMG | WINPN--- SGGTNSAQK FQG | RVTMTRDTSISTAYMELSRL RSDDTAVYYCAR | APGKAAAG--------TWGYFDY | WGQGT LVTVS S |
| iPS:4 34369 | 21-225_66B1 | VH1\|1-02\|D7\|7-27\|RF1/JH4 | QVQLVQS-GAEVKKPGASVKV SCKASG-YTFT | G-----YYMH | WVRQAPGQ GLEWMG | WINPN--- SGGTNNAQK FQG | RVTMTRATSISTAYMELSRL RSDDTAVYYCAR | APGTAAAG--------TWGYFDY | WGQGT LVTVS S |
| iPS:4 34373 | 21-225_66A7 | VH1\|1-02\|D7\|7-27\|RF1/JH4 | QVQLVQS-GAEVKKPGASVKV SCKASG-YTFT | G-----YYMH | WVRQAPGQ GLEWMG | WIKPN--- SGGTNQAQK FQG | RVTMTRDTSISTGYMELSRL RSDDTAVYYCAR | APGTVAAG--------TWGYFDY | WGQGT LVTVS S |
| iPS:4 34397 | 21-225_67H4 | VH1\|1-02\|D7\|7-27\|RF1/JH4 | QVQLVQS-GAEVKKPGASVKV SCKASG-YTFT | G-----YYMH | WVRQAPGQ GLEWMG | WIKPN--- SGGTNQAQK FQG | RVTMTRDTSISTAYMELSGL RSDDTAVYYCAR | APGTAATG--------TWGYFDY | WGQGT LVTVS S |
| IPS:4 34427 | 21-225_70D6 | VH1\|1-02\|D7\|7-27\|RF1/JH4 | QVQLVQS-GAEVKKPGASVKV SCKASG-YIFT | G-----YYMH | WVRQAPGQ GLEWMG | WINPK--- SGGTNSAQK FQG | RVSMTRDTSIGTAYMELRGL RSDDTAEYYCAR | APGKAAAG--------TWGFFDY | WGQGT LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 34435 | 21-225_70G9 | VH1\|1-02\|D7\|7-27\|RF1\|JH4 | | QVQLVQS-GAEVKKPGASVKVSCKASG-YTFT | G-----YYMH | WVRQAPGQGLEWMG | WIKPN---SGGTNQAQKFQG | RVTMTRDISISTAYMELSSLRSDDTAVYYCAR | APGIAAAG---------------TWGYFDY | WGQGTLVTVSS |
| iPS:4 34437 | 21-225_70A12 | VH1\|1-02\|D7\|7-27\|RF1\|JH4 | | QVQLVQS-GAEVKKPGASVKVSCKASG-YTFT | G-----YYMH | WVRQAPGQGLEWMG | WIKPN---SGGTNQAQKFQG | RVTMTRDISISTAYMELSGLRSDDTAVYYCAR | APGTAATG---------------TWGYFDY | WGQGTLVTVSS |
| iPS:4 34451 | 21-225_71B7 | VH1\|1-02\|D7\|7-27\|RF1\|JH4 | | QVQLVQS-GAEVKKPGASVKVSCKASG-YIFT | G-----YYMH | WVRQAPGQGLEWMG | WINPN---SGGTNSAQKFQG | RVTMTRDTSIGTAYMELSGLRSDDTAVYYCAR | APGKAAAG---------------TWGFFDY | WGQGTLVTVSS |
| iPS:4 34459 | 21-225_71A7 | VH1\|1-02\|D7\|7-27\|RF1\|JH4 | | QVQLVQS-GAEVKKPGASVKVSCKASG-YTFT | G-----YYMH | WVRQAPGQGLEWMG | WINPK---SGGTNYVQKFQG | RVTMTRDTSISTAYMELSSLRSDDTAVYYCAR | APGTAPAG---------------SWGYFDY | WGQGTLVTVSS |
| iPS:4 34461 | 21-225_73A3 | VH1\|1-02\|D7\|7-27\|RF1\|JH4 | | QVQLVQS-GAEVKKPGASVKVSCKASG-YTFT | G-----YYMH | WVRQAPGQGLDWMG | WINPK---SGGTNHVQKFQG | RVAMTRDTSISTAYMELSSLRSDDTAVYYCAR | APGTAAAG---------------SWGCFDY | WGQGTLVTVSS |
| | VH3\|3-23\|D4\|4-11\|RF3\|JH4 | Germline | 32291 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | TTVT-------------------------YFDY | WGQGTLVTVSS |
| iPS:4 34261 | 21-225_56F7 | VH3\|3-23\|D4\|4-11\|RF3\|JH4 | | EVQLLES-GGGLVQPGGSLRLSCAASG-FTFS | S-----YVLN | WVRQAPGKGLEWVS | AMSGS---GGRTYYADSVKG | RFTISRDNSKNTLYLQMNSLRAEDTAVYFCAM | TTH----------------------FDY | WGQGTLVTVSS |
| iPS:4 35461 | 21-225_153A1 | VH3\|3-23\|D4\|4-11\|RF3\|JH4 | | EVQLLES-GGGLVQPGGSLRLSCAASG-FSFS | S-----YVMS | WVRQGPGKGLEWVS | AISGS---GDRTYYADSVKG | RFTISRDNSKNTLYLQMNSLRAEDTAVYYCAR | TAT----------------------KDY | WGQGTLVTVSS |
| iPS:4 35509 | 21-225_157H1 | VH3\|3-23\|D4\|4-11\|RF3\|JH4 | | EVQLLES-GGGLVQPGGSLRLSCAASG-FTFS | S-----YAMR | WIRQAPGKGLQWVS | DISGS---GGTTYYADSVKG | RFTISRDNSKNTLYLQMNSLRAEDTAVYYCAK | TY-----------------------L | WGQGTLVTVSS |
| iPS:4 35747 | 21-225_175C4 | VH3\|3-23\|D4\|4-11\|RF3\|JH4 | | EVQLLES-GGGLVQPGGSLRLSCAASA-FTFS | S-----YVMS | WVRQAPGKGLEWVS | AISGS---GDRTYYADSVKG | RFTISRDDSNTTLYLQMNSLRAEDTAVYYCAR | TAG----------------------FDY | WGQGTLVTVSS |
| IPS:3 92784 | 21-225_23C7 | VH3\|3-23\|D4\|4-11\|RF3\|JH4 | | EVQLLES-GGGLVQPGGSLRLSCAASG-FTFS | S-----YVMS | WVRQAPGKGLEWVS | AMSGS---GGSTYYVDSVKG | RFTISRDNSKNTLYLQMNSLRAEDTAVYYCAR | TGV----------------------FDY | WGQGTLVIVSS |
| IPS:3 92802 | 21-225_23E7 | VH3\|3-23\|D4\|4-11\|RF3\|JH4 | | EVQLLES-GGGLVQPGGSLRLSCTASG-FTFS | S-----YAMN | WVRQAPGKGLEWVS | AISGS---GGFTYYADSVKG | RFTISRDNSRNTLYLQMNSLRAEDTAVYYCAR | TSG----------------------FDY | WGQGTLVTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:3 92962 | 21-225_30A1 | VH3\|3-23/D4\|4-11\|RF3/JH4 | EVQLLES-GGGLVQPGGSLRL SCAASG-FTFS | S-----YVMN | WVRQAPGK GLEWVS | AISGS---GGSTYYADS VKG | RFTISRNNSKNTLYLQMNSL RAEDTAVYYCAR | TGV------------------------FDY | WGQGT LVTVS S |
| iPS:3 93090 | 21-225_33A5 | VH3\|3-23/D4\|4-11\|RF3/JH4 | EVQLLES-GGGLVQPGGSLRL SCAASG-FTFS | S-----YVIN | WVRQAPGK GLEWVS | AISGS---GVSTYYADS VKG | RFTISRDNSKNTLYLQMNSL RAEDTAVYYCAR | TSL------------------------FDY | WGQGT LVTVS S |
| | | Germline | H_FR1 | H_CDR1 | H_FR2 | H_CDR2 | H_FR3 | H_CDR3 | H_FR4 |
| | VH3\|3-23/D1\|1-1\|RF3/JH4 | 32292 | EVQLLES-GGGLVQPGGSLRL SCAASG-FTFS | S-----YAMS | WVRQAPGK GLEWVS | AISGS---GGSTYYADS VKG | RFTISRDNSKNTLYLQMNSL RAEDTAVYYCAK | YNWND-----------------YFDY | WGQGT LVTVS S |
| iPS:4 34273 | 21-225_57E4 | VH3\|3-23/D1\|1-1\|RF3/JH4 | EVQLLES-GGGLVQPGGSLRL SCAASG-FTFS | S-----YAMS | WVRQAPGK GLEWVS | VISGS---GGSTFYADS VKG | RFTISRDNSKTTLYLQMNSL RAEDTAVYYCAK | RDWND-----------------VFDY | WGQGT LVTVS S |
| | | Germline | H_FR1 | H_CDR1 | H_FR2 | H_CDR2 | H_FR3 | H_CDR3 | H_FR4 |
| | VH3\|3-23/D3\|3-3\|RF3/JH4 | 32293 | EVQLLES-GGGLVQPGGSLRL SCAASG-FTFS | S-----YAMS | WVRQAPGK GLEWVS | AISGS---GGSTYYADS VKG | RFTISRDNSKNTLYLQMNSL RAEDTAVYYCAK | IILFGVV-----------------IIYFDY | WGQGT LVTVS S |
| IPS:4 34279 | 21-225_57F7 | VH3\|3-23/D3\|3-3\|RF3/JH4 | EVQLLES-GGGLVQPGGSLRL SCAASG-FTFS | S-----YAMS | WVRQAPGK GLEWVS | AISGS---GGNTFYADS VKG | RFTISRDNSKNTLYLQMNSL RAEDTAVYYCAK | FFGVVG-----------------VGCFDY | WGQGT LVTVS S |
| iPS:4 37228 | 21-225_60C11 | VH3\|3-23/D3\|3-3\|RF3/JH4 | EVQLLES-GGGLVQPGGSLRL SCAASG-FPFS | S-----YAMS | WVRQAPGK GLEWVS | AISGS---GGNTFYADS VKG | RFTISRDNSKNTLYLQMNSL RAEDTAVYYCAK | FFGVVG-----------------VGCFDY | WGQGT LVTVS S |
| | | Germline | H_FR1 | H_CDR1 | H_FR2 | H_CDR2 | H_FR3 | H_CDR3 | H_FR4 |
| | VH3\|3-23/D3\|3-22\|RF3/JH1 | 32294 | EVQLLES-GGGLVQPGGSLRL SCAASG-FTFS | S-----YAMS | WVRQAPGK GLEWVS | AISGS---GGSTYYADS VKG | RFTISRDNSKNTLYLQMNSL RAEDTAVYYCAK | IIMIVVVI-----------------TAEYFQH | WGQGT LVTVS S |
| iPS:4 34291 | 21-225_58A4 | VH3\|3-23/D3\|3-22\|RF3/JH1 | EVQLLES-GGGLVQPGGSLRL SCAASG-LTFS | S-----YAMS | WVRQDPGK GLEWVS | AISGS---GGNTFYGDS VKG | RFTISRDNSKKTLYLQMNSL RAEDTAVYYCAK | FFGIVG-----------------AGFFDS | WGQGT LVTVS S |
| | | Germline | H_FR1 | H_CDR1 | H_FR2 | H_CDR2 | H_FR3 | H_CDR3 | H_FR4 |
| | VH3\|3-33/D2\|2-15\|RF3/JH4 | 32295 | QVQLVES-GGGVVQPGRSLRL SCAASG-FTFS | S-----YCMH | WVRQAPGK GLEWVA | VIWYD---GSNKYYADS VKG | RFTISRDNSKNTLYLQMNSL RAEDTAVYYCAR | DIVVVVA-----------------ATYFDY | WGQGT LVTVS S |
| iPS:4 34299 | 21-225_58D11 | VH3\|3-33/D2\|2-15\|RF3/JH4 | QVQLVES-GGGVVQPGRSLRL SCAASG-FTFS | D-----YDIH | WVRQSPGK GLEWVA | VIWYD---GSKKYYADS VKG | RFTISRDNSKNTLYLQMNSL RAEDTALYYCAR | DRVT------------------------FDY | WGQGT LVTVS S |
| iPS:4 34871 | 21-225_85H1 | VH3\|3-33/D2\|2-15\|RF3/JH4 | QVQLVES-GGGVVQPGRSLRL SCAASG-FTFS | S-----YGIH | WVRQAPGK GLEWVA | VIWYD---GSNKYYADS VKG | RFTISRDNSKNTLYLQMNSL RAEDTAVYYCAR | DPFIVG-----------------ATYFDY | WGQGT LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 35109 | 21-225_92H5 | VH3\|3-33/D2\|2-15\|RF2/JH4 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | DPFIVG-------------------ATYFDY | WGQGT LVTVS S |
| iPS:4 36434 | 21-225_216B10 | VH3\|3-33/D2\|2-15\|RF3/JH4 | | QVQLVES-GGGVVQPGRSLRL SCAASG-FTFS | S-----YGMH | WVRQAPGK GLEWVA | AIWYD--- GSNKYYADS VKG | RFTISRDNSKRTLYLQMNSL RAEDTAVYYCAR | DPNIVG-------------------ATWFDY | WGQGT LVTVS S |
| | | Germline | | H_FR1 | H_CDR1 | H_FR2 | H_CDR2 | H_FR3 | H_CDR3 | H_FR4 |
| | VH1\|1-02/D4\|4-17\|RF2/JH4 | | 32296 | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | DYGDY----YFDY | WGQGT LVTVS S |
| iPS:4 34307 | 21-225_59B2 | VH1\|1-02/D4\|4-17\|RF2/JH4 | | QVQLVQS-GAEVKKPGASVKV SCKASG-YTFA | G-----YYIH | WVRQAPGQ GLEWLG | WINPN--- SGGTNYAQK FQG | RVTMTRDTSISTAYMELSRL RFDDTAVYYCAR | DPGP-----------FDY | WGQGT LVTVS S |
| | | Germline | | H_FR1 | H_CDR1 | H_FR2 | H_CDR2 | H_FR3 | H_CDR3 | H_FR4 |
| | VH3\|3-33/D3\|3-22\|RF3/JH6 | | 32297 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | ITMIVVVIT----------YYYYYGMDV | WGQGT TVTVS S |
| iPS:4 34311 | 21-225_59H5 | VH3\|3-33/D3\|3-22\|RF3/JH6 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | ERGIAVG----------YYGMDV | WGQGT TVTVS S |
| | | Germline | | H_FR1 | H_CDR1 | H_FR2 | H_CDR2 | H_FR3 | H_CDR3 | H_FR4 |
| | VH4\|4-39/D1\|1-26\|RF3/JH4 | | 32298 | QLQLQES-GPGLVKPSETLSL TCTVSG-GSIS | SS----SYYWG | WIRQPPGK GLEWIG | SIYY---- SGSTYYNPS LKS | RVTISVDTSKNQFSLKLSSV TAADTAVYYCAR | YSGSY----------YYFDY | WGQGT LVTVS S |
| iPS:4 34313 | 21-225_59E6 | VH4\|4-39/D1\|1-26\|RF3/JH4 | | QLQLQES-GPGLVKPSETLSL TCTVSG-GSIS | RS---SYYWG | WIRQPPGK GLEWIG | NIYY---- SGSTYYNPS LKS | RVTISVDTSKNQFSLKLSSV TAADTALYYCAR | HSSSW---------------SLDY | WGQGT LVTVS S |
| iPS:4 34413 | 21-225_68D12 | VH4\|4-39/D1\|1-26\|RF3/JH4 | | QLQLQES-GPGLVKPSETLSL TCTVSG-GSIS | RS---SYYWG | WIRQPPGK GLEWIG | NIYY---- SGSTYYIPS LKS | RVTISVDTSKNQFSLKLTSV TAADTAVYYCAR | HSTSW---------------SIDY | WGQGT LVTVS S |
| iPS:3 92628 | 21-225_20C2 | VH4\|4-39/D1\|1-26\|RF3/JH4 | | QLQLQES-GPGLVKPSETLSL TCTVSG-GSIS | RS---SYYWG | WIRQPPGK GLEWIG | NIYY---- SGTAYCNSS LKS | RVIISVDTSKNQFSLKLSSV TATDTAVYYCAR | HSSSW---------------SLDN | WGQGT LVTVS S |
| iPS:3 92642 | 21-225_18C6 | VH4\|4-39/D1\|1-26\|RF3/JH4 | | QLQLQES-GPGLVKPSETLSL TCTVSG-GSIS | RS---SYYWG | WIRQPPGK GLEWIG | NIYY---- SGYTYYNPS LKS | RVIISVDTSKNQFSLKLSSV TAADTALYYCAR | HSSSW---------------SLDD | WGQGT LVTVS S |
| iPS:3 92706 | 21-225_18A3 | VH4\|4-39/D1\|1-26\|RF3/JH4 | | QLQLQES-GPGLVKFSETLSL TCTVSG-GSIS | RS---SYYWG | WIRQPPGK GLEWIG | NIYY---- SGYTYYTPS LKS | RVTISVDTSKNQFSLKLSSV TAADTAVYYCAR | HSTSW---------------SLDY | WGQGT LVTVS S |

| | | | | | | | | | | |
|---|---|---|---|---|---|---|---|---|---|---|
| iPS:3 92800 | 21-225_22D12 | VH4\|4-39/D1\|1-26\|RF3/JH4 | | QLQLQES-GPGLVKPSETLSLTCTVSG-GSIS | RS---SYYWG | WIRQPPGKGLEWIG | NIYY----SGTTSYNPSLKS | RVTISVDISRNQFSLKLSSVTAADTAVFYCAR | LSSSW---------------------SVDY | WGQGTLVTVSS |
| iPS:3 92820 | 21-225_23D1 | VH4\|4-39/D1\|1-26\|RF3/JH4 | | QLQLQES-GPGLVKPSETLSLTCTVSG-GSIS | RS---SYYWG | WIRQPPGKGLEWIG | SIYY----SGSAQYNPSLKS | RVTISVDTSKNQFSLTLSSVTAADTALYYCAR | LSSSW---------------------SFDY | WGQGTLVTVSS |
| iPS:3 92824 | 21-225_24E5 | VH4\|4-39/D1\|1-26\|RF3/JH4 | | QLQLQES-GPGLVKPSETLSLTCTVSG-GAIS | RS---SYYWG | WIRQPPGKGLEWIG | SIYY----SGSANYNPSLKS | RVTISVDTSKNQFSLKLSSVTAADTAVYYCAR | LSSSW---------------------SIDN | WGQGTLVTVSS |
| iPS:3 92834 | 21-225_22C1 | VH4\|4-39/D1\|1-26\|RF3/JH4 | | QLQLQES-GPGLVKPSETLSLTCTVSG-GSIN | RS---SYYWG | WIRQPPGKGLEWIG | NIYY----SGATYYNSSLKS | RVTISVDTSTNQFSLKLSSVTAADTAVYYCAR | HSGSW---------------------SLDY | WGQGTLVTVSS |
| iPS:3 92870 | 21-225_20G9 | VH4\|4-39/D1\|1-26\|RF3/JH4 | | QLQLQES-GPGLVKPSETLSLTCTVSG-GSIS | RS---SYYWG | WIRQPPGKGLEWIG | SIYY----SGSASYNPSLKS | RVTISVDTSRNQFSLKLSSVTAADTAAYYCAR | LSSSW---------------------SFDY | WGQGTLVTVSS |
| iPS:3 92896 | 21-225_21G7 | VH4\|4-39/D1\|1-26\|RF3/JH4 | | QLQLQES-GPGLVKFSETLSLTCTVSG-GSTS | RS---SYYWG | WIRQPPGKGQEWIG | NIYY----SGYSYYNPSLKS | RVTISVDTSKNQFSLKLSSVTAADTAVYYCAR | HSTSW---------------------SLDY | WGQGTLVTVSS |
| iPS:3 92904 | 21-225_22G9 | VH4\|4-39/D1\|1-26\|RF3/JH4 | | QLQLQES-GPGLVKPSETLSLTCTVSG-GAIS | GS---NYYWG | WIRQPPGKELEWIG | NIYY----SGSTYYNPSLKS | RVTISVDTSKNQFSLKLRSVTAEDTAVYYCAR | HSSSW---------------------SLDY | WGQGTLVTVSS |
| iPS:3 93094 | 21-225_34C4 | VH4\|4-39/D1\|1-26\|RF3/JH4 | | QLQLQES-GPGLVKPSETLSLTCTVSG-GSIS | RS---SYYWG | WIRQSPGKGLEWIG | SIYY----SGSTAYNPSLKS | RVTISVDTSKNQVSLKLSSVTAADTAVYYCAR | LSSSW---------------------SFDY | WGQGTLVTVSS |
| iPS:3 93806 | 21-225_6A6 | VH4\|4-39/D1\|1-26\|RF3/JH4 | | QLQLQES-GPGLVKPSETLSLTCTVSG-GSIS | RS---SYYWG | WIRQPPGKGLEWIG | NIYY----SGIPYYNPSLKS | RVNISVDTSKNQFSLKLNSVTAADTAVYYCAR | HSSSW---------------------SLDY | WGQGTLVTVSS |
| iPS:3 93814 | 21-225_7F4 | VH4\|4-39/D1\|1-26\|RF3/JH4 | | QLQLQES-GPGLVKPSETLSLTCTVSG-GSIS | RS---SYYWG | WIRQPPGKGLEWIG | NIYY----SGATYYNPSLKS | RVTISVDTSTNQFSLKLSSVTAADTAVYYCAR | HSGSW---------------------SLDY | WGQGTLVTVSS |
| iPS:3 93816 | 21-225_6D4 | VH4\|4-39/D1\|1-26\|RF3/JH4 | | QLQLQES-GPGLVKPSETLSLTCTVSG-GSIS | RS---SSYWG | WIRQPPGKGLEWIG | NIYY----SGSAYYIPSLKS | RVTISVATSKNQFSLNLTSVTAADTAVYYCAR | HSSSW---------------------SLDC | WGQGTLVTVSS |
| iPS:3 93880 | 21-225_15A1 | VH4\|4-39/D1\|1-26\|RF3/JH4 | | QLHLQES-GPGLVKPSETLSLTCTVSG-GSIS | RS---SYYWG | WIRQPPGKGLEWIG | SIYY----SGSAQYNPSLKS | RVTISVDTTKNQFSLTLSSVTAADTAVYYCAR | LSSSW---------------------SFDY | WGQGTLVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:3 94002 | 21-225_15G7 | VH4\|4-39/D1\|1-26\|RF3/JH4 | | QLQLQES-GPGLVKPSETLSLTCTVSG-GSIS | RS---SSYWG | WIRQPPGKGLEWIG | SIYY----SGYTYYTPSLKS | RVTISVDISKNQFSLRLSSVTAADTASYYCAR | LSSSW--------------------SFDF | WGQGTLVTVSS |
| iPS:3 94053 | 21-225_11F10 | VH4\|4-39/D1\|1-26\|RF3/JH4 | | QLHLQES-GPGLVKPSETLSLTCTVSG-ASIS | RS---SYYWG | WIRQPPGKGLEWIG | SIYY----SGSAQYNPSLKS | RVTISVDISKNQFSLTLSSVTAADTAVYYCAR | LSSSW--------------------SFDY | WGQGTLVTVSS |
| iPS:3 94057 | 21-225_15H1 | VH4\|4-39/D1\|1-26\|RF3/JH4 | | QLQLQES-GPGLVKFSETLSLTCTVSG-GSIS | RS---SYYWG | WIRQPPGKGLEWIG | NIYY----SGYPYYNPSLKS | RVTISVDISKNQFSLKLSSVTAADTAVYYCAR | HSTSW--------------------SLDY | WGQGTLVTVSS |
| iPS:3 94063 | 21-225_16A1 | VH4\|4-39/D1\|1-26\|RF3/JH4 | | QLQLQES-GPGLVKPSETLSLICTVSG-GSID | RS---SYYWG | WIRQPPGKGLEWIG | SIYY----SGSAYHNPSLKS | RGTISVDISKNQFSLKLSSVTAADTAAYYCAR | LSSSW--------------------SFDY | WGQGTLVTVSS |
| iPS:3 94075 | 21-225_8D12 | VH4\|4-39/D1\|1-26\|RF3/JH4 | | QLQLQES-GPGLVKFSETLSLTCTVSG-GSIS | RS---SYYWG | WIRQPPGKGLEWIG | NIYY----SGYPYYNPSLKS | RVTISIDTSKNQFSLKLSSVTAADTAVYYCAR | HSTSW--------------------SLDY | WGQGTLVTVSS |
| | Germline | | | H_FR1 | H_CDR1 | H_FR2 | H_CDR2 | H_FR3 | H_CDR3 | H_FR4 |
| | VH3\|3-48/D7\|7-27\|RF3/JH4 | | 32299 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | NWGY-----------------FDY | WGQGTLVTVSS |
| iPS:4 34317 | 21-225_59E8 | VH3\|3-48/D7\|7-27\|RF3/JH4 | | EVQLVES-GGGLVQPGGSLRLSCAASG-FTFS | S-----YSMN | WVRQAPGKGLGWVS | YISSS---SGTIYYADSVKG | RFTISRDNAKNSLYLQMNSLRDEDTAVYYCAR | EWGMAV----------------AGPFDY | WGQGTLVTVSS |
| | Germline | | | H_FR1 | H_CDR1 | H_FR2 | H_CDR2 | H_FR3 | H_CDR3 | H_FR4 |
| | VH3\|3-33/D1\|1-26\|RF3/JH4 | | 32300 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YSGSY-----------------YYFDY | WGQGTLVTVSS |
| iPS:4 34327 | 21-225_63G6 | VH3\|3-33/D1\|1-26\|RF3/JH4 | | QVQLVES-GGGVVQPGRSLRLSCAASG-FTFS | S-----YGMH | WVRQAPGKGLEWVT | VIWYD---GSNKYYADSVKG | RFTISRDNSKNTLYLQMNSLRAEDTAVYYCAR | DSLSGI----------------AAAFDY | WGQGTLVTVSS |
| iPS:4 37084 | 21-225_206B5 | VH3\|3-33/D1\|1-26\|RF3/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYNCAR | EGGSY--------------------HLDY | WGQGILVTVSS |
| iPS:4 37088 | 21-225_209H10 | VH3\|3-33/D1\|1-26\|RF3/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYNCAR | EGGSY--------------------HLDY | WGQGILVTVSS |
| iPS:3 92684 | 21-225_17F4 | VH3\|3-33/D1\|1-26\|RF3/JH4 | | QVQLVES-GGGVVQPGRSLRLSCAASG-FTFS | N-----YGMN | WVRQAPGKGLEWVA | VIWYD---GNNKHYADSVKG | RFTISRDNSKNTLYLQMNSLRAEDTAVYYCAS | SGSY-----------------FFDY | WGQGTLVTVSS |

| | Germline | | H_FR1 | H_CDR1 | H_FR2 | H_CDR2 | H_FR3 | H_CDR3 | H_FR4 |
|---|---|---|---|---|---|---|---|---|---|
| | VH1\|1-02/D6\|6-19\|RF2/JH4 | 32301 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GIAVA----------GYFDY | WGQGTLVTVSS |
| iPS:4 34333 | 21-225_63C9 | VH1\|1-02/D6\|6-19\|RF2/JH4 | QVQLVQS-GAEVKKPGASVKVSCKASG-YTFT | G-----YYMH | WVRQAPGQGLEWMG | WINPN---SGGTNFAQKFQG | RVTMTRDASINTAYMELRSLISDDTAVYYCAR | APGVAAAG--------SWGYFDY | WGQGTLVTVSS |

| | Germline | | H_FR1 | H_CDR1 | H_FR2 | H_CDR2 | H_FR3 | H_CDR3 | H_FR4 |
|---|---|---|---|---|---|---|---|---|---|
| | VH3\|3-33/D4\|4-11\|RF2/JH4 | 32302 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYSNY-----------YFDY | WGQGTLVTVSS |
| iPS:4 34335 | 21-225_63C10 | VH3\|3-33/D4\|4-11\|RF2/JH4 | QVQLVES-GGGVVQPGRSLRLSCAASG-FTFS | S-----YGMH | WVRQAPGKGLDWVA | VIWYD---GSNKYYADSVKG | RFTISRDNSKNTLYLQMNSLRAEDTAVYYCAR | DDPRS-----------SAGDY | WGQGTLVTVSS |
| iPS:4 34429 | 21-225_70H6 | VH3\|3-33/D4\|4-11\|RF2/JH4 | QVQLVES-GGGVVQPGRSLRLSCAASG-FTFS | S-----YGMH | WVRQTPGKGLDWVA | VIWHD---GSNKYYADSVKG | RFTISRDNSKNTLYLQMNSLRAEDTAVYYCAR | DDPRS-----------SAGDY | WGQGTLVTVSS |
| iPS:4 34569 | 21-225_77H5 | VH3\|3-33/D4\|4-11\|RF2/JH4 | QVQLVES-GGGVVQPGRSLRLSCAASG-FTFS | N-----YGMH | WVRQAPGKGLEWVA | VIWYD---GRNKYYADSVKG | RFTISRDNSKNTLYLQMNSLRAEDTALYYCAR | DRSILG---------ATFFDY | WGQGTLVTVSS |
| iPS:4 34629 | 21-225_74C3 | VH3\|3-33/D4\|4-11\|RF2/JH4 | QVQLVES-GGGVVQPGRSLRLSCAASG-FTFS | S-----FGMH | WARQAPGKGLEWVA | AIWYD---GSNKYCADSVKG | RFTISRDNSKNTLSLQMNSLRAEDSAVYYCAR | DRSILG---------AAFFDY | WGQGTLVTVSS |
| iPS:4 35793 | 21-225_180F8 | VH3\|3-33/D4\|4-11\|RF2/JH4 | QVQLVES-GGGVVQPGRSLRLSCAASG-FTFS | S-----YGMH | WVRQAPGKGLEWVS | VIWYD---GSDKYYEDSVKG | RFTISRDNSKNTLYLQMNSLRAEDTAVYYCAR | DHPRW-----------SYGDY | WGQGTLVTVSS |
| iPS:4 36382 | 21-225_212C10 | VH3\|3-33/D4\|4-11\|RF2/JH4 | QVQLVES-GGGVVQPGRSLRLSCAASG-FTFS | S-----YGMH | WVRQAPGKGLEWVT | AIWYD---GSHKYYTDSVKG | RFTISRDNSKNTLYLQMNSLRAEDTAVYYCAR | DRSIVG---------ATYFDY | WGQGTLVTVSS |
| iPS:3 92660 | 21-225_19B3 | VH3\|3-33/D4\|4-11\|RF2/JH4 | QVQLVES-GGGVVQPGRSLRLSCAASG-FTFS | S-----YDMH | WVRQAPGKGLEWVA | VIWYD---GSDKYYADSVKG | RFTISRDNSKNTLFLQMNSLRAEDTAVYYCAR | DRAYS-----------SSSDY | WGQGTLVTVSS |
| iPS:3 93904 | 21-225_8H11 | VH3\|3-33/D4\|4-11\|RF2/JH4 | QVQLVES-GGGVVQPGRSLRLSCAASG-FTFS | T-----YNMH | WVRQAPGKGLEWVA | VIWYD---GSDRYSADSVKG | RFTISRDNSKNTLYLQMNSLRAEDTAVYYCAR | DRAYS-----------SSSDF | WGQGTLVTVSS |

| | Germline | | H_FR1 | H_CDR1 | H_FR2 | H_CDR2 | H_FR3 | H_CDR3 | H_FR4 |
|---|---|---|---|---|---|---|---|---|---|
| | VH4\|4-59/D4\|4-11\|RF2/JH4 | 32303 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | DYSNY-----------YFDY | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 34341 | 21-225_64F7 | VH4|4-59/D4|4-11|RF2/JH4 | | QVQLQES-GPGLVKPSEILSL TCTVSG-GSIS | S-----YFWS | WIRQPAGK GLEWIG | RIYT---- SGISNYNPS LKS | RVTMSVDISKNQFSLKLSSV TAADTAVYYCAR | FSSG------------ ----------FFDY | WGQGT LVTVS S |
| | VH3|3-33/D7|7-27|RF2/JH1 | | 32304 & 32305 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | *LGAE------------ ----------YFQH | WGQGT LVTVS S |
| iPS:4 34351 | 21-225_64A12 | VH3|3-33/D7|7-27|RF2/JH1 | | QVQLVES-GGGVVQPGRSLRL SCAASG-FTFS | S-----YGMH | WVRQAPGK GLEWVA | VIWYD--- ESNKYYVDS VKG | RFTISRDNSKNTLYLQMNSL RAEDTAVYYCAR | ELGF------------ ----------LSDH | WGQGT LVTVS S |
| iPS:3 92700 | 21-225_16E12 | VH3|3-33/D7|7-27|RF2/JH1 | | QVQLVES-GGGLVQPGRSLRL SCAASG-FTFS | N-----YGMH | WVRQAPGK GLEWVA | VIWYE--- GSNKYYVDS VRG | RFTISRDNSKSTLYLQMNSL RAEDTAVYYCVR | ELGF------------ ----------QSDH | WGQGT PVTVS S |
| iPS:3 92710 | 21-225_19A10 | VH3|3-33/D7|7-27|RF2/JH1 | | QVQLVES-GGGVVQPGRSLRL SCAASG-FTFS | S-----YGMH | WVRQAPGK GLEWVA | VIWYD--- ESNKYYADS VKG | RFTISRDNSKNTLYLQMNSL RAEDTAVYYCAR | ELAW------------ ----------YEDS | WGQGT LVTVS S |
| iPS:3 94093 | 21-225_9D12 | VH3|3-33/D7|7-27|RF2/JH1 | | QVQLVES-GGGVVQPGRSLRL SCAASG-FTFS | D-----YGMH | WVRQAPGK GLEWVA | VIWYD--- GNNNYYADS VKG | RFTISRDNSKNTLYLQMNSL RAEDTAVYYCAR | ELAW------------ ----------YEDF | WGQGT LVTVS S |
| | | Germline | | H_FR1 | H_CDR1 | H_FR2 | H_CDR2 | H_FR3 | H_CDR3 | H_FR4 |
| | VH3|3-23/D3|3-22|RF2/JH3 | | 32306 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | YYYDSSGY-------- --------YYDAFDI | WGQGT MVTVS S |
| iPS:4 34355 | 21-225_64G12 | VH3|3-23/D3|3-22|RF2/JH3 | | EVQLLES-GGGLVQPGGSLRL SCAASG-FTFS | S-----NAMS | WVRQAPGK GLEWVS | VISGS--- GGSTYYADS VKG | RFTISRDNSKNTLYLQMNSL RAEDTALYYCAK | RNYDD----------- ----------AFDI | WGQGT MVTVS S |
| | | Germline | | H_FR1 | H_CDR1 | H_FR2 | H_CDR2 | H_FR3 | H_CDR3 | H_FR4 |
| | VH3|3-21/D4|4-11|RF3/JH4 | | 32307 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | TTVT------------ ------------YFDY | WGQGT LVTVS S |
| iPS:4 34367 | 21-225_65H11 | VH3|3-21/D4|4-11|RF3/JH4 | | EVQLVES-GGGLVKPGGSLRL SCAASG-FTFS | S-----YRMN | WVRQAPGK GLEWVS | SISSS--- NSSIYYADS VKG | RFTTSRDNAKNSLYLQMNSL RAEDTAVYYCTS | TS-------------- ------------GS | WGQGT LVTVS S |
| iPS:4 37220 | 21-225_55H6 | VH3|3-21/D4|4-11|RF3/JH4 | | EVHLVES-GGGLVKPGGSLRL SCAASG-FTFS | S-----YSMN | WVRQAPGK GLEWVS | SISGS--- STYIYYADS VKG | RFTISRDNAKNSLYLQMNSL RAEDTAVYYCAR | TGV------------ ----------FDY | WGQGT LVTVS S |
| iPS:4 02237 | 21-225_23D11 | VH3|3-21/D4|4-11|RF3/JH4 | | EVQLVES-GGGLVKPGGSLRL SCAASG-FTFS | S-----YNIN | WVRQAPGK GLEWVS | SISGN--- SGYIYYADS VKG | RFTISRDNAKNSLYLQMNSL RAEDTAVYYCAR | TNL------------ ----------FDY | WGQGT LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-21/D4\|4-11\|RF3/JH3 | | 32308 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | TIVTD--------------------AFDI | WGQGTMVTVSS |
| iPS:4 34383 | 21-225_66F9 | VH3\|3-21/D4\|4-11\|RF3/JH3 | | EVQLVES-GGGLVKPGGSLRLSCAASG-FTFS | S-----YSMN | WVRQAPGKGLEWVS | SISGT---SSYIYYADSVKG | RFTISRDNAKNSLYLQMNGLRAEDTAVYFCAR | TNA-----------------FDI | WGQGTMVTVSS |
| iPS:4 34449 | 21-225_71H6 | VH3\|3-21/D4\|4-11\|RF3/JH3 | | EVQLVES-GGGLVKPGGSLRLSCAASG-FTFS | S-----YSMN | WVRQAPGKGLEWVS | SISGT---SSYIYYADSVKG | RFTISRDNAKNSLYLQMNGLRAEDTAVYFCAK | TNA-----------------FDI | WGQGTMVTVSS |
| | | Germline | | H_FR1 | H_CDR1 | H_FR2 | H_CDR2 | H_FR3 | H_CDR3 | H_FR4 |
| | VH3\|3-33/D6\|6-6\|RF2/JH4 | | 32309 | QVQLVES-GGGVVQPGRSLRLSCAASG-FTFS | S-----YCMH | WVRQAPGKGLEWVA | VIWYD---GSNKYYADSVKG | RFTISRDNSKNTLYLQMNSLRAEDTAVYYCAR | SIAAR---------------YFDY | WGQGTLVTVSS |
| iPS:4 34399 | 21-225_67B7 | VH3\|3-33/D6\|6-6\|RF2/JH4 | | QVQLVES-GGGVVQPGRSLRLSCAASG-FTFS | N-----YGMH | WVRQAPGKGLEWVA | VILYD---GSKKYYADSVKG | RFTISRDNSKNTLYLQMNSLRAEDTAVYYCAR | SIPE-----------------FDY | WGQGTLVTVSS |
| iPS:4 34463 | 21-225_73A6 | VH3\|3-33/D6\|6-6\|RF2/JH4 | | QVQLVES-GGGVVQPGRSLRLSCAASG-FTFS | Y-----YGMH | WVRQAPGKGLEWVA | VILYD---GSKKYYAASVKG | RFTISRDNSKNTLYLQMNSLRAEDTAVYYCAR | SIPD-----------------FDY | WGQGTLVTVSS |
| | | Germline | | H_FR1 | H_CDR1 | H_FR2 | H_CDR2 | H_FR3 | H_CDR3 | H_FR4 |
| | VH3\|3-21/D1\|1-26\|RF3/JH4 | | 32310 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | YSGSY---------------YYFDY | WGQGTLVTVSS |
| iPS:4 34405 | 21-225_68E6 | VH3\|3-21/D1\|1-26\|RF3/JH4 | | EVQLVES-GGGLVKPGGSLRLSCAASG-FTLS | S-----FGMN | WVRQAPGKGLEWVS | YISRS---SSHIYYADSVKG | RFTISRDNAKNSLYLQMNSLRAEDTAVYYCAV | SSGS-----------------PFDY | WGQGTLVTVSS |
| iPS:4 35595 | 21-225_160H4 | VH3\|3-21/D1\|1-26\|RF3/JH4 | | EVQLVES-GGGLVKSGGSLRLSCAASG-FTFS | S-----YRMN | WVRQAPGKGLEWVS | SISGS---SSYIDYADSVKG | RFTISRDNAKNSLYLQMNSLRAEDTAVYYCAR | KSW-----------------FDY | WGQGTLVTVSS |
| iPS:4 35635 | 21-225_163F1 | VH3\|3-21/D1\|1-26\|RF3/JH4 | | EVQLVDS-GGGLVKPGGSLRLSCAASG-FTFS | S-----YSMN | WVRQAPGKGLEWVS | SISGS---GSYIYYADSVKG | RFTTSRDNAKNSLYLQMNSLSADDTAVYYCTL | YSS-----------------SHY | WGQGTLVTVSS |
| | | Germline | | H_FR1 | H_CDR1 | H_FR2 | H_CDR2 | H_FR3 | H_CDR3 | H_FR4 |
| | VH3\|3-30.3/D2\|2-15\|RF3/JH4 | | 32311 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DIVVVVA-------------ATYFDY | WGQGTLVTVSS |
| iPS:4 34417 | 21-225_69C8 | VH3\|3-30.3/D2\|2-15\|RF3/JH4 | | QVQLVES-GGGVVQPGRSLRLSCAASG-FTFS | N-----YAMH | WVRQAPGKGLEWVA | VIWYD---GSDKYYADSVKG | RFTISRDNSKNTLYLQMISLRAEDTAVYYCAR | DIPSN-------------SAGDY | WGQGTLVTVSS |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-33/D3\|3-10\|RF1/JH4 | 32512 & 32513 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | VLLWFGE--------------LL*YFDY | WGQGT LVTVS S |
| iPS:4 34433 | 21-225_70E8 | VH3\|3-33/D3\|3-10\|RF1/JH4 | QVQLVES-GGGVVQPGRSLRL SCAASG-FTFS | S-----YGML | WVRQAPGK GLEWVA | IIWYD--- ESNKYYADS VKG | RFTISRDNSKNTLYLQMNSL RAEDTAVYYCAR | DLLD----------------PRDY | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-23/D6\|6-13\|RF1/JH4 | 32314 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | GYSSSW----------YYFDY | WGQGT LVTVS S |
| iPS:4 34467 | 21-225_73H8 | VH3\|3-23/D6\|6-13\|RF1/JH4 | EVQLLES-GGGWVQSGGSLRL SCAASG-FTFS | S-----NAMS | WVRQAPGK GLEWVS | DISRS--- GGTTFYADS VKG | RFTISRDNSKNTLYLQMNSL RAEDTAVYYCAK | WDSSSWY----------DVTPFDY | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-34/D4\|4-17\|RF2/JH4 | 32315 | QVQLQQW-GAGLLKPSETLSL TCAVYG-GSFS | G-----YYWS | WIRQPPGK GLEWIG | EINH---- SGSTNYNPS LKS | RVTISVDTSKNQFSLKLSSV TAADTAVYYCAR | DYGDY-----------YFDY | WGQGT LVTVS S |
| iPS:4 34471 | 21-225_75G3 | VH4\|4-34/D4\|4-17\|RF2/JH4 | QVQLQQW-GAGLLKPSETLSL TCAVYG-GSLS | G-----SYWS | WIRQPPGK GLEWIG | EINL---- SGSTNYNPS LKS | RVTISVDTSKSQFSLTLRSV TAADTAVYYCAR | DYGG-----------LDY | WGQGT LVTVS S |
| iPS:4 34517 | 21-225_76A7 | VH4\|4-34/D4\|4-17\|RF2/JH4 | QVQLQQW-GAGLLKPSETLSL TCAVYG-GSFS | G-----CYWS | WIRQPPGK GLEWIG | EINH---- SGRTNYNPS LKS | RVTISVDTSENQFSLKLSSV TAADTAVYYCAR | DYGG-----------LDY | WGQGA LVTVS S |
| iPS:4 34519 | 21-225_74C7 | VH4\|4-34/D4\|4-17\|RF2/JH4 | QVQLQQW-GAGLLKPSETLSL TCAVYG-GSLS | G-----SYWS | WIRQPPGK GLEWIG | EINL---- SGSTNYNPS LKS | RVTISVDTSKSQFSLTLRSV TAADTAVYYCAR | DYGG-----------LDY | WGQGT LVTVS S |
| iPS:4 34571 | 21-225_74D2 | VH4\|4-34/D4\|4-17\|RF2/JH4 | QVQLQQW-GAGLLKPSETLSL TCAVYG-GSFS | G-----CYWS | WIRQPPGK GLEWIG | EINH---- SGRTNYNPS LKS | RVTISVDTSENQFSLKLSSV TAADTAVYYCAR | DYGG-----------LDY | WGQGT LVTVS S |
| iPS:4 34637 | 21-225_78E7 | VH4\|4-34/D4\|4-17\|RF2/JH4 | QVQLQQW-GAGLLKPSETLSL TCAVYG-GSLS | G-----SYWS | WIRQPPGK GLEWIG | EINL---- SGSTNYNPS LKS | RVTISVDTSKSQFSLTLRSV TAADTAVYYCAR | DYGG-----------LDY | WGQGT LVTVS S |
| iPS:4 34717 | 21-225_80A6 | VH4\|4-34/D4\|4-17\|RF2/JH4 | QVQLQQW-GAGLLKPSETLSL TCAVYG-GSFS | G-----CYWS | WIRQPPGK GLEWIG | EINH---- SGRTNYNPS LKS | RVTISVDTSEKQFSLKLSSV TAADTAVYYCAR | DYGG-----------LDY | WGQGT LVTVS S |
| iPS:4 34735 | 21-225_80B10 | VH4\|4-34/D4\|4-17\|RF2/JH4 | QVQLQQW-GAGLLKPSETLSL TCAVYG-GSFS | G-----CYWS | WIRQPPGK GLEWIG | EINH---- SGSTNYNPS LKS | RVTISVDTSKNQFSLKLSSV TAADTAVYYCAR | DYGG-----------LDY | WGQGT LVTVS S |

| | | | | | | | | | | |
|---|---|---|---|---|---|---|---|---|---|---|
| iPS:4 34835 | 21-225_83B6 | VH4\|4-34/D4\|4-17\|RF2/J H4 | | QVQLQQW-GAGLLKPSETLSL TCAVYG-GSFS | G-----CYWS | WIRQPPGK GLEWIG | EINH---- SGRTNYNPS LKS | RVTISVDISENQFSLKLSSV TAADTAVYYCAR | DYGG------------ -----------LDY | WGQGT LVTVS S |
| iPS:4 34849 | 21-225_83C10 | VH4\|4-34/D4\|4-17\|RF2/J H4 | | QVQLQQW-GAGLLKPSETLSL TCTVYG-GSFS | G-----YYWS | WIRQPPGK GLEWIG | EINH---- SGSTNFNPS LKS | RVTISVDTSKNQFSLKLSSV TAADTAVFYCAR | DYGG------------ -----------LDY | WGQGT LVTVS S |
| iPS:4 34891 | 21-225_85G6 | VH4\|4-34/D4\|4-17\|RF2/J H4 | | QVQLQQW-GAGLLKPSETLSL TCAVYG-GSFS | D-----CYWS | WIRQPPGK GLEWIG | EINH---- SGRTNYNPS LKS | RVTISVDISENQFSLKLSSV TAADTAVYYCAR | DYGG------------ -----------LDY | WGQGT LVTVS S |
| iPS:4 35183 | 21-225_93E9 | VH4\|4-34/D4\|4-17\|RF2/J H4 | | QVQLQQW-GAGLLKPSETLSL TCAVYG-GSFS | G-----CYWS | WIRQPPGK GLEWIG | EINH---- SGRTNYNPS LKS | RVTISVDTSENKFSLKLSSV TAADTAVYYCAR | DYGG------------ -----------LDY | WGQGT LVTVS S |
| iPS:4 35331 | 21-225_147G8 | VH4\|4-34/D4\|4-17\|RF2/J H4 | | QVQLQQW-GAGLLKPSETLSL TCGVYG-GSFS | A-----YYWS | WIRQPPGK GLEWIG | EINH---- SGSTNYKPS LKS | RVTISVDTSKNQFSLKLSSV TAADTAVYYCAR | DYGV------------ -----------FDY | WGQGT LVTVS S |
| iPS:4 35995 | 21-225_192F8 | VH4\|4-34/D4\|4-17\|RF2/J H4 | | QVQLQQW-GAGLLKPSETLSL TCAVYG-GSFS | G-----CYWS | WIRQPPGK GLEWIG | EINQ---- SGRSNYNPS LKS | RVTISVDTSTNQFSLKLRSV TAADTAVYYCAR | DYGV------------ -----------FDY | WGQGT LVTVS S |
| iPS:4 36027 | 21-225_193E6 | VH4\|4-34/D4\|4-17\|RF2/J H4 | | QVQLQQW-GAGLLKPSETLSL TCAVFG-GSFS | G-----PYWS | WIRQPPGK GLEWIG | ESNH---- SGRTNYNPS LKS | RVTISVDTSKNQFSLRLSSV TAADTAVYYCAR | DYGG------------ -----------LDY | WGQGT LVTVS S |
| iPS:4 36080 | 21-225_195B1 | VH4\|4-34/D4\|4-17\|RF2/J H4 | | QVQLQQW-GAGLLKPSETLSL TCAVSG-GSFR | Y-----YFWS | WIRQSPGK GLEWFG | EINH---- SGRTNYNPS LKS | RVTISVDTSKNQFSLKLRSV TAADTAVYYCAR | DYGA------------ -----------FDI | WGQGT LVTVS S |
| iPS:4 36232 | 21-225_201E1 | VH4\|4-34/D4\|4-17\|RF2/J H4 | | QVQLQQW-GAGLLKPSETLSL TCAVFD-GSFS | P-----YYWS | WIRQPPGK GLEWIG | EVNH---- SGSTNYNPS LKS | RVTISVDTSKNQFSLKLSSV TAADTAVYYCAR | DYGG------------ -----------LDY | WGQGA LVTVS S |
| iPS:4 36238 | 21-225_201B2 | VH4\|4-34/D4\|4-17\|RF2/J H4 | | QVQLQQW-GAGLLKPSETLSL TCAVFG-GSIS | V-----YYWT | WIRQPPGK GLEWIG | EINH---- SGSTNYNPS LKS | RVTISVDTSKNQFSLKLSSV TAADTAVYYCAR | DYGV------------ -----------FDY | WGQGT LVTVS S |
| iPS:4 36256 | 21-225_202D9 | VH4\|4-34/D4\|4-17\|RF2/J H4 | | QVQLQQW-GAGLLKPSETLSL TCAVYD-GSFS | P-----YYWS | WIRQPPGK GLEWIG | EINH---- SGSTNYNPS LKS | RVTISVDTSKNQFSLKLSSV TAADTAVYYCAR | DYGG------------ -----------LDY | WGQGT LVTVS S |
| iPS:4 36302 | 21-225_205G7 | VH4\|4-34/D4\|4-17\|RF2/J H4 | | QVQLQQW-GAGLLKPSETLSL TCAVYG-GSFS | V-----YYWS | WIRQPPGK GLEWIG | ESNQ---- SGRTTYNPS LKS | RVTISVDTSKNQFSLNLISV TAADTAVYYCAR | DYGV------------ -----------FDY | WGQGT LVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 36310 | 21-225_202D11 | VH4\|4-34/D4\|4-17\|RF2/JH4 | | QVQLQQR-GAGLLKPSETLSLTCAAYG-GSFS | G-----PYWS | WIRQPPGKGLEWIG | EINH----SGSTNYNPSLKS | RVTISVDISKNQFSLKLSSVTAADTAVYYCAR | DYGV----------------------LDY | WGQGTLVTVSS |
| iPS:4 36336 | 21-225_208B5 | VH4\|4-34/D4\|4-17\|RF2/JH4 | | QVQLQQW-GAGLLKPSETLSLTCAVFG-GSIS | V-----YYWT | WIRQPPGKGLEWIG | EINH----SGSTNYNPSLKS | RVTISVDISKNQFSLKLSSVTAADTAVYYCAR | DYGV----------------------FDY | WGQGTLVTVSS |
| iPS:4 36340 | 21-225_208A9 | VH4\|4-34/D4\|4-17\|RF2/JH4 | | QVQLQQW-GAGLLKPSEPLSLTCAVYG-GSFS | V-----SYWS | WIRQPPGKGLEWIG | EINH----SGRANYNPSLKS | RVTISIDTSKNQFSLKLSSVTAADTAVYYCAR | DYGV----------------------LDY | WGQGTLVTVSS |
| iPS:4 37340 | 21-225_75G9 | VH4\|4-34/D4\|4-17\|RF2/JH4 | | QVQLQQW-GAGLLKPSETLSLTCAVYG-GSFS | G-----CYWS | WIRQPPGKGLEWIG | EINH----SGRTNYNPSLKS | RVTISVDTSENQFSLKLSSVTAADTAVYYCAR | DYGG----------------------LDY | WGQGTLVTVSS |
| iPS:4 51122 | 21-225_200A1 | VH4\|4-34/D4\|4-17\|RF2/JH4 | | QVQLQQW-GAGLLKPSETLSLTCAVYG-GSFS | V-----YYWS | WIRQPPGKGLEWIG | EINH----SGSTNYNPSLKS | RVTISLDTSKNQFSLKLSSVTAADTAVYYCAR | DYGV----------------------FDY | WGQGTLVTVSS |
| | Germline | | | H_FR1 | H_CDR1 | H_FR2 | H_CDR2 | H_FR3 | H_CDR3 | H_FR4 |
| | VH3\|3-33/D2\|2-15\|RF3/JH1 | | 32316 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DIVVVVAA-----------------TAEYFQH | WGQGTLVTVSS |
| iPS:4 34485 | 21-225_76D2 | VH3\|3-33/D2\|2-15\|RF3/JH1 | | QVQLVES-GGGVVQPGRSLRLSCAASG-FTFS | S-----YGMH | WVRQAPGKGLEWVA | VIWYD---GSNKYYADSVKG | RFTISRDNSKNTLFLQMNSLRAEDTAVYYCAS | DRNIVG-------------------ATYFES | WGQGTLVTVSS |
| iPS:4 34537 | 21-225_74E11 | VH3\|3-33/D2\|2-15\|RF3/JH1 | | QVQLVES-GGGVVQPGRSLRLSCAASG-FTFS | S-----YGMH | WVRQAPGKGLEWVA | VIWYD---GSNKYYADSVKG | RFTISRDNSKNTLFLQMNSLRAEDTAVYYCAS | DRNIVG-------------------ATYFES | WGQGTLVTVSS |
| iPS:4 34673 | 21-225_74E3 | VH3\|3-33/D2\|2-15\|RF3/JH1 | | QVQLVES-GGGVVQPGRSLRLSCTASG-FTFS | S-----YGMH | WVRQAPGKGLEWVA | VIWYD---GSNKYYADSVKG | RFTISRDNSKNTLFLQMNSLRAEDTAVYYCAS | DRNIVG-------------------ATYFES | WGQGTLVTVSS |
| iPS:4 35221 | 21-225_95G2 | VH3\|3-33/D2\|2-15\|RF3/JH1 | | QVQLVES-GGGVVQPGRSLRLSCTASG-FTFS | S-----YGMH | WVRQAPGKGLEWVA | VIWYD---GSNKYYADSVKG | RFTISRDNSKNTLFLQMNSLRAEDTAVYYCAS | DRNIVG-------------------ATYFES | WGQGTLVTVSS |
| | Germline | | | H_FR1 | H_CDR1 | H_FR2 | H_CDR2 | H_FR3 | H_CDR3 | H_FR4 |
| | VH4\|4-39/D7\|7-27\|RF1/JH5 | | 32317 | QLQLQES-GPGLVKPSETLSLTCTVSG-GSIS | SS---SYYWG | WIRQPPGKGLEWIG | SIYY----SGSTYNPSLKS | RVTISVDTSKNQFSLKLSSVTAADTAVYYCAR | LTGN----------------------WFDP | WGQGTLVTVSS |
| IPS:4 34503 | 21-225_74D7 | VH4\|4-39/D7\|7-27\|RF1/JH5 | | QLQLQES-GPGLVKPSETLSLTCSVSG-GSIF | RS---SYYWG | WIRQPPGKGLEWIG | GIYY----SGSTSYNPSLKS | RVTISVDTSENQFSLKLSSVTAADTAVYYCAR | LRPNW-------------------DFDY | WGQGTLVTVSS |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-15/D1\|1-1\|RF2/JH4 | 32318 | EVQLVES-GGGLVKPGGSLRLSCAASG-FTFS | N-----AWMS | WVRQAPGK GLEWVG | RIKSKT-DGGTTDYAA PVKG | RFTISRDDSKNTLYLQMNSL KTEDTAVYYCTT | VQLER----------------YFDY | WGQGT LVTVS S |
| iPS:4 34531 | 21-225_76C9 | VH3\|3-15/D1\|1-1\|RF2/JH4 | EVQLVES-GGGLVKPGGSLRLSCAASG-FSFS | N-----AWMN | WVRQAPGK GLEWVG | RIKNKA-DGGTTDFAA PVKG | RFTISRDDSKHTLYLQMNSL KTEDTAVYYCTT | VGPT----------------TDY | WGQGT LVTVS S |
| iPS:4 34633 | 21-225_74G8 | VH3\|3-15/D1\|1-1\|RF2/JH4 | EVQLVES-GGGLVKPGGSLRLSCAASG-FTFS | N-----AWMN | WVRQAPGK GLEWVG | RIKNKA-DGGTTDYAA PVKG | RFTISRDDSKNTLYLQMNSL KTEDTAVYYCTT | VGAT----------------TDY | WGQGT LVTVS S |
| iPS:4 34671 | 21-225_74F4 | VH3\|3-15/D1\|1-1\|RF2/JH4 | EVQLVES-GGGLVKPGGSLRLSCAASG-FTFS | N-----AWMN | WVRQAPGK GLEWVG | RIKNKI-DGGTTDYAA PVKG | RFTISRDDSKNTLYLQMNSL KTEDTAVYYCTT | VGAT----------------TDY | WGQGT LVTVS S |
| iPS:4 37383 | 21-225_74H8 | VH3\|3-15/D1\|1-1\|RF2/JH4 | EVHLVES-GGGLVKPGGSLRLSCAASG-FTFS | N-----AWMN | WVRQAPGK GLEWVG | RIKSKT-DGGTTDYAA PVKG | RFTISRDDSKNTLYLQMNSL KTEDTAVYYCTT | VGAT----------------TDY | WGQGT LVTVS S |
| | | Germline | H_FR1 | H_CDR1 | H_FR2 | H_CDR2 | H_FR3 | H_CDR3 | H_FR4 |
| | VH3\|3-33/D5\|5-18\|RF3/JH4 | 32319 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAR | GYSYG----------------YYFDY | WGQGT LVTVS S |
| iPS:4 34535 | 21-225_74C8 | VH3\|3-33/D5\|5-18\|RF3/JH4 | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DGSYGY----------------DGLDY | WGQGT LVTVS S |
| iPS:4 34573 | 21-225_77E6 | VH3\|3-33/D5\|5-18\|RF3/JH4 | QVQLVES-GGGVVQSGRSLRLSCAASG-FTFS | S-----YGMH | WVRQAPGK GLEWVA | VIWYD---GSNQNYADS VKG | RFTISRDNSKNTLFLQMNSL RAEDTAVYYCAR | DGSYGY----------------DGLDY | WGQGT LVTVS S |
| iPS:4 34615 | 21-225_76C5 | VH3\|3-33/D5\|5-18\|RF3/JH4 | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DGSYGY----------------DGLDY | WGQGT LVTVS S |
| iPS:4 34669 | 21-225_79F4 | VH3\|3-33/D5\|5-18\|RF3/JH4 | QVQLVES-GGGVVQSGRSLRLSCAASG-FTFS | N-----YGMH | WVRQAPGK GLEWVA | VIWYD---GSNQNYADS VKG | RFTISRDNSKNTLFLQMNSL RAEDTAVYYCAR | DGSYGY----------------DGLDY | WGQGT LVTVS S |
| iPS:4 34737 | 21-225_74G6 | VH3\|3-33/D5\|5-18\|RF3/JH4 | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DGSYGY----------------DGLDY | WGQGT LVTVS S |
| iPS:4 34741 | 21-225_80C11 | VH3\|3-33/D5\|5-18\|RF3/JH4 | QVQLVES-GGGVVQPGRSLRLSCAASG-FTFS | S-----YGMH | WVRQAPGK GLEWVA | VIWYD---GSNKNYADS VKG | RFTISRDNSKNTLSLQMNSL RAEDTAVYYCAR | DGSYGY----------------DGLDY | WGQGT LVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 34867 | 21-225_79A12 | VH3\|3-33/D5\|5-18\|RF3/JH4 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DGSYGY-------------------DGLDY | WGQGT LVAVS S |
| | Germline | | | H_FR1 | H_CDR1 | H_FR2 | H_CDR2 | H_FR3 | H_CDR3 | H_FR4 |
| | VH4\|4-34/D3\|3-10\|RF2/JH6 | | 32320 | QVQLQQW-GAGLLKPSETLSLTCAVYG-GSFS | G-----YYWS | WIRQPPGK GLEWIG | EINH----SGSTNYNPS LKS | RVTISVDTSKNQFSLKLSSV TAADTAVYYCAR | YYYGSGSYYN-----------YYYYGMDV | WGQGT TVTVS S |
| iPS:4 34539 | 21-225_74A2 | VH4\|4-34/D3\|3-10\|RF2/JH6 | | QVQVQQW-GAGLLKPSETLSLTCAVYG-GSFT | D-----YYWS | WIRQPPGK GLEWIG | EINH----SGDTNYNPS LKS | RVTISVDTSKNQFSLKLSSV TAADTAVYYCAR | EFPYSGSY-------------LYYYGMDV | WGQGT TVTVS S |
| iPS:4 37248 | 21-225_97H3 | VH4\|4-34/D3\|3-10\|RF2/JH6 | | QVQVQQW-GAGLLKPSETLSLTCAVYG-GSFT | D-----YYWS | WIRQPPGK GLEWIG | EINH----SGDTNYNPS LKS | RVTISVDTSKNQFSLKLSSV TAADTAVYYCAR | EFPYSGSY-------------LYYYGMDV | WGQGT TVTVS S |
| iPS:4 37320 | 21-225_75A1 | VH4\|4-34/D3\|3-10\|RF2/JH6 | | QVQVQQW-GAGLLKHSETLSLTCAVYG-GSFT | D-----YYWS | WIRQPPGK GLEWIG | EINH----SGDTNYNPS LKS | RVTISVDTSKNQFSLKLSSV TAADTAVYYCAR | EFPYSGSY-------------LYYYGMDV | WGQGT TVTVS S |
| | Germline | | | H_FR1 | H_CDR1 | H_FR2 | H_CDR2 | H_FR3 | H_CDR3 | H_FR4 |
| | VH3\|3-13/D3\|3-9\|RF1/JH6 | | 32321 & 32322 | EVQLVES-GGGLVQPGGSLRLSCAASG-FTFS | S-----YDMH | WVRQATGK GLEWVS | AIGT----AGDTYYPGS VKG | RFTISRENAKNSLYLQMNSL RAGDTAVYYCAR | VLRYFDWLL*-----------YYYYGMDV | WGQGT TVTVS S |
| iPS:4 34563 | 21-225_75D8 | VH3\|3-13/D3\|3-9\|RF1/JH6 | | EVQLVES-GGGLVQPGGSLRLSCAASG-FTFS | N-----YDMH | WVRQATGK GLEWVS | AIGT----AGDTYYPGS VKG | RFTISRENAKNSLYLQMNSL RAGDTAVYYCAR | VLDYGDSLG-------------YYYYGMDV | WGQGT TVTVS S |
| iPS:4 35009 | 21-225_89G4 | VH3\|3-13/D3\|3-9\|RF1/JH6 | | EVQLVES-GGGLVQPGGSLRLSCAASG-FTFS | S-----YDMH | WVRQATGK GLEWVS | AIGT----AGDTYYPGS VKG | RFTISRENAKNSLYLQMNSL RAGDTAVYFCAR | ALDYGDSLG-------------YYYYGMDV | WGQGT TVTVS S |
| iPS:4 35059 | 21-225_90C11 | VH3\|3-13/D3\|3-9\|RF1/JH6 | | EVQLVES-GGGLVQPGGSLRLSCAASG-FTFS | N-----YDMH | WVRQATGK GLEWVS | AIGT----AGDTYYPCS VKG | RFTISRENAKNSLYLQMNSL RAGDTAVYYCAR | VLDYGDSLG-------------YYYYGMDV | WGQGT TVTVS S |
| iPS:4 35103 | 21-225_92B2 | VH3\|3-13/D3\|3-9\|RF1/JH6 | | EVQLVES-GGGLVQPGGSLRLSCAASG-FTFS | N-----YDMH | WVRQATGK GLEWVS | AIGT----AGDTYYPGS VKG | RFTISRENAKNSLYLQMNSL RAGDTAVYFCAR | ALDYGDSLG-------------YYYYGMDV | WGQGT TVTVS S |
| iPS:4 37371 | 21-225_74D8 | VH3\|3-13/D3\|3-9\|RF1/JH6 | | EVQLVES-GGGLVQPGGSLRLSCAASG-FTFS | N-----YDMH | WVRQATGK GLEWVS | AIGT----AGDTYYPGS VKG | RFTISRENAKNSLYLQMNSL RAGDTAVYYCAR | VLDYGDSLG-------------YYYYGMDV | WGQGT TVTVS S |
| | Germline | | | H_FR1 | H_CDR1 | H_FR2 | H_CDR2 | H_FR3 | H_CDR3 | H_FR4 |
| | VH1\|1-08/D1\|1-26\|RF3/JH4 | | 32323 | QVQLVQS-GAEVKKPGASVKVSCKASG-YTFT | S-----YDIN | WVRQATGQ GLEWMG | WMNPN---SGNTGYAQK FQG | RVIMTRNTSISTAYMELSSL RSEDTAVYYCAR | YSGSY-------------------YYFDY | WGQGT LVTVS S |

| ID | Name | VH | Num | H_FR1 | H_CDR1 | H_FR2 | H_CDR2 | H_FR3 | H_CDR3 | H_FR4 |
|---|---|---|---|---|---|---|---|---|---|---|
| iPS:4 34711 | 21-225_80H3 | VH1\|1-08\|D1\|1-26\|RF3\|JH4 | | QVQLVQS-GAEVKKPGASVKVSCKASG-YTLT | N-----YDIN | WVRQATGQ GLEWMG | WMNPN--- SGNTGYAQK FQG | RVTMTRNTSISTAYMELSSL RSEDTAVYYCGS | TSGWN----------- ----------FFDY | WGQGT LVTVS S |
| iPS:4 34901 | 21-225_85H9 | VH1\|1-08\|D1\|1-26\|RF3\|JH4 | | QVQLVQS-GAEVKKPGASVKVSCKASG-YTLT | N-----YDIN | WVRQATGQ GLEWMG | WMNPN--- SGNTGYAQK FQG | RVTMTRNTSISTAYMELSSL RSEDTAVYYRGS | TSGWN----------- ----------FFDY | WGQGT LVTVS S |
| iPS:4 35167 | 21-225_92F12 | VH1\|1-08\|D1\|1-26\|RF3\|JH4 | | QVQLVQS-GAEVKKPGASVKVSCKASG-YTLT | N-----YDIN | WVRQATGQ GLEWMG | WMNPN--- SGNTGYAQK FQG | RVTMTRNTSISTAYMELSSL RSEDTAVYYRGS | TSGGK----------- ----------FFDY | WGQGT LVTVS S |
| iPS:4 35215 | 21-225_94E12 | VH1\|1-08\|D1\|1-26\|RF3\|JH4 | | QVQLVQS-GAEVKKPGASVKVSCKASG-YTLT | N-----YDIN | WVRQATGQ GLEWMG | WMNPN--- SGNTGYAQK FQG | RVTMTRNTSISTAYMELSSL RSEDTAVYYRGS | TSGWK----------- ----------FFDY | WGQGT LVTVS S |
| iPS:4 37356 | 21-225_74B1 | VH1\|1-08\|D1\|1-26\|RF3\|JH4 | | QVQLVQS-GAEVKKPGASVKVSCKASG-YTLT | N-----YDIN | WVRQATGQ GLEWMG | WMNPN--- SGNTGYAQK FQG | RVTMTRNTSISTAYMELSSL RSEDTAVYYCGS | TSGWN----------- ----------FFDY | WGQGT LVTVS S |
| | Germline | | | H_FR1 | H_CDR1 | H_FR2 | H_CDR2 | H_FR3 | H_CDR3 | H_FR4 |
| VH1\|1-08\|D1\|1-1\|RF1\|JH6 | | | 32324 | QVQLVQS-GAEVKKPGASVKVSCKASG-YTFT | S-----YDIN | WVRQATGQ GLEWMG | WMNPN--- SGNTGYAQK FQG | RVTMTRNTSISTAYMELSSL RSEDTAVYYCAR | GTTGTYY--------- -------YYYGMDV | WGQGT TVTVS S |
| iPS:4 34815 | 21-225_74A11 | VH1\|1-08\|D1\|1-1\|RF1\|JH6 | | QVQLVQS-GAEVKKPGASVKVSCKASG-YTFT | S-----YDIN | WVRQATGQ GLEWMG | WMNPN--- SGNTGYAQK FQG | RVTMTWNTSKSTAYMELSSL RSEDTAVYYCAR | GFYDTLTGS------- ------GYYYVMDV | WGQGT TVTVS S |
| iPS:4 35253 | 21-225_96A4 | VH1\|1-08\|D1\|1-1\|RF1\|JH6 | | QVQLVQS-GAEVKKPGASVKVSCKASG-YTFT | S-----YDIN | WVRQATGH GLEWMG | WMNPN--- SGNTGYAQK FQG | RVTMTWNTSKSTAYMELSSL RSEDTAVYYCAR | GFYDTLTGS------- ------GYYYVMDV | WGQGT TVTVS S |
| | Germline | | | H_FR1 | H_CDR1 | H_FR2 | H_CDR2 | H_FR3 | H_CDR3 | H_FR4 |
| VH1\|1-08\|D3\|3-22\|RF2\|JH6 | | | 32325 | QVQLVQS-GAEVKKPGASVKVSCKASG-YTFT | S-----YDIN | WVRQATGQ GLEWMG | WMNPN--- SGNTGYAQK FQG | RVTMTRNTSISTAYMELSSL RSEDTAVYYCAR | YYYDSSGYYY------ -----YYYYYGMDV | WGQGT TVTVS S |
| iPS:4 34977 | 21-225 88A5 | VH1\|1-08\|D3\|3-22\|RF2\|JH6 | | QVQLVQS-GAEVKKPGASVKVSCKASG-YTFT | S-----YDIN | WVRQATGQ GLEWMG | WMNPN--- SGNTGYAQK FQG | RVTMTWNTSIRTAYMELSSL RSEDTAVYYCAR | GFYDFLTGYS------ ----PTYYYYDMDV | WGQGT TVTVS S |
| iPS:4 35259 | 21-225_96C6 | VH1\|1-08\|D3\|3-22\|RF2\|JH6 | | QVQLVQS-GAEVKKPGASVKVSCKASG-YTFT | S-----YDIN | WVRQATGQ GLEWMG | WMNPN--- SRNTGYAQK FQG | RVTMTWNTSISTAYMELSSL RSEDTAVYYCAR | GGYDVLPGN------- ------NYYYDMDV | WGQGT TVTVS S |
| | Germline | | | H_FR1 | H_CDR1 | H_FR2 | H_CDR2 | H_FR3 | H_CDR3 | H_FR4 |
| VH3\|3-33\|D2\|2-15\|RF3\|JH3 | | | 32326 | QVQLVES-GGGVVQPGRSLRLSCAASG-FTFS | S-----YGMH | WVRQAPGK GLEWVA | VIWYD--- GSNKYYADS VKG | RFTISRDNSKNTLYLQMNSL RAEDTAVYYCAR | DIVVVVA--------- -------ATDAFDI | WGQGT MVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 35291 | 21-225_146E1 | VH3\|3-33/D2\|2-15\|RF3/JH3 | | QVQLVES-GGGVVQPGRSLRLSCEASG-ITFS | S-----YGMH | WVRQAPGKGLEWVA | IIWYD---GSNKYYADSVKG | RFTISRDNSKNTLFLQMNSLRAEDTAVYYCAR | DRLVGAT------------------ADAFDI | WGQGTMVTVSS |
| iPS:4 36360 | 21-225_210H11 | VH3\|3-33/D2\|2-15\|RF3/JH3 | | QVQLVES-GGGVVQPGRSLRLSCAASG-FTFS | S-----HGMH | WVRQAPGKGLEWVA | VTWYD---GSDKYYADSVKG | RFTISRDNSKNTLYLQMNSLRAEDTAVYYCAR | DRLVGAT------------------TDAFDI | WGQGTMVTVSS |
| iPS:4 36370 | 21-225_211A6 | VH3\|3-33/D2\|2-15\|RF3/JH3 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVFYCAR | DRLVGY--------------------DGFDI | WGQGTMVTVSS |
| iPS:4 36392 | 21-225_213B3 | VH3\|3-33/D2\|2-15\|RF3/JH3 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DRLVGY--------------------DGFDI | WGQGTMVTVSS |
| iPS:4 36406 | 21-225_214E4 | VH3\|3-33/D2\|2-15\|RF3/JH3 | | QVQLVES-GGGVVQPGRSLRLSCAASG-FTFS | S-----YGMH | WVRQAPGKGLEWVA | VIWYD---GSNENYADSVKG | RITISRDNSKNTLYLQMNSLRAEDTAVYYCAR | DRLVGY--------------------DGCDI | WGQGAMVTVSS |
| iPS:4 37326 | 21-225_75C10 | VH3\|3-33/D2\|2-15\|RF3/JH3 | | QVQLVES-GGGVVQPGRSLRLSCAASG-FTFS | S-----YGMH | WVRQAPGKGLEWVA | VIWYD---GSDKYYADSVKG | RFTISRDNSKNTLYLQMNSLRAEDTAVYYCAR | DRLVGAT------------------VDAFDI | WGQGTMVTVSS |
| | VH4\|4-39/D7\|7-27\|RF1/JH4 | | 32327 | QLQLQES-GPGLVKPSETLSLTCTVSG-GSIS | SS----SYYWG | WIRQPPGKGLEWIG | SIYY----SGSTYYNPSLKS | RVTISVDTSKNQFSLKLSSVTAADTAVYYCAR | LTGY---------------------------FDY | WGQGTLVTVSS |
| iPS:4 35293 | 21-225_146F1 | VH4\|4-39/D7\|7-27\|RF1/JH4 | | QLQLQES-GPGLVKPSETLSLTCTVSG-GSIS | RS----SYYWG | WIRQPPGKGLEWIG | SIYY----SGSTSYNPSLKS | RVTISVDTSKNQFSLKLSSVTAADTAVYYCAR | LDLLW------------------SFDY | WGQGTLVTVSS |
| iPS:4 35361 | 21-225_148E11 | VH4\|4-39/D7\|7-27\|RF1/JH4 | | QLQLQES-GPGLVKPSETLSLTCTVSG-VSIS | RS----SYYWG | WIRQPPGKGLEWIG | SIYY----SGSTSYNPSLKS | RVTISVDTSKNQFSLKLSSVTAADTAVFYCAR | LDPQW------------------SFDY | WGQGILVTVSS |
| iPS:4 35449 | 21-225_152H9 | VH4\|4-39/D7\|7-27\|RF1/JH4 | | QLQLQES-GPGLVKPSETLSLTCTVSG-GSIS | RS----SYYWG | WIRQPPGKGLEWIG | SIYY----SGSASYNPSLKS | RVTISVDTSKNQFSLKLSSVTAADTAVFYCAR | LDLQW------------------SFDF | WGQGTLVTVSS |
| iPS:4 35499 | 21-225_156G1 | VH4\|4-39/D7\|7-27\|RF1/JH4 | | QLQLQES-GPGLVKPSETLSLTCTVSG-GSIS | RS----SYYWG | WIRQPPGKGLEWIG | SIYY----SGSASYNPSLKS | RVTISVDTSKNQFSLKLSSVTAADTAVFYCAR | LDLQW------------------SFDF | WGQGTLVTVSS |
| iPS:4 35587 | 21-225_160H3 | VH4\|4-39/D7\|7-27\|RF1/JH4 | | QLQLQES-GPGLVKPSETLSLTCTVSG-GSIS | RS----SYYWG | WIRQPPGKGLEWIG | SIYY----SGSTSYNPSLES | RVTISVDTSKNQFSLKLSSVTAADTAVFYCAR | LSQRW------------------DFDY | WGQGTLVTVSS |

| iPS | Clone | Germline | No. | H_FR1 | H_CDR1 | H_FR2 | H_CDR2 | H_FR3 | H_CDR3 | H_FR4 |
|---|---|---|---|---|---|---|---|---|---|---|
| iPS:4 03868 | 21-225_19D11 | VH4|4-39/D7|7-27|RF1/JH4 | | QLQLQES-GPGLVKPSETLSL TCTVSG-GSIS | RS---SYYWG | WIRQPPGK GLDWIG | SIYY---- SGSANYNPS LKS | RVTISVDISKNQFSLKLSSV TAADAAVYYCAR | LDRGW---------- ----------SFDY | WGQGT LVTVS S |
| | Germline | | | H_FR1 | H_CDR1 | H_FR2 | H_CDR2 | H_FR3 | H_CDR3 | H_FR4 |
| | VH3|3-23/D4|4-17|RF2/JH5 | | 32328 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | DYGDY---------- ---------NWFDP | WGQGT LVTVS S |
| iPS:4 35295 | 21-225_146H1 | VH3|3-23/D4|4-17|RF2/JH5 | | EVQLLES-GGGLVQPGGSLRL SCAASG-FTFS | S-----YAMS | WVRQAPGK GLEWVS | AISGR--- GGNTFYADS VKG | RFTISRDNSKNTLYLQMNSL RAEDTAVYYCAK | RVTDYGG--------- --------NDWFDP | WGQGT LVTVS S |
| iPS:4 35307 | 21-225_146E9 | VH3|3-23/D4|4-17|RF2/JH5 | | EVQLLES-GGGLVQPGGSLRL SCAASG-FTFS | S-----YAMS | WVRQAPGK GLEWVS | AISGR--- GGNTFYADS VKG | RFTISRDNSKKTLYLQMNSL RAEDTAVYYCAK | RVTDYGG--------- --------NDWFDP | WGQGT LVTVS S |
| iPS:4 35347 | 21-225_148C4 | VH3|3-23/D4|4-17|RF2/JH5 | | EVQLLES-GGGLVQPGGSLRL SCAASG-FTFS | S-----YAMN | WVRQAPGK GLEWVS | AISGS--- GGNTFYADS VKG | RFTISRDNSKNTLYLQMNSL RAEDTAVYYCAK | RVTDYGG--------- --------NDWFDP | WGQGT LVTVS S |
| iPS:4 35355 | 21-225_148H9 | VH3|3-23/D4|4-17|RF2/JH5 | | EVQLLES-GGGLVQPGGSLRL SCAASG-FTFS | S-----YAMS | WVRQAPGK GLEWVS | AISGR--- GGNTFYADS VKG | RFTISRDNSKNTLYLQMNSL RAEDTAVYYCAK | RVTDYGG--------- --------NDWFDP | WGQGT LVTVS S |
| iPS:4 35371 | 21-225_149A3 | VH3|3-23/D4|4-17|RF2/JH5 | | EVQLLES-GGGLVQPGGSLRL SCAASG-FTFS | N-----YAMT | WVRQAPGK GLEWVS | AISGR--- GGNTFYADS VKG | RFTISRDNSKNTLYLQMNSL RAEDTAVYYCTK | RVTDYGG--------- --------NDWFDP | WGQGT LVTVS S |
| iPS:4 35415 | 21-225_150C11 | VH3|3-23/D4|4-17|RF2/JH5 | | EVQLLES-GGGLVQPGGSLRL SCAASG-FTFS | S-----YAMN | WVRQAPGK GLEWVS | AISGS--- GGNTFYADS VKG | RFTISRDNSKNTLNLQMSSL RAEDTAVYYCAK | RVTDYGG--------- --------NDWFDP | WGQGT LVTVS S |
| iPS:4 35419 | 21-225_150C12 | VH3|3-23/D4|4-17|RF2/JH5 | | EVQLLES-GGGLVQPGGSLRL SCAASG-FTFS | S-----YAMS | WVRQAPGK GLEWVS | AISGR--- GGNTFYADS VKG | RFTISRDNSKNTLYLQMNSL RAEDTAVYYCAK | RVTDYGG--------- --------NDWFDP | WGQGT LVTVS S |
| iPS:4 35425 | 21-225_151B12 | VH3|3-23/D4|4-17|RF2/JH5 | | EVQLLES-GGGLVQPGGSLRL SCAASG-FTFS | S-----YAMN | WVRHAPGK GLEWVS | AISGS--- GKNTFYADS VKG | RFTISRDNSKNTLYLQMNSL RAEDTAVYYCAK | RVTDYGG--------- --------NDWFDP | WGQGT LVTVS S |
| iPS:4 35431 | 21-225_152D2 | VH3|3-23/D4|4-17|RF2/JH5 | | EVQLLES-GGGLVQPGGSLRL SCAASG-FTFS | S-----YAMS | WVRQAPGK GLEWVS | AISGR--- GGNTFYADS VKG | RFTISRDNSKKTLYLQMNSL RAEDTAVYYCAK | RVTDYGG--------- --------NDWFDP | WGQGT LVTVS S |
| iPS:4 35439 | 21-225_152G4 | VH3|3-23/D4|4-17|RF2/JH5 | | EVQLLES-GGGLVQPGGSLRL SCAASG-FTFS | S-----YAMS | WVRQAPGK GLEWVS | AISGR--- GGNTFYADS VKG | RFTISRDNSKNTLYLQMNSL RAEDTAVYYCAK | RVTDYGG--------- --------NDWFDP | WGQGT LVTVS S |

EP 4 435 105 A2

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35455 | 21-225_152B11 | VH3\|3-23/D4\|4-17\|RF2/JH5 | | EVQLLES-GGGLVQPGGSLRLSCAASG-FTFS | S-----YAMN | WVRQAPGKGLEWVS | AISGR---GGNTFYADSVKG | RFTISRDNSKNTLYLQMNSLRAEDTAVYYCAK | RVTDYGG-----------------NDWFDP | WGQGTLVTVSS |
| iPS:4 35487 | 21-225_155C4 | VH3\|3-23/D4\|4-17\|RF2/JH5 | | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | RVTDYGG-----------------NDWFDP | WGQGTLVTVSS |
| iPS:4 35503 | 21-225_156E4 | VH3\|3-23/D4\|4-17\|RF2/JH5 | | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | RVTDYGG-----------------NDWFDP | WGQGTLVTVSS |
| | | Germline | | H_FR1 | H_CDR1 | H_FR2 | H_CDR2 | H_FR3 | H_CDR3 | H_FR4 |
| | VH3\|3-30/D3\|3-22\|RF3/JH6 | | 32329 | QVQLVES-GGGVVQPGRSLRLSCAASG-FTFS | S-----YGMH | WVRQAPGKGLEWVA | VISYD---GSNKYYADSVKG | RFTISRDNSKNTLYLQMNSLRAEDTAVYYCAK | IIMIVVVIT-------------YYYYYGMDV | WGQGTTVTVSS |
| iPS:4 35297 | 21-225_146B3 | VH3\|3-30/D3\|3-22\|RF3/JH6 | | QVQLVES-GGGVVQPGRSLRLSCAASG-FTFS | S-----YGMH | WVRQAPGKGLEWVA | VIWYD---GSYKYYADSVKG | RFTISRDNSKNTLDLQMNSLRAEDTAVYYCVK | MGIEVAVD--------------YYYGMDV | WGQGTTVTVSS |
| | | Germline | | H_FR1 | H_CDR1 | H_FR2 | H_CDR2 | H_FR3 | H_CDR3 | H_FR4 |
| | VH4\|4-30.1/D1\|1-1\|RF1/JH3 | | 32330 | QVQLQES-GPGLVKPSQILSLTCTVSG-GSIS | SG---GYYWS | WIRQHPGKGLEWIG | YIYY----SGSTYYNPSLKS | RVTISVDISKNQFSLKLSSVTAADTAVYYCAR | GITGT-------------------DAFDI | WGQGTMVTVSS |
| iPS:4 35301 | 21-225_146G4 | VH4\|4-30.1/D1\|1-1\|RF1/JH3 | | QVQLQES-GPGLVKPSQTLSLNCTVSG-GSIS | NS---GYYWS | WIRQHPGKGLEWIG | YSYY----SGSTYYNPSLKS | RITISVDISNNQFSLKLTSVTAADTAVYYCAR | GKYNWN--------------------HAFDI | WGQGTMVTVSS |
| | | Germline | | H_FR1 | H_CDR1 | H_FR2 | H_CDR2 | H_FR3 | H_CDR3 | H_FR4 |
| | VH3\|3-21/D6\|6-13\|RF1/JH4 | | 32331 | EVQLVES-GGGLVKPGGSLRLSCAASG-FTFS | S-----YSMN | WVRQAPGKGLEWVS | SISGS---SSYIYYADSVKG | RFTISRDNAKNSLYLQMNSLRAEDTAVYYCAR | GYSSSW------------------YYFDY | WGQGTLVTVSS |
| iPS:4 35313 | 21-225_146G11 | VH3\|3-21/D6\|6-13\|RF1/JH4 | | EVQLVES-GGGLVKPGGSLRLSCAASG-FTFS | S-----YSMN | WVRQAPGKGLEWVS | SISGS---GSYTYYADSVKG | RFTISRDNAKNSLYLQMNSLRAEDTAVYYCAR | GSSSS-------------------GFDY | WGQGTLVTVSS |
| iPS:3 93808 | 21-225_1A2 | VH3\|3-21/D6\|6-13\|RF1/JH4 | | EVQLVES-GGGLVKPGGSLRLSCAASG-FTFS | S-----YTMN | WVRQAPGKGLEWVS | SISGS---GSYIYYADSVKG | RFTISRDNAKNSLYLQMNSLRAEDTAVYYCAR | GSSSS-------------------GFDY | WGQGTLVTVSS |
| iPS:3 93958 | 21-225_5H2 | VH3\|3-21/D6\|6-13\|RF1/JH4 | | EVQLVES-GGGLVKPGGSLRLSCAASG-FTFS | S-----YTMN | WVRQAPGKGLEWVS | SISGS---SSYIYYADSVKG | RFTISRANAKNSLYLQMNSLRAEDTAVYYCAR | GSSSS-------------------GFDY | WGQGTLVTVSS |
| | | Germline | | H_FR1 | H_CDR1 | H_FR2 | H_CDR2 | H_FR3 | H_CDR3 | H_FR4 |
| | VH4\|4-30.4/D5\|5-18\|RF3/JH6 | | 32332 | QVQLQES-GPGLVKPSQTLSLTCTVSG-GSIS | SG---DYYWS | WIRQPPGKGLEWIG | YIYY----SGSTYYNPSLKS | RVTISVDTSKNQFSLKLSSVTAADTAVYYCAR | GYSYGYYY----------------YYYGMDV | WGQGTTVTVSS |

2959

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 35317 | 21-225_147D2 | VH4\|4-30.4/D5\|5-18\|RF3/JH6 | | QVLLQES-GPGLVKPSQTLSL TCAVSG-GPIS | SG---DYYWN | WIRQRPGK GLEWIG | FIYY---- TGSTYYNPS LKS | RVSISEDISENQFSLNLSSV TAADTAVYYCAR | GGAYYS---------- --------YYGMDV | WGQGT TVTVS S |
| | **VH4\|4-30.1/D5\|5-24\|RF3/JH3** | | 32333 | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV TAADTAVYYCAR | RDGYNY---------- --------DAFDI | WGQGT MVTVS S |
| iPS:4 35319 | 21-225_147E3 | VH4\|4-30.1/D5\|5-24\|RF3/JH3 | | QVQLQES-GPGLVKPSQTLSL TCTVSG-GSIT | NS---GYYYS | WIRQHPGK GLEWIG | YIYY---- SGGTYYNPS LKS | RITISVDTSNNQFSLKLSSV TAADTAVYYCAR | GGYNWN---------- --------HAFDF | WGQGT MVTVS S |
| iPS:4 35383 | 21-225_149D7 | VH4\|4-30.1/D5\|5-24\|RF3/JH3 | | QVQLQES-GPGLVKPSQTLSL NCTVSG-GSIS | NS---GYYWS | WIRQHPGK GLEWIG | YSYY---- SGSTYYNPS LKS | RITISVDTSNNQFSLKLSSV TAADTAVYYCAR | GGYNWN---------- --------HAFDI | WGQGT MVIVS S |
| iPS:4 35443 | 21-225_152E7 | VH4\|4-30.1/D5\|5-24\|RF3/JH3 | | QVQLQES-GPGLVKPSQTLSL NCTVSG-GSIS | NS---GYYWS | WIRQHPGK GLEWIG | YSYY---- SGSTYYNPS LKS | RITISVDTSNNQFSLNLSSV TAADTAVYYCAR | GGYNWN---------- --------HAFDI | WGQGT MVTVS S |
| iPS:4 35465 | 21-225_153A6 | VH4\|4-30.1/D5\|5-24\|RF3/JH3 | | QVQLQES-GPGLVKPSQTLSL NCTVSG-GSIS | NS---GYYWS | WIRQHPGK GLEWIG | YSYY---- SGSTYYNPS LKS | RITISVDTSNNQFSLNLSSV TAADTAVYYCAR | GGYNWN---------- --------HAFDI | WGQGT MVTVS S |
| iPS:4 42568 | 21-225_149D8 | VH4\|4-30.1/D5\|5-24\|RF3/JH3 | | QVQLQES-GPGLVKPSQTLSL NCTVSG-GSIS | NS---GYYWS | WIRQHPGK GLEWIG | YSYY---- SGSTYYNPS LKS | RITISVDTSNNQFSLKLSSV TAADTAVYYCAR | GGYNWN---------- --------HAFDI | WGQGT MVTVS S |
| | Germline | | | H_FR1 | H_CDR1 | H_FR2 | H_CDR2 | H_FR3 | H_CDR3 | H_FR4 |
| | **VH3\|3-48/D4\|4-11\|RF2/JH4** | | 32334 | EVQLVES-GGGLVQPGGSLRL SCAASG-FTFS | S-----YSMN | WVRQAPGK GLEWVS | YISSS--- SSTIYYADS VKG | RFTISRDNAKNSLYLQMNSL RDEDTAVYYCAR | DYSNY---------- --------YFDY | WGQGT LVTVS S |
| iPS:4 35333 | 21-225_147E9 | VH3\|3-48/D4\|4-11\|RF2/JH4 | | EVQLVES-GGGLVQPGGSLRL SCAASG-FTFS | S-----YSMN | WVRQAPGK GLEWVS | SISGR--- NTTIYYADS VKG | RFTISRDNAKNSLYLQMNSL RDEDTAVYYCSR | DRG------------ ------------SC | WGQGT LVTVS S |
| iPS:4 35637 | 21-225_163E2 | VH3\|3-48/D4\|4-11\|RF2/JH4 | | EVQLVES-GGGLVQPGGSLRL SCAASG-FTFS | S-----YSMN | WVRQAPGK GLEWVS | STSGS--- STYIYYADS VKG | RFTISRDNAKNLVYLQMNSL RPEDTAVYYCAR | DRG------------ -----------SL | WGQGT LVTVS S |
| | Germline | | | H_FR1 | H_CDR1 | H_FR2 | H_CDR2 | H_FR3 | H_CDR3 | H_FR4 |
| | **VH1\|1-02/D2\|2-15\|RF3/JH4** | | 32335 | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | DIVVVVA- --------ATYFDY | WGQGT LVTVS S |
| iPS:4 35351 | 21-225_148B6 | VH1\|1-02/D2\|2-15\|RF3/JH4 | | QVQLVQS-GAEVKKPGASVKV SCKASG-YTFT | G-----YYMH | WVRQAPGQ GLEWMG | WIHPN--- NGGTNYAQT FQG | RVTMTRDTSISTVYMELSRL RSDDTAVYYCAR | DPVVVP---------- --------AAPFDY | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-30.1/D5\|5-12\|RF3/JH6 | 32336 | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV TAADTAVYYCAR | GYSGYDYY---------- ------YYYYGMDV | WGQGT TVTVS S |
| iPS:4 35363 | 21-225_148F12 | VH4\|4-30.1/D5\|5-12\|RF3/JH6 | QVQLQES-GPGLVKPSQTLSL TCTVSG-GSIS | NG---GYYWN | WIRQHPGK GLEWIG | YIYY---- SGSTYYNPS LKS | QITISVDTSKDQFSLRLSSV TAADTAVYYCAR | YSTYDY---------- --------YYGMDV | WGQGT TVTVS S |
| iPS:4 35377 | 21-225_149G5 | VH4\|4-30.1/D5\|5-12\|RF3/JH6 | QVQLQES-GPGLVKPSQTLSL TCTVSG-GSIS | NG---GYYWN | WIRQHPGK GLEWIG | YIYY---- SGSTYYNPS LKS | RVTISVDTSKDQFSLRLSSV TAADTAVYYCAR | YSTYDY---------- --------YYGMDV | WGQGT TVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-48/D6\|6-6\|RF2/JH4 | 32337 | EVQLVES-GGGLVQPGGSLRL SCAASG-FTFS | S-----YSMN | WVRQAPGK GLEWVS | YISSS--- SSTIYYADS VKG | RFTISRDNAKNSLYLQMNSL RDEDTAVYYCAR | SIAAR----------- ----------YFDY | WGQGT LVTVS S |
| iPS:4 35409 | 21-225_150G8 | VH3\|3-48/D6\|6-6\|RF2/JH4 | EVQLVES-GGGLVQPGGSLRL SCAASG-FTFS | T-----YSMT | WVRQAPGK GLDWVS | YISRS--- SSTIYYADS VKG | RFSISRDNAKNSLYLQMNSL RDEDTALYYCAR | SAFS------------ ----------PFDY | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-30.3/D5\|5-18\|RF2/JH5 | 32338 | QVQLVES-GGGVVQPGRSLRL SCAASG-FTFS | S-----YAMH | WVRQAPGK GLEWVA | VISYD--- GSNKYYADS VKG | RFTISRDNSKNTLYLQMNSL RAEDTAVYYCAR | WIQLWL----------- ---------NWFDP | WGQGT LVTVS S |
| iPS:4 35427 | 21-225_151C9 | VH3\|3-30.3/D5\|5-18\|RF2/JH5 | QVQLVES-GGGVVQPGRSLRL SCAASG-FTFS | S-----YGMH | WVRQAPGK GLEWVA | VISYD--- GSNKYYADS VKG | RFTISRDNSKNTLYLQMNSL RAEDTAVYYCAR | GVLLWFGE-------- ------LEDDWFDP | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-33/D3\|3-22\|RF2/JH4 | 32339 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | YYYDSSG--------- --------YYYYFDY | WGQGT LVTVS S |
| iPS:4 35441 | 21-225_152F6 | VH3\|3-33/D3\|3-22\|RF2/JH4 | QVQLVES-GGGVVQPGRSLRL SCAASG-FTFS | S-----YGMH | WVRQAPGK GLEWVA | IIWYD--- GSYKYYADS VKG | RFTISRDNSKNTLYLQMNSL RAEDTAVYYCAR | EGYDFW---------- --------SGYLGY | WGQGT LVTVS S |
| iPS:4 35457 | 21-225_152C11 | VH3\|3-33/D3\|3-22\|RF2/JH4 | QVQLVES-GGGVVQPGRSLRL SCAASG-FTFR | N-----YGMH | WVRQAPGK GLEWVA | IIWYD--- GSNKYYADS VKG | RFTISRDNSKNTLYLQMNSL RAEDTAVYYCAR | EAYDFW---------- --------SGYFDY | WGQGI LVTVS S |
| iPS:4 35463 | 21-225_153D2 | VH3\|3-33/D3\|3-22\|RF2/JH4 | QVQLVES-GGGVVQPGRSLRL SCAASG-FTFS | S-----YGMH | WVRQAPGK GLEWVA | IIWYD--- GSYKYYADS VKG | RFTISRDNSKNTVYLQMNSL RAEDTAVYYCAR | EGYDFW---------- --------SGYLGY | WGQGT LVTVS S |
| iPS:4 35531 | 21-225_157G8 | VH3\|3-33/D3\|3-22\|RF2/JH4 | QVQLVES-GGGVVQPGRSLRL SCAASG-FTFS | N-----YGMH | WVRQAPGK GLEWVA | IIWYD--- GSYKYYADS VKG | RFTVSRDNSKNTLYLQMNSL RAEDTAVYYCAR | EGYDFW---------- --------SGFFDS | WGQGT LVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35577 | 21-225_160B1 | VH3\|3-33/D3\|3-22\|RF2/JH4 | | QVQLVES-GGGVVQPGRSLRLSCAASG-FTFS | S-----YGMH | WVRQAPGKGLEWVA | VIWYD---GSYKYYADSVKG | RFTISRDNSKNTLYLQMNSLRAEDTAVYYCAR | EAYDFW-----------------SGYYDY | WGQGTLVTVSS |
| iPS:4 35723 | 21-225_172B7 | VH3\|3-33/D3\|3-22\|RF2/JH4 | | QVQLVES-GGGVVQPGRSLRLSCAASG-FTFS | N-----YGMH | WVRQAPGKGLEWVA | IIWYD---GSNKYYVDSVKG | RFTISRDNSKNTLYLQMNSLRAEDTAVYYCAR | EAYDFW-----------------SGYWDY | WGQGTLVTVSS |
| iPS:4 35731 | 21-225_173A11 | VH3\|3-33/D3\|3-22\|RF2/JH4 | | QVQLVES-GGGVVQPGRSLRLSCAASG-FTFS | S-----YGMH | WVRQAPGKGLEWVA | IIWYD---GSNKYYADSVKG | RFTISRDNSKNTLYLQMNSLRAEDTAVYYCAR | EAYDFW-----------------SGFFDS | WGQGTLVTVSS |
| iPS:4 35781 | 21-225_178G10 | VH3\|3-33/D3\|3-22\|RF2/JH4 | | QVQLVES-GGGVVQPGRSLRLSCAASG-FTFS | T-----YGMH | WVRQAPGKGLEWVA | VIWYD---GSNKYYADSVKG | RFTISRDNFKNTLYLQMNSLRAEDTAVYYCAR | ERYDFW-----------------SGHFDY | WGQGTLVTVSS |
| iPS:4 35899 | 21-225_188G11 | VH3\|3-33/D3\|3-22\|RF2/JH4 | | QVQLVES-GGGVVQPGRSLRLSCAASG-FTFS | S-----YGMH | WVRQAPGKGLEWVT | IIWYD---GSYKYYADSVKG | RFTISRDNSKNTLFLQMNSLRAEDTAVYYCAR | ERYDFW-----------------SGHFDY | WGQGTLVTVSS |
| iPS:4 36602 | 21-225_226E7 | VH3\|3-33/D3\|3-22\|RF2/JH4 | | QVQLVES-GGGVVQPGRSLRLSCAASG-FTFS | T-----YGMH | WVRQAPGKGLEWVA | IIWYD---GSNKYYADSVKG | RFTISRDNFKNTLYLQMHSLRADDTAVYYCAR | ENYDFW-----------------SGYYGY | WGQGTLVTVSS |
| iPS:3 92930 | 21-225_25H9 | VH3\|3-33/D3\|3-22\|RF2/JH4 | | QVQLVES-GGGVVQPGRSLRLSCAASG-FPFN | N-----YGMH | WVRQAPGKGLEWVS | IIWYD---GSYKYYADSVKG | RFTISRDNSKNTLYLQMNSLRAEDTAVYYCAR | EGYDFW-----------------SGFFDS | WGQGTLVTVSS |
| | | Germline | | H_FR1 | H_CDR1 | H_FR2 | H_CDR2 | H_FR3 | H_CDR3 | H_FR4 |
| | VH3\|3-23/D1\|1-26\|RF2/JH4 | | 32340 & 32341 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | V*WEL-----------------LYFDY | WGQGTLVTVSS |
| iPS:4 35479 | 21-225_154E9 | VH3\|3-23/D1\|1-26\|RF2/JH4 | | EVKLLES-GGGLVQPGGSLRLSCAASG-FTFS | S-----YAMS | WVRQAPGKGLEWVS | AISGR---GGNTFYADSVKG | RFTISRDNSKNTLFLQMNSLRAEDTAVYYCAK | RGFRFLE---------------WLGGFDY | WGQGTLVTVSS |
| | | Germline | | H_FR1 | H_CDR1 | H_FR2 | H_CDR2 | H_FR3 | H_CDR3 | H_FR4 |
| | VH3\|3-23/D5\|5-18\|RF3/JH4 | | 32342 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSYG-----------------YYFDY | WGQGTLVTVSS |
| iPS:4 35497 | 21-225_155H9 | VH3\|3-23/D5\|5-18\|RF3/JH4 | | EVQLLES-GGGLVQPGGSLRLSCAASG-FTFS | S-----YAMN | WVRQAPGKGLEWVS | TISGR---GLGTYYADSVKG | RFTISRDNSKNTLYLQLNSLRAEDTAVYYCAK | DHDYGDY---------------NIYFDY | WGQGTLVTVSS |
| | | Germline | | H_FR1 | H_CDR1 | H_FR2 | H_CDR2 | H_FR3 | H_CDR3 | H_FR4 |
| | VH3\|3-23/D6\|6-19\|RF1/JH3 | | 32343 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSSGW----------------YDAFDI | WGQGTMVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35513 | 21-225_157F3 | VH3\|3-23/D6\|6-19\|RF1/JH3 | | EVQLLES-GGGLVQPGGSLRLSCAASE-FTFS | T-----YAMS | WVRQAPGKGLEWVS | VISGS---GGSTYYADSVKG | RFTISRDNSKNTLYLQMNSLRAEDTAVYYCAK | RSSGWY-----------------EDALDI | WGQGTMVTVSS |
| iPS:3 92766 | 21-225_23H4 | VH3\|3-23/D6\|6-19\|RF1/JH3 | | EVQLLES-GGGLVQPGGSLRLSCAASG-FTFS | S-----YAMN | WVRQAPGKGLEWVS | VISGS---GGTTYFADSVKG | RFTISRDNSKNTLYLQMNSLRAEDTAVYYCAK | RNSSGW-----------------HDVFDI | WGQGTKVTVSS |
| iPS:3 92808 | 21-225_20F8 | VH3\|3-23/D6\|6-19\|RF1/JH3 | | EVQLLES-GGGLVQPGGSLRLSCAASG-FTFR | S-----YAMS | WIRQAPGRGLEWSS | VISGS---GGSTYYADSVKG | RFTISRDNSKNTLYLQMSSLRAEDTAVYYCAK | RYNSGW-----------------HDVFDI | WGQGTMVTVSS |
| | | Germline | | H_FR1 | H_CDR1 | H_FR2 | H_CDR2 | H_FR3 | H_CDR3 | H_FR4 |
| | VH4\|4-39/D2\|2-21\|RF3/JH4 | | 32344 | QLQLQES-GPGLVKPSETLSLTCTVSG-GSIS | SS---SYYWC | WIRQPPGKGLEWIG | SIYY-SGSTYYNPSLKS | RVTISVDTSKNQFSLKLSSVTAADTAVYYCAR | HIVVVI-----------AIYFDY | WCQGTLVTVSS |
| iPS:4 35525 | 21-225_157E7 | VH4\|4-39/D2\|2-21\|RF3/JH4 | | QLHLQES-GPGLVKPSETLSLTCTVSG-GSIS | SG---SYYWG | WIRQPPGKGLEWIG | SIYY----SGSTYYNPSLKS | RVTISVDTSKNQFSLNLSSVTAADTAVYYCAR | HKVAG---------------PFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D3\|3-10\|RF2/JH6 | | 32345 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYYGSGSYYN-----------YYYYYGMDV | WGQGTTVTVSS |
| iPS:4 35543 | 21-225_158D4 | VH3\|3-33/D3\|3-10\|RF2/JH6 | | QVQLVES-GGGVVQPGRSLRLSCAASG-FTFS | D-----YVMH | WVRQAPGKGLEWVS | VIWYD---GSNKYYADSVKG | RFTISRDNSKNTLYLQMNSLRAEDTAVYYCAR | EPYTSGW-----------YDYGMDV | WGQGTTVTVSS |
| iPS:4 35571 | 21-225_159C8 | VH3\|3-33/D3\|3-10\|RF2/JH6 | | QVQLVES-GGGVVQPGRSLRLSCAASG-FTFS | D-----YVMQ | WVRQAPGKGLEWVA | VIWYD---GSNKYYADSVKG | RFTISRDNSKNTLYLQMNSLRAEDTAVYYCAR | EPYNSGW-----------YDYGMDV | WGQGTTVTVSS |
| iPS:4 35591 | 21-225_160C4 | VH3\|3-33/D3\|3-10\|RF2/JH6 | | QVQLVES-GGGVVQPGRSLRLSCAASG-FTFS | D-----YVMQ | WVRQAPGKGLEWVA | VIWYD---GSNKYYADSVKG | RFTISRDNSKNTLYLQMNSLRAEDTAVYYCAR | EPYNSGW-----------YDYGMDV | WGQGTTVTVSS |
| iPS:4 35615 | 21-225_161G12 | VH3\|3-33/D3\|3-10\|RF2/JH6 | | QVQLVES-GGGVVQPGRSLRLSCAASG-FTFS | D-----YVMQ | WVRQAPGKGLEWVA | VIWYD---GSNKYYADSVKG | RFTISRDNSKNTLYLQMNSLRAEDTAVYYCAR | EPYNSGW-----------YDYGMDV | WGQGTTVTVSS |
| iPS:4 36604 | 21-225_226F7 | VH3\|3-33/D3\|3-10\|RF2/JH6 | | QVQLVES-GGGVVQPGRSLRLSCAASG-FTFS | S-----YGMH | WVRQAPGKGLEWVA | IIWYD---GSNKYYADSVKG | RFTISRDNSKNTLYLQMNSLRAEDTAVYYCAR | ERYNSGW-----------YDYGLDV | WGQGTTVTVSS |
| iPS:4 51114 | 21-225_159A3 | VH3\|3-33/D3\|3-10\|RF2/JH6 | | QVQLVES-GGGVVQPGRSLRLSCAASG-FTFS | D-----YVMQ | WVRQAPGKGLEWVA | VIWYD---GSNKYYADSVKG | RFTISRDISKNTLYLQMNSLRAEDTAVYYCAR | EPYNSGW-----------YDYGMDV | WGQGTTVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 72732 | 21-225_2B10_LC1 | VH3\|3-33/D3\|3-10\|RF2/JH6 | | QVQLVES-GGGVVQPGRSLRLSCAASG-FTFS | S-----YVMH | WVRQAPGKGLEWVA | VIWYD---GSNKYYADSVKV | RFTVSRDNSKNTLSLQMNSLRAEDTAVYYCAR | ERYTSGW----------------YDYGMDV | WGQGTTVTVSS |
| iPS:4 72733 | 21-225_2B10_LC2 | VH3\|3-33/D3\|3-10\|RF2/JH6 | | QVQLVES-GGGVVQPGRSLRLSCAASG-FTFS | S-----YVMH | WVRQAPGKGLEWVA | VIWYD---GSNKYYADSVKV | RFTVSRDNSKNTLSLQMNSLRAEDTAVYYCAR | ERYTSGW----------------YDYGMDV | WGQGTTVTVSS |
| iPS:3 92872 | 21-225_20B11 | VH3\|3-33/D3\|3-10\|RF2/JH6 | | QVQLVES-GGGVVQPGRSLRLSCAASG-FTFS | S-----YVMH | WVRQAPGKGLEWVA | VIWYD---GSNKYYADSVKV | RFTVSRDNSKNTLSLQMNSLRAEDTAVYYCAR | ERYTSGW----------------YDYGMDV | WGQGTTVTVSS |
| iPS:3 93966 | 21-225_7F8 | VH3\|3-33/D3\|3-10\|RF2/JH6 | | QVQLVES-GGGVVQPGRSLRLSCAASG-FTFS | S-----CVMH | WVRQAPGKGLEWVA | VIWYD---GSNKYYADSVKG | RFTISRDNSXNTLYLQMNSLRAEDTAVYYCAR | ERYTSGW----------------HDYGMDV | WGQGTTVTVSS |
| | Germline | | | H_FR1 | H_CDR1 | H_FR2 | H_CDR2 | H_FR3 | H_CDR3 | H_FR4 |
| | VH3\|3-23/D1\|1-1\|RF1/JH6 | | 32346 | EVQLLES-GGGLVQPGGSLRLSCAASG-FTFS | S-----YAMS | WVRQAPGKGLEWVS | AISGS---GGSTYYADSVKG | RFTISRDNSKNILYLQMNSLRAEDTAVYYCAK | GTGTYY----------------YYYGMDV | WGQGTTVTVSS |
| iPS:4 35559 | 21-225_158H12 | VH3\|3-23/D1\|1-1\|RF1/JH6 | | EVQLLES-GGGLVQPGGSLRLSCAASG-FTFS | S-----YVMS | WVRQAPGKGLEWVS | AISGS---GGRTDYADSVKG | RFTISRDNSKNTLYLQMNSLRAEDTAVYYCAK | GGW----------------------NHD | WGQGTTVTVSS |
| | Germline | | | H_FR1 | H_CDR1 | H_FR2 | H_CDR2 | H_FR3 | H_CDR3 | H_FR4 |
| | VH3\|3-21/D1\|1-1\|RF1/JH6 | | 32347 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GTGTYY----------------YYYGMDV | WGQGTTVTVSS |
| iPS:4 35561 | 21-225_159F1 | VH3\|3-21/D1\|1-1\|RF1/JH6 | | EVQLVES-GGGLVKPGGSLRLSCAASG-FTFS | S-----YRMN | WVRQAPGKGLEWVS | SISGS---GNYIDYADSVKG | RFTISRDNAKNSLYLQMNSLRAEDTAVYYCAR | GW------------------------DV | WGQGTTVTVSS |
| | Germline | | | H_FR1 | H_CDR1 | H_FR2 | H_CDR2 | H_FR3 | H_CDR3 | H_FR4 |
| | VH1\|1-08/D1\|1-1\|RF1/JH4 | | 32348 | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GTGT------------------YFDY | WGQGTLVTVSS |
| iPS:4 35563 | 21-225_159H2 | VH1\|1-08/D1\|1-1\|RF1/JH4 | | QVQLVQS-GAEVKKPGASVKVSCKASG-YTFT | S-----YDIN | WVRQATGQGLEWMG | WMNPN---SGNTGYVQKFQG | RVTMTRNTSISTAYMELSSLRSEDTAVYYCAR | KKT-------------------GDY | WGQGTLVTVSS |
| iPS:3 92718 | 21-225_17B8 | VH1\|1-08/D1\|1-1\|RF1/JH4 | | QVQLVQS-GAEVKKPGASVKVSCKASG-YTFT | S-----YAIN | WVRQATGQGLEWMG | WMNPN---TGNTGYAQKFQG | RVTMTRNTSISTAYMELSSLRSEDTAVYFCTR | KAG-------------------FDY | WGQGTLVTVSS |
| iPS:3 93064 | 21-225_33A9 | VH1\|1-08/D1\|1-1\|RF1/JH4 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLGSEDTAVYYCAR | KKA-------------------NDY | WGQGTLVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:3 93148 | 21-225_35E5 | VH1\|1-08/D1\|1-1\|RF1/JH4 | | QVQLVQS-GAEVKKPGASVKVSCKASG-YTFT | S-----YDIN | WVRQATGQGLEWMG | WMNPN---SGNTGYAQKFQG | RVTMTRNISISTAYMELSSLRSEDTAVYYCAR | KKS---------------------NDY | WGQGTLVTVSS |
| iPS:3 98530 | 21-225_32G4 | VH1\|1-08/D1\|1-1\|RF1/JH4 | | QVQLVQS-GAEVKKPGASVKVSCKASG-YTFT | S-----YDIN | WVRQATGQGLEWMG | WMNPN---SGNTGYAQKFQG | RVTMTRNISISTAYMELSSLRSEDTAVYYCAR | KKA---------------------NDY | WGQGTLVTVSS |
| **Germline** | | | | **H_FR1** | **H_CDR1** | **H_FR2** | **H_CDR2** | **H_FR3** | **H_CDR3** | **H_FR4** |
| | VH3\|3-30.3/D1\|1-26\|RF3/JH6 | | 32349 | QVQLVES-GGGVVQPGRSLRLSCAASG-FTFS | S------YAMH | WVRQAPGKGLEWVA | VISYD---GSNKYYADSVKG | RFTISRDNSKNTLYLQMNSLRAEDTAVYYCAR | YSGSYYYY-----------YYYGMDV | WGQGTTVTVSS |
| iPS:4 35565 | 21-225_159C4 | VH3\|3-30.3/D1\|1-26\|RF3/JH6 | | QVQLVES-GGGVVQPGRSLRLSCAASG-FTFS | N-----YGMH | WVRQAPGKGLEWVA | VISYS---GNNKYYADSVKG | RFTISRDNSKNTLYLQLNSLRAEDMAVYYCAR | RSSSWG-----------GYGMDV | WGHGTTVTVSS |
| iPS:3 93692 | 21-225_6G7 | VH3\|3-30.3/D1\|1-26\|RF3/JH6 | | QVQLVES-GGGVVQPGRSLRLSCAASG-FTFS | S-----YGMH | WVRQTPGKGLEWVA | IISYV---GKNKYYADSVKG | RFTISRDNSKNTLYLQMNSLRAEDTAVYYCAR | RGNSYG-----------GYGMDV | WGQGTTVTVSS |
| **Germline** | | | | **H_FR1** | **H_CDR1** | **H_FR2** | **H_CDR2** | **H_FR3** | **H_CDR3** | **H_FR4** |
| | VH3\|3-23/D2\|2-21\|RF3/JH4 | | 32350 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | HIVVVI-----------AIYFDY | WGQGTLVTVSS |
| iPS:4 35579 | 21-225_160G1 | VH3\|3-23/D2\|2-21\|RF3/JH4 | | EVQLLES-GGGLVQPGGSLRLSCAASG-FTFS | S-----YVMS | WVRQAPGKGLEWVS | AMSGS---GGHTYYADSVKG | RFTISRDNSKNIVYLQMNSLRAEDTAVYYCVK | HG--------------------YS | WGQGTLVTVSS |
| iPS:4 35585 | 21-225_160G3 | VH3\|3-23/D2\|2-21\|RF3/JH4 | | EVQLLES-GGGLVQPGGSLRLSCAASG-FTFS | S-----YVMS | WVRQTPGKGLEWVS | AMSGS---GGHTYYADSVKG | RFTISRDNSKNTLYLQMNSLRAEDTAIYYCVK | HG--------------------YS | WGQGTLVTVSS |
| **Germline** | | | | **H_FR1** | **H_CDR1** | **H_FR2** | **H_CDR2** | **H_FR3** | **H_CDR3** | **H_FR4** |
| | VH4\|4-39/D1\|1-1\|RF3/JH5 | | 32351 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | YNWND------------NWFDP | WGQGTLVTVSS |
| iPS:4 35599 | 21-225_160B10 | VH4\|4-39/D1\|1-1\|RF3/JH5 | | QLQLQES-GPGLVKPSETLSLTCTVSG-GSIS | RS---SYYWG | WIRQYPGKGLEWIG | NIYY----SGSAYHIPSLKS | RVTISVDTSKNQFSLKLNSVTAADTAVYYCVR | HDPNW-----------GVDY | WGQGTLVTVSS |
| **Germline** | | | | **H_FR1** | **H_CDR1** | **H_FR2** | **H_CDR2** | **H_FR3** | **H_CDR3** | **H_FR4** |
| | VH3\|3-33/D1\|1-1\|RF2/JH6 | | 32352 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VQLERYY-----------YYYGMDV | WGQGTTVTVSS |
| iPS:4 35601 | 21-225_160G10 | VH3\|3-33/D1\|1-1\|RF2/JH6 | | QVQLVES-GGGVVQPGRSLRLSCAASG-FTFS | S-----FGMH | WVRQAPGKGLEWVA | VIWYD---GSYKYYADSVKG | RFTISRDNSKNTLYLQMNSLRAEDTAVYYCAR | VGIEVAGD----------YYFGMEV | WGQGTTVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35655 | 21-225_167E2 | VH3\|3-33/D1\|1-1\|RF2/JH6 | | QVQLVES-GGGVVQPGRSLRLSCAASG-FTFS | S-----FGMH | WVRQAPGK GLEWVA | VIWYD--- GTYKYYADS VKG | RFTISRDNSKNTLYLQMNSL RAEDTAVYYCAR | VGIEVAGD---------------YYYGMEV | WGQGT TVTVS S |
| iPS:4 35657 | 21-225_167H10 | VH3\|3-33/D1\|1-1\|RF2/JH6 | | QVQLVES-GGGVVQPGRSQRLSCAASG-FTFS | S-----FGMH | WVRQAPGK GLEWVA | VIWYD--- GSYKYHADS VKG | RFTISRDNSKNTLYLQMNSL RAEDTAVYYCAR | VGIEVAGD---------------YYYGMEV | WGQGT TVTVS S |
| | Germline | | | H_FR1 | H_CDR1 | H_FR2 | H_CDR2 | H_FR3 | H_CDR3 | H_FR4 |
| | VH3\|3-53/D7\|7-27\|RF3/JH4 | | 32353 | EVQLVET-GGGLIQPGGSLRLSCAASG-FTVS | S-----NYMS | WVRQAPGK GLEWVS | VIYS--- GGSTYYADS VKG | RFTISRDNSKNTLYLQMNSL RAEDTAVYYCAR | NWGY-----------FDY | WGQGT LVTVS S |
| iPS:4 35605 | 21-225_161A4 | VH3\|3-53/D7\|7-27\|RF3/JH4 | | EVQLVES-GGGLIQPGGSLRLSCAASG-FTVS | S-----NYMS | WVRQAPGK GLEWVS | VIYT---- GGSTYNADS VKG | RFTISRDNSKNTLYLQMNSL RAEDTAVYYCAR | NWGMA-----------GPFDY | WGQGT LVTVS S |
| | Germline | | | H_FR1 | H_CDR1 | H_FR2 | H_CDR2 | H_FR3 | H_CDR3 | H_FR4 |
| | VH3\|3-30.3/D6\|6-6\|RF1/JH6 | | 32354 | QVQLVES-GGGVVQPGRSLRLSCAASG-FTFS | S-----YAMH | WVRQAPGK GLEWVA | VISYD--- GSNKYYADS VKG | RFTISRDNSKNTLYLQMNSL RAEDTAVYYCAR | EYSSSSYY---------YYYGMDV | WGQGT TVTVS S |
| iPS:4 35607 | 21-225_161G4 | VH3\|3-30.3/D6\|6-6\|RF1/JH6 | | QVQLVES-GGGVVQPGRSLRLSCAASG-FTFS | S-----YGMH | WVRQAPGK GLEWVA | VISYG--- GSNKYHADS VKG | RFTISRDNSKNTLYLQMNSL RAEDTAVYYCVR | RSSSSG-----------GYGMDV | WGQGT TVTVS S |
| iPS:3 93020 | 21-225_30E2 | VH3\|3-30.3/D6\|6-6\|RF1/JH6 | | QVQLVES-GGGVVQPGRSLRLSCAASG-FTFS | S-----YGMH | WVRQAPGK GLEWVA | VISYG--- GSNKFYAVS VKG | RFNISRDNSKNTLYLQMNSL RAEDTAVYYCVR | RGYSSG-----------GYGMDV | WGQGT TVTVS S |
| iPS:3 93062 | 21-225_33H3 | VH3\|3-30.3/D6\|6-6\|RF1/JH6 | | QVQLVES-GGGVVQPGRSLRLSCAASG-FTFS | S-----YGMH | WVRRAPGK GLEWVA | IISYG--- GSNNFYADS VKG | RFTISRDNSKNTLYLQMNSL RAEDTAVYYCAR | RGYSSG-----------GYGMDV | WGQGT TVTVS S |
| iPS:3 93138 | 21-225_35E3 | VH3\|3-30.3/D6\|6-6\|RF1/JH6 | | QVQLVES-GGGVVQPGRSLRLSCAASG-FTFS | S-----YGMH | WVRQAPGK GLEWVA | VISYG--- GSNKYYADS VKG | RFTISRDNSKNTLYLQMNSL RAEDTAVYYCVR | RGYSSG-----------GYGMDV | WGQGT TVTVS S |
| | Germline | | | H_FR1 | H_CDR1 | H_FR2 | H_CDR2 | H_FR3 | H_CDR3 | H_FR4 |
| | VH3\|3-30.3/D6\|6-13\|RF2/JH6 | | 32355 | QVQLVES-GGGVVQPGRSLRLSCAASG-FTFS | S-----YAMH | WVRQAPGK GLEWVA | VISYD--- GSNKYYADS VKG | RFTISRDNSKNTLYLQMNSL RAEDTAVYYCAR | GIAAAGYY---------YYYGMDV | WGQGT TVTVS S |
| iPS:4 35611 | 21-225_161F10 | VH3\|3-30.3/D6\|6-13\|RF2/JH6 | | QVQLVES-GGGVVQPGRSLRLSCAASG-FTFS | S-----YGMH | WVRQAPGK GLEWVA | IISYS--- GRNDFYADS VKG | RFTISRDNSKNTLYLQMNSL RAEDTAVYYCAR | RIAAAG-----------HYGMDV | WGQGT TVTVS S |
| | Germline | | | H_FR1 | H_CDR1 | H_FR2 | H_CDR2 | H_FR3 | H_CDR3 | H_FR4 |
| | VH3\|3-33/D5\|5-18\|RF1/JH6 | | 32356 | QVQLVES-GGGVVQPGRSLRLSCAASG-FTFS | S-----YGMH | WVRQAPGK GLEWVA | VIWYD--- GSNKYYADS VKG | RFTISRDNSKNTLYLQMNSL RAEDTAVYYCAR | VDTAMVYY---------YYYGMDV | WGQGT TVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35629 | 21-225_162H6 | VH3\|3-33/D5\|5-18\|RF1/JH6 | | QVQLVES-GGGVVQPGRSLRLSCAASG-FTFN | N-----YGMH | WVRQAPGK GLEWVA | VIWYD---GSNKYYADSVKG | RFTISRDNSKNTLYLQMNSLRAEDTAVYYCAR | KGIAAVGD---------------YYYGMDV | WGQGTTVTVSS |
| | Germline | | | H_FR1 | H_CDR1 | H_FR2 | H_CDR2 | H_FR3 | H_CDR3 | H_FR4 |
| | VH3\|3-21/D7\|7-27\|RF1/JH6 | | 32357 | EVQLVES-GGGLVKPGGSLRLSCAASG-FTFS | S-----YSMN | WVRQAPGK GLEWVS | SISSS---SSYIYYADSVKG | RFTISRDNAKNSLYLQMNSLRAEDTAVYYCAR | LTGYYY---------------YYGMDV | WGQGTTVTVSS |
| iPS:4 35639 | 21-225_163G6 | VH3\|3-21/D7\|7-27\|RF1/JH6 | | EVQLVES-GGGLVKPGGSLRLSCAASG-FTFS | S-----YSMS | WVRQAPGK GLEWVS | SISGS---SAYIYYADSVKG | RFTISRDNAKNSLYLQMNSLRAEDTAVYYCAR | LSG------------------MDV | WGQGTTVTVSS |
| | Germline | | | H_FR1 | H_CDR1 | H_FR2 | H_CDR2 | H_FR3 | H_CDR3 | H_FR4 |
| | VH3\|3-21/D5\|5-24\|RF2/JH6 | | 32358 & 32359 | EVQLVES-GGGLVKPGGSLRLSCAASG-FTFS | S-----YSMN | WVRQAPGK GLEWVS | SISSS---SSYIYYADSVKG | RFTISRDNAKNSLYLQMNSLRAEDTAVYYCAR | *RWLQLYY---------------YYYGMDV | WGQGTTVTVSS |
| iPS:4 35643 | 21-225_163G10 | VH3\|3-21/D5\|5-24\|RF2/JH6 | | EVQLVES-GGGLVKPGGSLRLSCAASG-FTFS | S-----YSMN | WVRQAPGK GLEWVS | SISGS---STYIYYADSVKG | RFTISRDNAKNSLYLQMNSLRAEDTAVYYCAR | ARM------------------DV | WGQGTTVTVSS |
| | Germline | | | H_FR1 | H_CDR1 | H_FR2 | H_CDR2 | H_FR3 | H_CDR3 | H_FR4 |
| | VH3\|3-33/D3\|3-9\|RF2/JH4 | | 32360 | QVQLVES-GGGVVQPGRSLRLSCAASG-FTFS | S-----YGMH | WVRQAPGK GLEWVA | VIWYD---GSNKYYADSVKG | RFTISRDNSKNTLYLQMNSLRAEDTAVYYCAR | YYDILTG---------------YYNYFDY | WGQGTLVTVSS |
| iPS:4 35663 | 21-225_169B1 | VH3\|3-33/D3\|3-9\|RF2/JH4 | | QVQLVES-GGGVVQPGRSLRLSCAASG-FTFS | S-----YGMH | WVRQAPGK GLEWVA | VIWYD---GSNKYYADSVRG | RFTISRDNSKNTLYLQMNSLRAEDTAVYYCAR | DPLRGYN---------------DPVMDY | WGQGTLVTVSS |
| iPS:4 35669 | 21-225_169F9 | VH3\|3-33/D3\|3-9\|RF2/JH4 | | QVQLVES-GGGVVQPGRSLRLSCAASG-FTFS | T-----YGMH | WVRQAPGK GLEWVS | IIWYD---GTNKYYADSVKG | RFTISRDNSKNTLYLQLNSLRAEDTAVYYCAR | DPLRGYN---------------DPVMDY | WGQGTLVTVSS |
| iPS:4 35693 | 21-225_170G4 | VH3\|3-33/D3\|3-9\|RF2/JH4 | | QVQLVES-GGGVVQPGRSLRLSCTASG-FTFS | T-----YGMH | WVRQAPGK GLEWVS | IIWYD---GTNKYYADSVKG | RFTISRDNSKNTLFLQLNSLRAEDTAMYYCAR | DPLRGYN---------------DPVMDY | WGQGTLVTVSS |
| iPS:4 35695 | 21-225_170D5 | VH3\|3-33/D3\|3-9\|RF2/JH4 | | QVQLVES-GGGVVQPGRSLRLSCAASG-FTFS | S-----YGMH | WVRQAPGK GLEWVA | IIWYD---GTNKYYADSVKG | RFTISRDNSKNTLYLQLNSLRAEDTAVYYCAR | DPLRGYN---------------DPVMDY | WGQGTLVTVSS |
| iPS:4 35697 | 21-225_170G5 | VH3\|3-33/D3\|3-9\|RF2/JH4 | | QVQLVES-GGGVVQPGGSLRLSCAASG-FTFS | T-----YGMH | WVRQAPGK GLEWVT | IIWYD---GTNKYYADSVKG | RFTISRDNSKSTLYLQMNSLRAEDTAVYFCAR | DPLRGYN---------------DPVMDY | WGQGTLVTVSS |
| iPS:4 35703 | 21-225_170D11 | VH3\|3-33/D3\|3-9\|RF2/JH4 | | QVQLVES-GGGVVQPGRSLRLSCVASG-FTFS | S-----YGMH | WVRQAPGK GLEWVA | IIWYD---GTNKYYADSVKG | RFTISRDNSKNTLYLQLNSLRAEDTAVYYCAR | DPLRGYN---------------DPVMDY | WGQGTLVTVSS |

| ID | Clone | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 35705 | 21-225_171C3 | VH3\|3-33/D3\|3-9\|RF2/JH4 | | QVQLVES-GGGVVQPGGSLRLSCAASG-FTFS | T-----YGMH | WVRQAPGK GLEWVT | IIWYD--- GTNKYYADS VKG | RFTISRDNSKNTLYLQMNSL RAEDTAVYYCAR | DPLRGYN--------- --------DPVMDY | WGQGT LVTVS S |
| iPS:4 35709 | 21-225_171A4 | VH3\|3-33/D3\|3-9\|RF2/JH4 | | QVQLVES-GGGVVQPGGSLRLSCAASG-FTFS | T-----YGMH | WVRQAPGK GLEWVA | IIWYD--- GSNKYYADS VKG | RFTISRDNSKNTLYLQMNSL RAEDTAVYYCAR | DPLRGYN--------- --------DPVMDY | WGQGT LVTVS S |
| iPS:4 35721 | 21-225_172B3 | VH3\|3-33/D3\|3-9\|RF2/JH4 | | QVQLVES-GGGVVQPGRPLRLSCAASG-FTFS | T-----YGMH | WVRQAPGK GLEWVA | VIWYD--- GSNKYYADS VKG | RFTISRDNSKNTLYLQMNSL RAEDTAVYYCAR | DPLRGYN--------- --------DPVMDY | WGQGT LVTVS S |
| iPS:4 35725 | 21-225_172G8 | VH3\|3-33/D3\|3-9\|RF2/JH4 | | QVQMVES-GGGVVQPGRSLRLSCAASG-FTFS | T-----YGMH | WVRQAPGK GLEWMA | IIWYD--- GTNKYYADS VKG | RFTISRDNSKNTLYLQLNSL RAEDTAVYYCAR | DPLRGYN--------- --------DPVMDY | WGQGT LVTVS S |
| iPS:4 35735 | 21-225_173H12 | VH3\|3-33/D3\|3-9\|RF2/JH4 | | QVQLVES-GGGVVQPGRSLRLSCAASG-FTFS | S-----YGMH | WVRQAPGK GLEWVA | IIWYD--- GTNKYYADS VKG | RFTISRDNSKNTLYLQLNSL RAEDTAVYYCAR | DPLRGYN--------- --------DPVMDY | WGQGT LVTVS S |
| iPS:4 35743 | 21-225_175G1 | VH3\|3-33/D3\|3-9\|RF2/JH4 | | QVQLVES-GGGVVQPGGSLRLSCAASG-FTFS | T-----YGMH | WVRQAPGK GLEWVA | VIWYD--- GSNKYYADS VKG | RFTISRDNSKNTLYVQMNSL RAEDTAVYYCAR | DPLRGYN--------- --------DPVMDY | WGQGT LVTVS S |
| iPS:4 35761 | 21-225_176B11 | VH3\|3-33/D3\|3-9\|RF2/JH4 | | QVQLVES-GGGVVQPGRSLRLSCAASG-FTFS | T-----YGMH | WVRQAPGK GLEWVS | IIWYD--- GTNKYYADS VKG | RFTISRDNSKNTLFLQLNSL RAEDTAVYYCAR | DPLRGYN--------- --------DPVLDY | WGQGT LVTVS S |
| iPS:4 35779 | 21-225_178B10 | VH3\|3-33/D3\|3-9\|RF2/JH4 | | QVQLVES-GGGVVQPGRSLRLSCVASG-FTSS | T-----YGMH | WVRQAPGK GLEWMA | IIWYD--- GTNKYYADS VKG | RFTISRDNSKNTLYLQLNSL RAEDTAVYYCAR | DPLRGYN--------- --------DPVMDY | WGQGT LVTVS S |
| | | Germline | | H_FR1 | H_CDR1 | H_FR2 | H_CDR2 | H_FR3 | H_CDR3 | H_FR4 |
| | VH3\|3-48/D2\|2-15\|RF3/JH6 | | 32361 | EVQLVES-GGGLVQPGGSLRLSCAASG-FTFS | S-----YSMN | WVRQAPGK GLEWVS | YISSS--- SSTIYYADS VKG | RFTISRDNAKNSLYLQMNSL RDEDTAVYYCAR | DIVVVVAAT------- -----YYYYYGMDV | WGQGT TVTVS S |
| iPS:4 35667 | 21-225_169E3 | VH3\|3-48/D2\|2-15\|RF3/JH6 | | EVQLVES-GGGLVQPGGSLRLSCAASG-FTFS | G-----HSMN | WVRQAPGK GLEWVA | YISLS--- GSTIKYADS VKG | RFTISRDNARDSLYLQMNSL RDEDTAVYYCAR | RGITVVR--------- -------NEDGLDV | WGQGT TVTVS S |
| iPS:4 35673 | 21-225_169E6 | VH3\|3-48/D2\|2-15\|RF3/JH6 | | EVQLVES-GGGLVQPGGSLRLSCAASG-FTFS | G-----HSMN | WVRQAPGK GLEWVA | YISIS--- SSTIKYADS VKG | RFTISRDNARDSLYLQMNSL RDEDTAVYYCAR | RGITVVR--------- -------NEDGLDV | WGQGT TVTVS S |
| iPS:4 35759 | 21-225_176E6 | VH3\|3-48/D2\|2-15\|RF3/JH6 | | EVQLVES-GGGLVQPGGSLRLSCAASG-FTFS | G-----HSMN | WVRQAPGK GLEWVA | YISIS--- GSTIKYADS VKG | RFIISRDNARDSLYLQMNSL RDEDTAVYYCAR | RGITVVR--------- -------NEDGLDV | WGQGT TVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-59/D3\|3-9\|RF1/JH6 | 32362 & 32363 | QVQLQES-GPGLVKPSEILSL TCTVSG-GSIS | S-----YYWS | WIRQPPGK GLEWIG | YIYY---- SGSTNYNPS LKS | RVTISVDTSKNQFSLKLSSV TAADTAVYYCAR | VLRYFDWLL*------ -----YYYYYGMDV | WGQGT TVTVS S |
| iPS:4 35675 | 21-225_169D7 | VH4\|4-59/D3\|3-9\|RF1/JH6 | QVQLQES-GPGLVKPSEILSL TCTVSG-GSIS | S-----YYWI | WIRQPAGK GLEWIG | RIYT---- SGSTNYNPS LKS | RVTMSVDTSKNQFSLKLSSV TAADTAVYFCAK | VGRYY----------- ---------YGMDV | WGQGT TVTVS S |
| iPS:4 35687 | 21-225_170H1 | VH4\|4-59/D3\|3-9\|RF1/JH6 | QVQLQES-GPGLVKPSEILSL TCTVSG-GSIS | S-----YYWS | WIRQPAGK GLEWIG | RIYT---- SGSTNYNPS LKS | RVTMSVDTSKNQFSLKLSSV TAADTAVYYCAR | VGRYY----------- ---------YGMDV | WGQGT TVTVS S |
| | | Germline | H_FR1 | H_CDR1 | H_FR2 | H_CDR2 | H_FR3 | H_CDR3 | H_FR4 |
| | VH3\|3-23/D4\|4-23\|RF2/JH4 | 32364 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | DYGCN----- ---------SYFDY | WGQGT LVTVS S |
| iPS:4 35711 | 21-225_171G4 | VH3\|3-23/D4\|4-23\|RF2/JH4 | EVQLLES-GGDLVQPGGSLRL SCAASG-FTFS | S-----CAMT | WVRQAPGK GLEWVS | AISGR--- GGTTFYADS VRG | RFTISRDNSKNTLFLQMNSL RAEDTAVYYCAK | DLIGGA---------- ---------TYFDY | WGQGT LVTVS S |
| iPS:4 35875 | 21-225_190B9 | VH3\|3-23/D4\|4-23\|RF2/JH4 | EVQLLES-GGGLVQPGGSLRL SCAASG-FTFS | T-----YAMS | WVRQAPGK GLEWVS | AISRS--- GGNTHYADS VKG | RFTISRDNSKNTLYLQMNSL RAEDTAVYSCAK | DGFGGS---------- ---------SYFDY | WGQGT LVTVS S |
| iPS:4 35909 | 21-225_190H3 | VH3\|3-23/D4\|4-23\|RF2/JH4 | EVQLLES-GGGLVQPGGSLRL SCAASG-FTFS | S-----YAMS | WVRQAPGK GLEWVS | AISGR--- GGNTYYADS VKG | RFTISRDNSKNTLYLQMNSL RAEDTAVYYCAK | DGFGGS---------- ---------SYFDY | WGQGT LVTVS S |
| iPS:4 36013 | 21-225_193F2 | VH3\|3-23/D4\|4-23\|RF2/JH4 | EVQLLES-GGGLVQPGGSLRL SCAASG-FTFS | T-----FAMS | WVRQAPGK GLEWVS | AISRS--- GGNTHYADS VKG | RFTISRDNSKNTLYLQMNSL RAEDTAVYSCAK | DGFGGS---------- ---------SYFDY | WGQGT LVTVS S |
| iPS:4 36100 | 21-225_195G12 | VH3\|3-23/D4\|4-23\|RF2/JH4 | EVQLLES-GGGLVQPGGSLRL SCAASG-FTFS | T-----YAMS | WVRQAPGK GLEWVS | AISRS--- GGNTHYADS VKG | RFTISRDNSKNTLYLQMNSL RAEDTAVYSCAK | DGFGGS---------- ---------SYFDY | WGQGT LVTVS S |
| | | Germline | H_FR1 | H_CDR1 | H_FR2 | H_CDR2 | H_FR3 | H_CDR3 | H_FR4 |
| | VH3\|3-30.3/D1\|1-7\|RF2/JH6 | 32365 & 32366 | QVQLVES-GGGVVQPGRSLRL SCAASG-FTFS | S-----YAMH | WVRQAPGK GLEWVA | VISYD--- GSNKYYADS VKG | RFTISRDNSKNTLYLQMNSL RAEDTAVYYCAR | V*LELYY------- ------YYYGMDV | WGQGT TVTVS S |
| iPS:4 35713 | 21-225_171D7 | VH3\|3-30.3/D1\|1-7\|RF2/JH6 | QVQLVES-RGGVVQPGRSLRL SCAASG-FTFS | S-----YGMH | WVRQAPGK GLEWVA | VISYD--- GNNRHYADS VQG | RFTISRDNSKNTLSLQMNSL GAEDTAVYYCAR | DRHRLD---------- --------YYALDV | WGQGT TVTVS S |
| | | Germline | H_FR1 | H_CDR1 | H_FR2 | H_CDR2 | H_FR3 | H_CDR3 | H_FR4 |
| | VH3\|3-23/D5\|5-24\|RF3/JH3 | 32367 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | RDGYNY---------- --------DAFDI | WGQGT MVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35729 | 21-225_173E7 | VH3\|3-23/D5\|5-24\|RF3/JH3 | | EVQLLES-GGGSVQPGGSLRLSCAASG-FTFS | S-----YAMS | WVRQAPGK GLEWVS | FISGS--- GGNTFYADS VKG | RFTISRDNSKNTLYLQMNSL QAEDTAVYYCTK | RDTYNG------------------WDAFDI | WGQGT MVTVS S |
| iPS:4 35753 | 21-225_175G10 | VH3\|3-23/D5\|5-24\|RF3/JH3 | | EVQLLES-GGGLVQPGGSLRLSCAASG-FTFS | S-----YAMS | WVRQTPGK GLEWVS | IISGS--- GGNTYYADS VKG | RFTISRDNSKNTLYLQMNSL RAEDTAVYYCAK | RDTWNG------------------WDAFDI | WGQGT MVTVS L |
| iPS:3 93024 | 21-225_31H9 | VH3\|3-23/D5\|5-24\|RF3/JH3 | | EVQLLES-GGGLVQPGGSLRLSCAASG-FTFS | S-----CAMN | WVRQAPGK GLEWVS | AISGS--- GGSSFYADS VKG | RFTISRDNSKNTLYLQMNSL RAEDTAVYYCAK | RIPYD------------------VFDI | WGQGT MVTVS S |
| iPS:3 98474 | 21-225_17B10 | VH3\|3-23/D5\|5-24\|RF3/JH3 | | EVQLLES-GGGLVQPGGSLRLSCAASG-FTFR | S-----YAMS | WVRQAPGK GLEWVS | VISGS--- GGNTYYADS VKG | RFTISRDNSKNTLYLQMDSL RAEDTAVYYCAK | RCIPEA------------------DAFDI | WGQGT MVTVS S |
| | | Germline | | H_FR1 | H_CDR1 | H_FR2 | H_CDR2 | H_FR3 | H_CDR3 | H_FR4 |
| VH1\|1-02/D1\|1-1\|RF1/JH3 | | | 32368 | QVQLVQS-GAEVKKPGASVKVSCKASG-YTFT | G-----YYMH | WVRQAPGQ GLEWMG | WINPN--- SGGTNYAQK FQG | RVTMTRDTSISTAYMELSRL RSDDTAVYYCAR | GTTGT------------DAFDI | WGQGT MVTVS S |
| iPS:4 35745 | 21-225_175G3 | VH1\|1-02/D1\|1-1\|RF1/JH3 | | QVQVVQS-GAEVKKPGASVKVSCKASG-YTFT | G-----YFMH | WVRQAPGQ GLEWMG | WIKPK--- SGGTNCAQR FQG | RVTMTRDTSITTAYMELSRL RSDDTAVYYCVR | GGTTVIT------------WGVFDY | WGQGT MVTVS S |
| iPS:4 37270 | 21-225_178H4 | VH1\|1-02/D1\|1-1\|RF1/JH3 | | QVQLVQS-GAEVKKPGASVKVSCKASG-YTFT | G-----YFMH | WVRQAPGQ GLEWMG | WIKPK--- SGGTNCAQR FQG | RVTMTRDTSISTAYMELSRL RSDDTAVYYCVR | GGTTVIT------------WGVFDY | WGQGT MVTVS S |
| | | Germline | | H_FR1 | H_CDR1 | H_FR2 | H_CDR2 | H_FR3 | H_CDR3 | H_FR4 |
| VH3\|3-23/D3\|3-22\|RF2/JH4 | | | 32369 | EVQLLES-GGGLVQPGGSLRLSCAASG-FTFS | S-----YAMS | WVRQAPGK GLEWVS | AISGS--- GGSTYYADS VKG | RFTISRDNSKNTLYLQMNSL RAEDTAVYYCAK | YYYDSSG------------YYYYFDY | WGQGT LVTVS S |
| iPS:4 35769 | 21-225_177B6 | VH3\|3-23/D3\|3-22\|RF2/JH4 | | EVQLLES-GGGLVQPGGSRRLSCEASG-FTFS | S-----YAMS | WVRQAPGK GLEWVS | VISGS--- GSNTYYVDS VKG | RFTISRDNSKNTLNLQMNSL RAEDSAVYYCTK | GYYDSSG------------YYYPFDF | WGQGT LVTVS S |
| | | Germline | | H_FR1 | H_CDR1 | H_FR2 | H_CDR2 | H_FR3 | H_CDR3 | H_FR4 |
| VH3\|3-33/D3\|3-22\|RF2/JH1 | | | 32370 | QVQLVES-GGGVVQPGRSLRLSCAASG-FTFS | S-----YGMH | WVRQAPGK GLEWVA | VIWYD--- GSNKYYADS VKG | RFTISRDNSKNTLYLQMNSL RAEDTAVYYCAR | YYYDSSGY------------YYAEYFQH | WGQGT LVTVS S |
| iPS:4 35771 | 21-225_177B11 | VH3\|3-33/D3\|3-22\|RF2/JH1 | | QVQLVES-GGGVVQPGRSLRLTCAASG-FTFS | S-----YGMH | WVRQAPGK GLEWVA | IIWYD--- GSYKYYTDS VKG | RFTISRDNSKNTLYLQMNSL RAEDTAVYYCAR | ETYDFW------------SGYFVF | WGQGT LVTVS S |
| | | Germline | | H_FR1 | H_CDR1 | H_FR2 | H_CDR2 | H_FR3 | H_CDR3 | H_FR4 |
| VH3\|3-23/D5\|5-24\|RF3/JH4 | | | 32371 | EVQLLES-GGGLVQPGGSLRLSCAASG-FTFS | S-----YAMS | WVRQAPGK GLEWVS | AISGS--- GGSTYYADS VKG | RFTISRDNSKNTLYLQMNSL RAEDTAVYYCAK | RDGYN------------YYFDY | WGQGT LVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35775 | 21-225_178A5 | VH3\|3-23/D5\|5-24\|RF3/JH4 | | EVHLLES-GGGLVQTGGSLRLSCAASG-FTFS | S-----YAMT | WVRQAPGK GLEWVS | VISGS--- GGNTFYADS VKG | RFTISRDNSKNTLYLQMNSL RAEDTAVYYCAR | RDGD------------ ----------YFDY | WGQGT LVTVS S |
| iPS:4 37214 | 21-225_48B12 | VH3\|3-23/D5\|5-24\|RF3/JH4 | | EVQLLES-GGGLVQPGGSLRLSCAASG-FTFS | S-----YAMN | WVRQAPGK GLEWVS | AISGR--- GGNTFYADS VKG | RFTISRDNSKNTLYLQMNSL RAEDTAVYYCAK | RETYNWN--------- --------YEGFDY | WGQGT LVTVS S |
| iPS:3 93028 | 21-225_25D7 | VH3\|3-23/D5\|5-24\|RF3/JH4 | | EVQLLES-GGGLVQPGGSLRLSCAASG-FTFS | S-----YAMS | WVRQTPGQ GLEWVS | AISGR--- GGTTFYADS VKG | RFTISRDNSKNTLYLQMNSL RAEDTAVYYCAK | DCYCGN---------- ---------SFFDY | WGQGT LVTVS S |
| | Germline | | | H_FR1 | H_CDR1 | H_FR2 | H_CDR2 | H_FR3 | H_CDR3 | H_FR4 |
| | VH3\|3-33/D4\|4-11\|RF3/JH6 | | 32372 | QVQLVES-GGGVVQPGRSLRLSCAASG-FTFS | S------YGMH | WVRQAPGK GLEWVA | VIWYD--- GSNKYYADS VKG | RFTISRDNSKNTLYLQMNSL RAEDTAVYYCAR | IIVIYYY----------- --------YYGMDV | WGQGT TVTVS S |
| iPS:4 35789 | 21-225_180C4 | VH3\|3-33/D4\|4-11\|RF3/JH6 | | QVQLVES-GGGVVQPGRSLRLSCAASG-FTFS | A-----YGMH | WVRQAPGK GLEWVT | IIWYD--- GSYKYYADS VKG | RFAISRDNSKNTLYLQMNSL RAEDTAVYYCAR | TGVDPWD--------- -------YYNGMDV | WGQGT TVTVS S |
| | Germline | | | H_FR1 | H_CDR1 | H_FR2 | H_CDR2 | H_FR3 | H_CDR3 | H_FR4 |
| | VH3\|3-23-D2\|2-8\|RF1/JH3 | | 32373 & 32374 | EVQLLES-GGGLVQPGGSLRLSCAASG-FTFS | S------YAMS | WVRQAPGK GLEWVS | AISGS--- GGSTYYADS VKG | RFTISRDNSKNTLYLQMNSL RAEDTAVYYCAK | RILY*WCM-------- -------LYDAFDI | WGQGT MVTVS S |
| iPS:4 35799 | 21-225_181G3 | VH3\|3-23/D2\|2-8\|RF1/JH3 | | EVQLLES-GGGLVQPGGSLRLSCAASG-FTFS | S-----YAMS | WVRQAPGK GLEWVS | VISGS--- GGNTFYGDS VKG | RFTISRDNSKNTLYLQMNSL RAEDTAIYYCAK | RETYDWG--------- --------SDAFDI | WGQGT MVTVS S |
| | Germline | | | H_FR1 | H_CDR1 | H_FR2 | H_CDR2 | H_FR3 | H_CDR3 | H_FR4 |
| | VH3\|3-30.3/D5\|5-18\|RF2/JH6 | | 32375 | QVQLVES-GGGVVQPGRSLRLSCAASG-FTFS | S------YAMH | WVRQAPGK GLEWVA | VISYD--- GSNKYYADS VKG | RFTISRDNSKNTLYLQMNSL RAEDTAVYYCAR | WIQLWLYY-------- -------YYYGMDV | WGQGT TVTVS S |
| iPS:4 35811 | 21-225_183H6 | VH3\|3-30.3/D5\|5-18\|RF2/JH6 | | QVQLVES-GGGVVQPGRSLRLSCAASG-FTFS | S-----YGMH | WVRQAPGK GLEWVA | IISYA--- GSTKFYADS VKG | RFTISRDNSKNTLYLQMNSL RAEDTAVYYCVR | RPPQWLV--------- -------EGYGMDV | WGQGT TVTVS S |
| iPS:4 36754 | 21-225_155G3 | VH3\|3-30.3/D5\|5-18\|RF2/JH6 | | QVQLVES-GGGVVQPGRSLRLSCTASG-FTFS | S-----YGMH | WVRQAPGK GLEWVA | VISYD--- GSNKYYADS VKG | RFTISRDNSKNTLYLQMNSL RAEDTAVYYCAR | DTERWLP--------- -------YSYGMDV | WGQGT TVTVS S |
| iPS:4 48908 | 21-225_50G9 | VH3\|3-30.3/D5\|5-18\|RF2/JH6 | | QVQLVES-GGGVVQPGRSLRLSCAASG-FIFS | S-----YGMH | WVRQAPGK GLEWVA | VISQD--- GIIRYYADS VKG | RFTISRDNSKNTLYLQMNSL RAEDTAVYYCAR | DVKQWLV--------- -------RTYGMDV | WGQGT TVTVS S |
| | Germline | | | H_FR1 | H_CDR1 | H_FR2 | H_CDR2 | H_FR3 | H_CDR3 | H_FR4 |
| | VH3\|3-30.3/D6\|6-19\|RF1/JH5 | | 32376 | QVQLVES-GGGVVQPGRSLRLSCAASG-FTFS | S-----YAMH | WVRQAPGK GLEWVA | VISYD--- GSNKYYADS VKG | RFTISRDNSKNTLYLQMNSL RAEDTAVYYCAR | GYSSGW----------- --------YNWFDP | WGQGT LVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35813 | 21-225_183A12 | VH3\|3-30.3/D6\|6-19\|RF1/JH5 | | QVQLVES-GGGVVQPGRSLRLSCAASG-FTFS | S-----YGMH | WVRQAPGK GLEWVA | VISSA---GSNKYYADS VKG | RFTISRDNSKNTLYLQMNSL RAEDTAVYYCAR | RYSSGW-------------------DWFDP | WGQGT LVTVS S |
| | Germline | | | H_FR1 | H_CDR1 | H_FR2 | H_CDR2 | H_FR3 | H_CDR3 | H_FR4 |
| | VH3\|3-21/D3\|3-10\|RF3/JH4 | | 32377 | EVQLVES-GGGLVKPGGSLRLSCAASG-FTFS | S-----YSMN | WVRQAPGK GLEWVS | SISSS---SSYIYYADS VKG | RFTISRDNAKNSLYLQMNSL RAEDTAVYYCAR | IIMVRGV----------------IIYFDY | WGQGT LVTVS S |
| iPS:4 35815 | 21-225_190G10 | VH3\|3-21/D3\|3-10\|RF3/JH4 | | EVQLVES-GGGLVKPGGSLRLSCAASG-FTFR | D-----YSMN | WVRQAPGK GLEWVS | SISSG---SCYIHYADS VKG | RFTISRDNAKNSLYLQMNSL RAEDTAVYYCAR | ATMA-----------------LDY | WGQGT LVTVS S |
| iPS:4 35865 | 21-225_191A5 | VH3\|3-21/D3\|3-10\|RF3/JH4 | | EIQVVES-GGGLVKPGGSLRLSCAASG-FTFR | D-----YSMN | WVRQAPGK GLEWVS | SISSG---SGYIYYADS VKG | RFTISRDNAKNSLYLQMNSL RAEDTAVYYCAR | ATMA-----------------LDY | WGQGA LVTVS S |
| iPS:4 36047 | 21-225_193B10 | VH3\|3-21/D3\|3-10\|RF3/JH4 | | EVQLVES-GGGLVKPGGSLRLSCAASG-FTFR | D-----YSMN | WVRQAPGK GLEWVS | SISSA---GGYIYYADS LKG | RFTISRDNAKNSLYLQMNSL RAEDTAVYYCAR | ATMA-----------------LDY | WGQGT LVTVS S |
| iPS:4 36122 | 21-225_196G10 | VH3\|3-21/D3\|3-10\|RF3/JH4 | | EVQLVES-GGGLVKPGGSLRLSCAASG-FTFR | D-----YSMN | WVRQAPGK GLEWVS | SISSG---SGYIHYADS VKG | RFTISRDNAKNSLYLQMNSL RAEDTAVYYCAR | ATMA-----------------LDY | WGQGT LVTVS S |
| | Germline | | | H_FR1 | H_CDR1 | H_FR2 | H_CDR2 | H_FR3 | H_CDR3 | H_FR4 |
| | VH4\|4-59/D4\|4-17\|RF2/JH6 | | 32378 | QVQLQES-GPGLVKPSETLSLTCTVSG-GSIS | S-----YYWS | WIRQPPGK GLEWIG | YIYY----SGSTNYNPS LKS | RVTISVDTSKNQFSLKLSSV TAADTAVYYCAR | DYGDYYY---------------YYGMDV | WGQGT TVTVS S |
| iPS:4 35817 | 21-225_190B11 | VH4\|4-59/D4\|4-17\|RF2/JH6 | | QVQLQES-GPGLVKPSETLSLTCTVSG-GSIN | N-----YYWS | WIRQPAGK GLEWIG | RIYT----SGSTNYNPS LKS | RVTMSVDTSKNQFSLKLSSV TAADTAVHYCAR | DRGYYG-------------YYGMDV | WGQGT TVTVS S |
| iPS:4 35917 | 21-225_190D5 | VH4\|4-59/D4\|4-17\|RF2/JH6 | | QVQLQES-GPGLVKPSETLSLTCTVSG-GSIN | N-----YYWS | WIRQPAGK GLEWIG | RIYA----SGSTNYNPS LKS | RVTMSIDTSKNQFSLKLSSV TAADTAVYYCAR | DRGYYG-------------YYGMDV | WGQGT TVTIS S |
| iPS:4 36056 | 21-225_194C3 | VH4\|4-59/D4\|4-17\|RF2/JH6 | | QVQLQES-GPGLVKPSETLSLTCTVSG-GSIN | N-----YYWS | WIRQPAGK GLEWIG | RIYA----SGSTNYNPS LKS | RVTMSIDTSKNQFSLKLSSV TAADTAVHYCAR | DRGYYG-------------YYGMDV | WGQGT TVTIS S |
| iPS:4 36220 | 21-225_200F8 | VH4\|4-59/D4\|4-17\|RF2/JH6 | | QVQLQES-GPGLVKPSETLSLTCTVSG-GSIN | N-----YYWS | WIRQPAGK GLEWIG | RIYT----SGSTNYNPS LKS | RVTMSVDTSKNQFSLKLSSV TAADTAVYYCAR | DRGYYG-------------YYGMDV | WGQGT TVTVS S |
| | Germline | | | H_FR1 | H_CDR1 | H_FR2 | H_CDR2 | H_FR3 | H_CDR3 | H_FR4 |
| | VH3\|3-30/D4\|4-17\|RF2/JH6 | | 32379 | QVQLVES-GGGVVQPGRSLRLSCAASG-FTFS | S-----YGMH | WVRQAPGK GLEWVA | VISYD---GSNKYYADS VKG | RFTISRDNSKNTLYLQMNSL RAEDTAVYYCAK | DYGDYYY--------------YYGMDV | WGQGT TVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35821 | 21-225_190E11 | VH3\|3-30/D4\|4-17\|RF2/JH6 | | QVQLVES-GGGVVQPGRSLRLSCTASG-FTFS | N-----YGMH | WVRQAPGK GLEWVA | IIWFD---GSNKYYADSVKG | RFTISRDNSXNTLYLQMNSLRAEDTAVYYCAK | AQGVYY-------------------YVMDV | WGQGTTVTVS |
| | Germline | | | H_FR1 | H_CDR1 | H_FR2 | H_CDR2 | H_FR3 | H_CDR3 | H_FR4 |
| | VH3\|3-23/D5\|5-12\|RF3/JH5 | | 32380 | EVQLLES-GGGLVQPGGSLRLSCAASG-FTFS | S-----YAMS | WVRQAPGK GLEWVS | AISGS---GGSTYYADSVKG | RFTISRDNSKNTLYLQMNSLRAEDTAVYYCAK | GYSGYD---------------YNWFDP | WGQGTLVTVS |
| iPS:4 35823 | 21-225_190F11 | VH3\|3-23/D5\|5-12\|RF3/JH5 | | EVQLLDS-GGGLCQPGGSLRLSCAASG-FTFD | S-----YAMN | WVRQAPGK GLEWVS | TISGT---GRRTYYADSVKG | RFTISRDNSKNTLYLQMNSLRAEDTAVYYCAK | EEDYYDS---------SGPGFDP | WGQGTLVTVS |
| iPS:4 35867 | 21-225_191E5 | VH3\|3-23/D5\|5-12\|RF3/JH5 | | EVQLLDS-GGGLGQPGGSLRLSCAASG-FTFD | S-----YAMN | WVRQAPGK GLEWVS | TISGT---GRRTYYADSVKG | RFTISRDNSKNTLYLQMNSLRAEDTAVYYCAK | EEDYYDS---------SGPGFDP | WGQGTLVTVS |
| iPS:4 35929 | 21-225_190D9 | VH3\|3-23/D5\|5-12\|RF3/JH5 | | EVQLLDS-GGGLGQPGGSLRLSCAASG-FTFD | S-----YAMS | WVRQAPGK GLEWVS | TISGT---GRRTYYADSVKG | RFTISRDNSKNTLYLQMNSLRAEDTAVYYCAK | EEDYYDS---------SGPGFDP | WGQGTLVTVS |
| iPS:4 35935 | 21-225_190H8 | VH3\|3-23/D5\|5-12\|RF3/JH5 | | GVQLLDS-GGGLGQPGGSLRLSCAASG-FTFD | S-----YAMS | WVRQAPGK GLEWVS | TISGT---GRRTYYADSVKG | RFTISRDNSKNTLYLQMNSLRAEDTAVYYCAK | EEDYYDS---------SGPGFDP | WGQGTLVTVS |
| | Germline | | | H_FR1 | H_CDR1 | H_FR2 | H_CDR2 | H_FR3 | H_CDR3 | H_FR4 |
| | VH4\|4-39/D3\|3-9\|RF1/JH4 | | 32381 & 32382 | QLQLQES-GPGLVKPSETLSLTCTVSG-GSIS | SS----SYYWG | WIRQPPGK GLEWIG | SIYY----SGSTYYNPSLKS | RVTISVDTSKNQFSLKLSSVTAADTAVYYCAR | VLRYFDW----------LL*YFDY | WGQGTLVTVS |
| iPS:4 35827 | 21-225_190H11 | VH4\|4-39/D3\|3-9\|RF1/JH4 | | QLQLQES-GPGLVKPSETLSLTCTVSG-GSIR | S-----YHWS | WIRQPAGK GLEWIG | LIYT----SRSTIYNPSLKS | RVTLSVDTSKNQFSLKLSSVTAADTAVYYCAR | LRYNWN---------------FPYFDY | WGQGTLVTVS |
| iPS:4 35853 | 21-225_191E3 | VH4\|4-39/D3\|3-9\|RF1/JH4 | | QLQLQES-GPGLVKPSETLSLTCTVSG-GSIR | S-----YHWS | WIRQPAGK GLEWIG | LIYT----SRSTNYNPSLKS | RVTMSVDTSKNQFSLKLNSVTAADTAVYYCAR | LRYNWN---------------FPYFDY | WGQGTLVTVS |
| iPS:4 35871 | 21-225_191E6 | VH4\|4-39/D3\|3-9\|RF1/JH4 | | QLQLQES-GPGLVKPSETLSLTCTVSG-GSIR | S-----YHWS | WIRQPAGK GLEWIG | LIYT----SRSTNYNPSLKS | RVTMSVDTSKNQFSLKLSSVTAADTAVYYCAR | LRYNWN---------------FPYFDF | WGQGTLVTVS |
| iPS:4 35927 | 21-225_190E7 | VH4\|4-39/D3\|3-9\|RF1/JH4 | | QLQLQES-GPGLVKPSETLSLTCTVSG-GSIR | S-----YHWS | WIRQPAGK GLEWIG | HIYT----SRSTNYNPSLKS | RVTISVDTSKNQFSLKLSSVTATDTAVYYCAR | LRYNWN---------------FPYFDY | WGQGTLVTVS |
| iPS:4 35999 | 21-225_192F9 | VH4\|4-39/D3\|3-9\|RF1/JH4 | | QLQLQES-GPGLVKPSETLSLTCTVSG-GSIR | S-----YHWS | WIRQPAGK GLEWIG | LIYT----SRSTNYNPSLKS | RVTMSVDTSKNQFSLKLNSVTAADTAVYYCAR | LRYNWN---------------FPYFDY | WGQGTLVTVS |

| ID | Name | Germline | # | H_FR1 | H_CDR1 | H_FR2 | H_CDR2 | H_FR3 | H_CDR3 | H_FR4 |
|---|---|---|---|---|---|---|---|---|---|---|
| iPS:4 36060 | 21-225_194F4 | VH4\|4-39/D3\|3-9\|RF1/JH4 | | QLQLQES-GPGLVKPSEILSL TCTVSG-GSIS | S-----YHWS | WIRQPAGK GLEWIG | LIYT---- SRSTNYNPS LKS | RVTMSVDRSKSQFSLKLSSV TAADTAVYYCAR | LRYNWN-------------------FPYFDY | WGQGT LVTVS S |
| iPS:4 36193 | 21-225_19BA10 | VH4\|4-39/D3\|3-9\|RF1/JH4 | | QLQLQES-GPGLVKPSEILSL TCTVSG-GSIR | S-----YHWS | WIRQPAGK GLEWIG | HIYT---- SRSTNYNPS LKS | RVTISVDTSKNQFSLKLSSV TATDTAVYYCAR | LRYNWN-------------------FPYFDY | WGQGT LVTVS S |
| | **Germline** | | | **H_FR1** | **H_CDR1** | **H_FR2** | **H_CDR2** | **H_FR3** | **H_CDR3** | **H_FR4** |
| | **VH4\|4-30.1/D5\|5-24\|RF3/JH2** | | 32383 | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV TAADTAVYYCAR | RDGYNY-------------------YWYFDL | WGRGT LVTVS S |
| iPS:4 35829 | 21-225_190B12 | VH4\|4-30.1/D5\|5-24\|RF3/JH2 | | QVQLQES-GPGLVKPSQTLSL TCTVSG-GSIS | SG---GYYWN | WIRQHPGK GLEWIG | YIYY---- SGSTYYNPS LKS | RVTISVDTSKNQFFLKLNSV TAADTAVYYCAR | SGYNWD-------------------AGVDP | WGRGT LVTVS S |
| | **Germline** | | | **H_FR1** | **H_CDR1** | **H_FR2** | **H_CDR2** | **H_FR3** | **H_CDR3** | **H_FR4** |
| | **VH3\|3-33/D4\|4-11\|RF2/JH6** | | 32384 | QVQLVES-GGGVVQPGRSLRL SCAASG-FTFS | S-----YCMH | WVRQAPGK GLEWVA | VIWYD--- GSNKYYADS VKG | RFTISRDNSKNTLYLQMNSL RAEDTAVYYCAR | DYSNYYY-------------YYYGMDV | WGQGT TVTVS S |
| iPS:4 35835 | 21-225_190F12 | VH3\|3-33/D4\|4-11\|RF2/JH6 | | QVQLVES-GGGVVQPGRSLRL SCAASG-FTFR | N-----YGMH | WVRQAPGK GLEWVA | VIWFD--- GSNDYYADS VKG | RFTISRDNSKNTLSLQMNSL RAEDTAVYYCAR | DRSVGY-------------------DGLDV | WGQGT TVTVS S |
| iPS:4 35861 | 21-225_190A5 | VH3\|3-33/D4\|4-11\|RF2/JH6 | | QVQLVES-GGGVGQPGRSLRL SCAASG-FTFS | S-----YGMH | WVRQAPGK GLEWVA | VIWYD--- GSNKNYADS VKG | RFTISRDNSKNTLYLQMNSL RAEDTAVYYCAR | DFSVGY-------------------DGMDV | WGQGT TVTVS S |
| iPS:4 35937 | 21-225_190H9 | VH3\|3-33/D4\|4-11\|RF2/JH6 | | QVQLVES-GGGVVQPGRSLRL SCAASG-FTFR | N-----YGMH | WVRQAPGK GLEWVA | VIWFD--- GSNDYYADS VKG | RFTISRDNSKNTLSLQMNSL RAEDTAVYYCAR | DRSVGY-------------------DGLDV | WGQGT TVTVS S |
| iPS:4 35977 | 21-225_192E4 | VH3\|3-33/D4\|4-11\|RF2/JH6 | | QVQLVES-GGGVVQPGRSLRL SCAASG-FTFS | S-----YGMH | WVRQAPGK GLEWVA | VIWYD--- GSNKNYVDS VRG | RFTISRDNSKNTLYLQMNSL RAEDTAVYYCAR | DRSVGY-------------------DGMDV | WGQGT TVTVS S |
| iPS:4 36001 | 21-225_192C10 | VH3\|3-33/D4\|4-11\|RF2/JH6 | | QVQLVES-GGGVVQPGRSLRL SCAASSG-FTFR | N-----YGMH | WVRQAPGK GLKWVA | VIWFD--- GSNDYYADS VKG | RFTISRDNSKNTLSLQMNSL RAEDTAVYYCAR | DRSVGY-------------------DGLDV | WGQGT TVTVS S |
| iPS:4 36066 | 21-225_194B7 | VH3\|3-33/D4\|4-11\|RF2/JH6 | | QVQLVES-GGGVVQPGRSLRL SCAASG-FTFS | S-----YGMH | WVRQAPGK GLEWVA | VIWYD--- GSNKNYADS VKG | RFTISRDNSKNTLYLQMNSL RAEDTAVYYCAR | DRSKGY-------------------DGMDV | WGQGT TVTVS S |
| iPS:4 36078 | 21-225_194H12 | VH3\|3-33/D4\|4-11\|RF2/JH6 | | QVQLVES-GGGVVQPGRSLRL SCAASG-FTFR | N-----YGMH | WVRQAPGK GLEWVA | VIWFD--- GSNDYYADS VKG | RFTISRDNSKNTLSLQMNSL RAEDTAVYYCAR | DRSVGY-------------------DGLDV | WGQGT TVTVS S |

| iPS:4 36140 | 21-225_197G3 | VH3\|3-33/D4\|4-11\|RF2/J H6 | | QVQLVES-GGGVVQPGRSLRL SCVASG-FTFS | S-----HGMH | WVRQAPGK GLEWVA | VIWYD---GSNKNYADS VKG | RFTISRDNSKNTLYLQMNSL RAEDTAVYYCAR | DPSVGY-------------------DGMDV | WGQGT TVTVS S |
| iPS:4 36167 | 21-225_197E11 | VH3\|3-33/D4\|4-11\|RF2/J H6 | | QVQLVES-GGGVVQPGRSLRL SCAASG-FTFR | N-----YGMH | WVRQAPGK GLEWVA | VIWFD---GSNDYYADS VKG | RFTISRDNSKNTLSLQMNSL RAEDTAVYYCAR | DRSVGY-------------------DGLDV | WGQGT TVTVS S |
| iPS:4 36292 | 21-225_205H3 | VH3\|3-33/D4\|4-11\|RF2/J H6 | | QVQLVES-GGGVVQPGRSLRL SCAASG-FTFS | S-----YGMH | WVRQAPGK GLEWVA | VIWYD---GSNKYYADS VKG | RFTISRDNSKNTLYLQMNSL RAEDTAVYYCAR | DRSVGY-------------------DGTDV | WGQGT TVTVS S |
| iPS:4 36802 | 21-225_171E12 | VH3\|3-33/D4\|4-11\|RF2/J H6 | | QVQLVES-GGGVVQPGRSLRL SCAASG-FTFS | S-----YGMH | WVRQAPGK GLEWVA | VIWND---GGNKYNGDS VKG | RFTISRDNSKNTLYLQMNSL RAEDTAVYYCAR | DRTYYSGSGSP---------PYYYYYGMDV | WGQGT TVTVS S |
| iPS:4 36816 | 21-225_179H5 | VH3\|3-33/D4\|4-11\|RF2/J H6 | | QVQLVES-GGGVVQPGRSLRL SCAASG-FTFS | S-----YGMH | WVRQAPGK GLEWVA | VIWYD---GSNEYYADS VKG | RFTISRDNSKNTLYLQMNSL RAEDTAVYYCAR | DIRNYY-------------------YGLDV | WGQGT TVTVS S |
| iPS:4 36960 | 21-225_198D2 | VH3\|3-33/D4\|4-11\|RF2/J H6 | | QVQLVES-GGGVVQPGRSLRL SCAASG-FTFR | S-----YGMH | WVRQAPGK GLEWVA | VIIYD---GSYKYYADS VKG | RFTISRDNSKNTLYLQMNSL RAEDTAVYYCAR | TYSG-------------------GMDV | WGQGT TVTVS S |
| iPS:4 36974 | 21-225_190H7 | VH3\|3-33/D4\|4-11\|RF2/J H6 | | QVQLVES-GGGVVQPGRSLRL SCAASG-FNFR | S-----YGMH | WVRQAPGK GLEWVA | VIIYD---GSYKYYADS VKG | RFTISRDNSKNTLYLQMNSL RAEDTAVYYCAR | TYSG-------------------GMDV | WGQGT TVTVS S |
| iPS:4 36982 | 21-225_190D10 | VH3\|3-33/D4\|4-11\|RF2/J H6 | | QVQLVES-GGGVVQPGRSLRL SCAASG-FTFR | S-----YGMH | WVRQAPGK GLEWVA | VIIYD---GSYKYYADS VKG | RFTISRDNSKNTLYLQMNSL RAEDTAVYYCAR | TYSG-------------------GMDV | WGQGT TVTVS S |
| iPS:4 37274 | 21-225_196D4 | VH3\|3-33/D4\|4-11\|RF2/J H6 | | QVQVVES-GGGVVQPGRSLRL SCAASG-FTFS | S-----YGMH | WVRQAPGK GLEWVA | VIWYD---GSNRNYADS VKG | RFTISRDNSKNTLYLQMNSL RVEDTAVYYCAR | DRSKGY-------------------DGMDV | WGQGT TVTVS S |
| iPS:3 92664 | 21-225_20F6 | VH3\|3-33/D4\|4-11\|RF2/J H6 | | QVQLVES-GGGVVQPGRSLRL SCVASG-FTFS | S-----YGMH | WVRQAPGK GLEWGA | VIWHD---GSNKYYADS VKG | RFTISRDNAKNTLYLQMNSL RAEDTAVYYCAR | DLSMG-------------------GMDV | WGQGT TVTVS S |
| iPS:3 92738 | 21-225_18G4 | VH3\|3-33/D4\|4-11\|RF2/J H6 | | QVQLVES-GGGVVQPGRSLRL SCAASG-FTFS | S-----YGMH | WVRQAPGK GLEWVA | IIWYD---GSNKYYADS VKG | RFTISRDNSKNTLYLQMNSL RAEDTAVYYCAR | DLSMG-------------------GMDV | WGQGT TVTVS S |
| iPS:3 92798 | 21-225_22C7 | VH3\|3-33/D4\|4-11\|RF2/J H6 | | QVQLVES-GGGVVQPGRSLRL SCAASG-FTFS | S-----YGMH | WVRQAPGK GLEWGA | VIWHD---GSNKYYADS VKG | RFTISRDNAKNTLYLQMNSL RAEDTAVYYCAR | DLSMG-------------------GMDV | WGQGT TVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| iPS:3 92956 | 21-225_27A11 | VH3\|3-33/D4\|4-11\|RF2/JH6 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYHCAR | DSSPY------------------GMDV | WGQGT TVTVS S |
| iPS:3 92994 | 21-225_26G11 | VH3\|3-33/D4\|4-11\|RF2/JH6 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | RYSNSW-------------------SGGMDV | WGQGT TVTVS S |
| iPS:3 93014 | 21-225_26D12 | VH3\|3-33/D4\|4-11\|RF2/JH6 | | QVQLVES-GGGVVQPGRSLRL SCAASG-FTFS | S-----YGMH | WVRQAPGK GLEWVA | VIWYD--- GSNEYYADS VKG | RFTISRDNSKNTLYLQMNSL RAADTAVYYCAR | DSSPY------------------GMDV | WGQGT TVTVS S |
| iPS:3 93152 | 21-225_25B3 | VH3\|3-33/D4\|4-11\|RF2/JH6 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | DSSPY------------------GMDV | WGQGT TVTVS S |
| iPS:3 93840 | 21-225_3F8 | VH3\|3-33/D4\|4-11\|RF2/JH6 | | QVQLVES-GGGVVQPGRSLRL SCAASG-FTFS | S-----YGMH | WVRQAPGK GLEWGA | VIWHD--- GSNKYYADS VKG | RFTISRDNAKNTLYLQMNSL RAEDTAVYYCAR | DLSMG------------------GMDV | WGQGT TVTVS S |
| iPS:3 93930 | 21-225_7E11 | VH3\|3-33/D4\|4-11\|RF2/JH6 | | QVQLVES-GGGVVQPGRSLRL SCAASG-FTFS | S-----FGMH | WVRQAPGK GLEWVA | IIWHD--- GSNKYYADS VKG | RFTISRDNSNNTLYLQMSSL RAEDTAVYYCAR | DLSMG------------------GMDV | WGQGT TVTVS S |
| iPS:3 93964 | 21-225_6G1 | VH3\|3-33/D4\|4-11\|RF2/JH6 | | QVQLVES-GGGVVQPGRSLRL SCAASG-FIFS | S-----YGMH | WVRQAPGK GLEWVA | IIWYD--- GSNKYYADS VKG | RFTISRDNSKNTLYLQMNSL RAEDTAMYYCAR | DLSMG------------------GMDV | WGQGT TVTVS S |
| iPS:3 94012 | 21-225_15A3 | VH3\|3-33/D4\|4-11\|RF2/JH6 | | QVQLVES-GGGVVQPGRSLRL SCAASG-FTFS | S-----YGIH | WVRQAPGK GLEWVA | VIWHD--- GSNKYYADS VKG | RFTISRDNSKNTLYLQMNSL RAEDTAVYYCAR | DLSMG------------------GMDV | WGQGT TVTVS S |
| iPS:3 94016 | 21-225_13D4 | VH3\|3-33/D4\|4-11\|RF2/JH6 | | QVQLVES-GGGVVQPGRSLRL SCAASG-FTFS | S------YGMH | WVRQAPGK GLEWVA | VIWHD--- GSNKYYADS VKG | RFTISRDNSKNTLYLQMNSL RAEDTAVYYCAR | DLSMG------------------GMDV | WGQGT TVTVS S |
| iPS:3 94083 | 21-225_16E6 | VH3\|3-33/D4\|4-11\|RF2/JH6 | | QVQLVES-GGGVVQPGRSLRL SCAASG-FTFS | S-----YGMH | WVRQAPGK GLEWVA | VIWHD--- GSNKYYVDS VKG | RFTISRDNSKNTLNLQMNSL RAEDTAVYYCAR | DLSMG------------------GMDV | WGQGT TVTVS S |
| | | Germline | H_FR1 | H_CDR1 | H_FR2 | H_CDR2 | H_FR3 | H_CDR3 | H_FR4 |
| | VH4\|4-59/D3\|3-9\|RF1/JH4 | | 32385 & 32386 | QVQLQES-GPGLVKPSETLSL TCTVSG-GSIS | S-------YYWS | WIRQPPGK GLEWIG | YIYY---- SGSTNYNPS LKS | RVTISVDTSKNQFSLKLSSV TAADTAVYYCAR | VLRYFDW-------------------LL*YFDY | WGQGT LVTVS S |
| iPS:4 35839 | 21-225_191B1 | VH4\|4-59/D3\|3-9\|RF1/JH4 | | QVQLQES-GPGLVKPSETLSL TCTVSG-GSIS | S-----YHWS | WIRQPAGK GLEWIG | HIYT---- SGSTKYNPS LKS | RVTMSVDTSKNQFSLKLSSV TAADTAVYYCAR | LRYNWN-------------------FPFFDY | WGQGT LVTVS S |

| iPS:4 | | | | H_FR1 | H_CDR1 | H_FR2 | H_CDR2 | H_FR3 | H_CDR3 | H_FR4 |
|---|---|---|---|---|---|---|---|---|---|---|
| iPS:4 36158 | 21-225_197G8 | VH4\|4-59/D3\|3-9\|RF1/JH4 | | QVHLQES-GPGLVKPSEILSL TCTVSG-GSIS | A-----YSWS | WIRQPAGK GLEWIG | RLSP---- GGSTNFNPS LKS | RVTMSVDISKNQFSLRLSSV TAADTAVYYCAR | LRYNWN--------- --------FPYFDY | WGQGA LVTVS S |
| | | Germline | | H_FR1 | H_CDR1 | H_FR2 | H_CDR2 | H_FR3 | H_CDR3 | H_FR4 |
| | VH4\|4-30.1/D3\|3-22\|RF2/JH6 | | 32387 | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV TAADTAVYYCAR | YYYDSSGYYY------ -----YYYYGMDV | WGQGT TVTVS S |
| iPS:4 35843 | 21-225_191F1 | VH4\|4-30.1/D3\|3-22\|RF2/J H6 | | QVQLQES-GPGLVKPSQTLSL TCTVSG-GSIN | SG---GYYWN | WIRQHPGK GLEWIG | YIFY---- SGSTYYNPS LKS | RVTISVDTSKNQFSLKLSSV TAADTAVYYCAR | GDYDGSGSY------- ------HYYYGMDV | WGQGT TVTVS S |
| iPS:4 35847 | 21-225_191A3 | VH4\|4-30.1/D3\|3-22\|RF2/J H6 | | QVQLQES-GPGLVKPSQTLSL TCTVSG-GSIS | SG---DYYWN | WIRQHPGK GLEWIG | YIFY---- SGSTYYNPS LKS | RVTISVDTSKNQFSLKLSSV TAADTAVYYCAR | GDYDGSGSY------- ------HYYYGMDV | WGQGT TVTVS S |
| iPS:4 35851 | 21-225_191D3 | VH4\|4-30.1/D3\|3-22\|RF2/J H6 | | QVQLKES-GPGLVKPSQTLSL TCTVSG-GSIN | SG---DYYWN | WIRQHPGK GLDWIG | YIFY---- SGSTYYNPS LKS | RVTISVDTSKNQFSLKLSSV TAADTAVYYCAR | GDYDGSGSY------- ------HYYYGMDV | WGQGT TVTVS S |
| iPS:4 35905 | 21-225_190A3 | VH4\|4-30.1/D3\|3-22\|RF2/J H6 | | QVQLQES-GPGLVKPSQTLSL TCTVSG-GSIN | SG---GYYWN | WIRQHPGK GLEWIG | FIFY---- SGSTYYNPS LKS | RVTISVDTSKNQFSLKLNSV TAADTAVYYCAR | GDYDGSGSY------- ------HYYYGMDV | WGQGT TVTVS S |
| iPS:4 35911 | 21-225_190B4 | VH4\|4-30.1/D3\|3-22\|RF2/J H6 | | QVQLQES-GPGLVKPSQTLSL TCTVSG-GSIS | SG---DYYWN | WIRQHPGK GLEWIG | YIFY---- SGSTYYNPS LKS | RVTISVDTSKNQFSLKLSSV TAADTAVYYCAR | GDYDGSGSY------- ------HYYYGMDV | WGQGT TVTVS S |
| iPS:4 35913 | 21-225_190A7 | VH4\|4-30.1/D3\|3-22\|RF2/J H6 | | QVQLQES-GPGLVNPSQTLSL TCTVSG-GSIS | SG---VYYWS | WIRQHPGK GLEWIG | NIYY---- SGSTYNNPS LKS | RIIISVDTSKNQFSLKLSSV TAADTAVYYCAR | GDYDGSGSY------- ------HYYYGLDV | WGQGT TVTVS S |
| iPS:4 35939 | 21-225_191H7 | VH4\|4-30.1/D3\|3-22\|RF2/J H6 | | QVQLQES-GPGLVKPSQTLSL TCTVSG-GSIN | SG---DYYWN | WIRQHPGK GLEWIG | YIFY---- SGSTYYNPS LKS | RVTISVDTSKNQFSLKLSSV TAADTAVYYCAR | GDYDGSGSY------- ------HYYYGMDV | WGQGT TVTVS S |
| iPS:4 35967 | 21-225_192B3 | VH4\|4-30.1/D3\|3-22\|RF2/J H6 | | QVQLQES-GPGLVKPSQTLSL TCTVSG-GSIS | SG---DYYWN | WIRQHPGK GLEWIG | FIFY---- SGSTYYNPS LKS | RVTISVDTSKNQFSLKLSSV TVADTAVYYCAR | GDYDGSGSY------- ------HHYYGMDV | WGQGT TVTVS S |
| iPS:4 35973 | 21-225_192H3 | VH4\|4-30.1/D3\|3-22\|RF2/J H6 | | QVQLQES-GPGLVKPSQTLSL TCTVSG-GSIS | SV---SYYWS | WIRQRPGK GLEWIG | NLYY---- SGSTYYNPS LRS | RATISVDTSKNQFSLKLSSV TAADTAVYYCTR | GDYDGSGSY------- ------HYYHGMDV | WGQGT TVTVS S |
| iPS:4 36007 | 21-225_192G12 | VH4\|4-30.1/D3\|3-22\|RF2/J H6 | | QVQLQES-GPGLVKPSQTLSL TCTVSG-GSIS | SG---VYHWS | WIRQHPGK GLEWIG | NIHY---- SGSTYNNPS LKS | RVTISVDTSKNQFSLKLSSV TAADTAVYYCAR | GDYDGSGSY------- ------HYYYGMDV | WGQGT TVTVS S |

| iPS:4 36009 | 21-225_193A1 | VH4\|4-30.1/D3\|3-22\|RF2/JH6 | | QVQLQES-GPGLVKPSQTLSLTCTVSG-GSIS | SG---VYYWS | WIRQHPGKGLEWIG | NIYY----SGSTYNNPSLKS | RLTISADISKNQFSLKLSSVTAADTAVYYCAR | GDYDGSGSY-------------HYYYGMDV | WGQGTTVTVSS |
| iPS:4 36011 | 21-225_193B1 | VH4\|4-30.1/D3\|3-22\|RF2/JH6 | | QVQLQES-GPGLVKPSQTLSLTCTVSG-GSIS | SG---VYYWS | WIRQHPGKGLEWIG | NIYY----SGSTYNNPSLKS | RVTISVDTSKNQFSLKLSSVTAADTAVYYCAR | GDYDGSGSY-------------HYYYGMDV | WGQGTTVTVSS |
| iPS:4 36017 | 21-225_193F3 | VH4\|4-30.1/D3\|3-22\|RF2/JH6 | | QVQLQES-GPGLVKPSQTLSLTCTVSG-GSIN | SG---DYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 36029 | 21-225_193H6 | VH4\|4-30.1/D3\|3-22\|RF2/JH6 | | QVQLQES-GPGLVKPSQTLSLTCTVSG-GSIN | SG---DYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 36035 | 21-225_193C8 | VH4\|4-30.1/D3\|3-22\|RF2/JH6 | | QVQLQES-GPGLVKPSQTLSLTCTVSG-GSIS | SG---DYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 36037 | 21-225_193D8 | VH4\|4-30.1/D3\|3-22\|RF2/JH6 | | QVQLQES-GPGLVKPSQTLSLTCTVSG-GSIN | SG---GYYWN | WIRQHPGKGLEWIG | FIFY----SGSTYYNPSLKS | RVSISVDTSKNQFSLKLSSVTAADTAVYYCAR | GDYDGSGSY-------------HYYYGMDV | WGQGTTVTVSS |
| iPS:4 36041 | 21-225_193G8 | VH4\|4-30.1/D3\|3-22\|RF2/JH6 | | QVQLQEK-GPGLVKPSQTLSLTCTVSG-GSVS | SG---VYYWS | WIRQHPGKGLEWIG | NIYY----SGSTYNNPSLKS | RVTISVDTSKNQFSLKLNSVTAADTAVYYCAR | GDYDGSGSY-------------HFYYGLDV | WGHGTTVTVSS |
| iPS:4 36062 | 21-225_194E5 | VH4\|4-30.1/D3\|3-22\|RF2/JH6 | | QVQLQES-GPGLVKPSQTLSLTCTVSG-GSIN | SG---DYYWN | WIRHHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 36064 | 21-225_194E6 | VH4\|4-30.1/D3\|3-22\|RF2/JH6 | | QVQLQES-GPGLVKPSQTLSLTCTVSG-DSIN | SG---DYYWN | WIRQHPGKGLEWIG | FIFY----SGSTYYNPSLKS | RVTISIDTSKNQFSLKLSSVNVADTAVYYCAR | GDYDGSGSY-------------HYYYGMDV | WGQGTTVTVSS |
| iPS:4 36134 | 21-225_196H12 | VH4\|4-30.1/D3\|3-22\|RF2/JH6 | | QVQLQES-GPGLVNPSQTLSLTCTVSG-GSIS | SG----VYYWS | WIRQHPGKGLEWIG | NIYY----SGSTYNNPSLKS | RIIISVDTSKNQFSLKLSSVTAADTAVYYCAR | GDYDGSGSY-------------HYYYGLDV | WGQGTTVTVSS |
| iPS:4 36146 | 21-225_197F4 | VH4\|4-30.1/D3\|3-22\|RF2/JH6 | | QVQLQES-GPGLVKPSQTLSLTCTVSG-GSIS | SG---DYYWN | WIRQHPGKGLEWIG | YIFY----SGSTYYNPSLKS | RITISVDTSKNQFSLKLSSVTAADTAVYYCAR | GDYDGSGSY-------------HYYYGLDV | WGQGTTVTVSS |
| iPS:4 36177 | 21-225_198B1 | VH4\|4-30.1/D3\|3-22\|RF2/JH6 | | QVQLKES-GPGLVKPSQTLSLTCTVSG-GSIN | SG---DYYWN | WFRQHPGKGLEWIG | YIFH----SGSTYYNPSLKS | RVTVSVDTSKNQFSLKLSSVTAADTAVYYCAR | GDYDGSGSY-------------HYYYGMDV | WGRGTTVTVSS |

| | | Germline | | | | | | | | |
|---|---|---|---|---|---|---|---|---|---|---|
| iPS:4 36179 | 21-225_198E1 | VH4\|4-30.1/D3\|3-22\|RF2/JH6 | QVQLQES-GPGLVKPSQTLSLTCTVSG-GSIN | SG---GYYWN | WIRQHPGKGLEWIG | FIFY----SGSTYYNPSLKS | RLSISVDTSKNQFSLKLSSVTAADTAVYYCAR | GDYDGSGSY-------------HYYYGMDV | WGQGTTVTVSS |
| iPS:4 36197 | 21-225_199C2 | VH4\|4-30.1/D3\|3-22\|RF2/JH6 | QVQLQES-GPGLVKPSQTLSLTCTVSG-GSIS | SG---DYYWN | WLRQHPEKGLEWIG | YIFY----SGSTYYNPSLKS | RVTISVDTSMTQFSLKLTSVTAADTAVYYCAR | GDYDGSGSY-------------HYYYGMDV | WGQGTTVTVSS |
| iPS:4 36207 | 21-225_199C7 | VH4\|4-30.1/D3\|3-22\|RF2/JH6 | QVQLQES-GPGLVKPSQTLSLTCTVSG-GSIN | SG---GYYWN | WIRQHPGKGLEWIG | FIFY----SGSTYYNPSLKS | RVSISVDTSKNQFSLKLSSVTAADTAVYYCAR | GDYDGSGSY-------------HYYYGMDV | WGQGTTVTVSS |
| iPS:4 36226 | 21-225_200F10 | VH4\|4-30.1/D3\|3-22\|RF2/JH6 | QVQLQES-GPGLVKPSQTLSLTCTVSG-GSIS | SG---DYYWN | WLRQHPEKGLEWIG | YIFY----SGSTYYNPSLKS | RITISVDTSMTQFSLKLTSVTAADTAVYYCAR | GDYDGSGSY-------------HYYYGMDV | WGQGTTVTVSS |
| | | Germline | H_FR1 | H_CDR1 | H_FR2 | H_CDR2 | H_FR3 | H_CDR3 | H_FR4 |
| | VH4\|4-30.4/D3\|3-22\|RF2/JH6 | 32388 | QVQLQES-GPGLVKPSQTLSLTCTVSG-GSIS | SG---DYYWS | WIRQPPGKGLEWIG | YIYY----SGSTYYNPSLKS | RVTISVDTSKNQFSLKLSSVTAADTAVYYCAR | YYYDSSGYYY-------------YYYYGMDV | WGQGTTVTVSS |
| iPS:4 35849 | 21-225_191C3 | VH4\|4-30.4/D3\|3-22\|RF2/JH6 | QVQLQES-GPGLVKPSQTLSLTCTVSG-GSIS | SG---DYYWN | WIRQLPGKGLEWIG | YIFY----SGSTYYNPSLKS | RLTISVDTSKNQFSLKLNSVTAADTAVYYCAR | GDYDGSGSY-------------HFYYGMDV | WGQGTTVTVSS |
| iPS:4 36015 | 21-225_193D3 | VH4\|4-30.4/D3\|3-22\|RF2/JH6 | QVQLQES-GPGLVKPSQTLSLTCTVSG-GSIS | SG---DYYWN | WIRQLPGKGLEWIG | YIFY----SGSTYYNPSLKS | RLTISVDTSKNQFSLKLSSVTAADTAVYYCTR | GDYDGSGSY-------------HFYYGMDV | WGQGTTVTVSS |
| iPS:4 36049 | 21-225_193B12 | VH4\|4-30.4/D3\|3-22\|RF2/JH6 | QVQLQES-GPGLVKPSQTLSLTCTVSG-GSIS | SA---DYYWN | WIRQLPGKGLEWIG | YIFY----SGSTYYNPSLKS | RLTISVDTSKNQFSLKLSSVTAADTAVYYCAR | GDYDGSGSY-------------HFYYGMDV | WGQGTTVTVSS |
| iPS:4 36088 | 21-225_195C8 | VH4\|4-30.4/D3\|3-22\|RF2/JH6 | QVQLQES-GPGLVKPSQTLSLTCTVSG-GSIS | SG---DYYWN | WIRQLPGKGLEWIG | YIFY----SGSTYYNPSLKS | RLTISVDTSKNQFSLKLSSVTAADTAVYYCAR | GDYDGSGSY-------------HFYYGMDV | WGQGTTVTVSS |
| iPS:4 36195 | 21-225_198G10 | VH4\|4-30.4/D3\|3-22\|RF2/JH6 | QVQLQES-GPGLVKPSQTLSLTCTVSG-GSIS | SG---DYYWN | WIRQLPGKGLEWIG | YIFY----SGSTYYNPSLKS | RLTISVDTSKNQFSLKLSSVTAADTAVYYCAR | GDYDGSGSY-------------HFYYGMDV | WGQGTTVTVSS |
| | | Germline | H_FR1 | H_CDR1 | H_FR2 | H_CDR2 | H_FR3 | H_CDR3 | H_FR4 |
| | VH4\|4-30.1/D5\|5-24\|RF3/JH5 | 32389 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | RDCYNY---------NWFDP | WGQGTLVTVSS |
| iPS:4 35863 | 21-225_191H4 | VH4\|4-30.1/D5\|5-24\|RF3/JH5 | QVQLQES-GPGLVKPSQTLSLTCTVSG-GSIS | SG---GYYWN | WIRQHPGKGLEWIG | YIFY----SGSTYYNPSLRS | RLTISIDTSKNQFSLKLTSVTAADTAVYYCGR | SGYNWD---------NGVDP | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 35943 | 21-225_191C9 | VH4\|4-30.1/D5\|5-24\|RF3/JH5 | | QVQLQES-GPGLVKPSQILSLTCTVSG-GSIS | SG---GYYWN | WIRQHPGK GLDWIG | YIYY----SGSTYYNPS LKS | RVTISVDISKNQFSLKLNSV TAADTAVYYCAR | SGYNWD-------------------AGVDP | WGQGT LVTVS S |
| iPS:4 36094 | 21-225_195B10 | VH4\|4-30.1/D5\|5-24\|RF3/JH5 | | QVQLQES-GPGLVKPSQILSLTCTVSG-GSIS | NS---GYYWS | WIRQHPGK GLEWIG | YMYY----SGSTYYNPS LKS | RVTISVDISKNQFYLKLSAV TAADTAVYYCAK | GGYNWN-------------------NGFDC | WGQGT LVTVS S |
| | **Germline** | | | **H_FR1** | **H_CDR1** | **H_FR2** | **H_CDR2** | **H_FR3** | **H_CDR3** | **H_FR4** |
| | VH3\|3-33/D2\|2-15\|RF3/JH6 | | 32390 | QVQLVES-GGGVVQPGRSLRLSCAASG-FTFS | S------YGMH | WVRQAPGK GLEWVA | VIWYD---GSNKYYADS VKG | RFTISRDNSKNTLYLQMNSL RAEDTAVYYCAR | DIVVVVAAT------------YYYYYGMDV | WGQGT TVTVS S |
| iPS:4 35869 | 21-225_190B1 | VH3\|3-33/D2\|2-15\|RF3/JH6 | | QVQLVES-GGGVVQPGRSLRLSCATSG-FTFS | S------YGMH | WVRQAPGK GLEWVA | VIWYD---GSNKHYADS VKG | RFTISRDNSKNTLYLQMNSL RAEDTAVYYCAR | DRTVGY----------------SGMDV | WGQGT TVTVS S |
| iPS:4 36260 | 21-225_203H1 | VH3\|3-33/D2\|2-15\|RF3/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DRTVGY----------------NGMDV | WGQGT TVTVS S |
| iPS:4 36490 | 21-225_221F6 | VH3\|3-33/D2\|2-15\|RF3/JH6 | | QVQLVES-GGGVVQPGRSLRLSCAASG-FTFS | S-----NGMH | WVRQAPGK GLEWVA | VIWYD---GSNENYADS VKG | RFTISRDNSKNTLYLQMNSL RAEDTAVYYCAR | DRTVGY----------------NGMDV | WGQGT TVTVS S |
| iPS:4 36502 | 21-225_222A11 | VH3\|3-33/D2\|2-15\|RF3/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DRDVGY----------------NGMDV | WGQGT TVTVS S |
| iPS:4 36514 | 21-225_222D10 | VH3\|3-33/D2\|2-15\|RF3/JH6 | | QVQLVES-GGGVVQPGRSLRLSCAASG-FTFS | S-----YGMH | WVRQAPGK GLEWVA | VIWYD---GSNKNYADS VKG | RFTISRDNSKNTLYLQMNSL RAEDTAVYYCAR | DRDVGY----------------NGMDV | WGQGT TVTVS S |
| iPS:4 36522 | 21-225_223H10 | VH3\|3-33/D2\|2-15\|RF3/JH6 | | QVQLVES-GGGVVQPGRSLRLSCAASG-FTFS | S-----YGMH | WVRQAPGK GLEWVA | VIWYD---GSNKNYADS VKG | RFTISRDHSKNTLYLQMNSL RAEDTAVYYCAR | DRDVGY----------------NGMDV | WGQGT TVTVS S |
| | **Germline** | | | **H_FR1** | **H_CDR1** | **H_FR2** | **H_CDR2** | **H_FR3** | **H_CDR3** | **H_FR4** |
| | VH3\|3-21/D1\|1-1\|RF3/JH5 | | 32391 | EVQLVES-GGGLVKPGGSLRLSCAASG-FTFS | S------YSMN | WVRQAPGK GLEWVS | SISSS---SSYIYYADS VKG | RFTISRDNAKNSLYLQMNSL RAEDTAVYYCAR | YNWND-----------------NWFDP | WGQGT LVTVS S |
| iPS:4 35883 | 21-225_185A1 | VH3\|3-21/D1\|1-1\|RF3/JH5 | | EVQLVES-GGGLVKPGGSLRLSCAASG-FTFN | S-----YSMN | WVRQAPGK GLEWVS | SISSS---GSYIYYADS VKG | RFTISRDNAKNSLYLQMHSL RAEDTAVYYCAR | SNL-------------------FDC | WGQGT PVTVS S |
| | **Germline** | | | **H_FR1** | **H_CDR1** | **H_FR2** | **H_CDR2** | **H_FR3** | **H_CDR3** | **H_FR4** |
| | VH3\|3-23/D1\|1-1\|RF1/JH3 | | 32382 | EVQLLES-GGGLVQPGGSLRLSCAASG-FTFS | S-----YAMS | WVRQAPGK GLEWVS | AISGS---GGSTYYADS VKG | RFTISRDNSKNTLYLQMNSL RAEDTAVYYCAK | GITGT-----------------DAFDI | WGQGT MVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 35895 | 21-225_188E8 | VH3\|3-23/D1\|1-1\|RF1/JH3 | | EVQLLES-GGGLVQPGGSLRLSCAASG-FTFS | S-----SAMN | WVRQAPGK GLEWVS | VISGS---GGYTYYADS VKG | RFTISRDNSKNTLYLQMNSL RAEDTAVYFCAK | RNTDD---------------------AFDI | WGQGT MVTVS S |
| | Germline | | | H_FR1 | H_CDR1 | H_FR2 | H_CDR2 | H_FR3 | H_CDR3 | H_FR4 |
| | VH3\|3-11/D7\|7-27\|RF3/JH4 | | 32393 | QVQLVES-GGGLVKPGGSLRLSCAASG-FTFS | D-----YYMS | WIRQAPGK GLEWVS | YISSS---GSTIYYADS VKG | RFTISRDNAKNSLYLQMNSL RAEDTAVYYCAR | NWGY------------------------FDY | WGQGT LVTVS S |
| iPS:4 35903 | 21-225_190E2 | VH3\|3-11/D7\|7-27\|RF3/JH4 | | QVQLVES-GGGLVKPGGSLRLSCAASG-FTFS | D-----YYMS | WIRQAPGK GLEWLS | YISSS---GTTVFYADS VKG | RFTISRDNAKNSLYLQMNSL RAEDTAVYYCAR | EWVG-------------------------ADY | WGQGT LVTVS S |
| iPS:4 35923 | 21-225_190H6 | VH3\|3-11/D7\|7-27\|RF3/JH4 | | QVQLVES-GGGLVKPGGSLRLSCAASG-FTFS | D-----YYMS | WIRQAPGK GLEWLS | YISSS---GTTVFYADS VKG | RFTISRDNAKNSLYLQMNSL RAEDTAVYYCAR | EWVG-------------------------ADY | WGQGT LVTVS S |
| iPS:4 35953 | 21-225_191B12 | VH3\|3-11/D7\|7-27\|RF3/JH4 | | QVQLVES-GGGLVKPGGSLRLSCAASG-FTFS | D-----YYMS | WIRQAPGK GLEWVS | YISSS---GTTVFYADS VKG | RFTISRDNAKNSLYLQMNSL RAEDTAVYYCAR | EWVG-------------------------ADY | WGQGT LVTVS S |
| iPS:4 36098 | 21-225_195G11 | VH3\|3-11/D7\|7-27\|RF3/JH4 | | QVQLVES-GGGLVKPGGSLRLSCAASG-FTFS | D-----YYMS | WIRQAPGK GLEWLS | YISSS---GTTVFYADS VKG | RFTISRDNAKNSLYLQMNSL RAEDTAVYYCAR | EWVG-------------------------ADY | WGQGT LVTVS S |
| iPS:4 36102 | 21-225_196B1 | VH3\|3-11/D7\|7-27\|RF3/JH4 | | QVQLVES-GGGLVKPGGSLRLSCAASG-FTFS | D-----YYMS | WIRQAPGK GLEWIS | YISSS---GITMYYADS VKG | RFTISRDNAKNSLYLQMNSL RAEDTAVYYCAR | EWVG-------------------------ADY | WGQGT LVTVS S |
| iPS:4 36104 | 21-225_196C1 | VH3\|3-11/D7\|7-27\|RF3/JH4 | | QVQLVES-GGGLVKPGGSLRLSCAASG-FTFS | D-----YYMS | WIRQAPGK GLEWLS | YISSS---GTTVFYADS VKG | RFTISRDNAKNSLYLQMNSL RAEDTAVYYCAR | EWVG-------------------------ADY | WGQGT LVTVS S |
| | Germline | | | H_FR1 | H_CDR1 | H_FR2 | H_CDR2 | H_FR3 | H_CDR3 | H_FR4 |
| | VH3\|3-23/D6\|6-19\|RF2/JH4 | | 32394 | EVQLLES-GGGLVQPGGSLRLSCAASG-FTFS | S-----YAMS | WVRQAPGK GLEWVS | AISGS---GGSTYYADS VKG | RFTISRDNSKNTLYLQMNSL RAEDTAVYYCAK | GIAVA------------------------GYFDY | WGQGT LVTVS S |
| iPS:4 35965 | 21-225_192H2 | VH3\|3-23/D6\|6-19\|RF2/JH4 | | EVQLLES-GGDLIQPGGSLRLSCAASG-FTFS | S-----SAMS | WVRQAPGK GLEWVS | AISGS---GGNTFYADS VKG | RFTISRDNSKNTLYLQMNSL RAEDTAVYYCAK | LIAVVG---------------SHYFDY | WGQGT LVTVS S |
| iPS:4 36160 | 21-225_197C9 | VH3\|3-23/D6\|6-19\|RF2/JH4 | | EVQLLES-GGGLAQPGGSLRLSCAASG-FTFR | S-----YAMS | WVRQAPGK GLEWVS | VISGR---GGNTYYADS VKG | RFTISRDNSKNTLYLQMNSL RAEDTAVYYCAK | GIAVAG---------------SHYFDY | WGQGT LVTVS S |
| iPS:3 92954 | 21-225_26A10 | VH3\|3-23/D6\|6-19\|RF2/JH4 | | EVQLLES-GGGLVQPGGSLRLSCAASG-FTFS | S-----YAMS | WVRQAPGK GLEWVS | VISGS---GVNTFYADS VKG | RFTISRDNSKNTLYLLMNSL RAEDTAVYYCAK | KIAVAG---------------THYFDY | WGQGT LVTVS S |

| | | | Germline | H_FR1 | H_CDR1 | H_FR2 | H_CDR2 | H_FR3 | H_CDR3 | H_FR4 |
|---|---|---|---|---|---|---|---|---|---|---|
| | VH4\|4-30.1/D5\|5-24\|RF3/JH4 | | 32395 | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV TAADTAVYYCAR | RIGYN----------------YYFDY | WGQGT LVTVS S |
| iPS:4 35983 | 21-225_192E5 | VH4\|4-30.1/D5\|5-24\|RF3/JH4 | | QVQLQES-GPGLVKPSQILSL TCTVSG-DSIN | NG---GYYWS | WIRQHPGK GLEWIG | YIFY---- SGSTYYNPS LKS | RVTISVDTSKNQFSLKLNSV TAADTAVYFCAR | AGYNWD-----------------NGFDY | WGQGT LVTVS S |
| iPS:4 36043 | 21-225_193G9 | VH4\|4-30.1/D5\|5-24\|RF3/JH4 | | QVQLQES-GPGLVKPSQTLSL TCTVSG-DSIN | NG---GYYWS | WIRQHPGK GLEWIG | YIFY---- SGSTYYNPS LKS | RVTISVDTSKNQFSLKLNSV TAADTAVYFCAR | AGYNWD-----------------NGFDY | WGQGT LVTVS S |
| iPS:4 36084 | 21-225_195F2 | VH4\|4-30.1/D5\|5-24\|RF3/JH4 | | QVQLQES-GPGLVKPSQTLSL TCTVSG-DSIS | SG---GYYWS | WIRQHPGK GLEWIG | YSYY---- SGSTNYNPS LKS | RVTISVDMSKNQFSLKLSSV TDADTAVYYCAR | GGYNWN-----------------NGFDY | WGQGA LVTVS S |
| iPS:4 37138 | 21-225_214D8 | VH4\|4-30.1/D5\|5-24\|RF3/JH4 | | QVQLQES-GPGLVKPSQTLSL TCTVSG-GSIS | TA---FYYWS | WIRQHPGK GLEWIG | YIYF---- SGSTYYNPS LKS | RVTISVDTSKNQFSLNLSSV TAADTAVYYCAR | ARGYHY-----------------SIFDY | WGQGT LVTVS S |
| | | | Germline | H_FR1 | H_CDR1 | H_FR2 | H_CDR2 | H_FR3 | H_CDR3 | H_FR4 |
| | VH3\|3-23/D3\|3-16\|RF1/JH3 | | 32396 & 32397 | EVQLLES-GGGLVQPGGSLRL SCAASG-FTFS | S------YAMS | WVRQAPGK GLEWVS | AISGS--- GGSTYYADS VKG | RFTISRDNSKNTLYLQMNSL RAEDTAVYYCAK | VL*LRLGEL----------SLYDAFDI | WGQGT MVTVS S |
| iPS:4 36003 | 21-225_192G10 | VH3\|3-23/D3\|3-16\|RF1/JH3 | | EVQLLES-GGGLVQPGGSLRL SCSASG-FTFS | S-----YAMS | WVRQAPGK GLEWVS | AISGR--- GGSTFYADS VKG | RFTISRDNSKNTLYLQMNSL RAEDTAVYYCAR | RLALDG----------------YDAFDI | WGQGT MVTVS S |
| | | | Germline | H_FR1 | H_CDR1 | H_FR2 | H_CDR2 | H_FR3 | H_CDR3 | H_FR4 |
| | VH3\|3-23/D7\|7-27\|RF2/JH4 | | 32398 & 32399 | EVQLLES-GGGLVQPGGSLRL SCAASG-FTFS | S------YAMS | WVRQAPGK GLEWVS | AISGS--- GGSTYYADS VKG | RFTISRDNSKNTLYLQMNSL RAEDTAVYYCAK | *LGY----------------FDY | WGQGT LVTVS S |
| iPS:4 36019 | 21-225_193C4 | VH3\|3-23/D7\|7-27\|RF2/JH4 | | EVQLLES-GGGLAQPGGSLRL SCAASG-FTFS | S-----YAMN | WVRQAPGK GLEWVS | AIIGN--- GGRTYYADS VKG | RFSISRDNSKNTLFLQMNSL RAEDTAVYYCAK | DLGRYS----------------YGFFDY | WGQGT LVTVS S |
| | | | Germline | H_FR1 | H_CDR1 | H_FR2 | H_CDR2 | H_FR3 | H_CDR3 | H_FR4 |
| | VH4\|4-30.1/D1\|1-1\|RF1/JH6 | | 32400 | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV TAADTAVYYCAR | GTTGTYY-------------YYYGMDV | WGQGT TVTVS S |
| iPS:4 36025 | 21-225_193B5 | VH4\|4-30.1/D1\|1-1\|RF1/JH6 | | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV AAADTAVYYCAR | GEYNWN-----------------HGMDV | WGQGT TVTVS S |
| | | | Germline | H_FR1 | H_CDR1 | H_FR2 | H_CDR2 | H_FR3 | H_CDR3 | H_FR4 |
| | VH4\|4-34/D4\|4-11\|RF2/JH4 | | 32401 | QVQLQQW-GAGLLKPSETLSL TCAVYG-GSFS | G------YYWS | WIRQPPGK GLEWIG | EINH---- SGSTNYNPS LKS | RVTISVDTSKNQFSLKLSSV TAADTAVYYCAR | DYSNY-----------------YFDY | WGQGT LVTVS S |

| iPS:4 36033 | 21-225_193E7 | VH4\|4-34/D4\|4-11\|RF2/JH4 | | QVQLQQW-GAGLLKPSETLSLTCAVYG-GSFS | G-----YFWT | WIRQPPGKGLEWIG | EINH----SGSTNYNPSLKS | RVTISVDISKNQFSLKLSSVTAADTAVYYCAR | DYG----------------------ADY | WGQGTLVAVSA |
|---|---|---|---|---|---|---|---|---|---|---|
| iPS:4 36199 | 21-225_199E3 | VH4\|4-34/D4\|4-11\|RF2/JH4 | | QVQLQQW-GAGLLKPSETLSLTCAVYG-GSFS | G-----YFWT | WIRQPPGKGLEWIG | EINH----SGSTNYNPSLKS | RVTISVDISKNQFSLKLSSVTAADTAVYYCAR | DYG----------------------ADY | WGQGTLVAVSA |
| iPS:4 36228 | 21-225_200F12 | VH4\|4-34/D4\|4-11\|RF2/JH4 | | QVQLQQW-GAGLLKPSETLSLTCAVYG-GSFS | G-----YFWT | WIRQPPGKGLEWIG | EISH----SGSTNYNPSLKS | RVTISVDISKNQFSLKVSSVTAADTAVYYCAR | DYG----------------------ADY | WGQGTLVTVSS |
| iPS:4 36230 | 21-225_201A1 | VH4\|4-34/D4\|4-11\|RF2/JH4 | | QVQLQQW-GAGLLKPSETLSLTCAVTG-GSFS | G-----YFWT | WIRQPPGKGLEWIG | EISH----SGRTNYNPSLKS | RVTISGDTSKNQFSLKLSSVTAADTAVYYCAR | DYG----------------------ADY | WGQGTLVTVSS |
| iPS:4 36242 | 21-225_201A10 | VH4\|4-34/D4\|4-11\|RF2/JH4 | | QVQLQQW-GAGLLKPSETLSLTCAVYG-GSFS | G-----YFWT | WIRQPPGKGLEWIG | EISH----SGSTNYNPSLKS | RVTISVDTSKNQFSLKVNSVTAADTAVYYCAR | DYG----------------------ADY | WGQGTLVTVSS |
| iPS:4 36286 | 21-225_204H8 | VH4\|4-34/D4\|4-11\|RF2/JH4 | | QVQLQQW-GAGLLKPSETLFLTCAVYG-GSFS | G-----YFWT | WVRQPPGKGLEWIG | EISH----SGSTSYNPSLKS | RVTISVDKSKNQFSLKLSSVTAADTAVYYCAR | DYG----------------------ADY | WGQGTLVTVSS |
| iPS:4 36308 | 21-225_205H8 | VH4\|4-34/D4\|4-11\|RF2/JH4 | | QVQLQQW-GAGLLKPSETLSLTCAVYG-GSFS | G-----YFWS | WIRQPPGKGLEWIG | EISH----SGRTNYNPSLKS | RVTISVDTSKNQFSLKVSSVTAADTAVYYCAR | DYG----------------------ADY | WGQGTLVTVSS |
| | Germline | | | H_FR1 | H_CDR1 | H_FR2 | H_CDR2 | H_FR3 | H_CDR3 | H_FR4 |
| | VH3\|3-30.3/D1\|1-1\|RF1/JH4 | | 32402 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | GTTGT-------------YFDY | WGQGTLVTVSS |
| iPS:4 36051 | 21-225_193G12 | VH3\|3-30.3/D1\|1-1\|RF1/JH4 | | QVQLVES-GGGVVQPGRSLRLSCGASG-FTFS | S-----YAMH | WVRQAPGKGLEWVA | VIWYD---GTNKYYGDSVKG | RFTISRDNSKNTLNLQMNSLRAEDTAVYYCAR | DFTITG-----------------ATYFDY | WGQGTLVTVSS |
| | Germline | | | H_FR1 | H_CDR1 | H_FR2 | H_CDR2 | H_FR3 | H_CDR3 | H_FR4 |
| | VH3\|3-33/D7\|7-27\|RF3/JH6 | | 32403 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | NWGYYY-----------------YYGMDV | WGQGTTVTVSS |
| iPS:4 36054 | 21-225_194C1 | VH3\|3-33/D7\|7-27\|RF3/JH6 | | QVQLVES-GGGVVQPGRSLRLSCAASG-FTFS | S-----YGMH | WVRQAPGKGLEWVA | VIWYD---GSNEHYADSVKG | RFTISRDNSKNTLYLQMNSLRAEDTAVYYCAR | NRGVGY-----------------YGLDV | WGQGTTVTVSS |
| | Germline | | | H_FR1 | H_CDR1 | H_FR2 | H_CDR2 | H_FR3 | H_CDR3 | H_FR4 |
| | VH1\|1-02/D5\|5-18\|RF3/JH1 | | 32404 | QVQLVQS-GAEVKKPGASVKVSCKASG-YTFT | G-----YYMH | WVRQAPGQGLEWMG | WINPN---SGGINYAQKFQG | RVTMTRDTSISTAYMELSRLRSDDTAVYYCAR | GYSYGY--------------AEYFQH | WGQGTLVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 36058 | 21-225_194A4 | VH1\|1-02/D5\|5-18\|RF3/JH1 | | QVQLVQS-GTEVKKPGASLKV SCKASG-YTFT | V-----YYLN | WVRQAPGQ GLEWMG | WINPN--- SGGTNYAQK FQG | RVTMTRDISISTAYMELSRL RSDDTAVYYCAR | GYDI-----------------------LTG | WGQGT LVTVS S |
| | VH1\|1-02/D1\|1-26\|RF3/JH6 | | 32405 | QVQLVQS-GAEVKKPGASVKV SCKASG-YTFT | G-----YYMH | WVRQAPGQ GLEWMG | WINPN--- SGGTNYAQK FQG | RVTMTRDISISTAYMELSRL RSDDTAVYYCAR | YSGSYYYY-------------YYYGMDV | WGQGT TVTVS S |
| iPS:4 36068 | 21-225_194F7 | VH1\|1-02/D1\|1-26\|RF3/JH6 | | QVQLVQS-GAEVKKPGASVKV SCKASG-YTFT | D-----YYIH | WVRQAPGQ GLEWMG | WINPN--- NGGTNYAQK FQG | RVTMTRDISISTAYMELSRL RSDDTAVYYCAR | EPLGYYGSG------------SYGAYGMDV | WGQGT TVTVS S |
| | VH4\|4-34/D4\|4-17\|RF2/JH3 | | 32406 | QVQLQQW-GAGLLKPSETLSL TCAVYG-GSFS | G-----YYWS | WIRQPPGK GLEWIG | EINH---- SGSTNYNPS LKS | RVTISVDTSKNQFSLKLSSV TAADTAVYYCAR | DYGDY----------DAFDI | WGQGT MVTVS S |
| iPS:4 36072 | 21-225_194C10 | VH4\|4-34/D4\|4-17\|RF2/JH3 | | QVQLQQW-GAGLLKPSETLSL TCAVSG-GSFR | Y-----YYWS | WIRQPPGK GLEWFG | EINH---- SGSTNYNPS LKS | RVTISIDTSKNQFSLKLRSV TAADTAVYYCAR | DYGA-----------------FDI | WGQGT MVTVS S |
| iPS:4 36506 | 21-225_222C7 | VH4\|4-34/D4\|4-17\|RF2/JH3 | | QVQLQQW-GAGLLKPSETLSL TCAVYG-GSFS | G-----RYWS | WIRQPPGK GLEWIG | EINH---- SGSANYNPS LKS | RVTISVDTSKNQFSLKLSSV TAADTAVYYCAR | DYGA-----------------LDF | WGQGT MVTVS S |
| | VH3\|3-33/D5\|5-24\|RF2/JH6 | | 32407 & 32408 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | *RWLQLYY------------YYYGMDV | WGQGT TVTVS S |
| iPS:4 36092 | 21-225_195B9 | VH3\|3-33/D5\|5-24\|RF2/JH6 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | EWLQFRY------------YYGMDV | WGQGT TVTVS S |
| iPS:4 36164 | 21-225_197G10 | VH3\|3-33/D5\|5-24\|RF2/JH6 | | QVQLVES-GGGVVQPGRSLRL SCVASG-FTFS | S-----YGMH | WVRQAPGK GLEWVA | VTWYD--- GSNKYYADS VKG | RFTISRDNSKNTLYLQMNSL RAEDTAVYYCAR | EWLQFRY------------YYGIDV | WGQGT TVTVS S |
| iPS:4 36191 | 21-225_198B9 | VH3\|3-33/D5\|5-24\|RF2/JH6 | | QVQLVES-GGGVVQPGRSLRL SCAASG-FTFS | N-----YGMH | WVRQAPGK GLEWVA | IIWFD--- GSNKYYVDS VKG | RFTISRDNSKNTLFLQMSSL RAEDTAVYYCAR | EWLQFRY------------YYGMDV | WGQGT TVTVS S |
| iPS:4 36205 | 21-225_199A7 | VH3\|3-33/D5\|5-24\|RF2/JH6 | | QVQLVES-GGGVVQPGRSLRL SCAASG-FTFS | N-----YGMH | WVRQAPGK GLEWVA | IIWFD--- GSNKYYADS VKG | RFTISRDNSKNTLFLQMSSL RAEDTAVYYCAR | EWLQFRY------------YYGMDV | WGQGT TVTVS S |
| iPS:4 36214 | 21-225_200F6 | VH3\|3-33/D5\|5-24\|RF2/JH6 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCTR | EWLQFRY------------YYGMDV | WGQGT TVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3|3-33/D6|6-19|RF3/JH6 | | 32409 & 32410 | QVQLVES-GGGVVQPGRSLRL SCAASG-FTFS | S------YGMH | WVRQAPGK GLEWVA | VIWYD--- GSNKYYADS VKG | RFTISRDNSKNTLYLQMNSL RAEDTAVYYCAR | V*QWLVYY--------- --------YYYGMDV | WGQGT TVTVS S |
| iPS:4 36106 | 21-225_196F2 | VH3|3-33/D6|6-19|RF3/J H6 | | QVQLVES-GGGVVQPGRSLRL SCAASG-FTFS | S------FGMH | WVRQAPGK GLEWVA | VILND--- GSNKKCADS VKG | RCTISRDNSKNTLYLQMNSL RAEDTAVYYCAR | GQQWLV---------- ---------NGVDV | WGQGT TVTVS S |
| | VH3|3-23/D6|6-6|RF3/JH6 | | 32411 & 32412 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | V*QLVYY--------- --------YYYGMDV | WGQGT TVTVS S |
| iPS:4 36110 | 21-225_196F4 | VH3|3-23/D6|6-6|RF3/JH 6 | | EVQLLES-GGGLVQPGGSLRF SCAASG-FTFS | S-----CAMT | WVRQAPGK GLEWVS | AISGS--- GGSTYYADS VKG | RFTISRDNSKNTLYLQMNSL RAEDTAVYYCAK | VGGLTGSY--------- -------YYYGMDV | WGQGT TVTVS S |
| | VH1|1-08/D2|2-21|RF1/JH5 | | 32413 & 32414 | QVQLVQS-GAEVKKPGASVKV SCKASG-YTFT | S-----YDIN | WVRQATGQ GLEWMG | WMNPN--- SGNTGYAQK FQG | RVTMIRNISISTAYMELSSL RSEDTAVYYCAR | SILWW*L---------- -------LFNWFDP | WGQGT LVTVS S |
| iPS:4 36114 | 21-225_196G8 | VH1|1-08/D2|2-21|RF1/J H5 | | QVQLVQS-GAEVKKPGASVKV SCKASG-YTFT | N-----YDIN | WMRQATGQ GLEWMG | WMHLN--- SGNTGYAPK FQG | RVTMTRDTSISTAFMELSSL RSEDTAVYYCAY | SGGWY----------- ----------VFDP | WGQGT LVTVS S |
| iPS:4 36218 | 21-225_200G7 | VH1|1-08/D2|2-21|RF1/J H5 | | QVQLVQS-GAEVKKPGASVKV SCKASG-YTFT | N-----YDIN | WMRQATGQ GLEWMG | WMHLN--- SGNTGYAPK FQG | RVTMTRDTSISTAFMELSSL RSEDTAVYYCAY | SGGWY----------- ----------VFDP | WGQGT LVTVS S |
| | VH1|1-02/D3|3-10|RF3/JH6 | | 32415 | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | IITMVRGVII------- ------YYYYYGMDV | WGQGT TVTVS S |
| iPS:4 36116 | 21-225_196B9 | VH1|1-02/D3|3-10|RF3/J H6 | | QVQLVQS-GAEVKKPGASMKV SCKASG-YTFT | G-----YYMH | WVRQAPGQ GLEWMG | WINPN--- SGGTNFAQK FRG | RVTMTRDTSISTAYMELSRL SSDDTAVYYCAR | GGVRGVPN-------- -------YYYVMDV | WGQGT TVTVS S |
| | VH4|4-30.1/D5|5-18|RF3/JH6 | | 32416 | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV TAADTAVYYCAR | GYSYGYYY--------- --------YYYGMDV | WGQGT TVTVS S |
| iPS:4 36181 | 21-225_198C2 | VH4|4-30.1/D5|5-18|RF3/J H6 | | QVQLQES-GPGLVKPSQTLSL TCTVSG-GSIS | SG---GYYWS | WIRQHPGK GLEWIG | NIYY---- SGSTYYNPS LKS | RVTISVDTSKNQFSLKLSSV TAADTAVYYCAR | GDYYGSGSY------- ------HNYYGLDV | WGQGT TVTVS S |
| iPS:4 36210 | 21-225_199G11 | VH4|4-30.1/D5|5-18|RF3/J H6 | | QVQLQES-GPGLVKPSQTLSL TCTVSG-GSIS | SG---GYYWS | WIRQHPGK GLEWIG | NIYY---- SGSTYYNPS LKS | RVTISVDTSKNQFSLKLSSV TAADTAVYYCAR | GDYYGSGSY------- ------HNYYGLDV | WGQGT TVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH1\|1-18/D3\|3-3\|RF2/JH6 | 32417 | QVQLVQS-GAEVKKPGASVKVSCKASG-YTFT | S------YGIS | WVRQAPGQGLEWMG | WISAY---NGNTNYAQKLQG | RVTMTTDISTSTAYMELRSLRSDDTAVYYCAR | YYDFWSGYYT----------YYYYYGMDV | WGQGTTVTVSS |
| iPS:436234 | 21-225_51E3 | VH1\|1-18/D3\|3-3\|RF2/JH6 | QVQLVQS-GAEVKKPGASVKVSCKASG-YTFN | S------YGIS | WVRLAPGQGLEWMG | WISAY---NGNTKNAQRFQG | RVTMTTDISTSTAYMELRSLRSDDTAVYYCAR | HDFWSGY----------------YKGMDV | WGQGTTVTVSS |
| iPS:436830 | 21-225_51F4 | VH1\|1-18/D3\|3-3\|RF2/JH6 | QVQLVQS-GAEVKKPGASVKVSCKASG-YTFN | S------YGIS | WVRQAPGQGLEWMG | WISAY---NGNTKYAQKLQG | RVTMTTDISTSTAYMELRSLRSDDTAVYYCAR | HDFWSGY----------------YKGMDV | WGQGTTVTVSS |
| iPS:436834 | 21-225_52F1 | VH1\|1-18/D3\|3-3\|RF2/JH6 | QVQLVQS-GAEVKKPGASVKVSCKASG-YTFN | S------YGVS | WVRQAPGQGLEWMG | WISAY---NGNRKYAQKLQG | RVSMTTDISTSTAYMELRSLRSDDTAVYYCAR | HDFWSGY----------------YKGMDV | WGQGTTVTVSS |
| iPS:436842 | 21-225_54E9 | VH1\|1-18/D3\|3-3\|RF2/JH6 | QVQLVQS-GAEVKKPGASVKVSCKASG-YTFN | S------YGIS | WVRLAPGQGLEWMG | WISAY---NGNTKNAQKLQG | RVTMTTDISTSTAYMELRSLRSDDTAVYYCAR | HDFWSGY----------------YKGMDV | WGQGTTVTVSS |
| iPS:436844 | 21-225_56G1 | VH1\|1-18/D3\|3-3\|RF2/JH6 | QVQLVQS-GAEVKKPGASVKVSCKASG-YTFN | S------YGIS | WVRQAPGQGLEWMG | WISAY---NGNTKYAQKFQG | RVTMTTDISTSTAYMELRSLRSDDTAVYYCAR | HDFWSGY----------------YKGMDV | WGQGTTVTVSS |
| iPS:436846 | 21-225_56E3 | VH1\|1-18/D3\|3-3\|RF2/JH6 | QVQLVQS-GAEVKKPGASVKVSCKASG-YTFN | S------YGFS | WVRQAPGQGLEWMG | WISAY---NGNTKEAQKFQG | RVTMTTDISTSTAYMELRSLRADDTAVYYCAR | HDFWSGY----------------YKGMDV | WGQGTTVTVSS |
| iPS:451104 | 21-225_49C5 | VH1\|1-18/D3\|3-3\|RF2/JH6 | QVQLVQS-GAEVKKPGASVKVSCKASG-YTFN | S------YGIS | WVRQAPGQGLEWMG | WISAY---NGNTKYAQKLQG | RVTMTTDISTSTAYMELRSLRSDDTAVYYCAR | HDFWSGY----------------YKGMDV | WGQGTTVTVSS |
| iPS:451106 | 21-225_49D10 | VH1\|1-18/D3\|3-3\|RF2/JH6 | QVQLVQS-GAEVKKPGASVKVSCKASG-YTFN | S------YGIS | WVRLAPGQGFEWMG | WISAY---NGNTKNAQKLQG | RVTMTTDISTSTAYMELRSLRSDDTAVYYCAR | HDFWSGY----------------YKGMDV | WGQGTTVTVSS |
| iPS:451108 | 21-225_53E8 | VH1\|1-18/D3\|3-3\|RF2/JH6 | QVQLVQS-GAEVKKPGASVKVSCKASG-YTFN | S------YGIS | WVRQAPGQGLEWMG | WISAY---NGNTKFAQKLQG | RVTMTTDISTSTAYMELRSLRSDDTAVYYCAR | HDFWSGY----------------YKGMDV | WGQGTTVTVSS |
| | | Germline | H_FR1 | H_CDR1 | H_FR2 | H_CDR2 | H_FR3 | H_CDR3 | H_FR4 |
| | VH6\|6-01/D3\|3-9\|RF1/JH6 | 32418 & 32419 | QVQLQQS-GPGLVKPSQTLSLTCAISG-DSVS | SN----SAAWN | WIRQSPSRGLEWLG | RTYYR--SKWYNDYAVSVKS | RITINPDTSKNQFSLQLNSVTPEDTAVYYCAR | VLRYFDWLL*----------YYYYYGMDV | WGQGTTVTVSS |
| iPS:436236 | 21-225_201F7 | VH6\|6-01/D3\|3-9\|RF1/JH6 | QVQLQQS-GPGLVKPSQTLSLTCAISG-DSVS | SN---SAAWN | WIRQSPSRGLEWLG | RTYYR--SKWYNYYEVSVRS | RITINPDTSKNQFSLQLNSVTPEDTAVYFCAR | DQRYY--------------GMDV | WGQGTTVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 36250 | 21-225_201A4 | VH6\|6-01\|D3\|3-9\|RF1/JH6 | | QVQLQQS-GPGLVKPSQTLSLTCAISG-DSVS | SN---SAAWN | WIRQSPSRGLEWLG | RTYYR--SKWYNYYEVSVKS | RITINPDTSKNQFSLQLNSVTPEDTAVYFCAR | DQRYY-------------------GMDV | WGQGTTVTVSS |
| iPS:4 36252 | 21-225_202A8 | VH6\|6-01\|D3\|3-9\|RF1/JH6 | | QVQLQQS-GPGLVKPSQTLSLTCAISG-DSVS | SN---SAAWN | WIRQSPSRGLEWLG | RTYYR--SKWYNEYAVSVRS | RITINPDTSKNQFSLQLNSVTPEDTALYYCTR | DQRYY-------------------GMDV | WGQGTPVTVSS |
| iPS:4 36278 | 21-225_201F2 | VH6\|6-01\|D3\|3-9\|RF1/JH6 | | QVQLQQS-GPGLVKPSQTLSLTCAISG-DSVS | SN---SAAWN | WIRQSPSRGLEWLG | RTYYR--SKWYNYYEVSVRS | RVTINPDTSKNQFSLQLNSVTPEDTAVYFCAR | DQRYY-------------------GMDV | WGQGTTVTVSS |
| iPS:4 36294 | 21-225_205G4 | VH6\|6-01\|D3\|3-9\|RF1/JH6 | | QVQLQQS-GPGLVKPSQTLSLTCAISG-DSVS | SN---SAAWN | WIRQSPSRGLEWLG | RTYYR--SKWYNYYEVSVRS | RITINPDTSKNQFSLQLNSVTPEDTAVYFCAR | DQRYY-------------------GMDV | WGQGTTVTVSS |
| iPS:4 36356 | 21-225_210H10 | VH6\|6-01\|D3\|3-9\|RF1/JH6 | | QVQLQQS-GPGLVKPSQTLSLTCAISG-DSVS | SN---SAAWN | WIRQSPSRGLEWLG | RTYYR--SKWYNYYPVSVRS | RITINPDTSKNQFSLLLNSVTPEDTAVYYCAR | DQRYY-------------------GMDV | WGQGTTVTVSS |
| | Germline | | | H_FR1 | H_CDR1 | H_FR2 | H_CDR2 | H_FR3 | H_CDR3 | H_FR4 |
| | VH1\|1-02\|D4\|4-23\|RF2/JH4 | | 32420 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | DYGGN----------------SYFDY | WGQGTLVTVSS |
| iPS:4 36240 | 21-225_201E8 | VH1\|1-02\|D4\|4-23\|RF2/JH4 | | QVQLVQS-GAEVKKPGASVKVSCKASG-YTFT | G-----YYMH | WVRQAPGQGLEWMG | WIDPN---SGGTNYPQKFQG | RVTMTRDTSISTAYMELSRLRSDDTAVFYCAK | DQGYNW---------------NSFDY | WGQGTLVTVSS |
| iPS:4 36314 | 21-225_206G4 | VH1\|1-02\|D4\|4-23\|RF2/JH4 | | QVQLVQS-GAEVKKPGASVKVSCKASG-YTFT | G-----YYIH | WVRQAPGQGLEWMG | WIDPN---SGGTNYAQKFQG | RITMTRDTSISTAYMELSRLRSDDTAVFYCAK | DQGYNW---------------NSFDY | WGQGTLVTVSS |
| | Germline | | | H_FR1 | H_CDR1 | H_FR2 | H_CDR2 | H_FR3 | H_CDR3 | H_FR4 |
| | VH1\|1-02\|D5\|5-18\|RF3/JH5 | | 32421 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GYSYGY---------------NWFDP | WGQGTLVTVSS |
| iPS:4 36244 | 21-225_201H10 | VH1\|1-02\|D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTYMELSRLRSDDTAVYYCAR | GYSYGY---------------NWFDP | WGQGTLVTVSS |
| iPS:4 36262 | 21-225_203E3 | VH1\|1-02\|D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTAYMELSRLRSDDTAVYYCAR | GYSYGY---------------NWFDP | WGQGTLVTVSS |
| iPS:4 36276 | 21-225_204H4 | VH1\|1-02\|D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTAYMELSRLRSDDTAVYYCAR | GYSYGY---------------NWFDP | WGQGTLVTVSS |

| iPS:4 36312 | 21-225_206A4 | VH1\|1-02/D5\|5-18\|RF3/J H5 | | QVQLVQS-GAEVKKPGASVKV SCKASG-YTFT | G-----YYIH | WVRQAPGQ GLEWMA | WINPN---SGGTNYAQK FQG | RVTMTRDTSITTAYMELSRL RSDDTAVYYCAR | GYSYGY----------------NWFDP | WGQGT LVTVS S |
|---|---|---|---|---|---|---|---|---|---|---|
| iPS:4 36316 | 21-225_206A5 | VH1\|1-02/D5\|5-18\|RF3/J H5 | | QVQLVQS-GAEVKKPGASVKV SCKASG-YTFT | G-----YYIH | WVRQAPGQ GLEWMA | WINPN---SGGTNYAQK FQG | RVTMTRDTSITTAYMELSRL RSDDTAVYYCAR | GYSYGY----------------NWFDP | WGQGT LVTVS S |
| iPS:4 36338 | 21-225_208E8 | VH1\|1-02/D5\|5-18\|RF3/J H5 | | QVQLVQS-GAEVKKPGASVKV SCKASG-YTFT | G-----YYIH | WVRQAPGQ GLEWMA | WINPN---SGGTNYAQK FQG | RVTMTRDTSITTAYMELSRL RSDDTAVYYCAR | GYSYGY----------------NWFDP | WGQGT LVTVS S |
| iPS:4 36344 | 21-225_208B11 | VH1\|1-02/D5\|5-18\|RF3/J H5 | | QVQLVQS-GAEVKKPGASVKV SCKASG-YTFT | G-----YYIH | WVRQAPGQ GLEWMA | WINPN---SGGTNYAQK FQG | RVTMTRDTSITTAYMELSRL RSDDTAVYYCAR | GYSYGY----------------NWFDP | WGQGT LVTVS S |
| iPS:4 36358 | 21-225_210D11 | VH1\|1-02/D5\|5-18\|RF3/J H5 | | QVQLVQS-GAEVKKPGASVKV SCKASG-YTFT | G-----YYIH | WVRQAPGQ GLEWMA | WINPN---SGGTNYAQK FQG | RVTMTRDTSITTAYMELSRL RSDDTAVYYCAR | GYSYGY----------------NWFDP | WGQGT LVTVS S |
| iPS:4 36408 | 21-225_214H8 | VH1\|1-02/D5\|5-18\|RF3/J H5 | | QVQLVQS-GAEVKKPGASVKV SCKASG-YTFT | G-----HYIH | WVRQAPGQ GLEWMG | WINSN---SGGTNYAQK FQG | RVTMTRDTSISTAYMELSRL RSDDTAVYYCAK | DGRYSYG----------------YDWFDP | WGQGT LVTVS S |
| iPS:4 36424 | 21-225_215H6 | VH1\|1-02/D5\|5-18\|RF3/J H5 | | QVQLVQS-GAEVKKPGASVKV SCKASG-YTFT | G-----HYIH | WVRQAPGQ GLEWMG | WINSN---SGGTNYAEK FQG | RVTMTRDTSISTAYMELSRL RSDDTAVYYCAK | DGRYSYG----------------HDWFDP | WGQGT LVTVS S |
| iPS:4 37092 | 21-225_210B12 | VH1\|1-02/D5\|5-18\|RF3/J H5 | | QVQLVQS-GAEVKKPGASVKI SCKASG-FTFT | D-----YYMN | WVRQAPGQ GLEWMG | WINPN---SGGTNYAQK FQG | RVTMTRDTSISTAYMELSRL RSDDTAVYYCAR | GYDS----------------FAP | WGQGT LVTVS S |
| iPS:4 37134 | 21-225_213A7 | VH1\|1-02/D5\|5-18\|RF3/J H5 | | QVQLVQS-GAEVKKPGASVKI SCRASG-FTFT | D-----YYMN | WVRQAPGQ GLEWMG | WINPK---NGGTNYAQK FQG | RVTMTRDTSLSRAYMELSRL RSDDTAVYYCAK | GYDS----------------FAP | WGQGT LVTVS S |
| iPS:4 37194 | 21-225_226B2 | VH1\|1-02/D5\|5-18\|RF3/J H5 | | QVQLVQS-GAEVKKPGASVKV SCKASG-YTFT | G-----YFMH | WVRQAPGQ GLEWMG | WINPN---SGDTNYAQK FQG | RVTMTRDTSLNTAYMELSRL RSDDTAIYYCAR | GTYYGSGS----------------YFNELDS | WGQGT LVTVS S |
| iPS:4 37196 | 21-225_226B7 | VH1\|1-02/D5\|5-18\|RF3/J H5 | | QVQLVQS-GAEVKKPGASVKV SCKASG-YTFT | G-----YYMH | WVRQAPGQ GLEWMG | WINPN---SGGTNYAQK FQD | RVTMTRDTSISTAHMELSRL RSDDTAVYYCAR | GYYYGSGS----------------YYNWFDS | WGQGT LVTVS S |
| iPS:4 37200 | 21-225_226A10 | VH1\|1-02/D5\|5-18\|RF3/J H5 | | QVQLVQS-GAEVKKPGASVKV SCKASG-YTFT | G-----YFMH | WVRQAPGQ GLEWMG | WINPN---SGDTNYAQK FQG | RVTMTRDTSLSTAYMELSRL RSDDTAIYYCAR | GTYYGSGS----------------YFNELDS | WGQGT LVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:3 93168 | 21-225_32B11 | VH1\|1-02\|D5\|5-18\|RF3\|JH5 | | QVQLVQS-GAEVKKPGASVKVSCKASG-YTFT | G-----YYMH | WVRQAPGQGLEWMG | WINPN---SDGTNYAQKFQG | RVTMTRDISISTAYMELNRLRSDDTAVYYCAR | GFYYGSGS----------------YYNDLDP | WGQGTLVTVSS |
| iPS:3 93178 | 21-225_34D7 | VH1\|1-02\|D5\|5-18\|RF3\|JH5 | | QVQLVQS-GAEVKKPGASVKVSCKASG-YTFT | G-----YYMH | WVRQAPGQGLEWMG | WINPN---SGGTNYAQKFQG | RVTMTRDISISTAYMELSRLRSDDTAVYYCAR | GYYYGSGS----------------YYNDLDP | WGQGTLVTVSS |
| iPS:3 98480 | 21-225_17G4 | VH1\|1-02\|D5\|5-18\|RF3\|JH5 | | QVQLVQS-GAEVKKPGASVKVSCKTSG-YTFT | D-----YYMH | WVRQAPGQGLEWMG | WINPN---SGGTNYEQKFQG | RVTMTRDISISTAYMELSRLRSDDTAVYYCAS | GYSYGY-------------------NWFDP | WGQGTLVTVSS |
| iPS:3 98486 | 21-225_19A1 | VH1\|1-02\|D5\|5-18\|RF3\|JH5 | | QVQLVQS-GAEVKKPGASVKVSCKASG-YTFT | G-----YYMH | WVRQAPGQGLEWMG | WINPN---SGGTNYAQKFQG | RVTMTRDISISTAYMELSRLRSDDTAVYYCAS | GYSYGY-------------------NWFDP | WGQGTLVTVSS |
| | **Germline** | | | **H_FR1** | **H_CDR1** | **H_FR2** | **H_CDR2** | **H_FR3** | **H_CDR3** | **H_FR4** |
| **VH1\|1-08\|D6\|6-13\|RF1\|JH6** | | | 32422 | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GYSSSWYY----------YYYYGMDV | WGQGTTVTVSS |
| iPS:4 36248 | 21-225_202A3 | VH1\|1-08\|D6\|6-13\|RF1\|JH6 | | QVQLEQS-GAEVKKPGASVKVSCKASG-YTFT | S-----YDIN | WVRQATGQGLEWLG | WMNPK---RGNTGYAQKFQG | RVTMTRNTSISTAHMELSSLRSEDTAVYYCAR | GRYSREDY----------YYYYDMDV | WGQGTTVTVSS |
| | **Germline** | | | **H_FR1** | **H_CDR1** | **H_FR2** | **H_CDR2** | **H_FR3** | **H_CDR3** | **H_FR4** |
| **VH3\|3-11\|D4\|4-17\|RF2\|JH6** | | | 32423 | QVQLVES-GGGLVKPGGSLRLSCAASG-FTFS | D-----YYMS | WIRQAPGKGLEWVS | YISSS---GSTIYYADSVKG | RFTISRDNAKNSLYLQMNSLRAEDTAVYYCAR | DYGDYYY-----------YYYGMDV | WGQGTTVTVSS |
| iPS:4 36270 | 21-225_203F10 | VH3\|3-11\|D4\|4-17\|RF2\|JH6 | | QVQLVES-GGGLVKPGGSLRLSCAASG-FTFS | D-----YYMS | WIRQAPGKGLEWVL | YISGS---GTTTYYADSVKG | RFTISRDNAKNSLYLQMNSLRAEDTAVYYCAR | DRGG-------------------LDV | WGQGTTVTVSS |
| | **Germline** | | | **H_FR1** | **H_CDR1** | **H_FR2** | **H_CDR2** | **H_FR3** | **H_CDR3** | **H_FR4** |
| **VH3\|3-23\|D1\|1-20\|RF1\|JH6** | | | 32424 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GITGTYY-----------YYYGMDV | WGQGTTVTVSS |
| iPS:4 36280 | 21-225_204D6 | VH3\|3-23\|D1\|1-20\|RF1\|JH6 | | EVQLLES-GGGLVQPGGSLRLSCAASG-FTFS | T-----YAMS | WVRQAPGKGLEWVS | AISGS---GGSTYYADSVKG | RFTISRDNSKNTLYLQMDSLRAEDTAVYYCAK | GISGTGSY----------YYYGVDV | WGQGTTVTVSS |
| | **Germline** | | | **H_FR1** | **H_CDR1** | **H_FR2** | **H_CDR2** | **H_FR3** | **H_CDR3** | **H_FR4** |
| **VH3\|3-33\|D7\|7-27\|RF2\|JH6** | | | 32425 & 32426 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LGYYY------------YYGMDV | WGQGTTVTVSS |
| iPS:4 36284 | 21-225_204G8 | VH3\|3-33\|D7\|7-27\|RF2\|JH6 | | QVQLVES-GGDVVQPGRSLRLSCAASG-FTFS | S-----YGMH | WGRQAPGKGLEWVA | VIWYD---GSNENYVASVKG | RFTIFRDNSKNTLYLQMNSLRAEDTAVYYCAR | DLGIGY--------------YGMDV | WGQGTTVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 36968 | 21-225_190B10 | VH3\|3-33\|D7\|7-27\|RF2\|JH6 | | QVQLVES-GGGVVQPGRSLRLSCVASG-FTFS | S-----YGMH | WVRQAPGKGLEWVA | VIWND---GSKKYHVDSVKG | RFTISRDNSKNTLYLQMNSLRAEDTALYYCAR | DLDKRNFPY-------------YYYYGMDV | WGQGTTVTVSS |
| iPS:4 37006 | 21-225_192G2 | VH3\|3-33\|D7\|7-27\|RF2\|JH6 | | QVQLVES-GGGVVQPGRSLRLSCAASG-FTFS | S-----YGMH | WVRQAPGKGLEWVT | VIWND---GSNKYYADSVKG | RFTISRDNSKNTLYLQMNSLRAEDTALYYCAR | DLDKRNFPY-------------YYYYGMDV | WGQGTTVTVSS |
| iPS:4 37024 | 21-225_194F11 | VH3\|3-33\|D7\|7-27\|RF2\|JH6 | | QVQLVES-GGGVVQPGRSLRLSCAASG-FTFS | S-----YGMH | WVRQAPGKGLEWVA | VIWND---GSKKYHVDSVKG | RFTISRDNSKNTLYLQMNSLRAEDTALYYCAR | DLDKRNFPY-------------YYYYGMDV | WGQGTTVTVSS |
| iPS:4 37028 | 21-225_194G12 | VH3\|3-33\|D7\|7-27\|RF2\|JH6 | | QVQLVES-GGGVVQPGRSLRLSCAASG-FTFS | S-----YGMH | WVRQAPGKGLEWVT | VIWND---GSNKYYADSVKG | RFTISRDNSKNTLYLQMNSLRAEDTALYYCAR | DLDKRNFPY-------------YYYYGMDV | WGQGTTVTVSS |
| | | Germline | | H_FR1 | H_CDR1 | H_FR2 | H_CDR2 | H_FR3 | H_CDR3 | H_FR4 |
| | VH4\|4-34\|D6\|6-19\|RF3\|JH3 | | 32427 & 32428 | QVQLQQW-GAGLLKPSETLSLTCAVYG-GSFS | G-----YYWS | WIRQPPGKGLEWIG | EINH----SGSTNYNPSLKS | RVTISVDTSKNQFSLKLSSVTAADTAVYYCAR | V*QWLV-------------------DAFDI | WGQGTMVTVSS |
| iPS:4 36290 | 21-225_205G3 | VH4\|4-34\|D6\|6-19\|RF3\|JH3 | | QVQLQQW-GAGLLKPSETLSLTCAVFG-GSFS | G-----HYWS | WIRQPPGKGLEWIG | EMYH----FGNTNYNPSLKS | RVTMSVDTSKKQFSLKLSSVTAADTAVYYCAR | VGQWL-----------------AFDI | WGQGTMVTVSS |
| | | Germline | | H_FR1 | H_CDR1 | H_FR2 | H_CDR2 | H_FR3 | H_CDR3 | H_FR4 |
| | VH3\|3-30.3\|D4\|4-17\|RF2\|JH1 | | 32429 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYGDYA-----------EYFQH | WGQGTLVTVSS |
| iPS:4 36306 | 21-225_201H4 | VH3\|3-30.3\|D4\|4-17\|RF2\|JH1 | | QVQLVES-GGGVVQPGRSLRLSCAASG-FTFS | S-----YAMH | WVRQAPGKGLEWVA | AIWYD---GSNKYNADSVKG | RFTISRDNSKNTLYMQMNSLRAEDTAVYYCAR | DVGTVG-------------ATYFDC | WGPGTLVTVSS |
| | | Germline | | H_FR1 | H_CDR1 | H_FR2 | H_CDR2 | H_FR3 | H_CDR3 | H_FR4 |
| | VH1\|1-18\|D1\|1-1\|RF1\|JH6 | | 32430 | QVQLVQS-GAEVKKPGASVKVSCKASG-YTFT | S-----YGIS | WVRQAPGQGLEWMG | WISAY---NGNTNYAQKLQG | RVTMTTDTSTSTAYMELRSLRSDDTAVYYCAR | GTTGTYY----------YYYGMDV | WGQGTTVTVSS |
| iPS:4 36362 | 21-225_210C12 | VH1\|1-18\|D1\|1-1\|RF1\|JH6 | | QVQLVQS-GAEVTKPGASVKVSCKASG-YTFT | N-----NGIS | WVRQAPGQGLEWMG | WINAY---NGHTNYAQKFQG | RVTMTTDTSTSTAYMELRSLRSDDTAVYYCAR | DPTVTHY----------YYYGMDV | WGQGTTVTVSS |
| iPS:4 36374 | 21-225_211C10 | VH1\|1-18\|D1\|1-1\|RF1\|JH6 | | QVQLVQS-GAEVKKPGASVKVSCKASG-YTFT | R-----HGIS | WVRLAPGQGLEWMG | WISAY---NGLTNYAQKFQG | RVTMTTDTSTSTGYMELRSLRSDDTAVYYCAR | DPTVTHY----------YYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33\|D5\|5-18\|RF3\|JH6 | | 32431 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GYSYGYYY----------YYYGMDV | WGQGTTVTVSS |

| iPS ID | Clone | Germline | H FR1 | H CDR1 | H FR2 | H CDR2 | H FR3 | H CDR3 | H FR4 |
|---|---|---|---|---|---|---|---|---|---|
| iPS:4 36366 | 21-225_211A3 | VH3\|3-33/D5\|5-18\|RF3/JH6 | QVQLVES-GGGVVQPGRSLRLSCAVSG-ITFS | S-----YGMH | WARQAPGK GLEWVA | VIWYD---GSNKNYEDS VKG | RFTISRDNSKNTLYLQMNSL RAEDTAVYYCAR | DGSYGY-------------------DGMDV | WGQGT TVTVS S |
| iPS:4 36388 | 21-225_212H11 | VH3\|3-33/D5\|5-18\|RF3/JH6 | QVQLVES-GGGVVQPGRSLRLSCAVSG-ITFS | S-----YGMH | WARQAPGK GLEWVA | VIWYD---GSNKNYEDS VKG | RFTISRDNSKNTLYLQMNSL RAEDTAVYYCAR | DCSYGY-------------------DGMDV | WGQGT TVTVS S |
| iPS:4 36396 | 21-225_213E5 | VH3\|3-33/D5\|5-18\|RF3/JH6 | QVQLVES-GGGVVQPGRSLRLSCAVSG-ITFS | S-----YGMH | WARQAPGK GLEWVA | VIWYD---GSNKNYEDS VKG | RFTISRDNSKNTLYLQMNSL RAEDTAVYYCAR | DGSYGY-------------------DGMDV | WGQGT TVTVS S |
| iPS:4 36454 | 21-225_217B10 | VH3\|3-33/D5\|5-18\|RF3/JH6 | QVQLVES-GGGVVQPGRSLRLSCAVSG-ITFS | S-----YGMH | WARQAPGK GLEWVA | VIWYD---GSNKNYEDS VKG | RFTISRDNSKNTLYLQMNSL RAEDTAVYYCAR | DCSYGY-------------------DGMDV | WGQGT TVTVS S |
| iPS:4 36668 | 21-225_147B9 | VH3\|3-33/D5\|5-18\|RF3/JH6 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAR | DRDYGDPPY-------------YYYYGMDV | WGQGT TVTVS S |
| iPS:4 36688 | 21-225_148C8 | VH3\|3-33/D5\|5-18\|RF3/JH6 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAR | DRDYGDPPY-------------YYYYGMDV | WGQGT TVTVS S |
| iPS:4 36706 | 21-225_149A11 | VH3\|3-33/D5\|5-18\|RF3/JH6 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAR | DRDYGDPPY-------------YYYYGMDV | WGQGT TVTVS S |
| iPS:4 36760 | 21-225_155E10 | VH3\|3-33/D5\|5-18\|RF3/JH6 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAR | DRDYGDPPY-------------YYYYGMDV | WGQGT TVTVS S |
| iPS:4 36966 | 21-225_190C3 | VH3\|3-33/D5\|5-18\|RF3/JH6 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAN | WYYYY-----------------YGMDV | WGQGT TVTVS S |
| iPS:4 36976 | 21-225_190D8 | VH3\|3-33/D5\|5-18\|RF3/JH6 | QVQLVES-GGGDVVQPGRSLRLSCEASG-FTFS | S-----YGLH | WVRQAPGK GLEWVA | VIWYD---GSNKYYADS VKG | RFTISRDNSKNTLYLQMNSL RAEDTAVYYCAN | WYYYY-----------------YGMDV | WGQGT TVTVS S |
| iPS:4 37168 | 21-225_218G4 | VH3\|3-33/D5\|5-18\|RF3/JH6 | QVQLVES-GGGVVQPGRSLRLSCAASG-FTFS | S-----YGLH | WVRQAPGK GLEWVA | VIWYD---GSNKYYADS VKG | RFTISRDNSKNTLYLQMNSL RVEDTAVYYCAN | WYYYY-----------------YGMDV | WGQGT TVTVS S |
| | Germline | | H FR1 | H CDR1 | H FR2 | H CDR2 | H FR3 | H CDR3 | H FR4 |
| | VH3\|3-33/D5\|5-24\|RF3/JH5 | 32432 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAR | RDGYNY-----------------NWFDP | WGQGT LVTVS S |

| iPS | Clone | Germline | No. | H_FR1 | H_CDR1 | H_FR2 | H_CDR2 | H_FR3 | H_CDR3 | H_FR4 |
|---|---|---|---|---|---|---|---|---|---|---|
| iPS:4 36368 | 21-225_211G3 | VH3\|3-33\|D5\|5-24\|RF3\|JH5 | | QVQLVES-GGGVVQPGRSLRLSCAASG-FTFS | N-----YGMH | WVRQAPGK GLEWVA | VIWHD---GSNKYYADS VKG | RFTISRDNSKNTLYLQMNSL RAEDTAVYYCAR | LDYSNY-------------------GWFDP | WGQGT LVTVS S |
| iPS:4 36426 | 21-225_215C7 | VH3\|3-33\|D5\|5-24\|RF3\|JH5 | | QVQLVES-GGGVVQPGRSLRLSCAASG-FTFS | Y-----YGMH | WVRQAPGK GLEWVA | VIWHD---GSNKYYADS VKG | RFTISRDNSKNTLYLQMNSL RAEDTAVYYCAR | LDYSNY-------------------GWFDP | WGQGT LVTVS S |
| iPS:4 36432 | 21-225_215H12 | VH3\|3-33\|D5\|5-24\|RF3\|JH5 | | QVQLVES-GGGVVQPGRSLRLSCAASG-FTFS | N-----YGMH | WVRQAPGK GLEWVA | VIWHD---GSNKYYADS VKG | RFTISRDNSKNTLYLQMNSL RAEDTAVYYCAR | LDYSNY-------------------GWFDP | WGQGT LVTVS S |
| | | Germline | | H_FR1 | H_CDR1 | H_FR2 | H_CDR2 | H_FR3 | H_CDR3 | H_FR4 |
| | VH1\|1-18\|D4\|4-11\|RF2\|JH6 | | 32433 | QVQLVQS-GAEVKKPGASVKVSCKASG-YTFT | S-----YGIS | WVRQAPGQ GLEWMG | WISAY---NGNTNYAQK LQG | RVTMTTDTSTSTAYMELRSL RSDDTAVYYCAR | DYSNYYY-------------YYYGMDV | WGQGT TVTVS S |
| iPS:4 36402 | 21-225_213H12 | VH1\|1-18\|D4\|4-11\|RF2\|JH6 | | QVQLVQS-GAEVKKPGASVKVSCKASG-FTFT | S-----YGIN | WVRQAPGQ GLEWMG | WISVH---NGNTDYAQK FQG | RVTMTTDTSTSTAYMELRSL RSDDTAVYYCAR | DYYY--------------GMDV | WGQGT KVTVS S |
| iPS:4 36500 | 21-225_222H3 | VH1\|1-18\|D4\|4-11\|RF2\|JH6 | | QVQLVQS-GAEVKKPGASVKVSCKASG-FTFT | S-----YGIN | WVRQAPGQ GLEWMG | WISVY---NGNTNYAQK LQG | RVTMTTDTSTSTAYMELRSL RSDDTAVYYCAR | DYYY--------------GFDV | WGQGT TVTVS S |
| iPS:4 36520 | 21-225_223G10 | VH1\|1-18\|D4\|4-11\|RF2\|JH6 | | QVQLVQS-GAEVKKPGASVKVSCKASG-FTFT | S-----YGIN | WVRQAPGQ GLEWMG | WISVY---SGNTNYAQK LQG | RVTMTTDTSTSTAYMELRSL RSDDTAVYYCAR | DYYY--------------GMDV | WGQGT TVTVS S |
| | | Germline | | H_FR1 | H_CDR1 | H_FR2 | H_CDR2 | H_FR3 | H_CDR3 | H_FR4 |
| | VH3\|3-48\|D2\|2-21\|RF1\|JH4 | | 32434 & 32435 | EVQLVES-GGGLVQPGGSLRLSCAASG-FTFS | S-----YSMN | WVRQAPGK GLEWVS | YISSS---SSTIYYADS VKG | RFTISRDNAKNSLYLQMNSL RDEDTAVYYCAR | SILWW*L----------LFYFDY | WGQGT LVTVS S |
| iPS:4 36436 | 21-225_216F10 | VH3\|3-48\|D2\|2-21\|RF1\|JH4 | | EVQLVES-GGGLVQPGGSLRLSCAASG-FSFR | S-----YSMN | WVRQAPGK GLEWVS | YITGS---SSTIYYADS VKG | RFTISRDNAKNSLYLQMNSL RDEDTAVYYCAR | SGLA-------------VEDY | WGQGT LVTVS S |
| | | Germline | | H_FR1 | H_CDR1 | H_FR2 | H_CDR2 | H_FR3 | H_CDR3 | H_FR4 |
| | VH3\|3-48\|D6\|6-6\|RF1\|JH4 | | 32436 | EVQLVES-GGGLVQPGGSLRLSCAASG-FTFS | S-----YSMN | WVRQAPGK GLEWVS | YISSS---SSTIYYADS VKG | RFTISRDNAKNSLYLQMNSL RDEDTAVYYCAR | EYSSS--------------SYFDY | WGQGT LVTVS S |
| iPS:4 36448 | 21-225_217A3 | VH3\|3-48\|D6\|6-6\|RF1\|JH4 | | EVQLVES-GGGLVQPGGSLRLSCAASG-FTFS | S-----YNMN | WVRQAPGK GLEWVS | YISSS---RNIIYYADS VKG | RFTISRENAKNSLSLQMDSL RDEDTAVYYCAR | DGSYSSG----------WYWGFDY | WGQGT LVTVS S |
| | | Germline | | H_FR1 | H_CDR1 | H_FR2 | H_CDR2 | H_FR3 | H_CDR3 | H_FR4 |
| | VH4\|4-59\|D6\|6-19\|RF3\|JH6 | | 32437 & 32438 | QVQLQES-GPGLVKPSETLSLTCTVSG-GSIS | S-----YYWS | WIRQPPGK GLEWIG | YIYY---SGSTNYNPS LKS | RVTISVDTSKNQFSLKLSSV TAADTAVYYCAR | V*QWLVYY-------------YYYGMDV | WGQGT TVTVS S |

| ID | Clone | Germline | Num | H_FR1 | H_CDR1 | H_FR2 | H_CDR2 | H_FR3 | H_CDR3 | H_FR4 |
|---|---|---|---|---|---|---|---|---|---|---|
| iPS:4 36472 | 21-225_220E1 | VH4\|4-59/D6\|6-19\|RF3/JH6 | | QVQLQES-GPGLVKPSEILSLTCTVSG-GSIS | T-----YYWS | WIRQPPGK GLEWIG | YIYY----SGTTNYNPSLKS | RVTISVDISKNQFSLKLSSVTAADTAVYYCAR | DQQWLVRGR----------DNYYYGMDV | WGQGT TVTVS S |
| | **Germline** | | | **H_FR1** | **H_CDR1** | **H_FR2** | **H_CDR2** | **H_FR3** | **H_CDR3** | **H_FR4** |
| | VH3\|3-23/D2\|2-15\|RF3/JH4 | | 32439 | EVQLLES-GGGLVQPGGSLRLSCAASG-FTFS | S-----YAMS | WVRQAPGK GLEWVS | AISGS---GGSTYYADSVKG | RFTISRDNSKNTLYLQMNSLRAEDTAVYYCAK | DIVVVVA--------------ATYFDY | WGQGT LVTVS S |
| iPS:4 36504 | 21-225_222H4 | VH3\|3-23/D2\|2-15\|RF3/JH4 | | EVQLLES-GGGLVQPGGSLRLSCAASG-FTFS | N-----FAMS | WVRQAPGK GLEWVS | SIVGS---GGRTYYADSVKG | RFTISRDNSKNTLYLQMNSLRAEDTAVYYCAK | DPYRVAV----------AGAFDY | WGQGT LITVS S |
| iPS:4 36510 | 21-225_222H8 | VH3\|3-23/D2\|2-15\|RF3/JH4 | | EVQLLES-GGGLVQPGGSLRLSCAASG-FTFS | N-----FAMS | WVRQAPGK GLEWVS | SIVGS---GGRTYYADSVKG | RFTISRDNSKNTLYLQMNSLRAEDTAVYYCAK | DPYRVAV----------AGAFDY | WGQGT LITVS S |
| | **Germline** | | | **H_FR1** | **H_CDR1** | **H_FR2** | **H_CDR2** | **H_FR3** | **H_CDR3** | **H_FR4** |
| | VH3\|3-23/D3\|3-22\|RF2/JH1 | | 32440 | EVQLLES-GGGLVQPGGSLRLSCAASG-FTFS | S-----YAMS | WVRQAPGK GLEWVS | AISGS---GGSTYYADSVKG | RFTISRDNSKNTLYLQMNSLRAEDTAVYYCAK | YYYDSSGY----------YYAEYFQH | WGQGT LVTVS S |
| iPS:4 36526 | 21-225_224A1 | VH3\|3-23/D3\|3-22\|RF2/JH1 | | EVQVLES-GGGLVQPGGSLRLSCAASG-FTFS | S-----YAMS | WVRQAPGK GLEWVS | AISGR---GGSTYYADAVKG | RFTISRDNSKNTLYLQMNSLRAEDTAVYYCAK | GSYDSSG----------YYHYLDH | WGQGT LVTVS S |
| | **Germline** | | | **H_FR1** | **H_CDR1** | **H_FR2** | **H_CDR2** | **H_FR3** | **H_CDR3** | **H_FR4** |
| | VH1\|1-02/D4\|4-23\|RF2/JH6 | | 32441 | QVQLVQS-GAEVKKPGASVKVSCKASG-YTFT | G-----YYMH | WVRQAPGQ GLEWMG | WINPN---SGGTNYAQKFQG | RVTMTRDTSISTAYMELSRLRSDDTAVYYCAR | DYGGNSYY----------YYYGMDV | WGQGT TVTVS S |
| iPS:4 36536 | 21-225_224G1 | VH1\|1-02/D4\|4-23\|RF2/JH6 | | QVQLVQS-GAEVKKPGASVKVSCKASG-YTFT | G-----YYIH | WVRQAPGQ GLEWMG | WINPY---SGDTNYAQKFQG | RVTMTRDTSISTAYMELSRLRFDDTAVFYCAR | DWGGYSS----------YYYGMDV | WGQGT TVTVS S |
| iPS:4 36548 | 21-225_224A7 | VH1\|1-02/D4\|4-23\|RF2/JH6 | | QVQLVQS-GAEVKKPGASVKVSCKASG-YTFT | G-----YYIH | WVRQAPGQ GLEWMG | WINPY---SGDTNYAQKFQG | RVTMTRDTSITTAYMELSRLRFDDTAVFYCAR | DWGGYSS----------YYYGMDV | WGQGT TVTVS S |
| iPS:4 36558 | 21-225_224C11 | VH1\|1-02/D4\|4-23\|RF2/JH6 | | QVQLVQS-GAEVKKPGASVKVSCKASG-YTFT | G-----YYIH | WVRQAPGQ GLEWMG | WINPY---SGDTNYAQKFQG | RVTMTRDTSISTAYMELRRLRFDDTAVFYCAR | DWGGYSS----------YYFGMDV | WGQGT TVTVS S |
| iPS:4 36562 | 21-225_224H11 | VH1\|1-02/D4\|4-23\|RF2/JH6 | | QVQLVQS-GAEVKKPGASVKVSCKASG-YTFT | G-----YYIH | WVRQTPGQ GLEWMG | WINPY---SGDTNYAQKFQG | RVTMTRDTSISTAYMELRRLRFDDTAVFYCAR | DWGGYSS----------YYYGMDV | WGQGT TVTVS S |
| iPS:4 36572 | 21-225_225G4 | VH1\|1-02/D4\|4-23\|RF2/JH6 | | QVQLVQS-GAEVKKPGASVKVSCKASG-YTFT | G-----YYIH | WVRQAPGQ GLEWMG | WINPY---SGDTNYAQKFQG | RVTMTRDTSISTAYMELRRLRFDDTAVFYCAR | DWGGYSS----------YYYGMDV | WGQGT TVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 36606 | 21-225_226G8 | VH1\|1-02\|D4\|4-23\|RF2\|JH6 | | QVQLVQS-GAEVKKPGASVKVSCKASG-YTFT | G-----YYIH | WVRQAPGQGLEWMG | WINPY---SGDTKYAQKFQG | RVTMTRDISISTAYMELSRLRFDDTAVFYCAR | DWGGYSS----------------YYYGMDV | WGQGTTVTVSS |
| iPS:4 36610 | 21-225_226F9 | VH1\|1-02\|D4\|4-23\|RF2\|JH6 | | QVQLVQS-GAEVKKPGASVKVSCKASG-YTFT | G-----YYIH | WVRQAPGQGLEWMG | WINPY---SGDTKYAQKFQG | RVTMTRATSISTAYMELSRLRFDDTAVFYCAR | DWGGYSS----------------YYYGMDV | WGQGTTVTVSS |
| iPS:4 36612 | 21-225_226H9 | VH1\|1-02\|D4\|4-23\|RF2\|JH6 | | QVQLVQS-GAEVKKPGASVKVSCKASG-YTFT | G-----YYIH | WVRQAPGQGLEWMG | WINPY---SGDTNSAQKFQG | RVTMTRDISISTAYMELSRLRFDDTAVFYCAR | DWGGYSS----------------YYYGMDV | WGQGTTVTVSS |
| iPS:4 36614 | 21-225_226F10 | VH1\|1-02\|D4\|4-23\|RF2\|JH6 | | QVQLVQS-GGEVKKLRASVKVSCKASG-YTFT | G-----YYIH | WVRQAPGQGLEWMG | WINPY---SGDTNYAQKFQG | RVTMTRDISVSTAYMELSRLRLDDTAVFYCAR | DWGGYSS----------------YYYGMDV | WGQGTPVTVSS |
| iPS:4 36618 | 21-225_226E11 | VH1\|1-02\|D4\|4-23\|RF2\|JH6 | | QVQLVQS-GAEVKKPGASVKVSCKASG-YTFT | G-----YYIH | WVRQAPGQGLEWMG | WINPY---SGDTNYAQKFQG | RVTMTRDTSISTAYMELSRLRFDDTAVFYCAR | DWGGYSS----------------YYYGMDV | WGQGTTVTVSS |
| iPS:4 36624 | 21-225_226H12 | VH1\|1-02\|D4\|4-23\|RF2\|JH6 | | QVQLVQS-GAEVKKPGASVKVSCKASG-YTFT | G-----YYIH | WVRQAPGQGLEWMG | WINPY---SGDTNYAQKFQG | RVTMTRDISISTAYMELRRLRFDDTAVFYCAR | DWGGYSS----------------YYYGMDV | WGQGTTVTVSS |
| iPS:4 36626 | 21-225_227C1 | VH1\|1-02\|D4\|4-23\|RF2\|JH6 | | QVQLVQS-GAEVKKPGASVKVSCKASG-YTFT | G-----YYTH | WVRQAPGQGLEWMG | WINPY---SGGTNYAQKFQG | RVTMTRDTSISTAYMELSRLRFDDTAVYYCAR | DWGGYSS----------------YYYGMDV | WGQGTTVTVSS |
| iPS:4 36628 | 21-225_227F2 | VH1\|1-02\|D4\|4-23\|RF2\|JH6 | | QVHLVQS-GAEVKKPGASVKVSCKASG-YTFT | G-----YYIH | WVRQAPGQGLEWMG | WINPY---SGDTKYAQKFQG | RVTMTRDTSISTAYMELSRLRFDDTAVFYCAR | DWGGYSS----------------YYYGMDV | WGQGTTVTVSS |
| iPS:4 36640 | 21-225_227A8 | VH1\|1-02\|D4\|4-23\|RF2\|JH6 | | QVQLVQS-GAEVKKPGASVKVSCKASG-YTFT | G-----YYIH | WVRQAPGQGLEWMG | WINPY---SGDTNYAQKFQG | RVTMTRDTSVSTAYMELSRLRFDDTAVFYCAR | DWGGYSS----------------YYYGMDV | WGQGTTVTVSS |
| | | Germline | | H_FR1 | H_CDR1 | H_FR2 | H_CDR2 | H_FR3 | H_CDR3 | H_FR4 |
| | VH4\|4-39/D4\|4-17\|RF1/JH4 | | 32442 & 32443 & 32444 | QLQLQES-GPGLVKPSETLSLTCTVSG-GSIS | SS---SYYWC | WIRQPPGKGLEWIG | SIYY---SGSTYYNPSLKS | RVTISVDTSKNQFSLKLSSVTAADTAVYYCAR | *LR*L----------------YFDY | WGQGTLVTVSS |
| iPS:4 36538 | 21-225_224C3 | VH4\|4-39\|D4\|4-17\|RF1\|JH4 | | QLQLQES-GPGLVKSSETLSLTCTVSG-GSIS | RS---SYYWG | WIRQPPGKGLEWIG | NIYY----SGSTYYNPSLKS | RVTISVDTSKNQFSLKLSSVTAADTAVYYCAR | QGRDW--------------------GVDY | GGQGTLVTVSS |
| | | Germline | | H_FR1 | H_CDR1 | H_FR2 | H_CDR2 | H_FR3 | H_CDR3 | H_FR4 |
| | VH3\|3-23/D4\|4-11\|RF2/JH5 | | 32445 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | DYSNY----------------------NWFDP | WGQGTLVTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:4 36546 | 21-225_224D6 | VH3\|3-23/D4\|4-11\|RF2/JH5 | EVQVLES-GGGLVQPGGSLRLSCAASG-STFS | S-----DAMS | WVRQAPGKGLEWVS | AISGS---GDNTFYADSVKG | RFTISRDNSKNTLYLQMNSLRAEDTAVYYCAK | VYSAYD-------------SHWFDP | WGQGTLVTVSS |
| | Germline | | H_FR1 | H_CDR1 | H_FR2 | H_CDR2 | H_FR3 | H_CDR3 | H_FR4 |
| | VH1\|1-46/D6\|6-6\|RF2/JH6 | 32446 | QVQLVQS-GAEVKKPGASVKVSCKASG-YTFT | S-----YYMH | WVRQAPGQGLEWMG | IINPS---GGSTSYAQKFQG | RVTMTRDISTSTVYMELSSLRSEDTAVYCAR | STAARYY-----------YYGMDV | WGQGTTVTVSS |
| iPS:4 36568 | 21-225_225B3 | VH1\|1-46/D6\|6-6\|RF2/JH6 | QVQLVQS-GAEVKKPGASVKVSCKASG-YTFT | S-----YYMH | WVRQAPGQGLEWMG | IINPS---GGSTSYAQKFQG | RVTMTRDISTSTVYMELSSLRSADTAVYYCAR | DLAARSYY--------YYFGMDV | WGQGATVTVSS |
| | Germline | | H_FR1 | H_CDR1 | H_FR2 | H_CDR2 | H_FR3 | H_CDR3 | H_FR4 |
| | VH4\|4-30.1/D4\|4-17\|RF2/JH6 | 32447 | QVQLQES-GPGLVKPSQTLSLTCTVSG-GSIS | SG---GYYWS | WIRQHPGKGLEWIG | YIYY----SCSTYYNPSLKS | RVTISVDTSKNQFSLKLSSVTAADTAVYYCAR | DYCDYYY-----------YYYGMDV | WGQGTTVTVSS |
| iPS:4 36580 | 21-225_225E7 | VH4\|4-30.1/D4\|4-17\|RF2/JH6 | QVQLQES-GPGLVKPSQTLSLTCTVSG-NSIS | SG---HYYWS | WIRQHPGKGLEWIG | FIYY----TGSTYYNPSLKS | RVTISVDTSKNQFSLKLSSVTAADTAVYYCAR | EAGDYG-------------YYGMDV | WGQGTTVTVSS |
| iPS:4 36926 | 21-225_78D10 | VH4\|4-30.1/D4\|4-17\|RF2/JH6 | QVQLQES-GPGLVKPSQTLSLTCTVSG-GSIS | SG---GYYWS | WIRQHPGKGLEWIG | YIYY----IGSVYYNPSLKS | RVTISVDTSKNQFSLKLSSVTAADTAVYYCAR | DAPDF-------------GMDV | WGQGTTVTVSS |
| | Germline | | H_FR1 | H_CDR1 | H_FR2 | H_CDR2 | H_FR3 | H_CDR3 | H_FR4 |
| | VH3\|3-33/D4\|4-17\|RF2/JH1 | 32448 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYGDYA-----------EYFQH | WGQGTLVTVSS |
| iPS:4 36592 | 21-225_226B1 | VH3\|3-33/D4\|4-17\|RF2/JH1 | QVQLVES-GGGVVQPGRSLRLSCAASG-FTFS | T-----YGMH | WVRQAPGKGLEWVA | IIWYD---GGYKYYADSVKG | RFTISRDNSKNTLFLQMNSLRAEDTAVYYCAR | DHYDFW-------------SGYLTH | WGQGTLVTVSS |
| | Germline | | H_FR1 | H_CDR1 | H_FR2 | H_CDR2 | H_FR3 | H_CDR3 | H_FR4 |
| | VH3\|3-23/D7\|7-27\|RF1/JH6 | 32449 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | LTGYYY-----------YYGMDV | WGQGTTVTVSS |
| iPS:4 36652 | 21-225_146B11 | VH3\|3-23/D7\|7-27\|RF1/JH6 | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT-------------DYGMDV | WGQGTTVTVSS |
| iPS:4 36654 | 21-225_146C11 | VH3\|3-23/D7\|7-27\|RF1/JH6 | EVQLLES-GGGLVQPGGSLRLSCAASG-ITFS | S-----YAMS | WVRQAPGKGLEWVS | VISGG---GSSTYYADSVKG | RFTISRDNSKNTLYLQMNSLRAEDTAVYYCAK | WRGNPT-------------DYGMDV | WGQGTTVTVSS |
| iPS:4 36658 | 21-225_146A2 | VH3\|3-23/D7\|7-27\|RF1/JH6 | EVQLLES-GGGLVQPGGSLRLSCAASG-FTFS | S-----YAMS | WVRQAPGKGLEWVS | VISGG---GSSTYYADSVKG | RFTISRDNSKNTLHLQMNSLRAEDTAVYYCAK | WRGNPT-------------DYGMDV | WGQGTTVTVSS |

| | | | | | | | | | | |
|---|---|---|---|---|---|---|---|---|---|---|
| iPS:4 36664 | 21-225_147E7 | VH3|3-23|D7|7-27|RF1|JH6 | | EVQLLES-GGGLVQPGGSLRLSCAASG-FTFS | S-----YAMS | WVRQAPGKGLEWVS | VISGG---GSSTYYADSVKG | RFTLSRDNSKNTLYLQMNSLRAEDTAVFYCAK | WRGNPT------------------DYGMDV | WGQGTTVTVSS |
| iPS:4 36676 | 21-225_147E11 | VH3|3-23|D7|7-27|RF1|JH6 | | EVQLLES-GGGLVQPGGSLRLSCAASG-FTFS | N-----YVMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36678 | 21-225_147B12 | VH3|3-23|D7|7-27|RF1|JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36686 | 21-225_148G6 | VH3|3-23|D7|7-27|RF1|JH6 | | EVQLLES-GGGLVQPGGSLRLSCAASG-FTFS | S-----YAMS | WVRQAPGKGLEWVS | VISGG---GSSTYYADSVKG | RFTISRDNSKNMLHLQMNSLRAEDTAVYYCAK | WRGNPT------------------DYGMDV | WGQGTTVTVSS |
| iPS:4 36694 | 21-225_148G11 | VH3|3-23|D7|7-27|RF1|JH6 | | EVQLLES-GGGLVQPGGSLRLSCAASG-FTFS | S-----YPMS | WVRQAPGKGLEWVS | VISGG---GSSAYYADSVKG | RFTISRDNSKNTLYLQMNSLRAEDTAVYYCAK | WRGNPT------------------DYGMDV | WGQGTTVTVSS |
| iPS:4 36700 | 21-225_149C7 | VH3|3-23|D7|7-27|RF1|JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36704 | 21-225_149C10 | VH3|3-23|D7|7-27|RF1|JH6 | | EVQLLES-GGGLVQPGGSLRFSCAASG-FTFS | S-----HAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36710 | 21-225_150F6 | VH3|3-23|D7|7-27|RF1|JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36714 | 21-225_150H11 | VH3|3-23|D7|7-27|RF1|JH6 | | EVQLLES-GGGLVQPGGSLRLSCAASG-FTFS | T-----YAMS | WVRQAPGKGLEWVS | IISGG---GSSTYYADSVKG | RFTISRDNSKNTLYLQMNSLRAEDTAVYYCAK | WRGNPT------------------DYGMDV | WGQGTTVTVSS |
| iPS:4 36718 | 21-225_151H5 | VH3|3-23|D7|7-27|RF1|JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36722 | 21-225_151H7 | VH3|3-23|D7|7-27|RF1|JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36724 | 21-225_151B9 | VH3|3-23|D7|7-27|RF1|JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 36728 | 21-225_152G6 | VH3\|3-23/D7\|7-27\|RF1/JH6 | | EVQLLES-GGGLVQPGGSLRL SCAASG-FTFS | S-----YAMS | WVRQAPGK GLEWVS | VISGG---GSSTYYADS VKG | RFTISRDNSKNTLYLQMNSL RAEDTAVYYCAK | WRGNPI-----------------DYGMDV | WGQGT TVTVS S |
| iPS:4 36730 | 21-225_152D7 | VH3\|3-23/D7\|7-27\|RF1/JH6 | | EVQLLES-GGGLVQPGGSLRL SCAASG-FTFS | S-----YAMS | WVRQAPGK GLEWVS | VISGG---GSSTYYADS VKG | RFTISRDNSKNTLYLQMNSL RAEDTAVYYCAK | WRGNPI-----------------DSGMDV | WGQGT TVTVS S |
| iPS:4 36742 | 21-225_154C4 | VH3\|3-23/D7\|7-27\|RF1/JH6 | | EVQLLES-GGGLIQPGGSLRL SCAASG-FTFS | S-----YAMS | WVRQAPGK GLEWVS | VISGG---GSSTYYADS VKG | RFTISRDNSKNTLYLQMNSL RAEDTAVYYCAK | WRGNPI-----------------DYGMDV | WGQGT TVTVS S |
| iPS:4 36746 | 21-225_154E10 | VH3\|3-23/D7\|7-27\|RF1/JH6 | | EVQLLES-GGGLVQPGGSLRL SCAASG-FTFS | S-----YAMS | WVRQAPGK GLEWVS | VISGG---GSSTYYADS VKG | RFTISRDNSKNTLYLQMNSL RAEDTAVYYCAK | WRGNPI-----------------DYGMDV | WGQGT TVTVS S |
| iPS:4 36758 | 21-225_155C10 | VH3\|3-23/D7\|7-27\|RF1/JH6 | | EVQLLES-GGGLVQPGGSLRL SCAASG-FTFS | S-----YAMS | WVRQAPGK GLEWVS | VISGG---GSSTYYADS VKG | RFTISRDNSKNTLYLQMNSL RAEDTAVYYCAK | WRGNPI-----------------DYGMDV | WGQGT TVTVS S |
| iPS:4 36938 | 21-225_146A3 | VH3\|3-23/D7\|7-27\|RF1/JH6 | | EVQLLES-GGGLVQPGGSLRL SCAASG-FTFS | S-----YAMS | WVRQAPGK GLEWVS | VISGG---GTTTYYADS VKG | RFTISRDNSKNTLYLQMNSL RAEDTAVYYCAK | WRGNPI-----------------DYGMDV | WGQGT TVTVS S |
| iPS:4 37250 | 21-225_148C6 | VH3\|3-23/D7\|7-27\|RF1/JH6 | | EVQLLES-GGGLVQPGGSLRL SCAASG-FTFS | S-----YAMS | WVRQAPGK GLEWVS | VISGG---GSSTYYADS VKG | RFTISRDNSKNTLYLQMNSL RAEDTAVYYCAK | WRGNPI-----------------DYGMDV | WGQGT TVTVS S |
| iPS:4 37252 | 21-225_148H11 | VH3\|3-23/D7\|7-27\|RF1/JH6 | | EVQLLES-GGGLVQPGGSLRL SCAASG-FTFS | S-----YAMS | WVRQAPGK GLEWVS | VISGG---GSSTYYADS VKG | RFTISRDNSKNTLYLQMNSL RAEDTAVYYCAK | WRGNPI-----------------DYGMDV | WGQGT TVTVS S |
| iPS:4 37282 | 21-225_207C9 | VH3\|3-23/D7\|7-27\|RF1/JH6 | | EVQLLES-GGGLVQPGGSLRL SCAASG-FTFS | S-----YAMS | WVRQAPGK GLEWVS | AISGS---GGSTYYADS VKG | RFTISRDNSKNTLYLQMNSL RAEDTAVYYCAK | AGGTTGSY------------------YYNGMDV | WGQGT TVTVS S |
| | | Germline | | H_FR1 | H_CDR1 | H_FR2 | H_CDR2 | H_FR3 | H_CDR3 | H_FR4 |
| | VH3\|3-48/D4\|4-11\|RF2/JH6 | | 32450 | EVQLVES-GGGLVQPGGSLRL SCAASG-FTFS | S-------YSMN | WVRQAPGK GLEWVS | YISSS---SSTIYYADS VKG | RFTISRDNAKNSLYLQMNSL RDEDTAVYYCAR | DYSNYYY-----------YYYGMDV | WGQGT TVTVS S |
| iPS:4 36660 | 21-225_146D8 | VH3\|3-48/D4\|4-11\|RF2/JH6 | | EVQLVES-GGGLVQPGGSLRL SCAASG-FTFS | N-----YNMN | WVRQAPGK GLEWVS | YISRS---SNTKYYVDS VKG | RFTISRDNAKNSLYLQMNSL RDEDTAVYYCAR | DRSGSYGY-----------FYYYGLDV | WGQGT TVTVS S |
| iPS:4 36682 | 21-225_146A8 | VH3\|3-48/D4\|4-11\|RF2/JH6 | | EVKLVES-GGGLVQPGESLRL SCVASG-FTFS | N-----YNMN | WVRQAPGK GLEWVS | YISRS---SNTKYYADS VRG | RFTISRDNAKNSLYLQMNSL RDEDTAVYYCAR | DRSGSYGY-----------FYYYGLDV | WGQGT TVTVS S |

| iPS:4 36684 | 21-225_146B6 | VH3|3-48/D4|4-11|RF2/JH6 | | EVQLVES-GGGLVQPGGSLRLSCAASG-FTFS | S-----YNMN | WVRQAPGKGLEWVS | YISRS---SNTKHYADSVKG | RFTISRDNAKNSLYLQMDSLRDEDTAVYYCAR | DRSGSYGY--------------FYYYGLDV | WGQGTTVTVSS |
| iPS:4 36696 | 21-225_149A1 | VH3|3-48/D4|4-11|RF2/JH6 | | EVQLVES-GGGLVQPGGSLRLSCAASG-FTFS | S-----YNMN | WVRQAPGKGLEWVS | YISRS---SNTKHYADSVKG | RFTISRDNAKNSLYLQMNSLRDEDTAVYYCAR | DRSGSYGY--------------FYYYGLDV | WGQGTTVTVSS |
| iPS:4 36712 | 21-225_150F9 | VH3|3-48/D4|4-11|RF2/JH6 | | EMQLVES-GGGLVQPGGSLRLSCAASG-FTFS | S-----YNMN | WVRQVPGKGLEWVS | YISRS---SNTKHYADSVKG | RFTISRDNAKNSLYLQMNSLRDEDTAVYYCAR | DRSGSYGY--------------FYYYGLDV | WGQGTTVTVSS |
| iPS:4 36762 | 21-225_156H2 | VH3|3-48/D4|4-11|RF2/JH6 | | EVQLVES-GGGLIQPGGSLRLSCAASG-FTFS | N-----YNMN | WVRQAPCKGLEWVS | YISRS---SNTKYYADSVKG | RFTISRDNAKNSLYLQMNSLRDEDTAVYYCAR | DRSGSYGY--------------FYYYGMDV | WGQGTTVTVSS |
| iPS:4 36820 | 21-225_179D10 | VH3|3-48/D4|4-11|RF2/JH6 | | EVQLVES-GGGLVQPGGSLRFSCAASG-FTFS | S-----YSMN | WVRQAPGKGLEWVA | YISSS---GSTTYYADSVQG | RFTISRDNAKNSLYLQMNSLRDEDTAVYRCAR | DSRKGF-----------------YYGLDV | WGQGITVTVSS |
| iPS:4 37262 | 21-225_170E4 | VH3|3-48/D4|4-11|RF2/JH6 | | EVQLVES-GGGSVQPGGSLRLSCAASG-FTFS | S-----YSMN | WVRQAPGKGLEWVS | YISSS---GSTKYYADSVEG | RFTISRDNAKNSLDLQMNSLRDEDTAVYRCAR | DSRKGF-----------------YYGLDV | WGQGTTVTVSS |
| | Germline | | | H_FR1 | H_CDR1 | H_FR2 | H_CDR2 | H_FR3 | H_CDR3 | H_FR4 |
| | VH1|1-08/D5|5-12|RF1/JH6 | | 32451 | QVQLVQS-GAEVKKPGASVKVSCKASG-YTFT | S-----YDIN | WVRQATGQGLEWMG | WMNPN---SGNTGYAQKFQG | RVTMTRNISISTAYMELSSLRSEDTAVYYCAR | VDIVATIY--------------YYYYGMDV | WGQGTTVTVSS |
| iPS:4 36662 | 21-225_147D7 | VH1|1-08/D5|5-12|RF1/JH6 | | QVQLVQS-GAEVKKPGASVKVSCKASG-YTFT | S-----YDIN | WVRQATGQGLEWMG | WMNPN---SGNTGYAQKFQG | RVTMTRNISISTAYMELRSLRSEDTAVYYCAR | ADIVLVPAAI----------PYNYYFAMDV | WGQGTTVTVSS |
| | Germline | | | H_FR1 | H_CDR1 | H_FR2 | H_CDR2 | H_FR3 | H_CDR3 | H_FR4 |
| | VH1|1-02/D3|3-3|RF2/JH6 | | 32452 | QVQLVQS-GAEVKKPGASVKVSCKASG-YTFT | G-----YYMH | WVRQAPGQGLEWMG | WINPN---SCGTNYAQKFQG | RVTMTRDISISTAYMELSRLRSDDTAVYYCAR | YYDFWSGYYT----------YYYYGMDV | WGQGITVTVSS |
| iPS:4 36666 | 21-225_147B8 | VH1|1-02/D3|3-3|RF2/JH6 | | QVQLVQS-GAEVKKPGASVKVSCKASG-YTFT | D-----YYLH | WVRQAPGQGLEWMG | WINPN---SGDTNYAQKFQG | RVTMTRDISISTAYMELSRLRSDDTAVYYCAR | DRDSGSGSYP----------YYYYGMDV | WGQGTTVTVSS |
| | Germline | | | H_FR1 | H_CDR1 | H_FR2 | H_CDR2 | H_FR3 | H_CDR3 | H_FR4 |
| | VH3|3-33/D3|3-3|RF2/JH6 | | 32453 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYDFWSGYYT----------YYYYGMDV | WGQGTTVTVSS |
| iPS:4 36672 | 21-225_147F9 | VH3|3-33/D3|3-3|RF2/JH6 | | QVQLVES-GGGVVQPGRSLRLSCAASG-FTFS | T-----YGMH | WVRQAPGKGLEWVA | VIWYG---GSDKDYADSVKG | RFTISRDISKNTLYLQMNSLRAEDTAVYYCAR | DRDYCSGGTC--------PYYYYYGMDV | WGQGTTVTVSS |

| | | | | | | | | | | |
|---|---|---|---|---|---|---|---|---|---|---|
| iPS:4 36674 | 21-225_147G9 | VH3\|3-33/D3\|3-3\|RF2/JH6 | | QVQLVES-GGGVVQPGRSLRL SCAASG-FTFS | T-----YGMH | WVRQAPGK GLEWVA | VIWYG--- GSDKDYADS VKG | RFTISRDISKNTLYLQMNSL RAEDTAVYYCAR | DRDYCSGGSC------ ----PYYYYYGMDV | WGQGT TVTVS S |
| iPS:4 36690 | 21-225_148A9 | VH3\|3-33/D3\|3-3\|RF2/JH6 | | QVQLVES-GGGVVQPGRSLRL SCAASG-FTFS | T-----YGMH | WVRQAPGK GLEWVA | VIWYG--- GSDKDYADS VKG | RFTISRDISKNTLYLQMNSL RAEDTAVYYCAR | DRDYCSGGTC------ ----PYYYYYGMDV | WGQGT TVTVS S |
| iPS:4 36708 | 21-225_150D3 | VH3\|3-33/D3\|3-3\|RF2/JH6 | | QVQLVES-GGGVVQPGRSLRL SCAASG-FTFS | T-----YGMH | WVRQAPGK GLEWVA | VIWYG--- GSNKDYADS VKG | RFTISRDNSKNTLYLQMNSL RAEDTAVYYCAR | DRDYCSGGTC------ ----PYYYYYGMDV | WGQGT TVTVS S |
| iPS:4 36716 | 21-225_151F3 | VH3\|3-33/D3\|3-3\|RF2/JH6 | | QVQLVES-GGGVVQPGRSLRL SCAASG-FTFS | T-----YGMH | WVRQAPGK GLEWVA | VIWYG--- GSNTDYADS VKG | RFTISRDISKNTLYLQMNSL RAEDTAVYYCAR | DRDYCSGTSC------ ----PYYYYYGMDV | WGQGT TVTVS S |
| iPS:4 36738 | 21-225_153D9 | VH3\|3-33/D3\|3-3\|RF2/JH6 | | QVQLVES-GGGVVQPGRSLRL SCAASG-FTVS | T-----YGMH | WVRQAPGK GLEWVA | VIWYG--- GSNKDYADS VKG | RFTISRDISKNTLYLQMNSL RAEDTAVYYCAR | DRDYCSGGSC------ ----PYYYYYGMDV | WGQGT TVTVS S |
| iPS:4 36740 | 21-225_154C3 | VH3\|3-33/D3\|3-3\|RF2/JH6 | | QVQLVES-GGGVVQPGRSLRL SCTASG-FTFS | T-----YGMH | WVRQAPGK GLEWVA | VVWYG--- GNNKDYADS VKG | RFTISRDISKNTLYLQMNSL RAEDTAVYYCAR | DRDYCSGGSC------ ----PYYYYYGMDV | WGQGT TVTVS S |
| iPS:4 36748 | 21-225_154D11 | VH3\|3-33/D3\|3-3\|RF2/JH6 | | QVQLVES-GGGVVQPGRSLRL SCAASG-FNVS | T-----YGMH | WVRQAPGK GLEWVA | VIWYG--- GSNKDYADS VKG | RFTISRDSSKNTLYLQMNSL RAEDTAVYYCAR | DRDYCSGGSC------ ----PYYYYYGMDV | WGQGT TVTVS S |
| iPS:4 36764 | 21-225_158E9 | VH3\|3-33/D3\|3-3\|RF2/JH6 | | QVQLVES-GGGVVQPGRSLRL SCAASG-FTFS | S-----YGMH | WVRQAPGK GLEWVA | VIWYD--- GSSKYYADS VKG | RFTISRDNSKNTLYLQMNSL RAEDTAVYYCAR | DRVFCSGTSC------ ----PYYYYYGMDV | WGQGT TVTVS S |
| iPS:4 36774 | 21-225_161E10 | VH3\|3-33/D3\|3-3\|RF2/JH6 | | QVQLVES-GGGVVQPGRSLRL SCAASG-FTFS | S-----YGMH | WVRQAPGK GLEWVA | VIWYD--- GSNKYYVDS VKG | RFTISRDNSKNTLYLQMNSL RAEDTAVYYCAR | DRVFCSGTSC------ ----PYYYYYGMDV | WGQGT TVTVS S |
| iPS:4 36916 | 21-225_74A9 | VH3\|3-33/D3\|3-3\|RF2/JH6 | | QVQLVES-GGGVVQPGRSLRL SCAASG-FTFS | S-----YGMH | WVRQAPGK GLEWVA | VIWYD--- GNNKSYADS VKG | RFTISRDISKNTLFLQMNSL RADDTAVYYCAR | DRDYCSGTSC------ ----PYYYYYGMDV | WGQGT TVTVS S |
| iPS:4 36940 | 21-225_146B8 | VH3\|3-33/D3\|3-3\|RF2/JH6 | | QVQLVES-GGGVVQPGRSLRL SCAASG-FTFS | T-----YGMH | WVRQAPGK GLEWVA | VVWYG--- GNDKDFADS VTG | RFTISRDISKNTLYLQMNSL RAEDTAVYYCAR | DRDYCSGGSC------ ----PYYYYYGMDV | WGQGT TVTVS S |
| iPS:4 37258 | 21-225_153F9 | VH3\|3-33/D3\|3-3\|RF2/JH6 | | QVHLVES-GGGVVQPGRSLRL SCAASG-FTIS | T-----YGMH | WVRQGPGK GLEWVA | VIWYG--- GSDTDYADS VRG | RFTISRDISKNTLYLQMNSL RAEDTAVYYCAR | DRDYCSGGNC------ ----PYYYYYGMDV | WGQGT TVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-30.1/D7\|7-27\|RF3/JH5 | 32454 | QVQLQES-GPGLVKPSQILSL TCTVSG-GSIS | SG---GYYWS | WIRQHPGK GLEWIG | YIYY---- SGSTYYNPS LKS | RVTISVDISKNQFSLKLSSV TAADTAVYYCAR | NWGX--------- ----------WFDP | WGQGT LVTVS S |
| iPS:4 36680 | 21-225_147H12 | VH4\|4-30.1/D7\|7-27\|RF3/JH5 | QVQLQES-GPGLVNPSQILSL TCAVSG-GSIS | SG---YYHWS | WIRQHPGK GLEWIG | YIYY---- SGSTYYNPS LKS | RVTISVDISKNQFSLKLTSV TAADTAVYYCAR | DWGGYDS--------- --------SGWFDP | WGQGT LVTVS S |
| iPS:4 36750 | 21-225_154G12 | VH4\|4-30.1/D7\|7-27\|RF3/JH5 | QVQLQES-GPGLVNPSQTLSL TCGVSG-GSIS | SG----YYYWS | WIRQHPGK GLEWIG | YIYY---- SGSTYYNPS LKS | RVSISLDTPKNQFSLKLTSV TAADTAVYYCAR | DWGGYDS--------- --------SGWFDP | WGQGT LVTVS S |
| | | Germline | H_FR1 | H_CDR1 | H_FR2 | H_CDR2 | H_FR3 | H_CDR3 | H_FR4 |
| | VH3\|3-07/D6\|6-13\|RF1/JH4 | 32455 | EVQLVES-GGGLVQPGGSLRL SCAASG-FTFS | S------YWMS | WVRQAPGK GLEWVA | NIKQD--- GSEKYYVDS VKG | RFTISRDNAKNSLYLQMNSL RAEDTAVYYCAR | GYSSSW--------- --------YYFDY | WGQGT LVTVS S |
| iPS:4 36698 | 21-225_149B5 | VH3\|3-07/D6\|6-13\|RF1/JH4 | EVQLVES-GGGLVQPGGSLRL SCAASG-FTFS | G------YWMN | WVRQAPGK GLEWVA | NIKQD--- GSEKYYVDS VKG | RFTISRDNAKNSLYLQMNSL RAEDTAVYYCAR | GMYSSG---------- --------WYVFDY | WGQGT LVTVS S |
| | | Germline | H_FR1 | H_CDR1 | H_FR2 | H_CDR2 | H_FR3 | H_CDR3 | H_FR4 |
| | VH3\|3-21/D6\|6-19\|RF2/JH5 | 32456 | EVQLVES-GGGLVKPGGSLRL SCAASG-FTFS | S------YSMN | WVRQAPGK GLEWVS | SISSS--- SSYIYYADS VKG | RFTISRDNAKNSLYLQMNSL RAEDTAVYYCAR | GIAVAG---------- --------NWFDP | WGQGT LVTVS S |
| iPS:4 36702 | 21-225_149E8 | VH3\|3-21/D6\|6-19\|RF2/JH5 | EVQLVES-GGGLVKPGGSLRL SCAASG-FTFS | S-----YSMN | WVRRAPGK GLEWVS | AISST--- GSYIYYADS VKG | RFTISRDNAKNSLYLQMNSL RAEDTAVYYCAR | TAVAGT---------- --------GWFDP | WGQGT LVTVS S |
| | | Germline | H_FR1 | H_CDR1 | H_FR2 | H_CDR2 | H_FR3 | H_CDR3 | H_FR4 |
| | VH5\|5-51/D3\|3-22\|RF2/JH6 | 32457 | EVQLVQS-GAEVKKPGESLRI SCKGSG-YSFT | S------YWIG | WVRQMPGK GLEWMG | IIYPG--- DSDTRYSPS FQG | QVTISADKSISTAYLQWSSL KASDTAMYYCAR | YYYDSSGYYY------ -----YYYYYGMDV | WGQGT TVTVS S |
| iPS:4 36752 | 21-225_155H1 | VH5\|5-51/D3\|3-22\|RF2/JH6 | EVQLVQS-GAEVKKPGESLRI SCKGSG-YSFT | S-----YWIG | WVRQMPGK GLEWMG | LIYPG--- ASDTRYSPS FQG | QVTISADKSISTAYLQWSSL KASDTAMYYCAR | QAIASRGR-------- ------YYYYGMDV | WGQGT TVTVS S |
| | | Germline | H_FR1 | H_CDR1 | H_FR2 | H_CDR2 | H_FR3 | H_CDR3 | H_FR4 |
| | VH3\|3-33/D6\|6-19\|RF1/JH4 | 32458 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | GYSSGW--------- --------YYFDY | WGQGT LVTVS S |
| iPS:4 36772 | 21-225_161H3 | VH3\|3-33/D6\|6-19\|RF1/JH4 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | VCYSGG---------- --------WYIFDY | WGQGT LVTVS S |
| | | Germline | H_FR1 | H_CDR1 | H_FR2 | H_CDR2 | H_FR3 | H_CDR3 | H_FR4 |

| | | | H_FR1 | H_CDR1 | H_FR2 | H_CDR2 | H_FR3 | H_CDR3 | H_FR4 |
|---|---|---|---|---|---|---|---|---|---|
| | VH4\|4-30.1\|D2\|2-2\|RF2\|JH6 | 32459 | QVQLQES-GPGLVKPSQILSL TCTVSG-GSIS | SG---GYYWS | WIRQHPGK GLEWIG | YIYY---- SGSTYYNPS LKS | RVTISVDISKNQFSLKLSSV TAADTAVYYCAR | GYCSSTSCYT------ ---YYYYYGMDV | WGQGT TVTVS S |
| iPS:4 36776 | 21-225_161F12 | VH4\|4-30.1\|D2\|2-2\|RF2\|JH6 | QVQLQES-GPGLVKPSQILSL TCTVSG-GSIS | SG---GYYWS | WIRQHPGK GLEWIG | YIYY---- SGSPYYNPS LKS | RITISIDTSKNQFSLKLNSV TAADTAVYYCAR | SNCSSANCYT------ ----VGFYYYGLDV | WGRGT TVTVS S |
| iPS:4 36780 | 21-225_165H3 | VH4\|4-30.1\|D2\|2-2\|RF2\|JH6 | QVQLQES-GPGLVKPSQTLSL TCTVSG-GSIS | SG---GYYWS | WIRQHPGK GLEWIG | YIYY---- SGSPYYNPS LKS | RITISIDTSKNQFSLKLNSV TAADTAVYYCAR | SNCSSANCYT------ ----VGFYYYGMDV | WGQGT TVTVS S |
| | Germline | | **H_FR1** | **H_CDR1** | **H_FR2** | **H_CDR2** | **H_FR3** | **H_CDR3** | **H_FR4** |
| | VH3\|3-33\|D5\|5-24\|RF3\|JH6 | 32460 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | RDGYNYYY-------- -----YYYGMDV | WGQGT TVTVS S |
| iPS:4 36784 | 21-225_169C1 | VH3\|3-33\|D5\|5-24\|RF3\|JH6 | QVQLVES-GGGVVQPGRSLRL SCAASG-FTLS | S-----NGMH | WVRQAPGK GLEWVA | VIWYD--- GSNKYYADS VKG | RFTISRDTSKNTLYLQMNSL RAEDTAVYYCAR | DQYNRNDGPP------ ----AYYYYYGLDV | WGQGT TVTVS S |
| iPS:4 36786 | 21-225_169A6 | VH3\|3-33\|D5\|5-24\|RF3\|JH6 | QVQLVES-GGGVVQPGRSLRL SCAASG-FTLS | S-----NGMH | WVRQAPGK GLEWVA | VIWYD--- GSNKYYADS VKG | RFTISRDNSKNTLSLQMNSL RAEDTAVYYCAR | DQYNRNDGPP------ ----AYYYYYGMDV | WGQGT TVTVS S |
| iPS:4 36796 | 21-225_170A5 | VH3\|3-33\|D5\|5-24\|RF3\|JH6 | QVQLVES-GGGVVQPGRSLRL SCAASG-LTFS | N-----CGMH | WVRQAPGK GLEWVA | IIWYD--- GSNKYYADS VKG | RFTISRDNSKNTLDLQMNSL RAEDTAVYYCAR | DQYNRNDGPP------ ----AYYYYYGLDV | WGQGT TVTVS S |
| iPS:4 36812 | 21-225_175C6 | VH3\|3-33\|D5\|5-24\|RF3\|JH6 | QVQLVES-GGGVVQPGRSLRL SCAASG-LTFS | N-----CGMH | WVRQAPGK GLEWVA | IIWYD--- GSNKYYADS VKG | RFTVSRDNSKNTLDLQMNSL RAEDTAVYYCAR | DQYNRNDGPP------ ----AYYYYYGLDV | WGQGT TVTVS S |
| | Germline | | **H_FR1** | **H_CDR1** | **H_FR2** | **H_CDR2** | **H_FR3** | **H_CDR3** | **H_FR4** |
| | VH3\|3-48\|D5\|5-18\|RF1\|JH6 | 32461 | EVQLVES-GGGLVQPGGSLRL SCAASG-FTFS | S------YSMN | WVRQAPGK GLEWVS | YISSS SSTIYYADS VKG | RFTISRDNAKNSLYLQMNSL RDEDTAVYYCAR | VDTAMVYY-------- -------YYYGMDV | WGQGT TVTVS S |
| iPS:4 36788 | 21-225_169B7 | VH3\|3-48\|D5\|5-18\|RF1\|JH6 | EVQLVES-GGGLVQPGGSLRL SCAASG-FTFS | S-----YSLN | WVRQAPGK GLEWVS | YIGSS--- GSIIFYADS VKG | RFTISRDNAKNSLYLQMNSL RDEDTAVYYCAR | GDTAGVT--------- -------YYYGMDV | WGQGT TVTVS S |
| iPS:4 36798 | 21-225_171F5 | VH3\|3-48\|D5\|5-18\|RF1\|JH6 | EVQLVES-GGGLVQPGGSLRL SCAASG-FTFS | S-----YSLN | WVRQAPGK GLEWVS | YIGSS--- GSIIFYADS VKG | RFTISRDNAKNSLYLQMNSL RDEDTAVYYCAR | GDTAGVT--------- -------YYYGMDV | WGQGT TVTVS S |
| iPS:4 36864 | 21-225_58G11 | VH3\|3-48\|D5\|5-18\|RF1\|JH6 | EVQLVES-GGGLVQPGGSLRL SCAASG-FTFS | S-----YSMN | WVRQAPGK GLEWIS | YISTS--- SSTIFYADS VKG | RFTISRDSAKNSLYLQMNSL RDEDTAVYYCAR | GDTAMVL--------- -------YYYGMDV | WGQGT TVTVS S |

| Clone | Name | VH Germline | No. | H_FR1 | H_CDR1 | H_FR2 | H_CDR2 | H_FR3 | H_CDR3 | H_FR4 |
|---|---|---|---|---|---|---|---|---|---|---|
| iPS:4 36866 | 21-225_59F2 | VH3\|3-48/D5\|5-18\|RF1/JH6 | | EVQLVES-GGGLVQPGGSLRL SCGASG-FTFS | S-----YSMN | WVRQAPGK GLEWVS | YISGS--- SNIIYYTDS VKG | RFTISRDNAKNSLYLQMNSL RDEDTAVYYCAR | ADTPMVL--------- -------YFYGMDV | WGQGT TVTVS S |
| iPS:4 36872 | 21-225_60D2 | VH3\|3-48/D5\|5-18\|RF1/JH6 | | EVQLVES-GGGLVQPGGSLRL SCGASG-FTFS | S-----YSMN | WVRQAPGK GLEWVS | YISES--- SNIIYYTDS VKG | RFTISRDNAMNSLYLQMNSL RDEDTAVYYCAR | ADTPMVL--------- -------YFYGMDV | WGQGT TVTVS S |
| | Germline | | | H_FR1 | H_CDR1 | H_FR2 | H_CDR2 | H_FR3 | H_CDR3 | H_FR4 |
| | VH3\|3-33/D5\|5-24\|RF1/JH6 | | 32462 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | VEMATIYY--------- -------YYYGMDV | WGQGT TVTVS S |
| iPS:4 36790 | 21-225_169G11 | VH3\|3-33/D5\|5-24\|RF1/JH6 | | QVQLVES-GGGVVQPGRSLRL SCAASG-FTFS | S-----YGMH | WVRQAPGK GLEWVA | IIWYD--- GSNKYYADS VKG | RFTISRDNSKNTLYLQMNSL RAEDTGVYYCAR | EGATYYHGSGS----- ---YYPATNYGMDV | WGQGT TVTVS S |
| | Germline | | | H_FR1 | H_CDR1 | H_FR2 | H_CDR2 | H_FR3 | H_CDR3 | H_FR4 |
| | VH3\|3-33/D3\|3-10\|RF1/JH6 | | 32463 & 32464 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | VLLWFGELL*-------- ------YYYYYGMDV | WGQGT TVTVS S |
| iPS:4 36792 | 21-225_169D12 | VH3\|3-33/D3\|3-10\|RF1/JH6 | | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAS | PLYDMGL--------- --------YYDMDV | WGQGT TVTVS S |
| | Germline | | | H_FR1 | H_CDR1 | H_FR2 | H_CDR2 | H_FR3 | H_CDR3 | H_FR4 |
| | VH1\|1-46/D7\|7-27\|RF3/JH4 | | 32465 | QVQLVQS-GAEVKKPGASVKV SCKASG-YTFT | S-----YYMH | WVRQAPGQ GLEWMG | IINPS--- GGSTSYAQK FQG | RVTMTRDTSTSTVYMELSSL RSEDTAVYYCAR | NWGY------------- FDY | WGQGT LVTVS S |
| iPS:4 36800 | 21-225_171D12 | VH1\|1-46/D7\|7-27\|RF3/JH4 | | QVQLVQS-GAEVKKPGASVKV SCKASG-YTFN | S-----YYMY | WVRQAPGQ GLEWMG | IINPS--- GGSTNYAQK FQG | RVTMTRDTSTSTLYMELNSL RSEDTAVYYCAS | GWE------------- -----------LNY | WGQGT LVTVS S |
| iPS:4 36804 | 21-225_172C3 | VH1\|1-46/D7\|7-27\|RF3/JH4 | | QVQLVQS-GAEVKKPGASVKV SCKASG-YTFR | S-----YYMY | WVRQAPGQ GLEWVG | TINPS--- GGSTNYAQK FQG | RVTMTRDTSTSTLYMELNSL RSEDTAVYYCAS | GWE------------- -----------LNY | WGQGT LVTVS S |
| iPS:4 36806 | 21-225_172B12 | VH1\|1-46/D7\|7-27\|RF3/JH4 | | QVQLVQS-GAEVKKPGASVTV SCKASG-YTFR | S-----YYMY | WVRQAPGQ GLEWVG | TINPS--- GGSTDYAQK FQG | RVTMTRDTSTSTLYMELNSL RSEDTAVYYCAS | GWE------------- -----------LNY | WGQGT LVTVS S |
| | Germline | | | H_FR1 | H_CDR1 | H_FR2 | H_CDR2 | H_FR3 | H_CDR3 | H_FR4 |
| | VH3\|3-30.3/D4\|4-23\|RF1/JH6 | | 32466 & 32467 & 32468 | QVQLVES-GGGVVQPGRSLRL SCAASG-FTFS | S-----YAMH | WVRQAPGK GLEWVA | VISYD--- GSNKYYADS VKG | RFTISRDNSKNTLYLQMNSL RAEDTAVYYCAR | *LRW*LYY--------- YYYGMDV | WGQGT TVTVS S |
| iPS:4 36808 | 21-225_173F8 | VH3\|3-30.3/D4\|4-23\|RF1/JH6 | | QVQLVES-GGGVVQPGRSLRL SCAASG-FTFS | S-----YGMH | WVRQAPGK GLEWVA | VISYD--- GSPKYCADS VKG | RFTISRDNSKNTLFLQMNSL RAEDTAVYYCAR | DERQWLP--------- -------APYGMDV | WGQGT TVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| VH6\|6-01/D6\|6-6\|RF2/JH6 | | 32469 | QVQLQQS-GPGLVKPSQILSLTCAISG-DSVS | SN---SAAWN | WIRQSPSR GLEWLG | RTYYR--SKWYNDYAV SVKS | RITINPDISKNQFSLQLNSV TPEDTAVYYCAR | SIAARYY----------YYYGMDV | WGQGT TVTVS S |
| iPS:4 36810 | 21-225_175F4 | VH6\|6-01/D6\|6-6\|RF2/JH 6 | QVQLQQS-GPGLVKPSQILSL TCAISG-DSVS | SN---SAAWN | WIRQSPSR GLEWLG | RTYYR--SKWYNAYPV SMES | RISINPDISKNQFSLQLNSV TPEDTAVYYCAR | DKAAGRND--------------FYYYGMDV | WGQGT TVTVS S |
| iPS:4 36814 | 21-225_178H10 | VH6\|6-01/D6\|6-6\|RF2/JH 6 | QVQLQQS-GPGLVKPSQILSL TCAISG-DSVS | SN---SAAWN | WIRQSPSR GLEWLG | RTYYR--SKWYSAYPV SMES | RVSINPDISKNQFSLQLNSV TPEDTAVYYCAR | DKAAGRND--------------FYYYGMDV | WGQGT TVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| VH3\|3-33/D1\|1-1\|RF3/JH6 | | 32470 | QVQLVES-GGGVVQPGRSLRL SCAASG-FTFS | S------YGMH | WVRQAPGK GLEWVA | VIWYD---GSNKYYADS VKG | RFTISRDNSKNTLYLQMNSL RAEDTAVYYCAR | YNWNDYY----------------YYYGMDV | WGQGT TVTVS S |
| iPS:4 36818 | 21-225_179C7 | VH3\|3-33/D1\|1-1\|RF3/JH 6 | QAQLVES-GGGVVQPGRSLRL SCAASG-FTFS | N------SGMH | WVRQGPGK GLEWVA | IIYYD---GSYKYNADS VKG | RFTISRDNSKNTLYLQMNSL RAEDTAVYYCAR | DRHYDFHVP------------YYYYYGMDV | WGQGT TVTVS S |
| iPS:4 37094 | 21-225_210D12 | VH3\|3-33/D1\|1-1\|RF3/JH 6 | QVQLVES-GGGVVQPGRSLRL SCAASG-FTFS | H------YGMH | WVRQAPGK GLEWVA | VIWYD---GSNKYYADS VKG | RFTISRDNSKNTLYLQMNSL RAEDTAVYYCAR | GDWNP-------------------EGLDV | WGQGT TVTVS S |
| iPS:4 37096 | 21-225_210E12 | VH3\|3-33/D1\|1-1\|RF3/JH 6 | QVHLVES-GGGVVQPGRSLRL SCAASG-FTFS | N------YGMH | WVRQAPGK GLEWVA | VIWYD---GSNKYYADS VKG | RFTISRDNSKNTLYLQMNSL RAEDTAVYYCAR | GDWNP-------------------EGMDV | WGQGT TVIVS S |
| iPS:4 37098 | 21-225_211C1 | VH3\|3-33/D1\|1-1\|RF3/JH 6 | QVQLVES-GGGVVQPGRSLRL SCAASG-FTFS | H------YGMH | WVRQAPGK GLEWVA | VIWYD---GSNKYYADS VKG | RFTISRDNSKNTLYLQMNTL RAEDTAIYYCAR | GDWNP-------------------EGLDV | WGQGT TVTVS S |
| iPS:4 37104 | 21-225_211G5 | VH3\|3-33/D1\|1-1\|RF3/JH 6 | QVQLVES-GGGVVQPGRSLRL SCAASG-FTFS | H------YGMH | WVRQAPGK GLEWVA | VIWYD---GSNKYYADS VKG | RFTISRDNSKNTLYLQMNSL RAEDTAVYYCAR | GDWNP-------------------EGLDV | WGQGT TVTVS S |
| iPS:4 37112 | 21-225_212C2 | VH3\|3-33/D1\|1-1\|RF3/JH 6 | QVQLVES-GGGVVQPGRSLRL SCAASG-FTFS | H------YGMH | WVRQAPGK GLEWVA | VIWYD---GSNKYYADS VKG | RFTISRDNSKNTLYLQMNSL RAEDTAVYYCAR | GDWNP-------------------EGMDV | WGQGT SVTVS S |
| iPS:4 37114 | 21-225_212A4 | VH3\|3-33/D1\|1-1\|RF3/JH 6 | QVHLVES-GGGVVQAGRSLRL SCAASG-FTFS | N------YGMH | WVRQAPGK GLEWVA | VIWYD---GSNKYYADS VKG | RFTISRDNSKNTLYLQMNSL RAEDTAVYYCAR | GDWNP-------------------EGMDV | WGQGT TVIVS S |
| iPS:4 37116 | 21-225_212F6 | VH3\|3-33/D1\|1-1\|RF3/JH 6 | QVQLVES-GGGVVQPGRSLRL SCAASG-FTFS | H------YGMH | WVRQAPGK GLEWVA | VIWYD---GSNKYYADS VKG | RFTISRDNSKNTLYLQMNSL RAEDTAVYYCAR | GDWNP-------------------EGLDV | WGQGT TVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 37118 | 21-225_212G7 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYCADSVKG | RFTISRDNSINTLYLQMNSLRAEDTAVYYCAR | GDWNP--------------------EGLDV | WGQGTTVTVSS |
| iPS:4 37128 | 21-225_213G3 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLHLQMNSLRAEDTAVYYCAR | GDWNP--------------------EGMDV | WGQGTSVTVSS |
| iPS:4 37130 | 21-225_213D5 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLFLQMNSLRVEDTAVYYCAR | GDWNP--------------------EGMDV | WGQGTTVTVSS |
| iPS:4 37146 | 21-225_215D3 | VH3\|3-33/D1\|1-1\|RF3/JH6 | | QTQLVES-GGGVVQPGRSLRLSCAASG-FTFS | H-----YGMH | WVRQAPGKGLEWVA | VIWYD---GSNEYYADSVKG | RFTISRDNSKNTLYLQMNSLRAEDTAVYYCAR | GDWNP--------------------EGMDV | WGQGTTVTVSS |
| iPS:4 37150 | 21-225_216A3 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLYLQMNSLRAEDTAVYYCAR | GDWNP--------------------EGLDV | WGQGTTVTVSS |
| iPS:4 37162 | 21-225_217B2 | VH3\|3-33/D1\|1-1\|RF3/JH6 | | QVHLVES-GGGVVQPGRSLRLSCAASG-FTFS | N-----YGMH | WVRQAPGKGLEWVA | VIWYD---GSNKYYADSVKG | RFTISRDNSKNTLYLQMNSLRAEDTAVYYCAR | GDWNP--------------------EGMDV | WGQGTTVIVSS |
| iPS:4 37172 | 21-225_219A7 | VH3\|3-33/D1\|1-1\|RF3/JH6 | | QVQLVES-GGGVVQPGRSLRLSCPASG-FTFS | H-----YGMH | WVRQAPGKGLEWVA | VIWYD---GSNKYYADSVKG | RFTISRDNSKNMLYLQMNSLRAEDTAVYYCAR | GDWNP--------------------EGMDV | WGQGTTVTVSS |
| iPS:4 37182 | 21-225_221H2 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLHLQMNSLRAEDTAVYYCAR | GDWNP--------------------EGMDV | WGQGTTVTVSS |
| iPS:4 37184 | 21-225_221G4 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLHLQMNSLRAEDTAVYYCAR | GDWNP--------------------EGMDV | WGQGTSVTVSS |
| iPS:4 38664 | 21-225_216G1 | VH3\|3-33/D1\|1-1\|RF3/JH6 | | QVHLVES-GGGVVQPGRSLRLSCAASG-FTFS | N-----YGMH | WVRQAPGKGLEWVA | VIWYD---GSNKYYADSVKG | RFTISRDNSKNTLYLQMNSLRAEDTAVYYCAR | GDWNP--------------------EGMDV | WGQGTTVIVSS |
| | Germline | | | H_FR1 | H_CDR1 | H_FR2 | H_CDR2 | H_FR3 | H_CDR3 | H_FR4 |
| | VH3\|3-33/D1\|1-1\|RF1/JH4 | | 32471 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GTTGT--------------------YFDY | WGQGTLVTVSS |
| iPS:4 36822 | 21-225_180D4 | VH3\|3-33/D1\|1-1\|RF1/JH4 | | QVQLVES-GGGVVQPGRSLRLSCAASG-FTFS | N-----FGMH | WVRQAPGKGLEWVA | IIWYD---GSDKYYADSVKG | RFTISRDNSKNTLYLQMNTLRAEDTAVYFCAR | GGPPFST--------------VTMYFDY | WGQGTLVTVSS |

| | | | | H_FR1 | H_CDR1 | H_FR2 | H_CDR2 | H_FR3 | H_CDR3 | H_FR4 |
|---|---|---|---|---|---|---|---|---|---|---|
| iPS:4 36828 | 21-225_181H1 | VH3\|3-33/D1\|1-1\|RF1/JH4 | | QVQLVES-GGGVVQPGRSLRLSCAASG-FTFS | N-----YGMH | WVRQAPGK GLEWVA | IIWYD---GSDKYYADS VKG | RITISRDNSKNTLYLQMNTL RAEDTAVYFCAR | GGPPFST----------------VTMYFDY | WGQGT LVTVS S |
| iPS:4 36950 | 21-225_184G4 | VH3\|3-33/D1\|1-1\|RF1/JH4 | | QVQLVES-GGGVVQPGRSLRLSCAVSG-FTFS | S-----YGMH | WVRQAPGK GLEWVA | IIWYD---GSNKYYADS VKG | RFTISRDNSKNTLYLQMNSL RAEDTAVYYCAR | GGPPFST----------------VTMYFDY | WGQGT LVTVS S |
| iPS:4 36952 | 21-225_185D2 | VH3\|3-33/D1\|1-1\|RF1/JH4 | | QVQLVES-GGGVVQPGRSLRLSCAASG-FTFS | N-----YGMH | WVRQAPCK GLEWVA | IIWYD---GSDKYYADS VKG | RFTISRDNSKNTLYLQMNTL RAEDTAVYFCAR | GGPPFST----------------VTMYFDY | WGQGT LVTVS S |
| | Germline | | | H_FR1 | H_CDR1 | H_FR2 | H_CDR2 | H_FR3 | H_CDR3 | H_FR4 |
| | VH2\|2-05/D6\|6-6\|RF2/JH3 | | 32472 | QITLKES-GPTLVKPTQTLTLTCTFSG-FSLS | TS---GVGVG | WIRQPPGK ALEWLA | LIYW----NDDKRYSPS LKS | RLTITKDTSKNQVVLTMTNM DPVDTATYYCAH | SIAAR------------------DAFDI | WGQGT MVTVS S |
| iPS:4 36824 | 21-225_180C5 | VH2\|2-05/D6\|6-6\|RF2/JH3 | | QITLKES-GPTLVKPTQTLTLTCTFSG-FSLS | TS---GVGVG | WIRQPPGK ALEWLG | FISW----NDDKRYSPS LKS | SLTITKDTSKNQVVLIMTNM DPVDTATYYCAH | KAAAV---------------AFDI | WGQGT MVTVS S |
| iPS:4 36956 | 21-225_186H6 | VH2\|2-05/D6\|6-6\|RF2/JH3 | | QITLKES-GPTLVKPTQTLTLTCTFSG-FSLS | TS---GVGVG | WIRQPPGK ALEWLG | FISW----NDDKRYSPS LKS | SLTITKDTSKNQVVLTMTNM DPVDTATYYCAH | KAAAV---------------AFDI | WGQGT MVTVS S |
| | Germline | | | H_FR1 | H_CDR1 | H_FR2 | H_CDR2 | H_FR3 | H_CDR3 | H_FR4 |
| | VH1\|1-08/D5\|5-18\|RF3/JH6 | | 32473 | QVQLVQS-GAEVKKPGASVKVSCKASG-YTFT | S-----YDIN | WVRQATGQ GLEWMG | WMNPN---SGNTGYAQK FQG | RVTMTRNTSISTAYMELSSL RSEDTAVYCAR | GYSYGYYY---------YYYGMDV | WGQGT TVTVS S |
| iPS:4 36826 | 21-225_180G5 | VH1\|1-08/D5\|5-18\|RF3/JH6 | | QVQLVQS-GAEVKKPGASVKVSCKASG-YTFT | S-----YDIN | WVRQATGQ GLEWMG | WMNPN---SGNTGYAQK FQG | RVTMTRNTSISTAYMELSSL RSEDTAVYYCAR | GFYYYGSGSHV---------PYHYYYGLDV | WGQGT TVTVS S |
| | Germline | | | H_FR1 | H_CDR1 | H_FR2 | H_CDR2 | H_FR3 | H_CDR3 | H_FR4 |
| | VH6\|6-01/D5\|5-24\|RF3/JH2 | | 32474 | QVQLQQS GPGLVKPSQTLSLTCAISG-DSVS | SN---SAAWN | WIRQSPSR GLEWLG | RTYYR--SKWYNDYAV SVKS | RITINPDTSKNQFSLQLNSV TPEDTAVYYCAR | RDGYNY---------YWYFDL | WCRGT LVTVS S |
| iPS:4 36832 | 21-225_51D8 | VH6\|6-01/D5\|5-24\|RF3/JH2 | | QVQLQQS-GPGLVKPSQTLSLTCAISG-DSVS | SN---SAAWN | WIRQSPSR GLEWLG | RTYYR--SKWYNDYAV SVKS | RITFNPDTSKNQFSLRLNSV TPEDTAVYYCAR | DRYNWNY----------------PYWYFDL | WGRGT LVTVS S |
| | Germline | | | H_FR1 | H_CDR1 | H_FR2 | H_CDR2 | H_FR3 | H_CDR3 | H_FR4 |
| | VH1\|1-02/D5\|5-24\|RF3/JH5 | | 32475 | QVQLVQS-GAEVKKPGASVKVSCKASG-YTFT | G-----YYMH | WVRQAPGQ GLEWMG | WINPN---SGGTNYAQK FQG | RVTMTRDTSISTAYMELSRL RSDDTAVYYCAR | RDGYNY----------NWFDP | WGQGT LVTVS S |
| iPS:4 36840 | 21-225_53E9 | VH1\|1-02/D5\|5-24\|RF3/JH5 | | QVQLVQS-GAEVKKPGASVKVSCKASG-NTFT | G-----YYMH | WVRQAPGQ GLEWMG | WIIPN---SGDTNYAQK FQG | RVTMTRDTSISTAYMELSRL RSDDTAVYYCAR | DGYSSGW----------------FNWFDP | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH2\|2-05/D7\|7-27\|RF1/JH4 | | 32476 | QITLKES-GPTLVKPTQTLTL TCTFSG-FSLS | TS---GVGVG | WIRQPPGK ALEWLA | LIYW---- NDDKRYSPS LKS | RLTITKDTSKNQVVLTMTNM DPVDTATYYCAH | LIGY-----------------FDY | WGQGT LVTVS S |
| IPS:4 36848 | 21-225_57F1 | VH2\|2-05/D7\|7-27\|RF1/JH4 | | QITLKES-GPTLVKPTQTLTL TCTFSG-FSLS | TS---GVGVG | WIRQPPGK ALEWLA | LIYW---- HEDKRYSPS LKS | RLTITEDTSKNQVDLTMTNM APVDTATYYCAH | VIGI---------------AAPY | WGQGT LVTVS S |
| IPS:4 36852 | 21-225_57H11 | VH2\|2-05/D7\|7-27\|RF1/JH4 | | QITLKES-GPTLVKPTQTLTL TCTFSG-FSLT | TS---GVGVG | WIRQPPGK ALEWLA | LIYW---- HEDRRYSPS LKS | RLTITEDTSKNQVDLTMTNM APVDTATYYCAH | VIGI---------------AAPY | WGQGT LVTVS S |
| IPS:4 36870 | 21-225_60B1 | VH2\|2-05/D7\|7-27\|RF1/JH4 | | QITLKES-GPTLVKPTQTLTL TCTFSG-FSLS | TS---GVGVG | WIRQPPGK ALEWLA | LIYW---- HEDKRYSPS LKS | RLTITEDTSKNQVDLTMTNM APVDTATYYCAH | VTYI---------------AAPY | WGQGT LVTVS S |
| IPS:4 36876 | 21-225_61F5 | VH2\|2-05/D7\|7-27\|RF1/JH4 | | QITLKES-GPTLVKPTQTLTL TCTFSG-FSLS | TS---GLGVG | WIRQPPGK ALEWLA | LIYS---- HEDKRYSPS LKS | RLTITEDTSKNQVDLTMTNM APVDTATYYCAH | VIGI---------------AAPY | WGQGT LVTVS S |
| IPS:3 92593 | 21-225_3E10 | VH2\|2-05/D7\|7-27\|RF1/JH4 | | QITLKES-GPTLVKPTQTLTL TCTFSG-FSLN | TG---GVGVG | WIRQPPGK ALEWLA | LIYW---- NDDKRHSPS LKS | RLTITKDTSKNQVVLTMTHM APVDTATYYCAH | LIEV---------------AFDY | WGQGT LVTVS S |
| | | Germline | H_FR1 | H_CDR1 | H_FR2 | H_CDR2 | H_FR3 | H_CDR3 | H_FR4 |
| | VH2\|2-05/D6\|6-6\|RF2/JH1 | | 32477 | QITLKES-GPTLVKPTQTLTL TCTFSG-FSLS | TS---GVGVG | WIRQPPGK ALEWLA | LIYW---- NDDKRYSPS LKS | RLTITKDTSKNQVVLTMTNM DPVDTATYYCAH | SIAARA-----------EYFQH | WGQGT LVTVS S |
| IPS:4 36854 | 21-225_58C1 | VH2\|2-05/D6\|6-6\|RF2/JH1 | | QITLKES-GPTLVKPTQTLTL TCTFSG-FSLS | TS---GVGVG | WIRQPPGK ALEWLA | LIYW---- DDDKRYSPS LKS | RLTITEDTSKNQVVLTMTNM DPVDTATYYCAH | LIAV---------------AFDS | WGQGT LVTVS S |
| IPS:4 36874 | 21-225_60A12 | VH2\|2-05/D6\|6-6\|RF2/JH1 | | QITLKES-GPTLVKPTQTLTL TCTFSG-FSLS | TS---GVGVG | WIRQPPGK ALEWLA | LIYW---- DDDKRYSPS LKS | RLTITEDTSKNQVVLTMTNM DPVDTATYYCAH | LIAV---------------AFDS | WGQGT LVTVS S |
| IPS:4 36954 | 21-225_185G7 | VH2\|2-05/D6\|6-6\|RF2/JH1 | | QITLKES-GPTLVKPTQTLTL TCTFSG-FSLT | TG---GVGVG | WIRQPPGK ALEWLA | LIYW---- NDDERYSPS LKS | RLTITKDTSKNQVVLTMTNM DPVDTATYYCAH | IIAV---------------AFQH | WGQGT LVTVS S |
| IPS:3 93188 | 21-225_34B9 | VH2\|2-05/D6\|6-6\|RF2/JH1 | | QITLRES-GPTLVKPTQTLTL TCTFSG-FSLS | TS---GVGVG | WIRQPPGK ALEWLA | LIYW---- NDDKRYSPS LKS | RLTITKDTSKNQVVLTMTNM DPVDTATYYCAH | LIAV---------------TFDS | WGQGS LVTVS S |
| | | Germline | H_FR1 | H_CDR1 | H_FR2 | H_CDR2 | H_FR3 | H_CDR3 | H_FR4 |
| | VH3\|3-30.3/D3\|3-10\|RF2/JH6 | | 32478 | QVQLVES-GGGVVQPGRSLRL SCAASG-FTFS | S------YAMH | WVRQAPGK GLEWVA | VISYD--- GSNKYYADS VKG | RFTISRDNSKNTLYLQMNSL RAEDTAVYYCAR | YYYGSGSYYN-------YYYYYGMDV | WGQGT TVTVS S |

| iPS ID | Clone | VH | No. | H_FR1 | H_CDR1 | H_FR2 | H_CDR2 | H_FR3 | H_CDR3 | H_FR4 |
|---|---|---|---|---|---|---|---|---|---|---|
| iPS:4 36858 | 21-225_58E7 | VH3\|3-30.3/D3\|3-10\|RF2/JH6 | | QVQLVES-GGGVVQPGRSLRLSCAASG-FTFS | F-----YGMH | WVRQAPGK GLEWVA | VTSYD---GSDKYYADS VKG | RFSISRDNSKNTLYLQMSSL RAEDTAMYYCAR | DDYGSGSP---------------LYYGMDV | WGQGT TVTVS S |
| Germline | | | | H_FR1 | H_CDR1 | H_FR2 | H_CDR2 | H_FR3 | H_CDR3 | H_FR4 |
| | VH3\|3-07/D5\|5-12\|RF3/JH6 | | 32479 | EVQLVES-GGGLVQPGGSLRLSCAASG-FTFS | S-----YWMS | WVRQAPGK GLEWVA | NIKQD---GSEKYYVDS VKG | RFTISRDNAKNSLYLQMNSL RAEDTAVYYCAR | GYSGYDYY----------YYYYGMDV | WGQGT TVTVS S |
| iPS:4 36860 | 21-225_58F7 | VH3\|3-07/D5\|5-12\|RF3/JH6 | | EVQLVES-GGGLVQPGGSLRLSCAASG-FTFS | S-----FWMS | WVRQAPGK GLEWVA | HIKQD---GSEKYYVDS VKG | RFTISRDNAKNSLYLQMNSL RAEDTAVYYCAR | GDLPYSSG----------YYYYGMDV | WGQGT TVTVS S |
| Germline | | | | H_FR1 | H_CDR1 | H_FR2 | H_CDR2 | H_FR3 | H_CDR3 | H_FR4 |
| | VH3\|3-30.3/D4\|4-17\|RF2/JH6 | | 32480 | QVQLVES-GGGVVQPGRSLRLSCAASG-FTFS | S-----YAMH | WVRQAPGK GLEWVA | VISYD---GSNKYYADS VKG | RFTISRDNSKNTLYLQMNSL RAEDTAVYYCAR | DYGDYYY----------YYYYGMDV | WGQGT TVTVS S |
| iPS:4 36862 | 21-225_58F8 | VH3\|3-30.3/D4\|4-17\|RF2/JH6 | | QVQLVES-GGGVVQPGRSLRLSCAASG-FTFS | S-----YGMH | WVRQAPGK GLEWVA | VISYD---GSNKYYADS VKG | RFTISRDNSKNTLYLQMNSL RAEDTAVYYCAR | DEGRGYGGYER-----GYYYYYYGMDV | WGQGT TVTVS S |
| Germline | | | | H_FR1 | H_CDR1 | H_FR2 | H_CDR2 | H_FR3 | H_CDR3 | H_FR4 |
| | VH3\|3-33/D3\|3-16\|RF2/JH6 | | 32481 | QVQLVES-GGGVVQPGRSLRLSCAASG-FTFS | S-----YGMH | WVRQAPGK GLEWVA | VIWYD---GSNKYYADS VKG | RFTISRDNSKNTLYLQMNSL RAEDTAVYYCAR | YYDYVWGSYRY-----TYYYYYGMDV | WGQGT TVTVS S |
| iPS:4 36868 | 21-225_59B11 | VH3\|3-33/D3\|3-16\|RF2/JH6 | | QVQLVES-GGGVVQPGRSLRLSCAASG-FTFS | S-----YGVH | WVRQAPGK GLEWVA | AIWYD---GSNKYYADS VKG | RFTISRDNSKNTLYLLMNSL RAEDTAVYYCAR | DRDYCSSSSC-----PYYYYYGMDV | WGQGT TVTVS S |
| Germline | | | | H_FR1 | H_CDR1 | H_FR2 | H_CDR2 | H_FR3 | H_CDR3 | H_FR4 |
| | VH2\|2-05/D1\|1-1\|RF1/JH3 | | 32482 | QITLKES-GPTLVKPTQTLTLTCTFSG-FSLS | TS---GVGVG | WIRQPPGK ALEWLA | LIYW----NDDKRYSPS LKS | RLTITKDISKNQVVLTMTNM DPVDTATYYCAR | GTGT------------DAFDI | WGQGT MVTVS S |
| iPS:4 36878 | 21-225_62E3 | VH2\|2-05/D1\|1-1\|RF1/JH3 | | QITLKES-GPTLVKPTQTLTLTCTFSG-FSLS | TS---GVGVG | WIRQPPGK ALEWLA | LINW----NDDKRYSPS LKS | RFTITRDTSKDQVVLTMTNM DPVDTATYYCAH | KATWV------------AFDI | WGQGT MVTVS S |
| iPS:4 36880 | 21-225_62E8 | VH2\|2-05/D1\|1-1\|RF1/JH3 | | QITLKES-GPTLVKPTQTLTLTCTFSG-FSLS | TS---GVGVG | WIRQPPGK ALEWLA | LINW----NDDKRYSPS LKS | RFTITRDTSKDQVVLTMTNM DPVDTATYYCAH | KATWV------------AFDI | WGQGT MVTVS S |
| iPS:4 36882 | 21-225_62D10 | VH2\|2-05/D1\|1-1\|RF1/JH3 | | QITLKES-GPTLVKSTQTLTLTCTFSG-FSLS | TS---GVGVG | WIRQPPGK ALEWLA | LINW----NDDKRYSPS LKS | RFTITRDTSKDQVVLTMTNM DPVDTATYYCAH | KATWV------------AFDI | WGQGT MVTVS S |
| iPS:4 36884 | 21-225_62A12 | VH2\|2-05/D1\|1-1\|RF1/JH3 | | QITLKES-GPTLVKPTQTLTLTCTFSG-FSLS | TS---GVGVG | WIRQPPGK ALEWLA | LINW----NDDKRYSPS LKS | RFTITRDTSKDQVVLTMTNM DPLDTATYYCAH | KTTWV------------AFDI | WGQGT MVTVS S |

| iPS ID | Clone | VH gene | | H_FR1 | H_CDR1 | H_FR2 | H_CDR2 | H_FR3 | H_CDR3 | H_FR4 |
|---|---|---|---|---|---|---|---|---|---|---|
| iPS:4 36886 | 21-225_62B12 | VH2\|2-05/D1\|1-1\|RF1/JH3 | | QITLKES-GPTLVKPTQILTL TCTFSG-FSLN | TS---GVGVG | WIRQPPGK ALEWLA | LINW---- NDDKRYSPS LKS | RFTITRDISKDQVVLTMTNM DPVDTATYYCAH | KATWV---------- ----------AFDI | WGQGT MVTVS S |
| iPS:4 36894 | 21-225_66G9 | VH2\|2-05/D1\|1-1\|RF1/JH3 | | QITLKES-GPTLVKPTQILTL TCTFSG-FSLS | TS---GVGVG | WIRQPPGK ALEWLA | LINW---- NDDKRFSPS LKS | RFTITRDISKDQVVLTMTNM DPVDTATYYCAH | KATWV---------- ----------AFDI | WGQGT MVTVS S |
| iPS:4 36908 | 21-225_72D5 | VH2\|2-05/D1\|1-1\|RF1/JH3 | | QITLKES-GPTLVKPTQILTL TCTFSG-FSLS | TS---GVGVG | WIRQPPGK ALEWLA | LINW---- NDDKRYSPS LKS | RFTITRDISKDQVVFTMTNM DPVDTGTYYCAH | KATWV---------- ----------AFDI | WGQGT MVTVS S |
| iPS:4 36912 | 21-225_73C4 | VH2\|2-05/D1\|1-1\|RF1/JH3 | | QITLKES-GPTLVKPTQILTL TCTFSG-FSLS | TS---GVGVG | WIRQPPGK ALEWLA | LINW---- NDDKRYSPS LKS | RFTITRDISKDQVVLTMTNM DPVDTATYYCAH | KTTWV---------- ----------AFDI | WGQGT MVTVS S |
| | Germline | | | H_FR1 | H_CDR1 | H_FR2 | H_CDR2 | H_FR3 | H_CDR3 | H_FR4 |
| | VH3\|3-48/D4\|4-11\|RF2/JH3 | | 32483 | EVQLVES-GGGLVQPGGSLRL SCAASG-FTFS | S-----YSMN | WVRQAPGK GLEWVS | YISSS--- SSTIYYADS VKG | RFTISRDNAKNSLYLQMNSL RDEDTAVYYCAR | DYSNY----------- ---------DAFDI | WGQGT MVTVS S |
| iPS:4 36888 | 21-225_63G7 | VH3\|3-48/D4\|4-11\|RF2/JH3 | | EVQLVES-GGGLVQPGGSLRL SCAASG-FTFS | S-----YSMN | WVRQAPGK GLEWVS | YISSS--- TSTIYYAAS VKG | RFTISRDNAKNSLYLQMNSL RDEDTAVYYCAR | DHRYYDSSG------- ------YYSDAFDI | WGQGT MVTVS S |
| iPS:4 36890 | 21-225_63A10 | VH3\|3-48/D4\|4-11\|RF2/JH3 | | EVQLVES-GGGLVQPGGSLRL SCAASG-FTFS | S-----YSMN | WVRQAPGK GLEWVS | YISSS--- TSTIYYAAS VKG | RFTISRDNAKNSLYLQMNSL RDEDTAVYYCAR | DHRYYDSSG------- ------YYSDAFDI | WGQGT MVTVS S |
| | Germline | | | H_FR1 | H_CDR1 | H_FR2 | H_CDR2 | H_FR3 | H_CDR3 | H_FR4 |
| | VH1\|1-02/D3\|3-22\|RF2/JH3 | | 32484 | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | YYYDSSGY-------- -------YYDAFDI | WGQGT MVTVS S |
| iPS:4 36892 | 21-225_65E9 | VH1\|1-02/D3\|3-22\|RF2/JH3 | | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | AYYYGSGS-------- -------YYNEFDM | WGQGT MVTVS S |
| iPS:4 36900 | 21-225_69B9 | VH1\|1-02/D3\|3-22\|RF2/JH3 | | QVQMVQS-GDEVKKPGASVKV SCKASG-YTFT | G-----YHMH | WVRQAPGQ GLEWMG | WINPN--- SGGTNYAQK FQG | RVTMTRDTSISTAYMELSRM RSDDTAVYYCAR | AYYYGSGS-------- -------YYNESDM | WGQGT MVTVS S |
| | Germline | | | H_FR1 | H_CDR1 | H_FR2 | H_CDR2 | H_FR3 | H_CDR3 | H_FR4 |
| | VH6\|6-01/D4\|4-17\|RF2/JH6 | | 32485 | QVQLQQS-GPGLVKPSQTLSL TCAISG-DSVS | SN---SAAWN | WIRQSPSR GLEWLG | RTYYR-- SKWYNDYAV SVKS | RITINPDTSKNQFSLQLNSV TPEDTAVYYCAR | DYGDYYY--------- YYYGMDV | WGQGT TVTVS S |
| iPS:4 36898 | 21-225_68D8 | VH6\|6-01/D4\|4-17\|RF2/JH6 | | QVQLQQS-GPGLVKPSQTLSL TCAISG-DSVS | SN---SAAWN | WIRQSPSR GLEWLG | RTYYR-- SECYNDYAV SVQS | RITINPDTSKNQFSLHLNSV TPEDTAVYFCAR | DRGHRG--------- --------FYGMDV | WGQGT TVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-33/D7\|7-27\|RF1/JH3 | 32486 | QVQLVES-GGGVVQPGRSLRLSCAASG-FTFS | S------YGMH | WVRQAPGK GLEWVA | VIWYD---GSNKYYADS VKG | RFTISRDNSKNTLYLQMNSL RAEDTAVYYCAR | LTGD---------------------AFDI | WGQGT MVTVS S |
| iPS:4 36910 | 21-225_73G1 | VH3\|3-33/D7\|7-27\|RF1/JH3 | QVQ_VES-GGGVVQPGRSLRLSCAGTG-FTFS | Y------YGMH | WVRQAPGK GLEWVA | VTTYD---GSNKYYADS VKG | RFTSSRDNSKNTLYLQMNSL RAEDTAVYHCAR | ETGTW-------------------AFDI | WGQGT MVTVS L |
| | | Germline | H_FR1 | H_CDR1 | H_FR2 | H_CDR2 | H_FR3 | H_CDR3 | H_FR4 |
| | VH4\|4-30.1/D2\|2-15\|RF3/JH4 | 32487 | QVQLQES-GPGLVKPSQTLSLTCTVSG-GSIS | SG---GYYWS | WIRQHPGK GLEWIG | YIYY----SGSTYYNPS LKS | RVTISVDTSKNQFSLKLSSV TAADTAVYYCAR | DIVVVVA-------------------ATYFDY | WGQGT LVTVS S |
| iPS:4 36920 | 21-225_74E5 | VH4\|4-30.1/D2\|2-15\|RF3/JH4 | QVQLQES-GPGLVKPSQTLSLTCTVSG-GSIR | SG---GYYWS | WIRQHPGK GLEWIG | YIYY----SGSTYYNPS LRS | RASISVDTSKNQFSLKLSSV TAADTAVYYCAR | DSPVA-------------------GTDY | WGQGT LVTVS S |
| iPS:4 37012 | 21-225_192G7 | VH4\|4-30.1/D2\|2-15\|RF3/JH4 | QVQLQES-GPGLVKPSQTLSLTCTVSG-GSIS | SG---GYYWS | WIRQHPGK GLEWIG | YIYY----RGSTYYNPS LKS | RVTISVDTSKNQFSLKLNSV TAADTAVYYCAR | DSPVT-------------------GFDY | WGQGI LVTVS S |
| | | Germline | H_FR1 | H_CDR1 | H_FR2 | H_CDR2 | H_FR3 | H_CDR3 | H_FR4 |
| | VH1\|1-08/D5\|5-12\|RF3/JH6 | 32488 | QVQLVQS-GAEVKKPGASVKVSCKA3G-YTFT | S------YDIN | WVRQATGQ GLEWMG | WMNPN---SGNTGYAQK FQG | RVTMTRNTSISTAYMELSSL RSEDTAVYYCAR | GYSGYDYY---------------YYYYGMDV | WGQGT TVTVS S |
| iPS:4 36942 | 21-225_146H8 | VH1\|1-08/D5\|5-12\|RF3/JH6 | QVQLVQS-GAEVKKPGASVKVSCKASG-YTFI | S------YDIN | WVRQATGQ GLEWMG | WMNPN---SGNTGYAQK FQG | RVTMTRNTSKSTAYMELSSL RSEDTAVYYCAR | GDYYYDSSGHQ-----------PYYYYYGMDV | WGQGT TVTVS S |
| | | Germline | H_FR1 | H_CDR1 | H_FR2 | H_CDR2 | H_FR3 | H_CDR3 | H_FR4 |
| | VH2\|2-05/D6\|6-6\|RF3/JH4 | 32489 & 32490 | QITLKES-GPTLVKPTQTLTLTCTFSG-FSLS | TS----GVGVG | WIRQPPGK ALEWLA | LIYW----NDDKRYSPS LKS | RLTITKDTSKNQVVLTMTNM DPVDTATYYCAH | V*QLV-----------------YFDY | WGQGT LVTVS S |
| iPS:4 36944 | 21-225_182D12 | VH2\|2-05/D6\|6-6\|RF3/JH4 | QITLKES-GPTLVKPTQPLILTCTFSG-FSLS | TT---GVGVG | WIRQPPGK ALEWLG | ILFW----NDDERYSPS LKS | RLTITKDTSKNQVVLTMTNM DPVDTATYYCAH | KSQLV-------------------YFDY | WGQGT LVTVS S |
| | | Germline | H_FR1 | H_CDR1 | H_FR2 | H_CDR2 | H_FR3 | H_CDR3 | H_FR4 |
| | VH4\|4-30.1/D2\|2-8\|RF3/JH4 | 32491 | QVQLQES-GPGLVKPSQTLSLTCTVSG-GSIS | SG---GYYWS | WIRQHPGK GLEWIG | YIYY----SGSTYYNPS LKS | RVTISVDTSKNQFSLKLSSV TAADTAVYYCAR | DIVLMVY-----------------AIYFDY | WGQGT LVTVS S |
| iPS:4 36958 | 21-225_190D1 | VH4\|4-30.1/D2\|2-8\|RF3/JH4 | QVQLQES-GPGLVKPSQTLSLTCTVSG-GSIS | SG---GYYWS | WIRQHPGK GLEWIG | YIYY----SGSTYYNPS LKS | RVTISVDTSKNQFSLKLSSV TAADTAVYYCAR | DSPLR-------------------GFDY | WGQGT LVTVS S |
| | | Germline | H_FR1 | H_CDR1 | H_FR2 | H_CDR2 | H_FR3 | H_CDR3 | H_FR4 |

| | | | H_FR1 | H_CDR1 | H_FR2 | H_CDR2 | H_FR3 | H_CDR3 | H_FR4 |
|---|---|---|---|---|---|---|---|---|---|
| VH6\|6-01/D4\|4-17\|RF2/JH4 | | 32492 | QVQLQQS-GPGLVKPSQTLSL TCAISG-DSVS | SN---SAAWN | WIRQSPSR GLEWLG | RTYYR-- SKWYNDYAV SVKS | RITINPDISKNQFSLQLNSV TPEDTAVYYCAR | DYGDY----------YFDY | WGQGT LVTVS S |
| iPS:4 36962 | 21-225_190H1 | VH6\|6-01/D4\|4-17\|RF2/JH4 | | QVQLQQS-GPGLVKPSQTLSL TCDISG-DSVS | RK---SAIWN | WIRQSPSR GLEWLG | KTYYR-- SKWYNDYAV SVKS | RITINPDISKNQFSLQLNSV TPEDTAVYYCAR | DPGG----------LFDY | WGQGT LVTVS S |
| iPS:4 36978 | 21-225_190G9 | VH6\|6-01/D4\|4-17\|RF2/JH4 | | QVQLQQS-GPGLVKPSQTLSL TCAISG-DSVS | RK---SAIWN | WIRQSPSR GLEWLG | RTYYR-- SKWYNDYAV SVKS | RIIINPDISKNQFSLQLNSV TPEDTAVYYCAR | DPGG----------LFDY | WGQGT LVTVS S |
| iPS:4 37070 | 21-225_201G11 | VH6\|6-01/D4\|4-17\|RF2/JH4 | | QVQLQQS-GPGLVKPSQTLSL TCAISG-DSVS | RI---NPTWN | WIRQSPSR GLEWLG | RTYYR-- SKWYHVYAV SVKS | RITINPDISKNQFSLQLNSV TPEDTAVYYCAR | DPGG----------LFDY | WGQGT LVTVS S |
| iPS:4 37076 | 21-225_203G6 | VH6\|6-01/D4\|4-17\|RF2/JH4 | | QVQLQQS-GPGLVKPSQTLSL TCAISG-DSVS | RT---NPTWN | WIRQSPSR GLEWLG | RTYYR-- SKWYHVYAL SVKS | RITITPDISKNQFSLQLNSV TPEDTAVYYCAR | DPGG----------LFDY | WGQGT LVTVS S |
| | | Germline | H_FR1 | H_CDR1 | H_FR2 | H_CDR2 | H_FR3 | H_CDR3 | H_FR4 |
| VH3\|3-33/D1\|1-7\|RF3/JH6 | | 32493 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | YNWNYYY---------YYYGMDV | WGQGT TVTVS S |
| iPS:4 36964 | 21-225_190B3 | VH3\|3-33/D1\|1-7\|RF3/JH6 | | QVQLVES-GGGVVQPGRSLRL SCAASG-FTFN | N-----YGIH | WVRQAPGK GLEWVA | VIWFD--- GDNKYYADS VKG | RFTISRDNSKNTLYLQMNSL RAEDTAVYYCAR | DNWNYGDH---------YYYFGMDV | WGQGT TVTVS S |
| iPS:4 36970 | 21-225_190B8 | VH3\|3-33/D1\|1-7\|RF3/JH6 | | QVQLVES-GGGVVQPGRSLRL SCAASG-FTFS | S-----YGMH | WVRQAPGK GLEWVA | VIWFD--- GSNKYYTDS VKG | RFTISRDNSKNTLYLQMNSL RAEDTAVYYCAR | DNWNYGDY---------YYYYGMDV | WGQGT TVTVS S |
| iPS:4 36980 | 21-225_190C10 | VH3\|3-33/D1\|1-7\|RF3/JH6 | | QVQLVES-GGGVVQPGRSLRL SCAASG-FTLS | N-----YGMH | WVRQAPGK GLEWVA | VIWFG--- GDNKYYADS VRG | RFTISRDNSKNTLYLQMNSL RAEDTAVFYCAR | DNWNYGDH---------YYYYGMDV | WGQGT TVTVS S |
| iPS:4 36992 | 21-225_191B8 | VH3\|3-33/D1\|1-7\|RF3/JH6 | | QVQLVES-GGGVVQPGRSLRL SCAASG-FTLS | S-----YGMH | WVRQAPGK GLEWVA | VIWFD--- GSNKYYADS VKG | RFTISRDNSKNTLYLQMNSL RAEDTAVYYCAR | DNWNYGDH---------YYYYGMDV | WGQGT TVTVS S |
| iPS:4 36994 | 21-225_191A9 | VH3\|3-33/D1\|1-7\|RF3/JH6 | | QVQLVES-GGGVVQPGRSLRL SCAASG-FTLS | N-----YGMH | WVRQAPGK GLEWVA | VIWFG--- GDNKYYADS VKG | RFTISRDNSKNTLYLQMNSL RAEDTAVFYCAR | DNWNYGDH---------YYYYGMDV | WGQGT TVTVS S |
| | | Germline | H_FR1 | H_CDR1 | H_FR2 | H_CDR2 | H_FR3 | H_CDR3 | H_FR4 |
| VH4\|4-30.1/D4\|4-11\|RF2/JH6 | | 32494 | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV TAADTAVYYCAR | DYSNYYY---------YYYGMDV | WGQGT TVTVS S |

| | | | H_FR1 | H_CDR1 | H_FR2 | H_CDR2 | H_FR3 | H_CDR3 | H_FR4 |
|---|---|---|---|---|---|---|---|---|---|
| iPS:4 36984 | 21-225_190F10 | VH4\|4-30.1/D4\|4-11\|RF2/JH6 | QVQLQES-GPGLVKPSQILSL TCTVSG-GSIR | SG---GDYWS | WIRQHPGE GLEWIG | YIYY---- SGITYYNPS LKS | RVTISVDISKNQFSLKLSSV TAADTAVYYCAR | DSSSR----------- ----------GMDV | WGQGT TVTVS S |
| iPS:4 36988 | 21-225_191A2 | VH4\|4-30.1/D4\|4-11\|RF2/JH6 | QVQLQES-GPGLVKPSQILSL TCTVSG-GSIR | SG---GDYWS | WIRQHPGE GLEWIG | YIYY---- SGITYYNPS LKS | RVTISVDISKNQFSLKLSSV TAADTAVYYCAR | DSSSR----------- ----------GMDV | WGQGT TVTVS S |
| iPS:4 37014 | 21-225_192H8 | VH4\|4-30.1/D4\|4-11\|RF2/JH6 | QVQLQES-GPGVVKPSQILSL TCTVSG-GSIS | SG---GDYWS | WIRQHPGK GLEWIG | YIYY---- SGPTYYNPS LKS | RLTMSADTSKNQFSLKLSSV TAADTAVYYCAR | DSSLY----------- ----------GMDV | WGQGT TVTVS S |
| iPS:4 37022 | 21-225_194G5 | VH4\|4-30.1/D4\|4-11\|RF2/JH6 | QVQLQES-GPGLVKPSQILSL ICTVSG-GSIR | SG---GDYWS | WIRQHPGK GLEWIG | YIYY---- SGSTYYNPS LKS | RVTISVDISKNQFSLKLSSV TAADTAVYYCAR | DHSLY----------- ----------GMDV | WGQGT TVTVS S |
| iPS:4 37026 | 21-225_194D12 | VH4\|4-30.1/D4\|4-11\|RF2/JH6 | QVQLQES-GPGLVKPSQILSL TCTVSG-GSTS | SG---GDYWS | WIRQHPGK GLEWIG | YIYY---- SGSTYYNPS LKS | RVTISVDISKNQFSLKLSSV TAADTAVYYCAR | DGARH----------- ----------GMDV | WGQGT TVTVS S |
| iPS:4 37056 | 21-225_198B8 | VH4\|4-30.1/D4\|4-11\|RF2/JH6 | QVQLQES-GPGLVKPSQILSL TCTVSG-GSIR | SG---GDYWS | WIRQHPGE GLEWIG | YIYY---- SGITYYNPS LKS | RVTISVDISKNQFSLKLSSV TAADTAVYYCAR | DSSSR----------- ----------GMDV | WGQGT TVTVS S |
| iPS:4 37124 | 21-225_212H12 | VH4\|4-30.1/D4\|4-11\|RF2/JH6 | QVQLQES-GPGLVKPSQILSL TCTVSG-GSIR | SG---GDYWS | WIRQHPGK GLEWIG | YIYY---- SGSTYYNPS LKS | RVTISGDTSKNQFSLKLSSV TAADTAVYYCAR | DSSSY----------- ----------GMDV | WGQGT TVTVS S |
| iPS:4 37136 | 21-225_214H3 | VH4\|4-30.1/D4\|4-11\|RF2/JH6 | QVQLQES-GPGLVKPSQILSL TCTVSG-GSIR | SG---GDYWS | WIRQHPGK GLEWIG | YIYY---- SGSTYYNPS LKS | RVTISGDTSKNQFSLKLSSV TAADTAVYYCAR | DSSSY----------- ----------GMDV | WGQGT TVTVS S |
| Germline | | | H_FR1 | H_CDR1 | H_FR2 | H_CDR2 | H_FR3 | H_CDR3 | H_FR4 |
| | VH4\|4-59/D1\|1-26\|RF1/JH4 | 32495 | QVQLQES-GPGLVKPSETLSL TCTVSG-GSIS | S-----YYWS | WIRQPPGK GLEWIG | YIYY---- SGSTNYNPS LKS | RVTISVDTSKNQFSLKLSSV TAADTAVYYCAR | GIVGA----------- ---------TYFDY | WGQGT LVTVS S |
| iPS:4 36986 | 21-225_191A1 | VH4\|4-59/D1\|1-26\|RF1/JH4 | QVQLQES-GPGLVKPSETLSL TCTVSG-GSIR | S-----YYWI | WIRQPPGK GLEWIG | YIYY---- SGSTKYNPS LKS | RVTISVDTSKNQFSLKLSSV TAADTAVYYCAR | KGVGT----------- ---------IHFDY | WGQGT LVTVS S |
| iPS:4 37064 | 21-225_200G8 | VH4\|4-59/D1\|1-26\|RF1/JH4 | QVQLQES-GPGLVKPSETLSL TCTVSG-GSIR | S-----YYWS | WIRQPPGK GLEWIG | YIYY---- SGSTKYNPS LKS | RVTISVDTSKNQFSLKLSSV TAADTAVYYCAR | KGVGT----------- ---------IHFDY | WGQGT LVTVS S |
| Germline | | | H_FR1 | H_CDR1 | H_FR2 | H_CDR2 | H_FR3 | H_CDR3 | H_FR4 |
| | VH3\|3-30/D6\|6-6\|RF1/JH4 | 32496 | QVQLVES-GGGVVQPGRSLRL SCAASG-FTFS | S-----YGMH | WVRQAPGK GLEWVA | VISYD--- GSNKYYADS VKG | RFTISRDNSKNTLYLQMNSL RAEDTAVYYCAK | EYSSS----------- ---------SYFDY | WGQGT LVTVS S |

| ID | Clone | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 36996 | 21-225_191B9 | VH3|3-30/D6|6-6|RF1/JH4 | | QVQLVES-GGGVVQPGRSLRL SCAASG-FTFS | F-----HGMH | WVRQAPGK GLEWVA | VIWYD--- GSKKYYADS VKG | RFTISRDNSKNTLHLQMNSL RAEDTAVYYCAK | EGYSSGF-----------------YRGFDN | WGQGT LVTVS S |
| iPS:4 37054 | 21-225_194G3 | VH3|3-30/D6|6-6|RF1/JH4 | | QVQLVES-GGGVVQPGRSLRL SCAASG-FTFS | F-----HGMH | WVRQAPGK GLEWVA | VIWYD--- GSKKYYADS VKG | RFTISRDNSKNTLHLQMNSL RAEDTAVYYCAK | EGFSSGF-----------------YRGFDN | WGQGT LVTVS S |
| | | Germline | | H_FR1 | H_CDR1 | H_FR2 | H_CDR2 | H_FR3 | H_CDR3 | H_FR4 |
| | VH3|3-33/D1|1-26|RF1/JH6 | | 32497 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | GIVGAIYY-----------YYYGMDV | WGQGT TVTVS S |
| iPS:4 37000 | 21-225_191G9 | VH3|3-33/D1|1-26|RF1/JH6 | | QVQLVES-GGGVVQPGRSLRL SCAASG-FTFS | T-----YGMH | WVRQAPGK GLEWVT | LIWFD--- GSNKYYADS VKG | RFTISRDNSKNTLYLQMNSL RAEDTAVYYCAR | DRVGGISPP-----------YYYYYGMDV | WGQGT TVTVS S |
| iPS:3 93192 | 21-225_12B1 | VH3|3-33/D1|1-26|RF1/JH6 | | QVQLVES-GGGVVQPGRSLRL SCAASG-FTFS | S-----YGMH | WVRQAPGK GLEWVA | VIWND--- GSNKYYADS VKG | RFTISRDNSKNTLYLQMNSL RAEDTAVYYCAR | DRVAAAGTP-----------YYYYYGMDV | WGQGT TVTVS S |
| | | Germline | | H_FR1 | H_CDR1 | H_FR2 | H_CDR2 | H_FR3 | H_CDR3 | H_FR4 |
| | VH1|1-02/D3|3-10|RF2/JH5 | | 32498 | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | YYYCSGSY-----------YNNWFDP | WGQGT LVTVS S |
| iPS:4 37002 | 21-225_191H9 | VH1|1-02/D3|3-10|RF2/JH5 | | QVQLVQS-GAEVKKPGASVKV SCKVSG-YTFT | G-----YNMH | WVRQAPGK GLEWMG | WINPN--- SGGTNYAHK FQG | RVTMTRDTSISTAYMELSRL RSDDTAVYYCAR | DFYDSGG-------------EGWFDP | WGQGT LVTVS S |
| | | Germline | | H_FR1 | H_CDR1 | H_FR2 | H_CDR2 | H_FR3 | H_CDR3 | H_FR4 |
| | VH4|4-30.4/D2|2-8|RF3/JH6 | | 32499 | QVQLQES-GPGLVKPSQTLSL TCTVSG-GSIS | SG----DYYWS | WIRQPPGK GLEWIG | YIYY---- SGSTYYNPS LKS | RVTISVDTSKNQFSLKLSSV TAADTAVYYCAR | DIVLMVYAI-----------YYYYYGMDV | WGQGT TVTVS S |
| iPS:4 37008 | 21-225_192E3 | VH4|4-30.4/D2|2-8|RF3/JH6 | | QVQLQES-GPGLVKPSQTLSL TCTVSG-GSIS | SG---GYYWS | WIRQPPGK GLEWIG | YIYY---- TGSTYYNPS LKS | RVTISVDTSKNQFSLKLSSV TAADTAVYYCAR | DDPLY-----------GMDV | WGQGT TVTVS S |
| iPS:4 37048 | 21-225_197B11 | VH4|4-30.4/D2|2-8|RF3/JH6 | | QVQLQES-GPGLVKPSQTLSL TCTVSG-GSIS | SG---GYYWS | WIRQPPGK GLEWIG | YIYY---- TGSTYYNPS LKS | RVTISVDTSKNQFSLKLSSV TAADTAVYYCAR | DDPLY-----------GMDV | WGQGT TVTVS S |
| | | Germline | | H_FR1 | H_CDR1 | H_FR2 | H_CDR2 | H_FR3 | H_CDR3 | H_FR4 |
| | VH4|4-59/D7|7-27|RF3/JH4 | | 32500 | QVQLQES-GPGLVKPSETLSL TCTVSG-GSIS | S-----YYWS | WIRQPPGK GLEWIG | YIYY---- SGSTNYNPS LKS | RVTISVDTSKNQFSLKLSSV TAADTAVYYCAR | NNGY-----------------FDY | WGQGT LVTVS S |
| iPS:4 37010 | 21-225_192G3 | VH4|4-59/D7|7-27|RF3/JH4 | | QVQLQES-GPGLVKPSETLSL TCTVSG-GSIS | N-----YYWS | WIRQPAGK GLEWIG | RIYS---- SGSTNYNPS LKS | RVTMSVDTSKNQFSLKLNSV TAADTAVYYCAK | GWE-----------------LNY | WGQGT LVTVS S |

| ID | Clone | Germline | No. | H_FR1 | H_CDR1 | H_FR2 | H_CDR2 | H_FR3 | H_CDR3 | H_FR4 |
|---|---|---|---|---|---|---|---|---|---|---|
| iPS:4 37016 | 21-225_193A6 | VH4\|4-59/D7\|7-27\|RF3/JH4 | | QVQLQES-GPGLVKPSETLSL TCTVSG-GSIS | S-----YYWS | WIRQPPGK GLEWIG | YIYY---- SGSTNYNPS LKS | RVTISVDISKNQFSLKLSSV TAADTAVYYCAG | GWE----------- -----------LNY | WGQGT LVTVS S |
| | | Germline | | H_FR1 | H_CDR1 | H_FR2 | H_CDR2 | H_FR3 | H_CDR3 | H_FR4 |
| | VH3\|3-15/D4\|4-17\|RF2/JH4 | | 32501 | EVQLVES-GGGLVKPGGSLRL SCAASG-FTFS | N-----AWMS | WVRQAPCK GLEWVG | RIKSKT-DGGTTDYAA PVKG | RFTISRDDSKNTLYLQMNSL KTEDTAVYYCTT | DYGDY-------------------YFDY | WGQGT LVTVS S |
| iPS:4 37018 | 21-225_193H5 | VH3\|3-15/D4\|4-17\|RF2/JH4 | | EVQLVES-GGGLVMPGGSLSL SCAASG-FTFS | N-----AYMT | WVRQAPGK GLEWVG | RIKSKT-DGGTTDYAA PVKG | RFTISRDDSKNTLYLQMNSL KTEDTAVYYCTT | DPGG----------------IFDY | WGQGT LVTVS S |
| iPS:4 37144 | 21-225_215B3 | VH3\|3-15/D4\|4-17\|RF2/JH4 | | EVQLVES-GGGLVKPGGSLRL SCAASG-FTFS | N-----AWMH | WVRQAPGK GLEWVG | RIKSKT-NGGTTDYAA PVKG | RFTISRDDSKNTLYLQMNSL KTEDTAVYYCTT | DPGG----------------IFDY | WGQGT LVTVS S |
| | | Germline | | H_FR1 | H_CDR1 | H_FR2 | H_CDR2 | H_FR3 | H_CDR3 | H_FR4 |
| | VH3\|3-11/D3\|3-9\|RF1/JH4 | | 32502 & 32503 | QVQLVES-GGGLVKPGGSLRL SCAASG-FTFS | D-----YYMS | WIRQAPGK GLEWVS | YISSS---- GSTIYYADS VKG | RFTISRDNAKNSLYLQMNSL RAEDTAVYYCAR | VLRYFDW------------LL*YFDY | WGQGT LVTVS S |
| iPS:4 37030 | 21-225_195E3 | VH3\|3-11/D3\|3-9\|RF1/JH4 | | QVQLVES-GGGLVKPGGSLRL SCAASG-FTFS | D-----YYMS | WIRQAPGK GLEWVS | YITSS--- GNTIYYADS VKG | RFTISRDNAKNSLYLQMNSL RAEDTAVYYCAR | DSRYF-------------------DWFDY | WGQGT LVTVS S |
| | | Germline | | H_FR1 | H_CDR1 | H_FR2 | H_CDR2 | H_FR3 | H_CDR3 | H_FR4 |
| | VH4\|4-59/D7\|7-27\|RF3/JH1 | | 32504 | QVQLQES-GPGLVKPSETLSL TCTVSG-GSIS | S-----YYWS | WIRQPPGK GLEWIG | YIYY---- SGSTNYNPS LKS | RVTISVDTSKNQFSLKLSSV TAADTAVYYCAR | NWGAE---------------YFQH | WGQGT LVTVS S |
| iPS:4 37032 | 21-225_195H6 | VH4\|4-59/D7\|7-27\|RF3/JH1 | | QVQLQES-GPGLVKPSETLSL TCTVSG-GSIR | N-----YYWS | WIRQPAGK GLEWIG | RIYS---- SGSTNYNPS LKS | RVSMSVDTSKNQFSLKLSSV TAADTAVYYCTR | GWE-----------------LNN | WGQGT LVTVS S |
| | | Germline | | H_FR1 | H_CDR1 | H_FR2 | H_CDR2 | H_FR3 | H_CDR3 | H_FR4 |
| | VH4\|4-59/D6\|6-6\|RF1/JH6 | | 32505 | QVQLQES-GPGLVKPSETLSL TCTVSG-GSIS | S-----YYWS | WIRQPPGK GLEWIG | YIYY---- SGSTNYNPS LKS | RVTISVDTSKNQFSLKLSSV TAADTAVYYCAR | EYSSSSYY----------------YYYGMDV | WCQGT TVTVS S |
| iPS:4 37044 | 21-225_197F9 | VH4\|4-59/D6\|6-6\|RF1/JH6 | | QVQLQES-GPGLVKPSETLSL TCTVSG-GSIR | I-----YYWS | WIRQPPGK GLEWIG | YVYY---- SGSTTYNPS LKS | RVTISVDTSKNQFSLKLNSV TAADTAVYYCVR | ERGSSHRW--------------GDYYGMDV | WGRGT TVTVS S |
| iPS:4 37060 | 21-225_199C3 | VH4\|4-59/D6\|6-6\|RF1/JH6 | | QVQLQES-GPGLVKPSETLSL TCTVSG-GSIR | I-----YYWS | WIRQTPGK GLEWIG | YIYY---- SGSTTYNPS LKS | RVTISVDTSKNQFSLKLNSV TAADTAVYYCVR | ERGSSHRW--------------GDYYGMDV | WGRGT TVTVS S |
| | | Germline | | H_FR1 | H_CDR1 | H_FR2 | H_CDR2 | H_FR3 | H_CDR3 | H_FR4 |
| | VH3\|3-30/D6\|6-6\|RF1/JH1 | | 32506 | QVQLVES-GGGVVQPGRSLRL SCAASG-FTFS | S-----YGMH | WVRQAPGK GLEWVA | VISYD--- GSNKYYADS VKG | RFTISRDNSKNTLYLQMNSL RAEDTAVYYCAK | EYSSSS----------------AEYFQH | WGQGT LVTVS S |

| iPS ID | Clone | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 37058 | 21-225_199F3 | VH3\|3-30/D6\|6-6\|RF1/JH1 | | QVQLVES-GGGVVQPGRSLRL SCAASG-FTFS | F-----YGMH | WVRQAPGK GLEWVA | VIWYD--- GSSKYYADS VKG | RFTVSRDNSKNTLYLQMNSL RAEDTAVYYCAK | EGYSSGF---------------YRGFAN | WGQGT LVTVS S |
| | | Germline | | H_FR1 | H_CDR1 | H_FR2 | H_CDR2 | H_FR3 | H_CDR3 | H_FR4 |
| | VH4\|4-30.1/D6\|6-6\|RF2/JH6 | | 32507 | QVQLQES-GPGLVKPSQTLSL TCTVSG-GSIS | SG---GYYWS | WIRQHPGK GLEWIG | YIYY---- SGSTYYNPS LKS | RVTISVDTSKNQFSLKLSSV TAADTAVYYCAR | STAARYY---------YYYGMDV | WGQGT TVTVS S |
| iPS:4 37062 | 21-225_200H1 | VH4\|4-30.1/D6\|6-6\|RF2/JH6 | | QVQLQES-GPGLVKPSQTLSL TCTVSG-GSIS | SG---GDYWS | WIRQHPGK GLEWIG | YIYY---- SGSTYYNPS LKS | RVTISVDTSKNQFSLKLNSV TAADTAVYYCAR | DGAAL-----------GMDV | WGQGT TVTVS S |
| iPS:4 37066 | 21-225_200G9 | VH4\|4-30.1/D6\|6-6\|RF2/JH6 | | QVQLQES-GPGLVKPSQTLSL TCTVSG-GSIS | SG---GDYWS | WIRQHPGK GLEWIG | YIYY---- SGSTYYNPS LKS | RVTISVDTSKNQFSLKLNSV TAADTAVYYCAR | DGAAL-----------GMDV | WGQGT TVTVS S |
| iPS:4 37068 | 21-225_200A11 | VH4\|4-30.1/D6\|6-6\|RF2/JH6 | | QVQLQES-GPGLVKPSQTLSL TCTVSG-GSIS | SG---GDYWS | WIRQHPGK GLEWIG | YIYY---- RGSTYYNPS LKS | RVTISVDTSKNQFSLKLSSV TAADTAVYYCAR | DAAAH-----------GMDV | WGQGT TVTVS S |
| iPS:4 37140 | 21-225_214E12 | VH4\|4-30.1/D6\|6-6\|RF2/JH6 | | QVQLQES-GPGLVKPSQTLSL TCTVSG-GSIS | SG---GDYWS | WIRQHPGK GLEWIG | YIYY---- SGPTYYNPS LKS | RVTISVDTSKNQFSLKLSSV TAADTAVYYCAR | DGAAE-----------GMDV | WGQGT TVTVS S |
| iPS:4 37158 | 21-225_216H11 | VH4\|4-30.1/D6\|6-6\|RF2/JH6 | | QVQLQES-GPGLVKPSQTLSL TCTVSG-GSIS | SG---GDYWS | WIRQHPGK GLEWIG | YIYY---- SGPTYYNPS LKS | RVTISVDTSKNQFSLKLSSV TAADTAVYYCAR | DGAAE-----------GLDV | WGQGT TVTVS S |
| | | Germline | | H_FR1 | H_CDR1 | H_FR2 | H_CDR2 | H_FR3 | H_CDR3 | H_FR4 |
| | VH3\|3-33/D1\|1-26\|RF3/JH3 | | 32508 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | YSGSYY---------DAFDI | WGQGT MVTVS S |
| iPS:4 37074 | 21-225_203B2 | VH3\|3-33/D1\|1-26\|RF3/JH3 | | QVQLVES-GGGVVQPGRSLRL SCAASG-FTFS | N-----YGMH | WVRQAPGK GLEWVA | IIWFD--- GSNEYYADS VKG | RFTISRDNSMSTLYLQMNSL RAEDTAVYYCAR | ESGSY-----------ALYI | WGQGT MVTVS S |
| iPS:4 37082 | 21-225_205E12 | VH3\|3-33/D1\|1-26\|RF3/JH3 | | QVQLVES-GGGVVQPGRSLRL SCAASG-FTFS | N-----YGMH | WVRQAPGK GLEWVA | IIWFD--- GSNEYYADS VKG | RFTISRDNSMSTLYLQMNSL RAEDTAVYYCAR | ESGSY-----------ALYI | WGQGT MVTVS S |
| | | Germline | | H_FR1 | H_CDR1 | H_FR2 | H_CDR2 | H_FR3 | H_CDR3 | H_FR4 |
| | VH3\|3-48/D4\|4-17\|RF2/JH4 | | 32509 | EVQLVES-GGGLVQPGGSLRL SCAASG-FTFS | S-----YSMN | WVRQAPGK GLEWVS | YISSS--- SSTIYYADS VKG | RFTISRDNAKNSLYLQMNSL RDEDTAVYYCAR | DYGDY-----------YFDY | WGQGT LVTVS S |
| iPS:4 37086 | 21-225_209A8 | VH3\|3-48/D4\|4-17\|RF2/JH4 | | EVQLVES-GGGLVQPGGSLRL SCAASG-FTFR | S-----YSMN | WVRQAPGK GLEWVL | YISSS--- SSIKKYADS VKG | RFTISRDNAKNSLYLQMNSL RDEDTAVYYCVR | DDGSY-----------YFDY | WGQGT LVTVS S |

EP 4 435 105 A2

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-30.1/D2\|2-8\|RF3/JH6 | | 32510 | QVQLQES-GPGLVKPSQILSLTCTVSG-GSIS | SG---GYYWS | WIRQHPGKGLEWIG | YIYY----SGSTYYNPSLKS | RVTISVDTSKNQFSLKLSSVTAADTAVYYCAR | DIVLMVYAI--------------YYYYYGMDV | WGQGTTVTVSS |
| IPS:4 37090 | 21-225_210F11 | VH4\|4-30.1/D2\|2-8\|RF3/JH6 | | QVQLQES-GPGLVKPSQILSLTCTVSG-GSIS | SG---GSYWS | WIRQHPGKGLEWIG | YIYY----IGTTYYNPSLKS | RVTISVDTSTNHFSLKLSSVTAADTAVYYCAR | DEPLT-----------------GMDV | WGQGTTVTVSS |
| IPS:4 37106 | 21-225_211H7 | VH4\|4-30.1/D2\|2-8\|RF3/JH6 | | QVQLQES-GPGLVKPSQTLSLTCTVSG-GSIS | SG---GYYWS | WTRQHPGKGLEWIG | YIYY----VGSTYYNPSLKS | RVTISVDTSKNQFSLKLSSVTAADTAVYYCAR | DGPLS-----------------GMDV | WGQGTTVTVSS |
| | | Germline | | H_FR1 | H_CDR1 | H_FR2 | H_CDR2 | H_FR3 | H_CDR3 | H_FR4 |
| | VH3\|3-30.3/D4\|4-17\|RF2/JH5 | | 32511 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYGDY-----------------NWFDP | WGQGTLVTVSS |
| IPS:4 37100 | 21-225_211H2 | VH3\|3-30.3/D4\|4-17\|RF2/JH5 | | QVQLVES-GGGVVQPGRSLRLSCAASG-FTFS | S-----YAMH | WVRQAPGKGLEWVA | VIWYD---GSNKYYADSVKG | RFTIARDNSKNTLYLQMNSLRAEDTAVYYCAR | DFGSY-----------------GFDP | WGQGTLVTVSS |
| | | Germline | | H_FR1 | H_CDR1 | H_FR2 | H_CDR2 | H_FR3 | H_CDR3 | H_FR4 |
| | VH3\|3-33/D5\|5-12\|RF3/JH6 | | 32512 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GYSCYDYY--------------YYYYGMDV | WGQGTTVTVSS |
| IPS:4 37102 | 21-225_211E5 | VH3\|3-33/D5\|5-12\|RF3/JH6 | | QVQLVES-GGGVVQPGRSLRLSCAASG-FTFR | N-----YGMH | WVRQAPGKGLEWVS | IIWFD---GSDQYYADSVKG | RFTISRDNSKNTLYLQMNSLRAEDTAVYYCAR | GLSVYY----------------YGMGV | WGQGTTVTVSS |
| IPS:4 37164 | 21-225_217C6 | VH3\|3-33/D5\|5-12\|RF3/JH6 | | QVQLVES-GGGVVQPGRSLRLSCAASG-FTFS | N-----YGMH | WVRQAPGKGLEWMA | IIWFD---GSDEYYADSVKG | RFTISRDNSKNTMYLQMNSLRAEDTAVYYCAR | GLSVYY----------------YGMDV | WGQGTTVTVSS |
| IPS:4 37166 | 21-225_217G11 | VH3\|3-33/D5\|5-12\|RF3/JH6 | | QVQLVES-GGGVVQPGRSLRLSCAASG-FTFR | N-----YGMH | WVRQAPGKGLEWVS | IIWFD---GSDQYYADSVKG | RFTISRDNSKNTLYLQMNSLRAEDTAVYYCAR | GLSVYY----------------YGMDV | WGQGTTVTVSS |
| IPS:4 37170 | 21-225_218E5 | VH3\|3-33/D5\|5-12\|RF3/JH6 | | QVQLVES-GGGVVQPGRSLRLSCAASG-FTFS | N-----YGMH | WVRQAPGKGLEWMA | IIWFD---GSDEYYADSVKG | RFTISRDNSKNTLYLQMNSLRAEDTAVYYCAR | GLSVYY----------------YGMDV | WGQGTTVTVSS |
| | | Germline | | H_FR1 | H_CDR1 | H_FR2 | H_CDR2 | H_FR3 | H_CDR3 | H_FR4 |
| | VH4\|4-30.1/D6\|6-19\|RF2/JH6 | | 32513 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | GIAVAGYY--------------YYYGMDV | WGQGTTVTVSS |

3015

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:4 37108 | 21-225_211C9 | VH4\|4-30.1/D6\|6-19\|RF2/JH6 | | QVQLQES-GPGLVKPSQILSLTCTVSG-GSIR | SG---GDYWS | WIRQHPGK GLEWIG | YIYY---- SGSTYYNPS LKS | RVTILLDISKNQFSLKLSSV TVADTAVYYCAR | DSAVY-----------------------NMDV | WGQGT TVTVS S |
| iPS:4 37110 | 21-225_211E9 | VH4\|4-30.1/D6\|6-19\|RF2/JH6 | | QVQLQES-GPGLVKPSQILSLTCTVSG-GSIS | SG---GDYWS | WIRQHPGK GLEWIG | YIYY---- TGSNYYNPS LKS | RVTISVDTSKNQFSLNLISV TAADTAVYYCAR | DSAVY-----------------GMDV | WGQGT TVTVS S |
| iPS:4 37120 | 21-225_212A9 | VH4\|4-30.1/D6\|6-19\|RF2/JH6 | | QVQLQES-GPGLVKPSQILSLTCSVSG-GSIR | SG---GDYWS | WIRQHPGK GLEWIG | YMYY---- SGSTYYNPS LKS | RVTISVDTSKNQFSLKLSSV TVADTAVYYCAR | DSAVY-----------------GMDV | WGQGT TVTVS S |
| iPS:4 37132 | 21-225_213F5 | VH4\|4-30.1/D6\|6-19\|RF2/JH6 | | QVQLQES-GPGLVKPSQILSLTCTVSG-GSIR | SG---GDYWS | WIRQHPGK GLEWIG | YIYY---- SGSTYYNPS LKS | RVTISLDTSKNQFSLKLSSV TVADTAVYYCAR | DSAVY-----------------NMDV | WGQGT TVTVS S |
| iPS:4 37142 | 21-225_215A3 | VH4\|4-30.1/D6\|6-19\|RF2/JH6 | | QVQLQES-GPGLVKPSQILSLTCTVSG-GSTS | SG---GDYWS | WIRQHPGK GLEWIG | YIYY---- TGSNYYNPS LKS | RVTISVDTSKNQFSLKVISV TAADTAVYYCAR | DSAVY-----------------GMDV | WGQGT TVTVS S |
| iPS:4 37148 | 21-225_215H3 | VH4\|4-30.1/D6\|6-19\|RF2/JH6 | | QVQLQES-GPGLVKPSQILSLTCSVSG-GSIR | SG---GDYWS | WIRQHPGK GLEWIG | YMYY---- SGSTYYNPS LKS | RVTISVDTSKNQFSLKLSSV TVADTAVYYCAR | DSAVY-----------------GMDV | WGQGT TVTVS S |
| iPS:4 37154 | 21-225_216A7 | VH4\|4-30.1/D6\|6-19\|RF2/JH6 | | QVQLQES-GPGLVKPSQILSLTCTVSG-GSIR | SG---GDYWS | WIRQHPGK GLEWIG | YMYY---- SGSTYYNPS LKS | RVTISVDTSKNQFSLKLSSV TVADTAVYYCAR | DSAVY-----------------GMDV | WGQGT TVTVS S |
| | | **Germline** | | **H_FR1** | **H_CDR1** | **H_FR2** | **H_CDR2** | **H_FR3** | **H_CDR3** | **H_FR4** |
| | VH3\|3-30.3/D7\|7-27\|RF2/JH4 | | 32514 & 32515 | QVQLVES-GGGVVQPGRSLRLSCAASG-FTFS | S-----YAMH | WVRQAPGK GLEWVA | VISYD--- GSNKYYADS VKG | RFTISRDNSKNTLYLQMNSL RAEDTAVYYCAR | *LGY-----------------FDY | WGQGT LVTVS S |
| iPS:4 37160 | 21-225_216B12 | VH3\|3-30.3/D7\|7-27\|RF2/JH4 | | QVQLVES-GGGVVQPGRSLRLSCAASG-FTFS | S-----YAMH | WVRQAPGK GLEWVA | VIWYD--- GSNKYYADS VKG | RFTISRDNSKNTLYLQMNSL RAEDTAVYYCAR | DLGLG-----------YFFDY | WGQGT LVTVS S |
| | | **Germline** | | **H_FR1** | **H_CDR1** | **H_FR2** | **H_CDR2** | **H_FR3** | **H_CDR3** | **H_FR4** |
| | VH6\|6-01/D2\|2-21\|RF2/JH5 | | 32516 | QVQLQQS-GPGLVKPSQTLSLTCAISG-DSVS | SN---SAAWN | WIRQSPSR GLEWLG | RTYYR-- SKWYNDYAV SVKS | RITINPDTSKNQFSLQLNSV TPEDTAVYYCAR | AYCGGDC--------YSNWFDP | WGQGT LVTVS S |
| iPS:4 37186 | 21-225_224H2 | VH6\|6-01/D2\|2-21\|RF2/JH5 | | QVQLQQS-GPGLVKPSQTLSLTCDISG-DSVS | SN---SAAWN | WIRQSPSR GLEWLG | RTYYR-- SKWYNDYAV SVKS | RVTINPDTSKNQFSLQLNSV TPEDTAVYYCAR | EGGLGYCSST-----SCYGGWFDP | WGQGT LVTVS S |
| | | **Germline** | | **H_FR1** | **H_CDR1** | **H_FR2** | **H_CDR2** | **H_FR3** | **H_CDR3** | **H_FR4** |
| | VH1\|1-02/D4\|4-17\|RF2/JH6 | | 32517 | QVQLVQS-GAEVKKPGASVKVSCKASG-YTFT | G-----YYMH | WVRQAPGQ GLEWMG | WINPN--- SGGTNYAQK FQG | RVTMTRDTSISTAYMELSRL RSDDTAVYYCAR | DYGDYYY---------YYYGMDV | WGQGT TVTVS S |

| iPS ID | Sample | Germline / VH | No. | H_FR1 | H_CDR1 | H_FR2 | H_CDR2 | H_FR3 | H_CDR3 | H_FR4 |
|---|---|---|---|---|---|---|---|---|---|---|
| iPS:4 37188 | 21-225_224B11 | VH1|1-02/D4|4-17|RF2/JH6 | | QVHLVQS-GAEVKKPGASVKVSCKASG-YTFT | G-----YYIH | WVRQAPGQGLEWMG | WINPK---NGGTNYAQKFQG | RVTMTRDASISITYMELSRLRSDDTAVYYCAR | GAFDYFY-----------------YYAMDV | WGHGTTVTVSS |
| iPS:4 37198 | 21-225_226F8 | VH1|1-02/D4|4-17|RF2/JH6 | | QVQLVQS-GAEVKKPGASVKVSCKASG-YTFT | G-----YYIH | WVRQAPGQGLEWMG | WINPN---SGGTNYARKFQG | RVTMTRDTSISTAYMELSRLRSDDTAVYYCAR | GAFDYYY-----------------YYALDV | WGQGTTVTVSS |
| iPS:4 37202 | 21-225_227D3 | VH1|1-02/D4|4-17|RF2/JH6 | | QVQLVQS-GAEVKKPGASVKVSCKASG-YTFT | G-----YYMH | WVRQAPGQGLERMG | WINPK---SGGTNFAQKFQG | RVTMTRDTSISTAYMELSRLRSDDTAVYYCAR | GAFDYFY-----------------YYGMDV | WGQGTTVTVSS |
| iPS:4 37208 | 21-225_227C10 | VH1|1-02/D4|4-17|RF2/JH6 | | QVQLVQS-GAEVKKPGASVKVSCKASG-YTFT | G-----YYMH | WVRQAPGQGLERMG | WINPK---SGGTNYAQKFQG | RVTMTRDTSISTAYMELSRLRSDDTAVYYCAR | GAFDYFY-----------------YYGMDV | WGQGTTVTVSS |
| **Germline** | | | | **H_FR1** | **H_CDR1** | **H_FR2** | **H_CDR2** | **H_FR3** | **H_CDR3** | **H_FR4** |
| | **VH3|3-33/D2|2-2|RF2/JH6** | | 32518 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GYCSSTSCYT-----------YYYYYGMDV | WGQGTTVTVSS |
| iPS:4 37192 | 21-225_225E9 | VH3|3-33/D2|2-2|RF2/JH6 | | QVQLVES-GGGVVQPGRSLRLSCEASG-FIFS | S-----YGMH | WVRQAPGKGLEWVA | VMWYD---GGNKDYADSVKG | RFTISRDNSKNTLYLQMNSLRAEDTAVYYCAR | DREYCTSTSC----------PYYYYYGMDV | WGQGTTVTVSS |
| **Germline** | | | | **H_FR1** | **H_CDR1** | **H_FR2** | **H_CDR2** | **H_FR3** | **H_CDR3** | **H_FR4** |
| | **VH3|3-23/D2|2-21|RF2/JH6** | | 32519 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | AYCGGDCYS-----------YYYYYGMDV | WGQGTTVTVSS |
| iPS:4 37204 | 21-225_227E5 | VH3|3-23/D2|2-21|RF2/JH6 | | EVQLLES-GGGLVQPEGSLRLSCAASG-FTFS | S-----YAMS | WVRQAPGKGLEWVS | AISGS---GGSTYYADSVKG | RFTISRDNSKNTLCLQMNSLRAEDTAVYYCAK | EYCGGDCYSP-----------YYYYYGMDV | WGQGTTVTVSS |
| iPS:3 93196 | 21-225_16G8 | VH3|3-23/D2|2-21|RF2/JH6 | | EVQLLES-GGGLVQPEGSLRLSCAASG-FTFS | S-----YAMS | WVRQAPGKGLEWVS | GISGS---GGSTYYADSVKG | RFTISRDNSKNTLCLHMNSLRAEDTAVYYCAK | EYCGGDCYSP-----------YYYYYGMDV | WGQGTTVTVSS |
| iPS:3 93202 | 21-225_6B4 | VH3|3-23/D2|2-21|RF2/JH6 | | EVQLLES-GGGLVQPEGSLRLSCAASG-FTFS | S-----YAMS | WVRQAPGKGLEWVS | AISGS---GSSTYYADSVKG | RFTISRDNSKNTLCLQMNSLRAEDTAVYYCAK | EYCGGDCYSP-----------YYYYYGMDV | WGQGTTVTVSS |
| iPS:3 93345 | 21-225_5G7 | VH3|3-23/D2|2-21|RF2/JH6 | | EVQLLES-GGGLVQPEGSLRLSCAASG-FTFS | S-----YAMS | WVRQAPGKGLEWVS | AISGS---GGSTYYADSVKG | RFTISRDNSKNTLCLQMNSLRAEDTAVYYCAK | EYCGGDCYSP-----------YYYYYGMDV | WGQGTTVTVSS |
| **Germline** | | | | **H_FR1** | **H_CDR1** | **H_FR2** | **H_CDR2** | **H_FR3** | **H_CDR3** | **H_FR4** |
| | **VH2|2-05/D6|6-13|RF3/JH4** | | 32520 & 32521 | QITLKES-GPTLVKPTQTLILTCTFSG-FSLS | TS---GVGVG | WIRQPPGKALEWLA | LIYW----NDDKRYSPSLKS | RLTITKDTSKNQVVLTMTNMDPVDTATYYCAH | V*QQL-----------------VYFDY | WGQGTLVTVSS |

| iPS:4 37210 | 21-225_227E12 | VH2\|2-05\|D6\|6-13\|RF3/JH4 | | QITLKES-GPTLVKPTQILTL TCTFSG-FSLS | TS---GVGVG | WIRQPPGK ALECLS | LIYW---- NDDKVYSPS LKS | RLTITKYISKNQVVLTMTNM DPVDTATYYCAH | RGQQL----------- ----------ALDY | WGQGT LVTVS S |
| iPS:3 92587 | 21-225_18G5 | VH2\|2-05\|D6\|6-13\|RF3/JH4 | | QITLKES-GPTLVKPTQILTL TCTFSG-FSLS | TS---GVGVG | WIRQPPGK ALECLS | LIYW---- NDDKVYSPS LKS | RLTITKYISKNQVVLTMTNM DPVDTATYYCAH | RGQQL----------- ----------ALDY | WGQGT LVTVS S |
| iPS:3 98504 | 21-225_23D7 | VH2\|2-05\|D6\|6-13\|RF3/JH4 | | QITLKES-GPTLVKPTQILTL TCTFSG-FSLS | TS---GVGVG | WIRQPPGK ALECLS | LIYW---- NNDKVYSPS LKS | RLTITKYISKNQVVLTMSNM DPVDTATYYCAH | RGQQL----------- ----------ALDY | WGQGT LVTVS S |
| | Germline | | | H_FR1 | H_CDR1 | H_FR2 | H_CDR2 | H_FR3 | H_CDR3 | H_FR4 |
| | VH3\|3-21\|D4\|4-11\|RF2/JH6 | | 32522 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | DYSNYYY-------- -------YYYGMDV | WGQGT TVTVS S |
| iPS:4 37226 | 21-225_57C2 | VH3\|3-21\|D4\|4-11\|RF2/JH6 | | EVQLVES-GGGLVKPGGSLRL SCAASG-FTFS | S-----FGMN | WVRQAPGK GLEWVS | SISSS--- TGYIYNADS VKG | RFTISRDNAKNSLYLQMNSL RAEDTAVYYCAR | TYSG----------- ----------SLDV | WGQGT TVTVS S |
| | Germline | | | H_FR1 | H_CDR1 | H_FR2 | H_CDR2 | H_FR3 | H_CDR3 | H_FR4 |
| | VH3\|3-21\|D5\|5-18\|RF3/JH5 | | 32523 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | GYSYGY---------- ------NWFDP | WGQGT LVTVS S |
| iPS:4 37230 | 21-225_62H10 | VH3\|3-21\|D5\|5-18\|RF3/JH5 | | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | GGSR----------- ----------GFDP | WGQGT LVTVS S |
| iPS:4 48906 | 21-225_72G9 | VH3\|3-21\|D5\|5-18\|RF3/JH5 | | EVQLVES-GGGLVKPGGSLRL SCAASG-FTFS | S-----YSMN | WVRQAPGK GLEWVS | SISGS--- SSYIYYADS VKG | RFTISRDNAKNSLYLQMNSL RAEDTAVYFCAR | GGSR----------- ----------GFDP | WGQGT LVTVS S |
| | Germline | | | H_FR1 | H_CDR1 | H_FR2 | H_CDR2 | H_FR3 | H_CDR3 | H_FR4 |
| | VH1\|1-02\|D1\|1-26\|RF1/JH4 | | 32524 | QVQLVQS-GAEVKKPGASVKV SCKASG-YTFT | C----YYMH | WVRQAPGQ GLEWMG | WINPN--- SGGTNYAQK FQG | RVTMTRDTSISTAYMELSRL RSDDTAVYYCAR | GIVGA---------- ----------TYFDY | WCQGT LVTVS S |
| iPS:4 37260 | 21-225_170D1 | VH1\|1-02\|D1\|1-26\|RF1/JH4 | | QVQLVQS-GAEVKKPGASVKV SCKASG-YTFT | G-----YFMH | WVRQAPGQ GLEWMG | WIKPK--- SGGTNCAQK FQG | RVTMTRDTSSSTAYMELSRL TSDDTAVYYCAR | GGATVIT--------- --------WGVFDY | WGQGT LVTVS S |
| | Germline | | | H_FR1 | H_CDR1 | H_FR2 | H_CDR2 | H_FR3 | H_CDR3 | H_FR4 |
| | VH3\|3-33\|D2\|2-21\|RF2/JH6 | | 32525 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | AYCGGDCYS------- ------YYYYYGMDV | WGQGT TVTVS S |
| iPS:4 37268 | 21-225_177D2 | VH3\|3-33\|D2\|2-21\|RF2/JH6 | | QVQLVES-GGGVVQPGRSLRL SCAASG-FTFS | S-----YGMD | WVRQAPGK GLEWVA | IIWFD--- GSNKYYADS VKG | RFTISRDNSKNTLYLQMNSL RAEDTAVYYCAR | AYCGGDCYFP------ -----HLHYYGMDV | WGQGT TVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-30.1/D4\|4-17\|RF2/JH4 | 32526 | QVQLQES-GPG_VKPSQILSL TCTVSG-GSIS | SG---GYYWS | WIRQHPGK GLEWIG | YIYY---- SGSTYYNPS LKS | RVTISVDISKNQFSLKLSSV TAADTAVYYCAR | DYGDY--------- ----------YFDY | WGQGT LVTVS S |
| IPS:4 37294 | 21-225_216D5 | VH4\|4-30.1/D4\|4-17\|RF2/JH4 | QVQLQES-GPGLVKPSQILSL TCTVSG-GSIS | SG---GYYWS | WIRQHPGK GLEWIG | YIYY---- SGSTYYNPS LKS | RVTISVDISKNQFSLKLNSV TAADTAVYFCAR | DSPDR--------- --------GFDY | WGQGT LVTVS S |
| | | Germline | H_FR1 | H_CDR1 | H_FR2 | H_CDR2 | H_FR3 | H_CDR3 | H_FR4 |
| | VH3\|3-30.3/D5\|5-24\|RF3/JH6 | 32527 | QVQLVES-GGGVVQPGRSLRL SCAASG-FTFS | S------YAMH | WVRQAPGK GLEWVA | VISYD--- GSNKYYADS | RFTISRDNSKNTLYLQMNSL RAEDTAVYYCAR | RDGYNYYY--------- YYYGMDV | WGQGT TVTVS S |
| IPS:4 37302 | 21-225_225B11 | VH3\|3-30.3/D5\|5-24\|RF3/JH6 | QVQLVES-GGGVVQPGRSLRL SCAASG-FTFS | S-----YGMH | WVRQAPGK GLEWVT | IISYS--- GSNKYYADS VKG | RFTISRDNSKNTLYLQMNSL RAEDTAVYYCAR | RGYSYG---------- --------GYGMDV | WGQGT TVTVS S |
| IPS:4 51102 | 21-225_45F6 | VH3\|3-30.3/D5\|5-24\|RF3/JH6 | QVQLVES-GGGVVQPGRSLRL SCAASG-FTFS | Y-----YGLH | WVRQAPGK GLEWVA | VISYD--- GSNKYYADS VKG | RFTISRDNSKNTLYLQMNNL RAEDTAVFYCAR | EDRYCSGTSC------ ----PYYYYYGMDV | WGQGT TVTVS S |
| IPS:3 92868 | 21-225_24D6 | VH3\|3-30.3/D5\|5-24\|RF3/JH6 | QVQLVES-GGGVVQPGRSLRL SCAASG-FTFS | S-----YGMH | WVRQVPGK GLEWVA | IISYA--- GSNKSYADS VKG | RFTISRDNSKNTLYLQMNSL RAEDTAVYYCVR | RGYSYG---------- --------GYGMDV | WGQGA TVTVS S |
| IPS:3 93910 | 21-225_15F10 | VH3\|3-30.3/D5\|5-24\|RF3/JH6 | QVQLVES-GGGVVQPGRSLRL SCAASG-FTFS | S-----YGMH | WVRQAPGK GLEWVS | VISYG--- GSNNYYADS VKG | RFTISRDNSKNTLYLQMNSL RAEDTAVYYCVR | RGYSYG---------- --------GYGMDV | WGQGT TVTVS S |
| IPS:3 94000 | 21-225_11A2 | VH3\|3-30.3/D5\|5-24\|RF3/JH6 | QVQLVES-GGGVVQPGRSLRL SCAASG-FTFS | S-----YGMH | WVRQAPGK GLEWVA | VISYG--- GSNKDSADS VKG | RFIISRDNSKNTLYLQMNSL RAEDTAVYYCVR | RGYSYG---------- --------GYGMDV | WGQGT TVTVS S |
| IPS:3 94004 | 21-225_13A1 | VH3\|3-30.3/D5\|5-24\|RF3/JH6 | QVQLVES-GGGVVQPGRSLRL SCAASG-FTFS | S-----YGMH | WVRQAPGK GLEWVA | VISYA--- GTNQYYADS VKG | RFTISRDNSKNTLYLQMNSL RAEDTAVYYCVR | RGYSYG---------- --------GYGMDV | WGQGT TVTVS S |
| IPS:3 94006 | 21-225_15C2 | VH3\|3-30.3/D5\|5-24\|RF3/JH6 | QVQLVES-GGGVVQPGRSLRL SCAASG-FTFS | S-----YGMH | WVRQAPGK GLEWVS | IISYG--- GRNNHYADS VKG | RFTISRDNSKNTLYLQMNSL RAEDTAVYYCVR | RGYSYG---------- --------GYGMDV | WGQGT TVTVS S |
| IPS:3 94029 | 21-225_1B12 | VH3\|3-30.3/D5\|5-24\|RF3/JH6 | QVQLVES-GGGVVQPGRSLRL SCAASG-FTFS | S-----YGMH | WVRQAPGK GLEWVA | IISYA--- GSNKSYADS VKG | RFTISRDNSKNMLYLQMNSL RAEDTAVYYCVR | RGYSYG---------- --------GYGMDV | WGQGA TVTVS S |
| IPS:3 94047 | 21-225_5E6 | VH3\|3-30.3/D5\|5-24\|RF3/JH6 | QVQLVES-GGGVVQPGRSLRL SCAASG-FTFS | S-----YGMH | WVRQAPGK GLEWVA | IISYV--- GNNKYYADS VKG | RFTISRDNSKNTLYLQMNSL RVEDTAVYYCAR | RGYSYG---------- --------GYGMDV | WGQGT TVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 94081 | 21-225_16B3 | VH3\|3-30.3/D5\|5-24\|RF3/JH6 | | QVQLVES-GGGVVQPGRSLRL SCVASG-FTFS | S-----YGMH | WVRQAPGK GLEWVA | VISYA--- GINKSYADS VKG | RFTISRDNSXNTLYLQMNSL RAEDTAVYYCVR | RGYSYG---------- --------GYGMDV | WGQGA TVTVS S |
| | Germline | | | H_FR1 | H_CDR1 | H_FR2 | H_CDR2 | H_FR3 | H_CDR3 | H_FR4 |
| | VH4\|4-61/D3\|3-9\|RF1/JH4 | | 32528 & 32529 | QVQLQES-GPGLVKPSETLSL TCTVSG-GSVS | SG---SYYWS | WIRQPPGK GLEWIG | YIYY---- SGSTNYNPS LKS | RVTISVDTSKNQFSLKLSSV TAADTAVYYCAR | VLRYFDW--------- -------LL*YFDY | WGQGT LVTVS S |
| iPS:4 51118 | 21-225_191C8 | VH4\|4-61/D3\|3-9\|RF1/JH4 | | QVQLQES-GPGLVKPSEILSL TCTVSS-GSVS | SG---GYYWS | WIRQPPGK GLEWIG | YIYY---- SGTTIYNPS LKS | RVTISVDISKNQFSLKLTSV TVADTAVYYCAR | DIFCFDG--------- -------CGYFFDS | WGQGT LVTVS S |
| | Germline | | | H_FR1 | H_CDR1 | H_FR2 | H_CDR2 | H_FR3 | H_CDR3 | H_FR4 |
| | VH2\|2-05/D4\|4-11\|RF3/JH4 | | 32530 | QITLKES-GPTLVKPTQTLTL TCTFSG-FSLS | TS---GVGVG | WIRQPPGK ALEWLA | LIYW---- NDDKRYSPS LKS | RLTITKDTSKNQVVLTMTNM DPVDTATYYCAH | TTVT------------ ----------YFDY | WGQGT LVTVS S |
| iPS:3 92573 | 21-225_15G2 | VH2\|2-05/D4\|4-11\|RF3/JH4 | | QITLKES-GPTLVKPTQTLTL TCTFSG-FSLS | TS---GVGVG | WIRQPPGK ALEWLA | LIYW---- NDDKRYSPS LKS | RLTITKDTSKNQVVLTMTNM DPVDTATYYCAD | TGVSC----------- ---------CYFHY | WGQGT LVTVS S |
| | Germline | | | H_FR1 | H_CDR1 | H_FR2 | H_CDR2 | H_FR3 | H_CDR3 | H_FR4 |
| | VH1\|1-02/D2\|2-2\|RF2/JH6 | | 32531 | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | GYCSSTSCYT------ -----YYYYYGMDV | WGQGT TVTVS S |
| iPS:3 92585 | 21-225_14H11 | VH1\|1-02/D2\|2-2\|RF2/JH6 | | QVQLVQS-GAEVKKPGASVKV SCQGSG-YTFT | G-----HYMC | WVRQAPGQ GLEWMG | WINPN--- SGGTNYAQK FQG | RVTMTRDTSISTAYMELSRL RSDDTAVYYCAA | GYCSSSSCYL------ -----QPGYYGMDV | WGQGT TVTVS S |
| iPS:3 93186 | 21-225_27D9 | VH1\|1-02/D2\|2-2\|RF2/JH6 | | QVQLVQS-GAEVKKPGASVKV SCKASG-YTFT | G-----YYMH | WVRQAPGQ GLEWMG | WINPN--- SGGTKYAQK FQG | RVTMTRDTSISTAYMELNRL RSDDTAVYYCAR | ERCSTISCYL------ ----GITGYYGMDV | WGQGT TVTVS S |
| iPS:3 93234 | 21-225_26C10 | VH1\|1-02/D2\|2-2\|RF2/JH6 | | QVQLVQS-GAEVKKPGASVKV SCKASG-YTFT | G-----YYVH | WVRQAPGQ GLEWMG | WINPN--- SGGTNYAQK FQG | RVTMTRDTSISTAYMELSRL RSDDTAVYYCAR | ERCSTISCYL------ ----GITGYYGMDV | WGQGT TVTVS S |
| | Germline | | | H_FR1 | H_CDR1 | H_FR2 | H_CDR2 | H_FR3 | H_CDR3 | H_FR4 |
| | VH3\|3-23/D5\|5-12\|RF3/JH6 | | 32532 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | GYSGYDYY-------- ------YYYYGMDV | WGQGT TVTVS S |
| iPS:3 92596 | 21-225_12D8 | VH3\|3-23/D5\|5-12\|RF3/JH6 | | EVQLLES-GGGLVQPGGSLRL SCAASG-FTFS | S-----YVMS | WVRQAPGK GLEWVS | TISVG--- GGSTYYADS VKG | RFTISRDNSKTTLYLQMNSL RAEDTAVYYCAK | WGRGYSYE-------- -------YYYGMDV | WGQGT TVTVS S |
| iPS:3 92942 | 21-225_30E9 | VH3\|3-23/D5\|5-12\|RF3/JH6 | | EVQVLES-GGGLVQPGGSLRL SCAASG-FTFS | S-----CAMN | WVRQAPGK GLEWVS | AISGR--- GGSTFYADS VKG | RFTISRDNSKNTLYLQMNSL RAEDTAVYYCAK | GELLEDY--------- -------YYYGMDV | WGQGT TVTVS S |

| | | | | | | | | | | |
|---|---|---|---|---|---|---|---|---|---|---|
| iPS:3 92944 | 21-225_31H5 | VH3\|3-23/D5\|5-12\|RF3/J H6 | | EVQLLES-GGGLVQPGGSLRL SCAASG-FTFS | S-----YAMS | WVRQAPGK GLEWVS | AISGR---GGSIFHADS VKG | RFTISRDNSKNTLYLQMNSL RAEDTAVYYCVK | GELLEDY-----------------YFYGMDV | WGQGT TVTVS S |
| iPS:3 92964 | 21-225_31A8 | VH3\|3-23/D5\|5-12\|RF3/J H6 | | EVQLLES-GGGLVQPGGSLRL SCAASG-FTFS | S-----YAMS | WVRQAPGK GLEWVS | AISGR---GGSTFHADS VKG | RFTISRDNSKNTLYLQMNSL RDEDTAVYYCVK | GELLEDY-----------------YFYGMDV | WGQGT TVTVS S |
| iPS:3 92982 | 21-225_30D1 | VH3\|3-23/D5\|5-12\|RF3/J H6 | | EVQVLES-GGGLVQPGGSLRL SCAASG-FTFS | S-----YAMS | WVRQAPGK GLEWVS | AISGR---GGSTFHADS VKG | RFTISRDNSKNTLYLQMNSL RAEDTAVYYCVK | GELLEDY-----------------YFYGLDV | WGQGT TVTVS S |
| iPS:3 92986 | 21-225_31B8 | VH3\|3-23/D5\|5-12\|RF3/J H6 | | EVQLLES-GGGLVQPGGSLRL SCAASG-FTFS | S-----YAMS | WVRQAPCK GLEWVS | AISGR---GGSTFHADS VKG | RFTISRDNSKNTLYLQLNSL RADDTAVYYCVK | GELLEDY-----------------YFYGMDV | WGQGT TVTVS S |
| iPS:3 93004 | 21-225_30G11 | VH3\|3-23/D5\|5-12\|RF3/J H6 | | EVQLLES-GGGLVQPGGSLRL SCAASG-FTFS | S-----YAMS | WVRQAPGK GLEWVS | AISGR---GGSTFNADS VKG | RFTISRDNSKTTLYLQMNSL RAEDTAVYYCVK | GELLEDY-----------------YFYGMDV | WGQGT TVTVS S |
| iPS:3 93040 | 21-225_30E3 | VH3\|3-23/D5\|5-12\|RF3/J H6 | | EVQVLES-GGGLVQPGGSLRL SCAASG-FTFS | S-----YAMN | WVRQAPGK GLEWVS | AISGR---GGSTFYADS EKG | RFTISRDNSKNTLYLQMNSL RAEDTALYYCAK | GELLEDY-----------------YYYGMDV | WGQGT TVTVS S |
| iPS:3 93058 | 21-225_31H3 | VH3\|3-23/D5\|5-12\|RF3/J H6 | | EVQVLES-GGGLVQPGGSLRL SCAASG-FTFS | S-----YAMN | WVRQAPGK GLEWVS | AISGR---GGNTFYADS VKG | RFTISRDNSKNTLYLQMNSL RAEDTAVYYCAK | GELLEDY-----------------YYYGMDV | WGQGT TVTVS S |
| iPS:3 93060 | 21-225_32G12 | VH3\|3-23/D5\|5-12\|RF3/J H6 | | EVQLLES-GGGLLQPGGSLRL SCAASG-FTFN | S-----YAMS | WVRQAPGK GLEWVS | SISGR---GGSTFHADS VKG | RFTISRDNSRNTLYLQMNSL RAEDTAVYYCVK | GELLEDY-----------------YFYGMDV | WGQGT TVTVS S |
| iPS:3 93068 | 21-225_34G9 | VH3\|3-23/D5\|5-12\|RF3/J H6 | | EVQLVES-GGGLVQPGGSLRL SCAASG-FTFS | S-----YAMS | WVRQAPGK GLEWVS | AISGR---GGSTFHADS VKG | RFTISRDNSKNTLYLQMNSL RAEDTAVYYCVK | GELLEDY-----------------YFYGMDV | WGQGT TVTVS S |
| iPS:3 93072 | 21-225_36C5 | VH3\|3-23/D5\|5-12\|RF3/J H6 | | EVQLVES-GGGLVQPGGSLRL SCAASG-FTFS | S-----YAMN | WVRQAPGK GLEWVS | AISGR---GGSTFHADS VKG | RFTISRDNSKNTLYLQMNSL RAEDTAVYYCVK | GELLEDY-----------------YFYGMDV | WGQGT TVTVS S |
| iPS:3 93076 | 21-225_33A4 | VH3\|3-23/D5\|5-12\|RF3/J H6 | | EVQLLES-GGGLVQSGGSLRL SCEASG-FIFS | S-----YAMN | WVRQVPGK GLEWVS | AISRR---GGSTFYADS VKG | RFTISRDNSKNTLYLQMNSL RAEDTAVYFCAK | GELLEDY-----------------SYYGIDV | WGQGT TVTVS S |
| iPS:3 93102 | 21-225_33F1 | VH3\|3-23/D5\|5-12\|RF3/J H6 | | EVQLLES-GGGLLQPGGSLRL SCAASG-FTFS | S-----YAMS | WVRQAPGK GLEWVS | SISGR---GGSTFHADS VKG | RFTISRDNSKNTLYLQMNSL RAEDTAVYYCVK | GELLEDY-----------------YFYGMDV | WGQGT TVTVS S |

| | | | | | | | | | | |
|---|---|---|---|---|---|---|---|---|---|---|
| iPS:3 93104 | 21-225_33A7 | VH3\|3-23/D5\|5-12\|RF3/JH6 | | EVQLLES-GGGLVQPGGSLRLSCAASG-FTFS | S-----CAMS | WVRQAPGKGLEWVS | AISGR---GGSTFHADSVKG | RFTISRDNSKNTLYLQMNSLRAEDTAVYYCVK | GELLEDY----------------YFYGMDV | WGQGTTVTVSS |
| iPS:3 93106 | 21-225_34A6 | VH3\|3-23/D5\|5-12\|RF3/JH6 | | EVQLLES-GGGLVQPGGSLRLSCAASG-FTFN | N-----YAMN | WVRQAPGKGLEWVS | AISRR---GGSTFYADSVKG | RFTISRDNSKNTLYLQMNSLRAEDTAVYYCAK | GELLEDY----------------YYFAMDV | WGQGTTVTVSS |
| iPS:3 93110 | 21-225_35B7 | VH3\|3-23/D5\|5-12\|RF3/JH6 | | EVQLLES-GGGLVQPGGSLRLSCAASG-FTFS | S-----YAMS | WVRQAPGKGLEWVS | AISGR---GGSTFHADSVKG | RFTISRDNSKNTLYLQMNSLRAEDTAVYYCVK | GELLEDY----------------YFYGMDV | WGQGATVTVSS |
| iPS:3 93118 | 21-225_34H11 | VH3\|3-23/D5\|5-12\|RF3/JH6 | | EVQLLES-GGGLVQPGGSLRLSCAASG-FTFS | S-----YAMN | WVRQAPGEGLEWVS | AISGR---GGSTFYADSVKG | RFTISRDNSKNTLFLQMNSLRAEDTAVYYCAK | GELLEDY----------------YYYGMDV | WGQGTTVTVSS |
| iPS:3 93124 | 21-225_33G7 | VH3\|3-23/D5\|5-12\|RF3/JH6 | | EVQLLES-GGGLVQPGGSLRLSCAASG-FTFS | N-----YAMS | WVRQAPGKGLEWVS | AISRR---GGSTFYADSVKG | QFTISRDNSKNTLYLQMNSLRAEDTAVYYCAK | GELLEDY----------------SYYGMDV | WGQGTTVTVSS |
| iPS:3 93126 | 21-225_35D1 | VH3\|3-23/D5\|5-12\|RF3/JH6 | | EVQLLES-GGGLVQPGGSLRLSCAASG-FTFS | S-----YAMS | WVRQAPGKGLEWVS | AISGR---GGSTFHADSVKG | RFTISRDNSXNTLYLQMNSLRAEDTAVYYCVK | GELLEDY----------------YFYGMDV | WGQGATVTVSS |
| iPS:3 93128 | 21-225_35F11 | VH3\|3-23/D5\|5-12\|RF3/JH6 | | EVQLLES-GGGLVQPGGSLRLSCAASG-FTFS | S-----YAMS | WVRQAPGKGLEWVS | AISGR---GGSTFHADSMKG | RFTISRDNSKNTLYLQMNSLRAEDTAVYYCVK | GELLEDY----------------YFYGMDV | WGQGTTVTVSS |
| iPS:3 93146 | 21-225_34G8 | VH3\|3-23/D5\|5-12\|RF3/JH6 | | EVQLLES-GGGLVQPGGSLRLSCAASG-FTFS | S-----YAMS | WVRQAPGKGLEWVS | AISGR---GGSTFHADSVKG | RFTISRDNSKNTLYLQINSLRAEDTAVYYCVK | GELLEDY----------------YFYGMDV | WGQGTTVTVSS |
| iPS:3 93150 | 21-225_36A5 | VH3\|3-23/D5\|5-12\|RF3/JH6 | | EVQLLES-GGGLVQPGGSLRLSCAASG-FTFN | N-----YAMN | WVRQAPGKGLEWVS | AISRR---GGNTFYADSVKG | RFTISRDNSKNTLYLQMNSLRAEDTAVYYCAK | GELLEDY----------------YYYAMDV | WGQGTTVTVSS |
| iPS:3 93180 | 21-225_4G12 | VH3\|3-23/D5\|5-12\|RF3/JH6 | | EVQLLES-GGGLVQPGGSLRLSCAASG-FTFS | S-----YAMS | WVRQAPGKGLEWVS | TLSGR---GGSTYYADSVKG | RSTISRDNSKNTLYLQMSSLRAEDTAVYYCAK | WGRGYSYE--------------YYYGMDV | WGQGTTVTVSS |
| iPS:3 93232 | 21-225_17F12 | VH3\|3-23/D5\|5-12\|RF3/JH6 | | EVQLLES-GGGLVQPGGSLRLSCAASG-FTFS | S-----YAMS | WVRQAPGKGLEWVS | AISGG---GGSTYYADSVKG | RVTISRDNSKNTLYLQMNSLRAEDTAVYYCAK | WGRGYNYE--------------YYYGMDV | WGQGTTVTVSS |
| iPS:3 98494 | 21-225_21H4 | VH3\|3-23/D5\|5-12\|RF3/JH6 | | EVQLLES-GGGLVQPGGSLRLSCAASG-FTFS | S-----YAMS | WVRQAPGKGLEWVS | ALSGR---GGSTYYADSVKG | RFTISRDNSKNTLYLQMNSLRAEDTAVYYCAK | WGRGYSYE--------------YYYGMDV | WGQGTTVTVSS |

| iPS | Clone | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 98508 | 21-225_24B1 | VH3\|3-23/D5\|5-12\|RF3/JH6 | | EVQLLES-GGGLVQPGGSLRLSCAASG-FTFS | S-----YAMS | WVRQAPGK GLEWVS | AISGR--- GGSTYYADS VKG | RFTISRDNSKNTLYLQMNSL RAEDTAVYYCAK | WGRGYSYE---------------YYYGMDV | WGQGT TVTVS S |
| iPS:3 98528 | 21-225_32G1 | VH3\|3-23/D5\|5-12\|RF3/JH6 | | EVQLLES-GGGLAQPGGSLRLSCAASG-FTFS | S-----YAMS | WVRQAPGK GLEWVS | AISGR--- GGSTFHADS VKG | RFTISRDNSKNTLYLQMNSL RAEDTAVYYCVK | GELLEDY----------------YFYGMDV | WGQGT TVTVS S |
| iPS:3 98534 | 21-225_33B8 | VH3\|3-23/D5\|5-12\|RF3/JH6 | | EVQLLES-GGGLVQPGGSLRLSCAASG-FTFS | S-----YAMS | WVRQAPGK GLEWVS | AISGR--- GGSTFHADS VKG | RFTISRDNSKNTLYLQMNSL RAEDTAVYYCVK | GELLEDY----------------YFYGMDV | WGQGT TVTVS S |
| iPS:3 98540 | 21-225_35A6 | VH3\|3-23/D5\|5-12\|RF3/JH6 | | EVQLLES-GGGLVQPGGSLRLSCAASG-FTFS | S-----YAMS | WVRQAPGK GLEWVS | TISGR--- GGSTFHADS VKG | RFTISRDNSKNTLYLQMNSL RAEDTAVYYCVK | GELLEDY----------------YFYGMDV | WGQGT TVTVS S |
| | | Germline | | H_FR1 | H_CDR1 | H_FR2 | H_CDR2 | H_FR3 | H_CDR3 | H_FR4 |
| | VH4\|4-39/D4\|4-17\|RF2/JH4 | | 32533 | QLQLQES-GPGLVKPSETLSLTCTVSG-GSIS | SS---SYYWG | WIRQPPGK GLEWIG | SIYY---- SGSTYYNPS LKS | RVTISVDTSKNQFSLKLSSV TAADTAVYYCAR | DYGDY------------------YFDY | WGQGT LVTVS S |
| iPS:3 92622 | 21-225_17H8 | VH4\|4-39/D4\|4-17\|RF2/JH4 | | QLQLQES-GPGLVKPSETLSLTCTVSG-GSIS | RS---SYYWG | WIRQPPGK GLEWIG | NIYY---- GGNTYYNPS LKS | RVTISVDTSKNQFSLKLSSV TAADTAVYYCAR | HGKDW---------------------GLDY | WGQGT LVTVS S |
| iPS:3 92638 | 21-225_17F9 | VH4\|4-39/D4\|4-17\|RF2/JH4 | | QLQLQES-GPGLVKPSETLSLTCTVSG-GSIS | RS---SYYWG | WIRQPPGK GLEWIG | NIYY---- SGSTYYNPS LKS | RVTISVDSSKNQFSLNLNSV TAADTAVYSCAR | HGKDW---------------------GLDY | WGQGT LVTVS S |
| iPS:3 92656 | 21-225_1F2 | VH4\|4-39/D4\|4-17\|RF2/JH4 | | QLQLQES-GPGLVKPSETLSLTCTVSG-GSIS | RS---SYYWG | WIRQPPGK GLEWIG | NIYY---- SGSAYNNPS LKG | RVTISVDTSKNQFSLKLNSV TAADTAVYYCGR | HGKDW---------------------GLDY | WGQGT LVTVS S |
| iPS:3 92794 | 21-225_21H3 | VH4\|4-39/D4\|4-17\|RF2/JH4 | | QLQLQES-GPGLVKPSETLSLTCTVSG-GSIS | RS---SYYWG | WIRQPPGK GLEWIG | NIYY---- SGSTYDNPS LKS | RVTISVDTSKNQFSLKLSSV TAADTAVYYCGR | HGKDW---------------------GLDY | WGQGT LVTVS S |
| iPS:3 92822 | 21-225_23C8 | VH4\|4-39/D4\|4-17\|RF2/JH4 | | QLQLQES-GPGLVKPSETLSLTCTVSG-GSIS | RS---SYYWG | WIRQPPGK GLEWIG | NIYY---- SGTTYNNPS LKS | RVTISVDTSKNHFSLKLSSV TAADTAVYYCGR | HGKDW---------------------GLDY | WGQGT LVTVS S |
| iPS:3 92838 | 21-225_22G8 | VH4\|4-39/D4\|4-17\|RF2/JH4 | | QLQLQES-GPGLVKPSETLSLTCTVSG-GSIN | RS---SYYWG | WIRQPPGK GLDWIG | NIYY---- SGSTYYNPS VKS | RFTISVDTSKNQFSLKLSSV TAADTAVYYCAR | HGKDW---------------------GLDY | WGQGT LVTVS S |
| iPS:3 92858 | 21-225_22H4 | VH4\|4-39/D4\|4-17\|RF2/JH4 | | QLQLQES-GPGLVKPSETLSLTCTVSG-GSIS | RS---SYYWG | WIRQPPGK GLEWIG | NIYY---- SGSTYHNPS LKS | RVTISVDTSMNQFSLKLTSV TAADTAVYFCGR | HGKDW---------------------GLDY | WGQGT LVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:3 92882 | 21-225_23A3 | VH4\|4-39/D4\|4-17\|RF2/JH4 | | QLQLQES-GPGLVKPSETLSLTCTVSG-GSIS | RS---SYYWG | WIRQPPGQGLEWIG | NIYY----SGSTYNNPSLKS | RVSISVDISKNQFSLNLSSVTAADTAVYYCGR | HGKDW---------------------GLDF | WGQGTLVTVSS |
| iPS:3 93804 | 21-225_5H7 | VH4\|4-39/D4\|4-17\|RF2/JH4 | | QLQLQES-GPGLVKPSETLSLTCTVSG-GSIN | RS---SYYWG | WIRQPPGKGLEWIG | NIYY----SGTTYYNPSLKS | RVTISVDISKNQFSLNLSSVTAADTAVYSCAR | HGKDW---------------------GLDY | WGQGTLVTVSS |
| iPS:3 93832 | 21-225_14B2 | VH4\|4-39/D4\|4-17\|RF2/JH4 | | QLQLQES-GPGLVKPSETLSLTCTVSG-GSIN | RS---SYYWG | WIRQPPGKGLEWIG | NIYY----SGTTYYNPSLKS | RVTISVDISKNQFSLNLSSVTAADTAVYSCAR | HGKDW---------------------GLDY | WGQGALVTVSS |
| iPS:3 94037 | 21-225_4F4 | VH4\|4-39/D4\|4-17\|RF2/JH4 | | QLQLQES-GPGLVRPSETLSLTCTVSG-GSIS | RS---SYYWG | WIRQPPGKGLEWIG | NIYY----SGSTYDNPSLKS | RVTISVDISKNQFSLKLSSVTAADTAVYYCGR | HGKDW---------------------GLDY | WGQGTLVTVSS |
| iPS:3 94045 | 21-225_4H4 | VH4\|4-39/D4\|4-17\|RF2/JH4 | | QLQLQES-GPGLVKPSETLSLTCTVSG-GSIS | RS---SYYWG | WIRQPPGMGLEWIG | NIYY----SGNTYNNPSLKS | RVTISVDISKNQFSLKLNSVTAADTAVYYCGR | HGKDW---------------------GLDY | WGQGTLVIVSS |
| iPS:3 94079 | 21-225_11F5 | VH4\|4-39/D4\|4-17\|RF2/JH4 | | QLQLQES-GPGLVKPSETLSLTCTVSG-GSTS | RS---SYYWG | WIRQPPGKGLEWIG | NIYY----SGSTYTNPSLKS | RVTISVDISKNHFSLKLSSVTAADTAVYYCGR | HGKDW---------------------GLDN | WGQGTLVTVSS |
| | Germline | | | **H_FR1** | **H_CDR1** | **H_FR2** | **H_CDR2** | **H_FR3** | **H_CDR3** | **H_FR4** |
| **VH3\|3-21/D4\|4-11\|RF2/JH3** | | | 32534 | EVQLVES-GGGLVKPGGSLRLSCAASG-FTFS | S------YSMN | WVRQAPGKGLEWVS | SISSS----SSYIYYADSVKG | RFTISRDNAKNSLYLQMNSLRAEDTAVYYCAR | DYSNY---------------------DAFDI | WGQGTMVTVSS |
| iPS:3 92624 | 21-225_17H12 | VH3\|3-21/D4\|4-11\|RF2/JH3 | | EVQLVES-GGGLVKPGGSLRLSCAASG-FTFS | S------YTMN | WVRQAPGKGLEWVS | SISGS----SSYIYYADSVKG | RFTISRDNAKNSLYLQMNSLRAEDTAVYYCAR | DRG---------------------------SI | WGQGTMVTVSS |
| iPS:3 93946 | 21-225_16A4 | VH3\|3-21/D4\|4-11\|RF2/JH3 | | EVQLVES-GGGLVKPGGSLRLSCAASG-FTFS | S------YSMN | WVRQAPGKGLEWVS | SISGS----STYKYYADSVKG | RFTISRDNAKNSLYLQMNSLRTEDTAVYYCAR | DRG---------------------------SY | WGQGTQVTVSS |
| iPS:3 94008 | 21-225_15H8 | VH3\|3-21/D4\|4-11\|RF2/JH3 | | EVQLVES-GGGLVKPGGSLRLSCAASG-FTFS | S------YVMN | WVRQAPGKGLEWVS | SISGS----STYIYCADSIKG | RFTISRDNAKNSLYLQMNSLRADDTAVYYCAR | DRG---------------------------SI | WGQGTMVTVSS |
| | Germline | | | **H_FR1** | **H_CDR1** | **H_FR2** | **H_CDR2** | **H_FR3** | **H_CDR3** | **H_FR4** |
| **VH6\|6-01/D6\|6-19\|RF1/JH6** | | | 32535 | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | GYSSGWYY-------------YYYYGMDV | WGQGTTVTVSS |
| iPS:3 92636 | 21-225_17A6 | VH6\|6-01/D6\|6-19\|RF1/JH6 | | QVQLQQS-GPGLVKPSQTLSLTCAISG-DSVS | RN---TAAWS | WIRQSPSRGLEWLG | RTYYR--SKWYNDYAVSVKS | RVTINPDTSKNQFSLQLNSVTPEDTAVYYCAR | VSSGWSHH-------------YYYYGMDV | WGQGTTVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH6\|6-01/D2\|2-15\|RF2/JH6 | | 32536 | QVQLQQS-GPGLVKPSQILSL TCAISG-DSVS | SN---SAAWN | WIRQSPSR GLEWLG | RTYYR-- SKWYNDYAV SVKS | RITINPDTSKNQFSLQLNSV TPEDTAVYYCAR | GYCSGGSCYS----- -----YYYYYGMDV | WGQGT TVTVS S |
| IPS:3 92648 | 21-225_16D11 | VH6\|6-01/D2\|2-15\|RF2/JH6 | | QVQLQQS-GPGLVKPSQILSL TCAISG-DSVS | RN---TAAWS | WIRQSPSR GLEWLG | RTYYR-- SKWYNDYAV SVKS | RITINPDTSKNQFSLQLNSV TPEDTAVYYCAR | VNSGWSHH-------- ------YYYYGMDV | WGQGT TVTVS S |
| | | Germline | | H_FR1 | H_CDR1 | H_FR2 | H_CDR2 | H_FR3 | H_CDR3 | H_FR4 |
| | VH3\|3-11/D1\|1-1\|RF3/JH2 | | 32537 | QVQLVES-GGGLVKPGGSLRL SCAASG-FTFS | D-----YYMS | WIRQAPGK GLEWVS | YISSS--- GSTIYYADS VKG | RFTISRDNAKNSLYLQMNSL RAEDTAVYYCAR | YNWXDY---------- --------WYFDL | WGRGT LVTVS S |
| IPS:3 92650 | 21-225_17A4 | VH3\|3-11/D1\|1-1\|RF3/JH2 | | QVQLVES-GGGLVKPGGSLRL SCAASG-FTFS | D-----YYMS | WIRQAPGK GLEWVS | HISSS--- GSTIYYADS VKG | RFTISRDNAKNSLYLQMNSL RAEDTAVYYCAR | YRNNR---------- ---------GYFDL | WGRGT LVTVS S |
| IPS:3 92728 | 21-225_20F7 | VH3\|3-11/D1\|1-1\|RF3/JH2 | | QVQLVES-GGGLVKPGGSLRL SCAASG-FTFS | D-----YYMS | WIRQAPGK GLEWVS | HISSS--- GSTIYYADS VKG | RFTISRDNGENSLYLQMNSL RAEDTAVYYCAR | YRNNR---------- ---------GYFDL | WGRGS LVTVS S |
| | | Germline | | H_FR1 | H_CDR1 | H_FR2 | H_CDR2 | H_FR3 | H_CDR3 | H_FR4 |
| | VH4\|4-59/D1\|1-26\|RF3/JH4 | | 32538 | QVQLQES-GPGLVKPSETLSL TCTVSG-GSTS | S-----YYWS | WIRQPPGK GLEWIG | YIYY---- SGSTNYNPS LKS | RVTISVDTSKNQFSLKLSSV TAADTAVYYCAR | YSGSY---------- ---------YYFDY | WGQGT LVTVS S |
| IPS:3 92676 | 21-225_19F3 | VH4\|4-59/D1\|1-26\|RF3/JH4 | | QVQLQES-GPGLVKPSETLSL TCTVSG-GAIS | GS---SYYWG | WIRQPPGK QLEWIG | NIYY---- SGSTYYNPS FKS | RVTISVDTSKNQFSLKLSSV TAEDTAVYYCAR | HSSSW----------- ----------SLDY | WGQGT LVTVS S |
| | | Germline | | H_FR1 | H_CDR1 | H_FR2 | H_CDR2 | H_FR3 | H_CDR3 | H_FR4 |
| | VH3\|3-23/D6\|6-13\|RF2/JH2 | | 32539 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | GIAAAG--------- --------YWYFDL | WGRGT LVTVS S |
| IPS:3 92678 | 21-225_20F3 | VH3\|3-23/D6\|6-13\|RF2/JH2 | | EVQLLES-GGGLVQPGGSLRL SCAASG-FTFS | S-----YAMN | WVRQAPGK GLEWVS | VISGS--- GGSTYYADS VKG | RFTISRDNSKNTLYLQMNSL RAEDTAVYYCAK | RIAAAG---------- --------TEYFDL | WGRGT LVTVS S |
| | | Germline | | H_FR1 | H_CDR1 | H_FR2 | H_CDR2 | H_FR3 | H_CDR3 | H_FR4 |
| | VH3\|3-21/D5\|5-24\|RF3/JH4 | | 32540 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | RDGYN---------- --------YYFDY | WGQGT LVTVS S |
| IPS:3 92682 | 21-225_16A12 | VH3\|3-21/D5\|5-24\|RF3/JH4 | | EVQLVES-GGGLVKPGGSLRL SCAASG-FTFS | S-----YRMN | WFRQAPGK GLEWVS | SISGS--- STDIYYADS VKG | RFTISRDNAENSLYLQMNSL RAEDTAVYYCAR | RD------------- ------------F | WGQGT LVTVS S |
| | | Germline | | H_FR1 | H_CDR1 | H_FR2 | H_CDR2 | H_FR3 | H_CDR3 | H_FR4 |
| | VH3\|3-48/D7\|7-27\|RF1/JH4 | | 32541 | EVQLVES-GGGLVQPGGSLRL SCAASG-FTFS | S-----YSMN | WVRQAPGK GLEWVS | YISSS--- SSTIYYADS VKG | RFTISRDNAKNSLYLQMNSL RDEDTAVYYCAR | LTGY---------- -----------FDY | WGQGT LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| iPS:3 92692 | 21-225_18G10 | VH3\|3-48/D7\|7-27\|RF1/JH4 | | EVQLVES-GGGLVQPGGSLRL LCAASG-ITFS | T-----YSMN | WVRQAPGK GLEWVS | YISRS--- SSTIDYADS VKG | RFTISRDNAKNSLYLQMNSL RDEDTAVYYCAR | GGGS------------ ----------PFDY | WGQGT LVTVS S |
| iPS:3 92708 | 21-225_18D11 | VH3\|3-48/D7\|7-27\|RF1/JH4 | | EVQLVES-GGGLVQPGGSLRL SCAASG-FTFR | S-----YSMN | WVRQAPGK GLEWLS | YISSS--- SGTIYYADS VKG | RFTISRDNARNSLNLQMNSL RDEDTAVYYCAR | GGGS------------ ----------PFDY | WGQGI LVTVS S |
| iPS:3 93992 | 21-225_14H8 | VH3\|3-48/D7\|7-27\|RF1/JH4 | | EVQLVES-GGGLVQPGGSLRL SCAASG-FTFS | S-----NSMN | WVRQAPGK GLEWVS | YISSS--- SSTIYYADS VKG | RFTIARDNAKNSLYLQMNSL RDEDTAVYYCAR | GGGS------------ ----------PFDY | WGQGT LVTVS S |
| iPS:3 94055 | 21-225_9C8 | VH3\|3-48/D7\|7-27\|RF1/JH4 | | EVQLVES-GGGLVQPGGSLRL SCVVSG-FTFS | S-----QSMN | WVRQAPGK GLEWVS | YISI---- SSTIYYADS VKG | RFTISRDNAKNSLYLQMNSL RDEDTAVYYCAR | GGGS------------ ----------PFDS | WGQGT LVTVS S |
| | | Germline | | **H_FR1** | **H_CDR1** | **H_FR2** | **H_CDR2** | **H_FR3** | **H_CDR3** | **H_FR4** |
| | **VH3\|3-30.3/D4\|4-11\|RF2/JH4** | | 32542 | QVQLVES-GGGVVQPGRSLRL SCAASG-FTFS | S-----YAMH | WVRQAPGK GLEWVA | VISYD--- GSNKYYADS VKG | RFTISRDNSKNTLYLQMNSL RAEDTAVYYCAR | DYSNY---------- ----------YFDY | WGQGT LVTVS S |
| iPS:3 92694 | 21-225_19A5 | VH3\|3-30.3/D4\|4-11\|RF2/JH4 | | QVQLVES-GGGVVQPGRSLRL SCAASG-FTFS | S-----YAMH | WVRQAPGK GLEWVA | VIWFD--- GSDKYYADS VKG | RFTISRDNSKNTLYLQMNSL RAEDTAVYYCAR | DRAYS----------- ---------SSSDY | WGQGT LVTVS S |
| | | Germline | | **H_FR1** | **H_CDR1** | **H_FR2** | **H_CDR2** | **H_FR3** | **H_CDR3** | **H_FR4** |
| | **VH3\|3-23/D1\|1-1\|RF1/JH4** | | 32543 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | GTTGT----------- ----------YFDY | WGQGT LVTVS S |
| iPS:3 92714 | 21-225_16G12 | VH3\|3-23/D1\|1-1\|RF1/JH4 | | EVQLLES-GGGLVQPGGSLRL SCAASG-FSFS | S-----YAMT | WVRQAPGK GLEWVS | TISGR--- GGHTYYADS VRG | RFAISRDSSKNTLYLQMNSL RAEDTAVYYCAK | QD-------------- -------------C | WGQGT LVTVS S |
| iPS:3 92890 | 21-225_20H9 | VH3\|3-23/D1\|1-1\|RF1/JH4 | | EVQLLES-GGGLVQPGGSLRL SCAASG-FTFS | S-----YAMS | WVRQAPGK GLEWVS | AISGS--- GGYTYYADS VKG | RFTISRDNSENTLYLQMSSL RAEDTAVYYCAK | GGS------------- -----------LFY | WGQGT LVTVS S |
| iPS:3 92892 | 21-225_20C11 | VH3\|3-23/D1\|1-1\|RF1/JH4 | | EVQLLES-GGGLVQPGGSLRL SCAASG-FSFS | S-----YAMS | WVRQAPGK GLEWVS | TISGR--- GGHTYYADS VKG | RFAISRDSSKNTLYLQMNSL RAEDTAVYYCAK | QD-------------- -------------C | WGQGT LVTVS S |
| iPS:3 93968 | 21-225_5A5 | VH3\|3-23/D1\|1-1\|RF1/JH4 | | EVQLWES-GGGLVQPGGSLRL SCAASG-FTFS | S-----YAMN | WVRQAPGK GLEWVS | AISGS--- GGYTYYADS VKG | RFTISRDNSKNTLYLQMSSL RAEDTAVYYCAK | GGS------------- -----------LFY | WGQGT LVTVS S |
| | | Germline | | **H_FR1** | **H_CDR1** | **H_FR2** | **H_CDR2** | **H_FR3** | **H_CDR3** | **H_FR4** |
| | **VH3\|3-23/D6\|6-6\|RF1/JH3** | | 32544 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | EYSSSS---------- ---------DAFDI | WGQGT MVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:3 92730 | 21-225_17A1 | VH3\|3-23/D6\|6-6\|RF1/JH3 | | EVQLLES-GGGLVQPGGSLRLSCAASG-FTFS | S-----YAMN | WVRQAPGKGLEWVS | VISGS---GSNTYYADSVKG | RFTISRDNSKNTLYLQMNSLRAEDTAVYYCAK | RYTSDW-----------------HDAFDI | WGQGTMVTVSS |
| iPS:3 92736 | 21-225_17B12 | VH3\|3-23/D6\|6-6\|RF1/JH3 | | EVQLLES-GGGLVQPGGSLRLSCVASG-FTFS | S-----YAMN | WVRQAPGKGLEWVS | VISGS---GGSTYYADSVKG | RFTISRDNSKNTLYLQMNTLRAEDTAVYYCAK | RYTSDW-----------------HDAFDI | WGQGTMVTVSS |
| iPS:3 92770 | 21-225_20C10 | VH3\|3-23/D6\|6-6\|RF1/JH3 | | EVQLLES-GGGLVQPGGSLRLSCAASG-FTFS | S-----YAMN | WVRQAPGKGLEWVS | VISGS---GGTTYYADSVKG | RFTISRDNSKNTLYLQMNTLRAEDTAVYYCAK | RYTSDW-----------------HDAFDI | WGQGTMVTVSS |
| iPS:3 93878 | 21-225_7G12 | VH3\|3-23/D6\|6-6\|RF1/JH3 | | EVQLLES-GGGLVQPGGSLRLSCAASG-FTFS | S-----YAMN | WVRQAPGKGLEWVS | VISGS---GGSTYYADSVKG | RFTISRDNSKNTLYLQMNTLRAEDTAVYYCAK | RYTSDW-----------------HDAFDI | WGQGTMVTVSS |
| | Germline | | | H_FR1 | H_CDR1 | H_FR2 | H_CDR2 | H_FR3 | H_CDR3 | H_FR4 |
| | VH3\|3-21/D7\|7-27\|RF3/JH5 | | 32545 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | NWGN-------------------WFDP | WGQGTLVTVSS |
| iPS:3 92748 | 21-225_20A8 | VH3\|3-21/D7\|7-27\|RF3/JH5 | | EVQLVES-GGGLVKPGGSLRLSCAASG-FTFS | S-----YSVN | WVRRAPGKGLEWVS | SISSS---SSFLYYADSVKG | RFTISRDNAKNSVYLQMNSLRAEDTAVYYCAR | NW-------------------DY | WGQGTLVTVSS |
| | Germline | | | H_FR1 | H_CDR1 | H_FR2 | H_CDR2 | H_FR3 | H_CDR3 | H_FR4 |
| | VH3\|3-30.3/D1\|1-1\|RF1/JH5 | | 32546 | QVQLVES-GGGVVQPGRSLRLSCAASG-FTFS | S-----YAMH | WVRQAPGKGLEWVA | VISYD---GSNYYADSVKG | RFTISRDNSKNTLYLQMNSLRAEDTAVYYCAR | GTTGT-------------------NWFDP | WGQGTLVTVSS |
| iPS:3 92754 | 21-225_21D3 | VH3\|3-30.3/D1\|1-1\|RF1/JH5 | | QVQLVES-GGGVVQPGRSLRLSCVASG-FTFS | S-----YGMH | WVRQAPGKGLEWVT | VISYD---GSNKYYADSVKG | RFTISRDNSKNTLYLQMNSLRAEDTAVYYCAR | GVWF-------------------GDL | WGQGTLVTVSS |
| iPS:3 92818 | 21-225_22D8 | VH3\|3-30.3/D1\|1-1\|RF1/JH5 | | QVQLVES-GGGVVQPGRSLRLSCVASG-FTFR | S-----YGMH | WVRQAPGKGLEWVT | IISYD---GSNKYYADSVKG | RFTISRDNSKNTLYLQMNSLRAEDTAVYFCAR | GVWF-------------------GDF | WGQGTLVTVSS |
| | Germline | | | H_FR1 | H_CDR1 | H_FR2 | H_CDR2 | H_FR3 | H_CDR3 | H_FR4 |
| | VH3\|3-23/D6\|6-19\|RF2/JH1 | | 32547 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GIAVAG-------------------AEYFQH | WGQGTLVTVSS |
| iPS:3 92762 | 21-225_22G5 | VH3\|3-23/D6\|6-19\|RF2/JH1 | | EVQLLES-GGGLVQPGGSLRLSCAASG-FTFS | S-----YAMN | WVRQAPGMGLEWVS | VISRS---GGYTYYADSVKG | RFTISRDNSKNTLYLQMNSLRAEDTAVYYCAS | RLAVAG-------------------SEAFDI | WGQGTLVTVSS |
| iPS:3 94051 | 21-225_9E5 | VH3\|3-23/D6\|6-19\|RF2/JH1 | | EVQLLES-GGGLVQPGGSLRLSCAASG-FTFS | N-----YAMN | WVRQAPGKGLEWVS | AISGG---GGNTFYADSVKG | RFTISRDNSKNTLYLQMNGLRAEDTAVYYCAS | RIAVAG-------------------SEAFAI | WGQGTLVTVSS |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-39/D1\|1-26\|RF3/JH1 | 32548 | QLQLQES-GPGLVKPSETLSL TCTVSG-GSIS | SS---SYYWG | WIRQPPGK GLEWIG | SIYY---- SGSTYYNPS LKS | RVTISVDTSKNQFSLKLSSV TAADTAVYYCAR | YSGSYY---------- --------ABYFQH | WGQGT LVTVS S |
| iPS:3 92774 | 21-225_21F3 | VH4\|4-39/D1\|1-26\|RF3/JH1 | QLQLQES-GPGLVKPAETLSL TCTVSG-GSIS | RS---SYYWG | WIRQPPGK VLEWIG | SIYY---- SGSTYYNPS LKS | RVTISVDTSKNQFSLKLSSV TAADTAEYYCAS | LSSSW----------- ----------DFQH | WGQGT LVTVS S |
| iPS:3 93962 | 21-225_7H7 | VH4\|4-39/D1\|1-26\|RF3/JH1 | QLQLQES-GPGLVKPSETLSL TCTVSG-GSIS | RS---SYYWG | WIRQPPGK GLEWIG | NIYY---- SGSTYYIPS LKS | RVTISVDTSKNQFSLKLSSV TAADTAVYYCAR | HSTSW---------- ----------SLDH | WGQGT LVTVS S |
| iPS:3 94049 | 21-225_13H5 | VH4\|4-39/D1\|1-26\|RF3/JH1 | QLQLQES-GPGLVKPAETLSL TCTVSG-GSIS | RS---SYYWG | WIRQPPGK GLEWIG | SIYY---- SGSTYYNPS LKS | RVTISVDTSKNQFSLKLSSV TAADTAVYYCAS | LSSSW----------- ----------DFQH | WGQGT LVTVS S |
| | | Germline | H_FR1 | H_CDR1 | H_FR2 | H_CDR2 | H_FR3 | H_CDR3 | H_FR4 |
| | VH3\|3-21/D2\|2-21\|RF2/JH4 | 32549 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | AYCGGDC--------- ---------YSYFDY | WGQGT LVTVS S |
| iPS:3 92776 | 21-225_21A12 | VH3\|3-21/D2\|2-21\|RF2/JH4 | EVQLVES-GGGLVKPGGSLRL SCAASG-FTFN | S-----YSMN | WVRQAPGK GLEWVS | SISGS--- SSYIYYADS VKG | RFTISRDNAKNSLYLQMNSL RAEDTALYYCAR | AAG------------- ----------FDY | WGQGT LVTVS S |
| | | Germline | H_FR1 | H_CDR1 | H_FR2 | H_CDR2 | H_FR3 | H_CDR3 | H_FR4 |
| | VH3\|3-33/D6\|6-19\|RF2/JH6 | 32550 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | GIAVAGYY-------- --------YYYGMDV | WGQGT TVTVS S |
| iPS:3 92806 | 21-225_24H3 | VH3\|3-33/D6\|6-19\|RF2/JH6 | QVQLVES-GGGVVQPGRSLRL SCGASG-FTFS | S-----YGMH | WVRQAPGK GLEWVA | VIWYD--- GSNKYYADS VKG | RFTISRDNSKNTLYLQMNSL RAEDTAVYYCAR | VAVAG----------- ----------GMDV | WGQGT TVTVS S |
| | | Germline | H_FR1 | H_CDR1 | H_FR2 | H_CDR2 | H_FR3 | H_CDR3 | H_FR4 |
| | VH3\|3-48/D7\|7-27\|RF2/JH4 | 32551 & 32552 | EVQLVES-GGGLVQPGGSLRL SCAASG-FTFS | S-----YSMN | WVRQAPGK GLEWVS | YISSS--- SSTIYYADS VKG | RFTISRDNAKNSLYLQMNSL RDEDTAVYYCAR | *LGY------------ ----------FDY | WGQGT LVTVS S |
| iPS:3 92826 | 21-225_20B9 | VH3\|3-48/D7\|7-27\|RF2/JH4 | EVQLVES-GGGLVQPGGSLRL SCAASG-FTFS | S-----YSMN | WVRQAPGK GLEWVS | YISSS--- SSTIYYADS VKG | RFTISRDNAKNSLYLQMNSL RDEDTAVYYCAR | SLWS------------ ----------PFDY | WGQGT LVTVS S |
| | | Germline | H_FR1 | H_CDR1 | H_FR2 | H_CDR2 | H_FR3 | H_CDR3 | H_FR4 |
| | VH4\|4-59/D1\|1-1\|RF1/JH5 | 32553 | QVQLQES-GPGLVKPSETLSL TCTVSG-GSIS | S-----YYWS | WIRQPPGK GLEWIG | YIYY---- SGSTNYPS LKS | RVTISVDTSKNQFSLKLSSV TAADTAVYYCAR | GTTCT----------- ---------NWFDP | WGQGT LVTVS S |
| iPS:3 92830 | 21-225_21A5 | VH4\|4-59/D1\|1-1\|RF1/JH5 | QVQLQES-GPGLVKPSETLSL TCTVSG-GSIS | S-----YFWS | WIRQPAGK GLEWIG | RIYT---- SGITNYNPS LKS | RVTMSVDTSKNQFSLKLSSV TAADTAIYYCAR | GPTSG----------- ----------WFDP | WGQGT LVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-23/D6\|6-6\|RF2/JH4 | | 32554 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | SIAAR----------- ---------YFDY | WGQGT LVTVS S |
| IPS:3 92840 | 21-225_23G1 | VH3\|3-23/D6\|6-6\|RF2/JH 4 | | EVQLLES-GGGLVQPGGSLRL SCAASG-FTFS | S-----YAMS | WVRQAPGK GLEWVS | VISGS--- GGTTYNTDS VKG | RFTISRDNSKNTLYLQMNSL RAEDTAVYYCAR | SSL------------- -----------FDY | WGQGT LVTVS S |
| IPS:3 94018 | 21-225_15B1 | VH3\|3-23/D6\|6-6\|RF2/JH 4 | | EVQLLES-GGGLVQPGGSLRL SCATSG-FTFS | S-----YVMS | WVRQAPGK GLEWVS | GISGS--- GGSTNNADS VKG | RFTISRDNSKNTLYLQVNSL RAEDTAVYYCAR | SSL------------- -----------FDY | WGQGT LVTVS S |
| IPS:3 94026 | 21-225_16C7 | VH3\|3-23/D6\|6-6\|RF2/JH 4 | | EVQLLES-GGGLVQPGGSLRL SCAASG-FTFS | S-----YVMT | WVRQAPGK GLEWVS | TISGS--- GGWTYYADS VKG | RFTISRDNSKNTLYLQMNSL RAEDTAEYYCAR | SSL------------- -----------FDY | WGQGT LVTVS S |
| | | Germline | | H_FR1 | H_CDR1 | H_FR2 | H_CDR2 | H_FR3 | H_CDR3 | H_FR4 |
| | VH3\|3-23/D6\|6-6\|RF2/JH5 | | 32555 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | SIAAR----------- ---------NWFDP | WGQGT LVTVS S |
| IPS:3 92842 | 21-225_23G8 | VH3\|3-23/D6\|6-6\|RF2/JH 5 | | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAV | SSG------------- -----------WFA | WGQGT LVTVS S |
| | | Germline | | H_FR1 | H_CDR1 | H_FR2 | H_CDR2 | H_FR3 | H_CDR3 | H_FR4 |
| | VH3\|3-23/D1\|1-1\|RF2/JH2 | | 32556 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | VQLERY--------- ---------WYFDL | WGRGT LVTVS S |
| IPS:3 92856 | 21-225_22A2 | VH3\|3-23/D1\|1-1\|RF2/JH 2 | | EVQLLES-GGGLVQPGGSLRL SCAASG-FTFS | S-----YAMS | WVRQAPGK GLEWVS | GISGS--- GGNTPYADS VKG | RFTISRDISKNTLYLQMNSL RAEDTAVYYCAK | VVG------------- -----------AVH | WGRGT LVTVS S |
| | | Germline | | H_FR1 | H_CDR1 | H_FR2 | H_CDR2 | H_FR3 | H_CDR3 | H_FR4 |
| | VH4\|4-30.1/D5\|5-18\|RF1/JH6 | | 32557 | QVQLQES-GPGLVKPSQTLSL TCTVSG-GSIS | SG---GYYWS | WIRQHPGK GLEWIG | YIYY---- SGSTYYNFS LKS | RVTISVDTSKNQFSLKLSSV TAADTAVYYCAR | VDTAMVYY--------- -------YYYGMDV | WGQGT TVTVS S |
| IPS:3 92864 | 21-225_23B9 | VH4\|4-30.1/D5\|5-18\|RF1/J H6 | | QVQLQAS-GPGLVKPSQTLSL TCTVSD-GSIS | SG---GYYWS | WIRQHPGK GLEWIG | YIYY---- SGSTYYNPS LKS | RVTISVDTSKNQFSLKLSSV TAADTAVYYCAR | EDGAFG---------- --------YYGMDV | WGQGT TVTVS S |
| | | Germline | | H_FR1 | H_CDR1 | H_FR2 | H_CDR2 | H_FR3 | H_CDR3 | H_FR4 |
| | VH3\|3-23/D2\|2-2\|RF2/JH3 | | 32558 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | GYCSSTSC--------- -------YTDAFDI | WGQGT MVTVS S |
| IPS:3 92874 | 21-225_21D2 | VH3\|3-23/D2\|2-2\|RF2/JH 3 | | EVKLLES-GGGLVQPGGSLRL SCAASG-FTFN | N-----YAMS | WVRQAPGK GLEWVS | VLSGS--- GGSTFYADS VKG | RFTISRDNSKNTLYLQMSSL RAGDTAVYFCAR | YCSSARC--------- -------PYDAFDI | WGQGT MVTVS S |

| iPS ID | Clone | VH Germline | No. | H_FR1 | H_CDR1 | H_FR2 | H_CDR2 | H_FR3 | H_CDR3 | H_FR4 |
|---|---|---|---|---|---|---|---|---|---|---|
| iPS:3 93940 | 21-225_16B2 | VH3\|3-23/D2\|2-2\|RF2/JH3 | | EVQLLES-GGGLVQPGGSLRLSCAASG-FTFS | S-----YAMT | WVRQAPGKGPEWVS | VISGS---GGSTFYADSVKG | RFTISRDNSKNTLYLQMSSLRAEDTAVYFCAR | YCSSTRC----------------PYDAFDI | WGQGTMVTVSS |
| iPS:3 93956 | 21-225_4D7 | VH3\|3-23/D2\|2-2\|RF2/JH3 | | EVKLLES-GGGLVQPGGSLRLSCAASG-FTFS | S-----YAMS | WVRQAPGKGLEWVS | VLSGS---GGSTFYADSVKG | RFTISRDNSKNTLYLQMSSLRAGDTAVYFCAR | YCSSARC----------------PYDAFDI | WGQGTMVTVSS |
| iPS:3 98476 | 21-225_17C1 | VH3\|3-23/D2\|2-2\|RF2/JH3 | | EVQLLES-GGGLEQPGGSLRLSCAASG-FTFS | S-----YAMS | WVRQAPGKGLEWVS | VISGS---GGTTFYADSVKG | RFTISRDNSKNTLYLQMSSLRAEDTAVYFCAR | YCSSTRC----------------PYDAFDI | WGQGTMVTVSS |
| | Germline | | | H_FR1 | H_CDR1 | H_FR2 | H_CDR2 | H_FR3 | H_CDR3 | H_FR4 |
| | VH3\|3-15/D1\|1-1\|RF3/JH4 | | 32559 | EVQLVES-GGGLVKPGGSLRLSCAASG-FTFS | N-----AWMS | WVRQAPGKGLEWVG | RIKSKT-DGGTTDYAAPVKG | RFTISRDDSKNTLYLQMNSLKTEDTAVYYCTT | YNWND-----------YFDY | WGQGTLVTVSS |
| iPS:3 92898 | 21-225_21H10 | VH3\|3-15/D1\|1-1\|RF3/JH4 | | EVQLVES-GGGLVKPGGSLRLSCAASG-FTFS | N-----AWMN | WVRQAPGKGLEWVG | RIKSKT-DGGTTDYAAPVKG | RFTISRDDSKNTLYLQMNSLKTEDTAVYYCTT | EGWN----------------TDY | WGQGTLVTVSS |
| iPS:3 93802 | 21-225_3D12 | VH3\|3-15/D1\|1-1\|RF3/JH4 | | EVQLVES-GGGLVKPGGSLRLSCAASG-VTFS | T-----AWMN | WVRQAPGKGLEWVG | RIKNKI-DGGTTDYVAPVKG | RFTISRDDSKNTLYLQMNSLKTEDTAVYYCTT | EGWN----------------TDY | WGQGTLVTVSS |
| | Germline | | | H_FR1 | H_CDR1 | H_FR2 | H_CDR2 | H_FR3 | H_CDR3 | H_FR4 |
| | VH3\|3-48/D4\|4-11\|RF3/JH4 | | 32560 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | ITVT-----------YFDY | WGQGTLVTVSS |
| iPS:3 92950 | 21-225_25C10 | VH3\|3-48/D4\|4-11\|RF3/JH4 | | EVQLVES-GGGLVQPGGSLRLSCAASG-FTFS | S-----YRMN | WVRQAPGKGLEWVS | SISSS---SSTIYYADSVKG | RFTISRDNAKNSLYLQMNSLRDEDTAVYYCAR | TAG-------------FDY | WGQGTLVTVSS |
| | Germline | | | H_FR1 | H_CDR1 | H_FR2 | H_CDR2 | H_FR3 | H_CDR3 | H_FR4 |
| | VH3\|3-23/D1\|1-26\|RF3/JH4 | | 32561 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | YSGSY------------YYFDY | WGQGTLVTVSS |
| iPS:3 93010 | 21-225_25E11 | VH3\|3-23/D1\|1-26\|RF3/JH4 | | EVQLLES-GGGLVQPGGSLRLSCAASG-FTFS | S-----YAMS | WVRQAPGKGLEWVS | VISGG---GGSTYYADSVKG | RFTISRDNSKNTLYLQMNSLRAEDTAVYYCAK | RGYSGYE---------DLLYFDC | WGQGTLVTVSS |
| | Germline | | | H_FR1 | H_CDR1 | H_FR2 | H_CDR2 | H_FR3 | H_CDR3 | H_FR4 |
| | VH3\|3-23/D3\|3-3\|RF3/JH3 | | 32562 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | ITIFGVV----------IIDAFDI | WGQGTMVTVSS |
| iPS:3 93016 | 21-225_28F11 | VH3\|3-23/D3\|3-3\|RF3/JH3 | | EVQLLES-GGGLVQPGGSLRLSCAASG-FIFS | S-----YAMS | WVRQAPGKGLEWVS | VISGS---GGTTFYADSVKG | RFTISRDNSKNTLYLQMNSLRAEDTAVYYCAK | RTQFD----------DFDI | WGQGTMVTVSS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-33/D3\|3-22\|RF2/JH6 | | 32563 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | YYYDSSGYYY------ -----YYYYYGMDV | WGQGT TVTVS S |
| iPS:3 93032 | 21-225_26F8 | VH3\|3-33/D3\|3-22\|RF2/J H6 | | QVQLVES-GGGVVQPGRSLRL SCAASG-FTFS | G-----YGMH | WVRQAPGK GLEWVA | IIWYD--- GSNKYYADS VKG | RFTISRDNSKNTLYLQMNSL RAEDTAVYYCAR | ERYDFW---------- --------SGCMDV | WGQGT TVTVS S |
| | VH1\|1-02/D4\|4-11\|RF2/JH6 | | 32564 | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | DYSYYYY--------- -------YYYGMDV | WGQGT TVTVS S |
| iPS:3 93042 | 21-225_31F1 | VH1\|1-02/D4\|4-11\|RF2/J H6 | | QVQLVQS-GAEVKKPGASVKV SCKASG-YTFT | D-----YYMH | WVRQAPGQ GLEWMG | WINPN--- SGGTNYAQK FQG | RVTMTRDTSIMTAYMELSRL RSDDTAVYYCAR | DSSNFSNW-------- ------YDYYGMDV | WGQGT TVTVS S |
| iPS:3 93108 | 21-225_34G11 | VH1\|1-02/D4\|4-11\|RF2/J H6 | | QVQLVQS-GAEVKKPGASVKV SCKASG-YTFT | G-----YYMH | WVRQAPGQ GLEWMG | WINPN--- SGGTNYAQK FQG | RVTMTRDTSISTAYMELSRL RSDDTAVYYCAR | DISNFSSW-------- ------YDYYAMDV | WGQGT TVTVS S |
| | VH1\|1-18/D5\|5-12\|RF3/JH4 | | 32565 | QVQLVQS-GAEVKKPGASVKV SCKASG-YTFT | S-----YGIS | WVRQAPGQ GLEWMG | WISAY--- NGNTNYAQK LQG | RVTMTTDTSTSTAYMELRSL RSDDTAVYYCAR | GYSGYD---------- ---------YYFDY | WGQGT LVTVS S |
| iPS:3 93044 | 21-225_25B8 | VH1\|1-18/D5\|5-12\|RF3/J H4 | | QVQLVQS-GAEVKKPGASVKV SCKASG-YTFT | S-----YGIS | WVRQAPGQ GLEWMG | WISAY--- NGNTTYAQK LRG | RVTMTTDTSTSTAYMDLRSL RSDDTAVYYCAR | TAAGYS---------- --------SSWFDY | WGQGT LVTVS S |
| iPS:3 93050 | 21-225_28C5 | VH1\|1-18/D5\|5-12\|RF3/J H4 | | QVQLVQS-GAEVKKPGASVKV SCKASD-YTFT | S-----YGIS | WVRQAPGQ GLEWMG | WISAY--- NGNTTYAQK LRG | RVTMTTDTSTSTAYMDLRSL RSDDTAVYYCAR | TAAGYS---------- --------SSWFDY | WGQGT LVTVS S |
| | VH3\|3-33/D6\|6-6\|RF1/JH3 | | 32566 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | EYSSSS---------- --------DAFDI | WGQGT MVTVS S |
| iPS:3 93046 | 21-225_25A12 | VH3\|3-33/D6\|6-6\|RF1/JH 3 | | QVQLVES-GGGVVQPGRSLRL SCAASG-FTFS | N-----CVMH | WVRQAPGK GLEWVA | VIWYD--- GSNKYYADS VKG | RFTISRDNSKNTLYLQMNSL RAEDTAVYYCAR | EEYSSGW--------- -------YDYGMDV | WGQGT MVTVS S |
| | VH3\|3-21/D4\|4-11\|RF3/JH6 | | 32567 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | TTVTYYY--------- ---------YYGMDV | WGQGT TVTVS S |
| iPS:3 93078 | 21-225_33H11 | VH3\|3-21/D4\|4-11\|RF3/J H6 | | EVQLVES-GGGLVKPGGSLRL SCAASG-FTFS | S-----YSMN | WVRQAPGK GLEWVS | SISGS--- SSYIYYADS VKG | RFTISRDNAKNSLYLQMSSL RAEDTAVYYCAR | TNG------------- -----------MDV | WGQGT TVTVS S |

| | | | | H_FR1 | H_CDR1 | H_FR2 | H_CDR2 | H_FR3 | H_CDR3 | H_FR4 |
|---|---|---|---|---|---|---|---|---|---|---|
| iPS:3 93142 | 21-225_33A3 | VH3|3-21/D4|4-11|RF3/JH6 | | EVQLVES-GGGLVKPGGSLRLSCAASG-FTFS | S-----YGMN | WVRQAPGK GLEWVS | SISGS---STYIYYADS VKG | RFTISRDNAKNSLYLQMNSL RAEDTAVYYCAR | TNG-------------------------MDV | WGQGT TVTVS S |
| | Germline | | | H_FR1 | H_CDR1 | H_FR2 | H_CDR2 | H_FR3 | H_CDR3 | H_FR4 |
| | VH3|3-23/D6|6-19|RF2/JH6 | | 32568 | EVQLLES-GGGLVQPGGSLRLSCAASG-FTFS | S-----YAMS | WVRQAPGK GLEWVS | AISGS---GGSTYYADS VKG | RFTISRDNSKNTLYLQMNSL RAEDTAVYYCAK | GIAVAGYY----------------YYYGMDV | WGQGT TVTVS S |
| iPS:3 93096 | 21-225_34D11 | VH3|3-23/D6|6-19|RF2/JH6 | | EVQLSES-GGGLVQPGGSLRLSCAASG-FTFS | S-----YAMN | WVRQAPGK GLEWVS | AISGR---GGSTFHADS VKG | RFTISRDNSKNTLYLQMNSL RAEDTAVYYCVK | GELVEDY---------------YFYGMDV | WGQGT TVTVS S |
| | Germline | | | H_FR1 | H_CDR1 | H_FR2 | H_CDR2 | H_FR3 | H_CDR3 | H_FR4 |
| | VH3|3-30.3/D4|4-23|RF2/JH3 | | 32569 | QVQLVES-GGGVVQPGRSLRLSCAASG-FTFS | S-----YAMH | WVRQAPGK GLEWVA | VISYD---GSNKYYADS VKG | RFTISRDNSKNTLYLQMNSL RAEDTAVYYCAR | DYGCNS-------------DAFDI | WGQGT MVTVS S |
| iPS:3 93172 | 21-225_3B12 | VH3|3-30.3/D4|4-23|RF2/JH3 | | QVHLVES-GGGVVQPGRSLRLSCAASG-FTFS | Y-----YGMH | WVRQAPGK GLEWVA | VISYD---GSNKYYADS VKG | RFTISRDNSKNTLHLQMNSL RAEDTAVYYCAR | DRRCGYG---------------VPDAFDI | WGQGT MVTVS S |
| | Germline | | | H_FR1 | H_CDR1 | H_FR2 | H_CDR2 | H_FR3 | H_CDR3 | H_FR4 |
| | VH3|3-30.3/D4|4-23|RF2/JH6 | | 32570 | QVQLVES-GGGVVQPGRSLRLSCAASG-FTFS | S-----YAMH | WVRQAPGK GLEWVA | VISYD---GSNKYYADS VKG | RFTISRDNSKNTLYLQMNSL RAEDTAVYYCAR | DYGGNSYY--------------YYYGMDV | WGQGT TVTVS S |
| iPS:3 93174 | 21-225_15D8 | VH3|3-30.3/D4|4-23|RF2/JH6 | | QVQLVES-GGGVVQTGRSLRLSCAASG-FTFS | G-----YGMH | WVRQAPGK GLEWVS | VISYD---GSNKYYADS VKG | RFTISRDNSKNTLYLQMNSL RAEDTAVYYCAR | DRVYCSSTSCV---------PYYDYYGMDV | WGQGT TVTVS S |
| | Germline | | | H_FR1 | H_CDR1 | H_FR2 | H_CDR2 | H_FR3 | H_CDR3 | H_FR4 |
| | VH3|3-15/D7|7-27|RF1/JH6 | | 32571 | EVQLVES-GGGLVKPGGSLRLSCAASG-FTFS | N-----AWMS | WVRQAPGK GLEWVG | RIKSKT-DGGTTDYAA PVKG | RFTISRDDSKNTLYLQMNSL KTEDTAVYYCTT | LTGYYY----------------YYGMDV | WGQGT TVTVS S |
| iPS:3 93194 | 21-225_16D2 | VH3|3-15/D7|7-27|RF1/JH6 | | EVQLVES-GGGLVKPGGSLRLSCAASG-FTFS | N-----AWMN | WVRQAPGK GLEWVG | RIKSKT-DGGTTDYAA PVKG | RFTISRDDSKNTLYLQMHSL KTEDTAVYYCTT | DTGPIAARLA------------YYYYYAMDV | WGQGT TVTVS S |
| iPS:3 98488 | 21-225_19F6 | VH3|3-15/D7|7-27|RF1/JH6 | | EVQLVES-GGGLVKPGGSLRLSCAASG-FTFS | N-----AWMN | WVRQAPGK GLEWVG | RIKSKT-DGGTTDYAA PVKG | RFTISRDDSKNTLYLQMNSL KTEDTAVYYCTT | DTGPIAARLA------------YYYYYAMDV | WGHGT TVTVS S |
| iPS:3 98544 | 21-225_7C8 | VH3|3-15/D7|7-27|RF1/JH6 | | EVQLVES-GGGLVKPGGSLRLSCAASG-FTFS | N-----ARMN | WVRLAPGK GLEWVG | RIKSKT-DGGTTDYAA PVKG | RFTISRDESENTLYLQMNSL KTEDTGVYYCST | DTGPIAARLA------------YYYYYAMDV | WGQGT TVTVS S |
| iPS:4 02231 | 21-225_6D9 | VH3|3-15/D7|7-27|RF1/JH6 | | EVQLVES-GGGLVKPGGSLRLSCAASG-FTFS | N-----AWMN | WVRLAPGK GLEWVG | RIKSKT-DGGTTDYAA PVKG | RFTISRDDSENTLYLQMNSL KTEDTAVYYCST | DTGPIAARLA------------YYYYYAMDV | WGQGT TVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-23/D4\|4-11\|RF2/JH4 | | 32572 | EVQLLES-GGGLVQPGGSLRL SCAASG-FTFS | S-----YAMS | WVRQAPGK GLEWVS | AISGS---GGSTYYADS VKG | RFTISRDNSKNTLYLQMNSL RAEDTAVYYCAK | DYSYY----------YFDY | WGQGT LVTVS S |
| iPS:3 93870 | 21-225_7B1 | VH3\|3-23/D4\|4-11\|RF2/J H4 | | EVQLLES-GGGLVQPGGSLRL SCAASG-FTFS | S-----YDMS | WVRQAPGK GLEWVS | TISGS---GGITYYADS VKG | RFTISRDNSKKILYLQMNSL RAEDTAVYFCAR | DRG-------------SV | WGQGT LVTVS S |
| | VH4\|4-39/D4\|4-17\|RF2/JH1 | | 32573 | QLQLQES-GPGLVKPSETLSL TCTVSG-GSIS | SS---SYYWG | WIRQPPGK GLEWIG | SIYY---SGSTYYNPS LKS | RVTISVDTSKNQFSLKLSSV TAADTAVYYCAR | DYGDYA----------EYFQH | WGQGT LVTVS S |
| iPS:3 93872 | 21-225_2A11 | VH4\|4-39/D4\|4-17\|RF2/J H1 | | QLQLQES-GPGLVKPSETLSL TCTVSG-GSIS | RS---SYYWG | WIRQPPGK GLEWIG | NIYY---SGSTYYNPS VKS | RVTISVDTSKNQFSLKLSTV TAADTAVYYCAR | HGKDW----------GLED | WGQGT LVTVS S |
| | VH4\|4-59/D6\|6-6\|RF1/JH4 | | 32574 | QVQLQES-GPGLVKPSETLSL TCTVSG-GSIS | S-----YYWS | WIRQPPGK GLEWIG | YIYY---SGSTNYNPS LKS | RVTISVDTSKNQFSLKLSSV TAADTAVYYCAR | EYSSS----------SYFDY | WGQGT LVTVS S |
| iPS:3 93890 | 21-225_4B1 | VH4\|4-59/D6\|6-6\|RF1/JH 4 | | QVQLQES-GPGLVKPSETLSL TCSVSG-DSIS | S-----YSWS | WIRQPAGK GLEWIG | RIYT---SGSTNYIPS LKS | RITMSVDTSKKQFSLKLSSV TAADTAVYYCAR | DLKSSG----------CLFFDY | WGQGT LVTVS S |
| | VH3\|3-33/D4\|4-17\|RF1/JH6 | | 32575 & 32576 & 32577 | QVQLVES-GGGVVQPGRSLRL SCAASG-FTFS | S-----YGMH | WVRQAPGK GLEWVA | VIWYD---GSNKYYADS VKG | RFTISRDNSKNTLYLQMNSL RAEDTAVYYCAR | *LR*LYY---------YYYGMDV | WGQGT TVTVS S |
| iPS:3 93926 | 21-225_4G4 | VH3\|3-33/D4\|4-17\|RF1/J H6 | | QVQLVES-GGGVVQPGRSLRL SCAASG-FTFS | S-----YGMH | WVRQAPGK GLEWGA | VIWHD---GSNKYYADS VKG | RFTISRDNAKNTLYLQMNSL RAEDTAVYYCAR | DLRMG----------GMDV | WGQGT TVTVS S |
| | VH3\|3-23/D6\|6-6\|RF2/JH2 | | 32578 | EVQLLES-GGGLVQPGGSLRL SCAASG-FTFS | S-----YAMS | WVRQAPGK GLEWVS | AISGS---GGSTYYADS VKG | RFTISRDNSKNTLYLQMNSL RAEDTAVYYCAK | SIAARY----------WYFDL | WGRGT LVTVS S |
| iPS:3 93936 | 21-225_14A11 | VH3\|3-23/D6\|6-6\|RF2/JH 2 | | EVQLLES-GGGLVQPGGSLRL SCAASG-FTFS | S-----YAMN | WVRQAPGK GLEWVS | VISGR---GGSTYYADS VKG | RFTISRDNSKNTLYLQMNSL RAEDTAVYYCAK | RIAAGM----------EYFDL | WGRGT LVTVS S |
| | VH3\|3-21/D1\|1-1\|RF3/JH4 | | 32579 | EVQLVES-GGGLVKPGGSLRL SCAASG-FTFS | S-----YSMN | WVRQAPGK GLEWVS | SISSS---SSYIYYADS VKG | RFTISRDNAKNSLYLQMNSL RAEDTAVYYCAR | YNWND----------YFDY | WGQGT LVTVS S |
| iPS:3 93988 | 21-225_7F10 | VH3\|3-21\|D1\|1-1\|RF3/JH 4 | | EVQLVES-GGGLVKPGGSLRL SCAASG-FTFS | S-----YSMN | WVRQAPGK GLEWVS | SISSS---SSYIYYADS VKG | RFTISRDNAKNSLYLQMNSL RTEDTAVYYCAR | ANL-------------FDY | WGQGT LVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-39/D6\|6-6\|RF1/JH4 | 32580 | QLQLQES-GPGLVKPSEILSL TCTVSG-GSIS | SS---SYYWG | WIRQPPGK GLEWIG | SIYY---- SGSTYYNPS LKS | RVTISVDTSKNQFSLKLSSV TAADTAVYYCAR | EYSSS------------SYFDY | WGQGT LVTVS S |
| iPS:3 93990 | 21-225_11G7 | VH4\|4-39/D6\|6-6\|RF1/JH4 | QLQLQES-GPGLVKPSEILSL TCTVSG-GFIS | RS---TYYWG | WIRQPPGK GLEWIG | SIYY---- SGSTSYSPS LKS | RVTISVDISKNQFSLKLSSV TAADTAVYYCAR | LNSSW----------SFDY | WGQGT LVTVS S |
| | | Germline | H_FR1 | H_CDR1 | H_FR2 | H_CDR2 | H_FR3 | H_CDR3 | H_FR4 |
| | VH3\|3-30.3/D2\|2-15\|RF3/JH6 | 32581 | QVQLVES-GGGVVQPGRSLRL SCAASG-YTFS | S-----YAMH | WVRQAPGK GLEWVA | VISYD--- GSNKYYADS VKG | RFTISRDNSKNTLYLQMNSL RAEDTAVYYCAR | DIVVVVAAT--------YYYYYGMDV | WGQGT TVTVS S |
| iPS:3 94010 | 21-225_12G5 | VH3\|3-30.3/D2\|2-15\|RF3/JH6 | QVQLVES-GGGVVQPGRSLRL SCAASG-FTFS | N-----YGIY | WVRQAPGK GLEWVA | VISYD--- GSNKYYADS VKG | RFTISRDNSKNTLYLQMNSL RAEDTAVYYCAR | DRGAVAAY--------YYYGIDV | WGQGT TVTVS S |
| | | Germline | H_FR1 | H_CDR1 | H_FR2 | H_CDR2 | H_FR3 | H_CDR3 | H_FR4 |
| | VH1\|1-08/D2\|2-21\|RF1/JH4 | 32582 & 32583 | QVQLVQS-GAEVKKPGASVKV SCKASG-YTFT | S-----YDIN | WVRQATGQ GLEWMG | WMNPN--- SGNTGYAQK FQG | RVTMTRNTSISTAYMELSSL RSEDTAVYYCAR | SILWW*L----------LFYFDY | WGQGT LVTVS S |
| iPS:3 94065 | 21-225_11E2 | VH1\|1-08/D2\|2-21\|RF1/JH4 | QVQVVQS-GAEVKKPGASVKV SCKASG-YTFT | N-----YDIN | WVRQATGQ GLEWMG | WMNTN--- SGNTGYAQK FQG | RVTMTRNTSISTAYMDLSSL RSEDTAVYYCAY | SHGWF----------LFDY | WGQGI LVTVS S |
| iPS:3 98506 | 21-225_23G12 | VH1\|1-08/D2\|2-21\|RF1/JH4 | QVQLLQS-GAEVKKPGASVKV SCKASG-YTFT | N-----YDIN | WVRQATGQ GLEWMG | WMYPN--- SGNTGYAQK FQG | RVTMTMNTSISTAYMELSSL RSEDTAVYYCAI | SGGWY----------YFDY | WGQGT LVTVS S |
| iPS:3 98512 | 21-225_25E12 | VH1\|1-08/D2\|2-21\|RF1/JH4 | QVQLVQS-GAEVKKPGASVKV SCKASG-YTFT | N-----YDIN | WVRQATGQ GLEWMG | WMNPN--- SGNTGYAQK FQG | RVTMTRNTSISTAYLELSSL RSEDTAVYYCAG | SNGWY----------YFDY | WGQGT LVTVS S |
| | | Germline | H_FR1 | H_CDR1 | H_FR2 | H_CDR2 | H_FR3 | H_CDR3 | H_FR4 |
| | VH3\|3-33/D7\|7-27\|RF2/JH3 | 32584 & 32585 | QVQLVES-GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | *LGD----------AFDI | WGQGT MVTVS S |
| iPS:3 94091 | 21-225_13H3 | VH3\|3-33/D7\|7-27\|RF2/JH3 | QVQLVES-GGGLVQPGRSLRL SCAASG-FTFS | S-----YGMH | WVRQAPGK GLEWVA | VIWYE--- ESNKYYVDS VRG | RFTISRDNSKSTLYLQMNSL RAEDTAVYYCVR | ELGF----------QSDF | WGQGT PVTVS S |
| | | Germline | H_FR1 | H_CDR1 | H_FR2 | H_CDR2 | H_FR3 | H_CDR3 | H_FR4 |
| | VH3\|3-23/D4\|4-23\|RF2/JH6 | 32586 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | DYGGNSYY--------YYYGMDV | WGQGT TVTVS S |
| iPS:3 98472 | 21-225_16E4 | VH3\|3-23/D4\|4-23\|RF2/JH6 | EVQLLES-GGGLIQPGGSLRL SCAASG-FTFS | S-----YVMS | WVRQAPGK GLEWVS | TISVG--- GGTTYYADS VKG | RFTISRDNSKNTLYLQMNSL RAEDTAVYYCAK | WGRGNSYE--------YYYGMDV | WGQGT TVTVS S |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH2\|2-05/D6\|6-19\|RF2/JH4 | | 32587 | QITLKES-GPTLVKPTQILTL TCTFSG-FSLS | TS---GVGVG | WIRQPPGK ALEWLA | LIYW---- NDDKRYSPS LKS | RLTITKDTSKNQVVLTMTNM DPVDTATYYCAR | GIAVA---------- ---------GYFDY | WGQGT LVTVS S |
| iPS:3 98498 | 21-225_22E6 | VH2\|2-05/D6\|6-19\|RF2/J H4 | | QITLKES-GPTLVKPTQILTL TCTFSG-FSLS | TG---GVGVG | WIRQPPGK ALEWLA | LIYW---- NDDKRYSPS LKS | RLTITRDTSKNQVVLTMTNM DPVDTATYYCAH | TIAVR----------- ----------GFDY | WGQGT LVTVS S |
| | VH1\|1-08/D5\|5-24\|RF2/JH4 | | 32588 & 32589 | QVQLVQS-GAEVKKPGASVKV SCKASG-YTFT | S------YDIN | WVRQATGQ GLEWMG | WMNPN--- SGNTGYAQK FQG | RVTMTRNTSISTAYMELSSL RSEDTAVYYCAR | *RWLQ---------- ---------LYFDY | WGQGT LVTVS S |
| iPS:3 98536 | 21-225_33D12 | VH1\|1-08/D5\|5-24\|RF2/J H4 | | QVQLVQS-GAEVKKPGASVKV SCKASG-YTFT | S------YDIS | WVRLATGQ GLEWMG | WMNPN--- SGNTGYAQK FQG | RVTMTRNTSISTAYMELSSL RSEDTAVYYCAR | KRA------------- ----------NDY | WGQGT LVTVS S |
| | VH2\|2-05/D6\|6-6\|RF1/JH4 | | 32590 | QITLKES-GPTLVKPTQILTL TCTFSG-FSLS | TS---GVGVG | WIRQPPGK ALEWLA | LIYW---- NDDKRYSPS LKS | RLTITKDTSKNQVVLTMTNM DPVDTATYYCAH | EYSSS---------- ---------SYFDY | WGQGT LVTVS S |
| iPS:3 98546 | 21-225_9H10 | VH2\|2-05/D6\|6-6\|RF1/JH 4 | | QITLKES-GPTLVKPTQILTL TCTFSG-FSLT | TS---GVGVG | WIRQPPGK ALEWLA | LIYW---- SDDKRYSPS LKS | RLTITKDTSKNQVVLTMTNM APVDTATYYCAH | TGSSC----------- ---------CYFDY | WGQGT LVTVS S |
| | VH3\|3-21/D1\|1-1\|RF2/JH6 | | 32591 | EVQLVES-GGGLVKPGGSLRL SCAASG-FTFS | S------YSMN | WVRQAPGK GLEWVS | SISSS--- SSYIYYADS VKG | RFTISRDNAKNSLYLQMNSL RAEDTAVYYCAR | VQLERYY-------- --------YYYGMDV | WGQGT TVTVS S |
| iPS:4 02229 | 21-225_16H9 | VH3\|3-21/D1\|1-1\|RF2/JH 6 | | EVQLVES-GGGLVKPGGSLRL SCAASG-FTFS | S-----YSMN | WVRQAPGK GLEWVS | SISGS--- SSYIYYADS VKG | RFTISRDNAKNSLYLQMNSL RAEDTAVYYCAR | VNG------------ ----------MDV | WGQGT TVTVS S |
| | VH3\|3-21/D4\|4-11\|RF3/JH5 | | 32592 | EVQLVES-GGGLVKPGGSLRL SCAASG-FTFS | S------YSMN | WVRQAPGK GLEWVS | SISSS--- SSYIYYADS VKG | RFTISRDNAKNSLYLQMNSL RAEDTAVYYCAR | TTVIN---------- --------WFDP | WGQGT LVTVS S |
| iPS:4 02233 | 21-225_16D10 | VH3\|3-21/D4\|4-11\|RF3/J H5 | | EVQLVES-GGGLVKPGGSLRL SCAASG-FTFS | T-----YNLN | WVRQAPGK GLEWVS | SISGG--- AGHIYYSDS VKG | RFTISRDNAKNSLYLQMNSL RAEDTAVYYCAR | TNG------------ ----------FDF | WGQGT LVTVS S |
| | VH3\|3-21/D1\|1-1\|RF1/JH3 | | 32593 | EVQLVES-GGGLVKPGGSLRL SCAASG-FTFS | S-----YSMN | WVRQAPGK GLEWVS | SISSS--- SSYIYYADS VKG | RFTISRDNAKNSLYLQMNSL RAEDTAVYYCAR | GTTGT---------- ---------DAFDI | WGQGT MVTVS S |
| iPS:4 02235 | 21-225_20F10 | VH3\|3-21/D1\|1-1\|RF1/JH 3 | | EVQLVES-GGGLVKPGGSLRL SCAASG-FTFS | S-----YSMN | WVRQAPGK GLEWVS | SIST---- STFIYYADS VKG | RFTISRDNAKNSLYLQMNSL RAEDTAVYYCAR | KAG------------ ----------LDI | WGQGT MVTVS S |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-23/D5\|5-12\|RF1/JH3 | 32594 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | VLVAT--------- --------IDAFDI | WGQGT MVTVS S |
| iPS:4 03870 | 21-225_23G4 | VH3\|3-23/D5\|5-12\|RF1/JH3 | EVQLLES-GGGLVQPGGSLRL SCAASG-FTFS | S-----YAMS | WVRQAPGK GLEWVS | VISGR--- GGSTYYADS VKG | RFTISRDNSKNTLYLQMNSL RAEDTAVYYCAK | RGIVGA---------- --------TEAFDI | WGQGT MVTVS S |
| | VH4\|4-39/D4\|4-11\|RF1/JH4 | 32595 & 32596 & 32597 | QLQLQES-GPCLVKPSETLSL TCTVSG-GSIS | SS---SYYWG | WIRQPPGK GLEWIG | SIYY---- SGSTYYNPS LKS | RVTISVDISKNQFSLKLSSV TAADTAVYYCAR | *LQ*L----------- --------YFDY | WGQGT LVTVS S |
| iPS:4 03872 | 21-225_8F11 | VH4\|4-39/D4\|4-11\|RF1/JH4 | QLQLQES-GPGLVQPSETLSL TCTVSG-VSIS | RT---SYYWG | WLRQPPGK GLEWIG | NIYY---- SGSAYNNPS LKS | RVTISVDTSKNQFSLKLSSV TAADTAVYYCGR | HGQDW----------- --------GLDY | WGQGT LVTVS S |
| | VH1\|1-08/D3\|3-9\|RF2/JH6 | 32598 | QVQLVQS-GAEVKKPGASVKV SCKASG-YTFT | S-----YDIN | WVRQATGQ GLEWMG | WMNPN--- SGNTGYAQK FQG | RVTMTRNTSISTAYMELSSL RSEDTAVYYCAR | YYDILTGYYN------ ------YYYYYGMDV | WGQGT TVTVS S |
| iPS:4 37240 | 21-225_84H12 | VH1\|1-08/D3\|3-9\|RF2/JH6 | QVQLVQS-GAEVKKPGASVKV SCKASG-YTFT | S-----YDIN | WVRQATGQ GLEWMG | WLNPH--- SGNTGYAQK FQG | RITMTWNTSIRTAYMELSSL RSEDTAVYYCAR | GFYDILTGYS------ -----PTYYYYDMDV | WGQGT TVTVS S |
| iPS:4 34577 | 21-225_75C11 | VH1\|1-08/D3\|3-9\|RF2/JH6 | QVQLVQS-GAEVKKPGASVKV SCKASG-YTFT | S-----YDIN | WVRQATGQ GLEWMG | WLNPH--- SGNTGYAQK FQG | RITMTWNTSIRTAYMELSSL RSEDTAVYYCAR | GFYDILTGYS------ -----PTYYYYDMDV | WGQGT TVTVS S |
| iPS:4 34553 | 21-225_76H12 | VH1\|1-08/D3\|3-9\|RF2/JH6 | QVQLVQS-GAEVKKPGASVKV SCKASG-YTFT | S-----YDIN | WVRQATGQ GLEWMG | WLNPH--- SGNTGYAQK FQG | RITMTWNTSIRTAYMELSSL RSEDTAVYYCAR | GFYDILTGYS------ -----PTYYYYDMDV | WGQGT TVTVS S |
| iPS:4 34927 | 21-225_86E5 | VH1\|1-08/D3\|3-9\|RF2/JH6 | QVQLVQS-GAEVKKPGASVKV SCKASG-YTFT | S-----YDIN | WVRQATGQ GLEWMG | WMNPN--- SGNTGYAQK FQG | RVTMTWNTSIRTAYMELSSL RSEDTAVYYCAR | GFYDILTGYS------ -----PTYYYYDMDV | WGQGT TVTVS S |
| | VH3\|3-23/D6\|6-19\|RF2/JH4 | 32599 | EVQLLES-GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | GIAVA----------- --------GYFDY | WGQGT LVTVS S |
| iPS:4 35477 | 21-225_154E8 | VH3\|3-23/D6\|6-19\|RF2/JH4 | EVQLLES-GGGLVQPGGSLRL SCAASG-FTFS | S-----YAMS | WVRQAPGR GLEWVS | AISGS--- GGNTFYADS VKG | RFTISRDNSKNTLYLQMNSL RAEDTAVYYCAI | HGIAVAGT-------- --------GAHYFDY | WGQGT LATVS S |
| iPS:4 35385 | 21-225_149G7 | VH3\|3-23/D6\|6-19\|RF2/JH4 | EVQLLES-GGGLVQPGGSLRL SCAASG-FTFS | S-----YAMS | WVRQAPGK GLEWVS | AISGS--- GGNTFYADS VKG | RFTISRDNSKNTLYLQMNSL RAEDTAVYYCAI | HGIAVAGT-------- --------GAHYFDY | WGQGT LATVS S |

Figure 52 - Table 5
Standard IgG Antibody Variable Region Consensus Protein Alignment
CDRs defined by Kabat
Alignment numbering defined by Amgen Reference (AHo)

**KAPPA_VARIABLE**

| | | Germline | K_FR1 | K_CDR1 | K_FR2 | K_CDR2 | K_FR3 | K_CDR3 | K_FR4 |
|---|---|---|---|---|---|---|---|---|---|
| | VK4\|B3\|JK3 | | DIVMTQSPDSLA VSLGERATINC | KSS-- QSVLYSSNNKN YLA | WYQQKPGQ PPKLLIY | W------- -ASTRES | GVPDRFSGSGSG-- TDFTLTISSLQAEDVAV YYC | QQYYS-------------- -----------TPFT | FGPGTK VDIK |
| iPS:42 6126 | 21-225_6G6 | VK4\|B3\|JK3 | ............ <br> .......... | .........HN <br> ...Y.... | ........ <br> ..N...F | ........ <br> | ..........F...... <br> ..N............. | ....D............ <br> ............ | ..H... <br> .... |
| iPS:41 2232 | 21-225_4A2 | VK4\|B3\|JK3 | ............... <br> ........... | ........I.H. <br> ...N.... | .F..... <br> .....L. | ........ <br> | ............... <br> ........P........ | .....N........... <br> ............V. | .... <br> .G.. |
| iPS:45 1141 | 21-225_164B11 | VK4\|B3\|JK3 | ............... <br> .......... | .......L.K. <br> ....S... | S....... <br> L...... | ........ <br> ...S... | ............... <br> ............L... | ................ <br> ..........I.P. | ..H..N <br> ...T |
| iPS:42 3314 | 21-225_12F11 | VK4\|B3\|JK3 | ............... <br> .......... | ..........H. <br> ...Y.... | ........ <br> ..N...F | ........ <br> | ..........F...... <br> ..N............. | ....D............ <br> ............ | .... <br> .... |
| iPS:43 5327 | 21-225_147G6 | VK4\|B3\|JK3 | ............... <br> .......... | ........... <br> ..SN.... | ........ <br> | ........ <br> ...A... | ............... <br> ............... | ....T............ <br> ............P. | .... <br> .... |
| iPS:43 5345 | 21-225_148G3 | VK4\|B3\|JK3 | ............... <br> .........H. | ......R..H. <br> ...Y.... | ........ <br> | ........ <br> .....D. | ..............A.. <br> ............L... | ................ <br> ............... | .... <br> .... |
| iPS:43 5405 | 21-225_150B7 | VK4\|B3\|JK3 | ............... <br> .......... | ......N.... <br> .H.N.... | ........ <br> | ........ <br> .....K. | ....T............ <br> ............... | ................ <br> ............... | .... <br> .... |
| iPS:43 5433 | 21-225_152E3 | VK4\|B3\|JK3 | ............... <br> .......... | ..........H. <br> ...Y...V | ........ <br> S..R... | ........ <br> | S............... <br> ............... | ................ <br> ............... | .... <br> .... |
| iPS:43 5437 | 21-225_152F4 | VK4\|B3\|JK3 | ............... <br> .......... | ........... <br> ...Y.... | ........ <br> | ........ <br> .....K. | ...V............ <br> ...V........... | ...FN............ <br> ............P. | .... <br> .... |
| iPS:43 5649 | 21-225_165H2 | VK4\|B3\|JK3 | ...........T <br> ..P....... | .........H. <br> .......T | ........ <br> | ........ <br> | ...V............ <br> .VP...M.DD......R | ..S............... <br> ..........I.P. | .....N <br> .... |
| iPS:43 5855 | 21-225_191G3 | VK4\|B3\|JK3 | ............... <br> .........D. | .........H. <br> ..SY.... | .....L.. <br> | ........ <br> .....K. | ............... <br> ..............F... | ................ <br> ...........S.P. | M... <br> |
| iPS:43 5903 | 21-225_190E2 | VK4\|B3\|JK3 | ............... <br> .......... | .........FN <br> ....... | ........ <br> ..N.... | ........ <br> | ............... <br> ......M......... | ...C............ <br> ..........L... | ...... <br> ...R |
| iPS:43 5915 | 21-225_190H4 | VK4\|B3\|JK3 | AN.......... <br> .....T.... | .........H. <br> ...Y.... | ..R..... <br> | ........ <br> | ............... <br> ............... | ................ <br> ..........I.P. | ...... <br> .... |
| iPS:43 5923 | 21-225_190H6 | VK4\|B3\|JK3 | ............... <br> .......... | .........FN <br> ....... | ........ <br> ..N.... | ........ <br> | ..........C...... <br> ............... | ...C............ <br> ..........L... | ...... <br> ...R |
| iPS:43 5953 | 21-225_191B12 | VK4\|B3\|JK3 | ............... <br> .......... | .........FN <br> ....... | ........ <br> ....... | ........ <br> | ............... <br> .....N.......I... | ...S............ <br> ..........L... | ...... <br> .... |
| iPS:43 6098 | 21-225_195G11 | VK4\|B3\|JK3 | ............... <br> .......... | .........FN <br> ....... | ........ <br> ..N.... | ........ <br> ....L.. | ..........C...... <br> ......M......... | ...C............ <br> ..........F... | ...R <br> ...R |

| | | | K_FR1 | K_CDR1 | K_FR2 | K_CDR2 | K_FR3 | K_CDR3 | K_FR4 |
|---|---|---|---|---|---|---|---|---|---|
| iPS:43 6102 | 21-225_196B1 | VK4\|B3/JK3 | ............ / .F......... | .......I.F. / ....R... | ........ / ...I... | ........ / | ................. / | ...S............. / ..........L... | .... / .... |
| iPS:43 6104 | 21-225_196C1 | VK4\|B3/JK3 | ........... / ........... | .........FN / ........ | ........ / L.N.... | ........ / ....L.. | ..........C...... / ......M......... | ...C............. / .........F... | .... / ...R |
| iPS:43 6156 | 21-225_197C8 | VK4\|B3/JK3 | .....P...... / ........... | .........H. / ........ | ........ / ........ | ........ / ........ | ................. / ..S............. | ..S.T............ / ..........I... | .... / ..N. |
| iPS:43 6270 | 21-225_203F10 | VK4\|B3/JK3 | ........... / ........... | .......FFH / ........ | ........ / ........ | ........ / ........ | ................. / ..............T.... | ...F............. / ..........L... | .... / ...T |
| iPS:43 6570 | 21-225_225F4 | VK4\|B3/JK3 | ........... / ........... | .......... / .......... | ........ / ........ | ........ / ........ | ......T......... / .....CV.P....... | H..HN............ / ..........S.P. | ..H..E / .... |
| iPS:39 4065 | 21-225_11E2 | VK4\|B3/JK3 | ........... / ........... | ..N...R..S. / ...H.... | .....R... / ........ | ........ / ........ | ................. / ................. | ...F............. / ................. | .... / .... |
| | | Germline | K_FR1 | K_CDR1 | K_FR2 | K_CDR2 | K_FR3 | K_CDR3 | K_FR4 |
| | VK1\|A30/JK5 | | DIQMTQSPSSLS ASVGDRVTIIC | RAS--QGIR-- --NDLG | WYQQKPGK APKRLIY | A------ -ASSLQS | GVPSRFSGSGSG-- TEFTLTISSLQPEDFAT YYC | LQHNS------------ --------YPIT | FGQGTR LEIK |
| iPS:47 3253 | 21-225_7C3_LC1 | VK1\|A30/JK5 | ............ / ............ | ............ / .....S... | ....N.V. / ....... | ....... / ....... | ................. / ...V.........F... | ................. / .........YL... | .... |
| iPS:47 3256 | 21-225_9F12_LC2 | VK1\|A30/JK5 | ............ / ............ | ........ / ........ | ......V. / ....... | ....... / ....... | ................. / ................. | ................. / .........YL... | .... |
| iPS:45 3449 | 21-225_208A2 | VK1\|A30/JK5 | ............ / ............ | .T........ / ........ | ....Q... / T...... | ....... / .....L. | ................. / ..........R.....D. | ..Y.............. / ................P. | .... |
| iPS:43 4467 | 21-225_73H8 | VK1\|A30/JK5 | .........Y / ..........R | ......D.... / ........ | ........ / .L..V.. | ....... / ....... | ....S............ / ................G | I................ / .........YP... | V..... / .... |
| iPS:43 5045 | 21-225_90H5 | VK1\|A30/JK5 | ............ / ............ | ........ / ........ | ........ / ........ | I...... / ....... | ................. / ................. | ................. / ................. | .... |
| iPS:43 5561 | 21-225_159F1 | VK1\|A30/JK5 | ............ / ............ | ......RV... / ........ | ......A. / ....I.F | D...... / ...N... | ................. / ................. | ...H............. / ..........F... | .... |
| iPS:43 6328 | 21-225_207F12 | VK1\|A30/JK5 | ............ / ............ | ........ / ........ | ........ / ........ | ....... / ....... | ................F. / ................. | ..............L / | .... |
| iPS:43 6354 | 21-225_210G10 | VK1\|A30/JK5 | ............ / .........F | .T........ / ........ | ....Q... / T...M.. | ....... / .....F. | .........R.....D. / | ..Y.............. / ................P. | .... |
| iPS:39 3094 | 21-225_34C4 | VK1\|A30/JK5 | ............ / ............ | ........ / ........ | ........ / ........ | T...... / ...N... | ................. / ................. | ...S............. / ................ | .... |
| iPS:39 8484 | 21-225_18D4 | VK1\|A30/JK5 | ............ / ............ | ........ / ........ | ........ / ........ | .....E. / | ...V............. / | .H..N........... / .........YL... | .... |
| | | Germline | K_FR1 | K_CDR1 | K_FR2 | K_CDR2 | K_FR3 | K_CDR3 | K_FR4 |
| | VK1\|L5/JK3 | | DIQMTQSPSSVS ASVGDRVTIIC | RAS QGIS ----SWLA | WYQQKPGK APKLLIY | A -ASSLQS | GVPSRFSGSGSG TDFTLTISSLQPEDFAT YYC | QQANS ---------FPFT | FGPGTK VDIK |

| ID | Name | Germline | | | | | | |
|---|---|---|---|---|---|---|---|---|
| IPS:47 3254 | 21-225_7C3_LC2 | VK1\|L5/JK3 | . . . . . . . . . . | . . . . . . . . . . | . . . . . Q . . | . . . . . . . | .A. . . . . . . . . . . . . . . | . . . . . . . . . . . . . . . . . . | . . . . . |
| | | | . . L . . . . . . . | . . . . . . . . | . . . . . . F | . . . R . . . | . . . . . . . . . . . . . . . . . | . . . . . . . . . . . . . . . | . . . . |
| IPS:47 3255 | 21-225_9F12_LC1 | VK1\|L5/JK3 | . . . . . . . . . . | . . . . . . . . . . | . . . . . . . . | . . . . . . . | . . . . . . . . . . . . . . . . | . . . . . . . . . . . . . . . . . . | . . . . . |
| | | | . . . . . . . . . . | . . . . R . . . | . . . . . . . | . . . R . . . | . . . . . G . L . . . . . I . . . | . . . . . . . . . . . . . . . | . . F . |
| IPS:42 6108 | 21-225_10G6 | VK1\|L5/JK3 | . . . . . . . . . . | . . . . . . . . . . | . . . . . . . . | . . . . . . . | . . . A . . . . . . . . . . . . | . . . . . . . . . . . . . . . . . . | . . . . . |
| | | | . . . . . . . . . . | . . . . K . . . | . . . I . . . | . . Y . . . . | . . . . R . . . . . . . . . . . | . . . . . . . . . . . . . . . | . . . . |
| IPS:42 6110 | 21-225_12E9 | VK1\|L5/JK3 | . . . . . . . . . . | . . . . . . . . . . | . . . . . . . E | . . . . . . . | . . . . . . . . . . . . . . . . | . . . . . . . . . . . . . . . . . . | . . . . . |
| | | | . . . R . . . . . . | . . . . . . . . | . . . . . . . | . . . R . . . | . . . . . . . . . . . . . . . . | . . . . . . . . . . . . . . . | . . . . |
| IPS:45 3447 | 21-225_65F10 | VK1\|L5/JK3 | . . . . . . . . . . | . GG . . . . . . | . . . . . . . . | . . . . . . . | . . . . . . R . R . . . . . . . | . . G . I . . . . . . . . . . | . . R . . . |
| | | | . . . . . . . . . . | . . . . T . . . | . . . . . . . | . . . I . . . | . . . . . . . . . . . . . . . . | . . . . . . . . . . . . . . . | . . . . |
| IPS:45 3451 | 21-225_52G11 | VK1\|L5/JK3 | . . . . . . . . . . | . . . . . . . . . . | . . . . . . . . | . . . . . . . | . . . . . . . A . . . . . . . . | . . . . . . . . . . . . . . . . . . | . . . . . |
| | | | . . . . . . . . . . | . . . . K . . . | . . . . . L . | . . . . . . . | . . . . . . . . . . . . . . . . | . . . . . . . . . . . . . . . | . . V . |
| IPS:45 3453 | 21-225_53F2 | VK1\|L5/JK3 | . . . . . . . . . . | . . . . . . . . . . | . . . . . . . . | . . . . . . . | . . . K . . . . . . . . . . . . | . . . . . . . . . . . . . . . . . . | . . . . . |
| | | | . . . . . . . . . . | . . . . K . . . | . . N . . L . | . . . . . . . | . . . . . . . . . . . . . . . . | . . . . . . . . . . . . . . . | . . V . |
| IPS:43 3915 | 21-225_43H9 | VK1\|L5/JK3 | . . . . . . . . . . | . . . . . D . . . . | . . . K . . . . | D . . . . . . | . . . . . . . . . . . . . . . . | . . . . . . . . . . . . . . . . . . | . . . . . |
| | | | . . . . . . . . . . | . . . . . . . . | . . . . . . . | . . . . . . . | I . . . . . . . . . . . . . F . | . . . . . . . . . L . . . | . . . . |
| IPS:43 3925 | 21-225_44F3 | VK1\|L5/JK3 | . . . . . . . . . . | . . . . . . . . . . | . . . . . . . | . . . . . . . . | . . . . . . . . . . F . . . . . | . . . . . . . . . . . . . . . . . . | . . . . . |
| | | | . . . . . . . . . . | . . . . D . . . | . . . . . . . | . . . . . . . | . . . . . . . . . . . . . . . . | . . . . . . . . . . . . . . . | . . . . |
| IPS:43 3953 | 21-225_45H4 | VK1\|L5/JK3 | . . . . . . . . . . | . . . . . . D . . . | . . . K . . . . | D . . . . . . | . . . . . . . . . . . . . . . . | . . . . . . . . . . . . . . . . . . | . . . . . |
| | | | . . . . . . . . . . | . . . . . . . . | . . . Y . . . | . . . . . . . | I . . . . . . . . . . . . I . F . | . . . . . . . . . L . . . | . . . . |
| IPS:43 3959 | 21-225_45C9 | VK1\|L5/JK3 | . . . . . . . . . . | . . . . . . D . . . | . . . R . . . | . . . . . . . | . . . . . . . . . . . . . . . . | . . . . . . . . . . . . . . . . . . | . . . . . |
| | | | V . . . . . . . . . | . . . . D . . . | . . . . . . . | . . . . . E . | . . . . . . . . . . . . . . . . | . . . . . . . . . . . . . . . | . . . . |
| IPS:43 4023 | 21-225_49F1 | VK1\|L5/JK3 | . . . . . . . . . . | . . . . . RD . N . . | . . . . . . . | T . . . . . . | . . . . . . . . . . . . . . . . | . . S . . . . . . . . . . . . | . . . . . |
| | | | . . . . . . . . . . | . . . . G . . . | . . . . . . . | . V . . . . | . . . . . . . . . . . . . . . . | . . . . . . . . . . . . . . . | . . . . |
| IPS:43 4027 | 21-225_49H5 | VK1\|L5/JK3 | . . . . . . . . . . | . . . . . . . F . . . | . F . . . . . | . . . . . . . | . . . . . . . . . . . . . . . . | . T . . . . . . . . . . . . . | . . . . . |
| | | | . . . . . . . . . . | . . . . T . . . | . . . . . . . | . . . . . D | . . . . . . . . . . . . . . . . | . . . . . . . . . . . . . . . | . . . . |
| IPS:43 4035 | 21-225_49F10 | VK1\|L5/JK3 | . . . . . . . . . . | . . . . . . . . . . | . . . . . . . | . . . . . . . | . . . . . . . . . . . . . . . . | . . . . . . . . . . . . . . . . . . | . . . . . |
| | | | . . . . . . . . . . | . . . R . . . | . . . V . . . | . . . T . . . | . . . . . . . . . . . . . . . . | . . . . . . . . . . . . . . . | . . . . |
| IPS:43 4061 | 21-225_51C7 | VK1\|L5/JK3 | . . . . . . . . . . | . . . . . . DVN . . | . F . . . . . | . . . . . . . | . . . . . . . . . . . . . . . . | . . T . . . . . . . . . . . . | . . . . . |
| | | | . . . . . . . . . . | . . . . NY . . | . . . . . . . | . . . . . N | . . . . . . . L . . . . . . . . | . . . . . . . . . . . . . . . | . . . . |
| IPS:43 4065 | 21-225_50D4 | VK1\|L5/JK3 | . . . . . . . . . . | . . . . . . . . . . | . . . . . . . | . . . . . . . | . . . . . . . . . . . . . . . . | . . . . . . . . . . . . . . . . . . | . . . . . |
| | | | . . . . . . L . . . | . . . . R . . . | . . . V . . . | . . . T . . . | . . . . . . . . . . . . . . . . | . . . . . . . . . . . . . . . | . . . R |
| IPS:43 4069 | 21-225_51E9 | VK1\|L5/JK3 | . . . . . . . . . . | . . . . . . . . . | . . . . . . . | V . . . . . . | . . . . . . . . . . . . . . . . | . . . K . . . . . . . . . . . | . . . . . |
| | | | . . . . . . . . . . | . . . . . . . . | . . . . . . . | . . . . . . . | . . . . R . . . . . . . . . . . | . . . . . . . . . . . . . . . | . . . . |
| IPS:43 4079 | 21-225_52B1 | VK1\|L5/JK3 | . . . . . . . . . . | . . . . . . D . R . . | . . . . . . . | . . . . . . . | . . . . . . . . . . . . . . . . | . . . K . . . . . . . . . . . | . . . . . |
| | | | TF . . . . I . . . . | . . . . T . . . | . . . . . . . | . . . . . . N | . . . . . . . . . . . . . . F . | . . . . . . . . . . . . . . . | . . . . |
| IPS:43 4097 | 21-225_52H10 | VK1\|L5/JK3 | . . . . . . . . . . | . . . . . . D . N . . | . . . . . . . | V . . . . . . | .A . . . . . . . . . . . . . . . | . . . K . . . . . . . . . . . | . . . . . |
| | | | . . . . . . . . . . | . . . . . . . . | . . . . . . . | . . . . . . . | . . . . R . . . . . . . . . . . | . . . . . . . . . . . . . . . | . . . . |
| IPS:43 4123 | 21-225_53F7 | VK1\|L5/JK3 | . . . . S . . . . . . | . . . . . . . . . . | . . . . . . . | . . . . . . . | . . . . . . . . . . . . . . . . | . . . . . . . . . . . . . . . . . . | . . . . . |
| | | | . . . . . . . . . . | . . . . R . . . | . . N . . . . | . . . . . . . | . . . . . . . . . . . . . . I . . | . . . . . . . . . . . . . . . | . . . . |
| IPS:43 4145 | 21-225_55B1 | VK1\|L5/JK3 | . . . . . . . . . . | . . . . . . V . . . . | . . . . . . . | . . . . . . . | . . . . . . . . . . . . . . . . | . . . . . . . . . . . . . . . . . . | . . . . . |
| | | | . . . . . . . . . . | . . . . R . . . | . . N . . . . | . . . . . . . | . . . . . . . . . . . . . . . . | . . . . . . . . . . . . . . . | . . L . |

| ID | Clone | Gene | Seq1 | Seq2 | Seq3 | Seq4 | Seq5 | Seq6 | Seq7 |
|---|---|---|---|---|---|---|---|---|---|
| iPS:43 4167 | 21-225_50F3 | VK1\|L5/JK3 | .......... | ......D.... | .F..... | ....... | ................ | ..T............... | .... |
| | | | .......... | ........ | ....... | ......N | ................ | ................ | .... |
| iPS:43 4189 | 21-225_56E5 | VK1\|L5/JK3 | .......... | .........R.. | ....... | ....... | ................ | ................ | .... |
| | | | .......... | ....K... | ....... | ....... | ................ | ................ | .... |
| iPS:43 4193 | 21-225_56C6 | VK1\|L5/JK3 | .......... | ........ | .....S.N | ....... | ...............Y. | ................ | .... |
| | | | .......... | ........ | ....... | ...R... | ..I....S | ................ | .... |
| iPS:43 4195 | 21-225_56F6 | VK1\|L5/JK3 | .........C | .V....D.... | .F..... | V...... | .F.............. | ................ | .... |
| | | | .Y........ | ....K... | ..F... | ...G... | .F.............. | ................ | .... |
| iPS:43 4273 | 21-225_57E4 | VK1\|L5/JK3 | .......... | ......D.... | ....... | ....... | ................ | ..G............. | .... |
| | | | ......S... | ....N... | ....... | ....... | ...............S. | ................ | .... |
| iPS:43 4277 | 21-225_57A7 | VK1\|L5/JK3 | .......... | ............ | ....... | ....... | ................ | ................ | .... |
| | | | .......... | ....R... | ..N.... | ...N... | ................ | ................ | .... |
| iPS:43 4355 | 21-225_64G12 | VK1\|L5/JK3 | .......... | ......N.T.. | ....... | ....... | ................ | ................ | .... |
| | | | .......... | ....T... | ......S | ....... | ...............I. | ................ | L... |
| iPS:43 4389 | 21-225_66F11 | VK1\|L5/JK3 | .........C | .E........ | ....... | ....... | ..........Y..... | ................ | .... |
| | | | .......... | ....I... | ....... | ....... | ..........V..... | ................ | .... |
| iPS:43 4423 | 21-225_70D1 | VK1\|L5/JK3 | .......... | ......V... | ....... | ....... | ................ | ................ | .... |
| | | | .......... | ....R... | ....... | ....... | .V.............. | ................ | .... |
| iPS:43 5291 | 21-225_146E1 | VK1\|L5/JK3 | ..K....... | ........N.. | ....... | ....... | ......R......... | ................ | .... |
| | | | .......... | ....N..V | ....... | ....... | ................ | ................ | .... |
| iPS:43 5303 | 21-225_146A6 | VK1\|L5/JK3 | .......A... | ............ | ....... | ....... | ................ | ..TD............ | .... |
| | | | .......... | ....N... | ....... | ......G | ..S............. | ................ | .... |
| iPS:43 5335 | 21-225_147D10 | VK1\|L5/JK3 | .......A... | ......N.... | ....... | ....... | ................ | ..TD............ | .... |
| | | | .......... | ....N..T | ....... | ....... | ..S............. | ................ | ..V. |
| iPS:43 5339 | 21-225_147D12 | VK1\|L5/JK3 | .......A... | ............ | ...E.... | ....... | ................ | ..TD............ | .... |
| | | | .......... | ....N... | ....... | ....... | ..S............. | ................ | ..V. |
| iPS:43 5343 | 21-225_148E2 | VK1\|L5/JK3 | .......A... | ............ | ....... | ....... | ........NE...... | ..TD............ | .... |
| | | | .......... | ....N... | ....... | ....... | ..S............. | ................ | ..V. |
| iPS:43 5379 | 21-225_149B6 | VK1\|L5/JK3 | .......A... | ........I.. | ....... | ....... | ................ | ..G............. | .... |
| | | | .......... | ............ | ....... | ....... | ................ | ................ | .... |
| iPS:43 5381 | 21-225_149C6 | VK1\|L5/JK3 | ......A... | ............ | ......R | ....... | ...............Y | ..TD............ | .... |
| | | | .......... | ....N... | ....... | ......G | ..S............. | ................ | .... |
| iPS:43 5391 | 21-225_149F8 | VK1\|L5/JK3 | ......A... | ............ | ....... | ....... | ........NE...... | ..TD............ | .... |
| | | | .......... | ....N... | ....... | ....... | ..S..........I... | ................ | ..V. |
| iPS:43 5395 | 21-225_149D11 | VK1\|L5/JK3 | ......A... | ......N.... | ....... | ....... | ................ | ..TD............ | .... |
| | | | .......... | ....N..T | ....... | ....... | ..S............. | ................ | ..V. |
| iPS:43 5403 | 21-225_150C5 | VK1\|L5/JK3 | .......G... | ........N.. | ....... | ....... | ................ | ..TD............ | .... |
| | | | .......... | ....N... | ....... | ......G | ..S............. | ................ | .... |
| iPS:43 5447 | 21-225_152H7 | VK1\|L5/JK3 | .......A... | ......D.... | ....... | ....... | ................ | ..TD............ | .... |
| | | | .......... | ....N... | ....... | ......G | ..S..T.......... | ................ | .... |
| iPS:43 5453 | 21-225_152G10 | VK1\|L5/JK3 | ......A... | ............ | ....... | ....... | ........NE...... | ..TD............ | .... |
| | | | .......... | ....N... | ......S | ....... | ..S............. | ................ | ..V. |

| | | | | | | | | | |
|---|---|---|---|---|---|---|---|---|---|
| IPS:43 5483 | 21-225_155A4 | VK1\|L5/J K3 | .......A... | .......... | ..H..... | ....... | ............... | H.TD............. | .... |
| | | | .......... | ....N... | ....... | ......G | ..S.......... | ............. | .... |
| IPS:43 5485 | 21-225_155B4 | VK1\|L5/J K3 | .......A... | ......D.... | ........ | ....... | ......N........ | H.TD............. | .... |
| | | | .......... | ....N... | ....... | ......G | ..S.......... | ............. | .... |
| IPS:43 5787 | 21-225_180A3 | VK1\|L5/J K3 | .......... | ......D.T.. | ........ | ....... | ................ | ...........I... | ...N |
| | | | .......... | ........ | ....... | ....... | I...........F.H. | ............. | .... |
| IPS:43 5809 | 21-225_182H5 | VK1\|L5/J K3 | ..........Y | ......D.T.. | ........ | ....... | ................ | ..V.......... | ..H... |
| | | | .......... | ........ | ....... | ....... | I...T.V..D...... | ............. | .... |
| IPS:43 5889 | 21-225_186A11 | VK1\|L5/J K3 | .......... | ......D.T.. | ........ | ....... | ................ | ..V.......... | ..H... |
| | | | .......... | ........ | ....... | ....... | I...T.V..D...... | ............. | .... |
| IPS:43 5965 | 21-225_192H2 | VK1\|L5/J K3 | .......... | .......... | ........ | G...... | ................ | ..S.......... | .... |
| | | | .......... | ......I. | ....... | ...... | ............. | ..........V... | ..V. |
| IPS:43 6106 | 21-225_196F2 | VK1\|L5/J K3 | .......... | .......... | ........ | ....... | ................ | ..T.......... | .... |
| | | | .......... | .......... | ....... | ....... | ............. | ..........V... | .... |
| IPS:43 6360 | 21-225_210H11 | VK1\|L5/J K3 | .......... | ....I... | ..N.... | ....... | .......R........ | ...K.......... | .... |
| | | | .......... | .......... | ....... | ....... | ............. | ............. | .... |
| IPS:43 6488 | 21-225_221A6 | VK1\|L5/J K3 | .......... | .......... | ........ | T...... | ................ | ............. | .... |
| | | | .......... | ........ | ....... | ...N... | ............. | ............. | .... |
| IPS:43 6494 | 21-225_221F12 | VK1\|L5/J K3 | .......... | .......... | ........ | T...... | ................ | ............. | .... |
| | | | .......... | ........ | ....... | ...N... | .......R........ | ............. | .... |
| IPS:43 6496 | 21-225_222E1 | VK1\|L5/J K3 | .......... | .......... | ........ | T...... | ................ | ............. | ..R... |
| | | | .......... | ........ | ....... | ...N... | ............. | ............. | .... |
| IPS:43 6508 | 21-225_222F7 | VK1\|L5/J K3 | .......... | .......... | ........ | T...... | ................ | ............. | .... |
| | | | .......... | ........ | ....... | ...N... | ......V........ | ............. | .... |
| IPS:43 6516 | 21-225_222C12 | VK1\|L5/J K3 | ......C..... | .V........ | ........ | T...... | ................ | ..D........... | ..R... |
| | | | .......... | .......... | .L..V.. | ...N... | ......V.R...... | ............. | .... |
| IPS:43 7234 | 21-225_64E2 | VK1\|L5/J K3 | .......... | .......... | ....... | ....... | ................ | ............. | .... |
| | | | .......... | ....R... | ....... | ....... | ............. | ............. | .... |
| IPS:39 2996 | 21-225_28B1 | VK1\|L5/J K3 | .......... | ......A.N.. | ....... | ....... | ................ | ...S.......... | .... |
| | | | .......... | ....D... | ....... | ....F.. | ....T........ | ............. | .... |
| IPS:39 3010 | 21-225_25E11 | VK1\|L5/J K3 | .......... | .......... | ........ | T...... | ................ | ............. | .... |
| | | | .......... | ....N... | ....... | ......G | .IS.......... | ............I. | .... |
| IPS:39 3016 | 21-225_28F11 | VK1\|L5/J K3 | .......... | .......... | ........ | ........ | ......R........ | ............. | .... |
| | | | .......... | ....N... | ......S | ...N.... | ....T.........C. | ..........L... | .... |
| IPS:39 3024 | 21-225_31H9 | VK1\|L5/J K3 | .......... | ........T.. | ....R... | D...... | ................ | ..G........... | ..Q... |
| | | | .......... | .......T | ....... | .T..... | IF........... | ............. | .... |
| IPS:39 3080 | 21-225_34F3 | VK1\|L5/J K3 | .......... | .......... | ....... | ....... | ................ | ............. | .... |
| | | | .T......S.. | ....K... | ....... | ....... | ...........S.... | ............. | .... |
| IPS:39 3084 | 21-225_35C6 | VK1\|L5/J K3 | .......... | .......... | ........ | ....... | ...T.......... | ............. | .... |
| | | | .......... | ....K... | ...P... | ....... | ............I... | ............. | .... |
| IPS:39 3086 | 21-225_36H5 | VK1\|L5/J K3 | .......... | .......... | ........ | ....... | .I............ | ............. | .... |
| | | | .......... | ....R... | ..E.... | ...R... | ............. | ............. | .... |

| ID | Clone | Germline | K_FR1 | K_CDR1 | K_FR2 | K_CDR2 | K_FR3 | K_CDR3 | K_FR4 |
|---|---|---|---|---|---|---|---|---|---|
| iPS:39 3098 | 21-225_35G6 | VK1\|L5/JK3 | ............ | ........... | .....V.. | ........ | ...............A. | ................. | ...... |
| | | | ......L.... | ....R... | V...... | ...R... | ....G........... | ................. | ..L. |
| iPS:39 3112 | 21-225_33G1 | VK1\|L5/JK3 | ............ | ........... | ........ | G....... | ................ | ................. | ...... |
| | | | V.......... | ....R... | ....... | ..Y.... | ................ | ................. | .... |
| iPS:39 3116 | 21-225_34G7 | VK1\|L5/JK3 | ............ | ......L.... | ........ | ........ | ...L............ | ................. | ...... |
| | | | ........... | ....K... | ....... | ....... | ................ | ................. | .... |
| iPS:39 3132 | 21-225_33H7 | VK1\|L5/JK3 | ............ | ........... | .....V.. | ........ | ................ | ....I............ | ...... |
| | | | ........... | ....R... | V...... | ...R... | ....G........... | ................. | ..L. |
| iPS:39 3140 | 21-225_35H12 | VK1\|L5/JK3 | ............ | ........... | ........ | ........ | .I.............. | ................. | ...... |
| | | | ........... | ....R... | ..E.... | ...R... | ................ | ................. | .... |
| iPS:39 3954 | 21-225_4H6 | VK1\|L5/JK3 | ............ | ........... | ........ | G....... | ................ | ................. | ...... |
| | | | ........... | ....R... | ....... | ....... | ...............I.... | ................. | .... |
| iPS:39 8502 | 21-225_23B11 | VK1\|L5/JK3 | ............ | ........T.. | ........ | ........ | R.......R....... | ................. | ...... |
| | | | ........... | ....K... | ...V... | ....... | ................ | ................. | .... |
| iPS:39 8520 | 21-225_31C4 | VK1\|L5/JK3 | ............ | ........... | ........ | ........ | ...T............ | ................. | ...... |
| | | | ........... | ....K... | ...P... | ....... | ...............I.... | ................. | .... |
| iPS:40 2223 | 21-225_30A11 | VK1\|L5/JK3 | ............ | ........... | .......R | ........ | .I.............. | ................. | ...... |
| | | | ........... | ....R... | ..E.... | ...R... | ................ | ................. | ..N. |
| **Germline** | | | **K_FR1** | **K_CDR1** | **K_FR2** | **K_CDR2** | **K_FR3** | **K_CDR3** | **K_FR4** |
| | **VK4\|B3/JK1** | | DIVMTQSPDSLA VSLGERATINC | KSS-- QSVLYSSNNKN YLA | WYQQKPGQ PPKLLIY | W------- -ASTRES | GVPDRFSGSGSG-- TDFTLTISSLQAEDVAV YYC | QQYYS--------------- ---------TPWT | FGQGTK VEIK |
| iPS:42 6112 | 21-225_12F12 | VK4\|B3/JK1 | ........... | ......T..F. | ........ | ........ | ................. | ................. | ...... |
| | | | ........... | ...NH... | ..N.... | .....A. | ................. | ..........S... | .... |
| iPS:45 1137 | 21-225_74A7 | VK4\|B3/JK1 | ........... | ..........F. | ....R... | ........ | ................. | ...H........... | .......T |
| | | | ........... | ...Y.... | ..N.... | ....... | .........A...... | .........S.P. | .Q.. |
| iPS:43 3909 | 21-225_43D8 | VK4\|B3/JK1 | ........... | .........MT | ....R... | ........ | ..........G.... | ................. | ...... |
| | | | ........... | ..D....T | ....... | ....... | .....G.......... | ............P. | .... |
| iPS:43 4177 | 21-225_56A1 | VK4\|B3/JK1 | ........... | .........H. | ....R... | ........ | ................. | ................. | ...... |
| | | | ........... | .......V | ..N.... | ....... | ................. | ............P. | .... |
| iPS:43 4237 | 21-225_61B5 | VK4\|B3/JK1 | ........... | ........... | ...L.... | ........ | ................. | ................. | ....S. |
| | | | ........... | ...N.S.T | ...K... | ....... | ................. | ............P. | .... |
| iPS:43 4285 | 21-225_57A11 | VK4\|B3/JK1 | ...........T | ..........H. | ....R... | ........ | ................. | ................. | ...... |
| | | | ........... | ...Y.... | ....V... | .....A. | ...........M..... | ................. | ..F. |
| iPS:43 4295 | 21-225_58B9 | VK4\|B3/JK1 | ........... | ..G....I.... | ........ | ........ | ...A............ | ................. | ....S. |
| | | | ........... | ...N.... | ...K... | .....D. | ................. | ............P. | .... |
| iPS:43 4321 | 21-225_59F10 | VK4\|B3/JK1 | ......F..... | ......T...R | ........ | ........ | ................. | ...F........... | ...... |
| | | | ........... | ...Y.... | ....... | ....... | ................. | ............P. | .... |
| iPS:43 4431 | 21-225_70E7 | VK4\|B3/JK1 | ........... | ........... | ........ | ........ | ................. | ....N.......... | ...... |
| | | | .......L.. | ...N.... | ....... | ....... | ................. | .........I.P. | .... |
| iPS:43 4475 | 21-225_74F9 | VK4\|B3/JK1 | ........... | ........... | ........ | ........ | ................. | ................. | ...... |
| | | | ........... | ...Y.... | ...K... | ....... | .................S. | .........S.P. | .... |

| | | | | | | | | | |
|---|---|---|---|---|---|---|---|---|---|
| iPS:43 4477 | 21-225_74A6 | VK4\|B3/JK1 | ............ | .........H. | ....... | ....... | ................ | ...F............ | ......Q |
| | | | ..P........ | ...N.... | ..D.... | ....... | ................ | ............. | .... |
| iPS:43 4481 | 21-225_74B10 | VK4\|B3/JK1 | ............ | .........H. | ....... | ....... | ................ | ............. | ..... |
| | | | ............ | .......T | ....... | ....... | S...G........... | ..........I.P. | .... |
| iPS:43 4487 | 21-225_76G2 | VK4\|B3/JK1 | ............ | .........H. | ....R... | ....... | ................ | ............. | ..... |
| | | | ............ | ......Y.... | ..R.... | ....... | ........V....... | ..........S.P. | .... |
| iPS:43 4493 | 21-225_76F3 | VK4\|B3/JK1 | ......C...P. | .........F. | ....R... | ....... | ................ | ...H............ | ......T |
| | | | ............ | ...Y.... | .HD.... | ....... | .........A....... | ..........S.L. | .Q.. |
| iPS:43 4509 | 21-225_76F5 | VK4\|B3/JK1 | V..L........ | .........H. | ....... | ....... | ................ | ............. | ..... |
| | | | ............ | ..SY.... | S..V... | .T..... | ................ | ..........S.P. | .... |
| iPS:43 4525 | 21-225_76E8 | VK4\|B3/JK1 | ............ | ......T..H. | ....... | ....... | ................ | ...F............ | ..... |
| | | | ............ | ...Y.... | ...V... | .T..... | ................ | ..........S.L. | .... |
| iPS:43 4549 | 21-225_76E11 | VK4\|B3/JK1 | ............ | ..R......H. | .....A.. | ....... | ................ | ............. | ..... |
| | | | .....G..... | ...Y.... | ....... | ....... | ................ | ............P. | ...R |
| iPS:43 4551 | 21-225_75C4 | VK4\|B3/JK1 | ............ | .......I... | .F...... | ....... | ................ | ....I............ | ..... |
| | | | ............ | ...N.... | ....... | ....... | ................ | ............P. | .... |
| iPS:43 4575 | 21-225_77C7 | VK4\|B3/JK1 | ............ | ......T..H. | ....... | ....... | ................ | ...F............ | ......R |
| | | | ............ | ...Y.... | ...V.L. | .T..... | ................ | ..........S.P. | .... |
| iPS:43 4597 | 21-225_77C10 | VK4\|B3/JK1 | ........C.. | ..........T | ....... | ....... | ................ | .H..N........... | .V.. |
| | | | ............ | ...N.... | ....... | ....... | ................ | ...............K | ...T |
| iPS:43 4617 | 21-225_74B8 | VK4\|B3/JK1 | .S......... | .........H. | ....... | ....... | ................ | ....R........... | ..... |
| | | | ............ | ..K..... | ....... | ....... | ................ | ............. | .... |
| iPS:43 4619 | 21-225_78C1 | VK4\|B3/JK1 | ........C.. | ..........T | ....... | ....... | ................ | .H..N........... | .V.... |
| | | | ............ | ...N.... | ....... | ....... | ................ | ...............K | .... |
| iPS:43 4639 | 21-225_74B7 | VK4\|B3/JK1 | ............ | ......T..H. | ....... | ....... | ................ | ...F............ | ..... |
| | | | ............ | ...Y.... | ...V... | .T..... | ................ | ..........S.P. | .... |
| iPS:43 4649 | 21-225_78E11 | VK4\|B3/JK1 | ............ | ............ | ....... | ....... | ................ | ............. | ..... |
| | | | ............ | F..Y.... | ...K... | ....... | ...............S. | ..........S.P. | .... |
| iPS:43 4653 | 21-225_74B5 | VK4\|B3/JK1 | ............ | .........F. | ....R... | ....... | ................ | ............. | ......T |
| | | | ............ | ...Y.... | ..N.... | ....... | .........A....... | ..........S.P. | .Q.. |
| iPS:43 4655 | 21-225_78H12 | VK4\|B3/JK1 | ............ | ......T..H. | ....... | ....... | ................ | ...F............ | ..... |
| | | | ............ | F..Y.... | ...V... | .T..... | ................ | ..........S.P. | .... |
| iPS:43 4665 | 21-225_74G4 | VK4\|B3/JK1 | ............ | ............ | ....A.. | ....... | ................ | ............. | ..... |
| | | | ............ | ..N.... | ....... | ....... | ................ | ..........I.P. | .... |
| iPS:43 4675 | 21-225_79G6 | VK4\|B3/JK1 | ........C.. | M........H. | ....... | ....... | ................ | ............. | ..... |
| | | | .........S. | F......T | ....... | ....W.. | S.P.G........... | ..........I.P. | .... |
| iPS:43 4685 | 21-225_79E9 | VK4\|B3/JK1 | ............ | .......I... | .F...... | ....... | ...............C. | ....I........... | ..... |
| | | | ............ | ...N.... | ....... | ....... | ................ | ............P. | .... |
| iPS:43 4689 | 21-225_79G10 | VK4\|B3/JK1 | ............ | ..........F. | ....R... | ....... | ................ | ...H............ | ......T |
| | | | ............ | ...Y.... | .HN.... | ....... | .........A....... | ..........S.L. | .Q.. |
| iPS:43 4697 | 21-225_79F12 | VK4\|B3/JK1 | ............ | .........I... | ....... | ....... | ................ | ............. | ..... |
| | | | ............ | ...Y.... | ....... | ....... | ................ | ............. | .... |

| iPS ID | Clone | Gene | | | | | | | |
|---|---|---|---|---|---|---|---|---|---|
| iPS:43 4707 | 21-225_80D3 | VK4\|B3/JK1 | ........C..<br>........... | ..........T<br>...N.... | .......<br>....... | .......<br>....... | ................<br>................ | .H.NE............<br>............GK | .V.V..<br>...T |
| iPS:43 4711 | 21-225_80H3 | VK4\|B3/JK1 | ...........<br>........T.. | .........HR<br>...Y.... | .......<br>....... | .......<br>....... | ................<br>................ | ................<br>..........P. | ....<br>.... |
| iPS:43 4731 | 21-225_80E9 | VK4\|B3/JK1 | ........C..<br>........... | ..........T<br>...N.... | .......<br>....... | .......<br>....... | ................<br>................ | ...N............<br>........... | .V....<br>.... |
| iPS:43 4761 | 21-225_81E5 | VK4\|B3/JK1 | ...........<br>........... | .........F.<br>...Y.... | ....R...<br>..N.... | .......<br>....... | ................<br>........A...... | ................<br>.........S.L. | .....T<br>.Q.. |
| iPS:43 4771 | 21-225_81F9 | VK4\|B3/JK1 | ........C..<br>........... | ..........T<br>...N.... | .......<br>....... | .......<br>....... | ................<br>..............R | .H.ND............<br>............GK | .V..IM<br>...T |
| iPS:43 4827 | 21-225_83F3 | VK4\|B3/JK1 | ........C..<br>........... | ..........T<br>...N.... | .......<br>....... | .......<br>....... | ................<br>................ | .H.ND............<br>...........K | .V..I.<br>.... |
| iPS:43 4829 | 21-225_83G3 | VK4\|B3/JK1 | ........C..<br>........... | ..........T<br>...N.... | .......<br>....... | .......<br>....... | ................<br>................ | .H..N............<br>........... | .V....<br>.... |
| iPS:43 4841 | 21-225_83G7 | VK4\|B3/JK1 | ...........<br>........I. | ......T..H.<br>...Y.... | ......<br>..V... | .T....<br>....... | ................<br>................ | ...F............<br>.........S.L. | ......<br>.... |
| iPS:43 4863 | 21-225_84G7 | VK4\|B3/JK1 | .........P.<br>.........I. | ......T..H.<br>...Y.... | ......<br>...V... | .T....<br>....... | ................<br>................ | ...F............<br>.........S.P. | ......<br>.... |
| iPS:43 4877 | 21-225_85H2 | VK4\|B3/JK1 | .SM........<br>........... | .........H.<br>..K..... | .......<br>....... | .......<br>....... | ................<br>................ | ...R............<br>........... | ......<br>.... |
| iPS:43 4901 | 21-225_85H9 | VK4\|B3/JK1 | ...........<br>........T.. | .........HR<br>...Y.... | .......<br>....... | .......<br>....... | ................<br>................ | ................<br>..........P. | ......<br>.... |
| iPS:43 4935 | 21-225_86E9 | VK4\|B3/JK1 | ......C...P.<br>........... | .........HR<br>...Y.... | ......<br>..N... | .......<br>....... | ................<br>........A...... | ...H............<br>.........S.L. | .....T<br>.... |
| iPS:43 4965 | 21-225_88A1 | VK4\|B3/JK1 | N..........<br>....A...... | .........H.<br>...Y...T | ......<br>.....L. | .......<br>.....K. | ................<br>...............F. | ................<br>.........S.P. | ......<br>.... |
| iPS:43 4971 | 21-225_88G2 | VK4\|B3/JK1 | ........C..<br>........... | ..........T<br>...N.... | .......<br>....... | .......<br>....... | ................<br>................ | ....N............<br>........... | .V....<br>.... |
| iPS:43 4973 | 21-225_88B4 | VK4\|B3/JK1 | ...........<br>........... | ..........I<br>...N.... | .......<br>....... | .......<br>....... | ...A............<br>................ | ................<br>..........P. | ......<br>.... |
| iPS:43 4997 | 21-225_88C10 | VK4\|B3/JK1 | ...........<br>........... | .........H.<br>...W.... | .H.....<br>......H | .......<br>..F..K. | ........G.....N.<br>................ | ....R............<br>..........A.P. | ......<br>.... |
| iPS:43 5051 | 21-225_90D9 | VK4\|B3/JK1 | ...........<br>.........I. | ......T..H.<br>..Y.... | ......<br>..V... | .T....<br>....... | ................<br>................ | ...L............<br>.........S.L. | ......<br>.... |
| iPS:43 5053 | 21-225_75F9 | VK4\|B3/JK1 | .........P<br>........V.. | .........HN<br>...N.... | .......<br>....... | .......<br>....... | ................<br>................ | ................<br>.........S.P. | ......<br>.... |
| iPS:43 5071 | 21-225_91F1 | VK4\|B3/JK1 | ........C..<br>........... | ..........T<br>...N.... | .......<br>....... | .......<br>....... | ................<br>................ | .H..N............<br>...........K | .V....<br>.... |
| iPS:43 5087 | 21-225_91G8 | VK4\|B3/JK1 | ........C..<br>........... | ..........T<br>...N.... | .......<br>....... | .......<br>....... | ................<br>................ | ....T............<br>........... | ......<br>.... |
| iPS:43 5113 | 21-225_92E6 | VK4\|B3/JK1 | ...........<br>A.......... | ......NI.S.<br>.......T | .......<br>...I... | .......<br>.T..... | .........F......<br>................ | ...F............<br>.........V.P. | ......<br>.... |

| | | | | | | | | | |
|---|---|---|---|---|---|---|---|---|---|
| iPS:43 5167 | 21-225_92F12 | VK4\|B3/JK1 | ............ | ........HR | ....... | ....... | ................. | ................. | .... |
| | | | ........T.. | ...Y.... | ....... | ....... | ................. | .............P. | .... |
| iPS:43 5203 | 21-225_75A7 | VK4\|B3/JK1 | ............ | ......T..H. | ....... | ....... | ................. | ...F............. | .... |
| | | | ............ | ...Y.... | ...V... | .T..... | ................. | .........S.P. | .... |
| iPS:43 5209 | 21-225_75A10 | VK4\|B3/JK1 | N........... | .........HN | ....... | ....... | ................. | ................. | .... |
| | | | ....A...... | ...Y...T | .....L. | ....K. | ..............F. | .........S.P. | .... |
| iPS:43 5211 | 21-225_94E11 | VK4\|B3/JK1 | ............ | .........F. | ....R... | ....... | ................. | ..H.............. | .....T |
| | | | ............ | ...Y.... | ..N.... | ....... | .........A....... | .........S.L. | .Q.. |
| iPS:43 5215 | 21-225_94E12 | VK4\|B3/JK1 | ............ | ........HR | ....... | ....... | ................. | ................. | .... |
| | | | ........T.. | ...Y.... | ....... | ....... | ................. | .............P. | .... |
| iPS:43 5227 | 21-225_95G4 | VK4\|B3/JK1 | ......C.... | .........FR | ....R... | ....... | .........Y....... | ..H.............. | .....T |
| | | | ............ | ...Y.... | .HN.... | ....... | .....V..A....... | .........S.L. | .Q.. |
| iPS:43 5245 | 21-225_95E12 | VK4\|B3/JK1 | ............ | ............ | ....... | ....... | ................. | ................. | .... |
| | | | ............ | ...A.... | ..N.F.. | ....... | ................. | ...........CS | .... |
| iPS:43 5249 | 21-225_96E2 | VK4\|B3/JK1 | .S.......... | .........H. | ....... | ....... | ................. | ....R............ | .... |
| | | | ............ | ..K..... | ....... | ....W. | ................. | ................. | .... |
| iPS:43 5255 | 21-225_96D5 | VK4\|B3/JK1 | ............ | .........H. | ....... | ....... | ................. | ................. | .....T |
| | | | ............ | ...Y.... | ..N.... | ....... | .........A....... | .........S.P. | .... |
| iPS:43 5257 | 21-225_96H5 | VK4\|B3/JK1 | ............ | ............ | ....... | ....... | ................. | ................. | .... |
| | | | ............ | .SH.... | ....... | ..I... | .S...M....... | ...........CS | .... |
| iPS:43 5267 | 21-225_96D10 | VK4\|B3/JK1 | ............ | ......NI.S. | ....... | ....... | .........F...... | ...F............ | .... |
| | | | ............ | ......T | ...I... | .T..... | ................. | .........V.P. | .... |
| iPS:43 5279 | 21-225_97H4 | VK4\|B3/JK1 | .........C.. | ..........T | ....... | ....... | ................. | .H.ND............ | .V.... |
| | | | ............ | ...N.... | ....... | ....... | ................. | .............K | ...T |
| iPS:43 5321 | 21-225_147E4 | VK4\|B3/JK1 | ............ | .........H. | ...L..R. | ....... | .........R....... | ................. | ..P... |
| | | | ............ | ...Y.... | ....... | ....... | .............I... | .............S. | .... |
| iPS:43 5353 | 21-225_148F8 | VK4\|B3/JK1 | ......L.... | .......A.H. | ....... | ....... | ................. | ................. | .... |
| | | | ............ | ...Y.... | ....... | .....K. | ................. | ..........I.P. | .... |
| iPS:43 5369 | 21-225_149A2 | VK4\|B3/JK1 | ............ | ............ | ....... | ....... | ................. | ................. | .... |
| | | | ............ | P..N.... | ....... | ....... | ................. | ...........CS | .... |
| iPS:43 5373 | 21-225_149E3 | VK4\|B3/JK1 | ............ | ......T..HN | ....... | ....... | ................. | ................. | .... |
| | | | .........S. | ...H..F. | ....... | ....LR. | ................. | .............P. | .... |
| iPS:43 5375 | 21-225_149H4 | VK4\|B3/JK1 | ............ | .........S. | ......R | ....... | ................. | H................ | .... |
| | | | ............ | ..DN.... | ....... | .S..... | ................. | ..........Y.P. | .... |
| iPS:43 5481 | 21-225_154A11 | VK4\|B3/JK1 | ............ | R.......H. | ....... | ....... | ................. | ...F............ | .... |
| | | | ............ | ...Y.... | ....... | ...K.D. | ................. | .........S.R. | .... |
| iPS:43 5557 | 21-225_158B12 | VK4\|B3/JK1 | .........P. | ......N..H. | ....R... | ....... | .............E. | ................. | .... |
| | | | ............ | ...N.... | ....... | ....... | ................. | .............P. | .... |
| iPS:43 5627 | 21-225_162F6 | VK4\|B3/JK1 | ............ | ......N..H. | ....R... | ....... | .............E. | ................. | .... |
| | | | ............ | ...N...T | ....... | ....... | ................. | .............P. | .... |
| iPS:43 5701 | 21-225_170F6 | VK4\|B3/JK1 | ............ | .........H. | ....R... | ....... | .........V...... | ................. | .... |
| | | | ....A...... | ...Y.... | ...V..H | .....K. | ....N........... | ............. | .... |

| ID | Clone | Gene | | | | | | |
|---|---|---|---|---|---|---|---|---|
| iPS:43 5737 | 21-225_174G5 | VK4\|B3/JK1 | `..........`<br>`....A......` | `.........H.`<br>`...Y...T` | `....S..`<br>`.......` | `.......`<br>`.......` | `................`<br>`...............N.` | `....R.............`<br>`................` | `....`<br>`....` |
| iPS:43 5751 | 21-225_175D10 | VK4\|B3/JK1 | `..........`<br>`..........` | `..........`<br>`...N....` | `.......`<br>`..N....` | `.......`<br>`.T.....` | `...............N.`<br>`................` | `.................`<br>`...........P.` | `....`<br>`....` |
| iPS:43 5773 | 21-225_177B12 | VK4\|B3/JK1 | `..........`<br>`.......V..` | `.........H.`<br>`...N...T` | `.......`<br>`.......` | `.......`<br>`.......` | `................`<br>`................` | `................`<br>`..........S.P.` | `....`<br>`....` |
| iPS:43 5801 | 21-225_181E5 | VK4\|B3/JK1 | `..........`<br>`..........` | `.........H.`<br>`...Y...T` | `.......`<br>`.......` | `.......`<br>`.......` | `................`<br>`................` | `H..FI.............`<br>`................` | `....`<br>`....` |
| iPS:43 5841 | 21-225_191D8 | VK4\|B3/JK1 | `..M.......`<br>`......I.S.` | `R........H.`<br>`...Y....` | `.......H`<br>`.......` | `.......`<br>`.......` | `................`<br>`................` | `.................`<br>`...........P.` | `..L...`<br>`....` |
| iPS:43 5925 | 21-225_190D7 | VK4\|B3/JK1 | `..........`<br>`..........` | `..........S.`<br>`...Y...V` | `.......`<br>`.......` | `.......`<br>`.....K.` | `................`<br>`.........D......` | `....R.............`<br>`................` | `....`<br>`....` |
| iPS:43 6021 | 21-225_193G4 | VK4\|B3/JK1 | `..........`<br>`..........` | `..........`<br>`...Y...T` | `.......`<br>`.......` | `.......`<br>`.....K.` | `................`<br>`.........D......` | `....I.............`<br>`................` | `......`<br>`.D..` |
| iPS:43 6114 | 21-225_196G8 | VK4\|B3/JK1 | `.........T`<br>`.......V..` | `.........H.`<br>`...N....` | `.......`<br>`.......` | `.......`<br>`.......` | `................`<br>`..A............` | `....N.............`<br>`...........P.` | `......`<br>`......` |
| iPS:43 6150 | 21-225_197H4 | VK4\|B3/JK1 | `..M....S...T`<br>`.......I.S.` | `R........H.`<br>`F..Y....` | `.....A.H`<br>`..N....` | `.......`<br>`.......` | `................`<br>`..P............` | `.................`<br>`...........P.` | `..L...`<br>`....` |
| iPS:43 6154 | 21-225_197C6 | VK4\|B3/JK1 | `..M.......`<br>`.......I.S.` | `R........H.`<br>`...Y....` | `.......H`<br>`..N....` | `.......`<br>`.......` | `................`<br>`................` | `.................`<br>`...........P.` | `..L...`<br>`....` |
| iPS:43 6218 | 21-225_200G7 | VK4\|B3/JK1 | `.........PT`<br>`A.......VK.` | `.........H.`<br>`...N....` | `.......`<br>`.......` | `.......`<br>`.......` | `................`<br>`..A............` | `....N.............`<br>`...........P.` | `....`<br>`....` |
| iPS:43 6272 | 21-225_201F5 | VK4\|B3/JK1 | `........E...`<br>`..........` | `..........`<br>`.........V` | `.......`<br>`.......` | `.......`<br>`.......` | `................`<br>`................` | `.................`<br>`...........P.` | `....`<br>`....` |
| iPS:43 6400 | 21-225_213H7 | VK4\|B3/JK1 | `..........`<br>`..........` | `......N..DI`<br>`.....S.G` | `.F.....`<br>`......N` | `.......`<br>`.......` | `................`<br>`........T......H.` | `....N.............`<br>`..........I.P.` | `..R...`<br>`....` |
| iPS:43 6402 | 21-225_213H12 | VK4\|B3/JK1 | `..........`<br>`.........T.` | `......N..KT`<br>`...R....` | `.......`<br>`...V...` | `.......`<br>`.......` | `................`<br>`..I............` | `H.................`<br>`...........I...` | `....`<br>`....` |
| iPS:43 6500 | 21-225_222H3 | VK4\|B3/JK1 | `..........`<br>`..........` | `........K.`<br>`.HR....` | `.......`<br>`..Q....` | `.......`<br>`......T` | `................`<br>`............S..S.` | `....S.............`<br>`...........I...` | `......`<br>`...N` |
| iPS:43 6520 | 21-225_223G10 | VK4\|B3/JK1 | `..........`<br>`..........` | `.......I.L.`<br>`.......` | `.H.....`<br>`.......` | `.......`<br>`.......` | `................`<br>`................` | `L..F.............`<br>`................` | `....`<br>`....` |
| iPS:43 6544 | 21-225_224H5 | VK4\|B3/JK1 | `..........`<br>`..........` | `..........`<br>`...F...T` | `.......`<br>`.......` | `.......`<br>`.......` | `................`<br>`....N...........` | `.................`<br>`...........P.` | `....`<br>`....` |
| iPS:43 6550 | 21-225_224D8 | VK4\|B3/JK1 | `..........`<br>`.......A...` | `..........`<br>`.......` | `.......`<br>`.......` | `.......`<br>`.S...K.` | `................`<br>`................` | `...F.............`<br>`...........P.` | `....`<br>`....` |
| iPS:43 6574 | 21-225_225F5 | VK4\|B3/JK1 | `..........`<br>`..........` | `......N...N`<br>`...N....` | `.......`<br>`..N....` | `.......`<br>`.......` | `................`<br>`.............F.` | `.................`<br>`..........S.P.` | `......`<br>`...N` |
| iPS:43 6586 | 21-225_225F11 | VK4\|B3/JK1 | `..........`<br>`..........` | `..........`<br>`...Y....` | `....R...`<br>`.......` | `.......`<br>`.......` | `...............P..`<br>`................` | `....T.............`<br>`...........P.` | `....`<br>`....` |
| iPS:43 6600 | 21-225_226F6 | VK4\|B3/JK1 | `..........`<br>`..........` | `.......I...`<br>`...Y....` | `.......`<br>`.......` | `.......`<br>`.......` | `................P.`<br>`................` | `....T.............`<br>`...........P.` | `....`<br>`....` |

| | | | K_FR1 | K_CDR1 | K_FR2 | K_CDR2 | K_FR3 | K_CDR3 | K_FR4 |
|---|---|---|---|---|---|---|---|---|---|
| iPS:43 6616 | 21-225_226D11 | VK4\|B3/J K1 | ............<br>............ | ......N..H.<br>..SN...V | .......<br>....... | .......<br>....... | ......R..........<br>................ | ....K..............<br>................ | ......<br>.... |
| iPS:43 6622 | 21-225_226A12 | VK4\|B3/J K1 | ............<br>............ | ......N....<br>...N.... | ....T....<br>.....F. | .......<br>.....K. | ................<br>................ | ................<br>..........S.P. | ......<br>.... |
| iPS:43 6638 | 21-225_227C7 | VK4\|B3/J K1 | ............<br>............ | R.....I..SD<br>...N.... | .......<br>..N.... | .......<br>....... | ................<br>...............F. | ................<br>..........S.P. | ......<br>.... |
| iPS:43 7356 | 21-225_74B1 | VK4\|B3/J K1 | ........F..<br>.......T.. | .........HR<br>...Y.... | .......<br>....... | .......<br>....... | ................<br>................ | ................<br>...........P. | ......<br>.... |
| iPS:43 7361 | 21-225_74C1 | VK4\|B3/J K1 | ......C...P.<br>.......S... | .......I.H.<br>...Y.... | .......H<br>.H..... | .......<br>....... | ..........Y......<br>................ | ................<br>................ | ......<br>.... |
| iPS:43 7379 | 21-225_74H2 | VK4\|B3/J K1 | ............<br>............ | .........H.<br>.......T | .......<br>.H..... | .......<br>....... | S...G...........<br>................ | ................<br>...........I.P. | ......<br>.... |
| iPS:44 6094 | 21-225_77E1 | VK4\|B3/J K1 | ............<br>............ | ......T..H.<br>...Y.... | .......<br>...V... | .......<br>.T..... | ..H.............<br>................ | H..L............<br>..........S.L. | ......<br>.... |
| iPS:45 1116 | 21-225_164A4 | VK4\|B3/J K1 | ......Y...R.<br>.........K. | ..........<br>.......T | .......<br>....F.. | .......<br>....... | ...........C.....<br>.........V...... | ...F............<br>................ | ......<br>.... |
| iPS:45 1124 | 21-225_74F6 | VK4\|B3/J K1 | ............<br>............ | ......NI.S.<br>.......T | .......<br>...I... | .......<br>.T..... | .........F......<br>................ | ...F............<br>..........V.L. | ......<br>.... |
| iPS:45 1127 | 21-225_164A7 | VK4\|B3/J K1 | ......C.....<br>............ | .........H.<br>...N.... | .......<br>.H..... | .......<br>.T..... | ...........Y.....<br>S...A........... | ................<br>..........I.L. | ......<br>.... |
| iPS:45 1129 | 21-225_94D2 | VK4\|B3/J K1 | ............<br>............ | .........H.<br>.......T | .......<br>.H..... | .......<br>....... | ................<br>S...G...H....... | ...H.............<br>..........I.P. | ..H...<br>.... |
| iPS:45 1133 | 21-225_95H4 | VK4\|B3/J K1 | ......C.....<br>............ | .........F.<br>...Y.... | ....R...<br>.HN.... | .......<br>....... | ................<br>..........A....... | ...H.............<br>..........S.L. | .....T<br>.Q.. |
| iPS:39 2786 | 21-225_24E1 | VK4\|B3/J K1 | ............<br>............ | .........T<br>...N...T | .......<br>R.N.... | .......<br>....... | ..F.............<br>................ | ................<br>...........P. | ......<br>.... |
| iPS:39 2886 | 21-225_23A12 | VK4\|B3/J K1 | ............<br>............ | ..........<br>...N.... | .......<br>....... | .......<br>.T..... | ................<br>................ | ...D............<br>...........P. | ......<br>.... |
| iPS:39 2928 | 21-225_25A4 | VK4\|B3/J K1 | ......F.....<br>.........K. | ..........<br>.H.N.... | .......<br>....L.. | .......<br>....... | ..............E.<br>......E........ | ................<br>...........P. | ......<br>.... |
| iPS:39 2960 | 21-225_29E6 | VK4\|B3/J K1 | ......F.....<br>............ | ..........<br>.H.NY..T | .......<br>.H...L. | .......<br>...S... | ..............E.<br>................ | ................<br>...........P. | ......<br>.... |
| iPS:39 2992 | 21-225_26C4 | VK4\|B3/J K1 | ............<br>............ | .........R<br>...Y.... | ...H....<br>....... | .......<br>....... | ................<br>................ | ................<br>...........P. | ......<br>..F. |
| iPS:39 3368 | 21-225_29H8 | VK4\|B3/J K1 | ............<br>............ | R.....TI.H.<br>...Y.... | .......<br>....... | .......<br>....... | ................<br>................ | ...C............<br>...........P. | ......<br>.... |
| iPS:39 3942 | 21-225_11E5 | VK4\|B3/J K1 | ............<br>.......L.. | ..........<br>...N...T | .......<br>R...... | .......<br>....... | ..............N.<br>................ | ................<br>...........P. | ......<br>.... |
| iPS:39 8506 | 21-225_23G12 | VK4\|B3/J K1 | ............<br>............ | ......I.F.<br>...N.... | .......<br>....... | .......<br>....... | ................<br>...............F. | ...S............<br>................ | ......<br>.... |
| | Germline | K_FR1 | K_CDR1 | K_FR2 | K_CDR2 | K_FR3 | K_CDR3 | K_FR4 | |

| | VK1\|A30/JK2 | | DIQMTQSPSSLS ASVGDRVTITC | RAS--QGIR-- ----NDLG | WYQQKPGK APKRLIY | A------- -ASSLQS | GVPSRFSGSGSG-- TEFTLTISSLQPEDFAT YYC | LQHNS-------------- ----------YPYT | FGQGTK LEIK |
|---|---|---|---|---|---|---|---|---|---|
| iPS:42 6114 | 21-225_28H2 | VK1\|A30/ JK2 | ..........<br>..I........ | ..........<br>........ | .......<br>....... | .......<br>....... | .................<br>................. | ...Y...........<br>...........RS | .... |
| iPS:42 6116 | 21-225_29E2 | VK1\|A30/ JK2 | ..........<br>.......... | ......A....<br>........ | ....R...<br>....... | .......<br>....... | .................<br>I................ | ...YN..........<br>...........RS | .... |
| iPS:43 4231 | 21-225_61F2 | VK1\|A30/ JK2 | ..........<br>.......V.. | ..........<br>....D... | ..E....<br>....... | .......<br>....... | .................<br>................. | ...Y...........<br>...........RS | .... |
| iPS:43 5401 | 21-225_150E2 | VK1\|A30/ JK2 | ..........<br>........S. | .......G..<br>........ | .......<br>..T.... | .......<br>....... | .................<br>........A........ | ...Y...........<br>.........F..S | .... |
| iPS:43 5445 | 21-225_152F7 | VK1\|A30/ JK2 | ..........<br>.......... | ..........<br>........ | .......<br>....... | .......<br>.T..... | .................<br>................. | ...YN..........<br>.........F..S | .... |
| iPS:43 5763 | 21-225_176H12 | VK1\|A30/ JK2 | ..........<br>.......... | ..........<br>........ | ..N....<br>....... | .......<br>....... | .................<br>.......R.......G | ...............<br>...........RS | .... |
| iPS:43 5767 | 21-225_177B4 | VK1\|A30/ JK2 | ..........<br>.......... | ..........<br>........ | .......<br>....... | .......<br>....... | .................<br>................. | ...Y...........<br>.........F.RS | .... |
| iPS:39 2974 | 21-225_26A11 | VK1\|A30/ JK2 | .......I...<br>.......... | ......A....<br>........ | ....R...<br>....... | .......<br>....... | .................<br>I................ | ...YN..........<br>...........RS | ...R |
| iPS:39 3046 | 21-225_25A12 | VK1\|A30/ JK2 | ..........<br>.......V.. | ......A....<br>....D... | ....R...<br>....... | .......<br>....... | .................<br>I................ | ...YN..........<br>...........RS | .... |
| | Germline | K_FR1 | DIQMTQSPSSLS ASVGDRVTITC | K_CDR1<br>RAS QSIS ----SYLN | K_FR2<br>WYQQKPGK APKLLIY | K_CDR2<br>A----- -ASSLQS | K_FR3<br>GVPSRFSGSGSG TDFTLTISSLQPEDFAT YYC | K_CDR3<br>QQSYS ---------TPLT | K_FR4<br>FGGGTK VEIK |

| | VK1\|O12/JK4 | | DIQMTQSPSSLS ASVGDRVTITC | RAS QSIS ----SYLN | WYQQKPGK APKLLIY | A----- -ASSLQS | GVPSRFSGSGSG TDFTLTISSLQPEDFAT YYC | QQSYS ---------TPLT | FGGGTK VEIK |
|---|---|---|---|---|---|---|---|---|---|
| iPS:42 6118 | 21-225_7A10 | VK1\|O12/ JK4 | ..........<br>.......... | ......N.Y..<br>........ | .......R<br>....V.. | S.......<br>.T..... | .................<br>S....N......S.... | ..............<br>..........P... | ......<br>...R |
| iPS:42 6124 | 21-225_32D6 | VK1\|O12/ JK4 | ..........<br>T......... | ......N.I..<br>........ | .....M.<br>...R... | V.......<br> | .................<br>........A........ | ..............<br>..........Y. | .A.. |
| iPS:45 1135 | 21-225_64A11 | VK1\|O12/ JK4 | ........F...<br>.......... | .....R.V...<br>....R... | ....TL..<br>.L....S | V.......<br>...R... | .................<br>......V.R......F. | ...D..........<br>..........F... | .... |
| iPS:43 4011 | 21-225_48B10 | VK1\|O12/ JK4 | ..........<br>.......... | ........R..<br>....K... | ...KT...<br>....... | .......<br>....... | .................<br>......V......... | ..T...........<br>..........N... | ..FT |
| iPS:43 4015 | 21-225_48F12 | VK1\|O12/ JK4 | ..........<br>.......... | ........R..<br>....K... | ...KT...<br>....... | .......<br>....... | .................<br>.............N... | ..T...........<br>..........N... | .....E<br>...T |
| iPS:43 4017 | 21-225_48G12 | VK1\|O12/ JK4 | ..........<br>.......... | ........R..<br>....K... | ...KT...<br>....... | .......<br>....... | .................<br>......V......N... | ..T...........<br>..........N... | .....E<br>...T |
| iPS:43 4165 | 21-225_50F2 | VK1\|O12/ JK4 | ........L..<br>......I.... | ..........<br>........ | .......<br>....... | V.......<br>....F.. | .................<br>........D........ | ..............<br>..........P... | .... |
| iPS:43 4191 | 21-225_56B6 | VK1\|O12/ JK4 | ..........<br>V......... | ......F..<br>....R... | .......R<br>......F | .......<br>....F.. | .................<br>................. | ..T...........<br>..........P... | .... |
| iPS:43 4247 | 21-225_62D2 | VK1\|O12/ JK4 | ..........<br>.........I. | .......I..<br>........ | .......<br>....... | .......<br>.T..... | .................<br>................. | ..T...........<br>..........P... | .... |

| | | | | | | | | |
|---|---|---|---|---|---|---|---|---|
| iPS:43 4335 | 21-225_63C10 | VK1|O12/ JK4 | ...........<br>T.......... | .......F..<br>.......H | .......<br>......S | .......<br>....... | ................<br>.............F.. | ..T............P...<br>.........P... | ......<br>.... |
| iPS:43 4341 | 21-225_64F7 | VK1|O12/ JK4 | ...........<br>........... | ......N.K..<br>....K... | .......<br>..F... | G......<br>....... | ................<br>.............A... | ....N...........<br>.........ISF. | ......<br>..L. |
| iPS:43 5295 | 21-225_148H1 | VK1|O12/ JK4 | ...........<br>........... | ..........<br>....D... | .......<br>...V... | T......<br>.T..... | ................<br>................ | ....–<br>.....STP. | ......<br>.... |
| iPS:43 5307 | 21-225_146E9 | VK1|O12/ JK4 | ...........<br>........... | ..........<br>....D... | ...L....<br>...V... | T......<br>.T..... | ................<br>.............F. | ....–<br>.....STP. | ......<br>.... |
| iPS:43 5347 | 21-225_148C4 | VK1|O12/ JK4 | ...........<br>........... | .......I..<br>....N... | .......<br>...V... | T......<br>....... | ................<br>..........T.... | ....–<br>.....STP. | ......<br>...E |
| iPS:43 5355 | 21-225_148H9 | VK1|O12/ JK4 | ...........<br>........... | ..........<br>....N... | .......<br>...V... | I......<br>....... | ................<br>................ | ....–<br>.....STP. | ......<br>.... |
| iPS:43 5371 | 21-225_149A3 | VK1|O12/ JK4 | ...........<br>........... | ..........<br>........ | ....VM.<br>...VM.. | T......<br>....... | ................<br>....N........... | ....–<br>.....SIP. | ......<br>.... |
| iPS:43 5415 | 21-225_150C11 | VK1|O12/ JK4 | ...........<br>........... | ..........<br>........ | .......<br>....... | T......<br>....... | ................<br>................ | ....–<br>.....SIY. | ....S.<br>.... |
| iPS:43 5419 | 21-225_150C12 | VK1|O12/ JK4 | ...........<br>........... | ..........<br>....D... | .......<br>...V... | T......<br>.T..... | ................<br>................ | ...F–<br>.....STP. | ......<br>.... |
| iPS:43 5425 | 21-225_151B12 | VK1|O12/ JK4 | ...........<br>........... | ..........<br>....NF.. | .......<br>...V... | T......<br>.....E. | .........E......<br>................ | ....–<br>.....STP. | .....R<br>.... |
| iPS:43 5431 | 21-225_152D2 | VK1|O12/ JK4 | .....L......<br>........... | ..........<br>....D... | ...L....<br>...V... | T......<br>.T..... | ................<br>.............F. | ....–<br>.....STP. | ......<br>.... |
| iPS:43 5439 | 21-225_152G4 | VK1|O12/ JK4 | ...........<br>........... | ..........<br>....D... | .......<br>...V... | T......<br>.T..... | ................<br>.............F. | ....–<br>.....STP. | ......<br>.... |
| iPS:43 5455 | 21-225_152B11 | VK1|O12/ JK4 | ...........<br>........... | ..........<br>....D... | .......<br>...V... | T......<br>.T..... | ................<br>.............F. | ....–<br>.....STP. | ......<br>.... |
| iPS:43 5487 | 21-225_155C4 | VK1|O12/ JK4 | ...........<br>........... | ..........<br>........ | .......<br>...V..F | T......<br>....... | ................<br>................ | ....–<br>.....STP. | .....R<br>.... |
| iPS:43 5503 | 21-225_156E4 | VK1|O12/ JK4 | ...........<br>........... | ..........<br>........ | .......<br>...V... | T......<br>....... | ................<br>................ | ....–<br>.....SAP. | ......<br>.... |

| iPS:43 5563 | 21-225_159H2 | VK1\|O12/ JK4 | ...⎵.......... | .......... | ....... | ....... | ................ | .................... | .... |
| | | | .......... | ....K... | ..E.... | .T.N... | ......T......V.... | ..........L.V. | .... |
| iPS:43 6110 | 21-225_196F4 | VK1\|O12/ JK4 | .F..I...... | ......R.H.. | ....... | T...... | ................ | ....G............. | .... |
| | | | .......... | ....... | ....... | ......G | ................ | ..........S... | .... |
| iPS:43 6244 | 21-225_201H10 | VK1\|O12/ JK4 | .......... | .....HN.N.. | .....S.. | ....... | ................ | ................ | .... |
| | | | .......... | ....... | ....... | ....... | ...F............ | ..........F... | ..MR |
| iPS:43 6262 | 21-225_203E3 | VK1\|O12/ JK4 | .........P. | .....HN.N.. | .....S.. | ....... | ................ | ................ | .... |
| | | | .........R | ....... | ....... | ....... | ...F............ | ..........F... | ..MR |
| iPS:43 6276 | 21-225_204H4 | VK1\|O12/ JK4 | .....L....P. | .....HN.N.. | .....S.. | ....... | ................ | ................ | .... |
| | | | .F.......R | ....... | ....... | ....... | ...F............ | ..........F... | ..MR |
| iPS:43 6280 | 21-225_204D6 | VK1\|O12/ JK4 | .S.......... | .....R.VH.. | ....... | G...... | ................ | ................ | .... |
| | | | .......... | ....T... | ...V... | ......R | ................V..... | ..........S... | ...Q |
| iPS:43 6312 | 21-225_206A4 | VK1\|O12/ JK4 | .....L....P. | .....HN.N.. | .....S.. | ....... | ................ | ................ | .... |
| | | | .F.......R | ....... | .....C | ....... | ...F............ | ..........F... | ..MR |
| iPS:43 6316 | 21-225_206A5 | VK1\|O12/ JK4 | .........P. | .....HN.N.. | .....S.. | ....... | ................ | ................ | .... |
| | | | .........R | ....... | .....C | ....... | ...F............ | ..........F... | ..MR |
| iPS:43 6338 | 21-225_208E8 | VK1\|O12/ JK4 | .........P. | .....HN.N.. | .....S.. | ....... | ................ | ................ | .... |
| | | | .F.......R | ....... | ....... | ....... | ...F............ | ..........F... | ..MR |
| iPS:43 6344 | 21-225_208B11 | VK1\|O12/ JK4 | .........P. | .....HN.N.. | .....S.. | ....... | ................ | ................ | .... |
| | | | .......... | ....... | ....... | ....... | ...F............ | ..........F... | ..MR |
| iPS:43 6358 | 21-225_210D11 | VK1\|O12/ JK4 | .......... | .....HN.N.. | .....S.. | ....... | ................ | ................ | .... |
| | | | .......... | ....... | ....... | ....... | ...F............ | ..........F... | ..MR |
| iPS:43 7282 | 21-225_207C9 | VK1\|O12/ JK4 | .N.......... | ......RF... | ....... | T...... | ......A.V..... | ................ | .... |
| | | | .......... | ....N... | ....... | ....... | ................ | ..........I... | .... |
| iPS:39 2636 | 21-225_17A6 | VK1\|O12/ JK4 | .......... | ......T.... | ..H.... | ....... | ................ | ...HT........... | .... |
| | | | .......... | ....N... | ....... | ....... | ................ | ..........S... | .... |
| iPS:39 2648 | 21-225_16D11 | VK1\|O12/ JK4 | .......... | ......T.... | ..H.... | ....... | ................ | ...H........... | .... |
| | | | .......... | ....N... | ....... | ....... | ................ | ..........S... | .... |
| iPS:39 2664 | 21-225_20F6 | VK1\|O12/ JK4 | ........I.. | ........I.. | ....... | T...... | ................ | ..T............. | .... |
| | | | .......... | ....T... | ..V..H | ....... | ................ | ..........P... | .... |
| iPS:39 2738 | 21-225_18G4 | VK1\|O12/ JK4 | ..H....... | ........I.. | ....... | T...... | ....G.......... | ..T............. | .... |
| | | | .......... | ....... | ...V... | ......T | ................ | ..........P... | .... |
| iPS:39 2798 | 21-225_22C7 | VK1\|O12/ JK4 | .......... | ......N.I.. | ......E. | I...... | ................ | ..T............. | .... |
| | | | .......... | ....... | ......H | ....... | ................ | ................ | .... |
| iPS:39 2922 | 21-225_30G4 | VK1\|O12/ JK4 | .......P... | ..T...N.F.. | .H..... | T...... | ................ | .L.............. | .... |
| | | | T......... | ....... | ......H | ......G | ....I.M.....S.... | ..........P.Y. | .... |
| iPS:39 3002 | 21-225_30G1 | VK1\|O12/ JK4 | .......... | ......N.Y.. | ....... | ....... | ................ | ................ | .... |
| | | | .......... | ....... | D...... | .....H. | ................ | ................ | .... |
| iPS:39 3042 | 21-225_31F1 | VK1\|O12/ JK4 | .......... | ......R.... | ....... | ....... | ................ | ....I........... | ......T |
| | | | .......... | ....... | ......F | ....S.. | ................ | ................ | ...R |
| iPS:39 3066 | 21-225_34D3 | VK1\|O12/ JK4 | .......... | ......N.Y.. | ....... | ....... | ................ | ................ | .... |
| | | | .......... | ....... | D...... | .....H. | .............F.. | ............. | .... |

| | | Germline | K_FR1 | K_CDR1 | K_FR2 | K_CDR2 | K_FR3 | K_CDR3 | K_FR4 |
|---|---|---|---|---|---|---|---|---|---|
| iPS:39 3082 | 21-225_34C11 | VK1\|O12/JK4 | ......F..... | ......N.R.. | ......E. | G....... | .........F...... | ..TC.............. | ...... |
| | | | T.......S.. | ....NF.. | D...Q.. | ...T... | .........K...... | .............. | .... |
| iPS:39 3092 | 21-225_33C12 | VK1\|O12/JK4 | T.......... | ........I.. | ........ | V....... | ......N.... | .................. | ...... |
| | | | T.......... | ........ | ........ | ......G | .................... | ............Y. | .... |
| iPS:39 3100 | 21-225_36B8 | VK1\|O12/JK4 | .......... | ........I.. | ........ | V....... | .................... | .................. | ...... |
| | | | T.......... | ........ | ........ | ...... | .................... | ............Y. | M... |
| iPS:39 3108 | 21-225_34G11 | VK1\|O12/JK4 | .......... | ......N.N.. | ........ | G....... | .................... | ..T.I............. | ...... |
| | | | .......... | ....R... | ........ | ...... | .........T...... | .............. | .... |
| iPS:39 3122 | 21-225_33B2 | VK1\|O12/JK4 | .......... | ........I.. | ........ | V....... | .................... | .................. | ...... |
| | | | T....K..... | ........ | ........ | ...... | .................... | ............Y. | .... |
| iPS:39 3134 | 21-225_34C2 | VK1\|O12/JK4 | .......... | ......R.I.. | .F...... | V....... | .................Y. | .................. | ...... |
| | | | TF......... | ........ | ........ | ...... | .................... | ............Y. | M... |
| iPS:39 3136 | 21-225_34D8 | VK1\|O12/JK4 | .......... | ......I.I.. | ........ | V....... | .................... | .................. | ...... |
| | | | T.......... | ........ | ......F | ...... | .................... | ............Y. | .... |
| iPS:39 3840 | 21-225_3F8 | VK1\|O12/JK4 | .F........ | ........L.. | ..R....R | T....... | .................V. | ..T............... | .....R |
| | | | .......... | ........ | ..QV..H | .T..... | .................... | .............. | ...N |
| iPS:39 3844 | 21-225_3G7 | VK1\|O12/JK4 | .......... | ......N.Y.. | ...GR..R | ........ | .................... | .................. | ....A. |
| | | | .......V.. | ....R... | ....M.. | ....S.. | .V................. | ............P.F. | ...D |
| iPS:39 3852 | 21-225_12A10 | VK1\|O12/JK4 | .V........ | ......N.Y.. | .......R | T....... | ...............A.. | .................. | ...... |
| | | | ..A....... | ........ | ...V..H | ...... | ....N............. | ............P... | .... |
| iPS:39 3900 | 21-225_10E12 | VK1\|O12/JK4 | .......... | ......N.Y.. | ...E...R | ........ | ......G.......... | ..N............... | ...... |
| | | | .......... | ........ | ........ | .T..... | .................... | ............P... | ...E |
| iPS:39 3920 | 21-225_1H12 | VK1\|O12/JK4 | .......... | ......N.Y.. | ...E...R | T....... | .........D........ | .................. | ...... |
| | | | .......... | ....R... | ........ | ...... | .................... | ............P... | ...... |
| iPS:39 3926 | 21-225_4G4 | VK1\|O12/JK4 | .........A | ......T.I.. | ........ | T....... | .................. | ..T............... | ...... |
| | | | .......... | ........ | ......H | ...... | ...S............. | .............. | ...... |
| iPS:39 3930 | 21-225_7E11 | VK1\|O12/JK4 | .......... | ......N.I.. | ........ | T....... | .................. | ..T............... | ...... |
| | | | ........A. | ........ | ..F... | ...... | .............I... | .............. | .... |
| iPS:39 3932 | 21-225_10F5 | VK1\|O12/JK4 | .......... | ......N.Y.. | ...E...R | T....... | .........D........ | .................. | ...... |
| | | | .......... | ....R... | ........ | ...... | .................... | ............P... | .... |
| iPS:39 3964 | 21-225_6G1 | VK1\|O12/JK4 | .......... | .T....N.I.. | ........ | T....... | ....G............ | ..PH.............. | ...... |
| | | | .......... | ........ | ..V... | ...N..T | .................... | ............P... | .... |
| iPS:39 4012 | 21-225_15A3 | VK1\|O12/JK4 | .......... | ........I.. | ..L... | T....... | .................. | ..T............... | ...... |
| | | | .......... | ........ | ..F... | ...... | .................... | .............. | .... |
| iPS:39 4016 | 21-225_13D4 | VK1\|O12/JK4 | .L........ | ........F.. | ........ | T....... | .................. | ..T............... | ...... |
| | | | .......... | ........ | ......C | ......N | .................... | ............L... | .... |
| iPS:39 4083 | 21-225_16E6 | VK1\|O12/JK4 | .......... | ........I.. | ........ | T....... | .................. | ..T............... | ...... |
| | | | .......... | ........ | ..F... | .T..... | .................... | .............. | .... |
| iPS:39 8480 | 21-225_17G4 | VK1\|O12/JK4 | .......... | .T....N.... | ........ | V....... | ...............E. | ..NF.............. | ...... |
| | | | ..A....... | ....N... | ........ | ....FP. | .................... | ............F... | ...I |
| iPS:39 8486 | 21-225_19A1 | VK1\|O12/JK4 | .......... | ......HT.T.. | ........ | .T.N... | .F...........I... | ...N.............. | .... |
| | | | .......... | ........ | ..F... | | | ............F... | |

| | VK2\|A18/JK 4 | | DIVMTQTPLSLS VTPGQPASISC | KSS-- QSLLHSD- GKIYLY | WYLQKPGQ SPQLLIY | E------- VSSRFS | GVPDRFSGSGSG-- TDFTLKISRVEAEDVGV YYC | MQGIH-------------- --------LPLT | FGGGTK VEIK |
|---|---|---|---|---|---|---|---|---|---|
| IPS:45 1139 | 21-225_71A6 | VK2\|A18/ JK4 | .............. | ........R. | ........ | ........ | ..S............. | ..SKQ........... | ...... |
| | | | .......... | .....H.. | P...... | ...N... | ................. | ............... | ..F. |
| IPS:43 3937 | 21- 225_44B10 | VK2\|A18/ JK4 | H........... | .......... | ........ | ........ | ................. | ..S.............. | ...... |
| | | | .......... | E..R.... | P...... | ..I.H... | ................. | ...............F. | ...... |
| IPS:43 3979 | 21-225_46B9 | VK2\|A18/ JK4 | ............ | .......... | ........ | ........ | ................. | .HS.Q........... | ...... |
| | | | .......... | E....... | P...... | ...Y... | ......M......... | .............Y... | .... |
| IPS:43 4201 | 21- 225_59A12 | VK2\|A18/ JK4 | ............ | ........Q.G | ..V..... | ........ | ................. | ..STQ........... | ...... |
| | | | .......... | E....... | P...... | ...Y... | ........V........ | ............... | ...Q |
| IPS:43 4205 | 21-225_60G2 | VK2\|A18/ JK4 | ............ | .......... | ........ | ........ | ................. | ..S.Q........... | ...... |
| | | | ....S..... | E....... | P...... | ...N.I. | ................. | ............... | .... |
| IPS:43 4223 | 21- 225_60C12 | VK2\|A18/ JK4 | ............ | .......... | ........ | ........ | ................. | ..S.K........... | ...... |
| | | | .......... | E....... | P..F... | ...N... | ............I.... | ............Y... | .... |
| IPS:43 4233 | 21-225_61B3 | VK2\|A18/ JK4 | ............ | .......... | ........ | ........ | ................. | ..S.Q........... | ...... |
| | | | .......... | E....... | P...... | ...N.I. | ................. | ............... | ...... |
| IPS:43 4303 | 21- 225_58H11 | VK2\|A18/ JK4 | ............ | .......... | ..V..... | ........ | ................. | ..S.Q........... | ...... |
| | | | .......... | E....... | P...... | ...Y... | ................. | ............... | .... |
| IPS:43 5349 | 21- 225_148F5 | VK2\|A18/ JK4 | ............ | .......... | ........ | ........ | ................. | ..S.Q........... | ...... |
| | | | .......... | E....... | P...... | ...Y.V. | ................. | ............... | .... |
| IPS:43 5359 | 21- 225_148H10 | VK2\|A18/ JK4 | ............ | .......... | ........ | ........ | ................F. | ..S.Q........... | ...... |
| | | | .......... | E....... | P...... | ...Y.V. | ................. | ............... | .... |
| IPS:43 5417 | 21- 225_150D11 | VK2\|A18/ JK4 | ............ | .......... | ........ | ........ | ................. | ....Q........... | ...... |
| | | | .......... | E....... | P...... | ...Y... | ................. | ............... | .... |
| IPS:43 5469 | 21- 225_153G9 | VK2\|A18/ JK4 | ........F.. | .......... | ........ | ........ | ................. | ..N.K........... | ...... |
| | | | .......... | ......... | P..F... | ...N... | ..........D...... | ............Y... | .... |
| IPS:43 5733 | 21- 225_173C11 | VK2\|A18/ JK4 | ............ | .......... | ........ | ........ | ................. | ..S.- | ...... |
| | | | .......... | E....... | P...... | ...H... | ................. | ......QL.. | .... |
| IPS:43 5785 | 21- 225_179C2 | VK2\|A18/ JK4 | ............ | .......... | ........ | ........ | ................. | ..S.- | ...... |
| | | | .......... | E....... | P...... | ...H... | ................. | ......QV.. | .... |
| IPS:39 2618 | 21- 225_16F10 | VK2\|A18/ JK4 | ............ | .......... | ........ | ........ | ...............V. | F.S.Q........... | ...... |
| | | | .I......... | ....H.N | P...... | ...Y... | ..E......A....... | ............... | .... |
| IPS:39 2860 | 21-225_22H8 | VK2\|A18/ JK4 | ............ | .......... | ........ | .......H | ................. | ..S.Q........... | ...... |
| | | | .......... | ....F.. | P...... | ...N... | ............I... | .............S | ...N |
| IPS:39 2888 | 21-225_25A2 | VK2\|A18/ JK4 | ...N........ | .......... | ........ | ........ | ...A.L........... | ..STQ........... | ...... |
| | | | .......... | E....... | P...... | .I.N... | ................. | ............F... | .... |
| IPS:39 2938 | 21-225_29H4 | VK2\|A18/ JK4 | ............ | .......... | ........ | ........ | .L............... | ..S.Q........... | ...... |
| | | | ........F.. | ......... | P....F | ...H... | ................. | ............H.F. | .....R |
| IPS:39 2994 | 21- 225_26G11 | VK2\|A18/ JK4 | ............ | ......T...G | ........ | ........ | ................. | ..S.K........... | ...... |
| | | | .......... | E....... | P.H.... | ...N... | ............I... | ............... | .... |

| | | Germline | K_FR1 | K_CDR1 | K_FR2 | K_CDR2 | K_FR3 | K_CDR3 | K_FR4 |
|---|---|---|---|---|---|---|---|---|---|
| iPS:39 3012 | 21-225_26G7 | VK2|A18/JK4 | ..L......... ............ | ............ E....... | ......... P..F... | ....... ...H.L. | .................... .................... | ..S.Q............. ............. | ...... .... |
| iPS:39 3144 | 21-225_34D2 | VK2|A18/JK4 | ............ ............ | ............ ........ | ......... P...... | ....... ...N... | .................... .................... | ..SK- .....QLPP | ...... ...R |
| | **VK1|L1/JK3** | | DIQMTQSPSSLS ASVGDRVTITC | RAS—QCIS ----NYLA | WFQQKPGK APKSLIY | A------ -ASSLQS | GVPSRFSGSGSG-- TDFTLTISSLQPEDFAT YYC | QQYNS----------- ----------YPFT | FGPGTK VDIK |
| iPS:45 1143 | 21-225_66H11 | VK1|L1/JK3 | ......F....F .F.......... | P........... ........ | ........ ...... | G....... ..FN.H. | ....K.....F..... ....N........N... | ...SC............. ............. | ..H... .... |
| iPS:46 8814 | 21-225_223D11 | VK1|L1/JK3 | ............ ............ | ............ ........ | ........ ...... | ........ ...T... | ....K....R....... N....N........... | ...SG............. ............. | ...... ..T. |
| iPS:43 3901 | 21-225_43A4 | VK1|L1/JK3 | ............ ............ | .........N.. ........ | ........ ......N | ....... ....... | ................. ..A.............. | ...Y............. ............. | ...... .... |
| iPS:43 3961 | 21-225_45D9 | VK1|L1/JK3 | ............ ............ | .........N.. ........ | ........ ......N | ........ ....... | ................. ..A.............. | .H.Y............. ............. | ..R... .... |
| iPS:43 4059 | 21-225_51C5 | VK1|L1/JK3 | ............ ............ | ............ ........ | ........E ...... | ........ ....R. | ....Q............ I............... | ...Y............. ............. | ...... .... |
| iPS:43 4085 | 21-225_52E3 | VK1|L1/JK3 | ............ ............ | ............ ........ | ........ ......N | ........ ....... | ....K............ ................ | .............F... ............. | ...... .... |
| iPS:43 4115 | 21-225_53E4 | VK1|L1/JK3 | ............ ............ | ......V.... ........ | ........ ......S | ........ ....... | ....K............ ................ | ...H............. ............L. | ..R... .... |
| iPS:43 4213 | 21-225_60A4 | VK1|L1/JK3 | ............ ............ | ............ ........ | ........ ...... | ........ ....... | ....K............ .........S...... | ...K............. .........H... | ...... .... |
| iPS:43 4215 | 21-225_60F7 | VK1|L1/JK3 | ............ ..........S. | ......V.K.. .......V | .V...... ...... | ........ ....... | ....T............ ................ | L.FH............. ............. | ...... M... |
| iPS:43 4261 | 21-225_56F7 | VK1|L1/JK3 | ..L......... ............ | ............ ....T... | ....T..T ...... | ........ ......G | ....K............ ................ | H................. .........F..K | ..R... ...T |
| iPS:43 4331 | 21-225_63H8 | VK1|L1/JK3 | ............ ............ | ............ ........ | ........ .H..... | ........ ....... | ....K............ ................ | ...H............. ............. | ...... ...R |
| iPS:43 4361 | 21-225_65D5 | VK1|L1/JK3 | ............ ............ | ......D.N.. ........ | ........ ..R.... | ........ ....H. | ....Q..A......S.. ................ | PL.K............. ............L. | ...... .... |
| iPS:43 4405 | 21-225_68E6 | VK1|L1/JK3 | ............ ............ | ............ ....Y... | ......R ...... | V....... ....... | ................. ..........I... | ...D............. ............. | ...... ...R |
| iPS:43 5259 | 21-225_96C6 | VK1|L1/JK3 | ............ ............ | ............ ........ | ........ ...... | ........ ....... | ....K............ ....N........... | H...D............ ............. | ...... .... |
| iPS:43 5351 | 21-225_148B6 | VK1|L1/JK3 | ............ ........S... | ....K... ........ | ......F ...... | ........ ....... | ....K............ ................ | .............F... ............. | ...... .... |
| iPS:43 5461 | 21-225_153A1 | VK1|L1/JK3 | ............ ............ | ......V.... ........ | ........ ......S | ........ ....R. | ....N............ ................ | ...H............. ............. | ...... ..F. |
| iPS:43 5509 | 21-225_157H1 | VK1|L1/JK3 | ............ ............ | ......D.... ......V | ....R... ..R.... | ........ ....... | ....K............ ................ | ...H............. ............. | ...... .... |

| | | | | | | | | |
|---|---|---|---|---|---|---|---|---|
| iPS:43 5515 | 21-225_157E4 | VK1|L1/J K3 | . . . . . . . . . . . .<br>. . . . . . . . F . . | . . . . . . . . .<br>. . . . . . . . | . . . . . . R<br>. . . . . . . | . . . . . . .<br>. . . . . R . | . . . . Q . . . . . . . . . . . .<br>. . . . N . . . . . . . . . . . | . . . H . . . . . . . . . . . . . .<br>. . . . . . . . . . . . . . . | . . . .<br>. . . . |
| iPS:43 5523 | 21-225_157G5 | VK1|L1/J K3 | . . . . . . . . . . . .<br>. . . . . . . . . . | . . . . . . . . N . .<br>. . . . . . . | . . . . . . .<br>. . E . . . . | . . . . . . .<br>. . . . . . . | . . . . Q . . . . . . . . . . . .<br>. . . . . . . . . . . . . . . . | . . . . . . . . . . . . . . . . . .<br>. . . . . . . . . . . . . . . | . . . .<br>. . . . |
| iPS:43 5535 | 21-225_157H10 | VK1|L1/J K3 | . . . . . . . . . . . .<br>. . . . . . . . . . | . . . . . . . . T . .<br>. . . . . . . | . . . . . . .<br>. . . . . . . | T . . . . . .<br>. . . N . . . | . . . . K . . . . . . . . . . . .<br>. . . . . . . . . . . . . . . . | . . . H . . . . . . . . . . . . . .<br>. . . . . . . . . . . . . . . | . . . .<br>. . . . |
| iPS:43 5559 | 21-225_158H12 | VK1|L1/J K3 | . . . . . . . . . . . .<br>. . . . . . . . . . | . . . . . . . . N . .<br>. . . . . . . | . . . . . . .<br>. . . . . . S | . . . . . . .<br>. . . . . . . | . . . . K . . . . . . . . . . . .<br>. . S . . . . . . . . . . . . . | . . . H . . . . . . . . . . . . . .<br>. . . . . . . . . . . . . . . | . . . .<br>. . . . |
| iPS:43 5575 | 21-225_159H11 | VK1|L1/J K3 | . . . . . . . . . . . .<br>. . . . . . . . . . | . . . . . K . . V<br>. . . . . . . | . . . . . . .<br>. . . . . . . | . . . . . . .<br>. . . . . . . | . . . . K . . . . . . . . . . . .<br>. . . . N . . . . . . . . . . . | . . . . . . . . . . . . . . . . . .<br>. . . . . . . . . . . . . . . | . . . .<br>. . . . |
| iPS:43 5579 | 21-225_160G1 | VK1|L1/J K3 | . . . . . . . . . . . .<br>. . . . . . . . . . | . . . . . . D . N . .<br>. . . . . . . | . . . . . . .<br>. . T . . . . | . . . . . . .<br>. S . . . . . | . . . . K . . . . . . . . . . . .<br>. . . . . . . . . . . . . . . . | . . . H . . . . . . . . . . . . . .<br>. . . . . . . . . . . . . . . | . . . .<br>. . . R |
| iPS:43 5585 | 21-225_160G3 | VK1|L1/J K3 | . . . . . . . . . . . .<br>. . . . . . . . . . | . . . . . . D . N . .<br>. . . . . . . | . . . . R . . .<br>. . T . . . . | . . . . . . .<br>. S . . . . . | . . . . K . . . . . . . . . . . .<br>. . . . . . . . . . . . . . . . | . . . H . . . . . . . . . . . . . .<br>. . . . . . . . . . . . . . . | . . . .<br>. . . . |
| iPS:43 5635 | 21-225_163F1 | VK1|L1/J K3 | . . . . . . . . . . . .<br>. . . . . . . . . . | . . . . . . D . . . .<br>. . . . . . . | . . . . . . .<br>. . . . . . . | . . . . . . .<br>. . . . . . . | . . . . K . . . . . . . . . . . .<br>. . . . . . . . . . A . . . | . . . . . . . . . . . . . . . . . .<br>. . . . . . . . . . . . . . . | . . . . . Q<br>. . A Q |
| iPS:43 5659 | 21-225_167D12 | VK1|L1/J K3 | . . . . . . . . . . . .<br>. . . . . . . . . . | . . . . . . . . N . .<br>. . . . . . . | . . . . . . .<br>. . E . . . . | . . . . . . .<br>. . . . . . . | . . . . K . . . . . . . . . . . .<br>. . . . . . . . . . . . . F . | . . . . . . . . . . . . . . . . . .<br>. . . . . . . . . . . . . . . | . . . .<br>. . . . |
| iPS:43 5679 | 21-225_169D10 | VK1|L1/J K3 | . . . . . . . . . . . .<br>. . . . . . . . . . | . . . . . . D . . . .<br>. . . . . . . | . . . . . . .<br>. . . . . . . | . . . . . . .<br>. . . . . . . | . . . . K . . . . . . . . . . . .<br>. . . . . . . . . . . . . . . . | . . . H . . . . . . . . . . . . . .<br>. . . . . . . . . . . . . . . | . . . .<br>. . . . |
| iPS:43 5685 | 21-225_170E1 | VK1|L1/J K3 | . . . . . . . . . . . .<br>. . E . . . . . . . | . . . . . . . . . . .<br>. . . . . . . | . . . . . . .<br>. . . . . . . | . . . . . . .<br>. . . . . . . | . . . . K . . . . . . . . . . . .<br>. . . . . . . . . . . . . . . . | . . . H . . . . . . . . . . . . . .<br>. . . . . . . . . . . . . . . | . . . .<br>. . . . |
| iPS:43 5747 | 21-225_175C4 | VK1|L1/J K3 | . . . . . . . . . . . .<br>. . . . . . . . . . | . . . . . . . G . .<br>. . . . . . . | . . . . . . .<br>. . . . . . . | . . . . . . .<br>. . . G . . . | . F . . K . . . . . . . . . . . .<br>. . . . . . . . . . . . . . . . | . . . Y . . . . . . . . . . . . . .<br>. . . . . . . . . . . . . . . | . . . .<br>. . . . |
| iPS:43 5765 | 21-225_177D3 | VK1|L1/J K3 | . . . . S . . . . . . .<br>. . . . . . . . . . | . . . . . . . . . T . .<br>. . . . . . . | . . . . . . .<br>. . . . . . . | . . . . . . .<br>. . . . . . . | . . . . K . . . . . . . . . . . .<br>. . . . . . . . . . . . . . . . | . . . . . . . . . . . . . . . . . .<br>. . . . . . . . . . . . . . . | . . . .<br>. . . . |
| iPS:43 5797 | 21-225_181G2 | VK1|L1/J K3 | . . . . . . . . . . . .<br>. . I . E . . . . . | . . . . . . . . . . .<br>. . . . . . . | . I . . . . . T<br>. . . . . . . | . . . . . . .<br>. . . . . . . | . . S . . . . . . . F . . . . . .<br>. . . . . . . . . . . . . . . . | . . . . G . . . . . . . . . . . . .<br>. . . . . . . . . . . . . . . | . . . .<br>. . . . |
| iPS:43 5835 | 21-225_190F12 | VK1|L1/J K3 | . . . . . . . . . . . .<br>. . . . . . . . . . | . . . . . . . G . .<br>. . . . K . . . | . . . . . . .<br>. . . . . . . | . . . . . . .<br>. . . . . . . | . . . . K . . . . . . . . . . . .<br>. . . . . . . . . D . . . . . | . R . D T . . . . . . . . . . . . .<br>. . . . . . . . . . . . . . . | . . . .<br>. . . . |
| iPS:43 5861 | 21-225_190A5 | VK1|L1/J K3 | . . . . . . . . . . . .<br>. . . . . . . . . . | . . . . . . . G . .<br>. . . . . H . . | . . . . . . .<br>. . . . . . H | . . . . . . .<br>. . . . . . . | . . . . K . . . . . . . . . . . .<br>. . . . . . . . . . . . . . . . | . . . S N . . . . . . . . . . . . .<br>. . . . . . . . . . . . V . | . . . .<br>. . . . |
| iPS:43 5869 | 21-225_190B1 | VK1|L1/J K3 | . . . . . . . . . . . .<br>. . . . . . . . . . | . . . . . . . R . .<br>. . . . . . . | . . . . . . .<br>. . . . . . . | V . . . . . .<br>. . . E . . . | . . . . K . . . . . . . . . E . .<br>. . . . . . . . G . . . . | . . L N . . . . . . . . . . . . . .<br>. . . . . . . . . . . V . | . . . R |
| iPS:43 5877 | 21-225_184E7 | VK1|L1/J K3 | . . . . . . . . . R .<br>. . I . E . . . . . | . . . . . . . . . . .<br>. . . . . . . | . I . . . . . T<br>. . . . . . . | . . . . . . .<br>. . . . . . . | . . S . . . . . . . F . . . . . .<br>. I . . . V . R . . . . . . | . . . . G . . . . . . . . . . . . .<br>. . . . . . . . . . . . . . . | . . H . . .<br>|
| iPS:43 5883 | 21-225_185A1 | VK1|L1/J K3 | . . . . . . . . . . . .<br>. . . . . . . . . . | . . . . . . . . . . .<br>. . . . . . . | . . . . T . . .<br>. . . . . . S | V . . . . . .<br>. . . . . . . | . . . . . . . A . . . . . . . .<br>. . . . . . . . . . . . . . . . | R . . H . . . . . . . . . . . . . .<br>. . . . . . . . . . . . . . . | . . . .<br>. . . . |
| iPS:43 5885 | 21-225_185E10 | VK1|L1/J K3 | . . . . . . . . . R .<br>. . I . E . . . . . | . . . . . . . . . . .<br>. . . . . . . | . I . . . . . T<br>. . . . . . . | . . . . . . .<br>. . . . . . . | . . S . . . . . . . F . . . . . .<br>. . . . . . . . . . . . . . . . | . . . . G . . . . . . . . . . . . .<br>. . . . . . . . . . . . . . . | . . . .<br>. . . . |
| iPS:43 5891 | 21-225_188H5 | VK1|L1/J K3 | . . . . . . . . . . . .<br>. . I . E . . . . . | . . . . . . . . . . .<br>. . . . . . . | . L . . . . . T<br>. . . . . . . | . . . . . . .<br>. . . . . . . | . . S . . . . . . . F . . . . . .<br>. . . . . . . . . . . . . . . . | . . . . . . . . . . . . . . . . . .<br>. . . . . . . . . . . . . . . | . . . .<br>. . . . |

| | | | | | | | | |
|---|---|---|---|---|---|---|---|---|
| iPS:43 5897 | 21-225_188B9 | VK1\|L1/J K3 | ............ <br> ..I,E...... | ............ <br> ........ | ,L.....T <br> ....... | ........ <br> ....... | ..S.......F. <br> ................ | ................. <br> ............. | ...... <br> .... |
| iPS:43 5937 | 21-225_190H9 | VK1\|L1/J K3 | ............ <br> ............ | ........G.. <br> ....K... | ........ <br> .L..... | ........ <br> ....... | ....K....... <br> ........D.... | .R.DT............ <br> ............. | .... <br> .... |
| iPS:43 5961 | 21-225_192A2 | VK1\|L1/J K3 | ............ <br> ............ | ..N.....N.. <br> ........ | ........ <br> ....... | ........ <br> ....... | ....K....... <br> ............ | ................. <br> ............. | .... <br> .... |
| iPS:43 5977 | 21-225_192E4 | VK1\|L1/J K3 | ............ <br> ............ | ........G.. <br> ........ | ........ <br> ....... | V....... <br> .V..... | ............ <br> ............ | .R.DT............ <br> ............. | .... <br> .... |
| iPS:43 6001 | 21-225_192C10 | VK1\|L1/J K3 | ............ <br> ............ | ........G.. <br> ....K... | ........ <br> ....... | ........ <br> ....... | ....K....... <br> ........D.... | .R.DT............ <br> ............. | ...... <br> ..F. |
| iPS:43 6039 | 21-225_193F8 | VK1\|L1/J K3 | ....I...... <br> ............ | ......V... <br> ....H.. | ......R <br> ....... | ........ <br> ....... | ....K........A.. <br> ............ | ................. <br> ............. | ...... <br> .... |
| iPS:43 6078 | 21-225_194H12 | VK1\|L1/J K3 | ............ <br> .........R | ........G.. <br> ....K... | ........ <br> .L..... | ........ <br> ....... | ....K....... <br> ........D.... | .R.DT............ <br> ............. | .... <br> .... |
| iPS:43 6140 | 21-225_197G3 | VK1\|L1/J K3 | ............ <br> ............ | ........G.. <br> ....H.. | ........ <br> ....... | ........ <br> ....... | ....K....R...... <br> S........... | ....SN........... <br> ..........V. | .... <br> .... |
| iPS:43 6167 | 21-225_197E11 | VK1\|L1/J K3 | ............ <br> ............ | ........N.. <br> ....K..S | ........ <br> ....... | ........ <br> ....V.. | ....K....... <br> ....R...D......G | .R.DT............ <br> ............. | .... <br> .... |
| iPS:43 6370 | 21-225_211A6 | VK1\|L1/J K3 | ............ <br> ....S..... | ........G.. <br> ....K... | ........ <br> ....... | ........ <br> ....L. | ....K....... <br> ............ | .K.DT............ <br> ............. | .... <br> .... |
| iPS:43 6392 | 21-225_213B3 | VK1\|L1/J K3 | ............ <br> ............ | ........G.. <br> ....K... | ........ <br> .....S | ........ <br> ....VL. | ....K....... <br> ............ | .K.DT............ <br> ............. | .... <br> .... |
| iPS:43 6404 | 21-225_214C3 | VK1\|L1/J K3 | ............ <br> ............ | ........G.. <br> ........ | ........ <br> V...... | ........ <br> ....... | ....K....... <br> ..........T.... | ....MT........... <br> ............I. | .... <br> .... |
| iPS:43 6406 | 21-225_214E4 | VK1\|L1/J K3 | ............ <br> ............ | ............ <br> ............ | ........ <br> ....... | ........ <br> ....... | ....K....... <br> ............ | ....LT........... <br> ............. | ...... <br> .... |
| iPS:43 7216 | 21-225_51D5 | VK1\|L1/J K3 | ............ <br> ............ | ........N.. <br> ........ | ......E <br> ....... | ........ <br> .....R. | ....Q....... <br> I........... | ....Y........... <br> ............. | .... <br> .... |
| iPS:43 7224 | 21-225_56H1 | VK1\|L1/J K3 | ............ <br> ............ | ............ <br> ....H... | ........ <br> ..Q..MS | ........ <br> ...G... | ....K....... <br> ............ | ....QN........... <br> ............. | ...... <br> .... |
| iPS:39 2620 | 21-225_17H5 | VK1\|L1/J K3 | ............ <br> ............ | ............ <br> ........ | ........ <br> ......N | ........ <br> ....... | ....K....... <br> ............ | ....H........... <br> ............. | ...... <br> .... |
| iPS:39 2692 | 21-225_18G10 | VK1\|L1/J K3 | ............ <br> ............ | ......D.... <br> ....Y... | ........ <br> ....... | V....... <br> ....... | ...K.G...F..... <br> ............ | L................ <br> ............. | .... <br> .... |
| iPS:39 2708 | 21-225_18D11 | VK1\|L1/J K3 | ..........F <br> .F........ | ..........D <br> ....Y... | ........ <br> ....... | V....... <br> ....... | ....K....F...... <br> ............ | ....T............ <br> ............. | .....T <br> .... |
| iPS:39 2714 | 21-225_16G12 | VK1\|L1/J K3 | ............ <br> ............ | ......D.... <br> ........ | ........ <br> ....... | ........ <br> ....... | ....K.R......... <br> .....N.......S... | ....H........... <br> ...........F... | .... <br> .... |
| iPS:39 2748 | 21-225_20H7 | VK1\|L1/J K3 | ............ <br> ............ | .T......N.. <br> ......V | ........ <br> ...R... | ........ <br> ....... | ....K........... <br> ............ | ....Y........... <br> ............. | .... <br> M.F. |
| iPS:39 2782 | 21-225_22B12 | VK1\|L1/J K3 | ............ <br> ........I. | ......D.... <br> ........ | ...E....G <br> .H..... | ........ <br> .....R. | ....K........... <br> N.........L..... | ....H........... <br> ............. | ...... <br> ..F. |

| | | | | | | | | | |
|---|---|---|---|---|---|---|---|---|---|
| iPS:39 2784 | 21-225_23C7 | VK1|L1/J K3 | . . . . . . . . . . .<br>. . . . . . . . . . . | . . . . . . . G . .<br>. . . . I . . . . | . . . . . . . .<br>. . . . . . S | . . . . . . .<br>. . . . . . . | . . . . K . . . . . . . . . . . F .<br>. . . . . . . . . . . . . . . . . . . . | . . . . . . . . . . . . . . . . .<br>. . . . . . . . . . . . . . . . . | . . . . . .<br>. . . . . |
| iPS:39 2802 | 21-225_23E7 | VK1|L1/J K3 | . . . . . . . . . . .<br>. . . . . . . . . | . . . . . . . . . .<br>. . . . . . . . | . . . . I . . . .<br>. . . . . . . | . . . . . . .<br>. . . . . . . | . . . . K . . . . . . . . . . . . .<br>. . . . . . . . . . . . . . . . . . . | . . F Y . . . . . . . . . . . . . .<br>. . . . . . . . . . . . . . . . . | . . . . . .<br>. . . N |
| iPS:39 2826 | 21-225_20B9 | VK1|L1/J K3 | . . . . . . . . . . .<br>. . . . . . . . . | . . . . . . . . . .<br>. . . . . . . . | . . . . . . . .<br>. . . . . . . | V . . . . . .<br>. . . . . . . | . . . . K . . . . . . . . . . . . .<br>. . . . . . . . . . . . . . . . . . . | . . . . T . . . . . . . . . . . . .<br>. . . . . . . . . . . . . . . . . | . . . . . .<br>. . . . |
| iPS:39 2840 | 21-225_23G1 | VK1|L1/J K3 | . . . . . . . . . . .<br>. . . . . . . . . | . . . . . . . . . .<br>. . . . . . . . | . . . . . . . .<br>. . . . . . S | . . . . . . .<br>. . . . . . . | . . . . Q . . . . . F . . . . . . .<br>. . . . . . . . . . . . . . . . . . . | . . . . . . . . . . . . . . . . .<br>. . . . . . . . . . . . . . . . . | . . . . . .<br>. . . . |
| iPS:39 2842 | 21-225_23G8 | VK1|L1/J K3 | . . . . . . . . . . .<br>. . . . . . . . . | . . . . . . . R . .<br>. . . . . . . . | . . . . . . . .<br>. . . . . . . | . . . . . . .<br>. . . . . . . | . . . . N . . . . . . . . . . . . .<br>. . . . . . . . . . . . . . . . . . . | . . . . . . . . . . . . . . . . .<br>. . . . . . . . . . . . . . . . . | I . . . . .<br>. . . . |
| iPS:39 2890 | 21-225_20H9 | VK1|L1/J K3 | . . . . . . . . . . .<br>. . . . . . . . . | . . . . . . . . . .<br>. . . . . . . . | . . . . . . . .<br>. . . . . . S | . . . . . . .<br>. . . . . . . | . . . . K . . . . . . . . . . . . .<br>. . . . . . . . . . . . . . . . . . . | . . . . . . . . . . . . . . . . .<br>. . . . . . . . . . . . . . . . . | . . . . . .<br>. . . . |
| iPS:39 2892 | 21-225_20C11 | VK1|L1/J K3 | . . . . . . . . . . .<br>. . . . . . . . . | . . . . . . D . . . .<br>. . . . . . . . | . . . . . . . .<br>. . . . . . . | . . . . . . .<br>. . . . . . . | . . . . K . R . . . . . . . . . .<br>. . . . . . . . . . . . . . . . . . . | . . . H . . . . . . . . . . . . . .<br>. . . . . . . . . . F . . . | . . . . . .<br>. . V . |
| iPS:39 2950 | 21-225_25C10 | VK1|L1/J K3 | . . . . . . . . . . .<br>. . . . . . . . . | . . . . . . . . . .<br>. . . . . . . . | . . . . . . . .<br>. . . . . . . | . . . . . . .<br>. . . . . . . | . F . K . . . . . . . . . . . . .<br>. . . . . . . . . . . . . . . . . . . | . . . H . . . . . . . . . . . . . .<br>. . . . . . . . . . . . . . . . . | . . . . . .<br>. . . . |
| iPS:39 2952 | 21-225_26G1 | VK1|L1/J K3 | . . . . . . . . . . .<br>. . . . . . . . . | . . . . . . D . . . .<br>. . . . . . . . | . . . . . . . .<br>. . . . . . . | . . . . . . .<br>. . . . . R . | . . . . N . . . . . . . . . . . . .<br>. . . . . . . . . . . N . . . | . . . H . . . . . . . . . . . . . .<br>. . . . . . . . . . . . . . . . . | . . . . . .<br>. . . . |
| iPS:39 2962 | 21-225_30A1 | VK1|L1/J K3 | . . . . . . . . . . .<br>. . . . . . . . . | . . . . . . . . . .<br>. . . . . . . . | . . . . . . . .<br>. . . . . . S | . . . . . . .<br>. . . . . . T | . . . . K . . . . . . . . . . . . .<br>. . . . . . . . . . . . . . . . . . . | . . . . . . . . . . . . . . . . .<br>. . . . . . . . . . . . . . . . . | . . . . . .<br>. . . . |
| iPS:39 2976 | 21-225_27H12 | VK1|L1/J K3 | . . . . . . . . . . .<br>. . . . . . . . . | . . . . . . . . . .<br>. . . . . . . . | . . . . . . . .<br>. . . . . . N | G . . . . . .<br>. . . . . . . | . . . . K . . . . . . . . . . . . .<br>. . . . . . . . . . . . . . . . . . . | . . . Y . . . . . . . . . . . . . .<br>. . . . . . . . . . . . . . . . . | . . . . . .<br>. N . N |
| iPS:39 3090 | 21-225_33A5 | VK1|L1/J K3 | . . . . . . . . . . .<br>. . . . . . . . . | . . . . . . . . . .<br>. . . . . . . . | . . . . . . . .<br>. . . . . . S | . . . . . . .<br>. . . . . . . | . . . . K . . . . . . . . . . . . .<br>. . . . . . . . . . . . . . . . . . . | . . . . . . . . . . . . . . . . .<br>. . . . . . . . . . . . . . . . . | . . . . . N<br>. . . . |
| iPS:39 3120 | 21-225_35H8 | VK1|L1/J K3 | . . . . . . . . . . .<br>. . . . . . . . . | . . . . . . A . . . .<br>. . . . . . . . | . . . . . . . .<br>. . . . . . . | G . . . . . .<br>. . . G . . . | . . . . K . . . . . . . . . . . . .<br>. . . . . . . . . . . . . . . . . . . | . . . . . . . . . . . . . . . . .<br>. . . . . . . . . . . . . . . . . | . . . . . .<br>. . F . |
| iPS:39 3836 | 21-225_15A2 | VK1|L1/J K3 | . . . . . . . . . . F<br>. F I . . . . . . . | . . . . . . . . . .<br>. . . . . . . . | . . . . . . . .<br>. . . . . . F | . . . . . . .<br>. . . . . . . | . . . . K . . . . . F . . . . . . .<br>. F P . . . . . . . . . . N . . . | . . . Y . . . . . . . . . . . . . .<br>. . . . . . . . . . . . . . . . . | . . . . . Q<br>. . V . |
| iPS:39 3870 | 21-225_7B1 | VK1|L1/J K3 | . . . . . . A . . . . .<br>. . . . . . . . . | . . . . . . D . . . .<br>. . . . . H . V | . . . . . . . .<br>. . . . . . F | . . . . . . .<br>. . . . . . . | . . . . Q . . . . . . . . . . . . .<br>. . . . . . . I . . . | H . . . . . . . . . . . . . . . .<br>. . . . . . . . . . . . . . . . . | . . . . . .<br>. . F . |
| iPS:39 3894 | 21-225_5E11 | VK1|L1/J K3 | V . . . . . . . . . .<br>. . . . . . . . . | . . . . . . . . . .<br>. . . . . . . . | . . . . . . . .<br>. . . . . . N | . . . . . . .<br>. . . . V . . | . . . . K . . . N . . . . . . . .<br>. . . . . . . . . . . . . . . . . . . | H . . H . . . . . . . . . . . . .<br>. . . . . . . . . . . . . . . . . | . . . . . .<br>. . . . |
| iPS:39 3896 | 21-225_2A4 | VK1|L1/J K3 | . . . . . . . . . . F<br>. . . . . . . . . | . . . . . . . . . . .<br>. . . . . . . . | . . . . . . . .<br>. . . R . . . | T . . . . . .<br>. . . . . . . | . . . K . . . . . F . . . . . . .<br>. . . . . . . . . . . . . . . . . . . | . . . . . . . . . . . . . . . . .<br>. . . . . . . . . . . . . . . . . | . . . . . .<br>. . . . |
| iPS:39 3914 | 21-225_16B8 | VK1|L1/J K3 | . . . . . . . . . . .<br>. . . . . . . . . | . . . . . . . N . .<br>. . . . . . . . | . . . . . . . .<br>. L . . . . N | . . . . . . .<br>. . . . V . . | . . . . K . . . . . . . . . . . . .<br>. . . . . . . . . . . . . . . . . . . | H . . H . . . . . . . . . . . . . .<br>. . . . . . . . . . . . . . . . . | . . . . . .<br>. . . V |
| iPS:39 3968 | 21-225_5A5 | VK1|L1/J K3 | . . . . . . . . . . .<br>. . . . . . . . . | . . . . . . . . . .<br>. . . . . . . . | . . . . . . . .<br>. . . . . . S | . . . . . . .<br>. . . . . . . | . . . K . . . . . . . . . . . . .<br>. . . . . . . . . . . . . . . . . . . | . . . . . . . . . . . . . . . . .<br>. . . . . . . . . . . . . . . . . | . . . . . .<br>. . . . |
| iPS:39 3992 | 21-225_14H8 | VK1|L1/J K3 | . . . . . . . . . . .<br>. . . . . . . . . | . . . . . . . . . .<br>. . . . Y . . . | . . . . . . . .<br>. . . . . . . | V . . . . . .<br>. . . . . . . | . . . K . . . . . . . . . . . . .<br>. . . . . . . . . . . . . . . . . . . | . . . . . . . . . . . . . . . . .<br>. . . . . . . . . . . . . . . . . | . . . . . .<br>. . . N |
| iPS:39 4018 | 21-225_15B1 | VK1|L1/J K3 | . . . . . . . . . . .<br>. . . . . . . . . | . . . . . . . . . .<br>. . . . . . . . | . . . . . . . .<br>. . . . . . S | . . . . . . .<br>. . . . . . . | . . . . N . . . . . . . . . . . . .<br>. . . . . . . . . . . . . . . . . . . | . . . H . . . . . . . . . . . . . .<br>. . . . . . . . . . . . . . . . . | . . . . . .<br>. . . . |

| iPS | Clone | Germline | K_FR1 | K_CDR1 | K_FR2 | K_CDR2 | K_FR3 | K_CDR3 | K_FR4 |
|---|---|---|---|---|---|---|---|---|---|
| iPS:39 4026 | 21-225_16C7 | VK1\|L1/JK3 | .............<br>........... | ...........<br>........ | ........<br>......S | ........<br>....... | ....K..........<br>................ | ..................<br>................ | ......<br>.... |
| iPS:39 4055 | 21-225_9C8 | VK1\|L1/JK3 | .............<br>........... | ...........<br>....Y... | ........<br>....... | V.......<br>....... | ....K..........<br>................ | ...D.............<br>................ | ......<br>.... |
| iPS:39 8482 | 21-225_17H6 | VK1\|L1/JK3 | .............<br>........... | ......RD....<br>........ | ........<br>......S | T.......<br>....... | ....K..........<br>............I... | ..H.............<br>................ | ......<br>...Q |
| iPS:39 8492 | 21-225_21F12 | VK1\|L1/JK3 | .............<br>........... | ........R..<br>.....F.. | ........<br>......S | ........<br>......T | ....K..........<br>................ | ..................<br>..........F... | ......<br>.... |
| iPS:39 8500 | 21-225_23A11 | VK1\|L1/JK3 | .............<br>T.......... | ......D....<br>........ | ........<br>...R... | ........<br>...T... | ....K..........<br>................ | ..................<br>................ | ......<br>..L. |
| iPS:39 8526 | 21-225_32B3 | VK1\|L1/JK3 | .............<br>..I........ | ........<br>........ | ........<br>..R.... | ........<br>....... | ....T..........<br>................ | ..................<br>................ | ......<br>.... |
| iPS:40 2235 | 21-225_20F10 | VK1\|L1/JK3 | ...L........<br>........... | ........N..<br>........ | ........<br>....... | ........<br>....... | ..........F.....<br>...............F. | ..................<br>................ | ......<br>..N. |
| | | Germline | K_FR1 | K_CDR1 | K_FR2 | K_CDR2 | K_FR3 | K_CDR3 | K_FR4 |
| | **VK3\|A27/JK 1** | | EIVLTQSPGTLS LSPGERATLSC | RAS--QSVSS- ----SYLA | WYQQKPGQ APRLLIY | G------- -ASSRAT | GIPDRFSGSGSG-- TDFTLTISRLEPEDFAV YYC | QQYGS-------------- -----------SPWT | FGQGTK VEIK |
| iPS:46 8810 | 21-225_74D5 | VK3\|A27/ JK1 | ............<br>........... | ........N..<br>....N... | ..R.....<br>....... | ........<br>....... | <br>I.I............. | ...E............<br>................ | ......<br>.... |
| iPS:46 8834 | 21-225_94G10 | VK3\|A27/ JK1 | ............<br>........... | ........N..<br>....N... | ..R.....<br>....... | ........<br>....... | <br>I.I............. | ...E............<br>................ | ......<br>.... |
| iPS:46 8838 | 21-225_80E12 | VK3\|A27/ JK1 | ......C.....<br>........V.. | ........N..<br>....N... | ..R...D.<br>....... | ........<br>....... | <br>I.I............. | ...E............<br>................ | ......<br>.... |
| iPS:46 8820 | 21-225_76E10 | VK3\|A27/ JK1 | ............<br>........... | ........N..<br>....N... | ..R.....<br>....... | ........<br>....... | <br>I.I............. | ...E............<br>................ | ......<br>.... |
| iPS:43 3931 | 21-225_44F6 | VK3\|A27/ JK1 | ............<br>........... | .........G.<br>........ | ........<br>....... | ........<br>....... | <br> | ................<br>................ | ......<br>.... |
| iPS:43 4307 | 21-225_59B2 | VK3\|A27/ JK1 | ............<br>........... | W.......Y..<br>....F... | .F...S..<br>....... | ........<br>....... | <br>..............F.. | ....T...........<br>................ | ......<br>.... |
| iPS:43 4471 | 21-225_75G3 | VK3\|A27/ JK1 | ............<br>........... | ..R...N.D..<br>........ | ........<br>....... | ........<br>....... | <br>..............F.. | ...ER...........<br>................ | ......<br>.... |
| iPS:43 4473 | 21-225_76D1 | VK3\|A27/ JK1 | ............<br>........... | ......NIY..<br>....N... | ...E....<br>....... | ........<br>....... | <br>..............V.. | ...E............<br>................ | ......<br>.... |
| iPS:43 4495 | 21-225_74B2 | VK3\|A27/ JK1 | .L..........<br>........... | ......NIY..<br>........ | ........<br>....... | ........<br>....... | <br>..............C. | ...E............<br>................ | ......<br>.... |
| iPS:43 4497 | 21-225_76A4 | VK3\|A27/ JK1 | ..........Y<br>........... | ........Y..<br>........ | ........<br>....... | ........<br>....... | .<br>A............... | .HSDN...........<br>................ | ......<br>.... |
| iPS:43 4501 | 21-225_76G4 | VK3\|A27/ JK1 | ............<br>........... | ........Y..<br>........ | ........<br>....... | ........<br>....... | .<br>A.........C..... | .HSDN...........<br>................ | ......<br>.... |
| iPS:43 4507 | 21-225_74C5 | VK3\|A27/ JK1 | ............<br>........... | ........N..<br>....N... | ........<br>....... | ........<br>..F.... | .....V.........<br>N.I............. | ...E............<br>................ | ......<br>.... |

| | | | | | | | | |
|---|---|---|---|---|---|---|---|---|
| iPS:43 4517 | 21-225_76A7 | VK3\|A27/ JK1 | ..A.........<br>.......... | .....P..D..<br>........ | ....R...<br>....... | .......<br>......P | ...............<br>............... | ..E.............<br>.............. | ......<br>.... |
| iPS:43 4519 | 21-225_74C7 | VK3\|A27/ JK1 | ..........<br>.......... | .T...PN.D..<br>........ | .......<br>....... | .......<br>....... | ...............<br>.............F.. | ...ER...........<br>.............. | ......<br>.... |
| iPS:43 4523 | 21-225_75C3 | VK3\|A27/ JK1 | ..........<br>..Q....... | ..........<br>....R... | .......<br>....... | .......<br>....... | ...............<br>............... | .H.D...........<br>.............. | ......<br>.... |
| iPS:43 4531 | 21-225_76C9 | VK3\|A27/ JK1 | ..........<br>.......... | ..........<br>.......S | .......<br>....... | .......<br>....... | ...............<br>............... | ....‒<br>.....RSR. | ......<br>...R |
| iPS:43 4533 | 21-225_85F7 | VK3\|A27/ JK1 | .P.........<br>.......... | ......NIY..<br>....N... | .......<br>....... | .......<br>....... | ...............<br>.............C. | ...E...........<br>.............. | ......<br>.... |
| iPS:43 4547 | 21-225_74H5 | VK3\|A27/ JK1 | ..........<br>.......... | .......N..<br>....N... | ..R.....<br>....... | .......<br>....... | ...............<br>I.I............ | ...E...........<br>.............. | ......<br>.... |
| iPS:43 4559 | 21-225_74D11 | VK3\|A27/ JK1 | ..........<br>.......... | .......Y..<br>........ | .......<br>....... | .......<br>....... | ...............<br>A.............. | .H.DN..........<br>.............. | ......<br>.... |
| iPS:43 4561 | 21-225_77G1 | VK3\|A27/ JK1 | .........Y<br>.......... | .......Y..<br>........ | .......<br>....... | .......<br>....... | ...............<br>A........C.... | .H.DN..........<br>.............. | ......<br>.... |
| iPS:43 4565 | 21-225_75B10 | VK3\|A27/ JK1 | ..........<br>.......... | ....P..N..<br>....Y... | .......<br>....... | .......<br>..T.... | ...............<br>............F. | ...ED..........<br>.............. | ......<br>.... |
| iPS:43 4571 | 21-225_74D2 | VK3\|A27/ JK1 | ..........<br>.......... | .......D..<br>....N... | .......<br>....... | .......<br>......P | ...............<br>....N........F. | ...E...........<br>.............. | ......<br>.... |
| iPS:43 4579 | 21-225_77F7 | VK3\|A27/ JK1 | .........Y<br>.......... | .......Y..<br>........ | .......<br>....I.. | .......<br>....... | ...........C.....<br>A.....V.H..C.... | .H.DN..........<br>.............. | ......<br>.... |
| iPS:43 4581 | 21-225_74B12 | VK3\|A27/ JK1 | .........Y<br>.......... | .......Y..<br>........ | .......<br>....... | .......<br>.....S. | ...............<br>A.............. | .H.DN..........<br>.............. | ......<br>.... |
| iPS:43 4585 | 21-225_75A12 | VK3\|A27/ JK1 | ..........<br>.......... | ..........<br>....R... | .......<br>....... | .......<br>....... | ...............<br>.........A...... | .H.D...........<br>.............. | ......<br>.... |
| iPS:43 4595 | 21-225_77A10 | VK3\|A27/ JK1 | ..........<br>.......... | .......H..<br>....R... | .......<br>..K...F | .......<br>....... | ...............<br>............... | .H.D...........<br>.............. | ......<br>.... |
| iPS:43 4611 | 21-225_77C12 | VK3\|A27/ JK1 | ..........<br>.......... | ..R.....D..<br>........ | .....R..<br>....... | .......<br>....... | ...............<br>............... | ...E...........<br>.............. | ......<br>.... |
| iPS:43 4633 | 21-225_74G8 | VK3\|A27/ JK1 | ..........<br>.......... | .......F...<br>....A... | .......<br>....... | .......<br>.T..... | ...............<br>....G.......... | ....‒<br>.....NSR. | ......<br>.... |
| iPS:43 4637 | 21-225_78E7 | VK3\|A27/ JK1 | ..........<br>.......... | ......N.D..<br>....N... | .......<br>....... | .......<br>....... | ...............<br>.............F.. | ...ER...........<br>.............. | ......<br>.... |
| iPS:43 4657 | 21-225_79G1 | VK3\|A27/ JK1 | .........Y<br>..S....... | .......Y..<br>........ | ......A.<br>....... | .......<br>.....S. | ...............<br>A.............. | .H.DN..........<br>.............. | ......<br>.... |
| iPS:43 4663 | 21-225_79F3 | VK3\|A27/ JK1 | .........Y<br>.......... | .......Y..<br>........ | ......A.<br>....... | .......<br>.....S. | ...............<br>A.............. | .H.DN..........<br>.............. | ......<br>.... |
| iPS:43 4671 | 21-225_74F4 | VK3\|A27/ JK1 | ..........<br>.......... | ......IF...<br>........ | .......<br>S...... | .......<br>....... | ...............<br>............... | ....‒<br>......SR. | ......<br>.... |

| | | | | | | | | |
|---|---|---|---|---|---|---|---|---|
| iPS:43 4687 | 21-225_75A5 | VK3\|A27/ JK1 | ..........Y ........... | ........Y.. ........ | ........ ........ | ....... ....... | ................ A............... | .H.DN............ ............ | .... .... |
| iPS:43 4691 | 21-225_75G7 | VK3\|A27/ JK1 | .......... ........... | ..R.....D.. ........ | .....R.. ........ | ....... ....... | ................ ............... | ...E............ ............... | .... .... |
| iPS:43 4693 | 21- 225_79F11 | VK3\|A27/ JK1 | .......... ........... | ........Y.. ........ | ........ ........ | ....... ....... | ................ A............... | .HSDN........... ............... | .... .... |
| iPS:43 4699 | 21- 225_79G12 | VK3\|A27/ JK1 | ..........Y ........... | ........Y.. ........ | ........ ........ | ....... ......S. | ................ A.........C..... | .HSDN........... ............... | .... .... |
| iPS:43 4701 | 21-225_80A1 | VK3\|A27/ JK1 | ........RY ........... | ........Y.. ........ | ........ ........ | ....... ......S. | ..........C..... A.....V....C..... | .HSDN........... ............... | .... .... |
| iPS:43 4703 | 21-225_80C1 | VK3\|A27/ JK1 | .........Y ........... | ........Y.. ........ | ........ ........ | ....... ......S. | ................ A.....V....C..... | .HSDN........... ............... | .... .... |
| iPS:43 4709 | 21-225_80E3 | VK3\|A27/ JK1 | .........Y ..S........ | ........Y.. ........ | ........ ........ | ....... ......S. | ..........C..... A.....V......... | .HSDN........... ............... | .... .... |
| iPS:43 4715 | 21-225_80D5 | VK3\|A27/ JK1 | .L......... ....K.V.... | ......NIY.. ........ | ........ ........ | ....... ....... | ................ ....T.........C. | ...E............ ............... | .... .... |
| iPS:43 4717 | 21-225_80A6 | VK3\|A27/ JK1 | .......... .....IP.... | ........D.. ....G... | ........ ........ | ....... ......P | ................ ................ | ...E............ ............... | .... .... |
| iPS:43 4725 | 21-225_80H7 | VK3\|A27/ JK1 | .......... ........... | .......IN.. ....N... | ........ ........ | ....... ....... | ................ ..............F.. | ...E............ ............... | .... .... |
| iPS:43 4735 | 21- 225_80B10 | VK3\|A27/ JK1 | .......... ........... | ........D.. ........ | ........ ........ | ....... ......P | ................ ................ | ...E............ ............... | .... .... |
| iPS:43 4743 | 21-225_74A4 | VK3\|A27/ JK1 | ..........Y ........... | ........Y.. ........ | ........ ........ | ....... ......S. | ..........C..... A.........C..... | .HSDN........... ............... | .... ... |
| iPS:43 4751 | 21- 225_80H12 | VK3\|A27/ JK1 | .......... ........... | ........Y.. ........ | ........ ........ | ....... ......S. | ................ A............... | .HSDN........... ............... | .... .... |
| iPS:43 4759 | 21-225_81C5 | VK3\|A27/ JK1 | .........Y ..S....... | ........Y.. ........ | ........ ........ | ....... ......S. | ..........C..... A.....V....C.... | .HSDN........... ............... | .... .... |
| iPS:43 4773 | 21-225_75D9 | VK3\|A27/ JK1 | .......... ........... | .......... ........ | .....R.. ........ | ....... ....... | ................ ................ | ...E............ ............... | .... .... |
| iPS:43 4777 | 21- 225_81C11 | VK3\|A27/ JK1 | ........RY ...V....... | ........Y.. ........ | ........ ........ | ....... ......S. | .L.......C..... A.....V....C..... | .HSDN........... ............... | .... .... |
| iPS:43 4809 | 21-225_74F5 | VK3\|A27/ JK1 | .........Y ........... | ........Y.. ........ | ........ ........ | ....... ......S. | ................ A............... | .HSDN........... ............... | .... .... |
| iPS:43 4821 | 21-225_83G1 | VK3\|A27/ JK1 | ........RY ........... | ........Y.. ........ | ........ ........ | ....... ......S. | VL.............. A.....V....C..... | .HSDN........... ............... | .... .... |
| iPS:43 4835 | 21-225_83B6 | VK3\|A27/ JK1 | .......... ........... | ........D.. ....G... | ........ ........ | ....... .....TP | ................ ................ | ...E............ ............... | .... .... |
| iPS:43 4839 | 21-225_83B7 | VK3\|A27/ JK1 | ........RY ..SV....... | ........Y.. ........ | ........ ........ | ....... ......S. | ................ A.........C..... | .HSDN........... ............... | .... .... |
| iPS:43 4849 | 21- 225_83C10 | VK3\|A27/ JK1 | .......... ........... | .....P..H.. ....N... | ........ ........ | ....... ....... | ................ I............... | ...E............ ............... | .... .... |

# EP 4 435 105 A2

| | | | | | | | | | |
|---|---|---|---|---|---|---|---|---|---|
| IPS:43 4869 | 21-225_84E12 | VK3\|A27/ JK1 | . . . . . . . . . . . . / . . . . . . . . . . | . . . . . . . IN . . / . . . . N . . . | . . . . . . . . / . . . . . . . | . . . . . . . . / . . . . . . . | . . . . . . . . . . . . . / . . . . . . . . D . . . . F . . | . . . E . . . . . . . . . . . / . . . . . . . . . . . . . . . | . . . . . . / . . . . |
| IPS:43 4879 | 21-225_85A3 | VK3\|A27/ JK1 | . . . . . . . . . . . F / . . . . . . . . . . | . . . . . . . . Y . . / . . . . . . . | . . . . . . A . / . . . . . . . | . . . . . . . / . . . . . . S | . . . . . . . . . . . . . / A . . . . . . . . . . . . . | . H . DN . . . . . . . . . . . / . . . . . . . . . . . . . . | . . . . / . . . . |
| IPS:43 4881 | 21-225_85B4 | VK3\|A27/ JK1 | . . . . . . . . . . . F / . . . . . . . . . . | . . . . . . . . Y . . / . . . . . . . | . . . . . . . . / . . . . . . . | . . . . . . . / . . . . . . . | . . . . . . . . . . . . . / A . . . . . . . . . . . . . | . H . DN . . . . . . . . . . . / . . . . . . . . . . . . . . | . . . . / . . . . |
| IPS:43 4887 | 21-225_85D6 | VK3\|A27/ JK1 | . . . . . . . . . . . F / . . Q . . . . . . . | . . . . . . . . . . / . . . . R . . . | . . . . . . . . / . . . . . . . | . . . . . . . / . . . . . . . | . . . . . . . . . . . . . / . . . . . . . . . . . . . . | . H . D . . . . . . . . . . . . / . . . . . . . . . . . . . . | . . . . . . / . . . . |
| IPS:43 4891 | 21-225_85G6 | VK3\|A27/ JK1 | . . A . . . . . . . . / . . . . . . . . . . | . . . . . P . . D . . / . . . . . . . | . . . . . . . . / . . . . . . . | . . . . . . . / . . A . . . P | . . . . . . . . . . . V . . . / . . . . . . . . . . . . . | . . . E . . . . . . . . . . . / . . . . . . . . . . . . . . | . . . . / . . . . |
| IPS:43 4895 | 21-225_74H7 | VK3\|A27/ JK1 | . L . . . . . . . . . / . . . . . . . . . . | . . . . . NIY . . / . . . . . . . | . . . . . . . . / . . . . . . . | . . . . . . . / . . . . . . . | . . . . . . . . . . . . . / . . . . . . . . . . . . C . | . . . E . . . . . . . . . . . . / . . . . . . . . . . . . . . | . . . . . . / . . . . |
| IPS:43 4899 | 21-225_85B9 | VK3\|A27/ JK1 | . . . . . . . . . . . F / . . . . . . . . . . | . . . . . . . . N . . / . . . . N . . . | . . R . . . . / . . . . . . . | . . . . . . . / . . . . . . . | . . . . . . . . . . . . . / I . I . . . . . . . . . . . | . . . E . . . . . . . . . . . . / . . . . . . . . . . . . . . | . . . . / . . . . |
| IPS:43 4907 | 21-225_85G10 | VK3\|A27/ JK1 | . . . . . . . . S . . / . . . . . . . . . . | . . . . . . . W . . / . . . . G . . . | . . . . . . . . / . . . . . . . | . . . . . . . / . . . . . . . | . . . . . . . . . . . . . / . . . . . . . . . . . . F . | . . . E . . . . . . . . . . . / . . . . . . . . . . . . . . | . . . . / . . . . |
| IPS:43 4913 | 21-225_86C1 | VK3\|A27/ JK1 | . . . . . . . . . Y / . . . . . . . . . . | . . . . . . . Y . . / . . . . . . . | . . . . . . A . / . . . . . . . | . . . . . . . / . . . . . . . | . . . . . . . . . . . . . / A . . . . . . . . . C . . . . | . H . DN . . . . . . . . . . . / . . . . . . . . . . . . . . | . . . . . / . . . . |
| IPS:43 4921 | 21-225_86E4 | VK3\|A27/ JK1 | . . . . . . . . . RY / . . . . . . . . . . | . . . . . . . Y . . / . . . . . . . | . . . . . . . . / . . . . . . . | . . . . . . . / . . . . . . S . | . . . . . . . . . . . . . / A . . . . . . . . . C . . . . | . HSDN . . . . . . . . . . . . / . . . . . . . . . . . . . . | . . . . / . . . . |
| IPS:43 4939 | 21-225_86C11 | VK3\|A27/ JK1 | . . . . . . . . . Y / . . . . . . . . . . | . . . . . . . Y . . / . . . . . . . | . . . . . . . . / . . . . . . . | . . . . . . . / . . . . . S . | . L . . . . . . . . . . . / A . . . . . . . . . C . . . . | . HSDN . . . . . . . . . . . / . . . . . . . . . . . . . . | . . . . / . . . . |
| IPS:43 4943 | 21-225_87H1 | VK3\|A27/ JK1 | . . . . . . . . . . . / . . . . . . . . . . | . . . . . . . . D . . / . . . . N . . . | . . . . . . . . / . . . . . . . | . . . . . . . / . . . A . T . | . . . . . . . . . . . . . / . . . . . . . . . . . . S . | . . . E . . . . . . . . . . . / . . . . . . . . . . . . . . | . . . . / . . . . |
| IPS:43 4945 | 21-225_87E5 | VK3\|A27/ JK1 | . F . . . . . . . . Y / . . . . . . . . . . | . . . . . . . Y . . / . . . . . . . | . . . . . . . . / . . . . . . . | . . . . . . . / . . . . . S . | . . . . . . . . . . . . . / A . . . . . V . . . C . . . . | . HSDN . . . . . . . . . . . / . . . . . . . . . . . . . . | . . . . / . . . . |
| IPS:43 4955 | 21-225_87C9 | VK3\|A27/ JK1 | . . . . . . . . . . Y / . . . V . . . . . . | . . . . . . . Y . . / . . . . . . . | . . . . . . . . / . . . . . . . | . . . . . . . / . . . . . S . | . . . . . . . . . . . . . / A . . . . . . . . . C . . . . | . HSDN . . . . . . . . . . . / . . . . . . . . . . . . . . | . . . . / . . . . |
| IPS:43 4961 | 21-225_87A12 | VK3\|A27/ JK1 | . . . . . . . . . RY / . . . . . . . . . . | . . . . . . . Y . . / . . . . . . . | . . . . . . . . / . . . . . . . | . . . . . . . / . . . . . S . | V . . . . . . . . C . . . . / A . . . . . V . . . C . . . . | . HSDN . . . . . . . . . . . / . . . . . . . . . . . . . . | . . . . / . . . . |
| IPS:43 4969 | 21-225_88H1 | VK3\|A27/ JK1 | . . . . . . . . . . Y / . . . . . . . . . . | . . . . . . . Y . . / . . . . . . . | . . . . . . . . / . . . . . . . | . . . . . . . / . . . . . S . | V . . . . . . . . C . . . . / A . . . . . V . . . C . . . . | . HSDN . . . . . . . . . . . / . . . . . . . . . . . . . . | . . . . / . . . . |
| IPS:43 4981 | 21-225_88E7 | VK3\|A27/ JK1 | . . . . . . . . . . . / . . . . . . . . . . | . . . . . . . Y . . / . . . . . . . | . . . . . . . . / . . . . . . . | . . . . . . . / . . . . . S . | . . . . . . . . . . . . . / A . . . . . V . . . C . . . . | . HSDN . . . . . . . . . . . / . . . . . . . . . . . . . . | . . . . / . . . . |
| IPS:43 4983 | 21-225_88F7 | VK3\|A27/ JK1 | . . . . . . . . . . . / . . . . . . . . . . | . . . . . . . Y . . / . . . . . . . | . . . . . . . . / . . . . . . . | . . . . . . . / . . . . . S . | . . . . . . . . . . . . . / A . . . . . . . . . C . . . . | . HSDN . . . . . . . . . . . / . . . . . . . . . . . . . . | . . . . / . . . . |
| IPS:43 4995 | 21-225_88G9 | VK3\|A27/ JK1 | . . . . . . . . RY / . . . . . . . . . . | . . . . . . . Y . . / . . . . . . . | . . . . . . . . / . . . . . . . | . . . . . . . / . . . . . S . | . . . . . . . . . . . . . / A . . . . . V . . . C . . . . | . HSDN . . . . . . . . . . . / . . . . . . . . . . . . . . | . . . . / . . . . |
| IPS:43 4999 | 21-225_75A8 | VK3\|A27/ JK1 | . . . . . . . . . Y / . . . . . . . . . . | . . . . . . . Y . . / . . . . . . . | . . . . . . . . / . . . . . . . | . . . . . . . / . . . . . . . | . . . . . . . . . . . . . / A . . . . . V . . . C . . . . | . HSDN . . . . . . . . . . . / . . . . . . . . . . . . . . | . . . . / . . . . |
| IPS:43 5013 | 21-225_89D5 | VK3\|A27/ JK1 | . . . . . . . . . Y / . . S . . . . . . . | . . . . . . . Y . . / . . . . . . . | . . . . . . . . / . . . . . . . | . . . . . . . / . . . . . S . | . . . . . . . . . . . . . / A . . . . . . . . . . . . . . | . H . DN . . . . . . . . . . . / . . . . . . . . . . . . . . | . . . . / . . . . |

3060

| ID | Clone | Germline | | | | | | | |
|---|---|---|---|---|---|---|---|---|---|
| IPS:43 5015 | 21-225_89H5 | VK3\|A27/ JK1 | ........... | .......N.. | ......... | ......... | ......V........... | ...E............... | ...... |
| | | | ........... | ....N... | ........ | .F.... | N.I............... | ...........V.. | .... |
| IPS:43 5025 | 21-225_89E10 | VK3\|A27/ JK1 | ..........Y | ........Y.. | ......... | ......... | V............... | .HSDN............. | .... |
| | | | ..S....... | ........ | ....... | .....S. | A.....V........... | ............... | |
| IPS:43 5029 | 21-225_89A11 | VK3\|A27/ JK1 | ........... | .......D.. | ......... | ......... | ............... | ...EI............ | ... |
| | | | ........... | ....NF.. | ....... | ..A.T. | .................S. | ............... | .... |
| IPS:43 5039 | 21-225_90G4 | VK3\|A27/ JK1 | ..........Y | .......Y.. | ......... | ......... | V............... | .HSDN............. | .... |
| | | | .......... | ........ | ....... | .....S. | A.....V....C..... | ............... | .. |
| IPS:43 5041 | 21-225_90A5 | VK3\|A27/ JK1 | ..........Y | .......Y.. | ......... | ......... | ............... | .H.DN............. | .... |
| | | | .......... | ........ | ....... | ....... | A.....V.H......... | ............... | . |
| IPS:43 5043 | 21-225_90G5 | VK3\|A27/ JK1 | ..........Y | .......Y.. | ......... | ......... | ............... | .H.DN............. | ... |
| | | | .......... | ........ | ....I.. | ....... | A.....V.H......... | ............... | .... |
| IPS:43 5055 | 21-225_90F10 | VK3\|A27/ JK1 | ..........Y | .......Y.. | ......... | ......... | ............... | .H.DN............. | ... |
| | | | .......... | ........ | ....... | .....S. | A.........C..... | ............... | . |
| IPS:43 5073 | 21-225_91B2 | VK3\|A27/ JK1 | ..........Y | .......Y.. | ......... | ......... | ............... | .H.DN............. | .... |
| | | | .......... | ........ | ....... | .....S. | A.....V....C..... | ............... | . |
| IPS:43 5075 | 21-225_91B3 | VK3\|A27/ JK1 | ..........Y | .......Y.. | ......... | ......... | ............... | .H.DN............. | .... |
| | | | .......... | ........ | ....... | .....S. | A.....V.H..C..... | ............... | . |
| IPS:43 5077 | 21-225_91F3 | VK3\|A27/ JK1 | ..........Y | .......Y.. | ......A. | ......... | ............... | .HSDN............. | .... |
| | | | .......... | ........ | ....... | .....S. | A.....V....C..... | ............... | |
| IPS:43 5079 | 21-225_91B4 | VK3\|A27/ JK1 | ..........Y | .......Y.. | ......... | ......... | ............... | .H.DN............. | .... |
| | | | .......... | ........ | ....I.. | .....S. | A.....V....C..... | ............... | |
| IPS:43 5089 | 21-225_91E9 | VK3\|A27/ JK1 | ..........Y | .......Y.. | ......... | ......... | .........C...... | .HSDN............. | .... |
| | | | .......... | ........ | ....... | ....... | A.....V.H..C..... | ............... | |
| IPS:43 5097 | 21-225_92B1 | VK3\|A27/ JK1 | .......... | .......G.. | .....R.. | ......... | D............... | ...E............... | .... |
| | | | .......... | ....N... | ....... | ....... | ............... | ............... | |
| IPS:43 5111 | 21-225_92D6 | VK3\|A27/ JK1 | ..........Y | .......Y.. | ......... | ......... | V.........C...... | .H.DN............. | .... |
| | | | .......... | ........ | ....... | .....S. | A.........C..... | ............... | |
| IPS:43 5115 | 21-225_77C5 | VK3\|A27/ JK1 | .......... | .......Y.. | ......... | ......... | ............... | .H.DN............. | .... |
| | | | .......... | ........ | ....... | .....S. | A............... | ............... | |
| IPS:43 5171 | 21-225_93C2 | VK3\|A27/ JK1 | .........Y | .......Y.. | ......... | ......... | ............... | .H.DN............. | ... |
| | | | .......... | ........ | ....... | ....... | A........H..C.... | ............... | |
| IPS:43 5177 | 21-225_93E4 | VK3\|A27/ JK1 | .........Y | .......Y.. | ......... | ......... | ..........C...... | .HSDN............. | .... |
| | | | .......... | ........ | ....... | .....S. | A.....V......... | ............... | |
| IPS:43 5183 | 21-225_93E9 | VK3\|A27/ JK1 | .......... | .......D.. | .....T.. | ......... | ............... | ...E............... | .... |
| | | | .......... | ........ | ....... | .....P | ............... | ............... | |
| IPS:43 5195 | 21-225_94D3 | VK3\|A27/ JK1 | ..Q....... | ....R... | ......... | ......... | ............... | .H.D.............. | |
| | | | .......... | ........ | ....... | ....... | ............... | ............... | ..NQ |
| IPS:43 5217 | 21-225_94F12 | VK3\|A27/ JK1 | ..........Y | .......Y.. | ......... | ......... | ............... | .H.DN............. | ...... |
| | | | ........F.. | ........ | ....... | .....S. | A............... | ............... | |
| IPS:43 5219 | 21-225_95D2 | VK3\|A27/ JK1 | .......... | .......Y.. | ......... | ......... | ............... | .H.DN............. | .... |
| | | | .......... | ........ | ....... | ....... | A.........C..... | ............... | |

| | | | | | | | | |
|---|---|---|---|---|---|---|---|---|
| IPS:43 5235 | 21-225_95F9 | VK3\|A27/ JK1 | ...........Y ............. | ........Y.. ........ | ......... ........ | ........ .....S. | ..........C...... A............... | .H.DN............. ............ | ...... .... |
| IPS:43 5237 | 21-225_95G9 | VK3\|A27/ JK1 | ..........Y ..S........ | .......Y.. ........ | ........ .....S. | ........ A............... | .................. ............ | .H.DN............. ........ | .... .... |
| IPS:43 5239 | 21- 225_95H10 | VK3\|A27/ JK1 | ....S....I.Y ..S........ | .......Y.. ........ | ........ ........ | ........ A............... | .....V.H.C.... .................. | .H.DN............. ............ | .... .... |
| IPS:43 5273 | 21-225_97A2 | VK3\|A27/ JK1 | ..........Y ..S........ | .......Y.. ........ | ........ .....S. | ........ A.........C.... | ..........C...... A............... | .HSDN............. ............ | .... .... |
| IPS:43 5281 | 21-225_97E5 | VK3\|A27/ JK1 | ............ ............ | .......Y.. ........ | ........ ........ | ........ A.....V....C... | ..........C...... .................. | .HSDN............. ............ | .... .... |
| IPS:43 5331 | 21- 225_147G8 | VK3\|A27/ JK1 | Q........... ............ | .....RIF.. ....N... | ........ ...I... | ........ ....T........... | .....I............ ............... | ...D............. ........ | .... .... |
| IPS:43 5815 | 21- 225_190G10 | VK3\|A27/ JK1 | ............ ............ | .....P..... ....RF.. | ........ ........ | ........ ...N............ | .................. .................. | ........SP... ............... | .... .... |
| IPS:43 5843 | 21- 225_191F1 | VK3\|A27/ JK1 | ............ .......A... | .......I.L. ....NF.. | ........ ........ | ........ .................. | ....N............ .................. | ...R............. .................. | ..V. .... |
| IPS:43 5847 | 21- 225_191A3 | VK3\|A27/ JK1 | ............ ............ | ......IR.. .....F.. | ........ ........ | ........ .................. | .................. ....N............ | .................. ....N............. | ..V. .... |
| IPS:43 5849 | 21- 225_191C3 | VK3\|A27/ JK1 | ............ ............ | ......IR.. .....F.. | ........ ........ | ........ .................. | .................. ....N............ | ..............A ............... | .... .... |
| IPS:43 5851 | 21- 225_191D3 | VK3\|A27/ JK1 | ............ .....S..... | ..G....IRT. ....NF.. | ....Q... ........ | ........ .................. | ....N............ .................. | ..............A .................. | .... .... |
| IPS:43 5865 | 21- 225_191A5 | VK3\|A27/ JK1 | ............ ............ | ............ ....RF.. | ........ ...N... | ........ .................. | .................. ....G............ | ........SP... .................. | .V... .... |
| IPS:43 5905 | 21- 225_190A3 | VK3\|A27/ JK1 | ..M........ ............ | .....NIR.. ....NF.. | ........ ........ | ........ .........V...... ....N............ | .................. ....N............ | ............... | .... .... |
| IPS:43 5911 | 21- 225_190B4 | VK3\|A27/ JK1 | ............ ............ | .......IR.. ....F.. | ........ ........ | ........ .................. | .................. ....N............ | ..............A .................. | .... .... |
| IPS:43 5913 | 21- 225_190A7 | VK3\|A27/ JK1 | ............ ............ | .........R... ....NF.. | .H...... ........ | ........ ..YR... | .................. ....N............ | ....N............. .................. | ...... .... |
| IPS:43 5939 | 21- 225_191H7 | VK3\|A27/ JK1 | ............ .....S..... | ..G....IRT. ....DF.. | ....Q... ........ | ........ .P..... | .................. ....N............F. | .................. ............... | ...N .... |
| IPS:43 5967 | 21- 225_192B3 | VK3\|A27/ JK1 | ............ ............ | .........R... .....F.. | ........ ........ | ........ ........ | .................. ....N............ | ..............A .................. | .... .... |
| IPS:43 5973 | 21- 225_192H3 | VK3\|A27/ JK1 | ............ ............ | ............ ....NF.. | ........ ......F | ........ ........ | .................. ....N............ | ....I............. .................. | .... .... |
| IPS:43 5995 | 21- 225_192F8 | VK3\|A27/ JK1 | ............ ............ | .......I... ............ | ........ ........ | ........ ........ | ...A............ .................. | ...E............. .................. | .... .... |
| IPS:43 6007 | 21- 225_192G12 | VK3\|A27/ JK1 | ............ ............ | .........R... ....DF.. | .L...... ........ | ........ .V.R... | .................. ....N............ | ....N............. .................. | .... .... |
| IPS:43 6009 | 21- 225_193A1 | VK3\|A27/ JK1 | ............ ............ | .........R... ....NF.. | .H...... ....F.. | ........ ...R... | .................. ....N............ | ....N............. .............. | .... .... |

| | | | | | | | | |
|---|---|---|---|---|---|---|---|---|
| iPS:43 6011 | 21-225_193B1 | VK3\|A27/ JK1 | ............ ....D...... | ........R.. ....NF.. | .E...... ........ | ........ ...R... | ............... ....N.........F. | ....N........... ................ | .... .... |
| iPS:43 6015 | 21-225_193D3 | VK3\|A27/ JK1 | ............ ............ | .......IR.. .....F.. | ........ ........ | ........ ...N... | ............... ............... | ....N........... ..............A | .... .... |
| iPS:43 6017 | 21-225_193F3 | VK3\|A27/ JK1 | ............ .....S..... | ..G....IRT. ....DF.V | ....Q... ........ | ........ ........ | .F.E........... ....N.......... | ............... ............... | .... ...N |
| iPS:43 6027 | 21-225_193E6 | VK3\|A27/ JK1 | ....A....... ............ | ........R.. ....G... | ........ ........ | ........ ........ | ......G........ ............... | ...E........... ............... | .... .... |
| iPS:43 6029 | 21-225_193H6 | VK3\|A27/ JK1 | ............ .....S..... | ..G....IRT. ....NF.. | ....Q... ........ | ........ ........ | ....N.........F. ............... | ............... ............... | .... .... |
| iPS:43 6035 | 21-225_193C8 | VK3\|A27/ JK1 | ..........F ............ | .......IR.. .....F.. | ........ ........ | ........ ........ | ............... ............... | ....N........... ..............A | ....I. ..V. |
| iPS:43 6037 | 21-225_193D8 | VK3\|A27/ JK1 | ...K......F ............ | .......IRT. ....NF.. | ........ ........ | ........ ........ | ............... ....N.......... | ....N........... ............... | .... .... |
| iPS:43 6041 | 21-225_193G8 | VK3\|A27/ JK1 | ............ ............ | ........RT. ....NF.. | .H...... ........ | ........ ...R... | ............... ....N.......L... | ....N........... ............... | .... .... |
| iPS:43 6047 | 21-225_193B10 | VK3\|A27/ JK1 | ............ ............ | .....P..... ........ | ........ ....V.. | ........ ...R... | ......R........ ............... | ........SP... ............... | .... .... |
| iPS:43 6049 | 21-225_193B12 | VK3\|A27/ JK1 | ............ ............ | .......IR.. .....F.. | ........ ........ | D....... ........ | ............... ............... | ....N........... ..............A | .... .... |
| iPS:43 6062 | 21-225_194E5 | VK3\|A27/ JK1 | ............ .....S..... | ..G....IRT. ....NF.. | ........ ........ | ........ ........ | ....N....K...... ............... | ............... ............... | .... .... |
| iPS:43 6064 | 21-225_194E6 | VK3\|A27/ JK1 | ............ ............ | .......IR.. ....NF.. | ........ ........ | ........ ........ | ........V...... ....N.......... | ............... ............... | .... .... |
| iPS:43 6072 | 21-225_194C10 | VK3\|A27/ JK1 | ............ ............ | .....P..N.. .....G... | ........ T......F | ........ ........ | ......A......A.. ............F. | ...E........... ............... | .... .... |
| iPS:43 6080 | 21-225_195B1 | VK3\|A27/ JK1 | ............ ..S........ | .....P..N.. ....N... | ........ T...... | ........ ...N... | .V.....A........ ....R.........F. | ...E........... ............... | .... .... |
| iPS:43 6088 | 21-225_195C8 | VK3\|A27/ JK1 | ............ ............ | .......IR.. .....F.. | ........ ........ | ........ ..F.... | ............... ............... | ....N........... ..............A | .... .... |
| iPS:43 6122 | 21-225_196G10 | VK3\|A27/ JK1 | ..........L ............ | .....P...N. .....F.. | ........ ........ | ........ ........ | ............... ...N........... | .........SP... ............... | .... ..L. |
| iPS:43 6134 | 21-225_196H12 | VK3\|A27/ JK1 | ..........F ............ | ........R.. ....NF.. | .H...... ........ | ........ ..YR... | ....N.......... ............... | ....N........... ............... | .... .... |
| iPS:43 6146 | 21-225_197F4 | VK3\|A27/ JK1 | ............ .....G..... | .......IR.. .....F.. | ..L..... ........ | ........ ........ | ............... ............... | ....N........... ..............A | .... .... |
| iPS:43 6177 | 21-225_198B1 | VK3\|A27/ JK1 | ............ .....S..... | ..G....IRT. ....NF.. | ....Q... ........ | ........ ........ | ............... ....N.......... | ............... ............... | .... .... |
| iPS:43 6179 | 21-225_198E1 | VK3\|A27/ JK1 | ............ ............ | .......IR.. ....NF.. | ........ ........ | ........ ..F.... | ............... ....N.......... | ....N........... ............... | .... .... |
| iPS:43 6181 | 21-225_198C2 | VK3\|A27/ JK1 | ...V.......L .YSE..S.... | ........R.. ........ | ........ ......C | ........ ..F...S | ............... ............... | ....N........... ............... | L.HA.. .... |

| iPS:43 | 21-225 | VK3\|A27/JK1 | | | | | | |
|---|---|---|---|---|---|---|---|---|
| iPS:43 6195 | 21-225_198G10 | VK3\|A27/ JK1 | ............ | .......IR.. | ........ | ........ | ................ | ....N............. | .... |
| | | | ............ | .....F.. | ........ | ........ | ................ | ............A | .... |
| iPS:43 6197 | 21-225_199C2 | VK3\|A27/ JK1 | ............ | .......IR.. | ........ | ........ | ................ | ....N............. | .... |
| | | | ............ | .....F.. | ........ | ........ | ....N........... | ............A | .... |
| iPS:43 6207 | 21-225_199C7 | VK3\|A27/ JK1 | ............ | .......IRT. | ........ | ........ | ................ | ....N............. | .... |
| | | | ............ | ....NF.. | ........ | ........ | ....N........... | ............... | .... |
| iPS:43 6210 | 21-225_199G11 | VK3\|A27/ JK1 | ...V........ | ........R.. | ........ | ........ | ................ | ....N............. | ..H... |
| | | | ............ | ........ | ........ | ..F.... | ................ | ............... | .... |
| iPS:43 6226 | 21-225_200F10 | VK3\|A27/ JK1 | ............ | ......NIR.. | ........ | ........ | ................ | ....N............. | .... |
| | | | ............ | ....F.. | ........ | ........ | ....N........... | ............A | .... |
| iPS:43 6232 | 21-225_201E1 | VK3\|A27/ JK1 | ........D... | .....P.IN.. | ........ | ........ | ................ | H..ET............ | .... |
| | | | ............ | ....GF.. | ........ | ........ | ..........MFH. | ............... | .... |
| iPS:43 6238 | 21-225_201B2 | VK3\|A27/ JK1 | ............ | ............ | ....R... | ........ | ................ | ...EN............ | .... |
| | | | ............ | ....N... | ........ | ........ | ..........F. | ............... | .... |
| iPS:43 6256 | 21-225_202D9 | VK3\|A27/ JK1 | ........D... | ........N.. | ........ | ........ | ................ | ...ET............ | .... |
| | | | ............ | ....G... | S...... | ........ | ..........H.. | ............... | .... |
| iPS:43 6302 | 21-225_205G7 | VK3\|A27/ JK1 | ............ | ........F.. | ........ | ........ | ................ | ...E............. | .... |
| | | | ............ | ....N... | ........ | ......A | ..........N..... | ............... | .... |
| iPS:43 6310 | 21-225_202D11 | VK3\|A27/ JK1 | ............ | ........IN.. | ...R... | ........ | ................ | ..EN............ | .... |
| | | | ............ | ....N... | ...V... | ........ | ..............F.. | ............... | .... |
| iPS:43 6336 | 21-225_208B5 | VK3\|A27/ JK1 | ............ | ............ | ....R... | ........ | ................ | .H.EN............ | .... |
| | | | ............ | ....N... | ........ | ........ | ..............F. | ............... | .... |
| iPS:43 6340 | 21-225_208A9 | VK3\|A27/ JK1 | ............ | ........N. | ........ | ........ | ................ | .H.H............. | .... |
| | | | ............ | ....N... | ...V... | ........ | ................ | ............... | .... |
| iPS:43 6472 | 21-225_220E1 | VK3\|A27/ JK1 | ............ | .......I.R. | ......N. | V...... | ................ | ................ | .... |
| | | | ............ | .....H.V | ......L. | .T..... | ....RS.......M... | ............... | .... |
| iPS:43 6506 | 21-225_222C7 | VK3\|A27/ JK1 | ............ | ........Y.. | ........ | ........ | ......G.......... | ...ED............ | .... |
| | | | ............ | ....N... | ........ | ........ | ..A........TI... | ............... | .... |
| iPS:43 6580 | 21-225_225E7 | VK3\|A27/ JK1 | ............ | ........Y.. | ........ | ........ | ................ | ....T............ | .... |
| | | | ............ | ........ | ........ | ........ | ................ | ..........R. | .... |
| iPS:43 7324 | 21-225_75C2 | VK3\|A27/ JK1 | .........RY | ........Y.. | ........ | ........ | ................ | .H.DN............ | ..R... |
| | | | ............ | ........ | ........ | ........ | A.........C..... | ............... | .... |
| iPS:43 7328 | 21-225_75D3 | VK3\|A27/ JK1 | ..........Y | ........Y.. | ........ | ........ | ..........C..... | .H.DN............ | .... |
| | | | ............ | ........ | ....I.. | ......S. | A.....V.H..C..... | ............... | .... |
| iPS:43 7332 | 21-225_75F3 | VK3\|A27/ JK1 | ..........Y | ........Y.. | ........ | ........ | .........C..... | .H.DN............ | .... |
| | | | ............ | ........ | ........ | ........ | A.......H...... | ............... | .... |
| iPS:43 7340 | 21-225_75G9 | VK3\|A27/ JK1 | ..A......... | .....P..D.. | ........ | ........ | ................ | ...E............ | .... |
| | | | ............ | ........ | ........ | ......P | ................ | ............... | .... |
| iPS:43 7344 | 21-225_75G12 | VK3\|A27/ JK1 | ..........Y | ........Y.. | ........ | ........ | ................ | .H.DN............ | .... |
| | | | ............ | ........ | ........ | ........ | A.....V....C..... | ............... | .... |
| iPS:43 7350 | 21-225_74A3 | VK3\|A27/ JK1 | ............ | ........Y.. | ........ | ........ | .........C...... | .HSDN............ | .... |
| | | | ............ | ........ | ........ | .....S. | A............... | ............... | .... |

| iPS | Clone | VK | K_FR1 | K_CDR1 | K_FR2 | K_CDR2 | K_FR3 | K_CDR3 | K_FR4 |
|---|---|---|---|---|---|---|---|---|---|
| iPS:43 7369 | 21-225_74D6 | VK3\|A27/JK1 | ............<br>............ | ........Y..<br>........ | ........<br>........ | ........<br>........ | .........C......<br>A............... | .H.DN............<br>............ | ......<br>.... |
| iPS:43 7383 | 21-225_74H8 | VK3\|A27/JK1 | ............<br>............ | .........F...<br>........ | ........<br>........ | ........<br>........ | .................<br>................. | ....−<br>......SR. | ......<br>.... |
| iPS:45 1122 | 21-225_200A1 | VK3\|A27/JK1 | .........I..<br>.Y......... | .........N.<br>....N... | ....R...<br>..S.... | ........<br>........ | C.L.......C......<br>.................C. | ...EI............<br>............ | ......<br>..S. |
| iPS:39 2864 | 21-225_23B9 | VK3\|A27/JK1 | ............<br>............ | ......N.Y..<br>........ | T......<br>........ | ......S<br>........ | .................<br>................. | .................<br>............R. | ......<br>.... |

| Germline | K_FR1 | K_CDR1 | K_FR2 | K_CDR2 | K_FR3 | K_CDR3 | K_FR4 |
|---|---|---|---|---|---|---|---|
| VK1\|A30/JK4 | DIQMTQSPSSLS ASVGDRVTIIC | RAS--QGIR- ----NDLG | WYQQKPGK APKRLIY | A- -ASSLQS | GVPSRFSGSGSG TEFTLTISSLQPEDFAT YYC | LQHNS ----------YPLT | FGGGTK VEIK |

| iPS | Clone | VK | K_FR1 | K_CDR1 | K_FR2 | K_CDR2 | K_FR3 | K_CDR3 | K_FR4 |
|---|---|---|---|---|---|---|---|---|---|
| iPS:46 8812 | 21-225_48H4 | VK1\|A30/JK4 | ......F.....<br>............ | .....RD....<br>........ | ........<br>........ | ........<br>........ | .................<br>................. | ...Y............<br>............ | ......<br>.... |
| iPS:46 8824 | 21-225_73G6 | VK1\|A30/JK4 | ............<br>............ | ........<br>........ | ........<br>........ | ........<br>........ | .................<br>................. | .................<br>............ | ......<br>.... |
| iPS:46 8818 | 21-225_190C8 | VK1\|A30/JK4 | ............<br>............ | ........<br>........ | ........<br>........ | ........<br>........ | .................<br>...............E.... | .....D............<br>............F. | ......<br>.... |
| iPS:46 8840 | 21-225_200H9 | VK1\|A30/JK4 | ............<br>............ | ........<br>........ | ........<br>........ | ........<br>........ | .I...............<br>................. | .................<br>................. | ......<br>.... |
| iPS:46 8868 | 21-225_74A1 | VK1\|A30/JK4 | ............<br>............ | ........<br>........ | ........<br>........ | ........<br>........ | .................<br>................. | ...D.............<br>................. | ....A<br>.... |
| iPS:39 2920 | 21-225_29G4 | VK1\|A30/JK4 | ............<br>............ | ........<br>........ | ........<br>........ | ........<br>........ | .................<br>................. | .................<br>................. | ......<br>...T |
| iPS:43 3899 | 21-225_43C3 | VK1\|A30/JK4 | ............<br>............ | ........<br>........ | ........<br>........ | ........<br>........ | .................<br>................. | .................<br>................. | ......<br>.G.T |
| iPS:43 3921 | 21-225_44C3 | VK1\|A30/JK4 | ............<br>............ | ........<br>........ | ........<br>........ | ........<br>........ | .........G.<br>................. | ...S.............<br>................. | ......<br>.... |
| iPS:43 3947 | 21-225_44E12 | VK1\|A30/JK4 | ............<br>............ | .T........<br>........ | ........<br>........ | ........<br>........ | .................<br>................. | .................<br>................. | ......<br>.... |
| iPS:43 3963 | 21-225_46B1 | VK1\|A30/JK4 | ............<br>............ | ........<br>........ | ........<br>........ | ........<br>......G | .................<br>................. | .................<br>................. | ......<br>.... |
| iPS:43 3969 | 21-225_46F3 | VK1\|A30/JK4 | ............<br>............ | ........<br>....K... | ........<br>........ | ........<br>........ | .................<br>S................ | .................<br>................. | ......<br>.... |
| iPS:43 3975 | 21-225_46C6 | VK1\|A30/JK4 | ............<br>............ | ........<br>........ | ........<br>........ | ........<br>........ | .................<br>................. | .................<br>................. | ......<br>...T |
| iPS:43 3977 | 21-225_46D8 | VK1\|A30/JK4 | ............<br>............ | ........<br>....K... | ........<br>........ | ........<br>........ | .................<br>S................ | .................<br>................. | ......<br>.... |
| iPS:43 3983 | 21-225_47A1 | VK1\|A30/JK4 | ............<br>............ | ......D....<br>........ | ........<br>........ | ........<br>.F.... | .........R......<br>................. | .................<br>................. | ......<br>.... |
| iPS:43 3987 | 21-225_47A5 | VK1\|A30/JK4 | ............<br>............ | ........<br>........ | ........<br>........ | ........<br>........ | .................<br>................. | .................<br>................. | ......<br>.... |

| iPS:43 4013 | 21-225_48D12 | VK1\|A30/ JK4 | . . . . . . . . . . . . . . . . . . . . . . . . | . . . . . . . . . . . . . . . . . . . | . . . . . . . . . . . . . . | . . . . . . . . . . . T . . . | . . . . . . I . . . . . . . . . . . . . . . . . . . . | . . . . . . . . . . . . . . . . . . . . . . . . . . . . . . . | . . . . . . . . . . . |
|---|---|---|---|---|---|---|---|---|---|
| iPS:43 4019 | 21-225_49A1 | VK1\|A30/ JK4 | . . . . . . . . . . . . . . . . . . . . . . | . . . . . . . . . . . . . . D | . . . . . . . . . . . . . . | . . . . . . . . . . . . . | . . . . . . . . . . . . . . . . . . . . | . . . . . . . . . . . . . . . . . . . . . . . . . . . | . . . . |
| iPS:43 4029 | 21-225_49C6 | VK1\|A30/ JK4 | . . . . . . . . . . . . . . . . . . . . . . | . . . . . . . . . . . . . . | . . . . . . . . . . . . . . | . . . . . . . . . . . . . | . F . . . . . . . . . . . . . . . . . . | . . . . . . . . . . . . . . . . . . . . . . . . . . . | . . . . |
| iPS:43 4043 | 21-225_50G10 | VK1\|A30/ JK4 | . . . . . . . . . . . . . . . . . . . . . . | . . . . . N . . . . . . . . | V . . . . . . . . . . . . . | . . . . . . . . . . . . . | . . . . . . . . . . . . . . . . . . . . | . . Y . . . . . . . . . . . . . . . . . . . . . . . . F . | . . S . |
| iPS:43 4077 | 21-225_51F11 | VK1\|A30/ JK4 | . . . . . . . . . A . . . . . . . . . . . . | . . . . . . . . . . . . . . | . . . . . . . . . . . . . . | . . . . . . . . . . . . . | . . . . . . . . . . . . . . . . . . . . | . . . . . . . . . . . . . . . . . . . . . . . F . | . . . . |
| iPS:43 4081 | 21-225_52B2 | VK1\|A30/ JK4 | . . . . . . . . . . . . . . . . . . . . . . | . . . . . . . . . . . . . . | . . R . . . . . | . . . F . . . . . . . . . | . . . . T . . . . . . . . . . . . . . | . . . . . . . . . . . . . . . . . . . . . . . . . . . | . . . . |
| iPS:43 4105 | 21-225_53D2 | VK1\|A30/ JK4 | . . . . . . . . . . . . . . . . . . . . . . | . . . . . . . . . . . . . . | . . . . . . . . . . . . . . | . . . . . . . . . . . . . | . . . . . . . . . . . . . . . . . . . . | . . . R . . . . . . . . . . . . . . . . . . . . . . | . . . . |
| iPS:43 4119 | 21-225_53E6 | VK1\|A30/ JK4 | . . . . A . . . . . . . . . . . . . . . . . | . . . . . . . . . . . . . . | . . . . N . . . . . . . . | . . . N . . . . . . . . | . . . . . . . . . . . . . . . . . . . F . | . . . R . . . . . . . . . . . . . . . . . . . . . . | . . . . |
| iPS:43 4141 | 21-225_54C6 | VK1\|A30/ JK4 | . . . . A . . . . . . . . . . . . . . . . . | . . . . . . . . . . . . . . | . . . . N . . . . . . . . | . . . N . . . . . . . . | . . . . . . . . . . . . . . . . . . . F . | . . . . R . . . . . . . . . . . . . . . . . . . . . | . . . . |
| iPS:43 4159 | 21-225_55B8 | VK1\|A30/ JK4 | S . . . . . . . . . . . . . . . . . . . . . | . . . . . . A . . . . . . . | . . . . . . . H | . . FR . . . | . . . . . . . . . . . . . . . . . . . . | . . . . . . . . . . . . . . . . . . . . . . . . . . . | . G . . |
| iPS:43 4179 | 21-225_56F1 | VK1\|A30/ JK4 | . . . . . . . . . . . . . . . . . . . . . . | . . . . . . D . . . . . . N . . | . . . . . . . N | . . . . . . . | . . . . . . . . . . . . . . . . . . . . | . . D . . . . . . . . . . . . . . . . . . . . . H . F . | . . . . |
| iPS:43 4217 | 21-225_60E8 | VK1\|A30/ JK4 | . . . . . . . . . . . . . . . . . . . F . . | . . . . . . . . . . . . . . | . F . . . . . . | . . . . . . . | . . . . . . . . . . . . . . . . . . . . | . . . S . . . . . . . . . . . . . . . . . . . | . . . . |
| iPS:43 4249 | 21-225_62E2 | VK1\|A30/ JK4 | . . . . . . . . . . . . . . . . . . . . . . | . . . . . . . . . . . . . . | . . . . . . . . | . . . R . . . | . . . . . . . . . . . . . . . . . . . . | . . . SN . . . . . . . . . . . . . | . . . . R |
| iPS:43 4253 | 21-225_62E4 | VK1\|A30/ JK4 | . . . . . . . . . . . . . . . . . . . . . . | . . . . . . . . . . . . . . | . . . . . . . . | . F . . . . . | . . . . . . . . . . . . . . . . . . . . | . . . . . . . . . . . . . . . . . . . . . . . . . . . | . . . . |
| iPS:43 4313 | 21-225_59E6 | VK1\|A30/ JK4 | . . . . . . . . . . . . . . . . . . . . . . | . . . . . . . . . . . . . . | . . . . . . . . | . . . . . . . | . . . . . . . . . . . . . . . . . . . . | . . . . . . . . . . . . . . . . . . . . . . . . . . . | . . . . R |
| iPS:43 4337 | 21-225_64E1 | VK1\|A30/ JK4 | . . . . . . . . . . . . . . . . . . . . . . | . . . . . . . . . . . . . . | . . . . . . . . | . . . . . . . | . . . . . . . K . . . . . . . . . L . . . | . . . . . . . . . . . . . . . . . . . . . . . . . . . | . . . . |
| iPS:43 4411 | 21-225_68F11 | VK1\|A30/ JK4 | . . . . . . . . . . . . . . . . . . . . . . | . . . . . . . . . . . . . . | . . . . . . . . | . . . . . . . | . . . . . . . . . . . . . . P . . . . . S . . . . . . . . . . . | . . . . . . . . . . . . . . . . . . . . . . . . F . | . . . . |
| iPS:43 4413 | 21-225_68D12 | VK1\|A30/ JK4 | . . . . . . . . . . . . . . . . . . . . . . | . . . . . . . . . . . . . . | . . . . . . . . | . . . . . . . | . . . . . . . . . . . . . . . . . . . . | . . . ST . . . . . . . . . . . . . | . . . . . . |
| iPS:43 4433 | 21-225_70E8 | VK1\|A30/ JK4 | . . . . . . . . . . . . . . . . . . . . . . T . . . . . . . . . | . . . . . . . . . . . . . . . . K . . . | . . . . . . . . | . . . . . . . | . . . . . . . . . . . . . . . . . . . . | . . . . R . . . . . . . . . . . . . . | . . . . |
| iPS:43 4439 | 21-225_70E12 | VK1\|A30/ JK4 | . . . . . . . . . . . . . . N . . . . . . . . . . . . . | . F . . . . . | . . . . . . . | . . . . . . . . . . . . . . . . . . . . | . . . . . . . . . . . . . . . . . . . . . . . . . . . | . . . . S . |
| iPS:43 4489 | 21-225_74E4 | VK1\|A30/ JK4 | . . . . . . . . . . . . . . . . . . . . . . | . . . . . . . . . . . . . . | . . . . . . . . | . . . T . . . | . . . . . G . . . . . . . . H . | . . . SN . . . . . . . . . . . . . | . . . . |

| ID | Clone | Germline | | | | | | |
|---|---|---|---|---|---|---|---|---|
| iPS:43 4503 | 21-225_74D7 | VK1|A30/ JK4 | .............<br>............. | ...........<br>....... | ........<br>....... | ........<br>....... | ...........F......<br>.............. | ...SN............<br>.... |
| iPS:43 5251 | 21-225_96A3 | VK1|A30/ JK4 | .............<br>............. | ...........<br>....... | ........<br>....... | ...T...<br>....... | ......G..R......H.<br>.............. | ...SN............<br>.... |
| iPS:43 5293 | 21-225_146F1 | VK1|A30/ JK4 | .............<br>............. | ...........<br>....... | ........<br>....... | ........<br>....... | ..............G........<br>.............. | ...ST............<br>.... |
| iPS:43 5311 | 21-225_146H9 | VK1|A30/ JK4 | .............<br>............. | ...........<br>....... | ........<br>....... | ........<br>....... | .............<br>.............. | ...............<br>.... |
| iPS:43 5313 | 21-225_146G11 | VK1|A30/ JK4 | .............<br>............. | ......D....<br>....NF. | ........<br>....... | ........<br>....... | ...L............<br>.............. | ...D............<br>.... |
| iPS:43 5361 | 21-225_148E11 | VK1|A30/ JK4 | .............<br>............. | ...........<br>....... | ........<br>......S | ........<br>....... | ......T.........<br>.F....V......... | ...RN............<br>.... |
| iPS:43 5363 | 21-225_148F12 | VK1|A30/ JK4 | .............<br>............. | .....A..<br>....... | ........<br>....... | ........<br>....... | ..............F.<br>.............. | ...............I<br>.... |
| iPS:43 5367 | 21-225_149G1 | VK1|A30/ JK4 | ....A......<br>............. | ...........<br>....... | ........<br>....... | ...N...<br>....... | .............<br>.............. | ...............<br>.... |
| iPS:43 5377 | 21-225_149G5 | VK1|A30/ JK4 | .............<br>............. | ...........<br>.....A.. | .F......<br>....... | ........<br>....... | .............<br>.............. | ...............<br>.... |
| iPS:43 5397 | 21-225_149F12 | VK1|A30/ JK4 | .............<br>............. | ...........<br>....... | ........<br>....... | ...N...<br>....... | ........I...<br>.............. | ...............<br>.... |
| iPS:43 5407 | 21-225_150E7 | VK1|A30/ JK4 | .............<br>............. | ...........<br>....... | ........<br>....... | ...N...<br>....... | ...L..........D.<br>.........A... | ...............<br>.... |
| iPS:43 5449 | 21-225_152H9 | VK1|A30/ JK4 | .............<br>............. | ...........<br>....... | ........<br>....... | ........<br>....... | ......T.........<br>.F.............. | ...SN............<br>.... |
| iPS:43 5499 | 21-225_156G1 | VK1|A30/ JK4 | .............<br>............. | ...........<br>....... | ........<br>....... | ........<br>....... | .F..............<br>.............. | ...SN............<br>.... |
| iPS:43 5549 | 21-225_158H5 | VK1|A30/ JK4 | ....I....F.F<br>............. | .......M...<br>....I... | ........<br>....... | R......<br>....... | .........C......<br>......V.R....S... | V...............<br>.... |
| iPS:43 5587 | 21-225_160H3 | VK1|A30/ JK4 | .............<br>............. | ...........<br>....... | ........<br>....... | ........<br>....... | .............<br>.............. | ...SN............<br>..S. ....Q |
| iPS:43 5599 | 21-225_160B10 | VK1|A30/ JK4 | .........P.<br>............. | ...........<br>....... | .......T<br>....... | ........<br>....... | ......N.........<br>.............. | ...S............<br>.... |
| iPS:43 5663 | 21-225_169B1 | VK1|A30/ JK4 | .............<br>............. | ...........<br>....... | ........<br>......G | .E.....<br>....... | ......G.........<br>.............. | ...Y............<br>.... |
| iPS:43 5669 | 21-225_169F9 | VK1|A30/ JK4 | .............<br>............. | ...........<br>....... | ........<br>......F | ........<br>....... | .............<br>.............. | ...Y............<br>.... |
| iPS:43 5693 | 21-225_170G4 | VK1|A30/ JK4 | .............<br>............. | ...........<br>....... | .H.....<br>....... | ...T...<br>....... | .............<br>.............. | ...Y............<br>.... |
| iPS:43 5695 | 21-225_170D5 | VK1|A30/ JK4 | .............<br>............. | ...........<br>....... | ..E....<br>...H... | ......N<br>....... | .............<br>.............. | ...Y............<br>..........F... |
| iPS:43 5697 | 21-225_170G5 | VK1|A30/ JK4 | .............<br>............. | ...........<br>....T... | .F......<br>....... | T......<br>....... | .............<br>.............. | ...Y............<br>.... |

| iPS:43 5703 | 21-225_170D11 | VK1\|A30/ JK4 | ............ ........... | ......... ....... | ....... ...H... | ....... ......N | ................. ................. | ...v.............. .........F... | ..... ...R |
|---|---|---|---|---|---|---|---|---|---|
| iPS:43 5705 | 21-225_171C3 | VK1\|A30/ JK4 | ............ ........... | ......... ....T... | .F...... ....... | T...... ....... | ................. ................. | ...Y.............. ................. | ..... .... |
| iPS:43 5709 | 21-225_171A4 | VK1\|A30/ JK4 | ............ ........... | ......... ....... | ....... ....... | T...... ....... | ................. ................. | ...Y.............. ................. | ..... .... |
| iPS:43 5721 | 21-225_172B3 | VK1\|A30/ JK4 | ............ ........... | ......... ....... | ....... ......G | ....... .E..... | ................. ......G.......... | ...Y.............. ................. | ..... .... |
| iPS:43 5725 | 21-225_172G8 | VK1\|A30/ JK4 | ............ ........... | ......V... ....... | ....... ...H... | ....... ......N | ................. ................. | .H.Y............. .........F... | ..... .... |
| iPS:43 5735 | 21-225_173H12 | VK1\|A30/ JK4 | ............ ........... | ......... ....... | ....... ....... | T...... .T..... | ................. .......S... | ...Y.............. .........F.N. | ..... .... |
| iPS:43 5743 | 21-225_175G1 | VK1\|A30/ JK4 | ............ ........... | ......... ....T... | ....... ....... | T...... ....... | ................. ................. | ...Y.............. ................. | ..... .... |
| iPS:43 5761 | 21-225_176B11 | VK1\|A30/ JK4 | ............ ........... | ......... ....... | .F...... ....... | ....... ....... | ...L........... | ...Y.............. ................. | ..... .... |
| iPS:43 5779 | 21-225_178B10 | VK1\|A30/ JK4 | ............ ........... | ......... ....... | ....... ...H... | ....... ......N | ................. ................. | .H.Y............. .........F... | ..... .... |
| iPS:43 6023 | 21-225_193A5 | VK1\|A30/ JK4 | ..........F .....I.S. | ......... ......... | ...Y... ....V.. | ....... ....... | .......F... .I....V.....E.... | ....D............. .......F.F. | .... .... |
| iPS:43 6033 | 21-225_193E7 | VK1\|A30/ JK4 | ............ ........... | ......... ......... | ....... ....... | S...... ......R | ................. ................. | ...KR............. ................. | .... .... |
| iPS:43 6120 | 21-225_196C10 | VK1\|A30/ JK4 | ............ ........... | ......... ......... | ....... ....... | ....... ....... | ................. ................. | ..Y............... ................. | .... .... |
| iPS:43 6199 | 21-225_199E3 | VK1\|A30/ JK4 | ............ ........... | ......... ......... | ....... ......S | S...... ......R | ................. ................. | ...KR............. ................. | .... .... |
| iPS:43 6228 | 21-225_200F12 | VK1\|A30/ JK4 | ............ ........... | ......... ......... | ......R ....... | S...... .....HT | ................. ................. | ...K.............. ................. | .... .... |
| iPS:43 6230 | 21-225_201A1 | VK1\|A30/ JK4 | ............ ........... | ......... ......... | ....... ....... | S...... ...I..R | ................. ................. | ...K.............. ................. | .... ..V. |
| iPS:43 6242 | 21-225_201A10 | VK1\|A30/ JK4 | ............ ........... | ......... ......... | ......R ....... | S...... .T...H. | ................. ................. | ...K.............. ................. | .... .... |
| iPS:43 6286 | 21-225_204H8 | VK1\|A30/ JK4 | ............ ........... | ......... ......... | ....... ....... | S...... ....... | .......R... ...V............ | ................. ................. | .... .... |
| iPS:43 6308 | 21-225_205H8 | VK1\|A30/ JK4 | ............ ........... | ......... ......... | ....Q.. ...L... | S...... ...F..R | ...........S.A... | ................. ................. | ....T .K.. |
| iPS:43 6526 | 21-225_224A1 | VK1\|A30/ JK4 | ............ ........... | ......E.. ......... | ....... ....... | ....... ....... | ................. ................. | ................. ................. | .... .... |
| iPS:43 6528 | 21-225_224B1 | VK1\|A30/ JK4 | ............ ........... | ......S.. ......... | ....... ....... | ....... ....... | ................. ................H. | ............P. ................. | .... .... |
| iPS:43 6538 | 21-225_224C3 | VK1\|A30/ JK4 | ............ ........... | ......D.... ......... | ....... ....... | ....... .T..... | ................. ................. | ................. ................. | .... .... |

| iPS ID | Clone | Germline | Region 1 | Region 2 | Region 3 | Region 4 | Region 5 | Region 6 | Region 7 |
|---|---|---|---|---|---|---|---|---|---|
| iPS:43 6556 | 21-225_224D10 | VK1\|A30/JK4 | ..L......... / ............ | ............ / ........ | ........ / ....... | ....... / G...... | ................. / ............. | .................. / ................. | ...... / .... |
| iPS:43 7220 | 21-225_55H6 | VK1\|A30/JK4 | ............ / ............ | ............ / ........ | ........ / ....... | G...... / ....... | ................. / ...........A... | ..RD............. / ...........F. | .... |
| iPS:43 7346 | 21-225_75H7 | VK1\|A30/JK4 | .........RY / .........NS | ............ / ........ | ........ / S.Q.... | D...... / ....... | ................. / ......V....GV... | I..SN........... / ............... | ...... |
| iPS:47 2730 | 21-225_14B1_LC1 | VK1\|A30/JK4 | ........Y. / .......... | ......D.... / ....DN.. | ........ / ....... | T...... / ..Y.... | ................. / ........... | ...YN........... / ............... | .... |
| iPS:39 2622 | 21-225_17H8 | VK1\|A30/JK4 | ............ / ........... | ............ / ........ | ........ / ....... | G...... / ....... | ................. / ...........T.... | ................. / ............... | .... |
| iPS:39 2624 | 21-225_17H12 | VK1\|A30/JK4 | ............ / ........... | ......D.... / ........ | ....A.. / ......N | ....... / ....... | .........I....... / ....TG........... | ...Y............ / ..........MF. | .... |
| iPS:39 2628 | 21-225_20C2 | VK1\|A30/JK4 | ............ / ...Q...... | ............ / ........ | ........ / ......F | ....... / ....... | ................. / ................. | ...A............ / ............... | ...E |
| iPS:39 2630 | 21-225_20E5 | VK1\|A30/JK4 | ............ / ........... | ............ / ........ | ........ / ....... | ....... / ....... | ................. / ................. | ................. / ............... | .... |
| iPS:39 2638 | 21-225_17F9 | VK1\|A30/JK4 | ............ / ........... | ......V.... / ........ | ........ / ....... | ....... / .V..... | ............. / ....N.... | ....T........... / ........... | ..F. |
| iPS:39 2640 | 21-225_18A1 | VK1\|A30/JK4 | ............ / ........... | ............ / ........ | ........ / ....... | ....... / ....... | ................. / ................. | ................. / ............... | .... |
| iPS:39 2642 | 21-225_18C6 | VK1\|A30/JK4 | ............ / ........... | .T......... / ........ | ........ / ....... | ....... / ....... | ................. / ................. | ...S............ / ............... | .... |
| iPS:39 2644 | 21-225_19E1 | VK1\|A30/JK4 | ............ / ........... | ............ / ........ | ........ / ....... | ....... / ...N... | ................. / ................. | ................. / .........F... | .... |
| iPS:39 2646 | 21-225_20G2 | VK1\|A30/JK4 | ............ / ........... | ............ / ........ | ........ / ....... | ....... / ....F.. | ................. / ...........S... | ................. / ............... | .... |
| iPS:39 2654 | 21-225_17A10 | VK1\|A30/JK4 | ............ / ........... | ............ / ........ | ........ / ....... | ....... / ....... | ................. / ................. | ................. / ............... | .... |
| iPS:39 2656 | 21-225_1F2 | VK1\|A30/JK4 | ............ / ........... | ............ / ........ | ........ / ....... | ....... / ....V.. | ................. / ............I... | ................. / ............... | .... |
| iPS:39 2658 | 21-225_18E8 | VK1\|A30/JK4 | .....A..... / ........... | ............ / ........ | ........ / ....... | ....... / ....... | ................. / ................. | ................. / ............... | .... |
| iPS:39 2666 | 21-225_16F11 | VK1\|A30/JK4 | ............ / ........... | ............ / ........ | ........ / ....... | ....... / ....V.. | ................. / ................. | ................. / ............... | .... |
| iPS:39 2678 | 21-225_19F3 | VK1\|A30/JK4 | ............ / ...R...... | ............ / ........ | ........ / ....... | ....... / .V..... | ................. / ................. | ...A............ / ............... | .... |
| iPS:39 2680 | 21-225_20A7 | VK1\|A30/JK4 | ............ / ........... | ............ / ........ | ........ / ....... | ....... / ....... | S................ / ................. | ................. / ............... | .... |
| iPS:39 2700 | 21-225_16E12 | VK1\|A30/JK4 | ............ / ........... | ............ / ........ | ........ / ....... | ....... / ....... | ................. / ................. | ................. / ............... | .... |
| iPS:39 2706 | 21-225_18A3 | VK1\|A30/JK4 | ............ / ........... | ............ / ........ | ........ / ....... | V...... / ....... | ......N.......... / ............... | ...S............ / ............... | .... |

| iPS ID | Clone | Germline | Seq 1 | Seq 2 | Seq 3 | Seq 4 | Seq 5 | Seq 6 | Seq 7 |
|---|---|---|---|---|---|---|---|---|---|
| iPS:39 2716 | 21-225_17B5 | VK1|A30/JK4 | ············· / ············· | ········· / ········ | ········ / ······· | ········ / ...N... | ··············· / SF·············· | ················· / ················· | ······ / ···· |
| iPS:39 2744 | 21-225_20D5 | VK1|A30/JK4 | ············· / ············ | ········· / ········ | ········ / ······· | ········ / ········ | ················ / ················ | ················· / ···············F· | ······ / ···· |
| iPS:39 2750 | 21-225_20A10 | VK1|A30/JK4 | ············· / ············ | ········· / ········ | ······Q·· / ······· | ········ / ········ | ················ / ················ | ·····R··········· / ················· | ······ / ···· |
| iPS:39 2772 | 21-225_20E12 | VK1|A30/JK4 | ············· / ············ | ········· / ········ | ········ / ······F | ········ / ········ | ················ / ················ | ················· / ···············F· | ······ / ···· |
| iPS:39 2774 | 21-225_21F3 | VK1|A30/JK4 | ············· / ············ | ········· / ········ | ········ / ······· | ········ / ········ | ···············D· / I··············· | ····S············ / ················· | ······ / ···· |
| iPS:39 2780 | 21-225_22B7 | VK1|A30/JK4 | ············· / ············ | ········· / ········ | ········ / ······· | T······· / ········ | ················ / ················ | ·····T··········· / ················· | ······ / ···· |
| iPS:39 2788 | 21-225_20C8 | VK1|A30/JK4 | ············· / ············ | ········· / ········ | ···K···· / ······N | ········ / ····· | ················ / ················ | ··D·············· / ···········F· | ······ / ···T |
| iPS:39 2794 | 21-225_21H3 | VK1|A30/JK4 | ············· / ············ | ········· / ········ | ········ / ······· | ········ / ···.V.T | ·······A··L.I··· / ················ | ················ / ················ | ······ / ···· |
| iPS:39 2800 | 21-225_22D12 | VK1|A30/JK4 | ············· / ············ | ········· / ········ | ········ / ········ | ········ / ········ | ···············D· / ················ | ····ST··········· / ················· | ······ / ···· |
| iPS:39 2810 | 21-225_20H12 | VK1|A30/JK4 | ············· / ············ | ········· / ·······D | ········ / ······· | ········ / ········ | ················ / ················ | ················· / ················· | ······ / ···· |
| iPS:39 2820 | 21-225_23D1 | VK1|A30/JK4 | ············· / ············ | ········· / ········ | ········ / ······· | ········ / ········ | ················ / ····V··········· | ····S············ / ················· | ······ / ···· |
| iPS:39 2822 | 21-225_23C8 | VK1|A30/JK4 | ·········R· / ············ | ······D···· / ········ | ·······R / ······N | G······· / ····V·· | ············VI··· / ················ | ················· / ················· | ······ / ···· |
| iPS:39 2824 | 21-225_24E5 | VK1|A30/JK4 | ············· / ············ | ········· / ········ | ········ / ······· | ········ / ········ | ················ / ················ | ····SN··········· / ················· | ······ / ···· |
| iPS:39 2834 | 21-225_22C1 | VK1|A30/JK4 | ············· / ············ | ·T······· / ········ | ········ / ······· | ········ / ········ | ················ / ················ | ····ST··········· / ················· | ······ / ···· |
| iPS:39 2838 | 21-225_22G8 | VK1|A30/JK4 | ····I······F / ············ | ·T········ / ········ | ········ / ······· | ········ / ········ | ····K··········· / ··S············ | ················· / ················· | ······ / ···· |
| iPS:39 2850 | 21-225_20H10 | VK1|A30/JK4 | G··········· / ············ | ········K·· / ····N·· | ········ / G··C··· | ········ / ······· | ················ / ················ | ················· / ················· | ······ / ···· |
| iPS:39 2854 | 21-225_21E5 | VK1|A30/JK4 | ············· / ············ | ········· / ········ | ········ / ······· | ········ / ·T····· | ················ / ················ | ················· / ················· | ······ / ···· |
| iPS:39 2858 | 21-225_22H4 | VK1|A30/JK4 | ············· / ············ | ········· / ········ | ········ / ······· | ········ / ····V·· | ················ / ···········L··· | ················· / ················· | ······ / ···· |
| iPS:39 2866 | 21-225_23H11 | VK1|A30/JK4 | ············· / ············ | ········· / ·······D | ········ / ······· | ········ / ········ | ················ / ················ | ····R············ / ················· | ······ / ···· |
| iPS:39 2870 | 21-225_20G9 | VK1|A30/JK4 | ············· / ············ | ········· / ········ | ········ / ······· | ········ / ········ | ················ / ················ | ····ST··········· / ················· | ······ / ···· |
| iPS:39 2880 | 21-225_22F9 | VK1|A30/JK4 | ············· / ············ | ········· / ········ | ········ / ······· | ········ / ········ | ················ / ················ | ················· / ················· | ······ / ···· |

| iPS:39 | clone | germline | | | | | | |
|---|---|---|---|---|---|---|---|---|
| iPS:39 2882 | 21-225_23A3 | VK1\|A30/ JK4 | . . . . . . . . . . <br> . . . . . . . . . . | . . . . . . . <br> . . . . . . . | . . . . . . . <br> . . . . . . . | . . . . . . . <br> . . . .V. . . | . . . . . . . . . . . . . . . . <br> . . . . . . . . . . . .I.F. | . . . . . . . . . . . . . . . <br> . . . . . . . . . . . . . . . | . . . . . . <br> . . . . |
| iPS:39 2896 | 21-225_21G7 | VK1\|A30/ JK4 | . . . . . . . . . . <br> . . . . . . . . . . | . . . . . .V. . . <br> . . . . . . . | . . . . . . . <br> . .Q. . . . | . . . . . . . <br> . . . . . . . | . . . . . .N. . . . . . . . <br> . . . . . . . . . . . . . . . | . . .S. . . . . . . . . . . . <br> . . . . . . . . . . . . . . . | . . . . . . <br> . . . . |
| iPS:39 2900 | 21-225_22F2 | VK1\|A30/ JK4 | . . . . . . . . . . <br> . . . . . . . . . . | . . . . . . . <br> . . . . . . . | . . . . . . . <br> . . . . . . . | . . . . . . . <br> . . . . . . . | . . . . . . . . . . . . . . . . <br> . . . . . . . . . . . . . . . | . . . . . . . . . . . . . . . <br> . . . . . . . . . . . . . . . | . . . . . . <br> . . . . |
| iPS:39 2904 | 21-225_22G9 | VK1\|A30/ JK4 | . . . . . . . . . . <br> . . . . . . . . . . | . . . . . . . <br> . . . . . . . | . . . . . . . <br> . . . . . . . | . . . . . . . <br> . . . . . . . | . . . . . . . . . . . . . . . . <br> . . . . . . . . . . . . . . . | . . .A. . . . . . . . . . . . . <br> . . . . . . . . . . . . . . . | . . . . . . <br> . . . . |
| iPS:39 2942 | 21-225_30E9 | VK1\|A30/ JK4 | . . . . . . . . . . <br> . . . . . . . . . . | . . . . . .D. . . . <br> . . . .D. . . | . . . . . . . <br> . .R. . . . | G. . . . . . <br> . .F. . . . | . . . . . . . . . . . . . . . . <br> . . . . . . . . . . . . . . . | . . .T. . . . . . . . . . . . . <br> . . . . . . . . . . . . . . P. | . . . . . . <br> . . . . |
| iPS:39 2944 | 21-225_31H5 | VK1\|A30/ JK4 | . . . . . . . . . . <br> . . . . . .F. .SF | . . . . . .D. . . . <br> . . . .S. . . | . . . . . . . <br> .H. .I. . | . . . . . . . <br> . . . . . . . | . . . . . .V. . . . . . . . <br> .F. . . .M. .D. .SN. . . | I. .II. . . . . . . . . . . . <br> . . . . . . . . . . . . . . P. | . . . . . . <br> . . . . |
| iPS:39 2964 | 21-225_31A8 | VK1\|A30/ JK4 | . . . . . . . . . . <br> . . . . . . . . . . | . . . . . .D. . . . <br> . . . .S. . . | . . . . . . . <br> . . . . . . . | . . . . . . . <br> .V. . . . . | . . . . . . . . . . . . . . . . <br> . . . . . . . . . . . . . . . | . . .TI. . . . . . . . . . . . <br> . . . . . . . . . . . . . . P. | . . . . . . <br> . . . . |
| iPS:39 2980 | 21-225_29H6 | VK1\|A30/ JK4 | . . . . . . . . . . . <br> . . . . . . . . . . | . . . . . . . . . . <br> . . . . . . . | . . . . . .S. . <br> T. . . . . . | . . . . . . . <br> . . . . . . . | . . . . . . . . . . . . . . . . <br> . . . . . . . . . . . . . . . | . . . . . . . . . . . . . . . <br> . . . . . . . . . . . . . . . | . . . . . . <br> . . . . |
| iPS:39 2982 | 21-225_30D1 | VK1\|A30/ JK4 | . . . . . . . . . . <br> . . . . . . . . . . | . . . . . .D. . . . <br> . . . .S. . . | . . . . . . . <br> . .E. . . . | . . . . . . . <br> . . . . . . . | . . . . . . . . . . . . . . . . <br> . . . . . . . . . . . .T. . . . | . . .TI. . . . . . . . . . . . <br> . . . . . . . . . . . . . . P. | . . . . . . <br> . . . . |
| iPS:39 2986 | 21-225_31B8 | VK1\|A30/ JK4 | . . . .I. . . . . . . <br> . . . . . . . . . . | . . . . . .D. . . . <br> . . . .S. . . | . . . . . . . <br> . . . . . . . | G. . . . . . <br> . . . . . . . | . . . . . . . . . . . . . . . . <br> . . . . . . . . . . . . . . . | . . .II. . . . . . . . . . . . <br> . . . . . . . . . . . . . . P. | . . . . . . <br> . . . . |
| iPS:39 2988 | 21-225_25E6 | VK1\|A30/ JK4 | . . . . . . . . . . <br> . . . . . . . . . . | . . . . . . . <br> . . . . . . . | . . .R. . . . <br> . . . . . . . | . . . . . . . <br> . . . . . . . | . . . . . .R. . . . . . . . <br> . . . .N. . . . . . . . . | . . . . . . . . . . . . . . . <br> . . . . . . . . . . . . . . . | . . . . . . <br> . . . . |
| iPS:39 2990 | 21- 225_25H10 | VK1\|A30/ JK4 | . . . . . . . . . . <br> . . . . . . . . . . | . . . . .R. . . . . <br> . . . . . . . . . | . . . . . . . <br> . . . . . . . | . . . . . . . <br> . .F. . . . | . . . . . . . . . . . . . . . . <br> . . . . . . . . . . . . . . . | . . . . . . . . . . . . . . . <br> . . . . . . . . . . . . . . . | . . . . . . <br> . . . . |
| iPS:39 3004 | 21- 225_30G11 | VK1\|A30/ JK4 | . . . . . . . . . . <br> . . . . . . . . . . | . . . . . .D. . . . <br> . . . .S. . . | . . . . . . . <br> . . . . . . . | . . . . . . . <br> . . . . . . . | . . . . . . . . . . . . . . . . <br> . . . .G. . . . . . . . .F. | . . .TI. . . . . . . . . . . . <br> . . . . . . . . . . . . . . P. | . . . . . . <br> . . . . |
| iPS:39 3018 | 21-225_29B8 | VK1\|A30/ JK4 | . . . . . . . . . . <br> . . . . . . . . . . | . . . . . . . <br> . . . . . . . | . . . . . . . <br> . . . . . . . | . . . . . . . <br> . . . . . . . | . . . . . . . . . .R. . . . . . <br> . . . . . . . . . . . . . . . | . . . . . . . . . . . . . . . <br> . . . . . . . . . . . . . . . | . . . . . . <br> . . . . |
| iPS:39 3030 | 21- 225_25H11 | VK1\|A30/ JK4 | . . . . . . . . . . <br> . . . . . . . . . . | . . . . . . . <br> . . . .T. . . | . . . . . . . <br> . . . . . . . | . . . . . . . <br> . . . . . . . | . . . . . . . . . . . . . . . . <br> . . . . . . . . . . . . . . . | . . . . . . . . . . . . . . . <br> . . . . . . . . . . . . . . . | . . . . . . <br> . . . . |
| iPS:39 3034 | 21-225_27F2 | VK1\|A30/ JK4 | . . . . . . . . . . <br> . . . . . . . . . . | . . . . . . . <br> . . . . . . . | . . . . . . . <br> . . . . . . . | V. . . . . . <br> . . . . . . . | . . . . . . . . . . . . . . . . <br> . . . . . . . . . . . . . . . | . . . . . . . . . . . . . . . <br> . . . . . . . . . . . . . . . | . . . . . . <br> . . . . |
| iPS:39 3040 | 21-225_30E3 | VK1\|A30/ JK4 | . . . . . . . . . . <br> . . . . . . . . . . | . . . . . .D. . . . <br> . . . .D. . . | . . . . . . . <br> . .R. . . . | . . . . . . . <br> . .F. . . . | . . . . . . . . . . . . . . . . <br> . . . . . . . . . . . . . . . | . . .T. . . . . . . . . . . . . <br> . . . . . . . . . . . . . . P. | . . . . . . <br> . . . . |
| iPS:39 3048 | 21-225_27C3 | VK1\|A30/ JK4 | . . . . . . . . . . <br> . . . . . . . . . . | . . . . . . . <br> . . . . . . . | . . . . . . . <br> . . . . . . . | . . . . . . . <br> . . . . . . . | . . . . . . . . . . . . . . . . <br> . . . . . . . . . . . . . . . | . . . .R. . . . . . . . . . . . <br> . . . . . . . . . . . . . . . | . . . . . . <br> . . . . |
| iPS:39 3054 | 21-225_29G8 | VK1\|A30/ JK4 | . . . . . . . . . . <br> . . . . . . . . . . | . . . . . . . <br> . . . . . . . | . . .L. . . . <br> . . . . . . . | . . . . . . . <br> . . . . . . . | . . . . . . . . . . . . . . . . <br> . . . . . . . . . . . . . .N. | . . . . . . . . . . . . . . . <br> . . . . . . . . . . . . . . . | . . . . . . <br> . . . . |
| iPS:39 3056 | 21-225_30F3 | VK1\|A30/ JK4 | . . . . . . . . . . <br> . . . . . . . . . . | . . . . . . . <br> . . . . . . . | . . . . . . . <br> . . . . . . . | T. . . . . . <br> . . . . . . . | . . . . . . . . . . . . . .D. <br> . . . . . . . . . . . . S. . . | . . . . . . . . . . . . . . . <br> . . . . . . . . . . . . . .F. | . . . . . . <br> . . . . |
| iPS:39 3058 | 21-225_31H3 | VK1\|A30/ JK4 | . . . . . . . . . . <br> . . . . . . . . . . | . . . . . .D. . . . <br> . . . .D. . . | . . . . . . . <br> . .R. . . . | . . . . . . . <br> . .F. . . . | . . . . . . . . . . . . . . . . <br> . . . . . . . . . . . . . . . | . . .T. . . . . . . . . . . . . <br> . . . . . . . . . . . . . . P. | . . . . . . <br> . . . . |

| iPS:39 | 21-225 | V/J | | | | | | |
|---|---|---|---|---|---|---|---|---|
| iPS:39 3060 | 21-225_32G12 | VK1\|A30/JK4 | ............ / ............ | ......D.... / ....S... | ....R... / ....... | ....... / ....... | ................. / ................. | ...TI............ / .............P. | .... / .... |
| iPS:39 3068 | 21-225_34G9 | VK1\|A30/JK4 | ............ / ............ | ......D.... / ....S... | ....... / ....... | ....... / ....... | .......T......... / .........A..I... | ...TI............ / .............P. | .... / .W.. |
| iPS:39 3072 | 21-225_36C5 | VK1\|A30/JK4 | ............ / ............ | ......D.... / ....S... | ....... / ....... | ....... / ....... | .......T......... / ......V......... | .H.PI............ / .............P. | .... / .W.. |
| iPS:39 3074 | 21-225_33B1 | VK1\|A30/JK4 | ............ / ............ | .T......... / ........ | .F...... / ....... | T....... / ...... | ................. / ................. | ................. / ................. | .... / ..V. |
| iPS:39 3076 | 21-225_33A4 | VK1\|A30/JK4 | ............ / ............ | ......D.... / ......V. | ........ / ..E.... | ........ / ......R | ................. / ............R... | ...Y............. / .............P. | .... / .... |
| iPS:39 3096 | 21-225_34D11 | VK1\|A30/JK4 | ............ / ............ | ......D.... / ....S... | ....... / ....... | ........ / ...... | ................. / ...VI........... | ...TI............ / .............P. | ....... / .G.. |
| iPS:39 3102 | 21-225_33F1 | VK1\|A30/JK4 | ............ / ............ | .T....D.... / ....S... | ........ / ....... | ........ / ...... | ................. / .............I... | ...TI............ / .............P. | .... / .... |
| iPS:39 3104 | 21-225_33A7 | VK1\|A30/JK4 | ............ / ............ | ......D.... / ....S... | ........ / ....... | V....... / ...... | ................. / ............A... | ...TI............ / .............P. | ....... / .... |
| iPS:39 3106 | 21-225_34A6 | VK1\|A30/JK4 | ............ / ............ | .T....D.... / ........ | ........ / ......F | ........ / ...... | ................. / ...V........... | ................. / .............P. | .... / .... |
| iPS:39 3110 | 21-225_35B7 | VK1\|A30/JK4 | ............ / ......I.... | ......D.... / ....S... | ........ / ..E.... | ........ / ...... | ................. / ................. | ...TI............ / .............P. | .... / .... |
| iPS:39 3118 | 21-225_34H11 | VK1\|A30/JK4 | ............ / ............ | ......D.... / ........ | ........ / ....... | ........ / .T..... | ................. / ................. | ................. / .............P. | .... / .... |
| iPS:39 3124 | 21-225_33G7 | VK1\|A30/JK4 | ............ / ............ | ........ / ........ | ........ / ....... | ........ / ...... | ................. / ................. | ...Y............. / .............P. | ....... / .... |
| iPS:39 3126 | 21-225_35D1 | VK1\|A30/JK4 | ............ / ............ | ......D.... / ....S... | ........ / ....... | ........ / ...... | ................. / ................. | ...TI............ / .............P. | ....... / .... |
| iPS:39 3128 | 21-225_35F11 | VK1\|A30/JK4 | ............ / ............ | ......D.... / ....S... | ........ / ....... | ........ / ...... | ................. / ................. | ...TV............ / .............P. | .... / .... |
| iPS:39 3146 | 21-225_34G8 | VK1\|A30/JK4 | ............ / ............ | ......D.... / ....S... | ........ / ....... | ........ / ...... | ................. / ................. | ...TI............ / .............P. | ....... / .... |
| iPS:39 3150 | 21-225_36A5 | VK1\|A30/JK4 | ............ / ............ | .T....D.... / ........ | ........ / ......F | ........ / V...... | ................. / ................. | .H............... / ............PK | ....I. / ...T |
| iPS:39 3804 | 21-225_5H7 | VK1\|A30/JK4 | ............ / ............ | ........ / ........ | ........ / ....... | V....... / .T....G | ................. / ................. | ................. / ................. | ...... / .... |
| iPS:39 3806 | 21-225_6A6 | VK1\|A30/JK4 | ............ / ............ | ........ / ........ | ........ / ....... | ........ / ...... | ................. / ................. | ...S............. / ................. | ...... / .... |
| iPS:39 3808 | 21-225_1A2 | VK1\|A30/JK4 | ............ / ............ | ........ / ........ | ........ / ....... | ........ / ...... | ................. / ................. | .............H... / ................. | .... / .... |
| iPS:39 3814 | 21-225_7F4 | VK1\|A30/JK4 | ............ / ............ | .T......... / ........ | ........ / ....... | ........ / ...N... | ................. / ................. | ...SA............ / ................. | ...... / .... |
| iPS:39 3816 | 21-225_6D4 | VK1\|A30/JK4 | ............ / ............ | ......A.... / ........ | ........ / ..... | ........ / ...... | ................. / ................. | ...S............. / ................. | .... / .... |

| iPS:39 3818 | 21-225_6G12 | VK1\|A30/ JK4 | . . . . . . . . . . . . . . . . . . . . . . . . | . . . . . . . . . . . . . . . . . | . . . . . . . . . . . . . . | . . . . . . . . . . . T . . . | . . . . . . . . . . . . . . . . . . . . . . . . . . . . . | . . . . . . . . . . . . . . . . . . . . . . . . . . . | . . . . . . . . . . |
|---|---|---|---|---|---|---|---|---|---|
| iPS:39 3820 | 21-225_8H7 | VK1\|A30/ JK4 | . . . . . . . . . . . . . . . . . . . . . . . . | . . . . . . . . . . . . . . . . . | . . . . . . . . . . . . . . | . . . . . . . . . . . . . . | . . . . . . . . . . . . . . . . . . . . . . . . . . . . . | . . . . . . . . . . . . . . . F . . | . . . . . . . . |
| iPS:39 3826 | 21-225_10G5 | VK1\|A30/ JK4 | . . . . . . . . . . . . . . . . . . . . . . . . | . . . . . . . . . . . . . . . . . | . . . . . . . . . . . . . . | . . . . . . . . . . . . . . | . . . . . . . . . . . . . . . . . . . . . . . . . . . . . | . . . . . . . . . . . . . . . . . . . . . . . . . . . | . . . . . . . . |
| iPS:39 3828 | 21-225_10H12 | VK1\|A30/ JK4 | . . . . . . . . . . . . . . . . . . . . . . . . | . . . . . . . . . . . . . . . . . | . . . . . . . . . . . . . . | G . . . . . . . . . . . . . | . . . . . . . . . . . . . . . . . . . . . . . . . . . . . | . . . . . . . . . . . . . . . . . . . . . . . . . . . | . . . . . . . . |
| iPS:39 3830 | 21-225_12A1 | VK1\|A30/ JK4 | . . . . . . . . . . . . . . . . . . . . . . . . | . . . . . . . . . . . . . . . . . | . . . . . . . . . . . . . . | . . . . . . . . . . . . . . | . . . . . . . . . . . . . . . . . . . . . . . . . . . . . | . . . . . . . . . . . . . . . . . . . . . . . . . . . | . . . . . . . . |
| iPS:39 3832 | 21-225_14B2 | VK1\|A30/ JK4 | . . . . . . . . . . . . . . . . . . . . . . . . | . . . . . . . . . . . . . . . . . | . . . . . . . . . . . . . . | V . . . . . . . .T . . . G | . . . . . . . . . . . . . . . . . . . . . . . . . . . . . | . . . . . . . . . . . . . . . . . . . . . . . . . . . | . . . . . . . . |
| iPS:39 3854 | 21-225_7H11 | VK1\|A30/ JK4 | . . . . . . . . . A . . . . V . . . I . | L . . . . . . . . . . . . . . . . | . . . . . . . . . . . I . . | V . . . . . . . . C . F . . | . . . . . . . . . Y . . . . . . . . . IM . . . . . . . . | . . . L . . . . . . . . . . . . . . . . . . . . . . . | . . . . . . . . |
| iPS:39 3856 | 21-225_14C2 | VK1\|A30/ JK4 | . . . I . . . . . F .C . . . . . I . . . | . . . . . . . D . . . . . . . . . | . . . . . . . . . . . . . . | . . . . . . . . . . . . . . | . . . . . . . . . . . . . . . . . . . V . R . . . . . . | V . . . . . . . . . . . . . . . . . . . . . . . . . . | . . . R |
| iPS:39 3866 | 21-225_14E3 | VK1\|A30/ JK4 | . . . . . . . . . . . . . . . . . . . . . . . . | . . . . . . . . . . . . . . . . . | . . . . . . . . . . . I . . | S . . . . . . . . . . . . . | . . . . . . I . . . C . . . . . R . . . . A . . S . . . | V . . Y . . . . . . . . . . . . . . . . . . . . . . F . | . . . . |
| iPS:39 3872 | 21-225_2A11 | VK1\|A30/ JK4 | . . . . . . . . . . . . P . . . . . . . . . . . | . . . . . . . . . . . . . . . . . | . . . . . . . . .Q . . . S | . . . . . . . . . . . . . . | . . . . . . . . . . . . . . . . . . . . . . . . D . . . | . . . . . . . . . . . . . . . . . . . . . . . . . . . | . . . . M . . . |
| iPS:39 3874 | 21-225_4C8 | VK1\|A30/ JK4 | . . . . I . . . . F .F .C . . . . . . . . | . . . . . . . . . . . . . . . . . | . . . . . . . . . . . . . . | . . . . . . . . . . . . . . | . . . . . . . . . . . . . . . . . . V . . . . . . . . . | . . . . . . . . . . . . . . . . . . . . . . . . . . . | . . . . |
| iPS:39 3880 | 21-225_15A1 | VK1\|A30/ JK4 | . . . . . . . . . . . . . . . . . . . . . . . . | . . . . . . . . . . . . . . . . . | . . . . . . . . . . . . . . | . . . . . . . . . . . . . . | . . . V . . . . . . . . . . S . . . . . . . . . . . . . | .HNS . . . . . . . . . . . . . . . . . . . . . . . VK | . . . . I . . . . T |
| iPS:39 3882 | 21-225_15E3 | VK1\|A30/ JK4 | . . . . . . . . . . . . . . . . . . . . . . . . | . . . . . . . . . . . . . . . . . | . . . . . . . . . . . . . . | . . . . . . . .S . . . . . | . . . . . . . . . . R . . . . . . . L . A . . . . . . . | . . . H . . . . . . . . . . . . . . . . . . . . . . . | . . . . . E . . . Y |
| iPS:39 3884 | 21-225_16F4 | VK1\|A30/ JK4 | . . . I . . . . . P . . . . . . . . . . . . . . | . . . . . . . . . . . . . . . . . | . . . . . S . . . . . . . . | V . . . . . . . . . . . . . | . . . . . . . . . . . . . . . I . . . . V . . . . . . . | I . . . . . . . . . . . . . . . . . . . . . . . . F . | . . . . |
| iPS:39 3886 | 21-225_2G9 | VK1\|A30/ JK4 | . . . . . . . . . . . . . . . . . . F . . . . . | . . . . . . . . . . . . . . . . . | . . . . . . . . .Q . . . . | . . . . . . . . . . . . . . | . . . . . . . . . . F . . . . . . . . . . L . . . . . . | . . . E . . . . . . . . . . . . . . . . . . . . . . . | . . . . |
| iPS:39 3922 | 21-225_2B2 | VK1\|A30/ JK4 | . . . . . . . . . . . . . . . . . . . . . . . . | . . . . . . . . . . . . . . . . . | . . . . . . . . . . . . .Q | . . . . . . . . . . . . . . | . . . . . . . . . . . . . . . . . . . . L . . . H . . . | . . . . . . . . . . . . . . . . . . . . . . . . . . . | . . . . |
| iPS:39 3928 | 21-225_4E10 | VK1\|A30/ JK4 | . . . . . . . . . . . . . . . . . . F . . . . . | . . . . . . . . . . . . . . . . . | . . . . . . . . . . . . . . | . . . . . . . . . . . . . . | . . . . . . . . . . F . . . . . . . . . . L . . . . . . | . . . DN . . . . . . . . . . . . . . . . . . . . . . . | . . . . |
| iPS:39 3934 | 21-225_13E6 | VK1\|A30/ JK4 | . . . V . . . . . . . . . . . . . . . . . . . S | . . . . . . . . . . . . . . . . . | . . . . . . . . . . . . . . | . . . . . . . . . . . . . . | . . . . . . . . . . . . . . . . . . . . . . . . . . . . | V . . . . . . . . . . . . . . . . . . . . . . . . . . | . . . . . A . . |
| iPS:39 3958 | 21-225_5H2 | VK1\|A30/ JK4 | . . . . . . . . . . . . . . .T . . . . . . . | . . . . . . . . . . . . . . . . . | . . . . . . . . . . . . . . | . . . . . . . . . . . . . . | S . . . . . . . . . . . . . . . . . . . . . . . . . . . | . . . . . . . . . . . . . . . . . . . . . . . . . . . | . . . . |
| iPS:39 3962 | 21-225_7H7 | VK1\|A30/ JK4 | . . . . . . . . . . . . . . . . . . . . . . . . | . . . . . . . . . . . . . . . . . | . . . . . . . . . . . . . . | . . . . . . . . . . . . . . | . .T . . . . . . . . . . . . . . . . . . . . . . . . . | . . . S . . . . . . . . . . . . . . . . . . . . F . | . V . . |
| iPS:39 3974 | 21-225_7C4 | VK1\|A30/ JK4 | . . . . . . . . . . . . . . . . . F . . . . . . | . . . . . . . . . . . . . . . . . | . H . . . . . . . . . . . . | . . . . . . . . . . . . . . | . . . . . . . . . . . . . . . . . . . . . . . . . . . . | . . . . . . . . . . . . . . . . . . . . . . . . . . . | . . . . |

| iPS ID | Clone | Germline | | | | | | |
|---|---|---|---|---|---|---|---|---|
| iPS:39 3976 | 21-225_7E9 | VK1\|A30/JK4 | ...........F<br>............ | ,,E.....<br>...... | T.......<br>...... | .................<br>................. | .................<br>................. | ......<br>.... |
| iPS:39 3982 | 21-225_6C12 | VK1\|A30/JK4 | ..........<br>......G.... | ............<br>....SN.. | .......<br>....... | .......<br>.....E. | ................F.<br>.................. | ..D.............<br>...........F. | .... |
| iPS:39 3984 | 21-225_4F12 | VK1\|A30/JK4 | ..........<br>.......... | ............<br>.......... | .......<br>....... | .......<br>....... | .......G.....I.<br>.............. | .................<br>...........A.. | .... |
| iPS:39 3990 | 21-225_11G7 | VK1\|A30/JK4 | ..........<br>.......... | ............<br>.......... | .......<br>....... | .......<br>...... | ..............<br>.............. | ...SN............<br>................. | .....M<br>...R |
| iPS:39 3994 | 21-225_8C9 | VK1\|A30/JK4 | ..........<br>..I....... | ......A....<br>.......D | .......<br>....... | .......<br>...... | .............<br>.........T..... | .................<br>................. | ......<br>.... |
| iPS:39 4002 | 21-225_15G7 | VK1\|A30/JK4 | ..........<br>.......... | ............<br>.......... | .......<br>....... | .......<br>...... | .I........F......<br>.............. | ...SN............<br>................. | ......<br>.... |
| iPS:39 4008 | 21-225_15H8 | VK1\|A30/JK4 | ..........<br>.......... | ............<br>........ | .......<br>......F | .......<br>...... | ..............<br>.............. | .................<br>................. | ......<br>...R |
| iPS:39 4020 | 21-225_15H10 | VK1\|A30/JK4 | ..........<br>.......... | ............<br>.......... | .......<br>....... | .......<br>...... | ..........S...<br>.............. | .................<br>................. | ......<br>...N |
| iPS:39 4024 | 21-225_16B7 | VK1\|A30/JK4 | ..........<br>.......... | ............<br>........ | .......<br>....... | .......<br>...... | ..............<br>.............. | .................<br>................. | ......<br>..FN |
| iPS:39 4037 | 21-225_4F4 | VK1\|A30/JK4 | ..........<br>.......... | ............<br>........ | .......<br>....... | ....V.T<br>...... | .............I...<br>.............. | .................<br>................. | ......<br>.... |
| iPS:39 4045 | 21-225_4H4 | VK1\|A30/JK4 | ..........<br>.......... | ............<br>........ | .......<br>....... | ....V..<br>...... | .............I...<br>.............. | .................<br>................. | ......<br>.... |
| iPS:39 4049 | 21-225_13H5 | VK1\|A30/JK4 | ..........<br>.......... | ...........<br>........ | .......<br>....... | ......T<br>...... | I.............<br>.............. | .................<br>................. | ......<br>.... |
| iPS:39 4053 | 21-225_11F10 | VK1\|A30/JK4 | ..........<br>.......... | ...........<br>........ | .......<br>....... | .......<br>...... | ...V..........<br>.............. | ...S............<br>................. | ......<br>.... |
| iPS:39 4057 | 21-225_15H1 | VK1\|A30/JK4 | ..........<br>.......... | ...........<br>........ | .......<br>....... | T.......<br>...... | ......N.........<br>.............. | ...S............<br>................. | ......<br>.... |
| iPS:39 4059 | 21-225_9E8 | VK1\|A30/JK4 | ..........F<br>.......... | ...........<br>........ | .......<br>....... | .......<br>...... | ..............<br>.............. | .................<br>................. | ......<br>.... |
| iPS:39 4063 | 21-225_16A1 | VK1\|A30/JK4 | ..........<br>.......... | ...........<br>........ | .......<br>....... | .......<br>...... | ..............<br>.............. | .H.SN...........<br>................. | ......<br>...E |
| iPS:39 4073 | 21-225_15C9 | VK1\|A30/JK4 | ..........<br>.......... | ...........<br>........ | .......<br>....... | .......<br>...... | .........V....<br>.............. | ...T............<br>................. | ......<br>.... |
| iPS:39 4075 | 21-225_8D12 | VK1\|A30/JK4 | ..........<br>.......... | ...........<br>........ | .......<br>....... | .......<br>...... | ......N.........<br>.............. | ...S............<br>................. | ......<br>.... |
| iPS:39 4079 | 21-225_11F5 | VK1\|A30/JK4 | ..........<br>.......... | ...........<br>........ | .......<br>....... | ....V..<br>...... | .............I...<br>.............. | .................<br>................. | .... |
| iPS:39 4091 | 21-225_13H3 | VK1\|A30/JK4 | ..........<br>.......... | ...........<br>....... | .......<br>....... | .......<br>...... | ..............<br>.............. | .................<br>................. | ......<br>.... |
| iPS:39 8528 | 21-225_32G1 | VK1\|A30/JK4 | ..........<br>.......... | ...DM...<br>....S... | .......<br>....... | .......<br>....... | ..............<br>.............. | ...TI...........<br>...........S.P. | ......<br>.... |

| | | | K_FR1 | K_CDR1 | K_FR2 | K_CDR2 | K_FR3 | K_CDR3 | K_FR4 |
|---|---|---|---|---|---|---|---|---|---|
| iPS:39 8534 | 21-225_33B8 | VK1\|A30/ JK4 | ............ | ......⊃.... | ........ | ........ | ................. | ...⊓I............. | ...... |
| | | | ............ | ....S... | ....... | ....... | ................F. | .............P. | .... |
| iPS:39 8540 | 21-225_35A6 | VK1\|A30/ JK4 | ............ | ......D.... | ........ | ........ | ................. | ...TI............. | ...... |
| | | | ............ | ....S... | ....... | .T..... | ................. | .............P. | |
| iPS:40 2219 | 21-225_1C12 | VK1\|A30/ JK4 | ............ | ......... | ........ | ........ | ................. | ................. | ...... |
| | | | ............ | ......... | ....... | ....... | ................. | ............. | .A.. |
| iPS:40 3868 | 21-225_19D11 | VK1\|A30/ JK4 | ............ | ........ | ........ | ........ | ................. | ..YY............. | .....E |
| | | | ............ | ........ | ....... | .T..... | ................. | ............. | .... |
| iPS:40 3872 | 21-225_8F11 | VK1\|A30/ JK4 | ............ | ........ | .F..... | D...... | ................. | ...YT............. | .... |
| | | | ............ | ....S... | ......F | ....V.. | .............I... | ............. | |
| Germline | | | K_FR1 | K_CDR1 | K_FR2 | K_CDR2 | K_FR3 | K_CDR3 | K_FR4 |
| | VK2\|A18/JK5 | | DIVMTQTPLSLS VTPGQPASISC | KSS-- QSLLHSD- GKTYLY | WYLQKPGQ SPQLLIY | E------ -VSSRFS | GVPDRFSGSGSG-- TDFTLKISRVEAEDVGV YYC | MQGIH----------LPIT | FGQGTR LEIK |
| iPS:46 8816 | 21-225_52G8 | VK2\|A18/ JK5 | ............ | ............ | ......T.. | ........ | ................. | ..SMQ............. | ...... |
| | | | ........M.. | E....... | P.H.... | ...K.L. | ......M......... | .............I | .... |
| iPS:43 4021 | 21-225_49C1 | VK2\|A18/ JK5 | ............ | ...........R | ........ | ........ | ................. | ..S.Q............. | L..... |
| | | | ............ | E....... | A..F..F | ...H... | ................. | ..........I... | .... |
| iPS:43 4025 | 21-225_49G3 | VK2\|A18/ JK5 | ............ | ............ | ......T.. | ........ | ................. | ..SMQ............. | ...... |
| | | | ........M.. | E....... | P.H.... | ...N.L. | ......M........F. | ............. | .... |
| iPS:43 4031 | 21-225_49E7 | VK2\|A18/ JK5 | ............ | ......IF... | ......T.. | ........ | ................. | ..SMQ............. | ...... |
| | | | ........FM.. | E....... | P.H.... | ...K.L. | ......M......... | .............I | .... |
| iPS:43 4033 | 21-225_49F9 | VK2\|A18/ JK5 | ............ | ..N.....V.N | ......F | ........ | ................. | ..S.Q............. | ...... |
| | | | ............ | E....... | P.....F | ...N... | ................. | ..........Y... | .... |
| iPS:43 4093 | 21-225_52D10 | VK2\|A18/ JK5 | .VM...I..... | ............ | ........ | ........ | ........R........ | ..S.Q............. | ...... |
| | | | ............ | E....... | P.....F | ...N.V. | ................. | ..........Y... | .... |
| iPS:43 4151 | 21-225_55C2 | VK2\|A18/ JK5 | .VM...I..... | ........V.. | ........ | ........ | ........R........ | ..S.L............. | ...... |
| | | | ............ | E....... | P.....F | ...N.V. | ................. | ..........Y... | .... |
| iPS:43 4161 | 21-225_55F9 | VK2\|A18/ JK5 | ............ | ............ | ........ | ........ | ................. | I.S.Q............. | ...... |
| | | | .....S..... | E....... | P...... | ...N... | ................. | ............. | .... |
| iPS:43 5329 | 21-225_147A8 | VK2\|A18/ JK5 | ............ | .T........ | ........ | ........ | ................. | ..S.- | ...... |
| | | | ............ | E....... | P...... | ...N... | ................. | .....QL.. | |
| iPS:39 2924 | 21-225_32H2 | VK2\|A18/ JK5 | ............ | R......... | ........ | ........ | ................. | L.S.Q............. | ...... |
| | | | ............ | ...R.... | P...... | .L.N... | .............I.S. | ..........Y... | .... |
| Germline | | | K_FR1 | K_CDR1 | K_FR2 | K_CDR2 | K_FR3 | K_CDR3 | K_FR4 |
| | VK2\|A18/JK1 | | DIVMTQTPLSLS VTPGQPASISC | KSS-- QSLLHSD- GKTYLY | WYLQKPGQ SPQLLIY | E------ -VSSRFS | GVPDRFSGSGSG-- TDFTLKISRVEAEDVGV YYC | MQGIH----------LPWT | FGQGTK VEIK |
| iPS:46 8822 | 21-225_147E10 | VK2\|A18/ JK1 | A........... | ......R...G | ........ | ........ | ................. | ..S.Q............. | ...... |
| | | | ............ | ........ | P.H...S | ...N... | ................. | ..........V... | .... |
| iPS:43 3917 | 21-225_43E11 | VK2\|A18/ JK1 | ............ | ...R.... | P...... | ...N... | ................. | ..S.Q............. | ...... |
| | | | ............ | | | | | | .... |

3075

| ID | Clone | Germline | Seq1 | Seq2 | Seq3 | Seq4 | Seq5 | Seq6 | Seq7 |
|---|---|---|---|---|---|---|---|---|---|
| iPS:43 3965 | 21-225_46F2 | VK2\|A18/JK1 | ..........T | .........G | ....... | ....... | ................. | ..S.Q............. | ..... |
| | | | ........... | ....... | P..V... | ...N... | ...L.......... | .............. | .... |
| iPS:43 3985 | 21-225_47C1 | VK2\|A18/JK1 | ........... | T......... | ....... | ....... | ................. | ..S.Q............. | ..... |
| | | | ........... | E...... | P...... | ...N... | ..............F.. | .............. | .... |
| iPS:43 3991 | 21-225_47E7 | VK2\|A18/JK1 | ........... | ......... | ....... | ....... | ................. | ..STQ............. | ..... |
| | | | ........... | ...R.... | P...... | ....... | A................ | .............. | A... |
| iPS:43 4345 | 21-225_64H9 | VK2\|A18/JK1 | E.......... | .........G | ....... | ....... | ................. | ..S.Q............. | ..... |
| | | | ........... | .......F | P..V... | ...N.LC | S................ | ..........V... | ...T |
| iPS:43 5297 | 21-225_146B3 | VK2\|A18/JK1 | ........... | .........G | ....... | ....... | ................. | ..S.Q............. | ..... |
| | | | ........... | ....... | P...... | ...N... | ..............L.H. | .............. | .... |
| iPS:43 5341 | 21-225_148B2 | VK2\|A18/JK1 | A.......... | .........G | ....... | ....... | ................. | ..S.Q............. | ..... |
| | | | ........... | ......F. | P...... | ...H... | ................. | ..........I... | .... |
| iPS:43 5357 | 21-225_148G10 | VK2\|A18/JK1 | ........... | .........G | ....R... | ....... | ................. | ..S.Q............. | ..... |
| | | | ........... | ....... | P...... | ...N... | ................. | .............. | .... |
| iPS:43 5365 | 21-225_149F1 | VK2\|A18/JK1 | ......S....F | .........G | ....... | ....... | ................. | ..S.Q............. | ..... |
| | | | .........Y | ......F. | P...... | ...H... | ................. | ..........I... | .... |
| iPS:43 5413 | 21-225_150B11 | VK2\|A18/JK1 | ........... | .......V.G | ....R... | ....... | ................. | ..S.Q............. | ..... |
| | | | ........... | ....... | P...... | ...N... | ................. | .............. | .... |
| iPS:43 5423 | 21-225_151G5 | VK2\|A18/JK1 | ........... | ......R...G | ....... | ....... | ................. | ..S.Q............. | ..... |
| | | | ........... | ....... | P....L. | ...N... | ................. | ..........V... | .... |
| iPS:43 5429 | 21-225_151A10 | VK2\|A18/JK1 | ........... | .........G | ....... | ....... | ................. | ..S.Q............. | ..... |
| | | | ........... | ....... | P...... | ...H... | ................. | ..........I... | .... |
| iPS:43 5441 | 21-225_152F6 | VK2\|A18/JK1 | ........... | .......R.G | ....R... | ....... | ................. | ..S.Q............. | ..... |
| | | | ........... | .......T | P.V..H | .I.K..T | ..N.........F... | ..........V... | .... |
| iPS:43 5457 | 21-225_152C11 | VK2\|A18/JK1 | ......A..... | .........G | ....... | ....... | ................. | ..S.Q............. | ..... |
| | | | ........... | ....... | P..I... | ...N... | ..N......F.F... | ..........I... | .D.. |
| iPS:43 5463 | 21-225_153D2 | VK2\|A18/JK1 | ........... | .......R.G | ....R... | ....... | ................. | ..S.Q............. | ..... |
| | | | ........... | .......T | P.V..H | ...K..T | ..N.........F... | ..........V... | .... |
| iPS:43 5489 | 21-225_155A5 | VK2\|A18/JK1 | ........... | .........G | ....... | ....... | ................. | ..S.Q............. | ..... |
| | | | ........... | ....... | P...... | ...N... | .............F... | ..........V... | .... |
| iPS:43 5531 | 21-225_157G8 | VK2\|A18/JK1 | ........... | .........G | ....... | ....... | ................. | ..S.Q............. | ..... |
| | | | ........... | ....... | ....... | .I.K... | ................. | ..........V... | .... |
| iPS:43 5577 | 21-225_160B1 | VK2\|A18/JK1 | ........... | .........G | ....... | ....... | .SE........... | ..S.Q............. | ..... |
| | | | ........... | ......F. | P..V... | ...K... | ................. | ..........I... | .... |
| iPS:43 5601 | 21-225_160G10 | VK2\|A18/JK1 | A.......... | .........G | ....... | ....... | ................. | ..S.Q............. | .V.... |
| | | | ........... | ....... | P.H.... | ...K... | ...........L... | .............. | ...T |
| iPS:43 5629 | 21-225_162H6 | VK2\|A18/JK1 | ........... | ..T......G | ....... | ....... | ...E.......... | K.S.Q............. | ..... |
| | | | ........... | ....... | P...... | ...K... | ................. | .............. | .... |
| iPS:43 5655 | 21-225_167E2 | VK2\|A18/JK1 | ........... | .........G | ....... | ....... | ................. | ..S.Q............. | ..... |
| | | | ........... | ....... | P.H.... | ...K... | ..............L.H. | .............. | .... |
| iPS:43 5657 | 21-225_167H10 | VK2\|A18/JK1 | ........... | .........G | ....... | ....... | ................. | ..S.Q............. | ..... |
| | | | ........... | ....... | P.H.... | ...K... | ...............L.H. | .............. | .... |

| iPS:43 5683 | 21- 225_170A1 | VK2\|A18/ JK1 | E........F.. | ..........G | ........ | ........ | ................ | ..S.Q............. | ...- |
|---|---|---|---|---|---|---|---|---|---|
| | | | ........... | .......F | P..V... | ...N... | ............... | ............. | .... |
| iPS:43 5723 | 21- 225_172B7 | VK2\|A18/ JK1 | ........... | ..........G | ........ | ........ | .......G........ | ..S.Q............. | .....R |
| | | | ........... | ......F. | P..V.LF | ...H... | ............... | .........F... | .D.. |
| iPS:43 5731 | 21- 225_173A11 | VK2\|A18/ JK1 | ........... | ..........G | ........ | ........ | ............... | ..S.Q............. | ...- |
| | | | ........... | ........ | P...... | ...N... | ..........FF. | .........V... | .... |
| iPS:43 5755 | 21- 225_176H4 | VK2\|A18/ JK1 | ........... | ..........G | ........ | ........ | ............... | ..S.Q............. | .....R |
| | | | ........... | ........ | P...... | ...N... | ...........I... | ..........I... | .... |
| iPS:43 5771 | 21- 225_177B11 | VK2\|A18/ JK1 | ........... | ......R...G | ........ | ........ | .............E. | ..S.Q............. | ...- |
| | | | ........... | ........ | P..I... | ...N... | ............... | ..........I... | .... |
| iPS:43 5781 | 21- 225_178G10 | VK2\|A18/ JK1 | H.......... | ..........G | ........ | ........ | .....L..G...... | ..S.Q............. | ...- |
| | | | ........... | ........ | P...... | ...N... | ...........I... | .........V... | .... |
| iPS:43 5789 | 21- 225_180C4 | VK2\|A18/ JK1 | ........... | ..........G | ........ | A....... | ..S............ | ..S.Q............. | ...- |
| | | | ........... | ........ | P...... | .T.N..P | ............... | .........V... | .... |
| iPS:43 5795 | 21- 225_181C2 | VK2\|A18/ JK1 | E.......... | ..........G | ........ | ........ | ............... | ..S.Q............. | ...- |
| | | | ........... | ........ | P....H | ...N... | ............... | .........V... | .... |
| iPS:43 5807 | 21- 225_181C10 | VK2\|A18/ JK1 | ........... | ..........G | ........ | ........ | ............... | ..S.Q............. | ...- |
| | | | ........... | ........ | P...... | ...N... | ...........H. | ..........I... | .... |
| iPS:43 5827 | 21- 225_190H11 | VK2\|A18/ JK1 | ........... | ...R.... | ........ | ........ | ..T........... | ..S.Q............. | ...- |
| | | | ........... | ...R.... | P....C | ...N..A | .........G...... | .........F... | .... |
| iPS:43 5839 | 21- 225_191B1 | VK2\|A18/ JK1 | ........... | R.......... | ........ | ........ | ............... | ..SFQ............. | ...- |
| | | | ........... | .......F | P..V... | .L.N... | ............... | ............. | ...N |
| iPS:43 5853 | 21- 225_191E3 | VK2\|A18/ JK1 | ........... | .......... | ........ | ........ | ..T........... | ..S.Q............. | ...- |
| | | | ........... | ...R.... | P....C | ...N..A | .........G...... | ............. | .... |
| iPS:43 5871 | 21- 225_191E6 | VK2\|A18/ JK1 | ........... | .......... | ........ | ........ | ..T........... | ..S............ | ...- |
| | | | ........... | ...R.... | P....C | ...N..A | ........G...I... | .........F... | .... |
| iPS:43 5887 | 21- 225_186F7 | VK2\|A18/ JK1 | .V..A...... | ..........G | ........ | ........ | ............... | ..S.Q............. | ...- |
| | | | ........... | ........C | P...... | ...K... | ............... | .........V... | .... |
| iPS:43 5899 | 21- 225_188G11 | VK2\|A18/ JK1 | ........... | M.........G | ........ | ........ | ....T.......... | ..S.Q............. | ...- |
| | | | ........... | ........ | P...... | ...N... | ............... | ..........I... | .... |
| iPS:43 5901 | 21- 225_189G2 | VK2\|A18/ JK1 | ........... | ..........G | ........ | ........ | ..S............ | ..S.Q............. | ...- |
| | | | ........... | .......F | P...... | ...N... | ............... | ..........I... | .... |
| iPS:43 5927 | 21- 225_190E7 | VK2\|A18/ JK1 | ...L....... | ............ | ........ | ........ | ............... | ..S.Q............. | ...- |
| | | | ........... | ...R.... | P..V..C | ...N... | .........G...... | ............. | .... |
| iPS:43 5999 | 21- 225_192F9 | VK2\|A18/ JK1 | ........... | ............ | ....R... | ........ | ..T........... | ..S.Q............. | ...- |
| | | | ........... | ...R.... | P....C | ...N..A | .........G...... | ............. | .... |
| iPS:43 6060 | 21- 225_194F4 | VK2\|A18/ JK1 | ........... | ............ | ........ | ........ | ............... | ..S.Q............. | ...- |
| | | | ........... | ...R.... | P....C | ...N... | .........G...... | ............. | .... |
| iPS:43 6158 | 21- 225_197G8 | VK2\|A18/ JK1 | E.......... | ......N.... | ........ | ........ | ............... | ..S.Q............. | .A..S. |
| | | | .I......... | ........ | P..V... | ...N... | ...........S. | ............. | .... |
| iPS:43 6193 | 21- 225_198A10 | VK2\|A18/ JK1 | ...L....... | .........Y. | ........ | ........ | ............... | ..S.Q............. | ...- |
| | | | ........... | ...R.... | P..V..C | ...N... | .........G...... | ............. | .... |

| | | | K_FR1 | K_CDR1 | K_FR2 | K_CDR2 | K_FR3 | K_CDR3 | K_FR4 |
|---|---|---|---|---|---|---|---|---|---|
| iPS:43 6536 | 21-225_224G1 | VK2\|A18/ JK1 | ............ ............ | ..G........ .....F.S | ....... P...... | ....... .I.N... | ................ ........IF.. | ..STQ............ ...........R. | ...... .... |
| iPS:43 6548 | 21-225_224A7 | VK2\|A18/ JK1 | ............ ............ | ............ .....F.. | .F..... P...... | ....... .I.N... | ................ ...........I... | ..STQ............ ...........R. | ...... .... |
| iPS:43 6558 | 21-225_224C11 | VK2\|A18/ JK1 | .F.......... ............ | ............ .....F.. | ....... P...... | ....... .I.N... | ................ ...........I... | ..STQ............ ...........R. | ...... .... |
| iPS:43 6562 | 21-225_224H11 | VK2\|A18/ JK1 | ............ ............ | ............ .....F.. | ....... P...... | ....... .I.N... | ................ ...........I... | ..STQ............ ...........R. | ...... .... |
| iPS:43 6572 | 21-225_225G4 | VK2\|A18/ JK1 | ............ ............ | ............ .....F.. | ....... P...... | ....... .I.N... | ................ ...........I... | ..STQ............ ...........R. | ...... .... |
| iPS:43 6592 | 21-225_226B1 | VK2\|A18/ JK1 | ............ ............ | .........G ....... | ....R... P.....N | ....... ...I... | ................ ...........I... | ..S.Q............ ...........I... | ...... .D.. |
| iPS:43 6594 | 21-225_226A5 | VK2\|A18/ JK1 | ............ ............ | .........G ....... | ....... P...... | ....... ...N... | ................ ................ | ..S.Q............ ...........I... | ...... .... |
| iPS:43 6602 | 21-225_226E7 | VK2\|A18/ JK1 | ............ ............ | .........G ....... | ..Q.... P..I... | ....... ...N... | ................ ................ | ..S.Q............ ...........V... | ...... .... |
| iPS:43 6606 | 21-225_226G8 | VK2\|A18/ JK1 | ............ ............ | ............ .....F.. | ....... P...... | ....... .I.N... | ................ ...........I... | ..STQ............ ...........R. | ...... .... |
| iPS:43 6610 | 21-225_226F9 | VK2\|A18/ JK1 | ............ ............ | ............ .....F.. | ....... P...... | ....... .I.N... | ................ ...........I... | ..STQ............ ...........R. | ...... .... |
| iPS:43 6612 | 21-225_226H9 | VK2\|A18/ JK1 | ............ .I.......... | ............ .....F.. | ....R... P...... | ....... ...N... | ................ ...........I... | ..STQ............ ...........R. | ...... .... |
| iPS:43 6614 | 21-225_226F10 | VK2\|A18/ JK1 | ............ ............ | ............ .....F.. | ....... P...... | ....... .I.N... | ................ ...........I... | ..STQ............ ...........R. | ...... .... |
| iPS:43 6618 | 21-225_226E11 | VK2\|A18/ JK1 | ............ ............ | ............ .....F.. | ...H.... P.H.... | ....... .I.N... | ................ ...........I... | ..STQ............ ...........R. | ...... .... |
| iPS:43 6624 | 21-225_226H12 | VK2\|A18/ JK1 | ............ ............ | .....KT.... .....F.. | ....... P..P... | ....... .I.N... | ................ ...........I... | ..STQ............ ...........R. | ...... .... |
| iPS:43 6626 | 21-225_227C1 | VK2\|A18/ JK1 | ............ ............ | ............ .....F.. | ....... P...... | ....... .I.N... | ................ ...........I... | ..STQ............ ...........R. | ...... .... |
| iPS:43 6628 | 21-225_227F2 | VK2\|A18/ JK1 | ............ ............ | ............ .....F.. | ....... P...... | ....... .I.N... | ................ ...........I... | ..STQ............ ...........R. | ...... .... |
| iPS:43 6640 | 21-225_227A8 | VK2\|A18/ JK1 | ............ ............ | ............ .....F.. | ....... P...... | ....... .I.N... | ................ ...........I... | ..STQ............ ...........R. | ......R .... |
| iPS:39 2814 | 21-225_22A1 | VK2\|A18/ JK1 | ............ ............ | ............ G....... | ....... P...... | ....... ...N... | .L.............. ...........A....... | ..TL............ ................ | ...... .... |
| iPS:39 2930 | 21-225_25H9 | VK2\|A18/ JK1 | ............ ............ | .........G .......F | ....... P...... | ....... ...N... | ................ ................ | ..S.Q............ ................ | ...... .... |
| iPS:39 3032 | 21-225_26F8 | VK2\|A18/ JK1 | ............ ............ | .........G ....... | ....... P...... | ....... ...N... | ................ ................ | ..S.Q............ ................ | ...... .... |
| iPS:39 3036 | 21-225_28G3 | VK2\|A18/ JK1 | E........... ............ | .........G ....... | ....... P...... | ....... ...N... | ...E............ ................ | ..S.Q............ ...........I... | ...... .... |
| | | Germline | K_FR1 | K_CDR1 | K_FR2 | K_CDR2 | K_FR3 | K_CDR3 | K_FR4 |

| | VK1|A30/JK 1 | | DIQMIQSPSSLS ASVGDRVTITC | RAS--QGIR-- ----NDLG | WYQQKPGK APKRLIY | A------- -ASSLQS | GVPSRFSGSGSG-- TEFTLTISSLQPEDFAT YYC | LQHNS------------- ----------YPWT | FGQGTK VEIK |
|---|---|---|---|---|---|---|---|---|---|
| IPS:46 8826 | 21-225_201C5 | VK1|A30/ JK1 | ............ ............ | ........... ....H... | ........ ........ | ....... ....... | ................ ................ | ...Y............ .........F.R. | ...... .... |
| IPS:46 8842 | 21-225_50H4 | VK1|A30/ JK1 | ............ ............ | ........... ........ | ........ ........ | ....... ....... | ................ ................ | ...Y............ ...........R. | ...... .... |
| IPS:46 8858 | 21-225_148C9 | VK1|A30/ JK1 | ............ ............ | .....R..... ....D... | ........ ........ | ....... ....... | ................ ................ | ...Y............ ...........R. | ...... .... |
| IPS:46 8860 | 21-225_224E7 | VK1|A30/ JK1 | ............ ............ | ........... ........ | ..T..... ........ | ....... ....... | ................ ................ | ...Y............ ...........R. | ...... .... |
| IPS:43 3919 | 21-225_44B3 | VK1|A30/ JK1 | ............ ............ | ........... ........ | ........ ........ | ....... ....... | ................ ................ | .L.YN........... ...........R. | ...... .... |
| IPS:43 3923 | 21-225_44D3 | VK1|A30/ JK1 | ............ ........F.. | ........... ....D... | ........ ........ | ....... ....... | ................ .........R....R.. | ...Y............ ...........R. | ...... .... |
| IPS:43 3929 | 21-225_44D5 | VK1|A30/ JK1 | ............ ............ | ......D.... ....K... | ........ ........ | ....... ...T.E. | ................ ................ | ...Y............ ..........F... | ...... .... |
| IPS:43 3935 | 21-225_44F9 | VK1|A30/ JK1 | .V.......... ............ | ........... ........ | ........ ........ | ....... ....... | ................ ................ | .H.YN........... ...........R. | ...... ...T |
| IPS:43 3939 | 21-225_44C10 | VK1|A30/ JK1 | ............ ........F.. | ........... ....D... | ........ ........ | ....... .T..... | ................ ................ | ...Y............ ...........R. | ...... .... |
| IPS:43 3951 | 21-225_45B4 | VK1|A30/ JK1 | ............ ........F.. | ........... ....D... | ........ ........ | ....... ....... | ................ ................ | ...Y............ ...........R. | ...... .... |
| IPS:43 3955 | 21-225_45B8 | VK1|A30/ JK1 | ............ ........F.. | ......D.... ....D... | ........ ........ | ....... ....... | ................ ................ | ...YN........... ...........R. | ...... .... |
| IPS:43 3967 | 21-225_46C3 | VK1|A30/ JK1 | ............ ........F.. | ........... ....D... | ........ ........ | ....... ....... | ................ .........R....... | ...Y............ ...........R. | ...... .... |
| IPS:43 3971 | 21-225_46D4 | VK1|A30/ JK1 | ............ ............ | .T.....D.... ....K... | ....V.. ....... | ....... .....E. | ................ ................ | ...Y............ ..........F... | ...... .... |
| IPS:43 3997 | 21-225_48C1 | VK1|A30/ JK1 | ............ ............ | .T......... ........ | ........ ........ | R...... ....... | ...A............ ............I... | ....F........... ............ | ...... .... |
| IPS:43 4001 | 21-225_48F2 | VK1|A30/ JK1 | ............ ............ | .....R..... ....D... | ........ P...... | ....... ....... | ......N......... ........S..L... | ..QY............ ...........R. | ...... .... |
| IPS:43 4009 | 21-225_48A9 | VK1|A30/ JK1 | ............ ............ | ........... ........ | ........ ....... | I...... ....... | ................ .....G.......I... | ....R........... ............ | ...... .... |
| IPS:43 4047 | 21-225_50A12 | VK1|A30/ JK1 | ............ ............ | ........... ........ | ........ ....... | T...... ...N... | ................ ................ | ................ ............ | ...... .... |
| IPS:43 4067 | 21-225_51H8 | VK1|A30/ JK1 | ............ ............ | ........... ........ | ........ ....... | ....... ....... | ................ ........G.....H. | ................ ............ | ...... .... |
| IPS:43 4135 | 21-225_54H3 | VK1|A30/ JK1 | ............ ............ | ......D.... .....I.. | ........ ....... | ....... ...N... | ................ ................ | ..Y............. ............ | ...... .... |
| IPS:43 4197 | 21-225_56C7 | VK1|A30/ JK1 | ............ ............ | ........... ........ | ........ ....... | T...... ...N... | ................ ................ | ................ ............ | ...... .... |

| iPS:43 4203 | 21-225_60E2 | VK1|A30/ JK1 | . . . . . . . . . . . . | . . . . . . . . . . . | . . . . . . . | . . . . . . . | . . . . . . . . . . . . . . . . | . . . . . . . . . . . . . . . . . . | . . . . . . |
| | | | . . . . . . . . . . . | . . . . K . . . | . . . . . . . | . . . . . . . | . . . . . . . . . . . . . . . . | . . . . . . . . . . . . . . . . . . | . . . . |
| iPS:43 4209 | 21-225_60C3 | VK1|A30/ JK1 | . . . . . . . . . . . . | . . . . . . . . . . . | . . . . . . . | S . . . . . . . | . . . . . . . . . . . . . . . . | . . . . . . . . . . . . . . . . . . | . . L . . . |
| | | | . . . . . . . . . . . | . . . . . . . . . | . . . . . . . | . . . . . . . | . . . . . . . . . . . . . . . . | . . . . . . . . . . . . . . . . . . | . . . . |
| iPS:43 4229 | 21-225_61H1 | VK1|A30/ JK1 | . . . . . . . . . . . . | . . . . . . . . . . . | . . . . . . . | . . . . . . . | . . . . . . . . . . . . . . . . | . . . . . . . . . . . . . . . . . . | . . . . |
| | | | . . . . . . . . . . . | . . . . K . . . | . . . . . . F | . . . . . . . | . . . . . . . . . . . . . . . . | . . . . . . . . . . . . . . . . . . | . . . . |
| iPS:43 4241 | 21-225_61E6 | VK1|A30/ JK1 | . . . . . . . . . . . . | . . . . . . . G . . | . . . . . . . | . . . . . . . | . . . . . . . . . . . . . . . . | . . . . . . . . . . . . . . . . . . | . . . . |
| | | | . . . . . . I . . . . | . . . . . . . . . | . . E . . . . | . . . . . . . | . . . . . . . . . . . . . . . . | . . . . . . . . . FP . . . | . . . . |
| iPS:43 4257 | 21-225_62F7 | VK1|A30/ JK1 | . . . . . . . . . . . . | . . . . . . A . . . . | . . . . . . . | P . . . . . . | . . . . . . . . . . . . . . . . | . . Y . . . . . . . . . . . . . . . | . . . . S . |
| | | | . . . . . . . . . . . | . . . . . . . . . | . . . . . . . | . . . R . . . | . . . . . . . . . . . . . . . . | . . . . . . . . . YP . . . | . . . . |
| iPS:43 4281 | 21-225_57B8 | VK1|A30/ JK1 | . . . . . . . . . . . . | . . . . . . . G . . | . . . . . . . | . . . . . . . | . . . . . . . . . . . . . . . . | . . . . . . . . . . . . . . . . . . | . . . . . . |
| | | | . . . . . . I . . . . | . . . . . . . . . | . . E . . . . | . . . . . . . | . . . . . . . . . . . . . . . . | . . . . . . . . . FP . . . | . . . . |
| iPS:43 4315 | 21-225_59G7 | VK1|A30/ JK1 | . . . . . . . . . . . . | . . . . . . . . . . . | . . . . . . . | S . . . . . . | . . . . . . . . . . . . . . . . | . . . . . . . . . . . . . . . . . . | . . . . |
| | | | . . . . . . . . . . . | . . . . . . . . . | . . . . . . . | . . . . . . . | . . . . . . . . . . . . . . . . | . . . . . . . . . . . . . . . . . . | . . . . |
| iPS:43 4319 | 21-225_59B9 | VK1|A30/ JK1 | . . . . . . . . . . . . | . . . . . D . . . . | . . . . H . . . | . . . . . . . . | . . . . . . . . TR . . . . . . | . . . . . . . . . . . . . . . . . . | . . . . |
| | | | . . . . . . . . . . . | . . . . . . . . . | . . . . . . . | . . . . . . . | . . . . . . . . . . . . . . . . | . . . . . . . . . . . . . . . . . . | . . . . |
| iPS:43 4339 | 21-225_64A4 | VK1|A30/ JK1 | . . . . . . . . . . . . | . . . . . . . . . . . | . . . . . . . | . . . . . . . | . . . . . . . . . . . . . . . . | . . . Y . . . . . . . . . . . . . | . . . . . . |
| | | | . . . . . . . . . . . | . . . . . . . . . | . . . . . . . | . . . . . . . | . . . . . . . . . . L . . . . | . . . . . . . . . . . . . R . | . . . . |
| iPS:43 4343 | 21-225_64C8 | VK1|A30/ JK1 | . . . . . . . . . . . . | . . . . . . . . . . . | . . . . . . . | . . . . . . . | . . . . . . . . . . . . . . . . | . H . Y . . . . . . . . . . . . . | . . . . . . |
| | | | . A . . . . . . . . . | . . . . . . . . . | . . . C . . . | . . . . . . . | . . . . . . . . . . . . . . . . | . . . . . . . . . . . . . R . | . . . . |
| iPS:43 4385 | 21-225_66C10 | VK1|A30/ JK1 | . . . . . . . . . . . . | . . . . . . A . . . . | . . . . . . . | P . . . . . . | . . . . . . . . . . . . . . . . | . . Y . . . . . . . . . . . . . . . | . . . . S . |
| | | | . . . . . . . . . . . | . . . . . . . . . | . . . . . . . | . . . . . . . | . . . . . . . . . . . . . . . . | . . . . . . . . . YP . . . | . . . . |
| iPS:43 4387 | 21-225_66D11 | VK1|A30/ JK1 | . . . . . . F . . . Q . | . . . . . . . . . . . | . . . . . . . | . . . . . . . | . . . . . . . . . . . . C . . . . . . | IV . . . . . . . . . . . . . . . . | . . . . . . |
| | | | . . . . . . . . . . . | . . . . . . . . . | . H . . . . . | . . . . C . . | . IS . . . M . R . . . . . . . . | . . . . . . . . . . . R . | . . . . |
| iPS:43 4441 | 21-225_71A2 | VK1|A30/ JK1 | . . . . . . . . . R . | . . . . . . . . . . . | . . . . . . . | I . . . . . . . | . . . . . . . . . . . . . . . . | IH . . . . . . . . . . . . . . . . | . . . . . . |
| | | | . . . . . . . . . . . | . . . . . . . . . | . . . . . . . | . . FR . . I | . . . . . . . . . . . . . . . . | . . . . . . . . . . . . . R . | . . . . |
| iPS:43 4469 | 21-225_73C9 | VK1|A30/ JK1 | . . . . . . . . . . . . | . . . . . . . . . . . | . . . . . . . | . . . . . . . | . . . . . . . . . . . . . . . . | . . . Y . . . . . . . . . . . . . | . . . . |
| | | | . . . . . . . . . . . | . . . . . . . . . | . . . . . . . | . . . . . . . | . . . . . . . . . . . . . . . . | . . . . . . . . . . . . . R . | . . . . |
| iPS:43 5197 | 21-225_94F3 | VK1|A30/ JK1 | . . . . . . . . . . . . | . . . . . . A . . . . | . . . . . . . | . . . . . . . | . . . . . . . . . . . . . . . . | . . . Y . . . . . . . . . . . . . | . . R . . . |
| | | | . . . . . . . . . . . | . . . . D . . . | . . Q . . . F | . . . . . . . | . . . . . . . . . . . . . . . . | . . . . . . . . . . . . . R . | . A . . |
| iPS:43 5325 | 21-225_147H5 | VK1|A30/ JK1 | . . . . . . . . . . . . | . . . . . R . . . . . | . . . . . . . | . . . . . . . | . . . . . . . . . . . . . . . . | . . . Y . . . . . . . . . . . . . | . . . . |
| | | | . . . . . . . . . . . | . . . . D . . . | . . . . . . . | . . . . . . . | . . . . . . . . . . . . . . . . | . . . . . . . . . . . . . R . | . . . . |
| iPS:43 5393 | 21-225_149D10 | VK1|A30/ JK1 | . . . . . . . . . . . . | . . . . . R . . . . . | . . . . . . . | . . . . . . . | . . . . . . . . . . . . . . . . | . . . Y . . . . . . . . . . . . . | . . . . |
| | | | . . . . . . . . . . . | . . . . . . . . . | . . . . . . . | . . . . . . . | . . . . . . . . . . . . . . . . | . . . . . . . . . . . . . R . | . . . . |
| iPS:43 5539 | 21-225_158G1 | VK1|A30/ JK1 | . . . . . . . . . . . . | . . . . . . . . . . . | . . . . . . . | T . . . . . . | . . . . . . . . C . . . . . . | . . . . . . . . . . . . . . . . . . | . . . . |
| | | | . . . . . . . . . . . | . . . . . . . . . | . . . . . . . | . . N . . . | . . . V . . . . . . . | . . . . . . . . . . . . . . . . . . | . . . . |
| iPS:43 5543 | 21-225_158D4 | VK1|A30/ JK1 | . . . . . . . . . . . . | . . . . . . D . . . . | . . . . . . . | . . . . . . . | . . . . . . . . . . . . . . . . | . . . Y . . . . . . . . . . . . . | . . . . |
| | | | . . . . . . . . . . . | . . . . K . . . | . . . . . . . | . . . . . . . | . . F . . . . . . . . . . . | . . . . . . . . . . . . . R . | . . . . |
| iPS:43 5571 | 21-225_159C8 | VK1|A30/ JK1 | . . . . . . . . . . . . | . . . . . . D . . . . | . . . . . . . | . . . . . . . | . . . . . . . . . . . . . . . . | . . . H . . . . . . . . . . . . . | . . . . . . |
| | | | . . . . . . . . . . . | . . . . K . . . | . . N . . . . | . . . . . . . | . . F . . . . . . . . . . . | . . . . . . . . . . . . . R . | . . . . |
| iPS:43 5573 | 21-225_159D8 | VK1|A30/ JK1 | . . . . . . . . . . . . | . . . . . RD . G . . | . . . . . . . | . . . . . . . | . . . . . . . . . . . . . . . . | . . . Y . . . . . . . . . . . . . | . . . . . . |
| | | | . . . . . . . . . . . | . . . . . . . . . | . . . . . . S | . . . . . . . | . . . F . . . . . . . . . . . . | . . . . . . . . . . . . . R . | . . . . |

| iPS:43 | 21-225 | Gene | | | | | | | |
|---|---|---|---|---|---|---|---|---|---|
| 5581 | 160H1 | VK1\|A30/JK1 | ............<br>............ | ......⊃....<br>........ | ........<br>........ | ........<br>......N | ................<br>..........T.... | ..................<br>..........F... | ....<br>.... |
| 5583 | 160F2 | VK1\|A30/JK1 | ............<br>............ | ........<br>........ | ........<br>........ | T......<br>...N... | ................<br>...V............ | ................<br>................ | ....<br>.... |
| 5591 | 160C4 | VK1\|A30/JK1 | ............<br>............ | ......D....<br>....K... | ........<br>..N.... | ........<br>........ | ................<br>...F............ | ...Y............<br>..............R. | ....<br>.... |
| 5615 | 161G12 | VK1\|A30/JK1 | ..........R.<br>............ | ......D....<br>....K... | ........<br>..N.... | ........<br>........ | ................<br>...F............ | ...H............<br>..............R. | ....<br>.... |
| 5675 | 169D7 | VK1\|A30/JK1 | ............<br>............ | ........<br>........ | ........<br>........ | ........<br>........ | ................<br>................ | ...H............<br>..........C... | ....<br>.... |
| 5681 | 169D11 | VK1\|A30/JK1 | ............<br>............ | ........<br>....D... | ........<br>V...... | ........<br>........ | ................<br>................ | ...Y............<br>..............R. | ....<br>.... |
| 5687 | 170H1 | VK1\|A30/JK1 | ............<br>............ | ........<br>........ | ........<br>........ | ........<br>.T..... | ................<br>................ | ...S............<br>..........N... | ....<br>..S. |
| 5689 | 170F3 | VK1\|A30/JK1 | ............<br>...Q...... | ......R....<br>........ | ........<br>........ | ........<br>........ | ................<br>....N........ | ...Y............<br>..............R. | ....<br>.... |
| 5741 | 174G10 | VK1\|A30/JK1 | ............<br>............ | ........<br>....D... | ........<br>V...... | ........<br>........ | ................<br>................ | ...H............<br>...........R. | ....<br>.... |
| 5831 | 190C12 | VK1\|A30/JK1 | ............<br>............ | ........<br>....K... | ........<br>......H | T......<br>........ | ..L........<br>................ | ................<br>................ | ....<br>.... |
| 5857 | 191A4 | VK1\|A30/JK1 | ............<br>............ | ........<br>....K... | ........<br>......H | T......<br>.......N | ..L........<br>................ | ................<br>................ | ....<br>.... |
| 5907 | 190G3 | VK1\|A30/JK1 | ............<br>...R...... | ........<br>....K... | ........<br>......H | T......<br>........ | ..L........<br>................ | ................<br>................ | ....<br>.... |
| 5919 | 190H5 | VK1\|A30/JK1 | ............<br>............ | ........<br>....K... | ........<br>......H | T......<br>........ | ..L........<br>................ | ....N...........<br>................ | ....<br>.... |
| 5989 | 192F7 | VK1\|A30/JK1 | ............<br>............ | ........<br>....K... | ........<br>......H | T......<br>........ | ..L........<br>................ | ...T............<br>................ | ......<br>...Y |
| 6132 | 196C12 | VK1\|A30/JK1 | .....L.....F<br>.C....I... | ............<br>........ | .S..N...<br>........ | ........<br>........ | ................<br>.I.......E.... | ....D............<br>.........F.F. | ..R...<br>.... |
| 6222 | 200C9 | VK1\|A30/JK1 | ............<br>............ | ..T.......<br>....K... | ........<br>......H | T......<br>........ | ..L........<br>................ | ................<br>................ | ....<br>.... |
| 6264 | 203F7 | VK1\|A30/JK1 | .........RF<br>............ | ............<br>....H... | ........<br>.L..... | ........<br>........ | .........C....<br>......V......N... | ...Y............<br>..........F.R. | ....<br>.... |
| 6274 | 204H3 | VK1\|A30/JK1 | ............<br>............ | ............<br>........ | ........<br>.EL... | ........<br>..A...G | ................<br>................ | ...Y............<br>..............R. | ......<br>..LQ |
| 6332 | 208B2 | VK1\|A30/JK1 | ............<br>............ | ............<br>....H... | ........<br>........ | ........<br>........ | ....G........<br>................ | ...Y............<br>..............R. | ....<br>.... |
| 6352 | 210G5 | VK1\|A30/JK1 | ............<br>............ | ............<br>....H... | ........<br>........ | ........<br>........ | ....G........<br>................ | .H.Y............<br>..............R. | ....<br>.... |
| 6386 | 212B11 | VK1\|A30/JK1 | ...........P<br>............ | ............<br>........ | ........<br>........ | ........<br>........ | ................<br>I............... | .L.Y............<br>...........R. | ....<br>.... |

| iPS:43 | 21- | VK1\|A30/ | | | | | | | |
|---|---|---|---|---|---|---|---|---|---|
| iPS:43 6412 | 21- 225_214H9 | VK1\|A30/ JK1 | ...........<br>........... | ........<br>....... | .......<br>....... | .......<br>....... | .................<br>................. | ...Y...............<br>..............R. | ....<br>.... |
| iPS:43 6414 | 21- 225_214G10 | VK1\|A30/ JK1 | .....LC...PP<br>........... | ...........<br>....... | ........<br>....... | .......<br>....... | .................<br>I................ | .L................<br>..............R. | ....<br>.... |
| iPS:43 6416 | 21- 225_214G12 | VK1\|A30/ JK1 | ........PP<br>........... | ...........<br>....... | ........<br>....... | .......<br>....... | .................<br>I................ | VM.Y..............<br>..............R. | ....<br>.... |
| iPS:43 6418 | 21- 225_215E3 | VK1\|A30/ JK1 | ........PP<br>........... | ...........<br>....... | ........<br>....... | .......<br>....... | .................<br>................. | VM..............<br>..............R. | ....<br>.... |
| iPS:43 6428 | 21- 225_215E11 | VK1\|A30/ JK1 | ......C...PP<br>........... | ...........<br>....... | ........<br>....... | .......<br>....... | .................<br>I.....V.R....... | VM.Y..............<br>..............R. | ....<br>.... |
| iPS:43 6438 | 21- 225_216E8 | VK1\|A30/ JK1 | ........P<br>........... | ...........<br>....... | ........<br>....... | .......<br>....... | .................<br>I................ | .M.Y..............<br>..............R. | ....<br>.... |
| iPS:43 6440 | 21- 225_216H12 | VK1\|A30/ JK1 | .....L.....P<br>........... | ...........<br>....... | ........<br>....... | G.......<br>....... | .................<br>I................ | VM..............<br>..............R. | ....<br>.... |
| iPS:43 6450 | 21- 225_217E5 | VK1\|A30/ JK1 | ......C...PP<br>.F........ | ...........<br>....... | ........<br>......C | .......<br>....... | .................<br>I................ | VM..............<br>..............R. | ....<br>.... |
| iPS:43 6456 | 21- 225_217G10 | VK1\|A30/ JK1 | ........PP<br>........... | ...........<br>....... | ........<br>....... | .......<br>....... | .................<br>I................ | VM..............<br>..............R. | ....<br>.... |
| iPS:43 6458 | 21- 225_217H12 | VK1\|A30/ JK1 | ........PP<br>........... | ...........<br>....... | ........<br>....... | .......<br>....... | .................<br>I................ | .M.Y..............<br>..............R. | ....<br>.... |
| iPS:43 6462 | 21- 225_218C4 | VK1\|A30/ JK1 | ......C...PP<br>.F........ | ...........<br>....... | ........<br>....... | .......<br>....... | .................<br>I.....V.R....... | VM.Y..............<br>..............R. | ....<br>.... |
| iPS:43 6480 | 21- 225_220F8 | VK1\|A30/ JK1 | .....L....PP<br>.F.......R | ...........<br>....... | ........<br>......C | G.......<br>....... | .................<br>I.......R....... | VM..............<br>..............R. | ....<br>.... |
| iPS:43 6534 | 21- 225_224F1 | VK1\|A30/ JK1 | A..........<br>........... | ...........<br>....D... | ........<br>....... | .......<br>....... | .....I.........<br>...........I... | ...Y..............<br>..............R. | ....<br>.... |
| iPS:43 6540 | 21- 225_224F3 | VK1\|A30/ JK1 | ...........<br>........... | ...........<br>....... | ........<br>..E.... | .......<br>....... | .................<br>................. | ...YN.............<br>............RA | ....<br>.... |
| iPS:43 6564 | 21- 225_225A1 | VK1\|A30/ JK1 | ...........<br>........... | ...........<br>....D... | ........<br>....I... | .......<br>....... | .................<br>................. | ...Y..............<br>..............R. | ....<br>.... |
| iPS:43 6596 | 21- 225_226C6 | VK1\|A30/ JK1 | ...........<br>..I........ | ...........<br>....... | ........<br>....... | .......<br>....... | .L...............<br>.....N.......... | ...YN.............<br>............RA | ....<br>...Q |
| iPS:43 6604 | 21- 225_226F7 | VK1\|A30/ JK1 | ...........<br>........... | ........G..<br>....... | ........<br>....... | .......<br>....... | .........V......<br>I................ | .H.Y..............<br>..............R. | ....<br>.... |
| iPS:43 6620 | 21- 225_226H11 | VK1\|A30/ JK1 | ...........<br>........... | .T.........<br>....... | ........<br>....... | .......<br>Y...... | ............D.<br>................. | ...Y..............<br>..............R. | ....<br>.... |
| iPS:43 7262 | 21- 225_170E4 | VK1\|A30/ JK1 | ...........<br>........... | ...........<br>....... | Y.......<br>....... | V.......<br>...G... | .................<br>................. | ................<br>.........YP... | ....<br>.D.. |
| iPS:43 7280 | 21- 225_203C10 | VK1\|A30/ JK1 | ...........<br>........... | ......D....<br>....... | ......A.<br>....... | R.......<br>....... | .................<br>...........A... | ................<br>................. | ....<br>.... |
| iPS:43 7286 | 21- 225_208F1 | VK1\|A30/ JK1 | ...........<br>........... | ...........<br>....H... | ........<br>......F | .......<br>....... | .................<br>................. | ...Y..............<br>.........F.R. | ....<br>.... |

| iPS ID | Clone | V/J | Seq 1 | Seq 2 | Seq 3 | Seq 4 | Seq 5 | Seq 6 | Seq 7 |
|---|---|---|---|---|---|---|---|---|---|
| iPS:43 7290 | 21-225_210G6 | VK1\|A30/JK1 | ..........RF<br>.F........ | ............<br>....H... | ........<br>.L..... | ........<br>....S.. | ..........C......<br>......V.R....N... | V..Y.............<br>..........F.R. | ....<br>.... |
| iPS:45 1114 | 21-225_159A3 | VK1\|A30/JK1 | ..........<br>.......... | ......D....<br>....K... | ........<br>..N.... | .......<br>....... | ............<br>...F.... | ...H............<br>............R. | ....<br>.... |
| iPS:39 2626 | 21-225_18A5 | VK1\|A30/JK1 | ..........<br>.......... | ........<br>........ | ........<br>........ | .......<br>....... | ............<br>............ | ............<br>............ | ....<br>.... |
| iPS:39 2634 | 21-225_17H3 | VK1\|A30/JK1 | ..........<br>.......F.. | ........<br>....S... | ........<br>........ | .......<br>....... | ............<br>............ | ..QY...........<br>..........R. | ....<br>.... |
| iPS:39 2674 | 21-225_18C2 | VK1\|A30/JK1 | ..........<br>.......... | ........<br>........ | ........<br>........ | T......<br>....... | ..........F....<br>............ | ............<br>............ | ..L...<br>.V.. |
| iPS:39 2686 | 21-225_17C7 | VK1\|A30/JK1 | ..........<br>.......... | ........<br>........ | ........<br>........ | .......<br>....... | ............<br>............ | ............<br>............ | ......<br>.... |
| iPS:39 2690 | 21-225_18F2 | VK1\|A30/JK1 | ..........<br>.......... | ........<br>........ | ........<br>........ | .......<br>....... | ............<br>............ | ............<br>............ | ....<br>.... |
| iPS:39 2710 | 21-225_19A10 | VK1\|A30/JK1 | ..........<br>.......... | ........<br>....T... | ....R...<br>........ | T......<br>....... | ............<br>....R...... | ...G.............<br>............ | ....<br>.... |
| iPS:39 2740 | 21-225_18H12 | VK1\|A30/JK1 | ..........<br>.......... | ........<br>........ | ........<br>........ | T......<br>...N... | ............<br>............ | ....N............<br>............ | ..L...<br>.V.. |
| iPS:39 2742 | 21-225_20B2 | VK1\|A30/JK1 | ..........<br>......N... | ......D....<br>........ | ........<br>........ | .......<br>....... | ............<br>............ | ...YN............<br>..........RA | ......<br>.D.. |
| iPS:39 2758 | 21-225_21G11 | VK1\|A30/JK1 | ..........<br>.......... | ........<br>........ | ........<br>........ | T......<br>....... | ............<br>............ | ....N............<br>............ | ..L...<br>.V.. |
| iPS:39 2790 | 21-225_20D10 | VK1\|A30/JK1 | ..........<br>.F........ | ........<br>........ | ........<br>.R..... | V......<br>..Y.... | .........Y......<br>............ | I.Q..............<br>............ | ......<br>.... |
| iPS:39 2796 | 21-225_22A4 | VK1\|A30/JK1 | ..........<br>.......... | ........<br>........ | ........<br>........ | .......<br>....... | ............<br>............ | ............<br>............ | ......<br>.... |
| iPS:39 2832 | 21-225_21H8 | VK1\|A30/JK1 | ..........<br>.......... | ........<br>........ | ........<br>........ | .......<br>....... | ......R....<br>............ | ............<br>............ | ......<br>.... |
| iPS:39 2836 | 21-225_22F4 | VK1\|A30/JK1 | ..........<br>.......... | ......V....<br>....D... | ........<br>........ | .......<br>....... | ........R....D.<br>............ | .H.Y............<br>..........R. | ......<br>.... |
| iPS:39 2844 | 21-225_23E11 | VK1\|A30/JK1 | ..........<br>......I.... | ........<br>........ | ........<br>........ | P......<br>....... | ............<br>............ | ............<br>............ | ......<br>.... |
| iPS:39 2846 | 21-225_24B6 | VK1\|A30/JK1 | ..........<br>.......... | ......D....<br>........ | ........<br>........ | .......<br>...H.. | ............<br>........T........ | ...Y............<br>..........R. | ......<br>..V. |
| iPS:39 2872 | 21-225_20B11 | VK1\|A30/JK1 | ..........<br>.......... | ........G..<br>........ | ......E.<br>........ | .......<br>....H. | ............<br>............ | ...Y............<br>..........R. | ......<br>.... |
| iPS:39 2876 | 21-225_21F7 | VK1\|A30/JK1 | ..........<br>.......... | ......D....<br>........ | ........<br>........ | .......<br>...NF.. | ............<br>............ | ....N...........<br>............ | ......<br>.... |
| iPS:39 2884 | 21-225_23A10 | VK1\|A30/JK1 | ..........<br>.......... | ........<br>........ | ........<br>........ | .......<br>....... | ............<br>....G........ | ...Y............<br>..........R. | ..L...<br>.V.. |
| iPS:39 2894 | 21-225_21G2 | VK1\|A30/JK1 | ..........<br>.......... | ........<br>........ | ........<br>........ | T......<br>.S..... | ............<br>............ | ................<br>............ | ..L...<br>.V.. |

| Seq ID | Clone | Germline | | | | | | | |
|---|---|---|---|---|---|---|---|---|---|
| iPS:39 2908 | 21-225_23F12 | VK1\|A30/JK1 | . . . . . . . . . . . | . . . . . . . . . . | . . . . . . . | V . . . . . . | . . . . . . . . . . . . . . | . . . . . . . . . . . . . . . . | . . . . . . |
| | | | . . . . . . . . . . | . . . . . . . | . . . . . . | . . . . . . . | . . . . . . . . . . . . S . . . | . . . . . . . . . . . . . . . . | . . . . |
| iPS:39 2914 | 21-225_25D12 | VK1\|A30/JK1 | . . . . . . . . . . | . T . . . . . . . | . . . . . . . | . . . . . . . | . . . . . . . . . . . . . . . | . . . Y . . . . . . . . . . . | . . . . . . |
| | | | . . . . . . . . . | . . . . . . . | . . . . . . | . . . . . . . | . . . . . . . . . . . . . . . | . . . . . . . . . . F . R . | . . . . |
| iPS:39 2918 | 21-225_28F5 | VK1\|A30/JK1 | . . . . . . . . . . | . . . . . . . . | . . . . . . . | T . . . . . . | . . . . . . . . . . . . . . | . . . . T . . . . . . . . . . | . . . . . . |
| | | | . . . . . . . . | . . . . . . . | . . . . . . | . . . . . . . | . . . . . . . . . . . . . . | . . . . . . . . . . . . . . | . . V . |
| iPS:39 2958 | 21-225_28C7 | VK1\|A30/JK1 | . . . . . . . . . . | . . . . . . . . | . . . . . . . | I . . . . . . | . . . . . . . . . . . . . . | . . . . T . . . . . . . . . . | . . . . . . |
| | | | . . . . . . . | . . . . . . . | . . . . . . | . . . . . . . | . . . . . . . . . . . . . . | . . . . . . . . . . . . . . | . . . . |
| iPS:39 2972 | 21-225_26A2 | VK1\|A30/JK1 | . . . . . . . . . . | . . . . . . . . | . . . . . . . | T . . . . . . | . . . . . . . . . . . . . . | . . . . R . . . . . . . . . . | . . . . . . |
| | | | . . . . . . . | . . . S . . . | . . . . . . | . . . . . . . | . . . F . . . . . . . . . . | . . . . . . . . . . . . . . | . . . . |
| iPS:39 3026 | 21-225_32B6 | VK1\|A30/JK1 | . . . . . . . . . . | . . . . . . . . | . . . . . . . | I . . . . . . | . . . . . . . . . . . . . . | . . . . . . . . . . . . . . | . . . . . . |
| | | | . . . . . . . . . | . . . . . . . | . . . . . . | . . . . . . . | . . . . . . . . . . . . . . | . . . . . . . . . . . . . . | . . . . |
| iPS:39 3130 | 21-225_33C2 | VK1\|A30/JK1 | . . . . . . . . . . | . . . . . . . . | . . . . . . V . | . . . . . . . | . . . . . . . . . . . . . . | . . . . . . . . . . . . . . | . . . . . . |
| | | | . . . . . . . . . | . . . . . . . | . . . . . . | . . P . . . . | . . . . . . . . . . H . . . . . | . . . . . . . . . . . . . . | . . . . |
| iPS:39 3812 | 21-225_6A11 | VK1\|A30/JK1 | . . . . . . . . . . . | . . . . . . . . . . | . . . . . . . | . . . . . . . | . . . . . . . . . . . . . . | . . . . . . . . . . . . . . . . | . . . . |
| | | | . . . . . . . . . | . . . . . . . | . . . . . . | . . . . . . . | . . . . . . . . . . . . . . | . . . . . . . . . . . . . . | . . . . |
| iPS:39 3838 | 21-225_6G2 | VK1\|A30/JK1 | . . . . . . . . . R . | . . . . . . . . | . . . . . . . | . . . . . . . | . . . . . . . . . Y . . . . . | I . . . . . . . . . . . . . . . | . . . . . |
| | | | . F . . . . . . . Y | . . . . . . R . . | . . Q . . . . | . . . . . . . | N I . . . . . . . . . . I . . . | . . . . . . . . . . . . L . . | . . . T |
| iPS:39 3864 | 21-225_4C5 | VK1\|A30/JK1 | . . . . . . . . . . | . . . . . R . . . | . . R . . . . | . . . . . . . | . . . . . . . . . Y . . . . . | . . . Y . . . . . . . . . . . | . . . . . . |
| | | | . . . H . . H L . . | . . . . G . . . | . . . . . . | . . . N . . . | . . . . G . . . . . . . . . . . | . . . . . . . . . . . R . | . . . . |
| iPS:39 3868 | 21-225_9C11 | VK1\|A30/JK1 | . . . . . . . . . . | . . . . . . N . . . | . . . . . S . R | V . . . . . . | . . . . . . . . . . . . . . | H . S . . . . . . . . . . . . . | . . . . . . |
| | | | . . . . . . . . . | . . . . . Y . N | . . . L . . . | . . . . . . . | . . . . N . . . . . . . . I . H . | . . . . . . . . . . T . L . | . . . . |
| iPS:39 3876 | 21-225_9A1 | VK1\|A30/JK1 | . . . . . . . . . . R . | . . . . . . . . . . | . . . . . . . | T . . . . . . | . . . . . . . . . Y . . . . . | I . . . . . . . . . . . . . . . | . . . . . . |
| | | | . F . . . . . . . | . . . . . . . | . R . . . . | . . . . . . . | . I . . . . . . . . . . . . . | . . . . . . . . . . . . L . . | . . . . |
| iPS:39 3902 | 21-225_14E10 | VK1\|A30/JK1 | . . . . . . . . . . | . T . . . . . . . | . . . H . . . Q | . . . . . . . | . . . . . . . . . . . . . . | . . . Y . . . . . . . . . . . | . . . . . . |
| | | | . . . . . . . . . . | . . . . . . . | . . . . . . | . . . . . . . | . . . . . . . . . . . T A . . . | . . . . . . . . . . . R . | . . . . |
| iPS:39 3908 | 21-225_10E9 | VK1\|A30/JK1 | . . . . . . . . . . | . . . . . . D . . . | . . . . . . . | . . . . . . . | . . . . . . . . . . . . . . | . . . Y . . . . . . . . . . . | . . . . . . |
| | | | . . . . . . . F . . | . . . . S . . . | . . T . . . F | . . . . . . . | . . . N . . . . . . . . . . . . | . . . . . . . . . . . R . | . . . . |
| iPS:39 3916 | 21-225_2G4 | VK1\|A30/JK1 | . . . . . . . . . . . | . . . . . . . . . . | . . . . . . . | . . . . . . . | . . . . . . . . . . . . . . | . . . Y . . . . . . . . . . . | . . R . . . |
| | | | . . . . . . . . . | . . . . . . . | . . T . . . . | . . . . H . | . . . . . . . . . L . . . . . | . . . . . . . . . . F . R . | . . . . |
| iPS:39 3948 | 21-225_16A5 | VK1\|A30/JK1 | . . . . . . . . . . . | . . . . . . . . . . | C . . . . . . | . . . . . . . | . . . . . . N . Y . . . . . | . . . . . . . . . . . . . . . . | . . . . |
| | | | . . . . . . . . . | . . . . . . . | . . . . . . | . . . . . . . | . . . . . . . . . . . . . . | . . . . . . . . . . . . . . | . . . . |
| iPS:39 3960 | 21-225_7G2 | VK1\|A30/JK1 | . . . . . . . . . . . | . . . . . . . . . . | . . . . . . . | T . . . . . . | . . . . . . . . . . . . . . | . . . . . . . . . . . . . . . . | . . L . . . |
| | | | . . . . . . . . . | . . . . . . . | . . . . . . | . . . . . . . | . . . . . . . . . . . . . . | . . . . . . . . . . . . . . | . V . . |
| iPS:39 3966 | 21-225_7F8 | VK1\|A30/JK1 | . . . . . . . . . . . | . . . . . . G . . | . . . . . . . | . . . . . . . | . . . . . . . . V . . . . . | . . . Y T . . . . . . . . . . | . . . . . . |
| | | | . . . . . . . . . | . . . . . . . | . . . . . . | . . . . . . . | . . P . . . . . . . . . . . | . . . . . . . . . . . R . | . . . . |
| iPS:39 3972 | 21-225_7C9 | VK1\|A30/JK1 | . . . . . . . . . . | . . . . . . . . | . . . . . . . | . . . . . . . | . . . . . . . . . . . . . . | . . L Y . . . . . . . . . . . | . . . . . . |
| | | | . . G . . . . . . | . . . . . . . | . . . . . . | . . . . . . . | . . . . . . . . . . . . . . | . . . . . . . . . . . R . | . D . . |
| iPS:39 3978 | 21-225_4C12 | VK1\|A30/JK1 | . . . . . . . . . . | . . . . . . . . | . . . . . . . | . . . . . . . | . . . . . . . . . . . . . . | . . . Y . . . . . . . . . . . | . . . . . . |
| | | | . . . . . . . . . | . . . . . . . | . . T . . . | . . . . H . | . . . . . . . . . L . . . | . . . . . . . . . . F . R . | . . . . |
| iPS:39 3986 | 21-225_7G4 | VK1\|A30/JK1 | . . . . . . . . . . | . . . . . . . . | . . . . R . . . | . . . . . . . | . . . . . . . . . . . . . . | . H Q Y . . . . . . . . . . . | . . . . . . |
| | | | . . . . . . . . . | . . . . . . . | . . . . . . | . . . . . . . | . . . . . . . . . . S . . . . | . . . . . . . . . . R . | . . . . |

| iPS ID | Clone | VK/JK | K_FR1 | K_CDR1 | K_FR2 | K_CDR2 | K_FR3 | K_CDR3 | K_FR4 |
|---|---|---|---|---|---|---|---|---|---|
| iPS:39 3996 | 21-225_15C11 | VK1\|A30/ JK1 | ............. / ............ | ........... / ....... | ,....... / V...... | ........ / ........ | ............... / ............... | .L.Y............. / ............R. | ..R... / .... |
| iPS:39 3998 | 21-225_12B12 | VK1\|A30/ JK1 | .......... / .......... | .......... / ........ | ........ / ........ | T...... / ...... | ........... / .............. | ............... / ............... | ..L... / .V.. |
| iPS:39 4041 | 21-225_5E5 | VK1\|A30/ JK1 | ........... / ........ | ........... / ........ | ........ / ........ | ........ / ........ | ............. / ............. | ...Y............. / ...........R. | ...... / ..V. |
| iPS:39 4067 | 21-225_12F2 | VK1\|A30/ JK1 | ............. / ............ | ........... / ........ | ........ / ........ | ........ / ........ | ...........V.... / ............. | ............... / ............... | .... / .... |
| iPS:39 4089 | 21-225_12E6 | VK1\|A30/ JK1 | ............. / ............ | ....S... / ........ | ........ / ........ | ........ / ........ | ............. / ............. | ............... / ............... | ...... / .... |
| iPS:39 4093 | 21-225_9D12 | VK1\|A30/ JK1 | ............. / ............ | ........... / ........ | ........ / ........ | ........ / ........ | ............. / ............. | ............... / ............... | ...... / .... |
| iPS:39 4095 | 21-225_16H4 | VK1\|A30/ JK1 | ............. / ............ | ........... / ........ | ........ / ........ | T...... / ...... | ............. / ............. | ............... / ............... | .... / .... |
| iPS:39 4097 | 21-225_16G7 | VK1\|A30/ JK1 | ............. / ....... | ........... / ....... | ........ / ....... | ........ / ....... | ............. / ............. | ............... / ............... | .... / .... |
| Germline | | | K_FR1 | K_CDR1 | K_FR2 | K_CDR2 | K_FR3 | K_CDR3 | K_FR4 |
| | VK3\|L2/JK2 | | EIVMTQSPATLS VSPGERATLSC | RAS QSVS ----SNLA | WYQQKPGQ APRLLIY | G------ -ASTRAT | GIPARFSGSGSG-- TEFTLTISSLQSEDFAV YYC | QQYNN------------ ------WPYT | FGQGTK LEIK |
| iPS:46 8828 | 21-225_162A10 | VK3\|L2/J K2 | ............. / ............ | .......T.N.. / ....... | .....S.. / .....F | ........ / ...... | V....IN......... / ......R.......F. | ....D............ / ...........CS | ...... / .... |
| iPS:43 4255 | 21-225_62E6 | VK3\|L2/J K2 | ............. / ............ | .......N.. / ........ | ........ / .....S | V...... / ...... | ......N........ / ............... | ...D............. / ...........CS | ...... / .... |
| iPS:43 4269 | 21-225_57H3 | VK3\|L2/J K2 | ............. / ........... | ........... / .....S.. | ........ / ....... | ........ / ....... | .F....N....W.... / ..........I... | ....D............ / ...........CS | ...... / .... |
| iPS:43 4363 | 21-225_65A6 | VK3\|L2/J K2 | ........V..F / .......... | ........N.. / ........ | ........ / ....... | ........ / ....... | ......N........ / ............... | ...D............. / ...........CS | ..LE.. / .... |
| iPS:43 4393 | 21-225_67C3 | VK3\|L2/J K2 | ............. / ............ | ........N.. / ........ | ........ / .....S | I...... / ...... | ......N........ / ............... | ...D............. / ...........CS | ...... / .... |
| iPS:43 4425 | 21-225_70A5 | VK3\|L2/J K2 | ............. / ............ | ........N.. / ........ | ...L.... / .....S | I...... / ...... | ...P..N........ / ............... | ....D............ / ...........CS | .... / .... |
| iPS:43 4485 | 21-225_76D2 | VK3\|L2/J K2 | ..........P. / ........... | .....V..V.. / ...NS.. | ........ / .....H | ........ / ...... | ............... / ......V......I... | ...D............. / ...........CS | .... / .... |
| iPS:43 4537 | 21-225_74E11 | VK3\|L2/J K2 | ............. / ............ | ....L..V.. / ...NS.. | ........ / .....H | ........ / ...... | ............... / ............I... | ....D............ / ...........CS | ...... / .... |
| iPS:43 4569 | 21-225_77H5 | VK3\|L2/J K2 | .......V... / .......... | ........... / .....S.. | .......L / ....... | ........ / ...... | SF.............F. / ............... | ...D............. / ...........CS | ....S. / ...Q |
| iPS:43 4629 | 21-225_74C3 | VK3\|L2/J K2 | ............. / .......... | .......A.. / ...S.. | ........ / .....F | .T..... / ...... | ..I............ / ............... | ...D............. / ...........CS | ..L... / .... |
| iPS:43 4673 | 21-225_74E3 | VK3\|L2/J K2 | ............. / L.......... | ....L..V.. / ...NS.. | ........ / ....... | ........ / ....... | ............... / ..........I... | ...D............. / ...........CS | .... / .... |

| Clone | Name | Gene | K_FR1 | K_CDR1 | K_FR2 | K_CDR2 | K_FR3 | K_CDR3 | K_FR4 |
|---|---|---|---|---|---|---|---|---|---|
| iPS:43 5109 | 21-225_92H5 | VK3\|L2\|J K2 | ............ | ......C.I.. | ........ | ........ | .V.............. | .E..D........... | ..... |
| | | | ............ | ....TY.. | ....... | ....... | ....T........L... | ............CS | .... |
| iPS:43 5221 | 21-225_95G2 | VK3\|L2\|J K2 | ............ | .....M..V.. | ........ | ........ | ................ | ....D........... | ..... |
| | | | L........... | ....NS.. | ....... | ....... | ............I... | ............CS | .... |
| iPS:43 6051 | 21-225_193G12 | VK3\|L2\|J K2 | ............ | ........... | ........ | ........ | ................ | ................ | ..... |
| | | | ............ | .....S.. | ..I..S | ....... | ..........A..... | ............CS | .... |
| iPS:43 6236 | 21-225_201F7 | VK3\|L2\|J K2 | ............ | ......NIK.. | ........ | ........ | ................ | ..FY............ | ..... |
| | | | ............ | ....N... | ......F | ....... | ................ | ..........LCS | ..L. |
| iPS:43 6250 | 21-225_201A4 | VK3\|L2\|J K2 | ............ | .S....NIK.. | ........ | ........ | ................ | ..FY............ | ..... |
| | | | ....S..... | ........ | ......F | ....... | ................ | ..........LCS | ..L. |
| iPS:43 6252 | 21-225_202A8 | VK3\|L2\|J K2 | ............ | ......RIN.. | ....N... | ........ | .V.............. | ...Y............ | ..... |
| | | | ............ | ....N... | ....... | ....... | ..............T.... | ..........LCS | ...R |
| iPS:43 6258 | 21-225_202F12 | VK3\|L2\|J K2 | ............ | ........L.. | ....R... | ........ | ................ | ...D............ | ..... |
| | | | ............ | ....N... | ....... | ....... | ................ | ........WP.CS | .... |
| iPS:43 6278 | 21-225_201F2 | VK3\|L2\|J K2 | ............ | ......NIK.. | ........ | ........ | ................ | ..FY............ | ..... |
| | | | ............ | ........ | ......F | ....... | ................ | ..........LCS | ..L. |
| iPS:43 6294 | 21-225_205G4 | VK3\|L2\|J K2 | ............ | ......NIK.. | ........ | ........ | ................ | ..FY............ | ..... |
| | | | ............ | ........ | ......F | ....... | ................ | ..........LCS | ..L. |
| iPS:43 6306 | 21-225_201H4 | VK3\|L2\|J K2 | ............ | ........N.. | ........ | ........ | ................ | .E..D........... | .....N |
| | | | ............ | ....Y.. | ....... | ....... | ................ | ............CS | .... |
| iPS:43 6356 | 21-225_210H10 | VK3\|L2\|J K2 | ............ | ........K.. | ........ | ........ | ................ | ...Y............ | ..... |
| | | | ............ | ........ | ....... | ....... | ..............S. | ..........LCS | .... |
| iPS:43 6382 | 21-225_212C10 | VK3\|L2\|J K2 | ............ | ........A.. | ........ | ........ | DV......F.....SD. | ....D........... | ..... |
| | | | ............ | .....S.. | ......H | .T..... | ..............S. | ............CS | .... |
| iPS:43 6434 | 21-225_216B10 | VK3\|L2\|J K2 | .....E...... | ........N.. | ..R..... | ........ | ...P............ | ....D........... | ..... |
| | | | ............ | ....N... | ....... | ....... | ..S............. | ............CS | ...R |
| iPS:39 2806 | 21-225_24H3 | VK3\|L2\|J K2 | ............ | ........... | ........ | F...... | ................ | ................ | ..... |
| | | | ............ | ........ | ....... | ...I... | ................ | .........WPMCS | .... |

| Germline | | | K_FR1 | K_CDR1 | K_FR2 | K_CDR2 | K_FR3 | K_CDR3 | K_FR4 |
|---|---|---|---|---|---|---|---|---|---|
| VK3\|L2\|JK4 | | | EIVMTQSPATLS VSPGERATLSC | RAS QSVS ----SNLA | WYQQKPGQ APRLLIY | G -ASTRAT | GIPARFSGSGSG TEFTLTISSLQSEDFAV YYC | QQYNN ----------WPLT | FGGGTK VEIK |
| iPS:46 8830 | 21-225_191G11 | VK3\|L2\|J K4 | ............ | .T......W.. | ..H..... | ........ | ................ | ....Y............ | ..... |
| | | | .......N... | ....ISV. | ....... | ..A.... | ................ | ................ | .... |
| iPS:43 4147 | 21-225_55E1 | VK3\|L2\|J K4 | ............ | ........... | ...L.... | D....... | ................ | ...Y............ | ..... |
| | | | ............ | .....D.. | ....... | ...A... | ..............F... | ................ | .... |
| iPS:43 4621 | 21-225_74D1 | VK3\|L2\|J K4 | ............ | ........... | .F...... | ........ | ................ | ................ | ..... |
| | | | ............ | ....R... | ....... | ...I... | ....Y........... | .........WP... | .... |
| iPS:43 5821 | 21-225_190E11 | VK3\|L2\|J K4 | ............ | ......FR.. | ....R... | ........ | ................ | ................ | ..... |
| | | | ............ | ....I... | ....... | ....... | ................ | ................ | .... |
| iPS:43 5941 | 21-225_191E8 | VK3\|L2\|J K4 | ............ | .P.....F... | ........ | ........ | ...S............ | ................ | ....I. |
| | | | ............ | ....R... | ....... | ....... | ......E........ | ................ | .... |

| ID | Clone | VK | K_FR1 | K_CDR1 | K_FR2 | K_CDR2 | K_FR3 | K_CDR3 | K_FR4 |
|---|---|---|---|---|---|---|---|---|---|
| iPS:43 6203 | 21-225_199A6 | VK3\|L2/JK4 | D...........<br>....D...... | .P.....F...<br>....R... | ........<br>....... | ........<br>....... | ...............<br>.......EC....... | .................<br>................ | ....<br>.... |
| iPS:43 7334 | 21-225_75F11 | VK3\|L2/JK4 | ...........F<br>........... | ...........<br>....R... | .F.....<br>.....F. | ........<br>...I... | ...............<br>....Y...Y....... | .................<br>.........WP... | ....<br>.... |
| iPS:44 8904 | 21-225_65C12 | VK3\|L2/JK4 | ...........<br>.F...G..... | ...........<br>....I... | ........<br>....... | ........<br>....... | .......NA......<br>..S.......N... | ....T............<br>................ | ....<br>.... |
| | Germline | | K_FR1 | K_CDR1 | K_FR2 | K_CDR2 | K_FR3 | K_CDR3 | K_FR4 |
| | VK1\|A30/JK3 | | DIQMTQSPSSLS<br>ASVGDRVTITC | RAS--QGIR--<br>----NDLG | WYQQKPGK<br>APKRLIY | A------<br>-ASSLQS | GVPSRFSGSGSG--<br>TEFTLTISSLQPEDFAT<br>YYC | LQHNS-----------<br>-----------YPFT | FGPGTK<br>VDIK |
| iPS:46 8832 | 21-225_76H10 | VK1\|A30/JK3 | ............<br>..A........ | ......D....<br>.....Y.. | ........<br>....... | G......<br>....... | .................<br>.............F.. | ..Y..............<br>................ | ....<br>.... |
| iPS:46 8836 | 21-225_198E3 | VK1\|A30/JK3 | ............<br>........... | ...........<br>....K... | ........<br>....... | .......<br>....... | .................<br>..............V. | ...YR...........<br>................ | ......<br>..F. |
| iPS:46 8844 | 21-225_48E10 | VK1\|A30/JK3 | ............<br>........... | ...........<br>......S... | ........<br>....... | T......<br>....... | .................<br>................ | ..Y.............<br>................ | ......<br>.... |
| iPS:46 8846 | 21-225_53B10 | VK1\|A30/JK3 | ............<br>..A........ | ......D....<br>.....Y.. | ........<br>....... | G......<br>....... | .................<br>.............F.. | ..Y..............<br>................ | ....<br>.... |
| iPS:43 3895 | 21-225_43E1 | VK1\|A30/JK3 | ............<br>........... | ...........<br>........ | ...K....<br>......N | .......<br>....... | .................<br>................ | .................<br>................ | ....<br>.... |
| iPS:43 3905 | 21-225_43E5 | VK1\|A30/JK3 | ............<br>........... | ...........<br>........ | ........<br>....... | G......<br>...N... | .................<br>...V..........F. | ...T............<br>..........F... | ....<br>.... |
| iPS:43 3913 | 21-225_43H8 | VK1\|A30/JK3 | ............<br>........... | ...........<br>........ | .H......<br>....... | .......<br>....... | .................<br>...S............ | .................<br>..........F... | ....<br>.... |
| iPS:43 3933 | 21-225_44C8 | VK1\|A30/JK3 | ............<br>........... | ...........<br>........ | ........<br>....... | .......<br>...N... | .........R......<br>................ | .................<br>................ | ....<br>.... |
| iPS:43 3949 | 21-225_45H2 | VK1\|A30/JK3 | ............<br>........... | ...........<br>........ | ........<br>....... | G......<br>...N... | .................<br>...V..........F. | ...T............<br>..........F... | ....<br>.... |
| iPS:43 3981 | 21-225_46E9 | VK1\|A30/JK3 | ............<br>........... | ...........<br>........ | ........<br>....... | .......<br>....... | .................<br>...V..........F. | ...T............<br>..........F... | ....<br>.... |
| iPS:43 3995 | 21-225_47H7 | VK1\|A30/JK3 | ............<br>........... | .T.........<br>........ | ...K....<br>....... | .......<br>....... | .................<br>...V..........F. | ...T............<br>..........F... | ....<br>.... |
| iPS:43 4039 | 21-225_43B1 | VK1\|A30/JK3 | ............<br>........... | ...........<br>........ | ........<br>....... | .......<br>....... | .................<br>...V..........F. | ...T............<br>..........F... | ....<br>.... |
| iPS:43 4057 | 21-225_51E4 | VK1\|A30/JK3 | ............<br>........... | ...........<br>........ | ..Q....<br>....... | .......<br>....... | .................<br>............A... | .................<br>................ | ....<br>.... |
| iPS:43 4071 | 21-225_51F9 | VK1\|A30/JK3 | ............<br>........... | ...........<br>....T... | ......R.<br>..Q.... | .......<br>......R | ................D.<br>.............S... | ................<br>................ | ....<br>.... |
| iPS:43 4075 | 21-225_51B11 | VK1\|A30/JK3 | A...........<br>........... | .T.........<br>........ | ......R.<br>....... | .......<br>....... | .................<br>................ | .................<br>................ | ....<br>.... |
| iPS:43 4091 | 21-225_52B9 | VK1\|A30/JK3 | A...........<br>........... | ...........<br>........ | ......R.<br>....... | .......<br>....... | ...L.............<br>....R........... | .................<br>................ | ....<br>.... |

| iPS:43 | Clone | Germline | | | | | | |
|---|---|---|---|---|---|---|---|---|
| 4101 | 21-225_52H12 | VK1\|A30/JK3 | . . . . . . . . . . .<br>. . . . . . . . . | . . . . . . . . . . .<br>. . . . . N . . | . . . . . . . .<br>. . . . . . . | G . . . . . . .<br>. . . . . . . | . . . . . . . . . . . . . . . . .<br>. . . . . . . . . . . . . . . . . | . . Y . . . . . . . . . . . . . . .<br>. . . . . . . . . . . . . . | . . . . . . .<br>. . . . . |
| 4103 | 21-225_53G1 | VK1\|A30/JK3 | . . . . . . . . . . .<br>. . . . . . . . . | . . . . . . . . . .<br>. . . . . . . | . . . . . . . .<br>. . . . . . . | P . . . . . . .<br>. . . . . . . | . . . . . . . . . . . . . . . . .<br>S . . . . . . . . . . . . . . . . | . . D . . . . . . . . . . . . . .<br>. . . . . . . . . . . . . . | . . . . . . .<br>. . . . |
| 4129 | 21-225_53B12 | VK1\|A30/JK3 | . . . . . . . . . . .<br>. . . . . . . . . | . . . . . . . . . .<br>. . . . . . . | . . . . . . . .<br>. . . . . . . | . . . . . . . .<br>. . . . . . . | . . . . . . . . . . . . . . . . .<br>. . . . . . . . . . . . . . . . . | . . . . . . . . . . . . . . . . .<br>. . . . . . . . . . . . . . | . . . . . . .<br>. . . . |
| 4131 | 21-225_54D3 | VK1\|A30/JK3 | . . . . . . . . . . .<br>. . . . . . . . . | . . . . . . . . . .<br>. . . . . . . | . . . . . . . .<br>. . . . . . . | . . . . . . . .<br>. . . . . . . | . . . . . . . . . . . . . . . . .<br>. . . . . . . . . . . . . . . . . | . . . . . . . . . . . . . . . . .<br>. . . . . . . . . . . . . . | . . . . . . .<br>. . . . |
| 4143 | 21-225_54G7 | VK1\|A30/JK3 | . . . . . . . . . . .<br>. . . . . . . . . | . . . . . . . . . .<br>. . . . . . . | . . . . . . . .<br>. . . . . . . | . . . . . . . .<br>. . . . . . . | . . . . . . . . . . . . . . . . .<br>. . . . . . . . . . . . . . . . . | . H . . T . . . . . . . . . . . . .<br>. . . . . . . . . . . . . . | . . . . . . .<br>. . . . |
| 4155 | 21-225_55B3 | VK1\|A30/JK3 | . . . . . . . . . . .<br>. . . . . . . . . | . . . . . . . . . .<br>. . . . . . . | . . . . . . . .<br>. . . . . . . | . . . . . . . .<br>. . . . . . . | . . . . . . . . . R . . . . . . .<br>. . . . . . . . . . . S . . . . | . . . . . . . . . . . . . . . . .<br>. . . . . . . . . . . . . . | . . . . . . .<br>. . . . |
| 4169 | 21-225_50C4 | VK1\|A30/JK3 | . . . . . . . . . . .<br>. . . . . . . L . . | . . . . . . . . . .<br>. . . . . . . | . . . . . . . I<br>. . . . . . . | . . . . . . . .<br>. . . . . . . | . . . . . . . . . . . . . . . . .<br>. . . . . . . . . . . . . . . . . | . . . . R . . . . . . . . . . . . .<br>. . . . . . . . . . . . . . | . . . . .<br>. . . . |
| 4187 | 21-225_56A5 | VK1\|A30/JK3 | . . . . . . . . . . .<br>. . . . . . . . . | . . . . . . D . . .<br>. . . . . L . . | . . . . . . . .<br>. . . . . . . | . . . . . . . .<br>. . . . . . . | . . . . . . . . . . . . . . . . .<br>. . . . . . . . . . . . . . . . . | . . Y . . . . . . . . . . . . . . .<br>. . . . . . . . . . . . . . | . . . . . . .<br>. . . R |
| 4199 | 21-225_59F11 | VK1\|A30/JK3 | . . . . . . . . . . .<br>. . . . . . . . . | . . . . . . . . . .<br>. . . . . . . | . . . . . . . .<br>. . . . . . . | . . . . . . . .<br>. . . . . . . | . . . . . . . . . . . . . . . . .<br>. . . . . . . . . . . . . . . . . | . . . . R . . . . . . . . . . . . .<br>. . . . . . . . . . . . . . | . . . . . . .<br>. . F . |
| 4207 | 21-225_60A3 | VK1\|A30/JK3 | . . . . . . . . . . .<br>. . . . . . . . . | . . . . . . . . . .<br>. . . . . . . | . . . . . . . .<br>. . . . . . . | . . . . . . . .<br>. . F . . . . | . . . . . . . . . . . . . . . . .<br>. . . . . . . . . . . . . . . . . | . . . . . . . . . . . . . . . . .<br>. . . . . . . . . . . . . . | . . . . . . .<br>. . . . |
| 4251 | 21-225_62G3 | VK1\|A30/JK3 | . . H . . . . . . .<br>. . . . . . L . . | . . . . . . D . . . .<br>. . . . . N . . | . . . . . . . .<br>. . . . . . . | T . . . . . . .<br>. . . . . . . | . . . . . . . . . . . . . . . . .<br>. . . . . . . . . . . . . . . . . | . . Y . . . . . . . . . . . . . . .<br>. . . . . . . . . . . . . . | . . . . . . .<br>. . . . |
| 4263 | 21-225_56H7 | VK1\|A30/JK3 | . . . L . . . . . .<br>. . . . . . . . . | . . . . . . D . . . .<br>. . . . . . . | . . . . R . . .<br>. . . . . . . | P . . . . . . .<br>. . . . L . | . . . . . . . . . . . . . . . . .<br>. . . . . . . . . . . . . . . . . | . . D . . . . . . . . . . . . . . .<br>. . . . . . . . . . . . . . | . . . . . . .<br>. . S . |
| 4265 | 21-225_57B2 | VK1\|A30/JK3 | . . . . . . . . . . .<br>V . . . . . . . . . | . . . . . . . . . .<br>. . . . . A . . | . . . . . . . .<br>. . . . . . . | . . . . . . . .<br>. . . . . . . | . . . . . . . . . . . . . . . . .<br>. . . . . . . . . . . . . . . . . | . . . . . . . . . . . . . . . . .<br>. . . . . . . . . . . . . . | . . . . . . .<br>. . . . |
| 4271 | 21-225_57A4 | VK1\|A30/JK3 | . . . . . . . . . . .<br>. . . . . . . . . | . . . . . . . . . .<br>. . . . . . . | . . L . . . . .<br>. . . . . . . | . . . . . . . .<br>. . . . L . | . . . . . . . . . . . . . . . . .<br>. . . . . . . . . . . . . . . . . | . . . . . . . . . . . . . . . . .<br>. . . . . . . . . . . . . . | . . . . . . .<br>. . . . |
| 4275 | 21-225_57F4 | VK1\|A30/JK3 | . . . . . . . . . . .<br>. . . . . . . . . | . . . . . . V . . . .<br>. . . . . . . | . . . . . . . .<br>. . . . . . . | . . . . . . . .<br>. . . . . . . | . . . . . . . . . . . . . . . . .<br>. . . . . . . . . . H . | . . Y G . . . . . . . . . . . . . .<br>. . . . . . . . . . . F . . . | . . . . . . .<br>. . . . |
| 4293 | 21-225_58F5 | VK1\|A30/JK3 | . . . . . . . . . . .<br>. . . . . . . . . | . . . . . . . . . .<br>. . . . . . . | . F L . T . . .<br>. . . . . . . | . . . . . . . .<br>. . . . L . | . . . . . G . . . . . . . . . . .<br>. . . . . . . . . . . . . . . . . | . . . . . . . . . . . . . . . . .<br>. . . . . . . . . . . . . . | . . . . . . .<br>. E . . |
| 4299 | 21-225_58D11 | VK1\|A30/JK3 | . . . . . . . . . . .<br>. . . . . . . . . | . . . . . . . . . .<br>. . . . S . . D | . . . . . . . .<br>. . . . . . . | . . . . . . . .<br>. . . . . . . | . . . . . . . . . . . . . . . . .<br>. . A . . . . R . . . . . . . . . | . . . . N . . . . . . . . . . . . .<br>. . . . . . . . . F . . . | . . . . |
| 4351 | 21-225_64A12 | VK1\|A30/JK3 | . . . . . . . . . . .<br>. . . . . . . . . | . . . . . . . . . .<br>. . . . . . . | . . . . . . . .<br>. . T . . . . | T . . . . . . .<br>. . . T . . . | . . . . . . . . . . . . . . . . .<br>. . . . . . . . . . . . . . . . . | . . . . G . . . . . . . . . . . . .<br>. . . . . . . . . . . . . . | . . . . . . .<br>. . . . |
| 4383 | 21-225_66F9 | VK1\|A30/JK3 | . . . . . . . . . . .<br>. . . . . . . . . | . . . . . . D . . . .<br>. . . . V . . | . . . . . . . .<br>. . . . . . . | T . . . . . . .<br>. . . . . . . | . . . . G . . . . . . . . . . . .<br>. . . . . . . . . . . . . . . . . | . . Y . . . . . . . . . . . . . . .<br>. . . . . . . . . . . . . . | . . . . . . .<br>. . . . |
| 4399 | 21-225_67B7 | VK1\|A30/JK3 | . . . . . . . . . . .<br>. . . . . . . . . | . . . . . . . . . .<br>. . . . . . . | . F . . . . . .<br>. . . . . . . | . . . . . . . .<br>. . . . . . . | . . . . . . . . . . . . . . . . .<br>S . . . . . . . . . . . . . . . . | . H . . . . . . . . . . . . . . . .<br>. . . . . . . . . . . . . . K | . . . . . . .<br>. . . . |
| 4407 | 21-225_68G8 | VK1\|A30/JK3 | . . . . . . . . . . .<br>. . . . . . . . . | . . . . . . . . . .<br>. . . . . N . . | . . . . R . . .<br>. . . . . . . | . . . . . . . .<br>. . . . V . . | . . . . . . . . . . . . . . . . .<br>. . . . . . . . . . . . . . . . . | . . Y . . . . . . . . . . . . . . .<br>. . . . . . . . . . . . . . | . . . . . . .<br>. . . . |

| ID | Clone | Gene | Seq 1 | Seq 2 | Seq 3 | Seq 4 | Seq 5 | Seq 6 | Seq 7 |
|---|---|---|---|---|---|---|---|---|---|
| iPS:43 4447 | 21-225_71B6 | VK1\|A30/ JK3 | . . . . . . . . . . .P. / . . . . . . . . . . . | . . . . . . . . . . . / . . . . . . .D | . . . . . . . . / . .Q. . . . | . . . . . . . . / . . . . . . . | . . . . . . . . . . . . . . . . . . / . . . . . . . . .D. . . . . | . . . . . . . . . . . . . . . . . . . / . . . . . . . . . . . . . . | . . . . . . / . . . . . |
| iPS:43 4449 | 21-225_71H6 | VK1\|A30/ JK3 | . . . . . . . . . . . / . . . . . . . . . . . | . . . . . . . . . . . / . . . . .V. . | . . . . . . . . / . . . . . . . | . . . . . . . . / . . . . . . . | . . . . . . . . . . . . . . . . . . / . . . . . . . . . . . . . . . . . | . .Y. . . . . . . . . . . . . . / . . . . . . . . . . . . . . . . . . . | . . . . . . / . . . . |
| iPS:43 4453 | 21- 225_71B11 | VK1\|A30/ JK3 | . . . . . . . . . . . / . . . . . . . . . . . | . . . . . . . . . . . / . . . . . .D | . . . .T. . . / . . . . . . . | . . . . . . . . / . . . . . . . | . . . . . . . . . . .G. . . . . . / S. . . . . . . . . . . .T. . . . | . . . .T. . . . . . . . . . . . . / . . . . . . . . . . . . . . . . . | . . . . . . / . .V. |
| iPS:43 4463 | 21-225_73A6 | VK1\|A30/ JK3 | . . . . . . . . . . . / . . . . . . . . . . . | . . . . . . . . . . . / . . . . . . . | .F. . . . . / . . . . . . . | . . . . . . . . / . . . .N. . . | . . . . . . . . . . . . . . . . . . / . . . . . . . . . . . . . . . . . | . . . . . . . . . . . . . . . . . . / . . . . . . . . . . . . . . . . . | . . . . . . / . . . . |
| iPS:43 4815 | 21- 225_74A11 | VK1\|A30/ JK3 | . . . . . . . . . . . / . . . . . . . . . . . | . . . . . . .D. . . . / . . . . . . . | . . . . . . . . / . . . . . .C | . . . . . . . . / . . . . . . . | . . . . . . . . . . . . . . . . . . / . . . . . . . . . . . . . . . .F. | . . . .D. . . . . . . . . . . . / . . . . . . . . . . . . . . . . . | . . . . . . / . . . . |
| iPS:43 4977 | 21-225_88A5 | VK1\|A30/ JK3 | . . . . . . . . . . . / . . . . . . . . . . . | . . . . . . . . . . . / . . . . . . . | . . . . . . . . / . . . . . . . | . . . . . . . . / . . . . . . . | . . . . . . . . . . . . . . . . . . / . . . . . . . . . . . . . . . . . | . . . .D. . . . . . . . . . . . / . . . . . . . . . . . . . . . . . | . . . . . . / .E. . |
| iPS:43 5253 | 21-225_96A4 | VK1\|A30/ JK3 | . . . . . . . . . . . / . . . . . . . . . . . | . . . . . . .D. . / . . . . . . . | . . . . . . . . / . . . . . . . | G. . . . . . / .V. . . . . | . . . . . . . . . . . . . . . . . . / . . . . . . .R. . . . . . . | . . . .D. . . . . . . . . . . . / . . . . . . . . . . . . . . . . . | . .R. . . / . . . . |
| iPS:43 5511 | 21- 225_157C3 | VK1\|A30/ JK3 | . . . . . . . . . . . / . . . . . . . . . . . | . . . . . . . . . . . / . . . . . . . | . . . . . . . . / . . . . . . . | . . . . . . . / . . . . . . . | . . . . . . . . . . . . . . . . .D. / . . . . . . . . . .D. . . . . . | . . . . . . . . . . . . . . . . . . / . . . . . . . . . . . . . . . . . | . . . . |
| iPS:43 5521 | 21- 225_157H4 | VK1\|A30/ JK3 | . . . . . . . . . . . / . . . . . . . . . . . | . . . . . . . . . / . . . . . . . | . . . . . . . . / . . . . . . . | P. . . . . . / . . . . . .T | . . . . . . . . . . . . . . . . . . / . . . . . . . . . . . . . . . . . | . .D. . . . . . . . . . . . . . . / . . . . . . . . . . . . . . . . . | . . . . |
| iPS:43 5527 | 21- 225_157G7 | VK1\|A30/ JK3 | . . . . . . . . . .C / . . . . . . . . . . . | . . . . . . . . . / . . . . . . . | . . . . . . . . / . . . . .N | V. . . . . . / . . . . . . . | . . . . . . . . . . . . . . . . . . / . . . . . .V.R. . . . . . . | I.D. . . . . . . . . . . . . / . . . . . . . . .H. . . | . . . . . . / .E. . |
| iPS:43 5533 | 21- 225_157H8 | VK1\|A30/ JK3 | . . . . . . . . . . . / . . . . . . . . . . . | . . . . . . . . . / . . . . . . . | . . . . . . . . / . . . . . . . | . . . . . . . / . . . . . . . | . . . . . . . . . . . . . . . . .D. / . . . . . . . . . .D. . . . . . | . . . . . . . . . . . . . . . . . . / . . . . . . . . . . . . . . . . . | . . . . |
| iPS:43 5537 | 21- 225_157H12 | VK1\|A30/ JK3 | . . . . . . . . . . . / . . . . . . . . . . . | . . . . . . . . . . . / . . . . . .F. | . . . .R. . . / . . .C. .H | . . . . . . . . / . . . . . . . | . . . . . . . . . . . . . . . . . . / . . . . . . . . . . . . . . . . . | . . .Y. . . . . . . . . . . . / . . . . . . . . . . . . . . . . . | . . . . . . / . . . . |
| iPS:43 5547 | 21- 225_158F5 | VK1\|A30/ JK3 | . . . . . . . . . . . / . . . . . . . . . . . | . . . . . . . . . . . / . . . . . . . | . . . . . . . . / . . . . . .N | . . . . . . . . / . . . . . . . | . . . . . . . . . . . . . . . . . . / . . . . . . . . . . . . . . . . . | . .D. . . . . . . . . . . . . . / . . . . . . . . . .H. . . | .E. . |
| iPS:43 5551 | 21- 225_158H8 | VK1\|A30/ JK3 | . . . . . . . . . . . / . . . . . . . . . . . | . . . . . . . . . . . / . . . .S. . . | . . . . . . . . / . . . . . . . | T. . . . . . / . . . . . . . | . . . . . . . . . . . . . . . . . . / . . . . . . . . . . . . . . . . . | . . . . . . . . . . . . . . . . . . / . . . . . . . . . . . . . . . . . | . . . . |
| iPS:43 5553 | 21- 225_158G8 | VK1\|A30/ JK3 | . . . . . . . . . . . / . . . . . . . . . . . | . . . . . . .D. . . . / . . . . . . . | . . . . . . . . / . . . .M. | T. . . . . . / . . . . . . . | . . . . . . . . . . . . . . . . . . / . . . . . . . . . . . . . . . . . | . . . . . . . . . . . . . . . . . . / . . . . . . . . . . . . . . . . . | . . . . |
| iPS:43 5569 | 21- 225_159C5 | VK1\|A30/ JK3 | . . . . . . . . . . . / . . . . . . . . . . . | .T. . . . . . . . / . . . . . . . | . . . . . . . . / . . . . . . . | . . . . . . . / . . . . . . . | . . . . . . . . . . . . . . . . .D. / . . . .I. . . .D. . . . . . . | . . . . . . . . . . . . . . . . . . / . . . . . . . . . . . . . . . . . | . . . . |
| iPS:43 5593 | 21- 225_160F4 | VK1\|A30/ JK3 | . . . . . . . . . .P. / . . . . . . . . . . . | . . . . . . . . . . . / . . . . . . . | . . . . . . . . / . . . . . .N | V. . . . . . / . . . . . . . | . . . . . . . . . . . . . . . . . . / . . . . . .V. . . . . . . . . | I.D. . . . . . . . . . . . . . / . . . . . . . . . .H. . . | .E. . |
| iPS:43 5609 | 21- 225_161F7 | VK1\|A30/ JK3 | . . . . . . . . . . . / . . . . . . . . . . . | . . . . . . . . . . . / . . . . . . . | . . . . . . .E / . . . . . . . | . . . . . . . / . . .T. . . | . . . . . . . . . . . . . . . .A. . / .V. .G.V.R. . . . . . .G | .LYIR. . . . . . . . . . . . . / . . . . . . . . . . . . . . . . . | . .R. . . / . . . . |
| iPS:43 5613 | 21- 225_161D11 | VK1\|A30/ JK3 | . . . . . . . . . . . / . . . . . . . . . . . | . . . . . . . . . . . / . . . . . . . | . . . . . . .E / . . . . . . . | . . . . . . . / . . .T. . . | . . . . . . . . . . . . . . . . .A. . / . . . .G.V.R. . . . . . .G | . .Y.R. . . . . . . . . . . . / . . . . . . . . . . . . . . . . . | . .R. . . / . . . . |
| iPS:43 5617 | 21- 225_162F2 | VK1\|A30/ JK3 | . . . . . . . . . .C / . . . . . . . . . . . | . . . . . . . . . / . . . . . . . | . . . . . . . . / . . . . . .N | . . . . . . . / . . . . . . . | . . . . . . . . . . . . . . . . . . / . . . . . .V. . . . . . . . | I.D. . . . . . . . . . . . . . / . . . . . . . . . .H. . . | .E. . |
| iPS:43 5621 | 21- 225_162H3 | VK1\|A30/ JK3 | . . . . . . . . . . . / . . . . . . . . . . . | . . . . . . . . . / . . . . . . . | . . . . . . . . / . . . . . .N | . . . . . . . / . . . . . . . | . . . . . . . . . . . . . . . . . . / . . . . . . . . . . . . . . . . . | . .D. . . . . . . . . . . . . . / . . . . . . . . . .H. . . | .E. . |

| | | | | | | | | | |
|---|---|---|---|---|---|---|---|---|---|
| iPS:43 5637 | 21-225_163E2 | VK1\|A30/ JK3 | ............ | ......D.... | ....... | P...... | ............... | ................. | .... |
| | | | ............ | ........ | ..T.... | ....... | ..S...... | ..........H... | .... |
| iPS:43 5641 | 21-225_163F9 | VK1\|A30/ JK3 | ............ | ......D.... | ....... | P...... | ............... | ..D.............. | .... |
| | | | ............ | ....D... | ....... | ....... | .............A... | ............... | .... |
| iPS:43 5643 | 21-225_163G10 | VK1\|A30/ JK3 | ............ | ......D.... | ....... | P...... | ............... | ..DY............. | .... |
| | | | ............ | ....N.. | ....... | ....... | ............... | ............... | .... |
| iPS:43 5719 | 21-225_171A11 | VK1\|A30/ JK3 | ............ | ........... | ....... | P...... | ............... | ..DH............. | .... |
| | | | ............ | ....N.. | ....... | ....... | ............... | ............... | .... |
| iPS:43 5769 | 21-225_177B6 | VK1\|A30/ JK3 | ............ | ......D.S... | ....... | ....... | ............... | ..L.............. | .... |
| | | | ............ | ........ | ....... | ....... | ....N......... | ............... | .... |
| iPS:43 5791 | 21-225_180H7 | VK1\|A30/ JK3 | ............ | ........... | ....... | T...... | ............... | ............... | ...... |
| | | | ............ | ........ | ....... | ....... | ...........S... | ............... | ..F. |
| iPS:43 5805 | 21-225_181A8 | VK1\|A30/ JK3 | ............ | ........... | ....... | T...... | ............D. | ............... | ...... |
| | | | ............ | ........ | ....... | ....... | ...........S... | ............... | ..F. |
| iPS:43 5879 | 21-225_184H10 | VK1\|A30/ JK3 | ............ | ........... | ....... | I...... | ............... | ............... | .... |
| | | | ............ | ........ | ....... | ....... | ............... | ............... | .... |
| iPS:43 5881 | 21-225_184D11 | VK1\|A30/ JK3 | ............ | ........... | ....... | I...... | ............... | ............... | .... |
| | | | ............ | ........ | ....... | ....... | ............... | ............... | .... |
| iPS:43 5921 | 21-225_190D6 | VK1\|A30/ JK3 | ............ | ........... | ...L.... | ....... | ............P.. | ...Y............. | .... |
| | | | ............ | ........ | ....... | ....... | ............... | ..........F... | .... |
| iPS:43 5985 | 21-225_192F6 | VK1\|A30/ JK3 | ............ | ........... | ...L.... | ....... | ............P.. | ...Y............. | .... |
| | | | ............ | ........ | ....... | ....... | ............... | ..........F... | .... |
| iPS:43 6074 | 21-225_194F10 | VK1\|A30/ JK3 | ............ | ........... | ...L.... | ....... | ............P.. | ...Y............. | .... |
| | | | ............ | ........ | ....... | ....... | ............... | ..........F... | .... |
| iPS:43 6092 | 21-225_195B9 | VK1\|A30/ JK3 | ............ | ........... | ....... | ....... | ............... | ...YR............ | .... |
| | | | ............ | ....K... | ....... | ...D... | ............C. | ............... | ..F. |
| iPS:43 6164 | 21-225_197G10 | VK1\|A30/ JK3 | ............ | ........... | ....... | G...... | ............... | ...YR............ | .... |
| | | | ............ | ........ | ....... | ....... | ............... | ............... | .... |
| iPS:43 6191 | 21-225_198B9 | VK1\|A30/ JK3 | ............ | ........... | ....... | ....... | ............... | ...YR............ | .... |
| | | | ............ | ....K... | ....... | ....... | ............V. | ............... | ..F. |
| iPS:43 6205 | 21-225_199A7 | VK1\|A30/ JK3 | ............ | ........... | ....... | ....... | ............... | ...YR............ | .... |
| | | | ............ | ....K... | ....... | ....... | ............V. | ............... | ..F. |
| iPS:43 6214 | 21-225_200F6 | VK1\|A30/ JK3 | ............ | ......D.... | ....... | ....... | ............... | ...YR............ | .... |
| | | | ............ | ........ | ....... | ....... | ...........H. | ............... | .... |
| iPS:43 6248 | 21-225_202A3 | VK1\|A30/ JK3 | ....S...... | ........... | ....... | ....... | ............... | ...HD............ | .... |
| | | | ............ | ........ | ......C | ...R... | ...........I... | ............... | .... |
| iPS:43 6268 | 21-225_203B9 | VK1\|A30/ JK3 | ............ | ........... | ....... | R...... | ............... | ............... | .... |
| | | | ............ | ........ | ....... | ....V.N | ...........H. | ............... | .... |
| iPS:43 6350 | 21-225_210E4 | VK1\|A30/ JK3 | ............ | ........... | ....... | ....... | ............... | ............... | .... |
| | | | ............ | ........ | ....... | ....... | ............... | ............... | .... |
| iPS:43 6576 | 21-225_225B6 | VK1\|A30/ JK3 | ............ | .......M... | ....... | ....... | ............... | ............... | .... |
| | | | .......I... | ....K... | ....... | ..T.... | ............... | ............... | .... |

EP 4 435 105 A2

| | | | | | | | | | |
|---|---|---|---|---|---|---|---|---|---|
| iPS:43 6578 | 21-225_225D6 | VK1|A30/ JK3 | . . . . . . . . . . .<br>. . . . . . . . . . . | . . . . . . . .<br>. . . . . . . . | . . . . . . . .<br>. . . . . . . | . . . . . . .<br>. . . . . . . | . . . . . . . . . . . . . . . . .<br>. . . . . . . . . . . . . . . .C. | . . . . .T. . . . . . . . . . . . .<br>. . . . . . . . . . . . . | . . . . . .<br>. . . . |
| iPS:43 6582 | 21-225_225F8 | VK1|A30/ JK3 | . . . . . . . . . . .<br>. . . . . . . . . . | . . . . . . . .<br>. . . . . . . | . . . . . . . .<br>. . . . . . . | . . . . . . .<br>. . . . .LG | . . . . . . . . . . . . . . . . .<br>. . . . . . . . . . . . . . . . | . . . . . . . . . . . . . . . . . . .<br>. . . . . . . . . . . . . | . . . . . .<br>. . . . |
| iPS:43 6608 | 21-225_226A9 | VK1|A30/ JK3 | . . . . . . . . . . .<br>. . . . . . . . . . | . . . . . . . .<br>. . . . . . . | . . . . . . . .<br>. . . . . . . | . . . . . . .<br>. . . . . . . | . . . . . . . . . . . . . . . . .<br>. . . . . . . . . . . . . . . . | . . . . . . . . . . . . . . . . . . .<br>. . . . . . . . . . . . . | . . . . . .<br>. . . . |
| iPS:43 6630 | 21-225_227G3 | VK1|A30/ JK3 | . . . . . . . . . . .<br>. . . . . . . . . | . . . . . . . .<br>. . . . . . . | . . . . . . . .<br>. . . . . . . | . . . . . . .<br>. . . . . . . | . . . . . . . . . . . . . . . . .<br>. . . . . . . . . .A. . . . . . . | .H. . . . . . . . . . . . . . . . .<br>. . . . . . . . . . . . . | . . . . . .<br>. . . . |
| iPS:43 6634 | 21-225_227H5 | VK1|A30/ JK3 | . . . . . . . . . . .<br>. . . . . . . . . . | . . . . . .D. . . .<br>. . . . . . . . | . . . . . . . .<br>. . . . . . . | . . . . . . .<br>. . . . . . . | . . . . . . . . . . . . . . . . .<br>. . . . . . . . . . . . . . . . | . . . . . . . . . . . . . . . . . . .<br>. . . . . . . . . . . . . | . . . . . .<br>. . . . |
| iPS:43 6650 | 21-225_227C12 | VK1|A30/ JK3 | . . . . . . . . . . .<br>. . . . . . . . . . | . . . . . . . .<br>. . . . . . . . | . . . . . . . .<br>. . . . . . . | . . . . . . .<br>. . . . . . . | . . . . . . . . . . . . . . . . .<br>. . . . . . . . . . . . . . . . | . . . . . . . . . . . . . . . . . . .<br>. . . . . . . . . . . . . | . . . . . .<br>. . . . |
| iPS:39 2632 | 21-225_16A11 | VK1|A30/ JK3 | . . . . . . . . . . .<br>. . . . .S. . .S. | . . . . . .D. . . .<br>. . . . .H. . | . . .RN. . .<br>. . . . . . . | . . . . . . .<br>. . . . . . . | . . . . . . . . . . . . . . . . .<br>. . . . . . . . . . . . . . . . | . . .Y. . . . . . . . . . . . . . .<br>. . . . . . . . . . . . . | . . . . . .<br>. . . . |
| iPS:39 2684 | 21-225_17F4 | VK1|A30/ JK3 | . . . . . . . . . . . .<br>. . . . . . . . . . | . . . . . . . . . .<br>. . . . . . . | . . . . . . . .<br>. . . . . . . | . . . . . . .<br>. . . . . . . | . . . . . . . . . . . . . . . . .<br>. . . . . . . . . . . . . . . . | . . . . . . . . . . . . . . . . . . .<br>. . . . . . . . . . . . . | . . . . . .<br>. . . . |
| iPS:39 2732 | 21-225_17E5 | VK1|A30/ JK3 | . . . . . . . . . . .<br>. . . . . . . . . . | . . . . . . . .<br>. . . . . . . | . . . . . . . .<br>. . . . . . . | . . . . . . .<br>T. . . . . . | . . . . . . . . . . . . . . . . .<br>. . . . . . . . . . . . . . . . | . . . . . . . . . . . .D. . . . . .L.<br>. . . . . . . | .V. .<br>. . |
| iPS:39 2778 | 21-225_22H3 | VK1|A30/ JK3 | . . . . . . . . . . .<br>. . . . . . . . . | . . . . . .D. . . .<br>. . . . .N. . | . . . . . . . .<br>. . . . . . . | P. . . . . .<br>. . . . . . .T | . . . . . . . . . . . . . . . . .<br>. . . . . . . . . . . . . . .F. | . .D. . . . . . . . . . . . . . . .<br>. . . . . . . . . . . . . | . . . . .<br>. . . . |
| iPS:39 2912 | 21-225_25A9 | VK1|A30/ JK3 | . . . . . . . . . . .<br>. . . . . . . . . | . . . . . . . . .<br>. . . . . . . | . . . . . . . .<br>. . . . . . . | . . . . . . .<br>. . . . . . . | . . . . . . . . . . . . . . . . .<br>. . . . . . . . . . . . . . . . | . . . . . . . . . . . . . . . . . . .<br>. . . . . . . . . . . . . | . . . . . .<br>. . . . |
| iPS:39 2934 | 21-225_27D5 | VK1|A30/ JK3 | . . . . . . . . . . .<br>. . . . . . . . . | . . . . . . . . .<br>. . . . . . . | . . . . . . . .<br>. . . . . . . | . . . . . . .<br>. . . . . . . | . . . . . . . . . . . . . . . . .<br>. . . . . . . . . . . . . . . . | . . . . . . . . . . . . . . . . . . .<br>. . . . . . . . . . . . . | . . . . . .<br>. . . . |
| iPS:39 2940 | 21-225_29D9 | VK1|A30/ JK3 | . . . . . . . . . . .<br>. . . . . . . . . . | . . . . . . . . . .<br>. . . . . . . | .F. . . . . .<br>. . . . . . . | . . . . . . .<br>. . . . . . . | . . . . . . . . . . . . . . . . .<br>. . . . . . . . . . . . . . . . | . . . .T. . . . . . . . . . . . . .<br>. . . . . . . . . . . . . | . .F.<br>. . |
| iPS:39 2948 | 21-225_25G5 | VK1|A30/ JK3 | . . . . . . . . . . .<br>. . . . . . . . . | . . . . . .D. . . .<br>. . . . . . . | . . . . . . . .<br>. . . . . . . | . . . . . . .<br>. . . . . . . | . . . . . . . . . . . . . . . . .<br>. . .V. . . . . . . . . . . . | . . . . . . . . . . . . . . . . . . .<br>. . . . . . . . . . . . . | . . . .<br>. . |
| iPS:39 2968 | 21-225_25B6 | VK1|A30/ JK3 | . . . . . . . . . . .<br>. . . . . . . . . | . . . . . . . . . .<br>. . . . . . . | . . . . . . . .<br>. . . . . . . | R. . . . . .<br>. . . . . . . | . . . . . . . . . . . . . . . . .<br>. . . . . . . . . . . . . . . . | . . . . . . . . . . . . . . . . . . .<br>. . . . . . . . . . . . . | . . . . . .<br>. . . . |
| iPS:39 2978 | 21-225_26B8 | VK1|A30/ JK3 | . . . . . . . . . . .<br>. . . . . . . . . | . . . . . . . . . .<br>. . . . . . . | . . .H. . . .<br>. . . . . . . | . . . . . . .<br>. . . . . . . | . . . . . . . . . . . . . . . . .<br>. . . . . . . . . . . . . . . . | . . . . . . . . . . . . . . . . . . .<br>. . . . . . . . . . . . . | . . . . . .<br>. . .N |
| iPS:39 2998 | 21-225_28A9 | VK1|A30/ JK3 | . . . . . . . . . . .<br>. . . . . . . . . | . . . . . . . . . .<br>. . . . . . . | . . . . . . . .<br>. . . . . . . | . . . . . . .<br>. . . . . .N | . . . . . . . . . . . . . . . . .<br>. . . . . . . . . . . . . . . . | . . . .R. . . . . . . . . . . . . .<br>. . . . . . . . . . . . . | . . . . . .<br>. . . . |
| iPS:39 3000 | 21-225_29D7 | VK1|A30/ JK3 | . . . . . . . . . . .<br>. . . . . . . . . . | . . . . . . . . . .<br>. . . . . . . | . . . . . . . .<br>. . . . . . . | . . . . . . .<br>. . . . . . . | . . . . . . . . . . . . . . . . .<br>. . . . . . . . . . . . . . . . | . . . . . . . . . . . . . . . . . . .<br>. . . . . . . . . . . . . | . . . . . .<br>. . . . |
| iPS:39 3006 | 21-225_31G9 | VK1|A30/ JK3 | . . . . . . . . . . .<br>. .I. . . . . . . . | . . . . . . . . .<br>. . . . . . . | . . . . . . . .<br>. . . . . . . | P. . . . . .<br>. . . . . . . | . . . . . . . . . . . . . . . . .<br>. . . . . . . . . . . . . . . . | . .D. . . . . . . . . . . . . . . .<br>. . . . . . . . . . . . . | . . . . . .<br>. . . . |
| iPS:39 3022 | 21-225_30H11 | VK1|A30/ JK3 | . . . . . . . . . . .<br>. . . . . . . . . . | . . . . . . . . .<br>. . . . . . . | . . . . . . . .<br>. . . . .V. | P. . . . . .<br>. . . . . . . | . . . . . . . . . . . . . . . . .<br>. . . . . . . . . . . . . . . . | . .D. . . . . . . . . . . . . . . .<br>. . . . . . . . . . . .H. . . | . . .T |
| iPS:39 3038 | 21-225_29D8 | VK1|A30/ JK3 | . . . . . . . . . . .<br>. . . . . . . . . | . . . . . . . . . .<br>. . . . . . . | . . . . . . . .<br>. . . . . . . | T. . . . . .<br>. . . . . . . | . . . . . . . . . . . . . . . . .<br>. . . . . . . . . . . . . . . . | . . . . . . . . . . . . . . . . . .<br>. . . . . . . . . . . . . | . . . .<br> |

| IPS | 21-225 | Germline | K_FR1 | K_CDR1 | K_FR2 | K_CDR2 | K_FR3 | K_CDR3 | K_FR4 |
|---|---|---|---|---|---|---|---|---|---|
| IPS:39 3822 | 21-225_15B11 | VK1\|A30/ JK3 | ...........<br>........... | ..........<br>........ | .......<br>....... | .......<br>....... | .................<br>................. | ................<br>................ | ......<br>.... |
| IPS:39 3944 | 21-225_14D6 | VK1\|A30/ JK3 | ...........<br>.....S..... | ......D....<br>.....H.. | ...H....<br>....... | .......<br>....... | .................<br>................. | ..Y.............<br>............... | ......<br>...N |
| IPS:39 4033 | 21-225_5F4 | VK1\|A30/ JK3 | ...........<br>........... | ..........<br>.....H.. | .......<br>....... | .......<br>....... | .................<br>...V........... | ..Y.G...........<br>............... | ......<br>.... |
| IPS:39 4069 | 21-225_16H1 | VK1\|A30/ JK3 | ...........<br>........... | ..........<br>....DI.. | .......<br>....... | .......<br>......N | .................<br>................. | ..YH............<br>............... | ....<br>..V. |
| IPS:40 2229 | 21-225_16H9 | VK1\|A30/ JK3 | ...........<br>........... | ..........<br>.....Y.. | .F......<br>....... | G.......<br>....... | .................<br>.....I........... | ..YH............<br>...........L.. | ......<br>...... |
| | VK1\|O12/JK 1 | | DIQMTQSPSSLS ASVGDRVTITC | RAS--QSIS----SYLN | WYQQKPGK APKLLIY | A-------ASSLQS | GVPSRFSGSGSG-- TDFTLTISSLQPEDFAT YYC | QQSYS------------TPWT | FGQGTK VEIK |
| IPS:46 8848 | 21-225_54B1 | VK1\|O12/ JK1 | ...........<br>..I........ | .......N....<br>........ | ........<br>...F... | ........<br>.....H. | ...P.............<br>..............I... | ....R.............<br>.........TPL.. | ......<br>.... |
| IPS:43 4239 | 21-225_58F1 | VK1\|O12/ JK1 | ...........<br>........... | ........T..<br>....NF.. | ........<br>......F | .......<br>....... | .I.............<br>................. | .................<br>...........I... | ....<br>.... |
| IPS:43 5513 | 21-225_157F3 | VK1\|O12/ JK1 | ...........<br>........... | ......N....<br>........ | ...L....<br>....... | T......<br>....... | .................<br>................. | ....N.............<br>.........TPT.. | ......<br>.... |
| IPS:43 5729 | 21-225_173E7 | VK1\|O12/ JK1 | .........R.<br>.CI...A...Y | ......T....<br>....N... | ........<br>....... | .......<br>......I | .................<br>......V........F. | ....R.............<br>.........TPQ.. | ......<br>.... |
| IPS:43 5753 | 21-225_175G10 | VK1\|O12/ JK1 | ...........<br>.......I... | ......T.G..<br>....N... | ......R<br>....... | .......<br>......H. | .................<br>..............F. | ....R.............<br>.........TPQ.. | ......<br>.... |
| IPS:43 5799 | 21-225_181G3 | VK1\|O12/ JK1 | ...........<br>.......N... | ......H.....<br>....N... | .....A..<br>..N.... | T......<br>.TLN... | .................<br>................. | .................<br>.........SP... | ......<br>.... |
| IPS:43 5813 | 21-225_183A12 | VK1\|O12/ JK1 | ..........C<br>........... | .....RN....<br>....N... | ........<br>....... | V......<br>.V..... | .................<br>................. | .................<br>.........SP... | ......<br>.D.R |
| IPS:43 6003 | 21-225_192G10 | VK1\|O12/ JK1 | ...........<br>........... | ..........<br>....N... | ....T...<br>....... | .......<br>.E..... | .................<br>..............S. | .................<br>.........SP... | ......<br>.... |
| IPS:43 6212 | 21-225_200G1 | VK1\|O12/ JK1 | ...........<br>........... | ..........<br>........ | .F......<br>....... | .......<br>.E..... | .................<br>..............S. | .................<br>.........SP... | .....<br>..F. |
| IPS:39 2730 | 21-225_17A1 | VK1\|O12/ JK1 | ...........<br>........... | ......N.N..<br>....N... | ........<br>G..V..F | T......<br>.T..... | .................<br>................. | ....T.............<br>.........TPT.. | ......<br>.... |
| IPS:39 2736 | 21-225_17B12 | VK1\|O12/ JK1 | ...........<br>.......S... | ......N.N..<br>....N... | ........<br>G..V..L | T......<br>....... | .................<br>................. | ..T.T.............<br>.........TPT.. | ......<br>.... |
| IPS:39 2766 | 21-225_23H4 | VK1\|O12/ JK1 | ...........<br>........... | ..........<br>....R... | .......R<br>......C | S......<br>.T..... | .................<br>................. | .................<br>.........TPT.. | ......<br>...R |
| IPS:39 2770 | 21-225_20C10 | VK1\|O12/ JK1 | ..H........<br>.....K.S... | .....HH....<br>....N... | ........<br>G..V..L | T......<br>....... | .................<br>................. | ....T.............<br>.........TPT.. | ......<br>.... |
| IPS:39 2808 | 21-225_20F8 | VK1\|O12/ JK1 | ...........<br>........... | ..........<br>....R... | ........<br>..E.... | .......<br>....... | .................<br>I................ | ....N.............<br>.........TPT.. | ......<br>.... |

| IPS | Clone | Germline | K_FR1 | K_CDR1 | K_FR2 | K_CDR2 | K_FR3 | K_CDR3 | K_FR4 |
|---|---|---|---|---|---|---|---|---|---|
| IPS:39 2954 | 21-225_26A10 | VK1\|O12/JK1 | ............<br>......I.... | ........<br>........ | ........<br>...V... | .......<br>....... | ................<br>................ | ....................<br>.........TPT.. | ....<br>.... |
| IPS:39 3878 | 21-225_7G12 | VK1\|O12/JK1 | ............<br>.......S... | ......N.N..<br>....N... | ........<br>G..V..L | T.......<br>....... | ................<br>....N........... | ....T...............<br>.........TPT.. | ....<br>.... |
| IPS:39 8474 | 21-225_17B10 | VK1\|O12/JK1 | ............<br>............ | .S......N..<br>........ | ........<br>......F | .......<br>.....H. | ................<br>................ | ..G.N...............<br>.........TPT.. | ....<br>...N |
| | | Germline | K_FR1 | K_CDR1 | K_FR2 | K_CDR2 | K_FR3 | K_CDR3 | K_FR4 |
| | **VK4\|B3/JK2** | | DIVMTQSPDSLA VSLGERATINC | KSS-- QSVLYSSNNKN YLA | WYQQKPGQ PPKLLIY | W------ -ASTRES | GVPDRFSGSGSG-- TDFTLTISSLQAEDVAV YYC | QQYYS------------- ---------TPYT | FGQGIK LEIK |
| IPS:46 8850 | 21-225_63F4 | VK4\|B3/JK2 | ............<br>............ | .........S.<br>...N.... | .......<br>....... | .......<br>....... | .I............G.<br>........... | ....T...............<br>...........CS | ......<br>...N |
| IPS:46 8852 | 21-225_71F3 | VK4\|B3/JK2 | ............<br>....A...... | .........SN<br>...N.... | .......<br>....... | .......<br>....... | .I.............<br>....N......... | ....T...............<br>...........CS | ......<br>.... |
| IPS:46 8870 | 21-225_74A8 | VK4\|B3/JK2 | ............<br>............ | ..........<br>..SH.... | .......<br>....... | .......<br>....... | ...............<br>............... | ....................<br>...........CS | ......<br>.... |
| IPS:43 4211 | 21-225_60F3 | VK4\|B3/JK2 | .........T<br>............ | ........<br>........ | .......<br>....... | .......<br>....... | ...............<br>..............I... | ....................<br>...........CS | ......<br>...N |
| IPS:43 4235 | 21-225_61E3 | VK4\|B3/JK2 | ............<br>............ | .........HI<br>...N.... | ....Q...<br>....... | .......<br>...I... | ...............<br>............... | ....................<br>..........I.CS | ......<br>.... |
| IPS:43 4287 | 21-225_57F12 | VK4\|B3/JK2 | ............<br>............ | .........F.<br>...Y.... | .....T..<br>....I.. | .......<br>....... | ...............<br>..............I... | ....................<br>..........N.CS | ......<br>.... |
| IPS:43 4305 | 21-225_59E1 | VK4\|B3/JK2 | ............<br>............ | ..........<br>...N.... | ....R...<br>....... | .......<br>.S..... | ...............<br>............... | ...F...............<br>..........I.CS | ......<br>.... |
| IPS:43 4443 | 21-225_71G3 | VK4\|B3/JK2 | ............<br>......VA... | .........H.<br>...N...D | .......<br>L......F | .......<br>......F | ...............<br>...........F..... | ....I...............<br>...........D...  ...........CS | ......<br>.... |
| IPS:43 4483 | 21-225_74C12 | VK4\|B3/JK2 | ............<br>............ | ..........<br>......A.... | .......<br>..N.... | .......<br>....... | ...............<br>............... | ....................<br>...........CS | ......<br>.... |
| IPS:43 4613 | 21-225_77D12 | VK4\|B3/JK2 | ............<br>...A...... | R.........<br>...Y.... | .......<br>....... | .......<br>.....D. | ...............<br>............... | ....T...............<br>...........CS | ......<br>.... |
| IPS:43 4635 | 21-225_78E6 | VK4\|B3/JK2 | ............<br>............ | ..........<br>..SH.... | .......<br>....... | .......<br>...I... | ...............<br>..S............ | ....................<br>...........CS | ......<br>.... |
| IPS:43 4679 | 21-225_79G7 | VK4\|B3/JK2 | ............<br>............ | ..........<br>..SH.... | .......<br>......F | .......<br>...I... | ...............<br>..S...M...... | ....................<br>...........CS | ......<br>.... |
| IPS:43 4909 | 21-225_85C11 | VK4\|B3/JK2 | ............<br>............ | ..........<br>..SH.F.. | ....N...<br>......F | .......<br>..FI... | ...EG.........A..<br>..S..G......... | ....................<br>...........CS | ......<br>.... |
| IPS:43 4959 | 21-225_87E10 | VK4\|B3/JK2 | ............<br>.........K. | .........H.<br>...M.... | .......<br>....... | .......<br>.....K. | ..............T<br>............... | ....................<br>.........S.CS | ......<br>.K.. |
| IPS:43 5299 | 21-225_146D4 | VK4\|B3/JK2 | ............<br>............ | ..........<br>...N.... | .......<br>....V.. | .......<br>....... | ...............<br>............... | ....................<br>...........CS | ......<br>.... |
| IPS:43 5305 | 21-225_146C9 | VK4\|B3/JK2 | ............<br>............ | .........H.<br>...Y.... | .......<br>...V... | .......<br>.....K. | ...............<br>............... | ....................<br>...........CS | ......<br>.... |

| iPS:43 | 21- | VK4\|B3/J | | | | | | | |
|---|---|---|---|---|---|---|---|---|---|
| 5309 | 225_146F9 | K2 | ............ | ......NI.H. | ........ | ........ | ............... | ....T............... | ..... |
| | | | ............ | ...N.... | ..Y.... | ....... | ....T.......... | ............CS | ...T |
| iPS:43 | 21- | VK4\|B3/J | ............ | ............ | ........ | ........ | ............... | H................... | ..... |
| 5323 | 225_147D5 | K2 | ............ | ...N.... | ........ | ....... | ..S.......... | ............CS | .... |
| iPS:43 | 21- | VK4\|B3/J | ...........R | ............ | ........ | ........ | ............... | ...F............... | ..... |
| 5399 | 225_150D2 | K2 | .........K. | ..S.K..T | ....F.. | .....K. | ......N........F. | ................N | R... |
| iPS:43 | 21- | VK4\|B3/J | ............ | .........H. | ........ | ........ | ............... | ................... | ..... |
| 5435 | 225_152H3 | K2 | .....K..... | ...Y...T | ........ | .....K. | ............... | ............CS | .... |
| iPS:43 | 21- | VK4\|B3/J | G........... | ............ | ........ | ........ | ............... | ....− | ..... |
| 5451 | 225_152D10 | K2 | ....A....D. | ........ | ........ | ........ | ....Y.......... | .....RSPS | .... |
| iPS:43 | 21- | VK4\|B3/J | AV.......... | T......I.H. | ........ | ........ | ............... | ................... | ..... |
| 5459 | 225_152E12 | K2 | ............ | ...Y.... | ........ | ........ | ............... | ..........G.CS | .... |
| iPS:43 | 21- | VK4\|B3/J | G.....F..... | ............ | ........ | ........ | .........C...... | ...N− | ..... |
| 5467 | 225_153B9 | K2 | ............ | ........ | .H..... | ......F | ....Y.V.......... | .....RSLS | .... |
| iPS:43 | 21- | VK4\|B3/J | ............ | ............ | ........ | ........ | ............... | ................... | ..... |
| 5471 | 225_153F11 | K2 | ............ | ...YK... | ..N.... | .....K. | ............... | ............CS | .... |
| iPS:43 | 21- | VK4\|B3/J | ............ | .........H. | ........ | ........ | .............H. | .H................. | ..... |
| 5475 | 225_154H6 | K2 | ............ | ...Y.... | ........ | .T...K. | ..S........... | ............CS | .... |
| iPS:43 | 21- | VK4\|B3/J | ...........S. | ............ | ........ | ........ | ............... | ................... | ..... |
| 5491 | 225_155E5 | K2 | ............ | ...N.... | ........ | .....K. | ............... | ............CS | .... |
| iPS:43 | 21- | VK4\|B3/J | ............ | .........H. | ........ | ........ | ............... | ................... | ..... |
| 5495 | 225_155B6 | K2 | ............ | ...Y.... | ........ | .T..... | ............... | ............CS | .... |
| iPS:43 | 21- | VK4\|B3/J | ............ | ............ | ........ | ........ | ...A.......... | H................. | ..... |
| 5501 | 225_156H1 | K2 | ............ | ...N.... | ........ | ........ | ............... | ............CS | .... |
| iPS:43 | 21- | VK4\|B3/J | ............ | .........H. | ........ | ........ | ............... | ....N.............. | ..... |
| 5589 | 225_160A4 | K2 | ............ | ...N.... | ........ | ....... | ............... | ..........S.CS | .... |
| iPS:43 | 21- | VK4\|B3/J | ............ | .........H. | ........ | ........ | ............... | ...FT.............. | ..... |
| 5727 | 225_172E11 | K2 | ............ | ...N.... | ........ | ....... | I....G.......... | ............CS | .... |
| iPS:43 | 21- | VK4\|B3/J | N........... | .........S. | ........ | ........ | ............... | ....T.............. | ..... |
| 6560 | 225_224F11 | K2 | ...D....... | ...H.... | ...M... | ....... | ............... | ............CS | .... |
| iPS:43 | 21- | VK4\|B3/J | ............ | ............ | ........ | ........ | ............... | .HSK............... | ....I. |
| 6584 | 225_225B9 | K2 | ............ | ...N.... | ......F | ....... | ............... | ..........I.GK | ...Q |
| iPS:43 | 21- | VK4\|B3/J | ............ | ............ | ........ | ........ | ............... | ....I.............. | ..... |
| 6588 | 225_225F12 | K2 | ............ | ...Q.... | ..R.... | .T..... | .....N........ | ............CS | .... |
| iPS:43 | 21- | VK4\|B3/J | ............ | ............N | ........ | ........ | ............... | ....I.............. | ..... |
| 6590 | 225_225H12 | K2 | ............ | ...N.... | ..N.... | ....... | ......T....... | ............CS | .... |
| iPS:43 | 21- | VK4\|B3/J | ............ | R......I..I | ........ | ........ | ............... | ................... | ..... |
| 6598 | 225_226D6 | K2 | ............ | ........ | ...M... | ....... | ............... | ..........S.CS | .... |
| iPS:43 | 21- | VK4\|B3/J | ...........T | ............ | ........ | ........ | ............... | ....I.............. | ..... |
| 6636 | 225_227E6 | K2 | ............ | ..D..... | ...M... | ....... | ............... | ............CS | .... |

| iPS | Name | Germline | K_FR1 | K_CDR1 | K_FR2 | K_CDR2 | K_FR3 | K_CDR3 | K_FR4 |
|---|---|---|---|---|---|---|---|---|---|
| iPS:43 6644 | 21-225_227G9 | VK4\|B3/JK2 | ............<br>........... | ..........H.<br>...N.... | ........<br>........ | .......<br>.G..... | .................<br>................. | ................<br>.........A..S | .....<br>.... |
| iPS:43 6646 | 21-225_227D11 | VK4\|B3/JK2 | ............<br>...D....... | ..........H.<br>...N.... | ........<br>........ | .......<br>....... | .I.......<br>................. | ....N...........<br>..........CS | .....<br>.... |
| iPS:43 7363 | 21-225_74C10 | VK4\|B3/JK2 | ............<br>........... | ........<br>...A.... | ........<br>..N..... | .......<br>....... | .................<br>................. | ................<br>..........CS | .....<br>.... |
| iPS:45 1131 | 21-225_160A7 | VK4\|B3/JK2 | ...L......P.<br>........... | .........SN<br>.H.N.... | ...R..H<br>.H....F | .......<br>....... | ........Y.....<br>................. | ................<br>..........CS | .....<br>.... |
| iPS:39 3088 | 21-225_33D1 | VK4\|B3/JK2 | ...........S<br>........... | ..I.......R<br>.......T | ........<br>.R..F.. | .......<br>....... | ..L......C...<br>....I........L... | ................<br>.........S.CS | .....<br>.... |
| iPS:39 4085 | 21-225_8B11 | VK4\|B3/JK2 | ............<br>........... | ......N...N<br>...N.... | ........<br>........ | .......<br>.....K. | .................<br>................. | ...T...........<br>..........CS | .....<br>.... |
| iPS:39 8496 | 21-225_22D2 | VK4\|B3/JK2 | ............<br>........I. | ..........H.<br>...N.... | ........<br>........ | .......<br>.....K. | .................<br>...............F. | ................<br>..........CS | .....<br>.... |
| iPS:39 8512 | 21-225_25E12 | VK4\|B3/JK2 | ...L....F..<br>M......... | ..........H<br>...Y.... | ......K.<br>........ | .......<br>....... | ..............Y.<br>................. | ................<br>..........CS | .....N<br>.... |
| iPS:39 8522 | 21-225_32A1 | VK4\|B3/JK2 | ............<br>........... | ............<br>...Y.... | ...L....<br>........ | .......<br>.....K. | .................<br>......T....L... | ....T...........<br>.........S.CS | .....<br>.... |
| iPS:39 8524 | 21-225_32A5 | VK4\|B3/JK2 | ............<br>........... | ........H.<br>........ | ........<br>........ | .......<br>....... | .................<br>..A.........L.H. | ................<br>.........S.CS | .....G<br>.... |
| iPS:39 8538 | 21-225_34H7 | VK4\|B3/JK2 | ............<br>........... | ............<br>...Y.... | ...L....<br>........ | .......<br>.....K. | .................<br>.......T....L... | ....T...........<br>.........S.CS | .....<br>.... |
| | Germline | | K_FR1 | K_CDR1 | K_FR2 | K_CDR2 | K_FR3 | K_CDR3 | K_FR4 |
| | VK2\|A17/JK4 | | DVVMTQSPLSLP VTLCQPASISC | RSS--- QSLVYSD- GNTYLN | WFQQRPGQ SPRRLIY | K------- -VSNRDS | GVPDRFSGSGSG--- TDFTLKISRVEAEDVGV YYC | MQGTH------------ -----------WPLT | FGGGTK VEIK |
| iPS:46 8854 | 21-225_72C4 | VK2\|A17/JK4 | ............<br>........... | ..G.......<br>........ | ........<br>........ | E.......<br>...KW.. | ...............N.<br>...............F.. | .................<br>........... | .....<br>.... |
| iPS:43 7250 | 21-225_148C6 | VK2\|A17/JK4 | ............<br>........... | ........<br>........ | ........<br>........ | ........<br>....W.. | .................<br>................. | .................<br>..........S.. | .....<br>.... |
| iPS:43 7252 | 21-225_148H11 | VK2\|A17/JK4 | ............<br>........... | ........<br>........ | ........<br>........ | ........<br>....W.. | .................<br>................. | .................<br>...........L.. | .....<br>.... |
| iPS:43 7254 | 21-225_149F2 | VK2\|A17/JK4 | ............<br>........... | ........<br>.....S.. | ........<br>........ | ........<br>....W.. | ...V........<br>..............I... | .................<br>...........P. | .....<br>.... |
| iPS:43 7256 | 21-225_150F11 | VK2\|A17/JK4 | ....S.Y.....<br>..F........ | ............<br>.....S.. | ........<br>Y...... | ........<br>....W.Y | ...V........<br>..............I... | .................<br>...........P. | .....<br>.... |
| iPS:43 7268 | 21-225_177D2 | VK2\|A17/JK4 | ............<br>........... | ........<br>........ | ........<br>........ | ........<br>....W.. | .................<br>................. | .................<br>................. | .....<br>.... |
| iPS:44 3005 | 21-225_43F11_LC1 | VK2\|A17/JK4 | ............<br>........... | ........<br>........ | ........<br>........ | ........<br>....W.. | .................<br>................. | ...............<br>............... | ....<br> |
| iPS:39 8530 | 21-225_32G4 | VK2\|A17/JK4 | ...........S<br>........... | ........<br>........ | ........<br>........ | ........<br>....W.. | .................<br>................. | ...I-<br>.................<br>.....HW.. | .....<br>.... |

| | | Germline | K_FR1 | K_CDR1 | K_FR2 | K_CDR2 | K_FR3 | K_CDR3 | K_FR4 |
|---|---|---|---|---|---|---|---|---|---|
| | **VK2\|A17/JK3** | | DVVMTQSPLSLP VTLGQPASISC | RSS-- QSLVYSD- GNTYLN | WFQQRPGQ SPRRLIY | K- -VSNRDS | GVPDRFSGSGSG-- TDFTLKISRVEAEDVGV YYC | MQGTH ----------WPFT | FGPGTK VDIK |
| iPS:46 8856 | 21-225_77C9 | VK2\|A17/ JK3 | .......... .......... | .......... V....S.S | ....... ....... | ....... ....W.. | ................. ................R | .............. .... |
| iPS:39 2936 | 21-225_28B6 | VK2\|A17/ JK3 | .......... .......... | .......... ...D.... | ....... ....Q.. | ....... ....W.. | ................. ..N..........I.F. | .HC.- .................. .....HWLL | ...... .... |
| | **VK2\|A17/JK5** | Germline | K_FR1 DVVMTQSPLSLP VTLGQPASISC | K_CDR1 RSS-- QSLVYSD- GNTYLN | K_FR2 WFQQRPGQ SPRRLIY | K_CDR2 K- -VSNRDS | K_FR3 GVPDRFSGSGSG TDFTLKISRVEAEDVGV YYC | K_CDR3 MQGTH ----------WPIT | K_FR4 FGQGTR LEIK |
| iPS:47 2741 | 21-225_30D9_L C1 | VK2\|A17/ JK5 | .......... .......... | .........S. .....F.. | ....... ....... | ....... ....W.. | ................. ......L.......... | L................ ...............L. | ...... .... |
| iPS:43 7294 | 21-225_216D5 | VK2\|A17/ JK5 | .......... .......... | ....... ....... | ....... ....... | ....... ....W.. | ......I........... ...............I... | ...A- .............. .....HWF. | ...... .... |
| iPS:47 2732 | 21-225_2B10_L C1 | VK2\|A17/ JK5 | .......... .......... | ....... .....F.. | ....... ....... | ....... ....W.. | ................. ...............G. | I................ .............FP | .... |
| iPS:39 8508 | 21-225_24B1 | VK2\|A17/ JK5 | .......... .......... | ....... ....... | ....... ....... | ....... ....W.. | .....W........C.. | ...A............. .........WP... | .... |
| iPS:42 3019 | 21-225_31D12_ LC1 | VK2\|A17/ JK5 | .......... .......... | .......I.. .....F.. | ....... ....... | ....... ....W.. | ................. .....L.....I... | ................. ...............L. | .... |
| | **VK1\|L5/JK1** | Germline | K_FR1 DIQMTQSPSSVS ASVGDRVIIC | K_CDR1 RAS--QGIS-- ----SWLA | K_FR2 WYQQKPGK APKLLIY | K_CDR2 A------- -ASSLQS | K_FR3 GVPSRFSGSGSG-- TDFTLTISSLQPEDFAT YYC | K_CDR3 QQANS------------- ---------FPWT | K_FR4 FGQGTK VEIK |
| iPS:43 3897 | 21-225_43C2 | VK1\|L5/J K1 | .......... .......... | ..........T ....D... | .......R .. | ....... ....... | .....N........... | ..T............. .............. | ...... .... |
| iPS:43 3903 | 21-225_43H4 | VK1\|L5/J K1 | .......... .......... | .........I.. ....N... | ....... ....... | ....... ....... | .....N........... | ..T............. ............. | .... |
| iPS:43 3911 | 21-225_43E8 | VK1\|L5/J K1 | ....N..... .......... | ............ ....N... | .......R ....... | ....... ....... | .....N........... | ..T............. ............. | .... |
| iPS:43 3941 | 21-225_44D10 | VK1\|L5/J K1 | .......... .......... | ............ ....D... | .......R .. | ....... ....... | ....K........... .....N........... | ..T............. ............. | .... |
| iPS:43 3945 | 21-225_44C12 | VK1\|L5/J K1 | .......... .......... | ............ ....N... | ......S ..F.... | ....... ....... | ..S............. | ..S............. ............. | .... |
| iPS:43 3957 | 21-225_45F8 | VK1\|L5/J K1 | .......... .......... | ............ ....D... | .......R .. | G...... ....... | .....N........... | ..T............. ............. | .... |

| | | | | | | | | |
|---|---|---|---|---|---|---|---|---|
| iPS:43 3973 | 21-225_46A6 | VK1\|L5/J K1 | ....N....... ........N... | ..........  ....N.... | ........  V...... | ........  ....... | ............  .....N...... | ..T............  ............ | ......  .... |
| iPS:43 3993 | 21-225_47G7 | VK1\|L5/J K1 | ...........  ........... | ..........  ....N... | ........  ......F | ........  ...N... | ............  .........A.....C. | ..V............  ............ | ....  .... |
| iPS:43 4007 | 21-225_48D7 | VK1\|L5/J K1 | ...........  ........... | ......N.T..  ....... | ........  ....... | S......  ......N | ............  ........V..C. | ............  ............ | ....  .... |
| iPS:43 4063 | 21-225_51G7 | VK1\|L5/J K1 | ...........  ........... | ......D....  ....... | ........  ...V... | ........  .P.N... | A...........  ............ | ...H............  ............ | ....  .... |
| iPS:43 4083 | 21-225_52H2 | VK1\|L5/J K1 | ...........  ........... | ......N.T..  ....N... | .F.....R  ....... | T......  .T.... | ............  ............C. | ..T............  ............ | ..H...  ..V. |
| iPS:43 4133 | 21-225_54G3 | VK1\|L5/J K1 | ...........  ........... | ..........  ....... | ........  ....... | D......  ....... | ..........E.....  ............ | ............  ............ | ......  .... |
| iPS:43 4221 | 21-225_60A11 | VK1\|L5/J K1 | ...........  ........... | ......V....  ....N... | ...L....  ....... | T......  ....... | ........NE......  ............ | ............  ............ | ....  .... |
| iPS:43 4283 | 21-225_57F8 | VK1\|L5/J K1 | ...........  ........... | ..........  ....N... | ........  ....... | T......  ....... | ........NE......  ............ | ............  ............ | ....  .... |
| iPS:43 5711 | 21-225_171G4 | VK1\|L5/J K1 | ...........  ........... | ......VN..  ....D... | .......R  ....... | D......  ....... | ............  ............ | ............  ............ | ....  .... |
| iPS:43 5715 | 21-225_171A8 | VK1\|L5/J K1 | A..........  ........... | ..........  ....N... | ........  .N.M.H | ..F...G  ....... | ............  ............ | ..T............  ............ | ....  .... |
| iPS:43 5717 | 21-225_171A9 | VK1\|L5/J K1 | ...........  ........... | ......D.T..  ....T... | ........  ....... | D......  ....... | A...........  ...V........ | L.T............  ............ | ......  .... |
| iPS:43 5739 | 21-225_174G7 | VK1\|L5/J K1 | A..........  ........... | ..........  ....N... | ........  .N...H | ..F...G  ....... | ............  ............ | ..T............  ............ | ....  .... |
| iPS:43 5749 | 21-225_175C10 | VK1\|L5/J K1 | ...........  ........... | ........T..  ....D... | ........  ....... | ........  ....... | ...F.....D......  ............ | ..T............  ............ | ......  .... |
| iPS:43 5775 | 21-225_178A5 | VK1\|L5/J K1 | ...........  ........... | ..........  ....N... | ........  ....... | ........  ....... | ......N......  ............C. | ............  .........L... | ....  .... |
| iPS:43 5777 | 21-225_178F7 | VK1\|L5/J K1 | ...........  ........... | ......D.T..  ....D... | ........  .....S | ........  ....... | .A.........  ............ | ............  .........L... | ......  .... |
| iPS:43 5783 | 21-225_179G1 | VK1\|L5/J K1 | ...........  ........... | ......D....  ....D... | .....S..  .....S | ........  ....... | ....G......  ............ | ............  .........L... | ....  .... |
| iPS:43 5875 | 21-225_190B9 | VK1\|L5/J K1 | ...........  ........... | ........N..  ....N... | ........  ....... | G......  .V..... | ............  ............S. | ............  ............ | ...R  |
| iPS:43 5895 | 21-225_188E8 | VK1\|L5/J K1 | ...........  ........... | ..N...D....  ....... | ........  ....... | ........  ...N... | ......G......  ............ | ............  ............ | ....  |
| iPS:43 5909 | 21-225_190H3 | VK1\|L5/J K1 | ...........  ........... | ......LN...  ....N... | ...L....  ....... | ........  .V..... | ..........SE.  ............H. | ............  .........L... | ....  |
| iPS:43 6013 | 21-225_193F2 | VK1\|L5/J K1 | ...........  ........... | ........N..  ....N... | ........  ....... | G......  .V..... | ............  ............S. | ............  ............ | ...R  |
| iPS:43 6068 | 21-225_194F7 | VK1\|L5/J K1 | ...........  ........... | ..........  ....R... | .F......  ...I... | ........  ....... | ............  ............ | ............  ............ | ....  |

| ID | Name | Gene | K_FR1 | K_CDR1 | K_FR2 | K_CDR2 | K_FR3 | K_CDR3 | K_FR4 |
|---|---|---|---|---|---|---|---|---|---|
| iPS:43 6100 | 21-225_195G12 | VK1|L5/JK1 | ............. | ........,N.. | ........ | G....... | ................ | .................. | ..R... |
| | | | ............. | ....N... | ........ | .V..... | ................S. | ............... | ..NQ |
| iPS:43 6116 | 21-225_196B9 | VK1|L5/JK1 | ............. | ........... | ........ | ........ | A............... | ..GD............. | ...... |
| | | | ............. | ....NC.. | ...F..C | ........ | ................S... | ............P. | ..FR |
| iPS:43 6160 | 21-225_197C9 | VK1|L5/JK1 | ............. | ........... | .F..... | ........ | ................ | .................. | ...... |
| | | | ............. | ....N... | ..Q.... | ........ | S.............. | ................. | ...... |
| iPS:43 6546 | 21-225_224D6 | VK1|L5/JK1 | ............. | ........ | ........ | ........ | ........ | ........ | ..P... |
| | | | ............. | ........ | ........ | ........ | ........ | ........ | .... |
| iPS:43 6632 | 21-225_227E4 | VK1|L5/JK1 | ..L........ | ........I.. | ........ | ........ | ........ | ........ | ...... |
| | | | ........... | ....N... | ........ | ........ | ................I.... | ............... | .... |
| iPS:39 2650 | 21-225_17A4 | VK1|L5/JK1 | ........... | ........G.. | ........ | ........ | ........ | ........ | ...... |
| | | | ........... | ....N... | ......F | ........ | ................V.... | ...........R. | .... |
| iPS:39 2728 | 21-225_20F7 | VK1|L5/JK1 | ........... | ........ | ........ | ........ | ........ | ........ | ...... |
| | | | .....G..... | ........ | ........ | ........ | ........ | ...........R. | .... |
| iPS:39 2916 | 21-225_27C5 | VK1|L5/JK1 | ........... | ........ | ........ | G....... | ......A.......E. | ..YD........... | ...... |
| | | | ........... | ........ | ..F... | ........ | ................C. | ...........R. | .... |
| iPS:39 2956 | 21-225_27A11 | VK1|L5/JK1 | ........... | ........ | ........ | G....... | ......A......... | ..SD........... | ...... |
| | | | ........... | ........ | ........ | ........ | ................C. | ...........R. | .... |
| iPS:39 3014 | 21-225_26D12 | VK1|L5/JK1 | ........... | ........ | ........ | G....... | ......A......... | ..SD........... | ...... |
| | | | ........... | ........ | ........ | ........ | ................C. | ...........R. | .... |
| iPS:39 3028 | 21-225_25D7 | VK1|L5/JK1 | ........... | ......D.F.. | .......T | ........ | ..............N. | ...Y........... | ...... |
| | | | .......F.. | ....D... | ........ | ........ | ...V.G.......... | ............... | .... |
| iPS:39 3152 | 21-225_25B3 | VK1|L5/JK1 | ........... | ........ | ........ | G....... | ......A......... | ..SD........... | ...... |
| | | | ........... | ........ | ........ | ........ | ................C. | ...........R. | .... |
| iPS:39 3810 | 21-225_5A4 | VK1|L5/JK1 | ........... | ........ | ........ | D....... | ........ | .................. | ...... |
| | | | ........... | ....T... | ........ | ........ | ........ | ................. | .... |
| **Germline** | | | **K_FR1** | **K_CDR1** | **K_FR2** | **K_CDR2** | **K_FR3** | **K_CDR3** | **K_FR4** |
| **VK2|A19/JK3** | | | DIVMTQSPLSLP VTPGEPASISC | RSS-- QSLLHSN- GYNYLD | WYLQKPGQ SPQLLIY | L------- -GSNRAS | GVPDRFSGSGSG-- TDFTLKISRVEAEDVGV YYC | MQALQ------------ ----------TPFT | FGPGTK VDIK |
| iPS:43 3943 | 21-225_44E10 | VK2|A19/JK3 | ........... | ........... | ........ | ........ | ................ | ..T........ | ...... |
| | | | ........... | ....S..E | ........ | ........ | ................. | ............... | .... |
| iPS:43 3989 | 21-225_47C7 | VK2|A19/JK3 | .........P. | ........... | .....S.. | .F.... | ......T........ | ..V........... | ...... |
| | | | ........... | ........E | ..F... | ........ | ................. | ............... | .... |
| **Germline** | | | **K_FR1** | **K_CDR1** | **K_FR2** | **K_CDR2** | **K_FR3** | **K_CDR3** | **K_FR4** |
| **VK1|O12/JK3** | | | DIQMTQSPSSLS ASVGDRVTITC | RAS--QSIS-- ----SYLN | WYQQKPGK APKLLIY | A------- -ASSLQS | GVPSRFSGSGSG-- TDFTLTISSLQPEDFAT YYC | QQSYS----------- ----------TPFT | FGPGTK VDIK |
| iPS:43 3999 | 21-225_48D1 | VK1|O12/JK3 | ........... | ........... | ........ | ........ | ......A......... | ...N........... | ...... |
| | | | .......N... | .......I | ........ | ........ | ................S... | ...........I... | .... |
| iPS:43 4003 | 21-225_48C3 | VK1|O12/JK3 | ........... | .......I.. | ........ | ........ | ......A......... | ..TN........... | ...... |
| | | | .......N... | .......I | ..R.... | ........ | ................S... | ..........I... | .... |

| | | | | | | | | |
|---|---|---|---|---|---|---|---|---|
| iPS:43 4037 | 21-225_49G12 | VK1\|O12/ JK3 | ............<br>............ | .S.........<br>....T..M | .......<br>....... | .......<br>......I | ....E..A.........<br>............. | .................<br>..........I... | ....<br>.... |
| iPS:43 4041 | 21-225_50H8 | VK1\|O12/ JK3 | ...........<br>.......N... | ...........<br>.......I | .......<br>....... | .......<br>....... | ..........A....<br>............S... | ...N.............<br>.........L... | ....<br>.... |
| iPS:43 4045 | 21-225_50H10 | VK1\|O12/ JK3 | ...........<br>.......N... | .........Y..<br>.......I | .......<br>....... | .......<br>....... | ..........A....<br>............S... | ...N.............<br>..........I... | ....<br>.... |
| iPS:43 4049 | 21-225_50B12 | VK1\|O12/ JK3 | ...........<br>........... | ...........<br>....... | ...H....<br>..R.... | .......<br>....... | ..........E......<br>I...........T.... | ....I............<br>.........A... | ....<br>.... |
| iPS:43 4073 | 21-225_51H10 | VK1\|O12/ JK3 | ...........<br>........... | ...........<br>....T..M | .......<br>....... | .......<br>....... | ....E..A.........<br>................. | .................<br>..........I... | ....<br>.... |
| iPS:43 4107 | 21-225_53E2 | VK1\|O12/ JK3 | ...........<br>........... | .........F...<br>....H... | .......<br>..N...F | .......<br>.V..... | ...........S..<br>..P.........I.F. | ...F.............<br>................ | ....<br>.... |
| iPS:43 4181 | 21-225_56B2 | VK1\|O12/ JK3 | ...........<br>........... | .........F...<br>....H... | .......<br>..N...F | .......<br>.V..... | .................<br>............I.F. | .................<br>................ | ....<br>.... |
| iPS:43 4225 | 21-225_60E12 | VK1\|O12/ JK3 | ...........<br>........... | ...........<br>....... | .......<br>....... | .......<br>....... | .................<br>............P... | ....N............<br>.........IS.. | ....<br>.... |
| iPS:43 4227 | 21-225_61A1 | VK1\|O12/ JK3 | ...........<br>........... | ...........<br>....... | .......<br>....... | .......<br>....... | .................<br>............P... | ....N............<br>..........IS.. | ....<br>.... |
| iPS:43 4245 | 21-225_62H1 | VK1\|O12/ JK3 | ...........<br>.Y......... | ......N.F..<br>....... | .......<br>...V... | .......<br>VF.... | .................<br>................. | .................<br>................. | ....<br>.... |
| iPS:43 4267 | 21-225_57F2 | VK1\|O12/ JK3 | ...........<br>........... | ...........<br>....... | .......<br>....... | .......<br>....... | .................<br>............P... | ....N............<br>.........IS.. | ....<br>.... |
| iPS:43 4323 | 21-225_62H8 | VK1\|O12/ JK3 | ...........<br>........... | ........F..<br>....... | ....N...<br>....... | .......<br>.S..... | .....L..N.......<br>I................ | .................<br>................ | .....R<br>.... |
| iPS:43 4379 | 21-225_66A9 | VK1\|O12/ JK3 | ...........<br>........... | ......N.F..<br>....... | .......<br>......F | .......<br>....... | .................<br>................. | ..T............<br>.........V... | ....<br>..F. |
| iPS:43 4417 | 21-225_69C8 | VK1\|O12/ JK3 | ...........<br>........F.. | .G...T.Y..<br>...N... | .......<br>......H | V......<br>....... | .................<br>..V.............. | .................<br>................ | ....<br>.... |
| iPS:43 5545 | 21-225_158F4 | VK1\|O12/ JK3 | ..........P<br>........... | ......N.R..<br>....K..H | ...FL...<br>....... | T......<br>...T... | .................<br>................. | ....N............<br>.........IS.. | ....<br>.... |
| iPS:43 5793 | 21-225_180F8 | VK1\|O12/ JK3 | ...........<br>.....G..... | ......T.L..<br>....... | .......<br>....... | G......<br>.V..... | .................<br>S................ | .................<br>................ | ....<br>.... |
| iPS:43 6504 | 21-225_222H4 | VK1\|O12/ JK3 | .....H.....<br>........... | ......N....<br>....N.V. | .......<br>..F... | T......<br>....... | ..S............<br>......VHRD...I... | ..Y.F............<br>................ | ..R...<br>.... |
| iPS:43 6510 | 21-225_222H8 | VK1\|O12/ JK3 | .....H.....<br>........... | ......N....<br>....N.V. | .......<br>..F... | I......<br>....... | ..S............<br>......VHRD...I... | ..Y.F............<br>................ | ..R...<br>.... |
| iPS:43 7230 | 21-225_62H10 | VK1\|O12/ JK3 | ...........<br>........... | ........T..<br>....... | .......<br>V.....S | T......<br>....... | .................<br>................. | ...H............<br>.........F... | .....N<br>..F. |
| iPS:44 8906 | 21-225_72G9 | VK1\|O12/ JK3 | ...........<br>........... | ........T..<br>....... | .......<br>....... | T......<br>....... | .................<br>................. | ...H............<br>.........F... | ....<br>.... |
| iPS:39 2652 | 21-225_17C6 | VK1\|O12/ JK3 | ...........<br>........... | ........N..<br>....T... | .......<br>....... | .......<br>....... | .................<br>..............F. | ....R............<br>.........TPF.. | .....<br>.... |

| ID | Clone | Germline | | | | | | | |
|---|---|---|---|---|---|---|---|---|---|
| IPS:39 2660 | 21-225_19B3 | VK1IO12I JK3 | ..............<br>.......I.... | ..G...N.I..<br>....,N... | ........<br>..N.... | V.......<br> | .......N.........<br> | ....................<br> | ....<br>..... |
| IPS:39 2668 | 21-225_17B4 | VK1IO12I JK3 | ..............<br>.............. | ......N....<br>........ | ........<br>......F | G.......<br>.......T | ....N.........F.<br> | ....R...............<br>.TPF.. | ....<br>.... |
| IPS:39 2678 | 21-225_20F3 | VK1IO12I JK3 | ..............<br> | ..........<br>.......Y | ........<br>........ | | | .........AP...<br> | ....<br>.... |
| IPS:39 2694 | 21-225_19A5 | VK1IO12I JK3 | ..............<br>.P.....S... | ......N.I..<br>....,N... | ........<br>......D | V.......<br>...N..G | ................<br> | ....................<br> | ....<br>.... |
| IPS:39 2696 | 21-225_20A4 | VK1IO12I JK3 | ........A...<br> | ........I..<br>....N... | ....R...<br>S..... | ......H. | ........R.......<br> | ....R...............<br>.TPL.. | ....<br>..F. |
| IPS:39 2702 | 21-225_17F7 | VK1IO12I JK3 | ..............<br> | .....T...<br>....F.. | ........<br>........ | ........<br> | ....G.........F.<br> | ...R................<br>.TPF.. | ..F.<br>...S. |
| IPS:39 2704 | 21-225_17F11 | VK1IO12I JK3 | ..............<br>..I....S... | .....RT.N..<br>....N... | ........<br>.....F | .T.....<br> | ................<br> | ..T.................<br>.TPL.A | ....<br>.... |
| IPS:39 2720 | 21-225_17A12 | VK1IO12I JK3 | ..............<br> | ........<br> | ..H....<br> | ........<br> | ....N...........<br> | .........TPL..<br> | ....<br>.... |
| IPS:39 2722 | 21-225_18E12 | VK1IO12I JK3 | ..............<br> | ........<br> | ........<br>..T.... | ........<br> | ....N.........F.<br> | ...R................<br>.TPF.. | ....<br>.... |
| IPS:39 2760 | 21-225_22G3 | VK1IO12I JK3 | ..............<br> | ........<br>....N... | ........<br>......F | ........<br> | ................<br> | ...FR...............<br>.TPF.. | ....<br>.... |
| IPS:39 2762 | 21-225_22G5 | VK1IO12I JK3 | ..............<br> | .....N....<br>........ | ........<br>....Q.. | ........<br>.....N | ........R.......<br> | ....R...............<br>.TPL.. | ..F.<br> |
| IPS:39 2764 | 21-225_22G10 | VK1IO12I JK3 | ..............<br> | ........<br>.......F.. | ........<br> | ........<br> | ................F.<br> | ...FR...............<br>.TPL.. | ..F.<br> |
| IPS:39 2812 | 21-225_21F4 | VK1IO12I JK3 | ..............<br> | .....N.G..<br>........ | ........<br>......F | ........<br> | ................FF.<br> | ....R...............<br>.TPF.. | ..F.<br> |
| IPS:39 2816 | 21-225_22E4 | VK1IO12I JK3 | ..............<br> | .....N....<br> | ........<br> | ........<br> | ................<br> | ....N...............<br>.TPL.. | ....<br> |
| IPS:39 2830 | 21-225_21A5 | VK1IO12I JK3 | ...........C<br> | ......T....<br>....H.. | ..R....<br> | ........<br> | ....V...........<br> | ....N...............<br>.IS.. | ....<br> |
| IPS:39 2852 | 21-225_21A2 | VK1IO12I JK3 | ..............<br> | ........<br> | ........<br> | ........<br> | ................F.<br> | ....R...............<br>.TPF.. | ....<br> |
| IPS:39 2878 | 21-225_22C5 | VK1IO12I JK3 | ........A...<br> | .....N....<br> | ........<br>...V..H | .I.....R.....<br>.T.............. | ................<br> | ....R...............<br>.TPL.. | ..F.<br> |
| IPS:39 2902 | 21-225_22D5 | VK1IO12I JK3 | ..............<br> | .....N.F..<br> | ..H....<br> | ........<br> | ................<br> | .........TPL..<br> | ....<br> |
| IPS:39 2984 | 21-225_30E11 | VK1IO12I JK3 | ..............<br> | ........<br>...N... | ....QT..<br>..F.. | ........<br> | ................<br> | ....................<br> | ....<br> |
| IPS:39 3114 | 21-225_33G12 | VK1IO12I JK3 | ..............<br> | ........<br>...N... | ....QT..<br>..F... | ........<br> | ................<br> | ....................<br> | ....<br> |
| IPS:39 3824 | 21-225_10F12 | VK1IO12I JK3 | ..............<br> | .....N....<br> | ........<br> | ........<br> | ................<br> | ....N...............<br>.TPF.. | ....<br> |

| iPS | Clone | Germline | K_FR1 | K_CDR1 | K_FR2 | K_CDR2 | K_FR3 | K_CDR3 | K_FR4 |
|---|---|---|---|---|---|---|---|---|---|
| iPS:39 3848 | 21-225_4H2 | VK1\|O12/ JK3 | .....L......<br>............ | ..I...N....<br>........ | ........<br>....V.. | ........<br>....... | .........R........<br>....GCV.R....... | ....R.............<br>.........TPL.. | ......<br>.... |
| iPS:39 3862 | 21-225_5G2 | VK1\|O12/ JK3 | .........P.<br>.........F | ......N.I..<br>........ | ........<br>.R..V.. | G.......<br>....... | ................<br>..N.R........... | ................<br>.........TPL.. | ......<br>.... |
| iPS:39 3888 | 21-225_3E3 | VK1\|O12/ JK3 | .........P.<br>.........F | ........R..<br>........ | ........<br>.H..V.. | G.......<br>.T..... | ................<br>................ | ................<br>.........TPL.. | ......<br>.... |
| iPS:39 3890 | 21-225_4B1 | VK1\|O12/ JK3 | ............<br>............ | ......HT.R..<br>....T... | ........<br>........ | ........<br>........ | ......IN........<br>....TN.......... | ....N............<br>...........IS.. | ......<br>.... |
| iPS:39 3898 | 21-225_5F7 | VK1\|O12/ JK3 | .........P.<br>.........F | ......T....<br>........ | ........<br>......S | ........<br>........ | ................<br>................ | ....N............<br>.........TPL.. | ......<br>.V.. |
| iPS:39 3904 | 21-225_8H11 | VK1\|O12/ JK3 | ............<br>.F......... | ......N.I..<br>........ | ........<br>..N.M.. | V.......<br>.T...H. | S...............<br>................ | ................<br>................ | ......<br>.... |
| iPS:39 3936 | 21-225_14A11 | VK1\|O12/ JK3 | ............<br>..I........ | ........<br>........ | ........<br>......F | ........<br>......N | ................<br>................ | ..T..............<br>.........SP... | .A....<br>.... |
| iPS:39 3980 | 21-225_6D3 | VK1\|O12/ JK3 | ............<br>............ | .T.........<br>....T... | ........<br>......F | ........<br>........ | ................<br>...............F. | ....R............<br>.........TPF.. | ......<br>.... |
| iPS:39 4014 | 21-225_8G6 | VK1\|O12/ JK3 | ............<br>............ | ........<br>........ | ........<br>......F | ........<br>........ | ................<br>...............F. | ....R............<br>.........TPF.. | ......<br>.... |
| iPS:39 4022 | 21-225_16H6 | VK1\|O12/ JK3 | ............<br>............ | ......N....<br>........ | ........<br>........ | ........<br>........ | ........R........<br>................ | ....R............<br>.........TPL.. | ......<br>..F. |
| iPS:39 4043 | 21-225_3B1 | VK1\|O12/ JK3 | ............<br>............ | ........N..<br>....N... | ........<br>........ | ........<br>.T...N | ................<br>................ | ................<br>.........TPL.. | ......<br>...N |
| iPS:39 4051 | 21-225_9E5 | VK1\|O12/ JK3 | .......Q....<br>............ | ........A..<br>........ | ........<br>........ | G.......<br>........ | ................<br>................ | ................<br>.........TPL.S | ......<br>.... |
| iPS:39 4077 | 21-225_8E12 | VK1\|O12/ JK3 | ............<br>............ | ........<br>....N... | ........<br>........ | ........<br>........ | ................<br>...............F. | ....R............<br>.........TPF.. | ......<br>.... |
| iPS:39 4087 | 21-225_11A5 | VK1\|O12/ JK3 | ............<br>............ | ......N.Y..<br>........ | ........<br>........ | ........<br>........ | ................<br>................ | ....N............<br>.........TPL.. | ......<br>.... |
| | | Germline | K_FR1 | K_CDR1 | K_FR2 | K_CDR2 | K_FR3 | K_CDR3 | K_FR4 |
| | VK2\|A18/JK3 | | DIVMTQTPLSLS VTPGQPASISC | KSS-- QSLLHSD- GKTYLY | WYLQKPGQ SPQLLIY | E----- -VSSRFS | GVPDRFSGSGSG-- TDFTLKISRVEAEDVGV YYC | MQGIH------ ---------LPFT | FGPGTK VDIK |
| iPS:43 4053 | 21-225_51E1 | VK2\|A18/ JK3 | ............<br>........M.. | ...R....<br>E....... | ...R....<br>P..F..F | ........<br>...N... | ................E.<br>................ | ..S.Q............<br>................ | ......<br>.... |
| iPS:43 4137 | 21-225_54D4 | VK2\|A18/ JK3 | ............<br>........M.. | ...R....<br>E....... | ...R....<br>P..F..F | ........<br>...N... | ................E.<br>............I... | ..S.Q............<br>..........F... | ......<br>.... |
| iPS:43 4149 | 21-225_55H1 | VK2\|A18/ JK3 | ............<br>............ | ........<br>E....... | ........<br>P..F..F | ........<br>...H... | ................<br>................ | ..S.Q............<br>................ | ......<br>.... |
| iPS:43 5315 | 21-225_147B2 | VK2\|A18/ JK3 | ............<br>............ | ........<br>E....... | ........<br>P...... | ........<br>...H.V. | .V...............<br>................ | ..STQ............<br>..........F.P. | ......<br>.... |
| | | Germline | K_FR1 | K_CDR1 | K_FR2 | K_CDR2 | K_FR3 | K_CDR3 | K_FR4 |

| ID | Name | Germline | K_FR1 | K_CDR1 | K_FR2 | K_CDR2 | K_FR3 | K_CDR3 | K_FR4 |
|---|---|---|---|---|---|---|---|---|---|
| | VK1\|O18/JK3 | | DIQMTQSPSSLS ASVGDRVTITC | QAS--QDIS-- ----NYLN | WYQQKPGK APKLLIY | D------- -ASNLET | GVPSRFSGSGSG-- TDFTFTISSLQPEDIAT YYC | QQYDN------------- -----------LPFT | FGPGTK VDIK |
| IPS:43 4055 | 21-225_51B4 | VK1\|O18/JK3 | ..........  .......... | .....R..T..  ....F... | .......  ....... | .......  ....... | ..............  .....CVH.......L. | ................  ............... | .....T  .... |
| | Germline | | K_FR1 | K_CDR1 | K_FR2 | K_CDR2 | K_FR3 | K_CDR3 | K_FR4 |
| | VK1\|O18/JK4 | | DIQMTQSPSSLS ASVGDRVTITC | QAS--QDIS-- ----NYLN | WYQQKPGK APKLLIY | D------- -ASNLET | GVPSRFSGSGSG-- TDFTFTISSLQPEDIAT YYC | QQYDN------------- -----------LPLT | FGGGTK VEIK |
| IPS:43 4087 | 21-225_52F6 | VK1\|O18/JK4 | ..........  .......... | ..........  .......H | .......  ....... | .......  ...T.G. | ...L..........E.  ....N.........S. | ..C...............  ............... | ......  .... |
| IPS:43 4111 | 21-225_53H2 | VK1\|O18/JK4 | ..........  .......... | ..........  .......H | .......  ..Q.... | .......  ....... | ......T  ............... | H...............  ............... | ......  .... |
| IPS:43 4121 | 21-225_53F6 | VK1\|O18/JK4 | ..........  .......... | .......T..  .......D | .......  ....... | .......  .....G. | ..............  ............... | ..C...............  ............... | ......  .... |
| IPS:43 4163 | 21-225_50H1 | VK1\|O18/JK4 | .........P.  .......... | ..........  .......D | .......  ....... | .......  ....... | ...............  A.............. | ..C...............  ............... | ......  .... |
| IPS:43 5611 | 21-225_161F10 | VK1\|O18/JK4 | ..........  .......... | .......Y..  .....H.S | .......  ....... | .......  ....W.. | ........G.  ......F.... | ...E...............  ............... | ......  .... |
| IPS:43 5811 | 21-225_183H6 | VK1\|O18/JK4 | ..........  .........A. | ..........  ......... | ...T...  ...V... | .......  ....... | ...A  ............... | ...............  ............... | .D..  .... |
| IPS:39 4035 | 21-225_5G9 | VK1\|O18/JK4 | ..........  .......... | .....G....  .....S.. | .......  ....... | .......  ....... | ...............  .....A......... | ...............  ............... | ......  .... |
| | Germline | | K_FR1 | K_CDR1 | K_FR2 | K_CDR2 | K_FR3 | K_CDR3 | K_FR4 |
| | VK1\|L1/JK1 | | DIQMTQSPSSLS ASVGDRVTITC | RAS--QGIS-- ----NYLA | WFQQKPGK APKSLIY | A------- -ASSLQS | GVPSRFSGSGSG-- TDFTLTISSLQPEDFAT YYC | QQYNS------------- -----------YPWT | FGQGTK VEIK |
| IPS:43 4095 | 21-225_52F10 | VK1\|L1/JK1 | ..........  V......... | .......  .......G | .......  ....... | .......  ....... | ...............  ............... | ...............  ...........P. | ......  .... |
| IPS:39 2848 | 21-225_20F9 | VK1\|L1/JK1 | ..........  ......I.... | .......  ......... | .......  ......S | .......  ....... | ....K  ............... | ...H...............  ............C. | ......  .... |
| IPS:39 3078 | 21-225_33H11 | VK1\|L1/JK1 | ..........  .F........ | W.......N..  ....S... | ......R...  .H..... | .......  ......G | ....K  I.......R........ | ..F...............  ...............L. | ......  .... |
| IPS:39 3142 | 21-225_33A3 | VK1\|L1/JK1 | ..........  .......... | .......N..  ......... | .......  ....... | .......  ....... | ....K  ............... | ..F...............  ...........P. | ......  .... |
| IPS:39 3946 | 21-225_16A4 | VK1\|L1/JK1 | ..........  .......... | ......D....  ......... | .......  ......S | .......  ....... | ....K  ............... | ...H...............  ............... | .....  .....N |
| | Germline | | K_FR1 | K_CDR1 | K_FR2 | K_CDR2 | K_FR3 | K_CDR3 | K_FR4 |
| | VK1\|O12/JK5 | | DIQMTQSPSSLS ASVGDRVTITC | RAS--QSIS-- ----SYLN | WYQQKPGK APKLLIY | A------- -ASSLQS | GVPSRFSGSGSC-- TDFTLTISSLQPEDFAT YYC | QQSYS------------- -----------TPIT | FGQCTR LEIK |
| IPS:43 4117 | 21-225_53C6 | VK1\|O12/JK5 | ..........  .......... | ......YS...  ....D... | ...V..F  .....K. | .......  ....... | .......E.......F.  ............... | ...............  ...........F. | ......  .... |

| ID | Clone | Germline | K_FR1 | K_CDR1 | K_FR2 | K_CDR2 | K_FR3 | K_CDR3 | K_FR4 |
|---|---|---|---|---|---|---|---|---|---|
| iPS:43 4317 | 21-225_59E8 | VK1|O12/ JK5 | .............<br>............. | .........<br>........ | ........<br>....... | .......<br>....... | ....................<br>....................F. | ...F...............<br>..........NS.. | ......<br>.... |
| iPS:43 4327 | 21-225_63G6 | VK1|O12/ JK5 | .............<br>......I.S. | .........F..<br>......... | ...V....<br>........ | D.......<br>.T.T..T | ....................<br>....N............ | ....G...............<br>....................I... | ......<br>...Q |
| iPS:43 4455 | 21-225_72F5 | VK1|O12/ JK5 | .............<br>......I.P. | ......N....<br>........ | ........<br>........ | ........<br>........ | ....................<br>...................S. | ..T.-<br>...................<br>.....STP. | ......<br>.D.N |
| iPS:43 5525 | 21-225_157E7 | VK1|O12/ JK5 | .............<br>............. | ......F...<br>........ | .......I<br>........ | ........<br>........ | ....................<br>.................... | .E...............<br>.................IRFA | ......<br>.... |
| iPS:39 2754 | 21-225_21D3 | VK1|O12/ JK5 | .............<br>........... | .......T..<br>....G.S. | ........<br>T.....F | ........<br>.TY..E. | ....................<br>....T............ | .........F....N.<br>..............S.. | ......<br>.... |
| iPS:39 2818 | 21-225_22D8 | VK1|O12/ JK5 | .............<br>............ | .T....NSN..<br>......... | ........<br>....Q.F | ........<br>..Y..E. | ............NR.....E.<br>.................... | ..T.G.............<br>..............S.. | ......<br>.... |
| iPS:39 3064 | 21-225_33A9 | VK1|O12/ JK5 | .............<br>........... | ......R<br>....R..S | .......R<br>..N.Q.. | ........<br>....... | ....................<br>.................... | ....N.............<br>..............I... | ......<br>.... |
| iPS:39 3148 | 21-225_35E5 | VK1|O12/ JK5 | ....I.......<br>...........Y | ...........<br>......... | ......A.<br>....H.. | G.......<br>....F.. | W..........S.....<br>....I.M..G.Y.... | H...N.............<br>..............L... | ......<br>.... |
| iPS:39 8536 | 21-225_33D12 | VK1|O12/ JK5 | .V..........<br>..L........ | ......R..<br>........ | ........<br>..N.... | S.......<br>....... | ....................<br>.................N.. | ..................<br>..............I... | ......<br>.... |
| **Germline** | **VK3|A27/JK 2** | | EIVLTQSPGILS LSPGERATLSC | RAS--QSVSS----SYLA | WYQQKPGQ APRLLIY | G-ASSRAT | GIPDRFSGSGSG TDFTLTISRLEPEDFAV YYC | QQYGS---------SPYT | FGQGTK LEIK |
| iPS:43 4127 | 21-225_53H8 | VK3|A27/ JK2 | ...........<br>........... | ......IT..<br>........ | ........<br>........ | ........<br>...G... | ....................<br>.................... | ..FE.............<br>.........SPMCS | ......N<br>.... |
| iPS:43 6290 | 21-225_205G3 | VK3|A27/ JK2 | ...........<br>........... | .....N..Y.<br>........ | ........<br>........ | ........<br>...R... | ....................<br>.................... | ...................<br>...............CS | ......<br>.... |
| iPS:43 6568 | 21-225_225B3 | VK3|A27/ JK2 | ...........<br>........... | ......NL...<br>......G | ........<br>........ | D.......<br>.T..... | ....................<br>.................... | .E...............<br>.........SLMCS | ......<br>.... |
| iPS:39 2898 | 21-225_21H10 | VK3|A27/ JK2 | ...........<br>........... | ......F...<br>........ | ........<br>........ | ........<br>........ | ....................<br>.................... | ....-<br>...................<br>......SRS | ......<br>.... |
| iPS:39 3802 | 21-225_3D12 | VK3|A27/ JK2 | ...........<br>........... | ......<br>...... | ........<br>........ | ........<br>.T..... | ....................<br>.................... | ....-<br>...................<br>......SRS | ......<br>.... |
| **Germline** | **VK1|L1/JK4** | | DIQMTQSPSSLS ASVGDRVTIIC | RAS--QGIS----NYLA | WFQQKPGK APKSLIY | A-------ASSLQS | GVPSRFSGSGSG-- TDFTLTISSLQPEDFAT YYC | QQYNS-----------YPLT | FGGGTK VEIK |
| iPS:43 4157 | 21-225_55D4 | VK1|L1/J K4 | ...........F<br>........... | ......D....<br>.......I | ........<br>....... | T.......<br>....... | ....K.....F......<br>.....N.......... | ...H.............<br>..........F... | ......R<br>...R |
| iPS:43 4175 | 21-225_55A11 | VK1|L1/J K4 | ...........<br>........... | .....D.N..<br>....I... | ........<br>....... | ........<br>....... | ....K...........<br>.................... | ...................<br>................... | ......<br>.... |

| | | | | | | | | | |
|---|---|---|---|---|---|---|---|---|---|
| iPS:43 4367 | 21-225_65H11 | VK1\|L1/J K4 | . . . . . . . . . . . .<br>. . . . . . . . . . . | . . . . . . D . . . .<br>. . . . T . . . | . . . . . . . .<br>. . . . . . . | . . . . . . . .<br>. . . . . . . | . . . . K . . . . . . . . . . . .<br>. . . . . . . . . . . . . . . . . . | . . . . . . . . . . . . . . . . . . . . . . .<br>. . . . . . . . . . . . . . . . F . . . | . . . . .<br>. . . . |
| iPS:43 4429 | 21-225_70H6 | VK1\|L1/J K4 | . . . . . . . . . . . .<br>. . L . . . . . . . . | . T . . . . S . F . .<br>. . . . . . . N | . . . R . . . .<br>. . . V . . . | T . . . . . . .<br>. . . . . . . | . I . . . . . . . . . . . . . . .<br>. . . . . . . . . . . S . . . . | . . SY . . . . . . . . . . . . . . . . .<br>. . . . . . . . . . . I . . . | . . . .<br>. . . . |
| iPS:43 4535 | 21-225_74C8 | VK1\|L1/J K4 | . . . . . . . . . . . .<br>. . . . . . . . . . . | . . . . . . . G . .<br>. . . K . . . | . . . . . . . .<br>. . . . . . . | T . . . . . . .<br>. T . N . . . | . A . . K . . . . . . . . . . . .<br>. . . . . . . . Y . . . . . . . | . . . SN . . . . . . . . . . . . . . . .<br>. . . . . . . . . . . . . . . . | . . . . . .<br>. . N |
| iPS:43 4573 | 21-225_77E6 | VK1\|L1/J K4 | . . . . . . . . . . . .<br>. . . . . . . . . . . | . . . . . . . . . . .<br>. . . . K . . . | . . . . . . . .<br>. . . . . . . | . . . . . . . .<br>. . . . . . G | . A . . K . . . . . . . . . . . .<br>. . . . . . . . . . . . . . . . . . | . . . SN . . . . . . . . . . . . . . . .<br>. . . . . . . . . . . . . . . . | . . . . R<br>. . . . |
| iPS:43 4615 | 21-225_76C5 | VK1\|L1/J K4 | . . . . . . . . . . . .<br>. . . . . . . . . . . | . . . . . V . . . .<br>. . . . K . . . | . . . . . . . .<br>. . . . . . . | . . . . . . . .<br>. . . . . . . | . A . . K . . . . . . . . . . . .<br>. . . . . . . . . . . . . . . . . . | . . . SN . . . . . . . . . . . . . . . .<br>. . . . . . . . . . . . . . . . | . . . . . .<br>. . . . |
| iPS:43 4669 | 21-225_79F4 | VK1\|L1/J K4 | . . . . . . . . . . . .<br>. . . . . . . . . . . | . . . . . . . . . . .<br>. . . . K . . . | . . . . . . . .<br>. . . . . . . | . . . . . . . .<br>. . . . . . G | . A . . K . . . . . . . . . . . .<br>. . . . . . . . . . . . . . . . . . | . . . SN . . . . . . . . . . . . . . . .<br>. . . . . . . . . . . . . . . . | . . . . R<br>. . . . |
| iPS:43 4737 | 21-225_74G6 | VK1\|L1/J K4 | . . . . . . . . . . . .<br>. . . . . . . . . . . | . . . . . . . G . .<br>. . . K . . . | . . . . . . . .<br>. . . . . . . | T . . . . . . .<br>. T . . . . | . A . . K . . . . . . . . . . . .<br>. . . . . . . . Y . . . . . . . | . . . SN . . . . . . . . . . . . . . . .<br>. . . . . . . . . . . . . . | . . . . . .<br>. . . N |
| iPS:43 4741 | 21-225_80C11 | VK1\|L1/J K4 | . . . . . . . . . . . .<br>. . . . . . . . . . . | . . . . . . . G . .<br>. . . R . . . | . . . . . . . R<br>. . . . . . . | T . . . . . . .<br>. . . . . . . | . A . . K . . . . . . . . . . . .<br>. . . . . . . . . . . . . . . . . . | . . . SN . . . . . . . . . . . . . . . .<br>. . . . . . . . . . . . . . . . | . . . . . .<br>. . . N |
| iPS:43 4867 | 21-225_79A12 | VK1\|L1/J K4 | . . . . . . . . . . . .<br>. . . . . . . . . . . | . . . . . . V . . . .<br>. . . . K . . . | . . . . . . . .<br>. . . . . . . | . . . . . . . .<br>. . . . . . . | . A . . K . . . . . . . . . . . .<br>. . . . . . . . . . . . . . . . . . | . . . SN . . . . . . . . . . . . . . . .<br>. . . . . . . . . . . . . . . . | . . . .<br>. . . . |
| iPS:43 5333 | 21-225_147E9 | VK1\|L1/J K4 | . . . . . . . . . . . .<br>. . . . . . . . . . . | . . . . . . D . N . .<br>. . . . . . . . | . . . . . . . .<br>. . . . . V | . . . . . . . .<br>. . . . . . . | . . . . K . . . . . . . . . . . .<br>. . . . . . . . . . . . . . . . . . | . . . . . . . . . . . . . . . . . . . . . . .<br>. . . . . . . . . . . . . . . . | . . . .<br>. . . . |
| iPS:43 5409 | 21-225_150G8 | VK1\|L1/J K4 | . . . . . . . . . . . .<br>. . . . . . . . . . . | . . . . . . . . . . .<br>. . . . H . . . | . . . . . . . .<br>. . . . . . . | V . . . . . . .<br>. . . . . N | . . . . . . . . . . . . . . . . .<br>. . . . . . . . . . . . . . . . . . | . . . . N . . . . . . . . . . . . . . .<br>. . . . . . . . . . . . . . . . | . . . .<br>. . . . |
| iPS:43 5505 | 21-225_157C1 | VK1\|L1/J K4 | . . . . . . . . . . . .<br>. . I . . . I . L . . | . . . . . . . . . . .<br>. . . . . . . . | . . . R . . . .<br>. . . . . . . | . . . . . . . .<br>. . . . L . | . . . . K . . . . . . . . . . . .<br>. . . . . . . . . . . . . . . . . . | . . . . . . . . . . . . . . . . . . . . . . .<br>. . . . . . . . . F . F . | . . L .<br>. . L . |
| iPS:43 5595 | 21-225_160H4 | VK1\|L1/J K4 | . . . . . . . . . . . .<br>. . . . . . . . . . . | . . . . . . D . . . .<br>. . . . . . . V | . . . . L . . .<br>. . . . . . . | V . . . . . . .<br>. . . . . . . | . . . . . . . . . . . . . . . . .<br>. . . . . . . . . . . . . . . . . . | . . . . . . . . . . . . . . . . . . . . . . .<br>. . . . . . . . . . . . . . . . | . . . . .<br>. . . . |
| iPS:43 5639 | 21-225_163G8 | VK1\|L1/J K4 | . . . . . . . . . . . .<br>. . . . . . . . . . . | . . . . . . D . . . .<br>. . . . . . . V | . . . . . . . .<br>. . . . . . . | . . . . . . . .<br>. . . . L . | . . . . K . . . . . . . . . . . .<br>. . . . . . . . . . . . . . . . . . | . . . H . . . . . . . . . . . . . . .<br>. . . . . . . . . . . . . . . . | . A . .<br>. A . . |
| iPS:43 5653 | 21-225_166H12 | VK1\|L1/J K4 | . . . . . . . . . . . .<br>T . . . . . . . . . . | . . . . . . D . . . .<br>. . . . H . . . | . . . . . . . .<br>. . . . . . . | . . . . . . . .<br>. . . . . . . | . . . . K . . . . . . . . . . . .<br>. . . . . . . . . . . . . . . . . . | . . . . . . . . . . . . . . . . . . . . . . .<br>. . . . . . . . . . . . . . F . . . | . . . T<br>. . . T |
| iPS:43 5677 | 21-225_169C10 | VK1\|L1/J K4 | . . . . . . . . . . . .<br>. . . . . . . . . . . | . . . . . . D . . . .<br>. . . . . . . . | . . . . . . . .<br>. . . . . F | S . . . . . . .<br>. . . . . . . | . . . . K . . . . . . . . . . . .<br>N . . . . . . . . . . . . . . . . . | . . SD . . . . . . . . . . . . . . . .<br>. . . . . . . . . . . . . . . . | . . . .<br>. . . . |
| iPS:43 5699 | 21-225_170D6 | VK1\|L1/J K4 | . . . . . . . . . . . .<br>. . . . . . A . . . | . . . . . . D . G . .<br>. . . . C . . . | . . . . . . . .<br>. . . . . . . | S . . . . . . .<br>. . . . . . . | . . . . K . . . . . . . . . . . .<br>. . . . . . . . . . . . . . . . . . | . . SD . . . . . . . . . . . . . . . .<br>. . . . . . . . . . . . . . . . | . . . .<br>. . . . |
| iPS:43 5745 | 21-225_175G3 | VK1\|L1/J K4 | . . . . . . . . . . . .<br>. . . . . . . . . . . | . . . . . . D . . . .<br>. . . . . D . . | . . . . . . . .<br>. . . . . F | S . . . . . . .<br>. . . . . . . | . . . . K . . . . . . . . . . . .<br>. . . . . . . . . . . . . . . . . . | . . SD . . . . . . . . . . . . . . . .<br>. . . . . . . . . . . . . . . . | . . . .<br>. . . . |
| iPS:43 5819 | 21-225_190C11 | VK1\|L1/J K4 | . . . . . . . . . . . .<br>. . . . . . . . . . . | . T . . . . . G . .<br>. . . . . . . . | . . . . . . . .<br>. . . . L . | K . . . . . . .<br>. T . . . . | . . . . K . . . . . . . . . . . .<br>. . . . . . . . . . . . . . . . . . | . . . MT . . . . . . . . . . . . . .<br>. . . . . . . . . . . . . . . . | . . . .<br>. . . . |
| iPS:43 5825 | 21-225_190G11 | VK1\|L1/J K4 | . . . . . . . . . . . .<br>. . . . . . . . . . . | . T . . . . . G . .<br>. . . . . . . . | . . . . . . . .<br>. . . . L . | K . . . . . . .<br>. . . . . . . | . . . . K . . . . . . . . . . . .<br>. . . . . . . . . . . . . . . . . . | . . . MT . . . . . . . . . . . . . .<br>. . . . . . . . . . . . . . . . | . . . .<br>. . . . |
| iPS:43 5837 | 21-225_198G3 | VK1\|L1/J K4 | . . . . A . . . . . . .<br>. . . . . . . . . . . | . T . . . . . G . .<br>. . . . K . . . | . . . . . . . .<br>. . . . . L . | K . . . . . . .<br>. . . . . . G | . . . . K . . . . . . . . . . . .<br>. . . . . . . . . . . . . . . . . . | . . . MT . . . . . . . . . . . . . .<br>. . . . . . . . . . . . . . . . | . . . .<br>. . . . |

| iPS:43 5845 | 21-225_191G1 | VK1\|L1\|JK4 | . . . . . . . . . . . .<br>. . . . . . . . . . | . . . . . . . . . .<br>. . . . . . . | . . . . . . . .<br>. . . . . . . | . . . . . . . .<br>. . . . . . . | . . . . K. . . . . . . . . . . . . .<br>. . . . . . . . . . . . . . . . . | .H.LT. . . . . . . . . . . . . . .<br>. . . . . . . . . . . . . . | . . . . .<br>. . . . |
| iPS:43 5859 | 21-225_190E6 | VK1\|L1\|JK4 | . . . . . . . . . . . .<br>. . . . . . . . . . | .T. . . . . .G. .<br>. . . . . . . | . . . . . . . .<br>. . . . L. | K. . . . . . .<br>. . . . . . . | . . . . K. . . . . . . . . . . . . .<br>. . . . . . . . . . . . . . . . . | . . .MT. . . . . . . . . . . . . .<br>. . . . . . . . . . . . . . | . . . . .<br>. . . . |
| iPS:43 5873 | 21-225_190G4 | VK1\|L1\|JK4 | . . . . . . . . . . . .<br>. . . . . . . . . . | . . . . . .D.G. .<br>. . . .R. . . | . . . . . . . .<br>. . . . . . . | . . . . . . . .<br>. . . . . . . | . . . . K. . . . . . . . . . . . . .<br>. . . . . . . . . . .N. . . . . | . . .ST. . . . . . . . . . . . . .<br>. . . . . . . . . . . . . . | . . . . .<br>. . . . |
| iPS:43 5933 | 21-225_190F8 | VK1\|L1\|JK4 | . . . . . . . . . . . .<br>. . . . . . . . . . | .T. . . . . .G. .<br>. . . . . . . | . . . . . . . .<br>. . . . . L. | K. . . . . . .<br>. . . . . . . | . . . . K. . . . . . . . . . . . . .<br>. . . . . . . . . . . . . . . . . | . . .MT. . . . . . . . . . . . . .<br>. . . . . . . . . . . . . . | . . . . .<br>. . . . |
| iPS:43 5945 | 21-225_191A10 | VK1\|L1\|JK4 | . . . . . . . . . . . .<br>. . . . . . . . . . | . . . . . . . .G. .<br>. . . .K. . . | . . . . . . . .<br>. . . . . . . | . . . . . . . .<br>. . . . . . . | . . . . K. . . . .Y. . . . .<br>. . . . . . . . . .N. .I. . . | . . .ST. . . . . . . . . . . . . .<br>. . . . . . . . . . . . . . | . . . . .<br>. . . . |
| iPS:43 5947 | 21-225_191E10 | VK1\|L1\|JK4 | . . . . . . . . . . . .<br>. . . . . . . . . . | . . . . . . . .G. .<br>. . . .K. . . | . . . . . . . .<br>. . . . . . . | . . . . . . . .<br>. . . . . . . | . . . . K. . . . . . . . . . . . . .<br>. . . . . . . . . . .N. . . . . | . . .ST. . . . . . . . . . . . . .<br>. . . . . . . . . . . . . . | . . . . .<br>. . . . |
| iPS:43 5957 | 21-225_191G12 | VK1\|L1\|JK4 | . . . . . . . . . . . .<br>. . . . . . . . . . | .T. . . . . .G. .<br>. . . . . . . | . . . . . . . .<br>. . . . L. | K. . . . . . .<br>. . . . . . . | . . . . K. . . . . . . . . . . . . .<br>. . . . . . . . . . . . . . . . . | . . .IT. . . . . . . . . . . . . .<br>. . . . . . . . . . . . . . | . . . .S.<br>. . . . |
| iPS:43 5963 | 21-225_192D2 | VK1\|L1\|JK4 | . . . .I. . . . . . .<br>. . . . . . . . . . | . . . . . . . . . .<br>. . . . . . . | . . . . . . . .<br>. . . . . . . | . . . . . . . .<br>. . . . . . . | . . . . K. . . . . . . . . . . . . .<br>. . . . . . . . . . . . . . . . . | .H.VT. . . . . . . . . . . . . .<br>. . . . . . . . . . . .N. | . . . . . .<br>. . . . |
| iPS:43 5971 | 21-225_192D3 | VK1\|L1\|JK4 | . . . . . . . . . . . .<br>. . . . . . . . . . | . . . . . . . . . .<br>. . . . . . . | . . . . . . . .<br>. . . . . . . | . . . . . . . .<br>. . . . . . . | . . . . K. . . . . . . . . . . . . .<br>. . . . . . . . . . . . . . . . . | LH.LT. . . . . . . . . . . . . .<br>. . . . . . . . . . . . . . | . . . . .<br>. . . . |
| iPS:43 5979 | 21-225_192H4 | VK1\|L1\|JK4 | . . . . . . . . . . . .<br>. . . . . . . . . . | . . . . . .D. . . .<br>. . . . . . . | . . . . . . . .<br>. . . .S | . . . . . . . .<br>. . . . . . . | . . . . K. . . .R. . . . . . . .<br>. . . . . . . . . . . . . . . .G | LH.LN. . . . . . . . . . . . . .<br>. . . . . . . . . . . . . . | . . . . .R<br>. . .R |
| iPS:43 5987 | 21-225_192G6 | VK1\|L1\|JK4 | . . K. . . . . . . .<br>. . . . . . . . . . | .T. . . . . .G. .<br>. . . . . . . | . . . . . . . .<br>. . . . . L. | K. . . . . . .<br>. . . . . . . | . . . . K. . . . . . . . . . . . . .<br>. . . . . . . . . . . . . . . . . | . . .MT. . . . . . . . . . . . . .<br>. . . . . . . . . . . . . . | . . . . .<br>. . . . |
| iPS:43 5993 | 21-225_192C8 | VK1\|L1\|JK4 | . . . . . . . . . . . .<br>. . . . . . . . . . | . . . . . . . . . .<br>. . . . . . . | . . . . . . . .<br>. . . . . . . | . . . . . . . .<br>. . . . . . . | . . . . K. . . . . . . . . . . . . .<br>. . . . . . . . . . . . . . . . . | .H.LT. . . . . . . . . . . . . .<br>. . . . . . . . . . . . . . | . . . . .<br>. . . . |
| iPS:43 5997 | 21-225_192G8 | VK1\|L1\|JK4 | . . . . . . . . . . . .<br>. . . . . . . . . . | .T. . . . . .G. .<br>. . . . . . . | . . . . . . . .<br>. . . . . L. | K. . . . . . .<br>. . . . . . . | . . . . K. . . . . . . . . . . . . .<br>. . . . . . . . . . . . . . . . . | . . .IT. . . . . . . . . . . . . .<br>. . . . . . . . . . . . . . | . . . . .<br>. . . . |
| iPS:43 6005 | 21-225_192H10 | VK1\|L1\|JK4 | . . . . . . . . . . . .<br>. . . . . . . . . . | . . . . . . . .G. .<br>. . . .K. . . | . . . . . . . .<br>. . . . . . . | . . . . . . . .<br>. . . . . . . | . . . . K. . . . .Y. . . . . .<br>. . . . . . . . . .N. . . . | . . .ST. . . . . . . . . . . . . .<br>. . . . . . . . . . . . . . | . . . . .<br>. . . . |
| iPS:43 6031 | 21-225_193C7 | VK1\|L1\|JK4 | . . . . . . . . . . . .<br>. . . . . . . . . . | . . . . . . . .G. .<br>. . . .K. . . | . . . . . . . .<br>. . . . . . . | . . . . . . . .<br>. . . . . . . | . . . . K. . . . . . . . . . . . . .<br>. . . . . . . . . . . . . . . . . | . . .ST. . . . . . . . . . . . . .<br>. . . . . . . . . . . . . . | . . . . .<br>. . . . |
| iPS:43 6045 | 21-225_193A10 | VK1\|L1\|JK4 | . . . . . . . . . . . .<br>. . . . . . . . . . | . . . . . . . . . .<br>. . . . . . . | . . . . . . . .<br>. . . . . . . | . . . . . . . .<br>. . . . . . . | . . . . K. . . . . . . . . . . . . .<br>. . . . . . . . . . . . . .F. | .H.LT. . . . . . . . . . . . . .<br>. . . . . . . . . . . . . . | . . . . .<br>. . . . |
| iPS:43 6054 | 21-225_194C1 | VK1\|L1\|JK4 | . . . . . . . . . . . .<br>. . . . . . . . . . | . . . . . . . . . .<br>. . . . . . . | . . . . . . . .<br>. . . . . . . | . . . . . . . .<br>. . . . . . . | . . . . K. . . . . . . . . . . . . .<br>. . . . . . . . . . . . . . . . . | LH.LT. . . . . . . . . . . . . .<br>. . . . . . . . . . . . . . | . . . . .<br>. . . . |
| iPS:43 6076 | 21-225_194H11 | VK1\|L1\|JK4 | . . . . . . . . . . . .<br>. . . . . . . . . . | . . . . . . . . . .<br>. . . . . . . | . . . . . . . .<br>. . . . . . . | . . . . . . . .<br>. . . . . . . | . . . . K. . . . . . . . . . . . . .<br>. . . . . . . . . . . . . . . . . | .H.LT. . . . . . . . . . . . . .<br>. . . . . . . . . . . . . . | . . . . .<br>. . . . |
| iPS:43 6086 | 21-225_191G10 | VK1\|L1\|JK4 | . . . . . . . . . . . .<br>. . . . . . . . . . | .T. . . . . .G. .<br>. . . .K. . . | . . . . . . . .<br>. . . . . L. | K. . . . . . .<br>.V. . . . . | . . . . K. . . . . . . . . . . . . .<br>. . . . . . . . . . . . . . . . . | . . .MT. . . . . . . . . . . . . .<br>. . . . . . . . . . . . . . | . . . . .<br>. . . . |
| iPS:43 6090 | 21-225_195A9 | VK1\|L1\|JK4 | . . . . . . . . . . . .<br>. . . . . . . . . . | . . . . . . . . . .<br>. . . . . . . | . . . . . . . .<br>. . . . . . . | . . . . . . . .<br>. . . . . . . | . . . . K. . . . . . . . . . . . . .<br>. . . . . . . . . . . . . . . . . | .H.LT. . . . . . . . . . . . . .<br>. . . . . . . . . . . . . . | . . . . .<br>. . . . |
| iPS:43 6112 | 21-225_196C7 | VK1\|L1\|JK4 | . . . . . . . . . . . .<br>. . . . . . . . . . | . .N. . .A. . . .<br>. . . . . . . | . . . . . . . .<br>. . . . . . . | . . . . . . . .<br>. . . . . . . | . . . . K. . . . . . . . . . . . . .<br>. . . . . . . . . . . . . . . . . | .H.LT. . . . . . . . . . . . . .<br>. . . . . . . . . . . . . . | . . . . .<br>. . . . |

| iPS:43 6138 | 21-225_197F2 | VK1\|L1/J K4 | ....¯........ | .¯......G.. | ........ | K...... | ....K............ | ...¯T............ | .... |
| | | | ............ | ........ | .....L. | .T..... | ...............F | ............... | .... |
| iPS:43 6152 | 21-225_197B6 | VK1\|L1/J K4 | ............ | .......G.. | ........ | G...... | ....K............ | ...ST........... | .... |
| | | | ............ | ....K... | ........ | ...... | .........N..... | ............... | .... |
| iPS:43 6173 | 21-225_197G12 | VK1\|L1/J K4 | ....I....... | .T......G.. | ........ | K...... | F..K............ | ...MT........... | .... |
| | | | ............ | ........ | .....L. | .T..... | ...............F | ............... | .... |
| iPS:43 6189 | 21-225_198B6 | VK1\|L1/J K4 | ............ | .......G.. | ........ | ........ | ....K...NR...... | ...ST........... | .... |
| | | | ............ | ........ | ........ | ........ | ................ | ............... | .... |
| iPS:43 6201 | 21-225_199C5 | VK1\|L1/J K4 | ............ | ........ | ........ | ........ | ....K............ | ...LT........... | .... |
| | | | ............ | ....K... | ........ | ........ | ..............A... | ............... | .... |
| iPS:43 6260 | 21-225_203H1 | VK1\|L1/J K4 | ............ | .......G.. | ........ | V...... | ....K...T.....S.. | .R.HT........... | .... |
| | | | ............ | ........ | ........ | ...R... | ........D...... | ............... | .... |
| iPS:43 6282 | 21-225_204G6 | VK1\|L1/J K4 | ..R......... | .T......G.. | ........ | ........ | ................ | .H.L............ | .... |
| | | | ......I.... | ........ | ........ | ........ | ................ | ............... | .... |
| iPS:43 6284 | 21-225_204G8 | VK1\|L1/J K4 | ............ | .....H.. | ......F | ........ | ....Q..........E. | ...SN........... | .... |
| | | | ............ | .....H.. | ......F | ........ | ................ | ...........V. | .... |
| iPS:43 6292 | 21-225_205H3 | VK1\|L1/J K4 | ..P......... | ......A.... | ..L.... | ........ | ....K.........S.. | ...SN........... | .... |
| | | | ............ | .....H.. | ........ | ........ | ................ | ............... | .... |
| iPS:43 6296 | 21-225_205F5 | VK1\|L1/J K4 | ............ | .......G.. | ........ | G...... | ....K............ | ...SN........... | .... |
| | | | ............ | ........ | ........ | .V.... | ...............T. | ............... | .D.R |
| iPS:43 6324 | 21-225_207G6 | VK1\|L1/J K4 | ............ | ........R.. | .L.... | ........ | ....K...NR...... | ...SN........... | .... |
| | | | ............ | ........ | ......H | ........ | ................ | ............... | .... |
| iPS:43 6364 | 21-225_211A11 | VK1\|L1/J K4 | ............ | .......G.. | ........ | D...... | ....K....R...... | .H.MT............ | ..A... |
| | | | ............ | ........ | ..R.... | .....E. | ....G............ | ............... | .... |
| iPS:43 6366 | 21-225_211A3 | VK1\|L1/J K4 | ..R......... | ......A.G.. | ....R... | ........ | ....K............ | ...SN........... | .... |
| | | | ............ | ....KH.. | ........ | ...R... | ..........L..... | ............... | .... |
| iPS:43 6372 | 21-225_211A8 | VK1\|L1/J K4 | ..E.....P... | ............ | .V..... | ........ | ..S............. | LR.DT........... | .... |
| | | | .F......... | ....R... | ........ | ........ | ................ | .............I | .... |
| iPS:43 6376 | 21-225_212E6 | VK1\|L1/J K4 | ............ | ............ | .......Q | ........ | ....K....R...... | ...VT........... | .... |
| | | | ............ | .....H.. | ........ | ........ | ................ | ............... | .... |
| iPS:43 6378 | 21-225_212D7 | VK1\|L1/J K4 | ............ | ............ | ........ | ........ | ....K...NR...... | ...SN........... | .... |
| | | | ............ | .....H.. | ......H | ........ | ....NN......V.... | ............... | .... |
| iPS:43 6380 | 21-225_212H9 | VK1\|L1/J K4 | ..E......... | ............ | .L..... | ........ | ................ | LR.DT........... | .... |
| | | | ............ | ....S... | ........ | ........ | ................ | ............... | .... |
| iPS:43 6384 | 21-225_212F10 | VK1\|L1/J K4 | ............ | ............ | ........ | S...... | ....K....R...... | .H.SN........... | .... |
| | | | ............ | .......D | ........ | ...N... | ................ | ............... | .... |
| iPS:43 6388 | 21-225_212H11 | VK1\|L1/J K4 | ..R......... | ......A.G.. | ....R... | ........ | ....K............ | .H.SN........... | .V.... |
| | | | ............ | ....KH.. | ........ | ...R... | ..........L..... | ............... | ...T |
| iPS:43 6390 | 21-225_213D2 | VK1\|L1/J K4 | ............ | ............ | ........ | ........ | ....K...NR...... | H..SN........... | .... |
| | | | ............ | .....H.. | ......H | ........ | ....NN......V.... | ............... | .... |
| iPS:43 6394 | 21-225_213C4 | VK1\|L1/J K4 | ..H......... | ......A.R.. | .C..... | ........ | ....K............ | ...SN........... | .... |
| | | | ............ | ........ | ...T... | ........ | ................ | ............... | .... |

3106

| | | | | | | | | |
|---|---|---|---|---|---|---|---|---|
| iPS:43 6396 | 21-225_213E5 | VK1\|L1/J K4 | ..R......... | ......A.G.. | ....R... | ........ | ....K........... | .H.SN............ | 21- |
| | | | ............ | ....KH.. | ........ | ...R... | ...........L..... | ............. | .... |
| iPS:43 6398 | 21-225_213B8 | VK1\|L1/J K4 | ............ | ......H.. | ........ | ........ | ....K........... | ...SN............ | 21- |
| | | | ............ | .....H.. | ........ | ........ | ................ | ............. | .... |
| iPS:43 6410 | 21-225_212E10 | VK1\|L1/J K4 | ............ | ......H.. | ........ | ........ | ....K........... | ...SN............ | 21- |
| | | | ............ | .....H.. | ........ | ........ | ................ | ............. | .... |
| iPS:43 6420 | 21-225_215B5 | VK1\|L1/J K4 | ............ | ......H.. | ........ | ........ | ....K...NR...... | ...IN............ | 21- |
| | | | ............ | .....H.. | ......H | ........ | ....NN......V.... | ............. | .... |
| iPS:43 6422 | 21-225_215D6 | VK1\|L1/J K4 | ............ | ............ | ........ | ........ | ....K....R...... | ...VT............ | 21- |
| | | | ............ | .....H.. | ........ | .....H. | ................ | ............. | .... |
| iPS:43 6430 | 21-225_215A12 | VK1\|L1/J K4 | ............ | .T....D.G.. | ........ | ........ | ....K....R...... | ...VT............ | 21- |
| | | | ......I.... | ........ | ........ | ........ | ........S........ | ............. | .... |
| iPS:43 6452 | 21-225_217G5 | VK1\|L1/J K4 | ..L......... | ........G.. | ....R... | ........ | ....K...TR...... | ...VN............ | 21- |
| | | | ............ | ........ | ........ | ........ | ...........D..... | ............. | ...N |
| iPS:43 6454 | 21-225_217B10 | VK1\|L1/J K4 | ..R......... | ......A.G.. | ....R... | ........ | ....K........... | .HTSK............ | LV.... |
| | | | ............ | ....KH.. | ........ | ...R... | ......V....L....R | ...........S.VQ | ...T |
| iPS:43 6464 | 21-225_219H1 | VK1\|L1/J K4 | .........Q. | ............ | ........ | S....... | ....K....R...... | .H.SN............ | 21- |
| | | | ............ | ........D | ........ | ...N... | ................ | ............. | .... |
| iPS:43 6490 | 21-225_221F6 | VK1\|L1/J K4 | ............ | ......... | ........ | ........ | ....K....R...... | ...MT............ | ....R |
| | | | G........... | ......... | ........ | ...N... | ................ | ............. | .... |
| iPS:43 6502 | 21-225_222A11 | VK1\|L1/J K4 | ............ | ......... | ........ | ........ | ....K........... | LY.LN............ | 21- |
| | | | ............ | ......... | ........ | ........ | ................ | ............. | .... |
| iPS:43 6514 | 21-225_222D10 | VK1\|L1/J K4 | ............ | ......... | ........ | ........ | ....K........... | LH.LN............ | ....R |
| | | | ............ | ......... | ........ | ........ | ................ | ............. | .... |
| iPS:43 6522 | 21-225_223H10 | VK1\|L1/J K4 | ............ | ......... | ........ | ........ | ....K........... | LH.LN............ | 21- |
| | | | ............ | ......... | ........ | ...N... | ................ | ............. | .... |
| iPS:43 7258 | 21-225_153F9 | VK1\|L1/J K4 | ............ | ......... | ........ | ........ | ....K........... | ................ | 21- |
| | | | ............ | ......... | ......S | ........ | ................ | .............S | .... |
| iPS:43 7260 | 21-225_170D1 | VK1\|L1/J K4 | ...........A | ......D.... | ........ | ........ | ................ | ..CD............ | 21- |
| | | | ............ | ......... | ........ | ........ | ................ | ..........F... | .... |
| iPS:43 7264 | 21-225_171H12 | VK1\|L1/J K4 | ............ | ......D.... | ........ | S....... | ....K........... | ..SD............ | 21- |
| | | | ............ | ......... | ........ | ........ | ................ | ............. | .... |
| iPS:43 7266 | 21-225_177A5 | VK1\|L1/J K4 | ............ | ......D.... | ........ | S....... | ....K........... | ..SD............ | 21- |
| | | | ............ | ......... | ........ | ........ | ................ | ............. | .... |
| iPS:43 7270 | 21-225_178H4 | VK1\|L1/J K4 | ............ | ......D.... | ........ | S....... | ....K........... | ..S............. | 21- |
| | | | ............ | ......... | ......F | ........ | ....N........... | ............. | .... |
| iPS:43 8664 | 21-225_216G1 | VK1\|L1/J K4 | ..E......... | ............ | .L...... | ........ | ................ | LR.DT............ | 21- |
| | | | ............ | ....S... | ........ | ........ | ................ | ............. | .... |
| iPS:45 1120 | 21-225_197D3 | VK1\|L1/J K4 | ............ | ........R.. | ........ | ........ | ....K........... | .H.LT............ | 21- |
| | | | ............ | ......... | ........ | ........ | ................ | ............. | .... |
| iPS:39 2682 | 21-225_16A12 | VK1\|L1/J K4 | ............ | ......A.N.. | ........ | ........ | ....K.........E. | ...Y............ | 21- |
| | | | ............ | ....T... | ......S | ........ | ................ | ............. | .... |

| iPS | Clone | Germline | K_FR1 | K_CDR1 | K_FR2 | K_CDR2 | K_FR3 | K_CDR3 | K_FR4 |
|---|---|---|---|---|---|---|---|---|---|
| iPS:39 2856 | 21-225_22A2 | VK1\|L1/JK4 | ............ | ......D.... | .V...... | ........ | ....K......... | ................... | ..... |
| | | | ........... | ........ | ....... | ....... | ..............S... | ..........F... | .... |
| iPS:39 2966 | 21-225_32G3 | VK1\|L1/JK4 | ........T... | ......A.... | ........ | D...... | ....K......... | ...H............ | ..... |
| | | | ........... | ........ | ....... | .T..... | .....T | ............... | .... |
| iPS:39 3952 | 21-225_1F1 | VK1\|L1/JK4 | ............ | ........N.. | .....S.. | V...... | ................ | ................... | ..... |
| | | | ........... | ........ | ......S | ......T | ................ | ............... | .... |
| iPS:39 3988 | 21-225_7F10 | VK1\|L1/JK4 | ........A.. | ......D.R.. | ........ | V...... | ................ | ................... | ..... |
| | | | ........... | ........ | ......S | ........ | ................ | ............... | .... |
| iPS:40 2233 | 21-225_16D10 | VK1\|L1/JK4 | ............ | ......D.... | ........ | ........ | ....K.........E. | ................... | ..... |
| | | | ........... | ........ | ....... | .TP.... | ................ | ............... | .... |
| iPS:40 2237 | 21-225_23D11 | VK1\|L1/JK4 | ............ | ........A.. | ....R... | ........ | ....K.........E. | ...H............ | ....S. |
| | | | ........... | ........ | ....... | ......S | ................ | ............... | .... |
| **Germline** | | | **K_FR1** | **K_CDR1** | **K_FR2** | **K_CDR2** | **K_FR3** | **K_CDR3** | **K_FR4** |
| | VK1\|O18/JK5 | | DIQMTQSPSSLS ASVGDRVTIIC | QAS QDIS ----NYLN | WYQQKPGK APKLLIY | D -ASNLET | GVPSRFSGSGSG TDFTFTISSLQPEDIAT YYC | QQYDN ----------LPIT | FGQGTR LEIK |
| iPS:43 4171 | 21-225_50G4 | VK1\|O18/JK5 | ........T... | ........T.. | ........ | ........ | ................ | ....- | ..... |
| | | | ........... | .....F.. | ....... | ....... | ................ | .....NL.. | .... |
| iPS:43 5565 | 21-225_159C4 | VK1\|O18/JK5 | ............ | ........... | ........ | ........ | ................ | ............... | ..... |
| | | | ........... | ....D... | ....... | ...T... | ...........F. | ............... | .... |
| iPS:43 5607 | 21-225_161G4 | VK1\|O18/JK5 | ............ | .......Y.. | ........ | ........ | ................ | ....I........... | ..... |
| | | | ........... | ........ | ....... | ....... | ..A............. | ............... | .... |
| iPS:43 7302 | 21-225_225B11 | VK1\|O18/JK5 | ............ | .......F.. | ........ | ........ | ................ | ................... | ..... |
| | | | ..I........ | ........ | ....... | ...T... | ................ | ............... | .... |
| iPS:39 2868 | 21-225_24D6 | VK1\|O18/JK5 | ............ | ........N.. | ........ | ........ | ................ | ...E............ | ..... |
| | | | ........... | ........ | .L..... | ...D... | ............I... | ............... | .... |
| iPS:39 3020 | 21-225_30E2 | VK1\|O18/JK5 | .........H... | ......Y.... | .....S.. | ........ | ................ | ................... | ..... |
| | | | ........... | ........ | ....... | .G.S... | ..........L..... | ............... | .... |
| iPS:39 3138 | 21-225_35E3 | VK1\|O18/JK5 | ............ | .......F.. | ........ | ........ | ................ | ................... | ..... |
| | | | ........... | ........ | ..N.... | ....... | ...............F. | ............... | .D.R |
| iPS:39 3892 | 21-225_6G7 | VK1\|O18/JK5 | .........R. | ........... | .C...... | ........ | ................ | ................... | ..... |
| | | | ........... | ........ | .L..... | ...T... | ......V......... | ...........V... | .... |
| iPS:39 3910 | 21-225_15F10 | VK1\|O18/JK5 | ............ | ..N.....T.. | ...L.... | ........ | ................ | ................... | ..... |
| | | | ........... | .....F.. | ..N...S | ....... | ........V..... | ............... | .... |
| iPS:39 3912 | 21-225_16F6 | VK1\|O18/JK5 | ............ | ..N.....T.. | ...L.... | ........ | ................ | ................... | ..... |
| | | | ........... | .....F.. | ..N...S | ....... | ........V..... | ............... | .... |
| iPS:39 4000 | 21-225_11A2 | VK1\|O18/JK5 | ............ | ........... | ........ | ........ | ................ | ................... | ..... |
| | | | ........... | ........ | ....... | ....... | ........V..... | ............... | .D.. |
| iPS:39 4004 | 21-225_13A1 | VK1\|O18/JK5 | ............ | ........N.. | .....L.T | ........ | ................ | ...E............ | ..... |
| | | | ........... | ........ | ....... | .G..... | ................ | ............... | .... |
| iPS:39 4006 | 21-225_15C2 | VK1\|O18/JK5 | ............ | ........T.. | ........ | ........ | ................ | ................... | .A.... |
| | | | ........... | ........ | ..N.... | ....... | ................ | ............... | .... |

| iPS ID | Clone | Germline | K_FR1 | K_CDR1 | K_FR2 | K_CDR2 | K_FR3 | K_CDR3 | K_FR4 |
|---|---|---|---|---|---|---|---|---|---|
| iPS:39 4029 | 21-225_1B12 | VK1|O18/JK5 | ............. / ............. | ........,N.. / ........ | ........ / ........ | ........ / ........ | ................ / ............T.... | ...,E............. / .............. | .... / .... |
| iPS:39 4047 | 21-225_5E6 | VK1|O18/JK5 | ............. / ............. | ........N.. / ........ | .C...... / ........ | ........ / ........ | ................ / ................ | .............. / .............. | .... / .... |
| iPS:39 4081 | 21-225_16B3 | VK1|O18/JK5 | ............. / ............. | ........N.. / ........ | .......R / ........ | ........ / ........ | ................ / ................ | ..F........... / .............. | .... / .... |
| **Germline** | **VK3|L2/JK3** | | **K_FR1** EIVMTQSPATLS VSPGERATLSC | **K_CDR1** RAS--QSVS-- ----SNLA | **K_FR2** WYQQKPGQ APRLLIY | **K_CDR2** G------- -ASTRAT | **K_FR3** GIPARFSGSGSG-- TEFTLTISSLQSEDFAV YYC | **K_CDR3** QQYNN------------ --------WPFT | **K_FR4** FGPGTK VDIK |
| iPS:43 4219 | 21-225_60E9 | VK3|L2/JK3 | ............. / ............. | ............. / ......S.. | ..R..... / ........ | ........ / ........ | ................ / ..............C. | .............. / .............. | .... / I... |
| iPS:43 4279 | 21-225_57F7 | VK3|L2/JK3 | ........I.. / .F......... | ............. / .....D.. | ........ / ........ | ........ / ........ | .M......G........ / ..........H..... | ...S........... / .............. | .... / .... |
| iPS:43 4289 | 21-225_57H12 | VK3|L2/JK3 | ............. / ............. | ............. / .....D.. | ........ / ........ | A....... / ........ | ................ / ................ | ...D........... / .............. | .... / ..N. |
| iPS:43 4291 | 21-225_58A4 | VK3|L2/JK3 | ............. / ............. | ............. / .....D.. | ....R... / ........ | A....... / ........ | ................ / ................ | ..F........... / .............. | .... / .... |
| iPS:43 4297 | 21-225_58A10 | VK3|L2/JK3 | ............. / .C......... | ............. / .....S.. | ........ / ........ | ........ / ........ | ................ / ................ | .............. / .............. | .... / .... |
| iPS:43 4301 | 21-225_58F11 | VK3|L2/JK3 | ............. / ............. | ............. / .....D.V | ........ / ........ | .V..... / ........ | ................ / ................ | .............. / .............. | .... / .... |
| iPS:43 7228 | 21-225_60C11 | VK3|L2/JK3 | ............. / ............. | ............. / .....ND.. | ........ / ........ | ........ / ........ | .......G........ / .....A....H...... | ...S........... / .............. | .... / .... |
| **Germline** | **VK2|A19/JK5** | | **K_FR1** DIVMTQSPLSLP VTPGEPASISC | **K_CDR1** RSS--- QSLLHSN- GYNYLD | **K_FR2** WYLQKPGQ SPQLLIY | **K_CDR2** L------- -GSNRAS | **K_FR3** GVPDRFSGSGSG-- TDFTLKISRVEAEDVGV YYC | **K_CDR3** MQALQ------------ --------TPIT | **K_FR4** FGQGTR LEIK |
| iPS:43 4243 | 21-225_62C1 | VK2|A19/JK5 | ............. / ............. | ............. / ........ | ........ / .V.... | ........ / ........ | ................ / .......G.......F. | L............. / .............L. | .... / .... |
| iPS:43 6648 | 21-225_227F11 | VK2|A19/JK5 | .V.......... / ............. | W........ / ........ | ........ / ...V... | ........ / ........ | ................ / ................ | .............. / .............L. | .... / .... |
| iPS:39 4061 | 21-225_12D2 | VK2|A19/JK5 | ............. / ............. | ............. / ........ | ........ / ...V... | ........ / ........ | ................ / ................ | .............. / .............. | .... / .... |
| iPS:39 4071 | 21-225_10C7 | VK2|A19/JK5 | ............. / ............. | ............. / K...... | ........ / ...V... | ........ / ........ | ................ / ................ | .............. / .............L. | .... / .... |
| **Germline** | **VK1|L5/JK4** | | **K_FR1** DIQMTQSPSSVS ASVGDRVTIIC | **K_CDR1** RAS--QGIS-- ----SWLA | **K_FR2** WYQQKPGK APKLLIY | **K_CDR2** A------- -ASSLQS | **K_FR3** GVPSRFSGSGSG-- TDFTLTISSLQPEDFAT YYC | **K_CDR3** QQANS------------ --------FPLT | **K_FR4** FGGGTK VEIK |
| iPS:43 4259 | 21-225_62G7 | VK1|L5/JK4 | ............. / ........F.. | ......D.... / ........ | ....N... / ........ | ........ / ........ | ...............N. / ................ | ..T........... / .............. | .... / .... |

| ID | Clone | Germline | K_FR1 | K_CDR1 | K_FR2 | K_CDR2 | K_FR3 | K_CDR3 | K_FR4 |
|---|---|---|---|---|---|---|---|---|---|
| IPS:43 4333 | 21-225_63C9 | VK1\|L5/JK4 | ............. | .......... | ........ | ........ | ................ | ..I.............. | ...... |
| | | | ........... | ........ | ..N.... | ....... | .....G.........F. | ............... | .A.. |
| IPS:43 4347 | 21-225_64H10 | VK1\|L5/JK4 | ............. | .......... | ........ | ........ | ................ | ..I.............. | ...... |
| | | | ........... | ........ | .L..... | ....... | ................ | ............... | .... |
| IPS:43 4359 | 21-225_65G3 | VK1\|L5/JK4 | ............. | .......... | ........ | ........ | ................ | ..V.............. | ...... |
| | | | ........... | ........ | ........ | ....... | ..............F.. | ............... | .... |
| IPS:43 4369 | 21-225_66B1 | VK1\|L5/JK4 | ............. | .......... | ........ | ........ | ................ | ..T.............. | ...... |
| | | | ........... | ........ | .L..... | ....... | ................ | ............... | .... |
| IPS:43 4373 | 21-225_66A7 | VK1\|L5/JK4 | ............. | .......... | ........ | ........ | ................ | ..I.............. | ...... |
| | | | ........... | ........ | ........ | ....... | ................ | ............... | .... |
| IPS:43 4397 | 21-225_67H4 | VK1\|L5/JK4 | ............. | .......... | ........ | ........ | ................ | ..I.............. | ...... |
| | | | ........... | ........ | ........ | ....... | ................ | ............... | .... |
| IPS:43 4427 | 21-225_70D6 | VK1\|L5/JK4 | ............. | .......... | ....N... | ........ | ................ | ..T.............. | ...... |
| | | | ........... | ....K... | .L....F | ....... | ...........N... | ............... | .... |
| IPS:43 4435 | 21-225_70G9 | VK1\|L5/JK4 | ............. | ......D.... | ....N... | ........ | ................ | ..T.............. | ...... |
| | | | ........... | ........ | ........ | ....... | ................ | ............... | .... |
| IPS:43 4437 | 21-225_70A12 | VK1\|L5/JK4 | ............. | .......... | ........ | ........ | ................ | ..I.............. | ...... |
| | | | ........... | ........ | .L..... | ....... | .....V......... | ............... | .... |
| IPS:43 4451 | 21-225_71B7 | VK1\|L5/JK4 | ............. | .......... | ....N..E | ........ | ................ | ..T.............. | ...... |
| | | | ........... | ........ | ........ | .....G | .............N... | ............... | .... |
| IPS:43 4459 | 21-225_71A7 | VK1\|L5/JK4 | ............. | .......... | ........ | ........ | ................ | ..V.............. | ...... |
| | | | ........... | ........ | ........ | ....... | ................ | ............... | ..L. |
| IPS:43 4461 | 21-225_73A3 | VK1\|L5/JK4 | ............. | .......... | ........ | ........ | ............E.... | ..V.............. | ...... |
| | | | ..I........ | ....N... | ........ | ....... | ................ | ............... | .... |
| IPS:43 5479 | 21-225_154E9 | VK1\|L5/JK4 | .....L.....Y | ......D.... | ....R... | ........ | ................ | ..G.............. | ...... |
| | | | ........... | ....N... | ...V... | ....... | ........V....... | ............... | .D.. |
| IPS:43 7232 | 21-225_63E1 | VK1\|L5/JK4 | ...........Y | .......... | ........ | ........ | ................ | ............... | ...... |
| | | | ........... | ....Y.. | ........ | ....... | ................ | ............... | .... |
| IPS:43 7326 | 21-225_75C10 | VK1\|L5/JK4 | .......I... | ....I... | ........ | ........ | ...L............ | ...K........... | ...... |
| | | | ........... | ........ | ........ | ....... | ................ | ............... | .... |
| **Germline** | | | **K_FR1** | **K_CDR1** | **K_FR2** | **K_CDR2** | **K_FR3** | **K_CDR3** | **K_FR4** |
| | VK1\|O12/JK2 | | DIQMTQSPSSLS ASVGDRVTIIC | RAS--QSIS-- --SYLN | WYQQKPGK APKLLIY | A------- -ASSLQS | GVPSRFSGSGSG-- TDFTLTISSLQPEDFAT YYC | QQSYS------ ----------TFYT | FGQGTK LEIK |
| IPS:43 4309 | 21-225_59B5 | VK1\|O12/JK2 | .......... | ........I.. | ........ | G....... | ................ | ............... | ...... |
| | | | ........... | ........ | ........ | ....... | ................ | .........TPMFS | .... |
| IPS:39 2874 | 21-225_21D2 | VK1\|O12/JK2 | ..........F | .......... | .......R | D....... | ................ | ..T.NI......... | ..R... |
| | | | ........... | ....D... | ........ | .I..... | ....N........... | .........LPERS | .... |
| IPS:39 3940 | 21-225_16B2 | VK1\|O12/JK2 | GV........ | .......... | ........ | ........ | ........G....... | ..T.NT......... | ...... |
| | | | ........... | ....G... | ........ | ....... | ................ | .........PPERS | .... |
| IPS:39 3956 | 21-225_4D7 | VK1\|O12/JK2 | .......... | .......... | ........ | D....... | ................ | ..T.NT......... | ...... |
| | | | ........... | ....D... | ......F | .TT.... | ....N........... | .........PPERS | .... |

| | | Germline | K_FR1 | K_CDR1 | K_FR2 | K_CDR2 | K_FR3 | K_CDR3 | K_FR4 |
|---|---|---|---|---|---|---|---|---|---|
| iPS:39 8476 | 21-225_17C1 | VK1\|O12/ JK2 | ............<br>............ | ......N.N..<br>....D... | ........<br>....... | ........<br>...N... | ...A.....R.......<br>................ | ..T.NT............<br>.........PPERS | ......<br>.... |
| iPS:40 3870 | 21-225_23G4 | VK1\|O12/ JK2 | ............<br>........ | ......N.Y..<br>........ | ........<br>....... | ........<br>....... | ..............E.<br>......I......... | ....NT............<br>.........PPECN | ......<br>.... |
| | | **Germline** | **K_FR1** | **K_CDR1** | **K_FR2** | **K_CDR2** | **K_FR3** | **K_CDR3** | **K_FR4** |
| | **VK3\|A27/JK3** | | EIVLTQSPGTLS<br>LSPGERATLSC | RAS--QSVSS-<br>----SYLA | WYQQKPGQ<br>APRLLIY | G-------<br>-ASSRAT | GIPDRFSGSGSG--<br>TDFTLTISRLEPEDFAV<br>YYC | QQYGS-------------<br>----------SPFT | FGPGTK<br>VDIK |
| iPS:43 4311 | 21-225_59H5 | VK3\|A27/ JK3 | ............<br>............ | ............<br>....I... | .FL.....<br>........ | ........<br>....... | ................<br>................ | H...N............<br>.............. | ......<br>..F. |
| iPS:43 5301 | 21-225_146G4 | VK3\|A27/ JK3 | ............<br>.F........ | ......NII..<br>........ | ........<br>......F | ........<br>.V..... | ................<br>................ | ....R............<br>............N | ......<br>...N |
| iPS:43 5317 | 21-225_147D2 | VK3\|A27/ JK3 | ............<br>............ | ........G..<br>........ | ........<br>........ | ........<br>........ | | ....-<br>......L.. | ......<br>...... |
| iPS:43 5319 | 21-225_147E3 | VK3\|A27/ JK3 | ............<br>............ | ..........I..<br>........ | ........<br>........ | ........<br>........ | A...............<br>................ | ....R............<br>............N | ......<br>.... |
| iPS:43 5383 | 21-225_149D7 | VK3\|A27/ JK3 | ............<br>.F....V.... | .......II..<br>....N... | ........<br>......F | ........<br>.V..... | ................<br>................ | ....R............<br>............N | ......<br>.... |
| iPS:43 5443 | 21-225_152E7 | VK3\|A27/ JK3 | ............<br>.F........ | ........I..<br>........ | ........<br>......F | ........<br>.V..... | ................<br>................ | ....R............<br>............N | ......<br>.... |
| iPS:43 5465 | 21-225_153A6 | VK3\|A27/ JK3 | ............<br>.F.....P... | ........I..<br>........ | ........<br>......F | ........<br>.V..... | ................<br>................ | ....R............<br>............N | ......<br>.... |
| iPS:43 6058 | 21-225_194A4 | VK3\|A27/ JK3 | ............<br>............ | ......RG..N.<br>....I... | ........<br>....... | ........<br>...N... | ................<br>................ | .HNDY............<br>............M.. | ......<br>.... |
| iPS:43 6436 | 21-225_216F10 | VK3\|A27/ JK3 | .V........<br>............ | ............<br>....F... | ........<br>....... | ........<br>.T.T... | I...............<br>................ | ...DR............<br>.............. | ......<br>.... |
| iPS:44 2568 | 21-225_149D8 | VK3\|A27/ JK3 | ............<br>.F........ | ..........I..<br>........ | ........<br>......F | ........<br>.V..W.. | ................<br>................ | ....R............<br>............N | ......<br>.... |
| | | **Germline** | **K_FR1** | **K_CDR1** | **K_FR2** | **K_CDR2** | **K_FR3** | **K_CDR3** | **K_FR4** |
| | **VK1\|O18/JK1** | | DIQMTQSPSSLS<br>ASVGDRVTITC | QAS--QDIS--<br>----NYLN | WYQQKPGK<br>APKLLIY | D-------<br>-ASNLET | GVPSRFSGSGSG--<br>TDFTFTISSLQPEDIAT<br>YYC | QQYDN-------------<br>----------LPWT | FGQGTK<br>VEIK |
| iPS:43 4353 | 21-225_64B12 | VK1\|O18/ JK1 | ............<br>............ | R..........<br>........ | .......T<br>..N...S | ........<br>...I... | ....T...........<br>................ | ..S..............<br>...........CS | ......<br>.... |
| | | **Germline** | **K_FR1** | **K_CDR1** | **K_FR2** | **K_CDR2** | **K_FR3** | **K_CDR3** | **K_FR4** |
| | **VK1\|A20/JK4** | | DIQMTQSPSSLS<br>ASVGDRVTITC | RAS--QGIS--<br>----NYLA | WYQQKPGK<br>VPKLLIY | A-------<br>-ASTLQS | GVPSRFSGSGSG--<br>TDFTLTISSLQPEDVAT<br>YYC | QKYNS-------------<br>----------APLT | FGGGTK<br>VEIK |
| iPS:43 4357 | 21-225_65C1 | VK1\|A20/ JK4 | ...L........<br>............ | ......V....<br>....S..H | ........<br>...V... | S.......<br>...N..C | ................<br>...F.........G | .RPYN............<br>.............. | ......<br>.... |
| iPS:43 4375 | 21-225_66C7 | VK1\|A20/ JK4 | ...L........<br>..........R | ......V....<br>.......H | ........<br>A...... | C.......<br>...N..C | ................<br>................ | .QH.N............<br>..........S... | ......<br>.... |

| | | | K_FR1 | K_CDR1 | K_FR2 | K_CDR2 | K_FR3 | K_CDR3 | K_FR4 |
|---|---|---|---|---|---|---|---|---|---|
| IPS:43 4457 | 21-225_72G12 | VK1|A20/ JK4 | ...L......... / ........... | ........... / ....S..N | .S...... / ......C | G...... / ...N... | .........A.....E. / I.............T...G | .QNYN........... / ........... | ...... / .... |
| | | Germline | **K_FR1** | **K_CDR1** | **K_FR2** | **K_CDR2** | **K_FR3** | **K_CDR3** | **K_FR4** |
| | VK3|A27/JK 4 | | EIVLTQSPGTLS LSPGERATLSC | RAS--QSVSS- ----SYLA | WYQQKPCQ APRLLIY | G------- -ASSRAT | GIPDRFSGSGSG-- TDFTLTISRLEPEDFAV YYC | QQYGS------------ ----------SPLT | FGGGTK VEIK |
| IPS:43 4479 | 21-225_76H1 | VK3|A27/ JK4 | .FM........Y / W........... | ........... / .......V | ........ / ....... | ........ / ...T... | ................... / ................Y...... | ....C............. / ........... | ..... / ...T |
| IPS:43 4513 | 21-225_76A6 | VK3|A27/ JK4 | ..........R. / W........... | ........... / ........ | ........ / ....... | ........ / ...T... | ................... / .............Y...... | ....N............. / ........... | .... / ... |
| IPS:43 4515 | 21-225_74A5 | VK3|A27/ JK4 | .FM......... / ........... | ........... / .......V | ........ / ....... | ........ / ...T... | ................... / .............Y...... | ....C............. / ........... | .... / ... |
| IPS:43 4529 | 21-225_76B9 | VK3|A27/ JK4 | .FM......... / W........... | ........... / .......V | ........ / ....... | ........ / ...T... | ................... / .............Y...... | ....C............. / ........... | ..... / ...T |
| IPS:43 4583 | 21-225_74B6 | VK3|A27/ JK4 | ........... / ........... | ........... / ........ | ........ / ....... | ........ / ...T... | ................... / .............Y...... | ....N............. / ........... | ...... / .... |
| IPS:43 4587 | 21-225_74G3 | VK3|A27/ JK4 | .FM........C / W........... | ........... / .......V | ........ / ....... | ........ / ...T... | ................... / .............Y...... | ....C............. / ........... | ..... / ...T |
| IPS:43 4603 | 21-225_77D11 | VK3|A27/ JK4 | .FM........Y / W......I... | ........... / ......V | ........ / ....... | ........ / ...T... | ................... / .............Y...... | ....C............. / ........... | ..... / ...T |
| IPS:43 4705 | 21-225_80A2 | VK3|A27/ JK4 | .FM........Y / ........... | ........... / ......V | ........ / ....... | ........ / ...T... | ................... / .............Y...... | ....C............. / ........... | ..... / ...T |
| IPS:43 4747 | 21-225_80C12 | VK3|A27/ JK4 | ..........Y / ........... | ........... / ........ | ........ / ....... | ........ / ...T... | ................... / .............Y...... | ....N............. / ........... | ...... / .... |
| IPS:43 4793 | 21-225_82A5 | VK3|A27/ JK4 | ........... / W........... | ........... / ........ | ........ / ....... | ........ / ...T... | ................... / .............Y...... | ....N............. / ........... | ..... / .... |
| IPS:43 4797 | 21-225_82G5 | VK3|A27/ JK4 | .FM........Y / W........S | .....E.... / ......V | ........ / ....... | ........ / ...T... | ................... / .............Y...... | ....C............. / ........... | .... / ...T |
| IPS:43 4805 | 21-225_82D9 | VK3|A27/ JK4 | .FM......... / W........... | ....E.... / .......V | ........ / ....... | ........ / ...T... | ................... / .............Y...... | ....C............. / ........... | ..... / ...T |
| IPS:43 4813 | 21-225_82C12 | VK3|A27/ JK4 | ........... / W........... | ........... / ........ | ........ / ....... | ........ / ...T..S | ................... / .............Y...... | ....N............. / ........... | .... / .... |
| IPS:43 4825 | 21-225_83C2 | VK3|A27/ JK4 | .FM......C / ........... | ........... / .......V | ........ / ....... | ........ / ...T... | ................... / .............Y...... | ....C............. / ........... | .... / ...T |
| IPS:43 4833 | 21-225_83C5 | VK3|A27/ JK4 | .FM......C / W........... | .....E.... / ......V | ........ / ....... | ........ / ...T... | ................... / .............Y...... | ....C............. / ........... | .... / ...T |
| IPS:43 4883 | 21-225_85B5 | VK3|A27/ JK4 | .FM......... / ........... | .S........ / .......V | ........ / ....... | ........ / ...T... | ................... / .............Y...... | ....C............. / ........... | ..... / ...T |
| IPS:43 4911 | 21-225_85D11 | VK3|A27/ JK4 | .FM......... / ..T........ | .S........ / .......V | ........ / ....... | ........ / ...T... | ................... / .............Y...... | ....C............. / ........... | ..... / ...T |
| IPS:43 4957 | 21-225_87A10 | VK3|A27/ JK4 | .F........Y / ........... | ........... / ........ | ........ / ....... | ........ / ...T..S | ................... / ..............Y...... | ....N............. / ........... | .... / .... |

| IPS | Clone | Germline | K_FR1 | K_CDR1 | K_FR2 | K_CDR2 | K_FR3 | K_CDR3 | K_FR4 |
|---|---|---|---|---|---|---|---|---|---|
| IPS:43 5247 | 21-225_96G1 | VK3\|A27/JK4 | ............. / ..T........ | ............ / .......... | ........ / P...... | ........ / ...T..S | ................... / ..........Y...... | ....N............ / ............... | ...... / .... |
| IPS:43 6368 | 21-225_211G3 | VK3\|A27/JK4 | ............. / ............. | ............ / .....F.. | ........ / ........ | ........ / ........ | ................... / ................... | ...V............. / ............. | .... / .... |
| IPS:43 6426 | 21-225_215C7 | VK3\|A27/JK4 | ............. / ......V.... | ......RITT. / ....NF.. | ........ / ........ | ........ / ........ | ................... / ........S........ | ...V............. / ............. | .... / .... |
| IPS:43 6432 | 21-225_215H12 | VK3\|A27/JK4 | ............. / ............. | .....F.. / ........ | .....M. / ........ | ......I / ........ | ................... / ................... | ...V............. / ............. | .... / .... |
| IPS:43 7322 | 21-225_75B1 | VK3\|A27/JK4 | .FM........Y / W.......I.S | ........ / ......V | ........ / ........ | ........ / ...T... | ................... / ..........Y...... | ....C............ / ............... | .... / ...... |
| IPS:43 7377 | 21-225_74G9 | VK3\|A27/JK4 | .FM......... / ............ | .......V / ........ | ........ / ........ | ........ / ...T... | ................... / ..........Y...... | ....C............ / ............... | ...T / ...T |
| Germline | VK4\|B3/JK4 | | DIVMTQSPDSLA VSLGERATINC | KSS-- QSVLYSSNNKN YLA | WYQQKPGQ PPKLLIY | W------ ASTRES | GVPDRFSGSGSG-- TDFTLTISSLQAEDVAV YYC | QQYYS-------------- ----------TPLT | FGGGTK VEIK |
| IPS:43 4511 | 21-225_74B11 | VK4\|B3/JK4 | ...........T / ............ | .........I..N / ...N.... | ........ / ........ | ........ / ........ | I................... / ................... | ................... / ................... | ...... / ...... |
| IPS:43 4729 | 21-225_80B12 | VK4\|B3/JK4 | ...L........ / ............ | ..R....... / ...Y...T | ........ / ........ | ........ / ........ | ................... / ................... | ................... / ..............P. | .... / .... |
| IPS:43 4851 | 21-225_75A6 | VK4\|B3/JK4 | ............ / ............ | ..R......H. / ...Y.... | ........ / ..E.... | ........ / ........ | ................... / ................... | ................... / ..............P. | .... / .... |
| IPS:43 5623 | 21-225_162D5 | VK4\|B3/JK4 | ..........FRN / ..M....I..F | ..N..H....R / ...NQ... | ...R.... / ........ | R....... / .T.I.K. | .........C....... / ....D.............. | ................... / ..............P. | .... / .... |
| IPS:44 6086 | 21-225_94D8 | VK4\|B3/JK4 | ...L........ / ............ | ..R....... / ...Y...T | ........ / ........ | ........ / ........ | ................... / ................... | ................... / ..............S.P. | .... / .... |
| Germline | VK2\|A19/JK4 | | DIVMTQSPLSLP VTPGEPASISC | RSS-- QSLLHSN- GYNYLD | WYLQKPGQ SPQLLIY | L------ -GSNRAS | GVPDRFSGSGSG-- TDFTLKISRVEAEDVGV YYC | MQALQ-------------- ----------TPLT | FGGGTK VEIK |
| IPS:43 4539 | 21-225_74A2 | VK2\|A19/JK4 | ............ / ............ | ............ / ...H.... | .......R / ........ | ........ / ........ | ...E............... / ................... | ..P............... / ............F. | .... / .... |
| IPS:43 4563 | 21-225_75D8 | VK2\|A19/JK4 | ............ / ............ | ............ / S....... | ........ / ........ | ........ / ........ | ................... / .............S.. | ...H............... / .............P... | .... / .... |
| IPS:43 5009 | 21-225_89G4 | VK2\|A19/JK4 | ............ / ............ | ............ / S....... | ........ / ........ | ........ / ........ | ................... / .............L... | ....H.............. / ............I... | .... / .... |
| IPS:43 5059 | 21-225_90C11 | VK2\|A19/JK4 | ............ / ............ | .Y......V.. / S....... | ........ / ...V.. | ........ / ........ | ................... / .............S.. / .............L... | ....H.............. / .............P... | .... / .... |
| IPS:43 5103 | 21-225_92B2 | VK2\|A19/JK4 | ............ / ............ | ........V.. / S.-..-.. | ........ / ...V.. | ........ / ........ | ..........V....... / ..R........I.I... | ....H.............. / ............I... | .... / .... |
| IPS:43 5713 | 21-225_171D7 | VK2\|A19/JK4 | ............ / ............ | .........YH / ........ | .....T... / ........ | V....... / ........ | ................... / ................... | ..T................ / ............... | .... / .... |

| | | | K_FR1 | K_CDR1 | K_FR2 | K_CDR2 | K_FR3 | K_CDR3 | K_FR4 |
|---|---|---|---|---|---|---|---|---|---|
| iPS:43 6246 | 21-225_201G6 | VK2\|A19/ JK4 | . . . . . . . . . . .<br>. . . . . . . . . . . | . . . . . . . . . N<br>. . R . . H . . | . . . . . . . .<br>. . . . . . . . | . . . . . . .<br>. . . . . . . | . . . . . . . . . . . . . . . . .<br>. . . . . . . . . . . . . . . . . | . . . . −<br>. . . . . . . . . . . . . . . . .<br>. . . . . QTP . | . . . . . .<br>. . . . |
| iPS:43 6254 | 21-225_202C12 | VK2\|A19/ JK4 | . . . L . . . . . . .<br>. . . . . . . . . . . | . . . . . . . . . N<br>. . K . . H . . | . . . . . . . .<br>. . . . . . . . | . . . . . . .<br>. . . . . . . | . . . . . . . . . . . . . . . . .<br>. . . . . . . . . . . . . . . . . | . . . . −<br>. . . . . . . . . . . . . . . . .<br>. . . . . QTP . | . . . . . .<br>. . . . |
| iPS:43 6304 | 21-225_201F3 | VK2\|A19/ JK4 | . . . . . . . . . . .<br>. . . . . . . . . . . | . . . . . . . . . N<br>. . R . . H . . | . . . . . . . .<br>. . . . . . . . | . . . . . . .<br>. . . . . . . | . . . . . . . . . . . . . . . . .<br>. . . . . . . . . . . . . . . . . | . . . . −<br>. . . . . . . . . . . . . . . . .<br>. . . . . QTP . | . . . . . .<br>. . . . |
| iPS:43 6334 | 21-225_208G3 | VK2\|A19/ JK4 | . . . L . . . . . . .<br>. . . . . . . . . . . | . . . . . . . . . N<br>. . K . . H . . | . . . . . . . .<br>. . . . . . . . | . . . . . . .<br>. . . . . . . | . . . . . . . . . . . . . . . . .<br>. . . . . . . . . . . . . . . . . | . . . . −<br>. . . . . . . . . . . . . . . . .<br>. . . . . QTP . | . . . . . .<br>. . . . |
| iPS:43 7248 | 21-225_97H3 | VK2\|A19/ JK4 | . . . . I . . . . . .<br>. . . . . . . . . . . | . . . . . . . . . .<br>. . . H . . . . | . . . . . . . R<br>. . . . . . . | . . . . . . .<br>. . . . . . . | . . . E . . . . . . . . . . .<br>. . . . . . . . . . . . . . . . . | . . P . . . . . . . . . . . . .<br>. . . . . . . . . . . . . F . | . . . . . .<br>. . . . |
| iPS:43 7320 | 21-225_75A1 | VK2\|A19/ JK4 | . . . . . . . . . . .<br>. . . . . . . . . . . | . . . . . . . . . .<br>. . . H . . . . | . . . . . . . R<br>. . . . . . . | . . . . . . .<br>. . . . . . . | . . . E . . . . . . . . . . .<br>. . . . . . . . . . . . . . . . . | . . P . . . . . . . . . . . . .<br>. . . . . . . . . . . . . F . | . . . . . .<br>. . . . |
| iPS:43 7371 | 21-225_74D8 | VK2\|A19/ JK4 | . . . . . . . . . . .<br>. . . . . . . . . . . | . . . . . . . V . .<br>S . . . . . . . | . . . . . . . .<br>. . . V . . | . . . . . . . .<br>. . . . . . . | . . . . . . . . . . . . . S . .<br>. . . . . . . . . . . L . . . | . . . . H . . . . . . . . . . .<br>. . . . . . . . . . P . . . | . . . .<br>. . . . |
| iPS:39 2718 | 21-225_17B8 | VK2\|A19/ JK4 | . . . . . . . . . . .<br>. . . . . . . . . . | . . . . . . . . . .<br>. . . N . S . . | . . . . . . . .<br>. . . . . . . | . . . . . . . .<br>. . . H . . . | . . . . . . . . . . . . D . . . . . .<br>. . . . . . . . . . . . . . . . | . . . V . . . . . . . . . . . . .<br>. . . . . . . . . TP . . . | . . . .<br>. . . . |
| | Germline | | K_FR1 | K_CDR1 | K_FR2 | K_CDR2 | K_FR3 | K_CDR3 | K_FR4 |
| | **VK3\|L2/JK1** | | EIVMTQSPATLS VSPGERATLSC | RAS−−QSVS−− −−−−SNLA | WYQQKPGQ APRLLIY | G−−−−−− −ASTRAT | GIPARFSGSGSG−− TEFTLTISSLQSEDFAV YYC | QQYNN−−−−−−−−−−− −−−−−−−−WPWT | FGQGTK VEIK |
| iPS:43 4871 | 21-225_85H1 | VK3\|L2/J K1 | . . . . . . . . . . . .<br>. . . . . . . . . . . | . . . . . . D . I . .<br>. . . . TY . . | . . . . . . . .<br>. . . . . . . | . . . . . . .<br>. . . . . . . | . V . . . . . . . . . . . . . . .<br>. . . . . . . . . . . L . . . | . E . . D . . . . . . . . . . .<br>. . . . . . . . . . . CS | . . . . . .<br>. . . . |
| iPS:43 5421 | 21-225_151F1 | VK3\|L2/J K1 | . M . . . . . . . . . .<br>. . . . . V . . . . | . . . . . . . IN . .<br>. . . . I . I . | . . . . . . . .<br>. . . . . . . | . . . . . . .<br>. . . . . . . | . . . . . . . . . . . . . . . . .<br>. . . . . . . . . . . . . . . . . | . . . . D . . . . . . . . . . .<br>. . . . . . . . . WP . . . | . . . . . .<br>. . . . |
| iPS:43 5497 | 21-225_155H9 | VK3\|L2/J K1 | . . . . . . . . . . . .<br>. . . . . . . . . . . | . . . . . . . . . . .<br>. S . . . . . . | . . . . . . . .<br>. . . . . . . | . . . . . . .<br>. . . . . . . | . . . . . . . . . . . . . . . . .<br>. . . . . . . . . . . . . . . . . | . . . DD . . . . . . . . . . .<br>. . . . . . . . . WP . . . | . . . . . .<br>. . . . |
| iPS:43 5605 | 21-225_161A4 | VK3\|L2/J K1 | . . . . . . . . . .<br>. . . . . . . S . . . | . S . . . . . N . .<br>. . . . . . . . | . . . . R . . .<br>. L . . . . . | . . . . . . . .<br>. . . I . . . | D . . . . N . . . . . . . . . .<br>. . . . . . . . . . . . . . F . | . . . . −<br>. . . . . NW . . | . . . . . T<br>. . . . |
| iPS:45 1118 | 21-225_191C8 | VK3\|L2/J K1 | . . . . . . . . . . . .<br>. . . . . . . . . . . | . . . . . . . R . .<br>. . . . . . . . | . . . . E . . .<br>. . . . . . . | . . . . . . .<br>. . . . . . . | . . . . . . . . . . . . . . . . .<br>. . . . . . . . . . . . . . . . . | . SFT . . . . . . . . . . . . .<br>. . . . . . . . . LR . | . . . . . .<br>. . . . |
| iPS:39 2734 | 21-225_17D8 | VK3\|L2/J K1 | . . . . . . . S . . .<br>. . . . . . . . . . | . . . . . . . . . . .<br>. . . . . . . . | . F . . . . . .<br>. . . . . . N | . . . . . . .<br>. . . . . S | . . . . . . . . . . . . . . . . .<br>. . . . . . . . . . . . . . . . | . . . . . . . . . . . . . . . .<br>. . . . . . . . . . . L . | . . . .<br>. . . . |
| iPS:39 2768 | 21-225_20B8 | VK3\|L2/J K1 | . . . . . . . S . . .<br>. . . . . V . . . . | . . . . . . . . . . .<br>. . . . . . . . | . F . . . . . .<br>. . . . . . N | . . . . . . .<br>. . . . . S | . . . . . . . . . . . . . . . . .<br>. . . . . . . . . . . . . . . . | . . . . . . . . . . . . . . . .<br>. . . . . . . . . C . L . | . . . .<br>. . . . |
| iPS:39 3044 | 21-225_25B8 | VK3\|L2/J K1 | . . . . . . . . . . . .<br>. . . . . . . . . . | . . . . . . . R . .<br>. . . . . . . . | . . . . . . . .<br>. . . . . . . | . . . . . . .<br>. . . . . . . | . . . . . . . . . . . . . . . . .<br>. . . . . . . . . . . L . . . | . . . . . . . . . . . . . . . .<br>. . . . . . . . . WP . . P | . . . .<br>. . . . |
| iPS:39 3050 | 21-225_28C5 | VK3\|L2/J K1 | . . . . . . . . . . . .<br>. . . . . . . . . . | . . . . . . . . . . .<br>. . . . . . . | . . H . . . . .<br>. . . . . . . | . . . . . . .<br>. . . . . . . | . . . . . . . . . . . . . . . . .<br>. . . . . . . . . . . L . . . | . . . . . . . . . . . . . . . .<br>. . . . . . . . . WP . . P | . . . . . .<br>. . . . |

| | | | K_FR1 | K_CDR1 | K_FR2 | K_CDR2 | K_FR3 | K_CDR3 | K_FR4 |
|---|---|---|---|---|---|---|---|---|---|
| iPS:39 3906 | 21-225_13D3 | VK3\|L2\|JK1 | ..............<br>.....S..... | ......T....<br>........ | .F......<br>......N | .......<br>....... | ...................<br>..........F. | ...HD...........<br>.........P. | ......<br>.... |
| Germline | | | **K_FR1** | **K_CDR1** | **K_FR2** | **K_CDR2** | **K_FR3** | **K_CDR3** | **K_FR4** |
| **VK1\|L1\|JK5** | | | DIQMTQSPSSLS ASVGDRVTITC | RAS--QGIS-- ----NYLA | WFQQKPGK APKSLIY | A------- -ASSLQS | GVPSRFSGSGSG-- TDFTLTISSLQPEDFAT YYC | QQYNS----------- ----------YPIT | FGQGTR LEIK |
| iPS:43 4947 | 21-225_87B7 | VK1\|L1\|JK5 | ..............<br>.......... | ..........<br>.......... | ........<br>........ | ........<br>......H. | ....K........<br>.........S...F. | LL.LT...........<br>..........L. | ......<br>.... |
| iPS:43 5427 | 21-225_151C9 | VK1\|L1\|JK5 | ..............<br>.......... | ..........<br>....K... | ........<br>....... | D.......<br>...R... | ....K........<br>.........S... | H..KH...........<br>.......... | ......<br>.... |
| iPS:43 5529 | 21-225_157H7 | VK1\|L1\|JK5 | ..............<br>.......... | ......D....<br>.....F.. | ........<br>.....VS | T.......<br>....... | ....K........<br>.......... | ...H...........<br>.......... | ......<br>.... |
| iPS:43 6066 | 21-225_194B7 | VK1\|L1\|JK5 | ..............<br>.......... | ..........<br>....K... | ........<br>....... | G.......<br>...R... | ....K........<br>.......... | .H.LN...........<br>..........L. | ......<br>.... |
| iPS:43 7274 | 21-225_196D4 | VK1\|L1\|JK5 | ..............<br>.......... | ..........<br>........ | ....N...<br>....... | ........<br>........ | ....K.....C......<br>......P.....V..... | .H.LN...........<br>..........L. | ......<br>.... |
| iPS:39 2748 | 21-225_20A8 | VK1\|L1\|JK5 | ..............<br>.......... | ........N..<br>.......V | ........<br>....... | ........<br>.....L. | ....K........<br>.......... | ...............<br>.......... | ......<br>.... |
| iPS:39 3062 | 21-225_33H3 | VK1\|L1\|JK5 | ..............<br>.......... | Q.....D....<br>.....F.N | ..R.....<br>..N.... | D.......<br>...N.VT | .......R........<br>.F.............. | ...DN...........<br>..........L... | ......<br>.... |
| iPS:39 8532 | 21-225_33B7 | VK1\|L1\|JK5 | ..............<br>.......... | ......D....<br>........ | ........<br>..T.... | ........<br>....... | ....K........<br>.........F. | ...H...........<br>..........L. | ......<br>.... |
| iPS:40 2221 | 21-225_2C12 | VK1\|L1\|JK5 | ..............<br>.......... | ..........<br>.......... | .....S..<br>......S | ........<br>..T.... | ....Q........<br>.......... | ...Y...........<br>.......... | ......<br>.... |
| Germline | | | **K_FR1** | **K_CDR1** | **K_FR2** | **K_CDR2** | **K_FR3** | **K_CDR3** | **K_FR4** |
| **VK4\|B3\|JK5** | | | DIVMTQSPDSLA VSLGERATINC | KSS-- QSVLYSSNNKN YLA | WYQQKPGQ PPKLLIY | W------- -ASTRES | GVPDRFSGSGSG-- TDFTLTISSLQAEDVAV YYC | QQYYS----------- ----------TPIT | FGQGTR LEIK |
| iPS:43 5665 | 21-225_169F2 | VK4\|B3\|JK5 | ...........I<br>.......... | ..........<br>........ | ........<br>....... | ........<br>....... | ...............<br>............ | ....-<br>.................<br>.....RAP. | ......<br>.... |
| iPS:43 5671 | 21-225_169H5 | VK4\|B3\|JK5 | ...........I<br>.......... | ..........<br>........ | ........<br>....... | ........<br>....... | ...............<br>............ | ....-<br>.................<br>.....RAP. | ......<br>.... |
| iPS:43 6554 | 21-225_224C10 | VK4\|B3\|JK5 | ..........N<br>A........T. | ..........<br>........ | ........<br>....... | ........<br>....... | ...............<br>............I... | ....I...........<br>..........N.CS | ......<br>.... |
| iPS:39 8510 | 21-225_25A3 | VK4\|B3\|JK5 | ..........T<br>.......... | ..........<br>........ | ........<br>....... | ........<br>....... | ...............<br>............ | ...............<br>.............CS | ......<br>.... |
| iPS:39 8516 | 21-225_26A9 | VK4\|B3\|JK5 | ..............<br>.......... | ..........<br>........ | ........<br>....... | ........<br>....... | ...............<br>............ | ...............<br>..........S.CS | ......<br>.... |
| Germline | | | **K_FR1** | **K_CDR1** | **K_FR2** | **K_CDR2** | **K_FR3** | **K_CDR3** | **K_FR4** |

| iPS ID | Clone | VK/JK | FR1 | CDR1 | FR2 | CDR2 | FR3 | CDR3 | FR4 |
|---|---|---|---|---|---|---|---|---|---|
| | **VK2\|A19/JK1** | | DIVMTQSPLSLP<br>VTPGEPASISC | RSS--<br>QSLLHSN-<br>GYNYLD | WYLQKPGQ<br>SPQLLIY | L-------<br>-GSNRAS | GVPDRFSGSGSG--<br>TDFTLKISRVEAEDVGV<br>YYC | MQALQ------------<br>------------TPWT | FGQGTK<br>VEIK |
| iPS:43 5667 | 21-225_169E3 | VK2\|A19/JK1 | ............<br>............ | .........N<br>....K... | .......<br>....... | .......<br>....... | .......A.......<br>............... | ..V...........<br>........... | ....-<br>.... |
| iPS:43 5673 | 21-225_169E6 | VK2\|A19/JK1 | ............<br>............ | .........N<br>....K... | .......<br>....... | .......<br>....... | .......A.......<br>............... | ..V...........<br>............... | ....-<br>.... |
| iPS:43 5759 | 21-225_176E6 | VK2\|A19/JK1 | ............<br>............ | .........N<br>....K... | .......<br>....... | .......<br>....... | .......A.......<br>............... | ..V...........<br>............... | ...-<br>.... |
| | **Germline** | | K_FR1 | K_CDR1 | K_FR2 | K_CDR2 | K_FR3 | K_CDR3 | K_FR4 |
| | **VK6\|A26/JK1** | | EIVLTQSPDFQS<br>VTPKEKVTIIC | RAS--QSIG--<br>----SSLH | WYQQKPDQ<br>SPKLLIK | Y-------<br>-ASQSFS | GVPSRFSGSGSG--<br>TDFTLTINSLEAEDAAT<br>YYC | HQSSS----------<br>----------LPWT | FGQGTK<br>VEIK |
| iPS:43 5817 | 21-225_190B11 | VK6\|A26/JK1 | ............<br>.A......... | ...........<br>......N.. | .......<br>....... | S......<br>....... | ...............<br>........T..... | Q...............<br>............... | ....<br>.... |
| iPS:43 5823 | 21-225_190F11 | VK6\|A26/JK1 | ......F..S..<br>........... | ......N....<br>......... | ......E.<br>...V... | .......<br>....... | ...............<br>............... | ...............<br>..........F.R. | ....<br>.... |
| iPS:43 5867 | 21-225_191E5 | VK6\|A26/JK1 | ......F..S..<br>........... | ...........<br>......... | .......<br>....... | .......<br>....... | ...............<br>............... | ...............<br>..........F.R. | ....<br>.... |
| iPS:43 5917 | 21-225_190D5 | VK6\|A26/JK1 | ............<br>.A......... | ...........<br>......N.. | .......<br>....... | S......<br>....... | ...............<br>........T..... | Q...............<br>............... | ....<br>.... |
| iPS:43 5929 | 21-225_190D9 | VK6\|A26/JK1 | ......F..S..<br>........... | ...........<br>......... | .......<br>....... | .......<br>....... | ...............<br>............... | ...............<br>..........F.R. | ....<br>.... |
| iPS:43 5935 | 21-225_190H8 | VK6\|A26/JK1 | ......F..S..<br>........... | ...........<br>......... | .......<br>....... | .......<br>S...... | ...............<br>............... | ...............<br>..........F.R. | ....<br>.... |
| iPS:43 6056 | 21-225_194C3 | VK6\|A26/JK1 | ............<br>.A......... | ...........<br>......N.. | .......<br>....... | S......<br>....... | ...............<br>........T..... | Q...............<br>............... | ....<br>.... |
| iPS:43 6216 | 21-225_200B7 | VK6\|A26/JK1 | ............<br>........... | ......N....<br>....NT.. | .......<br>....... | .......<br>....... | I..............<br>............... | ...G...........<br>............Q. | ....<br>.... |
| iPS:43 6220 | 21-225_200F8 | VK6\|A26/JK1 | ............<br>.A......... | ...........<br>......N.. | .......<br>....... | S......<br>....... | ...............<br>........T..... | Q...............<br>............... | ....-<br>.... |
| iPS:43 6448 | 21-225_217A3 | VK6\|A26/JK1 | ........K.<br>........... | ...........<br>......... | .......<br>.....V. | .......<br>....... | ...............<br>........G..... | ...R...........<br>............... | ....<br>.... |
| | **Germline** | | K_FR1 | K_CDR1 | K_FR2 | K_CDR2 | K_FR3 | K_CDR3 | K_FR4 |
| | **VK6\|A26/JK4** | | EIVLTQSPDFQS<br>VTPKEKVTIIC | RAS--QSIG--<br>---SSLH | WYQQKPDQ<br>SPKLLIK | Y-------<br>-ASQSFS | GVPSRFSGSGSG--<br>TDFTLTINSLEAEDAAT<br>YYC | HQSSS----------<br>-------LPLT | FGGGTK<br>VEIK |
| iPS:43 5829 | 21-225_190B12 | VK6\|A26/JK4 | ...........<br>........... | .........<br>......... | .......<br>....... | .......<br>....... | .D.............<br>............... | ..TR...........<br>............... | ...-<br>.... |
| iPS:43 5863 | 21-225_191H4 | VK6\|A26/JK4 | ...........<br>........... | ..N......<br>......... | .......<br>....... | .......<br>.....L. | .......A.......<br>.......D....... | ..TGR...........<br>............... | ....<br>.... |
| iPS:43 5943 | 21-225_191C9 | VK6\|A26/JK4 | ...........<br>........... | .........<br>......... | .......<br>....... | .......<br>....... | .D.............<br>............... | ..TR...........<br>............... | ....<br>.... |

| | | | K_FR1 | K_CDR1 | K_FR2 | K_CDR2 | K_FR3 | K_CDR3 | K_FR4 |
|---|---|---|---|---|---|---|---|---|---|
| iPS:43 5983 | 21-225_192E5 | VK6|A26/ JK4 | ............ / ............ | ........... / ....R... | ........ / ....... | ....... / ....... | ................ / ............... | ....R............ / ............... | ...... / .... |
| iPS:43 6043 | 21-225_193G9 | VK6|A26/ JK4 | ............ / ............ | ............ / ....R... | ........ / .L..... | ...R... / ....... | ................ / ..............F. | ....R............ / ............... | ...... / .... |
| iPS:43 6084 | 21-225_195F2 | VK6|A26/ JK4 | ............ / ............ | ........ / ....... | ........ / ....... | ....... / ....... | ................ / A...S............ | ...RT............ / ............... | ...... / .... |
| iPS:43 6094 | 21-225_195B10 | VK6|A26/ JK4 | ............ / ............ | ......... / ......... | ........ / ........ | ...... / ....... | ................ / ................ | ...GR............ / ............... | ...... / .... |
| iPS:43 6240 | 21-225_201E8 | VK6|A26/ JK4 | .......A... / ............ | ......N.... / ....R... | ........ / ....... | ....... / ....... | ...............N. / ..............V... | ...R............ / ............... | ...... / ...R |
| iPS:43 6314 | 21-225_206G4 | VK6|A26/ JK4 | ............ / ............ | ......... / ....R... | ........ / ........ | ...... / ....... | ................ / ................ | ...R............ / ............... | ...... / .... |
| **Germline** | | | **K_FR1** | **K_CDR1** | **K_FR2** | **K_CDR2** | **K_FR3** | **K_CDR3** | **K_FR4** |
| **VK1|A20/JK3** | | | DIQMTQSPSSLS ASVGDRVTIIC | RAS--QGIS-- ----NYLA | WYQQKPGK VPKLLIY | A------- -ASTLQS | GVPSRFSGSGSG-- TDFTLTISSLQPEDVAT YYC | QKYNS------------ ---------APFT | FGPGTK VDIK |
| iPS:43 5833 | 21-225_190D12 | VK1|A20/ JK3 | ............ / ............ | .......... / .......... | ......... / ......... | V....... / ....... | ................ / ................ | ............... / ............... | ...... / .... |
| iPS:43 6019 | 21-225_193C4 | VK1|A20/ JK3 | ............ / ............ | .P........ / ....I... | .......N / ....... | ...... / ....... | ................ / ................ | ............... / ............... | ...... / .... |
| iPS:39 4010 | 21-225_12G5 | VK1|A20/ JK3 | ............ / .........P. | ......D.... / ......... | ........ / ....... | ....... / ..YI... | ................ / ..............A... | ...D............ / ............... | ...... / .... |
| **Germline** | | | **K_FR1** | **K_CDR1** | **K_FR2** | **K_CDR2** | **K_FR3** | **K_CDR3** | **K_FR4** |
| **VK6|A26/JK3** | | | EIVLTQSPDFQS VTPKEKVTIIC | RAS--QSIG-- ----SSLH | WYQQKPDQ SPKLLIK | Y------- -ASQSFS | GVPSRFSGSGSG-- TDFTLTINSLEAEDAAT YYC | HQSSS------------ ---------LPFT | FGPGTK VDIK |
| iPS:43 6025 | 21-225_193B5 | VK6|A26/ JK3 | ............ / ............ | ........ / ........ | ......... / ......... | ....... / ....... | ................ / ................ | ....R............ / ............... | ...... / .... |
| iPS:43 6096 | 21-225_195E10 | VK6|A26/ JK3 | ............ / ............ | ........ / ........ | ......... / ......... | ....... / ....... | ................ / ................ | ....R............ / ............... | ...... / .... |
| iPS:43 6408 | 21-225_214H8 | VK6|A26/ JK3 | ............ / ............ | ....V... / ........ | ......... / ..Q.... | ....... / ....... | ................ / ................ | ...R............ / ............... | ....S. / .... |
| iPS:43 6424 | 21-225_215H6 | VK6|A26/ JK3 | ............ / ............ | ....V... / ........ | ......... / ..Q.... | ....... / .....L. | ................ / ................ | ...R............ / ............... | ....S. / .... |
| **Germline** | | | **K_FR1** | **K_CDR1** | **K_FR2** | **K_CDR2** | **K_FR3** | **K_CDR3** | **K_FR4** |
| **VK1|L5/JK2** | | | DIQMTQSPSSVS ASVGDRVTIIC | RAS--QGIS-- ----SWLA | WYQQKPGK APKLLIY | A------- -ASSLQS | GVPSRFSGSGSG TDFTLTISSLQPEDFAT YYC | QQANS------------ ---------FPYT | FGQGTK LEIK |
| iPS:43 6082 | 21-225_195D9 | VK1|L5/J K2 | ............ / ............ | .......... / ....R... | ........ / ....... | ....... / .....LG | ................ / ................ | .R............... / ............CS | ...... / .... |
| iPS:43 6118 | 21-225_196A10 | VK1|L5/J K2 | .......Y..Y.. / .......S... | .......... / ....R... | ........ / .A.F... | ....... / .....LG | ..S............ / ...............I... | .RD............. / ..........L.CS | ...... / .... |
| **Germline** | | | **K_FR1** | **K_CDR1** | **K_FR2** | **K_CDR2** | **K_FR3** | **K_CDR3** | **K_FR4** |

| ID | Clone | VK/JK | K_FR1 | K_CDR1 | K_FR2 | K_CDR2 | K_FR3 | K_CDR3 | K_FR4 |
|---|---|---|---|---|---|---|---|---|---|
| | VK2\|A19/JK2 | | DIVMTQSPLSLP VTPGEPASISC | RSS-- QSLLHSN- GYNYLD | WYLQKPGQ SPQLLIY | L------- -GSNRAS | GVPDRFSGSGSG-- TDFTLKISRVEAEDVGV YYC | MQALQ------------- -----------TPYT | FGQGTK LEIK |
| iPS:43 6362 | 21-225_210C12 | VK2\|A19/JK2 | ............ / ............ | ..........Y / ...H.F.. | ........ / ....... | ....... / .V..... | ................ / ................ | ................ / ..........TPMCS | ...... / .... |
| iPS:43 6374 | 21-225_211C10 | VK2\|A19/JK2 | ............ / ............ | ........ / ........ | ...L.... / ....... | ....... / ....... | ................ / ......M......I... | ....L. / .........TPVCS | ...... / .... |
| | Germline | | K_FR1 | K_CDR1 | K_FR2 | K_CDR2 | K_FR3 | K_CDR3 | K_FR4 |
| | VK2\|A17/JK1 | | DVVMTQSPLSLP VTLGQPASISC | RSS QSLVYSD- GNTYLN | WFQQRPGQ SPRRLIY | K------ -VSNRDS | GVPDRFSGSGSG-- TDFTLKISRVEAEDVGV YYC | MQGTH---------- ----------WPWT | FGQGTK VEIK |
| iPS:43 7226 | 21-225_57C2 | VK2\|A17/JK1 | ............ / ............ | ........ / ........ | ........ / ....... | E....... / ....W.. | ...N............ / .....A........... | V................ / ............R. | ...... / .... |
| | Germline | | K_FR1 | K_CDR1 | K_FR2 | K_CDR2 | K_FR3 | K_CDR3 | K_FR4 |
| | VK1\|A20/JK5 | | DIQMTQSPSSLS ASVGDRVTIIC | RAS QGIS ----NYLA | WYQQKPGK VPKLLIY | A------ -ASTLQS | GVPSRFSGSGSG TDFTLTISSLQPEDVAT YYC | QKYNS------------ ---------APIT | FGQGTR LEIK |
| iPS:39 2726 | 21-225_20B5 | VK1\|A20/JK5 | ............ / ............ | .........N.. / ........ | ........ / I...... | ....... / ....... | ................ / ................C. | ................ / .........AP... | ...... / .... |
| iPS:39 2792 | 21-225_20G12 | VK1\|A20/JK5 | ............ / ............ | ............ / ........ | ........ / ...V... | T....... / ....... | ................ / ...V........... | ................ / .........AP... | ...... / .... |
| iPS:39 8478 | 21-225_17C10 | VK1\|A20/JK5 | .........Q. / ............ | ............ / ........ | .......R / ....... | ....... / ....... | ................ / .........D....... | ................ / .........AP.L. | ...... / .... |
| | Germline | | K_FR1 | K_CDR1 | K_FR2 | K_CDR2 | K_FR3 | K_CDR3 | K_FR4 |
| | VK1\|L1/JK2 | | DIQMTQSPSSLS ASVGDRVTIIC | RAS--QGIS-- ----NYLA | WFQQKPGK APKSLIY | A------ -ASSLQS | GVPSRFSGSGSG-- TDFTLTISSLQPEDFAT YYC | QQYNS---------- ----------YPYT | FGQGTK LEIK |
| iPS:39 2776 | 21-225_21A12 | VK1\|L1/JK2 | ............ / ............ | ............ / ...K... | ........ / ....... | ....... / ....... | ....K........... / ................ | ................ / ............FR | ...... / .... |
| | Germline | | K_FR1 | K_CDR1 | K_FR2 | K_CDR2 | K_FR3 | K_CDR3 | K_FR4 |
| | VK1\|A30/JK3 | | DIQMTQSPSSLS ASVGDRVTIIC | RAS--QGIR-- ----NDLG | WYQQKPGK APKRLIY | A------ -ASSLQS | GVPSRFSGSGSG-- TEFTLTISSLQPEDFAT YYC | LQHNS------------ ---------YPFT | FGPGTK VDIK |
| iPS:43 7240 | 21-225_84H12 | VK1\|A30/JK3 | .........YQ. / ............ | ............ / ........ | .F...... / ....... | ....... / ....... | ................ / ................ | ....D........... / ................ | ...... / .... |
| iPS:43 4577 | 21-225_75C11 | VK1\|A30/JK3 | ............ / ............ | ............ / ........ | .F...... / ....... | ....... / ....... | ................ / ................ | ....D........... / ................ | ...... / .... |
| iPS:43 4553 | 21-225_76H12 | VK1\|A30/JK3 | ............ / ............ | ............ / ........ | .F...... / ....... | ....... / ...R... | ................ / ................ | ....D........... / ................ | ...... / .... |
| iPS:43 4927 | 21-225_86E5 | VK1\|A30/JK3 | ............ / ............ | ............ / ........ | ....... / ....... | ....... / ....... | ................ / ...............H. | ....D........... / ................ | .E.. / .... |
| | Germline | | K_FR1 | K_CDR1 | K_FR2 | K_CDR2 | K_FR3 | K_CDR3 | K_FR4 |

**First alignment block — VK1|L5/JK1**

| | VK1|L5/JK1 | | DIQM_QSPSSVS ASVGDRVTIIC | RAS--QGIS-- ----SWLA | WYQQKPGK APKLLIY | A------- -ASSLQS | GVPSRFSGSGSG--- TDFTLTISSLQPEDFAT YYC | QQANS------------ ----------FPWT | FGQGTK VEIK |
|---|---|---|---|---|---|---|---|---|---|
| IPS:43 5477 | 21-225_154E8 | VK1|L5/JK1 | ............ ..I........ | ......F.... ........ | ........ ....... | T....... ....... | ............... .................C. | ................ ............... | ...... ..FN |
| IPS:43 5385 | 21-225_149G7 | VK1|L5/JK1 | ............ ........... | ......F.... ........ | ........ ...F... | ....... ....... | ............... .................. | ................ ............... | ...... ...N |

**LAMBDA_VARIABLE**

| | | Germline | L_FR1 | L_CDR1 | L_FR2 | L_CDR2 | L_FR3 | L_CDR3 | L_FR4 |
|---|---|---|---|---|---|---|---|---|---|
| | VL3|3r/JL2 | | SYELTQP- PSVSVSPGQIAS ITC | SGD----KLGD- ----KYAC | WYQQKPGQ SPVLVIY | Q------- -DSKRPS | GIPERFSGSNSG-- NTATLTISGTQAMDEAD YYC | QAWDS------------ ----------STAVV | FGGGTK LTVL |
| IPS:45 3445 | 21-225_148E10 | VL3|3r/JL2 | ............ ............ | ..........N. ......V. | ....R..H AA..I.. | ....... ....... | .............. ................F. | ....R......... .........N.Y.. | ...... .... |
| IPS:47 2742 | 21-225_30D9_L C2 | VL3|3r/JL2 | ............ ............ | ............ ......VY | .F...... ....... | ....... ..R.... | .............. .........L....... | ....N......... .........-STA. | ...... .... |
| IPS:47 2743 | 21-225_68G6 | VL3|3r/JL2 | ...V........ ............ | ............ ......TY | .....A.. ..F.... | ....... ..R.... | ..D........... ..........A..F. | ....N......... .........-STA. | ...... .... |
| IPS:43 6652 | 21-225_146B11 | VL3|3r/JL2 | ............ ............ | ............ .......S | ....... ....... | ....... ....... | .............. ......I......... | .............. .........-ST.. | ...... .... |
| IPS:43 6654 | 21-225_146C11 | VL3|3r/JL2 | ............ ............ | ............ .......S | ....... ....... | ....... ....... | .............. ......I......... | .............. .........-ST.. | ...... .... |
| IPS:43 6658 | 21-225_146A2 | VL3|3r/JL2 | ............ ............ | ............ .......S | ....... ....... | ....... ....... | .............. ......I......... | .............. .........-ST.. | ...... .... |
| IPS:43 6664 | 21-225_147E7 | VL3|3r/JL2 | ............ ............ | ............ .......S | ....... ....... | ....... ....... | .............. ......I......... | .............. .........-ST.. | ...... .... |
| IPS:43 6666 | 21-225_147B8 | VL3|3r/JL2 | ............ ............ | ............ ......V. | ..E.... ....... | ....... ..R.... | .............. .............. | ...G.......... .........N.... | ...... .... |
| IPS:43 6668 | 21-225_147B9 | VL3|3r/JL2 | ............ ............ | ............ ......VS | ....R... ....... | ....... ..R.... | .............. ......L.V...... | L............. .........F.. | ...... .... |
| IPS:43 6670 | 21-225_147D9 | VL3|3r/JL2 | ............ .........S | ..........N. ......V. | ....R... ....... | ....... ....... | .............. .............. | ....R......... .........N.Y.. | ...... .... |
| IPS:43 6672 | 21-225_147F9 | VL3|3r/JL2 | ............ ............ | ......E..N. ........ | ....... ....... | ....... ....... | .............. .............. | ...H.......... .........-ST.. | ...... .... |
| IPS:43 6674 | 21-225_147G9 | VL3|3r/JL2 | ............ ............ | ............ ........ | ....... ....... | ....... ..R.... | .............. .............. | ...H.......... .........-ST.. | ...... .... |
| IPS:43 6676 | 21-225_147E11 | VL3|3r/JL2 | ............ ............ | ............ .......S | ....... ....... | ....... ....... | .............. ......I......... | .............. .........-ST.. | ...... .... |
| IPS:43 6678 | 21-225_147B12 | VL3|3r/JL2 | ............ ............ | ............ .......S | ....... ....... | ....... ....... | .............. ......I.......A. | .............. .........-ST.. | ...... .... |
| IPS:43 6686 | 21-225_148G6 | VL3|3r/JL2 | ............ ............ | ............ .......S | ....... ....... | ....... ....... | .............. ......I......... | .............. .........-ST.. | ...... .... |

| | | | | | | | |
|---|---|---|---|---|---|---|---|
| iPS:43 6688 | 21-225_148C8 | VL3\|3r/JL 2 | ...........<br>........... | ...........<br>......VS | .........<br>..I.... | .......<br>..R.... | .T..............<br>............... | L..................<br>..........F.. | ....<br>.... |
| iPS:43 6690 | 21-225_148A9 | VL3\|3r/JL 2 | ...........<br>........... | .........N.<br>......V. | .......<br>....... | .......<br>....... | ...............<br>............... | ...H..............<br>.........-ST.. | ....<br>.... |
| iPS:43 6694 | 21-225_148G11 | VL3\|3r/JL 2 | ...........<br>........... | ...........<br>.....F.S | .......<br>....... | .......<br>....... | ...............<br>......I........ | ..................<br>.........-ST.. | ....<br>.... |
| iPS:43 6698 | 21-225_149B5 | VL3\|3r/JL 2 | ...........<br>........... | ..Y......Y.<br>......V. | .......<br>......F | .......<br>.NNQ... | ...............<br>S.............. | ..................<br>.........-ST.. | ....<br>.... |
| iPS:43 6700 | 21-225_149C7 | VL3\|3r/JL 2 | ...........<br>........... | ..N........<br>.......S | .......<br>....... | .......<br>....... | .......K.......<br>......I........ | ..................<br>.........-ST.. | ....<br>...P |
| iPS:43 6704 | 21-225_149C10 | VL3\|3r/JL 2 | ...........<br>........... | ...........<br>.......S | .......<br>....... | .......<br>..N.... | ...............<br>....G.I........ | ..................<br>.........-ST.. | ....<br>.... |
| iPS:43 6706 | 21-225_149A11 | VL3\|3r/JL 2 | ...........<br>........... | .........N.<br>......VS | .......<br>....... | .......<br>...R... | ...............<br>............... | L..................<br>..........F.. | ....<br>.... |
| iPS:43 6708 | 21-225_150D3 | VL3\|3r/JL 2 | ...........<br>........... | ......E..N.<br>.......... | .......<br>....... | .......<br>..N.... | ...............<br>........S...... | ...H..............<br>.........-ST.. | ....<br>.... |
| iPS:43 6710 | 21-225_150F6 | VL3\|3r/JL 2 | ...........<br>........... | ...........<br>.......S | .......<br>......C | .......<br>....... | ...............<br>......I.......C. | ..................<br>.........-ST.. | ....<br>.... |
| iPS:43 6714 | 21-225_150H11 | VL3\|3r/JL 2 | ...........<br>........... | ...........<br>.......S | .......<br>....... | .......<br>....... | ...............<br>......I.......F.. | ..................<br>.........-ST.. | ....<br>.... |
| iPS:43 6716 | 21-225_151F3 | VL3\|3r/JL 2 | ...........<br>........... | ...........<br>......V. | .......<br>....... | .......<br>..R.... | ...............<br>............... | ...H..............<br>.........-ST.. | ....<br>.... |
| iPS:43 6718 | 21-225_151H5 | VL3\|3r/JL 2 | ...........<br>.........A. | ...........<br>.......S | .......<br>....... | .......<br>....... | ...............<br>......I........ | ..................<br>.........-ST.. | ....<br>.... |
| iPS:43 6722 | 21-225_151H7 | VL3\|3r/JL 2 | ..D........<br>........... | ...........<br>.......S | .......<br>....... | .......<br>....... | ...............<br>......I........ | ..................<br>.........-ST.. | ....<br>.... |
| iPS:43 6724 | 21-225_151B9 | VL3\|3r/JL 2 | ...........<br>........A. | ......N....<br>.......S | .......<br>....... | .......<br>....... | ...............<br>......I........ | ..................<br>.........-ST.. | ....<br>.... |
| iPS:43 6728 | 21-225_152G6 | VL3\|3r/JL 2 | ...........<br>........... | ...........<br>.......S | .......<br>....... | .......<br>....... | ...............<br>......I..L...... | ....N.............<br>.........-ST.. | ....<br>.... |
| iPS:43 6730 | 21-225_152D7 | VL3\|3r/JL 2 | ...........<br>........... | ...........<br>.......S | .......<br>....... | .......<br>....... | ...............<br>......I........ | ..................<br>.........-ST.. | ....<br>.... |
| iPS:43 6736 | 21-225_153E8 | VL3\|3r/JL 2 | ...........<br>........... | ..S......N.<br>......V. | ....R...<br>....... | .......<br>..N.... | ...............<br>..........G... | ..................<br>..........Y.I | ....<br>.... |
| iPS:43 6738 | 21-225_153D9 | VL3\|3r/JL 2 | ...........<br>........... | ...........<br>........... | .......<br>....... | .......<br>..R.... | ...............<br>............... | ...H..............<br>.........-ST.. | ....<br>V... |
| iPS:43 6740 | 21-225_154C3 | VL3\|3r/JL 2 | ...........<br>........... | ...........<br>......V. | .......<br>....... | .......<br>..R.... | ...............<br>....T.......... | ...H..............<br>.........-ST.. | ....<br>.... |
| iPS:43 6742 | 21-225_154C4 | VL3\|3r/JL 2 | ...........<br>.......R... | ...........<br>.......S | .......<br>....... | .......<br>....... | ...............<br>......I........ | ..................<br>.........-ST.. | ....<br>.... |
| iPS:43 6744 | 21-225_154F4 | VL3\|3r/JL 2 | ...........<br>........... | .........N.<br>......V. | .......<br>...E... | K......<br>....... | ...............<br>...............G | ....N.............<br>.........-STL. | ....<br>.... |

| | | | | | | | | |
|---|---|---|---|---|---|---|---|---|
| iPS:43 6746 | 21-225_154E10 | VL3\|3r/JL 2 | ............ | .......... | ........ | ....... | ................ | ....N........... | .... |
| | | | ........... | .......S | ........ | ....... | ......I..... | .........-ST.. | .... |
| iPS:43 6748 | 21-225_154D11 | VL3\|3r/JL 2 | ............ | .......... | ........ | ....... | ...............I. | ...H........... | .... |
| | | | ........... | ....... | ........ | ..K.... | ................ | .........-SI.. | .... |
| iPS:43 6756 | 21-225_146A10 | VL3\|3r/JL 2 | ............ | .......... | ........ | ....... | ................ | ............... | .... |
| | | | ........... | ......V. | ......F | ..R.... | ................ | .........-ST.. | V... |
| iPS:43 6758 | 21-225_155C10 | VL3\|3r/JL 2 | ............ | .......... | ........ | ....... | ................ | ............... | .... |
| | | | ......V.... | ......VS | ........ | ....... | ......I..... | .........-ST.. | .... |
| iPS:43 6760 | 21-225_155E10 | VL3\|3r/JL 2 | ............ | .......... | ........ | ....... | .T.............. | L.............. | .... |
| | | | ........... | ......VS | ........ | ..R.... | ................ | ..........F.. | .... |
| iPS:43 6764 | 21-225_158E9 | VL3\|3r/JL 2 | ..D......... | .......... | ........ | ....... | ................ | ....N........... | .... |
| | | | ........... | ......V. | ........ | ....... | ....T........N... | .........SF.L | .... |
| iPS:43 6766 | 21-225_158D10 | VL3\|3r/JL 2 | ...........T | .......... | ........ | ....... | .....L.......... | ...GN........... | .... |
| | | | ........... | ......V. | ........ | ..R.... | ..........L...... | .........SF.. | .... |
| iPS:43 6768 | 21-225_159H8 | VL3\|3r/JL 2 | ............ | .......... | ........ | ....... | ................ | ...GN........... | .... |
| | | | ........... | ......V. | ........ | ..R.... | ..........L...... | .........SF.. | .... |
| iPS:43 6770 | 21-225_160B12 | VL3\|3r/JL 2 | .D....S..... | .......... | ........ | ....... | ................ | ...GN........... | .... |
| | | | ........... | ......V. | ........ | ....... | ..........L...... | .........SF.. | .... |
| iPS:43 6772 | 21-225_161H3 | VL3\|3r/JL 2 | ............ | ......R.... | ........ | ....... | ................ | ...VN........... | .... |
| | | | ........... | ......V. | ......F | ..N.... | ..........L...... | ..........N.... | .... |
| iPS:43 6774 | 21-225_161E10 | VL3\|3r/JL 2 | .FD......... | .......... | ........ | ....... | .......I........ | .T..N........... | .... |
| | | | ........... | ......V. | ........ | ....... | ................ | .........SFAL | .... |
| iPS:43 6776 | 21-225_161F12 | VL3\|3r/JL 2 | ............ | .......... | ........ | ....... | ................ | ............... | .... |
| | | | ........... | ....... | ........ | ..T.... | ................ | .........-.TL. | .... |
| iPS:43 6780 | 21-225_165H3 | VL3\|3r/JL 2 | ............ | .......... | ........ | ....... | ................ | ............... | .... |
| | | | ........... | ....... | ........ | ....... | ................ | .........-.TL. | .... |
| iPS:43 6782 | 21-225_166G11 | VL3\|3r/JL 2 | ....S....... | .......... | ........ | ....... | ................ | ....N........... | .... |
| | | | ........... | ......VH | ........ | ....... | ................ | .........-STA. | .... |
| iPS:43 6784 | 21-225_169C1 | VL3\|3r/JL 2 | ............ | .......... | ........ | K...... | ................ | ....T........... | .... |
| | | | ........... | ......V. | ..L.... | ..I.... | ................ | .........-NT.I | .... |
| iPS:43 6786 | 21-225_169A6 | VL3\|3r/JL 2 | ............ | .......... | ...R.... | ....... | ................ | ....T........... | .... |
| | | | ........... | ......V. | ........ | ..Y.... | ................ | .........-NT.L | .... |
| iPS:43 6788 | 21-225_169B7 | VL3\|3r/JL 2 | A.D......... | .........G. | ........ | ....... | ................ | ....K........... | .... |
| | | | .......R... | .......S | ........ | ..R.... | ................ | ........-NT.. | .... |
| iPS:43 6790 | 21-225_169G11 | VL3\|3r/JL 2 | ............ | .......... | ........ | ....... | ................ | ....N........... | .... |
| | | | ........... | ....... | ........ | ....... | ................ | .........-STA. | .... |
| iPS:43 6794 | 21-225_170F1 | VL3\|3r/JL 2 | ....S....... | .......... | ........ | ....... | ...A............ | ............... | .... |
| | | | ........... | ......S. | ........ | ....... | ................ | .........-NTA. | .... |
| iPS:43 6796 | 21-225_170A5 | VL3\|3r/JL 2 | ..D......... | .......... | ........ | ....... | ................ | ....N........... | ....R |
| | | | ........... | ....... | ..I.... | ..Y.... | ................ | .........-STM. | .... |
| iPS:43 6798 | 21-225_171F5 | VL3\|3r/JL 2 | A.D......... | .........G. | ........ | ....... | ................ | ....K........... | .... |
| | | | ........... | .......S | ........ | ..R.... | ................ | .........-NT.. | .... |

| iPS:43 6802 | 21-225_171E12 | VL3|3r/JL 2 | ............ ............ | ............ ......... | ........ ....... | ...... ..R.... | ................ ................ | ....I........... ...........Y.. | ...... .... |
| iPS:43 6808 | 21-225_173F8 | VL3|3r/JL 2 | ............ ............ | ..N......N. ......V. | ....R... ......S | ........ ...R... | ................ ................ | ............... ..........-FT.. | .... .... |
| iPS:43 6812 | 21-225_175C6 | VL3|3r/JL 2 | ..D......... ............ | ............ ........ | ........ ..I..... | ........ ..Y.... | ................ ................ | ....N........... .........-STM. | .....R .... |
| iPS:43 6818 | 21-225_179C7 | VL3|3r/JL 2 | ............ ............ | ............ ......V. | ....R... ....... | ........ ........ | ................ ....R........... | ............... ..........N.... | .... .... |
| iPS:43 6822 | 21-225_180D4 | VL3|3r/JL 2 | ......T..... ............ | ......R.... ........ | ........ ....... | E...... ..R.... | ................ ................ | ............... .........-RK.. | .... .... |
| iPS:43 6824 | 21-225_180C5 | VL3|3r/JL 2 | ............ ............ | .........E. ........ | ........ ....... | ........ ..R.... | ................ ................ | ............G... .........-STA. | .... .... |
| iPS:43 6826 | 21-225_180G5 | VL3|3r/JL 2 | .Y.......... ............ | ......VS ........ | ........ ....... | ........ S....... | ................P. ................ | ....I........... ........-.TA. | .... .... |
| iPS:43 6828 | 21-225_181H1 | VL3|3r/JL 2 | .....I..... ............ | ............ ........ | ........ ....... | E...... ..R.... | ................ ................ | ............... .........-RK.. | .... .... |
| iPS:43 6838 | 21-225_52H1 | VL3|3r/JL 2 | ..........F ..........S | ............ ......VS | ........ ....... | ........ ..R.... | ................ ................ | ....N........... .........-ST.. | .... .... |
| iPS:43 6848 | 21-225_57F1 | VL3|3r/JL 2 | ..........A. ............ | ..........E. ........ | ........ ....... | ........ ..R.... | ................ ................ | ............... .........-ST.. | .... .... |
| iPS:43 6850 | 21-225_57D9 | VL3|3r/JL 2 | ............ ............ | ..E.......E. ....F.. | .S...... ....... | ........ ........ | ................ ................ | ............... .........-ST.. | .... .... |
| iPS:43 6852 | 21-225_57H11 | VL3|3r/JL 2 | ..A.......A. ............ | ..........E. ........ | ........ ....... | ........ ..R.... | ................ ................ | ............... .........-ST.. | .... .... |
| iPS:43 6854 | 21-225_58C1 | VL3|3r/JL 2 | ............ ......N... | .........N. ......... | ........ ....... | ........ ..R.... | ................ ................ | ....− .....-SSTA | .... .... |
| iPS:43 6858 | 21-225_58E7 | VL3|3r/JL 2 | ......S..... ............ | ............ ......T. | ..K.... ....... | ........ ..N.... | ................ ...........F. | ...NN........... .........-YT.. | ...... ..A. |
| iPS:43 6860 | 21-225_58F7 | VL3|3r/JL 2 | ............ ............ | ............ ........ | ........ ....... | ........ ..R.... | ................ ................ | ............... .........-ST.. | .... .... |
| iPS:43 6862 | 21-225_58F8 | VL3|3r/JL 2 | ............ ............ | ..........N. ........ | ........ ....... | ........ ........ | ................ ................ | ............... .........-ST.. | .... .... |
| iPS:43 6864 | 21-225_58G11 | VL3|3r/JL 2 | ............ ............ | ............ .......S | ........ ....... | ........ ..N.... | ................ ................ | ...NN........... .........-NT.M | .... .... |
| iPS:43 6866 | 21-225_59F2 | VL3|3r/JL 2 | ............ ............ | ............ .......S | ....R... ....... | ........ ..N.... | ................ ................ | ....N........... .........-NT.. | .... .... |
| iPS:43 6868 | 21-225_59B11 | VL3|3r/JL 2 | ............ ............ | ............ ........ | ........ ....... | ........ ........ | ..L............. ................ | ............... ..........Y.. | .... .... |
| iPS:43 6870 | 21-225_60B1 | VL3|3r/JL 2 | ..........A. ............ | .........E. ........ | ........ ....... | ........ ..R.... | ................ ................ | ............... .........-ST.. | .... .... |
| iPS:43 6872 | 21-225_60D2 | VL3|3r/JL 2 | ............ ............ | ..N........ .......S | ....R... ....... | ........ ..N.... | ................ ........T........ | ....N........... .........-NT.. | .... .... |

| iPS:43 6874 | 21-225_60A12 | VL3\|3r/JL 2 | . . . . . . . . . . . .<br>. . . . . . N . . . | . . . . . . . . . N .<br>. . . . . . . . . | . . . . . . . .<br>. . . . . . . | . . . . . . . .<br>. . R . . . . | I . . . . . . . . . . . . . . . . .<br>. . . . . . . . . . . . . . . . . . | . . . . −<br>. . . . −SSTA | . . . . . . .<br>. . . . |
| iPS:43 6876 | 21-225_61F5 | VL3\|3r/JL 2 | . . . . . . . . . . . .<br>. . . . . . . . . | . . . . . . . . . E .<br>. . . . . . . . | . . . . . . . .<br>. . . . . . . | . . . . . . . .<br>. . . . . . . | . . . . . . . . . . . . . . . . . .<br>. . . . . . . . . . . . . . . . . . | . . . . . . . . . . −ST . .<br> | . . . . .<br>. . . . |
| iPS:43 6878 | 21-225_62E3 | VL3\|3r/JL 2 | . . D . . . . . . . . .<br>. . . . . . . . . | . . . . . . . . . N .<br>. . . . . . . . | . . . . . . . .<br>. . . V . . . | . . . . . . . .<br>. . R . . . . | . . . . . . . . . . . . . . . . . .<br>. . . . . . . . . . . . . . . . . . | . . . . . . . . . −STA .<br> | . . . .<br>. . . . |
| iPS:43 6880 | 21-225_62E8 | VL3\|3r/JL 2 | . . D . . . . . . . . .<br>. . . . . . . . . | . . . . . . R . . N .<br>. . . . . . . S | . . . . . . . .<br>. . . V . . . | . . . . . . . .<br>. . R . . . . | . . . . . . . . . . . . . . . . . .<br>. . . . . . . . . . . . . . . . . . | . . . . . . . . . −STA .<br> | . . . .<br> |
| iPS:43 6882 | 21-225_62D10 | VL3\|3r/JL 2 | . . D . . . . . . . . .<br>. . . . . . . . . | . . . . . . . . . N .<br>. . . . . . . | . . . . . . . .<br>. . . V . . . | . . . . . . . .<br>. . R . . . . | . . . . . . . . . . . . . . . . . .<br>. . . . . . . . . . . . . . . . . . | . . . . . . . . . −STA .<br> | . . . .<br>. . . . |
| iPS:43 6884 | 21-225_62A12 | VL3\|3r/JL 2 | . . D . . . . . . . . .<br>. . . . . . . . | . . . . . . . . . N .<br>. . . . . . . . | . . . . . . . .<br>. . . V . . . | . . . . . . . .<br>. . R . . . . | . . . . . . . . . . . . . . . . . .<br>. . . . . . . . . . . . . . . . . . | . . . . . . . . . −STA .<br> | . . . .<br>. . . . |
| iPS:43 6886 | 21-225_62B12 | VL3\|3r/JL 2 | . . D . . . . . . . . .<br>. . . . . . . . | . . . . . . . . . N .<br>. . . . . . . T . | . . . . . . . .<br>. . V . . . | . . . . . . . .<br>. . R . . . . | . . . . . . . . . . . . . . . . . .<br>. . . . . . . . . . . . . . . . . . | . . . . . . . . . −STA .<br> | . . . .<br> |
| iPS:43 6892 | 21-225_65E9 | VL3\|3r/JL 2 | . . . . . . . . . . . .<br>. . . . . . . . . | . . . . . . . . . N .<br>. . . . . . DY | . . . . . . . .<br>. . . . . . . | . . . . . . . .<br>. . R . . . . | . . . . . . . . . . . . . . . . . .<br>. . . . . . . . . . . . . . . . . . | . . . . N . . . . . . . . . . . .<br>. . . . . . . . . −ST . . | . . . . .<br> |
| iPS:43 6894 | 21-225_66G9 | VL3\|3r/JL 2 | . . D . . . . . . . . T<br>. . . . . . . . . | . . . . . . . . . N .<br>. . . . . . . . | . . . . . . . .<br>. . . V . . . | . . . . . . . .<br>. . R . . . . | . . . . . . . . . . . . . . . . . .<br>. . . . . . . . . . . . . . . . . . | . . . . I . . . . . . . . . . . .<br>. . . . . . . . . −NTA . | . . . .<br> |
| iPS:43 6896 | 21-225_67F10 | VL3\|3r/JL 2 | . . . . . . . . . . . .<br>. . . . . . . . . | . . . . . . . . . Y .<br>. . . . . . . W | . . . . . . . .<br>. . . . . . F | E . . . . . .<br>. . R . . . . | . . . . . . . . . . . . . . . . . .<br>. . . . . . . . . . . . . . . . . . | . . . . N . . . . . . . . . . . .<br>. . . . . . . . . −ST . . | . . . . . R<br>. . . . |
| iPS:43 6898 | 21-225_68D8 | VL3\|3r/JL 2 | . . . . . . . . . . . .<br>. . . . . . . . . | . . . . . . . . . . .<br>. . . . . . . . | . . . . . . . .<br>. . . . . . . | . . . . . . . .<br>. . . . . . . | . . . . . . . . . . . . . . . . . .<br>. . . . . . . . . . . . . . . . . . | . . . . N . . . . . . . . . . . .<br>. . . . . . . . . −ST . . | . . . .<br> |
| iPS:43 6900 | 21-225_69B9 | VL3\|3r/JL 2 | . . . . . . . . . . . .<br>. . . . . . . . . | . . . . . . . . . N .<br>. . . . . DY | . . . . . . . .<br>. . . . . . . | . . . . . . . .<br>. . R . . . . | . . . . . . . . . . . . . . . . . .<br>. . . . . . . . . . . . . . . . . . | . . . . N . . . . . . . . . . . .<br>. . . . . . . . . −ST . . | . . . .<br> |
| iPS:43 6902 | 21-225_69B11 | VL3\|3r/JL 2 | . . . . . . . . . . . .<br>. . . . . . A . . . . . | . . . . . . . . . . .<br>. . . . . . . W | . . . . . . . .<br>. . . . . . . | E . . . . . .<br>. . R . . . . | . . . . . . . . . . . . . . . . . .<br>. . . . . . . . . . . . . . . . . . | . . . . N . . . . . . . . . . . .<br>. . . . . . . . . −ST . . | . . . .<br> |
| iPS:43 6904 | 21-225_71D4 | VL3\|3r/JL 2 | . . . . . . . . . . . .<br>. . . . . . . . . | . . . . . . . . . . .<br>. . . . . . . Y | . . . . . . . .<br>. . V . . . | . . . . . . . .<br>. . R . . . . | . . . . . . . . . . . . . . . . . .<br>. . . . . . . . . . . . . . . . . . | . . . VN . . . . . . . . . . . . .<br>. . . . . . . . . −ST . . | . . . . .<br> |
| iPS:43 6906 | 21-225_72B4 | VL3\|3r/JL 2 | . . . . . . . . . . . .<br>. . . . . . . . . | . . . . . . . . . . .<br>. . . . . . . W | . . . R . . .<br>. . . . . . . | E . . . . . .<br>. . R . . . . | . . . . . . . . . . . . . . . . . .<br>. . . . . . . . . . . . . . . . . . | . . . . N . . . . . . . . . . . .<br>. . . . . . . . . −ST . . | . . . .<br> |
| iPS:43 6908 | 21-225_72D5 | VL3\|3r/JL 2 | . . D . . . . . . . . .<br>. . . . . . . . . | . . . . . . . . . N .<br>. . . . . . . | . . . . . . . .<br>. . . V . . . | . . . . . . . .<br>. . R . . . . | . . . . . . . . . . . . . . . . . .<br>. . . . . . . . . . . . . . . . . . | . . . . . . . . . . −STA .<br> | . . . .<br> |
| iPS:43 6912 | 21-225_73C4 | VL3\|3r/JL 2 | . . D . . . . . . . . .<br>. . . . . . . . . | . . . . . . . . . N .<br>. . . . . . . | . . . . . . . .<br>. . . V . . . | . . . . . . . .<br>. . M . . . . | . . . . . . . . . . . . . . . . . .<br>. . . . . . . . . . . . . . . . . . | . . . . . . . . . . −STA .<br> | . . . .<br> |
| iPS:43 6914 | 21-225_76B4 | VL3\|3r/JL 2 | P . . . N . T . . . . .<br>. . . . . . . . . . . | . . . . . . R . . T .<br>. . . . . F . . | . . . . . . . .<br>. . . . . . . | . . . . . . . .<br>. . . . . . . | . . . . . . . . . . . . . . . . . .<br>. . . . . . . . . . . . . . . . . . | . . . . −<br>. . . . −SST . | . . . .<br> |
| iPS:43 6916 | 21-225_74A9 | VL3\|3r/JL 2 | . . . . . . . . . . . .<br>. . . . . . . . . . | . . . . . . . . . N .<br>. . . . . . V . | . . . . . . . .<br>. . . . . . . | . . . . . . . .<br>. . NR . . . | . . . . . . . . . . . . . . . . . .<br>. . . . . . . . . . . . . . . . . . | . . . . . . . . . −SP . I<br> | . . . . .<br> |
| iPS:43 6918 | 21-225_77A2 | VL3\|3r/JL 2 | . . . . . . . . . . .<br>. . . . . . . . . . | . . . . . . R . . . .<br>. . . . . . . . | . . . . . . . .<br>. . . . . . . | . . . . . . . .<br>. . R . . . . | . . . . . . . . . . . . . . . . . .<br>. . . . . . . . . . . . . . . . . . | . . . . . . . . . . −STA .<br> | . . . .<br> |

| iPS:43 6922 | 21-225_78E9 | VL3|3r/JL 2 | ..........Ē.<br>............ | ..........N.<br>......VS | .......<br>....... | .......<br>..NR... | ....................S..<br>.................... | ....................<br>.........-SP.I | .....<br>.... |
|---|---|---|---|---|---|---|---|---|---|
| iPS:43 6924 | 21-225_74B3 | VL3|3r/JL 2 | ...........<br>........... | ..........<br>.......... | .......<br>....... | .......<br>....... | ....................<br>.................... | ....................<br>..........-.T.. | ....<br>.... |
| iPS:43 6928 | 21-225_79E7 | VL3|3r/JL 2 | ...........<br>........... | ..........N.<br>......VS | .......<br>....... | .......<br>..NR... | ....................S..<br>.................... | ....................<br>.........-SP.I | ....<br>.... |
| iPS:43 6932 | 21-225_92A4 | VL3|3r/JL 2 | ...........<br>.......N... | ..........N.<br>......V. | .......<br>....... | .......<br>..NR... | ....................<br>.................... | ....................<br>.........-SP.I | ....<br>.... |
| iPS:43 6934 | 21-225_96B5 | VL3|3r/JL 2 | P...N.T.....<br>........... | ......R..T.<br>....F.. | .......<br>....... | .......<br>....... | ....................<br>.................... | ....-<br>....................<br>....-SST. | .....<br>.... |
| iPS:43 6936 | 21-225_97E6 | VL3|3r/JL 2 | ...........<br>......V.... | ..........N.<br>......VS | .......<br>....... | .......<br>..NR... | ....................S..<br>.................... | ....................<br>.........-.P.I | ....<br>.... |
| iPS:43 6938 | 21-225_146A3 | VL3|3r/JL 2 | ..AM......M.<br>........... | ..N......N.<br>....R... | .......<br>....... | .......<br>....... | ...................I.<br>.................... | ....................<br>.........-ST.. | ....<br>.... |
| iPS:43 6940 | 21-225_146B8 | VL3|3r/JL 2 | ...........<br>........... | ..........<br>......V. | .......<br>....... | .......<br>..R.... | ....................<br>.................... | ...H...........<br>.........-ST.. | ....<br>.... |
| iPS:43 6942 | 21-225_146H8 | VL3|3r/JL 2 | ...........<br>........... | ..........<br>.......... | .......<br>....... | .......<br>..K.... | ....................<br>........V......... | ....I...........<br>.........-RT.. | ....<br>.... |
| iPS:43 6944 | 21-225_182D12 | VL3|3r/JL 2 | ...........<br>........... | ..........E.<br>....... | ....S..<br>....... | .......<br>..R.... | ....................<br>.................... | ....................<br>.........-RTA. | ....<br>.... |
| iPS:43 6946 | 21-225_183F4 | VL3|3r/JL 2 | ...........<br>........... | ..........<br>....... | .......<br>....... | .......<br>..K.... | ....................<br>.................... | ...N...........<br>.................... | ....<br>.... |
| iPS:43 6952 | 21-225_185D2 | VL3|3r/JL 2 | ......T.....<br>........... | ..........<br>....... | .......<br>....... | E......<br>..R.... | ...D...............<br>.................... | ....................<br>.........-RK.. | .....<br>.... |
| iPS:43 6954 | 21-225_185G7 | VL3|3r/JL 2 | ...........<br>........... | .........H.<br>.....FV. | .......<br>....... | .......<br>..R.... | ....................<br>.................... | ....-<br>....................<br>....-SST. | ......<br>.... |
| iPS:43 6956 | 21-225_186H6 | VL3|3r/JL 2 | ...........<br>........... | ........M.E.<br>....... | .......<br>....... | .......<br>..R.... | ....................<br>.................... | ....................<br>.........-STA. | .....<br>.... |
| iPS:43 6962 | 21-225_190H1 | VL3|3r/JL 2 | ...........<br>........... | ......RF....<br>....RF.Y | .......<br>....... | .......<br>....... | ....................<br>.................... | K...................<br>.........-ST.. | ....<br>.... |
| iPS:43 6978 | 21-225_190G9 | VL3|3r/JL 2 | ...........<br>........... | ......RF....<br>....RF.Y | .......<br>....... | .......<br>..N.... | S...................<br>.................... | ....................<br>.........-ST.. | ....R<br>.... |
| iPS:43 7018 | 21-225_193H5 | VL3|3r/JL 2 | ......S...<br>........... | ..........<br>....RF.. | .......<br>....... | .......<br>....... | ....................<br>.................... | ....................<br>.........-STA. | ....<br>.... |
| iPS:43 7030 | 21-225_195E3 | VL3|3r/JL 2 | ...........<br>........... | .........Y.<br>....RSV. | .......<br>....... | E......<br>....... | ....................<br>.................... | ....................<br>.........-VT.. | .I..<br>.... |
| iPS:43 7034 | 21-225_195E9 | VL3|3r/JL 2 | ...........<br>........... | ..........N.<br>......Y | .......<br>....... | .......<br>.R.... | ....................G<br>.........G........ | ...R...........<br>.........-GI.. | ....<br>.... |
| iPS:43 7070 | 21-225_201G11 | VL3|3r/JL 2 | ...........<br>........... | ..........<br>....RF.. | .......<br>....... | .......<br>....... | ....................<br>.................... | ....................<br>.........-ST.. | ....<br>.... |

| IPS:43 7076 | 21-225_203G6 | VL3\|3r/JL2 | . . . . . . . . . . . | . . . . . . . . . . . | . . . . . . . . | . . . . . . . | . . . . . . . . . . . . . . . . . . | . . . . . . . . . . . . . . . . . . . | . . . . . |
|---|---|---|---|---|---|---|---|---|---|
| | | | . . . . . . . . . . . | . . . . RF . . | . . . . . . . | . . N . . . . | . . . . . . . . . S . . . . . . . . | . . . . . . . . . . . –ST . . | . . . . |
| IPS:43 7144 | 21-225_215B3 | VL3\|3r/JL2 | . . . . . . . . . . . | . . . . . . . . . . . | . . . . . . . | . . . . . . . | . . . . . . . . . . . . . . . . . . | . . . . . . . . . . . . . . . . . . . | . . . . . |
| | | | . . . . . . . . . . . | . . . . . F . . | . . . . . . . | . . . . . . . | . . . . . . . . . . . . . . . . . . | . . . . . . . . . . . . –ST . . | . . . . |
| IPS:43 7186 | 21-225_224H2 | VL3\|3r/JL2 | . . . . . . . S . . . | . . . . . . N . . V . | . . . . . . . | . . . . . . . | . . . . . . . . . . . . . . . . . . | . . . . . . . . . . . . . . . . . . . | . . . . . |
| | | | . . . . . . . . . . . | . . . . . . TY | . . . . . V . | . . . . . . . | . . . . . . . . . . . . . . . . . . | . . . . . . . . . . . –ST . . | . . . . |
| IPS:43 7192 | 21-225_225E9 | VL3\|3r/JL2 | . . D . . . . . . . . | . . . . . . . N . . N . | . . . . . . . | . . . . . . . | . . . . . . . . . . . . . . . . . . | . . . . . . . . . . . . . . . . . . . | . . . . . |
| | | | . . . . . . . . . . . | . . . . . R . . . | . . . . . M . | . . R . . . . | . . . . . . . . . . . . . . . . . . | . . . . . . . . . R . . . . | . . . . |
| IPS:43 7194 | 21-225_226B2 | VL3\|3r/JL2 | . . . . . . . . . . . | . . . . . . T . . G . | . . . . R . . . | . . . . . . . | . . . . . . . . . . . . . . . . . . | . . . N . . . . . . . . . . . . . . | . . . . . |
| | | | . . . . . . . . . . . | . . . . . . . . W | . . . . . . . | . . R . . . . | . . . . . . . . . S . . . . . . . | . . . . . . . . . –G . A . | . . . . |
| IPS:43 7200 | 21-225_226A10 | VL3\|3r/JL2 | . . . . . . . . . . . | . . . . . . T . . G . | . . . . . . . | . . . . . . . | . . . . . . . . . S . . . . . . . | . . . . N . . . . . . . . . . . . . | . . . . . |
| | | | . . . . . . . . . . . | . . . . . . . . W | . . . . . . . | . . R . . . . | . . . . . . . . . . . . . . . . . . | . . . . . . . . . –G . A . | . . . . |
| IPS:43 7204 | 21-225_227E5 | VL3\|3r/JL2 | . . . . S . . . . . . | . . . . . . . . . . E . | . . . . . . . | . . . . . . . | . . . . . . . . . . . . . . . . . K . | . . . VN . . . . . . . . . . | . . . . . |
| | | | . . . . . . . . . . . | . . . . . . . . . | . . . . . . . | . . R . . . . | . . . . . . . . . . . . . . . . . . | . . . . . . . . . –NTMI | . . . . |
| IPS:43 7210 | 21-225_227E12 | VL3\|3r/JL2 | . . . . . . . . . . . | . . . . . . . . . . . | . . . . . . . | . . . . . . . | . . . . . . . . . . . . . . . . . . | . . . N . . . . . . . . . . . . . . | . . . . . |
| | | | . . . . . . . . . . . | . . . . . V . | . . . . . . . | . . . . . . . | . . . . . . . . . . . . . . . . . . | . . . . . . . . . . –SN . . | . . . . |
| IPS:44 8908 | 21-225_50G9 | VL3\|3r/JL2 | . . . . . . . . . . . | . . . . . . . . . . . | . . . . . . . | . . . . . . . | . . . . . . . . . . . . . . . . . . | . . RN . . . . . . . . . . . . . | . . . . R |
| | | | . . . . . . . . . . . | . . . . . . . . | . . . . . . . | . . . . . . . | . . . . . . . . . R . . . E . . . | . . . . . . . . . . –RRG . | . . . . |
| IPS:45 1102 | 21-225_45F6 | VL3\|3r/JL2 | . . . . . . . . . . . | . . . . . . . . . . . | . . . . . . . | . . . . . . . | . . . . . . . . . . . . . . . . . . | . . . . N . . . . . . . . . . . . . | . . . . . |
| | | | . . . . . . . . . . . | . . . . . . . S | . . . . . . . | . . . . . . . | . . . . . . . . . . . . . . . . . . | . . . . . . . . . . –RTM . | . . . . |
| IPS:45 1110 | 21-225_74C9 | VL3\|3r/JL2 | . . . . . . . . . . E . | . . . . . . . S . N . | . . . . . . . | . . . . . . . | . . . . . . . . . . . . . . . . . . | . . . . . . . . . . . . . . . . . . . | . . . . . |
| | | | . . . . . . . . . . . | . . . . . . VS | . . . . . . . | . . NR . . . | . . . . . . . . . S . . . . . . . | . . . . . . . . . . –. P . I | . . . . |
| IPS:45 1112 | 21-225_53D10 | VL3\|3r/JL2 | . . . . . . . . . . . | . . . . . . . . . . N . | . . . . R . . . | . . . . . . . | . . . . . . . . . . . . . . . . . . | . . . . . . . . . . . . . . . . . . . | . . . . . |
| | | | . . . . . . . . . . . | . . . . . . . . | . . . . . . . | . . R . . . . | . . . . . . . . . . . . . . . . . . | . . . . . . . . . . –ST . . | . . . . |
| IPS:47 2731 | 21-225_14B1_LC2 | VL3\|3r/JL2 | . F . . . . . . . . . | . . . . . . . . . . . | . . . . . . . | . . . . . . . | . . . . . . . . . . . . . . . . . . | . . . . N . . . . . . . . . . . . . | . . . . . |
| | | | . . . . . . . . . . . | . . . . . . . Y | . . . . . . . | . . R . . . . | . . . . . . . . . . . . . . . . . . | . . . . . . . . . . –ST . . | . . . . |
| IPS:39 2583 | 21-225_10B10 | VL3\|3r/JL2 | . . . . . . . . . . . | . . . . . . . . . . N . | . . . . . . . | . . . . . . . | . . . . . . . . . . . . . . . . . . | . . . . N . . . . . . . . . . . . . | . . . . . |
| | | | . . . . . . . . . . . | . . . . . . . W | . . . . . . . | . . R . . . . | . . . . . . . . . . . . . . . . . . | . . . . . . . . . . –ST . . | . . . . |
| IPS:39 2585 | 21-225_14H11 | VL3\|3r/JL2 | T . . . . . . S . . . | . . . . . . . . . . E . | . . . . . . . | . . . . . . . | . . . . . . . . . . . . . . . . . . | . . . . – | . . . . . |
| | | | . . . . . . . . . . . | . . . . . . V . | . . . . . . . | . . T . . . . | | . . . . . –SSTI | . . . . |
| IPS:39 2587 | 21-225_18G5 | VL3\|3r/JL2 | . . . . . . . . . . . | . . E . . . . . . . | . . . . . . . | . . . . . . . | . . . . . . . . . . . . . . . . . . | . . . N . . . . . . . . . . . . . . | . . . . . |
| | | | . . . . . . . . . . . | . . . . . . V . | . . . . . . . | . . . . . . . | . . . . . . . . . . . . . . . . . . | . . . . . . . . . . –SN . . | . . . . |
| IPS:39 2589 | 21-225_27H2 | VL3\|3r/JL2 | . . . . . . . . . . . | . . . . . . . . . . . | . . . . . . . | . . . . . . . | . . . . . . . . . . . . . . . . . . | . . . . . . . . . . . . . . . . . . . | . . . . . |
| | | | . . . . . . . . . . . | . . . . . . . S | . . . . . . . | . . G . . . . | . . . L . . . . . . . . . . . . . | . . . . . . . . . . . . . Y . . | . . . . |
| IPS:39 2598 | 21-225_18E10 | VL3\|3r/JL2 | . . . . . . . . . . . | . . . . . . R . . . . | . . . . . . . | . . . . . . . | . . . . . . . . . . . . . . . . . . | . . . . . . . . . . . . . . . . . . . | . . . . . |
| | | | . . . . . . . . . . . | . . . . . . . W | . . . . . . . | . . R . . . . | . . . . . . . . . . . . . . . . . . | . . . . . . . . . . –ST . . | . . . . |
| IPS:39 3166 | 21-225_27G6 | VL3\|3r/JL2 | . . . . . . . . . . . | . . . . . . . . . . . | . . . . . . . | . . . . . . . | . . . . . . . . . . . . . . . . . . | . . . . . . . . . . . . . . . . . . . | . . . . . |
| | | | . . . . . . . . . . . | . . . . . . . . | . . . . . . . | . . R . . . . | . . . . . . . . T . . . . . . . . | . . . . . . . . . . . . SY . . | . . . . |
| IPS:39 3168 | 21-225_32B11 | VL3\|3r/JL2 | . . . . . . . . . . . | . . . . . . . . . . . | . . . . . . . | . . . . . . . | . . . . . . . . . . . . . . . . . . | . . . N . . . . . . . . . . . . . . | . . . . . |
| | | | . . . . . . . . . . . | . . . . . . . Y | . . . . . . . | . . . . . S . | . . . . . . . . . . . . . . . . . . | . . . . . . . . . . –ST . . | . . . . |
| IPS:39 3172 | 21-225_3B12 | VL3\|3r/JL2 | . . . . S . . . . . . | . . . . . . . . . . E . | . . . . . . . | . . . . . . . | . . . . . . . . . . . . . . . . . K . | . . . VN . . . . . . . . . . | . . . . . |
| | | | . . . . . . . . . . . | . . . . . . . . | . . . . . . . | . . R . . . . | . . . . . . . . . . . . . . . . . . | . . . . . . . . . –NTMI | . . . . |

| iPS:39 | clone | germline | | | | | | | |
|---|---|---|---|---|---|---|---|---|---|
| iPS:39 3176 | 21-225_27E7 | VL3\|3r/JL 2 | . . . . . . . . . . .<br>. . . . . . . . . . . | . . . . . . . . . N.<br>. . . . . . . . | . . . . . . .<br>. . . E . . . . | . . . . . . .<br>. . . . . . . L | . . . . . . . . . . . . . . . . . .<br>. . . . . . . . . . . . . . . . . . | . . . . . . . . . . . . . . . . . .<br>. . . . . . . . . - ST. . | . . . . .<br>. . . . |
| iPS:39 3178 | 21-225_34D7 | VL3\|3r/JL 2 | . . . . . . . . . . .<br>. . . . . . . . . . . | . . . . . . . . . E.<br>. . . . . . . Y | . . . . . . .<br>. . . . . L. | . . . . . . .<br>. . . . . . . | . . . . . . . . . . . . . . . . . .<br>. . . . . . . T . . . . F . . | . . . . N. . . . . . . . . . . . .<br>. . . . . . . . . . - . T . . | . . . . |
| iPS:39 3182 | 21-225_4B3 | VL3\|3r/JL 2 | . . . . . . . . . . .<br>. . . R . . V . . . . | . . . . . . . . . .<br>. . . . . . . . | . . . . . . .<br>. . . . . . . | . . . . . . .<br>. . R . . . . | . . . . . . . . . . . . . . . . . .<br>. . . . . . . . . . . . . . . . . . | . . . . N. . . . . . . . . . . . .<br>. . . . . . . . - NT . I | . . . . . |
| iPS:39 3184 | 21-225_15H11 | VL3\|3r/JL 2 | . . . . . . . . . . .<br>. . . . . . . . . . . | . . . . . . . . . E.<br>. . . . . . . . | . . . . . . .<br>. . . V . . . | . . . . . . .<br>. . R . . . . | . . . . . . . . . . . . . . . . . .<br>. . . . . . . I . . . . . . . . | . . . . . . . . . . - STA .<br>. . . . . . . . . . . . . . . . | . . . . |
| iPS:39 3186 | 21-225_27D9 | VL3\|3r/JL 2 | . . . . . . . . M.<br>. . . . . . . . . . . | . . Y . . . . . . .<br>. . . . . . . . | . F . . . . . .<br>. . . . . . . | . . . . . . . .<br>. . . . . . . | . . . . . . . . . . . . . . . . . .<br>. . . . . . . . . . . . . . . . . . | . . . V -<br>. . . . . - NNT . | . . . . . .<br>. . . . |
| iPS:39 3188 | 21-225_34B9 | VL3\|3r/JL 2 | . . . . . . A . . . . .<br>. . . . . . . . . . . | . . . . . . . . . E.<br>. . . . . . VS | . . . E . . . .<br>. . . . . . . | . . . . . . . .<br>. . . . . . . | . . . . . . . . . . . . . . . . . .<br>. . . . . . . . . . . . . . . . . . | . . . . -<br>. . . . - SST . | . . . . . .<br>. . . . |
| iPS:39 3192 | 21-225_12B1 | VL3\|3r/JL 2 | . . . . . . . . . . . .<br>. , . R . . V . . . . | . . . . . . . . . .<br>. . . . . . . . | . . . . . . . .<br>. . . . . . . | . . . . . . . .<br>. . R . . . . | . . . . . . . . . . . . . . . . . .<br>. . . . . . . . . . . . . . . . . . | . . . . N. . . . . . . . . . . . .<br>. . . . . . . . - NT . I | . . . . |
| iPS:39 3194 | 21-225_16D2 | VL3\|3r/JL 2 | . . . . . . . . . . . .<br>. . . . . . . . . . . | . . . . . . . . .<br>. . . . . . . . | . . . . . . . .<br>. . . . . . . | . . . . . . . .<br>. . . . . . . | . . . . . . . . . . . . . . . . . .<br>. . . . . . . . . . . . . . . . . . | . . . . . . . . . . . . . . . . . .<br>. . . . . . . . . . . . . Y . . | . . . . |
| iPS:39 3196 | 21-225_16G8 | VL3\|3r/JL 2 | . . . . S . . . . . . .<br>. . . . . . . . . . . | . . . . . . . . . E.<br>. . . . . . . . | . . . . . . . .<br>. . . . . . . | . . . . . . . .<br>. . R . . . . | . . . . . . . . . . . . . . . . . .<br>. . . . . . . . . . . . . . . K. | . . VN . . . . . . . . . . . .<br>. . . . . . . . - NTMI | . . . . |
| iPS:39 3198 | 21-225_28A11 | VL3\|3r/JL 2 | . . . . . . . . . . . .<br>. . . . . . . . . . . | . . . . . . . . .<br>. . . . . . . . | . . . . . . . .<br>. . . . . . . | . . . . . . . .<br>. . . . . . . | . . . . . . . . . . . . . . . . . .<br>. . . . . . . . . . . . . . . . . . | . . . . . . . . . . . . . . . . . .<br>. . . . . . . . . . . Y . . | . . . . |
| iPS:39 3200 | 21-225_35E1 | VL3\|3r/JL 2 | . . . . . . . . . . . .<br>. . . . . . . . . . . | . . . . . . . . . E.<br>. . . . . . . Y | . F . . . . . .<br>. . . I . . . | . . . . . . . .<br>. . R . . . . | . . . . . . . . . . . . . . . . . .<br>. . . . . . . . . . . . . . . . . . | . . . . N . . . . . . . . . . . . .<br>. . . . . . . . . - STA . | . . . . |
| iPS:39 3202 | 21-225_6B4 | VL3\|3r/JL 2 | . . . . . . . . . . . .<br>. . . R . . V . . . . | . . . . . . . . .<br>. . . . . . . | . . . . . . . .<br>. . . . . . . | . . . . . . . .<br>. . R . . . . | . . . . . . . . . . . . . . . . . .<br>. . . . . . . . . . . . . . . . . . | . . . N . . . . . . . . . . . . .<br>. . . . . . . . . - NT . I | . . . . |
| iPS:39 3206 | 21-225_13F6 | VL3\|3r/JL 2 | . . . . . . . . . . . .<br>. . . . . . . . . . . | . . . . . . . . .<br>. . . . . . . | . . . . . . . .<br>. . . . . . . | . . . . . . . .<br>. . . . . . . | . . . . . . . . . . . . . . . . . .<br>. . . . . . . . . . . . . . . . . . | . . . GN . . . . . . . . . . . .<br>. . . . . . . . . . . . . . . | . . . . . .<br>. . . . |
| iPS:39 3210 | 21-225_17D3 | VL3\|3r/JL 2 | . . . . . . S . . . . .<br>. . . . . . . . . . . | . . . . . . . . .<br>. . . . . . VY | . . . . . . . .<br>. . . V . . . | . . . . . . . .<br>. . R . . . . | . . . . . . . . . . . . . . . . . .<br>. . . . . . . . . . . . . . . . . . | . . . . . . . . . . . . . . . . . .<br>. . . . . . . . . - ITA . | . . . R |
| iPS:39 3212 | 21-225_30H6 | VL3\|3r/JL 2 | . . . . . . . . . . . .<br>. . . . . . . . . . . | . . . . . . . . , N.<br>. . . . . . . . | . . . . . . . .<br>. . . . . . . | . . . . . . . .<br>. . . . . . . | . . . . . . . . . . . . . . . . . .<br>. . . . . . . . . . . . . . . . . . | . . . . -<br>. . . . . - SST . | . . . . . .<br>. . . . |
| iPS:39 3214 | 21-225_33A1 | VL3\|3r/JL 2 | . . . . . . . . . . . .<br>. . . . . . . . . . . | . . . . . . . . .<br>. . . . . FVY | . . . . . . . .<br>. . . . . . . | . . . . . . . .<br>. . . . . . . | . . . . . . . . . . . . . . . . . .<br>. . . . . . . . . . . . . . . . . . | . . . . . . . . . . . . . . . . . .<br>. . . . . . . . . - . T . . | . . . . |
| iPS:39 3218 | 21-225_14G3 | VL3\|3r/JL 2 | . . . . . . . . . . . .<br>. . . . . . . . . . . | . . . . . . . . .<br>. . . . . . V . | . . . . . . . .<br>. . . . . . . | . . . . . . . .<br>. . R . . . . | . . . . . . . . . . . . . . . . . .<br>. . . . . . . . . . . . . . . . . . | . . . GN . . . . . . . . . . . .<br>. . . . . . . . . . . . . . . | . . . . |
| iPS:39 3222 | 21-225_17F5 | VL3\|3r/JL 2 | . . . . . . . . . . . .<br>. . . . . . . . . . . | . . . . . . . . E.<br>. . . . . . . | . . . . . . . .<br>. . . . . . . | . . . . . . . .<br>. . R . . . . | . . . . . . . . . . . . . . . . . .<br>. . . . . . . . . . . . . . . . . . | . . . . -<br>. . . . . - SST . | . . . . . .<br>. . . . |
| iPS:39 3224 | 21-225_31C2 | VL3\|3r/JL 2 | . . . . . . . . . . . .<br>. . . . . . . . . . . | . . . . . . . . . N.<br>. . . . . . . | . . . . . . . .<br>. . . . . . . | . . . . . . . .<br>. . . . . . . | . . . . . . . . . . . . . . . . . .<br>. . . . . . . . . . . . . . . . . . | . . . . -<br>. . . . - SST . | . . . . . .<br>. . . . |

| iPS | Clone | Germline | L_FR1 | L_CDR1 | L_FR2 | L_CDR2 | L_FR3 | L_CDR3 | L_FR4 |
|---|---|---|---|---|---|---|---|---|---|
| iPS:39 3226 | 21-225_33E6 | VL3\|3r/JL2 | ............<br>..........Y | ..........<br>......Y | .F.....<br>...I... | ........<br>..R.... | .................<br>................. | ....N..............<br>.........-STA. | ......<br>.... |
| iPS:39 3234 | 21-225_26C10 | VL3\|3r/JL2 | ...V......M.<br>...........V. | ..........<br>........V. | .F.....<br>....... | ........<br>........ | .................<br>................. | ...V-<br>....-NNT. | ......<br>.... |
| iPS:39 3345 | 21-225_5G7 | VL3\|3r/JL2 | ............<br>............ | .........N.<br>.......W | ........<br>....... | ........<br>..R.... | .................<br>................. | ....N..............<br>.........-ST.. | ......<br>.... |
| iPS:39 3565 | 21-225_34B11 | VL3\|3r/JL2 | ............<br>...........S | ..........<br>.......... | .......<br>...V... | ........<br>..M.... | .................<br>................. | ....N..............<br>.........-STA. | ......<br>.... |
| iPS:39 3950 | 21-225_3H10 | VL3\|3r/JL2 | .....S......<br>............ | .........E.<br>.......... | .......<br>....... | ........<br>..R.... | ...............K.<br>................. | ...VN..............<br>.........-NTMI | ......<br>.... |
| iPS:39 8470 | 21-225_14B7 | VL3\|3r/JL2 | ............<br>....R...... | .........N.<br>.......Y | .......<br>....... | ........<br>..R.... | ........T........<br>................. | ...NN..............<br>.........-ST.. | ......<br>.... |
| iPS:39 8472 | 21-225_16E4 | VL3\|3r/JL2 | P...........<br>............ | ..........<br>......VY | .....S..<br>........ | ........<br>........ | A................<br>................. | .........-ST..<br> | ......<br>.... |
| iPS:39 8488 | 21-225_19F6 | VL3\|3r/JL2 | ............<br>......V.... | ..........<br>.......... | ........<br>........ | .T.......<br>......... | .................<br>................. | ....N..............<br>.........-NT.. | ......<br>.... |
| iPS:39 8490 | 21-225_21D12 | VL3\|3r/JL2 | ............<br>............ | .........N.<br>.......Y | ........<br>....... | ........<br>..R.... | ..........F..<br>................. | ....N..............<br>.........-ST.. | .....R<br>.... |
| iPS:39 8498 | 21-225_22E6 | VL3\|3r/JL2 | ............<br>............ | .........E.<br>.......... | ........<br>........ | ........<br>..R.... | .....Y....T......<br>................. | .................<br>.........-STA. | ......<br>.... |
| iPS:39 8504 | 21-225_23D7 | VL3\|3r/JL2 | ............<br>............ | ..E.......<br>......V. | .......<br>...V... | ........<br>........ | .................<br>................. | ...N...............<br>.........-SN.. | ......<br>.... |
| iPS:39 8546 | 21-225_9H10 | VL3\|3r/JL2 | ............<br>............ | ..........<br>.......... | ........<br>........ | ........<br>........ | .................<br>................. | ...............Y..<br> | ......<br>.... |
| iPS:40 2225 | 21-225_2B1 | VL3\|3r/JL2 | ............<br>......V.... | ..........<br>.......... | ........<br>........ | ........<br>..R.... | .................<br>................. | ....N..............<br>.........-NT.. | ......<br>.... |
| iPS:40 2231 | 21-225_6D9 | VL3\|3r/JL2 | ............<br>............ | .........E.<br>.......... | ........<br>........ | ........<br>..K.... | .................<br>................. | .....-<br>....-SST. | ......<br>.... |
| iPS:40 4090 | 21-225_8D8 | VL3\|3r/JL2 | ............<br>............ | .........E.<br>.......... | .......<br>...V... | ........<br>..R.... | ..........I......<br>................. | .................<br>.........-STA. | ......<br>.... |
| iPS:42 3018 | 21-225_31D12_LC2 | VL3\|3r/JL2 | ............<br>............ | ..........<br>.......Y | .F.....<br>...I... | ........<br>..R.... | .................<br>................. | ....N..............<br>.........-STA. | ......<br>.... |

| | | Germline | L_FR1 | L_CDR1 | L_FR2 | L_CDR2 | L_FR3 | L_CDR3 | L_FR4 |
|---|---|---|---|---|---|---|---|---|---|
| | VL2\|2a2/JL3b | | QSALTQP-ASVSGSPGQSITISC | TGTS-SDVGGYNYVS | WYQQHPGKAFKLMIY | E--VSNRPS | GVSNRFSGSKSG--NTASLTISGLQAEDEADYYC | SSYTSS--------STLWV | FGGGTKLTVL |
| iPS:46 8862 | 21-225_178H8 | VL2\|2a2/JL3b | ......S.....<br>............ | ..........<br>.....F.. | ........<br>V..F... | ........<br>....... | ..P.............<br>................. | ......-<br>...YT.. | ......<br>.... |

| IPS:43 6838 | 21-225_52H4 | VL2\|2a2/JL3b | . . . . . . . . . . . .<br>. . . . . . . . . . | . . . . . . . . . . .<br>. . . . . . . . | . . . . . . . .<br>. . . . . . . . | . . . . . . . .<br>. . . . . . . | . . . . . . . . . . . . . . . . .<br>. . . . . . . . . . . . . . . . . . . | N. . . . -<br>. . . . . . . . . . . . . . . . . . . .<br>. . . NIT. . | . . . . . .<br>. . . . |
|---|---|---|---|---|---|---|---|---|---|
| IPS:43 7094 | 21-225_210D12 | VL2\|2a2/JL3b | . . . . . . . . . . . .<br>. . . . . . . . . . | . . . . . . . . . . .<br>. . . . . . . . | . . . . . . .V.<br>. . . .L. . | . . . . . . . .<br>. . . . . . . | . . . . . . . . . . . . . . . . .<br>. . . . . . . . . . . . . . . . . . . | N. . . . -<br>. . . . . . . . . . . . . . . . . . . .<br>. . . .IT. . | . . . . . .<br>. . . . |
| IPS:43 7096 | 21-225_210E12 | VL2\|2a2/JL3b | . . . . . . . . . . . .<br>. . . . . . . . . . | . . . . . . . . . . .<br>. . . . . . . . | . . . . . . . .<br>. . . . . . . | . . . . . . . .<br>. . . . . . . | . . . . . . . . . . . . . . . . .<br>. . . . . . . . . . . . . . . . . . . | G. .VK-<br>. . . . . . . . . . . . . . . . . . . .<br>. . .GIT. . | . . . . . .S<br>. . . . |
| IPS:43 7098 | 21-225_211C1 | VL2\|2a2/JL3b | . . . . . . . . . . . .<br>. . . . . . . . . . | . . . . . . . .S.<br>. . . . . . . . | . . . .Y. . .<br>. . . . . . . | . . . . . . . .<br>. . . . . . . | . . . . . . . . . . . . . . . . .<br>. . . . . . . . . . . . . . . . . . . | N. . . . -<br>. . . . . . . . . . . . . . . . . . . .<br>. . . .IT. . | . . . . . .<br>. . . . |
| IPS:43 7104 | 21-225_211G5 | VL2\|2a2/JL3b | . . . . . . . . . . . .<br>. . . . . . . . . . | . . . . . . . . . . .<br>. . . . . . . . | . . . . . . . .<br>. . . . . . . | . . . . . . . .<br>. . . . . . . | . . . . . . . . . . . . . . . . .<br>. . . . . . . . . . . . . . . . . . . | N. . .R-<br>. . . . . . . . . . . . . . . . . . . .<br>. . . .IT. . | . . . . . .<br>. . . . |
| IPS:43 7112 | 21-225_212C2 | VL2\|2a2/JL3b | . . . . . . . . . . . .<br>. . . . . . . . . . | . . . . . . . . . . .<br>. . . . . . . . | . . . . . . . .<br>. . . . . . . | . . . . . . . .<br>. .R. . . . | . . . . . . . . . . . . . . . . .<br>. . . . . . . . . . . . . . . . . . . | . . . .R-<br>. . . . . . . . . . . . . . . . . . . .<br>. . . .IT. . | . . . . . .<br>. . . . |
| IPS:43 7114 | 21-225_212A4 | VL2\|2a2/JL3b | . . . . . . . . . . . .<br>. . . . . . . . . . | . . . . . . . . . . .<br>. . . . . . . . | . . . . . . . .<br>. . . . . . . | . . . . . . . .<br>. . . . . . . | . . . . . . . . . . . . . . . . .<br>. . . . . . . . . . . . . . . . . . . | G. .VK-<br>. . . . . . . . . . . . . . . . . . . .<br>. . .GIT. . | . . . . . .S<br>. . . . |
| IPS:43 7116 | 21-225_212F6 | VL2\|2a2/JL3b | . . . . . . . . . . . .<br>. . . . . . . . . . | . . . . . . . . . . .<br>. . . . . . . . | . . . .Y. . .<br>. . . . . . . | . . . . . . . .<br>. . . . . . . | . . . . . . . . . . . . . . . . .<br>. . . . . . . . . . . . . . . . . . . | N. . . . -<br>. . . . . . . . . . . . . . . . . . . .<br>. . . .IT. . | . . . . . .<br>. . . . |
| IPS:43 7118 | 21-225_212G7 | VL2\|2a2/JL3b | . . . . . . . . . . . .<br>. . . . . . . . . . | . . . . . . . . . . .<br>. . . . . . . | T. . . . . . .<br>. . . . . . . | . . . . . . . .<br>. . . . . . . | . . . . . . . . . . . . . . . . .<br>. . . . . . . . . . . . . . . . . . . | N. . . . -<br>. . . . . . . . . . . . . . . . . . . .<br>. . . .IT. . | . . . . . .<br>. . . . |
| IPS:43 7128 | 21-225_213G3 | VL2\|2a2/JL3b | L. . . . . . . . . . .<br>. . . . . . . . . . | . . . . . . . . . . .<br>. . . . . . . . | . . . . . . . .<br>. . . . . . .S | . . . . . . . .<br>. .R. . . . | . . . . . . . . . . . . . . . . .<br>. . . . . . . . . . . . . . . . . . . | N. . .R-<br>. . . . . . . . . . . . . . . . . . . .<br>. . . .IT. . | . . . . . .<br>. . . . |
| IPS:43 7130 | 21-225_213D5 | VL2\|2a2/JL3b | . . . . . . . . . . . .<br>. . . . . . . . . . | . . . . . . . . . . .<br>. . . . . . . . | . . . . . . . .<br>. . . .V. . | . . . . . . . .<br>. .R. . . . | . . .T. . . . . . . . . . . .K.<br>. . . . . . . . . . . . . . . . . . . | C. . .R-<br>. . . . . . . . . . . . . . . . . . . .<br>. . .RIT. . | . . . . . .<br>. . . . |
| IPS:43 7146 | 21-225_215D3 | VL2\|2a2/JL3b | . . . . . . . . . . . .<br>. . . . . . . . . . | . . . . . . . .I. . .<br>. . . . . . . . | . . . . . . . .<br>. .T. . . . | . . . . . . . .<br>. . . . . . . | . . . . . . . . . . . . . . . . .<br>. . . . . . . . . . . . . . . . . . . | N. .KR-<br>. . . . . . . . . . . . . . . . . . . .<br>. . .GST. . | . . . . . .<br>V. . . |
| IPS:43 7150 | 21-225_216A3 | VL2\|2a2/JL3b | . . . . . . . . . . . .<br>. . . . . . . . . . | . . . . . . . . . . .<br>. . . . . . . . | . . . . . . . .<br>. . . . . . . | . . . . . . . .<br>. . . . . . . | . . . . . . . . . . . . . . . . .<br>. . . . . . . . . . . . . . . . . . . | N. . . . -<br>. . . . . . . . . . . . . . . . . . . .<br>. . . .IT. . | . . . . . .<br>. . . . |
| IPS:43 7162 | 21-225_217B2 | VL2\|2a2/JL3b | . . . . . . . . . . . .<br>. . . . . . . . . . | . . . . . . . . . . .<br>. . . . . . . | . . . . . . . .<br>. . . .L. . | . . . . . . . .<br>. . . . . . . | . . .Y. . . . . . . . . . . . .<br>. . . . . . . . . . . . . . . . . . . | G. .VK-<br>. . . . . . . . . . . . . . . . . . . .<br>. . .GIT. . | . . . . . .S<br>. . . . |

| ID | Clone | Germline | L_FR1 | L_CDR1 | L_FR2 | L_CDR2 | L_FR3 | L_CDR3 | L_FR4 |
|---|---|---|---|---|---|---|---|---|---|
| iPS:43 7172 | 21-225_219A7 | VL2\|2a2/ JL3b | ............ ............ | .......... ........ | ........ ....... | ........ ..R.... | ................ ................ | C...R- ................... ....IT.. | ...... .... |
| iPS:43 7182 | 21-225_221H2 | VL2\|2a2/ JL3b | L........... ............ | .......... ........ | ........ ......S | ........ ..R.... | ................ ................ | N...R- ................... ....IT.. | ...... .... |
| iPS:43 7184 | 21-225_221G4 | VL2\|2a2/ JL3b | ............ ............ | .......... ........ | ........ ....... | ........ ..R.... | ................ ................ | N...R- ................... ....IT.. | ...... .... |
| Germline | | | L_FR1 | L_CDR1 | L_FR2 | L_CDR2 | L_FR3 | L_CDR3 | L_FR4 |
| **VL1\|1c/JL2** | | | QSVLTQP-PSASGTPGQRVTISC | SGSS-SNIGS-----NTVN | WYQQLPGTAPKLLIY | S-NNQRPS | GVPDRFSGSKSG-TSASLAISGLQSEDEADYYC | AAWDDS---------LNGVV | FGGGTKLTVL |
| iPS:46 8864 | 21-225_60D6 | VL1\|1c/JL2 | ............ ............ | .......... ........ | ........ ....... | ........ ........ | ................ ................ | .....- ................... ...SLNGP | V..... .... |
| iPS:43 6660 | 21-225_146D8 | VL1\|1c/JL2 | .........T. ............ | ......Y.... .......D | ........ ........ | ........ ........ | ................ ................ | ................... ................... | ...... .... |
| iPS:43 6680 | 21-225_147H12 | VL1\|1c/JL2 | ............ ............ | ......... ....YA.. | ........ ........ | ........ ...H... | ................ ................ | E................... ...........P. | ...... .... |
| iPS:43 6682 | 21-225_146A8 | VL1\|1c/JL2 | ............ ............ | .......... .....SI. | ........ ......R. | ........ ..D.... | ................ ................ | ................... ................... | ...... .... |
| iPS:43 6684 | 21-225_146B6 | VL1\|1c/JL2 | ............ ............ | .......... .....A.. | ........ ........ | ........ ........ | ................ ................ | ................... ................... | ...... .... |
| iPS:43 6696 | 21-225_149A1 | VL1\|1c/JL2 | ............ ............ | .......... .....A.. | ........ ........ | ........ ........ | ................ ................ | ................... ................... | ...... .... |
| iPS:43 6712 | 21-225_150F9 | VL1\|1c/JL2 | ............ ............ | .......... .....A.. | ........ ........ | ........ ..S.... | ................ ..............F. | ................... ................... | ...... .... |
| iPS:43 6750 | 21-225_154G12 | VL1\|1c/JL2 | ............ ............ | .........N. .....A.S | ........ ........ | ........ ..DH... | ................ ................ | ................... ...........K.P. | ...... .... |
| iPS:43 6762 | 21-225_156H2 | VL1\|1c/JL2 | ...V....... ............ | .......... ........ | ........ ........ | ........ .S.... | ............L... ................ | ................... ................... | V... .... |
| iPS:43 7044 | 21-225_197F9 | VL1\|1c/JL2 | ............ ............ | .......... ........ | ........ ........ | ........ ........ | ................ ................ | ................... .........M..P. | ...... .... |
| iPS:43 7060 | 21-225_199C3 | VL1\|1c/JL2 | ............ ............ | .......... ........ | ........ ........ | ........ ........ | ................ ................ | ................... ...............P. | ...... .... |
| iPS:39 3180 | 21-225_4G12 | VL1\|1c/JL2 | ............ .......NM.. | ..TN...... ....Y... | ........ ........ | I....... ........ | ................ ................ | ................... .......LNGH.. | ...R.. .... |
| iPS:39 3230 | 21-225_9G9 | VL1\|1c/JL2 | ............ .......NM.. | ..TN...... ....Y... | ........ ........ | I....... ........ | ................ ................ | ................... ........LNGH.. | ...R.. .... |
| Germline | | | L_FR1 | L_CDR1 | L_FR2 | L_CDR2 | L_FR3 | L_CDR3 | L_FR4 |

| | | | L_FR1 | L_CDR1 | L_FR2 | L_CDR2 | L_FR3 | L_CDR3 | L_FR4 |
|---|---|---|---|---|---|---|---|---|---|
| **VL3\|3p/JL2** | | | SYELTQP-PSVSVSPGQTAR ITC | SGD---ALPK- ----KYAY | WYQQKSGQ APVLVIY | E------- -DSKRPS | GIPERFSGSSSG-- TMATLTISGAQVEDEAD YYC | YSTDSS----------- ----------GNHVV | FGGGTK LTVL |
| iPS:46 8866 | 21-225_190C1 | VL3\|3p/J L2 | ............ / ............ | T......M... / ........ | .D...... / ......S | ........ / ....... | ................ / P..........T..D. | N....- / ...SGNR. | ...... / .... |
| iPS:43 7214 | 21-225_48B12 | VL3\|3p/J L2 | ............ / .......... | .......... / ........ | ........ / ....... | ........ / ....... | ................ / ................ | N............... / ................ | ...... / .... |
| | | Germline | L_FR1 | L_CDR1 | L_FR2 | L_CDR2 | L_FR3 | L_CDR3 | L_FR4 |
| **VL1\|1c/JL3b** | | | QSVLTQP-PSASGTPGQRVT ISC | SGSS-SNIGS- ----NTVN | WYQQLPGT APKLLIY | S------- -NNQRPS | GVPDRFSGSKSG-- TSASLAISGLQSEDEAD YYC | AAWDDS----------- ----------LNGWV | FGGGTK LTVL |
| iPS:43 6234 | 21-225_51E3 | VL1\|1c/J L3b | ............ / ............ | ...N...... / .....I.T | ........ / ........ | ........ / ..D.... | ................ / ................ | T................ / ................ | .....T / .... |
| iPS:43 6830 | 21-225_51F4 | VL1\|1c/J L3b | ............ / ............ | .......... / .....I.T | ........ / ........ | ........ / ..D.... | ................ / ...........T.... | T................ / ................ | .....T / .... |
| iPS:43 6834 | 21-225_52F1 | VL1\|1c/J L3b | ............ / ............ | .......... / .....I.T | ........ / ........ | ........ / ..D.... | ................ / ................ | ................. / ................ | .....T / .... |
| iPS:43 6842 | 21-225_54E9 | VL1\|1c/J L3b | ............ / ............ | ...N.....N. / .....I.T | ........ / ........ | V....... / ..D.... | ................ / ................ | ................. / ................ | .....T / .... |
| iPS:43 6844 | 21-225_56G1 | VL1\|1c/J L3b | ............ / ............ | .......... / ....HI.T | ........ / ........ | ........ / ..D.... | ................ / ................ | .V............... / ............I... | .....T / .... |
| iPS:43 6846 | 21-225_56E3 | VL1\|1c/J L3b | ............ / ............ | .......... / .....I.T | ........ / ....... | ........ / ........ | ................ / ............C. | ................. / ................ | .....T / .... |
| iPS:43 7010 | 21-225_192G3 | VL1\|1c/J L3b | ............ / ........M.. | .......... / ........ | ........ / ....F... | G....... / ..K.... | R............... / ...........T.... | ................. / ................ | .....T / .... |
| iPS:43 7032 | 21-225_195H6 | VL1\|1c/J L3b | ............ / ............ | .......... / ....H... | ........ / ........ | N....... / ..Y.... | ................ / ..T........... | .T............... / ..........SV.. | .....T / V... |
| iPS:45 1104 | 21-225_49C5 | VL1\|1c/J L3b | ............ / ............ | .......... / .....I.T | ........ / ........ | ........ / ..D.... | ................ / ................ | T................ / ................ | .....T / .... |
| iPS:45 1106 | 21-225_49D10 | VL1\|1c/J L3b | ............ / ............ | ...N...... / .....I.T | ........ / ........ | ........ / ..D.... | ................ / ................ | ................. / ................ | .....T / .... |
| iPS:45 1108 | 21-225_53E8 | VL1\|1c/J L3b | ............ / ............ | ...C...... / .....I.T | ........ / ........ | ........ / ..D.... | ................ / ................ | T................ / ..............D.. | .....T / .... |
| | | Germline | L_FR1 | L_CDR1 | L_FR2 | L_CDR2 | L_FR3 | L_CDR3 | L_FR4 |
| **VL3\|3r/JL1** | | | SYELTQP-PSVSVSPGQTAS ITC | SGD---KLGD- ----KYAC | WYQQKPGQ SPVLVIY | Q------- -DSKRPS | GIPERFSGSNSG-- NTATLTISGTQAMDEAD YYC | QAWDS------------ ----------STAYV | FGTGTK VTVL |
| iPS:43 6662 | 21-225_147D7 | VL3\|3r/JL 1 | ............ / ............ | .......... / .....F.. | ........ / ........ | ........ / ..R.... | ................ / ................ | ....R............ / ..........-NTA. | .... / .... |
| iPS:43 6720 | 21-225_151H6 | VL3\|3r/JL 1 | ............ / .......... | .......... / ........ | ........ / ....... | ........ / ..T.... | ................ / ................ | ................. / ..........-ST.. | .... / .... |

3130

| iPS:43 6726 | 21-225_152G5 | VL3\|3r/JL 1 | ...M........ .......I... | .......... ......... | ........ ....... | ....... ....... | .............. ............ | ................ .........-ST.. | ...... .... |
|---|---|---|---|---|---|---|---|---|---|
| iPS:43 6732 | 21-225_152B12 | VL3\|3r/JL 1 | ............ .......... | .........N. ......... | ....... ....... | ....... ..T.... | .............. ............ | ................ .........-ST.. | ...... .... |
| iPS:43 6734 | 21-225_153A8 | VL3\|3r/JL 1 | ............ .......... | ......... ......... | ....... ....... | ....... ..T.... | .............. ............ | ................ .........-ST.. | ...... .... |
| iPS:43 6754 | 21-225_155G3 | VL3\|3r/JL 1 | ............ .......... | ......... ......V. | ..M.... ....... | ....... ....... | .............. ............ | ....N........... .........-SI.. | ...... .... |
| iPS:43 7190 | 21-225_225A9 | VL3\|3r/JL 1 | ............ .......... | .........N. ......... | ....... ....... | ....... ....... | .............. ............ | ................ .........N..C. | ...... .... |
| | Germline | | L_FR1 QAVLTQP- ASLSASPGASAS LTC | L_CDR1 TLRS- GINVGT---- YRIY | L_FR2 WYQQKPGS PPQYLLR | L_CDR2 YKS---- DSDKQQGS | L_FR3 GVPSRFSGSKDASANAG ILLISGLQSEDEADYYC | L_CDR3 MIWHS--------- --------SASWV | L_FR4 FGGGTK LTVL |
| | **VL5\|5c/JL3b** | | | | | | | | |
| iPS:43 6702 | 21-225_149E8 | VL5\|5c/J L3b | ...S....S... ............ | .......T.T. ......... | ......... ...F... | .T..... ...H... | ................ ..F............. | ................ .........-SA.. | ...... .... |
| | Germline | | L_FR1 QSVLTQP- PSVSGAPGQRVT ISC | L_CDR1 TGSS- SNIGAG---- YDVH | L_FR2 WYQQLPGT APKLLIY | L_CDR2 G------ -NSNRPS | L_FR3 GVPDRFSGSKSG--- TSASLAITGLQAEDEAD YYC | L_CDR3 QSYDSS--------- --------LSGVV | L_FR4 FGGGTK LTVL |
| | **VL1\|1e/JL2** | | | | | | | | |
| iPS:43 6752 | 21-225_155H1 | VL1\|1e/J L2 | ............ .......... | .......... ......... | ......... ......... | ....... ....... | ................ ...............H. | ................ ........LSGP.I | ...... .... |
| iPS:43 6820 | 21-225_179D10 | VL1\|1e/J L2 | ............ .......... | .......F.TD ......... | ....F... ....... | ........ .H..... | ................ ................ | ....R- ................ ...SLN.. | ...... .... |
| iPS:43 7188 | 21-225_224B11 | VL1\|1e/J L2 | ............ .......... | .......... ......... | ......... ......F | ........ ....... | ................ ...............H. | ....N- ................ ...SLSG. | ...... .... |
| iPS:43 7198 | 21-225_226F8 | VL1\|1e/J L2 | ............ .......... | .......... ......... | ......... ....... | ........ ....... | ...........D..... ................ | ....N- ................ ...SLSG. | ...... .... |
| iPS:43 7202 | 21-225_227D3 | VL1\|1e/J L2 | ............ .......... | .......... ....... | ......... ....... | ........ ....... | ...........D..... ................ | ....N- ................ ...SLSG. | ...... .... |
| iPS:43 7208 | 21-225_227C10 | VL1\|1e/J L2 | ............ .......... | .......... ....... | ......... ....... | ........ ....... | ...........D..... ................ | ....N- ................ ...NLSG. | ...... .... |
| iPS:44 3003 | 21-225_43F11_ LC2 | VL1\|1e/J L2 | ............ .......... | .......... ....... | ......... ....... | ........ ....... | ................ ................ | ....N........... ............S. | ...... .... |
| | Germline | | L_FR1 NFMLTQP- HSVSESPGKIVT ISC | L_CDR1 TRSS-GSIAS- -----NYVQ | L_FR2 WYQQRPGS SPTTVIY | L_CDR2 E------ -DNQRPS | L_FR3 GVPDRFSGSIDSSSNSA SLTISGLKTEDEADYYC | L_CDR3 QSYDS--------- --------SNWV | L_FR4 FGGGTK LTVL |
| | **VL6\|6a/JL3b** | | | | | | | | |

| | | | L_FR1 | L_CDR1 | L_FR2 | L_CDR2 | L_FR3 | L_CDR3 | L_FR4 |
|---|---|---|---|---|---|---|---|---|---|
| iPS:43 6792 | 21- 225_169D12 | VL6\|6a/J L3b | ...........<br>........... | ........TG.<br>........ | .H.....N<br>....L.. | .......<br>..KK... | ................<br>................ | ...Y.............<br>..........G... | ....<br>.... |
| | **VL3\|3l/JL2** | | **Germline** **L_FR1**<br>SSELTQD-<br>PAVSVALGQTVR<br>ITC | **L_CDR1**<br>QGD---SLRS-<br>----YYAS | **L_FR2**<br>WYQQKPGQ<br>APVLVIY | **L_CDR2**<br>G-------<br>-KNNRPS | **L_FR3**<br>GIPDRFSGSSSG--<br>NTASLTITGAQAEDEAD<br>YYC | **L_CDR3**<br>NSRDSS-----------<br>---------GNHVV | **L_FR4**<br>FGGGTK<br>LTVL |
| iPS:43 6800 | 21- 225_171D12 | VL3\|3l/JL 2 | ...........<br>........... | ...........<br>........ | ........<br>..I.... | A.......<br>....... | .........N......<br>................ | ................<br>..........S... | ......<br>.... |
| iPS:43 6804 | 21- 225_172C3 | VL3\|3l/JL 2 | ...........<br>........... | ........N.<br>......V. | ........<br>..I.... | T.......<br>...S... | .........T......<br>......T......... | ................<br>................ | ......<br>.... |
| iPS:43 6806 | 21- 225_172B12 | VL3\|3l/JL 2 | ...........<br>........... | ........N.<br>........ | ........<br>..I.... | T.......<br>...S... | .........T......<br>................ | ................<br>................ | ......<br>.... |
| iPS:43 6964 | 21- 225_190B3 | VL3\|3l/JL 2 | ...........<br>........... | ......K..T.<br>........ | ........<br>.....V. | .......<br>....... | ................<br>................ | ................<br>.........GNHL.L | ......<br>.... |
| iPS:43 6970 | 21- 225_190B8 | VL3\|3l/JL 2 | ...........<br>........... | ......T..P.<br>......V. | ........<br>....... | .......<br>....... | ................<br>................ | ................<br>..........GNHL.. | ......<br>.... |
| iPS:43 6980 | 21- 225_190C10 | VL3\|3l/JL 2 | ...........<br>........... | ........P.<br>........ | ........<br>....... | .......<br>....... | .....E..........<br>................ | ................<br>.......GNHL.. | ......<br>.... |
| iPS:43 6992 | 21- 225_191B8 | VL3\|3l/JL 2 | ...........<br>........... | ......T..P.<br>........ | ........<br>....... | .......<br>....... | ..S.............<br>................ | ................<br>..........GNHL.. | ......<br>.... |
| iPS:43 6994 | 21- 225_191A9 | VL3\|3l/JL 2 | ...........<br>........... | ........P.<br>........ | ........<br>....... | .......<br>....... | .....E..........<br>................ | .....C..........<br>.........GNHL.. | ......<br>.... |
| iPS:43 7016 | 21- 225_193A6 | VL3\|3l/JL 2 | ...........<br>........... | ........N<br>......F. | ........<br>....... | A.......<br>....... | .........N......<br>................ | ................<br>...............L. | ......<br>.... |
| | **VL2\|2b2/JL2** | | **Germline** **L_FR1**<br>QSALTQP-<br>ASVSGSPGQSIT<br>ISC | **L_CDR1**<br>TGTS-<br>SDVGSY----<br>NLVS | **L_FR2**<br>WYQQHPGK<br>APKLMIY | **L_CDR2**<br>E-------<br>VSKRPS | **L_FR3**<br>GVSNRFSGSKSG--<br>NTASLTISGLQAEDEAD<br>YYC | **L_CDR3**<br>CSYAGS-----------<br>---------STFVV | **L_FR4**<br>FGGGTK<br>LTVL |
| iPS:43 6810 | 21- 225_175F4 | VL2\|2b2/ JL2 | ...........<br>........... | .........RF<br>........ | .......Y<br>....... | .......<br>....... | ................<br>................ | ................<br>..........Y.. | ......<br>.... |
| iPS:43 6814 | 21- 225_178H10 | VL2\|2b2/ JL2 | ...........<br>........... | .........RF<br>........ | ...H...N<br>....... | .......<br>....... | ................<br>................ | ................<br>................ | ......<br>.... |
| | **VL3\|3l/JL3b** | | **Germline** **L_FR1**<br>SSELTQD-<br>PAVSVALGQTVR<br>ITC | **L_CDR1**<br>QGD---SLRS-<br>----YYAS | **L_FR2**<br>WYQQKPGQ<br>APVLVIY | **L_CDR2**<br>G-------<br>-KNNRPS | **L_FR3**<br>GIPDRFSGSSSG--<br>NTASLTITGAQAEDEAD<br>YYC | **L_CDR3**<br>NSRDSS-----------<br>---------GNHWV | **L_FR4**<br>FGGGTK<br>LTVL |
| iPS:43 6816 | 21- 225_179H5 | VL3\|3l/JL 3b | ...........<br>........... | .........N.<br>........ | ........<br>..F... | .......<br>....... | .........R......<br>................ | ................<br>................ | ......<br>.... |
| | **VL1\|1e/JL1** | | **Germline** **L_FR1**<br>QSVLTQP-<br>PSVSGAPGQRVT<br>ISC | **L_CDR1**<br>TGSS-<br>SNIGAG----<br>YDVH | **L_FR2**<br>WYQQLPGT<br>APKLLIY | **L_CDR2**<br>G-------<br>-NSNRPS | **L_FR3**<br>GVPDRFSGSKSG--<br>TSASLAITGLQAEDEAD<br>YYC | **L_CDR3**<br>QSYDSS-----------<br>---------LSGYV | **L_FR4**<br>FGTGTK<br>VIVL |

| ID | Clone | Gene | L_FR1 | L_CDR1 | L_FR2 | L_CDR2 | L_FR3 | L_CDR3 | L_FR4 |
|---|---|---|---|---|---|---|---|---|---|
| IPS:43 6832 | 21-225_51D8 | VL1\|1e/JL1 | ............ <br> ............ | ............ <br> ....FE.. | ........ <br> ........ | ....... <br> ....... | ................ <br> ................ | .................. <br> ................ | .... <br> .... |
| Germline | | | L_FR1 | L_CDR1 | L_FR2 | L_CDR2 | L_FR3 | L_CDR3 | L_FR4 |
| VL3\|3r/JL7 | | | SYELTQP-PSVSVSPGQIASITC | SGD---KLGD-----KYAC | WYQQKPGQSPVLVIY | Q-------DSKRPS | GIPERFSGSNSG--NTATLTISGTQAMDEADYYC | QAWDS------------STAAV | FGGGTQLTVL |
| IPS:43 6840 | 21-225_53E9 | VL3\|3r/JL7 | ............ <br> ............ | ..T........ <br> ......V. | ........ <br> ......N | ........ <br> ..TM... | ................ <br> ................ | .T................ <br> .........-ST.. | .....T <br> .... |
| IPS:43 6950 | 21-225_184G4 | VL3\|3r/JL7 | ............ <br> ............ | ............ <br> .....F.. | ........ <br> ........ | E....... <br> ..R.... | ................ <br> ................ | ................ <br> ........-RTV. | ..... <br> .... |
| Germline | | | L_FR1 | L_CDR1 | L_FR2 | L_CDR2 | L_FR3 | L_CDR3 | L_FR4 |
| VL1\|1b/JL2 | | | QSVLTQP-PSVSAAPGQKVTISC | SGSS-SNIGN----NYVS | WYQQLPGTAPKLLIY | D-------NNKRPS | GIPDRFSGSKSG--TSATLGITGLQTGDEADYYC | GTWDSS----------LSAVV | FGGGTKLTVL |
| IPS:43 6856 | 21-225_58C5 | VL1\|1b/JL2 | ............ <br> ............ | ........ <br> ........ | ........ <br> ........ | ........ <br> ........ | ................ <br> ................ | ....I........ <br> ...........VG. | ....... <br> .... |
| IPS:43 6960 | 21-225_198D2 | VL1\|1b/JL2 | ............ <br> ............ | .........S. <br> ........ | ........ <br> ...V... | ........ <br> ........ | ................ <br> ................ | .....R........ <br> ..........NVG. | ....... <br> .... |
| IPS:43 6966 | 21-225_190C3 | VL1\|1b/JL2 | ............ <br> ............ | ........ <br> ........ | ........ <br> ....L. | ........ <br> .S..... | ...G........ <br> ................ | ................ <br> ............T.. | ....... <br> .... |
| IPS:43 6968 | 21-225_190B10 | VL1\|1b/JL2 | ............ <br> ............ | ........ <br> ........ | ........ <br> ...H.... | ........ <br> ........ | ................ <br> ................ | ................ <br> ...........G. | ....... <br> .... |
| IPS:43 6974 | 21-225_190H7 | VL1\|1b/JL2 | ............ <br> ............ | .........S. <br> ........ | ........ <br> ...V... | ........ <br> ........ | ................ <br> ................ | ....GR........ <br> ..........NVG. | ....... <br> .... |
| IPS:43 6976 | 21-225_190D8 | VL1\|1b/JL2 | ............ <br> ............ | ........ <br> .....H... | ........ <br> ........ | ........ <br> .SS.... | E............... <br> ................ | ................ <br> ............T.. | ....... <br> .... |
| IPS:43 6982 | 21-225_190D10 | VL1\|1b/JL2 | ............ <br> ............ | .........S. <br> ........ | ........ <br> V..V... | ........ <br> ........ | ................ <br> ................ | .....R........ <br> ..........NVG. | ....... <br> .... |
| IPS:43 6986 | 21-225_191A1 | VL1\|1b/JL2 | ............ <br> .....R..... | .......L... <br> ....F.. | ....F... <br> ........ | ........ <br> ..Y.... | .......V........ <br> ................ | ................ <br> ..........NTG. | ....... <br> .... |
| IPS:43 7006 | 21-225_192G2 | VL1\|1b/JL2 | ............ <br> ............ | ........ <br> ........ | ........ <br> ........ | ........ <br> ........ | R............... <br> ................ | ................ <br> ...........G. | ....... <br> .... |
| IPS:43 7024 | 21-225_194F11 | VL1\|1b/JL2 | ............ <br> ............ | ........ <br> ........ | ........ <br> ........ | ........ <br> ........ | ................ <br> ................ | ................ <br> ...........G. | ....... <br> .... |
| IPS:43 7028 | 21-225_194G12 | VL1\|1b/JL2 | ............ <br> ............ | ........ <br> ........ | ........ <br> ........ | ........ <br> ........ | R............... <br> ..........E..... | ................ <br> ...........VG. | ....... <br> .... |
| IPS:43 7042 | 21-225_197E8 | VL1\|1b/JL2 | ............ <br> ............ | ........ <br> ....K... | ....F... <br> ........ | ........ <br> ........ | K............... <br> ......L........ | .I..R........ <br> ...........VM. | ....... <br> .... |
| IPS:43 7064 | 21-225_200G8 | VL1\|1b/JL2 | ............ <br> .....R..... | .......L... <br> .....F.. | ....F... <br> ........ | ........ <br> ..Y.... | .......V........ <br> ................ | ................ <br> ..........NTG. | ....... <br> .... |
| IPS:43 7086 | 21-225_209A8 | VL1\|1b/JL2 | ............ <br> ............ | .........S. <br> .....FL. | ........ <br> ........ | ........ <br> ........ | ................ <br> ................ | ................ <br> ...........G. | ....... <br> .... |

| | | | L_FR1 | L_CDR1 | L_FR2 | L_CDR2 | L_FR3 | L_CDR3 | L_FR4 |
|---|---|---|---|---|---|---|---|---|---|
| IPS:43 7138 | 21-225_214D8 | VL1\|1b/JL2 | ............. | .......... | ....F... | ........ | ................. | .A............... | I...S. |
| | | | ............. | ........ | ......H | ....... | ................. | ................ | .... |
| IPS:43 7168 | 21-225_218G4 | VL1\|1b/JL2 | ............. | .......... | ........ | ........ | ...A............. | ................. | .... |
| | | | ............. | ........ | .....L. | .S.... | ................. | ..........NT.. | .... |
| Germline | | | L_FR1 | L_CDR1 | L_FR2 | L_CDR2 | L_FR3 | L_CDR3 | L_FR4 |
| VL6\|6a/JL2 | | | NFMLTQP-HSVSESPGKIVTISC | TRSS-GSIAS-----NYVQ | WYQQRPGS SPTTVIY | E--------DNQRPS | GVPDRFSGSIDSSSNSA SLTISGLKTEDEADYYC | QSYDS----------SNVV | FGGGTK LTVL |
| IPS:43 6888 | 21-225_63G7 | VL6\|6a/JL2 | ............. | ...N....V.. | ........ | ........ | ................. | ....G............ | .... |
| | | | ............. | ........ | ....M.. | ..SR... | ...............S. | ..........I... | .... |
| IPS:43 6890 | 21-225_63A10 | VL6\|6a/JL2 | ............. | ...N....V.. | ........ | ........ | ................. | ................. | .... |
| | | | ............. | ........ | ........ | ..KR... | ................. | ..........I... | .... |
| Germline | | | L_FR1 | L_CDR1 | L_FR2 | L_CDR2 | L_FR3 | L_CDR3 | L_FR4 |
| VL8\|8a/JL3b | | | QTVVTQE-PSFSVSPGGIVTLTC | GLSS-GSVTS----YYPS | WYQQTPGQ APRTLIY | S--------INTRSS | GVPDRFSGSILG--NKAALTITGAQADDESD YYC | VLYMG-----------SGIWV | FGGGTK LTVL |
| IPS:43 6910 | 21-225_73G1 | VL8\|8a/JL3b | ............. | .......... | ........ | N....... | ................. | ................. | .... |
| | | | ............. | ........ | ....... | ....... | ................. | ..........A... | .... |
| IPS:43 6948 | 21-225_183F5 | VL8\|8a/JL3b | ............. | ..........T | ........ | N....... | ................. | ................. | .... |
| | | | ............. | .....F.. | ....... | ....... | ................. | ................. | .... |
| Germline | | | L_FR1 | L_CDR1 | L_FR2 | L_CDR2 | L_FR3 | L_CDR3 | L_FR4 |
| VL7\|7a/JL2 | | | QTVVTQE-PSLIVSPGGIVTLTC | ASST-GAVTSG----YYPN | WFQQKPGQ APRALIY | S--------TSNKHS | WTPARFSGSLLG--GKAALTLSGVQPEDEAE YYC | LLYYG-----------GAQVV | FGGGTK LTVL |
| IPS:43 6920 | 21-225_74E5 | VL7\|7a/JL2 | ............. | .....ET.... | ........ | ........ | ................. | ................. | .... |
| | | | ............. | .....S... | ....... | ....... | .....D........... | ..............L. | .... |
| IPS:43 6926 | 21-225_78D10 | VL7\|7a/JL2 | ............. | .....F.. | ........ | ........ | ................. | ................. | .... |
| | | | ............. | ........ | ....... | ..D.... | ................. | ..........LM | .... |
| IPS:43 6958 | 21-225_190D1 | VL7\|7a/JL2 | ............. | ....S... | ........ | ........ | ................. | ................. | .... |
| | | | ............. | ........ | ....... | ....... | ..........D... | ..............A | .... |
| IPS:43 6984 | 21-225_190F10 | VL7\|7a/JL2 | ............. | VF........ | ........ | ........ | ................. | .....C........... | .... |
| | | | ............. | ....SF.. | ....... | ....... | ..........D... | ..............L. | .... |
| IPS:43 6988 | 21-225_191A2 | VL7\|7a/JL2 | ............. | VL........ | ........ | ........ | ................. | M..C............ | .... |
| | | | ............. | ....SF.. | ....... | ....... | ..........D... | ..............L. | .... |
| IPS:43 7002 | 21-225_191H9 | VL7\|7a/JL2 | ............. | ..........A | .L...... | ........ | ................. | .IF.............. | .... |
| | | | ............. | ........ | ...T... | ..N.... | .....D........... | ............VH.I | .... |
| IPS:43 7008 | 21-225_192E3 | VL7\|7a/JL2 | ............. | .F....S.... | ........ | ........ | ................. | ................. | .... |
| | | | ............. | ....S... | ....... | ..N.... | .......R......... | ..............L. | .... |
| IPS:43 7012 | 21-225_192G7 | VL7\|7a/JL2 | ............. | ....N..Q | ........ | ........ | ................. | .F............... | .... |
| | | | ............. | ........ | ....... | ..T.R.. | ................. | ...............I | .... |
| IPS:43 7014 | 21-225_192H8 | VL7\|7a/JL2 | ............. | .F....T.... | ........ | N....... | ..............M. | ................. | .... |
| | | | ............. | ....F... | ....... | ....R.. | ............D... | ..........LM | .... |

| | | | | | | | | | |
|---|---|---|---|---|---|---|---|---|---|
| iPS:43 7022 | 21-225_194G5 | VL7\|7a/J L2 | ............ <br> ........... | .......... <br> ....N... | ....... <br> T...... | ....... <br> ....... | ................G <br> ...................... | .I................. <br> ...........LM | ..... <br> .... |
| iPS:43 7026 | 21-225_194D12 | VL7\|7a/J L2 | ............ <br> ........... | .......... <br> ....SF.S | ....... <br> ....... | ....... <br> ....R.. | S............... <br> ..............D... | .I................. <br> ...........LA | ..... <br> .... |
| iPS:43 7040 | 21-225_196E7 | VL7\|7a/J L2 | ............ <br> ........... | ..........A <br> ....... | .L...... <br> ...T... | ....... <br> ..N.... | <br> .....D........... | .IF............... <br> ..........VH.I | .... <br> ... |
| iPS:43 7048 | 21-225_197B11 | VL7\|7a/J L2 | ............ <br> ........... | .F....S.... <br> ....S... | ....... <br> ....... | ....... <br> ..N.... | ................... <br> ................... | ................... <br> .............L. | .... <br> .... |
| iPS:43 7050 | 21-225_197C11 | VL7\|7a/J L2 | ............ <br> ........... | ..........A <br> ....... | .L...... <br> ...T... | ....... <br> ....... | ................... <br> .....D........... | .IF............... <br> ..........VH.I | .... <br> ... |
| iPS:43 7056 | 21-225_198B8 | VL7\|7a/J L2 | ........S... <br> ........... | VL......... <br> ....SF.. | ....... <br> ....... | ....... <br> ....... | ................... <br> ..............D..F | M..S............... <br> ...............M. | .... <br> .... |
| iPS:43 7062 | 21-225_200H1 | VL7\|7a/J L2 | ............ <br> ........... | ..N....... <br> ....S... | ....... <br> ....... | H...... <br> ..N.... | ................... <br> ................... | .I................. <br> .............L. | ..... <br> .... |
| iPS:43 7066 | 21-225_200G9 | VL7\|7a/J L2 | ............ <br> ........... | ..N....... <br> ....S... | .L...... <br> ....... | H...... <br> ..D.... | ................... <br> ......A........... | .I................. <br> .............L. | ..... <br> .... |
| iPS:43 7068 | 21-225_200A11 | VL7\|7a/J L2 | ............ <br> ........... | ....... <br> ....... | ....... <br> V...... | ....... <br> ..N.... | ................... <br> ...............D... | ................... <br> ..........HLA | .... <br> .... |
| iPS:43 7090 | 21-225_210F11 | VL7\|7a/J L2 | ............ <br> ........... | .F......... <br> ....N... | ....... <br> ....... | ....... <br> ....... | ................... <br> ................... | ................... <br> .............L. | .... <br> .... |
| iPS:43 7106 | 21-225_211H7 | VL7\|7a/J L2 | ............ <br> ........... | .F......... <br> ....N..S | ....... <br> V...... | ....... <br> ....R.. | ................... <br> ................... | ................... <br> .............L. | .... <br> .... |
| iPS:43 7108 | 21-225_211C9 | VL7\|7a/J L2 | ............ <br> ........... | G.....S.... <br> .....F.. | ....... <br> ...P... | ....... <br> ..N.... | ................... <br> ........D......D... | ................... <br> .............LA | .... <br> .... |
| iPS:43 7110 | 21-225_211E9 | VL7\|7a/J L2 | ............ <br> ........... | ........... <br> ....N... | ....... <br> ....... | ....... <br> ..I.... | G.....T.F....... <br> ................... | ................... <br> .............LA | .... <br> .... |
| iPS:43 7120 | 21-225_212A9 | VL7\|7a/J L2 | ............ <br> ........... | ........... <br> ....... | ....... <br> ....... | ....... <br> ..N.... | ................... <br> .........D...D... | ................... <br> .............G | .... <br> .... |
| iPS:43 7124 | 21-225_212H12 | VL7\|7a/J L2 | ............ <br> ........... | ........... <br> ....... | ....... <br> ....... | ....... <br> ....... | ................... <br> ................... | ................... <br> ............H.. | .... <br> .... |
| iPS:43 7132 | 21-225_213F5 | VL7\|7a/J L2 | ............ <br> ........... | G.....S.... <br> .....F.. | ....... <br> T..P... | ....... <br> ..N.... | ..................T <br> ......D......D... | ...F............... <br> .............LA | .... <br> .... |
| iPS:43 7136 | 21-225_214H3 | VL7\|7a/J L2 | ............ <br> ........... | ........... <br> ....... | ....... <br> ....... | ....... <br> ....... | C............... <br> ................... | ................... <br> ............H.. | .... <br> .... |
| iPS:43 7140 | 21-225_214E12 | VL7\|7a/J L2 | ............ <br> ........... | ........... <br> ....... | ....... <br> ....... | ....... <br> ....... | ................... <br> ......D........... | ...CD............. <br> .............L. | .... <br> .... |
| iPS:43 7142 | 21-225_215A3 | VL7\|7a/J L2 | ............ <br> ........... | .....E.... <br> ....N..S | ....... <br> ....... | ....... <br> ....... | G.....T......... <br> ................... | ................... <br> .............LA | .... <br> .A.. |
| iPS:43 7148 | 21-225_215H3 | VL7\|7a/J L2 | ............ <br> ........... | ........... <br> ....... | ....... <br> ....... | ....... <br> ..N.... | CG............... <br> ........D...D... | ................... <br> .............G | .... <br> .... |
| iPS:43 7154 | 21-225 216A7 | VL7\|7a/J L2 | ............ <br> ........... | ....... <br> ....... | ....... <br> ....... | ....... <br> ..N.... | C............... <br> .........D...D... | ................... <br> .............G | .... <br> .... |

| IPS:43 | 21-225 | VL/J | L_FR1 | L_CDR1 | L_FR2 | L_CDR2 | L_FR3 | L_CDR3 | L_FR4 |
|---|---|---|---|---|---|---|---|---|---|
| IPS:43 7158 | 21-225_216H11 | VL7\|7a/J L2 | ............. / ............ | ........... / ........ | ........ / ....... | ....... / ....... | ............... / .....D...... | ...CD........... / ...........L. | .... / .... |
| | **VL1\|1b/JL3b** | | QSVLTQP-PSVSAAPGQKVTISC | SGSS-SNIGN-----NYVS | WYQQLPGTAPKLLIY | D-------NNKRPS | GIPDRFSGSKSG--TSATLGITGLQTGDEADYYC | GTWDSS--------------LSAWV | FGGGTKLTVL |
| IPS:43 6972 | 21-225_190C7 | VL1\|1b/JL3b | ............. / ............ | ..G........ / ........ | .F..F... / ...F... | ........ / ....... | ............... / ............ | ....RT........... / ...........D.. | .... / .... |
| IPS:43 7020 | 21-225_193F11 | VL1\|1b/JL3b | .Y........... / ............ | F.G........ / ........ | .F..F... / ...F... | ........ / ....... | ..L..L....... / ..D.....N....... | ....RT........... / .........M.D.. | .... / .... |
| IPS:43 7036 | 21-225_195H9 | VL1\|1b/JL3b | .Y........... / ............ | ..G........ / ........ | .F..F... / ...F... | ........ / ....... | ..L............ / ............ | ....RT........... / .........M.D.. | .... / .... |
| | **VL1\|1b/JL1** | | QSVLTQP-PSVSAAPGQKVTISC | SGSS-SNIGN-----NYVS | WYQQLPGTAPKLLIY | D-------NNKRPS | GIPDRFSGSKSG--TSATLGITGLQTGDEADYYC | GTWDSS--------------LSAYV | FGTGTKVTVL |
| IPS:43 6996 | 21-225_191B9 | VL1\|1b/JL1 | ............. / ............ | ........... / ........ | ........ / ....... | ..K.... / ....... | ............... / ............ | ...........VC. / ............ | .... / .... |
| IPS:43 7054 | 21-225_194G3 | VL1\|1b/JL1 | ............. / ............ | ........... / ......I. | ........ / ....... | ..K.... / ....... | ............... / ............ | ...........VC. / ............ | .... / .... |
| IPS:43 7058 | 21-225_199F3 | VL1\|1b/JL1 | ............. / ............ | ........... / ........ | ........ / ....... | ....... / ....... | ............... / ............ | ...........C. / ............ | .... / .... |
| | **VL5\|5c/JL2** | | QAVLTQP-ASLSASPGASASLTC | TLRSGINVGT----YRIY | WYQQKPGSPPQYLLR | YKSDSDKQQGS | GVPSRFSGSKDASANAGILLISGLQSEDEADYYC | MIWHS---------SASVV | FGGGTKLTVL |
| IPS:43 7000 | 21-225_191G9 | VL5\|5c/JL2 | ...S.R..S.... / ............ | ........... / ........ | ........ / ....... | ....... / ....... | ............... / ............ | ...........-SA.. / ............ | .... / .... |
| | **VL3\|3j/JL2** | | SYELTQP-LSVSVALGQTARITC | GGN--NIGS-----KNVH | WYQQKPGQAPVLVIY | R-------DSNRPS | GIPERFSGSNSG--NTATLTISRAQAGDEADYYC | QVWDS---------STAVV | FGGGTKLTVL |
| IPS:43 7074 | 21-225_203B2 | VL3\|3j/JL2 | ............. / ............ | .........R. / ........ | ........ / S...I.H | .......  / ...D... | ............... / ............ | ............... / ..........-GTA. | .... / .P.. |
| IPS:43 7082 | 21-225_205E12 | VL3\|3j/JL2 | ............. / A......... | .........R. / ........ | ........ / S...I.H | ....... / ...D... | ............... / ............ | ............... / ..........-GTA. | .... / .P.. |
| IPS:43 7084 | 21-225_206B5 | VL3\|3j/JL2 | ............. / .........A. | .........R. / ........ | .......L / ...P..X | ........ / ...Y.S. | ...D......C.....T / .V.......E..E... | .D............. / ..........-ST.. | .... / .... |
| IPS:43 7088 | 21-225_209H10 | VL3\|3j/JL2 | ............. / .........A. | .........R. / ........ | .......L / ...P..L | ........ / ...Y.S. | ...D......W...... / .V.......E..E... | .D............. / ..........-ST.. | .... / .... |
| IPS:43 7100 | 21-225_211H2 | VL3\|3j/JL2 | ............. / ............ | .........R. / ....R... | ........ / ..I.... | ........ / ..RD... | ............... / ..............F. | ............... / ..........-STA. | .... / .... |

| | | | L_FR1 | L_CDR1 | L_FR2 | L_CDR2 | L_FR3 | L_CDR3 | L_FR4 |
|---|---|---|---|---|---|---|---|---|---|
| iPS:43 7160 | 21-225_216B12 | VL3\|3j/JL2 | ............<br>............ | ..D.....RR.<br>....R... | ........<br>........ | .......<br>....... | .................<br>................. | .................<br>..........-STG. | .....<br>.... |
| iPS:39 2593 | 21-225_3E10 | VL3\|3j/JL2 | .......H...<br>..TA.M..... | ............<br>.....A.. | ........<br>D...... | S.......<br>....... | ..........P......<br>......IE......... | ......S...........<br>..........DH.. | .....<br>.... |
| iPS:39 3204 | 21-225_8C12 | VL3\|3j/JL2 | .......H...<br>..TA.M..... | ............<br>.....A.. | ........<br>D...... | S.......<br>....... | ..........P......<br>......IE......... | ......S...........<br>..........DH.. | .....<br>.... |
| Germline | **VL2\|2a2/JL2** | | QSALTQP-ASVSGSPGQSITISC | TGTS-SDVGGY----NYVS | WYQQHPGKAPKLMIY | E-------VSNRPS | GVSNRFSGSKSG--NTASLTISGLQAEDEADYYC | SSYTSS------------STLVV | FGGGTKLTVL |
| iPS:43 7092 | 21-225_210B12 | VL2\|2a2/JL2 | ............<br>............ | ........<br>........ | ........<br>...F... | ........<br>..R.... | .................<br>................. | .....-<br>....RTL. | ......<br>.... |
| iPS:43 7134 | 21-225_213A7 | VL2\|2a2/JL2 | ............<br>............ | ........<br>........ | ........<br>...F... | ........<br>..R.... | .................<br>................. | .....-<br>....RTL. | ......<br>.... |
| iPS:47 2733 | 21-225_2B10_LC2 | VL2\|2a2/JL2 | ............<br>............ | ........<br>.....F.. | ......D.<br>........ | ........<br>........ | .................<br>................. | .....-<br>...TGT.. | I.....<br>.... |
| iPS:39 2573 | 21-225_15G2 | VL2\|2a2/JL2 | ............<br>............ | ........<br>........ | ........<br>........ | ........<br>........ | .................<br>................. | T....-<br>...TST.. | ......<br>.... |
| iPS:39 3232 | 21-225_17F12 | VL2\|2a2/JL2 | ............<br>............ | ..A......D.<br>......S.. | ........<br>........ | ........<br>....... | .................<br>................. | .....-<br>....IT.. | ......<br>.... |
| iPS:39 8494 | 21-225_21H4 | VL2\|2a2/JL2 | ............<br>............ | ........<br>.....S.. | ......D.<br>........ | ........<br>........ | .................<br>................. | ....R-<br>...ST.. | ......<br>.... |
| Germline | **VL3\|3m/JL1** | | SYELMQP-PSVSVSPGQIARITC | SGD---ALPK-----QYAY | WYQQKPGQAPVLVIY | K-------DSERPS | GIPERFSGSSSG--TTVTLTISGVQAEDEADYYC | QSADSS------------GTYYV | FGTGTKVTVL |
| iPS:43 7102 | 21-225_211E5 | VL3\|3m/JL1 | ....T......<br>............ | ............<br>........ | ........<br>........ | ........<br>...A... | .........R.......<br>...V........... | .LVY.-<br>...SDT.. | .....M<br>L... |
| iPS:43 7164 | 21-225_217C6 | VL3\|3m/JL1 | ....T......<br>............ | ............<br>........ | ........<br>........ | ........<br>........ | .........R.......<br>....R.......... | .LIV.-<br>...SDT.. | ......<br>.... |
| iPS:43 7166 | 21-225_217G11 | VL3\|3m/JL1 | ....T......<br>............ | ............<br>........ | ........<br>........ | ........<br>........ | .........R.......<br>...V........... | .LVY.-<br>...SDT.. | ......<br>.... |
| iPS:43 7170 | 21-225_218E5 | VL3\|3m/JL1 | ....T......<br>............ | .R....V....<br>........ | ........<br>........ | ........<br>........ | .........R.......<br>....R.......... | .LVV.-<br>...SDT.. | ......<br>.... |

| iPS:43 7196 | 21-225_226B7 | VL3\|3m/J L1 | .F..⊓........<br>............ | .........R.<br>....H.V. | ....N...<br>........ | ........<br>....... | ................<br>................ | .....‐<br>................<br>...SGT.. | ......<br>.... |
|---|---|---|---|---|---|---|---|---|---|
| | **Germline** | | **L_FR1** | **L_CDR1** | **L_FR2** | **L_CDR2** | **L_FR3** | **L_CDR3** | **L_FR4** |
| | **VL4\|4c/JL2** | | LPVLTQP-<br>PSASALLGASIK<br>LTC | TLSS-EHSTY-<br>-----TIE | WYQQRPGR<br>SPQYIMK | VKS-<br>DGSHSKGD | GIPDRFMGSSSG<br>ADRYLTFSNLQSDDEAE<br>YHC | GESHTID<br>--------GQVGVV | FGGGTK<br>LTVL |
| iPS:39 2596 | 21-225_12D8 | VL4\|4c/J L2 | ............<br>............ | ..........<br>......... | ........<br>....... | ........<br>....... | ................<br>.............D.... | ...............<br>............... | ......<br>.... |
| iPS:39 3174 | 21-225_15D8 | VL4\|4c/J L2 | ............<br>............ | ..........<br>......... | ........<br>....... | ........<br>....... | .I.............E....<br>................ | ...............<br>............... | ......<br>.... |
| iPS:39 8544 | 21-225_7C8 | VL4\|4c/J L2 | ............<br>............ | ..........<br>......... | ........<br>....... | ........<br>....... | ...............G..<br>.............D.... | ....P..........<br>............... | ......<br>.... |
| | **Germline** | | **L_FR1** | **L_CDR1** | **L_FR2** | **L_CDR2** | **L_FR3** | **L_CDR3** | **L_FR4** |
| | **VL3\|3h/JL2** | | SYVLTQP-<br>PSVSVAPGKIAR<br>ITC | GGN---NIGS-<br>----KSVH | WYQQKPGQ<br>APVLVIY | Y------<br>-DSDRPS | GIPERFSGSNSG--<br>NTATLTISRVEAGDEAD<br>YYC | QVWDSS--------<br>--------SDHVV | FGGGTK<br>LTVL |
| iPS:39 3208 | 21-225_16F3 | VL3\|3h/J L2 | ............<br>....Q...... | ..........<br>......... | ........<br>.....V. | D.......<br>..T.... | ................<br>................ | ...............<br>............... | ......<br>.... |
| **HEAVY_VARIABLE** | | | | | | | | | |
| | **Germline** | | **H_FR1** | **H_CDR1** | **H_FR2** | **H_CDR2** | **H_FR3** | **H_CDR3** | **H_FR4** |
| | **VH1\|1-08/D6\|6-19\|RF1/JH4** | | QVQLVQS-<br>GAEVKKPGASVK<br>VSCKASG-YIFT | S------YDIN | WVRQATGQ<br>GLEWMG | WMNPN---<br>SGNTGYAQ<br>KFQG | RVTMTRNTSISTAYMEL<br>SSLRSEDTAVYYCAR | GYSSGW----------<br>-------YYFDY | WGQGTL<br>VTVSS |
| iPS:42 6126 | 21-225_6G6 | VH1\|1-08/D6\|6-19\|RF1/J H4 | ............<br>R...........<br>........ | N......... | ........<br>.P.... | ..H.....<br>.......K<br>.... | ...........A...V.<br>...............L | SSGWY-<br>.................<br>.-.... | ......<br>.... |
| iPS:41 2232 | 21-225_4A2 | VH1\|1-08/D6\|6-19\|RF1/J H4 | .........I..<br>............<br>........ | N......... | ........<br>...... | ..H.....<br>........<br>.... | ...L.............<br>...............S | SSGWY-<br>.................<br>.-.... | ......<br>.... |
| iPS:42 6112 | 21-225_12F12 | VH1\|1-08/D6\|6-19\|RF1/J H4 | ....L.......<br>............<br>........ | N......... | ........<br>...... | ..Y.....<br>........<br>.... | ......M...........<br>................M | SSGWY-<br>.................<br>.-...F | ......<br>.... |
| iPS:45 1141 | 21-225_164B11 | VH1\|1-08/D6\|6-19\|RF1/J H4 | ............<br>............<br>........ | N......... | ........<br>...... | ..T.....<br>........<br>.... | .........M.......<br>.......S.....SY | SSGWY-<br>.................<br>.-M... | ......<br>.... |
| iPS:46 8850 | 21-225_63F4 | VH1\|1-08/D6\|6-19\|RF1/J H4 | ............<br>............<br>........ | N.......V. | ........<br>...... | ..H.....<br>........<br>..R. | .........L..V....<br>...............Y | SSGWY-<br>.................<br>.-V... | ......<br>.... |

| iPS:46 8852 | 21-225_71F3 | VH1\|1-08/D6\|6-19\|RF1/J H4 | . . . . . . . . . . .<br>. . . . . . . . . . .<br>. . . . . . . | N . . . . . . . V . | . . . . . . . .<br>. . . . . .<br> | . . H . . . . .<br>. . . . . . .<br>. . . . | . . . . . . . . . . . . . . . .<br>. . . . . . . . . . . . . . . . . Y<br>. . . . | SSGWY-<br>. . . . . . . . . . . . . . . . . .<br>. -V . . S | . . . . . .<br>. . . . . |
| iPS:46 8854 | 21-225_72C4 | VH1\|1-08/D6\|6-19\|RF1/J H4 | . . . . . . . . . . .<br>. . . . . . . . . . .<br>. . . . . . . | N . . . . . . . . . | . . . . V . . .<br>. . . . . .<br> | . . H . . . . .<br>. . . . . . .<br>. . . . | . . . . . . . . . . . . . . . .<br>N . . . . . . . . . . . . SH<br> | SSGWY-<br>. . . . . . . . . . . . . . . . . .<br>. -L . . . | . . . . . .<br>. . . . . |
| iPS:46 8870 | 21-225_74A8 | VH1\|1-08/D6\|6-19\|RF1/J H4 | . . . . . . . . . . .<br>. . . . . . . . . . .<br>. . . . . . . | N . . . . . . . . | . . . . . S . .<br>. . . . . .<br> | . . H . . . . .<br>. . . . . . .<br>. . . . | . . . . . . . . . . . . . . . .<br>. . . . . . . S . . . . . Y<br> | SSGWY-<br>. . . . . . . . . . . . . . . . . .<br>. -K . . . | . S . . . .<br>. . . . . |
| iPS:42 3314 | 21-225_12F11 | VH1\|1-08/D6\|6-19\|RF1/J H4 | . . . . . . . . . . .<br>. . . . . . . . . . .<br>. . . . . . . | N . . . . . . . . | . . . . . . . .<br>. P . . . .<br> | . . H . . . . .<br>. . . . . . . K<br>. . . . | . . . . . . . . . . . A . . . V .<br>. . . . . . . . . . . . . . . L<br> | SSGWY-<br>. . . . . . . . . . . . . . . . . .<br>. - . . . | . . . . . .<br>. . . . . |
| iPS:43 3909 | 21-225_43D8 | VH1\|1-08/D6\|6-19\|RF1/J H4 | . . . . . . . . . . .<br>. . . . . . . . . . .<br>. . . . . . . | N . . . . . . . . | . . . . . . . .<br>. . . . . .<br> | . . H . . . . .<br>. . . . . . .<br>. . . . | . . . . . . . . . . . . . . . .<br>. . . T . . . . . . . . H<br> | SSGWT-<br>. . . . . . . . . . . . . . . . . .<br>. -L . . . | . . . . . .<br>. . . . A |
| iPS:43 4177 | 21-225_56A1 | VH1\|1-08/D6\|6-19\|RF1/J H4 | . . . . . . . . . . .<br>. . . . . . . . . . .<br>. . . . . . . | N . . . . . . . . | . . . . . . . .<br>. . . . L .<br> | . . H . . . . .<br>. . . . . . .<br>. . . . | . . . . . . . . . . . . . . . .<br>. . . . . . . . . . . . Y<br> | SSGWY-<br>. . . . . . . . . . . . . . . . . .<br>. -V . . . | . . . . . .<br>. . . . . |
| iPS:43 4211 | 21-225_60F3 | VH1\|1-08/D6\|6-19\|RF1/J H4 | . . L . . . . . . . .<br>. . . . . . . . . . .<br>. . . . . . . | N . . . . . . . . | . . . . . . . .<br>. . . . . .<br> | . . H . . . . .<br>. . . . . . .<br>. . . . | . . . . . . . . . . . . . . . .<br>. . . . . . . . . . . . Y<br> | SSGWY-<br>. . . . . . . . . . . . . . . . . .<br>. -F . . . | . . . . . .<br>. . . . . |
| iPS:43 4235 | 21-225_61E3 | VH1\|1-08/D6\|6-19\|RF1/J H4 | . . . . . . . . . . .<br>. . . . . . . . . . .<br>. . . . . . . | N . . . . . . . . | . . . . . . . .<br>. . . . . .<br> | . . T . . . . .<br>. . . . . . .<br>. . . . | . . . . . . . . . . . . . . . .<br>. . . K . . . . . . . . . Y<br> | SSGWY-<br>. . . . . . . . . . . . . . . . . .<br>. -R . . . | . . . . . .<br>. . . . . |
| iPS:43 4237 | 21-225_61B5 | VH1\|1-08/D6\|6-19\|RF1/J H4 | . . . . . . . . . . .<br>. . . . . . . . . . .<br>. . . . . . . | N . . . . . . . . | . . . . . . . .<br>. . . . . .<br> | . . H . . . . .<br>. . S . . . .<br>. . . . | . . . . . . . . . . . . . . . .<br>. . . . . . . . . . . . Y<br> | SSGWY-<br>. . . . . . . . . . . . . . . . . .<br>. - . . . | . . . . . .<br>. . . . . |
| iPS:43 4295 | 21-225_58B9 | VH1\|1-08/D6\|6-19\|RF1/J H4 | . . . . . . . . . . .<br>. . . . . . . . . . .<br>. . . . . . . | N . . . . . . . . | . . . . . . . .<br>. . . . . .<br> | . . . . . . . .<br>. . S . . . .<br>. . . . | . . . . . . . . . . . . . . . .<br>. . . . . . . . . . . . Y<br> | SSGWY-<br>. . . . . . . . . . . . . . . . . .<br>. - . . . | . . . . . .<br>. . . . . |
| iPS:43 4305 | 21-225_59E1 | VH1\|1-08/D6\|6-19\|RF1/J H4 | . . . . . . . . . . .<br>. . . . . . . . . . .<br>. . . . . . . | N . . . . . . . . | . . . . . . . .<br>. . . . . .<br> | . . T . . . . .<br>. . . . . . .<br>. . . . | . . . . . . T . . . . . . . . .<br>. . . . . . . . . . . . . F<br> | SSGWY-<br>. . . . . . . . . . . . . . . . . .<br>. -F . . . | . . . . . .<br>. . . . . |
| iPS:43 4321 | 21-225_59F10 | VH1\|1-08/D6\|6-19\|RF1/J H4 | . . . . . . . . . . .<br>. . . . . . . . . . .<br>. . . . . . . | N . . . . . . . . | . . . . . . . .<br>. . . . . .<br> | . . . . . . . .<br>. . . . . . .<br>. . . . | . . . . . . . . . . . . . . . .<br>. . . . . . . . . . N . . V<br> | SSGWY-<br>. . . . . . . . . . . . . . . . . .<br>. - . . . | . . . . . .<br>. . . . . |

| | | | | | | | | | |
|---|---|---|---|---|---|---|---|---|---|
| iPS:43 4431 | 21-225_70E7 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . H . . . . .<br>. . . . . . . . .<br>. . . . | . . . . . . . . . . . . . . . . .<br>. . . . . . . . . . . . . . . . Y | SSGWY-<br>. . . . . . . . . . . . . . . . . . .<br>. - V . . . | . . . . . .<br>. . . . . . |
| iPS:43 4443 | 21-225_71G3 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . H . . . . .<br>. . S . . . . .<br>. . . . | . . . . . . . D . . V . . . . . . .<br>. . . . . . . . . . . . . . . . Y | SSGWY-<br>. . . . . . . . . . . . . . . . . . .<br>. - . . . . | . . . . . .<br>. . . . . . |
| iPS:43 4475 | 21-225_74F9 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . . . . C . .<br>. . . . | . . . . . . . . . . . . . . . . .<br>. . . . . . . . . . . . . . . S | SSGWN-<br>. . . . . . . . . . . . . . . . . . .<br>. - F . . . | . . . . . .<br>. . . . . . |
| iPS:43 4477 | 21-225_74A6 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . H . . . . .<br>. . . . . . . . .<br>. . R . | . . . . . W . . . . . . . . . . .<br>. . . . . . . . . . . . . . . S | SSGWY-<br>. . . . . . . . . . . . . . . . . . .<br>. - . . . . | . . . . . .<br>. . . . . . |
| iPS:43 4487 | 21-225_76G2 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . . . . . . . .<br>. . . . | . . . . . . . . . . . . . . . . .<br>. . . . . . . . . . . . . . . G | SSGWY-<br>. . . . . . . . . . . . . . . . . . .<br>. - M . . . | . . . . . .<br>. . . . . . |
| iPS:43 4511 | 21-225_74B11 | VH1\|1-08/D6\|6-19\|RF1/J H4 | . . . . . . . . . . .<br>. . . . . . R . . . .<br>. . . . . . . . | N . . . . . . . . . | . . . . . . . .<br>. . . . . .<br>. . . . | . . H . . . . .<br>. . . . . . . . .<br>. . . . | . . . . . . . . . . . . . . . . .<br>. . . . . . . . . . . . . . . . Y | SSGWY-<br>. . . . . . . . . . . . . . . . . . .<br>. - . . . . | . . . . . .<br>. . . . . . |
| iPS:43 4549 | 21-225_76E11 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . . . . . . . .<br>. . . . | . L . . . . . . . . . . . . . . .<br>. . . . . . . . . . F . . . Y | SSGWY-<br>. . . . . . . . . . . . . . . . . . .<br>. - . . . . | . . . . . .<br>. . . . . . |
| iPS:43 4551 | 21-225_75C4 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . H . . . . .<br>. . . . . . . . .<br>. . . . | . . . . . . . . . . . . . . . . .<br>. . . . . . . . . . . . . . . S | SSGWY-<br>. . . . . . . . . . . . . . . . . . .<br>. - . . . . | . . . . . .<br>. . . . . . |
| iPS:43 4635 | 21-225_78E6 | VH1\|1-08/D6\|6-19\|RF1/J H4 | . . . . . . . . . . .<br>. . . . . . . . . . .<br>. . . . . . . . | N . . . . . . . . . | . . . . . S . .<br>. . . . . .<br>. . . . | . . H . . . . .<br>. . . . . . . . .<br>. . . . | . . . . . . . . . . . . . . . . .<br>. . . . . . . S . . . . . . Y | SSGWY-<br>. . . . . . . . . . . . . . . . . . .<br>. - K . . . | . S . . . .<br>. . . . . |
| iPS:43 4649 | 21-225_78E11 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . . . . C . .<br>. . . . | . . . . . . . . . . . . . . . . .<br>. . . . . . . . . . . . . . . S | SSGWN-<br>. . . . . . . . . . . . . . . . . . .<br>. - F . . . | . . . . . .<br>. . . . . . |
| iPS:43 4665 | 21-225_74G4 | VH1\|1-08/D6\|6-19\|RF1/J H4 | . . . . . . . . . . .<br>. . . . . . . . . . .<br>. . . . . . . . | N . . . . . . . V . | . . . . . . . .<br>. . . . . .<br>. . . . | . . H . . . . .<br>. . . . . . . . .<br>. . . . | . . . . . . . . . R . . . . . . .<br>. . . . . . . . . . . . . . . S | SSGWY-<br>. . . . . . . . . . . . . . . . . . .<br>. - H . . . | . . . . . .<br>. . . . . . |
| iPS:43 4679 | 21-225_79G7 | VH1\|1-08/D6\|6-19\|RF1/J H4 | . . . . . . . . . . .<br>. . . . . . . . . . .<br>. . . . . . . . | N . . . . . . . . . | . . . . . S . .<br>. . . . . .<br>. . . . | . . H . . . . .<br>. . . . . . . . .<br>. . . . | . . . . . . . . . . . . . . . . .<br>. . . . . . . S . . . . . . Y | SSGWY-<br>. . . . . . . . . . . . . . . . . . .<br>. - K . . . | . S . . . .<br>. . . . . |

| | | | | | | | | | |
|---|---|---|---|---|---|---|---|---|---|
| iPS:43 4685 | 21-225_79E9 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . H . . . . .<br>. . . . . . . .<br>. . . . | . . . . . . . . . . . . . . . . .<br>. . . . . . . . . . . . . . . . . S | SSGWY-<br>. . . . . . . . . . . . . . . . . .<br>. - . . . . | . . . . . .<br>. . . . . |
| iPS:43 4697 | 21-225_79F12 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . . S | SSGWY-<br>. . . . . . . . . . . . . . . . . .<br>. - F . . . | . . . . . .<br>. . L . . |
| iPS:43 4729 | 21-225_80B12 | VH1\|1-08/D6\|6-19\|RF1/J H4 | . . . . . . . . . . .<br>. . . . . . . . . . .<br>T . . . . . . . | N . . . . . . . . . | . . . . . . . .<br>. . . . . .<br>. . . . | . . . . . . . .<br>. . . . . . . .<br>. . . V | . . . . . . . . . . . . . . . . .<br>. . . . . . . . . . . . . . . . . Y | SSGWY-<br>. . . . . . . . . . . . . . . . . .<br>. - I . . . | . . . . . .<br>. . . . . |
| iPS:43 4851 | 21-225_75A6 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . . Y | SSGWY-<br>. . . . . . . . . . . . . . . . . .<br>. - I . . . | . . . . . .<br>. . . . . |
| iPS:43 4909 | 21-225_85C11 | VH1\|1-08/D6\|6-19\|RF1/J H4 | . . . . . . . . . . .<br>. . . . . . . . . . .<br>. . . . . . . . | N . . . . . . . . . | . . . . . S . .<br>. . . . . .<br>. . . . | . . H . . . . .<br>. . . . . . . .<br>. . . . | . . . . . . . . . . . . . . . . .<br>. . . . . . . S . . . . . . Y | SSGWY-<br>. . . . . . . . . . . . . . . . . .<br>. - K . . . | . S . . . .<br>. . . . |
| iPS:43 4959 | 21-225_87E10 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . . Y | SSGWY-<br>. . . . . . . . . . . . . . . . . .<br>. - F . . . | . . . . . .<br>. . . . . |
| iPS:43 4965 | 21-225_88A1 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . . Y | SSGWY-<br>. . . . . . . . . . . . . . . . . .<br>. - . . . . | . . . . . .<br>. . . . . |
| iPS:43 4973 | 21-225_88B4 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . D . . . . .<br>. . . . | S . . . . . . . . . T . . . . . .<br>. . . . . . . . . . . . SY | SSGWY-<br>. . . . . . . . . . . . . . . . . .<br>. - . . . . | . . . . . .<br>. . . . . |
| iPS:43 4997 | 21-225_88C10 | VH1\|1-08/D6\|6-19\|RF1/J H4 | . . . . . . . . . . .<br>. . . . . . . R . . . .<br>. . . . . . . S | N . . . . . . . . . | . . . . . . . .<br>. . . . . .<br>. . . . | . . T . . . . .<br>. . . . . . . .<br>. . . . | . . . . . . . . . . . . . . . . .<br>. . . . . . . . . . . . . . . . . S | SSGWY-<br>. . . . . . . . . . . . . . . . . .<br>. - . . . S | . . . . . .<br>. . . . L |
| iPS:43 5053 | 21-225_75F9 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . H . . . . .<br>. . . . . . . .<br>. . . . | . . . . . . . . . . . . . . . . .<br>. . . . . . . . . . . . . . . . . Y | SSGWY-<br>. . . . . . . . . . . . . . . . . .<br>. - I . . . | . . . . . .<br>. . . . . |
| iPS:43 5113 | 21-225_92E6 | VH1\|1-08/D6\|6-19\|RF1/J H4 | . . . . . . . . . . .<br>. . . . . . . R . . . .<br>. . . . . . . . | N . . . . . . . . . | . . . . . . . .<br>. . . . . .<br>. . . . | . . H . . . . .<br>. . . . . . . .<br>. . . . | . . . . . . . . . M . . . . . . .<br>. . . . . . . . . . . . . . . . . H | SSGWY-<br>. . . . . . . . . . . . . . . . . .<br>. - F . . . | . . . . . .<br>. . . . . |
| iPS:43 5209 | 21-225_75A10 | VH1\|1-08/D6\|6-19\|RF1/J H4 | . . . . . . . . . . .<br>. . . . . . . . . . .<br>. . . . . . . . | N . . . . . . . . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . . Y | SSGWY-<br>. . . . . . . . . . . . . . . . . .<br>. - . . . . | . . . . . .<br>. . . . . |

| iPS | Name | VH | | N | | | | | SSGWY | |
|---|---|---|---|---|---|---|---|---|---|---|
| iPS:43 5257 | 21-225_96H5 | VH1|1-08|D6|6-19|RF1/JH4 | ...........<br>...........<br>....... | N.........<br><br> | .....S..<br>......<br> | ..H.....<br>........<br>.... | ..................<br>.......S......Y<br> | SSGWY-<br>..................<br>.-K... | .S....<br>......<br> |
| iPS:43 5267 | 21-225_96D10 | VH1|1-08|D6|6-19|RF1/JH4 | ...........<br>......R....<br>....... | N.........<br><br> | ........<br>......<br> | ..H.....<br>........<br>.... | .........M......<br>.................H<br> | SSGWY-<br>..................<br>.-F... | ......<br>......<br> |
| iPS:43 5299 | 21-225_146D4 | VH1|1-08|D6|6-19|RF1/JH4 | ...........<br>...........<br>....... | N.........<br><br> | ........<br>......<br> | .VH.....<br>........<br>.... | ..................<br>.................G<br> | SSGWY-<br>..................<br>.-.... | ......<br>......<br> |
| iPS:43 5305 | 21-225_146C9 | VH1|1-08|D6|6-19|RF1/JH4 | ...........<br>...........<br>....... | N.........<br><br> | ........<br>......<br> | ..H.....<br>........<br>.... | .....W.........A.<br>.................Y<br> | SSGWY-<br>..................<br>.-S... | ......<br>......<br> |
| iPS:43 5309 | 21-225_146F9 | VH1|1-08|D6|6-19|RF1/JH4 | ...........<br>...........<br>....... | N.........<br><br> | ........<br>......<br> | ..H.....<br>........<br>.... | ..................<br>.................S<br> | SSGWY-<br>..................<br>.-F... | ......<br>......<br> |
| iPS:43 5321 | 21-225_147E4 | VH1|1-08|D6|6-19|RF1/JH4 | ...........<br>...........<br>....... | N.........<br><br> | ........<br>......<br> | ........<br>........<br>.... | ..................<br>.............S<br> | SSGWY-<br>..................<br>.-F... | ......<br>......<br> |
| iPS:43 5323 | 21-225_147D5 | VH1|1-08|D6|6-19|RF1/JH4 | ...........<br>...........<br>....... | N.........<br><br> | ........<br>......<br> | .VH.....<br>........<br>.... | ..................<br>......G........G<br> | SSGWY-<br>..................<br>.-.... | ......<br>......<br> |
| iPS:43 5345 | 21-225_148G3 | VH1|1-08|D6|6-19|RF1/JH4 | ...........<br>...........<br>....... | N.........<br><br> | .....S..<br>......<br> | ..H.....<br>........<br>N... | ..................<br>.............S<br> | SSGWY-<br>..................<br>.-F... | ......<br>......<br> |
| iPS:43 5353 | 21-225_148F8 | VH1|1-08|D6|6-19|RF1/JH4 | ...........<br>...........<br>....... | N.........<br><br> | ........<br>......<br> | ........<br>........<br>.... | ..................<br>.............S<br> | SSGWY-<br>..................<br>.-.... | ......<br>......<br> |
| iPS:43 5369 | 21-225_149A2 | VH1|1-08|D6|6-19|RF1/JH4 | ...........<br>...........<br>....... | N.........<br><br> | ........<br>......<br> | .VH.....<br>........<br>.... | ..................<br>.................G<br> | SSGWY-<br>..................<br>.-.... | ......<br>......<br> |
| iPS:43 5373 | 21-225_149E3 | VH1|1-08|D6|6-19|RF1/JH4 | ...........<br>...........<br>....... | N.........<br><br> | ........<br>......<br> | ..H.....<br>........<br>.... | ..................<br>...T..........Y<br> | SSGWY-<br>..................<br>.-W... | ......<br>......<br> |
| iPS:43 5375 | 21-225_149H4 | VH1|1-08|D6|6-19|RF1/JH4 | ...........<br>...........<br>....... | N.........<br><br> | ........<br>......<br> | ..H.....<br>....D...<br>.... | .........T.V....<br>.............TF<br> | SSGWY-<br>..................<br>.-.... | ......<br>......<br> |

| | | | | | | | | | | |
|---|---|---|---|---|---|---|---|---|---|---|
| iPS:43 5399 | 21-225_150D2 | VH1\|1-08/D6\|6-19\|RF1/J H4 | . . . . . . . . . . .<br>. . . . . . . . . .<br>. . . . . . . | N . . . . . . . . . | . . . . . . . .<br>. . . . . . | . . H . . . . .<br>. . . . . . . .<br>. . . . | . . . . . . . . . . . . . . . .<br>. . . . . . . . . . . . . . . . . Y | SSGWY-<br>. . . . . . . . . . . . . . . . . . .<br>. - . . . . | . . . . . . |
| iPS:43 5405 | 21-225_150B7 | VH1\|1-08/D6\|6-19\|RF1/J H4 | . . . . . . . . . . .<br>. . . . . . T . . . .<br>. . . . FP . . | N . . . . . . . . . | . . . . . . . .<br>. . . . . . | . . H . . . . .<br>. . . . . . . .<br>. . . . | . . . . . . . . . . . . . . . L . . .<br>. . . . . . . . . . . . . . . . . S | SSGWY-<br>. . . . . . . . . . . . . . . . . . .<br>. - F . . . | . . . . . . |
| iPS:43 5433 | 21-225_152E3 | VH1\|1-08/D6\|6-19\|RF1/J H4 | . . . . . . . . . . .<br>. . . . . . . . . .<br>. . . . . . . | N . . . . . . . . . | . . . . . . . .<br>. . . . . . | . . H . . . . .<br>. . . . . . . .<br>. . . . | . . . . . . . . . . . . . . . . .<br>. . . . . . . . . . . . . . . . . S | SSGWY-<br>. . . . . . . . . . . . . . . . . . .<br>. - F . . . | . . . . . . |
| iPS:43 5435 | 21-225_152H3 | VH1\|1-08/D6\|6-19\|RF1/J H4 | . . . . . . . . . . .<br>. . . . . . . . . .<br>. . . . . . . P | N . . . . . . . . . | . . . . . . . .<br>. . . . . . | . . H . . . . .<br>. . . . . . . .<br>. . . . | . . . . . . . . . . . . . . . . .<br>. . . . . . . . . . . . . . . . . Y | SSGWY-<br>. . . . . . . . . . . . . . . . . . .<br>. - W . . . | . . . . . . |
| iPS:43 5459 | 21-225_152E12 | VH1\|1-08/D6\|6-19\|RF1/J H4 | . . . . . . . . . . .<br>. . . . . . . . . .<br>. . . . . . . | N . . . . . . . . . | . . . . . . . .<br>. . . . . . | . . . . . . . .<br>. . . . . . . .<br>. . . . | . . . . . . . . . . . . . . . . .<br>. . . . . . . . . . . . . . . . . Y | SSGWY-<br>. . . . . . . . . . . . . . . . . . .<br>. - . . . . | . . . . . . |
| iPS:43 5471 | 21-225_153F11 | VH1\|1-08/D6\|6-19\|RF1/J H4 | . . . . . . . . . . .<br>. E . . . . . . . . .<br>. . . . . . . | N . . . . . . . . . | . . . . . . . .<br>. . . . . . | . . H . . . . .<br>. . . . . . . .<br>. . . . | . . . . . . . . . . . . . N . . . . .<br>. . . . . . . . . . . . . . . . . Y | SSGWY-<br>. . . . . . . . . . . . . . . . . . .<br>. - F . . N | . . . . . . |
| iPS:43 5475 | 21-225_154H6 | VH1\|1-08/D6\|6-19\|RF1/J H4 | . . . . . . . . . . .<br>. . . . . . . . . .<br>. . . . . . . | N . . . . . . . . . | . . . . . . . .<br>. . . . . . | . . . . . . . .<br>. . . . . . . .<br>. . . . | . . . . . . . . . . . . . . . . .<br>. . . . . . . . . . . . . . . . . Y | SSGWY-<br>. . . . . . . . . . . . . . . . . . .<br>. - I . . . | . . . . . . |
| iPS:43 5481 | 21-225_154A11 | VH1\|1-08/D6\|6-19\|RF1/J H4 | . . . . . . . . . . .<br>. . . . . . . . . .<br>. . . . . . . | N . . . . . . . . . | . . . . . . . .<br>. . . . . . | . . . . . . . .<br>. . . . . . . .<br>. . . . | . . . . . . . . . . . . . . . . .<br>. . . . . . . . . . . . . . . . . S | SSGWY-<br>. . . . . . . . . . . . . . . . . . .<br>. - . . . . | . . . . . . |
| iPS:43 5491 | 21-225_155E5 | VH1\|1-08/D6\|6-19\|RF1/J H4 | . . . . . . . . . . .<br>. . . . . . . . . .<br>. . . . . . . | N . . . . . . . . . | . . . . . . . .<br>. . . . . . | . . H . . . . .<br>. . S . . . . .<br>R . . . | . . . . . . . . . . . . . . . . .<br>. . . . . . . . . . . . . . F | SSGWY-<br>. . . . . . . . . . . . . . . . . . .<br>. - . . . . | . . . . . . |
| iPS:43 5495 | 21-225_155B6 | VH1\|1-08/D6\|6-19\|RF1/J H4 | . . . . . . . . . . .<br>. . . . . . . . . .<br>. . . . . . . | N . . . . . . . . . | . . . . . . . .<br>. . . . . . | . . . . . . . .<br>. . . . . . . .<br>. . . . | . . . . . . . . . . . . . . . . .<br>. . . . . . . . . . . . . . . . . Y | SSGWY-<br>. . . . . . . . . . . . . . . . . . .<br>. - . . . . | . . . . . . |
| iPS:43 5501 | 21-225_156H1 | VH1\|1-08/D6\|6-19\|RF1/J H4 | . . . . . . . . . . .<br>. . . . . . . . . .<br>. . . . . . . | N . . . . . . . . . | . . . . . . . .<br>. . . . . . | . VH . . . . .<br>. . . . . . . .<br>. . . . | . . . . . . . . . . . . . . . . .<br>. . . . . . . . . . . . . . . . . G | SSGWY-<br>. . . . . . . . . . . . . . . . . . .<br>. - . . . . | . . . . . . |
| iPS:43 5557 | 21-225_158B12 | VH1\|1-08/D6\|6-19\|RF1/J H4 | . . . . . . . . . . .<br>R . . . . . . . . . .<br>. . . . . . . | N . . . . . . . . . | . . . . . . . .<br>. . . . . . | . . H . . . . .<br>. . . . . . . .<br>. . . . | . F . . . . . . . . . . . . . .<br>. . . . . . . . . . . . . . . . . Y | SSGWY-<br>. . . . . . . . . . . . . . . . . . .<br>. - R . . . | . . . . . . |

| | | | | | | | | | |
|---|---|---|---|---|---|---|---|---|---|
| iPS:43 5589 | 21-225_160A4 | VH1\|1-08/D6\|6-19\|RF1/JH4 | .........T...<br>..........<br>....... | N......... | ........<br>......<br>.... | ..H.....<br>......P.<br>.... | ................<br>...............Y | SSGWY-<br>...............<br>.-I... | ......<br>...... |
| iPS:43 5623 | 21-225_162D5 | VH1\|1-08/D6\|6-19\|RF1/JH4 | .........S...<br>..........<br>....... | N......... | ........<br>......<br>.... | ..H.....<br>.......<br>.... | .........D.....<br>...S.......F..F | SSGWY-<br>...............<br>.-F... | ......<br>...... |
| iPS:43 5627 | 21-225_162F6 | VH1\|1-08/D6\|6-19\|RF1/JH4 | ..........<br>R.........<br>....... | N....... | ........<br>......<br>.... | ..H.....<br>.......<br>.... | .F.............<br>...............Y | SSGWY-<br>...............<br>.-R... | ......<br>...... |
| iPS:43 5649 | 21-225_165H2 | VH1\|1-08/D6\|6-19\|RF1/JH4 | ..........<br>..........<br>....... | H......... | ........<br>....V.<br>.... | ..H.....<br>.HK.....<br>.... | .........N.....D.<br>...............Y | SSGWY-<br>...............<br>.-M... | ......<br>...... |
| iPS:43 5727 | 21-225_172E11 | VH1\|1-08/D6\|6-19\|RF1/JH4 | ..........<br>.......M....<br>....... | N......... | ........<br>......<br>.... | ..H.....<br>.......<br>.... | ................<br>...............Y | SSGWY-<br>...............<br>.-R... | ......<br>...... |
| iPS:43 5751 | 21-225_175D10 | VH1\|1-08/D6\|6-19\|RF1/JH4 | ..........<br>..........<br>....... | N.......L. | ........<br>......<br>.... | ..H.....<br>.......<br>.... | ............V....<br>...............Y | SSGWY-<br>...............<br>.-.... | ......<br>...... |
| iPS:43 5773 | 21-225_177B12 | VH1\|1-08/D6\|6-19\|RF1/JH4 | ..........<br>..........<br>....... | N....... | ........<br>......<br>.... | ..H.....<br>.......<br>.... | ................<br>...............Y | SSGWY-<br>...............<br>.-...F | ......<br>...... |
| iPS:43 5801 | 21-225_181E5 | VH1\|1-08/D6\|6-19\|RF1/JH4 | ..........<br>..........<br>....... | N....... | ........<br>......<br>.... | ........<br>.......<br>.... | ................<br>...............S | SSGWY-<br>...............<br>.-I... | ......<br>...... |
| iPS:43 5841 | 21-225_191D8 | VH1\|1-08/D6\|6-19\|RF1/JH4 | ..........<br>..........<br>....... | N......... | ........<br>......<br>.... | ..H.....<br>.......<br>.... | ................<br>...............H | SSGWY-<br>...............<br>.-.... | ......<br>...... |
| iPS:43 5855 | 21-225_191G3 | VH1\|1-08/D6\|6-19\|RF1/JH4 | ..........<br>..........<br>....... | N......... | ........<br>......<br>.... | R.......<br>.......<br>.... | ................<br>...............H | SSGWY-<br>...............<br>.-I... | ......<br>...... |
| iPS:43 5915 | 21-225_190H4 | VH1\|1-08/D6\|6-19\|RF1/JH4 | ..........<br>..........<br>....N... | N......... | ........<br>......<br>.... | ........<br>.......<br>.... | ................<br>...............H | SSGWY-<br>...............<br>.-I... | ......<br>...... |
| iPS:43 5925 | 21-225_190D7 | VH1\|1-08/D6\|6-19\|RF1/JH4 | ..........<br>..........<br>....... | N......... | ........<br>......<br>.... | ........<br>.......<br>.... | ................<br>...............S | SSGWY-<br>...............<br>.-F... | ......<br>...... |

| iPS:43 6021 | 21-225_193G4 | VH1\|1-08/D6\|6-19\|RF1/JH4 | ............ ............ ........ | N......... | ........ ...... | ........ .... | ............R...... N..............S | SSGWY- .................... .-F... | ...... ...... |
|---|---|---|---|---|---|---|---|---|---|
| iPS:43 6150 | 21-225_197H4 | VH1\|1-08/D6\|6-19\|RF1/JH4 | ............ ............ ........ | N......... | ........ ...... | ..H..... ........ .... | .................... ..............H | SSGWY- .................... .-.... | ...... ...... |
| iPS:43 6154 | 21-225_197C6 | VH1\|1-08/D6\|6-19\|RF1/JH4 | ............ ............ ........ | N......... | ........ ...... | ..H..... ........ .... | .................... ..............H | SSGWY- .................... .-.... | ...... ...... |
| iPS:43 6272 | 21-225_201F5 | VH1\|1-08/D6\|6-19\|RF1/JH4 | ..........A. ............ ........ | N......... | ........ ...... | ..H..... ........ .... | .................... ..............Y | SSGWY- .................... .-.... | ...... ...... |
| iPS:43 6550 | 21-225_224D8 | VH1\|1-08/D6\|6-19\|RF1/JH4 | ............ ............ ........ | N......... | ........ ...... | .LY..... ........ .... | .................... ..............Y | SSGWY- .................... .-.... | ...... .I... |
| iPS:43 6554 | 21-225_224C10 | VH1\|1-08/D6\|6-19\|RF1/JH4 | ............ ............ ....S... | N......... | ........ ...... | ..H..... ........ .... | .................... ..............Y | SSGWY- .................... .-K... | ...... ...... |
| iPS:43 6560 | 21-225_224F11 | VH1\|1-08/D6\|6-19\|RF1/JH4 | ............ ............ ........ | N......... | ........ ...... | ..H..... ........ .... | .................... ..............Y | SSGWY- .................... .-K... | ...... ...... |
| iPS:43 6574 | 21-225_225F5 | VH1\|1-08/D6\|6-19\|RF1/JH4 | ............ ...R........ ....H... | N......... | ........ ...... | ..H..... .....F.. .... | .................... ..............Y | SSGWY- .................... .-R... | ...... ...... |
| iPS:43 6584 | 21-225_225B9 | VH1\|1-08/D6\|6-19\|RF1/JH4 | .........I.. R........... ........ | N......... | ........ R..... | ..H..... ........ ..R. | ..........N...... N..............Y | SSGWT- .................... .-L... | ...... ...... |
| iPS:43 6586 | 21-225_225F11 | VH1\|1-08/D6\|6-19\|RF1/JH4 | ............ ............ ........ | N......... | ........ ...... | ..H..... ........ .... | ..........N...... ..............Y | SSGWY- .................... .-R... | ...... ...... |
| iPS:43 6588 | 21-225_225F12 | VH1\|1-08/D6\|6-19\|RF1/JH4 | ............ ............ ........ | H......... | ........ ...... | ..H..... ........ .... | ....A............ ..............Y | SSGWY- .................... .-K... | ...... ...... |
| iPS:43 6590 | 21-225_225H12 | VH1\|1-08/D6\|6-19\|RF1/JH4 | ............ ............ ........ | N......... | ........ ...... | ..H..... ........ .... | .................... ..............Y | SSGWY- .................... .-K... | ...... ...... |

| iPS:43 6598 | 21-225_226D6 | VH1\|1-08/D6\|6-19\|RF1/JH4 | ..............<br>..............<br>........ | N......... | ........<br>...... | ..H.....<br>........<br>.... | .................<br>................Y | SSGWY-<br>...................<br>.-K... | ......<br>..... |
| iPS:43 6600 | 21-225_226F6 | VH1\|1-08/D6\|6-19\|RF1/JH4 | ..............<br>..............<br>........ | N......... | ........<br>...... | ..H.....<br>........<br>.... | ...............N......<br>................Y | SSGWY-<br>...................<br>.-R... | ......<br>..... |
| iPS:43 6616 | 21-225_226D11 | VH1\|1-08/D6\|6-19\|RF1/JH4 | ..............<br>...........R<br>S....... | N......... | ........<br>...... | ..H.....<br>........<br>.... | .................<br>................Y | SSGWY-<br>...................<br>.-.... | ......<br>..... |
| iPS:43 6622 | 21-225_226A12 | VH1\|1-08/D6\|6-19\|RF1/JH4 | ..............<br>..............<br>........ | N......... | ........<br>...... | ..H.....<br>........<br>.... | .................<br>...............Y | SSGWY-<br>...................<br>.-.... | ......<br>..... |
| iPS:43 6636 | 21-225_227E6 | VH1\|1-08/D6\|6-19\|RF1/JH4 | ..............<br>..............<br>........ | N......... | ........<br>...... | ..H.....<br>........<br>.... | .................<br>................Y | SSGWY-<br>...................<br>.-K... | ......<br>..... |
| iPS:43 6638 | 21-225_227C7 | VH1\|1-08/D6\|6-19\|RF1/JH4 | ..............<br>...R.........<br>....H... | N......... | ........<br>...... | ..H.....<br>........<br>.... | .................<br>................Y | SSGWY-<br>...................<br>.-R... | ......<br>..... |
| iPS:43 6646 | 21-225_227D11 | VH1\|1-08/D6\|6-19\|RF1/JH4 | ..............<br>..............<br>........ | N......... | ........<br>...... | ..H.....<br>........<br>.... | .................<br>................Y | SSGWY-<br>...................<br>.-.... | ......<br>..... |
| iPS:44 6086 | 21-225_94D6 | VH1\|1-08/D6\|6-19\|RF1/JH4 | ..............<br>..............<br>T....... | N......... | ........<br>...... | ........<br>........<br>...V | ................V<br>................Y | SSGWY-<br>...................<br>.-I... | ......<br>..... |
| iPS:45 1116 | 21-225_164A4 | VH1\|1-08/D6\|6-19\|RF1/JH4 | ..............<br>....F..P | N......... | ........<br>...... | ..H.....<br>........<br>.... | .................<br>.................S | SSGWY-<br>...................<br>.-F... | ......<br>..... |
| iPS:45 1124 | 21-225_74F6 | VH1\|1-08/D6\|6-19\|RF1/JH4 | ..............<br>.......R....<br>........ | N......... | ........<br>...... | ..H.....<br>........<br>.... | ..........M......<br>.................H | SSGWY-<br>...................<br>.-F... | ......<br>..... |
| iPS:45 1127 | 21-225_164A7 | VH1\|1-08/D6\|6-19\|RF1/JH4 | ..............<br>..............<br>........ | N......V. | ........<br>...... | ..H.....<br>........<br>.... | ...........T......<br>.......S......S | SSGWY-<br>...................<br>.-L... | ......<br>..... |
| iPS:45 1131 | 21-225_160A7 | VH1\|1-08/D6\|6-19\|RF1/JH4 | ..............<br>..............<br>........ | N......... | ........<br>...... | ..H.H...<br>........<br>.... | .............N......<br>..............H | SSGWY-<br>...................<br>.-.... | ......<br>..... |

| iPS ID | Clone | Germline | | | | | | | |
|---|---|---|---|---|---|---|---|---|---|
| iPS:39 2786 | 21-225_24E1 | VH1\|1-08/D6\|6-19\|RF1/JH4 | ...........<br>...........<br>........ | N......... | ........<br>...... | ..H.....<br>R... | ................<br>...............T | SSGWE-<br>...................<br>.-V... | ...... |
| iPS:39 2886 | 21-225_23A12 | VH1\|1-08/D6\|6-19\|RF1/JH4 | ...........<br>...........<br>.....P.. | N......... | ........<br>...... | ..H.....<br>........<br>.... | ..........N.....<br>...............G | SSGWY-<br>...................<br>.-.... | ...... |
| iPS:39 2928 | 21-225_25A4 | VH1\|1-08/D6\|6-19\|RF1/JH4 | ..L........<br>.R.........<br>........ | N......... | ........<br>...... | ..Y.....<br>........<br>.... | ................<br>................S | SSGWY-<br>...................<br>.-.... | ...... |
| iPS:39 2936 | 21-225_28B6 | VH1\|1-08/D6\|6-19\|RF1/JH4 | ..L........<br>.R.........<br>........ | N......... | ........<br>...... | ..H.D...<br>........<br>.... | ................<br>................S | SSGWY-<br>...................<br>.-.... | ...... |
| iPS:39 2960 | 21-225_29E6 | VH1\|1-08/D6\|6-19\|RF1/JH4 | ..L........<br>.R.........<br>........ | N......... | ........<br>...... | ..H.....<br>........<br>.... | ................<br>................S | SSGWY-<br>...................<br>.-.... | ...... |
| iPS:39 2992 | 21-225_26C4 | VH1\|1-08/D6\|6-19\|RF1/JH4 | ...........<br>...........<br>........ | N......... | .....S..<br>...... | ........<br>R... | ..........N.....<br>................S | SSGWY-<br>...................<br>.-.... | ...... |
| iPS:39 3088 | 21-225_33D1 | VH1\|1-08/D6\|6-19\|RF1/JH4 | ...........<br>...........<br>........ | N......... | ........<br>...... | ..H.....<br>.....F..<br>..R. | ................<br>................S | SSGWY-<br>...................<br>.-F... | ...... |
| iPS:39 3144 | 21-225_34D2 | VH1\|1-08/D6\|6-19\|RF1/JH4 | ...........<br>...........<br>........ | N......... | ........<br>....V. | .LH.....<br>..T..F..<br>..R. | ...............L..<br>................S | SSGWY-<br>...................<br>.-F... | ...... |
| iPS:39 3368 | 21-225_29H8 | VH1\|1-08/D6\|6-19\|RF1/JH4 | ...........<br>Q..........<br>........ | N......... | ........<br>...... | ........<br>R... | ...........A.....<br>................S | SSGWY-<br>...................<br>.-.... | ...... |
| iPS:39 3942 | 21-225_11E5 | VH1\|1-08/D6\|6-19\|RF1/JH4 | ........P..<br>...........<br>........ | N......... | ........<br>...... | ..H.....<br>..A.....<br>R... | ................<br>...............T | SSGWE-<br>...................<br>.-V... | ...... |
| iPS:39 4085 | 21-225_8B11 | VH1\|1-08/D6\|6-19\|RF1/JH4 | ...........<br>...........<br>........ | N......... | .....A..<br>...... | ..H.....<br>........<br>.... | ........R.....<br>................Y | SSGWY-<br>...................<br>.-F... | ...... |
| iPS:39 8496 | 21-225_22D2 | VH1\|1-08/D6\|6-19\|RF1/JH4 | ...........<br>...........<br>.......I | N......... | ........<br>...... | ..H.D...<br>........<br>.... | ................<br>................Y | SSGWY-<br>...................<br>.-.... | ...... |

| ID | Clone | Germline | H_FR1 | H_CDR1 | H_FR2 | H_CDR2 | H_FR3 | H_CDR3 | H_FR4 |
|---|---|---|---|---|---|---|---|---|---|
| iPS:39 8522 | 21-225_32A1 | VH1\|1-08\|D6\|6-19\|RF1\|JH4 | ...........<br>...........<br>........ | N......... | ........<br>...... | ........<br>........<br>.... | ................<br>................S | SSGWY-<br>.................<br>.-F... | ......<br>..... |
| iPS:39 8524 | 21-225_32A5 | VH1\|1-08\|D6\|6-19\|RF1\|JH4 | ...........<br>...........<br>........ | N......... | ........<br>...... | ..H.....<br>.....F..<br>..R. | ................<br>................SS | SSGWY-<br>.................<br>.-F... | ......<br>..... |
| iPS:39 8538 | 21-225_34H7 | VH1\|1-08\|D6\|6-19\|RF1\|JH4 | ...........<br>...........<br>........ | N......... | ........<br>...... | ........<br>........<br>.... | ................<br>N.............S | SSGWY-<br>.................<br>.-F... | ......<br>..... |
| | Germline | VH1\|1-02/D1\|1-1\|RF1/JH4 | QVQLVQS-GAEVKKPGASVK VSCKASG-YTFT | G------YYMH | WVRQAPGQ GLEWMG | WINPN--- SGGTNYAQ KFQG | RVTMTRDTSISTAYMEL SRLRSDDTAVYYCAR | GTTGT------------------YFDY | WGQGTL VTVSS |
| iPS:47 3253 | 21-225_7C3_LC1 | VH1\|1-02/D1\|1-1\|RF1/JH4 | ...........<br>...........<br>.......N | D.......L. | ........<br>...... | ..H.....<br>........<br>.... | ................<br>.S..........S... | DG.S-<br>.................<br>...S... | ......<br>..... |
| iPS:47 3254 | 21-225_7C3_LC2 | VH1\|1-02/D1\|1-1\|RF1/JH4 | ...........<br>...........<br>.......N | D.......L. | ........<br>...... | ..H.....<br>........<br>.... | ................<br>.S..........S... | DG.S-<br>.................<br>...S... | ......<br>..... |
| iPS:47 3255 | 21-225_9F12_LC1 | VH1\|1-02/D1\|1-1\|RF1/JH4 | ...........<br>...........<br>.......A | D.......L. | ........<br>...... | ..H.....<br>.....F..<br>.... | ...............L..<br>.S.......F...... | DG.S-<br>.................<br>...S... | ......<br>..... |
| iPS:47 3256 | 21-225_9F12_LC2 | VH1\|1-02/D1\|1-1\|RF1/JH4 | ...........<br>...........<br>.......A | D.......L. | ........<br>...... | ..H.....<br>.....F..<br>.... | ...............L..<br>.S.......F...... | DG.S-<br>.................<br>...S... | ......<br>..... |
| iPS:42 6108 | 21-225_10G6 | VH1\|1-02/D1\|1-1\|RF1/JH4 | ...........<br>...........<br>........ | A......H.. | ........<br>...... | ........<br>NN......<br>.... | ................<br>...............G. | DV.S-<br>.................<br>...S... | ......<br>..... |
| iPS:42 6110 | 21-225_12E9 | VH1\|1-02/D1\|1-1\|RF1/JH4 | ...........<br>...........<br>........ | D.......L. | ........<br>...... | .VH.....<br>.....F..<br>...D | ................<br>.S........I.S... | DG.S-<br>.................<br>...S... | ......<br>..... |
| iPS:45 3451 | 21-225_52G11 | VH1\|1-02/D1\|1-1\|RF1/JH4 | ...........<br>...........<br>........ | ........L. | ........<br>...... | ........<br>RN......<br>.... | ..............F...<br>................ | DG.S-<br>.................<br>...S... | ......<br>..... |

3148

| | | | | | | | | | |
|---|---|---|---|---|---|---|---|---|---|
| iPS:45 3453 | 21-225_53F2 | VH1\|1-02/D1\|1-1\|RF1/JH 4 | .......... <br> .......... <br> ....... | ......L. | ....... <br> ...... | ........ <br> RN...... <br> N... | ................ <br> ...K............ | DG.S- <br> ................ <br> ...S... | ...... <br> ...... |
| iPS:43 4035 | 21-225_49F10 | VH1\|1-02/D1\|1-1\|RF1/JH 4 | .......... <br> .......... <br> ....... | ......H.. | ....... <br> ...... | ........ <br> NNA..... <br> N... | ...L............ | DG.S- <br> ................ <br> ...S..F | ...... <br> ...... |
| iPS:43 4065 | 21-225_50D4 | VH1\|1-02/D1\|1-1\|RF1/JH 4 | .......... <br> .......... <br> ....... | ......H.. | ....... <br> ...... | ........ <br> NNA..... <br> S... | ...L............ | DG.S- <br> ................ <br> ...S..F | ...... <br> ...... |
| iPS:43 4069 | 21-225_51E9 | VH1\|1-02/D1\|1-1\|RF1/JH 4 | .......... <br> ..R..... <br> ....... | ......HI. | ....... <br> ...... | ........ <br> TN..Q... <br> .... | ................ <br> ................ | DG.S- <br> ................ <br> ...S... | ...... <br> ...... |
| iPS:43 4079 | 21-225_52B1 | VH1\|1-02/D1\|1-1\|RF1/JH 4 | .......... <br> .......... <br> ....... | ......H.Q | ....... <br> ...... | ........ <br> ..A..... <br> N... | ..............LD. | DG.S- <br> ................ <br> ...S... | ...... <br> ...... |
| iPS:43 4097 | 21-225_52H10 | VH1\|1-02/D1\|1-1\|RF1/JH 4 | .......... <br> .......... <br> ....... | ......H.Q | ....... <br> ...... | ........ <br> N...Q... <br> .... | ................ | DG.S- <br> ................ <br> ...S... | ...... <br> ...... |
| iPS:43 4123 | 21-225_53F7 | VH1\|1-02/D1\|1-1\|RF1/JH 4 | .......... <br> .......... <br> ....... | ......H.. | ....... <br> ...... | ........ <br> NN...... <br> .... | N..T........... | DG.S- <br> ................ <br> ...S... | ...... <br> ...... |
| iPS:43 4189 | 21-225_56E5 | VH1\|1-02/D1\|1-1\|RF1/JH 4 | .......... <br> .........Q <br> ....... | ......H.. | ....... <br> ...... | ........ <br> NNA..... <br> .... | R..........H... | DG.S- <br> ................ <br> ...S... | ...... <br> ...... |
| iPS:43 5677 | 21-225_169C10 | VH1\|1-02/D1\|1-1\|RF1/JH 4 | .......... <br> .......R.... <br> ....... | ......F.. | ....... <br> ...... | ..K.K... <br> .....S.. <br> R... | ..........N...... <br> N.............. | .G.TVAT.......... <br> ......WGV... | ...... <br> ...... |
| iPS:43 5699 | 21-225_170D6 | VH1\|1-02/D1\|1-1\|RF1/JH 4 | .......... <br> .......R.... <br> ....... | ......FI. | ....... <br> ...... | ..K..... <br> .....S.. <br> R... | ..........N...... <br> N.............. | .G.TVAT.......... <br> ......WGV... | ...... <br> ...... |
| iPS:43 5797 | 21-225_181G2 | VH1\|1-02/D1\|1-1\|RF1/JH 4 | ......... <br> .T.........R <br> ....... | S......N.. | ....V... <br> ...... | ........ <br> N..S..T. <br> .... | ................ | KF--- <br> ................ <br> ...--GD | ...... <br> ...... |
| iPS:43 5877 | 21-225_184E7 | VH1\|1-02/D1\|1-1\|RF1/JH 4 | ......... <br> .T.........R <br> ....... | S......N.. | ....V... <br> ...... | ........ <br> N..S..T. <br> .... | ................ | KF--- <br> ................ <br> ...--GD | ...... <br> ...... |

| iPS:43 5885 | 21-225_185E10 | VH1\|1-02/D1\|1-1\|RF1/JH4 | ............<br>.T.........R<br>........ | S......N.. | ....V...<br>...... | ........<br>N..S..T.<br>.... | ................ | KF---<br>................<br>...--GD | ...... |
|---|---|---|---|---|---|---|---|---|---|
| iPS:43 5891 | 21-225_188H5 | VH1\|1-02/D1\|1-1\|RF1/JH4 | ............<br>.T.........R<br>........ | S......N.. | ....V...<br>...... | ........<br>...S..T.<br>.... | .I.............. | KF---<br>................<br>...--CD | ...... |
| iPS:43 5897 | 21-225_188B9 | VH1\|1-02/D1\|1-1\|RF1/JH4 | ............<br>.T.......R<br>........ | S......N.. | ....V...<br>...... | ........<br>...S..T.<br>.... | ................ | KF---<br>................<br>...--GD | ...... |
| iPS:43 6400 | 21-225_213H7 | VH1\|1-02/D1\|1-1\|RF1/JH4 | ............<br>............<br>........ | ......H.. | ........<br>...... | ....K...<br>.D......<br>.... | ................ | EKP.S..........<br>.........YK. | ......<br>...... |
| iPS:43 6488 | 21-225_221A6 | VH1\|1-02/D1\|1-1\|RF1/JH4 | ............<br>............<br>T....... | .......... | ........<br>...... | ..H.....<br>........<br>.... | ...L..........D. | DG.S-<br>................<br>...S... | ...... |
| iPS:43 6496 | 21-225_222E1 | VH1\|1-02/D1\|1-1\|RF1/JH4 | ............<br>............<br>T....... | .......... | ........<br>...... | ..H.....<br>........<br>.... | ...L..........D. | DG.S-<br>................<br>...S... | ...... |
| iPS:43 6508 | 21-225_222F7 | VH1\|1-02/D1\|1-1\|RF1/JH4 | ............<br>............<br>T....... | .......... | ........<br>...... | ..H.....<br>........<br>.... | ...L..........D. | DG.S-<br>................<br>...S... | ...... |
| iPS:43 6516 | 21-225_222C12 | VH1\|1-02/D1\|1-1\|RF1/JH4 | ............<br>............<br>T....... | .......... | ........<br>...... | ..H.....<br>........<br>.... | ...L..........D. | DG.S-<br>................<br>...S... | ...... |
| iPS:43 7264 | 21-225_171H12 | VH1\|1-02/D1\|1-1\|RF1/JH4 | ............<br>......R....<br>........ | .......F.. | ........<br>...... | ..K.K...<br>.....S..<br>R... | .........N...... | .G.TVAT.........<br>......WGV... | ......<br>...... |
| iPS:43 7266 | 21-225_177A5 | VH1\|1-02/D1\|1-1\|RF1/JH4 | ............<br>......R....<br>........ | .......F.. | ........<br>...... | ..K.K...<br>.....S..<br>R... | NW.............. | .G.TVAT.........<br>......WGV... | ......<br>...... |
| iPS:39 3080 | 21-225_34F3 | VH1\|1-02/D1\|1-1\|RF1/JH4 | ............<br>............<br>........ | .......H.. | ..D...<br>...... | ........<br>........<br>.... | ................ | DG.S-<br>................<br>...S... | ......<br>...... |
| iPS:39 3084 | 21-225_35C6 | VH1\|1-02/D1\|1-1\|RF1/JH4 | .........T..<br>............<br>........ | ......D... | ........<br>...... | ..S.K...<br>N.......<br>.... | N.............. | DG..-<br>................<br>...S... | ......<br>...... |

| | | Germline | H_FR1 | H_CDR1 | H_FR2 | H_CDR2 | H_FR3 | H_CDR3 | H_FR4 |
|---|---|---|---|---|---|---|---|---|---|
| IPS:39 3086 | 21-225_36H5 | VH1\|1- 02/D1\|1- 1\|RF1/JH 4 | ...........<br>......M.....<br>........ | D......H.. | ........<br>...... | .......<br>R.......<br>...D | .................<br>...........F... | DG..-<br>.................<br>...S... | ......<br>..... |
| IPS:39 3098 | 21-225_35G6 | VH1\|1- 02/D1\|1- 1\|RF1/JH 4 | .........D.<br>...........<br>........ | D......HI. | ........<br>...... | .......<br>N...H...<br>E... | .................<br>.S............. | DG..-<br>.................<br>...S... | ......<br>..... |
| IPS:39 3112 | 21-225_33G1 | VH1\|1- 02/D1\|1- 1\|RF1/JH 4 | ....A.......<br>...........<br>........ | ......... | ........<br>...... | .......<br>..S.....<br>N....<br>.... | .................<br>................. | DG..-<br>.................<br>...S... | ......<br>..... |
| IPS:39 3116 | 21-225_34G7 | VH1\|1- 02/D1\|1- 1\|RF1/JH 4 | ...........<br>..........R<br>........ | D......HI. | ........<br>...... | .......<br>N...H...<br>.... | .................<br>................. | DG..-<br>.................<br>...S... | ....N.<br>..... |
| IPS:39 3132 | 21-225_33H7 | VH1\|1- 02/D1\|1- 1\|RF1/JH 4 | .........D.<br>...........<br>........ | D......HI. | ........<br>...... | .......<br>N...H...<br>E... | .................<br>.S.........H... | DG..-<br>.................<br>...S... | ......<br>..... |
| IPS:39 3140 | 21- 225_35H12 | VH1\|1- 02/D1\|1- 1\|RF1/JH 4 | ...........<br>...........<br>........ | D.......I. | ........<br>...... | .......<br>R.......<br>.... | .................<br>................. | DG..-<br>.................<br>...S... | ......<br>..... |
| IPS:39 3954 | 21-225_4H6 | VH1\|1- 02/D1\|1- 1\|RF1/JH 4 | ...........<br>...........<br>........ | D.......L. | ........<br>...... | .......<br>..H.....<br>.... | ................G.<br>.S............. | DG.S-<br>.................<br>...S... | ......<br>..... |
| IPS:39 8484 | 21-225_18D4 | VH1\|1- 02/D1\|1- 1\|RF1/JH 4 | ...........<br>...........<br>........ | ........L. | ........<br>....L. | .......<br>.N..IS..<br>.... | .................<br>...I............. | DG.S-<br>.................<br>...SL.. | ......<br>..... |
| IPS:39 8502 | 21- 225_23B11 | VH1\|1- 02/D1\|1- 1\|RF1/JH 4 | ...........<br>...........<br>........ | ........L. | ........<br>...... | .......<br>NN......<br>.... | .................<br>................. | DG.S-<br>.................<br>...S... | ......<br>..... |
| IPS:39 8520 | 21-225_31C4 | VH1\|1- 02/D1\|1- 1\|RF1/JH 4 | .........T..<br>...........<br>........ | ......D... | ........<br>...... | .......<br>..S.K...<br>N....<br>.... | .............V....<br>N.............. | DG..-<br>.................<br>...S... | ......<br>..... |
| IPS:40 2223 | 21- 225_30A11 | VH1\|1- 02/D1\|1- 1\|RF1/JH 4 | ...........<br>...........<br>........ | D......H.. | ........<br>...... | .......<br>R.......<br>...D | .................<br>...........F... | DG..-<br>.................<br>...S... | ......<br>..... |

| | VH3\|3-33/D6\|6-6\|RF1/JH6 | | QVQLVES-GGGVVQPGRSLR LSCAASG FTFS | S-----YGMH | WVRQAPGK GLEWVA | VIWYD--- GSNKYYAD SVKG | RFTISRDNSKNTLYLQM NSLRAEDTAVYYCAR | EYSSSSYY-------------- ---YYYGMDV | WGQGTT VTVSS |
|---|---|---|---|---|---|---|---|---|---|
| IPS:42 6114 | 21-225_28H2 | VH3\|3-33/D6\|6-6\|RF1/JH 6 | ............ ............ ........ | N.......V.. | ........ ...... | ........ .... | ...............V......... | .EY..GW- ................D. .... | ...... ..... |
| IPS:42 6116 | 21-225_29E2 | VH3\|3-33/D6\|6-6\|RF1/JH 6 | ............ ............ ........ | N.....CV.. | ........ ...... | ........ .... | .I.................. | .EY..GW- ................D. .... | ...... ..... |
| IPS:46 8812 | 21-225_48H4 | VH3\|3-33/D6\|6-6\|RF1/JH 6 | ............ ............ ........ | D.....SL.. | ........ ...... | ........ ......T. .... | ................... | .NY..GW- ...............G. .... | ...... ..... |
| IPS:46 8816 | 21-225_52G8 | VH3\|3-33/D6\|6-6\|RF1/JH 6 | ............ ............ ........ | .......... | ........ ...... | ........ .... | ..........L..... | R....W-- ...............- SG.... | ...... ..... |
| IPS:46 8826 | 21-225_201C5 | VH3\|3-33/D6\|6-6\|RF1/JH 6 | ............ ............ ........ | D......V.. | ........ ...... | ........ ......V. .... | .........R....... | .RY..GL- ................D. .... | ...... ..... |
| IPS:46 8842 | 21-225_50H4 | VH3\|3-33/D6\|6-6\|RF1/JH 6 | ............ ............ ........ | .......V.. | ........ ...... | A....... ......... .... | ..............D... | .LY..NW- ................D. .... | ...... ..... |
| IPS:46 8858 | 21-225_148C9 | VH3\|3-33/D6\|6-6\|RF1/JH 6 | ............ A........... ........ | D......... | ........ ...... | ........ .... | .L.............. | .RY..GW- ................D. .L.. | ...... ..... |
| IPS:46 8860 | 21-225_224E7 | VH3\|3-33/D6\|6-6\|RF1/JH 6 | ............ .......S.... ........ | .......V.. | ........ ...... | ........ .... | ................... | .QY...W- ...............DF .L.. | ...... ..... |
| IPS:43 3917 | 21-225_43E11 | VH3\|3-33/D6\|6-6\|RF1/JH 6 | ............ ............ .....S.. | D......... | ........ ...... | ........ .... | .L.............. | R.VR.W-- ...............- VG.... | ...... ..... |
| IPS:43 3919 | 21-225_44B3 | VH3\|3-33/D6\|6-6\|RF1/JH 6 | ............ ...........T ........ | D......... | ........ ...... | ........ .... | ................... | .RY..GW- ................D. .... | ...... ..... |
| IPS:43 3923 | 21-225_44D3 | VH3\|3-33/D6\|6-6\|RF1/JH 6 | ............ ............ ........ | .......V.. | ........ ...... | ........ .... | ................... | .RY..GL- ................D. .... | ...... ..... |

| | | | | | | | | | | |
|---|---|---|---|---|---|---|---|---|---|---|
| iPS:43 3929 | 21-225_44D5 | VH3\|3-33/D6\|6-6\|RF1/JH6 | . . . . . . . . . . .<br>. . . . . . . . . .<br>. . . . . . . | . . . . . . . V . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | VPY . . . W–<br>. . . . . . . . . . . . . . . . . D .<br>. . . . | . . . . . . |
| iPS:43 3935 | 21-225_44F9 | VH3\|3-33/D6\|6-6\|RF1/JH6 | . . . . . . . . . . .<br>. . . . . . . . . .<br>. . . . . . . | . . . . . . . V . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | . PY . . . W–<br>. . . . . . . . . . . . . . . . . D .<br>. . . . | G . . . . . .<br>. . . . . |
| iPS:43 3937 | 21-225_44B10 | VH3\|3-33/D6\|6-6\|RF1/JH6 | . . . . . . . . . . .<br>. . . . . . . . . . V<br>. . . . . . . | . . . . . . . . . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | R . . . . W– –<br>. . . . . . . . . . . . . . . –<br>VG . . . . | . . . . . .<br>. . . . . |
| iPS:43 3939 | 21-225_44C10 | VH3\|3-33/D6\|6-6\|RF1/JH6 | . . . . . . . . . . .<br>. . . . . . . . . .<br>. . . . . . . | D . . . . . CV . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | . RY . . CL–<br>. . . . . . . . . . . . . . . . . D .<br>. . . . | . . . . . . |
| iPS:43 3951 | 21-225_45B4 | VH3\|3-33/D6\|6-6\|RF1/JH6 | . . . . . . . . . . .<br>. . . . . . . . . .<br>. . . . . . . | D . . . . . CV . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | . RY . . GL–<br>. . . . . . . . . . . . . . . . . D .<br>. . . . | . . . . . . |
| iPS:43 3955 | 21-225_45B8 | VH3\|3-33/D6\|6-6\|RF1/JH6 | . . . . . . . . . . .<br>. . . . . . . . . .<br>. . . . . . . | D . . . . . CV . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | . RY . . GL–<br>. . . . . . . . . . . . . . . . . D .<br>. . . . | . . . . . . |
| iPS:43 3967 | 21-225_46C3 | VH3\|3-33/D6\|6-6\|RF1/JH6 | . . . . . . . . . . .<br>. . . . . . . . . .<br>. . . . . . . | . . . . . . . V . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | . RY . . GL–<br>. . . . . . . . . . . . . . . . . D .<br>. . . . | . . . . . . |
| iPS:43 3971 | 21-225_46D4 | VH3\|3-33/D6\|6-6\|RF1/JH6 | . . . . . . . . . . .<br>. . . . . . . . . .<br>. . . . . . . | . . . . . . . V . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | VPY . . . W–<br>. . . . . . . . . . . . . . . . . D .<br>. . . . | . . . . . . |
| iPS:43 3979 | 21-225_46B9 | VH3\|3-33/D6\|6-6\|RF1/JH6 | . . . . . . . . . . .<br>. . . . . . . . . . S<br>. . . . . . . | . . . . . . . . . . | . . . . . . . .<br>. . . . . .<br>. . . . | . . . . . . . .<br>. R . . . . .<br>. . . . | . I . . . . . . . . . . . . . . .<br>. . . . . . . . . . . . . . . . | R . . . . W– –<br>. . . . . . . . . . . . . . . –<br>MG . . . . | . . . . . . |
| iPS:43 3985 | 21-225_47C1 | VH3\|3-33/D6\|6-6\|RF1/JH6 | . . . . . . . . . . .<br>. . . . . . . . . .<br>. . . . . . . | D . . . . . . . . . | . . . . T . . . .<br>. . . . . .<br>. . . . | . . . . . . . .<br>. . . . . . . .<br>. . . . | . . . . . . . . . . . . . F . . .<br>. . . . D . . . . . . . . . | R . . R . W– –<br>. . . . . . . . . . . . . . . –<br>VG . . . . | . . . . . . |
| iPS:43 3991 | 21-225_47E7 | VH3\|3-33/D6\|6-6\|RF1/JH6 | . . . . . . . . . . .<br>. . . . . . . . . .<br>. . . . . . . | I . . . . . . . . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | R . . R . W– –<br>. . . . . . . . . . . . . . . –<br>VG . . . . | . . . . . . |
| iPS:43 4001 | 21-225_48F2 | VH3\|3-33/D6\|6-6\|RF1/JH6 | . . . . . . . . . . .<br>. . . . . . . . . .<br>. . . . . . . | N . . . . . . V . . | . . . . . . . .<br>. . . . . .<br>. . . . | . . . . . . . .<br>. . . . . . . .<br>. . . . | . . . . . . . . . . . . . . . . .<br>. . . . . . . . . . . . . . . . | . RY . . . W–<br>. . . . . . . . . . . . . . . . . D .<br>. L . . | . . . . . . |

| | | | | | | | | | |
|---|---|---|---|---|---|---|---|---|---|
| iPS:43 4021 | 21-225_49C1 | VH3\|3-33/D6\|6-6\|RF1/JH6 | . . . . . . . . . . . <br> . . . . . . . . . . .T <br> . . . . . . . . | . . . . . . . . . . . | . . . . . . . . <br> . . . . . . <br> . . . . | . . . . . . . . <br> . . . . . . . . <br> . . . . | . . . . . . . . . . . . . . . . . | R . . . . W-- <br> . . . . . . . . . . . . . . .- <br> SG . . . . | . . . . . . |
| iPS:43 4025 | 21-225_49G3 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | . . . . . . . . <br> . . . . . . . . <br> . . . . | . . . . . . . . . . . . . . . . . | R . . . . W-- <br> . . . . . . . . . . . . . . .- <br> SG . . . . | . . . . . . |
| iPS:43 4031 | 21-225_49E7 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | . . . . . . . . <br> . . . . . . . . <br> . . . . | . . . . . . . . . . . . . . . . . <br> . . . . . . . . . .L . . . . . . | R . . . . W-- <br> . . . . . . . . . . . . . . .- <br> SG . . . . | . . . . . . |
| iPS:43 4033 | 21-225_49F9 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | L . . . . . . . <br> . R . . . . . . <br> . . . . | . . . . . . . .P . . . . . . . <br> . . . . . . . . . . .H . . . | R . . . . W-- <br> . . . . . . . . . . . . . . .- <br> SG . . . . | . . . . . . |
| iPS:43 4053 | 21-225_51E1 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | . . . . . . . . <br> . . S . . . . . <br> . . . . | . . . . . . . . . . . . . . . . . | R . . . . W-- <br> . . . . . . . . . . . . . . .- <br> SG . . . . | . . . . . . |
| iPS:43 4093 | 21-225_52D10 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | L . . . . . . . <br> . . . . .H . . <br> . . . . | . . . . . . . . . . . . . . . . . | R . . . . W-- <br> . . . . . . . . . . . . . . .- <br> SG . . . . | . . . . . . |
| iPS:43 4137 | 21-225_54D4 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | . . . . . . . . <br> . . . . . . . . <br> . . . . | . . . . . . . . . . . . . . . . . | R . . . . W-- <br> . . . . . . . . . . . . . . .- <br> SG . . . . | . . . . . . |
| iPS:43 4149 | 21-225_55H1 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | . . . . . . . . <br> . . . . . . . . <br> . . . . | . . . . . . . . . . . . . . . . . | R . . . . W-- <br> . . . . . . . . . . . . . . .- <br> SG . . . . | . . . . . . |
| iPS:43 4151 | 21-225_55C2 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | L . . . . . . . <br> . . . . .H . . <br> . . . . | . . . . . . . . . . . . . . . . . | R . . . . W-- <br> . . . . . . . . . . . . . . .- <br> SG . . . . | . . . . . . |
| iPS:43 4161 | 21-225_55F9 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | . . . . . . . . <br> .NG . . . . . <br> . . . . | . . . . . . . . . . . . . . . . . | R . . . . W-- <br> . . . . . . . . . . . . . . .- <br> SG . . . . | . . . . . . |
| iPS:43 4201 | 21-225_59A12 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | . . . . . . . . <br> . . . . . . . . <br> . . . . | . . . . . . . . . . . . . . . . . | R . . . . W-- <br> . . . . . . . . . . . . . . .- <br> DG . . . . | . . . . . . |
| iPS:43 4205 | 21-225_60G2 | VH3\|3-33/D6\|6-6\|RF1/JH6 | . . . . . . . . . . . <br> . . . . . . . . . . . <br> . . . . . . . . | . . . . . . . . . . . | . . . . . . . . <br> . . . . . . <br> . . . . | . . . . . . . . <br> . . . . . . . . <br> . . . . | . . . . . . . . . . . . . . . . . | R . . R .W-- <br> . . . . . . . . . . . . . . .- <br> TG . . . . | . . . . . . |

| | | | | | | | | | |
|---|---|---|---|---|---|---|---|---|---|
| iPS:43 4223 | 21-225_60C12 | VH3|3-33/D6|6-6|RF1/JH6 | ............ ............ ........ | ........... | ........ ...... | ........ ......V. .... | ................... ................ | R..R.W-- ...............- TG.... | ...... ..... |
| iPS:43 4231 | 21-225_61F2 | VH3|3-33/D6|6-6|RF1/JH6 | ............ ............ ........ | ........... | ........ ...... | ........ ........ .... | ................... ................ | .RY..GW- ...................D. .... | ...... ..... |
| iPS:43 4233 | 21-225_61B3 | VH3|3-33/D6|6-6|RF1/JH6 | ............ ............ ........ | ........... | ........ ...... | ........ ........ .... | ................... ................ | R..R.W-- ...............- AG.... | ...... ..... |
| iPS:43 4303 | 21-225_58H11 | VH3|3-33/D6|6-6|RF1/JH6 | ............ ............ ........ | ........... | ........ ...... | ........ ......H.. .... | ................... ................ | R....W-- ...............- DG.... | ...... .... |
| iPS:43 4339 | 21-225_64A4 | VH3|3-33/D6|6-6|RF1/JH6 | ............ ............ ........ | D......V.. | ........ ...... | ........ ........ .... | .........Q... ................ | .RY...W- ...................D. .... | ...... ..... |
| iPS:43 4343 | 21-225_64C8 | VH3|3-33/D6|6-6|RF1/JH6 | ............ ............ ........ | D......V.. | ........ ...... | ........ .......M.. | ................... ................ | .RY..GW- ...................D. .... | ...... ..... |
| iPS:43 4387 | 21-225_66D11 | VH3|3-33/D6|6-6|RF1/JH6 | ............ ..........G ........ | ........... | ........ ...... | ........ ........ .... | ...........S.... ........L..... | .MY..NW- ...................D. .L.. | ...... ..... |
| iPS:43 4469 | 21-225_73C9 | VH3|3-33/D6|6-6|RF1/JH6 | ............ ............ ........ | N......V.. | ........ ...... | ........ ........ .... | ................... ................ | .RY...W- ..................FD. .... | ...... ..... |
| iPS:43 5197 | 21-225_94F3 | VH3|3-33/D6|6-6|RF1/JH6 | ............ ............ ........ | N.....DI.. | ........ ...... | ........ ........ .... | ...............I ...........F... | .KY..GW- ...................D. .... | ...... ..... |
| iPS:43 5315 | 21-225_147B2 | VH3|3-33/D6|6-6|RF1/JH6 | ............ ............ ........ | ........... | ........ ...... | ........ ........ .... | ................... ................ | R....W-- ...............- SG.... | ...... ..... |
| iPS:43 5325 | 21-225_147H5 | VH3|3-33/D6|6-6|RF1/JH6 | ............ A........... ........ | D......... | ........ ...... | ........ ........ .... | .L.............. ...S........... | .RY..GW- ...................D. .L.. | ...... ..... |
| iPS:43 5329 | 21-225_147A8 | VH3|3-33/D6|6-6|RF1/JH6 | ............ ............ ........ | ........... | ........ ...... | ........ ........ .... | ................... ................ | R....W-- ...............- TG.... | ...... ..... |

| iPS:43 5349 | 21-225_148F5 | VH3|3-33/D6|6-6|RF1/JH6 | ...........<br>...........<br>........ | ..........<br><br> | ........<br>......<br>.... | ........<br>........<br>.... | .................<br><br> | R....W--<br>................⁻<br>SG.... | ......<br>.... |
|---|---|---|---|---|---|---|---|---|---|
| iPS:43 5359 | 21-225_148H10 | VH3|3-33/D6|6-6|RF1/JH6 | ...........<br>...........<br>........ | ..........<br><br> | ........<br>......<br>.... | ........<br>......G.<br>.... | .................<br><br> | R....W--<br>................⁻<br>SG.... | ......<br>.... |
| iPS:43 5393 | 21-225_149D10 | VH3|3-33/D6|6-6|RF1/JH6 | ...........<br>A..........<br>........ | D.........<br><br> | ........<br>......<br>.... | ........<br>........<br>.... | .L..............<br><br> | .RY..GW--<br>...................D.<br>.... | ......<br>.... |
| iPS:43 5401 | 21-225_150E2 | VH3|3-33/D6|6-6|RF1/JH6 | ...........<br>...........<br>........ | .......V..<br><br> | ........<br>......<br>.... | ........<br>........<br>.... | .................<br><br> | .EY...W--<br>..................G.<br>.... | ......<br>.... |
| iPS:43 5417 | 21-225_150D11 | VH3|3-33/D6|6-6|RF1/JH6 | ...........<br>...........<br>........ | ..........<br><br> | ........<br>......<br>.... | ..F.....<br>....H...<br>.... | .......S.....T.<br><br> | RF...W--<br>................⁻<br>SG.... | ......<br>.... |
| iPS:43 5445 | 21-225_152F7 | VH3|3-33/D6|6-6|RF1/JH6 | ...........<br>...........<br>........ | .......I..<br><br> | ........<br>......<br>.... | ........<br>........<br>.... | .................<br><br> | .EY...W--<br>..................G.<br>.... | ......<br>.... |
| iPS:43 5469 | 21-225_153G9 | VH3|3-33/D6|6-6|RF1/JH6 | ...........<br>...........<br>........ | ..........<br><br> | ........<br>......<br>.... | L.F.....<br>........<br>.... | ................I<br>...........F...<br> | R..R.W--<br>................⁻<br>AG.... | .....A<br>.... |
| iPS:43 5573 | 21-225_159D8 | VH3|3-33/D6|6-6|RF1/JH6 | ...........<br>...........<br>........ | N......V..<br><br> | C.......<br>......<br>.... | ........<br>........<br>.... | .................<br><br> | .PY..GW--<br>...................D.<br>.... | ......<br>.... |
| iPS:43 5681 | 21-225_169D11 | VH3|3-33/D6|6-6|RF1/JH6 | ...........<br>...........<br>........ | D......V..<br><br> | ........<br>......<br>.... | ........<br>........<br>.... | .................L<br><br> | .RY..GW--<br>...................D.<br>.... | ......<br>.... |
| iPS:43 5689 | 21-225_170F3 | VH3|3-33/D6|6-6|RF1/JH6 | ...........<br>...........<br>.....S.. | D......V..<br><br> | ........<br>......<br>.... | ........<br>......E.<br>.... | .................<br><br> | .TY...W--<br>..................D.<br>.... | ......<br>.... |
| iPS:43 5733 | 21-225_173C11 | VH3|3-33/D6|6-6|RF1/JH6 | ...........<br>...........<br>........ | ........I.<br><br> | ........<br>......<br>.... | L.F.....<br>........<br>.... | ................H.<br>S...............<br> | R....W--<br>................⁻<br>SG.... | ......<br>.... |
| iPS:43 5741 | 21-225_174G10 | VH3|3-33/D6|6-6|RF1/JH6 | ...........<br>...........<br>........ | D......V..<br><br> | ........<br>......<br>.... | ........<br>........<br>.... | .................L<br><br> | .RY..GW--<br>...................D.<br>.... | ......<br>.... |

| | | | | | | | | | |
|---|---|---|---|---|---|---|---|---|---|
| iPS:43 5763 | 21-225_176H12 | VH3\|3-33/D6\|6-6\|RF1/JH6 | ............<br>............<br>........ | D......V.. | ........<br>......<br>.... | ........<br>........<br> | ................ | .KY..NW-<br>...............D.<br>.... | ......<br>..... |
| iPS:43 5767 | 21-225_177B4 | VH3\|3-33/D6\|6-6\|RF1/JH6 | ............<br>............<br>........ | D......V.. | ........<br>......<br>.... | ........<br>........<br> | ..IV............ | .KY...W-<br>...............D.<br>.L.. | ......<br>..... |
| iPS:43 5785 | 21-225_179C2 | VH3\|3-33/D6\|6-6\|RF1/JH6 | ............<br>............<br>........ | ........ | ........<br>......<br>.... | L.F.....<br>........<br> | ............F... | R..G.W--<br>..............‾<br>SG.... | ......<br>..... |
| iPS:43 5921 | 21-225_190D6 | VH3\|3-33/D6\|6-6\|RF1/JH6 | ............<br>............<br>........ | ......FI.. | .......R<br>......<br>.... | ........<br>........<br> | ................ | .EY..CW-<br>...............FG.<br>.... | ......<br>..... |
| iPS:43 5961 | 21-225_192A2 | VH3\|3-33/D6\|6-6\|RF1/JH6 | ............<br>............<br>........ | ........ | ........<br>....L.<br>.... | ........<br>..Y.....<br> | ................ | .D.PY.G.A.........<br>...LD.F.....<br> | ......<br>..... |
| iPS:43 5985 | 21-225_192F6 | VH3\|3-33/D6\|6-6\|RF1/JH6 | ............<br>............<br>........ | ......FI.. | .......R<br>......<br>.... | ........<br>......V.<br> | ................ | .EY..GW-<br>...............FG.<br>.... | ......<br>..... |
| iPS:43 6039 | 21-225_193F8 | VH3\|3-33/D6\|6-6\|RF1/JH6 | ............<br>............<br>........ | I........D | ........<br>......<br>.... | ........<br>..Y.....<br> | ..............V. | .D.PY.G.G.........<br>...LD.......<br> | ......<br>..... |
| iPS:43 6074 | 21-225_194F10 | VH3\|3-33/D6\|6-6\|RF1/JH6 | ............<br>............<br>........ | ......FI.. | .......R<br>......<br>.... | ........<br>........<br> | ...............E.<br>....V......... | .EY..GW-<br>...............FG.<br>.... | ......<br>..... |
| iPS:43 6264 | 21-225_203F7 | VH3\|3-33/D6\|6-6\|RF1/JH6 | ............<br>............<br>........ | D......V.. | ........<br>......<br>.... | ........<br>......V.<br> | .........R....... | .RY..GL-<br>...............D.<br>.... | ......<br>..... |
| iPS:43 6274 | 21-225_204H3 | VH3\|3-33/D6\|6-6\|RF1/JH6 | ............<br>G..........T<br>........ | ......V.. | ........<br>......<br>.... | ........<br>.....N..<br> | ................ | .PY...W-<br>...............D.<br>.... | S.....<br>..... |
| iPS:43 6332 | 21-225_208B2 | VH3\|3-33/D6\|6-6\|RF1/JH6 | ............<br>............<br>........ | D.....CV.. | ........<br>......<br>.... | ........<br>......V.<br> | .........R....... | .RY..GL-<br>...............D.<br>.L.. | ......<br>..... |
| iPS:43 6352 | 21-225_210G5 | VH3\|3-33/D6\|6-6\|RF1/JH6 | ............<br>............<br>........ | D.....CV.. | ........<br>......<br>.... | ........<br>......V.<br> | .........R....... | .RY..GL-<br>...............D.<br>.L.. | ......<br>..... |

| | | | | | | | | | |
|---|---|---|---|---|---|---|---|---|---|
| iPS:43 6386 | 21-225_212B11 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |
| iPS:43 6412 | 21-225_214H9 | VH3\|3-33/D6\|6-6\|RF1/JH 6 | ..........<br>..........<br>........ | .......V.. | .......<br>......<br>.... | .......<br>.......<br>.......F.... | ................ | .RYT..W—<br>...............D.<br>.... | ...... |
| iPS:43 6414 | 21-225_214G10 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |
| iPS:43 6416 | 21-225_214G12 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |
| iPS:43 6418 | 21-225_215E3 | VH3\|3-33/D6\|6-6\|RF1/JH 6 | ..........<br>..........<br>........ | D......VI. | .......<br>.....T | .......<br>.......<br>.... | ..V............ | .RY..GW—<br>...............D.<br>.... | ...... |
| iPS:43 6428 | 21-225_215E11 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |
| iPS:43 6438 | 21-225_216E8 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |
| iPS:43 6440 | 21-225_216H12 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |
| iPS:43 6450 | 21-225_217E5 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |
| iPS:43 6456 | 21-225_217G10 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |
| iPS:43 6458 | 21-225_217H12 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |
| iPS:43 6462 | 21-225_218C4 | VH3\|3-33/D6\|6-6\|RF1/JH 6 | ..........<br>..........<br>........ | D......V.. | .......<br>.....T | .......<br>.......<br>.... | ................ | .RY..GW—<br>...............D.<br>.... | ...... |

| | | | | | | | | | |
|---|---|---|---|---|---|---|---|---|---|
| iPS:43 6480 | 21-225_220F8 | VH3\|3-33/D6\|6-6\|RF1/JH6 | ............<br>............<br>........ | D......V.. | ........<br>.....T | ........<br>........<br>.... | ................ | .RY..CW-<br>................D.<br>.... | ...... |
| iPS:43 6534 | 21-225_224F1 | VH3\|3-33/D6\|6-6\|RF1/JH6 | ............<br>............<br>........ | .......I.. | ........<br>...... | ........<br>........<br>.... | ................ | .RY..NW-<br>................D.<br>.... | ...... |
| iPS:43 6540 | 21-225_224F3 | VH3\|3-33/D6\|6-6\|RF1/JH6 | ............<br>............<br>.....S.. | ........ | ........<br>...... | ........<br>........<br>.... | ................ | .RY...W-<br>................D.<br>.... | ...... |
| iPS:43 6564 | 21-225_225A1 | VH3\|3-33/D6\|6-6\|RF1/JH6 | ............<br>............<br>........ | D......VI. | ........<br>...... | ........<br>........<br>.... | ................ | .RY..CW-<br>................D.<br>.... | ...... |
| iPS:43 6596 | 21-225_226C6 | VH3\|3-33/D6\|6-6\|RF1/JH6 | ............<br>............<br>........ | D......... | ........<br>...... | ........<br>........<br>.... | ................T | .RY...W-<br>................D.<br>.... | ...... |
| iPS:43 6620 | 21-225_226H11 | VH3\|3-33/D6\|6-6\|RF1/JH6 | ............<br>............<br>........ | N.....C... | ........<br>.....T | ........<br>........<br>.... | ................ | .LY...W-<br>................D.<br>.L.. | ...... |
| iPS:43 6744 | 21-225_154F4 | VH3\|3-33/D6\|6-6\|RF1/JH6 | ............<br>............<br>........ | ........ | ........<br>...... | ........<br>........<br>.... | ............... | .D.YC.GTSC........<br>..PYY....... | ......<br>..... |
| iPS:43 6946 | 21-225_183F4 | VH3\|3-33/D6\|6-6\|RF1/JH6 | ............<br>............<br>........ | ........ | ........<br>...... | ........<br>........<br>.... | ............... | .RTYC.GTTC........<br>..PYY....LG. | ......<br>..... |
| iPS:43 7286 | 21-225_208F1 | VH3\|3-33/D6\|6-6\|RF1/JH6 | ............<br>..R..... | D......V.. | ........<br>...... | ........<br>......V.<br>.... | .........R....... | .RY..GL-<br>................D.<br>.... | ...... |
| iPS:43 7290 | 21-225_210G6 | VH3\|3-33/D6\|6-6\|RF1/JH6 | ............<br>............<br>........ | D......V.. | ........<br>...... | ........<br>......V.<br>.... | .........R....... | .RY..GL-<br>................D.<br>.... | ...... |
| iPS:39 2634 | 21-225_17H3 | VH3\|3-33/D6\|6-6\|RF1/JH6 | ............<br>............<br>........ | N.....CV.. | ........<br>...... | ........<br>........<br>.... | ................ | .KY...W-<br>................D.<br>.... | ...... |
| iPS:39 2742 | 21-225_20B2 | VH3\|3-33/D6\|6-6\|RF1/JH6 | ............<br>............<br>........ | N......VI. | ........<br>...... | ........<br>........<br>.... | ..........S... | .KY...W-<br>................D.<br>.... | ...... |

| iPS ID | Clone | Germline | | | | | | | |
|---|---|---|---|---|---|---|---|---|---|
| iPS:39 2836 | 21-225_22F4 | VH3\|3-33/D6\|6-6\|RF1/JH 6 | . . . . . . . . . . . . . . . . . . . . . . . . . . . . . . | D . . . . . . . . . | . . . . . . . . . . . . . . | . . . . . . . . . . . . . V . . . . . | . . . . . . . . . . . . . . . . . . . . . . . . | .KY...W— . . . . . . . . . . . . . . . . . . .D. . . . . | . . . . . . . . . . . |
| iPS:39 2846 | 21-225_24B6 | VH3\|3-33/D6\|6-6\|RF1/JH 6 | . . . . . . . . . . . . . . . . . P . . . . . . . . . . I . . | N . . . . . . V . . | . . . . . . . . . . . . . . | . . . . . . . . . . . . . . . . . | . . . . . . . . . . . . . . . . . . | .EY..GW— . . . . . . . . . . . . . . . . . . .D. . . . . | . . . . . . . . . . . |
| iPS:39 2884 | 21-225_23A10 | VH3\|3-33/D6\|6-6\|RF1/JH 6 | . . . . . . . . . . . . . . . . . . . . . . . . . . . . . . | . . . . . . . . . . | . . . . . . . . . . . . . . | . . . . . . . . . . . . . . . . . | . . . . . . . . . . . . . . . . . . | .RY..GW— . . . . . . . . . . . . . . .HD. . . . . | . . . . . . . . . . . |
| iPS:39 2888 | 21-225_25A2 | VH3\|3-33/D6\|6-6\|RF1/JH 6 | . . . . . . . . . . . . . . . . . . . . . . . . . . . . . . | . . . . . . . . . . | . . . . . . . . . . . . . . | . . . . . . . . . . . . . . . . . | . . . . . . . . . . . . . . . . . . | R....W-- . . . . . . . . . . . . . .— SG.... | . . . . . . . . . . . |
| iPS:39 2914 | 21-225_25D12 | VH3\|3-33/D6\|6-6\|RF1/JH 6 | . . . . . . . . . . . . . . . . . . . . . . . . . . . . . . | . . . . . . D . . . | . . . . . . . . . . . . . . | . . . . . . . . . . . . . . . . . | . . . . . . . . . . . . . . . . . . | .RY...W— . . . . . . . . . . . . . . . . . . .D. . . . . | . . . . . . . . . . . |
| iPS:39 2924 | 21-225_32H2 | VH3\|3-33/D6\|6-6\|RF1/JH 6 | . . . . . . . . . . . . . . . . . . . . . . . . . . . . . . | . . . . . . . . . . | . . . . . . . . . . . . . . | . . . . . . . . . . . . . . . . . | . . . . . . . . . . . . . . .N... . . . . . .G... | R....W-- . . . . . . . . . . . . . .— TG.... | . . . . . . . . . . . |
| iPS:39 2938 | 21-225_29H4 | VH3\|3-33/D6\|6-6\|RF1/JH 6 | . . . . . . . . . . . . . . . . . . . . . . . . . . . . . . | . . . . . . . . .I. | . . . . . . . . . . . . . . | . . . . . . . . . . . . . . . . . | . . . . . . . . . . . . . . . . . . | R....W-- . . . . . . . . . . . . . .— SG.... | . . . . . . . . . . . |
| iPS:39 2974 | 21-225_26A11 | VH3\|3-33/D6\|6-6\|RF1/JH 6 | . . . . . . . . . . . . . . . . . . . . . . . . . . . . . . | N . . . . . CV . . | . . . . . . . . . . . . . . | . . . . . . . . . . . . . . . . . | . . . . . . . . . . . . . . . . . . | .EY..GW— . . . . . . . . . . . . . . . . . . .D. . . . . | . . . . . . . . . . . |
| iPS:39 3012 | 21-225_26G7 | VH3\|3-33/D6\|6-6\|RF1/JH 6 | . . . . . . . . . . . . . . . . . . . . . . . . . . . . . . | . . . . . . . . . . | . . . . . . . . . . . . . . | . . . . . . . . . . . . . . . . . | . . . . . . . . . .N....... | R....W-- . . . . . . . . . . . . . .— SG.... | . . . . . . . . . . . |
| iPS:39 3176 | 21-225_27E7 | VH3\|3-33/D6\|6-6\|RF1/JH 6 | . . . . . . . . . . . . . . . . . . . . . . . . . . . . . . | . . . . . . . . . . | . . . . . . . . . . . . . . | . . . . . . . . . . . . . . . . . | . . . . . . . . . . . . . . . . . . | .D.YC.STSC........ ..PYY....... | . . . . . . . . . . . |
| iPS:39 3864 | 21-225_4C5 | VH3\|3-33/D6\|6-6\|RF1/JH 6 | . . . . . . . . . . . . . . . . . . . . . . . . . . . . . . | . . . . . . .VL. | . . . . . . . . . . . . . . | . . . . . . . . . . . . . . . . . | . . . . . . . . . . . . . . . . . . | .KYT..W— . . . . . . . . . . . . . . . . . . .D. . . . . | . . . . . . . . . . . |
| iPS:39 3902 | 21-225_14E10 | VH3\|3-33/D6\|6-6\|RF1/JH 6 | . . . . . . . . . . . . . . . . . . . . . . . . . . . . . . | D . . . . . . . . . | . . . . . . . . . . . . . . | . . . . . . . . . . . . . . . . . | . . . . . . . . . . . . . . . . . . | .KY...W— . . . . . . . . . . . . . . . . . . .D. . . . . | . . . . . . . . . . . |

| | | Germline | H_FR1 | H_CDR1 | H_FR2 | H_CDR2 | H_FR3 | H_CDR3 | H_FR4 |
|---|---|---|---|---|---|---|---|---|---|
| iPS:39 3908 | 21-225_10E9 | VH3\|3-33/D6\|6-6\|RF1/JH6 | ............<br>............<br>........ | N......VI. | ........<br>...... | ........<br>........<br>.... | ................ | .KY...W-<br>.................D.<br>.... | ......<br>..... |
| iPS:39 3916 | 21-225_2G4 | VH3\|3-33/D6\|6-6\|RF1/JH6 | ............<br>............<br>........ | .......V.. | ........<br>...... | ........<br>........<br>.... | ................ | .KY...W-<br>.................D.<br>.... | ..H...<br>..... |
| iPS:39 3950 | 21-225_3H10 | VH3\|3-33/D6\|6-6\|RF1/JH6 | ............<br>............<br>........ | .......V.. | ........<br>...... | ........<br>........<br>.... | ................ | .RY..GW-<br>.................D.<br>.L.. | ......<br>..... |
| iPS:39 3972 | 21-225_7C9 | VH3\|3-33/D6\|6-6\|RF1/JH6 | ............<br>............<br>....L... | N......V.. | ........<br>...... | ........<br>........<br>.... | ................ | .KY...W-<br>.................D.<br>.... | ......<br>..... |
| iPS:39 3978 | 21-225_4C12 | VH3\|3-33/D6\|6-6\|RF1/JH6 | ............<br>............<br>........ | .......V.. | ........<br>...... | ........<br>........<br>.... | ................ | .KY...W-<br>.................D.<br>.... | ..H...<br>..... |
| iPS:39 3986 | 21-225_7G4 | VH3\|3-33/D6\|6-6\|RF1/JH6 | ............<br>............<br>.......N | N......V.. | ........<br>...... | ........<br>........<br>.... | T............... | .KY..NW-<br>.................D.<br>.... | ......<br>..... |
| iPS:39 3996 | 21-225_15C11 | VH3\|3-33/D6\|6-6\|RF1/JH6 | ............<br>............<br>........ | D......V.. | ........<br>...... | ........<br>........<br>.... | ................ | .KY...W-<br>.................D.<br>.L.. | ......<br>..... |
| iPS:39 4041 | 21-225_5E5 | VH3\|3-33/D6\|6-6\|RF1/JH6 | ............<br>............<br>........ | N......V.. | ........<br>...... | ........<br>........<br>.... | ..............T. | .VY..GW-<br>.................D.<br>.... | ......<br>..... |
| | | Germline | H_FR1 | H_CDR1 | H_FR2 | H_CDR2 | H_FR3 | H_CDR3 | H_FR4 |
| | VH3\|3-33/D2\|2-8\|RF3/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DIVLMVY-------------------AIYFDY | WGQGTLVTVSS |
| iPS:42 6118 | 21-225_7A10 | VH3\|3-33/D2\|2-8\|RF3/JH4 | ............<br>............<br>.....N.. | .......... | ........<br>.....M.<br>.... | ........<br>........ | H............... | .ER.G--<br>.................-<br>-I... | ......<br>..... |
| iPS:39 3844 | 21-225_3G7 | VH3\|3-33/D2\|2-8\|RF3/JH4 | ............<br>............<br>.....N.. | N......... | ........<br>...... | ...H....<br>......V.<br>.... | ............V.... | .ER.G--<br>.................-<br>-I... | ......<br>..... |

| iPS | Clone | Germline | H_FR1 | H_CDR1 | H_FR2 | H_CDR2 | H_FR3 | H_CDR3 | H_FR4 |
|---|---|---|---|---|---|---|---|---|---|
| iPS:39 3852 | 21-225_12A10 | VH3\|3-33/D2\|2-8\|RF3/JH4 | ............<br>............<br>........ | ......... | ........<br>...... | ...H....<br>E.....T.<br>.... | ................. | .ER.G--<br>.................-<br>-I... | ...... |
| iPS:39 3868 | 21-225_9C11 | VH3\|3-33/D2\|2-8\|RF3/JH4 | ............<br>............<br>........ | ......... | ........<br>...... | ...H....<br>ET......<br>.... | ..............S... | .ER.G--<br>.................-<br>-I... | ...... |
| iPS:39 3900 | 21-225_10E12 | VH3\|3-33/D2\|2-8\|RF3/JH4 | ............<br>............<br>.....N.. | N......... | ....V...<br>...... | ...H....<br>......V.<br>.... | ..............S...<br>...........C... | .ER.G--<br>.................-<br>-I... | ...... |
| iPS:39 3920 | 21-225_1H12 | VH3\|3-33/D2\|2-8\|RF3/JH4 | ..S......<br>.....N.. | ......... | ........<br>...... | I..H....<br>......V.<br>.... | ................. | .ER.G--<br>.................-<br>-I... | ...... |
| iPS:39 3932 | 21-225_10F5 | VH3\|3-33/D2\|2-8\|RF3/JH4 | ............<br>............<br>.....N.. | ......... | ........<br>.....S | I..H....<br>......V.<br>.... | ................. | .ER.G--<br>.................-<br>-I... | ...... |
| **VH3\|3-33/D6\|6-6\|RF1/JH4** | | Germline | QVOLVES-<br>GGGVVQPGRSLR<br>LSCAASG-FIFS | S-----YGMH | WVRQAPGK<br>GLEWVA | VIWYD---<br>GSNKYYAD<br>SVKG | RFTISRDNSKNTLYLQM<br>NSLRAEDTAVYYCAR | EYSSS---------------<br>-------SYFDY | WGQGTL<br>VTVSS |
| iPS:42 6124 | 21-225_32D6 | VH3\|3-33/D6\|6-6\|RF1/JH4 | ............<br>............<br>........ | ......... | ........<br>.....T | ...H....<br>...A....<br>.... | ................. | .N................<br>.......Y.... | ...... |
| iPS:39 2922 | 21-225_30G4 | VH3\|3-33/D6\|6-6\|RF1/JH4 | ............<br>............<br>........ | ......... | ........R<br>...... | ........<br>.TD...V.<br>.... | .......S........ | .N................<br>.......Y.... | ...... |
| iPS:39 3002 | 21-225_30G1 | VH3\|3-33/D6\|6-6\|RF1/JH4 | ............<br>............<br>........ | Y......... | ........<br>...... | ...H....<br>......V.<br>.... | ................. | .N................<br>.......Y.... | ...... |
| iPS:39 3066 | 21-225_34D3 | VH3\|3-33/D6\|6-6\|RF1/JH4 | ............<br>............<br>........ | Y......... | ........<br>...... | ...H....<br>......V.<br>.... | ................. | .N................<br>.......F.... | ...... |
| iPS:39 3092 | 21-225_33C12 | VH3\|3-33/D6\|6-6\|RF1/JH4 | ............<br>............<br>........ | H......... | ........<br>...... | ........<br>........<br>.... | ................. | .N................<br>.......Y.... | ...... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:39 3100 | 21-225_36B8 | VH3\|3-33/D6\|6-6\|RF1/JH4 | ............<br>............<br>........ | N......... | ........<br>...... | ...H....<br>......G.<br>.... | ................<br>................ | .N..............<br>......Y.... | .....<br>..... |
| iPS:39 3122 | 21-225_33B2 | VH3\|3-33/D6\|6-6\|RF1/JH4 | ............<br>............<br>........ | N......... | ........<br>...... | ...H....<br>........<br>.... | ................<br>................ | .N..............<br>......Y.... | ..... |
| iPS:39 3134 | 21-225_34C2 | VH3\|3-33/D6\|6-6\|RF1/JH4 | ............<br>............<br>........ | N......V.. | ........<br>...... | L.......<br>........<br>.... | ................<br>................ | .N..............<br>......Y.... | ..... |
| iPS:39 3136 | 21-225_34D8 | VH3\|3-33/D6\|6-6\|RF1/JH4 | ............<br>............<br>........ | N......... | ........<br>...... | ........<br>........<br>.... | ................<br>................ | .N..............<br>......Y.... | ..... |
| | **Germline** | | **H_FR1** | **H_CDR1** | **H_FR2** | **H_CDR2** | **H_FR3** | **H_CDR3** | **H_FR4** |
| | **VH3\|3-30.3/D6\|6-19\|RF2/JH6** | | QVQLVES GGGVVQPGRSLR LSCAASG-FIFS | S YAMH | WVRQAPGK GLEWVA | VISYD GSNKYYAD SVKG | RFTISRDNSKNTLYLQM NSLRAEDTAVYYCAR | GIAVAGYY ------YYYGMDV | WGQGTT VTVSS |
| iPS:45 1135 | 21-225_64A11 | VH3\|3-30.3/D6\|6-19\|RF2/JH6 | ............<br>............<br>........ | N......G.. | ........<br>...... | ....V...<br>........<br>.... | .T..............<br>...... | RG...P--<br>................-<br>...... | ...... |
| | **Germline** | | **H_FR1** | **H_CDR1** | **H_FR2** | **H_CDR2** | **H_FR3** | **H_CDR3** | **H_FR4** |
| | **VH1\|1-08/D6\|6-19\|RF1/JH5** | | QVQLVQS GAEVKKPGASVK VSCKASG-YIFT | S YDIN | WVRQATGQ GLEWMG | WMNPN SGNTGYAQ KFQG | RVTMTRNTSISTAYMEL SSLRSEDTAVYYCAR | GYSSGW ------YNWFDP | WGQGTL VTVSS |
| iPS:45 1137 | 21-225_74A7 | VH1\|1-08/D6\|6-19\|RF1/JH5 | ............<br>............<br>........ | N......... | ........<br>...... | ........<br>........<br>.... | .........T...H...<br>.................V | SSGWN-<br>................<br>--.... | ...... |
| iPS:43 4285 | 21-225_57A11 | VH1\|1-08/D6\|6-19\|RF1/JH5 | ............<br>............<br>........ | N......... | ........<br>...... | ........<br>.V......<br>.... | ......D.........<br>.................I | SSGWN-<br>................<br>--.... | ...... |
| iPS:43 4287 | 21-225_57F12 | VH1\|1-08/D6\|6-19\|RF1/JH5 | ............<br>............<br>........ | N......... | ........<br>...... | ........<br>........<br>.... | .................<br>.................I | SSGWY-<br>................<br>--R... | ...... |
| iPS:43 4479 | 21-225_76H1 | VH1\|1-08/D6\|6-19\|RF1/JH5 | ............<br>............<br>........ | N......... | ....VP..<br>...... | ..H.....<br>........<br>.... | .................<br>.................I | SSGWY-<br>................<br>--.... | ...... |

| | | | | | | | | | |
|---|---|---|---|---|---|---|---|---|---|
| iPS:43 4481 | 21-225_74B10 | VH1\|1-08/D6\|6-19\|RF1/J H5 | ............ ............ ....F... | N......... | ........ ...... | ..H..... .....F.. .... | ................. ..............V | SSGWY- ................... --.... | ...... ...... |
| iPS:43 4483 | 21-225_74C12 | VH1\|1-08/D6\|6-19\|RF1/J H5 | ............ ............ ........ | N......... | ........ ...... | ........ ........ .... | ................. ..............I | SSGWY- ................... --.... | ...... ...... |
| iPS:43 4493 | 21-225_76F3 | VH1\|1-08/D6\|6-19\|RF1/J H5 | ............ ............ ........ | N......... | ........ ...... | ........ ........ .... | .........T...H.... ..............V | SSGWN- ................... --.... | ...... ...... |
| iPS:43 4509 | 21-225_76F5 | VH1\|1-08/D6\|6-19\|RF1/J H5 | ............ ............ ........ | N......... | ........ ...... | ........ ........ .... | ................. ..............S | SSGWY- ................... --.... | ...... ...... |
| iPS:43 4513 | 21-225_76A6 | VH1\|1-08/D6\|6-19\|RF1/J H5 | ............ ............ ........ | N......... | ........ ...... | ..H..... N....... .... | ................. N.............I | SSGWY- ................... --.... | ...... ....L |
| iPS:43 4515 | 21-225_74A5 | VH1\|1-08/D6\|6-19\|RF1/J H5 | ............ ............ ........ | N......... | ....VP.. ...... | ..H..... ........ .... | ................. ..............I | SSGWY- ................... --.... | ...... ...... |
| iPS:43 4525 | 21-225_76E8 | VH1\|1-08/D6\|6-19\|RF1/J H5 | ............ ............ .......P | N......... | ........ ...... | ........ ........ .... | ................. ..............V | SSGWH- ................... --.... | ...... ...A. |
| iPS:43 4529 | 21-225_76B9 | VH1\|1-08/D6\|6-19\|RF1/J H5 | ............ ............ ........ | N......... | ....VP.. ...... | ..H..... ........ .... | ................. ..............I | SSGWY- ................... --.... | ...... ...... |
| iPS:43 4575 | 21-225_77C7 | VH1\|1-08/D6\|6-19\|RF1/J H5 | ............ ............ .......P | N......... | ........ ...... | ........ ........ .... | ................. ..............V | SSGWH- ................... --.... | ...... ...A. |
| iPS:43 4583 | 21-225_74B6 | VH1\|1-08/D6\|6-19\|RF1/J H5 | ............ ............ ........ | N......... | ........ ...... | ..H..... N....... .... | ................. N.............I | SSGWY- ................... --.... | ...... ....L |
| iPS:43 4587 | 21-225_74G3 | VH1\|1-08/D6\|6-19\|RF1/J H5 | ............ ............ ........ | N......... | ....VP.. ...... | ..H..... ........ .... | ................. ..............I | SSGWY- ................... --.... | ...... ...... |
| iPS:43 4597 | 21-225_77C10 | VH1\|1-08/D6\|6-19\|RF1/J H5 | ............ ............ ........ | N......... | ........ ...... | ..H..... ........ .... | ................. ..............I | SSGWY- ................... --.... | ...... ...... |

| | | | | | | | | | |
|---|---|---|---|---|---|---|---|---|---|
| iPS:43 4603 | 21-225_77D11 | VH1\|1-08/D6\|6-19\|RF1/JH5 | ..........<br>..........<br>........ | N......... | ....VP..<br>......<br>.... | ..H.....<br>........<br>.... | ................<br>.............I | SSGWY-<br>...................<br>--.... | ......<br>.....................<br>...... |
| iPS:43 4613 | 21-225_77D12 | VH1\|1-08/D6\|6-19\|RF1/JH5 | ..........<br>..........<br>........ | N......... | ........<br>......<br>.... | ........<br>........<br>.... | ..........N......<br>.............I | SSGWY-<br>...................<br>--.... | ......<br>.....................<br>...... |
| iPS:43 4617 | 21-225_74B8 | VH1\|1-08/D6\|6-19\|RF1/JH5 | ..........<br>..........<br>........ | N......... | ........<br>......<br>.... | ..H.....<br>........<br>.... | ...L............<br>................V | SSGWY-<br>...................<br>--.... | ......<br>.....................<br>...... |
| iPS:43 4619 | 21-225_78C1 | VH1\|1-08/D6\|6-19\|RF1/JH5 | ..........<br>..........<br>........ | N......... | ........<br>......<br>.... | ..H.....<br>........<br>.... | ................<br>.............I | SSGWY-<br>...................<br>--.... | ......<br>.....................<br>...... |
| iPS:43 4639 | 21-225_74B7 | VH1\|1-08/D6\|6-19\|RF1/JH5 | ..........<br>..........<br>.......P | N......... | ........<br>......<br>.... | ........<br>........<br>.... | ................<br>...............V | SSGWH-<br>...................<br>--.... | ......<br>.....................<br>...A. |
| iPS:43 4653 | 21-225_74B5 | VH1\|1-08/D6\|6-19\|RF1/JH5 | ..........<br>..........<br>........ | N......... | ........<br>......<br>.... | ........<br>........<br>.... | .........T...H....<br>..............V | SSGWN-<br>...................<br>--.... | ......<br>.....................<br>...... |
| iPS:43 4655 | 21-225_78H12 | VH1\|1-08/D6\|6-19\|RF1/JH5 | ..........<br>..........<br>.......P | N......... | ........<br>......<br>.... | ........<br>........<br>.... | ................<br>...............V | SSGWH-<br>...................<br>--.... | ......<br>.....................<br>...A. |
| iPS:43 4675 | 21-225_79G6 | VH1\|1-08/D6\|6-19\|RF1/JH5 | ..........<br>..........<br>....F... | N......... | ........<br>......<br>.... | ..H.....<br>.....F..<br>.... | ................<br>...............V | SSGWY-<br>...................<br>--.... | ......<br>.....................<br>...... |
| iPS:43 4689 | 21-225_79G10 | VH1\|1-08/D6\|6-19\|RF1/JH5 | ..........<br>..........<br>........ | N......... | ........<br>......<br>.... | ........<br>........<br>.... | .........T...H...<br>...............V | SSGWN-<br>...................<br>--.... | ......<br>.....................<br>...... |
| iPS:43 4705 | 21-225_80A2 | VH1\|1-08/D6\|6-19\|RF1/JH5 | ..........<br>..........<br>........ | N......... | ....VP..<br>......<br>.... | ..H.....<br>........<br>.... | ................<br>.............I | SSGWY-<br>...................<br>--.... | ......<br>.....................<br>...... |
| iPS:43 4707 | 21-225_80D3 | VH1\|1-08/D6\|6-19\|RF1/JH5 | ..........<br>..........<br>........ | N......... | ........<br>......<br>.... | ..H.....<br>........<br>.... | ................<br>.............I | SSGWY-<br>...................<br>--.... | ......<br>.....................<br>...... |
| iPS:43 4731 | 21-225_80E9 | VH1\|1-08/D6\|6-19\|RF1/JH5 | ..........<br>..........<br>........ | N......... | ........<br>......<br>.... | ..H.....<br>........<br>.... | ................<br>.............I | SSGWY-<br>...................<br>--.... | ......<br>.....................<br>...... |

| iPS:43 4747 | 21-225_80C12 | VH1\|1-08/D6\|6-19\|RF1/JH5 | . . . . . . . . . . .<br>. . . . . . . . . . .<br>. . . . . . . | N . . . . . . . . . | . . . . . . . .<br>. . . . . .<br>. . . . | . . H . . . . .<br>N . . . . . . .<br>. . . . | . . . . . . . . . . . . . . . . .<br>N . . . . . . . . . . . . . I | SSGWY-<br>. . . . . . . . . . . . . . . . . .<br>— — . . . . | . . . . . .<br>. . . . L |
| iPS:43 4761 | 21-225_81E5 | VH1\|1-08/D6\|6-19\|RF1/JH5 | . . . . . . . . . . .<br>. . . . . . . . . . .<br>. . . . . . . | N . . . . . . . . . | . . . . . . . .<br>. . . . . .<br>. . . . | . . H . . . . .<br>. . . . . . .<br>. . . . | . . . . . . . . . T . . . H . . .<br>. . . . . . . . . . . . . . . V | SSGWN-<br>. . . . . . . . . . . . . . . . . .<br>— — . . . . | . . . . . .<br>. . . . . |
| iPS:43 4771 | 21-225_81F9 | VH1\|1-08/D6\|6-19\|RF1/JH5 | . . . . . . . . . . .<br>. . . . . . . . . . .<br>. . . . . . . | N . . . . . . . . . | . . . . . . . .<br>. . . . . .<br>. . . . | . . H . . . . .<br>. . . . . . .<br>. . . . | . . . . . . . . . . . . . . . . .<br>. . . . . . . . . . . . . . . I | SSGWY-<br>. . . . . . . . . . . . . . . . . .<br>— — . . . . | . . . . . .<br>. . . . . |
| iPS:43 4793 | 21-225_82A5 | VH1\|1-08/D6\|6-19\|RF1/JH5 | . . . . . . . . . . .<br>. . . . . . . . . . .<br>. . . . . . . | N . . . . . . . . . | . . . . . . . .<br>. . . . . .<br>. . . . | . . H . . . . .<br>N . . . . . . .<br>. . . . | . . . . . . . . . . . . . . . . .<br>N . . . . . . . . . . . . . I | SSGWY-<br>. . . . . . . . . . . . . . . . . .<br>— — . . . . | . . . . . .<br>. . . . L |
| iPS:43 4797 | 21-225_82G5 | VH1\|1-08/D6\|6-19\|RF1/JH5 | . . . . . . . . . . .<br>. . . . . . . . . . .<br>. . . . . . . | N . . . . . . . . . | . . . . VP . .<br>. . . . . .<br>. . . . | . . H . . . . .<br>. . . . . . .<br>. . . . | . . . . . . . . . . . . . . . . .<br>. . . . . . . . . . . . . . . I | SSGWY-<br>. . . . . . . . . . . . . . . . . .<br>— — . . . . | . . . . . .<br>. . . . . |
| iPS:43 4805 | 21-225_82D9 | VH1\|1-08/D6\|6-19\|RF1/JH5 | . . . . . . . . . . .<br>. . . . . . . . . . .<br>. . . . . . . | N . . . . . . . . . | . . . . VP . .<br>. . . . . .<br>. . . . | . . H . . . . .<br>. . . . . . .<br>. . . . | . . . . . . . . . . . . . . . . .<br>. . . . . . . . . . . . . . . I | SSGWY-<br>. . . . . . . . . . . . . . . . . .<br>— — . . . . | . . . . . .<br>. . . . . |
| iPS:43 4813 | 21-225_82C12 | VH1\|1-08/D6\|6-19\|RF1/JH5 | . . . . . . . . . . .<br>. . . . . . . . . . .<br>. . . . . . . | N . . . . . . . . . | . . . . . . . .<br>. . . . . .<br>. . . . | . . H . . . . .<br>N . . . . . . .<br>. . . . | . . . . . . . . . . . . . . . . .<br>N . . . . . . . . . . . . . I | SSGWY-<br>. . . . . . . . . . . . . . . . . .<br>— — . . . . | . . . . . .<br>. . . . L |
| iPS:43 4825 | 21-225_83C2 | VH1\|1-08/D6\|6-19\|RF1/JH5 | . . . . . . . . . . .<br>. . . . . . . . . . .<br>. . . . . . . | N . . . . . . . . . | . . . . VP . .<br>. . . . . .<br>. . . . | . . H . . . . .<br>. . . . . . .<br>. . . . | . . . . . . . . . . . . . . . . .<br>. . . . . . . . . . . . . . . I | SSGWY-<br>. . . . . . . . . . . . . . . . . .<br>— — . . . . | . . . . . .<br>. . . . . |
| iPS:43 4827 | 21-225_83F3 | VH1\|1-08/D6\|6-19\|RF1/JH5 | . . . . . . . . . . .<br>. . . . . . . . . . .<br>. . . . . . . | N . . . . . . . . . | . . . . . . . .<br>. . . . . .<br>. . . . | . . H . . . . .<br>. . . . . . .<br>. . . . | . . . . . . . . . . . . . . . . .<br>. . . . . . . . . . . . . . . I | SSGWY-<br>. . . . . . . . . . . . . . . . . .<br>— — . . . . | . . . . . .<br>. . . . . |
| iPS:43 4829 | 21-225_83G3 | VH1\|1-08/D6\|6-19\|RF1/JH5 | . . . . . . . . . . .<br>. . . . . . . . . . .<br>. . . . . . . | N . . . . . . . . . | . . . . . . . .<br>. . . . . .<br>. . . . | . . H . . . . .<br>. . . . . . .<br>. . . . | . . . . . . . . . . . . . . . . .<br>. . . . . . . . . . . . . . . I | SSGWY-<br>. . . . . . . . . . . . . . . . . .<br>— — . . . . | . . . . . .<br>. . . . . |
| iPS:43 4833 | 21-225_83C5 | VH1\|1-08/D6\|6-19\|RF1/JH5 | . . . . . . . . . . .<br>. . . . . . . . . . .<br>. . . . . . . | N . . . . . . . . . | . . . . VP . .<br>. . . . . .<br>. . . . | . . H . . . . .<br>. . . . . . .<br>. . . . | . . . . . . . . . . . . . . . . .<br>. . . . . . . . . . . . . . . I | SSGWY-<br>. . . . . . . . . . . . . . . . . .<br>— — . . . . | . . . . . .<br>. . . . . |
| iPS:43 4841 | 21-225_83G7 | VH1\|1-08/D6\|6-19\|RF1/JH5 | . . . . . . . . . . .<br>. . . . . . . . . . .<br>. . . . . . . P | N . . . . . . . . . | . . . . . . . .<br>. . . . . .<br>. . . . | . . H . . . . .<br>. . . . . . .<br>. . . . | . . . . . . . . . . . . . . . . .<br>. . . . . . . . . . . . . . . V | SSGWH-<br>. . . . . . . . . . . . . . . . . .<br>— — . . . . | . . . . . .<br>. . . A . |

| | | | | | | | | | | |
|---|---|---|---|---|---|---|---|---|---|---|
| iPS:43 4863 | 21-225_84G7 | VH1\|1-08/D6\|6-19\|RF1/JH5 | ............ ............ .......P | N......... | | ........ ...... | ........ ........ ...D | .................. .................V | SSGWH- .................. --.... | ...... ...A. |
| iPS:43 4877 | 21-225_85H2 | VH1\|1-08/D6\|6-19\|RF1/JH5 | ............ ............ ........ | N......... | | ........ ...... | ..H..... ........ .... | ...L......... .................V | SSGWY- .................. --.... | ...... .... |
| iPS:43 4883 | 21-225_85B5 | VH1\|1-08/D6\|6-19\|RF1/JH5 | ............ ............ ........ | N......... | | ....VP.. ...... | ..H..... ........ .... | .................. ...............I | SSGWY- .................. --.... | ...... .... |
| iPS:43 4911 | 21-225_85D11 | VH1\|1-08/D6\|6-19\|RF1/JH5 | ............ ............ ........ | N......... | | ....VP.. ...... | ..H..... .... | .................. ...............I | SSGWY- .................. --.... | ...... .... |
| iPS:43 4935 | 21-225_86E9 | VH1\|1-08/D6\|6-19\|RF1/JH5 | ............ ............ ........ | N......... | | ......... ...... | ........ .... | ......S..T...H... .................V | SSGWS- .................. --.... | ...... .... |
| iPS:43 4957 | 21-225_87A10 | VH1\|1-08/D6\|6-19\|RF1/JH5 | ............ ............ ........ | N......... | | ........ ...... | ..H..... N....... .... | N.............I | SSGWY- .................. --.... | ...... ....L |
| iPS:43 4971 | 21-225_88G2 | VH1\|1-08/D6\|6-19\|RF1/JH5 | ............ ............ ........ | N......... | | ........ ...... | ..H..... ........ .... | .................. ...............I | SSGWY- .................. --.... | ...... .... |
| iPS:43 5051 | 21-225_90D9 | VH1\|1-08/D6\|6-19\|RF1/JH5 | ............ ............ .......P | N......... | | ........ ...... | ........ .... | .................. .................V | SSGWH- .................. --.... | ...... ...A. |
| iPS:43 5071 | 21-225_91F1 | VH1\|1-08/D6\|6-19\|RF1/JH5 | ............ ............ ........ | N......... | | ........ ...... | ..H..... ........ .... | .................. ...............I | SSGWY- .................. --.... | ...... .... |
| iPS:43 5087 | 21-225_91G8 | VH1\|1-08/D6\|6-19\|RF1/JH5 | ............ ............ ........ | N......... | | ........ ...... | ..H..... ........ .... | .................. ...............I | SSGWY- .................. --.... | ...... .... |
| iPS:43 5203 | 21-225_75A7 | VH1\|1-08/D6\|6-19\|RF1/JH5 | ............ ............ .......P | N......... | | ........ ...... | ........ ...D | .................. .................V | SSGWH- .................. --.... | ...... ...A. |
| iPS:43 5211 | 21-225_94E11 | VH1\|1-08/D6\|6-19\|RF1/JH5 | ............ ............ ........ | N......... | | ........ ...... | ........ .... | .........T...H... .................V | SSGWK- .................. --.... | ...... .... |

| iPS:43 5227 | 21-225_95G4 | VH1\|1-08/D6\|6-19\|RF1/JH5 | . . . . . . . . . . . | N . . . . . . . . | . . . . . . . . | . . . . . . . . | . . . . . . . . .T. . .H. . . | SSGWN- | . . . . . . |
|---|---|---|---|---|---|---|---|---|---|
| | | | . . . . . . . . . . . | | . . . . . . | . . . . . . . . | . . . . . . . . . . . . . . . .V | . . . . . . . . . . . . . . . . . . | . . . . . |
| | | | . . . . . . . | | . . . . | | | ── . . . . | |
| iPS:43 5245 | 21-225_95E12 | VH1\|1-08/D6\|6-19\|RF1/JH5 | . . . . . . . . . . . | N . . . . . . . . | . . . . . . . . | . . . . . . . . | . . . . . . . . . . . . . . . . | SSGWY- | . . . . . . |
| | | | . . . . . . . . . . . | | . . . . . . | . . . . . . . . | . . . . . . . . . . . . . . . .I | . . . . . . . . . . . . . . . . . . | . . . . . |
| | | | . . . . . . . | | . . . . | | | ── . . . . | |
| iPS:43 5247 | 21-225_96G1 | VH1\|1-08/D6\|6-19\|RF1/JH5 | . . . . . . . . . . . | N . . . . . . . . | . . . . . . . . | . .H . . . . . | . . . . . . . . . . . . . . . . | SSGWY- | . . . . . . |
| | | | . . . . . . . . . . . | | . . . . . . | N . . . . . . . | N . . . . . . . . . . . . . .I | . . . . . . . . . . . . . . . . . . | . . . .L |
| | | | . . . . . . . | | . . . . | . . . . | | ── . . . . | |
| iPS:43 5249 | 21-225_96E2 | VH1\|1-08/D6\|6-19\|RF1/JH5 | . . . . . . . . . . . | N . . . . . . . . | . . . . . . . . | . .H . . . . . | . . .L . . . . . . . . . . . . | SSGWY- | . . . . . . |
| | | | . . . . . . . . . . . | | . . . . . . | . . . . . . . . | . . . . . . . . . . . . . . .V | . . . . . . . . . . . . . . . . . . | . . . . |
| | | | . . . . . . . | | . . . . | | | ── . . . . | |
| iPS:43 5255 | 21-225_96D5 | VH1\|1-08/D6\|6-19\|RF1/JH5 | . . . . . . . . . . . | N . . . . . . . . | . . . . . . . . . | . . . . . . . . | . . . . . . .S . .T . . .H . . . | SSGWS- | . . . . . . |
| | | | . . . . . . . . . . . | | . . . . . . | . . . . . . . . | . . . . . . . . . . . . . . . .V | . . . . . . . . . . . . . . . . . . | . . . . |
| | | | . . . . . . . | | . . . . | | | ── . . . . | |
| iPS:43 5279 | 21-225_97H4 | VH1\|1-08/D6\|6-19\|RF1/JH5 | . . . . . . . . . . . | N . . . . . . . . | . . . . . . . . | . .H . . . . . | . . . . . . . . . . . . . . . . | SSGWY- | . . . . . . |
| | | | . . . . . . . . . . . | | . . . . . . | . . . . . . . . | . . . . . . . . . . . . . . . .I | . . . . . . . . . . . . . . . . . . | . . . . |
| | | | . . . . . . . | | . . . . | | | ── . . . . | |
| iPS:43 5327 | 21-225_147G6 | VH1\|1-08/D6\|6-19\|RF1/JH5 | . . . . . . . . . . . | N . . . . . . . . | . . . . . . . . | . .H . . . . . | . . . . . . . . . . . . . . . . | SSGWY- | . . . . . . |
| | | | . . . . . . . . . . . | | . . . . . . | . . . . . . . . | . . . . . . . . . . . . . . . .Y | . . . . . . . . . . . . . . . . . . | . . . . |
| | | | . . . . . . . | | . . . . | | | ── . . . . | |
| iPS:43 5437 | 21-225_152F4 | VH1\|1-08/D6\|6-19\|RF1/JH5 | . . . . . . . . . . . | N . . . . . . . . | . . . . . . . . | . .H . . . . . | . . . . . . . . . . . . . . . . | SSGWY- | . . . . . . |
| | | | . . . . . . . . . . . | | . . . . . . | . . . . . . . . | . . . . . .D . . . . . . . .Y | . . . . . . . . . . . . . . . . . . | . . . . |
| | | | . . . . . . . | | . . . . | | | ── . . . . | |
| iPS:43 5701 | 21-225_170F6 | VH1\|1-08/D6\|6-19\|RF1/JH5 | . . . . . . . . . . . | N . . . . . . . . | . . . . . . . . | . . . . . . . . | . . . . . . .H . . . . . . . . . | SSGWY- | . . . . . . |
| | | | . . . . . . . . . . . | | . . . . . . | . . . . . . . . | . . . . . . . . . . . . . . . .I | . . . . . . . . . . . . . . . . . . | . . . . |
| | | | . . . . . . . | | . . . . | . . .D | | ── . . . . | |
| iPS:43 5737 | 21-225_174G5 | VH1\|1-08/D6\|6-19\|RF1/JH5 | . . . . . . . . . . . | N . . . . . . . . | . . . . . . . . | . .H . . . . . | . . . . . . . . . . . . . . . . | SSGWY- | . . . . . . |
| | | | . . . . . . . . . . . | | . . . . . . | . . . . . . . . | . . . . . . . . . . . . . . . .V | . . . . . . . . . . . . . . . . . . | . . . . |
| | | | . . . . . . . | | . . . . | | | ── . . . . | |
| iPS:43 6544 | 21-225_224H5 | VH1\|1-08/D6\|6-19\|RF1/JH5 | . . . . . . . . . . . | N . . . . . . . . | . . . . . . . . | . . . . . . . . | . . . . . . . . . . . . . . . . | SSGWN- | . . . . . . |
| | | | . . . . . . . . . . . | | . . . . . . | . . . . . . . . | . . . . . . . . . . . . . . . .S | . . . . . . . . . . . . . . . . . . | . . . . |
| | | | . . . . . . . | | . . . . | | | ── . . . . | |
| iPS:43 6570 | 21-225_225F4 | VH1\|1-08/D6\|6-19\|RF1/JH5 | . . . . . . . . . . . | N . . . . . . . . | . . . . . . . . | . .H . . . . . | .L . . . . . . . . . . .V . . . . | SSGWY- | . . . . . . |
| | | | . . . . . . . . . . . | | . . . . . . | . .S . . . . . | N . . . . . . . . . . . . . .S | . . . . . . . . . . . . . . . . . . | . . . . . |
| | | | . . . . . . . | | . . . . | | | ── . . . . | |

| | | | H_FR1 | H_CDR1 | H_FR2 | H_CDR2 | H_FR3 | H_CDR3 | H_FR4 |
|---|---|---|---|---|---|---|---|---|---|
| IPS:43 6644 | 21-225_227G9 | VH1\|1-08\|D6\|6-19\|RF1\|JH5 | . . . . . . . . . . . .<br>. . . . . . . . . . . R<br>. . . . . . . . | N . . . . . . . . . | . . . . . . . R<br>. . . . . . | . . Y . . . . .<br>. . . . . . . .<br>. . . . | . . . . . . . . . . . . . . . . .<br>. . . . . . . . . . . . . . . . L | SSGWY-<br>. . . . . . . . . . . . . . . . . . . .<br>- - . . . . | . . . . . .<br>. . . . |
| IPS:43 7322 | 21-225_75B1 | VH1\|1-08\|D6\|6-19\|RF1\|JH5 | . . . . . . . . . . . .<br>. . . . . . . . . . . .<br>. . . . . . . . | N . . . . . . . . . | . . . . VP . .<br>. . . . . . | . . H . . . . .<br>. . . . . . . .<br>. . . . | . . . . . . . . . . . . . . . . .<br>. . . . . . . . . . . . . . . . I | SSGWY-<br>. . . . . . . . . . . . . . . . . . . .<br>- - . . . . | . . . . . .<br>. . . . |
| IPS:43 7361 | 21-225_74C1 | VH1\|1-08\|D6\|6-19\|RF1\|JH5 | . . . . . . . . . . . .<br>. . . . . . . . . . . .<br>. . . . . . . . | N . . . . . . . . . | . . . . . . . .<br>. . . . . . | . . . . D . . .<br>. . . . . F . .<br>. . . . | . . . . . . . . . . . . . . . . .<br>. . . . . . . . . . . . . . . . V | SSGWY-<br>. . . . . . . . . . . . . . . . . . . .<br>- - . . . . | . . . . . .<br>. . . . |
| IPS:43 7363 | 21-225_74C10 | VH1\|1-08\|D6\|6-19\|RF1\|JH5 | . . . . . . . . . . . .<br>. . . . . . . . . . . .<br>. . . . . . . . | N . . . . . . . . . | . . . . . . . .<br>. . . . . . | . . . I . . . .<br>. . . . | . . . . . . . . . . . . . . . . .<br>. . . . . . . . . . . . . . . . I | SSGWY-<br>. . . . . . . . . . . . . . . . . . . .<br>- - . . . . | . . . . . .<br>. . . . |
| IPS:43 7379 | 21-225_74H2 | VH1\|1-08\|D6\|6-19\|RF1\|JH5 | . . . . . . . . . . . .<br>. . . . . . . . . . . .<br>. . . . F . . . | N . . . . . . . . . | . . . . . . . .<br>. . . . . . | . . H . . . . .<br>. . . . . F . .<br>. . . . | . . . . . . . . . . . . . . . . .<br>. . . . . . . . . . . . . . . . V | SSGWY-<br>. . . . . . . . . . . . . . . . . . . .<br>- - . . . . | . . . . . .<br>. . . . |
| IPS:44 6094 | 21-225_77E1 | VH1\|1-08\|D6\|6-19\|RF1\|JH5 | . . . . . . . . . . . .<br>. . . . . . . . . . . .<br>. . . . . . . P | N . . . . . . . . . | . . . . . . . .<br>. . . . . . | . . . . . . . .<br>. . . . | . . . . . . . . . . . . . . . . .<br>. . . . . . . . . . . . . . . . V | SSGWH-<br>. . . . . . . . . . . . . . . . . . . .<br>- - . . . . | . . . . . .<br>. . . A . |
| IPS:45 1129 | 21-225_94D2 | VH1\|1-08\|D6\|6-19\|RF1\|JH5 | . . . . . . . . . . . .<br>. . . . . . . . . . . .<br>. . . . F . . . | N . . . . . . . . . | . . . . . . . .<br>. . . . . . | . . H . . . . .<br>. . . . . F . .<br>. . . . | . . . . . . . . . . . . . . . . .<br>. . . . . . . . . . . . . . . . V | SSGWY-<br>. . . . . . . . . . . . . . . . . . . .<br>- - . . . . | . . . . . .<br>. . . . |
| IPS:45 1133 | 21-225_95H4 | VH1\|1-08\|D6\|6-19\|RF1\|JH5 | . . . . . . . . . . . .<br>. . . . . . . . . . . .<br>. . . . . . . . | N . . . . . . . . . | . . . . . . . .<br>. . . . . . | . . . . . . . .<br>. . . . | . . . . . . . . . . T . . . H . . . .<br>. . . . . . . . . . . . . . . . V | SSGWN-<br>. . . . . . . . . . . . . . . . . . . .<br>- - . . . . | . . . . . .<br>. . . . |
| IPS:39 8510 | 21-225_25A3 | VH1\|1-08\|D6\|6-19\|RF1\|JH5 | . . . . . . . . . . . .<br>. . . . . . . . . . . .<br>. . . . . I . . | N . . . . . . . . . | . . . . . . . .<br>. . . . . . | . . H . . . . .<br>. . . . . . . .<br>. . . . | . . . . . W . . . . . . . N . . .<br>. . . . . . . . . . . . . . . . S | SSGWY-<br>. . . . . . . . . . . . . . . . . . . .<br>- - . . . . | . . . . . .<br>. . . . |
| IPS:39 8516 | 21-225_26A9 | VH1\|1-08\|D6\|6-19\|RF1\|JH5 | . . . . . . . . . . . .<br>. . . . . . . . . . . .<br>. . . . . . . . | N . . . . . . . . . | . . . . . . . .<br>. . . . . . | . . H . . . . .<br>. . . . . C . .<br>. . . . | . . . . . W . M . . . . . . . . .<br>. . . . . . . . . . . . . . . . S | SSGWY-<br>. . . . . . . . . . . . . . . . . . . .<br>- - . . . . | . . . . . .<br>. . . . |
| | | Germline | H_FR1 | H_CDR1 | H_FR2 | H_CDR2 | H_FR3 | H_CDR3 | H_FR4 |
| VH3\|3-30.3\|D5\|5-18\|RF3\|JH4 | | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | GYSYG-----------<br>------YYFDY | WGQGTL<br>VTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:45 1139 | 21-225_71A6 | VH3\|3-30.3/D5\|5-18\|RF3/JH4 | ............<br>............<br>........ | N......G.. | ........<br>......<br>.... | ........<br>...E....<br>.... | ................. | DHR..V............<br>......RGG... | ......<br>..... |
| | | Germline | H_FR1 | H_CDR1 | H_FR2 | H_CDR2 | H_FR3 | H_CDR3 | H_FR4 |
| | VH1\|1-18/D6\|6-19\|RF2/JH5 | | QVQLVQS-GAEVKKPGASVK VSCKASG-YIFT | S-----YGIS | WVRQAPGQ GLEWMG | WISAY NGNTNYAQ KLQG | RVTMTTDTSTSTAYMEL RSLRSDDTAVYYCAR | GIAVAG ------NWFDP | WGQGTL VTVSS |
| iPS:45 1143 | 21-225_66H11 | VH1\|1-18/D6\|6-19\|RF2/JH5 | ............<br>............<br>.......A | T......... | ........<br>...... | ........<br>........<br>.... | ................. | .E...-<br>................<br>.-V... | ......<br>..... |
| iPS:43 4361 | 21-225_65D5 | VH1\|1-18/D6\|6-19\|RF2/JH5 | ............<br>............<br>.......P | .......... | ........<br>...... | ........<br>S.......<br>.... | ...........F... | .E...-<br>................<br>.-V... | ......<br>..... |
| | | Germline | H_FR1 | H_CDR1 | H_FR2 | H_CDR2 | H_FR3 | H_CDR3 | H_FR4 |
| | VH3\|3-33/D4\|4-23\|RF2/JH6 | | QVQLVES-GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | DYGGNSYY---------- -----YYYGMDV | WGQGTT VTVSS |
| iPS:45 3445 | 21-225_148E10 | VH3\|3-33/D4\|4-23\|RF2/JH6 | ............<br>............<br>........ | .......... | ........<br>...... | ...F....<br>......V.<br>...D | ................. | .RVEG.GTP.........<br>....Y....... | ......<br>..... |
| iPS:43 6082 | 21-225_195D9 | VH3\|3-33/D4\|4-23\|RF2/JH6 | ............<br>............<br>........ | H......V.. | ........<br>...... | ........<br>.T......<br>.... | ................. | .WF.EGN-<br>...............-<br>...... | ......<br>..... |
| iPS:43 6118 | 21-225_196A10 | VH3\|3-33/D4\|4-23\|RF2/JH6 | ............<br>............<br>........ | H......V.. | ........<br>...... | ........<br>.T......<br>.... | ................. | .WF.EGN-<br>...............-<br>...... | ......<br>..... |
| iPS:43 6670 | 21-225_147D9 | VH3\|3-33/D4\|4-23\|RF2/JH6 | ............<br>............<br>........ | .......... | ........<br>...... | D..F....<br>......V.<br>...D | ................. | .RVEG.GTP.........<br>....Y....... | ......<br>..... |
| iPS:43 6720 | 21-225_151H6 | VH3\|3-33/D4\|4-23\|RF2/JH6 | ............<br>............<br>........ | D......... | ........<br>...... | L.......<br>........<br>.... | ................. | .DRSC.RTSC........<br>..PYY....L.. | ......<br>.I... |
| iPS:43 6726 | 21-225_152G5 | VH3\|3-33/D4\|4-23\|RF2/JH6 | ............<br>............<br>........ | D......... | ........<br>...... | L.......<br>........<br>.... | ................. | .DRSC.RTSC........<br>..PYY....L.. | ......<br>.I... |

| iPS:43 6732 | 21-225_152B12 | VH3\|3-33/D4\|4-23\|RF2/JH6 | ...V......... .......... ........ | D......... | ........ ...... | ........ .... | ................. | .DRSC.STSC........ ..PYY....L.. | ...... ...... |
|---|---|---|---|---|---|---|---|---|---|
| iPS:43 6734 | 21-225_153A8 | VH3\|3-33/D4\|4-23\|RF2/JH6 | ....M......... .......... ........ | D......... | ........ ...... | L....... ........ .... | ................. | .DRSC.RTSC........ ..PYY....L.. | ...... .I... |
| iPS:43 6736 | 21-225_153E8 | VH3\|3-33/D4\|4-23\|RF2/JH6 | .......... .......... ......L. | N......... | ........ ...... | ...F.... ......V. ...D | ................. | RVEG.GTP......... ....Y....... | ...... ...... |
| iPS:43 6756 | 21-225_146A10 | VH3\|3-33/D4\|4-23\|RF2/JH6 | ....E......... .......... ........ | G......... | ........ ....MT | L.R.... ..D.N... .... | ................. | .RVFC.STSCL........ ..SYY....... | ...... ...... |
| iPS:43 6766 | 21-225_158D10 | VH3\|3-33/D4\|4-23\|RF2/JH6 | .......... .......... ........ | .......... | ........ ...... | ........ ........ .... | ................. | .RVSC.STSC........ ..PYY....... | ...... ...... |
| iPS:43 6768 | 21-225_159H8 | VH3\|3-33/D4\|4-23\|RF2/JH6 | .......... .......... ........ | T......... | ........ ...... | ........ ........ .... | ................. | .RVSC.STSC........ ..PYY....... | ...... ...... |
| iPS:43 6770 | 21-225_160B12 | VH3\|3-33/D4\|4-23\|RF2/JH6 | .......... .......... ........ | .......... | ........ ...... | ........ ........ .... | ................. | .RVSC.STSC........ ..PYY....... | ...... ...... |
| iPS:43 6782 | 21-225_166G11 | VH3\|3-33/D4\|4-23\|RF2/JH6 | .......... .......... .......N | G......... | ........ ...... | ........ ........ .... | .......T.....F... ...T........... | .DRYC.SPTCH....... ..PYY....L.. | ...... ...... |
| iPS:43 6794 | 21-225_170F1 | VH3\|3-33/D4\|4-23\|RF2/JH6 | .......... .......... ........ | G........N | ........ ...... | I....... .N...... .... | ................. | .RVYC.STSCH....... ..PYY...A... | ...... ...... |
| iPS:43 6836 | 21-225_52H1 | VH3\|3-33/D4\|4-23\|RF2/JH6 | .......... .......... ........ | G......... | ........ ...... | ........ ........ .... | ................. | .RVYC.SSSCS....... ..YYY....... | ...... ...... |
| iPS:43 6922 | 21-225_78E9 | VH3\|3-33/D4\|4-23\|RF2/JH6 | .......... .......... ........ | .......... | ........ ...... | ........ .N..S... .... | .......I........ | .RDYC.STSC........ ..PYY....... | ...... ...... |
| iPS:43 6924 | 21-225_74B3 | VH3\|3-33/D4\|4-23\|RF2/JH6 | .......... .......... ........ | R......... | ........ ...... | .F...... ....D... .... | ................. | .RDYC.STSC........ ..PYY....... | ...... ...... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 6928 | 21-225_79E7 | VH3\|3-33/D4\|4-23\|RF2/J H6 | ............<br>............<br>........ | ..........<br><br> | ........<br>......<br>.... | ........<br>.N..S...<br> | ........I......... | .RDYC.STSC........<br>..PYY....... | ...... |
| iPS:43 6932 | 21-225_92A4 | VH3\|3-33/D4\|4-23\|RF2/J H6 | ............<br>............<br>........ | ..........<br><br> | ........<br>......<br>.... | ........<br>.N..S...<br> | ....Y..I......... | .RDYC.STSC........<br>..PYY....... | ...... |
| iPS:43 6936 | 21-225_97E6 | VH3\|3-33/D4\|4-23\|RF2/J H6 | ............<br>............<br>........ | ..........<br><br> | ........<br>......<br>.... | ........<br>.N..S...<br> | ........I......... | .RDYC.STSC........<br>..PYY....... | ...... |
| iPS:43 7190 | 21-225_225A9 | VH3\|3-33/D4\|4-23\|RF2/J H6 | ..........<br>....G......<br>........ | T.........<br><br> | ...L....<br>......<br>.... | ........<br><br> | .......I.Q....... | .NHYC.STSCS.......<br>..PYY..F.... | ...... |
| iPS:43 7254 | 21-225_149F2 | VH3\|3-33/D4\|4-23\|RF2/J H6 | ....G.A.....<br>............<br>........ | R.........<br><br> | ........<br>......<br>.... | F.......<br>..EN....<br> | ......V..R....... | .RVEG.GTP........<br>....Y....... | ...... |
| iPS:43 7256 | 21-225_150F11 | VH3\|3-33/D4\|4-23\|RF2/J H6 | ....G.A.....<br>............<br>........ | R.........<br><br> | ........<br>......<br>.... | F.......<br>..EN....<br> | ......V..R....... | .RVEG.GTP........<br>....Y....... | ...... |
| iPS:45 1110 | 21-225_74C9 | VH3\|3-33/D4\|4-23\|RF2/J H6 | ............<br>............<br>........ | ..........<br><br> | ........<br>......<br>.... | ........<br>.N..S...<br> | ........I......... | .RDYC.STSC........<br>..PYY....... | ...... |
| iPS:39 2589 | 21-225_27H2 | VH3\|3-33/D4\|4-23\|RF2/J H6 | ............<br>............<br>........ | G.........<br><br> | ........<br>......<br>.... | ........<br><br> | ..............F... | .RVYC.STSCS.......<br>..PYY....... | ...... |
| iPS:39 3166 | 21-225_27G6 | VH3\|3-33/D4\|4-23\|RF2/J H6 | ............<br>............<br>........ | G.........<br><br> | ........<br>......<br>.... | I.......<br>..K..N..<br> | ................ | .RVYC.STSCS.......<br>..PYY....... | ...... |
| iPS:39 3198 | 21-225_28A11 | VH3\|3-33/D4\|4-23\|RF2/J H6 | ............<br>............<br>........ | G.......L.<br><br> | ........<br>......<br>.... | L.......<br>.N.T....<br> | ................ | .RVYC.STSCS.......<br>..PYY....... | ...... |
| iPS:39 3204 | 21-225_8C12 | VH3\|3-33/D4\|4-23\|RF2/J H6 | ............<br>............<br>.......G | ..........<br><br> | ........<br>......<br>.... | L.......<br><br> | ................ | .RVSC.SSSCY.......<br>..PYY....... | ...... |

| | | Germline | H_FR1 | H_CDR1 | H_FR2 | H_CDR2 | H_FR3 | H_CDR3 | H_FR4 |
|---|---|---|---|---|---|---|---|---|---|
| | | VH1\|1-02\|D4\|4-11\|RF2/JH4 | QVQLVQS-GAEVKKPGASVK VSCKASG-YTFT | G-----YYMH | WVRQAPGQ GLEWMG | WINPN--- SGGTNYAQ KFQG | RVTMTRDTSISTAYMEL SRLRSDDTAVYYCAR | DYSNY----------- --------YFDY | WGQGTL VTVSS |
| iPS:45 3447 | 21-225_65F10 | VH1\|1-02/D4\|4-11\|RF2/JH4 | ............ ............ ........ | .......H.. | ......... ...... | ........ N...S... ...D | ..N.............. ................ | .SRS- ................... ...SW.. | ...... |
| iPS:43 4145 | 21-225_55B1 | VH1\|1-02/D4\|4-11\|RF2/JH4 | ............ ............ ........ | .......F. | ......... ...... | ..H..... NNA..... .... | ................ ...............K | .GRS- ................... ...S... | ...... |
| iPS:43 4277 | 21-225_57A7 | VH1\|1-02/D4\|4-11\|RF2/JH4 | ............ ............ ........ | .......HI. | ......... D..... | ........ NN...... .... | ................ ................ | .GRS- ................... ...G... | ...... |
| iPS:43 4389 | 21-225_66F11 | VH1\|1-02/D4\|4-11\|RF2/JH4 | ............ ............ ........ | .......H.. | ......... ...... | ........ N...H... ...D | W................. ................ | .SRS- ................... ...SW.. | ...... .S... |
| iPS:43 4423 | 21-225_70D1 | VH1\|1-02/D4\|4-11\|RF2/JH4 | ............ ............ ........ | .......H.. | ......... ...... | ........ .NA..... .... | ................ ................ | .SIS- ................... ...SW.. | ...... |
| iPS:43 7234 | 21-225_64E2 | VH1\|1-02/D4\|4-11\|RF2/JH4 | ............ ............ ........ | ......... | ......... ...... | ........ NN...... .... | ................ ................ | .G.S- ................... ...G... | ...... |
| | | Germline | H_FR1 | H_CDR1 | H_FR2 | H_CDR2 | H_FR3 | H_CDR3 | H_FR4 |
| | | VH4\|4-59/D7\|7-27\|RF1/JH4 | QVQLQES-GPGLVKPSEILS LTCTVSG-GSIS | S-----YYWS | WIRQPPGK GLEWIG | YIYY---- SGSTNYNP SLKS | RVTISVDTSKNQFSLKL SSVTAADTAVYYCAR | LTGY----------- --------FDY | WGQGTL VTVSS |
| iPS:45 3449 | 21-225_208A2 | VH4\|4-59/D7\|7-27\|RF1/JH4 | .......... .........N.. .......R | .......... | .....A.. ...... | R..T.... ....D... .... | .I.M............ ................ | GF.D............. .........W.. | ...... ..... |
| iPS:43 5451 | 21-225_152D10 | VH4\|4-59/D7\|7-27\|RF1/JH4 | .......... ............ ........ | N......... | .....A.. ...... | R.DT.... ..I..... .... | ...M............ T............... | EG.LGA........... .......TF... | ...... ..... |
| iPS:43 5467 | 21-225_153B9 | VH4\|4-59/D7\|7-27\|RF1/JH4 | .......... ............ ........ | .......... | .....A.. ...... | R.DT.... ..I..... .... | ...M............ T............... | EG.VGA........... .......TY... | ...... ..... |
| iPS:43 5545 | 21-225_158F4 | VH4\|4-59/D7\|7-27\|RF1/JH4 | .......... ............ ........ | ......HF.. | .....A.. ...... | R..T.... ..T...T. .... | ...M............ ................ | .SSG............. ........W... | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 5665 | 21-225_169F2 | VH4\|4-59/D7\|7-27\|RF1/JH4 | ...........<br>...........<br>........ | | ......A..<br>......<br>.... | R.DT....<br>..I.....<br>.... | ...M.I....S.I....<br>................ | EG.VGA............<br>.......TY... | ......<br>..... |
| iPS:43 5671 | 21-225_169H5 | VH4\|4-59/D7\|7-27\|RF1/JH4 | ...........<br>...........<br>........ | | ......A..<br>......<br>.... | R.DT....<br>..I.....<br>.... | ...M......S.I....<br>................ | EG.VGA............<br>.......TY... | ......<br>..... |
| iPS:43 6354 | 21-225_210G10 | VH4\|4-59/D7\|7-27\|RF1/JH4 | ...........<br>.........N..<br>.......R | | ......A..<br>......<br>.... | R..T....<br>....D...<br>.... | .I.M............<br>................ | GF.D.............<br>.........W.. | ......<br>..... |
| | VH4\|4-34/D4\|4-17\|RF2/JH6 | | QVQLQQW<br>GAGLLKPSETLS<br>LTCAVYG-GSFS | G----YYWS | WIRQPPGK<br>GLEWIG | EINH<br>SGSTNYNP<br>SLKS | RVTISVDTSKNQFSLKL<br>SSVTAADTAVYYCAR | DYGDYYY<br>-----YYYGMDV | WGQGTT<br>VTVSS |
| iPS:46 8810 | 21-225_74D5 | VH4\|4-34/D4\|4-17\|RF2/JH6 | ...........<br>...........<br>.N...P.. | ......C... | ........<br>...... | ...Y....<br>..R..F..<br>.... | ................ | ...G---<br>.................--<br>--... | ......<br>..... |
| iPS:46 8832 | 21-225_76H10 | VH4\|4-34/D4\|4-17\|RF2/JH6 | ...........<br>...........<br>.N...P.. | ......C... | ........<br>...... | ...Y....<br>..R..F..<br>.... | ................ | ...G---<br>.................--<br>--... | ......<br>..... |
| iPS:46 8834 | 21-225_94G10 | VH4\|4-34/D4\|4-17\|RF2/JH6 | ...........<br>...........<br>.N...P.. | ......C... | G.......<br>.R.... | ...Y....<br>..R..F..<br>.... | ................ | ...G---<br>.................--<br>--... | ......<br>..... |
| iPS:46 8838 | 21-225_80E12 | VH4\|4-34/D4\|4-17\|RF2/JH6 | ...........<br>...........<br>.N...P.. | ......C... | ........<br>...... | ...Y....<br>..R..F..<br>.... | ................ | ...G---<br>.................--<br>--... | ......<br>..... |
| iPS:46 8820 | 21-225_76E10 | VH4\|4-34/D4\|4-17\|RF2/JH6 | ...........<br>...........<br>.N...P.. | ......S... | ........<br>...... | ...Y....<br>..R..F..<br>.... | ................ | ...G---<br>.................--<br>--... | ......<br>..... |
| iPS:43 4473 | 21-225_76D1 | VH4\|4-34/D4\|4-17\|RF2/JH6 | ...........<br>...........<br>..S..... | ......C... | ........<br>...... | ........<br>..R.....<br>.... | ................ | ...,G---<br>.................--<br>--... | ......<br>..... |
| iPS:43 4495 | 21-225_74B2 | VH4\|4-34/D4\|4-17\|RF2/JH6 | ...........<br>.....P.....<br>........ | ......P... | ........<br>...... | ........<br>........<br>..T. | T......S...... | ...G---<br>.................--<br>--L.. | ......<br>..... |

3174

| iPS:43 4497 | 21-225_76A4 | VH4\|4-34/D4\|4-17\|RF2/J H6 | . . . . . . . . . . . .<br>. . . . . . . . . . . .<br>. H . . . . . . | . . . . . . C . . . | . . . . . . . .<br>. . . . . .<br>. . . . | . . . . . . . .<br>. . . . . . . .<br>. . . . | . . . . . . . . . . . . . . . . . . | . . . G - - -<br>. . . . . . . . . . . . . . . . - -<br>- - . . . | . . . . . . |
| iPS:43 4501 | 21-225_76G4 | VH4\|4-34/D4\|4-17\|RF2/J H6 | . . . . . . . . . . . .<br>. . . . . . . . . . . .<br>. H . . . . . . | . . . . . . C . . . | . . . . . . . .<br>. . . . . .<br>. . . . | . . . . . . . .<br>. . . . . . . .<br>. . . . | . . . . . . . . . . . . . . . . . . | . . . G - - -<br>. . . . . . . . . . . . . . . . - -<br>- - . . . | . . . . . . |
| iPS:43 4507 | 21-225_74C5 | VH4\|4-34/D4\|4-17\|RF2/J H6 | . . . . . . . . . . . .<br>. . . . . . . . . . . .<br>. . . . . . . | . . . . . . C . . . | . . . . . . . .<br>. . . . . .<br>. . . . | . . . . . . . .<br>. . C . . F . .<br>. . . . | . . . . . . . . . . . . . . . . . . | . . . G - - -<br>. . . . . . . . . . . . . . . . - -<br>- - . . . | . . . . . . |
| iPS:43 4523 | 21-225_75C3 | VH4\|4-34/D4\|4-17\|RF2/J H6 | . . . . . . G . . . P<br>. . . . . . . . . . . .<br>. . . . . . . | . . . . . . C . . . | . . . . . . . .<br>. . . . . .<br>. . . . | . . . Y . . . .<br>. . R . . . . .<br>. . . . | . . . . . . . . . . . . . . . . . . | . . . G - - -<br>. . . . . . . . . . . . . . . . - -<br>- - . . . | . . . . . . |
| iPS:43 4533 | 21-225_85F7 | VH4\|4-34/D4\|4-17\|RF2/J H6 | . . . . . . . . . . . .<br>. . . . . P . . . . . .<br>. . . . . . . | . . . . . . P . . . | . . . . . . . .<br>. . . . . .<br>. . . . | . . . . . . . .<br>. . . . . . . .<br>. . . . | T . . . . . . . . . . . . . . . | . . . G - - -<br>. . . . . . . . . . . . . . . . - -<br>- - L . . | . . . . . . |
| iPS:43 4547 | 21-225_74H5 | VH4\|4-34/D4\|4-17\|RF2/J H6 | . . . . . . . . . . . .<br>. . . . . . . . . . . .<br>. N . . . P . . | . . . . . . C . . . | . . . . . . . .<br>. . . . . .<br>. . . . | . . . . . . . .<br>. . R . . F . .<br>. . . . | . . . . . . . . . . . . . . . . . . | . . . G - - -<br>. . . . . . . . . . . . . . . . - -<br>- - . . . | . . . . . . |
| iPS:43 4559 | 21-225_74D11 | VH4\|4-34/D4\|4-17\|RF2/J H6 | . . . . . . . . . . . .<br>. . . . . . . . . . . .<br>. H . . . . . . | . . . . . . C . . . | . . . . . . . .<br>. . . . . .<br>. . . . | . . . . . . . .<br>. . . . . . . .<br>. . . . | . . . . . . . . . . . . . . . . . . | . . . G - - -<br>. . . . . . . . . . . . . . . . - -<br>- - . . . | . . . . . . |
| iPS:43 4561 | 21-225_77G1 | VH4\|4-34/D4\|4-17\|RF2/J H6 | . . . . . . . . . . . .<br>. . . . . . . . . . . .<br>. H . . . . . . | . . . . . . C . . . | . . . . . . . .<br>. . . . . .<br>. . . . | . . . . . . . .<br>. . . . . . . .<br>. . . . | . . . . . . . . . . . . . . . . . . | . . . G - - -<br>. . . . . . . . . . . . . . . . - -<br>- - . . . | . . . . . . |
| iPS:43 4565 | 21-225_75B10 | VH4\|4-34/D4\|4-17\|RF2/J H6 | . . . . . . . . . . . .<br>. . . . . . . . . K . D<br>. . . . . . . | . . . . . . . . . . | . . . . . . . .<br>. . . . . .<br>. . . . | . . . . . . . .<br>. . . . . . . .<br>. . . . | . . . . . . . . . . . . . . . . . . | . . . G - - -<br>. . . . . . . . . . . . . . . . - -<br>- - L . . | . . . . . . |
| iPS:43 4579 | 21-225_77F7 | VH4\|4-34/D4\|4-17\|RF2/J H6 | . . . . . . . . . . . .<br>. . . . . . . . . . . .<br>. H . . . . . . | . . . . . . C . . . | . . . . . . . .<br>. . . . . .<br>. . . . | . . . . . . . .<br>. . . . . . . .<br>. . . . | . . . . . . . . . . . . . . . . . . | . . . G - - -<br>. . . . . . . . . . . . . . . . - -<br>- - . . . | . . . . . . |
| iPS:43 4581 | 21-225_74B12 | VH4\|4-34/D4\|4-17\|RF2/J H6 | . . . . . . . . . . . .<br>. . . . . . . . . . . .<br>. H . . . . . . | . . . . . . C . . . | . . . . . . . .<br>. . . . . .<br>. . . . | . . . . . . . .<br>. . . . . . . .<br>. . . . | . . . . . . . . . . . . . . . . . . | . . . G - - -<br>. . . . . . . . . . . . . . . . - -<br>- - . . . | . . . . . . |
| iPS:43 4585 | 21-225_75A12 | VH4\|4-34/D4\|4-17\|RF2/J H6 | . . . . . . G . . . P<br>. . . . . . . . . . . .<br>. . . . . . . | . . . . . . C . . . | . . . . . . . .<br>. . . . . .<br>. . . . | . . . Y . . . .<br>. . R . . . . .<br>. . . . | . . . . . . . . . . . . . . . . . . | . . . G - - -<br>. . . . . . . . . . . . . . . . - -<br>- - . . . | . . . . . . |

| | | | | | | | | | |
|---|---|---|---|---|---|---|---|---|---|
| iPS:43 4595 | 21-225_77A10 | VH4\|4-34/D4\|4-17\|RF2/JH6 | ......G....P<br>...........<br>........ | ......C... | ........<br>...... | ...Y....<br>..R.....<br>.... | ................ | ...G---<br>................--<br>--... | ...... |
| iPS:43 4611 | 21-225_77C12 | VH4\|4-34/D4\|4-17\|RF2/JH6 | ...........<br>...........<br>.....A.. | ......S... | ....S...<br>...... | ...Y....<br>R.......<br>.... | ...............N. | ...G---<br>................--<br>--... | ...... |
| iPS:43 4657 | 21-225_79G1 | VH4\|4-34/D4\|4-17\|RF2/JH6 | ...........<br>...........<br>.H...... | ......C... | ........<br>...... | ........<br>.... | ................ | ...G---<br>................--<br>--... | ...... |
| iPS:43 4663 | 21-225_79F3 | VH4\|4-34/D4\|4-17\|RF2/JH6 | ...........<br>...........<br>.H...... | ......C... | ........<br>...... | ........<br>.... | ................ | ...G---<br>................--<br>--... | ...... |
| iPS:43 4687 | 21-225_75A5 | VH4\|4-34/D4\|4-17\|RF2/JH6 | ...........<br>...........<br>.H...... | ......C... | ........<br>...... | ........<br>.... | ................ | ...G---<br>................--<br>--... | ...... |
| iPS:43 4691 | 21-225_75G7 | VH4\|4-34/D4\|4-17\|RF2/JH6 | ...........<br>..........T<br>.....A.. | ......S... | ....S...<br>...... | ...Y....<br>R.......<br>.... | ...............N. | ...G---<br>................--<br>--... | ...... |
| iPS:43 4693 | 21-225_79F11 | VH4\|4-34/D4\|4-17\|RF2/JH6 | ...........<br>...........<br>.H...... | ......C... | ........<br>...... | ........<br>.... | ................ | ...G---<br>................--<br>--... | ...... |
| iPS:43 4699 | 21-225_79G12 | VH4\|4-34/D4\|4-17\|RF2/JH6 | ...........<br>...........<br>.H...... | ......C... | ........<br>...... | ........<br>.... | ................ | ...G---<br>................--<br>--... | ...... |
| iPS:43 4701 | 21-225_80A1 | VH4\|4-34/D4\|4-17\|RF2/JH6 | ...........<br>...........<br>.H...... | ......C... | ........<br>...... | ........<br>.... | ................ | ...G---<br>................--<br>--... | ...... |
| iPS:43 4703 | 21-225_80C1 | VH4\|4-34/D4\|4-17\|RF2/JH6 | ...........<br>...........<br>.H...... | ......C... | ........<br>...... | ........<br>.... | ................ | ...G---<br>................--<br>--... | ...... |
| iPS:43 4709 | 21-225_80E3 | VH4\|4-34/D4\|4-17\|RF2/JH6 | ...........<br>...........<br>.H...... | ......C... | ........<br>...... | ........<br>.... | ................ | ...G---<br>................--<br>--... | ...... |
| iPS:43 4715 | 21-225_80D5 | VH4\|4-34/D4\|4-17\|RF2/JH6 | ...........<br>.....P.....<br>........ | ......P... | ........<br>...... | ........<br>.... | T......M..... | ...G---<br>................--<br>--L.. | ...... |

| iPS:43 4725 | 21-225_80H7 | VH4\|4-34/D4\|4-17\|RF2/J H6 | . . . . . . . . . . .<br>. . . . . . . . . . .<br>. . v . . . . . | . . . . . . s . . . | . . . . . . . . .<br>. . . . . . | . . . Q . . . .<br>. . R . . . . .<br>. . . . | . . . . . . . . . . . . . . . . . . | . . . G - - -<br>. . . . . . . . . . . . . . . . . . --<br>--I . . | . . . . . . |
| iPS:43 4743 | 21-225_74A4 | VH4\|4-34/D4\|4-17\|RF2/J H6 | . . . . . . . . . . .<br>. . . . . . . . . . .<br>. H . . . . . . | . . . . . . C . . . | . . . . . . . . .<br>. . . . . . | . . . . . . . .<br>. . . . . . . .<br>. . . . | . . . . . . . . . . . . . . . . . . | . . . G - - -<br>. . . . . . . . . . . . . . . . . . --<br>-- . . . | . . . . . . |
| iPS:43 4751 | 21-225_80H12 | VH4\|4-34/D4\|4-17\|RF2/J H6 | . . . . . . . . . . .<br>. . . . . . . . . . .<br>. H . . . . . . | . . . . . . C . . . | . . . . . . . . .<br>. . . . . . | . . . . . . . .<br>. . . . . . . .<br>. . . . | . . . . . . . . . . . . . . . . . . | . . . G - - -<br>. . . . . . . . . . . . . . . . . . --<br>-- . . . | . . . . . . |
| iPS:43 4759 | 21-225_81C5 | VH4\|4-34/D4\|4-17\|RF2/J H6 | . . . . . . . . . . .<br>. . . . . . . . . . .<br>. H . . . . . . | . . . . . . C . . . | . . . . . . . . .<br>. . . . . . | . . . . . . . .<br>. . . . . . . .<br>. . . . | . . . . . . . . . . . . . . . . . . | . . . G - - -<br>. . . . . . . . . . . . . . . . . . --<br>-- . . . | . . . . . . |
| iPS:43 4773 | 21-225_75D9 | VH4\|4-34/D4\|4-17\|RF2/J H6 | . . . . . . . . . . .<br>. . . . . . . . . . .<br>. . . . . . . | . . . . . . P . . . | . . . . . . . . .<br>. . . . . . | . . . Y . . . .<br>R . . . . . . .<br>. . . . | . . . . . . . . . . . . . . . . .N. | . . . G - - -<br>. . . . . . . . . . . . . . . . . . --<br>-- . . . | . . . . . . |
| iPS:43 4777 | 21-225_81C11 | VH4\|4-34/D4\|4-17\|RF2/J H6 | . . . . . . . . . . .<br>. . . . . . . . . . .<br>. H . . . . . . | . . . . . . C . . . | . . . . . . . . .<br>. . . . . . | . . . . . . . .<br>. . . . . . . .<br>. . . . | . . . . . . . . . . . . . . . . . . | . . . G - - -<br>. . . . . . . . . . . . . . . . . . --<br>-- . . . | . . . . . . |
| iPS:43 4809 | 21-225_74F5 | VH4\|4-34/D4\|4-17\|RF2/J H6 | . . . . . . . . . . .<br>. . . . . . . . . . .<br>. H . . . . . . | . . . . . . C . . . | . . . . . . . . .<br>. . . . . . | . . . . . . . .<br>. . . . . . . .<br>. . . . | . . . . . . . . . . . . . . . . . . | . . . G - - -<br>. . . . . . . . . . . . . . . . . . --<br>-- . . . | . . . . . . |
| iPS:43 4821 | 21-225_83G1 | VH4\|4-34/D4\|4-17\|RF2/J H6 | . . . . . . . . . . .<br>. . . . . . . . . . .<br>. H . . . . . . | . . . . . . C . . . | . . . . . . . . .<br>. . . . . . | . . . . . . . .<br>. . . . . . . .<br>. . . . | . . . . . . . . . . . . . . . . . . | . . . G - - -<br>. . . . . . . . . . . . . . . . . . --<br>-- . . . | . . . . . . |
| iPS:43 4839 | 21-225_83B7 | VH4\|4-34/D4\|4-17\|RF2/J H6 | . . . . . . . . . . .<br>. . . . . . . . . . .<br>. H . . . . . . | . . . . . . C . . . | . . . . . . . .<br>. . . . . . | . . . . . . . .<br>. . . . . . . .<br>. . . . | . . . . . . . . . . . . . . . . . . | . . . G - - -<br>. . . . . . . . . . . . . . . . . . --<br>-- . . . | . . . . . . |
| iPS:43 4869 | 21-225_84E12 | VH4\|4-34/D4\|4-17\|RF2/J H6 | . . . . . . . . . . .<br>. . . . . . . . . . .<br>. . . . . . . | . . . . . . s . . . | . . . . . . . . .<br>. . . . . . | . . . Q . . . .<br>. . R . . . . .<br>. . . . | . . . . . . . . . . . . . . . . . . | . . . G - - -<br>. . . . . . . . . . . . . . . . . . --<br>--I . . | . . . . . . |
| iPS:43 4879 | 21-225_85A3 | VH4\|4-34/D4\|4-17\|RF2/J H6 | . . . . . . . . . . .<br>. . . . . . . . . . .<br>. H . . . . . . | . . . . . . C . . . | . . . . . . . . .<br>. . . . . . | . . . . . . . .<br>. . . . | . . . . . . . . . . . . . . . . . . | . . . G - - -<br>. . . . . . . . . . . . . . . . . . --<br>-- . . . | . . . . . . |
| iPS:43 4881 | 21-225_85B4 | VH4\|4-34/D4\|4-17\|RF2/J H6 | . . . . . . . . . . .<br>. . . . . . . . . . .<br>. H . . . . . . | . . . . . . C . . . | . . . . . . . .<br>. . . . . . | . . . . . . . .<br>. . . . | . . . . . . . . . . . . . . . . . . | . . . G - - -<br>. . . . . . . . . . . . . . . . . . --<br>-- . . . | . . . . . . |

| iPS | Clone | VH | | | | | | | |
|---|---|---|---|---|---|---|---|---|---|
| iPS:43 4887 | 21-225_85D6 | VH4|4-34/D4|4-17|RF2|JH6 | ......G....P<br>..........<br>........ | ......C... | ........<br>...... | ...V....<br>..R.....<br>.... | ................ | ...G---<br>...............--<br>--... | ...... |
| iPS:43 4895 | 21-225_74H7 | VH4|4-34/D4|4-17|RF2|JH6 | ..........<br>.....P.....<br>........ | ......P... | ........<br>...... | ........<br>........<br>.... | T............... | ...G---<br>................--<br>--L.. | ...... |
| iPS:43 4899 | 21-225_85B9 | VH4|4-34/D4|4-17|RF2|JH6 | ..........<br>..........<br>.N...P.. | ......C... | ........<br>...... | ........<br>..R..F..<br>.... | ................ | ...G---<br>...............--<br>--... | ...... |
| iPS:43 4907 | 21-225_85G10 | VH4|4-34/D4|4-17|RF2|JH6 | ......R.....<br>..........<br>........ | ......C... | ........<br>...... | ........<br>..I.....<br>.... | T............... | ...G---<br>................--<br>--L.. | ...... |
| iPS:43 4913 | 21-225_86C1 | VH4|4-34/D4|4-17|RF2|JH6 | ..........<br>..........<br>.H...... | ......C... | ........<br>...... | ........<br>........<br>.... | ................ | ...G---<br>...............--<br>--... | ...... |
| iPS:43 4921 | 21-225_86E4 | VH4|4-34/D4|4-17|RF2|JH6 | ..........<br>..........<br>.H...... | ......C... | ........<br>...... | ........<br>........<br>.... | ................ | ...G---<br>...............--<br>--... | ...... |
| iPS:43 4939 | 21-225_86C11 | VH4|4-34/D4|4-17|RF2|JH6 | ..........<br>..........<br>.H...... | ......C... | ........<br>...... | ........<br>........<br>.... | ................ | ...G---<br>...............--<br>--... | ...... |
| iPS:43 4943 | 21-225_87H1 | VH4|4-34/D4|4-17|RF2|JH6 | .....P.....<br>..........<br>........ | .......... | ........<br>...... | ........<br>..R.....<br>.... | ...........D...... | ...G---<br>...............--<br>--L.. | ...... |
| iPS:43 4945 | 21-225_87E5 | VH4|4-34/D4|4-17|RF2|JH6 | ..........<br>..........<br>.H...... | ......C... | ........<br>...... | ........<br>........<br>.... | ................ | ...G---<br>...............--<br>--... | ...... |
| iPS:43 4955 | 21-225_87C9 | VH4|4-34/D4|4-17|RF2|JH6 | ..........<br>..........<br>.H...... | ......C... | ........<br>...... | ........<br>........<br>.... | ................ | ...G---<br>...............--<br>--... | ...... |
| iPS:43 4961 | 21-225_87A12 | VH4|4-34/D4|4-17|RF2|JH6 | ..........<br>..........<br>.H...... | ......C... | ........<br>...... | ........<br>........<br>.... | ................ | ...G---<br>................--<br>--... | ...... |
| iPS:43 4969 | 21-225_88H1 | VH4|4-34/D4|4-17|RF2|JH6 | ..........<br>..........<br>.H...... | ......C... | ........<br>...... | ........<br>........<br>.... | ................ | ...G---<br>...............--<br>--... | ...... |

| | | | | | | | | | |
|---|---|---|---|---|---|---|---|---|---|
| iPS:43 4981 | 21-225_88E7 | VH4\|4-34/D4\|4-17\|RF2\|J H6 | ............ ............ .H...... | ......C... | ........ ...... | ........ ........ .... | ................. | ...G--- ...............--...--... | ...... |
| iPS:43 4983 | 21-225_88F7 | VH4\|4-34/D4\|4-17\|RF2\|J H6 | ............ ............ .H...... | ......C... | ........ ...... | ........ ........ .... | ................. | ...G--- ...............--...--... | ...... |
| iPS:43 4995 | 21-225_88G9 | VH4\|4-34/D4\|4-17\|RF2\|J H6 | ............ ............ .H...... | ......C... | ........ ...... | ........ ........ .... | ................. | ...●G--- ...............--...--... | ...... |
| iPS:43 4999 | 21-225_75A8 | VH4\|4-34/D4\|4-17\|RF2\|J H6 | ............ ............ .H...... | ......C... | ........ ...... | ........ ........ .... | ................. | ...G--- ...............--...--... | ...... |
| iPS:43 5013 | 21-225_89D5 | VH4\|4-34/D4\|4-17\|RF2\|J H6 | ............ ............ .H...... | ......C... | ........ ...... | ........ ........ .... | ................. | ...G--- ...............--...--... | ...... |
| iPS:43 5015 | 21-225_89H5 | VH4\|4-34/D4\|4-17\|RF2\|J H6 | ............ ............ ........ | ......C... | ........ ...... | ...Y.... .....F.. .... | ......A........... | ...G--- ...............--...--... | ...... |
| iPS:43 5025 | 21-225_89E10 | VH4\|4-34/D4\|4-17\|RF2\|J H6 | ............ ............ .H...... | ......C... | ........ ...... | ........ ........ .... | ................. | ...●G--- ...............--...--... | ...... |
| iPS:43 5029 | 21-225_89A11 | VH4\|4-34/D4\|4-17\|RF2\|J H6 | .....P...... ............ ........ | .......... | ........ ...... | ........ .R.S... .... | ................. | ...●G--- ...............--...--L.. | ...... |
| iPS:43 5039 | 21-225_90G4 | VH4\|4-34/D4\|4-17\|RF2\|J H6 | ............ ............ .H...... | ......C... | ........ ...... | ........ ........ .... | ................. | ...G--- ...............--...--... | ...... |
| iPS:43 5041 | 21-225_90A5 | VH4\|4-34/D4\|4-17\|RF2\|J H6 | ............ ............ .H...... | ......C... | ........ ...... | ........ ........ .... | ................. | ...G--- ...............--...--... | ...... |
| iPS:43 5043 | 21-225_90G5 | VH4\|4-34/D4\|4-17\|RF2\|J H6 | ............ ............ .H...... | ......C... | ........ ...... | ........ ........ .... | ................. | ...●G--- ...............--...--... | ...... |
| iPS:43 5055 | 21-225_90F10 | VH4\|4-34/D4\|4-17\|RF2\|J H6 | ............ ............ .H...... | ......C... | ........ ...... | ........ ........ .... | ................. | ...G--- ...............--...--... | ...... |

| | | | | | | | | | |
|---|---|---|---|---|---|---|---|---|---|
| iPS:43 5073 | 21-225_91B2 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5075 | 21-225_91B3 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5077 | 21-225_91F3 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5079 | 21-225_91B4 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5089 | 21-225_91E9 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5097 | 21-225_92B1 | VH4\|4-34/D4\|4-17\|RF2\|J H6 | . . . . . . . . . . . .<br>. . . . . . . . . . . .<br>. . . . . . . . | . . . . . .S. . . . | . . . . . . . .<br>. . . . . .<br> | . . .Y. . . .<br>R. . . . . . .<br>. . . . | . .A. . . . . . . . . . . . .N.<br>T. . . . . . . . . . . . . . . | . . .G---<br>. . . . . . . . . . . . . . . . . . .--<br>--L. . | . . . . . . |
| iPS:43 5111 | 21-225_92D6 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5115 | 21-225_77C5 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5171 | 21-225_93C2 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5177 | 21-225_93E4 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5195 | 21-225_94D3 | VH4\|4-34/D4\|4-17\|RF2\|J H6 | . . . . . .G. . . .P<br>. . . . . . . . . . . .<br>. . . . . . . . | . . . . . .C. . . . | . . . . . . . .<br>. . . . . .<br> | . . .Y. . . .<br>. .R. . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |
| iPS:43 5217 | 21-225_94F12 | VH4\|4-34/D4\|4-17\|RF2\|J H6 | . . . . . . . . . . . .<br>. . . . . . . . . . . .<br>.H. . . . . . | . . . . . .C. . . . | . . . . . . . .<br>. . . . . .<br> | . . . . . . . .<br>. . . . . . . .<br>. . . . | . . . . . . . . . . . . . . . . . .<br>. . . . . . . . . . . . . . . . . | . . .G---<br>. . . . . . . . . . . . . . . . . . .--<br>--. . . | . . . . . . |

| | | | | | | | | | |
|---|---|---|---|---|---|---|---|---|---|
| iPS:43 5219 | 21-225_95D2 | VH4\|4-34/D4\|4-17\|RF2/J H6 | ...........<br>...........<br>.H...... | ......C... | | ........<br>...... | ........<br>........<br>.... | ..................... | ...G---<br>.....................--<br>--... | ...... |
| iPS:43 5235 | 21-225_95F9 | VH4\|4-34/D4\|4-17\|RF2/J H6 | ...........<br>...........<br>.H...... | ......C... | | ........<br>...... | ........<br>........<br>.... | ..................... | ...G---<br>.....................--<br>--... | ...... |
| iPS:43 5237 | 21-225_95G9 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>--... | ...... |
| iPS:43 5239 | 21-225_95H10 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>--... | ...... |
| iPS:43 5273 | 21-225_97A2 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>--... | ...... |
| iPS:43 5281 | 21-225_97E5 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>--... | ...... |
| iPS:43 7324 | 21-225_75C2 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>--... | ...... |
| iPS:43 7328 | 21-225_75D3 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>--... | ...... |
| iPS:43 7332 | 21-225_75F3 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>--... | ...... |
| iPS:43 7344 | 21-225_75G12 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>--... | ...... |
| iPS:43 7350 | 21-225_74A3 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>--... | ...... |
| iPS:43 7369 | 21-225_74D6 | VH4\|4-34/D4\|4-17\|RF2/J H6 | ...........<br>...........<br>.H...... | ......C... | | ........<br>...... | ........<br>........<br>.... | ..................... | ...·G---<br>.....................--<br>-·... | ...... |

| | Germline | H_FR1 | H_CDR1 | H_FR2 | H_CDR2 | H_FR3 | H_CDR3 | H_FR4 |
|---|---|---|---|---|---|---|---|---|
| VH3|3-33/D4|4-17|RF2/JH6 | | QVQLVES- GGGVVQPGRSLR LSCAASG-FTFS | S YGMH | WVRQAPGK GLEWVA | VIWYD GSNKYYAD SVKG | RFTISRDNSKNTLYLQM NSLRAEDTAVYYCAR | DYGDYYY -----YYYGMDV | WGQGTT VTVSS |
| IPS:46 8814 | 21- 225_223D11 | VH3|3- 33/D4|4- 17|RF2/J H6 | .............<br>.............<br>........ | N........D | ........<br>......<br>.... | ........<br>...D....<br> | ................F...<br>................ | .R.IG.-<br>................--<br>ND... | ......<br>..... |
| IPS:43 4621 | 21-225_74D1 | VH3|3- 33/D4|4- 17|RF2/J H6 | .............<br>.............<br>........ | ......... | ........<br>......<br>.... | ........<br>.....H..<br> | ................<br>.........M..... | .E.FGEFD..........<br>....Y.N..... | ......<br>..... |
| IPS:43 4947 | 21-225_87B7 | VH3|3- 33/D4|4- 17|RF2/J H6 | .............<br>.............<br>........ | ......... | ........<br>......<br>.... | ........<br>....N...<br> | ................<br>................ | .F.VG.-<br>................--<br>..... | ......<br>..... |
| IPS:43 5819 | 21- 225_190C11 | VH3|3- 33/D4|4- 17|RF2/J H6 | .............<br>.............<br>........ | ......... | ........<br>......<br>.... | ........<br>....N...<br> | ................<br>................ | .Q.VG.-<br>................--<br>D.L.. | ......<br>..... |
| IPS:43 5825 | 21- 225_190G11 | VH3|3- 33/D4|4- 17|RF2/J H6 | .............<br>.............<br>........ | I......... | ........<br>......<br>.... | ........<br>....N...<br> | ................<br>................ | .Q.VG.-<br>................--<br>D.L.. | ......<br>..... |
| IPS:43 5837 | 21- 225_198G3 | VH3|3- 33/D4|4- 17|RF2/J H6 | .............<br>.......K....<br>........ | T......... | ........<br>......<br>.... | ........<br>.T..N...<br> | ................C...<br>................ | .Q.VG.-<br>................--<br>D.L.. | ......<br>..... |
| IPS:43 5845 | 21- 225_191G1 | VH3|3- 33/D4|4- 17|RF2/J H6 | .............<br>.............<br>........ | ......... | ........<br>......<br>.... | ........<br>...EH...<br> | ................<br>................ | .R.VG.-<br>................--<br>..L.. | ......<br>..... |
| IPS:43 5859 | 21- 225_190E6 | VH3|3- 33/D4|4- 17|RF2/J H6 | .............<br>.............<br>........ | ......... | ........<br>......<br>.... | ........<br>....N...<br> | ................<br>................ | .Q.VG.-<br>................--<br>D.L.. | ......<br>..... |
| IPS:43 5873 | 21- 225_190G4 | VH3|3- 33/D4|4- 17|RF2/J H6 | .............<br>.............<br>........ | ......... | ........<br>......<br>.... | ........<br>....N...<br> | ................<br>..........M..... | .Q.VG.-<br>................--<br>D.L.. | ......S<br>..... |
| IPS:43 5933 | 21- 225_190F8 | VH3|3- 33/D4|4- 17|RF2/J H6 | .............<br>.............<br>........ | T......... | ........<br>......<br>.... | ........<br>....N...<br> | ................<br>................ | .Q.VG.-<br>................--<br>D.L.. | ......<br>..... |

| | | | | | | | | | |
|---|---|---|---|---|---|---|---|---|---|
| iPS:43 5941 | 21-225_191E8 | VH3\|3-33/D4\|4-17\|RF2/JH6 | ............<br>.........<br>........ | N......... | ........<br>......<br>.... | I..F....<br>...Q....<br>.... | .................L<br>................--<br> | AH.V..-<br>.A... | ......<br> |
| IPS:43 5945 | 21-225_191A10 | VH3\|3-33/D4\|4-17\|RF2/JH6 | ............<br>.........<br>........ | ......... | ........<br>......<br>.... | ........<br>....N...<br>.... | ...............<br>.........M.....<br> | .Q.VG.-<br>...............--<br>D.L.. | .....S<br>.... |
| IPS:43 5947 | 21-225_191E10 | VH3\|3-33/D4\|4-17\|RF2/JH6 | ............<br>.........<br>........ | ......... | ........<br>......<br>.... | ........<br>....N...<br>.... | ...............<br>.........M.....<br> | .Q.VG.-<br>...............--<br>D.L.. | .....S<br>.... |
| iPS:43 5957 | 21-225_191G12 | VH3\|3-33/D4\|4-17\|RF2/JH6 | ............<br>.........<br>........ | ......... | ........<br>......<br>.... | ........<br>....N...<br>.... | ...............<br>...............<br> | .Q.VG.-<br>...............--<br>D.L.. | ......<br>.... |
| iPS:43 5963 | 21-225_192D2 | VH3\|3-33/D4\|4-17\|RF2/JH6 | ............<br>.........<br>........ | ......... | ........<br>......<br>.... | ........<br>....N...<br>.... | .........M.....<br>......E......<br> | .R.VG.-<br>...............--<br>..... | ......<br>.... |
| IPS:43 5971 | 21-225_192D3 | VH3\|3-33/D4\|4-17\|RF2/JH6 | ............<br>.........<br>........ | ......... | ........<br>......<br>.... | ........<br>...EH...<br>.... | ...............<br>...............<br> | .R.VG.-<br>...............--<br>..L.. | ......<br>.... |
| iPS:43 5979 | 21-225_192H4 | VH3\|3-33/D4\|4-17\|RF2/JH6 | ............<br>.........<br>........ | ......... | ........<br>......<br>.... | ........<br>....N...<br>.... | ...............<br>...............<br> | .Q.VG.-<br>...............--<br>..... | ......<br>.... |
| iPS:43 5987 | 21-225_192G6 | VH3\|3-33/D4\|4-17\|RF2/JH6 | ............<br>.........<br>........ | ......... | ........<br>......<br>.... | .L......<br>.T..N...<br>.... | ...............<br>...............<br> | .Q.VG.-<br>...............--<br>D.L.. | ......<br>.... |
| IPS:43 5993 | 21-225_192C8 | VH3\|3-33/D4\|4-17\|RF2/JH6 | ............<br>.........<br>........ | ......... | ........<br>......<br>.... | ........<br>...EH...<br>.... | ..M............<br>...............<br> | .R.VG.-<br>...............--<br>..... | ......<br>.... |
| iPS:43 5997 | 21-225_192G8 | VH3\|3-33/D4\|4-17\|RF2/JH6 | ............<br>.........<br>........ | ......... | ........<br>......<br>.... | ........<br>....N...<br>.... | ...............<br>...............<br> | .Q.VG.-<br>...............--<br>D.L.. | ......<br> |
| iPS:43 6005 | 21-225_192H10 | VH3\|3-33/D4\|4-17\|RF2/JH6 | ............<br>.........<br>........ | ......... | ........<br>......<br>.... | ........<br>....N...<br>.... | ...............<br>.........M.....<br> | .Q.VG.-<br>...............--<br>D.L.. | ......S<br>.... |
| iPS:43 6031 | 21-225_193C7 | VH3\|3-33/D4\|4-17\|RF2/JH6 | ............<br>.........<br>........ | ......... | ........<br>......<br>.... | ........<br>....N...<br>.... | ...............<br>.........M.....<br> | .Q.VG.-<br>...............--<br>D.L.. | .....S<br>.... |

| | | | | | | | | | |
|---|---|---|---|---|---|---|---|---|---|
| iPS:43 6045 | 21-225_193A10 | VH3\|3-33/D4\|4-17\|RF2\|JH6 | ............<br>............<br>........ | .......... | ........<br>...... | .......<br>...EH...<br>.... | ................. | .R.VG.-<br>...............--<br>..L.. | ...... |
| iPS:43 6076 | 21-225_194H11 | VH3\|3-33/D4\|4-17\|RF2\|JH6 | ............<br>............<br>........ | .......... | ........<br>...... | .......<br>...EH...<br>.... | ..............S... | .R.VG.-<br>...............--<br>..L.. | ...... |
| iPS:43 6086 | 21-225_191G10 | VH3\|3-33/D4\|4-17\|RF2\|JH6 | ............<br>............<br>........ | .......... | ........<br>...... | .......<br>....N...<br>.... | ................. | .Q.VG.-<br>...............--<br>D.L.. | ...... |
| iPS:43 6090 | 21-225_195A9 | VH3\|3-33/D4\|4-17\|RF2\|JH6 | ............<br>............<br>........ | .......... | ........<br>..Q... | .......<br>...EH...<br>.... | ................. | .R.VG.-<br>...............--<br>..L.. | ...... |
| iPS:43 6112 | 21-225_196C7 | VH3\|3-33/D4\|4-17\|RF2\|JH6 | ............<br>............<br>........ | .......... | ........<br>...... | .......<br>...EH...<br>.... | ................. | .R.VG.-<br>...............--<br>..L.. | ...... |
| iPS:43 6138 | 21-225_197F2 | VH3\|3-33/D4\|4-17\|RF2\|JH6 | ............<br>............<br>........ | .......... | ........<br>...... | .......<br>....N...<br>.... | ................. | .Q.VG.-<br>...............--<br>D.L.. | ...... |
| iPS:43 6152 | 21-225_197B6 | VH3\|3-33/D4\|4-17\|RF2\|JH6 | ............<br>............<br>........ | .......... | ........<br>...... | .......<br>....N...<br>.... | ...........M.....<br>..........M..... | .Q.VG.-<br>...............--<br>D.L.. | ......S |
| iPS:43 6173 | 21-225_197G12 | VH3\|3-33/D4\|4-17\|RF2\|JH6 | ............<br>............<br>........ | .......... | ........<br>...... | .......<br>....N...<br>.... | ................. | .Q.VG.-<br>...............--<br>D.L.. | ...... |
| iPS:43 6189 | 21-225_198B6 | VH3\|3-33/D4\|4-17\|RF2\|JH6 | ............<br>............<br>........ | .......... | ........<br>...... | .......<br>....H...<br>.... | ................. | .Q.VG.-<br>...............--<br>..... | ...... |
| iPS:43 6201 | 21-225_199C5 | VH3\|3-33/D4\|4-17\|RF2\|JH6 | ............<br>............<br>........ | .......... | ........<br>...... | .......<br>....N...<br>.... | ................. | .Q.VG.-<br>...............--<br>..... | ...... |
| iPS:43 6203 | 21-225_199A6 | VH3\|3-33/D4\|4-17\|RF2\|JH6 | ............<br>............<br>........ | N......... | ........<br>...... | I..F....<br>...Q....<br>.... | ...............L | AH.V..-<br>...............--<br>.A... | ...... |
| iPS:43 6282 | 21-225_204G6 | VH3\|3-33/D4\|4-17\|RF2\|JH6 | ............<br>...........T<br>........ | .......... | ........<br>...... | .......<br>....N...<br>.... | ................. | .R.VG.-<br>...............--<br>D.... | ...... |

| iPS:43 6296 | 21-225_205F5 | VH3\|3-33/D4\|4-17\|RF2\|JH6 | . . . . . . . . . . .<br>. . . . . . . . . . .<br>. . . . . . . . | R . . . . . . . . . | . . . . . . . . .<br>. . . . . .<br>. . . . | . . . . . . .<br>. . .EN.V.<br>. . . . | . . . . . . .T..KM.F. . . .<br>. . . .TD. . . . . . . . . | .M.IG.–<br>. . . . . . . . . . . . . . . . . ––<br>. . . . . | . . . . . .<br>. . . . . |
| iPS:43 6324 | 21-225_207G6 | VH3\|3-33/D4\|4-17\|RF2\|JH6 | . . . . . . . . . . .<br>. . . . . . . . . . . .<br>. . . . . . . . | . . . . . . . . . | . . . . . . . . .<br>. . . . . .<br>. . . . | . . . . . . .<br>. . . .N..E<br>. . . . | . . . . . . . . . . . . . . . . .<br>. . . . . . . . . . . . . . . . | .A.IG.–<br>. . . . . . . . . . . . . . . . . ––<br>..I.. | . . . . . .<br>. . . . . |
| iPS:43 6364 | 21-225_211A11 | VH3\|3-33/D4\|4-17\|RF2\|JH6 | . . . . .G. . . . . .<br>. . . . . . . . . . . .<br>. . . . . . . . | . . . . . . . . . | . . . . . . . . .<br>. . . . . .<br>. . . . | .L.F. . . .<br>. . .RN. . .<br>. . . . | . . . . . . . . . . . . . . . . .<br>. . . . . . . . . . . . . . . . | .R.VG.–<br>. . . . . . . . . . . . . . . . . ––<br>..T.. | . . . . . .<br>. . . . . |
| iPS:43 6372 | 21-225_211A8 | VH3\|3-33/D4\|4-17\|RF2\|JH6 | . . . . . . . . . . .<br>. . . . . . . . . . . .<br>. . . . . . . . | . . . . . . . . . | . . . . . . . . .<br>. . . . . .<br>. . . . | . . . . . . .<br>. . .EH. . .<br>. . . . | . . . . . . . . . .K. . . . . .<br>. . . . . . . . . . . . . . . . | .H.VG.–<br>. . . . . . . . . . . . . . . . . ––<br>. . . . . | . . . . . .<br>. . . . . |
| iPS:43 6376 | 21-225_212E6 | VH3\|3-33/D4\|4-17\|RF2\|JH6 | . . . . . . . . . . .<br>. . . . . . . . . . . .<br>. . . . . . . . | . . . . . . . . . | . . . . . . . . .<br>. . . . . .<br>. . . . | . . . . . . .<br>. . . .N.V.<br>. . . . | . . . . . . . . . . . . . . . . .<br>. . . . . . . . . . . . . . . . | . . .VG.–<br>. . . . . . . . . . . . . . . . . ––<br>..T.. | . . . . . .<br>. . . . . |
| iPS:43 6378 | 21-225_212D7 | VH3\|3-33/D4\|4-17\|RF2\|JH6 | . . . . . . . . . . .<br>. . . . . . . . . . . .<br>. . . . . . . . | . . . . . . . . . | . . . . . . . . .<br>. . . . . .<br>. . . . | . . . . . . .<br>. . . .N. . .<br>. . . . | . . . . . . . . . . . . . .S. . .<br>. . . . . . . . . . . . . . . . | . . .VG.–<br>. . . . . . . . . . . . . . . . . ––<br>..T.. | . . . . . .<br>. . . . . |
| iPS:43 6380 | 21-225_212H9 | VH3\|3-33/D4\|4-17\|RF2\|JH6 | . . . . . . . . . . .<br>. . . . . . . . . . . .<br>. . . . . . . . | . . . . . . . . . | . . . . . . . . .<br>. . . . . .<br>. . . . | . . . . . . .<br>. . .EH. . .<br>. . . . | . . . . . . . . . .K. . . . . .<br>. . . . . . . . . . . . . . . . | .H.VG.–<br>. . . . . . . . . . . . . . . . . ––<br>. . . . . | . . . . . .<br>. . . . . |
| iPS:43 6384 | 21-225_212F10 | VH3\|3-33/D4\|4-17\|RF2\|JH6 | . . . . . . . . . . .<br>. . . . . . . . . . . .<br>. . . . . . . . | R . . . . . . . . . | . . . . . . . . .<br>. . . . . .<br>. . . . | . . . . . . .<br>. . . .H. . .<br>. . . . | . . . . . . . . . . . . . . . . .<br>. . . .G. . . . . . . . . . | .R.VG.–<br>. . . . . . . . . . . . . . . . . ––<br>N. . . . | . . . . . .<br>. . . . . |
| iPS:43 6390 | 21-225_213D2 | VH3\|3-33/D4\|4-17\|RF2\|JH6 | . . . . . . . . . . .<br>. . . . . . . . . . . .<br>. . . . . . . . | . . . . . . . . . | . . . . . . . . .<br>. . . . . .<br>. . . . | . . . . . . .<br>. . . .N. . .<br>. . . . | . . . . . . . . . . . . . .S. . .<br>. . . . . . . . . . . . . . . . | . . .VG.–<br>. . . . . . . . . . . . . . . . . ––<br>..T.. | . . . . . .<br>. . . . . |
| iPS:43 6394 | 21-225_213C4 | VH3\|3-33/D4\|4-17\|RF2\|JH6 | . . . . . . . . . . .<br>. . . . . . . . . . . .<br>T. . . . . . . | . . . . . . . . . | . . . . . . . . .<br>. . . . . .<br>. . . . | . . . . . . .<br>. . . .H. . .<br>. . . . | . . . . . . . . . . . . . . . . .<br>. . . . . . . . . . . . . . . . | . . .VG.–<br>. . . . . . . . . . . . . . . . . ––<br>D. . . . | . . . . . .<br>. . . . . |
| iPS:43 6398 | 21-225_213B8 | VH3\|3-33/D4\|4-17\|RF2\|JH6 | . . . . . . . . . . .<br>. . . . . . . . . . . .<br>. . . . . . . . | . . . . . . . . . | . . . . . . . . .<br>. . . . . .<br>. . . . | . . . . . . .<br>. . . .H. . .<br>. . . . | . . . . . . . . . . . . . . . . .<br>. . . . . . . . . . . . . . . . | . . .VG.–<br>. . . . . . . . . . . . . . . . . ––<br>..T.. | . . . . . .<br>. . . . . |
| iPS:43 6404 | 21-225_214C3 | VH3\|3-33/D4\|4-17\|RF2\|JH6 | . . . . . . . . . . .<br>. . . . . . . . . . .E<br>. . . . . . . . | . . . . . . . . . | . . . . . . . . .<br>. . . . . .<br>. . . . | . . . . . . .<br>. . . .N.G.<br>. . . . | . . . . . . . . . . . . . . . . .<br>. . . . . . . . . . . . . . . . | .R.VG.–<br>. . . . . . . . . . . . . . . . . ––<br>D. . . . | . . . . . .<br>. . . . . |

| | | | H_FR1 | H_CDR1 | H_FR2 | H_CDR2 | H_FR3 | H_CDR3 | H_FR4 |
|---|---|---|---|---|---|---|---|---|---|
| iPS:43 6410 | 21-225_212E10 | VH3\|3-33/D4\|4-17\|RF2/J H6 | ............ ............ ........ | .......... | ........ ...... | ........ ........ .... | ...............S... ................ | ...VG.- ...............-- ..T.. | ...... ..... |
| iPS:43 6420 | 21-225_215B5 | VH3\|3-33/D4\|4-17\|RF2/J H6 | ............ ............ ........ | .......... | ........ ...... | ........ ....N... .... | ...............S... ................ | ...VG.- ...............-- ..T.. | ...... ..... |
| iPS:43 6422 | 21-225_215D6 | VH3\|3-33/D4\|4-17\|RF2/J H6 | ............ ............ ........ | .......... | ........ ...... | ........ ....N... .... | ................ ................ | .C.VG.- ...............-- ..T.. | ...... ..... |
| iPS:43 6430 | 21-225_215A12 | VH3\|3-33/D4\|4-17\|RF2/J H6 | ............ ............ ....L... | .......... | ........ ...... | ........ ...EH... .... | ................ ................ | .R.VG.- ...............-- ..... | ...... ..... |
| iPS:43 6452 | 21-225_217G5 | VH3\|3-33/D4\|4-17\|RF2/J H6 | ............ ............ ........ | .......N... | ........ ...... | ........ ....N... .... | ................ ................ | ...VG.- ...............-- ..L.. | ...... ..... |
| iPS:43 6464 | 21-225_219H1 | VH3\|3-33/D4\|4-17\|RF2/J H6 | ............ ............ ........ | R......... | ........ ...... | ........ ....H... .... | ....G........... ................ | .R.VG.- ...............-- N.... | ...... .... |
| iPS:45 1120 | 21-225_197D3 | VH3\|3-33/D4\|4-17\|RF2/J H6 | ............ ............ .....I.. | ......H... | ........ ...... | ........ ...EH... .... | ................ ................ | .Q.VG.- ...............-- ..... | ...... ..... |
| Germline | | | H_FR1 | H_CDR1 | H_FR2 | H_CDR2 | H_FR3 | H_CDR3 | H_FR4 |
| VH3\|3-33/D4\|4-17\|RF2/JH4 | | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | DYGDY----------------YFDY | WGQGTLVTVSS |
| iPS:46 8822 | 21-225_147E10 | VH3\|3-33/D4\|4-17\|RF2/J H4 | ............ ....K....... ........ | N.......L. | ........ ...... | I....... .... | ................ | .HY.FW............ ......SGH... | ..... |
| iPS:43 3965 | 21-225_46F2 | VH3\|3-33/D4\|4-17\|RF2/J H4 | ............ ............ ........ | .......... | ........ ...... | I....... ......V. .... | ................ | .RY.FW............ ......SG.... | ..... |
| iPS:43 4255 | 21-225_62E6 | VH3\|3-33/D4\|4-17\|RF2/J H4 | ............ ............ ........ | .......... | ........ ...... | A....... ......G. .... | .V.........S.H... | .Q.IVG............ ......ATW... | ..... |

| | | | | | | | | |
|---|---|---|---|---|---|---|---|---|
| iPS:43 4269 | 21-225_57H3 | VH3\|3-33/D4\|4-17\|RF2/J H4 | . . . . . . . . . . . . . . . . . . . . . . . . . . . . . . . | . . . . . . . . . | . . . . . . . . . . . . . . . . | A . . . . . . . . . . . . . . . . . . . . . . . . | . . . . . . . . . . . . . . . . . . . . . . . . . . . . . . . . . . . . | . . . IVG . . . . . . . . . . . . . . . . . . . AT . . . . . | . . . . . . . . . . . |
| iPS:43 4345 | 21-225_64H9 | VH3\|3-33/D4\|4-17\|RF2/J H4 | . . . . . . . . . . . . . . . . . . . . . . . . . . . . . . . | T . . . . . . . . . | . . . . . . . . . . . . . . . . | I . . . . . . . . . . . . . . . . . . . . . | . . . . . . . . . . . . . . . . . . . . . . . . . . . . . . . . . . . . | .TY.FW . . . . . . . . . . . . . . . . . . . SG.LG. | . . . . . . . . . . . |
| iPS:43 4363 | 21-225_65A6 | VH3\|3-33/D4\|4-17\|RF2/J H4 | . . . . . . . . . . . . . . . . . . . . . . . . . . . . . . . | N . . . . . . . . . | . . . . . . . . . . . . . . . . | . . . . . . . . . . . . . . G . . . . . . | . . . . . . . . . . . . . . . . . . . . . . . . . . . . . . . . . . . . | .Q.IVG . . . . . . . . . . . . . . . . . . . ATW . . . | . . . . . . . . . . . |
| iPS:43 4393 | 21-225_67C3 | VH3\|3-33/D4\|4-17\|RF2/J H4 | . . . . . . . . . . . . . . . . . . . . . . . . . . . . . . . | . . . . . . . . . | . . . . . . . . . . . . . . . . | A . . . . . . . . . . . . . . G . . . . . . | .V . . . . . . . . . S.H . . . . . . . . . . . . . . . . . . . . | .Q.IVG . . . . . . . . . . . . . . . . . . . ATW . . . | . . . . . . . . . . . |
| iPS:43 4425 | 21-225_70A5 | VH3\|3-33/D4\|4-17\|RF2/J H4 | . . . . . . . . . . . . . . . . . . . . . . . . . . . . . . . | . . . . . . . . . | . . . . . . . . . . . . . . . . | . . . . . . . . . . . . . . . . . . . . . | . . . . . . . . . . . . S . . . . .S . . . . . . . . . . . | .Q.IVG . . . . . . . . . . . . . . . . . . . ATW . . . | . . . . . . . . . . . |
| IPS:43 5341 | 21-225_148B2 | VH3\|3-33/D4\|4-17\|RF2/J H4 | . . . . . . . . . . . . . . . . . . . . . . . . . . . . . . . | . . . . . . . . . | . . . . . . . . . . . . . . . . | I . . . . . . . . .Y . . . . . . . . . . . | . . . . . . . . . . . . . . . . .S . . . . . . . . . . . | .HF.FW . . . . . . . . . . . . . . . . . . . SGH . . . | . . . . . . . . . . . |
| IPS:43 5357 | 21-225_148G10 | VH3\|3-33/D4\|4-17\|RF2/J H4 | . . . . . . . . . . . . . . . . . . . . . . . . . . . . . . . | . . . . . . . . . | . . . . . . . . . . . . . . . . | I . . . . . . . . . . . . . . . . . . . . . | . . . . . . . . . . . . . . . . . . . . . . . . . . . . . . . . . . . . | .RY.FW . . . . . . . . . . . . . . . . . . . SGH . . . | . . . . . . . . . . . |
| IPS:43 5365 | 21-225_149F1 | VH3\|3-33/D4\|4-17\|RF2/J H4 | . . . . . . . . . . . . . . . . . . . . . . . . . . . . . . . | . . . . . . . . . | . . . . . . . . . . . . . . . . | I . . . . . . . . .Y . . . . . . . . . . . | . . . . . . . . . . . . . . . . .S . . . . . . . . . . . | .HF.FW . . . . . . . . . . . . . . . . . . . SGH . . . | . . . . . . . . . . . |
| iPS:43 5413 | 21-225_150B11 | VH3\|3-33/D4\|4-17\|RF2/J H4 | . . . . . . . . . . . . . . . . . . . . . . . . . . . . . . . | . . . . . . . . . | . . . . . . . . . . . . . . . . | I . . . . . . . . . . . . . . . . . . . . . | . . . . . . . . . . . . . . . . . . . . . . . . . . . . . . . . . . . . | .RY.FW . . . . . . . . . . . . . . . . . . . SGH . . . | . . . . . . . . . . . |
| iPS:43 5423 | 21-225_151G5 | VH3\|3-33/D4\|4-17\|RF2/J H4 | . . . .M . . . . . . . . . . . .I . . | . . . . . . . . . | . . . . . . . . . . . . . . . . | I . . . . . . . . . . . . . . . . . . . . . | . . . . . . . . . . . . . . . . . . . . . . . . . . . . . . . . . . . . | .RY.FW . . . . . . . . . . . . . . . . . . . SGH . . . | . . . . . . . . . . . |
| iPS:43 5429 | 21-225_151A10 | VH3\|3-33/D4\|4-17\|RF2/J H4 | . . . . . . . . . . . . . . . . . . . . . . . . . . . . . . . | N . . . . . . . . . | . . . .L . | I . . . . . . . . . . . . . . . . . . . . . | . . . . . . . . . . . . . . . . . . . . . . . . . . . . . . . . . . . . | .HY.FW . . . . . . . . . . . . . . . . . . . SGH . . . | . . . . . . . . . . . |
| iPS:43 5489 | 21-225_155A5 | VH3\|3-33/D4\|4-17\|RF2/J H4 | . . . . . . . . . .D. . . . . . . . . . . . . . . . . . . . . . | . . . . . . . . . | . . . . . . . . . . . . . . . . | I . . . . . . .S . . . . . . . . . . . | . . . . . . . . . . . . . . . .H. . . . . . . . . . . . . . . . . | .RY.FW . . . . . . . . . . . . . . . . . . . SGH . . . | . . . . . . . . . . . |

| ID | Clone | Germline | H_FR1 | H_CDR1 | H_FR2 | H_CDR2 | H_FR3 | H_CDR3 | H_FR4 |
|---|---|---|---|---|---|---|---|---|---|
| iPS:43 5683 | 21-225_170A1 | VH3\|3-33/D4\|4-17\|RF2/JH4 | ............ ............ ........ | .......... | ........ ...... .... | I....... ..Y..... .... | ................ | .AH.FW............ ......SG...S | ...... ..... |
| iPS:43 5755 | 21-225_176H4 | VH3\|3-33/D4\|4-17\|RF2/JH4 | ............ ............ ........ | .......... | ........ ...... .... | I....... ..Y..... .... | S............... | .AH.FW............ ......SG..A. | ....A. ..... |
| iPS:43 5795 | 21-225_181C2 | VH3\|3-33/D4\|4-17\|RF2/JH4 | ............ ..........V ........ | .......... | ........ ......T .... | I....... ..Y..... .... | ................ | .HY.FW............ ......SGH..F | ...... ..... |
| iPS:43 5807 | 21-225_181C10 | VH3\|3-33/D4\|4-17\|RF2/JH4 | ............ ............ ........ | .......... | ........ ....M. .... | I....... ..Y..... .... | ................ | .HY.FW............ ......SGH... | ...... ..... |
| iPS:43 5887 | 21-225_186F7 | VH3\|3-33/D4\|4-17\|RF2/JH4 | ............ T......... ........ | .......... | ........ ...... .... | I....... ..Y..... .... | ................ | .HY.FW............ ......SGH... | ...... ..... |
| iPS:43 5901 | 21-225_189G2 | VH3\|3-33/D4\|4-17\|RF2/JH4 | ............ N......... ........ | .......... | ........ ...... .... | I....... ..Y..... .... | ................ | .RF.FW............ ......SG.S.. | ...... ..... |
| iPS:43 6594 | 21-225_226A5 | VH3\|3-33/D4\|4-17\|RF2/JH4 | ............ N......... ........ | .......... | ........ ...... .... | I....... .I....T. .... | ................ | EGH.FW............ ......SGF.C. | ...... ..... |
| iPS:39 2814 | 21-225_22A1 | VH3\|3-33/D4\|4-17\|RF2/JH4 | ............ ............ ........ | .......... | ........ ...... .... | .M...... ........ .... | ................ | .G.FL............ .......EWL.. | ...... ..... |
| iPS:39 3036 | 21-225_28G3 | VH3\|3-33/D4\|4-17\|RF2/JH4 | ............ T......... .....I.. | .......... | ........ ...... .... | ........ ........ .... | ................ | .RY.FW............ ......SG.... | ...... ..... |
| | | Germline | H_FR1 | H_CDR1 | H_FR2 | H_CDR2 | H_FR3 | H_CDR3 | H_FR4 |
| | VH3\|3-33/D7\|7-27\|RF1/JH4 | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | LTGY--------------------FDY | WGQGTLVTVSS |
| iPS:46 8824 | 21-225_73G6 | VH3\|3-33/D7\|7-27\|RF1/JH4 | ............ ............ ........ | .......... | ........ ...... .... | ........ V.....G. .... | ................ | EV.M............. ........TS.. | ...... ..... |

| | | | | | | | | | |
|---|---|---|---|---|---|---|---|---|---|
| iPS:43 4169 | 21-225_50C4 | VH3\|3-33/D7\|7-27\|RF1/J H4 | ............ ............ ........ | D......... | ........ ...... .... | ....E... EI...... .... | ................ | EV.F............... ........LN.. | ....I. ..... |
| iPS:43 5045 | 21-225_90H5 | VH3\|3-33/D7\|7-27\|RF1/J H4 | ............ ............ ........ | D......... | ....S... ...... .... | ....E... ...T.... .... | ...............V. | EM.W............... ........LD.. | ..... ..... |
| iPS:43 5367 | 21-225_149G1 | VH3\|3-33/D7\|7-27\|RF1/J H4 | ............ ............ ........ | D......... | ........ ...... .... | ....E... ........ .... | ...........M..... ................ | EI.F............... ........SE.. | ..... ..... |
| iPS:43 5397 | 21-225_149F12 | VH3\|3-33/D7\|7-27\|RF1/J H4 | ............ ............ ........ | D......... | ........ ...... .... | ....E... EN...... .... | ..............F... | EI.F............... ........SE.. | ..... ..... |
| iPS:43 5407 | 21-225_150E7 | VH3\|3-33/D7\|7-27\|RF1/J H4 | ............ ............ ........ | D......... | ........ ...... .... | ....E... EN...... .... | ..............F... | EI.F............... ........SE.. | ..... ..... |
| iPS:43 5609 | 21-225_161F7 | VH3\|3-33/D7\|7-27\|RF1/J H4 | ............ ............ ........ | D.....F.L. | .......Q ...... .... | ...F.... ........ .... | ....F........... | EI.W............... ........LS.. | ..... ..... |
| iPS:43 5613 | 21-225_161D11 | VH3\|3-33/D7\|7-27\|RF1/J H4 | ............ ............ ........ | D.....F.L. | .......Q ...... .... | ...F.... ........ .... | ....F........... ................ | EI.W............... ........LS.. | ..... ..... |
| iPS:43 5791 | 21-225_180H7 | VH3\|3-33/D7\|7-27\|RF1/J H4 | ............ ............ ........ | D......... | ........ ...... | ........ EN..H... .A.. | ....V........... | EV.W............... ........SD.. | ..... ..... |
| iPS:43 5805 | 21-225_181A8 | VH3\|3-33/D7\|7-27\|RF1/J H4 | ............ ............ ........ | D......... | ........ ...... | ........ EN..H... .A.. | ................ | EV.W............... ........SD.. | ..... .I... |
| iPS:43 5879 | 21-225_184H10 | VH3\|3-33/D7\|7-27\|RF1/J H4 | ............ ..........V ........ | D......... | ........ ...... | ........ ET..H.G. .... | ...........D..... | EV.W............... ........HD.. | ..... ..... |
| iPS:43 5881 | 21-225_184D11 | VH3\|3-33/D7\|7-27\|RF1/J H4 | ............ ............ ........ | D......... | ........ ...... | ........ ET..H.G. .... | ...........D..... | EV.W............... ........HD.. | ..... ..... |
| iPS:43 6350 | 21-225_210E4 | VH3\|3-33/D7\|7-27\|RF1/J H4 | ............ ............ ......LI | N......... | ........ ...... | ........ EN....V. .... | .......D. ......S....... | E..F............... ........LS.. | ..... ..... |

| | | | | | | | | |
|---|---|---|---|---|---|---|---|---|
| iPS:43 6576 | 21-225_225B6 | VH3\|3-33/D7\|7-27\|RF1/JH4 | ..R.......... ............ ........ | D......... | ........ ...... | ........ EN...... .... | ................ ................ | EV.F............... ........TE.. | ...... ...... |
| iPS:43 6578 | 21-225_225D6 | VH3\|3-33/D7\|7-27\|RF1/JH4 | ............ ............ ........ | N......... | ........ ...... | ........ EN...... .... | ........Q...... T............. | EV.F............... ........TE.. | ...... ...... |
| iPS:43 6582 | 21-225_225F8 | VH3\|3-33/D7\|7-27\|RF1/JH4 | ............ ............ ........ | .......... | ........ ...... | ........ EN....V. .... | ................ ................ | EV.F............... ........TE.. | ...... ...... |
| iPS:43 6608 | 21-225_226A9 | VH3\|3-33/D7\|7-27\|RF1/JH4 | ............ ............ ........ | N......... | ........ ...... | ....E... E.....T. .... | ...........F... ................ | EV.F............... ........TE.. | ...... ...... |
| iPS:43 6630 | 21-225_227G3 | VH3\|3-33/D7\|7-27\|RF1/JH4 | ............ ............ ........ | .......... | ........ ...... | ....V... ...Q.... .... | ................ S............... | EV.F............... ........TE.. | ...... ...... |
| iPS:43 6634 | 21-225_227H5 | VH3\|3-33/D7\|7-27\|RF1/JH4 | ............ ............ ........ | N......... | ........ ..D... | ....E... E....... .... | ................ .........M..... | EV.F............... ........TE.. | ...... ...... |
| iPS:43 6650 | 21-225_227C12 | VH3\|3-33/D7\|7-27\|RF1/JH4 | ............ ...K....... ........ | N......... | ........ ...... | ....I... ...Q.... .... | ................ S............... | EV.F............... ........TE.. | ...... ...... |
| iPS:43 7280 | 21-225_203C10 | VH3\|3-33/D7\|7-27\|RF1/JH4 | ............ ............ ........ | D......... | ........ ...... | ........ .G.TH.T. .... | ................ ................ | EV.W............... ........LD.. | ...... ...... |
| iPS:39 2740 | 21-225_18H12 | VH3\|3-33/D7\|7-27\|RF1/JH4 | ............ ............ ........ | D......... | ........ ....M. | ........ VT...... .... | ................ ................ | EV.W............... ........YE.. | ...... ...... |
| iPS:39 2780 | 21-225_22B7 | VH3\|3-33/D7\|7-27\|RF1/JH4 | ............ ............ ........ | .......... | ........ ...... | ....E... EN.Q.... .... | ................ ................ | EV.F............... ........RS.. | ...... ...... |
| iPS:39 2912 | 21-225_25A9 | VH3\|3-33/D7\|7-27\|RF1/JH4 | ............ ............ ........ | .......... | ........ ...... | ........ VT....TG .... | ................ ................ | EI.W............... ........LD.. | ...... ...... |
| iPS:39 2940 | 21-225_29D9 | VH3\|3-33/D7\|7-27\|RF1/JH4 | ............ ......K...S ........ | D.......I. | ........ ...... | ........ E..N.... .... | ................ ................ | EI.W............... ........LD.. | .....Q ...... |

| iPS ID | Antibody | V-gene | | | | | | |
|---|---|---|---|---|---|---|---|---|
| iPS:39 2948 | 21-225_25G5 | VH3\|3-33\|D7\|7-27\|RF1\|JH4 | .......... ........... ........ | D.......I. | ........ ...... .... | ........ .N...... .... | ................. .N........... | EI.W............... ........LD.. | ...... ..... |
| iPS:39 2978 | 21-225_28B8 | VH3\|3-33\|D7\|7-27\|RF1\|JH4 | .......... ........... ........ | D......... | ........ ......T .... | ........ AN...... .... | ........F...V.... ................. | EI.W............... ........LD.. | ...... ..... |
| iPS:39 2998 | 21-225_28A9 | VH3\|3-33\|D7\|7-27\|RF1\|JH4 | .......... ........... ........ | ........I. | ........ ...... .... | ...F.... ...... .... | ...............F... ....V.......... | EI.W............... ........LD.. | ...... ..... |
| iPS:39 3038 | 21-225_29D8 | VH3\|3-33\|D7\|7-27\|RF1\|JH4 | .......... ........... .......R | D......I. | ........ ...... .... | ...F.... .T..... .... | ................. ...............G. | EI.W............... ........LD.. | ...... ..... |
| iPS:39 3058 | 21-225_30F3 | VH3\|3-33\|D7\|7-27\|RF1\|JH4 | .......... ........... ........ | ........... | ........ ...... .... | V....... .... | ................. ................. | EM.W............... ........YD.. | ...... ..... |
| iPS:39 3074 | 21-225_33B1 | VH3\|3-33\|D7\|7-27\|RF1\|JH4 | .......... ........... ........ | D......... | ......A. ...... .... | RN..... .... | ........ ................. | EM.W............... ........YD.. | ...... ..... |
| iPS:39 3822 | 21-225_15B11 | VH3\|3-33\|D7\|7-27\|RF1\|JH4 | .......... ........... ........ | N......... | ........ ...... .... | ....E... E.....T. .... | ................. ....P.......... | EV.F............... ........TE.. | ...... ..... |
| iPS:39 3856 | 21-225_14C2 | VH3\|3-33\|D7\|7-27\|RF1\|JH4 | .......... ........... ........ | D......... | ........ ...... .... | E.....E. .... | ................. K.......G...... | EV.F............... ........RS.. | ...... ..... |
| iPS:39 3874 | 21-225_4C8 | VH3\|3-33\|D7\|7-27\|RF1\|JH4 | .......... ........... ........ | N......... | ........ ...... .... | ....E... EN.Q.... .... | ................. ...T........SP. | EM.F............... ........LS.. | ...... ..... |
| iPS:39 3984 | 21-225_4F12 | VH3\|3-33\|D7\|7-27\|RF1\|JH4 | .......... ........... ........ | ........... | ........ ...... .... | ........ VT..K... .... | ................. ...TP.N.GG.ENQ. | EK.G............... ........L... | ...... ..... |
| iPS:39 4020 | 21-225_15H10 | VH3\|3-33\|D7\|7-27\|RF1\|JH4 | .......... ........... ........ | N......... | ........ ...... .... | ........ E.....E. .... | ................. ...........V. | EV.F............... ........LS.. | ....I. ..... |
| iPS:39 4095 | 21-225_16H4 | VH3\|3-33\|D7\|7-27\|RF1\|JH4 | .......... ........... ........ | N......... | ........ ...... .... | ........ V....... .... | ............M... ................. | EM.W............... ........YD.C | ...... ..... |

EP 4 435 105 A2

| | | Germline | H_FR1 | H_CDR1 | H_FR2 | H_CDR2 | H_FR3 | H_CDR3 | H_FR4 |
|---|---|---|---|---|---|---|---|---|---|
| | VH1\|1-08/D5\|5-24\|RF3/JH6 | | QVQLVQS-GAEVKKPGASVK VSCKASG-YIFT | S-----YDIN | WVRQATGQ GLEWMG | WMNPN---- SGNTGYAQ KFQG | RVTMTRNTSISTAYMEL SSLRSEDTAVYYCAR | RDGYNYYY ------YYYGMDV | WGQGTT VTVSS |
| IPS:46 8818 | 21-225_190C8 | VH1\|1-08/D5\|5-24\|RF3/J H6 | .............  ............. .......I | .......... | ........ ...... | ....K... R....... .... | .......D.......... ............... | G.P..WN- ................- S.A... | ....A. ..... |
| iPS:43 6023 | 21-225_193A5 | VH1\|1-08/D5\|5-24\|RF3/J H6 | .............  ............. .......I | .......... | ........ ...... | ....K... R....... .... | .......D.......... ............... | G.P..WN- ................- S.A... | ....A. ..... |
| iPS:43 6132 | 21-225_196C12 | VH1\|1-08/D5\|5-24\|RF3/J H6 | .............  ............. .......I | .......... | ........ ...... | ....K... R....... .... | .......D.......... ............... | G.P..WN- ................- S.A... | ....A. ..... |
| | VH3\|3-33/D2\|2-8\|RF3/JH5 | Germline | QVQLVES-GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | DIVLMVY----------- -----AINWFDP | WGQGTL VTVSS |
| IPS:46 8828 | 21-225_162A10 | VH3\|3-33/D2\|2-8\|RF3/JH 5 | ...........  ........ | ......C... | ........ ...... | A....... ........ .... | ..............F... ............... | .KNI.G- ...............- DT...F | ...... |
| | VH1\|1-02/D5\|5-18\|RF3/JH4 | Germline | QVQLVQS-GAEVKKPGASVK VSCKASG-YIFT | G-----YYMH | WVRQAPGQ GLEWMG | WINPN---- SGGTNYAQ KFQG | RVTMTRDTSISTAYMEL SRLRSDDTAVYYCAR | GYSYG----------- ------YYFDY | WGQGTL VTVSS |
| IPS:46 8830 | 21-225_191G11 | VH1\|1-02/D5\|5-18\|RF3/J H4 | ...........  ........ | .......... | ........ ...... | ........ .....F. .... | ...L......N...... .W............... | .KN............. ........S.... | ...... |
| iPS:43 6896 | 21-225_67F10 | VH1\|1-02/D5\|5-18\|RF3/J H4 | ...........  ........ | .......... | ........ ...... | ........ ......G. .... | ............... | T.F..SGS.......... .....YYNG... | ...... |
| iPS:39 3218 | 21-225_14G3 | VH1\|1-02/D5\|5-18\|RF3/J H4 | ...........  ........ | .........Y | ........ ...... | ........ ........ .... | ............... | S.F..SGS.......... .....YYNE... | ...... |
| iPS:39 3565 | 21-225_34B11 | VH1\|1-02/D5\|5-18\|RF3/J H4 | ..K.........  ........ | .......... | ........ ...... | ........ ........ .... | ............... | V.F..SGS.......... .....YYNE... | ...... |

3192

| | | | H_FR1 | H_CDR1 | H_FR2 | H_CDR2 | H_FR3 | H_CDR3 | H_FR4 |
|---|---|---|---|---|---|---|---|---|---|
| iPS:39 8470 | 21-225_14B7 | VH1\|1-02/D5\|5-18\|RF3/JH4 | ............ ............ ........ | ......... | ........ ...... .... | ........ ......V. | ...............C... ...K.......F... | SFF..SGS.......... .....YYNE... | ...... ..... |
| | | Germline | H_FR1 | H_CDR1 | H_FR2 | H_CDR2 | H_FR3 | H_CDR3 | H_FR4 |
| | VH3\|3-30.3/D1\|1-1\|RF1/JH6 | | QVQLVES-GGGVVQPGRSLR LSCAASG-FITS | S-----YAMH | WVRQAPGK GLEWVA | VISYD---GSNKYYAD SVKG | RFTISRDNSKNTLYLQM NSLRAEDTAVYYCAR | GTIGTYY---------------YYYGMDV | WGQGTT VTVSS |
| iPS:46 8836 | 21-225_198E3 | VH3\|3-30.3/D1\|1-1\|RF1/JH6 | ............ ............ ........ | .......G.. | ........ ...... .... | ........ .GY.N... .... | ................. | ..H.Y.- ................-- ..V.. | ...... ..... |
| iPS:43 5831 | 21-225_190C12 | VH3\|3-30.3/D1\|1-1\|RF1/JH6 | .........A. ............ ........ | .......G.. | ........ ...... .... | ........ .GY.N.V. .... | ................. | ..H.Y.- ................-- ..V.. | ...... ..... |
| iPS:43 5857 | 21-225_191A4 | VH3\|3-30.3/D1\|1-1\|RF1/JH6 | ............ ............ ........ | .......G.. | ........ ...... .... | ........ .GY.N... .... | ................. | ..H.Y.- ................-- ..V.. | ...... ..... |
| iPS:43 5907 | 21-225_190G3 | VH3\|3-30.3/D1\|1-1\|RF1/JH6 | ............ ............ ........ | .......G.. | ........ ...... .... | ........ .GY.N.V. .... | ................. | ..H.Y.- ................-- ..V.. | ...... ..... |
| iPS:43 5919 | 21-225_190H5 | VH3\|3-30.3/D1\|1-1\|RF1/JH6 | ............ ............ ........ | .......G.. | ........ ...... .... | ........ .GY.N... .... | ................. | ..H.Y.- ................-- ..V.. | ...... ..... |
| iPS:43 5989 | 21-225_192F7 | VH3\|3-30.3/D1\|1-1\|RF1/JH6 | ............ ............ ........ | .......G.. | ........ ......T .... | ........ .GY.N... .... | ................. | ..H.Y.- ................-- ..V.. | ...... ..... |
| iPS:43 6222 | 21-225_200C9 | VH3\|3-30.3/D1\|1-1\|RF1/JH6 | ............ ............ ........ | .......G.. | ........ ...... .... | ........ .GY.N.I. .... | ..........F.... | ..H.Y.- ................-- ..V.. | ...... ..... |
| | | Germline | H_FR1 | H_CDR1 | H_FR2 | H_CDR2 | H_FR3 | H_CDR3 | H_FR4 |
| | VH4\|4-30.1/D5\|5-24\|RF3/JH6 | | QVQLQES-GPGLVKPSQILS LTCTVSG-GSIS | SG---GYYWS | WIRQHPGK GLEWIG | YIYY----SGSTYYNP SLKS | RVTISVDTSKNQFSLKL SSVTAADTAVYYCAR | RDGYNYYY---------------YYYGMDV | WGQGTT VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:46 8840 | 21-225_200H9 | VH4\|4-30.1/D5\|5-24\|RF3/JH6 | .............<br>...........I<br>......MR | ......D... | .........<br>....F.<br>.... | ......... | ...L........... | M.YS..--<br>..............-<br>...... | .....S<br>..... |
| iPS:43 6096 | 21-225_195E10 | VH4\|4-30.1/D5\|5-24\|RF3/JH6 | .............<br>.............<br>........ | ......... | .........<br>......<br>.... | ......... | ...A........... | GGYNWN--<br>...............--<br>H.... | ......<br>..... |
| iPS:43 6120 | 21-225_196C10 | VH4\|4-30.1/D5\|5-24\|RF3/JH6 | .............<br>.............<br>......MR | ......D... | .........<br>....F.<br>.... | F....... | ...L........... | M.YS..--<br>..............-<br>...... | ......<br>..... |
| iPS:43 6216 | 21-225_200B7 | VH4\|4-30.1/D5\|5-24\|RF3/JH6 | .............<br>.............<br>........ | ......... | .........<br>......<br>.... | ..F.....<br>....N...<br>..R. | ............... | AGYNWN--<br>...............--<br>N.... | ......<br>..... |
| | | Germline | H_FR1 | H_CDR1 | H_FR2 | H_CDR2 | H_FR3 | H_CDR3 | H_FR4 |
| | VH3\|3-21/D6\|6-6\|RF2/JH4 | | EVQLVES-GGGLVKPGGSLR LSCAASG-FIFS | S-----YSMN | WVRQAPGK GLEWVS | SISSS--- SSYIYYAD SVKG | RFTISRDNAKNSLYLQM NSLRAEDTAVYYCAR | SIAAR----------- --------YFDY | WGQGTL VTVSS |
| iPS:46 8844 | 21-225_48E10 | VH3\|3-21/D6\|6-6\|RF2/JH4 | .............<br>.............<br>........ | .......N.. | .........<br>......<br>.... | ...G....<br>.... | ...............<br>............... | .L---<br>...............<br>...--.L | ......<br>..... |
| iPS:43 5537 | 21-225_157H12 | VH3\|3-21/D6\|6-6\|RF2/JH4 | ...W........<br>........ | ......... | .........<br>......<br>.... | ...G....<br>G...N...<br>.... | ..........T.....V<br>.G............. | .KF--<br>...............<br>...--.S | ......<br>..... |
| iPS:43 5539 | 21-225_158G1 | VH3\|3-21/D6\|6-6\|RF2/JH4 | .............<br>.............<br>........ | .......G.. | .........<br>....I.<br>.... | ...G....<br>G.......<br>.... | ...............<br>..............I | .SG--<br>...............<br>...--WS | ......<br>..... |
| iPS:43 5583 | 21-225_160F2 | VH3\|3-21/D6\|6-6\|RF2/JH4 | .............<br>.............<br>........ | .......G.. | .........<br>....I.<br>.... | ...G....<br>G.......<br>.... | ...............<br>..............I | .SG--<br>...............<br>...--WS | ......<br>..... |
| | | Germline | H_FR1 | H_CDR1 | H_FR2 | H_CDR2 | H_FR3 | H_CDR3 | H_FR4 |
| | VH3\|3-21/D1\|1-1\|RF2/JH5 | | EVQLVES-GGGLVKPGGSLR LSCAASG-FIFS | S-----YSMN | WVRQAPGK GLEWVS | SISSS--- SSYIYYAD SVKG | RFTISRDNAKNSLYLQM NSLRAEDTAVYYCAR | VQLER----------- -------NWFDP | WGQGTL VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:46 8846 | 21-225_53B10 | VH3\|3-21/D1\|1-1\|RF2/JH5 | ............<br>............<br>........ | ......... | ........<br>......<br>.... | ...G....<br>......V.<br>.... | ................ | .NS--<br>....................<br>..--..S | ......<br>..... |
| iPS:43 4251 | 21-225_62G3 | VH3\|3-21/D1\|1-1\|RF2/JH5 | ............<br>............<br>........ | ......... | ........<br>......<br>.... | ........<br>........<br>.... | ................ | .NS--<br>....................<br>..--..S | ......<br>..... |
| iPS:43 4407 | 21-225_68G8 | VH3\|3-21/D1\|1-1\|RF2/JH5 | ............<br>............<br>........ | ......... | ........<br>......<br>.... | ...G....<br>........<br>..M. | ................ | .NS--<br>....................<br>..--..S | ......<br>..... |
| iPS:43 5575 | 21-225_159H11 | VH3\|3-21/D1\|1-1\|RF2/JH5 | ............<br>............<br>........ | .......T... | ........<br>......<br>.... | ...G....<br>........<br>.... | ................ | .SW--<br>....................<br>..--A.C | ......<br>..... |
| | | Germline | H_FR1 | H_CDR1 | H_FR2 | H_CDR2 | H_FR3 | H_CDR3 | H_FR4 |
| | VH3\|3-23/D1\|1-1\|RF2/JH4 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | VQLER----------YFDY | WGQGTLVTVSS |
| iPS:46 8848 | 21-225_54B1 | VH3\|3-23\|1-1\|RF2/JH4 | ............<br>............<br>........ | ......... | ........<br>......<br>.... | VL......<br>....F...<br>.... | ......E..........<br>S............R | RGR.YSG...........<br>......YD.... | ......<br>..... |
| iPS:43 3993 | 21-225_47G7 | VH3\|3-23\|1-1\|RF2/JH4 | .....D......<br>............<br>........ | ......... | ........<br>......<br>.... | ....R...<br>..N.F..E<br>..R. | ................ | IIR.Q.............<br>.......WA... | ......<br>..... |
| iPS:43 4007 | 21-225_48D7 | VH3\|3-23\|1-1\|RF2/JH4 | ............<br>............<br>.......R | N.....S..N | ........<br>......<br>.... | ........<br>..T.F...<br>.... | ................I | CGR.Q.............<br>.......WL.. | ......<br>..... |
| iPS:43 4115 | 21-225_53E4 | VH3\|3-23\|1-1\|RF2/JH4 | ............<br>............<br>........ | .......V.. | ........<br>......<br>.... | G.......<br>..R.....<br>.... | ..N.............R | .A.--<br>...-... | ......<br>..... |
| iPS:43 5679 | 21-225_169D10 | VH3\|3-23\|1-1\|RF2/JH4 | ............<br>..S.........<br>........ | .......V.. | ........<br>......<br>.... | ........<br>.SRI....<br>.... | .........L....R | .AF--<br>...-... | ......<br>..... |
| iPS:43 5685 | 21-225_170E1 | VH3\|3-23\|1-1\|RF2/JH4 | ............<br>............<br>........ | .......V.. | ........<br>......<br>.... | ........<br>.NRI....<br>.... | .........L....R | .AF--<br>...-... | ......<br>..... |

| iPS ID | Clone | Germline | H_FR1 | H_CDR1 | H_FR2 | H_CDR2 | H_FR3 | H_CDR3 | H_FR4 |
|---|---|---|---|---|---|---|---|---|---|
| iPS:43 6632 | 21-225_227E4 | VH3\|3-23/D1\|1-1\|RF2/JH4 | .G.......... ...........T ........ | T.....F..T | ........R ...... | V...R... ...SF... .... | ........T........ ............... | D..W- ................. ...-... | ...... ..... |
| **Germline** | | | **H_FR1** | **H_CDR1** | **H_FR2** | **H_CDR2** | **H_FR3** | **H_CDR3** | **H_FR4** |
| | **VH4\|4-39/D4\|4-11\|RF2/JH5** | | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | DYSNY-----------------NWFDP | WGQGTLVTVSS |
| iPS:46 8856 | 21-225_77C9 | VH4\|4-39/D4\|4-11\|RF2/JH5 | .T.......... ........ | R......... | ........ ...... | ...A.S.. .... | ................LF.... | LD..W............. ........-GL.Y | ...... ..... |
| iPS:43 4489 | 21-225_74E4 | VH4\|4-39/D4\|4-11\|RF2/JH5 | ...........I.. ........ | .....N.... | ........ ...... | ..Y.S... .... | .......S...H...R. ................ | LD..W............. ........-GL.Y | ...... ..... |
| iPS:43 5251 | 21-225_96A3 | VH4\|4-39/D4\|4-11\|RF2/JH5 | ...........I.. ........ | .....N.... | ........ ...... | ..Y.S... .... | .......S...H...R. ................ | LD..W............. ........-GL.Y | ...... ..... |
| iPS:43 7346 | 21-225_75H7 | VH4\|4-39/D4\|4-11\|RF2/JH5 | .T.......... ........ | R......... | ........ ...... | ...A.S.. .... | .........LF.... | LD..W............. ........-GL.Y | ...... ..... |
| iPS:39 3886 | 21-225_2G9 | VH4\|4-39/D4\|4-11\|RF2/JH5 | ........... ........R | PN...-.... | ........ ...... | ....S... ..N. | N.............. | LS..W............. ........-D..N | ...... ..... |
| iPS:39 3928 | 21-225_4E10 | VH4\|4-39/D4\|4-11\|RF2/JH5 | ........... ........... | R......... | ........ ...... | .V...... ..A.S... | N........L...V. | LS..W............. ........-D..Y | F.... |
| **Germline** | | | **H_FR1** | **H_CDR1** | **H_FR2** | **H_CDR2** | **H_FR3** | **H_CDR3** | **H_FR4** |
| | **VH1\|1-02/D6\|6-6\|RF1/JH6** | | QVQLVQSGAEVKKPGASVKVSCKASG-YIFT | G-------YYMH | WVRQAPGQGLEWMG | WINPNSGGTNYAQKFQG | RVTMTRDTSISTAYMELSRLRSDDTAVYYCAR | EYSSSSYY------YYYGMDV | WGQGTTVTVSS |
| iPS:46 8862 | 21-225_178H8 | VH1\|1-02/D6\|6-6\|RF1/JH6 | ...........RT.......... ........ | D......... | ........ ...... | R....... .... | ................ | .EDR.GW........... ............ | ...... ..... |
| iPS:45 1112 | 21-225_53D10 | VH1\|1-02/D6\|6-6\|RF1/JH6 | ........... .....I.. | ........I. | ........ ...... | ........ .... | I.............. | .NE.LATRP......... ...FYD...... | ...... ..... |
| **Germline** | | | **H_FR1** | **H_CDR1** | **H_FR2** | **H_CDR2** | **H_FR3** | **H_CDR3** | **H_FR4** |

| VH2\|2-05/D6\|6-6\|RF2/JH4 | | QITLKES-GPTLVKPTQILT LTCTFSG-FSLS | TS---GVGVG | WIRQPPGK ALEWLA | LIYW---- NDDKRYSP SLKS | RLTITKDTSKNQVVLTM CNMDPVDTATYYCAE | SIAAR------------ --------YFDY | WGQGTL VTVSS |
|---|---|---|---|---|---|---|---|---|
| iPS:46 8864 | 21-225_60D6 | VH2\|2-05/D6\|6-6\|RF2/JH 4 | ............ ............ ........ | .......... | ........ ...... | K..E.... .... | ................ | AV.V- ................... ...S... | ...... ..... |
| iPS:43 6850 | 21-225_57D9 | VH2\|2-05/D6\|6-6\|RF2/JH 4 | .........M. ............ ........ | .......... | ........ ...... | ........ .... | .....E.......... | AV.V- ................... ...S... | ...... ..... |
| iPS:43 6914 | 21-225_76B4 | VH2\|2-05/D6\|6-6\|RF2/JH 4 | ............ ............ ........ | .G........ | ........ ...... | ........ D....... .... | ........P........ | L..V- ................... ...A... | ...... ..... |
| iPS:43 6918 | 21-225_77A2 | VH2\|2-05/D6\|6-6\|RF2/JH 4 | .........S. ............ ........ | .......... | ........ .....V | F....... D....... .... | ................ | L..V- ................... ...A... | ...... ..... |
| iPS:43 6934 | 21-225_96B5 | VH2\|2-05/D6\|6-6\|RF2/JH 4 | ............ ............ ........ | .G........ | ........ ...... | ........ D....... .... | ........P........ | L..V- ................... ...AC.. | ...... ..... |
| iPS:43 7334 | 21-225_75F11 | VH2\|2-05/D6\|6-6\|RF2/JH 4 | ............ ............ ........ | .G........ | ........ ...... | ........ D....... .... | ........P........ | L..V- ................... ...A... | ...... ..... |
| iPS:43 7377 | 21-225_74G9 | VH2\|2-05/D6\|6-6\|RF2/JH 4 | ............ ............ ........ | .G........ | ........ ...... | ........ D....... .... | ........P........ | L..V- ................... ...A... | ...... ..... |
| iPS:39 2583 | 21-225_10B10 | VH2\|2-05/D6\|6-6\|RF2/JH 4 | ............ ............ ........ | .G........ | ........ .....V | F....... S....... .... | ..S............. ..............R | IA.V- ................... ...A... | ...... ..... |
| iPS:39 3184 | 21-225_15H11 | VH2\|2-05/D6\|6-6\|RF2/JH 4 | .........L.. M........... ........ | .G........ | ........ ...... | ........ H....... ..R. | ............... ..............R | IV.V- ................... ...A... | ...... I.... |
| iPS:39 3212 | 21-225_30H6 | VH2\|2-05/D6\|6-6\|RF2/JH 4 | ............ ............ ........ | .G........ | ........ ...... | ........ H....... .... | ..A.............I | L..V- ................... ...A... | ...... ..... |
| iPS:39 3222 | 21-225_17F5 | VH2\|2-05/D6\|6-6\|RF2/JH 4 | .........S. ............ ........ | .......... | ........ ...... | ........ D....... .... | .............S... | I..V- ................... ...A... | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:39 3224 | 21-225_31C2 | VH2\|2-05/D6\|6-6\|RF2/JH4 | ............ ............ .......N | .G........ | ........ ...... | ....... ...E.... .... | ................ .....L...S..... | L..V- ................ ...S... | ....A. ..... |
| | | Germline | H_FR1 | H_CDR1 | H_FR2 | H_CDR2 | H_FR3 | H_CDR3 | H_FR4 |
| | VH1\|1-02/D6\|6-19\|RF2/JH6 | | QVQLVQS-GAEVKKPGASVK VSCKASG-YTFT | G-----YYMH | WVRQAPGQ GLEWMG | WINPN--- SGGTNYAQ KFQG | RVTMTRDTSISTAYMEL SRLRSDDTAVYYCAR | GIAVAGYY-------------YYYGMDV | WGQGTT VTVSS |
| iPS:46 8866 | 21-225_190C1 | VH1\|1-02/D6\|6-19\|RF2/JH6 | ............ ............ ........ | .......... | ........ ...... .... | ....Y... ........ .... | ................ | DR....N............ ......F...... | ...... ..... |
| iPS:43 6972 | 21-225_190C7 | VH1\|1-02/D6\|6-19\|RF2/JH6 | ............ ............ ........ | .......... | ........ ...... .... | ........ ........ .... | ................ | DR....N............ ......F...... | ...... ..... |
| iPS:43 7020 | 21-225_193F11 | VH1\|1-02/D6\|6-19\|RF2/JH6 | ............ ............ ........ | .......... | ........ ...... | ....Y... .... | ................ | DR....N............ ......F...... | ...... ..... |
| iPS:43 7036 | 21-225_195H9 | VH1\|1-02/D6\|6-19\|RF2/JH6 | ............ ............ ........ | .......... | ........ ...... | ....Y... ...D | ..........T | DR....N............ ......F...... | ...... ..... |
| iPS:43 7042 | 21-225_197E8 | VH1\|1-02/D6\|6-19\|RF2/JH6 | ....L....... R......R.... ........ | ........I. | ........ ...... | ........ R... | ................ | E.....N............ .....F....G. | .A... |
| | | Germline | H_FR1 | H_CDR1 | H_FR2 | H_CDR2 | H_FR3 | H_CDR3 | H_FR4 |
| | VH4\|4-39/D1\|1-1\|RF2/JH4 | | QLQLQES-GPGLVKPSETLS LTCTVSG-GSIS | SS---SYYWG | WIRQPPGK GLEWIG | SIYY---- SGSTYYNP SLKS | RVTISVDTSKNQFSLKL SSVTAADTAVYYCAR | VQLER-------------YFDY | WGQGTL VTVSS |
| iPS:46 8868 | 21-225_74A1 | VH4\|4-39/D1\|1-1\|RF2/JH4 | ............ ............ ........ | G........ | ........ ...... | N....... .....H.. .... | T........F.... | HD.LW............. ........SL.F | ....I. ..... |
| | | Germline | H_FR1 | H_CDR1 | H_FR2 | H_CDR2 | H_FR3 | H_CDR3 | H_FR4 |
| | VH1\|1-02/D3\|3-22\|RF2/JH5 | | QVQLVQS-GAEVKKPGASVK VSCKASG-YTFT | G-----YYMH | WVRQAPGQ GLEWMG | WINPN--- SGGTNYAQ KFQG | RVTMTRDTSISTAYMEL SRLRSDDTAVYYCAR | YYYDSSGY-------------YYNWFDP | WGQGTL VTVSS |
| iPS:47 2742 | 21-225_30D9_LC2 | VH1\|1-02/D3\|3-22\|RF2/JH5 | ..K........ E........... ........ | ........L. | ........ ...... | ........ .... | ................ | V..YG..S........... ........E..N | ...... ..... |

3198

| ID | Clone | Germline | H_FR1 | H_CDR1 | H_FR2 | H_CDR2 | H_FR3 | H_CDR3 | H_FR4 |
|---|---|---|---|---|---|---|---|---|---|
| iPS:47 2741 | 21-225_30D9_LC1 | VH1\|1-02/D3\|3-22\|RF2/JH5 | ..K.........<br>E...........<br>........ | ........L. | .........<br>...... | .........<br>........<br>.... | .................<br>................. | V..YG..S..........<br>........Ξ..N | ......<br>..... |
| iPS:43 7040 | 21-225_196E7 | VH1\|1-02/D3\|3-22\|RF2/JH5 | ...........<br>...........<br>V....... | ........N.. | .........<br>...... | .........<br>.......H<br>.... | ................. | D...T..-<br>...............-<br>EG.... | ......<br>..... |
| iPS:43 7050 | 21-225_197C11 | VH1\|1-02/D3\|3-22\|RF2/JH5 | ...........<br>...........<br>V....... | ........N.. | .........<br>...... | .........<br>.......H<br>.... | ................. | D......-<br>...............-<br>EG.... | ......<br>..... |
| iPS:39 3214 | 21-225_33A1 | VH1\|1-02/D3\|3-22\|RF2/JH5 | ...........<br>...........<br>.......S | ......... | .........<br>...... | .........<br>N...H...<br>.... | .........R..S...<br>...........F... | G..YA..S..........<br>........DL.. | ......<br>..... |
| | **Germline** | **H_FR1** | **H_CDR1** | **H_FR2** | **H_CDR2** | **H_FR3** | **H_CDR3** | **H_FR4** | |
| | **VH1\|1-02/D3\|3-22\|RF2/JH4** | QVQLVQS-GAEVKKPGASVKVSCKASG-YIFT | G-----YYMH | WVRQAPGQGLEWMG | WINPN---SGGTNYAQKFQG | RVTMTRDTSISTAYMELSRLRSDDTAVYYCAR | YYYDSSG--------YYYYFDY | WGQGTLVTVSS | |
| iPS:47 2743 | 21-225_68G6 | VH1\|1-02/D3\|3-22\|RF2/JH4 | .....F..G..<br>....S.......<br>........ | .......... | .........<br>...... | S.YR....<br>.........<br>.... | ........K........K<br>..I............ | AF.YG..T..........<br>.......NE... | ......<br>..... |
| iPS:43 6902 | 21-225_69B11 | VH1\|1-02/D3\|3-22\|RF2/JH4 | ...........<br>.......R....<br>........ | .......... | .........<br>...... | .........<br>......G.<br>...D | .................F...<br>..........A..... | T..YG..S..........<br>.......NG... | ......<br>..... |
| iPS:43 6904 | 21-225_71D4 | VH1\|1-02/D3\|3-22\|RF2/JH4 | ...........<br>...........<br>........ | .......C.. | .........<br>...... | .........<br>........<br>...V | ..........V..V..D.<br>................. | A..YG..T..........<br>......HNE... | ....S.<br>..... |
| iPS:43 6906 | 21-225_72B4 | VH1\|1-02/D3\|3-22\|RF2/JH4 | ...........<br>...........<br>....... | .......... | .........<br>...... | .........<br>......G.<br>.... | ................. | T..YG..S..........<br>.......NG... | ......<br>..... |
| iPS:43 7034 | 21-225_195E9 | VH1\|1-02/D3\|3-22\|RF2/JH4 | ...V.......<br>...........<br>........ | .......... | .........<br>...... | .........<br>..A.....<br>.... | N.............. | A..YG..T..........<br>.......NE... | ......<br>..... |
| iPS:39 2598 | 21-225_18E10 | VH1\|1-02/D3\|3-22\|RF2/JH4 | ...........<br>...........<br>........ | .......... | .........<br>...... | .........<br>........<br>.... | .......S..N......<br>................. | S..YG..S..........<br>.......NE... | ......<br>..... |

| | | | H_FR1 | H_CDR1 | H_FR2 | H_CDR2 | H_FR3 | H_CDR3 | H_FR4 |
|---|---|---|---|---|---|---|---|---|---|
| IPS:39 3182 | 21-225_4B3 | VH1\|1-02\|D3\|3-22\|RF2\|JH4 | ..............<br>..............<br>........ | .......... | ........ .<br>...... | ........<br>.....S..<br>.... | ............... | S..YG..S...........<br>.......NE... | ......<br>..... |
| IPS:39 3200 | 21-225_35E1 | VH1\|1-02\|D3\|3-22\|RF2\|JH4 | ..K..........<br>..............<br>........ | .......... | ........ .<br>...... | ....K...<br>........<br>.... | ...............V...P | V..HG..S...........<br>.......NE... | ......<br>..... |
| IPS:39 3206 | 21-225_13F6 | VH1\|1-02\|D3\|3-22\|RF2\|JH4 | ..............<br>..............<br>........ | ,......... | ........ .<br>...... | ........<br>...A....<br>.... | ..........F... | SF.YG..T...........<br>.......NE... | ......<br>..... |
| IPS:39 3208 | 21-225_16F3 | VH1\|1-02\|D3\|3-22\|RF2\|JH4 | ..............<br>..............<br>........ | ,....H,.. | ........ .<br>...... | ........<br>........<br>.... | ............... | S..YG..T...........<br>.......NE... | ......<br>..... |
| IPS:39 3210 | 21-225_17D3 | VH1\|1-02\|D3\|3-22\|RF2\|JH4 | ..............<br>..............<br>........ | .......... | ........ .<br>...... | ........<br>........<br>.... | ............... | AN.YG..S...........<br>.......ND... | ......<br>..... |
| IPS:39 3226 | 21-225_33E6 | VH1\|1-02\|D3\|3-22\|RF2\|JH4 | ..K..........<br>..............<br>........ | .......... | ........ .<br>...... | ........<br>........<br>.... | ............... | V..YG..S...........<br>.......NE... | ......<br>..... |
| IPS:39 3230 | 21-225_9G9 | VH1\|1-02\|D3\|3-22\|RF2\|JH4 | ..............<br>..............<br>........ | .........I. | ........ .<br>...... | ........<br>....D...<br>.... | ............... | S..YG..T...........<br>.......NE... | ......<br>..... |
| IPS:39 8490 | 21-225_21D12 | VH1\|1-02\|D3\|3-22\|RF2\|JH4 | ..............<br>..............<br>........ | D........I. | ........ .<br>...... | ........<br>.....N..<br>.... | ............S... | S..YG..T...........<br>.......NE... | ......<br>..... |
| IPS:42 3018 | 21-225_31D12_LC2 | VH1\|1-02\|D3\|3-22\|RF2\|JH4 | ..K..........<br>..............<br>........ | .......... | ........ .<br>...... | ........<br>......V.<br>.... | ............... | V..YG..S...........<br>.......NE... | ......<br>..... |
| IPS:42 3019 | 21-225_31D12_LC1 | VH1\|1-02\|D3\|3-22\|RF2\|JH4 | ..K..........<br>..............<br>........ | .......... | ........ .<br>...... | ........<br>......V.<br>.... | ............... | V..YG..S...........<br>.......NE... | ......<br>..... |
| Germline | VH3\|3-33\|D7\|7-27\|RF2\|JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LGY-----------FDY | WGQGTLVTVSS |

| iPS:39 2920 | 21-225_29G4 | VH3\|3-33/D7\|7-27\|RF2\|JH4 | ............ ........I.. ........ | D.......I. | ........ ...... | ....... E....... .M.. | ................ ................ | E..M............ ........IG.. | ...... .... |
| iPS:43 3899 | 21-225_43C3 | VH3\|3-33/D7\|7-27\|RF2\|JH4 | ............ ............ ........ | ........I. | ........ ...... | ....... EN...... .... | ................ ................ | E..F............ ........SN.. | ...... .... |
| iPS:43 3921 | 21-225_44C3 | VH3\|3-33/D7\|7-27\|RF2\|JH4 | ............ ............ ........ | ........ | ........ ...... | ...FE... ........ .... | ................ S............V. | E..F............ ........ST.. | ...... .... |
| iPS:43 3933 | 21-225_44C8 | VH3\|3-33/D7\|7-27\|RF2\|JH4 | ............ ............ .......N | N......... | ........ ...... | ....E... ........ .... | ................ .............V. | E..F............ ........LS.. | ...... .... |
| iPS:43 3969 | 21-225_46F3 | VH3\|3-33/D7\|7-27\|RF2\|JH4 | ............ ............ ........ | D......... | ........ ...... | ...FE... ........ .... | ............V.. .............V. | E..F............ ........SN.. | ...... .... |
| iPS:43 3975 | 21-225_46C6 | VH3\|3-33/D7\|7-27\|RF2\|JH4 | ............ ............ ........ | ........I. | ........ ...... | ........ EN...... .... | ................ ................ | E..F............ ........SN.. | ...... .... |
| iPS:43 3977 | 21-225_46D8 | VH3\|3-33/D7\|7-27\|RF2\|JH4 | ............ ............ ........ | D......I. | ........ ...... | ...FE... ........ .... | ............V.. .............V. | E..F............ ........SN.. | ...... .... |
| iPS:43 3983 | 21-225_47A1 | VH3\|3-33/D7\|7-27\|RF2\|JH4 | ............ ............ ........ | D......... | ........ ...... | ........ DY..K... .... | .........A......V ....V.........T | E..M............ ........L... | ...... .... |
| iPS:43 3997 | 21-225_48C1 | VH3\|3-33/D7\|7-27\|RF2\|JH4 | ............ ............ ........ | ........ | ........ ...... | .V...... EI..K... .... | .V.............. .........M..... | E..W............ ........EA.. | ...... .... |
| iPS:43 4009 | 21-225_48A9 | VH3\|3-33/D7\|7-27\|RF2\|JH4 | ............ ............ ........ | ........ | ........ ...... | ....E... ENK..... .... | ............F... .........M.F... | E.AW............ ........YE.. | ...... .... |
| iPS:43 4013 | 21-225_48D12 | VH3\|3-33/D7\|7-27\|RF2\|JH4 | ............ ............ ........ | D......... | .......R ...... | ........ V.....V. .... | ................ ................ | E..M............ ........RS.. | ...... .... |
| iPS:43 4019 | 21-225_49A1 | VH3\|3-33/D7\|7-27\|RF2\|JH4 | ............ ............ ........ | D......... | ........ ...... | ........ ED....V. .... | ................ ................ | E..F............ ........LS.. | ...... .... |

| | | | | | | | | | |
|---|---|---|---|---|---|---|---|---|---|
| IPS:43 4029 | 21-225_49C6 | VH3\|3-33/D7\|7-27\|RF2/JH4 | ............ ............ ........ | N......... | ........ ...... .... | ...F.... V...K.V. | ................ S............... | D..M............... .........IE.. | ...... ..... |
| IPS:43 4057 | 21-225_51E4 | VH3\|3-33/D7\|7-27\|RF2/JH4 | ............ ............ ........ | | ........ ...... .... | ...F.... E....... | ................ ................ | E..F............... .........LS.. | ...... ..... |
| IPS:43 4071 | 21-225_51F9 | VH3\|3-33/D7\|7-27\|RF2/JH4 | ............ ............ ........ | D......... | ........ ...... .... | ...F.... .N...... | ................ ...S........... | E..F............... .........LS.. | ...... ..... |
| IPS:43 4075 | 21-225_51B11 | VH3\|3-33/D7\|7-27\|RF2/JH4 | ............ ............ ........ | D......... | ........ ...... .... | ...FG... .N....G. | ................ ...S........... | E..F............... .........LS.. | ...... ..... |
| IPS:43 4077 | 21-225_51F11 | VH3\|3-33/D7\|7-27\|RF2/JH4 | ............ ............ ........ | N.....F... | ........ ...... .... | ....E... E....... | ................ ................ | E..F............... ........LS.F | ...... ..... |
| IPS:43 4081 | 21-225_52B2 | VH3\|3-33/D7\|7-27\|RF2/JH4 | ............ ............ ........ | N......... | ........ ...... .... | .T.F.... ...QR... | .......I......... ...S........... | D..M............... ........IE.F | ...... ..... |
| IPS:43 4091 | 21-225_52B9 | VH3\|3-33/D7\|7-27\|RF2/JH4 | ............ ............ ........ | D......... | ........ ...... .... | ...F.... .N...... | ................ ...S........... | E..F............... .........LS.. | ...... ..... |
| IPS:43 4105 | 21-225_53D2 | VH3\|3-33/D7\|7-27\|RF2/JH4 | ............ ............ ........ | N......... | ........ .....T .... | .V.D.... ........ | ................ ...............T | G..F............... .........TG.. | ....A. ..... |
| IPS:43 4119 | 21-225_53E6 | VH3\|3-33/D7\|7-27\|RF2/JH4 | ............ ...........T T....... | D.......I. | ........ ...... .... | ........ E.....G. | ..A.............. ..............V. | E..M............... .........TS.. | ...... ..... |
| IPS:43 4129 | 21-225_53B12 | VH3\|3-33/D7\|7-27\|RF2/JH4 | ............ ............ ........ | N.....F... | ........ ...... .... | .V...... .N.R.... | H............... | E..F............... .........LS.F | ...... ..... |
| IPS:43 4131 | 21-225_54D3 | VH3\|3-33/D7\|7-27\|RF2/JH4 | ............ ............ ........ | N......... | ........ ...... .... | .T.F.... .N.N.... | ................ | E..F............... .........LS.. | ...... ..... |
| IPS:43 4141 | 21-225_54C6 | VH3\|3-33/D7\|7-27\|RF2/JH4 | ............ ...........T T....... | D.......I. | ........ ..D... .... | ........ EN...... | ..A.............. ..............V. | E..M............... .........TS.. | ...... ..... |

| | | | | | | | |
|---|---|---|---|---|---|---|---|
| iPS:43 4143 | 21-225_54G7 | VH3\|3-33/D7\|7-27\|RF2/JH4 | . . . . . . . . . . . . . . . . . . . . . . . . . . . . . . . | N . . . . . . . . . | . . . . . . . . . . . . . . . . . . . . . . | . . . . E . . . E . . . . . G . . . . . | . . . . . . . . . . . . . . . . . . . . . . . . . . . . . . . . | E . . F . . . . . . . . . . . . . . . . . . . . L S . . | . . . . . . . . . . . |
| iPS:43 4155 | 21-225_55B3 | VH3\|3-33/D7\|7-27\|RF2/JH4 | . . . . . . . . . . . . . . . . . . . . . . . . . . . . . . | . . . . . . . . . | . . . . . . . . . . . . . . . . | . . . F . . . . . N . . . . E . . . . | . . . . . . . . . . . . . . . . . . . . . . V . | E . . F . . . . . . . . . . . . . . . . . . . . . L S . . | . . . . . . . . . . |
| iPS:43 4199 | 21-225_59F11 | VH3\|3-33/D7\|7-27\|RF2/JH4 | . . . . . . . . . . . . . . . . . . . . . . . . . . . . . . | N . . . . . . . . | . . . . . . . . . . . . . . . | . . . . . . . E . . . H . . . . . . . | . . . . . . . . . T . . . . . . S . . . . . . . . . . . . S . | E . M . . . . . . . . . . . . . . . . . . . . . N G . . | . . . . . . . . . . |
| iPS:43 4207 | 21-225_60A3 | VH3\|3-33/D7\|7-27\|RF2/JH4 | . . . . . . . . . . . . . . . . . . . . . . . . . . . . . . | . . . . . . . . . | . . . . . . . . . . . . . . . | . . . . E . . . E . . . . . . . . . . | . . . . . . . . . . . . . . . . . . . . . . . . | E . M . . . . . . . . . . . . . . . . . . . . . T G . . | . . . . . . . . . . |
| iPS:43 4253 | 21-225_62E4 | VH3\|3-33/D7\|7-27\|RF2/JH4 | . . . . . . . . . . . . . . . . . . . . . . . . . . . . . . | D . . . . . . . . | . . . . . . . . . . . . . . . | . . . . R . . . . . . . . . . . . | . . . . V . . . . . . H . . . . . . | E . . F . . . . . . . . . . . . . . . . . . . . . S S . . | . . . . . . . . . . |
| iPS:43 4271 | 21-225_57A4 | VH3\|3-33/D7\|7-27\|RF2/JH4 | . . . . . . . . . . . . . . . . . . . . . . V . . . . . . . | D . . . . . . . . | . . . . . . . . . . . . . . | . . . A . . . . . . . . V . . . . . | . . . . . . . . . . . . . . . . . . . | E . M . . . . . . . . . . . . . . . . . . . . . R S . . | . . . . . . . . . |
| iPS:43 4293 | 21-225_58F5 | VH3\|3-33/D7\|7-27\|RF2/JH4 | . . . . . . . . . . . . . . . . . . . . . . V . . . . . . . | D . . . . . . . . | . . . . . . . . . . . . . . | . . . A . . . . . . . H V . . . . | E . M . . . . . . . . . . . . . . . . . . . . . R S . . | . . . . . . . . . |
| iPS:43 4337 | 21-225_84E1 | VH3\|3-33/D7\|7-27\|RF2/JH4 | . . . . . . . . . . . . . . . . . . . . . . V . . . . . . . | . . . . . . . . . | . . . . . . . . . . . . . . | . . . F . . . . E T . . . . G . . . . | . . . . . . . . . . . . . . . . . . . . | E . . F . . . . . . . . . . . . . . . . . . . . . S S . . | . . . . . . . . . |
| iPS:43 4357 | 21-225_85C1 | VH3\|3-33/D7\|7-27\|RF2/JH4 | . . . . . . . . . . . . . . . . . . . . . . . . . . . . . . | D . . . . . . . . | . . . . . . . . . . . . . . | . . . F E . . . . . . . H . T . . . . | . . . . . . . . . . . . . . . . . . . . | E . . F . . . . . . . . . . . . . . . . . . . . . S S . . | . . . . . . . . . |
| iPS:43 4375 | 21-225_66C7 | VH3\|3-33/D7\|7-27\|RF2/JH4 | . . . . . . . . . . . . . . . . . . . . . . V . . . . . . . | D . . . . . . . . | . . . . . . . . . . . . . . | . . . F E . . . . . H . . . . T . . . | . . . . . . . . . . . . . . . . . . . . | E . . F . . . . . . . . . . . . . . . . . . . . . S S . . | . . . . . . . . . |
| iPS:43 4411 | 21-225_68F11 | VH3\|3-33/D7\|7-27\|RF2/JH4 | . . . . . . . . . . . . . . . . . . . . . . . . . . . . . . | D . . . . . . . . | . . . . . . . . . . . . . . | V . . . . . . . . . . . . | . . . . . . . . . . . . . . . . . . . . V . | E . M . . . . . . . . . . . . . . . . . . . . T S . C | . . . . . . . . . |
| iPS:43 4441 | 21-225_71A2 | VH3\|3-33/D7\|7-27\|RF2/JH4 | . . . . . . . . . . . . . . . . . . . . . T . . . . . . . | D . . . . . . . . | . . . . . . . . . . . . . . | . . . . . E . . . . . . . . . . . | . . . . . . . . . . . . . . . . . . . . . . | E . . W . . . . . . . . . . . . . . . . . . . . Q D . . | . . . . . . . . . |

| iPS:43 4447 | 21-225_71B6 | VH3\|3-33/D7\|7-27\|RF2\|JH4 | ...........<br>...........<br>........ | N......... | ........<br>...... | ........<br>RT......<br>.... | ..............H...<br>................. | E..M..............<br>........LS.. | ......<br>..... |
| iPS:43 4453 | 21-225_71B11 | VH3\|3-33/D7\|7-27\|RF2\|JH4 | ...........<br>...........<br>........ | D......... | ........<br>...... | ........<br>RN....G.<br>.... | .................<br>................. | E..M..............<br>........LS.. | ......<br>..... |
| iPS:43 4457 | 21-225_72G12 | VH3\|3-33/D7\|7-27\|RF2\|JH4 | ...........<br>...........<br>........ | ......... | ......DT<br>...... | ...F....<br>E.......<br>.... | .................<br>................. | E..F..............<br>........SS.. | ......<br>..... |
| iPS:43 5311 | 21-225_146H9 | VH3\|3-33/D7\|7-27\|RF2\|JH4 | ...........<br>...........<br>.....S.. | ......... | ........<br>...... | ...F....<br>E...H.G.<br>.... | .................<br>................. | E..F..............<br>........LS.. | ......<br>..... |
| iPS:43 5511 | 21-225_157C3 | VH3\|3-33/D7\|7-27\|RF2\|JH4 | ...........<br>...........<br>........ | T......... | ........<br>...... | ........<br>VN......<br>.... | .................<br>................. | E..F..............<br>........LS.. | ......<br>..... |
| iPS:43 5533 | 21-225_157H8 | VH3\|3-33/D7\|7-27\|RF2\|JH4 | ...........<br>...........<br>........ | ......... | ........<br>...... | ........<br>VN......<br>.... | .................<br>................. | E..F..............<br>........LS.. | ......<br>..... |
| iPS:43 5551 | 21-225_158H6 | VH3\|3-33/D7\|7-27\|RF2\|JH4 | ...........<br>...........<br>V....... | ......... | ........<br>...... | ........<br>VT......<br>.... | ....D........V.<br>................. | E..W..............<br>........AE.. | ......<br>..... |
| iPS:43 5569 | 21-225_159C5 | VH3\|3-33/D7\|7-27\|RF2\|JH4 | ...........<br>...........<br>........ | ........I. | ........<br>...... | .V......<br>VN......<br>.... | .................<br>................. | E..F..............<br>........LS.. | ......<br>..... |
| iPS:43 6268 | 21-225_203B9 | VH3\|3-33/D7\|7-27\|RF2\|JH4 | ...........<br>...........<br>......L. | ......F... | ........<br>...... | ........<br>VN......<br>.... | ....P...........<br>................. | E..F..............<br>........LS.. | ....I.<br>..... |
| iPS:43 6328 | 21-225_207F12 | VH3\|3-33/D7\|7-27\|RF2\|JH4 | ...........<br>...........<br>........ | N......... | ........<br>...... | ........<br>RN....G.<br>.... | .................<br>................. | E..F..............<br>........L... | ......<br>..... |
| iPS:43 6556 | 21-225_224D10 | VH3\|3-33/D7\|7-27\|RF2\|JH4 | ..........L<br>...K.......<br>........ | ......... | ........<br>...... | ....E...<br>......V.<br>..R. | ..........S....<br>...........V. | E..F..............<br>........QS.. | ....P<br>..... |
| iPS:39 2618 | 21-225_16F10 | VH3\|3-33/D7\|7-27\|RF2\|JH4 | ...........<br>...........<br>........ | D......... | ........<br>...... | ........<br>.N....V.<br>.... | .................<br>................. | E.AW..............<br>........YE.. | ......<br>..... |

| | | | | | | | | | |
|---|---|---|---|---|---|---|---|---|---|
| iPS:39 2626 | 21-225_18A5 | VH3\|3-33/D7\|7-27\|RF2\|J H4 | ..........,<br>.........YT<br>........ | D......... | ........<br>...... | ...F....<br>.... | ................. | D..W...............<br>........TEE. | ...... |
| iPS:39 2630 | 21-225_20E5 | VH3\|3-33/D7\|7-27\|RF2\|J H4 | ..........<br>..........<br>........ | D......... | ........<br>...... | ....E...<br>EN.Q....<br>.... | ............... | E..F...............<br>........RS.. | ...... |
| iPS:39 2640 | 21-225_18A1 | VH3\|3-33/D7\|7-27\|RF2\|J H4 | ..........<br>..........<br>V....... | ......... | ........<br>...... | ....E...<br>EN....V.<br>.... | ...........S......<br>...........V. | E..F...............<br>........QS.. | ......P |
| iPS:39 2644 | 21-225_19E1 | VH3\|3-33/D7\|7-27\|RF2\|J H4 | ..........<br>..........<br>........ | N......... | ........<br>...... | ....E...<br>EN.Q....<br>.... | ............... | E..F...............<br>........RS.. | ...... |
| iPS:39 2654 | 21-225_17A10 | VH3\|3-33/D7\|7-27\|RF2\|J H4 | ..........<br>..........<br>........ | N......... | ......A.<br>...... | ....E...<br>EN.Q....<br>.... | ............... | E..F...............<br>........RS.. | ...... |
| iPS:39 2658 | 21-225_18E8 | VH3\|3-33/D7\|7-27\|RF2\|J H4 | ..........<br>..........<br>........ | N......... | ........<br>...... | ....E...<br>EN.Q....<br>.... | ...V.........F... | E..F...............<br>........RS.. | ...... |
| iPS:39 2666 | 21-225_16F11 | VH3\|3-33/D7\|7-27\|RF2\|J H4 | ...M......<br>...........E<br>........ | ......... | ........<br>...... | ....E...<br>EN....V.<br>.... | ...........S......<br>...........V. | E..F...............<br>........QS.. | ......P |
| iPS:39 2674 | 21-225_18C2 | VH3\|3-33/D7\|7-27\|RF2\|J H4 | ..........<br>..........V<br>........ | D......... | ........<br>....M. | VT......<br>.... | ...S........... | E..W...............<br>........YE.. | ...... |
| iPS:39 2680 | 21-225_20A7 | VH3\|3-33/D7\|7-27\|RF2\|J H4 | ..........,<br>..........<br>........ | N......... | ........<br>...... | ....E...<br>EN.Q....<br>.... | .........I..... | E..F...............<br>........RS.. | ...... |
| iPS:39 2686 | 21-225_17C7 | VH3\|3-33/D7\|7-27\|RF2\|J H4 | ..........<br>...K......<br>........ | D......... | ........<br>...... | ...F....<br>.... | .......G....... | D..W...............<br>........TEE. | ...... |
| iPS:39 2690 | 21-225_18F2 | VH3\|3-33/D7\|7-27\|RF2\|J H4 | ..........<br>..........,<br>........ | D......... | ........<br>...... | ...F....<br>......V.<br>.... | ....V........... | D..W...............<br>........TEE. | ...... |
| iPS:39 2716 | 21-225_17B5 | VH3\|3-33/D7\|7-27\|RF2\|J H4 | ..........<br>..........,<br>........ | D......... | ........<br>...... | E...H.I.<br>.... | ............V. | E..F...............<br>........R... | ...... |

| | | | | | | | | |
|---|---|---|---|---|---|---|---|---|
| IPS:39 2732 | 21-225_17E5 | VH3|3-33/D7|7-27|RF2/JH4 | ........... ............ ........ | D......... | ........ ...... .... | L....... VT....G. .... | .........Q......L ................. | E..W............. ........YE.. | ..... .... |
| IPS:39 2744 | 21-225_20D5 | VH3|3-33/D7|7-27|RF2/JH4 | ........... ............ ........ | .......... | ........ ...... .... | ....E... EN.Q.... .... | ................. .....D......... | E..F............. ........RS.. | ..... |
| IPS:39 2758 | 21-225_21G11 | VH3|3-33/D7|7-27|RF2/JH4 | ........... ............ ........ | D......... | ........ ....M. .... | ......... VT.E.... .... | ................. ................. | E..W............. ........YE.. | ..... |
| IPS:39 2772 | 21-225_20E12 | VH3|3-33/D7|7-27|RF2/JH4 | ........... ............ ........ | .......... | ........ ...... .... | .M...... E...H... .... | .........R....... ................. | E..F............. ........R... | ..... |
| IPS:39 2790 | 21-225_20D10 | VH3|3-33/D7|7-27|RF2/JH4 | ........... ............ ........ | D......... | ........ ...... .... | ...F.... ......V. .... | ........D......... ................. | D..W............. ........TEE. | ..... |
| IPS:39 2796 | 21-225_22A4 | VH3|3-33/D7|7-27|RF2/JH4 | ........... ............ ........ | D.......I. | ........ ...... .... | ...F.... ......... .... | .I............... ................. | D..W............. ........TEE. | ..... |
| IPS:39 2810 | 21-225_20H12 | VH3|3-33/D7|7-27|RF2/JH4 | ........... ............ ........ | N......... | ........ ...... .... | ........ EN....V. .... | ................. ................. | E..F............. ........RS.. | ..... |
| IPS:39 2832 | 21-225_21H8 | VH3|3-33/D7|7-27|RF2/JH4 | ........... ..S....... ........ | D......... | ........ ...... .... | ...F.... ......... .... | ................. ................. | D..W............. ........TEE. | ..... |
| IPS:39 2854 | 21-225_21E5 | VH3|3-33/D7|7-27|RF2/JH4 | ........... ..........S ........ | D......... | ........ ...... .... | ........ E........ .... | ................. .........M...T. | E..F............. ........RS.. | ..... |
| IPS:39 2860 | 21-225_22H8 | VH3|3-33/D7|7-27|RF2/JH4 | ........... ............ ....... | .......... | ........ ...... .... | ........ .N...... .... | ................. ................. | E.AW............. ........YE.. | ..... |
| IPS:39 2866 | 21-225_23H11 | VH3|3-33/D7|7-27|RF2/JH4 | .E......... ............ ........ | .......... | ........ ...... .... | ........ EN....V. .... | ................. ................. | E..F............. ........RS.. | ..... |
| IPS:39 2876 | 21-225_21F7 | VH3|3-33/D7|7-27|RF2/JH4 | ........... ............ ........ | D......... | ........ ...... .... | ...F.... .N....V. .... | ................. ................. | D..W............. ........TEE. | ..... |

| | | | | | | | | | |
|---|---|---|---|---|---|---|---|---|---|
| iPS:39 2880 | 21-225_22F9 | VH3\|3-33/D7\|7-27\|RF2/J H4 | ...M......... ............ ......... | ........... | ......... ...... | ....E... EN..D.V. .... | ..........S...... ............V. | E..F............... .........QS.. | ....P ..... |
| iPS:39 2894 | 21-225_21G2 | VH3\|3-33/D7\|7-27\|RF2/J H4 | ............ ............ ........ | D.......... | ......... ....M. | ......... VT...... .... | ................E. ............... | E..W............... .........YE.. | ..... ..... |
| iPS:39 2900 | 21-225_22F2 | VH3\|3-33/D7\|7-27\|RF2/J H4 | ............ ............ ........ | D.......... | ......... ...... | ....E... ......V. ..R. | ..........S...... ............V. | E..F............... .........QS.. | ....P ..... |
| iPS:39 2908 | 21-225_23F12 | VH3\|3-33/D7\|7-27\|RF2/J H4 | ............ ..........V ........ | ........... | ......... ...... | ......... ET...... .... | ............... ..........I..... | E.AW............... .........YE.. | ....S. ..... |
| iPS:39 2918 | 21-225_28F5 | VH3\|3-33/D7\|7-27\|RF2/J H4 | ............ ............ ........ | ........... | ......... ...... | ......... EN...... .... | ............... ............... | E..W............... .........YD.. | ..... ..... |
| iPS:39 2934 | 21-225_27D5 | VH3\|3-33/D7\|7-27\|RF2/J H4 | ............ ............ ........ | ........... | ......... ...... | ......... E.....G. .... | .........M..... ....G....L..... | E..F............... .........LS.. | ..... ..... |
| iPS:39 2958 | 21-225_28C7 | VH3\|3-33/D7\|7-27\|RF2/J H4 | ............ ............ ........ | ........... | ......... ...... | ......... E....... .... | ............... ............... | E..W............... .........YD.. | ..... ..... |
| iPS:39 2968 | 21-225_25B6 | VH3\|3-33/D7\|7-27\|RF2/J H4 | ..........V..T ......LR | N.......... | ......... ...... | ....E... E.....TE .... | ............... ............... | E..F............... .........LS.. | ..... ..... |
| iPS:39 2972 | 21-225_26A2 | VH3\|3-33/D7\|7-27\|RF2/J H4 | ............ ............ ........ | ........... | ......... ...... | ....E... ......V. .... | ............... ............... | E..W............... .........YD.. | ..... ..... |
| iPS:39 2980 | 21-225_29H6 | VH3\|3-33/D7\|7-27\|RF2/J H4 | ............ ............ ........ | D.......... | ......... .....T | ....N... EN...... .... | ............... ............... | E..M............... .........TG.S | ..... ..... |
| iPS:39 2988 | 21-225_25E6 | VH3\|3-33/D7\|7-27\|RF2/J H4 | ............ ............ ........ | D.......... | ......... ...... | ......... EN...... .... | ............... ..........L....T | E..M............... .........TG.S | ..... ..... |
| iPS:39 2990 | 21-225_25H10 | VH3\|3-33/D7\|7-27\|RF2/J H4 | ............ ............ ........ | D.......... | ......... ...... | ......... E....... .M.. | ............... ............... | E..M............... .........TG.. | ..... ..... |

| | | | | | | | | |
|---|---|---|---|---|---|---|---|---|
| IPS:39 3000 | 21-225_29D7 | VH3\|3-33/D7\|7-27\|RF2/J H4 | . . . . . . . . . . . .<br>. . . . . . . . . . .<br>. . . . . . . . | . . . . . . . . . | . . . . . . . .<br>. . . . . . | . . . . . . .<br>E . . . . . G .<br>. . . . | . . . . . . . . . . . . . . . .<br>. . . . G . . . . L . . . . . | E . . F . . . . . . . . . . . . .<br>. . . . . . . . LS . . | . . . . . |
| IPS:39 3018 | 21-225_29B8 | VH3\|3-33/D7\|7-27\|RF2/J H4 | . . . . . . . . . . . .<br>. . . . . . . . . . .<br>. . . . . . . . | D . . . . . . . . . | . . . . . . . .<br>. . . . . . | . . . . . . .<br>EN . . . . . .<br>. . . . | . . . . . . . . . . . . . . . . | E . . M . . . . . . . . . . . . .<br>. . . . . . . . TG . S | . . . . . |
| IPS:39 3030 | 21-225_25H11 | VH3\|3-33/D7\|7-27\|RF2/J H4 | . . . . . . . . . . . .<br>. . . . . . . . . . .<br>. . . . . . . . | D . . . . . . . . . | . . . . . . . .<br>. . . . . . | . . . . . . .<br>EN . E . . . .<br>. . . . | . . . . . . . . . . . . . . . . | E . . M . . . . . . . . . . . . .<br>. . . . . . . . TG . S | . . . . . |
| IPS:39 3034 | 21-225_27F2 | VH3\|3-33/D7\|7-27\|RF2/J H4 | . E . . . . . . . . . .<br>. . . . . . . . . . . .<br>. . . . . I . . | D . . . . . . . . . | . . . . . . . .<br>. . . . . . | . . . . . . .<br>EN . . . . V .<br>. . R . | . . . . . . . . . . . . . . . . | E . . M . . . . . . . . . . . . .<br>. . . . . . . . TG . S | . . . . . |
| IPS:39 3048 | 21-225_27C3 | VH3\|3-33/D7\|7-27\|RF2/J H4 | . . . . . . . . . . . .<br>. . . . . . . . . . . .<br>. . . . . . . . | D . . . . . . . . . | . . . . . . . .<br>. . . . . . | . . . . . . .<br>EN . . S . . .<br>. . . . | . . . . . . . . . . . . . . . . | E . . M . . . . . . . . . . . . .<br>. . . . . . . . TG . S | . . . . . |
| IPS:39 3054 | 21-225_29G8 | VH3\|3-33/D7\|7-27\|RF2/J H4 | . . . . . . . . . . . .<br>. . . . . . . . . . . .<br>. . . . . . . . | D . . . . . . . . . | . . . . . . . .<br>. . . . . . | . . . . . . .<br>ET . . . . . .<br>. . . . | . . . . . . . . . . . . . . . . | E . . M . . . . . . . . . . . . .<br>. . . . . . . . TS . . | . . . . . |
| IPS:39 3812 | 21-225_6A11 | VH3\|3-33/D7\|7-27\|RF2/J H4 | . . . . . . . . . . . .<br>. . . . . . . . . . . .<br>. . . . . . . . | D . . . . . . . . . | . . . . . . . .<br>. . . . . . | . . . F . . . .<br>. . . . . . . .<br>. . . . | . . . . . . . . . . . . . . . .<br>. . . . . . . . . . D . . . | D . . W . . . . . . . . . . . . .<br>. . . . . . . . TEE . | . . . . . |
| IPS:39 3818 | 21-225_6G12 | VH3\|3-33/D7\|7-27\|RF2/J H4 | . . . . . . . . . . . .<br>. . . . . . . . . . .<br>. . . . . . . . | | . . . . . . . .<br>. . . . . . | . . . . . . .<br>R . . N . . . .<br>. . . . | . . . . . . . . . . . . . . . . | E . . F . . . . . . . . . . . . .<br>. . . . . . . . RS . . | . . . . . |
| IPS:39 3820 | 21-225_8H7 | VH3\|3-33/D7\|7-27\|RF2/J H4 | . . . . . . . . . . . .<br>. . . . . . . . . . . P<br>. . . . . . . . | D . . . . . F . . . | . . . . . . . .<br>. . . . . . | . . . . . E . .<br>EN . Q . . . .<br>. . . . | . . . . . . . . . . . . . . . . | E . . F . . . . . . . . . . . . .<br>. . . . . . . . RS . . | . . . . . |
| IPS:39 3826 | 21-225_10G5 | VH3\|3-33/D7\|7-27\|RF2/J H4 | . . . . . . . . . . . .<br>. . . . . . . . . . .<br>. . . . . . . . | N . . . . . . . . . | . . . . . . . .<br>. . . . . . | . . . . . E . .<br>EN . Q . . . .<br>. . . . | . . . . . . . . . . . . . . . .<br>. . . . . . . . . . . . . T . | E . . F . . . . . . . . . . . . .<br>. . . . . . . . RS . . | . . . . . |
| IPS:39 3828 | 21-225_10H12 | VH3\|3-33/D7\|7-27\|RF2/J H4 | . . . . . . . . . . . .<br>. . . . . . . . . . . .<br>. . . . . . . . | D . . . . . . . . . | . . . . . . . .<br>. . . . . . | . . . . . E . .<br>DN . Q . . . .<br>. . . . | . . . . . . . . . . . . . . . . | E . . F . . . . . . . . . . . . .<br>. . . . . . . . RS . . | . . . . . |
| IPS:39 3830 | 21-225_12A1 | VH3\|3-33/D7\|7-27\|RF2/J H4 | . . . . . . . . . . . .<br>. . . . . . . . . . .<br>. . . . . . . . | . . . . . . . . . | . . . . . . . .<br>. . . . . . | . . . . . E . .<br>EN . Q . . . .<br>. . . . | . . . . . . . . . . . . . . . . | E . . F . . . . . . . . . . . . .<br>. . . . . . . . RS . . | . . . . . |

| | | | | | | | | |
|---|---|---|---|---|---|---|---|---|
| IPS:39 3838 | 21-225_8G2 | VH3\|3-33\|D7\|7-27\|RF2\|JH4 | L............<br>....K.......<br>........ | D.......I.<br>......<br> | ........<br>......<br>.... | ...F....<br>........<br> | .....S...........<br>...............<br> | D..W..............<br>........TEE.<br> | ......<br>..... |
| IPS:39 3854 | 21-225_7H11 | VH3\|3-33\|D7\|7-27\|RF2\|JH4 | ............<br>...........S<br>........ | D.........<br>......<br> | ........<br>......<br>.... | .........<br>EN......<br>.... | ...............<br>...............<br> | E..F.............<br>.......RS..<br> | ......<br>..... |
| IPS:39 3866 | 21-225_14E3 | VH3\|3-33\|D7\|7-27\|RF2\|JH4 | ............<br>............<br>........ | D.....F....<br>......<br> | ........<br>......<br>.... | ....E...<br>EN.Q....<br>.... | ...............<br>.........F.....<br> | E..F.............<br>.......RS..<br> | ......<br>..... |
| IPS:39 3876 | 21-225_9A1 | VH3\|3-33\|D7\|7-27\|RF2\|JH4 | ...V.......<br>............<br>........ | D.........<br>......<br> | ........<br>......<br>.... | ...F....<br>........<br> | ...............<br>...............<br> | D..W..............<br>........TEE.<br> | .....P<br>..... |
| IPS:39 3882 | 21-225_15E3 | VH3\|3-33\|D7\|7-27\|RF2\|JH4 | .........T.<br>............<br>........ | ..........<br>......<br> | ........<br>......<br>.... | ....E...<br>EN..H...<br>.... | ...............<br>...............<br> | E..F.............<br>........LS..<br> | ......<br>..... |
| IPS:39 3884 | 21-225_16F4 | VH3\|3-33\|D7\|7-27\|RF2\|JH4 | ............<br>............<br>........ | N.........<br>......<br> | ........<br>......<br>.... | ....E...<br>...Q..G.<br>.... | ..........V....<br>H..............<br> | E..F.............<br>........LS..<br> | ......<br>..... |
| IPS:39 3912 | 21-225_16F6 | VH3\|3-33\|D7\|7-27\|RF2\|JH4 | ............<br>............<br>........ | N.........<br>......<br> | R.......<br>......<br>.... | ....E...<br>...Q..G.<br>.... | ..........V....<br>H..............<br> | E..F.............<br>........LS..<br> | ......<br>..... |
| IPS:39 3922 | 21-225_2B2 | VH3\|3-33\|D7\|7-27\|RF2\|JH4 | ............<br>............<br>........ | ..........<br>......<br> | ........<br>......<br>.... | ....E...<br>EN....V.<br>.... | ..........S......<br>..............V.<br> | E..F.............<br>........QS..<br> | .....P<br>..... |
| IPS:39 3934 | 21-225_13E6 | VH3\|3-33\|D7\|7-27\|RF2\|JH4 | ............<br>............<br>........ | N.........<br>......<br> | ........<br>......<br>.... | ....E...<br>EN....I.<br>.... | ...............<br>...............<br> | E..F.............<br>........RS..<br> | ......<br>..... |
| IPS:39 3948 | 21-225_16A5 | VH3\|3-33\|D7\|7-27\|RF2\|JH4 | ............<br>............<br>........ | D.........<br>......<br> | ........<br>......<br>.... | ...F....<br>......V.<br>.... | ...............<br>S...............<br> | D..W..............<br>........TEE.<br> | ......<br>..... |
| IPS:39 3960 | 21-225_7G2 | VH3\|3-33\|D7\|7-27\|RF2\|JH4 | ............<br>............<br>........ | D.........<br>......<br> | ........<br>....M.<br>.... | ........<br>VT......<br>.... | ...............<br>...............L | E..W.............<br>........YE..<br> | ......<br>..... |
| IPS:39 3974 | 21-225_7C4 | VH3\|3-33\|D7\|7-27\|RF2\|JH4 | ............<br>....K.......<br>........ | N.........<br>......<br> | ........<br>......<br>.... | ....E...<br>EN.Q....<br>.... | ...............<br>...............<br> | E..F.............<br>........RS..<br> | ......<br>..... |

| | | | H_FR1 | H_CDR1 | H_FR2 | H_CDR2 | H_FR3 | H_CDR3 | H_FR4 |
|---|---|---|---|---|---|---|---|---|---|
| iPS:39 3976 | 21-225_7E9 | VH3\|3-33\|D7\|7-27\|RF2\|JH4 | ..............<br>.............<br>........ | D......... | .........<br>....... | .......<br>EN....V.<br>.... | .................. | E..F...............<br>.........RS.. | ......<br>..... |
| iPS:39 3994 | 21-225_8C9 | VH3\|3-33\|D7\|7-27\|RF2\|JH4 | ............<br>............<br>........ | .......... | ........<br>...... | ........<br>EN....V.<br>.... | .................. | E..F...............<br>.........RS.. | ......<br>..... |
| iPS:39 3998 | 21-225_12B12 | VH3\|3-33\|D7\|7-27\|RF2\|JH4 | ............<br>............<br>........ | D......... | .........<br>....M. | ........<br>VT......<br>.... | .................. | E..W...............<br>.........YE.. | ......<br>..... |
| iPS:39 4024 | 21-225_16B7 | VH3\|3-33\|D7\|7-27\|RF2\|JH4 | ............<br>............<br>........ | ........... | ........<br>...... | ........<br>E.......<br>.... | .................. | E..F...............<br>.........LS.. | ......<br>..... |
| iPS:39 4059 | 21-225_9E8 | VH3\|3-33\|D7\|7-27\|RF2\|JH4 | ...........E<br>........ | .......... | ........<br>...... | ...E..<br>EN.Q....<br>.... | .................. | E..F...............<br>.........RS.. | ......<br>..... |
| iPS:39 4067 | 21-225_12F2 | VH3\|3-33\|D7\|7-27\|RF2\|JH4 | ............<br>............<br>........ | D......... | ........<br>...... | ...F....<br>.N....V.<br>.... | ............F...<br>............V. | E.AW...............<br>.........SE.. | ......<br>..... |
| iPS:39 4089 | 21-225_12E6 | VH3\|3-33\|D7\|7-27\|RF2\|JH4 | ............<br>............<br>........ | D......... | ........<br>.....T | ........<br>E.......<br>.... | ........D......... | E.AW...............<br>.........YE.. | ......<br>..... |
| iPS:39 4097 | 21-225_16G7 | VH3\|3-33\|D7\|7-27\|RF2\|JH4 | ............<br>............<br>.......T | D......... | ........<br>...... | ........<br>EN.E....<br>.... | .......A.......... | E.AW...............<br>.........YE.. | ......<br>..... |
| iPS:40 2219 | 21-225_1C12 | VH3\|3-33\|D7\|7-27\|RF2\|JH4 | ............<br>............<br>........ | N......... | ........<br>...... | ........<br>EN....V.<br>.... | .................. | E..F...............<br>.........RS.. | ......<br>..... |
| | | Germline | **H_FR1** | **H_CDR1** | **H_FR2** | **H_CDR2** | **H_FR3** | **H_CDR3** | **H_FR4** |
| | **VH3\|3-21\|D4\|4-11\|RF2\|JH4** | | EVQLVES-GGGLVKPGGSLRLSCAASG-FTFS | S------YSMN | WVRQAPGKGLEWVS | SISSS---SSYIYYADSVKG | RFTISRDNAKNSLYLQMNSLRAEDTAVYYCAR | DYSNY-------------YPDY | WGQGTLVTVSS |
| iPS:43 3895 | 21-225_43E1 | VH3\|3-21\|D4\|4-11\|RF2\|JH4 | ............<br>............<br>........ | .......... | ........<br>...... | A..GN...<br>.T......<br>.L.. | ............F..L | .RG--<br>...--SE | ......<br>..... |

| iPS:43 4103 | 21-225_53G1 | VH3\|3-21/D4\|4-11\|RF2/JH4 | . . . . . . . . . . .<br>. . . . E . . . . . . . .<br>. . . . . . . . | . . . . . . . . . . | . . . . . . . . .<br>. . . . . . | . . . G . . . .<br>. . . . . . . .<br>. . . . | . . . . . . . . . . . . . . . . . | .RG--<br>. . . . . . . . . . . . . . . . . . .<br>. . .--ST | . . . . . . |
| iPS:43 4179 | 21-225_56F1 | VH3\|3-21/D4\|4-11\|RF2/JH4 | . . . . . . . . . . .<br>. . F . . . . . . . .<br>. . . . . . . . | . . . . . . . . . . | . . . . . . . . .<br>. . . . . . | . . . . . . . .<br>. T . . . . G .<br>. . . . | . . . . . . . . . . . . . . . . . | .RG--<br>. . . . . . . . . . . . . . . . . . .<br>. . .--SS | . . . . . . |
| iPS:43 4263 | 21-225_56H7 | VH3\|3-21/D4\|4-11\|RF2/JH4 | . . . . . . . . . . .<br>. . . . . . . . . . .<br>. . . . . S . . | . . . . . . . . . . | . . . . . . . . .<br>. . . . . . | . . . . . . . .<br>. T . . . . G .<br>. . . . | . . . . . . . . . . . . . . . . . | .RG--<br>. . . . . . . . . . . . . . . . . . .<br>. . .--SS | . . . . . . |
| iPS:43 5521 | 21-225_157H4 | VH3\|3-21/D4\|4-11\|RF2/JH4 | . . . . . . . . . . .<br>. . . . . . . . . . .<br>. . . . . . . . | . . . . . . . . . . | . . . . . . . . .<br>. . . . . . | . . . . . . . .<br>. T . . . . . .<br>. . . . | . . . . . . . . . . . . . . . . . | .RC--<br>. . . . . . . . . . . . . . . . . . .<br>. . .--SI | . . . . . . |
| iPS:43 5527 | 21-225_157G7 | VH3\|3-21/D4\|4-11\|RF2/JH4 | . . . . . . . . . . .<br>. . . . . . . . F . . .<br>. . . . . . . . | . . . . . . . . . . | . . . . . . . . .<br>. . . . . . | . . . G . . . .<br>. T . . . . . .<br>. . . . | . . . . . . . . . . . . . . . . . | .RG--<br>. . . . . . . . . . . . . . . . . . .<br>. . .--SS | . . . . . . |
| iPS:43 5529 | 21-225_157H7 | VH3\|3-21/D4\|4-11\|RF2/JH4 | . . . . . . . . . . .<br>. . . . . . . . . . .<br>. . . . . . . . | . . . . . . . . . . | . . . . . . . . .<br>. . . . . . | C . . G . . . .<br>. . . . . . . .<br>. . . . | . . M . . . . . . . . . . . .<br>. . . . . . . . . . . . . V . | .RG--<br>. . . . . . . . . . . . . . . . . . .<br>. . .--G. | . . . . . . |
| iPS:43 5547 | 21-225_158F5 | VH3\|3-21/D4\|4-11\|RF2/JH4 | . . . . . . . . . . .<br>. . . . . . . . F . . .<br>. . . . . . . . | . . . . . . . . . . | . . . . . . . . .<br>. . . . . . | . . . G . . . .<br>. T . . . . . .<br>. . . . | . . . . . . . . . . . . . . . . . | .RG--<br>. . . . . . . . . . . . . . . . . . .<br>. . .--SS | . . . . . . |
| iPS:43 5549 | 21-225_158H5 | VH3\|3-21/D4\|4-11\|RF2/JH4 | . . . . . . . . . . .<br>. . . . . . . . . . .<br>. . . . . . . . | . . . . . . . . . . | . . . . . . . . .<br>. . . . . . | . . . G . . . .<br>. T . . . . . .<br>. . . . | . . . . . . . . . . . . . H . . . | .RG--<br>. . . . . . . . . . . . . . . . . . .<br>. . .--SS | . . . . . . |
| iPS:43 5553 | 21-225_158G8 | VH3\|3-21/D4\|4-11\|RF2/JH4 | . . . . . . . . . . .<br>. T . . . . . . . . .<br>. . . . . . . . | . . . . . . . T . . | . . . . . . . . .<br>. . . . . . | L . . G . . . .<br>. . . . . . . .<br>. . . . | . . . . . . . . . . . . . . . . . | .RG--<br>. . . . . . . . . . . . . . . . . . .<br>. . .--SL | . . . . . . |
| iPS:43 5581 | 21-225_160H1 | VH3\|3-21/D4\|4-11\|RF2/JH4 | . . . . . . . . . . .<br>. . . . . . . . . . .<br>. . . . . . . . | . . . . . . . R . . | . . . . . . . . .<br>. . . . . . | TG.M . . . .<br>. . . . | . . . . . . . . . . . F . . . | .K---<br>. . . . . . . . . . . . . . . . . . .<br>. . .--. . | . . . . . . |
| iPS:43 5593 | 21-225_160F4 | VH3\|3-21/D4\|4-11\|RF2/JH4 | . . . . . . . . . . .<br>. . . . . . . . F . . .<br>. . . . . . . . | . . . . . . . . . . | . . . . . . . . .<br>. . . . . . | . . . G . . . .<br>. T . . . . . .<br>. . . . | . . . . . . . . . . . . . . . . . | .RG--<br>. . . . . . . . . . . . . . . . . . .<br>. . .--SS | . . . . . . |
| iPS:43 5617 | 21-225_162F2 | VH3\|3-21/D4\|4-11\|RF2/JH4 | . . . . . . . . . . .<br>. . . . . . . . F . . .<br>. . . . . . . . | . . . . . . . . . . | . . . . . . . . .<br>. . . . . . | . . . G . . . .<br>. T . . . . . .<br>. . . . | . . . . . . . . . . . . . . . . . | .RG--<br>. . . . . . . . . . . . . . . . . . .<br>. . .--SS | . . . . . . |

| | | | | | | | | | |
|---|---|---|---|---|---|---|---|---|---|
| iPS:43<br>5621 | 21-<br>225_162H3 | VH3\|3-<br>21\|D4\|4-<br>11\|RF2\|J<br>H4 | ...........<br>.......F...<br>........ | .......... | R.......<br>......<br> | ...G....<br>.T......<br>.... | ................. | .RG--<br>.................<br>...--SS | ...... |
| iPS:43<br>5641 | 21-<br>225_163F9 | VH3\|3-<br>21\|D4\|4-<br>11\|RF2\|J<br>H4 | ...........<br>.Q..........<br>........ | .......... | ........<br>......<br> | ...G....<br>.T......<br>.... | ................. | .RG--<br>.................<br>...--SL | ...... |
| iPS:43<br>5719 | 21-<br>225_171A11 | VH3\|3-<br>21\|D4\|4-<br>11\|RF2\|J<br>H4 | ...........<br>...........<br>........ | .......... | ........<br>......<br> | ...G....<br>......<br>.... | ................. | .RG--<br>.................<br>...--SS | ....I.<br>...... |
| iPS:43<br>6856 | 21-225_58C5 | VH3\|3-<br>21\|D4\|4-<br>11\|RF2\|J<br>H4 | ...........<br>...........<br>.......N | .......... | ........<br>....I.<br> | ........<br>.Y.L....<br>.... | .......S........ | T..G-<br>.................<br>...S... | ...... |
| iPS:44<br>8904 | 21-<br>225_65C12 | VH3\|3-<br>21\|D4\|4-<br>11\|RF2\|J<br>H4 | ...........<br>...........<br>.......R | ......F.L. | ........<br>......<br> | ........<br>........<br>.... | ................. | .AY--<br>.................<br>...-SH. | ...... |
| iPS:47<br>2730 | 21-<br>225_14B1_L<br>C1 | VH3\|3-<br>21\|D4\|4-<br>11\|RF2\|J<br>H4 | ...........<br>...........<br>........ | .......T.. | ........<br>......<br> | ...G....<br>...L..P.<br>.... | ................. | .RG--<br>.................<br>...--SS | ...... |
| iPS:47<br>2731 | 21-<br>225_14B1_L<br>C2 | VH3\|3-<br>21\|D4\|4-<br>11\|RF2\|J<br>H4 | ...........<br>...........<br>........ | .......T.. | ........<br>......<br> | ...G....<br>...L..P.<br>.... | ................. | .RG--<br>.................<br>...--SS | ...... |
| iPS:39<br>2726 | 21-225_20B5 | VH3\|3-<br>21\|D4\|4-<br>11\|RF2\|J<br>H4 | ......A.....<br>...........<br>.......T | .......... | ........<br>......<br> | ...G....<br>........<br>.... | ................. | .RG--<br>.................<br>...--S. | ...... |
| iPS:39<br>2734 | 21-225_17D8 | VH3\|3-<br>21\|D4\|4-<br>11\|RF2\|J<br>H4 | ...........<br>...........<br>........ | .......GL. | ........<br>......<br> | ...G....<br>G.H.S...<br>.... | ................L | .RG--<br>.................<br>...--SG | ...... |
| iPS:39<br>2768 | 21-225_20B8 | VH3\|3-<br>21\|D4\|4-<br>11\|RF2\|J<br>H4 | ...........<br>...........<br>........ | .......... | ........<br>......<br> | ...G....<br>G.H.....<br>.... | ................. | .RG--<br>.................<br>...--SG | ...... |
| iPS:39<br>2778 | 21-225_22H3 | VH3\|3-<br>21\|D4\|4-<br>11\|RF2\|J<br>H4 | A..........<br>...........<br>........ | .......... | ........<br>......<br> | ........<br>........<br>.... | ..........F.... | .RG--<br>.................<br>...--SL | ...... |
| iPS:39<br>2788 | 21-225_20C8 | VH3\|3-<br>21\|D4\|4-<br>11\|RF2\|J<br>H4 | ...........<br>...........<br>........ | .......... | ........<br>......<br> | ...G....<br>........<br>...A | ................. | .RG--<br>.................<br>...--SL | ...... |

| | | | | | | | | | |
|---|---|---|---|---|---|---|---|---|---|
| iPS:39 2792 | 21-225_20G12 | VH3\|3-21/D4\|4-11\|RF2/JH4 | . . . . . . . . . . . . . . . . . . . . . . . . . . . . . . | . . . . . . . . . . | . . . . . . . . . . . . . . | . . .G. . . . . . . . . . . . . .L. . | . . . . . . . . . . . . . . . . | .RG-- . . . . . . . . . . . . . . . . . . . . . . . . . . .--S. | . . . . . . . . . . . |
| iPS:39 2844 | 21-225_23E11 | VH3\|3-21/D4\|4-11\|RF2/JH4 | . . . . . . . . . . . . . . . . . . . . . . . . . . . . . . | . . . . . . .T. . | . . . . . . . . . . . . . . | . . .G. . . . . . . .W.V. . . . . | . . . . . . . . . . . . . . . . | .RG-- . . . . . . . . . . . . . . . . . . . . . . . . .--SL | . . . . . . . . . . . |
| iPS:39 2848 | 21-225_20F9 | VH3\|3-21/D4\|4-11\|RF2/JH4 | . . . . . . . . . . . . . . . . . . . . . . . . . . . . . . | . . . . . . . . . . | . . . . . . . . . . . . . . | . . . . . . . . . . . . . . . . . . . | . . . . . . . . . . . . . . . . | .RG-- . . . . . . . . . . . . . . . . . . . . . . . . .--SC | . . . . . . . .I. . |
| iPS:39 2850 | 21-225_20H10 | VH3\|3-21/D4\|4-11\|RF2/JH4 | . . . . . . . . . . . . . . . . . . . . . . . . . . . . . . | T. . . . . . . . . | . . . . . . . . . . . . . . | . . . . . . . . . . . . . . . . . . . | . . . . . . . . . .I. . . . . | .RG-- . . . . . . . . . . . . . . . . . . . . . . . . .--SL | . . . . . . . . . . . |
| iPS:39 3006 | 21-225_31G9 | VH3\|3-21/D4\|4-11\|RF2/JH4 | . . . . . . . . . . . . . . . . . . . . . . . . . . . . . . | . . . . . . . . . . | . . . . . . . . . . . . . . | . . . . . . . . . . . . . . . . . . . | . . . . . . . . . . . . . . . . | .RG-- . . . . . . . . . . . . . . . . . . . . . . . . .--SS | . . . . . . . . . . . |
| iPS:39 3022 | 21-225_30H11 | VH3\|3-21/D4\|4-11\|RF2/JH4 | . . . . . . . . . . . . . . . . . . . . . . . . . . . . . . | . . . . . . . . . . | . . . . . . . . . . . . . . | . . .G. . . . . . . . . . . . . . . . | . . . . . . . . . . . . . . . . | .RG-- . . . . . . . . . . . . . . . . . . . . . . . . .--SL | . . . . . . . . . . . |
| iPS:39 3130 | 21-225_33C2 | VH3\|3-21/D4\|4-11\|RF2/JH4 | . . . . . . . . . . . . . . . . . . . . . . . . . . . . . . | . . . . . . .T. . | . . . . . . . . . .Q. . . . | . . .G. . . . . . . . . . . . . . . . | . . . . . . . . . . . . .F. . . . . . . . . . . . .IF. . . . | .RG-- . . . . . . . . . . . . . . . . . . . . . . . . .--GT | . . . . . . . . . . . |
| iPS:39 3906 | 21-225_13D3 | VH3\|3-21/D4\|4-11\|RF2/JH4 | . . . . . . . . . . . . . . . . . . . . . . . . . . . . . . | . . . . . . .T. . | . . . . . . . . . .D. . . . | . . .G. . . . . . . . . . . . . . . . | . . . . . . . . . . . . . . . . | .RG-- . . . . . . . . . . . . . . . . . . . . . . . . .--SG | . . . . . . . . . . . |
| iPS:39 3982 | 21-225_6C12 | VH3\|3-21/D4\|4-11\|RF2/JH4 | . . . . . . . . . . . . . . . . . . . . . . . . . . . . . . | . . . . . . . . . . | . . . . . . . . . . . . . . | . . . . . . . . . . . . . . . . . . . | . . . . . . . . . . . . . . . . | .RG-- . . . . . . . . . . . . . . . . . . . . . . . . .--SL | . . . . . . . . . . . |
| iPS:39 8478 | 21-225_17C10 | VH3\|3-21/D4\|4-11\|RF2/JH4 | . . . . . . . . . . . . . . . . . . . . . . . . . . . . . . | . . . . . . . . . . | . . . . . . . . . . . . . . | . . .G. . . . . . . .M. . . . . . . . | . . . . . . . . . . .L. . . . . | .RG-- . . . . . . . . . . . . . . . . . . . . . . . . .--SS | . . . . . . . . . . . |
| | Germline | H_FR1 | H_CDR1 | H_FR2 | H_CDR2 | H_FR3 | H_CDR3 | H_FR4 | |
| VH3\|3-23/D6\|6-6\|RF1/JH4 | | EVQLLES-GGGLVQPGGSLRLSCAASG-FIFS | S-----YAMS | WVRQAPGK GLEWVS | AISGS---GGSTYYAD SVKG | RFTISRDNSKNTLYLQM NSLRAEDTAVYYCAK | EYSSS---------------------SYFDY | WGQGTL VTVSS | |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 3897 | 21-225_43C2 | VH3\|3-23\|D6\|6-6\|RF1/JH4 | ............<br>............<br>........ | .......... | ........<br>......<br>.... | ....R...<br>.VN.FD..<br>.... | ................<br>................ | .R.G.............<br>.......⁻.... | ......<br>..... |
| iPS:43 3903 | 21-225_43H4 | VH3\|3-23\|D6\|6-6\|RF1/JH4 | ............<br>............<br>........ | .......... | ........<br>......<br>.... | ....R...<br>.IN.FD..<br>.... | ................<br>................ | .R.G.............<br>.......⁻.... | ......<br>..... |
| iPS:43 3911 | 21-225_43E8 | VH3\|3-23\|D6\|6-6\|RF1/JH4 | ............<br>............<br>........ | .......... | ........<br>......<br>.... | ....R...<br>.IN.FD..<br>.... | ................<br>................ | .R.G.............<br>.......⁻.... | ......<br>..... |
| iPS:43 3941 | 21-225_44D10 | VH3\|3-23\|D6\|6-6\|RF1/JH4 | ............<br>............<br>........ | .......... | ........<br>......<br>.... | ....R...<br>.VN.FD..<br>.... | ................<br>................ | .R.G.............<br>.......⁻.... | ......<br>..... |
| iPS:43 3957 | 21-225_45F8 | VH3\|3-23\|D6\|6-6\|RF1/JH4 | ............<br>............<br>........ | .......... | ........<br>......<br>.... | ....R...<br>.VN.FD..<br>.... | ................<br>................ | .R.G.............<br>.......⁻.... | ......<br>..... |
| iPS:43 3973 | 21-225_46A6 | VH3\|3-23\|D6\|6-6\|RF1/JH4 | ............<br>............<br>........ | .......... | ........<br>......<br>.... | ....R...<br>.IN.FD..<br>.... | ................<br>................ | .R.G.............<br>.......⁻.... | ......<br>..... |
| iPS:43 5715 | 21-225_171A8 | VH3\|3-23\|D6\|6-6\|RF1/JH4 | ............<br>............<br>.......R | ......S... | ........<br>......<br>.... | V.......<br>....F.T.<br>.... | ................<br>................ | SN..G............<br>........⁻W... | ......<br>..... |
| iPS:43 5739 | 21-225_174G7 | VH3\|3-23\|D6\|6-6\|RF1/JH4 | ............<br>............<br>.......R | ......S... | ........<br>......<br>.... | V.......<br>....F.T.<br>.... | ................<br>................ | SN..G............<br>........⁻W... | ......<br>..... |
| iPS:43 5749 | 21-225_175C10 | VH3\|3-23\|D6\|6-6\|RF1/JH4 | ............<br>............<br>........ | .......... | ........<br>......<br>.... | S...R...<br>....F...<br>.... | ...V............<br>................ | SN..G............<br>........⁻W... | ......<br>..... |
| | | Germline | H_FR1 | H_CDR1 | H_FR2 | H_CDR2 | H_FR3 | H_CDR3 | H_FR4 |
| | VH3\|3-21\|D7\|7-27\|RF1/JH4 | | EVQLVES-<br>GGGLVKPGGSLR<br>LSCAASG-FIFS | S-----YSMN | WVRQAPGK<br>GLEWVS | SISSS---<br>SSYIYYAD<br>SVKG | RFTISRDNAKNSLYLQM<br>NSLRAEDTAVYYCAR | LTGY------------<br>--------FDY | WGQGTL<br>VTVSS |
| iPS:43 3901 | 21-225_43A4 | VH3\|3-21\|D7\|7-27\|RF1/JH4 | ............<br>............<br>........ | ........T.. | ....V...<br>...... | ...G....<br>.T......<br>.... | .......D.Q.......<br>....G.......... | V.S-<br>................<br>........ | ....A.<br>..... |

| | | | | | | | | | |
|---|---|---|---|---|---|---|---|---|---|
| iPS:43 3961 | 21-225_45D9 | VH3\|3-21/D7\|7-27\|RF1/J H4 | ............<br>............<br>........ | .......T..<br><br> | ....V...<br>......<br>.... | ...G....<br>.I......<br>.... | ......D.Q.......<br>....G...........<br> | V.S-<br>...................<br>........ | ....A.<br>.....<br> |
| iPS:43 4135 | 21-225_54H3 | VH3\|3-21/D7\|7-27\|RF1/J H4 | ............<br>............<br>........ | .........I<br><br> | ........<br>.....<br>.... | ...GT...<br>........<br>.... | ................<br>...............G<br> | M.T-<br>...................<br>......-VI | ......<br><br> |
| iPS:43 4331 | 21-225_63H8 | VH3\|3-21/D7\|7-27\|RF1/J H4 | ............<br>............<br>........ | .......N..<br><br> | ........<br>......<br>.... | ...G....<br>.T.MN.T.<br>.... | ................<br>................<br> | .RN-<br>...................<br>........ | ......<br><br> |
| iPS:43 5421 | 21-225_151F1 | VH3\|3-21/D7\|7-27\|RF1/J H4 | ............<br>............<br>....Y..R | ......F...<br><br> | ........<br>......<br>.... | ...Y.....<br>....<br> | ................<br>................<br> | D.P-<br>...................<br>......LV. | ......<br><br> |
| iPS:43 5653 | 21-225_166H12 | VH3\|3-21/D7\|7-27\|RF1/J H4 | ...........A.<br>............<br>........ | .........S<br><br> | ........<br>..G...<br>.... | ...G....<br>...S....<br>.... | ................<br>................<br> | ...-<br>...................<br>........ | ......<br><br> |
| iPS:43 6648 | 21-225_227F11 | VH3\|3-21/D7\|7-27\|RF1/J H4 | ............<br>............<br>V....... | T.........<br><br> | ........<br>......<br>.... | IN.M....<br>....<br> | ................<br>.......S........<br> | .G~~<br>...................<br>......-V. | ......<br><br> |
| iPS:39 2952 | 21-225_26G1 | VH3\|3-21/D7\|7-27\|RF1/J H4 | ............<br>............<br>........ | .......G..<br><br> | ........<br>......<br>.... | ...G....<br>........<br>.... | ................<br>................<br> | ..T-<br>...................<br>.......F | ......<br><br> |
| iPS:39 3082 | 21-225_34C11 | VH3\|3-21/D7\|7-27\|RF1/J H4 | ............<br>............<br>........ | .......T.S<br><br> | ........<br>......<br>.... | ...G....<br>.N......<br>.... | ................<br>................<br> | ...-<br>...................<br>........ | ......<br><br> |
| iPS:39 4061 | 21-225_12D2 | VH3\|3-21/D7\|7-27\|RF1/J H4 | ............<br>............<br>........ | ..........<br><br> | ........<br>......<br>.... | ........<br>........<br>.... | ................<br>................<br> | .G~~<br>...................<br>.....-~.. | ......<br>.A...<br> |
| iPS:39 4071 | 21-225_10C7 | VH3\|3-21/D7\|7-27\|RF1/J H4 | ............<br>............<br>........ | ..........<br><br> | ........<br>......<br>.... | NN......<br>.... | ................<br>.......S.......<br> | .G~~<br>...................<br>......-V. | ......<br><br> |
| iPS:39 8532 | 21-225_33B7 | VH3\|3-21/D7\|7-27\|RF1/J H4 | ............<br>............<br>........ | .......N..<br><br> | ........<br>......<br>.... | ...G....<br>........<br>.... | ................<br>................<br> | .N.-<br>...................<br>........ | ......<br><br> |
| iPS:40 2225 | 21-225_2B1 | VH3\|3-21/D7\|7-27\|RF1/J H4 | ............<br>............<br>........ | ..........<br><br> | ........<br>......<br>.... | ........<br>.... | ................<br>................<br> | .G~~<br>...................<br>......-N. | .....<br><br> |

| iPS:40 4090 | 21-225_8D8 | VH3I3-21/D7I7-27IRF1/JH4 | . . . . . . . . . . . .<br>. . . . . . . . . .<br>. . . . . . . . | . . . . . . . . . . | . . . . . . . .<br>. . . . . .<br>. . . . | . . . . . . . .<br>. . . . . . .<br>. . . . | . . . . . . . . . . . . . . . . . | .G--<br>. . . . . . . . . . . . . . . . . . .<br>. . . . . . -N. | . . . . . .<br>. . . . . |
|---|---|---|---|---|---|---|---|---|---|
| | **Germline** | | **H_FR1** | **H_CDR1** | **H_FR2** | **H_CDR2** | **H_FR3** | **H_CDR3** | **H_FR4** |
| | **VH3I3-11/D4I4-11IRF2/JH4** | | QVQLVES-GGGLVKPGGSLR LSCAASG-FIFS | D-----YYMS | WIRQAPGK GLEWVS | YISSS---GSTIYYAD SVKG | RFTISRDNAKNSLYLQM NSLRAEDTAVYYCAR | DYSNY----------------YFDY | WGQGTL VTVSS |
| iPS:43 3905 | 21-225_43E5 | VH3I3-11/D4I4-11IRF2/J H4 | . . . . . . . . . . . .<br>. . . . . . . . . .<br>. . . . . . . . | . . . . . . . . . I | . . . . . . . .<br>. . . . . . | . . . . . . . .<br>.I.K. . . .<br>.M. . | . . . . . . . . . . . . . . . . .A | .T---<br>. . . . . . . . . . . . . . . . . . .<br>. . . .--I. | . . . . . .<br>. . . . . |
| iPS:43 3913 | 21-225_43H8 | VH3I3-11/D4I4-11IRF2/J H4 | . . . . . . . . . . . .<br>. . . . . . . . . .<br>. . . .I. . . . | . . . . . . . . . N | . . . . . . . .<br>. . . . . . | . . . . . . . .<br>.R. .F. . .<br>.L. . | . . . . . . . . . . . . . . . . .A | .T---<br>. . . . . . . . . . . . . . . . . . .<br>. . . .--I. | . . . . . .<br>. . . . . |
| iPS:43 3949 | 21-225_45H2 | VH3I3-11/D4I4-11IRF2/J H4 | . . . . . . . . . . . .<br>. . . . . . . . . .<br>. . . . . . . . | . . . . . . . . . N | . . . . . . . .<br>. . . . . . | . . . . . . . .<br>.I.K. . . .<br>. . . . | . . . . . . . . . . . . . . . . .A | .T---<br>. . . . . . . . . . . . . . . . . . .<br>. . . .--I. | . . . . . .<br>. . . . . |
| iPS:43 3981 | 21-225_46E9 | VH3I3-11/D4I4-11IRF2/J H4 | . . . . . . . . . . . .<br>. . . . . . . . . .<br>. . . . . . . . | . . . . . . . . . N | . . . . . . . .<br>. . . . . . | . .N.N. . .<br>.F. . . . . .<br>. . . . | . . . . . . . . . . . . . . . . .A | .T---<br>. . . . . . . . . . . . . . . . . . .<br>. . . .--I. | . . . . . .<br>. . . . . |
| iPS:43 3995 | 21-225_47H7 | VH3I3-11/D4I4-11IRF2/J H4 | . . . . . . . . . . . .<br>. . . . . . . . . .<br>. . . . . . . . | . . . . . . . . . I | . . . . . . . .<br>. . . . . . | . .N.N. . .<br>.F.K. . . .<br>. . . . | . . . . . . . . . . . . . . . . .A | .T---<br>. . . . . . . . . . . . . . . . . . .<br>. . . .--V. | . . . . . .<br>. . . . . |
| iPS:43 4039 | 21-225_43B1 | VH3I3-11/D4I4-11IRF2/J H4 | . . . . . . . . . . . .<br>. . . . . . . . . .<br>. . . . . . . . | . . . . . . . . . N | . . . . . . . .<br>. . . . . . | . .N.N. . .<br>.F. . . . . .<br>. . . . | . . . . . . . . . . . . . . . . .A | .T---<br>. . . . . . . . . . . . . . . . . . .<br>. . . .--I. | . . . . . .R<br>. . . . . |
| iPS:43 4275 | 21-225_57F4 | VH3I3-11/D4I4-11IRF2/J H4 | . . . . . . . . . . . .<br>. . . . . . . . . .<br>. . . . .I. . | . . . . . . . . . N | . . . . . . . .<br>. . . . . . | . . . . . . . .<br>. . . . . . .<br>. . . . | . . . . . . . . . . . . . . . . . | .M---<br>. . . . . . . . . . . . . . . . . . .<br>. . . .--IT | . . . . . .<br>. . . . . |
| | **Germline** | | **H_FR1** | **H_CDR1** | **H_FR2** | **H_CDR2** | **H_FR3** | **H_CDR3** | **H_FR4** |
| | **VH3I3-23/D7I7-27IRF1/JH3** | | EVQLLES-GGGLVQPGGSLR LSCAASG-FIFS | S-----YAMS | WVRQAPGK GLEWVS | AISGS---GGSTYYAD SVKG | RFTISRDNSKNTLYLQM NSLRAEDTAVYYCAK | LTGD------------------AFDI | WGQGTM VTVSS |
| iPS:43 3915 | 21-225_43H9 | VH3I3-23/D7I7-27IRF1/J H3 | . . . . . . . . . . . .<br>. . . . . . . . . .<br>. . . . . . . . | . . . . . . . . . . | . . . . . . .M<br>. . . . . . | . . . . . . . .<br>.SN.F. . .<br>. . . . | . . . . . . . . . . . . . . .H.<br>. . . . . . . . . . .F. . . | R.PSD. . . . . . . . . . . .<br>. . . . . . . .V. . . | . . . . . .<br>. . . . . |

| ID | Name | Germline | H_FR1 | H_CDR1 | H_FR2 | H_CDR2 | H_FR3 | H_CDR3 | H_FR4 |
|---|---|---|---|---|---|---|---|---|---|
| iPS:43 3925 | 21-225_44F3 | VH3\|3-23/D7\|7-27\|RF1/JH3 | ..H.........<br>...........<br>........ | ........... | ........<br>......<br>.... | IL..G...<br>.KT.....<br>.... | ..............F...<br>............... | R.PSD.............<br>............. | ......<br>..... |
| iPS:43 3953 | 21-225_45H4 | VH3\|3-23/D7\|7-27\|RF1/JH3 | ...........<br>...........<br>........ | ........... | ........M<br>......<br>.... | .SN.F...<br>.... | ..............H.<br>..........F... | R.PSD.............<br>........V... | ......<br>..... |
| iPS:43 3959 | 21-225_45C9 | VH3\|3-23/D7\|7-27\|RF1/JH3 | ...........<br>...........<br>.......N | ........... | ........<br>......<br>.... | V...R...<br>..T.....<br>.... | ...............<br>............... | R.PSD.............<br>............. | ......<br>..... |
| iPS:43 5379 | 21-225_149B6 | VH3\|3-23/D7\|7-27\|RF1/JH3 | ...........<br>...........<br>........ | ........... | ........<br>......<br>.... | V.......<br>....F...<br>.... | .............F...<br>............... | R.PED.............<br>........V... | ......<br>..... |
| iPS:43 5787 | 21-225_180A3 | VH3\|3-23/D7\|7-27\|RF1/JH3 | ...........<br>E..........<br>........ | ......F..N | ........<br>......<br>.... | V...R...<br>..N.F...<br>.... | .............F...<br>..........F... | R...D.............<br>........V..V | ......<br>..... |
| iPS:43 5809 | 21-225_182H5 | VH3\|3-23/D7\|7-27\|RF1/JH3 | ...........<br>...........<br>........ | ........... | ........<br>......<br>.... | V...R...<br>..T.F...<br>.... | ...............<br>............... | R...D.............<br>........V... | ......<br>..... |
| iPS:43 5889 | 21-225_186A11 | VH3\|3-23/D7\|7-27\|RF1/JH3 | ...........<br>...........<br>........ | ........... | ........<br>......<br>.... | V...R...<br>..T.F...<br>.... | ...............<br>............... | R...D.............<br>........V... | ......<br>..... |

| | Germline | | H_FR1 | H_CDR1 | H_FR2 | H_CDR2 | H_FR3 | H_CDR3 | H_FR4 |
|---|---|---|---|---|---|---|---|---|---|
| | VH4\|4-59/D6\|6-13\|RF2/JH4 | | QVQLQES-GPGLVKPSEILS LTCTVSG-GSIS | S-----YYWS | WIRQPPGK GLEWIG | YIYY----SGSTNYNP SLKS | RVTISVDTSKNQFSLKL SSVTAADTAVYYCAR | GIAAA----------GYFDY | WGQGTL VTVSS |
| iPS:43 3931 | 21-225_44F6 | VH4\|4-59/D6\|6-13\|RF2/JH4 | ..H.........<br>...........<br>........ | ........... | .........<br>......<br>.... | ..N.....<br>.... | ..................<br>.............V. | .V.I-<br>.................<br>..--KN. | ....I.<br>..... |

| | Germline | | H_FR1 | H_CDR1 | H_FR2 | H_CDR2 | H_FR3 | H_CDR3 | H_FR4 |
|---|---|---|---|---|---|---|---|---|---|
| | VH3\|3-23/D4\|4-17\|RF2/JH6 | | EVQLLES-GGGLVQPGGSLR LSCAASG-FIFS | S-----YAMS | WVRQAPGK GLEWVS | AISGS---GGSTYYAD SVKG | RFTISRDNSKNTLYLQM NSLRAEDTAVYYCAK | DYGDYYY----------YYYGMDV | WGQGTT VTVSS |
| iPS:43 3943 | 21-225_44E10 | VH3\|3-23/D4\|4-17\|RF2/JH6 | ...........<br>..S.........<br>........ | .........N | ........<br>......<br>.... | GVV.....<br>..R.....<br>.... | ..I...............<br>............... | .R.QWL-<br>................-<br>LG.... | ......<br>..... |

| | | | H_FR1 | H_CDR1 | H_FR2 | H_CDR2 | H_FR3 | H_CDR3 | H_FR4 |
|---|---|---|---|---|---|---|---|---|---|
| iPS:43 3989 | 21-225_47C7 | VH3\|3-23/D4\|4-17\|RF2/JH6 | ............ / ............ / ........ | N......... | ........ / ...... / .... | G....... / .SR..... / | ................ / ............... | .R.QWL- / ................- / IG.... | ...... / ..... |
| iPS:43 4133 | 21-225_54G3 | VH3\|3-23/D4\|4-17\|RF2/JH6 | ............ / ............ / ........ | T......... | ........ / ...... / .... | ........ / .VN.F... / | ...............F... / ............... | LGK...- / ................- / ...... | ...... / ..... |
| iPS:43 4221 | 21-225_60A11 | VH3\|3-23/D4\|4-17\|RF2/JH6 | ............ / ............ / ........ | N........T | ........ / ...... / | ........ / ..N.F... / ..T. | .V............... / ............... | LGK..H- / ................- / ...... | ...... |
| iPS:43 4257 | 21-225_62F7 | VH3\|3-23/D4\|4-17\|RF2/JH6 | ............ / ............ / ........ | .......V.. | ........ / ...... / .... | G....... / .AK..... / | ................ / ..............E | LGI...- / ................-- / ..... | ...... |
| iPS:43 4283 | 21-225_57F8 | VH3\|3-23/D4\|4-17\|RF2/JH6 | ............ / .........V / V....... | N......... | ........ / ...... / | .S...... / ..N.F... / ..T. | .V............... / ............... | LGK..H- / ...............- / ...... | ...... |
| iPS:43 4385 | 21-225_66C10 | VH3\|3-23/D4\|4-17\|RF2/JH6 | ............ / ............ / ........ | .......V.. | ........ / ...... / | G....... / .AR..... / | ................ / ..............E | LGI...- / ................-- / ..... | ...... |
| iPS:43 5717 | 21-225_171A9 | VH3\|3-23/D4\|4-17\|RF2/JH6 | ............ / A........... / ........ | .........T | ......... / ...... / | ........ / ..N.FN.. / | ................ / ............... | LGI...- / ................- / ...... | ...... |
| iPS:43 6528 | 21-225_224B1 | VH3\|3-23/D4\|4-17\|RF2/JH6 | ...V........ / ............ / ........ | .......... | ........ / ...... / .... | ........ / ..N..... / | ...............H. / ..............G | EG.Y..- / ................- / ...V.. | ...... |
| iPS:39 3810 | 21-225_5A4 | VH3\|3-23/D4\|4-17\|RF2/JH6 | ...V........ / ............ / ....L... | ......S... | ........ / ...... / | ....R... / ..N.F.T. / .... | ................ / ............... | LGK...- / ................- / ...... | ...... |
| | Germline | H_FR1 | H_CDR1 | H_FR2 | H_CDR2 | H_FR3 | H_CDR3 | H_FR4 | |
| | VH3\|3-48/D5\|5-24\|RF3/JH6 | | EVQLVES-GGGLVQPGGSLRLSCAASG-FIFS | S-----YSMN | WVRQAPGKGLEWVS | YISSS---SSTIYYADSVKG | RFTISRDNAKNSLYLQMNSLRDEDTAVYYCAR | RDGYNYYY-----------YYYGMDV | WGQGTTVTVSS |
| iPS:43 3945 | 21-225_44C12 | VH3\|3-48/D5\|5-24\|RF3/JH6 | ............ / ............ / ........ | ........V. | ........ / ...... | ........ / .... | ................ / ............... | SGYSYA............ | ...... / ..... |
| | Germline | H_FR1 | H_CDR1 | H_FR2 | H_CDR2 | H_FR3 | H_CDR3 | H_FR4 | |

| VH3l3-33/D6\|6-13\|RF2/JH4 | | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | GIAAA------------ -------GYFDY | WGQGTL VTVSS |
|---|---|---|---|---|---|---|---|---|
| iPS:43 3947 | 21-225_44E12 | VH3l3-33/D6\|6-13\|RF2/JH4 | ..H.A....... ...........E ......L. | ......DDT. | ....P... ...... | ...F.... EY...... .... | ................. .......A....... | DLI..A........... ........TG.. | ...... ..... |
| iPS:43 3963 | 21-225_46B1 | VH3l3-33/D6\|6-13\|RF2/JH4 | ..H.A....... ...........E ......L. | ......DDS. | ....P... ...... | ...F.... EYT..... .... | ................. .N.....A....... | DLI..T........... ........TG.. | ...... ..... |
| iPS:43 3987 | 21-225_47A5 | VH3l3-33/D6\|6-13\|RF2/JH4 | ............ ............ ............ | D.....DDT. | ........ ...... | ...F.... ........ .... | ................. ...S........... | DLI..A........... ........TV.. | ...... ..... |
| iPS:43 6258 | 21-225_202F12 | VH3l3-33/D6\|6-13\|RF2/JH4 | ............ ...........E ........ | Y......... | ........ ...... | I....... ....F... .... | .......S......... ...............S | N....A........... .......P.... | ...... ..... |
| iPS:39 2646 | 21-225_20G2 | VH3l3-33/D6\|6-13\|RF2/JH4 | ............ ............ ......L. | ......DD.. | ....E... ...... | ...F.... ........ .... | ..IM............. ......G....... | DLI..A........... ........TV.. | ...... ..... |
| iPS:39 2750 | 21-225_20A10 | VH3l3-33/D6\|6-13\|RF2/JH4 | ............ ............ ......L. | ......DD.. | ........ ...... | ...F.... ........ .... | ..I.............. .........M..... | DLI..A........... ........TV.. | ...... ..... |
| | Germline | H_FR1 | H_CDR1 | H_FR2 | H_CDR2 | H_FR3 | H_CDR3 | H_FR4 | |
| VH3l3-23/D6\|6-19\|RF2/JH3 | | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | GIAVAG----------- -----DAFDI | WGQGTM VTVSS |
| iPS:43 3999 | 21-225_48D1 | VH3l3-23/D6\|6-19\|RF2/JH3 | ............ ............ ..R..... | .......... | ........ ...... | V...R... ....F... .... | ................. ..............R | R................ ......NE.... | ...... ..... |
| iPS:43 4003 | 21-225_48C3 | VH3l3-23/D6\|6-19\|RF2/JH3 | ............ ............ ..R..... | .......... | ........ ...... | V...R... ....F... .... | ................. ..............R | R................ ......NE.... | ...... ..... |
| iPS:43 4037 | 21-225_49G12 | VH3l3-23/D6\|6-19\|RF2/JH3 | ............ ...........T ........ | .......... | ........ ...... | V....... ..T.F... .... | .L............... ..............R | R................ ......N..... | ...... ..... |
| iPS:43 4041 | 21-225_50H8 | VH3l3-23/D6\|6-19\|RF2/JH3 | ............ ............ ..R..... | .......... | ........ ...... | V...R... ..T.F... .... | ................. ..............R | R................ ......NE.... | ...... ..... |

| | | | | | | | | |
|---|---|---|---|---|---|---|---|---|
| iPS:43 4045 | 21-225_50H10 | VH3\|3-23/D6\|6-19\|RF2/J H3 | .G.......... ............ ..R..... | .......... | ........ ...... .... | V...R... ....F... | ................ ..............R | R................. ......NE.... .....|
| iPS:43 4073 | 21-225_51H10 | VH3\|3-23/D6\|6-19\|RF2/J H3 | ............ ...........T ........ | .......... | ........ ...... .... | V....... ..T.F... | .L.............. ..............R | R................. ......N..... .....|
| iPS:43 6212 | 21-225_200G1 | VH3\|3-23/D6\|6-19\|RF2/J H3 | ............ ............ ........ | .......... | ........ ...... .... | ...R... ..N.F... ..R. | ................ ..............R | R...D............ ......Y....V .....|
| iPS:39 2852 | 21-225_17C6 | VH3\|3-23/D6\|6-19\|RF2/J H3 | ............ ............ ..E..... | .........N | ........ ...... .... | V...R... ..N.F... | ..............F... ..............S | RL............... ......SE.... .....|
| iPS:39 2668 | 21-225_17B4 | VH3\|3-23/D6\|6-19\|RF2/J H3 | ............ ............ ..E..... | .........N | ........ ...... .... | V...R... ..N.F... | ................ ..............S | RL............... ......SE.... .....|
| iPS:39 2696 | 21-225_28A4 | VH3\|3-23/D6\|6-19\|RF2/J H3 | ............ ............ ........ | .........T | ....G..M ...... .... | V....... ..Y..N.. | ....V.........S | R................ ......SE.... .....|
| iPS:39 2702 | 21-225_17F7 | VH3\|3-23/D6\|6-19\|RF2/J H3 | ............ ............ ..E..... | .........N | ........ ...... .... | I...R... ..N.F... | ................ ..............S | RL............... ......SE.... .....|
| iPS:39 2704 | 21-225_17F11 | VH3\|3-23/D6\|6-19\|RF2/J H3 | .A.......... E........... ........ | .......... | ........ ...... .... | V...R... ..N.S.. | ................ ..............S | RL............... ......SE..H. .....|
| iPS:39 2720 | 21-225_17A12 | VH3\|3-23/D6\|6-19\|RF2/J H3 | ............ I........... ..E..... | .......... | ....D... ...... .... | I...R... ..NAF... | ................ ..............S | R................ ......SE.... .....|
| iPS:39 2722 | 21-225_18E12 | VH3\|3-23/D6\|6-19\|RF2/J H3 | ............ ............ ..E..... | .......... | ........T ...... .... | I...R... ..N.F... | ................ ..............S | RL............... ......SE.... .....|
| iPS:39 2760 | 21-225_22G3 | VH3\|3-23/D6\|6-19\|RF2/J H3 | ............ ............ ..E..... | .........N | ........ ...... .... | I...R... .VN.F... | ................ ..............S | RL............... ......SE.... .....|
| iPS:39 2764 | 21-225_22G10 | VH3\|3-23/D6\|6-19\|RF2/J H3 | ..........D. ...........T ........ | .........N | ........ ...... .... | V....... ..N.F... | ..............F.H. ..............S | RM............... ......SE.... .....|

| | | | | | | | | | |
|---|---|---|---|---|---|---|---|---|---|
| iPS:39 2812 | 21-225_21F4 | VH3\|3-23\|D6\|6-19\|RF2\|JH3 | ..........<br>..........<br>..E..... | .........N | ........<br>...... | V...R...<br>..N.F...<br>.... | ................<br>................S | RL................<br>......SE.C.. | ......<br>..... |
| iPS:39 2816 | 21-225_22E4 | VH3\|3-23\|D6\|6-19\|RF2\|JH3 | ..........<br>..........<br>..E..... | .......... | ........<br>...... | I...R...<br>.TN.F...<br>.... | .......V.........<br>................S | R.................<br>......SE.... | ......<br>..... |
| iPS:39 2852 | 21-225_21A2 | VH3\|3-23\|D6\|6-19\|RF2\|JH3 | ..........<br>..........<br>..K..... | .........N | ........<br>....I. | I...R...<br>..N.F...<br>.... | ................<br>................S | RL................<br>......SE.... | ......<br>..... |
| iPS:39 2878 | 21-225_22C5 | VH3\|3-23\|D6\|6-19\|RF2\|JH3 | ..........<br>..V.......<br>......... | .......... | .......M<br>...... | I.......<br>..Y.....<br>.... | ................<br>................S | R.................<br>......SE.... | ......<br>..... |
| iPS:39 2902 | 21-225_22D5 | VH3\|3-23\|D6\|6-19\|RF2\|JH3 | ..........<br>..........<br>..E..... | .......... | ........<br>...... | V...R...<br>..N.F...<br>.... | ................<br>................S | R.................<br>......SE.... | ......<br>..... |
| iPS:39 3824 | 21-225_10F12 | VH3\|3-23\|D6\|6-19\|RF2\|JH3 | ..........<br>..........<br>..E..... | .......... | ........<br>...... | I...R...<br>..N.F...<br>.... | ................<br>................S | RV................<br>......SE..A. | ......<br>..... |
| iPS:39 3848 | 21-225_4H2 | VH3\|3-23\|D6\|6-19\|RF2\|JH3 | ..........<br>..........<br>........ | G.........N | .......M<br>...... | V..R....<br>..Y.....<br>.... | ................<br>................S | RL................<br>......SE.... | ......<br>..... |
| iPS:39 3862 | 21-225_5G2 | VH3\|3-23\|D6\|6-19\|RF2\|JH3 | ..........<br>..........<br>..E..... | .......... | ........<br>...... | V...R...<br>.VN.F...<br>.... | ................<br>................S | R.................<br>......SE.... | ......<br>..... |
| iPS:39 3888 | 21-225_3E3 | VH3\|3-23\|D6\|6-19\|RF2\|JH3 | ..........T.<br>..........<br>..E..... | .......V.. | ........<br>...... | I...R...<br>..N.F...<br>.... | ................<br>.........L....S | RL................<br>......SE.... | ......<br>..... |
| iPS:39 3898 | 21-225_5F7 | VH3\|3-23\|D6\|6-19\|RF2\|JH3 | ..........<br>..........<br>..E..... | .......... | ........<br>...... | I...R...<br>..N.F...<br>.... | ................<br>................S | R.................<br>......SE..A. | ......<br>..... |
| iPS:39 3980 | 21-225_6D3 | VH3\|3-23\|D6\|6-19\|RF2\|JH3 | ..........<br>..........<br>..E..... | .........N | ........<br>...... | V...R...<br>..N.F...<br>.... | ................<br>..........F..S | RL................<br>......SE.... | ......<br>..... |
| iPS:39 4014 | 21-225_8G6 | VH3\|3-23\|D6\|6-19\|RF2\|JH3 | ..........<br>..........V<br>..E..... | .......V.N | ........<br>...... | V...R...<br>..N.F...<br>.... | ................<br>................S | RL................<br>......SE.... | ......<br>.S... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:39 4022 | 21-225_16H6 | VH3\|3-23/D6\|6-19\|RF2/JH3 | .............<br>.............<br>........ | .........X | ........M<br>...... | V..R....<br>..Y.....<br>.... | ................<br>...............S | RL...............<br>......SE.... | ......<br>..... |
| iPS:39 4043 | 21-225_3B1 | VH3\|3-23/D6\|6-19\|RF2/JH3 | .............<br>.............<br>..E..... | .........N | ........<br>...... | V...R....<br>.IN.F...<br>.... | ................<br>...............S | RL...............<br>......SE.... | ......<br>..... |
| iPS:39 4077 | 21-225_8E12 | VH3\|3-23/D6\|6-19\|RF2/JH3 | .............<br>.............<br>..E..... | ......... | ........<br>...... | I...R....<br>..N.F...<br>.... | ................<br>...............S | RM...............<br>......SE.... | ......<br>..... |
| iPS:39 4087 | 21-225_11A5 | VH3\|3-23/D6\|6-19\|RF2/JH3 | ..........D.<br>.............<br>..E..... | ......... | ........<br>....I. | V...R....<br>.VN.F...<br>.... | ................<br>...............S | R................<br>......SE.... | ......<br>..... |
| | | Germline | H_FR1 | H_CDR1 | H_FR2 | H_CDR2 | H_FR3 | H_CDR3 | H_FR4 |
| | VH3\|3-11/D6\|6-6\|RF2/JH3 | | QVQLVES-GGGLVKPGGSLR LSCAASG-FIFS | D-----YYMS | WIRQAPGK GLEWVS | YISSS--- GSTIYYAD SVKG | RFTISRDNAKNSLYLQM NSLRAEDTAVYYCAR | SIAAR------------ ------DAFDI | WGQGTM VTVSS |
| iPS:43 4011 | 21-225_48B10 | VH3\|3-11/D6\|6-6\|RF2/JH3 | .............<br>.............<br>........ | .......F.T | ........Q<br>...... | ....A...<br>.GA.....<br>.... | ................<br>...............I | AV..P...........<br>.......GV... | ......<br>..... |
| | | Germline | H_FR1 | H_CDR1 | H_FR2 | H_CDR2 | H_FR3 | H_CDR3 | H_FR4 |
| | VH3\|3-11/D6\|6-6\|RF2/JH4 | | QVQLVES-GGGLVKPGGSLR LSCAASG-FIFS | D-----YYMS | WIRQAPGK GLEWVS | YISSS--- GSTIYYAD SVKG | RFTISRDNAKNSLYLQM NSLRAEDTAVYYCAR | SIAAR-------------- ------YFDY | WGQGTL VTVSS |
| iPS:43 4015 | 21-225_48F12 | VH3\|3-11/D6\|6-6\|RF2/JH4 | .............<br>.............<br>........ | .......F.T | ........Q<br>...... | ....A...<br>.GA.....<br>.... | ..............F...<br>...............I | AV..P...........<br>.......GA..I | ......<br>..... |
| iPS:43 4017 | 21-225_48G12 | VH3\|3-11/D6\|6-6\|RF2/JH4 | .............<br>.............<br>........ | .......F.T | ........Q<br>...... | ....A...<br>.GA.....<br>.... | ..............F...<br>...............I | AV..P...........<br>.......GA..I | ......<br>..... |
| | | Germline | H_FR1 | H_CDR1 | H_FR2 | H_CDR2 | H_FR3 | H_CDR3 | H_FR4 |
| | VH3\|3-23/D6\|6-13\|RF2/JH4 | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | GIAAA------------ ------GYFDY | WGQGTL VTVSS |
| iPS:43 4023 | 21-225_49F1 | VH3\|3-23/D6\|6-13\|RF2/JH4 | .............<br>.............<br>........ | ......... | ..D...<br>.... | V...<br>....F...<br>.... | ..............S...<br>...............S | A....G...........<br>......AH.... | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| IPS:43 6246 | 21-225_201G6 | VH3\|3-23\|D6\|6-13\|RF2\|JH4 | ............ ............V ........ | .......... | ........ ...... .... | T....... .VR..... .... | ...............F... ................ | .G.RSSG............ ......WFH... | ..... ..... |
| IPS:43 6254 | 21-225_202C12 | VH3\|3-23\|D6\|6-13\|RF2\|JH4 | ............ ............ ........ | .......... | ........ ...... .... | T....... .VR..... .... | .S...........F... ................ | .G.RSSG............ ......WFH... | ..... ..... |
| IPS:43 6304 | 21-225_201F3 | VH3\|3-23\|D6\|6-13\|RF2\|JH4 | ............ ......Q..... ........ | .......... | ........ ...... .... | T....... .VR..... .... | ..........N...F... ................ | .G.RSSG............ ......WFH... | ..... ..... |
| IPS:43 6334 | 21-225_208G3 | VH3\|3-23\|D6\|6-13\|RF2\|JH4 | ............ ............ ........ | .......... | ........ ...... .... | T....... .VR..... .... | .S............F... ......K....... | .G.RSSG............ ......WFH... | ..... ..... |
| | | Germline | H_FR1 | H_CDR1 | H_FR2 | H_CDR2 | H_FR3 | H_CDR3 | H_FR4 |
| | VH3\|3-23\|D6\|6-19\|RF2\|JH5 | | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | CIAVAG------------NWFDP | WGQGTL VTVSS |
| IPS:43 4027 | 21-225_49H5 | VH3\|3-23\|D6\|6-19\|RF2\|JH5 | ............ ............ ........ | ..........T | .....T... ...... .... | ......... ..NSF... .... | ..........E....... ................ | AR................. ......SH.... | ..... |
| IPS:43 4061 | 21-225_51C7 | VH3\|3-23\|D6\|6-19\|RF2\|JH5 | ............ ............ ......I. | N........T | .....T... ...... | V..A.... ..NSF... .... | ..........E..F.... ................ | AR................. ......SH.... | ..... |
| IPS:43 4167 | 21-225_50F3 | VH3\|3-23\|D6\|6-19\|RF2\|JH5 | ............ ............ .......R | T........T | ........ ...... | ......... .VNSF... .... | ..........E....... ................ | AR................. ......SH.... | ..... |
| IPS:43 4455 | 21-225_72F5 | VH3\|3-23\|D6\|6-19\|RF2\|JH5 | ............ ............ ........ | ..........I | ........ ...... | T....... ..Y..S.. .... | ................ ................R | R...T............. ......TE.Y.. | ..... |
| IPS:43 7232 | 21-225_63E1 | VH3\|3-23\|D6\|6-19\|RF2\|JH5 | ...M........ G.S........T .........T | T.....S... | ........ ...... | ......... .AN.F... .... | ...VT........... ...T.........V. | V................. ......GHF... | ..... |
| | | Germline | H_FR1 | H_CDR1 | H_FR2 | H_CDR2 | H_FR3 | H_CDR3 | H_FR4 |
| | VH3\|3-21\|D1\|1-1\|RF2\|JH4 | | EVQLVES-GGGLVKPGGSLR LSCAASG-FTFS | S-----YSMN | WVRQAPGK GLEWVS | SISSS--- SSYIYYAD SVKG | RFTISRDNAKNSLYLQM NSLRAEDTAVYYCAR | VQLER----------YFDY | WGQGTL VTVSS |

| iPS ID | Clone | V gene | | | | | | | |
|---|---|---|---|---|---|---|---|---|---|
| iPS:43 4043 | 21-225_50G10 | VH3\|3-21/D1\|1-1\|RF2/JH4 | ..........<br>..........<br>........ | .......... | ........<br>...... | ...G....<br>........<br>.... | ................<br>................ | .AT--<br>................<br>...-... | ......<br>..... |
| iPS:43 4085 | 21-225_52E3 | VH3\|3-21/D1\|1-1\|RF2/JH4 | ..........<br>..........<br>........ | .......K.. | ........<br>...... | ...G....<br>N.S.....<br>.... | .........E.......<br>................ | .SS--<br>................<br>...-N.. | ......<br>..... |
| iPS:43 4101 | 21-225_52H12 | VH3\|3-21/D1\|1-1\|RF2/JH4 | ..........<br>..........<br>........ | .......... | ........<br>...... | ...G....<br>.T......<br>.... | ...V...........V<br>................ | .NS--<br>................<br>...-... | ......<br>..... |
| iPS:43 4187 | 21-225_56A5 | VH3\|3-21/D1\|1-1\|RF2/JH4 | ..........<br>..........<br>........ | .......... | ........<br>...... | ...G....<br>........<br>.... | ................<br>................ | .AT--<br>................<br>...-... | ......<br>..... |
| iPS:43 4265 | 21-225_57B2 | VH3\|3-21/D1\|1-1\|RF2/JH4 | ..........<br>..........<br>........ | .......... | ........<br>...... | ...G....<br>....N.T.<br>.... | ................<br>................ | .AG--<br>................<br>...-... | ......<br>..... |
| iPS:43 4439 | 21-225_70E12 | VH3\|3-21/D1\|1-1\|RF2/JH4 | ..........<br>..........<br>........ | .......... | ........<br>...... | ...GN...<br>.T....T.<br>.... | ................<br>D..T............ | .AA--<br>................<br>...-..C | ......<br>..... |
| iPS:43 5515 | 21-225_157E4 | VH3\|3-21/D1\|1-1\|RF2/JH4 | ..........<br>..........<br>........ | .......T.. | ........<br>...... | ...G....<br>........<br>.... | ................<br>...K........... | .AT--<br>................<br>...-... | ......<br>..... |
| iPS:43 5535 | 21-225_157H10 | VH3\|3-21/D1\|1-1\|RF2/JH4 | ..........<br>..........<br>........ | .......T.. | ........<br>...... | ...G....<br>....N...<br>.... | ................<br>................ | .AH--<br>................<br>...-... | ......<br>..... |
| iPS:43 7224 | 21-225_56H1 | VH3\|3-21/D1\|1-1\|RF2/JH4 | ..........<br>..........<br>........ | N......R.. | ....G...<br>....I. | ...G....<br>.TD.....<br>.... | ................<br>................ | .AS--<br>................<br>...-... | ......<br>..... |
| iPS:39 2620 | 21-225_17H5 | VH3\|3-21/D1\|1-1\|RF2/JH4 | ..........<br>..........<br>........ | .......... | ........<br>...... | ...G....<br>.T......<br>.... | ................<br>................ | .AS--<br>................<br>...-... | ......<br>..... |
| iPS:39 2632 | 21-225_16A11 | VH3\|3-21/D1\|1-1\|RF2/JH4 | ..........<br>..........<br>........ | .......... | ........<br>...... | ...G....<br>..L.....<br>.... | ................<br>................ | .AA--<br>................<br>...-... | ......<br>..... |
| iPS:39 2746 | 21-225_20H7 | VH3\|3-21/D1\|1-1\|RF2/JH4 | ..H.......<br>..........<br>........ | .......... | ........<br>...... | ...G....<br>..F.....<br>.... | ................<br>................ | .AA--<br>................<br>...-L.. | ......<br>..... |

| | | | | | | | | | |
|---|---|---|---|---|---|---|---|---|---|
| iPS:39 2782 | 21-225_22B12 | VH3\|3-21\|D1\|1-1\|RF2/JH4 | . . . . . . . . . . . .<br>. . . . . . . . . . .<br>. . . . . . . | . . . . . . . . . . | . . . . . . . .<br>. . . . . . | . . .G. . . .<br>. . .T. . . .<br>. . . . | . . . . . . . . . . . . . . . . | .AA--<br>. . . . . . . . . . . . . . . . . . . .<br>. . .-.L.S | . . . . . . |
| iPS:39 2916 | 21-225_27C5 | VH3\|3-21\|D1\|1-1\|RF2/JH4 | . . . . . . . . . . . .<br>. . . . . . . . . . .<br>. . . . . . . | . . . . . . . . . . | . . . . . . . .<br>. . . . . . | .T. . . . . .<br>D. . . . . . .<br>. . . . | . . . . . . . . . . . . . . . . | .AS--<br>. . . . . . . . . . . . . . . . . . . .<br>. . .-. .C | . . . . . . |
| iPS:39 2976 | 21-225_27H12 | VH3\|3-21\|D1\|1-1\|RF2/JH4 | . . . . . . . . . . . .<br>. . . . . . . . . . .<br>. . . . . . . | . . . . . . . .L. | . . . . . . . .<br>. . . . . . | . . .G. . . .<br>. .N. . .T.<br>. . . . | . . . . . . . . . . . . . .F. . . | .AS--<br>. . . . . . . . . . . . . . . . . . . .<br>. . .-. . . | . . . . . . |
| iPS:39 3120 | 21-225_35H8 | VH3\|3-21\|D1\|1-1\|RF2/JH4 | . . . . . . . . . . . .<br>. . . . . . . . . . .<br>. . . . . . . | . . . . . . . . . . | . . . . . . . .<br>. . . . . . | . . .GT. . .<br>G.F. . . . .<br>. . . . | . . . . . . . . . .K.V. . . . | .SG--<br>. . . . . . . . . . . . . . . . . . . .<br>. . .-. . . | . . . . . . |
| iPS:39 3836 | 21-225_15A2 | VH3\|3-21\|D1\|1-1\|RF2/JH4 | . . . . . . . . . . . .<br>. . . . . . . . . . .<br>. . . . . . . | . . . . . . .T. . | . . . . . . . .<br>. . . . . . | . . .G. . . .<br>G. . . . . . .<br>. . . . | .A. . . . . . . . . . . . . . | .AS--<br>. . . . . . . . . . . . . . . . . . . .<br>. . .-. . . | . . . . . . |
| iPS:39 3894 | 21-225_5E11 | VH3\|3-21\|D1\|1-1\|RF2/JH4 | . . . . . . . . . . . .<br>. . . . . . . . . . .<br>. . . . . . . | . . . . . . . . . . | . . . . . . . .<br>. . . . . . | . . .G. . . .<br>.T. . . . . .<br>. . . . | . . . . . . . . . .F. . . . | .AS--<br>. . . . . . . . . . . . . . . . . . . .<br>. . .-. . . | . . . . |
| iPS:39 3896 | 21-225_2A4 | VH3\|3-21\|D1\|1-1\|RF2/JH4 | . . . . . . . . . . . .<br>. . . . . . . . . . .<br>. . . . . . . | . . . . . . . . . . | . . . . . . . .<br>. . . . . . | . . .G. . . .<br>.T. . . . .<br>. . . . | .IA. . . . . . . . . . . . | .AS--<br>. . . . . . . . . . . . . . . . . . . .<br>. . .-. . . | . . . . . . |
| iPS:39 3914 | 21-225_16B8 | VH3\|3-21\|D1\|1-1\|RF2/JH4 | . . . . . . . . . . . .<br>. . . . . . . . . . .<br>. . . . . . . | . . . . . . . . . . | . . . . . . . .<br>. . . .I. | . . .G. . . .<br>.T. . . . . .<br>. . . . | . . . . . . . . . . . . . . . . | .AS--<br>. . . . . . . . . . . . . . . . . . . .<br>. . .-. . . | . . . . . . |
| iPS:39 3944 | 21-225_14D6 | VH3\|3-21\|D1\|1-1\|RF2/JH4 | . . . . . . . . . . . .<br>. . . . . . . . . . .<br>. . . . . . . | . . . . . . .T. . | . . . . . . . .<br>. . . . . . | . . .G. . . .<br>G. . . . . . .<br>. . . . | . . .M. . . . . . . . . . . . | .AA--<br>. . . . . . . . . . . . . . . . . . . .<br>. . .-. .S | . . . . . .<br>.S. . . |
| iPS:39 3952 | 21-225_1F1 | VH3\|3-21\|D1\|1-1\|RF2/JH4 | . . . . . . . . . . . .<br>. . . . . . . . . . .<br>. . . . . . . | . . . . . . .N. . | . . . . . . . .<br>. . . . . . | . . . . . . . .<br>. . . . . . . .<br>. . . . | . . . . . . . . . . . . . . . . | .N.--<br>. . . . . . . . . . . . . . . . . . . .<br>. . .-. . . | . . . . . . |
| iPS:39 4033 | 21-225_5F4 | VH3\|3-21\|D1\|1-1\|RF2/JH4 | . . . . . . . . . . . .<br>. . . . . . . . . .V<br>. . . . . . . | . . . . . . . . . . | . . . . . . . .<br>. . . . . . | . . .G. . . .<br>. . . . . . .<br>. . . . | . . . . . . . . . . . . .F. . . | .NN--<br>. . . . . . . . . . . . . . . . . . . .<br>. . .-. . . | . . . . . . |
| iPS:39 4069 | 21-225_16H1 | VH3\|3-21\|D1\|1-1\|RF2/JH4 | . . . . . . . . . . . .<br>. . . . . . . . . . .<br>. . . . . . . | . . . . . . . . . . | . . . . . . . .<br>. . . . . . | . . .G. . . .<br>.T. . . . .<br>. . . . | . . . . . . . . . . . . . . . . | .AA--<br>. . . . . . . . . . . . . . . . . . . .<br>. . .-. . . | . . . . . . |

| | | Germline | H_FR1 | H_CDR1 | H_FR2 | H_CDR2 | H_FR3 | H_CDR3 | H_FR4 |
|---|---|---|---|---|---|---|---|---|---|
| iPS:39 8482 | 21-225_17H6 | VH3\|3-21\|D1\|1-1\|RF2/JH4 | ............ ............ ........ | .......... | .......R ...... .... | ...G.... ........ .... | ....F............ ................ | .AS-- ................ ...-... | ...... ..... |
| iPS:39 8500 | 21-225_23A11 | VH3\|3-21\|D1\|1-1\|RF2/JH4 | ............ ............ ........ | .......... | .I...... ...... .... | ...G.... .T...... .... | .IA............ ................ | .AS-- ................ ...-... | ...... ..... |
| iPS:39 8526 | 21-225_32B3 | VH3\|3-21\|D1\|1-1\|RF2/JH4 | ............ ............ ........ | .......... | .......... ...... .... | ...G.... .T...... .... | ................ ................ | .AG-- ...-... | ...... ..... |
| iPS:40 2221 | 21-225_2C12 | VH3\|3-21\|D1\|1-1\|RF2/JH4 | ............ ............ ........ | .......... | .......... ...... .... | ...G.... ...M.... .... | ..........D...... ................ | .N.-- ...-... | ...... ..... |
| | | **Germline** | **H_FR1** | **H_CDR1** | **H_FR2** | **H_CDR2** | **H_FR3** | **H_CDR3** | **H_FR4** |
| | **VH1\|1-02\|D2\|2-15\|RF2/JH6** | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | GYCSGGSCYS-------- ---YYYYYGMDV | WGQGTT VTVSS |
| iPS:43 4047 | 21-225_50A12 | VH1\|1-02\|D2\|2-15\|RF2/JH6 | ............ ............ ........ | .......H.IN | ........ .P...A .... | .V...... .....S.. .... | ..............V.... | .GQL..FN-- ...........- ........ | ...... ..... |
| iPS:43 4067 | 21-225_51H8 | VH1\|1-02\|D2\|2-15\|RF2/JH6 | ............ ............ ........ | ......H..N | ........ ...... Q... | .V...... ...S.S.. .... | ...S............V... | .GQL..FN-- ...........- F....... | ...... ..... |
| iPS:43 4197 | 21-225_56C7 | VH1\|1-02\|D2\|2-15\|RF2/JH6 | ............ ............ ........ | .......H.IN | ........ .....A .... | .V...... .....S.. .... | ..............V... | .GQL..FN-- ...........- ........ | ...... ..... |
| | | **Germline** | **H_FR1** | **H_CDR1** | **H_FR2** | **H_CDR2** | **H_FR3** | **H_CDR3** | **H_FR4** |
| | **VH3\|3-21\|D2\|2-15\|RF3/JH6** | | EVQLVES-GGGLVKPGGSLR LSCAASG-FTFS | S-----YSMN | WVRQAPGK GLEWVS | SISSS--- SSYIYYAD SVKG | RFTISRDNAKNSLYLQM NSLRAEDTAVYYCAR | DIVVVVAAT ---YYYYYGMDV | WGQGTT VTVSS |
| iPS:43 4049 | 21-225_50B12 | VH3\|3-21\|D2\|2-15\|RF3/JH6 | ............ ............ ........ | ......H... | ........ ...... .... | ....... .N...... .... | .......Y......... | .RSI...GP......... ...-WD...... | ...... ..... |
| | | **Germline** | **H_FR1** | **H_CDR1** | **H_FR2** | **H_CDR2** | **H_FR3** | **H_CDR3** | **H_FR4** |
| | **VH3\|3-23\|D1\|1-20\|RF1/JH3** | | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | GITGT------------ ------DAFDI | WGQGTM VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| IPS:43 4055 | 21-225_51B4 | VH3/3-23/D1/1-20/RF1/J H3 | ............ .......S.... .......R | .......V.. | ........ ...... .... | ...R... .SN.F.T. | ................ ................ | ....SH........... .......G.... | ...... ..... |
| | | VH3/3-23/D7/7-27/RF1/JH4 | EVQLLES-GGGLVQPGGSLRLSCAASG-FIFS | S----YAMS | WVRQAPGKGLEWVS | AISGSGGSTYYADSVKG | RFTISRDNSKNTLYLQMNSLRAEDTAVYYCAK | LTCY----FDY | WGQGTLVTVSS |
| IPS:43 4059 | 21-225_51C5 | VH3/3-23/D7/7-27/RF1/J H4 | ............ ............ ........ | .......V.. | ....T... ...... | TM...... ..R..... ..N. | ...V............ S.............R | V.A- ........... ........ | ...... ..... |
| IPS:43 4213 | 21-225_60A4 | VH3/3-23/D7/7-27/RF1/J H4 | ............ ............ ........ | .......V.. | ........ ...... | S....... ..W.N... .... | ...T............ ..............R | ...- ............ ........ | ...... ..... |
| IPS:43 4215 | 21-225_60F7 | VH3/3-23/D7/7-27/RF1/J H4 | ............ ............ ........ | .......V.. | ........ ...... | G....... .NR..... .... | ................ .......S.....GS | .G-- ............ .....-ID | ...... ..... |
| IPS:43 4241 | 21-225_61E6 | VH3/3-23/D7/7-27/RF1/J H4 | ............ ............ .......R | ........... | ........ ...... | .T...... .VN.F... .... | ................ ................ | .ELG........... ........I... | ...... ..... |
| IPS:43 4261 | 21-225_57B6 | VH3/3-23/D7/7-27/RF1/J H4 | ............ ............ .......R | ........... | ........ ...... | ........ ..N.F... .... | ................ ................ | .ELG........... ........I... | ...... ..... |
| IPS:43 4301 | 21-225_58F11 | VH3/3-23/D7/7-27/RF1/J H4 | ............ ............ ........ | ........... | ........ ...... | ........ ..N.F... .... | .......Y........ ................ | FF.MVG........... ......AGF... | ...... ..... |
| IPS:43 5523 | 21-225_157G5 | VH3/3-23/D7/7-27/RF1/J H4 | ............ ............ ........ | .......V.. | ........ ..D... | .M...... ..R..... .... | ................ ................ | Y.W- ............ .....NG. | ...... ..... |
| IPS:43 5659 | 21-225_167D12 | VH3/3-23/D7/7-27/RF1/J H4 | ............ ............ ........ | .......V.. | ........ ...... | .M...... ..R..... .... | ................ ................ | Y.W- ............ .....NG. | ...... ..... |
| IPS:43 5765 | 21-225_177D3 | VH3/3-23/D7/7-27/RF1/J H4 | ............ ............ ........ | .......V.N | ........ ...... | GM...... ..R..... ...D | ..............S... ..............R | V.F- ............ ........ | ...... ..... |

3227

| iPS:43 7216 | 21-225_51D5 | VH3\|3-23\|D7\|7-27\|RF1\|JH4 | ............ ............ ........ | .......V.. | ....T.... ...... ..N. | TM...... ..R..... | ...V............ S.............R | V.A- ................. ........ | ...... .... |
| --- | --- | --- | --- | --- | --- | --- | --- | --- | --- |
| | | Germline | H_FR1 | H_CDR1 | H_FR2 | H_CDR2 | H_FR3 | H_CDR3 | H_FR4 |
| | **VH3\|3-21\|D5\|5-24\|RF2\|JH4** | | EVQLVES-GGGLVKPGGSLRLSCAASG-FIFS | S-----YSMN | WVRQAPGKGLEWVS | SISSS---SSYIYYADSVKG | RFTISRDNAKNSLYLQMNSLRAEDTAVYYCAR | *RWLQ------------------LYFDY | WGQGTLVTVSS |
| iPS:43 4063 | 21-225_51G7 | VH3\|3-21\|D5\|5-24\|RF2\|JH4 | ............ ............ ........ | .......... | ........ ...... ........ | ........ ........ .... | .................. ................ | A.L-- ................... ..---.. | ...... .... |
| | | Germline | H_FR1 | H_CDR1 | H_FR2 | H_CDR2 | H_FR3 | H_CDR3 | H_FR4 |
| | **VH3\|3-23\|D7\|7-27\|RF3\|JH4** | | EVQLLES-GGGLVQPGGSLRLSCAASG-FIFS | S-----YAMS | WVRQAPGKGLEWVS | AISGS---GGSTYYADSVKG | RFTISRDNSKNTLYLQMNSLRAEDTAVYYCAK | NWCY----------------FDY | WGQGTLVTVSS |
| iPS:43 4083 | 21-225_52H2 | VH3\|3-23\|D7\|7-27\|RF3\|JH4 | ............ ............ ........ | R.....N... | .......M ...... | ....R... ..N.F... .... | ...V.........F... ................ | .GREQ............. ........WL.. | ...... .... |
| | | Germline | H_FR1 | H_CDR1 | H_FR2 | H_CDR2 | H_FR3 | H_CDR3 | H_FR4 |
| | **VH3\|3-30.3\|D6\|6-6\|RF2\|JH6** | | QVQLVES-GGGVVQPGRSLRLSCAASG-FIFS | S-----YAMH | WVRQAPGKGLEWVA | VISYD---GSNKYYADSVKG | RFTISRDNSKNTLYLQMNSLRAEDTAVYYCAR | SIAARYY-----------YYYGMDV | WGQGTTVTVSS |
| iPS:43 4087 | 21-225_52F6 | VH3\|3-30.3\|D6\|6-6\|RF2\|JH6 | ............ ............ ........ | .......G.. | ........ ...... .... | I...G... ........ | .................. ......D........ | RS...P- ................-G..... | ...... .... |
| iPS:43 4111 | 21-225_53H2 | VH3\|3-30.3\|D6\|6-6\|RF2\|JH6 | ............ ............ ........ | .......G.. | ........ .P.... .... | ....G... .....H.. | .................. ................ | RG...P- ................-G..... | ...... .... |
| iPS:43 4121 | 21-225_53F6 | VH3\|3-30.3\|D6\|6-6\|RF2\|JH6 | ............ ............ ........ | .......G.. | ........ ...... .... | ....G... .....D.. .... | .................. T............... | RR...P- ................-G..... | ...... .... |
| iPS:43 4163 | 21-225_50H1 | VH3\|3-30.3\|D6\|6-6\|RF2\|JH6 | ............ ............ ........ | .......G.. | ........ .....S .... | I...G... .....D.. | .................. T............... | RR...P- ................-G..... | ....I. .... |

| | | | H_FR1 | H_CDR1 | H_FR2 | H_CDR2 | H_FR3 | H_CDR3 | H_FR4 |
|---|---|---|---|---|---|---|---|---|---|
| iPS:39 4035 | 21-225_5G9 | VH3|3-30.3/D6|6-6|RF2/JH6 | ............ ............ ........ | N......G.. ...... | ........ ...... .... | I...A... ........ | ................ .............V. | R.T..L- ................- ...... | ...... ..... |
| Germline | | | **H_FR1** | **H_CDR1** | **H_FR2** | **H_CDR2** | **H_FR3** | **H_CDR3** | **H_FR4** |
| | **VH3|3-33/D2|2-8|RF1/JH4** | | QVQLVES-GGGVVQPGRSLRLSCAASG-FIFS | S-----YGMH | WVRQAPGK GLEWVA | VIWYD---GSNKYYAD SVKG | RFTISRDNSKNTLYLQMNSLRAEDTAVYYCAR | RILY*WC--------------MLYYFDY | WGQGTL VTVSS |
| iPS:43 4095 | 21-225_52F10 | VH3|3-33/D2|2-8|RF1/JH4 | ............ ............ ........ | F......... | ........ ...... .... | ...D.... ........ | ..............F...M............. | DS..SS- .................- SWL... | ...... ..... |
| Germline | | | **H_FR1** | **H_CDR1** | **H_FR2** | **H_CDR2** | **H_FR3** | **H_CDR3** | **H_FR4** |
| | **VH3|3-23/D4|4-23|RF3/JH4** | | EVQLLES-GGGLVQPGGSLRLSCAASG-FIFS | S-----YAMS | WVRQAPGK GLEWVS | AISGS---GGSTYYAD SVKG | RFTISRDNSKNTLYLQMNSLRAEDTAVYYCAK | TTVVT-----------------YFDY | WGQGTL VTVSS |
| iPS:43 4107 | 21-225_53E2 | VH3|3-23/D4|4-23|RF3/JH4 | ............ I........... .......R | .......... | ........ ...... .... | ..N.F... .... | .T...D......... | KV.D.A............ .......MAL.. | ...... ..... |
| iPS:43 4181 | 21-225_56B2 | VH3|3-23/D4|4-23|RF3/JH4 | ............ I........... .......R | .......... | ........ ...... .... | ..N.F... .... | .T...D......... | KV.D.A............ .......MAL.. | ...... ..... |
| Germline | | | **H_FR1** | **H_CDR1** | **H_FR2** | **H_CDR2** | **H_FR3** | **H_CDR3** | **H_FR4** |
| | **VH3|3-23/D1|1-26|RF1/JH3** | | EVQLLES-GGGLVQPGGSLRLSCAASG-FIFS | S-----YAMS | WVRQAPGK GLEWVS | AISGS---GGSTYYAD SVKG | RFTISRDNSKNILYLQMNSLRAEDTAVYYCAK | GIVGAT----------DAFDI | WGQGTM VTVSS |
| iPS:43 4117 | 21-225_53C6 | VH3|3-23/D1|1-26|RF1/JH3 | ............ ............ ........ | .........N | ........ ...... .... | ..A..... .... | ................ | PL...H............ ..........E. | ...... ..... |
| iPS:39 2984 | 21-225_30E11 | VH3|3-23/D1|1-26|RF1/JH3 | ..........DM ............ ........ | I......... | ........ ...... .... | V....... ...SF... .... | ...............T. | DR.K.H............ .........G... | ...... ..... |
| iPS:39 3114 | 21-225_33G12 | VH3|3-23/D1|1-26|RF1/JH3 | ..........DM ............ ........ | .......... | ........ ...... .... | V....... ...SF... .... | ................ | DR.R.H............ .........G... | ...... ..... |
| Germline | | | **H_FR1** | **H_CDR1** | **H_FR2** | **H_CDR2** | **H_FR3** | **H_CDR3** | **H_FR4** |
| | **VH3|3-33/D5|5-24|RF2/JH4** | | QVQLVES-GGGVVQPGRSLRLSCAASG-FIFS | S-----YGMH | WVRQAPGK GLEWVA | VIWYD---GSNKYYAD SVKG | RFTISRDNSKNTLYLQMNSLRAEDTAVYYCAR | *RWLQ------------LYFDY | WGQGTL VTVSS |

| IPS:43 4127 | 21-225_53H8 | VH3\|3-33/D5\|5-24\|RF2/JH4 | .............<br>.............<br>........ | .......... | .........<br>......<br>.... | ........<br>........ | .................<br>................. | A.IG-<br>.................<br>..-...S | ......<br>..... |
| | | Germline | **H_FR1** | **H_CDR1** | **H_FR2** | **H_CDR2** | **H_FR3** | **H_CDR3** | **H_FR4** |
| | **VH3\|3-23/D4\|4-17\|RF2/JH4** | | EVQLLES-<br>GGGLVQPGGSLR<br>LSCAASG-FIFS | S-----YAMS | WVRQAPGK<br>GLEWVS | AISGS---<br>GGSTYYAD<br>SVKG | RFTISRDNSKNTLYLQM<br>NSLRAEDTAVYYCAK | DYGDY---------<br>--------YFDY | WGQGTL<br>VTVSS |
| IPS:43 4147 | 21-225_55E1 | VH3\|3-23/D4\|4-17\|RF2/JH4 | .............<br>.............<br>........ | .......... | .........<br>......<br>.... | ....R...<br>.S..F...<br>.... | .................<br>..........F.... | .H.IVG............<br>......TI.... | ......<br>..... |
| IPS:43 5303 | 21-225_146A6 | VH3\|3-23/D4\|4-17\|RF2/JH4 | .............<br>.............<br>.......N | .........N | .........<br>......<br>.... | ........<br>..N.F...<br>.... | ................. | KDN..VW...........<br>.....GSP.... | ......<br>..... |
| IPS:43 5335 | 21-225_147D10 | VH3\|3-23/D4\|4-17\|RF2/JH4 | .............<br>.............<br>........ | .......... | .........<br>......<br>.... | ....R...<br>..N.F...<br>.... | ................. | KDY..VW...........<br>.....GSP.... | ......<br>..... |
| IPS:43 5339 | 21-225_147D12 | VH3\|3-23/D4\|4-17\|RF2/JH4 | ..S.........<br>.............<br>........ | .......... | .........<br>......<br>.... | ....R...<br>..N.F...<br>.... | ................. | KDY..VW...........<br>.....GSP.... | ......<br>..... |
| IPS:43 5343 | 21-225_148E2 | VH3\|3-23/D4\|4-17\|RF2/JH4 | .............<br>.............<br>........ | .......... | .........<br>......<br>.... | ....R...<br>..N.F...<br>.... | ................. | KDY..VW...........<br>.....GSP.... | ......<br>..... |
| IPS:43 5381 | 21-225_149C6 | VH3\|3-23/D4\|4-17\|RF2/JH4 | .............<br>.............<br>........ | .......... | .........<br>......<br>.... | ........<br>..N.F...<br>.... | ................H. | KDY..VW...........<br>.....GSP.... | ......<br>..... |
| IPS:43 5391 | 21-225_149F6 | VH3\|3-23/D4\|4-17\|RF2/JH4 | .............<br>.............<br>........ | .......... | .........<br>......<br>.... | ....R...<br>..N.F...<br>.... | ................. | KDY..VW...........<br>.....GSP.... | ......<br>..... |
| IPS:43 5395 | 21-225_149D11 | VH3\|3-23/D4\|4-17\|RF2/JH4 | .............<br>.............<br>........ | .......... | .........<br>......<br>.... | ....R...<br>..N.F...<br>.... | ...............R.. | KDY..VW...........<br>.....GSP.... | ......<br>..... |
| IPS:43 5403 | 21-225_150C5 | VH3\|3-23/D4\|4-17\|RF2/JH4 | .............<br>.............<br>........ | .......... | .........<br>......<br>.... | ........<br>..N.F...<br>.... | ................. | KDY..VW...........<br>.....GSP.... | ......<br>..... |

| | | | | | | | | | |
|---|---|---|---|---|---|---|---|---|---|
| IPS:43 5447 | 21-225_152H7 | VH3\|3-23/D4\|4-17\|RF2/JH4 | ............<br>............<br>.......N | .........N | ........<br>......<br>.... | ......<br>..N.F...<br>.... | ................ | KDN..VW..........<br>.....GSP.... | .....<br>..... |
| IPS:43 5453 | 21-225_152G10 | VH3\|3-23/D4\|4-17\|RF2/JH4 | ............<br>............<br>........ | .......... | ........<br>......<br>.... | ....R...<br>..N.F...<br>.... | ................ | KDY..VW..........<br>.....GSP.... | ......<br>..... |
| IPS:43 5483 | 21-225_155A4 | VH3\|3-23/D4\|4-17\|RF2/JH4 | ............<br>............<br>.......N | .......... | ........<br>......<br>.... | ......<br>..N.F...<br>.... | ................ | KDY..VW..........<br>.....GSP.... | ......<br>..... |
| iPS:43 5485 | 21-225_155B4 | VH3\|3-23/D4\|4-17\|RF2/JH4 | ............<br>............<br>........ | .......... | ........<br>......<br>.... | ......<br>..N.F...<br>.... | ................ | KDY..VW..........<br>.....GSP.... | ......<br>..... |
| IPS:43 5777 | 21-225_178F7 | VH3\|3-23/D4\|4-17\|RF2/JH4 | ..H.........<br>..T.........<br>........ | .........T | ........<br>......<br>.... | V.......<br>..N.F...<br>.... | ................<br>...............R | R...-<br>....... | ......<br>..... |
| iPS:43 5783 | 21-225_179G1 | VH3\|3-23/D4\|4-17\|RF2/JH4 | ..H.........<br>..T.........<br>........ | .........T | ........<br>......<br>.... | V...F...<br>..N.F...<br>.... | ................<br>.............R | R...-<br>....... | ......<br>..... |
| iPS:43 5833 | 21-225_190D12 | VH3\|3-23/D4\|4-17\|RF2/JH4 | ............<br>............<br>........ | .......... | ........<br>.....D.<br>.... | ..I.N...<br>..R.....<br>.... | ................ | .M.R.S...........<br>......YGF... | ......<br>.... |
| IPS:43 6156 | 21-225_197C8 | VH3\|3-23/D4\|4-17\|RF2/JH4 | ............<br>............<br>........ | ......S..T | ........<br>......<br>.... | ..I.N...<br>..RA....<br>.... | ................<br>.....T......... | .R.YSRIA.........<br>......VAGT... | ......<br>..... |
| Germline | | | **H_FR1** | **H_CDR1** | **H_FR2** | **H_CDR2** | **H_FR3** | **H_CDR3** | **H_FR4** |
| | VH3\|3-21/D1\|1-1\|RF1/JH4 | | EVQLVES-<br>GGGLVKPGGSLR<br>LSCAASG-FTFS | S-----YSMN | WVRQAPGK<br>CLEWVS | SISSS---<br>SSYIYYAD<br>SVKG | RFTISRDNAKNSLYLQM<br>NSLRAEDTAVYYCAR | GTTGT----------<br>----------YFDY | WGQGTL<br>VTVSS |
| IPS:43 4157 | 21-225_55D4 | VH3\|3-21/D1\|1-1\|RF1/JH4 | ............<br>............<br>.......N | .......R.. | ........<br>...... | .NH.D...<br>.... | ................<br>...........L..... | ..---<br>...--.. | ......<br>.S... |
| IPS:43 4243 | 21-225_62C1 | VH3\|3-21/D1\|1-1\|RF1/JH4 | ............<br>............<br>........ | .......... | ........<br>...... | ........<br>.... | ................<br>................I | FG---<br>...--VD | ......<br>..... |

| | Germline | H_FR1 | H_CDR1 | H_FR2 | H_CDR2 | H_FR3 | H_CDR3 | H_FR4 |
|---|---|---|---|---|---|---|---|---|
| iPS:43 5505 / 21-225_157C1 | VH3\|3-21\|D1\|1-1\|RF1/JH4 | ............ ............ ........ | .......,R.. | ....... ...... .... | .M.N.... ..S..... .... | ................ ................ | QAA-- .................. ...-Q.. | ...... ..... |
| iPS:39 2966 / 21-225_32G3 | VH3\|3-21\|D1\|1-1\|RF1/JH4 | ............ ............ ........ | .......... | ........ ...... .... | ...G.... ...... .... | ..................I | .NIA- .................. ...-R.. | ...... ..... |
| **Germline** VH3\|3-33\|D7\|7-27\|RF3/JH4 | | QVQLVES-GGGVVQPGRSLRLSCAASG-FIFS | S YGMH | WVRQAPGK GLEWVA | VIWYD- GSNKYYAD SVKG | RFTISRDNSKNTLYLQM NSLRAEDTAVYYCAR | NWGY ----------FDY | WGQGTL VTVSS |
| iPS:43 4159 / 21-225_55B8 | VH3\|3-33\|D7\|7-27\|RF3/JH4 | ............ ............ ........ | D......... | ...... ...... | EN...... .... | .................. ..............T. | E.F- ......-.. | ...... ..... |
| iPS:39 3026 / 21-225_32B6 | VH3\|3-33\|D7\|7-27\|RF3/JH4 | ............ ............ ........ | D......... | ......D. ...... | ENT..... .... | .................. .................. | E..- ......-.. | ...... ..... |
| **Germline** VH3\|3-33\|D6\|6-6\|RF3/JH4 | | QVQLVES-GGGVVQPGRSLRLSCAASG-FIFS | S-----YGMH | WVRQAPGK GLEWVA | VIWYD--- GSNKYYAD SVKG | RFTISRDNSKNTLYLQM NSLRAEDTAVYYCAR | V*QLV------------- ---------YFDY | WGQGTL VTVSS |
| iPS:43 4165 / 21-225_50F2 | VH3\|3-33\|D6\|6-6\|RF3/JH4 | ............ ............ ..A..... | .......... | ....T... ...... | ...H.... .... | .................. .................. | DE..G............. .........T... | ...... ..... |
| iPS:43 4191 / 21-225_56B6 | VH3\|3-33\|D6\|6-6\|RF3/JH4 | ............ ............ ........ | .......... | ........ ...... | ...H.... ......V. .... | .................. .................. | DE..G............. .........T... | ...... ..... |
| **Germline** VH1\|1-46\|D4\|4-17\|RF2/JH6 | | QVQLVQS-GAEVKKPGASVKVSCKASG-YTFT | S YYMH | WVRQAPGQ GLEWMG | IINPS- GGSTSYAQ KFQG | RVTMTRDTSTSTVYMEL SSLRSEDTAVYYCAR | DYGDYYY ------YYYGMDV | WGQGTT VTVSS |
| iPS:43 4171 / 21-225_50G4 | VH1\|1-46\|D4\|4-17\|RF2/JH6 | ............ .E......... ........ | ........I. | ........ ...... | V...... N.R..... .... | .................. .................. | .R..G............. .....F...... | ...... ..... |
| **Germline** VH3\|3-30.3\|D4\|4-17\|RF2/JH3 | | QVQLVES-GGGVVQPGRSLRLSCAASG-FIFS | S-----YAMH | WVRQAPGK GLEWVA | VISYD--- GSNKYYAD SVKG | RFTISRDNSKNTLYLQM NSLRAEDTAVYYCAR | DYGDY------------- --------DAFDI | WGQGIM VTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:43 4175 | 21-225_55A11 | VH3\|3-30.3/D4\|4-17\|RF2/JH3 | ............ ............ ........ | .......G.. | ....T... ...... ..R. | ....V... ..T..... | ................ ....T.......... | GR.R.SDY.......... .....GH..... | ...... ..... |
| | Germline | | H_FR1 | H_CDR1 | H_FR2 | H_CDR2 | H_FR3 | H_CDR3 | H_FR4 |
| | VH1\|1-02/D1\|1-1\|RF1/JH2 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | CTTGTY-------- -------WYFDL | WGRGTL VTVSS |
| iPS:43 4193 | 21-225_56C6 | VH1\|1-02/D1\|1-1\|RF1/JH2 | ............ ............ ........ | D......H.. | ........ ...... .... | ........ R.....V. | ..A..N.......... .G............. | DG.S-- ................ .-S..Y | ...... ..... |
| | Germline | | H_FR1 | H_CDR1 | H_FR2 | H_CDR2 | H_FR3 | H_CDR3 | H_FR4 |
| | VH1\|1-02/D5\|5-24\|RF1/JH4 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | VEMAT------- -------IYFDY | WGQGTL VTVSS |
| iPS:43 4195 | 21-225_56F6 | VH1\|1-02/D5\|5-24\|RF1/JH4 | ............ ............ ....F... | D........ | ........ ...... R... | ........ ........ | ................ ...............T. | EGATRP.......... .......TG... | ...... ..... |
| | Germline | | H_FR1 | H_CDR1 | H_FR2 | H_CDR2 | H_FR3 | H_CDR3 | H_FR4 |
| | VH3\|3-33/D1\|1-1\|RF2/JH4 | | QVQLVES-GGGVVQPGRSLR LSCAASG-FTFS | S-----YGMH | WVRQAPGK GLEWVA | VIWYD-- GSNKYYAQ SVKG | RFTISRDNSKNTLYLQM NSLRAEDTAVYYCAR | VQLER-------- -------YFDY | WGQGTL VTVSS |
| iPS:43 4203 | 21-225_60E2 | VH3\|3-33/D1\|1-1\|RF2/JH4 | ............ ............ .......N | D........ | ........ ...... .... | I....... EN...... | ................ S.............. | DV.D- ................ ...P... | ...... ..... |
| iPS:43 4229 | 21-225_61H1 | VH3\|3-33/D1\|1-1\|RF2/JH4 | ............ ............ .......N | D........ | ........ ...... .... | I....... E....... | ................ ................ | DV.D- ................ ...P... | ...... ..... |
| | Germline | | H_FR1 | H_CDR1 | H_FR2 | H_CDR2 | H_FR3 | H_CDR3 | H_FR4 |
| | VH1\|1-02/D5\|5-18\|RF3/JH6 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | GYSYGYYY ------YYYGMDV | WGQGTT VTVSS |
| iPS:43 4209 | 21-225_60C3 | VH1\|1-02/D5\|5-18\|RF3/JH6 | ............ ............ .....S.. | ......H.I. | ........ ...Y.. .... | ........ ......V. | ................ .........I...S. | .GLL.ATN.......... ....Y....... | ...... ..... |
| iPS:43 4315 | 21-225_59G7 | VH1\|1-02/D5\|5-18\|RF3/JH6 | ............ ............ .....S.. | ......H.I. | ........ ...Y.. .... | ........ ......V. | ................ .........I...S. | .GLL.ATN.......... ....Y....... | ...... ...T. |

| iPS | Clone | Germline | H_FR1 | H_CDR1 | H_FR2 | H_CDR2 | H_FR3 | H_CDR3 | H_FR4 |
|---|---|---|---|---|---|---|---|---|---|
| iPS:43 4319 | 21-225_59B9 | VH1\|1-02/D5\|5-18\|RF3/JH6 | .........P..<br>...........<br>.....I.. | ......N.I. | .........<br>...Y..<br>.... | ........<br>......V. | ..............N...<br>⌐S...........S.<br>.... | .GLL.AT...........<br>....Y........... | ......<br>..... |
| iPS:44 3003 | 21-225_43F11_ LC2 | VH1\|1-02/D5\|5-18\|RF3/JH6 | ...........<br>...........<br>........ | .........I. | .........<br>......<br>.... | ........<br>.........<br>.... | G.....L...N....D.<br>............... | .GN.F.--<br>................-<br>NHV... | .....P<br>..... |
| iPS:44 3005 | 21-225_43F11_ LC1 | VH1\|1-02/D5\|5-18\|RF3/JH6 | ...........<br>...........<br>........ | .........I. | .........<br>......<br>.... | ........<br>.........<br>.... | G.....L...N....D.<br>............... | .GN.F.--<br>................-<br>NHV... | .....P<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-30.4/D5\|5-12\|RF3/JH4 | | QVQLQES GPGLVKPSQILS LTCIVSG-GSIS | SG  DYYWS | WIRQPPGK GLEWIG | YIYY SGSTYYNP SLKS | RVTISVDTSKNQFSLKL SSVTAADTAVYYCAR | GYSGYD -------YYFDY | WGQGTL VTVSS |
| iPS:43 4217 | 21-225_60E8 | VH4\|4-30.4/D5\|5-12\|RF3/JH4 | ...........<br>.R..A.......<br>........ | RS...S...G | .........<br>......<br>.... | S.......<br>...AS...<br>.... | .........E.....R.<br>................ | LD..W-<br>.................<br>.-S... | ...... |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-23/D3\|3-22\|RF3/JH4 | | EVQLLES- GGGLVQPGGSLR LSCAASG-FIFS | S-----YAMS | WVRQAPGK GLEWVS | AISGS-- GGSTYYAD SVKG | RFTISRDNSKNTLYLQM NSLRAEDTAVYYCAK | ITMIVVV----------- ------ITYFDY | WGQGTL VTVSS |
| iPS:43 4219 | 21-225_60E9 | VH3\|3-23/D3\|3-22\|RF3/JH4 | ...........<br>...........<br>........ | .......... | .........<br>......<br>.... | ........<br>..N.F...<br>.... | ..............F... | FFG..G-<br>..................A<br>G.... | ...... |
| iPS:43 4289 | 21-225_57H12 | VH3\|3-23/D3\|3-22\|RF3/JH4 | ...........<br>...........<br>....L... | .......... | ....D...<br>......<br>.... | ........<br>..N.F.G.<br>.... | ..........K...... | FFG..G-<br>..................A<br>G.... | ...... |
| iPS:43 4297 | 21-225_58A10 | VH3\|3-23/D3\|3-22\|RF3/JH4 | ...........<br>...........<br>.......R | .......... | .........<br>......<br>.... | ........<br>..N.F...<br>.... | ..............F... | FFG..G-<br>..................A<br>G.... | ...... |
| iPS:39 2996 | 21-225_28B1 | VH3\|3-23/D3\|3-22\|RF3/JH4 | ...........<br>...........<br>........ | .......... | .........<br>......<br>.... | ........<br>..N.F...<br>.... | ................ | LGR.A.T...........<br>......GP.... | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-59/D4\|4-11\|RF3/JH4 | | QVQLQES- GPGLVKPSETLS LTCIVSG-GSIS | S-----YYWS | WIRQPPGK GLEWIG | YIYY---- SGSTNYNP SLKS | RVTISVDTSKNQFSLKL SSVTAADTAVYYCAR | TTVT--------------- --------YFDY | WGQGTL VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 4225 | 21-225_60E12 | VH4\|4-59/D4\|4-11\|RF3/JH4 | ............ ........ | .......F.. | .....A.. ...... | R..?.... R....... .... | ...M............ ................ | EGK.GG............ ......VS.... | ...... ..... |
| iPS:43 4227 | 21-225_61A1 | VH4\|4-59/D4\|4-11\|RF3/JH4 | ............ ........ | ......HF.. | .....A.. ...... | R..I.... R....... .... | ...M............ ................ | EGK.GG............ ......VS.... | ...... ..... |
| iPS:43 4267 | 21-225_57F2 | VH4\|4-59/D4\|4-11\|RF3/JH4 | ............ ........ | .......... | .....A.. ...... | R..T.... R....... .... | ...M.I.......... ................ | EGK.GG............ ......VS.... | ...... ..... |
| | | Germline | H_FR1 | H_CDR1 | H_FR2 | H_CDR2 | H_FR3 | H_CDR3 | H_FR4 |
| | **VH3\|3-23/D5\|5-18\|RF3/JH3** | | EVQLLES-GGGLVQPGGSLRLSCAASG-FIFS | S-----YAMS | WVRQAPGKGLEWVS | AISGS---GGSTYYADSVKG | RFTISRDNSKNTLYLQMNSLRAEDTAVYYCAK | GYSYGY--------------DAFDI | WGQGTMVTVSS |
| iPS:43 4239 | 21-225_58F1 | VH3\|3-23/D5\|5-18\|RF3/JH3 | ............ ........ | .......... | .......... ...... | ...TG... ..N..... .... | ................ ................ | RGV..D............ ......F..... | ...... ..... |
| iPS:43 4309 | 21-225_59B5 | VH3\|3-23/D5\|5-18\|RF3/JH3 | ............ ........ | .........N | .......... ...... | ...N.F... .... | ................ | RGV..D............ ......YE.... | ...... ..... |
| | | Germline | H_FR1 | H_CDR1 | H_FR2 | H_CDR2 | H_FR3 | H_CDR3 | H_FR4 |
| | **VH3\|3-33/D5\|5-18\|RF1/JH4** | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | VDTAM--------------VYFDY | WGQGTLVTVSS |
| iPS:43 4245 | 21-225_62H1 | VH3\|3-33/D5\|5-18\|RF1/JH4 | ............ ........ | .......... | ........Q ....M. | I....... ........ .... | ................ ...............I | E.PRT............. .......SCS.. | ...... ..... |
| iPS:43 4323 | 21-225_62H8 | VH3\|3-33/D5\|5-18\|RF1/JH4 | ............ ........ | T......... | .......... ...... | ...H.... ..D...V. .... | ................ | E.PRT............. .......SCS.. | ...... ..... |
| iPS:43 4379 | 21-225_66A9 | VH3\|3-33/D5\|5-18\|RF1/JH4 | ............ ........ | .......... | .......... ...... | ...H.... ..D..... .... | ................ | E.PRT............. .......SCS.. | ...... ..... |
| | | Germline | H_FR1 | H_CDR1 | H_FR2 | H_CDR2 | H_FR3 | H_CDR3 | H_FR4 |
| | **VH3\|3-33/D1\|1-1\|RF3/JH4** | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | YNWND---------------YFDY | WGQGTLVTVSS |

| iPS | Clone | Germline | H_FR1 | H_CDR1 | H_FR2 | H_CDR2 | H_FR3 | H_CDR3 | H_FR4 |
|---|---|---|---|---|---|---|---|---|---|
| iPS:43 4247 | 21-225_62D2 | VH3\|3-33/D1\|1-1\|RF3/JH4 | ..Y......... ............ ........ | .......... | ........ ...... .... | ........ .....S.. | ...............D... | D.G.W............. .......N.L.. | ...... ..... |
| iPS:43 6838 | 21-225_52H4 | VH3\|3-33/D1\|1-1\|RF3/JH4 | ............ ............ ........ | H.....F... | ........ ..K... .... | ........ ........ | ................ | GD..Y............. .......EG... | ...... ..... |
| iPS:43 6948 | 21-225_183F5 | VH3\|3-33/D1\|1-1\|RF3/JH4 | ............ ............ ........ | .........L | ........ .....T .... | ........ ..G..... .... | ................ | E.FW— ................. ...SG.. | ...... ..... |
| | Germline | | QLQLQES-GPGLVKPSEILSLTCTVSG-GSIS | SS---SYYWG | WIRQPPGKGLEWIG | SIYY----SGSTYYNPSLKS | RVTISVDTSKNQFSLKLSSVTAADTAVYYCAR | GYSGYD---------YYFDY | WGQGTLVTVSS |
| iPS:43 4249 | 21-225_62E2 | VH4\|4-39/D5\|5-12\|RF3/JH4 | ............ ...........I ........ | R......... | ........ ...... .... | ........ ..IAS... | .................. N....T.......... | LS..W— ................. .-S... | ...... ..... |
| iPS:43 4353 | 21-225_64B12 | VH4\|4-39/D5\|5-12\|RF3/JH4 | ............ ............ ....V... | R......... | ........ ...... .... | ........ ....S... .... | .................. | LD..W— ................. .-S... | ...... ..... |
| iPS:39 4073 | 21-225_15C9 | VH4\|4-39/D5\|5-12\|RF3/JH4 | ............ ............ ........ | R......... | ......... ...... .... | N....... .....N.. | ................ | QG..W— ................. .-EV.. | ...... ..... |
| | Germline | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | LTGY-----------FDY | WGQGTLVTVSS |
| iPS:43 4259 | 21-225_62G7 | VH1\|1-02/D7\|7-27\|RF1/JH4 | ............ ............ ........ | .......... | ........ ...... .... | ..K.K... .....Q.. | .S.............. | AP.IAAAG.......... .....TWGY... | ...... ..... |
| iPS:43 4347 | 21-225_64H10 | VH1\|1-02/D7\|7-27\|RF1/JH4 | ............ ............ ........ | .......... | ........ ...... .... | ..K..... .....Q.. | .G.............. | AP.TAATG.......... .....TWGY... | ...... ..... |
| iPS:43 4359 | 21-225_65G3 | VH1\|1-02/D7\|7-27\|RF1/JH4 | ............ ............ ........ | .........I. | ........ ...... .... | ........ .....S.. | ................ | AP.KAAAG.......... .....TWGY... | ...... ..... |

| | | | | | | | | | |
|---|---|---|---|---|---|---|---|---|---|
| iPS:43 4369 | 21-225_66B1 | VH1\|1-02\|D7\|7-27\|RF1\|JH4 | . . . . . . . . . . . .<br>. . . . . . . . . . . .<br>. . . . . . . . | . . . . . . . . . . | . . . . . . . .<br>. . . . . . | . . . . . . . .<br>. . . . . N . .<br>. . . . | . . . . . . A . . . . . . . . . . | AP.TAAAG. . . . . . . . . .<br>. . . . . TWGY. . . | . . . . . |
| iPS:43 4373 | 21-225_66A7 | VH1\|1-02\|D7\|7-27\|RF1\|JH4 | . . . . . . . . . . . .<br>. . . . . . . . . . . .<br>. . . . . . . . | . . . . . . . . . . | . . . . . . . .<br>. . . . . . | . . K . . . . .<br>. . . . . Q . .<br>. . . . | . . . . . . . . . . . . . G . . . . | AP.TVAAG. . . . . . . . . .<br>. . . . . TWGY. . . | . . . . . |
| iPS:43 4397 | 21-225_67H4 | VH1\|1-02\|D7\|7-27\|RF1\|JH4 | . . . . . . . . . . . .<br>. . . . . . . . . . . .<br>. . . . . . . . | . . . . . . . . . . | . . . . . . . .<br>. . . . . . | . . K . . . . .<br>. . . . . Q . .<br>. . . . | . . . . . . . . . . . . . . . . . .<br>. G . . . . . . . . . . . . | AP.TAATG. . . . . . . . . .<br>. . . . . TWGY. . . | . . . . . |
| iPS:43 4427 | 21-225_70D6 | VH1\|1-02\|D7\|7-27\|RF1\|JH4 | . . . . . . . . . . . .<br>. . . . . . . . . . . .<br>. . . . . I . . | . . . . . . . . . . | . . . . . . . .<br>. . . . . . | . . . . K . . .<br>. . . . . S . .<br>. . . . | . . S . . . . . . . G . . . . .<br>RG . . . . . . . E . . . . . | AP.KAAAG. . . . . . . . . .<br>. . . . . TWGF. . . | . . . . . |
| iPS:43 4435 | 21-225_70G9 | VH1\|1-02\|D7\|7-27\|RF1\|JH4 | . . . . . . . . . . . .<br>. . . . . . . . . . . .<br>. . . . . . . . | . . . . . . . . . . | . . . . . . . .<br>. . . . . . | . . K . . . . .<br>. . . . . Q . .<br>. . . . | . S . . . . . . . . . . . . . . . . | AP.IAAAG. . . . . . . . . .<br>. . . . . TWGY. . . | . . . . . |
| iPS:43 4437 | 21-225_70A12 | VH1\|1-02\|D7\|7-27\|RF1\|JH4 | . . . . . . . . . . . .<br>. . . . . . . . . . . .<br>. . . . . . . . | . . . . . . . . . . | . . . . . . . .<br>. . . . . . | . . K . . . . .<br>. . . . . Q . .<br>. . . . | . . . . . . . . . . . . . . . . . .<br>. G . . . . . . . . . . . . | AP.TAATG. . . . . . . . . .<br>. . . . . TWGY. . . | . . . . . |
| iPS:43 4451 | 21-225_71B7 | VH1\|1-02\|D7\|7-27\|RF1\|JH4 | . . . . . . . . . . . .<br>. . . . . . . . . . . .<br>. . . . . I . . | . . . . . . . . . . | . . . . . . . .<br>. . . . . . | . . . . . . . .<br>. . . . . S . .<br>. . . . | . . . . . . . . . . G . . . . .<br>. G . . . . . . . . . . . . | AP.KAAAG. . . . . . . . . .<br>. . . . . TWGF. . . | . . . . . |
| iPS:43 4459 | 21-225_71A7 | VH1\|1-02\|D7\|7-27\|RF1\|JH4 | . . . . . . . . . . . .<br>. . . . . . . . . . . .<br>. . . . . . . . | . . . . . . . . . . | . . . . . . . .<br>. . . . . . | . . . . K . . .<br>. . . . . V . .<br>. . . . | . S . . . . . . . . . . . . . . . . | AP.TAPAG. . . . . . . . . .<br>. . . . . SWGY. . . | . . . . . |
| iPS:43 4461 | 21-225_73A3 | VH1\|1-02\|D7\|7-27\|RF1\|JH4 | . . . . . . . . . . . .<br>. . . . . . . . . . . .<br>. . . . . . . . | . . . . . . . . . . | . . . . . . . .<br>. . D . . . | . . . . K . . .<br>. . . . . EV .<br>. . . . | . . A . . . . . . . . . . . . .<br>. S . . . . . . . . . . . . | AP.TAAAG. . . . . . . . . .<br>. . . . . SWGC. . . | . . . . . |
| | Germline | | H_FR1 | H_CDR1 | H_FR2 | H_CDR2 | H_FR3 | H_CDR3 | H_FR4 |
| | VH3\|3-23\|D4\|4-11\|RF3\|JH4 | | EVQLLES-GGGLVQPGGSLR LSCAASG-FTFS | S-----YAMS | WVRQAPGK GLEWVS | AISGS---GGSTYYAD SVKG | RFTISRDNSKNTLYLQM NSLRAEDTAVYYCAK | TTVT------------YFDY | WGQGTL VTVSS |
| iPS:43 4261 | 21-225_56F7 | VH3\|3-23\|D4\|4-11\|RF3\|JH4 | . . . . . . . . . . . .<br>. . . . . . . . . . . .<br>. . . . . . . . | . . . . . . . VLN | . . . . . . . .<br>. . . . . . | . M . . . . . .<br>. . R . . . . .<br>. . . . | . . . . . . . . . . . . . . . . . .<br>. . . . . . . . . . . . F . . M | . . H~<br>. . . . . . . . . . . . . . . . . . .<br>. . . . ~ . . . | . . . . . . |

| iPS:43 5461 | 21-225_153A1 | VH3\|3-23/D4\|4-11\|RF3/JH4 | . . . . . . . . . . . . <br> . . . . . . . . . . . . <br> . . . . . S . . | . . . . . . . V . . | . . . . G . . . <br> . . . . . . | . D R . . . . . . <br> . D R . . . . <br> . . . . | . . . . . . . . . . . . . . . . <br> . . . . . . . . . . . . . . . R | . A T – <br> . . . . . . . . . . . . . . . . <br> . . . . – K . . | . . . . . . <br> . . . . |
| iPS:43 5509 | 21-225_157H1 | VH3\|3-23/D4\|4-11\|RF3/JH4 | . . . . . . . . . . . . <br> . . . . . . . . . . . . <br> . . . . . . . . | . . . . . . . . . R | . I . . . . . . <br> . . Q . . . | D . . . . . . . <br> . . T . . . . . <br> . . . . | . . . . . . . . . . . . . . . . | . Y – – <br> . . . . . . . . . . . . . . . . <br> . . . . – – – L | . . . . . . <br> . . . . |
| iPS:43 5747 | 21-225_175C4 | VH3\|3-23/D4\|4-11\|RF3/JH4 | . . . . . . . . . . . . <br> . . . . . . . . . . . . <br> . . A . . . . . | . . . . . . . V . . | . . . . . . . . <br> . . . . . . | . . . . . . . . <br> . D R . . . . <br> . . . . | . . . . . . . D . N T . . . . . . <br> . . . . . . . . . . . . . . . R | . A G – <br> . . . . . . . . . . . . . . . . <br> . . . . – . . . | . . . . . . <br> . . . . . |
| iPS:39 2784 | 21-225_23C7 | VH3\|3-23/D4\|4-11\|RF3/JH4 | . . . . . . . . . . . . <br> . . . . . . . . . . . . <br> . . . . . . . . | . . . . . . . V . . | . . . . . . . . <br> . . . . . . | . M . . . . . . <br> . . . . . . V . <br> . . . . | . . . . . . . . . . . . . . . . <br> . . . . . . . . . . . . . . . R | . G . – <br> . . . . . . . . . . . . . . . . <br> . . . . – . . . | . . . . . . <br> . I . . . |
| iPS:39 2802 | 21-225_23E7 | VH3\|3-23/D4\|4-11\|RF3/JH4 | . . . . . . . . . . . . <br> . . . . . . . . . . . T <br> . . . . . . . . | . . . . . . . . . N | . . . . . . . . <br> . . . . . . | . . . . . . . . <br> . . F . . . . <br> . . . . | . . . . . . . . . . R . . . . . . <br> . . . . . . . . . . . . . . . R | . S G – <br> . . . . . . . . . . . . . . . . <br> . . . . – . . . | . . . . . . <br> . . . . |
| iPS:39 2962 | 21-225_30A1 | VH3\|3-23/D4\|4-11\|RF3/JH4 | . . . . . . . . . . . . <br> . . . . . . . . . . . . <br> . . . . . . . . | . . . . . . . V . N | . . . . . . . . <br> . . . . . . | . . . . . . . . <br> . . . . . . . <br> . . . . | . . . . . . N . . . . . . . . . <br> . . . . . . . . . . . . . . . R | . G . – <br> . . . . . . . . . . . . . . . . <br> . . . . – . . . | . . . . . . <br> . . . . |
| iPS:39 3090 | 21-225_33A5 | VH3\|3-23/D4\|4-11\|RF3/JH4 | . . . . . . . . . . . . <br> . . . . . . . . . . . . <br> . . . . . . . . | . . . . . . . V I N | . . . . . . . . <br> . . . . . . | . . . . . . . . <br> . V . . . . . <br> . . . . | . . . . . . . . . . . . . . . . <br> . . . . . . . . . . . . . . . R | . S L – <br> . . . . . . . . . . . . . . . . <br> . . . . – . . . | . . . . . . <br> . . . . |

| Germline | H_FR1 | H_CDR1 | H_FR2 | H_CDR2 | H_FR3 | H_CDR3 | H_FR4 |
|---|---|---|---|---|---|---|---|
| **VH3\|3-23/D1\|1-1\|RF3/JH4** | EVQLLES–GGGLVQPGGSLR LSCAASG–FIFS | S–––––YAMS | WVRQAPGK GLEWVS | AISGS–––GGSTYYAD SVKG | RFTISRDNSKNTLYLQM NSLRAEDTAVYYCAK | YNWND–––––––––––––––––––––––––YFDY | WGQGTL VTVSS |
| iPS:43 4273 | 21-225_57E4 VH3\|3-23/D1\|1-1\|RF3/JH4 | . . . . . . . . . . . . <br> . . . . . . . . . . . . <br> . . . . . . . . | . . . . . . . . . . | . . . . . . . . <br> . . . . . . | V . . . . . . <br> . . . . F . . . <br> . . . . | . . . . . . . . . . T . . . . . . <br> . . . . . . . . . . . . . . . . | R D . . . . . . . . . . . . . . . . <br> . . . . . . . . V . . . | . . . . . . <br> . . . . . |

| Germline | H_FR1 | H_CDR1 | H_FR2 | H_CDR2 | H_FR3 | H_CDR3 | H_FR4 |
|---|---|---|---|---|---|---|---|
| **VH3\|3-23/D3\|3-3\|RF3/JH4** | EVQLLES–GGGLVQPGGSLR LSCAASG–FIFS | S–––––YAMS | WVRQAPGK GLEWVS | AISGS–––GGSTYYAD SVKG | RFTISRDNSKNTLYLQM NSLRAEDTAVYYCAK | ITIFGVV–––––––––––––––––IIYFDY | WGQGTL VTVSS |
| iPS:43 4279 | 21-225_57F7 VH3\|3-23/D3\|3-3\|RF3/JH4 | . . . . . . . . . . . . <br> . . . . . . . . . . . . <br> . . . . . . . . | . . . . . . . . . . | . . . . . . . . <br> . . . . . . | . . N . F . . . <br> . . . . | . . . . . . . . . . . . . . . . | FFGVVG–<br> . . . . . . . . . . . . . . . V <br> GC . . . | . . . . . . <br> . . . . . |

| | | | H_FR1 | H_CDR1 | H_FR2 | H_CDR2 | H_FR3 | H_CDR3 | H_FR4 |
|---|---|---|---|---|---|---|---|---|---|
| iPS:43 7228 | 21-225_60C11 | VH3\|3-23/D3\|3-3\|RF3/JH4 | ............ ............ .....P.. | .......... | ...... ...... | ....... ..N.F... .... | ................ | FFGVVC- ................V GC... | ...... ..... |
| | **Germline** | | **H_FR1** | **H_CDR1** | **H_FR2** | **H_CDR2** | **H_FR3** | **H_CDR3** | **H_FR4** |
| | **VH3\|3-23/D3\|3-22\|RF3/JH1** | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | ITMIVVVI---------- -----TAEYFQH | WGQGTL VTVSS |
| iPS:43 4291 | 21-225_58A4 | VH3\|3-23/D3\|3-22\|RF3/JH1 | ............ ............ ....L... | .......... | ....D... ...... .... | ....... ..N.F.G. .... | ..........K...... | FFG..G-- ................- .GF.DS | ...... ..... |
| | **Germline** | | **H_FR1** | **H_CDR1** | **H_FR2** | **H_CDR2** | **H_FR3** | **H_CDR3** | **H_FR4** |
| | **VH3\|3-33/D2\|2-15\|RF3/JH4** | | QVQLVES-GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | DIVVVVA---------- -----ATYFDY | WGQGTL VTVSS |
| iPS:43 4299 | 21-225_58D11 | VH3\|3-33/D2\|2-15\|RF3/JH4 | ............ ............ ........ | D......DI. | .....S... ...... .... | ....... ..K..... .... | .........L...... | .R.T--- ................- --... | ...... ..... |
| iPS:43 4871 | 21-225_85H1 | VH3\|3-33/D2\|2-15\|RF3/JH4 | ............ ............ ........ | ........I. | ......... ...... .... | ........ ....... .... | ................ | .PFI.G- ................. ..... | ...... |
| iPS:43 5109 | 21-225_92H5 | VH3\|3-33/D2\|2-15\|RF3/JH4 | ............ ............ ........ | .......... | ........ ...... .... | ........ ....... .... | ................ | .PFI.G- ................. ..... | ...... |
| iPS:43 6434 | 21-225_216B10 | VH3\|3-33/D2\|2-15\|RF3/JH4 | ............ ............ ........ | .......... | ........ ...... .... | A....... ....... .... | ..........R...... | .PNI.G- ................. .W... | ...... |
| | **Germline** | | **H_FR1** | **H_CDR1** | **H_FR2** | **H_CDR2** | **H_FR3** | **H_CDR3** | **H_FR4** |
| | **VH1\|1-02/D4\|4-17\|RF2/JH4** | | QVQLVQS-GAEVKKPGASVK VSCKASG-YIFT | G------YYMH | WVRQAPGQ GLEWMG | WINPN-- SGGTNYAQ KFQG | RVTMTRDTSISTAYMEL SRLRSDDTAVYYCAR | DYGDY------------ ---------YFDY | WGQGTL VTVSS |
| iPS:43 4307 | 21-225_59B2 | VH1\|1-02/D4\|4-17\|RF2/JH4 | ............ ............ .......A | ........I. | ....... ....L. .... | ........ ....... .... | ....F.......... | .P.P- ...-... | ...... ..... |
| | **Germline** | | **H_FR1** | **H_CDR1** | **H_FR2** | **H_CDR2** | **H_FR3** | **H_CDR3** | **H_FR4** |
| | **VH3\|3-33/D3\|3-22\|RF3/JH6** | | QVQLVES-GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | ITMIVVVII--------- ---YYYYYGMDV | WGQGTT VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 4311 | 21-225_59H5 | VH3|3-33/D3|3-22|RF3/JH6 | . . . . . . . . . . . . . . . . . . . . . . . . . . . | . . . . . . . . . . | . . . . . . . . . . . . . . . | . . . . . . . . . . . . | . . . . . . . . . . . . . . . . . . | ERG.A.G-- . . . . . . . . . . . . . --- | . . . . . . . . . . . |
| | Germline | **H_FR1** | **H_CDR1** | **H_FR2** | **H_CDR2** | **H_FR3** | **H_CDR3** | **H_FR4** | |
| | **VH4|4-39/D1|1-26|RF3/JH4** | QLQLQES-GPGLVKPSETLS LTCTVSG-GSIS | SS---SYYWG | WIRQPPGK GLEWIG | SIYY---- SGSTYYNP SLKS | RVTISVDTSKNQFSLKL SSVTAADTAVYYCAR | YSGSY -------YYFDY | WGQGTL VTVSS | |
| iPS:43 4313 | 21-225_59E6 | VH4|4-39/D1|1-26|RF3/JH4 | . . . . . . . . . . . . . . . . . . . . . | R. . . . . . . . . | . . . . . . . . . . . . . . | N. . . . . . . . . . . . . . | . . . . . . . . . . . . . . . . . .L. . . . . | H.S.W. . . . . . . . . . . . . . . . . . .-SL. . | . . . . . |
| iPS:43 4413 | 21-225_68D12 | VH4|4-39/D1|1-26|RF3/JH4 | . . . . . . . . . . . . . . . . . . . . . | R. . . . . . . . . | . . . . . . . . . . . . . . | N. . . . . . . . . . . .I. . . . | T. . . . . . . . . . . . . . . . . | H.T.W. . . . . . . . . . . . . . . . . . .-SI. . | . . . . . |
| iPS:39 2628 | 21-225_20C2 | VH4|4-39/D1|1-26|RF3/JH4 | . . . . . . . . . . . . . . . . . . . . . | R. . . . . . . . . | . . . . . . . . . . . . . . | N. . . . . . . ..TA.C.S | . .I. . . . . . . . . . . . . . . . .T. . . . . . . . . | H.S.W. . . . . . . . . . . . . . . . . . .-SL.N | . . . . . |
| iPS:39 2642 | 21-225_18C6 | VH4|4-39/D1|1-26|RF3/JH4 | . . . . . . . . . . . . . . . . . . . . . | R. . . . . . . . . | . . . . . . . . . . . . . . | N. . . . . . . ..Y. . . . . | . .I. . . . . . . . . . . . . . . . . . . . . . . .L. . . . . | H.S.W. . . . . . . . . . . . . . . . . . .-SL.D | . . . . . |
| iPS:39 2706 | 21-225_18A3 | VH4|4-39/D1|1-26|RF3/JH4 | . .F. . . . . . . . . . . . . . . . . . | R. . . . . . . . . | . . . . . . . . . . . . . . | N. . . . . . . ..Y...T. | . . . . . . . . . . . . . . . . . . | H.T.W. . . . . . . . . . . . . . . . . . .-SL. . | . . . . . |
| iPS:39 2800 | 21-225_22D12 | VH4|4-39/D1|1-26|RF3/JH4 | . . . . . . . . . . . . . . . . . . . . . | R. . . . . . . . . | . . . . . . . . . . . . . . | N. . . . . . . ..T.S. . . | . . . . . . . . . .R. . . . . . . . . . . . . . . . .F. . . . | L.S.W. . . . . . . . . . . . . . . . . . .-SV. . | . . . . . |
| iPS:39 2820 | 21-225_23D1 | VH4|4-39/D1|1-26|RF3/JH4 | . . . . . . . . . . . . . . . . . . . . . | R. . . . . . . . . | . . . . . . . . . . . . . . | N. . . . . . ...AQ. . . | . . . . . . . . . . . . . . . .T. . . . . . . . .L. . . . . | L.S.W. . . . . . . . . . . . . . . . . . .-S. . . | . . . . . |
| iPS:39 2824 | 21-225_24E5 | VH4|4-39/D1|1-26|RF3/JH4 | . . . . . . . . . . . . . . . . .A. . | R. . . . . . . . . | . . . . . . . . . . . . . . | . . . . . . . ...AN. . . | . . . . . . . . . . . . . . . . . . | L.S.W. . . . . . . . . . . . . . . . . . .-SI.N | . . . . . |
| iPS:39 2834 | 21-225_22C1 | VH4|4-39/D1|1-26|RF3/JH4 | . . . . . . . . . . . . . . . . . . .N | R. . . . . . . . . | . . . . . . . . . . . . . . | N. . . . . . ..A. . . .S | . . . . . . . . .T. . . . . . . . . . . . . . . . . | H. . .W. . . . . . . . . . . . . . . . . .-SL. . | . . . . . |

| | | | | | | | | | |
|---|---|---|---|---|---|---|---|---|---|
| IPS:39 2870 | 21-225_20G9 | VH4\|4-39/D1\|1-26\|RF3/J H4 | . . . . . . . . . . . . . . . . . . . . . . . . . . . . . . . | R . . . . . . . . . | . . . . . . . . . . . . . | . . . . . . . . . . . AS . . . . . . . . | . . . . . . . . . R . . . . . . . . . . . . . . . A . . . . . | L . S . W . . . . . . . . . . . . . . . . . . . . . . . -S . . . | . . . . . . |
| IPS:39 2896 | 21-225_21G7 | VH4\|4-39/D1\|1-26\|RF3/J H4 | . . . . . . . . . . . . . . F . . . . . . . . . . . . . . . . | R . . . . . . . . . | . . . . . . . . . Q . . . . . | N . . . . . . . . . YS . . . . . . . . | . . . . . . . . . . . . . . . . . | H . T . W . . . . . . . . . . . . . . . . . . . . -SL . . | . . . . . |
| IPS:39 2904 | 21-225_22G9 | VH4\|4-39/D1\|1-26\|RF3/J H4 | . . . . . . . . . . . . . . . . . . . . . . . . . . A . . | G . . . . N . . . . | . . . . . . . . . E . . . . . | N . . . . . . . . . . . . . . . . . | . . . . . . . . . . . . . . R . . . . E . . . . . . . . . | H . S . W . . . . . . . . . . . . . . . . . . . -SL . . | . . . . . |
| IPS:39 3094 | 21-225_34C4 | VH4\|4-39/D1\|1-26\|RF3/J H4 | . . . . . . . . . . . . . . . . . . . . . . . . . . . . . | R . . . . . . . . . | . . . . S . . . . . . . . . | . . . . . . . . . . . A . . . . . . . | . . . . . . . . . . . . . . . . . . . . V . . . . | L . S . W . . . . . . . . . . . . . . . . . . . . . . -S . . . | . . . . . |
| IPS:39 3808 | 21-225_8A6 | VH4\|4-39/D1\|1-26\|RF3/J H4 | . . . . . . . . . . . . . . . . . . . . . . . . . . . . . | R . . . . . . . . . | . . . . . . . . . . . . . . | N . . . . . . . . . IP . . . . . . . . | . . N . . . . . . . . . . . . N . . . . . . . . . . . . . . | H . S . W . . . . . . . . . . . . . . . . . . . -SL . . | . . . . . |
| IPS:39 3814 | 21-225_7F4 | VH4\|4-39/D1\|1-26\|RF3/J H4 | . . . . . . . . . . . . . . . . . . . . . . . . . . . . . | R . . . . . . . . . | . . . . . . . . . . . . . . | N . . . . . . . . A . . . . . . . | . . . . . . . . . T . . . . . . . . . . . . . . . . . . | H . . . W . . . . . . . . . . . . . . . . . . . -SL . . | . . . . . |
| IPS:39 3816 | 21-225_6D4 | VH4\|4-39/D1\|1-26\|RF3/J H4 | . . . . . . . . . . . . . . . . . . . . . . . . . . . . . | R . . . . . S . . . | . . . . . . . . . . . . . . | N . . . . . . . . . A . . I . . . . | . . . . . A . . . . . . . . N . T . . . . . . . . . . . . . | H . S . W . . . . . . . . . . . . . . . . . . -SL . C | . . . . . |
| IPS:39 3880 | 21-225_15A1 | VH4\|4-39/D1\|1-26\|RF3/J H4 | . . H . . . . . . . . . . . . . . . . . . . . . . . . . . | R . . . . . . . . . | . . . . . . . . . . . . . . | . . . AQ . . . . . . . . | . . . . . . . . . . . T . . . . . . T . | L . S . W . . . . . . . . . . . . . . . . . . . . . . -S . . . | . . . . . |
| IPS:39 4002 | 21-225_15G7 | VH4\|4-39/D1\|1-26\|RF3/J H4 | . . . . . . . . . . . . . . . . . . . . . . . . . . . . . | R . . . . S . . . | . . . . . . . . . . . . . . | . . . . . . . Y . . . T . . . . . | . . . . . . . . . . . . . . . R . . . . . . . . . S . . . . | L . S . W . . . . . . . . . . . . . . . . . . . -S . . F | . . . . . |
| IPS:39 4053 | 21-225_11F10 | VH4\|4-39/D1\|1-26\|RF3/J H4 | . . H . . . . . . . . . . . . . . . . . . . . A . . . | R . . . . . . . . . | . . . . . . . . . . . . . . | . . . AQ . . . . . . . . | . . . . . . . . . . . . . . . T . | L . S . W . . . . . . . . . . . . . . . . . . . -S . . . | . . . . . |
| IPS:39 4057 | 21-225_15H1 | VH4\|4-39/D1\|1-26\|RF3/J H4 | . . . . . . . . . . . . F . . . . . . . . . . . . . . . | R . . . . . . . . . | . . . . . . . . . . . . . . | N . . . . . . . . YP . . . . . . . . | . . . . . . . . . . . . . . . . . | H . T . W . . . . . . . . . . . . . . . . . . . -SL . . | . . . . . |
| IPS:39 4063 | 21-225_16A1 | VH4\|4-39/D1\|1-26\|RF3/J H4 | . . . . . . . . . . . . . . . . . . . . . I . . . . . . . . D | R . . . . . . . . . | . . . . . . . . . . . . . . | N . . . . . . . . . A . H . . . . | . G . . . . . . . . . . . . . . . . . . . . . A . . . . . | L . S . W . . . . . . . . . . . . . . . . . . . -S . . . | . . . . . |

| iPS | Name | Germline | H_FR1 | H_CDR1 | H_FR2 | H_CDR2 | H_FR3 | H_CDR3 | H_FR4 |
|---|---|---|---|---|---|---|---|---|---|
| iPS:39 4075 | 21-225_8D12 | VH4|4-39/D1|1-26|RF3/JH4 | ............ ..F......... ........ | R......... | ........ ...... .... | N....... ..YP.... .... | .....I.......... ............. | H.T.W............ ........-SL.. | ...... ..... |
| | **Germline** | | **H_FR1** | **H_CDR1** | **H_FR2** | **H_CDR2** | **H_FR3** | **H_CDR3** | **H_FR4** |
| | VH3|3-48/D7|7-27|RF3/JH4 | | EVQLVES-GGGLVQPGGSLRLSCAASG-FIFS | S-----YSMN | WVRQAPGKGLEWVS | YISSS---SSTIYYADSVKG | RFTISRDNAKNSLYLQMNSLRDEDTAVYYCAR | NWGY-------------------FDY | WGQGTLVTVSS |
| iPS:43 4317 | 21-225_59E8 | VH3|3-48/D7|7-27|RF3/JH4 | ............ ............ ........ | .......... | ..G... | .G...... .... | ................ | E..MAV............ ......AGP... | ...... ..... |
| | **Germline** | | **H_FR1** | **H_CDR1** | **H_FR2** | **H_CDR2** | **H_FR3** | **H_CDR3** | **H_FR4** |
| | VH3|3-33/D1|1-26|RF3/JH4 | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | YSGSY-------------YYFDY | WGQGTLVTVSS |
| iPS:43 4327 | 21-225_63G6 | VH3|3-33/D1|1-26|RF3/JH4 | ............ ............ ........ | .......... | ......T | ........ .... | ................ | D.L.GI............ ......AAA... | ..... |
| iPS:43 7084 | 21-225_206B5 | VH3|3-33/D1|1-26|RF3/JH4 | ............ ............ ........ | .......... | ...... | ........ .... | .............N... | EG................ ........-HL.. | ....I. |
| iPS:43 7088 | 21-225_209H10 | VH3|3-33/D1|1-26|RF3/JH4 | ............ ............ ........ | .......... | ...... | ........ .... | .............N... | EG................ ........-HL.. | ....I. |
| iPS:39 2684 | 21-225_17F4 | VH3|3-33/D1|1-26|RF3/JH4 | ............ ............ ........ | N........N | ...... | .N..H... .... | ...............S | SGSY- ................... ..-F... | ...... |
| | **Germline** | | **H_FR1** | **H_CDR1** | **H_FR2** | **H_CDR2** | **H_FR3** | **H_CDR3** | **H_FR4** |
| | VH1|1-02/D6|6-19|RF2/JH4 | | QVQLVQS-GAEVKKPGASVKVSCKASG-YIFT | G-----YYMH | WVRQAPGQGLEWMG | WINPN-SGGTNYAQKFQG | RVTMTRDTSISTAYMELSRLRSDDTAVYYCAR | GIAVA-------GYFDY | WGQGTLVTVSS |
| iPS:43 4333 | 21-225_63C9 | VH1|1-02/D6|6-19|RF2/JH4 | ............ ............ ........ | .......... | ...... | .....F.. .... | .......A..N...... RS.I........... | APG..AAG.......... .....SW..... | ...... ..... |
| | **Germline** | | **H_FR1** | **H_CDR1** | **H_FR2** | **H_CDR2** | **H_FR3** | **H_CDR3** | **H_FR4** |
| | VH3|3-33/D4|4-11|RF2/JH4 | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | DYSNY--------------YFDY | WGQGTLVTVSS |

| | | | H_FR1 | H_CDR1 | H_FR2 | H_CDR2 | H_FR3 | H_CDR3 | H_FR4 |
|---|---|---|---|---|---|---|---|---|---|
| IPS:43 4335 | 21-225_63C10 | VH3|3-33/D4|4-11|RF2/JH4 | ............<br>............<br>........ | .......... | ...D....<br>..D... | ........<br>........<br>.... | ...............<br>............... | .DPRS............<br>.......SAG.. | ......<br>..... |
| IPS:43 4429 | 21-225_70H6 | VH3|3-33/D4|4-11|RF2/JH4 | ............<br>............<br>........ | .......... | ....T....<br>..D... | ...H....<br>........<br>.... | ...............<br>............... | .DPRS............<br>.......SAG.. | ......<br>..... |
| IPS:43 4569 | 21-225_77H5 | VH3|3-33/D4|4-11|RF2/JH4 | ............<br>............<br>........ | N......... | ........<br>...... | ........<br>.R......<br>.... | ...............<br>........L..... | .R.ILG............<br>......ATF... | ......<br>..... |
| IPS:43 4629 | 21-225_74C3 | VH3|3-33/D4|4-11|RF2/JH4 | ............<br>............<br>........ | ......F... | .A......<br>...... | A.......<br>......C..<br>.... | ..............S...<br>......S....... | .R.ILG............<br>......AAF... | ......<br>..... |
| IPS:43 5793 | 21-225_180F8 | VH3|3-33/D4|4-11|RF2/JH4 | ............<br>............<br>........ | .......... | ........<br>......S | ........<br>..D...E.<br>.... | ...............<br>............... | .HPRW............<br>.......S.G.. | ......<br>..... |
| IPS:43 6382 | 21-225_212C10 | VH3|3-33/D4|4-11|RF2/JH4 | ............<br>............<br>........ | .......... | ........<br>......T | A.......<br>..H...T.<br>.... | ...............<br>............... | .R.IVG............<br>......AT.... | ......<br>..... |
| IPS:39 2660 | 21-225_19B3 | VH3|3-33/D4|4-11|RF2/JH4 | ............<br>............<br>........ | .......D.. | ........<br>...... | ........<br>..D.....<br>.... | ..............F...<br>............... | .RAYS............<br>.......SSS.. | ......<br>..... |
| IPS:39 3904 | 21-225_8H11 | VH3|3-33/D4|4-11|RF2/JH4 | ............<br>............<br>........ | T......N.. | ........<br>...... | ........<br>..DR.S..<br>.... | ...............<br>............... | .RAYS............<br>.......SSS.F | ......<br>..... |
| | Germline | | H_FR1 | H_CDR1 | H_FR2 | H_CDR2 | H_FR3 | H_CDR3 | H_FR4 |
| | VH4|4-59/D4|4-11|RF2/JH4 | | QVQLQES-<br>GPGLVKPSETLS<br>LTCTVSG-GSIS | S-----YYWS | WIRQPPGK<br>GLEWIG | YIYY----<br>SGSTNYNP<br>SLKS | RVTISVDTSKNQFSLKL<br>SSVTAADTAVYYCAR | DYSNY------------<br>--------YFDY | WGQGTL<br>VTVSS |
| IPS:43 4341 | 21-225_64F7 | VH4|4-59/D4|4-11|RF2/JH4 | ............<br>............<br>....... | .......F.. | ......A..<br>...... | R..T....<br>..IS....<br>.... | ...M.............<br>............... | FS.G-<br>..................<br>...F... | ......<br>..... |
| | Germline | | H_FR1 | H_CDR1 | H_FR2 | H_CDR2 | H_FR3 | H_CDR3 | H_FR4 |
| | VH3|3-33/D7|7-27|RF2/JH1 | | QVQLVES-<br>GGGVVQPGRSLR<br>LSCAASG-FIFS | S-----YGMH | WVRQAPGK<br>GLEWVA | VIWYD----<br>GSNKYYAD<br>SVKG | RFTISRDNSKNTLYLQM<br>NSLRAEDTAVYYCAR | *LGAE-----------<br>--------YFQH | WGQGTL<br>VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 4351 | 21-225_64A12 | VH3\|3-33/D7\|7-27\|RF2/JH1 | ............ ............ ........ | .......... | ........ ...... | ........ E.....V. .... | ................. ............... | E..F- ................. ...LSD. | ...... ..... |
| iPS:39 2700 | 21-225_16E12 | VH3\|3-33/D7\|7-27\|RF2/JH1 | ...........L ............ ........ | N......... | ........ ...... | ....E... ......V. ..R. | ..........S.....V. ............ | E..F- ................. ...QSD. | .....P ..... |
| iPS:39 2710 | 21-225_19A10 | VH3\|3-33/D7\|7-27\|RF2/JH1 | ............ ............ ........ | .......... | ........ ...... | ........ E....... .... | ................. ............... | E.AW- ................. ....EDS | ...... ..... |
| iPS:39 4093 | 21-225_9D12 | VH3\|3-33/D7\|7-27\|RF2/JH1 | ............ ............ ........ | D......... | ........ ...... | ........ .N.N.... .... | ................. ............... | E.AW- ................. ....EDF | ...... ..... |
| | **Germline** | **H_FR1** | **H_CDR1** | **H_FR2** | **H_CDR2** | **H_FR3** | **H_CDR3** | **H_FR4** | |
| | **VH3\|3-23/D3\|3-22\|RF2/JH3** | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | YYYDSSGY-----------YYDAFDI | WGQGTM VTVSS | | |
| iPS:43 4355 | 21-225_64G12 | VH3\|3-23/D3\|3-22\|RF2/JH3 | ............ ............ ........ | .......N... | ........ ...... | V....... ........ .... | ................. ..........L..... | RN..D--- ................---- .... | ...... |
| | **Germline** | **H_FR1** | **H_CDR1** | **H_FR2** | **H_CDR2** | **H_FR3** | **H_CDR3** | **H_FR4** | | |
| | **VH3\|3-21/D4\|4-11\|RF3/JH4** | EVQLVES-GGGLVKPGGSLR LSCAASG-FIFS | S------YSMN | WVRQAPGK GLEWVS | SISSS--- SSYIYYAD SVKG | RFTISRDNAKNSLYLQM NSLRAEDTAVYYCAR | TTVT-----------------YFDY | WGQGTL VTVSS | | |
| iPS:43 4367 | 21-225_65H11 | VH3\|3-21/D4\|4-11\|RF3/JH4 | ............ ............ ........ | .......R.. | ........ ...... | V....... N.S..... .... | ...T............. ..............TS | .S-- ................. ....--GS | ...... |
| iPS:43 7220 | 21-225_55H6 | VH3\|3-21/D4\|4-11\|RF3/JH4 | ..H........ ............ ........ | .......... | ........ ...... | ...G.... .T...... .... | ................. ............... | .G.- ................. ....-... | ...... |
| iPS:40 2237 | 21-225_23D11 | VH3\|3-21/D4\|4-11\|RF3/JH4 | ............ ............ ........ | .......NI. | ........ ...... | ...GN... .G..... .... | ................. ............... | .NL- ................. ....-... | ...... |
| | **Germline** | **H_FR1** | **H_CDR1** | **H_FR2** | **H_CDR2** | **H_FR3** | **H_CDR3** | **H_FR4** | | |
| | **VH3\|3-21/D4\|4-11\|RF3/JH3** | EVQLVES-GGGLVKPGGSLR LSCAASG-FIFS | S------YSMN | WVRQAPGK GLEWVS | SISSS--- SSYIYYAD SVKG | RFTISRDNAKNSLYLQM NSLRAEDTAVYYCAR | TTVTD---------------AFDI | WGQGTM VTVSS | | |

EP 4 435 105 A2

| | | Germline | H_FR1 | H_CDR1 | H_FR2 | H_CDR2 | H_FR3 | H_CDR3 | H_FR4 |
|---|---|---|---|---|---|---|---|---|---|
| IPS:43 4383 | 21-225_66F9 | VH3\|3-21/D4\|4-11\|RF3/JH3 | . . . . . . . . . . .<br>. . . . . . . . . .<br>. . . . . . . . | . . . . . . . . . . | . . . . . . . .<br>. . . . . .<br>. . . . | . . . GT . . .<br>. . . . . . . .<br>. . . . | . . . . . . . . . . . . . . . .<br>. G . . . . . . . . . F . . . | . NA – –<br>. . . . . . . . . . . . . . . . . . .<br>. . . – . . . | . . . . . .<br>. . . . . |
| IPS:43 4449 | 21-225_71H6 | VH3\|3-21/D4\|4-11\|RF3/JH3 | . . . . . . . . . . .<br>. . . . . . . . . .<br>. . . . . . . . | . . . . . . . . . . | . . . . . . . .<br>. . . . . .<br>. . . . | . . . GT . . .<br>. . . . . . . .<br>. . . . | . . . . . . . . . . . . . . . .<br>. G . . . . . . . . . F . . K | . NA – –<br>. . . . . . . . . . . . . . . . . . .<br>. . . – . . . | . . . . . .<br>. . . . . |
| | **VH3\|3-33/D6\|6-6\|RF2/JH4** | Germline | QVQLVES–<br>GGGVVQPGRSLR<br>LSCAASG–FIFS | S – – – – – YGMH | WVRQAPGK<br>GLEWVA | VIWYD – – –<br>GSNKYYAD<br>SVKG | RFTISRDNSKNTLYLQM<br>NSLRAEDTAVYYCAR | SIAAR – – – – – – – – – – – – –<br>– – – – – – YFDY | WGQGTL<br>VTVSS |
| IPS:43 4399 | 21-225_67B7 | VH3\|3-33/D6\|6-6\|RF2/JH4 | . . . . . . . . . . .<br>. . . . . . . . . .<br>. . . . . . . . | N . . . . . . . . . | . . . . . . . .<br>. . . . . .<br>. . . . | . . L . . . . .<br>. . K . . . .<br>. . . . | . . . . . . . . . . . . . . . . | . . PE –<br>. . . . . . . . . . . . . . . . . . .<br>. . . – . . . | . . . . . . |
| IPS:43 4463 | 21-225_73A6 | VH3\|3-33/D6\|6-6\|RF2/JH4 | . . . . . . . . . . .<br>. . . . . . . . . .<br>. . . . . . . . | Y . . . . . . . . . | . . . . . . . .<br>. . . . . .<br>. . . . | . . L . . . . .<br>. . K . . . A<br>. . . . | . . . . . . . . . . . . . . . . | . . PD –<br>. . . . . . . . . . . . . . . . . . .<br>. . . – . . . | . . . . . . |
| | **VH3\|3-21/D1\|1-26\|RF3/JH4** | Germline | EVQLVES–<br>GGGLVKPGGSLR<br>LSCAASG–FIFS | S – – – – – YSMN | WVRQAPGK<br>GLEWVS | SISSS – – –<br>SSYIYYAD<br>SVKG | RFTISRDNAKNSLYLQM<br>NSLRAEDTAVYYCAR | YSGSY – – – – – – – – – – – – –<br>– – – – – – YYFDY | WGQGTL<br>VTVSS |
| IPS:43 4405 | 21-225_68E6 | VH3\|3-21/D1\|1-26\|RF3/JH4 | . . . . . . . . . . .<br>. . . . . . . . . .<br>. . . . . . L . | . . . . . . FG . . | . . . . . . . .<br>. . . . . .<br>. . . . | Y . . R . . . .<br>. . H . . . . .<br>. . . . | . . . . . . . . . . . . . . . . V | S . . . –<br>. . . . . . . . . . . . . . . . . . .<br>. . – P . . . | . . . . . . |
| IPS:43 5595 | 21-225_160H4 | VH3\|3-21/D1\|1-26\|RF3/JH4 | . . . . . . . . . . .<br>. . S . . . . . . .<br>. . . . . . . . | . . . . . . . R . . | . . . . . . . .<br>. . . . . .<br>. . . . | . . . G . . . .<br>. . . . D . . .<br>. . . . | . . . . . . . . . . . . . . . . | K . W – –<br>. . . . . . . . . . . . . . . . . . .<br>. . – – . . . | . . . . . . |
| IPS:43 5635 | 21-225_163F1 | VH3\|3-21/D1\|1-26\|RF3/JH4 | . . . . . D . . . . .<br>. . . . . . . . . .<br>. . . . . . . . | . . . . . . . . . | . . . . . . . .<br>. . . . . .<br>. . . . | . . . G . . . .<br>G . . . . . .<br>. . . . | . . . T . . . . . . . . . . . .<br>. . . S . D . . . . . . . TL | . S – –<br>. . . . . . . . . . . . . . . . . . .<br>. . – – SH . | . . . . . . |
| | **VH3\|3-30.3/D2\|2-15\|RF3/JH4** | Germline | QVQLVES–<br>GGGVVQPGRSLR<br>LSCAASG–FIFS | S – – – – – YAMH | WVRQAPGK<br>GLEWVA | VISYD – – –<br>GSNKYYAD<br>SVKG | RFTISRDNSKNTLYLQM<br>NSLRAEDTAVYYCAR | DIVVVVA – – – – – – – – – – – –<br>– – – – – ATYFDY | WGQGTL<br>VTVSS |
| IPS:43 4417 | 21-225_69C8 | VH3\|3-30.3/D2\|2-15\|RF3/JH4 | . . . . . . . . . . .<br>. . . . . . . . . .<br>. . . . . . . . | N . . . . . . . . . | . . . . . . . .<br>. . . . . .<br>. . . . | . . W . . . . .<br>. . D . . . . .<br>. . . . | I . . . . . . . . . . . . . . . | . . PSN – –<br>. . . . . . . . . . . . . . . . . . . –<br>SAG . . | . . . . . .<br>. . . . . |
| | | Germline | H_FR1 | H_CDR1 | H_FR2 | H_CDR2 | H_FR3 | H_CDR3 | H_FR4 |

| | Germline | H_FR1 | H_CDR1 | H_FR2 | H_CDR2 | H_FR3 | H_CDR3 | H_FR4 |
|---|---|---|---|---|---|---|---|---|
| | VH3\|3-33/D3\|3-10\|RF1/JH4 | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | VLLWFGE----------- -----LL*YFDY | WGQGTL VTVSS |
| iPS:43 4433 | 21-225_70E8 VH3\|3-33/D3\|3-10\|RF1/J H4 | ...........\n...........\n........ | .........L | ........\n......\n.... | I.......\nE.......\n.... | ................. | D..D---\n...................--\n-PR.. | ......\n..... |
| | Germline | H_FR1 | H_CDR1 | H_FR2 | H_CDR2 | H_FR3 | H_CDR3 | H_FR4 |
| | VH3\|3-23/D6\|6-13\|RF1/JH4 | EVQLLES-GGGLVQPGGSLR LSCAASG-FTFS | S-----YAMS | WVRQAPGK GLEWVS | AISGS---- GGSTYYAD SVKG | RFTISRDNSKNTLYLQM NSLRAEDTAVYYCAK | GYSSSW----------- --------YYFDY | WGQGTL VTVSS |
| iPS:43 4467 | 21-225_73H8 VH3\|3-23/D6\|6-13\|RF1/J H4 | ..........W\n..S.........\n........ | ......N... | ........\n......\n.... | D..R....\n..T.F...\n.... | ................. | WD....Y...........\n.....DVTP... | ......\n..... |
| | Germline | H_FR1 | H_CDR1 | H_FR2 | H_CDR2 | H_FR3 | H_CDR3 | H_FR4 |
| | VH4\|4-34/D4\|4-17\|RF2/JH4 | QVQLQQW-GAGLLKPSEILS LTCAVYG-GSFS | G-----YYWS | WIRQPPGK GLEWIG | EINH---- SGSTNYNP SLKS | RVTISVDTSKNQFSLKL SSVTAADTAVYYCAR | DYGDY------------- --------YFDY | WGQGTL VTVSS |
| iPS:43 4471 | 21-225_75G3 VH4\|4-34/D4\|4-17\|RF2/J H4 | ...........\n...........\n......L. | ......S... | ........\n......\n.... | ...L....\n........\n.... | ...........S....T.\nR................ | ...G-\n..................\n...-L.. | ......\n..... |
| iPS:43 4517 | 21-225_76A7 VH4\|4-34/D4\|4-17\|RF2/J H4 | ...........\n...........\n........ | ......C... | ........\n......\n.... | ........\n..R.....\n.... | .............E...... | ...G-\n..................\n...-L.. | ....A. |
| iPS:43 4519 | 21-225_74C7 VH4\|4-34/D4\|4-17\|RF2/J H4 | ...........\n...........\n......L. | ......S... | ........\n......\n.... | ...L....\n........\n.... | ...........S....T.\nR................ | ...G-\n..................\n...-L.. | ......\n..... |
| iPS:43 4571 | 21-225_74D2 VH4\|4-34/D4\|4-17\|RF2/J H4 | ...........\n...........\n........ | ......C... | ........\n......\n.... | ........\n..R.....\n.... | .........E...... | ...G-\n..................\n...-L.. | ......\n..... |
| iPS:43 4637 | 21-225_78E7 VH4\|4-34/D4\|4-17\|RF2/J H4 | ...........\n...........\n......L. | ......S... | ........\n......\n.... | ...L....\n........\n.... | ...........S....T.\nR................ | ...G-\n..................\n...-L.. | ......\n..... |
| iPS:43 4717 | 21-225_80A6 VH4\|4-34/D4\|4-17\|RF2/J H4 | ...........\n...........\n........ | ......C... | ........\n......\n.... | ........\n..R.....\n.... | .........EK...... | ...G-\n..................\n...-L.. | ......\n..... |
| iPS:43 4735 | 21-225_80B10 VH4\|4-34/D4\|4-17\|RF2/J H4 | ...........\n...........\n........ | ......C... | ........\n......\n.... | ........\n......\n.... | ................. | ...G-\n..................\n...-L.. | ......\n..... |

| | | | | | | | | | |
|---|---|---|---|---|---|---|---|---|---|
| iPS:43 4835 | 21-225_83B6 | VH4\|4-34/D4\|4-17\|RF2/JH4 | . . . . . . . . . . .<br>. . . . . . . . . . .<br>. . . . . . . . | . . . . . . C . . . . | . . . . . . . . .<br>. . . . . .<br>. . . . | . . . . . . . .<br>. . R . . . . .<br>. . . . | . . . . . . . . . E . . . . . .<br>. . . . . . . . . . . . . . . . . . . | . . . G –<br>. . . . . . . . . . . . . . . . . . . .<br>. . . . – L . . | . . . . . .<br>. . . . |
| iPS:43 4849 | 21-225_83C10 | VH4\|4-34/D4\|4-17\|RF2/JH4 | . . . . . . . . . . .<br>. . . . . . . . . . T<br>. . . . . . . . | . . . . . . . . . . | . . . . . . . . .<br>. . . . . .<br>. . . . | . . . . . . . .<br>. . . . . F . .<br>. . . . | . . . . . . . . . . . . . . . .<br>. . . . . . . . . F . . . . | . . . G –<br>. . . . . . . . . . . . . . . . . . . .<br>. . . . – L . . | . . . . . .<br>. . . . |
| iPS:43 4891 | 21-225_85G6 | VH4\|4-34/D4\|4-17\|RF2/JH4 | . . . . . . . . . . .<br>. . . . . . . . . . .<br>. . . . . . . . | D . . . . . C . . . . | . . . . . . . . .<br>. . . . . .<br>. . . . | . . . . . . . .<br>. . R . . . . .<br>. . . . | . . . . . . . . . E . . . . . .<br>. . . . . . . . . . . . . . | . . . G –<br>. . . . . . . . . . . . . . . . . . . .<br>. . . . – L . . | . . . . . .<br>. . . . |
| iPS:43 5183 | 21-225_93E9 | VH4\|4-34/D4\|4-17\|RF2/JH4 | . . . . . . . . . . .<br>. . . . . . . . . . .<br>. . . . . . . . | . . . . . . C . . . . | . . . . . . . . .<br>. . . . . .<br>. . . . | . . . . . . . .<br>. . R . . . . .<br>. . . . | . . . . . . . . . E . K . . . . .<br>. . . . . . . . . . . . . . | . . . G –<br>. . . . . . . . . . . . . . . . . . . .<br>. . . . – L . . | . . . . . .<br>. . . . |
| iPS:43 5331 | 21-225_147G8 | VH4\|4-34/D4\|4-17\|RF2/JH4 | . . . . . . . . . . .<br>. . . . . . . . . . G<br>. . . . . . . . | A . . . . . . . . . | . . . . . . . . .<br>. . . . . .<br>. . . . | . . . . . . . .<br>. . . . . K .<br>. . . . | . . . . . . . . . . . . . . . .<br>. . . . . . . . . . . . . . | . . . V –<br>. . . . . . . . . . . . . . . . . . . .<br>. . . – . . . | . . . . . .<br>. . . . |
| iPS:43 5995 | 21-225_192F8 | VH4\|4-34/D4\|4-17\|RF2/JH4 | . . . . . . . . . . .<br>. . . . . . . . . . .<br>. . . . . . . . | . . . . . . C . . . | . . . . . . . . .<br>. . . . . .<br>. . . . | . . . Q . . . .<br>. . R S . . . .<br>. . . . | . . . . . . . . . . T . . . . . . . .<br>R . . . . . . . . . . . . . | . . . V –<br>. . . . . . . . . . . . . . . . . . . .<br>. . . – . . . | . . . . . .<br>. . . . |
| iPS:43 6027 | 21-225_193E6 | VH4\|4-34/D4\|4-17\|RF2/JH4 | . . . . . . . . . . .<br>. . . . . . . . . . .<br>. F . . . . . . | . . . . . . P . . . | . . . . . . . . .<br>. . . . . .<br>. . . . | . S . . . . . .<br>. . R . . . . .<br>. . . . | . . . . . . . . . . . . . . . . R .<br>. . . . . . . . . . . . . . | . . . G –<br>. . . . . . . . . . . . . . . . . . . .<br>. . . – L . . | . . . . . .<br>. . . . |
| iPS:43 6080 | 21-225_195B1 | VH4\|4-34/D4\|4-17\|RF2/JH4 | . . . . . . . . . . .<br>. . . . . . . . . . .<br>. S . . . . . R | Y . . . . . . . F . . | . . . . S . . . .<br>. . . . F .<br>. . . . | . . . . . . . .<br>. . R . . . . .<br>. . . . | . . . . . . . . . . . . . . . .<br>R . . . . . . . . . . . . . | . . . A –<br>. . . . . . . . . . . . . . . . . . . .<br>. . . – . . I | . . . . . .<br>. . . . |
| iPS:43 6232 | 21-225_201E1 | VH4\|4-34/D4\|4-17\|RF2/JH4 | . . . . . . . . . . .<br>. . . . . . . . . . .<br>. F D . . . . . | P . . . . . . . . . | . . . . . . . . .<br>. . . . . .<br>. . . . | . V . . . . . .<br>. . . . . . . .<br>. . . . | . . . . . . . . . . . . . . . .<br>. . . . . . . . . . . . . . | . . . G –<br>. . . . . . . . . . . . . . . . . . . .<br>. . . – L . . | . . . . A .<br>. . . . |
| iPS:43 6238 | 21-225_201B2 | VH4\|4-34/D4\|4-17\|RF2/JH4 | . . . . . . . . . . .<br>. . . . . . . . . . .<br>. F . . . . I . | V . . . . . . . . T | . . . . . . . . .<br>. . . . . .<br>. . . . | . . . . . . . .<br>. . . . . . . .<br>. . . . | . . . . . . . . . . . . . . . .<br>. . . . . . . . . . . . . . | . . . V –<br>. . . . . . . . . . . . . . . . . . . .<br>. . . – . . . | . . . . . .<br>. . . . |
| iPS:43 6256 | 21-225_202D9 | VH4\|4-34/D4\|4-17\|RF2/JH4 | . . . . . . . . . . .<br>. . . . . . . . . . .<br>. . D . . . . | P . . . . . . . . | . . . . . . . . .<br>. . . . . .<br>. . . . | . . . . . . . .<br>. . . . . . . .<br>. . . . | . . . . . . . . . . . . . . . .<br>. . . . . . . . . . . . . . | . . . G –<br>. . . . . . . . . . . . . . . . . . . .<br>. . . – L . . | . . . . . .<br>. . . . |
| iPS:43 6302 | 21-225_205G7 | VH4\|4-34/D4\|4-17\|RF2/JH4 | . . . . . . . . . . .<br>. . . . . . . . . . .<br>. . . . . . . . | V . . . . . . . . . | . . . . . . . . .<br>. . . . . .<br>. . . . | . S . Q . . . .<br>. . R . T . . .<br>. . . . | . . . . . . . . . . . . . . N .<br>I . . . . . . . . . . . . . | . . . V –<br>. . . . . . . . . . . . . . . . . . . .<br>. . . – . . . | . . . . . .<br>. . . . |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 6310 | 21-225_202D11 | VH4\|4-34/D4\|4-17\|RF2/JH4 | ......R..... .......... A....... | ......?... | ........ ...... .... | ........ ......... .... | ................. | ...V- ................. ...-L.. | ...... ..... |
| iPS:43 6336 | 21-225_208B5 | VH4\|4-34/D4\|4-17\|RF2/JH4 | .......... .......... .F....I. | V........T | ........ ...... .... | ........ ......... .... | ................. | ...V- ................. ...-... | ...... ..... |
| iPS:43 6340 | 21-225_208A9 | VH4\|4-34/D4\|4-17\|RF2/JH4 | .......... .....P..... ........ | V.....S... | ........ ...... .... | ........ ..RA.... .... | ......I.......... | ...V- ................. ...-L.. | ...... ..... |
| iPS:43 7340 | 21-225_75G9 | VH4\|4-34/D4\|4-17\|RF2/JH4 | .......... .......... ........ | ......C... | ........ ...... .... | ........ ..R..... .... | .........E...... | ...G- ................. ...-L.. | ...... ..... |
| iPS:45 1122 | 21-225_200A1 | VH4\|4-34/D4\|4-17\|RF2/JH4 | .......... .......... ........ | V......... | ........ ...... .... | ........ ......... .... | ......L.......... | ...V- ................. ...-... | ...... ..... |
| | | Germline | H_FR1 | H_CDR1 | H_FR2 | H_CDR2 | H_FR3 | H_CDR3 | H_FR4 |
| | VH3\|3-33/D2\|2-15\|RF3/JH1 | | QVQLVES- GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | DIVVVVAA----------- ------TAEYFQH | WGQGTL VTVSS |
| iPS:43 4485 | 21-225_76D2 | VH3\|3-33/D2\|2-15\|RF3/JH1 | .......... .......... ........ | .......... | ........ ...... .... | ........ ......... .... | .............F... ............S | .RNI.G-- .............- .T..ES | ...... ..... |
| iPS:43 4537 | 21-225_74E11 | VH3\|3-33/D2\|2-15\|RF3/JH1 | .......... .......... ........ | .......... | ........ ...... .... | ........ ......... .... | .............F... ............S | .RNI.G-- .............- .T..ES | ...... ..... |
| iPS:43 4673 | 21-225_74E3 | VH3\|3-33/D2\|2-15\|RF3/JH1 | .......... ..........T ........ | .......... | ........ ...... .... | ........ ......... .... | .............F... ............S | .RNI.G-- .............- .T..ES | ...... ..... |
| iPS:43 5221 | 21-225_95G2 | VH3\|3-33/D2\|2-15\|RF3/JH1 | .......... ..........T ........ | .......... | ........ ...... .... | ........ ......... .... | .............F... ............S | .RNI.G-- .............- .T..ES | ...... ..... |
| | | Germline | H_FR1 | H_CDR1 | H_FR2 | H_CDR2 | H_FR3 | H_CDR3 | H_FR4 |
| | VH4\|4-39/D7\|7-27\|RF1/JH5 | | QLQLQES- GPGLVKPSEILS LTCTVSG-GSIS | SS---SYYWG | WIRQPPGK GLEWIG | SIYY--- SGSTYYNP SLKS | RVTISVDTSKNQFSLKL SSVTAADTAVYYCAR | LTGN--------- --------WFDP | WGQGTL VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 4503 | 21-225_74D7 | VH4\|4-39/D7\|7-27\|RF1/JH5 | ...........<br>...........S<br>......F | R......... | ........<br>......<br>.... | G.......<br>....S...<br>.... | .........E......<br>............... | .RP.W............<br>........⊃..Y | ......<br>.... |
| | | Germline | H_FR1 | H_CDR1 | H_FR2 | H_CDR2 | H_FR3 | H_CDR3 | H_FR4 |
| | VH3\|3-15/D1\|1-1\|RF2/JH4 | | EVQLVES-<br>GGGLVKPGGSLR<br>LSCAASG-FTFS | N-----AWMS | WVRQAPGK<br>GLEWVG | RIKSKT-<br>DGGTTDYA<br>APVKG | RFTISRDDSKNTLYLQM<br>NSLKTEDTAVYYCTT | VQLER------------<br>--------YFDY | WGQGTL<br>VTVSS |
| iPS:43 4531 | 21-225_76C9 | VH3\|3-15/D1\|1-1\|RF2/JH4 | ...........<br>...........<br>.....S.. | .........N | ........<br>......<br>.... | ...N.A..<br>.....F..<br>.... | ..........H......<br>............... | .GPT-<br>..................<br>...-T.. | ...... |
| iPS:43 4633 | 21-225_74G8 | VH3\|3-15/D1\|1-1\|RF2/JH4 | ...........<br>...........<br>........ | .........N | ........<br>......<br>.... | ...N.A..<br>........<br>.... | ................<br>................ | .GAT-<br>..................<br>...-T.. | ...... |
| iPS:43 4671 | 21-225_74F4 | VH3\|3-15/D1\|1-1\|RF2/JH4 | ...........<br>...........<br>........ | .........N | ........<br>......<br>.... | ...N.I..<br>........<br>.... | ................<br>................ | .GAT-<br>..................<br>...-T.. | ...... |
| iPS:43 7383 | 21-225_74H8 | VH3\|3-15/D1\|1-1\|RF2/JH4 | ..H........<br>...........<br>........ | .........N | ........<br>......<br>.... | ........<br>........<br>.... | ................<br>................ | .GAT-<br>..................<br>...-T.. | ...... |
| | | Germline | H_FR1 | H_CDR1 | H_FR2 | H_CDR2 | H_FR3 | H_CDR3 | H_FR4 |
| | VH3\|3-33/D5\|5-18\|RF3/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | GYSYG--------------------<br>--------YYFDY | WGQGTL<br>VTVSS |
| iPS:43 4535 | 21-225_74C8 | VH3\|3-33/D5\|5-18\|RF3/JH4 | ...........<br>...........<br>........ | .......... | ........<br>......<br>.... | ........<br>....N...<br>.... | ................<br>................ | DG...Y...........<br>.......DGL.. | ...... |
| iPS:43 4573 | 21-225_77E6 | VH3\|3-33/D5\|5-18\|RF3/JH4 | ...........<br>..S........<br>........ | .......... | ........<br>......<br>.... | ........<br>...QN...<br>.... | .............F...<br>................ | DG...Y...........<br>.......DGL.. | ...... |
| iPS:43 4615 | 21-225_76C5 | VH3\|3-33/D5\|5-18\|RF3/JH4 | ...........<br>...........<br>........ | .......... | ........<br>......<br>.... | ........<br>....N...<br>.... | ................<br>................ | DG...Y...........<br>.......DGL.. | ...... |
| iPS:43 4669 | 21-225_79F4 | VH3\|3-33/D5\|5-18\|RF3/JH4 | ...........<br>..S........<br>........ | N......... | ........<br>......<br>.... | ........<br>...QN...<br>.... | .............F...<br>................ | DG...Y...........<br>.......DGL.. | ...... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 4737 | 21-225_74G6 | VH3\|3-33/D5\|5-18\|RF3/JH4 | ............<br>............<br>........ | .......... | ........<br>......<br>.... | .......<br>....N...<br> | ................. | DG...Y............<br>.......DGL.. | ......<br>..... |
| iPS:43 4741 | 21-225_80C11 | VH3\|3-33/D5\|5-18\|RF3/JH4 | ............<br>............<br>........ | .......... | ........<br>......<br>.... | .......<br>....N...<br> | .............S... | DG...Y............<br>.......DGL.. | ......<br>..... |
| iPS:43 4867 | 21-225_79A12 | VH3\|3-33/D5\|5-18\|RF3/JH4 | ............<br>............<br>........ | .......... | ........<br>......<br>.... | .......<br>....N...<br> | ................ | DG...Y............<br>.......DGL.. | ......<br>.A... |
| | Germline | | H_FR1 | H_CDR1 | H_FR2 | H_CDR2 | H_FR3 | H_CDR3 | H_FR4 |
| **VH4\|4-34/D3\|3-10\|RF2/JH6** | | | QVQLQQW-<br>GAGLLKPSEILS<br>LTCAVYG-GSFS | G-----YYWS | WIRQPPGK<br>GLEWIG | EINH----<br>SGSTNYNP<br>SLKS | RVTISVDTSKNQFSLKL<br>SSVTAADTAVYYCAR | YYYGSGSYYN-------<br>---YYYYYGMDV | WGQGTT<br>VTVSS |
| iPS:43 4539 | 21-225_74A2 | VH4\|4-34/D3\|3-10\|RF2/JH6 | ...V........<br>............<br>.......T | D......... | ........<br>......<br>.... | .......<br>..D..... | ................. | EFPY....--<br>............-<br>L....... | ......<br>..... |
| iPS:43 7248 | 21-225_97H3 | VH4\|4-34/D3\|3-10\|RF2/JH6 | ...V........<br>............<br>.......T | D......... | ........<br>......<br>.... | .......<br>..D..... | ................. | EFPY....--<br>............-<br>L....... | ......<br>..... |
| iPS:43 7320 | 21-225_75A1 | VH4\|4-34/D3\|3-10\|RF2/JH6 | ...V........<br>..H.........<br>.......T | D......... | ........<br>......<br>.... | .......<br>..D..... | ................. | EFPY....--<br>............-<br>L....... | ......<br>..... |
| | Germline | | H_FR1 | H_CDR1 | H_FR2 | H_CDR2 | H_FR3 | H_CDR3 | H_FR4 |
| **VH3\|3-13/D3\|3-9\|RF1/JH6** | | | EVQLVES-<br>GGGLVQPGGSLR<br>LSCAASG-FIFS | S-----YDMH | WVRQATGK<br>GLEWVS | AIGT----<br>AGDTYYPG<br>SVKG | RFTISRENAKNSLYLQM<br>NSLRAGDTAVYYCAR | VLRYFDWLL*-------<br>---YYYYYGMDV | WGQGTT<br>VTVSS |
| iPS:43 4563 | 21-225_75D8 | VH3\|3-13/D3\|3-9\|RF1/JH6 | ............<br>............<br>........ | N......... | ........<br>......<br>.... | .......<br> | ................. | ..D.G.S.G-<br>...........-<br>........ | ......<br>..... |
| iPS:43 5009 | 21-225_89G4 | VH3\|3-13/D3\|3-9\|RF1/JH6 | ............<br>............<br>........ | .......... | ........<br>......<br>.... | .......<br> | ..........F... | A.D.G.S.G-<br>...........-<br>........ | ......<br>..... |
| iPS:43 5059 | 21-225_90C11 | VH3\|3-13/D3\|3-9\|RF1/JH6 | ............<br>............<br>........ | N......... | ........<br>......<br>.... | <br> | ................. | ..D.G.S.G-<br>...........-<br>........ | ......<br>..... |

| iPS | Clone | Germline | H_FR1 | H_CDR1 | H_FR2 | H_CDR2 | H_FR3 | H_CDR3 | H_FR4 |
|---|---|---|---|---|---|---|---|---|---|
| iPS:43 5103 | 21-225_92B2 | VH3|3-13/D3|3-9|RF1/JH6 | ............ ............ ........ | N........ | ........ ....... | ........ ....F... .... | .................. | A.D.G.S.C- .............- ........ | ...... ..... |
| iPS:43 7371 | 21-225_74D8 | VH3|3-13/D3|3-9|RF1/JH6 | ............ ............ ........ | N........ | ........ ...... | ........ ........ .... | .................. | ..D.G.S.G- .............- ........ | ...... ..... |
| **Germline** | | | **H_FR1** | **H_CDR1** | **H_FR2** | **H_CDR2** | **H_FR3** | **H_CDR3** | **H_FR4** |
| **VH1|1-08/D1|1-26|RF3/JH4** | | | QVQLVQS GAEVKKPGASVK VSCKASG-YIFT | S    YDIN | WVRQATGQ GLEWMG | WMNPN SGNTGYAQ KFQG | RVTMTRNTSISTAYMEL SSLRSEDTAVYYCAR | YSCSY ------YYFDY | WGQGTL VTVSS |
| iPS:43 4711 | 21-225_80H3 | VH1|1-08/D1|1-26|RF3/JH4 | ............ ............ ......L. | N........ | ........ ...... | ........ .... | .................. ..............GS | T..WN............. ........-F... | ...... ..... |
| iPS:43 4901 | 21-225_85H9 | VH1|1-08/D1|1-26|RF3/JH4 | ............ ............ ......L. | N........ | ........ ...... | ........ .... | .................. .............RGS | T..WN............. ........-F... | ...... ..... |
| iPS:43 5167 | 21-225_92F12 | VH1|1-08/D1|1-26|RF3/JH4 | ............ ............ ......L. | N........ | ........ ...... | ........ .... | .................. .............RGS | T..GK............. ........-F... | ...... ..... |
| iPS:43 5215 | 21-225_94E12 | VH1|1-08/D1|1-26|RF3/JH4 | ............ ............ ......L. | N........ | ........ ...... | ........ .... | .................. .............RGS | T..WK............. ........-F... | ...... ..... |
| iPS:43 7356 | 21-225_74B1 | VH1|1-08/D1|1-26|RF3/JH4 | ............ ............ ......L. | N........ | ........ ...... | ........ .... | .................. ..............GS | T..WN............. ........-F... | ...... ..... |
| **Germline** | | | **H_FR1** | **H_CDR1** | **H_FR2** | **H_CDR2** | **H_FR3** | **H_CDR3** | **H_FR4** |
| **VH1|1-08/D1|1-1|RF1/JH6** | | | QVQLVQS GAEVKKPGASVK VSCKASG-YIFT | S    YDIN | WVRQATGQ GLEWMG | WMNPN SGNTGYAQ KFQG | RVTMTRNTSISTAYMEL SSLRSEDTAVYYCAR | GTIGTYY ------YYYGMDV | WGQGTT VTVSS |
| iPS:43 4815 | 21-225_74A11 | VH1|1-08/D1|1-1|RF1/JH6 | ............ ............ ........ | ............ | ........ ...... | ........ .... | .....W...K....... .............. | .FYD.LTGS......... ....G...V... | ...... ..... |
| iPS:43 5253 | 21-225_96A4 | VH1|1-08/D1|1-1|RF1/JH6 | ............ ............ ........ | ............ | ........H ...... | ........ .... | .....W...K....... .............. | .FYD.LTGS......... ....G...V... | ...... ..... |
| **Germline** | | | **H_FR1** | **H_CDR1** | **H_FR2** | **H_CDR2** | **H_FR3** | **H_CDR3** | **H_FR4** |

| | VH1\|1-08\|D3\|3-22\|RF2\|JH6 | | QVQLVQS-GAEVKKPGASVK VSCKASG-YTFT | S-----YDIN | WVRQATGQ GLEWMG | WMNPN--- SGNTGYAQ KFQG | RVTMTRNTSISTAYMEL SSLRSEDTAVYYCAR | YYYDSSGYYY---------- ---YYYYYGMDV | WGQGTT VTVSS |
|---|---|---|---|---|---|---|---|---|---|
| iPS:43 4977 | 21-225_88A5 | VH1\|1-08\|D3\|3-22\|RF2\|JH6 | .............. ............. ........ | .......... | ......... ...... | ......... .... | .....W....R...... ................. | GF..FLTG.S........ ..PT....D... | ...... ..... |
| iPS:43 5259 | 21-225_96C6 | VH1\|1-08\|D3\|3-22\|RF2\|JH6 | ............. ............. ........ | .......... | ......... ...... | ......... .R...... .... | .....W........... ................. | GG..VLPGN- ...........- N...D... | ...... ..... |
| | Germline | H_FR1 | H_CDR1 | H_FR2 | H_CDR2 | H_FR3 | H_CDR3 | H_FR4 | |
| | VH3\|3-33\|D2\|2-15\|RF3\|JH3 | | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | DIVVVVA----------- -----ATDAFDI | WGQGTM VTVSS |
| iPS:43 5291 | 21-225_146E1 | VH3\|3-33\|D2\|2-15\|RF3\|JH3 | ............. ...........E ....I... | .......... | ......... ...... | I....... ......... .... | ................F... ................. | .RL.GAT........... ......-A..... | ...... ..... |
| iPS:43 6360 | 21-225_210H11 | VH3\|3-33\|D2\|2-15\|RF3\|JH3 | ............. ............. ........ | ......H... | ......... ...... | .T...... ..D..... .... | ................. ................. | .RL.GAT........... .....-..... | ...... ..... |
| iPS:43 6370 | 21-225_211A6 | VH3\|3-33\|D2\|2-15\|RF3\|JH3 | ............. ............. ........ | .......... | ......... ...... | ......... ....N... .... | ................. ..........F.... | .RT.GY- ................--- .G... | ...... ..... |
| iPS:43 6392 | 21-225_213B3 | VH3\|3-33\|D2\|2-15\|RF3\|JH3 | ............. ............. ........ | .......... | ......... ...... | ......... ....N... .... | ................. ................. | .RT.GY- ................--- .G... | ...... ..... |
| iPS:43 6406 | 21-225_214E4 | VH3\|3-33\|D2\|2-15\|RF3\|JH3 | ............. ............. ........ | .......... | ......... ...... | ......... ...EN... .... | .I............... ................. | .RT.GY- ................--- .GC.. | ....A. ..... |
| iPS:43 7326 | 21-225_75C10 | VH3\|3-33\|D2\|2-15\|RF3\|JH3 | ............. ............. ........ | .......... | ......... ...... | ......... ..D..... .... | ................. ................. | .RL.GAT........... ......-V..... | ...... ..... |
| | Germline | H_FR1 | H_CDR1 | H_FR2 | H_CDR2 | H_FR3 | H_CDR3 | H_FR4 | |
| | VH4\|4-39\|D7\|7-27\|RF1\|JH4 | | QLQLQES-GPGLVKPSETLS LTCTVSG-GSIS | SS---SYYWG | WIRQPPGK GLEWIG | SIYY---- SGSTYYNP SLKS | RVTISVDTSKNQFSLKL SSVTAADTAVYYCAR | LTGY---------- --------FDY | WGQGTL VTVSS |
| iPS:43 5293 | 21-225_146F1 | VH4\|4-39\|D7\|7-27\|RF1\|JH4 | ............. ............. ........ | R......... | ......... ...... | ......... ....S... .... | ................. ................. | .DLLW............ ........S... | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| IPS:43 5361 | 21-225_148E11 | VH4\|4-39/D7\|7-27\|RF1/JH4 | ...........R........<br>.............<br>....V... | | ........<br>......<br>.... | ........<br>....S...<br>.... | ................<br>........F.... | .DPQW.............<br>........S... | ....I.<br>..... |
| IPS:43 5449 | 21-225_152H9 | VH4\|4-39/D7\|7-27\|RF1/JH4 | ...........R........<br>.............<br>........ | | ........<br>......<br>.... | ........<br>...AS...<br>.... | ................<br>........F.... | .DLQW.............<br>........S..F | ......<br>..... |
| IPS:43 5499 | 21-225_156G1 | VH4\|4-39/D7\|7-27\|RF1/JH4 | ...........R........<br>.............<br>........ | | ........<br>......<br>.... | ........<br>...AS...<br>.... | ................<br>........F.... | .DLQW.............<br>........S..F | ......<br>..... |
| IPS:43 5587 | 21-225_160H3 | VH4\|4-39/D7\|7-27\|RF1/JH4 | ...........R........<br>........ | | ........<br>...... | ........<br>....S...<br>..E. | ................<br>........F.... | .SQRW.............<br>........D... | ......<br>..... |
| IPS:40 3868 | 21-225_19D11 | VH4\|4-39/D7\|7-27\|RF1/JH4 | ...........R........<br>........ | | ........<br>..D... | ........<br>...AN...<br>.... | ................<br>......A....... | .DRGW.............<br>........S... | ......<br>..... |
| | **VH3\|3-23/D4\|4-17\|RF2/JH5** | | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | DYGDY--------------- NWFDP | WGQCTL VTVSS |
| IPS:43 5295 | 21-225_146H1 | VH3\|3-23/D4\|4-17\|RF2/JH5 | ...........<br>...........<br>........ | ......... | ........<br>......<br>.... | ....R...<br>..N.F...<br>.... | ................ | RVT..GG............<br>......ND.... | ......<br>..... |
| IPS:43 5307 | 21-225_146E9 | VH3\|3-23/D4\|4-17\|RF2/JH5 | ...........<br>...........<br>........ | ......... | ........<br>......<br>.... | ....R...<br>..N.F...<br>.... | .............K...... | RVT..GG............<br>......ND.... | ......<br>..... |
| IPS:43 5347 | 21-225_148C4 | VH3\|3-23/D4\|4-17\|RF2/JH5 | ...........<br>...........<br>........ | .........N | ........<br>......<br>.... | ........<br>..N.F...<br>.... | ................ | RVT..GG............<br>......ND.... | ......<br>..... |
| IPS:43 5355 | 21-225_148H9 | VH3\|3-23/D4\|4-17\|RF2/JH5 | ...........<br>...........<br>........ | ........... | ........<br>......<br>.... | ....R...<br>..N.F...<br>.... | ................ | RVT..GG............<br>......ND.... | ......<br>..... |
| IPS:43 5371 | 21-225_149A3 | VH3\|3-23/D4\|4-17\|RF2/JH5 | ...........<br>...........<br>........ | N........T | ........<br>......<br>.... | ....R...<br>..N.F...<br>.... | ...............T. | RVT..GG............<br>......ND.... | ......<br>..... |

| ID | Clone | Germline | H_FR1 | H_CDR1 | H_FR2 | H_CDR2 | H_FR3 | H_CDR3 | H_FR4 |
|---|---|---|---|---|---|---|---|---|---|
| IPS:43 5415 | 21-225_150C11 | VH3\|3-23/D4\|4-17\|RF2/JH5 | ............. / ............. / ........ | .........X | ........ / ...... / .... | ......... / ..N.F... / .... | .................N... / S............... | RVT..GG............ / ......ND.... | ...... / ..... |
| IPS:43 5419 | 21-225_150C12 | VH3\|3-23/D4\|4-17\|RF2/JH5 | ............. / ............. / ........ | .......... | ........ / ...... / .... | ....R... / ..N.F... / .... | ................ | RVT..GG............ / ......ND.... | ...... / ..... |
| IPS:43 5425 | 21-225_151B12 | VH3\|3-23/D4\|4-17\|RF2/JH5 | ............. / ............. / ........ | .........N | ...H.... / ...... / .... | ......... / .KN.F... / .... | ................ | RVT..GG............ / ......ND.... | ...... / ..... |
| IPS:43 5431 | 21-225_152D2 | VH3\|3-23/D4\|4-17\|RF2/JH5 | ............. / ............. / ........ | .......... | ........ / ...... / .... | ....R... / ..N.F... / .... | ...........K... | RVT..GG............ / ......ND.... | ...... / ..... |
| IPS:43 5439 | 21-225_152G4 | VH3\|3-23/D4\|4-17\|RF2/JH5 | ............. / ............. / ........ | .......... | ........ / ...... / .... | ....R... / ..N.F... / .... | ................ | RVT..GG............ / ......ND.... | ...... / ..... |
| IPS:43 5455 | 21-225_152B11 | VH3\|3-23/D4\|4-17\|RF2/JH5 | ............. / ............. / ........ | .........N | ........ / ...... / .... | ....R... / ..N.F... / .... | ................ | RVT..GG............ / ......ND.... | ...... / ..... |
| IPS:43 5487 | 21-225_155C4 | VH3\|3-23/D4\|4-17\|RF2/JH5 | ............. / ............. / ........ | .......... | ........ / ...... / .... | ....R... / ..N.F... / .... | ................ | RVT..GG............ / ......ND.... | ...... / ..... |
| IPS:43 5503 | 21-225_156E4 | VH3\|3-23/D4\|4-17\|RF2/JH5 | ............. / ............. / ........ | .......... | ........ / ...... / .... | ....R... / ..N.F... / .... | ................ | RVT..GG............ / ......ND.... | ...... / ..... |
| | | Germline | H_FR1 | H_CDR1 | H_FR2 | H_CDR2 | H_FR3 | H_CDR3 | H_FR4 |
| | VH3\|3-30/D3\|3-22\|RF3/JH6 | | QVQLVES-GGGVVQPGRSLRLSCAASG-FIFS | S-----YGMH | WVRQAPGKGLEWVA | VISYD---GSNKYYADSVKG | RFTISRDNSKNTLYLQMNSLRAEDTAVYYCAK | ITMIVVVIT---------YYYYYGMDV | WGQGTTVTVSS |
| IPS:43 5297 | 21-225_146B3 | VH3\|3-30/D3\|3-22\|RF3/JH6 | ............. / ............. / ........ | .......... | ........ / ...... / .... | ..W..... / ..Y..... / .... | ..............D... / ..............V. | MGIE.A.D- / .............-- / ....... | ...... / ..... |
| | | Germline | H_FR1 | H_CDR1 | H_FR2 | H_CDR2 | H_FR3 | H_CDR3 | H_FR4 |
| | VH4\|4-30.1/D1\|1-1\|RF1/JH3 | | QVQLQES-GPGLVKPSQILSLTCTVSG-GSIS | SG---GYYWS | WIRQHPGKGLEWIG | YIYYSGSTYYNPSLKS | RVTISVDTSKNQFSLKLSSVTAADTAVYYCAR | GTTGT----------DAFDI | WGQGTMVTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:43 5301 | 21-225_146G4 | VH4\|4-30.1/D1\|1-1\|RF1/JH3 | ............ .........N.. ........ | NS........ | ........ ...... .... | .S...... ........ .... | .I.......N....... 〔............... | .KYNWN........... .......H.... | ...... ..... |
| | **VH3\|3-21/D6\|6-13\|RF1/JH4** | Germline | **H_FR1** EVQLVES- GGGLVKPGGSLR LSCAASG-FIFS | **H_CDR1** S-----YSMN | **H_FR2** WVRQAPGK GLEWVS | **H_CDR2** SISSS--- SSYIYYAD SVKG | **H_FR3** RFTISRDNAKNSLYLQM NSLRAEDTAVYYCAR | **H_CDR3** GYSSSW-------- -------YYFDY | **H_FR4** WGQGTL VTVSS |
| iPS:43 5313 | 21-225_146G11 | VH3\|3-21/D6\|6-13\|RF1/JH4 | .......... .......... ........ | .......... | ........ ...... .... | ...G.... G..T.... .... | ................ | .S...- ................ .-G... | ...... .... |
| iPS:39 3808 | 21-225_1A2 | VH3\|3-21/D6\|6-13\|RF1/JH4 | .......... .......... ........ | .......T.. | ........ ...... .... | ...G.... G....... .... | ................ | .S...- ................ .-G... | ...... .... |
| iPS:39 3958 | 21-225_5H2 | VH3\|3-21/D6\|6-13\|RF1/JH4 | .......... .......... ........ | .......T.. | ........ ...... .... | ...G.... ........ .... | ......A.......... | .S...- ................ .-G... | ...... .... |
| | **VH4\|4-30.4/D5\|5-18\|RF3/JH6** | Germline | **H_FR1** QVQLQES- GPGLVKPSQTLS LTCTVSG-GSIS | **H_CDR1** SG---DYYWS | **H_FR2** WIRQPPGK GLEWIG | **H_CDR2** YIYY---- SGSTYYNP SLKS | **H_FR3** RVTISVDTSKNQFSLKL SSVTAADTAVYYCAR | **H_CDR3** GYSYGYYY--------- -----YYYGMDV | **H_FR4** WGQGTT VTVSS |
| iPS:43 5317 | 21-225_147D2 | VH4\|4-30.4/D5\|5-18\|RF3/JH6 | ..L....... ..........A .....P.. | .........N | ....R... ...... .... | F....... T....... .... | ..S..E...E.....N. | .GA.YS-- ................- ...... | ...... ..... |
| | **VH4\|4-30.1/D5\|5-24\|RF3/JH3** | Germline | **H_FR1** QVQLQES- GPGLVKPSQTLS LTCTVSG-GSIS | **H_CDR1** SG---GYYWS | **H_FR2** WIRQHPGK GLEWIG | **H_CDR2** YIYY---- SGSTYYNP SLKS | **H_FR3** RVTISVDTSKNQFSLKL SSVTAADTAVYYCAR | **H_CDR3** RDCYNY---------- -------DAFDI | **H_FR4** WGQGTM VTVSS |
| iPS:43 5319 | 21-225_147E3 | VH4\|4-30.1/D5\|5-24\|RF3/JH3 | .......... .......... .......T | NS......Y. | ........ ...... .... | ........ ..G..... .... | .I.......N....... | GGYNWN........... .......H...F | ...... ..... |
| iPS:43 5383 | 21-225_149D7 | VH4\|4-30.1/D5\|5-24\|RF3/JH3 | .......... .........N.. ........ | NS........ | ........ ...... .... | .S...... ........ .... | .I.......N....... | GGYNWN........... .......H.... | ...... .I... |

| | | | H_FR1 | H_CDR1 | H_FR2 | H_CDR2 | H_FR3 | H_CDR3 | H_FR4 |
|---|---|---|---|---|---|---|---|---|---|
| iPS:43 5443 | 21-225_152E7 | VH4\|4-30.1/D5\|5-24\|RF3/JH3 | ............ .........N.. ........ | NS........ | ........ ...... | .S...... ........ .... | .I.......N.....N. ............... | GGYNWN............ .......H.... | ...... .... |
| iPS:43 5465 | 21-225_153A6 | VH4\|4-30.1/D5\|5-24\|RF3/JH3 | ............ .........N.. ........ | NS........ | ........ ...... | .S...... ........ .... | .I.......N.....N. ............... | GGYNWN............ .......H.... | ...... .... |
| iPS:44 2568 | 21-225_149D8 | VH4\|4-30.1/D5\|5-24\|RF3/JH3 | ............ ...........N.. ........ | NS........ | ........ ...... | .S...... ........ .... | .I.......N....... ............... | GGYNWN............ .......H.... | ...... .... |
| | Germline | | H_FR1 | H_CDR1 | H_FR2 | H_CDR2 | H_FR3 | H_CDR3 | H_FR4 |
| | VH3\|3-48/D4\|4-11\|RF2/JH4 | | EVQLVES-GGGLVQPGGSLR LSCAASG-FTFS | S-----YSMN | WVRQAPGK GLEWVS | YISSS--- SSTIYYAD SVKG | RFTISRDNAKNSLYLQM NSLRDEDTAVYYCAR | DYSNY------------ --------YFDY | WGQGTL VTVSS |
| iPS:43 5333 | 21-225_147E9 | VH3\|3-48/D4\|4-11\|RF2/JH4 | ............ ............ ........ | .......... | ........ ...... | S..GR... NT...... .... | ................. ..............S. | .RG-- ................ ...--SC | ...... |
| iPS:43 5637 | 21-225_163E2 | VH3\|3-48/D4\|4-11\|RF2/JH4 | ............ ............ ........ | .......... | ........ ...... | ST.G.... .TY..... .... | ...........LV.... ....P.......... | .RG-- ................ ...--SL | ...... |
| | Germline | | H_FR1 | H_CDR1 | H_FR2 | H_CDR2 | H_FR3 | H_CDR3 | H_FR4 |
| | VH1\|1-02/D2\|2-15\|RF3/JH4 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | DIVVVVA----------- ------ATYFDY | WGQGTL VTVSS |
| iPS:43 5351 | 21-225_148B6 | VH1\|1-02/D2\|2-15\|RF3/JH4 | ............ ............ ........ | .......... | ........ ...... | ..H..... N....... T... | ..............V.... ................ | .P...P- ................ AP... | ...... |
| | Germline | | H_FR1 | H_CDR1 | H_FR2 | H_CDR2 | H_FR3 | H_CDR3 | H_FR4 |
| | VH4\|4-30.1/D5\|5-12\|RF3/JH6 | | QVQIQES-GPGLVKPSQILS LTCTVSG-GSIS | SG---GYYWS | WIRQHPGK GLEWIG | YIYY---- SGSTYYNP SLKS | RVTISVDTSKNQFSLKL SSVTAADTAVYYCAR | GYSGYDYY---------- ----YYYYGMDV | WGQGTT VTVSS |
| iPS:43 5363 | 21-225_148F12 | VH4\|4-30.1/D5\|5-12\|RF3/JH6 | ............ ............ ........ | N........N | ........ ...... | ........ .... | QI........D....R. ................ | YSTYDY-- ..............-- ...... | ...... |

3256

| | | | H_FR1 | H_CDR1 | H_FR2 | H_CDR2 | H_FR3 | H_CDR3 | H_FR4 |
|---|---|---|---|---|---|---|---|---|---|
| iPS:43 5377 | 21-225_149G5 | VH4|4-30.1/D5|5-12|RF3/JH6 | ............<br>............<br>........ | N.........X | ........<br>......<br>.... | ........ | ..........D....R. | YSTYDY--<br>...............-- | ......<br>..... |
| | | Germline | H_FR1 | H_CDR1 | H_FR2 | H_CDR2 | H_FR3 | H_CDR3 | H_FR4 |
| | VH3|3-48/D6|6-6|RF2/JH4 | | EVQLVES-GGGLVQPGGSLR LSCAASG-FTFS | S------YSMN | WVRQAPGK GLEWVS | YISSS---SSTIYYAD SVKG | RFTISRDNAKNSLYLQM NSLRDEDTAVYYCAR | SIAAR-----------------YFDY | WGQGTL VTVSS |
| iPS:43 5409 | 21-225_150G8 | VH3|3-48/D6|6-6|RF2/JH4 | ............<br>.........<br>........ | T.........T | ........<br>..D...<br>.... | ...R.... | ..S.............<br>.............L..... | .AFS-<br>..................<br>...P... | ...... |
| | | Germline | H_FR1 | H_CDR1 | H_FR2 | H_CDR2 | H_FR3 | H_CDR3 | H_FR4 |
| | VH3|3-30.3/D5|5-18|RF2/JH5 | | QVQLVES-GGGVVQPGRSLR LSCAASG-FTFS | S------YAMH | WVRQAPGK GLEWVA | VISYD---GSNKYYAD SVKG | RFTISRDNSKNTLYLQM NSLRAEDTAVYYCAR | WIQLWL------------------NWFDP | WGQGTL VTVSS |
| iPS:43 5427 | 21-225_151C9 | VH3|3-30.3/D5|5-18|RF2/JH5 | ............<br>............<br>........ | .......G.. | ........<br>......<br>.... | ........ | ................. | GVL..FGE.........<br>....LEDD.... | ......<br>..... |
| | | Germline | H_FR1 | H_CDR1 | H_FR2 | H_CDR2 | H_FR3 | H_CDR3 | H_FR4 |
| | VH3|3-33/D3|3-22|RF2/JH4 | | QVQLVES-GGGVVQPGRSLR LSCAASG-FTFS | S------YGMH | WVRQAPGK GLEWVA | VIWYD---GSNKYYAD SVKG | RFTISRDNSKNTLYLQM NSLRAEDTAVYYCAR | YYYDSSG--------------------YYYYFDY | WGQGTL VTVSS |
| iPS:43 5441 | 21-225_152F6 | VH3|3-33/D3|3-22|RF2/JH4 | ............<br>............<br>........ | .......... | ........<br>......<br>.... | I.......<br>..Y.....<br>.... | ................. | EG..FW-<br>.....................-<br>SG.LG. | ......<br>..... |
| iPS:43 5457 | 21-225_152C11 | VH3|3-33/D3|3-22|RF2/JH4 | ............<br>............<br>.......R | N......... | ........<br>......<br>.... | I.......<br>.......<br>.... | ................. | EA..FW-<br>.....................-<br>SG.... | ....I.<br>..... |
| iPS:43 5463 | 21-225_153D2 | VH3|3-33/D3|3-22/RF2/JH4 | ............<br>............<br>........ | .......... | ........<br>......<br>.... | I.......<br>..Y.....<br>.... | ............V.... | EG..FW-<br>.....................-<br>SG.LG. | ......<br>..... |
| iPS:43 5531 | 21-225_157G8 | VH3|3-33/D3|3-22/RF2/JH4 | ............<br>............<br>........ | N......... | ........<br>......<br>.... | I.......<br>..Y.....<br>.... | ...V............. | EG..FW-<br>.....................-<br>SGF..S | ......<br>..... |
| iPS:43 5577 | 21-225_160B1 | VH3|3-33/D3|3-22/RF2/JH4 | ............<br>............<br>........ | .......... | ........<br>......<br>.... | .......<br>..Y.....<br>.... | ................. | EA..FW-<br>.....................-<br>SG.Y.. | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 5723 | 21-225_172B7 | VH3\|3-33/D3\|3-22\|RF2/JH4 | ............ ............ ....... | N......... | ........ ...... | I....... ......V. .... | ................ ................ | EA..FW- ...............- SG.W.. | ...... ..... |
| iPS:43 5731 | 21-225_173A11 | VH3\|3-33/D3\|3-22\|RF2/JH4 | ............ ............ ....... | ......... | ........ ...... | I....... ........ .... | ................ ................ | EA..FW- ...............- SGF..S | ...... ..... |
| iPS:43 5781 | 21-225_178G10 | VH3\|3-33/D3\|3-22\|RF2/JH4 | ............ ............ ....... | T......... | ........ ...... | ........ ........ .... | ........F........ ................ | ER..FW- ...............- SGH... | ...... ..... |
| iPS:43 5899 | 21-225_188G11 | VH3\|3-33/D3\|3-22\|RF2/JH4 | ............ ............ ....... | ......... | ........ ...... | I....... ..Y..... .... | ...............F... ................ | ER..FW- ...............- SGH... | ...... ..... |
| iPS:43 6602 | 21-225_226E7 | VH3\|3-33/D3\|3-22\|RF2/JH4 | ............ ............ ....... | T......... | ........ ...... | I....... ........ .... | ........F H....D.......... | EN..FW- ...............- SG.YG. | ...... ..... |
| iPS:39 2930 | 21-225_25H9 | VH3\|3-33/D3\|3-22\|RF2/JH4 | ............ ............ .....P.N | N......... | ........ ...... | I....... ..Y..... .... | ................ | EG..FW- ...............- SGF..S | ...... ..... |
| | | Germline | H_FR1 | H_CDR1 | H_FR2 | H_CDR2 | H_FR3 | H_CDR3 | H_FR4 |
| | VH3\|3-23/D1\|1-26\|RF2/JH4 | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | V*WEL------------- -------LYFDY | WGQGTL VTVSS |
| iPS:43 5479 | 21-225_154E9 | VH3\|3-23/D1\|1-26\|RF2/JH4 | ..K. ............ ....... | ......... | ........ ...... | ....R... ..N.F... .... | ..............F... | RGFRFLE........... .....WLGG... | ...... ..... |
| | | Germline | H_FR1 | H_CDR1 | H_FR2 | H_CDR2 | H_FR3 | H_CDR3 | H_FR4 |
| | VH3\|3-23/D5\|5-18\|RF3/JH4 | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | GYSYG------------- -------YYFDY | WGQGTL VTVSS |
| iPS:43 5497 | 21-225_155H9 | VH3\|3-23/D5\|5-18\|RF3/JH4 | ............ ............ ....... | .........N | ........ ...... | T...R... .LG..... .... | ................L | DHD..DY........... ......NI.... | ...... ..... |
| | | Germline | H_FR1 | H_CDR1 | H_FR2 | H_CDR2 | H_FR3 | H_CDR3 | H_FR4 |
| | VH3\|3-23/D6\|6-19\|RF1/JH3 | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | GYSSGW------------ ------YDAFDI | WGQGTM VTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:43 5513 | 21-225_157F3 | VH3\|3-23/D6\|6-19\|RF1/JH3 | ............<br>............<br>..E..... | T......... | ........<br>......<br>.... | V.......<br>........<br>.... | ................<br>................ | RS.GWY............<br>......E..L.. | ......<br>..... |
| iPS:39 2766 | 21-225_23H4 | VH3\|3-23/D6\|6-19\|RF1/JH3 | ............<br>............<br>........ | .........N | ........<br>......<br>.... | V.......<br>..T..F..<br>.... | ................<br>................ | RN.................<br>......H.V... | ......K<br>..... |
| iPS:39 2808 | 21-225_20F8 | VH3\|3-23/D6\|6-19\|RF1/JH3 | ............<br>............<br>......R | ......... | .I.....R<br>....S.<br>.... | V.......<br>........<br>.... | S...............<br>................ | R.N...............<br>......H.V... | ......<br>..... |
| | | **Germline** | **H_FR1** | **H_CDR1** | **H_FR2** | **H_CDR2** | **H_FR3** | **H_CDR3** | **H_FR4** |
| | **VH4\|4-39/D2\|2-21\|RF3/JH4** | | QLQLQES-GPGLVKPSEILS LTCTVSG-GSIS | SS---SYYWG | WIRQPPGK GLEWIG | SIYY---- SGSTYYNP SLKS | RVTISVDTSKNQFSLKL SSVTAADTAVYYCAR | HIVVVT--------- -----AIYFDY | WGQGTL VTVSS |
| iPS:43 5525 | 21-225_157E7 | VH4\|4-39/D2\|2-21\|RF3/JH4 | ..H.........<br>........ | .G........ | ........<br>......<br>.... | ........<br>........ | ................N.<br>................ | .K.AG-<br>................<br>--P... | ......<br>..... |
| | | **Germline** | **H_FR1** | **H_CDR1** | **H_FR2** | **H_CDR2** | **H_FR3** | **H_CDR3** | **H_FR4** |
| | **VH3\|3-33/D3\|3-10\|RF2/JH6** | | QVQLVES-GGGVVQPGRSLR LSCAASG-FTFS | S----YGMH | WVRQAPGK GLEWVA | VIWYD--- GSNKYYAD SVKG | RFTISRDNSKNTLYLQM NSLRAEDTAVYYCAR | YYYGSGSYYN------- ---YYYYYGMDV | WGQGTT VTVSS |
| iPS:43 5543 | 21-225_158D4 | VH3\|3-33/D3\|3-10\|RF2/JH6 | ............<br>............<br>........ | D......V.. | ........<br>......S<br>.... | ........<br>........<br>.... | ................<br>................ | EP.T..W---<br>..............--<br>.D..... | ......<br>..... |
| iPS:43 5571 | 21-225_159C8 | VH3\|3-33/D3\|3-10\|RF2/JH6 | ............<br>............<br>........ | D......V.Q | ........<br>......<br>.... | ........<br>........<br>.... | ................<br>................ | EP.N..W---<br>.............--<br>.D..... | ......<br>..... |
| iPS:43 5591 | 21-225_160C4 | VH3\|3-33/D3\|3-10\|RF2/JH6 | ............<br>............<br>........ | D......V.Q | ........<br>......<br>.... | ........<br>........<br>.... | ................<br>................ | EP.N..W---<br>.............--<br>.D..... | ......<br>..... |
| iPS:43 5615 | 21-225_161G12 | VH3\|3-33/D3\|3-10\|RF2/JH6 | ............<br>............<br>........ | D......V.Q | ........<br>......<br>.... | ........<br>........<br>.... | ................<br>................ | EP.N..W---<br>.............--<br>.D..... | ......<br>..... |
| iPS:43 6604 | 21-225_226F7 | VH3\|3-33/D3\|3-10\|RF2/JH6 | ............<br>............<br>........ | .......... | ........<br>......<br>.... | I.......<br>........<br>.... | ................<br>................ | ER.N..W---<br>.............--<br>.D..L.. | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:45 1114 | 21-225_159A3 | VH3\|3-33/D3\|3-10\|RF2/JH6 | ............<br>................<br>........ | D......V.Q | ........<br>...... | ........<br>.... | .......I.........<br>................. | EP.N..W---<br>.........--<br>.D..... | ......<br>..... |
| iPS:47 2732 | 21-225_2B10_LC1 | VH3\|3-33/D3\|3-10\|RF2/JH6 | ............<br>................<br>........ | .......V.. | ........<br>...... | ........<br>...V | ...V........S...<br>................. | ER.T..W---<br>.........--<br>.D..... | ......<br>..... |
| iPS:47 2733 | 21-225_2B10_LC2 | VH3\|3-33/D3\|3-10\|RF2/JH6 | ............<br>................<br>........ | .......V.. | ........<br>...... | ........<br>...V | ...V........S...<br>................. | ER.T..W---<br>............--<br>.D..... | ......<br>..... |
| iPS:39 2872 | 21-225_20B11 | VH3\|3-33/D3\|3-10\|RF2/JH6 | ............<br>................<br>........ | .......V.. | ........<br>...... | ........<br>...V | ...V........S...<br>................. | ER.T..W---<br>............--<br>.D..... | ......<br>..... |
| iPS:39 3966 | 21-225_7F8 | VH3\|3-33/D3\|3-10\|RF2/JH6 | ............<br>................<br>........ | ......CV.. | ........<br>...... | ........<br>.... | .........N....... | ER.T..W---<br>............--<br>HD..... | ......<br>..... |
| | | Germline | H_FR1 | H_CDR1 | H_FR2 | H_CDR2 | H_FR3 | H_CDR3 | H_FR4 |
| | VH3\|3-23/D1\|1-1\|RF1/JH6 | | EVQLLES-GGGLVQPGGSLRLSCAASG-FIFS | S-----YAMS | WVRQAPGKGLEWVS | AISGS---GGSTYYADSVKG | RFTISRDNSKNTLYLQMNSLRAEDTAVYYCAK | GTTGTYY----------------YYYGMDV | WGQGTTVTVSS |
| iPS:43 5559 | 21-225_158H12 | VH3\|3-23/D1\|1-1\|RF1/JH6 | ....V.......<br>............<br>........ | .......V.. | ...... | ..R.D...<br>.... | ................ | .GW----<br>.................--<br>--NHD | ..... |
| | | Germline | H_FR1 | H_CDR1 | H_FR2 | H_CDR2 | H_FR3 | H_CDR3 | H_FR4 |
| | VH3\|3-21/D1\|1-1\|RF1/JH6 | | EVQLVES-GGGLVKPGGSLRLSCAASG-FIFS | S-----YSMN | WVRQAPGKGLEWVS | SISSS---SSYIYYADSVKG | RFTISRDNAKNSLYLQMNSLRAEDTAVYYCAR | GTTGTYY----------------YYYGMDV | WGQGTTVTVSS |
| iPS:43 5561 | 21-225_159F1 | VH3\|3-21/D1\|1-1\|RF1/JH6 | ............<br>............<br>........ | .......R.. | ...... | ...G....<br>GN..D...<br>.... | ................ | .W-----<br>.................--<br>---.. | ..... |
| | | Germline | H_FR1 | H_CDR1 | H_FR2 | H_CDR2 | H_FR3 | H_CDR3 | H_FR4 |
| | VH1\|1-08/D1\|1-1\|RF1/JH4 | | QVQLVQS-GAEVKKPGASVKVSCKASG-YIFT | S     YDIN | WVRQATGQGLEWMG | WMNPN-SGNTGYAQKFQG | RVTMTRNTSISTAYMELSSLRSEDTAVYYCAR | GTTGT----------------YFDY | WGQGTLVTVSS |
| iPS:43 5563 | 21-225_159H2 | VH1\|1-08/D1\|1-1\|RF1/JH4 | ............<br>............<br>........ | .......... | ...... | ......V.<br>.... | ................ | KK.--<br>...-G.. | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:39 2718 | 21-225_17B8 | VH1\|1-08/D1\|1-1\|RF1/JH4 | ............ / ............ / ........ | ........A.. | ........ / ...... / .... | ........ / T........ / .... | ................ / ........F.T. | KAG-- / ................... / ...-... | ...... / ...... |
| iPS:39 3064 | 21-225_33A9 | VH1\|1-08/D1\|1-1\|RF1/JH4 | ............ / ............ / ........ | ........... | ........ / ...... / .... | ........ / ........ / .... | ................ / ...G......... | KKA-- / ................... / ...-N.. | ...... / ...... |
| iPS:39 3148 | 21-225_35E5 | VH1\|1-08/D1\|1-1\|RF1/JH4 | ............ / ............ / ........ | ........... | ........ / ...... / .... | ........ / ........ / .... | ................ / ............ | KKS-- / ................... / ...-N.. | ...... / ...... |
| iPS:39 8530 | 21-225_32G4 | VH1\|1-08/D1\|1-1\|RF1/JH4 | ............ / ............ / ........ | ........... | ........ / ...... / .... | ........ / ........ / .... | ................ / ............ | KKA-- / ................... / ...-N.. | ...... / ...... |
| | | Germline | H_FR1 | H_CDR1 | H_FR2 | H_CDR2 | H_FR3 | H_CDR3 | H_FR4 |
| | VH3\|3-30.3/D1\|1-26\|RF3/JH6 | | QVQLVES-GGGVVQPGRSLR LSCAASG-FTFS | S-----YAMH | WVRQAPGK GLEWVA | VISYD---- GSNKYYAD SVKG | RFTISRDNSKNTLYLQM NSLRAEDTAVYYCAR | YSGSYYYY ------YYYGMDV | WGQGTT VTVSS |
| iPS:43 5565 | 21-225_159C4 | VH3\|3-30.3/D1\|1-26\|RF3/JH6 | ............ / ............ / ........ | N......G.. | ........ / ...... / .... | ....S... / .N...... / .... | ................ / ......M....... | R.S.WG-- / .................- / G..... | ..H... / ..... |
| iPS:39 3892 | 21-225_6G7 | VH3\|3-30.3/D1\|1-26\|RF3/JH6 | ............ / ............ / ........ | .......G.. | ....T... / ...... / .... | I...V... / .K...... / .... | ................ / ................ | RGN..G-- / .................- / G..... | ...... / ..... |
| | | Germline | H_FR1 | H_CDR1 | H_FR2 | H_CDR2 | H_FR3 | H_CDR3 | H_FR4 |
| | VH3\|3-23/D2\|2-21\|RF3/JH4 | | EVQLLES-GGGLVQPGGSLR LSCAASG-FTFS | S-----YAMS | WVRQAPGK GLEWVS | AISGS---- GGSTYYAD SVKG | RFTISRDNSKNTLYLQM NSLRAEDTAVYYCAK | HIVVVT---------- ------AIYFDY | WGQGTL VTVSS |
| iPS:43 5579 | 21-225_160G1 | VH3\|3-23/D2\|2-21\|RF3/JH4 | ............ / ............ / ........ | .......V.. | ........ / ...... / .... | .M...... / ..H..... / .... | ............V.... / .............V. | .G---- / ................... / ----YS | ...... / ..... |
| iPS:43 5585 | 21-225_160G3 | VH3\|3-23/D2\|2-21\|RF3/JH4 | ............ / ............ / ........ | .......V.. | ....T... / ...... / .... | .M...... / ..H..... / .... | ................ / .........I...V. | .G---- / ................... / ----YS | ...... / ..... |
| | | Germline | H_FR1 | H_CDR1 | H_FR2 | H_CDR2 | H_FR3 | H_CDR3 | H_FR4 |
| | VH4\|4-39/D1\|1-1\|RF3/JH5 | | QLQLQES-GPGLVKPSETLS LTCTVSG-GSIS | SS----SYYWG | WIRQPPGK GLEWIG | SIYY---- SGSTYYNP SLKS | RVTISVDTSKNQFSLKL SSVTAADTAVYYCAR | YNWND----------- -------NWFDP | WGQGTL VTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:43 5599 | 21-225_160B10 | VH4\|4-39/D1\|1-1\|RF3/JH5 | ...........<br>...........<br>....... | R......... | ....Y... | N.......<br>...A.HI.<br>.... | ................<br>N...........V. | HDP.W..............<br>.......-GV.Y | ......<br>..... |
| | | Germline | H_FR1 | H_CDR1 | H_FR2 | H_CDR2 | H_FR3 | H_CDR3 | H_FR4 |
| | **VH3\|3-33/D1\|1-1\|RF2/JH6** | | QVQLVES-GGGVVQPGRSLR LSCAASG-FTFS | S-----YGMH | WVRQAPGK GLEWVA | VIWYD---GSNKYYAD SVKG | RFTISRDNSKNTLYLQM NSLRAEDTAVYYCAR | VQLERYY----------YYYGMDV | WGQGTT VTVSS |
| iPS:43 5601 | 21-225_160G10 | VH3\|3-33/D1\|1-1\|RF2/JH6 | ...........<br>...........<br>....... | .......F... | ......... | .......<br>..Y.....<br>.... | ................ | .GI.VAGD...........<br>.......F..E. | ......<br>..... |
| iPS:43 5655 | 21-225_167E2 | VH3\|3-33/D1\|1-1\|RF2/JH6 | ...........<br>...........<br>....... | .......F... | ......... | .......<br>.TY.....<br>.... | ................ | .GI.VAGD...........<br>..........E. | ......<br>..... |
| iPS:43 5657 | 21-225_167H10 | VH3\|3-33/D1\|1-1\|RF2/JH6 | ...........<br>......Q.....<br>....... | .......F... | ......... | .......<br>..Y..H..<br>.... | ................ | .GI.VAGD...........<br>..........E. | ......<br>..... |
| | | Germline | H_FR1 | H_CDR1 | H_FR2 | H_CDR2 | H_FR3 | H_CDR3 | H_FR4 |
| | **VH3\|3-53/D7\|7-27\|RF3/JH4** | | EVQLVET-GGGLIQPGGSLR LSCAASG-FTVS | S-----NYMS | WVRQAPGK GLEWVS | VIYS----GGSTYYAD SVKG | RFTISRDNSKNTLYLQM NSLRAEDTAVYYCAR | NWGY-----------FDY | WGQGTL VTVSS |
| iPS:43 5605 | 21-225_161A4 | VH3\|3-53/D7\|7-27\|RF3/JH4 | ......S.....<br>...........<br>....... | .......... | ......... | ...T....<br>.....N..<br>.... | ................ | ...MA............<br>.......GP... | ......<br>..... |
| | | Germline | H_FR1 | H_CDR1 | H_FR2 | H_CDR2 | H_FR3 | H_CDR3 | H_FR4 |
| | **VH3\|3-30.3/D6\|6-6\|RF1/JH6** | | QVQLVES-GGGVVQPGRSLR LSCAASG-FTFS | S-----YAMH | WVRQAPGK GLEWVA | VISYD---GSNKYYAD SVKG | RFTISRDNSKNTLYLQM NSLRAEDTAVYYCAR | EYSSSSYY----------YYYGMDV | WGQGTT VTVSS |
| iPS:43 5607 | 21-225_161G4 | VH3\|3-30.3/D6\|6-6\|RF1/JH6 | ...........<br>...........<br>....... | .......G.. | ......... | ...G...<br>.....H..<br>.... | ................V. | RS...G--<br>................-<br>G..... | ......<br>..... |
| iPS:39 3020 | 21-225_30E2 | VH3\|3-30.3/D6\|6-6\|RF1/JH6 | ...........<br>...........<br>....... | .......G.. | ......... | ....G...<br>....F..V<br>.... | ..N.............V. | RGY..G--<br>................-<br>G..... | ......<br>..... |

| | | | H_FR1 | H_CDR1 | H_FR2 | H_CDR2 | H_FR3 | H_CDR3 | H_FR4 |
|---|---|---|---|---|---|---|---|---|---|
| iPS:39 3062 | 21-225_33H3 | VH3\|3-30.3/D6\|6-6\|RF1/JH6 | ............. ............. ........ | .......G.. | ...R.... ...... .... | I...G... ...NF... .... | ................. | RGY..G-- ...............⁻ G..... | ...... .... |
| iPS:39 3138 | 21-225_35E3 | VH3\|3-30.3/D6\|6-6\|RF1/JH6 | ............. ............. ........ | .......G.. | ......... ...... .... | ....G... ........ .... | ................. ..............V. | RGY..G-- ...............⁻ G..... | ...... .... |
| | | Germline | H_FR1 | H_CDR1 | H_FR2 | H_CDR2 | H_FR3 | H_CDR3 | H_FR4 |
| | VH3-30.3/D6\|6-13\|RF2/JH6 | | QVQLVES-GGGVVQPGRSLR LSCAASG-FTFS | S-----YAMH | WVRQAPGK GLEWVA | VISYD--- GSNKYYAD SVKG | RFTISRDNSKNTLYLQM NSLRAEDTAVYYCAR | GIAAAGYY---------- -----YYYGMDV | WGQGTT VTVSS |
| iPS:43 5611 | 21-225_161F10 | VH3\|3-30.3/D6\|6-13\|RF2/JH6 | ............. ............. ........ | .......G.. | ......... ...... .... | I...S... .R.DF... .... | ................. | R.....-- ...............⁻ H..... | ...... .... |
| | | Germline | H_FR1 | H_CDR1 | H_FR2 | H_CDR2 | H_FR3 | H_CDR3 | H_FR4 |
| | VH3\|3-33/D5\|5-18\|RF1/JH6 | | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | VDTAMVYY---------- -----YYYGMDV | WGQGTT VTVSS |
| iPS:43 5629 | 21-225_162H6 | VH3\|3-33/D5\|5-18\|RF1/JH6 | ............. ............. .......N | N........ | ......... ...... .... | ........ ........ .... | ................. | KGI.A.GD......... ........... | ...... .... |
| | | Germline | H_FR1 | H_CDR1 | H_FR2 | H_CDR2 | H_FR3 | H_CDR3 | H_FR4 |
| | VH3\|3-21/D7\|7-27\|RF1/JH6 | | EVQLVES-GGGLVKPGGSLR LSCAASG-FTFS | S-----YSMN | WVRQAPGK GLEWVS | SISSS--- SSYIYYAD SVKG | RFTISRDNAKNSLYLQM NSLRAEDTAVYYCAR | LTGYYY------------ ------YYCMDV | WGQGTT VTVSS |
| iPS:43 5639 | 21-225_163G6 | VH3\|3-21/D7\|7-27\|RF1/JH6 | ............. ............. ........ | .........S | ......... ...... .... | ...G.... .A...... .... | ................. | .S.--- ---... | ...... .... |
| | | Germline | H_FR1 | H_CDR1 | H_FR2 | H_CDR2 | H_FR3 | H_CDR3 | H_FR4 |
| | VH3\|3-21/D5\|5-24\|RF2/JH6 | | EVQLVES-GGGLVKPGGSLR LSCAASG-FTFS | S-----YSMN | WVRQAPGK GLEWVS | SISSS--- SSYIYYAD SVKG | RFTISRDNAKNSLYLQM NSLRAEDTAVYYCAR | *RWLQLYY---------- -----YYYGMDV | WGQGTT VTVSS |
| iPS:43 5643 | 21-225_163G10 | VH3\|3-21/D5\|5-24\|RF2/JH6 | ............. ............. ........ | .......... | ......... ...... .... | ...G.... .T...... .... | ................. | A.M----- ...............--- --.. | ...... .... |
| | | Germline | H_FR1 | H_CDR1 | H_FR2 | H_CDR2 | H_FR3 | H_CDR3 | H_FR4 |

3263

| VH3\|3-33/D3\|3-9\|RF2/JH4 | | QVQLVES-GGGVVQPGRSLR-LSCAASG-FTFS | S-----YGMH | WVRQAPGK GLEWVA | VIWYD---GSNKYYAD SVKG | RFTISRDNSKNTLYLQM NSLRAEDTAVYYCAR | YYDILTG----------YYNYFDY | WGQGTL VTVSS |
|---|---|---|---|---|---|---|---|---|
| iPS:43 5663 | 21-225_169B1 | VH3\|3-33/D3\|3-9\|RF2/JH4 | ............ ............ ........ | .......... | ........ ...... | ........ ..R. | ............... | DPLRGYN........... ......-DPVM.. | ...... ..... |
| iPS:43 5669 | 21-225_169F9 | VH3\|3-33/D3\|3-9\|RF2/JH4 | ............ ............ ........ | T......... | ........ .....S | I....... .T...... .... | ................L | DPLRGYN........... ......-DPVM.. | ...... ..... |
| iPS:43 5693 | 21-225_170G4 | VH3\|3-33/D3\|3-9\|RF2/JH4 | ............ ...........T ........ | T......... | ........ .....S | I....... .T...... .... | ..............F..L ..........M..... | DPLRGYN........... ......-DPVM.. | ...... ..... |
| iPS:43 5695 | 21-225_170D5 | VH3\|3-33/D3\|3-9\|RF2/JH4 | ............ ............ ........ | .......... | ........ ...... | I....... .T...... .... | ................L | DPLRGYN........... ......-DPVM.. | ...... ..... |
| iPS:43 5697 | 21-225_170G5 | VH3\|3-33/D3\|3-9\|RF2/JH4 | ............ ....G....... ........ | T......... | ........ .....T | I....... .T...... .... | ..........S...... ...........F... | DPLRGYN........... ......-DPVM.. | ...... ..... |
| iPS:43 5703 | 21-225_170D11 | VH3\|3-33/D3\|3-9\|RF2/JH4 | ............ ...........V ........ | .......... | ........ ...... | I....... .T...... .... | ................L | DPLRGYN........... ......-DPVM.. | ...... ..... |
| iPS:43 5705 | 21-225_171C3 | VH3\|3-33/D3\|3-9\|RF2/JH4 | ............ ....G....... ........ | T......... | ........ .....T | I....... .T...... .... | ................ | DPLRGYN........... ......-DPVM.. | ...... ..... |
| iPS:43 5709 | 21-225_171A4 | VH3\|3-33/D3\|3-9\|RF2/JH4 | ............ ....G....... ........ | T......... | ........ ...... | I....... ........ .... | ................ | DPLRGYN........... .......-DPVM.. | ...... ..... |
| iPS:43 5721 | 21-225_172B3 | VH3\|3-33/D3\|3-9\|RF2/JH4 | ............ .....P...... ........ | T......... | ........ ...... | ........ ........ .... | ................ | DPLRGYN........... ......-DPVM.. | ...... ..... |
| iPS:43 5725 | 21-225_172G8 | VH3\|3-33/D3\|3-9\|RF2/JH4 | ...M........ ............ ........ | T......... | ........ ....M. | I....... .T...... .... | ................L | DPLRGYN........... ......-DPVM.. | ...... ..... |
| iPS:43 5735 | 21-225_173H12 | VH3\|3-33/D3\|3-9\|RF2/JH4 | ............ ............ ........ | .......... | ........ ...... | I....... .T...... .... | ................L | DPLRGYN........... ......-DPVM.. | ...... ..... |

| | | | H_FR1 | H_CDR1 | H_FR2 | H_CDR2 | H_FR3 | H_CDR3 | H_FR4 |
|---|---|---|---|---|---|---|---|---|---|
| iPS:43 5743 | 21-225_175G1 | VH3\|3-33/D3\|3-9\|RF2/JH4 | ............ ....G....... ........ | T......... | ........ ...... | ........ .... | ...............V.. .............. | DPLRGYN.......... .....-DPVM.. | ...... ..... |
| iPS:43 5761 | 21-225_176B11 | VH3\|3-33/D3\|3-9\|RF2/JH4 | ............ ............ ........ | T......... | ........ .....S | I....... .T...... .... | ...............F..L .............. | DPLRGYN.......... .....-DPVL.. | ...... ..... |
| iPS:43 5779 | 21-225_178B10 | VH3\|3-33/D3\|3-9\|RF2/JH4 | ............ ..........V ......S. | T......... | ........ ....M. | I....... .T...... .... | ...............L .............. | DPLRGYN.......... .....-DPVM.. | ...... ..... |

| Germline | | | H_FR1 | H_CDR1 | H_FR2 | H_CDR2 | H_FR3 | H_CDR3 | H_FR4 |
|---|---|---|---|---|---|---|---|---|---|
| VH3\|3-48/D2\|2-15\|RF3/JH6 | | | EVQLVES- GGGLVQPGGSLR LSCAASG-FIFS | S-----YSMN | WVRQAPGK GLEWVS | YISSS--- SSTIYYAD SVKG | RFTISRDNAKNSLYLQM NSLRDEDTAVYYCAR | DIVVVAAT---------- ---YYYYYCMDV | WGQGTT VTVSS |
| iPS:43 5667 | 21-225_169E3 | VH3\|3-48/D2\|2-15\|RF3/JH6 | ............ ............ ........ | G.....H... | ........ .....A | ...L.... G...K... .... | .........RD...... .............. | RGIT..R-- ..............-- NED.L.. | ...... ..... |
| iPS:43 5673 | 21-225_169E6 | VH3\|3-48/D2\|2-15\|RF3/JH6 | ............ ............ ........ | G.....H... | ........ .....A | ...I.... ....K... .... | .........RD...... .............. | RGIT..R-- ..............-- NED.L.. | ...... ..... |
| iPS:43 5759 | 21-225_176E6 | VH3\|3-48/D2\|2-15\|RF3/JH6 | ............ ............ ........ | G.....H... | ........ .....A | ...I.... G...K... .... | ..I......RD...... .............. | RGIT..R-- ..............-- NED.L.. | ...... ..... |

| Germline | | | H_FR1 | H_CDR1 | H_FR2 | H_CDR2 | H_FR3 | H_CDR3 | H_FR4 |
|---|---|---|---|---|---|---|---|---|---|
| VH4\|4-59\|3-9\|RF1/JH6 | | | QVQLQES- GPGLVKPSETLS LTCTVSG-GSIS | S-----YYWS | WIRQPPGK GLEWIG | YIYY---- SGSTNYNP SLKS | RVTISVDTSKNQFSLKL SSVTAADTAVYYCAR | VLRYFDWLL*-------- ---YYYYYGMDV | WGQGTT VTVSS |
| iPS:43 5675 | 21-225_169D7 | VH4\|4-59/D3\|3-9\|RF1/JH6 | ............ ............ ........ | ..........T | ......A.. ...... | R..T.... .... | ...M.............. ..........F..K | .G..Y----- ..............---- ..... | ...... ..... |
| iPS:43 5687 | 21-225_170H1 | VH4\|4-59/D3\|3-9\|RF1/JH6 | ............ ............ ........ | .......... | .....A.. ...... | R..T.... .... | ...M.............. .............. | .G..Y----- ..............---- ..... | ...... ..... |

| Germline | | | H_FR1 | H_CDR1 | H_FR2 | H_CDR2 | H_FR3 | H_CDR3 | H_FR4 |
|---|---|---|---|---|---|---|---|---|---|
| VH3\|3-23/D4\|4-23\|RF2/JH4 | | | EVQLLES- GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | DYGGN------------- -------SYFDY | WGQGTL VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 5711 | 21-225_171G4 | VH3\|3-23/D4\|4-23\|RF2/JH4 | ..........D.<br>............<br>........ | ......C..T | ........<br>...... | ....R...<br>..T.F...<br>..R. | ..............F...<br>................ | .LI.GA............<br>.......T.... | ......<br>..... |
| iPS:43 5875 | 21-225_190B9 | VH3\|3-23/D4\|4-23\|RF2/JH4 | ............<br>............<br>........ | T......... | ........<br>...... | ...R....<br>..N.H...<br>.... | ................<br>..........S... | .GF.GS............<br>............ | ......<br>..... |
| iPS:43 5909 | 21-225_190H3 | VH3\|3-23/D4\|4-23\|RF2/JH4 | ............<br>............<br>........ | ......... | ........<br>...... | ....R...<br>..N.....<br>.... | ................<br>................ | .GF.GS............<br>............ | ......<br>..... |
| iPS:43 6013 | 21-225_193F2 | VH3\|3-23/D4\|4-23\|RF2/JH4 | ............<br>............<br>........ | T.....F... | ........<br>...... | ...R....<br>..N.H...<br>.... | ................<br>................ | .GF.GS............<br>............ | ......<br>..... |
| iPS:43 6100 | 21-225_195G12 | VH3\|3-23/D4\|4-23\|RF2/JH4 | ............<br>............<br>........ | T......... | ........<br>...... | ...R....<br>..N.H...<br>.... | ................<br>..........S... | .GF.GS............<br>............ | ......<br>..... |
| | | Germline | H_FR1 | H_CDR1 | H_FR2 | H_CDR2 | H_FR3 | H_CDR3 | H_FR4 |
| | VH3\|3-30.3/D1\|1-7\|RF2/JH6 | | QVQLVES-GGGVVQPGRSLR LSCAASG-FIFS | S-----YAMH | WVRQAPGK GLEWVA | VISYD---GSNKYYAD SVKG | RFTISRDNSKNTLYLQM NSLRAEDTAVYYCAR | V*LELYY----------- -----YYYGMDV | WGQGTT VTVSS |
| iPS:43 5713 | 21-225_171D7 | VH3\|3-30.3/D1\|1-7\|RF2/JH6 | ........R...<br>............<br>........ | .......G.. | ........<br>...... | ........<br>.N.RH...<br>..Q. | ...............S...<br>...G.......... | DRHR.D-<br>.................-<br>..AL.. | ......<br>..... |
| | | Germline | H_FR1 | H_CDR1 | H_FR2 | H_CDR2 | H_FR3 | H_CDR3 | H_FR4 |
| | VH3\|3-23/D5\|5-24\|RF3/JH3 | | EVQLLES-GGGLVQPGGSLR LSCAASG-FIFS | S-----YAMS | WVRQAPGK GLEWVS | AISGS---GGSTYYAD SVKG | RFTISRDNSKNTLYLQM NSLRAEDTAVYYCAK | RDGYNY----------- -------DAFDI | WGQGTM VTVSS |
| iPS:43 5729 | 21-225_173E7 | VH3\|3-23/D5\|5-24\|RF3/JH3 | ...........S<br>............<br>........ | ......... | ........<br>...... | F.......<br>..N.F...<br>.... | ................<br>...Q.........T. | ..T..G............<br>......W..... | ......<br>..... |
| iPS:43 5753 | 21-225_175G10 | VH3\|3-23/D5\|5-24\|RF3/JH3 | ............<br>............<br>........ | ......... | ....T...<br>...... | I.......<br>..N.....<br>.... | ................<br>................ | ..TW.G............<br>......W..... | ......<br>....L |
| iPS:39 3024 | 21-225_31H9 | VH3\|3-23/D5\|5-24\|RF3/JH3 | ............<br>............<br>........ | ......C..N | ........<br>...... | ........<br>...SF...<br>.... | ................<br>................ | .TP.D-<br>..-V... | ......<br>..... |

| | | | H_FR1 | H_CDR1 | H_FR2 | H_CDR2 | H_FR3 | H_CDR3 | H_FR4 |
|---|---|---|---|---|---|---|---|---|---|
| iPS:39 8474 | 21-225_17B10 | VH3\|3-23\|D5\|5-24\|RF3/JH3 | .............<br>.............<br>.......R | .......... | ...... | V.......<br>..N..F..<br>.... | ................<br>D............. | .GIPEA...........<br>............ | ......<br>..... |
| | | Germline | H_FR1 | H_CDR1 | H_FR2 | H_CDR2 | H_FR3 | H_CDR3 | H_FR4 |
| | VH1\|1-02\|D1\|1-1\|RF1/JH3 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | GTTGT------------ -------DAFDI | WGQGTM VTVSS |
| iPS:43 5745 | 21-225_175G3 | VH1\|1-02\|D1\|1-1\|RF1/JH3 | ...V........<br>........ | .......F.. | .........<br>...... | ..K.K...<br>.....C..<br>R... | ..........T.<br>..............V. | .G.TVTT............<br>......WGV..Y | ......<br>..... |
| iPS:43 7270 | 21-225_178H4 | VH1\|1-02\|D1\|1-1\|RF1/JH3 | .............<br>........ | .......F.. | .........<br>...... | ..K.K...<br>.....C..<br>.... | ................<br>..............V. | .G.TVTT...........<br>......WGV..Y | ......<br>..... |
| | | Germline | H_FR1 | H_CDR1 | H_FR2 | H_CDR2 | H_FR3 | H_CDR3 | H_FR4 |
| | VH3\|3-23\|D3\|3-22\|RF2/JH4 | | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | YYYDSSG----------- -----YYYYFDY | WGQGTL VTVSS |
| iPS:43 5769 | 21-225_177B6 | VH3\|3-23\|D3\|3-22\|RF2/JH4 | ..........<br>......R....E<br>........ | .......... | .........<br>...... | V.......<br>.SN...V.<br>.... | ...............N...<br>.......S.....T. | G.................<br>........P..F | ......<br>..... |
| | | Germline | H_FR1 | H_CDR1 | H_FR2 | H_CDR2 | H_FR3 | H_CDR3 | H_FR4 |
| | VH3\|3-33\|D3\|3-22\|RF2/JH1 | | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | YYYDSSGY---------- ----YYAEYFQH | WGQGTL VTVSS |
| iPS:43 5771 | 21-225_177B11 | VH3\|3-33\|D3\|3-22\|RF2/JH1 | ..........<br>.........T..<br>........ | .......... | .........<br>...... | I.......<br>..Y...T.<br>.... | ................<br>............... | ET..FW--<br>...............--<br>SG..VF | ......<br>..... |
| | | Germline | H_FR1 | H_CDR1 | H_FR2 | H_CDR2 | H_FR3 | H_CDR3 | H_FR4 |
| | VH3\|3-23\|D5\|5-24\|RF3/JH4 | | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | RDGYN------------ -------YYFDY | WGQGTL VTVSS |
| iPS:43 5775 | 21-225_178A5 | VH3\|3-23\|D5\|5-24\|RF3/JH4 | ..H........<br>..T........<br>........ | .........T | .........<br>...... | V.......<br>..N.F...<br>.... | ................<br>..............R | ...D-<br>..-.... | ......<br>..... |
| iPS:43 7214 | 21-225_48B12 | VH3\|3-23\|D5\|5-24\|RF3/JH4 | .............<br>.............<br>........ | .........N | .........<br>...... | ....R...<br>..N.F...<br>.... | ................<br>............... | .ET..WN...........<br>......YEG... | ......<br>..... |

| | | | H_FR1 | H_CDR1 | H_FR2 | H_CDR2 | H_FR3 | H_CDR3 | H_FR4 |
|---|---|---|---|---|---|---|---|---|---|
| IPS:39 3028 | 21-225_25D7 | VH3\|3-23/D5\|5-24\|RF3/JH4 | .............<br>.............<br>......... | .......... | ....T..Q<br>......<br> | ....R...<br>..T.F...<br>.... | ................<br>................ | DGYGGN............<br>.......ST... | ......<br>..... |
| | | Germline | H_FR1 | H_CDR1 | H_FR2 | H_CDR2 | H_FR3 | H_CDR3 | H_FR4 |
| | VH3\|3-33/D4\|4-11\|RF3/JH6 | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | TTVCYYY------------------YYGMDV | WGQGTTVTVSS |
| IPS:43 5789 | 21-225_180C4 | VH3\|3-33/D4\|4-11\|RF3/JH6 | .............<br>.............<br>......... | A......... | .........<br>.....T<br>.... | I.......<br>..Y.....<br>.... | ..A..............<br>................ | .G.DPWD...........<br>.....Y.N.... | ......<br>..... |
| | | Germline | H_FR1 | H_CDR1 | H_FR2 | H_CDR2 | H_FR3 | H_CDR3 | H_FR4 |
| | VH3\|3-23/D2\|2-8\|RF1/JH3 | | EVQLLES-GGGLVQPGGSLRLSCAASG-FIFS | S-----YAMS | WVRQAPGKGLEWVS | AISGS---GGSTYYADSVKG | RFTISRDNSKNTLYLQMNSLRAEDTAVYYCAK | RILY*WCM------------LYDAFDI | WGQGTMVTVSS |
| IPS:43 5799 | 21-225_181G3 | VH3\|3-23/D2\|2-8\|RF1/JH3 | .............<br>.............<br>......... | .......... | .........<br>......<br>.... | V.......<br>..N.F.G.<br>.... | ................<br>.........I..... | .ET.D.G-<br>................-<br>S..... | ......<br>..... |
| | | Germline | H_FR1 | H_CDR1 | H_FR2 | H_CDR2 | H_FR3 | H_CDR3 | H_FR4 |
| | VH3\|3-30.3/D5\|5-18\|RF2/JH6 | | QVQLVES-GGGVVQPGRSLRLSCAASG-FIFS | S-----YAMH | WVRQAPGKGLEWVA | VISYD---GSNKYYADSVKG | RFTISRDNSKNTLYLQMNSLRAEDTAVYYCAR | WIQLWLYY------------YYYGMDV | WGQGTTVTVSS |
| IPS:43 5811 | 21-225_183H6 | VH3\|3-30.3/D5\|5-18\|RF2/JH6 | .............<br>.............<br>......... | .......G.. | .........<br>......<br>.... | I...A...<br>..T.F...<br>.... | ................<br>..............V. | RPPQ..V-<br>................EG.<br>.... | ......<br>..... |
| IPS:43 6754 | 21-225_155G3 | VH3\|3-30.3/D5\|5-18\|RF2/JH6 | .............<br>..........T<br>......... | .......G.. | .........<br>......<br>.... | ........<br>......<br>.... | ................<br>................ | DTER..P-<br>................S.<br>.... | ......<br>..... |
| IPS:44 8908 | 21-225_50G9 | VH3\|3-30.3/D5\|5-18\|RF2/JH6 | .............<br>.............<br>.....I.. | .......G.. | .........<br>......<br>.... | ...Q....<br>.IIR....<br>.... | ................<br>................ | DVKQ..V-<br>................RT.<br>.... | ......<br>..... |
| | | Germline | H_FR1 | H_CDR1 | H_FR2 | H_CDR2 | H_FR3 | H_CDR3 | H_FR4 |
| | VH3\|3-30.3/D6\|6-19\|RF1/JH5 | | QVQLVES-GGGVVQPGRSLRLSCAASG-FIFS | S-----YAMH | WVRQAPGKGLEWVA | VISYD---GSNKYYADSVKG | RFTISRDNSKNTLYLQMNSLRAEDTAVYYCAR | GYSSGW------------YNWFDP | WGQGTLVTVSS |

| ID | Clone | Germline / VH | H_FR1 | H_CDR1 | H_FR2 | H_CDR2 | H_FR3 | H_CDR3 | H_FR4 |
|---|---|---|---|---|---|---|---|---|---|
| iPS:43 5813 | 21-225_183A12 | VH3\|3-30.3/D6\|6-19\|RF1/JH5 | ............ ............ ........ | .......G.. | ........ ...... | ...SA... ........ .... | ................ | R................ .......-D.... | ...... ..... |
| | | **Germline** | **H_FR1** | **H_CDR1** | **H_FR2** | **H_CDR2** | **H_FR3** | **H_CDR3** | **H_FR4** |
| | **VH3\|3-21/D3\|3-10\|RF3/JH4** | | EVQLVES-GGGLVKPGGSLRLSCAASG-FTFS | S-----YSMN | WVRQAPGK GLEWVS | SISSS---SSYIYYAD SVKG | RFTISRDNAKNSLYLQM NSLRAEDTAVYYCAR | ITMVRGV-----------IIYFDY | WGQGTL VTVSS |
| iPS:43 5815 | 21-225_190G10 | VH3\|3-21/D3\|3-10\|RF3/JH4 | ............ ............ .......R | D......... | ........ ...... | ....G... .G..H... .... | ................ | A..A--- ................-  --L.. | ...... |
| iPS:43 5865 | 21-225_191A5 | VH3\|3-21/D3\|3-10\|RF3/JH4 | .I.V........ ............ .......R | D......... | ........ ...... | ....G... .G..... .... | ................ | A..A--- ................-  --L.. | ....A. |
| iPS:43 6047 | 21-225_193B10 | VH3\|3-21/D3\|3-10\|RF3/JH4 | ............ ............ .......R | D......... | ........ ...... | ....A... GG..... .L.. | ................ | A..A--- ................-  --L.. | ...... |
| iPS:43 6122 | 21-225_196G10 | VH3\|3-21/D3\|3-10\|RF3/JH4 | ............ ............ .......R | D......... | ........ ...... | ....G... .G..H... .... | ................ | A..A--- ................-  --L.. | ...... |
| | | **Germline** | **H_FR1** | **H_CDR1** | **H_FR2** | **H_CDR2** | **H_FR3** | **H_CDR3** | **H_FR4** |
| | **VH4\|4-59/D4\|4-17\|RF2/JH6** | | QVQLQES-GPGLVKPSETLSLTCTVSG-GSIS | S-----YYWS | WIRQPPGK GLEWIG | YIYY----SGSTNYNP SLKS | RVTISVDTSKNQFSLKL SSVTAADTAVYYCAR | DYGDYYY-------------YYYGMDV | WGQGTT VTVSS |
| iPS:43 5817 | 21-225_190B11 | VH4\|4-59/D4\|4-17\|RF2/JH6 | ............ ............ .......N | N......... | .....A.. ...... | R..T.... ........ .... | ...M............ ..........H.... | .R.Y.G- ................-  ...... | ...... |
| iPS:43 5917 | 21-225_190D5 | VH4\|4-59/D4\|4-17\|RF2/JH6 | ............ ............ .......N | N......... | .....A.. ...... | R..A.... ........ .... | ...M.I............ | .R.Y.G- ................-  ...... | ..I.. |
| iPS:43 6056 | 21-225_194C3 | VH4\|4-59/D4\|4-17\|RF2/JH6 | ............ ............ .......N | N......... | .....A.. ...... | R..A.... ........ .... | ...M.I............ ..........H.... | .R.Y.G- ................-  ...... | ..I.. |
| iPS:43 6220 | 21-225_200F8 | VH4\|4-59/D4\|4-17\|RF2/JH6 | ............ ............ .......N | N......... | .....A.. ...... | R..T.... ........ .... | ...M............ | .R.Y.G- ................-  ...... | ...... |
| | | **Germline** | **H_FR1** | **H_CDR1** | **H_FR2** | **H_CDR2** | **H_FR3** | **H_CDR3** | **H_FR4** |

| | Germline | H_FR1 | H_CDR1 | H_FR2 | H_CDR2 | H_FR3 | H_CDR3 | H_FR4 |
|---|---|---|---|---|---|---|---|---|
| | VH3\|3-30\|D4\|4-17\|RF2/JH6 | QVQLVES-GGGVVQPGRSLR LSCAASG-FIFS | S-----YGMH | WVRQAPGK GLEWVA | VISYD--- GSNKYYAD SVKG | RFTISRDNSKNTLYLQM NSLRAEDTAVYYCAK | DYGDYYY------------ -----YYYGMDV | WGQGTT VTVSS |
| iPS:43 5821 | 21-225_190E11 / VH3\|3-30\|D4\|4-17\|RF2/JH6 | ............ ...........T ........ | N......... | ........ ...... | I.WF.... ........ .... | .........N....... | AQ.V..- .................-- .V... | ...... |
| | Germline | H_FR1 | H_CDR1 | H_FR2 | H_CDR2 | H_FR3 | H_CDR3 | H_FR4 |
| | VH3\|3-23\|D5\|5-12\|RF3/JH5 | EVQLLES-GGGLVQPGGSLR LSCAASG-FIFS | S----- YAMS | WVRQAPGK GLEWVS | AISGS--- GGSTYYAD SVKG | RFTISRDNSKNTLYLQM NSLRAEDTAVYYCAK | GYSGYD ------YNWFDP | WGQGTL VTVSS |
| iPS:43 5823 | 21-225_190F11 / VH3\|3-23\|D5\|5-12\|RF3/JH5 | .....D...... G........... .......D | .........N | ........ ...... | T...T.... .RR..... .... | ................. | EEDY..S........... .....SGPG... | ...... |
| iPS:43 5867 | 21-225_191E5 / VH3\|3-23\|D5\|5-12\|RF3/JH5 | .....D...... G........... .......D | .........N | ........ ...... | T...T.... .RR..... .... | ................. | EEDY..S........... .....SGPG... | ...... |
| iPS:43 5929 | 21-225_190D9 / VH3\|3-23\|D5\|5-12\|RF3/JH5 | .....D...... G........... .......D | .......... | ........ ...... | T...T.... .RR..... .... | ................. | EEDY..S........... .....SGPG... | ...... |
| iPS:43 5935 | 21-225_190H8 / VH3\|3-23\|D5\|5-12\|RF3/JH5 | G....D...... G........... .......D | .......... | ........ ...... | T...T.... .RR..... .... | ................. | EEDY..S........... .....SGPG... | ...... |
| | Germline | H_FR1 | H_CDR1 | H_FR2 | H_CDR2 | H_FR3 | H_CDR3 | H_FR4 |
| | VH4\|4-39\|D3\|3-9\|RF1/JH4 | QLQLQES-GPGLVKPSETLS LTCIVSG-GSIS | SS----SYYWG | WIRQPPGK GLEWIG | SIYY---- SGSTYYNP SLKS | RVTISVDTSKNQFSLKL SSVTAADTAVYYCAR | VLRYFDW------------ ----LL*YFDY | WGQGTL VTVSS |
| iPS:43 5827 | 21-225_190H11 / VH4\|4-39\|D3\|3-9\|RF1/JH4 | ............ ............ .......R | .-...-.H.S | .....A.. ...... | L..T.... .R..I... .... | ...L............. | LRYNWN- ...................- FP.... | ...... |
| iPS:43 5853 | 21-225_191E3 / VH4\|4-39\|D3\|3-9\|RF1/JH4 | ............ ............ .......R | .-...-.H.S | .....A.. ...... | L..T.... .R..N... .... | ...M............ N............. | LRYNWN- ...................- FP.... | ...... |
| iPS:43 5871 | 21-225_191E6 / VH4\|4-39\|D3\|3-9\|RF1/JH4 | ............ ............ .......R | .-...-.H.S | .....A.. ...... | L..T.... .R..N... .... | ...M............ ................. | LRYNWN- ...................- FP...F | ...... |
| iPS:43 5927 | 21-225_190E7 / VH4\|4-39\|D3\|3-9\|RF1/JH4 | ............ ............ .......R | .-...-.H.S | .....A.. ...... | H..T.... .R..N... .... | ................. .....T......... | LRYNWN- ...................- FP.... | ...... |

| | | | H_FR1 | H_CDR1 | H_FR2 | H_CDR2 | H_FR3 | H_CDR3 | H_FR4 |
|---|---|---|---|---|---|---|---|---|---|
| iPS:43 5999 | 21-225_192F9 | VH4\|4-39/D3\|3-9\|RF1/JH4 | ............ ............ .......R | .-...-.H.S | .....A.. ...... | L..T.... .R..N... .... | ...M........... N............ | LRYNWN- ................- FP.... | ...... ..... |
| iPS:43 6060 | 21-225_194F4 | VH4\|4-39/D3\|3-9\|RF1/JH4 | ............ ............ ....... | .-...-.H.S | .....A.. ...... | L..T.... .R..N... .... | ...M...R..S...... ................ | LRYNWN- ................- FP.... | ...... ..... |
| iPS:43 6193 | 21-225_198A10 | VH4\|4-39/D3\|3-9\|RF1/JH4 | ............ ............ .......R | .-...-.H.S | .....A.. ...... | H..T.... .R..N... .... | ................ ......T........ | LRYNWN- ................- FP.... | ...... ..... |
| | **Germline** | | **H_FR1** | **H_CDR1** | **H_FR2** | **H_CDR2** | **H_FR3** | **H_CDR3** | **H_FR4** |
| **VH4\|4-30.1/D5\|5-24\|RF3/JH2** | | | QVQLQES- GPGLVKPSQILS LTCTVSG-GSIS | SG---GYYWS | WIRQHPGK GLEWIG | YIYY---- SGSTYYNP SLKS | RVTISVDTSKNQFSLKL SSVTAADTAVYYCAR | RDGYNY----------- ------YWYFDL | WGRGTL VTVSS |
| iPS:43 5829 | 21-225_190B12 | VH4\|4-30.1/D5\|5-24\|RF3/JH2 | ............ ............ ....... | .........N | ...... .... | ........ .... | .............F... N............. | SGYNWD........... ......-AGV.P | ..... |
| | **Germline** | | **H_FR1** | **H_CDR1** | **H_FR2** | **H_CDR2** | **H_FR3** | **H_CDR3** | **H_FR4** |
| **VH3\|3-33/D4\|4-11\|RF2/JH6** | | | QVQLVES- GGGVVQPGRSLR LSCAASG-FTFS | S----YGMH | WVRQAPGK GLEWVA | VIWYD--- GSNKYYAD SVKG | RFTISRDNSKNTLYLQM NSLRAEDTAVYYCAR | DYSNYYY---------- ------YYYGMDV | WGQGTT VTVSS |
| iPS:43 5835 | 21-225_190F12 | VH3\|3-33/D4\|4-11\|RF2/JH6 | ............ ............ .......R | N......... | ........ ...... | ...F.... ...D.... .... | ..............S... | .R.VG.- .................-- D.L.. | ...... ..... |
| iPS:43 5861 | 21-225_190A5 | VH3\|3-33/D4\|4-11\|RF2/JH6 | ............ G........... ....... | .......... | ........ ...... | ........ ....N... .... | ................ | .F.VG.- .................-- D.... | ...... ..... |
| iPS:43 5937 | 21-225_190H9 | VH3\|3-33/D4\|4-11\|RF2/JH6 | ............ ............ .......R | N......... | ........ ...... | ...F.... ...D.... .... | ................ | .R.VG.- .................-- D.L.. | ...... ..... |
| iPS:43 5977 | 21-225_192E4 | VH3\|3-33/D4\|4-11\|RF2/JH6 | ............ ............ ....... | .......... | ........ ...... | ....N.V. ..R. | ................ | .R.VG.- .................-- D.... | ...... ..... |
| iPS:43 6001 | 21-225_192C10 | VH3\|3-33/D4\|4-11\|RF2/JH6 | ............ ............ S......R | N......... | ........ ..K... | ...F.... ...D.... .... | ..............S... | .R.VG.- .................-- D.L.. | ...... ..... |

| | | | | | | | | | |
|---|---|---|---|---|---|---|---|---|---|
| iPS:43 6066 | 21-225_194B7 | VH3\|3-33/D4\|4-11\|RF2/J H6 | . . . . . . . . . . . .<br>. . . . . . . . . . .<br>. . . . . . . . | . . . . . . . . . . | . . . . . . .<br>. . . . . . | . . . . . . .<br>. . . . N . . . .<br>. . . . | . . . . . . . . . . . . . . . . . | .R.KG.—<br>. . . . . . . . . . . . . . . . . . . —<br>D . . . . | . . . . . .<br>. . . . |
| iPS:43 6078 | 21-225_194H12 | VH3\|3-33/D4\|4-11\|RF2/J H6 | . . . . . . . . . . . .<br>. . . . . . . . . . .<br>. . . . . . . R | N . . . . . . . . . | . . . . . . .<br>. . . . . . | . . . F . . . .<br>. . . D . . . .<br>. . . . | . . . . . . . . . . . . . . S . . . | .R.VG.—<br>. . . . . . . . . . . . . . . . . . . —<br>D . L . . | . . . . . .<br>. . . . |
| iPS:43 6140 | 21-225_197G3 | VH3\|3-33/D4\|4-11\|RF2/J H6 | . . . . . . . . . . . .<br>. . . . . . . . . . V<br>. . . . . . . | . . . . . . H . . . | . . . . . . .<br>. . . . . . | . . . . . . .<br>. . . . N . . .<br>. . . . | . . . . . . . . . . . . . . . . . | .P.VG.—<br>. . . . . . . . . . . . . . . . . . . —<br>D . . . . | . . . . . .<br>. . . . |
| iPS:43 6167 | 21-225_197E11 | VH3\|3-33/D4\|4-11\|RF2/J H6 | . . . . . . . . . . . .<br>. . . . . . . . . . .<br>. . . . . . . R | N . . . . . . . . . | . . . . . . .<br>. . . . . . | . . . F . . . .<br>. . . D . . . .<br>. . . . | . . . . . . . . . . . . . . S . . . | .R.VG.—<br>. . . . . . . . . . . . . . . . . . . —<br>D . L . . | . . . . . .<br>. . . . |
| iPS:43 6292 | 21-225_205H3 | VH3\|3-33/D4\|4-11\|RF2/J H6 | . . . . . . . . . . . .<br>. . . . . . . . . . .<br>. . . . . . . | . . . . . . . . . . | . . . . . . .<br>. . . . . . | . . . . . . .<br>. . . . | | .R.VG.—<br>. . . . . . . . . . . . . . . . . . . —<br>D . T . . | . . . . . .<br>. . . . |
| iPS:43 6802 | 21-225_171E12 | VH3\|3-33/D4\|4-11\|RF2/J H6 | . . . . . . . . . . . .<br>. . . . . . . . . . .<br>. . . . . . . | . . . . . . . . . . | . . . . . . .<br>. . . . . . | . . . N . . . .<br>. G . . . NG.<br>. . . . | . . . . . . . . . . . . . . . . . | .RTY.SGSGSP . . . . . . .<br>. . PYY . . . . . . . | . . . . . .<br>. . . . . |
| iPS:43 6816 | 21-225_179H5 | VH3\|3-33/D4\|4-11\|RF2/J H6 | . . . . . . . . . . . .<br>. . . . . . . . . . .<br>. . . . . . . | . . . . . . . . . . | . . . . . . .<br>. . . . . . | . . . . . . .<br>. . . E . . . .<br>. . . . | . . . . . . . . . . . . . . . . . | .IR . . . —<br>. . . . . . . . . . . . . . . . . . . —<br>. . L . . | . . . . . .<br>. . . . . |
| iPS:43 6960 | 21-225_198D2 | VH3\|3-33/D4\|4-11\|RF2/J H6 | . . . . . . . . . . . .<br>. . . . . . . . . . .<br>. . . . . . . R | . . . . . . . . . . | . . . . . . .<br>. . . . . . | . . I . . . . .<br>. . Y . . . . .<br>. . . . | . . . . . . . . . . . . . . . . . | T . . G — — —<br>. . . . . . . . . . . . . . . . . . . —<br>— . . . . | . . . . . .<br>. . . . . |
| iPS:43 6974 | 21-225_190H7 | VH3\|3-33/D4\|4-11\|RF2/J H6 | . . . . . . . . . . . .<br>. . . . . . . . . . .<br>. . . . . N . R | . . . . . . . . . . | . . . . . . .<br>. . . . . . | . . I . . . . .<br>. . Y . . . . .<br>. . . . | . . . . . . . . . . . . . . . . . | T . . G — — —<br>. . . . . . . . . . . . . . . . . . . —<br>— . . . . | . . . . . .<br>. . . . . |
| iPS:43 6982 | 21-225_190D10 | VH3\|3-33/D4\|4-11\|RF2/J H6 | . . . . . . . . . . . .<br>. . . . . . . . . . .<br>. . . . . . . R | . . . . . . . . . . | . . . . . . .<br>. . . . . . | . . I . . . . .<br>. . Y . . . . .<br>. . . . | . . . . . . . . . . . . . . . . . | T . . G — — —<br>. . . . . . . . . . . . . . . . . . . —<br>— . . . . | . . . . . .<br>. . . . . |
| iPS:43 7274 | 21-225_196D4 | VH3\|3-33/D4\|4-11\|RF2/J H6 | . . . V . . . . . . . .<br>. . . . . . . . . . .<br>. . . . . . . | . . . . . . . . . . | . . . . . . .<br>. . . . . . | . . . . . . .<br>. . . RN . . .<br>. . . . | . . . . V . . . . . . . . . . | .R.KG.—<br>. . . . . . . . . . . . . . . . . . . —<br>D . . . . | . . . . . .<br>. . . . . |
| iPS:39 2664 | 21-225_20F6 | VH3\|3-33/D4\|4-11\|RF2/J H6 | . . . . . . . . . . . .<br>. . . . . . . . . . V<br>. . . . . . . | . . . . . . . . . . | . . . . . . .<br>. . . . G . | . . . H . . . .<br>. . . . | . . . . . . . A . . . . . . . . | .L.MG——<br>. . . . . . . . . . . . . . . . . . . —<br>— . . . . | . . . . . .<br>. . . . . |

| | | | | | | | | | |
|---|---|---|---|---|---|---|---|---|---|
| iPS:39 2738 | 21-225_18G4 | VH3\|3-33/D4\|4-11\|RF2/JH6 | ............<br>............<br>........ | .........<br><br> | ........<br>......<br>.... | I.......<br>........<br>.... | .................<br>................. | .L.MG--<br>..................--<br>-.... | ......<br>..... |
| iPS:39 2798 | 21-225_22C7 | VH3\|3-33/D4\|4-11\|RF2/JH6 | ............<br>............<br>........ | .........<br><br> | ........<br>....G.<br>.... | ...H....<br>........<br>.... | ........A........<br>................. | .L.MG--<br>..................--<br>-.... | ......<br>..... |
| iPS:39 2956 | 21-225_27A11 | VH3\|3-33/D4\|4-11\|RF2/JH6 | ............<br>............<br>........ | .........<br><br> | ........<br>......<br>.... | ........<br>........<br>.... | .................<br>...........H... | .S.P.--<br>..................--<br>-.... | ......<br>..... |
| iPS:39 2994 | 21-225_26G11 | VH3\|3-33/D4\|4-11\|RF2/JH6 | ............<br>............<br>........ | .........<br><br> | ........<br>......<br>.... | ........<br>........<br>.... | .................<br>................. | R...SW-<br>..................-<br>SG.... | ......<br>..... |
| iPS:39 3014 | 21-225_26D12 | VH3\|3-33/D4\|4-11\|RF2/JH6 | ............<br>............<br>........ | .........<br><br> | ........<br>......<br>.... | ...E....<br>........<br>.... | ......A........<br>................. | .S.P.--<br>..................--<br>-.... | ......<br>..... |
| iPS:39 3152 | 21-225_25B3 | VH3\|3-33/D4\|4-11\|RF2/JH6 | ............<br>............<br>........ | .........<br><br> | ........<br>......<br>.... | ........<br>........<br>.... | .................<br>................. | .S.P.--<br>..................--<br>-.... | ......<br>..... |
| iPS:39 3840 | 21-225_3F8 | VH3\|3-33/D4\|4-11\|RF2/JH6 | ............<br>............<br>........ | .........<br><br> | ........<br>....G.<br>.... | ...H....<br>........<br>.... | ........A........<br>................. | .L.MG--<br>..................--<br>-.... | ......<br>..... |
| iPS:39 3930 | 21-225_7E11 | VH3\|3-33/D4\|4-11\|RF2/JH6 | ............<br>............<br>........ | ......F...<br><br> | ........<br>......<br>.... | I..H....<br>........<br>.... | .........N.......<br>S................. | .L.MG--<br>..................--<br>-.... | ......<br>..... |
| iPS:39 3964 | 21-225_6G1 | VH3\|3-33/D4\|4-11\|RF2/JH6 | ............<br>............<br>.....I.. | .........<br><br> | ........<br>......<br>.... | I.......<br>........<br>.... | .................<br>...........M..... | .L.MG--<br>..................--<br>-.... | ......<br>..... |
| iPS:39 4012 | 21-225_15A3 | VH3\|3-33/D4\|4-11\|RF2/JH6 | ............<br>............<br>........ | ........I.<br><br> | ........<br>......<br>.... | ...H....<br>........<br>.... | .................<br>................. | .L.MG--<br>..................--<br>-.... | ......<br>..... |
| iPS:39 4016 | 21-225_13D4 | VH3\|3-33/D4\|4-11\|RF2/JH6 | ............<br>............<br>........ | .........<br><br> | ........<br>......<br>.... | ...H....<br>........<br>.... | .................<br>................. | .L.MG--<br>..................--<br>-.... | ......<br>..... |
| iPS:39 4083 | 21-225_16E6 | VH3\|3-33/D4\|4-11\|RF2/JH6 | ............<br>............<br>........ | .........<br><br> | ........<br>......<br>.... | ...H....<br>......V.<br>.... | .................N...<br>................. | .L.MG--<br>..................--<br>-.... | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-59/D3\|3-9\|RF1/JH4 | | QVQLQES-GPGLVKPSETLS LTCTVSG-GSIS | S----YYWS | WIRQPPGK GLEWIG | YIYY- SGSTNYNP SLKS | RVTISVDTSKNQFSLKL SSVTAADTAVYYCAR | VLRYFDW- -----LL*YFDY | WGQGTL VTVSS |
| IPS:43 5839 | 21-225_191B1 | VH4\|4-59/D3\|3-9\|RF1/JH4 | ........... ........... ....... | .......H.. | .....A. ...... | H..T.... ....K... .... | ...M............ | LRYNWN- ................- FPF... | ...... ..... |
| IPS:43 6158 | 21-225_197G8 | VH4\|4-59/D3\|3-9\|RF1/JH4 | ..H......... ........... ....... | A......S.. | .....A. ...... | RLSP.... G....F.. .... | ...M...........R. | LRYNWN- ................- FP.... | ....A. ..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-30.1/D3\|3-22\|RF2/JH6 | | QVQLQES-GPGLVKPSQILS LTCTVSG-GSIS | SG---GYYWS | WIRQHPGK GLEWIG | YIYY---- SGSTYYNP SLKS | RVTISVDTSKNQFSLKL SSVTAADTAVYYCAR | YYYDSSGYYY-------- ---YYYYYGMDV | WGQGTT VTVSS |
| IPS:43 5843 | 21-225_191F1 | VH4\|4-30.1/D3\|3-22\|RF2/JH6 | ........... ........... .......N | .........N | ........ ...... .... | ..F..... ........ .... | ................ | GD..G..S.- ..........- H....... | ...... ..... |
| IPS:43 5847 | 21-225_191A3 | VH4\|4-30.1/D3\|3-22\|RF2/JH6 | ........... ........... ....... | .....D...N | ........ ...... .... | ..F..... ........ .... | ................ | GD..G..S.- ..........- H....... | ...... ..... |
| IPS:43 5851 | 21-225_191D3 | VH4\|4-30.1/D3\|3-22\|RF2/JH6 | ....K....... ........... .......N | .....D...N | ........ ..D... .... | ..F..... ........ .... | ................ | GD..G..S.- ..........- H....... | ...... ..... |
| IPS:43 5905 | 21-225_190A3 | VH4\|4-30.1/D3\|3-22\|RF2/JH6 | ........... ........... .......N | .........N | ........ ...... .... | F.F..... ........ .... | N............... | GD..G..S.- ..........- H....... | ...... ..... |
| IPS:43 5911 | 21-225_190B4 | VH4\|4-30.1/D3\|3-22\|RF2/JH6 | ........... ........... ....... | .....D...N | ........ ...... .... | ..F..... ........ .... | ................ | GD..G..S.- ..........- H....... | ...... ..... |
| IPS:43 5913 | 21-225_190A7 | VH4\|4-30.1/D3\|3-22\|RF2/JH6 | ........... .N......... ........ | .....V.... | ........ ...... .... | N....... .....N.. .... | .II............. | GD..G..S.- ..........- H....L.. | ...... ..... |

| | | | | | | | | | |
|---|---|---|---|---|---|---|---|---|---|
| iPS:43 5939 | 21-225_191H7 | VH4\|4-30.1/D3\|3-22\|RF2/JH6 | . . . . . . . . . . . .<br>. . . . . . . . . . .<br>. . . . . . . N | . . . . . D . . . N | . . . . . . . .<br>. . . . . .<br>. . . . | . . F . . . . .<br>. . . . . . . .<br>. . . . | . . . . . . . . . . . . . . . . . . | GD . . G . . S . –<br>. . . . . . . . . . . –<br>H . . . . . . . . | . . . . . .<br>. . . . . |
| iPS:43 5967 | 21-225_192B3 | VH4\|4-30.1/D3\|3-22\|RF2/JH6 | . . . . . . . . . . . .<br>. . . . . . . . . . .<br>. . . . . . . . | . . . . . D . . . N | . . . . . . . .<br>. . . . . .<br>. . . . | F . F . . . . .<br>. . . . . . . .<br>. . . . | . . . . V . . . . . . . . . . | GD . . G . . S . –<br>. . . . . . . . . . . –<br>HH . . . . . . | . . . . . .<br>. . . . . |
| iPS:43 5973 | 21-225_192H3 | VH4\|4-30.1/D3\|3-22\|RF2/JH6 | . . . . . . . . . . . .<br>. . . . . . . . . . .<br>. . . . . . . . | . V . . . S . . . . | . . . . R . . .<br>. . . . . .<br>. . R . | NL . . . . . .<br>. . . . . . . .<br>. . R . | . A . . . . . . . . . . . . . .<br>. . . . . . . . . . . . . T . | GD . . G . . S . –<br>. . . . . . . . . . . –<br>H . . H . . . . | . . . . . .<br>. . . . . |
| iPS:43 6007 | 21-225_192G12 | VH4\|4-30.1/D3\|3-22\|RF2/JH6 | . . . . . . . . . . . .<br>. . . . . . . . . . .<br>. . . . . . . . | . . . . . V . H . . | . . . . . . . .<br>. . . . . .<br>. . . . | N . H . . . . .<br>. . . . . N . .<br>. . . . | . . . . . . . . . . . . . . . . . . | GD . . G . . S . –<br>. . . . . . . . . . . –<br>H . . . . . . . | . . . . . .<br>. . . . . |
| iPS:43 6009 | 21-225_193A1 | VH4\|4-30.1/D3\|3-22\|RF2/JH6 | . . . . . . . . . . . .<br>. . . . . . . . . . .<br>. . . . . . . . | . . . . . V . . . . | . . . . . . . .<br>. . . . . .<br>. . . . | N . . . . . . .<br>. . . . . N . .<br>. . . . | . L . . . A . . . . . . . . . . .<br>. . . . . . . . . . . . . . . . | GD . . G . . S . –<br>. . . . . . . . . . . –<br>H . . . . . . . | . . . . . .<br>. . . . . |
| iPS:43 6011 | 21-225_193B1 | VH4\|4-30.1/D3\|3-22\|RF2/JH6 | . . . . . . . . . . . .<br>. . . . . . . . . . .<br>. . . . . . . . | . . . . . V . . . . | . . . . . . . .<br>. . . . . .<br>. . . . | N . . . . . . .<br>. . . . . N . .<br>. . . . | . . . . . . . . . . . . . . . . . . | GD . . G . . S . –<br>. . . . . . . . . . . –<br>H . . . . . . . | . . . . . .<br>. . . . . |
| iPS:43 6017 | 21-225_193F3 | VH4\|4-30.1/D3\|3-22\|RF2/JH6 | . . . . . . . . . . . .<br>. . . . . . . . . . .<br>. . . . . . . N | . . . . . D . . . N | . . . . . . . .<br>. . . . . .<br>. . . . | . . F . . . . .<br>. . . . . . . .<br>. . . . | . . . . . . . . . . . . . . . . . . | GD . . G . . S . –<br>. . . . . . . . . . . –<br>H . . . . . . . | . . . . . .<br>. . . . . |
| iPS:43 6029 | 21-225_193H6 | VH4\|4-30.1/D3\|3-22\|RF2/JH6 | . . . . . . . . . . . .<br>. . . . . . . . . . .<br>. . . . . . . N | . . . . . D . . . N | . . . . . . . .<br>. . . . . .<br>. . . . | . . F . . . . .<br>. . . . . . . .<br>. . . . | . . . . . . . . . . . . . . . . . . | GD . . G . . S . –<br>. . . . . . . . . . . –<br>H . . . . . . . | . . . . . .<br>. . . . . |
| iPS:43 6035 | 21-225_193C8 | VH4\|4-30.1/D3\|3-22\|RF2/JH6 | . . . . . . . . . . . .<br>. . . . . . . . . . .<br>. . . . . . . . | . . . . . D . . . N | . . . . . . . .<br>. . . . . .<br>. . . . | . . F . . . . .<br>. . . . . . . .<br>. . . . | . . . . . . . . . . . . . . . . . . | GD . . G . . S . –<br>. . . . . . . . . . . –<br>H . . . . . . . | . . . . . .<br>. . . . . |

| | | | | | | | | | |
|---|---|---|---|---|---|---|---|---|---|
| iPS:43 6037 | 21-225_193D8 | VH4\|4-30.1/D3\|3-22\|RF2/JH6 | ............<br>............<br>.......N | .........N | ........<br>......<br>.... | F.F.....<br>........<br>.... | ..S.............<br>............... | GD..G..S.-<br>...........-<br>H....... | ......<br>..... |
| iPS:43 6041 | 21-225_193G8 | VH4\|4-30.1/D3\|3-22\|RF2/JH6 | ......K.....<br>............<br>......V. | .....V.... | ........<br>......<br>.... | N.......<br>.....N..<br>.... | N..............<br>............... | GD..G..S.-<br>...........-<br>HF...L.. | ..H...<br>..... |
| iPS:43 6062 | 21-225_194E5 | VH4\|4-30.1/D3\|3-22\|RF2/JH6 | ............<br>............<br>.......N | .....D...N | ...H....<br>......<br>.... | ..F.....<br>........<br>.... | ...............<br>............... | GD..G..S.-<br>...........-<br>H....... | ......<br>..... |
| iPS:43 6064 | 21-225_194E6 | VH4\|4-30.1/D3\|3-22\|RF2/JH6 | ............<br>............<br>....D..N | .....D...N | ........<br>......<br>.... | F.F.....<br>........<br>.... | ......I........<br>...NV.......... | GD..G..S.-<br>...........-<br>H....... | ......<br>..... |
| iPS:43 6134 | 21-225_196H12 | VH4\|4-30.1/D3\|3-22\|RF2/JH6 | ............<br>.N..........<br>........ | .....V.... | ........<br>......<br>.... | N.......<br>.....N..<br>.... | .II............<br>............... | GD..G..S.-<br>...........-<br>H....L.. | ......<br>..... |
| iPS:43 6146 | 21-225_197F4 | VH4\|4-30.1/D3\|3-22\|RF2/JH6 | ............<br>............<br>........ | .....D...N | ........<br>......<br>.... | ..F.....<br>........<br>.... | .I.............<br>............... | GD..G..S.-<br>...........-<br>H....L.. | ......<br>..... |
| iPS:43 6177 | 21-225_198B1 | VH4\|4-30.1/D3\|3-22\|RF2/JH6 | ....K.......<br>............<br>.......N | .....D...N | .F......<br>......<br>.... | ..FH....<br>........<br>.... | ...V...........<br>............... | GD..G..S.-<br>...........-<br>H....... | ..R...<br>..... |
| iPS:43 6179 | 21-225_198E1 | VH4\|4-30.1/D3\|3-22\|RF2/JH6 | ............<br>............<br>.......N | .........N | ........<br>......<br>.... | F.F.....<br>........<br>.... | .LS............<br>............... | GD..G..S.-<br>...........-<br>H....... | ......<br>..... |
| iPS:43 6197 | 21-225_199C2 | VH4\|4-30.1/D3\|3-22\|RF2/JH6 | ............<br>............<br>........ | .....D...N | .L....E.<br>......<br>.... | ..F.....<br>........<br>.... | .........MT......<br>T.............. | GD..G..S.-<br>...........-<br>H....... | ......<br>..... |

| | | | H_FR1 | H_CDR1 | H_FR2 | H_CDR2 | H_FR3 | H_CDR3 | H_FR4 |
|---|---|---|---|---|---|---|---|---|---|
| iPS:43 6207 | 21-225_199C7 | VH4|4-30.1/D3|3-22|RF2/JH6 | ............. ............. .......N | .........X | ........ ...... | F.F..... ........ .... | ..S............... ................. | GD..G..S.- ...........- H....... | ...... ..... |
| iPS:43 6226 | 21-225_200F10 | VH4|4-30.1/D3|3-22|RF2/JH6 | ............. ............. ........ | .....D...N | .L....E. ...... | ..F..... ........ .... | .I.......MT...... T............... | GD..G..S.- ...........- H....... | ...... ..... |
| | | Germline | H_FR1 | H_CDR1 | H_FR2 | H_CDR2 | H_FR3 | H_CDR3 | H_FR4 |
| | VH4|4-30.4/D3|3-22|RF2/JH6 | | QVQLQES- GPGLVKPSQILS LTCIVSG-GSIS | SG---DYYWS | WIRQPPGK GLEWIG | YIYY---- SGSTYYNP SLKS | RVTISVDTSKNQFSLKL SSVTAADTAVYYCAR | YYYDSSGYYY-------- ---YYYYYGMDV | WGQGTT VTVSS |
| iPS:43 5849 | 21-225_191C3 | VH4|4-30.4/D3|3-22|RF2/JH6 | ............. ............. ........ | .........N | ....L... ...... | ..F..... ........ .... | .L............... N............. | GD..G..S.- ...........- HF...... | ...... ..... |
| iPS:43 6015 | 21-225_193D3 | VH4|4-30.4/D3|3-22|RF2/JH6 | ............. ............. ........ | .........N | ....L... ...... | ..F..... ........ .... | .L............... ..............T. | GD..G..S.- ...........- HF...... | ...... ..... |
| iPS:43 6049 | 21-225_193B12 | VH4|4-30.4/D3|3-22|RF2/JH6 | ............. ............. ........ | .A.......N | ....L... ...... | ..F..... ........ .... | .L............... ................. | GD..G..S.- ...........- HF...... | ...... ..... |
| iPS:43 6088 | 21-225_195C8 | VH4|4-30.4/D3|3-22|RF2/JH6 | ............. ............. ........ | .........N | ....L... ...... | ..F..... ........ .... | .L............... ................. | GD..G..S.- ...........- HF...... | ...... ..... |
| iPS:43 6195 | 21-225_198G10 | VH4|4-30.4/D3|3-22|RF2/JH6 | ............. ............. ........ | .........N | ....L... ...... | ..F..... ........ .... | .L............... ................. | GD..G..S.- ...........- HF...... | ...... ..... |
| | | Germline | H_FR1 | H_CDR1 | H_FR2 | H_CDR2 | H_FR3 | H_CDR3 | H_FR4 |
| | VH4|4-30.1/D5|5-24|RF3/JH5 | | QVQLQES- GPGLVKPSQILS LTCIVSG-GSIS | SG---GYYWS | WIRQHPGK GLEWIG | YIYY---- SGSTYYNP SLKS | RVTISVDTSKNQFSLKL SSVTAADTAVYYCAR | RDGYNY------------ -------NWFDP | WGQGTL VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 5863 | 21-225_191H4 | VH4\|4-30.1/D5\|5-24\|RF3/JH5 | ............ ............ ........ | .........X | ........ ...... | ..F..... ........ ..R. | .L...I........... ¯............,G. | SGYNWD............ ........GV.. | ...... ..... |
| iPS:43 5943 | 21-225_191C9 | VH4\|4-30.1/D5\|5-24\|RF3/JH5 | ............ ............ ........ | .........X | ........ ..D... | ........ ........ .... | ................ N............. | SGYNWD............ .......AGV.. | ...... ..... |
| iPS:43 6094 | 21-225_195B10 | VH4\|4-30.1/D5\|5-24\|RF3/JH5 | ............ ............ ........ | NS........ | ........ ...... | .M...... ........ .... | ..............Y... .A............K | GGYNWN............ ........G..C | ...... ..... |
| | | Germline | H_FR1 | H_CDR1 | H_FR2 | H_CDR2 | H_FR3 | H_CDR3 | H_FR4 |
| | **VH3\|3-33/D2\|2-15\|RF3/JH6** | | QVQLVES-GGGVVQPGRSLR LSCAASG-FIFS | S-----YGMH | WVRQAPGK GLEWVA | VIWYD---GSNKYYAD SVKG | RFTISRDNSKNTLYLQM NSLRAEDTAVYYCAR | DIVVVVAAT--------- ---YYYYYGMDV | WGQGTT VTVSS |
| iPS:43 5869 | 21-225_190B1 | VH3\|3-33/D2\|2-15\|RF3/JH6 | ............ ............ T....... | .......... | ........ ...... | ........ ....H... .... | ................ | .RT.GY--- ............---- S.... | ...... ..... |
| iPS:43 6260 | 21-225_203H1 | VH3\|3-33/D2\|2-15\|RF3/JH6 | ............ ............ ........ | .......... | ........ ...... | ........ ....N... .... | ................ | .RT.GY--- ............---- N.... | ...... ..... |
| iPS:43 6490 | 21-225_221F6 | VH3\|3-33/D2\|2-15\|RF3/JH6 | ............ ............ ........ | ......N... | ........ ...... | ........ ...EN... .... | ................ | .RT.GY--- .............---- N.... | ...... ..... |
| iPS:43 6502 | 21-225_222A11 | VH3\|3-33/D2\|2-15\|RF3/JH6 | ............ ............ ........ | .......... | ........ ...... | ........ ....N... .... | ................ | .RD.GY--- ............---- N.... | ...... ..... |
| iPS:43 6514 | 21-225_222D10 | VH3\|3-33/D2\|2-15\|RF3/JH6 | ............ ............ ........ | .......... | ........ ...... | ........ ....N... .... | ................ | .RD.GY--- .............---- N.... | ...... ..... |
| iPS:43 6522 | 21-225_223H10 | VH3\|3-33/D2\|2-15\|RF3/JH6 | ............ ............ ........ | .......... | ........ ...... | ........ ....N... .... | .......H......... | .RD.GY--- ............---- N.... | ...... ..... |
| | | Germline | H_FR1 | H_CDR1 | H_FR2 | H_CDR2 | H_FR3 | H_CDR3 | H_FR4 |
| | **VH3\|3-21/D1\|1-1\|RF3/JH5** | | EVQLVES-GGGLVKPGGSLR LSCAASG-FIFS | S-----YSMN | WVRQAPGK GLEWVS | SISSS---SSYIYYAD SVKG | RPTISRDNAKNSLYLQM NSLRAEDTAVYYCAR | YNWND------ -------NWFDP | WGQGTL VTVSS |

| | | | H_FR1 | H_CDR1 | H_FR2 | H_CDR2 | H_FR3 | H_CDR3 | H_FR4 |
|---|---|---|---|---|---|---|---|---|---|
| IPS:43 5883 | 21-225_185A1 | VH3\|3-21\|D1\|1-1\|RF3\|JH5 | . . . . . . . . . . .<br>. . . . . . . . . . .<br>. . . . . . . N | . . . . . . . . . . | . . . . . . . .<br>. . . . . . | . . . . . . . .<br>G . . . . . . .<br>. . . . | H . . . . . . . . . . . . . . | S . L - -<br>. . . . . . . . . . . . . . . . . . .<br>. . - - . . C | . . . . . P<br>. . . . . |
| Germline | | | **H_FR1** | **H_CDR1** | **H_FR2** | **H_CDR2** | **H_FR3** | **H_CDR3** | **H_FR4** |
| | VH3\|3-23\|D1\|1-1\|RF1\|JH3 | | EVQLLES-<br>GGGLVQPGGSLR<br>LSCAASG-FIFS | S - - - - - YAMS | WVRQAPGK<br>GLEWVS | AISGS- - -<br>GGSTYYAD<br>SVKG | RFTISRDNSKNTLYLQM<br>NSLRAEDTAVYYCAK | GTTGT- - - - - - - - - - - -<br>- - - - - - - - DAFDI | WGQGTM<br>VTVSS |
| IPS:43 5895 | 21-225_188E8 | VH3\|3-23\|D1\|1-1\|RF1\|JH3 | . . . . . . . . . . .<br>. . . . . . . . . . .<br>. . . . . . . . | . . . . . . S . . N | . . . . . . . . .<br>. . . . . . | V . . . . . . .<br>. . Y . . . . .<br>. . . . | . . . . . . . . . . . . . . . . . .<br>. . . . . . . . . . . . F . . . | RN . DD . . . . . . . . . . . . .<br>. . . . . . . - . . . . | . . . . . .<br>. . . . . |
| Germline | | | **H_FR1** | **H_CDR1** | **H_FR2** | **H_CDR2** | **H_FR3** | **H_CDR3** | **H_FR4** |
| | VH3\|3-11\|D7\|7-27\|RF3\|JH4 | | QVQLVES-<br>GGGLVKPGGSLR<br>LSCAASG-FTFS | D - - - - - YYMS | WIRQAPGK<br>GLEWVS | YISSS- - -<br>GSTIYYAD<br>SVKG | RFTISRDNAKNSLYIQM<br>NSLRAEDTAVYYCAR | NWGY- - - - - - - - - - - - -<br>- - - - - - - - FDY | WGQGTL<br>VTVSS |
| IPS:43 5903 | 21-225_190E2 | VH3\|3-11\|D7\|7-27\|RF3\|JH4 | . . . . . . . . . . .<br>. . . . . . . . . . .<br>. . . . . . . . | . . . . . . . . . . | . . . . . . . .<br>. . . . L . | . T . VF . . .<br>. . . . | . . . . . . . . . . . . . . . . . . | E . VG . . . . . . . . . . . . . .<br>. . . . . . . . . A . . | . . . . . .<br>. . . . . |
| IPS:43 5923 | 21-225_190H6 | VH3\|3-11\|D7\|7-27\|RF3\|JH4 | . . . . . . . . . . .<br>. . . . . . . . . . .<br>. . . . . . . . | . . . . . . . . . . | . . . . . . . .<br>. . . . L . | . T . VF . . .<br>. . . . | . . . . . . . . . . . . . . . . . . | E . VG . . . . . . . . . . . . . .<br>. . . . . . . . . A . . | . . . . . .<br>. . . . . |
| IPS:43 5953 | 21-225_191B12 | VH3\|3-11\|D7\|7-27\|RF3\|JH4 | . . . . . . . . . . .<br>. . . . . . . . . . .<br>. . . . . . . . | . . . . . . . . . . | . . . . . . . .<br>. . . . . . | . T . VF . . .<br>. . . . | . . . . . . . . . . . . . . . . . . | E . VG . . . . . . . . . . . . . .<br>. . . . . . . . . A . . | . . . . . .<br>. . . . . |
| IPS:43 6098 | 21-225_195G11 | VH3\|3-11\|D7\|7-27\|RF3\|JH4 | . . . . . . . . . . .<br>. . . . . . . . . . .<br>. . . . . . . . | . . . . . . . . . . | . . . . . . . .<br>. . . . L . | . T . VF . . .<br>. . . . | . . . . . . . . . . . . . . . . . . | E . VG . . . . . . . . . . . . . .<br>. . . . . . . . . A . . | . . . . . .<br>. . . . . |
| IPS:43 6102 | 21-225_196B1 | VH3\|3-11\|D7\|7-27\|RF3\|JH4 | . . . . . . . . . . .<br>. . . . . . . . . . .<br>. . . . . . . . | . . . . . . . . . . | . . . . . . . .<br>. . . . I . | . I . M . . . .<br>. . . . | . . . . . . . . . . . . . . . . . . | E . VG . . . . . . . . . . . . . .<br>. . . . . . . . . A . . | . . . . . .<br>. . . . . |
| IPS:43 6104 | 21-225_196C1 | VH3\|3-11\|D7\|7-27\|RF3\|JH4 | . . . . . . . . . . .<br>. . . . . . . . . . .<br>. . . . . . . . | . . . . . . . . . . | . . . . . . . .<br>. . . . L . | . T . VF . . .<br>. . . . | . . . . . . . . . . . . . . . . . . | E . VG . . . . . . . . . . . . . .<br>. . . . . . . . . A . . | . . . . . .<br>. . . . . |
| Germline | | | **H_FR1** | **H_CDR1** | **H_FR2** | **H_CDR2** | **H_FR3** | **H_CDR3** | **H_FR4** |
| | VH3\|3-23\|D6\|6-19\|RF2\|JH4 | | EVQLLES-<br>GGGLVQPGGSLR<br>LSCAASG-FTFS | S - - - - - YAMS | WVRQAPGK<br>GLEWVS | AISGS- - -<br>GGSTYYAD<br>SVKG | RFTISRDNSKNTLYLQM<br>NSLRAEDTAVYYCAK | GIAVA- - - - - - - - - - - -<br>- - - - - CYFDY | WGQGTL<br>VTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:43 5965 | 21-225_192H2 | VH3\|3-23/D6\|6-19\|RF2/JH4 | ..........D. I............ ........ | ......S... | ........ ...... .... | ........ ..N.F... .... | ................. | L...VG............ ......SH.... | ...... ..... |
| iPS:43 6160 | 21-225_197C9 | VH3\|3-23/D6\|6-19\|RF2/JH4 | ............ A........... .......R | .......... | ........ ...... .... | V...R... ..N.... .... | ................. | .....G............ ......SH.... | ...... ..... |
| iPS:39 2954 | 21-225_26A10 | VH3\|3-23/D6\|6-19\|RF2/JH4 | ............ ............ ........ | .......... | ........ ...... .... | V....... .VN.F... .... | ...............L. | K....G............ ......TH.... | ...... ..... |
| | Germline | | QVQLQES-GPGLVKPSQILS LTCTVSG-GSIS | SG---GYYWS | WIRQHPGK GLEWIG | YIYY---- SGSTYYNP SLKS | RVTISVDTSKNQFSLKL SSVTAADTAVYYCAR | RDGYN----------- -------YYFDY | WGQGTL VTVSS |
| iPS:43 5983 | 21-225_192E5 | VH4\|4-30.1/D5\|5-24\|RF3/JH4 | ............ ............ ....D..N | N......... | ........ ...... .... | ..F..... ........ .... | N..........F... | AGYNWD............ .......NG... | ...... ..... |
| iPS:43 6043 | 21-225_193G9 | VH4\|4-30.1/D5\|5-24\|RF3/JH4 | ............ ............ ....D..N | N......... | ........ ...... .... | ..F..... ........ .... | N..........F... | AGYNWD............ .......NG... | ...... ..... |
| iPS:43 6084 | 21-225_195F2 | VH4\|4-30.1/D5\|5-24\|RF3/JH4 | ............ ............ ....D... | .......... | ........ ...... .... | .S...... ....N... .... | .......M........ ....D.......... | GGYNWN............ .......NG... | ....A. ..... |
| iPS:43 7138 | 21-225_214D8 | VH4\|4-30.1/D5\|5-24\|RF3/JH4 | ............ ............ ........ | TA...F.... | ........ ...... .... | ...F.... ........ .... | ................N. | AR..HY............ .......SI... | ...... ..... |
| | Germline | | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | VL*LRLGEL-------- ----SLYDAFDI | WGQGTM VTVSS |
| iPS:43 6003 | 21-225_192G10 | VH3\|3-23/D3\|3-16\|RF1/JH3 | ............ .........S ........ | .......... | ........ ...... .... | ....R... ....F... .... | ................. ..............R | R.A.DG--- .............-- ...... | ...... .... |
| | Germline | | H_FR1 | H_CDR1 | H_FR2 | H_CDR2 | H_FR3 | H_CDR3 | H_FR4 |

| | | | H_FR1 | H_CDR1 | H_FR2 | H_CDR2 | H_FR3 | H_CDR3 | H_FR4 |
|---|---|---|---|---|---|---|---|---|---|
| | VH3\|3-23\|D7\|7-27\|RF2/JH4 | | EVQLLES-GGGLVQPGGSLR LSCAASG-FTFS | S-----YAMS | WVRQAPGK GLEWVS | ATSGS--- GGSTYYAD SVKG | RFTISRDNSKNTLYLQM NSLRAEDTAVYYCAK | *LGY---------------- ---------FDY | WGQGTL VTVSS |
| IPS:43 6019 | 21-225_193C4 | VH3\|3-23\|D7\|7-27\|RF2/J H4 | ............ A........... ........ | .........X | ........ ...... | ..I.N... ..R..... .... | ..S..........F... ................ | D..RYS............ ......YGF... | ...... ..... |
| | | Germline | H_FR1 | H_CDR1 | H_FR2 | H_CDR2 | H_FR3 | H_CDR3 | H_FR4 |
| | VH4\|4-30.1\|D1\|1-1\|RF1/JH6 | | QVQLQES- GPGLVKPSQTLS LTCIVSG-GSIS | SG---GYYWS | WIRQHPGK GLEWIG | YIYY---- SGSTYYNP SLKS | RVTISVDTSKNQFSLKL SSVTAADTAVYYCAR | GTTGTYY------------- -----YYYGMDV | WGQGTT VTVSS |
| IPS:43 6025 | 21-225_193B5 | VH4\|4-30.1\|D1\|1-1\|RF1/JH 6 | ............ ............ ........ | .......... | ........ ...... | ........ .... | ...A.......... ................ | .EYNWN- .................-- H.... | ...... ..... |
| | | Germline | H_FR1 | H_CDR1 | H_FR2 | H_CDR2 | H_FR3 | H_CDR3 | H_FR4 |
| | VH4\|4-34\|D4\|4-11\|RF2/JH4 | | QVQLQQW- GAGLLKPSETLS LTCAVYG-GSFS | G-----YYWS | WIRQPPGK GLEWIG | EINH---- SGSTNYNP SLKS | RVTISVDTSKNQFSLKL SSVTAADTAVYYCAR | DYSNY--------------- --------YFDY | WGQGTL VTVSS |
| IPS:43 6033 | 21-225_193E7 | VH4\|4-34\|D4\|4-11\|RF2/J H4 | ............ ............ ........ | .......F.T | ........ ...... | ........ ........ .... | ................. ................. | ..G-- ................ ...-A.. | ...... .A..A |
| IPS:43 6199 | 21-225_199E3 | VH4\|4-34\|D4\|4-11\|RF2/J H4 | ............ ............ ........ | .......F.T | ........ ...... | ........ ........ .... | ................. ................. | ..G-- ................ ...-A.. | ...... .A..A |
| IPS:43 6228 | 21-225_200F12 | VH4\|4-34\|D4\|4-11\|RF2/J H4 | ............ ............ ........ | .......F.T | ........ ...... | ..S..... ........ .... | ................V ................. | ..G-- ................ ...-A.. | ...... ..... |
| IPS:43 6230 | 21-225_201A1 | VH4\|4-34\|D4\|4-11\|RF2/J H4 | ............ ............ .T...... | .......F.T | ........ ...... | ..S..... ..R..... .... | .....G............ ................. | ..G-- ................ ...-A.. | ...... ..... |
| IPS:43 6242 | 21-225_201A10 | VH4\|4-34\|D4\|4-11\|RF2/J H4 | ............ ............ ........ | .......F.T | ........ ...... | ..S..... ........ .... | ................V N................ | ..G-- ................ ...-A.. | ...... ..... |
| IPS:43 6286 | 21-225_204H8 | VH4\|4-34\|D4\|4-11\|RF2/J H4 | ............ .......F.... ........ | .......F.T | .V...... ...... | ..S..... ....S... .... | .......K......... ................ | ..G-- ................ ...-A.. | ...... ..... |

| IPS:43 6308 | 21-225_205H8 | VH4\|4-34/D4\|4-11\|RF2/JH4 | ............ ............ ........ | .......F.. | ........ ...... .... | ..S..... ..R..... | ................V | ..G-- ................ ...-A.. | ...... ..... |
| | | Germline | H_FR1 | H_CDR1 | H_FR2 | H_CDR2 | H_FR3 | H_CDR3 | H_FR4 |
| | VH3\|3-30.3/D1\|1-1\|RF1/JH4 | | QVQLVES-GGGVVQPGRSLR LSCAASG-FIFS | S-----YAMH | WVRQAPGK GLEWVA | VISYD--- GSNKYYAD SVKG | RFTISRDNSKNTLYLQM NSLRAEDTAVYYCAR | GTTGT------------ --------YFDY | WGQGTL VTVSS |
| IPS:43 6051 | 21-225_193G12 | VH3\|3-30.3/D1\|1-1\|RF1/JH4 | ............ ..........G ........ | .......... | ........ ...... .... | ..W..... .T....G. | ..............N... | DF.I.G........... ......AT.... | ...... ..... |
| | | Germline | H_FR1 | H_CDR1 | H_FR2 | H_CDR2 | H_FR3 | H_CDR3 | H_FR4 |
| | VH3\|3-33/D7\|7-27\|RF3/JH6 | | QVQLVES-GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | NWGYYY----------- -------YYGMDV | WGQGTT VTVSS |
| IPS:43 6054 | 21-225_194C1 | VH3\|3-33/D7\|7-27\|RF3/JH6 | ............ ............ ........ | .......... | ........ ...... .... | ...EH... | ................ | .R.VG............ ......-..L.. | ...... ..... |
| | | Germline | H_FR1 | H_CDR1 | H_FR2 | H_CDR2 | H_FR3 | H_CDR3 | H_FR4 |
| | VH1\|1-02/D5\|5-18\|RF3/JH1 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | GYSYGY----------- --------AEYFQH | WGQGTL VTVSS |
| IPS:43 6058 | 21-225_194A4 | VH1\|1-02/D5\|5-18\|RF3/JH1 | .........T.. ......L..... ........ | V.......LN | ........ ...... .... | ........ | ................ | ..DI-- ................ ---LTG | ...... ..... |
| | | Germline | H_FR1 | H_CDR1 | H_FR2 | H_CDR2 | H_FR3 | H_CDR3 | H_FR4 |
| | VH1\|1-02/D1\|1-26\|RF3/JH6 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | YSGSYYYY--------- -----YYYGMDV | WGQGTT VTVSS |
| IPS:43 6068 | 21-225_194F7 | VH1\|1-02/D1\|1-26\|RF3/JH6 | ............ ............ ........ | D.......I. | ........ ...... .... | N....... | ................ | EPLG..GSC........ ...SYGA..... | ...... ..... |
| | | Germline | H_FR1 | H_CDR1 | H_FR2 | H_CDR2 | H_FR3 | H_CDR3 | H_FR4 |
| | VH4\|4-34/D4\|4-17\|RF2/JH3 | | QVQLQQW-GAGLLKPSEILS LTCAVYG-GSFS | G-----YYWS | WIRQPPGK GLEWIG | EINH--- SGSTNYNP SLKS | RVTISVDTSKNQFSLKL SSVTAADTAVYYCAR | DYGDY----------- -------DAFDI | WGQGTM VTVSS |
| IPS:43 6072 | 21-225_194C10 | VH4\|4-34/D4\|4-17\|RF2/JH3 | ............ ............ .S.....R | Y......... | ........ ....F. .... | ........ ........ | .....I........... R........... | ...A- ................ ..--... | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 6506 | 21-225_222C7 | VH4|4-34/D4|4-17|RF2/JH3 | ............ ............ ........ | ......R... | ........ ...... | ...A.... .... | ................. | ...A- ................. ..--L.F | ...... ..... |
| | | Germline | H_FR1 | H_CDR1 | H_FR2 | H_CDR2 | H_FR3 | H_CDR3 | H_FR4 |
| | VH3|3-33/D5|5-24|RF2/JH6 | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | *RWLQLYY----------------YYYGMDV | WGQGTTVTVSS |
| iPS:43 6092 | 21-225_195B9 | VH3|3-33/D5|5-24|RF2/JH6 | ............ ............ ........ | .......... | ........ ...... | ........ .... | ................. | EWLQFR.- ...............- ...... | ...... ..... |
| iPS:43 6164 | 21-225_197G10 | VH3|3-33/D5|5-24|RF2/JH6 | ............ ..........V ........ | .......... | ........ ...... | .I...... .... | ................. | EWLQFR.- ...............- ...I.. | ...... ..... |
| iPS:43 6191 | 21-225_198B9 | VH3|3-33/D5|5-24|RF2/JH6 | ............ ............ ........ | N......... | ........ ...... | I..F.... ......V. .... | ............F... S............... | EWLQFR.- ...............- ...... | ...... ..... |
| iPS:43 6205 | 21-225_199A7 | VH3|3-33/D5|5-24|RF2/JH6 | ............ ............ ........ | N......... | ........ ...... | I..F.... ........ .... | ............F... S............... | EWLQFR.- ...............- ...... | ...... ..... |
| iPS:43 6214 | 21-225_200F6 | VH3|3-33/D5|5-24|RF2/JH6 | ............ ............ ........ | .......... | ........ ...... | ........ .... | ............T. | EWLQFR.- ...............- | ...... ..... |
| | | Germline | H_FR1 | H_CDR1 | H_FR2 | H_CDR2 | H_FR3 | H_CDR3 | H_FR4 |
| | VH3|3-33/D6|6-19|RF3/JH6 | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | V*QWLVYY----------------YYYGMDV | WGQGTTVTVSS |
| iPS:43 6106 | 21-225_196F2 | VH3|3-33/D6|6-19|RF3/JH6 | ............ ............ ........ | ......F... | ........ ...... | ..LN.... ....KC.. .... | .C............... | GQ....-- ...............-- N.V.. | ...... ..... |
| | | Germline | H_FR1 | H_CDR1 | H_FR2 | H_CDR2 | H_FR3 | H_CDR3 | H_FR4 |
| | VH3|3-23/D6|6-6|RF3/JH6 | | EVQLLES-GGGLVQPGGSLRLSCAASG-FIFS | S-----YAMS | WVRQAPGKGLEWVS | AISGS---GGSTYYADSVKG | RFTISRDNSKNTLYLQMNSLRAEDTAVYYCAK | V*QLVYY----------------YYYGMDV | WGQGTTVTVSS |
| iPS:43 6110 | 21-225_196F4 | VH3|3-23/D6|6-6|RF3/JH6 | ............ ........F... ........ | ......C..T | ........ ...... | ........ .... | ................. | .GG.TGSY.......... ............ | ...... ..... |
| | | Germline | H_FR1 | H_CDR1 | H_FR2 | H_CDR2 | H_FR3 | H_CDR3 | H_FR4 |

| | | | H_FR1 | H_CDR1 | H_FR2 | H_CDR2 | H_FR3 | H_CDR3 | H_FR4 |
|---|---|---|---|---|---|---|---|---|---|
| | VH1|1-08/D2|2-21|RF1/JH5 | | QVQLVQS-GAEVKKPGASVK VSCKASG-YIFT | S-----YDIN | WVRQATGQ GLEWMG | WMNPN---SGNTGYAQ KFQG | RVTMTRNTSISTAYMEL SSLRSEDTAVYYCAR | SILWW*L-----------LFNWFDP | WGQGTL VTVSS |
| iPS:43 6114 | 21-225_196G8 | VH1|1-08/D2|2-21|RF1/JH5 | ............ ............ ........ | N......... | .M...... ...... | ..HL.... .......P .... | ......D......F... ..............Y | .GG.Y-- .................--  -V... | ...... ..... |
| iPS:43 6218 | 21-225_200G7 | VH1|1-08/D2|2-21|RF1/JH5 | ............ ............ ........ | N......... | .M...... ...... | ..HL.... .......P .... | ......D......F... ..............Y | .GG.Y-- .................--  -V... | ...... ..... |
| | Germline | | H_FR1 | H_CDR1 | H_FR2 | H_CDR2 | H_FR3 | H_CDR3 | H_FR4 |
| | VH1|1-02/D3|3-10|RF3/JH6 | | QVQLVQS-GAEVKKPGASVK VSCKASG-YIFT | G-----YYMH | WVRQAPGQ GLEWMG | WINPN---SGGTNYAQ KFQG | RVTMTRDTSISTAYMEL SRLRSDDTAVYYCAR | ITMVRGVII----------YYYYYGMDV | WGQGTT VTVSS |
| iPS:43 6116 | 21-225_196B9 | VH1|1-02/D3|3-10|RF3/JH6 | ............ ......M..... ........ | .......... | ........ ...... | ........ .....F.. ..R. | ................ ...S........... | GGVRGVPN- -- ...V... | ...... ..... |
| | Germline | | H_FR1 | H_CDR1 | H_FR2 | H_CDR2 | H_FR3 | H_CDR3 | H_FR4 |
| | VH4|4-30.1/D5|5-18|RF3/JH6 | | QVQLQES-GPGLVKPSQTLS LTCTVSG-GSIS | SG----GYYWS | WIRQHPGK GLEWIG | YIYY----SGSTYYNP SLKS | RVTISVDTSKNQFSLKL SSVTAADTAVYYCAR | GYSYGYYY---------YYYGMDV | WGQGTT VTVSS |
| iPS:43 6181 | 21-225_198C2 | VH4|4-30.1/D5|5-18|RF3/JH6 | ............ ............ ........ | .......... | ........ ...... | N....... ........ .... | ................ ................ | .DY..SGSY......... ....HN...L.. | ...... ..... |
| iPS:43 6210 | 21-225_199G11 | VH4|4-30.1/D5|5-18|RF3/JH6 | ............ ............ ........ | .......... | ........ ...... | N....... ........ .... | ................ ................ | .DY..SGSY......... ....HN...L.. | ...... ..... |
| | Germline | | H_FR1 | H_CDR1 | H_FR2 | H_CDR2 | H_FR3 | H_CDR3 | H_FR4 |
| | VH1|1-18/D3|3-3|RF2/JH6 | | QVQLVQS-GAEVKKPGASVK VSCKASG-YIFT | S-----YGIS | WVRQAPGQ GLEWMG | WISAY---NGNTNYAQ KLQG | RVTMTTDTSTSTAYMEL RSLRSDDTAVYYCAR | YYDFWSGYYT---------YYYYYGMDV | WGQGTT VTVSS |
| iPS:43 6234 | 21-225_51E3 | VH1|1-18/D3|3-3|RF2/JH6 | ............ ............ .......N | .......... | ...L.... ...... | ........ ....KN.. RF.. | ................ | HDFWSGY--- ..............--- .K.... | ...... ..... |
| iPS:43 6830 | 21-225_51F4 | VH1|1-18/D3|3-3|RF2/JH6 | ............ ............ .......N | .......... | ........ ...... | ........ ....K... .... | ................ | HDFWSGY--- .............--- .K.... | ...... ..... |

| | | | | | | | | | |
|---|---|---|---|---|---|---|---|---|---|
| IPS:43 6834 | 21-225_52F1 | VH1\|1-18/D3\|3-3\|RF2/JH6 | ............<br>............<br>.......N | ........V. | ........<br>......<br>.... | ........<br>...RK...<br>.... | ..S............<br>.............. | HDFWSCY---<br>...........---<br>.K.... | ......<br>..... |
| IPS:43 6842 | 21-225_54E9 | VH1\|1-18/D3\|3-3\|RF2/JH6 | ............<br>............<br>.......N | .......... | ...L....<br>......<br>.... | ........<br>....KN..<br>.... | ..............<br>.............. | HDFWSGY---<br>...........---<br>.K.... | ......<br>..... |
| IPS:43 6844 | 21-225_56G1 | VH1\|1-18/D3\|3-3\|RF2/JH6 | ............<br>............<br>......N | .......... | ........<br>......<br>.F.. | ........<br>....K...<br>.... | ..............<br>.............. | HDFWSGY---<br>...........---<br>.K.... | ......<br>..... |
| IPS:43 6846 | 21-225_56E3 | VH1\|1-18/D3\|3-3\|RF2/JH6 | ............<br>............<br>......N | .......F. | ........<br>......<br>.F.. | ........<br>....KE..<br>.... | ....A.........<br>.............. | HDFWSGY---<br>...........---<br>.K.... | ......<br>..... |
| IPS:45 1104 | 21-225_49C5 | VH1\|1-18/D3\|3-3\|RF2/JH6 | ............<br>............<br>......N | .......... | ........<br>......<br>.... | ........<br>....K...<br>.... | ..............<br>.............. | HDFWSGY---<br>...........---<br>.K.... | ......<br>..... |
| IPS:45 1106 | 21-225_49D10 | VH1\|1-18/D3\|3-3\|RF2/JH6 | ............<br>............<br>......N | .......... | ...L....<br>.F....<br>.... | ........<br>....KN..<br>.... | ..............<br>.............. | HDFWSGY---<br>...........---<br>.K.... | ......<br>..... |
| IPS:45 1108 | 21-225_53E8 | VH1\|1-18/D3\|3-3\|RF2/JH6 | ............<br>............<br>......N | .......... | ........<br>......<br>.... | ........<br>....KF..<br>.... | ..............<br>.............. | HDFWSGY---<br>...........---<br>.K.... | ......<br>..... |
| | | Germline | H_FR1 | H_CDR1 | H_FR2 | H_CDR2 | H_FR3 | H_CDR3 | H_FR4 |
| | VH6\|6-01/D3\|3-9\|RF1/JH6 | | QVQLQQS-GPGLVKPSQTLS LTCAISG-DSVS | SN---SAAWN | WIRQSPSR GLEWLG | RTYYR--SKWYNDYA VSVKS | RITINPDTSKNQFSLQL NSVTPEDTAVYYCAR | VLRYFDWLL* ---YYYYYGMDV | WGQGTT VTVSS |
| IPS:43 6236 | 21-225_201F7 | VH6\|6-01/D3\|3-9\|RF1/JH6 | ............<br>............<br>........ | .......... | ........<br>...... | ........<br>....Y.E.<br>..R. | ..............<br>..........F... | DQ..Y-----<br>...........-----<br>.... | ......<br>..... |
| IPS:43 6250 | 21-225_201A4 | VH6\|6-01/D3\|3-9\|RF1/JH6 | ............<br>............<br>........ | .......... | ........<br>...... | ........<br>....Y.E.<br>.... | ..............<br>...........F... | DQ..Y-----<br>...........-----<br>.... | ......<br>..... |
| IPS:43 6252 | 21-225_202A8 | VH6\|6-01/D3\|3-9\|RF1/JH6 | ............<br>............<br>........ | .......... | ........<br>...... | ........<br>....E...<br>..R. | ..............<br>.........L...T. | DQ..Y-----<br>...........-----<br>.... | ......P<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 6278 | 21-225_201F2 | VH6\|6-01/D3\|3-9\|RF1/JH6 | ............ ............ ........ | .......... | ........ ...... | ........ ....Y.E. ..R. | .V.............. ..........F... | DQ..Y----- ..........,----- .... | ...... .... |
| iPS:43 6294 | 21-225_205G4 | VH6\|6-01/D3\|3-9\|RF1/JH6 | ............ ............ ........ | .......... | ........ ...... | ........ ....Y.E. ..R. | ...............F... | DQ..Y----- ..........----- .... | ...... .... |
| iPS:43 6356 | 21-225_210H10 | VH6\|6-01/D3\|3-9\|RF1/JH6 | ............ ............ ........ | .......... | ........ ...... | ........ ....Y.P. ..R. | ................L. | DQ..Y----- ..........----- .... | ...... .... |
| | | Germline | H_FR1 | H_CDR1 | H_FR2 | H_CDR2 | H_FR3 | H_CDR3 | H_FR4 |
| | VH1\|1-02/D4\|4-23\|RF2/JH4 | | QVQLVQS-GAEVKKPGASVK VSCKASG-YIFT | G------YYMH | WVRQAPGQ GLEWMG | WINPN--- SGGTNYAQ KFQG | RVTMTRDTSISTAYMEL SRLRSDDTAVYYCAR | DYGGN --------SYFDY | WGQGTL VTVSS |
| iPS:43 6240 | 21-225_201E8 | VH1\|1-02/D4\|4-23\|RF2/JH4 | ............ ............ ........ | .......... | ........ ...... | ..D..... ......P. .... | ................ ..........F...K | .Q.Y.W............ .......NS... | ...... ..... |
| iPS:43 6314 | 21-225_206G4 | VH1\|1-02/D4\|4-23\|RF2/JH4 | ............ ............ ........ | .........I. | ........ ...... | ..D..... ...... .... | .I............... ..........F...K | .Q.Y.W............ .......NS... | ...... ..... |
| | | Germline | H_FR1 | H_CDR1 | H_FR2 | H_CDR2 | H_FR3 | H_CDR3 | H_FR4 |
| | VH1\|1-02/D5\|5-18\|RF3/JH5 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | GYSYGY----------- --------NWFDP | WGQGTL VTVSS |
| iPS:43 6244 | 21-225_201H10 | VH1\|1-02/D5\|5-18\|RF3/JH5 | ............ ............ ........ | .........I. | ........ .....A | ........ .... | ..........T.T.... ................ | ................ ............ | ...... ..... |
| iPS:43 6262 | 21-225_203E3 | VH1\|1-02/D5\|5-18\|RF3/JH5 | ............ ............ ........ | .........I. | ........ .....A | ........ .... | ..........T...... ................ | ................ ............ | ...... ..... |
| iPS:43 6276 | 21-225_204H4 | VH1\|1-02/D5\|5-18\|RF3/JH5 | ............ ............ ........ | .........I. | ........ .....A | ........ .... | ..........T...... ................ | ................ ............ | ...... ..... |
| iPS:43 6312 | 21-225_206A4 | VH1\|1-02/D5\|5-18\|RF3/JH5 | ............ ............ ........ | .........I. | ........ .....A | ........ .... | ..........T...... ................ | ................ ............ | ...... ..... |

| | | | | | | | | | |
|---|---|---|---|---|---|---|---|---|---|
| iPS:43 6316 | 21-225_206A5 | VH1|1-02/D5|5-18|RF3/JH5 | ...........<br>...........<br>........ | .........I. | ........<br>.....A | ........<br>........<br>.... | ..........T...... | ..................<br>............ | ......<br>..... |
| iPS:43 6338 | 21-225_208E8 | VH1|1-02/D5|5-18|RF3/JH5 | ...........<br>...........<br>........ | .........I. | ........<br>.....A | ........<br>........<br>.... | ..........T...... | ..................<br>........... | ......<br>..... |
| iPS:43 6344 | 21-225_208B11 | VH1|1-02/D5|5-18|RF3/JH5 | ...........<br>...........<br>........ | .........I. | ........<br>.....A | ........<br>........<br>.... | ..........T...... | ..................<br>........... | ......<br>..... |
| iPS:43 6358 | 21-225_210D11 | VH1|1-02/D5|5-18|RF3/JH5 | ...........<br>...........<br>........ | .........I. | ........<br>.....A | ........<br>........<br>.... | ..........T...... | ..................<br>.............. | ......<br>..... |
| iPS:43 6408 | 21-225_214H8 | VH1|1-02/D5|5-18|RF3/JH5 | ...........<br>...........<br>........ | ......H.I. | ........<br>...... | ...S....<br>........<br>.... | ................<br>...............K | DGR.S.G...........<br>......YD.... | ......<br>..... |
| iPS:43 6424 | 21-225_215H6 | VH1|1-02/D5|5-18|RF3/JH5 | ...........<br>...........<br>........ | ......H.I. | ........<br>...... | ...S....<br>.......E<br>.... | ................<br>...............K | DGR.S.G...........<br>......HD.... | ......<br>..... |
| iPS:43 7092 | 21-225_210B12 | VH1|1-02/D5|5-18|RF3/JH5 | ...........<br>........I...<br>....F... | D........N | ........<br>...... | ........<br>........<br>.... | ................<br>................ | ..DS--<br>..................<br>.--.A. | ......<br>..... |
| iPS:43 7134 | 21-225_213A7 | VH1|1-02/D5|5-18|RF3/JH5 | ...........<br>........I..R<br>....F... | D........N | ........<br>...... | ....K...<br>N.......<br>.... | .........L.R.....<br>...............K | ..DS--<br>..................<br>.--.A. | ......<br>..... |
| iPS:43 7194 | 21-225_226B2 | VH1|1-02/D5|5-18|RF3/JH5 | ...........<br>...........<br>........ | .......F.. | ........<br>...... | ........<br>..D.....<br>.... | .........LN......<br>.........I..... | .TY..SGS..........<br>.....YF.EL.S | ......<br>..... |
| iPS:43 7196 | 21-225_226B7 | VH1|1-02/D5|5-18|RF3/JH5 | ...........<br>...........<br>........ | ......... | ........<br>...... | ........<br>........<br>...D | ..............H...<br>............... | ..Y..SGS..........<br>.....YY....S | ......<br>..... |
| iPS:43 7200 | 21-225_226A10 | VH1|1-02/D5|5-18|RF3/JH5 | ...........<br>...........<br>........ | .......F.. | ........<br>...... | ........<br>..D.....<br>.... | .........L......<br>.........I..... | .TY..SGS.........<br>.....YF.EL.S | ......<br>..... |
| iPS:39 3168 | 21-225_32B11 | VH1|1-02/D5|5-18|RF3/JH5 | ...........<br>...........<br>........ | ......... | ........<br>...... | ........<br>.D......<br>.... | N.............. | .FY..SGS..........<br>.....YY.DL.. | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:39 3178 | 21-225_34D7 | VH1\|1-02/D5\|5-18\|RF3/JH5 | ............ / ............ / ........ | ......... | ....... / ...... | ........ / .... | .................... / ................. | ..Y..SGS.......... / .....YY.DL.. | ...... |
| iPS:39 8480 | 21-225_17G4 | VH1\|1-02/D5\|5-18\|RF3/JH5 | ............ / ............ / T....... | D......... | ....... | ...... / ......E. / .... | .................... / .................S | ............ | ...... |
| iPS:39 8486 | 21-225_19A1 | VH1\|1-02/D5\|5-18\|RF3/JH5 | ............ / ............ / ........ | ......... | ....... | ...... / ........ / .... | .................... / .................S | ............ | ...... |
| | **VH1\|1-08/D6\|6-13\|RF1/JH6** | Germline | QVQLVQS-GAEVKKPGASVK VSCKASG-YIFT | S------YDIN | WVRQATGQ GLEWMG | WMNPN---SGNTGYAQ KFQG | RVTMTRNTSISTAYMEL SSLRSEDTAVYYCAR | GYSSSWYY----------YYYGMDV | WGQGTT VTVSS |
| iPS:43 6248 | 21-225_202A3 | VH1\|1-08/D6\|6-13\|RF1/JH6 | ....E....... / ........... / ........ | .......... | ....... / .....L. | ...K... / R....... / .... | ................H... / ................ | .RY.RED........... / ........D... | ....... |
| | **VH3\|3-11/D4\|4-17\|RF2/JH6** | Germline | QVQLVES-GGGLVKPGGSLR LSCAASG-FIFS | D------YYMS | WIRQAPGK GLEWVS | YISSS---GSTIYYAD SVKG | RFTISRDNAKNSLYLQM NSLRAEDTAVYYCAR | DYGDYYY----------YYYGMDV | WGQGTT VTVSS |
| iPS:43 6270 | 21-225_203F10 | VH3\|3-11/D4\|4-17\|RF2/JH6 | ......... / ........... / ........ | .......... | ........ / .....L | ...G.... / .T.T.... / .... | ................ / ................ | .R.G--- / .................--  / --L.. | ...... |
| | **VH3\|3-23/D1\|1-20\|RF1/JH6** | Germline | EVQLLES-GGGLVQPGGSLR LSCAASG-FIFS | S------YAMS | WVRQAPGK GLEWVS | AISGS---GGSTYYAD SVKG | RFTISRDNSKNTLYLQM NSLRAEDTAVYYCAK | GITGTYY----------YYYGMDV | WGQGTT VTVSS |
| iPS:43 6280 | 21-225_204D6 | VH3\|3-23/D1\|1-20\|RF1/JH6 | ...V........ / ........... / ........ | T......... | ....... / ...... | ........ / ........ / .... | D................ / ................ | ..S..GSY.......... / .........V.. | ...... |
| | **VH3\|3-33/D7\|7-27\|RF2/JH6** | Germline | QVQLVES-GGGVVQPGRSLR LSCAASG-FIFS | S------YGMH | WVRQAPGK GLEWVA | VIWYD---GSNKYYAD SVKG | RFTISRDNSKNTLYLQM NSLRAEDTAVYYCAR | *LCYYY----------YYYGMDV | WGQGTT VTVSS |
| iPS:43 6284 | 21-225_204G8 | VH3\|3-33/D7\|7-27\|RF2/JH6 | ..........D. / ........... / ........ | .......... | .G...... / ...... | ........ / ...EN.VA / .... | ....F............. / ................ | D..IG............. / ......-..... | ...... |

| | | | H_FR1 | H_CDR1 | H_FR2 | H_CDR2 | H_FR3 | H_CDR3 | H_FR4 |
|---|---|---|---|---|---|---|---|---|---|
| iPS:43 6968 | 21-225_190B10 | VH3\|3-33/D7\|7-27\|RF2/JH6 | ............ ..........V ........ | .......... | ........ ...... | ...N.... ..K..HV. .... | ................. .........L..... | D.DKRNFPY......... ....YY...... | ...... ..... |
| iPS:43 7006 | 21-225_192G2 | VH3\|3-33/D7\|7-27\|RF2/JH6 | ............ .......... ........ | .......... | ........ .....T | ...N.... ......... .... | ................. .........L..... | D.DKRNFPY......... ....YY...... | ...... ..... |
| iPS:43 7024 | 21-225_194F11 | VH3\|3-33/D7\|7-27\|RF2/JH6 | ............ .......... ........ | .......... | ........ ...... | ...N.... ..K..HV. .... | ................. .........L..... | D.DKRNFPY......... ....YY...... | ...... ..... |
| iPS:43 7028 | 21-225_194G12 | VH3\|3-33/D7\|7-27\|RF2/JH6 | ............ .......... ........ | .......... | ........ .....T | ...N.... ......... .... | ................. .........L..... | D.DKRNFPY......... ....YY...... | ...... ..... |
| | Germline | **VH4\|4-34/D6\|6-19\|RF3/JH3** | QVQLQQW-GAGLLKPSEILSLTCAVYG-GSFS | G------YYWS | WIRQPPGKGLEWIG | EINH----SGSTNYNPSLKS | RVTISVDTSKNQFSLKLSSVTAADTAVYYCAR | V*QWLV------------DAFDI | WGQGTMVTVSS |
| iPS:43 6290 | 21-225_205G3 | VH4\|4-34/D6\|6-19\|RF3/JH3 | ............ .......... .F...... | ......H... | ......... ...... | .MY..... F.N..... .... | ...M......K....... | .G...- .-.... | ..... ..... |
| | Germline | **VH3\|3-30.3/D4\|4-17\|RF2/JH1** | QVQLVES-GGGVVQPGRSLRLSCAASG-FIFS | S------YAMH | WVRQAPGKGLEWVA | VISYD---GSNKYYADSVKG | RFTISRDNSKNTLYLQMNSLRAEDTAVYYCAR | DYGDYA------------EYFQH | WGQGTLVTVSS |
| iPS:43 6306 | 21-225_201H4 | VH3\|3-30.3/D4\|4-17\|RF2/JH1 | ............ .......... ........ | .......... | ......... ...... | A.W..... .....N.. .... | ...............M.. | .V.TVG............ ......AT..DC | ..P... ..... |
| | Germline | **VH1\|1-18/D1\|1-1\|RF1/JH6** | QVQLVQS-GAEVKKPGASVKVSCKASG-YIFT | S------YGIS | WVRQAPGQGLEWMG | WISAY---NGNTNYAQKLQG | RVTMTTDTSTSTAYMELRSLRSDDTAVYYCAR | GTTGTYY------------YYYGMDV | WGQGTTVTVSS |
| iPS:43 6362 | 21-225_210C12 | VH1\|1-18/D1\|1-1\|RF1/JH6 | ............ T........... ........ | N.....N... | ......... ...... | ...N.... ..H..... .F.. | ................. | DP.V.H............ ............ | ..... ..... |
| iPS:43 6374 | 21-225_211C10 | VH1\|1-18/D1\|1-1\|RF1/JH6 | ............ .......... ........ | R.....H... | ...L.... ...... | ........ ..L..... .F.. | .............G.... | DP.V.H............ ............ | ..... ..... |
| | Germline | | H_FR1 | H_CDR1 | H_FR2 | H_CDR2 | H_FR3 | H_CDR3 | H_FR4 |

| | | VH3\|3-33/D5\|5-18\|RF3/JH6 | QVQLVES-GGGVVQPGRSLR _SCAASG-FTFS | S-----YGMH | WVRQAPGK GLEWVA | VIWYD--- GSNKYYAD SVKG | RFTISRDNSKNTLYLQM NSLRAEDTAVYYCAR | GYSYGYYY---------- -----YYYGMDV | WGQGTT VTVSS |
|---|---|---|---|---|---|---|---|---|---|
| iPS:43 6366 | 21-225_211A3 | VH3\|3-33/D5\|5-18\|RF3/J H6 | ............ ............ V...I... | .......... | .A...... ...... | ........ ....N.E. .... | ................ | DG....-- ...............-- D.... | ...... ..... |
| iPS:43 6388 | 21-225_212H11 | VH3\|3-33/D5\|5-18\|RF3/J H6 | ............ ............ V...I... | .......... | .A...... ...... | ........ ....N.E. .... | ................ | DG....-- ...............-- D.... | ...... ..... |
| iPS:43 6396 | 21-225_213E5 | VH3\|3-33/D5\|5-18\|RF3/J H6 | ............ ............ V...I... | .......... | .A...... ...... | ........ ....N.E. .... | ................ | DG....-- ...............-- D.... | ...... ..... |
| iPS:43 6454 | 21-225_217B10 | VH3\|3-33/D5\|5-18\|RF3/J H6 | ............ ............ V...I... | .......... | .A...... ...... | ........ ....N.E. .... | ................ | DG....-- ...............-- D.... | ...... ..... |
| iPS:43 6668 | 21-225_147B9 | VH3\|3-33/D5\|5-18\|RF3/J H6 | ............ ............ ........ | .......... | ........ ...... | ........ ........ .... | ................ | DRD..DPPY......... ....Y....... | ...... ..... |
| iPS:43 6688 | 21-225_148C8 | VH3\|3-33/D5\|5-18\|RF3/J H6 | ............ ............ ........ | .......... | ........ ...... | ........ ........ .... | ................ | DRD..DPPY......... ....Y....... | ...... ..... |
| iPS:43 6706 | 21-225_149A11 | VH3\|3-33/D5\|5-18\|RF3/J H6 | ............ ............ ........ | .......... | ........ ...... | ........ ........ .... | ................ | DRD..DPPY......... ....Y....... | ...... ..... |
| iPS:43 6760 | 21-225_155E10 | VH3\|3-33/D5\|5-18\|RF3/J H6 | ............ ............ ........ | .......... | ........ ...... | ........ ........ .... | ................ | DRD..DPPY......... ....Y....... | ...... ..... |
| iPS:43 6966 | 21-225_190C3 | VH3\|3-33/D5\|5-18\|RF3/J H6 | ............ ............ ........ | .......... | ........ ...... | ........ ........ .... | ...............N | W.Y.Y--- ...............-- ..... | ...... ..... |
| iPS:43 6976 | 21-225_190D8 | VH3\|3-33/D5\|5-18\|RF3/J H6 | ..........D. ...........E ........ | ........L. | ........ ...... | ........ ........ .... | ...............N | W.Y.Y--- ...............-- ..... | ...... ..... |
| iPS:43 7168 | 21-225_218G4 | VH3\|3-33/D5\|5-18\|RF3/J H6 | ............ ............ ........ | ........L. | ........ ...... | ........ ........ .... | ....V.........N | W.Y.Y--- ...............-- ..... | ...... ..... |

| | Germline | H_FR1 | H_CDR1 | H_FR2 | H_CDR2 | H_FR3 | H_CDR3 | H_FR4 |
|---|---|---|---|---|---|---|---|---|
| | VH3\|3-33/D5\|5-24\|RF3/JH5 | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | RDGYNY-------NWFDP | WGQGTL VTVSS |
| IPS:43 6368 | 21-225_211G3 VH3\|3-33/D5\|5-24\|RF3/JH5 | ........... ........... ........ | N......... | ........ ...... | ...H.... .... | ................ | L.YS............ .......G.... | ...... ..... |
| IPS:43 6426 | 21-225_215C7 VH3\|3-33/D5\|5-24\|RF3/JH5 | ........... ........... ........ | Y......... | ........ ...... | ...H.... .... | ................ | L.YS............ .......G.... | ...... ..... |
| IPS:43 6432 | 21-225_215H12 VH3\|3-33/D5\|5-24\|RF3/JH5 | ........... ........... ........ | N......... | ........ ...... | ...H.... .... | ................ | L.YS............ .......G.... | ...... ..... |
| | Germline | H_FR1 | H_CDR1 | H_FR2 | H_CDR2 | H_FR3 | H_CDR3 | H_FR4 |
| | VH1\|1-18/D4\|4-11\|RF2/JH6 | QVQLVQS-GAEVKKPGASVK VSCKASG-YTFT | S-----YGIS | WVRQAPGQ GLEWMG | WISAY--- NGNTNYAQ KLQG | RVTMTTDTSTSTAYMEL RSLRSDDTAVYYCAR | DYSNYYY----------- -----YYYGMDV | WGQGTT VTVSS |
| IPS:43 6402 | 21-225_213H12 VH1\|1-18/D4\|4-11\|RF2/JH6 | ........... ........... ....F... | .........N | ........ ...... | ...VH... ....D... .F.. | ................ | ..YY--- -------------------- -.... | .....K ..... |
| IPS:43 6500 | 21-225_222H3 VH1\|1-18/D4\|4-11\|RF2/JH6 | ........... ........... ....F... | .........N | ........ ...... | ...V.... .... | ................ | ..YY--- --------------------- -.F.. | ...... ..... |
| IPS:43 6520 | 21-225_223G10 VH1\|1-18/D4\|4-11\|RF2/JH6 | ........... ........... ....F... | .........N | ........ ...... | ...V.... S....... .... | ................ | ..YY--- --------------------- -.... | ...... ..... |
| | Germline | H_FR1 | H_CDR1 | H_FR2 | H_CDR2 | H_FR3 | H_CDR3 | H_FR4 |
| | VH3\|3-48/D2\|2-21\|RF1/JH4 | EVQLVES-GGGLVQPGGSLR LSCAASG-FTFS | S-----YSMN | WVRQAPGK GLEWVS | YISSS--- SSTIYYAD SVKG | RFTISRDNAKNSLYLQM NSLRDEDTAVYYCAR | SILWW*L----------- ----LFYFDY | WGQGTL VTVSS |
| IPS:43 6436 | 21-225_216F10 VH3\|3-48/D2\|2-21\|RF1/JH4 | ........... ........... .....S.R | .......... | ........ ...... | ..TG.... .... | ................ | .G.A--- .................- -VE.. | ...... ..... |
| | Germline | H_FR1 | H_CDR1 | H_FR2 | H_CDR2 | H_FR3 | H_CDR3 | H_FR4 |
| | VH3\|3-48/D6\|6-6\|RF1/JH4 | EVQLVES-GGGLVQPGGSLR LSCAASG-FTFS | S-----YSMN | WVRQAPGK GLEWVS | YISSS--- SSTIYYAD SVKG | RFTISRDNAKNSLYLQM NSLRDEDTAVYYCAR | EYSSS------------- ------SYFDY | WGQGTL VTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:43 6448 | 21-225_217A3 | VH3\|3-48/D6\|6-6\|RF1/JH4 | ............ ............ ........ | .......N.. | ........ | ........ RNI..... .... | ......E......S... D............. | DG.Y.SG............ .....WYWG... | ...... ..... |
| | Germline | | H_FR1 | H_CDR1 | H_FR2 | H_CDR2 | H_FR3 | H_CDR3 | H_FR4 |
| | VH4\|4-59/D6\|6-19\|RF3/JH6 | | QVQLQES-GPGLVKPSETLS LTCTVSG-GSIS | S-----YYWS | WIRQPPGK GLEWIG | YIYY---- SGSTNYNP SLKS | RVTISVDTSKNQFSLKL SSVTAADTAVYYCAR | V*QWLVYY---------- -----YYYGMDV | WGQGTT VTVSS |
| iPS:43 6472 | 21-225_220E1 | VH4\|4-59/D6\|6-19\|RF3/JH6 | ............ ............ ........ | T......... | ........ | ........ ..T..... .... | ................. | DQ....RGR......... ...DN....... | ...... ..... |
| | Germline | | H_FR1 | H_CDR1 | H_FR2 | H_CDR2 | H_FR3 | H_CDR3 | H_FR4 |
| | VH3\|3-23/D2\|2-15\|RF3/JH4 | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | DIVVVVA---------- ------ATYFDY | WGQGTL VTVSS |
| iPS:43 6504 | 21-225_222H4 | VH3\|3-23/D2\|2-15\|RF3/JH4 | ........... ........ | N.....F... | ........ | S.V..... ..R..... .... | ................. | .PYR.AV........... .......GA... | I.... |
| iPS:43 6510 | 21-225_222H8 | VH3\|3-23/D2\|2-15\|RF3/JH4 | ........... ........ | N.....F... | ........ | S.V..... ..R..... .... | ................. | .PYR.AV........... .......GA... | I.... |
| | Germline | | H_FR1 | H_CDR1 | H_FR2 | H_CDR2 | H_FR3 | H_CDR3 | H_FR4 |
| | VH3\|3-23/D3\|3-22\|RF2/JH1 | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | YYYDSSGY---------- ----YYAEYFQH | WGQGTL VTVSS |
| iPS:43 6526 | 21-225_224A1 | VH3\|3-23/D3\|3-22\|RF2/JH1 | ...V........ ........ | .......... | ........ | ....R... A... | ................. | GS.....- ...............- .YH.LD. | ...... |
| | Germline | | H_FR1 | H_CDR1 | H_FR2 | H_CDR2 | H_FR3 | H_CDR3 | H_FR4 |
| | VH1\|1-02/D4\|4-23\|RF2/JH6 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | DYGGNSYY---------- -----YYYGMDV | WGQGTT VTVSS |
| iPS:43 6536 | 21-225_224G1 | VH1\|1-02/D4\|4-23\|RF2/JH6 | ............ ............ ........ | .........I. | ........ | ....Y... ..D..... .... | ................. | .W..Y.S- .................... | ...... |
| iPS:43 6548 | 21-225_224A7 | VH1\|1-02/D4\|4-23\|RF2/JH6 | ............ ............ ........ | .........I. | ........ | ...Y... ..D..... .... | ..........T...... | .W..Y.S- .................... | ...... |

| | | | | | | | | | |
|---|---|---|---|---|---|---|---|---|---|
| iPS:43 6558 | 21-225_224C11 | VH1\|1-02/D4\|4-23\|RF2/JH6 | ............<br>............<br>........ | ........I. | ........<br>......<br>.... | ....Y...<br>..D.....<br>.... | ................<br>R...F.....F....<br>.... | .W..Y.S-<br>................F<br>.... | ......<br>.... |
| iPS:43 6562 | 21-225_224H11 | VH1\|1-02/D4\|4-23\|RF2/JH6 | ............<br>............<br>........ | ........I. | ....T...<br>......<br>.... | ....Y...<br>..D.....<br>.... | ................<br>R...F.....F....<br>.... | .W..Y.S-<br>................<br>.... | ......<br>.... |
| iPS:43 6572 | 21-225_225G4 | VH1\|1-02/D4\|4-23\|RF2/JH6 | ............<br>............<br>........ | ........I. | ........<br>......<br>.... | ....Y...<br>..D.....<br>.... | ................<br>R...F.....F....<br>.... | .W..Y.S-<br>................<br>.... | ......<br>.... |
| iPS:43 6606 | 21-225_226G8 | VH1\|1-02/D4\|4-23\|RF2/JH6 | ............<br>............<br>........ | ........I. | ........<br>......<br>.... | ....Y...<br>..D.K...<br>.... | ................<br>....F.....F....<br>.... | .W..Y.S-<br>................<br>.... | ......<br>.... |
| iPS:43 6610 | 21-225_226F9 | VH1\|1-02/D4\|4-23\|RF2/JH6 | ............<br>............<br>........ | ........I. | ........<br>......<br>.... | ....Y...<br>..D.K...<br>.... | ......A.........<br>....F.....F....<br>.... | .W..Y.S-<br>................<br>.... | ......<br>.... |
| iPS:43 6612 | 21-225_226H9 | VH1\|1-02/D4\|4-23\|RF2/JH6 | ............<br>............<br>........ | ........I. | ........<br>......<br>.... | ....Y...<br>..D..S..<br>.... | ................<br>....F.....F....<br>.... | .W..Y.S-<br>................<br>.... | ......<br>.... |
| iPS:43 6614 | 21-225_226F10 | VH1\|1-02/D4\|4-23\|RF2/JH6 | .........G..<br>..LR........<br>........ | ........I. | ........<br>......<br>.... | ....Y...<br>..D.....<br>.... | .........V......<br>....L.....F....<br>.... | .W..Y.S-<br>................<br>.... | .....P<br>.... |
| iPS:43 6618 | 21-225_226E11 | VH1\|1-02/D4\|4-23\|RF2/JH6 | ............<br>............<br>........ | ........I. | ........<br>......<br>.... | ....Y...<br>..D.....<br>.... | ................<br>....F.....F....<br>.... | .W..Y.S-<br>................<br>.... | ......<br>.... |
| iPS:43 6624 | 21-225_226H12 | VH1\|1-02/D4\|4-23\|RF2/JH6 | ............<br>............<br>........ | ........I. | ........<br>......<br>.... | ....Y...<br>..D.....<br>.... | ................<br>R...F.....F....<br>.... | .W..Y.S-<br>................<br>.... | ......<br>.... |
| iPS:43 6626 | 21-225_227C1 | VH1\|1-02/D4\|4-23\|RF2/JH6 | ............<br>............<br>........ | ........T. | ........<br>......<br>.... | ....Y...<br>........<br>.... | ................<br>....F.........<br>.... | .W..Y.S-<br>................<br>.... | ......<br>.... |
| iPS:43 6628 | 21-225_227F2 | VH1\|1-02/D4\|4-23\|RF2/JH6 | ..H.........<br>............<br>........ | ........I. | ........<br>......<br>.... | ....Y...<br>..D.K...<br>.... | ................<br>....F.....F....<br>.... | .W..Y.S-<br>................<br>.... | ......<br>.... |
| iPS:43 6640 | 21-225_227A8 | VH1\|1-02/D4\|4-23\|RF2/JH6 | ............<br>............<br>........ | ........I. | ........<br>......<br>.... | ....Y...<br>..D.....<br>.... | .........V......<br>....F.....F....<br>.... | .W..Y.S-<br>................<br>.... | ......<br>.... |

| iPS | Sample | Germline | H_FR1 | H_CDR1 | H_FR2 | H_CDR2 | H_FR3 | H_CDR3 | H_FR4 |
|---|---|---|---|---|---|---|---|---|---|
| | | VH4\|4-39/D4\|4-17\|RF1/JH4 | QLQLQES-GPGLVKPSETLS LTCTVSG-GSIS | SS---SYYWG | WIRQPPGK GLEWIG | SIYY---- SGSTYYNP SLKS | RVTISVDTSKNQFSLKL SSVTAADTAVYYCAR | *LR*L-------------- ---------YFDY | WGQGTL VTVSS |
| iPS:43 6538 | 21-225_224C3 | VH4\|4-39/D4\|4-17\|RF1/JH4 | ........... ..S........ ........ | R........ | ........ ...... | N....... ....... .... | ................ | QG.DW............ ........GV.. | G..... ..... |
| | | VH3\|3-23/D4\|4-11\|RF2/JH5 | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | DYSNY------------- --------NWFDP | WGQGTL VTVSS |
| iPS:43 6546 | 21-225_224D6 | VH3\|3-23/D4\|4-11\|RF2/JH5 | ...V........ ............ ....S... | ......D... | ........ ...... | ........ .DN.F... .... | ................ | V..A.D........... ......SH.... | ...... |
| | | VH1\|1-46/D6\|6-6\|RF2/JH6 | QVQLVQS-GAEVKKPGASVK VSCKASG-YITFT | S-----YYMH | WVRQAPGQ GLEWMG | IINPS--- GGSTSYAQ KFQG | RVTMTRDTSTSTVYMEL SSLRSEDTAVYYCAR | SIAARYY--------- -----YYYGMDV | WGQGTT VTVSS |
| iPS:43 6568 | 21-225_225B3 | VH1\|1-46/D6\|6-6\|RF2/JH6 | ............ ............ ........ | .......... | ........ ...... | ........ ....... .... | ................ .....A......... | DL...S.Y.......... .......F.... | ....A. ..... |
| | | VH4\|4-30.1/D4\|4-17\|RF2/JH6 | QVQLQES-GPGLVKPSQILS LTCTVSG-GSIS | SG---GYYWS | WIRQHPGK GLEWIG | YIYY---- SGSTYYNP SLKS | RVTISVDTSKNQFSLKL SSVTAADTAVYYCAR | DYGDYYY--------- ------YYYGMDV | WGQGTT VTVSS |
| iPS:43 6580 | 21-225_225E7 | VH4\|4-30.1/D4\|4-17\|RF2/JH6 | ............ ............ ....N... | ......H.... | ........ ...... | F....... T....... .... | ................ | EA...G- ................- ...... | ...... |
| iPS:43 6926 | 21-225_78D10 | VH4\|4-30.1/D4\|4-17\|RF2/JH6 | ............ ............ ........ | .......... | ........ ...... | ........ I..V.... .... | ................ | .AP.F-- ................-- -.... | ...... |
| | | VH3\|3-33/D4\|4-17\|RF2/JH1 | QVQLVES-GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | DYGDYA---------- -------EYFQH | WGQGTL VTVSS |
| iPS:43 6592 | 21-225_226B1 | VH3\|3-33/D4\|4-17\|RF2/JH1 | ............ ............ ........ | T......... | ........ ...... | I....... .GY..... .... | .............F... | .HY.FW........... ......SG.LT. | ...... |

| | Germline | H_FR1 | H_CDR1 | H_FR2 | H_CDR2 | H_FR3 | H_CDR3 | H_FR4 |
|---|---|---|---|---|---|---|---|---|
| | VH3|3-23/D7|7-27|RF1/JH6 | EVQLLES-GGGLVQPGGSLR LSCAASG-FTFS | S......YAMS | WVRQAPGK GLEWVS | AISGS GGSTYYAD SVKG | RFTISRDNSKNTLYLQM NSLRAEDTAVYYCAK | LTGYYY ------YYGMDV | WGQGTT VTVSS |
| iPS:43 6652 | 21-225_146B11 | VH3|3-23/D7|7-27|RF1/J H6 | .......... .......... ........ | .......... | ........ ...... | V...G... .S...... .... | .................. | WR.NPT............ ......D..... | ...... ..... |
| iPS:43 6654 | 21-225_146C11 | VH3|3-23/D7|7-27|RF1/J H6 | .......... .......... ....I... | .......... | ........ ...... | V...G... .S...... .... | .................. | WR.NPT............ ......D..... | ...... ..... |
| iPS:43 6658 | 21-225_146A2 | VH3|3-23/D7|7-27|RF1/J H6 | .......... .......... ........ | .......... | ........ ...... | V...G... .S...... .... | ..............H... | WR.NPT............ ......D..... | ...... ..... |
| iPS:43 6664 | 21-225_147E7 | VH3|3-23/D7|7-27|RF1/J H6 | .......... .......... ........ | .......... | ........ ...... | V...G... .S...... .... | ...L............F.... | WR.NPT............ ......D..... | ...... ..... |
| iPS:43 6676 | 21-225_147E11 | VH3|3-23/D7|7-27|RF1/J H6 | .......... .......... ........ | N......V.. | ........ ...... | V...G... .S...... .... | .................. | WR.NPT............ ......D..... | ...... ..... |
| iPS:43 6678 | 21-225_147B12 | VH3|3-23/D7|7-27|RF1/J H6 | .......... .......... ........ | .......... | ........ ...... | V...G... .S...... .... | .................. | WR.NPT............ ......D..... | ...... ..... |
| iPS:43 6686 | 21-225_148G6 | VH3|3-23/D7|7-27|RF1/J H6 | .......... .......... ........ | .......... | ........ ...... | V...G... .S...... .... | ...........M.H... | WR.NPT............ ......D..... | ...... ..... |
| iPS:43 6694 | 21-225_148G11 | VH3|3-23/D7|7-27|RF1/J H6 | .......... .......... ........ | .......P.. | ........ ...... | V...G... .S.A.... .... | .................. | WR.NPT............ ......D..... | ...... ..... |
| iPS:43 6700 | 21-225_149C7 | VH3|3-23/D7|7-27|RF1/J H6 | .......... .......... ........ | .......... | ........ ...... | V...G... .S...... .... | .................. | WR.NPT............ ......D..... | ...... ..... |
| iPS:43 6704 | 21-225_149C10 | VH3|3-23/D7|7-27|RF1/J H6 | .......... ........F... ........ | ......H... | ........ ...... | V...G... .S...... .... | .................. | WR.NPT............ ......D..... | ...... ..... |

| | | | | | | | | | |
|---|---|---|---|---|---|---|---|---|---|
| iPS:43 6710 | 21-225_150F6 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .D. . . . . | . . . . . . . . . . |
| iPS:43 6714 | 21-225_150H11 | VH3\|3-23/D7\|7-27\|RF1/J H6 | . . . . . . . . . . . . . . . . . . . . . . . . . . . . . . | T. . . . . . . . . | . . . . . . . . . . . . . | I. . . G . . . .S. . . . . . . . . . | . . . . . . . . . . . . . . . . . | WR.NPT. . . . . . . . . . . . . . . . . . .D. . . . . | . . . . . . . . . . |
| iPS:43 6718 | 21-225_151H5 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .D. . . . . | . . . . . . . . . . |
| iPS:43 6722 | 21-225_151H7 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .D. . . . . | . . . . . . . . . . |
| iPS:43 6724 | 21-225_151B9 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .D. . . . . | . . . . . . . . . . |
| iPS:43 6728 | 21-225_152G6 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .D. . . . . | . . . . . . . . . . |
| iPS:43 6730 | 21-225_152D7 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .DS. . . . | . . . . . . . . . . |
| iPS:43 6742 | 21-225_154C4 | VH3\|3-23/D7\|7-27\|RF1/J H6 | . . . . . . . . . . . I. . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .D. . . . . | . . . . . . . . . . |
| iPS:43 6746 | 21-225_154E10 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .D. . . . . | . . . . . . . . . . |
| iPS:43 6758 | 21-225_155C10 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .D. . . . . | . . . . . . . . . . |
| iPS:43 6938 | 21-225_146A3 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .TT. . . . . . . . . | . . . . . . . . . . . . . . . . . | WR.NPT. . . . . . . . . . . . . . . . . . .D. . . . . | . . . . . . . . . . |
| iPS:43 7250 | 21-225_148C6 | VH3\|3-23/D7\|7-27\|RF1/J H6 | . . . . . . . . . . . . . . . . . . . . . . . . . . . . . . | . . . . . . . . . | . . . . . . . . . . . . . | V . . . G . . . .S. . . . . . . . . . | . . . . . . . . . . . . . . . . . | WR.NPT. . . . . . . . . . . . . . . . . . .D. . . . . | . . . . . . . . . . |

| | | | H_FR1 | H_CDR1 | H_FR2 | H_CDR2 | H_FR3 | H_CDR3 | H_FR4 |
|---|---|---|---|---|---|---|---|---|---|
| IPS:43 7252 | 21-225_148H11 | VH3\|3-23\|D7\|7-27\|RF1\|JH6 | ............ ............ ........ | ......... | ....... ...... | V...G... .S...... .... | ................. ................. | WR.NPT........... ......D..... | ...... ..... |
| IPS:43 7282 | 21-225_207C9 | VH3\|3-23\|D7\|7-27\|RF1\|JH6 | ............ ............ ........ | ......... | ....... ...... | ........ ........ .... | ................. ................. | AG.TTCSY.......... .....Y.N.... | ...... ..... |
| | Germline | | H_FR1 | H_CDR1 | H_FR2 | H_CDR2 | H_FR3 | H_CDR3 | H_FR4 |
| | VH3\|3-48\|D4\|4-11\|RF2\|JH6 | | EVQLVES-GGGLVQPGGSLR LSCAASG-FTFS | S-----YSMN | WVRQAPGK GLEWVS | YISSS---SSTIYYAD SVKG | RFTISRDNAKNSLYLQM NSLRDEDTAVYYCAR | DYSNYYY---------- -----YYYCMDV | WGQGTT VTVSS |
| IPS:43 6660 | 21-225_146D8 | VH3\|3-48\|D4\|4-11\|RF2\|JH6 | ............ ............ ........ | N......N.. | ........ ...... | ...R.... .N.K..V. .... | ................. | .R.GS.GY.......... ....F....L.. | ...... ..... |
| IPS:43 6682 | 21-225_146A8 | VH3\|3-48\|D4\|4-11\|RF2\|JH6 | ..K......... ....E......V ........ | N......N.. | ........ ...... | ...R.... .N.K.... ..R. | ................. | .R.GS.GY.......... ....F....L.. | ...... ..... |
| IPS:43 6684 | 21-225_146B6 | VH3\|3-48\|D4\|4-11\|RF2\|JH6 | ............ ............ ........ | .......N.. | ........ ...... | ...R.... .N.KH... .... | D............. | .R.GS.GY.......... ....F....L.. | ...... ..... |
| IPS:43 6696 | 21-225_149A1 | VH3\|3-48\|D4\|4-11\|RF2\|JH6 | ............ ............ ........ | .......N.. | ........ ...... | ...R.... .N.KH... .... | ................. | .R.GS.GY.......... ....F....L.. | ...... ..... |
| IPS:43 6712 | 21-225_150F9 | VH3\|3-48\|D4\|4-11\|RF2\|JH6 | .M.......... ............ ........ | .......N.. | ....V... ...... | ...R.... .N.KH... .... | ................. | .R.GS.GY.......... ....F....L.. | ...... ..... |
| IPS:43 6762 | 21-225_156H2 | VH3\|3-48\|D4\|4-11\|RF2\|JH6 | ............ I........... ........ | N......N.. | ........ ...... | ...R.... .N.K.... .... | ................. | .R.GS.GY.......... ....F....... | ...... ..... |
| IPS:43 6820 | 21-225_179D10 | VH3\|3-48\|D4\|4-11\|RF2\|JH6 | ............ ........F... ........ | ......... | ........ .....A | G..T.... ..Q. | ...............R... | .SRKGF- ................- ...L.. | ....I. ..... |
| IPS:43 7262 | 21-225_170E4 | VH3\|3-48\|D4\|4-11\|RF2\|JH6 | ...........S ............ ........ | ......... | ........ ...... | G..K.... ..E. | ..............D... ...............R... | .SRKGF- ................- ...L.. | ...... ..... |
| | Germline | | H_FR1 | H_CDR1 | H_FR2 | H_CDR2 | H_FR3 | H_CDR3 | H_FR4 |

| | | H_FR1 | H_CDR1 | H_FR2 | H_CDR2 | H_FR3 | H_CDR3 | H_FR4 |
|---|---|---|---|---|---|---|---|---|
| VH1\|1-08/D5\|5-12\|RF1/JH6 | | QVQLVQS-GAEVKKPGASVK VSCKASG-YTFT | S-----YDIN | WVRQATGQ GLEWMG | WMNPN--- SGNTGYAQ KFQG | RVTMTRNTSISTAYMEL SSLRSEDTAVYYCAR | VDIVATIY----------- ----YYYYGMDV | WGQGTT VTVSS |
| IPS:43 6662 | 21-225_147D7 | VH1\|1-08/D5\|5-12\|RF1/J H6 | . . . . . . . . . . . / . . . . . . . . . . . / . . . . . . . . | . . . . . . . . . . | . . . . . . . . / . . . . . . | . . . . . . . . / . . . . | R . . . . . . . . . . . . . . | A . . . LVPAAI . . . . . . . . / . . PYN . . FA . . . . | . . . . . . / . . . . . |
| | Germline | H_FR1 | H_CDR1 | H_FR2 | H_CDR2 | H_FR3 | H_CDR3 | H_FR4 |
| VH1\|1-02/D3\|3-3\|RF2/JH6 | | QVQLVQS-GAEVKKPGASVK VSCKASG-YTFT | G YYMH | WVRQAPGQ GLEWMG | WINPN SGGTNYAQ KFQG | RVTMTRDTSISTAYMEL SRLRSDDTAVYYCAR | YYDFWSGYYT- ---YYYYYGMDV | WGQGTT VTVSS |
| IPS:43 6666 | 21-225_147B8 | VH1\|1-02/D3\|3-3\|RF2/JH 6 | . . . . . . . . . . . / . . . . . . . . . . . / . . . . . . . . | D . . . . . . . L . | . . . . . . . . / . . . . . . | . . D . . . . / . . . . | . . . . . . . . . . . . . . . . | DR.SG . . S.P . . . . . . . / . . . . . . . . . . . | . . . . . . / . . . . . |
| | Germline | H_FR1 | H_CDR1 | H_FR2 | H_CDR2 | H_FR3 | H_CDR3 | H_FR4 |
| VH3\|3-33/D3\|3-3\|RF2/JH6 | | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | YYDFWSGYYT--------- ---YYYYYGMDV | WGQGTT VTVSS |
| IPS:43 6672 | 21-225_147F9 | VH3\|3-33/D3\|3-3\|RF2/JH 6 | . . . . . . . . . . . / . . . . . . . . . . . / . . . . . . . . | T . . . . . . . . . | . . . . . . . . / . . . . . . | . . . .G . . . / . . D.D . . . / . . . . | . . . . . . . .I . . . . . . . / . . . . . . . . . . . . . . . . | DR.YC . .GTC . . . . . . . / . . P . . . . . . . . . | . . . . . / . . . . . |
| IPS:43 6674 | 21-225_147G9 | VH3\|3-33/D3\|3-3\|RF2/JH 6 | . . . . . . . . . . . / . . . . . . . . . . . / . . . . . . . . | T . . . . . . . . . | . . . . . . . . / . . . . . . | . . . .G . . . / . . D.D . . . / . . . . | . . . . . . .I . . . . / . . . . . . . . . . . . . . . . | DR.YC . .GSC . . . . . . . / . . P . . . . . . . . . | . . . . . / . . . . . |
| IPS:43 6690 | 21-225_148A9 | VH3\|3-33/D3\|3-3\|RF2/JH 6 | . . . . . . . . . . . / . . . . . . . . . . . / . . . . . . . . | T . . . . . . . . . | . . . . . . . . / . . . . . . | . . . .G . . . / . . D.D . . . / . . . . | . . . . . . .I . . . . / . . . . . . . . . . . . . . . . | DR.YC . .GTC . . . . . . . / . . P . . . . . . . . . | . . . . . / . . . . . |
| IPS:43 6708 | 21-225_150D3 | VH3\|3-33/D3\|3-3\|RF2/JH 6 | . . . . . . . . . . . / . . . . . . . . . . . / . . . . . . . . | T . . . . . . . . . | . . . . . . . . / . . . . . . | . . . .G . . . / . . . .D . . . / . . . . | . . . . . . . . . . . . . . . . / . . . . . . . . . . . . . . . . | DR.YC . .GTC . . . . . . . / . . P . . . . . . . . . | . . . . . . / . . . . . |
| IPS:43 6716 | 21-225_151F3 | VH3\|3-33/D3\|3-3\|RF2/JH 6 | . . . . . . . . . . . / . . . . . . . . . . . / . . . . . . . . | T . . . . . . . . . | . . . . . . . . / . . . . . . | . . . .G . . . / . . .TD . . . / . . . . | . . . . . . .I . . . . . / . . . . . . . . . . . . . . . . | DR.YC . .TSC . . . . . . . / . . P . . . . . . . . . | . . . . . / . . . . . |
| IPS:43 6738 | 21-225_153D9 | VH3\|3-33/D3\|3-3\|RF2/JH 6 | . . . . . . . . . . . / . . . . . . . . . . . / . . . . . . V . | T . . . . . . . . . | . . . . . . . . / . . . . . . | . . . .G . . . / . . . .D . . . / . . . . | . . . . . . .I . . . . . / . . . . . . . . . . . . . . . . | DR.YC . .GSC . . . . . . . / . . P . . . . . . . . . | . . . . . / . . . . . |
| IPS:43 6740 | 21-225_154C3 | VH3\|3-33/D3\|3-3\|RF2/JH 6 | . . . . . . . . . . . / . . . . . . . . . . . T / . . . . . . . . | T . . . . . . . . . | . . . . . . . . / . . . . . . | .V . .G . . . / .N . .D . . . / . . . . | . . . . . . .I . . . . . / . . . . . . . . . . . . . . . . | DR.YC . .GSC . . . . . . . / . . P . . . . . . . . . | . . . . . . / . . . . . |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 6748 | 21-225_154D11 | VH3\|3-33/D3\|3-3\|RF2/JH6 | ............<br>............<br>.....NV. | T......... | ........<br>......<br>.... | ....G...<br>....D...<br>.... | .......S......... | DR.YC..GSC........<br>..P......... | ......<br>..... |
| iPS:43 6764 | 21-225_158E9 | VH3\|3-33/D3\|3-3\|RF2/JH6 | ............<br>............<br>........ | .......... | ........<br>......<br>.... | ..S.....<br>.... | ................ | DRV.C..TSC........<br>..P......... | ......<br>..... |
| iPS:43 6774 | 21-225_161E10 | VH3\|3-33/D3\|3-3\|RF2/JH6 | ............<br>............<br>........ | .......... | ........<br>......<br>.... | ......V.<br>.... | ................ | DRV.C..TSC........<br>..P......... | ......<br>..... |
| iPS:43 6916 | 21-225_74A9 | VH3\|3-33/D3\|3-3\|RF2/JH6 | ............<br>............<br>........ | .......... | ........<br>......<br>.... | .......<br>.N..S...<br>.... | .......I.....F...<br>......D....... | DR.YC..TSC........<br>..P......... | ......<br>..... |
| iPS:43 6940 | 21-225_146B8 | VH3\|3-33/D3\|3-3\|RF2/JH6 | ............<br>............<br>........ | T......... | ........<br>......<br>.... | .V..G...<br>.ND.DF..<br>..T. | .......I........ | DR.YC..GSC........<br>..P......... | ......<br>..... |
| iPS:43 7258 | 21-225_153F9 | VH3\|3-33/D3\|3-3\|RF2/JH6 | ..H.........<br>............<br>......I. | T......... | ....G...<br>......<br>.... | ....G...<br>..DTD...<br>..R. | .......I........ | DR.YC..GNC........<br>..P......... | ......<br>..... |
| | | Germline | H_FR1 | H_CDR1 | H_FR2 | H_CDR2 | H_FR3 | H_CDR3 | H_FR4 |
| | **VH4\|4-30.1/D7\|7-27\|RF3/JH5** | | QVQLQES-GPGLVKPSQTLS LTCTVSG-GSIS | SG---GYYWS | WIRQHPGK GLEWIG | YIYY---- SGSTYYNP SLKS | RVTISVDTSKNQFSLKL SSVTAADTAVYYCAR | NWCN------------<br>-------WFDP | WGQGTL VTVSS |
| iPS:43 6680 | 21-225_147H12 | VH4\|4-30.1/D7\|7-27\|RF3/JH5 | ............<br>.N.........A<br>........ | .....Y.H.. | ........<br>...... | ........<br>.... | ................<br>T............. | D..GYDS...........<br>......SG.... | ......<br>..... |
| iPS:43 6750 | 21-225_154G12 | VH4\|4-30.1/D7\|7-27\|RF3/JH5 | ............<br>.N.........G<br>........ | .....Y.... | ........<br>...... | ........<br>.... | ..S..L..P........<br>T............. | D..GYDS...........<br>......SG.... | ......<br>..... |
| | | Germline | H_FR1 | H_CDR1 | H_FR2 | H_CDR2 | H_FR3 | H_CDR3 | H_FR4 |
| | **VH3\|3-07/D6\|6-13\|RF1/JH4** | | EVQLVES-GGGLVQPGGSLR LSCAASG-FIFS | S-----YWMS | WVRQAPGK GLEWVA | NIKQD--- GSEKYYVD SVKG | RFTISRDNAKNSLYLQM NSLRAEDTAVYYCAR | GYSSSW----------<br>-------YYFDY | WGQGTL VTVSS |
| iPS:43 6698 | 21-225_149B5 | VH3\|3-07/D6\|6-13\|RF1/JH4 | ............<br>........ | G........N | ........<br>...... | ........<br>.... | ................<br>................ | .MY..G...........<br>......W.V... | ......<br>..... |
| | | Germline | H_FR1 | H_CDR1 | H_FR2 | H_CDR2 | H_FR3 | H_CDR3 | H_FR4 |

| | | | H_FR1 | H_CDR1 | H_FR2 | H_CDR2 | H_FR3 | H_CDR3 | H_FR4 |
|---|---|---|---|---|---|---|---|---|---|
| | VH3|3-21|D6|6-19|RF2/JH5 | | EVQLVES-GGGLVKPGGSLR LSCAASG-FIFS | S-----YSMN | WVRQAPGK GLEWVS | SISSS--- SSYIYYAD SVKG | RFTISRDNAKNSLYLQM NSLRAEDTAVYYCAR | GIAVAG------------ -------NWFDP | WGQGTL VTVSS |
| iPS:43 6702 | 21-225_149E8 | VH3|3-21|D6|6-19|RF2/JH5 | ............ ............ ........ | .......... | ...R.... | A...T... G...... .... | ................. | TAVAGT........... .......G.... | ...... |
| | Germline | | H_FR1 | H_CDR1 | H_FR2 | H_CDR2 | H_FR3 | H_CDR3 | H_FR4 |
| | VH5|5-51|D3|3-22|RF2/JH6 | | EVQLVQS-GAEVKKPGESLK ISCKGSG-YSFT | S----YWIG | WVRQMPGK GLEWMG | IIYPG--- DSDTRYSP SFQG | QVTISADKSISTAYLQW SSLKASDTAMYYCAR | YYYDSSGYYY ---YYYYYGMDV | WGQGTT VTVSS |
| iPS:43 6752 | 21-225_155H1 | VH5|5-51|D3|3-22|RF2/JH6 | ............ ............ ........ | .......... | ........ | L....... A....... .... | ................. | QAIA.R.R-- ........- | ..... |
| | Germline | | H_FR1 | H_CDR1 | H_FR2 | H_CDR2 | H_FR3 | H_CDR3 | H_FR4 |
| | VH3|3-33|D6|6-19|RF1/JH4 | | QVQLVES-GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | GYSSGW------------ -------YYFDY | WGQGTL VTVSS |
| iPS:43 6772 | 21-225_161H3 | VH3|3-33|D6|6-19|RF1/JH4 | ............ ............ ........ | .......... | ........ | ........ .... | ................. | VGY..G........... ......W.I... | ..... |
| | Germline | | H_FR1 | H_CDR1 | H_FR2 | H_CDR2 | H_FR3 | H_CDR3 | H_FR4 |
| | VH4|4-30.1/D2|2-2|RF2/JH6 | | QVQLQES-GPGLVKPSQTLS LTCTVSG-GSIS | SG---GYYWS | WIRQHPGK GLEWIG | YIYY---- SGSTYYNP SLKS | RVTISVDTSKNQFSLKL SSVTAADTAVYYCAR | GYCSSTSCYT-------- ---YYYYYGMDV | WGQGTT VTVSS |
| iPS:43 6776 | 21-225_161F12 | VH4|4-30.1/D2|2-2|RF2/JH6 | ............ ............ ........ | .......... | ........ | ........ ...P.... .... | .I...I........... N.............. | SN...AN........... ..VGF....L.. | ..R... ..... |
| iPS:43 6780 | 21-225_165H3 | VH4|4-30.1/D2|2-2|RF2/JH6 | ............ ............ ........ | .......... | ........ | ........ ...P.... .... | .I...I........... N.............. | SN...AN........... ..VGF....... | ..... |
| | Germline | | H_FR1 | H_CDR1 | H_FR2 | H_CDR2 | H_FR3 | H_CDR3 | H_FR4 |
| | VH3|3-33/D5|5-24|RF3/JH6 | | QVQLVES-GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | RDGYNYYY---------- ------YYYGMDV | WGQGTT VTVSS |
| iPS:43 6784 | 21-225_169C1 | VH3|3-33/D5|5-24|RF3/JH6 | ............ ............ ......L. | ......N... | ........ | ........ .... | .......T........ ................ | DQYNRNDGPP....... ..AYY....L.. | ...... |

| | | | H_FR1 | H_CDR1 | H_FR2 | H_CDR2 | H_FR3 | H_CDR3 | H_FR4 |
|---|---|---|---|---|---|---|---|---|---|
| iPS:43 6786 | 21-225_169A6 | VH3\|3-33/D5\|5-24\|RF3/JH6 | ................ ............ ......L. | ......N... | ........ ...... | ........ .... | ...............S... .............. | DQYNRNDGPP........ ..AYY....... | ...... ..... |
| iPS:43 6796 | 21-225_170A5 | VH3\|3-33/D5\|5-24\|RF3/JH6 | ............ ............ ....L... | N.....C... | ........ ...... | I....... .... | ...............D... .............. | DQYNRNDGPP........ ..AYY....L.. | ...... ..... |
| iPS:43 6812 | 21-225_175C6 | VH3\|3-33/D5\|5-24\|RF3/JH6 | ............ ............ ....L... | N.....C... | ........ ...... | I....... .... | ...V.........D... .............. | DQYNRNDGPP........ ..AYY....L.. | ...... ..... |
| | Germline | | H_FR1 | H_CDR1 | H_FR2 | H_CDR2 | H_FR3 | H_CDR3 | H_FR4 |
| | VH3\|3-48/D5\|5-18\|RF1/JH6 | | EVQLVES-GGGLVQPGGSLR LSCAASG-FTFS | S------YSMN | WVRQAPGK GLEWVS | YISSS--- SSTIYYAD SVKG | RFTISRDNAKNSLYLQM NSLRDEDTAVYYCAR | VDTAMVYY----------------YYYCMDV | WGQGTT VTVSS |
| iPS:43 6788 | 21-225_169B7 | VH3\|3-48/D5\|5-18\|RF1/JH6 | ............ ............ ........ | ........L. | ........ ...... | ..G..... G.I.F... .... | ................ | G...G.T- .... | ...... ..... |
| iPS:43 6798 | 21-225_171F5 | VH3\|3-48/D5\|5-18\|RF1/JH6 | ............ ............ ........ | ........L. | ........ ...... | ..G..... G.I.F... .... | ................ | G...G.T- .... | ...... ..... |
| iPS:43 6864 | 21-225_58G11 | VH3\|3-48/D5\|5-18\|RF1/JH6 | ............ ............ ........ | .......... | ........ ....I. | ...T.... ....F... .... | .......S......... | G.....L- .............. | ...... ..... |
| iPS:43 6866 | 21-225_59F2 | VH3\|3-48/D5\|5-18\|RF1/JH6 | ............ ..........G ........ | .......... | ........ ...... | ...G.... .NI...T. .... | ................ | A..P..L- ...............F. .... | ...... ..... |
| iPS:43 6872 | 21-225_60D2 | VH3\|3-48/D5\|5-18\|RF1/JH6 | ............ ..........G ........ | .......... | ........ ...... | ...E.... .NI...T. .... | .........M....... | A..P..L- ...............F. .... | ...... ..... |
| | Germline | | H_FR1 | H_CDR1 | H_FR2 | H_CDR2 | H_FR3 | H_CDR3 | H_FR4 |
| | VH3\|3-33/D5\|5-24\|RF1/JH6 | | QVQLVES-GGGVVQPGRSLR LSCAASG-FTFS | S------YGMH | WVRQAPGK GLEWVA | VIWYD--- GSNKYYAD SVKG | RFTISRDNSKNTLYLQM NSLRAEDTAVYYCAR | VEMATIYY----------------YYYCMDV | WGQGTT VTVSS |
| iPS:43 6790 | 21-225_169G11 | VH3\|3-33/D5\|5-24\|RF1/JH6 | ............ ............ ........ | .......... | ........ ...... | I....... ........ .... | ................ .........G...... | EGATYYHGSGS....... .YYPATN..... | ...... ..... |
| | Germline | | H_FR1 | H_CDR1 | H_FR2 | H_CDR2 | H_FR3 | H_CDR3 | H_FR4 |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-33\|D3\|3-10\|RF1\|JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VLLWFGELL*-------- ---YYYYYYGMDV | WGQGTTVTVSS |
| iPS:43 6792 | 21-225_169D12 | VH3\|3-33\|D3\|3-10\|RF1\|JH6 | ............ ........ | .......... | ........ ...... | ........ .... | ................ ...............S | P.YDM.L--- ...........--- ..D... | ...... ..... |
| | | Germline | H_FR1 | H_CDR1 | H_FR2 | H_CDR2 | H_FR3 | H_CDR3 | H_FR4 |
| | VH1\|1-46\|D7\|7-27\|RF3\|JH4 | | QVQLVQS-GAEVKKPGASVKVSCKASG-YIFT | S-----YYMH | WVRQAPGQGLEWMG | IINPS---GGSTSYAQKFQG | RVTMTRDTSTSTVYMELSSLRSEDTAVYYCAR | NWGY---------- ---------FDY | WGQGTLVTVSS |
| iPS:43 6800 | 21-225_171D12 | VH1\|1-46\|D7\|7-27\|RF3\|JH4 | ............ ............ .......N | .........Y | ........ ...... | ........ ....N... .... | ............L.... N............S | G.E- ................ .....LN. | ...... ..... |
| iPS:43 6804 | 21-225_172C3 | VH1\|1-46\|D7\|7-27\|RF3\|JH4 | ............ ............ .......R | .........Y | ........ ....V. | T....... ....N... .... | ............L.... N............S | G.E- ................ .....LN. | ...... ..... |
| iPS:43 6806 | 21-225_172B12 | VH1\|1-46\|D7\|7-27\|RF3\|JH4 | ............ .......T.... .......R | .........Y | ........ ....V. | T....... ....D... .... | ............L.... N............S | G.E- ................ .....LN. | ...... ..... |
| | | Germline | H_FR1 | H_CDR1 | H_FR2 | H_CDR2 | H_FR3 | H_CDR3 | H_FR4 |
| | VH3\|3-30.3\|D4\|4-23\|RF1\|JH6 | | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | *LRW*LYY---------- ------YYYGMDV | WGQGTTVTVSS |
| iPS:43 6808 | 21-225_173F8 | VH3\|3-30.3\|D4\|4-23\|RF1\|JH6 | ............ ........ | .......G.. | ........ ...... | ..P..C.. .... | ................ ...............F... | DE.QW.P- ...............AP. .... | ...... |
| | | Germline | H_FR1 | H_CDR1 | H_FR2 | H_CDR2 | H_FR3 | H_CDR3 | H_FR4 |
| | VH6\|6-01\|D6\|6-6\|RF2\|JH6 | | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | SIAARYY---------- ------YYYGMDV | WGQGTTVTVSS |
| iPS:43 6810 | 21-225_175F4 | VH6\|6-01\|D6\|6-6\|RF2\|JH6 | ............ ............ ........ | .......... | ........ ...... | ........ ....A.P. .ME. | ..S............. ................ | DK..GRND.......... ....F....... | ...... ..... |
| iPS:43 6814 | 21-225_178H10 | VH6\|6-01\|D6\|6-6\|RF2\|JH6 | ............ ............ ........ | .......... | ........ ...... | ........ ...SA.P. .ME. | .VS............. ................ | DK..GRND.......... ....F....... | ...... ..... |
| | | Germline | H_FR1 | H_CDR1 | H_FR2 | H_CDR2 | H_FR3 | H_CDR3 | H_FR4 |

| | VH3|3-33/D1|1-1|RF3/JH6 | | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | YNWNDYY------------ -----YYYGMDV | WGQGTT VTVSS |
|---|---|---|---|---|---|---|---|---|---|
| iPS:43 6818 | 21-225_179C7 | VH3|3-33/D1|1-1|RF3/JH 6 | .A.......... ............ ........ | N.....S... | ....G... ...... | I.Y..... ..Y..N.. .... | ................. ................. | DRHY.FHVP......... ...YY....... | ...... ..... |
| iPS:43 7094 | 21-225_210D12 | VH3|3-33/D1|1-1|RF3/JH 6 | ............ ............ ........ | H......... | ........ ...... | ........ .... | ................. ................. | GD..P-- .................-- E.L.. | ...... ..... |
| iPS:43 7096 | 21-225_210E12 | VH3|3-33/D1|1-1|RF3/JH 6 | ..H......... ............ ........ | N......... | ........ ...... | ........ .... | ................. ................. | GD..P-- .................-- E.... | ...... .I... |
| iPS:43 7098 | 21-225_211C1 | VH3|3-33/D1|1-1|RF3/JH 6 | ............ ............ ........ | H......... | ........ ...... | ........ .... | .T.......I..... ................. | GD..P-- .................-- E.L.. | ...... ..... |
| iPS:43 7104 | 21-225_211G5 | VH3|3-33/D1|1-1|RF3/JH 6 | ............ ............ ........ | H......... | ........ ...... | ........ .... | ................. ................. | GD..P-- .................-- E.L.. | ...... ..... |
| iPS:43 7112 | 21-225_212C2 | VH3|3-33/D1|1-1|RF3/JH 6 | ............ ............ ........ | H......... | ........ ...... | ........ .... | ................. ................. | GD..P-- .................-- E.... | .....S ..... |
| iPS:43 7114 | 21-225_212A4 | VH3|3-33/D1|1-1|RF3/JH 6 | ..H......... ..A......... ........ | N......... | ........ ...... | ........ .... | ................. ................. | GD..P-- .................-- E.... | ...... .I... |
| iPS:43 7116 | 21-225_212F6 | VH3|3-33/D1|1-1|RF3/JH 6 | ............ ............ ........ | H......... | ........ ...... | ........ .... | ................. ................. | GD..P-- .................-- E.L.. | ...... ..... |
| iPS:43 7118 | 21-225_212G7 | VH3|3-33/D1|1-1|RF3/JH 6 | ............ ............ ........ | H......... | ........ ...... | .....C.. .... | ...........T....... ................. | GD..P-- .................-- E.L.. | ...... ..... |
| iPS:43 7128 | 21-225_213G3 | VH3|3-33/D1|1-1|RF3/JH 6 | ............ ............ ........ | H......... | ........ ...... | ........ .... | ..............H... ................. | GD..P-- .................-- E.... | .....S ..... |
| iPS:43 7130 | 21-225_213D5 | VH3|3-33/D1|1-1|RF3/JH 6 | ............ ............ ........ | H......... | ........ ...... | ........ .... | ..............F... ....V.......... | GD..P-- .................-- E.... | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 7146 | 21-225_215D3 | VH3\|3-33/D1\|1-1\|RF3/JH6 | .T...........<br>.............<br>........ | H......... | ........<br>...... <br>.... | ........<br>...E....<br>.... | ................. | GD..P--<br>................--<br>E.... | ......<br>..... |
| iPS:43 7150 | 21-225_216A3 | VH3\|3-33/D1\|1-1\|RF3/JH6 | .............<br>.............<br>........ | H......... | ........<br>...... <br>.... | ........<br>.........<br>.... | ................. | GD..P--<br>................--<br>E.L.. | ......<br>..... |
| iPS:43 7162 | 21-225_217B2 | VH3\|3-33/D1\|1-1\|RF3/JH6 | ..H..........<br>.............<br>........ | N......... | ........<br>...... <br>.... | ........<br>.........<br>.... | ................. | GD..P--<br>................--<br>E.... | ......<br>.I... |
| iPS:43 7172 | 21-225_219A7 | VH3\|3-33/D1\|1-1\|RF3/JH6 | .............<br>............P<br>........ | H......... | ........<br>...... <br>.... | ........<br>.........<br>.... | ...............M..... | GD..P--<br>................--<br>E.... | ......<br>..... |
| iPS:43 7182 | 21-225_221H2 | VH3\|3-33/D1\|1-1\|RF3/JH6 | .............<br>.............<br>........ | H......... | ........<br>...... <br>.... | ........<br>.........<br>.... | ..............H... | GD..P--<br>................--<br>E.... | ......<br>..... |
| iPS:43 7184 | 21-225_221G4 | VH3\|3-33/D1\|1-1\|RF3/JH6 | .............<br>.............<br>........ | H......... | ........<br>...... <br>.... | ........<br>.........<br>.... | ..............H... | GD..P--<br>................--<br>E.... | .....S<br>..... |
| iPS:43 8664 | 21-225_216G1 | VH3\|3-33/D1\|1-1\|RF3/JH6 | ..H..........<br>.............<br>........ | N......... | ........<br>...... <br>.... | ........<br>.........<br>.... | ................. | GD..P--<br>................--<br>E.... | ......<br>.I... |
| | VH3\|3-33/D1\|1-1\|RF1/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GTTGT----------------YFDY | WGQGTLVTVSS |
| iPS:43 6822 | 21-225_180D4 | VH3\|3-33/D1\|1-1\|RF1/JH4 | .............<br>.............<br>........ | N.....F... | ........<br>...... <br>.... | I.......<br>..D.....<br>.... | ................. <br>.T.........F... | .GPPFST...........<br>.....VTM.... | ......<br>..... |
| iPS:43 6828 | 21-225_181H1 | VH3\|3-33/D1\|1-1\|RF1/JH4 | .............<br>.............<br>........ | N......... | ........<br>...... <br>.... | I.......<br>..D.....<br>.... | .I............... <br>.T.........F... | .GPPFST...........<br>.....VTM.... | ......<br>..... |
| iPS:43 6950 | 21-225_184G4 | VH3\|3-33/D1\|1-1\|RF1/JH4 | .............<br>.............<br>V....... | ......... | ........<br>...... <br>.... | I.......<br>.........<br>.... | ................. | .GPPFST...........<br>.....VTM.... | ......<br>..... |

| | | | H_FR1 | H_CDR1 | H_FR2 | H_CDR2 | H_FR3 | H_CDR3 | H_FR4 |
|---|---|---|---|---|---|---|---|---|---|
| iPS:43 6952 | 21-225_185D2 | VH3\|3-33/D1\|1-1\|RF1/JH4 | ............. ............. ........ | N......... | ........ ...... .... | I....... ..D..... | ................. .T..........F... | .GPPFST........... .....VTM.... | ...... ..... |
| | | Germline | H_FR1 | H_CDR1 | H_FR2 | H_CDR2 | H_FR3 | H_CDR3 | H_FR4 |
| | VH2\|2-05/D6\|6-6\|RF2/JH3 | | QITLKES-GPTLVKPTQILT LTCIFSG-FSLS | TS---GVGVG | WIRQPPGK ALEWLA | LIYW---- NDDKRYSP SLKS | RLTITKDTSKNQVVLTM TNMDPVDTATYYCAH | SIAAR------------- ------DAFDI | WGQGTM VTVSS |
| iPS:43 6824 | 21-225_180C5 | VH2\|2-05/D6\|6-6\|RF2/JH3 | ............. ............. ........ | .......... | ........ .....G .... | F.S..... | S.............I. ................. | KA..V............. .......-.... | ...... ..... |
| iPS:43 6956 | 21-225_186H6 | VH2\|2-05/D6\|6-6\|RF2/JH3 | ............. ............. ........ | .......... | ........ .....G .... | F.S..... | S................ ................. | KA..V............. .......-.... | ...... ..... |
| | | Germline | H_FR1 | H_CDR1 | H_FR2 | H_CDR2 | H_FR3 | H_CDR3 | H_FR4 |
| | VH1\|1-08/D5\|5-18\|RF3/JH6 | | QVQLVQS-GAEVKKPGASVK VSCKASG-YIFT | S-----YDIN | WVRQATGQ GLEWMG | WMNPN--- SGNTGYAQ KFQG | RVTMTRNTSISTAYMEL SSLRSEDTAVYYCAR | GYSYGYYY---------- -----YYYGMDV | WGQGTT VTVSS |
| iPS:43 6826 | 21-225_180G5 | VH1\|1-08/D5\|5-18\|RF3/JH6 | ............. ............. ........ | .......... | ........ ...... .... | ........ | ................. ................. | .FY.YGSGSHV....... ..PYH....L.. | ...... ..... |
| | | Germline | H_FR1 | H_CDR1 | H_FR2 | H_CDR2 | H_FR3 | H_CDR3 | H_FR4 |
| | VH6\|6-01/D5\|5-24\|RF3/JH2 | | QVQLQQS-GPGLVKPSQILS LTCAISG-DSVS | SN---SAAWN | WIRQSPSR GLEWLG | RIYYR-- SKWYNDYA VSVKS | RITINPDTSKNQFSLQL NSVTPEDTAVYYCAR | RDGYNY------------ -----YWYFDL | WGRGTL VTVSS |
| iPS:43 6832 | 21-225_51D8 | VH6\|6-01/D5\|5-24\|RF3/JH2 | ............. ............. ........ | .......... | ........ ...... .... | ........ | ...F...........R. ................. | DRYNWNY........... ......P...... | ...... ..... |
| | | Germline | H_FR1 | H_CDR1 | H_FR2 | H_CDR2 | H_FR3 | H_CDR3 | H_FR4 |
| | VH1\|1-02/D5\|5-24\|RF3/JH5 | | QVQLVQS-GAEVKKPGASVK VSCKASG-YIFT | G------YYMH | WVRQAPGQ GLEWMG | WINPN--- SGGTNYAQ KFQG | RVTMTRDTSISTAYMEL SRLRSDDTAVYYCAR | RDGYNY------------ -------NWFDP | WGQGTL VTVSS |
| iPS:43 6840 | 21-225_53E9 | VH1\|1-02/D5\|5-24\|RF3/JH5 | ............. ............. ....N... | .......... | ........ ...... .... | ..I..... ..D..... | ................. ................. | DGYSSGW........... ......F..... | ...... ..... |
| | | Germline | H_FR1 | H_CDR1 | H_FR2 | H_CDR2 | H_FR3 | H_CDR3 | H_FR4 |
| | VH2\|2-05/D7\|7-27\|RF1/JH4 | | QITLKES-GPTLVKPTQILT LTCIFSG-FSLS | TS---GVGVG | WIRQPPGK ALEWLA | LIYW---- NDDKRYSP SLKS | RLTTKDTSKNQVVLTM TNMDPVDTATYYCAH | LTGY-------------- --------FDY | WGQGTL VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 6848 | 21-225_57F1 | VH2\|2-05/D7\|7-27\|RF1/JH4 | ............ ............ ........ | .......... | ........ ...... | ....... HE...... .... | ......E.......D... ...A........... | V..I............... ........AAP. | ...... ..... |
| iPS:43 6852 | 21-225_57H11 | VH2\|2-05/D7\|7-27\|RF1/JH4 | ............ ............ .......T | .......... | ........ ...... | ....... HE.R.... .... | ......E.......D... ...A........... | V..I............... ........AAP. | ...... ..... |
| iPS:43 6870 | 21-225_60B1 | VH2\|2-05/D7\|7-27\|RF1/JH4 | ............ ............ ........ | .......... | ........ ...... | ....... HE...... .... | ......E.......D... ...A........... | V.YI............... ........AAP. | ...... ..... |
| iPS:43 6876 | 21-225_61F5 | VH2\|2-05/D7\|7-27\|RF1/JH4 | ............ ............ ........ | ......L... | ........ ...... | ...S.... HE...... .... | ......E.......D... ...A........... | V..I............... ........AAP. | ...... ..... |
| iPS:39 2593 | 21-225_3E10 | VH2\|2-05/D7\|7-27\|RF1/JH4 | ............ ............ .......N | .G........ | ........ ...... | ........ ......H.. .... | ................. .H.A........... | .IEV............... ........A... | ...... ..... |
| | Germline | | H_FR1 | H_CDR1 | H_FR2 | H_CDR2 | H_FR3 | H_CDR3 | H_FR4 |
| VH2\|2-05/D6\|6-6\|RF2/JH1 | | | QITLKES-GPTLVKPTQTLTLTCIFSG-FSLS | TS---GVGVG | WIRQPPGKALEWLA | LIYW----NDDKRYSPSLKS | RLTITKDTSKNQVVLTMTNMDPVDTATYYCAH | SIAARA-----------EYFQH | WGQGTLVTVSS |
| iPS:43 6854 | 21-225_58C1 | VH2\|2-05/D6\|6-6\|RF2/JH1 | .......... .......... ........ | .......... | .......... ...... | ........ D....... .... | ......E........... | L..V-- .-A.DS | ...... |
| iPS:43 6874 | 21-225_60A12 | VH2\|2-05/D6\|6-6\|RF2/JH1 | .......... .......... ........ | .......... | .......... ...... | ........ D....... .... | ......E........... | L..V-- .-A.DS | ...... |
| iPS:43 6954 | 21-225_185G7 | VH2\|2-05/D6\|6-6\|RF2/JH1 | .......... .......... .......T | .G........ | ........ ...... | ........ ...E.... .... | ................. | I..V-- .-A... | ...... |
| iPS:39 3188 | 21-225_34B9 | VH2\|2-05/D6\|6-6\|RF2/JH1 | ....R....... .......... ........ | .......... | ........ ...... | ........ ........ .... | ................. | L..V-- .-T.DS | ....S. ..... |
| | Germline | | H_FR1 | H_CDR1 | H_FR2 | H_CDR2 | H_FR3 | H_CDR3 | H_FR4 |
| VH3\|3-30.3/D3\|3-10\|RF2/JH6 | | | QVQLVES-GGGVVQPGRSLRLSCAASG-FIFS | S-----YAMH | WVRQAPGKGLEWVA | VISYD---GSNKYYADSVKG | RFTISRDNSKNTLYLQMNSLRAEDTAVYYCAR | YYYGSGSYYN----YYYYYGMDV | WGQGTTVTVSS |

EP 4 435 105 A2

| iPS:43 6858 | 21-225_58E7 | VH3|3-30.3/D3|3-10|RF2/JH6 | ............ ............ ........ | F......G.. | ........ ...... .... | .T...... ..D..... | ..S.............. S........M..... | DD.....P-- ...............-- L...... | ...... .... |

| Germline | H_FR1 | H_CDR1 | H_FR2 | H_CDR2 | H_FR3 | H_CDR3 | H_FR4 |
|---|---|---|---|---|---|---|---|
| VH3|3-07/D5|5-12|RF3/JH6 | EVQLVES-GGGLVQPGGSLRLSCAASG-FTFS | S-----YWMS | WVRQAPGKGLEWVA | NIKQD---GSEKYYVDSVKC | RFTISRDNAKNSLYLQMNSLRAEDTAVYYCAR | GYSGYDYY------------YYYYGMDV | WGQGTTVTVSS |
| iPS:43 6860 — 21-225_58F7 — VH3|3-07/D5|5-12|RF3/JH6 | ............ ............ ........ | ......F... | ......... ...... .... | H....... ......... .... | ................. | .DLP.SSG.......... ....-........ | ...... .... |

| Germline | H_FR1 | H_CDR1 | H_FR2 | H_CDR2 | H_FR3 | H_CDR3 | H_FR4 |
|---|---|---|---|---|---|---|---|
| VH3|3-30.3/D4|4-17|RF2/JH6 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYGDYYY-----------YYYGMDV | WGQGTTVTVSS |
| iPS:43 6862 — 21-225_58F8 — VH3|3-30.3/D4|4-17|RF2/JH6 | ............ ............ ........ | .......G.. | ......... ...... .... | ......... ......... .... | ................. | .E.RG.GGYER....... .GYYY....... | ...... .... |

| Germline | H_FR1 | H_CDR1 | H_FR2 | H_CDR2 | H_FR3 | H_CDR3 | H_FR4 |
|---|---|---|---|---|---|---|---|
| VH3|3-33/D3|3-16|RF2/JH6 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYDYVWGSYRY--------TYYYYYGMDV | WGQGTTVTVSS |
| iPS:43 6868 — 21-225_59B11 — VH3|3-33/D3|3-16|RF2/JH6 | ............ ............ ........ | .......V. | ......... ...... .... | A....... ......... .... | .................L. | DR..CSS.SC- .........P........ . | ...... |

| Germline | H_FR1 | H_CDR1 | H_FR2 | H_CDR2 | H_FR3 | H_CDR3 | H_FR4 |
|---|---|---|---|---|---|---|---|
| VH2|2-05/D1|1-1|RF1/JH3 | QITLKES-GPTLVKPTQILTLTCTFSG-FSLS | TS---GVGVG | WIRQPPGKALEWLA | LIYW----NDDKRYSPSLKS | RLTITKDTSKNQVVLTMTNMDPVDTATYYCAH | GTTGT-------------DAFDI | WGQGTMVTVSS |
| iPS:43 6878 — 21-225_62E3 — VH2|2-05/D1|1-1|RF1/JH3 | .......... .......... ........ | .......... | ......... ...... | ..N..... ......... .... | .F...R....D...... | KA.WV............. .......-.... | ...... .... |
| iPS:43 6880 — 21-225_62E8 — VH2|2-05/D1|1-1|RF1/JH3 | .......... .......... ........ | .......... | ......... ...... | ..N..... ......... .... | .F...R....D...... | KA.WV............. .......-.... | ...... .... |
| iPS:43 6882 — 21-225_62D10 — VH2|2-05/D1|1-1|RF1/JH3 | .......... ..S....... ........ | .......... | ......... ...... | ..N..... ......... .... | .F...R....D...... | KA.WV............. .......-.... | ...... |

| IPS ID | Name | Germline | H_FR1 | H_CDR1 | H_FR2 | H_CDR2 | H_FR3 | H_CDR3 | H_FR4 |
|---|---|---|---|---|---|---|---|---|---|
| IPS:43 6884 | 21-225_62A12 | VH2|2-05/D1|1-1|RF1/JH3 | ............ ............ ........ | .......... | ......... ...... .... | ..N..... | .F...R....D...... .....L | K..WV............ .......-.... | ...... ..... |
| IPS:43 6886 | 21-225_62B12 | VH2|2-05/D1|1-1|RF1/JH3 | ............ ............ .......N | .......... | ......... ...... .... | ..N..... | .F...R....D...... | KA.WV............ .......-.... | ...... ..... |
| IPS:43 6894 | 21-225_66G9 | VH2|2-05/D1|1-1|RF1/JH3 | ............ ............ ........ | .......... | ......... ...... .... | ..N..... .....F.. | .F...R....D...... | KA.WV............ .......-.... | ...... ..... |
| IPS:43 6908 | 21-225_72D5 | VH2|2-05/D1|1-1|RF1/JH3 | ............ ............ ........ | .......... | ......... ...... .... | ..N..... | .F...R....D...F.. ........G..... | KA.WV............ .......-.... | ...... ..... |
| IPS:43 6912 | 21-225_73C4 | VH2|2-05/D1|1-1|RF1/JH3 | ............ ............ ........ | .......... | ......... ...... .... | ..N..... | .F...R....D...... | K..WV............ .......-.... | ...... ..... |
| | | Germline | H_FR1 | H_CDR1 | H_FR2 | H_CDR2 | H_FR3 | H_CDR3 | H_FR4 |
| | VH3|3-48/D4|4-11|RF2/JH3 | | EVQLVES-GGGLVQPGGSLRLSCAASC-FTFS | S-----YSMN | WVRQAPGKGLEWVS | YISSS---SSTIYYADSVKC | RFTISRDNAKNSLYLQMNSLRDEDTAVYYCAR | DYSNY------------DAFDI | WGQGTMVTVSS |
| IPS:43 6888 | 21-225_63G7 | VH3|3-48/D4|4-11|RF2/JH3 | ............ ............ ........ | .......... | ......... ...... .... | ........ T......A | ................ | .HRY.DSSG......... ....YYS..... | ...... ..... |
| IPS:43 6890 | 21-225_63A10 | VH3|3-48/D4|4-11|RF2/JH3 | ............ ............ ........ | .......... | ......... ...... .... | ........ T......A | ................ | .HRY.DSSG......... ....YYS..... | ...... ..... |
| | | Germline | H_FR1 | H_CDR1 | H_FR2 | H_CDR2 | H_FR3 | H_CDR3 | H_FR4 |
| | VH1|1-02/D3|3-22|RF2/JH3 | | QVQLVQS-GAEVKKPGASVKVSCKASG-YIFT | G-----YYMH | WVRQAPGQGLEWMG | WINPN---SGGTNYAQKFQG | RVTMTRDTSISTAYMELSRLRSDDTAVYYCAR | YYYDSSGY---------YYDAFDI | WGQGTMVTVSS |
| IPS:43 6892 | 21-225_65E9 | VH1|1-02/D3|3-22|RF2/JH3 | ............ ............ ........ | .......... | ......... ...... .... | ........ | ................ | A..YG..S.......... .......NE..M | ...... ..... |
| IPS:43 6900 | 21-225_69B9 | VH1|1-02/D3|3-22|RF2/JH3 | ...M.....D.. ............ ........ | .......H.. | ......... ...... .... | ........ | ..M............. | A..YG..S.......... .......NES.M | ...... ..... |
| | | Germline | H_FR1 | H_CDR1 | H_FR2 | H_CDR2 | H_FR3 | H_CDR3 | H_FR4 |

| | | H_FR1 | H_CDR1 | H_FR2 | H_CDR2 | H_FR3 | H_CDR3 | H_FR4 |
|---|---|---|---|---|---|---|---|---|
| | VH6\|6-01\|D4\|4-17\|RF2/JH6 | QVQLQQS-GPGLVKPSQTLS LTCAISG-DSVS | SN---SAAWN | WIRQSPSR GLEWLG | RTYYR-- SKWYNDYA VSVKS | RITENPDTSKNQFSLQL NSVTPEDTAVYYCAR | DYGDYYY----------- -----YYYGMDV | WGQGTT VTVSS |
| iPS:43 6898 | 21-225_68D8 | VH6\|6-01\|D4\|4-17\|RF2/JH6 | .......... .......... ........ | | ...... ...... | ........ EC...... ..Q. | ................H. ...........F... | .R.HRG- ................-F..... | ...... ..... |
| | Germline | H_FR1 | H_CDR1 | H_FR2 | H_CDR2 | H_FR3 | H_CDR3 | H_FR4 |
| | VH3\|3-33\|D7\|7-27\|RF1/JH3 | QVQLVES-GGGVVQPGRSLR LSCAASG-FTFS | S----YGMH | WVRQAPGK GLEWVA | VIWYD GSNKYYAD SVKG | RFTISRDNSKNTLYLQM NSLRAEDTAVYYCAR | LTGD--------AFDI | WGQGTM VTVSS |
| iPS:43 6910 | 21-225_73G1 | VH3\|3-33\|D7\|7-27\|RF1/JH3 | .......... .......... GT...... | Y......... | ........ ...... | .TT..... .... | ...S........... ...........H... | E..TW............. ............ | ...... ....L |
| | Germline | H_FR1 | H_CDR1 | H_FR2 | H_CDR2 | H_FR3 | H_CDR3 | H_FR4 |
| | VH4\|4-30.1\|D2\|2-15\|RF3/JH4 | QVQLQES-GPGLVKPSQILS LTCTVSG-GSIS | SG---GYYWS | WIRQHPGK GLEWIG | YIYY---- SGSTYYNP SLKS | RVTISVDTSKNQFSLKL SSVTAADTAVYYCAR | DIVVVVA----------- ------ATYFDY | WGQGTL VTVSS |
| iPS:43 6920 | 21-225_74E5 | VH4\|4-30.1\|D2\|2-15\|RF3/JH4 | .......... .......... .......R | .......... | ........ ...... | ........ ..R. | .AS............. ................ | .SP.A-- .................-—GT.. | ...... ..... |
| iPS:43 7012 | 21-225_192G7 | VH4\|4-30.1\|D2\|2-15\|RF3/JH4 | .......... .......... ........ | .......... | ........ ...... | ........ R....... .... | ................ N............... | .SP.T-- .................-—G... | ....I. ..... |
| | Germline | H_FR1 | H_CDR1 | H_FR2 | H_CDR2 | H_FR3 | H_CDR3 | H_FR4 |
| | VH1\|1-08\|D5\|5-12\|RF3/JH6 | QVQLVQS-GAEVKKPGASVK VSCKASG-YIFT | S----YDIN | WVRQATGQ GLEWMG | WMNPN--- SGNTGYAQ KFQG | RVTMTRNTSISTAYMEL SSLRSEDTAVYYCAR | GYSGYDYY---------- -----YYYYGMDV | WGQGTT VTVSS |
| iPS:43 6942 | 21-225_146H8 | VH1\|1-08\|D5\|5-12\|RF3/JH6 | .......... .......... .......I | .......... | ........ ...... | ........ ........ .... | .........K....... ................ | .DYY..SSGHQ....... ..PY........ | ...... ..... |
| | Germline | H_FR1 | H_CDR1 | H_FR2 | H_CDR2 | H_FR3 | H_CDR3 | H_FR4 |
| | VH2\|2-05\|D6\|6-6\|RF3/JH4 | QITLKES-GPTLVKPTQILT LTCTFSG-FSLS | TS---GVGVG | WIRQPPGK ALEWLA | LIYW---- NDDKRYSP SLKS | RLTITKDTSKNQVVLTM TNMDPVDTATYYCAH | V*QLV------------- ---------YFDY | WGQGTL VTVSS |
| iPS:43 6944 | 21-225_182D12 | VH2\|2-05\|D6\|6-6\|RF3/JH4 | .......... .....P...... ........ | .T........ | ........ .....G | ILF.... ...E.... .... | ................ ................ | KS................ ............ | ...... ..... |
| | Germline | H_FR1 | H_CDR1 | H_FR2 | H_CDR2 | H_FR3 | H_CDR3 | H_FR4 |

| | | | H_FR1 | H_CDR1 | H_FR2 | H_CDR2 | H_FR3 | H_CDR3 | H_FR4 |
|---|---|---|---|---|---|---|---|---|---|
| VH4\|4-30.1/D2\|2-8\|RF3/JH4 | | | QVQLQES-GPGLVKPSQTLS LTCTVSG-GSIS | SG---GYYWS | WIRQHPGK GLEWIG | YIYY---- SGSTYYNP SLKS | RVTISVDTSKNQFSLKL SSVTAADTAVYYCAR | DIVLMVY---------- ------AIYFDY | WGQGTL VTVSS |
| iPS:43 6958 | 21-225_190D1 | VH4\|4-30.1/D2\|2-8\|RF3/JH4 | .............. ............ ........ | .......... | ........ ...... | ........ .... | ................ | .SP.R-- ...............- -G... | ...... ..... |
| Germline | | | H_FR1 | H_CDR1 | H_FR2 | H_CDR2 | H_FR3 | H_CDR3 | H_FR4 |
| VH6\|6-01/D4\|4-17\|RF2/JH4 | | | QVQLQQS-GPGLVKPSQTLS LTCAISG-DSVS | SN---SAAWN | WIRQSPSR GLEWLG | RTYYR-- SKWYNDYA VSVKS | RITINPDTSKNQFSLQL NSVTPEDTAVYYCAR | DYGDY----------- --------YFDY | WGQGTL VTVSS |
| iPS:43 6962 | 21-225_190H1 | VH6\|6-01/D4\|4-17\|RF2/JH4 | .............. ...........D ........ | RK.....T.. | ........ ...... | K....... .... | ................ | .P.G— .................... ...L... | ...... ..... |
| iPS:43 6978 | 21-225_190G9 | VH6\|6-01/D4\|4-17\|RF2/JH4 | .............. ............ ........ | RK.....T.. | ........ ...... | ........ .... | ..I.............. | .P.G— .................... ...L... | ...... ..... |
| iPS:43 7070 | 21-225_201G11 | VH6\|6-01/D4\|4-17\|RF2/JH4 | .............. ............ ........ | RI...NPT.. | ........ ...... | ...HV... .... | ................ | .P.G— .................... ...L... | ...... ..... |
| iPS:43 7076 | 21-225_203G6 | VH6\|6-01/D4\|4-17\|RF2/JH4 | .............. ............ ........ | RT...NPT.. | ........ ...... | ...HV..L .... | ....T............ | .P.G— .................... ...L... | ...... ..... |
| Germline | | | H_FR1 | H_CDR1 | H_FR2 | H_CDR2 | H_FR3 | H_CDR3 | H_FR4 |
| VH3\|3-33/D1\|1-7\|RF3/JH6 | | | QVQLVES-GGGVVQPGRSLR LSCAASG-FIFS | S-----YGMH | WVRQAPGK GLEWVA | VIWYD--- GSNKYYAD SVKG | RFTISRDNSKNTLYLQM NSLRAEDTAVYYCAR | YNWNYYY---------- -----YYYGMDV | WGQGTT VTVSS |
| iPS:43 6964 | 21-225_190B3 | VH3\|3-33/D1\|1-7\|RF3/JH6 | .............. ............ .......N | N.......I. | ........ ...... | ...F.... .D...... .... | ................ | D....GDH.......... ....Y..F.... | ...... ..... |
| iPS:43 6970 | 21-225_190B8 | VH3\|3-33/D1\|1-7\|RF3/JH6 | .............. ............ ........ | .......... | ........ ...... | ...F.... ......T. .... | ................ | D....GDY.......... ....Y....... | ...... ..... |
| iPS:43 6980 | 21-225_190C10 | VH3\|3-33/D1\|1-7\|RF3/JH6 | .............. ............ ......L. | N......... | ........ ...... | ...FG... .D...... ..R. | ..........F.... | D....GDH.......... ....Y....... | ...... ..... |

| Clone | ID | Germline/VH | H_FR1 | H_CDR1 | H_FR2 | H_CDR2 | H_FR3 | H_CDR3 | H_FR4 |
|---|---|---|---|---|---|---|---|---|---|
| iPS:43 6992 | 21-225_191B8 | VH3\|3-33/D1\|1-7\|RF3/JH6 | ............<br>............<br>......L. | .......... | ........<br>...... | ...F....<br>.......<br>.... | ................<br>....... | D....CDH..........<br>....Y....... | ......<br>..... |
| iPS:43 6994 | 21-225_191A9 | VH3\|3-33/D1\|1-7\|RF3/JH6 | ............<br>............<br>......L. | N......... | ........<br>...... | ...FG...<br>.D......<br>.... | ................<br>.........F.... | D....GDH..........<br>....Y....... | ......<br>..... |
| | | Germline | H_FR1 | H_CDR1 | H_FR2 | H_CDR2 | H_FR3 | H_CDR3 | H_FR4 |
| | | VH4\|4-30.1/D4\|4-11\|RF2/JH6 | QVQLQES-GPGLVKPSQILSLTCTVSG-GSIS | SG---GYYWS | WIRQHPGKGLEWIG | YIYY----SGSTYYNPSLKS | RVTISVDTSKNQFSLKLSSVTAADTAVYYCAR | DYSNYYY--------YYYGMDV | WGQGTTVTVSS |
| iPS:43 6984 | 21-225_190F10 | VH4\|4-30.1/D4\|4-11\|RF2/JH6 | ............<br>............<br>.......R | ......D... | .......E<br>...... | ........<br>..I.....<br>.... | ................ | .S.SR--<br>................--<br>-.... | ...... |
| iPS:43 6988 | 21-225_191A2 | VH4\|4-30.1/D4\|4-11\|RF2/JH6 | ............<br>............<br>.......R | ......D... | .......E<br>...... | ........<br>..I<br>.... | ................ | .S.SR--<br>................--<br>-.... | ...... |
| iPS:43 7014 | 21-225_192H8 | VH4\|4-30.1/D4\|4-11\|RF2/JH6 | ..........V<br>............<br>....... | ......D... | ........<br>...... | ........<br>..P.....<br>.... | .L.M.A.......... | .S.L.--<br>................--<br>-.... | ...... |
| iPS:43 7022 | 21-225_194G5 | VH4\|4-30.1/D4\|4-11\|RF2/JH6 | ..........I..<br>.......R | ......D... | ........<br>...... | ........<br>........<br>.... | ................ | .H.L.--<br>................--<br>-.... | ...... |
| iPS:43 7026 | 21-225_194D12 | VH4\|4-30.1/D4\|4-11\|RF2/JH6 | ............<br>............<br>....... | ......D... | ........<br>...... | ........<br>........<br>.... | ................ | .GARH--<br>................--<br>-.... | ...... |
| iPS:43 7056 | 21-225_198B8 | VH4\|4-30.1/D4\|4-11\|RF2/JH6 | ............<br>............<br>.......R | ......D... | .......E<br>...... | ........<br>..I.....<br>.... | ................ | .S.SR--<br>................--<br>-.... | ...... |
| iPS:43 7124 | 21-225_212H12 | VH4\|4-30.1/D4\|4-11\|RF2/JH6 | ............<br>............<br>.......R | ......D... | ........<br>...... | ........<br>........<br>.... | ......G.......... | .S.S.--<br>................--<br>-.... | ...... |

| | | | H_FR1 | H_CDR1 | H_FR2 | H_CDR2 | H_FR3 | H_CDR3 | H_FR4 |
|---|---|---|---|---|---|---|---|---|---|
| iPS:43 7136 | 21-225_214H3 | VH4\|4-30.1/D4\|4-11\|RF2\|JH6 | .......... .......... .......R | ......⊃... | ...... ....... .... | ....... ....... | .....C.......... ................ | .S.S.-- ..................-- -.... | ...... .... |
| **Germline** | | | **H_FR1** | **H_CDR1** | **H_FR2** | **H_CDR2** | **H_FR3** | **H_CDR3** | **H_FR4** |
| **VH4\|4-59/D1\|1-26\|RF1/JH4** | | | QVQLQES-GPGLVKPSEILSLTCTVSG-GSIS | S-----YYWS | WIRQPPGKGLEWIG | YIYY----SGSTNYNPSLKS | RVTISVDTSKNQFSLKLSSVTAADTAVYYCAR | GIVGA-----------TYFDY | WGQGTLVTVSS |
| iPS:43 6986 | 21-225_191A1 | VH4\|4-59/D1\|1-26\|RF1/JH4 | .......... .......... .......R | ..........I | ........ ...... .... | ........ ....K... .... | ................ | KG..T............. .......IH... | ..... |
| iPS:43 7064 | 21-225_200G8 | VH4\|4-59/D1\|1-26\|RF1/JH4 | .......... .......... .......R | | ........ ...... .... | ........ ....K... .... | ................ | KG..T............. .......IH... | ..... |
| **Germline** | | | **H_FR1** | **H_CDR1** | **H_FR2** | **H_CDR2** | **H_FR3** | **H_CDR3** | **H_FR4** |
| **VH3\|3-30/D6\|6-6\|RF1/JH4** | | | QVQLVES-GGGVVQPGRSLRLSCAASG-FIFS | S-----YGMH | WVRQAPGKGLEWVA | VISYD---GSNKYYADSVKG | RFTISRDNSKNTLYLQMNSLRAEDTAVYYCAK | EYSSS--------SYFDY | WGQGTLVTVSS |
| iPS:43 6996 | 21-225_191B9 | VH3\|3-30/D6\|6-6\|RF1/JH4 | .......... .......... ........ | F.....H... | ........ ...... .... | ..W..... ..K..... .... | ..............H... | .GY..GF........... ......YRG..N | ..... |
| iPS:43 7054 | 21-225_194G3 | VH3\|3-30/D6\|6-6\|RF1/JH4 | .......... .......... ........ | F.....H... | ........ ...... .... | ..W..... ..K..... .... | ................H... | .GF..GF........... ......YRG..N | ..... |
| **Germline** | | | **H_FR1** | **H_CDR1** | **H_FR2** | **H_CDR2** | **H_FR3** | **H_CDR3** | **H_FR4** |
| **VH3\|3-33/D1\|1-26\|RF1/JH6** | | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | GIVGATYY----------YYYGMDV | WGQGTLVTVSS |
| iPS:43 7000 | 21-225_191G9 | VH3\|3-33/D1\|1-26\|RF1/JH6 | .......... .......... ........ | T......... | ........ .....T .... | L..F.... ........ .... | ................ | DR..G.SPP......... ...YY....... | ..... |
| iPS:39 3192 | 21-225_12B1 | VH3\|3-33/D1\|1-26\|RF1/JH6 | .......... .......... ........ | .......... | ........ ...... .... | ...N.... ........ .... | ................ | DR.A.AGTP......... ....Y....... | ..... |
| **Germline** | | | **H_FR1** | **H_CDR1** | **H_FR2** | **H_CDR2** | **H_FR3** | **H_CDR3** | **H_FR4** |
| **VH1\|1-02/D3\|3-10\|RF2/JH5** | | | QVQLVQS-GAEVKKPGASVKVSCKASG-YIFT | G-----YYMH | WVRQAPGQGLEWMG | WINPN---SGGTNYAQKFQG | RVTMTRDTSISTAYMELSRLRSDDTAVYYCAR | YYYGSCSY----------YNNWFDP | WGQGTLVTVSS |

| | | | | | | | | | |
|---|---|---|---|---|---|---|---|---|---|
| iPS:43 7002 | 21-225_191H9 | VH1\|1-02/D3\|3-10\|RF2/JH5 | ............<br>............<br>V....... | .......N.. | ........<br>......<br> | ........<br>.......H<br>.... | ................<br>................<br> | DF.D..G-<br>...............-<br>EG.... | ......<br>..... |
| | Germline | | H_FR1 | H_CDR1 | H_FR2 | H_CDR2 | H_FR3 | H_CDR3 | H_FR4 |
| | VH4\|4-30.4/D2\|2-8\|RF3/JH6 | | QVQLQES-GPGLVKPSQILS LTCTVSG-GSIS | SG---DYYWS | WIRQPPGK GLEWIG | YIYY----SGSTYYNP SLKS | RVTISVDTSKNQFSLKL SSVTAADTAVYYCAR | DIVLMVYAI--------- ---YYYYYGMDV | WGQGTT VTVSS |
| iPS:43 7008 | 21-225_192E3 | VH4\|4-30.4/D2\|2-8\|RF3/JH6 | ............<br>............<br>........ | ......G.... | ........<br>......<br>.... | ........<br>T.......<br>.... | ................<br>................<br> | .DP.Y----<br>............----- <br>.... | ......<br>..... |
| iPS:43 7048 | 21-225_197B11 | VH4\|4-30.4/D2\|2-8\|RF3/JH6 | ............<br>............<br>........ | ......G.... | ........<br>......<br>.... | ........<br>T.......<br>.... | ................<br>................<br> | .DP.Y----<br>............----- <br>.... | ......<br>..... |
| | Germline | | H_FR1 | H_CDR1 | H_FR2 | H_CDR2 | H_FR3 | H_CDR3 | H_FR4 |
| | VH4\|4-59/D7\|7-27\|RF3/JH4 | | QVQLQES-GPGLVKPSEILS LTCTVSG-GSIS | S-----YYWS | WIRQPPGK GLEWIG | YIYY----SGSTNYNP SLKS | RVTISVDTSKNQFSLKL SSVTAADTAVYYCAR | NWGY------------- --------FDY | WGQGTL VTVSS |
| iPS:43 7010 | 21-225_192G3 | VH4\|4-59/D7\|7-27\|RF3/JH4 | ............<br>............<br>........ | N......... | .....A..<br>......<br>.... | R..S....<br>........<br> | ...M............<br>N.............K<br> | G.E-<br>................<br>.....LN. | ......<br>..... |
| iPS:43 7016 | 21-225_193A6 | VH4\|4-59/D7\|7-27\|RF3/JH4 | ............<br>............<br>........ | .......... | ........<br>......<br>.... | ........<br>........<br> | ................<br>...............G<br> | G.E-<br>................<br>.....LN. | ......<br>..... |
| | Germline | | H_FR1 | H_CDR1 | H_FR2 | H_CDR2 | H_FR3 | H_CDR3 | H_FR4 |
| | VH3\|3-15/D4\|4-17\|RF2/JH4 | | EVQLVES-GGGLVKPGGSLR LSCAASG-FIFS | N-----AWMS | WVRQAPGK GLEWVG | RIKSKT-DGGTIDYA APVKG | RFTISRDDSKNTLYLQM NSLKTEDTAVYYCTT | DYGDY------------ --------YFDY | WGQGTL VTVSS |
| iPS:43 7018 | 21-225_193H5 | VH3\|3-15/D4\|4-17\|RF2/JH4 | ............<br>.M.....S....<br>........ | .......Y.T | ........<br>......<br>.... | ........<br>........<br> | ................<br>................<br> | .P.G-<br>................<br>...I... | ......<br>..... |
| iPS:43 7144 | 21-225_215B3 | VH3\|3-15/D4\|4-17\|RF2/JH4 | ............<br>............<br>........ | .........H | ........<br>......<br>.... | .......N<br>........<br> | ................<br>................<br> | .P.G-<br>................<br>...I... | ......<br>..... |
| | Germline | | H_FR1 | H_CDR1 | H_FR2 | H_CDR2 | H_FR3 | H_CDR3 | H_FR4 |
| | VH3\|3-11/D3\|3-9\|RF1/JH4 | | QVQLVES-GGGLVKPGGSLR LSCAASG-FIFS | D-----YYMS | WIRQAPGK GLEWVS | YISSS---GSTIYYAD SVKG | RFTISRDNAKNSLYLQM NSLRAEDTAVYYCAR | VLRYFDW---------- -----LL*YFDY | WGQGTL VTVSS |

EP 4 435 105 A2

# EP 4 435 105 A2

| | | | H_FR1 | H_CDR1 | H_FR2 | H_CDR2 | H_FR3 | H_CDR3 | H_FR4 |
|---|---|---|---|---|---|---|---|---|---|
| iPS:43 7030 | 21-225_195E3 | VH3\|3-11/D3\|3-9\|RF1/JH4 | ............ ......... | ......... | ........ ...... | ..T..... .N...... .... | ................. | DS...-- .........--- DW... | ...... ..... |
| | Germline | | H_FR1 QVQLQES- GPGLVKPSEILS LTCTVSG-GSIS | H_CDR1 S-----YYWS | H_FR2 WIRQPPGK GLEWIG | H_CDR2 YIYY---- SGSTNYNP SLKS | H_FR3 RVTISVDTSKNQFSLKL SSVTAADTAVYYCAR | H_CDR3 NWGAE---------- ------YFQH | H_FR4 WGQGTL VTVSS |
| **VH4\|4-59/D7\|7-27\|RF3/JH1** | | | | | | | | | |
| iPS:43 7032 | 21-225_195H6 | VH4\|4-59/D7\|7-27\|RF3/JH1 | ............ ............ .......R | N........ | ......A.. ...... | R..S.... ........ .... | ..SM........... ..............T. | G.E-- ............... ...-LNN | ...... ..... |
| | Germline | | H_FR1 QVQLQES- GPGLVKPSEILS LTCTVSG-GSIS | H_CDR1 S-----YYWS | H_FR2 WIRQPPGK GLEWIG | H_CDR2 YIYY---- SGSTNYNP SLKS | H_FR3 RVTISVDTSKNQFSLKL SSVTAADTAVYYCAR | H_CDR3 EYSSSSYY------- -----YYYGMDV | H_FR4 WGQGTL VTVSS |
| **VH4\|4-59/D6\|6-6\|RF1/JH6** | | | | | | | | | |
| iPS:43 7044 | 21-225_197F9 | VH4\|4-59/D6\|6-6\|RF1/JH6 | ............ ............ .......R | I......... | ........ ...... | .V...... ....T... .... | ................. N............V. | .RG..HRW......... ....GD...... | ..R... ..... |
| iPS:43 7060 | 21-225_199C3 | VH4\|4-59/D6\|6-6\|RF1/JH6 | ............ ............ .......R | I......... | ....T... ...... | ........ ....T... N............V. | | .RG..HRW......... ....GD...... | ..R... ..... |
| | Germline | | H_FR1 QVQLVES- GGGVVQPGRSLR LSCAASG-FIFS | H_CDR1 S-----YGMH | H_FR2 WVRQAPGK GLEWVA | H_CDR2 VISYD--- GSNKYYAD SVKG | H_FR3 RFTISRDNSKNTLYLQM NSLRAEDTAVYYCAK | H_CDR3 EYSSSS--------- ------AEYFQH | H_FR4 WGQGTL VTVSS |
| **VH3\|3-30/D6\|6-6\|RF1/JH1** | | | | | | | | | |
| iPS:43 7058 | 21-225_199F3 | VH3\|3-30/D6\|6-6\|RF1/JH1 | ............ ............ ....... | F........ | ........ ...... | ..W..... ..S..... .... | ...V............ ................ | .GY..GF.......... ......YRG.AN | ...... ..... |
| | Germline | | H_FR1 QVQLQES- GPGLVKPSQILS LTCTVSG-GSIS | H_CDR1 SG---GYYWS | H_FR2 WIRQHPGK GLEWIG | H_CDR2 YIYY---- SGSTYYNP SLKS | H_FR3 RVTISVDTSKNQFSLKL SSVTAADTAVYYCAR | H_CDR3 SIAARYY-------- ------YYYGMDV | H_FR4 WGQGTT VTVSS |
| **VH4\|4-30.1/D6\|6-6\|RF2/JH6** | | | | | | | | | |
| iPS:43 7062 | 21-225_200H1 | VH4\|4-30.1/D6\|6-6\|RF2/JH6 | ............ ............ ........ | ......D... | ........ ...... | ........ .... | N............... | DG..L-- ...............-- -.... | ...... ..... |
| iPS:43 7066 | 21-225_200G9 | VH4\|4-30.1/D6\|6-6\|RF2/JH6 | ............ ............ ........ | ......D... | ........ ...... | ........ .... | N............... | DG..L-- ...............-- -.... | ...... ..... |

3314

| iPS:43 7068 | 21- 225_200A11 | VH4\|4- 30.1/D6\|6 - 6\|RF2/JH 6 | ............ ............ ........ | ......⊃.... | ........ ...... .... | ........ R...... .... | ................. | DA..H-- .................--- -.... | ...... ..... |
| iPS:43 7140 | 21- 225_214E12 | VH4\|4- 30.1/D6\|6 - 6\|RF2/JH 6 | ............ ............ ........ | ......D.... | ........ ...... .... | ........ ..P.... .... | ................. | DG..E-- .................--- -.... | ...... ..... |
| iPS:43 7158 | 21- 225_216H11 | VH4\|4- 30.1/D6\|6 - 6\|RF2/JH 6 | ............ ............ ........ | ......D.... | ........ ...... .... | ........ ..P.... .... | ................. | DG..E-- .................--- -.L.. | ...... ..... |

| | Germline | | H_FR1 | H_CDR1 | H_FR2 | H_CDR2 | H_FR3 | H_CDR3 | H_FR4 |
|---|---|---|---|---|---|---|---|---|---|
| | VH3\|3- 33/D1\|1- 26\|RF3/JH3 | | QVQLVES- GGGVVQPGRSLR LSCAASG-FIFS | S------YGMH | WVRQAPGK GLEWVA | VIWYD--- GSNKYYAD SVKG | RFTISRDNSKNTLYLQM NSLRAEDTAVYYCAR | YSGSYY--------------- -------DAFDI | WGQGTM VTVSS |
| iPS:43 7074 | 21- 225_203B2 | VH3\|3- 33/D1\|1- 26\|RF3/J H3 | .......... .......... ........ | N......... | ........ ...... .... | I..F.... ...E.... .... | ..........MS...... .............. | E....- ................... .-.LY. | ...... ..... |
| iPS:43 7082 | 21- 225_205E12 | VH3\|3- 33/D1\|1- 26\|RF3/J H3 | .......... .......... ........ | N......... | ........ ...... .... | I..F.... ...E.... .... | ..........MS...... .............. | E....- ................... .-.LY. | ...... ..... |

| | Germline | | H_FR1 | H_CDR1 | H_FR2 | H_CDR2 | H_FR3 | H_CDR3 | H_FR4 |
|---|---|---|---|---|---|---|---|---|---|
| | VH3\|3- 48/D4\|4- 17\|RF2/JH4 | | EVQLVES- GGGLVQPGGSLR LSCAASG-FIFS | S------YSMN | WVRQAPGK GLEWVS | YISSS--- SSTIYYAD SVKG | RFTISRDNAKNSLYLQM NSLRDEDTAVYYCAR | DYGDY--------------- -------YFDY | WGQGTL VTVSS |
| iPS:43 7086 | 21- 225_209A8 | VH3\|3- 48/D4\|4- 17\|RF2/J H4 | .......... .......... .......R | .......... | ........ .....L .... | ..IKK... .... | ..............V. .............. | .D.S............... .......... | ...... ..... |

| | Germline | | H_FR1 | H_CDR1 | H_FR2 | H_CDR2 | H_FR3 | H_CDR3 | H_FR4 |
|---|---|---|---|---|---|---|---|---|---|
| | VH4\|4- 30.1/D2\|2- 8\|RF3/JH6 | | QVQIQES- GPGLVKPSQTLS LTCTVSG-GSIS | SG----GYYWS | WIRQHPGK GLEWIG | YIYY---- SGSTYYNP SLKS | RVTISVDTSKNQFSLKL SSVTAADTAVYYCAR | DIVLMVYAI---------- ---YYYYYGMDV | WGQGTT VTVSS |
| iPS:43 7090 | 21- 225_210F11 | VH4\|4- 30.1/D2\|2- 8\|RF3/JH 6 | .......... .......... ........ | ......S... | ........ ...... .... | I.T..... .... | .........T.H..... .............. | .EP.T---- ............----- .... | ...... ..... |

| | | | H_FR1 | H_CDR1 | H_FR2 | H_CDR2 | H_FR3 | H_CDR3 | H_FR4 |
|---|---|---|---|---|---|---|---|---|---|
| iPS:43 7106 | 21-225_211H7 | VH4\|4-30.1/D2\|2-8\|RF3/JH6 | ............ ............ ........ | .......... | .T...... ...... .... | ........ V....... .... | ................ | .GP.S---- ............------ .... | ...... ..... |
| | Germline | | H_FR1 | H_CDR1 | H_FR2 | H_CDR2 | H_FR3 | H_CDR3 | H_FR4 |
| | VH3\|3-30.3/D4\|4-17\|RF2/JH5 | | QVQLVES-GGGVVQPGRSLR LSCAASG-FIFS | S------YAMH | WVRQAPGK GLEWVA | VISYD--- GSNKYYAD SVKG | RFTISRDNSKNTLYLQM NSLRAEDTAVYYCAR | DYGDY-------------------NWFDP | WGQGTL VTVSS |
| iPS:43 7100 | 21-225_211H2 | VH3\|3-30.3/D4\|4-17\|RF2/JH5 | ............ ............ ........ | .......... | ........ ...... .... | ..W..... ........ .... | ....A............ | .P.S...............-G... | ...... ..... |
| | Germline | | H_FR1 | H_CDR1 | H_FR2 | H_CDR2 | H_FR3 | H_CDR3 | H_FR4 |
| | VH3\|3-33/D5\|5-12\|RF3/JH6 | | QVQLVES GGGVVQPGRSLR LSCAASG-FIFS | S     YGMH | WVRQAPGK GLEWVA | VIWYD GSNKYYAD SVKG | RFTISRDNSKNTLYLQM NSLRAEDTAVYYCAR | GYSGYDYY ----YYYYGMDV | WGQGTT VTVSS |
| iPS:43 7102 | 21-225_211E5 | VH3\|3-33/D5\|5-12\|RF3/JH6 | ............ ............ .......R | N......... | ........ ......S .... | I..F.... ..DQ.... .... | ................ | .L.V.Y-- ............--- ...G. | ...... ..... |
| iPS:43 7164 | 21-225_217C6 | VH3\|3-33/D5\|5-12\|RF3/JH6 | ............ ............ ....... | N......... | ........ ....M. .... | I..F.... ..DE.... .... | ............M.... | .L.V.Y-- ...............--- ..... | ...... ..... |
| iPS:43 7166 | 21-225_217G11 | VH3\|3-33/D5\|5-12\|RF3/JH6 | ............ ............ .......R | N......... | ........ ......S .... | I..F.... ..DQ.... .... | ................ | .L.V.Y-- ...............--- ..... | ...... ..... |
| iPS:43 7170 | 21-225_218E5 | VH3\|3-33/D5\|5-12\|RF3/JH6 | ............ ............ ....... | N......... | ........ ....M. .... | I..F.... ..DE.... .... | ................ | .L.V.Y-- ...............--- ..... | ...... ..... |
| | Germline | | H_FR1 | H_CDR1 | H_FR2 | H_CDR2 | H_FR3 | H_CDR3 | H_FR4 |
| | VH4\|4-30.1/D6\|6-19\|RF2/JH6 | | QVQLQES-CPGLVKPSQTLS LTCTVSG-GSIS | SG---GYYWS | WIRQHPGK GLEWIG | YIYY---- SGSTYYNP SLKS | RVTISVDTSKNQFSLKL SSVTAADTAVYYCAR | GIAVAGYY-------------YYYCMDV | WGQGTT VTVSS |
| iPS:43 7108 | 21-225_211C9 | VH4\|4-30.1/D6\|6-19\|RF2/JH6 | ............ ............ .......R | .......D... | ........ ...... .... | ........ ........ .... | ....LL............ ....V.......... | DS..Y--- ...............--- N... | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:43 7110 | 21-225_211E9 | VH4\|4-30.1/D6\|6-19\|RF2/JH6 | ............<br>............<br>........ | ......Ɔ... | ........<br>...... | .......<br>T..N....<br>.... | ................N.<br>I.............. | DS..Y---<br>................---<br>.... | ......<br>.... |
| iPS:43 7120 | 21-225_212A9 | VH4\|4-30.1/D6\|6-19\|RF2/JH6 | ............<br>...........S<br>.......R | ......Ɔ... | ........<br>...... | .M......<br>........<br>.... | .................<br>....V......... | DS..Y---<br>................---<br>.... | ......<br>.... |
| iPS:43 7132 | 21-225_213F5 | VH4\|4-30.1/D6\|6-19\|RF2/JH6 | ............<br>............<br>.......R | ......D... | ........<br>...... | ........<br>........<br>.... | ......L.........<br>....V......... | DS..Y---<br>................---<br>N... | ......<br>.... |
| iPS:43 7142 | 21-225_215A3 | VH4\|4-30.1/D6\|6-19\|RF2/JH6 | ............<br>............<br>........ | ......D... | ........<br>...... | .......<br>T..N....<br>.... | ................V<br>I.............. | DS..Y---<br>................---<br>.... | ......<br>.... |
| iPS:43 7148 | 21-225_215H3 | VH4\|4-30.1/D6\|6-19\|RF2/JH6 | ............<br>...........S<br>.......R | ......D... | ........<br>...... | .M......<br>........<br>.... | .................<br>....V......... | DS..Y---<br>................---<br>.... | ......<br>.... |
| iPS:43 7154 | 21-225_216A7 | VH4\|4-30.1/D6\|6-19\|RF2/JH6 | ............<br>...........S<br>.......R | ......D... | ........<br>...... | .M......<br>........<br>.... | .................<br>....V......... | DS..Y---<br>................---<br>.... | ......<br>.... |
| | **VH3\|3-30.3/D7\|7-27\|RF2/JH4** | Germline | QVQLVES-<br>CCGVVQPGRSLR<br>LSCAASG-FIFS | S-----YAMH | WVRQAPGK<br>CLEWVA | VISYD---<br>GSNKYYAD<br>SVKG | RFTISRDNSKNTLYLQM<br>NSLRAEDTAVYYCAR | *LGY------------<br>----------FDY | WGQGTL<br>VTVSS |
| iPS:43 7160 | 21-225_216B12 | VH3\|3-30.3/D7\|7-27\|RF2/JH4 | ............<br>............<br>........ | .......... | ........<br>...... | ..W.....<br>........<br>.... | .................<br>................. | D..LG...........<br>.......YF... | ......<br>.... |
| | **VH6\|6-01/D2\|2-21\|RF2/JH5** | Germline | QVQLQQS-<br>GPGLVKPSQILS<br>LTCAISG-DSVS | SN---SAAWN | WIRQSPSR<br>GLEWLG | RIYYR--<br>SKWYNDYA<br>VSVKS | RITINPDTSKNQFSLQL<br>NSVTPEDTAVYYCAR | AYCGGDC----------<br>------YSNWFDP | WGQGTL<br>VTVSS |
| iPS:43 7186 | 21-225_224H2 | VH6\|6-01/D2\|2-21\|RF2/JH5 | ............<br>...........D<br>........ | .......... | ........<br>...... | ........<br>.V..............<br>.... | ................. | EGGL.Y.SST........<br>...SC.GG.... | ......<br>.... |

| | | Germline | H_FR1 | H_CDR1 | H_FR2 | H_CDR2 | H_FR3 | H_CDR3 | H_FR4 |
|---|---|---|---|---|---|---|---|---|---|
| | VH1\|1-02/D4\|4-17\|RF2/JH6 | | QVQLVQS-GAEVKKPGASVK VSCKASG-YIFT | G-----YYMH | WVRQAPGQ GLEWMG | WINPN---SGGTNYAQ KFQG | RVTMTRDTSISTAYMEL SRLRSDDTAVYYCAR | DYGDYYY -----YYYGMDV | WGQGTT VTVSS |
| IPS:43 7188 | 21-225_224B11 | VH1\|1-02/D4\|4-17\|RF2/JH6 | ..H.......... ............ ........ | .........I. | ......... ...... | ....K... N....... .... | .......A....T.... ................ | GAF..F............ .....-..A... | ..H... ..... |
| IPS:43 7198 | 21-225_226F8 | VH1\|1-02/D4\|4-17\|RF2/JH6 | ............ ............ ........ | .........I. | ......... ...... | ........ .......R .... | ................ ................ | GAF............... .....-..AL.. | ...... ..... |
| IPS:43 7202 | 21-225_227D3 | VH1\|1-02/D4\|4-17\|RF2/JH6 | ............ ............ ........ | .......... | ......... ...R.. | ....K... .....F.. .... | ................ ................ | GAF..F............ .....-...... | ...... ..... |
| IPS:43 7208 | 21-225_227C10 | VH1\|1-02/D4\|4-17\|RF2/JH6 | ............ ............ ........ | .......... | ......... ...R.. | ....K... ........ .... | ................ ................ | GAF..F............ .....-...... | ...... ..... |
| | VH3\|3-33/D2\|2-2\|RF2/JH6 | Germline | QVQLVES-GGGVVQPGRSLR LSCAASG-FTFS | S-----YGMH | WVRQAPGK GLEWVA | VIWYD---GSNKYYAD SVKG | RFTISRDNSKNTLYLQM NSLRAEDTAVYYCAR | GYCSSTSCYT-------- ---YYYYYGMDV | WGQGTT VTVSS |
| IPS:43 7192 | 21-225_225E9 | VH3\|3-33/D2\|2-2\|RF2/JH6 | ............ ..........E .....I.. | .......... | ......... ...... | .M..... .G..D... .... | ................ ................ | DREYC..TSC........ ..P......... | ...... ..... |
| | VH3\|3-23/D2\|2-21\|RF2/JH6 | Germline | EVQLLES-GGGLVQPGGSLR LSCAASG-FTFS | S-----YAMS | WVRQAPGK GLEWVS | AISGS---GGSTYYAD SVKG | RFTISRDNSKNTLYLQM NSLRAEDTAVYYCAK | AYCGGDCYS--------- ---YYYYYGMDV | WGQGTT VTVSS |
| IPS:43 7204 | 21-225_227E5 | VH3\|3-23/D2\|2-21\|RF2/JH6 | ............ ...E........ ........ | .......... | ......... ...... | ........ ........ .... | ..............C... ................ | E........P........ ............ | ...... ..... |
| IPS:39 3196 | 21-225_16G8 | VH3\|3-23/D2\|2-21\|RF2/JH6 | ............ ...E........ ........ | .......... | ......... ...... | G....... ........ .... | ..............C.H. ................ | E........P........ ............ | ...... ..... |
| IPS:39 3202 | 21-225_6B4 | VH3\|3-23/D2\|2-21\|RF2/JH6 | ............ ...E........ ........ | .......... | ......... ...... | ........ .S...... .... | ..............C... ................ | E........P........ ............ | ...... ..... |

| iPS id | name | Germline | H_FR1 | H_CDR1 | H_FR2 | H_CDR2 | H_FR3 | H_CDR3 | H_FR4 |
|---|---|---|---|---|---|---|---|---|---|
| iPS:39 3345 | 21-225_5G7 | VH3\|3-23/D2\|2-21\|RF2/JH6 | ............. ...E........ ........ | .......... | ........ ...... | ........ .... | ...............C... ................ | E........P........ ............ | ...... ..... |
| | | Germline | H_FR1 | H_CDR1 | H_FR2 | H_CDR2 | H_FR3 | H_CDR3 | H_FR4 |
| | **VH2\|2-05/D6\|6-13\|RF3/JH4** | | QITLKES-GPTLVKPTQILT LTCIFSG-FSLS | TS---GVGVG | WIRQPPGK ALEWLA | LIYW---- NDDKRYSP SLKS | RLTIITKDTSKNQVVLTM TNMDPVDTATYYCAH | V*QQL----------------- -------VYFDY | WGQGTL VTVSS |
| iPS:43 7210 | 21-225_227E12 | VH2\|2-05/D6\|6-13\|RF3/JH4 | ............. ............ ........ | .......... | ........ ...C.S | ........ ....V... .... | ......Y.......... ................ | RG................... ........-AL.. | ...... ..... |
| iPS:39 2587 | 21-225_18G5 | VH2\|2-05/D6\|6-13\|RF3/JH4 | ............. ............ ........ | .......... | ........ ...C.S | ........ ....V... .... | ......Y.......... ................ | RG................... ........-AL.. | ...... ..... |
| iPS:39 8504 | 21-225_23D7 | VH2\|2-05/D6\|6-13\|RF3/JH4 | ............. ............ ........ | .......... | ........ ...C.S | ........ .N..V... .... | ......Y.......... S............... | RG................... ........-AL.. | ...... ..... |
| | | Germline | H_FR1 | H_CDR1 | H_FR2 | H_CDR2 | H_FR3 | H_CDR3 | H_FR4 |
| | **VH3\|3-21/D4\|4-11\|RF2/JH6** | | EVQLVES-GGGLVKPGGSLR LSCAASG-FIFS | S------YSMN | WVRQAPGK GLEWVS | SISSS--- SSYIYYAD SVKG | RFTISRDNAKNSLYLQM NSLRAEDTAVYYCAR | DYSNYYY----------- -----YYYGMDV | WGQGTT VTVSS |
| iPS:43 7226 | 21-225_57C2 | VH3\|3-21/D4\|4-11\|RF2/JH6 | ............. ............ ........ | ......FG.. | ........ ...... | TG...N.. .... | ................ | T..G--- -- -SL.. | ...... ..... |
| | | Germline | H_FR1 | H_CDR1 | H_FR2 | H_CDR2 | H_FR3 | H_CDR3 | H_FR4 |
| | **VH3\|3-21/D5\|5-18\|RF3/JH5** | | EVQLVES-GGGLVKPGGSLR LSCAASG-FIFS | S------YSMN | WVRQAPGK GLEWVS | SISSS--- SSYIYYAD SVKG | RFTISRDNAKNSLYLQM NSLRAEDTAVYYCAR | GYSYGY----------- -------NWFDP | WGQGTL VTVSS |
| iPS:43 7230 | 21-225_62H10 | VH3\|3-21/D5\|5-18\|RF3/JH5 | ............. ............ ........ | .......... | ........ ...... | ........ .... | ................ | .G.R-- .-G... | ...... ..... |
| iPS:44 8906 | 21-225_72G9 | VH3\|3-21/D5\|5-18\|RF3/JH5 | ............. ............ ........ | .......... | ........ ...... | ...G.... .... | ................ F... | .G.R-- .-G... | ...... ..... |
| | | Germline | H_FR1 | H_CDR1 | H_FR2 | H_CDR2 | H_FR3 | H_CDR3 | H_FR4 |
| | **VH1\|1-02/D1\|1-26\|RF1/JH4** | | QVQLVQS-GAEVKKPGASVK VSCKASG-YIFT | G------YYMH | WVRQAPGQ GLEWMG | WINPN--- SGGTNYAQ KFQG | RVTMTRDTSISTAYMEL SRLRSDDTAVYYCAR | GIVGA------------- -------TYFDY | WGQGTL VTVSS |

| | | | | | | | | | |
|---|---|---|---|---|---|---|---|---|---|
| IPS:43 7260 | 21-225_170D1 | VH1\|1-02/D1\|1-26\|RF1/JH4 | ............<br>............<br>........ | .......F.. | ..........<br>...... | ..K.K...<br>......C..<br>.... | .........S.......<br>...⎯............. | .GA⎯VTT............<br>......WGV... | ......<br>..... |
| | | Germline | H_FR1 | H_CDR1 | H_FR2 | H_CDR2 | H_FR3 | H_CDR3 | H_FR4 |
| | VH3\|3-33/D2\|2-21\|RF2/JH6 | | QVQLVES-GGGVVQPGRSLR LSCAASG-FIFS | S-----YGMH | WVRQAPGK GLEWVA | VIWYD---GSNKYYAD SVKG | RFTISRDNSKNTLYLQM NSLRAEDTAVYYCAR | AYCGGDCYS--------- -YYYYYGMDV | WGQGTT VTVSS |
| IPS:43 7268 | 21-225_177D2 | VH3\|3-33/D2\|2-21\|RF2/JH6 | ..........<br>..........<br>........ | .........D | ..........<br>......<br>.... | I..F.... | .................. | ........FP........<br>...HLH...... | ..... |
| | | Germline | H_FR1 | H_CDR1 | H_FR2 | H_CDR2 | H_FR3 | H_CDR3 | H_FR4 |
| | VH4\|4-30.1/D4\|4-17\|RF2/JH4 | | QVQLQES-GPGLVKPSQILS LTCTVSG-GSIS | SG---GYYWS | WIRQHPGK GLEWIG | YIYY----SGSTYYNP SLKS | RVTISVDTSKNQFSLKL SSVTAADTAVYYCAR | DYGDY------------- ---------YFDY | WGQGTL VTVSS |
| IPS:43 7294 | 21-225_216D5 | VH4\|4-30.1/D4\|4-17\|RF2/JH4 | ..........<br>..........<br>........ | .......... | ..........<br>......<br>.... | ......... | N..........F... | .SP.R..............<br>........G... | ..... |
| | | Germline | H_FR1 | H_CDR1 | H_FR2 | H_CDR2 | H_FR3 | H_CDR3 | H_FR4 |
| | VH3\|3-30.3/D5\|5-24\|RF3/JH6 | | QVQLVES-GGGVVQPGRSLR LSCAASG-FIFS | S-----YAMH | WVRQAPGK GLEWVA | VISYD---GSNKYYAD SVKG | RFTISRDNSKNTLYLQM NSLRAEDTAVYYCAR | RDGYNYYY---------- ------YYYGMDV | WGQGTT VTVSS |
| IPS:43 7302 | 21-225_225B11 | VH3\|3-30.3/D5\|5-24\|RF3/JH6 | ..........<br>..........<br>........ | .......G.. | .........<br>.....T<br>.... | I....S... | .................. | .GYSYG--<br>...............⎯<br>G..... | ..... |
| IPS:45 1102 | 21-225_45F6 | VH3\|3-30.3/D5\|5-24\|RF3/JH6 | ..........<br>..........<br>........ | Y......GL. | .........<br>......<br>.... | ......... | .N.........F.... | E.R.CSGTSC........<br>..PYY....... | ......<br>..... |
| IPS:39 2868 | 21-225_24D6 | VH3\|3-30.3/D5\|5-24\|RF3/JH6 | ..........<br>..........<br>........ | .......G.. | ....V...<br>......<br>.... | I...A... | ..............V. | .GYSYG--<br>...............⎯<br>G..... | ....A.<br>..... |
| IPS:39 3910 | 21-225_15F10 | VH3\|3-30.3/D5\|5-24\|RF3/JH6 | ..........<br>..........<br>........ | .......G.. | .........<br>.....S<br>.... | ....G...<br>...N.... | ..............V. | .GYSYG--<br>...............⎯<br>G..... | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:39 4000 | 21-225_11A2 | VH3-30.3/D5\|5-24\|RF3/JH6 | ............ ............ ........ | .......G.. | ........ ...... .... | ....G... ....DS.. .... | ..I............. .........V. | .GYSYG-- ...............- G..... | ...... |
| iPS:39 4004 | 21-225_13A1 | VH3-30.3/D5\|5-24\|RF3/JH6 | ............ ............ ........ | .......G.. | ........ ...... .... | ....A... .T.Q.... .... | ...............V. | .GYSYG-- ...............- G..... | ...... |
| iPS:39 4006 | 21-225_15C2 | VH3-30.3/D5\|5-24\|RF3/JH6 | ............ ............ ........ | .......G.. | ........ .....S .... | I...G... .R.NH... .... | ...............V. | .GYSYG-- ...............- G..... | ...... |
| iPS:39 4029 | 21-225_1B12 | VH3-30.3/D5\|5-24\|RF3/JH6 | ............ ............ ........ | .......G.. | ........ ...... .... | I...A... ....S... .... | ..........M..... ...........V. | .GYSYG-- ...............- G..... | ....A. |
| iPS:39 4047 | 21-225_5E6 | VH3-30.3/D5\|5-24\|RF3/JH6 | ............ ............ ........ | .......G.. | ........ ...... .... | I...V... .N...... .... | ....V........ | .GYSYG-- ...............- G..... | ...... |
| iPS:39 4081 | 21-225_16B3 | VH3-30.3/D5\|5-24\|RF3/JH6 | ............ ...........V ........ | .......G.. | ........ ...... .... | ....A... .I..S... .... | ..........N....... ...........V. | .GYSYG-- ...............- G..... | ....A. |
| | | Germline | H_FR1 | H_CDR1 | H_FR2 | H_CDR2 | H_FR3 | H_CDR3 | H_FR4 |
| | VH4-61/D3\|3-9\|RF1/JH4 | | QVQLQES- GPGLVKPSEILS LTCTVSG-GSVS | SG---SYYWS | WIRQPPGK GLEWIG | YIYY---- SGSTNYNP SLKS | RVTISVDTSKNQFSLKL SSVTAADTAVYYCAR | VLRYFDW----------- ------LL*YFDY | WGQGTL VTVSS |
| iPS:45 1118 | 21-225_191C8 | VH4-61/D3\|3-9\|RF1/JH4 | ............ ............ ..S..... | ......G.... | ........ ...... | ........ ..T.I... .... | T...V.......... | DTFC..G........... .....CGYF..S | ...... |
| | | Germline | H_FR1 | H_CDR1 | H_FR2 | H_CDR2 | H_FR3 | H_CDR3 | H_FR4 |
| | VH2-05/D4\|4-11\|RF3/JH4 | | QITLKES- GPTLVKPTQILT LTCTFSG-FSLS | TS---GVGVG | WIRQPPGK ALEWLA | LIYW---- NDDKRYSP SLKS | RLTITKDTSKNQVVLTM TNMDPVDTATYYCAH | TTVT--------------- --------YFDY | WGQGTL VTVSS |
| iPS:39 2573 | 21-225_15G2 | VH2-05/D4\|4-11\|RF3/JH4 | ............ ........ | ......... | ........ ...... | ........ .... | ..................D | .G.SC............. .......C..H. | ...... |
| | | Germline | H_FR1 | H_CDR1 | H_FR2 | H_CDR2 | H_FR3 | H_CDR3 | H_FR4 |

| | | | H_FR1 | H_CDR1 | H_FR2 | H_CDR2 | H_FR3 | H_CDR3 | H_FR4 |
|---|---|---|---|---|---|---|---|---|---|
| | VH1|1-02/D2|2-2|RF2/JH6 | | QVQLVQS-GAEVKKPGASVK VSCKASG-YIFT | G-----YYMH | WVRQAPGQ GLEWMG | WINPN--- SGGTNYAQ KFQG | RVTMTRDTSISTAYMEL SRLRSDDTAVYYCAR | GYCSSTSCYT-------- ---YYYYYGMDV | WGQGTT VTVSS |
| iPS:39 2585 | 21-225_14H11 | VH1|1-02/D2|2-2|RF2/JH6 | ............. ...........Q G....... | ......H..C | ........ | ........ ...... .... | ................ ..............A | ......S...L........ ...QPG...... | ...... ..... |
| iPS:39 3188 | 21-225_27D9 | VH1|1-02/D2|2-2|RF2/JH6 | ............. ............. ........ | .......... | ........ | ...... ....K... .... | ................ N............. | ER..T....L........ ..GITG...... | ...... ..... |
| iPS:39 3234 | 21-225_26C10 | VH1|1-02/D2|2-2|RF2/JH6 | ............. ............. ........ | ........V. | ........ | ...... ........ .... | ................ | ER..T....L........ ..GITG...... | ...... ..... |
| | Germline | | H_FR1 | H_CDR1 | H_FR2 | H_CDR2 | H_FR3 | H_CDR3 | H_FR4 |
| | VH3|3-23/D5|5-12|RF3/JH6 | | EVQLLES-GGGLVQPGGSLR LSCAASG-FIFS | S-----YAMS | WVRQAPGK GLEWVS | AISGS--- GGSTYYAD SVKG | RFTISRDNSKNILYLQM NSLRAEDTAVYYCAK | GYSGYDYY---------- ---YYYYYGMDV | WGQGTT VTVSS |
| iPS:39 2596 | 21-225_12D8 | VH3|3-23/D5|5-12|RF3/JH6 | ............. ............. ........ | .......V.. | ........ | T..VG... ...... .... | ...........T...... | WGR..S.E.......... ....-...... | ...... ..... |
| iPS:39 2942 | 21-225_30E9 | VH3|3-23/D5|5-12|RF3/JH6 | ...V........ ............. ........ | ......C..N | ........ | ....R... ....F... .... | ................ | .ELLE..- .............-....... | ...... ..... |
| iPS:39 2944 | 21-225_31H5 | VH3|3-23/D5|5-12|RF3/JH6 | ............. ............. ........ | .......... | ........ | ....R... ...IFH.. .... | ................ .............V. | .ELLE..- .............- .F..... | ...... ..... |
| iPS:39 2964 | 21-225_31A8 | VH3|3-23/D5|5-12|RF3/JH6 | ............. ............. ........ | .......... | ........ | ....R... ....FH.. .... | ................ .....D........V. | .ELLE..- .............- .F..... | ...... ..... |
| iPS:39 2982 | 21-225_30D1 | VH3|3-23/D5|5-12|RF3/JH6 | ...V........ ............. ........ | .......... | ........ | ....R... ....FH.. .... | ................ .............V. | .ELLE..- .............- .F..L.. | ...... ..... |
| iPS:39 2986 | 21-225_31B8 | VH3|3-23/D5|5-12|RF3/JH6 | ............. ............. ........ | .......... | ........ | ....R... ....FH.. .... | ................L .....D.......V. | .ELLE..- .............- .F..... | ...... ..... |
| iPS:39 3004 | 21-225_30G11 | VH3|3-23/D5|5-12|RF3/JH6 | ............. ............. ........ | .......... | ........ | ....R... ....FN.. .... | ...........T...... .............V. | .ELLE..- .............- .F..... | ...... ..... |

| iPS:39 3040 | 21-225_30E3 | VH3\|3-23\|D5\|5-12\|RF3/J H6 | ...V........ ............ ........ | .........N | ........ ...... | ....R... ....F... .E.. | ................. .........L..... | .ELLE..- ..............- ....... | ...... ..... |
| iPS:39 3058 | 21-225_31H3 | VH3\|3-23\|D5\|5-12\|RF3/J H6 | ...V........ ............ ........ | .........N | ........ ...... | ....R... ..N.F... .... | ................. ................. | .ELLE..- ..............- ....... | ...... ..... |
| iPS:39 3060 | 21-225_32G12 | VH3\|3-23\|D5\|5-12\|RF3/J H6 | ............ L........... .......N | .......... | ........ ...... | S...R... ....FH.. .... | ..........R....... .............V. | .ELLE..- ..............- .F..... | ...... ..... |
| iPS:39 3068 | 21-225_34G9 | VH3\|3-23\|D5\|5-12\|RF3/J H6 | ....V....... ............ ........ | .......... | ........ ...... | ....R... ....FH.. .... | ................. .............V. | .ELLE..- ..............- .F..... | ...... ..... |
| iPS:39 3072 | 21-225_36C5 | VH3\|3-23\|D5\|5-12\|RF3/J H6 | ....V....... ............ ........ | .........N | ........ ...... | ....R... ....FH.. .... | ................. .............V. | .ELLE..- ..............- .F..... | ...... ..... |
| iPS:39 3076 | 21-225_33A4 | VH3\|3-23\|D5\|5-12\|RF3/J H6 | ............ ..S.......E .....I.. | .........N | ....V... ...... | ...RR... ....F... .... | ................. ..........F... | .ELLE..- ..............- S...I.. | ...... ..... |
| iPS:39 3102 | 21-225_33F1 | VH3\|3-23\|D5\|5-12\|RF3/J H6 | ............ L........... ........ | .......... | ........ ...... | S...R... ....FH.. .... | ................. .............V. | .ELLE..- ..............- .F..... | ...... ..... |
| iPS:39 3104 | 21-225_33A7 | VH3\|3-23\|D5\|5-12\|RF3/J H6 | ............ ............ ........ | ......C... | ........ ...... | ....R... ....FH.. .... | ................. .............V. | .ELLE..- ..............- .F..... | ...... ..... |
| iPS:39 3106 | 21-225_34A6 | VH3\|3-23\|D5\|5-12\|RF3/J H6 | ............ ............ .......N | N........N | ........ ...... | ...RR... ....F... .... | ................. ................. | .ELLE..- ..............- ..FA... | ...... ..... |
| iPS:39 3110 | 21-225_35B7 | VH3\|3-23\|D5\|5-12\|RF3/J H6 | ............ ............ ........ | .......... | ........ ...... | ....R... ....FH.. .... | ................. .............V. | .ELLE..- ..............- .F..... | ....A. ..... |
| iPS:39 3118 | 21-225_34H11 | VH3\|3-23\|D5\|5-12\|RF3/J H6 | ............ ............ ........ | .........N | .......E ...... | ....R... ....F... .... | ..............F... ................. | .ELLE..- ..............- ....... | ...... ..... |
| iPS:39 3124 | 21-225_33G7 | VH3\|3-23\|D5\|5-12\|RF3/J H6 | ............ ............ ........ | N......... | ........ ...... | ...RR... ....F... .... | Q................ ................. | .ELLE..- ..............- S...... | ...... ..... |

| iPS ID | Clone | Germline | H_FR1 | H_CDR1 | H_FR2 | H_CDR2 | H_FR3 | H_CDR3 | H_FR4 |
|---|---|---|---|---|---|---|---|---|---|
| iPS:39 3126 | 21-225_35D1 | VH3\|3-23/D5\|5-12\|RF3/JH6 | ...........<br>............<br>........ | .......... | ........<br>...... | ....R...<br>....FH..<br>.... | .........N.......<br>...............V. | .ELLE..−<br>.............−<br>.F..... | ....A.<br>..... |
| iPS:39 3128 | 21-225_35F11 | VH3\|3-23/D5\|5-12\|RF3/JH6 | ...........<br>............<br>........ | .......... | ........<br>...... | ....R...<br>....FH..<br>.M.. | ................<br>...............V. | .ELLE..−<br>.............−<br>.F..... | ...... |
| iPS:39 3146 | 21-225_34G8 | VH3\|3-23/D5\|5-12\|RF3/JH6 | ...........<br>............<br>........ | .......... | ........<br>...... | ....R...<br>....FH..<br>.... | ...............I<br>...............V. | .ELLE..−<br>.............−<br>.F..... | ...... |
| iPS:39 3150 | 21-225_36A5 | VH3\|3-23/D5\|5-12\|RF3/JH6 | ...........<br>............<br>.......N | N........N | ........<br>...... | ...RR...<br>..N.F...<br>.... | ................ | .ELLE..−<br>.............−<br>...A... | ...... |
| iPS:39 3180 | 21-225_4G12 | VH3\|3-23/D5\|5-12\|RF3/JH6 | ...........<br>............<br>........ | .......... | ........<br>...... | TL..R...<br>........<br>.... | .S...............<br>S............... | WGR..S.E.........<br>....−........ | ...... |
| iPS:39 3232 | 21-225_17F12 | VH3\|3-23/D5\|5-12\|RF3/JH6 | ...........<br>............<br>........ | .......... | ........<br>...... | ....G...<br>........<br>.... | .V............... | WGR..N.E.........<br>....−....... | ...... |
| iPS:39 8494 | 21-225_21H4 | VH3\|3-23/D5\|5-12\|RF3/JH6 | ...........<br>............<br>........ | .......... | ........<br>...... | .L..R...<br>........<br>.... | ................ | WGR..S.E.........<br>....−....... | ...... |
| iPS:39 8508 | 21-225_24B1 | VH3\|3-23/D5\|5-12\|RF3/JH6 | ...........<br>............<br>........ | .......... | ........<br>...... | ....R...<br>........<br>.... | ................ | WGR..S.E.........<br>....−....... | ...... |
| iPS:39 8528 | 21-225_32G1 | VH3\|3-23/D5\|5-12\|RF3/JH6 | A...........<br>............<br>........ | .......... | ........<br>...... | ....R...<br>....FH..<br>.... | ................<br>...............V. | .ELLE..−<br>.............−<br>.F..... | ...... |
| iPS:39 8534 | 21-225_33B8 | VH3\|3-23/D5\|5-12\|RF3/JH6 | ...........<br>............<br>........ | .......... | ........<br>...... | ....R...<br>....FH..<br>.... | ................<br>...............V. | .ELLE..−<br>.............−<br>.F..... | ...... |
| iPS:39 8540 | 21-225_35A6 | VH3\|3-23/D5\|5-12\|RF3/JH6 | ...........<br>............<br>........ | .......... | ........<br>...... | T...R...<br>....FH..<br>.... | ................<br>...............V. | .ELLE..−<br>.............−<br>.F..... | ...... |
| | | Germline | H_FR1 | H_CDR1 | H_FR2 | H_CDR2 | H_FR3 | H_CDR3 | H_FR4 |

| | VH4\|4-39/D4\|4-17\|RF2/JH4 | | QLQLQES-GPGLVKPSEILS LTCTVSG-GSIS | SS---SYYWG | WIRQPPGK GLEWIG | SIYY---- SGSTYYNP SLKS | RVTISVDTSKNQFSLKL SSVTAADTAVYYCAR | DYGDY--------------- --------YFDY | WGQGTL VTVSS |
|---|---|---|---|---|---|---|---|---|---|
| IPS:39 2622 | 21-225_17H8 | VH4\|4-39/D4\|4-17\|RF2/JH4 | .......... .......... ........ | R......... | ........R ...... | N....... G.N..... .... | ................ | HGK.W............. ........GL.. | ...... |
| IPS:39 2638 | 21-225_17F9 | VH4\|4-39/D4\|4-17\|RF2/JH4 | .......... .......... ........ | R......... | ........ ...... | N....... ........ .... | .......S.......N. N..........S... | HGK.W............. ........GL.. | ...... |
| IPS:39 2656 | 21-225_1F2 | VH4\|4-39/D4\|4-17\|RF2/JH4 | .......... .......... ........ | R......... | ........ ...... | N....... ...A.N.. ...G | ................ N...........G. | HGK.W............. ........GL.. | ...... |
| IPS:39 2794 | 21-225_21H3 | VH4\|4-39/D4\|4-17\|RF2/JH4 | .......... .......... ........ | R......... | ........ ...... | N....... .....D.. .... | ................ ............G. | HGK.W............. ........GL.. | ...... |
| IPS:39 2822 | 21-225_23C8 | VH4\|4-39/D4\|4-17\|RF2/JH4 | .......... .......... ........ | R......... | ........ ...... | N....... ..T..N.. .... | ...............H..... ............G. | HGK.W............. ........GL.. | ...... |
| IPS:39 2838 | 21-225_22G8 | VH4\|4-39/D4\|4-17\|RF2/JH4 | .......... .......... .......N | R......... | ........ ..D... | N....... ........ .V.. | .F............... ................ | HGK.W............. ........GL.. | ...... |
| IPS:39 2858 | 21-225_22H4 | VH4\|4-39/D4\|4-17\|RF2/JH4 | .......... .......... ........ | R......... | ........ ...... | N....... .....H.. .... | ............M...... T..........F.G. | HGK.W............. ........GL.. | ...... |
| IPS:39 2882 | 21-225_23A3 | VH4\|4-39/D4\|4-17\|RF2/JH4 | .......... .......... ........ | R......... | ........Q ...... | N....... .....N.. .... | ..S............N. ............G. | HGK.W............. ........GL.F | ...... |
| IPS:39 3804 | 21-225_5H7 | VH4\|4-39/D4\|4-17\|RF2/JH4 | .......... .......... .......N | R......... | ........ ...... | N....... ..T..... .... | ................N. ..........S... | HGK.W............. ........GL.. | ...... |
| IPS:39 3832 | 21-225_14B2 | VH4\|4-39/D4\|4-17\|RF2/JH4 | .......... .......... .......N | R......... | ........ ...... | N....... ..T..... .... | ................N. ..........S... | HGK.W............. ........GL.. | ....A. |
| IPS:39 4037 | 21-225_4F4 | VH4\|4-39/D4\|4-17\|RF2/JH4 | .R........ .......... ........ | R......... | ........ ...... | N....... .....D.. .... | ................ .............G. | HGK.W............. ........GL.. | ...... |

| | | | H_FR1 | H_CDR1 | H_FR2 | H_CDR2 | H_FR3 | H_CDR3 | H_FR4 |
|---|---|---|---|---|---|---|---|---|---|
| iPS:39 4045 | 21-225_4H4 | VH4|4-39|D4|4-17|RF2|JH4 | ............<br>............<br>........ | R......... | .......M<br>...... | N.......<br>..N..N..<br>.... | ................<br>N.............G. | HGK.W............<br>........GL.. | ......<br>.I... |
| iPS:39 4079 | 21-225_11F5 | VH4|4-39|D4|4-17|RF2|JH4 | ............<br>............<br>........ | R......... | ........<br>...... | N.......<br>......T..<br>.... | ................H...<br>..............G. | HGK.W............<br>........GL.N | ......<br>..... |
| | **Germline** | | **H_FR1** | **H_CDR1** | **H_FR2** | **H_CDR2** | **H_FR3** | **H_CDR3** | **H_FR4** |
| | **VH3|3-21|D4|4-11|RF2/JH3** | | EVQLVES-GGGLVKPGGSLR LSCAASG-FTFS | S YSMN | WVRQAPGK GLEWVS | SISSS SSYIYYAD SVKG | RFTISRDNAKNSLYLQM NSLRAEDTAVYYCAR | DYSNY ---------DAFDI | WGQGTM VTVSS |
| iPS:39 2624 | 21-225_17H12 | VH3|3-21|D4|4-11|RF2|JH3 | ............<br>............<br>........ | ........T.. | .........<br>...... | ...G....<br>.... | ................<br>................ | .RG--<br>..----S. | ......<br>..... |
| iPS:39 3946 | 21-225_16A4 | VH3|3-21|D4|4-11|RF2|JH3 | ............<br>............<br>........ | ........... | .........<br>...... | ...G....<br>.T.K....<br>.... | ................<br>....T........... | .RG--<br>................<br>..----SY | .....Q<br>..... |
| iPS:39 4008 | 21-225_15H8 | VH3|3-21|D4|4-11|RF2|JH3 | ............<br>............<br>........ | ........V.. | .........<br>...... | ...G....<br>.T...C..<br>.I.. | ................<br>......D......... | .RG--<br>..----S. | ......<br>..... |
| | **Germline** | | **H_FR1** | **H_CDR1** | **H_FR2** | **H_CDR2** | **H_FR3** | **H_CDR3** | **H_FR4** |
| | **VH6|6-01|RF1/JH6** | | QVQLQQS-GPGLVKPSQILS LTCAISG-DSVS | SN---SAAWN | WIRQSPSR GLEWLG | RTYYR-- SKWYNDYA VSVKS | RITINPDTSKNQFSLQL NSVTPEDTAVYYCAR | GYSSGWYY----------<br>----YYYYGMDV | WGQGTT VTVSS |
| iPS:39 2636 | 21-225_17A6 | VH6|6-01|D6|6-19|RF1|JH6 | R....T...S<br>............<br>........ | .........<br>...... | .V..............<br>.... | VS.GWSHH..........<br>............. | ......<br>..... |  |  |
| | **Germline** | | **H_FR1** | **H_CDR1** | **H_FR2** | **H_CDR2** | **H_FR3** | **H_CDR3** | **H_FR4** |
| | **VH6|6-01|D2|2-15|RF2/JH6** | | QVQLQQS-GPGLVKPSQILS LTCAISG-DSVS | SN SAAWN | WIRQSPSR GLEWLG | RTYYR-- SKWYNDYA VSVKS | RITINPDTSKNQFSLQL NSVTPEDTAVYYCAR | GYCSGGSCYS-------<br>----YYYYYGMDV | WGQGTT VTVSS |
| iPS:39 2648 | 21-225_16D11 | VH6|6-01|D2|2-15|RF2|JH6 | R....T...S<br>............<br>........ | .........<br>...... | ................<br>.... | VNSGWSHH--<br>............-<br>........ | ......<br>..... |  |  |
| | **Germline** | | **H_FR1** | **H_CDR1** | **H_FR2** | **H_CDR2** | **H_FR3** | **H_CDR3** | **H_FR4** |
| | **VH3|3-11|D1|1-1|RF3/JH2** | | QVQLVES-GGGLVKPGGSLR LSCAASG-FIFS | D-----YYMS | WIRQAPGK GLEWVS | YISSS--- GSTIYYAD SVKG | RFTISRDNAKNSLYLQM NSLRAEDTAVYYCAR | YNWNDY-----------<br>-------WYFDL | WGRGIL VTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:39 2650 | 21-225_17A4 | VH3\|3-11/D1\|1-1\|RF3/JH 2 | ............ ............ ........ | .......... | ........ ...... | H....... ........ .... | ................ ................ | .RN.R- ................. .G.... | ...... ...... |
| iPS:39 2728 | 21-225_20F7 | VH3\|3-11/D1\|1-1\|RF3/JH 2 | ............ ............ ........ | .......... | ........ ...... | H....... ........ .... | ........GE...... ................ | .RN.R- ................. .G.... | ....S. ...... |
| | Germline | H_FR1 | H_CDR1 | H_FR2 | H_CDR2 | H_FR3 | H_CDR3 | H_FR4 | |
| | VH4\|4-59/D1\|1-26\|RF3/JH4 | | QVQLQES-GPGLVKPSEILS LTCTVSG-GSIS | S     YYWS | WIRQPPGK GLEWIG | YIYY---- SGSTNYNP SLKS | RVTISVDTSKNQFSLKL SSVTAADTAVYYCAR | YSCSY -------YYFDY | WGQGTL VTVSS |
| iPS:39 2676 | 21-225_19F3 | VH4\|4-59/D1\|1-26\|RF3/JH4 | ............ ............ .....A.. | GS...S...G | ........ Q..... | N....... ....Y... .F.. | ................ ......E......... | H.S.W........... .......-SL.. | ...... ...... |
| | Germline | H_FR1 | H_CDR1 | H_FR2 | H_CDR2 | H_FR3 | H_CDR3 | H_FR4 | |
| | VH3\|3-23/D6\|6-13\|RF2/JH2 | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | GIAAAG------------ ------YWYFDL | WGRGTL VTVSS |
| iPS:39 2678 | 21-225_20F3 | VH3\|3-23/D6\|6-13\|RF2/JH2 | ............ ........ | .........N | ........ ...... | V....... ........ .... | ................ ................ | R............... ......TE.... | ...... ...... |
| | Germline | H_FR1 | H_CDR1 | H_FR2 | H_CDR2 | H_FR3 | H_CDR3 | H_FR4 | |
| | VH3\|3-21/D5\|5-24\|RF3/JH4 | | EVQLVES-GGGLVKPGGSLR LSCAASG-FIFS | S-----YSMN | WVRQAPGK GLEWVS | SISSS-- SSYIYYAD SVKG | RFTISRDNAKNSLYLQM NSLRAEDTAVYYCAR | RDGYN------------ -------YYFDY | WGQGTL VTVSS |
| iPS:39 2682 | 21-225_16A12 | VH3\|3-21/D5\|5-24\|RF3/JH4 | ............ ........ | .......R.. | .F...... ...... | ...G.... .TD..... .... | .........E...... ................ | ..--- ..----F | ...... ...... |
| | Germline | H_FR1 | H_CDR1 | H_FR2 | H_CDR2 | H_FR3 | H_CDR3 | H_FR4 | |
| | VH3\|3-48/D7\|7-27\|RF1/JH4 | | EVQLVES-GGGLVQPGGSLR LSCAASG-FIFS | S-----YSMN | WVRQAPGK GLEWVS | YISSS--- SSTIYYAD SVKG | RFTISRDNAKNSLYLQM NSLRDEDTAVYYCAR | LTGY------------ --------FDY | WGQGTL VTVSS |
| iPS:39 2692 | 21-225_18G10 | VH3\|3-48/D7\|7-27\|RF1/JH4 | ...........L.. ....I... | T......... | ........ ...... | ...R.... ....D... .... | ................ | GG.S............. ........P... | ...... ..... |
| iPS:39 2708 | 21-225_18D11 | VH3\|3-48/D7\|7-27\|RF1/JH4 | ............ .......R | ......... | ........ ....L. | ...R.... .G...... .... | .........R...N... ................ | GG.S............. ........P... | ....I. ..... |

| ID | Clone | Germline | H_FR1 | H_CDR1 | H_FR2 | H_CDR2 | H_FR3 | H_CDR3 | H_FR4 |
|---|---|---|---|---|---|---|---|---|---|
| IPS:39 3992 | 21-225_14H8 | VH3\|3-48\|D7\|7-27\|RF1\|JH4 | ............. ............. ........ | ......N... | ........ ...... | ........ ........ .... | ....A........... ............... | GG.S............ ........P... | ...... ..... |
| IPS:39 4055 | 21-225_9C8 | VH3\|3-48\|D7\|7-27\|RF1\|JH4 | ............. ............V V....... | ......Q... | ........ ...... | ...I- ........ ...... | ................ ............... | GG.S............ ........P..S | ...... ..... |
| Germline | VH3\|3-30.3\|D4\|4-11\|RF2\|JH4 | | QVQLVES-GGGVVQPGRSLR LSCAASG-FTFS | S-----YAMH | WVRQAPGK GLEWVA | VISYD---GSNKYYAD SVKG | RFTISRDNSKNTLYLQM NSLRAEDTAVYYCAR | DYSNY-------------YFDY | WGQGTL VTVSS |
| IPS:39 2694 | 21-225_19A5 | VH3\|3-30.3\|D4\|4-11\|RF2\|JH4 | ........... ........... ........ | .......... | ........ ...... | ..WF.... ..D..... .... | ................ ............... | .RAYS............ .......SSS.. | ...... ..... |
| Germline | VH3\|3-23\|D1\|1-1\|RF1\|JH4 | | EVQLLES-GGGLVQPGGSLR LSCAASG-FTFS | S-----YAMS | WVRQAPGK GLEWVS | AISGS---GGSTYYAD SVKG | RFTISRDNSKNTLYLQM NSLRAEDTAVYYCAK | GTTGT-------------YFDY | WGQGTL VTVSS |
| IPS:39 2714 | 21-225_16G12 | VH3\|3-23\|D1\|1-1\|RF1\|JH4 | ............. ............. ......S.. | .........T | ........ ...... | T...R... ..H..... ..R. | ..A....S......... ............... | QD--- ................ ...---C | ...... ..... |
| IPS:39 2890 | 21-225_20H9 | VH3\|3-23\|D1\|1-1\|RF1\|JH4 | ............. ............. ........ | .......... | ........ ...... | ........ ..Y..... .... | .........E....... S............... | .GS-- ................ ...-LF. | ...... ..... |
| IPS:39 2892 | 21-225_20C11 | VH3\|3-23\|D1\|1-1\|RF1\|JH4 | ............. ............. ......S.. | .......... | ........ ...... | T...R... ..H..... .... | ..A....S......... ............... | QD--- ................ ...---C | ...... ..... |
| IPS:39 3968 | 21-225_5A5 | VH3\|3-23\|D1\|1-1\|RF1\|JH4 | ....W....... ............. ........ | .........N | ........ ...... | ........ ..Y..... .... | ................ S............... | .GS-- ................ ...-LF. | ...... ..... |
| Germline | VH3\|3-23\|D6\|6-6\|RF1\|JH3 | | EVQLLES-GGGLVQPGGSLR LSCAASG-FIFS | S-----YAMS | WVRQAPGK GLEWVS | AISGS---GGSTYYAD SVKG | RFTISRDNSKNTLYLQM NSLRAEDTAVYYCAK | EYSSSS---------DAFDI | WGQGTM VTVSS |
| IPS:39 2730 | 21-225_17A1 | VH3\|3-23\|D6\|6-6\|RF1\|JH3 | ............. ............. ........ | .........N | ........ ...... | V....... .SN..... .... | ................ ............... | R.T.DW........... ......H..... | ...... ..... |

| | | | | | | | | | |
|---|---|---|---|---|---|---|---|---|---|
| iPS:39 2736 | 21-225_17B12 | VH3\|3-23/D6\|6-6\|RF1/JH3 | ............ ...........V ........ | .........X ...... .... | ........ ...... | V....... ......... .... | ............... .T............... | R.T.DW............ ......H..... | ...... ..... |
| iPS:39 2770 | 21-225_20C10 | VH3\|3-23/D6\|6-6\|RF1/JH3 | ............ ........... ........ | .........N ...... .... | ........ ...... | V....... ..T..... .... | ............... .T............... | R.T.DW............ ......H..... | ...... ..... |
| iPS:39 3878 | 21-225_7G12 | VH3\|3-23/D6\|6-6\|RF1/JH3 | ............ ........... ........ | .........N ...... .... | ........ ...... | V....... ......... .... | ............... .T............... | R.T.DW............ ......H..... | ...... ..... |
| | | **Germline** | **H_FR1** | **H_CDR1** | **H_FR2** | **H_CDR2** | **H_FR3** | **H_CDR3** | **H_FR4** |
| | **VH3\|3-21/D7\|7-27\|RF3/JH5** | | EVQLVES-GGGLVKPGGSLR LSCAASG-FTFS | S-----YSMN | WVRQAPGK GLEWVS | SISSS--- SSYIYYAD SVKG | RFTISRDNAKNSLYLQM NSLRAEDTAVYYCAR | NWGN-------- --------WFDP | WGQGTL VTVSS |
| iPS:39 2748 | 21-225_20A8 | VH3\|3-21/D7\|7-27\|RF3/JH5 | ............ ........... ........ | .........V. ...... | ...R.... ...... .... | ........ ..FL.... .... | ............V.... ................ | ..-- ................ ....--.Y | ...... ..... |
| | | **Germline** | **H_FR1** | **H_CDR1** | **H_FR2** | **H_CDR2** | **H_FR3** | **H_CDR3** | **H_FR4** |
| | **VH3\|3-30.3/D1\|1-1\|RF1/JH5** | | QVQLVES-GGGVVQPGRSLR LSCAASG-FTFS | S-----YAMH | WVRQAPGK GLEWVA | VISYD--- GSNKYYAD SVKG | RFTISRDNSKNTLYLQM NSLRAEDTAVYYCAR | CTTGT-------- --------NWFDP | WGQGTL VTVSS |
| iPS:39 2754 | 21-225_21D3 | VH3\|3-30.3/D1\|1-1\|RF1/JH5 | ............ ...........V ........ | .......G.. ...... | ........ .....T .... | ........ ......... .... | ................ ................ | .VWF- ................ ..--G.L | ...... ..... |
| iPS:39 2818 | 21-225_22D8 | VH3\|3-30.3/D1\|1-1\|RF1/JH5 | ............ ...........V .......R | .......G.. | ........ .....T .... | I....... ......... .... | ................ ...........F... | .VWF- ................ ..--G.F | ...... ..... |
| | | **Germline** | **H_FR1** | **H_CDR1** | **H_FR2** | **H_CDR2** | **H_FR3** | **H_CDR3** | **H_FR4** |
| | **VH3\|3-23/D6\|6-19\|RF2/JH1** | | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | GIAVAG-------- -----AEYFQH | WGQGTL VTVSS |
| iPS:39 2762 | 21-225_22G5 | VH3\|3-23/D6\|6-19\|RF2/JH1 | ............ ........... ........ | .........N | ......M ...... .... | V..R.... ..Y..... | ................ ..............S | RL............ ......S.A.DI | ...... ..... |
| iPS:39 4051 | 21-225_9E5 | VH3\|3-23/D6\|6-19\|RF2/JH1 | ............ ........... ........ | N........N | ........ ...... .... | ....G... ..N.F... | ................ .G............S | R................ ......S.A.AI | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-39/D1\|1-26\|RF3/JH1 | | QLQLQES-GPGLVKPSETLS LTCTVSG-GSIS | SS---SYYWG | WIRQPPGK GLEWIG | SIYY---- SGSYYNP SLKS | RVTISVDTSKNQFSLKL SSVTAADTAVYYCAR | YSGSYY------------ ------AEYFQH | WGQGTL VTVSS |
| IPS:39 2774 | 21-225_21F3 | VH4\|4-39/D1\|1-26\|RF3/JH1 | ............ ...A........ ........ | R......... | ......... V..... | ........ ....... .... | ................. .........E....S | L.S.W- .................... --D... | ...... ..... |
| IPS:39 3962 | 21-225_7H7 | VH4\|4-39/D1\|1-26\|RF3/JH1 | ............ ............ ........ | R......... | ......... ...... | N....... ......I. .... | ................. ................. | H.T.W- .................... --SLD. | ...... ..... |
| IPS:39 4049 | 21-225_13H5 | VH4\|4-39/D1\|1-26\|RF3/JH1 | ............ ...A........ ........ | R......... | ......... ...... | ........ ........ .... | ................. ...............S | L.S.W- .................... --D... | ...... ..... |
| | VH3\|3-21/D2\|2-21\|RF2/JH4 | Germline | EVQLVES-GGGLVKPGGSLR LSCAASG-FIFS | S-----YSMN | WVRQAPGK GLEWVS | SISSS--- SSYIYYAD SVKG | RFTISRDNAKNSLYLQM NSLRAEDTAVYYCAR | AYCGGDC----------- ------YSYFDY | WGQGTL VTVSS |
| IPS:39 2776 | 21-225_21A12 | VH3\|3-21/D2\|2-21\|RF2/JH4 | ............ ............ .......N | .......... | ......... ...... | ...G.... ........ .... | ................. .........L..... | .AG---- .................-- --... | ...... ..... |
| | VH3\|3-33/D6\|6-19\|RF2/JH6 | Germline | QVQLVES-GGGVVQPGRSLR LSCAASG-FIFS | S---YGMH | WVRQAPGK GLEWVA | VIWYD--- GSNKYYAD SVKG | RFTISRDNSKNTLYLQM NSLRAEDTAVYYCAR | GIAVAGYY---------- ------YYYGMDV | WGQGTT VTVSS |
| IPS:39 2806 | 21-225_24H3 | VH3\|3-33/D6\|6-19\|RF2/JH6 | ............ ...........G ........ | .......... | ......... ...... | ........ ........ .... | ................. ................. | VAVAG--- ...............--- .... | ...... ..... |
| | VH3\|3-48/D7\|7-27\|RF2/JH4 | Germline | EVQLVES-GGGLVQPGGSLR LSCAASG-FIFS | S-----YSMN | WVRQAPGK GLEWVS | YISSS--- SSTIYYAD SVKG | RFTISRDNAKNSLYLQM NSLRDEDTAVYYCAR | *LGY------------- --------FDY | WGQGTL VTVSS |
| IPS:39 2826 | 21-225_20B9 | VH3\|3-48/D7\|7-27\|RF2/JH4 | ............ ............ ........ | .......... | ......... ...... | ........ ........ .... | ................. ................. | S.WS................ .........P... | ...... ..... |
| | VH4\|4-59/D1\|1-1\|RF1/JH5 | Germline | QVQLQES-GPGLVKPSETLS LTCTVSG-GSIS | S-----YYWS | WIRQPPGK GLEWIG | YIYY---- SGSTNYNP SLKS | RVTISVDTSKNQFSLKL SSVTAADTAVYYCAR | GTTGT------------- ------NWFDP | WGQGTL VTVSS |

| | | | H_FR1 | H_CDR1 | H_FR2 | H_CDR2 | H_FR3 | H_CDR3 | H_FR4 |
|---|---|---|---|---|---|---|---|---|---|
| iPS:39 2830 | 21-225_21A5 | VH4\|4-59/D1\|1-1\|RF1/JH 5 | ............ ............ ........ | .......F.. | .....A.. ...... | R..C.... ..I..... | ...M............. ...........I..... | .P.SG.............. .......—.... | ...... ..... |
| | | Germline | H_FR1 | H_CDR1 | H_FR2 | H_CDR2 | H_FR3 | H_CDR3 | H_FR4 |
| | VH3\|3-23/D6\|6-6\|RF2/JH4 | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | SIAAR------------ --------YFDY | WGQGTL VTVSS |
| iPS:39 2840 | 21-225_23G1 | VH3\|3-23/D6\|6-6\|RF2/JH 4 | ............ ............ ........ | .......... | ........R ...... | V....... ..T..NT. .... | .................. .............R | .SL-- .................. ...-... | ...... ..... |
| iPS:39 4018 | 21-225_15B1 | VH3\|3-23/D6\|6-6\|RF2/JH 4 | ............ ............ T....... | .......V.. | ......... ...... | G....... ....NN.. .... | .................. .............R | .SL-- .................. ...-... | ...... ..... |
| iPS:39 4026 | 21-225_16C7 | VH3\|3-23/D6\|6-6\|RF2/JH 4 | ............ ............ ........ | .......V.T | ......... ...... | T....... ..W..... .... | .................. .........E....R | .SL-- .................. ...-... | ...... ..... |
| | | Germline | H_FR1 | H_CDR1 | H_FR2 | H_CDR2 | H_FR3 | H_CDR3 | H_FR4 |
| | VH3\|3-23/D6\|6-6\|RF2/JH5 | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | SIAAR------------- --------NWFDP | WGQGTL VTVSS |
| iPS:39 2842 | 21-225_23G8 | VH3\|3-23/D6\|6-6\|RF2/JH 5 | ............ ............ ........ | .......... | ......... ...... | ......... .... | .................. ................V | .SG-- .................. ...--WFA | ...... ..... |
| | | Germline | H_FR1 | H_CDR1 | H_FR2 | H_CDR2 | H_FR3 | H_CDR3 | H_FR4 |
| | VH3\|3-23/D1\|1-1\|RF2/JH2 | | EVQLLES-GGGLVQPGGSLR LSCAASG-FIFS | S----- YAMS | WVRQAPGK GLEWVS | AISGS--- GGSTYYAD SVKG | RFTISRDNSKNTLYLQM NSLRAEDTAVYYCAK | VQLERY------------ --------WYFDL | WGRGTL VTVSS |
| iPS:39 2856 | 21-225_22A2 | VH3\|3-23/D1\|1-1\|RF2/JH 2 | ............ ............ ........ | .......... | ......... ...... | G....... ..N.P... .... | .......I.......... ................ | .VG--- .................. .--AVH | ...... ..... |
| | | Germline | H_FR1 | H_CDR1 | H_FR2 | H_CDR2 | H_FR3 | H_CDR3 | H_FR4 |
| | VH4\|4-30.1/D5\|5-18\|RF1/JH6 | | QVQLQES-CPGLVKPSQTLS LTCTVSG-GSIS | SG----GYYWS | WIRQHPGK GLEWIG | YIYY---- SGSTYYNP SLKS | RVTISVDTSKNQFSLKL SSVTAADTAVYYCAR | VDTAMVYY---------- YYYCMDV | WGQGTT VTVSS |
| iPS:39 2864 | 21-225_23B9 | VH4\|4-30.1/D5\|5-18\|RF1/J H6 | .....A...... ............ ..D..... | .......... | ......... ...... | ......... .... | .................. ................ | E.G.FG-- .................— ...... | ...... ..... |
| | | Germline | H_FR1 | H_CDR1 | H_FR2 | H_CDR2 | H_FR3 | H_CDR3 | H_FR4 |

| | | Germline | H_FR1 | H_CDR1 | H_FR2 | H_CDR2 | H_FR3 | H_CDR3 | H_FR4 |
|---|---|---|---|---|---|---|---|---|---|
| | | VH3\|3-23/D2\|2-2\|RF2/JH3 | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | GYCSSTSC---------- ------YTDAFDI | WGQGTM VTVSS |
| iPS:39 2874 | 21-225_21D2 | VH3\|3-23/D2\|2-2\|RF2/JH3 | ..K......... ............ .......N | N......... | ........ ...... | VL...... ....F... .... | ................ S....G.....F..R | YCS.ARC- ...............PY. .... | ...... ..... |
| iPS:39 3940 | 21-225_16B2 | VH3\|3-23/D2\|2-2\|RF2/JH3 | ............ ............ ........ | .........T | ........ .P.... | V....... ....F... .... | ................ S..........F..R | YCS.TRC- ...............PY. .... | ...... ...... |
| iPS:39 3956 | 21-225_4D7 | VH3\|3-23/D2\|2-2\|RF2/JH3 | ..K......... ............ ........ | .......... | ........ ...... | VL...... ....F... .... | ................ S....G.....F..R | YCS.ARC- ...............PY. .... | ...... |
| iPS:39 8476 | 21-225_17C1 | VH3\|3-23/D2\|2-2\|RF2/JH3 | ............ E........... ........ | .......... | ........ ...... | V....... ..T.F... .... | ................ S..........F..R | YCS.TRC- ...............PY. .... | ...... ...... |
| | | Germline | H_FR1 | H_CDR1 | H_FR2 | H_CDR2 | H_FR3 | H_CDR3 | H_FR4 |
| | | VH3\|3-15/D1\|1-1\|RF3/JH4 | EVQLVES-GGGLVKPGGSLR LSCAASG-FTFS | N-----AWMS | WVRQAPGK GLEWVG | RIKSKT- DGGTTDYA APVKG | RFTISRDDSKNTLYLQM NSLKTEDTAVYYCTT | YNWND------------- --------YFDY | WGQGTL VTVSS |
| iPS:39 2898 | 21-225_21H10 | VH3\|3-15/D1\|1-1\|RF3/JH4 | ............ ............ ........ | .........N | ........ ...... | ........ .... | ................ | EG..- ...-T.. | ...... |
| iPS:39 3802 | 21-225_3D12 | VH3\|3-15/D1\|1-1\|RF3/JH4 | ............ ............ ....V... | T.........N | ........ ...... | ...N.I... ......V. .... | ................ | EG..- ................ ...-T.. | ...... |
| | | Germline | H_FR1 | H_CDR1 | H_FR2 | H_CDR2 | H_FR3 | H_CDR3 | H_FR4 |
| | | VH3\|3-48/D4\|4-11\|RF3/JH4 | EVQLVES-GGGLVQPGGSLR LSCAASG-FTFS | S-----YSMN | WVRQAPGK GLEWVS | YISSS--- SSTIYYAD SVKG | RFTISRDNAKNSLYLQM NSLRDEDTAVYYCAR | TTVT-------------- --------YFDY | WGQGTL VTVSS |
| iPS:39 2950 | 21-225_25C10 | VH3\|3-48/D4\|4-11\|RF3/JH4 | ............ ............ ........ | ........R.. | ........ ...... | S....... ........ .... | ................ | .AG- ....-... | ...... |
| | | Germline | H_FR1 | H_CDR1 | H_FR2 | H_CDR2 | H_FR3 | H_CDR3 | H_FR4 |
| | | VH3\|3-23/D1\|1-26\|RF3/JH4 | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | YSGSY------------- --------YYFDY | WGQGTL VTVSS |

EP 4 435 105 A2

| | | | H_FR1 | H_CDR1 | H_FR2 | H_CDR2 | H_FR3 | H_CDR3 | H_FR4 |
|---|---|---|---|---|---|---|---|---|---|
| iPS:39 3010 | 21-225_25E11 | VH3|3-23/D1|1-26|RF3/JH4 | .................... | .......... | ........ V...G... | .................. | RGY.GYE.......... .....DLL...C | ...... ..... |
| | **VH3|3-23/D3|3-3|RF3/JH3** | | EVQLLES-GGGLVQPGGSLR LSCAASG-FIFS | S-----YAMS | WVRQAPGK GLEWVS | AISGS---GGSTYYAD SVKG | RFTISRDNSKNTLYLQM NSLRAEDTAVYYCAK | ITIFGVV-----------------IIDAFDI | WGQGTM VTVSS |
| iPS:39 3016 | 21-225_28F11 | VH3|3-23/D3|3-3|RF3/JH3 | .................... .....I.. | .......... | ........ VT...... ..T.F... .... | .................. | R.Q.D-- ..................---D... | ...... ..... |
| | | Germline | H_FR1 | H_CDR1 | H_FR2 | H_CDR2 | H_FR3 | H_CDR3 | H_FR4 |
| | **VH3|3-33/D3|3-22|RF2/JH6** | | QVQLVES-GGGVVQPGRSLR LSCAASG-FIFS | S-----YGMH | WVRQAPGK GLEWVA | VIWYD---GSNKYYAD SVKG | RFTISRDNSKNTLYLQM NSLRAEDTAVYYCAR | YYYDSSGYYY--------------YYYYYGMDV | WGQGTT VTVSS |
| iPS:39 3032 | 21-225_26F8 | VH3|3-33/D3|3-22|RF2/JH6 | .................... | G......... | ........ I....... .... | .................. | ER..FW---- ............---SGC... | ...... ..... |
| | | Germline | H_FR1 | H_CDR1 | H_FR2 | H_CDR2 | H_FR3 | H_CDR3 | H_FR4 |
| | **VH1|1-02/D4|4-11|RF2/JH6** | | QVQLVQS-GAEVKKPGASVK VSCKASG-YITF | G-----YYMH | WVRQAPGQ GLEWMG | WINPN---SGGTNYAQ KFQG | RVTMTRDTSISTAYMEL SRLRSDDTAVYYCAR | DYSNYYY-------------YYYGMDV | WGQGTT VTVSS |
| iPS:39 3042 | 21-225_31F1 | VH1|1-02/D4|4-11|RF2/JH6 | .................... | D......... | ........ ........ .... | ..........M...... | .S..FSNW.......... ....YD...... | ...... ..... |
| iPS:39 3108 | 21-225_34G11 | VH1|1-02/D4|4-11|RF2/JH6 | .................... | .......... | ........ ........ .... | .................. | .I..FSSW.......... ....YD..A... | ...... ..... |
| | | Germline | H_FR1 | H_CDR1 | H_FR2 | H_CDR2 | H_FR3 | H_CDR3 | H_FR4 |
| | **VH1|1-18/D5|5-12|RF3/JH4** | | QVQLVQS-GAEVKKPGASVK VSCKASG-YITF | S-----YGIS | WVRQAPGQ GLEWMG | WISAY---NGNTNYAQ KLQG | RVTMTTDTSTSTAYMEL RSLRSDDTAVYYCAR | GYSGYD-------------------YYFDY | WGQGTL VTVSS |
| iPS:39 3044 | 21-225_25B8 | VH1|1-18/D5|5-12|RF3/JH4 | .................... | .......... | ........ ....T... ..R. | ..................D. | TAA...S............ ......SSW... | ...... ..... |
| iPS:39 3050 | 21-225_28C5 | VH1|1-18/D5|5-12|RF3/JH4 | .................... ..D..... | .......... | ........ ....T... ..R. | ..................D. | TAA...S............ ......SSW... | ...... ..... |
| | | Germline | H_FR1 | H_CDR1 | H_FR2 | H_CDR2 | H_FR3 | H_CDR3 | H_FR4 |

| ID | Clone | Germline | H_FR1 | H_CDR1 | H_FR2 | H_CDR2 | H_FR3 | H_CDR3 | H_FR4 |
|---|---|---|---|---|---|---|---|---|---|
|  |  | VH3\|3-33/D6\|6-6\|RF1/JH3 | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | EYSSSS-------------------DAFDI | WGQGTMVTVSS |
| iPS:39 3046 | 21-225_25A12 | VH3\|3-33/D6\|6-6\|RF1/JH3 | ..............................  ........ | N......CV.. | .......... | ........ .... | ................ | .EY..GW........... .....YDYGM.V | ...... ..... |
|  | Germline |  | H_FR1 | H_CDR1 | H_FR2 | H_CDR2 | H_FR3 | H_CDR3 | H_FR4 |
|  |  | VH3\|3-21/D4\|4-11\|RF3/JH6 | EVQLVES-GGGLVKPGGSLRLSCAASG-FIFS | S---YSMN | WVRQAPGKGLEWVS | SISSS-SSYIYYADSVKG | RFTISRDNAKNSLYLQMNSLRAEDTAVYYCAR | TTVTYYY------YYGMDV | WGQGTTVTVSS |
| iPS:39 3078 | 21-225_33H11 | VH3\|3-21/D4\|4-11\|RF3/JH6 | ..............................  ........ | .......... | .......... | ...G.... .... | S............... | .NG---- --... | ..... |
| iPS:39 3142 | 21-225_33A3 | VH3\|3-21/D4\|4-11\|RF3/JH6 | ..............................  ........ | .......G.. | .......... | ...G.... .I...... .... | ................ | .NG---- --... | ..... |
|  | Germline |  | H_FR1 | H_CDR1 | H_FR2 | H_CDR2 | H_FR3 | H_CDR3 | H_FR4 |
|  |  | VH3\|3-23/D6\|6-19\|RF2/JH6 | EVQLLES-GGGLVQPGGSLRLSCAASG-FIFS | S-----YAMS | WVRQAPGKGLEWVS | AISGS---GGSTYYADSVKG | RFTISRDNSKNTLYLQMNSLRAEDTAVYYCAK | GIAVAGYY----------YYYGMDV | WGQGTTVTVSS |
| iPS:39 3096 | 21-225_34D11 | VH3\|3-23/D6\|6-19\|RF2/JH6 | ....S.......  ............  ........ | .........N | .......... | ....R... ....FH.. .... | ................V. | .EL.ED.- ................F. | ..... |
|  | Germline |  | H_FR1 | H_CDR1 | H_FR2 | H_CDR2 | H_FR3 | H_CDR3 | H_FR4 |
|  |  | VH3\|3-30.3/D4\|4-23\|RF2/JH3 | QVQLVES-GGGVVQPGRSLRLSCAASG-FIFS | S-----YAMH | WVRQAPGKGLEWVA | VISYD---GSNKYYADSVKG | RFTISRDNSKNTLYLQMNSLRAEDTAVYYCAR | DYGGNS-------DAFDI | WGQGTMVTVSS |
| iPS:39 3172 | 21-225_3B12 | VH3\|3-30.3/D4\|4-23\|RF2/JH3 | ..H.........  ............  ........ | Y......G.. | .......... | ........ .... | ..............H... | .RR.GYG........... .....VP..... | ..... |
|  | Germline |  | H_FR1 | H_CDR1 | H_FR2 | H_CDR2 | H_FR3 | H_CDR3 | H_FR4 |
|  |  | VH3\|3-30.3/D4\|4-23\|RF2/JH6 | QVQLVES-GGGVVQPGRSLRLSCAASG-FIFS | S-----YAMH | WVRQAPGKGLEWVA | VISYD---GSNKYYADSVKG | RFTISRDNSKNTLYLQMNSLRAEDTAVYYCAR | DYGGNSYY----------YYYGMDV | WGQGTTVTVSS |
| iPS:39 3174 | 21-225_15D8 | VH3\|3-30.3/D4\|4-23\|RF2/JH6 | ............  ..T.........  ........ | G......G.. | ........ .....S | ........ .... | ................ | .RVYC.STSCV....... ..PYYD...... | ...... ..... |
|  | Germline |  | H_FR1 | H_CDR1 | H_FR2 | H_CDR2 | H_FR3 | H_CDR3 | H_FR4 |

| | | Germline | EVQLVES-GGGLVKPGGSLR LSCAASG-FIFS | N-----AWMS | WVRQAPGK GLEWVG | RIKSKT-DGGTTDYA APVKG | RFTISRDDSKNTLYLQM NSLKTEDTAVYYCTT | LTGYYY------------ ------YYGMDV | WGQGTT VTVSS |
|---|---|---|---|---|---|---|---|---|---|
| **VH3\|3-15\|D7\|7-27\|RF1\|JH6** | | | | | | | | | |
| iPS:39 3194 | 21-225_16D2 | VH3\|3-15\|D7\|7-27\|RF1\|JH6 | ............ ............ ........ | .........N | ........ ...... .... | ........ ........ | ................. H................ | D..PIAARLA........ ...YYY..A... | ...... ..... |
| iPS:39 8488 | 21-225_19F6 | VH3\|3-15\|D7\|7-27\|RF1\|JH6 | ............ ............ ........ | .........N | ........ ...... .... | ........ ........ | ................. ................. | D..PIAARLA........ ...YYY..A... | ..H... ..... |
| iPS:39 8544 | 21-225_7C8 | VH3\|3-15\|D7\|7-27\|RF1\|JH6 | ............ ............ ........ | .......R.N | ...L.... ...... .... | ........ ........ | .......E.E.... ........G....S. | D..PIAARLA........ ...YYY..A... | ...... ..... |
| iPS:40 2231 | 21-225_6D9 | VH3\|3-15\|D7\|7-27\|RF1\|JH6 | ............ ............ ........ | .........N | ...L.... ...... .... | ........ ........ | .........E.... ...............S. | D..PIAARLA........ ...YYY..A... | ...... ..... |
| | | Germline | H_FR1 | H_CDR1 | H_FR2 | H_CDR2 | H_FR3 | H_CDR3 | H_FR4 |
| **VH3\|3-23\|D4\|4-11\|RF2\|JH4** | | | EVQLLES-GGGLVQPGGSLR LSCAASG-FIFS | S-----YAMS | WVRQAPGK GLEWVS | AISGS---GGSTYYAD SVKG | RFTISRDNSKNTLYLQM NSLRAEDTAVYYCAK | DYSNY------------- --------YFDY | WGQGTL VTVSS |
| iPS:39 3870 | 21-225_7B1 | VH3\|3-23\|D4\|4-11\|RF2\|JH4 | ............ ............ ........ | .......D.. | ........ ...... .... | T....... ..I..... .... | ..........K.... ...........F..R | .RG-- ................. ...--SV | ...... ..... |
| | | Germline | H_FR1 | H_CDR1 | H_FR2 | H_CDR2 | H_FR3 | H_CDR3 | H_FR4 |
| **VH4\|4-39\|D4\|4-17\|RF2\|JH1** | | | QLQLQES-GPGLVKPSEILS LTCIVSG-GSIS | SS---SYYWG | WIRQPPGK GLEWIG | SIYY----SGSTYYNP SLKS | RVTISVDTSKNQFSLKL SSVTAADTAVYYCAR | DYGDYA------------ --------EYFQH | WGQGTL VTVSS |
| iPS:39 3872 | 21-225_2A11 | VH4\|4-39\|D4\|4-17\|RF2\|JH1 | ............ ............ ........ | R......... | ........ ...... .V.. | N....... ........ .... | .T............. | HGK.W- ................. .-GLED | ...... ..... |
| | | Germline | H_FR1 | H_CDR1 | H_FR2 | H_CDR2 | H_FR3 | H_CDR3 | H_FR4 |
| **VH4\|4-59\|D6\|6-6\|RF1\|JH4** | | | QVQLQES-GPGLVKPSEILS LTCIVSG-GSIS | S-----YYWS | WIRQPPGK GLEWIG | YIYY----SGSTNYNP SLKS | RVTISVDTSKNQFSLKL SSVTAADTAVYYCAR | EYSSS------------- --------SYFDY | WGQGTL VTVSS |
| iPS:39 3890 | 21-225_4B1 | VH4\|4-59\|D6\|6-6\|RF1\|JH4 | ............ ............S ....D... | .......S.. | .....A.. ...... .... | R..T.... ......I. | .I.M......K...... ................ | DLK..G............ ......CLF... | ...... ..... |
| | | Germline | H_FR1 | H_CDR1 | H_FR2 | H_CDR2 | H_FR3 | H_CDR3 | H_FR4 |

| | | H_FR1 | H_CDR1 | H_FR2 | H_CDR2 | H_FR3 | H_CDR3 | H_FR4 |
|---|---|---|---|---|---|---|---|---|
| | VH3\|3-33/D4\|4-17\|RF1/JH6 | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | *LR*LYY------------ -----YYYGMDV | WGQGTT VTVSS |
| iPS:39 3926 21-225_4G4 | VH3\|3-33/D4\|4-17\|RF1/JH6 | ............ ............ ....... | .......... | ........ ....G. .... | ...H.... ....... .... | ........A........ ................ | D..MG-- ................-- -.... | ...... ..... |
| | Germline | H_FR1 | H_CDR1 | H_FR2 | H_CDR2 | H_FR3 | H_CDR3 | H_FR4 |
| | VH3\|3-23/D6\|6-6\|RF2/JH2 | EVQLLES-GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | SIAARY------------ ------WYFDL | WGRGTL VTVSS |
| iPS:39 3936 21-225_14A11 | VH3\|3-23/D6\|6-6\|RF2/JH2 | ............ ............ ....... | .........N | ........ ...... | V...R... ........ .... | ................ | R...GM............ .......E.... | ...... ..... |
| | Germline | H_FR1 | H_CDR1 | H_FR2 | H_CDR2 | H_FR3 | H_CDR3 | H_FR4 |
| | VH3\|3-21/D1\|1-1\|RF3/JH4 | EVQLVES-GGGLVKPGGSLR LSCAASG-FTFS | S-----YSMN | WVRQAPGK GLEWVS | SISSS--- SSYIYYAD SVKG | RFTISRDNAKNSLYLQM NSLRAEDTAVYYCAR | YNWND------------ -----YFDY | WGQGTL VTVSS |
| iPS:39 3988 21-225_7F10 | VH3\|3-21/D1\|1-1\|RF3/JH4 | ............ ............ ....... | .......... | ........ ...... | ........ ........ .... | ................ ....T......... | A.L-- ................ ...-... | ...... ..... |
| | Germline | H_FR1 | H_CDR1 | H_FR2 | H_CDR2 | H_FR3 | H_CDR3 | H_FR4 |
| | VH4\|4-39/D6\|6-6\|RF1/JH4 | QLQLQES-GPGLVKPSEILS LTCTVSG-GSIS | SS----SYYWG | WIRQPPGK GLEWIG | SIYY---- SGSTYYNP SLKS | RVTISVDTSKNQFSLKL SSVTAADTAVYYCAR | EYSSS------------ ------SYFDY | WGQGTL VTVSS |
| iPS:39 3990 21-225_11G7 | VH4\|4-39/D6\|6-6\|RF1/JH4 | ............ ............ .....F.. | R....T.... | ........ ...... | ........ ....S.S. .... | ................ | LN..W............ ........-S... | ...... ..... |
| | Germline | H_FR1 | H_CDR1 | H_FR2 | H_CDR2 | H_FR3 | H_CDR3 | H_FR4 |
| | VH3\|3-30.3/D2\|2-15\|RF3/JH6 | QVQLVES-GGGVVQPGRSLR LSCAASG-FTFS | S-----YAMH | WVRQAPGK GLEWVA | VISYD--- GSNKYYAD SVKG | RFTISRDNSKNTLYLQM NSLRAEDTAVYYCAR | DIVVVVAAT--------- ----YYYYYGMDV | WGQGTT VTVSS |
| iPS:39 4010 21-225_12G5 | VH3\|3-30.3/D2\|2-15\|RF3/JH6 | ............ ............ ....... | N......GIY | ........ ...... | ........ ........ .... | ................ | .RGA.A.Y- .............-- ....I.. | ...... ..... |
| | Germline | H_FR1 | H_CDR1 | H_FR2 | H_CDR2 | H_FR3 | H_CDR3 | H_FR4 |
| | VH1\|1-08/D2\|2-21\|RF1/JH4 | QVQLVQS-GAEVKKPGASVK VSCKASG-YIFT | S-----YDIN | WVRQATGQ GLEWMG | WMNPN--- SGNTGYAQ KFQG | RVTMTRNTSISTAYMEL SSLRSEDTAVYYCAR | SILWW*L ------LFYFDY | WGQGTL VTVSS |

3336

EP 4 435 105 A2

| | | | H_FR1 | H_CDR1 | H_FR2 | H_CDR2 | H_FR3 | H_CDR3 | H_FR4 |
|---|---|---|---|---|---|---|---|---|---|
| iPS:39 4065 | 21-225_11E2 | VH1\|1-08/D2\|2-21\|RF1/JH4 | ...V........ .............. ........ | N......... | ......... ...... .... | ...T.... ......... | ..............,D. ................Y | .HG.F-- ..............- -L... | ....I. ..... |
| iPS:39 8506 | 21-225_23G12 | VH1\|1-08/D2\|2-21\|RF1/JH4 | ....L........ .............. ........ | N......... | ......... ...... .... | ..Y..... ......... | ......M........... ................I | .GG.Y-- ..............- -.... | ...... |
| iPS:39 8512 | 21-225_25E12 | VH1\|1-08/D2\|2-21\|RF1/JH4 | ............ .............. ........ | N......... | ......... ...... .... | ......... ......... | ...............L... ................G | .NG.Y-- ..............- -.... | ...... |
| Germline | | | H_FR1 | H_CDR1 | H_FR2 | H_CDR2 | H_FR3 | H_CDR3 | H_FR4 |
| | VH3\|3-33/D7\|7-27\|RF2/JH3 | | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | *LGD----------------AFDI | WGQGTMVTVSS |
| iPS:39 4091 | 21-225_13H3 | VH3\|3-33/D7\|7-27\|RF2/JH3 | ...........L .............. ........ | .......... | ......... ...... | ....E... E.....V. ..R. | ..........S...... ..............V. | E..F............... ........QS.F | .....P ..... |
| Germline | | | H_FR1 | H_CDR1 | H_FR2 | H_CDR2 | H_FR3 | H_CDR3 | H_FR4 |
| | VH3\|3-23/D4\|4-23\|RF2/JH6 | | EVQLLES-GGGLVQPGGSLRLSCAASG-FIFS | S-----YAMS | WVRQAPGKGLEWVS | ATSGS---GGSTYYADSVKG | RFTISRDNSKNTLYLQMNSLRAEDTAVYYCAK | DYGGNSYY----------------YYYGMDV | WGQGTTVTVSS |
| iPS:39 8472 | 21-225_16E4 | VH3\|3-23/D4\|4-23\|RF2/JH6 | ............ I........... ........ | .......V.. | ......... ...... | T..VG... ..T..... .... | ................. ............. | WGR....E.......... ............. | ...... ..... |
| Germline | | | H_FR1 | H_CDR1 | H_FR2 | H_CDR2 | H_FR3 | H_CDR3 | H_FR4 |
| | VH2\|2-05/D6\|6-19\|RF2/JH4 | | QITLKES-GPTLVKPTQILTLTCTFSG-FSLS | TS---GVGVG | WIRQPPGKALEWLA | LIYW----NDDKRYSPSLKS | RLTIIKDTSKNQVVLTMTNMDPVDTATYYCAH | GIAVA-----------------GYFDY | WGQGTLVTVSS |
| iPS:39 8498 | 21-225_22E6 | VH2\|2-05/D6\|6-19\|RF2/JH4 | ............ .............. ........ | .G........ | ......... ...... | ........ .... | ......R........... ................. | T....R............. ........-G... | ...... ..... |
| Germline | | | H_FR1 | H_CDR1 | H_FR2 | H_CDR2 | H_FR3 | H_CDR3 | H_FR4 |
| | VH1\|1-08/D5\|5-24\|RF2/JH4 | | QVQLVQS-CAEVKKPGASVKVSCKASG-YIFT | S-----YDIN | WVRQATGQGLEWMC | WMNPN----SCNTGYAQKFQG | RVTMTRNTSISTAYMELSSLRSEDTAVYYCAR | *RWLQ--------------LYFDY | WGQGTLVTVSS |
| iPS:39 8536 | 21-225_33D12 | VH1\|1-08/D5\|5-24\|RF2/JH4 | ............ .............. ........ | .........S | ...L.... ...... | ......... .... | ................. ................. | K.A-- ..--N.. | ...... ..... |
| Germline | | | H_FR1 | H_CDR1 | H_FR2 | H_CDR2 | H_FR3 | H_CDR3 | H_FR4 |

| | | H_FR1 | H_CDR1 | H_FR2 | H_CDR2 | H_FR3 | H_CDR3 | H_FR4 |
|---|---|---|---|---|---|---|---|---|
| | VH2\|2-05/D6\|6-6\|RF1/JH4 | QITLKES-GPTLVKPTQILTLTCTFSG-FSLS | TS---GVGVG | WIRQPPGKALEWLA | LIYW----NDDKRYSPSLKS | RLTITKDTSKNQVVLTMTNMDPVDTATYYCAE | EYSSS-------------------SYFDY | WGQGTLVTVSS |
| iPS:398546 | 21-225_9H10 VH2\|2-05/D6\|6-6\|RF1/JH4 | .................T | .......... | ...............  | S........... ...... | ...A............... | TG..C............ .......C.... | ...... ..... |
| | Germline | H_FR1 | H_CDR1 | H_FR2 | H_CDR2 | H_FR3 | H_CDR3 | H_FR4 |
| | VH3\|3-21/D1\|1-1\|RF2/JH6 | EVQLVES-GGGLVKPGGSLRLSCAASG-FIFS | S-----YSMN | WVRQAPGKGLEWVS | SISSS---SSYIYYADSVKG | RFTISRDNAKNSLYLQMNSLRAEDTAVYYCAR | VQLERYY-----------------YYYGMDV | WGQGTTVTVSS |
| iPS:402229 | 21-225_16H9 VH3\|3-21/D1\|1-1\|RF2/JH6 | ................ | .......... | ........ ...... | ...G.... .... | ................... | .NG---- .................---- --... | ...... ..... |
| | Germline | H_FR1 | H_CDR1 | H_FR2 | H_CDR2 | H_FR3 | H_CDR3 | H_FR4 |
| | VH3\|3-21/D4\|4-11\|RF3/JH5 | EVQLVES-GGGLVKPGGSLRLSCAASG-FIFS | S-----YSMN | WVRQAPGKGLEWVS | SISSS---SSYIYYADSVKG | RFTISRDNAKNSLYLQMNSLRAEDTAVYYCAR | TTVTN----------------WFDP | WGQGTLVTVSS |
| iPS:402233 | 21-225_16D10 VH3\|3-21/D4\|4-11\|RF3/JH5 | ................ | T......NL. | ........ ...... | ...GG... AGH...S. .... | ................. | .NG-- ................... ...-..F | ...... ..... |
| | Germline | H_FR1 | H_CDR1 | H_FR2 | H_CDR2 | H_FR3 | H_CDR3 | H_FR4 |
| | VH3\|3-21/D1\|1-1\|RF1/JH3 | EVQLVES-GGGLVKPGGSLRLSCAASG-FIFS | S-----YSMN | WVRQAPGKGLEWVS | SISSS---SSYIYYADSVKG | RFTISRDNAKNSLYLQMNSLRAEDTAVYYCAR | GTTGT--------DAFDI | WGQGTMVTVSS |
| iPS:402235 | 21-225_20F10 VH3\|3-21/D1\|1-1\|RF1/JH3 | ................ | .......... | ........ ...... | ...T- ....TF.. ....... | ................. | KAG-- .................. ..--L.. | ...... ..... |
| | Germline | H_FR1 | H_CDR1 | H_FR2 | H_CDR2 | H_FR3 | H_CDR3 | H_FR4 |
| | VH3\|3-23/D5\|5-12\|RF1/JH3 | EVQLLES-GGGLVQPGGSLRLSCAASG-FIFS | S-----YAMS | WVRQAPGKGLEWVS | AISGS---GGSTYYADSVKG | RFTISRDNSKNTLYLQMNSLRAEDTAVYYCAK | VDIVAT--------------IDAFDI | WGQGTMVTVSS |
| iPS:403870 | 21-225_23G4 VH3\|3-23/D5\|5-12\|RF1/JH3 | ................ | .......... | ........ ...... | V...R... .... | ................... | RG..GA............ ......TE.... | ...... ..... |
| | Germline | H_FR1 | H_CDR1 | H_FR2 | H_CDR2 | H_FR3 | H_CDR3 | H_FR4 |
| | VH4\|4-39/D4\|4-11\|RF1/JH4 | QLQLQES-GPGLVKPSEILSLTCTVSG-GSIS | SS---SYYWG | WIRQPPGKGLEWIG | SIYY----SGSTYYNPSLKS | RVTISVDTSKNQFSLKLSSVTAADTAVYYCAR | *LQ*L---------------YFDY | WGQGTLVTVSS |

| ID | Clone | Germline | H_FR1 | H_CDR1 | H_FR2 | H_CDR2 | H_FR3 | H_CDR3 | H_FR4 |
|---|---|---|---|---|---|---|---|---|---|
| iPS:40 3872 | 21-225_8F11 | VH4\|4-39/D4\|4-11\|RF1/JH4 | ............ .Q.......... ....V... | RC........ | .L...... ...... | N....... ...A.N.. .... | ................ .............G. | HG.DW............. ........GL.. | ...... ..... |
| | | Germline | H_FR1 | H_CDR1 | H_FR2 | H_CDR2 | H_FR3 | H_CDR3 | H_FR4 |
| VH1\|1-08/D3\|3-9\|RF2/JH6 | | | QVQLVQS-GAEVKKPGASVK VSCKASG-YIFT | S-----YDIN | WVRQATGQ GLEWMG | WMNPN--- SGNTGYAQ KFQG | RVTMTRNTSISTAYMEL SSLRSEDTAVYYCAR | -YYDILTGYYN------ ----YYYYYGMDV | WGQGTT VTVSS |
| iPS:43 7240 | 21-225_84H12 | VH1\|1-08/D3\|3-9\|RF2/JH6 | ............ ............ ........ | .......... | ........ ...... | .L..H... ........ .... | .I...W....R...... ................ | GF.......S- ..........PT....D. .. | ...... ..... |
| iPS:43 4577 | 21-225_75C11 | VH1\|1-08/D3\|3-9\|RF2/JH6 | ............ ............ ........ | .......... | ........ ...... | .L..H... ........ .... | .I...W....R...... ................ | GF.......S- ..........PT....D. .. | ...... ..... |
| iPS:43 4553 | 21-225_76H12 | VH1\|1-08/D3\|3-9\|RF2/JH6 | ............ ............ ........ | .......... | ........ ...... | .L..H... ........ .... | .I...W....R...... ................ | GF.......S- ..........PT....D. .. | ...... ..... |
| iPS:43 4927 | 21-225_86E5 | VH1\|1-08/D3\|3-9\|RF2/JH6 | ............ ............ ........ | .......... | ........ ...... | .L..H... ........ .... | .....W....R...... ................ | GF.......S- ..........PT....D. .. | ...... ..... |
| | | Germline | H_FR1 | H_CDR1 | H_FR2 | H_CDR2 | H_FR3 | H_CDR3 | H_FR4 |
| VH3\|3-23/D6\|6-19\|RF2/JH4 | | | EVQLLES-GGGLVQPGGSLR LSCAASG-FIFS | S-----YAMS | WVRQAPGK GLEWVS | AISGS--- GGSTYYAD SVKG | RFTISRDNSKNTLYLQM NSLRAEDTAVYYCAK | -GIAVA------------ -------GYFDY | WGQGTL VTVSS |
| iPS:43 5477 | 21-225_154E8 | VH3\|3-23/D6\|6-19\|RF2/JH4 | ............ ............ ........ | .......... | ........R ...... | ........ ..N.F... .... | ................ ...............I | H.....GT.......... ......GAH.... | ....... A.... |
| iPS:43 5385 | 21-225_149G7 | VH3\|3-23/D6\|6-19\|RF2/JH4 | ............ ............ ........ | .......... | ........ ...... | ........ ..N.F... .... | ................ ...............I | H.....GT.......... ......GAH.... | ....... A.... |

Figure 53 (Table 6)
Standard IgG Antibody Variable Region Protein Alignment
CDRs defined by Kabat
Alignment numbering defined by Amgen Reference (AHo)

| KAPPA_ VARIABLE | | Germline | K_FR1 | K_CDR1 | K_FR2 | K_CDR2 | K_FR3 | K_CDR3 | K_FR4 |
|---|---|---|---|---|---|---|---|---|---|
| | VK4\|B3/J K1 | | DIVMTQSPDSLAVSLG ERATINC | KSS QSVLYSSNNKNY LA | WYQQKPGQPPK LLIY | W-------- ASTRES | GVPDRFSGSGSG-- TDFTLIISSLQAEDVAVY YC | QQYYS----------- -----------TPWT | FGQGTK VEIK |
| IPS:39 2928 | 21- 225_25A4 | VK4\|B3/J K1 | DIVMTQFPDSLAVSLG ERATIKC | KSS--- QSVLYSSHNNNY LA | WYQQKPGQPPK LLLY | W-------- ASTRES | GVPDRFSGSGSG-- TEFTLTIISSLEAEDVAVY YC | QQYYS----------- -----------TPPT | FGQGTK VEIK |
| IPS:42 4419 | 21- 225_25A4 .001 | VK4\|B3/J K1 | DIVMTQFPDSLAVSLG ERATIKC | KSS--- QSVLYSSHNNNY LA | WYQQKPGQPPK LLLY | W-------- ASTRES | GVPDRFSGSGSG-- IEFTLTIISSLEAEDVAVY YC | QQYYS----------- -----------TPPT | FGQGTK VEIK |
| IPS:44 1468 | 21- 225_25A4 .001.001 | VK4\|B3/J K1 | DIVMTQFPDSLAVSLG ERATIKC | KSS--- QSVLYSSHNNNY LA | WYQQKPGQPPK LLLY | W-------- ASTRES | GVPDRFSGSGSG-- IEFTLTIISSLEAEDVAVY YC | QQYYS----------- -----------TPPT | FGQGTK VEIK |
| IPS:44 1475 | 21- 225_25A4 .001.002 | VK4\|B3/J K1 | DIVMTQFPDSLAVSLG ERATIKC | KSS--- QSVLYSSHNNNY LA | WYQQKPGQPPK LLLY | W-------- ASTRES | GVPDRFSGSGSG-- TEFTLTISSLEAEDVAVY YC | QQYYS----------- -----------TPPT | FGQGTK VEIK |
| IPS:44 1482 | 21- 225_25A4 .001.003 | VK4\|B3/J K1 | DIVMTQFPDSLAVSLG ERATIKC | KSS--- QSVLYSSHNNNY LA | WYQQKPGQPPK LLLY | W-------- ASTRES | GVPDRFSGSGSG-- TEFTLTISSLEAEDVAVY YC | QQYYS----------- -----------TPPT | FGQGTK VEIK |
| IPS:44 1489 | 21- 225_25A4 .001.004 | VK4\|B3/J K1 | DIVMTQFPDSLAVSLG ERATIKC | KSS--- QSVLYSSHNNNY LA | WYQQKPGQPPK LLLY | W-------- ASTRES | GVPDRFSGSGSG-- TEFTLTISSLEAEDVAVY YC | QQYYS----------- -----------TPPT | FGQGTK VEIK |
| IPS:44 1496 | 21- 225_25A4 .001.005 | VK4\|B3/J K1 | DIVMTQSPDSLAVSLG ERATIKC | KSS--- QSVLYSSHNNNY LA | WYQQKPGQPPK LLLY | W-------- ASTRES | GVPDRFSGSGSG-- TEFTLTISSLQAEDVAVY YC | QQYYS----------- -----------TPPT | FGQGTK VEIK |
| IPS:44 1505 | 21- 225_25A4 .001.006 | VK4\|B3/J K1 | DIVMTQSPDSLAVSLG ERATIKC | KSS--- QSVLYSSHNNNY LA | WYQQKPGQPPK LLLY | W-------- ASTRES | GVPDRFSGSGSG-- TEFTLTISSLQAEDVAVY YC | QQYYS----------- -----------TPPT | FGQGTK VEIK |
| IPS:44 1512 | 21- 225_25A4 .001.007 | VK4\|B3/J K1 | DIVMTQSPDSLAVSLG ERATIKC | KSS--- QSVLYSSHNNNY LA | WYQQKPGQPPK LLLY | W-------- ASTRES | GVPDRFSGSGSG-- TEFTLTISSLQAEDVAVY YC | QQYYS----------- -----------TPPT | FGQGTK VEIK |
| IPS:44 1519 | 21- 225_25A4 .001.008 | VK4\|B3/J K1 | DIVMTQSPDSLAVSLG ERATIKC | KSS--- QSVLYSSHNNNY LA | WYQQKPGQPPK LLLY | W-------- ASTRES | GVPDRFSGSGSG-- TEFTLTISSLQAEDVAVY YC | QQYYS----------- -----------TPPT | FGQGTK VEIK |
| IPS:44 1554 | 21- 225_25A4 .001.013 | VK4\|B3/J K1 | DIVMTQFPDSLAVSLG ERATIKC | KSS--- QSVLYSSHNNNY LA | WYQQKPGQPPK LLLY | W-------- ASTRES | GVPDRFSGSGSG-- TEFTLTISSLQAEDVAVY YC | QQYYS----------- -----------TPPT | FGQGTK VEIK |
| IPS:44 1595 | 21- 225_25A4 .001.019 | VK4\|B3/J K1 | DIVMTQSPDSLAVSLG ERATINC | KSS--- QSVLYSSHNNNY LA | WYQQKPGQPPK LLLY | W-------- ASTRES | GVPDRFSGSGSG-- TEFTLTISSLEAEDVAVY YC | QQYYS----------- -----------TPPT | FGQGTK VEIK |

| | | | Germline | K_FR1 | K_CDR1 | K_FR2 | K_CDR2 | K_FR3 | K_CDR3 | K_FR4 |
|---|---|---|---|---|---|---|---|---|---|---|
| iPS:44 1604 | 21-225_25A4.001.020 | VK4\|B3/J K1 | DIVMTQSPDSLAVSLG ERATINC | KSS--QSVLYSSHNNNY LA | WYQQKPGQPPK LLLY | W--------ASTRES | GVPDRFSGSGSG--TEFTLTIISSLQAEDVAVY YC | QQYYS------------ -----------TPPT | FGQGTK VEIK |
| iPS:44 1613 | 21-225_25A4.001.021 | VK4\|B3/J K1 | DIVMTQSPDSLAVSLG ERATINC | KSS--QSVLYSSHNNNY LA | WYQQKPGQPPK LLLY | W--------ASTRES | GVPDRFSGSGSG--TEFTLTIISSLQAEDVAVY YC | QQYYS------------ -----------TPPT | FGQGTK VEIK |
| iPS:44 3006 | 21-225_25A4.001.029 | VK4\|B3/J K1 | DIVMTQFPDSLAVSLG ERATIXC | KSS--QSVLYSSHNNNY LA | WYQQKPGQPPK LLLY | W--------ASTRES | GVPDRFSGSGSG--TEFTLTIISSLQAEDVAVY YC | QQYYS------------ -----------TPPT | FGQGTK VEIK |
| iPS:44 1962 | 21-225_4A2.001.023 | VK4\|B3/J K1 | DIVMTQSPDSLAVSLG ERATINC | KSS--QSILHSSNNNNY LA | WFQQKPGQPPK LLLY | W--------ASTRES | GVPDRFSGSGSG--IDFTLTISSLQPEDVAVY YC | QQYYS------------ -----------TPVT | FGQGTK VEIK |
| iPS:44 1999 | 21-225_4A2.001.028 | VK4\|B3/J K1 | DIVMTQSPDSLAVSLG ERATINC | KSS--QSILHSSNNNNY LA | WFQQKPGQPPK LLLY | W--------ASTRES | GVPDRFSGSGSG--IDFTLTISSLQPEDVAVY YC | QQYYS------------ -----------TPVT | FGQGTK VEIK |
| iPS:44 2006 | 21-225_4A2.001.029 | VK4\|B3/J K1 | DIVMTQSPDSLAVSLG ERATINC | KSS--QSILHSSNNNNY LA | WFQQKPGQPPK LLLY | W--------ASTRES | GVPDRFSGSGSG--TDFTLTISSLQPEDVAVY YC | QQYYS------------ -----------TPVT | FGQGTK VEIK |
| iPS:44 2020 | 21-225_4A2.001.031 | VK4\|B3/J K1 | DIVMTQSPDSLAVSLG ERATINC | KSS--QSILHSSNNNNY LA | WFQQKPGQPPK LLLY | W--------ASTRES | GVPDRFSGSGSG--TDFTLTISSLQPEDVAVY YC | QQYYN------------ -----------TPVT | FGPGTK VEIK |
| | VK1\|L5/J K3 | | DIQMTQSPSSVSASVG DRVTITC | RAS--QGIS--- ---SWLA | WYQQKPGKAPK LLIY | A--------ASSLQS | GVPSRFSGSGSG--TDFTLTISSLQPEDFATY YC | QQANS------ -------FPFT | FGPGTK VDIK |
| iPS:39 3954 | 21-225_4H6 | VK1\|L5/J K3 | DIQMTQSPSSVSASVG DRVTITC | RAS--QGIS--- ---RWLA | WYQQKPGKAPK LLIY | G--------ASSLQS | GVPSRFSGSGSG--TDFTLTISSLQPEDFAIY YC | QQANS------------ -----------FPFT | FGPGTK VDIK |
| iPS:44 2050 | 21-225_4H6.004 | VK1\|L5/J K3 | DIQMTQSPSSVSASVG DRVTITC | RAS--QGIS--- ---RWLA | WYQQKPGKAPK LLIY | G--------ASSLQS | GVPSRFSGSGSG--TDFTLTISSLQPEDFAIY YC | QQANS------------ -----------FPFT | FGPGTK VDIK |
| iPS:44 2059 | 21-225_4H6.005 | VK1\|L5/J K3 | DIQMTQSPSSVSASVG DRVTITC | RAS--QGIS--- ---RWLA | WYQQKPGKAPK LLIY | G--------ASSLQS | GVPSRFSGSGSG--TDFTLTISSLQPEDFATY YC | QQANS------------ -----------FPFT | FGQGTK VDIK |
| iPS:44 2065 | 21-225_4H6.006 | VK1\|L5/J K3 | DIQMTQSPSVSASVG DRVTITC | RAS--QGIS--- ---RWLA | WYQQKPGKAPK LLIY | G--------ASSLQS | GVPSRFSGSGSG--TDFTLTISSLQPEDFATY YC | QQANS------------ -----------FPFT | FGQGTK VDIK |
| iPS:44 2071 | 21-225_4H6.007 | VK1\|L5/J K3 | DIQMTQSPSSVSASVG DRVTITC | RAS--QGIS--- ---RWLA | WYQQKPGKAPK LLIY | G--------ASSLQS | GVPSRFSGSGSG--TDFTLTISSLQPEDFAIY YC | QQANS------------ -----------FPFT | FGPGTK VDIK |
| iPS:44 2078 | 21-225_4H6.008 | VK1\|L5/J K3 | DIQMTQSPSSVSASVG DRVTITC | RAS--QGIS--- ---RWLA | WYQQKPGKAPK LLIY | G--------ASSLQS | GVPSRFSGSGSG--TDFTLTISSLQPEDFAIY YC | QQANS------------ -----------FPFT | FGPGTK VDIK |
| iPS:44 2085 | 21-225_4H6.009 | VK1\|L5/J K3 | DIQMTQSPSSVSASVG DRVTITC | RAS--QGIS--- ---RWLA | WYQQKPGKAPK LLIY | G--------ASSLQS | GVPSRFSGSGSG--TDFTLTISSLQPEDFAIY YC | QQANS------------ -----------FPFT | FGQGTK VDIK |

| | | | K_FR1 / Germline | K_CDR1 | K_FR2 | K_CDR2 | K_FR3 | K_CDR3 | K_FR4 |
|---|---|---|---|---|---|---|---|---|---|
| iPS:44 2089 | 21-225_4H6.010 | VK1\|L5/J K3 | DIQMTQSPSSVSASVG DRVTITC | RAS--QGIS-----RWLA | WYQQKPGKAPK LLIY | G-------- ASSLQS | GVPSRFSGSGSG--TDFTLTISSLQPEDFAIY YC | QQANS----------------FPFT | FGQGTK VDIK |
| iPS:44 2093 | 21-225_4H6.011 | VK1\|L5/J K3 | DIQMTQSPSSVSASVG DRVTITC | RAS--QGIS-----RWLA | WYQQKPGKAPK LLIY | G-------- ASSLQS | GVPSRFSGSGSG--TDFTLTISSLQPEDFAIY YC | QQANS----------------FPFT | FGQGTK VDIK |
| iPS:44 3016 | 21-225_4H6.014 | VK1\|L5/J K3 | DIQMTQSPSSVSASVG DRVTITC | RAS--QGTS-----RWLA | WYQQKPGKAPK LLIY | G-------- ASSLQS | GVPSRFSGSGSG--TDFTLTISSLQPEDFATY YC | QQANS----------------FPFT | FGPGTK VDIK |
| | | Germline | K_FR1 | K_CDR1 | K_FR2 | K_CDR2 | K_FR3 | K_CDR3 | K_FR4 |
| | VK4\|B3/J K3 | | DIVMTQSPDSLAVSLG ERATINC | KSS--QSVLYSSNNKNY LA | WYQQKPGQPPK LLIY | W-------- ASTRES | GVPDRFSGSGSG--TDFTLTISSLQAEDVAVY YC | QQYYS------------TPFT | FGPGTK VDIK |
| iPS:41 2232 | 21-225_4A2 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYN------------TPVT | FGPGTK VGIK |
| iPS:42 2894 | 21-225_4A2.001 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYN------------TPVT | FGPGTK VGIK |
| iPS:44 1841 | 21-225_4A2.001.001 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYS------------TPVT | FGPGTK VGIK |
| iPS:44 1847 | 21-225_4A2.001.002 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYN------------APVT | FGPGTK VGIK |
| iPS:44 1853 | 21-225_4A2.001.003 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYQ------------TPVT | FGPGTK VGIK |
| iPS:44 1859 | 21-225_4A2.001.004 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYN------------TPVT | FGPGTK VGIK |
| iPS:44 1866 | 21-225_4A2.001.005 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYN------------TPVT | FGPGTK VGIK |
| iPS:44 1873 | 21-225_4A2.001.006 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYN------------TPVT | FGPGTK VGIK |
| iPS:44 1880 | 21-225_4A2.001.007 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYS------------TPVT | FGPGTK VGIK |
| iPS:44 1884 | 21-225_4A2.001.008 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYS------------TPVT | FGPGTK VGIK |
| iPS:44 1888 | 21-225_4A2.001.009 | VK4\|B3/J K3 | DIVMTQSPDSLAVSLG ERATINC | KSS--QSILHSSNNNNY LA | WFQQKPGQPPK LLLY | W-------- ASTRES | GVPDRFSGSGSG--TDFTLTISSLQPEDVAVY YC | QQYYN------------APVT | FGPGTK VGIK |

| | | | K_FR1 | K_CDR1 | K_FR2 | K_CDR2 | K_FR3 | K_CDR3 | K_FR4 |
|---|---|---|---|---|---|---|---|---|---|
| iPS:44 1892 | 21-225_4A2. 001.010 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYQ------------ -----------TPVT | FGPGTK VGIK |
| IPS:44 1896 | 21-225_4A2. 001.011 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYQ------------ -----------TPVT | FGPGTK VGIK |
| iPS:44 1900 | 21-225_4A2. 001.012 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYQ------------ -----------TPVT | FGPGTK VGIK |
| iPS:44 1955 | 21-225_4A2. 001.022 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS------------ -----------TPVT | FGPGTK VGIK |
| iPS:44 1971 | 21-225_4A2. 001.024 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS------------ -----------TPVT | FGPGTK VGIK |
| Germline | | K_FR1 | K_CDR1 | K_FR2 | K_CDR2 | K_FR3 | K_CDR3 | K_FR4 | |
| | VK1\|A30/ JK1 | | DIQMTQSPSSLSASVG DRVTITC | RAS--QGIR-- ---NDLG | WYQQKPGKAPK RLIY | A-------- ASSLQS | GVPSRFSGSGSG-- TEFTLTISSLQPEDFATY YC | LQHNS------------ -----------YPWT | FGQGTK VEIK |
| IPS:39 4041 | 21-225_5E5 | VK1\|A30/ JK1 | DIQMTQSPSSLSASVG DRVTITC | RAS--QGIR-- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2115 | 21-225_5E5. 003 | VK1\|A30/ JK1 | DIQMTQSPSSLSASVG DRVTITC | RAS--QGIR-- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2122 | 21-225_5E5. 004 | VK1\|A30/ JK1 | DIQMTQSPSSLSASVG DRVTITC | RAS--QGIR-- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2129 | 21-225_5E5. 005 | VK1\|A30/ JK1 | DIQMTQSPSSLSASVG DRVTITC | RAS--QGIR-- ---NDLG | WYQQKPGKAPK RLIY | A-------- ASSLQS | GVPSRFSGSGSG-- TEFTLIISSLQPEDFATY YC | LQHYS------------ -----------YPRT | FGQGTK VEVK |
| iPS:44 2136 | 21-225_5E5. 006 | VK1\|A30/ JK1 | DIQMTQSPSSLSASVG DRVTITC | RAS--QGIR-- ---NDLG | WYQQKPGKAPK RLIY | A-------- ASSLQS | GVPSRFSGSGSG-- TEFTLIISSLQPEDFATY YC | LQHYS------------ -----------YPRT | FGQGTK VEVK |
| iPS:44 2171 | 21-225_5E5. 011 | VK1\|A30/ JK1 | DIQMTQSPSSLSASVG DRVTITC | RAS--QGIR-- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2178 | 21-225_5E5. 012 | VK1\|A30/ JK1 | DIQMTQSPSSLSASVG DRVTITC | RAS--QGIR-- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2199 | 21-225_5E5. 015 | VK1\|A30/ JK1 | DIQMTQSPSSLSASVG DRVTITC | RAS--QGIR-- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2206 | 21-225_5E5. 016 | VK1\|A30/ JK1 | DIQMTQSPSSLSASVG DRVTITC | RAS--QGIR-- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |

| | | Germline | K_FR1 | K_CDR1 | K_FR2 | K_CDR2 | K_FR3 | K_CDR3 | K_FR4 |
|---|---|---|---|---|---|---|---|---|---|
| IPS:44 2213 | 21-225_5E5. 017 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2220 | 21-225_5E5. 018 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2227 | 21-225_5E5. 019 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2255 | 21-225_5E5. 023 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2262 | 21-225_5E5. 024 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| IPS:44 2269 | 21-225_5E5. 025 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| | | VK1\|O12/ JK4 | DIQMTQSPSSLSASVG DRVTITC | RAS--QSIS--- ---SYLN | WYQQKPGKAPK LLIY | A-------- ASSLQS | GVPSRFSGSGSG-- TDFTLTISSLQPEDFATY YC | QQSYS------------ -----------TPLT | FGGGTK VEIK |
| IPS:39 3930 | 21-225_7E11 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| IPS:42 4460 | 21-225_7E11 .001 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| IPS:44 2311 | 21-225_7E11 .001.001 | VK1\|O12/ JK4 | DIQMTQSPSSLSASVG DRVTITC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| IPS:44 2317 | 21-225_7E11 .001.002 | VK1\|O12/ JK4 | DIQMTQSPSSLSASVG DRVTITC | RAS--QNII--- ---SYLN | WYQQKPGKAPK LLIY | T-------- ASSLQS | GVPSRFSGSGSG-- TDFTLTISSLQPEDFATY YC | QQTYS------------ -----------TPLT | FGGGTK VEIK |
| IPS:44 2323 | 21-225_7E11 .001.003 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| IPS:44 2330 | 21-225_7E11 .001.004 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| IPS:44 2337 | 21-225_7E11 .001.005 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| IPS:44 2344 | 21-225_7E11 .001.006 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |

| | | | | | | | | | | |
|---|---|---|---|---|---|---|---|---|---|---|
| iPS:44 2351 | 21-225_7E11 .001.007 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| iPS:44 2358 | 21-225_7E11 .001.008 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| iPS:44 2365 | 21-225_7E11 .001.009 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| iPS:44 2372 | 21-225_7E11 .001.010 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| iPS:44 2379 | 21-225_7E11 .001.011 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| iPS:44 2386 | 21-225_7E11 .001.012 | VK1\|O12/ JK4 | DIQMTQSPSSLSASVG DRVTITC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| iPS:44 2390 | 21-225_7E11 .001.013 | VK1\|O12/ JK4 | DIQMTQSPSSLSASVG DRVTITC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| iPS:44 2394 | 21-225_7E11 .001.014 | VK1\|O12/ JK4 | DIQMTQSPSSLSASVG DRVTITC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| iPS:44 2398 | 21-225_7E11 .001.015 | VK1\|O12/ JK4 | DIQMTQSPSSLSASVG DRVIITC | RAS--QNII--- ---SYLN | WYQQKPGKAPK LLIY | T-------- ASSLQS | GVPSRFSGSGSG-- TDFTLTISSLQPEDFATY YC | QQTYS------------ -----------TPLT | FGGGTK VEIK |
| iPS:44 2402 | 21-225_7E11 .001.016 | VK1\|O12/ JK4 | DIQMTQSPSSLSASVG DRVIITC | RAS--QNII--- ---SYLN | WYQQKPGKAPK LLIY | T-------- ASSLQS | GVPSRFSGSGSG-- TDFTLTISSLQPEDFATY YC | QQTYS------------ -----------TPLT | FGGGTK VEIK |
| iPS:44 2406 | 21-225_7E11 .001.017 | VK1\|O12/ JK4 | DIQMTQSPSSLSASVG DRVIITC | RAS--QNII--- ---SYLN | WYQQKPGKAPK LLIY | T-------- ASSLQS | GVPSRFSGSGSG-- TDFTLTISSLQPEDFATY YC | QQTYS------------ -----------TPLT | FGGGTK VEIK |
| iPS:44 2410 | 21-225_7E11 .001.018 | VK1\|O12/ JK4 | DIQMTQSPSSLSASVG DRVIITC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGTK VEIK |
| iPS:44 2417 | 21-225_7E11 .001.019 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGIK VEIK |
| iPS:44 2431 | 21-225_7E11 .001.021 | VK1\|O12/ JK4 | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGIK VEIK |
| iPS:44 2438 | 21-225_7E11 .001.022 | VK1\|O12/ JK4 | DIQMTQSPSSLSASVG DRVTITC | RAS--QNII--- ---SYLN | WYQQKPGKAPK FLIY | T-------- ASSLQS | GVPSRFSGSGSG-- TDFTLTISSLQPEDFAIY YC | QQTYS------------ -----------TPLT | FGGGIK VEIK |

| iPS:44 3027 | 21-225_7E11 .001.023 | VK1|O12/ JK4 | DIQMTQSPSSLSASVG DRVTIAC | RAS--QNII--- ---SYLN | WYQQKPCKAPK FLIY | T-------- ASSLQS | GVPSRFSGSGSG-- TDFTLTISSLQPEDFAIY YC | QQTYS------------ ------------TPLT | FGGGTK VEIK |

**HEAVY_VARIABLE**

| | Germline | H_FR1 | H_CDR1 | H_FR2 | H_CDR2 | H_FR3 | H_CDR3 | H_FR4 |
|---|---|---|---|---|---|---|---|---|
| | VH1|1- 08/D6|6- 19|RF1/J H4 | QVQLVQS- GAEVKKPGASVKVSCK ASG-YTFT | S-----YDIN | WVRQATGQGLE WMG | WMNPN--- SGNTGYAQK FQG | RVTMTRNISISTAYMELS SLRSEDTAVYYCAR | GYSSGW------------ --------YYFDY | WGQGTL VTVSS |
| iPS:39 2928 | 21-225_25A4 | VH1|1- 08/D6|6- 19|RF1/J H4 | QVLLVQS- GAEVKRPGASVKVSCK ASG-YTFT | N-----YDIN | WVRQATGQGLE WMG | WMYPN--- SGNTGYAQK FQG | RVTMTRNISISTAYMELS SLRSEDTAVYYCAS | SSGWY------------ ---------YFDY | WGQGTL VTVSS |
| iPS:42 4419 | 21-225_25A4 .001 | VH1|1- 08/D6|6- 19|RF1/J H4 | QVLLVQS- GAEVKRPGASVKVSCK ASG-YTFT | N-----YDIN | WVRQATGQGLE WMG | WMYPN--- SGNTGYAQK FQG | RVTMTRDTSISTAYMELS SLRSEDTAVYYCAS | SSGWY------------ ---------YFDY | WGQGTL VTVSS |
| iPS:44 1468 | 21-225_25A4 .001.001 | VH1|1- 08/D6|6- 19|RF1/J H4 | QVLLVQS- GAEVKRPGASVKVSCK ASG-YTFT | N-----YDIN | WVRQATGQGLE WMG | WMYPN--- SGSTGYAQK FQG | RVTMTRDTSISTAYMELS SLRSEDTAVYYCAS | SSGWY------------ ---------YFDY | WGQGTL VTVSS |
| iPS:44 1475 | 21-225_25A4 .001.002 | VH1|1- 08/D6|6- 19|RF1/J H4 | QVLLVQS- GAEVKRPGASVKVSCK ASG-YTFT | N-----YDIN | WVRQATGQGLE WMG | WMYPN--- SGNAGYAQK FQG | RVTMTRDTSISTAYMELS SLRSEDTAVYYCAS | SSGWY------------ ---------YFDY | WGQGTL VTVSS |
| iPS:44 1482 | 21-225_25A4 .001.003 | VH1|1- 08/D6|6- 19|RF1/J H4 | QVLLVQS- GAEVKRPGASVKVSCK ASG-YTFT | N-----YDIN | WVRQATGQGLE WMG | WMYPN--- SGNVGYAQK FQG | RVTMTRDTSISTAYMELS SLRSEDTAVYYCAS | SSGWY------------ ---------YFDY | WGQGTL VTVSS |
| iPS:44 1489 | 21-225_25A4 .001.004 | VH1|1- 08/D6|6- 19|RF1/J H4 | QVLLVQS- GAEVKRPGASVKVSCK ASG-YTFT | N-----YDIN | WVRQATGQGLE WMG | WMYPN--- SGQTGYAQK FQG | RVTMTRDTSISTAYMELS SLRSEDTAVYYCAS | SSGWY------------ ---------YFDY | WGQGTL VTVSS |
| iPS:44 1496 | 21-225_25A4 .001.005 | VH1|1- 08/D6|6- 19|RF1/J H4 | QVQLVQS- GAEVKRPGASVKVSCK ASG-YTFT | N-----YDIN | WVRQATGQGLE WMG | WMYPN--- SGSTGYAQK FQG | RVTMTRDTSISTAYMELS SLRSEDTAVYYCAS | SSGWY------------ ---------YFDY | WGQGTL VTVSS |
| iPS:44 1505 | 21-225_25A4 .001.006 | VH1|1- 08/D6|6- 19|RF1/J H4 | QVQLVQS- GAEVKRPGASVKVSCK ASG-YTFT | N-----YDIN | WVRQATGQGLE WMG | WMYPN--- SGNAGYAQK FQG | RVTMTRDTSISTAYMELS SLRSEDTAVYYCAS | SSGWY------------ ---------YFDY | WGQGTL VTVSS |
| iPS:44 1512 | 21-225_25A4 .001.007 | VH1|1- 08/D6|6- 19|RF1/J H4 | QVQLVQS- GAEVKRPGASVKVSCK ASG-YTFT | N-----YDIN | WVRQATGQGLE WMG | WMYPN--- SGNVGYAQK FQG | RVTMTRDTSISTAYMELS SLRSEDTAVYYCAS | SSGWY------------ ---------YFDY | WGQGTL VTVSS |

| | | | | | | | | | |
|---|---|---|---|---|---|---|---|---|---|
| iPS:44<br>1519 | 21-<br>225_25A4<br>.001.008 | VH1\|1-<br>08/D6\|6-<br>19\|RF1\|J<br>H4 | QVQLVQS-<br>GAEVKRPGASVKVSCK<br>ASG-YTFT | N-----YDIN | WVRQATGQGLE<br>WMG | WMYPN---<br>SGQTGYAQK<br>FQG | RVTMTRDISISTAYMELS<br>SLRSEDTAVYYCAS | SSGWY-----------<br>---------YFDY | WGQGTL<br>VTVSS |
| iPS:44<br>1554 | 21-<br>225_25A4<br>.001.013 | VH1\|1-<br>08/D6\|6-<br>19\|RF1\|J<br>H4 | QVLLVQS-<br>GAEVKRPGASVKVSCK<br>ASG-YTFT | N-----YDIN | WVRQATGQGLE<br>WMG | WMYPN---<br>SGSTGYAQK<br>FQG | RVTMTRDISISTAYMELS<br>SLRSEDTAVYYCAS | SSGWY-----------<br>---------YFDY | WGQGTL<br>VTVSS |
| iPS:44<br>1595 | 21-<br>225_25A4<br>.001.019 | VH1\|1-<br>08/D6\|6-<br>19\|RF1\|J<br>H4 | QVQLVQS-<br>GAEVKRPGASVKVSCK<br>ASG-YTFT | N-----YDIN | WVRQAPGQGLE<br>WMG | WMYPN---<br>SGSTGYAQK<br>FQG | RVTMTRDISISTAYMELS<br>SLRSEDTAVYYCAS | SSGWY-----------<br>---------YFDY | WGQGTL<br>VTVSS |
| iPS:44<br>1604 | 21-<br>225_25A4<br>.001.020 | VH1\|1-<br>08/D6\|6-<br>19\|RF1\|J<br>H4 | QVLLVQS-<br>GAEVKRPGASVKVSCK<br>ASG-YTFT | N-----YDIN | WVRQAPGQGLE<br>WMG | WMYPN---<br>SGQTGYAQK<br>FQG | RVTMTRDISISTAYMELS<br>SLRSEDTAVYYCAS | SSGWY-----------<br>---------YFDY | WGQGTL<br>VTVSS |
| iPS:44<br>1613 | 21-<br>225_25A4<br>.001.021 | VH1\|1-<br>08/D6\|6-<br>19\|RF1\|J<br>H4 | QVQLVQS-<br>GAEVKRPGASVKVSCK<br>ASG-YTFT | N-----YDIN | WVRQATGQGLE<br>WMG | WMYPN---<br>SGNAGYAQK<br>FQG | RVTMTRDISISTAYMELS<br>SLRSEDTAVYYCAS | SSGWY-----------<br>---------YFDY | WGQGTL<br>VTVSS |
| iPS:44<br>3006 | 21-<br>225_25A4<br>.001.029 | VH1\|1-<br>08/D6\|6-<br>19\|RF1\|J<br>H4 | QVLLVQS-<br>GAEVKRPGASVKVSCK<br>ASG-YTFT | N-----YDIN | WVRQATGQGLE<br>WMG | WMYPN---<br>SGQTGYAQK<br>FQG | RVTMTRDISISTAYMELS<br>SLRSEDTAVYYCAS | SSGWY-----------<br>---------YFDY | WGQGTL<br>VTVSS |
| iPS:41<br>2232 | 21-<br>225_4A2 | VH1\|1-<br>08/D6\|6-<br>19\|RF1\|J<br>H4 | QVQLVQS-<br>GTEVKKPGASVKVSCK<br>ASG-YTFT | N-----YDIN | WVRQATGQGLE<br>WMG | WMHPN---<br>SGNTGYAQK<br>FQG | RVTLTRNTSISTAYMELS<br>SLRSEDTAVYYCAS | SSGWY-----------<br>---------YFDY | WGQGTL<br>VTVSS |
| iPS:42<br>2894 | 21-<br>225_4A2.<br>001 | VH1\|1-<br>08/D6\|6-<br>19\|RF1\|J<br>H4 | QVQLVQS-<br>GTEVKKPGASVKVSCK<br>ASG-YTFT | N-----YDIN | WVRQATGQGLE<br>WMG | WMHPN---<br>SGNTGYAQK<br>FQG | RVTLTRDISISTAYMELS<br>SLRSEDTAVYYCAS | SSGWY-----------<br>---------YFDY | WGQGTL<br>VTVSS |
| iPS:44<br>1841 | 21-<br>225_4A2.<br>001.001 | VH1\|1-<br>08/D6\|6-<br>19\|RF1\|J<br>H4 | QVQLVQS-<br>GTEVKKPGASVKVSCK<br>ASG-YTFT | N-----YDIN | WVRQATGQGLE<br>WMG | WMHPN---<br>SGNTGYAQK<br>FQG | RVTLTRDISISTAYMELS<br>SLRSEDTAVYYCAS | SSGWY-----------<br>---------YFDY | WGQGTL<br>VTVSS |
| iPS:44<br>1847 | 21-<br>225_4A2.<br>001.002 | VH1\|1-<br>08/D6\|6-<br>19\|RF1\|J<br>H4 | QVQLVQS-<br>GTEVKKPGASVKVSCK<br>ASG-YTFT | N-----YDIN | WVRQATGQGLE<br>WMG | WMHPN---<br>SGNTGYAQK<br>FQG | RVTLTRDISISTAYMELS<br>SLRSEDTAVYYCAS | SSGWY-----------<br>---------YFDY | WGQGTL<br>VTVSS |
| iPS:44<br>1853 | 21-<br>225_4A2.<br>001.003 | VH1\|1-<br>08/D6\|6-<br>19\|RF1\|J<br>H4 | QVQLVQS-<br>GTEVKKPGASVKVSCK<br>ASG-YTFT | N-----YDIN | WVRQATGQGLE<br>WMG | WMHPN---<br>SGNTGYAQK<br>FQG | RVTLTRDTSISTAYMELS<br>SLRSEDTAVYYCAS | SSGWY-----------<br>---------YFDY | WGQGTL<br>VTVSS |
| iPS:44<br>1859 | 21-<br>225_4A2.<br>001.004 | VH1\|1-<br>08/D6\|6-<br>19\|RF1\|J<br>H4 | QVQLVQS-<br>GTEVKKPGASVKVSCK<br>ASG-YTFT | N-----YDIN | WVRQATGQGLE<br>WMG | WMHPN---<br>SGNAGYAQK<br>FQG | RVTLTRDTSISTAYMELS<br>SLRSEDTAVYYCAS | SSGWY-----------<br>---------YFDY | WGQGTL<br>VTVSS |

| | | | | | | | | | |
|---|---|---|---|---|---|---|---|---|---|
| iPS:44 1866 | 21-225_4A2.001.005 | VH1|1-08/D6|6-19|RF1/JH4 | QVQLVQS-GTEVKKPGASVKVSCK ASG-YTFT | N-----YDIN | WVRQATCQGLE WMG | WMHPN---SGSTGYAQK FQG | RVTLTRDISISTAYMELS SLRSEDTAVYYCAS | SSGWY------------------YFDY | WGQGTL VTVSS |
| iPS:44 1873 | 21-225_4A2.001.006 | VH1|1-08/D6|6-19|RF1/JH4 | QVQLVQS-GTEVKKPGASVKVSCK ASG-YTFT | N-----YDIN | WVRQATGQGLE WMG | WMHPN---SGQTGYAQK FQG | RVTLTRDISISTAYMELS SLRSEDTAVYYCAS | SSGWY------------------YFDY | WGQGTL VTVSS |
| iPS:44 1880 | 21-225_4A2.001.007 | VH1|1-08/D6|6-19|RF1/JH4 | QVQLVQS-GTEVKKPGASVKVSCK ASG-YTFT | N-----YDIN | WVRQATGQGLE WMG | WMHPN---SGQTGYAQK FQG | RVTLTRDISISTAYMELS SLRSEDTAVYYCAS | SSGWY------------------YFDY | WGQGTL VTVSS |
| iPS:44 1884 | 21-225_4A2.001.008 | VH1|1-08/D6|6-19|RF1/JH4 | QVQLVQS-GTEVKKPGASVKVSCK ASG-YTFT | N-----YDIN | WVRQATGQGLE WMG | WMHPN---SGNAGYAQK FQG | RVTLTRDISISTAYMELS SLRSEDTAVYYCAS | SSGWY------------------YFDY | WGQGTL VTVSS |
| iPS:44 1888 | 21-225_4A2.001.009 | VH1|1-08/D6|6-19|RF1/JH4 | QVQLVQS-GTEVKKPGASVKVSCK ASG-YTFT | N-----YDIN | WVRQATGQGLE WMG | WMHPN---SGQTGYAQK FQG | RVTLTRDISISTAYMELS SLRSEDTAVYYCAS | SSGWY------------------YFDY | WGQGTL VTVSS |
| iPS:44 1892 | 21-225_4A2.001.010 | VH1|1-08/D6|6-19|RF1/JH4 | QVQLVQS-GTEVKKPGASVKVSCK ASG-YTFT | N-----YDIN | WVRQATGQGLE WMG | WMHPN---SGNAGYAQK FQG | RVTLTRDISISTAYMELS SLRSEDTAVYYCAS | SSGWY------------------YFDY | WGQGTL VTVSS |
| iPS:44 1896 | 21-225_4A2.001.011 | VH1|1-08/D6|6-19|RF1/JH4 | QVQLVQS-GTEVKKPGASVKVSCK ASG-YTFT | N-----YDIN | WVRQATGQGLE WMG | WMHPN---SGQTGYAQK FQG | RVTLTRDTSISTAYMELS SLRSEDTAVYYCAS | SSGWY------------------YFDY | WGQGTL VTVSS |
| iPS:44 1900 | 21-225_4A2.001.012 | VH1|1-08/D6|6-19|RF1/JH4 | QVQLVQS-GTEVKKPGASVKVSCK ASG-YTFT | N-----YDIN | WVRQATGQGLE WMG | WMHPN---SGSTGYAQK FQG | RVTLTRDTSISTAYMELS SLRSEDTAVYYCAS | SSGWY------------------YFDY | WGQGTL VTVSS |
| iPS:44 1955 | 21-225_4A2.001.022 | VH1|1-08/D6|6-19|RF1/JH4 | QVQLVQS-GAEVKKPGASVKVSCK ASG-YTFT | N-----YDIN | WVRQATGQGLE WMG | WMHPN---SGNTGYAQK FQG | RVTLTRDTSISTAYMELS SLRSEDTAVYYCAS | SSGWY------------------YFDY | WGQGTL VTVSS |
| iPS:44 1962 | 21-225_4A2.001.023 | VH1|1-08/D6|6-19|RF1/JH4 | QVQLVQS-GAEVKKPGASVKVSCK ASG-YTFT | N-----YDIN | WVRQAPGQGLE WMG | WMHPN---SGNTGYAQK FQG | RVTLTRDTSISTAYMELS SLRSEDTAVYYCAS | SSGWY------------------YFDY | WGQGTL VTVSS |
| iPS:44 1971 | 21-225_4A2.001.024 | VH1|1-08/D6|6-19|RF1/JH4 | QVQLVQS-GAEVKKPGASVKVSCK ASG-YTFT | N-----YDIN | WVRQATCQGLE WMG | WMHPN---SGQTGYAQK FQG | RVTLTRDTSISTAYMELS SLRSEDTAVYYCAS | SSGWY------------------YFDY | WGQGTL VTVSS |
| iPS:44 1999 | 21-225_4A2.001.028 | VH1|1-08/D6|6-19|RF1/JH4 | QVQLVQS-GAEVKKPGASVKVSCK ASG-YTFT | N-----YDIN | WVRQAPGQGLE WMG | WMHPN---SGQTGYAQK FQG | RVTLTRDTSISTAYMELS SLRSEDTAVYYCAS | SSGWY------------------YFDY | WGQGTL VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:44 2006 | 21-225_4A2. 001.029 | VH1\|1-08\|D6\|6-19\|RF1/JH4 | QVQLVQS-GAEVKKPGASVKVSCK ASG-YTFT | N-----YDIN | WVRQAPGQGLE WMG | WMHPN---SGSTGYAQK FQG | RVTLTRDISISTAYMELS SLRSEDTAVYYCAS | SSGWY------------ ---------YFDY | WGQGTL VTVSS |
| iPS:44 2020 | 21-225_4A2. 001.031 | VH1\|1-08\|D6\|6-19\|RF1/JH4 | QVQLVQS-GGEVKKPGASVKVSCK ASG-YTFT | N-----YDIN | WVRQAEGQGLE WMG | WMHPN---SGNEGYAQK FQG | RVTLTRDISISTAYMELS SLRSEDTAVYYCAS | SSGWY------------ ---------YFDY | WGQGTL VTVSS |
| | VH1\|1-02/D1\|1-1\|RF1/JH4 | | QVQLVQS-GAEVKKPGASVKVSCK ASG-YTFT | G-----YYMH | WVRQAPGQGLE WMG | WIHPN---SGGTNYAQK FQG | RVTMTRDISISTAYMELS RLRSDDTAVYYCAR | GTTGT------------ ---------YFDY | WGQGTL VTVSS |
| iPS:39 3954 | 21-225_4H6 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDISISTAYMGLS SLRSDDTAVYYCAR | DGTS------------- ---------SFDY | WGQGTL VTVSS |
| iPS:44 2050 | 21-225_4H6. 004 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDISISTAYMELS SLRSDDTAVYYCAR | DGTS------------- ---------SFDY | WGQGTL VTVSS |
| iPS:44 2059 | 21-225_4H6. 005 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDISISTAYMELS SLRSDDTAVYYCAR | DGTS------------- ---------SFDY | WGQGTL VTVSS |
| iPS:44 2065 | 21-225_4H6. 006 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDTSISTAYMELS SLRSDDTAVYYCAR | DGTS------------- ---------SFDY | WGQGTL VTVSS |
| iPS:44 2071 | 21-225_4H6. 007 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDTSISTAYMELS SLRSDDTAVYYCAR | DATS------------- ---------SFDY | WGQGTL VTVSS |
| iPS:44 2078 | 21-225_4H6. 008 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDTSISTAYMELS SLRSDDTAVYYCAR | SGTS------------- ---------SFDY | WGQGTL VTVSS |
| iPS:44 2085 | 21-225_4H6. 009 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDTSISTAYMELS SLRSDDTAVYYCAR | SGTS------------- ---------SFDY | WGQGTL VTVSS |
| iPS:44 2089 | 21-225_4H6. 010 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDTSISTAYMELS SLRSDDTAVYYCAR | DATS------------- ---------SFDY | WGQGTL VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:44 2093 | 21-225_4H6.011 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDISISTAYMELS SLRSDDTAVYYCAR | DATS-------------SFDY | WGQGTL VTVSS |
| iPS:44 3016 | 21-225_4H6.014 | VH1\|1-02/D1\|1-1\|RF1/JH4 | QVQLVQS-GAEVKKPGASVKVSCK ASG-YTFT | D-----YYLH | WVRQAPGQGLE WMG | WIHPN---SGGTNYAQK FQG | RVTMTRDISISTAYMGLS SLRSDDTAVYYCAR | DATS-------------SFDY | WGQGTL VTVSS |
| | VH3\|3-33/D6\|6-6\|RF1/JH6 | Germline | QVQLVES-GGGVVQPGRSLRLSCA ASG-FTFS | S-----YGMH | WVRQAPGKGLE WVA | VIWYD---GSNKYYADS VKG | RFTISRDNSKNTLYLQMN SLRAEDTAVYYCAR | EYSSSSYY----------YYYGMDV | WGQGTT VTVSS |
| iPS:39 4041 | 21-225_5E5 | VH3\|3-33/D6\|6-6\|RF1/JH6 | QVQLVES-GGGVVQPGRSLRLSCA ASG-FTFS | N-----YVMH | WVRQAPGKGLE WVA | VIWYD---GSNKYYADS VKG | RFTISRDNSKNTLYLQMN SLRAEDTAVYYCTR | EVYSSGW----------YDYGMDV | WGQGTT VTVSS |
| iPS:44 2115 | 21-225_5E5.003 | VH3\|3-33/D6\|6-6\|RF1/JH6 | QVQLVES-GGGVVQPGRSLRLSCA ASG-FTFS | N-----YVMH | WVRQAPGKGLE WVA | VIWYD---ASNKYYADS VKG | RFTISRDNSKNTLYLQMN SLRAEDTAVYYCTR | EVYSSGW----------YDYGMDV | WGQGTT VTVSS |
| iPS:44 2122 | 21-225_5E5.004 | VH3\|3-33/D6\|6-6\|RF1/JH6 | QVQLVES-GGGVVQPGRSLRLSCA ASG-FTFS | N-----YVMH | WVRQAPGKGLE WVA | VIWYD---GSNKYYAES VKG | RFTISRDNSKNTLYLQMN SLRAEDTAVYYCTR | EVYSSGW----------YDYGMDV | WGQGTT VTVSS |
| iPS:44 2129 | 21-225_5E5.005 | VH3\|3-33/D6\|6-6\|RF1/JH6 | QVQLVES-GGGVVQPGRSLRLSCA ASG-FTFS | N-----YVMH | WVRQAPGKGLE WVA | VIWYD---GSNKYYAGS VKG | RFTISRDNSKNTLYLQMN SLRAEDTAVYYCTR | EVYSSGW----------YDYGMDV | WGQGTT VTVSS |
| iPS:44 2136 | 21-225_5E5.006 | VH3\|3-33/D6\|6-6\|RF1/JH6 | QVQLVES-GGGVVQPGRSLRLSCA ASG-FTFS | N-----YVMH | WVRQAPGKGLE WVA | VIWYD---GSNKYYADA VKG | RFTISRDNSKNTLYLQMN SLRAEDTAVYYCTR | EVYSSGW----------YDYGMDV | WGQGTT VTVSS |
| iPS:44 2171 | 21-225_5E5.011 | VH3\|3-33/D6\|6-6\|RF1/JH6 | QVQLVES-GGGVVQPGRSLRLSCA ASG-FTFS | N-----YVMH | WVRQAPGKGLE WVA | VIWYD---ASNKYYAES VKG | RFTISRDNSKNTLYLQMN SLRAEDTAVYYCTR | EVYSSGW----------YDYGMDV | WGQGTT VTVSS |
| iPS:44 2178 | 21-225_5E5.012 | VH3\|3-33/D6\|6-6\|RF1/JH6 | QVQLVES-GGGVVQPGRSLRLSCA ASG-FTFS | N-----YVMH | WVRQAPGKGLE WVA | VIWYD---ASNKYYADA VKG | RFTISRDNSKNTLYLQMN SLRAEDTAVYYCTR | EVYSSGW----------YDYGMDV | WGQGTT VTVSS |
| iPS:44 2199 | 21-225_5E5.015 | VH3\|3-33/D6\|6-6\|RF1/JH6 | QVQLVES-GGGVVQPGRSLRLSCA ASG-FTFS | N-----YVMH | WVRQAPGKGLE WVA | VIWYD---ASNKYYADS VKG | RFTISRDNSKNTLYLQMN SLRAEDTAVYYCTR | EVYSSGY----------YDYGMDV | WGQGTT VTVSS |

| | Germline | H_FR1 | H_CDR1 | H_FR2 | H_CDR2 | H_FR3 | H_CDR3 | H_FR4 |
|---|---|---|---|---|---|---|---|---|
| iPS:44 2206 | 21-225_5E5.016 | VH3\|3-33/D6\|6-6\|RF1/JH6 | QVQLVES-GGGVVQPGRSLRLSCAASG-FTFS | N-----YVMH | WVRQAPGKGLE WVA | VIWYD---ASNKYYADSVKG | RFTISRDNSKNTLYLQMNSLRAEDTAVYYCTR | EVYSSGF----------------YDYGMDV | WGQGTTVTVSS |
| iPS:44 2213 | 21-225_5E5.017 | VH3\|3-33/D6\|6-6\|RF1/JH6 | QVQLVES-GGGVVQPGRSLRLSCAASG-FTFS | N-----YVMH | WVRQAPGKGLE WVA | VIWYD---GSNKYYAESVKG | RFTISRDNSKNTLYLQMNSLRAEDTAVYYCTR | EVYSSGY----------------YDYGMDV | WGQGTTVTVSS |
| iPS:44 2220 | 21-225_5E5.018 | VH3\|3-33/D6\|6-6\|RF1/JH6 | QVQLVES-GGGVVQPGRSLRLSCAASG-FTFS | N-----YVMH | WVRQAPGKGLE WVA | VIWYD---GSNKYYADSVKG | RFTISRDNSKNTLYLQMNSLRAEDTAVYYCTR | EVYSSGY----------------YDYGMDV | WGQGTTVTVSS |
| iPS:44 2227 | 21-225_5E5.019 | VH3\|3-33/D6\|6-6\|RF1/JH6 | QVQLVES-GGGVVQPGRSLRLSCAASG-FTFS | N-----YVMH | WVRQAPGKGLE WVA | VIWYD---GSNKYYADSVKG | RFTISRDNSKNTLYLQMNSLRAEDTAVYYCTR | EVYSSGF----------------YDYGMDV | WGQGTTVTVSS |
| iPS:44 2255 | 21-225_5E5.023 | VH3\|3-33/D6\|6-6\|RF1/JH6 | QVQLVES-GGGVVQPGRSLRLSCAASG-FTFS | N-----YVMH | WVRQAPGKGLE WVA | VIWYD---ASNKYYAESVKG | RFTISRDNSKNTLYLQMNSLRAEDTAVYYCTR | EVYSSGY----------------YDYGMDV | WGQGTTVTVSS |
| iPS:44 2262 | 21-225_5E5.024 | VH3\|3-33/D6\|6-6\|RF1/JH6 | QVQLVES-GGGVVQPGRSLRLSCAASG-FTFS | N-----YVMH | WVRQAPGKGLE WVA | VIWYD---ASNKYYACAVKG | RFTISRDNSKNTLYLQMNSLRAEDTAVYYCTR | EVYSSGY----------------YDYGMDV | WGQGTTVTVSS |
| | Germline | H_FR1 | H_CDR1 | H_FR2 | H_CDR2 | H_FR3 | H_CDR3 | H_FR4 |
| | VH3\|3-07/D6\|6-6\|RF1/JH6 | EVQLVES-GGGLVQPGGSLRLSCAASG-FTFS | S-----YWMS | WVRQAPGKGLE WVA | NIKQD---GSEKYYVDSVKG | RFTISRDNAKNSLYLQMNSLRAEDTAVYYCAR | EYSSSSYY----------YYYGMDV | WGQGTTVTVSS |
| iPS:44 2269 | 21-225_5E5.025 | VH3\|3-07/D6\|6-6\|RF1/JH6 | EVQLVES-GGGVVQPGGSLRLSCAASG-FTFS | N-----YVMH | WVRQAPGKGLE WVA | VIWYD---GSNKYYAESVKG | RFTISRDNAKNTLYLQMNSLRAEDTAVYYCTR | EVYSSGW----------------YDYGMDV | WGQGTTVTVSS |
| | Germline | H_FR1 | H_CDR1 | H_FR2 | H_CDR2 | H_FR3 | H_CDR3 | H_FR4 |
| | VH3\|3-33/D4\|4-11\|RF2/JH6 | QVQLVES-GGGVVQPGRSLRLSCAASG-FTFS | S-----YGMH | WVRQAPGKGLE WVA | VIWYD---GSNKYYADSVKG | RFTISRDNSKNTLYLQMNSLRAEDTAVYYCAR | DYSNYYY----------------YYYGMDV | WGQGTTVTVSS |
| iPS:39 3930 | 21-225_7E11 | VH3\|3-33/D4\|4-11\|RF2/JH6 | QVQLVES-GGGVVQPGRSLRLSCAASG-FTFS | S-----FGMH | WVRQAPGKGLE WVA | IIWHD---GSNKYYADSVKG | RFTISRDNSNNTLYLQMSSLRAEDTAVYYCAR | DLSMG----------------------GMDV | WGQGTTVTVSS |
| iPS:42 4460 | 21-225_7E11.001 | VH3\|3-33/D4\|4-11\|RF2/JH6 | QVQLVES-GGGVVQPGRSLRLSCAASG-FTFS | S-----FGMH | WVRQAPGKGLE WVA | IIWHD---GSNKYYADSVKG | RFTISRDNSKNTLYLQMSSLRAEDTAVYYCAR | DLSMG--------------GMDV | WGQGTTVTVSS |

| iPS:44 2311 | 21-225_7E11 .001.001 | VH3\|3-33/D4\|4-11\|RF2\|JH6 | QVQLVES-GCGVVQPGRSLRLSCA ASG-FTFS | S-----FGMH | WVRQAPGKGLE WVA | IIWHD---GSNKYYADS VKG | RFTISRDNSKNTLYLQMS SLRAEDTAVYYCAR | DLSMG------------------GMDV | WGQGTT VTVSS |
|---|---|---|---|---|---|---|---|---|---|
| iPS:44 2317 | 21-225_7E11 .001.002 | VH3\|3-33/D4\|4-11\|RF2\|JH6 | QVQLVES-GGGVVQPGRSLRLSCA ASG-FTFS | S-----FGMH | WVRQAPGKGLE WVA | IIWHD---GSNKYYADS VKG | RFTISRDNSKNTLYLQMS SLRAEDTAVYYCAR | DLSMG------------------GMDV | WGQGTT VTVSS |
| iPS:44 2323 | 21-225_7E11 .001.003 | VH3\|3-33/D4\|4-11\|RF2\|JH6 | QVQLVES-GGGVVQPGRSLRLSCA ASG-FTFS | S-----FGMH | WVRQAPGKGLE WVA | IIWHS---GSNKYYADS VKG | RFTISRDNSKNTLYLQMS SLRAEDTAVYYCAR | DLSMG------------------GMDV | WGQGTT VTVSS |
| iPS:44 2330 | 21-225_7E11 .001.004 | VH3\|3-33/D4\|4-11\|RF2\|JH6 | QVQLVES-GGGVVQPGRSLRLSCA ASG-FTFS | S-----FGMH | WVRQAPGKGLE WVA | IIWHE---GSNKYYADS VKG | RFTISRDNSKNTLYLQMS SLRAEDTAVYYCAR | DLSMG------------------GMDV | WGQGTT VTVSS |
| iPS:44 2337 | 21-225_7E11 .001.005 | VH3\|3-33/D4\|4-11\|RF2\|JH6 | QVQLVES-GGGVVQPGRSLRLSCA ASG-FTFS | S-----FGMH | WVRQAPGKGLE WVA | IIWHD---ASNKYYADS VKG | RFTISRDNSKNTLYLQMS SLRAEDTAVYYCAR | DLSMG------------------GMDV | WGQGTT VTVSS |
| iPS:44 2344 | 21-225_7E11 .001.006 | VH3\|3-33/D4\|4-11\|RF2\|JH6 | QVQLVES-GGGVVQPGRSLRLSCA ASG-FTFS | S-----FGMH | WVRQAPGKGLE WVA | IIWHD---GSNKYYAES VKG | RFTISRDNSKNTLYLQMS SLRAEDTAVYYCAR | DLSMG------------------GMDV | WGQGTT VTVSS |
| iPS:44 2351 | 21-225_7E11 .001.007 | VH3\|3-33/D4\|4-11\|RF2\|JH6 | QVQLVES-GGGVVQPGRSLRLSCA ASG-FTFS | S-----FGMH | WVRQAPGKGLE WVA | IIWHD---GSNKYYADA VKG | RFTISRDNSKNTLYLQMS SLRAEDTAVYYCAR | DLSMG------------------GMDV | WGQGTT VTVSS |
| iPS:44 2358 | 21-225_7E11 .001.008 | VH3\|3-33/D4\|4-11\|RF2\|JH6 | QVQLVES-GGGVVQPGRSLRLSCA ASG-FTFS | S-----FGMH | WVRQAPGKGLE WVA | IIWHE---GSNKYYADA VKG | RFTISRDNSKNTLYLQMS SLRAEDTAVYYCAR | DLSMG------------------GMDV | WGQGTT VTVSS |
| iPS:44 2365 | 21-225_7E11 .001.009 | VH3\|3-33/D4\|4-11\|RF2\|JH6 | QVQLVES-GGGVVQPGRSLRLSCA ASG-FTFS | S-----FGMH | WVRQAPGKGLE WVA | IIWHE---GSNKYYAES VKG | RFTISRDNSKNTLYLQMS SLRAEDTAVYYCAR | DLSMG------------------GMDV | WGQGTT VTVSS |
| iPS:44 2372 | 21-225_7E11 .001.010 | VH3\|3-33/D4\|4-11\|RF2\|JH6 | QVQLVES-GGGVVQPGRSLRLSCA ASG-FTFS | S-----FGMH | WVRQAPGKGLE WVA | IIWHS---GSNKYYAES VKG | RFTISRDNSKNTLYLQMS SLRAEDTAVYYCAR | DLSMG------------------GMDV | WGQGTT VTVSS |
| iPS:44 2379 | 21-225_7E11 .001.011 | VH3\|3-33/D4\|4-11\|RF2\|JH6 | QVQLVES-GGGVVQPGRSLRLSCA ASG-FTFS | S-----FGMH | WVRQAPGKGLE WVA | IIWHS---GSNKYYADA VKG | RFTISRDNSKNTLYLQMS SLRAEDTAVYYCAR | DLSMG------------------GMDV | WGQGTT VTVSS |
| iPS:44 2366 | 21-225_7E11 .001.012 | VH3\|3-33/D4\|4-11\|RF2\|JH6 | QVQLVES-GGGVVQPGRSLRLSCA ASG-FTFS | S-----FGMH | WVRQAPGKGLE WVA | IIWHS---GSNKYYADS VKG | RFTISRDNSKNTLYLQMS SLRAEDTAVYYCAR | DLSMG------------------GMDV | WGQGTT VTVSS |

| | | Germline | H_FR1 | H_CDR1 | H_FR2 | H_CDR2 | H_FR3 | H_CDR3 | H_FR4 |
|---|---|---|---|---|---|---|---|---|---|
| iPS:44 2390 | 21-225_7E11.001.013 | VH3\|3-33/D4\|4-11\|RF2\|JH6 | QVQLVES-GGGVVQPGRSLRLSCAASG-FTFS | S-----FGMH | WVRQAPGKGLEWVA | IIWHD---GSNKYYAES VKG | RFTISRDNSKNTLYLQMSSLRAEDTAVYYCAR | DLSMG------------------GMDV | WGQGTTVTVSS |
| iPS:44 2394 | 21-225_7E11.001.014 | VH3\|3-33/D4\|4-11\|RF2\|JH6 | QVQLVES-GGGVVQPGRSLRLSCAASG-FTFS | S-----FGMH | WVRQAPGKGLEWVA | IIWHS---GSNKYYAES VKG | RFTISRDNSKNTLYLQMSSLRAEDTAVYYCAR | DLSMG------------------GMDV | WGQGTTVTVSS |
| iPS:44 2398 | 21-225_7E11.001.015 | VH3\|3-33/D4\|4-11\|RF2\|JH6 | QVQLVES-GGGVVQPGRSLRLSCAASG-FTFS | S-----FGMH | WVRQAPGKGLEWVA | IIWHS---GSNKYYADS VKG | RFTISRDNSKNTLYLQMSSLRAEDTAVYYCAR | DLSMG------------------GMDV | WGQGTTVTVSS |
| iPS:44 2402 | 21-225_7E11.001.016 | VH3\|3-33/D4\|4-11\|RF2\|JH6 | QVQLVES-GGGVVQPGRSLRLSCAASG-FTFS | S-----FGMH | WVRQAPGKGLEWVA | IIWHS---GSNKYYAES VKG | RFTISRDNSKNTLYLQMSSLRAEDTAVYYCAR | DLSMG------------------GMDV | WGQGTTVTVSS |
| iPS:44 2406 | 21-225_7E11.001.017 | VH3\|3-33/D4\|4-11\|RF2\|JH6 | QVQLVES-GGGVVQPGRSLRLSCAASG-FTFS | S-----FGMH | WVRQAPGKGLEWVA | IIWHE---GSNKYYADA VKG | RFTISRDNSKNTLYLQMSSLRAEDTAVYYCAR | DLSMG------------------GMDV | WGQGTTVTVSS |
| iPS:44 2410 | 21-225_7E11.001.018 | VH3\|3-33/D4\|4-11\|RF2\|JH6 | QVQLVES-GGGVVQPGRSLRLSCAASG-FTFS | S-----FGMH | WVRQAPGKGLEWVA | IIWHD---ASNKYYAES VKG | RFTISRDNSKNTLYLQMSSLRAEDTAVYYCAR | DLSMG------------------GMDV | WGQGTTVTVSS |
| iPS:44 3027 | 21-225_7E11.001.023 | VH3\|3-33/D4\|4-11\|RF2\|JH6 | QVQLVES-GGGVVQPGRSLRLSCAASG-FTFS | S-----FGMH | WVRQAPGKGLEWVA | IIWHD---ASNKYYADA VKG | RFTISRDNSKNTLYLQMSSLRAEDTAVYYCAR | DLSMG------------------GMDV | WGQGTTVTVSS |
| | | VH3\|3-07/D4\|4-11\|RF2\|JH6 | EVQLVES-GGGLVQPGGSLRLSCAASG-FTFS | S-----YWMS | WVRQAPGKGLEWVA | NIKQD---GSEKYYVDS VKG | RFTISRDNAKNSLYLQMNSLRAEDTAVYYCAR | DYSNYYY----------YYYGMDV | WGQGTTVTVSS |
| iPS:44 2417 | 21-225_7E11.001.019 | VH3\|3-07/D4\|4-11\|RF2\|JH6 | EVQLVES-GGGVVQPGGSLRLSCAASG-FTFS | S-----FGMH | WVRQAPGKGLEWVA | IIWHD---GSNKYYAES VKG | RFTISRDNAKNTLYLQMSSLRAEDTAVYYCAR | DLSMG------------------GMDV | WGQGTTVTVSS |
| iPS:44 2431 | 21-225_7E11.001.021 | VH3\|3-07/D4\|4-11\|RF2\|JH6 | EVQLVES-GGGVVQPGGSLRLSCAASG-FTFS | S-----FGMH | WVRQAPGKGLEWVA | IIWHS---GSNKYYAES VKG | RFTISRDNAKNTLYLQMSSLRAEDTAVYYCAR | DLSMG------------------GMDV | WGQGTTVTVSS |
| iPS:44 2438 | 21-225_7E11.001.022 | VH3\|3-07/D4\|4-11\|RF2\|JH6 | EVQLVES-GGGVVQPGGSLRLSCAASG-FTFS | S-----FGMH | WVRQAPGKGLEWVA | IIWHE---GSNKYYAES VKG | RFTISRDNAKNTLYLQMSSLRAEDTAVYYCAR | DLSMG------------------GMDV | WGQGTTVTVSS |

Figure 54 (Table 7)
Standard IgG Antibody Variable Region Consensus Protein Alignment
CDRs defined by Kabat
Alignment numbering defined by Amgen Reference (AHo)

| KAPPA_VARIABLE | | Germline | K_FR1 | K_CDR1 | K_FR2 | K_CDR2 | K_FR3 | K_CDR3 | K_FR4 |
|---|---|---|---|---|---|---|---|---|---|
| | VK4|B3/J K1 | | DIVMTQSPDSLAV SLGERATINC | KSS-- QSVLYSSNNKNYLA | WYQQKPGQPPK LLIY | W-------- ASTRES | GVPDRFSGSGSG-- TDFTLTISSLQAEDVAV YYC | QQYYS--------------- ----------TPWT | FGQGTKV EIK |
| iPS:39 2928 | 21-225_25A4 | VK4|B3/J K1 | ......F...... ........K. | .............H. N.... | .......... ..L. | .......... ..... | ................E. ......E......... | ................... ...........P. | ....... ... |
| iPS:42 4419 | 21-225_25A 4.001 | VK4|B3/J K1 | ......F...... ........K. | .............H. N.... | .......... ..L. | .......... ..... | ................E. ......E......... | ................... ...........P. | ....... ... |
| iPS:44 1468 | 21-225_25A 4.001.001 | VK4|B3/J K1 | ......F...... ........K. | .............H. N.... | .......... ..L. | .......... ..... | ................E. ......E......... | ................... ...........P. | ....... ... |
| iPS:44 1475 | 21-225_25A 4.001.002 | VK4|B3/J K1 | ......F...... ........K. | .............H. N.... | .......... ..L. | .......... ..... | ................E. ......E......... | ................... ...........P. | ....... ... |
| iPS:44 1482 | 21-225_25A 4.001.003 | VK4|B3/J K1 | ......F...... ........K. | .............H. N.... | .......... ..L. | .......... ..... | ................E. ......E......... | ................... ...........P. | ....... ... |
| iPS:44 1489 | 21-225_25A 4.001.004 | VK4|B3/J K1 | ......F...... ........K. | .............H. N.... | .......... ..L. | .......... ..... | ................E. ......E......... | ................... ...........P. | ....... ... |
| iPS:44 1496 | 21-225_25A 4.001.005 | VK4|B3/J K1 | .............. ........K. | .............H. N.... | .......... ..L. | .......... ..... | ................E. ................. | ................... ...........P. | ....... ... |
| iPS:44 1505 | 21-225_25A 4.001.006 | VK4|B3/J K1 | .............. ........K. | .............H. N.... | .......... ..L. | .......... ..... | ................E. ................. | ................... ...........P. | ....... ... |
| iPS:44 1512 | 21-225_25A 4.001.007 | VK4|B3/J K1 | .............. ........K. | .............H. N.... | .......... ..L. | .......... ..... | ................E. ................. | ................... ...........P. | ....... ... |
| iPS:44 1519 | 21-225_25A 4.001.008 | VK4|B3/J K1 | .............. ........K. | .............H. N.... | .......... ..L. | .......... ..... | ................E. ................. | ................... ...........P. | ....... ... |
| iPS:44 1554 | 21-225_25A 4.001.013 | VK4|B3/J K1 | ......F...... ........K. | .............H. N.... | .......... ..L. | .......... ..... | ................E. ................. | ................... ...........P. | ....... ... |
| iPS:44 1595 | 21-225_25A 4.001.019 | VK4|B3/J K1 | .............. .......... | .............H. N.... | .......... ..L. | .......... ..... | ................E. ......E......... | ................... ...........P. | ....... ... |

| | | Germline | | | | | | | |
|---|---|---|---|---|---|---|---|---|---|
| iPS:44 1604 | 21-225_25A 4.001.020 | VK4\|B3/J K1 | .............. .......... | ............H. N.... | ............ ..L. | ........ ..... | ...............E. ................ | ................ ...........P. | ....... ... |
| iPS:44 1613 | 21-225_25A 4.001.021 | VK4\|B3/J K1 | .............. .......... | ............H. N.... | ............ ..L. | ........ ..... | ...............E. ................ | ................ ...........P. | ....... ... |
| iPS:44 3006 | 21-225_25A 4.001.029 | VK4\|B3/J K1 | ......F...... ........K. | ............H. N.... | ............ ..L. | ........ ..... | ...............E. ................ | ................ ...........P. | ....... ... |
| iPS:44 1962 | 21-225_4A2. 001.023 | VK4\|B3/J K1 | .............. .......... | .......I.H.... N.... | .F........ ..L. | ........ ..... | ................ .........P....... | ................ ............V. | ....... ... |
| iPS:44 1999 | 21-225_4A2. 001.028 | VK4\|B3/J K1 | .............. .......... | .......I.H.... N.... | .F........ ..L. | ........ ..... | ................ .........P....... | ................ ............V. | ....... ... |
| iPS:44 2006 | 21-225_4A2. 001.029 | VK4\|B3/J K1 | .............. .......... | .......I.H.... N.... | .F........ ..L. | ........ ..... | ................ .........P....... | ................ ............V. | ....... ... |
| iPS:44 2020 | 21-225_4A2. 001.031 | VK4\|B3/J K1 | .............. .......... | .......I.H.... N.... | .F........ ..L. | ........ ..... | ................ .........P....... | ....N............. ............V. | ..P.... ... |

| | | Germline | K_FR1 | K_CDR1 | K_FR2 | K_CDR2 | K_FR3 | K_CDR3 | K_FR4 |
|---|---|---|---|---|---|---|---|---|---|
| | VK1\|L5/J K3 | | DIQMTQSPSSVSA SVGDRVTITC | RAS QGIS -SWLA | WYQQKPGKAPK LLIY | A ASSLQS | GVPSRFSGSGSG TDFTLTISSLQPEDFAT YYC | QQANS ----------FPFT | FGPGTKV DIK |
| iPS:39 3954 | 21-225_4H6 | VK1\|L5/J K3 | ............ .......... | ............ .R... | ........... .... | G........ ..... | ................ ..............I... | ................ ................ | ....... ... |
| iPS:44 2050 | 21-225_4H6. 004 | VK1\|L5/J K3 | ............ .......... | ............ .R... | ........... .... | G........ ..... | ................ ..............I... | ................ ................ | ....... ... |
| iPS:44 2059 | 21-225_4H6. 005 | VK1\|L5/J K3 | ............ .......... | ............ .R... | ........... .... | G........ ..... | ................ ..............I... | ................ ................ | ..Q.... ... |
| iPS:44 2065 | 21-225_4H6. 006 | VK1\|L5/J K3 | ............ .......... | ............ .R... | ........... .... | G........ ..... | ................ ................ | ................ ................ | ..Q.... ... |
| iPS:44 2071 | 21-225_4H6. 007 | VK1\|L5/J K3 | ............ .......... | ............ .R... | ........... .... | G........ ..... | ................ ..............I... | ................ ................ | ....... ... |
| iPS:44 2078 | 21-225_4H6. 008 | VK1\|L5/J K3 | ............ .......... | ............ .R... | ........... .... | G........ ..... | ................ ..............I... | ................ ................ | ....... ... |
| iPS:44 2085 | 21-225_4H6. 009 | VK1\|L5/J K3 | ............ .......... | ............ .R... | ........... .... | G........ ..... | ................ ..............I... | ................ ................ | ..Q.... ... |

| | | Germline | K_FR1 | K_CDR1 | K_FR2 | K_CDR2 | K_FR3 | K_CDR3 | K_FR4 |
|---|---|---|---|---|---|---|---|---|---|
| iPS:44 2089 | 21-225_4H6.010 | VK1\|L5/J K3 | .............<br>.......... | ...............<br>.R... | ..........<br>.... | G........<br>..... | ................<br>.......I... | ..................<br>............ | ..Q....<br>... |
| iPS:44 2093 | 21-225_4H6.011 | VK1\|L5/J K3 | .............<br>.......... | ...............<br>.R... | ..........<br>.... | G........<br>..... | ................<br>............... | ..................<br>............ | ..Q....<br>... |
| iPS:44 3016 | 21-225_4H6.014 | VK1\|L5/J K3 | .............<br>.......... | ...............<br>.R... | ..........<br>.... | G........<br>..... | ................<br>............... | ..................<br>............... | ... |
| | **VK4\|B3/J K3** | Germline | DIVMTQSPDSLAV SLGERATINC | KSS-- QSVLYSSNNKNYLA | WYQQKPGQPPK LLIY | W-------- ASTRES | GVPDRFSGSGSG-- TDFTLTISSLQAEDVAV YYC | QQYYS------------- ----------TPFT | FGPGTKV DIK |
| iPS:41 2232 | 21-225_4A2 | VK4\|B3/J K3 | ............<br>.......... | .......I.H....<br>N.... | .F........<br>..L. | ..........<br>..... | ..................<br>.......P........ | ....N............<br>............V. | .......<br>G.. |
| iPS:42 2894 | 21-225_4A2.001 | VK4\|B3/J K3 | ............<br>.......... | .......I.H....<br>N.... | .F........<br>..L. | ..........<br>..... | ..................<br>.......P........ | ....N............<br>............V. | .......<br>G.. |
| iPS:44 1841 | 21-225_4A2.001.001 | VK4\|B3/J K3 | ............<br>.......... | .......I.H....<br>N.... | .F........<br>..L. | ..........<br>..... | ..................<br>.......P........ | ....N............<br>............V. | .......<br>G.. |
| iPS:44 1847 | 21-225_4A2.001.002 | VK4\|B3/J K3 | ............<br>.......... | .......I.H....<br>N.... | .F........<br>..L. | ..........<br>..... | ..................<br>.......P........ | ....N............<br>..........A.V. | .......<br>G.. |
| iPS:44 1853 | 21-225_4A2.001.003 | VK4\|B3/J K3 | ............<br>.......... | .......I.H....<br>N.... | .F........<br>..L. | ..........<br>..... | ..................<br>.......P........ | ....Q............<br>............V. | .......<br>G.. |
| iPS:44 1859 | 21-225_4A2.001.004 | VK4\|B3/J K3 | ............<br>.......... | .......I.H....<br>N.... | .F........<br>..L. | ..........<br>..... | ..................<br>.......P........ | ....N............<br>............V. | .......<br>G.. |
| iPS:44 1866 | 21-225_4A2.001.005 | VK4\|B3/J K3 | ............<br>.......... | .......I.H....<br>N.... | .F........<br>..L. | ..........<br>..... | ..................<br>.......P........ | ....N............<br>............V. | .......<br>G.. |
| iPS:44 1873 | 21-225_4A2.001.006 | VK4\|B3/J K3 | ............<br>.......... | .......I.H....<br>N.... | .F........<br>..L. | ..........<br>..... | ..................<br>.......P........ | ....N............<br>............V. | .......<br>G.. |
| iPS:44 1880 | 21-225_4A2.001.007 | VK4\|B3/J K3 | ............<br>.......... | .......I.H....<br>N.... | .F........<br>..L. | ..........<br>..... | ..................<br>.......P........ | ..................<br>............V. | .......<br>G.. |
| iPS:44 1884 | 21-225_4A2.001.008 | VK4\|B3/J K3 | ............<br>.......... | .......I.H....<br>N.... | .F........<br>..L. | ..........<br>..... | ..................<br>.......P........ | ..................<br>............V. | .......<br>G.. |
| iPS:44 1888 | 21-225_4A2.001.009 | VK4\|B3/J K3 | ............<br>.......... | .......I.H....<br>N.... | .F........<br>..L. | ..........<br>..... | ..................<br>.......P........ | ....N............<br>..........A.V. | .......<br>G.. |

| | | | K_FR1 | K_CDR1 | K_FR2 | K_CDR2 | K_FR3 | K_CDR3 | K_FR4 |
|---|---|---|---|---|---|---|---|---|---|
| iPS:44 1892 | 21-225_4A2.001.010 | VK4|B3/JK3 | ............ / .......... | .......I.H.... / N.... | .F........ / ..L. | .......... / ..... | .......... / ......P........ | ....Q............ / ............V. | ....... / G.. |
| iPS:44 1896 | 21-225_4A2.001.011 | VK4|B3/JK3 | ............ / .......... | .......I.H.... / N.... | .F........ / ..L. | .......... / ..... | .......... / ......P........ | ....Q............ / ............V. | ....... / G.. |
| iPS:44 1900 | 21-225_4A2.001.012 | VK4|B3/JK3 | ............ / .......... | .......I.H.... / N.... | .F........ / ..L. | .......... / ..... | .......... / ......P........ | ....Q............ / ............V. | ....... / G.. |
| iPS:44 1955 | 21-225_4A2.001.022 | VK4|B3/JK3 | ............ / .......... | .......I.H.... / N.... | .F........ / ..L. | .......... / ..... | .......... / ......P........ | ................ / ............V. | ....... / G.. |
| iPS:44 1971 | 21-225_4A2.001.024 | VK4|B3/JK3 | ............ / .......... | .......I.H.... / N.... | .F........ / ..L. | .......... / ..... | .......... / ......P........ | ................ / ............V. | ....... / G.. |

| | | Germline | K_FR1 | K_CDR1 | K_FR2 | K_CDR2 | K_FR3 | K_CDR3 | K_FR4 |
|---|---|---|---|---|---|---|---|---|---|
| | VK1|A30/ JK1 | | DIQMTQSPSSLSA SVGDRVTITC | RAS--QGIR----- -NDLG | WYQQKPGKAPK RLIY | A--------- ASSLQS | GVPSRFSGSGSG-- TEFTLTISSLQPEDFAT YYC | LQHNS---------- ----------YPWT | FGQGTKV EIK |
| iPS:39 4041 | 21-225_5E5 | VK1|A30/ JK1 | ............ / .......... | ..... / ..... | .... / .... | ..... / ..... | ............ / ............ | ...Y............ / ............R. | ....... / .V. |
| iPS:44 2115 | 21-225_5E5.003 | VK1|A30/ JK1 | ............ / .......... | ..... / ..... | .... / .... | ..... / ..... | ............ / ............ | ...Y............ / ............R. | ....... / .V. |
| iPS:44 2122 | 21-225_5E5.004 | VK1|A30/ JK1 | ............ / .......... | ..... / ..... | .... / .... | ..... / ..... | ............ / ............ | ...Y............ / ............R. | ....... / .V. |
| iPS:44 2129 | 21-225_5E5.005 | VK1|A30/ JK1 | ............ / .......... | ..... / ..... | .... / .... | ..... / ..... | ............ / ............ | ...Y............ / ............R. | ....... / .V. |
| iPS:44 2136 | 21-225_5E5.006 | VK1|A30/ JK1 | ............ / .......... | ..... / ..... | .... / .... | ..... / ..... | ............ / ............ | ...Y............ / ............R. | ....... / .V. |
| iPS:44 2171 | 21-225_5E5.011 | VK1|A30/ JK1 | ............ / .......... | ..... / ..... | .... / .... | ..... / ..... | ............ / ............ | ...Y............ / ............R. | ....... / .V. |
| iPS:44 2178 | 21-225_5E5.012 | VK1|A30/ JK1 | ............ / .......... | ..... / ..... | .... / .... | ..... / ..... | ............ / ............ | ...Y............ / ............R. | ....... / .V. |
| iPS:44 2199 | 21-225_5E5.015 | VK1|A30/ JK1 | ............ / .......... | ..... / ..... | .... / .... | ..... / ..... | ............ / ............ | ...Y............ / ............R. | ....... / .V. |
| iPS:44 2206 | 21-225_5E5.016 | VK1|A30/ JK1 | ............ / .......... | ..... / ..... | .... / .... | ..... / ..... | ............ / ............ | ...Y............ / ............R. | ....... / .V. |

| | | Germline | K_FR1 | K_CDR1 | K_FR2 | K_CDR2 | K_FR3 | K_CDR3 | K_FR4 |
|---|---|---|---|---|---|---|---|---|---|
| iPS:44 2213 | 21-225_5E5.017 | VK1\|A30/JK1 | ............ .......... | .......... ..... | ........ .... | ......... ..... | ........................ .................... | ...Y............. ..........R. | ....... .V. |
| iPS:44 2220 | 21-225_5E5.018 | VK1\|A30/JK1 | ............ .......... | .......... ..... | ........ .... | ......... ..... | ........................ .................... | ...Y............. ..........R. | ....... .V. |
| iPS:44 2227 | 21-225_5E5.019 | VK1\|A30/JK1 | ............ .......... | .......... ..... | ........ .... | ......... ..... | ........................ .................... | ...Y............. ..........R. | ....... .V. |
| iPS:44 2255 | 21-225_5E5.023 | VK1\|A30/JK1 | ............ .......... | .......... ..... | ........ .... | ......... ..... | ........................ .................... | ...Y............. ..........R. | ....... .V. |
| iPS:44 2262 | 21-225_5E5.024 | VK1\|A30/JK1 | ............ .......... | .......... ..... | ........ .... | ......... ..... | ........................ .................... | ...Y............. ..........R. | ....... .V. |
| iPS:44 2269 | 21-225_5E5.025 | VK1\|A30/JK1 | ............ .......... | .......... ..... | ........ .... | ......... ..... | ........................ .................... | ...Y............. ..........R. | ....... .V. |
| | VK1\|O12/JK4 | | DIQMTQSPSSLSA SVGDRVTITC | RAS--QSIS----- -SYLN | WYQQKPGKAPK LLIY | A-------- ASSLQS | GVPSRFSGSGSG-- TDFTLTISSLQPEDFAT YYC | QQSYS-------------- ----------TPLT | FGGGTKV EIK |
| iPS:39 3930 | 21-225_7E1 1 | VK1\|O12/JK4 | ............ ........A. | ......N.I..... ..... | .......... F... | T......... ..... | ...................I... | ..T............. ............... | ....... ... |
| iPS:42 4460 | 21-225_7E1 1.001 | VK1\|O12/JK4 | ............ ........A. | ......N.I..... ..... | .......... F... | T......... ..... | ...................I... | ..T............. ............... | ....... ... |
| iPS:44 2311 | 21-225_7E1 1.001.001 | VK1\|O12/JK4 | ............ .......... | ......N.I..... ..... | .......... F... | T......... ..... | ...................I... | ..T............. ............... | ....... ... |
| iPS:44 2317 | 21-225_7E1 1.001.002 | VK1\|O12/JK4 | ............ .......... | ......N.I..... ..... | .......... .... | T......... ..... | ....................... | ..T............. ............... | ....... ... |
| iPS:44 2323 | 21-225_7E1 1.001.003 | VK1\|O12/JK4 | ............ ........A. | ......N.I..... ..... | .......... F... | T......... ..... | ...................I... | ..T............. ............... | ....... ... |
| iPS:44 2330 | 21-225_7E1 1.001.004 | VK1\|O12/JK4 | ............ ........A. | ......N.I..... ..... | .......... F... | T......... ..... | ...................I... | ..T............. ............... | ....... ... |
| iPS:44 2337 | 21-225_7E1 1.001.005 | VK1\|O12/JK4 | ............ ........A. | ......N.I..... ..... | .......... F... | T......... ..... | ...................I... | ..T............. ............... | ....... ... |
| iPS:44 2344 | 21-225_7E1 1.001.006 | VK1\|O12/JK4 | ............ ........A. | ......N.I..... ..... | .......... F... | T......... ..... | ...................I... | ..T............. ............... | ....... ... |

| | | | | | | | | | |
|---|---|---|---|---|---|---|---|---|---|
| iPS:44 2351 | 21- 225_7E1 1.001.007 | VK1\|O12/ JK4 | .............. ........A. | ......N.I..... ..... | ........... F... | T......... ..... | ...............I... | ..T............... .............. | ...... ... |
| iPS:44 2358 | 21- 225_7E1 1.001.008 | VK1\|O12/ JK4 | .............. ........A. | ......N.I..... ..... | ........... F... | T......... ..... | ...............I... | ..T............... .............. | ...... ... |
| iPS:44 2365 | 21- 225_7E1 1.001.009 | VK1\|O12/ JK4 | .............. ........A. | ......N.I..... ..... | ........... F... | T......... ..... | ...............I... | ..T............... .............. | ...... ... |
| iPS:44 2372 | 21- 225_7E1 1.001.010 | VK1\|O12/ JK4 | .............. ........A. | ......N.I..... ..... | ........... F... | T......... ..... | ...............I... | ..T............... .............. | ...... ... |
| iPS:44 2379 | 21- 225_7E1 1.001.011 | VK1\|O12/ JK4 | .............. ........A. | ......N.I..... ..... | ........... F... | T......... ..... | ...............I... | ..T............... .............. | ...... ... |
| iPS:44 2386 | 21- 225_7E1 1.001.012 | VK1\|O12/ JK4 | .............. .......... | ......N.I..... ..... | ........... F... | T......... ..... | ...............I... | ..T............... .............. | ...... ... |
| iPS:44 2390 | 21- 225_7E1 1.001.013 | VK1\|O12/ JK4 | .............. .......... | ......N.I..... ..... | ........... F... | T......... ..... | ...............I... | ..T............... .............. | ...... ... |
| iPS:44 2394 | 21- 225_7E1 1.001.014 | VK1\|O12/ JK4 | .............. .......... | ......N.I..... ..... | ........... F... | T......... ..... | ...............I... | ..T............... .............. | ...... ... |
| iPS:44 2398 | 21- 225_7E1 1.001.015 | VK1\|O12/ JK4 | .............. .......... | ......N.I..... ..... | ........... .... | T......... ..... | ................... | ..T............... .............. | ...... ... |
| iPS:44 2402 | 21- 225_7E1 1.001.016 | VK1\|O12/ JK4 | .............. .......... | ......N.I..... ..... | ........... .... | T......... ..... | ................... | ..T............... .............. | ...... ... |
| iPS:44 2406 | 21- 225_7E1 1.001.017 | VK1\|O12/ JK4 | .............. .......... | ......N.I..... ..... | ........... .... | T......... ..... | ................... | ..T............... .............. | ...... ... |
| iPS:44 2410 | 21- 225_7E1 1.001.018 | VK1\|O12/ JK4 | .............. .......... | ......N.I..... ..... | ........... F... | T......... ..... | ...............I... | ..T............... .............. | ...... ... |
| iPS:44 2417 | 21- 225_7E1 1.001.019 | VK1\|O12/ JK4 | .............. ........A. | ......N.I..... ..... | ........... F... | T......... ..... | ...............I... | ..T............... .............. | ...... ... |
| iPS:44 2431 | 21- 225_7E1 1.001.021 | VK1\|O12/ JK4 | .............. ........A. | ......N.I..... ..... | ........... F... | T......... ..... | ...............I... | ..T............... .............. | ...... ... |
| iPS:44 2438 | 21- 225_7E1 1.001.022 | VK1\|O12/ JK4 | .............. .......... | ......N.I..... ..... | ........... F... | T......... ..... | ............I... | ..T............... .............. | ...... ... |

| iPS:44 3027 | 21-225_7E1 1.001.023 | VK1\|O12/ JK4 | ............. ........A. | ......N.I..... ..... | ........... F... | T......... ..... | .................. .............I... | ..T.............. .............. | ....... ... |

**HEAVY_VARIABLE**

| | | Germline | H_FR1 | H_CDR1 | H_FR2 | H_CDR2 | H_FR3 | H_CDR3 | H_FR4 |
|---|---|---|---|---|---|---|---|---|---|
| | VH1\|1-08/D6\|6-19\|RF1/JH4 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GYSSGW--------------------YYFDY | WGQGTLVTVSS |
| iPS:39 2928 | 21-225_25A4 | VH1\|1-08/D6\|6-19\|RF1/JH4 | ..L.......... R............ ...... | N......... | ............ ... | ..Y....... .......... | ..................S | SSGWY- ................ .-.... | ....... .... |
| iPS:42 4419 | 21-225_25A4.001 | VH1\|1-08/D6\|6-19\|RF1/JH4 | ..L.......... R............ ...... | N......... | ............ ... | ..Y....... .......... | ......D..........S | SSGWY- ................ .-.... | ....... .... |
| iPS:44 1468 | 21-225_25A4.001.001 | VH1\|1-08/D6\|6-19\|RF1/JH4 | ..L.......... R............ ...... | N......... | ............ ... | ..Y....... S......... | ......D..........S | SSGWY- ................ .-.... | ....... .... |
| iPS:44 1475 | 21-225_25A4.001.002 | VH1\|1-08/D6\|6-19\|RF1/JH4 | ..L.......... R............ ...... | N......... | ............ ... | ..Y....... .A........ | ......D..........S | SSGWY- ................ .-.... | ....... .... |
| iPS:44 1482 | 21-225_25A4.001.003 | VH1\|1-08/D6\|6-19\|RF1/JH4 | ..L.......... R............ ...... | N......... | ............ ... | ..Y....... .V........ | ......D..........S | SSGWY- ................ .-.... | ....... .... |
| iPS:44 1489 | 21-225_25A4.001.004 | VH1\|1-08/D6\|6-19\|RF1/JH4 | ..L.......... R............ ...... | N......... | ............ ... | ..Y....... Q......... | ......D..........S | SSGWY- ................ .-.... | ....... .... |
| iPS:44 1496 | 21-225_25A4.001.005 | VH1\|1-08/D6\|6-19\|RF1/JH4 | ............. R............ ...... | N......... | ............ ... | ..Y....... S......... | ......D..........S | SSGWY- ................ .-.... | ....... .... |
| iPS:44 1505 | 21-225_25A4.001.006 | VH1\|1-08/D6\|6-19\|RF1/JH4 | ............. R............ ...... | N......... | ............ ... | ..Y....... .A........ | ......D..........S | SSGWY- ................ .-.... | ....... .... |
| iPS:44 1512 | 21-225_25A4.001.007 | VH1\|1-08/D6\|6-19\|RF1/JH4 | ............. R............ ...... | N......... | ............ ... | ..Y....... .V........ | ......D..........S | SSGWY- ................ .-.... | ....... .... |

| | | | | | | | | | |
|---|---|---|---|---|---|---|---|---|---|
| iPS:44 1519 | 21-225_25A 4.001.008 | VH1\|1-08/D6\|6-19\|RF1/J H4 | . . . . . . . . . . . . R . . . . . . . . . . . . . . . . . . | N . . . . . . . . | . . . . . . . . . . . . . . | . . Y . . . . . . . Q . . . . . . . . | . . . . D . . . . . . . . . . . . . . . . . . S | SSGWY- . . . . . . . . . . . . . . . . . . . . . ⁻ . . . . | . . . . . . . . . . |
| iPS:44 1554 | 21-225_25A 4.001.013 | VH1\|1-08/D6\|6-19\|RF1/J H4 | . . L . . . . . . . . . R . . . . . . . . . . . . . . . . . . | N . . . . . . . . | . . . . . . . . . . . . . . | . . Y . . . . . . . S . . . . . . . . | . . . . . . D . . . . . . . . . . . . . . . . S | SSGWY- . . . . . . . . . . . . . . . . . . . . . ⁻ . . . . | . . . . . . . . . . |
| iPS:44 1595 | 21-225_25A 4.001.019 | VH1\|1-08/D6\|6-19\|RF1/J H4 | . . . . . . . . . . . . R . . . . . . . . . . . . . . . . . . | N . . . . . . . . | . . . . . P . . . . . . . | . . Y . . . . . . . S . . . . . . . . | . . . . . . D . . . . . . . . . . . . . . . . S | SSGWY- . . . . . . . . . . . . . . . . . . . . . ⁻ . . . . | . . . . . . . . . . |
| iPS:44 1604 | 21-225_25A 4.001.020 | VH1\|1-08/D6\|6-19\|RF1/J H4 | . . L . . . . . . . . . R . . . . . . . . . . . . . . . . . . | N . . . . . . . . | . . . . . P . . . . . . . | . . Y . . . . . . . Q . . . . . . . . | . . . . . . D . . . . . . . . . . . . . . . . S | SSGWY- . . . . . . . . . . . . . . . . . . . . . ⁻ . . . . | . . . . . . . . . . |
| iPS:44 1613 | 21-225_25A 4.001.021 | VH1\|1-08/D6\|6-19\|RF1/J H4 | . . . . . . . . . . . . R . . . . . . . . . . . . . . . . . . | N . . . . . . . . | . . . . . . . . . . . . . . | . . Y . . . . . . . . A . . . . . . . | . . . . . . D . . . . . . . . . . . . . . . . S | SSGWY- . . . . . . . . . . . . . . . . . . . . . ⁻ . . . . | . . . . . . . . . . |
| iPS:44 3006 | 21-225_25A 4.001.029 | VH1\|1-08/D6\|6-19\|RF1/J H4 | . . L . . . . . . . . . R . . . . . . . . . . . . . . . . . . | N . . . . . . . . | . . . . . . . . . . . . . . | . . Y . . . . . . . Q . . . . . . . . | . . . . . . D . . . . . . . . . . . . . . . . S | SSGWY- . . . . . . . . . . . . . . . . . . . . . ⁻ . . . . | . . . . . . . . . . |
| iPS:41 2232 | 21-225_4A2 | VH1\|1-08/D6\|6-19\|RF1/J H4 | . . . . . . . . . T . . . . . . . . . . . . . . . . . . . . . . | N . . . . . . . . | . . . . . . . . . . . . . . | . . H . . . . . . . . . . . . . . . . . | . . . L . . . . . . . . . . . . . . . . . . . . S | SSGWY- . . . . . . . . . . . . . . . . . . . . . ⁻ . . . . | . . . . . . . . . . |
| iPS:42 2894 | 21-225_4A2. 001 | VH1\|1-08/D6\|6-19\|RF1/J H4 | . . . . . . . . . T . . . . . . . . . . . . . . . . . . . . . . | N . . . . . . . . | . . . . . . . . . . . . . . | . . H . . . . . . . . . . . . . . . . . | . . . L . . D . . . . . . . . . . . . . . . . . S | SSGWY- . . . . . . . . . . . . . . . . . . . . . ⁻ . . . . | . . . . . . . . . . |
| iPS:44 1841 | 21-225_4A2. 001.001 | VH1\|1-08/D6\|6-19\|RF1/J H4 | . . . . . . . . . T . . . . . . . . . . . . . . . . . . . . . . | N . . . . . . . . | . . . . . . . . . . . . . . | . . H . . . . . . . . . . . . . . . . . | . . . L . . D . . . . . . . . . . . . . . . . . S | SSGWY- . . . . . . . . . . . . . . . . . . . . . ⁻ . . . . | . . . . . . . . . |
| iPS:44 1847 | 21-225_4A2. 001.002 | VH1\|1-08/D6\|6-19\|RF1/J H4 | . . . . . . . . . T . . . . . . . . . . . . . . . . . . . . . . | N . . . . . . . . | . . . . . . . . . . . . . . | . . H . . . . . . . . . . . . . . . . . | . . . L . . D . . . . . . . . . . . . . . . . . S | SSGWY- . . . . . . . . . . . . . . . . . . . . . ⁻ . . . . | . . . . . . . . . . |
| iPS:44 1853 | 21-225_4A2. 001.003 | VH1\|1-08/D6\|6-19\|RF1/J H4 | . . . . . . . . . T . . . . . . . . . . . . . . . . . . . . . . | N . . . . . . . . | . . . . . . . . . . . . . . | . . H . . . . . . . . . . . . . . . . . | . . . L . . D . . . . . . . . . . . . . . . . . S | SSGWY- . . . . . . . . . . . . . . . . . . . . . ⁻ . . . . | . . . . . . . . . . |
| iPS:44 1859 | 21-225_4A2. 001.004 | VH1\|1-08/D6\|6-19\|RF1/J H4 | . . . . . . . . . T . . . . . . . . . . . . . . . . . . . . . . | N . . . . . . . . | . . . . . . . . . . . . . . | . . H . . . . . . . . A . . . . . . . . | . . . L . . D . . . . . . . . . . . . . . . . . S | SSGWY- . . . . . . . . . . . . . . . . . . . . . ⁻ . . . . | . . . . . . . . . . |

| | | | | | | | | | |
|---|---|---|---|---|---|---|---|---|---|
| iPS:44 1866 | 21-225_4A2. 001.005 | VH1\|1-08\|D6\|6-19\|RF1/J H4 | .........T.... ............. ...... | N......... | ........... ... | ..H...... S......... | ...L..D......... ............S | SSGWY- ................ .-.... | ....... .... |
| iPS:44 1873 | 21-225_4A2. 001.006 | VH1\|1-08\|D6\|6-19\|RF1/J H4 | .........T.... ............. ...... | N......... | ........... ... | ..H...... Q......... | ...L..D......... ............S | SSGWY- ................ .-.... | ....... .... |
| iPS:44 1880 | 21-225_4A2. 001.007 | VH1\|1-08\|D6\|6-19\|RF1/J H4 | .........T.... ............. ...... | N......... | ........... ... | ..H...... Q......... | ...L..D......... ............S | SSGWY- ................ .-.... | ....... .... |
| iPS:44 1884 | 21-225_4A2. 001.008 | VH1\|1-08\|D6\|6-19\|RF1/J H4 | .........T.... ............. ...... | N......... | ........... ... | ..H...... .A........ | ...L..D......... ............S | SSGWY- ................ .-.... | ....... .... |
| iPS:44 1888 | 21-225_4A2. 001.009 | VH1\|1-08\|D6\|6-19\|RF1/J H4 | .........T.... ............. ...... | N......... | ........... ... | ..H...... Q......... | ...L..D......... ............S | SSGWY- ................ .-.... | ....... .... |
| iPS:44 1892 | 21-225_4A2. 001.010 | VH1\|1-08\|D6\|6-19\|RF1/J H4 | .........T.... ............. ...... | N......... | ........... ... | ..H...... .A........ | ...L..D......... ............S | SSGWY- ................ .-.... | ....... .... |
| iPS:44 1896 | 21-225_4A2. 001.011 | VH1\|1-08\|D6\|6-19\|RF1/J H4 | .........T.... ............. ...... | N......... | ........... ... | ..H...... Q......... | ...L..D......... ............S | SSGWY- ................ .-.... | ....... .... |
| iPS:44 1900 | 21-225_4A2. 001.012 | VH1\|1-08\|D6\|6-19\|RF1/J H4 | .........T.... ............. ...... | N......... | ........... ... | ..H...... S......... | ...L..D......... ............S | SSGWY- ................ .-.... | ....... .... |
| iPS:44 1955 | 21-225_4A2. 001.022 | VH1\|1-08\|D6\|6-19\|RF1/J H4 | ............. ............. ...... | N......... | ........... ... | ..H...... ......... | ...L..D......... ............S | SSGWY- ................ .-.... | ....... .... |
| iPS:44 1962 | 21-225_4A2. 001.023 | VH1\|1-08\|D6\|6-19\|RF1/J H4 | ............. ............. ...... | N......... | ......P..... ... | ..H...... ......... | ...L..D......... ............S | SSGWY- ................ .-.... | ....... .... |
| iPS:44 1971 | 21-225_4A2. 001.024 | VH1\|1-08\|D6\|6-19\|RF1/J H4 | ............. ............. ...... | N......... | ........... ... | ..H...... Q......... | ...L..D......... ............S | SSGWY- ................ .-.... | ....... .... |
| iPS:44 1999 | 21-225_4A2. 001.028 | VH1\|1-08\|D6\|6-19\|RF1/J H4 | ............. ............. ...... | N......... | ......P..... ... | ..H...... Q......... | ...L..D......... ............S | SSGWY- ................ .-.... | ..... .... |

| iPS:44 2006 | 21-225_4A2.001.029 | VH1\|1-08/D6\|6-19\|RF1/JH4 | ............. ............. ...... | N......... | ......P..... ... | ..H....... S......... | ...L..D.......... .................S | SSGWY- .................... .-.... | ....... .... |
| iPS:44 2020 | 21-225_4A2.001.031 | VH1\|1-08/D6\|6-19\|RF1/JH4 | .........G.... ............. ...... | N......... | ......E..... ... | ..H....... .E........ | ...L..D.......... .................S | SSGWY- .................... .-.... | ....... .... |
| | Germline | | H_FR1 | H_CDR1 | H_FR2 | H_CDR2 | H_FR3 | H_CDR3 | H_FR4 |
| | VH1\|1-02/D1\|1-1\|RF1/JH4 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GTTGT---------------------YFDY | WGQGTLVTVSS |
| iPS:39 3954 | 21-225_4H6 | VH1\|1-02/D1\|1-1\|RF1/JH4 | ............. ............. ...... | D.......L. | ............ ... | ..H....... .......... | ................G. .S............. | DG.S- .................... ...S... | ....... .... |
| iPS:44 2050 | 21-225_4H6.004 | VH1\|1-02/D1\|1-1\|RF1/JH4 | ............. ............. ...... | D.......L. | ............ ... | ..H....... .......... | .................. .S............. | DG.S- .................... ...S... | ....... .... |
| iPS:44 2059 | 21-225_4H6.005 | VH1\|1-02/D1\|1-1\|RF1/JH4 | ............. ............. ...... | D.......L. | ............ ... | ..H....... .......... | .................. .S............. | DG.S- .................... ...S... | ....... .... |
| iPS:44 2065 | 21-225_4H6.006 | VH1\|1-02/D1\|1-1\|RF1/JH4 | ............. ............. ...... | D.......L. | ............ ... | ..H....... .......... | .................. .S............. | DG.S- .................... ...S... | ....... .... |
| iPS:44 2071 | 21-225_4H6.007 | VH1\|1-02/D1\|1-1\|RF1/JH4 | ............. ............. ...... | D.......L. | ............ ... | ..H....... .......... | .................. .S............. | DA.S- .................... ...S... | ....... .... |
| iPS:44 2078 | 21-225_4H6.008 | VH1\|1-02/D1\|1-1\|RF1/JH4 | ............. ............. ...... | D.......L. | ............ ... | ..H....... .......... | .................. .S............. | SG.S- .................... ...S... | ....... .... |
| iPS:44 2085 | 21-225_4H6.009 | VH1\|1-02/D1\|1-1\|RF1/JH4 | ............. ............. ...... | D.......L. | ............ ... | ..H....... .......... | .................. .S............. | SG.S- .................... ...S... | ....... .... |
| iPS:44 2089 | 21-225_4H6.010 | VH1\|1-02/D1\|1-1\|RF1/JH4 | ............. ............. ...... | D.......L. | ............ ... | ..H....... .......... | .................. .S............. | DA.S- .................... ...S... | ....... .... |

| | | Germline | H_FR1 | H_CDR1 | H_FR2 | H_CDR2 | H_FR3 | H_CDR3 | H_FR4 |
|---|---|---|---|---|---|---|---|---|---|
| iPS:44 2093 | 21-225_4H6.011 | VH1\|1-02/D1\|1-1\|RF1/JH4 | ............. ............. ...... | D.......L. | ........... ... | ..H....... .......... | ............... .S............ | DA.S- ............... ...S... | ....... .... |
| iPS:44 3016 | 21-225_4H6.014 | VH1\|1-02/D1\|1-1\|RF1/JH4 | ............. ............. ...... | D.......L. | ........... ... | ..H....... .......... | ...............G. .S............ | DA.S- ............... ...S... | ....... .... |
| | | **VH3\|3-33\|D6\|6-6\|RF1/JH6** | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | EYSSSSYY---------------YYYGMDV | WGQGTTVTVSS |
| iPS:39 4041 | 21-225_5E5 | VH3\|3-33\|D6\|6-6\|RF1/JH6 | ............. ............. ...... | N......V.. | ........... ... | .......... .......... | ............... ...........T. | .VY..GW- ................D. .... | ....... .... |
| iPS:44 2115 | 21-225_5E5.003 | VH3\|3-33\|D6\|6-6\|RF1/JH6 | ............. ............. ...... | N......V.. | ........... ... | ........A. .......... | ............... ...........T. | .VY..GW- ................D. .... | ....... .... |
| iPS:44 2122 | 21-225_5E5.004 | VH3\|3-33\|D6\|6-6\|RF1/JH6 | ............. ............. ...... | N......V.. | ........... ... | .......... .....E.... | ............... ...........T. | .VY..GW- ................D. .... | ....... .... |
| iPS:44 2129 | 21-225_5E5.005 | VH3\|3-33\|D6\|6-6\|RF1/JH6 | ............. ............. ...... | N......V.. | ........... ... | .......... .....G.... | ............... ...........T. | .VY..GW- ................D. .... | ....... .... |
| iPS:44 2136 | 21-225_5E5.006 | VH3\|3-33\|D6\|6-6\|RF1/JH6 | ............. ............. ...... | N......V.. | ........... ... | .......... ......A... | ............... ...........T. | .VY..GW- ................D. .... | ....... .... |
| iPS:44 2171 | 21-225_5E5.011 | VH3\|3-33\|D6\|6-6\|RF1/JH6 | ............. ............. ...... | N......V.. | ..........A. ... | .......... .....E.... | ............... ...........T. | .VY..GW- ................D. .... | ....... .... |
| iPS:44 2178 | 21-225_5E5.012 | VH3\|3-33\|D6\|6-6\|RF1/JH6 | ............. ............. ...... | N......V.. | ..........A. ... | .......... ......A... | ............... ...........T. | .VY..GW- ................D. .... | ....... .... |
| iPS:44 2199 | 21-225_5E5.015 | VH3\|3-33\|D6\|6-6\|RF1/JH6 | ............. ............. ...... | N......V.. | ..........A. ... | .......... ......A... | ............... ...........T. | .VY..G.- ................D. .... | ....... .... |

| | | | H_FR1 | H_CDR1 | H_FR2 | H_CDR2 | H_FR3 | H_CDR3 | H_FR4 |
|---|---|---|---|---|---|---|---|---|---|
| iPS:44 2206 | 21-225_5E5.016 | VH3\|3-33/D6\|6-6\|RF1/JH6 | .............. .............. ...... | N......V.. ... | ............ ... | ........A. .......... | ..................T. | .VY..GF- .................D. .... | ....... .... |
| iPS:44 2213 | 21-225_5E5.017 | VH3\|3-33/D6\|6-6\|RF1/JH6 | .............. .............. ...... | N......V.. ... | ............ ... | .......... .....E.... | ..................T. | .VY..G.- .................D. .... | ....... .... |
| iPS:44 2220 | 21-225_5E5.018 | VH3\|3-33/D6\|6-6\|RF1/JH6 | .............. .............. ...... | N......V.. ... | ............ ... | .......... .......... | ..................T. | .VY..G.- .................D. .... | ....... .... |
| iPS:44 2227 | 21-225_5E5.019 | VH3\|3-33/D6\|6-6\|RF1/JH6 | .............. .............. ...... | N......V.. ... | ............ ... | .......... .......... | ..................T. | .VY..GF- .................D. .... | ....... .... |
| iPS:44 2255 | 21-225_5E5.023 | VH3\|3-33/D6\|6-6\|RF1/JH6 | .............. .............. ...... | N......V.. ... | ............ ... | ........A. .....E.... | ..................T. | .VY..G.- .................D. .... | ....... .... |
| iPS:44 2262 | 21-225_5E5.024 | VH3\|3-33/D6\|6-6\|RF1/JH6 | .............. .............. ...... | N......V.. ... | ............ ... | ........A. ......A... | ..................T. | .VY..G.- .................D. .... | ....... .... |

| | Germline | | H_FR1 | H_CDR1 | H_FR2 | H_CDR2 | H_FR3 | H_CDR3 | H_FR4 |
|---|---|---|---|---|---|---|---|---|---|
| | VH3\|3-07/D6\|6-6\|RF1/JH6 | | EVQLVES-GGGLVQPGGSLRLSCAASG-FTFS | S-----YWMS | WVRQAPGKGLEWVA | NIKQD---GSEKYYVDSVKG | RFTISRDNAKNSLYLQMNSLRAEDTAVYYCAR | EYSSSSYY----------------YYYGMDV | WGQGTTVTVSS |
| iPS:44 2269 | 21-225_5E5.025 | VH3\|3-07/D6\|6-6\|RF1/JH6 | ...........V. .............. ...... | N......V.H ... | ............ ... | V.WY...... N...AE.... | ..............T..... ..............T. | .VY..GW- .................D. .... | ....... .... |

| | Germline | | H_FR1 | H_CDR1 | H_FR2 | H_CDR2 | H_FR3 | H_CDR3 | H_FR4 |
|---|---|---|---|---|---|---|---|---|---|
| | VH3\|3-33/D4\|4-11\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYSNYYY----------------YYYGMDV | WGQGTTVTVSS |
| iPS:39 3930 | 21-225_7E1.1 | VH3\|3-33/D4\|4-11\|RF2/JH6 | .............. .............. ...... | ......F... ... | ............ ... | I..H...... .......... | .........N....... S............ | .L.MG-- ..................-- -.... | ....... .... |
| iPS:42 4460 | 21-225_7E1.001 | VH3\|3-33/D4\|4-11\|RF2/JH6 | .............. .............. ...... | ......F... ... | ............ ... | I..H...... .......... | .............. S............ | .L.MG-- ..................-- -.... | ....... .... |

| | | | | | | | | |
|---|---|---|---|---|---|---|---|---|
| iPS:44 2311 | 21-225_7E1 1.001.001 | VH3\|3-33/D4\|4-11\|RF2/J H6 | ............. ............. ...... | ......F... | ........... ... | I..H...... .......... | .................. S................. | .L.MG-- ..................-- -.... | ...... .... |
| iPS:44 2317 | 21-225_7E1 1.001.002 | VH3\|3-33/D4\|4-11\|RF2/J H6 | ............. ............. ...... | ......F... | ........... ... | I..H...... .......... | .................. S................. | .L.MG-- ..................-- -.... | ...... .... |
| iPS:44 2323 | 21-225_7E1 1.001.003 | VH3\|3-33/D4\|4-11\|RF2/J H6 | ............. ............. ...... | ......F... | ........... ... | I..HS..... .......... | .................. S................. | .L.MG-- ..................-- -.... | ...... .... |
| iPS:44 2330 | 21-225_7E1 1.001.004 | VH3\|3-33/D4\|4-11\|RF2/J H6 | ............. ............. ...... | ......F... | ........... ... | I..HE..... .......... | .................. S................. | .L.MG-- ..................-- -.... | ...... .... |
| iPS:44 2337 | 21-225_7E1 1.001.005 | VH3\|3-33/D4\|4-11\|RF2/J H6 | ............. ............. ...... | ......F... | ........... ... | I..H....A. .......... | .................. S................. | .L.MG-- ..................-- -.... | ...... .... |
| iPS:44 2344 | 21-225_7E1 1.001.006 | VH3\|3-33/D4\|4-11\|RF2/J H6 | ............. ............. ...... | ......F... | ........... ... | I..H...... .....E.... | .................. S................. | .L.MG-- ..................-- -.... | ...... .... |
| iPS:44 2351 | 21-225_7E1 1.001.007 | VH3\|3-33/D4\|4-11\|RF2/J H6 | ............. ............. ...... | ......F... | ........... ... | I..H...... .....A.... | .................. S................. | .L.MG-- ..................-- -.... | ...... .... |
| iPS:44 2358 | 21-225_7E1 1.001.008 | VH3\|3-33/D4\|4-11\|RF2/J H6 | ............. ............. ...... | ......F... | ........... ... | I..HE..... .....A.... | .................. S................. | .L.MG-- ..................-- -.... | ...... .... |
| iPS:44 2365 | 21-225_7E1 1.001.009 | VH3\|3-33/D4\|4-11\|RF2/J H6 | ............. ............. ...... | ......F... | ........... ... | I..HE..... .....E.... | .................. S................. | .L.MG-- ..................-- -.... | ...... .... |
| iPS:44 2372 | 21-225_7E1 1.001.010 | VH3\|3-33/D4\|4-11\|RF2/J H6 | ............. ............. ...... | ......F... | ........... ... | I..HS..... .....E.... | .................. S................. | .L.MG-- ..................-- -.... | ...... .... |
| iPS:44 2379 | 21-225_7E1 1.001.011 | VH3\|3-33/D4\|4-11\|RF2/J H6 | ............. ............. ...... | ......F... | ........... ... | I..HS..... .....A.... | .................. S................. | .L.MG-- ..................-- -.... | ...... .... |
| iPS:44 2386 | 21-225_7E1 1.001.012 | VH3\|3-33/D4\|4-11\|RF2/J H6 | ............. ............. ...... | ......F... | ........... ... | I..HS..... .......... | .................. S................. | .L.MG-- ..................-- -.... | ...... .... |

| | | | H_FR1 | H_CDR1 | H_FR2 | H_CDR2 | H_FR3 | H_CDR3 | H_FR4 |
|---|---|---|---|---|---|---|---|---|---|
| iPS:44 2390 | 21-225_7E1 1.001.013 | VH3\|3-33/D4\|4-11\|RF2/J H6 | .............. .............. ...... | ......F... | .......... ... | I..H...... .....E.... | S................ | .L.MG-- ...................-- -.... | ....... .... |
| iPS:44 2394 | 21-225_7E1 1.001.014 | VH3\|3-33/D4\|4-11\|RF2/J H6 | .............. .............. ...... | ......F... | .......... ... | I..HS..... .....E.... | S................ | .L.MG-- ...................-- -.... | ....... .... |
| iPS:44 2398 | 21-225_7E1 1.001.015 | VH3\|3-33/D4\|4-11\|RF2/J H6 | .............. .............. ...... | ......F... | .......... ... | I..HS..... .......... | S................ | .L.MG-- ...................-- -.... | ....... .... |
| iPS:44 2402 | 21-225_7E1 1.001.016 | VH3\|3-33/D4\|4-11\|RF2/J H6 | .............. .............. ...... | ......F... | .......... ... | I..HS..... .....E.... | S................ | .L.MG-- ...................-- -.... | ....... .... |
| iPS:44 2406 | 21-225_7E1 1.001.017 | VH3\|3-33/D4\|4-11\|RF2/J H6 | .............. .............. ...... | ......F... | .......... ... | I..HE..... ......A... | S................ | .L.MG-- ...................-- -.... | ....... .... |
| iPS:44 2410 | 21-225_7E1 1.001.018 | VH3\|3-33/D4\|4-11\|RF2/J H6 | .............. .............. ...... | ......F... | .......... ... | I..H....A. .....E.... | S................ | .L.MG-- ...................-- -.... | ....... .... |
| iPS:44 3027 | 21-225_7E1 1.001.023 | VH3\|3-33/D4\|4-11\|RF2/J H6 | .............. .............. ...... | ......F... | .......... ... | I..H....A. ......A... | S................ | .L.MG-- ...................-- -.... | ....... .... |
| | | Germline | H_FR1 | H_CDR1 | H_FR2 | H_CDR2 | H_FR3 | H_CDR3 | H_FR4 |
| | VH3\|3-07/D4\|4-11\|RF2/J H6 | | EVQLVES-GGGLVQPGGSLRLSCAASG-FTFS | S-----YWMS | WVRQAPGKGLEWVA | NIKQD---GSEKYYVDSVKG | RFTISRDNAKNSLYLQMNSLRAEDTAVYYCAR | DYSNYYY-------------YYYGMDV | WGQGTTVTVSS |
| iPS:44 2417 | 21-225_7E1 1.001.019 | VH3\|3-07/D4\|4-11\|RF2/J H6 | ...........V. .............. ...... | ......FG.H | .......... ... | I.WH...... N...AE.... | S................T..... | .L.MG-- ...................-- -.... | ....... .... |
| iPS:44 2431 | 21-225_7E1 1.001.021 | VH3\|3-07/D4\|4-11\|RF2/J H6 | ...........V. .............. ...... | ......FG.H | .......... ... | I.WHS..... N...AE.... | S................T..... | .L.MG-- ...................-- -.... | ....... .... |
| iPS:44 2438 | 21-225_7E1 1.001.022 | VH3\|3-07/D4\|4-11\|RF2/J H6 | ...........V. .............. ...... | ......FG.H | .......... ... | I.WHE..... N...AE.... | S................T..... | .L.MG-- ...................-- -.... | ....... .... |

FIGURE 55

Table 19A

**VARIALBLE HEAVY CHAIN CONSENSUS SEQUENCES**

| NAME (ORIGINAL AND PATENT) | Patent SEQ ID NO.: | SEQUENCE |
|---|---|---|
| VH-Consensus 1 (Table 21) (generated from 13 heavy chain sequences) | SEQ ID NO: 50352 | QVQLVQSGAEVKKPGASVKVSCKASGYTFTDYYMH WVRQAPGQGLEWMGWINPNNGGTNYAQKFQGRVT MTRDTSISTAYMELSRLRSDDTAVYYCARDGTGSFD YWGQGTLVTVSS |
| VH-Consensus 2 (Table 22) (generated from 11 heavy chain sequences) | SEQ ID NO: 50353 | QVQLVQSGAEVKKPGASVKVSCKASGYTFTGYYMH WVRQAPGQGLEWMGWINPNSGGTNYAQKFQGRVT MTRDTSISTAYMELSRLRSDDTAVYYCARSYYYGSG SYYNEFDYWGQGTLVTVSS |
| VH-Consensus 3 (Table 23) (generated from 15 heavy chain sequences) | SEQ ID NO: 50354 | QVQLVQSGAEVKKPGASVKVSCKASGYTFTNYDIN WVRQATGQGLEWMGWMHPNSGNTGYAQKFQGRV TMTRNTSISTAYMELSSLRSEDTAVYYCASSSGWYY FDYWGQGTLVTVSS |
| VH-Consensus 4 (Table 24) (generated from 23 heavy chain sequences) | SEQ ID NO: 50355 | EVQLVESGGGLVKPGGSLRLSCAASGFTFSSYSMNW VRQAPGKGLEWVSSISGSSSYIYYADSVKGRFTISRD NAKNSLYLQMNSLRAEDTAVYYCARVASFDYWGQ GTLVTVSS |
| VH-Consensus 5 | SEQ ID NO: 50356 | EVQLVESGGGLVKPGGSLRLSCAASGFTFSSYSMNW VRQAPGKGLEWVSSISGSSSYIYYADSVKGRFTISRD NAKNSLYLQMNSLRAEDTAVYYCARDRGSLWGQG TLVTVSS |
| VH-Consensus 6 (Table 26) (generated from 11 heavy chain sequences) | SEQ ID NO: 50357 | EVQLLESGGGLVQPGGSLRLSCAASGFTFSSYVMNW VRQAPGKGLEWVSAISGSGGSTYYADSVKGRFTISR DNSKNTLYLQMNSLRAEDTAVYYCARTGVFDYWG QGTLVTVSS |
| VH-Consensus 7 (Table 27) (generated from 30 heavy chain sequences) | SEQ ID NO: 50358 | EVQLLESGGGLVQPGGSLRLSCAASGFTFSSYAMSW VRQAPGKGLEWVSAISGRGGSTFHADSVKGRFTISR DNSKNTLYLQMNSLRAEDTAVYYCVKGELLEDYYF YGMDVWGQGTTVTVSS |
| VH-Consensus 8 (Table 28) (generated from 25 heavy chain sequences) | SEQ ID NO: 50359 | EVQLLESGGGLVQPGGSLRLSCAASEFTFSSYAMXW VRQAPGKGLEWVSVISGRGGNTFYADSVKGRFTISR DNSKNTLYLQMNSLRAEDTAVYYCASRLAVAGSEA FDIWGQGTMVTVSS |
| VH-Consensus 9 (Table 29) (generated from 14 heavy chain sequences) | SEQ ID NO: 50360 | QVQLVESGGGVVQPGRSLRLSCAASGFTFSSYGMH WVRQAPGKGLEWVAVIWXDGSNKYYADSVKGRFT ISRDNSKNTLYLQMNSLRAEDTAVYYCARDLSMGG MDVWGQGTTVTVSS |

FIGURE 55

| | | |
|---|---|---|
| VH-Consensus 10 (Table 30) (generated from 22 heavy chain sequences) | SEQ ID NO: 50361 | QVQLVESGGGVVQPGRSLRLSCAASGFTFSSYVMH WVRQAPGKGLEWVAVIWYDGSNKYYADSVKGRFT ISRDNSKNTLYLQMNSLRAEDTAVYYCAREKYSSS WYDYGMDVWGQGTTVTVSS |
| VH-Consensus 11 (Table 31) (generated from 16 heavy chain sequences) | SEQ ID NO: 50362 | QVQLVESGGGVVQPGRSLRLSCAASGFTFSDYGMH WVRQAPGKGLEWVAVIWYDESNKYYADSVKGRFTI SRDNSKNTLYLQMNSLRAEDTAVYYCAREIGWLDD YWGQGTLVTVSS |
| VH-Consensus 12 (Table 32) (generated from 71 heavy chain sequences) | SEQ ID NO: 50363 | QVQLVESGGGVVQPGRSLRLSCAASGFTFSDYGMH WVRQAPGKGLEWVAVIWYDENNKYYADSVKGRFT ISRDNSKNTLYLQMNSLRAEDTAVYYCARELGFRSD YWGQGTLVTVSS |
| VH-Consensus 13 (Table 33) (generated from 21 heavy chain sequences) | SEQ ID NO: 50364 | QLQLQESGPGLVKPSETLSLTCTVSGGSISRSSYYWG WIRQPPGKGLEWIGNIYYSGSTYYNPSLKSRVTISVD TSKNQFSLKLSSVTAADTAVYYCARHSSSWSLDYW GQGTLVTVSS |
| VH-Consensus 14 (Table 34) (generated from 13 heavy chain sequences) | SEQ ID NO: 50365 | QLQLQESGPGLVKPSETLSLTCTVSGGSISRSSYYWG WIRQPPGKGLEWIGNIYYSGSTYYNPSLKSRVTISVD TSKNQFSLKLSSVTAADTAVYYCGRHGKDWGLDY WGQGTLVTVSS |
| VH-Consensus 15 (Table 49) ) (generated from 149 heavy chain sequences) | SEQ ID NO: 50266 | QVQLVQS-GAEVKKPGASVKVSCKASG-YTFTN----- YDINWVRQATGQGLEWMGWMHPN--- SGNTGYAQKFQGRVTMTRNTSISTAYMELSSLRSED TAVYYCASSSGWY--------------------- YFDYWGQGTLVTVSS |
| VH-Consensus 16 (Table 50) ) (generated from 128 heavy chain sequences) | SEQ ID NO: 50267 | QVQLVES-GGGVVQPGRSLRLSCAASG-FTFSS----- YVMHWVRQAPGKGLEWVAVIWYD--- GSNKYYADSVKGRFTISRDNSKNTLYLQMNSLRAED TAVYYCARERYSSGW--------------- YDYGMDVWGQGTTVTVSS |
| VH-Consensus 17 (Table 51) ) (generated from 117 heavy chain sequences) | SEQ ID NO: 50268 | QVQLVES-GGGVVQPGRSLRLSCAASG-FTFSD----- YGMHWVRQAPGKGLEWVAVIWYD--- ENNKYYADSVKGRFTISRDNSKNTLYLQMNSLRAE DTAVYYCARELGF-------------------- SDYWGQGTLVTVSS |
| VH-Consensus 18 (Table 52) ) (generated from 91 heavy chain sequences) | SEQ ID NO: 50269 | QVQLQQW-GAGLLKPSETLSLTCAVHG-GSFSG----- CYWSWIRQPPGKGLEWIGEINH---- SGSTNYNPSLKSRVTISVDTSKNQFSLKLSSVTAADT AVYYCARDYGG---------------------- MDVWGQGTTVTVSS |

FIGURE 55

| VH-Consensus 19 (Table 53) (generated from 74 heavy chain sequences) | SEQ ID NO: 50270 | QVQLVQS-GAEVKKPGASVKVSCKASG-YTFT<u>N-----</u><br><u>YDIN</u>WVRQATGQGLEWMGW<u>MHPN---</u><br><u>SGNTGY</u>AQKF<u>Q</u>GRVTMTRNTSISTAYMELSSLRSED<br>TAVYYCAIS<u>SGWY--------------------</u><br><u>WFDP</u>WGQGTLVTVSS |
|---|---|---|
| VH-Consensus 20 (Table 54) (generated from 53 heavy chain sequences) | SEQ ID NO: 50271 | QVQLVES-GGGVVQPGRSLRLSCAASG-FTFS<u>S-----</u><br><u>YGMH</u>WVRQAPGKGLEWVA<u>VIWYD---</u><br><u>GSNKNY</u>AD<u>S</u>VKGRFTISRDNSKNTLYLQMNSLRAED<br>TAVYYCARD<u>QGVGY------------------</u><br><u>YGLD</u>VWGQGTTVTVSS |
| VH-Consensus 21 (Table 55) (generated from 52 heavy chain sequences) | SEQ ID NO: 50272 | QVQLVQS-GAEVKKPGASVKVSCKASG-YTFT<u>G-----</u><br><u>YYMH</u>WVRQAPGQGLEWMGW<u>INPN---</u><br><u>SGGTNY</u>AQKF<u>Q</u>GRVTMTRDTSISTAYMELSRLRSDD<br>TAVYYCARD<u>GTS----------------------</u><br><u>SFDY</u>WGQGTLVTVSS |
| VH-Consensus 22 (Table 56) (generated from 49 heavy chain sequences) | SEQ ID NO: 50273 | QVQLVES-GGGVVQPGRSLRLSCAASG-FTFS<u>S-----</u><br><u>YGMH</u>WVRQAPGKGLEWVA<u>VIWHD---</u><br><u>GSNKYY</u>ADSVKGRFTISRDNSKNTLYLQMNSLRAED<br>TAVYYCARD<u>LSMG---------------------</u><br><u>GMDV</u>WGQGTTVTVSS |
| VH-Consensus 23 (Table 57) (generated from 37 heavy chain sequences) | SEQ ID NO: 50274 | QVQLVES-GGGVVQPGRSLRLSCAASG-FTFS<u>D-----</u><br><u>YGMH</u>WVRQAPGKGLEWVA<u>VIWYD---</u><br><u>ESNKYY</u>ADSVKGRFTISRDNSKNTLYLQMNSLRAED<br>TAVYYCAREV<u>GW----------------------</u><br><u>LDDY</u>WGQGTLVTVSS |
| VH-Consensus 24 (Table 58) (generated from 35 heavy chain sequences) | SEQ ID NO: 50275 | EVQLVES-GGGLVKPGGSLRLSCAASG-FTFS<u>S-----</u><br><u>YSMN</u>WVRQAPGKGLEWVS<u>SISGS---</u><br><u>SSYIYY</u>ADSVKGRFTISRDNAKNSLYLQMNSLRAED<br>TAVYYCARD<u>RG-------------------------</u><br><u>SSW</u>GQGTLVTVSS |
| VH-Consensus 25 (Table 59) (generated from 32 heavy chain sequences) | <u>SEQ ID NO:<br>50276</u> | EVQLLES-GGGLVQPGGSLRLSCAASE-FTFS<u>S-----</u><br><u>YAMS</u>WVRQAPGKGLEWVS<u>VISGR---</u><br><u>GGNTFY</u>ADSVKGRFTISRDNSKNTLYLQMNSLRAED<br>TAVYYCAS<u>RIAVAG------------------</u><br><u>SEAFDI</u>WGQGTMVTVSS |
| VH-Consensus 26 (Table 60) (generated from 30 heavy chain sequences) | SEQ ID NO: 50277 | EVQLLES-GGGLVQPGGSLRLSCAASG-FTFS<u>S-----</u><br><u>YAMS</u>WVRQAPGKGLEWVS<u>AISGR---</u><br><u>GGSTFH</u>ADSVKGRFTISRDNSKNTLYLQMNSLRAED<br>TAVYYCVKG<u>ELLEDY----------------</u><br><u>YFYGMDV</u>WGQGTTVTVSS |
| VH-Consensus 27 (Table 61 ) (generated | SEQ ID NO: 50278 | QVQLVES-GGGVVQPGRSLRLSCAASG-FTFS<u>S-----</u><br><u>YGMH</u>WVRQAPGKGLEWVA<u>VIWYD---</u> |

FIGURE 55

| from 29 heavy chain sequences) | | GSNKYYADSVKGRFTISRDNSKNTLYLQMNSLRAED TAVYYCARDRVYCSSTSC---------- PYYYYYGMDVWGQGTTVTVSS |
|---|---|---|
| VH-Consensus 28 (Table 62) (generated from 28 heavy chain sequences) | SEQ ID NO: 50279 | EVQLVES-GGGLVKPGGSLRLSCAASG-FTFSS----- YSMNWVRQAPGKGLEWVSSISGS--- SSYIYYADSVKGRFTISRDNAKNSLYLQMNSLRAED TAVYYCARVAS----------------------- FDYWGQGTLVTVSS |
| VH-Consensus 29 (Table 63) (generated from 26 heavy chain sequences) | SEQ ID NO: 50280 | QVQLQES-GPGLVKPSQTLSLTCTVSG-GSISSG--- DYYWNWIRQHPGKGLEWIGYIFY---- SGSTYYNPSLKSRVTISVDTSKNQFSLKLSSVTAADT AVYYCARGDYDGSGSY------------ HYYYGMDVWGQGTTVTVSS |
| VH-Consensus 30 (Table 64) (generated from 24 heavy chain sequences) | SEQ ID NO: 50281 | EVQLLES-GGGLVQPGGSLRLSCAASG-FTFSS----- YAMSWVRQAPGKGLEWVSVISGG--- GSSTYYADSVKGRFTISRDNSKNTLYLQMNSLRAED TAVYYCAKWRGNPT----------------- DYGMDVWGQGTTVTVSS |
| VH-Consensus 31 (Table 65) (generated from 24 heavy chain sequences) | SEQ ID NO: 50282 | QVQLVES-GGGVVQPGRSLRLSCAASG-FTFSS----- YGMHWVRQAPGKGLEWVAIIWYD--- GSNKYYADSVKGRFTISRDNSKNTLYLQMNSLRAED TAVYYCARDHYDFW----------------- SGHFDYWGQGTLVTVSS |
| VH-Consensus 32 (Table 66) (generated from 24 heavy chain sequences) | SEQ ID NO: 50283 | QVQLQQW-GAGLLKPSETLSLTCAVYG-GSFSG----- YYWSWIRQPPGKGLEWIGEINH---- SGRTNYNPSLKSRVTISVDTSKNQFSLKLSSVTAADT AVYYCARDYGG----------------------- LDYWGQGTLVTVSS |
| VH-Consensus 33 (Table 67) (generated from 22 heavy chain sequences) | SEQ ID NO: 50284 | QLQLQES-GPGLVKPSETLSLTCTVSG-GSISRS--- SYYWGWIRQPPGKGLEWIGNIYY---- SGSTYYNPSLKSRVTISVDTSKNQFSLKLSSVTAADT AVYYCARHSSSW-------------------- SLDYWGQGTLVTVSS |
| VH-Consensus 34 (Table 68) (generated from 19 heavy chain sequences) | SEQ ID NO: 50285 | QVQLVQS-GAEVKKPGASVKVSCKASG-YTFTG----- YYIHWVRQAPGQGLEWMGWINPN--- SGGTNYAQKFQGRVTMTRDTSISTAYMELSRLRSDD TAVYYCARGYSYGY------------------- NWFDPWGQGTLVTVSS |
| VH-Consensus 35 (Table 69) (generated from 18 heavy chain sequences) | SEQ ID NO: 50286 | QVQLVES-GGGVVQPGRSLRLSCAASG-FTFSH----- YGMHWVRQAPGKGLEWVAVIWYD--- GSNKYYADSVKGRFTISRDNSKNTLYLQMNSLRAED |

FIGURE 55

| | | |
|---|---|---|
| | | TAVYYCAR<u>GDWNP</u>------------------<br><u>EGMDV</u>WGQGTTVTVSS |
| VH-Consensus 36<br>(Table 70) (generated<br>from 17 heavy chain<br>sequences) | SEQ ID NO:<br>50287 | EVQLLES-GGGLVQPGGSLRLSCAASG-FTFS<u>S</u>-----<br><u>YAMS</u>WVRQAPGKGLEWVS<u>AISGS</u>---<br>GG<u>NTFY</u>AD<u>SVKG</u>RFTISRDNSKNTLYLQMNSLRAED<br>TAVYYCAKK<u>DYDYVW</u>----------------<br><u>GSPYFD</u>YWGQGTLVTVSS |
| VH-Consensus 37<br>(Table 71) (generated<br>from 16 heavy chain<br>sequences) | SEQ ID NO:<br>50288 | QVQLVQS-GAEVKKPGASVKVSCKASG-YTFT<u>G</u>-----<br><u>YYMH</u>WVRQAPGQGLEWMG<u>WINPN</u>---<br>SGG<u>TNY</u>AQKFQGRVTMTRDTSISTAYMELSRLRSDD<br>TAVYYCARS<u>YYYGSGS</u>---------------<br><u>YYNEFD</u>YWGQGTLVTVSS |
| VH-Consensus 38<br>(Table 72) (generated<br>from 14 heavy chain<br>sequences) | SEQ ID NO:<br>50289 | QVQLVQS-GAEVKKPGASVKVSCKASG-YTFT<u>G</u>-----<br><u>YYIH</u>WVRQAPGQGLEWMG<u>WINPY</u>---<br>SG<u>DTNY</u>AQKFQGRVTMTRDTSISTAYMELSRLRFDD<br>TAVFYCAR<u>DWGGYS</u>S----------------<br><u>YYYGMDV</u>WGQGTTVTVSS |
| VH-Consensus 39<br>(Table 73) (generated<br>from 14 heavy chain<br>sequences) | SEQ ID NO:<br>50290 | EVQLVES-GGGLVKPGGSLRLSCAASG-FTFS<u>S</u>-----<br><u>YSMN</u>WVRQAPGKGLEWVS<u>SISGS</u>---<br>S<u>SYIY</u>YAD<u>SVKG</u>RFTISRDNAKNSLYLQMNSLRAED<br>TAVYYCARL<u>T</u>-----------------------<br><u>FD</u>YWGQGTLVTVSS |
| VH-Consensus 40<br>(Table 74) (generated<br>from 14 heavy chain<br>sequences) | SEQ ID NO:<br>50291 | QVQLVES-GGGVVQPGRSLRLSCAASG-FTFS<u>T</u>-----<br><u>YGMH</u>WVRQAPGKGLEWVA<u>IIWYD</u>---<br>G<u>TNKY</u>YAD<u>SVKG</u>RFTISRDNSKNTLYLQLNSLRAED<br>TAVYYCAR<u>DPLRGYN</u>-----------------<br><u>DPVMDY</u>WGQGTLVTVSS |
| VH-Consensus 41<br>(Table 75) (generated<br>from 13 heavy chain<br>sequences) | SEQ ID NO:<br>50292 | EVQLLES-GGGLVQPGGSLRLSCAASG-FTFS<u>S</u>-----<br><u>YAMS</u>WVRQAPGKGLEWVS<u>AISGR</u>---<br>GG<u>NTFY</u>AD<u>SVKG</u>RFTISRDNSKNTLYLQMNSLRAED<br>TAVYYCAKR<u>VTDYGG</u>-----------------<br><u>NDWFDP</u>WGQGTLVTVSS |
| VH-Consensus 42<br>(Table 76) (generated<br>from 13 heavy chain<br>sequences) | SEQ ID NO:<br>50293 | QVQLVES-GGGVVQPGRSLRLSCAASG-FTFS<u>T</u>-----<br><u>YGMH</u>WVRQAPGKGLEWVA<u>VIWYG</u>---<br>G<u>SNKDY</u>AD<u>SVKG</u>RFTISRDISKNTLYLQMNSLRAED<br>TAVYYCAR<u>DRDYCSGGSC</u>----------<br><u>PYYYYYGMDV</u>WGQGTTVTVSS |
| VH-Consensus 43<br>(Table 77) (generated<br>from 13 heavy chain<br>sequences) | SEQ ID NO:<br>50294 | QLQLQES-GPGLVKPSETLSLTCTVSG-GSIS<u>RS</u>---<br><u>SYYW</u>GWIRQPPGKGLEWIG<u>NIYY</u>----<br>SG<u>STYY</u>NP<u>SLKS</u>RVTISVDTSKNQFSLKLSSVTAADT<br>AVYYCGR<u>HGKDW</u>---------------------<br><u>GLDY</u>WGQGTLVTVSS |

# EP 4 435 105 A2

FIGURE 55

| VH-Consensus 44 (Table 78) (generated from 12 heavy chain sequences) | SEQ ID NO: 50295 | QVQLVQS-GAEVKKPGASVKVSCKASG-YTFTG----- YYMHWVRQAPGQGLEWMGWIKPN--- SGGTNQAQKFQGRVTMTRDTSISTAYMELSGLRSDD TAVYYCARAPGTAAAG-------------- TWGYFDYWGQGTLVTVSS |
|---|---|---|
| VH-Consensus 45 (Table 79) (generated from 12 heavy chain sequences) | SEQ ID NO: 50296 | QITLKES-GPTLVKPTQTLTLTCTFSG-FSLSTG--- GVGVGWIRQPPGKALEWLALIYW---- DDDKRYSPSLKSRLTITKDTSKNQVVLTMTNMDPVD TATYYCAHLIAV--------------------- AFDYWGQGTLVTVSS |
| VH-Consensus 46 (Table 80) (generated from 11 heavy chain sequences) | SEQ ID NO: 50297 | QVQLVES-GGGVVQPGRSLRLSCAASG-FTFSS----- YGMHWVRQAPGKGLEWVAIIWYD--- GSYKYYADSVKGRFTISRDNSKNTLYLQMNSLRAED TAVYYCAREAYDFW----------------- SGYFDYWGQGTLVTVSS |
| VH-Consensus 47 (Table 81) (generated from 11 heavy chain sequences) | SEQ ID NO: 50298 | QVQLVES-GGGVVQPGRSLRLSCAASG-FTFSS----- YGMHWVRQAPGKGLEWVAVIWYD--- GSNKYYADSVKGRFTISRDNSKNTLYLQMNSLRAED TAVYYCARDRDYGD------------------- YGMDVWGQGTTVTVSS |
| VH-Consensus 48 (Table 82) (generated from 10 heavy chain sequences) | SEQ ID NO: 50299 | EVQLLES-GGGLVQPGGSLRLSCAASG-FTFSS----- YAMSWVRQAPGKGLEWVSAISGS--- GGNTFYADSVKGRFTISRDNSKNTLYLQMNSLRAED TAVYYCAKLGKDYY----------------- YYGMDVWGQGTTVTVSS |
| VH-Consensus 49 (Table 83) (generated from 10 heavy chain sequences) | SEQ ID NO: 50300 | EVQLLES-GGGLVQPGGSLRLSCAASG-FTFSS----- YVMSWVRQAPGKGLEWVSAMSGS--- GGRTYYADSVKGRFTISRDNSKNTLYLQMNSLRAED TAVYYCAKLTA----------------------- FDYWGQGTLVTVSS |
| VH-Consensus 50 (Table 84) (generated from 10 heavy chain sequences) | SEQ ID NO: 50301 | QVQLVES-GGGVVQPGRSLRLSCAASG-FTFSS----- YGMHWVRQAPGKGLEWVAIISYA--- GSNKYYADSVKGRFTISRDNSKNTLYLQMNSLRAED TAVYYCVRRGYSYG----------------- GYGMDVWGQGTTVTVSS |
| VH-Consensus 51 (Table 85) (generated from 10 heavy chain sequences) | SEQ ID NO: 50302 | QVQLVES-GGGVVQPGRSLRLSCAASG-FTFSD----- YVMHWVRQAPGKGLEWVAVIWYD--- GSNKYYADSVKGRFTISRDNSKNTLYLQMNSLRAED TAVYYCAREPYTSGW---------------- YDYGMDVWGQGTTVTVSS |

3373

FIGURE 55

| VH-Consensus 52 (Table 86) (generated from 9 heavy chain sequences) | SEQ ID NO: 50303 | QVQLVQS-GAEVKKPGASVKVSCKASG-YTFNS----- YGISWVRQAPGQGLEWMGWISAY--- NGNTKYAQKLQGRVTMTTDTSTSTAYMELRSLRSD DTAVYYCARHDFWSGY---------------- YKGMDVWGQGTTVTVSS |
|---|---|---|
| VH-Consensus 53 (Table 87) (generated from 9 heavy chain sequences) | SEQ ID NO: 50304 | EVQLLES-GGGLVQPGGSLRLSCAASG-FTFSS----- YAMSWVRQAPGKGLEWVSAISGR---GG NTFDADSVKGRFTISRDNSKNTLYLQMNSLRAEDTA VYYCAKERSGS-------------------- YFDYWGQGTLVTVSS |
| VH-Consensus 54 (Table 88) (generated from 9 heavy chain sequences) | SEQ ID NO: 50305 | QVQLVES-GGGVVQPGRSLRLSCAASG-FTFSN----- YGMHWVRQAPGKGLEWVAVIWHD--- GSNKYYADSVKGRFTISRDNSKNTLYLQMNSLRAED TAVYYCARENSSS------------------- YYFDYWGQGTLVTVSS |
| VH-Consensus 55 (Table 89) (generated from 8 heavy chain sequences) | SEQ ID NO: 50306 | QITLKES-GPTLVKPTQTLTLTCTFSG-FSLSTS--- GVGVGWIRQPPGKALEWLALINW---- NDDKRYSPSLKSRFTITRDTSKDQVVLTMTNMDPVD TATYYCAHKATWV-------------------- AFDIWGQGTMVTVSS |
| VH-Consensus 56 (Table 90) (generated from 8 heavy chain sequences) | SEQ ID NO: 50307 | EVQLLES-GGGLVQPGGSLRLSCAASG-FTFSS----- YVMNWVRQAPGKGLEWVSAISGS--- GGRTYYADSVKGRFTISRDNSKNTLYLQMNSLRAED TAVYYCARTAT----------------------- FDYWGQGTLVTVSS |
| VH-Consensus 57 (Table 91) (generated from 8 heavy chain sequences) | SEQ ID NO: 50308 | EVQLLES-GGGLVQPGGSLRLSCAASG-FTFSS----- YAMSWVRQAPGKGLEWVSVISGR--- GGTTFYADSVKGRFTISRDNSKNTLYLQMNSLRAED TAVYYCAKRTPSD-------------------- VFDIWGQGTMVTVSS |
| VH-Consensus 58 (Table 92) (generated from 8 heavy chain sequences) | SEQ ID NO: 50309 | QVQLVES-GGGVVQPGRSLRLSCAASG-FTFSS----- YGMHWVRQAPGKGLEWVAVIWYD--- GSNKYYADSVKGRFTISRDNSKNTLYLQMNSLRAED TAVYYCARDRPRS------------------SAF DYWGQGTLVTVSS |
| VH-Consensus 59 (Table 93) (generated from 8 heavy chain sequences) | SEQ ID NO: 50310 | EVQLVES-GGGLVQPGGSLRLSCAASG-FTFSS----- YNMNWVRQAPGKGLEWVSYISRS--- SNTKYYADSVKGRFTISRDNAKNSLYLQMNSLRDED TAVYYCARDRSGSYGY-------------- FYYYGLDVWGQGTTVTVSS |
| VH-Consensus 60 (Table 94) (generated | SEQ ID NO: 50311 | QVQLQES-GPGLVKPSQTLSLTCTVSG-GSIRSG--- GDYWSWIRQHPGKGLEWIGYIYY---- |

FIGURE 55

| from 8 heavy chain sequences) | | SGSTYYNPSLKSRVTISVDTSKNQFSLKLSSVTAADT AVYYCARDSSSY--------------------- GMDVWGQGTTVTVSS |
|---|---|---|

**FIGURE 55**

Table 19B

**VARIABLE HEAVY CDR CONSENSUS SEQUENCES I**

| NAME (ORIGINAL AND PATENT) | Patent SEQ ID NO: | CDR | Sequence |
|---|---|---|---|
| VH-CONSENSUS-1 | 50381 | VH1 | DYYMH |
| TABLE 21 | 50382 | VH2 | WINPNNGGTNYAQKFQG |
| | 50383 | VH3 | DGTGSFDY |
| | | | |
| VH-CONSENSUS-2 | 50384 | VH1 | GYYMH |
| TABLE 22 | 50385 | VH2 | WINPNSGGTNYAQKFQG |
| | 50386 | VH3 | SYYYGSGSYYNEFDY |
| | | | |
| VH-CONSENSUS-3 | 50387 | VH1 | NYDIN |
| TABLE 23 | 50388 | VH2 | WMHPNSGNTGYAQKFQG |
| | 50389 | VH3 | SSGWYYFDY |
| | | | |
| VH-CONSENSUS-4 | 50390 | VH1 | SYSMN |
| TABLE 24 | 50391 | VH2 | SISGSSSYIYYADSVKG |
| | 50392 | VH3 | VASFDY |
| | | | |
| VH-CONSENSUS-5 | 50393 | VH1 | SYSMN |
| TABLE 25 | 50394 | VH2 | SISGSSSYIYYADSVKG |
| | 50395 | VH3 | DRGSL |
| | | | |
| VH-CONSENSUS-6 | 50396 | VH1 | SYVMN |
| TABLE 26 | 50397 | VH2 | AISGSGGSTYYADSVKG |
| | 50398 | VH3 | TGVFDY |
| | | | |
| VH-CONSENSUS-7 | 50399 | VH1 | SYAMS |
| TABLE 27 | 50400 | VH2 | AISGRGGSTFHADSVKG |
| | 50401 | VH3 | GELLEDYYFYGMDV |
| | | | |
| VH-CONSENSUS-8 | 50402 | VH1 | SYAMX |
| TABLE 28 | 50403 | VH2 | VISGRGGNTFYADSVKG |
| | 50404 | VH3 | RLAVAGSEAFDI |
| | | | |
| VH-CONSENSUS-9 | 50405 | VH1 | SYGMH |
| TABLE 29 | 50406 | VH2 | VIWXDGSNKYYADSVKG |
| | 50407 | VH3 | DLSMGGMDV |
| | | | |
| VH-CONSENSUS-10 | 50408 | VH1 | SYVMH |
| TABLE 30 | 50409 | VH2 | VIWYDGSNKYYADSVKG |
| | 50410 | VH3 | EKYSSSWYDYGMDV |
| | | | |
| VH-CONSENSUS-11 | 50411 | VH1 | DYGMH |
| TABLE 31 | 50412 | VH2 | VIWYDESNKYYADSVKG |
| | 50413 | VH3 | EIGWLDDY |

**FIGURE 55**

| | | | |
|---|---|---|---|
| VH-CONSENSUS-12 | 50414 | VH1 | DYGMH |
| TABLE 32 | 50415 | VH2 | VIWYDENNKYYADSVKG |
| | 50416 | VH3 | ELGFRSDY |
| | | | |
| VH-CONSENSUS-13 | 50417 | VH1 | RSSYYWG |
| TABLE 33 | 50418 | VH2 | NIYYSGSTYYNPSLKS |
| | 50419 | VH3 | HSSSWSLDY |
| | | | |
| VH-CONSENSUS-14 | 50420 | VH1 | RSSYYWG |
| TABLE 34 | 50421 | VH2 | NIYYSGSTYYNPSLKS |
| | 50422 | VH3 | HGKDWGLDY |
| | | | |
| VH-CONSENSUS-15 | 50468 | VH1 | NYDIN |
| TABLE 49 | 50469 | VH2 | WMHPNSGNTGYAQKFQG |
| | 50470 | VH3 | SSGWYYFDY |
| | | | |
| VH-CONSENSUS-16 | 50471 | VH1 | SYVMH |
| TABLE 50 | 50472 | VH2 | VIWYDGSNKYYADSVKG |
| | 50473 | VH3 | ERYSSGWYDYGMDV |
| | | | |
| VH-CONSENSUS-17 | 50474 | VH1 | DYGMH |
| TABLE 51 | 50475 | VH2 | VIWYDENNKYYADSVKG |
| | 50476 | VH3 | ELGFSDY |
| | | | |
| VH-CONSENSUS-18 | 50477 | VH1 | GCYWS |
| TABLE 52 | 50478 | VH2 | EINHSGSTNYNPSLKS |
| | 50479 | VH3 | DYGGMDV |
| | | | |
| VH-CONSENSUS-19 | 50480 | VH1 | NYDIN |
| TABLE 53 | 50481 | VH2 | WMHPNSGNTGYAQKFQG |
| | 50482 | VH3 | SSGWYWFDP |
| | | | |
| VH-CONSENSUS-20 | 50483 | VH1 | SYGMH |
| TABLE 54 | 50484 | VH2 | VIWYDGSNKNYADSVKG |
| | 50485 | VH3 | DQGVGYYGLDV |
| | | | |
| VH-CONSENSUS-21 | 50486 | VH1 | GYYMH |
| TABLE 55 | 50487 | VH2 | WINPNSGGTNYAQKFQG |
| | 50488 | VH3 | DGTSSFDY |
| | | | |
| VH-CONSENSUS-22 | 50489 | VH1 | SYGMH |
| TABLE 56 | 50490 | VH2 | VIWHDGSNKYYADSVKG |
| | 50491 | VH3 | DLSMGGMDV |
| | | | |
| VH-CONSENSUS-23 | 50492 | VH1 | DYGMH |
| TABLE 57 | 50493 | VH2 | VIWYDESNKYYADSVKG |
| | 50494 | VH3 | EVGWLDDY |

**FIGURE 55**

| | | | |
|---|---|---|---|
| | | | |
| VH-CONSENSUS-24 TABLE 58 | 50495 | VH1 | SYSMN |
| | 50496 | VH2 | SISGSSSYIYYADSVKG |
| | 50497 | VH3 | DRGSS |
| | | | |
| VH-CONSENSUS-25 TABLE 59 | 50498 | VH1 | SYAMS |
| | 50499 | VH2 | VISGRGGNTFYADSVKG |
| | 50500 | VH3 | RIAVAGSEAFDI |
| | | | |
| VH-CONSENSUS-26 TABLE 60 | 50501 | VH1 | SYAMS |
| | 50502 | VH2 | AISGRGGSTFHADSVKG |
| | 50503 | VH3 | GELLEDYYFYGMDV |
| | | | |
| VH-CONSENSUS-27 TABLE 61 | 50504 | VH1 | SYGMH |
| | 50505 | VH2 | VIWYDGSNKYYADSVKG |
| | 50506 | VH3 | DRVYCSSTSCPYYYYYGMDV |
| | | | |
| VH-CONSENSUS-28 TABLE 62 | 50507 | VH1 | SYSMN |
| | 50508 | VH2 | SISGSSSYIYYADSVKG |
| | 50509 | VH3 | VASFDY |
| | | | |
| VH-CONSENSUS-29 TABLE 63 | 50510 | VH1 | SGDYYWN |
| | 50511 | VH2 | YIFYSGSTYYNPSLKS |
| | 50512 | VH3 | GDYDGSGSYHYYYGMDV |
| | | | |
| VH-CONSENSUS-30 TABLE 64 | 50513 | VH1 | SYAMS |
| | 50514 | VH2 | VISGGGSSTYYADSVKG |
| | 50515 | VH3 | WRGNPTDYGMD |
| | | | |
| VH-CONSENSUS-31 TABLE 65 | 50516 | VH1 | SYGMH |
| | 50517 | VH2 | IIWYDGSNKYYADSVKG |
| | 50518 | VH3 | DHYDFWSGHFDY |
| | | | |
| VH-CONSENSUS-32 TABLE 66 | 50519 | VH1 | GYYWS |
| | 50520 | VH2 | EINHSGRTNYNPSLKS |
| | 50521 | VH3 | DYGGLDY |
| | | | |
| VH-CONSENSUS-33 TABLE 67 | 50522 | VH1 | RSSYYWG |
| | 50523 | VH2 | NIYYSGSTYYNPSLKS |
| | 50524 | VH3 | HSSSWSLDY |
| | | | |
| VH-CONSENSUS-34 TABLE 68 | 50525 | VH1 | GYYIH |
| | 50526 | VH2 | WINPNSGGTNYAQKFQG |
| | 50527 | VH3 | GYSYGYNWFDP |
| | | | |
| VH-CONSENSUS-35 TABLE 69 | 50528 | VH1 | HYGMH |
| | 50529 | VH2 | VIWYDGSNKYYADSVKG |
| | 50530 | VH3 | GDWNPEGMDV |

FIGURE 55

| VH-CONSENSUS-36 TABLE 70 | 50531 | VH1 | SYAMS |
|---|---|---|---|
| | 50532 | VH2 | AISGSGGNTFYADSVKG |
| | 50533 | VH3 | KDYDYVWGSPYFDY |
| VH-CONSENSUS-37 TABLE 71 | 50534 | VH1 | GYYMH |
| | 50535 | VH2 | WINPNSGGTNYAQKFQG |
| | 50536 | VH3 | SYYYGSGSYYNEFDY |
| VH-CONSENSUS-38 TABLE 72 | 50537 | VH1 | GYYIH |
| | 50538 | VH2 | WINPYSGDTNYAQKFQG |
| | 50539 | VH3 | DWGGYSSYYYGMDV |
| VH-CONSENSUS-39 TABLE 73 | 50540 | VH1 | SYSMN |
| | 50541 | VH2 | SISGSSSYIYYADSVKG |
| | 50542 | VH3 | LTFDY |
| VH-CONSENSUS-40 TABLE 74 | 50543 | VH1 | TYGMH |
| | 50544 | VH2 | IIWYDGTNKYYADSVKG |
| | 50545 | VH3 | DPLRGYNDPVMDY |
| VH-CONSENSUS-41 TABLE 75 | 50546 | VH1 | SYAMS |
| | 50547 | VH2 | AISGRGGNTFYADSVKG |
| | 50548 | VH3 | RVTDYGGNDWFDP |
| VH-CONSENSUS-42 TABLE 76 | 50549 | VH1 | TYGMH |
| | 50550 | VH2 | VIWYGGSNKDYADSVKG |
| | 50551 | VH3 | DRDYCSGGSCPYYYYYGMDV |
| VH-CONSENSUS-43 TABLE 77 | 50552 | VH1 | RSSYYWG |
| | 50553 | VH2 | NIYYSGSTYYNPSLKS |
| | 50554 | VH3 | HGKDWGLDY |
| VH-CONSENSUS-44 TABLE 78 | 50555 | VH1 | GYYMH |
| | 50556 | VH2 | WIKPNSGGTNQAQKFQG |
| | 50557 | VH3 | APGTAAAGTWGYFDY |
| VH-CONSENSUS-45 TABLE 79 | 50558 | VH1 | TGGVGVG |
| | 50559 | VH2 | LIYWDDDKRYSPSLKS |
| | 50560 | VH3 | LIAVAFDY |
| VH-CONSENSUS-46 TABLE 80 | 50561 | VH1 | SYGMH |
| | 50562 | VH2 | IIWYDGSYKYYADSVKG |
| | 50563 | VH3 | EAYDFWSGYFDY |
| VH-CONSENSUS-47 TABLE 81 | 50564 | VH1 | SYGMH |
| | 50565 | VH2 | VIWYDGSNKYYADSVKG |
| | 50566 | VH3 | DRDYGDYGMDV |

**FIGURE 55**

| VH-CONSENSUS-48 | 50567 | VH1 | SYAMS |
| TABLE 82 | 50568 | VH2 | AISGSGGNTFYADSVKG |
| | 50569 | VH3 | LGKDYYYYGMDV |
| | | | |
| VH-CONSENSUS-49 | 50570 | VH1 | SYVMS |
| TABLE 83 | 50571 | VH2 | AMSGSGGRTYYADSVKG |
| | 50572 | VH3 | LTAFDY |
| | | | |
| VH-CONSENSUS-50 | 50573 | VH1 | SYGMH |
| TABLE 84 | 50574 | VH2 | IISYAGSNKYYADSVKG |
| | 50575 | VH3 | RGYSYGGYGMDV |
| | | | |
| VH-CONSENSUS-51 | 50576 | VH1 | DYVMH |
| TABLE 85 | 50577 | VH2 | VIWYDGSNKYYADSVKG |
| | 50578 | VH3 | EPYTSGWYDYGMDV |
| | | | |
| VH-CONSENSUS-52 | 50579 | VH1 | SYGIS |
| TABLE 86 | 50580 | VH2 | WISAYNGNTKYAQKLQG |
| | 50581 | VH3 | HDFWSGYYKGMDV |
| | | | |
| VH-CONSENSUS-53 | 50582 | VH1 | SYAMS |
| TABLE 87 | 50583 | VH2 | AISGRGG NTFDADSVKG |
| | 50584 | VH3 | ERSGSYFDY |
| | | | |
| VH-CONSENSUS-54 | 50585 | VH1 | NYGMH |
| TABLE 88 | 50586 | VH2 | VIWHDGSNKYYADSVKG |
| | 50587 | VH3 | ENSSSYYFDY |
| | | | |
| VH-CONSENSUS-55 | 50588 | VH1 | TSGVGVG |
| TABLE 89 | 50589 | VH2 | LINWNDDKRYSPSLKS |
| | 50590 | VH3 | KATWVAFDI |
| | | | |
| VH-CONSENSUS-56 | 50591 | VH1 | SYVMN |
| TABLE 90 | 50592 | VH2 | AISGSGGRTYYADSVKG |
| | 50593 | VH3 | TATFDY |
| | | | |
| VH-CONSENSUS-57 | 50594 | VH1 | SYAMS |
| TABLE 91 | 50595 | VH2 | VISGRGGTTFYADSVKG |
| | 50596 | VH3 | KRTPSDVFDI |
| | | | |
| VH-CONSENSUS-58 | 50597 | VH1 | SYGMH |
| TABLE 92 | 50598 | VH2 | VIWYDGSNKYYADSVKG |
| | 50599 | VH3 | DRPRSSAFDY |
| | | | |
| VH-CONSENSUS-59 | 50600 | VH1 | SYNMN |
| TABLE 93 | 50601 | VH2 | YISRSSNTKYYADSVKG |
| | 50602 | VH3 | DRSGSYGYFYYYGLDV |
| | | | |
| VH-CONSENSUS-60 | 50603 | VH1 | SGGDYWS |

**FIGURE 55**

| TABLE 94 | 50604 | VH2 | YIYYSGSTYYNPSLKS |
|---|---|---|---|
| | 50605 | VH3 | DSSSYGMDV |
| | | | |

FIGURE 55

Table 19C

**VARIABLE HEAVY CDR CONSENSUS SEQUENCES II**

| NAME (ORIGINAL AND PATENT) | Patent SEQ ID NO: | CDR | Sequence |
|---|---|---|---|
| VH-CONSENSUS-15 TABLE 49 | SEQ ID NO: 50001 | VH1 | X1 Tyr Asp X2 Asn, wherein X1 = N or H or a conservative substitution thereof, X2 = I or V or L or a conservative substitution thereof. |
| | SEQ ID NO: 50002 | VH2 | X1 X2 X3 Pro X4 Ser X5 X6 X7 X8 X9 X10 X11 X12 Phe X13 X14 wherein X1 = W or R or a conservative substitution thereof, X2 = M or V or L or a conservative substitution thereof, X3 = H or N or Y or T or a conservative substitution thereof, X4 = N or D or H or a conservative substitution thereof, X5 = G or H or a conservative substitution thereof, X6 = N or S or Q or A or D or K or T or a conservative substitution thereof, X7 = T or A or V or E or a conservative substitution thereof, X8 = G or D or a conservative substitution thereof, X9 = Y or F or C or a conservative substitution thereof, X10 = A or P or a conservative substitution thereof, X11 = Q or K or a conservative substitution thereof, X12 = K or R or N or a conservative substitution thereof, X13 = Q or R or a conservative substitution thereof, X14 = G or V or a conservative substitution thereof. |
| | SEQ ID NO: 50003 | VH3 | Ser Ser Gly Trp X1 X2 Phe Asp X3, wherein X1 = Y or E or N or T or a conservative substitution thereof, X2 = Y or F or K or I or R or V or L or M or W or H or S or a conservative substitution thereof, X3 = Y or F or S or N or a conservative substitution thereof. |
| VH-CONSENSUS-16 TABLE 50 | SEQ ID NO: 50004 | VH1 | X1 X2 X3 X4 X5, wherein X1 = S or D or N or I or a conservative substitution thereof, X2 = Y or C or F or D or S or a conservative substitution thereof, X3 = V or G or I or L or a conservative substitution thereof, X4 = M or I or L or a conservative substitution thereof, X5 = H or D or a conservative substitution thereof. |
| | SEQ ID NO: 50005 | VH2 | X1 Ile X2 Tyr Asp X3 X4 X5 Lys X6 X7 X8 X9 X10 X11 Lys Gly, wherein X1 = V or L or A or a conservative substitution thereof, X2 = W or F or a conservative substitution thereof, X3 = G or A or a conservative substitution thereof, X4 = S or R or N or a conservative substitution thereof, X5 = N or Y or G or S or a conservative substitution thereof, X6 = Y or H or a conservative substitution thereof, X7 = Y or H or N or a conservative substitution thereof, X8 = A or V or E or G or T or a conservative substitution thereof, X9 = D or E or G or a conservative substitution thereof, X10 = S or A or a conservative substitution thereof, X11 = V or M or a conservative substitution thereof. |

FIGURE 55

| | SEQ ID NO: 50006 | VH3 | X1 X2 X3 X4 X5 X6 X7 X8 X9 X10 X11 X12 X13 X14 X15 X16 Gly X17 X18 Val, wherein X1 = E or R or V or a conservative substitution thereof, X2 = R or Y or K or V or E or P or D or L or F or M or N or Q or T or a conservative substitution thereof, X3 = Y or S or T or V or a conservative substitution thereof, X4 = S or R or Y or P or T or G or a conservative substitution thereof, X5 = S or C or Y or a conservative substitution thereof, X6 = G or W or S or N or a conservative substitution thereof, X7 = W or Absent or L or Y or G or F or S or a conservative substitution thereof, X8 = Absent or Y or a conservative substitution thereof, X9 = Absent or A or G or T or a conservative substitution thereof, X10 = Absent or C or a conservative substitution thereof, X11 = Absent or P or a conservative substitution thereof, X12 = Absent or Y or L or a conservative substitution thereof, X13 = Absent or Y or D or a conservative substitution thereof, X14 = Y or Absent or F or H or a conservative substitution thereof, X15 = D or S or G or T or V or Y or A or F or M or a conservative substitution thereof, X16 = Y or G or F or a conservative substitution thereof, X17 = M or L or a conservative substitution thereof, X18 = D or G or a conservative substitution thereof. |
|---|---|---|---|
| VH-CONSENSUS-17 TABLE 51 | SEQ ID NO: 50007 | VH1 | X1 X2 Gly X3 His, wherein X1 = D or S or N or T or a conservative substitution thereof, X2 = Y or F or a conservative substitution thereof, X3 = M or I or a conservative substitution thereof. |
| | SEQ ID NO: 50008 | VH2 | X1 X2 Trp X3 X4 X5 X6 X7 X8 X9 X10 X11 X12 Ser X13 X14 Gly, wherein X1 = V or L or a conservative substitution thereof, X2 = I or V or T or M or a conservative substitution thereof, X3 = Y or F or D or a conservative substitution thereof, X4 = D or E or A or G or N or a conservative substitution thereof, X5 = E or G or V or R or D or a conservative substitution thereof, X6 = N or S or T or D or I or Y or a conservative substitution thereof, X7 = N or H or K or a conservative substitution thereof, X8 = K or Q or E or N or R or a conservative substitution thereof, X9 = Y or H or K or D or R or S or a conservative substitution thereof, X10 = Y or H or a conservative substitution thereof, X11 = A or V or G or T or I or E or a conservative substitution thereof, X12 = D or E or a conservative substitution thereof, X13 = V or M or a conservative substitution thereof, X14 = K or R or a conservative substitution thereof. |
| | SEQ ID NO: 50009 | VH3 | X1 Leu X2 X3 X4 X5 X6 X7, wherein X1 = E or D or G or a conservative substitution thereof, X2 = G or A or a conservative substitution thereof, X3 = F or W or M or a conservative substitution thereof, X4 = L or R or T or Y or S or Q or I or A or E or N or a conservative substitution |

FIGURE 55

| | | | thereof, X5 = S or E or G or D or F or N or A or T or a conservative substitution thereof, X6 = D or E or a conservative substitution thereof, X7 = Y or S or F or C or a conservative substitution thereof. |
|---|---|---|---|
| VH-CONSENSUS-18 TABLE 52 | SEQ ID NO: 50010 | VH1 | Gly X1 Tyr Trp Ser, wherein X1 = C or S or P or Y or a conservative substitution thereof. |
| | SEQ ID NO: 50011 | VH2 | Glu Ile Asn X1 X2 Gly X3 Thr X4 X5 Asn Pro Ser Leu X6 Ser, wherein X1 = H or Y or Q or a conservative substitution thereof, X2 = S or R or a conservative substitution thereof, X3 = S or R or C or I or a conservative substitution thereof, X4 = N or S or a conservative substitution thereof, X5 = Y or F or a conservative substitution thereof, X6 = K or T or a conservative substitution thereof. |
| | SEQ ID NO: 50012 | VH3 | Asp Tyr Gly Gly X1 Asp Val, wherein X1 = M or L or I or a conservative substitution thereof. |
| VH-CONSENSUS-19 TABLE 53 | SEQ ID NO: 50013 | VH1 | Asn Tyr Asp Ile Asn. |
| | SEQ ID NO: 50014 | VH2 | Trp Met X1 Pro X2 X3 X4 X5 X6 Gly X7 Ala Gln Lys Phe Gln X8, wherein X1 = H or N or Y or a conservative substitution thereof, X2 = N or D or a conservative substitution thereof, X3 = S or N or a conservative substitution thereof, X4 = G or V or a conservative substitution thereof, X5 = N or S or a conservative substitution thereof, X6 = T or I or a conservative substitution thereof, X7 = Y or F or C or a conservative substitution thereof, X8 = G or D or a conservative substitution thereof. |
| | SEQ ID NO: 50015 | VH3 | Ser Ser Gly Trp X1 X2 Phe Asp Pro, wherein X1 = Y or N or S or K or a conservative substitution thereof, X2 = W or R or a conservative substitution thereof. |
| VH-CONSENSUS-20 TABLE 54 | SEQ ID NO: 50016 | VH1 | X1 X2 Gly Met X3, wherein X1 = S or N or R or T or I or a conservative substitution thereof, X2 = Y or H or N or a conservative substitution thereof, X3 = H or D or a conservative substitution thereof. |
| | SEQ ID NO: 50017 | VH2 | X1 X2 Trp X3 Asp Gly X4 Asn X5 X6 X7 X8 X9 Ser Val Lys Gly, wherein X1 = V or I or a conservative substitution thereof, X2 = I or L or a conservative substitution thereof, X3 = Y or F or a conservative substitution thereof, X4 = S or T or a conservative substitution thereof, X5 = K or E or Q or D or R or a conservative substitution thereof, X6 = N or H or Y or a conservative substitution thereof, X7 = Y or H or a conservative substitution thereof, X8 = A or V or G |

FIGURE 55

| | | | |
|---|---|---|---|
| | | | or a conservative substitution thereof, X9 = D or E or a conservative substitution thereof. |
| | SEQ ID NO: 50018 | VH3 | X1 X2 Gly X3 X4 X5 X6 X7 X8 X9 X10 X11 X12 X13 Asp Val, wherein X1 = D or A or a conservative substitution thereof, X2 = Q or R or Y or H or A or C or E or F or M or a conservative substitution thereof, X3 = V or I or F or a conservative substitution thereof, X4 = G or Y or a conservative substitution thereof, X5 = Y or E or a conservative substitution thereof, X6 = Absent or F or a conservative substitution thereof, X7 = Absent or D or a conservative substitution thereof, X8 = Absent or Y or a conservative substitution thereof, X9 = Absent or Y or a conservative substitution thereof, X10 = Absent or N or a conservative substitution thereof, X11 = Y or D or N or a conservative substitution thereof, X12 = G or A or D or a conservative substitution thereof, X13 = L or M or T or I or a conservative substitution thereof. |
| VH-CONSENSUS-21 TABLE 55 | SEQ ID NO: 50019 | VH1 | X1 X2 X3 X4 X5, wherein X1 = G or D or S or A or a conservative substitution thereof, X2 = Y or D or a conservative substitution thereof, X3 = Y or H or N or F or a conservative substitution thereof, X4 = M or L or I or a conservative substitution thereof, X5 = H or Q or a conservative substitution thereof. |
| | SEQ ID NO: 50020 | VH2 | Trp X1 X2 Pro X3 X4 X5 X6 X7 X8 X9 X10 Gln X11 Phe Gln X12, wherein X1 = I or V or a conservative substitution thereof, X2 = N or H or K or S or a conservative substitution thereof, X3 = N or K or a conservative substitution thereof, X4 = S or N or R or T or a conservative substitution thereof, X5 = G or N or D or a conservative substitution thereof, X6 = G or A or a conservative substitution thereof, X7 = T or S or a conservative substitution thereof, X8 = N or H or Q or I or a conservative substitution thereof, X9 = Y or S or F or a conservative substitution thereof, X10 = A or T or a conservative substitution thereof, X11 = K or R or N or E or S or a conservative substitution thereof, X12 = G or D or a conservative substitution thereof. |
| | SEQ ID NO: 50021 | VH3 | X1 X2 X3 X4 X5 X6 X7 X8 X9 X10 X11 X12 X13, wherein X1 = D or K or G or S or E or a conservative substitution thereof, X2 = G or F or A or K or V or a conservative substitution thereof, X3 = T or Absent or P or a conservative substitution thereof, X4 = S or G or Absent or T or a conservative substitution thereof, X5 = Absent or V or S or a conservative substitution thereof, X6 = Absent or A or a conservative substitution thereof, X7 = Absent or T or a conservative substitution thereof, X8 = Absent or W or a conservative substitution thereof, X9 = Absent or G or a conservative substitution thereof, X10 = S or Absent or V or Y or a conservative substitution thereof, X11 = F or |

FIGURE 55

| | | | Absent or L or Y or a conservative substitution thereof, X12 = D or G or K or a conservative substitution thereof, X13 = Y or D or F or a conservative substitution thereof. |
|---|---|---|---|
| VH-CONSENSUS-22 TABLE 56 | SEQ ID NO: 50022 | VH1 | X1 X2 Gly X3 His, wherein X1 = S or N or a conservative substitution thereof, X2 = Y or F or H or a conservative substitution thereof, X3 = M or I or a conservative substitution thereof. |
| | SEQ ID NO: 50023 | VH2 | X1 Ile X2 X3 X4 X5 X6 X7 X8 X9 X10 X11 X12 X13 Val X14 Gly, wherein X1 = V or I or a conservative substitution thereof, X2 = W or I or a conservative substitution thereof, X3 = H or Y or F or N or a conservative substitution thereof, X4 = D or S or E or a conservative substitution thereof, X5 = G or A or a conservative substitution thereof, X6 = S or G or a conservative substitution thereof, X7 = N or Y or a conservative substitution thereof, X8 = K or D or E or R or a conservative substitution thereof, X9 = Y or N or a conservative substitution thereof, X10 = Y or N or a conservative substitution thereof, X11 = A or V or G or a conservative substitution thereof, X12 = D or E or a conservative substitution thereof, X13 = S or A or a conservative substitution thereof, X14 = K or R or a conservative substitution thereof. |
| | SEQ ID NO: 50024 | VH3 | X1 X2 X3 X4 X5 X6 X7 X8 X9 X10 X11 X12 X13 X14 X15 X16 X17 Gly X18 Asp Val, wherein X1 = D or T or R or a conservative substitution thereof, X2 = L or R or Y or S or F or I or P or a conservative substitution thereof, X3 = S or R or T or a conservative substitution thereof, X4 = M or V or G or P or K or N or Y or a conservative substitution thereof, X5 = G or Y or Absent or S or a conservative substitution thereof, X6 = Absent or Y or S or W or a conservative substitution thereof, X7 = Absent or G or a conservative substitution thereof, X8 = Absent or S or a conservative substitution thereof, X9 = Absent or G or a conservative substitution thereof, X10 = Absent or S or a conservative substitution thereof, X11 = Absent or P or a conservative substitution thereof, X12 = Absent or P or a conservative substitution thereof, X13 = Absent or Y or a conservative substitution thereof, X14 = Absent or Y or a conservative substitution thereof, X15 = Absent or Y or a conservative substitution thereof, X16 = Absent or S or Y or a conservative substitution thereof, X17 = Absent or D or Y or G or a conservative substitution thereof, X18 = M or L or T or a conservative substitution thereof. |
| VH-CONSENSUS-23 TABLE 57 | SEQ ID NO: 50025 | VH1 | X1 X2 Gly X3 His, wherein X1 = D or N or S or a conservative substitution thereof, X2 = Y or F or a conservative substitution thereof, X3 = M or I or L or a conservative substitution thereof. |

FIGURE 55

| | SEQ ID NO: 50026 | VH2 | Val Ile Trp X1 X2 X3 X4 Asn X5 X6 Tyr X7 X8 Ser X9 Lys Gly, wherein X1 = Y or F or a conservative substitution thereof, X2 = D or E or I or V or a conservative substitution thereof, X3 = E or G or V or A or R or a conservative substitution thereof, X4 = S or N or T or G or a conservative substitution thereof, X5 = K or Q or T or N or a conservative substitution thereof, X6 = Y or H or K or a conservative substitution thereof, X7 = A or T or G or E or V or a conservative substitution thereof, X8 = D or G or a conservative substitution thereof, X9 = V or A or a conservative substitution thereof. |
|---|---|---|---|
| | SEQ ID NO: 50027 | VH3 | Glu X1 Gly X2 X3 X4 Asp X5, wherein X1 = V or I or M or K or T or a conservative substitution thereof, X2 = W or F or G or M or a conservative substitution thereof, X3 = L or T or S or Y or H or R or a conservative substitution thereof, X4 = D or E or S or F or N or a conservative substitution thereof, X5 = Y or C or a conservative substitution thereof. |
| VH-CONSENSUS-24 TABLE 58 | SEQ ID NO: 50028 | VH1 | X1 X2 X3 X4 Asn, wherein X1 = S or T or a conservative substitution thereof, X2 = Y or F or a conservative substitution thereof, X3 = S or T or G or R or a conservative substitution thereof, X4 = M or L or a conservative substitution thereof. |
| | SEQ ID NO: 50029 | VH2 | X1 Ile Ser X2 Ser X3 X4 X5 X6 X7 Tyr X8 Asp Ser X9 Lys X10, wherein X1 = S or A or C or L or a conservative substitution thereof, X2 = G or S or a conservative substitution thereof, X3 = S or G or T or a conservative substitution thereof, X4 = S or T or G or Y or a conservative substitution thereof, X5 = Y or H or a conservative substitution thereof, X6 = I or L or M or a conservative substitution thereof, X7 = Y or S or W or a conservative substitution thereof, X8 = A or G or P or V or a conservative substitution thereof, X9 = V or L or a conservative substitution thereof, X10 = G or A or a conservative substitution thereof. |
| | SEQ ID NO: 50030 | VH3 | X1 Arg X2 X3 X4 X5 X6 X7, wherein X1 = D or T or a conservative substitution thereof, X2 = G or Absent or S or Y or a conservative substitution thereof, X3 = Absent or G or a conservative substitution thereof, X4 = Absent or S or a conservative substitution thereof, X5 = Absent or F or S or a conservative substitution thereof, X6 = S or D or G or H or a conservative substitution thereof, X7 = S or L or Y or G or T or C or E or I or a conservative substitution thereof. |
| VH-CONSENSUS-25 TABLE 59 | SEQ ID NO: 50031 | VH1 | X1 Tyr X2 Met X3, wherein X1 = S or G or a conservative substitution thereof, X2 = A or V or a conservative substitution thereof, X3 = S or N or T or a conservative substitution thereof. |

FIGURE 55

| | SEQ ID NO: 50032 | VH2 | X1 Ile Ser X2 X3 Gly X4 X5 X6 X7 X8 Ala Asp Ser Val X9 Gly, wherein X1 = V or I or A or a conservative substitution thereof, X2 = G or R or a conservative substitution thereof, X3 = R or S or a conservative substitution thereof, X4 = G or V or I or T or a conservative substitution thereof, X5 = N or Y or S or T or a conservative substitution thereof, X6 = T or A or a conservative substitution thereof, X7 = F or Y or a conservative substitution thereof, X8 = Y or N or S or a conservative substitution thereof, X9 = K or R or a conservative substitution thereof. |
| --- | --- | --- | --- |
| | SEQ ID NO: 50033 | VH3 | Arg X1 Ala Val X2 Gly X3 X4 Ala X5 X6 X7, wherein X1 = I or L or M or V or a conservative substitution thereof, X2 = A or D or a conservative substitution thereof, X3 = S or N or Y or a conservative substitution thereof, X4 = E or D or a conservative substitution thereof, X5 = F or C or a conservative substitution thereof, X6 = D or A or H or a conservative substitution thereof, X7 = I or V or a conservative substitution thereof. |
| VH-CONSENSUS-26 TABLE 60 | SEQ ID NO: 50034 | VH1 | X1 X2 X3 Met X4, wherein X1 = S or N or a conservative substitution thereof, X2 = Y or C or a conservative substitution thereof, X3 = A or V or a conservative substitution thereof, X4 = S or N or a conservative substitution thereof. |
| | SEQ ID NO: 50035 | VH2 | X1 X2 Ser X3 X4 Gly Gly X5 X6 X7 X8 Ala Asp Ser X9 Lys Gly, wherein X1 = A or T or S or a conservative substitution thereof, X2 = I or L or a conservative substitution thereof, X3 = G or R or V or a conservative substitution thereof, X4 = R or G or a conservative substitution thereof, X5 = S or N or a conservative substitution thereof, X6 = T or I or a conservative substitution thereof, X7 = F or Y or a conservative substitution thereof, X8 = H or Y or N or a conservative substitution thereof, X9 = V or E or M or a conservative substitution thereof. |
| | SEQ ID NO: 50036 | VH3 | X1 X2 Leu Leu X3 X4 Tyr X5 X6 X7 X8 X9 X10 Asp Val, wherein X1 = G or W or a conservative substitution thereof, X2 = E or G or a conservative substitution thereof, X3 = E or Y or a conservative substitution thereof, X4 = D or S or N or a conservative substitution thereof, X5 = Absent or E or a conservative substitution thereof, X6 = Y or S or a conservative substitution thereof, X7 = F or Y or a conservative substitution thereof, X8 = Y or F or a conservative substitution thereof, X9 = G or A or a conservative substitution thereof, X10 = M or I or L or a conservative substitution thereof. |

FIGURE 55

| VH-CONSENSUS-27 TABLE 61 | SEQ ID NO: 50037 | VH1 | X1 Tyr X2 X3 X4, wherein X1 = S or G or D or R or H or T or N or a conservative substitution thereof, X2 = G or V or a conservative substitution thereof, X3 = M or L or a conservative substitution thereof, X4 = H or N or a conservative substitution thereof. |
|---|---|---|---|
| | SEQ ID NO: 50038 | VH2 | X1 X2 X3 X4 Asp Gly X5 X6 X7 X8 X9 X10 Asp Ser Val Lys X11, wherein X1 = V or L or F or I or D or a conservative substitution thereof, X2 = I or F or a conservative substitution thereof, X3 = W or R or a conservative substitution thereof, X4 = Y or F or a conservative substitution thereof, X5 = S or N or T or a conservative substitution thereof, X6 = N or E or D or K or a conservative substitution thereof, X7 = K or N or T or a conservative substitution thereof, X8 = Y or S or D or N or a conservative substitution thereof, X9 = Y or N or a conservative substitution thereof, X10 = A or V or a conservative substitution thereof, X11 = G or D or a conservative substitution thereof. |
| | SEQ ID NO: 50039 | VH3 | Asp X1 X2 X3 X4 X5 X6 X7 X8 X9 X10 X11 X12 X13 X14 X15 Tyr X16 X17 Asp Val, wherein X1 = R or D or W or N or a conservative substitution thereof, X2 = V or D or R or F or H or a conservative substitution thereof, X3 = Y or S or E or G or F or a conservative substitution thereof, X4 = C or G or E or a conservative substitution thereof, X5 = S or G or a conservative substitution thereof, X6 = S or G or R or N or a conservative substitution thereof, X7 = T or Absent or S or P or a conservative substitution thereof, X8 = S or P or Absent or T or a conservative substitution thereof, X9 = C or Absent or a conservative substitution thereof, X10 = Absent or S or H or L or Y or a conservative substitution thereof, X11 = P or Absent or S or Y or a conservative substitution thereof, X12 = Y or Absent or a conservative substitution thereof, X13 = Y or Absent or a conservative substitution thereof, X14 = Y or Absent or a conservative substitution thereof, X15 = Y or F or a conservative substitution thereof, X16 = G or A or a conservative substitution thereof, X17 = M or L or a conservative substitution thereof. |
| VH-CONSENSUS-28 TABLE 62 | SEQ ID NO: 50040 | VH1 | X1 Tyr X2 X3 Asn, wherein X1 = S or N or a conservative substitution thereof, X2 = S or T or K or N or R or a conservative substitution thereof, X3 = M or L or a conservative substitution thereof. |
| | SEQ ID NO: 50041 | VH2 | Ser X1 Ser X2 X3 X4 X5 X6 X7 X8 Tyr X9 Asp Ser Val Lys Gly, wherein X1 = I or T or a conservative substitution thereof, X2 = G or S or a conservative substitution thereof, X3 = S or G or N or T or a conservative substitution thereof, X4 = S or G or D or N or a conservative substitution thereof, X5 = S or T or a conservative substitution thereof, X6 = Y or F or D or L or N or S or a |

FIGURE 55

| | | | |
|---|---|---|---|
| | | | conservative substitution thereof, X7 = I or M or T or a conservative substitution thereof, X8 = Y or N or a conservative substitution thereof, X9 = A or T or a conservative substitution thereof. |
| | SEQ ID NO: 50042 | VH3 | Val X1 X2 X3 Asp X4, wherein X1 = A or N or S or a conservative substitution thereof, X2 = S or A or G or T or L or H or N or a conservative substitution thereof, X3 = F or L or N or a conservative substitution thereof, X4 = Y or C or S or a conservative substitution thereof. |
| VH-CONSENSUS-29 TABLE 63 | SEQ ID NO: 50230 | VH1 | Ser X1 X2 Tyr X3 Trp X4, wherein X1 = G or V or a conservative substitution thereof, X2 = D or V or G or S or a conservative substitution thereof, X3 = Y or H or a conservative substitution thereof, X4 = N or S or a conservative substitution thereof. |
| | SEQ ID NO: 50231 | VH2 | X1 X2 X3 X4 Ser Gly Ser Thr Tyr X5 Asn Pro Ser Leu X6 Ser, wherein X1 = Y or N or F or a conservative substitution thereof, X2 = I or L or a conservative substitution thereof, X3 = F or Y or H or a conservative substitution thereof, X4 = Y or H or a conservative substitution thereof, X5 = Y or N or a conservative substitution thereof, X6 = K or R or a conservative substitution thereof. |
| | SEQ ID NO: 50232 | VH3 | Gly Asp Tyr Asp Gly Ser Gly Ser Tyr His X1 Tyr X2 Gly X3 Asp Val, wherein X1 = Y or F or H or a conservative substitution thereof, X2 = Y or H or a conservative substitution thereof, X3 = M or L or a conservative substitution thereof. |
| VH-CONSENSUS-30 TABLE 64 | SEQ ID NO: 50043 | VH1 | X1 X2 X3 Met Ser, wherein X1 = S or N or T or a conservative substitution thereof, X2 = Y or H or a conservative substitution thereof, X3 = A or P or V or a conservative substitution thereof. |
| | SEQ ID NO: 50044 | VH2 | X1 Ile Ser Gly X2 Gly X3 X4 X5 Tyr Tyr Ala Asp Ser Val Lys Gly, wherein X1 = V or A or I or a conservative substitution thereof, X2 = G or S or a conservative substitution thereof, X3 = S or G or T or a conservative substitution thereof, X4 = S or T or a conservative substitution thereof, X5 = T or A or a conservative substitution thereof. |
| | SEQ ID NO: 50045 | VH3 | X1 X2 Gly X3 X4 X5 X6 X7 X8 X9 X10 Gly Met Asp, wherein X1 = W or A or a conservative substitution thereof, X2 = R or G or a conservative substitution thereof, X3 = N or T or a conservative substitution thereof, X4 = P or T or a conservative substitution thereof, X5 = T or G or a conservative substitution thereof, X6 = Absent or S or a conservative substitution thereof, X7 = Absent or Y or a conservative substitution thereof, X8 = Absent or Y or a conservative substitution thereof, X9 = D or Y or a |

FIGURE 55

| | | | |
|---|---|---|---|
| | | | conservative substitution thereof, X10 = Y or N or S or a conservative substitution thereof. |
| VH-CONSENSUS-31 TABLE 65 | SEQ ID NO: 50253 | VH1 | X1 Tyr Gly X2 His, wherein X1 = S or N or T or a conservative substitution thereof, X2 = M or L or a conservative substitution thereof. |
| | SEQ ID NO: 50254 | VH2 | X1 X2 Trp Tyr Asp Gly X3 X4 Lys Tyr Tyr X5 Asp Ser Val Lys Gly, wherein X1 = I or V or A or a conservative substitution thereof, X2 = I or M or a conservative substitution thereof, X3 = S or T or a conservative substitution thereof, X4 = N or Y or S or a conservative substitution thereof, X5 = A or G or T or V or a conservative substitution thereof. |
| | SEQ ID NO: 50255 | VH3 | X1 X2 X3 X4 X5 X6 X7 X8 X9 X10 X11 X12, wherein X1 = D or E or a conservative substitution thereof, X2 = H or R or Q or A or G or T or Y or a conservative substitution thereof, X3 = Y or G or F or H or a conservative substitution thereof, X4 = D or I or F or a conservative substitution thereof, X5 = F or V or L or a conservative substitution thereof, X6 = W or G or Absent or a conservative substitution thereof, X7 = S or A or Absent or a conservative substitution thereof, X8 = G or T or E or a conservative substitution thereof, X9 = H or Y or W or F or a conservative substitution thereof, X10 = F or L or S or a conservative substitution thereof, X11 = D or A or C or G or a conservative substitution thereof, X12 = Y or F or S or a conservative substitution thereof. |
| VH-CONSENSUS-32 TABLE 66 | SEQ ID NO: 50233 | VH1 | X1 X2 X3 Trp Ser () wherein X1 = G or V or P or A or D or Y or a conservative substitution thereof, X2 = Y or C or S or P or a conservative substitution thereof, X3 = Y or F or a conservative substitution thereof. |
| | SEQ ID NO: 50234 | VH2 | Glu X1 Asn X2 Ser Gly X3 X4 X5 X6 Asn Pro Ser Leu Lys Ser, wherein X1 = I or S or V or a conservative substitution thereof, X2 = H or I or Q or a conservative substitution thereof, X3 = R or S or a conservative substitution thereof, X4 = T or A or S or a conservative substitution thereof, X5 = N or T or a conservative substitution thereof, X6 = Y or F or a conservative substitution thereof. |
| | SEQ ID NO: 50235 | VH3 | Asp Tyr Gly Gly Leu Asp Tyr. |
| VH-CONSENSUS-33 TABLE 67 | SEQ ID NO: 50046 | VH1 | X1 Ser X2 X3 Tyr Trp Gly, wherein X1 = R or G or a conservative substitution thereof, X2 = S or N or a conservative substitution thereof, X3 = Y or S or a conservative substitution thereof. |
| | SEQ ID NO: 50047 | VH2 | X1 Ile Tyr Tyr Ser Gly X2 X3 X4 X5 X6 Pro Ser Leu Lys Ser, wherein X1 = N or S or a conservative substitution thereof, X2 = S or Y or A or T or I or a conservative |

FIGURE 55

| | | | |
|---|---|---|---|
| | | | substitution thereof, X3 = T or A or P or S or a conservative substitution thereof, X4 = Y or Q or S or A or N or a conservative substitution thereof, X5 = Y or C or H or a conservative substitution thereof, X6 = N or I or T or a conservative substitution thereof. |
| | SEQ ID NO: 50048 | VH3 | X1 Ser X2 Ser Trp Ser X3 Asp X4, wherein X1 = H or L or a conservative substitution thereof, X2 = S or T or G or a conservative substitution thereof, X3 = L or F or I or V or a conservative substitution thereof, X4 = Y or N or C or D or F or a conservative substitution thereof. |
| VH-CONSENSUS-34 TABLE 68 | SEQ ID NO: 50049 | VH1 | X1 X2 X3 X4 X5, wherein X1 = G or D or a conservative substitution thereof, X2 = Y or H or a conservative substitution thereof, X3 = Y or F or a conservative substitution thereof, X4 = I or M or a conservative substitution thereof, X5 = H or N or a conservative substitution thereof. |
| | SEQ ID NO: 50050 | VH2 | Trp Ile Asn X1 X2 X3 X4 X5 Thr Asn Tyr X6 X7 Lys Phe Gln X8, wherein X1 = P or S or a conservative substitution thereof, X2 = N or K or a conservative substitution thereof, X3 = S or N or a conservative substitution thereof, X4 = G or D or a conservative substitution thereof, X5 = G or D or a conservative substitution thereof, X6 = A or E or a conservative substitution thereof, X7 = Q or E or a conservative substitution thereof, X8 = G or D or a conservative substitution thereof. |
| | SEQ ID NO: 50051 | VH3 | X1 X2 X3 X4 X5 X6 X7 X8 X9 X10 X11 X12 X13 X14 X15, wherein X1 = G or D or a conservative substitution thereof, X2 = Y or G or T or F or a conservative substitution thereof, X3 = S or Y or D or R or a conservative substitution thereof, X4 = Y or S or a conservative substitution thereof, X5 = G or Absent or S or a conservative substitution thereof, X6 = Y or S or Absent or a conservative substitution thereof, X7 = Absent or G or a conservative substitution thereof, X8 = Absent or S or a conservative substitution thereof, X9 = Absent or Y or a conservative substitution thereof, X10 = Absent or Y or F or H or a conservative substitution thereof, X11 = N or Absent or D or a conservative substitution thereof, X12 = W or Absent or D or E or a conservative substitution thereof, X13 = F or L or a conservative substitution thereof, X14 = D or A or a conservative substitution thereof, X15 = P or S or a conservative substitution thereof. |
| VH-CONSENSUS-35 TABLE 69 | SEQ ID NO: 50052 | VH1 | X1 X2 Gly Met His, wherein X1 = H or N or a conservative substitution thereof, X2 = Y or S or a conservative substitution thereof. |
| | SEQ ID NO: 50053 | VH2 | X1 Ile X2 Tyr Asp Gly Ser X3 X4 X5 Tyr Ala Asp Ser Val Lys Gly, wherein X1 = V or I or a conservative substitution thereof, X2 = W or Y or a conservative substitution thereof, |

FIGURE 55

| | | | |
|---|---|---|---|
| | | | X3 = N or Y or a conservative substitution thereof, X4 = K or E or a conservative substitution thereof, X5 = Y or C or N or a conservative substitution thereof. |
| | SEQ ID NO: 50054 | VH3 | X1 X2 X3 X4 X5 X6 X7 X8 X9 X10 X11 X12 X13 X14 Gly X15 Asp Val, wherein X1 = G or D or a conservative substitution thereof, X2 = D or R or a conservative substitution thereof, X3 = W or H or a conservative substitution thereof, X4 = N or Y or a conservative substitution thereof, X5 = P or D or a conservative substitution thereof, X6 = Absent or F or a conservative substitution thereof, X7 = Absent or H or a conservative substitution thereof, X8 = Absent or V or a conservative substitution thereof, X9 = Absent or P or a conservative substitution thereof, X10 = Absent or Y or a conservative substitution thereof, X11 = Absent or Y or a conservative substitution thereof, X12 = Absent or Y or a conservative substitution thereof, X13 = Absent or Y or a conservative substitution thereof, X14 = E or Y or a conservative substitution thereof, X15 = M or L or a conservative substitution thereof. |
| VH-CONSENSUS-36 TABLE 70 | SEQ ID NO: 50055 | VH1 | Ser X1 Ala Met X2, wherein X1 = Y or S or a conservative substitution thereof, X2 = S or T or N or a conservative substitution thereof. |
| | SEQ ID NO: 50056 | VH2 | X1 Ile X2 Gly X3 Gly X4 X5 X6 X7 Tyr Ala Asp Ser Val Lys Gly, wherein X1 = A or V or a conservative substitution thereof, X2 = S or I or a conservative substitution thereof, X3 = S or R or N or F or a conservative substitution thereof, X4 = G or S or a conservative substitution thereof, X5 = N or R or S or a conservative substitution thereof, X6 = T or A or a conservative substitution thereof, X7 = F or Y or a conservative substitution thereof. |
| | SEQ ID NO: 50057 | VH3 | X1 X2 X3 X4 X5 X6 X7 X8 X9 X10 X11 X12 Phe Asp Tyr, wherein X1 = K or D or R or a conservative substitution thereof, X2 = D or Y or H or M or R or a conservative substitution thereof, X3 = Y or G or N or a conservative substitution thereof, X4 = D or I or R or Y or a conservative substitution thereof, X5 = Y or Absent or S or V or a conservative substitution thereof, X6 = V or Absent or G or R or S or a conservative substitution thereof, X7 = W or Absent or I or a conservative substitution thereof, X8 = Absent or A or a conservative substitution thereof, X9 = G or Absent or V or a conservative substitution thereof, X10 = S or Absent or A or T or Y or a conservative substitution thereof, X11 = P or Absent or G or I or a conservative substitution thereof, X12 = Y or F or T or a conservative substitution thereof. |

FIGURE 55

| VH-CONSENSUS-37 TABLE 71 | SEQ ID NO: 50058 | VH1 | X1 X2 X3 X4 His, wherein X1 = G or D or a conservative substitution thereof, X2 = Y or H or a conservative substitution thereof, X3 = Y or C or a conservative substitution thereof, X4 = M or I or a conservative substitution thereof. |
|---|---|---|---|
| | SEQ ID NO: 50059 | VH2 | X1 Ile X2 X3 X4 Ser Gly X5 X6 X7 X8 X9 Gln Lys Phe Gln X10, wherein X1 = W or S or a conservative substitution thereof, X2 = N or Y or a conservative substitution thereof, X3 = P or R or a conservative substitution thereof, X4 = N or K or a conservative substitution thereof, X5 = G or A or a conservative substitution thereof, X6 = T or A or a conservative substitution thereof, X7 = N or D or a conservative substitution thereof, X8 = Y or N or S or a conservative substitution thereof, X9 = A or G or V or a conservative substitution thereof, X10 = G or D or V or a conservative substitution thereof. |
| | SEQ ID NO: 50060 | VH3 | X1 X2 Tyr X3 Gly Ser Gly X4 Tyr X5 Asn X6 Phe Asp Tyr, wherein X1 = S or A or V or T or a conservative substitution thereof, X2 = Y or F or N or a conservative substitution thereof, X3 = Y or H or a conservative substitution thereof, X4 = S or T or a conservative substitution thereof, X5 = Y or H or a conservative substitution thereof, X6 = E or G or D or a conservative substitution thereof. |

| VH-CONSENSUS-38 TABLE 72 | SEQ ID NO: 50061 | VH1 | Gly Tyr Tyr X1 His, wherein X1 = I or T or a conservative substitution thereof. |
|---|---|---|---|
| | SEQ ID NO: 50062 | VH2 | Trp Ile Asn Pro Tyr Ser Gly X1 Thr X2 X3 Ala Gln Lys Phe Gln Gly, wherein X1 = D or G or a conservative substitution thereof, X2 = N or K or a conservative substitution thereof, X3 = Y or S or a conservative substitution thereof. |
| | SEQ ID NO: 50236 | VH3 | Asp Trp Gly Gly Tyr Ser Ser Tyr Tyr X1 Gly Met Asp Val, wherein X1 = Y or F or a conservative substitution thereof. |

| VH-CONSENSUS-39 TABLE 73 | SEQ ID NO: 50063 | VH1 | X1 X2 X3 Met X4, wherein X1 = S or T or a conservative substitution thereof, X2 = Y or F or a conservative substitution thereof, X3 = S or T or N or G or a conservative substitution thereof, X4 = N or S or I or a conservative substitution thereof. |
|---|---|---|---|
| | SEQ ID NO: 50064 | VH2 | Ser Ile Ser X1 X2 X3 X4 Tyr X5 X6 Tyr X7 Asp Ser Val Lys Gly, wherein X1 = G or S or a conservative substitution thereof, X2 = S or T or a conservative substitution thereof, X3 = S or I or N or a conservative substitution thereof, X4 = S or N or T or Y or a conservative substitution thereof, X5 |

FIGURE 55

| | | | |
|---|---|---|---|
| | | | = I or M or S or a conservative substitution thereof, X6 = Y or N or a conservative substitution thereof, X7 = A or T or a conservative substitution thereof. |
| | SEQ ID NO: 50065 | VH3 | Leu Thr Phe Asp Tyr. |
| VH-CONSENSUS-40 TABLE 74 | SEQ ID NO: 50066 | VH1 | X1 Tyr Gly Met His, wherein X1 = T or S or a conservative substitution thereof. |
| | SEQ ID NO: 50067 | VH2 | X1 Ile Trp Tyr Asp Gly X2 Asn Lys Tyr Tyr Ala Asp Ser Val X3 Gly, wherein X1 = I or V or a conservative substitution thereof, X2 = T or S or a conservative substitution thereof, X3 = K or R or a conservative substitution thereof. |
| | SEQ ID NO: 50068 | VH3 | Asp Pro Leu Arg Gly Tyr Asn Asp Pro Val X1 Asp Tyr, wherein X1 = M or L or a conservative substitution thereof. |
| VH-CONSENSUS-41 TABLE 75 | SEQ ID NO: 50069 | VH1 | X1 Tyr Ala Met X2, wherein X1 = S or N or a conservative substitution thereof, X2 = S or N or T or a conservative substitution thereof. |
| | SEQ ID NO: 50070 | VH2 | Ala Ile Ser Gly X1 Gly X2 Asn Thr Phe Tyr Ala Asp Ser Val Lys Gly, wherein X1 = R or S or a conservative substitution thereof, X2 = G or K or a conservative substitution thereof. |
| | SEQ ID NO: 50071 | VH3 | Arg Val Thr Asp Tyr Gly Gly Asn Asp Trp Phe Asp Pro. |
| VH-CONSENSUS-42 TABLE 76 | SEQ ID NO: 50072 | VH1 | X1 Tyr Gly Met His, wherein X1 = T or S or a conservative substitution thereof. |
| | SEQ ID NO: 50073 | VH2 | Val X1 Trp Tyr X2 Gly X3 X4 X5 X6 X7 X8 Asp Ser Val X9 Gly, wherein X1 = I or V or a conservative substitution thereof, X2 = G or D or a conservative substitution thereof, X3 = S or N or a conservative substitution thereof, X4 = N or D or S or a conservative substitution thereof, X5 = K or T or a conservative substitution thereof, X6 = D or Y or S or a conservative substitution thereof, X7 = Y or F or a conservative substitution thereof, X8 = A or V or a conservative substitution thereof, X9 = K or R or T or a conservative substitution thereof. |
| | SEQ ID NO: 50074 | VH3 | Asp Arg X1 X2 Cys Ser Gly X3 X4 Cys Pro Tyr Tyr Tyr Tyr Tyr Gly Met Asp Val, wherein X1 = D or V or a conservative substitution thereof, X2 = Y or F or a conservative substitution thereof, X3 = G or T or a conservative substitution thereof, X4 = S or T or N or a conservative substitution thereof. |

FIGURE 55

| VH-CONSENSUS-43 TABLE 77 | SEQ ID NO: 50075 | VH1 | Arg Ser Ser Tyr Tyr Trp Gly. |
|---|---|---|---|
| | SEQ ID NO: 50076 | VH2 | Asn Ile Tyr Tyr X1 Gly X2 X3 Tyr X4 Asn Pro Ser X5 Lys X6, wherein X1 = S or G or a conservative substitution thereof, X2 = S or T or N or a conservative substitution thereof, X3 = T or A or a conservative substitution thereof, X4 = Y or N or D or H or T or a conservative substitution thereof, X5 = L or V or a conservative substitution thereof, X6 = S or G or a conservative substitution thereof. |
| | SEQ ID NO: 50077 | VH3 | His Gly Lys Asp Trp Gly Leu Asp X1, wherein X1 = Y or F or N or a conservative substitution thereof. |
| VH-CONSENSUS-44 TABLE 78 | SEQ ID NO: 50078 | VH1 | Gly Tyr Tyr X1 His, wherein X1 = M or I or a conservative substitution thereof. |
| | SEQ ID NO: 50079 | VH2 | Trp Ile X1 Pro X2 Ser Gly Gly Thr Asn X3 X4 Gln Lys Phe Gln Gly, wherein X1 = K or N or a conservative substitution thereof, X2 = N or K or a conservative substitution thereof, X3 = Q or S or H or N or Y or a conservative substitution thereof, X4 = A or V or a conservative substitution thereof. |
| | SEQ ID NO: 50080 | VH3 | Ala Pro Gly X1 X2 X3 X4 Gly X5 Trp Gly X6 Phe Asp Tyr, wherein X1 = T or K or I or a conservative substitution thereof, X2 = A or V or a conservative substitution thereof, X3 = A or P or a conservative substitution thereof, X4 = A or T or a conservative substitution thereof, X5 = T or S or a conservative substitution thereof, X6 = Y or F or C or a conservative substitution thereof. |
| VH-CONSENSUS-45 TABLE 79 | SEQ ID NO: 50081 | VH1 | Thr X1 Gly Val Gly Val Gly, wherein X1 = G or S or a conservative substitution thereof. |
| | SEQ ID NO: 50082 | VH2 | X1 Ile Tyr Trp X2 Asp Asp X3 Arg Tyr Ser Pro Ser Leu X4 Ser, wherein X1 = L or F or a conservative substitution thereof, X2 = D or H or N or K or S or a conservative substitution thereof, X3 = K or E or a conservative substitution thereof, X4 = K or R or a conservative substitution thereof. |
| | SEQ ID NO: 50083 | VH3 | X1 X2 Ala Val X3 X4 Asp Tyr, wherein X1 = L or I or A or a conservative substitution thereof, X2 = I or V or A or a conservative substitution thereof, X3 = A or S or a conservative substitution thereof, X4 = F or C or a conservative substitution thereof. |
| VH-CONSENSUS-46 TABLE 80 | SEQ ID NO: 50084 | VH1 | X1 Tyr Gly Met His () wherein X1 = S or N or T or a conservative substitution thereof. |

FIGURE 55

| | | | |
|---|---|---|---|
| | SEQ ID NO: 50085 | VH2 | X1 Ile Trp Tyr Asp Gly Ser X2 Lys Tyr Tyr X3 Asp Ser Val Lys Gly, wherein X1 = I or V or a conservative substitution thereof, X2 = Y or N or a conservative substitution thereof, X3 = A or V or a conservative substitution thereof. |
| | SEQ ID NO: 50086 | VH3 | Glu X1 Tyr Asp Phe Trp Ser Gly X2 X3 X4 X5, wherein X1 = A or G or R or N or a conservative substitution thereof, X2 = Y or F or H or a conservative substitution thereof, X3 = F or L or Y or W or a conservative substitution thereof, X4 = D or G or a conservative substitution thereof, X5 = Y or S or a conservative substitution thereof. |
| VH-CONSENSUS-47 TABLE 81 | SEQ ID NO: 50087 | VH1 | Ser Tyr Gly X1 His, wherein X1 = M or L or a conservative substitution thereof. |
| | SEQ ID NO: 50088 | VH2 | Val Ile Trp Tyr Asp Gly Ser Asn Lys X1 Tyr X2 Asp Ser Val Lys Gly, wherein X1 = Y or N or a conservative substitution thereof, X2 = A or E or a conservative substitution thereof. |
| | SEQ ID NO: 50089 | VH3 | X1 X2 X3 Tyr X4 X5 X6 X7 X8 X9 X10 X11 X12 Gly Met Asp Val, wherein X1 = D or W or a conservative substitution thereof, X2 = R or G or Y or a conservative substitution thereof, X3 = D or S or Y or a conservative substitution thereof, X4 = G or Y or a conservative substitution thereof, X5 = D or Y or a conservative substitution thereof, X6 = Absent or P or a conservative substitution thereof, X7 = Absent or P or a conservative substitution thereof, X8 = Absent or Y or a conservative substitution thereof, X9 = Absent or Y or a conservative substitution thereof, X10 = Absent or Y or a conservative substitution thereof, X11 = Absent or Y or a conservative substitution thereof, X12 = Y or D or a conservative substitution thereof. |
| VH-CONSENSUS-48 TABLE 82 | | VH1 | X1 X2 X3 Met X4 (SEQ ID NO: 50090) wherein X1 = S or N or T or a conservative substitution thereof, X2 = Y or S or a conservative substitution thereof, X3 = A or V or a conservative substitution thereof, X4 = S or T or N or a conservative substitution thereof. |
| | | VH2 | X1 X2 X3 Gly X4 Gly X5 X6 Thr X7 X8 X9 Asp Ser Val X10 Gly (SEQ ID NO: 50091) wherein X1 = A or G or a conservative substitution thereof, X2 = I or S or V or a conservative substitution thereof, X3 = S or V or a conservative substitution thereof, X4 = S or R or a conservative substitution thereof, X5 = G or A or S or V or a conservative substitution thereof, X6 = N or R or K or a conservative substitution thereof, X7 = F or Y or a conservative substitution thereof, X8 = Y or N or a |

FIGURE 55

| | | | |
|---|---|---|---|
| | | | conservative substitution thereof, X9 = A or T or a conservative substitution thereof, X10 = K or T or a conservative substitution thereof. |
| | | VH3 | X1 X2 X3 X4 X5 X6 X7 X8 Gly X9 Asp Val (SEQ ID NO: 50092) wherein X1 = L or D or E or a conservative substitution thereof, X2 = G or R or a conservative substitution thereof, X3 = K or G or I or a conservative substitution thereof, X4 = D or Q or Y or a conservative substitution thereof, X5 = Y or W or a conservative substitution thereof, X6 = Y or H or L or a conservative substitution thereof, X7 = Y or Absent or I or L or a conservative substitution thereof, X8 = Y or G or a conservative substitution thereof, X9 = M or V or a conservative substitution thereof. |
| VH-CONSENSUS-49 TABLE 83 | | VH1 | Ser Tyr X1 Met X2 (SEQ ID NO: 50093) wherein X1 = V or A or a conservative substitution thereof, X2 = S or N or a conservative substitution thereof. |
| | | VH2 | X1 X2 Ser Gly Ser Gly X3 X4 Thr X5 Tyr Ala Asp Ser Val X6 X7 (SEQ ID NO: 50094) wherein X1 = A or G or T or S or a conservative substitution thereof, X2 = M or I or T or a conservative substitution thereof, X3 = G or N or V or a conservative substitution thereof, X4 = R or N or W or a conservative substitution thereof, X5 = Y or F or N or a conservative substitution thereof, X6 = K or N or a conservative substitution thereof, X7 = G or D or a conservative substitution thereof. |
| | | VH3 | X1 X2 X3 X4 X5 X6 X7 X8 X9 X10 X11 X12 (SEQ ID NO: 50095) wherein X1 = L or V or Y or F or a conservative substitution thereof, X2 = T or E or F or G or a conservative substitution thereof, X3 = A or G or L or W or Absent or F or a conservative substitution thereof, X4 = Absent or G or M or a conservative substitution thereof, X5 = Absent or V or a conservative substitution thereof, X6 = Absent or G or a conservative substitution thereof, X7 = Absent or A or a conservative substitution thereof, X8 = Absent or G or a conservative substitution thereof, X9 = Absent or I or F or a conservative substitution thereof, X10 = F or N or Absent or a conservative substitution thereof, X11 = D or G or I or a conservative substitution thereof, X12 = Y or D or a conservative substitution thereof. |
| VH-CONSENSUS-50 TABLE 84 | | VH1 | X1 Tyr Gly X2 His (SEQ ID NO: 50096) wherein X1 = S or Y or a conservative substitution thereof, X2 = M or L or a conservative substitution thereof. |
| | | VH2 | X1 Ile Ser Tyr X2 Gly X3 Asn X4 X5 X6 Ala Asp Ser Val Lys Gly (SEQ ID NO: 50097) wherein X1 = I or V or a conservative substitution thereof, X2 = A or G or D or S or V or a conservative substitution thereof, X3 = S or I or N or R or T or a conservative substitution thereof, X4 = K or N |

EP 4 435 105 A2

FIGURE 55

| | | | or Q or a conservative substitution thereof, X5 = Y or S or D or H or a conservative substitution thereof, X6 = Y or S or a conservative substitution thereof. |
|---|---|---|---|
| | | VH3 | X1 X2 X3 X4 X5 X6 X7 X8 X9 X10 X11 X12 X13 X14 X15 Tyr Gly Met Asp Val (SEQ ID NO: 50098) wherein X1 = R or E or a conservative substitution thereof, X2 = G or D or a conservative substitution thereof, X3 = Y or R or a conservative substitution thereof, X4 = S or Y or a conservative substitution thereof, X5 = Y or C or a conservative substitution thereof, X6 = G or S or a conservative substitution thereof, X7 = Absent or G or a conservative substitution thereof, X8 = Absent or T or a conservative substitution thereof, X9 = Absent or S or a conservative substitution thereof, X10 = Absent or C or a conservative substitution thereof, X11 = Absent or P or a conservative substitution thereof, X12 = Absent or Y or a conservative substitution thereof, X13 = Absent or Y or a conservative substitution thereof, X14 = Absent or Y or a conservative substitution thereof, X15 = G or Y or a conservative substitution thereof. |
| VH-CONSENSUS-51 TABLE 85 | | VH1 | X1 X2 X3 Met X4 (SEQ ID NO: 50099) wherein X1 = D or S or a conservative substitution thereof, X2 = Y or C or a conservative substitution thereof, X3 = V or G or a conservative substitution thereof, X4 = H or Q or a conservative substitution thereof. |
| | | VH2 | X1 Ile Trp Tyr Asp Gly Ser Asn Lys Tyr Tyr Ala Asp Ser Val Lys X2 (SEQ ID NO: 50100) wherein X1 = V or I or a conservative substitution thereof, X2 = G or V or a conservative substitution thereof. |
| | | VH3 | Glu X1 Tyr X2 Ser Gly Trp X3 Asp Tyr Gly X4 Asp Val (SEQ ID NO: 50101) wherein X1 = P or R or a conservative substitution thereof, X2 = T or N or a conservative substitution thereof, X3 = Y or H or a conservative substitution thereof, X4 = M or L or a conservative substitution thereof. |
| VH-CONSENSUS-52 TABLE 86 | | VH1 | Ser Tyr Gly X1 Ser (SEQ ID NO: 50102) wherein X4 = I or F or V or a conservative substitution thereof. |
| | | VH2 | Trp Ile Ser Ala Tyr Asn Gly Asn X1 Lys X2 Ala Gln X3 X4 Gln Gly (SEQ ID NO: 50103) wherein X1 = T or R or a conservative substitution thereof, X2 = Y or N or E or F or a conservative substitution thereof, X3 = K or R or a conservative substitution thereof, X4 = L or F or a conservative substitution thereof. |
| | | VH3 | His Asp Phe Trp Ser Gly Tyr Tyr Lys Gly Met Asp Val (SEQ ID NO: 50227). |
| | | | |

3399

FIGURE 55

| VH-CONSENSUS-53 TABLE 87 | | VH1 | X1 Ser X2 Ala Met Ser (SEQ ID NO: 50104) wherein X1 = S or R or a conservative substitution thereof, X2 = Y or S or a conservative substitution thereof. |
|---|---|---|---|
| | | VH2 | X1 Ile Ser Gly X2 Gly X3 X4 Thr Phe X5 X6 Asp Ser Val Lys Gly (SEQ ID NO: 50105) wherein X1 = A or V or S or a conservative substitution thereof, X2 = R or S or a conservative substitution thereof, X3 = G or I or V or a conservative substitution thereof, X4 = N or S or a conservative substitution thereof, X5 = D or Y or a conservative substitution thereof, X6 = A or T or a conservative substitution thereof. |
| | | VH3 | X1 X2 Ser X3 X4 X5 Phe Asp Tyr (SEQ ID NO: 50106) wherein X1 = E or S or a conservative substitution thereof, X2 = R or N or a conservative substitution thereof, X3 = G or S or a conservative substitution thereof, X4 = S or G or a conservative substitution thereof, X5 = Y or W or a conservative substitution thereof. |
| VH-CONSENSUS-54 TABLE 88 | | VH1 | X1 Tyr X2 Met His (SEQ ID NO: 50107) wherein X1 = S or N or Y or H or a conservative substitution thereof, X2 = V or G or a conservative substitution thereof. |
| | | VH2 | X1 Ile Trp X2 Asp Gly X3 X4 X5 Tyr Tyr X6 Asp Ser Val Lys Gly (SEQ ID NO: 50108) wherein X1 = V or L or a conservative substitution thereof, X2 = H or Y or a conservative substitution thereof, X3 = S or T or a conservative substitution thereof, X4 = N or D or a conservative substitution thereof, X5 = K or A or a conservative substitution thereof, X6 = A or V or G or a conservative substitution thereof. |
| | | VH3 | Glu Asn Ser Ser Ser X1 Tyr Phe Asp Tyr Trp Gly Gln Gly Thr Leu Val Thr Val Ser Ser (SEQ ID NO: 50109) wherein X1 = Y or F or a conservative substitution thereof. |
| VH-CONSENSUS-55 TABLE 89 | | VH1 | Thr Ser Gly Val Gly Val Gly (SEQ ID NO: 50110). |
| | | VH2 | Leu Ile Asn Trp Asn Asp Asp Lys Arg X1 Ser Pro Ser Leu Lys Ser (SEQ ID NO: 50111) wherein X1 = Y or F or a conservative substitution thereof. |
| | | VH3 | Lys X1 Thr Trp Val Ala Phe Asp Ile (SEQ ID NO: 50112) wherein X1 = A or T or a conservative substitution thereof. |
| VH-CONSENSUS-56 TABLE 90 | | VH1 | Ser Tyr X1 X2 X3 (SEQ ID NO: 50113) wherein X1 = V or A or a conservative substitution thereof, X2 = M or I or L or a conservative substitution thereof, X3 = N or S or R or a conservative substitution thereof. |
| | | VH2 | X1 X2 Ser Gly Ser Gly X3 X4 Thr Tyr Tyr X5 Asp Ser Val Lys Gly (SEQ ID NO: 50114) wherein X1 = A or D or a conservative substitution thereof, X2 = I or M or a conservative substitution thereof, X3 = G or D or V or a conservative substitution thereof, X4 = R or S or F or T or a |

FIGURE 55

| | | | |
|---|---|---|---|
| | | | conservative substitution thereof, X5 = A or V or a conservative substitution thereof. |
| | | VH3 | Thr X1 X2 X3 X4 X5 (SEQ ID NO: 50115) wherein X1 = A or G or S or T or Y or a conservative substitution thereof, X2 = T or V or Absent or H or L or G or a conservative substitution thereof, X3 = F or Absent or K or a conservative substitution thereof, X4 = D or Absent or a conservative substitution thereof, X5 = Y or L or a conservative substitution thereof. |
| VH-CONSENSUS-57 TABLE 91 | | VH1 | Ser X1 Ala Met X2 (SEQ ID NO: 50116) wherein X1 = Y or F or a conservative substitution thereof, X2 = S or N or a conservative substitution thereof. |
| | | VH2 | X1 X2 Ser Gly X3 Gly X4 X5 Thr X6 Tyr Ala Asp Ser Val Lys Gly (SEQ ID NO: 50117) wherein X1 = V or A or I or a conservative substitution thereof, X2 = I or L or a conservative substitution thereof, X3 = R or S or G or a conservative substitution thereof, X4 = G or S or K or a conservative substitution thereof, X5 = T or N or S or a conservative substitution thereof, X6 = F or Y or a conservative substitution thereof. |
| | | VH3 | Lys Arg Thr X1 X2 Asp X3 Phe Asp X4 (SEQ ID NO: 50118) wherein X1 = P or G or a conservative substitution thereof, X2 = S or D or E or a conservative substitution thereof, X3 = V or A or a conservative substitution thereof, X4 = I or V or a conservative substitution thereof. |
| VH-CONSENSUS-58 TABLE 92 | | VH1 | X1 X2 X3 Met His (SEQ ID NO: 50237) wherein X1 = S or N or T or a conservative substitution thereof, X2 = Y or F or a conservative substitution thereof, X3 = G or D or N or a conservative substitution thereof. |
| | | VH2 | X1 Ile Trp X2 Asp Gly X3 X4 X5 Tyr X6 X7 Asp Ser Val Lys Gly (SEQ ID NO: 50238) wherein X1 = V or A or a conservative substitution thereof, X2 = Y or H or a conservative substitution thereof, X3 = S or R or a conservative substitution thereof, X4 = N or D or H or a conservative substitution thereof, X5 = K or R or a conservative substitution thereof, X6 = Y or C or S or a conservative substitution thereof, X7 = A or E or T or a conservative substitution thereof. |
| | | VH3 | Asp X1 X2 X3 X4 X5 X6 X7 X8 X9 Asp X10 (SEQ ID NO: 50239) wherein X1 = R or D or H or a conservative substitution thereof, X2 = P or S or A or a conservative substitution thereof, X3 = I or R or Y or a conservative substitution thereof, X4 = S or L or V or W or a conservative substitution thereof, X5 = Absent or G or a conservative substitution thereof, X6 = Absent or A or a conservative substitution thereof, X7 = S or T or A or a conservative substitution thereof, X8 = A or F or S or Y or a conservative substitution thereof, X9 = F or G or S or a |

**FIGURE 55**

| | | | |
|---|---|---|---|
| | | | conservative substitution thereof, X10 = Y or F or a conservative substitution thereof. |
| VH-CONSENSUS-59 TABLE 93 | | VH1 | X1 Tyr X2 Met Asn (SEQ ID NO: 50240) weherein X1 = S or N or a conservative substitution thereof, X2 = N or S or a conservative substitution thereof. |
| | | VH2 | Tyr Ile Ser X1 Ser X2 X3 Thr X4 X5 Tyr X6 Asp Ser Val X7 Gly (SEQ ID NO: 50241) wherein X1 = R or S or a conservative substitution thereof, X2 = S or G or a conservative substitution thereof, X3 = N or S or a conservative substitution thereof, X4 = K or T or a conservative substitution thereof, X5 = Y or H or a conservative substitution thereof, X6 = A or V or a conservative substitution thereof, X7 = K or R or E or Q or a conservative substitution thereof. |
| | | VH3 | Asp X1 X2 X3 X4 X5 X6 X7 X8 X9 Tyr Tyr Gly X10 Asp Val (SEQ ID NO: 50242) wherein X1 = R or S or a conservative substitution thereof, X2 = S or R or a conservative substitution thereof, X3 = G or K or a conservative substitution thereof, X4 = S or G or a conservative substitution thereof, X5 = Y or F or a conservative substitution thereof, X6 = G or Absent or a conservative substitution thereof, X7 = Y or Absent or a conservative substitution thereof, X8 = F or Absent or a conservative substitution thereof, X9 = Y or Absent or a conservative substitution thereof, X10 = L or M or a conservative substitution thereof. |
| VH-CONSENSUS-60 TABLE 94 | | VH1 | Ser Gly Gly Asp Tyr Trp Ser (SEQ ID NO: 50119). |
| | | VH2 | Tyr Ile Tyr Tyr Ser Gly X1 Thr Tyr Tyr Asn Pro Ser Leu Lys Ser (SEQ ID NO: 50120) wherein X1 = S or I or P or a conservative substitution thereof. |
| | | VH3 | Asp X1 X2 X3 X4 Gly Met Asp Val (SEQ ID NO: 50121) wherein X1 = S or G or H or a conservative substitution thereof, X2 = S or A or a conservative substitution thereof, X3 = S or L or R or a conservative substitution thereof, X4 = Y or R or H or a conservative substitution thereof. |

FIGURE 55

Table 20A

**VARIABLE LIGHT CDR CONSENSUS SEQUENCES I**

| NAME (ORIGINAL AND PATENT) | Patent SEQ ID NO.: | SEQUENCE |
|---|---|---|
| VL-Consensus-1 (Table 35) (generated from 13 light chain sequences) | SEQ ID NO: 50366 | DIQMTQSPSSVSASVGDRVTITCRASQGISRWLAWY QQKPGKAPKLLIYAASSLQSGVPSRFSGSGSGTDFTL TISSLQPEDFATYYCQQANSFPFTFGPGTKVDIKR |
| VL-Consensus-2 (Table 36) (generated from 11 light chain sequences) | SEQ ID NO: 50367 | SYELTQPPSVSVSPGQTASITCSGDKLGDKYAYWYQ QKPGQSPVLVIYQDRKRPSGIPERFSGSNSGNTATLTI SGTQAMDEADYYCQAWDNSTVVFGGGTKLTVLGG |
| VL-Consensus-3 (Table 37) (generated from 15 light chain sequences) | SEQ ID NO: 50368 | DIVMTQSPDSLAVSLGERATINCKSSQSVLYSSNNNN YLAWYQQKPGQPPKLLIYWASTRESGVPDRFSGSGS GTDFTLTISSLQAEDVAVYYCQQYYSTPPTFGQGTK VEIKR |
| VL-Consensus-4 (Table 38) (generated from 23 light chain sequences) | SEQ ID NO: 50369 | DIQMTQSPSSLSASVGDRVTITCRASQGISNYLAWFQ QKPGKAPKSLIYAASSLQSGVPSKFSGSGSGTDFTLTI SSLQPEDFATYYCQQYNSYPFTFGPGTKVDIKR |
| VL-Consensus 5 (Table 39) (generated from 17 light chain sequences) | SEQ ID NO: 50370 | DIQMTQSPSSLSASVGDRVTITCRASQGIRNNLGWY QQKPGKAPKRLIYAASSLQSGVPSRFSGSGSGTEFTL TISSLQPEDFATYYCLQYNSYPFTFGQGTKVEIKR |
| VL-Consensus 6 (Table 40) (generated from 11 light chain sequences) | SEQ ID NO: 50371 | DIQMTQSPSSLSASVGDRVTITCRASQGISNYLAWFQ QKPGKAPKSLISAASSLQSGVPSKFSGSGSGTDFTLTI SSLQPEDFATYYCQQYNSYPFTFGPGTKVDIKR |
| VL-Consensus 7 (kappa) (Table 41) (generated from 26 light chain sequences) | SEQ ID NO: 50372 | DIQMTQSPSSLSASVGDRVTITCRASQDIRSDLGWYQ QKPGKAPKRLIYAASSLQSGVPSRFSGSGSGTEFTLTI SSLQPEDFATYYCLQHTIYPPTFGGGTKVEIKR |
| VL-Consensus-7 (lambda) (Table 41) (generated from 26 light chain sequences) | SEQ ID NO: 50373 | QSXLTQPXSXSGSPGQSITISCTGTSSDVGXXNXVSW YQQHPGKAPKLMIYEVSNRPSGVXXRFSGSKSGNTA SLTISGLQXEDEADYYCSSYTXSXTVVFGGGTKLTV LG |
| VL-Consensus-8 (Table 42) (generated from 25 light chain sequences) | SEQ ID NO: 50374 | DIQMTQSPSSLSASVGDRVTITCRASQSISSYLNWYQ QKPGKAPKLLIYAASSLQSGVPSRFSGSGSGTDFTLTI SSLQPEDFATYYCQQSYRTPLFTFGPGTKVDIKR |

FIGURE 55

| | | |
|---|---|---|
| VL-Consensus-9 (Table 43) (generated from 14 light chain sequences) | SEQ ID NO: 50375 | DIQMTQSPSSLSASVGDRVTITC<u>RASQSIISYLN</u>WYQ QKPGKAPKLLIY<u>TASSLQS</u>GVPSRFSGSGSGTDFTLTI SSLQPEDFATYYC<u>QQTYSTPLT</u>FGGGTKVEIKR |
| VL-Consensus-10 (Table 44) (generated from 22 light chain sequences) | SEQ ID NO: 50376 | DIQMTQSPSSLSASVGDRVTITC<u>RASQGIRNDLG</u>WY QQKPGKAPKRLIY<u>AASSLQS</u>GVPSRFSGSGSGTEFTL TISSLQPEDFATYYC<u>LQHYSYPRT</u>FGQGTKVEIKR |
| VL-Consensus 11 (Table 45) (generated from 16 light chain sequences) | SEQ ID NO: 50377 | DIQMTQSPSSLSASVGDRVTITC<u>RASQGIRNDLG</u>WY QQKPGKAPKRLIY<u>AASSLQS</u>GVPSRFSGSGSGTEFTL TISSLQPEDFATYYC<u>LQHNSYPFT</u>FGXGTKVEIKR |
| VL-Consensus 12 (Table 46) (generated from 71 light chain sequences) | SEQ ID NO: 50378 | DIQMTQSPSSLSASVGDRVTITC<u>RASQGIRNDLG</u>WY QQKPGKAPKRLIY<u>AASSLQS</u>GVPSRFSGSGSGTEFTL TISSLQPEDFATYYC<u>LQHNSYPLT</u>FGGGTKVEIKR |
| VL-Consensus 13 (Table 47) (generated from 21 light chain sequences) | SEQ ID NO: 50379 | DIQMTQSPSSLSASVGDRVTITC<u>RASQGIRNDLG</u>WY QQKPGKAPKRLIY<u>AASSLQS</u>GVPSRFSGSGSGTEFTL TISSLQPEDFATYYC<u>LQHSSYPLT</u>FGGGTKVEIKR |
| VL-Consensus 14 (Table 48) (generated from 13 light chain sequences) | SEQ ID NO: 50380 | DIQMTQSPSSLSASVGDRVTITC<u>RASQGIRNDLG</u>WY QQKPGKAPKRLIY<u>AASSLQS</u>GVPSRFSGSGSGTEFTL TISSLQPEDFATYYC<u>LQHSSYPLT</u>FGGGTKVEIKR |
| VL-Consensus 15 (Table 95) (generated from 209 light chain sequences) | SEQ ID NO: 50312 | DIQMTQSPSSLSASVGDRVTITC<u>RASQGIRDLG</u>WYQ QKPGKAPKRLIY<u>AASSLQS</u>GVPSRFSGSGSGTEFTLTI SSLQPEDFATYYC<u>LQHNSYPLT</u>FGGGTKVEIKR |
| VL-Consensus 16 (Table 96) (generated from 174 light chain sequences) | SEQ ID NO: 50313 | SYELTQPPSVSVSPGQTASITC<u>SGDKLGDKYAC</u>WYQ QKPGQSPVLVIY<u>QDRKRPS</u>GIPERFSGSNSGNTATLTI SGTQAMDEADYYC<u>QAWDSSTVV</u>FGGGTKLTVLG |
| VL-Consensus 17 (Table 97) (generated from 162 light chain sequences) | SEQ ID NO: 50314 | EIVLTQSPGTLSLSPGERATLSC<u>RASQSVYSSYLA</u>WY QQKPGQAPRLLIY<u>GASSRAT</u>GIPDRFSGSGSGTDFTL TISRLEPEDFAVYYC<u>QQYDNSPWT</u>FGQGTKVEIKR |
| VL-Consensus 18 (Table 98) (generated from 147 light chain sequences) | SEQ ID NO: 50315 | DIQMTQSPSSLSASVGDRVTITC<u>RASQGIRNDLG</u>WY QQKPGKAPKRLIY<u>AASSLQS</u>GVPSRFSGSGSGTEFTL TISSLQPEDFATYYC<u>LQHYSYPRT</u>FGQGTKVEIKR |

FIGURE 55

| VL-Consensus 19 (Table 99) (generated from 132 light chain sequences) | SEQ ID NO: 50316 | DIVMTQSPDSLAVSLGERATINCKSSQSVLHSSNNNN YLAWYQQKPGQPPKLLIYWASTRESGVPDRFSGSGS GTDFTLTISSLQAEDVAVYYCQQYYSTPPTFGQGTK VEIKR |
|---|---|---|
| VL-Consensus 20 (Table 100) (generated from 109 light chain sequences) | SEQ ID NO: 50317 | DIQMTQSPSSLSASVGDRVTITCRASQGIRNDLGWY QQKPGKAPKRLIYAASSLQSGVPSRFSGSGSGEFTLTI SSLQPEDFATYYCLQHNSYPFTFGPGTKVDIKR |
| VL-Consensus 21 (Table 101) (generated from 92 light chain sequences) | SEQ ID NO: 50318 | DIQMTQSPSSLSASVGDRVTITCRASQGISNYLAWFQ QKPGKAPKSLIYAASSLQSGVPSKFSGSGSGTDFTLTI SSLQPEDFATYYCQQYSTYPLTFGGGTKVEIKR |
| VL-Consensus 22 (Table 102) (generated from 89 light chain sequences) | SEQ ID NO: 50319 | DIQMTQSPSSLSASVGDRVTITCRASQNIISYLNWYQ QKPGKAPKLLIYTASSLQSGVPSRFSGSGSGTDFTLTI SSLQPEDFATYYCQQSYSTPLTFGGGTKVEIKR |
| VL-Consensus 23 (Table 103) (generated from 86 light chain sequences) | SEQ ID NO: 50320 | DIQMTQSPSSLSASVGDRVTITCRASQGISNYLAWFQ QKPGKAPKSLIYAASSLQSGVPSKFSGSGSGTDFTLTI SSLQPEDFATYYCQQYNSYPFTFGPGTKVDIKR |
| VL-Consensus 24 (Table 104) (generated from 81 light chain sequences) | SEQ ID NO: 50321 | SYELTQPPSVSVSPGQTASITCSGDKLGDKYACWYQ QKPGQSPVLVIYQDRKRPSGIPERFSGSNSGNTATLTI SGTQAMDEADYYCQAWDSSTVVFGGGTKLTVLG |
| VL-Consensus 25 (Table 105) (generated from 65 light chain sequences) | SEQ ID NO: 50322 | DIVMTQTPLSLSVTPGQPASISCKSSQSLLHGD- GKTYLYWYLQKPGQPPQLLIYEVSNRFSGVPDRFSG SGSGTDFTLKISRVEAEDVGVYYCMQSIQLPWTFGQ GTKVEIKR |
| VL-Consensus 26 (Table 106) (generated from 58 light chain sequences) | SEQ ID NO: 50323 | DIQMTQSPSSLSASVGDRVTITCRASQSISSYLNWYQ QKPGKAPKLLIYAASSLQSGVPSRFSGSGSGTDFTLTI SSLQPEDFATYYCQQSYSPPFTFGPGTKVDIKR |
| VL-Consensus 27 (Table 107) (generated from 47 light chain sequences) | SEQ ID NO: 50324 | DIVMTQSPDSLAVSLGERATINCKSSQSVLYSSNNNN YLAWYQQKPGQPPKLLIYWASTRESGVPDRFSGSGS GTDFTLTISSLQAEDVAVYYCQQYYSTPCSFGQGTK LEIKR |
| VL-Consensus 28 (Table 108) (generated from 42 light chain sequences) | SEQ ID NO: 50325 | DIQMTQSPSSVSASVGDRVTITCRASQGISNWLAWY QQKPGKAPKLLIYAASSLQSGVPSRFSGSGSGTDFTL TISSLQPEDFATYYCQQAFPWTFGQGTKVEIKR |

FIGURE 55

| VL-Consensus 29 (Table 109) (generated from 37 light chain sequences) | SEQ ID NO: 50326 | DIVMTQSPDSLAVSLGERATINCKSSQSVLHSSNNNN YLAWYQQKPGQPPKLLIYWASTRESGVPDRFSGSGS GTDFTLTISSLQAEDVAVYYCQQYYSTPVTFGPGTK VDIKR |
| VL-Consensus 30 (Table 110) (generated from 31 light chain sequences) | SEQ ID NO: 50327 | QTVVTQEPSLTVSPGGTVTLTCASSTGAVTSG-YYPNWFQQKPGQAPRALIYSTSNKHSWTPARFSGSL LGGKAALTLSGVQPEDEAEYYCLLYYGGAQLVFGG GTKLTVLG |
| VL-Consensus 31 (Table 111) (generated from 25 light chain sequences) | SEQ ID NO: 50328 | EIVMTQSPATLSVSPGERATLSCRASQSVNSNLAWY QQKPGQAPRLLIYGASTRATGIPARFSGSGSGTEFTL TISSLQSEDFAVYYCQQYNDWPCSFGQGTKLEIKR |
| VL-Consensus 32 (Table 112) (generated from 24 light chain sequences) | SEQ ID NO: 50329 | EFMLTQSPGTLSLSPGERATLSCRASQSVSSSYLVWY QQKPGQAPRLLIYGASTRATGIPDRFSGSGSGTDFTL TISRLEPEYFAVYYCQQYGCSPLTFGGGTKVEITR |
| VL-Consensus-33 (Table 113) (generated from 21 light chain sequences) | SEQ ID NO: 50330 | DIVMTQTPLSLSVTPGQPASISCKSSQSLLHSEGKTYL YWYLQKPGQPPQLLIYEVSNRFSGVPDRFSGSGSGT DFTLKISRVEAEDVGVYYCMQSIQLPLTFGGGTKVEI KR |
| VL-Consensus-34 (Table 114) (generated from 18 light chain sequences) | SEQ ID NO: 50331 | QSALTQPASVSGSPGQSITISCTGTSSDVGGYNYVSW YQQHPGKAPKLMIYEVSNRPSGVSNRFSGSKSGNTA SLTISGLQABDEADYYCNSYTRSITWVFGGGTKLTV LG |
| VL-Consensus 35 (Table 115) (generated from 17 light chain sequences) | SEQ ID NO: 50332 | DIQMTQSPSSLSASVGDRVTITCRASQSISNYLNWYQ QKPGKAPKLLIYAASSLQSGVPSRFSGSGSGTDFTLTI SSLQPEDFATYYCQQSYSTPTWTFGQGTKVEIKR |
| VL-Consensus 36 (Table 116) (generated from 16 light chain sequences) | SEQ ID NO: 50333 | DIQMTQSPSSVSASVGDRVTITCRASQGISSWLAWY QQKPGKAPKLLIYAASSLQSGVPSRFSGSGSGTDFTL TISSLQPEDFATYYCQQINSFPLTFGGGTKVEIKR |
| VL-Consensus 37 (Table 117) (generated from 16 light chain sequences) | SEQ ID NO: 50334 | DIQMTQSPSSLSASVGDRVTITCQASQDINNYLNWY QQKPGKAPKLLIYDASNLETGVPSRFSGSGSGTDFTF TISSLQPEDIATYYCQQYDNLPITFGQGTRLEIKR |
| VL-Consensus 38 (Table 118) (generated from 16 light chain sequences) | SEQ ID NO: 50335 | QSVLTQPPSVSAAPGQKVTISCGSGSSNIGNNYVSWY QQLPGTAPKLLIYDNNKRPSGIPDRFSGSKSGTSATL GITGLQTGDEADYYCGTWDSSLSVGVFGGGTKLTV LG |

3406

FIGURE 55

| VL-Consensus 39 (Table 119) (generated from 14 light chain sequences) | SEQ ID NO: 50336 | DIVMTQSPLSLPVTPGEPASISCRSSQSLLHSNGYNYL DWYLQKPGQSPQLLIYLGSNRASGVPDRFSGSGSGT DFTLKISRVEAEDVGVYYCMQALHPPLTFGGGTKVE IKR |
|---|---|---|
| VL-Consensus 40 (Table 120) (generated from 13 light chain sequences) | SEQ ID NO: 50337 | QSVLTQPPSASGTPGQRVTISCSGSSSNIGSNTVNWY QQLPGTAPKLLIYSNNQRPSGVPDRFSGSKSGTSASL AISGLQSEDEADYYCAAWDDSLNGVVFGGGTKLTV LG |
| VL-Consensus 41 (Table 121) (generated from 11 light chain sequences) | SEQ ID NO: 50338 | QSVLTQPPSASGTPGQRVTISCSGSSSNIGSNIVTWYQ QLPGTAPKLLIYSNDQRPSGVPDRFSGSKSGTSASLAI SGLQSEDEADYYCAAWDDSLNGWVFGGGTTLTVL G |
| VL-Consensus 42 (Table 122) (generated from 10 light chain sequences) | SEQ ID NO: 50339 | DIQMTQSPSSLSASVGDRVTITCRASQGIRNDLGWY QQKPGKAPKRLIYAASSLQSGVPSRFSGSGSGTEFTL TISSLQPEDFATYYCLQHNSYPITFGQGTRLEIKR |
| VL-Consensus 43 (Table 123) (generated from 10 light chain sequences) | SEQ ID NO: 50340 | DIQMTQSPSSLSASVGDRVTITCRASQSISSYLNWYQ QKPGKAPKLLIYAASSLQSGVPSRFSGSGSGTDFTLTI SSLQPEDFATYYCQQSYSIPITFGQGTRLEIKR |
| VL-Consensus 44 (Table 124) (generated from 10 light chain sequences) | SEQ ID NO: 50341 | DIVMTQTPLSLSVTPGQPASISCKSSQSLLHSEGKTYL YWYLQKPGQPPQLLIYEVSNRFSGVPDRFSGSGSGT DFTLKISRVEAEDVGVYYCMQSIQLPITFGQGTRLEI KR |
| VL-Consensus 45 (Table 125) (generated from 10 light chain sequences) | SEQ ID NO: 50342 | EIVLTQSPGTLSLFPGERATLSCRASQSVISSYLAWYQ QKPGQAPRLLIFGVSSRATGIPDRFSGSGSGTDFTLTI SRLEPEDFAVYYCQQYGRSPFNFGPGTKVDIKR |
| VL-Consensus 46 (Table 126) (generated from 10 light chain sequences) | SEQ ID NO: 50343 | EIVMTQSPATLSVSPGERATLSCRASQSVSSNLAWY QQKPGQAPRLLIYGASTRATGIPARFSGSGSGTEFTL TISSLQSEDFAVYYCQQYNDWPWTFGQGTKVEIKR |
| VL-Consensus 47 (Table 127) (generated from 10 light chain sequences) | SEQ ID NO: 50344 | EIVLTQSPDFQSVTPKEKVTITCRASQSIGSSLHWYQ QKPDQSPKLLIKYASQSFSGVPSRFSGSGSGTDFTLTI NSLEAEDAATYYCHQSSSLPWTFGQGTKVEIKR |
| VL-Consensus 48 (Table 128) (generated from 9 light chain sequences) | SEQ ID NO: 50345 | DIQMTQSPSSLSASVGDRVTITCRASQGIRNDLGWY QQKPGKAPKRLIYAASSLQSGVPSRFSGSGSGTEFTL TISSLQPEDFATYYCLQHYSYPRSFGQGTKLEIKR |

FIGURE 55

| VL-Consensus 49 (Table 129) (generated from 9 light chain sequences) | SEQ ID NO: 50346 | DIQMTQSPSSLSASVGDRVTITCRASQGISNYLAWFQ QKPGKAPKSLIYAASSLQSGVPSKFSGSGSGTDFTLTI SSLQPEDFATYYCQQYLSYPITFGQGTRLEIKR |
|---|---|---|
| VL-Consensus 50 (Table 130) (generated from 9 light sequences) | SEQ ID NO: 50347 | EIVLTQSPDFQSVTPKEKVTITCRASQSIGSSLHWYQ QKPDQSPKLLIKYASQSFSGVPSRFSGSGSGTDFTLTI NSLEAEDAATYYCHQSRRLPLTFGGGTKVEIKR |
| VL-Consensus 51 (Table 131) (generated from 9 light chain sequences) | SEQ ID NO: 50348 | SSELTQDPAVSVALGQTVRITCQGDSLRPYYASWYQ QKPGQAPVLVIYGKNNRPSGIPDRFSGSSSGNTASLTI TGAQAEDEADYYCNSRDSSGNHLVVFGGGTKLTVL G |
| VL-Consensus 52 (Table 132) (generated from 8 light chain sequences) | SEQ ID NO: 50349 | DVVMTQSPLSLPVTLGQPASISCRSSQSLVYSDGNTY LNWFQQRPGQSPRRLIYKVSNWDSGVPDRFSGSGSG TDFTLKISRVEAEDVGVYYCMQGTHWPLTFGGGTK VEIKR |
| VL-Consensus 53 (Table 133) (generated from 8 light chain sequences) | SEQ ID NO: 50350 | EIVMTQSPATLSVSPGERATLSCRASQSVSRNLAWY QQKPGQAPRLLIYGASTRATGIPARFSGSGSGTEFTL TISSLQSEDFAVYYCQQYNNWPLTFGGGTKVEIKR |
| VL-Consensus 54 (Table 134) (generated from 8 light chain sequences) | SEQ ID NO: 50351 | SYELTQPLSVSVALGQTARITCGGNNIGRKNVHWYQ QKPGQAPVLVIYRDSDRPSGIPERFSGSNSGNTATLTI SRAQAGDEADYYCQVWDSSTVVFGGGTKLTVLG |
| | | |

**FIGURE 55**

Table 20B

VARIABLE LIGHT CDR CONSENSUS SEQUENCES

| Name (original and patent) | Patent SEQ ID NO: | CDR | Sequence |
|---|---|---|---|
| VL-Consensus-1 (Table 35) | 50423 | VL1 | RASQGISRWLA |
| | 50424 | VL2 | AASSLQS |
| | 50425 | VL3 | QQANSFPFT |
| | | | |
| VL-Consensus-2 (Table 36) | 50426 | VL1 | SGDKLGDKYAY |
| | 50427 | VL2 | QDRKRPS |
| | 50428 | VL3 | QAWDNSTVV |
| | | | |
| VL-Consensus-3 (Table 37) | 50429 | VL1 | KSSQSVLYSSNNNNYLA |
| | 50430 | VL2 | WASTRES |
| | 50431 | VL3 | QQYYSTPPT |
| | | | |
| VL-Consensus-4 (Table 38) | 50432 | VL1 | RASQGISNYLA |
| | 50433 | VL2 | AASSLQS |
| | 50434 | VL3 | QQYNSYPFT |
| | | | |
| VL-Consensus-5 (Table 39) | 50435 | VL1 | RASQGIRNNLG |
| | 50436 | VL2 | AASSLQS |
| | 50437 | VL3 | LQYNSYPFT |
| | | | |
| VL-Consensus-6 (Table 40) | 50438 | VL1 | RASQGISNYLA |
| | 50439 | VL2 | AASSLQS |
| | 50440 | VL3 | QQYNSYPFT |
| | | | |
| VL-Consensus-7κ (Table 41) | 50441 | VL1 | RASQDIRSDLG |
| | 50442 | VL2 | AASSLQS |
| | 50443 | VL3 | LQHTIYPPT |
| | | | |
| VL-Consensus-7λ (Table 41) | 50444 | VL1 | TGTSSDVGXXNXVS |
| | 50445 | VL2 | EVSNRPS |
| | 50446 | VL3 | SSYTXSXTVV |
| | | | |
| VL-Consensus-8 (Table 42) | 50447 | VL1 | RASQSISSYLN |
| | 50448 | VL2 | AASSLQS |
| | 50449 | VL3 | QQSYRTPLFT |
| | | | |
| VL-Consensus-9 (Table 43) | 50450 | VL1 | RASQSIISYLN |
| | 50451 | VL2 | TASSLQS |
| | 50452 | VL3 | QQTYSTPLT |

**FIGURE 55**

| | | | |
|---|---|---|---|
| VL-Consensus-10 (Table 44) | 50453 | VL1 | RASQGIRNDLG |
| | 50454 | VL2 | AASSLQS |
| | 50455 | VL3 | LQHYSYPRT |
| VL-Consensus-11 (Table 45) | 50456 | VL1 | RASQGIRNDLG |
| | 50457 | VL2 | AASSLQS |
| | 50458 | VL3 | LQHNSYPFT |
| VL-Consensus-12 (Table 46) | 50459 | VL1 | RASQGIRNDLG |
| | 50460 | VL2 | AASSLQS |
| | 50461 | VL3 | LQHNSYPLT |
| VL-Consensus-13 (Table 47) | 50462 | VL1 | RASQGIRNDLG |
| | 50463 | VL2 | AASSLQS |
| | 50464 | VL3 | LQHSSYPLT |
| VL-Consensus-14 (Table 48) | 50465 | VL1 | RASQGIRNDLG |
| | 50466 | VL2 | AASSVQS |
| | 50467 | VL3 | LQHNSYPLT |
| VL-Consensus-15 (Table 95) | 50606 | VL1 | RASQGIRNDLG |
| | 50607 | VL2 | AASSLQS |
| | 50608 | VL3 | LQHNSYPLT |
| VL-Consensus-16 (Table 96) | 50609 | VL1 | SGDKLGDKYAC |
| | 50610 | VL2 | AASSLQS |
| | 50611 | VL3 | QAWDSSTVV |
| VL-Consensus-17 (Table 97) | 50612 | VL1 | RASQSVYSSYLA |
| | 50613 | VL2 | GASSRAT |
| | 50614 | VL3 | QQYDNSPWT |
| VL-Consensus-18 (Table 98) | 50615 | VL1 | RASQGIRNDLG |
| | 50616 | VL2 | AASSLQS |
| | 50617 | VL3 | LQHYSYPRT |
| VL-Consensus-19 (Table 99) | 50618 | VL1 | KSSQSVLHSSNNNNYLA |
| | 50619 | VL2 | WASTRES |
| | 50620 | VL3 | QQYYSTPPT |
| VL-Consensus-20 (Table 100) | 50621 | VL1 | RASQGIRNDLG |
| | 50622 | VL2 | AASSLQS |
| | 50623 | VL3 | LQHNSYPFT |
| VL-Consensus-21 (Table 101) | 50624 | VL1 | RASQGISNYLA |
| | 50625 | VL2 | AASSLQS |
| | 50626 | VL3 | QQYSTYPLT |

FIGURE 55

| VL-Consensus-22 (Table 102) | 50627 | VL1 | RASQNIISYLN |
| | 50628 | VL2 | TASSLQS |
| | 50629 | VL3 | QQSYSTPLT |
| VL-Consensus-23 (Table 103) | 50630 | VL1 | RASQGISNYLA |
| | 50631 | VL2 | AASSLQS |
| | 50632 | VL3 | QQYNSYPFT |
| VL-Consensus-24 (Table 104) | 50633 | VL1 | RASQGISRWLA |
| | 50634 | VL2 | AASSLQS |
| | 50635 | VL3 | QQANSFPFT |
| VL-Consensus-25 (Table 105) | 50636 | VL1 | KSSQSLLHGDGKTYLY |
| | 50637 | VL2 | EVSNRFS |
| | 50638 | VL3 | MQSIQLPWT |
| VL-Consensus-26 (Table 106) | 50639 | VL1 | RASQSISSYLN |
| | 50640 | VL2 | AASSLQS |
| | 50641 | VL3 | QQSYSPPFT |
| VL-Consensus-27 (Table 107) | 50642 | VL1 | KSSQSVLYSSNNNNYLA |
| | 50643 | VL2 | WASTRES |
| | 50644 | VL3 | QQYYSTPCS |
| VL-Consensus-28 (Table 108) | 50645 | VL1 | RASQGISNWLA |
| | 50646 | VL2 | AASSLQS |
| | 50647 | VL3 | QQANSFPWT |
| VL-Consensus-29 (Table 109) | 50648 | VL1 | KSSQSVLHSSNNNNYLA |
| | 50649 | VL2 | WASTRES |
| | 50650 | VL3 | QQYYSTPVT |
| VL-Consensus-30 (Table 110) | 50651 | VL1 | ASSTGAVTSGYYPN |
| | 50652 | VL2 | STSNKHS |
| | 50653 | VL3 | LLYYGGAQLV |
| VL-Consensus-31 (Table 111) | 50654 | VL1 | RASQSVNSNLA |
| | 50655 | VL2 | GASTRAT |
| | 50656 | VL3 | QQYNDWPCS |

FIGURE 55

| VL-Consensus-32 (Table 112) | 50657 | VL1 | RASQSVSSSYLV |
|---|---|---|---|
| | 50658 | VL2 | GASTRAT |
| | 50659 | VL3 | QQYGCSPLT |
| VL-Consensus-33 (Table 113) | 50660 | VL1 | KSSQSLLHSEGKTYLY |
| | 50661 | VL2 | EVSNRFS |
| | 50662 | VL3 | MQSIQLPLT |
| VL-Consensus-34 (Table 114) | 50663 | VL1 | TGTSSDVGGYNYVS |
| | 50664 | VL2 | EVSNRPS |
| | 50665 | VL3 | NSYTRSITWV |
| VL-Consensus-35 (Table 115) | 50666 | VL1 | RASQSISNYLN |
| | 50667 | VL2 | AASSLQS |
| | 50668 | VL3 | QQSYSTPTWT |
| VL-Consensus-36 (Table 116) | 50669 | VL1 | RASQGISSWLA |
| | 50670 | VL2 | AASSLQS |
| | 50671 | VL3 | QQ NSFPLT |
| VL-Consensus-37 (Table 117) | 50672 | VL1 | QASQDINNYLN |
| | 50673 | VL2 | DASNLET |
| | 50674 | VL3 | QQYDNLPIT |
| VL-Consensus-38 (Table 118) | 50675 | VL1 | SGSSSNIGNNYVS |
| | 50676 | VL2 | DNNKRP |
| | 50677 | VL3 | GTWDSSLSVGV |
| VL-Consensus-39 (Table 119) | 50678 | VL1 | RSSQSLLHSNGYNYLD |
| | 50679 | VL2 | LGSNRAS |
| | 50680 | VL3 | MQALPLT |
| VL-Consensus-40 (Table 120) | 50681 | VL1 | SGSSSNIGSNTVN |
| | 50682 | VL2 | SNNQRPS |
| | 50683 | VL3 | AAWDDSLNGVV |
| VL-Consensus-41 (Table 121) | 50684 | VL1 | SGSSSNIGSNIVT |
| | 50685 | VL2 | SNDQRPS |
| | 50686 | VL3 | AAWDDSLNGWV |

FIGURE 55

| VL-Consensus-42 (Table 122) | 50687 | VL1 | RASQGIRNDLG |
|---|---|---|---|
| | 50688 | VL2 | AASSLQS |
| | 50689 | VL3 | LQHNSYPIT |
| VL-Consensus-43 (Table 123) | 50690 | VL1 | RASQSISSYLN |
| | 50691 | VL2 | AASSLQS |
| | 50692 | VL3 | QQSYSIPIT |
| VL-Consensus-44 (Table 124) | 50693 | VL1 | KSSQSLLHSEGKTYLY |
| | 50694 | VL2 | EVSNRFS |
| | 50695 | VL3 | MQSIQ PIT |
| VL-Consensus-45 (Table 125) | 50696 | VL1 | RASQSVISSYLA |
| | 50697 | VL2 | GVSSRAT |
| | 50698 | VL3 | QQYGRSPFN |
| VL-Consensus-46 (Table 126) | 50699 | VL1 | RASQSVSSNLA |
| | 50700 | VL2 | GASTRAT |
| | 50701 | VL3 | QQYNDWPWT |
| VL-Consensus-47 (Table 127) | 50702 | VL1 | RASQSIGSSLH |
| | 50703 | VL2 | YASQSFS |
| | 50704 | VL3 | HQSSSLPWT |
| VL-Consensus-48 (Table 128) | 50705 | VL1 | RASQGIRNDLG |
| | 50706 | VL2 | AASSLQS |
| | 50707 | VL3 | LQHYSYPRS |
| VL-Consensus-49 (Table 129) | 50708 | VL1 | RASQGISNYLA |
| | 50709 | VL2 | AASSLQS |
| | 50710 | VL3 | QQYLSYPIT |
| VL-Consensus-50 (Table 130) | 50711 | VL1 | RASQSIGSSLH |
| | 50712 | VL2 | YASQSFS |
| | 50713 | VL3 | HQSRRLPLT |
| VL-Consensus-51 (Table 131) | 50714 | VL1 | QGDSLRPYYAS |
| | 50715 | VL2 | GKNNRPS |

FIGURE 55

| | 50716 | VL3 | NSRDSSGNHLVV |
|---|---|---|---|
| | | | |
| VL-Consensus-52 (Table 132) | 50717 | VL1 | RSSQSLVYSDGNTYLN |
| | 50718 | VL2 | KVSNWDS |
| | 50719 | VL3 | MQGTHWPLT |
| | | | |
| VL-Consensus-53 (Table 133) | 50720 | VL1 | RASQSVSRNLA |
| | 50721 | VL2 | GASTRAT |
| | 50722 | VL3 | QQYNNWPLT |
| | | | |
| VL-Consensus-54 (Table 134) | 50723 | VL1 | GGNNIGRKNVH |
| | 50724 | VL2 | RDSRPS |
| | 50725 | VL3 | QVWDSSTVV |

EP 4 435 105 A2

FIGURE 55

Table 20C

**VARIABLE LIGHT CDR CONSENSUS SEQUENCES II**

| NAME (ORIGINAL AND PATENT) | Patent SEQ ID NO: | CDR | Sequence |
|---|---|---|---|
| VL-CONSENSUS-15 TABLE 95 | SEQ ID NO: 50122 | VL1 | X1 X2 Ser X3 X4 X5 X6 X7 X8 X9 X10, wherein X1 = R or L or a conservative substitution thereof, X2 = A or T or a conservative substitution thereof, X3 = Q or R or a conservative substitution thereof, X4 = G or D or A or V or a conservative substitution thereof, X5 = I or M or V or a conservative substitution thereof, X6 = R or E or K or N or S or a conservative substitution thereof, X7 = N or S or D or T or K or I or a conservative substitution thereof, X8 = D or N or A or a conservative substitution thereof, X9 = L or F or V or a conservative substitution thereof, X10 = G or D or N or a conservative substitution thereof. |
| | SEQ ID NO: 50123 | VL2 | X1 X2 X3 X4 X5 X6 X7, wherein X1 = A or T or S or V or G or D or R or a conservative substitution thereof, X2 = A or T or V or E or a conservative substitution thereof, X3 = S or F or C or Y or a conservative substitution thereof, X4 = S or N or T or F or R or I or a conservative substitution thereof, X5 = L or V or F or S or a conservative substitution thereof, X6 = Q or H or E or a conservative substitution thereof, X7 = S or R or N or T or G or a conservative substitution thereof. |
| | SEQ ID NO: 50124 | VL3 | X1 X2 X3 X4 X5 X6 X7 X8 X9, wherein X1 = L or V or I or a conservative substitution thereof, X2 = Q or H or a conservative substitution thereof, X3 = H or D or Y or N or R or a conservative substitution thereof, X4 = N or S or Y or T or D or K or A or I or E or H or P or R or a conservative substitution thereof, X5 = S or I or N or R or T or D or A or L or V or a conservative substitution thereof, X6 = Y or F or H or S or a conservative substitution thereof, X7 = P or A or M or a conservative substitution thereof, X8 = L or P or F or N or V or a conservative substitution thereof, X9 = T or K or I or a conservative substitution thereof. |
| VL-CONSENSUS-16 TABLE 96 | SEQ ID NO: 50125 | VL1 | Ser Gly X1 X2 X3 Gly X4 X5 X6 X7 X8, wherein X1 = D or N or E or Y or S or a conservative substitution thereof, X2 = K or R or N or E or T or a conservative substitution thereof, X3 = L or M or S or a conservative substitution thereof, X4 = D or N or E or G or Y or T or H or V or a conservative substitution thereof, X5 = K or R or a conservative substitution thereof, X6 = Y or F or S or a conservative substitution thereof, X7 = A or V or T or D or S or a conservative substitution thereof, X8 = C or S or Y or W or H or a conservative substitution thereof. |

3415

**FIGURE 55**

| | SEQ ID NO: 50126 | VL2 | X1 X2 X3 X4 Arg X5 X6, wherein X1 = Q or E or K or a conservative substitution thereof, X2 = D or N or a conservative substitution thereof, X3 = R or S or N or K or Y or M or T or G or I or a conservative substitution thereof, X4 = K or R or Q or a conservative substitution |
|---|---|---|---|
| | SEQ ID NO: 50228 | VL3 | X1 X2 X3 X4 X5 X6 X7 X8 X9 X10, wherein X1 = Q or L or K or a conservative substitution thereof, X2 = A or T or a conservative substitution thereof, X3 = W or R or a conservative substitution thereof, X4 = D or H or V or N or G or a conservative substitution thereof, X5 = S or N or Absent or I or R or K or T or a conservative substitution thereof, X6 = Absent or S or N or R or a conservative substitution thereof, X7 = S or T or N or R or G or F or I or V or Y or a conservative substitution thereof, X8 = T or S or Y or A or F or P or N or K or I or R or a conservative substitution thereof, X9 = V or A or T or M or L or G or a conservative substitution thereof, X10 = V or I or L or A or M. |
| VL-CONSENSUS-17 TABLE 97 | SEQ ID NO: 50127 | VL1 | X1 X2 X3 X4 X5 X6 X7 X8 X9 X10 Leu X11, wherein X1 = R or W or a conservative substitution thereof, X2 = A or T or a conservative substitution thereof, X3 = S or G or R or a conservative substitution thereof, X4 = Q or P or a conservative substitution thereof, X5 = S or N or I or R or a conservative substitution thereof, X6 = V or I or F or a conservative substitution thereof, X7 = Y or R or S or N or D or F or H or G or W or a conservative substitution thereof, X8 = S or T or N or G or L or R or a conservative substitution thereof, X9 = S or N or G or R or D or A or Y or a conservative substitution thereof, X10 = Y or F or H or a conservative substitution thereof, X11 = A or V or S or a conservative substitution thereof. |
| | SEQ ID NO: 50128 | VL2 | X1 X2 X3 X4 Arg X5 X6, wherein X1 = G or D or V or a conservative substitution thereof, X2 = A or T or P or V or a conservative substitution thereof, X3 = S or F or Y or A or T or a conservative substitution thereof, X4 = S or R or N or A or a conservative substitution thereof, X5 = A or S or T or a conservative substitution thereof, X6 = T or P or S or A or a conservative substitution thereof. |
| | SEQ ID NO: 50129 | VL3 | X1 X2 X3 X4 X5 X6 X7 X8 X9 X10, wherein X1 = Q or H or a conservative substitution thereof, X2 = Q or H or a conservative substitution thereof, X3 = Y or S or a conservative substitution thereof, X4 = D or E or G or H or a conservative substitution thereof, X5 = N or S or Absent or R or T or I or D or G or a conservative substitution thereof, X6 = Absent or S or a conservative substitution thereof, X7 = S or P or N or R or a conservative substitution thereof, X8 = P or S or V or a |

3416

FIGURE 55

| | | | conservative substitution thereof, X9 = W or R or a conservative substitution thereof, X10 = T or A or a conservative substitution thereof. |
|---|---|---|---|
| VL-CONSENSUS-18 TABLE 98 | SEQ ID NO: 50130 | VL1 | Arg X1 X2 X3 X4 Ile X5 X6 X7 Leu X8, wherein X1 = A or T or a conservative substitution thereof, X2 = S or T or a conservative substitution thereof, X3 = Q or R or a conservative substitution thereof, X4 = G or D or A or N or V or a conservative substitution thereof, X5 = R or G or a conservative substitution thereof, X6 = N or K or D or H or S or G or T or a conservative substitution thereof, X7 = D or I or Y or a conservative substitution thereof, X8 = G or N or a conservative substitution thereof. |
| | SEQ ID NO: 50131 | VL2 | X1 X2 X3 X4 X5 X6 X7, wherein X1 = A or T or I or V or P or G or R or S or a conservative substitution thereof, X2 = A or T or S or a conservative substitution thereof, X3 = S or A or F or P or Y or a conservative substitution thereof, X4 = S or N or R or G or T or a conservative substitution thereof, X5 = L or C or F or S or a conservative substitution thereof, X6 = Q or H or E or a conservative substitution thereof, X7 = S or N or G or I or a conservative substitution thereof. |
| | SEQ ID NO: 50132 | VL3 | X1 X2 X3 X4 X5 X6 X7 X8 X9 Thr, wherein X1 = L or V or I or H or a conservative substitution thereof, X2 = Q or M or H or L or V or a conservative substitution thereof, X3 = H or Q or Y or L or S or a conservative substitution thereof, X4 = Y or N or H or S or T or a conservative substitution thereof, X5 = S or N or T or R or D or F or G or a conservative substitution thereof, X6 = Absent or Y or F or a conservative substitution thereof, X7 = Y or F or P or C or N or T or a conservative substitution thereof, X8 = P or L or a conservative substitution thereof, X9 = R or W or F or L or a conservative substitution thereof. |
| VL-CONSENSUS-19 TABLE 99 | SEQ ID NO: 50133 | VL1 | X1 X2 X3 Gln Ser X4 Leu X5 X6 X7 X8 X9 X10 X11 X12 X13 X14, wherein X1 = K or R or M or a conservative substitution thereof, X2 = S or G or R or a conservative substitution thereof, X3 = S or T or N or I or a conservative substitution thereof, X4 = V or I or A or a conservative substitution thereof, X5 = H or Y or F or S or K or D or L or M or a conservative substitution thereof, X6 = S or T or R or N or I or D or a conservative substitution thereof, X7 = S or F or P or a conservative substitution thereof, X8 = N or H or a conservative substitution thereof, X9 = N or K or S or D or H or a conservative substitution thereof, X10 = N or Y or K or H or R or A or F or W or a conservative substitution thereof, X11 = N or H or Y or a |

FIGURE 55

| | | | |
|---|---|---|---|
| | | | conservative substitution thereof, X12 = Y or S or a conservative substitution thereof, X13 = L or F or a conservative substitution thereof, X14 = A or T or V or G or a conservative substitution thereof. |
| | SEQ ID NO: 50229 | VL2 | Trp X1 X2 X3 X4 X5 X6 wherein X1 = A or T or S or a conservative substitution thereof, X2 = S or F or a conservative substitution thereof, X3 = T or I or K or S or a conservative substitution thereof, X4 = R or W or L or a conservative substitution thereof, X5 = E or K or A or D or R or a conservative substitution thereof, X6 = S or T or a conservative substitution thereof. |
| | SEQ ID NO: 50134 | VL3 | X1 X2 X3 X4 X5 X6 Pro X7 X8, wherein X1 = Q or H or L or a conservative substitution thereof, X2 = Q or H or a conservative substitution thereof, X3 = Y or F or a conservative substitution thereof, X4 = Y or F or H or N or L or S or a conservative substitution thereof, X5 = S or N or R or D or I or T or C or E or K or a conservative substitution thereof, X6 = T or S or I or V or A or Y or a conservative substitution thereof, X7 = P or W or L or V or C or G or R or S or a conservative substitution thereof, X8 = T or K or S or a conservative substitution thereof. |
| VL-CONSENSUS-20 TABLE 100 | SEQ ID NO: 50135 | VL1 | Arg X1 Ser Gln X2 X3 X4 X5 X6 X7 X8, wherein X1 = A or T or a conservative substitution thereof, X2 = G or D or V or a conservative substitution thereof, X3 = I or M or a conservative substitution thereof, X4 = R or S or a conservative substitution thereof, X5 = N or K or S or D or T or a conservative substitution thereof, X6 = D or N or H or Y or V or A or I or L or a conservative substitution thereof, X7 = L or F or a conservative substitution thereof, X8 = G or D or a conservative substitution thereof. |
| | SEQ ID NO: 50136 | VL2 | X1 X2 X3 X4 X5 X6 X7, wherein X1 = A or P or T or G or I or R or V or a conservative substitution thereof, X2 = A or V or a conservative substitution thereof, X3 = S or F or T or a conservative substitution thereof, X4 = S or N or T or D or a conservative substitution thereof, X5 = L or V or a conservative substitution thereof, X6 = Q or L or a conservative substitution thereof, X7 = S or N or T or G or R or a conservative substitution thereof. |
| | SEQ ID NO: 50137 | VL3 | X1 X2 X3 X4 X5 X6 X7 X8 X9, wherein X1 = L or I or a conservative substitution thereof, X2 = Q or H or L or a conservative substitution thereof, X3 = H or Y or D or L or a conservative substitution thereof, X4 = N or Y or T or H or G or I or a conservative substitution thereof, X5 = S or R or D or T or G or N or a conservative substitution thereof, X6 = Y or F or H or a conservative substitution thereof, X7 = P or L or a |

FIGURE 55

| | | | |
|---|---|---|---|
| | | | conservative substitution thereof, X8 = F or L or a conservative substitution thereof, X9 = T or K or a conservative substitution thereof. |
| VL-CONSENSUS-21 TABLE 101 | SEQ ID NO: 50138 | VL1 | Arg X1 X2 Gln X3 Ile X4 X5 X6 Leu X7, wherein X1 = A or T or a conservative substitution thereof, X2 = S or N or a conservative substitution thereof, X3 = G or D or A or V or S or a conservative substitution thereof, X4 = S or G or N or R or A or F or a conservative substitution thereof, X5 = N or K or R or H or S or T or I or a conservative substitution thereof, X6 = Y or H or C or D or a conservative substitution thereof, X7 = A or D or V or I or N or a conservative substitution thereof. |
| | SEQ ID NO: 50139 | VL2 | X1 X2 X3 X4 Leu X5 X6, wherein X1 = A or K or S or T or V or D or G or a conservative substitution thereof, X2 = A or T or V or a conservative substitution thereof, X3 = S or P or a conservative substitution thereof, X4 = S or N or R or a conservative substitution thereof, X5 = Q or L or E or H or a conservative substitution thereof, X6 = S or G or N or T or a conservative substitution thereof. |
| | SEQ ID NO: 50140 | VL3 | X1 X2 X3 X4 X5 X6 Pro X7 X8, wherein X1 = Q or L or H or a conservative substitution thereof, X2 = Q or H or R or Y or a conservative substitution thereof, X3 = Y or S or C or T or a conservative substitution thereof, X4 = S or L or N or M or D or H or V or I or Y or a conservative substitution thereof, X5 = T or N or S or K or a conservative substitution thereof, X6 = Y or F or I or S or a conservative substitution thereof, X7 = L or V or F or N or a conservative substitution thereof, X8 = T or I or Q or S or a conservative substitution thereof. |
| VL-CONSENSUS-22 TABLE 102 | SEQ ID NO: 50141 | VL1 | Arg X1 X2 X3 X4 X5 X6 X7 X8 Leu X9, wherein X1 = A or T or a conservative substitution thereof, X2 = S or T or a conservative substitution thereof, X3 = Q or H or R or a conservative substitution thereof, X4 = N or S or R or T or I or a conservative substitution thereof, X5 = I or V or F or a conservative substitution thereof, X6 = I or S or N or Y or F or R or H or L or K or T or a conservative substitution thereof, X7 = S or N or D or R or K or T or a conservative substitution thereof, X8 = Y or F or a conservative substitution thereof, X9 = N or H or a conservative substitution thereof. |
| | SEQ ID NO: 50142 | VL2 | X1 X2 Ser X3 X4 X5 X6, wherein X1 = T or A or V or G or I or S or a conservative substitution thereof, X2 = A or T or a conservative substitution thereof, X3 = S or N or R or T or a conservative substitution thereof, X4 = L or F or S or a conservative substitution thereof, X5 = Q or H or E or P or a conservative substitution thereof, |

FIGURE 55

| | | | |
|---|---|---|---|
| | | | X6 = S or G or T or N or R or a conservative substitution thereof. |
| | SEQ ID NO: 50143 | VL3 | Gln X1 X2 X3 X4 X5 X6 X7 Thr, wherein X1 = Q or L or a conservative substitution thereof, X2 = S or T or N or P or a conservative substitution thereof, X3 = Y or H or C or D or F or N or a conservative substitution thereof, X4 = S or Absent or I or N or F or G or T or a conservative substitution thereof, X5 = T or S or P or F or N or I or L or a conservative substitution thereof, X6 = P or T or I or A or S or a conservative substitution thereof, X7 = L or P or Y or F or V or a conservative substitution thereof. |
| VL-CONSENSUS-23 TABLE 103 | SEQ ID NO: 50144 | VL1 | X1 X2 X3 X4 X5 X6 X7 X8 X9 Leu X10, wherein X1 = R or P or a conservative substitution thereof, X2 = A or T or a conservative substitution thereof, X3 = S or N or a conservative substitution thereof, X4 = Q or R or a conservative substitution thereof, X5 = G or D or V or A or a conservative substitution thereof, X6 = I or V or a conservative substitution thereof, X7 = S or N or G or R or T or K or a conservative substitution thereof, X8 = N or K or Y or H or I or T or a conservative substitution thereof, X9 = Y or H or a conservative substitution thereof, X10 = A or V or S or a conservative substitution thereof. |
| | SEQ ID NO: 50145 | VL2 | X1 X2 X3 X4 X5 X6 X7, wherein X1 = A or V or G or T or a conservative substitution thereof, X2 = A or S or V or a conservative substitution thereof, X3 = S or F or a conservative substitution thereof, X4 = S or G or N or T or a conservative substitution thereof, X5 = L or V or a conservative substitution thereof, X6 = Q or R or H or L or E or a conservative substitution thereof, X7 = S or G or T or a conservative substitution thereof. |
| | SEQ ID NO: 50146 | VL3 | X1 X2 X3 X4 X5 X6 Pro X7 X8, wherein X1 = Q or H or L or P or R or a conservative substitution thereof, X2 = Q or R or K or H or L or a conservative substitution thereof, X3 = Y or F or a conservative substitution thereof, X4 = N or H or D or Y or S or K or L or M or Q or a conservative substitution thereof, X5 = S or T or G or N or C or D or a conservative substitution thereof, X6 = Y or F or H or a conservative substitution thereof, X7 = F or V or L or I or a conservative substitution thereof, X8 = T or K or a conservative substitution thereof. |
| VL-CONSENSUS-24 TABLE 104 | SEQ ID NO: 50147 | VL1 | Arg X1 X2 X3 X4 X5 X6 X7 X8 X9 X10, wherein X1 = A or V or E or G or a conservative substitution thereof, X2 = S or G or a conservative substitution thereof, X3 = Q or R or a conservative substitution thereof, X4 = G or D or N or A or L or V or a |

**FIGURE 55**

| | | | |
|---|---|---|---|
| | | | conservative substitution thereof, X5 = I or V or F or a conservative substitution thereof, X6 = S or N or T or R or I or a conservative substitution thereof, X7 = R or S or N or K or T or D or I or G or a conservative substitution thereof, X8 = W or Y or a conservative substitution thereof, X9 = L or I or a conservative substitution thereof, 10 = A or T or V or a conservative substitution thereof. |
| | SEQ ID NO: 50148 | VL2 | X1 X2 X3 X4 X5 X6 X7, wherein X1 = A or G or T or D or V or a conservative substitution thereof, X2 = A or T or V or a conservative substitution thereof, X3 = S or Y or a conservative substitution thereof, X4 = S or R or N or T or G or I or a conservative substitution thereof, X5 = L or F or a conservative substitution thereof, X6 = Q or E or a conservative substitution thereof, X7 = S or G or N or D or a conservative substitution thereof. |
| | SEQ ID NO: 50149 | VL3 | X1 Gln X2 X3 X4 X5 Pro X6 Thr, wherein X1 = Q or H or a conservative substitution thereof, X2 = A or T or G or S or V or D or a conservative substitution thereof, X3 = N or D or K or S or a conservative substitution thereof, X4 = S or I or a conservative substitution thereof, X5 = F or L or I or V or a conservative substitution thereof, X6 = F or I or a conservative substitution thereof. |
| VL-CONSENSUS-25 TABLE 105 | SEQ ID NO: 50150 | VL1 | X1 Ser X2 X3 X4 Leu X5 X6 X7 X8 Gly X9 Thr X10 X11 X12, wherein X1 = K or M or R or T or a conservative substitution thereof, X2 = S or G or T or a conservative substitution thereof, X3 = Q or K or a conservative substitution thereof, X4 = S or R or N or T or a conservative substitution thereof, X5 = L or R or V or a conservative substitution thereof, X6 = H or Y or a conservative substitution thereof, X7 = G or S or a conservative substitution thereof, X8 = D or E or G or a conservative substitution thereof, X9 = K or R or a conservative substitution thereof, X10 = Y or F or a conservative substitution thereof, X11 = L or F or a conservative substitution thereof, X12 = Y or F or T or C or S or a conservative substitution thereof. |
| | SEQ ID NO: 50151 | VL2 | X1 X2 Ser X3 Arg X4 X5, wherein X1 = E or A or a conservative substitution thereof, X2 = V or I or L or T or a conservative substitution thereof, X3 = N or K or H or I or S or a conservative substitution thereof, X4 = F or L or a conservative substitution thereof, X5 = S or A or T or C or P or a conservative substitution thereof. |
| | SEQ ID NO: 50152 | VL3 | X1 Gln X2 X3 X4 X5 Pro X6 Thr, wherein X1 = M or K or a conservative substitution thereof, X2 = S or T or a conservative substitution thereof, X3 = I or T or F or L or a conservative substitution thereof, X4 = Q or H or a conservative substitution thereof, X5 = L or I or V or |

FIGURE 55

| | | | |
|---|---|---|---|
| | | | F or a conservative substitution thereof, X6 = W or R or a conservative substitution thereof. |
| VL-CONSENSUS-26 TABLE 106 | SEQ ID NO: 50153 | VL1 | Arg X1 X2 X3 X4 X5 X6 X7 X8 X9 X10, wherein X1 = A or S or T or a conservative substitution thereof, X2 = S or G or I or a conservative substitution thereof, X3 = Q or H or R or a conservative substitution thereof, X4 = S or N or T or a conservative substitution thereof, X5 = I or F or a conservative substitution thereof, X6 = S or I or F or N or R or Y or T or A or G or L or a conservative substitution thereof, X7 = S or N or T or H or K or a conservative substitution thereof, X8 = Y or F or H or a conservative substitution thereof, X9 = L or V or a conservative substitution thereof, X10 = N or I or M or H or Y or a conservative substitution thereof. |
| | SEQ ID NO: 50154 | VL2 | X1 X2 X3 X4 Leu X5 X6, wherein X1 = A or G or T or V or I or a conservative substitution thereof, X2 = A or T or V or S or a conservative substitution thereof, X3 = S or F or a conservative substitution thereof, X4 = S or N or T or V or a conservative substitution thereof, X5 = Q or H or a conservative substitution thereof, X6 = S or N or G or H or I or T or a conservative substitution thereof. |
| | SEQ ID NO: 50155 | VL3 | Gln Gln X1 X2 X3 X4 X5 X6 Phe X7, wherein X1 = S or T or Y or a conservative substitution thereof, X2 = Y or N or F or H or a conservative substitution thereof, X3 = S or R or N or F or I or a conservative substitution thereof, X4 = Absent or A or S or a conservative substitution thereof, X5 = P or T or I or F or A or L or V or a conservative substitution thereof, X6 = P or L or F or S or a conservative substitution thereof, X7 = T or A or S or a conservative substitution thereof. |
| VL-CONSENSUS-27 TABLE 107 | SEQ ID NO: 50156 | VL1 | X1 Ser X2 Gln X3 X4 Leu X5 X6 Ser X7 X8 X9 X10 X11 Leu X12, wherein X1 = K or R or T or a conservative substitution thereof, X2 = S or I or a conservative substitution thereof, X3 = S or N or a conservative substitution thereof, X4 = V or I or a conservative substitution thereof, X5 = Y or H or S or F or a conservative substitution thereof, X6 = S or N or I or R or H or a conservative substitution thereof, X7 = N or H or a conservative substitution thereof, X8 = N or S or D or a conservative substitution thereof, X9 = N or Y or K or H or A or M or Q or a conservative substitution thereof, X10 = N or K or a conservative substitution thereof, X11 = Y or F or a conservative substitution thereof, X12 = A or T or D or a conservative substitution thereof. |
| | SEQ ID NO: 50157 | VL2 | Trp X1 X2 X3 Arg X4 X5, wherein X1 = A or T or G or S or a conservative substitution thereof, X2 = S or F |

FIGURE 55

| | | | |
|---|---|---|---|
| | | | or a conservative substitution thereof, X3 = T or I or a conservative substitution thereof, X4 = E or K or D or a conservative substitution thereof, X5 = S or F or a conservative substitution thereof. |
| | SEQ ID NO: 50158 | VL3 | X1 X2 X3 X4 X5 X6 X7 X8 X9, wherein X1 = Q or H or a conservative substitution thereof, X2 = Q or H or a conservative substitution thereof, X3 = Y or S or a conservative substitution thereof, X4 = Y or F or K or N or a conservative substitution thereof, X5 = S or T or I or Absent or N or a conservative substitution thereof, X6 = T or S or I or R or A or G or N or a conservative substitution thereof, X7 = P or S or a conservative substitution thereof, X8 = C or Y or G or L or P or a conservative substitution thereof, X9 = S or K or N or a conservative substitution thereof. |
| VL-CONSENSUS-28 TABLE 108 | SEQ ID NO: 50159 | VL1 | Arg Ala X1 Gln X2 X3 X4 X5 X6 Leu Ala, wherein X1 = S or N or a conservative substitution thereof, X2 = G or D or F or N or V or a conservative substitution thereof, X3 = I or L or V or a conservative substitution thereof, X4 = S or N or T or I or F or G or a conservative substitution thereof, X5 = N or S or D or T or R or a conservative substitution thereof, X6 = W or C or a conservative substitution thereof. |
| | SEQ ID NO: 50160 | VL2 | X1 X2 X3 X4 Leu Gln X5, wherein X1 = A or G or D or T or S or a conservative substitution thereof, X2 = A or V or P or T or a conservative substitution thereof, X3 = S or F or a conservative substitution thereof, X4 = S or N or a conservative substitution thereof, X5 = S or G or N or a conservative substitution thereof. |
| | SEQ ID NO: 50161 | VL3 | X1 Gln X2 X3 Ser X4 Pro X5 Thr, wherein X1 = Q or L or a conservative substitution thereof, X2 = A or T or S or G or V or Y or a conservative substitution thereof, X3 = N or D or H or Y or a conservative substitution thereof, X4 = F or L or a conservative substitution thereof, X5 = W or R or P or a conservative substitution thereof. |
| VL-CONSENSUS-29 TABLE 109 | SEQ ID NO: 50162 | VL1 | Lys Ser X1 Gln X2 X3 X4 X5 X6 Ser X7 X8 X9 X10 Tyr Leu X11, wherein X1 = S or N or a conservative substitution thereof, X2 = S or R or N or a conservative substitution thereof, X3 = V or I or L or a conservative substitution thereof, X4 = L or F or a conservative substitution thereof, X5 = H or F or Y or K or S or a conservative substitution thereof, X6 = S or N or H or a conservative substitution thereof, X7 = N or H or a conservative substitution thereof, X8 = N or S or a conservative substitution thereof, X9 = N or K or Y or H or a conservative substitution thereof, X10 = N or R |

FIGURE 55

| | | | |
|---|---|---|---|
| | | | or S or a conservative substitution thereof, X11 = A or T or V or a conservative substitution thereof. |
| | SEQ ID NO: 50163 | VL2 | Trp Ala Ser X1 X2 X3 Ser, wherein X1 = T or A or I or S or a conservative substitution thereof, X2 = R or L or a conservative substitution thereof, X3 = E or K or D or a conservative substitution thereof. |
| | SEQ ID NO: 50164 | VL3 | X1 Gln X2 X3 X4 X5 Pro X6 Thr, wherein X1 = Q or H or a conservative substitution thereof, X2 = Y or S or a conservative substitution thereof, X3 = Y or C or F or S or H or a conservative substitution thereof, X4 = S or N or Q or D or T or a conservative substitution thereof, X5 = T or L or I or A or F or S or a conservative substitution thereof, X6 = V or F or P or a conservative substitution thereof. |
| VL-CONSENSUS-30 TABLE 110 | SEQ ID NO: 50165 | VL1 | X1 X2 X3 Thr X4 X5 Val Thr Ser X6 X7 X8 Pro X9, wherein X1 = A or V or G or a conservative substitution thereof, X2 = S or F or L or a conservative substitution thereof, X3 = S or N or a conservative substitution thereof, X4 = G or E or a conservative substitution thereof, X5 = A or S or T or a conservative substitution thereof, X6 = G or A or a conservative substitution thereof, X7 = Y or S or N or F or a conservative substitution thereof, X8 = Y or F or a conservative substitution thereof, X9 = N or S or Q or a conservative substitution thereof. |
| | SEQ ID NO: 50166 | VL2 | X1 Thr X2 Asn X3 His Ser, wherein X1 = S or H or N or a conservative substitution thereof, X2 = S or N or D or I or T or a conservative substitution thereof, X3 = K or R or a conservative substitution thereof. |
| | SEQ ID NO: 50167 | VL3 | X1 X2 X3 X4 X5 Gly X6 X7 X8 X9, wherein X1 = L or M or a conservative substitution thereof, X2 = L or I or F or a conservative substitution thereof, X3 = Y or F or a conservative substitution thereof, X4 = Y or C or F or S or a conservative substitution thereof, X5 = G or D or a conservative substitution thereof, X6 = A or V or a conservative substitution thereof, X7 = Q or H or a conservative substitution thereof, X8 = L or V or M or a conservative substitution thereof, X9 = V or A or I or G or M or a conservative substitution thereof. |
| VL-CONSENSUS-31 TABLE 111 | SEQ ID NO: 50168 | VL1 | Arg X1 Ser X2 X3 X4 X5 X6 X7 Leu Ala, wherein X1 = A or S or a conservative substitution thereof, X2 = Q or L or M or V or a conservative substitution thereof, X3 = S or N or D or R or T or a conservative substitution thereof, X4 = V or I or a conservative substitution thereof, X5 = N or K or S or V or A or I or L or a conservative substitution thereof, X6 = S or N or T or a conservative substitution thereof, X7 = N or S or Y or a conservative substitution thereof. |

**FIGURE 55**

|  | SEQ ID NO: 50169 | VL2 | X1 X2 Ser X3 Arg Ala Thr, wherein X1 = G or I or F or V or a conservative substitution thereof, X2 = A or T or a conservative substitution thereof, X3 = T or I or a conservative substitution thereof. |
|  | SEQ ID NO: 50170 | VL3 | Gln X1 X2 X3 X4 X5 X6 X7 Cys Ser, wherein X1 = Q or E or a conservative substitution thereof, X2 = Y or F or a conservative substitution thereof, X3 = N or Y or D or a conservative substitution thereof, X4 = D or N or a conservative substitution thereof, X5 = Absent or W or a conservative substitution thereof, X6 = W or P or a conservative substitution thereof, X7 = P or L or M or a conservative substitution thereof. |
| VL-CONSENSUS-32 TABLE 112 | SEQ ID NO: 50171 | VL1 | Arg X1 Ser X2 X3 X4 X5 X6 X7 X8 Leu X9, wherein X1 = A or S or a conservative substitution thereof, X2 = Q or E or a conservative substitution thereof, X3 = S or R or a conservative substitution thereof, X4 = V or I or a conservative substitution thereof, X5 = S or T or a conservative substitution thereof, X6 = S or T or a conservative substitution thereof, X7 = S or N or a conservative substitution thereof, X8 = Y or A or a conservative substitution thereof, X9 = V or S or a conservative substitution thereof. |
|  | SEQ ID NO: 50172 | VL2 | Gly Ala Ser X1 Arg Ala X2, wherein X1 = T or S or a conservative substitution thereof, X2 = T or S or I or a conservative substitution thereof. |
|  | SEQ ID NO: 50173 | VL3 | Gln Gln Tyr X1 X2 Ser X3 Leu Thr wherein X1 = G or V or a conservative substitution thereof, X2 = C or N or S or a conservative substitution thereof, X3 = P or L or a conservative substitution thereof. |
| VL-CONSENSUS-33 TABLE 113 | SEQ ID NO: 50174 | VL1 | Lys Ser Ser Gln X1 Leu X2 X3 X4 X5 Gly X6 Thr X7 Leu X8, wherein X1 = S or T or a conservative substitution thereof, X2 = L or Q or a conservative substitution thereof, X3 = H or R or a conservative substitution thereof, X4 = S or G or a conservative substitution thereof, X5 = E or D or a conservative substitution thereof, X6 = K or R or a conservative substitution thereof, X7 = Y or H or F or a conservative substitution thereof, X8 = Y or N or a conservative substitution thereof. |
|  | SEQ ID NO: 50175 | VL2 | Glu X1 Ser X2 Arg X3 Ser, wherein X1 = V or I or a conservative substitution thereof, X2 = N or Y or H or a conservative substitution thereof, X3 = F or I or V or L or a conservative substitution thereof. |
|  | SEQ ID NO: 50176 | VL3 | X1 X2 X3 X4 X5 X6 X7 X8 X9, wherein X1 = M or F or a conservative substitution thereof, X2 = Q or H or a conservative substitution thereof, X3 = S or G or N or a conservative substitution thereof, X4 = I or K or T or a conservative substitution thereof, X5 = Q or Absent or |

**FIGURE 55**

| | | | |
|---|---|---|---|
| | | | K or H or a conservative substitution thereof, X6 = L or Q or Y or F or H or a conservative substitution thereof, X7 = P or L or V or a conservative substitution thereof, X8 = L or F or P or a conservative substitution thereof, X9 = T or P or S or a conservative substitution thereof. |
| VL-CONSENSUS-34 TABLE 114 | SEQ ID NO: 50177 | VL1 | Thr Gly Thr Ser Ser Asp X1 Gly X2 Tyr Asn X3 Val Ser, wherein X1 = V or I or a conservative substitution thereof, X2 = G or S or a conservative substitution thereof, X3 = Y or F or a conservative substitution thereof. |
| | SEQ ID NO: 50178 | VL2 | Glu Val X1 Asn Arg Pro Ser, wherein X1 = S or R or a conservative substitution thereof. |
| | SEQ ID NO: 50179 | VL3 | X1 Ser Tyr X2 X3 X4 X5 Thr Trp Val, wherein X1 = N or G or C or S or a conservative substitution thereof, X2 = T or V or K or a conservative substitution thereof, X3 = R or S or K or a conservative substitution thereof, X4 = S or G or N or R or a conservative substitution thereof, X5 = I or S or Y or a conservative substitution thereof. |
| VL-CONSENSUS-35 TABLE 115 | SEQ ID NO: 50180 | VL1 | Arg X1 Ser X2 X3 Ile X4 X5 X6 Leu Asn, wherein X1 = A or S or a conservative substitution thereof, X2 = Q or H or R or a conservative substitution thereof, X3 = S or N or T or H or a conservative substitution thereof, X4 = S or N or G or T or a conservative substitution thereof, X5 = N or S or R or a conservative substitution thereof, X6 = Y or F or a conservative substitution thereof. |
| | SEQ ID NO: 50181 | VL2 | X1 X2 X3 X4 Leu X5 X6, wherein X1 = A or T or S or V or a conservative substitution thereof, X2 = A or T or E or V or a conservative substitution thereof, X3 = S or L or a conservative substitution thereof, X4 = S or N or a conservative substitution thereof, X5 = Q or H or a conservative substitution thereof, X6 = S or I or a conservative substitution thereof. |
| | SEQ ID NO: 50182 | VL3 | Gln Gln X1 Tyr X2 X3 X4 X5 Trp Thr, wherein X1 = S or G or T or a conservative substitution thereof, X2 = S or T or N or R or a conservative substitution thereof, X3 = T or S or Absent or a conservative substitution thereof, X4 = P or I or a conservative substitution thereof, X5 = T or P or Q or L or a conservative substitution thereof. |
| VL-CONSENSUS-36 TABLE 116 | SEQ ID NO: 50183 | VL1 | Arg Ala Ser Gln X1 Ile Ser X2 X3 Leu Ala, wherein X11 = G or D or a conservative substitution thereof, X2 = S or N or I or K or a conservative substitution thereof, X3 = W or Y or a conservative substitution thereof. |
| | SEQ ID NO: 50184 | VL2 | Ala Ala Ser Ser Leu Gln X1, wherein X1 = S or G or a conservative substitution thereof. |

FIGURE 55

| | SEQ ID NO: 50185 | VL3 | Gln Gln X1 X2 Ser Phe Pro Leu Thr, wherein X1 = I or T or V or A or G or a conservative substitution thereof, X2 = N or K or a conservative substitution thereof. |
|---|---|---|---|
| VL-CONSENSUS-37 TABLE 117 | SEQ ID NO: 50186 | VL1 | Gln Ala X1 Gln X2 Ile X3 X4 X5 Leu Asn, wherein X1 = S or N or a conservative substitution thereof, X2 = D or Y or a conservative substitution thereof, X3 = N or S or T or F or Y or a conservative substitution thereof, X4 = N or D or a conservative substitution thereof, X5 = Y or F or a conservative substitution thereof. |
| | SEQ ID NO: 50187 | VL2 | Asp X1 Ser X2 Leu Glu Thr wherein X1 = A or G or a conservative substitution thereof, X2 = N or T or D or S or a conservative substitution thereof. |
| | SEQ ID NO: 50188 | VL3 | Gln Gln X1 X2 X3 X4 X5 Ile Thr, wherein X1 = Y or F or a conservative substitution thereof, X2 = D or E or a conservative substitution thereof, X3 = N or Absent or I or a conservative substitution thereof, X4 = L or N or V or a conservative substitution thereof, X5 = P or L or a conservative substitution thereof. |
| VL-CONSENSUS-38 TABLE 118 | SEQ ID NO: 50189 | VL1 | Ser Gly Ser Ser Ser Asn X1 Gly X2 X3 X4 X5 Ser, wherein X1 = I or L or a conservative substitution thereof, X2 = N or S or a conservative substitution thereof, X3 = N or H or K or a conservative substitution thereof, X4 = Y or F or a conservative substitution thereof, X5 = V or L or a conservative substitution thereof. |
| | SEQ ID NO: 50190 | VL2 | Asp X1 X2 Lys Arg Pro Ser, wherein X1 = N or S or a conservative substitution thereof, X2 = N or Y or S or a conservative substitution thereof. |
| | SEQ ID NO: 50191 | VL3 | Gly X1 Trp Asp X2 X3 Leu X4 X5 X6 Val, wherein X1 = T or A or I or a conservative substitution thereof, X2 = S or G or I or R or a conservative substitution thereof, X3 = S or R or a conservative substitution thereof, X4 = S or N or a conservative substitution thereof, X5 = V or A or T or a conservative substitution thereof, X6 = G or V or M or a conservative substitution thereof. |
| VL-CONSENSUS-39 TABLE 119 | SEQ ID NO: 50243 | VL1 | Arg X1 Ser Gln Ser Leu X2 X3 X4 X5 X6 X7 Asn X8 Leu Asp, wherein X1 = S or Y or a conservative substitution thereof, X2 = L or V or a conservative substitution thereof, X3 = H or Y or a conservative substitution thereof, X4 = S or N or H or a conservative substitution thereof, X5 = N or S or a conservative substitution thereof, X6 = G or K or R or a conservative substitution thereof, X7 = Y or H or N or a conservative substitution thereof, X8 = Y or H or S or a conservative substitution thereof. |

FIGURE 55

| | SEQ ID NO: 50244 | VL2 | X1 Gly Ser X2 Arg Ala Ser, wherein X1 = L or V or a conservative substitution thereof, X2 = N or H or a conservative substitution thereof. |
|---|---|---|---|
| | SEQ ID NO: 50245 | VL3 | Met Gln X1 Leu X2 X3 X4 X5 X6 Thr, wherein X1 = A or P or T or V or a conservative substitution thereof, X2 = H or Q or Absent or a conservative substitution thereof, X3 = Absent or T or a conservative substitution thereof, X4 = P or Q or T or I or a conservative substitution thereof, X5 = P or T or a conservative substitution thereof, X6 = L or P or F or a conservative substitution thereof. |
| VL-CONSENSUS-40 TABLE 120 | SEQ ID NO: 50192 | VL1 | Ser Gly X1 X2 Ser X3 Ile Gly X4 X5 X6 X7 X8, wherein X1 = S or T or a conservative substitution thereof, X2 = S or N or a conservative substitution thereof, X3 = N or Y or a conservative substitution thereof, X4 = S or N or a conservative substitution thereof, X5 = N or Y or a conservative substitution thereof, X6 = T or A or S or a conservative substitution thereof, X7 = V or I or a conservative substitution thereof, X8 = N or D or S or a conservative substitution thereof. |
| | SEQ ID NO: 50193 | VL2 | X1 X2 X3 X4 Arg Pro Ser, wherein X1 = S or I or a conservative substitution thereof, X2 = N or S or a conservative substitution thereof, X3 = N or D or S or a conservative substitution thereof, X4 = Q or H or a conservative substitution thereof. |
| | SEQ ID NO: 50194 | VL3 | X1 Ala Trp Asp Asp X2 X3 X4 X5 X6 X7 X8, wherein X1 = A or E or a conservative substitution thereof, X2 = S or Absent or a conservative substitution thereof, X3 = Absent or L or a conservative substitution thereof, X4 = L or N or M or S or a conservative substitution thereof, X5 = N or G or K or L or a conservative substitution thereof, X6 = G or H or N or a conservative substitution thereof, X7 = V or P or G or a conservative substitution thereof, X8 = V or P or a conservative substitution thereof. |
| VL-CONSENSUS-41 TABLE 121 | SEQ ID NO: 50195 | VL1 | Ser Gly Ser X1 Ser Asn Ile Gly X2 X3 X4 Val X5, wherein X1 = S or N or C or a conservative substitution thereof, X2 = S or N or a conservative substitution thereof, X3 = N or H or a conservative substitution thereof, X4 = I or T or a conservative substitution thereof, X5 = T or N or a conservative substitution thereof. |
| | SEQ ID NO: 50196 | VL2 | X1 Asn X2 Gln Arg Pro Ser, wherein X1 = S or G or N or V or a conservative substitution thereof, X2 = D or K or N or Y or a conservative substitution thereof. |
| | SEQ ID NO: 50197 | VL3 | X1 X2 Trp Asp Asp Ser Leu X3 X4 Trp Val, wherein X1 = A or T or a conservative substitution thereof, X2 = |

3428

**FIGURE 55**

| | | | |
|---|---|---|---|
| | | | A or T or V or a conservative substitution thereof, X3 = N or I or S or a conservative substitution thereof, X4 = G or D or V or a conservative substitution thereof. |
| VL-CONSENSUS-42 TABLE 122 | SEQ ID NO: 50198 | VL1 | Arg X1 Ser Gln X2 X3 Arg X4 Asp Leu Gly, wherein X1 = A or T or a conservative substitution thereof, X2 = G or D or R or a conservative substitution thereof, X3 = I or V or a conservative substitution thereof, X4 = N or S or a conservative substitution thereof. |
| | SEQ ID NO: 50199 | VL2 | X1 Ala Ser X2 Leu X3 Ser, wherein X1 = A or D or I or T or a conservative substitution thereof, X2 = S or N or a conservative substitution thereof, X3 = Q or E or F or L or a conservative substitution thereof. |
| | SEQ ID NO: 50200 | VL3 | X1 X2 X3 X4 X5 X6 X7 Pro X8 Thr wherein, X1 = L or I or a conservative substitution thereof, X2 = Q or H or a conservative substitution thereof, X3 = H or Y or a conservative substitution thereof, X4 = N or H or S or a conservative substitution thereof, X5 = S or N or a conservative substitution thereof, X6 = Absent or Y or a conservative substitution thereof, X7 = Y or L or F or P or a conservative substitution thereof, X8 = I or P or L or a conservative substitution thereof. |
| VL-CONSENSUS-43 TABLE 123 | SEQ ID NO: 50201 | VL1 | Arg X1 Ser Gln X2 X3 X4 X5 Tyr X6 X7, wherein X1 = A or T or a conservative substitution thereof, X2 = S or N or Y or a conservative substitution thereof, X3 = I or S or F or a conservative substitution thereof, X4 = S or F or N or R or T or a conservative substitution thereof, X5 = S or D or G or R or a conservative substitution thereof, X6 = L or S or a conservative substitution thereof, X7 = N or S or a conservative substitution thereof. |
| | SEQ ID NO: 50202 | VL2 | X1 X2 X3 X4 X5 X6 X7, wherein X1 = A or D or G or S or a conservative substitution thereof, X2 = A or T or a conservative substitution thereof, X3 = S or Y or a conservative substitution thereof, X4 = S or T or a conservative substitution thereof, X5 = L or F or a conservative substitution thereof, X6 = Q or E or K or a conservative substitution thereof, X7 = S or T or a conservative substitution thereof. |
| | SEQ ID NO: 50203 | VL3 | X1 X2 X3 X4 X5 X6 X7 X8 X9, wherein X1 = Q or H or a conservative substitution thereof, X2 = Q or E or a conservative substitution thereof, X3 = S or T or a conservative substitution thereof, X4 = Y or F or a conservative substitution thereof, X5 = S or G or N or a conservative substitution thereof, X6 = I or T or L or N or S or a conservative substitution thereof, X7 = P or S or R or T or a conservative substitution thereof, X8 = I or F or P or a conservative substitution thereof, X9 = T or A or a conservative substitution thereof. |

FIGURE 55

| VL-CONSENSUS-44 TABLE 124 | SEQ ID NO: 50204 | VL1 | X1 X2 X3 Gln X4 X5 X6 His X7 X8 Gly X9 Thr Tyr Leu Tyr, wherein X1 = K or R or a conservative substitution thereof, X2 = S or T or a conservative substitution thereof, X3 = S or N or a conservative substitution thereof, X4 = S or I or a conservative substitution thereof, X5 = L or F or a conservative substitution thereof, X6 = L or V or a conservative substitution thereof, X7 = S or N or R or a conservative substitution thereof, X8 = E or D or a conservative substitution thereof, X9 = K or R or a conservative substitution thereof. |
|---|---|---|---|
| | SEQ ID NO: 50205 | VL2 | Glu X1 Ser X2 Arg X3 Ser, wherein X1 = V or L or a conservative substitution thereof, X2 = N or K or H or a conservative substitution thereof, X3 = F or L or V or a conservative substitution thereof. |
| | SEQ ID NO: 50246 | VL3 | X1 Gln Ser X2 X3 X4 X5 Ile X6, wherein X1 = M or I or L or a conservative substitution thereof, X2 = I or M or a conservative substitution thereof, X3 = Q or Absent or L or a conservative substitution thereof, X4 = L or Y or I or Q or a conservative substitution thereof, X5 = P or L or a conservative substitution thereof, X6 = T or I or a conservative substitution thereof. |
| VL-CONSENSUS-45 TABLE 125 | SEQ ID NO: 50206 | VL1 | Arg Ala Ser X1 X2 X3 X4 X5 X6 X7 Leu Ala, wherein X1 = Q or R or a conservative substitution thereof, X2 = S or G or N or a conservative substitution thereof, X3 = V or I or a conservative substitution thereof, X4 = I or S or G or a conservative substitution thereof, X5 = S or N or a conservative substitution thereof, X6 = S or I or N or a conservative substitution thereof, X7 = Y or F or a conservative substitution thereof. |
| | SEQ ID NO: 50207 | VL2 | Gly X1 Ser X2 X3 Ala Thr, wherein X1 = V or A or T or a conservative substitution thereof, X2 = S or N or T or a conservative substitution thereof, X3 = R or W or a conservative substitution thereof. |
| | SEQ ID NO: 50208 | VL3 | X1 X2 X3 X4 X5 Ser X6 X7 Asn, wherein X1 = Q or H or a conservative substitution thereof, X2 = Q or H or a conservative substitution thereof, X3 = Y or N or a conservative substitution thereof, X4 = G or D or a conservative substitution thereof, X5 = R or Absent or N or Y or a conservative substitution thereof, X6 = P or L or M or a conservative substitution thereof, X7 = N or T or a conservative substitution thereof. |
| VL-CONSENSUS-46 TABLE 126 | SEQ ID NO: 50209 | VL1 | Arg X1 Ser Gln X2 X3 X4 X5 X6 X7 Ala, wherein X1 = A or S or a conservative substitution thereof, X2 = S or D or T or a conservative substitution thereof, X3 = V or I or a conservative substitution thereof, X4 = S or N or R or I or a conservative substitution thereof, X5 = S |

FIGURE 55

| | | | |
|---|---|---|---|
| | | | or I or T or a conservative substitution thereof, X6 = N or Y or a conservative substitution thereof, X7 = L or I or a conservative substitution thereof. |
| | SEQ ID NO: 50210 | VL2 | Gly Ala Ser Thr Arg Ala X1, wherein X1 = T or S or a conservative substitution thereof. |
| | SEQ ID NO: 50211 | VL3 | Gln X1 X2 X3 X4 X5 X6 X7 X8 X9, wherein X1 = Q or E or a conservative substitution thereof, X2 = Y or S or a conservative substitution thereof, X3 = N or D or F or H or a conservative substitution thereof, X4 = D or N or Absent or T or a conservative substitution thereof, X5 = Absent or W or a conservative substitution thereof, X6 = W or P or C or N or a conservative substitution thereof, X7 = P or L or W or a conservative substitution thereof, X8 = W or L or C or P or R or a conservative substitution thereof, X9 = T or P or S or a conservative substitution thereof. |
| VL-CONSENSUS-47 TABLE 127 | SEQ ID NO: 50256 | VL1 | Arg Ala Ser Gln X1 Ile Gly X2 X3 Leu His, wherein X1 = S or N or a conservative substitution thereof, X2 = S or N or a conservative substitution thereof, X3 = S or N or T or a conservative substitution thereof. |
| | SEQ ID NO: 50257 | VL2 | X1 Ala Ser Gln Ser Phe Ser, wherein X1 = Y or S or a conservative substitution thereof. |
| | SEQ ID NO: 50258 | VL3 | X1 Gln Ser X2 Ser X3 Pro X4 Thr, wherein X1 = H or Q or a conservative substitution thereof, X2 = S or G or R or a conservative substitution thereof, X3 = L or F or a conservative substitution thereof, X4 = W or R or Q or a conservative substitution thereof. |
| VL-CONSENSUS-48 TABLE 128 | SEQ ID NO: 50247 | VL1 | Arg Ala Ser Gln X1 Ile X2 X3 Asp Leu Gly, wherein X1 = G or A or a conservative substitution thereof, X2 = R or G or a conservative substitution thereof, X3 = N or D or a conservative substitution thereof. |
| | SEQ ID NO: 50248 | VL2 | Ala X1 Ser Ser Leu Gln Ser, wherein X1 = A or T or a conservative substitution thereof. |
| | SEQ ID NO: 50249 | VL3 | Leu Gln His X1 X2 X3 Pro X4 Ser, wherein X1 = Y or N or a conservative substitution thereof, X2 = S or N or a conservative substitution thereof, X3 = Y or F or a conservative substitution thereof, X4 = R or Y or a conservative substitution thereof. |
| VL-CONSENSUS-49 TABLE 129 | SEQ ID NO: 50212 | VL1 | X1 Ala Ser Gln X2 Ile X3 X4 X5 Leu X6, wherein X1 = R or Q or a conservative substitution thereof, X2 = G or D or a conservative substitution thereof, X3 = S or N or a conservative substitution thereof, X4 = N or K or a conservative substitution thereof, X5 = Y or F or a conservative substitution thereof, X6 = A or N or V or a conservative substitution thereof. |

**FIGURE 55**

| | | | |
|---|---|---|---|
| | SEQ ID NO: 50213 | VL2 | X1 Ala X2 X3 Leu X4 X5, wherein X1 = A or D or G or T or a conservative substitution thereof, X2 = S or T or a conservative substitution thereof, X3 = S or R or N or a conservative substitution thereof, X4 = Q or H or L or V or a conservative substitution thereof, X5 = S or T or a conservative substitution thereof. |
| | SEQ ID NO: 50214 | VL3 | X1 X2 Tyr X3 X4 X5 Pro X6 Thr, wherein X1 = Q or H or L or a conservative substitution thereof, X2 = Q or H or L or a conservative substitution thereof, X3 = L or H or D or K or N or Y or a conservative substitution thereof, X4 = S or N or H or T or a conservative substitution thereof, X5 = Y or L or a conservative substitution thereof, X6 = I or L or a conservative substitution thereof. |
| VL-CONSENSUS-50 TABLE 130 | SEQ ID NO: 50215 | VL1 | X1 Ala X2 Gln Ser X3 Gly Ser Ser Leu His, wherein X1 = S or N or a conservative substitution thereof, X2 = S or N or a conservative substitution thereof, X3 = S or R or a conservative substitution thereof. |
| | SEQ ID NO: 50216 | VL2 | Tyr Ala Ser Gln Ser X1 Ser, wherein X1 = F or L or a conservative substitution thereof. |
| | SEQ ID NO: 50217 | VL3 | His Gln X1 X2 X3 Leu Pro Leu Thr, wherein X1 = S or T or a conservative substitution thereof, X2 = R or G or S or a conservative substitution thereof, X3 = R or S or T or a conservative substitution thereof. |
| VL-CONSENSUS-51 TABLE 131 | SEQ ID NO: 50218 | VL1 | Gln Gly Asp X1 Leu Arg X2 Tyr Tyr X3 X4, wherein X1 = S or T or K or a conservative substitution thereof, X2 = P or N or S or T or a conservative substitution thereof, X3 = A or V or a conservative substitution thereof, X4 = S or N or a conservative substitution thereof. |
| | SEQ ID NO: 50219 | VL2 | X1 Lys Asn X2 Arg Pro Ser, wherein X1 = G or A or T or a conservative substitution thereof, X2 = N or S or a conservative substitution thereof. |
| | SEQ ID NO: 50220 | VL3 | Asn Ser Arg Asp Ser X1 X2 X3 X4 X5 X6 X7, wherein X1 = S or C or a conservative substitution thereof, X2 = G or Absent or a conservative substitution thereof, X3 = N or G or a conservative substitution thereof, X4 = H or N or S or a conservative substitution thereof, X5 = L or H or a conservative substitution thereof, X6 = V or L or a conservative substitution thereof, X7 = V or L or a conservative substitution thereof. |
| VL-CONSENSUS-52 TABLE 132 | (SEQ ID NO: 50221) | VL1 | Arg Ser X1 Gln Ser Leu Val Tyr Ser Asp Gly Asn Thr X2 Leu Asn wherein X1 = S or G or a conservative substitution thereof, X2 = Y or S or a conservative substitution thereof. |
| | SEQ ID NO: 50222 | VL2 | X1 Val Ser X2 Trp Asp X3, wherein X1 = K or E or a conservative substitution thereof, X2 = N or K or a |

FIGURE 55

| | | | |
|---|---|---|---|
| | | | conservative substitution thereof, X3 = S or Y or a conservative substitution thereof. |
| | SEQ ID NO: 50223 | VL3 | Met Gln Gly X1 X2 X3 X4 X5 Thr, wherein X1 = T or I or a conservative substitution thereof, X2 = H or Absent or a conservative substitution thereof, X3 = W or H or a conservative substitution thereof, X4 = P or L or S or W or a conservative substitution thereof, X5 = L or P or a conservative substitution thereof. |
| VL-CONSENSUS-53 TABLE 133 | SEQ ID NO: 50224 | VL1 | Arg X1 Ser Gln Ser X2 X3 X4 X5 X6 Ala, wherein X1 = A or P or T or a conservative substitution thereof, X2 = V or F or a conservative substitution thereof, X3 = S or R or W or a conservative substitution thereof, X4 = R or I or S or a conservative substitution thereof, X5 = N or D or S or a conservative substitution thereof, X6 = L or V or a conservative substitution thereof. |
| | SEQ ID NO: 50225 | VL2 | X1 Ala X2 X3 Arg Ala Thr, wherein X1 = G or D or a conservative substitution thereof, X2 = S or A or a conservative substitution thereof, X3 = T or I or A or a conservative substitution thereof. |
| | SEQ ID NO: 50226 | VL3 | Gln Gln Tyr X1 X2 X3 X4 Pro Leu Thr, wherein X1 = N or Y or a conservative substitution thereof, X2 = N or T or Y or a conservative substitution thereof, X3 = Absent or W or a conservative substitution thereof, X4 = W or P or a conservative substitution thereof. |
| VL-CONSENSUS-54 TABLE 134 | SEQ ID NO: 50250 | VL1 | Gly Gly X1 Asn Ile X2 X3 X4 X5 Val His, wherein X1 = N or D or a conservative substitution thereof, X2 = G or R or a conservative substitution thereof, X3 = R or S or a conservative substitution thereof, X4 = K or R or a conservative substitution thereof, X5 = N or A or a conservative substitution thereof. |
| | SEQ ID NO: 50251 | VL2 | X1 Asp X2 X3 Arg X4 Ser, wherein X1 = R or S or a conservative substitution thereof, X2 = S or R or a conservative substitution thereof, X3 = D or N or Y or a conservative substitution thereof, X4 = P or S or a conservative substitution thereof. |
| | SEQ ID NO: 50252 | VL3 | Gln X1 Trp Asp Ser X2 X3 X4 X5 X6 Val, wherein X1 = V or D or a conservative substitution thereof, X2 = Absent or S or a conservative substitution thereof, X3 = Absent or S or a conservative substitution thereof, X4 = S or D or G or a conservative substitution thereof, X5 = T or H or a conservative substitution thereof, X6 = V or A or G or a conservative substitution thereof. |

FIGURE 56

Table 21

| Reference # | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| | | | | | | | | | | | | | | | | H... |
| CONSENSUS | Q | V | Q | L | V | Q | S | - | G | A | E | V | K | K | P | G |
| 21-225_30A11_HC | | | | | | | | | | | | | | | | |
| 21-225_34F3_HC | | | | | | | | | | | | | | | | |
| 21-225_35C6_HC | | | | | | | | | | T | | | | | | |
| 21-225_36H5_HC | | | | | | | | | | | | | | | | |
| 21-225_35G6_HC | | | | | | | | | | | D | | | | | |
| 21-225_33G1_HC | | | | | A | | | | | | | | | | | |
| 21-225_34G7_HC | | | | | | | | | | | | | | | | |
| 21-225_33H7_HC | | | | | | | | | | | D | | | | | |
| 21-225_35H12_HC | | | | | | | | | | | | | | | | |
| 21-225_4H6_HC | | | | | | | | | | | | | | | | |
| 21-225_18D4_HC | | | | | | | | | | | | | | | | |
| 21-225_23B11_HC | | | | | | | | | | | | | | | | |
| 21-225_31C4_HC | | | | | | | | | | T | | | | | | |

| Reference # | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|
| :R1 | | | | | | | | | | | | | | | | |
| CONSENSUS | A | S | V | K | V | S | C | K | A | S | G | - | Y | T | F | T |
| 21-225_30A11_HC | | | | | | | | | | | | | | | | |
| 21-225_34F3_HC | | | | | | | | | | | | | | | | |
| 21-225_35C6_HC | | | | | | | | | | | | | | | | |
| 21-225_36H5_HC | | | M | | | | | | | | | | | | | |
| 21-225_35G6_HC | | | | | | | | | | | | | | | | |
| 21-225_33G1_HC | | | | | | | | | | | | | | | | |
| 21-225_34G7_HC | | | | | | | | R | | | | | | | | |
| 21-225_33H7_HC | | | | | | | | | | | | | | | | |
| 21-225_35H12_HC | | | | | | | | | | | | | | | | |
| 21-225_4H6_HC | | | | | | | | | | | | | | | | |
| 21-225_18D4_HC | | | | | | | | | | | | | | | | |
| 21-225_23B11_HC | | | | | | | | | | | | | | | | |
| 21-225_31C4_HC | | | | | | | | | | | | | | | | |

| Reference # | 33 | 34 | 35 | 36 | 37 | 38 | 39 | 40 | 41 | 42 | 43 | 44 | 45 | 46 | 47 | 48 |
|---|---|---|---|---|---|---|---|---|---|---|---|---|---|---|---|---|
| | | | | | H_CDR1 | | | | | | | | | | | |
| CONSENSUS | D | - | - | - | - | - | Y | Y | M | H | W | V | R | Q | A | P |
| 21-225_30A11_HC | | | | | | | | H | | | | | | | | |
| 21-225_34F3_HC | G | | | | | | | H | | | | | | | | |
| 21-225_35C6_HC | G | | | | | | D | | | | | | | | | |
| 21-225_36H5_HC | | | | | | | | H | | | | | | | | |
| 21-225_35G6_HC | | | | | | | H | | I | | | | | | | |
| 21-225_33G1_HC | G | | | | | | | | | | | | | | | |
| 21-225_34G7_HC | | | | | | | H | | I | | | | | | | |
| 21-225_33H7_HC | | | | | | | H | | I | | | | | | | |
| 21-225_35H12_HC | | | | | | | | | I | | | | | | | |
| 21-225_4H6_HC | | | | | | | | | L | | | | | | | |
| 21-225_18D4_HC | G | | | | | | | | L | | | | | | | |
| 21-225_23B11_HC | G | | | | | | | | L | | | | | | | |
| 21-225_31C4_HC | G | | | | | | D | | | | | | | | | |

FIGURE 56

| Reference # | 49 | 50 | 51 | 52 | 53 | 54 | 55 | 56 | 57 | 58 | 59 | 60 | 61 | 62 | 63 | 64 |
|---|---|---|---|---|---|---|---|---|---|---|---|---|---|---|---|---|
| | H_FR2 | | | | | | | | | | | | | | | |
| CONSENSUS | G | Q | G | L | E | W | M | G | W | I | N | P | N | - | - | - |
| 21-225_30A11_HC | . | . | . | . | . | . | . | . | . | . | . | . | . | . | . | . |
| 21-225_34F3_HC | . | . | . | . | D | . | . | . | . | . | . | . | . | . | . | . |
| 21-225_35C6_HC | . | . | . | . | . | . | . | . | . | . | S | . | K | . | . | . |
| 21-225_36H5_HC | . | . | . | . | . | . | . | . | . | . | . | . | . | . | . | . |
| 21-225_35G6_HC | . | . | . | . | . | . | . | . | . | . | . | . | . | . | . | . |
| 21-225_33G1_HC | . | . | . | . | . | . | . | . | . | . | S | . | . | . | . | . |
| 21-225_34G7_HC | . | . | . | . | . | . | . | . | . | . | . | . | . | . | . | . |
| 21-225_33H7_HC | . | . | . | . | . | . | . | . | . | . | . | . | . | . | . | . |
| 21-225_35H12_HC | . | . | . | . | . | . | . | . | . | . | . | . | . | . | . | . |
| 21-225_4H6_HC | . | . | . | . | . | . | . | . | . | . | H | . | . | . | . | . |
| 21-225_18D4_HC | . | . | . | . | . | . | L | . | . | . | . | . | . | . | . | . |
| 21-225_23B11_HC | . | . | . | . | . | . | . | . | . | . | . | . | . | . | . | . |
| 21-225_31C4_HC | . | . | . | . | . | . | . | . | . | . | S | . | K | . | . | . |

| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| | H_CDR2 | | | | | | | | | | | | | | | |
| CONSENSUS | N | G | G | T | N | Y | A | Q | K | F | Q | G | R | V | T | M |
| 21-225_30A11_HC | R | . | . | . | . | . | . | . | . | . | . | D | . | . | . | . |
| 21-225_34F3_HC | S | . | . | . | . | . | . | . | . | . | . | . | . | . | . | . |
| 21-225_35C6_HC | . | . | . | . | . | . | . | . | . | . | . | . | . | . | . | . |
| 21-225_36H5_HC | R | . | . | . | . | . | . | . | . | . | . | D | . | . | . | . |
| 21-225_35G6_HC | . | . | . | . | H | . | . | . | E | . | . | . | . | . | . | . |
| 21-225_33G1_HC | . | . | . | . | . | . | . | . | . | . | . | . | . | . | . | . |
| 21-225_34G7_HC | . | . | . | . | H | . | . | . | . | . | . | . | . | . | . | . |
| 21-225_33H7_HC | . | . | . | . | H | . | . | . | E | . | . | . | . | . | . | . |
| 21-225_35H12_HC | R | . | . | . | . | . | . | . | . | . | . | . | . | . | . | . |
| 21-225_4H6_HC | S | . | . | . | . | . | . | . | . | . | . | . | . | . | . | . |
| 21-225_18D4_HC | S | N | . | . | I | S | . | . | . | . | . | . | . | . | . | . |
| 21-225_23B11_HC | . | N | . | . | . | . | . | . | . | . | . | . | . | . | . | . |
| 21-225_31C4_HC | . | . | . | . | . | . | . | . | . | . | . | . | . | . | . | . |

| Reference # | 81 | 82 | 83 | 84 | 85 | 86 | 87 | 88 | 89 | 90 | 91 | 92 | 93 | 94 | 95 | 96 |
|---|---|---|---|---|---|---|---|---|---|---|---|---|---|---|---|---|
| | | | | | | | | | | | | H_FR3 | | | | |
| CONSENSUS | T | R | D | T | S | I | S | T | A | Y | M | E | L | S | R | L |
| 21-225_30A11_HC | . | . | . | . | . | . | . | . | . | . | . | . | . | . | . | . |
| 21-225_34F3_HC | . | . | . | . | . | . | . | . | . | . | . | . | . | . | . | . |
| 21-225_35C6_HC | . | . | . | . | . | . | . | . | . | . | . | . | . | N | . | . |
| 21-225_36H5_HC | . | . | . | . | . | . | . | . | . | . | . | . | . | . | . | . |
| 21-225_35G6_HC | . | . | . | . | . | . | . | . | . | . | . | . | . | . | S | . |
| 21-225_33G1_HC | . | . | . | . | . | . | . | . | . | . | . | . | . | . | . | . |
| 21-225_34G7_HC | . | . | . | . | . | . | . | . | . | . | . | . | . | . | . | . |
| 21-225_33H7_HC | . | . | . | . | . | . | . | . | . | . | . | . | . | . | S | . |
| 21-225_35H12_HC | . | . | . | . | . | . | . | . | . | . | . | . | . | . | . | . |
| 21-225_4H6_HC | . | . | . | . | . | . | . | . | . | . | G | . | . | S | . | . |
| 21-225_18D4_HC | . | . | . | . | . | . | . | . | . | . | . | . | . | . | . | . |
| 21-225_23B11_HC | . | . | . | . | . | . | . | . | . | . | . | . | . | . | . | . |
| 21-225_31C4_HC | . | . | . | . | . | . | . | V | . | . | . | . | . | N | . | . |

**FIGURE 56**

| Reference # | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|
| CONSENSUS | R | S | D | D | T | A | V | Y | Y | C | A | R | D | G | T | G |
| 21-225_30A11_HC | . | . | . | . | . | . | . | . | F | . | . | . | | | . | . |
| 21-225_34F3_HC | . | . | . | . | . | . | . | . | . | . | . | . | | | . | S |
| 21-225_35C6_HC | . | . | . | . | . | . | . | . | . | . | . | . | | | . | . |
| 21-225_36H5_HC | . | . | . | . | . | . | . | . | F | . | . | . | | | . | . |
| 21-225_35G6_HC | . | . | . | . | . | . | . | . | . | . | . | . | | | . | . |
| 21-225_33G1_HC | . | . | . | . | . | . | . | . | . | . | . | . | | | . | . |
| 21-225_34G7_HC | . | . | . | . | . | . | . | . | . | . | . | . | | | . | . |
| 21-225_33H7_HC | . | . | . | . | . | . | . | . | H | . | . | . | | | . | . |
| 21-225_35H12_HC | . | . | . | . | . | . | . | . | . | . | . | . | | | . | . |
| 21-225_4H6_HC | . | . | . | . | . | . | . | . | . | . | . | . | | | . | S |
| 21-225_18D4_HC | I | . | . | . | . | . | . | . | . | . | . | . | | | . | S |
| 21-225_23B11_HC | . | . | . | . | . | . | . | . | . | . | . | . | | | . | S |
| 21-225_31C4_HC | . | . | . | . | . | . | . | . | . | . | . | . | | | . | . |

| Reference # | 113 | 114 | 115 | 116 | 117 | 118 | 119 | 120 | 121 | 122 | 123 | 124 | 125 | 126 | 127 | 128 |
|---|---|---|---|---|---|---|---|---|---|---|---|---|---|---|---|---|
| | | | | | | | | | | | | H_CDR3 | | | | |
| CONSENSUS | - | - | - | - | - | - | - | - | - | - | - | - | - | - | - | - |
| 21-225_30A11_HC | . | . | . | . | . | . | . | . | . | . | . | . | . | . | . | . |
| 21-225_34F3_HC | . | . | . | . | . | . | . | . | . | . | . | . | . | . | . | . |
| 21-225_35C6_HC | . | . | . | . | . | . | . | . | . | . | . | . | . | . | . | . |
| 21-225_36H5_HC | . | . | . | . | . | . | . | . | . | . | . | . | . | . | . | . |
| 21-225_35G6_HC | . | . | . | . | . | . | . | . | . | . | . | . | . | . | . | . |
| 21-225_33G1_HC | . | . | . | . | . | . | . | . | . | . | . | . | . | . | . | . |
| 21-225_34G7_HC | . | . | . | . | . | . | . | . | . | . | . | . | . | . | . | . |
| 21-225_33H7_HC | . | . | . | . | . | . | . | . | . | . | . | . | . | . | . | . |
| 21-225_35H12_HC | . | . | . | . | . | . | . | . | . | . | . | . | . | . | . | . |
| 21-225_4H6_HC | . | . | . | . | . | . | . | . | . | . | . | . | . | . | . | . |
| 21-225_18D4_HC | . | . | . | . | . | . | . | . | . | . | . | . | . | . | . | . |
| 21-225_23B11_HC | . | . | . | . | . | . | . | . | . | . | . | . | . | . | . | . |
| 21-225_31C4_HC | . | . | . | . | . | . | . | . | . | . | . | . | . | . | . | . |

| Reference # | 129 | 130 | 131 | 132 | 133 | 134 | 135 | 136 | 137 | 138 | 139 | 140 | 141 | 142 | 143 | 144 |
|---|---|---|---|---|---|---|---|---|---|---|---|---|---|---|---|---|
| | | | | | | | | | | | | | | | | H_FR |
| CONSENSUS | - | - | - | - | - | - | S | F | D | Y | W | G | Q | G | T | L |
| 21-225_30A11_HC | . | . | . | . | . | . | . | . | . | . | . | . | . | . | . | . |
| 21-225_34F3_HC | . | . | . | . | . | . | . | . | . | . | . | . | . | . | . | . |
| 21-225_35C6_HC | . | . | . | . | . | . | . | . | . | . | . | . | . | . | . | . |
| 21-225_36H5_HC | . | . | . | . | . | . | . | . | . | . | . | . | . | . | . | . |
| 21-225_35G6_HC | . | . | . | . | . | . | . | . | . | . | . | . | . | . | . | . |
| 21-225_33G1_HC | . | . | . | . | . | . | . | . | . | . | . | . | . | . | . | . |
| 21-225_34G7_HC | . | . | . | . | . | . | . | . | . | . | . | . | . | N | . | |
| 21-225_33H7_HC | . | . | . | . | . | . | . | . | . | . | . | . | . | . | . | . |
| 21-225_35H12_HC | . | . | . | . | . | . | . | . | . | . | . | . | . | . | . | . |
| 21-225_4H6_HC | . | . | . | . | . | . | . | . | . | . | . | . | . | . | . | . |
| 21-225_18D4_HC | . | . | . | . | . | . | L | . | . | . | . | . | . | . | . | . |
| 21-225_23B11_HC | . | . | . | . | . | . | . | . | . | . | . | . | . | . | . | . |
| 21-225_31C4_HC | . | . | . | . | . | . | . | . | . | . | . | . | . | . | . | . |

FIGURE 56

| Reference # | 145 | 146 | 147 | 148 | 149 |
|---|---|---|---|---|---|
| CONSENSUS | V | T | V | S | S |
| 21-225_30A11_HC | . | . | . | . | . |
| 21-225_34F3_HC | . | . | . | . | . |
| 21-225_35C6_HC | . | . | . | . | . |
| 21-225_36H5_HC | . | . | . | . | . |
| 21-225_35G6_HC | . | . | . | . | . |
| 21-225_33G1_HC | . | . | . | . | . |
| 21-225_34G7_HC | . | . | . | . | . |
| 21-225_33H7_HC | . | . | . | . | . |
| 21-225_35H12_HC | . | . | . | . | . |
| 21-225_4H6_HC | . | . | . | . | . |
| 21-225_18D4_HC | . | . | . | . | . |
| 21-225_23B11_HC | . | . | . | . | . |
| 21-225_31C4_HC | . | . | . | . | . |

FIGURE 56

Table 22

| Reference # | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| | | | | | | | | | | | | | | | | H_J |
| CONSENSUS | Q | V | Q | L | V | Q | S | - | G | A | E | V | K | K | P | G |
| 21-225_31D12_HC | . | . | K | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | K | . | . | . | . | . | . | . | . | . | . | . | . | . |
| 21-225_13F6_HC | . | . | . | . | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | K | . | . | . | . | . | . | . | . | . | . | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | . | . | . | . | . | . | . | . |

| Reference # | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|
| FR1 | | | | | | | | | | | | | | | | |
| CONSENSUS | A | S | V | K | V | S | C | K | A | S | G | - | Y | T | F | T |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | . | . | . | . |
| 21-225_13F6_HC | . | . | . | . | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | . | . | . | . | . | . | . | . |

| Reference # | 33 | 34 | 35 | 36 | 37 | 38 | 39 | 40 | 41 | 42 | 43 | 44 | 45 | 46 | 47 | 48 |
|---|---|---|---|---|---|---|---|---|---|---|---|---|---|---|---|---|
| | | | | | H_CDR1 | | | | | | | | | | | |
| CONSENSUS | G | - | - | - | - | - | Y | Y | M | H | W | V | R | Q | A | P |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | . | . | . | . |
| 21-225_13F6_HC | . | . | . | . | . | . | . | . | . | . | . | . | . | . | . | . |
| 21-225_16F3_HC | . | . | . | . | . | . | H | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | . | . | . | . | I | . | . | . | . | . | . | . |
| 21-225_19D1_HC | . | . | . | . | . | . | . | . | . | . | . | . | . | . | . | . |
| 21-225_21D12_HC | D | . | . | . | . | . | . | . | I | . | . | . | . | . | . | . |

FIGURE 56

| Reference # | 49 | 50 | 51 | 52 | 53 | 54 | 55 | 56 | 57 | 58 | 59 | 60 | 61 | 62 | 63 | 64 |
|---|---|---|---|---|---|---|---|---|---|---|---|---|---|---|---|---|
| H_FR2 | | | | | | | | | | | | | | | | |
| CONSENSUS | G | Q | G | L | E | W | M | G | W | I | N | P | N | - | - | - |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | K | . | . | . |
| 21-225_13F6_HC | . | . | . | . | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | . | . | . | . | . | . | . | . |

| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| H_CDR2 | | | | | | | | | | | | | | | | |
| CONSENSUS | S | G | G | T | N | Y | A | Q | K | F | Q | G | R | V | T | M |
| 21-225_31D12_HC | . | . | . | . | . | . | V | . | . | . | . | . | . | . | . | . |
| 21-225_18E10_HC | . | . | . | . | . | . | . | . | . | . | . | . | . | . | . | . |
| 21-225_4B3_HC | . | . | . | . | . | S | . | . | . | . | . | . | . | . | . | . |
| 21-225_35E1_HC | . | . | . | . | . | . | . | . | . | . | . | . | . | . | . | . |
| 21-225_13F6_HC | . | . | . | A | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | D | . | . | . | . | . | . | . | . | . | . | . |
| 21-225_19D1_HC | . | . | . | . | . | . | . | . | . | . | L | . | . | . | . | . |
| 21-225_21D12_HC | . | . | . | . | N | . | . | . | . | . | . | . | . | . | . | . |

| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| H_CDR2 | | | | | | | | | | | | | | | | |
| CONSENSUS | S | G | G | T | N | Y | A | Q | K | F | Q | G | R | V | T | M |
| 21-225_31D12_HC | . | . | . | . | . | . | V | . | . | . | . | . | . | . | . | . |
| 21-225_18E10_HC | . | . | . | . | . | . | . | . | . | . | . | . | . | . | . | . |
| 21-225_4B3_HC | . | . | . | . | . | S | . | . | . | . | . | . | . | . | . | . |
| 21-225_35E1_HC | . | . | . | . | . | . | . | . | . | . | . | . | . | . | . | . |
| 21-225_13F6_HC | . | . | . | A | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | D | . | . | . | . | . | . | . | . | . | . | . |
| 21-225_19D1_HC | . | . | . | . | . | . | . | . | . | . | L | . | . | . | . | . |
| 21-225_21D12_HC | . | . | . | . | N | . | . | . | . | . | . | . | . | . | . | . |

FIGURE 56

| Reference # | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|
| CONSENSUS | R | S | D | D | T | A | V | Y | Y | C | A | R | S | Y | Y | Y |
| 21-225_31D12_HC | . | . | . | . | . | . | . | . | . | . | . | . | V | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | V | . | . | H |
| 21-225_13F6_HC | . | . | . | . | . | . | . | . | F | . | . | . | . | F | . | . |
| 21-225_16F3_HC | . | . | . | . | . | . | . | . | . | . | . | . | . | . | . | . |
| 21-225_17D3_HC | . | . | . | . | . | . | . | . | . | . | . | . | A | N | . | . |
| 21-225_33E6_HC | . | . | . | . | . | . | . | . | . | . | . | . | V | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | S | . | . | . | . | . | . | . |

| Reference # | 113 | 114 | 115 | 116 | 117 | 118 | 119 | 120 | 121 | 122 | 123 | 124 | 125 | 126 | 127 | 128 |
|---|---|---|---|---|---|---|---|---|---|---|---|---|---|---|---|---|
| | | | | | | | | | | | H_CDR3 | | | | | |
| CONSENSUS | G | S | G | S | - | - | - | - | - | - | - | - | - | - | - | - |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | . | . | . | . |
| 21-225_13F6_HC | . | . | . | T | . | . | . | . | . | . | . | . | . | . | . | . |
| 21-225_16F3_HC | . | . | . | T | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | T | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | T | . | . | . | . | . | . | . | . | . | . | . | . |

| Reference # | 129 | 130 | 131 | 132 | 133 | 134 | 135 | 136 | 137 | 138 | 139 | 140 | 141 | 142 | 143 | 144 |
|---|---|---|---|---|---|---|---|---|---|---|---|---|---|---|---|---|
| | | | | | | | | | | | | | | | | H_FR |
| CONSENSUS | - | - | - | Y | Y | N | E | F | D | Y | W | G | Q | G | T | L |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | . | . | . | . |
| 21-225_13F6_HC | . | . | . | . | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | D | . | . | . | . | . | . | . | . | . |
| 21-225_33E6_HC | . | . | . | . | . | . | . | . | . | . | . | . | . | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | . | . | . | . | . | . | . | . |

**FIGURE 56**

| Reference # | 145 | 146 | 147 | 148 | 149 |
|---|---|---|---|---|---|
| 4 | | | | | |
| CONSENSUS | V | T | V | S | S |
| 21-225_31D12_HC | . | . | . | . | . |
| 21-225_18E10_HC | . | . | . | . | . |
| 21-225_4B3_HC | . | . | . | . | . |
| 21-225_35E1_HC | . | . | . | . | . |
| 21-225_13F6_HC | . | . | . | . | . |
| 21-225_16F3_HC | . | . | . | . | . |
| 21-225_17D3_HC | . | . | . | . | . |
| 21-225_33E6_HC | . | . | . | . | . |
| 21-225_9G9_HC | . | . | . | . | . |
| 21-225_19D1_HC | . | . | . | . | . |
| 21-225_21D12_HC | . | . | . | . | . |

FIGURE 56

Table 23

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | V | Q | L | V | Q | S | - | G | A | E | V | K | K | P | G | A |
| 21-225_24E1_HC | . | . | . | . | . | . | . | . | . | . | . | . | . | . | . | . | . |
| 21-225_23A12_HC | . | . | . | . | . | . | . | . | . | . | . | . | . | . | . | . | . |
| 21-225_25A4_HC | . | . | L | . | . | . | . | . | . | . | . | . | . | R | . | . | . |
| 21-225_28B6_HC | . | . | L | . | . | . | . | . | . | . | . | . | . | R | . | . | . |
| 21-225_29E6_HC | . | . | L | . | . | . | . | . | . | . | . | . | . | R | . | . | . |
| 21-225_26C4_HC | . | . | . | . | . | . | . | . | . | . | . | . | . | . | . | . | . |
| 21-225_33D1_HC | . | . | . | . | . | . | . | . | . | . | . | . | . | . | . | . | . |
| 21-225_34D2_HC | . | . | . | . | . | . | . | . | . | . | . | . | . | . | . | . | . |
| 21-225_29H8_HC | . | . | . | . | . | . | . | . | . | . | . | . | Q | . | . | . | . |
| 21-225_11E5_HC | . | . | . | . | . | . | . | . | . | P | . | . | . | . | . | . | . |
| 21-225_8B11_HC | . | . | . | . | . | . | . | . | . | . | . | . | . | . | . | . | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | V | K | V | S | C | K | A | S | G | - | Y | T | F | T | N | - |
| 21-225_24E1_HC | . | . | . | . | . | . | . | . | . | . | . | . | . | . | . | . | . |
| 21-225_23A12_HC | . | . | . | . | . | . | . | . | . | . | . | . | P | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | . | . | . | . | . | . | . | . | . | . |
| 21-225_33D1_HC | . | . | . | . | . | . | . | . | . | . | . | . | . | . | . | . | . |
| 21-225_34D2_HC | . | . | . | . | . | . | . | . | . | . | . | . | . | . | . | . | . |
| 21-225_29H8_HC | . | . | . | . | . | . | . | . | . | . | . | . | . | . | . | . | . |
| 21-225_11E5_HC | . | . | . | . | . | . | . | . | . | . | . | . | . | . | . | . | . |
| 21-225_8B11_HC | . | . | . | . | . | . | . | . | . | . | . | . | . | . | . | . | . |
| 21-225_22D2_HC | . | . | . | . | . | . | . | . | . | . | . | . | . | . | I | . | . |
| 21-225_32A1_HC | . | . | . | . | . | . | . | . | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | | | | | | | H_FR2 | | |
| CONSENSUS | - | - | - | - | Y | D | I | N | W | V | R | Q | A | T | G | Q | G |
| 21-225_24E1_HC | . | . | . | . | . | . | . | . | . | . | . | . | . | . | . | . | . |
| 21-225_23A12_HC | . | . | . | . | . | . | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | . | . | . | . | . | . | S | . | . | . |
| 21-225_33D1_HC | . | . | . | . | . | . | . | . | . | . | . | . | . | . | . | . | . |
| 21-225_34D2_HC | . | . | . | . | . | . | . | . | . | . | . | . | . | . | . | . | . |
| 21-225_29H8_HC | . | . | . | . | . | . | . | . | . | . | . | . | . | . | . | . | . |
| 21-225_11E5_HC | . | . | . | . | . | . | . | . | . | . | . | . | . | . | . | . | . |
| 21-225_8B11_HC | . | . | . | . | . | . | . | . | . | . | . | . | . | A | . | . | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | M | G | W | M | H | P | N | - | - | - | S | G | N | T |
| 21-225_24E1_HC | . | . | . | . | . | . | . | . | . | . | . | . | . | . | . | . | . |
| 21-225_23A12_HC | . | . | . | . | . | . | . | . | . | . | . | . | . | . | . | . | . |
| 21-225_25A4_HC | . | . | . | . | . | . | . | Y | . | . | . | . | . | . | . | . | . |
| 21-225_28B6_HC | . | . | . | . | . | . | . | . | . | D | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | N | . | . | . | . | . | . | . | . | . |
| 21-225_33D1_HC | . | . | . | . | . | . | . | . | . | . | . | . | . | . | . | . | . |
| 21-225_34D2_HC | . | . | V | . | . | . | . | L | . | . | . | . | . | . | . | T | . |
| 21-225_29H8_HC | . | . | . | . | . | . | . | N | . | . | . | . | . | . | . | . | . |
| 21-225_11E5_HC | . | . | . | . | . | . | . | . | . | . | . | . | . | . | . | A | . |
| 21-225_8B11_HC | . | . | . | . | . | . | . | . | . | . | . | . | . | . | . | . | . |
| 21-225_22D2_HC | . | . | . | . | . | . | . | . | . | D | . | . | . | . | . | . | . |
| 21-225_32A1_HC | . | . | . | . | . | . | . | N | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | N | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | G | Y | A | Q | K | F | Q | G | R | V | T | M | T | R | N | T | S |
| 21-225_24E1_HC | . | . | . | . | R | . | . | . | . | . | . | . | . | . | . | . | . |
| 21-225_23A12_HC | . | . | . | . | . | . | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | R | . | . | . | . | . | . | . | . | . | . | . | . |
| 21-225_33D1_HC | . | F | . | . | . | . | R | . | . | . | . | . | . | . | . | . | . |
| 21-225_34D2_HC | . | F | . | . | . | . | R | . | . | . | . | . | . | . | . | . | . |
| 21-225_29H8_HC | . | . | . | . | R | . | . | . | . | . | . | . | . | . | . | . | . |
| 21-225_11E5_HC | . | . | . | . | R | . | . | . | . | . | . | . | . | . | . | . | . |
| 21-225_8B11_HC | . | . | . | . | . | . | . | . | . | . | . | . | . | . | . | . | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | . | . |
| 21-225_32A5_HC | . | F | . | . | . | . | R | . | . | . | . | . | . | . | . | . | . |
| 21-225_34H7_HC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | | H_FR3 | | | | | | | | | | |
| CONSENSUS | I | S | T | A | Y | M | E | L | S | S | L | R | S | E | D | T | A |
| 21-225_24E1_HC | . | . | . | . | . | . | . | . | . | . | . | . | . | . | . | . | . |
| 21-225_23A12_HC | . | N | . | . | . | . | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | N | . | . | . | . | . | . | . | . | . | . | . | . | . | . | . |
| 21-225_33D1_HC | . | . | . | . | . | . | . | . | . | . | . | . | . | . | . | . | . |
| 21-225_34D2_HC | . | . | . | . | . | . | L | . | . | . | . | . | . | . | . | . | . |
| 21-225_29H8_HC | . | . | A | . | . | . | . | . | . | . | . | . | . | . | . | . | . |
| 21-225_11E5_HC | . | . | . | . | . | . | . | . | . | . | . | . | . | . | . | . | . |
| 21-225_8B11_HC | R | . | . | . | . | . | . | . | . | . | . | . | . | . | . | . | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | . | N | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | S | S | S | G | W | Y | - | - | - | - | - | - |
| 21-225_24E1_HC | . | . | . | . | . | T | . | . | . | . | E | . | . | . | . | . | . |
| 21-225_23A12_HC | . | . | . | . | . | G | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | . | . | . | . | . | . | . | . | . | . |
| 21-225_33D1_HC | . | . | . | . | . | . | . | . | . | . | . | . | . | . | . | . | . |
| 21-225_34D2_HC | . | . | . | . | . | . | . | . | . | . | . | . | . | . | . | . | . |
| 21-225_29H8_HC | . | . | . | . | . | . | . | . | . | . | . | . | . | . | . | . | . |
| 21-225_11E5_HC | . | . | . | . | . | T | . | . | . | . | E | . | . | . | . | . | . |
| 21-225_8B11_HC | . | . | . | . | . | Y | . | . | . | . | . | . | . | . | . | . | . |
| 21-225_22D2_HC | . | . | . | . | . | Y | . | . | . | . | . | . | . | . | . | . | . |
| 21-225_32A1_HC | . | . | . | . | . | . | . | . | . | . | . | . | . | . | . | . | . |
| 21-225_32A5_HC | . | . | . | . | S | . | . | . | . | . | . | . | . | . | . | . | . |
| 21-225_34H7_HC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | - | - | Y | F |
| 21-225_24E1_HC | . | . | . | . | . | . | . | . | . | . | . | . | . | . | . | V | . |
| 21-225_23A12_HC | . | . | . | . | . | . | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | . | . | . | . | . | . | . | . | . | . |
| 21-225_33D1_HC | . | . | . | . | . | . | . | . | . | . | . | . | . | . | . | F | . |
| 21-225_34D2_HC | . | . | . | . | . | . | . | . | . | . | . | . | . | . | . | F | . |
| 21-225_29H8_HC | . | . | . | . | . | . | . | . | . | . | . | . | . | . | . | . | . |
| 21-225_11E5_HC | . | . | . | . | . | . | . | . | . | . | . | . | . | . | . | V | . |
| 21-225_8B11_HC | . | . | . | . | . | . | . | . | . | . | . | . | . | . | . | F | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | F | . |
| 21-225_32A5_HC | . | . | . | . | . | . | . | . | . | . | . | . | . | . | . | F | . |
| 21-225_34H7_HC | . | . | . | . | . | . | . | . | . | . | . | . | . | . | . | F | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
|  |  |  |  |  |  |  | H_FR4 |  |  |  |  |  |  |
| CONSENSUS | D | Y | W | G | Q | G | T | L | V | T | V | S | S |
| 21-225_24E1_HC | . | . | . | . | . | . | . | . | . | . | . | . | . |
| 21-225_23A12_HC | . | . | . | . | . | . | . | . | . | . | . | . | . |
| 21-225_25A4_HC | . | . | . | . | . | . | . | . | . | . | . | . | . |
| 21-225_28B6_HC | . | . | . | . | . | . | . | . | . | . | . | . | . |
| 21-225_29E6_HC | . | . | . | . | . | . | . | . | . | . | . | . | . |
| 21-225_26C4_HC | . | . | . | . | . | . | . | . | . | . | . | . | . |
| 21-225_33D1_HC | . | . | . | . | . | . | . | . | . | . | . | . | . |
| 21-225_34D2_HC | . | . | . | . | . | . | . | . | . | . | . | . | . |
| 21-225_29H8_HC | . | . | . | . | . | . | . | . | . | . | . | . | . |
| 21-225_11E5_HC | . | . | . | . | . | . | . | . | . | . | . | . | . |
| 21-225_8B11_HC | . | . | . | . | . | . | . | . | . | . | . | . | . |
| 21-225_22D2_HC | . | . | . | . | . | . | . | . | . | . | . | . | . |
| 21-225_32A1_HC | . | . | . | . | . | . | . | . | . | . | . | . | . |
| 21-225_32A5_HC | . | . | . | . | . | . | . | . | . | . | . | . | . |
| 21-225_34H7_HC | . | . | . | . | . | . | . | . | . | . | . | . | . |

FIGURE 56

Table 24

| Reference # | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
|  |  |  |  |  |  |  |  |  |  |  |  |  |  |  |  | H |
| CONSENSUS | E | V | Q | L | V | E | S | - | G | G | G | L | V | K | P | G |
| 21-225_17H5_HC | . | . | . | . | . | . | . | . | . | . | . | . | . |  | . | . |
| 21-225_16A11_HC | . | . | . | . | . | . | . | . | . | . | . | . | . |  | . | . |
| 21-225_20H7_HC | . | . | **H** | . | . | . | . | . | . | . | . | . | . |  | . | . |
| 21-225_20D12_HC | . | . | . | . | . | . | . | . | . | . | . | . | . |  | . | . |
| 21-225_22B12_HC | . | . | . | . | . | . | . | . | . | . | . | . | . |  | . | . |
| 21-225_27C5_HC | . | . | . | . | . | . | . | . | . | . | . | . | . |  | . | . |
| 21-225_27H12_HC | . | . | . | . | . | . | . | . | . | . | . | . | . |  | . | . |
| 21-225_35H8_HC | . | . | . | . | . | . | . | . | . | . | . | . | . |  | . | . |
| 21-225_15F8_HC | . | . | . | . | . | . | . | . | . | . | . | . | . |  | . | . |
| 21-225_20D6_HC | . | . | . | . | . | . | . | . | . | . | . | . | . |  | . | . |
| 21-225_15A2_HC | . | . | . | . | . | . | . | . | . | . | . | . | . |  | . | . |
| 21-225_5E11_HC | . | . | . | . | . | . | . | . | . | . | . | . | . |  | . | . |
| 21-225_2A4_HC | . | . | . | . | . | . | . | . | . | . | . | . | . |  | . | . |
| 21-225_16B8_HC | . | . | . | . | . | . | . | . | . | . | . | . | . |  | . | . |
| 21-225_14D6_HC | . | . | . | . | . | . | . | . | . | . | . | . | . |  | . | . |
| 21-225_1F1_HC | . | . | . | . | . | . | . | . | . | . | . | . | . |  | . | . |
| 21-225_5F4_HC | . | . | . | . | . | . | . | . | . | . | . | . | . |  | . | . |
| 21-225_16H1_HC | . | . | . | . | . | . | . | . | . | . | . | . | . |  | . | . |
| 21-225_17H6_HC | . | . | . | . | . | . | . | . | . | . | . | . | . |  | . | . |
| 21-225_21F12_HC | . | . | . | . | . | . | . | . | . | . | . | . | . |  | . | . |
| 21-225_23A11_HC | . | . | . | . | . | . | . | . | . | . | . | . | . |  | . | . |
| 21-225_32B3_HC | . | . | . | . | . | . | . | . | . | . | . | . | . |  | . | . |
| 21-225_2C12_HC | . | . | . | . | . | . | . | . | . | . | . | . | . |  | . | . |

**FIGURE 56**

| Reference # | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|
| FR1 | | | | | | | | | | | | | | | | |
| CONSENSUS | G | S | L | R | L | S | C | A | A | S | G | - | F | T | F | S |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | . | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | . | . | . | . | . | . | . | . | . |
| 21-225_27C5_HC | . | . | . | . | . | . | . | . | . | . | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | . | . | . | . | . | . | . | . | . |
| 21-225_15A2_HC | . | . | . | . | . | . | . | . | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | . | . | . | . | . | . | . |
| 21-225_1F1_HC | . | . | . | . | . | . | . | . | . | . | . | . | . | . | . | . |
| 21-225_5F4_HC | . | . | . | . | . | . | . | V | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

**FIGURE 56**

| Reference # | 33 | 34 | 35 | 36 | 37 | 38 | 39 | 40 | 41 | 42 | 43 | 44 | 45 | 46 | 47 | 48 |
|---|---|---|---|---|---|---|---|---|---|---|---|---|---|---|---|---|
| | | | | H_CDR1 | | | | | | | | | | | | |
| CONSENSUS | S | - | - | - | - | - | Y | S | M | N | W | V | R | Q | A | P |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | . | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | . | . | . | . | . | . | . | . | . |
| 21-225_27C5_HC | . | . | . | . | . | . | . | . | . | . | . | . | . | . | . | . |
| 21-225_27H12_HC | . | . | . | . | . | . | . | . | L | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | R | . | . | . | . | . | . | . | . |
| 21-225_15A2_HC | . | . | . | . | . | . | . | T | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | T | . | . | . | . | . | . | . | . |
| 21-225_1F1_HC | . | . | . | . | . | . | . | N | . | . | . | . | . | . | . | . |
| 21-225_5F4_HC | . | . | . | . | . | . | . | . | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | R | . | . | . | . | . | . | . | . |
| 21-225_23A11_HC | . | . | . | . | . | . | . | . | . | . | . | I | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

FIGURE 56

**FIGURE 56**

| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| | H_CDR2 | | | | | | | | | | | | | | | |
| CONSENSUS | S | S | Y | I | Y | Y | A | D | S | V | K | G | R | F | T | I |
| 21-225_17H5_HC | | T | . | . | . | . | . | | | . | . | | . | . | . | . |
| 21-225_16A11_HC | . | . | L | . | . | . | . | | | . | . | | . | . | . | . |
| 21-225_20H7_HC | . | . | F | . | . | . | . | | | . | . | | . | . | . | . |
| 21-225_20D12_HC | . | . | . | K | . | . | . | | | . | . | | . | . | . | . |
| 21-225_22B12_HC | . | . | . | T | . | . | . | | | . | . | | . | . | . | . |
| 21-225_27C5_HC | D | | . | . | . | . | . | | | . | . | | . | . | . | . |
| 21-225_27H12_HC | . | . | N | . | . | . | T | | | . | . | | . | . | . | . |
| 21-225_35H8_HC | G | . | F | . | . | . | . | | | . | . | | . | . | . | . |
| 21-225_15F8_HC | . | . | . | . | . | . | . | | | . | . | | . | . | . | . |
| 21-225_20D6_HC | . | . | N | K | . | . | . | | | . | . | | . | . | . | . |
| 21-225_15A2_HC | G | . | . | . | . | . | . | | | . | . | | . | . | . | . |
| 21-225_5E11_HC | . | T | . | . | . | . | . | | | . | . | | . | . | . | . |
| 21-225_2A4_HC | . | T | . | . | . | . | . | | | . | . | | . | I | A | . |
| 21-225_16B8_HC | . | T | . | . | . | . | . | | | . | . | | . | . | . | . |
| 21-225_14D6_HC | G | . | . | . | . | . | . | | | . | . | | . | . | . | M |
| 21-225_1F1_HC | . | . | . | . | . | . | . | | | . | . | | . | . | . | . |
| 21-225_5F4_HC | . | . | . | . | . | . | . | | | . | . | | . | . | . | . |
| 21-225_16H1_HC | . | T | . | . | . | . | . | | | . | . | | . | . | . | . |
| 21-225_17H6_HC | . | . | . | . | . | . | . | | | . | . | | . | . | . | . |
| 21-225_21F12_HC | . | . | . | . | . | . | . | | | . | . | | . | . | . | . |
| 21-225_23A11_HC | . | T | . | . | . | . | . | | | . | . | | . | I | A | . |
| 21-225_32B3_HC | . | T | . | . | . | . | . | | | . | . | | . | . | . | . |
| 21-225_2C12_HC | . | . | . | M | . | . | . | | | . | . | | . | . | . | . |

**FIGURE 56**

| Reference # | 81 | 82 | 83 | 84 | 85 | 86 | 87 | 88 | 89 | 90 | 91 | 92 | 93 | 94 | 95 | 96 |
|---|---|---|---|---|---|---|---|---|---|---|---|---|---|---|---|---|
| | | | | | | | | | | | | | H_FR3 | | | |
| CONSENSUS | S | R | D | N | A | K | N | S | L | Y | L | Q | M | N | S | L |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | . | . | . | . | . | . | . | . | . |
| 21-225_20D12_HC | . | . | . | . | . | . | . | . | . | L | . | . | . | . | . | . |
| 21-225_22B12_HC | . | . | . | . | . | . | . | . | . | . | . | . | . | . | . | . |
| 21-225_27C5_HC | . | . | . | . | . | . | . | . | . | . | . | . | . | . | . | . |
| 21-225_27H12_HC | . | . | . | . | . | . | . | . | . | F | . | . | . | . | . | . |
| 21-225_35H8_HC | . | . | . | . | . | . | K | . | V | . | . | . | . | . | . | . |
| 21-225_15F8_HC | F | . | . | . | . | . | . | . | . | . | . | . | . | . | . | . |
| 21-225_20D6_HC | . | . | . | . | . | . | . | . | . | . | . | . | . | . | . | . |
| 21-225_15A2_HC | . | . | . | . | . | . | . | . | . | . | . | . | . | . | A | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | . | . | . | . | . | . | . |
| 21-225_1F1_HC | . | . | . | . | . | . | . | . | . | . | . | . | . | . | . | . |
| 21-225_5F4_HC | . | . | . | . | . | . | . | . | . | F | . | . | . | . | . | . |
| 21-225_16H1_HC | . | . | . | . | . | . | . | . | . | . | . | . | . | . | . | . |
| 21-225_17H6_HC | F | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | D | . | . | . | . | . | . | . | . | . |

**FIGURE 56**

| Reference # | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|
| CONSENSUS | R | A | E | D | T | A | V | Y | Y | C | A | R | V | A | S | - |
| 21-225_17H5_HC | . | . | . | . | . | . | . | . | . | . | . | . | . | . | . | . |
| 21-225_16A11_HC | . | . | . | . | . | . | . | . | . | . | . | . | . | . | A | . |
| 21-225_20H7_HC | . | . | . | . | . | . | . | . | . | . | . | . | . | . | A | . |
| 21-225_20D12_HC | . | . | . | . | . | . | . | . | . | . | . | . | . | N | N | . |
| 21-225_22B12_HC | . | . | . | . | . | . | . | . | . | . | . | . | . | . | A | . |
| 21-225_27C5_HC | . | . | . | . | . | . | . | . | . | . | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | S | G | . |
| 21-225_15F8_HC | . | . | . | . | . | . | . | . | . | . | . | . | . | . | . | . |
| 21-225_20D6_HC | . | . | . | . | . | . | . | . | . | . | . | . | . | . | A | . |
| 21-225_15A2_HC | . | . | . | . | . | . | . | . | . | . | . | . | . | . | . | . |
| 21-225_5E11_HC | . | . | . | . | . | . | . | F | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | . | . | . | . | . | A | . |
| 21-225_1F1_HC | . | . | . | . | . | . | . | . | . | . | . | . | . | N | L | . |
| 21-225_5F4_HC | . | . | . | . | . | . | . | . | . | . | . | . | . | N | N | . |
| 21-225_16H1_HC | . | . | . | . | . | . | . | . | . | . | . | . | . | . | A | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | G | . |
| 21-225_2C12_HC | . | . | . | . | . | . | . | . | . | . | . | . | . | N | L | . |

FIGURE 56

| Reference # | 113 | 114 | 115 | 116 | 117 | 118 | 119 | 120 | 121 | 122 | 123 | 124 | 125 | 126 | 127 | 128 |
|---|---|---|---|---|---|---|---|---|---|---|---|---|---|---|---|---|
| | | | | | | | | | | | H_CDR3 | | | | | |
| CONSENSUS | - | - | - | - | - | - | - | - | - | - | - | - | - | - | - | - |
| 21-225_17H5_HC | | | | | | | | | | | | | | | | |
| 21-225_16A11_HC | | | | | | | | | | | | | | | | |
| 21-225_20H7_HC | | | | | | | | | | | | | | | | |
| 21-225_20D12_HC | | | | | | | | | | | | | | | | |
| 21-225_22B12_HC | | | | | | | | | | | | | | | | |
| 21-225_27C5_HC | | | | | | | | | | | | | | | | |
| 21-225_27H12_HC | | | | | | | | | | | | | | | | |
| 21-225_35H8_HC | | | | | | | | | | | | | | | | |
| 21-225_15F8_HC | | | | | | | | | | | | | | | | |
| 21-225_20D6_HC | | | | | | | | | | | | | | | | |
| 21-225_15A2_HC | | | | | | | | | | | | | | | | |
| 21-225_5E11_HC | | | | | | | | | | | | | | | | |
| 21-225_2A4_HC | | | | | | | | | | | | | | | | |
| 21-225_16B8_HC | | | | | | | | | | | | | | | | |
| 21-225_14D6_HC | | | | | | | | | | | | | | | | |
| 21-225_1F1_HC | | | | | | | | | | | | | | | | |
| 21-225_5F4_HC | | | | | | | | | | | | | | | | |
| 21-225_16H1_HC | | | | | | | | | | | | | | | | |
| 21-225_17H6_HC | | | | | | | | | | | | | | | | |
| 21-225_21F12_HC | | | | | | | | | | | | | | | | |
| 21-225_23A11_HC | | | | | | | | | | | | | | | | |
| 21-225_32B3_HC | | | | | | | | | | | | | | | | |
| 21-225_2C12_HC | | | | | | | | | | | | | | | | |

**FIGURE 56**

| Reference # | 129 | 130 | 131 | 132 | 133 | 134 | 135 | 136 | 137 | 138 | 139 | 140 | 141 | 142 | 143 | 144 |
|---|---|---|---|---|---|---|---|---|---|---|---|---|---|---|---|---|
| | | | | | | | | | | | | | | | | H_FR |
| CONSENSUS | - | - | - | - | - | - | - | F | D | Y | W | G | Q | G | T | L |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | L | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | L | | S | . | . | . | . | . | . |
| 21-225_27C5_HC | . | . | . | . | . | . | . | . | . | C | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | L | . | . | . | . | . | . | . | . |
| 21-225_15A2_HC | . | . | . | . | . | . | . | . | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | | S | . | . | . | . | . | . |
| 21-225_1F1_HC | . | . | . | . | . | . | . | . | . | . | . | . | . | . | . | . |
| 21-225_5F4_HC | . | . | . | . | . | . | . | . | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

**FIGURE 56**

| Reference # | 129 | 130 | 131 | 132 | 133 | 134 | 135 | 136 | 137 | 138 | 139 | 140 | 141 | 142 | 143 | 144 H_FR |
|---|---|---|---|---|---|---|---|---|---|---|---|---|---|---|---|---|
| CONSENSUS | - | - | - | - | - | - | - | F | D | Y | W | G | Q | G | T | L |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | L | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | L | . | S | . | . | . | . | . | . |
| 21-225_27C5_HC | . | . | . | . | . | . | . | . | . | C | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | L | . | . | . | . | . | . | . | . |
| 21-225_15A2_HC | . | . | . | . | . | . | . | . | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | S | . | . | . | . | . | . |
| 21-225_1F1_HC | . | . | . | . | . | . | . | . | . | . | . | . | . | . | . | . |
| 21-225_5F4_HC | . | . | . | . | . | . | . | . | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

FIGURE 56

| Reference # | 145 | 146 | 147 | 148 | 149 |
|---|---|---|---|---|---|
| CONSENSUS | V | T | V | S | S |
| 21-225_17H5_HC | . | . | . | . | . |
| 21-225_16A11_HC | . | . | . | . | . |
| 21-225_20H7_HC | . | . | . | . | . |
| 21-225_20D12_HC | . | . | . | . | . |
| 21-225_22B12_HC | . | . | . | . | . |
| 21-225_27C5_HC | . | . | . | . | . |
| 21-225_27H12_HC | . | . | . | . | . |
| 21-225_35H8_HC | . | . | . | . | . |
| 21-225_15F8_HC | . | . | . | . | . |
| 21-225_20D6_HC | . | . | . | . | . |
| 21-225_15A2_HC | . | . | . | . | . |
| 21-225_5E11_HC | . | . | . | . | . |
| 21-225_2A4_HC | . | . | . | . | . |
| 21-225_16B8_HC | . | . | . | . | . |
| 21-225_14D6_HC | . | S | . | . | . |
| 21-225_1F1_HC | . | . | . | . | . |
| 21-225_5F4_HC | . | . | . | . | . |
| 21-225_16H1_HC | . | . | . | . | . |
| 21-225_17H6_HC | . | . | . | . | . |
| 21-225_21F12_HC | . | . | . | . | . |
| 21-225_23A11_HC | . | . | . | . | . |
| 21-225_32B3_HC | . | . | . | . | . |
| 21-225_2C12_HC | . | . | . | . | . |

FIGURE 56

Table 25

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K_FR1 | | | | | |
| CONSENSUS | D | I | Q | M | T | Q | S | P | S | S | L | S | A | S | V | G | D |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | . | . | . | . | . | . | . | . | . |
| 21-225_22B12_LC | . | . | . | . | . | . | . | . | . | . | . | . | . | . | . | . | . |
| 21-225_27C5_LC | . | . | . | . | . | . | . | . | . | . | V | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | F | . | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | F | . | F | I | . | . |
| 21-225_5E11_LC | V | . | . | . | . | . | . | . | . | . | . | . | . | . | . | . | . |
| 21-225_2A4_LC | . | . | . | . | . | . | . | . | . | . | . | F | . | . | . | . | . |
| 21-225_16B8_LC | . | . | . | . | . | . | . | . | . | . | . | . | . | . | . | . | . |
| 21-225_14D6_LC | . | . | . | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | . | . | . | . | . | . | . | . | . | . | . | . | . |
| 21-225_16H1_LC | . | . | . | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | T | . | . | . | . |
| 21-225_32B3_LC | . | . | . | . | . | . | . | . | . | . | . | . | . | . | I | . | . |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | S | - | - |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | S | . | . | . | S | . | . | . | . | . | . | . | D | . | R | . | . |
| 21-225_20H7_LC | . | . | . | . | . | . | . | T | . | . | . | . | . | . | N | . | . |
| 21-225_20D12_LC | . | . | . | . | . | . | . | . | . | . | . | . | D | . | R | . | . |
| 21-225_22B12_LC | . | . | . | . | I | . | . | . | . | . | . | . | D | . | . | . | . |
| 21-225_27C5_LC | . | . | . | . | . | . | . | . | . | . | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | A | . | . | . | . |
| 21-225_15F8_LC | . | . | . | . | . | . | . | . | . | . | . | R | D | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | N | . | . |
| 21-225_14D6_LC | S | . | . | . | . | . | . | . | . | . | . | . | D | . | R | . | . |
| 21-225_1F1_LC | . | . | . | . | . | . | . | . | . | . | . | . | . | . | N | . | . |
| 21-225_5F4_LC | . | . | . | . | . | . | . | . | . | . | . | . | . | . | R | . | . |
| 21-225_16H1_LC | . | . | . | . | . | . | . | . | . | . | . | . | . | . | R | . | . |
| 21-225_17H6_LC | . | . | . | . | . | . | . | . | . | . | . | R | D | . | . | . | . |
| 21-225_21F12_LC | . | . | . | . | . | . | . | . | . | . | . | . | . | . | R | . | . |
| 21-225_23A11_LC | . | . | . | . | . | . | . | . | . | . | . | . | D | . | . | . | . |
| 21-225_32B3_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | K_FR2 | | |
| CONSENSUS | - | - | - | - | N | Y | L | A | W | F | Q | Q | K | P | G | K | A |
| 21-225_2C12_LC | . | . | . | . | . | . | . | . | . | . | . | . | . | S | . | . | . |
| 21-225_17H5_LC | . | . | . | . | . | . | . | . | . | . | . | . | . | . | . | . | . |
| 21-225_16A11_LC | . | . | . | . | . | H | . | G | . | Y | . | R | N | . | . | . | . |
| 21-225_20H7_LC | . | . | . | . | . | . | . | V | . | . | . | . | . | . | . | . | . |
| 21-225_20D12_LC | . | . | . | . | . | H | . | G | . | Y | . | . | . | . | . | . | . |
| 21-225_22B12_LC | . | . | . | . | . | . | . | . | . | . | . | E | . | . | . | . | . |
| 21-225_27C5_LC | . | . | . | . | S | W | . | . | . | Y | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | . | . | . | H | . | G | . | Y | . | H | . | . | . | . | . |
| 21-225_1F1_LC | . | . | . | . | . | . | . | . | . | . | . | . | . | S | . | . | . |
| 21-225_5F4_LC | . | . | . | . | . | H | . | G | . | Y | . | . | . | . | . | . | . |
| 21-225_16H1_LC | . | . | . | . | D | I | . | G | . | Y | . | . | . | . | . | . | . |
| 21-225_17H6_LC | . | . | . | . | . | . | . | . | . | . | . | . | . | . | . | . | . |
| 21-225_21F12_LC | . | . | . | . | . | F | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | S | L | I | Y | A | - | - | - | - | - | - | - | - | A | S |
| 21-225_2C12_LC | . | . | . | . | . | S | . | . | . | . | . | . | . | . | . | . | T |
| 21-225_17H5_LC | . | . | . | . | . | N | . | . | . | . | . | . | . | . | . | . | . |
| 21-225_16A11_LC | . | . | R | . | . | . | . | . | . | . | . | . | . | . | . | . | . |
| 21-225_20H7_LC | . | . | R | . | . | . | . | . | . | . | . | . | . | . | . | . | . |
| 21-225_20D12_LC | . | . | R | . | . | . | . | . | . | . | . | . | . | . | . | . | . |
| 21-225_22B12_LC | H | . | . | . | . | . | G | . | . | . | . | . | . | . | . | . | . |
| 21-225_27C5_LC | . | . | F | . | . | . | G | . | . | . | . | . | . | . | . | . | . |
| 21-225_27H12_LC | . | . | . | . | . | N | G | . | . | . | . | . | . | . | . | . | . |
| 21-225_35H8_LC | . | . | . | . | . | . | G | . | . | . | . | . | . | . | . | . | . |
| 21-225_15F8_LC | . | . | . | . | . | C | S | . | . | . | . | . | . | . | . | T | . |
| 21-225_20D6_LC | . | . | . | . | . | S | . | . | . | . | . | . | . | . | . | . | . |
| 21-225_15A2_LC | . | . | . | . | . | F | . | . | . | . | . | . | . | . | . | . | . |
| 21-225_5E11_LC | . | . | . | . | . | N | . | . | . | . | . | . | . | . | . | . | . |
| 21-225_2A4_LC | . | . | R | . | . | . | T | . | . | . | . | . | . | . | . | . | . |
| 21-225_16B8_LC | L | . | . | . | . | N | . | . | . | . | . | . | . | . | . | . | . |
| 21-225_14D6_LC | . | . | R | . | . | . | . | . | . | . | . | . | . | . | . | . | . |
| 21-225_1F1_LC | . | . | . | . | . | S | V | . | . | . | . | . | . | . | . | . | . |
| 21-225_5F4_LC | . | . | R | . | . | . | . | . | . | . | . | . | . | . | . | . | . |
| 21-225_16H1_LC | . | . | R | . | . | . | . | . | . | . | . | . | . | . | . | . | . |
| 21-225_17H6_LC | . | . | . | . | . | S | T | . | . | . | . | . | . | . | . | . | . |
| 21-225_21F12_LC | . | . | . | . | . | S | . | . | . | . | . | . | . | . | . | . | . |
| 21-225_23A11_LC | . | . | R | . | . | . | . | . | . | . | . | . | . | . | . | . | . |
| 21-225_32B3_LC | . | R | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | K | F | S | G | S | G | S | G | - |
| 21-225_2C12_LC | . | . | . | . | . | . | . | . | Q | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | R | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | R | . | . | . | . | . | . | . | . |
| 21-225_22B12_LC | . | . | R | . | . | . | . | . | . | . | . | . | . | . | . | . | . |
| 21-225_27C5_LC | . | . | . | . | . | . | . | . | R | . | . | A | . | . | . | . | . |
| 21-225_27H12_LC | . | . | . | . | . | . | . | . | . | . | . | . | . | . | . | . | . |
| 21-225_35H8_LC | G | . | . | . | . | . | . | . | . | . | . | . | . | . | . | . | . |
| 21-225_15F8_LC | . | . | . | . | . | . | . | . | . | . | . | . | . | . | F | . | . |
| 21-225_20D6_LC | N | . | . | . | . | . | . | . | . | . | . | . | . | . | . | . | . |
| 21-225_15A2_LC | . | . | . | . | . | . | . | . | . | . | . | . | . | . | F | . | . |
| 21-225_5E11_LC | . | V | . | . | . | . | . | . | . | . | . | . | N | . | . | . | . |
| 21-225_2A4_LC | . | . | . | . | . | . | . | . | . | . | . | . | . | . | F | . | . |
| 21-225_16B8_LC | . | V | . | . | . | . | . | . | . | . | . | . | . | . | . | . | . |
| 21-225_14D6_LC | . | . | . | . | . | . | . | . | R | . | . | . | . | . | . | . | . |
| 21-225_1F1_LC | . | . | . | T | . | . | . | . | R | . | . | . | . | . | . | . | . |
| 21-225_5F4_LC | . | . | . | . | . | . | . | . | R | . | . | . | . | . | . | . | . |
| 21-225_16H1_LC | . | . | . | N | . | . | . | . | R | . | . | . | . | . | . | . | . |
| 21-225_17H6_LC | . | . | . | . | . | . | . | . | . | . | . | . | . | . | . | . | . |
| 21-225_21F12_LC | . | . | . | T | . | . | . | . | . | . | . | . | . | . | . | . | . |
| 21-225_23A11_LC | T | . | . | . | . | . | . | . | . | . | . | . | . | . | . | . | . |
| 21-225_32B3_LC | . | . | . | . | . | . | . | . | T | . | . | . | . | . | . | . | . |

3462

**FIGURE 56**

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | K_FR3 | | | | | | | | | | | | |
| CONSENSUS | - | T | D | F | T | L | T | I | S | S | L | Q | P | E | D | F | A |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | E | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | E | . | . | . | . | . | . | . | . | . | . | . | . | . | . |
| 21-225_22B12_LC | . | . | . | . | N | . | . | . | . | . | . | . | . | . | . | L | . |
| 21-225_27C5_LC | . | . | E | . | . | . | . | . | . | . | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | . | . |
| 21-225_15A2_LC | . | . | . | . | . | F | P | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | E | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | E | . | . | . | . | V | . | . | . | . | . | . | . | . | . |
| 21-225_16H1_LC | . | . | E | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | Y | N | S | - | - | - | - | - | - | - | - |
| 21-225_2C12_LC | . | . | . | . | . | . | . | Y | . | . | . | . | . | . | . | . | . |
| 21-225_17H5_LC | . | . | . | . | . | . | . | H | . | . | . | . | . | . | . | . | . |
| 21-225_16A11_LC | . | . | . | . | L | . | . | . | . | . | . | . | . | . | . | . | . |
| 21-225_20H7_LC | . | . | . | . | . | . | . | Y | . | . | . | . | . | . | . | . | . |
| 21-225_20D12_LC | . | . | . | . | L | . | . | . | . | . | . | . | . | . | . | . | . |
| 21-225_22B12_LC | . | . | . | . | . | . | . | H | . | . | . | . | . | . | . | . | . |
| 21-225_27C5_LC | . | . | C | . | . | . | . | D | . | . | . | . | . | . | . | . | . |
| 21-225_27H12_LC | . | . | . | . | . | . | . | Y | . | . | . | . | . | . | . | . | . |
| 21-225_35H8_LC | . | . | . | . | . | . | . | . | . | . | . | . | . | . | . | . | . |
| 21-225_15F8_LC | A | . | . | . | . | . | . | H | . | . | . | . | . | . | . | . | . |
| 21-225_20D6_LC | . | . | F | . | . | . | . | H | . | . | . | . | . | . | . | . | . |
| 21-225_15A2_LC | N | . | . | . | . | . | . | Y | . | . | . | . | . | . | . | . | . |
| 21-225_5E11_LC | . | . | . | . | H | . | . | H | . | . | . | . | . | . | . | . | . |
| 21-225_2A4_LC | . | . | . | . | . | . | . | . | . | . | . | . | . | . | . | . | . |
| 21-225_16B8_LC | . | . | . | . | H | . | . | H | . | . | . | . | . | . | . | . | . |
| 21-225_14D6_LC | . | . | . | . | L | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | L | . | . | . | G | . | . | . | . | . | . | . | . |
| 21-225_16H1_LC | . | . | . | . | L | . | . | H | . | . | . | . | . | . | . | . | . |
| 21-225_17H6_LC | I | . | . | . | . | . | . | H | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | . | . | . | . | . | . | . | . | . |
| 21-225_22B12_LC | . | . | . | . | . | . | . | . | . | . | . | . | . | . | . | . | . |
| 21-225_27C5_LC | . | . | . | . | . | . | . | . | . | . | . | . | . | . | . | F | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | F | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | . | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | . | . | . | . | . | . | . | . | . | . | . | . | . |
| 21-225_16H1_LC | . | . | . | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | F | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

**FIGURE 56**

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | | K_FR4 | | | | | |
| CONSENSUS | F | T | F | G | P | G | T | K | V | D | I | K | R |
| 21-225_2C12_LC | I | . | . | . | Q | . | . | R | L | E | . | . | . |
| 21-225_17H5_LC | . | . | . | . | . | . | . | . | . | . | . | . | . |
| 21-225_16A11_LC | . | . | . | . | . | . | . | . | . | . | . | . | . |
| 21-225_20H7_LC | . | . | . | . | . | . | . | . | M | . | F | . | . |
| 21-225_20D12_LC | . | . | . | . | . | . | . | . | . | . | . | . | . |
| 21-225_22B12_LC | . | . | . | . | . | . | . | . | . | . | F | . | . |
| 21-225_27C5_LC | R | . | . | . | Q | . | . | . | . | E | . | . | . |
| 21-225_27H12_LC | . | . | . | . | . | . | . | . | . | N | . | N | . |
| 21-225_35H8_LC | . | . | . | . | . | . | . | . | . | . | F | . | . |
| 21-225_15F8_LC | . | . | . | . | R | . | . | . | . | . | . | Q | . |
| 21-225_20D6_LC | . | . | . | . | . | . | . | . | . | . | . | . | . |
| 21-225_15A2_LC | . | . | . | . | . | . | . | Q | . | . | V | . | . |
| 21-225_5E11_LC | . | . | . | . | . | . | . | . | . | . | . | . | . |
| 21-225_2A4_LC | . | . | . | . | . | . | . | . | . | . | . | . | . |
| 21-225_16B8_LC | . | . | . | . | . | . | . | . | . | . | . | V | . |
| 21-225_14D6_LC | . | . | . | . | . | . | . | . | . | . | . | N | . |
| 21-225_1F1_LC | L | . | . | . | G | . | . | . | . | E | . | . | . |
| 21-225_5F4_LC | . | . | . | . | . | . | . | . | . | . | . | . | . |
| 21-225_16H1_LC | . | . | . | . | . | . | . | . | . | . | V | . | . |
| 21-225_17H6_LC | . | . | . | . | . | . | . | . | . | . | . | Q | . |
| 21-225_21F12_LC | . | . | . | . | . | . | . | . | . | . | . | . | . |
| 21-225_23A11_LC | . | . | . | . | . | . | . | . | . | . | L | . | . |
| 21-225_32B3_LC | . | . | . | . | . | . | . | . | . | . | . | . | . |

FIGURE 56

Table 26

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | | H_FR1 |
| CONSENSUS | E | V | Q | L | L | E | S | - | G | G | G | L | V | Q | P | G | G |
| 21-225_23C7_HC | . | . | . | . | . | . | . | . | . | . | . | . | . | . | . | . | . |
| 21-225_23E7_HC | . | . | . | . | . | . | . | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | A | . | . | . | . | . | . | . | . | . | . | D | . | . | . | . | . |
| 21-225_10C5_HC | . | . | . | . | . | . | . | . | . | . | . | . | . | . | . | . | . |
| 21-225_18F7_HC | . | . | . | . | . | . | . | . | . | . | . | . | E | . | . | . | . |
| 21-225_20G11_HC | . | . | . | . | . | . | . | . | . | . | . | . | E | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | S | - |
| 21-225_23C7_HC | . | . | . | . | . | . | . | . | . | . | . | . | . | . | . | . | . |
| 21-225_23E7_HC | . | . | . | . | . | . | T | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | . | . | . | . | . | . | . | . | . | . | . | . | . | . | . | . | . |
| 21-225_10C5_HC | . | . | . | . | . | . | . | . | . | . | . | . | . | T | . | . | . |
| 21-225_18F7_HC | . | . | . | . | . | . | . | . | . | . | . | . | . | R | N | . | . |
| 21-225_20G11_HC | . | . | . | . | . | . | . | . | . | . | . | . | . | R | . | . | . |

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | | | H_FR2 | | |
| CONSENSUS | - | - | - | - | Y | V | M | N | W | V | R | Q | A | P | G | K | G |
| 21-225_23C7_HC | . | . | . | . | . | . | . | S | . | . | . | . | . | . | . | . | . |
| 21-225_23E7_HC | . | . | . | . | . | A | . | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | I | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | A | . | . | . | . | . | . | . | . | . | . | . |
| 21-225_31G1_HC | . | . | . | . | . | . | . | . | . | . | . | . | . | . | . | . | . |
| 21-225_10C5_HC | . | . | . | . | . | . | . | . | . | . | . | . | . | . | . | . | . |
| 21-225_18F7_HC | . | . | . | . | . | . | . | . | . | . | . | . | . | . | . | . | . |
| 21-225_20G11_HC | . | . | . | . | . | . | . | . | . | . | . | . | . | . | . | . | . |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | | H_CDR2 | |
| CONSENSUS | L | E | W | V | S | A | I | S | G | S | - | - | - | G | G | S | T |
| 21-225_23C7_HC | . | . | . | . | . | . | M | . | . | . | . | . | . | . | . | . | . |
| 21-225_23E7_HC | . | . | . | . | . | . | . | . | . | . | . | . | . | . | . | F | . |
| 21-225_30A1_HC | . | . | . | . | . | . | . | . | . | . | . | . | . | . | . | . | . |
| 21-225_25D10_HC | . | . | . | . | . | V | . | . | . | . | . | . | . | . | . | . | . |
| 21-225_33A5_HC | . | . | . | . | . | . | . | . | . | . | . | . | . | . | V | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | G | . |
| 21-225_31G1_HC | . | . | . | . | . | . | . | . | . | . | . | . | . | . | . | R | . |
| 21-225_10C5_HC | . | . | . | . | . | . | . | . | . | . | . | . | . | . | . | R | . |
| 21-225_18F7_HC | . | . | . | . | . | G | . | . | . | . | . | . | . | . | . | R | . |
| 21-225_20G11_HC | . | . | . | . | . | G | . | . | . | . | . | . | . | . | . | . | . |

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_23C7_HC | . | . | V | . | . | . | . | . | . | . | . | . | . | . | . | . | . |
| 21-225_23E7_HC | . | . | . | . | . | . | . | . | . | . | . | . | . | . | . | . | . |
| 21-225_30A1_HC | . | . | . | . | . | . | . | . | . | . | . | . | . | . | N | . | . |
| 21-225_25D10_HC | D | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | . | . | . | . | . | . | . | . | . | . | . | . | . | . | . | . | . |
| 21-225_10C5_HC | N | . | . | . | . | . | . | . | . | . | . | . | . | . | . | . | . |
| 21-225_18F7_HC | . | . | . | . | . | . | . | D | . | . | . | . | . | . | . | . | . |
| 21-225_20G11_HC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | | H_FR3 | | | | | | | | | | |
| CONSENSUS | K | N | T | L | Y | L | Q | M | N | S | L | R | A | E | D | T | A |
| 21-225_23C7_HC | . | . | . | . | . | . | . | . | . | . | . | . | . | . | . | . | . |
| 21-225_23E7_HC | R | . | . | . | . | . | . | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | . | . | . | . | F | . | . | . | . | . | . | . | . | . | . | . | . |
| 21-225_10C5_HC | . | . | . | . | . | . | . | . | . | . | . | . | . | . | . | . | . |
| 21-225_18F7_HC | . | . | . | . | . | . | . | . | . | . | . | . | . | . | . | . | . |
| 21-225_20G11_HC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | T | G | V | - | - | - | - | - | - | - | - |
| 21-225_23C7_HC | . | . | . | . | . | . | . | . | . | . | . | . | . | . | . | . | . |
| 21-225_23E7_HC | . | . | . | . | . | . | . | S | G | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | S | L | . | . | . | . | . | . | . | . |
| 21-225_26C2_HC | . | F | . | . | . | . | . | . | . | . | . | . | . | . | . | . | . |
| 21-225_26D9_HC | . | . | . | . | . | . | . | . | G | . | . | . | . | . | . | . | . |
| 21-225_31G1_HC | . | . | . | . | . | . | . | S | L | . | . | . | . | . | . | . | . |
| 21-225_10C5_HC | . | . | . | . | . | . | . | . | . | . | . | . | . | . | . | . | . |
| 21-225_18F7_HC | . | . | . | . | . | . | . | S | L | . | . | . | . | . | . | . | . |
| 21-225_20G11_HC | . | . | . | . | . | . | . | S | L | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | |
| CONSENSUS | - | - | - | - | - | - | - | - | - | - | - | - | - | - | - | - | F |
| 21-225_23C7_HC | . | . | . | . | . | . | . | . | . | . | . | . | . | . | . | . | . |
| 21-225_23E7_HC | . | . | . | . | . | . | . | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | . | . | . | . | . | . | . | . | . | . | . | . | . | . | . | . | . |
| 21-225_10C5_HC | . | . | . | . | . | . | . | . | . | . | . | . | . | . | . | . | . |
| 21-225_18F7_HC | . | . | . | . | . | . | . | . | . | . | . | . | . | . | . | . | . |
| 21-225_20G11_HC | . | . | . | . | . | . | . | . | . | . | . | . | . | . | . | . | . |

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | H_FR4 | | | | | | | |
| CONSENSUS | D | Y | W | G | Q | G | T | L | V | T | V | S | S |
| 21-225_23C7_HC | . | . | . | . | . | . | . | . | . | I | . | . | . |
| 21-225_23E7_HC | . | . | . | . | . | . | . | . | . | . | . | . | . |
| 21-225_30A1_HC | . | . | . | . | . | . | . | . | . | . | . | . | . |
| 21-225_25D10_HC | . | . | . | . | . | . | . | . | . | . | . | . | . |
| 21-225_33A5_HC | . | . | . | . | . | . | . | . | . | . | . | . | . |
| 21-225_26C2_HC | . | . | . | . | . | . | . | . | . | . | . | . | . |
| 21-225_26D9_HC | . | . | . | . | . | . | . | . | . | . | . | . | . |
| 21-225_31G1_HC | . | . | . | . | . | . | . | . | . | . | . | . | . |
| 21-225_10C5_HC | . | . | . | . | . | . | . | . | . | . | . | . | . |
| 21-225_18F7_HC | . | S | . | . | . | . | . | . | . | . | . | . | . |
| 21-225_20G11_HC | . | S | . | . | . | . | . | . | . | . | . | . | . |

FIGURE 56

Table 27

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | H_FR1 | | |
| CONSENSUS | E | V | Q | L | L | E | S | - | G | G | G | L | V | Q | P | G | G |
| 21-225_12D8_HC | . | . | . | . | . | . | . | . | . | . | . | . | . | . | . | . | . |
| 21-225_30E9_HC | . | . | . | V | . | . | . | . | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | V | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | V | . | . | . | . | . | . | . | . | . | . | . | . | . |
| 21-225_31H3_HC | . | . | . | V | . | . | . | . | . | . | . | . | . | . | . | . | . |
| 21-225_32G12_HC | . | . | . | . | . | . | . | . | . | . | . | . | L | . | . | . | . |
| 21-225_34G9_HC | . | . | . | . | V | . | . | . | . | . | . | . | . | . | . | . | . |
| 21-225_36C5_HC | . | . | . | . | V | . | . | . | . | . | . | . | . | . | . | . | . |
| 21-225_33A4_HC | . | . | . | . | . | . | . | . | . | . | . | . | . | . | S | . | . |
| 21-225_33F1_HC | . | . | . | . | . | . | . | . | . | . | . | . | L | . | . | . | . |
| 21-225_33A7_HC | . | . | . | . | . | . | . | . | . | . | . | . | . | . | . | . | . |
| 21-225_34A6_HC | . | . | . | . | . | . | . | . | . | . | . | . | . | . | . | . | . |
| 21-225_35B7_HC | . | . | . | . | . | . | . | . | . | . | . | . | . | . | . | . | . |
| 21-225_34H11_HC | . | . | . | . | . | . | . | . | . | . | . | . | . | . | . | . | . |
| 21-225_33G7_HC | . | . | . | . | . | . | . | . | . | . | . | . | . | . | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | . | . | . | . | . | . | . | . | . | . |
| 21-225_4G12_HC | . | . | . | . | . | . | . | . | . | . | . | . | . | . | . | . | . |
| 21-225_17F12_HC | . | . | . | . | . | . | . | . | . | . | . | . | . | . | . | . | . |
| 21-225_21H4_HC | . | . | . | . | . | . | . | . | . | . | . | . | . | . | . | . | . |
| 21-225_24B1_HC | . | . | . | . | . | . | . | . | . | . | . | . | . | . | . | . | . |
| 21-225_32G1_HC | . | . | . | . | . | . | . | . | . | . | . | . | A | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | S | - |
| 21-225_12D8_HC | . | . | . | . | . | . | . | . | . | . | . | . | . | . | . | . | . |
| 21-225_30E9_HC | . | . | . | . | . | . | . | . | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | . | . | . | . | . | . | . | . | . | . | . | . | . | . |
| 21-225_31H3_HC | . | . | . | . | . | . | . | . | . | . | . | . | . | . | . | . | . |
| 21-225_32G12_HC | . | . | . | . | . | . | . | . | . | . | . | . | . | . | N | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | . | . | . | . | . | E | . | . | . | . | . | I | . | . | . | . |
| 21-225_33F1_HC | . | . | . | . | . | . | . | . | . | . | . | . | . | . | . | . | . |
| 21-225_33A7_HC | . | . | . | . | . | . | . | . | . | . | . | . | . | . | . | . | . |
| 21-225_34A6_HC | . | . | . | . | . | . | . | . | . | . | . | . | . | . | N | N | . |
| 21-225_35B7_HC | . | . | . | . | . | . | . | . | . | . | . | . | . | . | . | . | . |
| 21-225_34H11_HC | . | . | . | . | . | . | . | . | . | . | . | . | . | . | . | . | . |
| 21-225_33G7_HC | . | . | . | . | . | . | . | . | . | . | . | . | . | . | . | N | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | . | . | . | . | . | . | . | N | N | . |
| 21-225_4G12_HC | . | . | . | . | . | . | . | . | . | . | . | . | . | . | . | . | . |
| 21-225_17F12_HC | . | . | . | . | . | . | . | . | . | . | . | . | . | . | . | . | . |
| 21-225_21H4_HC | . | . | . | . | . | . | . | . | . | . | . | . | . | . | . | . | . |
| 21-225_24B1_HC | . | . | . | . | . | . | . | . | . | . | . | . | . | . | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | H_FR2 | | | | |
| CONSENSUS | - | - | - | - | Y | A | M | S | W | V | R | Q | A | P | G | K | G |
| 21-225_12D8_HC | . | . | . | . | . | V | . | . | . | . | . | . | . | . | . | . | . |
| 21-225_30E9_HC | . | . | . | C | . | . | . | N | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | . | . | . | . | N | . | . | . | . | . | . | . | . | . |
| 21-225_31H3_HC | . | . | . | . | . | . | . | N | . | . | . | . | . | . | . | . | . |
| 21-225_32G12_HC | . | . | . | . | . | . | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | N | . | . | . | . | . | . | . | . | . |
| 21-225_33A4_HC | . | . | . | . | . | . | . | N | . | . | . | . | V | . | . | . | . |
| 21-225_33F1_HC | . | . | . | . | . | . | . | . | . | . | . | . | . | . | . | . | . |
| 21-225_33A7_HC | . | . | . | C | . | . | . | . | . | . | . | . | . | . | . | . | . |
| 21-225_34A6_HC | . | . | . | . | . | . | . | N | . | . | . | . | . | . | . | . | . |
| 21-225_35B7_HC | . | . | . | . | . | . | . | . | . | . | . | . | . | . | . | . | . |
| 21-225_34H11_HC | . | . | . | . | . | . | . | N | . | . | . | . | . | . | . | E | . |
| 21-225_33G7_HC | . | . | . | . | . | . | . | . | . | . | . | . | . | . | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | N | . | . | . | . | . | . | . | . | . |
| 21-225_4G12_HC | . | . | . | . | . | . | . | . | . | . | . | . | . | . | . | . | . |
| 21-225_17F12_HC | . | . | . | . | . | . | . | . | . | . | . | . | . | . | . | . | . |
| 21-225_21H4_HC | . | . | . | . | . | . | . | . | . | . | . | . | . | . | . | . | . |
| 21-225_24B1_HC | . | . | . | . | . | . | . | . | . | . | . | . | . | . | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | | H_CDR2 | |
| CONSENSUS | L | E | W | V | S | A | I | S | G | R | - | - | - | G | G | S | T |
| 21-225_12D8_HC | . | . | . | . | . | T | . | . | V | G | . | . | . | . | . | . | . |
| 21-225_30E9_HC | . | . | . | . | . | . | . | . | . | . | . | . | . | . | . | . | . |
| 21-225_31H5_HC | . | . | . | . | . | . | . | . | . | . | . | . | . | . | . | . | I |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | . | . | . | . | . | . | . | . | . | . | . | . | . | . |
| 21-225_31H3_HC | . | . | . | . | . | . | . | . | . | . | . | . | . | . | . | N | . |
| 21-225_32G12_HC | . | . | . | . | . | S | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | . | . | . | . | . | . | . | R | . | . | . | . | . | . | . | . |
| 21-225_33F1_HC | . | . | . | . | . | S | . | . | . | . | . | . | . | . | . | . | . |
| 21-225_33A7_HC | . | . | . | . | . | . | . | . | . | . | . | . | . | . | . | . | . |
| 21-225_34A6_HC | . | . | . | . | . | . | . | . | R | . | . | . | . | . | . | . | . |
| 21-225_35B7_HC | . | . | . | . | . | . | . | . | . | . | . | . | . | . | . | . | . |
| 21-225_34H11_HC | . | . | . | . | . | . | . | . | . | . | . | . | . | . | . | . | . |
| 21-225_33G7_HC | . | . | . | . | . | . | . | . | R | . | . | . | . | . | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | . | R | . | . | . | . | . | . | N | . |
| 21-225_4G12_HC | . | . | . | . | . | T | L | . | . | . | . | . | . | . | . | . | . |
| 21-225_17F12_HC | . | . | . | . | . | . | . | . | . | G | . | . | . | . | . | . | . |
| 21-225_21H4_HC | . | . | . | . | . | . | L | . | . | . | . | . | . | . | . | . | . |
| 21-225_24B1_HC | . | . | . | . | . | . | . | . | . | . | . | . | . | . | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | T | . | . | . | . | . | . | . | . | . | . | . |

EP 4 435 105 A2

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | F | H | A | D | S | V | K | G | R | F | T | I | S | R | D | N | S |
| 21-225_12D8_HC | Y | Y | . | . | . | . | . | . | . | . | . | . | . | . | . | . | . |
| 21-225_30E9_HC | . | Y | . | . | . | . | . | . | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | N | . | . | . | . | . | . | . | . | . | . | . | . | . | . | . |
| 21-225_30E3_HC | . | Y | . | . | . | E | . | . | . | . | . | . | . | . | . | . | . |
| 21-225_31H3_HC | . | Y | . | . | . | . | . | . | . | . | . | . | . | . | . | . | . |
| 21-225_32G12_HC | . | . | . | . | . | . | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | Y | . | . | . | . | . | . | . | . | . | . | . | . | . | . | . |
| 21-225_33F1_HC | . | . | . | . | . | . | . | . | . | . | . | . | . | . | . | . | . |
| 21-225_33A7_HC | . | . | . | . | . | . | . | . | . | . | . | . | . | . | . | . | . |
| 21-225_34A6_HC | . | Y | . | . | . | . | . | . | . | . | . | . | . | . | . | . | . |
| 21-225_35B7_HC | . | . | . | . | . | . | . | . | . | . | . | . | . | . | . | . | . |
| 21-225_34H11_HC | . | Y | . | . | . | . | . | . | . | . | . | . | . | . | . | . | . |
| 21-225_33G7_HC | . | Y | . | . | . | . | . | . | Q | . | . | . | . | . | . | . | . |
| 21-225_35D1_HC | . | . | . | . | . | . | . | . | . | . | . | . | . | . | . | . | . |
| 21-225_35F11_HC | . | . | . | . | M | . | . | . | . | . | . | . | . | . | . | . | . |
| 21-225_34G8_HC | . | . | . | . | . | . | . | . | . | . | . | . | . | . | . | . | . |
| 21-225_36A5_HC | . | Y | . | . | . | . | . | . | . | . | . | . | . | . | . | . | . |
| 21-225_4G12_HC | Y | Y | . | . | . | . | . | . | . | S | . | . | . | . | . | . | . |
| 21-225_17F12_HC | Y | Y | . | . | . | . | . | . | . | V | . | . | . | . | . | . | . |
| 21-225_21H4_HC | Y | Y | . | . | . | . | . | . | . | . | . | . | . | . | . | . | . |
| 21-225_24B1_HC | Y | Y | . | . | . | . | . | . | . | . | . | . | . | . | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

3474

**FIGURE 56**

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | V | K | G | E | L | L | E | D | Y | - | - | - | - | - |
| 21-225_12D8_HC | . | . | . | . | A | . | W | G | R | G | Y | S | . | E | . | . | . |
| 21-225_30E9_HC | . | . | . | . | A | . | . | . | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | L | . | . | . | A | . | . | . | . | . | . | . | . | . | . | . | . |
| 21-225_31H3_HC | . | . | . | . | A | . | . | . | . | . | . | . | . | . | . | . | . |
| 21-225_32G12_HC | . | . | . | . | . | . | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | . | F | . | A | . | . | . | . | . | . | . | . | . | . | . | . |
| 21-225_33F1_HC | . | . | . | . | . | . | . | . | . | . | . | . | . | . | . | . | . |
| 21-225_33A7_HC | . | . | . | . | . | . | . | . | . | . | . | . | . | . | . | . | . |
| 21-225_34A6_HC | . | . | . | . | A | . | . | . | . | . | . | . | . | . | . | . | . |
| 21-225_35B7_HC | . | . | . | . | . | . | . | . | . | . | . | . | . | . | . | . | . |
| 21-225_34H11_HC | . | . | . | . | A | . | . | . | . | . | . | . | . | . | . | . | . |
| 21-225_33G7_HC | . | . | . | . | A | . | . | . | . | . | . | . | . | . | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | A | . | . | . | . | . | . | . | . | . | . | . | . |
| 21-225_4G12_HC | . | . | . | . | A | . | W | G | R | G | Y | S | . | E | . | . | . |
| 21-225_17F12_HC | . | . | . | . | A | . | W | G | R | G | Y | N | . | E | . | . | . |
| 21-225_21H4_HC | . | . | . | . | A | . | W | G | R | G | Y | S | . | E | . | . | . |
| 21-225_24B1_HC | . | . | . | . | A | . | W | G | R | G | Y | S | . | E | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

# EP 4 435 105 A2

**FIGURE 56**

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | Y | F | Y | G | M |
| 21-225_12D8_HC | . | . | . | . | . | . | . | . | . | . | . | . | . | Y | . | . | . |
| 21-225_30E9_HC | . | . | . | . | . | . | . | . | . | . | . | . | . | Y | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | L |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | . | . | . | . | . | . | . | . | . | . | Y | . | . | . |
| 21-225_31H3_HC | . | . | . | . | . | . | . | . | . | . | . | . | . | Y | . | . | . |
| 21-225_32G12_HC | . | . | . | . | . | . | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | . | . | . | . | . | . | . | . | . | . | . | S | Y | . | . | I |
| 21-225_33F1_HC | . | . | . | . | . | . | . | . | . | . | . | . | . | . | . | . | . |
| 21-225_33A7_HC | . | . | . | . | . | . | . | . | . | . | . | . | . | . | . | . | . |
| 21-225_34A6_HC | . | . | . | . | . | . | . | . | . | . | . | . | . | Y | F | A | . |
| 21-225_35B7_HC | . | . | . | . | . | . | . | . | . | . | . | . | . | . | . | . | . |
| 21-225_34H11_HC | . | . | . | . | . | . | . | . | . | . | . | . | . | Y | . | . | . |
| 21-225_33G7_HC | . | . | . | . | . | . | . | . | . | . | . | . | S | Y | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | . | . | . | . | . | . | Y | . | A | . |
| 21-225_4G12_HC | . | . | . | . | . | . | . | . | . | . | . | . | . | Y | . | . | . |
| 21-225_17F12_HC | . | . | . | . | . | . | . | . | . | . | . | . | . | Y | . | . | . |
| 21-225_21H4_HC | . | . | . | . | . | . | . | . | . | . | . | . | . | Y | . | . | . |
| 21-225_24B1_HC | . | . | . | . | . | . | . | . | . | . | . | . | . | Y | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

3477

# EP 4 435 105 A2

## FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | H_FR4 | | | | | | |
| CONSENSUS | D | V | W | G | Q | G | T | T | V | T | V | S | S |
| 21-225_12D8_HC | | | | | | | | | | | | | |
| 21-225_30E9_HC | | | | | | | | | | | | | |
| 21-225_31H5_HC | | | | | | | | | | | | | |
| 21-225_31A8_HC | | | | | | | | | | | | | |
| 21-225_30D1_HC | | | | | | | | | | | | | |
| 21-225_31B8_HC | | | | | | | | | | | | | |
| 21-225_30G11_HC | | | | | | | | | | | | | |
| 21-225_30E3_HC | | | | | | | | | | | | | |
| 21-225_31H3_HC | | | | | | | | | | | | | |
| 21-225_32G12_HC | | | | | | | | | | | | | |
| 21-225_34G9_HC | | | | | | | | | | | | | |
| 21-225_36C5_HC | | | | | | | | | | | | | |
| 21-225_33A4_HC | | | | | | | | | | | | | |
| 21-225_33F1_HC | | | | | | | | | | | | | |
| 21-225_33A7_HC | | | | | | | | | | | | | |
| 21-225_34A6_HC | | | | | | | | | | | | | |
| 21-225_35B7_HC | | | | | | | A | | | | | | |
| 21-225_34H11_HC | | | | | | | | | | | | | |
| 21-225_33G7_HC | | | | | | | | | | | | | |
| 21-225_35D1_HC | | | | | | | A | | | | | | |
| 21-225_35F11_HC | | | | | | | | | | | | | |
| 21-225_34G8_HC | | | | | | | | | | | | | |
| 21-225_36A5_HC | | | | | | | | | | | | | |
| 21-225_4G12_HC | | | | | | | | | | | | | |
| 21-225_17F12_HC | | | | | | | | | | | | | |
| 21-225_21H4_HC | | | | | | | | | | | | | |
| 21-225_24B1_HC | | | | | | | | | | | | | |
| 21-225_32G1_HC | | | | | | | | | | | | | |
| 21-225_33B8_HC | | | | | | | | | | | | | |
| 21-225_35A6_HC | | | | | | | | | | | | | |

# EP 4 435 105 A2

FIGURE 56

Table 28

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | E | V | Q | L | L | E | S | - | G | G | G | L | V | Q | P | G | G |
| 21-225_17C6_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | . | . | . | . | . | . | . | . | . | . | . | . | . | . | . | . | . |
| 21-225_17F7_HC | . | . | . | . | . | . | . | . | . | . | . | . | . | . | . | . | . |
| 21-225_17F11_HC | . | A | . | . | . | . | . | . | . | . | . | . | E | . | . | . | . |
| 21-225_17A12_HC | . | . | . | . | . | . | . | . | . | . | . | . | I | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | . | . | . | . | . | . | D | . | . | . | . | . | . |
| 21-225_21F4_HC | . | . | . | . | . | . | . | . | . | . | . | . | . | . | . | . | . |
| 21-225_22E4_HC | . | . | . | . | . | . | . | . | . | . | . | . | . | . | . | . | . |
| 21-225_21A2_HC | . | . | . | . | . | . | . | . | . | . | . | . | . | . | . | . | . |
| 21-225_22C5_HC | . | . | . | . | . | . | . | . | . | . | . | . | . | . | V | . | . |
| 21-225_22D5_HC | . | . | . | . | . | . | . | . | . | . | . | . | . | . | . | . | . |
| 21-225_10F12_HC | . | . | . | . | . | . | . | . | . | . | . | . | . | . | . | . | . |
| 21-225_4H2_HC | . | . | . | . | . | . | . | . | . | . | . | . | . | . | . | . | . |
| 21-225_5G2_HC | . | . | . | . | . | . | . | . | . | . | . | . | . | . | . | . | . |
| 21-225_3E3_HC | . | . | . | . | . | . | . | . | . | . | T | . | . | . | . | . | . |
| 21-225_5F7_HC | . | . | . | . | . | . | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | . | . | . | . | . | . | . | . | . | . | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | . | . | . | . | . | . | . | . | . | . |
| 21-225_11A5_HC | . | . | . | . | . | . | . | . | . | . | D | . | . | . | . | . | . |

3479

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | E | - | F | T | F | S | S | - |
| 21-225_17C6_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | . | . | . | . | . | . | . | . | . | G | . | . | . | . | . | . | . |
| 21-225_17F7_HC | . | . | . | . | . | . | . | . | . | . | . | . | . | . | . | . | . |
| 21-225_17F11_HC | . | . | . | . | . | . | . | . | . | G | . | . | . | . | . | . | . |
| 21-225_17A12_HC | . | . | . | . | . | . | . | . | . | . | . | . | . | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | . | . | T | . | . | G | . | . | . | . | . | . | . |
| 21-225_21F4_HC | . | . | . | . | . | . | . | . | . | . | . | . | . | . | . | . | . |
| 21-225_22E4_HC | . | . | . | . | . | . | . | . | . | . | . | . | . | . | . | . | . |
| 21-225_21A2_HC | . | . | . | . | . | . | . | . | . | K | . | . | . | . | . | . | . |
| 21-225_22C5_HC | . | . | . | . | . | . | . | . | . | G | . | . | . | . | . | . | . |
| 21-225_22D5_HC | . | . | . | . | . | . | . | . | . | . | . | . | . | . | . | . | . |
| 21-225_10F12_HC | . | . | . | . | . | . | . | . | . | . | . | . | . | . | . | . | . |
| 21-225_4H2_HC | . | . | . | . | . | . | . | . | . | G | . | . | . | . | . | G | . |
| 21-225_5G2_HC | . | . | . | . | . | . | . | . | . | . | . | . | . | . | . | . | . |
| 21-225_3E3_HC | . | . | . | . | . | . | . | . | . | . | . | . | . | . | . | . | . |
| 21-225_5F7_HC | . | . | . | . | . | . | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | V | . | . | . | . | . | . | . | . | . | . |
| 21-225_16H6_HC | . | . | . | . | . | . | . | . | . | G | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | . | . | . | . | . | . | . | . | . | . |
| 21-225_11A5_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | | | H_FR2 | | |
| CONSENSUS | - | - | - | - | Y | A | M | | W | V | R | Q | A | P | G | K | G |
| 21-225_17C6_HC | . | . | . | . | . | . | | N | . | . | . | . | . | . | . | . | . |
| 21-225_17B4_HC | . | . | . | . | . | . | | N | . | . | . | . | . | . | . | . | . |
| 21-225_20A4_HC | . | . | . | . | . | . | | T | . | . | . | . | G | . | . | M | . |
| 21-225_17F7_HC | . | . | . | . | . | . | | N | . | . | . | . | . | . | . | . | . |
| 21-225_17F11_HC | . | . | . | . | . | . | | S | . | . | . | . | . | . | . | . | . |
| 21-225_17A12_HC | . | . | . | . | . | . | | S | . | . | . | . | D | . | . | . | . |
| 21-225_18E12_HC | . | . | . | . | . | . | | S | . | . | . | . | . | . | . | T | . |
| 21-225_22G3_HC | . | . | . | . | . | . | | N | . | . | . | . | . | . | . | . | . |
| 21-225_22G10_HC | . | . | . | . | . | . | | N | . | . | . | . | . | . | . | . | . |
| 21-225_21F4_HC | . | . | . | . | . | . | | N | . | . | . | . | . | . | . | . | . |
| 21-225_22E4_HC | . | . | . | . | . | . | | S | . | . | . | . | . | . | . | . | . |
| 21-225_21A2_HC | . | . | . | . | . | . | | N | . | . | . | . | . | . | . | . | . |
| 21-225_22C5_HC | . | . | . | . | . | . | | S | . | . | . | . | . | . | . | M | . |
| 21-225_22D5_HC | . | . | . | . | . | . | | S | . | . | . | . | . | . | . | . | . |
| 21-225_10F12_HC | . | . | . | . | . | . | | S | . | . | . | . | . | . | . | . | . |
| 21-225_4H2_HC | . | . | . | . | . | . | | N | . | . | . | . | . | . | . | M | . |
| 21-225_5G2_HC | . | . | . | . | . | . | | S | . | . | . | . | . | . | . | . | . |
| 21-225_3E3_HC | . | . | . | . | V | . | | S | . | . | . | . | . | . | . | . | . |
| 21-225_5F7_HC | . | . | . | . | . | . | | S | . | . | . | . | . | . | . | . | . |
| 21-225_6D3_HC | . | . | . | . | . | . | | N | . | . | . | . | . | . | . | . | . |
| 21-225_8G6_HC | . | . | . | . | V | | . | N | . | . | . | . | . | . | . | . | . |
| 21-225_16H6_HC | . | . | . | . | . | . | | N | . | . | . | . | . | . | . | M | . |
| 21-225_3B1_HC | . | . | . | . | . | . | | N | . | . | . | . | . | . | . | . | . |
| 21-225_8E12_HC | . | . | . | . | . | . | | S | . | . | . | . | . | . | . | . | . |
| 21-225_11A5_HC | . | . | . | . | . | . | | S | . | . | . | . | . | . | . | . | . |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | S | V | I | S | G | R | - | - | - | G | G | N | T |
| 21-225_17C6_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | | . | . | . | . | . | . | . | . | S | . | . | . | . | . | Y | . |
| 21-225_17F7_HC | . | . | . | . | . | I | . | . | . | . | . | . | . | . | . | . | . |
| 21-225_17F11_HC | . | . | . | . | . | . | . | . | . | . | . | . | . | . | . | . | . |
| 21-225_17A12_HC | . | . | . | . | . | . | . | . | . | . | . | . | . | . | . | . | A |
| 21-225_18E12_HC | | . | . | . | . | I | . | . | . | . | . | . | . | . | . | . | . |
| 21-225_22G3_HC | . | . | . | . | . | I | . | . | . | . | . | . | . | . | V | . | . |
| 21-225_22G10_HC | . | . | . | . | . | . | . | . | . | S | . | . | . | . | . | . | . |
| 21-225_21F4_HC | . | . | . | . | . | . | . | . | . | . | . | . | . | . | . | . | . |
| 21-225_22E4_HC | . | . | . | . | . | I | . | . | . | . | . | . | . | . | T | . | . |
| 21-225_21A2_HC | . | . | . | I | . | I | . | . | . | . | . | . | . | . | . | . | . |
| 21-225_22C5_HC | . | . | . | . | . | I | . | . | . | S | . | . | . | . | . | Y | . |
| 21-225_22D5_HC | . | . | . | . | . | . | . | . | . | . | . | . | . | . | . | . | . |
| 21-225_10F12_HC | . | . | . | . | . | I | . | . | . | . | . | . | . | . | . | . | . |
| 21-225_4H2_HC | | . | . | . | . | . | . | . | R | S | . | . | . | . | . | Y | . |
| 21-225_5G2_HC | . | . | . | . | . | . | . | . | . | . | . | . | . | . | V | . | . |
| 21-225_3E3_HC | . | . | . | . | . | I | . | . | . | . | . | . | . | . | . | . | . |
| 21-225_5F7_HC | . | . | . | . | . | I | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | | . | . | . | . | . | . | . | R | S | . | . | . | . | . | Y | . |
| 21-225_3B1_HC | . | . | . | . | . | . | . | . | . | . | . | . | . | . | I | . | . |
| 21-225_8E12_HC | . | . | . | . | . | I | . | . | . | . | . | . | . | . | . | . | . |
| 21-225_11A5_HC | . | . | . | I | . | . | . | . | . | . | . | . | . | . | V | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | F | Y | A | D | S | V | K | G | R | F | T | I | S | R | D | N | S |
| 21-225_17C6_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | Y | N | . | . | . | . | . | . | . | . | . | . | . | . | . | . | . |
| 21-225_17F7_HC | . | . | . | . | . | . | . | . | . | . | . | . | . | . | . | . | . |
| 21-225_17F11_HC | Y | S | . | . | . | . | . | . | . | . | . | . | . | . | . | . | . |
| 21-225_17A12_HC | . | . | . | . | . | . | . | . | . | . | . | . | . | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | . | . | . | . | . | . | . | . | . | . | . | . | . |
| 21-225_21F4_HC | . | . | . | . | . | . | . | . | . | . | . | . | . | . | . | . | . |
| 21-225_22E4_HC | . | . | . | . | . | . | . | . | . | . | . | . | . | . | V | . | . |
| 21-225_21A2_HC | . | . | . | . | . | . | . | . | . | . | . | . | . | . | . | . | . |
| 21-225_22C5_HC | Y | . | . | . | . | . | . | . | . | . | . | . | . | . | . | . | . |
| 21-225_22D5_HC | . | . | . | . | . | . | . | . | . | . | . | . | . | . | . | . | . |
| 21-225_10F12_HC | . | . | . | . | . | . | . | . | . | . | . | . | . | . | . | . | . |
| 21-225_4H2_HC | Y | . | . | . | . | . | . | . | . | . | . | . | . | . | . | . | . |
| 21-225_5G2_HC | . | . | . | . | . | . | . | . | . | . | . | . | . | . | . | . | . |
| 21-225_3E3_HC | . | . | . | . | . | . | . | . | . | . | . | . | . | . | . | . | . |
| 21-225_5F7_HC | . | . | . | . | . | . | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | Y | . | . | . | . | . | . | . | . | . | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | . | . | . | . | . | . | . | . | . | . |
| 21-225_11A5_HC | . | . | . | . | . | . | . | . | . | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | | H_FR3 | | | | | | | | | | |
| CONSENSUS | K | N | T | L | Y | L | Q | M | N | S | L | R | A | E | D | T | A |
| 21-225_17C6_HC | . | . | . | . | F | . | . | . | . | . | . | . | . | . | . | . | . |
| 21-225_17B4_HC | . | . | . | . | . | . | . | . | . | . | . | . | . | . | . | . | . |
| 21-225_20A4_HC | . | . | . | . | . | . | . | . | . | . | . | . | V | . | . | . | . |
| 21-225_17F7_HC | . | . | . | . | . | . | . | . | . | . | . | . | . | . | . | . | . |
| 21-225_17F11_HC | . | . | . | . | . | . | . | . | . | . | . | . | . | . | . | . | . |
| 21-225_17A12_HC | . | . | . | . | . | . | . | . | . | . | . | . | . | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | F | . | H | . | . | . | . | . | . | . | . | . | . |
| 21-225_21F4_HC | . | . | . | . | . | . | . | . | . | . | . | . | . | . | . | . | . |
| 21-225_22E4_HC | . | . | . | . | . | . | . | . | . | . | . | . | . | . | . | . | . |
| 21-225_21A2_HC | . | . | . | . | . | . | . | . | . | . | . | . | . | . | . | . | . |
| 21-225_22C5_HC | . | . | . | . | . | . | . | . | . | . | . | . | . | . | . | . | . |
| 21-225_22D5_HC | . | . | . | . | . | . | . | . | . | . | . | . | . | . | . | . | . |
| 21-225_10F12_HC | . | . | . | . | . | . | . | . | . | . | . | . | . | . | . | . | . |
| 21-225_4H2_HC | . | . | . | . | . | . | . | . | . | . | . | . | . | . | . | . | . |
| 21-225_5G2_HC | . | . | . | . | . | . | . | . | . | . | . | . | . | . | . | . | . |
| 21-225_3E3_HC | . | . | . | . | . | . | . | . | . | . | . | . | . | . | . | . | . |
| 21-225_5F7_HC | . | . | . | . | . | . | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | . | . | . | . | . | . | . | . | . | . | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | . | . | . | . | . | . | . | . | . | . |
| 21-225_11A5_HC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | S | R | L | A | V | A | G | - | - | - | - | - |
| 21-225_17C6_HC | | | | | | | | | | | | | | | | | |
| 21-225_17B4_HC | | | | | | | | | | | | | | | | | |
| 21-225_20A4_HC | | | | | | | | I | | | | | | | | | |
| 21-225_17F7_HC | | | | | | | | | | | | | | | | | |
| 21-225_17F11_HC | | | | | | | | | | | | | | | | | |
| 21-225_17A12_HC | | | | | | | | I | | | | | | | | | |
| 21-225_18E12_HC | | | | | | | | | | | | | | | | | |
| 21-225_22G3_HC | | | | | | | | | | | | | | | | | |
| 21-225_22G10_HC | | | | | | | | M | | | | | | | | | |
| 21-225_21F4_HC | | | | | | | | | | | | | | | | | |
| 21-225_22E4_HC | | | | | | | | I | | | | | | | | | |
| 21-225_21A2_HC | | | | | | | | | | | | | | | | | |
| 21-225_22C5_HC | | | | | | | | I | | | | | | | | | |
| 21-225_22D5_HC | | | | | | | | I | | | | | | | | | |
| 21-225_10F12_HC | | | | | | | | V | | | | | | | | | |
| 21-225_4H2_HC | | | | | | | | | | | | | | | | | |
| 21-225_5G2_HC | | | | | | | | I | | | | | | | | | |
| 21-225_3E3_HC | L | | | | | | | | | | | | | | | | |
| 21-225_5F7_HC | | | | | | | | I | | | | | | | | | |
| 21-225_6D3_HC | | | F | | | | | | | | | | | | | | |
| 21-225_8G6_HC | | | | | | | | | | | | | | | | | |
| 21-225_16H6_HC | | | | | | | | | | | | | | | | | |
| 21-225_3B1_HC | | | | | | | | | | | | | | | | | |
| 21-225_8E12_HC | | | | | | | | M | | | | | | | | | |
| 21-225_11A5_HC | | | | | | | | I | | | | | | | | | |

3485

**FIGURE 56**

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | S | E | A | F |
| 21-225_17C6_HC | | | | | | | | | | | | | | | | | |
| 21-225_17B4_HC | | | | | | | | | | | | | | | | | |
| 21-225_20A4_HC | | | | | | | | | | | | | | | | | |
| 21-225_17F7_HC | | | | | | | | | | | | | | | | | |
| 21-225_17F11_HC | | | | | | | | | | | | | | | | | |
| 21-225_17A12_HC | | | | | | | | | | | | | | | | | |
| 21-225_18E12_HC | | | | | | | | | | | | | | | | | |
| 21-225_22G3_HC | | | | | | | | | | | | | | | | | |
| 21-225_22G10_HC | | | | | | | | | | | | | | | | | |
| 21-225_21F4_HC | | | | | | | | | | | | | | | | | C |
| 21-225_22E4_HC | | | | | | | | | | | | | | | | | |
| 21-225_21A2_HC | | | | | | | | | | | | | | | | | |
| 21-225_22C5_HC | | | | | | | | | | | | | | | | | |
| 21-225_22D5_HC | | | | | | | | | | | | | | | | | |
| 21-225_10F12_HC | | | | | | | | | | | | | | | | | |
| 21-225_4H2_HC | | | | | | | | | | | | | | | | | |
| 21-225_5G2_HC | | | | | | | | | | | | | | | | | |
| 21-225_3E3_HC | | | | | | | | | | | | | | | | | |
| 21-225_5F7_HC | | | | | | | | | | | | | | | | | |
| 21-225_6D3_HC | | | | | | | | | | | | | | | | | |
| 21-225_8G6_HC | | | | | | | | | | | | | | | | | |
| 21-225_16H6_HC | | | | | | | | | | | | | | | | | |
| 21-225_3B1_HC | | | | | | | | | | | | | | | | | |
| 21-225_8E12_HC | | | | | | | | | | | | | | | | | |
| 21-225_11A5_HC | | | | | | | | | | | | | | | | | |

**FIGURE 56**

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | H_FR4 | | | | | | |
| CONSENSUS | D | I | W | G | Q | G | T | M | V | T | V | S | S |
| 21-225_17C6_HC | | | | | | | | | | | | | |
| 21-225_17B4_HC | | | | | | | | | | | | | |
| 21-225_20A4_HC | | | | | | | | | | | | | |
| 21-225_17F7_HC | | | | | | | | | | | | | |
| 21-225_17F11_HC | H | | | | | | | | | | | | |
| 21-225_17A12_HC | | | | | | | | | | | | | |
| 21-225_18E12_HC | | | | | | | | | | | | | |
| 21-225_22G3_HC | | | | | | | | | | | | | |
| 21-225_22G10_HC | | | | | | | | | | | | | |
| 21-225_21F4_HC | | | | | | | | | | | | | |
| 21-225_22E4_HC | | | | | | | | | | | | | |
| 21-225_21A2_HC | | | | | | | | | | | | | |
| 21-225_22C5_HC | | | | | | | | | | | | | |
| 21-225_22D5_HC | | | | | | | | | | | | | |
| 21-225_10F12_HC | A | | | | | | | | | | | | |
| 21-225_4H2_HC | | | | | | | | | | | | | |
| 21-225_5G2_HC | | | | | | | | | | | | | |
| 21-225_3E3_HC | | | | | | | | | | | | | |
| 21-225_5F7_HC | A | | | | | | | | | | | | |
| 21-225_6D3_HC | | | | | | | | | | | | | |
| 21-225_8G6_HC | | . | | | | | | | S | | | | |
| 21-225_16H6_HC | | | | | | | | | | | | | |
| 21-225_3B1_HC | | | | | | | | | | | | | |
| 21-225_8E12_HC | | | | | | | | | | | | | |
| 21-225_11A5_HC | | | | | | | | | | | | | |

**FIGURE 56**

Table 29

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | V | Q | L | V | E | S | - | G | G | G | V | V | Q | P | G | R |
| 21-225_20F6_HC | . | . | . | . | . | . | . | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | . | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | . |
| 21-225_7E11_HC | . | . | . | . | . | . | . | . | . | . | . | . | . | . | . | . | . |
| 21-225_6G1_HC | . | . | . | . | . | . | . | . | . | . | . | . | . | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225_16E6_HC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | S | - |
| 21-225_20F6_HC | . | . | . | . | . | . | V | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | . | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | . |
| 21-225_7E11_HC | . | . | . | . | . | . | . | . | . | . | . | . | . | . | . | . | . |
| 21-225_6G1_HC | . | . | . | . | . | . | . | . | . | . | . | . | I | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225_16E6_HC | . | . | . | . | . | . | . | . | . | . | . | . | . | . | . | . | . |

EP 4 435 105 A2

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | | | H_FR2 | | |
| CONSENSUS | - | - | - | - | Y | G | M | H | W | V | R | Q | A | P | G | K | G |
| 21-225_20F6_HC | . | . | . | . | . | . | . | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | . | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | . |
| 21-225_7E11_HC | . | . | . | . | F | . | . | . | . | . | . | . | . | . | . | . | . |
| 21-225_6G1_HC | . | . | . | . | . | . | . | . | . | . | . | . | . | . | . | . | . |
| 21-225_15A3_HC | . | . | . | . | . | . | I | . | . | . | . | . | . | . | . | . | . |
| 21-225_13D4_HC | . | . | . | . | . | . | . | . | . | . | . | . | . | . | . | . | . |
| 21-225_16E6_HC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | A | V | I | W | D | - | - | - | G | S | N | K | |
| 21-225_20F6_HC | . | . | . | G | . | . | . | . | H | . | . | . | . | . | . | . | . |
| 21-225_18G4_HC | . | . | . | . | . | I | . | . | Y | . | . | . | . | . | . | . | . |
| 21-225_22C7_HC | . | . | . | G | . | . | . | . | H | . | . | . | . | . | . | . | . |
| 21-225_27A11_HC | . | . | . | . | . | . | . | . | Y | . | . | . | . | . | . | . | . |
| 21-225_26G11_HC | . | . | . | . | . | . | . | . | Y | . | . | . | . | . | . | . | . |
| 21-225_26D12_HC | . | . | . | . | . | . | . | . | Y | . | . | . | . | . | . | . | E |
| 21-225_25B3_HC | . | . | . | . | . | . | . | . | Y | . | . | . | . | . | . | . | . |
| 21-225_25F3_HC | . | . | . | . | . | . | . | . | Y | . | . | . | . | . | . | . | . |
| 21-225_3F8_HC | . | . | . | G | . | . | . | . | H | . | . | . | . | . | . | . | . |
| 21-225_7E11_HC | . | . | . | . | . | I | . | . | H | . | . | . | . | . | . | . | . |
| 21-225_6G1_HC | . | . | . | . | . | I | . | . | Y | . | . | . | . | . | . | . | . |
| 21-225_15A3_HC | . | . | . | . | . | . | . | . | H | . | . | . | . | . | . | . | . |
| 21-225_13D4_HC | . | . | . | . | . | . | . | . | H | . | . | . | . | . | . | . | . |
| 21-225_16E6_HC | . | . | . | . | . | . | . | . | H | . | . | . | . | . | . | . | . |

3489

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_20F6_HC | . | . | . | . | . | . | . | . | . | . | . | . | . | . | . | . | A |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | A |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | . | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | A |
| 21-225_7E11_HC | . | . | . | . | . | . | . | . | . | . | . | . | . | . | . | . | . |
| 21-225_6G1_HC | . | . | . | . | . | . | . | . | . | . | . | . | . | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225_16E6_HC | . | . | V | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | | H_FR3 | | | | | | | | | | |
| CONSENSUS | K | N | T | L | Y | L | Q | M | N | S | L | R | A | E | D | T | A |
| 21-225_20F6_HC | . | . | . | . | . | . | . | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | A | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | . |
| 21-225_7E11_HC | N | . | . | . | . | . | . | . | S | . | . | . | . | . | . | . | . |
| 21-225_6G1_HC | . | . | . | . | . | . | . | . | . | . | . | . | . | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225_16E6_HC | . | . | . | . | N | . | . | . | . | . | . | . | . | . | . | . | . |

# EP 4 435 105 A2

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | D | L | S | M | G | - | - | - | - | - | - |
| 21-225_20F6_HC | | | | | | | | | | | | | | | | | |
| 21-225_18G4_HC | | | | | | | | | | | | | | | | | |
| 21-225_22C7_HC | | | | | | | | | | | | | | | | | |
| 21-225_27A11_HC | | | H | | | | | S | | P | Y | | | | | | |
| 21-225_26G11_HC | | | | | | | R | Y | | N | S | W | | | | | |
| 21-225_26D12_HC | | | | | | | | S | | P | Y | | | | | | |
| 21-225_25B3_HC | | | | | | | | S | | P | Y | | | | | | |
| 21-225_25F3_HC | | | | | | | | S | | P | Y | | | | | | |
| 21-225_3F8_HC | | | | | | | | | | | | | | | | | |
| 21-225_7E11_HC | | | | | | | | | | | | | | | | | |
| 21-225_6G1_HC | M | | | | | | | | | | | | | | | | |
| 21-225_15A3_HC | | | | | | | | | | | | | | | | | |
| 21-225_13D4_HC | | | | | | | | | | | | | | | | | |
| 21-225 16E6 HC | | | | | | | | | | | | | | | | | |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | - | - | G | M |
| 21-225_20F6_HC | | | | | | | | | | | | | | | | | |
| 21-225_18G4_HC | | | | | | | | | | | | | | | | | |
| 21-225_22C7_HC | | | | | | | | | | | | | | | | | |
| 21-225_27A11_HC | | | | | | | | | | | | | | | | | |
| 21-225_26G11_HC | | | | | | | | | | | | | | S | G | | |
| 21-225_26D12_HC | | | | | | | | | | | | | | | | | |
| 21-225_25B3_HC | | | | | | | | | | | | | | | | | |
| 21-225_25F3_HC | | | | | | | | | | | | | | | | | |
| 21-225_3F8_HC | | | | | | | | | | | | | | | | | |
| 21-225_7E11_HC | | | | | | | | | | | | | | | | | |
| 21-225_6G1_HC | | | | | | | | | | | | | | | | | |
| 21-225_15A3_HC | | | | | | | | | | | | | | | | | |
| 21-225_13D4_HC | | | | | | | | | | | | | | | | | |
| 21-225 16E6 HC | | | | | | | | | | | | | | | | | |

3491

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | H_FR4 | | | | | | |
| CONSENSUS | D | V | W | G | Q | G | T | T | V | T | V | S | S |
| 21-225_20F6_HC | . | . | . | . | . | . | . | | . | . | . | . | . |
| 21-225_18G4_HC | . | . | . | . | . | . | . | | . | . | . | . | . |
| 21-225_22C7_HC | . | . | . | . | . | . | . | | . | . | . | . | . |
| 21-225_27A11_HC | . | . | . | . | . | . | . | | . | . | . | . | . |
| 21-225_26G11_HC | . | . | . | . | . | . | . | | . | . | . | . | . |
| 21-225_26D12_HC | . | . | . | . | . | . | . | | . | . | . | . | . |
| 21-225_25B3_HC | . | . | . | . | . | . | . | | . | . | . | . | . |
| 21-225_25F3_HC | . | . | . | . | . | . | . | | . | . | . | . | . |
| 21-225_3F8_HC | . | . | . | . | . | . | . | | . | . | . | . | . |
| 21-225_7E11_HC | . | . | . | . | . | . | . | | . | . | . | . | . |
| 21-225_6G1_HC | . | . | . | . | . | . | . | | . | . | . | . | . |
| 21-225_15A3_HC | . | . | . | . | . | . | . | | . | . | . | . | . |
| 21-225_13D4_HC | . | . | . | . | . | . | . | | . | . | . | . | . |
| 21-225_16E6_HC | . | . | . | . | . | . | . | | . | . | . | . | . |

FIGURE 56

Table 30

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | V | Q | L | V | E | S | - | G | G | G | V | V | Q | P | G | R |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | . | . | . | . | . |
| 21-225_25A2_HC | . | . | . | . | . | . | . | . | . | . | . | . | . | . | . | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | . | . | . | . | . |
| 21-225_29H4_HC | . | . | . | . | . | . | . | . | . | . | . | . | . | . | . | . | . |
| 21-225_26A11_HC | . | . | . | . | . | . | . | . | . | . | . | . | . | . | . | . | . |
| 21-225_26G7_HC | . | . | . | . | . | . | . | . | . | . | . | . | . | . | . | . | . |
| 21-225_27E7_HC | . | . | . | . | . | . | . | . | . | . | . | . | . | . | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | . |
| 21-225_5E5_HC | . | . | . | . | . | . | . | . | . | . | . | . | . | . | . | . | . |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | S | - |
| 21-225_17H3_HC | . | . | . | . | . | . | . | . | . | . | . | . | . | . | . | N | . |
| 21-225_20B2_HC | . | . | . | . | . | . | . | . | . | . | . | . | . | . | . | N | . |
| 21-225_22F4_HC | . | . | . | . | . | . | . | . | . | . | . | . | . | . | . | D | . |
| 21-225_24B6_HC | . | P | . | . | . | . | . | . | . | . | . | . | I | . | . | N | . |
| 21-225_23A10_HC | . | . | . | . | . | . | . | . | . | . | . | . | . | . | . | . | . |
| 21-225_25A2_HC | . | . | . | . | . | . | . | . | . | . | . | . | . | . | . | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | . | . | . | . | . |
| 21-225_29H4_HC | . | . | . | . | . | . | . | . | . | . | . | . | . | . | . | . | . |
| 21-225_26A11_HC | . | . | . | . | . | . | . | . | . | . | . | . | . | . | . | N | . |
| 21-225_26G7_HC | . | . | . | . | . | . | . | . | . | . | . | . | . | . | . | . | . |
| 21-225_27E7_HC | . | . | . | . | . | . | . | . | . | . | . | . | . | . | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | D | . |
| 21-225_10E9_HC | . | . | . | . | . | . | . | . | . | . | . | . | . | . | . | N | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | L | . | . | . | N | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | N | N | . |
| 21-225_15C11_HC | . | . | . | . | . | . | . | . | . | . | . | . | . | . | . | D | . |
| 21-225_5E5_HC | . | . | . | . | . | . | . | . | . | . | . | . | . | . | . | N | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | | | H_FR2 | | |
| CONSENSUS | - | - | - | - | Y | V | M | H | W | V | R | Q | A | P | G | K | G |
| 21-225_17H3_HC | . | . | . | . | C | . | . | . | . | . | . | . | . | . | . | . | . |
| 21-225_20B2_HC | . | . | . | . | . | . | I | . | . | . | . | . | . | . | . | . | . |
| 21-225_22F4_HC | . | . | . | . | . | G | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | G | . | . | . | . | . | . | . | . | . | . | . |
| 21-225_25A2_HC | . | . | . | . | . | G | . | . | . | . | . | . | . | . | . | . | . |
| 21-225_25D12_HC | . | . | . | . | D | G | . | . | . | . | . | . | . | . | . | . | . |
| 21-225_32H2_HC | . | . | . | . | . | G | . | . | . | . | . | . | . | . | . | . | . |
| 21-225_29H4_HC | . | . | . | . | . | G | I | . | . | . | . | . | . | . | . | . | . |
| 21-225_26A11_HC | . | . | . | . | C | . | . | . | . | . | . | . | . | . | . | . | . |
| 21-225_26G7_HC | . | . | . | . | . | G | . | . | . | . | . | . | . | . | . | . | . |
| 21-225_27E7_HC | . | . | . | . | . | G | . | . | . | . | . | . | . | . | . | . | . |
| 21-225_4C5_HC | . | . | . | . | . | . | L | . | . | . | . | . | . | . | . | . | . |
| 21-225_14E10_HC | . | . | . | . | . | G | . | . | . | . | . | . | . | . | . | . | . |
| 21-225_10E9_HC | . | . | . | . | . | . | I | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | . |
| 21-225_5E5_HC | . | . | . | . | . | . | . | . | . | . | . | . | . | . | . | . | . |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | A | V | I | W | Y | D | - | - | - | G | S | N | K |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | . | . | . | . | . |
| 21-225_25A2_HC | . | . | . | . | . | . | . | . | . | . | . | . | . | . | . | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | . | . | . | . | . |
| 21-225_29H4_HC | . | . | . | . | . | . | . | . | . | . | . | . | . | . | . | . | . |
| 21-225_26A11_HC | . | . | . | . | . | . | . | . | . | . | . | . | . | . | . | . | . |
| 21-225_26G7_HC | . | . | . | . | . | . | . | . | . | . | . | . | . | . | . | . | . |
| 21-225_27E7_HC | . | . | . | . | . | . | . | . | . | . | . | . | . | . | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | . |
| 21-225_5E5_HC | . | . | . | . | . | . | . | . | . | . | . | . | . | . | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_17H3_HC | . | . | . | | . | | . | | . | . | . | . | . | . | . | . | . |
| 21-225_20B2_HC | . | . | . | | . | | . | | . | . | . | . | . | . | . | . | . |
| 21-225_22F4_HC | . | . | V | | . | | . | | . | . | . | . | . | . | . | . | . |
| 21-225_24B6_HC | . | . | . | | . | | . | | . | . | . | . | . | . | . | . | . |
| 21-225_23A10_HC | . | . | . | | . | | . | | . | . | . | . | . | . | . | . | . |
| 21-225_25A2_HC | . | . | . | | . | | . | | . | . | . | . | . | . | . | . | . |
| 21-225_25D12_HC | . | . | . | | . | | . | | . | . | . | . | . | . | . | . | . |
| 21-225_32H2_HC | . | . | . | | . | | . | | . | . | . | . | . | . | . | . | . |
| 21-225_29H4_HC | . | . | . | | . | | . | | . | . | . | . | . | . | . | . | . |
| 21-225_26A11_HC | . | . | . | | . | | . | | . | . | . | . | . | . | . | . | . |
| 21-225_26G7_HC | . | . | . | | . | | . | | . | . | . | . | . | . | . | . | . |
| 21-225_27E7_HC | . | . | . | | . | | . | | . | . | . | . | . | . | . | . | . |
| 21-225_4C5_HC | . | . | . | | . | | . | | . | . | . | . | . | . | . | . | . |
| 21-225_14E10_HC | . | . | . | | . | | . | | . | . | . | . | . | . | . | . | . |
| 21-225_10E9_HC | . | . | . | | . | | . | | . | . | . | . | . | . | . | . | . |
| 21-225_2G4_HC | . | . | . | | . | | . | | . | . | . | . | . | . | . | . | . |
| 21-225_3H10_HC | . | . | . | | . | | . | | . | . | . | . | . | . | . | . | . |
| 21-225_7C9_HC | . | . | . | | . | | . | | . | . | . | . | . | . | . | . | . |
| 21-225_4C12_HC | . | . | . | | . | | . | | . | . | . | . | . | . | . | . | . |
| 21-225_7G4_HC | . | . | . | | . | | . | | . | . | . | . | . | . | . | . | . |
| 21-225_15C11_HC | . | . | . | | . | | . | | . | . | . | . | . | . | . | . | . |
| 21-225_5E5_HC | . | . | . | | . | | . | | . | . | . | . | . | . | . | . | . |

**FIGURE 56**

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | H_FR3 | | | | | | | | | | | |
| CONSENSUS | K | N | T | L | Y | L | Q | M | N | S | L | R | A | E | D | T | A |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | . | . | . | . | . |
| 21-225_25A2_HC | . | . | . | . | . | . | . | . | . | . | . | . | . | . | . | . | . |
| 21-225_25D12_HC | . | . | . | . | . | . | . | . | . | . | . | . | . | . | . | . | . |
| 21-225_32H2_HC | . | . | . | . | **N** | . | . | . | . | . | . | . | . | . | . | . | **G** |
| 21-225_29H4_HC | . | . | . | . | . | . | . | . | . | . | . | . | . | . | . | . | . |
| 21-225_26A11_HC | . | . | . | . | . | . | . | . | . | . | . | . | . | . | . | . | . |
| 21-225_26G7_HC | **N** | . | . | . | . | . | . | . | . | . | . | . | . | . | . | . | . |
| 21-225_27E7_HC | . | . | . | . | . | . | . | . | . | . | . | . | . | . | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | **T** | . | . | . | . | . | . | . | . |
| 21-225_15C11_HC | . | . | . | . | . | . | . | . | . | . | . | . | . | . | . | . | . |
| 21-225_5E5_HC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | E | K | Y | S | S | S | W | - | - | - | - |
| 21-225_17H3_HC | . | . | . | . | . | . | . | . | . | . | . | . | | . | . | . | . |
| 21-225_20B2_HC | . | . | S | . | . | . | . | . | . | . | . | . | | . | . | . | . |
| 21-225_22F4_HC | . | . | . | . | . | . | . | . | . | . | . | . | | . | . | . | . |
| 21-225_24B6_HC | . | . | . | . | . | . | . | E | . | . | . | G | | . | . | . | . |
| 21-225_23A10_HC | . | . | . | . | . | . | . | R | . | . | . | G | | . | . | . | . |
| 21-225_25A2_HC | . | . | . | . | . | . | R | Y | S | . | . | W | - | . | . | . | . |
| 21-225_25D12_HC | . | . | . | . | . | . | . | R | . | . | . | . | | . | . | . | . |
| 21-225_32H2_HC | . | . | . | . | . | . | R | Y | S | . | . | W | - | . | . | . | . |
| 21-225_29H4_HC | . | . | . | . | . | . | R | Y | S | . | . | W | - | . | . | . | . |
| 21-225_26A11_HC | . | . | . | . | . | . | . | E | . | . | . | G | | . | . | . | . |
| 21-225_26G7_HC | . | . | . | . | . | . | R | Y | S | . | . | W | - | . | . | . | . |
| 21-225_27E7_HC | . | . | . | . | . | . | . | D | S | Y | C | . | S | T | S | C | . |
| 21-225_4C5_HC | . | . | . | . | . | . | . | . | . | T | . | . | | . | . | . | . |
| 21-225_14E10_HC | . | . | . | . | . | . | . | . | . | . | . | . | | . | . | . | . |
| 21-225_10E9_HC | . | . | . | . | . | . | . | . | . | . | . | . | | . | . | . | . |
| 21-225_2G4_HC | . | . | . | . | . | . | . | . | . | . | . | . | | . | . | . | . |
| 21-225_3H10_HC | . | . | . | . | . | . | . | R | . | . | . | G | | . | . | . | . |
| 21-225_7C9_HC | . | . | . | . | . | . | . | . | . | . | . | . | | . | . | . | . |
| 21-225_4C12_HC | . | . | . | . | . | . | . | . | . | . | . | . | | . | . | . | . |
| 21-225_7G4_HC | . | . | . | . | . | . | . | . | . | . | . | N | | . | . | . | . |
| 21-225_15C11_HC | . | . | . | . | . | . | . | . | . | . | . | . | | . | . | . | . |
| 21-225_5E5_HC | . | . | . | . | T | . | . | V | . | . | . | G | | . | . | . | . |

**FIGURE 56**

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | Y | D | Y | G | M |
| 21-225_17H3_HC | . | . | . | . | . | . | . | . | . | . | . | . | . | . | . | . | |
| 21-225_20B2_HC | . | . | . | . | . | . | . | . | . | . | . | . | . | . | . | . | |
| 21-225_22F4_HC | . | . | . | . | . | . | . | . | . | . | . | . | . | . | . | . | |
| 21-225_24B6_HC | . | . | . | . | . | . | . | . | . | . | . | . | . | . | . | . | |
| 21-225_23A10_HC | . | . | . | . | . | . | . | . | . | . | . | . | H | . | . | . | |
| 21-225_25A2_HC | . | . | . | . | . | . | . | . | . | . | . | . | - | S | G | . | |
| 21-225_25D12_HC | . | . | . | . | . | . | . | . | . | . | . | . | . | . | . | . | |
| 21-225_32H2_HC | . | . | . | . | . | . | . | . | . | . | . | . | - | T | G | . | |
| 21-225_29H4_HC | . | . | . | . | . | . | . | . | . | . | . | . | - | S | G | . | |
| 21-225_26A11_HC | . | . | . | . | . | . | . | . | . | . | . | . | . | . | . | . | |
| 21-225_26G7_HC | . | . | . | . | . | . | . | . | . | . | . | . | - | S | G | . | |
| 21-225_27E7_HC | . | . | . | . | . | . | . | . | . | P | Y | Y | . | Y | . | . | |
| 21-225_4C5_HC | . | . | . | . | . | . | . | . | . | . | . | . | . | . | . | . | |
| 21-225_14E10_HC | . | . | . | . | . | . | . | . | . | . | . | . | . | . | . | . | |
| 21-225_10E9_HC | . | . | . | . | . | . | . | . | . | . | . | . | . | . | . | . | |
| 21-225_2G4_HC | . | . | . | . | . | . | . | . | . | . | . | . | . | . | . | . | |
| 21-225_3H10_HC | . | . | . | . | . | . | . | . | . | . | . | . | . | . | . | . | L |
| 21-225_7C9_HC | . | . | . | . | . | . | . | . | . | . | . | . | . | . | . | . | |
| 21-225_4C12_HC | . | . | . | . | . | . | . | . | . | . | . | . | . | . | . | . | |
| 21-225_7G4_HC | . | . | . | . | . | . | . | . | . | . | . | . | . | . | . | . | |
| 21-225_15C11_HC | . | . | . | . | . | . | . | . | . | . | . | . | . | . | . | . | L |
| 21-225_5E5_HC | . | . | . | . | . | . | . | . | . | . | . | . | . | . | . | . | |

3500

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | Y | D | Y | G | M |
| 21-225_17H3_HC | . | . | . | . | . | . | . | . | . | . | . | . | . | . | . | . | |
| 21-225_20B2_HC | . | . | . | . | . | . | . | . | . | . | . | . | . | . | . | . | |
| 21-225_22F4_HC | . | . | . | . | . | . | . | . | . | . | . | . | . | . | . | . | |
| 21-225_24B6_HC | . | . | . | . | . | . | . | . | . | . | . | . | . | . | . | . | |
| 21-225_23A10_HC | . | . | . | . | . | . | . | . | . | . | . | . | H | . | . | . | |
| 21-225_25A2_HC | . | . | . | . | . | . | . | . | . | . | . | . | - | S | G | . | |
| 21-225_25D12_HC | . | . | . | . | . | . | . | . | . | . | . | . | . | . | . | . | |
| 21-225_32H2_HC | . | . | . | . | . | . | . | . | . | . | . | . | - | T | G | . | |
| 21-225_29H4_HC | . | . | . | . | . | . | . | . | . | . | . | . | - | S | G | . | |
| 21-225_26A11_HC | . | . | . | . | . | . | . | . | . | . | . | . | . | . | . | . | |
| 21-225_26G7_HC | . | . | . | . | . | . | . | . | . | . | . | . | - | S | G | . | |
| 21-225_27E7_HC | . | . | . | . | . | . | . | . | . | P | Y | Y | . | Y | . | . | |
| 21-225_4C5_HC | . | . | . | . | . | . | . | . | . | . | . | . | . | . | . | . | |
| 21-225_14E10_HC | . | . | . | . | . | . | . | . | . | . | . | . | . | . | . | . | |
| 21-225_10E9_HC | . | . | . | . | . | . | . | . | . | . | . | . | . | . | . | . | |
| 21-225_2G4_HC | . | . | . | . | . | . | . | . | . | . | . | . | . | . | . | . | |
| 21-225_3H10_HC | . | . | . | . | . | . | . | . | . | . | . | . | . | . | . | . | L |
| 21-225_7C9_HC | . | . | . | . | . | . | . | . | . | . | . | . | . | . | . | . | |
| 21-225_4C12_HC | . | . | . | . | . | . | . | . | . | . | . | . | . | . | . | . | |
| 21-225_7G4_HC | . | . | . | . | . | . | . | . | . | . | . | . | . | . | . | . | |
| 21-225_15C11_HC | . | . | . | . | . | . | . | . | . | . | . | . | . | . | . | . | L |
| 21-225_5E5_HC | . | . | . | . | . | . | . | . | . | . | . | . | . | . | . | . | |

**FIGURE 56**

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | Y | D | Y | G | M |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | H | . | . | . | . |
| 21-225_25A2_HC | . | . | . | . | . | . | . | . | . | . | . | . | - | S | G | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | - | T | G | . | . |
| 21-225_29H4_HC | . | . | . | . | . | . | . | . | . | . | . | . | - | S | G | . | . |
| 21-225_26A11_HC | . | . | . | . | . | . | . | . | . | . | . | . | . | . | . | . | . |
| 21-225_26G7_HC | . | . | . | . | . | . | . | . | . | . | . | . | - | S | G | . | . |
| 21-225_27E7_HC | . | . | . | . | . | . | . | . | . | P | Y | Y | . | Y | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | L |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | L |
| 21-225_5E5_HC | . | . | . | . | . | . | . | . | . | . | . | . | . | . | . | . | . |

**FIGURE 56**

| Reference # | 137 | 138 | 139 | 140 | 141 | 142 | 143 | 144 | 145 | 146 | 147 | 148 | 149 |
|---|---|---|---|---|---|---|---|---|---|---|---|---|
| | | | | | | | | H_FR4 | | | | | |
| CONSENSUS | D | V | W | G | Q | G | T | T | V | T | V | S | S |
| 21-225_17H3_HC | . | . | . | . | . | . | . | | . | . | . | . | . |
| 21-225_20B2_HC | . | . | . | . | . | . | . | | . | . | . | . | . |
| 21-225_22F4_HC | . | . | . | . | . | . | . | | . | . | . | . | . |
| 21-225_24B6_HC | . | . | . | . | . | . | . | | . | . | . | . | . |
| 21-225_23A10_HC | . | . | . | . | . | . | . | | . | . | . | . | . |
| 21-225_25A2_HC | . | . | . | . | . | . | . | | . | . | . | . | . |
| 21-225_25D12_HC | . | . | . | . | . | . | . | | . | . | . | . | . |
| 21-225_32H2_HC | . | . | . | . | . | . | . | | . | . | . | . | . |
| 21-225_29H4_HC | . | . | . | . | . | . | . | | . | . | . | . | . |
| 21-225_26A11_HC | . | . | . | . | . | . | . | | . | . | . | . | . |
| 21-225_26G7_HC | . | . | . | . | . | . | . | | . | . | . | . | . |
| 21-225_27E7_HC | . | . | . | . | . | . | . | | . | . | . | . | . |
| 21-225_4C5_HC | . | . | . | . | . | . | . | | . | . | . | . | . |
| 21-225_14E10_HC | . | . | . | . | . | . | . | | . | . | . | . | . |
| 21-225_10E9_HC | . | . | . | . | . | . | . | | . | . | . | . | . |
| 21-225_2G4_HC | . | . | . | . | **H** | . | . | | . | . | . | . | . |
| 21-225_3H10_HC | . | . | . | . | . | . | . | | . | . | . | . | . |
| 21-225_7C9_HC | . | . | . | . | . | . | . | | . | . | . | . | . |
| 21-225_4C12_HC | . | . | . | . | **H** | . | . | | . | . | . | . | . |
| 21-225_7G4_HC | . | . | . | . | . | . | . | | . | . | . | . | . |
| 21-225_15C11_HC | . | . | . | . | . | . | . | | . | . | . | . | . |
| 21-225_5E5_HC | . | . | . | . | . | . | . | | . | . | . | . | . |

**FIGURE 56**

Table 31

| Reference # | 1 | 2 | 3 | 4 | 5 | 6 | 7 | 8 | 9 | 10 | 11 | 12 | 13 | 14 | 15 | 16 | 17 H_FR1 |
|---|---|---|---|---|---|---|---|---|---|---|---|---|---|---|---|---|---|
| CONSENSUS | Q | V | Q | L | V | E | S | - | G | G | G | V | V | Q | P | G | R |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | . | . |
| 21-225_25A9_HC | . | . | . | . | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | . | . | . | . | . | . | . | . | . | . | . |
| 21-225_29D8_HC | . | . | . | . | . | . | . | . | . | . | . | . | . | . | . | . | . |
| 21-225_30F3_HC | . | . | . | . | . | . | . | . | . | . | . | . | . | . | . | . | . |
| 21-225_33B1_HC | . | . | . | . | . | . | . | . | . | . | . | . | . | . | . | . | . |
| 21-225_15B11_HC | . | . | . | . | . | . | . | . | . | . | . | . | . | . | . | . | . |
| 21-225_14C2_HC | . | . | . | . | . | . | . | . | . | . | . | . | . | . | . | . | . |
| 21-225_4C8_HC | . | . | . | . | . | . | . | . | . | . | . | . | . | . | . | . | . |
| 21-225_4F12_HC | . | . | . | . | . | . | . | . | . | . | . | . | . | . | . | . | . |
| 21-225_15H10_HC | . | . | . | . | . | . | . | . | . | . | . | . | . | . | . | . | . |
| 21-225_16H4_HC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | D | - |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | S | . |
| 21-225_25A9_HC | . | . | . | . | . | . | . | . | . | . | . | . | . | . | . | S | . |
| 21-225_29D9_HC | . | . | K | . | . | . | S | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | . | . | . | . | . | . | . | . | . | S | . |
| 21-225_29D8_HC | . | . | . | . | . | . | . | . | . | . | . | . | . | . | R | . | . |
| 21-225_30F3_HC | . | . | . | . | . | . | . | . | . | . | . | . | . | . | . | S | . |
| 21-225_33B1_HC | . | . | . | . | . | . | . | . | . | . | . | . | . | . | . | . | . |
| 21-225_15B11_HC | . | . | . | . | . | . | . | . | . | . | . | . | . | . | . | N | . |
| 21-225_14C2_HC | . | . | . | . | . | . | . | . | . | . | . | . | . | . | . | . | . |
| 21-225_4C8_HC | . | . | . | . | . | . | . | . | . | . | . | . | . | . | . | N | . |
| 21-225_4F12_HC | . | . | . | . | . | . | . | . | . | . | . | . | . | . | . | S | . |
| 21-225_15H10_HC | . | . | . | . | . | . | . | . | . | . | . | . | . | . | . | N | . |
| 21-225_16H4_HC | . | . | . | . | . | . | . | . | . | . | . | . | . | . | . | N | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | | | | H_FR2 | |
| CONSENSUS | - | - | - | - | Y | G | M | H | W | V | R | Q | A | P | G | K | G |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | . | . |
| 21-225_25A9_HC | . | . | . | . | . | . | . | . | . | . | . | . | . | . | . | . | . |
| 21-225_29D9_HC | . | . | . | . | . | . | I | . | . | . | . | . | . | . | . | . | . |
| 21-225_25G5_HC | . | . | . | . | . | . | I | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | I | . | . | . | . | . | . | . | . | . | . |
| 21-225_29D8_HC | . | . | . | . | . | . | I | . | . | . | . | . | . | . | . | . | . |
| 21-225_30F3_HC | . | . | . | . | . | . | . | . | . | . | . | . | . | . | . | . | . |
| 21-225_33B1_HC | . | . | . | . | . | . | . | . | . | . | . | . | . | A | . | . | . |
| 21-225_15B11_HC | . | . | . | . | . | . | . | . | . | . | . | . | . | . | . | . | . |
| 21-225_14C2_HC | . | . | . | . | . | . | . | . | . | . | . | . | . | . | . | . | . |
| 21-225_4C8_HC | . | . | . | . | . | . | . | . | . | . | . | . | . | . | . | . | . |
| 21-225_4F12_HC | . | . | . | . | . | . | . | . | . | . | . | . | . | . | . | . | . |
| 21-225_15H10_HC | . | . | . | . | . | . | . | . | . | . | . | . | . | . | . | . | . |
| 21-225_16H4_HC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | A | V | I | W | Y | D | - | - | - | E | S | N | K |
| 21-225_18H12_HC | . | . | M | . | . | . | . | . | . | . | . | . | . | V | T | . | . |
| 21-225_22B7_HC | . | . | . | . | . | . | . | . | . | E | . | . | . | . | N | . | Q |
| 21-225_25A9_HC | . | . | . | . | . | . | . | . | . | . | . | . | . | V | T | . | . |
| 21-225_29D9_HC | . | . | . | . | . | . | . | . | . | . | . | . | . | . | . | . | N |
| 21-225_25G5_HC | . | . | . | . | . | . | . | . | . | . | . | . | . | G | N | . | . |
| 21-225_28B8_HC | . | . | T | . | . | . | . | . | . | . | . | . | . | A | N | . | . |
| 21-225_28A9_HC | . | . | . | . | . | . | . | . | F | . | . | . | . | G | . | . | . |
| 21-225_29D8_HC | . | . | . | . | . | . | . | . | F | . | . | . | . | G | T | . | . |
| 21-225_30F3_HC | . | . | . | . | . | . | . | . | . | . | . | . | . | V | . | . | . |
| 21-225_33B1_HC | . | . | . | . | . | . | . | . | . | . | . | . | . | R | N | . | . |
| 21-225_15B11_HC | . | . | . | . | . | . | . | . | . | E | . | . | . | . | . | . | . |
| 21-225_14C2_HC | . | . | . | . | . | . | . | . | . | . | . | . | . | . | . | . | . |
| 21-225_4C8_HC | . | . | . | . | . | . | . | . | . | E | . | . | . | . | N | . | Q |
| 21-225_4F12_HC | . | . | . | . | . | . | . | . | . | . | . | . | . | V | T | . | . |
| 21-225_15H10_HC | . | . | . | . | . | . | . | . | . | . | . | . | . | . | . | . | . |
| 21-225_16H4_HC | . | . | . | . | . | . | . | . | . | . | . | . | . | V | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | . | . |
| 21-225_25A9_HC | . | . | T | G | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | F |
| 21-225_28A9_HC | . | . | . | . | . | . | . | . | . | . | . | . | . | . | . | . | . |
| 21-225_29D8_HC | . | . | . | . | . | . | . | . | . | . | . | . | . | . | . | . | . |
| 21-225_30F3_HC | . | . | . | . | . | . | . | . | . | . | . | . | . | . | . | . | . |
| 21-225_33B1_HC | . | . | . | . | . | . | . | . | . | . | . | . | . | . | . | . | . |
| 21-225_15B11_HC | . | . | T | . | . | . | . | . | . | . | . | . | . | . | . | . | . |
| 21-225_14C2_HC | . | . | E | . | . | . | . | . | . | . | . | . | . | . | . | . | . |
| 21-225_4C8_HC | . | . | . | . | . | . | . | . | . | . | . | . | . | . | . | . | . |
| 21-225_4F12_HC | K | . | . | . | . | . | . | . | . | . | . | . | . | . | . | . | . |
| 21-225_15H10_HC | . | . | E | . | . | . | . | . | . | . | . | . | . | . | . | . | . |
| 21-225_16H4_HC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | | H_FR3 | | | | | | | | | | |
| CONSENSUS | K | N | T | L | Y | L | Q | M | N | S | L | R | A | E | D | T | A |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | . | . |
| 21-225_25A9_HC | . | . | . | . | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | N | . | . | . | . | . | . | . |
| 21-225_28B8_HC | . | . | . | V | . | . | . | . | . | . | . | . | . | . | . | . | . |
| 21-225_28A9_HC | . | . | . | . | F | . | . | . | . | . | . | . | V | . | . | . | . |
| 21-225_29D8_HC | . | . | . | . | . | . | . | . | . | . | . | . | . | . | . | . | . |
| 21-225_30F3_HC | . | . | . | . | . | . | . | . | . | . | . | . | . | . | . | . | . |
| 21-225_33B1_HC | . | . | . | . | . | . | . | . | . | . | . | . | . | . | . | . | . |
| 21-225_15B11_HC | . | . | . | . | . | . | . | . | . | . | . | . | P | . | . | . | . |
| 21-225_14C2_HC | . | . | . | . | . | . | . | . | K | . | . | . | . | . | . | . | G |
| 21-225_4C8_HC | . | . | . | . | . | . | . | . | . | . | . | T | . | . | . | . | . |
| 21-225_4F12_HC | . | . | . | . | . | . | . | . | . | . | . | T | P | . | N | . | G |
| 21-225_15H10_HC | . | . | . | . | . | . | . | . | . | . | . | . | . | . | . | . | . |
| 21-225_16H4_HC | . | . | M | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | E | I | G | W | - | - | - | - | - | - | - |
| 21-225_18H12_HC | . | . | . | . | . | . | . | V | . | . | . | . | . | . | . | . | . |
| 21-225_22B7_HC | . | . | . | . | . | . | . | V | . | F | . | . | . | . | . | . | . |
| 21-225_25A9_HC | . | . | . | . | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | . | . | . | . | . | . | . | . | . | . | . |
| 21-225_29D8_HC | . | . | . | . | G | . | . | . | . | . | . | . | . | . | . | . | . |
| 21-225_30F3_HC | . | . | . | . | . | . | . | M | . | . | . | . | . | . | . | . | . |
| 21-225_33B1_HC | . | . | . | . | . | . | . | M | . | . | . | . | . | . | . | . | . |
| 21-225_15B11_HC | . | . | . | . | . | . | . | V | . | F | . | . | . | . | . | . | . |
| 21-225_14C2_HC | . | . | . | . | . | . | . | V | . | F | . | . | . | . | . | . | . |
| 21-225_4C8_HC | . | . | . | S | P | . | . | M | . | F | . | . | . | . | . | . | . |
| 21-225_4F12_HC | G | . | E | N | Q | . | . | K | . | G | . | . | . | . | . | . | . |
| 21-225_15H10_HC | . | . | . | . | V | . | . | V | . | F | . | . | . | . | . | . | . |
| 21-225_16H4_HC | . | . | . | . | . | . | . | M | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | H_CDR3 | | | | | | | | | | | | | | |
| CONSENSUS | - | - | - | - | - | - | - | - | - | - | - | - | - | - | - | L | D |
| 21-225_18H12_HC | . | . | . | . | . | . | . | . | . | . | . | . | . | . | . | Y | E |
| 21-225_22B7_HC | . | . | . | . | . | . | . | . | . | . | . | . | . | . | . | R | S |
| 21-225_25A9_HC | . | . | . | . | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | . | . | . | . | . | . | . | . | . | . | . |
| 21-225_29D8_HC | . | . | . | . | . | . | . | . | . | . | . | . | . | . | . | . | . |
| 21-225_30F3_HC | . | . | . | . | . | . | . | . | . | . | . | . | . | . | . | Y | . |
| 21-225_33B1_HC | . | . | . | . | . | . | . | . | . | . | . | . | . | . | . | Y | . |
| 21-225_15B11_HC | . | . | . | . | . | . | . | . | . | . | . | . | . | . | . | T | E |
| 21-225_14C2_HC | . | . | . | . | . | . | . | . | . | . | . | . | . | . | . | R | S |
| 21-225_4C8_HC | . | . | . | . | . | . | . | . | . | . | . | . | . | . | . | . | S |
| 21-225_4F12_HC | . | . | . | . | . | . | . | . | . | . | . | . | . | . | . | . | F |
| 21-225_15H10_HC | . | . | . | . | . | . | . | . | . | . | . | . | . | . | . | . | S |
| 21-225_16H4_HC | . | . | . | . | . | . | . | . | . | . | . | . | . | . | . | T | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | | H_FR4 | | | | | |
| CONSENSUS | D | Y | W | G | Q | G | T | L | V | T | V | S | S |
| 21-225_18H12_HC | . | . | . | . | . | . | . | . | . | . | . | . | . |
| 21-225_22B7_HC | . | . | . | . | . | . | . | . | . | . | . | . | . |
| 21-225_25A9_HC | . | . | . | . | . | . | . | . | . | . | . | . | . |
| 21-225_29D9_HC | . | . | . | . | . | . | . | Q | . | . | . | . | . |
| 21-225_25G5_HC | . | . | . | . | . | . | . | . | . | . | . | . | . |
| 21-225_28B8_HC | . | . | . | . | . | . | . | . | . | . | . | . | . |
| 21-225_28A9_HC | . | . | . | . | . | . | . | . | . | . | . | . | . |
| 21-225_29D8_HC | . | . | . | . | . | . | . | . | . | . | . | . | . |
| 21-225_30F3_HC | . | . | . | . | . | . | . | . | . | . | . | . | . |
| 21-225_33B1_HC | . | . | . | . | . | . | . | . | . | . | . | . | . |
| 21-225_15B11_HC | . | . | . | . | . | . | . | . | . | . | . | . | . |
| 21-225_14C2_HC | . | . | . | . | . | . | . | . | . | . | . | . | . |
| 21-225_4C8_HC | . | . | . | . | . | . | . | . | . | . | . | . | . |
| 21-225_4F12_HC | . | . | . | . | . | . | . | . | . | . | . | . | . |
| 21-225_15H10_HC | . | . | . | . | . | . | I | . | . | . | . | . | . |
| 21-225_16H4_HC | . | C | . | . | . | . | . | . | . | . | . | . | . |

FIGURE 56

Table 32

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | V | Q | L | V | E | S | - | G | G | G | V | V | Q | P | G | R |
| 21-225_16F10_HC | . | . | . | . | . | . | . | . | . | . | . | . | . | . | . | . | . |
| 21-225_18A5_HC | . | . | . | . | . | . | . | . | . | . | . | . | . | . | . | . | . |
| 21-225_20E5_HC | . | . | . | . | . | . | . | . | . | . | . | . | . | . | . | . | . |
| 21-225_18A1_HC | . | . | . | . | . | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | . | . | . | . | . | . |
| 21-225_16F11_HC | . | . | . | M | . | . | . | . | . | . | . | . | . | . | . | . | . |
| 21-225_18C2_HC | . | . | . | . | . | . | . | . | . | . | . | . | . | . | . | . | . |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | . | K |
| 21-225_18F2_HC | . | . | . | . | . | . | . | . | . | . | . | . | . | . | . | . | . |
| 21-225_17B5_HC | . | . | . | . | . | . | . | . | . | . | . | . | . | . | . | . | . |
| 21-225_17E5_HC | . | . | . | . | . | . | . | . | . | . | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | . | . |
| 21-225_20E12_HC | . | . | . | . | . | . | . | . | . | . | . | . | . | . | . | . | . |
| 21-225_20D10_HC | . | . | . | . | . | . | . | . | . | . | . | . | . | . | . | . | . |
| 21-225_22A4_HC | . | . | . | . | . | . | . | . | . | . | . | . | . | . | . | . | . |
| 21-225_20H12_HC | . | . | . | . | . | . | . | . | . | . | . | . | . | . | . | . | . |
| 21-225_21H8_HC | . | . | . | . | . | . | . | . | . | . | . | . | . | . | S | . | . |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | . | . |
| 21-225_23H11_HC | . | E | . | . | . | . | . | . | . | . | . | . | . | . | . | . | . |
| 21-225_21F7_HC | . | . | . | . | . | . | . | . | . | . | . | . | . | . | . | . | . |
| 21-225_22F9_HC | . | . | . | M | . | . | . | . | . | . | . | . | . | . | . | . | . |
| 21-225_21G2_HC | . | . | . | . | . | . | . | . | . | . | . | . | . | . | . | . | . |
| 21-225_22F2_HC | . | . | . | . | . | . | . | . | . | . | . | . | . | . | . | . | . |
| 21-225_23F12_HC | . | . | . | . | . | . | . | . | . | . | . | . | . | . | . | . | . |
| 21-225_28F5_HC | . | . | . | . | . | . | . | . | . | . | . | . | . | . | . | . | . |
| 21-225_29G4_HC | . | . | . | . | . | . | . | . | . | . | . | . | . | . | . | . | . |
| 21-225_27D5_HC | . | . | . | . | . | . | . | . | . | . | . | . | . | . | . | . | . |
| 21-225_28C7_HC | . | . | . | . | . | . | . | . | . | . | . | . | . | . | . | . | . |
| 21-225_25B6_HC | . | . | . | . | . | . | . | . | . | . | . | . | . | . | . | . | . |
| 21-225_26A2_HC | . | . | . | . | . | . | . | . | . | . | . | . | . | . | . | . | . |
| 21-225_29H6_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | . | . | . | . | . | . | . | . | . | . | . | . |
| 21-225_29D7_HC | . | . | . | . | . | . | . | . | . | . | . | . | . | . | . | . | . |
| 21-225_29B8_HC | . | . | . | . | . | . | . | . | . | . | . | . | . | . | . | . | . |

**FIGURE 56**

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | V | Q | L | V | E | S | - | G | G | G | V | V | Q | P | G | R |
| 21-225_25H11_HC | . | . | . | . | . | . | . | . | . | . | . | . | . | . | . | . | . |
| 21-225_27F2_HC | . | E | . | . | . | . | . | . | . | . | . | . | . | . | . | . | . |
| 21-225_27C3_HC | . | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | L | . | . | . | . | . | . | . | . | . | . | . | . | . | . | . | K |
| 21-225_7H11_HC | . | . | . | . | . | . | . | . | . | . | . | . | . | . | . | . | . |
| 21-225_14E3_HC | . | . | . | . | . | . | . | . | . | . | . | . | . | . | . | . | . |
| 21-225_9A1_HC | . | . | . | V | . | . | . | . | . | . | . | . | . | . | . | . | . |
| 21-225_15E3_HC | . | . | . | . | . | . | . | . | . | . | T | . | . | . | . | . | . |
| 21-225_16F4_HC | . | . | . | . | . | . | . | . | . | . | . | . | . | . | . | . | . |
| 21-225_16F6_HC | . | . | . | . | . | . | . | . | . | . | . | . | . | . | . | . | . |
| 21-225_2B2_HC | . | . | . | . | . | . | . | . | . | . | . | . | . | . | . | . | . |
| 21-225_13E6_HC | . | . | . | . | . | . | . | . | . | . | . | . | . | . | . | . | . |
| 21-225_16A5_HC | . | . | . | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | . | K |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | . | . | . | . | . | . | . | . | . | . | . | . | . | . | . |
| 21-225_12E6_HC | . | . | . | . | . | . | . | . | . | . | . | . | . | . | . | . | . |
| 21-225_16G7_HC | . | . | . | . | . | . | . | . | . | . | . | . | . | . | . | . | . |
| 21-225_1C12_HC | . | . | . | . | . | . | . | . | . | . | . | . | . | . | . | . | . |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | D | - |
| 21-225_16F10_HC | . | . | . | . | . | . | . | . | . | . | . | . | . | . | . | . | . |
| 21-225_18A5_HC | . | . | . | . | . | Y | T | . | . | . | . | . | . | . | . | . | . |
| 21-225_20E5_HC | . | . | . | . | . | . | . | . | . | . | . | . | . | . | . | . | . |
| 21-225_18A1_HC | . | . | . | . | . | . | V | . | . | . | . | . | . | . | . | S | . |
| 21-225_19E1_HC | . | . | . | . | . | . | . | . | . | . | . | . | . | . | . | N | . |
| 21-225_17A10_HC | . | . | . | . | . | . | . | . | . | . | . | . | . | . | . | N | . |
| 21-225_18E8_HC | . | . | . | . | . | . | . | . | . | . | . | . | . | . | . | N | . |
| 21-225_16F11_HC | . | . | . | . | . | . | E | . | . | . | . | . | . | . | . | S | . |
| 21-225_18C2_HC | . | . | . | . | . | . | V | . | . | . | . | . | . | . | . | . | . |
| 21-225_20A7_HC | . | . | . | . | . | . | . | . | . | . | . | . | . | . | . | N | . |
| 21-225_17C7_HC | . | . | . | . | . | . | . | . | . | . | . | . | . | . | . | . | . |
| 21-225_18F2_HC | . | . | . | . | . | . | . | . | . | . | . | . | . | . | . | . | . |
| 21-225_17B5_HC | . | . | . | . | . | . | . | . | . | . | . | . | . | . | . | . | . |
| 21-225_17E5_HC | . | . | . | . | . | . | . | . | . | . | . | . | . | . | . | . | . |
| 21-225_20D5_HC | . | . | . | . | . | . | . | . | . | . | . | . | . | . | . | S | . |
| 21-225_21G11_HC | . | . | . | . | . | . | . | . | . | . | . | . | . | . | . | . | . |
| 21-225_20E12_HC | . | . | . | . | . | . | . | . | . | . | . | . | . | . | . | S | . |
| 21-225_20D10_HC | . | . | . | . | . | . | . | . | . | . | . | . | . | . | . | . | . |
| 21-225_22A4_HC | . | . | . | . | . | . | . | . | . | . | . | . | . | . | . | . | . |
| 21-225_20H12_HC | . | . | . | . | . | . | . | . | . | . | . | . | . | . | . | N | . |
| 21-225_21H8_HC | . | . | . | . | . | . | . | . | . | . | . | . | . | . | . | . | . |
| 21-225_21E5_HC | . | . | . | . | . | . | S | . | . | . | . | . | . | . | . | . | . |
| 21-225_22H8_HC | . | . | . | . | . | . | . | . | . | . | . | . | . | . | . | S | . |
| 21-225_23H11_HC | . | . | . | . | . | . | . | . | . | . | . | . | . | . | . | S | . |
| 21-225_21F7_HC | . | . | . | . | . | . | . | . | . | . | . | . | . | . | . | . | . |
| 21-225_22F9_HC | . | . | . | . | . | . | . | . | . | . | . | . | . | . | . | S | . |
| 21-225_21G2_HC | . | . | . | . | . | . | . | . | . | . | . | . | . | . | . | . | . |
| 21-225_22F2_HC | . | . | . | . | . | . | . | . | . | . | . | . | . | . | . | . | . |
| 21-225_23F12_HC | . | . | . | . | . | . | V | . | . | . | . | . | . | . | . | S | . |
| 21-225_28F5_HC | . | . | . | . | . | . | . | . | . | . | . | . | . | . | . | S | . |
| 21-225_29G4_HC | . | . | . | T | . | . | . | . | . | . | . | . | . | . | . | . | . |
| 21-225_27D5_HC | . | . | . | . | . | . | . | . | . | . | . | . | . | . | . | S | . |
| 21-225_28C7_HC | . | . | . | . | . | . | . | . | . | . | . | . | . | . | . | S | . |
| 21-225_25B6_HC | . | . | V | . | . | . | T | . | . | . | . | . | . | L | R | N | . |
| 21-225_26A2_HC | . | . | . | . | . | . | . | . | . | . | . | . | . | . | . | S | . |
| 21-225_29H6_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | . | . | . | . | . | . | . | . | . | . | . | . |
| 21-225_29D7_HC | . | . | . | . | . | . | . | . | . | . | . | . | . | . | . | S | . |
| 21-225_29B8_HC | . | . | . | . | . | . | . | . | . | . | . | . | . | . | . | . | . |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | D | - |
| 21-225_25H11_HC | . | . | . | . | . | . | . | . | . | . | . | . | . | . | . | . | . |
| 21-225_27F2_HC | . | . | . | . | . | . | . | . | . | . | . | . | I | . | . | . | . |
| 21-225_27C3_HC | . | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | S | . |
| 21-225_8H7_HC | . | . | . | . | . | . | P | . | . | . | . | . | . | . | . | . | . |
| 21-225_10G5_HC | . | . | . | . | . | . | . | . | . | . | . | . | . | . | . | N | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | S | . |
| 21-225_6G2_HC | . | . | . | . | . | . | . | . | . | . | . | . | . | . | . | . | . |
| 21-225_7H11_HC | . | . | . | . | . | . | S | . | . | . | . | . | . | . | . | . | . |
| 21-225_14E3_HC | . | . | . | . | . | . | . | . | . | . | . | . | . | . | . | . | . |
| 21-225_9A1_HC | . | . | . | . | . | . | . | . | . | . | . | . | . | . | . | . | . |
| 21-225_15E3_HC | . | . | . | . | . | . | . | . | . | . | . | . | . | . | . | S | . |
| 21-225_16F4_HC | . | . | . | . | . | . | . | . | . | . | . | . | . | . | . | N | . |
| 21-225_16F6_HC | . | . | . | . | . | . | . | . | . | . | . | . | . | . | . | N | . |
| 21-225_2B2_HC | . | . | . | . | . | . | . | . | . | . | . | . | . | . | . | S | . |
| 21-225_13E6_HC | . | . | . | . | . | . | . | . | . | . | . | . | . | . | . | N | . |
| 21-225_16A5_HC | . | . | . | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | N | . |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | S | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | S | . |
| 21-225_9E8_HC | . | . | . | . | . | . | E | . | . | . | . | . | . | . | . | S | . |
| 21-225_12F2_HC | . | . | . | . | . | . | . | . | . | . | . | . | . | . | . | . | . |
| 21-225_12E6_HC | . | . | . | . | . | . | . | . | . | . | . | . | . | . | . | . | . |
| 21-225_16G7_HC | . | . | . | . | . | . | . | . | . | . | . | . | . | T | . | . | . |
| 21-225_1C12_HC | . | . | . | . | . | . | . | . | . | . | . | . | . | . | . | N | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | | | H_FR2 | | |
| CONSENSUS | - | - | - | - | Y | G | M | H | W | V | R | Q | A | P | G | K | G |
| 21-225_16F10_HC | . | . | . | . | . | . | . | . | . | . | . | . | . | . | . | . | . |
| 21-225_18A5_HC | . | . | . | . | . | . | . | . | . | . | . | . | . | . | . | . | . |
| 21-225_20E5_HC | . | . | . | . | . | . | . | . | . | . | . | . | . | . | . | . | . |
| 21-225_18A1_HC | . | . | . | . | . | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | A | . | . |
| 21-225_18E8_HC | . | . | . | . | . | . | . | . | . | . | . | . | . | . | . | . | . |
| 21-225_16F11_HC | . | . | . | . | . | . | . | . | . | . | . | . | . | . | . | . | . |
| 21-225_18C2_HC | . | . | . | . | . | . | . | . | . | . | . | . | . | . | . | . | . |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | . | . |
| 21-225_18F2_HC | . | . | . | . | . | . | . | . | . | . | . | . | . | . | . | . | . |
| 21-225_17B5_HC | . | . | . | . | . | . | . | . | . | . | . | . | . | . | . | . | . |
| 21-225_17E5_HC | . | . | . | . | . | . | . | . | . | . | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | . | . |
| 21-225_20E12_HC | . | . | . | . | . | . | . | . | . | . | . | . | . | . | . | . | . |
| 21-225_20D10_HC | . | . | . | . | . | . | . | . | . | . | . | . | . | . | . | . | . |
| 21-225_22A4_HC | . | . | . | . | . | . | I | . | . | . | . | . | . | . | . | . | . |
| 21-225_20H12_HC | . | . | . | . | . | . | . | . | . | . | . | . | . | . | . | . | . |
| 21-225_21H8_HC | . | . | . | . | . | . | . | . | . | . | . | . | . | . | . | . | . |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | . | . |
| 21-225_23H11_HC | . | . | . | . | . | . | . | . | . | . | . | . | . | . | . | . | . |
| 21-225_21F7_HC | . | . | . | . | . | . | . | . | . | . | . | . | . | . | . | . | . |
| 21-225_22F9_HC | . | . | . | . | . | . | . | . | . | . | . | . | . | . | . | . | . |
| 21-225_21G2_HC | . | . | . | . | . | . | . | . | . | . | . | . | . | . | . | . | . |
| 21-225_22F2_HC | . | . | . | . | . | . | . | . | . | . | . | . | . | . | . | . | . |
| 21-225_23F12_HC | . | . | . | . | . | . | . | . | . | . | . | . | . | . | . | . | . |
| 21-225_28F5_HC | . | . | . | . | . | . | . | . | . | . | . | . | . | . | . | . | . |
| 21-225_29G4_HC | . | . | . | . | . | . | I | . | . | . | . | . | . | . | . | . | . |
| 21-225_27D5_HC | . | . | . | . | . | . | . | . | . | . | . | . | . | . | . | . | . |
| 21-225_28C7_HC | . | . | . | . | . | . | . | . | . | . | . | . | . | . | . | . | . |
| 21-225_25B6_HC | . | . | . | . | . | . | . | . | . | . | . | . | . | . | . | . | . |
| 21-225_26A2_HC | . | . | . | . | . | . | . | . | . | . | . | . | . | . | . | . | . |
| 21-225_29H8_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | . | . | . | . | . | . | . | . | . | . | . | . |
| 21-225_29D7_HC | . | . | . | . | . | . | . | . | . | . | . | . | . | . | . | . | . |
| 21-225_29B8_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | | | H_FR2 | | |
| CONSENSUS | - | - | - | - | Y | G | M | H | W | V | R | Q | A | P | G | K | G |
| 21-225_25H11_HC | . | . | . | . | . | . | . | . | . | . | . | . | . | . | . | . | . |
| 21-225_27F2_HC | . | . | . | . | . | . | . | . | . | . | . | . | . | . | . | . | . |
| 21-225_27C3_HC | . | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | . | . |
| 21-225_8H7_HC | . | . | . | . | F | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | I | . | . | . | . | . | . | . | . | . | . |
| 21-225_7H11_HC | . | . | . | . | . | . | . | . | . | . | . | . | . | . | . | . | . |
| 21-225_14E3_HC | . | . | . | . | F | . | . | . | . | . | . | . | . | . | . | . | . |
| 21-225_9A1_HC | . | . | . | . | . | . | . | . | . | . | . | . | . | . | . | . | . |
| 21-225_15E3_HC | . | . | . | . | . | . | . | . | . | . | . | . | . | . | . | . | . |
| 21-225_16F4_HC | . | . | . | . | . | . | . | . | . | . | . | . | . | . | . | . | . |
| 21-225_16F6_HC | . | . | . | . | . | . | . | . | R | . | . | . | . | . | . | . | . |
| 21-225_2B2_HC | . | . | . | . | . | . | . | . | . | . | . | . | . | . | . | . | . |
| 21-225_13E6_HC | . | . | . | . | . | . | . | . | . | . | . | . | . | . | . | . | . |
| 21-225_16A5_HC | . | . | . | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | . | . |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | . | . | . | . | . | . | . | . | . | . | . | . | . | . | . |
| 21-225_12E6_HC | . | . | . | . | . | . | . | . | . | . | . | . | . | . | . | . | . |
| 21-225_16G7_HC | . | . | . | . | . | . | . | . | . | . | . | . | . | . | . | . | . |
| 21-225_1C12_HC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | A | V | I | W | Y | D | - | - | - | E | N | N | K |
| 21-225_16F10_HC | . | . | . | . | . | . | . | . | . | . | . | . | . | G | . | . | . |
| 21-225_18A5_HC | . | . | . | . | . | . | . | . | F | . | . | . | . | G | S | . | . |
| 21-225_20E5_HC | . | . | . | . | . | . | . | . | . | E | . | . | . | . | . | . | Q |
| 21-225_18A1_HC | . | . | . | . | . | . | . | . | . | E | . | . | . | . | . | . | . |
| 21-225_19E1_HC | . | . | . | . | . | . | . | . | . | E | . | . | . | . | . | . | Q |
| 21-225_17A10_HC | . | . | . | . | . | . | . | . | . | E | . | . | . | . | . | . | Q |
| 21-225_18E8_HC | . | . | . | . | . | . | . | . | . | E | . | . | . | . | . | . | Q |
| 21-225_16F11_HC | . | . | . | . | . | . | . | . | . | E | . | . | . | . | . | . | . |
| 21-225_18C2_HC | . | . | M | . | . | . | . | . | . | . | . | . | . | V | T | . | . |
| 21-225_20A7_HC | . | . | . | . | . | . | . | . | . | E | . | . | . | . | . | . | Q |
| 21-225_17C7_HC | . | . | . | . | . | . | . | . | F | . | . | . | . | G | S | . | . |
| 21-225_18F2_HC | . | . | . | . | . | . | . | . | F | . | . | . | . | G | S | . | . |
| 21-225_17B5_HC | . | . | . | . | . | . | . | . | . | . | . | . | . | . | S | . | . |
| 21-225_17E5_HC | . | . | . | . | . | L | . | . | . | . | . | . | . | V | T | . | . |
| 21-225_20D5_HC | . | . | . | . | . | . | . | . | . | E | . | . | . | . | . | . | Q |
| 21-225_21G11_HC | . | . | M | . | . | . | . | . | . | . | . | . | . | V | T | . | E |
| 21-225_20E12_HC | . | . | . | . | . | . | M | . | . | . | . | . | . | . | S | . | . |
| 21-225_20D10_HC | . | . | . | . | . | . | . | . | F | . | . | . | . | G | S | . | . |
| 21-225_22A4_HC | . | . | . | . | . | . | . | . | F | . | . | . | . | G | S | . | . |
| 21-225_20H12_HC | . | . | . | . | . | . | . | . | . | . | . | . | . | . | . | . | . |
| 21-225_21H8_HC | . | . | . | . | . | . | . | . | F | . | . | . | . | G | S | . | . |
| 21-225_21E5_HC | . | . | . | . | . | . | . | . | . | . | . | . | . | . | S | . | . |
| 21-225_22H8_HC | . | . | . | . | . | . | . | . | . | . | . | . | . | G | . | . | . |
| 21-225_23H11_HC | . | . | . | . | . | . | . | . | . | . | . | . | . | . | . | . | . |
| 21-225_21F7_HC | . | . | . | . | . | . | . | . | F | . | . | . | . | G | . | . | . |
| 21-225_22F9_HC | . | . | . | . | . | . | . | . | . | E | . | . | . | . | . | . | . |
| 21-225_21G2_HC | . | . | M | . | . | . | . | . | . | . | . | . | . | V | T | . | . |
| 21-225_22F2_HC | . | . | . | . | . | . | . | . | . | E | . | . | . | G | S | . | . |
| 21-225_23F12_HC | . | . | . | . | . | . | . | . | . | . | . | . | . | . | T | . | . |
| 21-225_28F5_HC | . | . | . | . | . | . | . | . | . | . | . | . | . | . | . | . | . |
| 21-225_29G4_HC | . | . | . | . | . | . | . | . | . | . | . | . | . | . | S | . | . |
| 21-225_27D5_HC | . | . | . | . | . | . | . | . | . | . | . | . | . | . | S | . | . |
| 21-225_28C7_HC | . | . | . | . | . | . | . | . | . | . | . | . | . | . | S | . | . |
| 21-225_25B6_HC | . | . | . | . | . | . | . | . | . | E | . | . | . | . | S | . | . |
| 21-225_26A2_HC | . | . | . | . | . | . | . | . | . | E | . | . | . | G | S | . | . |
| 21-225_29H6_HC | . | . | . | T | . | . | . | . | . | N | . | . | . | . | . | . | . |
| 21-225_25E6_HC | . | . | . | . | . | . | . | . | . | . | . | . | . | . | . | . | . |
| 21-225_25H10_HC | . | . | . | . | . | . | . | . | . | . | . | . | . | . | S | . | . |
| 21-225_29D7_HC | . | . | . | . | . | . | . | . | . | . | . | . | . | . | S | . | . |
| 21-225_29B8_HC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | A | V | I | W | Y | D | - | - | - | E | N | N | K |
| 21-225_25H11_HC | . | . | . | . | . | . | . | . | . | . | . | . | . | . | . | . | E |
| 21-225_27F2_HC | . | . | . | . | . | . | . | . | . | . | . | . | . | . | . | . | . |
| 21-225_27C3_HC | . | . | . | . | . | . | . | . | . | . | . | . | . | . | . | T | . |
| 21-225_29G8_HC | . | . | . | . | . | . | . | . | . | . | . | . | . | . | . | T | . |
| 21-225_6A11_HC | . | . | . | . | . | . | . | . | F | . | . | . | . | G | S | . | . |
| 21-225_6G12_HC | . | . | . | . | . | . | . | . | . | . | . | . | . | R | S | . | N |
| 21-225_8H7_HC | . | . | . | . | . | . | . | . | . | E | . | . | . | . | . | . | Q |
| 21-225_10G5_HC | . | . | . | . | . | . | . | . | . | E | . | . | . | . | . | . | Q |
| 21-225_10H12_HC | . | . | . | . | . | . | . | . | . | E | . | . | . | D | . | . | Q |
| 21-225_12A1_HC | . | . | . | . | . | . | . | . | . | E | . | . | . | . | . | . | Q |
| 21-225_6G2_HC | . | . | . | . | . | . | . | . | F | . | . | . | . | G | S | . | . |
| 21-225_7H11_HC | . | . | . | . | . | . | . | . | . | . | . | . | . | . | . | . | . |
| 21-225_14E3_HC | . | . | . | . | . | . | . | . | . | E | . | . | . | . | . | . | Q |
| 21-225_9A1_HC | . | . | . | . | . | . | . | . | F | . | . | . | . | G | S | . | . |
| 21-225_15E3_HC | . | . | . | . | . | . | . | . | . | E | . | . | . | . | . | . | . |
| 21-225_16F4_HC | . | . | . | . | . | . | . | . | . | E | . | . | . | G | S | . | Q |
| 21-225_16F6_HC | . | . | . | . | . | . | . | . | . | E | . | . | . | G | S | . | Q |
| 21-225_2B2_HC | . | . | . | . | . | . | . | . | . | E | . | . | . | . | . | . | . |
| 21-225_13E6_HC | . | . | . | . | . | . | . | . | . | . | . | . | . | . | . | . | . |
| 21-225_16A5_HC | . | . | . | . | . | . | . | . | F | . | . | . | . | G | S | . | . |
| 21-225_7G2_HC | . | . | . | M | . | . | . | . | . | . | . | . | . | V | T | . | . |
| 21-225_7C4_HC | . | . | . | . | . | . | . | . | . | E | . | . | . | . | . | . | Q |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | M | . | . | . | . | . | . | . | . | . | V | T | . | . |
| 21-225_16B7_HC | . | . | . | . | . | . | . | . | . | . | . | . | . | . | S | . | . |
| 21-225_9E8_HC | . | . | . | . | . | . | . | . | . | E | . | . | . | . | . | . | Q |
| 21-225_12F2_HC | . | . | . | . | . | . | . | . | F | . | . | . | . | G | . | . | . |
| 21-225_12E6_HC | . | . | . | . | T | . | . | . | . | . | . | . | . | . | S | . | . |
| 21-225_16G7_HC | . | . | . | . | . | . | . | . | . | . | . | . | . | . | . | . | E |
| 21-225_1C12_HC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_16F10_HC | . | . | V | . | . | . | . | . | . | . | . | . | . | . | . | . | . |
| 21-225_18A5_HC | . | . | . | . | . | . | . | . | . | . | . | . | . | . | . | . | . |
| 21-225_20E5_HC | . | . | . | . | . | . | . | . | . | . | . | . | . | . | . | . | . |
| 21-225_18A1_HC | . | . | V | . | . | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | V | . | . | . | . | . |
| 21-225_16F11_HC | . | . | V | . | . | . | . | . | . | . | . | . | . | . | . | . | . |
| 21-225_18C2_HC | . | . | . | . | . | . | . | . | . | . | . | . | . | . | . | . | . |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | . | . |
| 21-225_18F2_HC | . | . | V | . | . | . | . | . | . | . | . | . | . | . | . | . | . |
| 21-225_17B5_HC | H | . | I | . | . | . | . | . | . | . | . | . | . | . | . | . | . |
| 21-225_17E5_HC | . | . | G | . | . | . | . | . | . | . | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | . | . |
| 21-225_20E12_HC | H | . | . | . | . | . | . | . | . | . | . | . | . | . | . | . | . |
| 21-225_20D10_HC | . | . | V | . | . | . | . | . | . | . | . | . | . | . | . | D | . |
| 21-225_22A4_HC | . | . | . | . | . | . | . | . | . | I | . | . | . | . | . | . | . |
| 21-225_20H12_HC | . | . | V | . | . | . | . | . | . | . | . | . | . | . | . | . | . |
| 21-225_21H8_HC | . | . | . | . | . | . | . | . | . | . | . | . | . | . | . | . | . |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | . | . |
| 21-225_23H11_HC | . | . | V | . | . | . | . | . | . | . | . | . | . | . | . | . | . |
| 21-225_21F7_HC | . | . | V | . | . | . | . | . | . | . | . | . | . | . | . | . | . |
| 21-225_22F9_HC | D | . | V | . | . | . | . | . | . | . | . | . | . | . | . | . | . |
| 21-225_21G2_HC | . | . | . | . | . | . | . | . | . | . | . | . | . | . | . | . | . |
| 21-225_22F2_HC | . | . | V | . | . | . | R | . | . | . | . | . | . | . | . | . | . |
| 21-225_23F12_HC | . | . | . | . | . | . | . | . | . | . | . | . | . | . | . | . | . |
| 21-225_28F5_HC | . | . | . | . | . | . | . | . | . | . | . | . | . | . | . | . | . |
| 21-225_29G4_HC | . | . | . | . | . | M | . | . | . | . | . | . | . | . | . | . | . |
| 21-225_27D5_HC | . | . | G | . | . | . | . | . | . | . | . | . | . | . | . | . | . |
| 21-225_28C7_HC | . | . | . | . | . | . | . | . | . | . | . | . | . | . | . | . | . |
| 21-225_25B6_HC | . | . | T | E | . | . | . | . | . | . | . | . | . | . | . | . | . |
| 21-225_26A2_HC | . | . | V | . | . | . | . | . | . | . | . | . | . | . | . | . | . |
| 21-225_29H6_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | M | . | . | . | . | . | . | . | . | . | . | . |
| 21-225_29D7_HC | . | . | G | . | . | . | . | . | . | . | . | . | . | . | . | . | . |
| 21-225_29B8_HC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_16F10_HC | . | . | V | . | . | . | . | . | . | . | . | . | . | . | . | . | . |
| 21-225_18A5_HC | . | . | . | . | . | . | . | . | . | . | . | . | . | . | . | . | . |
| 21-225_20E5_HC | . | . | . | . | . | . | . | . | . | . | . | . | . | . | . | . | . |
| 21-225_18A1_HC | . | . | V | . | . | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | V | . | . | . | . | . |
| 21-225_16F11_HC | . | . | V | . | . | . | . | . | . | . | . | . | . | . | . | . | . |
| 21-225_18C2_HC | . | . | . | . | . | . | . | . | . | . | . | . | . | . | . | . | . |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | . | . |
| 21-225_18F2_HC | . | . | V | . | . | . | . | . | . | . | . | . | . | . | . | . | . |
| 21-225_17B5_HC | H | . | I | . | . | . | . | . | . | . | . | . | . | . | . | . | . |
| 21-225_17E5_HC | . | . | G | . | . | . | . | . | . | . | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | . | . |
| 21-225_20E12_HC | H | . | . | . | . | . | . | . | . | . | . | . | . | . | . | . | . |
| 21-225_20D10_HC | . | . | V | . | . | . | . | . | . | . | . | . | . | . | . | D | . |
| 21-225_22A4_HC | . | . | . | . | . | . | . | . | . | I | . | . | . | . | . | . | . |
| 21-225_20H12_HC | . | . | V | . | . | . | . | . | . | . | . | . | . | . | . | . | . |
| 21-225_21H8_HC | . | . | . | . | . | . | . | . | . | . | . | . | . | . | . | . | . |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | . | . |
| 21-225_23H11_HC | . | . | V | . | . | . | . | . | . | . | . | . | . | . | . | . | . |
| 21-225_21F7_HC | . | . | V | . | . | . | . | . | . | . | . | . | . | . | . | . | . |
| 21-225_22F9_HC | D | . | V | . | . | . | . | . | . | . | . | . | . | . | . | . | . |
| 21-225_21G2_HC | . | . | . | . | . | . | . | . | . | . | . | . | . | . | . | . | . |
| 21-225_22F2_HC | . | . | V | . | . | . | R | . | . | . | . | . | . | . | . | . | . |
| 21-225_23F12_HC | . | . | . | . | . | . | . | . | . | . | . | . | . | . | . | . | . |
| 21-225_28F5_HC | . | . | . | . | . | . | . | . | . | . | . | . | . | . | . | . | . |
| 21-225_29G4_HC | . | . | . | . | . | M | . | . | . | . | . | . | . | . | . | . | . |
| 21-225_27D5_HC | . | . | G | . | . | . | . | . | . | . | . | . | . | . | . | . | . |
| 21-225_28C7_HC | . | . | . | . | . | . | . | . | . | . | . | . | . | . | . | . | . |
| 21-225_25B6_HC | . | . | T | E | . | . | . | . | . | . | . | . | . | . | . | . | . |
| 21-225_26A2_HC | . | . | V | . | . | . | . | . | . | . | . | . | . | . | . | . | . |
| 21-225_29H6_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | M | . | . | . | . | . | . | . | . | . | . | . |
| 21-225_29D7_HC | . | . | G | . | . | . | . | . | . | . | . | . | . | . | . | . | . |
| 21-225_29B8_HC | . | . | . | . | . | . | . | . | . | . | . | . | . | . | . | . | . |

**FIGURE 56**

| Reference # | 68 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_25H11_HC | . | . | . | . | . | . | . | . | . | . | . | . | . | . | . | . | . |
| 21-225_27F2_HC | . | . | V | . | . | . | R | . | . | . | . | . | . | . | . | . | . |
| 21-225_27C3_HC | S | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | . | . | . | . | . | . | . | S | . | . | . |
| 21-225_7H11_HC | . | . | . | . | . | . | . | . | . | . | . | . | . | . | . | . | . |
| 21-225_14E3_HC | . | . | . | . | . | . | . | . | . | . | . | . | . | . | . | . | . |
| 21-225_9A1_HC | . | . | . | . | . | . | . | . | . | . | . | . | . | . | . | . | . |
| 21-225_15E3_HC | H | . | . | . | . | . | . | . | . | . | . | . | . | . | . | . | . |
| 21-225_16F4_HC | . | . | G | . | . | . | . | . | . | . | . | . | . | . | . | . | . |
| 21-225_16F6_HC | . | . | G | . | . | . | . | . | . | . | . | . | . | . | . | . | . |
| 21-225_2B2_HC | . | . | V | . | . | . | . | . | . | . | . | . | . | . | . | . | . |
| 21-225_13E6_HC | . | . | I | . | . | . | . | . | . | . | . | . | . | . | . | . | . |
| 21-225_16A5_HC | . | . | V | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | . | . |
| 21-225_7E9_HC | . | . | V | . | . | . | . | . | . | . | . | . | . | . | . | . | . |
| 21-225_8C9_HC | . | . | V | . | . | . | . | . | . | . | . | . | . | . | . | . | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | V | . | . | . | . | . | . | . | . | . | . | . | . | . | . |
| 21-225_12E6_HC | . | . | . | . | . | . | . | . | . | . | . | . | . | . | . | D | . |
| 21-225_16G7_HC | . | . | . | . | . | . | . | . | . | . | . | . | . | . | A | . | . |
| 21-225_1C12_HC | . | . | V | . | . | . | . | . | . | . | . | . | . | . | . | . | . |

FIGURE 56

| Reference # | 68 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_25H11_HC | . | . | . | . | . | . | . | . | . | . | . | . | . | . | . | . | . |
| 21-225_27F2_HC | . | . | V | . | . | . | R | . | . | . | . | . | . | . | . | . | . |
| 21-225_27C3_HC | S | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | . | . | . | . | . | . | . | S | . | . | . |
| 21-225_7H11_HC | . | . | . | . | . | . | . | . | . | . | . | . | . | . | . | . | . |
| 21-225_14E3_HC | . | . | . | . | . | . | . | . | . | . | . | . | . | . | . | . | . |
| 21-225_9A1_HC | . | . | . | . | . | . | . | . | . | . | . | . | . | . | . | . | . |
| 21-225_15E3_HC | H | . | . | . | . | . | . | . | . | . | . | . | . | . | . | . | . |
| 21-225_16F4_HC | . | . | G | . | . | . | . | . | . | . | . | . | . | . | . | . | . |
| 21-225_16F6_HC | . | . | G | . | . | . | . | . | . | . | . | . | . | . | . | . | . |
| 21-225_2B2_HC | . | . | V | . | . | . | . | . | . | . | . | . | . | . | . | . | . |
| 21-225_13E6_HC | . | . | I | . | . | . | . | . | . | . | . | . | . | . | . | . | . |
| 21-225_16A5_HC | . | . | V | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | . | . |
| 21-225_7E9_HC | . | . | V | . | . | . | . | . | . | . | . | . | . | . | . | . | . |
| 21-225_8C9_HC | . | . | V | . | . | . | . | . | . | . | . | . | . | . | . | . | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | V | . | . | . | . | . | . | . | . | . | . | . | . | . | . |
| 21-225_12E6_HC | . | . | . | . | . | . | . | . | . | . | . | . | . | . | . | D | . |
| 21-225_16G7_HC | . | . | . | . | . | . | . | . | . | . | . | . | . | . | A | . | . |
| 21-225_1C12_HC | . | . | V | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | E | L | G | F | - | - | - | - | - | - | - |
| 21-225_16F10_HC | . | . | . | . | . | . | . | . | A | W | . | . | . | . | . | . | . |
| 21-225_18A5_HC | . | . | . | . | . | . | D | . | . | W | . | . | . | . | . | . | . |
| 21-225_20E5_HC | . | . | . | . | . | . | . | . | . | . | . | . | . | . | . | . | . |
| 21-225_18A1_HC | . | . | . | . | V | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | . | . | . | . | . | . |
| 21-225_16F11_HC | . | . | . | . | V | . | . | . | . | . | . | . | . | . | . | . | . |
| 21-225_18C2_HC | . | . | . | . | . | . | . | . | . | W | . | . | . | . | . | . | . |
| 21-225_20A7_HC | I | . | . | . | . | . | . | . | . | . | . | . | . | . | . | . | . |
| 21-225_17C7_HC | . | . | . | . | . | . | D | . | . | W | . | . | . | . | . | . | . |
| 21-225_18F2_HC | . | . | . | . | . | . | D | . | . | W | . | . | . | . | . | . | . |
| 21-225_17B5_HC | . | . | . | . | V | . | . | . | . | . | . | . | . | . | . | . | . |
| 21-225_17E5_HC | . | . | . | . | . | . | . | . | . | W | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | W | . | . | . | . | . | . | . |
| 21-225_20E12_HC | . | . | . | . | . | . | . | . | . | . | . | . | . | . | . | . | . |
| 21-225_20D10_HC | . | . | . | . | . | . | D | . | . | W | . | . | . | . | . | . | . |
| 21-225_22A4_HC | . | . | . | . | . | . | D | . | . | W | . | . | . | . | . | . | . |
| 21-225_20H12_HC | . | . | . | . | . | . | . | . | . | . | . | . | . | . | . | . | . |
| 21-225_21H8_HC | . | . | . | . | . | . | D | . | . | W | . | . | . | . | . | . | . |
| 21-225_21E5_HC | M | . | . | . | T | . | . | . | . | . | . | . | . | . | . | . | . |
| 21-225_22H8_HC | . | . | . | . | . | . | . | . | A | W | . | . | . | . | . | . | . |
| 21-225_23H11_HC | . | . | . | . | . | . | . | . | . | . | . | . | . | . | . | . | . |
| 21-225_21F7_HC | . | . | . | . | . | . | D | . | . | W | . | . | . | . | . | . | . |
| 21-225_22F9_HC | . | . | . | . | V | . | . | . | . | . | . | . | . | . | . | . | . |
| 21-225_21G2_HC | . | . | . | . | . | . | . | . | . | W | . | . | . | . | . | . | . |
| 21-225_22F2_HC | . | . | . | . | V | . | . | . | . | . | . | . | . | . | . | . | . |
| 21-225_23F12_HC | I | . | . | . | . | . | . | . | A | W | . | . | . | . | . | . | . |
| 21-225_28F5_HC | . | . | . | . | . | . | . | . | . | W | . | . | . | . | . | . | . |
| 21-225_29G4_HC | . | . | . | . | . | . | . | . | . | M | . | . | . | . | . | . | . |
| 21-225_27D5_HC | L | . | . | . | . | . | . | . | . | . | . | . | . | . | . | . | . |
| 21-225_28C7_HC | . | . | . | . | . | . | . | . | . | W | . | . | . | . | . | . | . |
| 21-225_25B6_HC | . | . | . | . | . | . | . | . | . | . | . | . | . | . | . | . | . |
| 21-225_26A2_HC | . | . | . | . | . | . | . | . | . | W | . | . | . | . | . | . | . |
| 21-225_29H6_HC | . | . | . | . | . | . | . | . | . | M | . | . | . | . | . | . | . |
| 21-225_25E6_HC | L | . | . | . | T | . | . | . | . | M | . | . | . | . | . | . | . |
| 21-225_25H10_HC | . | . | . | . | . | . | . | . | . | M | . | . | . | . | . | . | . |
| 21-225_29D7_HC | L | . | . | . | . | . | . | . | . | . | . | . | . | . | . | . | . |
| 21-225_29B8_HC | . | . | . | . | . | . | . | . | . | M | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | E | L | G | F | - | - | - | - | - | - | - |
| 21-225_25H11_HC | . | . | . | . | . | . | . | . | . | M | . | . | . | . | . | . | . |
| 21-225_27F2_HC | . | . | . | . | . | . | . | . | . | M | . | . | . | . | . | . | . |
| 21-225_27C3_HC | . | . | . | . | . | . | . | . | . | M | . | . | . | . | . | . | . |
| 21-225_29G8_HC | . | . | . | . | . | . | . | . | . | M | . | . | . | . | . | . | . |
| 21-225_6A11_HC | . | . | D | . | . | . | D | . | . | W | . | . | . | . | . | . | . |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | T | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | D | . | . | W | . | . | . | . | . | . | . |
| 21-225_7H11_HC | . | . | . | . | . | . | . | . | . | . | . | . | . | . | . | . | . |
| 21-225_14E3_HC | F | . | . | . | . | . | . | . | . | . | . | . | . | . | . | . | . |
| 21-225_9A1_HC | . | . | . | . | . | . | D | . | . | W | . | . | . | . | . | . | . |
| 21-225_15E3_HC | . | . | . | . | . | . | . | . | . | . | . | . | . | . | . | . | . |
| 21-225_16F4_HC | . | . | . | . | . | . | . | . | . | . | . | . | . | . | . | . | . |
| 21-225_16F6_HC | . | . | . | . | . | . | . | . | . | . | . | . | . | . | . | . | . |
| 21-225_2B2_HC | . | . | . | . | V | . | . | . | . | . | . | . | . | . | . | . | . |
| 21-225_13E6_HC | . | . | . | . | . | . | . | . | . | . | . | . | . | . | . | . | . |
| 21-225_16A5_HC | . | . | . | . | . | . | D | . | . | W | . | . | . | . | . | . | . |
| 21-225_7G2_HC | . | . | . | . | . | . | . | . | . | W | . | . | . | . | . | . | . |
| 21-225_7C4_HC | . | . | . | . | . | . | . | . | . | . | . | . | . | . | . | . | . |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | . | . | . | . | . | . | W | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | . | . | V | . | . | . | A | W | . | . | . | . | . | . | . |
| 21-225_12E6_HC | . | . | . | . | . | . | . | . | A | W | . | . | . | . | . | . | . |
| 21-225_16G7_HC | . | . | . | . | . | . | . | . | A | W | . | . | . | . | . | . | . |
| 21-225_1C12_HC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | - | - | R | S |
| 21-225_16F10_HC | . | . | . | . | . | . | . | . | . | . | . | . | . | . | . | Y | E |
| 21-225_18A5_HC | . | . | . | . | . | . | . | . | . | . | . | . | . | . | . | T | E |
| 21-225_20E5_HC | . | . | . | . | . | . | . | . | . | . | . | . | . | . | . | . | . |
| 21-225_18A1_HC | . | . | . | . | . | . | . | . | . | . | . | . | . | . | . | Q | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | . | . | . | . | . | . |
| 21-225_16F11_HC | . | . | . | . | . | . | . | . | . | . | . | . | . | . | . | Q | . |
| 21-225_18C2_HC | . | . | . | . | . | . | . | . | . | . | . | . | . | . | . | Y | E |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | T | E |
| 21-225_18F2_HC | . | . | . | . | . | . | . | . | . | . | . | . | . | . | . | T | E |
| 21-225_17B6_HC | . | . | . | . | . | . | . | . | . | . | . | . | . | . | . | . | F |
| 21-225_17E5_HC | . | . | . | . | . | . | . | . | . | . | . | . | . | . | . | Y | E |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | Y | E |
| 21-225_20E12_HC | . | . | . | . | . | . | . | . | . | . | . | . | . | . | . | . | F |
| 21-225_20D10_HC | . | . | . | . | . | . | . | . | . | . | . | . | . | . | . | T | E |
| 21-225_22A4_HC | . | . | . | . | . | . | . | . | . | . | . | . | . | . | . | T | E |
| 21-225_20H12_HC | . | . | . | . | . | . | . | . | . | . | . | . | . | . | . | . | . |
| 21-225_21H8_HC | . | . | . | . | . | . | . | . | . | . | . | . | . | . | . | T | E |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | Y | E |
| 21-225_23H11_HC | . | . | . | . | . | . | . | . | . | . | . | . | . | . | . | . | . |
| 21-225_21F7_HC | . | . | . | . | . | . | . | . | . | . | . | . | . | . | . | T | E |
| 21-225_22F9_HC | . | . | . | . | . | . | . | . | . | . | . | . | . | . | . | Q | . |
| 21-225_21G2_HC | . | . | . | . | . | . | . | . | . | . | . | . | . | . | . | Y | E |
| 21-225_22F2_HC | . | . | . | . | . | . | . | . | . | . | . | . | . | . | . | Q | . |
| 21-225_23F12_HC | . | . | . | . | . | . | . | . | . | . | . | . | . | . | . | Y | E |
| 21-225_28F5_HC | . | . | . | . | . | . | . | . | . | . | . | . | . | . | . | Y | D |
| 21-225_29G4_HC | . | . | . | . | . | . | . | . | . | . | . | . | . | . | . | T | G |
| 21-225_27D5_HC | . | . | . | . | . | . | . | . | . | . | . | . | . | . | . | L | . |
| 21-225_28C7_HC | . | . | . | . | . | . | . | . | . | . | . | . | . | . | . | Y | D |
| 21-225_25B6_HC | . | . | . | . | . | . | . | . | . | . | . | . | . | . | . | L | . |
| 21-225_26A2_HC | . | . | . | . | . | . | . | . | . | . | . | . | . | . | . | Y | D |
| 21-225_29H6_HC | . | . | . | . | . | . | . | . | . | . | . | . | . | . | . | T | G |
| 21-225_25E6_HC | . | . | . | . | . | . | . | . | . | . | . | . | . | . | . | T | G |
| 21-225_25H10_HC | . | . | . | . | . | . | . | . | . | . | . | . | . | . | . | T | G |
| 21-225_29D7_HC | . | . | . | . | . | . | . | . | . | . | . | . | . | . | . | L | . |
| 21-225_29B8_HC | . | . | . | . | . | . | . | . | . | . | . | . | . | . | . | T | G |

**FIGURE 56**

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | - | - | R | S |
| 21-225_25H11_HC | . | . | . | . | . | . | . | . | . | . | . | . | . | . | . | T | G |
| 21-225_27F2_HC | . | . | . | . | . | . | . | . | . | . | . | . | . | . | . | T | G |
| 21-225_27C3_HC | . | . | . | . | . | . | . | . | . | . | . | . | . | . | . | T | G |
| 21-225_29G8_HC | . | . | . | . | . | . | . | . | . | . | . | . | . | . | . | T | . |
| 21-225_6A11_HC | . | . | . | . | . | . | . | . | . | . | . | . | . | . | . | T | E |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | . | . | . | . | . | . | . | . | . | T | E |
| 21-225_7H11_HC | . | . | . | . | . | . | . | . | . | . | . | . | . | . | . | . | . |
| 21-225_14E3_HC | . | . | . | . | . | . | . | . | . | . | . | . | . | . | . | . | . |
| 21-225_9A1_HC | . | . | . | . | . | . | . | . | . | . | . | . | . | . | . | T | E |
| 21-225_15E3_HC | . | . | . | . | . | . | . | . | . | . | . | . | . | . | . | L | . |
| 21-225_16F4_HC | . | . | . | . | . | . | . | . | . | . | . | . | . | . | . | L | . |
| 21-225_16F6_HC | . | . | . | . | . | . | . | . | . | . | . | . | . | . | . | L | . |
| 21-225_2B2_HC | . | . | . | . | . | . | . | . | . | . | . | . | . | . | . | Q | . |
| 21-225_13E6_HC | . | . | . | . | . | . | . | . | . | . | . | . | . | . | . | . | . |
| 21-225_16A5_HC | . | . | . | . | . | . | . | . | . | . | . | . | . | . | . | T | E |
| 21-225_7G2_HC | . | . | . | . | . | . | . | . | . | . | . | . | . | . | . | Y | E |
| 21-225_7C4_HC | . | . | . | . | . | . | . | . | . | . | . | . | . | . | . | . | . |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | . | . | . | . | . | . | . | . | . | . | . | . | Y | E |
| 21-225_16B7_HC | . | . | . | . | . | . | . | . | . | . | . | . | . | . | . | L | . |
| 21-225_9E8_HC | . | . | . | . | . | . | . | . | . | . | . | . | . | . | . | . | . |
| 21-225_12F2_HC | . | . | . | . | . | . | . | . | . | . | . | . | . | . | . | S | E |
| 21-225_12E6_HC | . | . | . | . | . | . | . | . | . | . | . | . | . | . | . | Y | E |
| 21-225_16G7_HC | . | . | . | . | . | . | . | . | . | . | . | . | . | . | . | Y | E |
| 21-225_1C12_HC | . | . | . | . | . | . | . | . | . | . | . | . | . | . | . | . | . |

3524

EP 4 435 105 A2

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | | H_FR4 | | | | | |
| CONSENSUS | D | Y | W | G | Q | G | T | L | V | T | V | S | S |
| 21-225_16F10_HC | . | . | . | . | . | . | . | . | . | . | . | . | . |
| 21-225_18A5_HC | E | . | . | . | . | . | . | . | . | . | . | . | . |
| 21-225_20E5_HC | . | . | . | . | . | . | . | . | . | . | . | . | . |
| 21-225_18A1_HC | . | . | . | . | . | . | . | P | . | . | . | . | . |
| 21-225_19E1_HC | . | . | . | . | . | . | . | . | . | . | . | . | . |
| 21-225_17A10_HC | . | . | . | . | . | . | . | . | . | . | . | . | . |
| 21-225_18E8_HC | . | . | . | . | . | . | . | . | . | . | . | . | . |
| 21-225_16F11_HC | . | . | . | . | . | . | . | P | . | . | . | . | . |
| 21-225_18C2_HC | . | . | . | . | . | . | . | . | . | . | . | . | . |
| 21-225_20A7_HC | . | . | . | . | . | . | . | . | . | . | . | . | . |
| 21-225_17C7_HC | E | . | . | . | . | . | . | . | . | . | . | . | . |
| 21-225_18F2_HC | E | . | . | . | . | . | . | . | . | . | . | . | . |
| 21-225_17B5_HC | . | . | . | . | . | . | . | . | . | . | . | . | . |
| 21-225_17E5_HC | . | . | . | . | . | . | . | . | . | . | . | . | . |
| 21-225_20D5_HC | . | . | . | . | . | . | . | . | . | . | . | . | . |
| 21-225_21G11_HC | . | . | . | . | . | . | . | . | . | . | . | . | . |
| 21-225_20E12_HC | . | . | . | . | . | . | . | . | . | . | . | . | . |
| 21-225_20D10_HC | E | . | . | . | . | . | . | . | . | . | . | . | . |
| 21-225_22A4_HC | E | . | . | . | . | . | . | . | . | . | . | . | . |
| 21-225_20H12_HC | . | . | . | . | . | . | . | . | . | . | . | . | . |
| 21-225_21H8_HC | E | . | . | . | . | . | . | . | . | . | . | . | . |
| 21-225_21E5_HC | . | . | . | . | . | . | . | . | . | . | . | . | . |
| 21-225_22H8_HC | . | . | . | . | . | . | . | . | . | . | . | . | . |
| 21-225_23H11_HC | . | . | . | . | . | . | . | . | . | . | . | . | . |
| 21-225_21F7_HC | E | . | . | . | . | . | . | . | . | . | . | . | . |
| 21-225_22F9_HC | . | . | . | . | . | . | . | P | . | . | . | . | . |
| 21-225_21G2_HC | . | . | . | . | . | . | . | . | . | . | . | . | . |
| 21-225_22F2_HC | . | . | . | . | . | . | . | P | . | . | . | . | . |
| 21-225_23F12_HC | . | . | . | . | . | . | S | . | . | . | . | . | . |
| 21-225_28F5_HC | . | . | . | . | . | . | . | . | . | . | . | . | . |
| 21-225_29G4_HC | . | . | . | . | . | . | . | . | . | . | . | . | . |
| 21-225_27D5_HC | . | . | . | . | . | . | . | . | . | . | . | . | . |
| 21-225_28C7_HC | . | . | . | . | . | . | . | . | . | . | . | . | . |
| 21-225_25B6_HC | . | . | . | . | . | . | . | . | . | . | . | . | . |
| 21-225_26A2_HC | . | . | . | . | . | . | . | . | . | . | . | . | . |
| 21-225_29H6_HC | . | S | . | . | . | . | . | . | . | . | . | . | . |
| 21-225_25E6_HC | . | S | . | . | . | . | . | . | . | . | . | . | . |
| 21-225_25H10_HC | . | . | . | . | . | . | . | . | . | . | . | . | . |
| 21-225_29D7_HC | . | . | . | . | . | . | . | . | . | . | . | . | . |
| 21-225_29B8_HC | . | S | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | H_FR4 | | | | | | |
| CONSENSUS | D | Y | W | G | Q | G | T | L | V | T | V | S | S |
| 21-225_25H11_HC | . | S | . | . | . | . | . | . | . | . | . | . | . |
| 21-225_27F2_HC | . | S | . | . | . | . | . | . | . | . | . | . | . |
| 21-225_27C3_HC | . | S | . | . | . | . | . | . | . | . | . | . | . |
| 21-225_29G8_HC | . | . | . | . | . | . | . | . | . | . | . | . | . |
| 21-225_6A11_HC | E | . | . | . | . | . | . | . | . | . | . | . | . |
| 21-225_6G12_HC | . | . | . | . | . | . | . | . | . | . | . | . | . |
| 21-225_8H7_HC | . | . | . | . | . | . | . | . | . | . | . | . | . |
| 21-225_10G5_HC | . | . | . | . | . | . | . | . | . | . | . | . | . |
| 21-225_10H12_HC | . | . | . | . | . | . | . | . | . | . | . | . | . |
| 21-225_12A1_HC | . | . | . | . | . | . | . | . | . | . | . | . | . |
| 21-225_6G2_HC | E | . | . | . | . | . | . | . | . | . | . | . | . |
| 21-225_7H11_HC | . | . | . | . | . | . | . | . | . | . | . | . | . |
| 21-225_14E3_HC | . | . | . | . | . | . | . | . | . | . | . | . | . |
| 21-225_9A1_HC | E | . | . | . | . | . | . | P | . | . | . | . | . |
| 21-225_15E3_HC | . | . | . | . | . | . | . | . | . | . | . | . | . |
| 21-225_16F4_HC | . | . | . | . | . | . | . | . | . | . | . | . | . |
| 21-225_16F6_HC | . | . | . | . | . | . | . | . | . | . | . | . | . |
| 21-225_2B2_HC | . | . | . | . | . | . | . | P | . | . | . | . | . |
| 21-225_13E6_HC | . | . | . | . | . | . | . | . | . | . | . | . | . |
| 21-225_16A5_HC | E | . | . | . | . | . | . | . | . | . | . | . | . |
| 21-225_7G2_HC | . | . | . | . | . | . | . | . | . | . | . | . | . |
| 21-225_7C4_HC | . | . | . | . | . | . | . | . | . | . | . | . | . |
| 21-225_7E9_HC | . | . | . | . | . | . | . | . | . | . | . | . | . |
| 21-225_8C9_HC | . | . | . | . | . | . | . | . | . | . | . | . | . |
| 21-225_12B12_HC | . | . | . | . | . | . | . | . | . | . | . | . | . |
| 21-225_16B7_HC | . | . | . | . | . | . | . | . | . | . | . | . | . |
| 21-225_9E8_HC | . | . | . | . | . | . | . | . | . | . | . | . | . |
| 21-225_12F2_HC | . | . | . | . | . | . | . | . | . | . | . | . | . |
| 21-225_12E6_HC | . | . | . | . | . | . | . | . | . | . | . | . | . |
| 21-225_16G7_HC | . | . | . | . | . | . | . | . | . | . | . | . | . |
| 21-225_1C12_HC | . | . | . | . | . | . | . | . | . | . | . | . | . |

**FIGURE 56**

Table 33

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | H_FR1 | | |
| CONSENSUS | Q | L | Q | L | Q | E | S | - | G | P | G | L | V | K | P | S | E |
| 21-225_20C2_HC | . | . | . | . | . | . | . | . | . | . | . | . | . | . | . | . | . |
| 21-225_18C8_HC | . | . | . | . | . | . | . | . | . | . | . | . | . | . | . | . | . |
| 21-225_18A3_HC | . | . | . | . | . | . | . | . | . | . | . | . | . | . | **F** | . | . |
| 21-225_22D12_HC | . | . | . | . | . | . | . | . | . | . | . | . | . | . | . | . | . |
| 21-225_23D1_HC | . | . | . | . | . | . | . | . | . | . | . | . | . | . | . | . | . |
| 21-225_24E5_HC | . | . | . | . | . | . | . | . | . | . | . | . | . | . | . | . | . |
| 21-225_22C1_HC | . | . | . | . | . | . | . | . | . | . | . | . | . | . | . | . | . |
| 21-225_20G9_HC | . | . | . | . | . | . | . | . | . | . | . | . | . | . | . | . | . |
| 21-225_21G7_HC | . | . | . | . | . | . | . | . | . | . | . | . | . | . | **F** | . | . |
| 21-225_22G9_HC | . | . | . | . | . | . | . | . | . | . | . | . | . | . | . | . | . |
| 21-225_34C4_HC | . | . | . | . | . | . | . | . | . | . | . | . | . | . | . | . | . |
| 21-225_2F7_HC | . | . | . | . | . | . | . | . | . | . | . | . | . | . | . | . | . |
| 21-225_3G4_HC | . | . | . | . | . | . | . | . | . | . | . | . | . | . | . | . | . |
| 21-225_7F4_HC | . | . | . | . | . | . | . | . | . | . | . | . | . | . | . | . | . |
| 21-225_6D4_HC | . | . | . | . | . | . | . | . | . | . | . | . | . | . | . | . | . |
| 21-225_15A1_HC | . | . | **H** | . | . | . | . | . | . | . | . | . | . | . | . | . | . |
| 21-225_15G7_HC | . | . | . | . | . | . | . | . | . | . | . | . | . | . | . | . | . |
| 21-225_11F10_HC | . | . | **H** | . | . | . | . | . | . | . | . | . | . | . | . | . | . |
| 21-225_15H1_HC | . | . | . | . | . | . | . | . | . | . | . | . | . | . | **F** | . | . |
| 21-225_16A1_HC | . | . | . | . | . | . | . | . | . | . | . | . | . | . | . | . | . |
| 21-225_8D12_HC | . | . | . | . | . | . | . | . | . | . | . | . | . | . | **F** | . | . |

EP 4 435 105 A2

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | T | L | S | L | T | C | T | V | S | G | - | G | S | I | S | R | S |
| 21-225_20C2_HC | . | . | . | . | . | . | . | . | . | . | . | . | . | . | . | . | . |
| 21-225_18C6_HC | . | . | . | . | . | . | . | . | . | . | . | . | . | . | . | . | . |
| 21-225_18A3_HC | . | . | . | . | . | . | . | . | . | . | . | . | . | . | . | . | . |
| 21-225_22D12_HC | . | . | . | . | . | . | . | . | . | . | . | . | . | . | . | . | . |
| 21-225_23D1_HC | . | . | . | . | . | . | . | . | . | . | . | . | A | . | . | . | . |
| 21-225_24E5_HC | . | . | . | . | . | . | . | . | . | . | . | . | A | . | N | . | . |
| 21-225_22C1_HC | . | . | . | . | . | . | . | . | . | . | . | . | . | . | N | . | . |
| 21-225_20G9_HC | . | . | . | . | . | . | . | . | . | . | . | . | . | . | . | . | . |
| 21-225_21G7_HC | . | . | . | . | . | . | . | . | . | . | . | . | . | . | . | . | . |
| 21-225_22G9_HC | . | . | . | . | . | . | . | . | . | . | . | . | A | . | . | G | . |
| 21-225_34C4_HC | . | . | . | . | . | . | . | . | . | . | . | . | . | . | . | . | . |
| 21-225_2F7_HC | . | . | . | . | . | . | . | . | . | . | . | . | A | . | . | G | . |
| 21-225_3G4_HC | . | . | . | . | . | . | . | . | . | . | . | . | . | . | . | . | . |
| 21-225_7F4_HC | . | . | . | . | . | . | . | . | . | . | . | . | . | . | . | . | . |
| 21-225_6D4_HC | . | . | . | . | . | . | . | . | . | . | . | . | . | . | . | . | . |
| 21-225_15A1_HC | . | . | . | . | . | . | . | . | . | . | . | . | . | . | . | . | . |
| 21-225_15G7_HC | . | . | . | . | . | . | . | . | . | . | . | . | . | . | . | . | . |
| 21-225_11F10_HC | . | . | . | . | . | . | . | . | . | . | . | A | . | . | . | . | . |
| 21-225_15H1_HC | . | . | . | . | . | . | . | . | . | . | . | . | . | . | . | . | . |
| 21-225_16A1_HC | . | . | . | . | I | . | . | . | . | . | . | . | . | . | D | . | . |
| 21-225_8D12_HC | . | . | . | . | . | . | . | . | . | . | . | . | . | . | . | . | . |

3528

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | | | | | | | H_FR2 | | |
| CONSENSUS | - | - | - | S | Y | Y | W | G | W | I | R | Q | P | P | G | K | G |
| 21-225_20C2_HC | . | . | . | . | . | . | . | . | . | . | . | . | . | . | . | . | . |
| 21-225_18C6_HC | . | . | . | . | . | . | . | . | . | . | . | . | . | . | . | . | . |
| 21-225_18A3_HC | . | . | . | . | . | . | . | . | . | . | . | . | . | . | . | . | . |
| 21-225_22D12_HC | . | . | . | . | . | . | . | . | . | . | . | . | . | . | . | . | . |
| 21-225_23D1_HC | . | . | . | . | . | . | . | . | . | . | . | . | . | . | . | . | . |
| 21-225_24E5_HC | . | . | . | . | . | . | . | . | . | . | . | . | . | . | . | . | . |
| 21-225_22C1_HC | . | . | . | . | . | . | . | . | . | . | . | . | . | . | . | . | . |
| 21-225_20G9_HC | . | . | . | . | . | . | . | . | . | . | . | . | . | . | . | . | . |
| 21-225_21G7_HC | . | . | . | . | . | . | . | . | . | . | . | . | . | . | . | . | . |
| 21-225_22G9_HC | . | . | . | N | . | . | . | . | . | . | . | . | . | . | . | . | E |
| 21-225_34C4_HC | . | . | . | . | . | . | . | . | . | . | . | . | S | . | . | . | . |
| 21-225_2F7_HC | . | . | . | . | . | . | . | . | . | . | . | . | . | . | . | . | E |
| 21-225_3G4_HC | . | . | . | . | . | . | . | . | . | . | . | . | . | . | . | . | . |
| 21-225_7F4_HC | . | . | . | . | . | . | . | . | . | . | . | . | . | . | . | . | . |
| 21-225_6D4_HC | . | . | . | . | S | . | . | . | . | . | . | . | . | . | . | . | . |
| 21-225_15A1_HC | . | . | . | . | . | . | . | . | . | . | . | . | . | . | . | . | . |
| 21-225_15G7_HC | . | . | . | . | S | . | . | . | . | . | . | . | . | . | . | . | . |
| 21-225_11F10_HC | . | . | . | . | . | . | . | . | . | . | . | . | . | . | . | . | . |
| 21-225_15H1_HC | . | . | . | . | . | . | . | . | . | . | . | . | . | . | . | . | . |
| 21-225_16A1_HC | . | . | . | . | . | . | . | . | . | . | . | . | . | . | . | . | . |
| 21-225_8D12_HC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | | H_CDR2 | |
| CONSENSUS | L | E | W | I | G | N | I | Y | Y | - | - | - | - | S | G | S | T |
| 21-225_20C2_HC | . | . | . | . | . | . | . | . | . | . | . | . | . | . | . | T | A |
| 21-225_18C6_HC | . | . | . | . | . | . | . | . | . | . | . | . | . | . | . | Y | . |
| 21-225_18A3_HC | . | . | . | . | . | . | . | . | . | . | . | . | . | . | . | Y | . |
| 21-225_22D12_HC | . | . | . | . | . | . | . | . | . | . | . | . | . | . | . | T | . |
| 21-225_23D1_HC | . | . | . | . | . | S | . | . | . | . | . | . | . | . | . | . | A |
| 21-225_24E5_HC | . | . | . | . | . | S | . | . | . | . | . | . | . | . | . | . | A |
| 21-225_22C1_HC | . | . | . | . | . | . | . | . | . | . | . | . | . | . | . | A | . |
| 21-225_20G9_HC | . | . | . | . | . | S | . | . | . | . | . | . | . | . | . | . | A |
| 21-225_21G7_HC | Q | . | . | . | . | . | . | . | . | . | . | . | . | . | . | Y | S |
| 21-225_22G9_HC | . | . | . | . | . | . | . | . | . | . | . | . | . | . | . | . | . |
| 21-225_34C4_HC | . | . | . | . | . | S | . | . | . | . | . | . | . | . | . | . | . |
| 21-225_2F7_HC | . | . | . | . | . | . | . | . | . | . | . | . | . | . | . | . | . |
| 21-225_3G4_HC | . | . | . | . | . | . | . | . | . | . | . | . | . | . | . | I | P |
| 21-225_7F4_HC | . | . | . | . | . | . | . | . | . | . | . | . | . | . | . | A | . |
| 21-225_6D4_HC | . | . | . | . | . | . | . | . | . | . | . | . | . | . | . | . | A |
| 21-225_15A1_HC | . | . | . | . | . | S | . | . | . | . | . | . | . | . | . | . | A |
| 21-225_15G7_HC | . | . | . | . | . | S | . | . | . | . | . | . | . | . | . | Y | . |
| 21-225_11F10_HC | . | . | . | . | . | S | . | . | . | . | . | . | . | . | . | . | A |
| 21-225_15H1_HC | . | . | . | . | . | . | . | . | . | . | . | . | . | . | . | Y | P |
| 21-225_16A1_HC | . | . | . | . | . | S | . | . | . | . | . | . | . | . | . | . | A |
| 21-225_8D12_HC | . | . | . | . | . | . | . | . | . | . | . | . | . | . | . | Y | P |

## FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | N | P | S | L | K | S | R | V | T | I | S | V | D | T | S |
| 21-225_20C2_HC | . | C | . | S | . | . | . | . | . | . | I | . | . | . | . | . | . |
| 21-225_18C6_HC | . | . | . | . | . | . | . | . | . | . | I | . | . | . | . | . | . |
| 21-225_18A3_HC | . | . | T | . | . | . | . | . | . | . | . | . | . | . | . | . | . |
| 21-225_22D12_HC | S | . | . | . | . | . | . | . | . | . | . | . | . | . | . | . | . |
| 21-225_23D1_HC | Q | . | . | . | . | . | . | . | . | . | . | . | . | . | . | . | . |
| 21-225_24E5_HC | N | . | . | . | . | . | . | . | . | . | . | . | . | . | . | . | . |
| 21-225_22C1_HC | . | . | . | S | . | . | . | . | . | . | . | . | . | . | . | . | . |
| 21-225_20G9_HC | S | . | . | . | . | . | . | . | . | . | . | . | . | . | . | . | . |
| 21-225_21G7_HC | . | . | . | . | . | . | . | . | . | . | . | . | . | . | . | . | . |
| 21-225_22G9_HC | . | . | . | . | . | . | . | . | . | . | . | . | . | . | . | . | . |
| 21-225_34C4_HC | A | . | . | . | . | . | . | . | . | . | . | . | . | . | . | . | . |
| 21-225_2F7_HC | . | . | . | . | . | . | . | . | . | . | . | . | . | . | . | . | . |
| 21-225_3G4_HC | . | . | . | . | . | . | . | . | . | . | N | . | . | . | . | . | . |
| 21-225_7F4_HC | . | . | . | . | . | . | . | . | . | . | . | . | . | . | . | . | . |
| 21-225_6D4_HC | . | . | . | I | . | . | . | . | . | . | . | . | . | A | . | . | . |
| 21-225_15A1_HC | Q | . | . | . | . | . | . | . | . | . | . | . | . | . | . | . | T |
| 21-225_15G7_HC | . | . | T | . | . | . | . | . | . | . | . | . | . | . | . | . | . |
| 21-225_11F10_HC | Q | . | . | . | . | . | . | . | . | . | . | . | . | . | . | . | . |
| 21-225_15H1_HC | . | . | . | . | . | . | . | . | . | . | . | . | . | . | . | . | . |
| 21-225_16A1_HC | . | H | . | . | . | . | . | . | . | G | . | . | . | . | . | . | . |
| 21-225_8D12_HC | . | . | . | . | . | . | . | . | . | . | . | . | . | I | . | . | . |

FIGURE 56

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | H | S | S | S | W | - | - | - | - | - | - |
| 21-225_20C2_HC | . | . | . | . | . | . | . | . | . | . | . | . | . | . | . | . | . |
| 21-225_18C6_HC | L | . | . | . | . | . | . | . | . | . | . | . | . | . | . | . | . |
| 21-225_18A3_HC | . | . | . | . | . | . | . | . | T | . | . | . | . | . | . | . | . |
| 21-225_22D12_HC | . | F | . | . | . | . | L | . | . | . | . | . | . | . | . | . | . |
| 21-225_23D1_HC | L | . | . | . | . | . | L | . | . | . | . | . | . | . | . | . | . |
| 21-225_24E5_HC | . | . | . | . | . | . | L | . | . | . | . | . | . | . | . | . | . |
| 21-225_22C1_HC | . | . | . | . | . | . | . | . | G | . | . | . | . | . | . | . | . |
| 21-225_20G9_HC | A | . | . | . | . | . | L | . | . | . | . | . | . | . | . | . | . |
| 21-225_21G7_HC | . | . | . | . | . | . | . | . | T | . | . | . | . | . | . | . | . |
| 21-225_22G9_HC | . | . | . | . | . | . | . | . | . | . | . | . | . | . | . | . | . |
| 21-225_34C4_HC | . | . | . | . | . | . | L | . | . | . | . | . | . | . | . | . | . |
| 21-225_2F7_HC | . | . | . | . | . | . | . | . | T | . | . | . | . | . | . | . | . |
| 21-225_3G4_HC | . | . | . | . | . | . | . | . | . | . | . | . | . | . | . | . | . |
| 21-225_7F4_HC | . | . | . | . | . | . | . | . | G | . | . | . | . | . | . | . | . |
| 21-225_6D4_HC | . | . | . | . | . | . | . | . | . | . | . | . | . | . | . | . | . |
| 21-225_15A1_HC | . | . | . | . | . | . | L | . | . | . | . | . | . | . | . | . | . |
| 21-225_15G7_HC | S | . | . | . | . | . | L | . | . | . | . | . | . | . | . | . | . |
| 21-225_11F10_HC | . | . | . | . | . | . | L | . | . | . | . | . | . | . | . | . | . |
| 21-225_15H1_HC | . | . | . | . | . | . | . | . | T | . | . | . | . | . | . | . | . |
| 21-225_16A1_HC | A | . | . | . | . | . | L | . | . | . | . | . | . | . | . | . | . |
| 21-225_8D12_HC | . | . | . | . | . | . | . | . | T | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | - | - | S | L |
| 21-225_20C2_HC | | | | | | | | | | | | | | | | | |
| 21-225_18C6_HC | | | | | | | | | | | | | | | | | |
| 21-225_18A3_HC | | | | | | | | | | | | | | | | | |
| 21-225_22D12_HC | | | | | | | | | | | | | | | | | V |
| 21-225_23D1_HC | | | | | | | | | | | | | | | | | F |
| 21-225_24E5_HC | | | | | | | | | | | | | | | | | I |
| 21-225_22C1_HC | | | | | | | | | | | | | | | | | |
| 21-225_20G9_HC | | | | | | | | | | | | | | | | | F |
| 21-225_21G7_HC | | | | | | | | | | | | | | | | | |
| 21-225_22G9_HC | | | | | | | | | | | | | | | | | |
| 21-225_34C4_HC | | | | | | | | | | | | | | | | | F |
| 21-225_2F7_HC | | | | | | | | | | | | | | | | | |
| 21-225_3G4_HC | | | | | | | | | | | | | | | | | |
| 21-225_7F4_HC | | | | | | | | | | | | | | | | | |
| 21-225_6D4_HC | | | | | | | | | | | | | | | | | |
| 21-225_15A1_HC | | | | | | | | | | | | | | | | | F |
| 21-225_15G7_HC | | | | | | | | | | | | | | | | | F |
| 21-225_11F10_HC | | | | | | | | | | | | | | | | | F |
| 21-225_15H1_HC | | | | | | | | | | | | | | | | | |
| 21-225_16A1_HC | | | | | | | | | | | | | | | | | F |
| 21-225_8D12_HC | | | | | | | | | | | | | | | | | |

**FIGURE 56**

| Reference # | 137 | 138 | 139 | 140 | 141 | 142 | 143 | 144 | 145 | 146 | 147 | 148 | 149 |
|---|---|---|---|---|---|---|---|---|---|---|---|---|
| | | | | | | | H_FR4 | | | | | | |
| CONSENSUS | D | Y | W | G | Q | G | T | L | V | T | V | S | S |
| 21-225_20C2_HC | . | N | . | . | . | . | . | . | . | . | . | . | . |
| 21-225_18C6_HC | . | D | . | . | . | . | . | . | . | . | . | . | . |
| 21-225_18A3_HC | . | . | . | . | . | . | . | . | . | . | . | . | . |
| 21-225_22D12_HC | . | . | . | . | . | . | . | . | . | . | . | . | . |
| 21-225_23D1_HC | . | . | . | . | . | . | . | . | . | . | . | . | . |
| 21-225_24E5_HC | . | N | . | . | . | . | . | . | . | . | . | . | . |
| 21-225_22C1_HC | . | . | . | . | . | . | . | . | . | . | . | . | . |
| 21-225_20G9_HC | . | . | . | . | . | . | . | . | . | . | . | . | . |
| 21-225_21G7_HC | . | . | . | . | . | . | . | . | . | . | . | . | . |
| 21-225_22G9_HC | . | . | . | . | . | . | . | . | . | . | . | . | . |
| 21-225_34C4_HC | . | . | . | . | . | . | . | . | . | . | . | . | . |
| 21-225_2F7_HC | . | . | . | . | . | . | . | . | . | . | . | . | . |
| 21-225_3G4_HC | . | . | . | . | . | . | . | . | . | . | . | . | . |
| 21-225_7F4_HC | . | . | . | . | . | . | . | . | . | . | . | . | . |
| 21-225_6D4_HC | . | C | . | . | . | . | . | . | . | . | . | . | . |
| 21-225_15A1_HC | . | . | . | . | . | . | . | . | . | . | . | . | . |
| 21-225_15G7_HC | . | F | . | . | . | . | . | . | . | . | . | . | . |
| 21-225_11F10_HC | . | . | . | . | . | . | . | . | . | . | . | . | . |
| 21-225_15H1_HC | . | . | . | . | . | . | . | . | . | . | . | . | . |
| 21-225_16A1_HC | . | . | . | . | . | . | . | . | . | . | . | . | . |
| 21-225_8D12_HC | . | . | . | . | . | . | . | . | . | . | . | . | . |

FIGURE 56

Table 34

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | L | Q | L | Q | E | S | - | G | P | G | L | V | K | P | S | E |
| 21-225_17H8_HC | . | . | . | . | . | . | . | . | . | . | . | . | . | . | . | . | . |
| 21-225_17F9_HC | . | . | . | . | . | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | . | . | . | . | . | . | . | . | . | . | . | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | . | . | . | . | . | . | . | . | . | . | . | . | . | . | . |
| 21-225_14B2_HC | . | . | . | . | . | . | . | . | . | . | . | . | . | . | . | . | . |
| 21-225_4F4_HC | . | . | . | . | . | . | . | . | . | . | . | . | . | R | . | . | . |
| 21-225_4H4_HC | . | . | . | . | . | . | . | . | . | . | . | . | . | . | . | . | . |
| 21-225_11F5_HC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | T | L | S | L | T | C | T | V | S | G | - | G | S | I | S | R | S |
| 21-225_17H8_HC | . | . | . | . | . | . | . | . | . | . | . | . | . | . | . | . | . |
| 21-225_17F9_HC | . | . | . | . | . | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | . | . | . | . | . | . | . | . | . | . | N | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | . | . | . | . | . | . | . | . | . | . | . | . | N | . | . |
| 21-225_14B2_HC | . | . | . | . | . | . | . | . | . | . | . | . | . | . | N | . | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | . | . |
| 21-225_11F5_HC | . | . | . | . | . | . | . | . | . | . | . | . | . | . | . | . | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | | | | H_FR2 | |
| CONSENSUS | - | - | - | S | Y | Y | W | G | W | I | R | Q | P | P | G | K | G |
| 21-225_17H8_HC | . | . | . | . | . | . | . | . | . | . | . | . | . | . | . | R | . |
| 21-225_17F9_HC | . | . | . | . | . | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | . | . | . | . | . | . | . | . | . | . | . | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | Q | . |
| 21-225_5H7_HC | . | . | . | . | . | . | . | . | . | . | . | . | . | . | . | . | . |
| 21-225_14B2_HC | . | . | . | . | . | . | . | . | . | . | . | . | . | . | . | . | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | M | . |
| 21-225_11F5_HC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | | H_CDR2 | |
| CONSENSUS | L | E | W | I | G | N | I | Y | Y | - | - | - | - | S | G | S | T |
| 21-225_17H8_HC | . | . | . | . | . | . | . | . | . | . | . | . | . | G | . | N | . |
| 21-225_17F9_HC | . | . | . | . | . | . | . | . | . | . | . | . | . | . | . | . | A |
| 21-225_1F2_HC | . | . | . | . | . | . | . | . | . | . | . | . | . | . | . | . | A |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | T | . |
| 21-225_22G8_HC | . | D | . | . | . | . | . | . | . | . | . | . | . | . | . | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | . | . | . | . | . | . | . | . | . | . | . | . | . | T | . |
| 21-225_14B2_HC | . | . | . | . | . | . | . | . | . | . | . | . | . | . | . | T | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | N | . |
| 21-225_11F5_HC | . | . | . | . | . | . | . | . | . | . | . | . | . | . | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | N | P | S | L | K | S | R | V | T | I | S | V | D | T | S |
| 21-225_17H8_HC | . | . | . | . | . | . | . | . | . | . | . | . | . | . | . | . | . |
| 21-225_17F9_HC | . | . | . | . | . | . | . | . | . | . | . | . | . | . | . | S | . |
| 21-225_1F2_HC | . | N | . | . | . | . | . | G | . | . | . | . | . | . | . | . | . |
| 21-225_21H3_HC | . | D | . | . | . | . | . | . | . | . | . | . | . | . | . | . | . |
| 21-225_23C8_HC | . | N | . | . | . | . | . | . | . | . | . | . | . | . | . | . | . |
| 21-225_22G8_HC | . | . | . | . | . | V | . | . | . | F | . | . | . | . | . | . | . |
| 21-225_22H4_HC | . | H | . | . | . | . | . | . | . | . | . | . | . | . | . | . | . |
| 21-225_23A3_HC | . | N | . | . | . | . | . | . | . | . | S | . | . | . | . | . | . |
| 21-225_5H7_HC | . | . | . | . | . | . | . | . | . | . | . | . | . | . | . | . | . |
| 21-225_14B2_HC | . | . | . | . | . | . | . | . | . | . | . | . | . | . | . | . | . |
| 21-225_4F4_HC | . | D | . | . | . | . | . | . | . | . | . | . | . | . | . | . | . |
| 21-225_4H4_HC | . | N | . | . | . | . | . | . | . | . | . | . | . | . | . | . | . |
| 21-225_11F5_HC | . | T | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | | H_FR3 | | | | | | | | | | |
| CONSENSUS | K | N | Q | F | S | L | K | L | S | S | V | T | A | A | D | T | A |
| 21-225_17H8_HC | . | . | . | . | . | . | . | . | . | . | . | . | . | . | . | . | . |
| 21-225_17F9_HC | . | . | . | . | . | . | N | . | N | . | . | . | . | . | . | . | . |
| 21-225_1F2_HC | . | . | . | . | . | . | . | . | N | . | . | . | . | . | . | . | . |
| 21-225_21H3_HC | . | . | . | . | . | . | . | . | . | . | . | . | . | . | . | . | . |
| 21-225_23C8_HC | . | . | H | . | . | . | . | . | . | . | . | . | . | . | . | . | . |
| 21-225_22G8_HC | . | . | . | . | . | . | . | . | . | . | . | . | . | . | . | . | . |
| 21-225_22H4_HC | M | . | . | . | . | . | . | . | T | . | . | . | . | . | . | . | . |
| 21-225_23A3_HC | . | . | . | . | . | . | N | . | . | . | . | . | . | . | . | . | . |
| 21-225_5H7_HC | . | . | . | . | . | . | N | . | . | . | . | . | . | . | . | . | . |
| 21-225_14B2_HC | . | . | . | . | . | . | N | . | . | . | . | . | . | . | . | . | . |
| 21-225_4F4_HC | . | . | . | . | . | . | . | . | . | . | . | . | . | . | . | . | . |
| 21-225_4H4_HC | . | . | . | . | . | . | . | . | N | . | . | . | . | . | . | . | . |
| 21-225_11F5_HC | . | . | H | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | G | R | H | G | K | D | W | - | - | - | - | - | - |
| 21-225_17H8_HC | . | . | . | . | A | . | . | . | . | . | . | . | . | . | . | . | . |
| 21-225_17F9_HC | . | . | S | . | A | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | A | . | . | . | . | . | . | . | . | . | . | . | . |
| 21-225_22H4_HC | . | . | F | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | S | . | A | . | . | . | . | . | . | . | . | . | . | . | . |
| 21-225_14B2_HC | . | . | S | . | A | . | . | . | . | . | . | . | . | . | . | . | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | . | . |
| 21-225_11F5_HC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | - | - | G | L |
| 21-225_17H8_HC | . | . | . | . | . | . | . | . | . | . | . | . | . | . | . | . | . |
| 21-225_17F9_HC | . | . | . | . | . | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | . | . | . | . | . | . | . | . | . | . | . | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | . | . | . | . | . | . | . | . | . | . | . | . | . | . | . |
| 21-225_14B2_HC | . | . | . | . | . | . | . | . | . | . | . | . | . | . | . | . | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | . | . |
| 21-225_11F5_HC | . | . | . | . | . | . | . | . | . | . | . | . | . | . | . | . | . |

**FIGURE 56**

| Reference # | 137 | 138 | 139 | 140 | 141 | 142 | 143 | 144 | 145 | 146 | 147 | 148 | 149 |
|---|---|---|---|---|---|---|---|---|---|---|---|---|
| | | | | | | | H_FR4 | | | | | | |
| CONSENSUS | D | Y | W | G | Q | G | T | L | V | T | V | S | S |
| 21-225_17H8_HC | . | . | . | . | . | . | . | . | . | . | . | . | . |
| 21-225_17F9_HC | . | . | . | . | . | . | . | . | . | . | . | . | . |
| 21-225_1F2_HC | . | . | . | . | . | . | . | . | . | . | . | . | . |
| 21-225_21H3_HC | . | . | . | . | . | . | . | . | . | . | . | . | . |
| 21-225_23C8_HC | . | . | . | . | . | . | . | . | . | . | . | . | . |
| 21-225_22G8_HC | . | . | . | . | . | . | . | . | . | . | . | . | . |
| 21-225_22H4_HC | . | . | . | . | . | . | . | . | . | . | . | . | . |
| 21-225_23A3_HC | . | F | . | . | . | . | . | . | . | . | . | . | . |
| 21-225_5H7_HC | . | . | . | . | . | . | . | . | . | . | . | . | . |
| 21-225_14B2_HC | . | . | . | . | . | . | A | . | . | . | . | . | . |
| 21-225_4F4_HC | . | . | . | . | . | . | . | . | . | . | . | . | . |
| 21-225_4H4_HC | . | . | . | . | . | . | . | . | . | I | . | . | . |
| 21-225_11F5_HC | . | N | . | . | . | . | . | . | . | . | . | . | . |

FIGURE 56

Table 35

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K_FR1 | | | | | |
| CONSENSUS | D | I | Q | M | T | Q | S | P | S | S | V | S | A | S | V | G | D |
| 21-225_30A11_LC | . | . | . | . | . | . | . | . | . | . | . | . | . | . | . | . | . |
| 21-225_34F3_LC | . | . | . | . | . | . | . | . | . | . | . | . | . | T | . | . | . |
| 21-225_35C6_LC | . | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | . | . | . | . | . | . | . | . | . | . | . | . | . | . | . | . |
| 21-225_33G1_LC | . | . | . | . | . | . | . | . | . | . | . | . | V | . | . | . | . |
| 21-225_34G7_LC | . | . | . | . | . | . | . | . | . | . | . | . | . | . | . | . | . |
| 21-225_33H7_LC | . | . | . | . | . | . | . | . | . | . | . | . | . | . | . | . | . |
| 21-225_35H12_LC | . | . | . | . | . | . | . | . | . | . | . | . | . | . | . | . | . |
| 21-225_4H6_LC | . | . | . | . | . | . | . | . | . | . | . | . | . | . | . | . | . |
| 21-225_18D4_LC | . | . | . | . | . | . | . | . | . | . | L | . | . | . | . | . | . |
| 21-225_23B11_LC | . | . | . | . | . | . | . | . | . | . | . | . | . | . | . | . | . |
| 21-225_31C4_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | S | - | - |
| 21-225_30A11_LC | . | . | . | . | . | . | . | . | . | . | . | . | . | . | . | . | . |
| 21-225_34F3_LC | . | . | S | . | . | . | . | . | . | . | . | . | . | . | . | . | . |
| 21-225_35C6_LC | . | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | L | . | . | . | . | . | . | . | . | . | . | . | . | . | . | . |
| 21-225_33G1_LC | . | . | . | . | . | . | . | . | . | . | . | . | . | . | . | . | . |
| 21-225_34G7_LC | . | . | . | . | . | . | . | . | . | . | . | . | L | . | . | . | . |
| 21-225_33H7_LC | . | . | . | . | . | . | . | . | . | . | . | . | . | . | . | . | . |
| 21-225_35H12_LC | . | . | . | . | . | . | . | . | . | . | . | . | . | . | . | . | . |
| 21-225_4H6_LC | . | . | . | . | . | . | . | . | . | . | . | . | . | . | . | . | . |
| 21-225_18D4_LC | . | . | . | . | . | . | . | . | . | . | . | . | . | . | R | . | . |
| 21-225_23B11_LC | . | . | . | . | . | . | . | . | . | . | . | . | . | . | T | . | . |
| 21-225_31C4_LC | . | . | . | . | . | . | . | . | . | . | . | . | . | . | . | . | . |

EP 4 435 105 A2

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | K_FR2 | | |
| CONSENSUS | - | - | - | - | R | W | L | A | W | Y | Q | Q | K | P | G | K | A |
| 21-225_30A11_LC | . | . | . | . | . | . | . | . | . | . | . | . | . | . | . | R | . |
| 21-225_34F3_LC | . | . | . | . | K | . | . | . | . | . | . | . | . | . | . | . | . |
| 21-225_35C6_LC | . | . | . | . | K | . | . | . | . | . | . | . | . | . | . | . | . |
| 21-225_36H5_LC | . | . | . | . | . | . | . | . | . | . | . | . | . | . | . | . | . |
| 21-225_35G6_LC | . | . | . | . | . | . | . | . | . | . | . | . | . | V | . | . | V |
| 21-225_33G1_LC | . | . | . | . | . | . | . | . | . | . | . | . | . | . | . | . | . |
| 21-225_34G7_LC | . | . | . | . | K | . | . | . | . | . | . | . | . | . | . | . | . |
| 21-225_33H7_LC | . | . | . | . | . | . | . | . | . | . | . | . | . | V | . | . | V |
| 21-225_35H12_LC | . | . | . | . | . | . | . | . | . | . | . | . | . | . | . | . | . |
| 21-225_4H6_LC | . | . | . | . | . | . | . | . | . | . | . | . | . | . | . | . | . |
| 21-225_18D4_LC | . | . | . | . | N | D | . | G | . | . | . | . | . | . | . | . | . |
| 21-225_23B11_LC | . | . | . | . | K | . | . | . | . | . | . | . | . | . | . | . | . |
| 21-225_31C4_LC | . | . | . | . | K | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | L | L | I | Y | A | - | - | - | - | - | - | - | - | A | S |
| 21-225_30A11_LC | . | E | . | . | . | . | . | . | . | . | . | . | . | . | . | . | . |
| 21-225_34F3_LC | . | . | . | . | . | . | . | . | . | . | . | . | . | . | . | . | . |
| 21-225_35C6_LC | . | . | P | . | . | . | . | . | . | . | . | . | . | . | . | . | . |
| 21-225_36H5_LC | . | E | . | . | . | . | . | . | . | . | . | . | . | . | . | . | . |
| 21-225_35G6_LC | . | . | . | . | . | . | . | . | . | . | . | . | . | . | . | . | . |
| 21-225_33G1_LC | . | . | . | . | . | . | G | . | . | . | . | . | . | . | . | . | Y |
| 21-225_34G7_LC | . | . | . | . | . | . | . | . | . | . | . | . | . | . | . | . | . |
| 21-225_33H7_LC | . | . | . | . | . | . | . | . | . | . | . | . | . | . | . | . | . |
| 21-225_35H12_LC | . | E | . | . | . | . | . | . | . | . | . | . | . | . | . | . | . |
| 21-225_4H6_LC | . | . | . | . | . | . | G | . | . | . | . | . | . | . | . | . | . |
| 21-225_18D4_LC | . | . | R | . | . | . | . | . | . | . | . | . | . | . | . | . | . |
| 21-225_23B11_LC | . | . | V | . | . | . | . | . | . | . | . | . | . | . | . | . | . |
| 21-225_31C4_LC | . | . | P | . | . | . | . | . | . | . | . | . | . | . | . | . | . |

3542

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_30A11_LC | R | . | | | . | I | . | . | . | . | . | . | . | . | . | . | . |
| 21-225_34F3_LC | . | . | . | . | . | . | . | . | . | . | . | . | . | . | . | . | . |
| 21-225_35C6_LC | . | . | . | . | . | . | . | T | . | . | . | . | . | . | . | . | . |
| 21-225_36H5_LC | R | . | | | . | I | . | . | . | . | . | . | . | . | . | . | . |
| 21-225_35G6_LC | R | . | | | . | . | . | . | . | . | . | . | . | . | . | . | . |
| 21-225_33G1_LC | | | . | . | . | . | . | . | . | . | . | . | . | . | . | . | . |
| 21-225_34G7_LC | . | . | . | . | . | . | . | L | . | . | . | . | . | . | . | . | . |
| 21-225_33H7_LC | R | . | | | . | . | . | . | . | . | . | . | . | . | . | . | . |
| 21-225_35H12_LC | R | . | | | . | I | . | . | . | . | . | . | . | . | . | . | . |
| 21-225_4H6_LC | | | . | . | . | . | . | . | . | . | . | . | . | . | . | . | . |
| 21-225_18D4_LC | | | E | | . | . | . | . | . | . | . | . | . | . | . | . | . |
| 21-225_23B11_LC | . | . | . | R | . | . | . | . | . | . | . | . | . | R | . | . | . |
| 21-225_31C4_LC | . | . | . | . | . | . | . | T | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | K_FR3 | | | | | | | | | | | |
| CONSENSUS | - | T | D | F | T | L | T | I | S | S | L | Q | P | E | D | F | A |
| 21-225_30A11_LC | . | . | . | . | . | . | . | . | . | . | . | . | . | . | . | . | . |
| 21-225_34F3_LC | . | . | . | . | . | . | . | . | . | . | . | . | . | . | . | S | . |
| 21-225_35C6_LC | . | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | . | A | . | . | . | . | G | . | . | . | . | . | . | . | . | . |
| 21-225_33G1_LC | . | . | . | . | . | . | . | . | . | . | . | . | . | . | . | . | . |
| 21-225_34G7_LC | . | . | . | . | . | . | . | . | . | . | . | . | . | . | . | . | . |
| 21-225_33H7_LC | . | . | . | . | . | . | . | G | . | . | . | . | . | . | . | . | . |
| 21-225_35H12_LC | . | . | . | . | . | . | . | . | . | . | . | . | . | . | . | . | . |
| 21-225_4H6_LC | . | . | . | . | . | . | . | . | . | . | . | . | . | . | . | . | . |
| 21-225_18D4_LC | . | . | E | . | . | . | . | V | . | . | . | . | . | . | . | . | . |
| 21-225_23B11_LC | . | . | . | . | . | . | . | . | . | . | . | . | . | . | . | . | . |
| 21-225_31C4_LC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | A | N | S | - | - | - | - | - | - | - | - |
| 21-225_30A11_LC | . | . | . | . | . | . | . | . | . | . | . | . | . | . | . | . | . |
| 21-225_34F3_LC | . | . | . | . | . | . | . | . | . | . | . | . | . | . | . | . | . |
| 21-225_35C6_LC | I | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | . | . | . | . | . | . | . | . | . | . | . | . | . | . | . | . |
| 21-225_33G1_LC | . | . | . | . | . | . | . | . | . | . | . | . | . | . | . | . | . |
| 21-225_34G7_LC | . | . | . | . | . | . | . | . | . | . | . | . | . | . | . | . | . |
| 21-225_33H7_LC | . | . | . | . | . | . | . | . | I | . | . | . | . | . | . | . | . |
| 21-225_35H12_LC | . | . | . | . | . | . | . | . | . | . | . | . | . | . | . | . | . |
| 21-225_4H6_LC | I | . | . | . | . | . | . | . | . | . | . | . | . | . | . | . | . |
| 21-225_18D4_LC | . | . | . | . | L | H | H | . | N | . | . | . | . | . | . | . | . |
| 21-225_23B11_LC | . | . | . | . | . | . | . | . | . | . | . | . | . | . | . | . | . |
| 21-225_31C4_LC | I | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | K_CDR3 | | | | | | | | | | | | | | | |
| CONSENSUS | - | - | - | - | - | - | - | - | - | - | - | - | - | - | - | F | P |
| 21-225_30A11_LC | . | . | . | . | . | . | . | . | . | . | . | . | . | . | . | . | . |
| 21-225_34F3_LC | . | . | . | . | . | . | . | . | . | . | . | . | . | . | . | . | . |
| 21-225_35C6_LC | . | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | . | . | . | . | . | . | . | . | . | . | . | . | . | . | . | . |
| 21-225_33G1_LC | . | . | . | . | . | . | . | . | . | . | . | . | . | . | . | . | . |
| 21-225_34G7_LC | . | . | . | . | . | . | . | . | . | . | . | . | . | . | . | . | . |
| 21-225_33H7_LC | . | . | . | . | . | . | . | . | . | . | . | . | . | . | . | . | . |
| 21-225_35H12_LC | . | . | . | . | . | . | . | . | . | . | . | . | . | . | . | . | . |
| 21-225_4H6_LC | . | . | . | . | . | . | . | . | . | . | . | . | . | . | . | . | . |
| 21-225_18D4_LC | . | . | . | . | . | . | . | . | . | . | . | . | . | . | Y | L | . |
| 21-225_23B11_LC | . | . | . | . | . | . | . | . | . | . | . | . | . | . | . | . | . |
| 21-225_31C4_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|
| | | | | | | | K_FR4 | | | | | | |
| CONSENSUS | F | T | F | G | P | G | T | K | V | D | I | K | R |
| 21-225_30A11_LC | . | . | . | . | . | . | . | . | . | . | N | . | . |
| 21-225_34F3_LC | . | . | . | . | . | . | . | . | . | . | . | . | . |
| 21-225_35C6_LC | . | . | . | . | . | . | . | . | . | . | . | . | . |
| 21-225_36H5_LC | . | . | . | . | . | . | . | . | . | . | . | . | . |
| 21-225_35G6_LC | . | . | . | . | . | . | . | . | . | . | L | . | . |
| 21-225_33G1_LC | . | . | . | . | . | . | . | . | . | . | . | . | . |
| 21-225_34G7_LC | . | . | . | . | . | . | . | . | . | . | . | . | . |
| 21-225_33H7_LC | . | . | . | . | . | . | . | . | . | . | L | . | . |
| 21-225_35H12_LC | . | . | . | . | . | . | . | . | . | . | . | . | . |
| 21-225_4H6_LC | . | . | . | . | . | . | . | . | . | . | . | . | G |
| 21-225_18D4_LC | I | . | . | . | Q | . | . | R | L | E | . | . | . |
| 21-225_23B11_LC | . | . | . | . | . | . | . | . | . | . | . | . | . |
| 21-225_31C4_LC | . | . | . | . | . | . | . | . | . | . | . | . | . |

**FIGURE 56**

Table 36

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| Linear # | 1 | 2 | 3 | 4 | 5 | 6 | 7 | 8 | 9 | 10 | 11 | 12 | 13 | 14 | 15 | 16 | 17 |
| | | | | | | | | | | | | | K_FR1 | | | | |
| 21-225_31D12_LC | D | V | V | M | T | Q | S | P | L | S | L | P | V | T | L | G | Q |
| | | | | | | | | | | | | | | | | | |
| CONSENSUS | S | Y | E | L | T | Q | P | - | P | S | V | S | V | S | P | G | Q |
| 21-225_18E10_LC | . | . | . | . | . | . | . | . | . | . | . | . | . | . | . | . | . |
| 21-225_19D1_LC | . | . | . | . | . | . | . | . | . | . | . | . | . | . | . | . | . |
| 21-225_4B3_LC | . | . | . | . | . | . | . | . | . | . | . | . | . | . | . | R | . |
| 21-225_21D12_LC | . | . | . | . | . | . | . | . | . | . | . | . | . | . | . | . | . |
| 21-225_13F6_LC | . | . | . | . | . | . | . | . | . | . | . | . | . | . | . | . | . |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | . | . |
| 21-225_17D3_LC | . | . | . | . | . | . | S | . | . | . | . | . | . | . | . | . | . |
| 21-225_16F3_LC | . | . | V | . | . | . | . | . | . | . | . | . | . | A | . | . | . |
| 21-225 9G9 LC | Q | S | V | . | . | . | . | . | . | . | A | . | G | T | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| Linear # | 18 | 19 | 20 | 21 | 22 | 23 | 24 | 25 | 26 | 26.1 | 26.2 | 27 | 28 | 29 | 30 | 31 | 32 |
| | | | | | | | | | | | | | | | | K_CDR1 | |
| 21-225_31D12_LC | P | A | S | I | S | C | R | S | S | - | - | Q | S | L | I | Y | S |
| | | | | | | | | | | | | | | | | | |
| CONSENSUS | T | A | S | I | T | C | S | G | D | - | - | - | K | L | G | D | - |
| 21-225_18E10_LC | . | . | . | . | . | . | . | . | . | . | . | . | R | . | . | . | . |
| 21-225_19D1_LC | . | . | . | . | . | . | . | . | . | . | . | . | . | . | . | N | . |
| 21-225_4B3_LC | . | V | . | . | . | . | . | . | . | . | . | . | . | . | . | N | . |
| 21-225_21D12_LC | . | . | . | . | . | . | . | . | . | . | . | . | . | . | . | N | . |
| 21-225_13F6_LC | . | . | . | . | . | . | . | . | . | . | . | . | . | . | . | . | . |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | E | . |
| 21-225_17D3_LC | . | . | . | . | . | . | . | . | . | . | . | . | . | . | . | . | . |
| 21-225_16F3_LC | . | . | R | . | . | . | G | . | N | . | . | . | N | I | . | S | . |
| 21-225_9G9_LC | R | V | N | M | S | . | . | . | T | N | . | S | N | I | . | S | . |

**FIGURE 56**

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| Linear # | 58 | 59 | 60 | 61 | 62 | 63 | 64 | 65 | 66 | 67 | 68 | 69 | 70 | 71 | 72 | 73 | 73.1 |
| 21-225_31D12_LC | N | W | D | S | G | V | P | D | R | F | S | G | S | G | S | G | - |
| CONSENSUS | K | R | P | S | G | I | P | E | R | F | S | G | S | N | S | G | - |
| 21-225_18E10_LC | . | . | . | . | . | . | . | . | . | . | . | . | . | . | . | . | . |
| 21-225_19D1_LC | . | . | . | . | . | . | . | . | . | . | . | . | . | . | . | . | . |
| 21-225_4B3_LC | . | . | . | . | . | . | . | . | . | . | . | . | . | . | . | . | . |
| 21-225_21D12_LC | . | . | . | . | . | . | . | . | . | . | . | . | . | . | . | . | . |
| 21-225_13F6_LC | . | . | . | . | . | . | . | . | . | . | . | . | . | . | . | . | . |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | . | . |
| 21-225_17D3_LC | . | . | . | . | . | . | . | . | . | . | . | . | . | . | . | . | . |
| 21-225_16F3_LC | D | . | . | . | . | . | . | . | . | . | . | . | . | . | . | . | . |
| 21-225_9G9_LC | Q | . | . | . | . | V | . | D | . | . | . | . | . | K | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| Linear # | 73.2 | 74 | 75 | 76 | 77 | 78 | 79 | 80 | 81 | 82 | 83 | 84 | 85 | 86 | 87 | 88 | 89 |
|  |  |  |  | K_FR3 |  |  |  |  |  |  |  |  |  |  |  |  |  |
| 21-225_31D12_LC | - | T | D | F | T | L | K | I | S | R | L | E | A | E | D | V | G |
| CONSENSUS | - | N | T | A | T | L | T | I | S | G | T | Q | A | M | D | E | A |
| 21-225_18E10_LC | . | . | . | . | . | . | . | . | . | . | . | . | . | . | . | . | . |
| 21-225_19D1_LC | . | . | . | . | . | . | . | . | . | . | . | . | . | . | . | . | . |
| 21-225_4B3_LC | . | . | . | . | . | . | . | . | . | . | . | . | . | . | . | . | . |
| 21-225_21D12_LC | . | . | . | . | . | . | . | . | . | . | . | . | . | . | . | . | . |
| 21-225_13F6_LC | . | . | . | . | . | . | . | . | . | . | . | . | . | . | . | . | . |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | . | . |
| 21-225_17D3_LC | . | . | . | . | . | . | . | . | . | . | . | . | . | . | . | . | . |
| 21-225_16F3_LC | . | . | . | . | . | . | . | . | R | V | E | . | G | . | . | . | . |
| 21-225_9G9_LC | . | T | S | . | S | . | A | . | . | . | L | . | S | E | . | . | . |

**FIGURE 56**

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|
| Linear # | 101 | 102 | 103 | 104 | 105 | 106 | 107 | 108 | 109 | 110 | 111 | 112 | 113 |
| | | | | | | | K_FR4 | | | | | | |
| 21-225_31D12_LC | L | T | F | G | Q | G | T | R | L | E | I | K | R |
| | | | | | | | | | | | | | |
| CONSENSUS | V | V | F | G | G | G | T | K | L | T | V | L | G |
| 21-225_18E10_LC | . | . | . | . | . | . | . | . | . | . | . | . | . |
| 21-225_19D1_LC | . | . | . | . | . | . | . | . | . | . | . | . | . |
| 21-225_4B3_LC | . | I | . | . | . | . | . | . | . | . | . | . | . |
| 21-225_21D12_LC | . | . | . | . | . | . | . | R | . | . | . | . | . |
| 21-225_13F6_LC | . | . | . | . | . | . | . | . | . | . | . | . | . |
| 21-225_31D12_LC | A | . | . | . | . | . | . | . | . | . | . | . | . |
| 21-225_33E6_LC | A | . | . | . | . | . | . | . | . | . | . | . | . |
| 21-225_35E1_LC | A | . | . | . | . | . | . | . | . | . | . | . | . |
| 21-225_17D3_LC | A | . | . | . | . | . | . | . | . | . | . | R | . |
| 21-225_16F3_LC | . | . | . | . | . | . | . | . | . | . | . | . | . |
| 21-225_9G9_LC | . | . | . | . | . | R | . | . | . | . | . | . | . |

FIGURE 56

Table 37

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K_FR1 | | | | | |
| CONSENSUS | D | I | V | M | T | Q | S | P | D | S | L | A | V | S | L | G | E |
| 21-225_34H7_LC | . | . | . | . | . | . | . | . | . | . | . | . | . | . | . | . | . |
| 21-225_24E1_LC | . | . | . | . | . | . | . | . | . | . | . | . | . | . | . | . | . |
| 21-225_23A12_LC | . | . | . | . | . | . | . | . | . | . | . | . | . | . | . | . | . |
| 21-225_25A4_LC | . | . | . | . | . | . | F | . | . | . | . | . | . | . | . | . | . |
| 21-225_28B6_LC | . | V | . | . | . | . | . | . | L | . | . | P | . | T | . | . | Q |
| 21-225_29E6_LC | . | . | . | . | . | . | F | . | . | . | . | . | . | . | . | . | . |
| 21-225_26C4_LC | . | . | . | . | . | . | . | . | . | . | . | . | . | . | . | . | . |
| 21-225_33D1_LC | . | . | . | . | . | . | . | . | . | . | . | S | . | . | . | . | . |
| 21-225_34D2_LC | . | . | . | . | . | . | T | . | L | . | . | S | . | T | P | . | Q |
| 21-225_29H8_LC | . | . | . | . | . | . | . | . | . | . | . | . | . | . | . | . | . |
| 21-225_11E5_LC | . | . | . | . | . | . | . | . | . | . | . | . | . | . | . | . | . |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | . | . | . | . | . | . | . | . | . | . | . | . | . | . |
| 21-225_32A5_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | A | T | I | N | C | K | S | S | - | - | Q | S | V | L | Y | S |
| 21-225_34H7_LC | . | . | . | . | . | . | . | . | . | . | . | . | . | . | . | . | . |
| 21-225_24E1_LC | . | . | . | . | . | . | . | . | . | . | . | . | . | . | . | . | T |
| 21-225_23A12_LC | . | . | . | . | . | . | . | . | . | . | . | . | . | . | . | . | . |
| 21-225_25A4_LC | . | . | . | K | . | . | . | . | . | . | . | . | . | . | . | . | . |
| 21-225_28B6_LC | P | . | S | . | S | . | R | . | . | . | . | . | . | L | V | . | . |
| 21-225_29E6_LC | . | . | . | . | . | . | . | . | . | . | . | . | . | . | . | . | . |
| 21-225_26C4_LC | . | . | . | . | . | . | . | . | . | . | . | . | . | . | . | . | R |
| 21-225_33D1_LC | . | . | . | . | . | . | . | . | . | I | . | . | . | . | . | . | R |
| 21-225_34D2_LC | P | . | S | . | S | . | . | . | . | . | . | . | . | L | . | H | . |
| 21-225_29H8_LC | . | . | . | . | . | . | R | . | . | . | . | . | T | I | . | H | . |
| 21-225_11E5_LC | . | . | . | L | . | . | . | . | . | . | . | . | . | . | . | . | . |
| 21-225_8B11_LC | . | . | . | . | . | . | . | . | . | . | . | . | N | . | . | . | N |
| 21-225_22D2_LC | . | . | . | . | T | . | . | . | . | . | . | . | . | . | . | H | . |
| 21-225_32A1_LC | . | . | . | . | . | . | . | . | . | . | . | . | . | . | . | . | . |
| 21-225_32A5_LC | . | . | . | . | . | . | . | . | . | . | . | . | . | . | . | H | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | S | N | N | N | N | Y | L | A | W | Y | Q | Q | K | P | G | Q | P |
| 21-225_34H7_LC | . | . | . | Y | . | . | . | . | . | . | . | . | L | . | . | . | . |
| 21-225_24E1_LC | . | . | . | . | . | . | . | T | . | . | . | . | . | . | . | . | R |
| 21-225_23A12_LC | . | . | . | . | . | . | . | . | . | . | . | . | . | . | . | . | . |
| 21-225_25A4_LC | . | H | . | . | . | . | . | . | . | . | . | . | . | . | . | . | . |
| 21-225_28B6_LC | D | - | G | D | T | . | . | N | . | F | . | . | R | . | . | . | S |
| 21-225_29E6_LC | . | H | . | . | Y | . | . | T | . | . | . | . | . | . | . | . | . |
| 21-225_26C4_LC | . | . | . | Y | . | . | . | . | . | . | . | H | . | . | . | . | . |
| 21-225_33D1_LC | . | . | . | K | . | . | . | T | . | . | . | . | . | . | . | . | . |
| 21-225_34D2_LC | D | - | G | K | T | . | . | Y | . | . | L | . | . | . | . | . | . |
| 21-225_29H8_LC | . | . | . | Y | . | . | . | . | . | . | . | . | . | . | . | . | . |
| 21-225_11E5_LC | . | . | . | . | . | . | . | T | . | . | . | . | . | . | . | . | R |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | Y | . | . | . | . | . | . | . | . | L | . | . | . | . |
| 21-225_32A5_LC | . | . | . | K | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | K_CDR2 | | | | |
| CONSENSUS | P | K | L | L | I | Y | W | - | - | - | - | - | - | - | - | A | S |
| 21-225_34H7_LC | . | . | . | . | . | . | . | . | . | . | . | . | . | . | . | . | . |
| 21-225_24E1_LC | . | N | . | . | . | . | . | . | . | . | . | . | . | . | . | . | . |
| 21-225_23A12_LC | . | . | . | . | . | . | . | . | . | . | . | . | . | . | . | T | . |
| 21-225_25A4_LC | . | . | . | . | L | . | . | . | . | . | . | . | . | . | . | . | . |
| 21-225_28B6_LC | . | R | R | Q | . | . | K | . | . | . | . | . | . | . | . | V | . |
| 21-225_29E6_LC | H | . | . | . | L | . | . | . | . | . | . | . | . | . | . | . | . |
| 21-225_26C4_LC | R | . | . | . | . | . | . | . | . | . | . | . | . | . | . | . | . |
| 21-225_33D1_LC | R | . | . | F | . | . | . | . | . | . | . | . | . | . | . | . | . |
| 21-225_34D2_LC | . | Q | . | . | . | . | E | . | . | . | . | . | . | . | . | V | . |
| 21-225_29H8_LC | . | . | . | . | . | . | . | . | . | . | . | . | . | . | . | . | . |
| 21-225_11E5_LC | . | . | . | . | . | . | . | . | . | . | . | . | . | . | . | . | . |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | . | . | . | . | . | . | . | . | . | . | . | . | . | . |
| 21-225_32A5_LC | . | . | . | . | . | . | . | . | . | . | . | . | . | . | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | T | R | E | S | G | V | P | D | R | F | S | G | S | G | S | G | - |
| 21-225_34H7_LC | . | . | K | . | . | . | . | . | . | . | . | . | . | . | . | . | . |
| 21-225_24E1_LC | . | . | . | . | . | . | . | . | . | . | . | . | . | . | . | . | . |
| 21-225_23A12_LC | . | . | . | . | . | . | . | . | . | . | . | . | . | . | . | . | . |
| 21-225_25A4_LC | . | . | . | . | . | . | . | . | . | . | . | . | . | . | . | . | . |
| 21-225_28B6_LC | N | W | D | . | . | . | . | . | . | . | . | . | . | . | . | . | . |
| 21-225_29E6_LC | S | . | . | . | . | . | . | . | . | . | . | . | . | . | . | . | . |
| 21-225_26C4_LC | . | . | . | . | . | . | . | . | . | . | . | . | . | . | . | . | . |
| 21-225_33D1_LC | . | . | . | . | . | . | L | . | . | . | . | . | . | . | C | . | . |
| 21-225_34D2_LC | N | . | F | . | . | . | . | . | . | . | . | . | . | . | . | . | . |
| 21-225_29H8_LC | . | . | . | . | . | . | . | . | . | . | . | . | . | . | . | . | . |
| 21-225_11E5_LC | . | . | . | . | . | . | . | . | . | . | . | . | . | . | . | . | . |
| 21-225_8B11_LC | . | . | K | . | . | . | . | . | . | . | . | . | . | . | . | . | . |
| 21-225_22D2_LC | . | . | K | . | . | . | . | . | . | . | . | . | . | . | . | . | . |
| 21-225_32A1_LC | . | . | K | . | . | . | . | . | . | . | . | . | . | . | . | . | . |
| 21-225_32A5_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | | |
| CONSENSUS | - | T | D | F | T | L | T | I | S | S | L | Q | A | E | D | V | A |
| 21-225_34H7_LC | . | . | . | . | . | . | . | . | . | . | . | . | T | . | . | . | . |
| 21-225_24E1_LC | . | . | . | . | . | . | . | . | . | . | . | . | . | . | . | . | . |
| 21-225_23A12_LC | . | . | . | . | . | . | . | . | . | . | . | . | . | . | . | . | . |
| 21-225_25A4_LC | . | . | E | . | . | . | . | . | . | . | . | . | E | . | . | . | . |
| 21-225_28B6_LC | . | . | . | . | . | . | N | . | . | R | V | E | . | . | . | . | G |
| 21-225_29E6_LC | . | . | E | . | . | . | . | . | . | . | . | . | . | . | . | . | . |
| 21-225_26C4_LC | . | . | . | . | . | . | . | . | . | . | . | . | . | . | . | . | . |
| 21-225_33D1_LC | . | . | . | . | . | . | . | . | I | . | . | . | . | . | . | . | . |
| 21-225_34D2_LC | . | . | . | . | . | . | K | . | . | R | V | E | . | . | . | . | G |
| 21-225_29H8_LC | . | . | . | . | . | . | . | . | . | . | . | . | . | . | . | . | . |
| 21-225_11E5_LC | . | . | N | . | . | . | . | . | . | . | . | . | . | . | . | . | . |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | . | . | . | . | . | . | . | . | . | T | . | . | . | . |
| 21-225_32A5_LC | . | . | . | . | . | . | A | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | Q | Q | Y | Y | S | - | - | - | - | - | - | - | - |
| 21-225_34H7_LC | L | . | . | . | . | . | . | . | T | . | . | . | . | . | . | . | . |
| 21-225_24E1_LC | . | . | . | . | . | . | F | . | . | . | . | . | . | . | . | . | . |
| 21-225_23A12_LC | . | . | . | . | . | . | . | . | D | . | . | . | . | . | . | . | . |
| 21-225_25A4_LC | . | . | . | . | . | . | . | . | . | . | . | . | . | . | . | . | . |
| 21-225_28B6_LC | I | . | F | . | M | H | C | T | - | . | . | . | . | . | . | . | . |
| 21-225_29E6_LC | . | . | . | . | . | . | . | . | . | . | . | . | . | . | . | . | . |
| 21-225_26C4_LC | . | . | . | . | . | . | . | . | . | . | . | . | . | . | . | . | . |
| 21-225_33D1_LC | L | . | . | . | . | . | . | . | . | . | . | . | . | . | . | . | . |
| 21-225_34D2_LC | . | . | . | . | M | . | S | K | - | . | . | . | . | . | . | . | . |
| 21-225_29H8_LC | . | . | . | . | . | . | . | . | C | . | . | . | . | . | . | . | . |
| 21-225_11E5_LC | . | . | . | . | . | . | . | . | . | . | . | . | . | . | . | . | . |
| 21-225_8B11_LC | . | . | . | . | . | . | . | . | T | . | . | . | . | . | . | . | . |
| 21-225_22D2_LC | . | . | F | . | . | . | . | . | . | . | . | . | . | . | . | . | . |
| 21-225_32A1_LC | L | . | . | . | . | . | . | . | T | . | . | . | . | . | . | . | . |
| 21-225_32A5_LC | L | . | H | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | T | P |
| 21-225_34H7_LC | . | . | . | . | . | . | . | . | . | . | . | . | . | . | . | S | . |
| 21-225_24E1_LC | . | . | . | . | . | . | . | . | . | . | . | . | . | . | . | . | . |
| 21-225_23A12_LC | . | . | . | . | . | . | . | . | . | . | . | . | . | . | . | . | . |
| 21-225_25A4_LC | . | . | . | . | . | . | . | . | . | . | . | . | . | . | . | . | . |
| 21-225_28B6_LC | . | . | . | . | . | . | . | . | . | . | . | . | . | . | . | H | W |
| 21-225_29E6_LC | . | . | . | . | . | . | . | . | . | . | . | . | . | . | . | . | . |
| 21-225_26C4_LC | . | . | . | . | . | . | . | . | . | . | . | . | . | . | . | . | . |
| 21-225_33D1_LC | . | . | . | . | . | . | . | . | . | . | . | . | . | . | . | S | . |
| 21-225_34D2_LC | . | . | . | . | . | . | . | . | . | . | . | . | . | . | . | Q | L |
| 21-225_29H8_LC | . | . | . | . | . | . | . | . | . | . | . | . | . | . | . | . | . |
| 21-225_11E5_LC | . | . | . | . | . | . | . | . | . | . | . | . | . | . | . | . | . |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | . | . | . | . | . | . | . | . | . | . | . | . | S | . |
| 21-225_32A5_LC | . | . | . | . | . | . | . | . | . | . | . | . | . | . | . | S | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | K_FR4 | | | | | | |
| CONSENSUS | P | T | F | G | Q | G | T | K | V | E | I | K | R |
| 21-225_34H7_LC | C | S | . | . | . | . | . | . | L | . | . | . | . |
| 21-225_24E1_LC | . | . | . | . | . | . | . | . | . | . | . | . | . |
| 21-225_23A12_LC | . | . | . | . | . | . | . | . | . | . | . | . | . |
| 21-225_25A4_LC | . | . | . | . | . | . | . | . | . | . | . | . | . |
| 21-225_28B6_LC | L | L | . | . | P | . | . | . | . | D | . | . | . |
| 21-225_29E6_LC | . | . | . | . | . | . | . | . | . | . | . | . | . |
| 21-225_26C4_LC | . | . | . | . | . | . | . | . | . | . | F | . | . |
| 21-225_33D1_LC | C | S | . | . | . | . | . | . | L | . | . | . | . |
| 21-225_34D2_LC | . | P | . | . | G | . | . | . | . | . | . | R | . |
| 21-225_29H8_LC | . | . | . | . | . | . | . | . | . | . | . | . | . |
| 21-225_11E5_LC | . | . | . | . | . | . | . | . | . | . | . | . | . |
| 21-225_8B11_LC | C | S | . | . | . | . | . | . | L | . | . | . | . |
| 21-225_22D2_LC | C | S | . | . | . | . | . | . | L | . | . | . | . |
| 21-225_32A1_LC | C | S | . | . | . | . | . | . | L | . | . | . | . |
| 21-225_32A5_LC | C | S | . | . | . | . | . | G | L | . | . | . | . |

**FIGURE 56**

Table 38

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K FR1 | | | | | |
| CONSENSUS | D | I | Q | M | T | Q | S | P | S | S | L | S | A | S | V | G | D |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | . | . | . | . | . | . | . | . | . |
| 21-225_22B12_LC | . | . | . | . | . | . | . | . | . | . | . | . | . | . | . | . | . |
| 21-225_27C5_LC | . | . | . | . | . | . | . | . | . | . | V | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | F | . | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | F | . | F | I | . | . |
| 21-225_5E11_LC | V | . | . | . | . | . | . | . | . | . | . | . | . | . | . | . | . |
| 21-225_2A4_LC | . | . | . | . | . | . | . | . | . | . | . | F | . | . | . | . | . |
| 21-225_16B8_LC | . | . | . | . | . | . | . | . | . | . | . | . | . | . | . | . | . |
| 21-225_14D6_LC | . | . | . | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | . | . | . | . | . | . | . | . | . | . | . | . | . |
| 21-225_16H1_LC | . | . | . | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | . | T | . | . | . |
| 21-225_32B3_LC | . | . | . | . | . | . | . | . | . | . | . | . | . | . | I | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | S | - | - |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | S | . | . | . | S | . | . | . | . | . | . | . | D | . | R | . | . |
| 21-225_20H7_LC | . | . | . | . | . | . | . | T | . | . | . | . | . | . | N | . | . |
| 21-225_20D12_LC | . | . | . | . | . | . | . | . | . | . | . | . | D | . | R | . | . |
| 21-225_22B12_LC | . | . | . | . | I | . | . | . | . | . | . | . | D | . | . | . | . |
| 21-225_27C5_LC | . | . | . | . | . | . | . | . | . | . | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | A | . | . | . | . |
| 21-225_15F8_LC | . | . | . | . | . | . | . | . | . | . | . | R | D | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | N | . | . |
| 21-225_14D6_LC | S | . | . | . | . | . | . | . | . | . | . | . | D | . | R | . | . |
| 21-225_1F1_LC | . | . | . | . | . | . | . | . | . | . | . | . | . | . | N | . | . |
| 21-225_5F4_LC | . | . | . | . | . | . | . | . | . | . | . | . | . | . | R | . | . |
| 21-225_16H1_LC | . | . | . | . | . | . | . | . | . | . | . | . | . | . | R | . | . |
| 21-225_17H6_LC | . | . | . | . | . | . | . | . | . | . | . | R | D | . | . | . | . |
| 21-225_21F12_LC | . | . | . | . | . | . | . | . | . | . | . | . | . | . | R | . | . |
| 21-225_23A11_LC | . | . | . | . | . | . | . | . | . | . | . | . | D | . | . | . | . |
| 21-225_32B3_LC | . | . | . | . | . | . | . | . | . | . | . | . | . | . | . | . | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | K_FR2 | | | |
| CONSENSUS | - | - | - | - | N | Y | L | A | W | F | Q | Q | K | P | G | K | A |
| 21-225_2C12_LC | . | . | . | . | . | . | . | . | . | . | . | . | . | S | . | . | . |
| 21-225_17H5_LC | . | . | . | . | . | . | . | . | . | . | . | . | . | . | . | . | . |
| 21-225_16A11_LC | . | . | . | . | . | H | . | G | . | Y | . | R | N | . | . | . | . |
| 21-225_20H7_LC | . | . | . | . | . | . | . | V | . | . | . | . | . | . | . | . | . |
| 21-225_20D12_LC | . | . | . | . | . | H | . | G | . | Y | . | . | . | . | . | . | . |
| 21-225_22B12_LC | . | . | . | . | . | . | . | . | . | . | . | E | . | . | . | . | . |
| 21-225_27C5_LC | . | . | . | . | S | W | . | . | . | Y | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | . | . | . | H | . | G | . | Y | . | H | . | . | . | . | . |
| 21-225_1F1_LC | . | . | . | . | . | . | . | . | . | . | . | . | . | S | . | . | . |
| 21-225_5F4_LC | . | . | . | . | . | H | . | G | . | Y | . | . | . | . | . | . | . |
| 21-225_16H1_LC | . | . | . | . | D | I | . | G | . | Y | . | . | . | . | . | . | . |
| 21-225_17H6_LC | . | . | . | . | . | . | . | . | . | . | . | . | . | . | . | . | . |
| 21-225_21F12_LC | . | . | . | . | . | F | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | S | L | I | Y | A | - | - | - | - | - | - | - | - | A | S |
| 21-225_2C12_LC | . | . | . | . | . | S | . | . | . | . | . | . | . | . | . | . | T |
| 21-225_17H5_LC | . | . | . | . | . | N | . | . | . | . | . | . | . | . | . | . | . |
| 21-225_16A11_LC | . | . | R | . | . | . | . | . | . | . | . | . | . | . | . | . | . |
| 21-225_20H7_LC | . | . | R | . | . | . | . | . | . | . | . | . | . | . | . | . | . |
| 21-225_20D12_LC | . | . | R | . | . | . | . | . | . | . | . | . | . | . | . | . | . |
| 21-225_22B12_LC | H | . | . | . | . | . | G | . | . | . | . | . | . | . | . | . | . |
| 21-225_27C5_LC | . | . | F | . | . | . | G | . | . | . | . | . | . | . | . | . | . |
| 21-225_27H12_LC | . | . | . | . | . | N | G | . | . | . | . | . | . | . | . | . | . |
| 21-225_35H8_LC | . | . | . | . | . | . | G | . | . | . | . | . | . | . | . | . | . |
| 21-225_15F8_LC | . | . | . | . | . | C | S | . | . | . | . | . | . | . | . | T | . |
| 21-225_20D6_LC | . | . | . | . | . | S | . | . | . | . | . | . | . | . | . | . | . |
| 21-225_15A2_LC | . | . | . | . | . | F | . | . | . | . | . | . | . | . | . | . | . |
| 21-225_5E11_LC | . | . | . | . | . | N | . | . | . | . | . | . | . | . | . | . | . |
| 21-225_2A4_LC | . | . | R | . | . | . | T | . | . | . | . | . | . | . | . | . | . |
| 21-225_16B8_LC | L | . | . | . | . | N | . | . | . | . | . | . | . | . | . | . | . |
| 21-225_14D6_LC | . | . | R | . | . | . | . | . | . | . | . | . | . | . | . | . | . |
| 21-225_1F1_LC | . | . | . | . | . | S | V | . | . | . | . | . | . | . | . | . | . |
| 21-225_5F4_LC | . | . | R | . | . | . | . | . | . | . | . | . | . | . | . | . | . |
| 21-225_16H1_LC | . | . | R | . | . | . | . | . | . | . | . | . | . | . | . | . | . |
| 21-225_17H6_LC | . | . | . | . | . | S | T | . | . | . | . | . | . | . | . | . | . |
| 21-225_21F12_LC | . | . | . | . | . | S | . | . | . | . | . | . | . | . | . | . | . |
| 21-225_23A11_LC | . | . | R | . | . | . | . | . | . | . | . | . | . | . | . | . | . |
| 21-225_32B3_LC | . | R | . | . | . | . | . | . | . | . | . | . | . | . | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | K | F | S | G | S | G | S | G | - |
| 21-225_2C12_LC | . | . | . | . | . | . | . | . | Q | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | R | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | R | . | . | . | . | . | . | . | . |
| 21-225_22B12_LC | . | . | R | . | . | . | . | . | . | . | . | . | . | . | . | . | . |
| 21-225_27C5_LC | . | . | . | . | . | . | . | . | R | . | . | A | . | . | . | . | . |
| 21-225_27H12_LC | . | . | . | . | . | . | . | . | . | . | . | . | . | . | . | . | . |
| 21-225_35H8_LC | G | . | . | . | . | . | . | . | . | . | . | . | . | . | . | . | . |
| 21-225_15F8_LC | . | . | . | . | . | . | . | . | . | . | . | . | . | . | F | . | . |
| 21-225_20D6_LC | N | . | . | . | . | . | . | . | . | . | . | . | . | . | . | . | . |
| 21-225_15A2_LC | . | . | . | . | . | . | . | . | . | . | . | . | . | . | F | . | . |
| 21-225_5E11_LC | . | V | . | . | . | . | . | . | . | . | . | . | N | . | . | . | . |
| 21-225_2A4_LC | . | . | . | . | . | . | . | . | . | . | . | . | . | . | F | . | . |
| 21-225_16B8_LC | . | V | . | . | . | . | . | . | . | . | . | . | . | . | . | . | . |
| 21-225_14D6_LC | . | . | . | . | . | . | . | . | R | . | . | . | . | . | . | . | . |
| 21-225_1F1_LC | . | . | . | T | . | . | . | . | R | . | . | . | . | . | . | . | . |
| 21-225_5F4_LC | . | . | . | . | . | . | . | . | R | . | . | . | . | . | . | . | . |
| 21-225_16H1_LC | . | . | . | N | . | . | . | . | R | . | . | . | . | . | . | . | . |
| 21-225_17H6_LC | . | . | . | . | . | . | . | . | . | . | . | . | . | . | . | . | . |
| 21-225_21F12_LC | . | . | . | T | . | . | . | . | . | . | . | . | . | . | . | . | . |
| 21-225_23A11_LC | T | . | . | . | . | . | . | . | . | . | . | . | . | . | . | . | . |
| 21-225_32B3_LC | . | . | . | . | . | . | . | . | T | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | | |
| CONSENSUS | - | T | D | F | T | L | T | I | S | S | L | Q | P | E | D | F | A |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | E | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | E | . | . | . | . | . | . | . | . | . | . | . | . | . | . |
| 21-225_22B12_LC | . | . | . | . | N | . | . | . | . | . | . | . | . | . | . | L | . |
| 21-225_27C5_LC | . | . | E | . | . | . | . | . | . | . | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | . | . |
| 21-225_15A2_LC | . | . | . | . | . | F | P | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | E | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | E | . | . | . | . | V | . | . | . | . | . | . | . | . | . |
| 21-225_16H1_LC | . | . | E | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | Y | N | S | - | - | - | - | - | - | - | - |
| 21-225_2C12_LC | . | . | . | . | . | . | . | Y | . | . | . | . | . | . | . | . | . |
| 21-225_17H5_LC | . | . | . | . | . | . | . | H | . | . | . | . | . | . | . | . | . |
| 21-225_16A11_LC | . | . | . | . | L | . | . | . | . | . | . | . | . | . | . | . | . |
| 21-225_20H7_LC | . | . | . | . | . | . | . | Y | . | . | . | . | . | . | . | . | . |
| 21-225_20D12_LC | . | . | . | . | L | . | . | . | . | . | . | . | . | . | . | . | . |
| 21-225_22B12_LC | . | . | . | . | . | . | . | H | . | . | . | . | . | . | . | . | . |
| 21-225_27C5_LC | . | . | C | . | . | . | . | D | . | . | . | . | . | . | . | . | . |
| 21-225_27H12_LC | . | . | . | . | . | . | . | Y | . | . | . | . | . | . | . | . | . |
| 21-225_35H8_LC | . | . | . | . | . | . | . | . | . | . | . | . | . | . | . | . | . |
| 21-225_15F8_LC | A | . | . | . | . | . | . | H | . | . | . | . | . | . | . | . | . |
| 21-225_20D6_LC | . | . | F | . | . | . | . | H | . | . | . | . | . | . | . | . | . |
| 21-225_15A2_LC | N | . | . | . | . | . | . | Y | . | . | . | . | . | . | . | . | . |
| 21-225_5E11_LC | . | . | . | . | H | . | . | H | . | . | . | . | . | . | . | . | . |
| 21-225_2A4_LC | . | . | . | . | . | . | . | . | . | . | . | . | . | . | . | . | . |
| 21-225_16B8_LC | . | . | . | . | H | . | . | H | . | . | . | . | . | . | . | . | . |
| 21-225_14D6_LC | . | . | . | . | L | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | L | . | . | . | G | . | . | . | . | . | . | . | . |
| 21-225_16H1_LC | . | . | . | . | L | . | . | H | . | . | . | . | . | . | . | . | . |
| 21-225_17H6_LC | I | . | . | . | . | . | . | H | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | . | . | . | . | . | . | . | . | . |
| 21-225_22B12_LC | . | . | . | . | . | . | . | . | . | . | . | . | . | . | . | . | . |
| 21-225_27C5_LC | . | . | . | . | . | . | . | . | . | . | . | . | . | . | . | F | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | F | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | . | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | . | . | . | . | . | . | . | . | . | . | . | . | . |
| 21-225_16H1_LC | . | . | . | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | F | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

**FIGURE 56**

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | | K_FR4 | | | | | |
| CONSENSUS | F | T | F | G | P | G | T | K | V | D | I | K | R |
| 21-225_2C12_LC | I | . | . | . | Q | . | . | R | L | E | . | . | . |
| 21-225_17H5_LC | . | . | . | . | . | . | . | . | . | . | . | . | . |
| 21-225_16A11_LC | . | . | . | . | . | . | . | . | . | . | . | . | . |
| 21-225_20H7_LC | . | . | . | . | . | . | . | . | M | . | F | . | . |
| 21-225_20D12_LC | . | . | . | . | . | . | . | . | . | . | . | . | . |
| 21-225_22B12_LC | . | . | . | . | . | . | . | . | . | . | F | . | . |
| 21-225_27C5_LC | R | . | . | . | Q | . | . | . | . | E | . | . | . |
| 21-225_27H12_LC | . | . | . | . | . | . | . | . | . | N | . | N | . |
| 21-225_35H8_LC | . | . | . | . | . | . | . | . | . | . | F | . | . |
| 21-225_15F8_LC | . | . | . | . | R | . | . | . | . | . | . | Q | . |
| 21-225_20D6_LC | . | . | . | . | . | . | . | . | . | . | . | . | . |
| 21-225_15A2_LC | . | . | . | . | . | . | . | Q | . | . | V | . | . |
| 21-225_5E11_LC | . | . | . | . | . | . | . | . | . | . | . | . | . |
| 21-225_2A4_LC | . | . | . | . | . | . | . | . | . | . | . | . | . |
| 21-225_16B8_LC | . | . | . | . | . | . | . | . | . | . | . | V | . |
| 21-225_14D6_LC | . | . | . | . | . | . | . | . | . | . | . | N | . |
| 21-225_1F1_LC | L | . | . | . | G | . | . | . | . | E | . | . | . |
| 21-225_5F4_LC | . | . | . | . | . | . | . | . | . | . | . | . | . |
| 21-225_16H1_LC | . | . | . | . | . | . | . | . | . | . | V | . | . |
| 21-225_17H6_LC | . | . | . | . | . | . | . | . | . | . | . | Q | . |
| 21-225_21F12_LC | . | . | . | . | . | . | . | . | . | . | . | . | . |
| 21-225_23A11_LC | . | . | . | . | . | . | . | . | . | . | L | . | . |
| 21-225_32B3_LC | . | . | . | . | . | . | . | . | . | . | . | . | . |

**FIGURE 56**

Table 39

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | K_FR1 | | | | | | |
| CONSENSUS | D | I | Q | M | T | Q | S | P | S | S | L | S | A | S | V | G | D |
| 21-225_17C10_LC | . | . | . | . | . | . | . | . | . | . | Q | . | . | . | . | . | . |
| 21-225_20B5_LC | . | . | . | . | . | . | . | . | . | . | . | . | . | . | . | . | . |
| 21-225_17D8_LC | E | . | V | . | . | . | . | . | . | . | T | . | V | . | P | . | E |
| 21-225_20B8_LC | E | . | V | . | . | . | . | . | . | . | T | . | V | . | P | . | E |
| 21-225_22H3_LC | . | . | . | . | . | . | . | . | . | . | . | . | . | . | . | . | . |
| 21-225_20C8_LC | . | . | . | . | . | . | . | . | . | . | . | . | . | . | . | . | . |
| 21-225_20G12_LC | . | . | . | . | . | . | . | . | . | . | . | . | . | . | . | . | . |
| 21-225_23E11_LC | . | . | . | . | . | . | . | . | . | . | . | . | . | . | . | . | . |
| 21-225_20F9_LC | . | . | . | . | . | . | . | . | . | . | . | . | . | . | . | . | . |
| 21-225_20H10_LC | G | . | . | . | . | . | . | . | . | . | . | . | . | . | . | . | . |
| 21-225_31G9_LC | . | . | . | . | . | . | . | . | . | . | . | . | . | . | I | . | . |
| 21-225_30H11_LC | . | . | . | . | . | . | . | . | . | . | . | . | . | . | . | . | . |
| 21-225_33C2_LC | . | . | . | . | . | . | . | . | . | . | . | . | . | . | . | . | . |
| 21-225_14B1_LC | . | . | . | . | . | . | . | . | . | . | Y | . | . | . | . | . | . |
| 21-225_26B11_LC | . | . | . | . | . | . | . | . | . | . | . | . | . | . | . | . | . |
| 21-225_13D3_LC | E | . | V | . | . | . | . | . | . | A | T | . | V | . | P | . | E |
| 21-225_6C12_LC | . | . | . | . | . | . | . | . | . | . | . | . | . | . | . | . | . |

| Linear # | 1 | 2 | 3 | 4 | 5 | 6 | 7 | 7.1 | 8 | 9 | 10 | 11 | 12 | 13 | 14 | 15 | 16 |
|---|---|---|---|---|---|---|---|---|---|---|---|---|---|---|---|---|---|
| | | | | | | | | | | | L_FR1 | | | | | | |
| 21-225_14B1_LC | S | F | E | L | T | Q | P | - | P | S | V | S | V | S | P | G | Q |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | R | - | - |
| 21-225_17C10_LC | . | . | . | . | . | . | . | . | . | . | . | . | . | . | S | . | . |
| 21-225_20B5_LC | . | . | . | . | . | . | . | . | . | . | . | . | . | . | N | . | . |
| 21-225_17D8_LC | . | A | . | L | S | . | . | . | . | . | . | . | S | V | S | . | . |
| 21-225_20B8_LC | . | . | . | L | S | . | . | . | . | . | . | . | S | V | S | . | . |
| 21-225_22H3_LC | . | . | . | . | . | . | . | . | . | . | . | . | D | . | . | . | . |
| 21-225_20C8_LC | . | . | . | . | . | . | . | . | . | . | . | . | . | . | . | . | . |
| 21-225_20G12_LC | . | . | . | . | . | . | . | . | . | . | . | . | . | . | S | . | . |
| 21-225_23E11_LC | . | I | . | . | . | . | . | . | . | . | . | . | . | . | . | . | . |
| 21-225_20F9_LC | . | I | . | . | . | . | . | . | . | . | . | . | . | . | S | . | . |
| 21-225_20H10_LC | . | . | . | . | . | . | . | . | . | . | . | . | . | . | K | . | . |
| 21-225_31G9_LC | . | . | . | . | . | . | . | . | . | . | . | . | . | . | . | . | . |
| 21-225_30H11_LC | . | . | . | . | . | . | . | . | . | . | . | . | . | . | . | . | . |
| 21-225_33C2_LC | . | . | . | . | . | . | . | . | . | . | . | . | . | . | . | . | . |
| 21-225_14B1_LC | . | . | . | . | . | . | . | . | . | . | . | . | D | . | . | . | . |
| 21-225_26B11_LC | . | . | . | . | . | . | . | . | . | . | . | . | D | . | . | . | . |
| 21-225_13D3_LC | S | A | . | L | S | . | . | . | . | . | . | . | T | V | S | . | . |
| 21-225_6C12_LC | . | G | . | . | . | . | . | . | . | . | . | . | . | . | . | . | . |

| Linear # | 17 | 18 | 19 | 20 | 21 | 22 | 23 | 24 | 25 | 25.1 | 25.2 | 25.3 | 26 | 27 | 28 | 29 | 29.1 |
|---|---|---|---|---|---|---|---|---|---|---|---|---|---|---|---|---|---|
| | | | | | | | | | | | | | | | L_CDR1 | | |
| 21-225_14B1_LC | T | A | S | I | T | C | S | G | D | - | - | - | K | L | G | D | - |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | W | Y | Q | Q | K | P | G | K_FR2 | |
| CONSENSUS | - | - | - | - | N | N | L | G | W | Y | Q | Q | K | P | G | K | A |
| 21-225_17C10_LC | . | . | . | . | . | Y | . | A | . | . | . | . | . | . | . | R | V |
| 21-225_20B5_LC | . | . | . | . | . | Y | . | A | . | . | . | . | . | . | . | . | I |
| 21-225_17D8_LC | . | . | . | . | S | . | . | A | . | F | . | . | . | . | . | Q | . |
| 21-225_20B8_LC | . | . | . | . | S | . | . | A | . | F | . | . | . | . | . | Q | . |
| 21-225_22H3_LC | . | . | . | . | . | . | . | . | . | . | . | . | . | . | . | . | . |
| 21-225_20C8_LC | . | . | . | . | . | D | . | . | . | . | . | K | . | . | . | . | . |
| 21-225_20G12_LC | . | . | . | . | . | Y | . | A | . | . | . | . | . | . | . | . | V |
| 21-225_23E11_LC | . | . | . | . | . | D | . | . | . | . | . | . | . | . | . | . | . |
| 21-225_20F9_LC | . | . | . | . | . | Y | . | A | . | F | . | . | . | . | . | . | . |
| 21-225_20H10_LC | . | . | . | . | . | . | . | . | . | . | . | . | . | . | . | . | G |
| 21-225_31G9_LC | . | . | . | . | . | D | . | . | . | . | . | . | . | . | . | . | . |
| 21-225_30H11_LC | . | . | . | . | . | D | . | . | . | . | . | . | . | . | . | . | . |
| 21-225_33C2_LC | . | . | . | . | . | D | . | . | . | . | . | . | . | . | V | . | . |
| 21-225_14B1_LC | . | . | . | . | D | . | . | . | . | . | . | . | . | . | . | . | . |
| 21-225_26B11_LC | . | . | . | . | D | D | . | . | . | . | . | . | . | . | . | . | . |
| 21-225_13D3_LC | . | . | . | . | S | . | . | A | . | F | . | . | . | . | . | Q | . |
| 21-225_6C12_LC | . | . | . | . | S | . | . | . | . | . | . | . | . | . | . | . | . |

| Linear # | 29.2 | 29.3 | 29.4 | 29.5 | 30 | 31 | 32 | 33 | 34 | 35 | 36 | 37 | 38 | 39 | 40 | 41 | 42 |
|---|---|---|---|---|---|---|---|---|---|---|---|---|---|---|---|---|---|
| | | | | | | | | | W | Y | Q | Q | K | P | G | L_FR2 | |
| 21-225_14B1_LC | - | - | - | - | K | Y | A | Y | W | Y | Q | Q | K | P | G | Q | S |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | R | L | I | Y | A | - | - | - | - | - | - | - | - | A | S |
| 21-225_17C10_LC | . | . | L | . | . | . | . | . | . | . | . | . | . | . | . | . | . |
| 21-225_20B5_LC | . | . | L | . | . | . | . | . | . | . | . | . | . | . | . | . | . |
| 21-225_17D8_LC | . | R | L | . | . | N | G | . | . | . | . | . | . | . | . | . | . |
| 21-225_20B8_LC | . | R | L | . | . | N | G | . | . | . | . | . | . | . | . | . | . |
| 21-225_22H3_LC | . | . | . | . | . | . | P | . | . | . | . | . | . | . | . | . | . |
| 21-225_20C8_LC | . | . | . | . | . | N | . | . | . | . | . | . | . | . | . | . | . |
| 21-225_20G12_LC | . | . | V | . | . | . | T | . | . | . | . | . | . | . | . | . | . |
| 21-225_23E11_LC | . | . | . | . | . | . | P | . | . | . | . | . | . | . | . | . | . |
| 21-225_20F9_LC | . | . | S | . | . | S | . | . | . | . | . | . | . | . | . | . | . |
| 21-225_20H10_LC | . | . | C | . | . | . | . | . | . | . | . | . | . | . | . | . | . |
| 21-225_31G9_LC | . | . | . | . | . | . | P | . | . | . | . | . | . | . | . | . | . |
| 21-225_30H11_LC | . | . | . | . | V | . | P | . | . | . | . | . | . | . | . | . | . |
| 21-225_33C2_LC | . | . | . | . | . | . | . | . | . | . | . | . | . | . | . | . | P |
| 21-225_14B1_LC | . | . | . | . | . | . | T | . | . | . | . | . | . | . | . | . | Y |
| 21-225_26B11_LC | . | R | . | . | . | . | P | . | . | . | . | . | . | . | . | . | . |
| 21-225_13D3_LC | . | R | L | . | . | N | G | . | . | . | . | . | . | . | . | . | . |
| 21-225_6C12_LC | . | . | . | . | . | . | . | . | . | . | . | . | . | . | . | . | . |

| Linear # | 43 | 44 | 45 | 46 | 47 | 48 | 49 | 49.1 | 49.2 | 49.3 | 49.4 | 49.5 | 49.6 | 49.7 | 49.8 | 50 | 51 |
|---|---|---|---|---|---|---|---|---|---|---|---|---|---|---|---|---|---|
| | | | | | | | | | | | | | | | L_CDR2 | | |
| 21-225_14B1_LC | P | V | L | V | I | Y | Q | - | - | - | - | - | - | - | - | D | R |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_17C10_LC | T | . | . | . | . | . | . | . | . | . | . | . | . | . | . | . | . |
| 21-225_20B5_LC | T | . | . | . | . | . | . | . | . | . | . | . | . | . | . | . | . |
| 21-225_17D8_LC | T | R | A | . | . | I | . | A | . | . | . | . | . | . | . | . | . |
| 21-225_20B8_LC | T | R | A | . | . | I | . | A | . | . | . | . | . | . | . | . | . |
| 21-225_22H3_LC | . | . | . | T | . | . | . | . | . | . | . | . | . | . | . | . | . |
| 21-225_20C8_LC | . | . | . | . | . | . | . | . | . | . | . | . | . | . | . | . | . |
| 21-225_20G12_LC | T | . | . | . | . | . | . | . | . | . | . | . | . | . | . | . | . |
| 21-225_23E11_LC | . | . | . | . | . | . | . | . | . | . | . | . | . | . | . | . | . |
| 21-225_20F9_LC | . | . | . | . | . | . | . | . | K | . | . | . | . | . | . | . | . |
| 21-225_20H10_LC | . | . | . | . | . | . | . | . | . | . | . | . | . | . | . | . | . |
| 21-225_31G9_LC | . | . | . | . | . | . | . | . | . | . | . | . | . | . | . | . | . |
| 21-225_30H11_LC | . | . | . | . | . | . | . | . | . | . | . | . | . | . | . | . | . |
| 21-225_33C2_LC | . | . | . | . | . | . | . | . | . | . | . | . | . | . | . | . | . |
| 21-225_14B1_LC | . | . | . | . | . | . | . | . | . | . | . | . | . | . | . | . | . |
| 21-225_26B11_LC | . | . | . | . | . | . | . | . | . | . | . | . | . | . | . | . | . |
| 21-225_13D3_LC | T | R | A | T | . | I | . | A | . | . | . | . | . | . | . | . | . |
| 21-225_6C12_LC | . | . | E | . | . | . | . | . | . | . | . | . | . | . | . | . | . |

| Linear # | 52 | 53 | 54 | 55 | 56 | 57 | 58 | 59 | 60 | 61 | 62 | 63 | 64 | 65 | 66 | 67 | 67.1 |
|---|---|---|---|---|---|---|---|---|---|---|---|---|---|---|---|---|---|
| 21-225_14B1_LC | K | R | P | S | G | I | P | E | R | F | S | G | S | N | S | G | - |

**FIGURE 56**

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | K_FR3 | | | | | | | | | | | | | | |
| CONSENSUS | . | T | E | F | T | L | T | I | S | S | L | Q | P | E | D | F | A |
| 21-225_17C10_LC | . | . | D | . | . | . | . | . | . | . | . | . | . | . | D | . | V | . |
| 21-225_20B5_LC | . | . | D | . | . | . | . | . | . | . | . | . | . | . | . | V | . |
| 21-225_17D8_LC | . | . | . | . | . | . | . | . | . | . | . | . | S | . | . | . | . |
| 21-225_20B8_LC | . | . | . | . | . | . | . | . | . | . | . | . | S | . | . | . | . |
| 21-225_22H3_LC | . | . | . | . | . | . | . | . | . | . | . | . | . | . | . | . | . |
| 21-225_20C8_LC | . | . | . | . | . | . | . | . | . | . | . | . | . | . | . | . | . |
| 21-225_20G12_LC | . | . | D | . | . | . | . | V | . | . | . | . | . | . | . | V | . |
| 21-225_23E11_LC | . | . | . | . | . | . | . | . | . | . | . | . | . | . | . | . | . |
| 21-225_20F9_LC | . | . | D | . | . | . | . | . | . | . | . | . | . | . | . | . | . |
| 21-225_20H10_LC | . | . | . | . | . | . | . | . | . | . | . | . | . | . | . | . | . |
| 21-225_31G9_LC | . | . | . | . | . | . | . | . | . | . | . | . | . | . | . | . | . |
| 21-225_30H11_LC | . | . | . | . | . | . | . | . | . | . | . | . | . | . | . | . | . |
| 21-225_33C2_LC | . | . | . | . | . | . | . | . | . | . | . | . | . | . | H | . | . |
| 21-225_14B1_LC | . | . | . | . | . | . | . | . | . | . | . | . | . | . | . | . | . |
| 21-225_26B11_LC | . | . | . | . | . | . | . | . | . | . | . | . | . | . | . | . | . |
| 21-225_13D3_LC | . | . | . | . | . | . | . | . | . | . | . | . | S | . | . | . | . |
| 21-225_6C12_LC | . | . | . | . | . | . | . | . | . | . | . | . | . | . | . | . | . |

| Linear # | 67/2 | 68 | 69 | 70 | 71 | 72 | 73 | 74 | 75 | 76 | 77 | 78 | 79 | 80 | 81 | 82 | 83 |
|---|---|---|---|---|---|---|---|---|---|---|---|---|---|---|---|---|---|
| | | | L_FR3 | | | | | | | | | | | | | | |
| 21-225_14B1_LC | - | N | T | A | T | L | T | I | S | G | T | Q | A | M | D | E | A |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | Y | N | S | - | - | - | - | - | - | - | - |
| 21-225_17C10_LC | . | . | . | . | Q | K | . | . | . | . | . | . | . | . | . | . | . |
| 21-225_20B5_LC | . | . | C | . | Q | K | . | . | . | . | . | . | . | . | . | . | . |
| 21-225_17D8_LC | V | . | . | . | Q | . | . | . | N | . | . | . | . | . | . | . | . |
| 21-225_20B8_LC | V | . | . | . | Q | . | . | . | N | . | . | . | . | . | . | . | . |
| 21-225_22H3_LC | . | . | F | . | . | . | D | . | . | . | . | . | . | . | . | . | . |
| 21-225_20C8_LC | . | . | . | . | . | . | D | . | . | . | . | . | . | . | . | . | . |
| 21-225_20G12_LC | . | . | . | . | Q | K | . | . | . | . | . | . | . | . | . | . | . |
| 21-225_23E11_LC | . | . | . | . | . | . | H | . | . | . | . | . | . | . | . | . | . |
| 21-225_20F9_LC | . | . | C | . | Q | . | . | H | . | . | . | . | . | . | . | . | . |
| 21-225_20H10_LC | . | . | . | . | . | . | H | . | . | . | . | . | . | . | . | . | . |
| 21-225_31G9_LC | . | . | . | . | . | . | D | . | . | . | . | . | . | . | . | . | . |
| 21-225_30H11_LC | . | . | . | . | . | . | D | . | . | . | . | . | . | . | . | . | . |
| 21-225_33C2_LC | . | . | . | . | . | . | H | . | . | . | . | . | . | . | . | . | . |
| 21-225_14B1_LC | . | . | . | . | . | . | H | Y | N | . | . | . | . | . | . | . | . |
| 21-225_26B11_LC | . | . | . | . | . | . | D | . | . | . | . | . | . | . | . | . | . |
| 21-225_13D3_LC | V | . | F | . | Q | . | . | H | D | . | . | . | . | . | . | . | . |
| 21-225_6C12_LC | . | . | F | . | . | . | D | . | . | . | . | . | . | . | . | . | . |

| Linear # | 84 | 85 | 86 | 87 | 88 | 89 | 90 | 91 | 92 | 92.1 | 92.2 | 92.3 | 92.4 | 92.5 | 92.6 | 92.7 | 92.8 |
|---|---|---|---|---|---|---|---|---|---|---|---|---|---|---|---|---|---|
| 21-225_14B1_LC | D | Y | Y | C | Q | A | W | D | N | - | - | - | - | - | - | - | - |

**FIGURE 56**

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_17C10_LC | . | . | . | . | . | . | . | . | . | . | . | . | . | . | A | P | . |
| 21-225_20B5_LC | . | . | . | . | . | . | . | . | . | . | . | . | . | . | A | P | . |
| 21-225_17D8_LC | . | . | . | . | . | . | . | . | . | . | . | . | . | . | . | W | . |
| 21-225_20B8_LC | . | . | . | . | . | . | . | . | . | . | . | . | . | . | . | C | . |
| 21-225_22H3_LC | . | . | . | . | . | . | . | . | . | . | . | . | . | . | . | . | . |
| 21-225_20C8_LC | . | . | . | . | . | . | . | . | . | . | . | . | . | . | . | . | . |
| 21-225_20G12_LC | . | . | . | . | . | . | . | . | . | . | . | . | . | . | A | P | . |
| 21-225_23E11_LC | . | . | . | . | . | . | . | . | . | . | . | . | . | . | . | . | . |
| 21-225_20F9_LC | . | . | . | . | . | . | . | . | . | . | . | . | . | . | . | . | . |
| 21-225_20H10_LC | . | . | . | . | . | . | . | . | . | . | . | . | . | . | . | . | . |
| 21-225_31G9_LC | . | . | . | . | . | . | . | . | . | . | . | . | . | . | . | . | . |
| 21-225_30H11_LC | . | . | . | . | . | . | . | . | . | . | . | . | . | . | . | H | . |
| 21-225_33C2_LC | . | . | . | . | . | . | . | . | . | . | . | . | . | . | . | . | . |
| 21-225_14B1_LC | . | . | . | . | . | . | . | . | . | . | . | . | . | . | . | . | . |
| 21-225_26B11_LC | . | . | . | . | . | . | . | . | . | . | . | . | . | . | . | . | . |
| 21-225_13D3_LC | . | . | . | . | . | . | . | . | . | . | . | . | . | . | . | W | . |
| 21-225_6C12_LC | . | . | . | . | . | . | . | . | . | . | . | . | . | . | . | . | . |

| Linear # | 92.9 | 92.10 | 92.11 | 92.12 | 92.13 | 92.14 | 92.15 | 92.16 | 92.17 | 92.18 | 92.19 | 92.20 | 92.21 | 92.22 | 92.23 | 93 | 94 |
|---|---|---|---|---|---|---|---|---|---|---|---|---|---|---|---|---|---|
| | | | L_CDR3 | | | | | | | | | | | | | | |
| 21-225_14B1_LC | - | - | - | - | - | - | - | - | - | - | - | - | - | - | - | S | T |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | | K_FR4 | | | | | |
| CONSENSUS | F | T | F | G | Q | G | T | K | V | E | I | K | R |
| 21-225_17C10_LC | L | . | . | . | . | . | . | R | L | . | . | . | . |
| 21-225_20B5_LC | I | . | . | . | . | . | . | R | L | . | . | . | . |
| 21-225_17D8_LC | L | . | . | . | . | . | . | . | . | . | . | . | . |
| 21-225_20B8_LC | L | . | . | . | . | . | . | . | . | . | . | . | . |
| 21-225_22H3_LC | . | . | . | . | P | . | . | . | . | D | . | . | . |
| 21-225_20C8_LC | . | . | . | . | G | . | . | . | . | . | . | T | . |
| 21-225_20G12_LC | I | . | . | . | . | . | . | R | L | . | . | . | . |
| 21-225_23E11_LC | W | . | . | . | . | . | . | . | . | . | . | . | . |
| 21-225_20F9_LC | W | . | . | . | . | . | . | . | . | . | . | . | . |
| 21-225_20H10_LC | L | . | . | . | G | . | . | . | . | . | . | . | . |
| 21-225_31G9_LC | . | . | . | . | P | . | . | . | . | D | . | . | . |
| 21-225_30H11_LC | . | . | . | . | P | . | . | . | . | D | . | T | . |
| 21-225_33C2_LC | W | . | . | . | . | . | . | . | . | . | . | . | . |
| 21-225_14B1_LC | L | . | . | . | G | . | . | . | . | . | . | . | . |
| 21-225_26B11_LC | . | . | . | . | P | . | . | . | . | D | . | . | . |
| 21-225_13D3_LC | P | . | . | . | . | . | . | . | . | . | . | . | . |
| 21-225_6C12_LC | . | . | . | . | G | . | . | . | . | . | . | . | . |

| Linear # | 95 | 96 | 97 | 98 | 99 | 100 | 101 | 102 | 103 | 104 | 105 | 106 | 107 |
|---|---|---|---|---|---|---|---|---|---|---|---|---|---|
| | | | | | | | L_FR4 | | | | | | |
| 21-225_14B1_LC | V | V | F | G | G | G | T | K | L | T | V | L | G |

**FIGURE 56**

Table 40

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | K_FR1 | | | | |
| CONSENSUS | D | I | Q | M | T | Q | S | P | S | S | L | S | A | S | V | G | D |
| 21-225_23C7_LC | . | . | . | . | . | . | . | . | . | . | . | . | . | . | . | . | . |
| 21-225_23E7_LC | . | . | . | . | . | . | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | G | . | . | . | . | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | . | . | . | . | . | . | . | . | . | . | . | . | . | . | . | . | . |
| 21-225_18F7_LC | . | . | . | . | . | . | . | . | . | . | . | . | . | . | E | . | . |
| 21-225_20G11_LC | . | . | . | . | . | . | . | . | . | . | . | . | . | . | E | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | S | - | - |
| 21-225_23C7_LC | . | . | . | . | . | . | . | . | . | . | . | . | . | . | G | . | . |
| 21-225_23E7_LC | . | . | . | . | . | . | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | . | . | . | . | . | . | . | . | . | N | . | . |
| 21-225_31G1_LC | . | . | . | . | . | . | . | . | . | . | . | . | . | . | N | . | . |
| 21-225_10C5_LC | . | . | . | . | . | . | . | . | . | . | . | . | . | . | . | . | . |
| 21-225_18F7_LC | . | . | . | . | . | . | . | . | . | . | . | . | D | . | . | . | . |
| 21-225_20G11_LC | . | . | . | . | . | . | . | . | . | . | . | . | D | . | . | . | . |

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | Y | L | A | W | F | Q | Q | K | P | G | K | A |
| 21-225_23C7_LC | . | . | . | . | I | . | . | . | . | . | . | . | . | . | . | . | . |
| 21-225_23E7_LC | . | . | . | . | . | . | . | . | . | . | . | . | I | . | . | . | . |
| 21-225_30A1_LC | . | . | . | . | . | . | . | . | . | . | . | . | . | . | . | . | . |
| 21-225_25D10_LC | . | . | . | . | H | . | . | . | . | . | . | . | R | . | . | . | . |
| 21-225_33A5_LC | . | . | . | . | . | . | . | . | . | . | . | . | . | . | . | . | . |
| 21-225_26C2_LC | . | . | . | . | H | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | . | . | . | . | . | . | . | . | . | . | . | . | R | S | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | K_CDR2 | | | | |
| CONSENSUS | P | K | S | L | I | S | A | - | - | - | - | - | - | - | - | A | S |
| 21-225_23C7_LC | . | . | . | . | . | . | . | . | . | . | . | . | . | . | . | . | . |
| 21-225_23E7_LC | . | . | . | . | . | Y | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | Y | . | . | . | . | . | . | . | . | . | . | . |
| 21-225_31G1_LC | . | . | . | . | . | . | S | . | . | . | . | . | . | . | . | . | . |
| 21-225_10C5_LC | . | R | . | . | . | . | . | . | . | . | . | . | . | . | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | K | F | S | G | S | G | S | G | - |
| 21-225_23C7_LC | . | . | . | . | . | . | . | . | . | . | . | . | . | . | . | . | . |
| 21-225_23E7_LC | . | . | . | . | . | . | . | . | . | . | . | . | . | . | . | . | . |
| 21-225_30A1_LC | . | . | . | T | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | F | . | . | . | . | . | . | . | . | . | . | G | . | . | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | |
| CONSENSUS | - | T | D | F | T | L | T | I | S | S | L | Q | P | E | D | F | A |
| 21-225_23C7_LC | . | . | E | . | . | . | . | . | . | . | . | . | . | . | . | . | . |
| 21-225_23E7_LC | . | . | . | . | . | . | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | S | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | . | . | . | . | . | . | . | . | . | . | . | . | A | . | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

# FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | Y | N | S | - | - | - | - | - | - | - | - |
| 21-225_23C7_LC | . | . | . | . | . | . | . | . | . | . | . | . | . | . | . | . | . |
| 21-225_23E7_LC | . | . | . | . | . | . | F | Y | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | H | T | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | H | . | . | . | . | . | . | . | . | . |
| 21-225_26D9_LC | . | . | . | . | . | . | F | . | . | . | . | . | . | . | . | . | . |
| 21-225_31G1_LC | . | . | F | . | . | . | . | . | . | . | . | . | . | . | . | . | . |
| 21-225_10C5_LC | . | . | . | . | . | . | . | Y | . | . | . | . | . | . | . | . | . |
| 21-225_18F7_LC | . | . | . | . | . | . | . | H | . | . | . | . | . | . | . | . | . |
| 21-225_20G11_LC | . | . | . | . | . | . | . | H | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_23C7_LC | . | . | . | . | . | . | . | . | . | . | . | . | . | . | . | . | . |
| 21-225_23E7_LC | . | . | . | . | . | . | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | . | . | . | . | . | . | . | . | . | . | . | . | . | . | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

| Reference # | 137 | 138 | 139 | 140 | 141 | 142 | 143 | 144 | 145 | 146 | 147 | 148 | 149 | Reference # |
|---|---|---|---|---|---|---|---|---|---|---|---|---|---|---|
| | | | | | | | | K_FR4 | | | | | | |
| CONSENSUS | F | T | F | G | P | G | T | K | V | D | I | K | R | |
| 21-225_23C7_LC | . | . | . | . | . | . | . | . | . | . | . | . | . | |
| 21-225_23E7_LC | . | . | . | . | . | . | . | . | . | . | . | N | . | |
| 21-225_30A1_LC | . | . | . | . | . | . | . | . | . | . | . | . | . | |
| 21-225_25D10_LC | . | . | . | . | . | . | . | . | . | . | . | . | . | |
| 21-225_33A5_LC | . | . | . | . | . | . | . | N | . | . | . | . | . | |
| 21-225_26C2_LC | . | . | . | . | . | . | . | . | . | . | . | . | . | |
| 21-225_26D9_LC | . | . | . | . | . | . | . | . | . | . | . | . | . | |
| 21-225_31G1_LC | . | . | . | . | . | . | . | . | . | . | . | . | . | |
| 21-225_10C5_LC | . | . | . | . | . | . | . | . | . | . | . | R | . | |
| 21-225_18F7_LC | . | . | . | . | . | . | . | . | . | . | . | . | . | |
| 21-225_20G11_LC | . | . | . | . | . | . | . | . | . | . | . | . | . | |

FIGURE 56

Table 41

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | K_FR1 | | | | | | | | |
| CONSENSUS | D | I | Q | M | T | Q | S | P | S | S | L | S | A | S | V | G | D |
| 21-225_35A6_LC | . | . | . | . | . | . | . | . | . | . | . | . | . | . | . | . | . |
| 21-225_30E9_LC | . | . | . | . | . | . | . | . | . | . | . | . | . | . | . | . | . |
| 21-225_31H5_LC | . | . | . | . | . | . | . | . | . | . | . | . | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | I | . | . | . | . | . | . | . | . | . | . | . | . |
| 21-225_30G11_LC | . | . | . | . | . | . | . | . | . | . | . | . | . | . | . | . | . |
| 21-225_30E3_LC | . | . | . | . | . | . | . | . | . | . | . | . | . | . | . | . | . |
| 21-225_31H3_LC | . | . | . | . | . | . | . | . | . | . | . | . | . | . | . | . | . |
| 21-225_32G12_LC | . | . | . | . | . | . | . | . | . | . | . | . | . | . | . | . | . |
| 21-225_34G9_LC | . | . | . | . | . | . | . | . | . | . | . | . | . | . | . | . | . |
| 21-225_36C5_LC | . | . | . | . | . | . | . | . | . | . | . | . | . | . | . | . | . |
| 21-225_33A4_LC | . | . | . | . | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | . | . | . | . | . | . | . | . | . | . | . |
| 21-225_34A6_LC | . | . | . | . | . | . | . | . | . | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | . | . | . | . | . | . | . | . | . | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | . | . | . | . | . | . | . | . | . | . | . | . |
| 21-225_24B1_LC | . | V | V | . | . | . | . | . | . | L | . | . | P | V | T | L | . | Q |
| 21-225_32G1_LC | . | . | . | . | . | . | . | . | . | . | . | . | . | . | . | . | . |
| 21-225_33B8_LC | . | . | . | . | . | . | . | . | . | . | . | . | . | . | . | . | . |

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | L_FR1 | | | | | | | | |
| CONSENSUS | Q | S | | L | T | Q | P | - | | S | | S | G | S | P | G | Q |
| 21-225_4G12_LC | . | . | V | . | . | . | . | P | . | A | . | . | T | . | . | . |
| 21-225_17F12_LC | . | . | A | . | . | . | . | A | . | V | . | . | . | . | . | . |
| 21-225_21H4_LC | . | . | A | . | . | . | . | A | . | V | . | . | . | . | . | . |
| 21-225_12D8_LC | L | P | V | . | . | . | . | P | . | A | . | A | L | L | . | A |

3577

FIGURE 56

FIGURE 56

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | R | L | I | Y | A | - | - | - | - | - | - | - | - | A | S |
| 21-225_35A6_LC | . | . | . | . | . | . | . | . | . | . | . | . | . | . | . | T | . |
| 21-225_30E9_LC | . | R | . | . | . | . | G | . | . | . | . | . | . | . | . | . | F |
| 21-225_31H5_LC | H | . | . | I | . | . | . | . | . | . | . | . | . | . | . | . | . |
| 21-225_31A8_LC | . | . | . | . | . | . | . | . | . | . | . | . | . | . | . | V | . |
| 21-225_30D1_LC | . | E | . | . | . | . | . | . | . | . | . | . | . | . | . | . | . |
| 21-225_31B8_LC | . | . | . | . | . | . | G | . | . | . | . | . | . | . | . | . | . |
| 21-225_30G11_LC | . | . | . | . | . | . | . | . | . | . | . | . | . | . | . | . | . |
| 21-225_30E3_LC | . | R | . | . | . | . | . | . | . | . | . | . | . | . | . | . | F |
| 21-225_31H3_LC | . | R | . | . | . | . | . | . | . | . | . | . | . | . | . | . | F |
| 21-225_32G12_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36C5_LC | . | . | . | . | . | . | . | . | . | . | . | . | . | . | . | . | . |
| 21-225_33A4_LC | . | E | . | . | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | V | . | . | . | . | . | . | . | . | . | . |
| 21-225_34A6_LC | . | . | . | . | . | F | . | . | . | . | . | . | . | . | . | . | . |
| 21-225_35B7_LC | . | E | . | . | . | . | . | . | . | . | . | . | . | . | . | . | . |
| 21-225_34H11_LC | . | . | . | . | . | . | . | . | . | . | . | . | . | . | . | T | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | F | . | . | . | . | . | . | . | . | . | . | . |
| 21-225_24B1_LC | . | R | . | . | . | . | K | . | . | . | . | . | . | . | . | V | . |
| 21-225_32G1_LC | . | . | . | . | . | . | . | . | . | . | . | . | . | . | . | . | . |
| 21-225_33B8_LC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | L_CDR2 | | | |
| CONSENSUS | P | K | L | M | I | Y | E | - | - | - | - | - | - | - | - | V | S |
| 21-225_4G12_LC | . | . | . | L | . | . | I | . | . | . | . | . | . | . | . | N | N |
| 21-225_17F12_LC | . | . | . | . | . | . | . | . | . | . | . | . | . | . | . | . | . |
| 21-225_21H4_LC | . | . | . | . | . | . | . | . | . | . | . | . | . | . | . | . | . |
| 21-225_12D8_LC | . | Q | Y | I | M | K | V | K | S | . | . | . | . | D | G | S | H |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_35A6_LC | . | . | . | . | . | . | . | . | . | . | . | . | . | . | . | . | . |
| 21-225_30E9_LC | . | . | . | . | . | . | . | . | . | . | . | . | . | . | . | . | . |
| 21-225_31H5_LC | . | . | . | . | . | . | . | . | . | . | . | V | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | . | . | . | . | . | . | . | . | . | . | . | . | . |
| 21-225_30G11_LC | . | . | . | . | . | . | . | . | . | . | . | . | . | . | . | . | . |
| 21-225_30E3_LC | . | . | . | . | . | . | . | . | . | . | . | . | . | . | . | . | . |
| 21-225_31H3_LC | . | . | . | . | . | . | . | . | . | . | . | . | . | . | . | . | . |
| 21-225_32G12_LC | . | . | . | . | . | . | . | . | . | . | . | . | . | . | . | . | . |
| 21-225_34G9_LC | . | . | . | . | . | . | . | . | . | . | T | . | . | . | . | . | . |
| 21-225_36C5_LC | . | . | . | . | . | . | . | . | . | . | T | . | . | . | . | . | . |
| 21-225_33A4_LC | . | . | . | R | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | . | . | . | . | . | . | . | . | . | . | . |
| 21-225_34A6_LC | . | . | . | . | . | . | . | . | . | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | . | . | . | . | . | . | . | . | . | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | . | . | . | . | . | . | . | . | . | . | . | . |
| 21-225_24B1_LC | N | W | D | . | . | . | . | D | . | . | . | . | . | . | . | . | . |
| 21-225_32G1_LC | . | . | . | . | . | . | . | . | . | . | . | . | . | . | . | . | . |
| 21-225_33B8_LC | . | . | . | . | . | . | . | . | . | . | . | . | . | . | . | . | . |

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | N | R | P | S | G | V | | R | F | S | G | S | K | S | G | - | |
| 21-225_4G12_LC | Q | . | . | . | . | . | P | D | . | . | . | . | . | . | . | . | . |
| 21-225_17F12_LC | . | . | . | . | . | . | S | N | . | . | . | . | . | . | . | . | . |
| 21-225_21H4_LC | . | . | . | . | . | . | S | N | . | . | . | . | . | . | . | . | . |
| 21-225_12D8_LC | S | K | G | D | . | . | I | P | D | . | . | M | . | . | S | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | | |
| CONSENSUS | - | T | E | F | T | L | T | I | S | S | L | Q | P | E | D | F | A |
| 21-225_35A6_LC | . | . | . | . | . | . | . | . | . | . | . | . | . | . | . | . | . |
| 21-225_30E9_LC | . | . | . | . | . | . | . | . | . | . | . | . | . | . | . | . | . |
| 21-225_31H5_LC | . | . | . | . | . | F | . | . | . | . | M | . | . | D | . | . | S |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | T |
| 21-225_31B8_LC | . | . | . | . | . | . | . | . | . | . | . | . | . | . | . | . | . |
| 21-225_30G11_LC | . | . | . | . | . | . | . | G | . | . | . | . | . | . | . | . | . |
| 21-225_30E3_LC | . | . | . | . | . | . | . | . | . | . | . | . | . | . | . | . | . |
| 21-225_31H3_LC | . | . | . | . | . | . | . | . | . | . | . | . | . | . | . | . | . |
| 21-225_32G12_LC | . | . | . | . | . | . | . | . | . | . | . | . | . | . | . | . | . |
| 21-225_34G9_LC | . | . | . | . | . | . | . | . | . | . | . | . | . | . | A | . | . |
| 21-225_36C5_LC | . | . | . | . | . | . | . | . | . | . | V | . | . | . | . | . | . |
| 21-225_33A4_LC | . | . | . | . | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | . | . | . | . | . | . | . | . | . | . | . |
| 21-225_34A6_LC | . | . | . | . | . | . | . | . | V | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | . | . | . | . | . | . | . | . | . | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | . | . | . | . | . | . | . | . | . | . | . | . |
| 21-225_24B1_LC | . | . | D | . | . | . | K | . | . | W | V | E | A | . | . | V | G |
| 21-225_32G1_LC | . | . | . | . | . | . | . | . | . | . | . | . | . | . | . | . | . |
| 21-225_33B8_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | L_FR3 | | | | | | | | | | | | | |
| CONSENSUS | - | N | T | A | S | L | T | I | S | G | L | Q | | E | D | E | A |
| 21-225_4G12_LC | . | T | S | . | . | . | A | . | . | . | . | . | S | . | . | . | . |
| 21-225_17F12_LC | . | . | . | . | . | . | . | . | . | . | . | . | A | . | . | . | . |
| 21-225_21H4_LC | . | . | . | . | . | . | . | . | . | . | . | . | A | . | . | . | . |
| 21-225_12D8_LC | . | A | D | R | Y | . | . | F | . | N | . | . | S | D | . | . | D |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | T | I | - | - | - | - | - | - | - | - |
| 21-225_35A6_LC | . | . | . | . | . | . | . | . | . | . | . | . | . | . | . | . | . |
| 21-225_30E9_LC | . | . | . | . | . | . | . | . | S | . | . | . | . | . | . | . | . |
| 21-225_31H5_LC | N | . | . | . | I | . | . | . | I | . | . | . | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | . | . | . | I | . | . | . | . | . | . | . | . | . |
| 21-225_30G11_LC | . | . | F | . | . | . | . | . | . | . | . | . | . | . | . | . | . |
| 21-225_30E3_LC | . | . | . | . | . | . | . | . | S | . | . | . | . | . | . | . | . |
| 21-225_31H3_LC | . | . | . | . | . | . | . | . | S | . | . | . | . | . | . | . | . |
| 21-225_32G12_LC | . | . | . | . | . | . | . | . | . | . | . | . | . | . | . | . | . |
| 21-225_34G9_LC | I | . | . | . | . | . | . | . | . | . | . | . | . | . | . | . | . |
| 21-225_36C6_LC | . | . | . | . | . | H | . | P | . | . | . | . | . | . | . | . | . |
| 21-225_33A4_LC | R | . | . | . | . | . | . | Y | S | . | . | . | . | . | . | . | . |
| 21-225_33F1_LC | I | . | . | . | . | . | . | . | . | . | . | . | . | . | . | . | . |
| 21-225_33A7_LC | A | . | . | . | . | . | . | . | . | . | . | . | . | . | . | . | . |
| 21-225_34A6_LC | . | . | . | . | . | . | . | N | S | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | N | S | . | . | . | . | . | . | . | . |
| 21-225_33G7_LC | . | . | . | . | . | . | . | Y | S | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | V | . | . | . | . | . | . | . | . |
| 21-225_34G8_LC | . | . | . | . | . | . | . | . | . | . | . | . | . | . | . | . | . |
| 21-225_36A5_LC | . | . | . | . | . | H | . | N | S | . | . | . | . | . | . | . | . |
| 21-225_24B1_LC | V | C | . | . | M | . | G | A | H | . | . | . | . | . | . | . | . |
| 21-225_32G1_LC | . | . | . | . | . | . | . | . | . | . | . | . | . | . | . | . | . |
| 21-225_33B8_LC | . | . | F | . | . | . | . | . | . | . | . | . | . | . | . | . | . |

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | D | Y | Y | C | S | S | Y | T | | - | - | - | - | - | - | - | - |
| 21-225_4G12_LC | . | . | . | . | A | A | W | D | D | S | . | . | . | . | . | . | . |
| 21-225_17F12_LC | . | . | . | . | . | . | . | . | S | . | . | . | . | . | . | . | . |
| 21-225_21H4_LC | . | . | . | . | . | . | . | . | R | . | . | . | . | . | . | . | . |
| 21-225_12D8_LC | E | . | H | . | G | E | S | H | T | I | D | . | . | . | . | . | . |

**FIGURE 56**

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_35A6_LC | . | . | . | . | . | . | . | . | . | . | . | . | . | . | . | . | . |
| 21-225_30E9_LC | . | . | . | . | . | . | . | . | . | . | . | . | . | . | . | . | . |
| 21-225_31H5_LC | . | . | . | . | . | . | . | . | . | . | . | . | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | . | . | . | . | . | . | . | . | . | . | . | . | . |
| 21-225_30G11_LC | . | . | . | . | . | . | . | . | . | . | . | . | . | . | . | . | . |
| 21-225_30E3_LC | . | . | . | . | . | . | . | . | . | . | . | . | . | . | . | . | . |
| 21-225_31H3_LC | . | . | . | . | . | . | . | . | . | . | . | . | . | . | . | . | . |
| 21-225_32G12_LC | . | . | . | . | . | . | . | . | . | . | . | . | . | . | . | . | . |
| 21-225_34G9_LC | . | . | . | . | . | . | . | . | . | . | . | . | . | . | . | . | . |
| 21-225_36C5_LC | . | . | . | . | . | . | . | . | . | . | . | . | . | . | . | . | . |
| 21-225_33A4_LC | . | . | . | . | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | . | . | . | . | . | . | . | . | . | . | . |
| 21-225_34A6_LC | . | . | . | . | . | . | . | . | . | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | . | . | . | . | . | . | . | . | . | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | . | . | . | . | . | . | . | . | . | . | . | . |
| 21-225_24B1_LC | . | . | . | . | . | . | . | . | . | . | . | . | . | . | W | P | . |
| 21-225_32G1_LC | . | . | . | . | . | . | . | . | . | . | . | . | . | . | . | S | . |
| 21-225_33B8_LC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | L_CDR3 | | | | | | | | | | | | | | |
| CONSENSUS | - | - | - | - | - | - | - | - | - | - | - | - | - | - | S | - | T |
| 21-225_4G12_LC | . | . | . | . | . | . | . | . | . | . | . | . | . | L | N | G | H |
| 21-225_17F12_LC | . | . | . | . | . | . | . | . | . | . | . | . | . | . | . | I | . |
| 21-225_21H4_LC | . | . | . | . | . | . | . | . | . | . | . | . | . | . | . | S | . |
| 21-225_12D8_LC | . | . | . | . | . | . | . | . | . | . | . | . | . | G | Q | V | G |

**FIGURE 56**

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | K_FR4 | | | | | | |
| CONSENSUS | P | T | F | G | G | G | T | K | V | E | I | K | R |
| 21-225_35A6_LC | | | | | | | | | | | | | |
| 21-225_30E9_LC | | | | | | | | | | | | | |
| 21-225_31H5_LC | | | | | | | | | | | | | |
| 21-225_31A8_LC | | | | | | | | | | | | | |
| 21-225_30D1_LC | | | | | | | | | | | | | |
| 21-225_31B8_LC | | | | | | | | | | | | | |
| 21-225_30G11_LC | | | | | | | | | | | | | |
| 21-225_30E3_LC | | | | | | | | | | | | | |
| 21-225_31H3_LC | | | | | | | | | | | | | |
| 21-225_32G12_LC | | | | | | | | | | | | | |
| 21-225_34G9_LC | | | | | | | | | | W | | | |
| 21-225_36C5_LC | | | | | | | | | | W | | | |
| 21-225_33A4_LC | . | . | | | | | | | | | | | |
| 21-225_33F1_LC | | | | | | | | | | | | | |
| 21-225_33A7_LC | | | | | | | | | | | | | |
| 21-225_34A6_LC | | | | | | | | | | | | | |
| 21-225_35B7_LC | | | | | | | | | | | | | |
| 21-225_34H11_LC | | | | | | | | | | | | | |
| 21-225_33G7_LC | . | | | | | | | | | | | | |
| 21-225_35D1_LC | | | | | | | | | | | | | |
| 21-225_35F11_LC | | | | | | | | | | | | | |
| 21-225_34G8_LC | | | | | | | | | | | | | |
| 21-225_36A5_LC | | K | | | | | I | | | | | T | G |
| 21-225_24B1_LC | I | | | | Q | | | R | L | | | | |
| 21-225_32G1_LC | | | | | | | | | | | | | |
| 21-225_33B8_LC | | | | | | | | | | | | | |

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | L_FR4 | | | | | | |
| CONSENSUS | V | V | F | G | G | G | T | K | L | T | V | L | G |
| 21-225_4G12_LC | . | . | . | . | . | R | . | . | | . | . | | |
| 21-225_17F12_LC | | | | | | | | | | | | | |
| 21-225_21H4_LC | . | . | . | | | | | | | | | | |
| 21-225_12D8_LC | | | | | | | | | | | | | |

FIGURE 56

Table 42

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K_FR1 | | | | | |
| CONSENSUS | D | I | Q | M | T | Q | S | P | S | S | L | S | A | S | V | G | D |
| 21-225_11A5_LC | . | . | . | . | . | . | . | . | . | . | . | . | . | . | . | . | . |
| 21-225_17C6_LC | . | . | . | . | . | . | . | . | . | . | . | . | . | . | . | . | . |
| 21-225_17B4_LC | . | . | . | . | . | . | . | . | . | . | . | . | . | . | . | . | . |
| 21-225_20A4_LC | . | . | . | . | . | . | . | . | A | . | . | . | . | . | . | . | . |
| 21-225_17F7_LC | . | . | . | . | . | . | . | . | . | . | . | . | . | . | . | . | . |
| 21-225_17F11_LC | . | . | . | . | . | . | . | . | . | . | . | . | . | . | I | . | . |
| 21-225_17A12_LC | . | . | . | . | . | . | . | . | . | . | . | . | . | . | . | . | . |
| 21-225_18E12_LC | . | . | . | . | . | . | . | . | . | . | . | . | . | . | . | . | . |
| 21-225_22G3_LC | . | . | . | . | . | . | . | . | . | . | . | . | . | . | . | . | . |
| 21-225_22G10_LC | . | . | . | . | . | . | . | . | . | . | . | . | . | . | . | . | . |
| 21-225_21F4_LC | . | . | . | . | . | . | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C5_LC | . | . | . | . | . | . | . | . | A | . | . | . | . | . | . | . | . |
| 21-225_22D5_LC | . | . | . | . | . | . | . | . | . | . | . | . | . | . | . | . | . |
| 21-225_10F12_LC | . | . | . | . | . | . | . | . | . | . | . | . | . | . | . | . | . |
| 21-225_4H2_LC | . | . | . | . | . | L | . | . | . | . | . | . | . | . | . | . | . |
| 21-225_5G2_LC | . | . | . | . | . | . | . | . | . | . | . | P | . | . | . | . | . |
| 21-225_3E3_LC | . | . | . | . | . | . | . | . | . | . | . | P | . | . | . | . | . |
| 21-225_5F7_LC | . | . | . | . | . | . | . | . | . | . | . | P | . | . | . | . | . |
| 21-225_6D3_LC | . | . | . | . | . | . | . | . | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | . | . | . | . | . |
| 21-225_3B1_LC | . | . | . | . | . | . | . | . | . | . | . | . | . | . | . | . | . |
| 21-225_8E12_LC | . | . | . | . | . | . | . | . | . | . | . | . | . | . | . | . | . |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | S | I | S | - | - |
| 21-225_11A5_LC | . | . | . | . | . | . | . | . | . | . | . | . | N | . | Y | . | . |
| 21-225_17C6_LC | . | . | . | . | . | . | . | . | . | . | . | . | . | . | N | . | . |
| 21-225_17B4_LC | . | . | . | . | . | . | . | . | . | . | . | . | N | . | . | . | . |
| 21-225_20A4_LC | . | . | . | . | . | . | . | . | . | . | . | . | . | . | I | . | . |
| 21-225_17F7_LC | . | . | . | . | . | . | . | . | . | . | . | . | T | . | . | . | . |
| 21-225_17F11_LC | . | . | S | . | . | . | . | . | . | . | . | R | T | . | N | . | . |
| 21-225_17A12_LC | . | . | . | . | . | . | . | . | . | . | . | . | . | . | . | . | . |
| 21-225_18E12_LC | . | . | . | . | . | . | . | . | . | . | . | . | . | . | . | . | . |
| 21-225_22G3_LC | . | . | . | . | . | . | . | . | . | . | . | . | . | . | . | . | . |
| 21-225_22G10_LC | . | . | . | . | . | . | . | . | . | . | . | . | . | . | F | . | . |
| 21-225_21F4_LC | . | . | . | . | . | . | . | . | . | . | . | . | N | . | G | . | . |
| 21-225_22E4_LC | . | . | . | . | . | . | . | . | . | . | . | . | N | . | . | . | . |
| 21-225_21A2_LC | . | . | . | . | . | . | . | . | . | . | . | . | . | . | . | . | . |
| 21-225_22C5_LC | . | . | . | . | . | . | . | . | . | . | . | . | N | . | . | . | . |
| 21-225_22D5_LC | . | . | . | . | . | . | . | . | . | . | . | . | N | . | F | . | . |
| 21-225_10F12_LC | . | . | . | . | . | . | . | . | . | . | . | . | N | . | . | . | . |
| 21-225_4H2_LC | . | . | . | . | . | . | . | . | I | . | . | . | N | . | . | . | . |
| 21-225_5G2_LC | . | . | . | . | F | . | . | . | . | . | . | . | N | . | I | . | . |
| 21-225_3E3_LC | . | . | . | . | F | . | . | . | . | . | . | . | . | . | R | . | . |
| 21-225_5F7_LC | . | . | . | . | F | . | . | . | . | . | . | . | T | . | . | . | . |
| 21-225_6D3_LC | . | . | . | . | . | . | . | T | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | N | . | . | . | . |
| 21-225_3B1_LC | . | . | . | . | . | . | . | . | . | . | . | . | . | . | N | . | . |
| 21-225_8E12_LC | . | . | . | . | . | . | . | . | . | . | . | . | . | . | . | . | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | K_FR2 | | |
| CONSENSUS | - | - | - | - | S | Y | L | N | W | Y | Q | Q | K | P | G | K | A |
| 21-225_11A5_LC | . | . | . | . | . | . | . | . | . | . | . | . | . | . | . | . | . |
| 21-225_17C6_LC | . | . | . | . | T | . | . | . | . | . | . | . | . | . | . | . | . |
| 21-225_17B4_LC | . | . | . | . | . | . | . | . | . | . | . | . | . | . | . | . | . |
| 21-225_20A4_LC | . | . | . | . | N | . | . | . | . | . | . | . | R | . | . | . | S |
| 21-225_17F7_LC | . | . | . | . | . | F | . | . | . | . | . | . | . | . | . | . | . |
| 21-225_17F11_LC | . | . | . | . | N | . | . | . | . | . | . | . | . | . | . | . | . |
| 21-225_17A12_LC | . | . | . | . | . | . | . | . | . | . | H | . | . | . | . | . | . |
| 21-225_18E12_LC | . | . | . | . | . | . | . | . | . | . | . | . | . | . | . | . | . |
| 21-225_22G3_LC | . | . | . | . | N | . | . | . | . | . | . | . | . | . | . | . | . |
| 21-225_22G10_LC | . | . | . | . | . | . | . | . | . | . | . | . | . | . | . | . | . |
| 21-225_21F4_LC | . | . | . | . | . | . | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C5_LC | . | . | . | . | . | . | . | . | . | . | . | . | . | . | . | . | . |
| 21-225_22D5_LC | . | . | . | . | . | . | . | . | . | . | H | . | . | . | . | . | . |
| 21-225_10F12_LC | . | . | . | . | . | . | . | . | . | . | . | . | . | . | . | . | . |
| 21-225_4H2_LC | . | . | . | . | . | . | . | . | . | . | . | . | . | . | . | . | . |
| 21-225_5G2_LC | . | . | . | . | . | . | . | . | . | . | . | . | . | . | . | . | . |
| 21-225_3E3_LC | . | . | . | . | . | . | . | . | . | . | . | . | . | . | . | . | . |
| 21-225_5F7_LC | . | . | . | . | . | . | . | . | . | . | . | . | . | . | . | . | . |
| 21-225_6D3_LC | . | . | . | . | T | . | . | . | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | . | . | . | . | . |
| 21-225_3B1_LC | . | . | . | . | N | . | . | . | . | . | . | . | . | . | . | . | . |
| 21-225_8E12_LC | . | . | . | . | N | . | . | . | . | . | . | . | . | . | . | . | . |

**FIGURE 56**

| Reference # | | | | | | | | | | | | | | K_CDR2 | | | |
|---|---|---|---|---|---|---|---|---|---|---|---|---|---|---|---|---|---|
| CONSENSUS | P | K | L | L | I | Y | A | - | - | - | - | - | - | - | - | A | S |
| 21-225_11A5_LC | . | . | . | . | . | . | . | . | . | . | . | . | . | . | . | . | . |
| 21-225_17C6_LC | . | . | . | . | . | . | . | . | . | . | . | . | . | . | . | . | . |
| 21-225_17B4_LC | . | . | . | . | . | F | G | . | . | . | . | . | . | . | . | . | . |
| 21-225_20A4_LC | . | . | . | . | . | . | . | . | . | . | . | . | . | . | . | . | . |
| 21-225_17F7_LC | . | . | . | . | . | . | . | . | . | . | . | . | . | . | . | . | . |
| 21-225_17F11_LC | . | . | . | . | . | F | . | . | . | . | . | . | . | . | . | T | . |
| 21-225_17A12_LC | . | . | . | . | . | . | . | . | . | . | . | . | . | . | . | . | . |
| 21-225_18E12_LC | . | T | . | . | . | . | . | . | . | . | . | . | . | . | . | . | . |
| 21-225_22G3_LC | . | . | . | . | . | F | . | . | . | . | . | . | . | . | . | . | . |
| 21-226_22G10_LC | . | . | . | . | . | . | . | . | . | . | . | . | . | . | . | . | . |
| 21-225_21F4_LC | . | . | . | . | . | F | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C5_LC | . | . | . | . | . | . | . | . | . | . | . | . | . | . | . | . | . |
| 21-225_22D6_LC | . | . | . | . | . | . | . | . | . | . | . | . | . | . | . | . | . |
| 21-225_10F12_LC | . | . | . | . | . | . | . | . | . | . | . | . | . | . | . | . | . |
| 21-225_4H2_LC | . | . | . | V | . | . | . | . | . | . | . | . | . | . | . | . | . |
| 21-225_5G2_LC | R | . | . | V | . | . | G | . | . | . | . | . | . | . | . | . | . |
| 21-226_3E3_LC | N | . | . | V | . | . | G | . | . | . | . | . | . | . | . | T | . |
| 21-225_5F7_LC | . | . | . | . | . | S | . | . | . | . | . | . | . | . | . | . | . |
| 21-226_6D3_LC | . | . | . | . | . | F | . | . | . | . | . | . | . | . | . | . | . |
| 21-225_8G6_LC | . | . | . | . | . | F | . | . | . | . | . | . | . | . | . | . | . |
| 21-225_16H6_LC | . | . | . | . | . | . | . | . | . | . | . | . | . | . | . | . | . |
| 21-225_3B1_LC | . | . | . | . | . | . | . | . | . | . | . | . | . | . | . | T | . |
| 21-225_8E12_LC | . | . | . | . | . | . | . | . | . | . | . | . | . | . | . | . | . |

# EP 4 435 105 A2

## FIGURE 56

| Reference # | 70 | 71 | c | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_11A5_LC | . | . | . | . | . | . | . | . | . | . | . | . | . | . | . | . | . |
| 21-225_17C6_LC | . | . | . | . | . | . | . | . | . | . | . | . | . | . | . | . | . |
| 21-225_17B4_LC | . | . | . | T | . | . | . | . | . | . | . | . | . | . | . | . | . |
| 21-225_20A4_LC | . | . | H | . | . | . | . | . | . | . | . | . | R | . | . | . | . |
| 21-225_17F7_LC | . | . | . | . | . | . | . | . | . | . | . | . | . | . | . | . | . |
| 21-225_17F11_LC | . | . | . | . | . | . | . | . | . | . | . | . | . | . | . | . | . |
| 21-225_17A12_LC | . | . | . | . | . | . | . | . | . | . | . | . | . | . | . | . | . |
| 21-225_18E12_LC | . | . | . | . | . | . | . | . | . | . | . | . | . | . | . | . | . |
| 21-225_22G3_LC | . | . | . | . | . | . | . | . | . | . | . | . | . | . | . | . | . |
| 21-225_22G10_LC | . | . | . | . | . | . | . | . | . | . | . | . | . | . | . | . | . |
| 21-225_21F4_LC | . | . | . | . | . | . | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C6_LC | V | . | . | H | . | I | . | . | . | . | . | . | R | . | . | . | . |
| 21-225_22D5_LC | . | . | . | . | . | . | . | . | . | . | . | . | . | . | . | . | . |
| 21-225_10F12_LC | . | . | . | . | . | . | . | . | . | . | . | . | . | . | . | . | . |
| 21-226_4H2_LC | . | . | . | . | . | . | . | . | . | . | . | . | R | . | . | . | . |
| 21-225_5G2_LC | . | . | . | . | . | . | . | . | . | . | . | . | . | . | . | . | . |
| 21-225_3E3_LC | . | . | . | . | . | . | . | . | . | . | . | . | . | . | . | . | . |
| 21-225_5F7_LC | . | . | . | . | . | . | . | . | . | . | . | . | . | . | . | . | . |
| 21-225_6D3_LC | . | . | . | . | . | . | . | . | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | R | . | . | . | . |
| 21-225_3B1_LC | . | . | . | N | . | . | . | . | . | . | . | . | . | . | . | . | . |
| 21-225_8E12_LC | . | . | . | . | . | . | . | . | . | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | | |
| CONSENSUS | - | T | D | F | T | L | T | I | S | S | L | Q | P | E | D | F | A |
| 21-225_11A5_LC | . | . | . | . | . | . | . | . | . | . | . | . | . | . | . | . | . |
| 21-225_17C6_LC | . | . | . | . | . | . | . | . | . | . | . | . | . | . | . | . | . |
| 21-225_17B4_LC | . | . | . | . | . | . | . | . | N | . | . | . | . | . | . | . | . |
| 21-225_20A4_LC | . | . | . | . | . | . | . | . | . | . | . | . | . | . | . | . | . |
| 21-225_17F7_LC | . | . | . | . | . | . | . | . | . | G | . | . | . | . | . | . | . |
| 21-225_17F11_LC | . | . | . | . | . | . | . | . | . | . | . | . | . | . | . | . | . |
| 21-225_17A12_LC | . | . | . | . | . | . | . | . | . | N | . | . | . | . | . | . | . |
| 21-225_18E12_LC | . | . | . | . | . | . | . | . | N | . | . | . | . | . | . | . | . |
| 21-225_22G3_LC | . | . | . | . | . | . | . | . | . | G | . | . | . | . | . | . | . |
| 21-225_22G10_LC | . | . | . | . | . | . | . | . | . | . | . | . | . | . | . | . | . |
| 21-225_21F4_LC | . | . | . | . | . | . | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C5_LC | . | . | . | . | . | . | I | . | . | . | . | . | . | . | . | . | . |
| 21-225_22D5_LC | . | . | . | . | . | . | . | . | . | . | . | . | . | . | . | . | . |
| 21-225_10F12_LC | . | . | . | . | . | . | . | . | . | . | . | . | . | . | . | . | . |
| 21-225_4H2_LC | . | . | . | . | . | . | . | . | G | C | V | . | R | . | . | . | . |
| 21-225_5G2_LC | . | . | . | . | . | . | N | . | R | . | . | . | . | . | . | . | . |
| 21-225_3E3_LC | . | . | . | . | . | . | . | . | . | . | . | . | . | . | . | . | . |
| 21-225_5F7_LC | . | . | . | . | . | . | . | . | . | . | . | . | . | . | . | . | . |
| 21-225_6D3_LC | . | . | . | . | . | . | . | . | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | . | . | . | . | . |
| 21-225_3B1_LC | . | . | . | . | . | . | . | . | . | . | . | . | . | . | . | . | . |
| 21-225_8E12_LC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | S | Y | R | - | - | - | - | - | - | - | - |
| 21-225_11A5_LC | | | | | | | | | N | | | | | | | | |
| 21-225_17C6_LC | | | F | | | | | | | | | | | | | | |
| 21-225_17B4_LC | | | F | | | | | | | | | | | | | | |
| 21-225_20A4_LC | | | F | | | | | | | | | | | | | | |
| 21-225_17F7_LC | | | F | | | | | | | | | | | | | | |
| 21-225_17F11_LC | | | | | | | T | | S | | | | | | | | |
| 21-225_17A12_LC | | | | | | | | | N | | | | | | | | |
| 21-225_18E12_LC | | | F | | | | | | | | | | | | | | |
| 21-225_22G3_LC | | | F | | | | | F | | | | | | | | | |
| 21-225_22G10_LC | | | F | | | | | F | | | | | | | | | |
| 21-225_21F4_LC | | F | F | | | | | | | | | | | | | | |
| 21-225_22E4_LC | | | | | | | | | N | | | | | | | | |
| 21-225_21A2_LC | | | F | | | | | | | | | | | | | | |
| 21-225_22C5_LC | | | | | | | | | | | | | | | | | |
| 21-225_22D5_LC | | | | | | | | | S | | | | | | | | |
| 21-225_10F12_LC | | | | | | | | | N | | | | | | | | |
| 21-225_4H2_LC | | | | | | | | | | | | | | | | | |
| 21-225_5G2_LC | | | | | | | | | S | | | | | | | | |
| 21-225_3E3_LC | | | | | | | | | S | | | | | | | | |
| 21-225_6F7_LC | | | | | | | | | N | | | | | | | | |
| 21-225_6D3_LC | | | F | | | | | | | | | | | | | | |
| 21-225_8G6_LC | | | F | | | | | | | | | | | | | | |
| 21-225_16H6_LC | | | | | | | | | | | | | | | | | |
| 21-225_3B1_LC | | | | | | | | | S | | | | | | | | |
| 21-225_8E12_LC | | | F | | | | | | | | | | | | | | |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | T | P | L |
| 21-225_11A5_LC | | | | | | | | | | | | | | | | | |
| 21-225_17C6_LC | | | | | | | | | | | | | | | | | F |
| 21-225_17B4_LC | | | | | | | | | | | | | | | | | F |
| 21-225_20A4_LC | | | | | | | | | | | | | | | | | |
| 21-225_17F7_LC | | | | | | | | | | | | | | | | | F |
| 21-225_17F11_LC | | | | | | | | | | | | | | | | | |
| 21-225_17A12_LC | | | | | | | | | | | | | | | | | |
| 21-225_18E12_LC | | | | | | | | | | | | | | | | | F |
| 21-225_22G3_LC | | | | | | | | | | | | | | | | | F |
| 21-225_22G10_LC | | | | | | | | | | | | | | | | | F |
| 21-225_21F4_LC | | | | | | | | | | | | | | | | | F |
| 21-225_22E4_LC | | | | | | | | | | | | | | | | | |
| 21-225_21A2_LC | | | | | | | | | | | | | | | | | F |
| 21-225_22C6_LC | | | | | | | | | | | | | | | | | |
| 21-225_22D5_LC | | | | | | | | | | | | | | | | | |
| 21-225_10F12_LC | | | | | | | | | | | | | | | | | F |
| 21-225_4H2_LC | | | | | | | | | | | | | | | | | |
| 21-225_5G2_LC | | | | | | | | | | | | | | | | | |
| 21-225_3E3_LC | | | | | | | | | | | | | | | | | |
| 21-225_5F7_LC | | | | | | | | | | | | | | | | | |
| 21-225_6D3_LC | | | | | | | | | | | | | | | | | F |
| 21-225_8G6_LC | | | | | | | | | | | | | | | | | F |
| 21-225_16H6_LC | | | | | | | | | | | | | | | | | |
| 21-225_3B1_LC | | | | | | | | | | | | | | | | | |
| 21-225_8E12_LC | | | | | | | | | | | | | | | | | F |

**FIGURE 56**

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | | K_FR4 | | | | | |
| CONSENSUS | F | T | F | G | P | G | T | K | V | D | I | K | R |
| 21-225_11A5_LC | . | . | . | . | . | . | . | . | . | . | . | . | . |
| 21-225_17C6_LC | . | . | . | . | . | . | . | . | . | . | . | . | . |
| 21-225_17B4_LC | . | . | . | . | . | . | . | . | . | . | . | . | . |
| 21-225_20A4_LC | . | . | . | . | . | . | . | . | . | . | F | . | . |
| 21-225_17F7_LC | . | . | . | . | . | . | . | . | . | . | . | . | . |
| 21-225_17F11_LC | . | A | . | . | . | . | . | . | . | . | . | . | . |
| 21-225_17A12_LC | . | . | . | . | . | . | . | . | . | . | . | . | . |
| 21-225_18E12_LC | . | . | . | . | . | . | . | . | . | . | . | . | . |
| 21-225_22G3_LC | . | . | . | . | . | . | . | . | . | . | . | . | . |
| 21-225_22G10_LC | . | . | . | . | . | . | . | . | . | . | F | . | . |
| 21-225_21F4_LC | . | . | . | . | . | . | . | . | . | . | F | . | . |
| 21-225_22E4_LC | . | . | . | . | . | . | . | . | . | . | . | . | . |
| 21-225_21A2_LC | . | . | . | . | . | . | . | . | . | . | . | . | . |
| 21-225_22C5_LC | . | . | . | . | . | . | . | . | . | . | F | . | . |
| 21-225_22D5_LC | . | . | . | . | . | . | . | . | . | . | . | . | . |
| 21-225_10F12_LC | . | . | . | . | . | . | . | . | . | . | . | . | . |
| 21-225_4H2_LC | . | . | . | . | . | . | . | . | . | . | . | . | . |
| 21-225_5G2_LC | . | . | . | . | . | . | . | . | . | . | . | . | . |
| 21-225_3E3_LC | . | . | . | . | . | . | . | . | . | . | . | . | . |
| 21-225_5F7_LC | . | . | . | . | . | . | . | . | . | V | . | . | . |
| 21-225_8D3_LC | . | . | . | . | . | . | . | . | . | . | . | . | . |
| 21-225_8G6_LC | . | . | . | . | . | . | . | . | . | . | . | . | . |
| 21-225_18H6_LC | . | . | . | . | . | . | . | . | . | . | F | . | . |
| 21-225_3B1_LC | . | . | . | . | . | . | . | . | . | . | . | N | . |
| 21-225_8E12_LC | . | . | . | . | . | . | . | . | . | . | . | . | . |

**FIGURE 56**

Table 43

| Reference # | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| | | | | | | | | | | | K_FR1 | | | | | |
| CONSENSUS | D | I | Q | M | T | Q | S | P | S | S | L | S | A | S | V | G |
| 21-225_20F6_LC | . | . | . | . | . | . | . | . | . | . | . | . | . | . | . | . |
| 21-225_18G4_LC | . | . | H | . | . | . | . | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | . | . | . | . | V | . | . | . | . | . |
| 21-225_26G11_LC | . | . | V | . | . | . | T | . | L | . | . | . | V | T | P | . |
| 21-225_26D12_LC | . | . | . | . | . | . | . | . | . | . | V | . | . | . | . | . |
| 21-226_26B3_LC | . | . | . | . | . | . | . | . | . | . | V | . | . | . | . | . |
| 21-225_25F3_LC | . | . | . | . | . | . | . | . | . | . | V | . | . | . | . | . |
| 21-225_3F8_LC | . | F | . | . | . | . | . | . | . | . | . | . | . | . | . | . |
| 21-225_7E11_LC | . | . | . | . | . | . | . | . | . | . | . | . | . | . | . | . |
| 21-225_6G1_LC | . | . | . | . | . | . | . | . | . | . | . | . | . | . | . | . |
| 21-225_15A3_LC | . | . | . | . | . | . | . | . | . | . | . | . | . | . | . | . |
| 21-225_13D4_LC | . | L | . | . | . | . | . | . | . | . | . | . | . | . | . | . |
| 21-225_16E6_LC | . | . | . | . | . | . | . | . | . | . | . | . | . | . | . | . |

| Reference # | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|
| | | | | | | | | | | | | | | | | K |
| CONSENSUS | D | R | V | T | I | T | C | R | A | S | - | - | Q | S | I | I |
| 21-225_20F6_LC | . | . | . | . | . | . | . | . | . | . | . | . | . | . | . | . |
| 21-225_18G4_LC | . | . | . | . | . | . | . | . | . | . | . | . | . | . | . | . |
| 21-225_22C7_LC | . | . | . | . | . | . | . | . | . | . | . | . | . | N | . | . |
| 21-225_27A11_LC | . | . | . | . | . | . | . | . | . | . | . | . | . | G | . | S |
| 21-225_26G11_LC | Q | P | A | S | . | S | . | K | S | . | . | . | . | T | L | L |
| 21-225_26D12_LC | . | . | . | . | . | . | . | . | . | . | . | . | . | G | . | S |
| 21-225_25B3_LC | . | . | . | . | . | . | . | . | . | . | . | . | . | G | . | S |
| 21-225_25F3_LC | . | . | . | . | . | . | . | . | . | . | . | . | . | G | . | S |
| 21-225_3F8_LC | . | . | . | . | . | . | . | . | . | . | . | . | . | . | . | L |
| 21-225_7E11_LC | . | . | . | . | A | . | . | . | . | . | . | . | . | N | . | . |
| 21-225_6G1_LC | . | . | . | . | . | . | . | . | T | . | . | . | . | N | . | . |
| 21-225_15A3_LC | . | . | . | . | . | . | . | . | . | . | . | . | . | . | . | . |
| 21-225_13D4_LC | . | . | . | . | . | . | . | . | . | . | . | . | . | . | . | F |
| 21-225_16E6_LC | . | . | . | . | . | . | . | . | . | . | . | . | . | . | . | . |

**FIGURE 56**

FIGURE 56

**Reference # (CDR2 / positions 65–80)**

| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| CONSENSUS | - | - | A | S | S | L | Q | S | G | V | P | S | R | F | S | G |
| 21-225_20F6_LC | . | . | . | . | . | . | . | . | . | . | . | . | . | . | . | . |
| 21-225_18G4_LC | . | . | . | . | . | . | . | T | . | . | . | . | G | . | . | . |
| 21-225_22C7_LC | . | . | . | . | . | . | . | . | . | . | . | . | . | . | . | . |
| 21-225_27A11_LC | . | . | . | . | . | . | . | . | . | . | . | . | . | . | . | A |
| 21-225_26G11_LC | . | . | V | . | N | R | F | . | . | . | . | D | . | . | . | . |
| 21-225_26D12_LC | . | . | . | . | . | . | . | . | . | . | . | . | . | . | . | A |
| 21-225_25B3_LC | . | . | . | . | . | . | . | . | . | . | . | . | . | . | . | A |
| 21-225_25F3_LC | . | . | . | . | . | . | . | . | . | . | . | . | . | . | . | A |
| 21-225_3F8_LC | . | . | T | . | . | . | . | . | . | . | . | . | . | . | . | . |
| 21-225_7E11_LC | . | . | . | . | . | . | . | . | . | . | . | . | . | . | . | . |
| 21-225_6G1_LC | . | . | . | . | N | . | . | T | . | . | . | . | G | . | . | . |
| 21-225_15A3_LC | . | . | . | . | . | . | . | . | . | . | . | . | . | . | . | . |
| 21-225_13D4_LC | . | . | . | . | . | . | . | N | . | . | . | . | . | . | . | . |
| 21-225_16E6_LC | . | . | T | . | . | . | . | . | . | . | . | . | . | . | . | . |

**Reference # (K_FR3 / positions 81–96)**

| Reference # | 81 | 82 | 83 | 84 | 85 | 86 | 87 | 88 | 89 | 90 | 91 | 92 | 93 | 94 | 95 | 96 |
|---|---|---|---|---|---|---|---|---|---|---|---|---|---|---|---|---|
| CONSENSUS | S | G | S | G | - | - | T | D | F | T | L | T | I | S | S | L |
| 21-225_20F6_LC | . | . | . | . | . | . | . | . | . | . | . | . | . | . | . | . |
| 21-225_18G4_LC | . | . | . | . | . | . | . | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | . | . | . | . | . | . | . | . | . | . |
| 21-225_26G11_LC | . | . | . | . | . | . | . | . | . | . | . | K | . | . | R | V |
| 21-225_26D12_LC | . | . | . | . | . | . | . | . | . | . | . | . | . | . | . | . |
| 21-225_25B3_LC | . | . | . | . | . | . | . | . | . | . | . | . | . | . | . | . |
| 21-225_25F3_LC | . | . | . | . | . | . | E | . | . | . | . | . | . | . | . | . |
| 21-225_3F8_LC | . | . | . | . | . | . | V | . | . | . | . | . | . | . | . | . |
| 21-225_7E11_LC | . | . | . | . | . | . | . | . | . | . | . | . | . | . | . | . |
| 21-225_6G1_LC | . | . | . | . | . | . | . | . | . | . | . | . | . | . | . | . |
| 21-225_15A3_LC | . | . | . | . | . | . | . | . | . | . | . | . | . | . | . | . |
| 21-225_13D4_LC | . | . | . | . | . | . | . | . | . | . | . | . | . | . | . | . |
| 21-225_16E6_LC | . | . | . | . | . | . | . | . | . | . | . | . | . | . | . | . |

FIGURE 56

| Reference # | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|
| CONSENSUS | Q | P | E | D | F | A | T | Y | Y | C | Q | Q | T | Y | S | - |
| 21-225_20F6_LC | . | . | . | . | . | . | . | . | . | . | . | . | . | . | . | . |
| 21-225_18G4_LC | . | . | . | . | . | . | . | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | . | . | C | . | . | . | S | D | . | . |
| 21-225_26G11_LC | E | A | . | . | V | G | I | . | . | . | M | . | S | I | K | . |
| 21-225_26D12_LC | . | . | . | . | . | . | . | . | C | . | . | . | S | D | . | . |
| 21-225_25B3_LC | . | . | . | . | . | . | . | . | C | . | . | . | S | D | . | . |
| 21-225_25F3_LC | . | . | . | . | . | . | . | . | C | . | . | . | S | D | . | . |
| 21-225_3F8_LC | . | . | . | . | . | . | . | . | . | . | . | . | . | . | . | . |
| 21-225_7E11_LC | . | . | . | . | . | . | I | . | . | . | . | . | . | . | . | . |
| 21-225_6G1_LC | . | . | . | . | . | . | . | . | . | . | . | . | P | H | . | . |
| 21-225_15A3_LC | . | . | . | . | . | . | . | . | . | . | . | . | . | . | . | . |
| 21-225_13D4_LC | . | . | . | . | . | . | . | . | . | . | . | . | . | . | . | . |
| 21-225_16E6_LC | . | . | . | . | . | . | . | . | . | . | . | . | . | . | . | . |

| Reference # | 113 | 114 | 115 | 116 | 117 | 118 | 119 | 120 | 121 | 122 | 123 | 124 | 125 | 126 | 127 | 128 |
|---|---|---|---|---|---|---|---|---|---|---|---|---|---|---|---|---|
| | | | | | | | | | | K_CDR3 | | | | | | |
| CONSENSUS | - | - | - | - | - | - | - | - | - | - | - | - | - | - | - | - |
| 21-225_20F6_LC | . | . | . | . | . | . | . | . | . | . | . | . | . | . | . | . |
| 21-225_18G4_LC | . | . | . | . | . | . | . | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | . | . | . | . | . | . | . | . | . | . |
| 21-225_26G11_LC | . | . | . | . | . | . | . | . | . | . | . | . | . | . | . | . |
| 21-225_26D12_LC | . | . | . | . | . | . | . | . | . | . | . | . | . | . | . | . |
| 21-225_25B3_LC | . | . | . | . | . | . | . | . | . | . | . | . | . | . | . | . |
| 21-225_25F3_LC | . | . | . | . | . | . | . | . | . | . | . | . | . | . | . | . |
| 21-225_3F8_LC | . | . | . | . | . | . | . | . | . | . | . | . | . | . | . | . |
| 21-225_7E11_LC | . | . | . | . | . | . | . | . | . | . | . | . | . | . | . | . |
| 21-225_6G1_LC | . | . | . | . | . | . | . | . | . | . | . | . | . | . | . | . |
| 21-225_15A3_LC | . | . | . | . | . | . | . | . | . | . | . | . | . | . | . | . |
| 21-225_13D4_LC | . | . | . | . | . | . | . | . | . | . | . | . | . | . | . | . |
| 21-225_16E6_LC | . | . | . | . | . | . | . | . | . | . | . | . | . | . | . | . |

FIGURE 56

| Reference # | 129 | 130 | 131 | 132 | 133 | 134 | 135 | 136 | 137 | 138 | 139 | 140 | 141 | 142 | 143 | 144 |
|---|---|---|---|---|---|---|---|---|---|---|---|---|---|---|---|---|
| | | | | | | | | | | | | | | | | K_FR |
| CONSENSUS | - | - | - | - | - | - | T | P | L | T | F | G | G | G | T | K |
| 21-225_20F6_LC | . | . | . | . | . | . | P | . | . | . | . | . | . | . | . | . |
| 21-225_18G4_LC | . | . | . | . | . | . | P | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | F | . | R | . | . | . | Q | . | . | . |
| 21-225_26G11_LC | . | . | . | . | . | . | L | . | . | . | . | . | . | . | . | . |
| 21-225_26D12_LC | . | . | . | . | . | . | F | . | R | . | . | . | Q | . | . | . |
| 21-225_25B3_LC | . | . | . | . | . | . | F | . | R | . | . | . | Q | . | . | . |
| 21-225_25F3_LC | . | . | . | . | . | . | F | . | R | . | . | . | Q | . | . | . |
| 21-225_3F8_LC | . | . | . | . | . | . | . | . | . | . | . | . | . | . | . | R |
| 21-225_7E11_LC | . | . | . | . | . | . | . | . | . | . | . | . | . | . | . | . |
| 21-225_6G1_LC | . | . | . | . | . | . | P | . | . | . | . | . | . | . | . | . |
| 21-225_15A3_LC | . | . | . | . | . | . | . | . | . | . | . | . | . | . | . | . |
| 21-225_13D4_LC | . | . | . | . | . | . | L | . | . | . | . | . | . | . | . | . |
| 21-225_16E6_LC | . | . | . | . | . | . | . | . | . | . | . | . | . | . | . | . |

| Reference # | 145 | 146 | 147 | 148 | 149 |
|---|---|---|---|---|---|
| CONSENSUS | V | E | I | K | R |
| 21-225_20F6_LC | . | . | . | . | . |
| 21-225_18G4_LC | . | . | . | . | . |
| 21-225_22C7_LC | . | . | . | . | . |
| 21-225_27A11_LC | . | . | . | . | . |
| 21-225_26G11_LC | . | . | . | . | . |
| 21-225_26D12_LC | . | . | . | . | . |
| 21-225_25B3_LC | . | . | . | . | . |
| 21-225_25F3_LC | . | . | . | . | . |
| 21-225_3F8_LC | . | . | . | N | . |
| 21-225_7E11_LC | . | . | . | . | . |
| 21-225_6G1_LC | . | . | . | . | . |
| 21-225_15A3_LC | . | . | . | . | . |
| 21-225_13D4_LC | . | . | . | . | . |
| 21-225_16E6_LC | . | . | . | . | . |

**FIGURE 56**

Table 44

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | K_FR1 | | | | | | | | |
| CONSENSUS | D | I | Q | M | T | Q | S | P | S | S | L | S | A | S | V | G | D |
| 21-225_6E5_LC | . | . | . | . | . | . | . | . | . | . | . | . | . | . | . | . | . |
| 21-225_17H3_LC | . | . | . | . | . | . | . | . | . | . | . | . | . | . | . | . | . |
| 21-225_20B2_LC | . | . | . | . | . | . | . | . | . | . | . | . | . | . | . | . | . |
| 21-225_22F4_LC | . | . | . | . | . | . | . | . | . | . | . | . | . | . | . | . | . |
| 21-225_24B6_LC | . | . | . | . | . | . | . | . | . | . | . | . | . | . | . | . | . |
| 21-225_23A10_LC | . | . | . | . | . | . | . | . | . | . | . | . | . | . | . | . | . |
| 21-225_26A2_LC | . | V | . | N | . | T | . | L | . | . | . | . | V | T | P | . | Q |
| 21-225_25D12_LC | . | . | . | . | . | . | . | . | . | . | . | . | . | . | . | . | . |
| 21-225_32H2_LC | . | V | . | . | . | T | . | L | . | . | . | . | V | T | P | . | Q |
| 21-225_29H4_LC | . | V | . | . | . | T | . | L | . | . | . | . | V | T | P | . | Q |
| 21-225_26A11_LC | . | . | . | . | . | . | . | I | . | . | . | . | . | . | . | . | . |
| 21-225_26G7_LC | . | L | . | . | . | T | . | L | . | . | . | . | V | T | P | . | Q |
| 21-225_4C5_LC | . | . | . | . | . | . | . | . | . | . | . | . | . | . | . | . | H |
| 21-225_14E10_LC | . | . | . | . | . | . | . | . | . | . | . | . | . | . | . | . | . |
| 21-225_10E9_LC | . | . | . | . | . | . | . | . | . | . | . | . | . | . | . | . | . |
| 21-225_2G4_LC | . | . | . | . | . | . | . | . | . | . | . | . | . | . | . | . | . |
| 21-225_7C9_LC | . | . | . | . | . | . | . | . | . | . | . | . | . | . | G | . | . |
| 21-225_4C12_LC | . | . | . | . | . | . | . | . | . | . | . | . | . | . | . | . | . |
| 21-225_7G4_LC | . | . | . | . | . | . | . | . | . | . | . | . | . | . | . | . | . |
| 21-225_15C11_LC | . | . | . | . | . | . | . | . | . | . | . | . | . | . | . | . | . |

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | L_FR1 | | | | | | | | |
| CONSENSUS | S | Y | E | L | | Q | P | - | P | S | V | S | V | S | P | G | Q |
| 21-225_27E7_LC | . | . | . | . | T | . | . | . | . | . | . | . | . | . | . | . | . |
| 21-225_3H10_LC | . | . | . | . | S | . | . | . | . | . | . | . | . | . | . | . | . |

3600

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | K_CDR1 | |
| CONSENSUS | R | V | T | I | T | C | R | A | S | - | - | Q | G | I | R | - | - |
| 21-225_6E6_LC | . | . | . | . | . | . | . | . | . | . | . | . | . | . | . | . | . |
| 21-225_17H3_LC | . | . | . | F | . | . | . | . | . | . | . | . | . | . | . | . | . |
| 21-225_20B2_LC | . | . | N | . | . | . | . | . | . | . | . | . | D | . | . | . | . |
| 21-225_22F4_LC | . | . | . | . | . | . | . | . | . | . | . | . | V | . | . | . | . |
| 21-225_24B6_LC | . | . | . | . | . | . | . | . | . | . | . | . | D | . | . | . | . |
| 21-225_23A10_LC | . | . | . | . | . | . | . | . | . | . | . | . | . | . | . | . | . |
| 21-225_25A2_LC | P | A | S | . | S | . | K | S | . | . | . | . | S | L | L | H | S |
| 21-225_25D12_LC | . | . | . | . | . | . | . | T | . | . | . | . | . | . | . | . | . |
| 21-225_32H2_LC | P | A | S | . | S | . | . | S | . | . | . | . | S | L | L | H | S |
| 21-225_29H4_LC | P | A | S | F | S | . | K | S | . | . | . | . | S | L | L | H | S |
| 21-225_26A11_LC | . | . | . | . | . | . | . | . | . | . | . | . | A | . | . | . | . |
| 21-225_28G7_LC | P | A | S | . | S | . | K | S | . | . | . | . | S | L | L | H | S |
| 21-225_4C5_LC | . | . | H | L | . | . | . | . | . | . | . | R | . | . | . | . | . |
| 21-225_14E10_LC | . | . | . | . | . | . | . | T | . | . | . | . | . | . | . | . | . |
| 21-225_10E9_LC | . | . | . | F | . | . | . | . | . | . | . | . | D | . | . | . | . |
| 21-225_2G4_LC | . | . | . | . | . | . | . | . | . | . | . | . | . | . | . | . | . |
| 21-225_7C9_LC | . | . | . | . | . | . | . | . | . | . | . | . | . | . | . | . | . |
| 21-225_4C12_LC | . | . | . | . | . | . | . | . | . | . | . | . | . | . | . | . | . |
| 21-225_7G4_LC | . | . | . | . | . | . | . | . | . | . | . | . | . | . | . | . | . |
| 21-225_16C11_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | L_CDR1 | |
| CONSENSUS | T | A | S | I | T | C | S | G | D | - | - | - | K | L | G | - | - |
| 21-225_27E7_LC | . | . | . | . | . | . | . | . | . | . | . | . | . | . | . | N | . |
| 21-225_3H10_LC | . | . | . | . | . | . | . | . | . | . | . | . | . | . | . | E | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | D | L | G | W | Y | Q | Q | K | P | G | K | A |
| 21-225_5E5_LC | . | . | . | . | . | . | . | . | . | . | . | . | . | . | . | . | . |
| 21-225_17H3_LC | . | . | . | . | S | . | . | . | . | . | . | . | . | . | . | . | . |
| 21-225_20B2_LC | . | . | . | . | . | . | . | . | . | . | . | . | . | . | . | . | . |
| 21-225_22F4_LC | . | . | . | . | D | . | . | . | . | . | . | . | . | . | . | . | . |
| 21-225_24B6_LC | . | . | . | . | . | . | . | . | . | . | . | . | . | . | . | . | . |
| 21-225_23A10_LC | . | . | . | . | . | . | . | . | . | . | . | . | . | . | . | . | . |
| 21-225_25A2_LC | E | . | G | K | T | Y | . | Y | . | . | L | . | . | . | . | Q | P |
| 21-225_25D12_LC | . | . | . | . | . | . | . | . | . | . | . | . | . | . | . | . | . |
| 21-225_32H2_LC | D | . | G | R | T | Y | . | Y | . | . | L | . | . | . | . | Q | P |
| 21-225_29H4_LC | D | . | G | K | T | Y | . | Y | . | . | L | . | . | . | . | Q | P |
| 21-225_26A11_LC | . | . | . | . | . | . | . | . | . | . | . | . | R | . | . | . | . |
| 21-225_26G7_LC | E | . | G | K | T | Y | . | Y | . | . | L | . | . | . | . | Q | P |
| 21-225_4C5_LC | . | . | . | . | G | . | . | . | . | . | R | . | . | . | . | . | . |
| 21-225_14E10_LC | . | . | . | . | . | . | . | . | . | . | . | H | . | . | . | Q | . |
| 21-225_10E9_LC | . | . | . | . | S | . | . | . | . | . | . | . | . | . | . | . | . |
| 21-225_2G4_LC | . | . | . | . | . | . | . | . | . | . | . | . | . | . | . | . | . |
| 21-225_7C9_LC | . | . | . | . | . | . | . | . | . | . | . | . | . | . | . | . | . |
| 21-225_4C12_LC | . | . | . | . | . | . | . | . | . | . | . | . | . | . | . | . | . |
| 21-225_7G4_LC | . | . | . | . | . | . | . | . | . | . | . | . | R | . | . | . | . |
| 21-225_16C11_LC | . | . | . | . | . | . | . | . | . | . | . | . | . | . | . | . | V |

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | L_FR2 | |
| CONSENSUS | - | - | - | - | K | Y | A | C | W | Y | Q | Q | K | P | G | Q | S |
| 21-225_27E7_LC | . | . | . | . | . | . | . | . | . | . | . | . | . | . | . | . | . |
| 21-225_3H10_LC | . | . | . | . | . | . | . | . | . | . | . | . | . | . | . | . | . |

3602

**FIGURE 56**

**FIGURE 56**

**FIGURE 56**

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | K_FR3 | | | | | | | | | | | | | | |
| CONSENSUS | - | T | E | F | T | L | T | I | S | S | L | Q | P | E | D | F | A |
| 21-225_5E5_LC | . | . | . | . | . | . | . | . | . | . | . | . | . | . | . | . | . |
| 21-225_17H3_LC | . | . | . | . | . | . | . | . | . | . | . | . | . | . | . | . | . |
| 21-225_20B2_LC | . | . | . | . | . | . | . | . | . | . | . | . | . | . | . | . | . |
| 21-225_22F4_LC | . | . | D | . | . | . | . | . | . | . | . | . | . | . | . | . | . |
| 21-225_24B6_LC | . | . | . | . | . | . | . | . | . | . | . | . | T | . | . | . | . |
| 21-225_23A10_LC | . | . | . | . | . | . | . | . | G | . | . | . | . | . | . | . | . |
| 21-225_25A2_LC | . | . | D | . | . | . | K | . | . | R | V | E | A | . | . | V | G |
| 21-225_26D12_LC | . | . | . | . | . | . | . | . | . | . | . | . | . | . | . | . | . |
| 21-225_32H2_LC | . | . | D | . | . | . | K | . | . | R | V | E | A | . | . | V | G |
| 21-225_29H4_LC | . | . | D | . | . | . | K | . | . | R | V | E | A | . | . | V | G |
| 21-225_26A11_LC | . | . | . | . | I | . | . | . | . | . | . | . | . | . | . | . | . |
| 21-225_26G7_LC | . | . | D | . | . | . | K | . | . | R | V | E | A | . | . | V | G |
| 21-225_4C5_LC | . | . | . | . | . | . | . | . | G | . | . | . | . | . | . | . | . |
| 21-225_14E10_LC | . | . | . | . | . | . | . | . | . | . | . | . | . | . | . | . | T |
| 21-225_10E9_LC | . | . | . | . | . | . | . | N | . | . | . | . | . | . | . | . | . |
| 21-225_2G4_LC | . | . | . | . | . | . | . | . | . | . | . | . | . | . | . | L | . |
| 21-225_7C9_LC | . | . | . | . | . | . | . | . | . | . | . | . | . | . | . | . | . |
| 21-225_4C12_LC | . | . | . | . | . | . | . | . | . | . | . | . | . | . | . | L | . |
| 21-225_7G4_LC | . | . | . | . | . | . | . | . | . | . | . | . | . | . | . | S | . |
| 21-225_15C11_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | L_FR3 | | | | | | | | | | | | | | |
| CONSENSUS | - | N | | A | T | L | T | I | S | G | T | Q | A | M | D | E | A |
| 21-225_27E7_LC | . | . | T | . | . | . | . | . | . | . | . | . | . | . | . | . | . |
| 21-225_3H10_LC | . | . | K | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | Y | S | - | - | - | - | - | - | - | - |
| 21-225_5E5_LC | . | . | . | . | | | | | | | | | | | | | |
| 21-225_17H3_LC | . | . | . | . | . | . | Q | | | | | | | | | | |
| 21-225_20B2_LC | . | . | . | . | . | . | . | N | | | | | | | | | |
| 21-225_22F4_LC | . | . | . | . | . | H | . | . | | | | | | | | | |
| 21-225_24B6_LC | . | . | . | . | . | . | . | . | | | | | | | | | |
| 21-225_23A10_LC | . | . | . | . | . | . | . | . | | | | | | | | | |
| 21-225_25A2_LC | V | . | . | . | M | . | S | T | Q | . | . | . | . | . | . | . | . |
| 21-225_25D12_LC | . | . | . | . | | | | | | | | | | | | | |
| 21-225_32H2_LC | I | . | S | . | . | . | S | I | Q | . | . | . | . | . | . | . | . |
| 21-225_29H4_LC | V | . | . | . | M | . | S | I | Q | . | . | . | . | . | . | . | . |
| 21-225_26A11_LC | . | . | . | . | . | . | . | N | | | | | | | | | |
| 21-225_26G7_LC | V | . | . | . | M | . | S | I | Q | . | . | . | . | . | . | . | . |
| 21-225_4C5_LC | . | . | . | . | . | . | . | . | | | | | | | | | |
| 21-225_14E10_LC | A | . | . | . | . | . | . | . | | | | | | | | | |
| 21-225_10E8_LC | . | . | . | . | . | | | | | | | | | | | | |
| 21-225_2G4_LC | . | . | . | . | . | . | . | . | | | | | | | | | |
| 21-225_7C9_LC | . | . | . | . | . | . | L | . | | | | | | | | | |
| 21-225_4C12_LC | . | . | . | . | . | . | . | . | | | | | | | | | |
| 21-225_7G4_LC | . | . | . | . | . | H | Q | . | | | | | | | | | |
| 21-225_15C11_LC | . | . | . | . | . | L | . | | | | | | | | | | |

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | D | Y | Y | C | Q | A | W | | - | - | - | - | - | - | - | - | - |
| 21-225_27E7_LC | . | . | . | . | . | . | D | S | . | . | . | . | . | . | . | . | |
| 21-225_3H10_LC | . | . | . | . | . | . | V | N | . | . | . | . | . | . | . | . | |

**FIGURE 56**

FIGURE 56

FIGURE 56

Table 45

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K_FR1 | | | | | |
| CONSENSUS | D | I | Q | M | T | Q | S | P | S | S | L | S | A | S | V | G | D |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | . | I | . | . | . | . | . | . | F | . | C | . | . |
| 21-225_4C8_LC | . | . | . | . | . | I | . | . | . | . | F | . | F | . | C | . | . |
| 21-225_4F12_LC | . | . | . | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | R | - | - |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | D | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | T | . | . | . | . | . | . | . | . | . | . |
| 21-225_15B11_LC | . | . | . | . | . | . | . | . | . | . | . | . | . | . | . | . | . |
| 21-225_14C2_LC | . | . | I | . | . | . | . | . | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | D | L | G | W | Y | Q | Q | K | P | G | K | A |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | F | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | H | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | . | . | . | F | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | D | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | K_CDR2 | | | | | |
| CONSENSUS | P | K | R | L | I | Y | A | - | - | - | - | - | - | - | - | A | S |
| 21-225_18H12_LC | . | . | . | . | . | . | T | . | . | . | . | . | . | . | . | . | . |
| 21-225_22B7_LC | . | . | . | . | . | . | T | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | T | . | . | . | . | . | . | . | . | . | . |
| 21-225_30F3_LC | . | . | . | . | . | . | T | . | . | . | . | . | . | . | . | . | . |
| 21-225_33B1_LC | . | . | . | . | . | . | T | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | . | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | T | . | . | . | . | . | . | . | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_18H12_LC | N | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | N | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | . | . | . | . | . | . | . | . | . | . |
| 21-225_4C8_LC | . | . | . | . | . | . | . | . | . | . | . | . | . | V | . | . | . |
| 21-225_4F12_LC | . | . | . | . | . | . | . | . | . | . | . | . | G | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | K_FR3 | | | | | | | | | | | | | | |
| CONSENSUS | - | T | E | F | T | L | T | I | S | S | L | Q | P | E | D | F | A |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | V | . | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | D | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | . | . | . | V | . | R | . | . | . | . |
| 21-225_4C8_LC | . | . | . | . | . | . | . | . | . | . | . | . | . | . | . | . | . |
| 21-225_4F12_LC | . | . | I | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | N | S | - | - | - | - | - | - | - | - |
| 21-225_18H12_LC | . | . | . | . | . | . | . | . | N | . | . | . | . | . | . | . | . |
| 21-225_22B7_LC | . | . | . | . | . | . | . | . | T | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | T | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | R | . | . | . | . | . | . | . | . |
| 21-225_29D8_LC | . | . | . | . | . | . | . | . | . | . | . | . | . | . | . | . | . |
| 21-225_30F3_LC | S | . | . | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | V | . | . | . | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | . |
| 21-225_15H10_LC | S | . | . | . | . | . | . | . | . | . | . | . | . | . | . | . | . |
| 21-225_18H4_LC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | . | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | A |
| 21-225_15H10_LC | . | . | . | . | . | . | . | . | . | . | . | . | . | . | . | . | . |
| 21-225_18H4_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | K_FR4 | | | | | | |
| CONSENSUS | F | T | F | G | | G | T | K | V | E | I | K | R |
| 21-225_18H12_LC | W | . | . | . | L | . | . | . | . | V | . | . | . |
| 21-225_22B7_LC | L | . | . | . | G | . | . | . | . | . | . | . | . |
| 21-225_25A9_LC | . | . | . | . | P | . | . | . | . | D | . | . | . |
| 21-225_29D9_LC | . | . | . | . | P | . | . | . | . | D | F | . | . |
| 21-225_25G5_LC | . | . | . | . | P | . | . | . | . | D | . | . | . |
| 21-225_28B8_LC | . | . | . | . | P | . | . | . | . | D | . | N | . |
| 21-225_28A9_LC | . | . | . | . | P | . | . | . | . | D | . | . | . |
| 21-225_29D8_LC | . | . | . | . | P | . | . | . | . | D | . | . | . |
| 21-225_30F3_LC | . | . | . | . | G | . | . | . | . | . | . | . | . |
| 21-225_33B1_LC | L | . | . | . | G | . | . | . | . | . | V | . | . |
| 21-225_15B11_LC | . | . | . | . | P | . | . | . | . | D | . | . | . |
| 21-225_14C2_LC | L | . | . | . | G | . | . | . | . | . | . | R | . |
| 21-225_4C8_LC | L | . | . | . | G | . | . | . | . | . | . | . | . |
| 21-225_4F12_LC | L | . | . | . | G | . | . | . | . | . | . | . | . |
| 21-225_15H10_LC | L | . | . | . | G | . | . | . | . | . | . | N | . |
| 21-226_16H4_LC | W | . | . | . | Q | . | . | . | . | . | . | . | . |

FIGURE 56

Table 46

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | K_FR1 | | | | | | | | | |
| CONSENSUS | D | I | Q | M | T | Q | S | P | S | S | L | S | A | S | V | G | D |
| 21-225_16F10_LC | . | . | V | . | . | . | T | . | L | . | . | . | V | I | P | . | Q |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E6_LC | . | . | . | . | . | A | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | F | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | V | . | . | . | T | . | L | . | . | . | V | T | P | . | Q |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_26C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

# EP 4 435 105 A2

**FIGURE 56**

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K_FR1 | | | | | |
| CONSENSUS | D | I | Q | M | T | Q | S | P | S | S | L | S | A | S | V | G | D |
| 21-225_25H11_LC | . | . | . | . | . | . | . | . | . | . | . | . | . | . | . | . | . |
| 21-225_27F2_LC | . | . | . | . | . | . | . | . | . | . | . | . | . | . | . | . | . |
| 21-225_27C3_LC | . | . | . | . | . | . | . | . | . | . | . | . | . | . | . | . | . |
| 21-225_29G8_LC | . | . | . | . | . | . | . | . | . | . | . | . | . | . | . | . | . |
| 21-225_5A11_LC | . | . | . | . | . | . | . | . | . | . | . | . | . | . | . | . | . |
| 21-225_6G12_LC | . | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | . | . | . | . | . | . | . | . | . | . | R | . | . | F | . | . | . |
| 21-225_7H11_LC | . | . | . | . | . | . | . | . | . | . | A | . | . | . | . | . | V |
| 21-225_14E3_LC | . | . | . | . | . | . | . | . | . | . | . | . | . | . | . | . | . |
| 21-225_9A1_LC | . | . | . | . | . | . | . | . | . | . | R | . | . | F | . | . | . |
| 21-225_15E3_LC | . | . | . | . | . | . | . | . | . | . | . | . | . | . | . | . | . |
| 21-225_16F4_LC | . | . | . | . | I | . | . | . | . | . | P | . | . | . | . | . | . |
| 21-225_16F6_LC | . | . | . | . | . | . | . | . | . | . | . | . | . | . | . | . | . |
| 21-225_2B2_LC | . | . | . | . | . | . | . | . | . | . | . | . | . | . | . | . | . |
| 21-225_13E6_LC | . | . | . | V | . | . | . | . | . | . | . | . | . | . | . | . | . |
| 21-225_16A5_LC | . | . | . | . | . | . | . | . | . | . | . | . | . | . | . | . | . |
| 21-225_7G2_LC | . | . | . | . | . | . | . | . | . | . | . | . | . | . | . | . | . |
| 21-225_7C4_LC | . | . | . | . | . | . | . | . | . | . | . | F | . | . | . | . | . |
| 21-225_7E9_LC | . | . | . | . | . | . | . | . | . | . | . | F | . | . | . | . | . |
| 21-225_8C9_LC | . | . | . | . | . | . | . | . | . | . | . | . | . | . | I | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | F | . | . | . | . | . |
| 21-225_12F2_LC | . | . | . | . | . | . | . | . | . | . | . | . | . | . | . | . | . |
| 21-225_12E6_LC | . | . | . | . | . | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

3615

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | R | - | - |
| 21-225_16F10_LC | P | A | S | . | S | . | K | S | . | . | . | . | S | L | L | H | S |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | P | A | S | . | S | . | K | S | . | . | . | . | S | L | L | H | S |
| 21-225_23H11_LC | . | . | . | . | . | . | . | . | . | . | . | . | . | . | . | . | . |
| 21-225_21F7_LC | . | . | . | . | . | . | . | . | . | . | . | . | D | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | R | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | R | - | - |
| 21-225_25H11_LC | . | . | . | . | . | . | . | . | . | . | . | . | . | . | . | . | . |
| 21-225_27F2_LC | . | . | . | . | . | . | . | . | . | . | . | . | . | . | . | . | . |
| 21-225_27C3_LC | . | . | . | . | . | . | . | . | . | . | . | . | . | . | . | . | . |
| 21-225_29G8_LC | . | . | . | . | . | . | . | . | . | . | . | . | . | . | . | . | . |
| 21-225_6A11_LC | . | . | . | . | . | . | . | . | . | . | . | . | . | . | . | . | . |
| 21-225_6G12_LC | . | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G6_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | . | . | . | . | . | Y | . | . | . | . | . | . | . | . | . | . | . |
| 21-225_7H11_LC | . | . | . | . | I | . | L | . | . | . | . | . | . | . | . | . | . |
| 21-225_14E3_LC | . | . | . | . | . | . | . | . | . | . | . | . | . | . | . | . | . |
| 21-225_9A1_LC | . | . | . | . | . | . | . | . | . | . | . | . | . | . | . | . | . |
| 21-225_15E3_LC | . | . | . | . | . | . | . | . | . | . | . | . | . | . | . | . | . |
| 21-225_16F4_LC | . | . | . | . | . | . | . | . | . | . | . | . | . | . | . | . | . |
| 21-225_16F6_LC | . | . | . | . | . | . | Q | . | N | . | . | . | D | . | T | . | . |
| 21-225_2B2_LC | . | . | . | . | . | . | . | . | . | . | . | . | . | . | . | . | . |
| 21-225_13E6_LC | . | . | . | . | . | S | . | . | . | . | . | . | . | . | . | . | . |
| 21-225_16A5_LC | . | . | . | . | . | . | . | . | . | . | . | . | . | . | . | . | . |
| 21-225_7G2_LC | . | . | . | . | . | . | . | . | . | . | . | . | . | . | . | . | . |
| 21-225_7C4_LC | . | . | . | . | . | . | . | . | . | . | . | . | . | . | . | . | . |
| 21-225_7E9_LC | . | . | . | . | . | . | . | . | . | . | . | . | . | . | . | . | . |
| 21-225_8C9_LC | . | . | . | . | . | . | . | . | . | . | . | . | A | . | . | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | . | . | . | . | . | . |
| 21-225_12F2_LC | . | . | . | . | . | . | . | . | . | . | . | . | . | . | . | . | . |
| 21-225_12E6_LC | . | . | . | . | . | . | . | . | . | . | . | . | . | . | . | . | . |
| 21-225_18G7_LC | . | . | . | . | . | . | . | . | . | . | . | . | . | . | . | . | . |
| 21-225_1C12_LC | . | . | . | . | . | . | . | . | . | . | . | . | . | . | . | . | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | D | L | G | W | Y | Q | Q | K | P | G | K | A |
| 21-225_16F10_LC | D | . | G | K | T | H | . | N | . | . | L | . | . | . | . | Q | P |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | D | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | D | . | G | K | T | F | . | Y | . | . | L | . | . | . | . | H | P |
| 21-225_23H11_LC | . | . | . | . | . | . | . | D | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | S | . | . | . | . | . | . | . | . | . | . | . | . |
| 21-225_29H6_LC | . | . | . | . | . | . | . | . | . | . | . | . | . | S | . | . | T |
| 21-225_25E8_LC | . | . | . | . | . | . | . | . | . | . | . | R | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_28B8_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | K_FR2 | | | | | | | | |
| CONSENSUS | - | - | - | - | N | D | L | G | W | Y | Q | Q | K | P | G | K | A |
| 21-225_25H11_LC | . | . | . | . | T | . | . | . | . | . | . | . | . | . | . | . | . |
| 21-225_27F2_LC | . | . | . | . | . | . | . | . | . | . | . | . | . | . | . | . | . |
| 21-225_27C3_LC | . | . | . | . | . | . | . | . | . | . | . | . | . | . | . | . | . |
| 21-225_29G6_LC | . | . | . | . | . | . | . | . | . | . | . | L | . | . | . | . | . |
| 21-225_6A11_LC | . | . | . | . | . | . | . | . | . | . | . | . | . | . | . | . | . |
| 21-225_6G12_LC | . | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | . | . | . | . | . | . | . | . | . | . | . | . | . | . | . | . | . |
| 21-225_7H11_LC | . | . | . | . | . | . | . | . | . | . | . | . | . | . | . | . | . |
| 21-225_14E3_LC | . | . | . | . | . | . | . | . | . | . | . | . | . | . | . | . | . |
| 21-225_9A1_LC | . | . | . | . | . | . | . | . | . | . | . | . | . | . | . | . | . |
| 21-225_15E3_LC | . | . | . | . | . | . | . | . | . | . | . | . | . | . | . | . | . |
| 21-225_16F4_LC | . | . | . | . | . | . | . | . | . | . | . | . | . | . | S | . | . |
| 21-225_16F6_LC | . | . | . | . | . | F | . | N | . | . | . | L | . | . | . | . | . |
| 21-225_2B2_LC | . | . | . | . | . | . | . | . | . | . | . | . | . | . | . | Q | . |
| 21-225_13E6_LC | . | . | . | . | . | . | . | . | . | . | . | . | . | . | . | . | . |
| 21-225_16A5_LC | . | . | . | . | . | . | . | . | C | . | . | . | . | . | . | . | . |
| 21-225_7G2_LC | . | . | . | . | . | . | . | . | . | . | . | . | . | . | . | . | . |
| 21-225_7C4_LC | . | . | . | . | . | . | . | . | . | . | . | . | . | . | . | . | . |
| 21-225_7E9_LC | . | . | . | . | . | . | . | . | . | E | . | . | . | . | . | . | . |
| 21-225_8C8_LC | . | . | . | . | . | . | . | D | . | . | . | . | . | . | . | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | . | . | . | . | . | . |
| 21-225_12F2_LC | . | . | . | . | . | . | . | . | . | . | . | . | . | . | . | . | . |
| 21-225_12E6_LC | . | . | . | . | S | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | R | L | I | Y | A | - | - | - | - | - | - | - | - | A | S |
| 21-225_16F10_LC | . | Q | L | . | . | . | E | . | . | . | . | . | . | . | . | V | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | T | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B6_LC | . | . | . | . | . | . | . | . | . | . | . | . | . | . | . | . | . |
| 21-225_17E5_LC | . | . | . | . | . | . | T | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | T | . | . | . | . | . | . | . | . | . | . |
| 21-225_20E12_LC | . | . | . | . | . | F | . | . | . | . | . | . | . | . | . | . | . |
| 21-225_20D10_LC | R | . | . | . | . | . | V | . | . | . | . | . | . | . | . | . | Y |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | T | . |
| 21-225_22H8_LC | . | Q | L | . | . | . | E | . | . | . | . | . | . | . | . | V | . |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | T | . | . | . | . | . | . | . | . | S | . |
| 21-225_22F2_LC | . | . | . | . | . | . | . | . | . | . | . | . | . | . | . | . | . |
| 21-225_23F12_LC | . | . | . | . | . | . | V | . | . | . | . | . | . | . | . | . | . |
| 21-225_28F5_LC | . | . | . | . | . | . | T | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | I | . | . | . | . | . | . | . | . | . | . |
| 21-225_25B6_LC | . | . | . | . | . | . | R | . | . | . | . | . | . | . | . | . | . |
| 21-225_26A2_LC | . | . | . | . | . | . | T | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | . | . | . | . | . | F |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

## FIGURE 56

| Reference # | | | | | | | | | | | | | K_CDR2 | | | | |
|---|---|---|---|---|---|---|---|---|---|---|---|---|---|---|---|---|---|
| CONSENSUS | P | K | R | L | I | Y | A | - | - | - | - | - | - | - | - | - | A | S |
| 21-225_25H11_LC | . | . | . | . | . | . | . | . | . | . | . | . | . | . | . | . | . | . |
| 21-225_27F2_LC | . | . | . | . | . | . | V | . | . | . | . | . | . | . | . | . | . | . |
| 21-225_27C3_LC | . | . | . | . | . | . | . | . | . | . | . | . | . | . | . | . | . | . |
| 21-225_29G8_LC | . | . | . | . | . | . | . | . | . | . | . | . | . | . | . | . | . | . |
| 21-225_6A11_LC | . | . | . | . | . | . | . | . | . | . | . | . | . | . | . | . | . | . |
| 21-225_6G12_LC | . | . | . | . | . | . | . | . | . | . | . | . | . | . | . | . | . | . |
| 21-225_8H7_LC | . | . | . | . | . | . | . | . | . | . | . | . | . | . | . | . | . | . |
| 21-225_10G5_LC | . | . | . | . | . | . | . | . | . | . | . | . | . | . | . | . | . | . |
| 21-225_10H12_LC | . | . | . | . | . | . | G | . | . | . | . | . | . | . | . | . | . | . |
| 21-225_12A1_LC | . | . | . | . | . | . | . | . | . | . | . | . | . | . | . | . | . | . |
| 21-225_8G2_LC | . | Q | . | . | . | . | . | . | . | . | . | . | . | . | . | . | . | . |
| 21-225_7H11_LC | . | . | . | I | . | . | V | . | . | . | . | . | . | . | . | . | . | C |
| 21-225_14E3_LC | . | . | . | I | . | . | S | . | . | . | . | . | . | . | . | . | . | . |
| 21-225_9A1_LC | R | . | . | . | . | . | T | . | . | . | . | . | . | . | . | . | . | . |
| 21-225_15E3_LC | . | . | . | . | . | . | . | . | . | . | . | . | . | . | . | . | . | . |
| 21-225_16F4_LC | . | . | . | . | . | . | V | . | . | . | . | . | . | . | . | . | . | . |
| 21-225_16F6_LC | . | N | L | . | . | S | D | . | . | . | . | . | . | . | . | . | . | . |
| 21-225_2B2_LC | . | . | . | . | . | . | . | . | . | . | . | . | . | . | . | . | . | . |
| 21-225_13E8_LC | . | . | . | . | . | . | . | . | . | . | . | . | . | . | . | . | . | . |
| 21-225_16A5_LC | . | . | . | . | . | . | . | . | . | . | . | . | . | . | . | . | . | . |
| 21-225_7G2_LC | . | . | . | . | . | . | T | . | . | . | . | . | . | . | . | . | . | . |
| 21-225_7C4_LC | H | . | . | . | . | . | . | . | . | . | . | . | . | . | . | . | . | . |
| 21-225_7E9_LC | . | . | . | . | . | . | T | . | . | . | . | . | . | . | . | . | . | . |
| 21-225_8C9_LC | . | . | . | . | . | . | . | . | . | . | . | . | . | . | . | . | . | . |
| 21-225_12B12_LC | . | . | . | . | . | . | T | . | . | . | . | . | . | . | . | . | . | . |
| 21-226_16B7_LC | . | . | . | . | . | . | . | . | . | . | . | . | . | . | . | . | . | . |
| 21-225_9E8_LC | . | . | . | . | . | . | . | . | . | . | . | . | . | . | . | . | . | . |
| 21-225_12F2_LC | . | . | . | . | . | . | . | . | . | . | . | . | . | . | . | . | . | . |
| 21-225_12E8_LC | . | . | . | . | . | . | . | . | . | . | . | . | . | . | . | . | . | . |
| 21-225_16G7_LC | . | . | . | . | . | . | . | . | . | . | . | . | . | . | . | . | . | . |
| 21-226_1C12_LC | . | . | . | . | . | . | . | . | . | . | . | . | . | . | . | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_16F10_LC | Y | R | F | . | . | . | . | D | . | . | . | . | . | . | . | . | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | N | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | V | . | . | . | . | . | . | . | . | . | . | . | . | . | . | . |
| 21-225_18C2_LC | . | . | . | . | . | . | . | . | . | . | . | . | . | . | F | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B6_LC | N | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D6_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | Y | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | R | . | . | . | . | . | . |
| 21-225_21E5_LC | . | . | . | . | . | . | . | . | . | . | . | . | . | . | . | . | . |
| 21-225_22H8_LC | N | R | F | . | . | . | . | D | . | . | . | . | . | . | . | . | . |
| 21-225_23H11_LC | . | . | . | . | . | . | . | . | . | . | . | . | . | . | . | . | . |
| 21-225_21F7_LC | N | F | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E8_LC | . | . | . | . | . | . | . | . | . | . | R | . | . | . | . | . | . |
| 21-225_26H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | R | . | . | . |

**FIGURE 56**

| Reference # | | | | | | | | | | | | | | | | | |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_25H11_LC | . | . | . | . | . | . | . | . | . | . | . | . | . | . | . | . | . |
| 21-225_27F2_LC | . | . | . | . | . | . | . | . | . | . | . | . | . | . | . | . | . |
| 21-225_27C3_LC | . | . | . | . | . | . | . | . | . | . | . | . | . | . | . | . | . |
| 21-225_29G8_LC | . | . | . | . | . | . | . | . | . | . | . | . | . | . | . | . | . |
| 21-225_6A11_LC | . | . | . | . | . | . | . | . | . | . | . | . | . | . | . | . | . |
| 21-225_6G12_LC | T | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | . | . | . | . | . | . | . | . | . | . | . | . | . | Y | . | . | . |
| 21-225_7H11_LC | . | F | . | . | . | . | . | . | . | . | . | . | . | Y | . | . | . |
| 21-225_14E3_LC | . | . | . | . | . | . | . | . | . | . | I | . | . | C | . | . | . |
| 21-225_9A1_LC | . | . | . | . | . | . | . | . | . | . | . | . | . | Y | . | . | . |
| 21-225_15E3_LC | . | S | . | . | . | . | . | . | . | . | . | . | R | . | . | . | . |
| 21-225_16F4_LC | . | . | . | . | . | . | . | . | . | . | . | . | . | . | . | . | . |
| 21-225_16F6_LC | N | . | E | T | . | . | . | . | . | . | . | . | . | . | . | . | . |
| 21-225_2B2_LC | . | . | . | . | . | . | . | . | . | . | . | . | . | . | . | . | . |
| 21-225_13E6_LC | . | . | . | . | . | . | . | . | . | . | . | . | . | . | . | . | . |
| 21-225_18A5_LC | . | . | . | . | . | . | . | . | . | . | . | N | . | Y | . | . | . |
| 21-225_7G2_LC | . | . | . | . | . | . | . | . | . | . | . | . | . | . | . | . | . |
| 21-225_7C4_LC | . | . | . | . | . | . | . | . | . | . | . | . | . | . | . | . | . |
| 21-225_7E9_LC | . | . | . | . | . | . | . | . | . | . | . | . | . | . | . | . | . |
| 21-225_8C9_LC | . | . | . | . | . | . | . | . | . | . | . | . | . | . | . | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | . | . | . | . | . | . |
| 21-225_12F2_LC | . | . | . | . | . | . | . | . | . | . | . | . | . | . | . | . | . |
| 21-225_12E6_LC | . | . | . | . | . | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

**FIGURE 56**

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | K_FR3 | | | | | | | | | | | | |
| CONSENSUS | - | T | E | F | T | L | T | I | S | S | L | Q | P | E | D | F | A |
| 21-225_18F10_LC | . | . | V | . | . | . | E | . | . | R | V | E | A | A | . | V | G |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_18F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | S | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | S | F | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | D | . | . | . | . | K | . | R | V | E | A | . | . | V | G |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F8_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B8_LC | . | . | . | . | . | . | . | . | . | . | . | . | . | . | . | . | . |
| 21-225_26A2_LC | . | . | . | . | . | . | . | F | . | . | . | . | . | . | . | . | . |
| 21-225_29H6_LC | . | . | . | . | . | . | . | . | . | . | . | . | . | . | . | . | . |
| 21-225_25E8_LC | . | . | . | . | . | . | . | . | N | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | | |
| CONSENSUS | - | T | E | F | T | L | T | I | S | S | L | Q | P | E | D | F | A |
| 21-225_18F10_LC | . | . | V | . | . | . | E | . | . | R | V | E | A | A | . | V | G |
| 21-225_18A5_LC | . | . | . | . | . | . | . | F | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_18F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | S | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | S | F | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | D | . | . | . | K | . | . | R | V | E | A | . | . | V | G |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F8_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B8_LC | . | . | . | . | . | . | . | . | . | . | . | . | . | . | . | . | . |
| 21-225_26A2_LC | . | . | . | . | . | . | . | F | . | . | . | . | . | . | . | . | . |
| 21-225_29H6_LC | . | . | . | . | . | . | . | . | . | . | . | . | . | . | . | . | . |
| 21-225_25E8_LC | . | . | . | . | . | . | . | . | N | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | N | S | - | - | - | - | - | - | - | - |
| 21-225_16F10_LC | V | . | . | . | F | . | S | I | Q | . | . | . | . | . | . | . | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | N | . | . | . | . | . | . | . | . |
| 21-225_20E12_LC | . | . | . | . | . | . | . | . | . | . | . | . | . | . | . | . | . |
| 21-225_20D10_LC | . | . | . | . | I | . | Q | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | I | . | . | . | M | . | S | I | Q | . | . | . | . | . | . | . | . |
| 21-225_23H11_LC | . | . | . | . | . | . | . | . | R | . | . | . | . | . | . | . | . |
| 21-225_21F7_LC | . | . | . | . | . | . | . | . | N | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | S | . | . | . | . | . | . | . | . | . | . | . | . | . | . | . | . |
| 21-225_28F5_LC | . | . | . | . | . | . | . | . | T | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | T | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | R | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | N | S | - | - | - | - | - | - | - | - |
| 21-225_25H11_LC | . | . | . | . | . | . | . | . | . | . | . | . | . | . | . | . | . |
| 21-225_27F2_LC | . | . | . | . | . | . | . | . | . | . | . | . | . | . | . | . | . |
| 21-225_27C3_LC | . | . | . | . | . | . | . | . | R | . | . | . | . | . | . | . | . |
| 21-225_29G8_LC | . | . | N | . | . | . | . | . | . | . | . | . | . | . | . | . | . |
| 21-225_6A11_LC | . | . | . | . | . | . | . | . | . | . | . | . | . | . | . | . | . |
| 21-226_8G12_LC | . | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | I | . | . | . | I | . | . | . | . | . | . | . | . | . | . | . | . |
| 21-225_7H11_LC | . | . | . | . | . | . | . | . | L | . | . | . | . | . | . | . | . |
| 21-226_14E3_LC | A | . | . | S | V | . | . | Y | . | . | . | . | . | . | . | . | . |
| 21-225_9A1_LC | . | . | . | . | I | . | . | . | . | . | . | . | . | . | . | . | . |
| 21-225_15E3_LC | A | . | . | . | . | . | . | H | . | . | . | . | . | . | . | . | . |
| 21-225_16F4_LC | . | . | . | . | I | . | . | . | . | . | . | . | . | . | . | . | . |
| 21-225_16F6_LC | . | . | . | . | Q | . | Y | D | N | . | . | . | . | . | . | . | . |
| 21-225_2B2_LC | . | . | H | . | . | . | . | . | . | . | . | . | . | . | . | . | . |
| 21-225_13E6_LC | . | . | . | . | V | . | . | . | . | . | . | . | . | . | . | . | . |
| 21-225_16A5_LC | . | . | . | . | . | . | . | . | . | . | . | . | . | . | . | . | . |
| 21-225_7G2_LC | . | . | . | . | . | . | . | . | . | . | . | . | . | . | . | . | . |
| 21-225_7C4_LC | . | . | . | . | . | . | . | . | . | . | . | . | . | . | . | . | . |
| 21-225_7E9_LC | . | . | . | . | . | . | . | . | . | . | . | . | . | . | . | . | . |
| 21-226_8C8_LC | . | . | . | . | . | . | . | . | . | . | . | . | . | . | . | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | . | . | . | . | . | . |
| 21-225_12F2_LC | . | . | . | . | . | . | . | . | . | . | . | . | . | . | . | . | . |
| 21-225_12E6_LC | . | . | . | . | . | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_16F10_LC | . | . | . | . | . | . | . | . | . | . | . | . | . | . | . | L | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_19A1_LC | . | . | . | . | . | . | . | . | . | . | . | . | . | . | . | . | . |
| 21-225_19E1_LC | . | . | . | . | . | . | . | . | . | . | . | . | . | . | . | F | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | . | . | . | . | . | . | . | . | . | . | . | . | . | L | . |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_26H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_16F10_LC | . | . | . | . | . | . | . | . | . | . | . | . | . | . | . | L | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_19A1_LC | . | . | . | . | . | . | . | . | . | . | . | . | . | . | . | . | . |
| 21-225_19E1_LC | . | . | . | . | . | . | . | . | . | . | . | . | . | . | . | F | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | . | . | . | . | . | . | . | . | . | . | . | . | L | . | . |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_26H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | K_FR4 | | | | | | | | |
| CONSENSUS | L | T | F | G | G | G | T | K | V | E | I | K | R |
| 21-225_16F10_LC | . | . | . | . | . | . | . | . | . | . | . | . | . |
| 21-225_18A5_LC | W | . | . | . | Q | . | . | . | . | . | . | . | . |
| 21-225_20E5_LC | . | . | . | . | . | . | . | . | . | . | . | . | . |
| 21-225_18A1_LC | . | . | . | . | . | . | . | . | . | . | . | . | . |
| 21-225_19E1_LC | . | . | . | . | . | . | . | . | . | . | . | . | . |
| 21-225_17A10_LC | . | . | . | . | . | . | . | . | . | . | . | . | . |
| 21-225_18E8_LC | . | . | . | . | . | . | . | . | . | . | . | . | . |
| 21-225_16F11_LC | . | . | . | . | . | . | . | . | . | . | . | . | . |
| 21-225_18C2_LC | W | . | . | . | L | . | . | . | . | V | . | . | . |
| 21-225_20A7_LC | . | . | . | . | . | . | . | . | . | . | . | . | . |
| 21-225_17C7_LC | W | . | . | . | Q | . | . | . | . | . | . | . | . |
| 21-225_18F2_LC | W | . | . | . | Q | . | . | . | . | . | . | . | . |
| 21-225_17B5_LC | . | . | . | . | . | . | . | . | . | . | . | . | . |
| 21-225_17E5_LC | . | . | . | . | P | . | . | . | . | V | . | . | . |
| 21-225_20D5_LC | F | . | . | . | . | . | . | . | . | . | . | . | . |
| 21-225_21G11_LC | W | . | . | . | L | . | . | . | . | V | . | . | . |
| 21-225_20E12_LC | F | . | . | . | . | . | . | . | . | . | . | . | . |
| 21-225_20D10_LC | W | . | . | . | Q | . | . | . | . | . | . | . | . |
| 21-225_22A4_LC | W | . | . | . | Q | . | . | . | . | . | . | . | . |
| 21-225_20H12_LC | . | . | . | . | . | . | . | . | . | . | . | . | . |
| 21-225_21H8_LC | W | . | . | . | Q | . | . | . | . | . | . | . | . |
| 21-225_21E5_LC | . | . | . | . | . | . | . | . | . | . | . | . | . |
| 21-225_22H8_LC | . | S | . | . | . | . | . | . | . | . | . | N | . |
| 21-225_23H11_LC | . | . | . | . | . | . | . | . | . | . | . | . | . |
| 21-225_21F7_LC | W | . | . | . | Q | . | . | . | . | . | . | . | . |
| 21-225_22F9_LC | . | . | . | . | . | . | . | . | . | . | . | . | . |
| 21-225_21G2_LC | W | . | . | . | L | . | . | . | . | V | . | . | . |
| 21-225_22F2_LC | . | . | . | . | . | . | . | . | . | . | . | . | . |
| 21-225_23F12_LC | W | . | . | . | Q | . | . | . | . | . | . | . | . |
| 21-225_28F5_LC | W | . | . | . | Q | . | . | . | . | . | V | . | . |
| 21-225_29G4_LC | . | . | . | . | . | . | . | . | . | . | . | T | . |
| 21-225_27D5_LC | F | . | . | . | P | . | . | . | . | D | . | . | . |
| 21-225_28C7_LC | W | . | . | . | Q | . | . | . | . | . | . | . | . |
| 21-225_25B6_LC | F | . | . | . | P | . | . | . | . | D | . | . | . |
| 21-225_26A2_LC | W | . | . | . | Q | . | . | . | . | . | . | . | . |
| 21-225_29H6_LC | . | . | . | . | . | . | . | . | . | . | . | . | . |
| 21-225_25E6_LC | . | . | . | . | . | . | . | . | . | . | . | . | . |
| 21-225_25H10_LC | . | . | . | . | . | . | . | . | . | . | . | . | . |
| 21-225_29D7_LC | F | . | . | . | P | . | . | . | . | D | . | . | . |
| 21-225_29B8_LC | . | . | . | . | . | . | . | . | . | . | . | . | . |

3630

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | K_FR4 | | | | | | |
| CONSENSUS | L | T | F | G | G | G | T | K | V | E | I | K | R |
| 21-225_25H11_LC | . | . | . | . | . | . | . | . | . | . | . | . | . |
| 21-225_27F2_LC | . | . | . | . | . | . | . | . | . | . | . | . | . |
| 21-225_27C3_LC | . | . | . | . | . | . | . | . | . | . | . | . | . |
| 21-225_29G8_LC | . | . | . | . | . | . | . | . | . | . | . | . | . |
| 21-225_6A11_LC | W | . | . | . | Q | . | . | . | . | . | . | . | . |
| 21-225_8G12_LC | . | . | . | . | . | . | . | . | . | . | . | . | . |
| 21-225_8H7_LC | F | . | . | . | . | . | . | . | . | . | . | . | . |
| 21-225_10G5_LC | . | . | . | . | . | . | . | . | . | . | . | . | . |
| 21-225_10H12_LC | . | . | . | . | . | . | . | . | . | . | . | . | . |
| 21-226_12A1_LC | . | . | . | . | . | . | . | . | . | . | . | . | . |
| 21-225_6G2_LC | W | . | . | . | Q | . | . | . | . | . | . | T | . |
| 21-225_7H11_LC | . | . | . | . | . | . | . | . | . | . | . | . | . |
| 21-225_14E3_LC | F | . | . | . | . | . | . | . | . | . | . | . | . |
| 21-225_9A1_LC | W | . | . | . | Q | . | . | . | . | . | . | . | . |
| 21-225_15E3_LC | . | . | . | . | . | . | . | E | . | . | . | Y | . |
| 21-226_16F4_LC | F | . | . | . | . | . | . | . | . | . | . | . | . |
| 21-225_16F6_LC | I | . | . | . | Q | . | . | R | L | . | . | . | . |
| 21-225_2B2_LC | . | . | . | . | . | . | . | . | . | . | . | . | . |
| 21-225_13E6_LC | . | . | . | . | . | . | . | . | . | A | . | . | . |
| 21-225_16A5_LC | W | . | . | . | Q | . | . | . | . | . | . | . | . |
| 21-225_7G2_LC | W | . | . | . | L | . | . | . | . | V | . | . | . |
| 21-225_7C4_LC | . | . | . | . | . | . | . | . | . | . | . | . | . |
| 21-225_7E9_LC | . | . | . | . | . | . | . | . | . | . | . | . | . |
| 21-225_8C9_LC | . | . | . | . | . | . | . | . | . | . | . | . | . |
| 21-225_12B12_LC | W | . | . | . | L | . | . | . | . | V | . | . | . |
| 21-225_16B7_LC | . | . | . | . | . | . | . | . | . | . | F | N | . |
| 21-225_9E9_LC | . | . | . | . | . | . | . | . | . | . | . | . | . |
| 21-225_12F2_LC | W | . | . | . | Q | . | . | . | . | . | . | . | . |
| 21-225_12E6_LC | W | . | . | . | Q | . | . | . | . | . | . | . | . |
| 21-225_16G7_LC | W | . | . | . | Q | . | . | . | . | . | . | . | . |
| 21-225_1C12_LC | . | . | . | . | . | . | . | . | . | A | . | . | . |

FIGURE 56

Table 47

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K_FR1 | | | | | | |
| CONSENSUS | D | I | Q | M | T | Q | S | P | S | S | L | S | A | S | V | G | D |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | Q | . |
| 21-225_18C6_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | . | . | . | . | . | . | . | . | . |
| 21-225_6D4_LC | . | . | . | . | . | . | . | . | . | . | . | . | . | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_18A1_LC | . | . | . | . | . | . | . | . | . | . | . | . | . | . | . | . | . |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | R | - | - |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | . | . |
| 21-225_18C6_LC | . | . | . | . | . | . | . | T | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | T | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | V | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | T | . | . | . | . | . | . | . | . | . |
| 21-225_6D4_LC | . | . | . | . | . | . | . | . | . | . | . | . | A | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | K_FR2 | | | | | | | | |
| CONSENSUS | - | - | - | - | N | D | L | G | W | Y | Q | Q | K | P | G | K | A |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | . | . | . | . | . | . | . | . | . |
| 21-225_6D4_LC | . | . | . | . | . | . | . | . | . | . | . | . | . | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | K_CDR2 | | |
| CONSENSUS | P | K | R | L | I | Y | A | - | - | - | - | - | - | - | - | A | S |
| 21-226_8D12_LC | . | . | . | . | . | . | . | . | . | . | . | . | . | . | . | . | . |
| 21-225_20C2_LC | . | . | . | . | . | F | . | . | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | V | . | . | . | . | . | . | . | . | . | . |
| 21-225_22D12_LC | . | . | . | . | . | . | . | . | . | . | . | . | . | . | . | . | . |
| 21-226_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | Q | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | T | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-226_7F4_LC | . | . | . | . | . | . | . | . | . | . | . | . | . | . | . | . | . |
| 21-225_6D4_LC | . | . | . | . | . | . | . | . | . | . | . | . | . | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | T | . | . | . | . | . | . | . | . | . | . |
| 21-225_16A1_LC | . | . | . | . | . | . | . | . | . | . | . | . | . | . | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_8D12_LC | . | . | . | . | . | . | . | . | . | . | N | . | . | . | . | . | . |
| 21-225_20C2_LC | . | . | . | . | . | . | . | . | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | . | . | . | . | N | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | N | . | . | . | . | . | . |
| 21-225_22G9_LC | . | . | . | . | . | . | . | . | . | . | . | . | . | . | . | . | . |
| 21-225_34C4_LC | N | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | N | . | . | . | . | . | . | . | . | . | . | . | . | . | . | . | . |
| 21-225_6D4_LC | . | . | . | . | . | . | . | . | . | . | . | . | . | . | . | . | . |
| 21-225_16A1_LC | . | . | . | . | . | . | . | . | . | . | . | . | . | . | . | . | . |
| 21-225_15G7_LC | . | . | . | . | . | I | . | . | . | . | . | . | . | . | F | . | . |
| 21-225_11F10_LC | . | . | . | . | . | . | . | . | . | . | . | . | . | . | . | . | . |
| 21-225_15H1_LC | . | . | . | . | . | . | . | . | . | . | N | . | . | . | . | . | . |
| 21-225_16A1_LC | . | . | . | . | . | . | . | . | . | . | . | . | . | . | . | . | . |

**FIGURE 56**

| Reference # | 85 | 86 | 88 | 89 | 90 | 91 | 92 | 93 | 94 | 95 | 96 | 97 | 98 | 99 | 100 | 101 | 107 |
|---|---|---|---|---|---|---|---|---|---|---|---|---|---|---|---|---|---|
| | | | | K_FR3 | | | | | | | | | | | | | |
| CONSENSUS | - | T | E | F | T | L | T | I | S | S | L | Q | P | E | D | F | A |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | D | . | . | . | . | . | . | . | . | . | . | . | . | . | . |
| 21-225_23D1_LC | . | . | . | . | . | . | . | V | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | . | . | . | . | . | . | . | . | . |
| 21-225_6D4_LC | . | . | . | . | . | . | . | . | . | . | . | . | . | . | . | . | . |
| 21-225_15A1_LC | . | . | . | . | . | . | . | V | . | . | . | . | . | . | . | . | . |
| 21-225_16G7_LC | . | . | . | . | . | . | . | . | . | . | . | . | . | . | . | . | . |
| 21-225_11F10_LC | . | . | . | . | . | . | . | V | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | S | S | - | - | - | - | - | - | - | - |
| 21-225_3D12_LC | . | . | . | . | . | . | . | . | . | . | . | . | . | . | . | . | . |
| 21-225_20C2_LC | . | . | . | . | . | . | . | A | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | T | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | N | . | . | . | . | . | . | . | . |
| 21-235_22C1_LC | . | . | . | . | . | . | . | . | T | . | . | . | . | . | . | . | . |
| 21-225_20G9_LC | . | . | . | . | . | . | . | . | T | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | A | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | A | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | A | . | . | . | . | . | . | . | . |
| 21-225_6D4_LC | . | . | . | . | . | . | . | . | . | . | . | . | . | . | . | . | . |
| 21-225_15A1_LC | S | . | . | . | . | H | N | . | . | . | . | . | . | . | . | . | . |
| 21-225_15G7_LC | . | . | . | . | . | . | . | . | N | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | H | . | . | N | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | . | . |
| 21-225_18C8_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | . | . | . | . | . | . | . | . | . |
| 21-225_8D4_LC | . | . | . | . | . | . | . | . | . | . | . | . | . | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_18A1_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | | K_FR4 | | | | | |
| CONSENSUS | L | T | F | G | G | G | T | K | V | E | I | K | R |
| 21-225_8D12_LC | . | . | . | . | . | . | . | . | . | . | . | . | . |
| 21-225_20C2_LC | . | . | . | . | . | . | . | . | . | . | . | E | . |
| 21-225_18C6_LC | . | . | . | . | . | . | . | . | . | . | . | . | . |
| 21-225_18A3_LC | . | . | . | . | . | . | . | . | . | . | . | . | . |
| 21-225_22D12_LC | . | . | . | . | . | . | . | . | . | . | . | . | . |
| 21-225_23D1_LC | . | . | . | . | . | . | . | . | . | . | . | . | . |
| 21-225_24E5_LC | . | . | . | . | . | . | . | . | . | . | . | . | . |
| 21-225_22C1_LC | . | . | . | . | . | . | . | . | . | . | . | . | . |
| 21-225_20G9_LC | . | . | . | . | . | . | . | . | . | . | . | . | . |
| 21-225_21G7_LC | . | . | . | . | . | . | . | . | . | . | . | . | . |
| 21-225_22G9_LC | . | . | . | . | . | . | . | . | . | . | . | . | . |
| 21-225_34C4_LC | I | . | . | . | Q | . | . | R | L | . | . | . | . |
| 21-225_2F7_LC | . | . | . | . | . | . | . | . | . | . | . | . | . |
| 21-225_3G4_LC | . | . | . | . | . | . | . | . | . | . | . | . | . |
| 21-225_7F4_LC | . | . | . | . | . | . | . | . | . | . | . | . | . |
| 21-225_6D4_LC | . | . | . | . | . | . | . | . | . | . | . | . | . |
| 21-225_15A1_LC | V | K | . | . | . | . | I | . | . | . | . | T | G |
| 21-225_15G7_LC | . | . | . | . | . | . | . | . | . | . | . | . | . |
| 21-225_11F10_LC | . | . | . | . | . | . | . | . | . | . | . | . | . |
| 21-225_15H1_LC | . | . | . | . | . | . | . | . | . | . | . | . | . |
| 21-225_16A1_LC | . | . | . | . | . | . | . | . | . | . | . | E | . |

FIGURE 56

Table 48

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K_FR1 | | | | | |
| CONSENSUS | D | I | Q | M | T | Q | S | P | S | S | L | S | A | S | V | G | D |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | . |
| 21-225_17F9_LC | . | . | . | . | . | . | . | . | . | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | R | . | . | . | . | . | . |
| 21-225_22G8_LC | . | . | . | . | I | . | . | . | . | . | . | F | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | R | - | - |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | . |
| 21-225_17F9_LC | . | . | . | . | . | . | . | . | . | . | . | . | V | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | . | . | D | . | . | . | . |
| 21-225_22G8_LC | . | . | . | . | . | . | . | T | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

FIGURE 56

| Reference # | | | | | | | | | | | | | | | K_FR2 | |
|---|---|---|---|---|---|---|---|---|---|---|---|---|---|---|---|---|
| CONSENSUS | - | - | - | - | N | D | L | G | W | Y | Q | Q | K | P | G | K | A |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | . |
| 21-225_17F9_LC | . | . | . | . | . | . | . | . | . | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | . | . | . | . | . | R | . |
| 21-225_22G8_LC | . | . | . | . | . | . | . | . | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

| Reference # | | | | | | | K_CDR2 | | | | | | | | | | |
|---|---|---|---|---|---|---|---|---|---|---|---|---|---|---|---|---|---|
| CONSENSUS | P | K | R | L | I | Y | A | - | - | - | - | - | - | - | - | A | S |
| 21-225_11F5_LC | . | . | . | . | . | . | . | . | . | . | . | . | . | . | . | . | . |
| 21-225_17H8_LC | . | . | . | . | . | . | G | . | . | . | . | . | . | . | . | . | . |
| 21-225_17F9_LC | . | . | . | . | . | . | . | . | . | . | . | . | . | . | . | V | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | N | G | . | . | . | . | . | . | . | . | . | . |
| 21-225_22G8_LC | . | . | . | . | . | . | . | . | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | V | . | . | . | . | . | . | . | . | T | . |
| 21-225_14B2_LC | . | . | . | . | . | . | V | . | . | . | . | . | . | . | . | T | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | V | Q | S | G | V | P | S | R | F | S | G | S | G | S | G | - |
| 21-225_11F5_LC | . | . | . | . | . | . | . | . | . | . | . | . | . | . | . | . | . |
| 21-225_17H8_LC | . | L | . | . | . | . | . | . | . | . | . | . | . | . | . | . | . |
| 21-225_17F9_LC | . | L | . | . | . | . | . | . | . | . | . | . | . | . | . | . | . |
| 21-225_1F2_LC | . | . | . | . | . | . | . | . | . | . | . | . | . | . | . | . | . |
| 21-225_21H3_LC | . | . | . | T | . | . | . | . | . | . | . | . | . | . | . | . | . |
| 21-225_23C8_LC | . | . | . | . | . | . | . | . | . | . | . | . | . | . | . | . | . |
| 21-225_22G8_LC | . | L | . | . | . | . | . | . | K | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | L | . | G | . | . | . | . | . | . | . | . | . | . | . | . | . |
| 21-225_14B2_LC | . | L | . | G | . | . | . | . | . | . | . | . | . | . | . | . | . |
| 21-225_4F4_LC | . | . | . | T | . | . | . | . | . | . | . | . | . | . | . | . | . |
| 21-225_4H4_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | | |
| CONSENSUS | - | T | E | F | T | L | T | I | S | S | L | Q | P | E | D | F | A |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | T |
| 21-225_17F9_LC | . | . | . | . | . | . | . | . | N | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | A | . | . | L | . |
| 21-225_23C8_LC | . | . | . | . | . | . | . | . | . | . | . | . | . | . | . | . | V |
| 21-225_22G8_LC | . | . | . | . | . | . | S | . | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | I | Y | Y | C | L | Q | H | N | S | - | - | - | - | - | - | - | - |
| 21-225_11F5_LC | . | . | . | . | . | . | . | . | . | . | . | . | . | . | . | . | . |
| 21-225_17H8_LC | T | . | . | . | . | . | . | . | . | . | . | . | . | . | . | . | . |
| 21-225_17F9_LC | T | . | . | . | . | . | . | . | T | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | . | . | . | . | . | . | . |
| 21-225_22G8_LC | T | . | . | . | . | . | . | . | . | . | . | . | . | . | . | . | . |
| 21-225_22H4_LC | L | . | . | . | . | . | . | . | . | . | . | . | . | . | . | . | . |
| 21-225_23A3_LC | . | . | F | . | . | . | . | . | . | . | . | . | . | . | . | . | . |
| 21-225_5H7_LC | T | . | . | . | . | . | . | . | . | . | . | . | . | . | . | . | . |
| 21-225_14B2_LC | T | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | . |
| 21-225_17F9_LC | . | . | . | . | . | . | . | . | . | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | . | . | . | . | . | . | . |
| 21-225_22G8_LC | . | . | . | . | . | . | . | . | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

3644

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | | | | | K_FR4 | | | | | | |
| CONSENSUS | L | T | F | G | G | G | T | K | V | E | I | K | R |
| 21-225_11F5_LC | . | . | . | . | . | . | . | . | . | . | . | . | . |
| 21-225_17H8_LC | . | . | . | . | . | . | . | . | . | . | . | . | . |
| 21-225_17F9_LC | . | . | . | . | . | . | . | . | . | . | F | . | . |
| 21-225_1F2_LC | . | . | . | . | . | . | . | . | . | . | . | . | . |
| 21-225_21H3_LC | . | . | . | . | . | . | . | . | . | . | . | . | . |
| 21-225_23C8_LC | . | . | . | . | . | . | . | . | . | . | . | . | . |
| 21-225_22G8_LC | . | . | . | . | . | . | . | . | . | . | . | . | . |
| 21-225_22H4_LC | . | . | . | . | . | . | . | . | . | . | . | . | . |
| 21-225_23A3_LC | . | . | . | . | . | . | . | . | . | . | . | . | . |
| 21-225_5H7_LC | . | . | . | . | . | . | . | . | . | . | . | . | . |
| 21-225_14B2_LC | . | . | . | . | . | . | . | . | . | . | . | . | . |
| 21-225_4F4_LC | . | . | . | . | . | . | . | . | . | . | . | . | . |
| 21-225_4H4_LC | . | . | . | . | . | . | . | . | . | . | . | . | . |

**FIGURE 57**

Table 49:  Consensus 15-VH1|1-08/D6|6-19|RF1/JH4 (SEQ ID NO: 50266):

QVQLVQS-GAEVKKPGASVKVSCKASG-YTFTN-----YDINWVRQATGQGLEWMGWMHPN---
SGNTGYAQKFQGRVTMTRNTSISTAYMELSSLRSEDTAVYYCASSSGWY--------------------YFDYWGQGTLVTVSS wherein:

N at position 33 can be substituted with H

I at position 41 can be substituted with V or L

W at position 57 can be substituted with R

M at position 58 can be substituted with V or L

H at position 59 can substituted with N, Y or T

N at position 61 can be substituted with D or H

G at position 66 can be substituted with H

N at position 67 can be substituted with S, Q, A, D, K or T

T at position 68 can be substituted with A, V or E

G at position 69 can be substituted with D

Y at position 70 can be substituted with F or C

A at position 71 can be substituted with P

Q at position 72 can be substituted with K

K at position 73 can be substituted with R or N

Q at position 75 can be substituted with R

G at position 76 can be substituted with V

Y at position 113 can be substituted with E, N or T

**FIGURE 57**

Y at position 135 can be substituted with F, K, I, R, V, L, M, W, H or S

Y at position 138 can be substituted with F, S or N

Xaa Tyr Asp Xaa Asn (SEQ ID NO: 50001)

Xaa Xaa Xaa Pro Xaa Ser Xaa Xaa Xaa Xaa Xaa Xaa Xaa Phe Xaa Xaa (SEQ ID NO: 50002)

Ser Ser Gly Trp Xaa Xaa Phe Asp Xaa (SEQ ID NO: 50003)

Asn Tyr Asp Ile Asn (SEQ ID NO: 50468)

Trp Met His Pro Asn Ser Gly Asn Thr Gly Tyr Ala Gln Lys Phe Gln Gly (SEQ ID NO: 50469)

Ser Ser Gly Trp Tyr Tyr Phe Asp Tyr (SEQ ID NO: 50470)

# EP 4 435 105 A2

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H FR1 | | | | | | | | | |
| CONSENSUS | Q | V | Q | L | V | Q | S | - | G | A | E | V | K | K | P | G | A | S | V | K | V | S | C | K | A |
| 21-225_11E5_HC | . | . | . | . | . | . | . | . | . | P | . | . | . | . | . | . | . | . | . | . | . | . | . | . | . |
| 21-225_12F11_HC | . | . | . | . | . | . | . | . | . | . | . | . | . | . | . | . | . | . | . | . | . | . | . | . | . |
| 21-225_12F12_HC | . | . | . | . | L | . | . | . | . | . | . | . | . | . | . | . | . | . | . | . | . | . | . | . | . |
| 21-225_146C9_HC | . | . | . | . | . | . | . | . | . | . | . | . | . | . | . | . | . | . | . | . | . | . | . | . | . |
| 21-225_146D4_HC | . | . | . | . | . | . | . | . | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | . | . | . | . | . | . | . | . | . | . | . | . | . | . | . | . | . | . |
| 21-225_147E4_HC | . | . | . | . | . | . | . | . | . | . | . | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | . | . | . |
| 21-225_149A2_HC | . | . | . | . | . | . | . | . | . | . | . | . | . | . | . | . | . | . | . | . | . | . | . | . | . |
| 21-225_149E3_HC | . | . | . | . | . | . | . | . | . | . | . | . | . | . | . | . | . | . | . | . | . | . | . | . | . |
| 21-225_149H4_HC | . | . | . | . | . | . | . | . | . | . | . | . | . | . | . | . | . | . | . | . | . | . | . | . | . |
| 21-225_150B7_HC | . | . | . | . | . | . | . | . | . | . | . | . | . | . | . | . | . | . | . | T | . | . | . | . | . |
| 21-225_150D2_HC | . | . | . | . | . | . | . | . | . | . | . | . | . | . | . | . | . | . | . | . | . | . | . | . | . |
| 21-225_152E12_HC | . | . | . | . | . | . | . | . | . | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | . | . | . | . | . | . | . | . | . | . | . | . | . | . | . | . | . | . | . |
| 21-225_153F11_HC | . | . | . | . | . | . | . | . | . | . | . | . | . | E | . | . | . | . | . | . | . | . | . | . | . |
| 21-225_154A11_HC | . | . | . | . | . | . | . | . | . | . | . | . | . | . | . | . | . | . | . | . | . | . | . | . | . |
| 21-225_154H6_HC | . | . | . | . | . | . | . | . | . | . | . | . | . | . | . | . | . | . | . | . | . | . | . | . | . |
| 21-225_155B6_HC | . | . | . | . | . | . | . | . | . | . | . | . | . | . | . | . | . | . | . | . | . | . | . | . | . |
| 21-225_155E5_HC | . | . | . | . | . | . | . | . | . | . | . | . | . | . | . | . | . | . | . | . | . | . | . | . | . |
| 21-225_156H1_HC | . | . | . | . | . | . | . | . | . | . | . | . | R | . | . | . | . | . | . | . | . | . | . | . | . |
| 21-225_158B12_HC | . | . | . | . | . | . | . | . | . | . | . | . | . | . | . | . | . | . | . | . | . | . | . | . | . |
| 21-225_160A4_HC | . | . | . | . | . | . | . | . | . | T | . | . | . | . | . | . | . | . | . | . | . | . | . | . | . |
| 21-225_160A7_HC | . | . | . | . | . | . | . | . | . | . | . | . | . | . | . | . | . | . | . | . | . | . | . | . | . |
| 21-225_162D5_HC | . | . | . | . | . | . | . | . | . | S | . | . | . | . | . | . | . | . | . | . | . | . | . | . | . |
| 21-225_162F6_HC | . | . | . | . | . | . | . | . | . | . | . | . | R | . | . | . | . | . | . | . | . | . | . | . | . |

3648

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | Q | S | - | G | A | E | V | K | K | P | G | A | S | V | K | V | S | C | K | A |
| 21-225_164A4_HC | . | | | . | | . | . | | | | | | | | | | | | | | | | | | |
| 21-225_164A7_HC | . | | . | . | | | . | | | | | | | | | | | . | | | | | | | |
| 21-225_164B11_HC | . | | | . | | | | | | | | | | | | . | | | | | | | | | |
| 21-225_165H2_HC | . | | | | | | | | | | | | | | | | | | | | | | | | |
| 21-225_172E11_HC | . | | | | | | | | | | | | | | | | | | | M | | | | | |
| 21-225_175D10_HC | . | | | | | | | | | | | | | | | | | | | | | | | | |
| 21-225_177B12_HC | . | | | | | | | | | | | | | | | | | | | | | | | | |
| 21-225_101E5_HC | . | | | | | | | | | | | | | | | | | | | | | | | | |
| 21-225_190D7_HC | . | | | | | | | | | | | | | | | | | | | | | | | | |
| 21-225_190H4_HC | . | | | | | | | | | | | | | | | | | | | | | | | | |
| 21-225_191D8_HC | . | | | | | | | | | | | | | | | | | | | | | | | | |
| 21-225_191G3_HC | . | | | | | | | | | | | | | | | | | | | | | | | | |
| 21-225_193G4_HC | . | | | | | | | | | | | | | | | | | | | | | | | | |
| 21-225_197C6_HC | . | | | | | | | | | | | | | | | | | | | | | | | | |
| 21-225_197H4_HC | . | | | | | | | | | | | | | | | | | | | | | | | | |
| 21-225_201F5_HC | . | | | | | | | | | A | | | | | | | | | | | | | | | |
| 21-225_224C10_HC | . | | | | | | | | | | | | | | | | | | | | | | | | |
| 21-225_224D8_HC | . | | | | | | | | | | | | | | | | | | | | | | | | |
| 21-225_224F11_HC | . | | | | | | | | | | | | | | | | | | | | | | | | |
| 21-225_225B9_HC | . | | | | | | | | | T | | | R | | | | | | | | | | | | |
| 21-225_225F11_HC | . | | | | | | | | | | | | | | | | | | | | | | | | |
| 21-225_225F12_HC | . | | | | | | | | | | | | | | | | | | | | | | | | |
| 21-225_225F5_HC | . | | | | | | | | | | | | | | | R | | | | | | | | | |
| 21-225_225H12_HC | . | | | | | | | | | | | | | | | | | | | | | | | | |
| 21-225_226A12_HC | . | | | | | | | | | | | | | | | | | | | | | | | | |
| 21-225_226D11_HC | . | | | | | | | | | | | | | | | | | | | | | | | R | S |
| 21-225_226D6_HC | . | | | | | | | | | | | | | | | | | | | | | | | | |
| 21-225_226F6_HC | . | | | | | | | | | | | | | | | | | | | | | | | | |
| 21-225_227C7_HC | . | | | | | | | | | | | | | | | R | | | | | | | | | |

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | Q | S | - | G | A | E | V | K | K | P | G | A | S | V | K | V | S | C | K | A |
| 21-225_227D11_HC | . | . | . | . | . | . | . | . | . | . | . | . | . | . | . | . | . | . | . | . | . | . | . | . | . |
| 21-225_227E6_HC | . | . | . | . | . | . | . | . | . | . | . | . | . | . | . | . | . | . | . | . | . | . | . | . | . |
| 21-225_22D2_HC | . | . | . | . | . | . | . | . | . | . | . | . | . | . | . | . | . | . | . | . | . | . | . | . | . |
| 21-225_23A12_HC | . | . | . | . | . | . | . | . | . | . | . | . | . | . | . | . | . | . | . | . | . | . | . | . | . |
| 21-225_24E1_HC | . | . | . | . | . | . | . | . | . | . | . | . | . | . | . | . | . | . | . | . | . | . | . | . | . |
| 21-225_25A4_HC | . | . | L | . | . | . | . | . | . | . | . | . | . | R | . | . | . | . | . | . | . | . | . | . | . |
| 21-225_25A4.001_HC | . | . | L | . | . | . | . | . | . | . | . | . | . | R | . | . | . | . | . | . | . | . | . | . | . |
| 21-225_25A4.001.001_HC | . | . | L | . | . | . | . | . | . | . | . | . | . | R | . | . | . | . | . | . | . | . | . | . | . |
| 21-225_25A4.001.002_HC | . | . | L | . | . | . | . | . | . | . | . | . | . | R | . | . | . | . | . | . | . | . | . | . | . |
| 21-225_25A4.001.003_HC | . | . | L | . | . | . | . | . | . | . | . | . | . | R | . | . | . | . | . | . | . | . | . | . | . |
| 21-225_25A4.001.004_HC | . | . | L | . | . | . | . | . | . | . | . | . | . | R | . | . | . | . | . | . | . | . | . | . | . |
| 21-225_25A4.001.005_HC | . | . | . | . | . | . | . | . | . | . | . | . | . | R | . | . | . | . | . | . | . | . | . | . | . |
| 21-225_25A4.001.006_HC | . | . | . | . | . | . | . | . | . | . | . | . | . | R | . | . | . | . | . | . | . | . | . | . | . |
| 21-225_25A4.001.007_HC | . | . | . | . | . | . | . | . | . | . | . | . | . | R | . | . | . | . | . | . | . | . | . | . | . |
| 21-225_25A4.001.008_HC | . | . | . | . | . | . | . | . | . | . | . | . | . | R | . | . | . | . | . | . | . | . | . | . | . |
| 21-225_25A4.001.013_HC | . | . | L | . | . | . | . | . | . | . | . | . | . | R | . | . | . | . | . | . | . | . | . | . | . |
| 21-225_25A4.001.019_HC | . | . | . | . | . | . | . | . | . | . | . | . | . | R | . | . | . | . | . | . | . | . | . | . | . |
| 21-225_25A4.001.020_HC | . | . | L | . | . | . | . | . | . | . | . | . | . | R | . | . | . | . | . | . | . | . | . | . | . |
| 21-225_25A4.001.021_HC | . | . | . | . | . | . | . | . | . | . | . | . | . | R | . | . | . | . | . | . | . | . | . | . | . |
| 21-225_25A4.001.029_HC | . | . | L | . | . | . | . | . | . | . | . | . | . | R | . | . | . | . | . | . | . | . | . | . | . |
| 21-225_26C4_HC | . | . | . | . | . | . | . | . | . | . | . | . | . | . | . | . | . | . | . | . | . | . | . | . | . |
| 21-225_28B6_HC | . | . | L | . | . | . | . | . | . | . | . | . | . | R | . | . | . | . | . | . | . | . | . | . | . |
| 21-225_29E6_HC | . | . | L | . | . | . | . | . | . | . | . | . | . | R | . | . | . | . | . | . | . | . | . | . | . |
| 21-225_29H8_HC | . | . | . | . | . | . | . | . | . | . | . | . | Q | . | . | . | . | . | . | . | . | . | . | . | . |
| 21-225_32A1_HC | . | . | . | . | . | . | . | . | . | . | . | . | . | . | . | . | . | . | . | . | . | . | . | . | . |
| 21-225_32A5_HC | . | . | . | . | . | . | . | . | . | . | . | . | . | . | . | . | . | . | . | . | . | . | . | . | . |
| 21-225_33D1_HC | . | . | . | . | . | . | . | . | . | . | . | . | . | . | . | . | . | . | . | . | . | . | . | . | . |
| 21-225_34D2_HC | . | . | . | . | . | . | . | . | . | . | . | . | . | . | . | . | . | . | . | . | . | . | . | . | . |
| 21-225_34H7_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | Q | S | - | G | A | E | V | K | K | P | G | A | S | V | K | V | S | C | K | A |
| 21-225_6G6_HC | . | . | . | . | . | . | . | . | . | . | . | . | R | . | . | . | . | . | . | . | . | . | . | . | . |
| 21-225_70E7_HC | . | . | . | . | . | . | . | . | . | . | . | . | . | . | . | . | . | . | . | . | . | . | . | . | . |
| 21-225_71F3_HC | . | . | . | . | . | . | . | . | . | . | . | . | . | . | . | . | . | . | . | . | . | . | . | . | . |
| 21-225_71G3_HC | . | . | . | . | . | . | . | . | . | . | . | . | . | . | . | . | . | . | . | . | . | . | . | . | . |
| 21-225_72C4_HC | . | . | . | . | . | . | . | . | . | . | . | . | . | . | . | . | . | . | . | . | . | . | . | . | . |
| 21-225_74A6_HC | . | . | . | . | . | . | . | . | . | . | . | . | . | . | . | . | . | . | . | . | . | . | . | . | . |
| 21-225_74A8_HC | . | . | . | . | . | . | . | . | . | . | . | . | . | . | . | . | . | . | . | . | . | . | . | . | . |
| 21-225_74B11_HC | . | . | . | . | . | . | . | . | . | . | . | . | . | . | . | . | . | . | . | R | . | . | . | . | . |
| 21-225_74F6_HC | . | . | . | . | . | . | . | . | . | . | . | . | . | . | . | . | . | . | . | R | . | . | . | . | . |
| 21-225_74F9_HC | . | . | . | . | . | . | . | . | . | . | . | . | . | . | . | . | . | . | . | . | . | . | . | . | . |
| 21-225_74G4_HC | . | . | . | . | . | . | . | . | . | . | . | . | . | . | . | . | . | . | . | . | . | . | . | . | . |
| 21-225_75A10_HC | . | . | . | . | . | . | . | . | . | . | . | . | . | . | . | . | . | . | . | . | . | . | . | . | . |
| 21-225_75A6_HC | . | . | . | . | . | . | . | . | . | . | . | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . | . |
| 21-225_75F9_HC | . | . | . | . | . | . | . | . | . | . | . | . | . | . | . | . | . | . | . | . | . | . | . | . | . |
| 21-225_76E11_HC | . | . | . | . | . | . | . | . | . | . | . | . | . | . | . | . | . | . | . | . | . | . | . | . | . |
| 21-225_76G2_HC | . | . | . | . | . | . | . | . | . | . | . | . | . | . | . | . | . | . | . | . | . | . | . | . | . |
| 21-225_78E11_HC | . | . | . | . | . | . | . | . | . | . | . | . | . | . | . | . | . | . | . | . | . | . | . | . | . |
| 21-225_78E6_HC | . | . | . | . | . | . | . | . | . | . | . | . | . | . | . | . | . | . | . | . | . | . | . | . | . |
| 21-225_79E9_HC | . | . | . | . | . | . | . | . | . | . | . | . | . | . | . | . | . | . | . | . | . | . | . | . | . |
| 21-225_79F12_HC | . | . | . | . | . | . | . | . | . | . | . | . | . | . | . | . | . | . | . | . | . | . | . | . | . |
| 21-225_79G7_HC | . | . | . | . | . | . | . | . | . | . | . | . | . | . | . | . | . | . | . | . | . | . | . | . | T |
| 21-225_80B12_HC | . | . | . | . | . | . | . | . | . | . | . | . | . | . | . | . | . | . | . | . | . | . | . | . | . |
| 21-225_85C11_HC | . | . | . | . | . | . | . | . | . | . | . | . | . | . | . | . | . | . | . | . | . | . | . | . | . |
| 21-225_87E10_HC | . | . | . | . | . | . | . | . | . | . | . | . | . | . | . | . | . | . | . | . | . | . | . | . | . |
| 21-225_88A1_HC | . | . | . | . | . | . | . | . | . | . | . | . | . | . | . | . | . | . | . | . | . | . | . | . | . |
| 21-225_88B4_HC | . | . | . | . | . | . | . | . | . | . | . | . | . | . | . | . | . | . | . | . | . | . | . | . | . |
| 21-225_88C10_HC | . | . | . | . | . | . | . | . | . | . | . | . | . | . | . | . | . | . | . | R | . | . | . | . | . |
| 21-225_8B11_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | Q | S | - | G | A | E | V | K | K | P | G | A | S | V | K | V | S | C | K | A |
| 21-225_92E6_HC | . | . | . | . | . | . | . | . | . | . | . | . | . | . | . | . | . | . | . | R | . | . | . | . | . |
| 21-225_94D8_HC | . | . | . | . | . | . | . | . | . | . | . | . | . | . | . | . | . | . | . | . | . | . | . | . | T |
| 21-225_96D10_HC | . | . | . | . | . | . | . | . | . | . | . | . | . | . | . | . | . | . | . | R | . | . | . | . | . |
| 21-225_96H5_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | Q | S | - | G | A | E | V | K | K | P | G | A | S | V | K | V | S | C | K | A |
| 21-225_43D8_HC | . | . | . | . | . | . | . | . | . | . | . | . | . | . | . | . | . | . | . | . | . | . | . | . | . |
| 21-225_4A2_HC | . | . | . | . | . | . | . | . | . | T | . | . | . | . | . | . | . | . | . | . | . | . | . | . | . |
| 21-225_4A2.001_HC | . | . | . | . | . | . | . | . | . | T | . | . | . | . | . | . | . | . | . | . | . | . | . | . | . |
| 21-225_4A2.001.001_HC | . | . | . | . | . | . | . | . | . | T | . | . | . | . | . | . | . | . | . | . | . | . | . | . | . |
| 21-225_4A2.001.002_HC | . | . | . | . | . | . | . | . | . | T | . | . | . | . | . | . | . | . | . | . | . | . | . | . | . |
| 21-225_4A2.001.003_HC | . | . | . | . | . | . | . | . | . | T | . | . | . | . | . | . | . | . | . | . | . | . | . | . | . |
| 21-225_4A2.001.004_HC | . | . | . | . | . | . | . | . | . | T | . | . | . | . | . | . | . | . | . | . | . | . | . | . | . |
| 21-225_4A2.001.005_HC | . | . | . | . | . | . | . | . | . | T | . | . | . | . | . | . | . | . | . | . | . | . | . | . | . |
| 21-225_4A2.001.006_HC | . | . | . | . | . | . | . | . | . | T | . | . | . | . | . | . | . | . | . | . | . | . | . | . | . |
| 21-225_4A2.001.007_HC | . | . | . | . | . | . | . | . | . | T | . | . | . | . | . | . | . | . | . | . | . | . | . | . | . |
| 21-225_4A2.001.008_HC | . | . | . | . | . | . | . | . | . | T | . | . | . | . | . | . | . | . | . | . | . | . | . | . | . |
| 21-225_4A2.001.009_HC | . | . | . | . | . | . | . | . | . | T | . | . | . | . | . | . | . | . | . | . | . | . | . | . | . |
| 21-225_4A2.001.010_HC | . | . | . | . | . | . | . | . | . | T | . | . | . | . | . | . | . | . | . | . | . | . | . | . | . |
| 21-225_4A2.001.011_HC | . | . | . | . | . | . | . | . | . | T | . | . | . | . | . | . | . | . | . | . | . | . | . | . | . |
| 21-225_4A2.001.012_HC | . | . | . | . | . | . | . | . | . | T | . | . | . | . | . | . | . | . | . | . | . | . | . | . | . |
| 21-225_4A2.001.022_HC | . | . | . | . | . | . | . | . | . | . | . | . | . | . | . | . | . | . | . | . | . | . | . | . | . |
| 21-225_4A2.001.023_HC | . | . | . | . | . | . | . | . | . | . | . | . | . | . | . | . | . | . | . | . | . | . | . | . | . |
| 21-225_4A2.001.024_HC | . | . | . | . | . | . | . | . | . | . | . | . | . | . | . | . | . | . | . | . | . | . | . | . | . |
| 21-225_4A2.001.028_HC | . | . | . | . | . | . | . | . | . | . | . | . | . | . | . | . | . | . | . | . | . | . | . | . | . |
| 21-225_4A2.001.029_HC | . | . | . | . | . | . | . | . | . | . | . | . | . | . | . | . | . | . | . | . | . | . | . | . | . |
| 21-225_4A2.001.031_HC | . | . | . | . | . | . | . | . | . | G | . | . | . | . | . | . | . | . | . | . | . | . | . | . | . |
| 21-225_56A1_HC | . | . | . | . | . | . | . | . | . | . | . | . | . | . | . | . | . | . | . | . | . | . | . | . | . |
| 21-225_58B9_HC | . | . | . | . | . | . | . | . | . | . | . | . | . | . | . | . | . | . | . | . | . | . | . | . | . |
| 21-225_59E1_HC | . | . | . | . | . | . | . | . | . | . | . | . | . | . | . | . | . | . | . | . | . | . | . | . | . |
| 21-225_59F10_HC | . | . | . | . | . | . | . | . | . | . | . | . | . | . | . | . | . | . | . | . | . | . | . | . | . |
| 21-225_60F3_HC | . | . | L | . | . | . | . | . | . | . | . | . | . | . | . | . | . | . | . | . | . | . | . | . | . |
| 21-225_61B5_HC | . | . | . | . | . | . | . | . | . | . | . | . | . | . | . | . | . | . | . | . | . | . | . | . | . |
| 21-225_61E3_HC | . | . | . | . | . | . | . | . | . | . | . | . | . | . | . | . | . | . | . | . | . | . | . | . | . |
| 21-225_63F4_HC | . | . | . | . | . | . | . | . | . | . | . | . | . | . | . | . | . | . | . | . | . | . | . | . | . |

**FIGURE 57**

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | | H_FR2 |
| CONSENSUS | S | G | - | Y | T | F | T | N | - | - | - | - | - | Y | D | I | N | W | V | R | Q | A | T | G | Q |
| 21-225_11E5_HC | . | . | . | . | . | . | . | . | . | . | . | . | . | . | . | . | . | . | . | . | . | . | . | . | . |
| 21-225_12F11_HC | . | . | . | . | . | . | . | . | . | . | . | . | . | . | . | . | . | . | . | . | . | . | . | . | . |
| 21-225_12F12_HC | . | . | . | . | . | . | . | . | . | . | . | . | . | . | . | . | . | . | . | . | . | . | . | . | . |
| 21-225_146C9_HC | . | . | . | . | . | . | . | . | . | . | . | . | . | . | . | . | . | . | . | . | . | . | . | . | . |
| 21-225_146D4_HC | . | . | . | . | . | . | . | . | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | . | . | . | . | . | . | . | . | . | . | . | . | . | . | . | . | . | . |
| 21-225_147E4_HC | . | . | . | . | . | . | . | . | . | . | . | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | S | . | . |
| 21-225_149A2_HC | . | . | . | . | . | . | . | . | . | . | . | . | . | . | . | . | . | . | . | . | . | . | . | . | . |
| 21-225_149E3_HC | . | . | . | . | . | . | . | . | . | . | . | . | . | . | . | . | . | . | . | . | . | . | . | . | . |
| 21-225_149H4_HC | . | . | . | . | . | . | . | . | . | . | . | . | . | . | . | . | . | . | . | . | . | . | . | . | . |
| 21-225_150B7_HC | . | . | . | F | P | . | . | . | . | . | . | . | . | . | . | . | . | . | . | . | . | . | . | . | . |
| 21-225_150D2_HC | . | . | . | . | . | . | . | . | . | . | . | . | . | . | . | . | . | . | . | . | . | . | . | . | . |
| 21-225_152E12_HC | . | . | . | . | . | . | . | . | . | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | P | . | . | . | . | . | . | . | . | . | . | . | . | . | . | . | . | . | . |
| 21-225_153F11_HC | . | . | . | . | . | . | . | . | . | . | . | . | . | . | . | . | . | . | . | . | . | . | . | . | . |
| 21-225_154A11_HC | . | . | . | . | . | . | . | . | . | . | . | . | . | . | . | . | . | . | . | . | . | . | . | . | . |
| 21-225_154H6_HC | . | . | . | . | . | . | . | . | . | . | . | . | . | . | . | . | . | . | . | . | . | . | . | . | . |
| 21-225_155B6_HC | . | . | . | . | . | . | . | . | . | . | . | . | . | . | . | . | . | . | . | . | . | . | . | . | . |
| 21-225_155E5_HC | . | . | . | . | . | . | . | . | . | . | . | . | . | . | . | . | . | . | . | . | . | . | . | . | . |
| 21-225_156H1_HC | . | . | . | . | . | . | . | . | . | . | . | . | . | . | . | . | . | . | . | . | . | . | . | . | . |
| 21-225_158B12_HC | . | . | . | . | . | . | . | . | . | . | . | . | . | . | . | . | . | . | . | . | . | . | . | . | . |
| 21-225_160A4_HC | . | . | . | . | . | . | . | . | . | . | . | . | . | . | . | . | . | . | . | . | . | . | . | . | . |
| 21-225_160A7_HC | . | . | . | . | . | . | . | . | . | . | . | . | . | . | . | . | . | . | . | . | . | . | . | . | . |
| 21-225_162D5_HC | . | . | . | . | . | . | . | . | . | . | . | . | . | . | . | . | . | . | . | . | . | . | . | . | . |
| 21-225_162F6_HC | . | . | . | . | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | Y | T | F | T | N | - | - | - | - | - | Y | D | I | N | W | V | R | Q | A | T | G | Q |
| 21-225_164A4_HC | . | . | . | F | . | . | P | . | . | . | . | . | . | . | . | . | . | . | . | . | . | . | . | . | . |
| 21-225_164A7_HC | . | . | . | . | . | . | . | . | . | . | . | . | . | . | . | V | . | . | . | . | . | . | . | . | . |
| 21-225_164B11_HC | . | . | . | . | . | . | . | . | . | . | . | . | . | . | . | . | . | . | . | . | . | . | . | . | . |
| 21-225_165H2_HC | . | . | . | . | . | . | . | H | . | . | . | . | . | . | . | . | . | . | . | . | . | . | . | . | . |
| 21-225_172E11_HC | . | . | . | . | . | . | . | . | . | . | . | . | . | . | . | . | . | . | . | . | . | . | . | . | . |
| 21-225_175D10_HC | . | . | . | . | . | . | . | . | . | . | . | . | . | . | . | L | . | . | . | . | . | . | . | . | . |
| 21-225_177B12_HC | . | . | . | . | . | . | . | . | . | . | . | . | . | . | . | . | . | . | . | . | . | . | . | . | . |
| 21-225_181E5_HC | . | . | . | . | . | . | . | . | . | . | . | . | . | . | . | . | . | . | . | . | . | . | . | . | . |
| 21-225_190D7_HC | . | . | . | . | . | . | . | . | . | . | . | . | . | . | . | . | . | . | . | . | . | . | . | . | . |
| 21-225_190H4_HC | . | . | N | . | . | . | . | . | . | . | . | . | . | . | . | . | . | . | . | . | . | . | . | . | . |
| 21-225_191D8_HC | . | . | . | . | . | . | . | . | . | . | . | . | . | . | . | . | . | . | . | . | . | . | . | . | . |
| 21-225_191G3_HC | . | . | . | . | . | . | . | . | . | . | . | . | . | . | . | . | . | . | . | . | . | . | . | . | . |
| 21-225_193G4_HC | . | . | . | . | . | . | . | . | . | . | . | . | . | . | . | . | . | . | . | . | . | . | . | . | . |
| 21-225_197C6_HC | . | . | . | . | . | . | . | . | . | . | . | . | . | . | . | . | . | . | . | . | . | . | . | . | . |
| 21-225_197H4_HC | . | . | . | . | . | . | . | . | . | . | . | . | . | . | . | . | . | . | . | . | . | . | . | . | . |
| 21-225_201F5_HC | . | . | . | . | . | . | . | . | . | . | . | . | . | . | . | . | . | . | . | . | . | . | . | . | . |
| 21-225_224C10_HC | . | . | . | S | . | . | . | . | . | . | . | . | . | . | . | . | . | . | . | . | . | . | . | . | . |
| 21-225_224D8_HC | . | . | . | . | . | . | . | . | . | . | . | . | . | . | . | . | . | . | . | . | . | . | . | . | . |
| 21-225_224F11_HC | . | . | . | . | . | . | . | . | . | . | . | . | . | . | . | . | . | . | . | . | . | . | . | . | . |
| 21-225_225B9_HC | . | . | . | . | . | . | . | . | . | . | . | . | . | . | . | . | . | . | . | . | . | . | . | . | . |
| 21-225_225F11_HC | . | . | . | . | . | . | . | . | . | . | . | . | . | . | . | . | . | . | . | . | . | . | . | . | . |
| 21-225_225F12_HC | . | . | . | . | . | . | . | H | . | . | . | . | . | . | . | . | . | . | . | . | . | . | . | . | . |
| 21-225_225F5_HC | . | . | H | . | . | . | . | . | . | . | . | . | . | . | . | . | . | . | . | . | . | . | . | . | . |
| 21-225_225H12_HC | . | . | . | . | . | . | . | . | . | . | . | . | . | . | . | . | . | . | . | . | . | . | . | . | . |
| 21-225_226A12_HC | . | . | . | . | . | . | . | . | . | . | . | . | . | . | . | . | . | . | . | . | . | . | . | . | . |
| 21-225_226D11_HC | . | . | . | . | . | . | . | . | . | . | . | . | . | . | . | . | . | . | . | . | . | . | . | . | . |
| 21-225_226D6_HC | . | . | . | . | . | . | . | . | . | . | . | . | . | . | . | . | . | . | . | . | . | . | . | . | . |
| 21-225_226F6_HC | . | . | . | . | . | . | . | . | . | . | . | . | . | . | . | . | . | . | . | . | . | . | . | . | . |
| 21-225_227C7_HC | . | . | . | H | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | H_CDR1 | | | | | | | | | | | | | | H_FR2 | |
| CONSENSUS | S | G | - | Y | T | F | T | N | - | - | - | - | - | Y | D | I | N | W | V | R | Q | A | T | G | Q |
| 21-225_227D11_HC | . | . | . | . | . | . | . | . | . | . | . | . | . | . | . | . | . | . | . | . | . | . | . | . | . |
| 21-225_227E6_HC | . | . | . | . | . | . | . | . | . | . | . | . | . | . | . | . | . | . | . | . | . | . | . | . | . |
| 21-225_22D2_HC | . | . | . | . | . | . | I | . | . | . | . | . | . | . | . | . | . | . | . | . | . | . | . | . | . |
| 21-225_23A12_HC | . | . | . | . | P | . | . | . | . | . | . | . | . | . | . | . | . | . | . | . | . | . | . | . | . |
| 21-225_24E1_HC | . | . | . | . | . | . | . | . | . | . | . | . | . | . | . | . | . | . | . | . | . | . | . | . | . |
| 21-225_25A4_HC | . | . | . | . | . | . | . | . | . | . | . | . | . | . | . | . | . | . | . | . | . | . | . | . | . |
| 21-225_25A4.001_HC | . | . | . | . | . | . | . | . | . | . | . | . | . | . | . | . | . | . | . | . | . | . | . | . | . |
| 21-225_25A4.001.001_HC | . | . | . | . | . | . | . | . | . | . | . | . | . | . | . | . | . | . | . | . | . | . | . | . | . |
| 21-225_25A4.001.002_HC | . | . | . | . | . | . | . | . | . | . | . | . | . | . | . | . | . | . | . | . | . | . | . | . | . |
| 21-225_25A4.001.003_HC | . | . | . | . | . | . | . | . | . | . | . | . | . | . | . | . | . | . | . | . | . | . | . | . | . |
| 21-225_25A4.001.004_HC | . | . | . | . | . | . | . | . | . | . | . | . | . | . | . | . | . | . | . | . | . | . | . | . | . |
| 21-225_25A4.001.005_HC | . | . | . | . | . | . | . | . | . | . | . | . | . | . | . | . | . | . | . | . | . | . | . | . | . |
| 21-225_25A4.001.006_HC | . | . | . | . | . | . | . | . | . | . | . | . | . | . | . | . | . | . | . | . | . | . | . | . | . |
| 21-225_25A4.001.007_HC | . | . | . | . | . | . | . | . | . | . | . | . | . | . | . | . | . | . | . | . | . | . | . | . | . |
| 21-225_25A4.001.008_HC | . | . | . | . | . | . | . | . | . | . | . | . | . | . | . | . | . | . | . | . | . | . | . | . | . |
| 21-225_25A4.001.013_HC | . | . | . | . | . | . | . | . | . | . | . | . | . | . | . | . | . | . | . | . | . | . | . | . | . |
| 21-225_25A4.001.019_HC | . | . | . | . | . | . | . | . | . | . | . | . | . | . | . | . | . | . | . | . | . | . | P | . | . |
| 21-225_25A4.001.020_HC | . | . | . | . | . | . | . | . | . | . | . | . | . | . | . | . | . | . | . | . | . | . | P | . | . |
| 21-225_25A4.001.021_HC | . | . | . | . | . | . | . | . | . | . | . | . | . | . | . | . | . | . | . | . | . | . | . | . | . |
| 21-225_25A4.001.029_HC | . | . | . | . | . | . | . | . | . | . | . | . | . | . | . | . | . | . | . | . | . | . | . | . | . |
| 21-225_26C4_HC | . | . | . | . | . | . | . | . | . | . | . | . | . | . | . | . | . | . | . | . | . | . | S | . | . |
| 21-225_28B6_HC | . | . | . | . | . | . | . | . | . | . | . | . | . | . | . | . | . | . | . | . | . | . | . | . | . |
| 21-225_29E6_HC | . | . | . | . | . | . | . | . | . | . | . | . | . | . | . | . | . | . | . | . | . | . | . | . | . |
| 21-225_29H8_HC | . | . | . | . | . | . | . | . | . | . | . | . | . | . | . | . | . | . | . | . | . | . | . | . | . |
| 21-225_32A1_HC | . | . | . | . | . | . | . | . | . | . | . | . | . | . | . | . | . | . | . | . | . | . | . | . | . |
| 21-225_32A5_HC | . | . | . | . | . | . | . | . | . | . | . | . | . | . | . | . | . | . | . | . | . | . | . | . | . |
| 21-225_33D1_HC | . | . | . | . | . | . | . | . | . | . | . | . | . | . | . | . | . | . | . | . | . | . | . | . | . |
| 21-225_34D2_HC | . | . | . | . | . | . | . | . | . | . | . | . | . | . | . | . | . | . | . | . | . | . | . | . | . |
| 21-225_34H7_HC | . | . | . | . | . | . | . | . | . | . | . | . | . | . | . | . | . | . | . | . | . | . | . | . | . |

**FIGURE 57**

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | | | | | | | H_FR2 | |
| CONSENSUS | S | G | - | Y | T | F | T | N | - | - | - | - | - | Y | D | I | N | W | V | R | Q | A | T | G | Q |
| 21-225_43D8_HC | . | . | . | . | . | . | . | . | . | . | . | . | . | . | . | . | . | . | . | . | . | . | . | . | . |
| 21-225_4A2_HC | . | . | . | . | . | . | . | . | . | . | . | . | . | . | . | . | . | . | . | . | . | . | . | . | . |
| 21-225_4A2.001_HC | . | . | . | . | . | . | . | . | . | . | . | . | . | . | . | . | . | . | . | . | . | . | . | . | . |
| 21-225_4A2.001.001_HC | . | . | . | . | . | . | . | . | . | . | . | . | . | . | . | . | . | . | . | . | . | . | . | . | . |
| 21-225_4A2.001.002_HC | . | . | . | . | . | . | . | . | . | . | . | . | . | . | . | . | . | . | . | . | . | . | . | . | . |
| 21-225_4A2.001.003_HC | . | . | . | . | . | . | . | . | . | . | . | . | . | . | . | . | . | . | . | . | . | . | . | . | . |
| 21-225_4A2.001.004_HC | . | . | . | . | . | . | . | . | . | . | . | . | . | . | . | . | . | . | . | . | . | . | . | . | . |
| 21-225_4A2.001.005_HC | . | . | . | . | . | . | . | . | . | . | . | . | . | . | . | . | . | . | . | . | . | . | . | . | . |
| 21-225_4A2.001.006_HC | . | . | . | . | . | . | . | . | . | . | . | . | . | . | . | . | . | . | . | . | . | . | . | . | . |
| 21-225_4A2.001.007_HC | . | . | . | . | . | . | . | . | . | . | . | . | . | . | . | . | . | . | . | . | . | . | . | . | . |
| 21-225_4A2.001.008_HC | . | . | . | . | . | . | . | . | . | . | . | . | . | . | . | . | . | . | . | . | . | . | . | . | . |
| 21-225_4A2.001.009_HC | . | . | . | . | . | . | . | . | . | . | . | . | . | . | . | . | . | . | . | . | . | . | . | . | . |
| 21-225_4A2.001.010_HC | . | . | . | . | . | . | . | . | . | . | . | . | . | . | . | . | . | . | . | . | . | . | . | . | . |
| 21-225_4A2.001.011_HC | . | . | . | . | . | . | . | . | . | . | . | . | . | . | . | . | . | . | . | . | . | . | . | . | . |
| 21-225_4A2.001.012_HC | . | . | . | . | . | . | . | . | . | . | . | . | . | . | . | . | . | . | . | . | . | . | . | . | . |
| 21-225_4A2.001.022_HC | . | . | . | . | . | . | . | . | . | . | . | . | . | . | . | . | . | . | . | . | . | . | . | . | . |
| 21-225_4A2.001.023_HC | . | . | . | . | . | . | . | . | . | . | . | . | . | . | . | . | . | . | . | . | . | . | P | . | . |
| 21-225_4A2.001.024_HC | . | . | . | . | . | . | . | . | . | . | . | . | . | . | . | . | . | . | . | . | . | . | . | . | . |
| 21-225_4A2.001.028_HC | . | . | . | . | . | . | . | . | . | . | . | . | . | . | . | . | . | . | . | . | . | . | P | . | . |
| 21-225_4A2.001.029_HC | . | . | . | . | . | . | . | . | . | . | . | . | . | . | . | . | . | . | . | . | . | . | P | . | . |
| 21-225_4A2.001.031_HC | . | . | . | . | . | . | . | . | . | . | . | . | . | . | . | . | . | . | . | . | . | . | E | . | . |
| 21-225_56A1_HC | . | . | . | . | . | . | . | . | . | . | . | . | . | . | . | . | . | . | . | . | . | . | . | . | . |
| 21-225_58B9_HC | . | . | . | . | . | . | . | . | . | . | . | . | . | . | . | . | . | . | . | . | . | . | . | . | . |
| 21-225_59E1_HC | . | . | . | . | . | . | . | . | . | . | . | . | . | . | . | . | . | . | . | . | . | . | . | . | . |
| 21-225_59F10_HC | . | . | . | . | . | . | . | . | . | . | . | . | . | . | . | . | . | . | . | . | . | . | . | . | . |
| 21-225_60F3_HC | . | . | . | . | . | . | . | . | . | . | . | . | . | . | . | . | . | . | . | . | . | . | . | . | . |
| 21-225_61B5_HC | . | . | . | . | . | . | . | . | . | . | . | . | . | . | . | . | . | . | . | . | . | . | . | . | . |
| 21-225_61E3_HC | . | . | . | . | . | . | . | . | . | . | . | . | . | . | . | . | . | . | . | . | . | . | . | . | . |
| 21-225_63F4_HC | . | . | . | . | . | . | . | . | . | . | . | . | . | . | . | V | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | H_FR2 | |
| CONSENSUS | S | G | - | Y | T | F | T | N | - | - | - | - | - | Y | D | I | N | W | V | R | Q | A | T | G | Q |
| 21-225_6G6_HC | . | . | . | . | . | . | . | . | . | . | . | . | . | . | . | . | . | . | . | . | . | . | . | . | . |
| 21-225_70E7_HC | . | . | . | . | . | . | . | . | . | . | . | . | . | . | . | . | . | . | . | . | . | . | . | . | . |
| 21-225_71F3_HC | . | . | . | . | . | . | . | . | . | . | . | . | . | . | . | V | . | . | . | . | . | . | . | . | . |
| 21-225_71G3_HC | . | . | . | . | . | . | . | . | . | . | . | . | . | . | . | . | . | . | . | . | . | . | . | . | . |
| 21-225_72C4_HC | . | . | . | . | . | . | . | . | . | . | . | . | . | . | . | . | . | . | . | . | . | V | . | . | . |
| 21-225_74A6_HC | . | . | . | . | . | . | . | . | . | . | . | . | . | . | . | . | . | . | . | . | . | . | . | . | . |
| 21-225_74A8_HC | . | . | . | . | . | . | . | . | . | . | . | . | . | . | . | . | . | . | . | . | . | . | S | . | . |
| 21-225_74B11_HC | . | . | . | . | . | . | . | . | . | . | . | . | . | . | . | . | . | . | . | . | . | . | . | . | . |
| 21-225_74F6_HC | . | . | . | . | . | . | . | . | . | . | . | . | . | . | . | . | . | . | . | . | . | . | . | . | . |
| 21-225_74F9_HC | . | . | . | . | . | . | . | . | . | . | . | . | . | . | . | . | . | . | . | . | . | . | . | . | . |
| 21-225_74G4_HC | . | . | . | . | . | . | . | . | . | . | . | . | . | . | . | V | . | . | . | . | . | . | . | . | . |
| 21-225_75A10_HC | . | . | . | . | . | . | . | . | . | . | . | . | . | . | . | . | . | . | . | . | . | . | . | . | . |
| 21-225_75A6_HC | . | . | . | . | . | . | . | . | . | . | . | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . | . |
| 21-225_75F9_HC | . | . | . | . | . | . | . | . | . | . | . | . | . | . | . | . | . | . | . | . | . | . | . | . | . |
| 21-225_76E11_HC | . | . | . | . | . | . | . | . | . | . | . | . | . | . | . | . | . | . | . | . | . | . | . | . | . |
| 21-225_76G2_HC | . | . | . | . | . | . | . | . | . | . | . | . | . | . | . | . | . | . | . | . | . | . | . | . | . |
| 21-225_78E11_HC | . | . | . | . | . | . | . | . | . | . | . | . | . | . | . | . | . | . | . | . | . | . | . | . | . |
| 21-225_78E6_HC | . | . | . | . | . | . | . | . | . | . | . | . | . | . | . | . | . | . | . | . | . | . | S | . | . |
| 21-225_79E9_HC | . | . | . | . | . | . | . | . | . | . | . | . | . | . | . | . | . | . | . | . | . | . | . | . | . |
| 21-225_79F12_HC | . | . | . | . | . | . | . | . | . | . | . | . | . | . | . | . | . | . | . | . | . | . | . | . | . |
| 21-225_79G7_HC | . | . | . | . | . | . | . | . | . | . | . | . | . | . | . | . | . | . | . | . | . | . | S | . | . |
| 21-225_80B12_HC | . | . | . | . | . | . | . | . | . | . | . | . | . | . | . | . | . | . | . | . | . | . | . | . | . |
| 21-225_85C11_HC | . | . | . | . | . | . | . | . | . | . | . | . | . | . | . | . | . | . | . | . | . | . | S | . | . |
| 21-225_87E10_HC | . | . | . | . | . | . | . | . | . | . | . | . | . | . | . | . | . | . | . | . | . | . | . | . | . |
| 21-225_88A1_HC | . | . | . | . | . | . | . | . | . | . | . | . | . | . | . | . | . | . | . | . | . | . | . | . | . |
| 21-225_88B4_HC | . | . | . | . | . | . | . | . | . | . | . | . | . | . | . | . | . | . | . | . | . | . | . | . | . |
| 21-225_88C10_HC | . | . | . | . | . | . | S | . | . | . | . | . | . | . | . | . | . | . | . | . | . | . | . | . | . |
| 21-225_8B11_HC | . | . | . | . | . | . | . | . | . | . | . | . | . | . | . | . | . | . | . | . | . | . | A | . | . |

## FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | H_CDR1 | | | | | | | | | | | | | H_FR2 | |
| CONSENSUS | S | G | - | Y | T | F | T | N | - | - | - | - | - | Y | D | I | N | W | V | R | Q | A | T | G | Q |
| 21-225_92E6_HC | . | . | . | . | . | . | . | . | . | . | . | . | . | . | . | . | . | . | . | . | . | . | . | . | . |
| 21-225_94D8_HC | . | . | . | . | . | . | . | . | . | . | . | . | . | . | . | . | . | . | . | . | . | . | . | . | . |
| 21-225_96D10_HC | . | . | . | . | . | . | . | . | . | . | . | . | . | . | . | . | . | . | . | . | . | . | . | . | . |
| 21-225_96H5_HC | . | . | . | . | . | . | . | . | . | . | . | . | . | . | . | . | . | . | . | . | . | . | S | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | M | G | W | M | H | P | N | - | - | - | S | G | N | T | G | Y | A | Q | K | F | Q |
| 21-225_11E5_HC | . | . | . | . | . | . | . | . | . | . | . | . | . | . | . | . | A | . | . | . | . | . | R | . | . |
| 21-225_12F11_HC | . | P | . | . | . | . | . | . | . | . | . | . | . | . | . | . | . | . | . | . | . | K | . | . | . |
| 21-225_12F12_HC | . | . | . | . | . | . | . | . | Y | . | . | . | . | . | . | . | . | . | . | . | . | . | . | . | . |
| 21-225_146G9_HC | . | . | . | . | . | . | . | . | . | . | . | . | . | . | . | . | . | . | . | . | . | . | . | . | . |
| 21-225_146D4_HC | . | . | . | . | . | . | . | V | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | V | . | . | . | . | . | . | . | . | . | . | . | . | . | . | . | . | . |
| 21-225_147E4_HC | . | . | . | . | . | . | . | . | N | . | . | . | . | . | . | . | . | . | . | . | . | . | . | . | . |
| 21-225_148F8_HC | . | . | . | . | . | . | . | . | N | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | N | . | . |
| 21-225_149A2_HC | . | . | . | . | . | . | . | V | . | . | . | . | . | . | . | . | . | . | . | . | . | . | . | . | . |
| 21-225_149E3_HC | . | . | . | . | . | . | . | . | . | . | . | . | . | . | . | . | . | . | . | . | . | . | . | . | . |
| 21-225_149H4_HC | . | . | . | . | . | . | . | . | . | . | . | . | . | . | . | . | . | . | D | . | . | . | . | . | . |
| 21-225_150B7_HC | . | . | . | . | . | . | . | . | . | . | . | . | . | . | . | . | . | . | . | . | . | . | . | . | . |
| 21-225_150D2_HC | . | . | . | . | . | . | . | . | . | . | . | . | . | . | . | . | . | . | . | . | . | . | . | . | . |
| 21-225_152E12_HC | . | . | . | . | . | . | . | . | N | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | . | . | . | . | . | . | . | . | . | . | . | . | . | . | . | . | . | . | . |
| 21-225_153F11_HC | . | . | . | . | . | . | . | . | . | . | . | . | . | . | . | . | . | . | . | . | . | . | . | . | . |
| 21-225_154A11_HC | . | . | . | . | . | . | . | . | N | . | . | . | . | . | . | . | . | . | . | . | . | . | . | . | . |
| 21-225_154H6_HC | . | . | . | . | . | . | . | . | N | . | . | . | . | . | . | . | . | . | . | . | . | . | . | . | . |
| 21-225_155B6_HC | . | . | . | . | . | . | . | . | N | . | . | . | . | . | . | . | . | . | . | . | . | . | . | . | . |
| 21-225_155E5_HC | . | . | . | . | . | . | . | . | . | . | . | . | . | . | . | . | S | . | . | . | . | . | R | . | . |
| 21-225_156H1_HC | . | . | . | . | . | . | . | V | . | . | . | . | . | . | . | . | . | . | . | . | . | . | . | . | . |
| 21-225_158B12_HC | . | . | . | . | . | . | . | . | . | . | . | . | . | . | . | . | . | . | . | . | . | . | . | . | . |
| 21-225_160A4_HC | . | P | . | . | . | . | . | . | . | . | . | . | . | . | . | . | . | . | . | . | . | P | . | . | . |
| 21-225_160A7_HC | . | . | . | . | . | . | . | . | . | . | H | . | . | . | . | . | . | . | . | . | . | . | . | . | . |
| 21-225_162D5_HC | . | . | . | . | . | . | . | . | . | . | . | . | . | . | . | . | . | . | . | . | . | . | . | . | . |
| 21-225_162F6_HC | . | . | . | . | . | . | . | . | . | . | . | . | . | . | . | . | . | . | . | . | . | . | . | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | M | G | W | M | H | P | N | - | - | - | S | G | N | T | G | Y | A | Q | K | F | Q |
| 21-225_164A4_HC | . | . | . | . | . | . | . | . | . | . | . | . | . | . | . | . | . | . | . | . | . | . | . | . | . |
| 21-225_164A7_HC | . | . | . | . | . | . | . | . | . | . | . | . | . | . | . | . | . | . | . | . | . | . | . | . | . |
| 21-225_164B11_HC | . | . | . | . | . | . | . | . | T | . | . | . | . | . | . | . | . | . | . | . | . | . | . | . | . |
| 21-225_165H2_HC | . | . | . | . | V | . | . | . | . | . | . | . | . | . | . | H | K | . | . | . | . | . | . | . | . |
| 21-225_172E11_HC | . | . | . | . | . | . | . | . | . | . | . | . | . | . | . | . | . | . | . | . | . | . | . | . | . |
| 21-225_175D10_HC | . | . | . | . | . | . | . | . | . | . | . | . | . | . | . | . | . | . | . | . | . | . | . | . | . |
| 21-225_177B12_HC | . | . | . | . | . | . | . | . | . | . | . | . | . | . | . | . | . | . | . | . | . | . | . | . | . |
| 21-225_181E5_HC | . | . | . | . | . | . | . | . | N | . | . | . | . | . | . | . | . | . | . | . | . | . | . | . | . |
| 21-225_190D7_HC | . | . | . | . | . | . | . | . | N | . | . | . | . | . | . | . | . | . | . | . | . | . | . | . | . |
| 21-225_190H4_HC | . | . | . | . | . | . | . | . | N | . | . | . | . | . | . | . | . | . | . | . | . | . | . | . | . |
| 21-225_191D8_HC | . | . | . | . | . | . | . | . | . | . | . | . | . | . | . | . | . | . | . | . | . | . | . | . | . |
| 21-225_191G3_HC | . | . | . | . | . | . | R | . | N | . | . | . | . | . | . | . | . | . | . | . | . | . | . | . | . |
| 21-225_193G4_HC | . | . | . | . | . | . | . | . | N | . | . | . | . | . | . | . | . | . | . | . | . | . | . | . | . |
| 21-225_197C6_HC | . | . | . | . | . | . | . | . | . | . | . | . | . | . | . | . | . | . | . | . | . | . | . | . | . |
| 21-225_197H4_HC | . | . | . | . | . | . | . | . | . | . | . | . | . | . | . | . | . | . | . | . | . | . | . | . | . |
| 21-225_201F5_HC | . | . | . | . | . | . | . | . | . | . | . | . | . | . | . | . | . | . | . | . | . | . | . | . | . |
| 21-225_224C10_HC | . | . | . | . | . | . | . | . | . | . | . | . | . | . | . | . | . | . | . | . | . | . | . | . | . |
| 21-225_224D8_HC | . | . | . | . | . | . | . | L | Y | . | . | . | . | . | . | . | . | . | . | . | . | . | . | . | . |
| 21-225_224F11_HC | . | . | . | . | . | . | . | . | N | . | . | . | . | . | . | . | . | . | . | . | . | . | . | . | . |
| 21-225_225B9_HC | R | . | . | . | . | . | . | . | . | . | . | . | . | . | . | . | . | . | . | . | . | . | . | . | R |
| 21-225_225F11_HC | . | . | . | . | . | . | . | . | . | . | . | . | . | . | . | . | . | . | . | . | . | . | . | . | . |
| 21-225_225F12_HC | . | . | . | . | . | . | . | . | . | . | . | . | . | . | . | . | . | . | . | . | . | . | . | . | . |
| 21-225_225F5_HC | . | . | . | . | . | . | . | . | . | . | . | . | . | . | . | . | . | . | . | F | . | . | . | . | . |
| 21-225_225H12_HC | . | . | . | . | . | . | . | . | . | . | . | . | . | . | . | . | . | . | . | . | . | . | . | . | . |
| 21-225_226A12_HC | . | . | . | . | . | . | . | . | . | . | . | . | . | . | . | . | . | . | . | . | . | . | . | . | . |
| 21-225_226D11_HC | . | . | . | . | . | . | . | . | . | . | . | . | . | . | . | . | . | . | . | . | . | . | . | . | . |
| 21-225_226D6_HC | . | . | . | . | . | . | . | . | . | . | . | . | . | . | . | . | . | . | . | . | . | . | . | . | . |
| 21-225_226F6_HC | . | . | . | . | . | . | . | . | . | . | . | . | . | . | . | . | . | . | . | . | . | . | . | . | . |
| 21-225_227C7_HC | . | . | . | . | . | . | . | . | . | . | . | . | . | . | . | . | . | . | . | . | . | . | . | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H_CDR2 | | | | | | | | |
| CONSENSUS | G | L | E | W | M | G | W | M | H | P | N | - | - | - | S | G | N | T | G | Y | A | Q | K | F | Q |
| 21-225_227D11_HC | . | . | . | . | . | . | . | . | . | . | . | . | . | . | . | . | . | . | . | . | . | . | . | . | . |
| 21-225_227E6_HC | . | . | . | . | . | . | . | . | . | . | . | . | . | . | . | . | . | . | . | . | . | . | . | . | . |
| 21-225_22D2_HC | . | . | . | . | . | . | . | . | . | . | D | . | . | . | . | . | . | . | . | . | . | . | . | . | . |
| 21-225_23A12_HC | . | . | . | . | . | . | . | . | . | . | . | . | . | . | . | . | . | . | . | . | . | . | R | . | . |
| 21-225_24E1_HC | . | . | . | . | . | . | . | . | . | . | . | . | . | . | . | . | . | . | . | . | . | . | R | . | . |
| 21-225_25A4_HC | . | . | . | . | . | . | . | . | Y | . | . | . | . | . | . | . | . | . | . | . | . | . | . | . | . |
| 21-225_25A4.001_HC | . | . | . | . | . | . | . | . | Y | . | . | . | . | . | . | . | . | . | . | . | . | . | . | . | . |
| 21-225_25A4.001.001_HC | . | . | . | . | . | . | . | . | Y | . | . | . | . | . | . | . | S | . | . | . | . | . | . | . | . |
| 21-225_25A4.001.002_HC | . | . | . | . | . | . | . | . | Y | . | . | . | . | . | . | . | . | A | . | . | . | . | . | . | . |
| 21-225_25A4.001.003_HC | . | . | . | . | . | . | . | . | Y | . | . | . | . | . | . | . | . | V | . | . | . | . | . | . | . |
| 21-225_25A4.001.004_HC | . | . | . | . | . | . | . | . | Y | . | . | . | . | . | . | . | Q | . | . | . | . | . | . | . | . |
| 21-225_25A4.001.005_HC | . | . | . | . | . | . | . | . | Y | . | . | . | . | . | . | . | S | . | . | . | . | . | . | . | . |
| 21-225_25A4.001.006_HC | . | . | . | . | . | . | . | . | Y | . | . | . | . | . | . | . | . | A | . | . | . | . | . | . | . |
| 21-225_25A4.001.007_HC | . | . | . | . | . | . | . | . | Y | . | . | . | . | . | . | . | . | V | . | . | . | . | . | . | . |
| 21-225_25A4.001.008_HC | . | . | . | . | . | . | . | . | Y | . | . | . | . | . | . | . | Q | . | . | . | . | . | . | . | . |
| 21-225_25A4.001.013_HC | . | . | . | . | . | . | . | . | Y | . | . | . | . | . | . | . | S | . | . | . | . | . | . | . | . |
| 21-225_25A4.001.019_HC | . | . | . | . | . | . | . | . | Y | . | . | . | . | . | . | . | S | . | . | . | . | . | . | . | . |
| 21-225_25A4.001.020_HC | . | . | . | . | . | . | . | . | Y | . | . | . | . | . | . | . | Q | . | . | . | . | . | . | . | . |
| 21-225_25A4.001.021_HC | . | . | . | . | . | . | . | . | Y | . | . | . | . | . | . | . | . | A | . | . | . | . | . | . | . |
| 21-225_25A4.001.029_HC | . | . | . | . | . | . | . | . | Y | . | . | . | . | . | . | . | Q | . | . | . | . | . | . | . | . |
| 21-225_26C4_HC | . | . | . | . | . | . | . | . | N | . | . | . | . | . | . | . | . | . | . | . | . | . | R | . | . |
| 21-225_28B6_HC | . | . | . | . | . | . | . | . | . | . | D | . | . | . | . | . | . | . | . | . | . | . | . | . | . |
| 21-225_29E6_HC | . | . | . | . | . | . | . | . | . | . | . | . | . | . | . | . | . | . | . | . | . | . | . | . | . |
| 21-225_29H8_HC | . | . | . | . | . | . | . | . | N | . | . | . | . | . | . | . | . | . | . | . | . | . | R | . | . |
| 21-225_32A1_HC | . | . | . | . | . | . | . | . | N | . | . | . | . | . | . | . | . | . | . | . | . | . | . | . | . |
| 21-225_32A5_HC | . | . | . | . | . | . | . | . | . | . | . | . | . | . | . | . | . | . | . | F | . | . | . | . | R |
| 21-225_33D1_HC | . | . | . | . | . | . | . | . | . | . | . | . | . | . | . | . | . | . | . | F | . | . | . | . | R |
| 21-225_34D2_HC | . | . | . | V | . | . | L | . | . | . | . | . | . | . | . | . | T | . | . | F | . | . | . | . | R |
| 21-225_34H7_HC | . | . | . | . | . | . | . | . | N | . | . | . | . | . | . | . | . | . | . | . | . | . | . | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H_CDR2 | | | | | | | | |
| CONSENSUS | G | L | E | W | M | G | W | M | H | P | N | - | - | - | S | G | N | T | G | Y | A | Q | K | F | Q |
| 21-225_43D8_HC | . | . | . | . | . | . | . | . | . | . | . | . | . | . | . | . | . | . | . | . | . | . | . | . | . |
| 21-225_4A2_HC | . | . | . | . | . | . | . | . | . | . | . | . | . | . | . | . | . | . | . | . | . | . | . | . | . |
| 21-225_4A2.001_HC | . | . | . | . | . | . | . | . | . | . | . | . | . | . | . | . | . | . | . | . | . | . | . | . | . |
| 21-225_4A2.001.001_HC | . | . | . | . | . | . | . | . | . | . | . | . | . | . | . | . | . | . | . | . | . | . | . | . | . |
| 21-225_4A2.001.002_HC | . | . | . | . | . | . | . | . | . | . | . | . | . | . | . | . | . | . | . | . | . | . | . | . | . |
| 21-225_4A2.001.003_HC | . | . | . | . | . | . | . | . | . | . | . | . | . | . | . | . | . | . | . | . | . | . | . | . | . |
| 21-225_4A2.001.004_HC | . | . | . | . | . | . | . | . | . | . | . | . | . | . | . | . | . | A | . | . | . | . | . | . | . |
| 21-225_4A2.001.005_HC | . | . | . | . | . | . | . | . | . | . | . | . | . | . | . | . | S | . | . | . | . | . | . | . | . |
| 21-225_4A2.001.006_HC | . | . | . | . | . | . | . | . | . | . | . | . | . | . | . | . | Q | . | . | . | . | . | . | . | . |
| 21-225_4A2.001.007_HC | . | . | . | . | . | . | . | . | . | . | . | . | . | . | . | . | Q | . | . | . | . | . | . | . | . |
| 21-225_4A2.001.008_HC | . | . | . | . | . | . | . | . | . | . | . | . | . | . | . | . | . | A | . | . | . | . | . | . | . |
| 21-225_4A2.001.009_HC | . | . | . | . | . | . | . | . | . | . | . | . | . | . | . | . | Q | . | . | . | . | . | . | . | . |
| 21-225_4A2.001.010_HC | . | . | . | . | . | . | . | . | . | . | . | . | . | . | . | . | . | A | . | . | . | . | . | . | . |
| 21-225_4A2.001.011_HC | . | . | . | . | . | . | . | . | . | . | . | . | . | . | . | . | Q | . | . | . | . | . | . | . | . |
| 21-225_4A2.001.012_HC | . | . | . | . | . | . | . | . | . | . | . | . | . | . | . | . | S | . | . | . | . | . | . | . | . |
| 21-225_4A2.001.022_HC | . | . | . | . | . | . | . | . | . | . | . | . | . | . | . | . | . | . | . | . | . | . | . | . | . |
| 21-225_4A2.001.023_HC | . | . | . | . | . | . | . | . | . | . | . | . | . | . | . | . | . | . | . | . | . | . | . | . | . |
| 21-225_4A2.001.024_HC | . | . | . | . | . | . | . | . | . | . | . | . | . | . | . | . | Q | . | . | . | . | . | . | . | . |
| 21-225_4A2.001.028_HC | . | . | . | . | . | . | . | . | . | . | . | . | . | . | . | . | Q | . | . | . | . | . | . | . | . |
| 21-225_4A2.001.029_HC | . | . | . | . | . | . | . | . | . | . | . | . | . | . | . | . | S | . | . | . | . | . | . | . | . |
| 21-225_4A2.001.031_HC | . | . | . | . | . | . | . | . | . | . | . | . | . | . | . | . | . | E | . | . | . | . | . | . | . |
| 21-225_56A1_HC | . | . | . | L | . | . | . | . | . | . | . | . | . | . | . | . | . | . | . | . | . | . | . | . | . |
| 21-225_58B9_HC | . | . | . | . | . | . | . | . | N | . | . | . | . | . | . | . | S | . | . | . | . | . | . | . | . |
| 21-225_59E1_HC | . | . | . | . | . | . | . | . | T | . | . | . | . | . | . | . | . | . | . | . | . | . | . | . | . |
| 21-225_59F10_HC | . | . | . | . | . | . | . | . | N | . | . | . | . | . | . | . | . | . | . | . | . | . | . | . | . |
| 21-225_60F3_HC | . | . | . | . | . | . | . | . | . | . | . | . | . | . | . | . | . | . | . | . | . | . | . | . | . |
| 21-225_61B5_HC | . | . | . | . | . | . | . | . | . | . | . | . | . | . | . | . | S | . | . | . | . | . | . | . | . |
| 21-225_61E3_HC | . | . | . | . | . | . | . | . | T | . | . | . | . | . | . | . | . | . | . | . | . | . | . | . | . |
| 21-225_63F4_HC | . | . | . | . | . | . | . | . | . | . | . | . | . | . | . | . | . | . | . | . | . | . | . | . | R |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H_CDR2 | | | | | | | | |
| CONSENSUS | G | L | E | W | M | G | W | M | H | P | N | - | - | - | S | G | N | T | G | Y | A | Q | K | F | Q |
| 21-225_6G6_HC | . | P | . | . | . | . | . | . | . | . | . | . | . | . | . | . | . | . | . | . | . | K | . | . | . |
| 21-225_70E7_HC | . | . | . | . | . | . | . | . | . | . | . | . | . | . | . | . | . | . | . | . | . | . | . | . | . |
| 21-225_71F3_HC | . | . | . | . | . | . | . | . | . | . | . | . | . | . | . | . | . | . | . | . | . | . | . | . | . |
| 21-225_71G3_HC | . | . | . | . | . | . | . | . | . | . | . | . | . | . | . | . | S | . | . | . | . | . | . | . | . |
| 21-225_72C4_HC | . | . | . | . | . | . | . | . | . | . | . | . | . | . | . | . | . | . | . | . | . | . | . | . | . |
| 21-225_74A6_HC | . | . | . | . | . | . | . | . | . | . | . | . | . | . | . | . | . | . | . | . | . | . | . | . | R |
| 21-225_74A8_HC | . | . | . | . | . | . | . | . | . | . | . | . | . | . | . | . | . | . | . | . | . | . | . | . | . |
| 21-225_74B11_HC | . | . | . | . | . | . | . | . | . | . | . | . | . | . | . | . | . | . | . | . | . | . | . | . | . |
| 21-225_74F6_HC | . | . | . | . | . | . | . | . | . | . | . | . | . | . | . | . | . | . | . | . | . | . | . | . | . |
| 21-225_74F9_HC | . | . | . | . | . | . | . | . | N | . | . | . | . | . | . | . | . | . | . | C | . | . | . | . | . |
| 21-225_74G4_HC | . | . | . | . | . | . | . | . | . | . | . | . | . | . | . | . | . | . | . | . | . | . | . | . | . |
| 21-225_75A10_HC | . | . | . | . | . | . | . | . | N | . | . | . | . | . | . | . | . | . | . | . | . | . | . | . | . |
| 21-225_75A6_HC | . | . | . | . | . | . | . | . | N | . | . | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . | . |
| 21-225_75F9_HC | . | . | . | . | . | . | . | . | . | . | . | . | . | . | . | . | . | . | . | . | . | . | . | . | . |
| 21-225_76E11_HC | . | . | . | . | . | . | . | . | N | . | . | . | . | . | . | . | . | . | . | . | . | . | . | . | . |
| 21-225_76G2_HC | . | . | . | . | . | . | . | . | N | . | . | . | . | . | . | . | . | . | . | . | . | . | . | . | . |
| 21-225_78E11_HC | . | . | . | . | . | . | . | . | N | . | . | . | . | . | . | . | . | . | . | C | . | . | . | . | . |
| 21-225_78E6_HC | . | . | . | . | . | . | . | . | . | . | . | . | . | . | . | . | . | . | . | . | . | . | . | . | . |
| 21-225_79E9_HC | . | . | . | . | . | . | . | . | . | . | . | . | . | . | . | . | . | . | . | . | . | . | . | . | . |
| 21-225_79F12_HC | . | . | . | . | . | . | . | . | N | . | . | . | . | . | . | . | . | . | . | . | . | . | . | . | . |
| 21-225_79G7_HC | . | . | . | . | . | . | . | . | . | . | . | . | . | . | . | . | . | . | . | . | . | . | . | . | . |
| 21-225_80B12_HC | . | . | . | . | . | . | . | . | N | . | . | . | . | . | . | . | . | . | . | . | . | . | . | . | . |
| 21-225_85C11_HC | . | . | . | . | . | . | . | . | . | . | . | . | . | . | . | . | . | . | . | . | . | . | . | . | . |
| 21-225_87E10_HC | . | . | . | . | . | . | . | . | N | . | . | . | . | . | . | . | . | . | . | . | . | . | . | . | . |
| 21-225_88A1_HC | . | . | . | . | . | . | . | . | N | . | . | . | . | . | . | . | . | . | . | . | . | . | . | . | . |
| 21-225_88B4_HC | . | . | . | . | . | . | . | . | N | . | . | . | . | . | . | . | D | . | . | . | . | . | . | . | . |
| 21-225_88C10_HC | . | . | . | . | . | . | . | . | T | . | . | . | . | . | . | . | . | . | . | . | . | . | . | . | . |
| 21-225_8B11_HC | . | . | . | . | . | . | . | . | . | . | . | . | . | . | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H_CDR2 | | | | | | | | |
| CONSENSUS | G | L | E | W | M | G | W | M | H | P | N | - | - | - | S | G | N | T | G | Y | A | Q | K | F | Q |
| 21-225_92E6_HC | . | . | . | . | . | . | . | . | . | . | . | . | . | . | . | . | . | . | . | . | . | . | . | . | . |
| 21-225_94D8_HC | . | . | . | . | . | . | . | . | N | . | . | . | . | . | . | . | . | . | . | . | . | . | . | . | . |
| 21-225_96D10_HC | . | . | . | . | . | . | . | . | . | . | . | . | . | . | . | . | . | . | . | . | . | . | . | . | . |
| 21-225_96H5_HC | . | . | . | . | . | . | . | . | . | . | . | . | . | . | . | . | . | . | . | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | | H_FR3 | | | | | | | |
| CONSENSUS | G | R | V | T | M | T | R | N | T | S | I | S | T | A | Y | M | E | L | S | S | L | R | S | E | D |
| 21-225_11E5_HC | . | . | . | . | . | . | . | . | . | . | . | . | . | . | . | . | . | . | . | . | . | . | . | . | . |
| 21-225_12F11_HC | . | . | . | . | . | . | . | . | . | . | . | . | A | . | . | . | V | . | . | . | . | . | . | . | . |
| 21-225_12F12_HC | . | . | . | . | . | . | M | . | . | . | . | . | . | . | . | . | . | . | . | . | . | . | . | . | . |
| 21-225_146C9_HC | . | . | . | . | . | . | W | . | . | . | . | . | . | . | . | . | A | . | . | . | . | . | . | . | . |
| 21-225_146D4_HC | . | . | . | . | . | . | . | . | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | . | . | . | . | . | . | . | . | . | . | . | . | . | . | . | . | G | . |
| 21-225_147E4_HC | . | . | . | . | . | . | . | . | . | . | . | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | . | . | . |
| 21-225_149A2_HC | . | . | . | . | . | . | . | . | . | . | . | . | . | . | . | . | . | . | . | . | . | . | . | . | . |
| 21-225_149E3_HC | . | . | . | . | . | . | . | . | . | . | . | . | . | . | . | . | . | . | . | . | . | T | . | . | . |
| 21-225_149H4_HC | . | . | . | . | . | . | . | . | . | . | . | T | . | V | . | . | . | . | . | . | . | . | . | . | . |
| 21-225_150B7_HC | . | . | . | . | . | . | . | . | . | . | . | . | . | . | . | L | . | . | . | . | . | . | . | . | . |
| 21-225_150D2_HC | . | . | . | . | . | . | . | . | . | . | . | . | . | . | . | . | . | . | . | . | . | . | . | . | . |
| 21-225_152E12_HC | . | . | . | . | . | . | . | . | . | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | . | . | . | . | . | . | . | . | . | . | . | . | . | . | . | . | . | . | . |
| 21-225_153F11_HC | . | . | . | . | . | . | . | . | . | . | . | N | . | . | . | . | . | . | . | . | . | . | . | . | . |
| 21-225_154A11_HC | . | . | . | . | . | . | . | . | . | . | . | . | . | . | . | . | . | . | . | . | . | . | . | . | . |
| 21-225_154H6_HC | . | . | . | . | . | . | . | . | . | . | . | . | . | . | . | . | . | . | . | . | . | . | . | . | . |
| 21-225_155B6_HC | . | . | . | . | . | . | . | . | . | . | . | . | . | . | . | . | . | . | . | . | . | . | . | . | . |
| 21-225_155E5_HC | . | . | . | . | . | . | . | . | . | . | . | . | . | . | . | . | . | . | . | . | . | . | . | . | . |
| 21-225_156H1_HC | . | . | . | . | . | . | . | . | . | . | . | . | . | . | . | . | . | . | . | . | . | . | . | . | . |
| 21-225_158B12_HC | . | . | F | . | . | . | . | . | . | . | . | . | . | . | . | . | . | . | . | . | . | . | . | . | . |
| 21-225_160A4_HC | . | . | . | . | . | . | . | . | . | . | . | . | . | . | . | . | . | . | . | . | . | . | . | . | . |
| 21-225_160A7_HC | . | . | . | . | . | . | . | . | . | . | . | N | . | . | . | . | . | . | . | . | . | . | . | . | . |
| 21-225_162D5_HC | . | . | . | . | . | . | . | . | . | . | . | D | . | . | . | . | . | . | . | . | S | . | . | . | . |
| 21-225_162F6_HC | . | . | F | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | | H_FR3 | | | | | | | |
| CONSENSUS | G | R | V | T | M | T | R | N | T | S | I | S | T | A | Y | M | E | L | S | S | L | R | S | E | D |
| 21-225_164A4_HC | . | . | . | . | . | . | . | . | . | . | . | . | . | . | . | . | . | . | . | . | . | . | . | . | . |
| 21-225_164A7_HC | . | . | . | . | . | . | . | . | . | . | T | . | . | . | . | . | . | . | . | . | . | . | . | . | . |
| 21-225_164B11_HC | . | . | . | . | . | . | . | . | . | . | M | . | . | . | . | . | . | . | . | . | . | . | . | . | . |
| 21-225_165H2_HC | . | . | . | . | . | . | . | . | . | . | N | . | . | . | . | . | D | . | . | . | . | . | . | . | . |
| 21-225_172E11_HC | . | . | . | . | . | . | . | . | . | . | . | . | . | . | . | . | . | . | . | . | . | . | . | . | . |
| 21-225_175D10_HC | . | . | . | . | . | . | . | . | . | . | . | . | . | V | . | . | . | . | . | . | . | . | . | . | . |
| 21-225_177B12_HC | . | . | . | . | . | . | . | . | . | . | . | . | . | . | . | . | . | . | . | . | . | . | . | . | . |
| 21-225_181E5_HC | . | . | . | . | . | . | . | . | . | . | . | . | . | . | . | . | . | . | . | . | . | . | . | . | . |
| 21-225_190D7_HC | . | . | . | . | . | . | . | . | . | . | . | . | . | . | . | . | . | . | . | . | . | . | . | . | . |
| 21-225_190H4_HC | . | . | . | . | . | . | . | . | . | . | . | . | . | . | . | . | . | . | . | . | . | . | . | . | . |
| 21-225_191D8_HC | . | . | . | . | . | . | . | . | . | . | . | . | . | . | . | . | . | . | . | . | . | . | . | . | . |
| 21-225_191G3_HC | . | . | . | . | . | . | . | . | . | . | . | . | . | . | . | . | . | . | . | . | . | . | . | . | . |
| 21-225_193G4_HC | . | . | . | . | . | . | . | . | . | . | . | R | . | . | . | . | . | . | N | . | . | . | . | . | . |
| 21-225_197C6_HC | . | . | . | . | . | . | . | . | . | . | . | . | . | . | . | . | . | . | . | . | . | . | . | . | . |
| 21-225_197H4_HC | . | . | . | . | . | . | . | . | . | . | . | . | . | . | . | . | . | . | . | . | . | . | . | . | . |
| 21-225_201F5_HC | . | . | . | . | . | . | . | . | . | . | . | . | . | . | . | . | . | . | . | . | . | . | . | . | . |
| 21-225_224C10_HC | . | . | . | . | . | . | . | . | . | . | . | . | . | . | . | . | . | . | . | . | . | . | . | . | . |
| 21-225_224D8_HC | . | . | . | . | . | . | . | . | . | . | . | . | . | . | . | . | . | . | . | . | . | . | . | . | . |
| 21-225_224F11_HC | . | . | . | . | . | . | . | . | . | . | . | . | . | . | . | . | . | . | . | . | . | . | . | . | . |
| 21-225_225B9_HC | . | . | . | . | . | . | . | . | . | . | . | N | . | . | . | . | . | . | N | . | . | . | . | . | . |
| 21-225_225F11_HC | . | . | . | . | . | . | . | . | . | . | . | N | . | . | . | . | . | . | . | . | . | . | . | . | . |
| 21-225_225F12_HC | . | . | . | . | A | . | . | . | . | . | . | . | . | . | . | . | . | . | . | . | . | . | . | . | . |
| 21-225_225F5_HC | . | . | . | . | . | . | . | . | . | . | . | . | . | . | . | . | . | . | . | . | . | . | . | . | . |
| 21-225_225H12_HC | . | . | . | . | . | . | . | . | . | . | . | . | . | . | . | . | . | . | . | . | . | . | . | . | . |
| 21-225_226A12_HC | . | . | . | . | . | . | . | . | . | . | . | . | . | . | . | . | . | . | . | . | . | . | . | . | . |
| 21-225_226D11_HC | . | . | . | . | . | . | . | . | . | . | . | . | . | . | . | . | . | . | . | . | . | . | . | . | . |
| 21-225_226D6_HC | . | . | . | . | . | . | . | . | . | . | . | . | . | . | . | . | . | . | . | . | . | . | . | . | . |
| 21-225_226F6_HC | . | . | . | . | . | . | . | . | . | . | . | N | . | . | . | . | . | . | . | . | . | . | . | . | . |
| 21-225_227C7_HC | . | . | . | . | . | . | . | . | . | . | . | . | . | . | . | . | . | . | . | . | . | . | . | . | . |

3667

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | | H_FR3 | | | | | | | |
| CONSENSUS | G | R | V | T | M | T | R | N | T | S | I | S | T | A | Y | M | E | L | S | S | L | R | S | E | D |
| 21-225_227D11_HC | . | . | . | . | . | . | . | . | . | . | . | . | . | . | . | . | . | . | . | . | . | . | . | . | . |
| 21-225_227E6_HC | . | . | . | . | . | . | . | . | . | . | . | . | . | . | . | . | . | . | . | . | . | . | . | . | . |
| 21-225_22D2_HC | . | . | . | . | . | . | . | . | . | . | . | . | . | . | . | . | . | . | . | . | . | . | . | . | . |
| 21-225_23A12_HC | . | . | . | . | . | . | . | . | . | . | . | N | . | . | . | . | . | . | . | . | . | . | . | . | . |
| 21-225_24E1_HC | . | . | . | . | . | . | . | . | . | . | . | . | . | . | . | . | . | . | . | . | . | . | . | . | . |
| 21-225_25A4_HC | . | . | . | . | . | . | . | . | . | . | . | . | . | . | . | . | . | . | . | . | . | . | . | . | . |
| 21-225_25A4.001_HC | . | . | . | . | . | . | . | D | . | . | . | . | . | . | . | . | . | . | . | . | . | . | . | . | . |
| 21-225_25A4.001.001_HC | . | . | . | . | . | . | . | D | . | . | . | . | . | . | . | . | . | . | . | . | . | . | . | . | . |
| 21-225_25A4.001.002_HC | . | . | . | . | . | . | . | D | . | . | . | . | . | . | . | . | . | . | . | . | . | . | . | . | . |
| 21-225_25A4.001.003_HC | . | . | . | . | . | . | . | D | . | . | . | . | . | . | . | . | . | . | . | . | . | . | . | . | . |
| 21-225_25A4.001.004_HC | . | . | . | . | . | . | . | D | . | . | . | . | . | . | . | . | . | . | . | . | . | . | . | . | . |
| 21-225_25A4.001.005_HC | . | . | . | . | . | . | . | D | . | . | . | . | . | . | . | . | . | . | . | . | . | . | . | . | . |
| 21-225_25A4.001.006_HC | . | . | . | . | . | . | . | D | . | . | . | . | . | . | . | . | . | . | . | . | . | . | . | . | . |
| 21-225_25A4.001.007_HC | . | . | . | . | . | . | . | D | . | . | . | . | . | . | . | . | . | . | . | . | . | . | . | . | . |
| 21-225_25A4.001.008_HC | . | . | . | . | . | . | . | D | . | . | . | . | . | . | . | . | . | . | . | . | . | . | . | . | . |
| 21-225_25A4.001.013_HC | . | . | . | . | . | . | . | D | . | . | . | . | . | . | . | . | . | . | . | . | . | . | . | . | . |
| 21-225_25A4.001.019_HC | . | . | . | . | . | . | . | D | . | . | . | . | . | . | . | . | . | . | . | . | . | . | . | . | . |
| 21-225_25A4.001.020_HC | . | . | . | . | . | . | . | D | . | . | . | . | . | . | . | . | . | . | . | . | . | . | . | . | . |
| 21-225_25A4.001.021_HC | . | . | . | . | . | . | . | D | . | . | . | . | . | . | . | . | . | . | . | . | . | . | . | . | . |
| 21-225_25A4.001.029_HC | . | . | . | . | . | . | . | D | . | . | . | . | . | . | . | . | . | . | . | . | . | . | . | . | . |
| 21-225_26C4_HC | . | . | . | . | . | . | . | . | . | . | . | N | . | . | . | . | . | . | . | . | . | . | . | . | . |
| 21-225_28B6_HC | . | . | . | . | . | . | . | . | . | . | . | . | . | . | . | . | . | . | . | . | . | . | . | . | . |
| 21-225_29E6_HC | . | . | . | . | . | . | . | . | . | . | . | . | . | . | . | . | . | . | . | . | . | . | . | . | . |
| 21-225_29H8_HC | . | . | . | . | . | . | . | . | . | . | . | . | . | A | . | . | . | . | . | . | . | . | . | . | . |
| 21-225_32A1_HC | . | . | . | . | . | . | . | . | . | . | . | . | . | . | . | . | . | . | . | . | . | . | . | . | . |
| 21-225_32A5_HC | . | . | . | . | . | . | . | . | . | . | . | . | . | . | . | . | . | . | . | . | . | . | . | . | . |
| 21-225_33D1_HC | . | . | . | . | . | . | . | . | . | . | . | . | . | . | . | . | . | . | . | . | . | . | . | . | . |
| 21-225_34D2_HC | . | . | . | . | . | . | . | . | . | . | . | . | . | . | . | L | . | . | . | . | . | . | . | . | . |
| 21-225_34H7_HC | . | . | . | . | . | . | . | . | . | . | . | . | . | . | . | . | . | . | N | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
|  |  |  |  |  |  |  |  |  |  |  |  |  |  |  |  |  | H_FR3 |  |  |  |  |  |  |  |  |
| CONSENSUS | G | R | V | T | M | T | R | N | T | S | I | S | T | A | Y | M | E | L | S | S | L | R | S | E | D |
| 21-225_43D8_HC | . | . | . | . | . | . | . | . | . | . | . | . | . | . | . | . | . | . | . | . | . | T | . | . | . |
| 21-225_4A2_HC | . | . | . | . | L | . | . | . | . | . | . | . | . | . | . | . | . | . | . | . | . | . | . | . | . |
| 21-225_4A2.001_HC | . | . | . | . | L | . | . | D | . | . | . | . | . | . | . | . | . | . | . | . | . | . | . | . | . |
| 21-225_4A2.001.001_HC | . | . | . | . | L | . | . | D | . | . | . | . | . | . | . | . | . | . | . | . | . | . | . | . | . |
| 21-225_4A2.001.002_HC | . | . | . | . | L | . | . | D | . | . | . | . | . | . | . | . | . | . | . | . | . | . | . | . | . |
| 21-225_4A2.001.003_HC | . | . | . | . | L | . | . | D | . | . | . | . | . | . | . | . | . | . | . | . | . | . | . | . | . |
| 21-225_4A2.001.004_HC | . | . | . | . | L | . | . | D | . | . | . | . | . | . | . | . | . | . | . | . | . | . | . | . | . |
| 21-225_4A2.001.005_HC | . | . | . | . | L | . | . | D | . | . | . | . | . | . | . | . | . | . | . | . | . | . | . | . | . |
| 21-225_4A2.001.006_HC | . | . | . | . | L | . | . | D | . | . | . | . | . | . | . | . | . | . | . | . | . | . | . | . | . |
| 21-225_4A2.001.007_HC | . | . | . | . | L | . | . | D | . | . | . | . | . | . | . | . | . | . | . | . | . | . | . | . | . |
| 21-225_4A2.001.008_HC | . | . | . | . | L | . | . | D | . | . | . | . | . | . | . | . | . | . | . | . | . | . | . | . | . |
| 21-225_4A2.001.009_HC | . | . | . | . | L | . | . | D | . | . | . | . | . | . | . | . | . | . | . | . | . | . | . | . | . |
| 21-225_4A2.001.010_HC | . | . | . | . | L | . | . | D | . | . | . | . | . | . | . | . | . | . | . | . | . | . | . | . | . |
| 21-225_4A2.001.011_HC | . | . | . | . | L | . | . | D | . | . | . | . | . | . | . | . | . | . | . | . | . | . | . | . | . |
| 21-225_4A2.001.012_HC | . | . | . | . | L | . | . | D | . | . | . | . | . | . | . | . | . | . | . | . | . | . | . | . | . |
| 21-225_4A2.001.022_HC | . | . | . | . | L | . | . | D | . | . | . | . | . | . | . | . | . | . | . | . | . | . | . | . | . |
| 21-225_4A2.001.023_HC | . | . | . | . | L | . | . | D | . | . | . | . | . | . | . | . | . | . | . | . | . | . | . | . | . |
| 21-225_4A2.001.024_HC | . | . | . | . | L | . | . | D | . | . | . | . | . | . | . | . | . | . | . | . | . | . | . | . | . |
| 21-225_4A2.001.028_HC | . | . | . | . | L | . | . | D | . | . | . | . | . | . | . | . | . | . | . | . | . | . | . | . | . |
| 21-225_4A2.001.029_HC | . | . | . | . | L | . | . | D | . | . | . | . | . | . | . | . | . | . | . | . | . | . | . | . | . |
| 21-225_4A2.001.031_HC | . | . | . | . | L | . | . | D | . | . | . | . | . | . | . | . | . | . | . | . | . | . | . | . | . |
| 21-225_56A1_HC | . | . | . | . | . | . | . | . | . | . | . | . | . | . | . | . | . | . | . | . | . | . | . | . | . |
| 21-225_58B9_HC | . | . | . | . | . | . | . | . | . | . | . | . | . | . | . | . | . | . | . | . | . | . | . | . | . |
| 21-225_59E1_HC | . | . | . | . | . | . | . | T | . | . | . | . | . | . | . | . | . | . | . | . | . | . | . | . | . |
| 21-225_59F10_HC | . | . | . | . | . | . | . | . | . | . | . | . | . | . | . | . | . | . | . | . | . | . | . | . | . |
| 21-225_60F3_HC | . | . | . | . | . | . | . | . | . | . | . | . | . | . | . | . | . | . | . | . | . | . | . | . | . |
| 21-225_61B5_HC | . | . | . | . | . | . | . | . | . | . | . | . | . | . | . | . | . | . | . | . | . | . | . | . | . |
| 21-225_61E3_HC | . | . | . | . | . | . | . | . | . | . | . | . | . | . | . | . | . | . | . | . | . | K | . | . | . |
| 21-225_63F4_HC | . | . | . | . | . | . | . | . | . | . | L | . | . | V | . | . | . | . | . | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | | H_FR3 | | | | | | | |
| CONSENSUS | G | R | V | T | M | T | R | N | T | S | I | S | T | A | Y | M | E | L | S | S | L | R | S | E | D |
| 21-225_6G6_HC | . | . | . | . | . | . | . | . | . | . | . | . | A | . | . | . | V | . | . | . | . | . | . | . | . |
| 21-225_70E7_HC | . | . | . | . | . | . | . | . | . | . | . | . | . | . | . | . | . | . | . | . | . | . | . | . | . |
| 21-225_71F3_HC | . | . | . | . | . | . | . | . | . | . | . | . | . | . | . | . | . | . | . | . | . | . | . | . | . |
| 21-225_71G3_HC | . | . | . | . | . | . | . | D | . | . | V | . | . | . | . | . | . | . | . | . | . | . | . | . | . |
| 21-225_72C4_HC | . | . | . | . | . | . | . | . | . | . | . | . | . | . | . | . | . | . | N | . | . | . | . | . | . |
| 21-225_74A6_HC | . | . | . | . | . | . | W | . | . | . | . | . | . | . | . | . | . | . | . | . | . | . | . | . | . |
| 21-225_74A8_HC | . | . | . | . | . | . | . | . | . | . | . | . | . | . | . | . | . | . | . | . | . | . | . | . | . |
| 21-225_74B11_HC | . | . | . | . | . | . | . | . | . | . | . | . | . | . | . | . | . | . | . | . | . | . | . | . | . |
| 21-225_74F6_HC | . | . | . | . | . | . | . | . | . | . | M | . | . | . | . | . | . | . | . | . | . | . | . | . | . |
| 21-225_74F9_HC | . | . | . | . | . | . | . | . | . | . | . | . | . | . | . | . | . | . | . | . | . | . | . | . | . |
| 21-225_74G4_HC | . | . | . | . | . | . | . | . | . | . | . | R | . | . | . | . | . | . | . | . | . | . | . | . | . |
| 21-225_75A10_HC | . | . | . | . | . | . | . | . | . | . | . | . | . | . | . | . | . | . | . | . | . | . | . | . | . |
| 21-225_75A6_HC | . | . | . | . | . | . | . | . | . | . | . | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . | . |
| 21-225_75F9_HC | . | . | . | . | . | . | . | . | . | . | . | . | . | . | . | . | . | . | . | . | . | . | . | . | . |
| 21-225_76E11_HC | . | . | L | . | . | . | . | . | . | . | . | . | . | . | . | . | . | . | . | . | . | . | . | . | . |
| 21-225_76G2_HC | . | . | . | . | . | . | . | . | . | . | . | . | . | . | . | . | . | . | . | . | . | . | . | . | . |
| 21-225_78E11_HC | . | . | . | . | . | . | . | . | . | . | . | . | . | . | . | . | . | . | . | . | . | . | . | . | . |
| 21-225_78E6_HC | . | . | . | . | . | . | . | . | . | . | . | . | . | . | . | . | . | . | . | . | . | . | . | . | . |
| 21-225_79E9_HC | . | . | . | . | . | . | . | . | . | . | . | . | . | . | . | . | . | . | . | . | . | . | . | . | . |
| 21-225_79F12_HC | . | . | . | . | . | . | . | . | . | . | . | . | . | . | . | . | . | . | . | . | . | . | . | . | . |
| 21-225_79G7_HC | . | . | . | . | . | . | . | . | . | . | . | . | . | . | . | . | . | . | . | . | . | . | . | . | . |
| 21-225_80B12_HC | V | . | . | . | . | . | . | . | . | . | . | . | . | . | . | . | . | . | . | . | . | . | . | . | . |
| 21-225_85C11_HC | . | . | . | . | . | . | . | . | . | . | . | . | . | . | . | . | . | . | . | . | . | . | . | . | . |
| 21-225_87E10_HC | . | . | . | . | . | . | . | . | . | . | . | . | . | . | . | . | . | . | . | . | . | . | . | . | . |
| 21-225_88A1_HC | . | . | . | . | . | . | . | . | . | . | . | . | . | . | . | . | . | . | . | . | . | . | . | . | . |
| 21-225_88B4_HC | . | S | . | . | . | . | . | . | . | . | . | . | T | . | . | . | . | . | . | . | . | . | . | . | . |
| 21-225_88C10_HC | . | . | . | . | . | . | . | . | . | . | . | . | . | . | . | . | . | . | . | . | . | . | . | . | . |
| 21-225_8B11_HC | . | . | . | . | . | . | . | . | . | . | . | R | . | . | . | . | . | . | . | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | V | T | M | T | R | N | T | S | I | S | T | A | Y | M | E | L | S | S | L | R | S | E | D |
| 21-225_92E6_HC | . | . | . | . | . | . | . | . | . | . | M | . | . | . | . | . | . | . | . | . | . | . | . | . | . |
| 21-225_94D8_HC | V | . | . | . | . | . | . | . | . | . | . | . | . | . | . | . | V | . | . | . | . | . | . | . | . |
| 21-225_96D10_HC | . | . | . | . | . | . | . | . | . | . | M | . | . | . | . | . | . | . | . | . | . | . | . | . | . |
| 21-225_96H5_HC | . | . | . | . | . | . | . | . | . | . | . | . | . | . | . | . | . | . | . | . | . | . | . | . | . |

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_11E5_HC | . | . | . | . | . | . | . | T | . | . | . | . | E | . | . | . | . | . | . | . | . | . | . | . | . |
| 21-225_12F11_HC | . | . | . | . | . | . | . | L | . | . | . | . | . | . | . | . | . | . | . | . | . | . | . | . | . |
| 21-225_12F12_HC | . | . | . | . | . | . | . | M | . | . | . | . | . | . | . | . | . | . | . | . | . | . | . | . | . |
| 21-225_146C9_HC | . | . | . | . | . | . | . | Y | . | . | . | . | . | . | . | . | . | . | . | . | . | . | . | . | . |
| 21-225_146D4_HC | . | . | . | . | . | . | . | G | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | G | . | . | . | . | . | . | . | . | . | . | . | . | . | . | . | . | . |
| 21-225_147E4_HC | . | . | . | . | . | . | . | . | . | . | . | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | . | . | . |
| 21-225_149A2_HC | . | . | . | . | . | . | . | G | . | . | . | . | . | . | . | . | . | . | . | . | . | . | . | . | . |
| 21-225_149E3_HC | . | . | . | . | . | . | . | Y | . | . | . | . | . | . | . | . | . | . | . | . | . | . | . | . | . |
| 21-225_149H4_HC | . | . | . | . | . | . | T | F | . | . | . | . | . | . | . | . | . | . | . | . | . | . | . | . | . |
| 21-225_150B7_HC | . | . | . | . | . | . | . | . | . | . | . | . | . | . | . | . | . | . | . | . | . | . | . | . | . |
| 21-225_150D2_HC | . | . | . | . | . | . | . | Y | . | . | . | . | . | . | . | . | . | . | . | . | . | . | . | . | . |
| 21-225_152E12_HC | . | . | . | . | . | . | . | Y | . | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | . | Y | . | . | . | . | . | . | . | . | . | . | . | . | . | . | . | . | . |
| 21-225_153F11_HC | . | . | . | . | . | . | . | Y | . | . | . | . | . | . | . | . | . | . | . | . | . | . | . | . | . |
| 21-225_154A11_HC | . | . | . | . | . | . | . | . | . | . | . | . | . | . | . | . | . | . | . | . | . | . | . | . | . |
| 21-225_154H6_HC | . | . | . | . | . | . | . | Y | . | . | . | . | . | . | . | . | . | . | . | . | . | . | . | . | . |
| 21-225_155B6_HC | . | . | . | . | . | . | . | Y | . | . | . | . | . | . | . | . | . | . | . | . | . | . | . | . | . |
| 21-225_155E5_HC | . | . | . | . | . | . | . | F | . | . | . | . | . | . | . | . | . | . | . | . | . | . | . | . | . |
| 21-225_156H1_HC | . | . | . | . | . | . | . | G | . | . | . | . | . | . | . | . | . | . | . | . | . | . | . | . | . |
| 21-225_158B12_HC | . | . | . | . | . | . | . | Y | . | . | . | . | . | . | . | . | . | . | . | . | . | . | . | . | . |
| 21-225_160A4_HC | . | . | . | . | . | . | . | Y | . | . | . | . | . | . | . | . | . | . | . | . | . | . | . | . | . |
| 21-225_160A7_HC | . | . | . | . | . | . | . | H | . | . | . | . | . | . | . | . | . | . | . | . | . | . | . | . | . |
| 21-225_162D5_HC | . | . | . | . | F | . | . | F | . | . | . | . | . | . | . | . | . | . | . | . | . | . | . | . | . |
| 21-225_162F6_HC | . | . | . | . | . | . | . | Y | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_164A4_HC | . | . | . | . | . | . | . | . | . | . | . | . | . | . | . | . | . | . | . | . | . | . | . | . | . |
| 21-225_164A7_HC | S | . | . | . | . | . | . | . | . | . | . | . | . | . | . | . | . | . | . | . | . | . | . | . | . |
| 21-225_164B11_HC | S | . | . | . | . | . | S | Y | . | . | . | . | . | . | . | . | . | . | . | . | . | . | . | . | . |
| 21-225_165H2_HC | . | . | . | . | . | . | . | Y | . | . | . | . | . | . | . | . | . | . | . | . | . | . | . | . | . |
| 21-225_172E11_HC | . | . | . | . | . | . | . | Y | . | . | . | . | . | . | . | . | . | . | . | . | . | . | . | . | . |
| 21-225_175D10_HC | . | . | . | . | . | . | . | Y | . | . | . | . | . | . | . | . | . | . | . | . | . | . | . | . | . |
| 21-225_177B12_HC | . | . | . | . | . | . | . | Y | . | . | . | . | . | . | . | . | . | . | . | . | . | . | . | . | . |
| 21-225_181E5_HC | . | . | . | . | . | . | . | . | . | . | . | . | . | . | . | . | . | . | . | . | . | . | . | . | . |
| 21-225_190D7_HC | . | . | . | . | . | . | . | . | . | . | . | . | . | . | . | . | . | . | . | . | . | . | . | . | . |
| 21-225_190H4_HC | . | . | . | . | . | . | . | H | . | . | . | . | . | . | . | . | . | . | . | . | . | . | . | . | . |
| 21-225_191D8_HC | . | . | . | . | . | . | . | H | . | . | . | . | . | . | . | . | . | . | . | . | . | . | . | . | . |
| 21-225_191G3_HC | . | . | . | . | . | . | . | H | . | . | . | . | . | . | . | . | . | . | . | . | . | . | . | . | . |
| 21-225_193G4_HC | . | . | . | . | . | . | . | . | . | . | . | . | . | . | . | . | . | . | . | . | . | . | . | . | . |
| 21-225_197C6_HC | . | . | . | . | . | . | . | H | . | . | . | . | . | . | . | . | . | . | . | . | . | . | . | . | . |
| 21-225_197H4_HC | . | . | . | . | . | . | . | H | . | . | . | . | . | . | . | . | . | . | . | . | . | . | . | . | . |
| 21-225_201F5_HC | . | . | . | . | . | . | . | Y | . | . | . | . | . | . | . | . | . | . | . | . | . | . | . | . | . |
| 21-225_224C10_HC | . | . | . | . | . | . | . | Y | . | . | . | . | . | . | . | . | . | . | . | . | . | . | . | . | . |
| 21-225_224D8_HC | . | . | . | . | . | . | . | Y | . | . | . | . | . | . | . | . | . | . | . | . | . | . | . | . | . |
| 21-225_224F11_HC | . | . | . | . | . | . | . | Y | . | . | . | . | . | . | . | . | . | . | . | . | . | . | . | . | . |
| 21-225_225B9_HC | . | . | . | . | . | . | . | Y | . | . | . | . | T | . | . | . | . | . | . | . | . | . | . | . | . |
| 21-225_225F11_HC | . | . | . | . | . | . | . | Y | . | . | . | . | . | . | . | . | . | . | . | . | . | . | . | . | . |
| 21-225_225F12_HC | . | . | . | . | . | . | . | Y | . | . | . | . | . | . | . | . | . | . | . | . | . | . | . | . | . |
| 21-225_225F5_HC | . | . | . | . | . | . | . | Y | . | . | . | . | . | . | . | . | . | . | . | . | . | . | . | . | . |
| 21-225_225H12_HC | . | . | . | . | . | . | . | Y | . | . | . | . | . | . | . | . | . | . | . | . | . | . | . | . | . |
| 21-225_226A12_HC | . | . | . | . | . | . | . | Y | . | . | . | . | . | . | . | . | . | . | . | . | . | . | . | . | . |
| 21-225_226D11_HC | . | . | . | . | . | . | . | Y | . | . | . | . | . | . | . | . | . | . | . | . | . | . | . | . | . |
| 21-225_226D6_HC | . | . | . | . | . | . | . | Y | . | . | . | . | . | . | . | . | . | . | . | . | . | . | . | . | . |
| 21-225_226F6_HC | . | . | . | . | . | . | . | Y | . | . | . | . | . | . | . | . | . | . | . | . | . | . | . | . | . |
| 21-225_227C7_HC | . | . | . | . | . | . | . | Y | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_227D11_HC | . | . | . | . | . | . | . | Y | . | . | . | . | . | . | . | . | . | . | . | . | . | . | . | . | . |
| 21-225_227E6_HC | . | . | . | . | . | . | . | Y | . | . | . | . | . | . | . | . | . | . | . | . | . | . | . | . | . |
| 21-225_22D2_HC | . | . | . | . | . | . | . | Y | . | . | . | . | . | . | . | . | . | . | . | . | . | . | . | . | . |
| 21-225_23A12_HC | . | . | . | . | . | . | . | G | . | . | . | . | . | . | . | . | . | . | . | . | . | . | . | . | . |
| 21-225_24E1_HC | . | . | . | . | . | . | . | T | . | . | . | . | E | . | . | . | . | . | . | . | . | . | . | . | . |
| 21-225_25A4_HC | . | . | . | . | . | . | . | . | . | . | . | . | . | . | . | . | . | . | . | . | . | . | . | . | . |
| 21-225_25A4.001_HC | . | . | . | . | . | . | . | . | . | . | . | . | . | . | . | . | . | . | . | . | . | . | . | . | . |
| 21-225_25A4.001.001_HC | . | . | . | . | . | . | . | . | . | . | . | . | . | . | . | . | . | . | . | . | . | . | . | . | . |
| 21-225_25A4.001.002_HC | . | . | . | . | . | . | . | . | . | . | . | . | . | . | . | . | . | . | . | . | . | . | . | . | . |
| 21-225_25A4.001.003_HC | . | . | . | . | . | . | . | . | . | . | . | . | . | . | . | . | . | . | . | . | . | . | . | . | . |
| 21-225_25A4.001.004_HC | . | . | . | . | . | . | . | . | . | . | . | . | . | . | . | . | . | . | . | . | . | . | . | . | . |
| 21-225_25A4.001.005_HC | . | . | . | . | . | . | . | . | . | . | . | . | . | . | . | . | . | . | . | . | . | . | . | . | . |
| 21-225_25A4.001.006_HC | . | . | . | . | . | . | . | . | . | . | . | . | . | . | . | . | . | . | . | . | . | . | . | . | . |
| 21-225_25A4.001.007_HC | . | . | . | . | . | . | . | . | . | . | . | . | . | . | . | . | . | . | . | . | . | . | . | . | . |
| 21-225_25A4.001.008_HC | . | . | . | . | . | . | . | . | . | . | . | . | . | . | . | . | . | . | . | . | . | . | . | . | . |
| 21-225_25A4.001.013_HC | . | . | . | . | . | . | . | . | . | . | . | . | . | . | . | . | . | . | . | . | . | . | . | . | . |
| 21-225_25A4.001.019_HC | . | . | . | . | . | . | . | . | . | . | . | . | . | . | . | . | . | . | . | . | . | . | . | . | . |
| 21-225_25A4.001.020_HC | . | . | . | . | . | . | . | . | . | . | . | . | . | . | . | . | . | . | . | . | . | . | . | . | . |
| 21-225_25A4.001.021_HC | . | . | . | . | . | . | . | . | . | . | . | . | . | . | . | . | . | . | . | . | . | . | . | . | . |
| 21-225_25A4.001.029_HC | . | . | . | . | . | . | . | . | . | . | . | . | . | . | . | . | . | . | . | . | . | . | . | . | . |
| 21-225_26C4_HC | . | . | . | . | . | . | . | . | . | . | . | . | . | . | . | . | . | . | . | . | . | . | . | . | . |
| 21-225_28B6_HC | . | . | . | . | . | . | . | . | . | . | . | . | . | . | . | . | . | . | . | . | . | . | . | . | . |
| 21-225_29E6_HC | . | . | . | . | . | . | . | . | . | . | . | . | . | . | . | . | . | . | . | . | . | . | . | . | . |
| 21-225_29H8_HC | . | . | . | . | . | . | . | . | . | . | . | . | . | . | . | . | . | . | . | . | . | . | . | . | . |
| 21-225_32A1_HC | . | . | . | . | . | . | . | . | . | . | . | . | . | . | . | . | . | . | . | . | . | . | . | . | . |
| 21-225_32A5_HC | . | . | . | . | . | . | S | . | . | . | . | . | . | . | . | . | . | . | . | . | . | . | . | . | . |
| 21-225_33D1_HC | . | . | . | . | . | . | . | . | . | . | . | . | . | . | . | . | . | . | . | . | . | . | . | . | . |
| 21-225_34D2_HC | . | . | . | . | . | . | . | . | . | . | . | . | . | . | . | . | . | . | . | . | . | . | . | . | . |
| 21-225_34H7_HC | . | . | . | . | . | . | . | . | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_6G6_HC | | | | | | | | L | | | | | | | | | | | | | | | | | |
| 21-225_70E7_HC | | | | | | | | Y | | | | | | | | | | | | | | | | | |
| 21-225_71F3_HC | | | | | | | | Y | | | | | | | | | | | | | | | | | |
| 21-225_71G3_HC | | | | | | | | Y | | | | | | | | | | | | | | | | | |
| 21-225_72C4_HC | | | | | | | S | H | | | | | | | | | | | | | | | | | |
| 21-225_74A6_HC | | | | | | | | | | | | | | | | | | | | | | | | | |
| 21-225_74A8_HC | S | | | | | | | Y | | | | | | | | | | | | | | | | | |
| 21-225_74B11_HC | | | | | | | | Y | | | | | | | | | | | | | | | | | |
| 21-225_74F6_HC | | | | | | | | H | | | | | | | | | | | | | | | | | |
| 21-225_74F9_HC | | | | | | | | | | | | | N | | | | | | | | | | | | |
| 21-225_74G4_HC | | | | | | | | | | | | | | | | | | | | | | | | | |
| 21-225_75A10_HC | | | | | | | | Y | | | | | | | | | | | | | | | | | |
| 21-225_75A6_HC | | | | | | | | Y | | | | | | | | | | | | | | | | | |
| 21-225_75C4_HC | | | | | | | | | | | | | | | | | | | | | | | | | |
| 21-225_75F9_HC | | | | | | | | Y | | | | | | | | | | | | | | | | | |
| 21-225_76E11_HC | | | | F | | | | Y | | | | | | | | | | | | | | | | | |
| 21-225_76G2_HC | | | | | | | | G | | | | | | | | | | | | | | | | | |
| 21-225_78E11_HC | | | | | | | | | | | | | N | | | | | | | | | | | | |
| 21-225_78E6_HC | S | | | | | | | Y | | | | | | | | | | | | | | | | | |
| 21-225_79E9_HC | | | | | | | | | | | | | | | | | | | | | | | | | |
| 21-225_79F12_HC | | | | | | | | | | | | | | | | | | | | | | | | | |
| 21-225_79G7_HC | S | | | | | | | Y | | | | | | | | | | | | | | | | | |
| 21-225_80B12_HC | | | | | | | | Y | | | | | | | | | | | | | | | | | |
| 21-225_85C11_HC | S | | | | | | | Y | | | | | | | | | | | | | | | | | |
| 21-225_87E10_HC | | | | | | | | Y | | | | | | | | | | | | | | | | | |
| 21-225_88A1_HC | | | | | | | | Y | | | | | | | | | | | | | | | | | |
| 21-225_88B4_HC | | | | | | | S | Y | | | | | | | | | | | | | | | | | |
| 21-225_88C10_HC | | | | | | | | | | | | | | | | | | | | | | | | | |
| 21-225_8B11_HC | | | | | | | | Y | | | | | | | | | | | | | | | | | |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_92E6_HC | . | . | . | . | . | . | . | H | . | . | . | . | . | . | . | . | . | . | . | . | . | . | . | . | . |
| 21-225_94D8_HC | . | . | . | . | . | . | . | Y | . | . | . | . | . | . | . | . | . | . | . | . | . | . | . | . | . |
| 21-225_96D10_HC | . | . | . | . | . | . | . | H | . | . | . | . | . | . | . | . | . | . | . | . | . | . | . | . | . |
| 21-225_96H5_HC | S | . | . | . | . | . | . | Y | . | . | . | . | . | . | . | . | . | . | . | . | . | . | . | . | . |

**FIGURE 57**

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | H_FR4 | | | | | | |
| CONSENSUS | - | - | - | - | - | - | - | - | - | Y | F | D | Y | W | G | Q | G | T | L | V | T | V | S | S |
| 21-225_11E5_HC | | | | | | | | | | V | | | | | | | | | | | | | | |
| 21-225_12F11_HC | | | | | | | | | | | | | | | | | | | | | | | | |
| 21-225_12F12_HC | | | | | | | | | | | | | F | | | | | | | | | | | |
| 21-225_146C9_HC | | | | | | | | | | S | | | | | | | | | | | | | | |
| 21-225_146D4_HC | | | | | | | | | | | | | | | | | | | | | | | | |
| 21-225_146F9_HC | | | | | | | | | | F | | | | | | | | | | | | | | |
| 21-225_147D5_HC | | | | | | | | | | | | | | | | | | | | | | | | |
| 21-225_147E4_HC | | | | | | | | | | F | | | | | | | | | | | | | | |
| 21-225_148F8_HC | | | | | | | | | | | | | | | | | | | | | | | | |
| 21-225_148G3_HC | | | | | | | | | | F | | | | | | | | | | | | | | |
| 21-225_149A2_HC | | | | | | | | | | | | | | | | | | | | | | | | |
| 21-225_149E3_HC | | | | | | | | | | W | | | | | | | | | | | | | | |
| 21-225_149H4_HC | | | | | | | | | | | | | | | | | | | | | | | | |
| 21-225_150B7_HC | | | | | | | | | | F | | | | | | | | | | | | | | |
| 21-225_150D2_HC | | | | | | | | | | | | | | | | | | | | | | | | |
| 21-225_152E12_HC | | | | | | | | | | | | | | | | | | | | | | | | |
| 21-225_152E3_HC | | | | | | | | | | F | | | | | | | | | | | | | | |
| 21-225_152H3_HC | | | | | | | | | | W | | | | | | | | | | | | | | |
| 21-225_153F11_HC | | | | | | | | | | F | | | N | | | | | | | | | | | |
| 21-225_154A11_HC | | | | | | | | | | | | | | | | | | | | | | | | |
| 21-225_154H6_HC | | | | | | | | | | I | | | | | | | | | | | | | | |
| 21-225_155B6_HC | | | | | | | | | | | | | | | | | | | | | | | | |
| 21-225_155E5_HC | | | | | | | | | | | | | | | | | | | | | | | | |
| 21-225_156H1_HC | | | | | | | | | | | | | | | | | | | | | | | | |
| 21-225_158B12_HC | | | | | | | | | | R | | | | | | | | | | | | | | |
| 21-225_160A4_HC | | | | | | | | | | I | | | | | | | | | | | | | | |
| 21-225_160A7_HC | | | | | | | | | | | | | | | | | | | | | | | | |
| 21-225_162D5_HC | | | | | | | | | | F | | | | | | | | | | | | | | |
| 21-225_162F6_HC | | | | | | | | | | R | | | | | | | | | | | | | | |

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_43D8_HC | . | . | . | . | . | . | . | H | . | . | . | . | T | . | . | . | . | . | . | . | . | . | . | . | . |
| 21-225_4A2_HC | . | . | . | . | . | . | . | . | . | . | . | . | . | . | . | . | . | . | . | . | . | . | . | . | . |
| 21-225_4A2.001_HC | . | . | . | . | . | . | . | . | . | . | . | . | . | . | . | . | . | . | . | . | . | . | . | . | . |
| 21-225_4A2.001.001_HC | . | . | . | . | . | . | . | . | . | . | . | . | . | . | . | . | . | . | . | . | . | . | . | . | . |
| 21-225_4A2.001.002_HC | . | . | . | . | . | . | . | . | . | . | . | . | . | . | . | . | . | . | . | . | . | . | . | . | . |
| 21-225_4A2.001.003_HC | . | . | . | . | . | . | . | . | . | . | . | . | . | . | . | . | . | . | . | . | . | . | . | . | . |
| 21-225_4A2.001.004_HC | . | . | . | . | . | . | . | . | . | . | . | . | . | . | . | . | . | . | . | . | . | . | . | . | . |
| 21-225_4A2.001.005_HC | . | . | . | . | . | . | . | . | . | . | . | . | . | . | . | . | . | . | . | . | . | . | . | . | . |
| 21-225_4A2.001.006_HC | . | . | . | . | . | . | . | . | . | . | . | . | . | . | . | . | . | . | . | . | . | . | . | . | . |
| 21-225_4A2.001.007_HC | . | . | . | . | . | . | . | . | . | . | . | . | . | . | . | . | . | . | . | . | . | . | . | . | . |
| 21-225_4A2.001.008_HC | . | . | . | . | . | . | . | . | . | . | . | . | . | . | . | . | . | . | . | . | . | . | . | . | . |
| 21-225_4A2.001.009_HC | . | . | . | . | . | . | . | . | . | . | . | . | . | . | . | . | . | . | . | . | . | . | . | . | . |
| 21-225_4A2.001.010_HC | . | . | . | . | . | . | . | . | . | . | . | . | . | . | . | . | . | . | . | . | . | . | . | . | . |
| 21-225_4A2.001.011_HC | . | . | . | . | . | . | . | . | . | . | . | . | . | . | . | . | . | . | . | . | . | . | . | . | . |
| 21-225_4A2.001.012_HC | . | . | . | . | . | . | . | . | . | . | . | . | . | . | . | . | . | . | . | . | . | . | . | . | . |
| 21-225_4A2.001.022_HC | . | . | . | . | . | . | . | . | . | . | . | . | . | . | . | . | . | . | . | . | . | . | . | . | . |
| 21-225_4A2.001.023_HC | . | . | . | . | . | . | . | . | . | . | . | . | . | . | . | . | . | . | . | . | . | . | . | . | . |
| 21-225_4A2.001.024_HC | . | . | . | . | . | . | . | . | . | . | . | . | . | . | . | . | . | . | . | . | . | . | . | . | . |
| 21-225_4A2.001.028_HC | . | . | . | . | . | . | . | . | . | . | . | . | . | . | . | . | . | . | . | . | . | . | . | . | . |
| 21-225_4A2.001.029_HC | . | . | . | . | . | . | . | . | . | . | . | . | . | . | . | . | . | . | . | . | . | . | . | . | . |
| 21-225_4A2.001.031_HC | . | . | . | . | . | . | . | . | . | . | . | . | . | . | . | . | . | . | . | . | . | . | . | . | . |
| 21-225_56A1_HC | . | . | . | . | . | . | . | Y | . | . | . | . | . | . | . | . | . | . | . | . | . | . | . | . | . |
| 21-225_58B9_HC | . | . | . | . | . | . | . | Y | . | . | . | . | . | . | . | . | . | . | . | . | . | . | . | . | . |
| 21-225_59E1_HC | . | . | . | . | . | . | . | F | . | . | . | . | . | . | . | . | . | . | . | . | . | . | . | . | . |
| 21-225_59F10_HC | . | . | . | . | N | . | . | V | . | . | . | . | . | . | . | . | . | . | . | . | . | . | . | . | . |
| 21-225_60F3_HC | . | . | . | . | . | . | . | Y | . | . | . | . | . | . | . | . | . | . | . | . | . | . | . | . | . |
| 21-225_61B5_HC | . | . | . | . | . | . | . | Y | . | . | . | . | . | . | . | . | . | . | . | . | . | . | . | . | . |
| 21-225_61E3_HC | . | . | . | . | . | . | . | Y | . | . | . | . | . | . | . | . | . | . | . | . | . | . | . | . | . |
| 21-225_63F4_HC | . | . | . | . | . | . | . | Y | . | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | H_FR4 | | | | | | |
| CONSENSUS | - | - | - | - | - | - | - | - | - | Y | F | D | Y | W | G | Q | G | T | L | V | T | V | S | S |
| 21-225_11E5_HC | | | | | | | | | | V | | | | | | | | | | | | | | |
| 21-225_12F11_HC | | | | | | | | | | | | | | | | | | | | | | | | |
| 21-225_12F12_HC | | | | | | | | | | | | | F | | | | | | | | | | | |
| 21-225_146C9_HC | | | | | | | | | | S | | | | | | | | | | | | | | |
| 21-225_146D4_HC | | | | | | | | | | | | | | | | | | | | | | | | |
| 21-225_146F9_HC | | | | | | | | | | F | | | | | | | | | | | | | | |
| 21-225_147D5_HC | | | | | | | | | | | | | | | | | | | | | | | | |
| 21-225_147E4_HC | | | | | | | | | | F | | | | | | | | | | | | | | |
| 21-225_148F8_HC | | | | | | | | | | | | | | | | | | | | | | | | |
| 21-225_148G3_HC | | | | | | | | | | F | | | | | | | | | | | | | | |
| 21-225_149A2_HC | | | | | | | | | | | | | | | | | | | | | | | | |
| 21-225_149E3_HC | | | | | | | | | | W | | | | | | | | | | | | | | |
| 21-225_149H4_HC | | | | | | | | | | | | | | | | | | | | | | | | |
| 21-225_150B7_HC | | | | | | | | | | F | | | | | | | | | | | | | | |
| 21-225_150D2_HC | | | | | | | | | | | | | | | | | | | | | | | | |
| 21-225_152E12_HC | | | | | | | | | | | | | | | | | | | | | | | | |
| 21-225_152E3_HC | | | | | | | | | | F | | | | | | | | | | | | | | |
| 21-225_152H3_HC | | | | | | | | | | W | | | | | | | | | | | | | | |
| 21-225_153F11_HC | | | | | | | | | | F | | | N | | | | | | | | | | | |
| 21-225_154A11_HC | | | | | | | | | | | | | | | | | | | | | | | | |
| 21-225_154H6_HC | | | | | | | | | | I | | | | | | | | | | | | | | |
| 21-225_155B6_HC | | | | | | | | | | | | | | | | | | | | | | | | |
| 21-225_155E5_HC | | | | | | | | | | | | | | | | | | | | | | | | |
| 21-225_156H1_HC | | | | | | | | | | | | | | | | | | | | | | | | |
| 21-225_158B12_HC | | | | | | | | | | R | | | | | | | | | | | | | | |
| 21-225_160A4_HC | | | | | | | | | | I | | | | | | | | | | | | | | |
| 21-225_160A7_HC | | | | | | | | | | | | | | | | | | | | | | | | |
| 21-225_162D5_HC | | | | | | | | | | F | | | | | | | | | | | | | | |
| 21-225_162F6_HC | | | | | | | | | | R | | | | | | | | | | | | | | |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | H_FR4 | | | | | | | |
| CONSENSUS | - | - | - | - | - | - | - | - | - | Y | F | D | Y | W | G | Q | G | T | L | V | T | V | S | S |
| 21-225_164A4_HC | | | | | | | | | | F | | | | | | | | | | | | | | |
| 21-225_164A7_HC | | | | | | | | | | L | | | | | | | | | | | | | | |
| 21-225_164B11_HC | | | | | | | | | | M | | | | | | | | | | | | | | |
| 21-225_165H2_HC | | | | | | | | | | M | | | | | | | | | | | | | | |
| 21-225_172E11_HC | | | | | | | | | | R | | | | | | | | | | | | | | |
| 21-225_175D10_HC | | | | | | | | | | | | | | | | | | | | | | | | |
| 21-225_177B12_HC | | | | | | | | | | | | | F | | | | | | | | | | | |
| 21-225_181E5_HC | | | | | | | | | | I | | | | | | | | | | | | | | |
| 21-225_190D7_HC | | | | | | | | | | F | | | | | | | | | | | | | | |
| 21-225_190H4_HC | | | | | | | | | | I | | | | | | | | | | | | | | |
| 21-225_191D8_HC | | | | | | | | | | | | | | | | | | | | | | | | |
| 21-225_191G3_HC | | | | | | | | | | I | | | | | | | | | | | | | | |
| 21-225_193G4_HC | | | | | | | | | | F | | | | | | | | | | | | | | |
| 21-225_197C6_HC | | | | | | | | | | | | | | | | | | | | | | | | |
| 21-225_197H4_HC | | | | | | | | | | | | | | | | | | | | | | | | |
| 21-225_201F5_HC | | | | | | | | | | | | | | | | | | | | | | | | |
| 21-225_224C10_HC | | | | | | | | | | K | | | | | | | | | | | | | | |
| 21-225_224D8_HC | | | | | | | | | | | | | | | | | | | | | I | | | |
| 21-225_224F11_HC | | | | | | | | | | K | | | | | | | | | | | | | | |
| 21-225_225B9_HC | | | | | | | | | | L | | | | | | | | | | | | | | |
| 21-225_225F11_HC | | | | | | | | | | R | | | | | | | | | | | | | | |
| 21-225_225F12_HC | | | | | | | | | | K | | | | | | | | | | | | | | |
| 21-225_225F5_HC | | | | | | | | | | R | | | | | | | | | | | | | | |
| 21-225_225H12_HC | | | | | | | | | | K | | | | | | | | | | | | | | |
| 21-225_226A12_HC | | | | | | | | | | | | | | | | | | | | | | | | |
| 21-225_226D11_HC | | | | | | | | | | | | | | | | | | | | | | | | |
| 21-225_226D6_HC | | | | | | | | | | K | | | | | | | | | | | | | | |
| 21-225_226F6_HC | | | | | | | | | | R | | | | | | | | | | | | | | |
| 21-225_227C7_HC | | | | | | | | | | R | | | | | | | | | | | | | | |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | - | Y | F | D | Y | W | G | Q | G | T | L | V | T | V | S | S |
| 21-225_227D11_HC | | | | | | | | | | | | | | | | | | | | | | | | |
| 21-225_227E6_HC | | | | | | | | | | K | | | | | | | | | | | | | | |
| 21-225_22D2_HC | | | | | | | | | | | | | | | | | | | | | | | | |
| 21-225_23A12_HC | | | | | | | | | | | | | | | | | | | | | | | | |
| 21-225_24E1_HC | | | | | | | | | | V | | | | | | | | | | | | | | |
| 21-225_25A4_HC | | | | | | | | | | | | | | | | | | | | | | | | |
| 21-225_25A4.001_HC | | | | | | | | | | | | | | | | | | | | | | | | |
| 21-225_25A4.001.001_HC | | | | | | | | | | | | | | | | | | | | | | | | |
| 21-225_25A4.001.002_HC | | | | | | | | | | | | | | | | | | | | | | | | |
| 21-225_25A4.001.003_HC | | | | | | | | | | | | | | | | | | | | | | | | |
| 21-225_25A4.001.004_HC | | | | | | | | | | | | | | | | | | | | | | | | |
| 21-225_25A4.001.005_HC | | | | | | | | | | | | | | | | | | | | | | | | |
| 21-225_25A4.001.006_HC | | | | | | | | | | | | | | | | | | | | | | | | |
| 21-225_25A4.001.007_HC | | | | | | | | | | | | | | | | | | | | | | | | |
| 21-225_25A4.001.008_HC | | | | | | | | | | | | | | | | | | | | | | | | |
| 21-225_25A4.001.013_HC | | | | | | | | | | | | | | | | | | | | | | | | |
| 21-225_25A4.001.019_HC | | | | | | | | | | | | | | | | | | | | | | | | |
| 21-225_25A4.001.020_HC | | | | | | | | | | | | | | | | | | | | | | | | |
| 21-225_25A4.001.021_HC | | | | | | | | | | | | | | | | | | | | | | | | |
| 21-225_25A4.001.029_HC | | | | | | | | | | | | | | | | | | | | | | | | |
| 21-225_26C4_HC | | | | | | | | | | | | | | | | | | | | | | | | |
| 21-225_28B6_HC | | | | | | | | | | | | | | | | | | | | | | | | |
| 21-225_29E6_HC | | | | | | | | | | | | | | | | | | | | | | | | |
| 21-225_29H8_HC | | | | | | | | | | | | | | | | | | | | | | | | |
| 21-225_32A1_HC | | | | | | | | | | F | | | | | | | | | | | | | | |
| 21-225_32A5_HC | | | | | | | | | | F | | | | | | | | | | | | | | |
| 21-225_33D1_HC | | | | | | | | | | F | | | | | | | | | | | | | | |
| 21-225_34D2_HC | | | | | | | | | | F | | | | | | | | | | | | | | |
| 21-225_34H7_HC | | | | | | | | | | F | | | | | | | | | | | | | | |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | - | Y | F | D | Y | W | G | Q | G | T | L | V | T | V | S | S |
| 21-225_43D8_HC | | | | | | | | | | L | | | | | | | | | | | | | | A |
| 21-225_4A2_HC | | | | | | | | | | | | | | | | | | | | | | | | |
| 21-225_4A2.001_HC | | | | | | | | | | | | | | | | | | | | | | | | |
| 21-225_4A2.001.001_HC | | | | | | | | | | | | | | | | | | | | | | | | |
| 21-225_4A2.001.002_HC | | | | | | | | | | | | | | | | | | | | | | | | |
| 21-225_4A2.001.003_HC | | | | | | | | | | | | | | | | | | | | | | | | |
| 21-225_4A2.001.004_HC | | | | | | | | | | | | | | | | | | | | | | | | |
| 21-225_4A2.001.005_HC | | | | | | | | | | | | | | | | | | | | | | | | |
| 21-225_4A2.001.006_HC | | | | | | | | | | | | | | | | | | | | | | | | |
| 21-225_4A2.001.007_HC | | | | | | | | | | | | | | | | | | | | | | | | |
| 21-225_4A2.001.008_HC | | | | | | | | | | | | | | | | | | | | | | | | |
| 21-225_4A2.001.009_HC | | | | | | | | | | | | | | | | | | | | | | | | |
| 21-225_4A2.001.010_HC | | | | | | | | | | | | | | | | | | | | | | | | |
| 21-225_4A2.001.011_HC | | | | | | | | | | | | | | | | | | | | | | | | |
| 21-225_4A2.001.012_HC | | | | | | | | | | | | | | | | | | | | | | | | |
| 21-225_4A2.001.022_HC | | | | | | | | | | | | | | | | | | | | | | | | |
| 21-225_4A2.001.023_HC | | | | | | | | | | | | | | | | | | | | | | | | |
| 21-225_4A2.001.024_HC | | | | | | | | | | | | | | | | | | | | | | | | |
| 21-225_4A2.001.028_HC | | | | | | | | | | | | | | | | | | | | | | | | |
| 21-225_4A2.001.029_HC | | | | | | | | | | | | | | | | | | | | | | | | |
| 21-225_4A2.001.031_HC | | | | | | | | | | | | | | | | | | | | | | | | |
| 21-225_56A1_HC | | | | | | | | | | V | | | | | | | | | | | | | | |
| 21-225_58B9_HC | | | | | | | | | | | | | | | | | | | | | | | | |
| 21-225_59E1_HC | | | | | | | | | | F | | | | | | | | | | | | | | |
| 21-225_59F10_HC | | | | | | | | | | | | | | | | | | | | | | | | |
| 21-225_60F3_HC | | | | | | | | | | F | | | | | | | | | | | | | | |
| 21-225_61B5_HC | | | | | | | | | | | | | | | | | | | | | | | | |
| 21-225_61E3_HC | | | | | | | | | | R | | | | | | | | | | | | | | |
| 21-225_63F4_HC | | | | | | | | | | V | | | | | | | | | | | | | | |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | H_FR4 | | | | | | |
| CONSENSUS | - | - | - | - | - | - | - | - | - | Y | F | D | Y | W | G | Q | G | T | L | V | T | V | S | S |
| 21-225_6G6_HC | | | | | | | | | | | | | | | | | | | | | | | | |
| 21-225_70E7_HC | | | | | | | | | | V | | | | | | | | | | | | | | |
| 21-225_71F3_HC | | | | | | | | | | V | | | S | | | | | | | | | | | |
| 21-225_71G3_HC | | | | | | | | | | | | | | | | | | | | | | | | |
| 21-225_72C4_HC | | | | | | | | | | L | | | | | | | | | | | | | | |
| 21-225_74A6_HC | | | | | | | | | | | | | | | | | | | | | | | | |
| 21-225_74A8_HC | | | | | | | | | | K | | | | | S | | | | | | | | | |
| 21-225_74B11_HC | | | | | | | | | | | | | | | | | | | | | | | | |
| 21-225_74F6_HC | | | | | | | | | | F | | | | | | | | | | | | | | |
| 21-225_74F9_HC | | | | | | | | | | F | | | | | | | | | | | | | | |
| 21-225_74G4_HC | | | | | | | | | | H | | | | | | | | | | | | | | |
| 21-225_75A10_HC | | | | | | | | | | | | | | | | | | | | | | | | |
| 21-225_75A6_HC | | | | | | | | | | I | | | | | | | | | | | | | | |
| 21-225_75C4_HC | | | | | | | | | | | | | | | | | | | | | | | | |
| 21-225_75F9_HC | | | | | | | | | | I | | | | | | | | | | | | | | |
| 21-225_76E11_HC | | | | | | | | | | | | | | | | | | | | | | | | |
| 21-225_76G2_HC | | | | | | | | | | M | | | | | | | | | | | | | | |
| 21-225_78E11_HC | | | | | | | | | | F | | | | | | | | | | | | | | |
| 21-225_78E6_HC | | | | | | | | | | K | | | | | S | | | | | | | | | |
| 21-225_79E9_HC | | | | | | | | | | | | | | | | | | | | | | | | |
| 21-225_79F12_HC | | | | | | | | | | F | | | | | | | | | | | | L | | |
| 21-225_79G7_HC | | | | | | | | | | K | | | | | S | | | | | | | | | |
| 21-225_80B12_HC | | | | | | | | | | I | | | | | | | | | | | | | | |
| 21-225_85C11_HC | | | | | | | | | | K | | | | | S | | | | | | | | | |
| 21-225_87E10_HC | | | | | | | | | | F | | | | | | | | | | | | | | |
| 21-225_88A1_HC | | | | | | | | | | | | | | | | | | | | | | | | |
| 21-225_88B4_HC | | | | | | | | | | | | | | | | | | | | | | | | |
| 21-225_88C10_HC | | | | | | | | | | | | | S | | | | | | | | | | | L |
| 21-225_8B11_HC | | | | | | | | | | F | | | | | | | | | | | | | | |

FIGURE 57

Table 50.      Consensus 16- VH3|3-33/D6|6-6|RF1/JH6 (SEQ ID NO: 50267):

QVQLVES-GGGVVQPGRSLRLSCAASG-FTFSS-----YVMHWVRQAPGKGLEWVAVIWYD---
GSNKYYADSVKGRFTISRDNSKNTLYLQMNSLRAEDTAVYYCARERYSSGW---------------YDYGMDVWGQGTTVTVSS wherein:

S at position 33 can be substituted with D, N or I

Y at position 39 can be substituted with C, F, D or S

V at position 40 can be substituted with G, I or L

M at position 41 can be substituted with I or L

H at position 42 can be substituted with D

V at position 57 can be substituted with L or A

W at position 59 can be substituted with F

G at position 65 can be substituted with A

S at position 66 can be substituted with R or N

N at position 67 can be substituted with Y, G or S

Y at position 69 can be substituted with H

Y at position 70 can be substituted with H or N

A at position 71 can be substituted with V, E, G or T

D at position 72 can be substituted with E or G

S at position 73 can be substituted with A

V at position 74 can be substituted with M

E at position 109 can be substituted with R or V

FIGURE 57

R at position 110 can be substituted with Y, K, V, E, P, D, L, F, M, N, Q or T

Y at position 111 can be substituted with S, T or V

S at position 112 can be substituted with R, Y, P, T or G

S at position 113 can be substituted with C or Y

G at position 114 can be substituted with W, S or N

W at position 115 can be substituted with null (-), L, Y, G, F or S

null (-) at position 116 can be substituted with Y

null (-) at position 117 can be substituted with A, G or T

null (-) at position 118 can be substituted with C

null (-) at position 129 can be substituted with P

null (-) at position 130 can be substituted with Y or L

null (-) at position 131 can be substituted with Y or D

Y at position 132 can be substituted with null (-), F or H

D at position 133 can be substituted with S, G, T, V, Y, A, F or M

Y at position 134 can be substituted with G or F

M at position 136 can be substituted with L

D at position 137 can be substituted with G

Xaa Xaa Xaa Xaa Xaa (SEQ ID NO: 50004)

Xaa Ile Xaa Tyr Asp Xaa Xaa Xaa Lys Xaa Xaa Xaa Xaa Xaa Xaa Lys Gly (SEQ ID NO: 50005)

**FIGURE 57**

Xaa Xaa Xaa Xaa Xaa Xaa Xaa Xaa Xaa Xaa Xaa Xaa Xaa Xaa Xaa Xaa Gly Xaa Xaa Val (SEQ ID NO: 50006)

Ser Tyr Val Met His (SEQ ID NO: 50471)

Val Ile Trp Tyr Asp Gly Ser Asn Lys Tyr Tyr Ala Asp Ser Val Lys Gly (SEQ ID NO: 50472)

Glu Arg Tyr Ser Ser Gly Trp Tyr Asp Tyr Gly Met Asp Val (SEQ ID NO: 50473)

EP 4 435 105 A2

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_10E9_HC | . | . | . | . | . | . | . | . | . | . | . | . | . | . | . | . | . | . | . | . | . | . | . | . | . |
| 21-225_147A8_HC | . | . | . | . | . | . | . | . | . | . | . | . | . | . | . | . | . | . | . | . | . | . | . | . | . |
| 21-225_147B2_HC | . | . | . | . | . | . | . | . | . | . | . | . | . | . | . | . | . | . | . | . | . | . | . | . | . |
| 21-225_147H5_HC | . | . | . | . | . | . | . | . | . | . | . | . | A | . | . | . | . | . | . | . | . | . | . | . | . |
| 21-225_148C9_HC | . | . | . | . | . | . | . | . | . | . | . | . | A | . | . | . | . | . | . | . | . | . | . | . | . |
| 21-225_148F5_HC | . | . | . | . | . | . | . | . | . | . | . | . | . | . | . | . | . | . | . | . | . | . | . | . | . |
| 21-225_148H10_HC | . | . | . | . | . | . | . | . | . | . | . | . | . | . | . | . | . | . | . | . | . | . | . | . | . |
| 21-225_149D10_HC | . | . | . | . | . | . | . | . | . | . | . | . | A | . | . | . | . | . | . | . | . | . | . | . | . |
| 21-225_14E10_HC | . | . | . | . | . | . | . | . | . | . | . | . | . | . | . | . | . | . | . | . | . | . | . | . | . |
| 21-225_150D11_HC | . | . | . | . | . | . | . | . | . | . | . | . | . | . | . | . | . | . | . | . | . | . | . | . | . |
| 21-225_150E2_HC | . | . | . | . | . | . | . | . | . | . | . | . | . | . | . | . | . | . | . | . | . | . | . | . | . |
| 21-225_152F7_HC | . | . | . | . | . | . | . | . | . | . | . | . | . | . | . | . | . | . | . | . | . | . | . | . | . |
| 21-225_153G9_HC | . | . | . | . | . | . | . | . | . | . | . | . | . | . | . | . | . | . | . | . | . | . | . | . | . |
| 21-225_154F4_HC | . | . | . | . | . | . | . | . | . | . | . | . | . | . | . | . | . | . | . | . | . | . | . | . | . |
| 21-225_159D8_HC | . | . | . | . | . | . | . | . | . | . | . | . | . | . | . | . | . | . | . | . | . | . | . | . | . |
| 21-225_15C11_HC | . | . | . | . | . | . | . | . | . | . | . | . | . | . | . | . | . | . | . | . | . | . | . | . | . |
| 21-225_169D11_HC | . | . | . | . | . | . | . | . | . | . | . | . | . | . | . | . | . | . | . | . | . | . | . | . | . |
| 21-225_170F3_HC | . | . | . | . | . | . | . | . | . | . | . | . | . | . | . | . | . | . | . | . | . | . | . | . | . |
| 21-225_173C11_HC | . | . | . | . | . | . | . | . | . | . | . | . | . | . | . | . | . | . | . | . | . | . | . | . | . |
| 21-225_174G10_HC | . | . | . | . | . | . | . | . | . | . | . | . | . | . | . | . | . | . | . | . | . | . | . | . | . |
| 21-225_176H12_HC | . | . | . | . | . | . | . | . | . | . | . | . | . | . | . | . | . | . | . | . | . | . | . | . | . |
| 21-225_177B4_HC | . | . | . | . | . | . | . | . | . | . | . | . | . | . | . | . | . | . | . | . | . | . | . | . | . |
| 21-225_179C2_HC | . | . | . | . | . | . | . | . | . | . | . | . | . | . | . | . | . | . | . | . | . | . | . | . | . |
| 21-225_17H3_HC | . | . | . | . | . | . | . | . | . | . | . | . | . | . | . | . | . | . | . | . | . | . | . | . | . |
| 21-225_183F4_HC | . | . | . | . | . | . | . | . | . | . | . | . | . | . | . | . | . | . | . | . | . | . | . | . | . |
| 21-225_190D6_HC | . | . | . | . | . | . | . | . | . | . | . | . | . | . | . | . | . | . | . | . | . | . | . | . | . |
| 21-225_192A2_HC | . | . | . | . | . | . | . | . | . | . | . | . | . | . | . | . | . | . | . | . | . | . | . | . | . |
| 21-225_192F6_HC | . | . | . | . | . | . | . | . | . | . | . | . | . | . | . | . | . | . | . | . | . | . | . | . | . |
| 21-225_193F8_HC | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_194F10_HC | . | . | . | . | . | . | . | . | . | . | . | . | . | . | . | . | . | . | . | . | . | . | . | . | . |
| 21-225_201C5_HC | . | . | . | . | . | . | . | . | . | . | . | . | . | . | . | . | . | . | . | . | . | . | . | . | . |
| 21-225_203F7_HC | . | . | . | . | . | . | . | . | . | . | . | . | . | . | . | . | . | . | . | . | . | . | . | . | . |
| 21-225_204H3_HC | . | . | . | . | . | . | . | . | . | . | . | . | G | . | . | . | . | . | . | . | . | . | . | T | . |
| 21-225_208B2_HC | . | . | . | . | . | . | . | . | . | . | . | . | . | . | . | . | . | . | . | . | . | . | . | . | . |
| 21-225_208F1_HC | . | . | . | . | . | . | . | . | . | . | . | . | . | . | . | . | . | . | . | . | . | . | . | . | . |
| 21-225_20B2_HC | . | . | . | . | . | . | . | . | . | . | . | . | . | . | . | . | . | . | . | . | . | . | . | . | . |
| 21-225_210G5_HC | . | . | . | . | . | . | . | . | . | . | . | . | . | . | . | . | . | . | . | . | . | . | . | . | . |
| 21-225_210G6_HC | . | . | . | . | . | . | . | . | . | . | . | . | . | . | . | . | . | . | . | . | . | . | . | . | . |
| 21-225_212B11_HC | . | . | . | . | . | . | . | . | . | . | . | . | . | . | . | . | . | . | . | . | . | . | . | . | . |
| 21-225_214G10_HC | . | . | . | . | . | . | . | . | . | . | . | . | . | . | . | . | . | . | . | . | . | . | . | . | . |
| 21-225_214G12_HC | . | . | . | . | . | . | . | . | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . | . |
| 21-225_215E11_HC | . | . | . | . | . | . | . | . | . | . | . | . | . | . | . | . | . | . | . | . | . | . | . | . | . |
| 21-225_215E3_HC | . | . | . | . | . | . | . | . | . | . | . | . | . | . | . | . | . | . | . | . | . | . | . | . | . |
| 21-225_216E8_HC | . | . | . | . | . | . | . | . | . | . | . | . | . | . | . | . | . | . | . | . | . | . | . | . | . |
| 21-225_216H12_HC | . | . | . | . | . | . | . | . | . | . | . | . | . | . | . | . | . | . | . | . | . | . | . | . | . |
| 21-225_217E5_HC | . | . | . | . | . | . | . | . | . | . | . | . | . | . | . | . | . | . | . | . | . | . | . | . | . |
| 21-225_217G10_HC | . | . | . | . | . | . | . | . | . | . | . | . | . | . | . | . | . | . | . | . | . | . | . | . | . |
| 21-225_217H12_HC | . | . | . | . | . | . | . | . | . | . | . | . | . | . | . | . | . | . | . | . | . | . | . | . | . |
| 21-225_218C4_HC | . | . | . | . | . | . | . | . | . | . | . | . | . | . | . | . | . | . | . | . | . | . | . | . | . |
| 21-225_220F8_HC | . | . | . | . | . | . | . | . | . | . | . | . | . | . | . | . | . | . | . | . | . | . | . | . | . |
| 21-225_224E7_HC | . | . | . | . | . | . | . | . | . | . | . | . | . | . | . | . | . | . | . | S | . | . | . | . | . |
| 21-225_224F1_HC | . | . | . | . | . | . | . | . | . | . | . | . | . | . | . | . | . | . | . | . | . | . | . | . | . |
| 21-225_224F3_HC | . | . | . | . | . | . | . | . | . | . | . | . | . | . | . | . | . | . | . | . | . | . | . | . | . |
| 21-225_225A1_HC | . | . | . | . | . | . | . | . | . | . | . | . | . | . | . | . | . | . | . | . | . | . | . | . | . |
| 21-225_226C6_HC | . | . | . | . | . | . | . | . | . | . | . | . | . | . | . | . | . | . | . | . | . | . | . | . | . |
| 21-225_226H11_HC | . | . | . | . | . | . | . | . | . | . | . | . | . | . | . | . | . | . | . | . | . | . | . | . | . |
| 21-225_22F4_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_23A10_HC | . | . | . | . | . | . | . | . | . | . | . | . | . | . | . | . | . | . | . | . | . | . | . | . | . |
| 21-225_24B6_HC | . | . | . | . | . | . | . | . | . | . | . | . | . | . | . | . | . | . | P | . | . | . | . | . | . |
| 21-225_25A2_HC | . | . | . | . | . | . | . | . | . | . | . | . | . | . | . | . | . | . | . | . | . | . | . | . | . |
| 21-225_25D12_HC | . | . | . | . | . | . | . | . | . | . | . | . | . | . | . | . | . | . | . | . | . | . | . | . | . |
| 21-225_26A11_HC | . | . | . | . | . | . | . | . | . | . | . | . | . | . | . | . | . | . | . | . | . | . | . | . | . |
| 21-225_26G7_HC | . | . | . | . | . | . | . | . | . | . | . | . | . | . | . | . | . | . | . | . | . | . | . | . | . |
| 21-225_27E7_HC | . | . | . | . | . | . | . | . | . | . | . | . | . | . | . | . | . | . | . | . | . | . | . | . | . |
| 21-225_28H2_HC | . | . | . | . | . | . | . | . | . | . | . | . | . | . | . | . | . | . | . | . | . | . | . | . | . |
| 21-225_29E2_HC | . | . | . | . | . | . | . | . | . | . | . | . | . | . | . | . | . | . | . | . | . | . | . | . | . |
| 21-225_29H4_HC | . | . | . | . | . | . | . | . | . | . | . | . | . | . | . | . | . | . | . | . | . | . | . | . | . |
| 21-225_2G4_HC | . | . | . | . | . | . | . | . | . | . | . | . | . | . | . | . | . | . | . | . | . | . | . | . | . |
| 21-225_32H2_HC | . | . | . | . | . | . | . | . | . | . | . | . | . | . | . | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | . | . | . | . | . | . | . | . | . | . | . | . | . | . | . | . | . | . | . | . | . | . | . |
| 21-225_44B10_HC | . | . | . | . | . | . | . | . | . | . | . | . | . | . | . | . | . | . | . | . | . | . | . | V | . |
| 21-225_44B3_HC | . | . | . | . | . | . | . | . | . | . | . | . | . | . | . | . | . | . | . | . | . | . | . | T | . |
| 21-225_44C10_HC | . | . | . | . | . | . | . | . | . | . | . | . | . | . | . | . | . | . | . | . | . | . | . | . | . |
| 21-225_44D3_HC | . | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . | . |
| 21-225_44F9_HC | . | . | . | . | . | . | . | . | . | . | . | . | . | . | . | . | . | . | . | . | . | . | . | . | . |
| 21-225_45B4_HC | . | . | . | . | . | . | . | . | . | . | . | . | . | . | . | . | . | . | . | . | . | . | . | . | . |
| 21-225_45B8_HC | . | . | . | . | . | . | . | . | . | . | . | . | . | . | . | . | . | . | . | . | . | . | . | . | . |
| 21-225_46B9_HC | . | . | . | . | . | . | . | . | . | . | . | . | . | . | . | . | . | . | . | . | . | . | . | S | . |
| 21-225_46C3_HC | . | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . | . |
| 21-225_47C1_HC | . | . | . | . | . | . | . | . | . | . | . | . | . | . | . | . | . | . | . | . | . | . | . | . | . |
| 21-225_47E7_HC | . | . | . | . | . | . | . | . | . | . | . | . | . | . | . | . | . | . | . | . | . | . | . | . | . |
| 21-225_48F2_HC | . | . | . | . | . | . | . | . | . | . | . | . | . | . | . | . | . | . | . | . | . | . | . | . | . |
| 21-225_48H4_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_49C1_HC | . | | | | | | | | | | | | | | | | | | | | | | | T | . |
| 21-225_49E7_HC | . | | | | | | | | | | | | | | | | | | | | | | | | |
| 21-225_49F9_HC | . | | | | | | | | | | | | | | | | | | | | | | | | |
| 21-225_49G3_HC | . | | | | | | | | | | | | | | | | | | | | | | | | |
| 21-225_4C12_HC | . | | | | | | | | | | | | | | | | | | | | | | | | |
| 21-225_4C5_HC | . | | | | | | | | | | | | | | | | | | | | | | | | |
| 21-225_50H4_HC | . | | | | | | | | | | | | | | | | | | | | | | | | |
| 21-225_51E1_HC | . | | | | | | | | | | | | | | | | | | | | | | | | |
| 21-225_52D10_HC | . | | | | | | | | | | | | | | | | | | | | | | | | |
| 21-225_52G8_HC | . | | | | | | | | | | | | | | | | | | | | | | | | |
| 21-225_54D4_HC | . | | | | | | | | | | | | | | | | | | | | | | | | |
| 21-225_55C2_HC | . | | | | | | | | | | | | | | | | | | | | | | | | |
| 21-225_55F9_HC | . | | | | | | | | | | | | | | | | | | | | | | | | |
| 21-225_55H1_HC | . | | | | | | | | | | | | | | | | | | | | | | | | |
| 21-225_58H11_HC | . | | | | | | | | | | | | | | | | | | | | | | | | |
| 21-225_59A12_HC | . | | | | | | | | | | | | | | | | | | | | | | | | |
| 21-225_5E5_HC | . | | | | | | | | | | | | | | | | | | | | | | | | |
| 21-225_5E5.003_HC | . | | | | | | | | | | | | | | | | | | | | | | | | |
| 21-225_5E5.004_HC | . | | | | | | | | | | | | | | | | | | | | | | | | |
| 21-225_5E5.005_HC | . | | | | | | | | | | | | | | | | | | | | | | | | |
| 21-225_5E5.006_HC | . | | | | | | | | | | | | | | | | | | | | | | | | |
| 21-225_5E5.011_HC | . | | | | | | | | | | | | | | | | | | | | | | | | |
| 21-225_5E5.012_HC | . | | | | | | | | | | | | | | | | | | | | | | | | |
| 21-225_5E5.015_HC | . | | | | | | | | | | | | | | | | | | | | | | | | |
| 21-225_5E5.016_HC | . | | | | | | | | | | | | | | | | | | | | | | | | |
| 21-225_5E5.017_HC | . | | | | | | | | | | | | | | | | | | | | | | | | |
| 21-225_5E5.018_HC | . | | | | | | | | | | | | | | | | | | | | | | | | |
| 21-225_5E5.019_HC | . | | | | | | | | | | | | | | | | | | | | | | | | |
| 21-225_5E5.023_HC | . | | | | | | | | | | | | | | | | | | | | | | | | |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_5E5.024_HC | . | . | . | . | . | . | . | . | . | . | . | . | . | . | . | . | . | . | . | . | . | . | . | . | . |
| 21-225_60C12_HC | . | . | . | . | . | . | . | . | . | . | . | . | . | . | . | . | . | . | . | . | . | . | . | . | . |
| 21-225_60G2_HC | . | . | . | . | . | . | . | . | . | . | . | . | . | . | . | . | . | . | . | . | . | . | . | . | . |
| 21-225_61B3_HC | . | . | . | . | . | . | . | . | . | . | . | . | . | . | . | . | . | . | . | . | . | . | . | . | . |
| 21-225_61F2_HC | . | . | . | . | . | . | . | . | . | . | . | . | . | . | . | . | . | . | . | . | . | . | . | . | . |
| 21-225_64A4_HC | . | . | . | . | . | . | . | . | . | . | . | . | . | . | . | . | . | . | . | . | . | . | . | . | . |
| 21-225_64C8_HC | . | . | . | . | . | . | . | . | . | . | . | . | . | . | . | . | . | . | . | . | . | . | . | . | . |
| 21-225_66D11_HC | . | . | . | . | . | . | . | . | . | . | . | . | . | . | . | . | . | . | . | . | . | . | . | G | . |
| 21-225_73C9_HC | . | . | . | . | . | . | . | . | . | . | . | . | . | . | . | . | . | . | . | . | . | . | . | . | . |
| 21-225_7C9_HC | . | . | . | . | . | . | . | . | . | . | . | . | . | . | . | . | . | . | . | . | . | . | . | . | . |
| 21-225_7G4_HC | . | . | . | . | . | . | . | . | . | . | . | . | . | . | . | . | . | . | . | . | . | . | . | . | . |
| 21-225_94F3_HC | . | . | . | . | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | H_CDR1 | | | | | | | | | | | | | H_FR2 | |
| CONSENSUS | S | G | - | F | T | F | S | S | - | - | - | - | - | Y | V | M | H | W | V | R | Q | A | P | G | K |
| 21-225_10E9_HC | . | . | . | . | . | . | . | N | . | . | . | . | . | . | . | I | . | . | . | . | . | . | . | . | . |
| 21-225_147A8_HC | . | . | . | . | . | . | . | . | . | . | . | . | . | . | G | . | . | . | . | . | . | . | . | . | . |
| 21-225_147B2_HC | . | . | . | . | . | . | . | . | . | . | . | . | . | . | G | . | . | . | . | . | . | . | . | . | . |
| 21-225_147H5_HC | . | . | . | . | . | . | . | D | . | . | . | . | . | . | G | . | . | . | . | . | . | . | . | . | . |
| 21-225_148C9_HC | . | . | . | . | . | . | . | D | . | . | . | . | . | . | G | . | . | . | . | . | . | . | . | . | . |
| 21-225_148F5_HC | . | . | . | . | . | . | . | . | . | . | . | . | . | . | G | . | . | . | . | . | . | . | . | . | . |
| 21-225_148H10_HC | . | . | . | . | . | . | . | . | . | . | . | . | . | . | G | . | . | . | . | . | . | . | . | . | . |
| 21-225_149D10_HC | . | . | . | . | . | . | . | D | . | . | . | . | . | . | G | . | . | . | . | . | . | . | . | . | . |
| 21-225_14E10_HC | . | . | . | . | . | . | . | D | . | . | . | . | . | . | G | . | . | . | . | . | . | . | . | . | . |
| 21-225_150D11_HC | . | . | . | . | . | . | . | . | . | . | . | . | . | . | G | . | . | . | . | . | . | . | . | . | . |
| 21-225_150E2_HC | . | . | . | . | . | . | . | . | . | . | . | . | . | . | . | . | . | . | . | . | . | . | . | . | . |
| 21-225_152F7_HC | . | . | . | . | . | . | . | . | . | . | . | . | . | . | I | . | . | . | . | . | . | . | . | . | . |
| 21-225_153G9_HC | . | . | . | . | . | . | . | . | . | . | . | . | . | . | G | . | . | . | . | . | . | . | . | . | . |
| 21-225_154F4_HC | . | . | . | . | . | . | . | . | . | . | . | . | . | . | G | . | . | . | . | . | . | . | . | . | . |
| 21-225_159D8_HC | . | . | . | . | . | . | . | N | . | . | . | . | . | . | . | . | . | C | . | . | . | . | . | . | . |
| 21-225_15C11_HC | . | . | . | . | . | . | . | D | . | . | . | . | . | . | . | . | . | . | . | . | . | . | . | . | . |
| 21-225_169D11_HC | . | . | . | . | . | . | . | D | . | . | . | . | . | . | . | . | . | . | . | . | . | . | . | . | . |
| 21-225_170F3_HC | . | . | . | . | S | . | . | D | . | . | . | . | . | . | . | . | . | . | . | . | . | . | . | . | . |
| 21-225_173C11_HC | . | . | . | . | . | . | . | . | . | . | . | . | . | . | G | I | . | . | . | . | . | . | . | . | . |
| 21-225_174G10_HC | . | . | . | . | . | . | . | D | . | . | . | . | . | . | . | . | . | . | . | . | . | . | . | . | . |
| 21-225_176H12_HC | . | . | . | . | . | . | . | D | . | . | . | . | . | . | . | . | . | . | . | . | . | . | . | . | . |
| 21-225_177B4_HC | . | . | . | . | . | . | . | D | . | . | . | . | . | . | . | . | . | . | . | . | . | . | . | . | . |
| 21-225_179C2_HC | . | . | . | . | . | . | . | . | . | . | . | . | . | . | G | . | . | . | . | . | . | . | . | . | . |
| 21-225_17H3_HC | . | . | . | . | . | . | . | N | . | . | . | . | . | C | . | . | . | . | . | . | . | . | . | . | . |
| 21-225_183F4_HC | . | . | . | . | . | . | . | . | . | . | . | . | . | . | G | . | . | . | . | . | . | . | . | . | . |
| 21-225_190D6_HC | . | . | . | . | . | . | . | . | . | . | . | . | . | F | I | . | . | . | . | . | . | . | . | . | R |
| 21-225_192A2_HC | . | . | . | . | . | . | . | . | . | . | . | . | . | . | G | . | . | . | . | . | . | . | . | . | . |
| 21-225_192F6_HC | . | . | . | . | . | . | . | . | . | . | . | . | . | F | I | . | . | . | . | . | . | . | . | . | R |
| 21-225_193F8_HC | . | . | . | . | . | . | . | I | . | . | . | . | . | . | G | . | D | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | S | - | - | - | - | - | Y | V | M | H | W | V | R | Q | A | P | G | K |
| 21-225_194F10_HC | . | . | . | . | . | . | . | . | . | . | . | . | . | F | I | . | . | . | . | . | . | . | . | . | R |
| 21-225_201C5_HC | . | . | . | . | . | . | . | D | . | . | . | . | . | . | . | . | . | . | . | . | . | . | . | . | . |
| 21-225_203F7_HC | . | . | . | . | . | . | . | D | . | . | . | . | . | . | . | . | . | . | . | . | . | . | . | . | . |
| 21-225_204H3_HC | . | . | . | . | . | . | . | . | . | . | . | . | . | . | . | . | . | . | . | . | . | . | . | . | . |
| 21-225_208B2_HC | . | . | . | . | . | . | . | D | . | . | . | . | . | C | . | . | . | . | . | . | . | . | . | . | . |
| 21-225_208F1_HC | . | R | . | . | . | . | . | D | . | . | . | . | . | . | . | . | . | . | . | . | . | . | . | . | . |
| 21-225_20B2_HC | . | . | . | . | . | . | . | N | . | . | . | . | . | . | . | I | . | . | . | . | . | . | . | . | . |
| 21-225_210G5_HC | . | . | . | . | . | . | . | D | . | . | . | . | . | C | . | . | . | . | . | . | . | . | . | . | . |
| 21-225_210G6_HC | . | . | . | . | . | . | . | D | . | . | . | . | . | . | . | . | . | . | . | . | . | . | . | . | . |
| 21-225_212B11_HC | . | . | . | . | . | . | . | D | . | . | . | . | . | . | . | . | . | . | . | . | . | . | . | . | . |
| 21-225_214G10_HC | . | . | . | . | . | . | . | D | . | . | . | . | . | . | . | . | . | . | . | . | . | . | . | . | . |
| 21-225_214G12_HC | . | . | . | . | . | . | . | D | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . | . |
| 21-225_215E11_HC | . | . | . | . | . | . | . | D | . | . | . | . | . | . | . | . | . | . | . | . | . | . | . | . | . |
| 21-225_215E3_HC | . | . | . | . | . | . | . | D | . | . | . | . | . | . | . | I | . | . | . | . | . | . | . | . | . |
| 21-225_216E8_HC | . | . | . | . | . | . | . | D | . | . | . | . | . | . | . | . | . | . | . | . | . | . | . | . | . |
| 21-225_216H12_HC | . | . | . | . | . | . | . | D | . | . | . | . | . | . | . | . | . | . | . | . | . | . | . | . | . |
| 21-225_217E5_HC | . | . | . | . | . | . | . | D | . | . | . | . | . | . | . | . | . | . | . | . | . | . | . | . | . |
| 21-225_217G10_HC | . | . | . | . | . | . | . | D | . | . | . | . | . | . | . | . | . | . | . | . | . | . | . | . | . |
| 21-225_217H12_HC | . | . | . | . | . | . | . | D | . | . | . | . | . | . | . | . | . | . | . | . | . | . | . | . | . |
| 21-225_218C4_HC | . | . | . | . | . | . | . | D | . | . | . | . | . | . | . | . | . | . | . | . | . | . | . | . | . |
| 21-225_220F8_HC | . | . | . | . | . | . | . | D | . | . | . | . | . | . | . | . | . | . | . | . | . | . | . | . | . |
| 21-225_224E7_HC | . | . | . | . | . | . | . | . | . | . | . | . | . | . | . | . | . | . | . | . | . | . | . | . | . |
| 21-225_224F1_HC | . | . | . | . | . | . | . | . | . | . | . | . | . | . | I | . | . | . | . | . | . | . | . | . | . |
| 21-225_224F3_HC | . | . | . | . | S | . | . | . | . | . | . | . | . | . | G | . | . | . | . | . | . | . | . | . | . |
| 21-225_225A1_HC | . | . | . | . | . | . | . | D | . | . | . | . | . | . | . | I | . | . | . | . | . | . | . | . | . |
| 21-225_226C6_HC | . | . | . | . | . | . | . | D | . | . | . | . | . | . | G | . | . | . | . | . | . | . | . | . | . |
| 21-225_226H11_HC | . | . | . | . | . | . | . | N | . | . | . | . | . | C | G | . | . | . | . | . | . | . | . | . | . |
| 21-225_22F4_HC | . | . | . | . | . | . | . | D | . | . | . | . | . | . | G | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | H_CDR1 | | | | | | | | | | | | | | H_FR2 | |
| CONSENSUS | S | G | - | F | T | F | S | S | - | - | - | - | - | Y | V | M | H | W | V | R | Q | A | P | G | K |
| 21-225_23A10_HC | . | . | . | . | . | . | . | . | . | . | . | . | . | . | G | . | . | . | . | . | . | . | . | . | . |
| 21-225_24B6_HC | . | . | . | . | I | . | . | N | . | . | . | . | . | . | . | . | . | . | . | . | . | . | . | . | . |
| 21-225_25A2_HC | . | . | . | . | . | . | . | . | . | . | . | . | . | . | G | . | . | . | . | . | . | . | . | . | . |
| 21-225_25D12_HC | . | . | . | . | . | . | . | . | . | . | . | . | . | D | G | . | . | . | . | . | . | . | . | . | . |
| 21-225_26A11_HC | . | . | . | . | . | . | . | N | . | . | . | . | . | C | . | . | . | . | . | . | . | . | . | . | . |
| 21-225_26G7_HC | . | . | . | . | . | . | . | . | . | . | . | . | . | . | G | . | . | . | . | . | . | . | . | . | . |
| 21-225_27E7_HC | . | . | . | . | . | . | . | . | . | . | . | . | . | . | G | . | . | . | . | . | . | . | . | . | . |
| 21-225_28H2_HC | . | . | . | . | . | . | . | N | . | . | . | . | . | . | . | . | . | . | . | . | . | . | . | . | . |
| 21-225_29E2_HC | . | . | . | . | . | . | . | N | . | . | . | . | . | C | . | . | . | . | . | . | . | . | . | . | . |
| 21-225_29H4_HC | . | . | . | . | . | . | . | . | . | . | . | . | . | . | G | I | . | . | . | . | . | . | . | . | . |
| 21-225_2G4_HC | . | . | . | . | . | . | . | . | . | . | . | . | . | . | . | . | . | . | . | . | . | . | . | . | . |
| 21-225_32H2_HC | . | . | . | . | . | . | . | . | . | . | . | . | . | . | G | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | . | . | S | . | . | D | . | . | . | . | . | . | G | . | . | . | . | . | . | . | . | . | . |
| 21-225_44B10_HC | . | . | . | . | . | . | . | . | . | . | . | . | . | . | G | . | . | . | . | . | . | . | . | . | . |
| 21-225_44B3_HC | . | . | . | . | . | . | . | D | . | . | . | . | . | . | G | . | . | . | . | . | . | . | . | . | . |
| 21-225_44C10_HC | . | . | . | . | . | . | . | D | . | . | . | . | . | C | . | . | . | . | . | . | . | . | . | . | . |
| 21-225_44D3_HC | . | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . | . |
| 21-225_44F9_HC | . | . | . | . | . | . | . | . | . | . | . | . | . | . | . | . | . | . | . | . | . | . | . | . | . |
| 21-225_45B4_HC | . | . | . | . | . | . | . | D | . | . | . | . | . | C | . | . | . | . | . | . | . | . | . | . | . |
| 21-225_45B8_HC | . | . | . | . | . | . | . | D | . | . | . | . | . | C | . | . | . | . | . | . | . | . | . | . | . |
| 21-225_46B9_HC | . | . | . | . | . | . | . | . | . | . | . | . | . | . | G | . | . | . | . | . | . | . | . | . | . |
| 21-225_46C3_HC | . | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . | . |
| 21-225_47C1_HC | . | . | . | . | . | . | . | D | . | . | . | . | . | . | G | . | . | . | . | . | . | T | . | . | . |
| 21-225_47E7_HC | . | . | . | . | . | . | . | I | . | . | . | . | . | . | G | . | . | . | . | . | . | . | . | . | . |
| 21-225_48F2_HC | . | . | . | . | . | . | . | N | . | . | . | . | . | . | . | . | . | . | . | . | . | . | . | . | . |
| 21-225_48H4_HC | . | . | . | . | . | . | . | D | . | . | . | . | . | S | L | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | | H_CDR1 | | | | | | | | | | | H_FR2 | |
| CONSENSUS | S | G | - | F | T | F | S | S | - | - | - | - | - | Y | V | M | H | W | V | R | Q | A | P | G | K |
| 21-225_49C1_HC | . | . | . | . | . | . | . | . | . | . | . | . | . | . | G | . | . | . | . | . | . | . | . | . | . |
| 21-225_49E7_HC | . | . | . | . | . | . | . | . | . | . | . | . | . | . | G | . | . | . | . | . | . | . | . | . | . |
| 21-225_49F9_HC | . | . | . | . | . | . | . | . | . | . | . | . | . | . | G | . | . | . | . | . | . | . | . | . | . |
| 21-225_49G3_HC | . | . | . | . | . | . | . | . | . | . | . | . | . | . | G | . | . | . | . | . | . | . | . | . | . |
| 21-225_4C12_HC | . | . | . | . | . | . | . | . | . | . | . | . | . | . | . | . | . | . | . | . | . | . | . | . | . |
| 21-225_4C5_HC | . | . | . | . | . | . | . | . | . | . | . | . | . | . | . | L | . | . | . | . | . | . | . | . | . |
| 21-225_50H4_HC | . | . | . | . | . | . | . | . | . | . | . | . | . | . | . | . | . | . | . | . | . | . | . | . | . |
| 21-225_51E1_HC | . | . | . | . | . | . | . | . | . | . | . | . | . | . | G | . | . | . | . | . | . | . | . | . | . |
| 21-225_52D10_HC | . | . | . | . | . | . | . | . | . | . | . | . | . | . | G | . | . | . | . | . | . | . | . | . | . |
| 21-225_52G8_HC | . | . | . | . | . | . | . | . | . | . | . | . | . | . | G | . | . | . | . | . | . | . | . | . | . |
| 21-225_54D4_HC | . | . | . | . | . | . | . | . | . | . | . | . | . | . | G | . | . | . | . | . | . | . | . | . | . |
| 21-225_55C2_HC | . | . | . | . | . | . | . | . | . | . | . | . | . | . | G | . | . | . | . | . | . | . | . | . | . |
| 21-225_55F9_HC | . | . | . | . | . | . | . | . | . | . | . | . | . | . | G | . | . | . | . | . | . | . | . | . | . |
| 21-225_55H1_HC | . | . | . | . | . | . | . | . | . | . | . | . | . | . | G | . | . | . | . | . | . | . | . | . | . |
| 21-225_58H11_HC | . | . | . | . | . | . | . | . | . | . | . | . | . | . | G | . | . | . | . | . | . | . | . | . | . |
| 21-225_59A12_HC | . | . | . | . | . | . | . | . | . | . | . | . | . | . | G | . | . | . | . | . | . | . | . | . | . |
| 21-225_5E5_HC | . | . | . | . | . | . | . | N | . | . | . | . | . | . | . | . | . | . | . | . | . | . | . | . | . |
| 21-225_5E5.003_HC | . | . | . | . | . | . | . | N | . | . | . | . | . | . | . | . | . | . | . | . | . | . | . | . | . |
| 21-225_5E5.004_HC | . | . | . | . | . | . | . | N | . | . | . | . | . | . | . | . | . | . | . | . | . | . | . | . | . |
| 21-225_5E5.005_HC | . | . | . | . | . | . | . | N | . | . | . | . | . | . | . | . | . | . | . | . | . | . | . | . | . |
| 21-225_5E5.006_HC | . | . | . | . | . | . | . | N | . | . | . | . | . | . | . | . | . | . | . | . | . | . | . | . | . |
| 21-225_5E5.011_HC | . | . | . | . | . | . | . | N | . | . | . | . | . | . | . | . | . | . | . | . | . | . | . | . | . |
| 21-225_5E5.012_HC | . | . | . | . | . | . | . | N | . | . | . | . | . | . | . | . | . | . | . | . | . | . | . | . | . |
| 21-225_5E5.015_HC | . | . | . | . | . | . | . | N | . | . | . | . | . | . | . | . | . | . | . | . | . | . | . | . | . |
| 21-225_5E5.016_HC | . | . | . | . | . | . | . | N | . | . | . | . | . | . | . | . | . | . | . | . | . | . | . | . | . |
| 21-225_5E5.017_HC | . | . | . | . | . | . | . | N | . | . | . | . | . | . | . | . | . | . | . | . | . | . | . | . | . |
| 21-225_5E5.018_HC | . | . | . | . | . | . | . | N | . | . | . | . | . | . | . | . | . | . | . | . | . | . | . | . | . |
| 21-225_5E5.019_HC | . | . | . | . | . | . | . | N | . | . | . | . | . | . | . | . | . | . | . | . | . | . | . | . | . |
| 21-225_5E5.023_HC | . | . | . | . | . | . | . | N | . | . | . | . | . | . | . | . | . | . | . | . | . | . | . | . | . |

## FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | H_FR2 | |
| CONSENSUS | S | G | - | F | T | F | S | S | - | - | - | - | - | Y | V | M | H | W | V | R | Q | A | P | G | K |
| 21-225_5E5.024_HC | | | | | | | | N | | | | | | | | | | | | | | | | | |
| 21-225_60C12_HC | | | | | | | | | | | | | | | G | | | | | | | | | | |
| 21-225_60G2_HC | | | | | | | | | | | | | | | G | | | | | | | | | | |
| 21-225_61B3_HC | | | | | | | | | | | | | | | G | | | | | | | | | | |
| 21-225_61F2_HC | | | | | | | | | | | | | | | G | | | | | | | | | | |
| 21-225_64A4_HC | | | | | | | | D | | | | | | | | | | | | | | | | | |
| 21-225_64C8_HC | | | | | | | | D | | | | | | | | | | | | | | | | | |
| 21-225_66D11_HC | | | | | | | | | | | | | | | G | | | | | | | | | | |
| 21-225_73C9_HC | | | | | | | | N | | | | | | | | | | | | | | | | | |
| 21-225_7C9_HC | | | L | | | | | N | | | | | | | | | | | | | | | | | |
| 21-225_7G4_HC | | | | | | | N | N | | | | | | | | | | | | | | | | | |
| 21-225_94F3_HC | | | | | | | | N | | | | | | D | I | | | | | | | | | | |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H_CDR2 | | | | | | | | |
| CONSENSUS | G | L | E | W | V | A | V | I | W | Y | D | - | - | - | G | S | N | K | Y | Y | A | D | S | V | K |
| 21-225_10E9_HC | . | . | . | . | . | . | . | . | . | . | . | . | . | . | . | . | . | . | . | . | . | . | . | . | . |
| 21-225_147A8_HC | . | . | . | . | . | . | . | . | . | . | . | . | . | . | . | . | . | . | . | . | . | . | . | . | . |
| 21-225_147B2_HC | . | . | . | . | . | . | . | . | . | . | . | . | . | . | . | . | . | . | . | . | . | . | . | . | . |
| 21-225_147H5_HC | . | . | . | . | . | . | . | . | . | . | . | . | . | . | . | . | . | . | . | . | . | . | . | . | . |
| 21-225_148C9_HC | . | . | . | . | . | . | . | . | . | . | . | . | . | . | . | . | . | . | . | . | . | . | . | . | . |
| 21-225_148F5_HC | . | . | . | . | . | . | . | . | . | . | . | . | . | . | . | . | . | . | . | . | . | . | . | . | . |
| 21-225_148H10_HC | . | . | . | . | . | . | . | . | . | . | . | . | . | . | . | . | . | . | . | . | G | . | . | . | . |
| 21-225_149D10_HC | . | . | . | . | . | . | . | . | . | . | . | . | . | . | . | . | . | . | . | . | . | . | . | . | . |
| 21-225_14E10_HC | . | . | . | . | . | . | . | . | . | . | . | . | . | . | . | . | . | . | . | . | . | . | . | . | . |
| 21-225_150D11_HC | . | . | . | . | . | . | . | . | F | . | . | . | . | . | . | . | . | . | H | . | . | . | . | . | . |
| 21-225_150E2_HC | . | . | . | . | . | . | . | . | . | . | . | . | . | . | . | . | . | . | . | . | . | . | . | . | . |
| 21-225_152F7_HC | . | . | . | . | . | . | . | . | . | . | . | . | . | . | . | . | . | . | . | . | . | . | . | . | . |
| 21-225_153G9_HC | . | . | . | . | . | . | L | . | F | . | . | . | . | . | . | . | . | . | . | . | . | . | . | . | . |
| 21-225_154F4_HC | . | . | . | . | . | . | . | . | . | . | . | . | . | . | . | . | . | . | . | . | . | . | . | . | . |
| 21-225_159D8_HC | . | . | . | . | . | . | . | . | . | . | . | . | . | . | . | . | . | . | . | . | . | . | . | . | . |
| 21-225_15C11_HC | . | . | . | . | . | . | . | . | . | . | . | . | . | . | . | . | . | . | . | . | . | . | . | . | . |
| 21-225_169D11_HC | . | . | . | . | . | . | . | . | . | . | . | . | . | . | . | . | . | . | . | . | . | . | . | . | . |
| 21-225_170F3_HC | . | . | . | . | . | . | . | . | . | . | . | . | . | . | . | . | . | . | . | . | E | . | . | . | . |
| 21-225_173C11_HC | . | . | . | . | . | . | L | . | F | . | . | . | . | . | . | . | . | . | . | . | . | . | . | . | . |
| 21-225_174G10_HC | . | . | . | . | . | . | . | . | . | . | . | . | . | . | . | . | . | . | . | . | . | . | . | . | . |
| 21-225_176H12_HC | . | . | . | . | . | . | . | . | . | . | . | . | . | . | . | . | . | . | . | . | . | . | . | . | . |
| 21-225_177B4_HC | . | . | . | . | . | . | . | . | . | . | . | . | . | . | . | . | . | . | . | . | . | . | . | . | . |
| 21-225_179C2_HC | . | . | . | . | . | . | L | . | F | . | . | . | . | . | . | . | . | . | . | . | . | . | . | . | . |
| 21-225_17H3_HC | . | . | . | . | . | . | . | . | . | . | . | . | . | . | . | . | . | . | . | . | . | . | . | . | . |
| 21-225_183F4_HC | . | . | . | . | . | . | . | . | . | . | . | . | . | . | . | . | . | . | . | . | . | . | . | . | . |
| 21-225_190D6_HC | . | . | . | . | . | . | . | . | . | . | . | . | . | . | . | . | . | . | . | . | . | . | . | . | . |
| 21-225_192A2_HC | . | . | . | L | . | . | . | . | . | . | . | . | . | . | . | . | Y | . | . | . | . | . | . | . | . |
| 21-225_192F6_HC | . | . | . | . | . | . | . | . | . | . | . | . | . | . | . | . | . | . | . | . | V | . | . | . | . |
| 21-225_193F8_HC | . | . | . | . | . | . | . | . | . | . | . | . | . | . | . | . | Y | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | V | A | V | I | W | Y | D | - | - | - | G | S | N | K | Y | Y | A | D | S | V | K |
| 21-225_194F10_HC | . | . | . | . | . | . | . | . | . | . | . | . | . | . | . | . | . | . | . | . | . | . | . | . | . |
| 21-225_201C5_HC | . | . | . | . | . | . | . | . | . | . | . | . | . | . | . | . | . | . | . | . | V | . | . | . | . |
| 21-225_203F7_HC | . | . | . | . | . | . | . | . | . | . | . | . | . | . | . | . | . | . | . | . | V | . | . | . | . |
| 21-225_204H3_HC | . | . | . | . | . | . | . | . | . | . | . | . | . | . | . | . | . | . | . | N | . | . | . | . | . |
| 21-225_208B2_HC | . | . | . | . | . | . | . | . | . | . | . | . | . | . | . | . | . | . | . | . | V | . | . | . | . |
| 21-225_208F1_HC | . | . | . | . | . | . | . | . | . | . | . | . | . | . | . | . | . | . | . | . | V | . | . | . | . |
| 21-225_20B2_HC | . | . | . | . | . | . | . | . | . | . | . | . | . | . | . | . | . | . | . | . | . | . | . | . | . |
| 21-225_210G5_HC | . | . | . | . | . | . | . | . | . | . | . | . | . | . | . | . | . | . | . | . | V | . | . | . | . |
| 21-225_210G6_HC | . | . | . | . | . | . | . | . | . | . | . | . | . | . | . | . | . | . | . | . | V | . | . | . | . |
| 21-225_212B11_HC | . | . | . | . | . | T | . | . | . | . | . | . | . | . | . | . | . | . | . | . | . | . | . | . | . |
| 21-225_214G10_HC | . | . | . | . | . | T | . | . | . | . | . | . | . | . | . | . | . | . | . | . | . | . | . | . | . |
| 21-225_214G12_HC | . | . | . | . | . | T | . | . | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . | . |
| 21-225_215E11_HC | . | . | . | . | . | T | . | . | . | . | . | . | . | . | . | . | . | . | . | . | . | . | . | . | . |
| 21-225_215E3_HC | . | . | . | . | . | T | . | . | . | . | . | . | . | . | . | . | . | . | . | . | . | . | . | . | . |
| 21-225_216E8_HC | . | . | . | . | . | T | . | . | . | . | . | . | . | . | . | . | . | . | . | . | . | . | . | . | . |
| 21-225_216H12_HC | . | . | . | . | . | T | . | . | . | . | . | . | . | . | . | . | . | . | . | . | . | . | . | . | . |
| 21-225_217E5_HC | . | . | . | . | . | T | . | . | . | . | . | . | . | . | . | . | . | . | . | . | . | . | . | . | . |
| 21-225_217G10_HC | . | . | . | . | . | T | . | . | . | . | . | . | . | . | . | . | . | . | . | . | . | . | . | . | . |
| 21-225_217H12_HC | . | . | . | . | . | T | . | . | . | . | . | . | . | . | . | . | . | . | . | . | . | . | . | . | . |
| 21-225_218C4_HC | . | . | . | . | . | T | . | . | . | . | . | . | . | . | . | . | . | . | . | . | . | . | . | . | . |
| 21-225_220F8_HC | . | . | . | . | . | T | . | . | . | . | . | . | . | . | . | . | . | . | . | . | . | . | . | . | . |
| 21-225_224E7_HC | . | . | . | . | . | . | . | . | . | . | . | . | . | . | . | . | . | . | . | . | . | . | . | . | . |
| 21-225_224F1_HC | . | . | . | . | . | . | . | . | . | . | . | . | . | . | . | . | . | . | . | . | . | . | . | . | . |
| 21-225_224F3_HC | . | . | . | . | . | . | . | . | . | . | . | . | . | . | . | . | . | . | . | . | . | . | . | . | . |
| 21-225_225A1_HC | . | . | . | . | . | . | . | . | . | . | . | . | . | . | . | . | . | . | . | . | . | . | . | . | . |
| 21-225_226C6_HC | . | . | . | . | . | . | . | . | . | . | . | . | . | . | . | . | . | . | . | . | . | . | . | . | . |
| 21-225_226H11_HC | . | . | . | . | . | T | . | . | . | . | . | . | . | . | . | . | . | . | . | . | . | . | . | . | . |
| 21-225_22F4_HC | . | . | . | . | . | . | . | . | . | . | . | . | . | . | . | . | . | . | . | . | V | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | V | A | V | I | W | Y | D | - | - | - | G | S | N | K | Y | Y | A | D | S | V | K |
| 21-225_23A10_HC | . | . | . | . | . | . | . | . | . | . | . | . | . | . | . | . | . | . | . | . | . | . | . | . | . |
| 21-225_24B6_HC | . | . | . | . | . | . | . | . | . | . | . | . | . | . | . | . | . | . | . | . | . | . | . | . | . |
| 21-225_25A2_HC | . | . | . | . | . | . | . | . | . | . | . | . | . | . | . | . | . | . | . | . | . | . | . | . | . |
| 21-225_25D12_HC | . | . | . | . | . | . | . | . | . | . | . | . | . | . | . | . | . | . | . | . | . | . | . | . | . |
| 21-225_26A11_HC | . | . | . | . | . | . | . | . | . | . | . | . | . | . | . | . | . | . | . | . | . | . | . | . | . |
| 21-225_26G7_HC | . | . | . | . | . | . | . | . | . | . | . | . | . | . | . | . | . | . | . | . | . | . | . | . | . |
| 21-225_27E7_HC | . | . | . | . | . | . | . | . | . | . | . | . | . | . | . | . | . | . | . | . | . | . | . | . | . |
| 21-225_28H2_HC | . | . | . | . | . | . | . | . | . | . | . | . | . | . | . | . | . | . | . | . | . | . | . | . | . |
| 21-225_29E2_HC | . | . | . | . | . | . | . | . | . | . | . | . | . | . | . | . | . | . | . | . | . | . | . | . | . |
| 21-225_29H4_HC | . | . | . | . | . | . | . | . | . | . | . | . | . | . | . | . | . | . | . | . | . | . | . | . | . |
| 21-225_2G4_HC | . | . | . | . | . | . | . | . | . | . | . | . | . | . | . | . | . | . | . | . | . | . | . | . | . |
| 21-225_32H2_HC | . | . | . | . | . | . | . | . | . | . | . | . | . | . | . | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | . | . | . | . | . | . | . | . | . | . | . | . | . | . | . | . | . | . | . | . | . | . | . |
| 21-225_44B10_HC | . | . | . | . | . | . | . | . | . | . | . | . | . | . | . | . | . | . | . | . | . | . | . | . | . |
| 21-225_44B3_HC | . | . | . | . | . | . | . | . | . | . | . | . | . | . | . | . | . | . | . | . | . | . | . | . | . |
| 21-225_44C10_HC | . | . | . | . | . | . | . | . | . | . | . | . | . | . | . | . | . | . | . | . | . | . | . | . | . |
| 21-225_44D3_HC | . | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . | . |
| 21-225_44F9_HC | . | . | . | . | . | . | . | . | . | . | . | . | . | . | . | . | . | . | . | . | . | . | . | . | . |
| 21-225_45B4_HC | . | . | . | . | . | . | . | . | . | . | . | . | . | . | . | . | . | . | . | . | . | . | . | . | . |
| 21-225_45B8_HC | . | . | . | . | . | . | . | . | . | . | . | . | . | . | . | . | . | . | . | . | . | . | . | . | . |
| 21-225_46B9_HC | . | . | . | . | . | . | . | . | . | . | . | . | . | . | . | R | . | . | . | . | . | . | . | . | . |
| 21-225_46C3_HC | . | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . | . |
| 21-225_47C1_HC | . | . | . | . | . | . | . | . | . | . | . | . | . | . | . | . | . | . | . | . | . | . | . | . | . |
| 21-225_47E7_HC | . | . | . | . | . | . | . | . | . | . | . | . | . | . | . | . | . | . | . | . | . | . | . | . | . |
| 21-225_48F2_HC | . | . | . | . | . | . | . | . | . | . | . | . | . | . | . | . | . | . | . | . | . | . | . | . | . |
| 21-225_48H4_HC | . | . | . | . | . | . | . | . | . | . | . | . | . | . | . | . | . | . | . | . | T | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H_CDR2 | | | | | | | | |
| CONSENSUS | G | L | E | W | V | A | V | I | W | Y | D | - | - | - | G | S | N | K | Y | Y | A | D | S | V | K |
| 21-225_49C1_HC | . | . | . | . | . | . | . | . | . | . | . | . | . | . | . | . | . | . | . | . | . | . | . | . | . |
| 21-225_49E7_HC | . | . | . | . | . | . | . | . | . | . | . | . | . | . | . | . | . | . | . | . | . | . | . | . | . |
| 21-225_49F9_HC | . | . | . | . | . | . | L | . | . | . | . | . | . | . | . | R | . | . | . | . | . | . | . | . | . |
| 21-225_49G3_HC | . | . | . | . | . | . | . | . | . | . | . | . | . | . | . | . | . | . | . | . | . | . | . | . | . |
| 21-225_4C12_HC | . | . | . | . | . | . | . | . | . | . | . | . | . | . | . | . | . | . | . | . | . | . | . | . | . |
| 21-225_4C5_HC | . | . | . | . | . | . | . | . | . | . | . | . | . | . | . | . | . | . | . | . | . | . | . | . | . |
| 21-225_50H4_HC | . | . | . | . | . | . | A | . | . | . | . | . | . | . | . | . | . | . | . | . | . | . | . | . | . |
| 21-225_51E1_HC | . | . | . | . | . | . | . | . | . | . | . | . | . | . | . | . | S | . | . | . | . | . | . | . | . |
| 21-225_52D10_HC | . | . | . | . | . | . | L | . | . | . | . | . | . | . | . | . | . | . | . | H | . | . | . | . | . |
| 21-225_52G8_HC | . | . | . | . | . | . | . | . | . | . | . | . | . | . | . | . | . | . | . | . | . | . | . | . | . |
| 21-225_54D4_HC | . | . | . | . | . | . | . | . | . | . | . | . | . | . | . | . | . | . | . | . | . | . | . | . | . |
| 21-225_55C2_HC | . | . | . | . | . | . | L | . | . | . | . | . | . | . | . | . | . | . | . | H | . | . | . | . | . |
| 21-225_55F9_HC | . | . | . | . | . | . | . | . | . | . | . | . | . | . | . | N | G | . | . | . | . | . | . | . | . |
| 21-225_55H1_HC | . | . | . | . | . | . | . | . | . | . | . | . | . | . | . | . | . | . | . | . | . | . | . | . | . |
| 21-225_58H11_HC | . | . | . | . | . | . | . | . | . | . | . | . | . | . | . | . | . | . | . | H | . | . | . | . | . |
| 21-225_59A12_HC | . | . | . | . | . | . | . | . | . | . | . | . | . | . | . | . | . | . | . | . | . | . | . | . | . |
| 21-225_5E5_HC | . | . | . | . | . | . | . | . | . | . | . | . | . | . | . | . | . | . | . | . | . | . | . | . | . |
| 21-225_5E5.003_HC | . | . | . | . | . | . | . | . | . | . | . | . | . | . | A | . | . | . | . | . | . | . | . | . | . |
| 21-225_5E5.004_HC | . | . | . | . | . | . | . | . | . | . | . | . | . | . | . | . | . | . | . | . | . | E | . | . | . |
| 21-225_5E5.005_HC | . | . | . | . | . | . | . | . | . | . | . | . | . | . | . | . | . | . | . | . | . | G | . | . | . |
| 21-225_5E5.006_HC | . | . | . | . | . | . | . | . | . | . | . | . | . | . | . | . | . | . | . | . | . | . | A | . | . |
| 21-225_5E5.011_HC | . | . | . | . | . | . | . | . | . | . | . | . | . | . | A | . | . | . | . | . | . | E | . | . | . |
| 21-225_5E5.012_HC | . | . | . | . | . | . | . | . | . | . | . | . | . | . | A | . | . | . | . | . | . | . | A | . | . |
| 21-225_5E5.015_HC | . | . | . | . | . | . | . | . | . | . | . | . | . | . | A | . | . | . | . | . | . | . | . | . | . |
| 21-225_5E5.016_HC | . | . | . | . | . | . | . | . | . | . | . | . | . | . | A | . | . | . | . | . | . | . | . | . | . |
| 21-225_5E5.017_HC | . | . | . | . | . | . | . | . | . | . | . | . | . | . | . | . | . | . | . | . | . | E | . | . | . |
| 21-225_5E5.018_HC | . | . | . | . | . | . | . | . | . | . | . | . | . | . | . | . | . | . | . | . | . | . | . | . | . |
| 21-225_5E5.019_HC | . | . | . | . | . | . | . | . | . | . | . | . | . | . | . | . | . | . | . | . | . | . | . | . | . |
| 21-225_5E5.023_HC | . | . | . | . | . | . | . | . | . | . | . | . | . | . | A | . | . | . | . | . | . | E | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H_CDR2 | | | | | | | | |
| CONSENSUS | G | L | E | W | V | A | V | I | W | Y | D | - | - | - | G | S | N | K | Y | Y | A | D | S | V | K |
| 21-225_5E5.024_HC | . | . | . | . | . | . | . | . | . | . | . | . | . | . | A | . | . | . | . | . | . | . | A | . | . |
| 21-225_60C12_HC | . | . | . | . | . | . | . | . | . | . | . | . | . | . | . | . | . | . | . | . | V | . | . | . | . |
| 21-225_60G2_HC | . | . | . | . | . | . | . | . | . | . | . | . | . | . | . | . | . | . | . | . | . | . | . | . | . |
| 21-225_61B3_HC | . | . | . | . | . | . | . | . | . | . | . | . | . | . | . | . | . | . | . | . | . | . | . | . | . |
| 21-225_61F2_HC | . | . | . | . | . | . | . | . | . | . | . | . | . | . | . | . | . | . | . | . | . | . | . | . | . |
| 21-225_64A4_HC | . | . | . | . | . | . | . | . | . | . | . | . | . | . | . | . | . | . | . | . | . | . | . | . | . |
| 21-225_64C8_HC | . | . | . | . | . | . | . | . | . | . | . | . | . | . | . | . | . | . | . | . | . | . | . | M | . |
| 21-225_66D11_HC | . | . | . | . | . | . | . | . | . | . | . | . | . | . | . | . | . | . | . | . | . | . | . | . | . |
| 21-225_73C9_HC | . | . | . | . | . | . | . | . | . | . | . | . | . | . | . | . | . | . | . | . | . | . | . | . | . |
| 21-225_7C9_HC | . | . | . | . | . | . | . | . | . | . | . | . | . | . | . | . | . | . | . | . | . | . | . | . | . |
| 21-225_7G4_HC | . | . | . | . | . | . | . | . | . | . | . | . | . | . | . | . | . | . | . | . | . | . | . | . | . |
| 21-225_94F3_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | F | T | I | S | R | D | N | S | K | N | T | L | Y | L | Q | M | N | S | L | R | A | E | D |
| 21-225_10E9_HC | . | . | . | . | . | . | . | . | . | . | . | . | . | . | . | . | . | . | . | . | . | . | . | . | . |
| 21-225_147A8_HC | . | . | . | . | . | . | . | . | . | . | . | . | . | . | . | . | . | . | . | . | . | . | . | . | . |
| 21-225_147B2_HC | . | . | . | . | . | . | . | . | . | . | . | . | . | . | . | . | . | . | . | . | . | . | . | . | . |
| 21-225_147H5_HC | . | . | L | . | . | . | . | . | . | . | . | . | . | . | . | . | . | . | . | . | . | S | . | . | . |
| 21-225_148C9_HC | . | . | L | . | . | . | . | . | . | . | . | . | . | . | . | . | . | . | . | . | . | . | . | . | . |
| 21-225_148F5_HC | . | . | . | . | . | . | . | . | . | . | . | . | . | . | . | . | . | . | . | . | . | . | . | . | . |
| 21-225_148H10_HC | . | . | . | . | . | . | . | . | . | . | . | . | . | . | . | . | . | . | . | . | . | . | . | . | . |
| 21-225_149D10_HC | . | . | L | . | . | . | . | . | . | . | . | . | . | . | . | . | . | . | . | . | . | . | . | . | . |
| 21-225_14E10_HC | . | . | . | . | . | . | . | . | . | . | . | . | . | . | . | . | . | . | . | . | . | . | . | . | . |
| 21-225_150D11_HC | . | . | . | . | . | . | . | . | . | . | . | . | . | . | . | . | . | . | . | . | . | . | . | . | . |
| 21-225_150E2_HC | . | . | . | . | . | . | . | . | . | . | . | . | . | . | . | . | . | . | . | . | . | . | . | . | . |
| 21-225_152F7_HC | . | . | . | . | . | . | . | . | . | . | . | . | . | . | . | . | . | . | . | . | . | . | . | . | . |
| 21-225_153G9_HC | . | . | . | . | . | . | . | . | . | . | . | . | . | . | . | . | . | I | . | . | . | . | . | . | . |
| 21-225_154F4_HC | . | . | . | . | . | . | . | . | . | . | . | . | . | . | . | . | . | . | . | . | . | . | . | . | . |
| 21-225_159D8_HC | . | . | . | . | . | . | . | . | . | . | . | . | . | . | . | . | . | . | . | . | . | . | . | . | . |
| 21-225_15C11_HC | . | . | . | . | . | . | . | . | . | . | . | . | . | . | . | . | . | . | . | . | . | . | . | . | . |
| 21-225_169D11_HC | . | . | . | . | . | . | . | . | . | . | . | . | . | . | . | . | . | L | . | . | . | . | . | . | . |
| 21-225_170F3_HC | . | . | . | . | . | . | . | . | . | . | . | . | . | . | . | . | . | . | . | . | . | . | . | . | . |
| 21-225_173C11_HC | . | . | . | . | . | . | . | . | . | . | . | . | . | . | . | . | H | . | S | . | . | . | . | . | . |
| 21-225_174G10_HC | . | . | . | . | . | . | . | . | . | . | . | . | . | . | . | . | . | L | . | . | . | . | . | . | . |
| 21-225_176H12_HC | . | . | . | . | . | . | . | . | . | . | . | . | . | . | . | . | . | . | . | . | . | . | . | . | . |
| 21-225_177B4_HC | . | . | . | I | V | . | . | . | . | . | . | . | . | . | . | . | . | . | . | . | . | . | . | . | . |
| 21-225_179C2_HC | . | . | . | . | . | . | . | . | . | . | . | . | . | . | F | . | . | . | . | . | . | . | . | . | . |
| 21-225_17H3_HC | . | . | . | . | . | . | . | . | . | . | . | . | . | . | . | . | . | . | . | . | . | . | . | . | . |
| 21-225_183F4_HC | . | . | . | . | . | . | . | . | . | . | . | . | . | . | . | . | . | . | . | . | . | . | . | . | . |
| 21-225_190D6_HC | . | . | . | . | . | . | . | . | . | . | . | . | . | . | . | . | . | . | . | . | . | . | . | . | . |
| 21-225_192A2_HC | . | . | . | . | . | . | . | . | . | . | . | . | . | . | . | . | . | . | . | . | . | . | . | . | . |
| 21-225_192F6_HC | . | . | . | . | . | . | . | . | . | . | . | . | . | . | . | . | . | . | . | . | . | . | . | . | . |
| 21-225_193F8_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | F | T | I | S | R | D | N | S | K | N | T | L | Y | L | Q | M | N | S | L | R | A | E | D |
| 21-225_194F10_HC | . | . | . | . | . | . | . | . | . | . | . | . | . | . | . | . | E | . | . | . | . | . | V | . | . |
| 21-225_201C5_HC | . | . | . | . | . | . | . | . | . | . | R | . | . | . | . | . | . | . | . | . | . | . | . | . | . |
| 21-225_203F7_HC | . | . | . | . | . | . | . | . | . | . | R | . | . | . | . | . | . | . | . | . | . | . | . | . | . |
| 21-225_204H3_HC | . | . | . | . | . | . | . | . | . | . | . | . | . | . | . | . | . | . | . | . | . | . | . | . | . |
| 21-225_208B2_HC | . | . | . | . | . | . | . | . | . | . | R | . | . | . | . | . | . | . | . | . | . | . | . | . | . |
| 21-225_208F1_HC | . | . | . | . | . | . | . | . | . | . | R | . | . | . | . | . | . | . | . | . | . | . | . | . | . |
| 21-225_20B2_HC | . | . | . | . | . | . | . | . | . | . | . | . | . | . | . | . | . | . | . | . | . | . | . | . | . |
| 21-225_210G5_HC | . | . | . | . | . | . | . | . | . | . | R | . | . | . | . | . | . | . | . | . | . | . | . | . | . |
| 21-225_210G6_HC | . | . | . | . | . | . | . | . | . | . | R | . | . | . | . | . | . | . | . | . | . | . | . | . | . |
| 21-225_212B11_HC | . | . | . | . | . | . | . | . | . | . | . | . | . | . | . | . | . | . | . | . | . | . | . | . | . |
| 21-225_214G10_HC | . | . | . | . | . | . | . | . | . | . | . | . | . | . | . | . | . | . | . | . | . | . | . | . | . |
| 21-225_214G12_HC | . | . | . | . | . | . | . | . | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . | . |
| 21-225_215E11_HC | . | . | . | . | . | . | . | . | . | . | . | . | . | . | . | . | . | . | . | . | . | . | . | . | . |
| 21-225_215E3_HC | . | . | . | . | . | . | . | . | . | . | . | . | . | . | . | . | . | . | . | . | V | . | . | . | . |
| 21-225_216E8_HC | . | . | . | . | . | . | . | . | . | . | . | . | . | . | . | . | . | . | . | . | . | . | . | . | . |
| 21-225_216H12_HC | . | . | . | . | . | . | . | . | . | . | . | . | . | . | . | . | . | . | . | . | . | . | . | . | . |
| 21-225_217E5_HC | . | . | . | . | . | . | . | . | . | . | . | . | . | . | . | . | . | . | . | . | . | . | . | . | . |
| 21-225_217G10_HC | . | . | . | . | . | . | . | . | . | . | . | . | . | . | . | . | . | . | . | . | . | . | . | . | . |
| 21-225_217H12_HC | . | . | . | . | . | . | . | . | . | . | . | . | . | . | . | . | . | . | . | . | . | . | . | . | . |
| 21-225_218C4_HC | . | . | . | . | . | . | . | . | . | . | . | . | . | . | . | . | . | . | . | . | . | . | . | . | . |
| 21-225_220F8_HC | . | . | . | . | . | . | . | . | . | . | . | . | . | . | . | . | . | . | . | . | . | . | . | . | . |
| 21-225_224E7_HC | . | . | . | . | . | . | . | . | . | . | . | . | . | . | . | . | . | . | . | . | . | . | . | . | . |
| 21-225_224F1_HC | . | . | . | . | . | . | . | . | . | . | . | . | . | . | . | . | . | . | . | . | . | . | . | . | . |
| 21-225_224F3_HC | . | . | . | . | . | . | . | . | . | . | . | . | . | . | . | . | . | . | . | . | . | . | . | . | . |
| 21-225_225A1_HC | . | . | . | . | . | . | . | . | . | . | . | . | . | . | . | . | . | . | . | . | . | . | . | . | . |
| 21-225_226C6_HC | . | . | . | . | . | . | . | . | . | . | . | . | . | . | . | . | . | T | . | . | . | . | . | . | . |
| 21-225_226H11_HC | . | . | . | . | . | . | . | . | . | . | . | . | . | . | . | . | . | . | . | . | . | . | . | . | . |
| 21-225_22F4_HC | . | . | . | . | . | . | . | . | . | . | . | . | . | . | . | . | . | . | . | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | F | T | I | S | R | D | N | S | K | N | T | L | Y | L | Q | M | N | S | L | R | A | E | D |
| 21-225_23A10_HC | . | . | . | . | . | . | . | . | . | . | . | . | . | . | . | . | . | . | . | . | . | . | . | . | . |
| 21-225_24B6_HC | . | . | . | . | . | . | . | . | . | . | . | . | . | . | . | . | . | . | . | . | . | . | . | . | . |
| 21-225_25A2_HC | . | . | . | . | . | . | . | . | . | . | . | . | . | . | . | . | . | . | . | . | . | . | . | . | . |
| 21-225_25D12_HC | . | . | . | . | . | . | . | . | . | . | . | . | . | . | . | . | . | . | . | . | . | . | . | . | . |
| 21-225_26A11_HC | . | . | . | . | . | . | . | . | . | . | . | . | . | . | . | . | . | . | . | . | . | . | . | . | . |
| 21-225_26G7_HC | . | . | . | . | . | . | . | . | . | . | N | . | . | . | . | . | . | . | . | . | . | . | . | . | . |
| 21-225_27E7_HC | . | . | . | . | . | . | . | . | . | . | . | . | . | . | . | . | . | . | . | . | . | . | . | . | . |
| 21-225_28H2_HC | . | . | . | . | . | . | . | . | . | . | . | . | . | . | . | . | . | . | . | . | . | . | . | V | . |
| 21-225_29E2_HC | . | . | I | . | . | . | . | . | . | . | . | . | . | . | . | . | . | . | . | . | . | . | . | . | . |
| 21-225_29H4_HC | . | . | . | . | . | . | . | . | . | . | . | . | . | . | . | . | . | . | . | . | . | . | . | . | . |
| 21-225_2G4_HC | . | . | . | . | . | . | . | . | . | . | . | . | . | . | . | . | . | . | . | . | . | . | . | . | . |
| 21-225_32H2_HC | . | . | . | . | . | . | . | . | . | . | . | . | . | . | N | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | L | . | . | . | . | . | . | . | . | . | . | . | . | . | . | . | . | . | . | . | . | . | . |
| 21-225_44B10_HC | . | . | . | . | . | . | . | . | . | . | . | . | . | . | . | . | . | . | . | . | . | . | . | . | . |
| 21-225_44B3_HC | . | . | . | . | . | . | . | . | . | . | . | . | . | . | . | . | . | . | . | . | . | . | . | . | . |
| 21-225_44C10_HC | . | . | . | . | . | . | . | . | . | . | . | . | . | . | . | . | . | . | . | . | . | . | . | . | . |
| 21-225_44D3_HC | . | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . | . |
| 21-225_44F9_HC | . | . | . | . | . | . | . | . | . | . | . | . | . | . | . | . | . | . | . | . | . | . | . | . | . |
| 21-225_45B4_HC | . | . | . | . | . | . | . | . | . | . | . | . | . | . | . | . | . | . | . | . | . | . | . | . | . |
| 21-225_45B8_HC | . | . | . | . | . | . | . | . | . | . | . | . | . | . | . | . | . | . | . | . | . | . | . | . | . |
| 21-225_46B9_HC | . | . | I | . | . | . | . | . | . | . | . | . | . | . | . | . | . | . | . | . | . | . | . | . | . |
| 21-225_46C3_HC | . | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . | . |
| 21-225_47C1_HC | . | . | . | . | . | . | . | . | . | . | . | . | . | . | F | . | . | . | . | . | . | . | D | . | . |
| 21-225_47E7_HC | . | . | . | . | . | . | . | . | . | . | . | . | . | . | . | . | . | . | . | . | . | . | . | . | . |
| 21-225_48F2_HC | . | . | . | . | . | . | . | . | . | . | . | . | . | . | . | . | . | . | . | . | . | . | . | . | . |
| 21-225_48H4_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | F | T | I | S | R | D | N | S | K | N | T | L | Y | L | Q | M | N | S | L | R | A | E | D |
| 21-225_49C1_HC | . | . | . | . | . | . | . | . | . | . | . | . | . | . | . | . | . | . | . | . | . | . | . | . | . |
| 21-225_49E7_HC | . | . | . | . | . | . | . | . | . | . | . | . | . | . | . | . | . | . | . | . | . | . | . | . | . |
| 21-225_49F9_HC | . | . | . | . | . | . | . | . | . | P | . | . | . | . | . | . | . | . | . | . | . | . | . | . | . |
| 21-225_49G3_HC | . | . | . | . | . | . | . | . | . | . | . | . | . | . | . | . | . | . | . | . | . | . | . | . | . |
| 21-225_4C12_HC | . | . | . | . | . | . | . | . | . | . | . | . | . | . | . | . | . | . | . | . | . | . | . | . | . |
| 21-225_4C5_HC | . | . | . | . | . | . | . | . | . | . | . | . | . | . | . | . | . | . | . | . | . | . | . | . | . |
| 21-225_50H4_HC | . | . | . | . | . | . | . | . | . | . | . | . | . | . | D | . | . | . | . | . | . | . | . | . | . |
| 21-225_51E1_HC | . | . | . | . | . | . | . | . | . | . | . | . | . | . | . | . | . | . | . | . | . | . | . | . | . |
| 21-225_52D10_HC | . | . | . | . | . | . | . | . | . | . | . | . | . | . | . | . | . | . | . | . | . | . | . | . | . |
| 21-225_52G8_HC | . | . | . | . | . | . | . | . | . | . | . | . | . | . | . | . | . | . | . | . | . | . | . | . | . |
| 21-225_54D4_HC | . | . | . | . | . | . | . | . | . | . | . | . | . | . | . | . | . | . | . | . | . | . | . | . | . |
| 21-225_55C2_HC | . | . | . | . | . | . | . | . | . | . | . | . | . | . | . | . | . | . | . | . | . | . | . | . | . |
| 21-225_55F9_HC | . | . | . | . | . | . | . | . | . | . | . | . | . | . | . | . | . | . | . | . | . | . | . | . | . |
| 21-225_55H1_HC | . | . | . | . | . | . | . | . | . | . | . | . | . | . | . | . | . | . | . | . | . | . | . | . | . |
| 21-225_58H11_HC | . | . | . | . | . | . | . | . | . | . | . | . | . | . | . | . | . | . | . | . | . | . | . | . | . |
| 21-225_59A12_HC | . | . | . | . | . | . | . | . | . | . | . | . | . | . | . | . | . | . | . | . | . | . | . | . | . |
| 21-225_5E5_HC | . | . | . | . | . | . | . | . | . | . | . | . | . | . | . | . | . | . | . | . | . | . | . | . | . |
| 21-225_5E5.003_HC | . | . | . | . | . | . | . | . | . | . | . | . | . | . | . | . | . | . | . | . | . | . | . | . | . |
| 21-225_5E5.004_HC | . | . | . | . | . | . | . | . | . | . | . | . | . | . | . | . | . | . | . | . | . | . | . | . | . |
| 21-225_5E5.005_HC | . | . | . | . | . | . | . | . | . | . | . | . | . | . | . | . | . | . | . | . | . | . | . | . | . |
| 21-225_5E5.006_HC | . | . | . | . | . | . | . | . | . | . | . | . | . | . | . | . | . | . | . | . | . | . | . | . | . |
| 21-225_5E5.011_HC | . | . | . | . | . | . | . | . | . | . | . | . | . | . | . | . | . | . | . | . | . | . | . | . | . |
| 21-225_5E5.012_HC | . | . | . | . | . | . | . | . | . | . | . | . | . | . | . | . | . | . | . | . | . | . | . | . | . |
| 21-225_5E5.015_HC | . | . | . | . | . | . | . | . | . | . | . | . | . | . | . | . | . | . | . | . | . | . | . | . | . |
| 21-225_5E5.016_HC | . | . | . | . | . | . | . | . | . | . | . | . | . | . | . | . | . | . | . | . | . | . | . | . | . |
| 21-225_5E5.017_HC | . | . | . | . | . | . | . | . | . | . | . | . | . | . | . | . | . | . | . | . | . | . | . | . | . |
| 21-225_5E5.018_HC | . | . | . | . | . | . | . | . | . | . | . | . | . | . | . | . | . | . | . | . | . | . | . | . | . |
| 21-225_5E5.019_HC | . | . | . | . | . | . | . | . | . | . | . | . | . | . | . | . | . | . | . | . | . | . | . | . | . |
| 21-225_5E5.023_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | | H_FR3 | | | | | | | |
| CONSENSUS | G | R | F | T | I | S | R | D | N | S | K | N | T | L | Y | L | Q | M | N | S | L | R | A | E | D |
| 21-225_5E5.024_HC | . | . | . | . | . | . | . | . | . | . | . | . | . | . | . | . | . | . | . | . | . | . | . | . | . |
| 21-225_60C12_HC | . | . | . | . | . | . | . | . | . | . | . | . | . | . | . | . | . | . | . | . | . | . | . | . | . |
| 21-225_60G2_HC | . | . | . | . | . | . | . | . | . | . | . | . | . | . | . | . | . | . | . | . | . | . | . | . | . |
| 21-225_61B3_HC | . | . | . | . | . | . | . | . | . | . | . | . | . | . | . | . | . | . | . | . | . | . | . | . | . |
| 21-225_61F2_HC | . | . | . | . | . | . | . | . | . | . | . | . | . | . | . | . | . | . | . | . | . | . | . | . | . |
| 21-225_64A4_HC | . | . | . | . | . | . | . | . | . | . | Q | . | . | . | . | . | . | . | . | . | . | . | . | . | . |
| 21-225_64C8_HC | . | . | . | . | . | . | . | . | . | . | . | . | . | . | . | . | . | . | . | . | . | . | . | . | . |
| 21-225_66D11_HC | . | . | . | . | . | . | . | . | . | . | . | . | . | . | S | . | . | . | . | . | . | . | . | . | . |
| 21-225_73C9_HC | . | . | . | . | . | . | . | . | . | . | . | . | . | . | . | . | . | . | . | . | . | . | . | . | . |
| 21-225_7C9_HC | . | . | . | . | . | . | . | . | . | . | . | . | . | . | . | . | . | . | . | . | . | . | . | . | . |
| 21-225_7G4_HC | . | . | . | . | . | . | . | . | . | . | . | . | . | . | . | . | . | . | T | . | . | . | . | . | . |
| 21-225_94F3_HC | . | . | . | . | . | . | . | . | . | . | . | . | . | . | . | . | . | I | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | E | R | Y | S | S | G | W | - | - | - | - | - | - | - | - | - | - |
| 21-225_10E9_HC | . | . | . | . | . | . | . | . | . | K | . | . | . | S | . | . | . | . | . | . | . | . | . | . | . |
| 21-225_147A8_HC | . | . | . | . | . | . | . | . | R | Y | S | . | . | W | - | . | . | . | . | . | . | . | . | . | . |
| 21-225_147B2_HC | . | . | . | . | . | . | . | . | R | Y | S | . | . | W | - | . | . | . | . | . | . | . | . | . | . |
| 21-225_147H5_HC | . | . | . | . | . | . | . | . | . | . | . | . | . | . | . | . | . | . | . | . | . | . | . | . | . |
| 21-225_148C9_HC | . | . | . | . | . | . | . | . | . | . | . | . | . | . | . | . | . | . | . | . | . | . | . | . | . |
| 21-225_148F5_HC | . | . | . | . | . | . | . | . | R | Y | S | . | . | W | - | . | . | . | . | . | . | . | . | . | . |
| 21-225_148H10_HC | . | . | . | . | . | . | . | . | R | Y | S | . | . | W | - | . | . | . | . | . | . | . | . | . | . |
| 21-225_149D10_HC | . | . | . | . | . | . | . | . | . | . | . | . | . | . | . | . | . | . | . | . | . | . | . | . | . |
| 21-225_14E10_HC | . | . | . | . | . | . | . | . | . | K | . | . | . | S | . | . | . | . | . | . | . | . | . | . | . |
| 21-225_150D11_HC | S | . | . | . | . | . | T | . | R | F | S | . | . | W | - | . | . | . | . | . | . | . | . | . | . |
| 21-225_150E2_HC | . | . | . | . | . | . | . | . | . | E | . | . | . | S | . | . | . | . | . | . | . | . | . | . | . |
| 21-225_152F7_HC | . | . | . | . | . | . | . | . | . | E | . | . | . | S | . | . | . | . | . | . | . | . | . | . | . |
| 21-225_153G9_HC | . | . | . | . | F | . | . | . | R | Y | S | R | . | W | . | . | . | . | . | . | . | . | . | . | . |
| 21-225_154F4_HC | . | . | . | . | . | . | . | . | . | D | S | Y | C | S | G | T | S | C | . | . | . | . | . | . | . |
| 21-225_159D8_HC | . | . | . | . | . | . | . | . | . | P | . | . | . | . | . | . | . | . | . | . | . | . | . | . | . |
| 21-225_15C11_HC | . | . | . | . | . | . | . | . | . | K | . | . | . | S | . | . | . | . | . | . | . | . | . | . | . |
| 21-225_169D11_HC | . | . | . | . | . | . | . | . | . | . | . | . | . | . | . | . | . | . | . | . | . | . | . | . | . |
| 21-225_170F3_HC | . | . | . | . | . | . | . | . | . | T | . | . | . | S | . | . | . | . | . | . | . | . | . | . | . |
| 21-225_173C11_HC | . | . | . | . | . | . | . | . | R | Y | S | . | . | W | - | . | . | . | . | . | . | . | . | . | . |
| 21-225_174G10_HC | . | . | . | . | . | . | . | . | . | . | . | . | . | . | . | . | . | . | . | . | . | . | . | . | . |
| 21-225_176H12_HC | . | . | . | . | . | . | . | . | . | K | . | . | . | N | . | . | . | . | . | . | . | . | . | . | . |
| 21-225_177B4_HC | . | . | . | . | . | . | . | . | . | K | . | . | . | S | . | . | . | . | . | . | . | . | . | . | . |
| 21-225_179C2_HC | . | . | . | . | . | . | . | . | R | Y | S | G | . | W | - | . | . | . | . | . | . | . | . | . | . |
| 21-225_17H3_HC | . | . | . | . | . | . | . | . | . | K | . | . | . | S | . | . | . | . | . | . | . | . | . | . | . |
| 21-225_183F4_HC | . | . | . | . | . | . | . | . | . | . | T | Y | C | S | G | T | T | C | . | . | . | . | . | . | . |
| 21-225_190D6_HC | . | . | . | . | . | . | . | . | . | E | . | . | . | . | . | . | . | . | . | . | . | . | . | . | . |
| 21-225_192A2_HC | . | . | . | . | . | . | . | . | . | D | S | P | Y | S | G | Y | A | . | . | . | . | . | . | . | . |
| 21-225_192F6_HC | . | . | . | . | . | . | . | . | . | E | . | . | . | . | . | . | . | . | . | . | . | . | . | . | . |
| 21-225_193F8_HC | . | . | . | . | . | . | V | . | . | D | S | P | Y | S | G | Y | G | . | . | . | . | . | . | . | . |

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | E | R | Y | S | S | G | W | - | - | - | - | - | - | - | - | - | - |
| 21-225_194F10_HC | . | . | . | . | . | . | . | . | . | E | . | . | . | . | . | . | . | . | . | . | . | . | . | . | . |
| 21-225_201C5_HC | . | . | . | . | . | . | . | . | . | . | . | . | . | . | L | . | . | . | . | . | . | . | . | . | . |
| 21-225_203F7_HC | . | . | . | . | . | . | . | . | . | . | . | . | . | . | L | . | . | . | . | . | . | . | . | . | . |
| 21-225_204H3_HC | . | . | . | . | . | . | . | . | . | P | . | . | . | S | . | . | . | . | . | . | . | . | . | . | . |
| 21-225_208B2_HC | . | . | . | . | . | . | . | . | . | . | . | . | . | . | L | . | . | . | . | . | . | . | . | . | . |
| 21-225_208F1_HC | . | . | . | . | . | . | . | . | . | . | . | . | . | . | L | . | . | . | . | . | . | . | . | . | . |
| 21-225_20B2_HC | . | . | . | . | S | . | . | . | . | K | . | . | . | S | . | . | . | . | . | . | . | . | . | . | . |
| 21-225_210G5_HC | . | . | . | . | . | . | . | . | . | . | . | . | . | . | L | . | . | . | . | . | . | . | . | . | . |
| 21-225_210G6_HC | . | . | . | . | . | . | . | . | . | . | . | . | . | . | L | . | . | . | . | . | . | . | . | . | . |
| 21-225_212B11_HC | . | . | . | . | . | . | . | . | . | . | . | . | . | . | . | . | . | . | . | . | . | . | . | . | . |
| 21-225_214G10_HC | . | . | . | . | . | . | . | . | . | . | . | . | . | . | . | . | . | . | . | . | . | . | . | . | . |
| 21-225_214G12_HC | . | . | . | . | . | . | . | . | . | . | . | . | . | . | . | . | . | . | . | . | . | . | . | . | . |
| 21-225_214H9_HC | . | . | . | F | . | . | . | . | . | . | . | T | . | S | . | . | . | . | . | . | . | . | . | . | . |
| 21-225_215E11_HC | . | . | . | . | . | . | . | . | . | . | . | . | . | . | . | . | . | . | . | . | . | . | . | . | . |
| 21-225_215E3_HC | . | . | . | . | . | . | . | . | . | . | . | . | . | . | . | . | . | . | . | . | . | . | . | . | . |
| 21-225_216E8_HC | . | . | . | . | . | . | . | . | . | . | . | . | . | . | . | . | . | . | . | . | . | . | . | . | . |
| 21-225_216H12_HC | . | . | . | . | . | . | . | . | . | . | . | . | . | . | . | . | . | . | . | . | . | . | . | . | . |
| 21-225_217E5_HC | . | . | . | . | . | . | . | . | . | . | . | . | . | . | . | . | . | . | . | . | . | . | . | . | . |
| 21-225_217G10_HC | . | . | . | . | . | . | . | . | . | . | . | . | . | . | . | . | . | . | . | . | . | . | . | . | . |
| 21-225_217H12_HC | . | . | . | . | . | . | . | . | . | . | . | . | . | . | . | . | . | . | . | . | . | . | . | . | . |
| 21-225_218C4_HC | . | . | . | . | . | . | . | . | . | . | . | . | . | . | . | . | . | . | . | . | . | . | . | . | . |
| 21-225_220F8_HC | . | . | . | . | . | . | . | . | . | . | . | . | . | . | . | . | . | . | . | . | . | . | . | . | . |
| 21-225_224E7_HC | . | . | . | . | . | . | . | . | . | Q | . | . | . | S | . | . | . | . | . | . | . | . | . | . | . |
| 21-225_224F1_HC | . | . | . | . | . | . | . | . | . | . | . | . | . | N | . | . | . | . | . | . | . | . | . | . | . |
| 21-225_224F3_HC | . | . | . | . | . | . | . | . | . | . | . | . | . | S | . | . | . | . | . | . | . | . | . | . | . |
| 21-225_225A1_HC | . | . | . | . | . | . | . | . | . | . | . | . | . | . | . | . | . | . | . | . | . | . | . | . | . |
| 21-225_226C6_HC | . | . | . | . | . | . | . | . | . | . | . | . | . | S | . | . | . | . | . | . | . | . | . | . | . |
| 21-225_226H11_HC | . | . | . | . | . | . | . | . | . | L | . | . | . | S | . | . | . | . | . | . | . | . | . | . | . |
| 21-225_22F4_HC | . | . | . | . | . | . | . | . | . | K | . | . | . | S | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | E | R | Y | S | S | G | W | - | - | - | - | - | - | - | - | - | - |
| 21-225_23A10_HC | . | . | . | . | . | . | . | . | . | . | . | . | . | . | . | . | . | . | . | . | . | . | . | . | . |
| 21-225_24B6_HC | . | . | . | . | . | . | . | . | . | E | . | . | . | . | . | . | . | . | . | . | . | . | . | . | . |
| 21-225_25A2_HC | . | . | . | . | . | . | . | . | R | Y | S | . | . | W | - | . | . | . | . | . | . | . | . | . | . |
| 21-225_25D12_HC | . | . | . | . | . | . | . | . | . | . | . | . | . | S | . | . | . | . | . | . | . | . | . | . | . |
| 21-225_26A11_HC | . | . | . | . | . | . | . | . | . | E | . | . | . | . | . | . | . | . | . | . | . | . | . | . | . |
| 21-225_26G7_HC | . | . | . | . | . | . | . | . | R | Y | S | . | . | W | - | . | . | . | . | . | . | . | . | . | . |
| 21-225_27E7_HC | . | . | . | . | . | . | . | . | . | D | S | Y | C | S | S | T | S | C | . | . | . | . | . | . | . |
| 21-225_28H2_HC | . | . | . | . | . | . | . | . | . | E | . | . | . | . | . | . | . | . | . | . | . | . | . | . | . |
| 21-225_29E2_HC | . | . | . | . | . | . | . | . | . | E | . | . | . | . | . | . | . | . | . | . | . | . | . | . | . |
| 21-225_29H4_HC | . | . | . | . | . | . | . | . | R | Y | S | . | . | W | - | . | . | . | . | . | . | . | . | . | . |
| 21-225_2G4_HC | . | . | . | . | . | . | . | . | . | K | . | . | . | S | . | . | . | . | . | . | . | . | . | . | . |
| 21-225_32H2_HC | . | G | . | . | . | . | . | . | R | Y | S | . | . | W | - | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | . | . | . | . | . | . | R | Y | V | R | . | W | - | . | . | . | . | . | . | . | . | . | . |
| 21-225_44B10_HC | . | . | . | . | . | . | . | . | R | Y | S | . | . | W | - | . | . | . | . | . | . | . | . | . | . |
| 21-225_44B3_HC | . | . | . | . | . | . | . | . | . | . | . | . | . | . | . | . | . | . | . | . | . | . | . | . | . |
| 21-225_44C10_HC | . | . | . | . | . | . | . | . | . | . | . | . | . | . | L | . | . | . | . | . | . | . | . | . | . |
| 21-225_44D3_HC | . | . | . | . | . | . | . | . | . | . | . | . | . | . | L | . | . | . | . | . | . | . | . | . | . |
| 21-225_44D5_HC | . | . | . | . | . | . | . | . | V | P | . | . | . | S | . | . | . | . | . | . | . | . | . | . | . |
| 21-225_44F9_HC | . | . | . | . | . | . | . | . | . | P | . | . | . | S | . | . | . | . | . | . | . | . | . | . | . |
| 21-225_45B4_HC | . | . | . | . | . | . | . | . | . | . | . | . | . | . | L | . | . | . | . | . | . | . | . | . | . |
| 21-225_45B8_HC | . | . | . | . | . | . | . | . | . | . | . | . | . | . | L | . | . | . | . | . | . | . | . | . | . |
| 21-225_46B9_HC | . | . | . | . | . | . | . | . | R | Y | S | . | . | W | - | . | . | . | . | . | . | . | . | . | . |
| 21-225_46C3_HC | . | . | . | . | . | . | . | . | . | . | . | . | . | . | L | . | . | . | . | . | . | . | . | . | . |
| 21-225_46D4_HC | . | . | . | . | . | . | . | . | V | P | . | . | . | S | . | . | . | . | . | . | . | . | . | . | . |
| 21-225_47C1_HC | . | . | . | . | . | . | . | . | R | Y | S | R | . | W | - | . | . | . | . | . | . | . | . | . | . |
| 21-225_47E7_HC | . | . | . | . | . | . | . | . | R | Y | S | R | . | W | - | . | . | . | . | . | . | . | . | . | . |
| 21-225_48F2_HC | . | . | . | . | . | . | . | . | . | . | . | . | . | S | . | . | . | . | . | . | . | . | . | . | . |
| 21-225_48H4_HC | . | . | . | . | . | . | . | . | . | N | . | . | . | . | . | . | . | . | . | . | . | . | . | . | . |

EP 4 435 105 A2

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | E | R | Y | S | S | G | W | - | - | - | - | - | - | - | - | - | - |
| 21-225_49C1_HC | . | . | . | . | . | . | . | . | R | Y | S | . | . | W | - | . | . | . | . | . | . | . | . | . | . |
| 21-225_49E7_HC | . | . | L | . | . | . | . | . | R | Y | S | . | . | W | - | . | . | . | . | . | . | . | . | . | . |
| 21-225_49F9_HC | . | . | . | . | H | . | . | . | R | Y | S | . | . | W | - | . | . | . | . | . | . | . | . | . | . |
| 21-225_49G3_HC | . | . | . | . | . | . | . | . | R | Y | S | . | . | W | S | . | . | . | . | . | . | . | . | . | . |
| 21-225_4C12_HC | . | . | . | . | . | . | . | . | . | K | . | . | . | S | . | . | . | . | . | . | . | . | . | . | . |
| 21-225_4C5_HC | . | . | . | . | . | . | . | . | . | K | . | T | . | S | . | . | . | . | . | . | . | . | . | . | . |
| 21-225_50H4_HC | . | . | . | . | . | . | . | . | . | L | . | . | . | N | . | . | . | . | . | . | . | . | . | . | . |
| 21-225_51E1_HC | . | . | . | . | . | . | . | . | R | Y | S | . | . | W | - | . | . | . | . | . | . | . | . | . | . |
| 21-225_52D10_HC | . | . | . | . | . | . | . | . | R | Y | S | . | . | W | - | . | . | . | . | . | . | . | . | . | . |
| 21-225_52G8_HC | . | . | L | . | . | . | . | . | R | Y | S | . | . | W | - | . | . | . | . | . | . | . | . | . | . |
| 21-225_54D4_HC | . | . | . | . | . | . | . | . | R | Y | S | . | . | W | - | . | . | . | . | . | . | . | . | . | . |
| 21-225_55C2_HC | . | . | . | . | . | . | . | . | R | Y | S | . | . | W | - | . | . | . | . | . | . | . | . | . | . |
| 21-225_55F9_HC | . | . | . | . | . | . | . | . | R | Y | S | . | . | W | - | . | . | . | . | . | . | . | . | . | . |
| 21-225_55H1_HC | . | . | . | . | . | . | . | . | R | Y | S | . | . | W | - | . | . | . | . | . | . | . | . | . | . |
| 21-225_58H11_HC | . | . | . | . | . | . | . | . | R | Y | S | . | . | W | - | . | . | . | . | . | . | . | . | . | . |
| 21-225_59A12_HC | . | . | . | . | . | . | . | . | R | Y | S | . | . | W | - | . | . | . | . | . | . | . | . | . | . |
| 21-225_5E5_HC | . | . | . | . | . | . | T | . | . | V | . | . | . | . | . | . | . | . | . | . | . | . | . | . | . |
| 21-225_5E5.003_HC | . | . | . | . | . | . | T | . | . | V | . | . | . | . | . | . | . | . | . | . | . | . | . | . | . |
| 21-225_5E5.004_HC | . | . | . | . | . | . | T | . | . | V | . | . | . | . | . | . | . | . | . | . | . | . | . | . | . |
| 21-225_5E5.005_HC | . | . | . | . | . | . | T | . | . | V | . | . | . | . | . | . | . | . | . | . | . | . | . | . | . |
| 21-225_5E5.006_HC | . | . | . | . | . | . | T | . | . | V | . | . | . | . | . | . | . | . | . | . | . | . | . | . | . |
| 21-225_5E5.011_HC | . | . | . | . | . | . | T | . | . | V | . | . | . | . | . | . | . | . | . | . | . | . | . | . | . |
| 21-225_5E5.012_HC | . | . | . | . | . | . | T | . | . | V | . | . | . | . | . | . | . | . | . | . | . | . | . | . | . |
| 21-225_5E5.015_HC | . | . | . | . | . | . | T | . | . | V | . | . | . | . | Y | . | . | . | . | . | . | . | . | . | . |
| 21-225_5E5.016_HC | . | . | . | . | . | . | T | . | . | V | . | . | . | . | F | . | . | . | . | . | . | . | . | . | . |
| 21-225_5E5.017_HC | . | . | . | . | . | . | T | . | . | V | . | . | . | . | Y | . | . | . | . | . | . | . | . | . | . |
| 21-225_5E5.018_HC | . | . | . | . | . | . | T | . | . | V | . | . | . | . | Y | . | . | . | . | . | . | . | . | . | . |
| 21-225_5E5.019_HC | . | . | . | . | . | . | T | . | . | V | . | . | . | . | F | . | . | . | . | . | . | . | . | . | . |
| 21-225_5E5.023_HC | . | . | . | . | . | . | T | . | . | V | . | . | . | . | Y | . | . | . | . | . | . | . | . | . | . |

3710

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | E | R | Y | S | S | G | W | - | - | - | - | - | - | - | - | - | - |
| 21-225_5E5.024_HC | . | . | . | . | . | . | T | . | . | V | . | . | . | . | Y | . | . | . | . | . | . | . | . | . | . |
| 21-225_60C12_HC | . | . | . | . | . | . | . | . | R | Y | S | R | . | W | - | . | . | . | . | . | . | . | . | . | . |
| 21-225_60G2_HC | . | . | . | . | . | . | . | . | R | Y | S | R | . | W | - | . | . | . | . | . | . | . | . | . | . |
| 21-225_61B3_HC | . | . | . | . | . | . | . | . | R | Y | S | R | . | W | - | . | . | . | . | . | . | . | . | . | . |
| 21-225_61F2_HC | . | . | . | . | . | . | . | . | . | . | . | . | . | . | . | . | . | . | . | . | . | . | . | . | . |
| 21-225_64A4_HC | . | . | . | . | . | . | . | . | . | . | . | . | . | S | . | . | . | . | . | . | . | . | . | . | . |
| 21-225_64C8_HC | . | . | . | . | . | . | . | . | . | . | . | . | . | . | . | . | . | . | . | . | . | . | . | . | . |
| 21-225_66D11_HC | . | . | L | . | . | . | . | . | . | M | . | . | . | N | . | . | . | . | . | . | . | . | . | . | . |
| 21-225_73C9_HC | . | . | . | . | . | . | . | . | . | . | . | . | . | S | . | . | . | . | . | . | . | . | . | . | . |
| 21-225_7C9_HC | . | . | . | . | . | . | . | . | . | K | . | . | . | S | . | . | . | . | . | . | . | . | . | . | . |
| 21-225_7G4_HC | . | . | . | . | . | . | . | . | . | K | . | . | . | N | . | . | . | . | . | . | . | . | . | . | . |
| 21-225_94F3_HC | . | . | . | . | F | . | . | . | . | K | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | Y | D | Y | G | M | D | V | W | G | Q | G | T | T | V | T | V | S | S |
| 21-225_10E9_HC | . | . | . | . | . | . | . | . | . | . | . | . | . | . | . | . | . | . | . | . | . | . | . | . |
| 21-225_147A8_HC | . | . | . | . | . | . | - | T | G | . | . | . | . | . | . | . | . | . | . | . | . | . | . | . |
| 21-225_147B2_HC | . | . | . | . | . | . | - | S | G | . | . | . | . | . | . | . | . | . | . | . | . | . | . | . |
| 21-225_147H5_HC | . | . | . | . | . | . | . | . | . | . | L | . | . | . | . | . | . | . | . | . | . | . | . | . |
| 21-225_148C9_HC | . | . | . | . | . | . | . | . | . | . | L | . | . | . | . | . | . | . | . | . | . | . | . | . |
| 21-225_148F5_HC | . | . | . | . | . | . | - | S | G | . | . | . | . | . | . | . | . | . | . | . | . | . | . | . |
| 21-225_148H10_HC | . | . | . | . | . | . | - | S | G | . | . | . | . | . | . | . | . | . | . | . | . | . | . | . |
| 21-225_149D10_HC | . | . | . | . | . | . | . | . | . | . | . | . | . | . | . | . | . | . | . | . | . | . | . | . |
| 21-225_14E10_HC | . | . | . | . | . | . | . | . | . | . | . | . | . | . | . | . | . | . | . | . | . | . | . | . |
| 21-225_150D11_HC | . | . | . | . | . | . | - | S | G | . | . | . | . | . | . | . | . | . | . | . | . | . | . | . |
| 21-225_150E2_HC | . | . | . | . | . | . | . | G | . | . | . | . | . | . | . | . | . | . | . | . | . | . | . | . |
| 21-225_152F7_HC | . | . | . | . | . | . | . | G | . | . | . | . | . | . | . | . | . | . | . | . | . | . | . | . |
| 21-225_153G9_HC | . | . | . | . | . | . | - | A | G | . | . | . | . | . | . | . | . | . | A | . | . | . | . | . |
| 21-225_154F4_HC | . | . | . | P | Y | Y | . | Y | . | . | . | . | . | . | . | . | . | . | . | . | . | . | . | . |
| 21-225_159D8_HC | . | . | . | . | . | . | . | . | . | . | . | . | . | . | . | . | . | . | . | . | . | . | . | . |
| 21-225_15C11_HC | . | . | . | . | . | . | . | . | . | . | L | . | . | . | . | . | . | . | . | . | . | . | . | . |
| 21-225_169D11_HC | . | . | . | . | . | . | . | . | . | . | . | . | . | . | . | . | . | . | . | . | . | . | . | . |
| 21-225_170F3_HC | . | . | . | . | . | . | . | . | . | . | . | . | . | . | . | . | . | . | . | . | . | . | . | . |
| 21-225_173C11_HC | . | . | . | . | . | . | - | S | G | . | . | . | . | . | . | . | . | . | . | . | . | . | . | . |
| 21-225_174G10_HC | . | . | . | . | . | . | . | . | . | . | . | . | . | . | . | . | . | . | . | . | . | . | . | . |
| 21-225_176H12_HC | . | . | . | . | . | . | . | . | . | . | . | . | . | . | . | . | . | . | . | . | . | . | . | . |
| 21-225_177B4_HC | . | . | . | . | . | . | . | . | . | . | L | . | . | . | . | . | . | . | . | . | . | . | . | . |
| 21-225_179C2_HC | . | . | . | . | . | . | - | S | G | . | . | . | . | . | . | . | . | . | . | . | . | . | . | . |
| 21-225_17H3_HC | . | . | . | . | . | . | . | . | . | . | . | . | . | . | . | . | . | . | . | . | . | . | . | . |
| 21-225_183F4_HC | . | . | . | P | Y | Y | . | Y | . | . | L | G | . | . | . | . | . | . | . | . | . | . | . | . |
| 21-225_190D6_HC | . | . | . | . | . | . | F | G | . | . | . | . | . | . | . | . | . | . | . | . | . | . | . | . |
| 21-225_192A2_HC | . | . | . | . | L | D | . | F | . | . | . | . | . | . | . | . | . | . | . | . | . | . | . | . |
| 21-225_192F6_HC | . | . | . | . | . | . | F | G | . | . | . | . | . | . | . | . | . | . | . | . | . | . | . | . |
| 21-225_193F8_HC | . | . | . | . | L | D | . | Y | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | Y | D | Y | G | M | D | V | W | G | Q | G | T | T | V | T | V | S | S |
| 21-225_194F10_HC | . | . | . | . | . | . | F | G | . | . | . | . | . | . | . | . | . | . | . | . | . | . | . | . |
| 21-225_201C5_HC | . | . | . | . | . | . | . | . | . | . | . | . | . | . | . | . | . | . | . | . | . | . | . | . |
| 21-225_203F7_HC | . | . | . | . | . | . | . | . | . | . | . | . | . | . | . | . | . | . | . | . | . | . | . | . |
| 21-225_204H3_HC | . | . | . | . | . | . | . | . | . | . | . | . | . | S | . | . | . | . | . | . | . | . | . | . |
| 21-225_208B2_HC | . | . | . | . | . | . | . | . | . | . | L | . | . | . | . | . | . | . | . | . | . | . | . | . |
| 21-225_208F1_HC | . | . | . | . | . | . | . | . | . | . | . | . | . | . | . | . | . | . | . | . | . | . | . | . |
| 21-225_20B2_HC | . | . | . | . | . | . | . | . | . | . | . | . | . | . | . | . | . | . | . | . | . | . | . | . |
| 21-225_210G5_HC | . | . | . | . | . | . | . | . | . | . | L | . | . | . | . | . | . | . | . | . | . | . | . | . |
| 21-225_210G6_HC | . | . | . | . | . | . | . | . | . | . | . | . | . | . | . | . | . | . | . | . | . | . | . | . |
| 21-225_212B11_HC | . | . | . | . | . | . | . | . | . | . | . | . | . | . | . | . | . | . | . | . | . | . | . | . |
| 21-225_214G10_HC | . | . | . | . | . | . | . | . | . | . | . | . | . | . | . | . | . | . | . | . | . | . | . | . |
| 21-225_214G12_HC | . | . | . | . | . | . | . | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . |
| 21-225_215E11_HC | . | . | . | . | . | . | . | . | . | . | . | . | . | . | . | . | . | . | . | . | . | . | . | . |
| 21-225_215E3_HC | . | . | . | . | . | . | . | . | . | . | . | . | . | . | . | . | . | . | . | . | . | . | . | . |
| 21-225_216E8_HC | . | . | . | . | . | . | . | . | . | . | . | . | . | . | . | . | . | . | . | . | . | . | . | . |
| 21-225_216H12_HC | . | . | . | . | . | . | . | . | . | . | . | . | . | . | . | . | . | . | . | . | . | . | . | . |
| 21-225_217E5_HC | . | . | . | . | . | . | . | . | . | . | . | . | . | . | . | . | . | . | . | . | . | . | . | . |
| 21-225_217G10_HC | . | . | . | . | . | . | . | . | . | . | . | . | . | . | . | . | . | . | . | . | . | . | . | . |
| 21-225_217H12_HC | . | . | . | . | . | . | . | . | . | . | . | . | . | . | . | . | . | . | . | . | . | . | . | . |
| 21-225_218C4_HC | . | . | . | . | . | . | . | . | . | . | . | . | . | . | . | . | . | . | . | . | . | . | . | . |
| 21-225_220F8_HC | . | . | . | . | . | . | . | . | . | . | . | . | . | . | . | . | . | . | . | . | . | . | . | . |
| 21-225_224E7_HC | . | . | . | . | . | . | . | . | F | . | L | . | . | . | . | . | . | . | . | . | . | . | . | . |
| 21-225_224F1_HC | . | . | . | . | . | . | . | . | . | . | . | . | . | . | . | . | . | . | . | . | . | . | . | . |
| 21-225_224F3_HC | . | . | . | . | . | . | . | . | . | . | . | . | . | . | . | . | . | . | . | . | . | . | . | . |
| 21-225_225A1_HC | . | . | . | . | . | . | . | . | . | . | . | . | . | . | . | . | . | . | . | . | . | . | . | . |
| 21-225_226C6_HC | . | . | . | . | . | . | . | . | . | . | . | . | . | . | . | . | . | . | . | . | . | . | . | . |
| 21-225_226H11_HC | . | . | . | . | . | . | . | . | . | . | L | . | . | . | . | . | . | . | . | . | . | . | . | . |
| 21-225_22F4_HC | . | . | . | . | . | . | . | . | . | . | . | . | . | . | . | . | . | . | . | . | . | . | . | . |

**FIGURE 57**

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | Y | D | Y | G | M | D | V | W | G | Q | G | T | T | V | T | V | S | S |
| 21-225_23A10_HC | . | . | . | . | . | . | H | . | . | . | . | . | . | . | . | . | . | . | . | . | . | . | . | . |
| 21-225_24B6_HC | . | . | . | . | . | . | . | . | . | . | . | . | . | . | . | . | . | . | . | . | . | . | . | . |
| 21-225_25A2_HC | . | . | . | . | . | . | - | S | G | . | . | . | . | . | . | . | . | . | . | . | . | . | . | . |
| 21-225_25D12_HC | . | . | . | . | . | . | . | . | . | . | . | . | . | . | . | . | . | . | . | . | . | . | . | . |
| 21-225_26A11_HC | . | . | . | . | . | . | . | . | . | . | . | . | . | . | . | . | . | . | . | . | . | . | . | . |
| 21-225_26G7_HC | . | . | . | . | . | . | - | S | G | . | . | . | . | . | . | . | . | . | . | . | . | . | . | . |
| 21-225_27E7_HC | . | . | . | P | Y | Y | . | Y | . | . | . | . | . | . | . | . | . | . | . | . | . | . | . | . |
| 21-225_28H2_HC | . | . | . | . | . | . | . | . | . | . | . | . | . | . | . | . | . | . | . | . | . | . | . | . |
| 21-225_29E2_HC | . | . | . | . | . | . | . | . | . | . | . | . | . | . | . | . | . | . | . | . | . | . | . | . |
| 21-225_29H4_HC | . | . | . | . | . | . | - | S | G | . | . | . | . | . | . | . | . | . | . | . | . | . | . | . |
| 21-225_2G4_HC | . | . | . | . | . | . | . | . | . | . | . | . | . | . | . | H | . | . | . | . | . | . | . | . |
| 21-225_32H2_HC | . | . | . | . | . | . | - | T | G | . | . | . | . | . | . | . | . | . | . | . | . | . | . | . |
| 21-225_3H10_HC | . | . | . | . | . | . | . | . | . | . | L | . | . | . | . | . | . | . | . | . | . | . | . | . |
| 21-225_43E11_HC | . | . | . | . | . | . | - | V | G | . | . | . | . | . | . | . | . | . | . | . | . | . | . | . |
| 21-225_44B10_HC | . | . | . | . | . | . | - | V | G | . | . | . | . | . | . | . | . | . | . | . | . | . | . | . |
| 21-225_44B3_HC | . | . | . | . | . | . | . | . | . | . | . | . | . | . | . | . | . | . | . | . | . | . | . | . |
| 21-225_44C10_HC | . | . | . | . | . | . | . | . | . | . | . | . | . | . | . | . | . | . | . | . | . | . | . | . |
| 21-225_44D3_HC | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . |
| 21-225_44F9_HC | . | . | . | . | . | . | . | . | . | . | . | . | . | G | . | . | . | . | . | . | . | . | . | . |
| 21-225_45B4_HC | . | . | . | . | . | . | . | . | . | . | . | . | . | . | . | . | . | . | . | . | . | . | . | . |
| 21-225_45B8_HC | . | . | . | . | . | . | . | . | . | . | . | . | . | . | . | . | . | . | . | . | . | . | . | . |
| 21-225_46B9_HC | . | . | . | . | . | . | - | M | G | . | . | . | . | . | . | . | . | . | . | . | . | . | . | . |
| 21-225_46C3_HC | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . |
| 21-225_47C1_HC | . | . | . | . | . | . | - | V | G | . | . | . | . | . | . | . | . | . | . | . | . | . | . | . |
| 21-225_47E7_HC | . | . | . | . | . | . | - | V | G | . | . | . | . | . | . | . | . | . | . | . | . | . | . | . |
| 21-225_48F2_HC | . | . | . | . | . | . | . | . | . | . | . | L | . | . | . | . | . | . | . | . | . | . | . | . |
| 21-225_48H4_HC | . | . | . | . | . | . | . | G | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | | H_FR4 | | | | | |
| CONSENSUS | - | - | - | - | - | - | Y | D | Y | G | M | D | V | W | G | Q | G | T | T | V | T | V | S | S |
| 21-225_49C1_HC | . | . | . | . | . | . | - | S | G | . | . | . | . | . | . | . | . | . | . | . | . | . | . | . |
| 21-225_49E7_HC | . | . | . | . | . | . | - | S | G | . | . | . | . | . | . | . | . | . | . | . | . | . | . | . |
| 21-225_49F9_HC | . | . | . | . | . | . | - | S | G | . | . | . | . | . | . | . | . | . | . | . | . | . | . | . |
| 21-225_49G3_HC | . | . | . | . | . | . | - | S | G | . | . | . | . | . | . | . | . | . | . | . | . | . | . | . |
| 21-225_4C12_HC | . | . | . | . | . | . | . | . | . | . | . | . | . | . | . | H | . | . | . | . | . | . | . | . |
| 21-225_4C5_HC | . | . | . | . | . | . | . | . | . | . | . | . | . | . | . | . | . | . | . | . | . | . | . | . |
| 21-225_50H4_HC | . | . | . | . | . | . | . | . | . | . | . | . | . | . | . | . | . | . | . | . | . | . | . | . |
| 21-225_51E1_HC | . | . | . | . | . | . | - | S | G | . | . | . | . | . | . | . | . | . | . | . | . | . | . | . |
| 21-225_52D10_HC | . | . | . | . | . | . | - | S | G | . | . | . | . | . | . | . | . | . | . | . | . | . | . | . |
| 21-225_52G8_HC | . | . | . | . | . | . | - | S | G | . | . | . | . | . | . | . | . | . | . | . | . | . | . | . |
| 21-225_54D4_HC | . | . | . | . | . | . | - | S | G | . | . | . | . | . | . | . | . | . | . | . | . | . | . | . |
| 21-225_55C2_HC | . | . | . | . | . | . | - | S | G | . | . | . | . | . | . | . | . | . | . | . | . | . | . | . |
| 21-225_55F9_HC | . | . | . | . | . | . | - | S | G | . | . | . | . | . | . | . | . | . | . | . | . | . | . | . |
| 21-225_55H1_HC | . | . | . | . | . | . | - | S | G | . | . | . | . | . | . | . | . | . | . | . | . | . | . | . |
| 21-225_58H11_HC | . | . | . | . | . | . | - | . | G | . | . | . | . | . | . | . | . | . | . | . | . | . | . | . |
| 21-225_59A12_HC | . | . | . | . | . | . | - | . | G | . | . | . | . | . | . | . | . | . | . | . | . | . | . | . |
| 21-225_5E5_HC | . | . | . | . | . | . | . | . | . | . | . | . | . | . | . | . | . | . | . | . | . | . | . | . |
| 21-225_5E5.003_HC | . | . | . | . | . | . | . | . | . | . | . | . | . | . | . | . | . | . | . | . | . | . | . | . |
| 21-225_5E5.004_HC | . | . | . | . | . | . | . | . | . | . | . | . | . | . | . | . | . | . | . | . | . | . | . | . |
| 21-225_5E5.005_HC | . | . | . | . | . | . | . | . | . | . | . | . | . | . | . | . | . | . | . | . | . | . | . | . |
| 21-225_5E5.006_HC | . | . | . | . | . | . | . | . | . | . | . | . | . | . | . | . | . | . | . | . | . | . | . | . |
| 21-225_5E5.011_HC | . | . | . | . | . | . | . | . | . | . | . | . | . | . | . | . | . | . | . | . | . | . | . | . |
| 21-225_5E5.012_HC | . | . | . | . | . | . | . | . | . | . | . | . | . | . | . | . | . | . | . | . | . | . | . | . |
| 21-225_5E5.015_HC | . | . | . | . | . | . | . | . | . | . | . | . | . | . | . | . | . | . | . | . | . | . | . | . |
| 21-225_5E5.016_HC | . | . | . | . | . | . | . | . | . | . | . | . | . | . | . | . | . | . | . | . | . | . | . | . |
| 21-225_5E5.017_HC | . | . | . | . | . | . | . | . | . | . | . | . | . | . | . | . | . | . | . | . | . | . | . | . |
| 21-225_5E5.018_HC | . | . | . | . | . | . | . | . | . | . | . | . | . | . | . | . | . | . | . | . | . | . | . | . |
| 21-225_5E5.019_HC | . | . | . | . | . | . | . | . | . | . | . | . | . | . | . | . | . | . | . | . | . | . | . | . |
| 21-225_5E5.023_HC | . | . | . | . | . | . | . | . | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
|  |  |  |  |  |  |  |  |  |  |  |  |  |  |  |  |  |  |  | H_FR4 |  |  |  |  |  |
| CONSENSUS | - | - | - | - | - | - | Y | D | Y | G | M | D | V | W | G | Q | G | T | T | V | T | V | S | S |
| 21-225_5E5.024_HC | . | . | . | . | . | . | . | . | . | . | . | . | . | . | . | . | . | . | . | . | . | . | . | . |
| 21-225_60C12_HC | . | . | . | . | . | . | - | T | G | . | . | . | . | . | . | . | . | . | . | . | . | . | . | . |
| 21-225_60G2_HC | . | . | . | . | . | . | - | T | G | . | . | . | . | . | . | . | . | . | . | . | . | . | . | . |
| 21-225_61B3_HC | . | . | . | . | . | . | - | A | G | . | . | . | . | . | . | . | . | . | . | . | . | . | . | . |
| 21-225_61F2_HC | . | . | . | . | . | . | . | . | . | . | . | . | . | . | . | . | . | . | . | . | . | . | . | . |
| 21-225_64A4_HC | . | . | . | . | . | . | . | . | . | . | . | . | . | . | . | . | . | . | . | . | . | . | . | . |
| 21-225_64C8_HC | . | . | . | . | . | . | . | . | . | . | . | . | . | . | . | . | . | . | . | . | . | . | . | . |
| 21-225_66D11_HC | . | . | . | . | . | . | . | . | . | . | L | . | . | . | . | . | . | . | . | . | . | . | . | . |
| 21-225_73C9_HC | . | . | . | . | . | . | F | . | . | . | . | . | . | . | . | . | . | . | . | . | . | . | . | . |
| 21-225_7C9_HC | . | . | . | . | . | . | . | . | . | . | . | . | . | . | . | . | . | . | . | . | . | . | . | . |
| 21-225_7G4_HC | . | . | . | . | . | . | . | . | . | . | . | . | . | . | . | . | . | . | . | . | . | . | . | . |
| 21-225_94F3_HC | . | . | . | . | . | . | . | . | . | . | . | . | . | . | . | . | . | . | . | . | . | . | . | . |

FIGURE 57

Table 51.       Consensus 17- VH3|3-33/D7|7-27|RF2/JH4 (SEQ ID NO: 50268):

QVQLVES-GGGVVQPGRSLRLSCAASG-FTFS<u>D</u>-----<u>YGMH</u>WVRQAPGKGLEWVA<u>VIWYD</u>---
<u>ENNKYYADSVKG</u>RFTISRDNSKNTLYLQMNSLRAEDTAVYYCAR<u>ELGF</u>---------------------- <u>SDY</u>WGQGTLVTVSS wherein:

D at position 33 can be substituted with S, N or T

Y at position 39 can be substituted with F

M at position 41 can be substituted with I

V at position 57 can be substituted with L

I at position 58 can be substituted with V, T or M

Y at position 60 can be substituted with F or D

D at position 61 can be substituted with E, A, G or N

E at position 65 can be substituted with G, V, R or D

N at position 66 can be substituted with S, T, D, I or Y

N at position 67 can be substituted with H or K

K at position 68 can be substituted with Q, E, N or R

Y at position 69 can be substituted with H, K, D, R or S

Y at position 70 can be substituted with H

A at position 71 can be substituted with V, G, T, I or E

D at position 72 can be substituted with E

V at position 74 can be substituted with M

K at position 75 can be substituted with R

FIGURE 57

E at position 109 can be substituted with D or G

G at position 111 can be substituted with A

F at position 112 can be substituted with W or M

L at position 135 can be substituted with R, T, Y, S, Q, I, A, E or N

S at position 136 can be substituted with E, G, D, F, N, A or T

D at position 137 can be substituted with E

Y at position 138 can be substituted with S, F or C

Xaa Xaa Gly Xaa His (SEQ ID NO: 50007)

Xaa Xaa Trp Xaa Xaa Xaa Xaa Xaa Xaa Xaa Xaa Xaa Xaa Ser Xaa Xaa Gly (SEQ ID NO: 50008)

Xaa Leu Xaa Xaa Xaa Xaa Xaa Xaa (SEQ ID NO: 50009)

Asp Tyr Gly Met His (SEQ ID NO: 50474)

Val Ile Trp Tyr Asp Glu Asn Asn Lys Tyr Tyr Ala Asp Ser Val Lys Gly (SEQ ID NO: 50475)

Glu Leu Gly Phe Ser Asp Tyr (SEQ ID NO: 50476)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_10G5_HC | . | . | . | . | . | . | . | . | . | . | . | . | . | . | . | . | . | . | . | . | . | . | . | . | . | . |
| 21-225_10H12_HC | . | . | . | . | . | . | . | . | . | . | . | . | . | . | . | . | . | . | . | . | . | . | . | . | . | . |
| 21-225_12A1_HC | . | . | . | . | . | . | . | . | . | . | . | . | . | . | . | . | . | . | . | . | . | . | . | . | . | . |
| 21-225_12B12_HC | . | . | . | . | . | . | . | . | . | . | . | . | . | . | . | . | . | . | . | . | . | . | . | . | . | . |
| 21-225_12E6_HC | . | . | . | . | . | . | . | . | . | . | . | . | . | . | . | . | . | . | . | . | . | . | . | . | . | . |
| 21-225_12F2_HC | . | . | . | . | . | . | . | . | . | . | . | . | . | . | . | . | . | . | . | . | . | . | . | . | . | . |
| 21-225_13E6_HC | . | . | . | . | . | . | . | . | . | . | . | . | . | . | . | . | . | . | . | . | . | . | . | . | . | . |
| 21-225_146H9_HC | . | . | . | . | . | . | . | . | . | . | . | . | . | . | . | . | . | . | . | . | . | . | . | . | . | . |
| 21-225_14E3_HC | . | . | . | . | . | . | . | . | . | . | . | . | . | . | . | . | . | . | . | . | . | . | . | . | . | . |
| 21-225_157C3_HC | . | . | . | . | . | . | . | . | . | . | . | . | . | . | . | . | . | . | . | . | . | . | . | . | . | . |
| 21-225_157H8_HC | . | . | . | . | . | . | . | . | . | . | . | . | . | . | . | . | . | . | . | . | . | . | . | . | . | . |
| 21-225_158H6_HC | . | . | . | . | . | . | . | . | . | . | . | . | . | . | . | . | . | . | . | . | . | . | . | . | V | . |
| 21-225_159C5_HC | . | . | . | . | . | . | . | . | . | . | . | . | . | . | . | . | . | . | . | . | . | . | . | . | . | . |
| 21-225_15E3_HC | . | . | . | . | . | . | . | . | . | . | . | T | . | . | . | . | . | . | . | . | . | . | . | . | . | . |
| 21-225_16A5_HC | . | . | . | . | . | . | . | . | . | . | . | . | . | . | . | . | . | . | . | . | . | . | . | . | . | . |
| 21-225_16B7_HC | . | . | . | . | . | . | . | . | . | . | . | . | . | . | . | . | . | . | . | . | . | . | . | . | . | . |
| 21-225_16F10_HC | . | . | . | . | . | . | . | . | . | . | . | . | . | . | . | . | . | . | . | . | . | . | . | . | . | . |
| 21-225_16F11_HC | . | . | . | M | . | . | . | . | . | . | . | . | . | . | . | . | . | . | . | . | . | . | . | E | . | . |
| 21-225_16F4_HC | . | . | . | . | . | . | . | . | . | . | . | . | . | . | . | . | . | . | . | . | . | . | . | . | . | . |
| 21-225_16F6_HC | . | . | . | . | . | . | . | . | . | . | . | . | . | . | . | . | . | . | . | . | . | . | . | . | . | . |
| 21-225_16G7_HC | . | . | . | . | . | . | . | . | . | . | . | . | . | . | . | . | . | . | . | . | . | . | . | . | . | . |
| 21-225_17A10_HC | . | . | . | . | . | . | . | . | . | . | . | . | . | . | . | . | . | . | . | . | . | . | . | . | . | . |
| 21-225_17B5_HC | . | . | . | . | . | . | . | . | . | . | . | . | . | . | . | . | . | . | . | . | . | . | . | . | . | . |
| 21-225_17C7_HC | . | . | . | . | . | . | . | . | . | . | . | . | . | . | . | K | . | . | . | . | . | . | . | . | . | . |
| 21-225_17E5_HC | . | . | . | . | . | . | . | . | . | . | . | . | . | . | . | . | . | . | . | . | . | . | . | . | . | . |
| 21-225_18A1_HC | . | . | . | . | . | . | . | . | . | . | . | . | . | . | . | . | . | . | . | . | . | . | . | . | V | . |
| 21-225_18A5_HC | . | . | . | . | . | . | . | . | . | . | . | . | . | . | . | . | . | . | . | . | . | . | Y | T | . | . |
| 21-225_18C2_HC | . | . | . | . | . | . | . | . | . | . | . | . | . | . | . | . | . | . | . | . | . | . | . | V | . | . |
| 21-225_18E8_HC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A | S |
| 21-225_18F2_HC | . | . | . | . | . | . | . | . | . | . | . | . | . | . | . | . | . | . | . | . | . | . | . | . | . | . |
| 21-225_19E1_HC | . | . | . | . | . | . | . | . | . | . | . | . | . | . | . | . | . | . | . | . | . | . | . | . | . | . |
| 21-225_1C12_HC | . | . | . | . | . | . | . | . | . | . | . | . | . | . | . | . | . | . | . | . | . | . | . | . | . | . |
| 21-225_203B9_HC | . | . | . | . | . | . | . | . | . | . | . | . | . | . | . | . | . | . | . | . | . | . | . | . | . | . |
| 21-225_207F12_HC | . | . | . | . | . | . | . | . | . | . | . | . | . | . | . | . | . | . | . | . | . | . | . | . | . | . |
| 21-225_20A7_HC | . | . | . | . | . | . | . | . | . | . | . | . | . | . | . | . | . | . | . | . | . | . | . | . | . | . |
| 21-225_20D10_HC | . | . | . | . | . | . | . | . | . | . | . | . | . | . | . | . | . | . | . | . | . | . | . | . | . | . |
| 21-225_20D5_HC | . | . | . | . | . | . | . | . | . | . | . | . | . | . | . | . | . | . | . | . | . | . | . | . | . | . |
| 21-225_20E12_HC | . | . | . | . | . | . | . | . | . | . | . | . | . | . | . | . | . | . | . | . | . | . | . | . | . | . |
| 21-225_20E5_HC | . | . | . | . | . | . | . | . | . | . | . | . | . | . | . | . | . | . | . | . | . | . | . | . | . | . |
| 21-225_20H12_HC | . | . | . | . | . | . | . | . | . | . | . | . | . | . | . | . | . | . | . | . | . | . | . | . | . | . |
| 21-225_21E5_HC | . | . | . | . | . | . | . | . | . | . | . | . | . | . | . | . | . | . | . | . | . | . | . | S | . | . |
| 21-225_21F7_HC | . | . | . | . | . | . | . | . | . | . | . | . | . | . | . | . | . | . | . | . | . | . | . | . | . | . |
| 21-225_21G11_HC | . | . | . | . | . | . | . | . | . | . | . | . | . | . | . | . | . | . | . | . | . | . | . | . | . | . |
| 21-225_21G2_HC | . | . | . | . | . | . | . | . | . | . | . | . | . | . | . | . | . | . | . | . | . | . | . | . | . | . |
| 21-225_21H8_HC | . | . | . | . | . | . | . | . | . | . | . | . | . | . | S | . | . | . | . | . | . | . | . | . | . | . |
| 21-225_224D10_HC | . | . | . | . | . | . | . | . | . | . | . | L | . | . | . | . | K | . | . | . | . | . | . | . | . | . |
| 21-225_22A4_HC | . | . | . | . | . | . | . | . | . | . | . | . | . | . | . | . | . | . | . | . | . | . | . | . | . | . |
| 21-225_22F2_HC | . | . | . | . | . | . | . | . | . | . | . | . | . | . | . | . | . | . | . | . | . | . | . | . | . | . |
| 21-225_22F9_HC | . | . | . | M | . | . | . | . | . | . | . | . | . | . | . | . | . | . | . | . | . | . | . | . | . | . |
| 21-225_22H8_HC | . | . | . | . | . | . | . | . | . | . | . | . | . | . | . | . | . | . | . | . | . | . | . | . | . | . |
| 21-225_23F12_HC | . | . | . | . | . | . | . | . | . | . | . | . | . | . | . | . | . | . | . | . | . | . | . | V | . | . |
| 21-225_23H11_HC | . | E | . | . | . | . | . | . | . | . | . | . | . | . | . | . | . | . | . | . | . | . | . | . | . | . |
| 21-225_25B6_HC | . | . | . | . | . | . | . | . | . | . | . | . | . | . | . | . | . | . | . | . | V | . | . | T | . | . |
| 21-225_25E6_HC | . | . | . | . | . | . | . | . | . | . | . | . | . | . | . | . | . | . | . | . | . | . | . | . | . | . |
| 21-225_25H10_HC | . | . | . | . | . | . | . | . | . | . | . | . | . | . | . | . | . | . | . | . | . | . | . | . | . | . |
| 21-225_25H11_HC | . | . | . | . | . | . | . | . | . | . | . | . | . | . | . | . | . | . | . | . | . | . | . | . | . | . |
| 21-225_26A2_HC | . | . | . | . | . | . | . | . | . | . | . | . | . | . | . | . | . | . | . | . | . | . | . | . | . | . |
| 21-225_27C3_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
|  |  |  |  |  |  |  |  |  |  |  |  |  |  |  |  | H_FR1 |  |  |  |  |  |  |  |  |  |  |
| CONSENSUS | Q | V | Q | L | V | E | S | - | G | G | G | V | V | Q | P | G | R | S | L | R | L | S | C | A | A | S |
| 21-225_27D5_HC | . | . | . | . | . | . | . | . | . | . | . | . | . | . | . | . | . | . | . | . | . | . | . | . | . | . |
| 21-225_27F2_HC | . | E | . | . | . | . | . | . | . | . | . | . | . | . | . | . | . | . | . | . | . | . | . | . | . | . |
| 21-225_28C7_HC | . | . | . | . | . | . | . | . | . | . | . | . | . | . | . | . | . | . | . | . | . | . | . | . | . | . |
| 21-225_28F5_HC | . | . | . | . | . | . | . | . | . | . | . | . | . | . | . | . | . | . | . | . | . | . | . | . | . | . |
| 21-225_29B8_HC | . | . | . | . | . | . | . | . | . | . | . | . | . | . | . | . | . | . | . | . | . | . | . | . | . | . |
| 21-225_29D7_HC | . | . | . | . | . | . | . | . | . | . | . | . | . | . | . | . | . | . | . | . | . | . | . | . | . | . |
| 21-225_29G4_HC | . | . | . | . | . | . | . | . | . | . | . | . | . | . | . | . | . | . | . | . | . | T | . | . | . | . |
| 21-225_29G8_HC | . | . | . | . | . | . | . | . | . | . | . | . | . | . | . | . | . | . | . | . | . | . | . | . | . | . |
| 21-225_29H6_HC | . | . | . | . | . | . | . | . | . | . | . | . | . | . | . | . | . | . | . | . | . | . | . | . | . | . |
| 21-225_2B2_HC | . | . | . | . | . | . | . | . | . | . | . | . | . | . | . | . | . | . | . | . | . | . | . | . | . | . |
| 21-225_43C3_HC | . | . | . | . | . | . | . | . | . | . | . | . | . | . | . | . | . | . | . | . | . | . | . | . | . | . |
| 21-225_44C3_HC | . | . | . | . | . | . | . | . | . | . | . | . | . | . | . | . | . | . | . | . | . | . | . | . | . | . |
| 21-225_44C8_HC | . | . | . | . | . | . | . | . | . | . | . | . | . | . | . | . | . | . | . | . | . | . | . | . | . | . |
| 21-225_46C6_HC | . | . | . | . | . | . | . | . | . | . | . | . | . | . | . | . | . | . | . | . | . | . | . | . | . | . |
| 21-225_46D8_HC | . | . | . | . | . | . | . | . | . | . | . | . | . | . | . | . | . | . | . | . | . | . | . | . | . | . |
| 21-225_46F3_HC | . | . | . | . | . | . | . | . | . | . | . | . | . | . | . | . | . | . | . | . | . | . | . | . | . | . |
| 21-225_47A1_HC | . | . | . | . | . | . | . | . | . | . | . | . | . | . | . | . | . | . | . | . | . | . | . | . | . | . |
| 21-225_48A9_HC | . | . | . | . | . | . | . | . | . | . | . | . | . | . | . | . | . | . | . | . | . | . | . | . | . | . |
| 21-225_48C1_HC | . | . | . | . | . | . | . | . | . | . | . | . | . | . | . | . | . | . | . | . | . | . | . | . | . | . |
| 21-225_48D12_HC | . | . | . | . | . | . | . | . | . | . | . | . | . | . | . | . | . | . | . | . | . | . | . | . | . | . |
| 21-225_49A1_HC | . | . | . | . | . | . | . | . | . | . | . | . | . | . | . | . | . | . | . | . | . | . | . | . | . | . |
| 21-225_49C6_HC | . | . | . | . | . | . | . | . | . | . | . | . | . | . | . | . | . | . | . | . | . | . | . | . | . | . |
| 21-225_51B11_HC | . | . | . | . | . | . | . | . | . | . | . | . | . | . | . | . | . | . | . | . | . | . | . | . | . | . |
| 21-225_51E4_HC | . | . | . | . | . | . | . | . | . | . | . | . | . | . | . | . | . | . | . | . | . | . | . | . | . | . |
| 21-225_51F11_HC | . | . | . | . | . | . | . | . | . | . | . | . | . | . | . | . | . | . | . | . | . | . | . | . | . | . |
| 21-225_51F9_HC | . | . | . | . | . | . | . | . | . | . | . | . | . | . | . | . | . | . | . | . | . | . | . | . | . | . |
| 21-225_52B2_HC | . | . | . | . | . | . | . | . | . | . | . | . | . | . | . | . | . | . | . | . | . | . | . | . | . | . |
| 21-225_52B9_HC | . | . | . | . | . | . | . | . | . | . | . | . | . | . | . | . | . | . | . | . | . | . | . | . | . | . |
| 21-225_53B12_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_53D2_HC | . | . | . | . | . | . | . | . | . | . | . | . | . | . | . | . | . | . | . | . | . | . | . | . | . | . |
| 21-225_53E6_HC | . | . | . | . | . | . | . | . | . | . | . | . | . | . | . | . | . | . | . | . | . | . | . | T | T | . |
| 21-225_54C6_HC | . | . | . | . | . | . | . | . | . | . | . | . | . | . | . | . | . | . | . | . | . | . | . | T | T | . |
| 21-225_54D3_HC | . | . | . | . | . | . | . | . | . | . | . | . | . | . | . | . | . | . | . | . | . | . | . | . | . | . |
| 21-225_54G7_HC | . | . | . | . | . | . | . | . | . | . | . | . | . | . | . | . | . | . | . | . | . | . | . | . | . | . |
| 21-225_55B3_HC | . | . | . | . | . | . | . | . | . | . | . | . | . | . | . | . | . | . | . | . | . | . | . | . | . | . |
| 21-225_57A4_HC | . | . | . | . | . | . | . | . | . | . | . | . | . | . | . | . | . | . | . | . | . | . | . | V | . | . |
| 21-225_58F5_HC | . | . | . | . | . | . | . | . | . | . | . | . | . | . | . | . | . | . | . | . | . | . | . | V | . | . |
| 21-225_59F11_HC | . | . | . | . | . | . | . | . | . | . | . | . | . | . | . | . | . | . | . | . | . | . | . | . | . | . |
| 21-225_60A3_HC | . | . | . | . | . | . | . | . | . | . | . | . | . | . | . | . | . | . | . | . | . | . | . | . | . | . |
| 21-225_62E4_HC | . | . | . | . | . | . | . | . | . | . | . | . | . | . | . | . | . | . | . | . | . | . | . | . | . | . |
| 21-225_64E1_HC | . | . | . | . | . | . | . | . | . | . | . | . | . | . | . | . | . | . | . | . | . | . | . | V | . | . |
| 21-225_65C1_HC | . | . | . | . | . | . | . | . | . | . | . | . | . | . | . | . | . | . | . | . | . | . | . | . | . | . |
| 21-225_66C7_HC | . | . | . | . | . | . | . | . | . | . | . | . | . | . | . | . | . | . | . | . | . | . | . | V | . | . |
| 21-225_68F11_HC | . | . | . | . | . | . | . | . | . | . | . | . | . | . | . | . | . | . | . | . | . | . | . | . | . | . |
| 21-225_6A11_HC | . | . | . | . | . | . | . | . | . | . | . | . | . | . | . | . | . | . | . | . | . | . | . | . | . | . |
| 21-225_6G12_HC | . | . | . | . | . | . | . | . | . | . | . | . | . | . | . | . | . | . | . | . | . | . | . | . | . | . |
| 21-225_6G2_HC | L | . | . | . | . | . | . | . | . | . | . | . | . | . | . | . | K | . | . | . | . | . | . | . | . | . |
| 21-225_71A2_HC | . | . | . | . | . | . | . | . | . | . | . | . | . | . | . | . | . | . | . | . | . | . | . | . | T | . |
| 21-225_71B11_HC | . | . | . | . | . | . | . | . | . | . | . | . | . | . | . | . | . | . | . | . | . | . | . | . | . | . |
| 21-225_71B6_HC | . | . | . | . | . | . | . | . | . | . | . | . | . | . | . | . | . | . | . | . | . | . | . | . | . | . |
| 21-225_72G12_HC | . | . | . | . | . | . | . | . | . | . | . | . | . | . | . | . | . | . | . | . | . | . | . | . | . | . |
| 21-225_7C4_HC | . | . | . | . | . | . | . | . | . | . | . | . | . | . | . | . | K | . | . | . | . | . | . | . | . | . |
| 21-225_7E9_HC | . | . | . | . | . | . | . | . | . | . | . | . | . | . | . | . | . | . | . | . | . | . | . | . | . | . |
| 21-225_7G2_HC | . | . | . | . | . | . | . | . | . | . | . | . | . | . | . | . | . | . | . | . | . | . | . | . | . | . |
| 21-225_7H11_HC | . | . | . | . | . | . | . | . | . | . | . | . | . | . | . | . | . | . | . | . | . | . | . | S | . | . |
| 21-225_8C9_HC | . | . | . | . | . | . | . | . | . | . | . | . | . | . | . | . | . | . | . | . | . | . | . | . | . | . |
| 21-225_8H7_HC | . | . | . | . | . | . | . | . | . | . | . | . | . | . | . | . | . | . | . | . | . | . | . | P | . | . |
| 21-225_9A1_HC | . | . | . | V | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_9E8_HC | . | . | . | . | . | . | . | . | . | . | . | . | . | . | . | . | . | . | . | . | . | . | . | E | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_10G5_HC | . | . | . | . | . | . | N | . | . | . | . | . | . | . | . | . | . | . | . | . | . | . | . | . | . | . |
| 21-225_10H12_HC | . | . | . | . | . | . | . | . | . | . | . | . | . | . | . | . | . | . | . | . | . | . | . | . | . | . |
| 21-225_12A1_HC | . | . | . | . | . | . | S | . | . | . | . | . | . | . | . | . | . | . | . | . | . | . | . | . | . | . |
| 21-225_12B12_HC | . | . | . | . | . | . | . | . | . | . | . | . | . | . | . | . | . | . | . | . | . | . | . | . | . | . |
| 21-225_12E6_HC | . | . | . | . | . | . | . | . | . | . | . | . | . | . | . | . | . | . | . | . | . | . | . | . | . | . |
| 21-225_12F2_HC | . | . | . | . | . | . | . | . | . | . | . | . | . | . | . | . | . | . | . | . | . | . | . | . | . | . |
| 21-225_13E6_HC | . | . | . | . | . | . | N | . | . | . | . | . | . | . | . | . | . | . | . | . | . | . | . | . | . | . |
| 21-225_146H9_HC | . | . | S | . | . | . | S | . | . | . | . | . | . | . | . | . | . | . | . | . | . | . | . | . | . | . |
| 21-225_14E3_HC | . | . | . | . | . | . | . | . | . | . | . | . | F | . | . | . | . | . | . | . | . | . | . | . | . | . |
| 21-225_157C3_HC | . | . | . | . | . | . | T | . | . | . | . | . | . | . | . | . | . | . | . | . | . | . | . | . | . | . |
| 21-225_157H8_HC | . | . | . | . | . | . | S | . | . | . | . | . | . | . | . | . | . | . | . | . | . | . | . | . | . | . |
| 21-225_158H6_HC | . | . | . | . | . | . | S | . | . | . | . | . | . | . | . | . | . | . | . | . | . | . | . | . | . | . |
| 21-225_159C5_HC | . | . | . | . | . | . | S | . | . | . | . | . | . | . | I | . | . | . | . | . | . | . | . | . | . | . |
| 21-225_15E3_HC | . | . | . | . | . | . | S | . | . | . | . | . | . | . | . | . | . | . | . | . | . | . | . | . | . | . |
| 21-225_16A5_HC | . | . | . | . | . | . | . | . | . | . | . | . | . | . | . | . | . | . | . | . | . | . | . | . | . | . |
| 21-225_16B7_HC | . | . | . | . | . | . | S | . | . | . | . | . | . | . | . | . | . | . | . | . | . | . | . | . | . | . |
| 21-225_16F10_HC | . | . | . | . | . | . | . | . | . | . | . | . | . | . | . | . | . | . | . | . | . | . | . | . | . | . |
| 21-225_16F11_HC | . | . | . | . | . | . | S | . | . | . | . | . | . | . | . | . | . | . | . | . | . | . | . | . | . | . |
| 21-225_16F4_HC | . | . | . | . | . | . | N | . | . | . | . | . | . | . | . | . | . | . | . | . | . | . | . | . | . | . |
| 21-225_16F6_HC | . | . | . | . | . | . | N | . | . | . | . | . | . | . | . | . | R | . | . | . | . | . | . | . | . | . |
| 21-225_16G7_HC | . | . | . | . | . | T | . | . | . | . | . | . | . | . | . | . | . | . | . | . | . | . | . | . | . | . |
| 21-225_17A10_HC | . | . | . | . | . | . | N | . | . | . | . | . | . | . | . | . | . | . | . | . | . | . | A | . | . | . |
| 21-225_17B5_HC | . | . | . | . | . | . | . | . | . | . | . | . | . | . | . | . | . | . | . | . | . | . | . | . | . | . |
| 21-225_17C7_HC | . | . | . | . | . | . | . | . | . | . | . | . | . | . | . | . | . | . | . | . | . | . | . | . | . | . |
| 21-225_17E5_HC | . | . | . | . | . | . | . | . | . | . | . | . | . | . | . | . | . | . | . | . | . | . | . | . | . | . |
| 21-225_18A1_HC | . | . | . | . | . | . | S | . | . | . | . | . | . | . | . | . | . | . | . | . | . | . | . | . | . | . |
| 21-225_18A5_HC | . | . | . | . | . | . | . | . | . | . | . | . | . | . | . | . | . | . | . | . | . | . | . | . | . | . |
| 21-225_18C2_HC | . | . | . | . | . | . | . | . | . | . | . | . | . | . | . | . | . | . | . | . | . | . | . | . | . | . |
| 21-225_18E8_HC | . | . | . | . | . | . | N | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_18F2_HC | | | | | | | | | | | | | | | | | | | | | | | | | | |
| 21-225_19E1_HC | | | | | | | N | | | | | | | | | | | | | | | | | | | |
| 21-225_1C12_HC | | | | | | | N | | | | | | | | | | | | | | | | | | | |
| 21-225_203B9_HC | | | | | L | | S | | | | | | F | | | | | | | | | | | | | |
| 21-225_207F12_HC | | | | | | | N | | | | | | | | | | | | | | | | | | | |
| 21-225_20A7_HC | | | | | | | N | | | | | | | | | | | | | | | | | | | |
| 21-225_20D10_HC | | | | | | | | | | | | | | | | | | | | | | | | | | |
| 21-225_20D5_HC | | | | | | | S | | | | | | | | | | | | | | | | | | | |
| 21-225_20E12_HC | | | | | | | S | | | | | | | | | | | | | | | | | | | |
| 21-225_20E5_HC | | | | | | | | | | | | | | | | | | | | | | | | | | |
| 21-225_20H12_HC | | | | | | | N | | | | | | | | | | | | | | | | | | | |
| 21-225_21E5_HC | | | | | | | | | | | | | | | | | | | | | | | | | | |
| 21-225_21F7_HC | | | | | | | | | | | | | | | | | | | | | | | | | | |
| 21-225_21G11_HC | | | | | | | | | | | | | | | | | | | | | | | | | | |
| 21-225_21G2_HC | | | | | | | | | | | | | | | | | | | | | | | | | | |
| 21-225_21H8_HC | | | | | | | | | | | | | | | | | | | | | | | | | | |
| 21-225_224D10_HC | | | | | | | S | | | | | | | | | | | | | | | | | | | |
| 21-225_22A4_HC | | | | | | | | | | | | | | | l | | | | | | | | | | | |
| 21-225_22F2_HC | | | | | | | | | | | | | | | | | | | | | | | | | | |
| 21-225_22F9_HC | | | | | | | S | | | | | | | | | | | | | | | | | | | |
| 21-225_22H8_HC | | | | | | | S | | | | | | | | | | | | | | | | | | | |
| 21-225_23F12_HC | | | | | | | S | | | | | | | | | | | | | | | | | | | |
| 21-225_23H11_HC | | | | | | | S | | | | | | | | | | | | | | | | | | | |
| 21-225_25B6_HC | | | | | L | R | N | | | | | | | | | | | | | | | | | | | |
| 21-225_25E6_HC | | | | | | | | | | | | | | | | | | | | | | | | | | |
| 21-225_25H10_HC | | | | | | | | | | | | | | | | | | | | | | | | | | |
| 21-225_25H11_HC | | | | | | | | | | | | | | | | | | | | | | | | | | |
| 21-225_26A2_HC | | | | | | | S | | | | | | | | | | | | | | | | | | | |
| 21-225_27C3_HC | | | | | | | | | | | | | | | | | | | | | | | | | | |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_27D5_HC | . | . | . | . | . | . | S | . | . | . | . | . | . | . | . | . | . | . | . | . | . | . | . | . | . | . |
| 21-225_27F2_HC | . | . | . | I | . | . | . | . | . | . | . | . | . | . | . | . | . | . | . | . | . | . | . | . | . | . |
| 21-225_28C7_HC | . | . | . | . | . | . | S | . | . | . | . | . | . | . | . | . | . | . | . | . | . | . | . | . | . | . |
| 21-225_28F5_HC | . | . | . | . | . | . | S | . | . | . | . | . | . | . | . | . | . | . | . | . | . | . | . | . | . | . |
| 21-225_29B8_HC | . | . | . | . | . | . | . | . | . | . | . | . | . | . | . | . | . | . | . | . | . | . | . | . | . | . |
| 21-225_29D7_HC | . | . | . | . | . | . | S | . | . | . | . | . | . | . | . | . | . | . | . | . | . | . | . | . | . | . |
| 21-225_29G4_HC | . | . | . | . | . | . | . | . | . | . | . | . | . | . | I | . | . | . | . | . | . | . | . | . | . | . |
| 21-225_29G8_HC | . | . | . | . | . | . | . | . | . | . | . | . | . | . | . | . | . | . | . | . | . | . | . | . | . | . |
| 21-225_29H6_HC | . | . | . | . | . | . | . | . | . | . | . | . | . | . | . | . | . | . | . | . | . | . | . | . | . | . |
| 21-225_2B2_HC | . | . | . | . | . | . | S | . | . | . | . | . | . | . | . | . | . | . | . | . | . | . | . | . | . | . |
| 21-225_43C3_HC | . | . | . | . | . | . | S | . | . | . | . | . | . | . | I | . | . | . | . | . | . | . | . | . | . | . |
| 21-225_44C3_HC | . | . | . | . | . | . | S | . | . | . | . | . | . | . | . | . | . | . | . | . | . | . | . | . | . | . |
| 21-225_44C8_HC | . | . | . | . | . | N | N | . | . | . | . | . | . | . | . | . | . | . | . | . | . | . | . | . | . | . |
| 21-225_46C6_HC | . | . | . | . | . | . | S | . | . | . | . | . | . | . | I | . | . | . | . | . | . | . | . | . | . | . |
| 21-225_46D8_HC | . | . | . | . | . | . | . | . | . | . | . | . | . | . | I | . | . | . | . | . | . | . | . | . | . | . |
| 21-225_46F3_HC | . | . | . | . | . | . | . | . | . | . | . | . | . | . | . | . | . | . | . | . | . | . | . | . | . | . |
| 21-225_47A1_HC | . | . | . | . | . | . | . | . | . | . | . | . | . | . | . | . | . | . | . | . | . | . | . | . | . | . |
| 21-225_48A9_HC | . | . | . | . | . | . | S | . | . | . | . | . | . | . | . | . | . | . | . | . | . | . | . | . | . | . |
| 21-225_48C1_HC | . | . | . | . | . | . | S | . | . | . | . | . | . | . | . | . | . | . | . | . | . | . | . | . | . | . |
| 21-225_48D12_HC | . | . | . | . | . | . | . | . | . | . | . | . | . | . | . | . | . | . | . | . | . | . | R | . | . | . |
| 21-225_49A1_HC | . | . | . | . | . | . | . | . | . | . | . | . | . | . | . | . | . | . | . | . | . | . | . | . | . | . |
| 21-225_49C6_HC | . | . | . | . | . | . | N | . | . | . | . | . | . | . | . | . | . | . | . | . | . | . | . | . | . | . |
| 21-225_51B11_HC | . | . | . | . | . | . | . | . | . | . | . | . | . | . | . | . | . | . | . | . | . | . | . | . | . | . |
| 21-225_51E4_HC | . | . | . | . | . | . | S | . | . | . | . | . | . | . | . | . | . | . | . | . | . | . | . | . | . | . |
| 21-225_51F11_HC | . | . | . | . | . | . | N | . | . | . | . | . | F | . | . | . | . | . | . | . | . | . | . | . | . | . |
| 21-225_51F9_HC | . | . | . | . | . | . | . | . | . | . | . | . | . | . | . | . | . | . | . | . | . | . | . | . | . | . |
| 21-225_52B2_HC | . | . | . | . | . | . | N | . | . | . | . | . | . | . | . | . | . | . | . | . | . | . | . | . | . | . |
| 21-225_52B9_HC | . | . | . | . | . | . | . | . | . | . | . | . | . | . | . | . | . | . | . | . | . | . | . | . | . | . |
| 21-225_53B12_HC | . | . | . | . | . | . | N | . | . | . | . | . | F | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_53D2_HC | . | . | . | . | . | . | N | . | . | . | . | . | . | . | . | . | . | . | . | . | . | . | . | . | . | . |
| 21-225_53E6_HC | . | . | . | . | . | . | . | . | . | . | . | . | . | . | I | . | . | . | . | . | . | . | . | . | . | . |
| 21-225_54C6_HC | . | . | . | . | . | . | . | . | . | . | . | . | . | . | I | . | . | . | . | . | . | . | . | . | . | . |
| 21-225_54D3_HC | . | . | . | . | . | . | N | . | . | . | . | . | . | . | . | . | . | . | . | . | . | . | . | . | . | . |
| 21-225_54G7_HC | . | . | . | . | . | . | N | . | . | . | . | . | . | . | . | . | . | . | . | . | . | . | . | . | . | . |
| 21-226_55B3_HC | . | . | . | . | . | . | S | . | . | . | . | . | . | . | . | . | . | . | . | . | . | . | . | . | . | . |
| 21-225_57A4_HC | . | . | . | . | . | . | . | . | . | . | . | . | . | . | . | . | . | . | . | . | . | . | . | . | . | . |
| 21-225_58F5_HC | . | . | . | . | . | . | . | . | . | . | . | . | . | . | . | . | . | . | . | . | . | . | . | . | . | . |
| 21-225_59F11_HC | . | . | . | . | . | . | N | . | . | . | . | . | . | . | . | . | . | . | . | . | . | . | . | . | . | . |
| 21-225_60A3_HC | . | . | . | . | . | . | S | . | . | . | . | . | . | . | . | . | . | . | . | . | . | . | . | . | . | . |
| 21-225_62E4_HC | . | . | . | . | . | . | . | . | . | . | . | . | . | . | . | . | . | . | . | . | . | . | . | . | . | . |
| 21-225_64E1_HC | . | . | . | . | . | . | S | . | . | . | . | . | . | . | . | . | . | . | . | . | . | . | . | . | . | . |
| 21-225_65C1_HC | . | . | . | . | . | . | . | . | . | . | . | . | . | . | . | . | . | . | . | . | . | . | . | . | . | . |
| 21-225_66C7_HC | . | . | . | . | . | . | . | . | . | . | . | . | . | . | . | . | . | . | . | . | . | . | . | . | . | . |
| 21-225_68F11_HC | . | . | . | . | . | . | . | . | . | . | . | . | . | . | . | . | . | . | . | . | . | . | . | . | . | . |
| 21-225_6A11_HC | . | . | . | . | . | . | . | . | . | . | . | . | . | . | . | . | . | . | . | . | . | . | . | . | . | . |
| 21-225_6G12_HC | . | . | . | . | . | . | S | . | . | . | . | . | . | . | . | . | . | . | . | . | . | . | . | . | . | . |
| 21-225_6G2_HC | . | . | . | . | . | . | . | . | . | . | . | . | . | . | I | . | . | . | . | . | . | . | . | . | . | . |
| 21-225_71A2_HC | . | . | . | . | . | . | . | . | . | . | . | . | . | . | . | . | . | . | . | . | . | . | . | . | . | . |
| 21-225_71B11_HC | . | . | . | . | . | . | . | . | . | . | . | . | . | . | . | . | . | . | . | . | . | . | . | . | . | . |
| 21-225_71B6_HC | . | . | . | . | . | . | N | . | . | . | . | . | . | . | . | . | . | . | . | . | . | . | . | . | . | . |
| 21-225_72G12_HC | . | . | . | . | . | . | S | . | . | . | . | . | . | . | . | . | . | . | . | . | . | . | D | T | . | . |
| 21-225_7C4_HC | . | . | . | . | . | . | N | . | . | . | . | . | . | . | . | . | . | . | . | . | . | . | . | . | . | . |
| 21-225_7E9_HC | . | . | . | . | . | . | . | . | . | . | . | . | . | . | . | . | . | . | . | . | . | . | . | . | . | . |
| 21-225_7G2_HC | . | . | . | . | . | . | . | . | . | . | . | . | . | . | . | . | . | . | . | . | . | . | . | . | . | . |
| 21-225_7H11_HC | . | . | . | . | . | . | . | . | . | . | . | . | . | . | . | . | . | . | . | . | . | . | . | . | . | . |
| 21-225_8C9_HC | . | . | . | . | . | . | S | . | . | . | . | . | . | . | . | . | . | . | . | . | . | . | . | . | . | . |
| 21-225_8H7_HC | . | . | . | . | . | . | . | . | . | . | . | F | . | . | . | . | . | . | . | . | . | . | . | . | . | . |
| 21-225_9A1_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | H_FR2 | | | | | | |
| CONSENSUS | G | - | F | T | F | S | D | - | - | - | - | - | Y | G | M | H | W | V | R | Q | A | P | G | K | G | L |
| 21-225_9E8_HC | . | . | . | . | . | . | S | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | V | A | V | I | W | Y | D | - | - | - | E | N | N | K | Y | Y | A | D | S | V | K | G | R | F |
| 21-225_10G5_HC | . | . | . | . | . | . | . | . | E | . | . | . | . | . | . | Q | . | . | . | . | . | . | . | . | . | . |
| 21-225_10H12_HC | . | . | . | . | . | . | . | . | E | . | . | . | D | . | . | Q | . | . | . | . | . | . | . | . | . | . |
| 21-225_12A1_HC | . | . | . | . | . | . | . | . | E | . | . | . | . | . | . | Q | . | . | . | . | . | . | . | . | . | . |
| 21-225_12B12_HC | . | . | M | . | . | . | . | . | . | . | . | . | V | T | . | . | . | . | . | . | . | . | . | . | . | . |
| 21-225_12E6_HC | . | . | . | T | . | . | . | . | . | . | . | . | . | S | . | . | . | . | . | . | . | . | . | . | . | . |
| 21-225_12F2_HC | . | . | . | . | . | . | . | F | . | . | . | . | G | . | . | . | . | . | V | . | . | . | . | . | . | . |
| 21-225_13E6_HC | . | . | . | . | . | . | . | . | . | . | . | . | . | . | . | . | . | . | I | . | . | . | . | . | . | . |
| 21-225_146H9_HC | . | . | . | . | . | . | . | F | . | . | . | . | . | S | . | . | H | . | G | . | . | . | . | . | . | . |
| 21-225_14E3_HC | . | . | . | . | . | . | . | . | E | . | . | . | . | . | . | Q | . | . | . | . | . | . | . | . | . | . |
| 21-225_157C3_HC | . | . | . | . | . | . | . | . | . | . | . | . | V | . | . | . | . | . | . | . | . | . | . | . | . | . |
| 21-225_157H8_HC | . | . | . | . | . | . | . | . | . | . | . | . | V | . | . | . | . | . | . | . | . | . | . | . | . | . |
| 21-225_158H6_HC | . | . | . | . | . | . | . | . | . | . | . | . | V | T | . | . | . | . | . | . | . | . | . | . | . | . |
| 21-225_159C5_HC | . | . | . | . | V | . | . | . | . | . | . | . | V | . | . | . | . | . | . | . | . | . | . | . | . | . |
| 21-225_15E3_HC | . | . | . | . | . | . | . | . | E | . | . | . | . | . | . | . | H | . | . | . | . | . | . | . | . | . |
| 21-225_16A5_HC | . | . | . | . | . | . | . | F | . | . | . | . | G | S | . | . | . | . | V | . | . | . | . | . | . | . |
| 21-225_16B7_HC | . | . | . | . | . | . | . | . | . | . | . | . | . | S | . | . | . | . | . | . | . | . | . | . | . | . |
| 21-225_16F10_HC | . | . | . | . | . | . | . | . | . | . | . | . | G | . | . | . | . | . | V | . | . | . | . | . | . | . |
| 21-225_16F11_HC | . | . | . | . | . | . | . | . | E | . | . | . | . | . | . | . | . | . | V | . | . | . | . | . | . | . |
| 21-225_16F4_HC | . | . | . | . | . | . | . | . | E | . | . | . | G | S | . | Q | . | . | G | . | . | . | . | . | . | . |
| 21-225_16F6_HC | . | . | . | . | . | . | . | . | E | . | . | . | G | S | . | Q | . | . | G | . | . | . | . | . | . | . |
| 21-225_16G7_HC | . | . | . | . | . | . | . | . | E | . | . | . | . | . | . | E | . | . | . | . | . | . | . | . | . | . |
| 21-225_17A10_HC | . | . | . | . | . | . | . | . | E | . | . | . | . | . | . | Q | . | . | . | . | . | . | . | . | . | . |
| 21-225_17B5_HC | . | . | . | . | . | . | . | . | . | . | . | . | . | S | . | . | H | . | I | . | . | . | . | . | . | . |
| 21-225_17C7_HC | . | . | . | . | . | . | . | F | . | . | . | . | G | S | . | . | . | . | . | . | . | . | . | . | . | . |
| 21-225_17E5_HC | . | . | . | . | L | . | . | . | . | . | . | . | V | T | . | . | . | . | G | . | . | . | . | . | . | . |
| 21-225_18A1_HC | . | . | . | . | . | . | . | . | E | . | . | . | . | . | . | . | . | . | V | . | . | . | . | . | . | . |
| 21-225_18A5_HC | . | . | . | . | . | . | . | F | . | . | . | . | G | S | . | . | . | . | . | . | . | . | . | . | . | . |
| 21-225_18C2_HC | . | . | M | . | . | . | . | . | . | . | . | . | V | T | . | . | . | . | . | . | . | . | . | . | . | . |
| 21-225_18E8_HC | . | . | . | . | . | . | . | . | E | . | . | . | . | . | . | Q | . | . | . | . | . | . | . | . | . | . |

# EP 4 435 105 A2

## FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | E | N | N | K | Y | Y | A | D | S | V | K | G | R | F |
| 21-225_18F2_HC | . | . | . | . | . | . | . | F | . | . | . | . | G | S | . | . | . | . | V | . | . | . | . | . | . | . |
| 21-225_19E1_HC | . | . | . | . | . | . | . | E | . | . | . | . | . | . | . | Q | . | . | . | . | . | . | . | . | . | . |
| 21-225_1C12_HC | . | . | . | . | . | . | . | . | . | . | . | . | . | . | . | . | . | . | V | . | . | . | . | . | . | . |
| 21-225_203B9_HC | . | . | . | . | . | . | . | . | . | . | . | . | V | . | . | . | . | . | . | . | . | . | . | . | . | . |
| 21-225_207F12_HC | . | . | . | . | . | . | . | . | . | . | . | . | R | . | . | . | . | . | G | . | . | . | . | . | . | . |
| 21-225_20A7_HC | . | . | . | . | . | . | . | E | . | . | . | . | . | . | . | Q | . | . | . | . | . | . | . | . | . | . |
| 21-225_20D10_HC | . | . | . | . | . | . | . | F | . | . | . | . | G | S | . | . | . | . | V | . | . | . | . | . | . | . |
| 21-225_20D5_HC | . | . | . | . | . | . | . | E | . | . | . | . | . | . | . | Q | . | . | . | . | . | . | . | . | . | . |
| 21-225_20E12_HC | . | . | . | . | . | M | . | . | . | . | . | . | . | S | . | . | H | . | . | . | . | . | . | . | . | . |
| 21-225_20E5_HC | . | . | . | . | . | . | . | E | . | . | . | . | . | . | . | Q | . | . | . | . | . | . | . | . | . | . |
| 21-225_20H12_HC | . | . | . | . | . | . | . | . | . | . | . | . | . | . | . | . | . | . | V | . | . | . | . | . | . | . |
| 21-225_21E5_HC | . | . | . | . | . | . | . | . | . | . | . | . | . | S | . | . | . | . | . | . | . | . | . | . | . | . |
| 21-225_21F7_HC | . | . | . | . | . | . | . | F | . | . | . | . | G | . | . | . | . | . | V | . | . | . | . | . | . | . |
| 21-225_21G11_HC | . | . | M | . | . | . | . | . | . | . | . | . | V | T | . | E | . | . | . | . | . | . | . | . | . | . |
| 21-225_21G2_HC | . | . | M | . | . | . | . | . | . | . | . | . | V | T | . | . | . | . | . | . | . | . | . | . | . | . |
| 21-225_21H8_HC | . | . | . | . | . | . | . | F | . | . | . | . | G | S | . | . | . | . | . | . | . | . | . | . | . | . |
| 21-225_224D10_HC | . | . | . | . | . | . | . | E | . | . | . | . | G | S | . | . | . | . | V | . | . | . | R | . | . | . |
| 21-225_22A4_HC | . | . | . | . | . | . | . | F | . | . | . | . | G | S | . | . | . | . | . | . | . | . | . | . | . | I |
| 21-225_22F2_HC | . | . | . | . | . | . | . | E | . | . | . | . | G | S | . | . | . | . | V | . | . | . | R | . | . | . |
| 21-225_22F9_HC | . | . | . | . | . | . | . | E | . | . | . | . | . | . | . | . | D | . | V | . | . | . | . | . | . | . |
| 21-225_22H8_HC | . | . | . | . | . | . | . | . | . | . | . | . | G | . | . | . | . | . | . | . | . | . | . | . | . | . |
| 21-225_23F12_HC | . | . | . | . | . | . | . | . | . | . | . | . | . | T | . | . | . | . | . | . | . | . | . | . | . | . |
| 21-225_23H11_HC | . | . | . | . | . | . | . | . | . | . | . | . | . | . | . | . | . | . | V | . | . | . | . | . | . | . |
| 21-225_25B6_HC | . | . | . | . | . | . | . | E | . | . | . | . | . | S | . | . | . | . | T | E | . | . | . | . | . | . |
| 21-225_25E6_HC | . | . | . | . | . | . | . | . | . | . | . | . | . | . | . | . | . | . | . | . | . | . | . | . | . | . |
| 21-225_25H10_HC | . | . | . | . | . | . | . | . | . | . | . | . | . | S | . | . | . | . | . | . | . | M | . | . | . | . |
| 21-225_25H11_HC | . | . | . | . | . | . | . | . | . | . | . | . | . | . | . | E | . | . | . | . | . | . | . | . | . | . |
| 21-225_26A2_HC | . | . | . | . | . | . | . | E | . | . | . | . | G | S | . | . | . | . | V | . | . | . | . | . | . | . |
| 21-225_27C3_HC | . | . | . | . | . | . | . | . | . | . | . | . | . | . | . | . | S | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | E | N | N | K | Y | Y | A | D | S | V | K | G | R | F |
| 21-225_27D5_HC | . | . | . | . | . | . | . | . | . | . | . | . | . | S | . | . | . | . | G | . | . | . | . | . | . | . |
| 21-225_27F2_HC | . | . | . | . | . | . | . | . | . | . | . | . | . | . | . | . | . | . | V | . | . | . | R | . | . | . |
| 21-225_28C7_HC | . | . | . | . | . | . | . | . | . | . | . | . | . | S | . | . | . | . | . | . | . | . | . | . | . | . |
| 21-225_28F5_HC | . | . | . | . | . | . | . | . | . | . | . | . | . | . | . | . | . | . | . | . | . | . | . | . | . | . |
| 21-225_29B8_HC | . | . | . | . | . | . | . | . | . | . | . | . | . | . | . | . | . | . | . | . | . | . | . | . | . | . |
| 21-225_29D7_HC | . | . | . | . | . | . | . | . | . | . | . | . | . | S | . | . | . | . | G | . | . | . | . | . | . | . |
| 21-225_29G4_HC | . | . | . | . | . | . | . | . | . | . | . | . | . | S | . | . | . | . | . | . | . | M | . | . | . | . |
| 21-225_29G8_HC | . | . | . | . | . | . | . | . | . | . | . | . | . | T | . | . | . | . | . | . | . | . | . | . | . | . |
| 21-225_29H6_HC | . | . | . | T | . | . | . | . | N | . | . | . | . | . | . | . | . | . | . | . | . | . | . | . | . | . |
| 21-225_2B2_HC | . | . | . | . | . | . | . | . | E | . | . | . | . | . | . | . | . | . | V | . | . | . | . | . | . | . |
| 21-225_43C3_HC | . | . | . | . | . | . | . | . | . | . | . | . | . | . | . | . | . | . | . | . | . | . | . | . | . | . |
| 21-225_44C3_HC | . | . | . | . | . | . | . | F | E | . | . | . | G | S | . | . | . | . | . | . | . | . | . | . | . | . |
| 21-225_44C8_HC | . | . | . | . | . | . | . | . | E | . | . | . | G | S | . | . | . | . | . | . | . | . | . | . | . | . |
| 21-225_46C6_HC | . | . | . | . | . | . | . | . | . | . | . | . | . | . | . | . | . | . | . | . | . | . | . | . | . | . |
| 21-225_46D8_HC | . | . | . | . | . | . | . | F | E | . | . | . | G | S | . | . | . | . | . | . | . | . | . | . | . | . |
| 21-225_46F3_HC | . | . | . | . | . | . | . | F | E | . | . | . | G | S | . | . | . | . | . | . | . | . | . | . | . | . |
| 21-225_47A1_HC | . | . | . | . | . | . | . | . | . | . | . | . | D | Y | . | . | K | . | . | . | . | . | . | . | . | . |
| 21-225_48A9_HC | . | . | . | . | . | . | . | . | E | . | . | . | . | . | K | . | . | . | . | . | . | . | . | . | . | . |
| 21-225_48C1_HC | . | . | . | . | V | . | . | . | . | . | . | . | . | I | . | . | K | . | . | . | . | . | . | . | . | V |
| 21-225_48D12_HC | . | . | . | . | . | . | . | . | . | . | . | . | V | S | . | . | . | . | V | . | . | . | . | . | . | . |
| 21-225_49A1_HC | . | . | . | . | . | . | . | . | . | . | . | . | . | D | . | . | . | . | V | . | . | . | . | . | . | . |
| 21-225_49C6_HC | . | . | . | . | . | . | F | . | . | . | . | . | V | S | . | . | K | . | V | . | . | . | . | . | . | . |
| 21-225_51B11_HC | . | . | . | . | . | . | . | F | G | . | . | . | G | . | . | . | . | . | G | . | . | . | . | . | . | . |
| 21-225_51E4_HC | . | . | . | . | . | . | . | . | . | . | . | . | . | S | . | . | . | . | . | . | . | . | . | . | . | . |
| 21-225_51F11_HC | . | . | . | . | . | . | . | . | E | . | . | . | . | S | . | . | . | . | . | . | . | . | . | . | . | . |
| 21-225_51F9_HC | . | . | . | . | . | . | . | F | . | . | . | . | G | . | . | . | . | . | . | . | . | . | . | . | . | . |
| 21-225_52B2_HC | . | . | . | . | . | T | . | F | . | . | . | . | G | S | . | Q | R | . | . | . | . | . | . | . | . | . |
| 21-225_52B9_HC | . | . | . | . | . | . | . | F | . | . | . | . | G | . | . | . | . | . | . | . | . | . | . | . | . | . |
| 21-225_53B12_HC | . | . | . | . | V | . | . | . | . | . | . | . | G | . | . | . | R | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
|  |  |  |  |  |  |  |  |  |  |  |  |  |  | H_CDR2 |  |  |  |  |  |  |  |  |  |  |  |  |
| CONSENSUS | E | W | V | A | V | I | W | Y | D | - | - | - | E | N | N | K | Y | Y | A | D | S | V | K | G | R | F |
| 21-225_53D2_HC |  |  |  | T |  | V |  | D |  |  |  |  | G | S |  |  |  |  |  |  |  |  |  |  |  |  |
| 21-225_53E6_HC |  |  |  |  |  |  |  |  |  |  |  |  |  | S |  |  |  |  | G |  |  |  |  |  |  |  |
| 21-225_54C6_HC | D |  |  |  |  |  |  |  |  |  |  |  |  |  |  |  |  |  |  |  |  |  |  |  |  |  |
| 21-225_54D3_HC |  |  |  |  |  | T |  | F |  |  |  |  | G |  |  | N |  |  |  |  |  |  |  |  |  |  |
| 21-225_54G7_HC |  |  |  |  |  |  |  |  | E |  |  |  |  | S |  |  |  |  | G |  |  |  |  |  |  |  |
| 21-225_55B3_HC |  |  |  |  |  |  |  | F |  |  |  |  | G |  |  |  |  |  | E |  |  |  |  |  |  |  |
| 21-225_57A4_HC |  |  |  |  |  |  |  |  | A |  |  |  | G | S |  |  |  |  | V |  |  |  |  |  |  |  |
| 21-225_58F5_HC |  |  |  |  |  |  |  |  | A |  |  |  | G | S |  |  | H |  | V |  |  |  |  |  |  |  |
| 21-225_59F11_HC |  |  |  |  |  |  |  |  |  |  |  |  |  | S |  |  | H |  |  |  |  |  |  |  |  |  |
| 21-225_60A3_HC |  |  |  |  |  |  |  |  | E |  |  |  |  | S |  |  |  |  |  |  |  |  |  |  |  |  |
| 21-225_62E4_HC |  |  |  |  |  |  |  |  |  |  |  |  | R | S |  |  |  |  |  |  |  |  |  |  |  |  |
| 21-225_64E1_HC |  |  |  |  |  |  |  | F |  |  |  |  |  | T |  |  |  |  | G |  |  |  |  |  |  |  |
| 21-225_65C1_HC |  |  |  |  |  |  |  | F | E |  |  |  | G | S |  |  | H |  | T |  |  |  |  |  |  |  |
| 21-225_66C7_HC |  |  |  |  |  |  |  | F | E |  |  |  | G | S | H |  |  |  | T |  |  |  |  |  |  |  |
| 21-225_68F11_HC |  |  |  |  |  |  |  |  |  |  |  |  | V | S |  |  |  |  |  |  |  |  |  |  |  |  |
| 21-225_6A11_HC |  |  |  |  |  |  |  | F |  |  |  |  | G | S |  |  |  |  |  |  |  |  |  |  |  |  |
| 21-225_6G12_HC |  |  |  |  |  |  |  |  |  |  |  |  | R | S |  | N |  |  |  |  |  |  |  |  |  |  |
| 21-225_6G2_HC |  |  |  |  |  |  |  | F |  |  |  |  | G | S |  |  |  |  |  |  |  |  |  |  |  |  |
| 21-225_71A2_HC |  |  |  |  |  |  |  |  |  |  |  |  |  | S |  |  |  |  |  |  |  |  |  |  |  |  |
| 21-225_71B11_HC |  |  |  |  |  |  |  |  |  |  |  |  | R |  |  |  |  |  | G |  |  |  |  |  |  |  |
| 21-225_71B6_HC |  |  |  |  |  |  |  |  |  |  |  |  | R | T |  |  |  |  |  |  |  |  |  |  |  |  |
| 21-225_72G12_HC |  |  |  |  |  |  |  | F |  |  |  |  |  | S |  |  |  |  |  |  |  |  |  |  |  |  |
| 21-225_7C4_HC |  |  |  |  |  |  |  |  | E |  |  |  |  |  |  | Q |  |  |  |  |  |  |  |  |  |  |
| 21-225_7E9_HC |  |  |  |  |  |  |  |  |  |  |  |  |  |  |  |  |  |  | V |  |  |  |  |  |  |  |
| 21-225_7G2_HC |  |  | M |  |  |  |  |  |  |  |  |  | V | T |  |  |  |  |  |  |  |  |  |  |  |  |
| 21-225_7H11_HC |  |  |  |  |  |  |  |  |  |  |  |  |  |  |  |  |  |  |  |  |  |  |  |  |  |  |
| 21-225_8C9_HC |  |  |  |  |  |  |  |  |  |  |  |  |  |  |  |  |  |  | V |  |  |  |  |  |  |  |
| 21-225_8H7_HC |  |  |  |  |  |  |  |  | E |  |  |  |  |  |  | Q |  |  |  |  |  |  |  |  |  |  |
| 21-225_9A1_HC |  |  |  |  |  |  |  | F |  |  |  |  | G | S |  |  |  |  |  |  |  |  |  |  |  |  |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | | H_CDR2 | | | | | | | | | | |
| CONSENSUS | E | W | V | A | V | I | W | Y | D | - | - | - | E | N | N | K | Y | Y | A | D | S | V | K | G | R | F |
| 21-225_9E8_HC | . | . | . | . | . | . | . | E | . | . | . | . | . | . | Q | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_10G5_HC | . | . | . | . | . | . | . | . | . | . | . | . | . | . | . | . | . | . | . | . | . | . | . | . | . | . |
| 21-225_10H12_HC | . | . | . | . | . | . | . | . | . | . | . | . | . | . | . | . | . | . | . | . | . | . | . | . | . | . |
| 21-225_12A1_HC | . | . | . | . | . | . | . | . | . | . | . | . | . | . | . | . | . | . | . | . | . | . | . | . | . | . |
| 21-225_12B12_HC | . | . | . | . | . | . | . | . | . | . | . | . | . | . | . | . | . | . | . | . | . | . | . | . | . | . |
| 21-225_12E6_HC | . | . | . | . | . | D | . | . | . | . | . | . | . | . | . | . | . | . | . | . | . | . | . | . | . | . |
| 21-225_12F2_HC | . | . | . | . | . | . | . | . | . | . | . | F | . | . | . | . | . | . | . | . | . | . | . | . | . | . |
| 21-225_13E6_HC | . | . | . | . | . | . | . | . | . | . | . | . | . | . | . | . | . | . | . | . | . | . | . | . | . | . |
| 21-225_146H9_HC | . | . | . | . | . | . | . | . | . | . | . | . | . | . | . | . | . | . | . | . | . | . | . | . | . | . |
| 21-225_14E3_HC | . | . | . | . | . | . | . | . | . | . | . | . | . | . | . | . | . | . | . | . | . | . | . | . | F | . |
| 21-225_157C3_HC | . | . | . | . | . | . | . | . | . | . | . | . | . | . | . | . | . | . | . | . | . | . | . | . | . | . |
| 21-225_157H8_HC | . | . | . | . | . | . | . | . | . | . | . | . | . | . | . | . | . | . | . | . | . | . | . | . | . | . |
| 21-225_158H6_HC | . | . | . | . | . | . | . | . | . | . | . | . | . | . | . | . | . | . | . | D | . | . | . | . | . | . |
| 21-225_159C5_HC | . | . | . | . | . | . | . | . | . | . | . | . | . | . | . | . | . | . | . | . | . | . | . | . | . | . |
| 21-225_15E3_HC | . | . | . | . | . | . | . | . | . | . | . | . | . | . | . | . | . | . | . | . | . | . | . | . | . | . |
| 21-225_16A5_HC | . | . | . | . | . | . | . | . | . | . | . | . | . | . | . | S | . | . | . | . | . | . | . | . | . | . |
| 21-225_16B7_HC | . | . | . | . | . | . | . | . | . | . | . | . | . | . | . | . | . | . | . | . | . | . | . | . | . | . |
| 21-225_16F10_HC | . | . | . | . | . | . | . | . | . | . | . | . | . | . | . | . | . | . | . | . | . | . | . | . | . | . |
| 21-225_16F11_HC | . | . | . | . | . | . | . | . | S | . | . | . | . | . | . | . | . | . | . | . | . | . | . | . | . | . |
| 21-225_16F4_HC | . | . | . | . | . | . | . | . | . | . | V | . | . | . | . | H | . | . | . | . | . | . | . | . | . | . |
| 21-225_16F6_HC | . | . | . | . | . | . | . | . | . | . | V | . | . | . | . | H | . | . | . | . | . | . | . | . | . | . |
| 21-225_16G7_HC | . | . | . | . | A | . | . | . | . | . | . | . | . | . | . | . | . | . | . | . | . | . | . | . | . | . |
| 21-225_17A10_HC | . | . | . | . | . | . | . | . | . | . | . | . | . | . | . | . | . | . | . | . | . | . | . | . | . | . |
| 21-225_17B5_HC | . | . | . | . | . | . | . | . | . | . | . | . | . | . | . | . | . | . | . | . | . | . | . | . | . | . |
| 21-225_17C7_HC | . | . | . | . | . | . | . | . | . | . | . | . | . | . | . | . | . | . | . | . | . | . | . | G | . | . |
| 21-225_17E5_HC | . | . | . | . | . | . | . | Q | . | . | . | . | . | L | . | . | . | . | . | . | . | . | . | . | . | . |
| 21-225_18A1_HC | . | . | . | . | . | . | . | . | S | . | . | . | . | . | . | . | . | . | . | . | . | . | . | . | . | . |
| 21-225_18A5_HC | . | . | . | . | . | . | . | . | . | . | . | . | . | . | . | . | . | . | . | . | . | . | . | . | . | . |
| 21-225_18C2_HC | . | . | . | . | . | . | . | . | . | . | . | . | . | . | . | . | . | . | S | . | . | . | . | . | . | . |
| 21-225_18E8_HC | . | V | . | . | . | . | . | . | . | . | . | F | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_18F2_HC | | | | | | | | | | | | | | | | | | | | V | | | | | | |
| 21-225_19E1_HC | | | | | | | | | | | | | | | | | | | | | | | | | | |
| 21-225_1C12_HC | | | | | | | | | | | | | | | | | | | | | | | | | | |
| 21-225_203B9_HC | | | | | | | | | | | | | | | | | | | | P | | | | | | |
| 21-225_207F12_HC | | | | | | | | | | | | | | | | | | | | | | | | | | |
| 21-225_20A7_HC | | | | | | | | | | | | | | | | | | | | | | | | | I | |
| 21-225_20D10_HC | | | | | | D | | | | | | | | | | | | | | | | | | | | |
| 21-225_20D5_HC | | | | | | | | | | | | | | | | | | | | | D | | | | | |
| 21-225_20E12_HC | | | | | | | | | R | | | | | | | | | | | | | | | | | |
| 21-225_20E5_HC | | | | | | | | | | | | | | | | | | | | | | | | | | |
| 21-225_20H12_HC | | | | | | | | | | | | | | | | | | | | | | | | | | |
| 21-225_21E5_HC | | | | | | | | | | | | | | | | | | | | | | | | | M | |
| 21-225_21F7_HC | | | | | | | | | | | | | | | | | | | | | | | | | | |
| 21-225_21G11_HC | | | | | | | | | | | | | | | | | | | | | | | | | | |
| 21-225_21G2_HC | | | | | | | | | | | | | | E | | | | | | | | | | | | |
| 21-225_21H8_HC | | | | | | | | | | | | | | | | | | | | | | | | | | |
| 21-225_224D10_HC | | | | | | | | | S | | | | | | | | | | | | | | | | | |
| 21-225_22A4_HC | | | | | | | | | | | | | | | | | | | | | | | | | | |
| 21-225_22F2_HC | | | | | | | | | S | | | | | | | | | | | | | | | | | |
| 21-225_22F9_HC | | | | | | | | | S | | | | | | | | | | | | | | | | | |
| 21-225_22H8_HC | | | | | | | | | | | | | | | | | | | | | | | | | | |
| 21-225_23F12_HC | | | | | | | | | | | | | | | | | | | | | | | | | I | |
| 21-225_23H11_HC | | | | | | | | | | | | | | | | | | | | | | | | | | |
| 21-225_25B6_HC | | | | | | | | | | | | | | | | | | | | | | | | | | |
| 21-225_25E6_HC | | | | | | | | | | | | | | | | | | | | | | | | | L | |
| 21-225_25H10_HC | | | | | | | | | | | | | | | | | | | | | | | | | | |
| 21-225_25H11_HC | | | | | | | | | | | | | | | | | | | | | | | | | | |
| 21-225_26A2_HC | | | | | | | | | | | | | | | | | | | | | | | | | | |
| 21-225_27C3_HC | | | | | | | | | | | | | | | | | | | | | | | | | | |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_27D5_HC | . | . | . | . | . | . | . | . | . | M | . | . | . | . | . | . | . | . | . | G | . | . | . | . | L | . |
| 21-225_27F2_HC | . | . | . | . | . | . | . | . | . | . | . | . | . | . | . | . | . | . | . | . | . | . | . | . | . | . |
| 21-225_28C7_HC | . | . | . | . | . | . | . | . | . | . | . | . | . | . | . | . | . | . | . | . | . | . | . | . | . | . |
| 21-225_28F5_HC | . | . | . | . | . | . | . | . | . | . | . | . | . | . | . | . | . | . | . | . | . | . | . | . | . | . |
| 21-225_29B8_HC | . | . | . | . | . | . | . | . | . | . | . | . | . | . | . | . | . | . | . | . | . | . | . | . | . | . |
| 21-225_29D7_HC | . | . | . | . | . | . | . | . | . | . | . | . | . | . | . | . | . | . | . | G | . | . | . | . | L | . |
| 21-225_29G4_HC | . | . | . | . | . | . | . | . | . | . | . | . | . | . | . | . | . | . | . | . | . | . | . | . | . | . |
| 21-225_29G8_HC | . | . | . | . | . | . | . | . | . | . | . | . | . | . | . | . | . | . | . | . | . | . | . | . | . | . |
| 21-225_29H6_HC | . | . | . | . | . | . | . | . | . | . | . | . | . | . | . | . | . | . | . | . | . | . | . | . | . | . |
| 21-225_2B2_HC | . | . | . | . | . | . | S | . | . | . | . | . | . | . | . | . | . | . | . | . | . | . | . | . | . | . |
| 21-225_43C3_HC | . | . | . | . | . | . | . | . | . | . | . | . | . | . | . | . | . | . | . | . | . | . | . | . | . | . |
| 21-225_44C3_HC | . | . | . | . | . | . | . | . | . | . | . | . | . | . | . | S | . | . | . | . | . | . | . | . | . | . |
| 21-225_44C8_HC | . | . | . | . | . | . | . | . | . | . | . | . | . | . | . | . | . | . | . | . | . | . | . | . | . | . |
| 21-225_46C6_HC | . | . | . | . | . | . | . | . | . | . | . | . | . | . | . | . | . | . | . | . | . | . | . | . | . | . |
| 21-225_46D8_HC | . | . | . | . | . | . | . | . | . | . | . | . | V | . | . | . | . | . | . | . | . | . | . | . | . | . |
| 21-225_46F3_HC | . | . | . | . | . | . | . | . | . | . | . | . | V | . | . | . | . | . | . | . | . | . | . | . | . | . |
| 21-225_47A1_HC | . | . | . | . | . | . | A | . | . | . | . | . | . | V | . | . | . | . | . | V | . | . | . | . | . | . |
| 21-225_48A9_HC | . | . | . | . | . | . | . | . | . | . | . | F | . | . | . | . | . | . | . | . | . | . | . | . | M | . |
| 21-225_48C1_HC | . | . | . | . | . | . | . | . | . | . | . | . | . | . | . | . | . | . | . | . | . | . | . | . | M | . |
| 21-225_48D12_HC | . | . | . | . | . | . | . | . | . | . | . | . | . | . | . | . | . | . | . | . | . | . | . | . | . | . |
| 21-225_49A1_HC | . | . | . | . | . | . | . | . | . | . | . | . | . | . | . | . | . | . | . | . | . | . | . | . | . | . |
| 21-225_49C6_HC | . | . | . | . | . | . | . | . | . | . | . | . | . | . | . | S | . | . | . | . | . | . | . | . | . | . |
| 21-225_51B11_HC | . | . | . | . | . | . | . | . | . | . | . | . | . | . | . | . | . | . | S | . | . | . | . | . | . | . |
| 21-225_51E4_HC | . | . | . | . | . | . | . | . | . | . | . | . | . | . | . | . | . | . | . | . | . | . | . | . | . | . |
| 21-225_51F11_HC | . | . | . | . | . | . | . | . | . | . | . | . | . | . | . | . | . | . | . | . | . | . | . | . | . | . |
| 21-225_51F9_HC | . | . | . | . | . | . | . | . | . | . | . | . | . | . | . | . | . | . | S | . | . | . | . | . | . | . |
| 21-225_52B2_HC | . | . | . | . | I | . | . | . | . | . | . | . | . | . | . | . | . | . | S | . | . | . | . | . | . | . |
| 21-225_52B9_HC | . | . | . | . | . | . | . | . | . | . | . | . | . | . | . | . | . | . | S | . | . | . | . | . | . | . |
| 21-225_53B12_HC | . | . | . | . | . | . | . | . | . | . | . | . | . | . | . | H | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | | H_FR3 | | | | | | | | | | | |
| CONSENSUS | T | I | S | R | D | N | S | K | N | T | L | Y | L | Q | M | N | S | L | R | A | E | D | T | A | V | Y |
| 21-225_53D2_HC | . | . | . | . | . | . | . | . | . | . | . | . | . | . | . | . | . | . | . | . | . | . | . | . | . | . |
| 21-225_53E6_HC | A | . | . | . | . | . | . | . | . | . | . | . | . | . | . | . | . | . | . | . | . | . | . | . | . | . |
| 21-225_54C6_HC | A | . | . | . | . | . | . | . | . | . | . | . | . | . | . | . | . | . | . | . | . | . | . | . | . | . |
| 21-225_54D3_HC | . | . | . | . | . | . | . | . | . | . | . | . | . | . | . | . | . | . | . | . | . | . | . | . | . | . |
| 21-225_54G7_HC | . | . | . | . | . | . | . | . | . | . | . | . | . | . | . | . | . | . | . | . | . | . | . | . | . | . |
| 21-225_55B3_HC | . | . | . | . | . | . | . | . | . | . | . | . | . | . | . | . | . | . | . | . | . | . | . | . | . | . |
| 21-225_57A4_HC | . | . | . | . | . | . | . | . | . | . | . | . | . | . | . | . | . | . | . | . | . | . | . | . | . | . |
| 21-225_58F5_HC | . | . | . | . | . | . | . | . | . | . | . | . | . | . | . | . | . | . | . | . | . | . | . | . | . | . |
| 21-225_59F11_HC | . | . | . | . | . | . | . | . | T | . | . | . | . | . | . | S | . | . | . | . | . | . | . | . | . | . |
| 21-225_60A3_HC | . | . | . | . | . | . | . | . | . | . | . | . | . | . | . | . | . | . | . | . | . | . | . | . | . | . |
| 21-225_62E4_HC | . | . | . | . | . | . | . | . | . | . | . | . | . | . | . | . | . | . | . | V | . | . | . | . | . | . |
| 21-225_64E1_HC | . | . | . | . | . | . | . | . | . | . | . | . | . | . | . | . | . | . | . | . | . | . | . | . | . | . |
| 21-225_65C1_HC | . | . | . | . | . | . | . | . | . | . | . | . | . | . | . | . | . | . | . | . | . | . | . | . | . | . |
| 21-225_66C7_HC | . | . | . | . | . | . | . | . | . | . | . | . | . | . | . | . | . | . | . | . | . | . | . | . | . | . |
| 21-225_68F11_HC | . | . | . | . | . | . | . | . | . | . | . | . | . | . | . | . | . | . | . | . | . | . | . | . | . | . |
| 21-225_6A11_HC | . | . | . | . | . | . | . | . | . | . | . | . | . | . | . | . | . | . | . | . | . | . | . | . | . | . |
| 21-225_6G12_HC | . | . | . | . | . | . | . | . | . | . | . | . | . | . | . | . | . | . | . | . | . | . | . | . | . | . |
| 21-225_6G2_HC | . | . | S | . | . | . | . | . | . | . | . | . | . | . | . | . | . | . | . | . | . | . | . | . | . | . |
| 21-225_71A2_HC | . | . | . | . | . | . | . | . | . | . | . | . | . | . | . | . | . | . | . | . | . | . | . | . | . | . |
| 21-225_71B11_HC | . | . | . | . | . | . | . | . | . | . | . | . | . | . | . | . | . | . | . | . | . | . | . | . | . | . |
| 21-225_71B6_HC | . | . | . | . | . | . | . | . | . | . | H | . | . | . | . | . | . | . | . | . | . | . | . | . | . | . |
| 21-225_72G12_HC | . | . | . | . | . | . | . | . | . | . | . | . | . | . | . | . | . | . | . | . | . | . | . | . | . | . |
| 21-225_7C4_HC | . | . | . | . | . | . | . | . | . | . | . | . | . | . | . | . | . | . | . | . | . | . | . | . | . | . |
| 21-225_7E9_HC | . | . | . | . | . | . | . | . | . | . | . | . | . | . | . | . | . | . | . | . | . | . | . | . | . | . |
| 21-225_7G2_HC | . | . | . | . | . | . | . | . | . | . | . | . | . | L | . | . | . | . | . | . | . | . | . | . | . | . |
| 21-225_7H11_HC | . | . | . | . | . | . | . | . | . | . | . | . | . | . | . | . | . | . | . | . | . | . | . | . | . | . |
| 21-225_8C9_HC | . | . | . | . | . | . | . | . | . | . | . | . | . | . | . | . | . | . | . | . | . | . | . | . | . | . |
| 21-225_8H7_HC | . | . | . | . | . | . | . | . | . | . | . | . | . | . | . | . | . | . | . | . | . | . | . | . | . | . |
| 21-225_9A1_HC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | H_FR3 | | | | | | | | | | | | | | | |
| CONSENSUS | T | I | S | R | D | N | S | K | N | T | L | Y | L | Q | M | N | S | L | R | A | E | D | T | A | V | Y |
| 21-225_9E8_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | L | G | F | - | - | - | - | - | - | - | - | - | - | - | - | - | - | - | - | - | - |
| 21-225_10G5_HC | | | T | | | | | | | | | | | | | | | | | | | | | | | |
| 21-225_10H12_HC | | | | | | | | | | | | | | | | | | | | | | | | | | |
| 21-225_12A1_HC | | | | | | | | | | | | | | | | | | | | | | | | | | |
| 21-225_12B12_HC | | | | | | | | W | | | | | | | | | | | | | | | | | | |
| 21-225_12E6_HC | | | | | | | A | W | | | | | | | | | | | | | | | | | | |
| 21-225_12F2_HC | | | V | | | | A | W | | | | | | | | | | | | | | | | | | |
| 21-225_13E6_HC | | | | | | | | | | | | | | | | | | | | | | | | | | |
| 21-225_146H9_HC | | | | | | | | | | | | | | | | | | | | | | | | | | |
| 21-225_14E3_HC | | | | | | | | | | | | | | | | | | | | | | | | | | |
| 21-225_157C3_HC | | | | | | | | | | | | | | | | | | | | | | | | | | |
| 21-225_157H8_HC | | | | | | | | | | | | | | | | | | | | | | | | | | |
| 21-225_158H6_HC | | | V | | | | | W | | | | | | | | | | | | | | | | | | |
| 21-225_159C5_HC | | | | | | | | | | | | | | | | | | | | | | | | | | |
| 21-225_15E3_HC | | | | | | | | | | | | | | | | | | | | | | | | | | |
| 21-225_16A5_HC | | | | | D | | | W | | | | | | | | | | | | | | | | | | |
| 21-225_16B7_HC | | | | | | | | | | | | | | | | | | | | | | | | | | |
| 21-225_16F10_HC | | | | | | | A | W | | | | | | | | | | | | | | | | | | |
| 21-225_16F11_HC | | | V | | | | | | | | | | | | | | | | | | | | | | | |
| 21-225_16F4_HC | | | | | | | | | | | | | | | | | | | | | | | | | | |
| 21-225_16F6_HC | | | | | | | | | | | | | | | | | | | | | | | | | | |
| 21-225_16G7_HC | | | | | | | A | W | | | | | | | | | | | | | | | | | | |
| 21-225_17A10_HC | | | | | | | | | | | | | | | | | | | | | | | | | | |
| 21-225_17B5_HC | | | V | | | | | | | | | | | | | | | | | | | | | | | |
| 21-225_17C7_HC | | | | | D | | | W | | | | | | | | | | | | | | | | | | |
| 21-225_17E5_HC | | | | | | | | W | | | | | | | | | | | | | | | | | | |
| 21-225_18A1_HC | | | V | | | | | | | | | | | | | | | | | | | | | | | |
| 21-225_18A5_HC | | | | | D | | | W | | | | | | | | | | | | | | | | | | |
| 21-225_18C2_HC | | | | | | | | W | | | | | | | | | | | | | | | | | | |
| 21-225_18E8_HC | | | | | | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | L | G | F | - | - | - | - | - | - | - | - | - | - | - | - | - | - | - | - | - | - |
| 21-225_18F2_HC | | | | | D | | | W | | | | | | | | | | | | | | | | | | |
| 21-225_19E1_HC | | | | | | | | | | | | | | | | | | | | | | | | | | |
| 21-225_1C12_HC | | | | | | | | | | | | | | | | | | | | | | | | | | |
| 21-225_203B9_HC | | | | | | | | | | | | | | | | | | | | | | | | | | |
| 21-225_207F12_HC | | | | | | | | | | | | | | | | | | | | | | | | | | |
| 21-225_20A7_HC | | | | | | | | | | | | | | | | | | | | | | | | | | |
| 21-225_20D10_HC | | | | | D | | | W | | | | | | | | | | | | | | | | | | |
| 21-225_20D5_HC | | | | | | | | | | | | | | | | | | | | | | | | | | |
| 21-225_20E12_HC | | | | | | | | | | | | | | | | | | | | | | | | | | |
| 21-225_20E5_HC | | | | | | | | | | | | | | | | | | | | | | | | | | |
| 21-225_20H12_HC | | | | | | | | | | | | | | | | | | | | | | | | | | |
| 21-225_21E5_HC | | | T | | | | | | | | | | | | | | | | | | | | | | | |
| 21-225_21F7_HC | | | | | D | | | W | | | | | | | | | | | | | | | | | | |
| 21-225_21G11_HC | | | | | | | | W | | | | | | | | | | | | | | | | | | |
| 21-225_21G2_HC | | | | | | | | W | | | | | | | | | | | | | | | | | | |
| 21-225_21H8_HC | | | | | D | | | W | | | | | | | | | | | | | | | | | | |
| 21-225_224D10_HC | | | V | | | | | | | | | | | | | | | | | | | | | | | |
| 21-225_22A4_HC | | | | | D | | | W | | | | | | | | | | | | | | | | | | |
| 21-225_22F2_HC | | | V | | | | | | | | | | | | | | | | | | | | | | | |
| 21-225_22F9_HC | | | V | | | | | | | | | | | | | | | | | | | | | | | |
| 21-225_22H8_HC | | | | | | | A | W | | | | | | | | | | | | | | | | | | |
| 21-225_23F12_HC | | | | | | | A | W | | | | | | | | | | | | | | | | | | |
| 21-225_23H11_HC | | | | | | | | | | | | | | | | | | | | | | | | | | |
| 21-225_25B6_HC | | | | | | | | | | | | | | | | | | | | | | | | | | |
| 21-225_25E6_HC | | | | T | | | | M | | | | | | | | | | | | | | | | | | |
| 21-225_25H10_HC | | | | | | | | M | | | | | | | | | | | | | | | | | | |
| 21-225_25H11_HC | | | | | | | | M | | | | | | | | | | | | | | | | | | |
| 21-225_26A2_HC | | | | | | | | W | | | | | | | | | | | | | | | | | | |
| 21-225_27C3_HC | | | | | | | | M | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | L | G | F | - | - | - | - | - | - | - | - | - | - | - | - | - | - | - | - | - | - |
| 21-225_27D5_HC | . | . | . | . | . | . | . | . | . | . | . | . | . | . | . | . | . | . | . | . | . | . | . | . | . | . |
| 21-225_27F2_HC | . | . | . | . | . | . | . | M | . | . | . | . | . | . | . | . | . | . | . | . | . | . | . | . | . | . |
| 21-225_28C7_HC | . | . | . | . | . | . | . | W | . | . | . | . | . | . | . | . | . | . | . | . | . | . | . | . | . | . |
| 21-225_28F5_HC | . | . | . | . | . | . | . | W | . | . | . | . | . | . | . | . | . | . | . | . | . | . | . | . | . | . |
| 21-225_29B8_HC | . | . | . | . | . | . | . | M | . | . | . | . | . | . | . | . | . | . | . | . | . | . | . | . | . | . |
| 21-225_29D7_HC | . | . | . | . | . | . | . | . | . | . | . | . | . | . | . | . | . | . | . | . | . | . | . | . | . | . |
| 21-225_29G4_HC | . | . | . | . | . | . | . | M | . | . | . | . | . | . | . | . | . | . | . | . | . | . | . | . | . | . |
| 21-225_29G8_HC | . | . | . | . | . | . | . | M | . | . | . | . | . | . | . | . | . | . | . | . | . | . | . | . | . | . |
| 21-225_29H6_HC | . | . | . | . | . | . | . | M | . | . | . | . | . | . | . | . | . | . | . | . | . | . | . | . | . | . |
| 21-225_2B2_HC | . | . | V | . | . | . | . | . | . | . | . | . | . | . | . | . | . | . | . | . | . | . | . | . | . | . |
| 21-225_43C3_HC | . | . | . | . | . | . | . | . | . | . | . | . | . | . | . | . | . | . | . | . | . | . | . | . | . | . |
| 21-225_44C3_HC | . | . | V | . | . | . | . | . | . | . | . | . | . | . | . | . | . | . | . | . | . | . | . | . | . | . |
| 21-225_44C8_HC | . | . | V | . | . | . | . | . | . | . | . | . | . | . | . | . | . | . | . | . | . | . | . | . | . | . |
| 21-225_46C6_HC | . | . | . | . | . | . | . | . | . | . | . | . | . | . | . | . | . | . | . | . | . | . | . | . | . | . |
| 21-225_46D8_HC | . | . | V | . | . | . | . | . | . | . | . | . | . | . | . | . | . | . | . | . | . | . | . | . | . | . |
| 21-225_46F3_HC | . | . | V | . | . | . | . | . | . | . | . | . | . | . | . | . | . | . | . | . | . | . | . | . | . | . |
| 21-225_47A1_HC | . | . | . | T | . | . | . | M | . | . | . | . | . | . | . | . | . | . | . | . | . | . | . | . | . | . |
| 21-225_48A9_HC | F | . | . | . | . | . | A | W | . | . | . | . | . | . | . | . | . | . | . | . | . | . | . | . | . | . |
| 21-225_48C1_HC | . | . | . | . | . | . | . | W | . | . | . | . | . | . | . | . | . | . | . | . | . | . | . | . | . | . |
| 21-225_48D12_HC | . | . | . | . | . | . | . | M | . | . | . | . | . | . | . | . | . | . | . | . | . | . | . | . | . | . |
| 21-225_49A1_HC | . | . | . | . | . | . | . | . | . | . | . | . | . | . | . | . | . | . | . | . | . | . | . | . | . | . |
| 21-225_49C6_HC | . | . | . | . | D | . | . | M | . | . | . | . | . | . | . | . | . | . | . | . | . | . | . | . | . | . |
| 21-225_51B11_HC | . | . | . | . | . | . | . | . | . | . | . | . | . | . | . | . | . | . | . | . | . | . | . | . | . | . |
| 21-225_51E4_HC | . | . | . | . | . | . | . | . | . | . | . | . | . | . | . | . | . | . | . | . | . | . | . | . | . | . |
| 21-225_51F11_HC | . | . | . | . | . | . | . | . | . | . | . | . | . | . | . | . | . | . | . | . | . | . | . | . | . | . |
| 21-225_51F9_HC | . | . | . | . | . | . | . | . | . | . | . | . | . | . | . | . | . | . | . | . | . | . | . | . | . | . |
| 21-225_52B2_HC | . | . | . | . | D | . | . | M | . | . | . | . | . | . | . | . | . | . | . | . | . | . | . | . | . | . |
| 21-225_52B9_HC | . | . | . | . | . | . | . | . | . | . | . | . | . | . | . | . | . | . | . | . | . | . | . | . | . | . |
| 21-225_53B12_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | L | G | F | - | - | - | - | - | - | - | - | - | - | - | - | - | - | - | - | - | - |
| 21-225_9E8_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | | S | D | Y | W | G | Q | G | T | L | V | T | V | S | S |
| 21-225_10G5_HC | . | . | . | . | R | . | . | . | . | . | . | . | . | . | . | . | . | . | . |
| 21-225_10H12_HC | . | . | . | . | R | . | . | . | . | . | . | . | . | . | . | . | . | . | . |
| 21-225_12A1_HC | . | . | . | . | R | . | . | . | . | . | . | . | . | . | . | . | . | . | . |
| 21-225_12B12_HC | . | . | . | . | Y | E | . | . | . | . | . | . | . | . | . | . | . | . | . |
| 21-225_12E6_HC | . | . | . | . | Y | E | . | . | . | . | . | . | . | . | . | . | . | . | . |
| 21-225_12F2_HC | . | . | . | . | S | E | . | . | . | . | . | . | . | . | . | . | . | . | . |
| 21-225_13E6_HC | . | . | . | . | R | . | . | . | . | . | . | . | . | . | . | . | . | . | . |
| 21-225_146H9_HC | . | . | . | . | L | . | . | . | . | . | . | . | . | . | . | . | . | . | . |
| 21-225_14E3_HC | . | . | . | . | R | . | . | . | . | . | . | . | . | . | . | . | . | . | . |
| 21-225_157C3_HC | . | . | . | . | L | . | . | . | . | . | . | . | . | . | . | . | . | . | . |
| 21-225_157H8_HC | . | . | . | . | L | . | . | . | . | . | . | . | . | . | . | . | . | . | . |
| 21-225_158H6_HC | . | . | . | . | A | E | . | . | . | . | . | . | . | . | . | . | . | . | . |
| 21-225_159C5_HC | . | . | . | . | L | . | . | . | . | . | . | . | . | . | . | . | . | . | . |
| 21-225_15E3_HC | . | . | . | . | L | . | . | . | . | . | . | . | . | . | . | . | . | . | . |
| 21-225_16A5_HC | . | . | . | . | T | E | E | . | . | . | . | . | . | . | . | . | . | . | . |
| 21-225_16B7_HC | . | . | . | . | L | . | . | . | . | . | . | . | . | . | . | . | . | . | . |
| 21-225_16F10_HC | . | . | . | . | Y | E | . | . | . | . | . | . | . | . | . | . | . | . | . |
| 21-225_16F11_HC | . | . | . | . | Q | . | . | . | . | . | . | . | . | P | . | . | . | . | . |
| 21-225_16F4_HC | . | . | . | . | L | . | . | . | . | . | . | . | . | . | . | . | . | . | . |
| 21-225_16F6_HC | . | . | . | . | L | . | . | . | . | . | . | . | . | . | . | . | . | . | . |
| 21-225_16G7_HC | . | . | . | . | Y | E | . | . | . | . | . | . | . | . | . | . | . | . | . |
| 21-225_17A10_HC | . | . | . | . | R | . | . | . | . | . | . | . | . | . | . | . | . | . | . |
| 21-225_17B5_HC | . | . | . | . | R | F | . | . | . | . | . | . | . | . | . | . | . | . | . |
| 21-225_17C7_HC | . | . | . | . | T | E | E | . | . | . | . | . | . | . | . | . | . | . | . |
| 21-225_17E5_HC | . | . | . | . | Y | E | . | . | . | . | . | . | . | . | . | . | . | . | . |
| 21-225_18A1_HC | . | . | . | . | Q | . | . | . | . | . | . | . | . | P | . | . | . | . | . |
| 21-225_18A5_HC | . | . | . | . | T | E | E | . | . | . | . | . | . | . | . | . | . | . | . |
| 21-225_18C2_HC | . | . | . | . | Y | E | . | . | . | . | . | . | . | . | . | . | . | . | . |
| 21-225_18E8_HC | . | . | . | . | R | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  | H_FR4 | | | | | | | | | | |
| CONSENSUS | - | - | - | - | S | D | Y | W | G | Q | G | T | L | V | T | V | S | S |  |
| 21-225_18F2_HC |  |  |  |  | T | E | E |  |  |  |  |  |  |  |  |  |  |  |  |
| 21-225_19E1_HC |  |  |  |  | R |  |  |  |  |  |  |  |  |  |  |  |  |  |  |
| 21-225_1C12_HC |  |  |  |  | R |  |  |  |  |  |  |  |  |  |  |  |  |  |  |
| 21-225_203B9_HC |  |  |  |  | L |  |  |  |  |  |  |  | I |  |  |  |  |  |  |
| 21-225_207F12_HC |  |  |  |  | L | F |  |  |  |  |  |  |  |  |  |  |  |  |  |
| 21-225_20A7_HC |  |  |  |  | R |  |  |  |  |  |  |  |  |  |  |  |  |  |  |
| 21-225_20D10_HC |  |  |  |  | T | E | E |  |  |  |  |  |  |  |  |  |  |  |  |
| 21-225_20D5_HC |  |  |  |  | R |  |  |  |  |  |  |  |  |  |  |  |  |  |  |
| 21-225_20E12_HC |  |  |  |  | R | F |  |  |  |  |  |  |  |  |  |  |  |  |  |
| 21-225_20E5_HC |  |  |  |  | R |  |  |  |  |  |  |  |  |  |  |  |  |  |  |
| 21-225_20H12_HC |  |  |  |  | R |  |  |  |  |  |  |  |  |  |  |  |  |  |  |
| 21-225_21E5_HC |  |  |  |  | R |  |  |  |  |  |  |  |  |  |  |  |  |  |  |
| 21-225_21F7_HC |  |  |  |  | T | E | E |  |  |  |  |  |  |  |  |  |  |  |  |
| 21-225_21G11_HC |  |  |  |  | Y | E |  |  |  |  |  |  |  |  |  |  |  |  |  |
| 21-225_21G2_HC |  |  |  |  | Y | E |  |  |  |  |  |  |  |  |  |  |  |  |  |
| 21-225_21H8_HC |  |  |  |  | T | E | E |  |  |  |  |  |  |  |  |  |  |  |  |
| 21-225_224D10_HC |  |  |  |  | Q |  |  |  |  |  |  |  |  | P |  |  |  |  |  |
| 21-225_22A4_HC |  |  |  |  | T | E | E |  |  |  |  |  |  |  |  |  |  |  |  |
| 21-225_22F2_HC |  |  |  |  | Q |  |  |  |  |  |  |  |  | P |  |  |  |  |  |
| 21-225_22F9_HC |  |  |  |  | Q |  |  |  |  |  |  |  |  | P |  |  |  |  |  |
| 21-225_22H8_HC |  |  |  |  | Y | E |  |  |  |  |  |  |  |  |  |  |  |  |  |
| 21-225_23F12_HC |  |  |  |  | Y | E |  |  |  |  |  |  | S |  |  |  |  |  |  |
| 21-225_23H11_HC |  |  |  |  | R |  |  |  |  |  |  |  |  |  |  |  |  |  |  |
| 21-225_25B6_HC |  |  |  |  | L |  |  |  |  |  |  |  |  |  |  |  |  |  |  |
| 21-225_25E6_HC |  |  |  |  | T | G |  | S |  |  |  |  |  |  |  |  |  |  |  |
| 21-225_25H10_HC |  |  |  |  | T | G |  |  |  |  |  |  |  |  |  |  |  |  |  |
| 21-225_25H11_HC |  |  |  |  | T | G |  | S |  |  |  |  |  |  |  |  |  |  |  |
| 21-225_26A2_HC |  |  |  |  | Y | D |  |  |  |  |  |  |  |  |  |  |  |  |  |
| 21-225_27C3_HC |  |  |  |  | T | G |  | S |  |  |  |  |  |  |  |  |  |  |  |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | S | D | Y | | W | G | Q | G | T | L | V | T | V | S | S |
| 21-225_27D5_HC | . | . | . | . | L | . | . | . | . | . | . | . | . | . | . | . | . | . | . |
| 21-225_27F2_HC | . | . | . | . | T | G | . | S | . | . | . | . | . | . | . | . | . | . | . |
| 21-225_28C7_HC | . | . | . | . | Y | D | . | . | . | . | . | . | . | . | . | . | . | . | . |
| 21-225_28F5_HC | . | . | . | . | Y | D | . | . | . | . | . | . | . | . | . | . | . | . | . |
| 21-225_29B8_HC | . | . | . | . | T | G | . | S | . | . | . | . | . | . | . | . | . | . | . |
| 21-225_29D7_HC | . | . | . | . | L | . | . | . | . | . | . | . | . | . | . | . | . | . | . |
| 21-225_29G4_HC | . | . | . | . | T | G | . | . | . | . | . | . | . | . | . | . | . | . | . |
| 21-225_29G8_HC | . | . | . | . | T | . | . | . | . | . | . | . | . | . | . | . | . | . | . |
| 21-225_29H6_HC | . | . | . | . | T | G | . | S | . | . | . | . | . | . | . | . | . | . | . |
| 21-225_2B2_HC | . | . | . | . | Q | . | . | . | . | . | . | . | . | P | . | . | . | . | . |
| 21-225_43C3_HC | . | . | . | . | S | N | . | . | . | . | . | . | . | . | . | . | . | . | . |
| 21-225_44C3_HC | . | . | . | . | S | T | . | . | . | . | . | . | . | . | . | . | . | . | . |
| 21-225_44C8_HC | . | . | . | . | L | . | . | . | . | . | . | . | . | . | . | . | . | . | . |
| 21-225_46C6_HC | . | . | . | . | S | N | . | . | . | . | . | . | . | . | . | . | . | . | . |
| 21-225_46D8_HC | . | . | . | . | S | N | . | . | . | . | . | . | . | . | . | . | . | . | . |
| 21-225_46F3_HC | . | . | . | . | S | N | . | . | . | . | . | . | . | . | . | . | . | . | . |
| 21-225_47A1_HC | . | . | . | . | L | F | . | . | . | . | . | . | . | . | . | . | . | . | . |
| 21-225_48A9_HC | . | . | . | . | Y | E | . | . | . | . | . | . | . | . | . | . | . | . | . |
| 21-225_48C1_HC | . | . | . | . | E | A | . | . | . | . | . | . | . | . | . | . | . | . | . |
| 21-225_48D12_HC | . | . | . | . | R | . | . | . | . | . | . | . | . | . | . | . | . | . | . |
| 21-225_49A1_HC | . | . | . | . | L | . | . | . | . | . | . | . | . | . | . | . | . | . | . |
| 21-225_49C6_HC | . | . | . | . | I | E | . | . | . | . | . | . | . | . | . | . | . | . | . |
| 21-225_51B11_HC | . | . | . | . | L | . | . | . | . | . | . | . | . | . | . | . | . | . | . |
| 21-225_51E4_HC | . | . | . | . | L | . | . | . | . | . | . | . | . | . | . | . | . | . | . |
| 21-225_51F11_HC | . | . | . | . | L | . | . | F | . | . | . | . | . | . | . | . | . | . | . |
| 21-225_51F9_HC | . | . | . | . | L | . | . | . | . | . | . | . | . | . | . | . | . | . | . |
| 21-225_52B2_HC | . | . | . | . | I | E | . | F | . | . | . | . | . | . | . | . | . | . | . |
| 21-225_52B9_HC | . | . | . | . | L | . | . | . | . | . | . | . | . | . | . | . | . | . | . |
| 21-225_53B12_HC | . | . | . | . | L | . | . | F | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | | S | D | Y | W | G | Q | G | T | L | V | T | V | S | S |
| 21-225_53D2_HC | . | . | . | . | T | G | . | . | . | . | . | . | A | . | . | . | . | . | . |
| 21-225_53E6_HC | . | . | . | . | T | . | . | . | . | . | . | . | . | . | . | . | . | . | . |
| 21-225_54G6_HC | . | . | . | . | T | . | . | . | . | . | . | . | . | . | . | . | . | . | . |
| 21-225_54D3_HC | . | . | . | . | L | . | . | . | . | . | . | . | . | . | . | . | . | . | . |
| 21-225_54G7_HC | . | . | . | . | L | . | . | . | . | . | . | . | . | . | . | . | . | . | . |
| 21-225_55B3_HC | . | . | . | . | L | . | . | . | . | . | . | . | . | . | . | . | . | . | . |
| 21-225_57A4_HC | . | . | . | . | R | . | . | . | . | . | . | . | . | . | . | . | . | . | . |
| 21-225_58F5_HC | . | . | . | . | R | . | . | . | . | . | . | . | . | . | . | . | . | . | . |
| 21-225_59F11_HC | . | . | . | . | N | G | . | . | . | . | . | . | . | . | . | . | . | . | . |
| 21-225_60A3_HC | . | . | . | . | T | G | . | . | . | . | . | . | . | . | . | . | . | . | . |
| 21-225_62E4_HC | . | . | . | . | S | . | . | . | . | . | . | . | . | . | . | . | . | . | . |
| 21-225_64E1_HC | . | . | . | . | S | . | . | . | . | . | . | . | . | . | . | . | . | . | . |
| 21-225_65C1_HC | . | . | . | . | S | . | . | . | . | . | . | . | . | . | . | . | . | . | . |
| 21-225_66C7_HC | . | . | . | . | S | . | . | . | . | . | . | . | . | . | . | . | . | . | . |
| 21-225_68F11_HC | . | . | . | . | T | . | . | C | . | . | . | . | . | . | . | . | . | . | . |
| 21-225_6A11_HC | . | . | . | . | T | E | E | . | . | . | . | . | . | . | . | . | . | . | . |
| 21-225_6G12_HC | . | . | . | . | R | . | . | . | . | . | . | . | . | . | . | . | . | . | . |
| 21-225_6G2_HC | . | . | . | . | T | E | E | . | . | . | . | . | . | . | . | . | . | . | . |
| 21-225_71A2_HC | . | . | . | . | Q | D | . | . | . | . | . | . | . | . | . | . | . | . | . |
| 21-225_71B11_HC | . | . | . | . | L | . | . | . | . | . | . | . | . | . | . | . | . | . | . |
| 21-225_71B6_HC | . | . | . | . | L | . | . | . | . | . | . | . | . | . | . | . | . | . | . |
| 21-225_72G12_HC | . | . | . | . | S | . | . | . | . | . | . | . | . | . | . | . | . | . | . |
| 21-225_7C4_HC | . | . | . | . | R | . | . | . | . | . | . | . | . | . | . | . | . | . | . |
| 21-225_7E9_HC | . | . | . | . | R | . | . | . | . | . | . | . | . | . | . | . | . | . | . |
| 21-225_7G2_HC | . | . | . | . | Y | E | . | . | . | . | . | . | . | . | . | . | . | . | . |
| 21-225_7H11_HC | . | . | . | . | R | . | . | . | . | . | . | . | . | . | . | . | . | . | . |
| 21-225_8C9_HC | . | . | . | . | R | . | . | . | . | . | . | . | . | . | . | . | . | . | . |
| 21-225_8H7_HC | . | . | . | . | R | . | . | . | . | . | . | . | . | . | . | . | . | . | . |
| 21-225_9A1_HC | . | . | . | . | T | E | E | . | . | . | . | . | . | P | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | | S | D | Y | W | G | Q | G | T | L | V | T | V | S | S |
| 21-225_9E8_HC | | | | | R | | | | | | | | | | | | | | |

FIGURE 57

Table 52. Consensus 18- VH4|4-34/D4|4-17|RF2/JH6 (SEQ ID NO: 50269):

QVQLQQW-GAGLLKPSETLSLTCAVHG-GSFSG-----CYWSWIRQPPGKGLEWIGEINH----
SGSTNYNPSLKSRVTISVDTSKNQFSLKLSSVTAADTAVYYCARDYGG---------------------MDVWGQGTTVTVSS wherein:

C at position 39 can be substituted with S, P or Y

H at position 60 can be substituted with Y or Q

S at position 65 can be substituted with R

S at position 67 can be substituted with R, C, or I

N at position 69 can be substituted with S

Y at position 70 can be substituted with F

K at position 75 can be substituted with T

M at position 136 can be substituted with L or I

Gly Xaa Tyr Trp Ser (SEQ ID NO: 50010)

Glu Ile Asn Xaa Xaa Gly Xaa Thr Xaa Xaa Asn Pro Ser Leu Xaa Ser (SEQ ID NO: 50011)

Asp Tyr Gly Gly Xaa Asp Val (SEQ ID NO: 50012)

Gly Cys Tyr Trp Ser (SEQ ID NO: 50477)

Glu Ile Asn His Ser Gly Ser Thr Asn Tyr Asn Pro Ser Leu Lys Ser (SEQ ID NO: 50478)

Asp Tyr Gly Gly Met Asp Val (SEQ ID NO: 50479)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | Q | Q | W | - | G | A | G | L | L | K | P | S | E | T | L | S | L | T | C | A | V | H |
| 21-225_74A3_HC | . | . | . | . | . | . | . | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . | . | . | . | . | . | . | . |
| 21-225_74B2_HC | . | . | . | . | . | . | . | . | . | . | . | . | . | . | . | . | . | P | . | . | . | . | . | . | . | Y |
| 21-225_74C5_HC | . | . | . | . | . | . | . | . | . | . | . | . | . | . | . | . | . | . | . | . | . | . | . | . | . | Y |
| 21-225_74D11_HC | . | . | . | . | . | . | . | . | . | . | . | . | . | . | . | . | . | . | . | . | . | . | . | . | . | . |
| 21-225_74D5_HC | . | . | . | . | . | . | . | . | . | . | . | . | . | . | . | . | . | . | . | . | . | . | . | . | . | N |
| 21-225_74D6_HC | . | . | . | . | . | . | . | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . | . | . | . | . | . | . | . |
| 21-225_74H5_HC | . | . | . | . | . | . | . | . | . | . | . | . | . | . | . | . | . | . | . | . | . | . | . | . | . | N |
| 21-225_74H7_HC | . | . | . | . | . | . | . | . | . | . | . | . | . | . | . | . | . | P | . | . | . | . | . | . | . | Y |
| 21-225_75A12_HC | . | . | . | . | . | . | G | . | . | . | . | P | . | . | . | . | . | . | . | . | . | . | . | . | . | Y |
| 21-225_75A5_HC | . | . | . | . | . | . | . | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . | . | . | . | . | . | . | . |
| 21-225_75B10_HC | . | . | . | . | . | . | . | . | . | . | . | . | . | . | . | . | . | . | . | . | . | K | . | D | . | Y |
| 21-225_75C2_HC | . | . | . | . | . | . | . | . | . | . | . | . | . | . | . | . | . | . | . | . | . | . | . | . | . | . |
| 21-225_75C3_HC | . | . | . | . | . | . | G | . | . | . | . | P | . | . | . | . | . | . | . | . | . | . | . | . | . | Y |
| 21-225_75D3_HC | . | . | . | . | . | . | . | . | . | . | . | . | . | . | . | . | . | . | . | . | . | . | . | . | . | . |
| 21-225_75D9_HC | . | . | . | . | . | . | . | . | . | . | . | . | . | . | . | . | . | . | . | . | . | . | . | . | . | Y |
| 21-225_75F3_HC | . | . | . | . | . | . | . | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . | . | . | . | . | . | . | . |
| 21-225_75G7_HC | . | . | . | . | . | . | . | . | . | . | . | . | . | . | . | . | . | . | . | . | . | . | . | T | . | Y |
| 21-225_75H8_HC | . | . | . | . | . | . | . | . | . | . | . | . | . | . | . | . | . | P | . | . | . | . | . | . | . | Y |
| 21-225_76A4_HC | . | . | . | . | . | . | . | . | . | . | . | . | . | . | . | . | . | . | . | . | . | . | . | . | . | . |
| 21-225_76D1_HC | . | . | . | . | . | . | . | . | . | . | . | . | . | . | . | . | . | . | . | . | . | . | . | . | . | Y |
| 21-225_76E10_HC | . | . | . | . | . | . | . | . | . | . | . | . | . | . | . | . | . | . | . | . | . | . | . | . | . | N |
| 21-225_76G4_HC | . | . | . | . | . | . | . | . | . | . | . | . | . | . | . | . | . | . | . | . | . | . | . | . | . | . |
| 21-225_76H10_HC | . | . | . | . | . | . | . | . | . | . | . | . | . | . | . | . | . | . | . | . | . | . | . | . | . | N |
| 21-225_77A10_HC | . | . | . | . | . | . | G | . | . | . | . | P | . | . | . | . | . | . | . | . | . | . | . | . | . | Y |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | Q | Q | W | - | G | A | G | L | L | K | P | S | E | T | L | S | L | T | C | A | V | H |
| 21-225_77C12_HC | . | . | . | . | . | . | . | . | . | . | . | . | . | . | . | . | . | . | . | . | . | . | . | . | . | Y |
| 21-225_77C5_HC | . | . | . | . | . | . | . | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . | . | . | . | . | . | . | . |
| 21-225_80D5_HC | . | . | . | . | . | . | . | . | . | . | . | . | . | . | . | . | . | P | . | . | . | . | . | . | . | Y |
| 21-225_80E12_HC | . | . | . | . | . | . | . | . | . | . | . | . | . | . | . | . | . | . | . | . | . | . | . | . | . | N |
| 21-225_80E3_HC | . | . | . | . | . | . | . | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . | . | . | . | . | . | . | . |
| 21-225_80H7_HC | . | . | . | . | . | . | . | . | . | . | . | . | . | . | . | . | . | . | . | . | . | . | . | . | . | Y |
| 21-225_81C11_HC | . | . | . | . | . | . | . | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . | . | . | . | . | . | . | . |
| 21-225_84E12_HC | . | . | . | . | . | . | . | . | . | . | . | . | . | . | . | . | . | . | . | . | . | . | . | . | . | Y |
| 21-225_85A3_HC | . | . | . | . | . | . | . | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . | . | . | . | . | . | . | . |
| 21-225_85B9_HC | . | . | . | . | . | . | . | . | . | . | . | . | . | . | . | . | . | . | . | . | . | . | . | . | . | N |
| 21-225_85D6_HC | . | . | . | . | . | . | G | . | . | . | . | P | . | . | . | . | . | . | . | . | . | . | . | . | . | Y |
| 21-225_85G10_HC | . | . | . | . | . | . | R | . | . | . | . | . | . | . | . | . | . | . | . | . | . | . | . | . | . | Y |
| 21-225_86C1_HC | . | . | . | . | . | . | . | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | Q | Q | W | - | G | A | G | L | L | K | P | S | E | T | L | S | L | T | C | A | V | H |
| 21-225_87C9_HC | . | | | | | | | | | | | | | | | | | | | | | | | | | |
| 21-225_87E5_HC | . | | | | | | | | | | | | | | | | | | | | | | | | | |
| 21-225_87H1_HC | . | | | | | P | | | | | | | | | | | | | | | | | | | | Y |
| 21-225_88E7_HC | . | | | | | | | | | | | | | | | | | | | | | | | | | |
| 21-225_88F7_HC | . | | | | | | | | | | | | | | | | | | | | | | | | | |
| 21-225_88G9_HC | . | | | | | | | | | | | | | | | | | | | | | | | | | |
| 21-225_88H1_HC | . | | | | | | | | | | | | | | | | | | | | | | | | | |
| 21-225_89A11_HC | . | | | | | P | | | | | | | | | | | | | | | | | | | | Y |
| 21-225_89D5_HC | . | | | | | | | | | | | | | | | | | | | | | | | | | |
| 21-225_89E10_HC | . | | | | | | | | | | | | | | | | | | | | | | | | | |
| 21-225_89H5_HC | . | | | | | | | | | | | | | | | | | | | | | | | | | Y |
| 21-225_90A5_HC | . | | | | | | | | | | | | | | | | | | | | | | | | | |
| 21-225_90F10_HC | . | | | | | | | | | | | | | | | | | | | | | | | | | |
| 21-225_90G4_HC | . | | | | | | | | | | | | | | | | | | | | | | | | | |
| 21-225_90G5_HC | . | | | | | | | | | | | | | | | | | | | | | | | | | |
| 21-225_91B2_HC | . | | | | | | | | | | | | | | | | | | | | | | | | | |
| 21-225_91B3_HC | . | | | | | | | | | | | | | | | | | | | | | | | | | |
| 21-225_91B4_HC | . | | | | | | | | | | | | | | | | | | | | | | | | | |
| 21-225_91E9_HC | . | | | | | | | | | | | | | | | | | | | | | | | | | |
| 21-225_91F3_HC | . | | | | | | | | | | | | | | | | | | | | | | | | | |
| 21-225_92B1_HC | . | | | | | | | | | | | | | | | | | | | | | | | | | Y |
| 21-225_92D6_HC | . | | | | | | | | | | | | | | | | | | | | | | | | | |
| 21-225_93C2_HC | . | | | | | | | | | | | | | | | | | | | | | | | | | |
| 21-225_93E4_HC | . | | | | | | | | | | | | | | | | | | | | | | | | | |
| 21-225_94D3_HC | . | | | | | | G | | | | | P | | | | | | | | | | | | | | Y |
| 21-225_94F12_HC | . | | | | | | | | | | | | | | | | | | | | | | | | | |
| 21-225_94G10_HC | . | | | | | | | | | | | | | | | | | | | | | | | | | N |
| 21-225_95D2_HC | . | | | | | | | | | | | | | | | | | | | | | | | | | |
| 21-225_95F9_HC | . | | | | | | | | | | | | | | | | | | | | | | | | | |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | Q | Q | W | - | G | A | G | L | L | K | P | S | E | T | L | S | L | T | C | A | V | H |
| 21-225_95G9_HC | . | . | . | . | . | . | . | . | . | . | . | . | . | . | . | . | . | . | . | . | . | . | . | . | . | . |
| 21-225_95H10_HC | . | . | . | . | . | . | . | . | . | . | . | . | . | . | . | . | . | . | . | . | . | . | . | . | . | . |
| 21-225_97A2_HC | . | . | . | . | . | . | . | . | . | . | . | . | . | . | . | . | . | . | . | . | . | . | . | . | . | . |
| 21-225_97E5_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | G | S | F | S | G | - | - | - | - | - | C | Y | W | S | W | I | R | Q | P | P | G | K | G | L |
| 21-225_74A3_HC | . | . | . | . | . | . | . | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . | . | . | . | . | . | . | . |
| 21-225_74B2_HC | . | . | . | . | . | . | . | . | . | . | . | . | P | . | . | . | . | . | . | . | . | . | . | . | . | . |
| 21-225_74C5_HC | . | . | . | . | . | . | . | . | . | . | . | . | . | . | . | . | . | . | . | . | . | . | . | . | . | . |
| 21-225_74D11_HC | . | . | . | . | . | . | . | . | . | . | . | . | . | . | . | . | . | . | . | . | . | . | . | . | . | . |
| 21-225_74D5_HC | . | . | . | P | . | . | . | . | . | . | . | . | . | . | . | . | . | . | . | . | . | . | . | . | . | . |
| 21-225_74D6_HC | . | . | . | . | . | . | . | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . | . | . | . | . | . | . | . |
| 21-225_74H5_HC | . | . | . | P | . | . | . | . | . | . | . | . | . | . | . | . | . | . | . | . | . | . | . | . | . | . |
| 21-225_74H7_HC | . | . | . | . | . | . | . | . | . | . | . | . | P | . | . | . | . | . | . | . | . | . | . | . | . | . |
| 21-225_75A12_HC | . | . | . | . | . | . | . | . | . | . | . | . | . | . | . | . | . | . | . | . | . | . | . | . | . | . |
| 21-225_75A5_HC | . | . | . | . | . | . | . | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . | . | . | . | . | . | . | . |
| 21-225_75B10_HC | . | . | . | . | . | . | . | . | . | . | . | . | Y | . | . | . | . | . | . | . | . | . | . | . | . | . |
| 21-225_75C2_HC | . | . | . | . | . | . | . | . | . | . | . | . | . | . | . | . | . | . | . | . | . | . | . | . | . | . |
| 21-225_75C3_HC | . | . | . | . | . | . | . | . | . | . | . | . | . | . | . | . | . | . | . | . | . | . | . | . | . | . |
| 21-225_75D3_HC | . | . | . | . | . | . | . | . | . | . | . | . | . | . | . | . | . | . | . | . | . | . | . | . | . | . |
| 21-225_75D9_HC | . | . | . | . | . | . | . | . | . | . | . | . | P | . | . | . | . | . | . | . | . | . | . | . | . | . |
| 21-225_75F3_HC | . | . | . | . | . | . | . | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . | . | . | . | . | . | . | . |
| 21-225_75G7_HC | . | . | . | A | . | . | . | . | . | . | . | . | S | . | . | . | . | . | . | . | S | . | . | . | . | . |
| 21-225_75H8_HC | . | . | . | . | . | . | . | . | . | . | . | . | P | . | . | . | . | . | . | . | . | . | . | . | . | . |
| 21-225_76A4_HC | . | . | . | . | . | . | . | . | . | . | . | . | . | . | . | . | . | . | . | . | . | . | . | . | . | . |
| 21-225_76D1_HC | S | . | . | . | . | . | . | . | . | . | . | . | . | . | . | . | . | . | . | . | . | . | . | . | . | . |
| 21-225_76E10_HC | . | . | . | P | . | . | . | . | . | . | . | . | S | . | . | . | . | . | . | . | . | . | . | . | . | . |
| 21-225_76G4_HC | . | . | . | . | . | . | . | . | . | . | . | . | . | . | . | . | . | . | . | . | . | . | . | . | . | . |
| 21-225_76H10_HC | . | . | . | P | . | . | . | . | . | . | . | . | . | . | . | . | . | . | . | . | . | . | . | . | . | . |
| 21-225_77A10_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | G | S | F | S | G | - | - | - | - | - | C | Y | W | S | W | I | R | Q | P | P | G | K | G | L |
| 21-225_77C12_HC | . | . | . | A | . | . | . | . | . | . | . | . | S | . | . | . | . | . | . | . | S | . | . | . | . | . |
| 21-225_77C5_HC | . | . | . | . | . | . | . | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . | . | . | . | . | . | . | . |
| 21-225_80D5_HC | . | . | . | . | . | . | . | . | . | . | . | . | P | . | . | . | . | . | . | . | . | . | . | . | . | . |
| 21-225_80E12_HC | . | . | . | P | . | . | . | . | . | . | . | . | . | . | . | . | . | . | . | . | . | . | . | . | . | . |
| 21-225_80E3_HC | . | . | . | . | . | . | . | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . | . | . | . | . | . | . | . |
| 21-225_80H7_HC | V | . | . | . | . | . | . | . | . | . | . | . | S | . | . | . | . | . | . | . | . | . | . | . | . | . |
| 21-225_81C11_HC | . | . | . | . | . | . | . | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . | . | . | . | . | . | . | . |
| 21-225_84E12_HC | . | . | . | . | . | . | . | . | . | . | . | . | S | . | . | . | . | . | . | . | . | . | . | . | . | . |
| 21-225_85A3_HC | . | . | . | . | . | . | . | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . | . | . | . | . | . | . | . |
| 21-225_85B9_HC | . | . | . | P | . | . | . | . | . | . | . | . | . | . | . | . | . | . | . | . | . | . | . | . | . | . |
| 21-225_85D6_HC | . | . | . | . | . | . | . | . | . | . | . | . | . | . | . | . | . | . | . | . | . | . | . | . | . | . |
| 21-225_85G10_HC | . | . | . | . | . | . | . | . | . | . | . | . | . | . | . | . | . | . | . | . | . | . | . | . | . | . |
| 21-225_86C1_HC | . | . | . | . | . | . | . | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | G | S | F | S | G | - | - | - | - | - | C | Y | W | S | W | I | R | Q | P | P | G | K | G | L |
| 21-225_87C9_HC | . | . | . | . | . | . | . | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . | . | . | . | . | . | . | . |
| 21-225_87H1_HC | . | . | . | . | . | . | . | . | . | . | . | . | Y | . | . | . | . | . | . | . | . | . | . | . | . | . |
| 21-225_88E7_HC | . | . | . | . | . | . | . | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . | . | . | . | . | . | . | . |
| 21-225_89A11_HC | . | . | . | . | . | . | . | . | . | . | . | . | Y | . | . | . | . | . | . | . | . | . | . | . | . | . |
| 21-225_89D5_HC | . | . | . | . | . | . | . | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . | . | . | . | . | . | . | . |
| 21-225_89H5_HC | . | . | . | . | . | . | . | . | . | . | . | . | . | . | . | . | . | . | . | . | . | . | . | . | . | . |
| 21-225_90A5_HC | . | . | . | . | . | . | . | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . | . | . | . | . | . | . | . |
| 21-225_92B1_HC | . | . | . | . | . | . | . | . | . | . | . | . | S | . | . | . | . | . | . | . | . | . | . | . | . | . |
| 21-225_92D6_HC | . | . | . | . | . | . | . | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . | . | . | . | . | . | . | . |
| 21-225_94D3_HC | . | . | . | . | . | . | . | . | . | . | . | . | . | . | . | . | . | . | . | . | . | . | . | . | . | . |
| 21-225_94F12_HC | . | . | . | . | . | . | . | . | . | . | . | . | . | . | . | . | . | . | . | . | . | . | . | . | . | . |
| 21-225_94G10_HC | . | . | . | P | . | . | . | . | . | . | . | . | . | . | . | . | G | . | . | . | . | . | . | . | . | R |
| 21-225_95D2_HC | . | . | . | . | . | . | . | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | H_FR2 | | | | | | | | | |
| CONSENSUS | G | - | G | S | F | S | G | - | - | - | - | - | C | Y | W | S | W | I | R | Q | P | P | G | K | G | L |
| 21-225_95G9_HC | | | | | | | | | | | | | | | | | | | | | | | | | | |
| 21-225_95H10_HC | | | | | | | | | | | | | | | | | | | | | | | | | | |
| 21-225_97A2_HC | | | | | | | | | | | | | | | | | | | | | | | | | | |
| 21-225_97E5_HC | | | | | | | | | | | | | | | | | | | | | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | E | I | N | H | - | - | - | - | S | G | S | T | N | Y | N | P | S | L | K | S | R | V |
| 21-225_74A3_HC | . | . | . | . | . | . | . | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . | . | . | . | . | . | . | . |
| 21-225_74B2_HC | . | . | . | . | . | . | . | . | . | . | . | . | . | . | . | . | . | . | . | . | . | . | T | . | . | . |
| 21-225_74C5_HC | . | . | . | . | . | . | . | . | . | . | . | . | . | . | C | . | . | F | . | . | . | . | . | . | . | . |
| 21-225_74D11_HC | . | . | . | . | . | . | . | . | . | . | . | . | . | . | . | . | . | . | . | . | . | . | . | . | . | . |
| 21-225_74D5_HC | . | . | . | . | . | . | . | Y | . | . | . | . | . | . | R | . | . | F | . | . | . | . | . | . | . | . |
| 21-225_74D6_HC | . | . | . | . | . | . | . | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . | . | . | . | . | . | . | . |
| 21-225_74H5_HC | . | . | . | . | . | . | . | . | . | . | . | . | . | . | R | . | . | F | . | . | . | . | . | . | . | . |
| 21-225_74H7_HC | . | . | . | . | . | . | . | . | . | . | . | . | . | . | . | . | . | . | . | . | . | . | . | . | . | . |
| 21-225_75A12_HC | . | . | . | . | . | . | . | Y | . | . | . | . | . | . | R | . | . | . | . | . | . | . | . | . | . | . |
| 21-225_75A5_HC | . | . | . | . | . | . | . | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . | . | . | . | . | . | . | . |
| 21-225_75B10_HC | . | . | . | . | . | . | . | . | . | . | . | . | . | . | . | . | . | . | . | . | . | . | . | . | . | . |
| 21-225_75C2_HC | . | . | . | . | . | . | . | . | . | . | . | . | . | . | . | . | . | . | . | . | . | . | . | . | . | . |
| 21-225_75C3_HC | . | . | . | . | . | . | . | Y | . | . | . | . | . | . | R | . | . | . | . | . | . | . | . | . | . | . |
| 21-225_75D3_HC | . | . | . | . | . | . | . | . | . | . | . | . | . | . | . | . | . | . | . | . | . | . | . | . | . | . |
| 21-225_75D9_HC | . | . | . | . | . | . | . | Y | . | . | . | . | R | . | . | . | . | . | . | . | . | . | . | . | . | . |
| 21-225_75F3_HC | . | . | . | . | . | . | . | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . | . | . | . | . | . | . | . |
| 21-225_75G7_HC | . | . | . | . | . | . | . | Y | . | . | . | . | R | . | . | . | . | . | . | . | . | . | . | . | . | . |
| 21-225_75H8_HC | . | . | . | . | . | . | . | . | . | . | . | . | . | . | . | . | . | . | . | . | . | . | . | . | . | . |
| 21-225_76A4_HC | . | . | . | . | . | . | . | . | . | . | . | . | . | . | . | . | . | . | . | . | . | . | . | . | . | . |
| 21-225_76D1_HC | . | . | . | . | . | . | . | . | . | . | . | . | . | . | R | . | . | . | . | . | . | . | . | . | . | . |
| 21-225_76E10_HC | . | . | . | . | . | . | . | Y | . | . | . | . | . | . | R | . | . | F | . | . | . | . | . | . | . | . |
| 21-225_76G4_HC | . | . | . | . | . | . | . | . | . | . | . | . | . | . | . | . | . | . | . | . | . | . | . | . | . | . |
| 21-225_76H10_HC | . | . | . | . | . | . | . | Y | . | . | . | . | . | . | R | . | . | F | . | . | . | . | . | . | . | . |
| 21-225_77A10_HC | . | . | . | . | . | . | . | Y | . | . | . | . | . | . | R | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | E | I | N | H | - | - | - | - | S | G | S | T | N | Y | N | P | S | L | K | S | R | V |
| 21-225_77C12_HC | | | | | | | | Y | | | | | R | | | | | | | | | | | | | |
| 21-225_77C5_HC | | | | | | | | | | | | | | | | | | | | | | | | | | |
| 21-225_77F7_HC | | | | | | | | | | | | | | | | | | | | | | | | | | |
| 21-225_77G1_HC | | | | | | | | | | | | | | | | | | | | | | | | | | |
| 21-225_79F11_HC | | | | | | | | | | | | | | | | | | | | | | | | | | |
| 21-225_79F3_HC | | | | | | | | | | | | | | | | | | | | | | | | | | |
| 21-225_79G1_HC | | | | | | | | | | | | | | | | | | | | | | | | | | |
| 21-225_79G12_HC | | | | | | | | | | | | | | | | | | | | | | | | | | |
| 21-225_80A1_HC | | | | | | | | | | | | | | | | | | | | | | | | | | |
| 21-225_80C1_HC | | | | | | | | | | | | | | | | | | | | | | | | | | |
| 21-225_80D5_HC | | | | | | | | | | | | | | | | | | | | | | | | | | |
| 21-225_80E12_HC | | | | | | | | Y | | | | | | | R | | | F | | | | | | | | |
| 21-225_80E3_HC | | | | | | | | | | | | | | | | | | | | | | | | | | |
| 21-225_80H12_HC | | | | | | | | | | | | | | | | | | | | | | | | | | |
| 21-225_80H7_HC | | | | | | | | Q | | | | | | | R | | | | | | | | | | | |
| 21-225_81C11_HC | | | | | | | | | | | | | | | | | | | | | | | | | | |
| 21-225_81C5_HC | | | | | | | | | | | | | | | | | | | | | | | | | | |
| 21-225_83B7_HC | | | | | | | | | | | | | | | | | | | | | | | | | | |
| 21-225_83G1_HC | | | | | | | | | | | | | | | | | | | | | | | | | | |
| 21-225_84E12_HC | | | | | | | | Q | | | | | | | R | | | | | | | | | | | |
| 21-225_85A3_HC | | | | | | | | | | | | | | | | | | | | | | | | | | |
| 21-225_85B4_HC | | | | | | | | | | | | | | | | | | | | | | | | | | |
| 21-225_85B9_HC | | | | | | | | | | | | | | | R | | | F | | | | | | | | |
| 21-225_85D6_HC | | | | | | | | Y | | | | | | | R | | | | | | | | | | | |
| 21-225_85G10_HC | | | | | | | | | | | | | | | I | | | | | | | | | | | |
| 21-225_86C1_HC | | | | | | | | | | | | | | | | | | | | | | | | | | |
| 21-225_86C11_HC | | | | | | | | | | | | | | | | | | | | | | | | | | |
| 21-225_86E4_HC | | | | | | | | | | | | | | | | | | | | | | | | | | |
| 21-225_87A12_HC | | | | | | | | | | | | | | | | | | | | | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | E | I | N | H | - | - | - | - | S | G | S | T | N | Y | N | P | S | L | K | S | R | V |
| 21-225_87C9_HC | . | . | . | . | . | . | . | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . | . | . | . | . | . | . | . |
| 21-225_87H1_HC | . | . | . | . | . | . | . | . | . | . | . | . | . | . | R | . | . | . | . | . | . | . | . | . | . | . |
| 21-225_88E7_HC | . | . | . | . | . | . | . | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . | . | . | . | . | . | . | . |
| 21-225_89A11_HC | . | . | . | . | . | . | . | . | . | . | . | . | . | . | R | . | S | . | . | . | . | . | . | . | . | . |
| 21-225_89D5_HC | . | . | . | . | . | . | . | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . | . | . | . | . | . | . | . |
| 21-225_89H5_HC | . | . | . | . | . | . | . | Y | . | . | . | . | . | . | . | . | . | F | . | . | . | . | . | . | . | . |
| 21-225_90A5_HC | . | . | . | . | . | . | . | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . | . | . | . | . | . | . | . |
| 21-225_92B1_HC | . | . | . | . | . | . | . | Y | . | . | . | . | R | . | . | . | . | . | . | . | . | . | . | . | . | . |
| 21-225_92D6_HC | . | . | . | . | . | . | . | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . | . | . | . | . | . | . | . |
| 21-225_94D3_HC | . | . | . | . | . | . | . | Y | . | . | . | . | . | . | R | . | . | . | . | . | . | . | . | . | . | . |
| 21-225_94F12_HC | . | . | . | . | . | . | . | . | . | . | . | . | . | . | . | . | . | . | . | . | . | . | . | . | . | . |
| 21-225_94G10_HC | . | . | . | . | . | . | . | Y | . | . | . | . | . | . | R | . | . | F | . | . | . | . | . | . | . | . |
| 21-225_95D2_HC | . | . | . | . | . | . | . | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | I | G | E | I | N | H | - | - | - | - | S | G | S | T | N | Y | N | P | S | L | K | S | R | V |
| 21-225_95G9_HC | | | | | | | | | | | | | | | | | | | | | | | | | | |
| 21-225_95H10_HC | | | | | | | | | | | | | | | | | | | | | | | | | | |
| 21-225_97A2_HC | | | | | | | | | | | | | | | | | | | | | | | | | | |
| 21-225_97E5_HC | | | | | | | | | | | | | | | | | | | | | | | | | | |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | V | D | T | S | K | N | Q | F | S | L | K | L | S | S | V | T | A | A | D | T | A | V | Y |
| 21-225_74A3_HC | . | . | . | . | . | . | . | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . | . | . | . | . | . | . | . |
| 21-225_74B2_HC | . | . | . | . | . | . | . | . | . | . | . | . | . | . | . | T | . | . | . | . | . | . | S | . | . | . |
| 21-225_74C5_HC | . | . | . | . | . | . | . | . | . | . | . | . | . | . | . | . | . | . | . | . | . | . | . | . | . | . |
| 21-225_74D11_HC | . | . | . | . | . | . | . | . | . | . | . | . | . | . | . | . | . | . | . | . | . | . | . | . | . | . |
| 21-225_74D5_HC | . | . | . | . | . | . | . | . | . | . | . | . | . | . | . | . | . | . | . | . | . | . | . | . | . | . |
| 21-225_74D6_HC | . | . | . | . | . | . | . | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . | . | . | . | . | . | . | . |
| 21-225_74H5_HC | . | . | . | . | . | . | . | . | . | . | . | . | . | . | . | . | . | . | . | . | . | . | . | . | . | . |
| 21-225_74H7_HC | . | . | . | . | . | . | . | . | . | . | . | . | . | . | . | T | . | . | . | . | . | . | . | . | . | . |
| 21-225_75A12_HC | . | . | . | . | . | . | . | . | . | . | . | . | . | . | . | . | . | . | . | . | . | . | . | . | . | . |
| 21-225_75A5_HC | . | . | . | . | . | . | . | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . | . | . | . | . | . | . | . |
| 21-225_75B10_HC | . | . | . | . | . | . | . | . | . | . | . | . | . | . | . | . | . | . | . | . | . | . | . | . | . | . |
| 21-225_75C2_HC | . | . | . | . | . | . | . | . | . | . | . | . | . | . | . | . | . | . | . | . | . | . | . | . | . | . |
| 21-225_75C3_HC | . | . | . | . | . | . | . | . | . | . | . | . | . | . | . | . | . | . | . | . | . | . | . | . | . | . |
| 21-225_75D3_HC | . | . | . | . | . | . | . | . | . | . | . | . | . | . | . | . | . | . | . | . | . | . | . | . | . | . |
| 21-225_75D9_HC | . | . | . | . | . | . | . | . | . | . | . | . | . | N | . | . | . | . | . | . | . | . | . | . | . | . |
| 21-225_75F3_HC | . | . | . | . | . | . | . | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . | . | . | . | . | . | . | . |
| 21-225_75G7_HC | . | . | . | . | . | . | . | . | . | . | . | . | . | N | . | . | . | . | . | . | . | . | . | . | . | . |
| 21-225_75H8_HC | . | . | . | . | . | . | . | . | . | . | . | . | . | . | . | T | . | . | . | . | . | . | . | . | . | . |
| 21-225_76A4_HC | . | . | . | . | . | . | . | . | . | . | . | . | . | . | . | . | . | . | . | . | . | . | . | . | . | . |
| 21-225_76D1_HC | . | . | . | . | . | . | . | . | . | . | . | . | . | . | . | . | . | . | . | . | . | . | . | . | . | . |
| 21-225_76E10_HC | . | . | . | . | . | . | . | . | . | . | . | . | . | . | . | . | . | . | . | . | . | . | . | . | . | . |
| 21-225_76G4_HC | . | . | . | . | . | . | . | . | . | . | . | . | . | . | . | . | . | . | . | . | . | . | . | . | . | . |
| 21-225_76H10_HC | . | . | . | . | . | . | . | . | . | . | . | . | . | . | . | . | . | . | . | . | . | . | . | . | . | . |
| 21-225_77A10_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | V | D | T | S | K | N | Q | F | S | L | K | L | S | S | V | T | A | A | D | T | A | V | Y |
| 21-225_77C12_HC | . | . | . | . | . | . | . | . | . | . | . | . | . | N | . | . | . | . | . | . | . | . | . | . | . | . |
| 21-225_77C5_HC | . | . | . | . | . | . | . | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . | . | . | . | . | . | . | . |
| 21-225_80D5_HC | . | . | . | . | . | . | . | . | . | . | . | . | . | . | . | T | . | . | . | . | . | . | M | . | . | . |
| 21-225_80E12_HC | . | . | . | . | . | . | . | . | . | . | . | . | . | . | . | . | . | . | . | . | . | . | . | . | . | . |
| 21-225_80E3_HC | . | . | . | . | . | . | . | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . | . | . | . | . | . | . | . |
| 21-225_80H7_HC | . | . | . | . | . | . | . | . | . | . | . | . | . | . | . | . | . | . | . | . | . | . | . | . | . | . |
| 21-225_81C11_HC | . | . | . | . | . | . | . | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . | . | . | . | . | . | . | . |
| 21-225_84E12_HC | . | . | . | . | . | . | . | . | . | . | . | . | . | . | . | . | . | . | . | . | . | . | . | . | . | . |
| 21-225_85A3_HC | . | . | . | . | . | . | . | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . | . | . | . | . | . | . | . |
| 21-225_85B9_HC | . | . | . | . | . | . | . | . | . | . | . | . | . | . | . | . | . | . | . | . | . | . | . | . | . | . |
| 21-225_85D6_HC | . | . | . | . | . | . | . | . | . | . | . | . | . | . | . | . | . | . | . | . | . | . | . | . | . | . |
| 21-225_85G10_HC | . | . | . | . | . | . | . | . | . | . | . | . | . | . | . | T | . | . | . | . | . | . | . | . | . | . |
| 21-225_86C1_HC | . | . | . | . | . | . | . | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | V | D | T | S | K | N | Q | F | S | L | K | L | S | S | V | T | A | A | D | T | A | V | Y |
| 21-225_87C9_HC | . | . | . | . | . | . | . | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . | . | . | . | . | . | . | . |
| 21-225_87H1_HC | . | . | . | . | . | . | . | . | D | . | . | . | . | . | . | . | . | . | . | . | . | . | . | . | . | . |
| 21-225_88E7_HC | . | . | . | . | . | . | . | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . | . | . | . | . | . | . | . |
| 21-225_89A11_HC | . | . | . | . | . | . | . | . | . | . | . | . | . | . | . | . | . | . | . | . | . | . | . | . | . | . |
| 21-225_89D5_HC | . | . | . | . | . | . | . | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . | . | . | . | . | . | . | . |
| 21-225_89H5_HC | . | . | . | A | . | . | . | . | . | . | . | . | . | . | . | . | . | . | . | . | . | . | . | . | . | . |
| 21-225_90A5_HC | . | . | . | . | . | . | . | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . | . | . | . | . | . | . | . |
| 21-225_92B1_HC | A | . | . | . | . | . | . | . | . | . | . | . | . | N | . | T | . | . | . | . | . | . | . | . | . | . |
| 21-225_92D6_HC | . | . | . | . | . | . | . | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . | . | . | . | . | . | . | . |
| 21-225_94D3_HC | . | . | . | . | . | . | . | . | . | . | . | . | . | . | . | . | . | . | . | . | . | . | . | . | . | . |
| 21-225_94F12_HC | . | . | . | . | . | . | . | . | . | . | . | . | . | . | . | . | . | . | . | . | . | . | . | . | . | . |
| 21-225_94G10_HC | . | . | . | . | . | . | . | . | . | . | . | . | . | . | . | . | . | . | . | . | . | . | . | . | . | . |
| 21-225_95D2_HC | . | . | . | . | . | . | . | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | V | D | T | S | K | N | Q | F | S | L | K | L | S | S | V | T | A | A | D | T | A | V | Y |
| 21-225_95G9_HC | . | . | . | . | . | . | . | . | . | . | . | . | . | . | . | . | . | . | . | . | . | . | . | . | . | . |
| 21-225_95H10_HC | . | . | . | . | . | . | . | . | . | . | . | . | . | . | . | . | . | . | . | . | . | . | . | . | . | . |
| 21-225_97A2_HC | . | . | . | . | . | . | . | . | . | . | . | . | . | . | . | . | . | . | . | . | . | . | . | . | . | . |
| 21-225_97E5_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | Y | G | G | - | - | - | - | - | - | - | - | - | - | - | - | - | - | - | - | - | - |
| 21-225_77C12_HC | . | . | . | . | . | . | . | . | . | . | . | . | . | . | . | . | . | . | . | . | . | . | . | . | . | . |
| 21-225_77C5_HC | . | . | . | . | . | . | . | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . | . | . | . | . | . | . | . |
| 21-225_80D5_HC | . | . | . | . | . | . | . | . | . | . | . | . | . | . | . | . | . | . | . | . | . | . | . | . | . | . |
| 21-225_80E12_HC | . | . | . | . | . | . | . | . | . | . | . | . | . | . | . | . | . | . | . | . | . | . | . | . | . | . |
| 21-225_80E3_HC | . | . | . | . | . | . | . | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . | . | . | . | . | . | . | . |
| 21-225_80H7_HC | . | . | . | . | . | . | . | . | . | . | . | . | . | . | . | . | . | . | . | . | . | . | . | . | . | . |
| 21-225_81C11_HC | . | . | . | . | . | . | . | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . | . | . | . | . | . | . | . |
| 21-225_84E12_HC | . | . | . | . | . | . | . | . | . | . | . | . | . | . | . | . | . | . | . | . | . | . | . | . | . | . |
| 21-225_85A3_HC | . | . | . | . | . | . | . | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . | . | . | . | . | . | . | . |
| 21-225_85B9_HC | . | . | . | . | . | . | . | . | . | . | . | . | . | . | . | . | . | . | . | . | . | . | . | . | . | . |
| 21-225_85D6_HC | . | . | . | . | . | . | . | . | . | . | . | . | . | . | . | . | . | . | . | . | . | . | . | . | . | . |
| 21-225_85G10_HC | . | . | . | . | . | . | . | . | . | . | . | . | . | . | . | . | . | . | . | . | . | . | . | . | . | . |
| 21-225_86C1_HC | . | . | . | . | . | . | . | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | Y | G | G | - | - | - | - | - | - | - | - | - | - | - | - | - | - | - | - | - | - |
| 21-225_87C9_HC | . | . | . | . | . | . | . | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . | . | . | . | . | . | . | . |
| 21-225_87H1_HC | . | . | . | . | . | . | . | . | . | . | . | . | . | . | . | . | . | . | . | . | . | . | . | . | . | . |
| 21-225_88E7_HC | . | . | . | . | . | . | . | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . | . | . | . | . | . | . | . |
| 21-225_89A11_HC | . | . | . | . | . | . | . | . | . | . | . | . | . | . | . | . | . | . | . | . | . | . | . | . | . | . |
| 21-225_89D5_HC | . | . | . | . | . | . | . | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . | . | . | . | . | . | . | . |
| 21-225_89H5_HC | . | . | . | . | . | . | . | . | . | . | . | . | . | . | . | . | . | . | . | . | . | . | . | . | . | . |
| 21-225_90A5_HC | . | . | . | . | . | . | . | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . | . | . | . | . | . | . | . |
| 21-225_92B1_HC | . | . | . | . | . | . | . | . | . | . | . | . | . | . | . | . | . | . | . | . | . | . | . | . | . | . |
| 21-225_92D6_HC | . | . | . | . | . | . | . | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . | . | . | . | . | . | . | . |
| 21-225_94D3_HC | . | . | . | . | . | . | . | . | . | . | . | . | . | . | . | . | . | . | . | . | . | . | . | . | . | . |
| 21-225_94F12_HC | . | . | . | . | . | . | . | . | . | . | . | . | . | . | . | . | . | . | . | . | . | . | . | . | . | . |
| 21-225_94G10_HC | . | . | . | . | . | . | . | . | . | . | . | . | . | . | . | . | . | . | . | . | . | . | . | . | . | . |
| 21-225_95D2_HC | . | . | . | . | . | . | . | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | Y | G | G | - | - | - | - | - | - | - | - | - | - | - | - | - | - | - | - | - | - |
| 21-225_74A3_HC | | | | | | | | | | | | | | | | | | | | | | | | | | |
| 21-225_74A4_HC | | | | | | | | | | | | | | | | | | | | | | | | | | |
| 21-225_74B12_HC | | | | | | | | | | | | | | | | | | | | | | | | | | |
| 21-225_74B2_HC | | | | | | | | | | | | | | | | | | | | | | | | | | |
| 21-225_74C5_HC | | | | | | | | | | | | | | | | | | | | | | | | | | |
| 21-225_74D11_HC | | | | | | | | | | | | | | | | | | | | | | | | | | |
| 21-225_74D5_HC | | | | | | | | | | | | | | | | | | | | | | | | | | |
| 21-225_74D6_HC | | | | | | | | | | | | | | | | | | | | | | | | | | |
| 21-225_74F5_HC | | | | | | | | | | | | | | | | | | | | | | | | | | |
| 21-225_74H5_HC | | | | | | | | | | | | | | | | | | | | | | | | | | |
| 21-225_74H7_HC | | | | | | | | | | | | | | | | | | | | | | | | | | |
| 21-225_75A12_HC | | | | | | | | | | | | | | | | | | | | | | | | | | |
| 21-225_75A5_HC | | | | | | | | | | | | | | | | | | | | | | | | | | |
| 21-225_75A8_HC | | | | | | | | | | | | | | | | | | | | | | | | | | |
| 21-225_75B10_HC | | | | | | | | | | | | | | | | | | | | | | | | | | |
| 21-225_75C2_HC | | | | | | | | | | | | | | | | | | | | | | | | | | |
| 21-225_75C3_HC | | | | | | | | | | | | | | | | | | | | | | | | | | |
| 21-225_75D3_HC | | | | | | | | | | | | | | | | | | | | | | | | | | |
| 21-225_75D9_HC | | | | | | | | | | | | | | | | | | | | | | | | | | |
| 21-225_75F3_HC | | | | | | | | | | | | | | | | | | | | | | | | | | |
| 21-225_75G12_HC | | | | | | | | | | | | | | | | | | | | | | | | | | |
| 21-225_75G7_HC | | | | | | | | | | | | | | | | | | | | | | | | | | |
| 21-225_75H8_HC | | | | | | | | | | | | | | | | | | | | | | | | | | |
| 21-225_76A4_HC | | | | | | | | | | | | | | | | | | | | | | | | | | |
| 21-225_76D1_HC | | | | | | | | | | | | | | | | | | | | | | | | | | |
| 21-225_76E10_HC | | | | | | | | | | | | | | | | | | | | | | | | | | |
| 21-225_76G4_HC | | | | | | | | | | | | | | | | | | | | | | | | | | |
| 21-225_76H10_HC | | | | | | | | | | | | | | | | | | | | | | | | | | |
| 21-225_77A10_HC | | | | | | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | Y | G | G | - | - | - | - | - | - | - | - | - | - | - | - | - | - | - | - | - | - |
| 21-225_95G9_HC | | | | | | | | | | | | | | | | | | | | | | | | | | |
| 21-225_95H10_HC | | | | | | | | | | | | | | | | | | | | | | | | | | |
| 21-225_97A2_HC | | | | | | | | | | | | | | | | | | | | | | | | | | |
| 21-225_97E5_HC | | | | | | | | | | | | | | | | | | | | | | | | | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | - | M | D | V | W | G | Q | G | T | T | V | T | V | S | S |
| 21-225_74A3_HC | | | | | | | | | | | | | | | | | | | |
| 21-225_74A4_HC | | | | | | | | | | | | | | | | | | | |
| 21-225_74B12_HC | | | | | | | | | | | | | | | | | | | |
| 21-225_74B2_HC | | | | | | L | | | | | | | | | | | | | |
| 21-225_74C5_HC | | | | | | | | | | | | | | | | | | | |
| 21-225_74D11_HC | | | | | | | | | | | | | | | | | | | |
| 21-225_74D5_HC | | | | | | | | | | | | | | | | | | | |
| 21-225_74D6_HC | | | | | | | | | | | | | | | | | | | |
| 21-225_74F5_HC | | | | | | | | | | | | | | | | | | | |
| 21-225_74H5_HC | | | | | | | | | | | | | | | | | | | |
| 21-225_74H7_HC | | | | | | L | | | | | | | | | | | | | |
| 21-225_75A12_HC | | | | | | | | | | | | | | | | | | | |
| 21-225_75A5_HC | | | | | | | | | | | | | | | | | | | |
| 21-225_75A8_HC | | | | | | | | | | | | | | | | | | | |
| 21-225_75B10_HC | | | | | | L | | | | | | | | | | | | | |
| 21-225_75C2_HC | | | | | | | | | | | | | | | | | | | |
| 21-225_75C3_HC | | | | | | | | | | | | | | | | | | | |
| 21-225_75D3_HC | | | | | | | | | | | | | | | | | | | |
| 21-225_75D9_HC | | | | | | | | | | | | | | | | | | | |
| 21-225_75F3_HC | | | | | | | | | | | | | | | | | | | |
| 21-225_75G12_HC | | | | | | | | | | | | | | | | | | | |
| 21-225_75G7_HC | | | | | | | | | | | | | | | | | | | |
| 21-225_75H8_HC | | | | | | L | | | | | | | | | | | | | |
| 21-225_76A4_HC | | | | | | | | | | | | | | | | | | | |
| 21-225_76D1_HC | | | | | | | | | | | | | | | | | | | |
| 21-225_76E10_HC | | | | | | | | | | | | | | | | | | | |
| 21-225_76G4_HC | | | | | | | | | | | | | | | | | | | |
| 21-225_76H10_HC | | | | | | | | | | | | | | | | | | | |
| 21-225_77A10_HC | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | - | M | D | V | W | G | Q | G | T | T | V | T | V | S | S |
| 21-225_77C12_HC | | | | | | | | | | | | | | | | | | | |
| 21-225_77C5_HC | | | | | | | | | | | | | | | | | | | |
| 21-225_77F7_HC | | | | | | | | | | | | | | | | | | | |
| 21-225_77G1_HC | | | | | | | | | | | | | | | | | | | |
| 21-225_79F11_HC | | | | | | | | | | | | | | | | | | | |
| 21-225_79F3_HC | | | | | | | | | | | | | | | | | | | |
| 21-225_79G1_HC | | | | | | | | | | | | | | | | | | | |
| 21-225_79G12_HC | | | | | | | | | | | | | | | | | | | |
| 21-225_80A1_HC | | | | | | | | | | | | | | | | | | | |
| 21-225_80C1_HC | | | | | | | | | | | | | | | | | | | |
| 21-225_80D5_HC | | | | | | L | | | | | | | | | | | | | |
| 21-225_80E12_HC | | | | | | | | | | | | | | | | | | | |
| 21-225_80E3_HC | | | | | | | | | | | | | | | | | | | |
| 21-225_80H12_HC | | | | | | | | | | | | | | | | | | | |
| 21-225_80H7_HC | | | | | | I | | | | | | | | | | | | | |
| 21-225_81C11_HC | | | | | | | | | | | | | | | | | | | |
| 21-225_81C5_HC | | | | | | | | | | | | | | | | | | | |
| 21-225_83B7_HC | | | | | | | | | | | | | | | | | | | |
| 21-225_83G1_HC | | | | | | | | | | | | | | | | | | | |
| 21-225_84E12_HC | | | | | | I | | | | | | | | | | | | | |
| 21-225_85A3_HC | | | | | | | | | | | | | | | | | | | |
| 21-225_85B4_HC | | | | | | | | | | | | | | | | | | | |
| 21-225_85B9_HC | | | | | | | | | | | | | | | | | | | |
| 21-225_85D6_HC | | | | | | | | | | | | | | | | | | | |
| 21-225_85G10_HC | | | | | | L | | | | | | | | | | | | | |
| 21-225_86C1_HC | | | | | | | | | | | | | | | | | | | |
| 21-225_86C11_HC | | | | | | | | | | | | | | | | | | | |
| 21-225_86E4_HC | | | | | | | | | | | | | | | | | | | |
| 21-225_87A12_HC | | | | | | | | | | | | | | | | | | | |

3770

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | M | D | V | W | G | Q | G | T | T | V | T | V | S | S |
| 21-225_87C9_HC | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . |
| 21-225_87H1_HC | . | . | . | . | . | L | . | . | . | . | . | . | . | . | . | . | . | . | . |
| 21-225_88E7_HC | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . |
| 21-225_89A11_HC | . | . | . | . | . | L | . | . | . | . | . | . | . | . | . | . | . | . | . |
| 21-225_89D5_HC | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . |
| 21-225_89H5_HC | . | . | . | . | . | . | . | . | . | . | . | . | . | . | . | . | . | . | . |
| 21-225_90A5_HC | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . |
| 21-225_92B1_HC | . | . | . | . | . | L | . | . | . | . | . | . | . | . | . | . | . | . | . |
| 21-225_92D6_HC | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . |
| 21-225_94D3_HC | . | . | . | . | . | . | . | . | . | . | . | . | . | . | . | . | . | . | . |
| 21-225_94F12_HC | . | . | . | . | . | . | . | . | . | . | . | . | . | . | . | . | . | . | . |
| 21-225_94G10_HC | . | . | . | . | . | . | . | . | . | . | . | . | . | . | . | . | . | . | . |
| 21-225_95D2_HC | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | M | D | V | W | G | Q | G | T | T | V | T | V | S | S |
| 21-225_95G9_HC | | | | | | | | | | | | | | | | | | | |
| 21-225_95H10_HC | | | | | | | | | | | | | | | | | | | |
| 21-225_97A2_HC | | | | | | | | | | | | | | | | | | | |
| 21-225_97E5_HC | | | | | | | | | | | | | | | | | | | |

Table 53.    Consensus 19-VH1|1-08/D6|6-19|RF1/JH5 (SEQ ID NO: 50270):

QVQLVQS-GAEVKKPGASVKVSCKASG-YTFTN-----YDINWVRQATGQGLEWMGWMHPN---
SGNTGYAQKFQGRVTMTRNTSISTAYMELSSLRSEDTAVYYCAISSGWY--------------------WFDPWGQGTLVTVSS wherein:

H at position 59 can be substituted with N, or Y

N at position 61 can be substituted with D

S at position 65 can be substituted with N

G at position 66 can be substituted with V

N at position 67 can be substituted with S

T at position 68 can be substituted with I

Y at position 70 can be substituted with F, or C

G at position 76 can be substituted with D

Y at position 113 can be substituted with H, N, S, or K

**FIGURE 57**

W at position 135 can be substituted with R

Asn Tyr Asp Ile Asn (SEQ ID NO: 50013)

Trp Met Xaa Pro Xaa Xaa Xaa Xaa Xaa Gly Xaa Ala Gln Lys Phe Gln Xaa (SEQ ID NO: 50014)

Ser Ser Gly Trp Xaa Xaa Phe Asp Pro (SEQ ID NO: 50015)

Asn Tyr Asp Ile Asn (SEQ ID NO: 50480)

Trp Met His Pro Asn Ser Gly Asn Thr Gly Tyr Ala Gln Lys Phe Gln Gly (SEQ ID NO: 50481)

Ser Ser Gly Trp Tyr Trp Phe Asp Pro (SEQ ID NO: 50482)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | Q | S | - | G | A | E | V | K | K | P | G | A | S | V | K | V | S | C | K | A | S |
| 21-225_147G6_HC | . | . | . | . | . | . | . | . | . | . | . | . | . | . | . | . | . | . | . | . | . | . | . | . | . | . |
| 21-225_152F4_HC | . | . | . | . | . | . | . | . | . | . | . | . | . | . | . | . | . | . | . | . | . | . | . | . | . | . |
| 21-225_170F6_HC | . | . | . | . | . | . | . | . | . | . | . | . | . | . | . | . | . | . | . | . | . | . | . | . | . | . |
| 21-225_174G5_HC | . | . | . | . | . | . | . | . | . | . | . | . | . | . | . | . | . | . | . | . | . | . | . | . | . | . |
| 21-225_224H5_HC | . | . | . | . | . | . | . | . | . | . | . | . | . | . | . | . | . | . | . | . | . | . | . | . | . | . |
| 21-225_225F4_HC | . | . | . | . | . | . | . | . | . | . | . | . | . | . | . | . | . | . | . | . | . | . | . | . | . | . |
| 21-225_227G9_HC | . | . | . | . | . | . | . | . | . | . | . | . | . | . | . | . | . | . | . | . | . | . | . | R | . | . |
| 21-225_25A3_HC | . | . | . | . | . | . | . | . | . | . | . | . | . | . | . | . | . | . | . | . | . | . | . | . | . | . |
| 21-225_26A9_HC | . | . | . | . | . | . | . | . | . | . | . | . | . | . | . | . | . | . | . | . | . | . | . | . | . | . |
| 21-225_57A11_HC | . | . | . | . | . | . | . | . | . | . | . | . | . | . | . | . | . | . | . | . | . | . | . | . | . | . |
| 21-225_57F12_HC | . | . | . | . | . | . | . | . | . | . | . | . | . | . | . | . | . | . | . | . | . | . | . | . | . | . |
| 21-225_74A5_HC | . | . | . | . | . | . | . | . | . | . | . | . | . | . | . | . | . | . | . | . | . | . | . | . | . | . |
| 21-225_74A7_HC | . | . | . | . | . | . | . | . | . | . | . | . | . | . | . | . | . | . | . | . | . | . | . | . | . | . |
| 21-225_74B10_HC | . | . | . | . | . | . | . | . | . | . | . | . | . | . | . | . | . | . | . | . | . | . | . | . | . | . |
| 21-225_74B5_HC | . | . | . | . | . | . | . | . | . | . | . | . | . | . | . | . | . | . | . | . | . | . | . | . | . | . |
| 21-225_74B6_HC | . | . | . | . | . | . | . | . | . | . | . | . | . | . | . | . | . | . | . | . | . | . | . | . | . | . |
| 21-225_74B7_HC | . | . | . | . | . | . | . | . | . | . | . | . | . | . | . | . | . | . | . | . | . | . | . | . | . | . |
| 21-225_74B8_HC | . | . | . | . | . | . | . | . | . | . | . | . | . | . | . | . | . | . | . | . | . | . | . | . | . | . |
| 21-225_74C1_HC | . | . | . | . | . | . | . | . | . | . | . | . | . | . | . | . | . | . | . | . | . | . | . | . | . | . |
| 21-225_74C10_HC | . | . | . | . | . | . | . | . | . | . | . | . | . | . | . | . | . | . | . | . | . | . | . | . | . | . |
| 21-225_74C12_HC | . | . | . | . | . | . | . | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . | . | . | . | . | . | . | . |
| 21-225_74H2_HC | . | . | . | . | . | . | . | . | . | . | . | . | . | . | . | . | . | . | . | . | . | . | . | . | . | . |
| 21-225_75A7_HC | . | . | . | . | . | . | . | . | . | . | . | . | . | . | . | . | . | . | . | . | . | . | . | . | . | . |
| 21-225_75B1_HC | . | . | . | . | . | . | . | . | . | . | . | . | . | . | . | . | . | . | . | . | . | . | . | . | . | . |
| 21-225_76A6_HC | . | . | . | . | . | . | . | . | . | . | . | . | . | . | . | . | . | . | . | . | . | . | . | . | . | . |
| 21-225_76B9_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | |
| CONSENSUS | Q | V | Q | L | V | Q | S | - | G | A | E | V | K | K | P | G | A | S | V | K | V | S | C | K | A | S |
| 21-225_76E8_HC | . | . | . | . | . | . | . | . | . | . | . | . | . | . | . | . | . | . | . | . | . | . | . | . | . | . |
| 21-225_76F3_HC | . | . | . | . | . | . | . | . | . | . | . | . | . | . | . | . | . | . | . | . | . | . | . | . | . | . |
| 21-225_76F5_HC | . | . | . | . | . | . | . | . | . | . | . | . | . | . | . | . | . | . | . | . | . | . | . | . | . | . |
| 21-225_76H1_HC | . | . | . | . | . | . | . | . | . | . | . | . | . | . | . | . | . | . | . | . | . | . | . | . | . | . |
| 21-225_77C10_HC | . | . | . | . | . | . | . | . | . | . | . | . | . | . | . | . | . | . | . | . | . | . | . | . | . | . |
| 21-225_77C7_HC | . | . | . | . | . | . | . | . | . | . | . | . | . | . | . | . | . | . | . | . | . | . | . | . | . | . |
| 21-225_77D11_HC | . | . | . | . | . | . | . | . | . | . | . | . | . | . | . | . | . | . | . | . | . | . | . | . | . | . |
| 21-225_77D12_HC | . | . | . | . | . | . | . | . | . | . | . | . | . | . | . | . | . | . | . | . | . | . | . | . | . | . |
| 21-225_77E1_HC | . | . | . | . | . | . | . | . | . | . | . | . | . | . | . | . | . | . | . | . | . | . | . | . | . | . |
| 21-225_78C1_HC | . | . | . | . | . | . | . | . | . | . | . | . | . | . | . | . | . | . | . | . | . | . | . | . | . | . |
| 21-225_78H12_HC | . | . | . | . | . | . | . | . | . | . | . | . | . | . | . | . | . | . | . | . | . | . | . | . | . | . |
| 21-225_79G10_HC | . | . | . | . | . | . | . | . | . | . | . | . | . | . | . | . | . | . | . | . | . | . | . | . | . | . |
| 21-225_79G6_HC | . | . | . | . | . | . | . | . | . | . | . | . | . | . | . | . | . | . | . | . | . | . | . | . | . | . |
| 21-225_80A2_HC | . | . | . | . | . | . | . | . | . | . | . | . | . | . | . | . | . | . | . | . | . | . | . | . | . | . |
| 21-225_80C12_HC | . | . | . | . | . | . | . | . | . | . | . | . | . | . | . | . | . | . | . | . | . | . | . | . | . | . |
| 21-225_80D3_HC | . | . | . | . | . | . | . | . | . | . | . | . | . | . | . | . | . | . | . | . | . | . | . | . | . | . |
| 21-225_80E9_HC | . | . | . | . | . | . | . | . | . | . | . | . | . | . | . | . | . | . | . | . | . | . | . | . | . | . |
| 21-225_81E5_HC | . | . | . | . | . | . | . | . | . | . | . | . | . | . | . | . | . | . | . | . | . | . | . | . | . | . |
| 21-225_81F9_HC | . | . | . | . | . | . | . | . | . | . | . | . | . | . | . | . | . | . | . | . | . | . | . | . | . | . |
| 21-225_82A5_HC | . | . | . | . | . | . | . | . | . | . | . | . | . | . | . | . | . | . | . | . | . | . | . | . | . | . |
| 21-225_82C12_HC | . | . | . | . | . | . | . | . | . | . | . | . | . | . | . | . | . | . | . | . | . | . | . | . | . | . |
| 21-225_82D9_HC | . | . | . | . | . | . | . | . | . | . | . | . | . | . | . | . | . | . | . | . | . | . | . | . | . | . |
| 21-225_82G5_HC | . | . | . | . | . | . | . | . | . | . | . | . | . | . | . | . | . | . | . | . | . | . | . | . | . | . |
| 21-225_83C2_HC | . | . | . | . | . | . | . | . | . | . | . | . | . | . | . | . | . | . | . | . | . | . | . | . | . | . |
| 21-225_83C5_HC | . | . | . | . | . | . | . | . | . | . | . | . | . | . | . | . | . | . | . | . | . | . | . | . | . | . |
| 21-225_83F3_HC | . | . | . | . | . | . | . | . | . | . | . | . | . | . | . | . | . | . | . | . | . | . | . | . | . | . |
| 21-225_83G3_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | Q | S | - | G | A | E | V | K | K | P | G | A | S | V | K | V | S | C | K | A | S |
| 21-225_83G7_HC | . | . | . | . | . | . | . | . | . | . | . | . | . | . | . | . | . | . | . | . | . | . | . | . | . | . |
| 21-225_84G7_HC | . | . | . | . | . | . | . | . | . | . | . | . | . | . | . | . | . | . | . | . | . | . | . | . | . | . |
| 21-225_85B5_HC | . | . | . | . | . | . | . | . | . | . | . | . | . | . | . | . | . | . | . | . | . | . | . | . | . | . |
| 21-225_85D11_HC | . | . | . | . | . | . | . | . | . | . | . | . | . | . | . | . | . | . | . | . | . | . | . | . | . | . |
| 21-225_85H2_HC | . | . | . | . | . | . | . | . | . | . | . | . | . | . | . | . | . | . | . | . | . | . | . | . | . | . |
| 21-225_86E9_HC | . | . | . | . | . | . | . | . | . | . | . | . | . | . | . | . | . | . | . | . | . | . | . | . | . | . |
| 21-225_87A10_HC | . | . | . | . | . | . | . | . | . | . | . | . | . | . | . | . | . | . | . | . | . | . | . | . | . | . |
| 21-225_88G2_HC | . | . | . | . | . | . | . | . | . | . | . | . | . | . | . | . | . | . | . | . | . | . | . | . | . | . |
| 21-225_90D9_HC | . | . | . | . | . | . | . | . | . | . | . | . | . | . | . | . | . | . | . | . | . | . | . | . | . | . |
| 21-225_91F1_HC | . | . | . | . | . | . | . | . | . | . | . | . | . | . | . | . | . | . | . | . | . | . | . | . | . | . |
| 21-225_91G8_HC | . | . | . | . | . | . | . | . | . | . | . | . | . | . | . | . | . | . | . | . | . | . | . | . | . | . |
| 21-225_94D2_HC | . | . | . | . | . | . | . | . | . | . | . | . | . | . | . | . | . | . | . | . | . | . | . | . | . | . |
| 21-225_94E11_HC | . | . | . | . | . | . | . | . | . | . | . | . | . | . | . | . | . | . | . | . | . | . | . | . | . | . |
| 21-225_95E12_HC | . | . | . | . | . | . | . | . | . | . | . | . | . | . | . | . | . | . | . | . | . | . | . | . | . | . |
| 21-225_95G4_HC | . | . | . | . | . | . | . | . | . | . | . | . | . | . | . | . | . | . | . | . | . | . | . | . | . | . |
| 21-225_95H4_HC | . | . | . | . | . | . | . | . | . | . | . | . | . | . | . | . | . | . | . | . | . | . | . | . | . | . |
| 21-225_96D5_HC | . | . | . | . | . | . | . | . | . | . | . | . | . | . | . | . | . | . | . | . | . | . | . | . | . | . |
| 21-225_96E2_HC | . | . | . | . | . | . | . | . | . | . | . | . | . | . | . | . | . | . | . | . | . | . | . | . | . | . |
| 21-225_96G1_HC | . | . | . | . | . | . | . | . | . | . | . | . | . | . | . | . | . | . | . | . | . | . | . | . | . | . |
| 21-225_97H4_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | H_CDR1 | | | | | | | | | | | | | H_FR2 | | | |
| CONSENSUS | G | - | Y | T | F | T | N | - | - | - | - | - | Y | D | I | N | W | V | R | Q | A | T | G | Q | G | L |
| 21-225_147G6_HC | | | | | | | | | | | | | | | | | | | | | | | | | | |
| 21-225_152F4_HC | | | | | | | | | | | | | | | | | | | | | | | | | | |
| 21-225_170F6_HC | | | | | | | | | | | | | | | | | | | | | | | | | | |
| 21-225_174G5_HC | | | | | | | | | | | | | | | | | | | | | | | | | | |
| 21-225_224H5_HC | | | | | | | | | | | | | | | | | | | | | | | | | | |
| 21-225_225F4_HC | | | | | | | | | | | | | | | | | | | | | | | | | | |
| 21-225_227G9_HC | | | | | | | | | | | | | | | | | | | | | | | | R | | |
| 21-225_25A3_HC | | | | I | | | | | | | | | | | | | | | | | | | | | | |
| 21-225_26A9_HC | | | | | | | | | | | | | | | | | | | | | | | | | | |
| 21-225_57A11_HC | | | | | | | | | | | | | | | | | | | | | | | | | | |
| 21-225_57F12_HC | | | | | | | | | | | | | | | | | | | | | | | | | | |
| 21-225_74A5_HC | | | | | | | | | | | | | | | | | | | | | V | P | | | | |
| 21-225_74A7_HC | | | | | | | | | | | | | | | | | | | | | | | | | | |
| 21-225_74B10_HC | | | F | | | | | | | | | | | | | | | | | | | | | | | |
| 21-225_74B5_HC | | | | | | | | | | | | | | | | | | | | | | | | | | |
| 21-225_74B6_HC | | | | | | | | | | | | | | | | | | | | | | | | | | |
| 21-225_74B7_HC | | | | | P | | | | | | | | | | | | | | | | | | | | | |
| 21-225_74B8_HC | | | | | | | | | | | | | | | | | | | | | | | | | | |
| 21-225_74C1_HC | | | | | | | | | | | | | | | | | | | | | | | | | | |
| 21-225_74C10_HC | | | | | | | | | | | | | | | | | | | | | | | | | | |
| 21-225_74C12_HC | | | | | | | | | | | | | | | | | | | | | | | | | | |
| 21-225_74G3_HC | | | | | | | | | | | | | | | | | | | | | V | P | | | | |
| 21-225_74H2_HC | | | F | | | | | | | | | | | | | | | | | | | | | | | |
| 21-225_75A7_HC | | | | | P | | | | | | | | | | | | | | | | | | | | | |
| 21-225_75B1_HC | | | | | | | | | | | | | | | | | | | | | V | P | | | | |
| 21-225_76A6_HC | | | | | | | | | | | | | | | | | | | | | | | | | | |
| 21-225_76B9_HC | | | | | | | | | | | | | | | | | | | | | V | P | | | | |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | H_CDR1 | | | | | | | | | | | | | H_FR2 | | | |
| CONSENSUS | G | - | Y | T | F | T | N | - | - | - | - | - | Y | D | I | N | W | V | R | Q | A | T | G | Q | G | L |
| 21-225_76E8_HC | | | | | | P | | | | | | | | | | | | | | | | | | | | |
| 21-225_76F3_HC | | | | | | | | | | | | | | | | | | | | | | | | | | |
| 21-225_76F5_HC | | | | | | | | | | | | | | | | | | | | | | | | | | |
| 21-225_76H1_HC | | | | | | | | | | | | | | | | | | | | | V | P | | | | |
| 21-225_77C10_HC | | | | | | | | | | | | | | | | | | | | | | | | | | |
| 21-225_77C7_HC | | | | | | P | | | | | | | | | | | | | | | | | | | | |
| 21-225_77D11_HC | | | | | | | | | | | | | | | | | | | | | V | P | | | | |
| 21-225_77D12_HC | | | | | | | | | | | | | | | | | | | | | | | | | | |
| 21-225_77E1_HC | | | | | | P | | | | | | | | | | | | | | | | | | | | |
| 21-225_78C1_HC | | | | | | | | | | | | | | | | | | | | | | | | | | |
| 21-225_78H12_HC | | | | | | P | | | | | | | | | | | | | | | | | | | | |
| 21-225_79G10_HC | | | | | | | | | | | | | | | | | | | | | | | | | | |
| 21-225_79G6_HC | | | F | | | | | | | | | | | | | | | | | | | | | | | |
| 21-225_80A2_HC | | | | | | | | | | | | | | | | | | | | | V | P | | | | |
| 21-225_80C12_HC | | | | | | | | | | | | | | | | | | | | | | | | | | |
| 21-225_80D3_HC | | | | | | | | | | | | | | | | | | | | | | | | | | |
| 21-225_80E9_HC | | | | | | | | | | | | | | | | | | | | | | | | | | |
| 21-225_81E5_HC | | | | | | | | | | | | | | | | | | | | | | | | | | |
| 21-225_81F9_HC | | | | | | | | | | | | | | | | | | | | | | | | | | |
| 21-225_82A5_HC | | | | | | | | | | | | | | | | | | | | | | | | | | |
| 21-225_82C12_HC | | | | | | | | | | | | | | | | | | | | | | | | | | |
| 21-225_82D9_HC | | | | | | | | | | | | | | | | | | | | | V | P | | | | |
| 21-225_82G5_HC | | | | | | | | | | | | | | | | | | | | | V | P | | | | |
| 21-225_83C2_HC | | | | | | | | | | | | | | | | | | | | | V | P | | | | |
| 21-225_83C5_HC | | | | | | | | | | | | | | | | | | | | | V | P | | | | |
| 21-225_83F3_HC | | | | | | | | | | | | | | | | | | | | | | | | | | |
| 21-225_83G3_HC | | | | | | | | | | | | | | | | | | | | | | | | | | |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | H_FR2 | | | | |
| CONSENSUS | G | - | Y | T | F | T | N | - | - | - | - | - | Y | D | I | N | W | V | R | Q | A | T | G | Q | G | L |
| 21-225_83G7_HC | . | . | . | . | . | P | . | . | . | . | . | . | . | . | . | . | . | . | . | . | . | . | . | . | . | . |
| 21-225_84G7_HC | . | . | . | . | . | P | . | . | . | . | . | . | . | . | . | . | . | . | . | . | . | . | . | . | . | . |
| 21-225_85B5_HC | . | . | . | . | . | . | . | . | . | . | . | . | . | . | . | . | . | . | . | . | V | P | . | . | . | . |
| 21-225_85D11_HC | . | . | . | . | . | . | . | . | . | . | . | . | . | . | . | . | . | . | . | . | V | P | . | . | . | . |
| 21-225_85H2_HC | . | . | . | . | . | . | . | . | . | . | . | . | . | . | . | . | . | . | . | . | . | . | . | . | . | . |
| 21-225_86E9_HC | . | . | . | . | . | . | . | . | . | . | . | . | . | . | . | . | . | . | . | . | . | . | . | . | . | . |
| 21-225_87A10_HC | . | . | . | . | . | . | . | . | . | . | . | . | . | . | . | . | . | . | . | . | . | . | . | . | . | . |
| 21-225_88G2_HC | . | . | . | . | . | . | . | . | . | . | . | . | . | . | . | . | . | . | . | . | . | . | . | . | . | . |
| 21-225_90D9_HC | . | . | . | . | . | P | . | . | . | . | . | . | . | . | . | . | . | . | . | . | . | . | . | . | . | . |
| 21-225_91F1_HC | . | . | . | . | . | . | . | . | . | . | . | . | . | . | . | . | . | . | . | . | . | . | . | . | . | . |
| 21-225_91G8_HC | . | . | . | . | . | . | . | . | . | . | . | . | . | . | . | . | . | . | . | . | . | . | . | . | . | . |
| 21-225_94D2_HC | . | . | F | . | . | . | . | . | . | . | . | . | . | . | . | . | . | . | . | . | . | . | . | . | . | . |
| 21-225_94E11_HC | . | . | . | . | . | . | . | . | . | . | . | . | . | . | . | . | . | . | . | . | . | . | . | . | . | . |
| 21-225_95E12_HC | . | . | . | . | . | . | . | . | . | . | . | . | . | . | . | . | . | . | . | . | . | . | . | . | . | . |
| 21-225_95G4_HC | . | . | . | . | . | . | . | . | . | . | . | . | . | . | . | . | . | . | . | . | . | . | . | . | . | . |
| 21-225_95H4_HC | . | . | . | . | . | . | . | . | . | . | . | . | . | . | . | . | . | . | . | . | . | . | . | . | . | . |
| 21-225_96D5_HC | . | . | . | . | . | . | . | . | . | . | . | . | . | . | . | . | . | . | . | . | . | . | . | . | . | . |
| 21-225_96E2_HC | . | . | . | . | . | . | . | . | . | . | . | . | . | . | . | . | . | . | . | . | . | . | . | . | . | . |
| 21-225_96G1_HC | . | . | . | . | . | . | . | . | . | . | . | . | . | . | . | . | . | . | . | . | . | . | . | . | . | . |
| 21-225_97H4_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | M | G | W | M | H | P | N | - | - | - | S | G | N | T | G | Y | A | Q | K | F | Q | G | R | V |
| 21-225_147G6_HC | . | . | . | . | . | . | . | . | . | . | . | . | . | . | . | . | . | . | . | . | . | . | . | . | . | . |
| 21-225_152F4_HC | . | . | . | . | . | . | . | . | . | . | . | . | . | . | . | . | . | . | . | . | . | . | . | . | . | . |
| 21-225_170F6_HC | . | . | . | . | . | . | N | . | . | . | . | . | . | . | . | . | . | . | . | . | . | . | . | D | . | . |
| 21-225_174G5_HC | . | . | . | . | . | . | . | . | . | . | . | . | . | . | . | . | . | . | . | . | . | . | . | . | . | . |
| 21-225_224H5_HC | . | . | . | . | . | . | N | . | . | . | . | . | . | . | . | . | . | . | . | . | . | . | . | . | . | . |
| 21-225_225F4_HC | . | . | . | . | . | . | . | . | . | . | . | . | . | S | . | . | . | . | . | . | . | . | . | . | . | L |
| 21-225_227G9_HC | . | . | . | . | . | . | Y | . | . | . | . | . | . | . | . | . | . | . | . | . | . | . | . | . | . | . |
| 21-225_25A3_HC | . | . | . | . | . | . | . | . | . | . | . | . | . | . | . | . | . | . | . | . | . | . | . | . | . | . |
| 21-225_26A9_HC | . | . | . | . | . | . | . | . | . | . | . | . | . | . | . | . | . | C | . | . | . | . | . | . | . | . |
| 21-225_57A11_HC | . | . | . | . | . | . | N | . | . | . | . | . | . | V | . | . | . | . | . | . | . | . | . | . | . | . |
| 21-225_57F12_HC | . | . | . | . | . | . | N | . | . | . | . | . | . | . | . | . | . | . | . | . | . | . | . | . | . | . |
| 21-225_74A5_HC | . | . | . | . | . | . | . | . | . | . | . | . | . | . | . | . | . | . | . | . | . | . | . | . | . | . |
| 21-225_74A7_HC | . | . | . | . | . | . | N | . | . | . | . | . | . | . | . | . | . | . | . | . | . | . | . | . | . | . |
| 21-225_74B10_HC | . | . | . | . | . | . | . | . | . | . | . | . | . | . | . | . | . | F | . | . | . | . | . | . | . | . |
| 21-225_74B5_HC | . | . | . | . | . | . | N | . | . | . | . | . | . | . | . | . | . | . | . | . | . | . | . | . | . | . |
| 21-225_74B6_HC | . | . | . | . | . | . | . | . | . | . | . | . | N | . | . | . | . | . | . | . | . | . | . | . | . | . |
| 21-225_74B7_HC | . | . | . | . | . | . | N | . | . | . | . | . | . | . | . | . | . | . | . | . | . | . | . | . | . | . |
| 21-225_74B8_HC | . | . | . | . | . | . | . | . | . | . | . | . | . | . | . | . | . | . | . | . | . | . | . | . | . | . |
| 21-225_74C1_HC | . | . | . | . | . | . | N | . | D | . | . | . | . | . | . | . | . | F | . | . | . | . | . | . | . | . |
| 21-225_74C10_HC | . | . | . | . | . | . | N | . | . | . | . | . | . | . | . | I | . | . | . | . | . | . | . | . | . | . |
| 21-225_74C12_HC | . | . | . | . | . | . | N | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . | . | . | . | . | . | . | . |
| 21-225_74H2_HC | . | . | . | . | . | . | . | . | . | . | . | . | . | . | . | . | . | F | . | . | . | . | . | . | . | . |
| 21-225_75A7_HC | . | . | . | . | . | . | N | . | . | . | . | . | . | . | . | . | . | . | . | . | . | . | . | D | . | . |
| 21-225_75B1_HC | . | . | . | . | . | . | . | . | . | . | . | . | . | . | . | . | . | . | . | . | . | . | . | . | . | . |
| 21-225_76A6_HC | . | . | . | . | . | . | . | . | . | . | . | . | N | . | . | . | . | . | . | . | . | . | . | . | . | . |
| 21-225_76B9_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | M | G | W | M | H | P | N | - | - | - | S | G | N | T | G | Y | A | Q | K | F | Q | G | R | V |
| 21-225_76E8_HC | | | | | | | N | | | | | | | | | | | | | | | | | | | |
| 21-225_76F3_HC | | | | | | | N | | | | | | | | | | | | | | | | | | | |
| 21-225_76F5_HC | | | | | | | N | | | | | | | | | | | | | | | | | | | |
| 21-225_76H1_HC | | | | | | | | | | | | | | | | | | | | | | | | | | |
| 21-225_77C10_HC | | | | | | | | | | | | | | | | | | | | | | | | | | |
| 21-225_77C7_HC | | | | | | | N | | | | | | | | | | | | | | | | | | | |
| 21-225_77D11_HC | | | | | | | | | | | | | | | | | | | | | | | | | | |
| 21-225_77D12_HC | | | | | | | N | | | | | | | | | | | | | | | | | | | |
| 21-225_77E1_HC | | | | | | | N | | | | | | | | | | | | | | | | | | | |
| 21-225_78C1_HC | | | | | | | | | | | | | | | | | | | | | | | | | | |
| 21-225_78H12_HC | | | | | | | N | | | | | | | | | | | | | | | | | | | |
| 21-225_79G10_HC | | | | | | | N | | | | | | | | | | | | | | | | | | | |
| 21-225_79G6_HC | | | | | | | | | | | | | | | | | | F | | | | | | | | |
| 21-225_80A2_HC | | | | | | | | | | | | | | | | | | | | | | | | | | |
| 21-225_80C12_HC | | | | | | | | | | | | | N | | | | | | | | | | | | | |
| 21-225_80D3_HC | | | | | | | | | | | | | | | | | | | | | | | | | | |
| 21-225_80E9_HC | | | | | | | | | | | | | | | | | | | | | | | | | | |
| 21-225_81E5_HC | | | | | | | N | | | | | | | | | | | | | | | | | | | |
| 21-225_81F9_HC | | | | | | | | | | | | | | | | | | | | | | | | | | |
| 21-225_82A5_HC | | | | | | | | | | | | | N | | | | | | | | | | | | | |
| 21-225_82C12_HC | | | | | | | | | | | | | N | | | | | | | | | | | | | |
| 21-225_82D9_HC | | | | | | | | | | | | | | | | | | | | | | | | | | |
| 21-225_82G5_HC | | | | | | | | | | | | | | | | | | | | | | | | | | |
| 21-225_83C2_HC | | | | | | | | | | | | | | | | | | | | | | | | | | |
| 21-225_83C5_HC | | | | | | | | | | | | | | | | | | | | | | | | | | |
| 21-225_83F3_HC | | | | | | | | | | | | | | | | | | | | | | | | | | |
| 21-225_83G3_HC | | | | | | | | | | | | | | | | | | | | | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | M | G | W | M | H | P | N | - | - | - | S | G | N | T | G | Y | A | Q | K | F | Q | G | R | V |
| 21-225_83G7_HC | | | | | | | N | | | | | | | | | | | | | | | | | | | |
| 21-225_84G7_HC | | | | | | | N | | | | | | | | | | | | | | | | | D | | |
| 21-225_85B5_HC | | | | | | | | | | | | | | | | | | | | | | | | | | |
| 21-225_85D11_HC | | | | | | | | | | | | | | | | | | | | | | | | | | |
| 21-225_85H2_HC | | | | | | | | | | | | | | | | | | | | | | | | | | |
| 21-225_86E9_HC | | | | | | | N | | | | | | | | | | | | | | | | | | | |
| 21-225_87A10_HC | | | | | | | | | | | | | N | | | | | | | | | | | | | |
| 21-225_88G2_HC | | | | | | | | | | | | | | | | | | | | | | | | | | |
| 21-225_90D9_HC | | | | | | | N | | | | | | | | | | | | | | | | | | | |
| 21-225_91F1_HC | | | | | | | | | | | | | | | | | | | | | | | | | | |
| 21-225_91G8_HC | | | | | | | | | | | | | | | | | | | | | | | | | | |
| 21-225_94D2_HC | | | | | | | | | | | | | | | | | | F | | | | | | | | |
| 21-225_94E11_HC | | | | | | | N | | | | | | | | | | | | | | | | | | | |
| 21-225_95E12_HC | | | | | | | N | | | | | | | | | | | | | | | | | | | |
| 21-225_95G4_HC | | | | | | | N | | | | | | | | | | | | | | | | | | | |
| 21-225_95H4_HC | | | | | | | N | | | | | | | | | | | | | | | | | | | |
| 21-225_96D5_HC | | | | | | | N | | | | | | | | | | | | | | | | | | | |
| 21-225_96E2_HC | | | | | | | | | | | | | | | | | | | | | | | | | | |
| 21-225_96G1_HC | | | | | | | | | | | | | N | | | | | | | | | | | | | |
| 21-225_97H4_HC | | | | | | | | | | | | | | | | | | | | | | | | | | |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | M | T | R | N | T | S | I | S | T | A | Y | M | E | L | S | S | L | R | S | E | D | T | A | V | Y |
| 21-225_147G6_HC | . | . | . | . | . | . | . | . | . | . | . | . | . | . | . | . | . | . | . | . | . | . | . | . | . | . |
| 21-225_152F4_HC | . | . | . | . | . | . | . | . | . | . | . | . | . | . | . | . | . | . | . | . | D | . | . | . | . | . |
| 21-225_170F6_HC | . | . | . | . | H | . | . | . | . | . | . | . | . | . | . | . | . | . | . | . | . | . | . | . | . | . |
| 21-225_174G5_HC | . | . | . | . | . | . | . | . | . | . | . | . | . | . | . | . | . | . | . | . | . | . | . | . | . | . |
| 21-225_224H5_HC | . | . | . | . | . | . | . | . | . | . | . | . | . | . | . | . | . | . | . | . | . | . | . | . | . | . |
| 21-225_225F4_HC | . | . | . | . | . | . | . | . | . | . | V | . | . | . | . | N | . | . | . | . | . | . | . | . | . | . |
| 21-225_227G9_HC | . | . | . | . | . | . | . | . | . | . | . | . | . | . | . | . | . | . | . | . | . | . | . | . | . | . |
| 21-225_25A3_HC | . | . | . | W | . | . | . | . | . | . | . | N | . | . | . | . | . | . | . | . | . | . | . | . | . | . |
| 21-225_26A9_HC | . | . | . | W | . | M | . | . | . | . | . | . | . | . | . | . | . | . | . | . | . | . | . | . | . | . |
| 21-225_57A11_HC | . | . | . | . | D | . | . | . | . | . | . | . | . | . | . | . | . | . | . | . | . | . | . | . | . | . |
| 21-225_57F12_HC | . | . | . | . | . | . | . | . | . | . | . | . | . | . | . | . | . | . | . | . | . | . | . | . | . | . |
| 21-225_74A5_HC | . | . | . | . | . | . | . | . | . | . | . | . | . | . | . | . | . | . | . | . | . | . | . | . | . | . |
| 21-225_74A7_HC | . | . | . | . | . | . | . | T | . | . | . | H | . | . | . | . | . | . | . | . | . | . | . | . | . | . |
| 21-225_74B10_HC | . | . | . | . | . | . | . | . | . | . | . | . | . | . | . | . | . | . | . | . | . | . | . | . | . | . |
| 21-225_74B5_HC | . | . | . | . | . | . | . | T | . | . | . | H | . | . | . | . | . | . | . | . | . | . | . | . | . | . |
| 21-225_74B6_HC | . | . | . | . | . | . | . | . | . | . | . | . | . | . | . | N | . | . | . | . | . | . | . | . | . | . |
| 21-225_74B7_HC | . | . | . | . | . | . | . | . | . | . | . | . | . | . | . | . | . | . | . | . | . | . | . | . | . | . |
| 21-225_74B8_HC | . | L | . | . | . | . | . | . | . | . | . | . | . | . | . | . | . | . | . | . | . | . | . | . | . | . |
| 21-225_74C1_HC | . | . | . | . | . | . | . | . | . | . | . | . | . | . | . | . | . | . | . | . | . | . | . | . | . | . |
| 21-225_74C10_HC | . | . | . | . | . | . | . | . | . | . | . | . | . | . | . | . | . | . | . | . | . | . | . | . | . | . |
| 21-225_74C12_HC | . | . | . | . | . | . | . | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . | . | . | . | . | . | . | . |
| 21-225_74H2_HC | . | . | . | . | . | . | . | . | . | . | . | . | . | . | . | . | . | . | . | . | . | . | . | . | . | . |
| 21-225_75A7_HC | . | . | . | . | . | . | . | . | . | . | . | . | . | . | . | . | . | . | . | . | . | . | . | . | . | . |
| 21-225_75B1_HC | . | . | . | . | . | . | . | . | . | . | . | . | . | . | . | . | . | . | . | . | . | . | . | . | . | . |
| 21-225_76A6_HC | . | . | . | . | . | . | . | . | . | . | . | . | . | . | . | N | . | . | . | . | . | . | . | . | . | . |
| 21-225_76B9_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | M | T | R | N | T | S | I | S | T | A | Y | M | E | L | S | S | L | R | S | E | D | T | A | V | Y |
| 21-225_76E8_HC | . | . | . | . | . | . | . | . | . | . | . | . | . | . | . | . | . | . | . | . | . | . | . | . | . | . |
| 21-225_76F3_HC | . | . | . | . | . | . | . | T | . | . | . | H | . | . | . | . | . | . | . | . | . | . | . | . | . | . |
| 21-225_76F5_HC | . | . | . | . | . | . | . | . | . | . | . | . | . | . | . | . | . | . | . | . | . | . | . | . | . | . |
| 21-225_76H1_HC | . | . | . | . | . | . | . | . | . | . | . | . | . | . | . | . | . | . | . | . | . | . | . | . | . | . |
| 21-225_77C10_HC | . | . | . | . | . | . | . | . | . | . | . | . | . | . | . | . | . | . | . | . | . | . | . | . | . | . |
| 21-225_77C7_HC | . | . | . | . | . | . | . | . | . | . | . | . | . | . | . | . | . | . | . | . | . | . | . | . | . | . |
| 21-225_77D11_HC | . | . | . | . | . | . | . | . | . | . | . | . | . | . | . | . | . | . | . | . | . | . | . | . | . | . |
| 21-225_77D12_HC | . | . | . | . | . | . | . | N | . | . | . | . | . | . | . | . | . | . | . | . | . | . | . | . | . | . |
| 21-225_77E1_HC | . | . | . | . | . | . | . | . | . | . | . | . | . | . | . | . | . | . | . | . | . | . | . | . | . | . |
| 21-225_78C1_HC | . | . | . | . | . | . | . | . | . | . | . | . | . | . | . | . | . | . | . | . | . | . | . | . | . | . |
| 21-225_78H12_HC | . | . | . | . | . | . | . | . | . | . | . | . | . | . | . | . | . | . | . | . | . | . | . | . | . | . |
| 21-225_79G10_HC | . | . | . | . | . | . | . | T | . | . | . | H | . | . | . | . | . | . | . | . | . | . | . | . | . | . |
| 21-225_79G6_HC | . | . | . | . | . | . | . | . | . | . | . | . | . | . | . | . | . | . | . | . | . | . | . | . | . | . |
| 21-225_80A2_HC | . | . | . | . | . | . | . | . | . | . | . | . | . | . | . | . | . | . | . | . | . | . | . | . | . | . |
| 21-225_80C12_HC | . | . | . | . | . | . | . | . | . | . | . | . | . | . | . | N | . | . | . | . | . | . | . | . | . | . |
| 21-225_80D3_HC | . | . | . | . | . | . | . | . | . | . | . | . | . | . | . | . | . | . | . | . | . | . | . | . | . | . |
| 21-225_80E9_HC | . | . | . | . | . | . | . | . | . | . | . | . | . | . | . | . | . | . | . | . | . | . | . | . | . | . |
| 21-225_81E5_HC | . | . | . | . | . | . | . | T | . | . | . | H | . | . | . | . | . | . | . | . | . | . | . | . | . | . |
| 21-225_81F9_HC | . | . | . | . | . | . | . | . | . | . | . | . | . | . | . | . | . | . | . | . | . | . | . | . | . | . |
| 21-225_82A5_HC | . | . | . | . | . | . | . | . | . | . | . | . | . | . | . | N | . | . | . | . | . | . | . | . | . | . |
| 21-225_82C12_HC | . | . | . | . | . | . | . | . | . | . | . | . | . | . | . | N | . | . | . | . | . | . | . | . | . | . |
| 21-225_82D9_HC | . | . | . | . | . | . | . | . | . | . | . | . | . | . | . | . | . | . | . | . | . | . | . | . | . | . |
| 21-225_82G5_HC | . | . | . | . | . | . | . | . | . | . | . | . | . | . | . | . | . | . | . | . | . | . | . | . | . | . |
| 21-225_83C2_HC | . | . | . | . | . | . | . | . | . | . | . | . | . | . | . | . | . | . | . | . | . | . | . | . | . | . |
| 21-225_83C5_HC | . | . | . | . | . | . | . | . | . | . | . | . | . | . | . | . | . | . | . | . | . | . | . | . | . | . |
| 21-225_83F3_HC | . | . | . | . | . | . | . | . | . | . | . | . | . | . | . | . | . | . | . | . | . | . | . | . | . | . |
| 21-225_83G3_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | M | T | R | N | T | S | I | S | T | A | Y | M | E | L | S | S | L | R | S | E | D | T | A | V | Y |
| 21-225_83G7_HC | . | . | . | . | . | . | . | . | . | . | . | . | . | . | . | . | . | . | . | . | . | . | . | . | . | . |
| 21-225_84G7_HC | . | . | . | . | . | . | . | . | . | . | . | . | . | . | . | . | . | . | . | . | . | . | . | . | . | . |
| 21-225_85B5_HC | . | . | . | . | . | . | . | . | . | . | . | . | . | . | . | . | . | . | . | . | . | . | . | . | . | . |
| 21-225_85D11_HC | . | . | . | . | . | . | . | . | . | . | . | . | . | . | . | . | . | . | . | . | . | . | . | . | . | . |
| 21-225_85H2_HC | . | L | . | . | . | . | . | . | . | . | . | . | . | . | . | . | . | . | . | . | . | . | . | . | . | . |
| 21-225_86E9_HC | . | . | . | . | S | . | . | T | . | . | . | H | . | . | . | . | . | . | . | . | . | . | . | . | . | . |
| 21-225_87A10_HC | . | . | . | . | . | . | . | . | . | . | . | . | . | . | . | N | . | . | . | . | . | . | . | . | . | . |
| 21-225_88G2_HC | . | . | . | . | . | . | . | . | . | . | . | . | . | . | . | . | . | . | . | . | . | . | . | . | . | . |
| 21-225_90D9_HC | . | . | . | . | . | . | . | . | . | . | . | . | . | . | . | . | . | . | . | . | . | . | . | . | . | . |
| 21-225_91F1_HC | . | . | . | . | . | . | . | . | . | . | . | . | . | . | . | . | . | . | . | . | . | . | . | . | . | . |
| 21-225_91G8_HC | . | . | . | . | . | . | . | . | . | . | . | . | . | . | . | . | . | . | . | . | . | . | . | . | . | . |
| 21-225_94D2_HC | . | . | . | . | . | . | . | . | . | . | . | . | . | . | . | . | . | . | . | . | . | . | . | . | . | . |
| 21-225_94E11_HC | . | . | . | . | . | . | . | T | . | . | . | H | . | . | . | . | . | . | . | . | . | . | . | . | . | . |
| 21-225_95E12_HC | . | . | . | . | . | . | . | . | . | . | . | . | . | . | . | . | . | . | . | . | . | . | . | . | . | . |
| 21-225_95G4_HC | . | . | . | . | . | . | . | T | . | . | . | H | . | . | . | . | . | . | . | . | . | . | . | . | . | . |
| 21-225_95H4_HC | . | . | . | . | . | . | . | T | . | . | . | H | . | . | . | . | . | . | . | . | . | . | . | . | . | . |
| 21-225_96D5_HC | . | . | . | . | S | . | . | T | . | . | . | H | . | . | . | . | . | . | . | . | . | . | . | . | . | . |
| 21-225_96E2_HC | . | L | . | . | . | . | . | . | . | . | . | . | . | . | . | . | . | . | . | . | . | . | . | . | . | . |
| 21-225_96G1_HC | . | . | . | . | . | . | . | . | . | . | . | . | . | . | . | N | . | . | . | . | . | . | . | . | . | . |
| 21-225_97H4_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | I | S | S | G | W | Y | - | - | - | - | - | - | - | - | - | - | - | - | - | - | - | - | - |
| 21-225_147G6_HC | . | . | . | Y | . | . | . | . | . | . | . | . | . | . | . | . | . | . | . | . | . | . | . | . | . | . |
| 21-225_152F4_HC | . | . | . | Y | . | . | . | . | . | . | . | . | . | . | . | . | . | . | . | . | . | . | . | . | . | . |
| 21-225_170F6_HC | . | . | . | . | . | . | . | . | . | . | . | . | . | . | . | . | . | . | . | . | . | . | . | . | . | . |
| 21-225_174G5_HC | . | . | . | V | . | . | . | . | . | . | . | . | . | . | . | . | . | . | . | . | . | . | . | . | . | . |
| 21-225_224H5_HC | . | . | . | S | . | . | . | . | N | . | . | . | . | . | . | . | . | . | . | . | . | . | . | . | . | . |
| 21-225_225F4_HC | . | . | . | S | . | . | . | . | . | . | . | . | . | . | . | . | . | . | . | . | . | . | . | . | . | . |
| 21-225_227G9_HC | . | . | . | L | . | . | . | . | . | . | . | . | . | . | . | . | . | . | . | . | . | . | . | . | . | . |
| 21-225_25A3_HC | . | . | . | S | . | . | . | . | . | . | . | . | . | . | . | . | . | . | . | . | . | . | . | . | . | . |
| 21-225_26A9_HC | . | . | . | S | . | . | . | . | . | . | . | . | . | . | . | . | . | . | . | . | . | . | . | . | . | . |
| 21-225_57A11_HC | . | . | . | . | . | . | . | . | N | . | . | . | . | . | . | . | . | . | . | . | . | . | . | . | . | . |
| 21-225_57F12_HC | . | . | . | . | . | . | . | . | . | . | . | . | . | . | . | . | . | . | . | . | . | . | . | . | . | . |
| 21-225_74A5_HC | . | . | . | . | . | . | . | . | . | . | . | . | . | . | . | . | . | . | . | . | . | . | . | . | . | . |
| 21-225_74A7_HC | . | . | . | V | . | . | . | . | N | . | . | . | . | . | . | . | . | . | . | . | . | . | . | . | . | . |
| 21-225_74B10_HC | . | . | . | V | . | . | . | . | . | . | . | . | . | . | . | . | . | . | . | . | . | . | . | . | . | . |
| 21-225_74B5_HC | . | . | . | V | . | . | . | . | N | . | . | . | . | . | . | . | . | . | . | . | . | . | . | . | . | . |
| 21-225_74B6_HC | . | . | . | . | . | . | . | . | . | . | . | . | . | . | . | . | . | . | . | . | . | . | . | . | . | . |
| 21-225_74B7_HC | . | . | . | V | . | . | . | . | H | . | . | . | . | . | . | . | . | . | . | . | . | . | . | . | . | . |
| 21-225_74B8_HC | . | . | . | V | . | . | . | . | . | . | . | . | . | . | . | . | . | . | . | . | . | . | . | . | . | . |
| 21-225_74C1_HC | . | . | . | V | . | . | . | . | . | . | . | . | . | . | . | . | . | . | . | . | . | . | . | . | . | . |
| 21-225_74C10_HC | . | . | . | . | . | . | . | . | . | . | . | . | . | . | . | . | . | . | . | . | . | . | . | . | . | . |
| 21-225_74C12_HC | . | . | . | . | . | . | . | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . | . | . | . | . | . | . | . |
| 21-225_74H2_HC | . | . | . | V | . | . | . | . | . | . | . | . | . | . | . | . | . | . | . | . | . | . | . | . | . | . |
| 21-225_75A7_HC | . | . | . | V | . | . | . | . | H | . | . | . | . | . | . | . | . | . | . | . | . | . | . | . | . | . |
| 21-225_75B1_HC | . | . | . | . | . | . | . | . | . | . | . | . | . | . | . | . | . | . | . | . | . | . | . | . | . | . |
| 21-225_76A6_HC | . | . | . | . | . | . | . | . | . | . | . | . | . | . | . | . | . | . | . | . | . | . | . | . | . | . |
| 21-225_76B9_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | I | S | S | G | W | Y | - | - | - | - | - | - | - | - | - | - | - | - | - | - | - | - | - |
| 21-225_76E8_HC | | | | V | | | | | H | | | | | | | | | | | | | | | | | |
| 21-225_76F3_HC | | | | V | | | | | N | | | | | | | | | | | | | | | | | |
| 21-225_76F5_HC | | | | S | | | | | | | | | | | | | | | | | | | | | | |
| 21-225_76H1_HC | | | | | | | | | | | | | | | | | | | | | | | | | | |
| 21-225_77C10_HC | | | | | | | | | | | | | | | | | | | | | | | | | | |
| 21-225_77C7_HC | | | | V | | | | | H | | | | | | | | | | | | | | | | | |
| 21-225_77D11_HC | | | | | | | | | | | | | | | | | | | | | | | | | | |
| 21-225_77D12_HC | | | | | | | | | | | | | | | | | | | | | | | | | | |
| 21-225_77E1_HC | | | | V | | | | | H | | | | | | | | | | | | | | | | | |
| 21-225_78C1_HC | | | | | | | | | | | | | | | | | | | | | | | | | | |
| 21-225_78H12_HC | | | | V | | | | | H | | | | | | | | | | | | | | | | | |
| 21-225_79G10_HC | | | | V | | | | | N | | | | | | | | | | | | | | | | | |
| 21-225_79G6_HC | | | | V | | | | | | | | | | | | | | | | | | | | | | |
| 21-225_80A2_HC | | | | | | | | | | | | | | | | | | | | | | | | | | |
| 21-225_80C12_HC | | | | | | | | | | | | | | | | | | | | | | | | | | |
| 21-225_80D3_HC | | | | | | | | | | | | | | | | | | | | | | | | | | |
| 21-225_80E9_HC | | | | | | | | | | | | | | | | | | | | | | | | | | |
| 21-225_81E5_HC | | | | V | | | | | N | | | | | | | | | | | | | | | | | |
| 21-225_81F9_HC | | | | | | | | | | | | | | | | | | | | | | | | | | |
| 21-225_82A5_HC | | | | | | | | | | | | | | | | | | | | | | | | | | |
| 21-225_82C12_HC | | | | | | | | | | | | | | | | | | | | | | | | | | |
| 21-225_82D9_HC | | | | | | | | | | | | | | | | | | | | | | | | | | |
| 21-225_82G5_HC | | | | | | | | | | | | | | | | | | | | | | | | | | |
| 21-225_83C2_HC | | | | | | | | | | | | | | | | | | | | | | | | | | |
| 21-225_83C5_HC | | | | | | | | | | | | | | | | | | | | | | | | | | |
| 21-225_83F3_HC | | | | | | | | | | | | | | | | | | | | | | | | | | |
| 21-225_83G3_HC | | | | | | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | I | S | S | G | W | Y | - | - | - | - | - | - | - | - | - | - | - | - | - | - | - | - | - |
| 21-225_83G7_HC | | | | V | | | | | H | | | | | | | | | | | | | | | | | |
| 21-225_84G7_HC | | | | V | | | | | H | | | | | | | | | | | | | | | | | |
| 21-225_85B5_HC | | | | | | | | | | | | | | | | | | | | | | | | | | |
| 21-225_85D11_HC | | | | | | | | | | | | | | | | | | | | | | | | | | |
| 21-225_85H2_HC | | | | V | | | | | | | | | | | | | | | | | | | | | | |
| 21-225_86E9_HC | | | | V | | | | | S | | | | | | | | | | | | | | | | | |
| 21-225_87A10_HC | | | | | | | | | | | | | | | | | | | | | | | | | | |
| 21-225_88G2_HC | | | | | | | | | | | | | | | | | | | | | | | | | | |
| 21-225_90D9_HC | | | | V | | | | | H | | | | | | | | | | | | | | | | | |
| 21-225_91F1_HC | | | | | | | | | | | | | | | | | | | | | | | | | | |
| 21-225_91G8_HC | | | | | | | | | | | | | | | | | | | | | | | | | | |
| 21-225_94D2_HC | | | | V | | | | | | | | | | | | | | | | | | | | | | |
| 21-225_94E11_HC | | | | V | | | | | K | | | | | | | | | | | | | | | | | |
| 21-225_95E12_HC | | | | | | | | | | | | | | | | | | | | | | | | | | |
| 21-225_95G4_HC | | | | V | | | | | N | | | | | | | | | | | | | | | | | |
| 21-225_95H4_HC | | | | V | | | | | N | | | | | | | | | | | | | | | | | |
| 21-225_96D5_HC | | | | V | | | | | S | | | | | | | | | | | | | | | | | |
| 21-225_96E2_HC | | | | V | | | | | | | | | | | | | | | | | | | | | | |
| 21-225_96G1_HC | | | | | | | | | | | | | | | | | | | | | | | | | | |
| 21-225_97H4_HC | | | | | | | | | | | | | | | | | | | | | | | | | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | W | F | D | P | W | G | Q | G | T | L | V | T | V | S | S |
| 21-225_147G6_HC | . | . | . | . | . | . | . | . | . | . | . | . | . | . | . | . | . | . | . |
| 21-225_152F4_HC | . | . | . | . | . | . | . | . | . | . | . | . | . | . | . | . | . | . | . |
| 21-225_170F6_HC | . | . | . | . | . | . | . | . | . | . | . | . | . | . | . | . | . | . | . |
| 21-225_174G5_HC | . | . | . | . | . | . | . | . | . | . | . | . | . | . | . | . | . | . | . |
| 21-225_224H5_HC | . | . | . | . | . | . | . | . | . | . | . | . | . | . | . | . | . | . | . |
| 21-225_225F4_HC | . | . | . | . | . | . | . | . | . | . | . | . | . | . | . | . | . | . | . |
| 21-225_227G9_HC | . | . | . | . | . | . | . | . | . | . | . | . | . | . | . | . | . | . | . |
| 21-225_25A3_HC | . | . | . | . | . | . | . | . | . | . | . | . | . | . | . | . | . | . | . |
| 21-225_26A9_HC | . | . | . | . | . | . | . | . | . | . | . | . | . | . | . | . | . | . | . |
| 21-225_57A11_HC | . | . | . | . | . | . | . | . | . | . | . | . | . | . | . | . | . | . | . |
| 21-225_57F12_HC | . | . | . | . | R | . | . | . | . | . | . | . | . | . | . | . | . | . | . |
| 21-225_74A5_HC | . | . | . | . | . | . | . | . | . | . | . | . | . | . | . | . | . | . | . |
| 21-225_74A7_HC | . | . | . | . | . | . | . | . | . | . | . | . | . | . | . | . | . | . | . |
| 21-225_74B10_HC | . | . | . | . | . | . | . | . | . | . | . | . | . | . | . | . | . | . | . |
| 21-225_74B5_HC | . | . | . | . | . | . | . | . | . | . | . | . | . | . | . | . | . | . | . |
| 21-225_74B6_HC | . | . | . | . | . | . | . | . | . | . | . | . | . | . | . | . | . | . | L |
| 21-225_74B7_HC | . | . | . | . | . | . | . | . | . | . | . | . | . | . | . | . | . | A | . |
| 21-225_74B8_HC | . | . | . | . | . | . | . | . | . | . | . | . | . | . | . | . | . | . | . |
| 21-225_74C1_HC | . | . | . | . | . | . | . | . | . | . | . | . | . | . | . | . | . | . | . |
| 21-225_74C10_HC | . | . | . | . | . | . | . | . | . | . | . | . | . | . | . | . | . | . | . |
| 21-225_74C12_HC | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . |
| 21-225_74H2_HC | . | . | . | . | . | . | . | . | . | . | . | . | . | . | . | . | . | . | . |
| 21-225_75A7_HC | . | . | . | . | . | . | . | . | . | . | . | . | . | . | . | . | . | A | . |
| 21-225_75B1_HC | . | . | . | . | . | . | . | . | . | . | . | . | . | . | . | . | . | . | . |
| 21-225_76A6_HC | . | . | . | . | . | . | . | . | . | . | . | . | . | . | . | . | . | . | L |
| 21-225_76B9_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | W | F | D | P | W | G | Q | G | T | L | V | T | V | S | S |
| 21-225_76E8_HC | . | . | . | . | . | . | . | . | . | . | . | . | . | . | . | . | . | A | . |
| 21-225_76F3_HC | . | . | . | . | . | . | . | . | . | . | . | . | . | . | . | . | . | . | . |
| 21-225_76F5_HC | . | . | . | . | . | . | . | . | . | . | . | . | . | . | . | . | . | . | . |
| 21-225_76H1_HC | . | . | . | . | . | . | . | . | . | . | . | . | . | . | . | . | . | . | . |
| 21-225_77C10_HC | . | . | . | . | . | . | . | . | . | . | . | . | . | . | . | . | . | . | . |
| 21-225_77C7_HC | . | . | . | . | . | . | . | . | . | . | . | . | . | . | . | . | . | A | . |
| 21-225_77D11_HC | . | . | . | . | . | . | . | . | . | . | . | . | . | . | . | . | . | . | . |
| 21-225_77D12_HC | . | . | . | . | . | . | . | . | . | . | . | . | . | . | . | . | . | . | . |
| 21-225_77E1_HC | . | . | . | . | . | . | . | . | . | . | . | . | . | . | . | . | . | A | . |
| 21-225_78C1_HC | . | . | . | . | . | . | . | . | . | . | . | . | . | . | . | . | . | . | . |
| 21-225_78H12_HC | . | . | . | . | . | . | . | . | . | . | . | . | . | . | . | . | . | A | . |
| 21-225_79G10_HC | . | . | . | . | . | . | . | . | . | . | . | . | . | . | . | . | . | . | . |
| 21-225_79G6_HC | . | . | . | . | . | . | . | . | . | . | . | . | . | . | . | . | . | . | . |
| 21-225_80A2_HC | . | . | . | . | . | . | . | . | . | . | . | . | . | . | . | . | . | . | . |
| 21-225_80C12_HC | . | . | . | . | . | . | . | . | . | . | . | . | . | . | . | . | . | . | L |
| 21-225_80D3_HC | . | . | . | . | . | . | . | . | . | . | . | . | . | . | . | . | . | . | . |
| 21-225_80E9_HC | . | . | . | . | . | . | . | . | . | . | . | . | . | . | . | . | . | . | . |
| 21-225_81E5_HC | . | . | . | . | . | . | . | . | . | . | . | . | . | . | . | . | . | . | . |
| 21-225_81F9_HC | . | . | . | . | . | . | . | . | . | . | . | . | . | . | . | . | . | . | . |
| 21-225_82A5_HC | . | . | . | . | . | . | . | . | . | . | . | . | . | . | . | . | . | . | L |
| 21-225_82C12_HC | . | . | . | . | . | . | . | . | . | . | . | . | . | . | . | . | . | . | L |
| 21-225_82D9_HC | . | . | . | . | . | . | . | . | . | . | . | . | . | . | . | . | . | . | . |
| 21-225_82G5_HC | . | . | . | . | . | . | . | . | . | . | . | . | . | . | . | . | . | . | . |
| 21-225_83C2_HC | . | . | . | . | . | . | . | . | . | . | . | . | . | . | . | . | . | . | . |
| 21-225_83C5_HC | . | . | . | . | . | . | . | . | . | . | . | . | . | . | . | . | . | . | . |
| 21-225_83F3_HC | . | . | . | . | . | . | . | . | . | . | . | . | . | . | . | . | . | . | . |
| 21-225_83G3_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | W | F | D | P | W | G | Q | G | T | L | V | T | V | S | S |
| 21-225_83G7_HC | | | | | | | | | | | | | | | | | | A | |
| 21-225_84G7_HC | | | | | | | | | | | | | | | | | | A | |
| 21-225_85B5_HC | | | | | | | | | | | | | | | | | | | |
| 21-225_85D11_HC | | | | | | | | | | | | | | | | | | | |
| 21-225_85H2_HC | | | | | | | | | | | | | | | | | | | |
| 21-225_86E9_HC | | | | | | | | | | | | | | | | | | | |
| 21-225_87A10_HC | | | | | | | | | | | | | | | | | | | L |
| 21-225_88G2_HC | | | | | | | | | | | | | | | | | | | |
| 21-225_90D9_HC | | | | | | | | | | | | | | | | | | A | |
| 21-225_91F1_HC | | | | | | | | | | | | | | | | | | | |
| 21-225_91G8_HC | | | | | | | | | | | | | | | | | | | |
| 21-225_94D2_HC | | | | | | | | | | | | | | | | | | | |
| 21-225_94E11_HC | | | | | | | | | | | | | | | | | | | |
| 21-225_95E12_HC | | | | | | | | | | | | | | | | | | | |
| 21-225_95G4_HC | | | | | | | | | | | | | | | | | | | |
| 21-225_95H4_HC | | | | | | | | | | | | | | | | | | | |
| 21-225_96D5_HC | | | | | | | | | | | | | | | | | | | |
| 21-225_96E2_HC | | | | | | | | | | | | | | | | | | | |
| 21-225_96G1_HC | | | | | | | | | | | | | | | | | | | L |
| 21-225_97H4_HC | | | | | | | | | | | | | | | | | | | |

| Reference # | 139 | 140 | 141 | 142 | 143 | 144 | 145 | 146 | 147 | 148 | 149 |
|---|---|---|---|---|---|---|---|---|---|---|---|
| | | | | | H_FR4 | | | | | | |
| CONSENSUS | W | G | Q | G | T | L | V | T | V | S | S |
| 21-225_147G6_HC | . | . | . | . | . | . | . | . | . | . | . |
| 21-225_152F4_HC | . | . | . | . | . | . | . | . | . | . | . |
| 21-225_170F6_HC | . | . | . | . | . | . | . | . | . | . | . |
| 21-225_174G5_HC | . | . | . | . | . | . | . | . | . | . | . |
| 21-225_224H5_HC | . | . | . | . | . | . | . | . | . | . | . |
| 21-225_225F4_HC | . | . | . | . | . | . | . | . | . | . | . |
| 21-225_227G9_HC | . | . | . | . | . | . | . | . | . | . | . |
| 21-225_25A3_HC | . | . | . | . | . | . | . | . | . | . | . |
| 21-225_26A9_HC | . | . | . | . | . | . | . | . | . | . | . |
| 21-225_57A11_HC | . | . | . | . | . | . | . | . | . | . | . |
| 21-225_57F12_HC | . | . | . | . | . | . | . | . | . | . | . |
| 21-225_74A5_HC | . | . | . | . | . | . | . | . | . | . | . |
| 21-225_74A7_HC | . | . | . | . | . | . | . | . | . | . | . |
| 21-225_74B10_HC | . | . | . | . | . | . | . | . | . | . | . |
| 21-225_74B5_HC | . | . | . | . | . | . | . | . | . | . | . |
| 21-225_74B6_HC | . | . | . | . | . | . | . | . | . | . | L |
| 21-225_74B7_HC | . | . | . | . | . | . | . | . | A | . | . |
| 21-225_74B8_HC | . | . | . | . | . | . | . | . | . | . | . |
| 21-225_74C1_HC | . | . | . | . | . | . | . | . | . | . | . |
| 21-225_74C10_HC | . | . | . | . | . | . | . | . | . | . | . |
| 21-225_74C12_HC | . | . | . | . | . | . | . | . | . | . | . |
| 21-225_74G3_HC | . | . | . | . | . | . | . | . | . | . | . |
| 21-225_74H2_HC | . | . | . | . | . | . | . | . | . | . | . |
| 21-225_75A7_HC | . | . | . | . | . | . | . | . | A | . | . |
| 21-225_75B1_HC | . | . | . | . | . | . | . | . | . | . | . |
| 21-225_76A6_HC | . | . | . | . | . | . | . | . | . | . | L |
| 21-225_76B9_HC | . | . | . | . | . | . | . | . | . | . | . |

| Reference # | 139 | 140 | 141 | 142 | 143 | 144 | 145 | 146 | 147 | 148 | 149 |
|---|---|---|---|---|---|---|---|---|---|---|---|
| | | | | | | H_FR4 | | | | | |
| CONSENSUS | W | G | Q | G | T | L | V | T | V | S | S |
| 21-225_76E8_HC | . | . | . | . | . | . | . | . | . | A | . |
| 21-225_76F3_HC | . | . | . | . | . | . | . | . | . | . | . |
| 21-225_76F5_HC | . | . | . | . | . | . | . | . | . | . | . |
| 21-225_76H1_HC | . | . | . | . | . | . | . | . | . | . | . |
| 21-225_77C10_HC | . | . | . | . | . | . | . | . | . | . | . |
| 21-225_77C7_HC | . | . | . | . | . | . | . | . | . | A | . |
| 21-225_77D11_HC | . | . | . | . | . | . | . | . | . | . | . |
| 21-225_77D12_HC | . | . | . | . | . | . | . | . | . | . | . |
| 21-225_77E1_HC | . | . | . | . | . | . | . | . | . | A | . |
| 21-225_78C1_HC | . | . | . | . | . | . | . | . | . | . | . |
| 21-225_78H12_HC | . | . | . | . | . | . | . | . | . | A | . |
| 21-225_79G10_HC | . | . | . | . | . | . | . | . | . | . | . |
| 21-225_79G6_HC | . | . | . | . | . | . | . | . | . | . | . |
| 21-225_80A2_HC | . | . | . | . | . | . | . | . | . | . | . |
| 21-225_80C12_HC | . | . | . | . | . | . | . | . | . | . | L |
| 21-225_80D3_HC | . | . | . | . | . | . | . | . | . | . | . |
| 21-225_80E9_HC | . | . | . | . | . | . | . | . | . | . | . |
| 21-225_81E5_HC | . | . | . | . | . | . | . | . | . | . | . |
| 21-225_81F9_HC | . | . | . | . | . | . | . | . | . | . | . |
| 21-225_82A5_HC | . | . | . | . | . | . | . | . | . | . | L |
| 21-225_82C12_HC | . | . | . | . | . | . | . | . | . | . | L |
| 21-225_82D9_HC | . | . | . | . | . | . | . | . | . | . | . |
| 21-225_82G5_HC | . | . | . | . | . | . | . | . | . | . | . |
| 21-225_83C2_HC | . | . | . | . | . | . | . | . | . | . | . |
| 21-225_83C5_HC | . | . | . | . | . | . | . | . | . | . | . |
| 21-225_83F3_HC | . | . | . | . | . | . | . | . | . | . | . |
| 21-225_83G3_HC | . | . | . | . | . | . | . | . | . | . | . |

FIGURE 57

| Reference # | 139 | 140 | 141 | 142 | 143 | 144 | 145 | 146 | 147 | 148 | 149 |
|---|---|---|---|---|---|---|---|---|---|---|---|
| | | | | | | H_FR4 | | | | | |
| CONSENSUS | W | G | Q | G | T | L | V | T | V | S | S |
| 21-225_83G7_HC | . | . | . | . | . | . | . | . | . | A | . |
| 21-225_84G7_HC | . | . | . | . | . | . | . | . | . | A | . |
| 21-225_85B5_HC | . | . | . | . | . | . | . | . | . | . | . |
| 21-225_85D11_HC | . | . | . | . | . | . | . | . | . | . | . |
| 21-225_85H2_HC | . | . | . | . | . | . | . | . | . | . | . |
| 21-225_86E9_HC | . | . | . | . | . | . | . | . | . | . | . |
| 21-225_87A10_HC | . | . | . | . | . | . | . | . | . | . | L |
| 21-225_88G2_HC | . | . | . | . | . | . | . | . | . | . | . |
| 21-225_90D9_HC | . | . | . | . | . | . | . | . | . | A | . |
| 21-225_91F1_HC | . | . | . | . | . | . | . | . | . | . | . |
| 21-225_91G8_HC | . | . | . | . | . | . | . | . | . | . | . |
| 21-225_94D2_HC | . | . | . | . | . | . | . | . | . | . | . |
| 21-225_94E11_HC | . | . | . | . | . | . | . | . | . | . | . |
| 21-225_95E12_HC | . | . | . | . | . | . | . | . | . | . | . |
| 21-225_95G4_HC | . | . | . | . | . | . | . | . | . | . | . |
| 21-225_95H4_HC | . | . | . | . | . | . | . | . | . | . | . |
| 21-225_96D5_HC | . | . | . | . | . | . | . | . | . | . | . |
| 21-225_96E2_HC | . | . | . | . | . | . | . | . | . | . | . |
| 21-225_96G1_HC | . | . | . | . | . | . | . | . | . | . | L |
| 21-225_97H4_HC | . | . | . | . | . | . | . | . | . | . | . |

FIGURE 57

Table 54.     Consensus 20- VH3|3-33/D4|4-17|RF2/JH6 (SEQ ID NO: 50271):

QVQLVES-GGGVVQPGRSLRLSCAASG-FTFSS-----YGMHWVRQAPGKGLEWVAVIWYD---
GSNKNYADSVKGRFTISRDNSKNTLYLQMNSLRAEDTAVYYCARDQGVGY------------------YGLDVWGQGTTVTVSS wherein:

S at position 33 can be substituted with N, R, T or I

Y at position 39 can be substituted with H or N

H at position 42 can be substituted with D

V at position 57 can be substituted with I

I at position 58 can be substituted with L

Y at position 60 can be substituted with F

S at position 66 can be substituted with T

K at position 68 can be substituted with E, Q, D or R

N at position 69 can be substituted with H or Y

Y at position 70 can be substituted with H

A at position 71 can be substituted with V or G

D at position 72 can be substituted with E

D at position 109 can be substituted with A

Q at position 110 can be substituted with R, Y, H, A, C, E, F or M

V at position 112 can be substituted with I or F

G at position 113 can be substituted with Y

FIGURE 57

Y at position 114 can be substituted with E

null (-) at position 115 can be substituted with F

null (-) at position 116 can be substituted with D

null (-) at position 131 can be substituted with Y

null (-) at position 132 can be substituted with Y

null (-) at position 133 can be substituted with N

Y at position 134 can be substituted with D, or N

G at position 135 can be substituted with A or D

L at position 136 can be substituted with M, T or I

Xaa Xaa Gly Met Xaa (SEQ ID NO: 50016)

Xaa Xaa Trp Xaa Asp Gly Xaa Asn Xaa Xaa Xaa Xaa Xaa Ser Val Lys Gly (SEQ ID NO: 50017)

Xaa Xaa Gly Xaa Xaa Xaa Xaa Xaa Xaa Xaa Xaa Xaa Xaa Xaa Asp Val (SEQ ID NO: 50018)

Ser Tyr Gly Met His (SEQ ID NO: 50483)

Val Ile Trp Tyr Asp Gly Ser Asn Lys Asn Tyr Ala Asp Ser Val Lys Gly (SEQ ID NO: 50484)

Asp Gln Gly Val Gly Tyr Tyr Gly Leu Asp Val (SEQ ID NO: 50485)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | . | . | . | . | . | . | . | . | . | . | . | . | . | K | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | . | . | . | . | . | . | . | . | . | . | . | . | . | . | . | . | . | . | . | . |
| 21-225_190G11_HC | . | . | . | . | . | . | . | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | . | . |
| 21-225_191A10_HC | . | . | . | . | . | . | . | . | . | . | . | . | . | . | . | . | . | . | . | . | . | . | . | . | . | . |
| 21-225_191E10_HC | . | . | . | . | . | . | . | . | . | . | . | . | . | . | . | . | . | . | . | . | . | . | . | . | . | . |
| 21-225_191E8_HC | . | . | . | . | . | . | . | . | . | . | . | . | . | . | . | . | . | . | . | . | . | . | . | . | . | . |
| 21-225_191G1_HC | . | . | . | . | . | . | . | . | . | . | . | . | . | . | . | . | . | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | . | . | . | . | . | . | . | . | . | . | . |
| 21-225_192C8_HC | . | . | . | . | . | . | . | . | . | . | . | . | . | . | . | . | . | . | . | . | . | . | . | . | . | . |
| 21-225_192D2_HC | . | . | . | . | . | . | . | . | . | . | . | . | . | . | . | . | . | . | . | . | . | . | . | . | . | . |
| 21-225_192D3_HC | . | . | . | . | . | . | . | . | . | . | . | . | . | . | . | . | . | . | . | . | . | . | . | . | . | . |
| 21-225_192G6_HC | . | . | . | . | . | . | . | . | . | . | . | . | . | . | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | . | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | . | . | . | . | . | . | . | . | . | . | . | . | . | . | . | . | . | . | . | . | . |
| 21-225_193C7_HC | . | . | . | . | . | . | . | . | . | . | . | . | . | . | . | . | . | . | . | . | . | . | . | . | . | . |
| 21-225_194H11_HC | . | . | . | . | . | . | . | . | . | . | . | . | . | . | . | . | . | . | . | . | . | . | . | . | . | . |
| 21-225_195A9_HC | . | . | . | . | . | . | . | . | . | . | . | . | . | . | . | . | . | . | . | . | . | . | . | . | . | . |
| 21-225_196C7_HC | . | . | . | . | . | . | . | . | . | . | . | . | . | . | . | . | . | . | . | . | . | . | . | . | . | . |
| 21-225_197B6_HC | . | . | . | . | . | . | . | . | . | . | . | . | . | . | . | . | . | . | . | . | . | . | . | . | . | . |
| 21-225_197D3_HC | . | . | . | . | . | . | . | . | . | . | . | . | . | . | . | . | . | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_199A6_HC | . | . | . | . | . | . | . | . | . | . | . | . | . | . | . | . | . | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | . | . | . | . | . | . | . | . | . | . | . | . | . | . | . | . | . | . | T | . | . |
| 21-225_205F5_HC | . | . | . | . | . | . | . | . | . | . | . | . | . | . | . | . | . | . | . | . | . | . | . | . | . | . |
| 21-225_207G6_HC | . | . | . | . | . | . | . | . | . | . | . | . | . | . | . | . | . | . | . | . | . | . | . | . | . | . |
| 21-225_211A11_HC | . | . | . | . | . | G | . | . | . | . | . | . | . | . | . | . | . | . | . | . | . | . | . | . | . | . |
| 21-225_211A8_HC | . | . | . | . | . | . | . | . | . | . | . | . | . | . | . | . | . | . | . | . | . | . | . | . | . | . |
| 21-225_212D7_HC | . | . | . | . | . | . | . | . | . | . | . | . | . | . | . | . | . | . | . | . | . | . | . | . | . | . |
| 21-225_212E10_HC | . | . | . | . | . | . | . | . | . | . | . | . | . | . | . | . | . | . | . | . | . | . | . | . | . | . |
| 21-225_212E6_HC | . | . | . | . | . | . | . | . | . | . | . | . | . | . | . | . | . | . | . | . | . | . | . | . | . | . |
| 21-225_212F10_HC | . | . | . | . | . | . | . | . | . | . | . | . | . | . | . | . | . | . | . | . | . | . | . | . | . | . |
| 21-225_212H9_HC | . | . | . | . | . | . | . | . | . | . | . | . | . | . | . | . | . | . | . | . | . | . | . | . | . | . |
| 21-225_213B8_HC | . | . | . | . | . | . | . | . | . | . | . | . | . | . | . | . | . | . | . | . | . | . | . | . | . | . |
| 21-225_213C4_HC | . | . | . | . | . | . | . | . | . | . | . | . | . | . | . | . | . | . | . | . | . | . | . | T | . | . |
| 21-225_213D2_HC | . | . | . | . | . | . | . | . | . | . | . | . | . | . | . | . | . | . | . | . | . | . | . | . | . | . |
| 21-225_214C3_HC | . | . | . | . | . | . | . | . | . | . | . | . | . | . | . | . | . | . | . | . | . | . | . | E | . | . |
| 21-225_215A12_HC | . | . | . | . | . | . | . | . | . | . | . | . | . | . | . | . | . | . | . | . | . | . | . | . | . | . |
| 21-225_215B5_HC | . | . | . | . | . | . | . | . | . | . | . | . | . | . | . | . | . | . | . | . | . | . | . | . | . | . |
| 21-225_215D6_HC | . | . | . | . | . | . | . | . | . | . | . | . | . | . | . | . | . | . | . | . | . | . | . | . | . | . |
| 21-225_217G5_HC | . | . | . | . | . | . | . | . | . | . | . | . | . | . | . | . | . | . | . | . | . | . | . | . | . | . |
| 21-225_219H1_HC | . | . | . | . | . | . | . | . | . | . | . | . | . | . | . | . | . | . | . | . | . | . | . | . | . | . |
| 21-225_223D11_HC | . | . | . | . | . | . | . | . | . | . | . | . | . | . | . | . | . | . | . | . | . | . | . | . | . | . |
| 21-225_74D1_HC | . | . | . | . | . | . | . | . | . | . | . | . | . | . | . | . | . | . | . | . | . | . | . | . | . | . |
| 21-225_87B7_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | G | M | H | W | V | R | Q | A | P | G | K | G | L |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | T | . | . | . | . | . | . | . | . | . | . | . | . | . | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | T | . | . | . | . | . | . | . | . | . | . | . | . | . | . | . | . | . | . | . |
| 21-225_190G11_HC | . | . | . | . | . | . | I | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | . | . |
| 21-225_191A10_HC | . | . | . | . | . | . | . | . | . | . | . | . | . | . | . | . | . | . | . | . | . | . | . | . | . | . |
| 21-225_191E10_HC | . | . | . | . | . | . | . | . | . | . | . | . | . | . | . | . | . | . | . | . | . | . | . | . | . | . |
| 21-225_191E8_HC | . | . | . | . | . | . | N | . | . | . | . | . | . | . | . | . | . | . | . | . | . | . | . | . | . | . |
| 21-225_191G1_HC | . | . | . | . | . | . | . | . | . | . | . | . | . | . | . | . | . | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | . | . | . | . | . | . | . | . | . | . | . |
| 21-225_192C8_HC | . | . | . | . | . | . | . | . | . | . | . | . | . | . | . | . | . | . | . | . | . | . | . | . | . | . |
| 21-225_192D2_HC | . | . | . | . | . | . | . | . | . | . | . | . | . | . | . | . | . | . | . | . | . | . | . | . | . | . |
| 21-225_192D3_HC | . | . | . | . | . | . | . | . | . | . | . | . | . | . | . | . | . | . | . | . | . | . | . | . | . | . |
| 21-225_192G6_HC | . | . | . | . | . | . | . | . | . | . | . | . | . | . | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | . | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | . | . | . | . | . | . | . | . | . | . | . | . | . | . | . | . | . | . | . | . | . |
| 21-225_193C7_HC | . | . | . | . | . | . | . | . | . | . | . | . | . | . | . | . | . | . | . | . | . | . | . | . | . | . |
| 21-225_194H11_HC | . | . | . | . | . | . | . | . | . | . | . | . | . | . | . | . | . | . | . | . | . | . | . | . | . | . |
| 21-225_195A9_HC | . | . | . | . | . | . | . | . | . | . | . | . | . | . | . | . | . | . | . | . | . | . | . | . | . | . |
| 21-225_196C7_HC | . | . | . | . | . | . | . | . | . | . | . | . | . | . | . | . | . | . | . | . | . | . | . | . | . | . |
| 21-225_197B6_HC | . | . | . | . | . | . | . | . | . | . | . | . | . | . | . | . | . | . | . | . | . | . | . | . | . | . |
| 21-225_197D3_HC | . | . | . | I | . | . | . | . | . | . | . | . | H | . | . | . | . | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | G | M | H | W | V | R | Q | A | P | G | K | G | L |
| 21-225_199A6_HC | . | . | . | . | . | . | N | . | . | . | . | . | . | . | . | . | . | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | . | . | . | . | . | . | . | . | . | . | . | . | . | . | . | . | . | . | . | . | . |
| 21-225_205F5_HC | . | . | . | . | . | . | R | . | . | . | . | . | . | . | . | . | . | . | . | . | . | . | . | . | . | . |
| 21-225_207G6_HC | . | . | . | . | . | . | . | . | . | . | . | . | . | . | . | . | . | . | . | . | . | . | . | . | . | . |
| 21-225_211A11_HC | . | . | . | . | . | . | . | . | . | . | . | . | . | . | . | . | . | . | . | . | . | . | . | . | . | . |
| 21-225_211A8_HC | . | . | . | . | . | . | . | . | . | . | . | . | . | . | . | . | . | . | . | . | . | . | . | . | . | . |
| 21-225_212D7_HC | . | . | . | . | . | . | . | . | . | . | . | . | . | . | . | . | . | . | . | . | . | . | . | . | . | . |
| 21-225_212E10_HC | . | . | . | . | . | . | . | . | . | . | . | . | . | . | . | . | . | . | . | . | . | . | . | . | . | . |
| 21-225_212E6_HC | . | . | . | . | . | . | . | . | . | . | . | . | . | . | . | . | . | . | . | . | . | . | . | . | . | . |
| 21-225_212F10_HC | . | . | . | . | . | . | R | . | . | . | . | . | . | . | . | . | . | . | . | . | . | . | . | . | . | . |
| 21-225_212H9_HC | . | . | . | . | . | . | . | . | . | . | . | . | . | . | . | . | . | . | . | . | . | . | . | . | . | . |
| 21-225_213B8_HC | . | . | . | . | . | . | . | . | . | . | . | . | . | . | . | . | . | . | . | . | . | . | . | . | . | . |
| 21-225_213C4_HC | . | . | . | . | . | . | . | . | . | . | . | . | . | . | . | . | . | . | . | . | . | . | . | . | . | . |
| 21-225_213D2_HC | . | . | . | . | . | . | . | . | . | . | . | . | . | . | . | . | . | . | . | . | . | . | . | . | . | . |
| 21-225_214C3_HC | . | . | . | . | . | . | . | . | . | . | . | . | . | . | . | . | . | . | . | . | . | . | . | . | . | . |
| 21-225_215A12_HC | . | . | L | . | . | . | . | . | . | . | . | . | . | . | . | . | . | . | . | . | . | . | . | . | . | . |
| 21-225_215B5_HC | . | . | . | . | . | . | . | . | . | . | . | . | . | . | . | . | . | . | . | . | . | . | . | . | . | . |
| 21-225_215D6_HC | . | . | . | . | . | . | . | . | . | . | . | . | . | . | . | . | . | . | . | . | . | . | . | . | . | . |
| 21-225_217G5_HC | . | . | . | . | . | . | . | . | . | . | . | . | . | N | . | . | . | . | . | . | . | . | . | . | . | . |
| 21-225_219H1_HC | . | . | . | . | . | . | R | . | . | . | . | . | . | . | . | . | . | . | . | . | . | . | . | . | . | . |
| 21-225_223D11_HC | . | . | . | . | . | . | N | . | . | . | . | . | . | . | . | D | . | . | . | . | . | . | . | . | . | . |
| 21-225_74D1_HC | . | . | . | . | . | . | . | . | . | . | . | . | . | . | . | . | . | . | . | . | . | . | . | . | . | . |
| 21-225_87B7_HC | . | . | . | . | . | . | . | . | . | . | . | . | . | . | . | . | . | . | . | . | . | . | . | . | . | . |

3800

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H CDR2 | | | | | | | | | | | | |
| CONSENSUS | E | W | V | A | V | I | W | Y | D | - | - | - | G | S | N | K | N | Y | A | D | S | V | K | G | R | F |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | . | . | . | . | . | . | . | T | . | . | . | . | . | . | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | . | . | . | . | . | . | . | . | . | . | . | . | . | . | . | . | . | . | . | . |
| 21-225_190G11_HC | . | . | . | . | . | . | . | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | . | . |
| 21-225_191A10_HC | . | . | . | . | . | . | . | . | . | . | . | . | . | . | . | . | . | . | . | . | . | . | . | . | . | . |
| 21-225_191E10_HC | . | . | . | . | . | . | . | . | . | . | . | . | . | . | . | . | . | . | . | . | . | . | . | . | . | . |
| 21-225_191E8_HC | . | . | . | . | I | . | . | F | . | . | . | . | . | . | . | Q | Y | . | . | . | . | . | . | . | . | . |
| 21-225_191G1_HC | . | . | . | . | . | . | . | . | . | . | . | . | . | . | . | E | H | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | E | H | . | . | . | . | . | . | . | . | . |
| 21-225_192C8_HC | . | . | . | . | . | . | . | . | . | . | . | . | . | . | . | E | H | . | . | . | . | . | . | . | . | . |
| 21-225_192D2_HC | . | . | . | . | . | . | . | . | . | . | . | . | . | . | . | . | . | . | . | . | . | . | . | . | . | . |
| 21-225_192D3_HC | . | . | . | . | . | . | . | . | . | . | . | . | . | . | . | E | H | . | . | . | . | . | . | . | . | . |
| 21-225_192G6_HC | . | . | . | . | L | . | . | . | . | . | . | . | . | T | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | . | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | . | . | . | . | . | . | . | . | . | . | E | H | . | . | . | . | . | . | . | . | . |
| 21-225_193C7_HC | . | . | . | . | . | . | . | . | . | . | . | . | . | . | . | . | . | . | . | . | . | . | . | . | . | . |
| 21-225_194H11_HC | . | . | . | . | . | . | . | . | . | . | . | . | . | . | . | E | H | . | . | . | . | . | . | . | . | . |
| 21-225_195A9_HC | Q | . | . | . | . | . | . | . | . | . | . | . | . | . | . | E | H | . | . | . | . | . | . | . | . | . |
| 21-225_196C7_HC | . | . | . | . | . | . | . | . | . | . | . | . | . | . | . | E | H | . | . | . | . | . | . | . | . | . |
| 21-225_197B6_HC | . | . | . | . | . | . | . | . | . | . | . | . | . | . | . | . | . | . | . | . | . | . | . | . | . | . |
| 21-225_197D3_HC | . | . | . | . | . | . | . | . | . | . | . | . | . | . | . | E | H | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | H | . | . | . | . | . | . | . | . | . |

3801

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | V | A | V | I | W | Y | D | . | . | . | G | S | N | K | N | Y | A | D | S | V | K | G | R | F |
| 21-225_199A6_HC | . | . | . | . | I | . | . | F | . | . | . | . | . | . | . | Q | Y | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | . | . | . | . | . | . | . | . | . | . | . | . | . | . | . | . | . | . | . | . | . |
| 21-225_205F5_HC | . | . | . | . | . | . | . | . | . | . | . | . | . | . | . | E | . | . | V | . | . | . | . | . | . | . |
| 21-225_207G6_HC | . | . | . | . | . | . | . | . | . | . | . | . | . | . | . | . | . | . | . | . | E | . | . | . | . | . |
| 21-225_211A11_HC | . | . | . | . | . | L | . | F | . | . | . | . | . | . | . | R | . | . | . | . | . | . | . | . | . | . |
| 21-225_211A8_HC | . | . | . | . | . | . | . | . | . | . | . | . | . | . | . | E | H | . | . | . | . | . | . | . | . | . |
| 21-225_212D7_HC | . | . | . | . | . | . | . | . | . | . | . | . | . | . | . | . | . | . | . | . | . | . | . | . | . | . |
| 21-225_212E10_HC | . | . | . | . | . | . | . | . | . | . | . | . | . | . | . | . | Y | . | . | . | . | . | . | . | . | . |
| 21-225_212E6_HC | . | . | . | . | . | . | . | . | . | . | . | . | . | . | . | . | . | . | V | . | . | . | . | . | . | . |
| 21-225_212F10_HC | . | . | . | . | . | . | . | . | . | . | . | . | . | . | . | . | H | . | . | . | . | . | . | . | . | . |
| 21-225_212H9_HC | . | . | . | . | . | . | . | . | . | . | . | . | . | . | . | E | H | . | . | . | . | . | . | . | . | . |
| 21-225_213B8_HC | . | . | . | . | . | . | . | . | . | . | . | . | . | . | . | . | H | . | . | . | . | . | . | . | . | . |
| 21-225_213C4_HC | . | . | . | . | . | . | . | . | . | . | . | . | . | . | . | . | H | . | . | . | . | . | . | . | . | . |
| 21-225_213D2_HC | . | . | . | . | . | . | . | . | . | . | . | . | . | . | . | . | . | . | . | . | . | . | . | . | . | . |
| 21-225_214C3_HC | . | . | . | . | . | . | . | . | . | . | . | . | . | . | . | . | . | . | . | G | . | . | . | . | . | . |
| 21-225_215A12_HC | . | . | . | . | . | . | . | . | . | . | . | . | . | . | . | E | H | . | . | . | . | . | . | . | . | . |
| 21-225_215B5_HC | . | . | . | . | . | . | . | . | . | . | . | . | . | . | . | . | . | . | . | . | . | . | . | . | . | . |
| 21-225_215D6_HC | . | . | . | . | . | . | . | . | . | . | . | . | . | . | . | . | . | . | . | . | . | . | . | . | . | . |
| 21-225_217G5_HC | . | . | . | . | . | . | . | . | . | . | . | . | . | . | . | . | . | . | . | . | . | . | . | . | . | . |
| 21-225_219H1_HC | . | . | . | . | . | . | . | . | . | . | . | . | . | . | . | . | H | . | . | . | . | . | . | . | . | . |
| 21-225_223D11_HC | . | . | . | . | . | . | . | . | . | . | . | . | . | . | . | D | Y | . | . | . | . | . | . | . | . | . |
| 21-225_74D1_HC | . | . | . | . | . | . | . | . | . | . | . | . | . | . | . | . | Y | H | . | . | . | . | . | . | . | . |
| 21-225_87B7_HC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | | H_FR3 | | | | | | | | | | | |
| CONSENSUS | T | I | S | R | D | N | S | K | N | T | L | Y | L | Q | M | N | S | L | R | A | E | D | T | A | V | Y |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | . | . | . | . | . | C | . | . | . | . | . | . | . | . | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | . | . | . | . | . | . | . | . | . | . | . | . | . | . | . | . | . | . | . | . |
| 21-225_190G11_HC | . | . | . | . | . | . | . | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | M | . |
| 21-225_191A10_HC | . | . | . | . | . | . | . | . | . | . | . | . | . | . | . | . | . | . | . | . | . | . | . | . | M | . |
| 21-225_191E10_HC | . | . | . | . | . | . | . | . | . | . | . | . | . | . | . | . | . | . | . | . | . | . | . | . | M | . |
| 21-225_191E8_HC | . | . | . | . | . | . | . | . | . | . | . | . | . | . | L | . | . | . | . | . | . | . | . | . | . | . |
| 21-225_191G1_HC | . | . | . | . | . | . | . | . | . | . | . | . | . | . | . | . | . | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | . | . | . | . | . | . | . | . | . | . | . |
| 21-225_192C8_HC | M | . | . | . | . | . | . | . | . | . | . | . | . | . | . | . | . | . | . | . | . | . | . | . | . | . |
| 21-225_192D2_HC | . | . | . | . | . | . | . | . | . | M | . | . | . | . | . | . | . | . | . | . | E | . | . | . | . | . |
| 21-225_192D3_HC | . | . | . | . | . | . | . | . | . | . | . | . | . | . | . | . | . | . | . | . | . | . | . | . | . | . |
| 21-225_192G6_HC | . | . | . | . | . | . | . | . | . | . | . | . | . | . | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | M | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | . | . | . | . | . | . | . | . | . | . | . | . | . | . | . | . | . | . | . | . | . |
| 21-225_193C7_HC | . | . | . | . | . | . | . | . | . | . | . | . | . | . | . | . | . | . | . | . | . | . | . | . | M | . |
| 21-225_194H11_HC | . | . | . | . | . | . | . | . | . | . | . | S | . | . | . | . | . | . | . | . | . | . | . | . | . | . |
| 21-225_195A9_HC | . | . | . | . | . | . | . | . | . | . | . | . | . | . | . | . | . | . | . | . | . | . | . | . | . | . |
| 21-225_196C7_HC | . | . | . | . | . | . | . | . | . | . | . | . | . | . | . | . | . | . | . | . | . | . | . | . | . | . |
| 21-225_197B6_HC | . | . | . | . | . | . | . | . | . | M | . | . | . | . | . | . | . | . | . | . | . | . | . | . | M | . |
| 21-225_197D3_HC | . | . | . | . | . | . | . | . | . | . | . | . | . | . | . | . | . | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_199A6_HC | . | . | . | . | . | . | . | . | . | . | . | . | . | . | L | . | . | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | . | . | . | . | . | . | . | . | . | . | . | . | . | . | . | . | . | . | . | . | . |
| 21-225_205F5_HC | . | . | . | . | . | T | . | . | K | M | . | F | . | . | . | . | . | . | . | T | D | . | . | . | . | . |
| 21-225_207G6_HC | . | . | . | . | . | . | . | . | . | . | . | . | . | . | . | . | . | . | . | . | . | . | . | . | . | . |
| 21-225_211A11_HC | . | . | . | . | . | . | . | . | . | . | . | . | . | . | . | . | . | . | . | . | . | . | . | . | . | . |
| 21-225_211A8_HC | . | . | . | . | . | . | . | . | K | . | . | . | . | . | . | . | . | . | . | . | . | . | . | . | . | . |
| 21-225_212D7_HC | . | . | . | . | . | . | . | . | . | . | . | S | . | . | . | . | . | . | . | . | . | . | . | . | . | . |
| 21-225_212E10_HC | . | . | . | . | . | . | . | . | . | . | . | S | . | . | . | . | . | . | . | . | . | . | . | . | . | . |
| 21-225_212E6_HC | . | . | . | . | . | . | . | . | . | . | . | . | . | . | . | . | . | . | . | . | . | . | . | . | . | . |
| 21-225_212F10_HC | . | . | . | . | . | . | . | . | . | . | . | . | . | . | . | . | . | . | G | . | . | . | . | . | . | . |
| 21-225_212H9_HC | . | . | . | . | . | . | . | . | K | . | . | . | . | . | . | . | . | . | . | . | . | . | . | . | . | . |
| 21-225_213B8_HC | . | . | . | . | . | . | . | . | . | . | . | . | . | . | . | . | . | . | . | . | . | . | . | . | . | . |
| 21-225_213C4_HC | . | . | . | . | . | . | . | . | . | . | . | . | . | . | . | . | . | . | . | . | . | . | . | . | . | . |
| 21-225_213D2_HC | . | . | . | . | . | . | . | . | . | . | . | S | . | . | . | . | . | . | . | . | . | . | . | . | . | . |
| 21-225_214C3_HC | . | . | . | . | . | . | . | . | . | . | . | . | . | . | . | . | . | . | . | . | . | . | . | . | . | . |
| 21-225_215A12_HC | . | . | . | . | . | . | . | . | . | . | . | . | . | . | . | . | . | . | . | . | . | . | . | . | . | . |
| 21-225_215B5_HC | . | . | . | . | . | . | . | . | . | . | . | S | . | . | . | . | . | . | . | . | . | . | . | . | . | . |
| 21-225_215D6_HC | . | . | . | . | . | . | . | . | . | . | . | . | . | . | . | . | . | . | . | . | . | . | . | . | . | . |
| 21-225_217G5_HC | . | . | . | . | . | . | . | . | . | . | . | . | . | . | . | . | . | . | . | . | . | . | . | . | . | . |
| 21-225_219H1_HC | . | . | . | . | . | . | . | . | . | . | . | . | . | . | . | . | . | . | G | . | . | . | . | . | . | . |
| 21-225_223D11_HC | . | . | . | . | . | . | . | . | . | . | . | F | . | . | . | . | . | . | . | . | . | . | . | . | . | . |
| 21-225_74D1_HC | . | . | . | . | . | . | . | . | . | . | . | . | . | . | . | . | . | . | . | . | . | . | . | . | M | . |
| 21-225_87B7_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | Q | G | V | G | Y | - | - | - | - | - | - | - | - | - | - | - | - | - | - | - | - |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | . | . | . | . | . | . | . | . | . | . | . | . | . | . | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | . | . | . | . | . | . | . | . | . | . | . | . | . | . | . | . | . | . | . | . |
| 21-225_190G11_HC | . | . | . | . | . | . | . | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | . | . |
| 21-225_191A10_HC | . | . | . | . | . | . | . | . | . | . | . | . | . | . | . | . | . | . | . | . | . | . | . | . | . | . |
| 21-225_191E10_HC | . | . | . | . | . | . | . | . | . | . | . | . | . | . | . | . | . | . | . | . | . | . | . | . | . | . |
| 21-225_191E8_HC | . | . | . | . | A | H | . | . | Y | . | . | . | . | . | . | . | . | . | . | . | . | . | . | . | . | . |
| 21-225_191G1_HC | . | . | . | . | . | R | . | . | . | . | . | . | . | . | . | . | . | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | . | . | . | . | . | . | . | . | . | . | . |
| 21-225_192C8_HC | . | . | . | . | . | R | . | . | . | . | . | . | . | . | . | . | . | . | . | . | . | . | . | . | . | . |
| 21-225_192D2_HC | . | . | . | . | . | R | . | . | . | . | . | . | . | . | . | . | . | . | . | . | . | . | . | . | . | . |
| 21-225_192D3_HC | . | . | . | . | . | R | . | . | . | . | . | . | . | . | . | . | . | . | . | . | . | . | . | . | . | . |
| 21-225_192G6_HC | . | . | . | . | . | . | . | . | . | . | . | . | . | . | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | . | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | R | . | . | . | . | . | . | . | . | . | . | . | . | . | . | . | . | . | . | . | . |
| 21-225_193C7_HC | . | . | . | . | . | . | . | . | . | . | . | . | . | . | . | . | . | . | . | . | . | . | . | . | . | . |
| 21-225_194H11_HC | . | . | . | . | . | R | . | . | . | . | . | . | . | . | . | . | . | . | . | . | . | . | . | . | . | . |
| 21-225_195A9_HC | . | . | . | . | . | R | . | . | . | . | . | . | . | . | . | . | . | . | . | . | . | . | . | . | . | . |
| 21-225_196C7_HC | . | . | . | . | . | R | . | . | . | . | . | . | . | . | . | . | . | . | . | . | . | . | . | . | . | . |
| 21-225_197B6_HC | . | . | . | . | . | . | . | . | . | . | . | . | . | . | . | . | . | . | . | . | . | . | . | . | . | . |
| 21-225_197D3_HC | . | . | . | . | . | . | . | . | . | . | . | . | . | . | . | . | . | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | Q | G | V | G | Y | - | - | - | - | - | - | - | - | - | - | - | - | - | - | - | - |
| 21-225_199A6_HC | . | . | . | . | A | H | . | . | Y | . | . | . | . | . | . | . | . | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | R | . | . | . | . | . | . | . | . | . | . | . | . | . | . | . | . | . | . | . | . |
| 21-225_205F5_HC | . | . | . | . | . | M | . | I | . | . | . | . | . | . | . | . | . | . | . | . | . | . | . | . | . | . |
| 21-225_207G6_HC | . | . | . | . | . | A | . | I | . | . | . | . | . | . | . | . | . | . | . | . | . | . | . | . | . | . |
| 21-225_211A11_HC | . | . | . | . | . | R | . | . | . | . | . | . | . | . | . | . | . | . | . | . | . | . | . | . | . | . |
| 21-225_211A8_HC | . | . | . | . | . | H | . | . | . | . | . | . | . | . | . | . | . | . | . | . | . | . | . | . | . | . |
| 21-225_212D7_HC | . | . | . | . | . | Y | . | . | . | . | . | . | . | . | . | . | . | . | . | . | . | . | . | . | . | . |
| 21-225_212E10_HC | . | . | . | . | . | Y | . | . | . | . | . | . | . | . | . | . | . | . | . | . | . | . | . | . | . | . |
| 21-225_212E6_HC | . | . | . | . | . | Y | . | . | . | . | . | . | . | . | . | . | . | . | . | . | . | . | . | . | . | . |
| 21-225_212F10_HC | . | . | . | . | . | R | . | . | . | . | . | . | . | . | . | . | . | . | . | . | . | . | . | . | . | . |
| 21-225_212H9_HC | . | . | . | . | . | H | . | . | . | . | . | . | . | . | . | . | . | . | . | . | . | . | . | . | . | . |
| 21-225_213B8_HC | . | . | . | . | . | Y | . | . | . | . | . | . | . | . | . | . | . | . | . | . | . | . | . | . | . | . |
| 21-225_213C4_HC | . | . | . | . | . | Y | . | . | . | . | . | . | . | . | . | . | . | . | . | . | . | . | . | . | . | . |
| 21-225_213D2_HC | . | . | . | . | . | Y | . | . | . | . | . | . | . | . | . | . | . | . | . | . | . | . | . | . | . | . |
| 21-225_214C3_HC | . | . | . | . | . | R | . | . | . | . | . | . | . | . | . | . | . | . | . | . | . | . | . | . | . | . |
| 21-225_215A12_HC | . | . | . | . | . | R | . | . | . | . | . | . | . | . | . | . | . | . | . | . | . | . | . | . | . | . |
| 21-225_215B5_HC | . | . | . | . | . | Y | . | . | . | . | . | . | . | . | . | . | . | . | . | . | . | . | . | . | . | . |
| 21-225_215D6_HC | . | . | . | . | . | C | . | . | . | . | . | . | . | . | . | . | . | . | . | . | . | . | . | . | . | . |
| 21-225_217G5_HC | . | . | . | . | . | Y | . | . | . | . | . | . | . | . | . | . | . | . | . | . | . | . | . | . | . | . |
| 21-225_219H1_HC | . | . | . | . | . | R | . | . | . | . | . | . | . | . | . | . | . | . | . | . | . | . | . | . | . | . |
| 21-225_223D11_HC | . | . | . | . | . | R | . | I | . | . | . | . | . | . | . | . | . | . | . | . | . | . | . | . | . | . |
| 21-225_74D1_HC | . | . | . | . | . | E | . | F | . | E | F | D | . | . | . | . | . | . | . | . | . | . | . | . | . | . |
| 21-225_87B7_HC | . | . | . | . | . | F | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | Y | G | L | D | V | W | G | Q | G | T | T | V | T | V | S | S |
| 21-225_190C11_HC | . | . | . | D | . | . | . | . | . | . | . | . | . | . | . | . | . | . | . |
| 21-225_190E10_HC | . | . | . | D | . | . | . | . | . | . | . | . | . | . | . | . | . | . | . |
| 21-225_190E6_HC | . | . | . | D | . | . | . | . | . | . | . | . | . | . | . | . | . | . | . |
| 21-225_190F8_HC | . | . | . | D | . | . | . | . | . | . | . | . | . | . | . | . | . | . | . |
| 21-225_190G11_HC | . | . | . | D | . | . | . | . | . | . | . | . | . | . | . | . | . | . | . |
| 21-225_190G4_HC | . | . | . | D | . | . | . | . | . | . | . | . | . | S | . | . | . | . | . |
| 21-225_191A10_HC | . | . | . | D | . | . | . | . | . | . | . | . | . | S | . | . | . | . | . |
| 21-225_191E10_HC | . | . | . | D | . | . | . | . | . | . | . | . | . | S | . | . | . | . | . |
| 21-225_191E8_HC | . | . | . | . | A | M | . | . | . | . | . | . | . | . | . | . | . | . | . |
| 21-225_191G1_HC | . | . | . | . | . | . | . | . | . | . | . | . | . | . | . | . | . | . | . |
| 21-225_191G10_HC | . | . | . | D | . | . | . | . | . | . | . | . | . | . | . | . | . | . | . |
| 21-225_191G12_HC | . | . | . | D | . | . | . | . | . | . | . | . | . | . | . | . | . | . | . |
| 21-225_192C8_HC | . | . | . | . | . | M | . | . | . | . | . | . | . | . | . | . | . | . | . |
| 21-225_192D2_HC | . | . | . | . | . | M | . | . | . | . | . | . | . | . | . | . | . | . | . |
| 21-225_192D3_HC | . | . | . | . | . | . | . | . | . | . | . | . | . | . | . | . | . | . | . |
| 21-225_192G6_HC | . | . | . | D | . | . | . | . | . | . | . | . | . | . | . | . | . | . | . |
| 21-225_192G8_HC | . | . | . | D | . | . | . | . | . | . | . | . | . | . | . | . | . | . | . |
| 21-225_192H10_HC | . | . | . | D | . | . | . | . | . | . | . | . | . | S | . | . | . | . | . |
| 21-225_192H4_HC | . | . | . | . | . | M | . | . | . | . | . | . | . | . | . | . | . | . | . |
| 21-225_193A10_HC | . | . | . | . | . | . | . | . | . | . | . | . | . | . | . | . | . | . | . |
| 21-225_193C7_HC | . | . | . | D | . | . | . | . | . | . | . | . | . | S | . | . | . | . | . |
| 21-225_194H11_HC | . | . | . | . | . | . | . | . | . | . | . | . | . | . | . | . | . | . | . |
| 21-225_195A9_HC | . | . | . | . | . | . | . | . | . | . | . | . | . | . | . | . | . | . | . |
| 21-225_196C7_HC | . | . | . | . | . | . | . | . | . | . | . | . | . | . | . | . | . | . | . |
| 21-225_197B6_HC | . | . | . | D | . | . | . | . | . | . | . | . | . | S | . | . | . | . | . |
| 21-225_197D3_HC | . | . | . | . | . | M | . | . | . | . | . | . | . | . | . | . | . | . | . |
| 21-225_197F2_HC | . | . | . | D | . | . | . | . | . | . | . | . | . | . | . | . | . | . | . |
| 21-225_197G12_HC | . | . | . | D | . | . | . | . | . | . | . | . | . | . | . | . | . | . | . |
| 21-225_198B6_HC | . | . | . | . | . | M | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | Y | G | L | D | V | W | G | Q | G | T | T | V | T | V | S | S |
| 21-225_199A6_HC | . | . | . | . | A | M | . | . | . | . | . | . | . | . | . | . | . | . | . |
| 21-225_199C5_HC | . | . | . | . | . | M | . | . | . | . | . | . | . | . | . | . | . | . | . |
| 21-225_204G6_HC | . | . | . | D | . | M | . | . | . | . | . | . | . | . | . | . | . | . | . |
| 21-225_205F5_HC | . | . | . | . | . | M | . | . | . | . | . | . | . | . | . | . | . | . | . |
| 21-225_207G6_HC | . | . | . | . | . | I | . | . | . | . | . | . | . | . | . | . | . | . | . |
| 21-225_211A11_HC | . | . | . | . | . | T | . | . | . | . | . | . | . | . | . | . | . | . | . |
| 21-225_211A8_HC | . | . | . | . | . | M | . | . | . | . | . | . | . | . | . | . | . | . | . |
| 21-225_212D7_HC | . | . | . | . | . | T | . | . | . | . | . | . | . | . | . | . | . | . | . |
| 21-225_212E10_HC | . | . | . | . | . | T | . | . | . | . | . | . | . | . | . | . | . | . | . |
| 21-225_212E6_HC | . | . | . | . | . | T | . | . | . | . | . | . | . | . | . | . | . | . | . |
| 21-225_212F10_HC | . | . | . | N | . | M | . | . | . | . | . | . | . | . | . | . | . | . | . |
| 21-225_212H9_HC | . | . | . | . | . | M | . | . | . | . | . | . | . | . | . | . | . | . | . |
| 21-225_213B8_HC | . | . | . | . | . | T | . | . | . | . | . | . | . | . | . | . | . | . | . |
| 21-225_213C4_HC | . | . | . | D | . | M | . | . | . | . | . | . | . | . | . | . | . | . | . |
| 21-225_213D2_HC | . | . | . | . | . | T | . | . | . | . | . | . | . | . | . | . | . | . | . |
| 21-225_214C3_HC | . | . | . | D | . | M | . | . | . | . | . | . | . | . | . | . | . | . | . |
| 21-225_215A12_HC | . | . | . | . | . | M | . | . | . | . | . | . | . | . | . | . | . | . | . |
| 21-225_215B5_HC | . | . | . | . | . | T | . | . | . | . | . | . | . | . | . | . | . | . | . |
| 21-225_215D6_HC | . | . | . | . | . | T | . | . | . | . | . | . | . | . | . | . | . | . | . |
| 21-225_217G5_HC | . | . | . | . | . | . | . | . | . | . | . | . | . | . | . | . | . | . | . |
| 21-225_219H1_HC | . | . | . | N | . | M | . | . | . | . | . | . | . | . | . | . | . | . | . |
| 21-225_223D11_HC | . | . | . | N | D | M | . | . | . | . | . | . | . | . | . | . | . | . | . |
| 21-225_74D1_HC | Y | Y | N | . | . | M | . | . | . | . | . | . | . | . | . | . | . | . | . |
| 21-225_87B7_HC | . | . | . | . | . | M | . | . | . | . | . | . | . | . | . | . | . | . | . |

**FIGURE 57**

Table 55.    <u>Consensus 21- VH1|1-02/D1|1-1|RF1/JH4 (SEQ ID NO: 50272)</u>:

QVQLVQS-GAEVKKPGASVKVSCKASG-YTFT<u>G-----YYMH</u>WVRQAPGQGLEWMG<u>WINPN---
SGGTNYAQKFQG</u>RVTMTRDTSISTAYMELSRLRSDDTAVYYCAR<u>DGTS--------------------SFDY</u>WGQGTLVTVSS wherein:

G at position 33 can be substituted with D, S or A

Y at position 39 can be substituted with D

Y at position 40 can be substituted with H, N or F

M at position 41 can be substituted with L or I

H at position 42 can be substituted with Q

I at position 58 can be substituted with V

N at position 59 can be substituted with H, K or S

N at position 61 can be substituted with K

S at position 65 can be substituted with N, R or T

G at position 66 can be substituted with N or D

G at position 67 can be substituted with A

T at position 68 can be substituted with S

N at position 69 can be substituted with H, Q or I

Y at position 70 can be substituted with S or F

A at position 71 can be substituted with T

K at position 73 can be substituted with R, N, E or S

G at position 76 can be substituted with D

FIGURE 57

D at position 109 can be substituted with K, G, S or E

G at position 110 can be substituted with F, A, K or V

T at position 111 can be substituted with null (-)or P

S at position 112 can be substituted with G, null (-, or T

null (-) at position 113 can be substituted with V or S

null (-) at position 114 can be substituted with A

null (-) at position 115 can be substituted with T

null (-) at position 133 can be substituted with W

null (-) at position 134 can be substituted with G

S at position 135 can be substituted with null (-), V or Y

F at position 136 can be substituted with null (-), L or Y

D at position 137 can be substituted with G or K

Y at position 138 can be substituted with D or F

Xaa Xaa Xaa Xaa Xaa (SEQ ID NO: 50019)

Trp Xaa Xaa Pro Xaa Xaa Xaa Xaa Xaa Xaa Xaa Xaa Gln Xaa Phe Gln Xaa (SEQ ID NO: 50020)

Xaa Xaa Xaa Xaa Xaa Xaa Xaa Xaa Xaa Xaa Xaa Xaa Xaa (SEQ ID NO: 50021)

Gly Tyr Tyr Met His (SEQ ID NO: 50486)

Trp Ile Asn Pro Asn Ser Gly Gly Thr Asn Tyr Ala Gln Lys Phe Gln Gly (SEQ ID NO: 50487)

Asp Gly Thr Ser Ser Phe Asp Tyr (SEQ ID NO: 50488)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 |
|---|---|---|---|---|---|---|---|---|---|---|---|---|---|---|---|---|---|---|---|---|---|---|---|---|---|---|---|---|
| | | | | | | | | | | | | | | H_FR1 | | | | | | | | | | | | | | |
| CONSENSUS | Q | V | Q | L | V | Q | S | - | G | A | E | V | K | K | P | G | A | S | V | K | V | S | C | K | A | S | G | - |
| 21-225_10G6_HC | . | . | . | . | . | . | . | . | . | . | . | . | . | . | . | . | . | . | . | . | . | . | . | . | . | . | . | . |
| 21-225_12E9_HC | . | . | . | . | . | . | . | . | . | . | . | . | . | . | . | . | . | . | . | . | . | . | . | . | . | . | . | . |
| 21-225_169C10_HC | . | . | . | . | . | . | . | . | . | . | . | . | . | . | . | . | . | . | . | R | . | . | . | . | . | . | . | . |
| 21-225_170D6_HC | . | . | . | . | . | . | . | . | . | . | . | . | . | . | . | . | . | . | . | R | . | . | . | . | . | . | . | . |
| 21-225_171H12_HC | . | . | . | . | . | . | . | . | . | . | . | . | . | . | . | . | . | . | . | R | . | . | . | . | . | . | . | . |
| 21-225_177A5_HC | . | . | . | . | . | . | . | . | . | . | . | . | . | . | . | . | . | . | . | R | . | . | . | . | . | . | . | . |
| 21-225_181G2_HC | . | . | . | . | . | . | . | . | . | . | . | . | . | T | . | . | . | . | . | . | . | . | . | R | . | . | . | . |
| 21-225_184E7_HC | . | . | . | . | . | . | . | . | . | . | . | . | . | T | . | . | . | . | . | . | . | . | . | R | . | . | . | . |
| 21-225_185E10_HC | . | . | . | . | . | . | . | . | . | . | . | . | . | T | . | . | . | . | . | . | . | . | . | R | . | . | . | . |
| 21-225_188B9_HC | . | . | . | . | . | . | . | . | . | . | . | . | . | T | . | . | . | . | . | . | . | . | . | R | . | . | . | . |
| 21-225_188H5_HC | . | . | . | . | . | . | . | . | . | . | . | . | . | T | . | . | . | . | . | . | . | . | . | R | . | . | . | . |
| 21-225_18D4_HC | . | . | . | . | . | . | . | . | . | . | . | . | . | . | . | . | . | . | . | . | . | . | . | . | . | . | . | . |
| 21-225_213H7_HC | . | . | . | . | . | . | . | . | . | . | . | . | . | . | . | . | . | . | . | . | . | . | . | . | . | . | . | . |
| 21-225_221A6_HC | . | . | . | . | . | . | . | . | . | . | . | . | . | . | . | . | . | . | . | . | . | . | . | . | T | . | . | . |
| 21-225_221F12_HC | . | . | . | . | . | . | . | . | . | . | . | . | . | . | . | . | . | . | . | . | . | . | . | . | T | . | . | . |
| 21-225_222C12_HC | . | . | . | . | . | . | . | . | . | . | . | . | . | . | . | . | . | . | . | . | . | . | . | . | T | . | . | . |
| 21-225_222E1_HC | . | . | . | . | . | . | . | . | . | . | . | . | . | . | . | . | . | . | . | . | . | . | . | . | T | . | . | . |
| 21-225_222F7_HC | . | . | . | . | . | . | . | . | . | . | . | . | . | . | . | . | . | . | . | . | . | . | . | . | T | . | . | . |
| 21-225_23B11_HC | . | . | . | . | . | . | . | . | . | . | . | . | . | . | . | . | . | . | . | . | . | . | . | . | . | . | . | . |
| 21-225_30A11_HC | . | . | . | . | . | . | . | . | . | . | . | . | . | . | . | . | . | . | . | . | . | . | . | . | . | . | . | . |
| 21-225_31C4_HC | . | . | . | . | . | . | . | . | . | T | . | . | . | . | . | . | . | . | . | . | . | . | . | . | . | . | . | . |
| 21-225_33G1_HC | . | . | . | . | A | . | . | . | . | . | . | . | . | . | . | . | . | . | . | . | . | . | . | . | . | . | . | . |
| 21-225_33H7_HC | . | . | . | . | . | . | . | . | . | . | D | . | . | . | . | . | . | . | . | . | . | . | . | . | . | . | . | . |
| 21-225_34F3_HC | . | . | . | . | . | . | . | . | . | . | . | . | . | . | . | . | . | . | . | . | . | . | . | . | . | . | . | . |
| 21-225_34G7_HC | . | . | . | . | . | . | . | . | . | . | . | . | . | . | . | . | . | . | . | . | . | . | . | R | . | . | . | . |
| 21-225_35C6_HC | . | . | . | . | . | . | . | . | . | T | . | . | . | . | . | . | . | . | . | . | . | . | . | . | . | . | . | . |
| 21-225_35G6_HC | . | . | . | . | . | . | . | . | . | . | D | . | . | . | . | . | . | . | . | . | . | . | . | . | . | . | . | . |
| 21-225_35H12_HC | . | . | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 |
|---|---|---|---|---|---|---|---|---|---|---|---|---|---|---|---|---|---|---|---|---|---|---|---|---|---|---|---|---|
|  |  |  |  |  |  |  |  |  |  |  |  |  |  |  | H_FR1 |  |  |  |  |  |  |  |  |  |  |  |  |  |
| CONSENSUS | Q | V | Q | L | V | Q | S | - | G | A | E | V | K | K | P | G | A | S | V | K | V | S | C | K | A | S | G | - |
| 21-225_36H5_HC | . | . | . | . | . | . | . | . | . | . | . | . | . | . | . | . | . | . | M | . | . | . | . | . | . | . | . | . |
| 21-225_49F10_HC | . | . | . | . | . | . | . | . | . | . | . | . | . | . | . | . | . | . | . | . | . | . | . | . | . | . | . | . |
| 21-225_4H6_HC | . | . | . | . | . | . | . | . | . | . | . | . | . | . | . | . | . | . | . | . | . | . | . | . | . | . | . | . |
| 21-225_4H6.004_HC | . | . | . | . | . | . | . | . | . | . | . | . | . | . | . | . | . | . | . | . | . | . | . | . | . | . | . | . |
| 21-225_4H6.005_HC | . | . | . | . | . | . | . | . | . | . | . | . | . | . | . | . | . | . | . | . | . | . | . | . | . | . | . | . |
| 21-225_4H6.006_HC | . | . | . | . | . | . | . | . | . | . | . | . | . | . | . | . | . | . | . | . | . | . | . | . | . | . | . | . |
| 21-225_4H6.007_HC | . | . | . | . | . | . | . | . | . | . | . | . | . | . | . | . | . | . | . | . | . | . | . | . | . | . | . | . |
| 21-225_4H6.008_HC | . | . | . | . | . | . | . | . | . | . | . | . | . | . | . | . | . | . | . | . | . | . | . | . | . | . | . | . |
| 21-225_4H6.009_HC | . | . | . | . | . | . | . | . | . | . | . | . | . | . | . | . | . | . | . | . | . | . | . | . | . | . | . | . |
| 21-225_4H6.010_HC | . | . | . | . | . | . | . | . | . | . | . | . | . | . | . | . | . | . | . | . | . | . | . | . | . | . | . | . |
| 21-225_4H6.011_HC | . | . | . | . | . | . | . | . | . | . | . | . | . | . | . | . | . | . | . | . | . | . | . | . | . | . | . | . |
| 21-225_4H6.014_HC | . | . | . | . | . | . | . | . | . | . | . | . | . | . | . | . | . | . | . | . | . | . | . | . | . | . | . | . |
| 21-225_50D4_HC | . | . | . | . | . | . | . | . | . | . | . | . | . | . | . | . | . | . | . | . | . | . | . | . | . | . | . | . |
| 21-225_51E9_HC | . | . | . | . | . | . | . | . | . | . | . | . | . | . | . | . | . | . | . | . | . | . | . | . | . | . | R | . |
| 21-225_52B1_HC | . | . | . | . | . | . | . | . | . | . | . | . | . | . | . | . | . | . | . | . | . | . | . | . | . | . | . | . |
| 21-225_52G11_HC | . | . | . | . | . | . | . | . | . | . | . | . | . | . | . | . | . | . | . | . | . | . | . | . | . | . | . | . |
| 21-225_52H10_HC | . | . | . | . | . | . | . | . | . | . | . | . | . | . | . | . | . | . | . | . | . | . | . | . | . | . | . | . |
| 21-225_53F2_HC | . | . | . | . | . | . | . | . | . | . | . | . | . | . | . | . | . | . | . | . | . | . | . | . | . | . | . | . |
| 21-225_53F7_HC | . | . | . | . | . | . | . | . | . | . | . | . | . | . | . | . | . | . | . | . | . | . | . | . | . | . | . | . |
| 21-225_56E5_HC | . | . | . | . | . | . | . | . | . | . | . | . | . | . | . | . | . | . | . | . | . | . | . | Q | . | . | . | . |
| 21-225_7C3_LC1_HC | . | . | . | . | . | . | . | . | . | . | . | . | . | . | . | . | . | . | . | . | . | . | . | . | . | . | . | . |
| 21-225_7C3_LC2_HC | . | . | . | . | . | . | . | . | . | . | . | . | . | . | . | . | . | . | . | . | . | . | . | . | . | . | . | . |
| 21-225_9F12_LC1_HC | . | . | . | . | . | . | . | . | . | . | . | . | . | . | . | . | . | . | . | . | . | . | . | . | . | . | . | . |
| 21-225_9F12_LC2_HC | . | . | . | . | . | . | . | . | . | . | . | . | . | . | . | . | . | . | . | . | . | . | . | . | . | . | . | . |

# FIGURE 57

| Reference # | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 |
|---|---|---|---|---|---|---|---|---|---|---|---|---|---|---|---|---|---|---|---|---|---|---|---|---|---|---|---|---|
| | | | | | | | | H_CDR1 | | | | | | | | | | | | | | H_FR2 | | | | | | |
| CONSENSUS | Y | T | F | T | G | - | - | - | - | - | Y | Y | M | H | W | V | R | Q | A | P | G | Q | G | L | E | W | M | G |
| 21-225_10G6_HC | . | . | . | . | A | . | . | . | . | . | . | H | . | . | . | . | . | . | . | . | . | . | . | . | . | . | . | . |
| 21-225_12E9_HC | . | . | . | . | D | . | . | . | . | . | . | . | L | . | . | . | . | . | . | . | . | . | . | . | . | . | . | . |
| 21-225_169C10_HC | . | . | . | . | . | . | . | . | . | . | . | F | . | . | . | . | . | . | . | . | . | . | . | . | . | . | . | . |
| 21-225_170D6_HC | . | . | . | . | . | . | . | . | . | . | . | F | I | . | . | . | . | . | . | . | . | . | . | . | . | . | . | . |
| 21-225_171H12_HC | . | . | . | . | . | . | . | . | . | . | . | F | . | . | . | . | . | . | . | . | . | . | . | . | . | . | . | . |
| 21-225_177A5_HC | . | . | . | . | . | . | . | . | . | . | . | F | . | . | . | . | . | . | . | . | . | . | . | . | . | . | . | . |
| 21-225_181G2_HC | . | . | . | . | S | . | . | . | . | . | . | N | . | . | . | . | . | . | V | . | . | . | . | . | . | . | . | . |
| 21-225_184E7_HC | . | . | . | . | S | . | . | . | . | . | . | N | . | . | . | . | . | . | V | . | . | . | . | . | . | . | . | . |
| 21-225_185E10_HC | . | . | . | . | S | . | . | . | . | . | . | N | . | . | . | . | . | . | V | . | . | . | . | . | . | . | . | . |
| 21-225_188B9_HC | . | . | . | . | S | . | . | . | . | . | . | N | . | . | . | . | . | . | V | . | . | . | . | . | . | . | . | . |
| 21-225_188H5_HC | . | . | . | . | S | . | . | . | . | . | . | N | . | . | . | . | . | . | V | . | . | . | . | . | . | . | . | . |
| 21-225_18D4_HC | . | . | . | . | . | . | . | . | . | . | . | . | L | . | . | . | . | . | . | . | . | . | . | . | . | . | L | . |
| 21-225_213H7_HC | . | . | . | . | . | . | . | . | . | . | . | H | . | . | . | . | . | . | . | . | . | . | . | . | . | . | . | . |
| 21-225_221A6_HC | . | . | . | . | . | . | . | . | . | . | . | . | . | . | . | . | . | . | . | . | . | . | . | . | . | . | . | . |
| 21-225_221F12_HC | . | . | . | . | . | . | . | . | . | . | . | . | . | . | . | . | . | . | . | . | . | . | . | . | . | . | . | . |
| 21-225_222C12_HC | . | . | . | . | . | . | . | . | . | . | . | . | . | . | . | . | . | . | . | . | . | . | . | . | . | . | . | . |
| 21-225_222E1_HC | . | . | . | . | . | . | . | . | . | . | . | . | . | . | . | . | . | . | . | . | . | . | . | . | . | . | . | . |
| 21-225_222F7_HC | . | . | . | . | . | . | . | . | . | . | . | . | . | . | . | . | . | . | . | . | . | . | . | . | . | . | . | . |
| 21-225_23B11_HC | . | . | . | . | . | . | . | . | . | . | . | . | L | . | . | . | . | . | . | . | . | . | . | . | . | . | . | . |
| 21-225_30A11_HC | . | . | . | . | D | . | . | . | . | . | . | H | . | . | . | . | . | . | . | . | . | . | . | . | . | . | . | . |
| 21-225_31C4_HC | . | . | . | . | . | . | . | . | . | . | D | . | . | . | . | . | . | . | . | . | . | . | . | . | . | . | . | . |
| 21-225_33G1_HC | . | . | . | . | . | . | . | . | . | . | . | . | . | . | . | . | . | . | . | . | . | . | . | . | . | . | . | . |
| 21-225_33H7_HC | . | . | . | . | D | . | . | . | . | . | . | H | I | . | . | . | . | . | . | . | . | . | . | . | . | . | . | . |
| 21-225_34F3_HC | . | . | . | . | . | . | . | . | . | . | . | H | . | . | . | . | . | . | . | . | . | . | . | . | D | . | . | . |
| 21-225_34G7_HC | . | . | . | . | D | . | . | . | . | . | . | H | I | . | . | . | . | . | . | . | . | . | . | . | . | . | . | . |
| 21-225_35C6_HC | . | . | . | . | . | . | . | . | . | . | D | . | . | . | . | . | . | . | . | . | . | . | . | . | . | . | . | . |
| 21-225_35G6_HC | . | . | . | . | D | . | . | . | . | . | . | H | I | . | . | . | . | . | . | . | . | . | . | . | . | . | . | . |
| 21-225_35H12_HC | . | . | . | . | D | . | . | . | . | . | . | . | I | . | . | . | . | . | . | . | . | . | . | . | . | . | . | . |

FIGURE 57

| Reference # | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 |
|---|---|---|---|---|---|---|---|---|---|---|---|---|---|---|---|---|---|---|---|---|---|---|---|---|---|---|---|---|
| | | | | | | | H_CDR1 | | | | | | | | | | | | | | H_FR2 | | | | | | | |
| CONSENSUS | Y | T | F | T | G | - | - | - | - | - | Y | Y | M | H | W | V | R | Q | A | P | G | Q | G | L | E | W | M | G |
| 21-225_36H5_HC | . | . | . | . | D | . | . | . | . | . | . | H | . | . | . | . | . | . | . | . | . | . | . | . | . | . | . | . |
| 21-225_49F10_HC | . | . | . | . | . | . | . | . | . | . | . | H | . | . | . | . | . | . | . | . | . | . | . | . | . | . | . | . |
| 21-225_4H6_HC | . | . | . | . | D | . | . | . | . | . | . | . | L | . | . | . | . | . | . | . | . | . | . | . | . | . | . | . |
| 21-225_4H6.004_HC | . | . | . | . | D | . | . | . | . | . | . | . | L | . | . | . | . | . | . | . | . | . | . | . | . | . | . | . |
| 21-225_4H6.005_HC | . | . | . | . | D | . | . | . | . | . | . | . | L | . | . | . | . | . | . | . | . | . | . | . | . | . | . | . |
| 21-225_4H6.006_HC | . | . | . | . | D | . | . | . | . | . | . | . | L | . | . | . | . | . | . | . | . | . | . | . | . | . | . | . |
| 21-225_4H6.007_HC | . | . | . | . | D | . | . | . | . | . | . | . | L | . | . | . | . | . | . | . | . | . | . | . | . | . | . | . |
| 21-225_4H6.008_HC | . | . | . | . | D | . | . | . | . | . | . | . | L | . | . | . | . | . | . | . | . | . | . | . | . | . | . | . |
| 21-225_4H6.009_HC | . | . | . | . | D | . | . | . | . | . | . | . | L | . | . | . | . | . | . | . | . | . | . | . | . | . | . | . |
| 21-225_4H6.010_HC | . | . | . | . | D | . | . | . | . | . | . | . | L | . | . | . | . | . | . | . | . | . | . | . | . | . | . | . |
| 21-225_4H6.011_HC | . | . | . | . | D | . | . | . | . | . | . | . | L | . | . | . | . | . | . | . | . | . | . | . | . | . | . | . |
| 21-225_4H6.014_HC | . | . | . | . | D | . | . | . | . | . | . | . | L | . | . | . | . | . | . | . | . | . | . | . | . | . | . | . |
| 21-225_50D4_HC | . | . | . | . | . | . | . | . | . | . | . | H | . | . | . | . | . | . | . | . | . | . | . | . | . | . | . | . |
| 21-225_51E9_HC | . | . | . | . | . | . | . | . | . | . | . | H | I | . | . | . | . | . | . | . | . | . | . | . | . | . | . | . |
| 21-225_52B1_HC | . | . | . | . | . | . | . | . | . | . | . | H | . | . | Q | . | . | . | . | . | . | . | . | . | . | . | . | . |
| 21-225_52G11_HC | . | . | . | . | . | . | . | . | . | . | . | . | L | . | . | . | . | . | . | . | . | . | . | . | . | . | . | . |
| 21-225_52H10_HC | . | . | . | . | . | . | . | . | . | . | . | H | . | . | Q | . | . | . | . | . | . | . | . | . | . | . | . | . |
| 21-225_53F2_HC | . | . | . | . | . | . | . | . | . | . | . | . | L | . | . | . | . | . | . | . | . | . | . | . | . | . | . | . |
| 21-225_53F7_HC | . | . | . | . | . | . | . | . | . | . | . | H | . | . | . | . | . | . | . | . | . | . | . | . | . | . | . | . |
| 21-225_56E5_HC | . | . | . | . | . | . | . | . | . | . | . | H | . | . | . | . | . | . | . | . | . | . | . | . | . | . | . | . |
| 21-225_7C3_LC1_HC | . | . | . | N | D | . | . | . | . | . | . | . | L | . | . | . | . | . | . | . | . | . | . | . | . | . | . | . |
| 21-225_7C3_LC2_HC | . | . | . | N | D | . | . | . | . | . | . | . | L | . | . | . | . | . | . | . | . | . | . | . | . | . | . | . |
| 21-225_9F12_LC1_HC | . | . | . | A | D | . | . | . | . | . | . | . | L | . | . | . | . | . | . | . | . | . | . | . | . | . | . | . |
| 21-225_9F12_LC2_HC | . | . | . | A | D | . | . | . | . | . | . | . | L | . | . | . | . | . | . | . | . | . | . | . | . | . | . | . |

FIGURE 57

| Reference # | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 | 82 | 83 | 84 |
|---|---|---|---|---|---|---|---|---|---|---|---|---|---|---|---|---|---|---|---|---|---|---|---|---|---|---|---|---|
| | | | | | | | | | | H_CDR2 | | | | | | | | | | | | | | | | | | |
| CONSENSUS | W | I | N | P | N | - | - | - | S | G | G | T | N | Y | A | Q | K | F | Q | G | R | V | T | M | T | R | D | T |
| 21-225_10G6_HC | . | . | . | . | . | . | . | . | N | N | . | . | . | . | . | . | . | . | . | . | . | . | . | . | . | . | . | . |
| 21-225_12E9_HC | . | V | H | . | . | . | . | . | . | . | . | . | . | F | . | . | . | . | . | D | . | . | . | . | . | . | . | . |
| 21-225_169C10_HC | . | . | K | . | K | . | . | . | . | . | . | . | . | S | . | . | R | . | . | . | . | . | . | . | . | . | . | . |
| 21-225_170D6_HC | . | . | K | . | . | . | . | . | . | . | . | . | . | S | . | . | R | . | . | . | . | . | . | . | . | . | . | . |
| 21-225_171H12_HC | . | . | K | . | K | . | . | . | . | . | . | . | . | S | . | . | R | . | . | . | . | . | . | . | . | . | . | . |
| 21-225_177A5_HC | . | . | K | . | K | . | . | . | . | . | . | . | . | S | . | . | R | . | . | . | . | . | . | . | . | . | . | . |
| 21-225_181G2_HC | . | . | . | . | . | . | . | . | N | . | . | S | . | . | T | . | . | . | . | . | . | . | . | . | . | . | . | . |
| 21-225_184E7_HC | . | . | . | . | . | . | . | . | N | . | . | S | . | . | T | . | . | . | . | . | . | . | . | . | . | . | . | . |
| 21-225_185E10_HC | . | . | . | . | . | . | . | . | N | . | . | S | . | . | T | . | . | . | . | . | . | . | . | . | . | . | . | . |
| 21-225_188B9_HC | . | . | . | . | . | . | . | . | . | . | . | S | . | . | T | . | . | . | . | . | . | . | . | . | . | . | . | . |
| 21-225_188H5_HC | . | . | . | . | . | . | . | . | . | . | . | S | . | . | T | . | . | . | . | . | . | . | I | . | . | . | . | . |
| 21-225_18D4_HC | . | . | . | . | . | . | . | . | . | N | . | . | I | S | . | . | . | . | . | . | . | . | . | . | . | . | . | . |
| 21-225_213H7_HC | . | . | . | . | K | . | . | . | . | D | . | . | . | . | . | . | . | . | . | . | . | . | . | . | . | . | . | . |
| 21-225_221A6_HC | . | . | H | . | . | . | . | . | . | . | . | . | . | . | . | . | . | . | . | . | . | . | . | L | . | . | . | . |
| 21-225_221F12_HC | . | . | H | . | . | . | . | . | . | . | . | . | . | . | . | . | . | . | . | . | . | . | . | L | . | . | . | . |
| 21-225_222C12_HC | . | . | H | . | . | . | . | . | . | . | . | . | . | . | . | . | . | . | . | . | . | . | . | L | . | . | . | . |
| 21-225_222E1_HC | . | . | H | . | . | . | . | . | . | . | . | . | . | . | . | . | . | . | . | . | . | . | . | L | . | . | . | . |
| 21-225_222F7_HC | . | . | H | . | . | . | . | . | . | . | . | . | . | . | . | . | . | . | . | . | . | . | . | L | . | . | . | . |
| 21-225_23B11_HC | . | . | . | . | . | . | . | . | N | N | . | . | . | . | . | . | . | . | . | . | . | . | . | . | . | . | . | . |
| 21-225_30A11_HC | . | . | . | . | . | . | . | . | R | . | . | . | . | . | . | . | . | . | . | D | . | . | . | . | . | . | . | . |
| 21-225_31C4_HC | . | . | S | . | K | . | . | . | N | . | . | . | . | . | . | . | . | . | . | . | . | . | . | . | . | . | . | . |
| 21-225_33G1_HC | . | . | S | . | . | . | . | . | N | . | . | . | . | . | . | . | . | . | . | . | . | . | . | . | . | . | . | . |
| 21-225_33H7_HC | . | . | . | . | . | . | . | . | N | . | . | . | H | . | . | . | E | . | . | . | . | . | . | . | . | . | . | . |
| 21-225_34F3_HC | . | . | . | . | . | . | . | . | . | . | . | . | . | . | . | . | . | . | . | . | . | . | . | . | . | . | . | . |
| 21-225_34G7_HC | . | . | . | . | . | . | . | . | N | . | . | . | H | . | . | . | . | . | . | . | . | . | . | . | . | . | . | . |
| 21-225_35C6_HC | . | . | S | . | K | . | . | . | N | . | . | . | . | . | . | . | . | . | . | . | . | . | . | . | . | . | . | . |
| 21-225_35G6_HC | . | . | . | . | . | . | . | . | N | . | . | . | H | . | . | . | E | . | . | . | . | . | . | . | . | . | . | . |
| 21-225_35H12_HC | . | . | . | . | . | . | . | . | R | . | . | . | . | . | . | . | . | . | . | . | . | . | . | . | . | . | . | . |

FIGURE 57

| Reference # | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 | 82 | 83 | 84 |
|---|---|---|---|---|---|---|---|---|---|---|---|---|---|---|---|---|---|---|---|---|---|---|---|---|---|---|---|---|
| | | | | | | | | | | | H_CDR2 | | | | | | | | | | | | | | | | | |
| CONSENSUS | W | I | N | P | N | - | - | - | S | G | G | T | N | Y | A | Q | K | F | Q | G | R | V | T | M | T | R | D | T |
| 21-225_36H5_HC | . | . | . | . | . | . | . | . | R | . | . | . | . | . | . | . | . | . | . | D | . | . | . | . | . | . | . | . |
| 21-225_49F10_HC | . | . | . | . | . | . | . | . | N | N | A | . | . | . | . | . | N | . | . | . | . | . | . | . | L | . | . | . |
| 21-225_4H6_HC | . | . | H | . | . | . | . | . | . | . | . | . | . | . | . | . | . | . | . | . | . | . | . | . | . | . | . | . |
| 21-225_4H6.004_HC | . | . | H | . | . | . | . | . | . | . | . | . | . | . | . | . | . | . | . | . | . | . | . | . | . | . | . | . |
| 21-225_4H6.005_HC | . | . | H | . | . | . | . | . | . | . | . | . | . | . | . | . | . | . | . | . | . | . | . | . | . | . | . | . |
| 21-225_4H6.006_HC | . | . | H | . | . | . | . | . | . | . | . | . | . | . | . | . | . | . | . | . | . | . | . | . | . | . | . | . |
| 21-225_4H6.007_HC | . | . | H | . | . | . | . | . | . | . | . | . | . | . | . | . | . | . | . | . | . | . | . | . | . | . | . | . |
| 21-225_4H6.008_HC | . | . | H | . | . | . | . | . | . | . | . | . | . | . | . | . | . | . | . | . | . | . | . | . | . | . | . | . |
| 21-225_4H6.009_HC | . | . | H | . | . | . | . | . | . | . | . | . | . | . | . | . | . | . | . | . | . | . | . | . | . | . | . | . |
| 21-225_4H6.010_HC | . | . | H | . | . | . | . | . | . | . | . | . | . | . | . | . | . | . | . | . | . | . | . | . | . | . | . | . |
| 21-225_4H6.011_HC | . | . | H | . | . | . | . | . | . | . | . | . | . | . | . | . | . | . | . | . | . | . | . | . | . | . | . | . |
| 21-225_4H6.014_HC | . | . | H | . | . | . | . | . | . | . | . | . | . | . | . | . | . | . | . | . | . | . | . | . | . | . | . | . |
| 21-225_50D4_HC | . | . | . | . | . | . | . | . | N | N | A | . | . | . | . | . | S | . | . | . | . | . | . | . | L | . | . | . |
| 21-225_51E9_HC | . | . | . | . | . | . | . | . | T | N | . | . | Q | . | . | . | . | . | . | . | . | . | . | . | . | . | . | . |
| 21-225_52B1_HC | . | . | . | . | . | . | . | . | . | . | A | . | . | . | . | . | N | . | . | . | . | . | . | . | . | . | . | . |
| 21-225_52G11_HC | . | . | . | . | . | . | . | . | R | N | . | . | . | . | . | . | . | . | . | . | . | . | . | . | . | . | . | . |
| 21-225_52H10_HC | . | . | . | . | . | . | . | . | N | . | . | . | Q | . | . | . | . | . | . | . | . | . | . | . | . | . | . | . |
| 21-225_53F2_HC | . | . | . | . | . | . | . | . | R | N | . | . | . | . | . | . | N | . | . | . | . | . | . | . | . | . | . | . |
| 21-225_53F7_HC | . | . | . | . | . | . | . | . | N | N | . | . | . | . | . | . | . | . | . | . | . | . | . | . | . | . | . | . |
| 21-225_56E5_HC | . | . | . | . | . | . | . | . | N | N | A | . | . | . | . | . | . | . | . | . | . | . | . | . | . | . | . | . |
| 21-225_7C3_LC1_HC | . | . | H | . | . | . | . | . | . | . | . | . | . | . | . | . | . | . | . | . | . | . | . | . | . | . | . | . |
| 21-225_7C3_LC2_HC | . | . | H | . | . | . | . | . | . | . | . | . | . | . | . | . | . | . | . | . | . | . | . | . | . | . | . | . |
| 21-225_9F12_LC1_HC | . | . | H | . | . | . | . | . | . | . | . | . | F | . | . | . | . | . | . | . | . | . | . | . | . | . | . | . |
| 21-225_9F12_LC2_HC | . | . | H | . | . | . | . | . | . | . | . | . | F | . | . | . | . | . | . | . | . | . | . | . | . | . | . | . |

FIGURE 57

Region marker: H_FR3 (spanning approximately positions 88–92)

| Reference # | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|---|---|---|---|---|---|---|---|---|---|---|---|
| CONSENSUS | S | I | S | T | A | Y | M | E | L | S | R | L | R | S | D | D | T | A | V | Y | Y | C | A | R | D | G | T | S |
| 21-225_10G6_HC | . | . | . | . | . | . | . | . | . | . | . | . | . | . | . | . | . | . | . | . | . | . | G | . | . | V | . | . |
| 21-225_12E9_HC | . | . | . | . | . | . | . | . | . | . | S | . | . | . | . | . | . | I | . | S | . | . | . | . | . | . | . | . |
| 21-225_169C10_HC | . | . | N | . | . | . | . | . | . | N | . | . | . | . | . | . | . | . | . | . | . | . | . | . | G | . | . | T |
| 21-225_170D6_HC | . | . | N | . | . | . | . | . | . | N | . | . | . | . | . | . | . | . | . | . | . | . | . | . | G | . | . | T |
| 21-225_171H12_HC | . | . | N | . | . | . | . | . | . | N | . | . | . | . | . | . | . | . | . | . | . | . | . | . | G | . | . | T |
| 21-225_177A5_HC | . | . | N | . | . | . | . | . | . | N | W | . | . | . | . | . | . | . | . | . | . | . | . | . | G | . | . | T |
| 21-225_181G2_HC | . | . | . | . | . | . | . | . | . | . | . | . | . | . | . | . | . | . | . | . | . | . | . | . | K | F | - | - |
| 21-225_184E7_HC | . | . | . | . | . | . | . | . | . | . | . | . | . | . | . | . | . | . | . | . | . | . | . | . | K | F | - | - |
| 21-225_185E10_HC | . | . | . | . | . | . | . | . | . | . | . | . | . | . | . | . | . | . | . | . | . | . | . | . | K | F | - | - |
| 21-225_188B9_HC | . | . | . | . | . | . | . | . | . | . | . | . | . | . | . | . | . | . | . | . | . | . | . | . | K | F | - | - |
| 21-225_188H5_HC | . | . | . | . | . | . | . | . | . | . | . | . | . | . | . | . | . | . | . | . | . | . | . | . | K | F | - | - |
| 21-225_18D4_HC | . | . | . | . | . | . | . | . | . | . | . | . | I | . | . | . | . | . | . | . | . | . | . | . | . | . | . | . |
| 21-225_213H7_HC | . | . | . | . | . | . | . | . | . | . | . | . | . | . | . | . | . | . | . | . | . | . | . | . | E | K | P | G |
| 21-225_221A6_HC | . | . | . | . | . | . | . | D | . | . | . | . | . | . | . | . | . | . | . | . | . | . | . | . | . | . | . | . |
| 21-225_221F12_HC | . | . | . | . | . | . | . | D | . | . | . | . | . | . | . | . | . | . | . | . | . | . | . | . | . | . | . | . |
| 21-225_222C12_HC | . | . | . | . | . | . | . | D | . | . | . | . | . | . | . | . | . | . | . | . | . | . | . | . | . | . | . | . |
| 21-225_222E1_HC | . | . | . | . | . | . | . | D | . | . | . | . | . | . | . | . | . | . | . | . | . | . | . | . | . | . | . | . |
| 21-225_222F7_HC | . | . | . | . | . | . | . | D | . | . | . | . | . | . | . | . | . | . | . | . | . | . | . | . | . | . | . | . |
| 21-225_23B11_HC | . | . | . | . | . | . | . | . | . | . | . | . | . | . | . | . | . | . | . | . | . | . | . | . | . | . | . | . |
| 21-225_30A11_HC | . | . | . | . | . | . | . | . | . | . | . | . | . | . | . | . | . | . | . | F | . | . | . | . | . | . | . | G |
| 21-225_31C4_HC | . | . | . | V | . | . | . | . | . | N | . | . | . | . | . | . | . | . | . | . | . | . | . | . | . | . | . | G |
| 21-225_33G1_HC | . | . | . | . | . | . | . | . | . | . | . | . | . | . | . | . | . | . | . | . | . | . | . | . | . | . | . | G |
| 21-225_33H7_HC | . | . | . | . | . | . | . | . | . | . | S | . | . | . | . | . | . | . | . | H | . | . | . | . | . | . | . | G |
| 21-225_34F3_HC | . | . | . | . | . | . | . | . | . | . | . | . | . | . | . | . | . | . | . | . | . | . | . | . | . | . | . | . |
| 21-225_34G7_HC | . | . | . | . | . | . | . | . | . | . | . | . | . | . | . | . | . | . | . | . | . | . | . | . | . | . | . | G |
| 21-225_35C6_HC | . | . | . | . | . | . | . | . | . | N | . | . | . | . | . | . | . | . | . | . | . | . | . | . | . | . | . | G |
| 21-225_35G6_HC | . | . | . | . | . | . | . | . | . | . | S | . | . | . | . | . | . | . | . | . | . | . | . | . | . | . | . | G |
| 21-225_35H12_HC | . | . | . | . | . | . | . | . | . | . | . | . | . | . | . | . | . | . | . | . | . | . | . | . | . | . | . | G |

FIGURE 57

| Reference # | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|---|---|---|---|---|---|---|---|---|---|---|---|
| | | | | | | | | H_FR3 | | | | | | | | | | | | | | | | | | | | |
| CONSENSUS | S | I | S | T | A | Y | M | E | L | S | R | L | R | S | D | D | T | A | V | Y | Y | C | A | R | D | G | T | S |
| 21-225_36H5_HC | . | . | . | . | . | . | . | . | . | . | . | . | . | . | . | . | . | . | . | . | F | . | . | . | . | . | . | G |
| 21-225_49F10_HC | . | . | . | . | . | . | . | . | . | . | . | . | . | . | . | . | . | . | . | . | . | . | . | . | . | . | . | . |
| 21-225_4H6_HC | . | . | . | . | . | . | . | G | . | . | S | . | . | . | . | . | . | . | . | . | . | . | . | . | . | . | . | . |
| 21-225_4H6.004_HC | . | . | . | . | . | . | . | . | . | . | S | . | . | . | . | . | . | . | . | . | . | . | . | . | . | . | . | . |
| 21-225_4H6.005_HC | . | . | . | . | . | . | . | . | . | . | S | . | . | . | . | . | . | . | . | . | . | . | . | . | . | . | . | . |
| 21-225_4H6.006_HC | . | . | . | . | . | . | . | . | . | . | S | . | . | . | . | . | . | . | . | . | . | . | . | . | . | . | . | . |
| 21-225_4H6.007_HC | . | . | . | . | . | . | . | . | . | . | S | . | . | . | . | . | . | . | . | . | . | . | . | . | . | A | . | . |
| 21-225_4H6.008_HC | . | . | . | . | . | . | . | . | . | . | S | . | . | . | . | . | . | . | . | . | . | . | . | . | S | . | . | . |
| 21-225_4H6.009_HC | . | . | . | . | . | . | . | . | . | . | S | . | . | . | . | . | . | . | . | . | . | . | . | . | S | . | . | . |
| 21-225_4H6.010_HC | . | . | . | . | . | . | . | . | . | . | S | . | . | . | . | . | . | . | . | . | . | . | . | . | . | A | . | . |
| 21-225_4H6.011_HC | . | . | . | . | . | . | . | . | . | . | S | . | . | . | . | . | . | . | . | . | . | . | . | . | . | A | . | . |
| 21-225_4H6.014_HC | . | . | . | . | . | . | . | G | . | . | S | . | . | . | . | . | . | . | . | . | . | . | . | . | . | A | . | . |
| 21-225_50D4_HC | . | . | . | . | . | . | . | . | . | . | . | . | . | . | . | . | . | . | . | . | . | . | . | . | . | . | . | . |
| 21-225_51E9_HC | . | . | . | . | . | . | . | . | . | . | . | . | . | . | . | . | . | . | . | . | . | . | . | . | . | . | . | . |
| 21-225_52B1_HC | . | . | . | . | . | . | L | D | . | . | . | . | . | . | . | . | . | . | . | . | . | . | . | . | . | . | . | . |
| 21-225_52G11_HC | . | . | . | . | F | . | . | . | . | . | . | . | . | . | . | . | . | . | . | . | . | . | . | . | . | . | . | . |
| 21-225_52H10_HC | . | . | . | . | . | . | . | . | . | . | . | . | . | . | . | . | . | . | . | . | . | . | . | . | . | . | . | . |
| 21-225_53F2_HC | . | . | . | . | . | . | . | . | . | . | . | . | K | . | . | . | . | . | . | . | . | . | . | . | . | . | . | . |
| 21-225_53F7_HC | . | . | . | . | . | . | . | . | . | N | . | . | T | . | . | . | . | . | . | . | . | . | . | . | . | . | . | . |
| 21-225_56E5_HC | . | . | . | . | . | . | . | . | . | . | R | . | . | . | . | . | . | . | . | . | H | . | . | . | . | . | . | . |
| 21-225_7C3_LC1_HC | . | . | . | . | . | . | . | . | . | . | S | . | . | . | . | . | . | . | . | . | S | . | . | . | . | . | . | . |
| 21-225_7C3_LC2_HC | . | . | . | . | . | . | . | . | . | . | S | . | . | . | . | . | . | . | . | . | S | . | . | . | . | . | . | . |
| 21-225_9F12_LC1_HC | . | . | . | . | . | . | L | . | . | . | S | . | . | . | . | . | . | . | F | . | . | . | . | . | . | . | . | . |
| 21-225_9F12_LC2_HC | . | . | . | . | . | . | L | . | . | . | S | . | . | . | . | . | . | . | F | . | . | . | . | . | . | . | . | . |

FIGURE 57

| Reference # | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 | 136 | 137 | 138 | 139 | 140 |
|---|---|---|---|---|---|---|---|---|---|---|---|---|---|---|---|---|---|---|---|---|---|---|---|---|---|---|---|---|
| | | | | | | | | | | | H_CDR3 | | | | | | | | | | | | | | | | | |
| CONSENSUS | - | - | - | - | - | - | - | - | - | - | - | - | - | - | - | - | - | - | - | - | - | - | S | F | D | Y | W | G |
| 21-225_10G6_HC | . | . | . | . | . | . | . | . | . | . | . | . | . | . | . | . | . | . | . | . | . | . | . | . | . | . | . | . |
| 21-225_12E9_HC | . | . | . | . | . | . | . | . | . | . | . | . | . | . | . | . | . | . | . | . | . | . | . | . | . | . | . | . |
| 21-225_169C10_HC | V | A | T | . | . | . | . | . | . | . | . | . | . | . | . | . | . | . | . | . | W | G | V | . | . | . | . | . |
| 21-225_170D6_HC | V | A | T | . | . | . | . | . | . | . | . | . | . | . | . | . | . | . | . | . | W | G | V | . | . | . | . | . |
| 21-225_171H12_HC | V | A | T | . | . | . | . | . | . | . | . | . | . | . | . | . | . | . | . | . | W | G | V | . | . | . | . | . |
| 21-225_177A5_HC | V | A | T | . | . | . | . | . | . | . | . | . | . | . | . | . | . | . | . | . | W | G | V | . | . | . | . | . |
| 21-225_181G2_HC | . | . | . | . | . | . | . | . | . | . | . | . | . | . | . | . | . | . | . | . | . | . | - | - | G | D | . | . |
| 21-225_184E7_HC | . | . | . | . | . | . | . | . | . | . | . | . | . | . | . | . | . | . | . | . | . | . | - | - | G | D | . | . |
| 21-225_185E10_HC | . | . | . | . | . | . | . | . | . | . | . | . | . | . | . | . | . | . | . | . | . | . | - | - | G | D | . | . |
| 21-225_188B9_HC | . | . | . | . | . | . | . | . | . | . | . | . | . | . | . | . | . | . | . | . | . | . | - | - | G | D | . | . |
| 21-225_188H5_HC | . | . | . | . | . | . | . | . | . | . | . | . | . | . | . | . | . | . | . | . | . | . | - | - | G | D | . | . |
| 21-225_18D4_HC | . | . | . | . | . | . | . | . | . | . | . | . | . | . | . | . | . | . | . | . | . | . | L | . | . | . | . | . |
| 21-225_213H7_HC | S | . | . | . | . | . | . | . | . | . | . | . | . | . | . | . | . | . | . | . | . | . | Y | Y | K | . | . | . |
| 21-225_221A6_HC | . | . | . | . | . | . | . | . | . | . | . | . | . | . | . | . | . | . | . | . | . | . | . | . | . | . | . | . |
| 21-225_221F12_HC | . | . | . | . | . | . | . | . | . | . | . | . | . | . | . | . | . | . | . | . | . | . | . | . | . | . | . | . |
| 21-225_222C12_HC | . | . | . | . | . | . | . | . | . | . | . | . | . | . | . | . | . | . | . | . | . | . | . | . | . | . | . | . |
| 21-225_222E1_HC | . | . | . | . | . | . | . | . | . | . | . | . | . | . | . | . | . | . | . | . | . | . | . | . | . | . | . | . |
| 21-225_222F7_HC | . | . | . | . | . | . | . | . | . | . | . | . | . | . | . | . | . | . | . | . | . | . | . | . | . | . | . | . |
| 21-225_23B11_HC | . | . | . | . | . | . | . | . | . | . | . | . | . | . | . | . | . | . | . | . | . | . | . | . | . | . | . | . |
| 21-225_30A11_HC | . | . | . | . | . | . | . | . | . | . | . | . | . | . | . | . | . | . | . | . | . | . | . | . | . | . | . | . |
| 21-225_31C4_HC | . | . | . | . | . | . | . | . | . | . | . | . | . | . | . | . | . | . | . | . | . | . | . | . | . | . | . | . |
| 21-225_33G1_HC | . | . | . | . | . | . | . | . | . | . | . | . | . | . | . | . | . | . | . | . | . | . | . | . | . | . | . | . |
| 21-225_33H7_HC | . | . | . | . | . | . | . | . | . | . | . | . | . | . | . | . | . | . | . | . | . | . | . | . | . | . | . | . |
| 21-225_34F3_HC | . | . | . | . | . | . | . | . | . | . | . | . | . | . | . | . | . | . | . | . | . | . | . | . | . | . | . | . |
| 21-225_34G7_HC | . | . | . | . | . | . | . | . | . | . | . | . | . | . | . | . | . | . | . | . | . | . | . | . | . | . | . | . |
| 21-225_35C6_HC | . | . | . | . | . | . | . | . | . | . | . | . | . | . | . | . | . | . | . | . | . | . | . | . | . | . | . | . |
| 21-225_35G6_HC | . | . | . | . | . | . | . | . | . | . | . | . | . | . | . | . | . | . | . | . | . | . | . | . | . | . | . | . |
| 21-225_35H12_HC | . | . | . | . | . | . | . | . | . | . | . | . | . | . | . | . | . | . | . | . | . | . | . | . | . | . | . | . |

FIGURE 57

| Reference # | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 | 136 | 137 | 138 | 139 | 140 |
|---|---|---|---|---|---|---|---|---|---|---|---|---|---|---|---|---|---|---|---|---|---|---|---|---|---|---|---|---|
| | | | | | | | | | | | H_CDR3 | | | | | | | | | | | | | | | | | |
| CONSENSUS | - | - | - | - | - | - | - | - | - | - | - | - | - | - | - | - | - | - | - | - | - | - | S | F | D | Y | W | G |
| 21-225_36H5_HC | | | | | | | | | | | | | | | | | | | | | | | | | | | | |
| 21-225_49F10_HC | | | | | | | | | | | | | | | | | | | | | | | | | | F | | |
| 21-225_4H6_HC | | | | | | | | | | | | | | | | | | | | | | | | | | | | |
| 21-225_4H6.004_HC | | | | | | | | | | | | | | | | | | | | | | | | | | | | |
| 21-225_4H6.005_HC | | | | | | | | | | | | | | | | | | | | | | | | | | | | |
| 21-225_4H6.006_HC | | | | | | | | | | | | | | | | | | | | | | | | | | | | |
| 21-225_4H6.007_HC | | | | | | | | | | | | | | | | | | | | | | | | | | | | |
| 21-225_4H6.008_HC | | | | | | | | | | | | | | | | | | | | | | | | | | | | |
| 21-225_4H6.009_HC | | | | | | | | | | | | | | | | | | | | | | | | | | | | |
| 21-225_4H6.010_HC | | | | | | | | | | | | | | | | | | | | | | | | | | | | |
| 21-225_4H6.011_HC | | | | | | | | | | | | | | | | | | | | | | | | | | | | |
| 21-225_4H6.014_HC | | | | | | | | | | | | | | | | | | | | | | | | | | | | |
| 21-225_50D4_HC | | | | | | | | | | | | | | | | | | | | | | | | | | F | | |
| 21-225_51E9_HC | | | | | | | | | | | | | | | | | | | | | | | | | | | | |
| 21-225_52B1_HC | | | | | | | | | | | | | | | | | | | | | | | | | | | | |
| 21-225_52G11_HC | | | | | | | | | | | | | | | | | | | | | | | | | | | | |
| 21-225_52H10_HC | | | | | | | | | | | | | | | | | | | | | | | | | | | | |
| 21-225_53F2_HC | | | | | | | | | | | | | | | | | | | | | | | | | | | | |
| 21-225_53F7_HC | | | | | | | | | | | | | | | | | | | | | | | | | | | | |
| 21-225_56E5_HC | | | | | | | | | | | | | | | | | | | | | | | | | | | | |
| 21-225_7C3_LC1_HC | | | | | | | | | | | | | | | | | | | | | | | | | | | | |
| 21-225_7C3_LC2_HC | | | | | | | | | | | | | | | | | | | | | | | | | | | | |
| 21-225_9F12_LC1_HC | | | | | | | | | | | | | | | | | | | | | | | | | | | | |
| 21-225_9F12_LC2_HC | | | | | | | | | | | | | | | | | | | | | | | | | | | | |

...

**FIGURE 57**

| Reference # | 141 | 142 | 143 | 144 | 145 | 146 | 147 | 148 | 149 |
|---|---|---|---|---|---|---|---|---|---|
| **H_FR4** | | | | | | | | | |
| CONSENSUS | Q | G | T | L | V | T | V | S | S |
| 21-225_10G6_HC | . | . | . | . | . | . | . | . | . |
| 21-225_12E9_HC | . | . | . | . | . | . | . | . | . |
| 21-225_169C10_HC | . | . | . | . | . | . | . | . | . |
| 21-225_170D6_HC | . | . | . | . | . | . | . | . | . |
| 21-225_171H12_HC | . | . | . | . | . | . | . | . | . |
| 21-225_177A5_HC | . | . | . | . | . | . | . | . | . |
| 21-225_181G2_HC | . | . | . | . | . | . | . | . | . |
| 21-225_184E7_HC | . | . | . | . | . | . | . | . | . |
| 21-225_185E10_HC | . | . | . | . | . | . | . | . | . |
| 21-225_188B9_HC | . | . | . | . | . | . | . | . | . |
| 21-225_188H5_HC | . | . | . | . | . | . | . | . | . |
| 21-225_18D4_HC | . | . | . | . | . | . | . | . | . |
| 21-225_213H7_HC | . | . | . | . | . | . | . | . | . |
| 21-225_221A6_HC | . | . | . | . | . | . | . | . | . |
| 21-225_221F12_HC | . | . | . | . | . | . | . | . | . |
| 21-225_222C12_HC | . | . | . | . | . | . | . | . | . |
| 21-225_222E1_HC | . | . | . | . | . | . | . | . | . |
| 21-225_222F7_HC | . | . | . | . | . | . | . | . | . |
| 21-225_23B11_HC | . | . | . | . | . | . | . | . | . |
| 21-225_30A11_HC | . | . | . | . | . | . | . | . | . |
| 21-225_31C4_HC | . | . | . | . | . | . | . | . | . |
| 21-225_33G1_HC | . | . | . | . | . | . | . | . | . |
| 21-225_33H7_HC | . | . | . | . | . | . | . | . | . |
| 21-225_34F3_HC | . | . | . | . | . | . | . | . | . |
| 21-225_34G7_HC | . | . | N | . | . | . | . | . | . |
| 21-225_35C6_HC | . | . | . | . | . | . | . | . | . |
| 21-225_35G6_HC | . | . | . | . | . | . | . | . | . |
| 21-225_35H12_HC | . | . | . | . | . | . | . | . | . |

| Reference # | 141 | 142 | 143 | 144 | 145 | 146 | 147 | 148 | 149 |
|---|---|---|---|---|---|---|---|---|---|
| **H_FR4** | | | | | | | | | |
| CONSENSUS | Q | G | T | L | V | T | V | S | S |
| 21-225_36H5_HC | . | . | . | . | . | . | . | . | . |
| 21-225_49F10_HC | . | . | . | . | . | . | . | . | . |
| 21-225_4H6_HC | . | . | . | . | . | . | . | . | . |
| 21-225_4H6.004_HC | . | . | . | . | . | . | . | . | . |
| 21-225_4H6.005_HC | . | . | . | . | . | . | . | . | . |
| 21-225_4H6.006_HC | . | . | . | . | . | . | . | . | . |
| 21-225_4H6.007_HC | . | . | . | . | . | . | . | . | . |
| 21-225_4H6.008_HC | . | . | . | . | . | . | . | . | . |
| 21-225_4H6.009_HC | . | . | . | . | . | . | . | . | . |
| 21-225_4H6.010_HC | . | . | . | . | . | . | . | . | . |
| 21-225_4H6.011_HC | . | . | . | . | . | . | . | . | . |
| 21-225_4H6.014_HC | . | . | . | . | . | . | . | . | . |
| 21-225_50D4_HC | . | . | . | . | . | . | . | . | . |
| 21-225_51E9_HC | . | . | . | . | . | . | . | . | . |
| 21-225_52B1_HC | . | . | . | . | . | . | . | . | . |
| 21-225_52G11_HC | . | . | . | . | . | . | . | . | . |
| 21-225_52H10_HC | . | . | . | . | . | . | . | . | . |
| 21-225_53F2_HC | . | . | . | . | . | . | . | . | . |
| 21-225_53F7_HC | . | . | . | . | . | . | . | . | . |
| 21-225_56E5_HC | . | . | . | . | . | . | . | . | . |
| 21-225_7C3_LC1_HC | . | . | . | . | . | . | . | . | . |
| 21-225_7C3_LC2_HC | . | . | . | . | . | . | . | . | . |
| 21-225_9F12_LC1_HC | . | . | . | . | . | . | . | . | . |
| 21-225_9F12_LC2_HC | . | . | . | . | . | . | . | . | . |

**FIGURE 57**

Table 56.    Consensus 22- VH3|3-33/D4|4-11|RF2/JH6 (SEQ ID NO: 50273):

QVQLVES-GGGVVQPGRSLRLSCAASG-FTFSS-----YGMHWVRQAPGKGLEWVAVIWHD---
GSNKYYADSVKGRFTISRDNSKNTLYLQMNSLRAEDTAVYYCARDLSMG--------------------GMDVWGQGTTVTVSS wherein:

S at position 33 can be substituted with N

Y at position 39 can be substituted with F or H

M at position 41 can be substituted with I

V at position 57 can be substituted with I

W at position 59 can be substituted with I

H at position 60 can be substituted with Y, F or N

D at position 61 can be substituted with S or E

G at position 65 can be substituted with A

S at position 66 can be substituted with G

N at position 67 can be substituted with Y

K at position 68 can be substituted with D, E or R

Y at position 69 can be substituted with N

Y at position 70 can be substituted with N

A at position 71 can be substituted with V or G

D at position 72 can be substituted with E

S at position 73 can be substituted with A

K at position 75 can be substituted with R

FIGURE 57

D at position 109 can be substituted with T or R

L at position 110 can be substituted with R, Y, S, F, I or P

S at position 111 can be substituted with R or T

M at position 112 can be substituted with V, G, P, K, N or Y

G at position 113 can be substituted with Y, null (-) or S

null (-) at position 114 can be substituted with Y, S or W

null (-) at position 115 can be substituted with G

null (-) at position 116 can be substituted with S

null (-) at position 117 can be substituted with G

null (-) at position 118 can be substituted with S

null (-) at position 119 can be substituted with P

null (-) at position 129 can be substituted with P

null (-) at position 130 can be substituted with Y

null (-) at position 131 can be substituted with Y

null (-) at position 132 can be substituted with Y

null (-) at position 133 can be substituted with S or Y

null (-) at position 134 can be substituted with D, Y or G

M at position 136 can be substituted with L or T

Xaa Xaa Gly Xaa His (SEQ ID NO: 50022)

**FIGURE 57**

Xaa Ile Xaa Xaa Xaa Xaa Xaa Xaa Xaa Xaa Xaa Xaa Xaa Xaa Val Xaa Gly (SEQ ID NO: 50023)

Xaa Xaa Xaa Xaa Xaa Xaa Xaa Xaa Xaa Xaa Xaa Xaa Xaa Xaa Xaa Xaa Gly Xaa Asp Val (SEQ ID NO: 50024)

Ser Tyr Gly Met His (SEQ ID NO: 50489)

Val Ile Trp His Asp Gly Ser Asn Lys Tyr Tyr Ala Asp Ser Val Lys Gly (SEQ ID NO: 50490)

Asp Leu Ser Met Gly Gly Met Asp Val (SEQ ID NO: 50491)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | . | . | . | . | . | . | . | . | . | . | . |
| 21-225_171E12_HC | . | . | . | . | . | . | . | . | . | . | . | . | . | . | . | . | . | . | . | . | . | . | . | . | . |
| 21-225_179H5_HC | . | . | . | . | . | . | . | . | . | . | . | . | . | . | . | . | . | . | . | . | . | . | . | . | . |
| 21-225_18G4_HC | . | . | . | . | . | . | . | . | . | . | . | . | . | . | . | . | . | . | . | . | . | . | . | . | . |
| 21-225_190A5_HC | . | . | . | . | . | . | . | . | . | . | . | . | G | . | . | . | . | . | . | . | . | . | . | . | . |
| 21-225_190D10_HC | . | . | . | . | . | . | . | . | . | . | . | . | . | . | . | . | . | . | . | . | . | . | . | . | . |
| 21-225_190F12_HC | . | . | . | . | . | . | . | . | . | . | . | . | . | . | . | . | . | . | . | . | . | . | . | . | . |
| 21-225_190H7_HC | . | . | . | . | . | . | . | . | . | . | . | . | . | . | . | . | . | . | . | . | . | . | . | . | . |
| 21-225_190H9_HC | . | . | . | . | . | . | . | . | . | . | . | . | . | . | . | . | . | . | . | . | . | . | . | . | . |
| 21-225_192C10_HC | . | . | . | . | . | . | . | . | . | . | . | . | . | . | . | . | . | . | . | . | . | . | . | . | S |
| 21-225_192E4_HC | . | . | . | . | . | . | . | . | . | . | . | . | . | . | . | . | . | . | . | . | . | . | . | . | . |
| 21-225_192H6_HC | . | . | . | . | . | . | . | . | . | . | . | . | . | . | . | . | . | . | . | . | . | . | . | V | . |
| 21-225_194B7_HC | . | . | . | . | . | . | . | . | . | . | . | . | . | . | . | . | . | . | . | . | . | . | . | . | . |
| 21-225_194H12_HC | . | . | . | . | . | . | . | . | . | . | . | . | . | . | . | . | . | . | . | . | . | . | . | . | . |
| 21-225_196D4_HC | . | . | . | V | . | . | . | . | . | . | . | . | . | . | . | . | . | . | . | . | . | . | . | . | . |
| 21-225_197E11_HC | . | . | . | . | . | . | . | . | . | . | . | . | . | . | . | . | . | . | . | . | . | . | . | . | . |
| 21-225_198D2_HC | . | . | . | . | . | . | . | . | . | . | . | . | . | . | . | . | . | . | . | . | . | . | . | . | . |
| 21-225_205H3_HC | . | . | . | . | . | . | . | . | . | . | . | . | . | . | . | . | . | . | . | . | . | . | . | . | . |
| 21-225_20F6_HC | . | . | . | . | . | . | . | . | . | . | . | . | . | . | . | . | . | . | . | . | . | . | . | V | . |
| 21-225_22C7_HC | . | . | . | . | . | . | . | . | . | . | . | . | . | . | . | . | . | . | . | . | . | . | . | . | . |
| 21-225_25B3_HC | . | . | . | . | . | . | . | . | . | . | . | . | . | . | . | . | . | . | . | . | . | . | . | . | . |
| 21-225_26D12_HC | . | . | . | . | . | . | . | . | . | . | . | . | . | . | . | . | . | . | . | . | . | . | . | . | . |
| 21-225_26G11_HC | . | . | . | . | . | . | . | . | . | . | . | . | . | . | . | . | . | . | . | . | . | . | . | . | . |
| 21-225_27A11_HC | . | . | . | . | . | . | . | . | . | . | . | . | . | . | . | . | . | . | . | . | . | . | . | . | . |
| 21-225_3F8_HC | . | . | . | . | . | . | . | . | . | . | . | . | . | . | . | . | . | . | . | . | . | . | . | . | . |
| 21-225_6G1_HC | . | . | . | . | . | . | . | . | . | . | . | . | . | . | . | . | . | . | . | . | . | . | . | . | . |
| 21-225_7E11_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | | H_FR1 | | | | | | | | |
| CONSENSUS | Q | V | Q | L | V | E | S | - | G | G | G | V | V | Q | P | G | R | S | L | R | L | S | C | A | A |
| 21-225_7E11.001_HC | . | . | . | . | . | . | . | . | . | . | . | . | . | . | . | . | . | . | . | . | . | . | . | . | . |
| 21-225_7E11.001.001_HC | . | . | . | . | . | . | . | . | . | . | . | . | . | . | . | . | . | . | . | . | . | . | . | . | . |
| 21-225_7E11.001.002_HC | . | . | . | . | . | . | . | . | . | . | . | . | . | . | . | . | . | . | . | . | . | . | . | . | . |
| 21-225_7E11.001.003_HC | . | . | . | . | . | . | . | . | . | . | . | . | . | . | . | . | . | . | . | . | . | . | . | . | . |
| 21-225_7E11.001.004_HC | . | . | . | . | . | . | . | . | . | . | . | . | . | . | . | . | . | . | . | . | . | . | . | . | . |
| 21-225_7E11.001.005_HC | . | . | . | . | . | . | . | . | . | . | . | . | . | . | . | . | . | . | . | . | . | . | . | . | . |
| 21-225_7E11.001.006_HC | . | . | . | . | . | . | . | . | . | . | . | . | . | . | . | . | . | . | . | . | . | . | . | . | . |
| 21-225_7E11.001.007_HC | . | . | . | . | . | . | . | . | . | . | . | . | . | . | . | . | . | . | . | . | . | . | . | . | . |
| 21-225_7E11.001.008_HC | . | . | . | . | . | . | . | . | . | . | . | . | . | . | . | . | . | . | . | . | . | . | . | . | . |
| 21-225_7E11.001.009_HC | . | . | . | . | . | . | . | . | . | . | . | . | . | . | . | . | . | . | . | . | . | . | . | . | . |
| 21-225_7E11.001.010_HC | . | . | . | . | . | . | . | . | . | . | . | . | . | . | . | . | . | . | . | . | . | . | . | . | . |
| 21-225_7E11.001.011_HC | . | . | . | . | . | . | . | . | . | . | . | . | . | . | . | . | . | . | . | . | . | . | . | . | . |
| 21-225_7E11.001.012_HC | . | . | . | . | . | . | . | . | . | . | . | . | . | . | . | . | . | . | . | . | . | . | . | . | . |
| 21-225_7E11.001.013_HC | . | . | . | . | . | . | . | . | . | . | . | . | . | . | . | . | . | . | . | . | . | . | . | . | . |
| 21-225_7E11.001.014_HC | . | . | . | . | . | . | . | . | . | . | . | . | . | . | . | . | . | . | . | . | . | . | . | . | . |
| 21-225_7E11.001.015_HC | . | . | . | . | . | . | . | . | . | . | . | . | . | . | . | . | . | . | . | . | . | . | . | . | . |
| 21-225_7E11.001.016_HC | . | . | . | . | . | . | . | . | . | . | . | . | . | . | . | . | . | . | . | . | . | . | . | . | . |
| 21-225_7E11.001.017_HC | . | . | . | . | . | . | . | . | . | . | . | . | . | . | . | . | . | . | . | . | . | . | . | . | . |
| 21-225_7E11.001.018_HC | . | . | . | . | . | . | . | . | . | . | . | . | . | . | . | . | . | . | . | . | . | . | . | . | . |
| 21-225_7E11.001.023_HC | . | . | . | . | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H CDR1 | | | | | | | | | | | | | H_FR2 |
| CONSENSUS | S | G | - | F | T | F | S | S | - | - | - | - | - | Y | G | M | H | W | V | R | Q | A | P | G | K |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | I | . | . | . | . | . | . | . | . | . |
| 21-225_16E6_HC | . | . | . | . | . | . | . | . | . | . | . | . | . | . | . | . | . | . | . | . | . | . | . | . | . |
| 21-225_171E12_HC | . | . | . | . | . | . | . | . | . | . | . | . | . | . | . | . | . | . | . | . | . | . | . | . | . |
| 21-225_179H5_HC | . | . | . | . | . | . | . | . | . | . | . | . | . | . | . | . | . | . | . | . | . | . | . | . | . |
| 21-225_18G4_HC | . | . | . | . | . | . | . | . | . | . | . | . | . | . | . | . | . | . | . | . | . | . | . | . | . |
| 21-225_190A5_HC | . | . | . | . | . | . | . | . | . | . | . | . | . | . | . | . | . | . | . | . | . | . | . | . | . |
| 21-225_190D10_HC | . | . | . | . | . | . | R | . | . | . | . | . | . | . | . | . | . | . | . | . | . | . | . | . | . |
| 21-225_190F12_HC | . | . | . | . | . | . | R | N | . | . | . | . | . | . | . | . | . | . | . | . | . | . | . | . | . |
| 21-225_190H7_HC | . | . | . | . | N | . | R | . | . | . | . | . | . | . | . | . | . | . | . | . | . | . | . | . | . |
| 21-225_190H9_HC | . | . | . | . | . | . | R | N | . | . | . | . | . | . | . | . | . | . | . | . | . | . | . | . | . |
| 21-225_192C10_HC | . | . | . | . | . | . | R | N | . | . | . | . | . | . | . | . | . | . | . | . | . | . | . | . | . |
| 21-225_192E4_HC | . | . | . | . | . | . | . | . | . | . | . | . | . | . | . | . | . | . | . | . | . | . | . | . | . |
| 21-225_192H6_HC | . | . | . | . | . | . | . | . | . | . | . | . | . | H | . | . | . | . | . | . | . | . | . | . | . |
| 21-225_194B7_HC | . | . | . | . | . | . | . | . | . | . | . | . | . | . | . | . | . | . | . | . | . | . | . | . | . |
| 21-225_194H12_HC | . | . | . | . | . | . | R | N | . | . | . | . | . | . | . | . | . | . | . | . | . | . | . | . | . |
| 21-225_196D4_HC | . | . | . | . | . | . | . | . | . | . | . | . | . | . | . | . | . | . | . | . | . | . | . | . | . |
| 21-225_197E11_HC | . | . | . | . | . | . | R | N | . | . | . | . | . | . | . | . | . | . | . | . | . | . | . | . | . |
| 21-225_198D2_HC | . | . | . | . | . | . | R | . | . | . | . | . | . | . | . | . | . | . | . | . | . | . | . | . | . |
| 21-225_205H3_HC | . | . | . | . | . | . | . | . | . | . | . | . | . | . | . | . | . | . | . | . | . | . | . | . | . |
| 21-225_20F6_HC | . | . | . | . | . | . | . | . | . | . | . | . | . | . | . | . | . | . | . | . | . | . | . | . | . |
| 21-225_22C7_HC | . | . | . | . | . | . | . | . | . | . | . | . | . | . | . | . | . | . | . | . | . | . | . | . | . |
| 21-225_25B3_HC | . | . | . | . | . | . | . | . | . | . | . | . | . | . | . | . | . | . | . | . | . | . | . | . | . |
| 21-225_26D12_HC | . | . | . | . | . | . | . | . | . | . | . | . | . | . | . | . | . | . | . | . | . | . | . | . | . |
| 21-225_26G11_HC | . | . | . | . | . | . | . | . | . | . | . | . | . | . | . | . | . | . | . | . | . | . | . | . | . |
| 21-225_27A11_HC | . | . | . | . | . | . | . | . | . | . | . | . | . | . | . | . | . | . | . | . | . | . | . | . | . |
| 21-225_3F8_HC | . | . | . | . | . | . | . | . | . | . | . | . | . | . | . | . | . | . | . | . | . | . | . | . | . |
| 21-225_6G1_HC | . | . | . | . | I | . | . | . | . | . | . | . | . | . | . | . | . | . | . | . | . | . | . | . | . |
| 21-225_7E11_HC | . | . | . | . | . | . | . | . | . | . | . | . | . | F | . | . | . | . | . | . | . | . | . | . | . |

**FIGURE 57**

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | H_CDR1 | | | | | | | | | | | | | | H_FR2 | |
| CONSENSUS | S | G | - | F | T | F | S | S | - | - | - | - | - | Y | G | M | H | W | V | R | Q | A | P | G | K |
| 21-225_7E11.001_HC | . | . | . | . | . | . | . | . | . | . | . | . | . | F | . | . | . | . | . | . | . | . | . | . | . |
| 21-225_7E11.001.001_HC | . | . | . | . | . | . | . | . | . | . | . | . | . | F | . | . | . | . | . | . | . | . | . | . | . |
| 21-225_7E11.001.002_HC | . | . | . | . | . | . | . | . | . | . | . | . | . | F | . | . | . | . | . | . | . | . | . | . | . |
| 21-225_7E11.001.003_HC | . | . | . | . | . | . | . | . | . | . | . | . | . | F | . | . | . | . | . | . | . | . | . | . | . |
| 21-225_7E11.001.004_HC | . | . | . | . | . | . | . | . | . | . | . | . | . | F | . | . | . | . | . | . | . | . | . | . | . |
| 21-225_7E11.001.005_HC | . | . | . | . | . | . | . | . | . | . | . | . | . | F | . | . | . | . | . | . | . | . | . | . | . |
| 21-225_7E11.001.006_HC | . | . | . | . | . | . | . | . | . | . | . | . | . | F | . | . | . | . | . | . | . | . | . | . | . |
| 21-225_7E11.001.007_HC | . | . | . | . | . | . | . | . | . | . | . | . | . | F | . | . | . | . | . | . | . | . | . | . | . |
| 21-225_7E11.001.008_HC | . | . | . | . | . | . | . | . | . | . | . | . | . | F | . | . | . | . | . | . | . | . | . | . | . |
| 21-225_7E11.001.009_HC | . | . | . | . | . | . | . | . | . | . | . | . | . | F | . | . | . | . | . | . | . | . | . | . | . |
| 21-225_7E11.001.010_HC | . | . | . | . | . | . | . | . | . | . | . | . | . | F | . | . | . | . | . | . | . | . | . | . | . |
| 21-225_7E11.001.011_HC | . | . | . | . | . | . | . | . | . | . | . | . | . | F | . | . | . | . | . | . | . | . | . | . | . |
| 21-225_7E11.001.012_HC | . | . | . | . | . | . | . | . | . | . | . | . | . | F | . | . | . | . | . | . | . | . | . | . | . |
| 21-225_7E11.001.013_HC | . | . | . | . | . | . | . | . | . | . | . | . | . | F | . | . | . | . | . | . | . | . | . | . | . |
| 21-225_7E11.001.014_HC | . | . | . | . | . | . | . | . | . | . | . | . | . | F | . | . | . | . | . | . | . | . | . | . | . |
| 21-225_7E11.001.015_HC | . | . | . | . | . | . | . | . | . | . | . | . | . | F | . | . | . | . | . | . | . | . | . | . | . |
| 21-225_7E11.001.016_HC | . | . | . | . | . | . | . | . | . | . | . | . | . | F | . | . | . | . | . | . | . | . | . | . | . |
| 21-225_7E11.001.017_HC | . | . | . | . | . | . | . | . | . | . | . | . | . | F | . | . | . | . | . | . | . | . | . | . | . |
| 21-225_7E11.001.018_HC | . | . | . | . | . | . | . | . | . | . | . | . | . | F | . | . | . | . | . | . | . | . | . | . | . |
| 21-225_7E11.001.023_HC | . | . | . | . | . | . | . | . | . | . | . | . | . | F | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H CDR2 | | | | | | | | |
| CONSENSUS | G | L | E | W | V | A | V | I | W | H | D | - | - | - | G | S | N | K | Y | Y | A | D | S | V | K |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | . | . | . | . | . | . | V | . | . | . | . |
| 21-225_171E12_HC | . | . | . | . | . | . | . | . | . | N | . | . | . | . | . | G | . | . | . | N | G | . | . | . | . |
| 21-225_179H5_HC | . | . | . | . | . | . | . | . | . | Y | . | . | . | . | . | . | . | E | . | . | . | . | . | . | . |
| 21-225_18G4_HC | . | . | . | . | . | . | I | . | . | Y | . | . | . | . | . | . | . | . | . | . | . | . | . | . | . |
| 21-225_190A5_HC | . | . | . | . | . | . | . | . | . | Y | . | . | . | . | . | . | . | . | N | . | . | . | . | . | . |
| 21-225_190D10_HC | . | . | . | . | . | . | . | . | I | Y | . | . | . | . | . | . | Y | . | . | . | . | . | . | . | . |
| 21-225_190F12_HC | . | . | . | . | . | . | . | . | . | F | . | . | . | . | . | . | . | D | . | . | . | . | . | . | . |
| 21-225_190H7_HC | . | . | . | . | . | . | . | . | I | Y | . | . | . | . | . | . | Y | . | . | . | . | . | . | . | . |
| 21-225_190H9_HC | . | . | . | . | . | . | . | . | . | F | . | . | . | . | . | . | . | D | . | . | . | . | . | . | . |
| 21-225_192C10_HC | . | . | K | . | . | . | . | . | . | F | . | . | . | . | . | . | . | D | . | . | . | . | . | . | . |
| 21-225_192E4_HC | . | . | . | . | . | . | . | . | . | Y | . | . | . | . | . | . | . | . | N | . | V | . | . | . | R |
| 21-225_192H6_HC | . | . | . | . | . | . | . | . | . | Y | . | . | . | . | . | . | . | . | N | . | . | . | . | . | . |
| 21-225_194B7_HC | . | . | . | . | . | . | . | . | . | Y | . | . | . | . | . | . | . | . | N | . | . | . | . | . | . |
| 21-225_194H12_HC | . | . | . | . | . | . | . | . | . | F | . | . | . | . | . | . | . | D | . | . | . | . | . | . | . |
| 21-225_196D4_HC | . | . | . | . | . | . | . | . | . | Y | . | . | . | . | . | . | . | R | N | . | . | . | . | . | . |
| 21-225_197E11_HC | . | . | . | . | . | . | . | . | . | F | . | . | . | . | . | . | . | D | . | . | . | . | . | . | . |
| 21-225_198D2_HC | . | . | . | . | . | . | . | . | I | Y | . | . | . | . | . | . | Y | . | . | . | . | . | . | . | . |
| 21-225_205H3_HC | . | . | . | . | . | . | . | . | . | Y | . | . | . | . | . | . | . | . | . | . | . | . | . | . | . |
| 21-225_20F6_HC | . | . | . | . | G | . | . | . | . | . | . | . | . | . | . | . | . | . | . | . | . | . | . | . | . |
| 21-225_22C7_HC | . | . | . | . | G | . | . | . | . | . | . | . | . | . | . | . | . | . | . | . | . | . | . | . | . |
| 21-225_25B3_HC | . | . | . | . | . | . | . | . | . | Y | . | . | . | . | . | . | . | . | . | . | . | . | . | . | . |
| 21-225_26D12_HC | . | . | . | . | . | . | . | . | . | Y | . | . | . | . | . | . | . | E | . | . | . | . | . | . | . |
| 21-225_26G11_HC | . | . | . | . | . | . | . | . | . | Y | . | . | . | . | . | . | . | . | . | . | . | . | . | . | . |
| 21-225_27A11_HC | . | . | . | . | . | . | . | . | . | Y | . | . | . | . | . | . | . | . | . | . | . | . | . | . | . |
| 21-225_3F8_HC | . | . | . | . | G | . | . | . | . | . | . | . | . | . | . | . | . | . | . | . | . | . | . | . | . |
| 21-225_6G1_HC | . | . | . | . | . | . | I | . | . | Y | . | . | . | . | . | . | . | . | . | . | . | . | . | . | . |
| 21-225_7E11_HC | . | . | . | . | . | . | I | . | . | . | . | . | . | . | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H_CDR2 | | | | | | | | |
| CONSENSUS | G | L | E | W | V | A | V | I | W | H | D | - | - | - | G | S | N | K | Y | Y | A | D | S | V | K |
| 21-225_7E11.001_HC | . | . | . | . | . | . | I | . | . | . | . | . | . | . | . | . | . | . | . | . | . | . | . | . | . |
| 21-225_7E11.001.001_HC | . | . | . | . | . | . | I | . | . | . | . | . | . | . | . | . | . | . | . | . | . | . | . | . | . |
| 21-225_7E11.001.002_HC | . | . | . | . | . | . | I | . | . | . | . | . | . | . | . | . | . | . | . | . | . | . | . | . | . |
| 21-225_7E11.001.003_HC | . | . | . | . | . | . | I | . | . | . | S | . | . | . | . | . | . | . | . | . | . | . | . | . | . |
| 21-225_7E11.001.004_HC | . | . | . | . | . | . | I | . | . | . | E | . | . | . | . | . | . | . | . | . | . | . | . | . | . |
| 21-225_7E11.001.005_HC | . | . | . | . | . | . | I | . | . | . | . | . | . | . | A | . | . | . | . | . | . | . | . | . | . |
| 21-225_7E11.001.006_HC | . | . | . | . | . | . | I | . | . | . | . | . | . | . | . | . | . | . | . | . | . | E | . | . | . |
| 21-225_7E11.001.007_HC | . | . | . | . | . | . | I | . | . | . | . | . | . | . | . | . | . | . | . | . | . | . | A | . | . |
| 21-225_7E11.001.008_HC | . | . | . | . | . | . | I | . | . | . | E | . | . | . | . | . | . | . | . | . | . | . | A | . | . |
| 21-225_7E11.001.009_HC | . | . | . | . | . | . | I | . | . | . | E | . | . | . | . | . | . | . | . | . | . | E | . | . | . |
| 21-225_7E11.001.010_HC | . | . | . | . | . | . | I | . | . | . | S | . | . | . | . | . | . | . | . | . | . | E | . | . | . |
| 21-225_7E11.001.011_HC | . | . | . | . | . | . | I | . | . | . | S | . | . | . | . | . | . | . | . | . | . | . | A | . | . |
| 21-225_7E11.001.012_HC | . | . | . | . | . | . | I | . | . | . | S | . | . | . | . | . | . | . | . | . | . | . | . | . | . |
| 21-225_7E11.001.013_HC | . | . | . | . | . | . | I | . | . | . | . | . | . | . | . | . | . | . | . | . | . | E | . | . | . |
| 21-225_7E11.001.014_HC | . | . | . | . | . | . | I | . | . | . | S | . | . | . | . | . | . | . | . | . | . | E | . | . | . |
| 21-225_7E11.001.015_HC | . | . | . | . | . | . | I | . | . | . | S | . | . | . | . | . | . | . | . | . | . | . | . | . | . |
| 21-225_7E11.001.016_HC | . | . | . | . | . | . | I | . | . | . | S | . | . | . | . | . | . | . | . | . | . | E | . | . | . |
| 21-225_7E11.001.017_HC | . | . | . | . | . | . | I | . | . | . | E | . | . | . | . | . | . | . | . | . | . | . | A | . | . |
| 21-225_7E11.001.018_HC | . | . | . | . | . | . | I | . | . | . | . | . | . | . | A | . | . | . | . | . | . | E | . | . | . |
| 21-225_7E11.001.023_HC | . | . | . | . | . | . | I | . | . | . | . | . | . | . | A | . | . | . | . | . | . | . | A | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | | H FR3 | | | | | | | |
| CONSENSUS | G | R | F | T | I | S | R | D | N | S | K | N | T | L | Y | L | Q | M | N | S | L | R | A | E | D |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | N | . | . | . | . | . | . | . | . | . | . |
| 21-225_171E12_HC | . | . | . | . | . | . | . | . | . | . | . | . | . | . | . | . | . | . | . | . | . | . | . | . | . |
| 21-225_179H5_HC | . | . | . | . | . | . | . | . | . | . | . | . | . | . | . | . | . | . | . | . | . | . | . | . | . |
| 21-225_18G4_HC | . | . | . | . | . | . | . | . | . | . | . | . | . | . | . | . | . | . | . | . | . | . | . | . | . |
| 21-225_190A5_HC | . | . | . | . | . | . | . | . | . | . | . | . | . | . | . | . | . | . | . | . | . | . | . | . | . |
| 21-225_190D10_HC | . | . | . | . | . | . | . | . | . | . | . | . | . | . | . | . | . | . | . | . | . | . | . | . | . |
| 21-225_190F12_HC | . | . | . | . | . | . | . | . | . | . | . | . | . | . | S | . | . | . | . | . | . | . | . | . | . |
| 21-225_190H7_HC | . | . | . | . | . | . | . | . | . | . | . | . | . | . | . | . | . | . | . | . | . | . | . | . | . |
| 21-225_190H9_HC | . | . | . | . | . | . | . | . | . | . | . | . | . | . | S | . | . | . | . | . | . | . | . | . | . |
| 21-225_192C10_HC | . | . | . | . | . | . | . | . | . | . | . | . | . | . | S | . | . | . | . | . | . | . | . | . | . |
| 21-225_192E4_HC | . | . | . | . | . | . | . | . | . | . | . | . | . | . | . | . | . | . | . | . | . | . | . | . | . |
| 21-225_192H6_HC | . | . | . | . | . | . | . | . | . | . | . | . | . | . | . | . | . | . | . | . | . | . | . | . | . |
| 21-225_194B7_HC | . | . | . | . | . | . | . | . | . | . | . | . | . | . | . | . | . | . | . | . | . | . | . | . | . |
| 21-225_194H12_HC | . | . | . | . | . | . | . | . | . | . | . | . | . | . | S | . | . | . | . | . | . | . | . | . | . |
| 21-225_196D4_HC | . | . | . | . | . | . | . | . | . | . | . | . | . | . | . | . | . | . | . | . | . | . | V | . | . |
| 21-225_197E11_HC | . | . | . | . | . | . | . | . | . | . | . | . | . | . | S | . | . | . | . | . | . | . | . | . | . |
| 21-225_198D2_HC | . | . | . | . | . | . | . | . | . | . | . | . | . | . | . | . | . | . | . | . | . | . | . | . | . |
| 21-225_205H3_HC | . | . | . | . | . | . | . | . | . | . | . | . | . | . | . | . | . | . | . | . | . | . | . | . | . |
| 21-225_20F6_HC | . | . | . | . | . | . | . | . | A | . | . | . | . | . | . | . | . | . | . | . | . | . | . | . | . |
| 21-225_22C7_HC | . | . | . | . | . | . | . | . | A | . | . | . | . | . | . | . | . | . | . | . | . | . | . | . | . |
| 21-225_25B3_HC | . | . | . | . | . | . | . | . | . | . | . | . | . | . | . | . | . | . | . | . | . | . | . | . | . |
| 21-225_26D12_HC | . | . | . | . | . | . | . | . | . | . | . | . | . | . | . | . | . | . | . | . | . | . | . | A | . |
| 21-225_26G11_HC | . | . | . | . | . | . | . | . | . | . | . | . | . | . | . | . | . | . | . | . | . | . | . | . | . |
| 21-225_27A11_HC | . | . | . | . | . | . | . | . | . | . | . | . | . | . | . | . | . | . | . | . | . | . | . | . | . |
| 21-225_3F8_HC | . | . | . | . | . | . | . | . | A | . | . | . | . | . | . | . | . | . | . | . | . | . | . | . | . |
| 21-225_6G1_HC | . | . | . | . | . | . | . | . | . | . | . | . | . | . | . | . | . | . | . | . | . | . | . | . | . |
| 21-225_7E11_HC | . | . | . | . | . | . | . | . | . | . | N | . | . | . | . | . | . | . | S | . | . | . | . | . | . |

FIGURE 57

| Reference # | 75 | 77 | 76 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | | H_FR3 | | | | | | | |
| CONSENSUS | G | R | F | T | I | S | R | D | N | S | K | N | T | L | Y | L | Q | M | N | S | L | R | A | E | D |
| 21-225_7E11.001_HC | . | . | . | . | . | . | . | . | . | . | . | . | . | . | . | . | . | . | S | . | . | . | . | . | . |
| 21-225_7E11.001.001_HC | . | . | . | . | . | . | . | . | . | . | . | . | . | . | . | . | . | . | S | . | . | . | . | . | . |
| 21-225_7E11.001.002_HC | . | . | . | . | . | . | . | . | . | . | . | . | . | . | . | . | . | . | S | . | . | . | . | . | . |
| 21-225_7E11.001.003_HC | . | . | . | . | . | . | . | . | . | . | . | . | . | . | . | . | . | . | S | . | . | . | . | . | . |
| 21-225_7E11.001.004_HC | . | . | . | . | . | . | . | . | . | . | . | . | . | . | . | . | . | . | S | . | . | . | . | . | . |
| 21-225_7E11.001.005_HC | . | . | . | . | . | . | . | . | . | . | . | . | . | . | . | . | . | . | S | . | . | . | . | . | . |
| 21-225_7E11.001.006_HC | . | . | . | . | . | . | . | . | . | . | . | . | . | . | . | . | . | . | S | . | . | . | . | . | . |
| 21-225_7E11.001.007_HC | . | . | . | . | . | . | . | . | . | . | . | . | . | . | . | . | . | . | S | . | . | . | . | . | . |
| 21-225_7E11.001.008_HC | . | . | . | . | . | . | . | . | . | . | . | . | . | . | . | . | . | . | S | . | . | . | . | . | . |
| 21-225_7E11.001.009_HC | . | . | . | . | . | . | . | . | . | . | . | . | . | . | . | . | . | . | S | . | . | . | . | . | . |
| 21-225_7E11.001.010_HC | . | . | . | . | . | . | . | . | . | . | . | . | . | . | . | . | . | . | S | . | . | . | . | . | . |
| 21-225_7E11.001.011_HC | . | . | . | . | . | . | . | . | . | . | . | . | . | . | . | . | . | . | S | . | . | . | . | . | . |
| 21-225_7E11.001.012_HC | . | . | . | . | . | . | . | . | . | . | . | . | . | . | . | . | . | . | S | . | . | . | . | . | . |
| 21-225_7E11.001.013_HC | . | . | . | . | . | . | . | . | . | . | . | . | . | . | . | . | . | . | S | . | . | . | . | . | . |
| 21-225_7E11.001.014_HC | . | . | . | . | . | . | . | . | . | . | . | . | . | . | . | . | . | . | S | . | . | . | . | . | . |
| 21-225_7E11.001.015_HC | . | . | . | . | . | . | . | . | . | . | . | . | . | . | . | . | . | . | S | . | . | . | . | . | . |
| 21-225_7E11.001.016_HC | . | . | . | . | . | . | . | . | . | . | . | . | . | . | . | . | . | . | S | . | . | . | . | . | . |
| 21-225_7E11.001.017_HC | . | . | . | . | . | . | . | . | . | . | . | . | . | . | . | . | . | . | S | . | . | . | . | . | . |
| 21-225_7E11.001.018_HC | . | . | . | . | . | . | . | . | . | . | . | . | . | . | . | . | . | . | S | . | . | . | . | . | . |
| 21-225_7E11.001.023_HC | . | . | . | . | . | . | . | . | . | . | . | . | . | . | . | . | . | . | S | . | . | . | . | . | . |

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | H CDR3 | | | |
| CONSENSUS | T | A | V | Y | Y | C | A | R | D | L | S | M | G | - | - | - | - | - | - | - | - | - | - | - | - |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | . | . | . | . | . | . | . | . | . | . | . |
| 21-225_171E12_HC | . | . | . | . | . | . | . | . | . | R | T | Y | Y | S | G | S | G | S | P | . | . | . | . | . | . |
| 21-225_179H5_HC | . | . | . | . | . | . | . | . | . | I | R | N | Y | Y | . | . | . | . | . | . | . | . | . | . | . |
| 21-225_18G4_HC | . | . | . | . | . | . | . | . | . | . | . | . | . | . | . | . | . | . | . | . | . | . | . | . | . |
| 21-225_190A5_HC | . | . | . | . | . | . | . | . | . | F | . | V | . | Y | . | . | . | . | . | . | . | . | . | . | . |
| 21-225_190D10_HC | . | . | . | . | . | . | . | . | T | Y | . | G | - | . | . | . | . | . | . | . | . | . | . | . | . |
| 21-225_190F12_HC | . | . | . | . | . | . | . | . | . | R | . | V | . | Y | . | . | . | . | . | . | . | . | . | . | . |
| 21-225_190H7_HC | . | . | . | . | . | . | . | . | T | Y | . | G | - | . | . | . | . | . | . | . | . | . | . | . | . |
| 21-225_190H9_HC | . | . | . | . | . | . | . | . | . | R | . | V | . | Y | . | . | . | . | . | . | . | . | . | . | . |
| 21-225_192C10_HC | . | . | . | . | . | . | . | . | . | R | . | V | . | Y | . | . | . | . | . | . | . | . | . | . | . |
| 21-225_192E4_HC | . | . | . | . | . | . | . | . | . | R | . | V | . | Y | . | . | . | . | . | . | . | . | . | . | . |
| 21-225_192H6_HC | . | . | . | . | . | . | . | . | . | P | . | V | . | Y | . | . | . | . | . | . | . | . | . | . | . |
| 21-225_194B7_HC | . | . | . | . | . | . | . | . | . | R | . | K | . | Y | . | . | . | . | . | . | . | . | . | . | . |
| 21-225_194H12_HC | . | . | . | . | . | . | . | . | . | R | . | V | . | Y | . | . | . | . | . | . | . | . | . | . | . |
| 21-225_196D4_HC | . | . | . | . | . | . | . | . | . | R | . | K | . | Y | . | . | . | . | . | . | . | . | . | . | . |
| 21-225_197E11_HC | . | . | . | . | . | . | . | . | . | R | . | V | . | Y | . | . | . | . | . | . | . | . | . | . | . |
| 21-225_198D2_HC | . | . | . | . | . | . | . | . | T | Y | . | G | - | . | . | . | . | . | . | . | . | . | . | . | . |
| 21-225_205H3_HC | . | . | . | . | . | . | . | . | . | R | . | V | . | Y | . | . | . | . | . | . | . | . | . | . | . |
| 21-225_20F6_HC | . | . | . | . | . | . | . | . | . | . | . | . | . | . | . | . | . | . | . | . | . | . | . | . | . |
| 21-225_22C7_HC | . | . | . | . | . | . | . | . | . | . | . | . | . | . | . | . | . | . | . | . | . | . | . | . | . |
| 21-225_25B3_HC | . | . | . | . | . | . | . | . | . | S | . | P | Y | . | . | . | . | . | . | . | . | . | . | . | . |
| 21-225_26D12_HC | . | . | . | . | . | . | . | . | . | S | . | P | Y | . | . | . | . | . | . | . | . | . | . | . | . |
| 21-225_26G11_HC | . | . | . | . | . | . | . | . | R | Y | . | N | S | W | . | . | . | . | . | . | . | . | . | . | . |
| 21-225_27A11_HC | . | . | . | . | H | . | . | . | . | S | . | P | Y | . | . | . | . | . | . | . | . | . | . | . | . |
| 21-225_3F8_HC | . | . | . | . | . | . | . | . | . | . | . | . | . | . | . | . | . | . | . | . | . | . | . | . | . |
| 21-225_6G1_HC | . | . | M | . | . | . | . | . | . | . | . | . | . | . | . | . | . | . | . | . | . | . | . | . | . |
| 21-225_7E11_HC | . | . | . | . | . | . | . | . | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | D | L | S | M | G | - | - | - | - | - | - | - | - | - | - | - | - |
| 21-225_7E11.001_HC | . | . | . | . | . | . | . | . | . | . | . | . | . | . | . | . | . | . | . | . | . | . | . | . | . |
| 21-225_7E11.001.001_HC | . | . | . | . | . | . | . | . | . | . | . | . | . | . | . | . | . | . | . | . | . | . | . | . | . |
| 21-225_7E11.001.002_HC | . | . | . | . | . | . | . | . | . | . | . | . | . | . | . | . | . | . | . | . | . | . | . | . | . |
| 21-225_7E11.001.003_HC | . | . | . | . | . | . | . | . | . | . | . | . | . | . | . | . | . | . | . | . | . | . | . | . | . |
| 21-225_7E11.001.004_HC | . | . | . | . | . | . | . | . | . | . | . | . | . | . | . | . | . | . | . | . | . | . | . | . | . |
| 21-225_7E11.001.005_HC | . | . | . | . | . | . | . | . | . | . | . | . | . | . | . | . | . | . | . | . | . | . | . | . | . |
| 21-225_7E11.001.006_HC | . | . | . | . | . | . | . | . | . | . | . | . | . | . | . | . | . | . | . | . | . | . | . | . | . |
| 21-225_7E11.001.007_HC | . | . | . | . | . | . | . | . | . | . | . | . | . | . | . | . | . | . | . | . | . | . | . | . | . |
| 21-225_7E11.001.008_HC | . | . | . | . | . | . | . | . | . | . | . | . | . | . | . | . | . | . | . | . | . | . | . | . | . |
| 21-225_7E11.001.009_HC | . | . | . | . | . | . | . | . | . | . | . | . | . | . | . | . | . | . | . | . | . | . | . | . | . |
| 21-225_7E11.001.010_HC | . | . | . | . | . | . | . | . | . | . | . | . | . | . | . | . | . | . | . | . | . | . | . | . | . |
| 21-225_7E11.001.011_HC | . | . | . | . | . | . | . | . | . | . | . | . | . | . | . | . | . | . | . | . | . | . | . | . | . |
| 21-225_7E11.001.012_HC | . | . | . | . | . | . | . | . | . | . | . | . | . | . | . | . | . | . | . | . | . | . | . | . | . |
| 21-225_7E11.001.013_HC | . | . | . | . | . | . | . | . | . | . | . | . | . | . | . | . | . | . | . | . | . | . | . | . | . |
| 21-225_7E11.001.014_HC | . | . | . | . | . | . | . | . | . | . | . | . | . | . | . | . | . | . | . | . | . | . | . | . | . |
| 21-225_7E11.001.015_HC | . | . | . | . | . | . | . | . | . | . | . | . | . | . | . | . | . | . | . | . | . | . | . | . | . |
| 21-225_7E11.001.016_HC | . | . | . | . | . | . | . | . | . | . | . | . | . | . | . | . | . | . | . | . | . | . | . | . | . |
| 21-225_7E11.001.017_HC | . | . | . | . | . | . | . | . | . | . | . | . | . | . | . | . | . | . | . | . | . | . | . | . | . |
| 21-225_7E11.001.018_HC | . | . | . | . | . | . | . | . | . | . | . | . | . | . | . | . | . | . | . | . | . | . | . | . | . |
| 21-225_7E11.001.023_HC | . | . | . | . | . | . | . | . | . | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | H_FR4 | | | | | | |
| CONSENSUS | - | - | - | - | - | - | - | - | - | G | M | D | V | W | G | Q | G | T | T | V | T | V | S | S |
| 21-225_13D4_HC | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | . | . | . | . | . | . | . | . | . | . |
| 21-225_171E12_HC | . | . | . | P | Y | Y | Y | Y | Y | . | . | . | . | . | . | . | . | . | . | . | . | . | . | . |
| 21-225_179H5_HC | . | . | . | . | . | . | . | . | Y | . | L | . | . | . | . | . | . | . | . | . | . | . | . | . |
| 21-225_18G4_HC | . | . | . | . | . | . | . | . | . | . | . | . | . | . | . | . | . | . | . | . | . | . | . | . |
| 21-225_190A5_HC | . | . | . | . | . | . | . | . | D | . | . | . | . | . | . | . | . | . | . | . | . | . | . | . |
| 21-225_190D10_HC | . | . | . | . | . | . | . | . | . | . | . | . | . | . | . | . | . | . | . | . | . | . | . | . |
| 21-225_190F12_HC | . | . | . | . | . | . | . | . | D | . | L | . | . | . | . | . | . | . | . | . | . | . | . | . |
| 21-225_190H7_HC | . | . | . | . | . | . | . | . | . | . | . | . | . | . | . | . | . | . | . | . | . | . | . | . |
| 21-225_190H9_HC | . | . | . | . | . | . | . | . | D | . | L | . | . | . | . | . | . | . | . | . | . | . | . | . |
| 21-225_192C10_HC | . | . | . | . | . | . | . | . | D | . | L | . | . | . | . | . | . | . | . | . | . | . | . | . |
| 21-225_192E4_HC | . | . | . | . | . | . | . | . | D | . | . | . | . | . | . | . | . | . | . | . | . | . | . | . |
| 21-225_192H6_HC | . | . | . | . | . | . | . | . | D | . | . | . | . | . | . | . | . | . | . | . | . | . | . | . |
| 21-225_194B7_HC | . | . | . | . | . | . | . | . | D | . | . | . | . | . | . | . | . | . | . | . | . | . | . | . |
| 21-225_194H12_HC | . | . | . | . | . | . | . | . | D | . | L | . | . | . | . | . | . | . | . | . | . | . | . | . |
| 21-225_196D4_HC | . | . | . | . | . | . | . | . | D | . | . | . | . | . | . | . | . | . | . | . | . | . | . | . |
| 21-225_197E11_HC | . | . | . | . | . | . | . | . | D | . | L | . | . | . | . | . | . | . | . | . | . | . | . | . |
| 21-225_198D2_HC | . | . | . | . | . | . | . | . | . | . | . | . | . | . | . | . | . | . | . | . | . | . | . | . |
| 21-225_205H3_HC | . | . | . | . | . | . | . | . | D | . | T | . | . | . | . | . | . | . | . | . | . | . | . | . |
| 21-225_20F6_HC | . | . | . | . | . | . | . | . | . | . | . | . | . | . | . | . | . | . | . | . | . | . | . | . |
| 21-225_22C7_HC | . | . | . | . | . | . | . | . | . | . | . | . | . | . | . | . | . | . | . | . | . | . | . | . |
| 21-225_25B3_HC | . | . | . | . | . | . | . | . | . | . | . | . | . | . | . | . | . | . | . | . | . | . | . | . |
| 21-225_26D12_HC | . | . | . | . | . | . | . | . | . | . | . | . | . | . | . | . | . | . | . | . | . | . | . | . |
| 21-225_26G11_HC | . | . | . | . | . | . | . | S | G | . | . | . | . | . | . | . | . | . | . | . | . | . | . | . |
| 21-225_27A11_HC | . | . | . | . | . | . | . | . | . | . | . | . | . | . | . | . | . | . | . | . | . | . | . | . |
| 21-225_3F8_HC | . | . | . | . | . | . | . | . | . | . | . | . | . | . | . | . | . | . | . | . | . | . | . | . |
| 21-225_6G1_HC | . | . | . | . | . | . | . | . | . | . | . | . | . | . | . | . | . | . | . | . | . | . | . | . |
| 21-225_7E11_HC | . | . | . | . | . | . | . | . | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | | H_FR4 | | | | | |
| CONSENSUS | - | - | - | - | - | - | - | - | - | G | M | D | V | W | G | Q | G | T | T | V | T | V | S | S |
| 21-225_7E11.001_HC | . | . | . | . | . | . | . | . | . | . | . | . | . | . | . | . | . | . | . | . | . | . | . | . |
| 21-225_7E11.001.001_HC | . | . | . | . | . | . | . | . | . | . | . | . | . | . | . | . | . | . | . | . | . | . | . | . |
| 21-225_7E11.001.002_HC | . | . | . | . | . | . | . | . | . | . | . | . | . | . | . | . | . | . | . | . | . | . | . | . |
| 21-225_7E11.001.003_HC | . | . | . | . | . | . | . | . | . | . | . | . | . | . | . | . | . | . | . | . | . | . | . | . |
| 21-225_7E11.001.004_HC | . | . | . | . | . | . | . | . | . | . | . | . | . | . | . | . | . | . | . | . | . | . | . | . |
| 21-225_7E11.001.005_HC | . | . | . | . | . | . | . | . | . | . | . | . | . | . | . | . | . | . | . | . | . | . | . | . |
| 21-225_7E11.001.006_HC | . | . | . | . | . | . | . | . | . | . | . | . | . | . | . | . | . | . | . | . | . | . | . | . |
| 21-225_7E11.001.007_HC | . | . | . | . | . | . | . | . | . | . | . | . | . | . | . | . | . | . | . | . | . | . | . | . |
| 21-225_7E11.001.008_HC | . | . | . | . | . | . | . | . | . | . | . | . | . | . | . | . | . | . | . | . | . | . | . | . |
| 21-225_7E11.001.009_HC | . | . | . | . | . | . | . | . | . | . | . | . | . | . | . | . | . | . | . | . | . | . | . | . |
| 21-225_7E11.001.010_HC | . | . | . | . | . | . | . | . | . | . | . | . | . | . | . | . | . | . | . | . | . | . | . | . |
| 21-225_7E11.001.011_HC | . | . | . | . | . | . | . | . | . | . | . | . | . | . | . | . | . | . | . | . | . | . | . | . |
| 21-225_7E11.001.012_HC | . | . | . | . | . | . | . | . | . | . | . | . | . | . | . | . | . | . | . | . | . | . | . | . |
| 21-225_7E11.001.013_HC | . | . | . | . | . | . | . | . | . | . | . | . | . | . | . | . | . | . | . | . | . | . | . | . |
| 21-225_7E11.001.014_HC | . | . | . | . | . | . | . | . | . | . | . | . | . | . | . | . | . | . | . | . | . | . | . | . |
| 21-225_7E11.001.015_HC | . | . | . | . | . | . | . | . | . | . | . | . | . | . | . | . | . | . | . | . | . | . | . | . |
| 21-225_7E11.001.016_HC | . | . | . | . | . | . | . | . | . | . | . | . | . | . | . | . | . | . | . | . | . | . | . | . |
| 21-225_7E11.001.017_HC | . | . | . | . | . | . | . | . | . | . | . | . | . | . | . | . | . | . | . | . | . | . | . | . |
| 21-225_7E11.001.018_HC | . | . | . | . | . | . | . | . | . | . | . | . | . | . | . | . | . | . | . | . | . | . | . | . |
| 21-225_7E11.001.023_HC | . | . | . | . | . | . | . | . | . | . | . | . | . | . | . | . | . | . | . | . | . | . | . | . |

Table 57. Consensus 23- VH3|3-33/D7|7-27|RF1/JH4 (SEQ ID NO: 50274):

QVQLVES-GGGVVQPGRSLRLSCAASG-FTFSD-----YGMHWVRQAPGKGLEWVAVIWYD---
ESNKYYADSVKGRFTISRDNSKNTLYLQMNSLRAEDTAVYYCAREVGW--------------------LDDYWGQGTLVTVSS wherein:

FIGURE 57

D at position 33 can be substituted with N or S

Y at position 39 can be substituted with F

M at position 41 can be substituted with I or L

Y at position 60 can be substituted with F

D at position 61 can be substituted with E, I or V

E at position 65 can be substituted with G, V, A or R

S at position 66 can be substituted with N, T or G

K at position 68 can be substituted with Q, T or N

Y at position 69 can be substituted with H or K

A at position 71 can be substituted with T, G, E or V

D at position 72 can be substituted with G

V at position 74 can be substituted with A

V at position 110 can be substituted with I, M, K or T

W at position 112 can be substituted with F, G or M

L at position 135 can be substituted with T, S, Y, H or R

D at position 136 can be substituted with E, S, F or N

Y at position 138 can be substituted with C

Xaa Xaa Gly Xaa His (SEQ ID NO: 50025)

Val Ile Trp Xaa Xaa Xaa Xaa Asn Xaa Xaa Tyr Xaa Xaa Ser Xaa Lys Gly (SEQ ID NO: 50026)

**FIGURE 57**

Glu Xaa Gly Xaa Xaa Xaa Asp Xaa (SEQ ID NO: 50027)

Asp Tyr Gly Met His (SEQ ID NO: 50492)

Val Ile Trp Tyr Asp Glu Ser Asn Lys Tyr Tyr Ala Asp Ser Val Lys Gly (SEQ ID NO: 50493)

Glu Val Gly Trp Leu Asp Asp Tyr (SEQ ID NO: 50494)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_149F12_HC | | | | | | | | | | | | | | | | | | | | | | | | | | |
| 21-225_149G1_HC | | | | | | | | | | | | | | | | | | | | | | | | | | |
| 21-225_14C2_HC | | | | | | | | | | | | | | | | | | | | | | | | | | |
| 21-225_150E7_HC | | | | | | | | | | | | | | | | | | | | | | | | | | |
| 21-225_15B11_HC | | | | | | | | | | | | | | | | | | | | | | | | | | |
| 21-225_15H10_HC | | | | | | | | | | | | | | | | | | | | | | | | | | |
| 21-225_161D11_HC | | | | | | | | | | | | | | | | | | | | | | | | | | |
| 21-225_161F7_HC | | | | | | | | | | | | | | | | | | | | | | | | | | |
| 21-225_16H4_HC | | | | | | | | | | | | | | | | | | | | | | | | | | |
| 21-225_180H7_HC | | | | | | | | | | | | | | | | | | | | | | | | | | |
| 21-225_181A8_HC | | | | | | | | | | | | | | | | | | | | | | | | | | |
| 21-225_184D11_HC | | | | | | | | | | | | | | | | | | | | | | | | | | |
| 21-225_184H10_HC | | | | | | | | | | | | | | | | | | | | | | | | | V | |
| 21-225_18H12_HC | | | | | | | | | | | | | | | | | | | | | | | | | | |
| 21-225_203C10_HC | | | | | | | | | | | | | | | | | | | | | | | | | | |
| 21-225_210E4_HC | | | | | | | | | | | | | | | | | | | | | | | | | | |
| 21-225_225B6_HC | | | | | | | | | | | | | | | | | | | | | | | | | | |
| 21-225_225D6_HC | | | | | | | | | | | | | | | | | | | | | | | | | | |
| 21-225_225F8_HC | | | | | | | | | | | | | | | | | | | | | | | | | | |
| 21-225_226A9_HC | | | | | | | | | | | | | | | | | | | | | | | | | | |
| 21-225_227C12_HC | | | | | | | | | | | | | | | | | | | | | | | | | | |
| 21-225_227G3_HC | | | | | | | | | | | | | | | | | | | | | | | | | | |
| 21-225_227H5_HC | | | | | | | | | | | | | | | | | | | | | | | | | | |
| 21-225_22B7_HC | | | | | | | | | | | | | | | | | | | | | | | | | | |
| 21-225_25A9_HC | | | | | | | | | | | | | | | | | | | | | | | | | | |
| 21-225_25G5_HC | | | | | | | | | | | | | | | | | | | | | | | | | | |
| 21-225_28A9_HC | | | | | | | | | | | | | | | | | | | | | | | | | | |
| 21-225_28B8_HC | | | | | | | | | | | | | | | | | | | | | | | | | | |
| 21-225_29D8_HC | | | | | | | | | | | | | | | | | | | | | | | | | | |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_29D9_HC | . | . | . | . | . | . | . | . | . | . | . | . | . | . | . | . | . | . | . | × | . | . | . | S | . | . |
| 21-225_30F3_HC | . | . | . | . | . | . | . | . | . | . | . | . | . | . | . | . | . | . | . | . | . | . | . | . | . | . |
| 21-225_33B1_HC | . | . | . | . | . | . | . | . | . | . | . | . | . | . | . | . | . | . | . | . | . | . | . | . | . | . |
| 21-225_4C8_HC | . | . | . | . | . | . | . | . | . | . | . | . | . | . | . | . | . | . | . | . | . | . | . | . | . | . |
| 21-225_4F12_HC | . | . | . | . | . | . | . | . | . | . | . | . | . | . | . | . | . | . | . | . | . | . | . | . | . | . |
| 21-225_50C4_HC | . | . | . | . | . | . | . | . | . | . | . | . | . | . | . | . | . | . | . | . | . | . | . | . | . | . |
| 21-225_73G6_HC | . | . | . | . | . | . | . | . | . | . | . | . | . | . | . | . | . | . | . | . | . | . | . | . | . | . |
| 21-225_90H5_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | H_CDR1 | | | | | | | | | | | | | | H_FR3 | | |
| CONSENSUS | G | - | F | T | F | S | D | - | - | - | - | - | Y | G | M | H | W | V | R | Q | A | P | G | K | G | L |
| 21-225_149F12_HC | . | . | . | . | . | . | . | . | . | . | . | . | . | . | . | . | . | . | . | . | . | . | . | . | . | . |
| 21-225_149G1_HC | . | . | . | . | . | . | . | . | . | . | . | . | . | . | . | . | . | . | . | . | . | . | . | . | . | . |
| 21-225_14C2_HC | . | . | . | . | . | . | . | . | . | . | . | . | . | . | . | . | . | . | . | . | . | . | . | . | . | . |
| 21-225_150E7_HC | . | . | . | . | . | . | . | . | . | . | . | . | . | . | . | . | . | . | . | . | . | . | . | . | . | . |
| 21-225_15B11_HC | . | . | . | . | . | . | N | . | . | . | . | . | . | . | . | . | . | . | . | . | . | . | . | . | . | . |
| 21-225_15H10_HC | . | . | . | . | . | . | N | . | . | . | . | . | . | . | . | . | . | . | . | . | . | . | . | . | . | . |
| 21-225_161D11_HC | . | . | . | . | . | . | . | . | . | . | . | . | P | . | L | . | . | . | . | . | . | . | Q | . | . | . |
| 21-225_161F7_HC | . | . | . | . | . | . | . | . | . | . | . | . | P | . | L | . | . | . | . | . | . | . | Q | . | . | . |
| 21-225_16H4_HC | . | . | . | . | . | . | N | . | . | . | . | . | . | . | . | . | . | . | . | . | . | . | . | . | . | . |
| 21-225_180H7_HC | . | . | . | . | . | . | . | . | . | . | . | . | . | . | . | . | . | . | . | . | . | . | . | . | . | . |
| 21-225_181A8_HC | . | . | . | . | . | . | . | . | . | . | . | . | . | . | . | . | . | . | . | . | . | . | . | . | . | . |
| 21-225_184D11_HC | . | . | . | . | . | . | . | . | . | . | . | . | . | . | . | . | . | . | . | . | . | . | . | . | . | . |
| 21-225_184H10_HC | . | . | . | . | . | . | . | . | . | . | . | . | . | . | . | . | . | . | . | . | . | . | . | . | . | . |
| 21-225_18H12_HC | . | . | . | . | . | . | . | . | . | . | . | . | . | . | . | . | . | . | . | . | . | . | . | . | . | . |
| 21-225_203C10_HC | . | . | . | . | . | . | . | . | . | . | . | . | . | . | . | . | . | . | . | . | . | . | . | . | . | . |
| 21-225_210E4_HC | . | . | . | . | L | i | N | . | . | . | . | . | . | . | . | . | . | . | . | . | . | . | . | . | . | . |
| 21-225_225B6_HC | . | . | . | . | . | . | . | . | . | . | . | . | . | . | . | . | . | . | . | . | . | . | . | . | . | . |
| 21-225_225D6_HC | . | . | . | . | . | . | N | . | . | . | . | . | . | . | . | . | . | . | . | . | . | . | . | . | . | . |
| 21-225_225F8_HC | . | . | . | . | . | . | S | . | . | . | . | . | . | . | . | . | . | . | . | . | . | . | . | . | . | . |
| 21-225_226A9_HC | . | . | . | . | . | . | N | . | . | . | . | . | . | . | . | . | . | . | . | . | . | . | . | . | . | . |
| 21-225_227C12_HC | . | . | . | . | . | . | N | . | . | . | . | . | . | . | . | . | . | . | . | . | . | . | . | . | . | . |
| 21-225_227G3_HC | . | . | . | . | . | . | S | . | . | . | . | . | . | . | . | . | . | . | . | . | . | . | . | . | . | . |
| 21-225_227H5_HC | . | . | . | . | . | . | N | . | . | . | . | . | . | . | . | . | . | . | . | . | . | . | . | . | . | . |
| 21-225_22B7_HC | . | . | . | . | . | . | S | . | . | . | . | . | . | . | . | . | . | . | . | . | . | . | . | . | . | . |
| 21-225_25A9_HC | . | . | . | . | . | . | S | . | . | . | . | . | . | . | . | . | . | . | . | . | . | . | . | . | . | . |
| 21-225_25G5_HC | . | . | . | . | . | . | . | . | . | . | . | . | . | . | I | . | . | . | . | . | . | . | . | . | . | . |
| 21-225_28A9_HC | . | . | . | . | . | . | S | . | . | . | . | . | . | . | I | . | . | . | . | . | . | . | . | . | . | . |
| 21-225_28B8_HC | . | . | . | . | . | . | . | . | . | . | . | . | . | . | . | . | . | . | . | . | . | . | . | . | . | . |
| 21-225_29D8_HC | . | . | . | . | . | R | . | . | . | . | . | . | . | . | I | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | F | T | F | S | D | - | - | - | - | - | Y | G | M | H | W | V | R | Q | A | P | G | K | G | L |
| 21-225_29D9_HC | . | . | . | . | . | . | . | . | . | . | . | . | . | . | ! | . | . | . | . | . | . | . | . | . | . | . |
| 21-225_30F3_HC | . | . | . | . | . | . | S | . | . | . | . | . | . | . | . | . | . | . | . | . | . | . | . | . | . | . |
| 21-225_33B1_HC | . | . | . | . | . | . | . | . | . | . | . | . | . | . | . | . | . | . | . | . | . | . | A | . | . | . |
| 21-225_4C8_HC | . | . | . | . | . | . | N | . | . | . | . | . | . | . | . | . | . | . | . | . | . | . | . | . | . | . |
| 21-225_4F12_HC | . | . | . | . | . | . | S | . | . | . | . | . | . | . | . | . | . | . | . | . | . | . | . | . | . | . |
| 21-225_50C4_HC | . | . | . | . | . | . | . | . | . | . | . | . | . | . | . | . | . | . | . | . | . | . | . | . | . | . |
| 21-225_73G6_HC | . | . | . | . | . | . | S | . | . | . | . | . | . | . | . | . | . | . | . | . | . | . | . | . | . | . |
| 21-225_90H5_HC | . | . | . | . | . | . | . | . | . | . | . | . | . | . | . | . | . | . | . | . | S | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | |
| CONSENSUS | E | W | V | A | V | I | W | Y | D | - | - | - | E | S | N | K | Y | Y | A | D | S | V | K | G | R | F |
| 21-225_149F12_HC | | | | | | | | | E | | | | | N | | | | | | | | | | | | |
| 21-225_149G1_HC | | | | | | | | | E | | | | G | | | | | | | | | | | | | |
| 21-225_14C2_HC | | | | | | | | | | | | | | | | | | | E | | | | | | | |
| 21-225_150E7_HC | | | | | | | | | E | | | | | N | | | | | | | | | | | | |
| 21-225_15B11_HC | | | | | | | | | E | | | | | | | | | | T | | | | | | | |
| 21-225_15H10_HC | | | | | | | | | | | | | | | | | | | E | | | | | | | |
| 21-225_161D11_HC | | | | | | | | F | | | | | G | | | | | | | | | | | | | |
| 21-225_161F7_HC | | | | | | | | F | | | | | G | | | | | | | | | | | | | |
| 21-225_16H4_HC | | | | | | | | | | | | | V | | | | | | | | | | | | | |
| 21-225_180H7_HC | | | | | | | | | | | | | | N | | H | | | | | | A | | | | |
| 21-225_181A8_HC | | | | | | | | | | | | | | N | | H | | | | | | A | | | | |
| 21-225_184D11_HC | | | | | | | | | | | | | | T | | H | | | G | | | | | | | |
| 21-225_184H10_HC | | | | | | | | | | | | | | T | | H | | | G | | | | | | | |
| 21-225_18H12_HC | | | M | | | | | | | | | | V | T | | | | | | | | | | | | |
| 21-225_203C10_HC | | | | | | | | | | | | | Q | S | | T | H | | T | | | | | | | |
| 21-225_210E4_HC | | | | | | | | | | | | | | N | | | | | V | | | | | | | |
| 21-225_225B6_HC | | | | | | | | | | | | | | N | | | | | | | | | | | | |
| 21-225_225D6_HC | | | | | | | | | | | | | | N | | | | | | | | | | | | |
| 21-225_225F8_HC | | | | | | | | | | | | | | N | | | | | V | | | | | | | |
| 21-225_226A9_HC | | | | | | | | E | | | | | | | | | | | T | | | | | | | |
| 21-225_227C12_HC | | | | | | | | I | | | | | G | | | Q | | | | | | | | | | |
| 21-225_227G3_HC | | | | | | | | V | | | | | G | | | Q | | | | | | | | | | |
| 21-225_227H5_HC | D | | | | | | | E | | | | | | | | | | | | | | | | | | |
| 21-225_22B7_HC | | | | | | | | E | | | | | | N | | Q | | | | | | | | | | |
| 21-225_25A9_HC | | | | | | | | | | | | | V | T | | | | | T | G | | | | | | |
| 21-225_25G5_HC | | | | | | | | | | | | | G | N | | | | | | | | | | | | |
| 21-225_28A9_HC | | | | | | | | F | | | | | G | | | | | | | | | | | | | |
| 21-225_28B8_HC | | | | T | | | | | | | | | A | N | | | | | | | | | | | | |
| 21-225_29D8_HC | | | | | | | | F | | | | | G | T | | | | | | | | | | | | |

3843

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | E | S | N | K | Y | Y | A | D | S | V | K | G | R | F |
| 21-225_29D9_HC | . | . | . | . | . | . | . | . | . | . | . | . | . | . | . | N | . | . | . | . | . | . | . | . | . | . |
| 21-225_30F3_HC | . | . | . | . | . | . | . | . | . | . | . | . | V | . | . | . | . | . | . | . | . | . | . | . | . | . |
| 21-225_33B1_HC | . | . | . | . | . | . | . | . | . | . | . | . | R | N | . | . | . | . | . | . | . | . | . | . | . | . |
| 21-225_4C8_HC | . | . | . | . | . | . | . | . | E | . | . | . | . | N | . | Q | . | . | . | . | . | . | . | . | . | . |
| 21-225_4F12_HC | . | . | . | . | . | . | . | . | . | . | . | . | V | T | . | . | X | . | . | . | . | . | . | . | . | . |
| 21-225_50C4_HC | . | . | . | . | . | . | . | . | E | . | . | . | . | T | . | . | . | . | . | . | . | . | . | . | . | . |
| 21-225_73G6_HC | . | . | . | . | . | . | . | . | . | . | . | . | V | . | . | . | . | . | @ | . | . | . | . | . | . | . |
| 21-225_90H5_HC | . | . | . | . | . | . | . | . | E | . | . | . | G | . | T | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FRS | | | | | | | | | | | | |
| CONSENSUS | T | I | S | R | D | N | S | K | N | T | L | Y | L | Q | M | N | S | L | R | A | E | D | T | A | V | Y |
| 21-225_149F12_HC | . | . | . | . | . | . | . | . | . | . | . | P | . | . | . | . | . | . | . | . | . | . | . | . | . | . |
| 21-225_149G1_HC | . | . | . | . | . | . | . | . | M | . | . | . | . | . | . | . | . | . | . | . | . | . | . | . | . | . |
| 21-225_14C2_HC | . | . | . | . | . | . | . | . | . | . | . | . | . | R | . | . | . | . | . | . | . | . | . | . | G | . |
| 21-225_150E7_HC | . | . | . | . | . | . | . | . | . | . | . | P | . | . | . | . | . | . | . | . | . | . | . | . | . | . |
| 21-225_15B11_HC | . | . | . | . | . | . | . | . | . | . | . | . | . | . | . | . | . | . | . | P | . | . | . | . | . | . |
| 21-225_15H10_HC | . | . | . | . | . | . | . | . | . | . | . | . | . | . | . | . | . | . | . | . | . | . | . | . | . | . |
| 21-225_161D11_HC | . | . | P | . | . | . | . | . | . | . | . | . | . | . | . | . | . | . | . | . | . | . | . | . | . | . |
| 21-225_161F7_HC | . | . | P | . | . | . | . | . | . | . | . | . | . | . | . | . | . | . | . | . | . | . | . | . | . | . |
| 21-225_16H4_HC | . | . | . | . | . | . | . | . | M | . | . | . | . | . | . | . | . | . | . | . | . | . | . | . | . | . |
| 21-225_180H7_HC | . | . | . | . | . | . | . | . | . | . | . | . | . | . | . | . | . | . | . | V | . | . | . | . | . | . |
| 21-225_181A8_HC | . | . | . | . | . | . | . | . | . | . | . | . | . | . | . | . | . | . | . | . | . | . | . | . | . | . |
| 21-225_184D11_HC | . | . | . | . | . | . | . | D | . | . | . | . | . | . | . | . | . | . | . | . | . | . | . | . | . | . |
| 21-225_184H10_HC | . | . | . | . | . | . | . | D | . | . | . | . | . | . | . | . | . | . | . | . | . | . | . | . | . | . |
| 21-225_18H12_HC | . | . | . | . | . | . | . | . | . | . | . | . | . | . | . | . | . | . | . | . | . | . | . | . | . | . |
| 21-225_203C10_HC | . | . | . | . | . | . | . | . | . | . | . | . | . | . | . | . | . | . | . | . | . | . | . | . | . | . |
| 21-225_210E4_HC | . | . | . | . | . | D | . | . | . | . | . | . | . | . | . | . | . | . | . | . | . | . | . | S | . | . |
| 21-225_225B6_HC | . | . | . | . | . | . | . | . | . | . | . | . | . | . | . | . | . | . | . | . | . | . | . | . | . | . |
| 21-225_225D6_HC | . | . | . | . | . | . | . | Q | . | . | . | . | . | . | . | T | . | . | . | . | . | . | . | . | . | . |
| 21-225_225F8_HC | . | . | . | . | . | . | . | . | . | . | . | . | . | . | . | . | . | . | . | . | . | . | . | . | . | . |
| 21-225_226A9_HC | . | . | . | . | . | . | . | . | . | . | . | P | . | . | . | . | . | . | . | . | . | . | . | . | . | . |
| 21-225_227C12_HC | . | . | . | . | . | . | . | . | . | . | . | . | . | . | . | S | . | . | . | . | . | . | . | . | . | . |
| 21-225_227G3_HC | . | . | . | . | . | . | . | . | . | . | . | . | . | . | . | S | . | . | . | . | . | . | . | . | . | . |
| 21-225_227H5_HC | . | . | . | . | . | . | . | . | . | . | . | . | . | . | . | . | . | . | . | . | . | . | . | . | M | . |
| 21-225_22B7_HC | . | . | . | . | . | . | . | . | . | . | . | . | . | . | . | . | . | . | . | . | . | . | . | . | . | . |
| 21-225_25A9_HC | . | . | . | . | . | . | . | . | . | . | . | . | . | . | . | . | . | . | . | . | . | . | . | . | . | . |
| 21-225_25G5_HC | . | . | . | . | . | . | . | . | . | . | . | . | . | . | . | N | . | . | . | . | . | . | . | . | . | . |
| 21-225_28A9_HC | . | . | . | . | . | . | . | . | . | . | . | P | . | . | . | . | . | . | . | V | . | . | . | . | . | . |
| 21-225_28B8_HC | . | . | . | . | . | P | . | . | . | V | . | . | . | . | . | . | . | . | . | . | . | . | . | . | . | . |
| 21-225_29D8_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | | |
| CONSENSUS | T | I | S | R | D | N | S | K | N | T | L | Y | L | Q | M | N | S | L | R | A | E | D | T | A | V | Y |
| 21-225_29D9_HC | . | . | . | . | . | . | . | . | . | . | . | . | . | . | . | . | . | . | . | . | . | . | . | . | . | . |
| 21-225_30F3_HC | . | . | . | . | . | . | . | . | . | . | . | . | . | . | . | . | . | . | . | . | . | . | . | . | . | . |
| 21-225_33B1_HC | . | . | . | . | . | . | . | . | . | . | . | . | . | . | . | . | . | . | Y | . | . | . | . | . | . | . |
| 21-225_4C8_HC | . | . | . | . | . | . | . | . | . | . | . | . | . | . | . | . | . | . | Y | . | . | . | . | . | . | . |
| 21-225_4F12_HC | . | . | . | . | . | . | . | . | . | . | . | . | . | . | . | . | . | . | Y | P | . | N | . | . | G | G | . |
| 21-225_50C4_HC | . | . | . | . | . | . | . | . | . | . | . | . | . | . | . | . | . | . | G | . | . | . | . | . | . | . |
| 21-225_73G6_HC | . | . | . | . | . | . | . | . | . | . | . | . | . | . | . | . | . | . | . | . | . | . | . | . | . | . |
| 21-225_90H5_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | V | G | W | - | - | - | - | - | - | - | - | - | - | - | - | - | - | - | - | - | - |
| 21-225_149F12_HC | . | . | . | . | . | I | . | F | . | . | . | . | . | . | . | . | . | . | . | . | . | . | . | . | . | . |
| 21-225_149G1_HC | . | . | . | . | . | I | . | F | . | . | . | . | . | . | . | . | . | . | . | . | . | . | . | . | . | . |
| 21-225_14C2_HC | . | . | . | . | . | . | . | F | . | . | . | . | . | . | . | . | . | . | . | . | . | . | . | . | . | . |
| 21-225_150E7_HC | . | . | . | . | . | I | . | F | . | . | . | . | . | . | . | . | . | . | . | . | . | . | . | . | . | . |
| 21-225_15B11_HC | . | . | . | . | . | . | . | F | . | . | . | . | . | . | . | . | . | . | . | . | . | . | . | . | . | . |
| 21-225_15H10_HC | . | . | V | . | . | . | . | F | . | . | . | . | . | . | . | . | . | . | . | . | . | . | . | . | . | . |
| 21-225_161D11_HC | . | . | . | . | . | I | . | . | . | . | . | . | . | . | . | . | . | . | . | . | . | . | . | . | . | . |
| 21-225_161F7_HC | . | . | . | . | . | I | . | . | . | . | . | . | . | . | . | . | . | . | . | . | . | . | . | . | . | . |
| 21-225_16H4_HC | . | . | . | . | . | M | . | . | . | . | . | . | . | . | . | . | . | . | . | . | . | . | . | . | . | . |
| 21-225_180H7_HC | . | . | . | . | . | . | . | . | . | . | . | . | . | . | . | . | . | . | . | . | . | . | . | . | . | . |
| 21-225_181A8_HC | . | . | . | . | . | . | . | . | . | . | . | . | . | . | . | . | . | . | . | . | . | . | . | . | . | . |
| 21-225_184D11_HC | . | . | . | . | . | . | . | . | . | . | . | . | . | . | . | . | . | . | . | . | . | . | . | . | . | . |
| 21-225_184H10_HC | . | . | . | . | . | . | . | . | . | . | . | . | . | . | . | . | . | . | . | . | . | . | . | . | . | . |
| 21-225_18H12_HC | . | . | . | . | . | . | . | . | . | . | . | . | . | . | . | . | . | . | . | . | . | . | . | . | . | . |
| 21-225_203C10_HC | . | . | . | . | . | . | . | . | . | . | . | . | . | . | . | . | . | . | . | . | . | . | . | . | . | . |
| 21-225_210E4_HC | . | . | . | . | . | T | . | F | . | . | . | . | . | . | . | . | . | . | . | . | . | . | . | . | . | . |
| 21-225_225B6_HC | . | . | . | . | . | . | . | F | . | . | . | . | . | . | . | . | . | . | . | . | . | . | . | . | . | . |
| 21-225_225D6_HC | . | . | . | . | . | . | . | F | . | . | . | . | . | . | . | . | . | . | . | . | . | . | . | . | . | . |
| 21-225_225F8_HC | . | . | . | . | . | . | . | F | . | . | . | . | . | . | . | . | . | . | . | . | . | . | . | . | . | . |
| 21-225_226A9_HC | . | . | . | . | . | . | . | F | . | . | . | . | . | . | . | . | . | . | . | . | . | . | . | . | . | . |
| 21-225_227C12_HC | . | . | . | . | . | . | . | F | . | . | . | . | . | . | . | . | . | . | . | . | . | . | . | . | . | . |
| 21-225_227G3_HC | . | . | . | . | . | . | . | F | . | . | . | . | . | . | . | . | . | . | . | . | . | . | . | . | . | . |
| 21-225_227H5_HC | . | . | . | . | . | . | . | F | . | . | . | . | . | . | . | . | . | . | . | . | . | . | . | . | . | . |
| 21-225_22B7_HC | . | . | . | . | . | . | . | F | . | . | . | . | . | . | . | . | . | . | . | . | . | . | . | . | . | . |
| 21-225_25A9_HC | . | . | . | . | . | I | . | . | . | . | . | . | . | . | . | . | . | . | . | . | . | . | . | . | . | . |
| 21-225_25G5_HC | . | . | . | . | . | I | . | . | . | . | . | . | . | . | . | . | . | . | . | . | . | . | . | . | . | . |
| 21-225_28A9_HC | . | . | . | . | . | I | . | . | . | . | . | . | . | . | . | . | . | . | . | . | . | . | . | . | . | . |
| 21-225_28B8_HC | . | . | . | . | . | I | . | . | . | . | . | . | . | . | . | . | . | . | . | . | . | . | . | . | . | . |
| 21-225_29D8_HC | . | . | Q | . | . | I | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | | H_CDR3 | | | | | | |
| CONSENSUS | Y | C | A | R | E | V | G | W | - | - | - | - | - | - | - | - | - | - | - | - | - | - | - | - | - | - |
| 21-225_29D9_HC | . | . | . | . | . | I | . | . | . | . | . | . | . | . | . | . | . | . | . | . | . | . | . | . | . | . |
| 21-225_30F3_HC | . | . | . | . | . | M | . | . | . | . | . | . | . | . | . | . | . | . | . | . | . | . | . | . | . | . |
| 21-225_33B1_HC | . | . | . | . | . | M | . | . | . | . | . | . | . | . | . | . | . | . | . | . | . | . | . | . | . | . |
| 21-225_4C8_HC | . | S | P | . | . | M | . | F | . | . | . | . | . | . | . | . | . | . | . | . | . | . | . | . | . | . |
| 21-225_4F12_HC | E | N | Q | . | . | H | . | Q | . | . | . | . | . | . | . | . | . | . | . | . | . | . | . | . | . | . |
| 21-225_50C4_HC | . | . | . | . | . | . | . | F | . | . | . | . | . | . | . | . | . | . | . | . | . | . | . | . | . | . |
| 21-225_73G6_HC | . | . | . | . | . | . | . | M | . | . | . | . | . | . | . | . | . | . | . | . | . | . | . | . | . | . |
| 21-225_90H5_HC | . | . | V | . | . | M | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | L | D | D | Y | W | G | Q | G | T | L | V | T | V | S | S |
| 21-225_149F12_HC | . | . | . | . | S | E | . | . | . | . | . | . | . | . | . | . | . | . | . |
| 21-225_149G1_HC | . | . | . | . | S | E | . | . | . | . | . | . | . | . | . | . | . | . | . |
| 21-225_14C2_HC | . | . | . | . | R | S | . | . | . | . | . | . | . | . | . | . | . | . | . |
| 21-225_150E7_HC | . | . | . | . | S | E | . | . | . | . | . | . | . | . | . | . | . | . | . |
| 21-225_15B11_HC | . | . | . | . | T | E | . | . | . | . | . | . | . | . | . | . | . | . | . |
| 21-225_15H10_HC | . | . | . | . | . | S | . | . | . | . | . | . | ! | . | . | . | . | . | . |
| 21-225_161D11_HC | . | . | . | . | . | S | . | . | . | . | . | . | . | . | . | . | . | . | . |
| 21-225_161F7_HC | . | . | . | . | . | S | . | . | . | . | . | . | . | . | . | . | . | . | . |
| 21-225_16H4_HC | . | . | . | . | T | . | . | C | . | . | . | . | . | . | . | . | . | . | . |
| 21-225_180H7_HC | . | . | . | . | S | . | . | . | . | . | . | . | . | . | . | . | . | . | . |
| 21-225_181A8_HC | . | . | . | . | S | . | . | . | . | . | . | . | . | . | . | ! | . | . | . |
| 21-225_184D11_HC | . | . | . | . | R | . | . | . | . | . | . | . | . | . | . | . | . | . | . |
| 21-225_184H10_HC | . | . | . | . | R | . | . | . | . | . | . | . | . | . | . | . | . | . | . |
| 21-225_18H12_HC | . | . | . | . | Y | S | . | . | . | . | . | . | . | . | . | . | . | . | . |
| 21-225_203C10_HC | . | . | . | . | . | . | . | . | . | . | . | . | . | . | . | . | . | . | . |
| 21-225_210E4_HC | . | . | . | . | . | S | . | . | . | . | . | . | . | . | . | . | . | . | . |
| 21-225_225B6_HC | . | . | . | . | T | S | . | . | . | . | . | . | . | . | . | . | . | . | . |
| 21-225_225D6_HC | . | . | . | . | T | S | . | . | . | . | . | . | . | . | . | . | . | . | . |
| 21-225_225F8_HC | . | . | . | . | T | S | . | . | . | . | . | . | . | . | . | . | . | . | . |
| 21-225_226A9_HC | . | . | . | . | T | S | . | . | . | . | . | . | . | . | . | . | . | . | . |
| 21-225_227C12_HC | . | . | . | . | T | S | . | . | . | . | . | . | . | . | . | . | . | . | . |
| 21-225_227G3_HC | . | . | . | . | T | S | . | . | . | . | . | . | . | . | . | . | . | . | . |
| 21-225_227H5_HC | . | . | . | . | T | S | . | . | . | . | . | . | . | . | . | . | . | . | . |
| 21-225_22B7_HC | . | . | . | . | R | S | . | . | . | . | . | . | . | . | . | . | . | . | . |
| 21-225_25A9_HC | . | . | . | . | . | . | . | . | . | . | . | . | . | . | . | . | . | . | . |
| 21-225_25G5_HC | . | . | . | . | . | . | . | . | . | . | . | . | . | . | . | . | . | . | . |
| 21-225_28A9_HC | . | . | . | . | . | . | . | . | . | . | . | . | . | . | . | . | . | . | . |
| 21-225_28B8_HC | . | . | . | . | . | . | . | . | . | . | . | . | . | . | . | . | . | . | . |
| 21-225_29D8_HC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  | H_FR4 |  |  |  |  |  |  |  |  |  |  |
| CONSENSUS | - | - | - | - | L | D | D | Y | W | G | Q | G | T | L | V | T | V | S | S |
| 21-225_29D9_HC | . | . | . | . | . | . | . | . | . | . | . | . | . | Q | . | . | . | . | . |
| 21-225_30F3_HC | . | . | . | . | Y | . | . | . | . | . | . | . | . | . | . | . | . | . | . |
| 21-225_33B1_HC | . | . | . | . | Y | . | . | . | . | . | . | . | . | . | . | . | . | . | . |
| 21-225_4C8_HC | . | . | . | . | . | S | . | . | . | . | . | . | . | . | . | . | . | . | . |
| 21-225_4F12_HC | . | . | . | . | . | S | . | . | . | . | . | . | . | . | . | . | . | . | . |
| 21-225_50C4_HC | . | . | . | . | . | N | . | . | . | . | . | . | . | . | . | . | . | . | . |
| 21-225_73G6_HC | . | . | . | . | T | S | . | . | . | . | . | . | . | . | . | . | . | . | . |
| 21-225_90H5_HC | . | . | . | . | . | . | . | . | . | . | . | . | . | . | . | . | . | . | . |

**FIGURE 57**

Table 58.    <u>Consensus 24-VH3|3-21/D4|4-11|RF2/JH4 (SEQ ID NO: 50275)</u>

EVQLVES-GGGLVKPGGSLRLSCAASG-FTF<u>SS</u>-----<u>YSMN</u>WVRQAPGKGLEWVS<u>SISGS</u>---
<u>SSYIYY</u>ADSVKGRFTISRDNAKNSLYLQMNSLRAEDTAVYYCAR<u>DRG</u>-----------------------SSWGQGTLVTVSS wherein:

S at position 33 can be substituted with T

Y at position 39 can be substituted with F

S at position 40 can be substituted with T, G or R

M at position 41 can be substituted with L

S at position 57 can be substituted with A, C or L

G at position 60 can be substituted with S

S at position 65 can be substituted with G or T

S at position 66 can be substituted with T, G or Y

Y at position 67 can be substituted with H

I at position 68 can be substituted with L or M

Y at position 69 can be substituted with S or W

A at position 71 can be substituted with G, P or V

V at position 74 can be substituted with L

G at position 76 can be substituted with A

D at position 109 can be substituted with T

G at position 111 can be substituted with null (-), S or Y

null (-) at position 112 can be substituted with G

**FIGURE 57**

null (-) at position 135 can be substituted with S

null (-) at position 136 can be substituted with F or S

S at position 137 can be substituted with D, G or H

S at position 138 can be substituted with L, Y, G, T, C, E or I

Xaa Xaa Xaa Xaa Asn (SEQ ID NO: 50028)

Xaa Ile Ser Xaa Ser Xaa Xaa Xaa Xaa Xaa Tyr Xaa Asp Ser Xaa Lys Xaa (SEQ ID NO: 50029)

Xaa Arg Xaa Xaa Xaa Xaa Xaa Xaa (SEQ ID NO: 50030)

Ser Tyr Ser Met Asn (SEQ ID NO: 50495)

Ser Ile Ser Gly Ser Ser Ser Tyr Ile Tyr Tyr Ala Asp Ser Val Lys Gly (SEQ ID NO: 50496)

Asp Arg Gly Ser Ser (SEQ ID NO: 50497)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | V | E | S | - | G | G | G | L | V | K | P | G | G | S | L | R | L | S | C | A | A | S |
| 21-225_13D3_HC | . | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . | . | F | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | . | . | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | F | . | . | . | . | . |
| 21-225_158G8_HC | . | . | . | . | . | . | . | . | . | . | . | . | . | T | . | . | . | . | . | . | . | . | . | . | . | . |
| 21-225_158H5_HC | . | . | . | . | . | . | . | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . | . | F | . | . | . | . | . |
| 21-225_160H1_HC | . | . | . | . | . | . | . | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | F | . | . | . | . | . |
| 21-225_162H3_HC | . | . | . | . | . | . | . | . | . | . | . | . | . | . | . | . | . | . | . | . | F | . | . | . | . | . |
| 21-225_163F9_HC | . | . | . | . | . | . | . | . | . | . | . | . | . | Q | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | . | . |
| 21-225_17D8_HC | . | . | . | . | . | . | . | . | . | . | . | . | . | . | . | . | . | . | . | . | . | . | . | . | . | . |
| 21-225_20B5_HC | . | . | . | . | . | A | . | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | . | . | . | . | . | . | . | . | . | . | . | . | . | . | . | . | . | . | . | . |
| 21-225_22H3_HC | A | . | . | . | . | . | . | . | . | . | . | . | . | . | . | . | . | . | . | . | . | . | . | . | . | . |
| 21-225_23E11_HC | . | . | . | . | . | . | . | . | . | . | . | . | . | . | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | . | . | . | . | . | . | . | . | . | . | . | . | . | . | . |
| 21-225_43E1_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | V | E | S | - | G | G | G | L | V | K | P | G | G | S | L | R | L | S | C | A | A | S |
| 21-225_53G1_HC | . | . | . | . | . | . | . | . | . | . | . | . | . | . | . | . | E | . | . | . | . | . | . | . | . | . |
| 21-225_56F1_HC | . | . | . | . | . | . | . | . | . | . | . | . | . | . | S | . | . | . | . | . | . | . | . | . | . | . |
| 21-225_56H7_HC | . | . | . | . | . | . | . | . | . | . | . | . | . | . | . | . | . | . | . | . | . | . | . | . | . | . |
| 21-225_58C5_HC | . | . | . | . | . | . | . | . | . | . | . | . | . | . | . | . | . | . | . | . | . | . | . | . | . | . |
| 21-225_65C12_HC | . | . | . | . | . | . | . | . | . | . | . | . | . | . | . | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | S | M | N | W | V | R | Q | A | P | G | K | G | L |
| 21-225_13D3_HC | . | . | . | . | . | . | . | . | . | . | . | . | . | T | . | . | . | . | . | . | . | . | . | . | . | . |
| 1-225_14B1_LC1_HC | . | . | . | . | . | . | . | . | . | . | . | . | . | T | . | . | . | . | . | . | . | . | . | . | . | . |
| 1-225_14B1_LC2_HC | . | . | . | . | . | . | . | . | . | . | . | . | . | T | . | . | . | . | . | . | . | . | . | . | . | . |
| 21-225_157G7_HC | . | . | . | . | . | . | . | . | . | . | . | . | . | . | . | . | . | . | . | . | . | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | . | . | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | . | . | . | . | . | . |
| 21-225_158G8_HC | . | . | . | . | . | . | . | . | . | . | . | . | . | T | . | . | . | . | . | . | . | . | . | . | . | . |
| 21-225_158H5_HC | . | . | . | . | . | . | . | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . | . | . | . | . | . | . | . |
| 21-225_160H1_HC | . | . | . | . | . | . | . | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | . | . | . | . | . | . |
| 21-225_162H3_HC | . | . | . | . | . | . | . | . | . | . | . | . | . | . | . | . | . | . | . | . | . | . | . | . | . | . |
| 21-225_163F9_HC | . | . | . | . | . | . | . | . | . | . | . | . | . | . | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | . | . |
| 21-225_17D8_HC | . | . | . | . | . | . | . | . | . | . | . | . | . | . | . | . | . | . | . | . | . | . | . | . | . | . |
| 21-225_20B5_HC | . | . | . | . | . | T | . | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | T | . | . | . | . | . | . | . | . | . | . | . | . | . | . | . | . | . | . | . |
| 21-225_22H3_HC | . | . | . | . | . | . | . | . | . | . | . | . | . | . | . | . | . | . | . | . | . | . | . | . | . | . |
| 21-225_23E11_HC | . | . | . | . | . | . | . | . | . | . | . | . | . | T | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | . | . | T | . | . | . | . | . | . | . | . | . | . | . | . |
| 21-225_43E1_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | H_CDR1 | | | | | | | | | | | | | | H_FR2 | | | |
| CONSENSUS | G | - | F | T | F | S | S | - | - | - | - | - | Y | S | M | N | W | V | R | Q | A | P | G | K | G | L |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | S | . | . | . | . | . | . | . | . | . | . | . | . | . | . | . | . | . | . | . | . | . | . |
| 21-225_58C5_HC | . | . | . | . | . | N | . | . | . | . | . | . | . | . | . | . | . | . | . | . | . | . | . | . | . | . |
| 21-225_65C12_HC | . | . | . | . | . | R | . | . | . | . | . | . | S | . | L | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | S | I | S | G | S | - | - | - | S | S | Y | I | Y | Y | A | D | S | V | K | G | R | F |
| 21-225_13D3_HC | D | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | L | . | . | P | . | . | . | . | . | . | . |
| 1-225_14B1_LC2_HC | . | . | . | . | . | . | . | . | . | . | . | . | . | . | . | L | . | . | P | . | . | . | . | . | . | . |
| 21-225_157G7_HC | . | . | . | . | . | . | . | . | . | . | . | . | . | . | T | . | . | . | . | . | . | . | . | . | . | . |
| 21-225_157H4_HC | . | . | . | . | . | . | . | S | . | . | . | . | . | . | T | . | . | . | . | . | . | . | . | . | . | . |
| 21-225_157H7_HC | . | . | . | O | . | . | . | . | . | . | . | . | . | . | . | . | . | . | . | . | . | . | . | . | . | . |
| 21-225_158F5_HC | . | . | . | . | . | . | . | . | . | . | . | . | . | . | T | . | . | . | . | . | . | . | . | . | . | . |
| 21-225_158G8_HC | . | . | . | . | L | . | . | . | . | . | . | . | . | . | . | . | . | . | . | . | . | . | . | . | . | . |
| 21-225_158H5_HC | . | . | . | . | . | . | . | . | . | . | . | . | . | . | T | . | . | . | . | . | . | . | . | . | . | . |
| 21-225_160F4_HC | . | . | . | . | . | . | . | . | . | . | . | . | . | . | T | . | . | . | . | . | . | . | . | . | . | . |
| 21-225_160H1_HC | . | . | . | . | . | . | . | S | . | . | . | . | T | G | . | M | . | . | . | . | . | . | . | . | . | . |
| 21-225_162F2_HC | . | . | . | . | . | . | . | . | . | . | . | . | . | . | T | . | . | . | . | . | . | . | . | . | . | . |
| 21-225_162H3_HC | . | . | . | . | . | . | . | . | . | . | . | . | . | . | T | . | . | . | . | . | . | . | . | . | . | . |
| 21-225_163F9_HC | . | . | . | . | . | . | . | . | . | . | . | . | . | . | T | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | M | . | . | . | . | . | . | . | . | . | . |
| 21-225_17D8_HC | . | . | . | . | . | . | . | . | . | . | . | . | G | . | H | . | S | . | . | . | . | . | . | . | . | . |
| 21-225_20B5_HC | . | . | . | . | . | . | . | . | . | . | . | . | . | . | . | . | . | . | . | . | . | . | . | . | . | . |
| 21-225_20B8_HC | . | . | . | . | . | . | . | . | . | . | . | . | G | . | H | . | . | . | . | . | . | . | . | . | . | . |
| 21-225_20C8_HC | . | . | . | . | . | . | . | . | . | . | . | . | . | . | . | . | . | . | . | . | . | . | . | A | . | . |
| 21-225_20F9_HC | . | . | . | . | . | . | S | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | L | . | . | . | . |
| 21-225_20H10_HC | . | . | . | . | . | . | . | S | . | . | . | . | . | . | . | . | . | . | . | . | . | . | . | . | . | . |
| 21-225_22H3_HC | . | . | . | . | . | . | . | S | . | . | . | . | . | . | . | . | . | . | . | . | . | . | . | . | . | . |
| 21-225_23E11_HC | . | . | . | . | . | . | . | . | . | . | . | . | . | . | . | . | W | . | V | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | S | . | . | . | . | . | . | . | . | . | . | . | . | . | . | . | . | . | . |
| 21-225_33C2_HC | Q | . | . | . | . | . | . | . | . | . | . | . | . | . | . | . | . | . | . | . | . | . | . | . | . | . |
| 21-225_43E1_HC | . | . | . | A | . | . | . | N | . | . | . | . | . | . | T | . | . | . | . | . | . | L | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | S | I | S | G | S | - | - | - | S | S | Y | I | Y | Y | A | D | S | V | K | G | R | F |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | S | . | . | . | . | . | T | . | . | . | . | G | . | . | . | . | . | . | . |
| 21-225_56H7_HC | . | . | . | . | . | . | . | S | . | . | . | . | . | T | . | . | . | . | G | . | . | . | . | . | . | . |
| 21-225_58C5_HC | . | . | I | . | . | . | . | S | . | . | . | . | . | Y | . | L | . | . | . | . | . | . | . | . | . | . |
| 21-225_65C12_HC | . | . | . | . | . | . | . | S | . | . | . | . | . | . | . | . | . | . | . | . | . | . | . | . | . | . |
| 21-225_6C12_HC | . | . | . | . | . | . | . | S | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | A | K | N | S | L | Y | L | Q | M | N | S | L | R | A | E | D | T | A | V | Y |
| 21-225_13D3_HC | . | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . | . | . | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | M | . | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | . | . | . | . | . | . |
| 21-225_158G8_HC | . | . | . | . | . | . | . | . | . | . | . | . | . | . | . | . | . | . | . | . | . | . | . | . | . | . |
| 21-225_158H5_HC | . | . | . | . | . | . | . | . | . | . | . | H | . | . | . | . | . | . | . | . | . | . | . | . | . | . |
| 21-225_160F4_HC | . | . | . | . | . | . | . | . | . | . | . | . | . | . | . | . | . | . | . | . | . | . | . | . | . | . |
| 21-225_160H1_HC | . | . | . | . | . | . | . | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | . | . | . | . | . | . |
| 21-225_162H3_HC | . | . | . | . | . | . | . | . | . | . | . | . | . | . | . | . | . | . | . | . | . | . | . | . | . | . |
| 21-225_163F9_HC | . | . | . | . | . | . | . | . | . | . | . | . | . | . | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | L | . |
| 21-225_17D8_HC | . | . | . | . | . | . | . | . | . | . | . | . | . | L | . | . | . | . | . | . | . | . | . | . | . | . |
| 21-225_20B5_HC | . | . | . | . | . | . | . | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | . | . | . | . | . | . | . | . | . | . | . | . | . | . | . | . | . | I | . | . |
| 21-225_22H3_HC | . | . | . | . | . | . | . | . | . | . | . | . | . | . | . | . | . | . | . | . | . | . | . | . | . | F |
| 21-225_23E11_HC | . | . | . | . | . | . | . | . | . | . | . | . | . | . | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | F |
| 21-225_33C2_HC | . | . | . | . | . | . | . | . | . | . | F | . | . | . | . | . | . | . | . | . | . | . | . | I | . | F |
| 21-225_43E1_HC | . | . | . | . | . | . | . | . | . | . | F | . | . | L | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | | |
| CONSENSUS | T | I | S | R | D | N | A | K | N | S | L | Y | L | Q | M | N | S | L | R | A | E | D | T | A | V | Y |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . | . | . | . | . | . | . | . |
| 21-225_58C5_HC | . | . | . | . | . | S | . | . | . | . | . | . | . | . | . | . | . | . | . | . | . | . | . | . | . | . |
| 21-225_65C12_HC | . | . | . | . | . | . | . | . | . | . | . | . | . | . | . | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | G | - | - | - | - | - | - | - | - | - | - | - | - | - | - | - | - | - | - | - |
| 21-225_13D3_HC | . | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . | . | . | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | . | V | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | . | . | . | . | . | . |
| 21-225_158G8_HC | . | . | . | . | . | . | . | . | . | . | . | . | . | . | . | . | . | . | . | . | . | . | . | . | . | . |
| 21-225_158H5_HC | . | . | . | . | . | . | . | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . | . | . | . | . | . | . | . |
| 21-225_160H1_HC | F | . | . | . | . | . | - | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | . | . | . | . | . | . |
| 21-225_162H3_HC | . | . | . | . | . | . | . | . | . | . | . | . | . | . | . | . | . | . | . | . | . | . | . | . | . | . |
| 21-225_163F9_HC | . | . | . | . | . | . | . | . | . | . | . | . | . | . | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | . | . |
| 21-225_17D8_HC | . | . | . | . | . | . | . | . | . | . | . | . | . | . | . | . | . | . | . | . | . | . | . | . | . | . |
| 21-225_20B5_HC | . | . | . | . | . | . | . | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | . | . | . | . | . | . | . | . | . | . | . | . | . | . | . | . | . | . | . | . |
| 21-225_22H3_HC | . | . | . | . | . | . | . | . | . | . | . | . | . | . | . | . | . | . | . | . | . | . | . | . | . | . |
| 21-225_23E11_HC | . | . | . | . | . | . | . | . | . | . | . | . | . | . | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | . | . | . | . | . | . | . | . | . | . | . | . | . | . | . |
| 21-225_43E1_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | G | - | - | - | - | - | - | - | - | - | - | - | - | - | - | - | - | - | - | - |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . | . | . | . | . | . | . | . |
| 21-225_58C5_HC | . | . | . | . | T | Y | S | G | . | . | . | . | . | . | . | . | . | . | . | . | . | . | . | . | . | . |
| 21-225_65C12_HC | . | . | . | . | . | A | Y | . | . | . | . | . | . | . | . | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

# EP 4 435 105 A2

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | - | - | S | S | W | G | Q | G | T | L | V | T | V | S | S |
| 21-225_13D3_HC | | | | | | | | Ø | | | | | | | | | | | |
| 1-225_14B1_LC1_HC | | | | | | | | | | | | | | | | | | | |
| 1-225_14B1_LC2_HC | | | | | | | | | | | | | | | | | | | |
| 21-225_157G7_HC | | | | | | | | | | | | | | | | | | | |
| 21-225_157H4_HC | | | | | | | | I | | | | | | | | | | | |
| 21-225_157H7_HC | | | | | | | Ø | Y | | | | | | | | | | | |
| 21-225_158F5_HC | | | | | | | | | | | | | | | | | | | |
| 21-225_158G8_HC | | | | | | | | L | | | | | | | | | | | |
| 21-225_158H5_HC | | | | | | | | | | | | | | | | | | | |
| 21-225_160F4_HC | | | | | | | | | | | | | | | | | | | |
| 21-225_160H1_HC | | | | | | | Ø | Y | | | | | | | | | | | |
| 21-225_162F2_HC | | | | | | | | | | | | | | | | | | | |
| 21-225_162H3_HC | | | | | | | | | | | | | | | | | | | |
| 21-225_163F9_HC | | | | | | | | L | | | | | | | | | | | |
| 21-225_171A11_HC | | | | | | | | | | | | | I | | | | | | |
| 21-225_17C10_HC | | | | | | | | | | | | | | | | | | | |
| 21-225_17D8_HC | | | | | | | | Ø | | | | | | | | | | | |
| 21-225_20B5_HC | | | | | | | | Y | | | | | | | | | | | |
| 21-225_20B8_HC | | | | | | | | Ø | | | | | | | | | | | |
| 21-225_20C8_HC | | | | | | | | L | | | | | | | | | | | |
| 21-225_20F9_HC | | | | | | | | C | | | | | | | | | I | | |
| 21-225_20G12_HC | | | | | | | | Y | | | | | | | | | | | |
| 21-225_20H10_HC | | | | | | | | L | | | | | | | | | | | |
| 21-225_22H3_HC | | | | | | | | L | | | | | | | | | | | |
| 21-225_23E11_HC | | | | | | | | L | | | | | | | | | | | |
| 21-225_30H11_HC | | | | | | | | L | | | | | | | | | | | |
| 21-225_31G9_HC | | | | | | | | | | | | | | | | | | | |
| 21-225_33C2_HC | | | | | | | Ø | T | | | | | | | | | | | |
| 21-225_43E1_HC | | | | | | | | E | | | | | | | | | | | |

3863

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | - | - | S | S | W | G | Q | G | T | L | V | T | V | S | S |
| 21-225_53G1_HC | . | . | . | . | . | . | . | T | . | . | . | . | . | . | . | . | . | . | . |
| 21-225_56F1_HC | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . |
| 21-225_58C5_HC | . | . | . | . | S | F | D | Y | . | . | . | . | . | . | . | . | . | . | . |
| 21-225_65C12_HC | . | . | . | . | . | S | H | Y | . | . | . | . | . | . | . | . | . | . | . |
| 21-225_6C12_HC | . | . | . | . | . | . | . | L | . | . | . | . | . | . | . | . | . | . | . |

**FIGURE 57**

Table 59.  <u>Consensus 25-VH3|3-23/D6|6-19|RF2/JH3 (SEQ ID NO: 50276)</u>

EVQLLES-GGGLVQPGGSLRLSCAASE-FTF<u>SS-----YAMS</u>WVRQAPGKGLEWVS<u>VISGR---GGNTF</u>YADSVKGRFTISRDNSKNTLYLQMNSLRAEDTAVYYCAS<u>RIAVAG-----------------SEAFDI</u>WGQGTMVTVSS wherein:

S at position 33 can be substituted with G

A at position 40 can be substituted with V

S at position 42 can be substituted with N or T

V at position 57 can be substituted with I or A

G at position 60 can be substituted with R

R at position 61 can be substituted with S

G at position 66 can be substituted with V, I or T

N at position 67 can be substituted with Y, S or T

T at position 68 can be substituted with A

F at position 69 can be substituted with Y

Y at position 70 can be substituted with N or S

K at position 75 can be substituted with R

I at position 110 can be substituted with L, M or V

A at position 113 can be substituted with D

S at position 133 can be substituted with N or Y

E at position 134 can be substituted with D

F at position 136 can be substituted with C

**FIGURE 57**

D at position 137 can be substituted with A or H

I at position 138 can be substituted with V

Xaa Tyr Xaa Met Xaa (SEQ ID NO: 50031)

Xaa Ile Ser Xaa Xaa Gly Xaa Xaa Xaa Xaa Xaa Ala Asp Ser Val Xaa Gly (SEQ ID NO: 50032)

Arg Xaa Ala Val Xaa Gly Xaa Xaa Ala Xaa Xaa Xaa (SEQ ID NO: 50033)

Ser Tyr Ala Met Ser (SEQ ID NO: 50498)

Val Ile Ser Gly Arg Gly Gly Asn Thr Phe Tyr Ala Asp Ser Val Lys Gly (SEQ ID NO: 50499)

Arg Ile Ala Val Ala Gly Ser Glu Ala Phe Asp Ile (SEQ ID NO: 50500)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_10F12_HC | | | | | | | | | | | | | | | | | | | | | | | | | | |
| 21-225_11A5_HC | | | | | | | | | | | D | | | | | | | | | | | | | | | |
| 21-225_16H6_HC | | | | | | | | | | | | | | | | | | | | | | | | | | |
| 21-225_17A12_HC | | | | | | | | | | | | | I | | | | | | | | | | | | | |
| 21-225_17B4_HC | | | | | | | | | | | | | | | | | | | | | | | | | | |
| 21-225_17C6_HC | | | | | | | | | | | | | | | | | | | | | | | | | | |
| 21-225_17F11_HC | | A | | | | | | | | | | | E | | | | | | | | | | | | | |
| 21-225_17F7_HC | | | | | | | | | | | | | | | | | | | | | | | | | | |
| 21-225_18E12_HC | | | | | | | | | | | | | | | | | | | | | | | | | | |
| 21-225_200G1_HC | | | | | | | | | | | | | | | | | | | | | | | | | | |
| 21-225_20A4_HC | | | | | | | | | | | | | | | | | | | | | | | | | | |
| 21-225_21A2_HC | | | | | | | | | | | | | | | | | | | | | | | | | | |
| 21-225_21F4_HC | | | | | | | | | | | | | | | | | | | | | | | | | | |
| 21-225_22C5_HC | | | | | | | | | | | | | | | | V | | | | | | | | | | |
| 21-225_22D5_HC | | | | | | | | | | | | | | | | | | | | | | | | | | |
| 21-225_22E4_HC | | | | | | | | | | | | | | | | | | | | | | | | | | |
| 21-225_22G10_HC | | | | | | | | | | | D | | | | | | | | | | | | | T | | |
| 21-225_22G3_HC | | | | | | | | | | | | | | | | | | | | | | | | | | |
| 21-225_3B1_HC | | | | | | | | | | | | | | | | | | | | | | | | | | |
| 21-225_3E3_HC | | | | | | | | | | | T | | | | | | | | | | | | | | | |
| 21-225_48C3_HC | | | | | | | | | | | | | | | | | | | | | | | | | | |
| 21-225_48D1_HC | | | | | | | | | | | | | | | | | | | | | | | | | | |
| 21-225_49G12_HC | | | | | | | | | | | | | | | | | | | | | | | | T | | |
| 21-225_4H2_HC | | | | | | | | | | | | | | | | | | | | | | | | | | |
| 21-225_50H10_HC | | G | | | | | | | | | | | | | | | | | | | | | | | | |
| 21-225_50H8_HC | | | | | | | | | | | | | | | | | | | | | | | | | | |
| 21-225_51H10_HC | | | | | | | | | | | | | | | | | | | | | | | | T | | |
| 21-225_5F7_HC | | | | | | | | | | | | | | | | | | | | | | | | | | |
| 21-225_5G2_HC | | | | | | | | | | | | | | | | | | | | | | | | | | |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_6D3_HC | . | . | . | . | . | . | . | . | . | . | . | . | . | . | . | . | . | . | . | . | . | . | . | . | . | . |
| 21-225_8E12_HC | . | . | . | . | . | . | . | . | . | . | . | . | . | . | . | . | . | . | . | . | . | . | . | . | . | . |
| 21-225_8G6_HC | . | . | . | . | . | . | . | . | . | . | . | . | . | . | . | . | . | . | . | . | . | . | . | V | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | E | - | F | T | F | S | S | - | - | - | - | - | Y | A | M | S | W | V | R | Q | A | P | G | K | G | L |
| 21-225_10F12_HC | . | . | . | . | . | . | . | . | . | . | . | . | . | . | . | . | . | . | . | . | . | . | . | . | . | . |
| 21-225_11A5_HC | . | . | . | . | . | . | . | . | . | . | . | . | . | . | . | . | . | . | . | . | . | . | . | . | . | . |
| 21-225_16H6_HC | G | . | . | . | . | . | . | . | . | . | . | . | . | . | . | N | . | . | . | . | . | . | . | M | . | . |
| 21-225_17A12_HC | . | . | . | . | . | . | . | . | . | . | . | . | . | . | . | . | . | . | . | . | O | . | . | . | . | . |
| 21-225_17B4_HC | . | . | . | . | . | . | . | . | . | . | . | . | . | . | . | N | . | . | . | . | . | . | . | . | . | . |
| 21-225_17C6_HC | . | . | . | . | . | . | . | . | . | . | . | . | . | . | . | N | . | . | . | . | . | . | . | . | . | . |
| 21-225_17F11_HC | G | . | . | . | . | . | . | . | . | . | . | . | . | . | . | . | . | . | . | . | . | . | . | . | . | . |
| 21-225_17F7_HC | . | . | . | . | . | . | . | . | . | . | . | . | . | . | . | N | . | . | . | . | . | . | . | . | . | . |
| 21-225_18E12_HC | . | . | . | . | . | . | . | . | . | . | . | . | . | . | . | . | . | . | . | . | . | . | . | T | . | . |
| 21-225_200G1_HC | G | . | . | . | . | . | . | . | . | . | . | . | . | . | . | . | . | . | . | . | . | . | . | . | . | . |
| 21-225_20A4_HC | G | . | . | . | . | . | . | . | . | . | . | . | . | . | . | T | . | . | . | G | . | . | . | M | . | . |
| 21-225_21A2_HC | . | . | . | . | . | . | . | . | . | . | . | . | . | . | . | N | . | . | . | . | . | . | . | . | . | . |
| 21-225_21F4_HC | . | . | . | . | . | . | . | . | . | . | . | . | . | . | . | N | . | . | . | . | . | . | . | . | . | . |
| 21-225_22C5_HC | G | . | . | . | . | . | . | . | . | . | . | . | . | . | . | . | . | . | . | . | . | . | . | M | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | . | . | . | . | . | . | . | . | . | . | . | . | . | . | . | . | . | . | . | . | . | . |
| 21-225_22G10_HC | G | . | . | . | . | . | . | . | . | . | . | . | . | . | . | N | . | . | . | . | . | . | . | . | . | . |
| 21-225_22G3_HC | . | . | . | . | . | . | . | . | . | . | . | . | . | . | . | N | . | . | . | . | . | . | . | . | . | . |
| 21-225_3B1_HC | . | . | . | . | . | . | . | . | . | . | . | . | . | . | . | N | . | . | . | . | . | . | . | . | . | . |
| 21-225_3E3_HC | . | . | . | . | . | . | . | . | . | . | . | . | . | V | . | . | . | . | . | . | . | . | . | . | . | . |
| 21-225_48C3_HC | . | . | . | . | . | . | . | . | . | . | . | . | . | . | . | . | . | . | . | . | . | . | . | . | . | . |
| 21-225_48D1_HC | . | . | . | . | . | . | . | . | . | . | . | . | . | . | . | . | . | . | . | . | . | . | . | . | . | . |
| 21-225_49G12_HC | G | . | . | . | . | . | . | . | . | . | . | . | . | . | . | . | . | . | . | . | . | . | . | . | . | . |
| 21-225_4H2_HC | G | . | . | . | . | . | G | . | . | . | . | . | . | . | . | N | . | . | . | . | . | . | . | M | . | . |
| 21-225_50H10_HC | . | . | . | . | . | . | . | . | . | . | . | . | . | . | . | . | . | . | . | . | . | . | . | . | . | . |
| 21-225_50H8_HC | . | . | . | . | . | . | . | . | . | . | . | . | . | . | . | . | . | . | . | . | . | . | . | . | . | . |
| 21-225_51H10_HC | G | . | . | . | . | . | . | . | . | . | . | . | . | . | . | . | . | . | . | . | . | . | . | . | . | . |
| 21-225_5F7_HC | . | . | . | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

3869

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | H_FR2 | | | | | | | | | |
| CONSENSUS | E | - | F | T | F | S | S | - | - | - | - | - | Y | A | M | S | W | V | R | Q | A | P | G | K | G | L |
| 21-225_6D3_HC | . | . | . | . | . | . | . | . | . | . | . | . | . | . | . | N | . | . | . | . | . | . | . | . | . | . |
| 21-225_8E12_HC | . | . | . | . | . | . | . | . | . | . | . | . | . | . | . | . | . | . | . | . | . | . | . | . | . | . |
| 21-225_8G6_HC | . | . | . | . | . | . | . | . | . | . | . | . | . | V | . | N | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
|  |  |  |  |  |  |  |  |  |  |  |  |  | H_CDR2 |  |  |  |  |  |  |  |  |  |  |  |  |  |
| CONSENSUS | E | W | V | S | V | I | S | G | R | - | - | - | G | G | N | T | F | Y | A | D | S | V | K | G | R | F |
| 21-225_10F12_HC |  |  |  |  | I |  |  |  |  |  |  |  |  |  |  |  |  |  |  |  |  |  |  |  |  |  |
| 21-225_11A5_HC |  |  | I |  |  |  |  |  |  |  |  |  |  | V |  |  |  |  |  |  |  |  |  |  |  |  |
| 21-225_16H6_HC |  |  |  |  |  |  |  |  | S |  |  |  |  |  | Y |  | Y |  |  |  |  |  |  |  |  |  |
| 21-225_17A12_HC |  |  |  |  | I |  |  |  |  |  |  |  |  |  | A |  |  |  |  |  |  |  |  |  |  |  |
| 21-225_17B4_HC |  |  |  |  |  |  |  |  |  |  |  |  |  |  |  |  |  |  |  |  |  |  |  |  |  |  |
| 21-225_17C6_HC |  |  |  |  |  |  |  |  |  |  |  |  |  |  |  |  |  |  |  |  |  |  |  |  |  |  |
| 21-225_17F11_HC |  |  |  |  |  |  |  |  |  |  |  |  |  |  |  |  | Y | S |  |  |  |  |  |  |  |  |
| 21-225_17F7_HC |  |  |  |  | I |  |  |  |  |  |  |  |  |  |  |  |  |  |  |  |  |  |  |  |  |  |
| 21-225_18E12_HC |  |  |  |  | I |  |  |  |  |  |  |  |  |  |  |  |  |  |  |  |  |  |  |  |  |  |
| 21-225_200G1_HC |  |  |  | A |  |  |  |  |  |  |  |  |  |  |  |  |  |  |  |  |  |  |  |  |  |  |
| 21-225_20A4_HC |  |  |  |  |  |  |  |  | S |  |  |  |  |  | Y |  | Y | N |  |  |  |  |  |  |  |  |
| 21-225_21A2_HC |  |  | I |  | I |  |  |  |  |  |  |  |  |  |  |  |  |  |  |  |  |  |  |  |  |  |
| 21-225_21F4_HC |  |  |  |  |  |  |  |  |  |  |  |  |  |  |  |  |  |  |  |  |  |  |  |  |  |  |
| 21-225_22C5_HC |  |  |  |  | I |  |  |  | S |  |  |  |  |  | Y |  | Y |  |  |  |  |  |  |  |  |  |
| 21-225_22D5_HC |  |  |  |  |  |  |  |  |  |  |  |  |  |  |  |  |  |  |  |  |  |  |  |  |  |  |
| 21-225_22E4_HC |  |  |  |  | I |  |  |  |  |  |  |  |  | T |  |  |  |  |  |  |  |  |  |  |  |  |
| 21-225_22G10_HC |  |  |  |  |  |  |  |  | S |  |  |  |  |  |  |  |  |  |  |  |  |  |  |  |  |  |
| 21-225_22G3_HC |  |  |  |  | I |  |  |  |  |  |  |  |  | V |  |  |  |  |  |  |  |  |  |  |  |  |
| 21-225_3B1_HC |  |  |  |  |  |  |  |  |  |  |  |  |  | I |  |  |  |  |  |  |  |  |  |  |  |  |
| 21-225_3E3_HC |  |  |  |  | I |  |  |  |  |  |  |  |  |  |  |  |  |  |  |  |  |  |  |  |  |  |
| 21-225_48C3_HC |  |  |  |  |  |  |  |  |  |  |  |  |  |  | S |  |  |  |  |  |  |  |  |  |  |  |
| 21-225_48D1_HC |  |  |  |  |  |  |  |  |  |  |  |  |  |  | S |  |  |  |  |  |  |  |  |  |  |  |
| 21-225_49G12_HC |  |  |  |  |  |  |  |  | S |  |  |  |  |  | T |  |  |  |  |  |  |  |  |  |  | L |
| 21-225_4H2_HC |  |  |  |  |  |  |  |  | S |  |  |  |  |  | Y |  | Y |  |  |  |  |  |  |  |  |  |
| 21-225_50H10_HC |  |  |  |  |  |  |  |  |  |  |  |  |  |  | S |  |  |  |  |  |  |  |  |  |  |  |
| 21-225_50H8_HC |  |  |  |  |  |  |  |  |  |  |  |  |  |  | T |  |  |  |  |  |  |  |  |  |  |  |
| 21-225_51H10_HC |  |  |  |  |  |  |  |  | S |  |  |  |  |  | T |  |  |  |  |  |  |  |  |  |  | L |
| 21-225_5F7_HC |  |  |  |  | I |  |  |  |  |  |  |  |  |  |  |  |  |  |  |  |  |  |  |  |  |  |
| 21-225_5G2_HC |  |  |  |  |  |  |  |  |  |  |  |  |  | V |  |  |  |  |  |  |  |  |  |  |  |  |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | H_CDR2 | | | | | | | | | | | | | |
| CONSENSUS | E | W | V | S | V | I | S | G | R | - | - | - | G | G | N | T | F | Y | A | D | S | V | K | G | R | F |
| 21-225_6D3_HC | . | . | . | . | . | . | . | . | . | . | . | . | . | . | . | . | . | . | . | . | . | . | . | . | . | . |
| 21-225_8E12_HC | . | . | . | . | ¡ | . | . | . | . | . | . | . | . | . | . | . | . | . | . | . | . | . | . | . | . | . |
| 21-225_8G6_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_10F12_HC | . | . | . | . | . | . | . | . | . | . | . | . | . | . | . | . | . | . | . | . | . | . | . | . | . | . |
| 21-225_11A5_HC | . | . | . | . | . | . | . | . | . | . | . | . | . | . | . | . | . | . | . | . | . | . | . | . | . | . |
| 21-225_16H6_HC | . | . | . | . | . | . | . | . | . | . | . | . | . | . | . | . | . | . | . | . | . | . | . | . | . | . |
| 21-225_17A12_HC | . | . | . | . | . | . | . | . | . | . | . | . | . | . | . | . | . | . | . | . | . | . | . | . | . | . |
| 21-225_17B4_HC | . | . | . | . | . | . | . | . | . | . | . | . | . | . | . | . | . | . | . | . | . | . | . | . | . | . |
| 21-225_17C6_HC | . | . | . | . | . | . | . | . | . | . | . | V | . | . | . | . | . | . | . | . | . | . | . | . | . | . |
| 21-225_17F11_HC | . | . | . | . | . | . | . | . | . | . | . | . | . | . | . | . | . | . | . | . | . | . | . | . | . | . |
| 21-225_17F7_HC | . | . | . | . | . | . | . | . | . | . | . | . | . | . | . | . | . | . | . | . | . | . | . | . | . | . |
| 21-225_18E12_HC | . | . | . | . | . | . | . | . | . | . | . | . | . | . | . | . | . | . | . | . | . | . | . | . | . | . |
| 21-225_200G1_HC | . | . | . | . | . | . | . | . | . | . | . | . | . | . | . | . | . | . | . | . | . | . | . | . | . | . |
| 21-225_20A4_HC | T | . | . | . | . | . | . | . | . | . | . | . | . | . | . | . | . | . | V | . | . | . | . | . | . | . |
| 21-225_21A2_HC | . | . | . | . | . | . | . | . | . | . | . | . | . | . | . | . | . | . | . | . | . | . | . | . | . | . |
| 21-225_21F4_HC | . | . | . | . | . | . | . | . | . | . | . | . | . | . | . | . | . | . | . | . | . | . | . | . | . | . |
| 21-225_22C5_HC | . | . | . | . | . | . | . | . | . | . | . | . | . | . | . | . | . | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | V | . | . | . | . | . | . | . | . | . | . | . | . | . | . | . | . | . | . | . | . | . |
| 21-225_22G10_HC | . | . | . | . | . | . | . | . | . | . | . | V | . | H | . | . | . | . | . | . | . | . | . | . | . | . |
| 21-225_22G3_HC | . | . | . | . | . | . | . | . | . | . | . | . | . | . | . | . | . | . | . | . | . | . | . | . | . | . |
| 21-225_3B1_HC | . | . | . | . | . | . | . | . | . | . | . | . | . | . | . | . | . | . | . | . | . | . | . | . | . | . |
| 21-225_3E3_HC | . | . | . | . | . | . | . | . | . | . | . | . | . | . | . | . | . | . | . | . | . | . | . | . | L | . |
| 21-225_48C3_HC | . | . | . | . | . | . | . | . | . | . | . | . | . | . | . | . | . | . | . | . | . | . | . | . | . | . |
| 21-225_48D1_HC | . | . | . | . | . | . | . | . | . | . | . | . | . | . | . | . | . | . | . | . | . | . | . | . | . | . |
| 21-225_49G12_HC | . | . | . | . | . | . | . | . | . | . | . | . | . | . | . | . | . | . | . | . | . | . | . | . | . | . |
| 21-225_4H2_HC | . | . | . | . | . | . | . | . | . | . | . | . | . | . | . | . | . | . | . | . | . | . | . | . | . | . |
| 21-225_50H10_HC | . | . | . | . | . | . | . | . | . | . | . | . | . | . | . | . | . | . | . | . | . | . | . | . | . | . |
| 21-225_50H8_HC | . | . | . | . | . | . | . | . | . | . | . | . | . | . | . | . | . | . | . | . | . | . | . | . | . | . |
| 21-225_51H10_HC | . | . | . | . | . | . | . | . | . | . | . | . | . | . | . | . | . | . | . | . | . | . | . | . | . | . |
| 21-225_5F7_HC | T | . | S | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | | |
| CONSENSUS | T | I | S | R | D | N | S | K | N | T | L | Y | L | Q | M | N | S | L | R | A | E | D | T | A | V | Y |
| 21-225_6D3_HC | . | . | . | . | . | . | . | . | . | . | . | . | . | . | . | . | . | . | . | . | . | . | . | . | . | . |
| 21-225_8E12_HC | . | . | . | . | . | . | . | . | . | . | . | . | . | . | . | . | . | . | . | . | . | . | . | . | . | . |
| 21-225_8G6_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | H_CDR3 | | | | | | | | |
| CONSENSUS | Y | C | A | S | R | I | A | V | A | G | - | - | - | - | - | - | - | - | - | - | - | - | - | - | - | - |
| 21-225_10F12_HC | . | . | . | . | . | V | . | . | . | . | . | . | . | . | . | . | . | . | . | . | . | . | . | . | . | . |
| 21-225_11A5_HC | . | . | . | . | . | . | . | . | . | . | . | . | . | . | . | . | . | . | . | . | . | . | . | . | . | . |
| 21-225_16H6_HC | . | . | . | . | . | L | . | . | . | . | . | . | . | . | . | . | . | . | . | . | . | . | . | . | . | . |
| 21-225_17A12_HC | . | . | . | . | . | . | . | . | . | . | . | . | . | . | . | . | . | . | . | . | . | . | . | . | . | . |
| 21-225_17B4_HC | . | . | . | . | . | L | . | . | . | . | . | . | . | . | . | . | . | . | . | . | . | . | . | . | . | . |
| 21-225_17C6_HC | . | . | . | . | . | L | . | . | . | . | . | . | . | . | . | . | . | . | . | . | . | . | . | . | . | . |
| 21-225_17F11_HC | . | . | . | . | . | L | . | . | . | . | . | . | . | . | . | . | . | . | . | . | . | . | . | . | . | . |
| 21-225_17F7_HC | . | . | . | . | . | L | . | . | . | . | . | . | . | . | . | . | . | . | . | . | . | . | . | . | . | . |
| 21-225_18E12_HC | . | . | . | . | . | L | . | . | . | . | . | . | . | . | . | . | . | . | . | . | . | . | . | . | . | . |
| 21-225_200G1_HC | . | . | . | R | . | . | . | . | G | . | . | . | . | . | . | . | . | . | . | . | . | . | . | . | . | . |
| 21-225_20A4_HC | . | . | . | . | . | . | . | . | . | . | . | . | . | . | . | . | . | . | . | . | . | . | . | . | . | . |
| 21-225_21A2_HC | . | . | . | . | . | L | . | . | . | . | . | . | . | . | . | . | . | . | . | . | . | . | . | . | . | . |
| 21-225_21F4_HC | . | . | . | . | . | L | . | . | . | . | . | . | . | . | . | . | . | . | . | . | . | . | . | . | . | . |
| 21-225_22C5_HC | . | . | . | . | . | . | . | . | . | . | . | . | . | . | . | . | . | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | . | . | . | . | . | . | . | . | . | . | . | . | . | . | . | . | . | . | . | . | . | . |
| 21-225_22G10_HC | . | . | . | . | . | M | . | . | . | . | . | . | . | . | . | . | . | . | . | . | . | . | . | . | . | . |
| 21-225_22G3_HC | . | . | . | . | . | L | . | . | . | . | . | . | . | . | . | . | . | . | . | . | . | . | . | . | . | . |
| 21-225_3B1_HC | . | . | . | . | . | L | . | . | . | . | . | . | . | . | . | . | . | . | . | . | . | . | . | . | . | . |
| 21-225_3E3_HC | . | . | . | . | . | L | . | . | . | . | . | . | . | . | . | . | . | . | . | . | . | . | . | . | . | . |
| 21-225_48C3_HC | . | . | . | P | . | . | . | . | . | . | . | . | . | . | . | . | . | . | . | . | . | . | . | . | . | . |
| 21-225_48D1_HC | . | . | . | P | . | . | . | . | . | . | . | . | . | . | . | . | . | . | . | . | . | . | . | . | . | . |
| 21-225_49G12_HC | . | . | . | P | . | . | . | . | . | . | . | . | . | . | . | . | . | . | . | . | . | . | . | . | . | . |
| 21-225_4H2_HC | . | . | . | . | . | L | . | . | . | . | . | . | . | . | . | . | . | . | . | . | . | . | . | . | . | . |
| 21-225_50H10_HC | . | . | . | R | . | . | . | . | . | . | . | . | . | . | . | . | . | . | . | . | . | . | . | . | . | . |
| 21-225_50H8_HC | . | . | . | R | . | . | . | . | . | . | . | . | . | . | . | . | . | . | . | . | . | . | . | . | . | . |
| 21-225_51H10_HC | . | . | . | R | . | . | . | . | . | . | . | . | . | . | . | . | . | . | . | . | . | . | . | . | . | . |
| 21-225_5F7_HC | Y | . | . | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

3875

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | S | R | I | A | V | A | G | - | - | - | - | - | - | - | - | - | - | - | - | - | - | - | - |
| 21-225_6D3_HC | ℱ | . | . | . | . | L | . | . | . | . | . | . | . | . | . | . | . | . | . | . | . | . | . | . | . | . |
| 21-225_8E12_HC | . | . | . | . | . | M | . | . | . | . | . | . | . | . | . | . | . | . | . | . | . | . | . | . | . | . |
| 21-225_8G6_HC | . | . | . | . | . | L | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | S | E | A | F | D | I | W | G | Q | G | T | M | V | T | V | S | S |
| 21-225_10F12_HC | . | | . | . | . | . | A | | . | | | . | . | | | . | . | | |
| 21-225_11A5_HC | . | | . | . | . | . | . | . | . | . | . | . | . | . | . | . | . | | |
| 21-225_16H6_HC | . | | . | . | . | . | . | . | . | . | . | . | . | . | . | . | . | | |
| 21-225_17A12_HC | . | | . | . | . | . | . | . | . | . | . | . | . | . | . | . | . | | |
| 21-225_17B4_HC | . | | . | . | . | . | . | . | . | . | . | . | . | . | . | . | . | | |
| 21-225_17C6_HC | . | | . | . | . | . | . | . | . | . | . | . | . | . | . | . | . | | |
| 21-225_17F11_HC | . | | . | . | . | . | 8 | . | . | . | . | . | . | . | . | . | . | | |
| 21-225_17F7_HC | | | . | . | . | . | . | . | . | . | . | . | . | . | . | . | . | | |
| 21-225_18E12_HC | . | | . | . | . | . | . | . | . | . | . | . | . | . | . | . | . | | |
| 21-225_200G1_HC | . | | Y | D | . | . | . | V | . | . | . | . | . | . | . | . | . | | |
| 21-225_20A4_HC | . | | . | . | . | . | . | . | . | . | . | . | . | . | . | . | . | | |
| 21-225_21A2_HC | . | | . | . | . | . | . | . | . | . | . | . | . | . | . | . | . | | |
| 21-225_21F4_HC | . | | . | . | . | C | . | . | . | . | . | . | . | . | . | . | . | | |
| 21-225_22C5_HC | . | | . | . | . | . | . | . | . | . | . | . | . | . | . | . | . | | |
| 21-225_22D5_HC | . | | . | . | . | . | . | . | . | . | . | . | . | . | . | . | . | | |
| 21-225_22E4_HC | . | | . | . | . | . | . | . | . | . | . | . | . | . | . | . | . | | |
| 21-225_22G10_HC | . | | . | . | . | . | . | . | . | . | . | . | . | . | . | . | . | | |
| 21-225_22G3_HC | . | | . | . | . | . | . | . | . | . | . | . | . | . | . | . | . | | |
| 21-225_3B1_HC | . | | . | . | . | . | . | . | . | . | . | . | . | . | . | . | . | | |
| 21-225_3E3_HC | . | | . | . | . | . | . | . | . | . | . | . | . | . | . | . | . | | |
| 21-225_48C3_HC | . | | N | . | . | . | . | . | . | . | . | . | . | . | . | . | . | | |
| 21-225_48D1_HC | . | | N | . | . | . | . | . | . | . | . | . | . | . | . | . | . | | |
| 21-225_49G12_HC | . | | N | D | . | . | . | . | . | . | . | . | . | . | . | . | . | | |
| 21-225_4H2_HC | . | | . | . | . | . | . | . | . | . | . | . | . | . | . | . | . | | |
| 21-225_50H10_HC | . | | N | . | . | . | . | . | . | . | . | . | . | . | . | . | . | | |
| 21-225_50H8_HC | . | | N | . | . | . | . | . | . | . | . | . | . | . | . | . | . | | |
| 21-225_51H10_HC | . | | N | D | . | . | . | . | . | . | . | . | . | . | . | . | . | | |
| 21-225_5F7_HC | . | | . | . | . | . | A | . | . | . | . | . | . | . | . | . | . | | |
| 21-225_5G2_HC | | | . | . | . | . | . | . | . | . | . | . | . | . | . | . | . | | |

EP 4 435 105 A2

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | S | E | A | F | D | I | W | G | Q | G | T | M | V | T | V | S | S |
| 21-225_6D3_HC | . | . | . | . | . | . | . | . | . | . | . | . | . | . | . | . | . | . | . |
| 21-225_8E12_HC | . | . | . | . | . | . | . | . | . | . | . | . | . | . | . | . | . | . | . |
| 21-225_8G6_HC | . | . | . | . | . | . | . | . | . | . | . | . | . | . | . | S | . | . | . |

3878

**FIGURE 57**

Table 60.        Consensus 26- VH3|3-23/D5|5-12|RF3/JH6 (SEQ ID NO: 50277):

EVQLLES-GGGLVQPGGSLRLSCAASG-FTFS<u>S</u>-----<u>YAMS</u>WVRQAPGKGLEWVS<u>AISGR</u>---GGSTFHADSVKGRFTISRDNSKNTLYLQMNSLRAEDTAVYYCVK<u>GELLEDY</u>---------------YFYGMD<u>V</u>WGQGTTVTVSS wherein:

S at position 33 can be substituted with N

Y at position 39 can be substituted with C

A at position 40 can be substituted with V

S at position 42 can be substituted with N

A at position 57 can be substituted with T or S

I at position 58 can be substituted with L

G at position 60 can be substituted with R or V

R at position 61 can be substituted with G

S at position 67 can be substituted with N

T at position 68 can be substituted with I

F at position 69 can be substituted with Y

H at position 70 can be substituted with Y or N

V at position 74 can be substituted with E or M

G at position 109 can be substituted with W

E at position 110 can be substituted with G

E at position 113 can be substituted with Y

D at position 114 can be substituted with S or N

**FIGURE 57**

null (-) at position 116 can be substituted with E

Y at position 132 can be substituted with S

F at position 133 can be substituted with Y

Y at position 134 can be substituted with F

G at position 135 can be substituted with A

M at position 136 can be substituted with I or L

Xaa Xaa Xaa Met Xaa (SEQ ID NO: 50034)

Xaa Xaa Ser Xaa Xaa Gly Gly Xaa Xaa Xaa Xaa Ala Asp Ser Xaa Lys Gly (SEQ ID NO: 50035)

Xaa Xaa Leu Leu Xaa Xaa Tyr Xaa Xaa Xaa Xaa Xaa Xaa Asp Val (SEQ ID NO: 50036)

Ser Tyr Ala Met Ser (SEQ ID NO: 50501)

Ala Ile Ser Gly Arg Gly Gly Ser Thr Phe His Ala Asp Ser Val Lys Gly (SEQ ID NO: 50502)

Gly Glu Leu Leu Glu Asp Tyr Tyr Phe Tyr Gly Met Asp Val (SEQ ID NO: 50503)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_12D8_HC | . | . | . | . | . | . | . | . | . | . | . | . | . | . | . | . | . | . | . | . | . | . | . | . | . | . |
| 21-225_17F12_HC | . | . | . | . | . | . | . | . | . | . | . | . | . | . | . | . | . | . | . | . | . | . | . | . | . | . |
| 21-225_21H4_HC | . | . | . | . | . | . | . | . | . | . | . | . | . | . | . | . | . | . | . | . | . | . | . | . | . | . |
| 21-225_24B1_HC | . | . | . | . | . | . | . | . | . | . | . | . | . | . | . | . | . | . | . | . | . | . | . | . | . | . |
| 21-225_30D1_HC | . | . | . | V | . | . | . | . | . | . | . | . | . | . | . | . | . | . | . | . | . | . | . | . | . | . |
| 21-225_30E3_HC | . | . | . | V | . | . | . | . | . | . | . | . | . | . | . | . | . | . | . | . | . | . | . | . | . | . |
| 21-225_30E9_HC | . | . | . | V | . | . | . | . | . | . | . | . | . | . | . | . | . | . | . | . | . | . | . | . | . | . |
| 21-225_30G11_HC | . | . | . | . | . | . | . | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | . | V | . | . | . | . | . | . | . | . | . | . | . | . | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | A | . | . | . | . | . | . | . | . | . | . | . | . | . |
| 21-225_32G12_HC | . | . | . | . | . | . | . | . | . | . | . | . | L | . | . | . | . | . | . | . | . | . | . | . | . | . |
| 21-225_33A4_HC | . | . | . | . | . | . | . | . | . | . | . | . | . | . | S | . | . | . | . | . | . | . | . | S | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | L | . | . | . | . | . | . | . | . | . | . | . | . | . |
| 21-225_33G7_HC | . | . | . | . | . | . | . | . | . | . | . | . | . | . | . | . | . | . | . | . | . | . | . | . | . | . |
| 21-225_34A6_HC | . | . | . | . | . | . | . | . | . | . | . | . | . | . | . | . | . | . | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | V | . | . | . | . | . | . | . | . | . | . | . | . | . | . | . | . | . | . | . | . | . | . |
| 21-225_34H11_HC | . | . | . | . | . | . | . | . | . | . | . | . | . | . | . | . | . | . | . | . | . | . | . | . | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | . | . | . | . | . | . | . | . | . | . | . | . | . | . | . | . | . | . | . | . | . | . | . | . | . | . |
| 21-225_36C5_HC | . | . | . | V | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_4G12_HC | . | . | . | . | . | . | . | | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | A | M | S | W | V | R | Q | A | P | G | K | G | L |
| 21-225_12D8_HC | . | . | . | . | . | . | . | . | . | . | . | . | . | V | . | . | . | . | . | . | . | . | . | . | . | . |
| 21-225_17F12_HC | . | . | . | . | . | . | . | . | . | . | . | . | . | . | . | . | . | . | . | . | . | . | . | . | . | . |
| 21-225_21H4_HC | . | . | . | . | . | . | . | . | . | . | . | . | . | . | . | . | . | . | . | . | . | . | . | . | . | . |
| 21-225_24B1_HC | . | . | . | . | . | . | . | . | . | . | . | . | . | . | . | . | . | . | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | . | . | . | . | . | . | . | . | . | . | . | . | . | N | . | . | . | . | . | . | . | . | . | . |
| 21-225_30E9_HC | . | . | . | . | . | . | . | . | . | . | . | . | G | . | . | N | . | . | . | . | . | . | . | . | . | . |
| 21-225_30G11_HC | . | . | . | . | . | . | . | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | . | . | . | . | . | . | . | . | . | . | . | . | . | N | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | . | . | N | . | . | . | . | . | . | . | . | . | . | . | . | . | . | . | . | . | . | . | . |
| 21-225_33A4_HC | . | . | . | I | . | . | . | . | . | . | . | . | . | . | . | N | . | . | . | . | V | . | . | . | . | . |
| 21-225_33A7_HC | . | . | . | . | . | . | . | . | . | . | . | . | G | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . | . | . | . | . | . | . | . |
| 21-225_33G7_HC | . | . | . | . | . | . | N | . | . | . | . | . | . | . | . | . | . | . | . | . | . | . | . | . | . | . |
| 21-225_34A6_HC | . | . | . | . | N | N | . | . | . | . | . | . | . | . | . | N | . | . | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | . | . | . | . | . | . | . | . | . | . | . | . | . | N | . | . | . | . | . | . | . | E | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | G | . | . | . | N | N | . | . | . | . | . | . | . | . | . | N | . | . | . | . | . | . | . | . | . | . |
| 21-225_36C5_HC | . | . | . | . | . | . | . | . | . | . | . | . | . | . | . | N | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | H_CDR1 | | | | | | | | | | | | | | | | H_FR2 | | |
| CONSENSUS | G | - | F | T | F | S | S | - | - | - | - | - | Y | A | M | S | W | V | R | Q | A | P | G | K | G | L |
| 21-225_4G12_HC | . | . | . | . | . | . | . | | . | . | . | . | . | . | . | . | . | . | | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | V | S | A | I | S | G | R | - | - | - | G | G | S | T | F | H | A | D | S | V | K | G | R | F |
| 21-225_12D8_HC | . | . | . | . | T | . | . | V | G | . | . | . | . | . | . | . | Y | Y | . | . | . | . | . | . | . | . |
| 21-225_17F12_HC | . | . | . | . | . | . | . | . | G | . | . | . | . | . | . | . | Y | Y | . | . | . | . | . | . | . | V |
| 21-225_21H4_HC | . | . | . | . | . | L | . | . | . | . | . | . | . | . | . | . | Y | Y | . | . | . | . | . | . | . | . |
| 21-225_24B1_HC | . | . | . | . | . | . | . | . | . | . | . | . | . | . | . | . | Y | Y | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | . | . | . | . | . | . | . | . | . | . | . | . | . | . | . | Y | . | . | . | S | . | . | . | . |
| 21-225_30E9_HC | . | . | . | . | . | . | . | . | . | . | . | . | . | . | . | . | . | Y | . | . | . | . | . | . | . | . |
| 21-225_30G11_HC | . | . | . | . | . | . | . | . | . | . | . | . | . | . | . | . | . | N | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | . | . | . | . | . | . | . | . | . | . | . | . | N | . | . | Y | . | . | . | . | . | . | . | . |
| 21-225_31H5_HC | . | . | . | . | . | . | . | . | . | . | . | . | . | . | . | I | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | S | . | . | . | . | . | . | . | . | . | . | . | . | . | . | . | . | . | . | . | . | . | . |
| 21-225_33A4_HC | . | . | . | . | . | . | . | R | . | . | . | . | . | . | . | . | . | Y | . | . | . | . | . | . | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | S | . | . | . | . | . | . | . | . | . | . | . | . | . | . | . | . | . | . | . | . | . | . |
| 21-225_33G7_HC | . | . | . | . | . | . | . | R | . | . | . | . | . | . | . | . | . | Y | . | . | . | . | . | . | Q | . |
| 21-225_34A6_HC | . | . | . | . | . | . | . | R | . | . | . | . | . | . | . | . | . | Y | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | . | . | . | . | . | . | . | . | . | . | . | . | . | . | . | Y | . | . | . | . | . | . | . | . |
| 21-225_35A6_HC | . | . | . | . | T | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | N | . | . | . | . |
| 21-225_36A5_HC | E | . | . | . | . | . | R | . | . | . | . | . | . | N | . | . | . | Y | . | . | . | . | . | . | . | . |
| 21-225_36C5_HC | . | . | . | . | . | . | . | . | . | . | . | . | . | . | . | . | . | . | . | . | . | . | . | . | . | . |

3885

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | A | I | S | G | R | - | - | - | G | G | S | T | F | H | A | D | S | V | K | G | R | F |
| 21-225_4G12_HC | . | . | . | . | Y | L | . | . | . | . | . | . | . | . | . | . | Y | Y | . | . | . | . | . | . | . | S |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FRS | | | | | | | | | | | | |
| CONSENSUS | T | I | S | R | D | N | S | K | N | T | L | Y | L | Q | M | N | S | L | R | A | E | D | T | A | V | Y |
| 21-225_12D8_HC | . | . | . | . | . | . | . | . | T | . | . | . | . | . | . | . | . | . | . | . | . | . | . | . | . | . |
| 21-225_17F12_HC | . | . | . | . | . | . | . | . | . | . | . | . | . | . | . | . | . | . | . | . | . | . | . | . | . | . |
| 21-225_21H4_HC | . | . | . | . | . | . | . | . | . | . | . | . | . | . | . | . | . | . | . | . | . | . | . | . | . | . |
| 21-225_24B1_HC | . | . | . | . | . | . | . | . | . | . | . | . | . | . | . | . | . | . | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | . | . | . | . | . | . | . | . | . | . | . | . | . | . | . | . | . | . | . | . | . | . | L | . |
| 21-225_30E9_HC | . | . | . | . | . | . | . | . | . | . | . | . | . | . | . | . | . | . | . | . | . | . | . | . | . | . |
| 21-225_30G11_HC | . | . | . | . | . | . | . | . | T | . | . | . | . | . | . | . | . | . | . | . | . | . | . | . | . | . |
| 21-225_31A8_HC | . | . | . | . | . | . | . | . | . | . | . | . | . | . | . | . | . | . | . | O | . | . | . | . | . | . |
| 21-225_31B8_HC | . | . | . | . | . | . | . | . | . | . | . | . | . | L | . | . | . | . | . | . | O | . | . | . | . | . |
| 21-225_31H3_HC | . | . | . | . | . | . | . | . | . | . | . | . | . | . | . | . | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | . | . | . | . | S | . | . | . | . | . | . | . | . | . | . | . | . | . | . | . | . | . | . |
| 21-225_33A4_HC | . | . | . | . | . | . | . | . | . | . | . | . | . | . | . | . | . | . | . | . | . | . | . | . | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . | . | . | . | . | . | . | . |
| 21-225_33G7_HC | . | . | . | . | . | . | . | . | . | . | . | . | . | . | . | . | . | . | . | . | . | . | . | . | . | . |
| 21-225_34A6_HC | . | . | . | . | . | . | . | . | . | . | . | . | . | . | . | . | . | . | . | . | . | . | . | . | . | . |
| 21-225_34G8_HC | . | . | . | . | . | . | . | . | . | . | . | . | . | . | I | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | . | . | . | . | . | . | . | . | . | P | . | . | . | . | . | . | . | . | . | . | . | . | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | N | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | . | . | . | . | . | . | . | . | . | . | . | . | . | . | . | . | . | . | . | . | . | . | . | . | . | . |
| 21-225_36C5_HC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | | |
| CONSENSUS | T | I | S | R | D | N | S | K | N | T | L | Y | L | Q | M | N | S | L | R | A | E | D | T | A | V | Y |
| 21-225_4G12_HC | . | . | . | . | . | | | | . | . | . | . | . | . | S | . | . | | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | V | K | G | E | L | L | E | D | Y | - | - | - | - | - | - | - | - | - | - | - | - | - | - | - |
| 21-225_12D8_HC | . | . | A | . | W | G | R | G | Y | S | . | E | . | . | . | . | . | . | . | . | . | . | . | . | . | . |
| 21-225_17F12_HC | . | . | A | . | W | G | R | G | Y | N | . | E | . | . | . | . | . | . | . | . | . | . | . | . | . | . |
| 21-225_21H4_HC | . | . | A | . | W | G | R | G | Y | S | . | E | . | . | . | . | . | . | . | . | . | . | . | . | . | . |
| 21-225_24B1_HC | . | . | A | . | W | G | R | G | Y | S | . | E | . | . | . | . | . | . | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | A | . | . | . | . | . | . | . | . | . | . | . | . | . | . | . | . | . | . | . | . | . | . | . |
| 21-225_30E9_HC | . | . | A | . | . | . | . | . | . | . | . | . | . | . | . | . | . | . | . | . | . | . | . | . | . | . |
| 21-225_30G11_HC | . | . | . | . | . | . | . | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | A | . | . | . | . | . | . | . | . | . | . | . | . | . | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | . | . | . | . | . | . | . | . | . | . | . | . | . | . | . | . | . | . | . | . | . | . | . |
| 21-225_33A4_HC | F | . | A | . | . | . | . | . | . | . | . | . | . | . | . | . | . | . | . | . | . | . | . | . | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . | . | . | . | . | . | . | . |
| 21-225_33G7_HC | . | . | A | . | . | . | . | . | . | . | . | . | . | . | . | . | . | . | . | . | . | . | . | . | . | . |
| 21-225_34A6_HC | . | . | A | . | . | . | . | . | . | . | . | . | . | . | . | . | . | . | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | A | . | . | . | . | . | . | . | . | . | . | . | . | . | . | . | . | . | . | . | . | . | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | . | . | A | . | . | . | . | . | . | . | . | . | . | . | . | . | . | . | . | . | . | . | . | . | . | . |
| 21-225_36C5_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
|  |  |  |  |  |  |  |  |  |  |  |  |  |  |  |  |  |  |  | H_CDR3 |  |  |  |  |  |  |  |
| CONSENSUS | Y | C | V | K | G | E | L | L | E | D | Y | - | - | - | - | - | - | - | - | - | - | - | - | - | - | - |
| 21-225_4G12_HC | . | . | A | . | W | G | R | G | Y | S | . | E | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | Y | F | Y | G | M | D | V | W | G | Q | G | T | T | V | T | V | S | S |
| 21-225_12D8_HC | . | Y | . | . | . | . | . | . | . | . | . | . | . | . | . | . | . | . | . |
| 21-225_17F12_HC | . | Y | . | . | . | . | . | . | . | . | . | . | . | . | . | . | . | . | . |
| 21-225_21H4_HC | . | Y | . | . | . | . | . | . | . | . | . | . | . | . | . | . | . | . | . |
| 21-225_24B1_HC | . | Y | . | . | . | . | . | . | . | . | . | . | . | . | . | . | . | . | . |
| 21-225_30D1_HC | . | . | . | . | L | . | . | . | . | . | . | . | . | . | . | . | . | . | . |
| 21-225_30E3_HC | . | Y | . | . | . | . | . | . | . | . | . | . | . | . | . | . | . | . | . |
| 21-225_30E9_HC | . | Y | . | . | . | . | . | . | . | . | . | . | . | . | . | . | . | . | . |
| 21-225_30G11_HC | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . |
| 21-225_31H3_HC | . | Y | . | . | . | . | . | . | . | . | . | . | . | . | . | . | . | . | . |
| 21-225_31H5_HC | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . |
| 21-225_32G12_HC | . | . | . | . | . | . | . | . | . | . | . | . | . | . | . | . | . | . | . |
| 21-225_33A4_HC | . | S | Y | . | . | I | . | . | . | . | . | . | . | . | . | . | . | . | . |
| 21-225_33A7_HC | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . |
| 21-225_33G7_HC | . | S | Y | . | . | . | . | . | . | . | . | . | . | . | . | . | . | . | . |
| 21-225_34A6_HC | . | . | Y | F | A | . | . | . | . | . | . | . | . | . | . | . | . | . | . |
| 21-225_34G8_HC | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . |
| 21-225_34H11_HC | . | . | Y | . | . | . | . | . | . | . | . | . | . | . | . | . | . | . | . |
| 21-225_35A6_HC | . | . | . | . | . | . | . | . | . | . | . | . | . | . | . | . | . | . | . |
| 21-225_35B7_HC | . | . | . | . | . | . | . | . | . | . | . | . | A | . | . | . | . | . | . |
| 21-225_35D1_HC | . | . | . | . | . | . | . | . | . | . | . | . | A | . | . | . | . | . | . |
| 21-225_35F11_HC | . | . | . | . | . | . | . | . | . | . | . | . | . | . | . | . | . | . | . |
| 21-225_36A5_HC | . | . | Y | . | A | . | . | . | . | . | . | . | . | . | . | . | . | . | . |
| 21-225_36C5_HC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | Y | F | Y | G | M | D | V | W | G | Q | G | T | T | V | T | V | S | S |
| 21-225_4G12_HC | . | . | Y | . | . | . | . | . | . | . | . | . | . | . | . | . | . | . | . |

**FIGURE 57**

Table 61.     Consensus 27- VH3|3-33/D4|4-23 RF2/JH6 (SEQ ID NO: 50278):

QVQLVES-GGGVVQPGRSLRLSCAASG-FTFSS-----YGMHWVRQAPGKGLEWVAVIWYD---
GSNKYYADSVKGRFTISRDNSKNTLYLQMNSLRAEDTAVYYCARDRVYCSSTSC----------PYYYYYGMDVWGQGTTVTVSS wherein:

S in position 33 can be substituted with G, D, R, H, T or N

G in position 40 can be substituted with V

M in position 41 can be substituted with L

H in position 42 can be substituted with N

V in position 57 can be substituted with L, F, I or D

I in position 58 can be substituted with F

W in position 59 can be substituted with R

Y in position 60 can be substituted with F

S in position 66 can be substituted with N or T

N in position 67 can be substituted with E, D or K

K in position 68 can be substituted with N or T

Y in position 69 can be substituted with S, D or N

Y in position 70 can be substituted with N

A in position 71 can be substituted with V

G in position 76 can be substituted with D

R in position 110 can be substituted with D, W or N

V in position 111 can be substituted with D, R, F or H

FIGURE 57

Y in position 112 can be substituted with S, E, G or F

C in position 113 can be substituted with G or E

S in position 114 can be substituted with G

S in position 115 can be substituted with G, R or N

T in position 116 can be substituted with null (-), S or P

S in position 117 can be substituted with P, null (-) or T

C in position 118 can be substituted with null (-)

null (-) in position 119 can be substituted with S, H, L or Y

P in position 129 can be substituted with null (-), S or Y

Y in position 130 can be substituted with null (-)

Y in position 131 can be substituted with null (-)

Y in position 132 can be substituted with null (-)

Y in position 134 can be substituted with F

G in position 135 can be substituted with A

M in position 136 can be substituted with L

Xaa Tyr Xaa Xaa Xaa (SEQ ID NO: 50037)

Xaa Xaa Xaa Xaa Asp Gly Xaa Xaa Xaa Xaa Xaa Xaa Asp Ser Val Lys Xaa (SEQ ID NO: 50038)

Asp Xaa Xaa Xaa Xaa Xaa Xaa Xaa Xaa Xaa Xaa Xaa Xaa Xaa Xaa Xaa Tyr Xaa Xaa Asp Val (SEQ ID NO: 50039)

Ser Tyr Gly Met His (SEQ ID NO: 50504)

**FIGURE 57**

Val Ile Trp Tyr Asp Gly Ser Asn Lys Tyr Tyr Ala Asp Ser Val Lys Gly (SEQ ID NO: 50505)

Asp Arg Val Tyr Cys Ser Ser Thr Ser Cys Pro Tyr Tyr Tyr Tyr Gly Met Asp Val (SEQ ID NO: 50506)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_146A10_HC | | | | | E | | | | | | | | | | | | | | | | | | | | | |
| 21-225_147D9_HC | | | | | | | | | | | | | | | | | | | | | | | | | | |
| 21-225_148E10_HC | | | | | | | | | | | | | | | | | | | | | | | | | | |
| 21-225_149F2_HC | | | | | G | | A | | | | | | | | | | | | | | | | | | | |
| 21-225_150F11_HC | | | | | G | | A | | | | | | | | | | | | | | | | | | | |
| 21-225_151H6_HC | | | | | | | | | | | | | | | | | | | | | | | | | | |
| 21-225_152B12_HC | | | | V | | | | | | | | | | | | | | | | | | | | | | |
| 21-225_152G5_HC | | | | | | | | | | | | | | | | | | | | | | | | | | |
| 21-225_153A8_HC | | | | | M | | | | | | | | | | | | | | | | | | | | | |
| 21-225_153E8_HC | | | | | | | | | | | | | | | | | | | | | | | | | | |
| 21-225_158D10_HC | | | | | | | | | | | | | | | | | | | | | | | | | | |
| 21-225_159H8_HC | | | | | | | | | | | | | | | | | | | | | | | | | | |
| 21-225_160B12_HC | | | | | | | | | | | | | | | | | | | | | | | | | | |
| 21-225_166G11_HC | | | | | | | | | | | | | | | | | | | | | | | | | | |
| 21-225_170F1_HC | | | | | | | | | | | | | | | | | | | | | | | | | | |
| 21-225_195D9_HC | | | | | | | | | | | | | | | | | | | | | | | | | | |
| 21-225_196A10_HC | | | | | | | | | | | | | | | | | | | | | | | | | | |
| 21-225_225A8_HC | | | | | | | | | | | | | | | | | G | | | | | | | | | |
| 21-225_27G6_HC | | | | | | | | | | | | | | | | | | | | | | | | | | |
| 21-225_27H2_HC | | | | | | | | | | | | | | | | | | | | | | | | | | |
| 21-225_28A11_HC | | | | | | | | | | | | | | | | | | | | | | | | | | |
| 21-225_52H1_HC | | | | | | | | | | | | | | | | | | | | | | | | | | |
| 21-225_74B3_HC | | | | | | | | | | | | | | | | | | | | | | | | | | |
| 21-225_74C9_HC | | | | | | | | | | | | | | | | | | | | | | | | | | |
| 21-225_78E9_HC | | | | | | | | | | | | | | | | | | | | | | | | | | |
| 21-225_79E7_HC | | | | | | | | | | | | | | | | | | | | | | | | | | |
| 21-225_8C12_HC | | | | | | | | | | | | | | | | | | | | | | | | | | |
| 21-225_92A4_HC | | | | | | | | | | | | | | | | | | | | | | | | | | |
| 21-225_97E6_HC | | | | | | | | | | | | | | | | | | | | | | | | | | |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | F | T | F | S | S | - | - | - | - | - | Y | G | M | H | W | V | R | Q | A | P | G | K | G | L |
| 21-225_146A10_HC | . | . | . | . | . | . | G | . | . | . | . | . | . | . | . | . | . | . | . | . | . | . | . | . | . | . |
| 21-225_147D9_HC | . | . | . | . | . | . | . | . | . | . | . | . | . | . | . | . | . | . | . | . | . | . | . | . | . | . |
| 21-225_148E10_HC | . | . | . | . | . | . | . | . | . | . | . | . | . | . | . | . | . | . | . | . | . | . | . | . | . | . |
| 21-225_149F2_HC | . | . | . | . | . | . | R | . | . | . | . | . | . | . | . | . | . | . | . | . | . | . | . | . | . | . |
| 21-225_150F11_HC | . | . | . | . | . | . | R | . | . | . | . | . | . | . | . | . | . | . | . | . | . | . | . | . | . | . |
| 21-225_151H6_HC | . | . | . | . | . | . | D | . | . | . | . | . | . | . | . | . | . | . | . | . | . | . | . | . | . | . |
| 21-225_152B12_HC | . | . | . | . | . | . | D | . | . | . | . | . | . | . | . | . | . | . | . | . | . | . | . | . | . | . |
| 21-225_152G5_HC | . | . | . | . | . | . | D | . | . | . | . | . | . | . | . | . | . | . | . | . | . | . | . | . | . | . |
| 21-225_153A8_HC | . | . | . | . | . | . | D | . | . | . | . | . | . | . | . | . | . | . | . | . | . | . | . | . | . | . |
| 21-225_153E8_HC | . | . | . | . | L | . | N | . | . | . | . | . | . | . | . | . | . | . | . | . | . | . | . | . | . | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | T | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | . | N | G | . | . | . | . | . | . | . | . | . | . | . | . | . | . | . | . | . | . | . |
| 21-225_170F1_HC | . | . | . | . | . | . | G | . | . | . | . | . | . | . | . | N | . | . | . | . | . | . | . | . | . | . |
| 21-225_195D9_HC | . | . | . | . | . | . | R | . | . | . | . | . | . | V | . | . | . | . | . | . | . | . | . | . | . | . |
| 21-225_196A10_HC | . | . | . | . | . | . | R | . | . | . | . | . | . | V | . | . | . | . | . | . | . | . | . | . | . | . |
| 21-225_225A8_HC | . | . | . | . | . | . | T | . | . | . | . | . | . | . | . | . | . | . | . | L | . | . | . | . | . | . |
| 21-225_27G6_HC | . | . | . | . | . | . | G | . | . | . | . | . | . | . | . | . | . | . | . | . | . | . | . | . | . | . |
| 21-225_27H2_HC | . | . | . | . | . | . | G | . | . | . | . | . | . | . | . | . | . | . | . | . | . | . | . | . | . | . |
| 21-225_28A11_HC | . | . | . | . | . | . | G | . | . | . | . | . | . | . | L | . | . | . | . | . | . | . | . | . | . | . |
| 21-225_52H1_HC | . | . | . | . | . | . | G | . | . | . | . | . | . | . | . | . | . | . | . | . | . | . | . | . | . | . |
| 21-225_74B3_HC | . | . | . | . | . | . | R | . | . | . | . | . | . | . | . | . | . | . | . | . | . | . | . | . | . | . |
| 21-225_74C9_HC | . | . | . | . | . | . | . | . | . | . | . | . | . | . | . | . | . | . | . | . | . | . | . | . | . | . |
| 21-225_78E9_HC | . | . | . | . | . | . | . | . | . | . | . | . | . | . | . | . | . | . | . | . | . | . | . | . | . | . |
| 21-225_79E7_HC | . | . | . | . | . | . | . | . | . | . | . | . | . | . | . | . | . | . | . | . | . | . | . | . | . | . |
| 21-225_8C12_HC | . | . | . | . | . | G | . | . | . | . | . | . | . | . | . | . | . | . | . | . | . | . | . | . | . | . |
| 21-225_92A4_HC | . | . | . | . | . | . | . | . | . | . | . | . | . | . | . | . | . | . | . | . | . | . | . | . | . | . |
| 21-225_97E6_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | H_CDR2 | | | | | | | | | | | | | |
| CONSENSUS | E | W | V | A | V | I | W | Y | D | - | - | - | G | S | N | K | Y | Y | A | D | S | V | K | G | R | F |
| 21-225_146A10_HC | . | . | M | T | L | . | W | . | . | . | . | . | . | . | D | . | N | . | . | . | . | . | . | . | . | . |
| 21-225_147D9_HC | . | . | . | . | D | . | . | F | . | . | . | . | . | . | . | . | . | V | . | . | . | . | . | D | . | . |
| 21-225_148E10_HC | . | . | . | . | . | . | . | F | . | . | . | . | . | . | . | . | . | V | . | . | . | . | . | D | . | . |
| 21-225_149F2_HC | . | . | . | . | F | . | . | . | . | . | . | . | . | E | N | . | . | . | . | . | . | . | . | . | . | . |
| 21-225_150F11_HC | . | . | . | . | F | . | . | . | . | . | . | . | . | E | N | . | . | . | . | . | . | . | . | . | . | . |
| 21-225_151H6_HC | . | . | . | . | L | . | . | . | . | . | . | . | . | . | . | . | . | . | . | . | . | . | . | . | . | . |
| 21-225_152B12_HC | . | . | . | . | . | . | . | . | . | . | . | . | . | . | . | . | . | . | . | . | . | . | . | . | . | . |
| 21-225_152G5_HC | . | . | . | . | L | . | . | . | . | . | . | . | . | . | . | . | . | . | . | . | . | . | . | . | . | . |
| 21-225_153A8_HC | . | . | . | . | L | . | . | . | . | . | . | . | . | . | . | . | . | . | . | . | . | . | . | . | . | . |
| 21-225_153E8_HC | . | . | . | . | . | . | . | F | . | . | . | . | . | . | . | . | . | V | . | . | . | . | . | D | . | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | . | . | . | . | . | . | . | . | . | . | . | . | . | . | . | . | . | . | . | . | . | . |
| 21-225_170F1_HC | . | . | . | . | I | . | . | . | . | . | . | . | . | N | . | . | . | . | . | . | . | . | . | . | . | . |
| 21-225_195D9_HC | . | . | . | . | . | . | . | . | . | . | . | . | . | T | . | . | . | . | . | . | . | . | . | . | . | . |
| 21-225_196A10_HC | . | . | . | . | . | . | . | . | . | . | . | . | . | T | . | . | . | . | . | . | . | . | . | . | . | . |
| 21-225_225A9_HC | . | . | . | . | . | . | . | . | . | . | . | . | . | . | . | . | . | . | . | . | . | . | . | . | . | . |
| 21-225_27G6_HC | . | . | . | . | I | . | . | . | . | . | . | . | . | . | . | . | N | . | . | . | . | . | . | . | . | . |
| 21-225_27H2_HC | . | . | . | . | . | . | . | . | . | . | . | . | . | . | . | . | . | . | . | . | . | . | . | . | . | . |
| 21-225_28A11_HC | . | . | . | . | L | . | . | . | . | . | . | . | . | N | . | T | . | . | . | . | . | . | . | . | . | . |
| 21-225_52H1_HC | . | . | . | . | . | . | . | . | . | . | . | . | . | . | . | . | . | . | . | . | . | . | . | . | . | . |
| 21-225_74B3_HC | . | . | . | . | . | F | . | . | . | . | . | . | . | . | . | D | . | . | . | . | . | . | . | . | . | . |
| 21-225_74C9_HC | . | . | . | . | . | . | . | . | . | . | . | . | . | N | . | S | . | . | . | . | . | . | . | . | . | . |
| 21-225_78E9_HC | . | . | . | . | . | . | . | . | . | . | . | . | . | N | . | S | . | . | . | . | . | . | . | . | . | . |
| 21-225_79E7_HC | . | . | . | . | . | . | . | . | . | . | . | . | . | N | . | S | . | . | . | . | . | . | . | . | . | . |
| 21-225_8C12_HC | . | . | . | . | L | . | . | . | . | . | . | . | . | . | . | . | . | . | . | . | . | . | . | . | . | . |
| 21-225_92A4_HC | . | . | . | . | . | . | . | . | . | . | . | . | . | N | . | S | . | . | . | . | . | . | . | . | . | . |
| 21-225_97E6_HC | . | . | . | . | . | . | . | . | . | . | . | . | . | N | . | S | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_146A10_HC | . | . | . | . | . | . | . | . | . | . | . | . | . | . | . | . | . | . | . | . | . | . | . | . | . | . |
| 21-225_147D9_HC | . | . | . | . | . | . | . | . | . | . | . | . | . | . | . | . | . | . | . | . | . | . | . | . | . | . |
| 21-225_148E10_HC | . | . | . | . | . | . | . | . | . | . | . | . | . | . | . | . | . | . | . | . | . | . | . | . | . | . |
| 21-225_149F2_HC | . | . | . | . | V | . | . | R | . | . | . | . | . | . | . | . | . | . | . | . | . | . | . | . | . | . |
| 21-225_150F11_HC | . | . | . | . | V | . | . | R | . | . | . | . | . | . | . | . | . | . | . | . | . | . | . | . | . | . |
| 21-225_151H6_HC | . | . | . | . | . | . | . | . | . | . | . | . | . | . | . | . | . | . | . | . | . | . | . | . | . | . |
| 21-225_152B12_HC | . | . | . | . | . | . | . | . | . | . | . | . | . | . | . | . | . | . | . | . | . | . | . | . | . | . |
| 21-225_152G5_HC | . | . | . | . | . | . | . | . | . | . | . | . | . | . | . | . | . | . | . | . | . | . | . | . | . | . |
| 21-225_153A8_HC | . | . | . | . | . | . | . | . | . | . | . | . | . | . | . | . | . | . | . | . | . | . | . | . | . | . |
| 21-225_153E8_HC | . | . | . | . | . | . | . | . | . | . | . | . | . | . | . | . | . | . | . | . | . | . | . | . | . | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | T | . | . | . | . | . | . | P | . | . | . | . | . | . | Y | . | . | . | . | . | . | . |
| 21-225_170F1_HC | . | . | . | . | . | . | . | . | . | . | . | . | . | . | . | . | . | . | . | . | . | . | . | . | . | . |
| 21-225_195D9_HC | . | . | . | . | . | . | . | . | . | . | . | . | . | . | . | . | . | . | . | . | . | . | . | . | . | . |
| 21-225_196A10_HC | . | . | . | . | . | . | . | . | . | . | . | . | . | . | . | . | . | . | . | . | . | . | . | . | . | . |
| 21-225_225A8_HC | . | . | . | . | I | . | . | Q | . | . | . | . | . | . | . | . | . | . | . | . | . | . | . | . | . | . |
| 21-225_27G6_HC | . | . | . | . | . | . | . | . | . | . | . | . | . | . | . | . | . | . | . | . | . | . | . | . | . | . |
| 21-225_27H2_HC | . | . | . | . | . | . | . | . | . | . | . | P | . | . | . | . | . | . | . | . | . | . | . | . | . | . |
| 21-225_28A11_HC | . | . | . | . | . | . | . | . | . | . | . | . | . | . | . | . | . | . | . | . | . | . | . | . | . | . |
| 21-225_52H1_HC | . | . | . | . | . | . | . | . | . | . | . | . | . | . | . | . | . | . | . | . | . | . | . | . | . | . |
| 21-225_74B3_HC | . | . | . | . | . | . | . | . | . | . | . | . | . | . | . | . | . | . | . | . | . | . | . | . | . | . |
| 21-225_74C9_HC | . | . | . | . | I | . | . | . | . | . | . | . | . | . | . | . | . | . | . | . | . | . | . | . | . | . |
| 21-225_78E9_HC | . | . | . | . | I | . | . | . | . | . | . | . | . | . | . | . | . | . | . | . | . | . | . | . | . | . |
| 21-225_79E7_HC | . | . | . | . | I | . | . | . | . | . | . | . | . | . | . | . | . | . | . | . | . | . | . | . | . | . |
| 21-225_8C12_HC | . | . | . | . | I | . | . | . | . | . | . | . | . | . | . | . | . | . | . | . | . | . | . | . | . | . |
| 21-225_92A4_HC | . | . | Y | . | I | . | . | . | . | . | . | . | . | . | . | . | . | . | . | . | . | . | . | . | . | . |
| 21-225_97E6_HC | . | . | . | . | I | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | V | Y | C | S | S | T | S | C | - | - | - | - | - | - | - | - | - | - | P | Y |
| 21-225_146A10_HC | . | . | . | . | . | . | . | F | . | . | . | . | . | . | L | . | . | . | . | . | . | . | . | . | S | . |
| 21-225_147D9_HC | . | . | . | . | . | . | . | E | G | . | G | . | P | - | . | . | . | . | . | . | . | . | . | . | - | - |
| 21-225_148E10_HC | . | . | . | . | . | . | . | E | G | . | G | . | P | - | . | . | . | . | . | . | . | . | . | . | - | - |
| 21-225_149F2_HC | . | . | . | . | . | . | . | E | G | . | G | . | P | - | . | . | . | . | . | . | . | . | . | . | - | - |
| 21-225_150F11_HC | . | . | . | . | . | . | . | E | G | . | G | . | P | - | . | . | . | . | . | . | . | . | . | . | - | - |
| 21-225_151H6_HC | . | . | . | . | . | D | R | S | . | . | . | R | . | . | . | . | . | . | . | . | . | . | . | . | . | . |
| 21-225_152B12_HC | . | . | . | . | . | D | R | S | . | . | . | . | . | . | . | . | . | . | . | . | . | . | . | . | . | . |
| 21-225_152G5_HC | . | . | . | . | . | D | R | S | . | . | . | R | . | . | . | . | . | . | . | . | . | . | . | . | . | . |
| 21-225_153A8_HC | . | . | . | . | . | D | R | S | . | . | . | R | . | . | . | . | . | . | . | . | . | . | . | . | . | . |
| 21-225_153E8_HC | . | . | . | . | . | . | . | E | G | . | G | . | P | - | . | . | . | . | . | . | . | . | . | . | - | - |
| 21-225_158D10_HC | . | . | . | . | . | . | . | S | . | . | . | . | . | . | . | . | . | . | . | . | . | . | . | . | . | . |
| 21-225_159H8_HC | . | . | . | . | . | . | . | S | . | . | . | . | . | . | . | . | . | . | . | . | . | . | . | . | . | . |
| 21-225_160B12_HC | . | . | . | . | . | . | . | S | . | . | . | . | . | . | . | . | . | . | . | . | . | . | . | . | . | . |
| 21-225_166G11_HC | . | . | . | . | . | D | R | . | . | . | . | P | T | . | M | . | . | . | . | . | . | . | . | . | . | . |
| 21-225_170F1_HC | . | . | . | . | . | . | . | . | . | . | . | . | . | . | M | . | . | . | . | . | . | . | . | . | . | . |
| 21-225_195D9_HC | . | . | . | . | . | W | F | G | E | G | N | - | - | - | . | . | . | . | . | . | . | . | . | . | - | - |
| 21-225_196A10_HC | . | . | . | . | . | W | F | G | E | G | N | - | - | - | . | . | . | . | . | . | . | . | . | . | - | - |
| 21-225_225A9_HC | . | . | . | . | . | N | H | . | . | . | . | . | . | . | S | . | . | . | . | . | . | . | . | . | . | . |
| 21-225_27G6_HC | . | . | . | . | . | . | . | . | . | . | . | . | . | . | S | . | . | . | . | . | . | . | . | . | . | . |
| 21-225_27H2_HC | . | . | . | . | . | . | . | . | . | . | . | . | . | . | S | . | . | . | . | . | . | . | . | . | . | . |
| 21-225_28A11_HC | . | . | . | . | . | . | . | . | . | . | . | . | . | . | S | . | . | . | . | . | . | . | . | . | . | . |
| 21-225_52H1_HC | . | . | . | . | . | . | . | . | . | . | . | S | . | . | S | . | . | . | . | . | . | . | . | . | Y | . |
| 21-225_74B3_HC | . | . | . | . | . | D | . | . | . | . | . | . | . | . | . | . | . | . | . | . | . | . | . | . | . | . |
| 21-225_74C9_HC | . | . | . | . | . | D | . | . | . | . | . | . | . | . | . | . | . | . | . | . | . | . | . | . | . | . |
| 21-225_78E9_HC | . | . | . | . | . | D | . | . | . | . | . | . | . | . | . | . | . | . | . | . | . | . | . | . | . | . |
| 21-225_79E7_HC | . | . | . | . | . | D | . | . | . | . | . | . | . | . | . | . | . | . | . | . | . | . | . | . | . | . |
| 21-225_8C12_HC | . | . | . | . | . | . | . | S | . | . | . | S | . | . | Y | . | . | . | . | . | . | . | . | . | . | . |
| 21-225_92A4_HC | . | . | . | . | . | D | . | . | . | . | . | . | . | . | . | . | . | . | . | . | . | . | . | . | . | . |
| 21-225_97E6_HC | . | . | . | . | . | D | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | H_FR4 | | | | | | | |
| CONSENSUS | Y | Y | Y | Y | G | M | D | V | W | G | Q | G | T | T | V | T | V | S | S |
| 21-225_146A10_HC | | | | | | | | | | | | | | | | | | | |
| 21-225_147D9_HC | | | | | | | | | | | | | | | | | | | |
| 21-225_148E10_HC | | | | | | | | | | | | | | | | | | | |
| 21-225_149F2_HC | | | | | | | | | | | | | | | | | | | |
| 21-225_150F11_HC | | | | | | | | | | | | | | | | | | | |
| 21-225_151H6_HC | | | | | | L | | | | | | | | | | | | | |
| 21-225_152B12_HC | | | | | | L | | | | | | | | | | | | | |
| 21-225_152G5_HC | | | | | | L | | | | | | | | | | | | | |
| 21-225_153A8_HC | | | | | | L | | | | | | | | | | | | | |
| 21-225_153E8_HC | | | | | | | | | | | | | | | | | | | |
| 21-225_158D10_HC | | | | | | | | | | | | | | | | | | | |
| 21-225_159H8_HC | | | | | | | | | | | | | | | | | | | |
| 21-225_160B12_HC | | | | | | | | | | | | | | | | | | | |
| 21-225_166G11_HC | | | | | | L | | | | | | | | | | | | | |
| 21-225_170F1_HC | | | | A | | | | | | | | | | | | | | | |
| 21-225_195D9_HC | - | - | | | | | | | | | | | | | | | | | |
| 21-225_196A10_HC | - | - | | | | | | | | | | | | | | | | | |
| 21-225_225A9_HC | | | | | | | | | | | | | | | | | | | |
| 21-225_27G6_HC | | | | | | | | | | | | | | | | | | | |
| 21-225_27H2_HC | | | | | | | | | | | | | | | | | | | |
| 21-225_28A11_HC | | | | | | | | | | | | | | | | | | | |
| 21-225_52H1_HC | | | | | | | | | | | | | | | | | | | |
| 21-225_74B3_HC | | | | | | | | | | | | | | | | | | | |
| 21-225_74C9_HC | | | | | | | | | | | | | | | | | | | |
| 21-225_78E9_HC | | | | | | | | | | | | | | | | | | | |
| 21-225_79E7_HC | | | | | | | | | | | | | | | | | | | |
| 21-225_8C12_HC | | | | | | | | | | | | | | | | | | | |
| 21-225_92A4_HC | | | | | | | | | | | | | | | | | | | |
| 21-225_97E6_HC | | | | | | | | | | | | | | | | | | | |

**FIGURE 57**

Table 62.    Consensus 28- VH3|3-21/D1|1-1|RF2/JH4 (SEQ ID NO: 50279):

EVQLVES-GGGLVKPGGSLRLSCAASG-FTFSS-----YSMNWVRQAPGKGLEWVSSISGS---SSYIYYADSVKGRFTISRDNAKNSLYLQMNSLRAEDTAVYYCARVAS----------------------FDYWGQGTLVTVSS wherein:

S at position 33 can be substituted with N

S at position 40 can be substituted with T, K, N or R

M at position 41 can be substituted with L

I at position 58 can be substituted with T

G at position 60 can be substituted with S

S at position 61 can be substituted with G, N or T

S at position 65 can be substituted with G, D or N

S at position 66 can be substituted with T

Y at position 67 can be substituted with F, D, L, N or S

I at position 68 can be substituted with M or T

Y at position 69 can be substituted with N

A at position 71 can be substituted with T

A at position 110 can be substituted with N or S

S at position 111 can be substituted with A, G, T, L, H or N

F at position 136 can be substituted with L or N

Y at position 138 can be substituted with C or S

Xaa Tyr Xaa Xaa Asn (SEQ ID NO: 50040)

**FIGURE 57**

Ser Xaa Ser Xaa Xaa Xaa Xaa Xaa Xaa Xaa Tyr Xaa Asp Ser Val Lys Gly (SEQ ID NO: 50041)

Val Xaa Xaa Xaa Asp Xaa (SEQ ID NO: 50042)

Ser Tyr Ser Met Asn (SEQ ID NO: 50507)

Ser Ile Ser Gly Ser Ser Ser Tyr Ile Tyr Tyr Ala Asp Ser Val Lys Gly (SEQ ID NO: 50508)

Val Ala Ser Phe Asp Tyr (SEQ ID NO: 50509)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | V | E | S | - | G | G | G | L | V | K | P | G | G | S | L | R | L | S | C | A | A | S |
| 21-225_14D6_HC | . | . | . | . | . | . | . | . | . | . | . | . | . | . | . | . | . | . | . | . | . | . | . | . | . | . |
| 21-225_157E4_HC | . | . | . | . | . | . | . | . | . | . | . | . | . | . | . | . | . | . | . | . | . | . | . | . | . | . |
| 21-225_157H10_HC | . | . | . | . | . | . | . | . | . | . | . | . | . | . | . | . | . | . | . | . | . | . | . | . | . | . |
| 21-225_15A2_HC | . | . | . | . | . | . | . | . | . | . | . | . | . | . | . | . | . | . | . | . | . | . | . | . | . | . |
| 21-225_16A11_HC | . | . | . | . | . | . | . | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | . | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . | . | . | . | . | . | . | . |
| 21-225_1F1_HC | . | . | . | . | . | . | . | . | . | . | . | . | . | . | . | . | . | . | . | . | . | . | . | . | . | . |
| 21-225_20H7_HC | . | . | : | . | . | . | . | . | . | . | . | . | . | . | . | . | . | . | . | . | . | . | . | . | . | . |
| 21-225_22B12_HC | . | . | . | . | . | . | . | . | . | . | . | . | . | . | . | . | . | . | . | . | . | . | . | . | . | . |
| 21-225_23A11_HC | . | . | . | . | . | . | . | . | . | . | . | . | . | . | . | . | . | . | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | . | . | . | . | . | . | . | . | . | . | . | . | . | . | . |
| 21-225_2A4_HC | . | . | . | . | . | . | . | . | . | . | . | . | . | . | . | . | . | . | . | . | . | . | . | . | . | . |
| 21-225_2C12_HC | . | . | . | . | . | . | . | . | . | . | . | . | . | . | . | . | . | . | . | . | . | . | . | . | . | . |
| 21-225_32B3_HC | . | . | . | . | . | . | . | . | . | . | . | . | . | . | . | . | . | . | . | . | . | . | . | . | . | . |
| 21-225_35H8_HC | . | . | . | . | . | . | . | . | . | . | . | . | . | . | . | . | . | . | . | . | . | . | . | . | . | . |
| 21-225_50G10_HC | . | . | . | . | . | . | . | . | . | . | . | . | . | . | . | . | . | . | . | . | . | . | . | . | . | . |
| 21-225_52E3_HC | . | . | . | . | . | . | . | . | . | . | . | . | . | . | . | . | . | . | . | . | . | . | . | . | . | . |
| 21-225_52H12_HC | . | . | . | . | . | . | . | . | . | . | . | . | . | . | . | . | . | . | . | . | . | . | . | . | . | . |
| 21-225_56A5_HC | . | . | . | . | . | . | . | . | . | . | . | . | . | . | . | . | . | . | . | . | . | . | . | . | . | . |
| 21-225_56H1_HC | . | . | . | . | . | . | . | . | . | . | . | . | . | . | . | . | . | . | . | . | . | . | . | . | . | . |
| 21-225_57B2_HC | . | . | . | . | . | . | . | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | . |
| 21-225_5F4_HC | . | . | . | . | . | . | . | . | . | . | . | . | . | . | . | . | . | . | . | . | . | . | . | v | . | . |
| 21-225_70E12_HC | E | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | | H_FR2 | | |
| CONSENSUS | G | - | F | T | F | S | S | - | - | - | - | - | Y | S | M | N | W | V | R | Q | A | P | G | K | G | L |
| 21-225_14D6_HC | . | . | . | . | . | . | . | . | . | . | . | . | . | T | . | . | . | . | . | . | . | . | . | . | . | . |
| 21-225_157E4_HC | . | . | . | . | . | . | . | . | . | . | . | . | . | T | . | . | . | . | . | . | . | . | . | . | . | . |
| 21-225_157H10_HC | . | . | . | . | . | . | . | . | . | . | . | . | . | T | . | . | . | . | . | . | . | . | . | . | . | . |
| 21-225_15A2_HC | . | . | . | . | . | . | . | . | . | . | . | . | . | T | . | . | . | . | . | . | . | . | . | . | . | . |
| 21-225_16A11_HC | . | . | . | . | . | . | . | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | . | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . | . | . | . | . | . | . | . |
| 21-225_1F1_HC | . | . | . | . | . | . | . | . | . | . | . | . | . | N | . | . | . | . | . | . | . | . | . | . | . | . |
| 21-225_20H7_HC | . | . | . | . | . | . | . | . | . | . | . | . | . | . | . | . | . | . | . | . | . | . | . | . | . | . |
| 21-225_22B12_HC | . | . | . | . | . | . | . | . | . | . | . | . | . | . | . | . | . | . | . | . | . | . | . | . | . | . |
| 21-225_23A11_HC | . | . | . | . | . | . | . | . | . | . | . | . | . | . | . | . | . | I | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | . | . | . | L | . | . | . | . | . | . | . | . | . | . | . |
| 21-225_2A4_HC | . | . | . | . | . | . | . | . | . | . | . | . | . | . | . | . | . | . | . | . | . | . | . | . | . | . |
| 21-225_2C12_HC | . | . | . | . | . | . | . | . | . | . | . | . | . | . | . | . | . | . | . | . | . | . | . | . | . | . |
| 21-225_32B3_HC | . | . | . | . | . | . | . | . | . | . | . | . | . | . | . | . | . | . | . | . | . | . | . | . | . | . |
| 21-225_35H8_HC | . | . | . | . | . | . | . | . | . | . | . | . | . | . | . | . | . | . | . | . | . | . | . | . | . | . |
| 21-225_50G10_HC | . | . | . | . | . | . | . | . | . | . | . | . | . | . | . | . | . | . | . | . | . | . | . | . | . | . |
| 21-225_52E3_HC | . | . | . | . | . | . | . | . | . | . | . | . | . | R | . | . | . | . | . | . | . | . | . | . | . | . |
| 21-225_52H12_HC | . | . | . | . | . | . | . | . | . | . | . | . | . | . | . | . | . | . | . | . | . | . | . | . | . | . |
| 21-225_56A5_HC | . | . | . | . | . | . | . | . | . | . | . | . | . | . | . | . | . | . | . | . | . | . | . | . | . | . |
| 21-225_56H1_HC | . | . | . | . | . | . | N | . | . | . | . | . | . | D | . | . | . | . | . | . | S | . | . | . | . | . |
| 21-225_57B2_HC | . | . | . | . | . | . | . | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | . |
| 21-225_5F4_HC | . | . | . | . | . | . | . | . | . | . | . | . | . | . | . | . | . | . | . | . | . | . | . | . | . | . |
| 21-225_70E12_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | S | I | S | G | S | - | - | - | S | S | Y | I | Y | Y | A | D | S | V | K | G | R | F |
| 21-225_14D6_HC | . | . | . | . | . | . | . | . | . | . | . | . | G | . | . | . | . | . | . | . | . | . | . | . | . | . |
| 21-225_157E4_HC | . | . | . | . | . | . | . | . | . | . | . | . | . | . | . | . | . | . | . | . | . | . | . | . | . | . |
| 21-225_157H10_HC | . | . | . | . | . | . | . | . | . | . | . | . | . | . | . | . | N | . | . | . | . | . | . | . | . | . |
| 21-225_15A2_HC | . | . | . | . | . | . | . | . | . | . | . | . | G | . | . | . | . | . | . | . | . | . | . | . | . | . |
| 21-225_16A11_HC | . | . | . | . | . | . | . | . | . | . | . | . | . | . | L | . | . | . | . | . | . | . | . | . | . | . |
| 21-225_16B8_HC | . | . | I | . | . | . | . | . | . | . | . | . | . | T | . | . | . | . | . | . | . | . | . | . | . | . |
| 21-225_16H1_HC | . | . | . | . | . | . | . | . | . | . | . | . | . | T | . | . | . | . | . | . | . | . | . | . | . | . |
| 21-225_17H5_HC | . | . | . | . | . | . | . | S | . | . | . | . | . | T | . | . | . | . | . | . | . | . | . | . | . | . |
| 21-225_17H6_HC | . | . | . | . | . | . | . | . | . | . | . | . | . | . | . | . | . | . | . | . | . | . | . | . | . | . |
| 21-225_1F1_HC | . | . | . | . | . | . | . | S | . | . | . | . | . | . | . | . | . | . | . | . | . | . | . | . | . | . |
| 21-225_20H7_HC | . | . | . | . | . | . | . | . | . | . | . | . | . | . | F | . | . | . | . | . | . | . | . | . | . | . |
| 21-225_22B12_HC | . | . | . | . | . | . | . | . | . | . | . | . | . | . | . | T | . | . | . | . | . | . | . | . | . | . |
| 21-225_23A11_HC | . | . | . | . | . | . | . | . | . | . | . | . | . | T | . | . | . | . | . | . | . | . | . | . | . | I |
| 21-225_27C5_HC | . | . | . | . | T | . | . | S | . | . | . | . | D | . | . | . | . | . | . | . | . | . | . | . | . | . |
| 21-225_27H12_HC | . | . | . | . | . | . | . | . | . | . | . | . | . | . | N | . | . | . | T | . | . | . | . | . | . | . |
| 21-225_2A4_HC | . | . | . | . | . | . | . | . | . | . | . | . | . | T | . | . | . | . | . | . | . | . | . | . | . | I |
| 21-225_2C12_HC | . | . | . | . | . | . | . | . | . | . | . | . | . | . | . | M | . | . | . | . | . | . | . | . | . | . |
| 21-225_32B3_HC | . | . | . | . | . | . | . | . | . | . | . | . | . | T | . | . | . | . | . | . | . | . | . | . | . | . |
| 21-225_35H8_HC | . | . | . | . | . | . | T | . | . | . | . | . | G | . | F | . | . | . | . | . | . | . | . | . | . | . |
| 21-225_50G10_HC | . | . | . | . | . | . | . | . | . | . | . | . | . | . | . | . | . | . | . | . | . | . | . | . | . | . |
| 21-225_52E3_HC | . | . | . | . | . | . | S | G | . | . | . | . | N | . | S | . | . | . | . | . | . | . | . | . | . | . |
| 21-225_52H12_HC | . | . | . | . | . | . | . | . | . | . | . | . | . | T | . | . | . | . | . | . | . | . | . | . | . | . |
| 21-225_56A5_HC | . | . | . | . | . | . | . | . | . | . | . | . | . | . | . | . | . | . | . | . | . | . | . | . | . | . |
| 21-225_56H1_HC | . | . | I | . | . | . | . | . | . | . | . | . | . | T | D | . | . | . | . | . | . | . | . | . | . | . |
| 21-225_57B2_HC | . | . | . | . | . | . | . | . | . | . | . | . | . | . | . | . | N | . | T | . | . | . | . | . | . | . |
| 21-225_5E11_HC | . | . | . | . | . | . | . | . | . | . | . | . | . | T | . | . | . | . | . | . | . | . | . | . | . | . |
| 21-225_5F4_HC | . | . | . | . | . | . | . | . | . | . | . | . | . | . | . | . | . | . | . | . | . | . | . | . | . | . |
| 21-225_70E12_HC | E | . | . | . | . | . | . | . | N | . | . | . | . | T | . | . | . | . | . | T | . | . | . | . | . | . |

## FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | A | K | N | S | L | Y | L | Q | M | N | S | L | R | A | E | D | T | A | V | Y |
| 21-225_14D6_HC | . | M | . | | | | | | | | | | | | | | | | | | | | | | | |
| 21-225_157E4_HC | . | | | | | | | | | | | | | | | | | | K | | | | | | | |
| 21-225_157H10_HC | . | | | | | | | | | | | | | | | | | | | | | | | | | |
| 21-225_15A2_HC | . | | | | | | | | | | | | | | | | A | | | | | | | | | |
| 21-225_16A11_HC | . | | | | | | | | | | | | | | | | | | | | | | | | | |
| 21-225_16B8_HC | . | | | | | | | | | | | | | | | | | | | | | | | | | |
| 21-225_16H1_HC | . | | | | | | | | | | | | | | | | | | | | | | | | | |
| 21-225_17H5_HC | . | | | | | | | | | | | | | | | | | | | | | | | | | |
| 21-225_17H6_HC | . | | F | | | | | | | | | | | | | | | | | | | | | | | |
| 21-225_1F1_HC | . | | | | | | | | | | | | | | | | | | | | | | | | | |
| 21-225_20H7_HC | . | | | | | | | | | | | | | | | | | | | | | | | | | |
| 21-225_22B12_HC | . | | | | | | | | | | | | | | | | | | | | | | | | | |
| 21-225_23A11_HC | A | | | | | | | | | | | | | | | | | | | | | | | | | |
| 21-225_27C5_HC | . | | | | | | | | | | | | | | | | | | | | | | | | | |
| 21-225_27H12_HC | . | | | | | | | | | | | F | | | | | | | | | | | | | | |
| 21-225_2A4_HC | A | | | | | | | | | | | | | | | | | | | | | | | | | |
| 21-225_2C12_HC | . | | | | | | | D | | | | | | | | | | | | | | | | | | |
| 21-225_32B3_HC | . | | | | | | | | | | | | | | | | | | | | | | | | | |
| 21-225_35H8_HC | . | | | | | | | | K | | | V | | | | | | | | | | | | | | |
| 21-225_50G10_HC | . | | | | | | | | | | | | | | | | | | | | | | | | | |
| 21-225_52E3_HC | . | | | | | | | E | | | | | | | | | | | | | | | | | | |
| 21-225_52H12_HC | . | V | | | | | | | | | | | | V | | | | | | | | | | | | |
| 21-225_56A5_HC | . | | | | | | | | | | | | | | | | | | | | | | | | | |
| 21-225_56H1_HC | . | | | | | | | | | | | | | | | | | | | | | | | | | |
| 21-225_57B2_HC | . | | | | | | | | | | | | | | | | | | | | | | | | | |
| 21-225_5E11_HC | . | | | | | | | | | | | | | | | | | | | | | | | | | F |
| 21-225_5F4_HC | . | | | | | | | | | | | F | | | | | | | | | | | | | | |
| 21-225_70E12_HC | . | | | | | | | | | | | | | | | D | | | T | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | V | A | S | - | - | - | - | - | - | - | - | - | - | - | - | - | - | - | - | - | - | - |
| 21-225_14D6_HC | . | . | . | . | . | . | A | . | . | . | . | . | . | . | . | . | . | . | . | . | . | . | . | . | . | . |
| 21-225_157E4_HC | . | . | . | . | . | . | T | . | . | . | . | . | . | . | . | . | . | . | . | . | . | . | . | . | . | . |
| 21-225_157H10_HC | . | . | . | . | . | . | H | . | . | . | . | . | . | . | . | . | . | . | . | . | . | . | . | . | . | . |
| 21-225_15A2_HC | . | . | . | . | . | . | . | . | . | . | . | . | . | . | . | . | . | . | . | . | . | . | . | . | . | . |
| 21-225_16A11_HC | . | . | . | . | . | . | A | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | A | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . | . | . | . | . | . | . | . |
| 21-225_1F1_HC | . | . | . | . | . | N | L | . | . | . | . | . | . | . | . | . | . | . | . | . | . | . | . | . | . | . |
| 21-225_20H7_HC | . | . | . | . | . | . | A | . | . | . | . | . | . | . | . | . | . | . | . | . | . | . | . | . | . | . |
| 21-225_22B12_HC | . | . | . | . | . | . | A | . | . | . | . | . | . | . | . | . | . | . | . | . | . | . | . | . | . | . |
| 21-225_23A11_HC | . | . | . | . | . | . | . | . | . | . | . | . | . | . | . | . | . | . | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | . | . | . | . | . | . | . | . | . | . | . | . | . | . | . |
| 21-225_2A4_HC | . | . | . | . | . | . | . | . | . | . | . | . | . | . | . | . | . | . | . | . | . | . | . | . | . | . |
| 21-225_2C12_HC | . | . | . | . | . | N | L | . | . | . | . | . | . | . | . | . | . | . | . | . | . | . | . | . | . | . |
| 21-225_32B3_HC | . | . | . | . | . | . | G | . | . | . | . | . | . | . | . | . | . | . | . | . | . | . | . | . | . | . |
| 21-225_35H8_HC | . | . | . | . | . | S | G | . | . | . | . | . | . | . | . | . | . | . | . | . | . | . | . | . | . | . |
| 21-225_50G10_HC | . | . | . | . | . | . | T | . | . | . | . | . | . | . | . | . | . | . | . | . | . | . | . | . | . | . |
| 21-225_52E3_HC | . | . | . | . | . | S | . | . | . | . | . | . | . | . | . | . | . | . | . | . | . | . | . | . | . | . |
| 21-225_52H12_HC | . | . | . | . | . | N | . | . | . | . | . | . | . | . | . | . | . | . | . | . | . | . | . | . | . | . |
| 21-225_56A5_HC | . | . | . | . | . | . | T | . | . | . | . | . | . | . | . | . | . | . | . | . | . | . | . | . | . | . |
| 21-225_56H1_HC | . | . | . | . | . | . | . | . | . | . | . | . | . | . | . | . | . | . | . | . | . | . | . | . | . | . |
| 21-225_57B2_HC | . | . | . | . | . | . | G | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | . |
| 21-225_5F4_HC | . | . | . | . | . | N | N | . | . | . | . | . | . | . | . | . | . | . | . | . | . | . | . | . | . | . |
| 21-225_70E12_HC | Y | . | . | . | . | . | A | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | F | D | Y | W | G | Q | G | T | L | V | T | V | S | S |
| 21-225_14D6_HC | | | | | | | | S | | | | | | | | S | | | |
| 21-225_157E4_HC | | | | | | | | | | | | | | | | | | | |
| 21-225_157H10_HC | | | | | | | | | | | | | | | | | | | |
| 21-225_15A2_HC | | | | | | | | | | | | | | | | | | | |
| 21-225_16A11_HC | | | | | | | | | | | | | | | | | | | |
| 21-225_16B8_HC | | | | | | | | | | | | | | | | | | | |
| 21-225_16H1_HC | | | | | | | | | | | | | | | | | | | |
| 21-225_17H5_HC | | | | | | | | | | | | | | | | | | | |
| 21-225_17H6_HC | | | | | | | | | | | | | | | | | | | |
| 21-225_1F1_HC | | | | | | | | | | | | | | | | | | | |
| 21-225_20H7_HC | | | | | L | | | | | | | | | | | | | | |
| 21-225_22B12_HC | | | | | L | | | S | | | | | | | | | | | |
| 21-225_23A11_HC | | | | | | | | | | | | | | | | | | | |
| 21-225_27C5_HC | | | | | | | | O | | | | | | | | | | | |
| 21-225_27H12_HC | | | | | | | | | | | | | | | | | | | |
| 21-225_2A4_HC | | | | | | | | | | | | | | | | | | | |
| 21-225_2C12_HC | | | | | | | | | | | | | | | | | | | |
| 21-225_32B3_HC | | | | | | | | | | | | | | | | | | | |
| 21-225_35H8_HC | | | | | | | | | | | | | | | | | | | |
| 21-225_50G10_HC | | | | | | | | | | | | | | | | | | | |
| 21-225_52E3_HC | | | | N | | | | | | | | | | | | | | | |
| 21-225_52H12_HC | | | | | | | | | | | | | | | | | | | |
| 21-225_56A5_HC | | | | | | | | | | | | | | | | | | | |
| 21-225_56H1_HC | | | | | | | | | | | | | | | | | | | |
| 21-225_57B2_HC | | | | | | | | | | | | | | | | | | | |
| 21-225_5E11_HC | | | | | | | | | | | | | | | | | | | |
| 21-225_5F4_HC | | | | | | | | | | | | | | | | | | | |
| 21-225_70E12_HC | | | | | | | | O | | | | | | | | | | | |

**FIGURE 57**

Table 63.     Consensus 29-VH4|4-30.1/D3|3-22|RF2/JH6 (SEQ ID NO: 50280):

QVQLQES-GPGLVKPSQTLSLTCTVSG-GSISSG---DYYWNWIRQHPGKGLEWIGYIFY----
SGSTYYNPSLKSRVTISVDTSKNQFSLKLSSVTAADTAVYYCARGDYDGSGSY------------HYYYGMDVWGQGTTVTVSS wherein:

G at position 34 can be substituted with V

D at position 38 can be substituted with V, G or S

Y at position 40 can be substituted with H

N at position 42 can be substituted with S

Y at position 57 can be substituted with N or F

I at position 58 can be substituted with L

F at position 59 can be substituted with Y or H

Y at position 60 can be substituted with H

Y at position 70 can be substituted with N

K at position 75 can be substituted with R

Y at position 132 can be substituted with F or H

Y at position 134 can be substituted with H

M at position 136 can be substituted with L

Ser Xaa Xaa Tyr Xaa Trp Xaa (SEQ ID NO: 50230)

Xaa Xaa Xaa Xaa Ser Gly Ser Thr Tyr Xaa Asn Pro Ser Leu Xaa Ser (SEQ ID NO: 50231)

Gly Asp Tyr Asp Gly Ser Gly Ser Tyr His Xaa Tyr Xaa Gly Xaa Asp Val (SEQ ID NO: 50232)

Ser Gly Asp Tyr Tyr Trp Asn (SEQ ID NO: 50510)

**FIGURE 57**

Tyr Ile Phe Tyr Ser Gly Ser Thr Tyr Tyr Asn Pro Ser Leu Lys Ser (SEQ ID NO: 50511)

Gly Asp Tyr Asp Gly Ser Gly Ser Tyr His Tyr Tyr Tyr Gly Met Asp Val (SEQ ID NO: 50512)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | | | | | | | | | | | | | | | | H_FR1 | | | | | | | | | | | |
| CONSENSUS | Q | V | Q | L | Q | E | S | - | G | P | G | L | V | K | P | S | Q | T | L | S | L | T | C | T | V | S | G |
| 21-225_190A3_HC | . | . | . | . | . | . | . | . | . | . | . | . | . | . | . | . | . | . | . | . | . | . | . | . | . | . | . |
| 21-225_190A7_HC | . | . | . | . | . | . | . | . | . | . | . | . | . | N | . | . | . | . | . | . | . | . | . | . | . | . | . |
| 21-225_190B4_HC | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . |
| 21-225_191D3_HC | . | . | . | . | N | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . |
| 21-225_192B3_HC | . | . | . | . | . | . | . | . | . | . | . | . | . | . | . | . | . | . | . | . | . | . | . | . | . | . | . |
| 21-225_192G12_HC | . | . | . | . | . | . | . | . | . | . | . | . | . | . | . | . | . | . | . | . | . | . | . | . | . | . | . |
| 21-225_192H3_HC | . | . | . | . | . | . | . | . | . | . | . | . | . | . | . | . | . | . | . | . | . | . | . | . | . | . | . |
| 21-225_193A1_HC | . | . | . | . | . | . | . | . | . | . | . | . | . | . | . | . | . | . | . | . | . | . | . | . | . | . | . |
| 21-225_193B1_HC | . | . | . | . | . | . | . | . | . | . | . | . | . | . | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . |
| 21-225_193D8_HC | . | . | . | . | . | . | . | . | . | . | . | . | . | . | . | . | . | . | . | . | . | . | . | . | . | . | . |
| 21-225_193F3_HC | . | . | . | . | . | . | . | . | . | . | . | . | . | . | . | . | . | . | . | . | . | . | . | . | . | . | . |
| 21-225_193G8_HC | . | . | . | . | . | . | N | . | . | . | . | . | . | . | . | . | . | . | . | . | . | . | . | . | . | . | . |
| 21-225_193H6_HC | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . |
| 21-225_194E6_HC | . | . | . | . | . | . | . | . | . | . | . | . | . | . | . | . | . | . | . | . | . | . | . | . | . | . | . |
| 21-225_196H12_HC | . | . | . | . | . | . | . | . | . | . | . | . | . | N | . | . | . | . | . | . | . | . | . | . | . | . | . |
| 21-225_197F4_HC | . | . | . | . | . | . | . | . | . | . | . | . | . | . | . | . | . | . | . | . | . | . | . | . | . | . | . |
| 21-225_198B1_HC | . | . | . | . | N | . | . | . | . | . | . | . | . | . | . | . | . | . | . | . | . | . | . | . | . | . | . |
| 21-225_198E1_HC | . | . | . | . | . | . | . | . | . | . | . | . | . | . | . | . | . | . | . | . | . | . | . | . | . | . | . |
| 21-225_199C2_HC | . | . | . | . | . | . | . | . | . | . | . | . | . | . | . | . | . | . | . | . | . | . | . | . | . | . | . |
| 21-225_199C7_HC | . | . | . | . | . | . | . | . | . | . | . | . | . | . | . | . | . | . | . | . | . | . | . | . | . | . | . |
| 21-225_200F10_HC | . | . | . | . | . | . | . | . | . | . | . | . | . | . | . | . | . | . | . | . | . | . | . | . | . | . | . |

FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | | | | | | H_CDR1 | | | | | | | | | | | | H_FR2 | | | | | |
| CONSENSUS | - | G | S | I | S | S | G | - | - | - | D | Y | Y | W | N | W | I | R | Q | H | P | G | K | G | L | E | W |
| 21-225_190A3_HC | . | | | . | N | . | | . | | . | G | | . | | . | | . | | . | | . | | . | | . | | . |
| 21-225_190A7_HC | . | | . | | . | | . | | . | | V | | . | | S | | . | | . | | . | | . | | . | | . |
| 21-225_190B4_HC | . | | . | | . | | . | | . | | . | | . | | . | | . | | . | | . | | . | | . | | . |
| 21-225_191A3_HC | . | | . | | . | | . | | . | | . | | . | | . | | . | | . | | . | | . | | . | | . |
| 21-225_191D3_HC | . | | . | | N | . | | . | | . | | . | | . | | . | | . | | . | | . | | . | | . | D | . |
| 21-225_191F1_HC | . | | . | | N | . | | . | | . | G | | . | | . | | . | | . | | . | | . | | . | | . |
| 21-225_191H7_HC | . | | . | | N | . | | . | | . | | . | | . | | . | | . | | . | | . | | . | | . | | . |
| 21-225_192B3_HC | . | | . | | . | | . | | . | | . | | . | | . | | . | | . | | . | | . | | . | | . |
| 21-225_192G12_HC | . | | . | | . | | . | | . | | V | | M | . | S | | . | | . | | . | | . | | . | | . |
| 21-225_192H3_HC | . | | . | | . | | V | | . | | S | | . | | S | | . | | . | R | . | | . | | . | | . |
| 21-225_193A1_HC | . | | . | | . | | . | | . | | V | | . | | S | | . | | . | | . | | . | | . | | . |
| 21-225_193B1_HC | . | | . | | . | | . | | . | | V | | . | | S | | . | | . | | . | | . | | . | | . |
| 21-225_193C8_HC | . | | . | | . | | . | | . | | . | | . | | . | | . | | . | | . | | . | | . | | . |
| 21-225_193D8_HC | . | | . | | N | . | | . | | . | G | | . | | . | | . | | . | | . | | . | | . | | . |
| 21-225_193F3_HC | . | | . | | N | . | | . | | . | | . | | . | | . | | . | | . | | . | | . | | . | | . |
| 21-225_193G8_HC | . | | . | V | . | | . | | . | | V | | . | | S | | . | | . | | . | | . | | . | | . |
| 21-225_193H6_HC | . | | . | | N | . | | . | | . | | . | | . | | . | | . | | . | | . | | . | | . | | . |
| 21-225_194E5_HC | . | | . | | N | . | | . | | . | | . | | . | | . | | . | | N | . | | . | | . | | . | | . |
| 21-225_194E6_HC | . | D | . | | N | . | | . | | . | | . | | . | | . | | . | | . | | . | | . | | . | | . |
| 21-225_196H12_HC | . | | . | | . | | . | | . | | V | | . | | S | | . | | . | | . | | . | | . | | . |
| 21-225_197F4_HC | . | | . | | . | | . | | . | | . | | . | | . | | . | | . | | . | | . | | . | | . |
| 21-225_198B1_HC | . | | . | | N | . | | . | | . | | . | | . | | . | P | . | | . | | . | | . | | . | | . |
| 21-225_198E1_HC | . | | . | | N | . | | . | | . | G | | . | | . | | . | | . | | . | | . | | . | | . |
| 21-225_199C2_HC | . | | . | | . | | . | | . | | . | | . | | . | | L | . | | . | | . | E | . | | . | | . |
| 21-225_199C7_HC | . | | . | | N | . | | . | | . | G | | . | | . | | . | | . | | . | | . | | . | | . |
| 21-225_200F10_HC | . | | . | | . | | . | | . | | . | | . | | . | | L | . | | . | | . | E | . | | . | | . |

FIGURE 57

| Reference # | 55 | 56 | 57 | 58 | 59 | 60 | 61 | 62 | 63 | 64 | 65 | 67 | 68 | 69 | 70 | 71 | 72 | 73 | 74 | 75 | 76 | 77 | 78 | 79 | 80 | 81 | 82 | 83 |
|---|---|---|---|---|---|---|---|---|---|---|---|---|---|---|---|---|---|---|---|---|---|---|---|---|---|---|---|---|
| | | | | | | | | | | | | | | | | | | | | | | | | | | | | |
| CONSENSUS | I | G | Y | I | F | Y | - | - | - | - | S | S | T | Y | Y | N | P | S | L | K | S | R | V | T | I | S | V | D |
| 21-225_190A3_HC | . | . | F | . | . | . | . | . | . | . | . | . | . | . | . | . | . | . | . | . | . | . | . | . | . | . | . | . |
| 21-225_190A7_HC | . | . | N | . | Y | . | . | . | . | . | . | . | . | . | N | . | . | . | . | . | . | . | I | I | . | . | . | . |
| 21-225_190B4_HC | . | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . | . |
| 21-225_191D3_HC | . | . | . | . | . | . | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . | . |
| 21-225_192B3_HC | . | . | F | . | . | . | . | . | . | . | . | . | . | . | . | . | . | . | . | . | . | . | . | . | . | . | . | . |
| 21-225_192G12_HC | . | . | N | . | H | . | . | . | . | . | . | . | . | . | N | . | . | . | . | . | . | . | . | . | . | . | . | . |
| 21-225_192H3_HC | . | . | N | L | Y | . | . | . | . | . | . | . | . | . | . | . | . | . | . | H | . | . | A | . | . | . | . | . |
| 21-225_193A1_HC | . | . | N | . | Y | . | . | . | . | . | . | . | . | . | N | . | . | . | . | . | . | . | L | . | . | . | A | . |
| 21-225_193B1_HC | . | . | N | . | Y | . | . | . | . | . | . | . | . | . | N | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . | . |
| 21-225_193D8_HC | . | . | F | . | . | . | . | . | . | . | . | . | . | . | . | . | . | . | . | . | . | . | . | S | . | . | . | . |
| 21-225_193F3_HC | . | . | . | . | . | . | . | . | . | . | . | . | . | . | . | . | . | . | . | . | . | . | . | . | . | . | . | . |
| 21-225_193G8_HC | . | . | N | . | Y | . | . | . | . | . | . | . | . | . | N | . | . | . | . | . | . | . | . | . | . | . | . | . |
| 21-225_193H6_HC | . | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . | . |
| 21-225_194E6_HC | . | . | F | . | . | . | . | . | . | . | . | . | . | . | . | . | . | . | . | . | . | . | . | . | . | I | . | . |
| 21-225_196H12_HC | . | . | N | . | Y | . | . | . | . | . | . | . | . | . | N | . | . | . | . | . | . | I | I | . | . | . | . | . |
| 21-225_197F4_HC | . | . | . | . | . | . | . | . | . | . | . | . | . | . | . | . | . | . | . | . | . | I | . | . | . | . | . | . |
| 21-225_198B1_HC | . | . | . | . | . | H | . | . | . | . | . | . | . | . | . | . | . | . | . | . | . | . | . | V | . | . | . | . |
| 21-225_198E1_HC | . | . | F | . | . | . | . | . | . | . | . | . | . | . | . | . | . | . | . | . | . | L | S | . | . | . | . | . |
| 21-225_199C2_HC | . | . | . | . | . | . | . | . | . | . | . | . | . | . | . | . | . | . | . | . | . | . | . | . | . | . | . | . |
| 21-225_199C7_HC | . | . | F | . | . | . | . | . | . | . | . | . | . | . | . | . | . | . | . | . | . | . | S | . | . | . | . | . |
| 21-225_200F10_HC | . | . | . | . | . | . | . | . | . | . | . | . | . | . | . | . | . | . | . | . | . | I | . | . | . | . | . | . |

FIGURE 57

| Reference # | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 | 109 | 110 |
|---|---|---|---|---|---|---|---|---|---|---|---|---|---|---|---|---|---|---|---|---|---|---|---|---|---|---|---|
| | | | | | | | | | | H_FR3 | | | | | | | | | | | | | | | | | |
| CONSENSUS | T | S | K | N | Q | F | S | L | K | L | S | S | V | T | A | A | D | T | A | V | Y | Y | C | A | R | G | D |
| 21-225_190A3_HC | . | | . | . | . | . | . | . | . | | N | | . | . | . | . | . | . | . | . | . | . | . | . | . | . | . |
| 21-225_190A7_HC | . | . | . | . | . | . | . | . | . | . | . | . | . | . | . | . | . | . | . | . | . | . | . | . | . | . | . |
| 21-225_190B4_HC | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . |
| 21-225_191D3_HC | . | . | . | . | . | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . |
| 21-225_192B3_HC | . | . | . | . | . | . | . | . | . | . | . | . | . | . | V | . | . | . | . | . | . | . | . | . | . | . | . |
| 21-225_192G12_HC | . | . | . | . | . | . | . | . | . | . | . | . | . | . | . | . | . | . | . | . | . | . | . | . | . | . | . |
| 21-225_192H3_HC | . | . | . | . | . | . | . | . | . | . | . | . | . | . | . | . | . | . | . | . | . | . | . | T | . | . | . |
| 21-225_193A1_HC | . | . | . | . | . | . | . | . | . | . | . | . | . | . | . | . | . | . | . | . | . | . | . | . | . | . | . |
| 21-225_193B1_HC | . | . | . | . | . | . | . | . | . | . | . | . | . | . | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . |
| 21-225_193D8_HC | . | . | . | . | . | . | . | . | . | . | . | . | . | . | . | . | . | . | . | . | . | . | . | . | . | . | . |
| 21-225_193F3_HC | . | . | . | . | . | . | . | . | . | . | . | . | . | . | . | . | . | . | . | . | . | . | . | . | . | . | . |
| 21-225_193G8_HC | . | . | . | . | . | . | . | . | . | . | N | . | . | . | . | . | . | . | . | . | . | . | . | . | . | . | . |
| 21-225_193H6_HC | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . |
| 21-225_194E6_HC | . | . | . | . | . | . | . | . | . | . | . | . | . | N | V | . | . | . | . | . | . | . | . | . | . | . | . |
| 21-225_196H12_HC | . | . | . | . | . | . | . | . | . | . | . | . | . | . | . | . | . | . | . | . | . | . | . | . | . | . | . |
| 21-225_197F4_HC | . | . | . | . | . | . | . | . | . | . | . | . | . | . | . | . | . | . | . | . | . | . | . | . | . | . | . |
| 21-225_198B1_HC | . | . | . | . | . | . | . | . | . | . | . | . | . | . | . | . | . | . | . | . | . | . | . | . | . | . | . |
| 21-225_198E1_HC | . | . | . | . | . | . | . | . | . | . | . | . | . | . | . | . | . | . | . | . | . | . | . | . | . | . | . |
| 21-225_199C2_HC | . | . | M | T | . | . | . | . | . | . | T | . | . | . | . | . | . | . | . | . | . | . | . | . | . | . | . |
| 21-225_199C7_HC | . | . | . | . | . | . | . | . | . | . | . | . | . | . | . | . | . | . | . | . | . | . | . | . | . | . | . |
| 21-225_200F10_HC | . | . | M | T | . | . | . | . | . | . | T | . | . | . | . | . | . | . | . | . | . | . | . | . | . | . | . |

FIGURE 57

| Reference # | 111 | 112 | 113 | 114 | 115 | 116 | 117 | 118 | 119 | 120 | 121 | 122 | 123 | 124 | 125 | 126 | 127 | 128 | 129 | 130 | 133 | 134 | 135 | 136 | 137 | 138 | 139 | 140 |
|---|---|---|---|---|---|---|---|---|---|---|---|---|---|---|---|---|---|---|---|---|---|---|---|---|---|---|---|---|
| | | | | | | | | | | | | H_CDR3 | | | | | | | | | | | | | | | | |
| CONSENSUS | Y | D | G | S | G | S | Y | - | - | - | - | - | - | - | - | - | - | - | - | - | Y | Y | G | M | D | V | W | G |
| 21-225_190A3_HC | . | . | . | . | . | . | . | . | . | . | . | . | . | . | . | . | . | . | . | . | . | . | . | . | . | . | . | . |
| 21-225_190A7_HC | . | . | . | . | . | . | . | . | . | . | . | . | . | . | . | . | . | . | . | . | . | . | L | . | . | . | . | . |
| 21-225_190B4_HC | . | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . | . |
| 21-225_191D3_HC | . | . | . | . | . | . | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . | . |
| 21-225_192B3_HC | . | . | . | . | . | . | . | . | . | . | . | . | . | . | . | . | . | . | . | . | . | . | . | . | . | . | . | . |
| 21-225_192G12_HC | . | . | . | . | . | . | . | . | . | . | . | . | . | . | . | . | . | . | . | . | . | . | . | . | . | . | . | . |
| 21-225_192H3_HC | . | . | . | . | . | . | . | . | . | . | . | . | . | . | . | . | . | . | . | . | . | M | . | . | . | . | . | . |
| 21-225_193A1_HC | . | . | . | . | . | . | . | . | . | . | . | . | . | . | . | . | . | . | . | . | . | . | . | . | . | . | . | . |
| 21-225_193B1_HC | . | . | . | . | . | . | . | . | . | . | . | . | . | . | . | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . | . |
| 21-225_193D8_HC | . | . | . | . | . | . | . | . | . | . | . | . | . | . | . | . | . | . | . | . | . | . | . | . | . | . | . | . |
| 21-225_193F3_HC | . | . | . | . | . | . | . | . | . | . | . | . | . | . | . | . | . | . | . | . | . | . | . | . | . | . | . | . |
| 21-225_193G8_HC | . | . | . | . | . | . | . | . | . | . | . | . | . | . | . | . | . | . | . | . | . | . | . | L | . | . | . | . |
| 21-225_193H6_HC | . | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . | . |
| 21-225_194E6_HC | . | . | . | . | . | . | . | . | . | . | . | . | . | . | . | . | . | . | . | . | . | . | . | . | . | . | . | . |
| 21-225_196H12_HC | . | . | . | . | . | . | . | . | . | . | . | . | . | . | . | . | . | . | . | . | . | . | . | L | . | . | . | . |
| 21-225_197F4_HC | . | . | . | . | . | . | . | . | . | . | . | . | . | . | . | . | . | . | . | . | . | . | . | L | . | . | . | . |
| 21-225_198B1_HC | . | . | . | . | . | . | . | . | . | . | . | . | . | . | . | . | . | . | . | . | . | . | . | . | . | . | . | . |
| 21-225_198E1_HC | . | . | . | . | . | . | . | . | . | . | . | . | . | . | . | . | . | . | . | . | . | . | . | . | . | . | . | . |
| 21-225_199C2_HC | . | . | . | . | . | . | . | . | . | . | . | . | . | . | . | . | . | . | . | . | . | . | . | . | . | . | . | . |
| 21-225_199C7_HC | . | . | . | . | . | . | . | . | . | . | . | . | . | . | . | . | . | . | . | . | . | . | . | . | . | . | . | . |
| 21-225_200F10_HC | . | . | . | . | . | . | . | . | . | . | . | . | . | . | . | . | . | . | . | . | . | . | . | . | . | . | . | . |

**FIGURE 57**

| Reference # | 141 | 142 | 143 | 144 | 145 | 146 | 147 | 148 | 149 |
|---|---|---|---|---|---|---|---|---|---|
| | | | | H_FR4 | | | | | |
| CONSENSUS | Q | G | T | T | V | T | V | S | S |
| 21-225_190A3_HC | . | . | . | . | . | . | . | . | . |
| 21-225_190A7_HC | . | . | . | . | . | . | . | . | . |
| 21-225_190B4_HC | . | . | . | . | . | . | . | . | . |
| 21-225_191A3_HC | . | . | . | . | . | . | . | . | . |
| 21-225_191D3_HC | . | . | . | . | . | . | . | . | . |
| 21-225_191F1_HC | . | . | . | . | . | . | . | . | . |
| 21-225_191H7_HC | . | . | . | . | . | . | . | . | . |
| 21-225_192B3_HC | . | . | . | . | . | . | . | . | . |
| 21-225_192G12_HC | . | . | . | . | . | . | . | . | . |
| 21-225_192H3_HC | . | . | . | . | . | . | . | . | . |
| 21-225_193A1_HC | . | . | . | . | . | . | . | . | . |
| 21-225_193B1_HC | . | . | . | . | . | . | . | . | . |
| 21-225_193C8_HC | . | . | . | . | . | . | . | . | . |
| 21-225_193D8_HC | . | . | . | . | . | . | . | . | . |
| 21-225_193F3_HC | . | . | . | . | . | . | . | . | . |
| 21-225_193G8_HC | ⁑ | . | . | . | . | . | . | . | . |
| 21-225_193H6_HC | . | . | . | . | . | . | . | . | . |
| 21-225_194E5_HC | . | . | . | . | . | . | . | . | . |
| 21-225_194E6_HC | . | . | . | . | . | . | . | . | . |
| 21-225_196H12_HC | . | . | . | . | . | . | . | . | . |
| 21-225_197F4_HC | . | . | . | . | . | . | . | . | . |
| 21-225_198B1_HC | ⁑ | . | . | . | . | . | . | . | . |
| 21-225_198E1_HC | . | . | . | . | . | . | . | . | . |
| 21-225_199C2_HC | . | . | . | . | . | . | . | . | . |
| 21-225_199C7_HC | . | . | . | . | . | . | . | . | . |
| 21-225_200F10_HC | . | . | . | . | . | . | . | . | . |

**FIGURE 57**

Table 64.    <u>Consensus 30- VH3|3-23/D7|7-27|RF1/JH6 (SEQ ID NO: 50281):</u>

EVQLLES-GGGLVQPGGSLRLSCAASG-FTFS<u>S-----YAMS</u>WVRQAPGKGLEWVS<u>VISGG---
GSSTYY</u>ADSVKGRFTISRDNSKNTLYLQMNSLRAEDTAVYYCAK<u>WRGNPT-----------------DYGMD</u>VWGQGTTVTVSS wherein:

S at position 33 can be substituted with N or T

Y at position 39 can be substituted with H

A at position 40 can be substituted with P or V

V at position 57 can be substituted with A or I

G at position 61 can be substituted with S

S at position 66 can be substituted with G or T

S at position 67 can be substituted with T

T at position 68 can be substituted with A

W at position 109 can be substituted with A

R at position 110 can be substituted with G

N at position 112 can be substituted with T

P at position 113 can be substituted with T

T at position 114 can be substituted with G

null (-) at position 115 can be substituted with S

null (-) at position 116 can be substituted with Y

null (-) at position 132 can be substituted with Y

D at position 133 can be substituted with Y

**FIGURE 57**

Y at position 134 can be substituted with N or S

Xaa Xaa Xaa Met Ser (SEQ ID NO: 50043)

Xaa Ile Ser Gly Xaa Gly Xaa Xaa Xaa Tyr Tyr Ala Asp Ser Val Lys Gly (SEQ ID NO: 50044)

Xaa Xaa Gly Xaa Xaa Xaa Xaa Xaa Xaa Xaa Xaa Gly Met Asp (SEQ ID NO: 50045)

Ser Tyr Ala Met Ser (SEQ ID NO: 50513)

Val Ile Ser Gly Gly Gly Ser Ser Thr Tyr Tyr Ala Asp Ser Val Lys Gly (SEQ ID NO: 50514)

Trp Arg Gly Asn Pro Thr Asp Tyr Gly Met Asp (SEQ ID NO: 50515)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
|  |  |  |  |  |  |  |  |  |  |  |  |  |  |  |  | H_FR1 |  |  |  |  |  |  |  |  |  |  |
| CONSENSUS | E | V | Q | L | L | E | S | - | G | G | G | L | V | Q | P | G | G | S | L | R | L | S | C | A | A | S |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | . | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | §: | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | . | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | ì | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | A | M | S | W | V | R | Q | A | P | G | K | G | L |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | ? | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | N | . | . | . | . | . | . | V | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | ? | . | . | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | X | . | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | . | . | T | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | V | I | S | G | G | - | - | - | G | S | S | T | Y | Y | A | D | S | V | K | G | R | F |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | T | T | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | A | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | I | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | A | . | . | S | . | . | . | . | G | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | | H_FR3 | | | | | | | | | | | |
| CONSENSUS | T | I | S | R | D | N | S | K | N | T | L | Y | L | Q | M | N | S | L | R | A | E | D | T | A | V | Y |
| 21-225_146A2_HC | . | . | . | . | . | . | . | . | . | . | . | M | . | . | . | . | . | . | . | . | . | . | . | . | . | . |
| 21-225_146A3_HC | . | . | . | . | . | . | . | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | L | . | . | . | . | . | . | . | . | . | . | . | . | . | . | . | . | . | . | . | . | . | . | . | F |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | . | . | . | . | . | . | . | . | . | . | . |
| 21-225_148G6_HC | . | . | . | . | . | . | . | . | M | . | . | M | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | . | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | K | W | R | G | N | P | T | - | - | - | - | - | - | - | - | - | - | - | - | - | - | - | - |
| 21-225_146A2_HC | . | | | | | | | | | | | | | | | | | | | | | | | | | |
| 21-225_146A3_HC | | | | | | | | | | | | | | | | | | | | | | | | | | |
| 21-225_146B11_HC | | | | | | | | | | | | | | | | | | | | | | | | | | |
| 21-225_146C11_HC | . | | | | | | | | | | | | | | | | | | | | | | | | | |
| 21-225_147B12_HC | . | | | | | | | | | | | | | | | | | | | | | | | | | |
| 21-225_147E11_HC | . | | | | | | | | | | | | | | | | | | | | | | | | | |
| 21-225_147E7_HC | . | | | | | | | | | | | | | | | | | | | | | | | | . | |
| 21-225_148C6_HC | . | | | | | | | | | | | | | | | | | | | | | | | | . | |
| 21-225_148G11_HC | . | | | | | | | | | | | | | | | | | | | | | | | | . | |
| 21-225_148G6_HC | . | | | | | | | | | | | | | | | | | | | | | | | | . | |
| 21-225_148H11_HC | . | | | | | | | | | | | | | | | | | | | | | | | | . | |
| 21-225_149C10_HC | | | | | | | | | | | | | | | | | | | | | | | | | . | |
| 21-225_149C7_HC | | | | | | | | | | | | | | | | | | | | | | | | | . | |
| 21-225_150F6_HC | | | | | | | | | | | | | | | | | | | | | | | | | . | |
| 21-225_150H11_HC | | | | | | | | | | | | | | | | | | | | | | | | | . | |
| 21-225_151B9_HC | | | | | | | | | | | | | | | | | | | | | | | | | . | |
| 21-225_151H5_HC | | | | | | | | | | | | | | | | | | | | | | | | | . | |
| 21-225_151H7_HC | | | | | | | | | | | | | | | | | | | | | | | | | . | |
| 21-225_152D7_HC | . | | | | | | | | | | | | | | | | | | | | | | | | . | |
| 21-225_152G6_HC | . | | | | | | | | | | | | | | | | | | | | | | | | . | |
| 21-225_154C4_HC | . | | | | | | | | | | | | | | | | | | | | | | | | . | |
| 21-225_154E10_HC | . | | | | | | | | | | | | | | | | | | | | | | | | . | |
| 21-225_155C10_HC | . | | | | | | | | | | | | | | | | | | | | | | | | . | |
| 21-225_207C9_HC | . | | | | A | G | . | T | T | G | S | Y | | | | | | | | | | | | | . | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | D | Y | G | M | D | V | W | G | Q | G | T | T | V | T | V | S | S |
| 21-225_146A2_HC | . | . | . | . | . | . | . | . | . | . | . | . | . | . | . | . | . | . | . |
| 21-225_146A3_HC | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . |
| 21-225_147E7_HC | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . |
| 21-225_148G11_HC | . | . | . | . | . | . | . | . | . | . | . | . | . | . | . | . | . | . | . |
| 21-225_148G6_HC | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . |
| 21-225_149C7_HC | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . |
| 21-225_150H11_HC | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . |
| 21-225_152D7_HC | . | . | . | S | . | . | . | . | . | . | . | . | . | . | . | . | . | . | . |
| 21-225_152G6_HC | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . |
| 21-225_207C9_HC | . | Y | Y | N | . | . | . | . | . | . | . | . | . | . | . | . | . | . | . |

**FIGURE 57**

Table 65.    Consensus 31-VH3|3-33/D4|4-17|RF2/JH4 (SEQ ID NO: 50282):

QVQLVES-GGGVVQPGRSLRLSCAASG-FTFSS-----YGMHWVRQAPGKGLEWVAIIWYD---
GSNKYYADSVKGRFTISRDNSKNTLYLQMNSLRAEDTAVYYCARDHYDFW----------------SGHFDYWGQGTLVTVSS  wherein:

S at position 33 can be substituted with N or T

M at position 41 can be substituted with L

I at position 57 can be substituted with V or A

I at position 58 can be substituted with M

S at position 66 can be substituted with T

N at position 67 can be substituted with Y or S

A at position 71 can be substituted with G, T or V

D at position 109 can be substituted with E

H at position 110 can be substituted with R, Q, A, G, T or Y

Y at position 111 can be substituted with G, F or II

D at position 112 can be substituted with I or F

F at position 113 can be substituted with V or L

W at position 114 can be substituted with G or null (-)

S at position 133 can be substituted with A or null (-)

G at position 134 can be substituted with T or E

H at position 135 can be substituted with Y, W or F

F at position 136 can be substituted with L or S

**FIGURE 57**

D at position 137 can be substituted with A, C or G

Y at position 138 can be substituted with F or S

Xaa Tyr Gly Xaa His (SEQ ID NO: 50253)

Xaa Xaa Trp Tyr Asp Gly Xaa Xaa Lys Tyr Tyr Xaa Asp Ser Val Lys Gly (SEQ ID NO: 50254)

Xaa Xaa Xaa Xaa Xaa Xaa Xaa Xaa Xaa Xaa Xaa Xaa (SEQ ID NO: 50255)

Ser Tyr Gly Met His (SEQ ID NO: 50516)

Ile Ile Trp Tyr Asp Gly Ser Asn Lys Tyr Tyr Ala Asp Ser Val Lys Gly (SEQ ID NO: 50517)

Asp His Tyr Asp Phe Trp Ser Gly His Phe Asp Tyr (SEQ ID NO: 50518)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_147E10_HC | . | . | . | . | . | . | . | . | . | . | . | . | . | . | . | . | X | . | . | . | . | . | . | . | . | . |
| 21-225_148B2_HC | | | . | | . | | . | | . | . | . | . | . | . | . | . | | | . | | . | . | . | | . | . |
| 21-225_148G10_HC | | | . | | . | | . | | . | . | . | . | . | . | . | . | | | . | | . | . | . | | . | . |
| 21-225_149F1_HC | . | | . | . | . | . | . | . | . | . | . | . | . | . | . | . | . | . | . | . | . | . | . | . | . | . |
| 21-225_150B11_HC | . | | . | . | . | . | . | . | . | . | . | . | . | . | . | . | . | . | . | . | . | . | . | . | . | . |
| 21-225_151A10_HC | . | | . | . | . | . | . | . | . | . | . | . | . | . | . | . | . | . | . | . | . | . | . | . | . | . |
| 21-225_151G5_HC | . | | . | . | M | . | . | . | . | . | . | . | . | . | . | . | . | . | . | . | . | . | . | . | . | . |
| 21-225_155A5_HC | . | | . | . | . | . | . | . | . | O | . | . | . | . | . | . | . | . | . | . | . | . | . | . | . | . |
| 21-225_170A1_HC | . | | . | . | . | . | . | . | . | . | . | . | . | . | . | . | . | . | . | . | . | . | . | . | . | . |
| 21-225_176H4_HC | . | | . | . | . | . | . | . | . | . | . | . | . | . | . | . | . | . | . | . | . | . | . | . | . | . |
| 21-225_181C10_HC | . | | . | . | . | . | . | . | . | . | . | . | . | . | . | . | . | . | . | . | . | . | . | . | . | . |
| 21-225_181C2_HC | | | . | | . | . | . | . | . | . | . | . | . | . | . | . | . | . | . | . | . | . | . | V | . | . |
| 21-225_186F7_HC | | | . | | . | . | . | . | . | . | . | . | . | . | . | . | . | . | . | | . | . | . | . | . | . |
| 21-225_189G2_HC | | | . | | . | . | . | . | . | . | . | . | . | . | . | . | . | . | . | | . | . | . | . | . | . |
| 21-225_226A5_HC | | | . | | . | . | . | . | . | . | . | . | . | . | . | . | . | . | . | | . | . | . | . | . | . |
| 21-225_22A1_HC | . | | . | . | . | . | . | . | . | . | . | . | . | . | . | . | . | . | . | . | . | . | . | . | . | . |
| 21-225_28G3_HC | . | | . | . | . | . | . | . | . | . | . | . | . | . | . | . | . | . | . | . | . | . | . | . | . | . |
| 21-225_46F2_HC | . | | . | . | . | . | . | . | . | . | . | . | . | . | . | . | . | . | . | . | . | . | . | . | . | . |
| 21-225_57H3_HC | . | | . | . | . | . | . | . | . | . | . | . | . | . | . | . | . | . | . | . | . | . | . | . | . | . |
| 21-225_62E6_HC | . | | . | . | . | . | . | . | . | . | . | . | . | . | . | . | . | . | . | . | . | . | . | . | . | . |
| 21-225_64H9_HC | . | | . | . | . | . | . | . | . | . | . | . | . | . | . | . | . | . | . | . | . | . | . | . | . | . |
| 21-225_65A6_HC | . | | . | . | . | . | . | . | . | . | . | . | . | . | . | . | . | . | . | . | . | . | . | . | . | . |
| 21-225_67C3_HC | . | | . | . | . | . | . | . | . | . | . | . | . | . | . | . | . | . | . | . | . | . | . | . | . | . |
| 21-225_70A5_HC | . | | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | G | M | H | W | V | R | Q | A | P | G | K | G | L |
| 21-225_147E10_HC | . | . | . | . | . | . | N | . | . | . | . | . | . | . | L | . | . | . | . | . | . | . | . | . | . | . |
| 21-225_148B2_HC | . | . | . | . | . | . | . | . | . | . | . | . | . | . | . | . | . | . | . | . | . | . | . | . | . | . |
| 21-225_148G10_HC | . | . | . | . | . | . | . | . | . | . | . | . | . | . | . | . | . | . | . | . | . | . | . | . | . | . |
| 21-225_149F1_HC | . | . | . | . | . | . | . | . | . | . | . | . | . | . | . | . | . | . | . | . | . | . | . | . | . | . |
| 21-225_150B11_HC | . | . | . | . | . | . | . | . | . | . | . | . | . | . | . | . | . | . | . | . | . | . | . | . | . | . |
| 21-225_151A10_HC | . | . | . | . | . | . | N | . | . | . | . | . | . | . | . | . | . | . | . | . | . | . | . | . | . | . |
| 21-225_151G5_HC | . | . | . | I | . | . | . | . | . | . | . | . | . | . | . | . | . | . | . | . | . | . | . | . | . | . |
| 21-225_155A5_HC | . | . | . | . | . | . | . | . | . | . | . | . | . | . | . | . | . | . | . | . | . | . | . | . | . | . |
| 21-225_170A1_HC | . | . | . | . | . | . | . | . | . | . | . | . | . | . | . | . | . | . | . | . | . | . | . | . | . | . |
| 21-225_176H4_HC | . | . | . | . | . | . | . | . | . | . | . | . | . | . | . | . | . | . | . | . | . | . | . | . | . | . |
| 21-225_181C10_HC | . | . | . | . | . | . | . | . | . | . | . | . | . | . | . | . | . | . | . | . | . | . | . | . | . | . |
| 21-225_181C2_HC | . | . | . | . | . | . | . | . | . | . | . | . | . | . | . | . | . | . | . | . | . | . | . | . | . | . |
| 21-225_186F7_HC | . | . | . | . | . | . | T | . | . | . | . | . | . | . | . | . | . | . | . | . | . | . | . | . | . | . |
| 21-225_189G2_HC | . | . | . | . | . | . | N | . | . | . | . | . | . | . | . | . | . | . | . | . | . | . | . | . | . | . |
| 21-225_226A5_HC | . | . | . | . | . | . | N | . | . | . | . | . | . | . | . | . | . | . | . | . | . | . | . | . | . | . |
| 21-225_22A1_HC | . | . | . | . | . | . | . | . | . | . | . | . | . | . | . | . | . | . | . | . | . | . | . | . | . | . |
| 21-225_28G3_HC | . | . | . | I | . | . | T | . | . | . | . | . | . | . | . | . | . | . | . | . | . | . | . | . | . | . |
| 21-225_46F2_HC | . | . | . | . | . | . | . | . | . | . | . | . | . | . | . | . | . | . | . | . | . | . | . | . | . | . |
| 21-225_57H3_HC | . | . | . | . | . | . | . | . | . | . | . | . | . | . | . | . | . | . | . | . | . | . | . | . | . | . |
| 21-225_62E6_HC | . | . | . | . | . | . | . | . | . | . | . | . | . | . | . | . | . | . | . | . | . | . | . | . | . | . |
| 21-225_64H9_HC | . | . | . | . | . | . | T | . | . | . | . | . | . | . | . | . | . | . | . | . | . | . | . | . | . | . |
| 21-225_65A6_HC | . | . | . | . | . | . | N | . | . | . | . | . | . | . | . | . | . | . | . | . | . | . | . | . | . | . |
| 21-225_67C3_HC | . | . | . | . | . | . | . | . | . | . | . | . | . | . | . | . | . | . | . | . | . | . | . | . | . | . |
| 21-225_70A5_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
|  |  |  |  |  |  |  |  |  |  |  |  |  | H_CDR2 |  |  |  |  |  |  |  |  |  |  |  |  |  |
| CONSENSUS | E | W | V | A | I | I | W | Y | D | - | - | - | G | S | N | K | Y | Y | A | D | S | V | K | G | R | F |
| 21-225_147E10_HC | . | . | . | . | . | . | . | . | . | . | . | . | . | . | . | . | . | . | . | . | . | . | . | . | . | . |
| 21-225_148B2_HC | . | . | . | . | . | . | . | . | . | . | . | . | . | . | Y | . | . | . | . | . | . | . | . | . | . | . |
| 21-225_148G10_HC | . | . | . | . | . | . | . | . | . | . | . | . | . | . | . | . | . | . | . | . | . | . | . | . | . | . |
| 21-225_149F1_HC | . | . | . | . | . | . | . | . | . | . | . | . | . | . | Y | . | . | . | . | . | . | . | . | . | . | . |
| 21-225_150B11_HC | . | . | . | . | . | . | . | . | . | . | . | . | . | . | . | . | . | . | . | . | . | . | . | . | . | . |
| 21-225_151A10_HC | . | . | L | . | . | . | . | . | . | . | . | . | . | . | . | . | . | . | . | . | . | . | . | . | . | . |
| 21-225_151G5_HC | . | . | . | . | . | . | . | . | . | . | . | . | . | . | . | . | . | . | . | . | . | . | . | . | . | . |
| 21-225_155A5_HC | . | . | . | . | . | . | . | . | . | . | . | . | . | . | S | . | . | . | . | . | . | . | . | . | . | . |
| 21-225_170A1_HC | . | . | . | . | . | . | . | . | . | . | . | . | . | . | Y | . | . | . | . | . | . | . | . | . | . | . |
| 21-225_176H4_HC | . | . | . | . | . | . | . | . | . | . | . | . | . | . | Y | . | . | . | . | . | . | . | . | . | . | . |
| 21-225_181C10_HC | . | . | M | . | . | . | . | . | . | . | . | . | . | . | Y | . | . | . | . | . | . | . | . | . | . | . |
| 21-225_181C2_HC | . | . | . | T | . | . | . | . | . | . | . | . | . | . | Y | . | . | . | . | . | . | . | . | . | . | . |
| 21-225_186F7_HC | . | . | . | . | . | . | . | . | . | . | . | . | . | . | Y | . | . | . | . | . | . | . | . | . | . | . |
| 21-225_189G2_HC | . | . | . | . | . | . | . | . | . | . | . | . | . | . | Y | . | . | . | . | . | . | . | . | . | . | . |
| 21-225_226A5_HC | . | . | . | . | . | . | . | . | . | . | . | . | . | T | . | . | . | . | Y | . | . | . | . | . | . | . |
| 21-225_22A1_HC | . | . | . | . | V | M | . | . | . | . | . | . | . | . | . | . | . | . | . | . | . | . | . | . | . | . |
| 21-225_28G3_HC | . | . | . | . | V | . | . | . | . | . | . | . | . | . | . | . | . | . | . | . | . | . | . | . | . | . |
| 21-225_46F2_HC | . | . | . | . | . | . | . | . | . | . | . | . | . | . | . | . | . | . | V | . | . | . | . | . | . | . |
| 21-225_57H3_HC | . | . | . | . | A | . | . | . | . | . | . | . | . | . | . | . | . | . | . | . | . | . | . | . | . | . |
| 21-225_62E6_HC | . | . | . | . | A | . | . | . | . | . | . | . | . | . | . | . | . | . | G | . | . | . | . | . | . | V |
| 21-225_64H9_HC | . | . | . | . | . | . | . | . | . | . | . | . | . | . | . | . | . | . | . | . | . | . | . | . | . | . |
| 21-225_65A6_HC | . | . | . | . | V | . | . | . | . | . | . | . | . | . | . | . | . | . | G | . | . | . | . | . | . | . |
| 21-225_67C3_HC | . | . | . | . | A | . | . | . | . | . | . | . | . | . | . | . | . | . | G | . | . | . | . | . | . | V |
| 21-225_70A5_HC | . | . | . | . | V | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | | H_FR3 | | | | | | | | | | | |
| CONSENSUS | T | I | S | R | D | N | S | K | N | T | L | Y | L | Q | M | N | S | L | R | A | E | D | T | A | V | Y |
| 21-225_147E10_HC | . | . | . | . | . | . | . | . | . | . | . | . | . | . | . | . | . | . | . | . | . | . | . | . | . | . |
| 21-225_148B2_HC | | | . | | . | . | . | . | . | . | . | . | . | . | . | . | . | . | S | . | | | | . | . | . |
| 21-225_148G10_HC | . | . | . | . | . | . | . | . | . | . | . | . | . | . | . | . | . | . | . | . | . | . | . | . | . | . |
| 21-225_149F1_HC | . | . | . | . | . | . | . | . | . | . | . | . | . | . | . | . | . | . | S | . | . | . | . | . | . | . |
| 21-225_150B11_HC | . | . | . | . | . | . | . | . | . | . | . | . | . | . | . | . | . | . | . | . | . | . | . | . | . | . |
| 21-225_151A10_HC | . | . | . | . | . | . | . | . | . | . | . | . | . | . | . | . | . | . | . | . | . | . | . | . | . | . |
| 21-225_151G5_HC | . | . | . | . | . | . | . | . | . | . | . | . | . | . | . | . | . | . | . | . | . | . | . | . | . | . |
| 21-225_155A5_HC | . | . | . | . | . | . | . | . | . | . | . | . | . | :: | . | . | . | . | . | . | . | . | . | . | . | . |
| 21-225_170A1_HC | . | . | . | . | . | . | . | . | . | . | . | . | . | . | . | . | . | . | . | . | . | . | . | . | . | . |
| 21-225_176H4_HC | . | . | . | . | . | . | . | . | . | . | . | . | . | . | . | S | . | . | . | . | . | . | . | . | . | . |
| 21-225_181C10_HC | . | . | . | . | . | . | . | . | . | . | . | . | . | . | . | . | . | . | . | . | . | . | . | . | . | . |
| 21-225_181C2_HC | | . | . | . | . | . | . | . | . | . | . | . | . | . | . | . | . | . | . | . | . | . | . | . | . | . |
| 21-225_186F7_HC | | . | . | . | . | . | . | . | . | . | . | . | . | . | . | . | . | . | . | . | . | . | . | . | . | . |
| 21-225_189G2_HC | | . | . | . | . | . | . | . | . | . | . | . | . | . | . | . | . | . | . | . | . | . | . | . | . | . |
| 21-225_226A5_HC | | . | . | . | . | . | . | . | . | . | . | . | . | . | . | . | . | . | . | . | . | . | . | . | . | . |
| 21-225_22A1_HC | . | . | . | . | . | . | . | . | . | . | . | . | . | . | . | . | . | . | . | . | . | . | . | . | . | . |
| 21-225_28G3_HC | . | . | . | . | . | . | . | . | . | . | . | . | . | . | . | . | . | . | . | . | . | . | . | . | . | . |
| 21-225_46F2_HC | . | . | . | . | . | . | . | . | . | . | . | . | . | . | . | . | . | . | . | . | . | . | . | . | . | . |
| 21-225_57H3_HC | . | . | . | . | . | . | . | . | . | | | | . | . | . | . | . | . | . | . | . | . | . | . | . | . |
| 21-225_62E6_HC | . | . | . | . | . | . | . | . | . | S | . | :: | . | . | . | . | . | . | . | . | . | . | . | . | . | . |
| 21-225_64H9_HC | . | . | . | . | . | . | . | . | . | . | . | . | . | . | . | . | . | . | . | . | . | . | . | . | . | . |
| 21-225_65A6_HC | . | . | . | . | . | . | . | . | . | . | . | . | . | . | . | . | . | . | . | . | . | . | . | . | . | . |
| 21-225_67C3_HC | . | . | . | . | . | . | . | . | . | S | . | :: | . | . | . | . | . | . | . | . | . | . | . | . | . | . |
| 21-225_70A5_HC | . | . | . | . | . | . | . | . | . | S | . | . | . | . | . | . | . | . | S | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | | H_CDR3 | | | | | | |
| CONSENSUS | Y | C | A | R | D | | Y | D | F | W | - | - | - | - | - | - | - | - | - | - | - | - | - | - | - | - |
| 21-225_147E10_HC | | | | | | H | | | | | | | | | | | | | | | | | | | | |
| 21-225_148B2_HC | | | | | | H | F | | | | | | | | | | | | | | | | | | | |
| 21-225_148G10_HC | | | | | | R | | | | | | | | | | | | | | | | | | | | |
| 21-225_149F1_HC | | | | | | H | F | | | | | | | | | | | | | | | | | | | |
| 21-225_150B11_HC | | | | | | R | | | | | | | | | | | | | | | | | | | | |
| 21-225_151A10_HC | | | | | | H | | | | | | | | | | | | | | | | | | | | |
| 21-225_151G5_HC | | | | | | R | | | | | | | | | | | | | | | | | | | | |
| 21-225_155A5_HC | | | | | | R | | | | | | | | | | | | | | | | | | | | |
| 21-225_170A1_HC | | | | | | A | H | | | | | | | | | | | | | | | | | | | |
| 21-225_176H4_HC | | | | | | A | H | | | | | | | | | | | | | | | | | | | |
| 21-225_181C10_HC | | | | | | H | | | | | | | | | | | | | | | | | | | | |
| 21-225_181C2_HC | | | | | | H | | | | | | | | | | | | | | | | | | | | |
| 21-225_186F7_HC | | | | | | H | | | | | | | | | | | | | | | | | | | | |
| 21-225_189G2_HC | | | | | | H | F | | | | | | | | | | | | | | | | | | | |
| 21-225_226A5_HC | | | | E | | G | H | | | | | | | | | | | | | | | | | | | |
| 21-225_22A1_HC | | | | | | G | G | F | L | - | | | | | | | | | | | | | | | | |
| 21-225_28G3_HC | | | | | | R | | | | | | | | | | | | | | | | | | | | |
| 21-225_46F2_HC | | | | | | R | | | | | | | | | | | | | | | | | | | | |
| 21-225_57H3_HC | | | | | | Y | G | I | V | G | | | | | | | | | | | | | | | | |
| 21-225_62E6_HC | | | | | | Q | G | I | V | G | | | | | | | | | | | | | | | | |
| 21-225_64H9_HC | | | | | | T | | | | | | | | | | | | | | | | | | | | |
| 21-225_65A6_HC | | | | | | Q | G | I | V | G | | | | | | | | | | | | | | | | |
| 21-225_67C3_HC | | | | | | Q | G | I | V | G | | | | | | | | | | | | | | | | |
| 21-225_70A5_HC | | | | | | Q | G | I | V | G | | | | | | | | | | | | | | | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  |  |  |  |  | H_FR4 |  |  |  |  |  |  |
| CONSENSUS | - | - | S | G | H | F | D | Y | W | G | Q | G | T | L | V | T | V | S | S |
| 21-225_147E10_HC | . | . | . | . | . | . | . | . | . | . | . | . | . | . | . | . | . | . | . |
| 21-225_148B2_HC | . | . | . | . | . | . | . | . | . | . | . | . | . | . | . | . | . | . | . |
| 21-225_148G10_HC | . | . | . | . | . | . | . | . | . | . | . | . | . | . | . | . | . | . | . |
| 21-225_149F1_HC | . | . | . | . | . | . | . | . | . | . | . | . | . | . | . | . | . | . | . |
| 21-225_150B11_HC | . | . | . | . | . | . | . | . | . | . | . | . | . | . | . | . | . | . | . |
| 21-225_151A10_HC | . | . | . | . | . | . | . | . | . | . | . | . | . | . | . | . | . | . | . |
| 21-225_151G5_HC | . | . | . | . | . | . | . | . | . | . | . | . | . | . | . | . | . | . | . |
| 21-225_155A5_HC | . | . | . | . | . | . | . | . | . | . | . | . | . | . | . | . | . | . | . |
| 21-225_170A1_HC | . | . | . | . | Y | . | . | S | . | . | . | . | . | . | . | . | . | . | . |
| 21-225_176H4_HC | . | . | . | . | Y | . | A | . | . | . | . | . | A | . | . | . | . | . | . |
| 21-225_181C10_HC | . | . | . | . | . | . | . | . | . | . | . | . | . | . | . | . | . | . | . |
| 21-225_181C2_HC | . | . | . | . | . | . | . | P | . | . | . | . | . | . | . | . | . | . | . |
| 21-225_186F7_HC | . | . | . | . | . | . | . | . | . | . | . | . | . | . | . | . | . | . | . |
| 21-225_189G2_HC | . | . | . | . | Y | S | . | . | . | . | . | . | . | . | . | . | . | . | . |
| 21-225_226A5_HC | . | . | . | . | P | . | G | . | . | . | . | . | . | . | . | . | . | . | . |
| 21-225_22A1_HC | . | . | - | E | W | L | . | . | . | . | . | . | . | . | . | . | . | . | . |
| 21-225_28G3_HC | . | . | . | . | Y | . | . | . | . | . | . | . | . | . | . | . | . | . | . |
| 21-225_46F2_HC | . | . | . | . | Y | . | . | . | . | . | . | . | . | . | . | . | . | . | . |
| 21-225_57H3_HC | . | . | A | T | Y | . | . | . | . | . | . | . | . | . | . | . | . | . | . |
| 21-225_62E6_HC | . | . | A | T | W | . | . | . | . | . | . | . | . | . | . | . | . | . | . |
| 21-225_64H9_HC | . | . | . | . | Y | L | G | . | . | . | . | . | . | . | . | . | . | . | . |
| 21-225_65A6_HC | . | . | A | T | W | . | . | . | . | . | . | . | . | . | . | . | . | . | . |
| 21-225_67C3_HC | . | . | A | T | W | . | . | . | . | . | . | . | . | . | . | . | . | . | . |
| 21-225_70A5_HC | . | . | A | T | W | . | . | . | . | . | . | . | . | . | . | . | . | . | . |

FIGURE 57

Table 66.       Consensus 32 -VH4|4-34/D4|4-17|RF2/JH4 (SEQ ID NO: 50283):

QVQLQQW-GAGLLKPSETLSLTCAVYG-GSFS<u>G-----YYWS</u>WIRQPPGKGLEWIG<u>EINH----
SGRTNYNPSLKS</u>RVTISVDTSKNQFSLKLSSVTAADTAVYYCAR<u>DYGG---------------------LDY</u>WGQGTLVTVSS wherein:

G at position 33 can be substituted with V, P, A, D or Y

Y at position 39 can be substituted with C, S or P

Y at position 40 can be substituted with F

I at position 58 can be substituted with S or V

H at position 60 can be substituted with I or Q

R at position 67 can be substituted with S

T at position 68 can be substituted with A or S

N at position 69 can be substituted with T

Y at position 70 can be substituted with F

Xaa Xaa Xaa Trp Ser (SEQ ID NO: 50233)

Glu Xaa Asn Xaa Ser Gly Xaa Xaa Xaa Xaa Asn Pro Ser Leu Lys Ser (SEQ ID NO: 50234)

Asp Tyr Gly Gly Leu Asp Tyr (SEQ ID NO: 50235)

Gly Tyr Tyr Trp Ser (SEQ ID NO: 50519)

Glu Ile Asn His Ser Gly Arg Thr Asn Tyr Asn Pro Ser Leu Lys Ser (SEQ ID NO: 50520)

Asp Tyr Gly Gly Leu Asp Tyr (SEQ ID NO: 50521)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | Q | Q | W | - | G | A | G | L | L | K | P | S | E | T | L | S | L | T | C | A | V | Y |
| 21-225_147G8_HC | . | . | . | . | . | . | . | . | . | . | . | . | . | . | . | . | . | . | . | . | . | . | . | G | . | . |
| 21-225_192F8_HC | . | . | . | . | . | . | . | . | . | . | . | . | . | . | . | . | . | . | . | . | . | . | . | . | . | . |
| 21-225_193E6_HC | . | . | . | . | . | . | . | . | . | . | . | . | . | . | . | . | . | . | . | . | . | . | . | . | . | F |
| 21-225_195B1_HC | . | . | . | . | . | . | . | . | . | . | . | . | . | . | . | . | . | . | . | . | . | . | . | . | . | S |
| 21-225_200A1_HC | . | . | . | . | . | . | . | . | . | . | . | . | . | . | . | . | . | . | . | . | . | . | . | . | . | . |
| 21-225_201B2_HC | . | . | . | . | . | . | . | . | . | . | . | . | . | . | . | . | . | . | . | . | . | . | . | . | . | F |
| 21-225_201E1_HC | . | . | . | . | . | . | . | . | . | . | . | . | . | . | . | . | . | . | . | . | . | . | . | . | . | F |
| 21-225_202D11_HC | . | . | . | . | . | . | R | . | . | . | . | . | . | . | . | . | . | . | . | . | . | . | . | A | . | . |
| 21-225_202D9_HC | . | . | . | . | . | . | . | . | . | . | . | . | . | . | . | . | . | . | . | . | . | . | . | . | . | . |
| 21-225_205G7_HC | . | . | . | . | . | . | . | . | . | . | . | . | . | . | . | . | . | . | . | . | . | . | . | . | . | . |
| 21-225_208A9_HC | . | . | . | . | . | . | . | . | . | . | . | . | . | . | . | . | . | P | . | . | . | . | . | . | . | . |
| 21-225_208B5_HC | . | . | . | . | . | . | . | . | . | . | . | . | . | . | . | . | . | . | . | . | . | . | . | . | . | F |
| 21-225_74C7_HC | . | . | . | . | . | . | . | . | . | . | . | . | . | . | . | . | . | . | . | . | . | . | . | . | . | . |
| 21-225_74D2_HC | . | . | . | . | . | . | . | . | . | . | . | . | . | . | . | . | . | . | . | . | . | . | . | . | . | . |
| 21-225_75G3_HC | . | . | . | . | . | . | . | . | . | . | . | . | . | . | . | . | . | . | . | . | . | . | . | . | . | . |
| 21-225_75G9_HC | . | . | . | . | . | . | . | . | . | . | . | . | . | . | . | . | . | . | . | . | . | . | . | . | . | . |
| 21-225_76A7_HC | . | . | . | . | . | . | . | . | . | . | . | . | . | . | . | . | . | . | . | . | . | . | . | . | . | . |
| 21-225_78E7_HC | . | . | . | . | . | . | . | . | . | . | . | . | . | . | . | . | . | . | . | . | . | . | . | . | . | . |
| 21-225_80A6_HC | . | . | . | . | . | . | . | . | . | . | . | . | . | . | . | . | . | . | . | . | . | . | . | . | . | . |
| 21-225_80B10_HC | . | . | . | . | . | . | . | . | . | . | . | . | . | . | . | . | . | . | . | . | . | . | . | . | . | . |
| 21-225_83B6_HC | . | . | . | . | . | . | . | . | . | . | . | . | . | . | . | . | . | . | . | . | . | . | . | . | . | . |
| 21-225_83C10_HC | . | . | . | . | . | . | . | . | . | . | . | . | . | . | . | . | . | . | . | . | . | . | Y | . | . | . |
| 21-225_85G6_HC | . | . | . | . | . | . | . | . | . | . | . | . | . | . | . | . | . | . | . | . | . | . | . | . | . | . |
| 21-225_93E9_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | | H_FR2 | | |
| CONSENSUS | G | - | G | S | F | S | G | - | - | - | - | - | Y | W | S | W | I | R | Q | P | P | G | K | G | L | |
| 21-225_147G8_HC | . | . | . | . | . | . | A | . | . | . | . | . | Y | . | . | . | . | . | . | . | . | . | . | . | . | . |
| 21-225_192F8_HC | . | . | . | . | . | . | . | . | . | . | . | . | C | . | . | . | . | . | . | . | . | . | . | . | . | . |
| 21-225_193E6_HC | . | . | . | . | . | . | . | . | . | . | . | . | P | . | . | . | . | . | . | . | . | . | . | . | . | . |
| 21-225_195B1_HC | . | . | . | . | . | R | Y | . | . | . | . | . | Y | P | . | . | . | . | . | . | S | | | | | |
| 21-225_200A1_HC | . | . | . | . | . | . | V | . | . | . | . | . | Y | . | . | . | . | . | . | . | . | . | . | . | . | . |
| 21-225_201B2_HC | . | . | . | . | I | . | V | . | . | . | . | . | Y | . | . | T | . | . | . | . | . | . | . | . | . | . |
| 21-225_201E1_HC | D | . | . | . | . | . | P | . | . | . | . | . | Y | . | . | . | . | . | . | . | . | . | . | . | . | . |
| 21-225_202D11_HC | . | . | . | . | . | . | . | . | . | . | . | . | P | . | . | . | . | . | . | . | . | . | . | . | . | . |
| 21-225_202D9_HC | D | . | . | . | . | . | P | . | . | . | . | . | Y | . | . | . | . | . | . | . | . | . | . | . | . | . |
| 21-225_205G7_HC | . | . | . | . | . | . | V | . | . | . | . | . | Y | . | . | . | . | . | . | . | . | . | . | . | . | . |
| 21-225_208A9_HC | . | . | . | . | . | . | V | . | . | . | . | . | S | . | . | . | . | . | . | . | . | . | . | . | . | . |
| 21-225_208B5_HC | . | . | . | . | I | . | V | . | . | . | . | . | Y | . | . | T | . | . | . | . | . | . | . | . | . | . |
| 21-225_74C7_HC | . | . | . | . | L | . | . | . | . | . | . | . | S | . | . | . | . | . | . | . | . | . | . | . | . | . |
| 21-225_74D2_HC | . | . | . | . | . | . | . | . | . | . | . | . | C | . | . | . | . | . | . | . | . | . | . | . | . | . |
| 21-225_75G3_HC | . | . | . | . | L | . | . | . | . | . | . | . | S | . | . | . | . | . | . | . | . | . | . | . | . | . |
| 21-225_75G9_HC | . | . | . | . | . | . | . | . | . | . | . | . | C | . | . | . | . | . | . | . | . | . | . | . | . | . |
| 21-225_76A7_HC | . | . | . | . | . | . | . | . | . | . | . | . | C | . | . | . | . | . | . | . | . | . | . | . | . | . |
| 21-225_78E7_HC | . | . | . | L | . | . | . | . | . | . | . | . | S | . | . | . | . | . | . | . | . | . | . | . | . | . |
| 21-225_80A6_HC | . | . | . | . | . | . | . | . | . | . | . | . | C | . | . | . | . | . | . | . | . | . | . | . | . | . |
| 21-225_80B10_HC | . | . | . | . | . | . | . | . | . | . | . | . | C | . | . | . | . | . | . | . | . | . | . | . | . | . |
| 21-225_83B6_HC | . | . | . | . | . | . | . | . | . | . | . | . | C | . | . | . | . | . | . | . | . | . | . | . | . | . |
| 21-225_83C10_HC | . | . | . | . | . | . | . | . | . | . | . | . | Y | . | . | . | . | . | . | . | . | . | . | . | . | . |
| 21-225_85G6_HC | . | . | . | . | . | . | D | . | . | . | . | . | C | . | . | . | . | . | . | . | . | . | . | . | . | . |
| 21-225_93E9_HC | . | . | . | . | . | . | . | . | . | . | . | . | C | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | E | I | N | H | - | - | - | - | S | G | | T | N | Y | N | P | S | L | K | S | R | V |
| 21-225_147G8_HC | . | . | . | . | . | . | . | . | . | . | . | . | . | . | S | . | . | . | K | . | . | . | . | . | . | . |
| 21-225_192F8_HC | . | . | . | . | . | . | . | Q | . | . | . | . | . | . | N | S | . | . | . | . | . | . | . | . | . | . |
| 21-225_193E6_HC | . | . | . | . | S | . | . | . | . | . | . | . | . | . | N | . | . | . | . | . | . | . | . | . | . | . |
| 21-225_195B1_HC | . | . | P | . | . | . | . | . | . | . | . | . | . | . | N | . | . | . | . | . | . | . | . | . | . | . |
| 21-225_200A1_HC | . | . | . | . | . | . | . | . | . | . | . | . | . | . | S | . | . | . | . | . | . | . | . | . | . | . |
| 21-225_201B2_HC | . | . | . | . | . | . | . | . | . | . | . | . | . | . | S | . | . | . | . | . | . | . | . | . | . | . |
| 21-225_201E1_HC | . | . | . | . | . | V | . | . | . | . | . | . | . | . | S | . | . | . | . | . | . | . | . | . | . | . |
| 21-225_202D11_HC | . | . | . | . | . | . | . | . | . | . | . | . | . | . | S | . | . | . | . | . | . | . | . | . | . | . |
| 21-225_202D9_HC | . | . | . | . | . | . | . | . | . | . | . | . | . | . | S | . | . | . | . | . | . | . | . | . | . | . |
| 21-225_205G7_HC | . | . | . | . | . | S | . | Q | . | . | . | . | . | . | N | . | . | T | . | . | . | . | . | . | . | . |
| 21-225_208A9_HC | . | . | . | . | . | . | . | . | . | . | . | . | . | . | N | A | . | . | . | . | . | . | . | . | . | . |
| 21-225_208B5_HC | . | . | . | . | . | . | . | . | . | . | . | . | . | . | S | . | . | . | . | . | . | . | . | . | . | . |
| 21-225_74C7_HC | . | . | . | . | . | . | . | L | . | . | . | . | . | . | S | . | . | . | . | . | . | . | . | . | . | . |
| 21-225_74D2_HC | . | . | . | . | . | . | . | . | . | . | . | . | . | . | N | . | . | . | . | . | . | . | . | . | . | . |
| 21-225_75G3_HC | . | . | . | . | . | . | . | L | . | . | . | . | . | . | S | . | . | . | . | . | . | . | . | . | . | . |
| 21-225_75G9_HC | . | . | . | . | . | . | . | . | . | . | . | . | . | . | N | . | . | . | . | . | . | . | . | . | . | . |
| 21-225_76A7_HC | . | . | . | . | . | . | . | . | . | . | . | . | . | . | N | . | . | . | . | . | . | . | . | . | . | . |
| 21-225_78E7_HC | . | . | . | . | . | . | . | L | . | . | . | . | . | . | S | . | . | . | . | . | . | . | . | . | . | . |
| 21-225_80A6_HC | . | . | . | . | . | . | . | . | . | . | . | . | . | . | N | . | . | . | . | . | . | . | . | . | . | . |
| 21-225_80B10_HC | . | . | . | . | . | . | . | . | . | . | . | . | . | . | S | . | . | . | . | . | . | . | . | . | . | . |
| 21-225_83B6_HC | . | . | . | . | . | . | . | . | . | . | . | . | . | . | N | . | . | . | . | . | . | . | . | . | . | . |
| 21-225_83C10_HC | . | . | . | . | . | . | . | . | . | . | . | . | . | . | S | . | . | P | . | . | . | . | . | . | . | . |
| 21-225_85G6_HC | . | . | . | . | . | . | . | . | . | . | . | . | . | . | N | . | . | . | . | . | . | . | . | . | . | . |
| 21-225_93E9_HC | . | . | . | . | . | . | . | . | . | . | . | . | . | . | N | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | V | D | T | S | K | N | Q | F | S | L | K | L | S | S | V | T | A | A | D | T | A | V | Y |
| 21-225_147G8_HC | . | . | . | . | . | . | . | . | . | . | . | . | . | . | . | . | . | . | . | . | . | . | . | . | . | . |
| 21-225_192F8_HC | . | . | . | . | . | . | . | T | . | . | . | . | . | . | . | . | . | . | . | . | . | . | . | . | . | . |
| 21-225_193E6_HC | . | . | . | . | . | . | . | . | . | . | . | . | . | . | . | . | . | . | . | . | . | . | . | . | . | . |
| 21-225_195B1_HC | . | . | . | . | . | . | . | . | . | . | . | . | . | . | . | . | . | . | . | . | . | . | . | . | . | . |
| 21-225_200A1_HC | . | . | . | L | . | . | . | . | . | . | . | . | . | . | . | . | . | . | . | . | . | . | . | . | . | . |
| 21-225_201B2_HC | . | . | . | . | . | . | . | . | . | . | . | . | . | . | . | . | . | . | . | . | . | . | . | . | . | . |
| 21-225_201E1_HC | . | . | . | . | . | . | . | . | . | . | . | . | . | . | . | . | . | . | . | . | . | . | . | . | . | . |
| 21-225_202D11_HC | . | . | . | . | . | . | . | . | . | . | . | . | . | . | . | . | . | . | . | . | . | . | . | . | . | . |
| 21-225_202D9_HC | . | . | . | . | . | . | . | . | . | . | . | . | . | . | . | . | . | . | . | . | . | . | . | . | . | . |
| 21-225_205G7_HC | . | . | . | . | . | . | . | . | . | . | . | . | . | N | . | . | . | . | . | . | . | . | . | . | . | . |
| 21-225_208A9_HC | . | . | . | I | . | . | . | . | . | . | . | . | . | . | . | . | . | . | . | . | . | . | . | . | . | . |
| 21-225_208B5_HC | . | . | . | . | . | . | . | . | . | . | . | . | . | . | . | . | . | . | . | . | . | . | . | . | . | . |
| 21-225_74C7_HC | . | . | . | . | . | . | . | . | S | . | . | . | . | T | . | . | . | . | . | . | . | . | . | . | . | . |
| 21-225_74D2_HC | . | . | . | . | . | . | . | S | . | . | . | . | . | . | . | . | . | . | . | . | . | . | . | . | . | . |
| 21-225_75G3_HC | . | . | . | . | . | . | . | . | S | . | . | . | . | T | . | . | . | . | . | . | . | . | . | . | . | . |
| 21-225_75G9_HC | . | . | . | . | . | . | . | S | . | . | . | . | . | . | . | . | . | . | . | . | . | . | . | . | . | . |
| 21-225_76A7_HC | . | . | . | . | . | . | . | S | . | . | . | . | . | . | . | . | . | . | . | . | . | . | . | . | . | . |
| 21-225_78E7_HC | . | . | . | . | . | . | . | . | S | . | . | . | . | T | . | . | . | . | . | . | . | . | . | . | . | . |
| 21-225_80A6_HC | . | . | . | . | . | . | . | S | . | . | . | . | . | . | . | . | . | . | . | . | . | . | . | . | . | . |
| 21-225_80B10_HC | . | . | . | . | . | . | . | . | . | . | . | . | . | . | . | . | . | . | . | . | . | . | . | . | . | . |
| 21-225_83B6_HC | . | . | . | . | . | . | . | S | . | . | . | . | . | . | . | . | . | . | . | . | . | . | . | . | . | . |
| 21-225_83C10_HC | . | . | . | . | . | . | . | . | . | . | . | . | . | . | . | . | . | . | . | . | . | . | . | . | . | F |
| 21-225_85G6_HC | . | . | . | . | . | . | . | S | . | . | . | . | . | . | . | . | . | . | . | . | . | . | . | . | . | . |
| 21-225_93E9_HC | . | . | . | . | . | . | . | S | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | Y | G | G | - | - | - | - | - | - | - | - | - | - | - | - | - | - | - | - | - | - |
| 21-225_147G8_HC | . | . | . | . | . | . | . | V | . | . | . | . | . | . | . | . | . | . | . | . | . | . | . | . | . | . |
| 21-225_192F8_HC | . | . | . | . | . | . | . | V | . | . | . | . | . | . | . | . | . | . | . | . | . | . | . | . | . | . |
| 21-225_193E6_HC | . | . | . | . | . | . | . | . | . | . | . | . | . | . | . | . | . | . | . | . | . | . | . | . | . | . |
| 21-225_195B1_HC | . | . | . | . | . | . | . | A | . | . | . | . | . | . | . | . | . | . | . | . | . | . | . | . | . | . |
| 21-225_200A1_HC | . | . | . | . | . | . | . | V | . | . | . | . | . | . | . | . | . | . | . | . | . | . | . | . | . | . |
| 21-225_201B2_HC | . | . | . | . | . | . | . | V | . | . | . | . | . | . | . | . | . | . | . | . | . | . | . | . | . | . |
| 21-225_201E1_HC | . | . | . | . | . | . | . | . | . | . | . | . | . | . | . | . | . | . | . | . | . | . | . | . | . | . |
| 21-225_202D11_HC | . | . | . | . | . | . | . | V | . | . | . | . | . | . | . | . | . | . | . | . | . | . | . | . | . | . |
| 21-225_202D9_HC | . | . | . | . | . | . | . | . | . | . | . | . | . | . | . | . | . | . | . | . | . | . | . | . | . | . |
| 21-225_205G7_HC | . | . | . | . | . | . | . | V | . | . | . | . | . | . | . | . | . | . | . | . | . | . | . | . | . | . |
| 21-225_208A9_HC | . | . | . | . | . | . | . | V | . | . | . | . | . | . | . | . | . | . | . | . | . | . | . | . | . | . |
| 21-225_208B5_HC | . | . | . | . | . | . | . | V | . | . | . | . | . | . | . | . | . | . | . | . | . | . | . | . | . | . |
| 21-225_74C7_HC | . | . | . | . | . | . | . | . | . | . | . | . | . | . | . | . | . | . | . | . | . | . | . | . | . | . |
| 21-225_74D2_HC | . | . | . | . | . | . | . | . | . | . | . | . | . | . | . | . | . | . | . | . | . | . | . | . | . | . |
| 21-225_75G3_HC | . | . | . | . | . | . | . | . | . | . | . | . | . | . | . | . | . | . | . | . | . | . | . | . | . | . |
| 21-225_75G9_HC | . | . | . | . | . | . | . | . | . | . | . | . | . | . | . | . | . | . | . | . | . | . | . | . | . | . |
| 21-225_76A7_HC | . | . | . | . | . | . | . | . | . | . | . | . | . | . | . | . | . | . | . | . | . | . | . | . | . | . |
| 21-225_78E7_HC | . | . | . | . | . | . | . | . | . | . | . | . | . | . | . | . | . | . | . | . | . | . | . | . | . | . |
| 21-225_80A6_HC | . | . | . | . | . | . | . | . | . | . | . | . | . | . | . | . | . | . | . | . | . | . | . | . | . | . |
| 21-225_80B10_HC | . | . | . | . | . | . | . | . | . | . | . | . | . | . | . | . | . | . | . | . | . | . | . | . | . | . |
| 21-225_83B6_HC | . | . | . | . | . | . | . | . | . | . | . | . | . | . | . | . | . | . | . | . | . | . | . | . | . | . |
| 21-225_83C10_HC | . | . | . | . | . | . | . | . | . | . | . | . | . | . | . | . | . | . | . | . | . | . | . | . | . | . |
| 21-225_85G6_HC | . | . | . | . | . | . | . | . | . | . | . | . | . | . | . | . | . | . | . | . | . | . | . | . | . | . |
| 21-225_93E9_HC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | - | L | D | Y | W | G | Q | G | T | L | V | T | V | S | S |
| 21-225_147G8_HC | . | | . | . | . | F | . | . | . | . | . | . | . | . | . | . | . | . | |
| 21-225_192F8_HC | . | . | . | . | . | F | . | . | . | . | . | . | . | . | . | . | . | . | . |
| 21-225_193E6_HC | . | | . | . | . | . | . | . | . | . | . | . | . | . | . | . | . | . | . |
| 21-225_195B1_HC | . | | . | . | . | F | . | I | . | . | . | . | . | . | . | . | . | . | |
| 21-225_200A1_HC | . | . | . | . | . | F | . | . | . | . | . | . | . | . | . | . | . | . | . |
| 21-225_201B2_HC | . | . | . | . | . | F | . | . | . | . | . | . | . | . | . | . | . | . | . |
| 21-225_201E1_HC | . | | . | . | . | . | . | . | . | . | . | . | A | . | . | . | . | . | . |
| 21-225_202D11_HC | . | | . | . | . | . | . | . | . | . | . | . | . | . | . | . | . | . | . |
| 21-225_202D9_HC | . | | . | . | . | . | . | . | . | . | . | . | . | . | . | . | . | . | . |
| 21-225_205G7_HC | . | | . | . | . | F | . | . | . | . | . | . | . | . | . | . | . | . | . |
| 21-225_208A9_HC | . | . | . | . | . | . | . | . | . | . | . | . | . | . | . | . | . | . | . |
| 21-225_208B5_HC | . | . | . | . | . | F | . | . | . | . | . | . | . | . | . | . | . | . | . |
| 21-225_74C7_HC | . | | . | . | . | . | . | . | . | . | . | . | . | . | . | . | . | . | . |
| 21-225_74D2_HC | . | | . | . | . | . | . | . | . | . | . | . | . | . | . | . | . | . | . |
| 21-225_75G3_HC | . | | . | . | . | . | . | . | . | . | . | . | . | . | . | . | . | . | . |
| 21-225_75G9_HC | . | | . | . | . | . | . | . | . | . | . | . | . | . | . | . | . | . | . |
| 21-225_76A7_HC | . | . | . | . | . | . | . | . | . | . | . | . | A | . | . | . | . | . | . |
| 21-225_78E7_HC | . | | . | . | . | . | . | . | . | . | . | . | . | . | . | . | . | . | . |
| 21-225_80A6_HC | . | | . | . | . | . | . | . | . | . | . | . | . | . | . | . | . | . | . |
| 21-225_80B10_HC | . | | . | . | . | . | . | . | . | . | . | . | . | . | . | . | . | . | . |
| 21-225_83B6_HC | . | | . | . | . | . | . | . | . | . | . | . | . | . | . | . | . | . | . |
| 21-225_83C10_HC | . | | . | . | . | . | . | . | . | . | . | . | . | . | . | . | . | . | . |
| 21-225_85G6_HC | . | | . | . | . | . | . | . | . | . | . | . | . | . | . | . | . | . | . |
| 21-225_93E9_HC | . | | . | . | . | . | . | . | . | . | . | . | . | . | . | . | . | . | . |

FIGURE 57

Table 67.     Consensus 33-VH4|4-39/D1|1-26|RF3/JH4 (SEQ ID NO: 50284):

QLQLQES-GPGLVKPSETLSLTCTVSG-GSISRS---SYYWGWIRQPPGKGLEWIGNIYY----
SGSTYYNPSLKSRVTISVDTSKNQFSLKLSSVTAADTAVYYCARHSSSW--------------------SLDYWGQGTLVTVSS wherein:

R in position 33 can be substituted with G

S in position 38 can be substituted with N

Y in position 39 can be substituted with S

N in position 57 can be substituted with S

S in position 67 can be substituted with Y, A, T or I

T in position 68 can be substituted with A, P or S

Y in position 69 can be substituted with Q, S, A or N

Y in position 70 can be substituted with C or H

N in position 71 can be substituted with I or T

H in position 109 can be substituted with L

S in position 111 can be substituted with T or G

L in position 136 can be substituted with F, I or V

Y in position 138 can be substituted with N, C, D or F

Xaa Ser Xaa Xaa Tyr Trp Gly (SEQ ID NO: 50046)

Xaa Ile Tyr Tyr Ser Gly Xaa Xaa Xaa Xaa Xaa Pro Ser Leu Lys Ser (SEQ ID NO: 50047)

Xaa Ser Xaa Ser Trp Ser Xaa Asp Xaa (SEQ ID NO: 50048)

Arg Ser Ser Tyr Tyr Trp Gly (SEQ ID NO: 50522)

**FIGURE 57**

Asn Ile Tyr Tyr Ser Gly Ser Thr Tyr Tyr Asn Pro Ser Leu Lys Ser (SEQ ID NO: 50523)

His Ser Ser Ser Trp Ser Leu Asp Tyr (SEQ ID NO: 50524)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | L | Q | L | Q | E | S | - | G | P | G | L | V | K | P | S | E | T | L | S | L | T | C | T | V |
| 21-225_11F10_HC | . | . | :: | . | . | . | . | . | . | . | . | . | . | . | . | . | . | . | . | . | . | . | . | . | . |
| 21-225_15A1_HC | . | . | :: | . | . | . | . | . | . | . | . | . | . | . | . | . | . | . | . | . | . | . | . | . | . |
| 21-225_15G7_HC | . | . | . | . | . | . | . | . | . | . | . | . | . | . | . | . | . | . | . | . | . | . | . | . | . |
| 21-225_15H1_HC | . | . | . | . | . | . | . | . | . | . | . | . | . | . | ୫ | . | . | . | . | . | . | . | . | . | . |
| 21-225_16A1_HC | . | . | . | . | . | . | . | . | . | . | . | . | . | . | . | . | . | . | . | . | . | i | . | . | . |
| 21-225_18A3_HC | . | . | . | . | . | . | . | . | . | . | . | . | . | . | ୫ | . | . | . | . | . | . | . | . | . | . |
| 21-225_18C6_HC | . | . | . | . | . | . | . | . | . | . | . | . | . | . | . | . | . | . | . | . | . | . | . | . | . |
| 21-225_20C2_HC | . | . | . | . | . | . | . | . | . | . | . | . | . | . | . | . | . | . | . | . | . | . | . | . | . |
| 21-225_20G9_HC | . | . | . | . | . | . | . | . | . | . | . | . | . | . | . | . | . | . | . | . | . | . | . | . | . |
| 21-225_21G7_HC | . | . | . | . | . | . | . | . | . | . | . | . | . | . | ୫ | . | . | . | . | . | . | . | . | . | . |
| 21-225_22C1_HC | . | . | . | . | . | . | . | . | . | . | . | . | . | . | . | . | . | . | . | . | . | . | . | . | . |
| 21-225_22D12_HC | . | . | . | . | . | . | . | . | . | . | . | . | . | . | . | . | . | . | . | . | . | . | . | . | . |
| 21-225_22G9_HC | . | . | . | . | . | . | . | . | . | . | . | . | . | . | . | . | . | . | . | . | . | . | . | . | . |
| 21-225_23D1_HC | . | . | . | . | . | . | . | . | . | . | . | . | . | . | . | . | . | . | . | . | . | . | . | . | . |
| 21-225_24E5_HC | . | . | . | . | . | . | . | . | . | . | . | . | . | . | . | . | . | . | . | . | . | . | . | . | . |
| 21-225_34C4_HC | . | . | . | . | . | . | . | . | . | . | . | . | . | . | . | . | . | . | . | . | . | . | . | . | . |
| 21-225_59E6_HC | . | . | . | . | . | . | . | . | . | . | . | . | . | . | . | . | . | . | . | . | . | . | . | . | . |
| 21-225_68D12_HC | . | . | . | . | . | . | . | . | . | . | . | . | . | . | . | . | . | . | . | . | . | . | . | . | . |
| 21-225_6A6_HC | . | . | . | . | . | . | . | . | . | . | . | . | . | . | . | . | . | . | . | . | . | . | . | . | . |
| 21-225_6D4_HC | . | . | . | . | . | . | . | . | . | . | . | . | . | . | . | . | . | . | . | . | . | . | . | . | . |
| 21-225_7F4_HC | . | . | . | . | . | . | . | . | . | . | . | . | . | . | . | . | . | . | . | . | . | . | . | . | . |
| 21-225_8D12_HC | . | . | . | . | . | . | . | . | . | . | . | . | . | . | ୫ | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | G | S | I | S | R | S | - | - | - | S | Y | Y | W | G | W | I | R | Q | P | P | G | K | G | L |
| 21-225_11F10_HC | . | . | A | . | . | . | . | . | . | . | . | . | . | . | . | . | . | . | . | . | . | . | . | . | . | . |
| 21-225_15A1_HC | . | . | . | . | . | . | . | . | . | . | . | . | . | . | . | . | . | . | . | . | . | . | . | . | . | . |
| 21-225_15G7_HC | . | . | . | . | . | . | . | . | . | . | . | . | S | . | . | . | . | . | . | . | . | . | . | . | . | . |
| 21-225_15H1_HC | . | . | . | . | . | . | . | . | . | . | . | . | . | . | . | . | . | . | . | . | . | . | . | . | . | . |
| 21-225_16A1_HC | . | . | . | . | . | D | . | . | . | . | . | . | . | . | . | . | . | . | . | . | . | . | . | . | . | . |
| 21-225_18A3_HC | . | . | . | . | . | . | . | . | . | . | . | . | . | . | . | . | . | . | . | . | . | . | . | . | . | . |
| 21-225_18C6_HC | . | . | . | . | . | . | . | . | . | . | . | . | . | . | . | . | . | . | . | . | . | . | . | . | . | . |
| 21-225_20C2_HC | . | . | . | . | . | . | . | . | . | . | . | . | . | . | . | . | . | . | . | . | . | . | . | . | . | . |
| 21-225_20G9_HC | . | . | . | . | . | . | . | . | . | . | . | . | . | . | . | . | . | . | . | . | . | . | . | . | . | . |
| 21-225_21G7_HC | . | . | . | . | . | . | . | . | . | . | . | . | . | . | . | . | . | . | . | . | . | . | . | . | . | Q |
| 21-225_22C1_HC | . | . | . | . | . | N | . | . | . | . | . | . | . | . | . | . | . | . | . | . | . | . | . | . | . | . |
| 21-225_22D12_HC | . | . | . | . | . | . | . | . | . | . | . | . | . | . | . | . | . | . | . | . | . | . | . | . | . | . |
| 21-225_22G9_HC | . | . | . | A | . | . | G | . | . | . | . | N | . | . | . | . | . | . | . | . | . | . | . | . | E | . |
| 21-225_23D1_HC | . | . | . | . | . | . | . | . | . | . | . | . | . | . | . | . | . | . | . | . | . | . | . | . | . | . |
| 21-225_24E5_HC | . | . | . | A | . | . | . | . | . | . | . | . | . | . | . | . | . | . | . | . | . | . | . | . | . | . |
| 21-225_34C4_HC | . | . | . | . | . | . | . | . | . | . | . | . | . | . | . | . | . | . | . | . | S | . | . | . | . | . |
| 21-225_59E6_HC | . | . | . | . | . | . | . | . | . | . | . | . | . | . | . | . | . | . | . | . | . | . | . | . | . | . |
| 21-225_68D12_HC | . | . | . | . | . | . | . | . | . | . | . | . | . | . | . | . | . | . | . | . | . | . | . | . | . | . |
| 21-225_6A6_HC | . | . | . | . | . | . | . | . | . | . | . | . | . | . | . | . | . | . | . | . | . | . | . | . | . | . |
| 21-225_6D4_HC | . | . | . | . | . | . | . | . | . | . | . | . | S | . | . | . | . | . | . | . | . | . | . | . | . | . |
| 21-225_7F4_HC | . | . | . | . | . | . | . | . | . | . | . | . | . | . | . | . | . | . | . | . | . | . | . | . | . | . |
| 21-225_8D12_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | N | I | Y | Y | - | - | - | - | S | G | S | T | Y | Y | N | P | S | L | K | S | R | V |
| 21-225_11F10_HC | . | . | . | . | S | . | . | . | . | . | . | . | . | . | . | A | Q | . | . | . | . | . | . | . | . | . |
| 21-225_15A1_HC | . | . | . | . | S | . | . | . | . | . | . | . | . | . | . | A | Q | . | . | . | . | . | . | . | . | . |
| 21-225_15G7_HC | . | . | . | . | S | . | . | . | . | . | . | . | . | . | Y | . | . | . | T | . | . | . | . | . | . | . |
| 21-225_15H1_HC | . | . | . | . | . | . | . | . | . | . | . | . | . | . | Y | P | . | . | . | . | . | . | . | . | . | . |
| 21-225_16A1_HC | . | . | . | . | S | . | . | . | . | . | . | . | . | . | . | A | . | R | . | . | . | . | . | . | . | G |
| 21-225_18A3_HC | . | . | . | . | . | . | . | . | . | . | . | . | . | . | Y | . | . | . | T | . | . | . | . | . | . | . |
| 21-225_18C6_HC | . | . | . | . | . | . | . | . | . | . | . | . | . | . | Y | . | . | . | . | . | . | . | . | . | . | . |
| 21-225_20C2_HC | . | . | . | . | . | . | . | . | . | . | . | . | . | . | T | A | . | C | . | S | . | . | . | . | . | . |
| 21-225_20G9_HC | . | . | . | . | S | . | . | . | . | . | . | . | . | . | . | A | S | . | . | . | . | . | . | . | . | . |
| 21-225_21G7_HC | . | . | . | . | . | . | . | . | . | . | . | . | . | . | Y | S | . | . | . | . | . | . | . | . | . | . |
| 21-225_22C1_HC | . | . | . | . | . | . | . | . | . | . | . | . | . | . | . | A | . | . | . | S | . | . | . | . | . | . |
| 21-225_22D12_HC | . | . | . | . | . | . | . | . | . | . | . | . | . | . | T | . | S | . | . | . | . | . | . | . | . | . |
| 21-225_22G9_HC | . | . | . | . | . | . | . | . | . | . | . | . | . | . | . | . | . | . | . | . | . | . | . | . | . | . |
| 21-225_23D1_HC | . | . | . | . | S | . | . | . | . | . | . | . | . | . | . | A | Q | . | . | . | . | . | . | . | . | . |
| 21-225_24E5_HC | . | . | . | . | S | . | . | . | . | . | . | . | . | . | . | A | N | . | . | . | . | . | . | . | . | . |
| 21-225_34C4_HC | . | . | . | . | S | . | . | . | . | . | . | . | . | . | . | A | . | . | . | . | . | . | . | . | . | . |
| 21-225_59E6_HC | . | . | . | . | . | . | . | . | . | . | . | . | . | . | . | . | . | . | . | . | . | . | . | . | . | . |
| 21-225_68D12_HC | . | . | . | . | . | . | . | . | . | . | . | . | . | . | . | . | . | . | I | . | . | . | . | . | . | . |
| 21-225_6A6_HC | . | . | . | . | . | . | . | . | . | . | . | . | . | . | I | P | . | . | . | . | . | . | . | . | . | . |
| 21-225_6D4_HC | . | . | . | . | . | . | . | . | . | . | . | . | . | . | . | A | . | . | I | . | . | . | . | . | . | . |
| 21-225_7F4_HC | . | . | . | . | . | . | . | . | . | . | . | . | . | . | A | . | . | . | . | . | . | . | . | . | . | . |
| 21-225_8D12_HC | . | . | . | . | . | . | . | . | . | . | . | . | . | . | Y | P | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_29D9_HC | . | . | . | . | . | . | . | . | . | . | . | . | . | . | . | . | . | . | . | . | . | . | . | . | . | . |
| 21-225_30F3_HC | . | . | . | . | . | . | . | . | . | . | . | . | . | . | . | . | . | . | . | . | . | . | . | . | . | . |
| 21-225_33B1_HC | . | . | . | . | . | . | . | . | . | . | . | . | . | . | . | . | . | . | . | . | . | . | . | . | . | . |
| 21-225_4C8_HC | . | . | . | . | . | . | . | . | . | . | . | . | . | . | . | . | . | . | Y | . | . | . | . | . | . | . |
| 21-225_4F12_HC | . | . | . | . | . | . | . | . | . | . | . | . | . | . | . | . | . | . | Y | P | . | N | . | G | G | . |
| 21-225_50C4_HC | . | . | . | . | . | . | . | . | . | . | . | . | . | . | . | . | . | . | G | . | . | . | . | . | . | . |
| 21-225_73G6_HC | . | . | . | . | . | . | . | . | . | . | . | . | . | . | . | . | . | . | . | . | . | . | . | . | . | . |
| 21-225_90H5_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | V | G | W | - | - | - | - | - | - | - | - | - | - | - | - | - | - | - | - | - | - |
| 21-225_149F12_HC | . | . | . | . | . | I | . | F | . | . | . | . | . | . | . | . | . | . | . | . | . | . | . | . | . | . |
| 21-225_149G1_HC | . | . | . | . | . | I | . | F | . | . | . | . | . | . | . | . | . | . | . | . | . | . | . | . | . | . |
| 21-225_14C2_HC | . | . | . | . | . | . | . | F | . | . | . | . | . | . | . | . | . | . | . | . | . | . | . | . | . | . |
| 21-225_150E7_HC | . | . | . | . | . | I | . | F | . | . | . | . | . | . | . | . | . | . | . | . | . | . | . | . | . | . |
| 21-225_15B11_HC | . | . | . | . | . | . | . | F | . | . | . | . | . | . | . | . | . | . | . | . | . | . | . | . | . | . |
| 21-225_15H10_HC | . | . | V | . | . | . | . | F | . | . | . | . | . | . | . | . | . | . | . | . | . | . | . | . | . | . |
| 21-225_161D11_HC | . | . | . | . | . | I | . | . | . | . | . | . | . | . | . | . | . | . | . | . | . | . | . | . | . | . |
| 21-225_161F7_HC | . | . | . | . | . | I | . | . | . | . | . | . | . | . | . | . | . | . | . | . | . | . | . | . | . | . |
| 21-225_16H4_HC | . | . | . | . | . | M | . | . | . | . | . | . | . | . | . | . | . | . | . | . | . | . | . | . | . | . |
| 21-225_180H7_HC | . | . | . | . | . | . | . | . | . | . | . | . | . | . | . | . | . | . | . | . | . | . | . | . | . | . |
| 21-225_181A8_HC | . | . | . | . | . | . | . | . | . | . | . | . | . | . | . | . | . | . | . | . | . | . | . | . | . | . |
| 21-225_184D11_HC | . | . | . | . | . | . | . | . | . | . | . | . | . | . | . | . | . | . | . | . | . | . | . | . | . | . |
| 21-225_184H10_HC | . | . | . | . | . | . | . | . | . | . | . | . | . | . | . | . | . | . | . | . | . | . | . | . | . | . |
| 21-225_18H12_HC | . | . | . | . | . | . | . | . | . | . | . | . | . | . | . | . | . | . | . | . | . | . | . | . | . | . |
| 21-225_203C10_HC | . | . | . | . | . | . | . | . | . | . | . | . | . | . | . | . | . | . | . | . | . | . | . | . | . | . |
| 21-225_210E4_HC | . | . | . | . | . | T | . | F | . | . | . | . | . | . | . | . | . | . | . | . | . | . | . | . | . | . |
| 21-225_225B6_HC | . | . | . | . | . | . | . | F | . | . | . | . | . | . | . | . | . | . | . | . | . | . | . | . | . | . |
| 21-225_225D6_HC | . | . | . | . | . | . | . | F | . | . | . | . | . | . | . | . | . | . | . | . | . | . | . | . | . | . |
| 21-225_225F8_HC | . | . | . | . | . | . | . | F | . | . | . | . | . | . | . | . | . | . | . | . | . | . | . | . | . | . |
| 21-225_226A9_HC | . | . | . | . | . | . | . | F | . | . | . | . | . | . | . | . | . | . | . | . | . | . | . | . | . | . |
| 21-225_227C12_HC | . | . | . | . | . | . | . | F | . | . | . | . | . | . | . | . | . | . | . | . | . | . | . | . | . | . |
| 21-225_227G3_HC | . | . | . | . | . | . | . | F | . | . | . | . | . | . | . | . | . | . | . | . | . | . | . | . | . | . |
| 21-225_227H5_HC | . | . | . | . | . | . | . | F | . | . | . | . | . | . | . | . | . | . | . | . | . | . | . | . | . | . |
| 21-225_22B7_HC | . | . | . | . | . | . | . | F | . | . | . | . | . | . | . | . | . | . | . | . | . | . | . | . | . | . |
| 21-225_25A9_HC | . | . | . | . | . | I | . | . | . | . | . | . | . | . | . | . | . | . | . | . | . | . | . | . | . | . |
| 21-225_25G5_HC | . | . | . | . | . | I | . | . | . | . | . | . | . | . | . | . | . | . | . | . | . | . | . | . | . | . |
| 21-225_28A9_HC | . | . | . | . | . | I | . | . | . | . | . | . | . | . | . | . | . | . | . | . | . | . | . | . | . | . |
| 21-225_28B8_HC | . | . | . | . | . | I | . | . | . | . | . | . | . | . | . | . | . | . | . | . | . | . | . | . | . | . |
| 21-225_29D8_HC | . | . | G | . | . | I | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | | H_CDR3 | | | | | | |
| CONSENSUS | Y | C | A | R | E | V | G | W | - | - | - | - | - | - | - | - | - | - | - | - | - | - | - | - | - | - |
| 21-225_29D9_HC | . | . | . | . | . | I | . | . | . | . | . | . | . | . | . | . | . | . | . | . | . | . | . | . | . | . |
| 21-225_30F3_HC | . | . | . | . | . | M | . | . | . | . | . | . | . | . | . | . | . | . | . | . | . | . | . | . | . | . |
| 21-225_33B1_HC | . | . | . | . | . | M | . | . | . | . | . | . | . | . | . | . | . | . | . | . | . | . | . | . | . | . |
| 21-225_4C8_HC | . | S | P | . | . | M | . | F | . | . | . | . | . | . | . | . | . | . | . | . | . | . | . | . | . | . |
| 21-225_4F12_HC | K | N | Q | . | . | H | . | Q | . | . | . | . | . | . | . | . | . | . | . | . | . | . | . | . | . | . |
| 21-225_50C4_HC | . | . | . | . | . | . | . | F | . | . | . | . | . | . | . | . | . | . | . | . | . | . | . | . | . | . |
| 21-225_73G6_HC | . | . | . | . | . | . | . | M | . | . | . | . | . | . | . | . | . | . | . | . | . | . | . | . | . | . |
| 21-225_90H5_HC | . | . | V | . | . | M | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | L | D | D | Y | W | G | Q | G | T | L | V | T | V | S | S |
| 21-225_149F12_HC | . | . | . | . | S | E | . | . | . | . | . | . | . | . | . | . | . | . | . |
| 21-225_149G1_HC | . | . | . | . | S | E | . | . | . | . | . | . | . | . | . | . | . | . | . |
| 21-225_14C2_HC | . | . | . | . | R | S | . | . | . | . | . | . | . | . | . | . | . | . | . |
| 21-225_150E7_HC | . | . | . | . | S | E | . | . | . | . | . | . | . | . | . | . | . | . | . |
| 21-225_15B11_HC | . | . | . | . | T | E | . | . | . | . | . | . | . | . | . | . | . | . | . |
| 21-225_15H10_HC | . | . | . | . | . | S | . | . | . | . | . | . | I | . | . | . | . | . | . |
| 21-225_161D11_HC | . | . | . | . | . | S | . | . | . | . | . | . | . | . | . | . | . | . | . |
| 21-225_161F7_HC | . | . | . | . | . | S | . | . | . | . | . | . | . | . | . | . | . | . | . |
| 21-225_16H4_HC | . | . | . | . | T | . | . | O | . | . | . | . | . | . | . | . | . | . | . |
| 21-225_180H7_HC | . | . | . | . | S | . | . | . | . | . | . | . | . | . | . | . | . | . | . |
| 21-225_181A8_HC | . | . | . | . | S | . | . | . | . | . | . | . | . | . | . | . | I | . | . |
| 21-225_184D11_HC | . | . | . | . | N | . | . | . | . | . | . | . | . | . | . | . | . | . | . |
| 21-225_184H10_HC | . | . | . | . | N | . | . | . | . | . | . | . | . | . | . | . | . | . | . |
| 21-225_18H12_HC | . | . | . | . | Y | S | . | . | . | . | . | . | . | . | . | . | . | . | . |
| 21-225_203C10_HC | . | . | . | . | . | . | . | . | . | . | . | . | . | . | . | . | . | . | . |
| 21-225_210E4_HC | . | . | . | . | . | S | . | . | . | . | . | . | . | . | . | . | . | . | . |
| 21-225_225B6_HC | . | . | . | . | T | S | . | . | . | . | . | . | . | . | . | . | . | . | . |
| 21-225_225D6_HC | . | . | . | . | T | S | . | . | . | . | . | . | . | . | . | . | . | . | . |
| 21-225_225F8_HC | . | . | . | . | T | S | . | . | . | . | . | . | . | . | . | . | . | . | . |
| 21-225_226A9_HC | . | . | . | . | T | S | . | . | . | . | . | . | . | . | . | . | . | . | . |
| 21-225_227C12_HC | . | . | . | . | T | S | . | . | . | . | . | . | . | . | . | . | . | . | . |
| 21-225_227G3_HC | . | . | . | . | T | S | . | . | . | . | . | . | . | . | . | . | . | . | . |
| 21-225_227H5_HC | . | . | . | . | T | S | . | . | . | . | . | . | . | . | . | . | . | . | . |
| 21-225_22B7_HC | . | . | . | . | R | S | . | . | . | . | . | . | . | . | . | . | . | . | . |
| 21-225_25A9_HC | . | . | . | . | . | . | . | . | . | . | . | . | . | . | . | . | . | . | . |
| 21-225_25G5_HC | . | . | . | . | . | . | . | . | . | . | . | . | . | . | . | . | . | . | . |
| 21-225_28A9_HC | . | . | . | . | . | . | . | . | . | . | . | . | . | . | . | . | . | . | . |
| 21-225_28B8_HC | . | . | . | . | . | . | . | . | . | . | . | . | . | . | . | . | . | . | . |
| 21-225_29D8_HC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | L | D | D | Y | W | G | Q | G | T | L | V | T | V | S | S |
| 21-225_29D9_HC | | | | | | | | | | | | | | Q | | | | | |
| 21-225_30F3_HC | | | | | Y | | | | | | | | | | | | | | |
| 21-225_33B1_HC | | | | | Y | | | | | | | | | | | | | | |
| 21-225_4C8_HC | | | | | | S | | | | | | | | | | | | | |
| 21-225_4F12_HC | | | | | | S | | | | | | | | | | | | | |
| 21-225_50C4_HC | | | | | | N | | | | | | | | | | | | | |
| 21-225_73G6_HC | | | | | T | S | | | | | | | | | | | | | |
| 21-225_90H5_HC | | | | | | | | | | | | | | | | | | | |

**FIGURE 57**

Table 58.  Consensus 24-VH3|3-21/D4|4-11|RF2/JH4 (SEQ ID NO: 50275)

EVQLVES-GGGLVKPGGSLRLSCAASG-FTFSS-----YSMNWVRQAPGKGLEWVSSISGS---
SSYIYYADSVKGRFTISRDNAKNSLYLQMNSLRAEDTAVYYCARDRG-----------------------SSWGQGTLVTVSS wherein:

S at position 33 can be substituted with T

Y at position 39 can be substituted with F

S at position 40 can be substituted with T, G or R

M at position 41 can be substituted with L

S at position 57 can be substituted with A, C or L

G at position 60 can be substituted with S

S at position 65 can be substituted with G or T

S at position 66 can be substituted with T, G or Y

Y at position 67 can be substituted with H

I at position 68 can be substituted with L or M

Y at position 69 can be substituted with S or W

A at position 71 can be substituted with G, P or V

V at position 74 can be substituted with L

G at position 76 can be substituted with A

D at position 109 can be substituted with T

G at position 111 can be substituted with null (-), S or Y

null (-) at position 112 can be substituted with G

FIGURE 57

null (-) at position 135 can be substituted with S

null (-) at position 136 can be substituted with F or S

S at position 137 can be substituted with D, G or H

S at position 138 can be substituted with L, Y, G, T, C, E or I

Xaa Xaa Xaa Xaa Asn (SEQ ID NO: 50028)

Xaa Ile Ser Xaa Ser Xaa Xaa Xaa Xaa Xaa Tyr Xaa Asp Ser Xaa Lys Xaa (SEQ ID NO: 50029)

Xaa Arg Xaa Xaa Xaa Xaa Xaa Xaa (SEQ ID NO: 50030)

Ser Tyr Ser Met Asn (SEQ ID NO: 50495)

Ser Ile Ser Gly Ser Ser Ser Tyr Ile Tyr Tyr Ala Asp Ser Val Lys Gly (SEQ ID NO: 50496)

Asp Arg Gly Ser Ser (SEQ ID NO: 50497)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | V | E | S | - | G | G | G | L | V | K | P | G | G | S | L | R | L | S | C | A | A | S |
| 21-225_13D3_HC | . | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . | . | F | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | . | . | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | F | . | . | . | . | . |
| 21-225_158G8_HC | . | . | . | . | . | . | . | . | . | . | . | . | . | T | . | . | . | . | . | . | . | . | . | . | . | . |
| 21-225_158H5_HC | . | . | . | . | . | . | . | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . | . | F | . | . | . | . | . |
| 21-225_160H1_HC | . | . | . | . | . | . | . | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | F | . | . | . | . | . |
| 21-225_162H3_HC | . | . | . | . | . | . | . | . | . | . | . | . | . | . | . | . | . | . | . | . | F | . | . | . | . | . |
| 21-225_163F9_HC | . | . | . | . | . | . | . | . | . | . | . | . | . | Q | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | . | . |
| 21-225_17D8_HC | . | . | . | . | . | . | . | . | . | . | . | . | . | . | . | . | . | . | . | . | . | . | . | . | . | . |
| 21-225_20B5_HC | . | . | . | . | . | . | A | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | . | . | . | . | . | . | . | . | . | . | . | . | . | . | . | . | . | . | . | . |
| 21-225_22H3_HC | A | . | . | . | . | . | . | . | . | . | . | . | . | . | . | . | . | . | . | . | . | . | . | . | . | . |
| 21-225_23E11_HC | . | . | . | . | . | . | . | . | . | . | . | . | . | . | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | . | . | . | . | . | . | . | . | . | . | . | . | . | . | . |
| 21-225_43E1_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | |
| CONSENSUS | E | V | Q | L | V | E | S | - | G | G | G | L | V | K | P | G | G | S | L | R | L | S | C | A | A | S |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . | . | . | . | . | . | . | . |
| 21-225_58C5_HC | . | . | . | . | . | . | . | . | . | . | . | . | . | . | . | . | . | . | . | . | . | . | . | . | . | . |
| 21-225_65C12_HC | . | . | . | . | . | . | . | . | . | . | . | . | . | . | . | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | H_CDR1 | | | | | | | | | | | | | H_FR2 | | | |
| CONSENSUS | G | - | F | T | F | S | S | - | - | - | - | - | Y | S | M | N | W | V | R | Q | A | P | G | K | G | L |
| 21-225_13D3_HC | . | . | . | . | . | . | . | . | . | . | . | . | . | T | . | . | . | . | . | . | . | . | . | . | . | . |
| 1-225_14B1_LC1_HC | . | . | . | . | . | . | . | . | . | . | . | . | . | T | . | . | . | . | . | . | . | . | . | . | . | . |
| 1-225_14B1_LC2_HC | . | . | . | . | . | . | . | . | . | . | . | . | . | T | . | . | . | . | . | . | . | . | . | . | . | . |
| 21-225_157G7_HC | . | . | . | . | . | . | . | . | . | . | . | . | . | . | . | . | . | . | . | . | . | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | . | . | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | . | . | . | . | . | . |
| 21-225_158G8_HC | . | . | . | . | . | . | . | . | . | . | . | . | . | T | . | . | . | . | . | . | . | . | . | . | . | . |
| 21-225_158H5_HC | . | . | . | . | . | . | . | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . | . | . | . | . | . | . | . |
| 21-225_160H1_HC | . | . | . | . | . | . | . | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | . | . | . | . | . | . |
| 21-225_162H3_HC | . | . | . | . | . | . | . | . | . | . | . | . | . | . | . | . | . | . | . | . | . | . | . | . | . | . |
| 21-225_163F9_HC | . | . | . | . | . | . | . | . | . | . | . | . | . | . | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | . | . |
| 21-225_17D8_HC | . | . | . | . | . | . | . | . | . | . | . | . | . | . | L | . | . | . | . | . | . | . | . | . | . | . |
| 21-225_20B5_HC | . | . | . | . | . | T | . | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | T | . | . | . | . | . | . | . | . | . | . | . | . | . | . | . | . | . | . | . |
| 21-225_22H3_HC | . | . | . | . | . | . | . | . | . | . | . | . | . | . | . | . | . | . | . | . | . | . | . | . | . | . |
| 21-225_23E11_HC | . | . | . | . | . | . | . | . | . | . | . | . | . | T | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | . | . | T | . | . | . | . | . | . | . | . | . | . | . | . |
| 21-225_43E1_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | H_CDR1 | | | | | | | | | | | | | | H_FR2 | | |
| CONSENSUS | G | - | F | T | F | S | S | - | - | - | - | - | Y | S | M | N | W | V | R | Q | A | P | G | K | G | L |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | S | . | . | . | . | . | . | . | . | . | . | . | . | . | . | . | . | . | . | . | . | . | . |
| 21-225_58C5_HC | . | . | . | . | . | N | . | . | . | . | . | . | . | . | . | . | . | . | . | . | . | . | . | . | . | . |
| 21-225_65C12_HC | . | . | . | . | . | R | . | . | . | . | . | . | F | . | L | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | V | S | S | I | S | G | S | - | - | - | S | S | Y | I | Y | Y | A | D | S | V | K | G | R | F |
| 21-225_13D3_HC | D | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | L | . | P | . | . | . | . | . | . | . | . | . |
| 1-225_14B1_LC2_HC | . | . | . | . | . | . | . | . | . | . | . | . | . | . | L | . | P | . | . | . | . | . | . | . | . | . |
| 21-225_157G7_HC | . | . | . | . | . | . | . | . | . | . | . | . | . | T | . | . | . | . | . | . | . | . | . | . | . | . |
| 21-225_157H4_HC | . | . | . | . | . | . | . | S | . | . | . | . | . | T | . | . | . | . | . | . | . | . | . | . | . | . |
| 21-225_157H7_HC | . | . | . | O | . | . | . | . | . | . | . | . | . | . | . | . | . | . | . | . | . | . | . | . | . | . |
| 21-225_158F5_HC | . | . | . | . | . | . | . | . | . | . | . | . | . | T | . | . | . | . | . | . | . | . | . | . | . | . |
| 21-225_158G8_HC | . | . | . | L | . | . | . | . | . | . | . | . | . | . | . | . | . | . | . | . | . | . | . | . | . | . |
| 21-225_158H5_HC | . | . | . | . | . | . | . | . | . | . | . | . | . | T | . | . | . | . | . | . | . | . | . | . | . | . |
| 21-225_160F4_HC | . | . | . | . | . | . | . | . | . | . | . | . | . | T | . | . | . | . | . | . | . | . | . | . | . | . |
| 21-225_160H1_HC | . | . | . | . | . | . | . | S | . | . | . | . | T | G | . | M | . | . | . | . | . | . | . | . | . | . |
| 21-225_162F2_HC | . | . | . | . | . | . | . | . | . | . | . | . | . | T | . | . | . | . | . | . | . | . | . | . | . | . |
| 21-225_162H3_HC | . | . | . | . | . | . | . | . | . | . | . | . | . | T | . | . | . | . | . | . | . | . | . | . | . | . |
| 21-225_163F9_HC | . | . | . | . | . | . | . | . | . | . | . | . | . | T | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | M | . | . | . | . | . | . | . | . | . | . |
| 21-225_17D8_HC | . | . | . | . | . | . | . | . | . | . | . | . | G | . | H | . | S | . | . | . | . | . | . | . | . | . |
| 21-225_20B5_HC | . | . | . | . | . | . | . | . | . | . | . | . | . | . | . | . | . | . | . | . | . | . | . | . | . | . |
| 21-225_20B8_HC | . | . | . | . | . | . | . | . | . | . | . | . | G | . | H | . | . | . | . | . | . | . | . | . | . | . |
| 21-225_20C8_HC | . | . | . | . | . | . | . | . | . | . | . | . | . | . | . | . | . | . | . | . | . | . | . | A | . | . |
| 21-225_20F9_HC | . | . | . | . | . | . | . | S | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | L | . | . | . | . |
| 21-225_20H10_HC | . | . | . | . | . | . | . | S | . | . | . | . | . | . | . | . | . | . | . | . | . | . | . | . | . | . |
| 21-225_22H3_HC | . | . | . | . | . | . | . | S | . | . | . | . | . | . | . | . | . | . | . | . | . | . | . | . | . | . |
| 21-225_23E11_HC | . | . | . | . | . | . | . | . | . | . | . | . | . | . | . | . | W | . | V | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | S | . | . | . | . | . | . | . | . | . | . | . | . | . | . | . | . | . | . |
| 21-225_33C2_HC | Q | . | . | . | . | . | . | . | . | . | . | . | . | . | . | . | . | . | . | . | . | . | . | . | . | . |
| 21-225_43E1_HC | . | . | . | . | A | . | . | . | N | . | . | . | . | T | . | . | . | . | . | . | . | L | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | V | S | S | I | S | G | S | - | - | - | S | S | Y | I | Y | Y | A | D | S | V | K | G | R | F |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | S | . | . | . | . | . | T | . | . | . | . | G | . | . | . | . | . | . | . |
| 21-225_56H7_HC | . | . | . | . | . | . | . | S | . | . | . | . | . | T | . | . | . | . | G | . | . | . | . | . | . | . |
| 21-225_58C5_HC | . | . | I | . | . | . | . | S | . | . | . | . | . | Y | . | L | . | . | . | . | . | . | . | . | . | . |
| 21-225_65C12_HC | . | . | . | . | . | . | . | S | . | . | . | . | . | . | . | . | . | . | . | . | . | . | . | . | . | . |
| 21-225_6C12_HC | . | . | . | . | . | . | . | S | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | A | K | N | S | L | Y | L | Q | M | N | S | L | R | A | E | D | T | A | V | Y |
| 21-225_13D3_HC | | | | | | | | | | | | | | | | | | | | | | | | | | |
| 1-225_14B1_LC1_HC | | | | | | | | | | | | | | | | | | | | | | | | | | |
| 1-225_14B1_LC2_HC | | | | | | | | | | | | | | | | | | | | | | | | | | |
| 21-225_157G7_HC | | | | | | | | | | | | | | | | | | | | | | | | | | |
| 21-225_157H4_HC | | | | | | | | | | | | | | | | | | | | | | | | | | |
| 21-225_157H7_HC | | M | | | | | | | | | | | | | | | | | | | | | | | | |
| 21-225_158F5_HC | | | | | | | | | | | | | | | | | | | | | | | | | | |
| 21-225_158G8_HC | | | | | | | | | | | | | | | | | | | | | | | | | | |
| 21-225_158H5_HC | | | | | | | | | | | | | | | | | | | | | | | | | | |
| 21-225_160F4_HC | | | | | | | | | | | | | | | | | | | | | | | | | | |
| 21-225_160H1_HC | | | | | | | | | | | | | | | | | | | | | | | | | | |
| 21-225_162F2_HC | | | | | | | | | | | | | | | | | | | | | | | | | | |
| 21-225_162H3_HC | | | | | | | | | | | | | | | | | | | | | | | | | | |
| 21-225_163F9_HC | | | | | | | | | | | | | | | | | | | | | | | | | | |
| 21-225_171A11_HC | | | | | | | | | | | | | | | | | | | | | | | | | | |
| 21-225_17C10_HC | | | | | | | | | | | | | | | | | | | | | | | | | L | |
| 21-225_17D8_HC | | | | | | | | | | | | | | | L | | | | | | | | | | | |
| 21-225_20B5_HC | | | | | | | | | | | | | | | | | | | | | | | | | | |
| 21-225_20B8_HC | | | | | | | | | | | | | | | | | | | | | | | | | | |
| 21-225_20C8_HC | | | | | | | | | | | | | | | | | | | | | | | | | | |
| 21-225_20F9_HC | | | | | | | | | | | | | | | | | | | | | | | | | | |
| 21-225_20G12_HC | | | | | | | | | | | | | | | | | | | | | | | | | | |
| 21-225_20H10_HC | | | | | | | | | | | | | | | | | | | | | | | | I | | |
| 21-225_22H3_HC | | | | | | | | | | | | | | | | | | | | | | | | | | F |
| 21-225_23E11_HC | | | | | | | | | | | | | | | | | | | | | | | | | | |
| 21-225_30H11_HC | | | | | | | | | | | | | | | | | | | | | | | | | | |
| 21-225_31G9_HC | | | | | | | | | | | | | | | | | | | | | | | | | | F |
| 21-225_33C2_HC | | | | | | | | | | | F | | | L | | | | | | | | | | I | | F |
| 21-225_43E1_HC | | | | | | | | | | | F | | | L | | | | | | | | | | | | |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | H_FR3 | | | | | | | | | | | | | | | |
| CONSENSUS | T | I | S | R | D | N | A | K | N | S | L | Y | L | Q | M | N | S | L | R | A | E | D | T | A | V | Y |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . | . | . | . | . | . | . | . |
| 21-225_58C5_HC | . | . | . | . | . | S | . | . | . | . | . | . | . | . | . | . | . | . | . | . | . | . | . | . | . | . |
| 21-225_65C12_HC | . | . | . | . | . | . | . | . | . | . | . | . | . | . | . | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | G | - | - | - | - | - | - | - | - | - | - | - | - | - | - | - | - | - | - | - |
| 21-225_13D3_HC | . | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . | . | . | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | . | V | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | . | . | . | . | . | . |
| 21-225_158G8_HC | . | . | . | . | . | . | . | . | . | . | . | . | . | . | . | . | . | . | . | . | . | . | . | . | . | . |
| 21-225_158H5_HC | . | . | . | . | . | . | . | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . | . | . | . | . | . | . | . |
| 21-225_160H1_HC | F | . | . | . | . | X | - | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | . | . | . | . | . | . |
| 21-225_162H3_HC | . | . | . | . | . | . | . | . | . | . | . | . | . | . | . | . | . | . | . | . | . | . | . | . | . | . |
| 21-225_163F9_HC | . | . | . | . | . | . | . | . | . | . | . | . | . | . | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | . | . |
| 21-225_17D8_HC | . | . | . | . | . | . | . | . | . | . | . | . | . | . | . | . | . | . | . | . | . | . | . | . | . | . |
| 21-225_20B5_HC | . | . | . | . | . | . | . | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | . | . | . | . | . | . | . | . | . | . | . | . | . | . | . | . | . | . | . | . |
| 21-225_22H3_HC | . | . | . | . | . | . | . | . | . | . | . | . | . | . | . | . | . | . | . | . | . | . | . | . | . | . |
| 21-225_23E11_HC | . | . | . | . | . | . | . | . | . | . | . | . | . | . | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | . | . | . | . | . | . | . | . | . | . | . | . | . | . | . |
| 21-225_43E1_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | | H_CDR3 | | | | | | |
| CONSENSUS | Y | C | A | R | D | R | G | - | - | - | - | - | - | - | - | - | - | - | - | - | - | - | - | - | - | - |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . | . | . | . | . | . | . | . |
| 21-225_58C5_HC | . | . | . | . | T | Y | S | G | . | . | . | . | . | . | . | . | . | . | . | . | . | . | . | . | . | . |
| 21-225_65C12_HC | . | . | . | . | . | A | Y | . | . | . | . | . | . | . | . | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  | H_FR4 | | | | | | | | | | |
| CONSENSUS | - | - | - | - | - | - | S | S | W | G | Q | G | T | L | V | T | V | S | S |
| 21-225_13D3_HC | . | . | . | . | . | . | . | Q | . | . | . | . | . | . | . | . | . | . | . |
| 1-225_14B1_LC1_HC | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . |
| 21-225_157H4_HC | . | . | . | . | . | . | . | I | . | . | . | . | . | . | . | . | . | . | . |
| 21-225_157H7_HC | . | . | . | . | . | . | Q | Y | . | . | . | . | . | . | . | . | . | . | . |
| 21-225_158F5_HC | . | . | . | . | . | . | . | . | . | . | . | . | . | . | . | . | . | . | . |
| 21-225_158G8_HC | . | . | . | . | . | . | . | L | . | . | . | . | . | . | . | . | . | . | . |
| 21-225_158H5_HC | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . |
| 21-225_160H1_HC | . | . | . | . | . | . | Q | Y | . | . | . | . | . | . | . | . | . | . | . |
| 21-225_162F2_HC | . | . | . | . | . | . | . | . | . | . | . | . | . | . | . | . | . | . | . |
| 21-225_162H3_HC | . | . | . | . | . | . | . | . | . | . | . | . | . | . | . | . | . | . | . |
| 21-225_163F9_HC | . | . | . | . | . | . | . | L | . | . | . | . | . | . | . | . | . | . | . |
| 21-225_171A11_HC | . | . | . | . | . | . | . | . | . | . | . | . | I | . | . | . | . | . | . |
| 21-225_17C10_HC | . | . | . | . | . | . | . | . | . | . | . | . | . | . | . | . | . | . | . |
| 21-225_17D8_HC | . | . | . | . | . | . | . | Q | . | . | . | . | . | . | . | . | . | . | . |
| 21-225_20B5_HC | . | . | . | . | . | . | . | Y | . | . | . | . | . | . | . | . | . | . | . |
| 21-225_20B8_HC | . | . | . | . | . | . | . | Q | . | . | . | . | . | . | . | . | . | . | . |
| 21-225_20C8_HC | . | . | . | . | . | . | . | L | . | . | . | . | . | . | . | . | . | . | . |
| 21-225_20F9_HC | . | . | . | . | . | . | . | C | . | . | . | . | . | . | . | . | I | . | . |
| 21-225_20G12_HC | . | . | . | . | . | . | . | Y | . | . | . | . | . | . | . | . | . | . | . |
| 21-225_20H10_HC | . | . | . | . | . | . | . | L | . | . | . | . | . | . | . | . | . | . | . |
| 21-225_22H3_HC | . | . | . | . | . | . | . | L | . | . | . | . | . | . | . | . | . | . | . |
| 21-225_23E11_HC | . | . | . | . | . | . | . | L | . | . | . | . | . | . | . | . | . | . | . |
| 21-225_30H11_HC | . | . | . | . | . | . | . | L | . | . | . | . | . | . | . | . | . | . | . |
| 21-225_31G9_HC | . | . | . | . | . | . | . | . | . | . | . | . | . | . | . | . | . | . | . |
| 21-225_33C2_HC | . | . | . | . | . | . | Q | T | . | . | . | . | . | . | . | . | . | . | . |
| 21-225_43E1_HC | . | . | . | . | . | . | . | E | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | - | - | S | S | W | G | Q | G | T | L | V | T | V | S | S |
| 21-225_53G1_HC | . | . | . | . | . | . | . | T | . | . | . | . | . | . | . | . | . | . | . |
| 21-225_56F1_HC | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . |
| 21-225_58C5_HC | . | . | . | . | S | F | D | Y | . | . | . | . | . | . | . | . | . | . | . |
| 21-225_65C12_HC | . | . | . | . | . | S | H | Y | . | . | . | . | . | . | . | . | . | . | . |
| 21-225_6C12_HC | . | . | . | . | . | . | . | L | . | . | . | . | . | . | . | . | . | . | . |

**FIGURE 57**

Table 59.     Consensus 25-VH3|3-23/D6|6-19|RF2/JH3 (SEQ ID NO: 50276)

EVQLLES-GGGLVQPGGSLRLSCAASE-FTFSS-----YAMSWVRQAPGKGLEWVSVISGR---
GGNTFYADSVKGRFTISRDNSKNTLYLQMNSLRAEDTAVYYCASRIAVAG-----------------SEAFDIWGQGTMVTVSS wherein:

S at position 33 can be substituted with G

A at position 40 can be substituted with V

S at position 42 can be substituted with N or T

V at position 57 can be substituted with I or A

G at position 60 can be substituted with R

R at position 61 can be substituted with S

G at position 66 can be substituted with V, I or T

N at position 67 can be substituted with Y, S or T

T at position 68 can be substituted with A

F at position 69 can be substituted with Y

Y at position 70 can be substituted with N or S

K at position 75 can be substituted with R

I at position 110 can be substituted with L, M or V

A at position 113 can be substituted with D

S at position 133 can be substituted with N or Y

E at position 134 can be substituted with D

F at position 136 can be substituted with C

**FIGURE 57**

D at position 137 can be substituted with A or H

I at position 138 can be substituted with V

Xaa Tyr Xaa Met Xaa (SEQ ID NO: 50031)

Xaa Ile Ser Xaa Xaa Gly Xaa Xaa Xaa Xaa Xaa Ala Asp Ser Val Xaa Gly (SEQ ID NO: 50032)

Arg Xaa Ala Val Xaa Gly Xaa Xaa Ala Xaa Xaa Xaa (SEQ ID NO: 50033)

Ser Tyr Ala Met Ser (SEQ ID NO: 50498)

Val Ile Ser Gly Arg Gly Gly Asn Thr Phe Tyr Ala Asp Ser Val Lys Gly (SEQ ID NO: 50499)

Arg Ile Ala Val Ala Gly Ser Glu Ala Phe Asp Ile (SEQ ID NO: 50500)

EP 4 435 105 A2

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_10F12_HC | . | . | . | . | . | . | . | . | . | . | . | . | . | . | . | . | . | . | . | . | . | . | . | . | . | . |
| 21-225_11A5_HC | . | . | . | . | . | . | . | . | . | . | D | . | . | . | . | . | . | . | . | . | . | . | . | . | . | . |
| 21-225_16H6_HC | . | . | . | . | . | . | . | . | . | . | . | . | . | . | . | . | . | . | . | . | . | . | . | . | . | . |
| 21-225_17A12_HC | . | . | . | . | . | . | . | . | . | . | . | . | I | . | . | . | . | . | . | . | . | . | . | . | . | . |
| 21-225_17B4_HC | . | . | . | . | . | . | . | . | . | . | . | . | . | . | . | . | . | . | . | . | . | . | . | . | . | . |
| 21-225_17C6_HC | . | . | . | . | . | . | . | . | . | . | . | . | . | . | . | . | . | . | . | . | . | . | . | . | . | . |
| 21-225_17F11_HC | . | A | . | . | . | . | . | . | . | . | . | . | E | . | . | . | . | . | . | . | . | . | . | . | . | . |
| 21-225_17F7_HC | . | . | . | . | . | . | . | . | . | . | . | . | . | . | . | . | . | . | . | . | . | . | . | . | . | . |
| 21-225_18E12_HC | . | . | . | . | . | . | . | . | . | . | . | . | . | . | . | . | . | . | . | . | . | . | . | . | . | . |
| 21-225_200G1_HC | . | . | . | . | . | . | . | . | . | . | . | . | . | . | . | . | . | . | . | . | . | . | . | . | . | . |
| 21-225_20A4_HC | . | . | . | . | . | . | . | . | . | . | . | . | . | . | . | . | . | . | . | . | . | . | . | . | . | . |
| 21-225_21A2_HC | . | . | . | . | . | . | . | . | . | . | . | . | . | . | . | . | . | . | . | . | . | . | . | . | . | . |
| 21-225_21F4_HC | . | . | . | . | . | . | . | . | . | . | . | . | . | . | . | . | . | . | . | . | . | . | . | . | . | . |
| 21-225_22C5_HC | . | . | . | . | . | . | . | . | . | . | . | . | . | . | V | . | . | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | . | . | . | . | . | . | . | . | . | . | . | . | . | . | . | . | . | . | . | . | . | . |
| 21-225_22G10_HC | . | . | . | . | . | . | . | . | . | . | D | . | . | . | . | . | . | . | . | . | . | . | . | T | . | . |
| 21-225_22G3_HC | . | . | . | . | . | . | . | . | . | . | . | . | . | . | . | . | . | . | . | . | . | . | . | . | . | . |
| 21-225_3B1_HC | . | . | . | . | . | . | . | . | . | . | . | . | . | . | . | . | . | . | . | . | . | . | . | . | . | . |
| 21-225_3E3_HC | . | . | . | . | . | . | . | . | . | . | T | . | . | . | . | . | . | . | . | . | . | . | . | . | . | . |
| 21-225_48C3_HC | . | . | . | . | . | . | . | . | . | . | . | . | . | . | . | . | . | . | . | . | . | . | . | . | . | . |
| 21-225_48D1_HC | . | . | . | . | . | . | . | . | . | . | . | . | . | . | . | . | . | . | . | . | . | . | . | . | . | . |
| 21-225_49G12_HC | . | . | . | . | . | . | . | . | . | . | . | . | . | . | . | . | . | . | . | . | . | . | . | T | . | . |
| 21-225_4H2_HC | . | . | . | . | . | . | . | . | . | . | . | . | . | . | . | . | . | . | . | . | . | . | . | . | . | . |
| 21-225_50H10_HC | . | G | . | . | . | . | . | . | . | . | . | . | . | . | . | . | . | . | . | . | . | . | . | . | . | . |
| 21-225_50H8_HC | . | . | . | . | . | . | . | . | . | . | . | . | . | . | . | . | . | . | . | . | . | . | . | . | . | . |
| 21-225_51H10_HC | . | . | . | . | . | . | . | . | . | . | . | . | . | . | . | . | . | . | . | . | . | . | . | T | . | . |
| 21-225_5F7_HC | . | . | . | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_6D3_HC | . | . | . | . | . | . | . | . | . | . | . | . | . | . | . | . | . | . | . | . | . | . | . | . | . | . |
| 21-225_8E12_HC | . | . | . | . | . | . | . | . | . | . | . | . | . | . | . | . | . | . | . | . | . | . | . | . | . | . |
| 21-225_8G6_HC | . | . | . | . | . | . | . | . | . | . | . | . | . | . | . | . | . | . | . | . | . | . | . | V | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | | H_FR2 | | |
| CONSENSUS | E | - | F | T | F | S | S | - | - | - | - | - | Y | A | M | S | W | V | R | Q | A | P | G | K | G | L |
| 21-225_10F12_HC | . | . | . | . | . | . | . | . | . | . | . | . | . | . | . | . | . | . | . | . | . | . | . | . | . | . |
| 21-225_11A5_HC | . | . | . | . | . | . | . | . | . | . | . | . | . | . | . | . | . | . | . | . | . | . | . | . | . | . |
| 21-225_16H6_HC | G | . | . | . | . | . | . | . | . | . | . | . | . | . | . | N | . | . | . | . | . | . | . | M | . | . |
| 21-225_17A12_HC | . | . | . | . | . | . | . | . | . | . | . | . | . | . | . | . | . | . | . | . | Q | . | . | . | . | . |
| 21-225_17B4_HC | . | . | . | . | . | . | . | . | . | . | . | . | . | . | . | N | . | . | . | . | . | . | . | . | . | . |
| 21-225_17C6_HC | . | . | . | . | . | . | . | . | . | . | . | . | . | . | . | N | . | . | . | . | . | . | . | . | . | . |
| 21-225_17F11_HC | G | . | . | . | . | . | . | . | . | . | . | . | . | . | . | . | . | . | . | . | . | . | . | . | . | . |
| 21-225_17F7_HC | . | . | . | . | . | . | . | . | . | . | . | . | . | . | . | N | . | . | . | . | . | . | . | . | . | . |
| 21-225_18E12_HC | . | . | . | . | . | . | . | . | . | . | . | . | . | . | . | . | . | . | . | . | . | . | . | T | . | . |
| 21-225_200G1_HC | G | . | . | . | . | . | . | . | . | . | . | . | . | . | . | . | . | . | . | . | . | . | . | . | . | . |
| 21-225_20A4_HC | G | . | . | . | . | . | . | . | . | . | . | . | . | . | . | T | . | . | . | . | G | . | . | M | . | . |
| 21-225_21A2_HC | R | . | . | . | . | . | . | . | . | . | . | . | . | . | . | N | . | . | . | . | . | . | . | . | . | . |
| 21-225_21F4_HC | . | . | . | . | . | . | . | . | . | . | . | . | . | . | . | N | . | . | . | . | . | . | . | . | . | . |
| 21-225_22C5_HC | G | . | . | . | . | . | . | . | . | . | . | . | . | . | . | . | . | . | . | . | . | . | . | M | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | . | . | . | . | . | . | . | . | . | . | . | . | . | . | . | . | . | . | . | . | . | . |
| 21-225_22G10_HC | G | . | . | . | . | . | . | . | . | . | . | . | . | . | . | N | . | . | . | . | . | . | . | . | . | . |
| 21-225_22G3_HC | . | . | . | . | . | . | . | . | . | . | . | . | . | . | . | N | . | . | . | . | . | . | . | . | . | . |
| 21-225_3B1_HC | . | . | . | . | . | . | . | . | . | . | . | . | . | . | . | N | . | . | . | . | . | . | . | . | . | . |
| 21-225_3E3_HC | . | . | . | . | . | . | . | . | . | . | . | . | . | V | . | . | . | . | . | . | . | . | . | . | . | . |
| 21-225_48C3_HC | R | . | . | . | . | . | . | . | . | . | . | . | . | . | . | . | . | . | . | . | . | . | . | . | . | . |
| 21-225_48D1_HC | R | . | . | . | . | . | . | . | . | . | . | . | . | . | . | . | . | . | . | . | . | . | . | . | . | . |
| 21-225_49G12_HC | G | . | . | . | . | . | . | . | . | . | . | . | . | . | . | . | . | . | . | . | . | . | . | . | . | . |
| 21-225_4H2_HC | G | . | . | . | . | . | G | . | . | . | . | . | . | . | . | N | . | . | . | . | . | . | . | M | . | . |
| 21-225_50H10_HC | R | . | . | . | . | . | . | . | . | . | . | . | . | . | . | . | . | . | . | . | . | . | . | . | . | . |
| 21-225_50H8_HC | R | . | . | . | . | . | . | . | . | . | . | . | . | . | . | . | . | . | . | . | . | . | . | . | . | . |
| 21-225_51H10_HC | G | . | . | . | . | . | . | . | . | . | . | . | . | . | . | . | . | . | . | . | . | . | . | . | . | . |
| 21-225_5F7_HC | . | . | . | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | H_CDR1 | | | | | | | | | | | | | H_FR2 | | | |
| CONSENSUS | E | - | F | T | F | S | S | - | - | - | - | - | Y | A | M | S | W | V | R | Q | A | P | G | K | G | L |
| 21-225_6D3_HC | . | . | . | . | . | . | . | . | . | . | . | . | . | . | . | N | . | . | . | . | . | . | . | . | . | . |
| 21-225_8E12_HC | . | . | . | . | . | . | . | . | . | . | . | . | . | . | . | . | . | . | . | . | . | . | . | . | . | . |
| 21-225_8G6_HC | . | . | . | . | . | . | . | . | . | . | . | . | . | V | . | N | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | V | I | S | G | R | - | - | - | G | G | N | T | F | Y | A | D | S | V | K | G | R | F |
| 21-225_10F12_HC | . | . | . | . | I | . | . | . | . | . | . | . | . | . | . | . | . | . | . | . | . | . | . | . | . | . |
| 21-225_11A5_HC | . | . | I | . | . | . | . | . | . | . | . | . | . | V | . | . | . | . | . | . | . | . | . | . | . | . |
| 21-225_16H6_HC | . | . | . | . | . | . | . | . | S | . | . | . | . | . | Y | . | Y | . | . | . | . | . | . | . | . | . |
| 21-225_17A12_HC | . | . | . | I | . | . | . | . | . | . | . | . | . | . | . | A | . | . | . | . | . | . | . | . | . | . |
| 21-225_17B4_HC | . | . | . | . | . | . | . | . | . | . | . | . | . | . | . | . | . | . | . | . | . | . | . | . | . | . |
| 21-225_17C6_HC | . | . | . | . | . | . | . | . | . | . | . | . | . | . | . | . | . | . | . | . | . | . | . | . | . | . |
| 21-225_17F11_HC | . | . | . | . | . | . | . | . | . | . | . | . | . | . | . | . | Y | S | . | . | . | . | . | . | . | . |
| 21-225_17F7_HC | . | . | . | . | I | . | . | . | . | . | . | . | . | . | . | . | . | . | . | . | . | . | . | . | . | . |
| 21-225_18E12_HC | . | . | . | . | I | . | . | . | . | . | . | . | . | . | . | . | . | . | . | . | . | . | . | . | . | . |
| 21-225_200G1_HC | . | . | . | A | . | . | . | . | . | . | . | . | . | . | . | . | . | . | . | . | . | . | . | . | . | . |
| 21-225_20A4_HC | . | . | . | . | . | . | . | S | . | . | . | . | . | . | Y | . | Y | N | . | . | . | . | . | . | . | . |
| 21-225_21A2_HC | . | . | I | . | I | . | . | . | . | . | . | . | . | . | . | . | . | . | . | . | . | . | . | . | . | . |
| 21-225_21F4_HC | . | . | . | . | . | . | . | . | . | . | . | . | . | . | . | . | . | . | . | . | . | . | . | . | . | . |
| 21-225_22C5_HC | . | . | . | . | I | . | . | S | . | . | . | . | . | . | Y | . | Y | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | I | . | . | . | . | . | . | . | . | T | . | . | . | . | . | . | . | . | . | . | . | . |
| 21-225_22G10_HC | . | . | . | . | . | . | . | S | . | . | . | . | . | . | . | . | . | . | . | . | . | . | . | . | . | . |
| 21-225_22G3_HC | . | . | . | . | I | . | . | . | . | . | . | . | . | V | . | . | . | . | . | . | . | . | . | . | . | . |
| 21-225_3B1_HC | . | . | . | . | . | . | . | . | . | . | . | . | . | I | . | . | . | . | . | . | . | . | . | . | . | . |
| 21-225_3E3_HC | . | . | . | . | I | . | . | . | . | . | . | . | . | . | . | . | . | . | . | . | . | . | . | . | . | . |
| 21-225_48C3_HC | . | . | . | . | . | . | . | . | . | . | . | . | . | . | S | . | . | . | . | . | . | . | . | . | . | . |
| 21-225_48D1_HC | . | . | . | . | . | . | . | . | . | . | . | . | . | . | S | . | . | . | . | . | . | . | . | . | . | . |
| 21-225_49G12_HC | . | . | . | . | . | . | . | S | . | . | . | . | . | . | T | . | . | . | . | . | . | . | . | . | . | L |
| 21-225_4H2_HC | . | . | . | . | . | . | . | . | S | . | . | . | . | . | Y | . | Y | . | . | . | . | . | . | . | . | . |
| 21-225_50H10_HC | . | . | . | . | . | . | . | . | . | . | . | . | . | . | S | . | . | . | . | . | . | . | . | . | . | . |
| 21-225_50H8_HC | . | . | . | . | . | . | . | . | . | . | . | . | . | . | T | . | . | . | . | . | . | . | . | . | . | . |
| 21-225_51H10_HC | . | . | . | . | . | . | . | S | . | . | . | . | . | . | T | . | . | . | . | . | . | . | . | . | . | L |
| 21-225_5F7_HC | . | . | . | . | I | . | . | . | . | . | . | . | . | . | . | . | . | . | . | . | . | . | . | . | . | . |
| 21-225_5G2_HC | . | . | . | . | . | . | . | . | . | . | . | . | . | V | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | V | S | V | I | S | G | R | - | - | - | G | G | N | T | F | Y | A | D | S | V | K | G | R | F |
| 21-225_6D3_HC | . | . | . | . | . | . | . | . | . | . | . | . | . | . | . | . | . | . | . | . | . | . | . | . | . | . |
| 21-225_8E12_HC | . | . | . | . | ! | . | . | . | . | . | . | . | . | . | . | . | . | . | . | . | . | . | . | . | . | . |
| 21-225_8G6_HC | . | . | . | . | . | . | . | . | . | . | . | . | . | . | . | . | . | . | . | . | . | . | . | . | . | . |

3972

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_10F12_HC | . | . | . | . | . | . | . | . | . | . | . | . | . | . | . | . | . | . | . | . | . | . | . | . | . | . |
| 21-225_11A5_HC | . | . | . | . | . | . | . | . | . | . | . | . | . | . | . | . | . | . | . | . | . | . | . | . | . | . |
| 21-225_16H6_HC | . | . | . | . | . | . | . | . | . | . | . | . | . | . | . | . | . | . | . | . | . | . | . | . | . | . |
| 21-225_17A12_HC | . | . | . | . | . | . | . | . | . | . | . | . | . | . | . | . | . | . | . | . | . | . | . | . | . | . |
| 21-225_17B4_HC | . | . | . | . | . | . | . | . | . | . | . | . | . | . | . | . | . | . | . | . | . | . | . | . | . | . |
| 21-225_17C6_HC | . | . | . | . | . | . | . | . | . | . | . | N | . | . | . | . | . | . | . | . | . | . | . | . | . | . |
| 21-225_17F11_HC | . | . | . | . | . | . | . | . | . | . | . | . | . | . | . | . | . | . | . | . | . | . | . | . | . | . |
| 21-225_17F7_HC | . | . | . | . | . | . | . | . | . | . | . | . | . | . | . | . | . | . | . | . | . | . | . | . | . | . |
| 21-225_18E12_HC | . | . | . | . | . | . | . | . | . | . | . | . | . | . | . | . | . | . | . | . | . | . | . | . | . | . |
| 21-225_200G1_HC | . | . | . | . | . | . | . | . | . | . | . | . | . | . | . | . | . | . | . | . | . | . | . | . | . | . |
| 21-225_20A4_HC | T | . | . | . | . | . | . | . | . | . | . | . | . | . | . | . | . | . | V | . | . | . | . | . | . | . |
| 21-225_21A2_HC | . | . | . | . | . | . | . | . | . | . | . | . | . | . | . | . | . | . | . | . | . | . | . | . | . | . |
| 21-225_21F4_HC | . | . | . | . | . | . | . | . | . | . | . | . | . | . | . | . | . | . | . | . | . | . | . | . | . | . |
| 21-225_22C5_HC | . | . | . | . | . | . | . | . | . | . | . | . | . | . | . | . | . | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | V | . | . | . | . | . | . | . | . | . | . | . | . | . | . | . | . | . | . | . | . | . |
| 21-225_22G10_HC | . | . | . | . | . | . | . | . | . | . | . | N | . | N | . | . | . | . | . | . | . | . | . | . | . | . |
| 21-225_22G3_HC | . | . | . | . | . | . | . | . | . | . | . | . | . | . | . | . | . | . | . | . | . | . | . | . | . | . |
| 21-225_3B1_HC | . | . | . | . | . | . | . | . | . | . | . | . | . | . | . | . | . | . | . | . | . | . | . | . | . | . |
| 21-225_3E3_HC | . | . | . | . | . | . | . | . | . | . | . | . | . | . | . | . | . | . | . | . | . | . | . | . | L | . |
| 21-225_48C3_HC | . | . | . | . | . | . | . | . | . | . | . | . | . | . | . | . | . | . | . | . | . | . | . | . | . | . |
| 21-225_48D1_HC | . | . | . | . | . | . | . | . | . | . | . | . | . | . | . | . | . | . | . | . | . | . | . | . | . | . |
| 21-225_49G12_HC | . | . | . | . | . | . | . | . | . | . | . | . | . | . | . | . | . | . | . | . | . | . | . | . | . | . |
| 21-225_4H2_HC | . | . | . | . | . | . | . | . | . | . | . | . | . | . | . | . | . | . | . | . | . | . | . | . | . | . |
| 21-225_50H10_HC | . | . | . | . | . | . | . | . | . | . | . | . | . | . | . | . | . | . | . | . | . | . | . | . | . | . |
| 21-225_50H8_HC | . | . | . | . | . | . | . | . | . | . | . | . | . | . | . | . | . | . | . | . | . | . | . | . | . | . |
| 21-225_51H10_HC | . | . | . | . | . | . | . | . | . | . | . | . | . | . | . | . | . | . | . | . | . | . | . | . | . | . |
| 21-225_5F7_HC | T | I | S | R | D | N | S | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_6D3_HC | . | . | . | . | . | . | . | . | . | . | . | . | . | . | . | . | . | . | . | . | . | . | . | . | . | . |
| 21-225_8E12_HC | . | . | . | . | . | . | . | . | . | . | . | . | . | . | . | . | . | . | . | . | . | . | . | . | . | . |
| 21-225_8G6_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | S | R | I | A | V | A | G | - | - | - | - | - | - | - | - | - | - | - | - | - | - | - | - |
| 21-225_10F12_HC | . | . | . | . | . | V | . | . | . | . | . | . | . | . | . | . | . | . | . | . | . | . | . | . | . | . |
| 21-225_11A5_HC | . | . | . | . | . | . | . | . | . | . | . | . | . | . | . | . | . | . | . | . | . | . | . | . | . | . |
| 21-225_16H6_HC | . | . | . | . | . | L | . | . | . | . | . | . | . | . | . | . | . | . | . | . | . | . | . | . | . | . |
| 21-225_17A12_HC | . | . | . | . | . | . | . | . | . | . | . | . | . | . | . | . | . | . | . | . | . | . | . | . | . | . |
| 21-225_17B4_HC | . | . | . | . | . | L | . | . | . | . | . | . | . | . | . | . | . | . | . | . | . | . | . | . | . | . |
| 21-225_17C6_HC | . | . | . | . | . | L | . | . | . | . | . | . | . | . | . | . | . | . | . | . | . | . | . | . | . | . |
| 21-225_17F11_HC | . | . | . | . | . | L | . | . | . | . | . | . | . | . | . | . | . | . | . | . | . | . | . | . | . | . |
| 21-225_17F7_HC | . | . | . | . | . | L | . | . | . | . | . | . | . | . | . | . | . | . | . | . | . | . | . | . | . | . |
| 21-225_18E12_HC | . | . | . | . | . | L | . | . | . | . | . | . | . | . | . | . | . | . | . | . | . | . | . | . | . | . |
| 21-225_200G1_HC | . | . | . | R | . | . | . | . | D | . | . | . | . | . | . | . | . | . | . | . | . | . | . | . | . | . |
| 21-225_20A4_HC | . | . | . | . | . | . | . | . | . | . | . | . | . | . | . | . | . | . | . | . | . | . | . | . | . | . |
| 21-225_21A2_HC | . | . | . | . | . | L | . | . | . | . | . | . | . | . | . | . | . | . | . | . | . | . | . | . | . | . |
| 21-225_21F4_HC | . | . | . | . | . | L | . | . | . | . | . | . | . | . | . | . | . | . | . | . | . | . | . | . | . | . |
| 21-225_22C5_HC | . | . | . | . | . | . | . | . | . | . | . | . | . | . | . | . | . | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | . | . | . | . | . | . | . | . | . | . | . | . | . | . | . | . | . | . | . | . | . | . |
| 21-225_22G10_HC | . | . | . | . | . | M | . | . | . | . | . | . | . | . | . | . | . | . | . | . | . | . | . | . | . | . |
| 21-225_22G3_HC | . | . | . | . | . | L | . | . | . | . | . | . | . | . | . | . | . | . | . | . | . | . | . | . | . | . |
| 21-225_3B1_HC | . | . | . | . | . | L | . | . | . | . | . | . | . | . | . | . | . | . | . | . | . | . | . | . | . | . |
| 21-225_3E3_HC | . | . | . | . | . | L | . | . | . | . | . | . | . | . | . | . | . | . | . | . | . | . | . | . | . | . |
| 21-225_48C3_HC | . | . | P | . | . | . | . | . | . | . | . | . | . | . | . | . | . | . | . | . | . | . | . | . | . | . |
| 21-225_48D1_HC | . | . | P | . | . | . | . | . | . | . | . | . | . | . | . | . | . | . | . | . | . | . | . | . | . | . |
| 21-225_49G12_HC | . | . | P | . | . | . | . | . | . | . | . | . | . | . | . | . | . | . | . | . | . | . | . | . | . | . |
| 21-225_4H2_HC | . | . | . | . | . | L | . | . | . | . | . | . | . | . | . | . | . | . | . | . | . | . | . | . | . | . |
| 21-225_50H10_HC | . | . | R | . | . | . | . | . | . | . | . | . | . | . | . | . | . | . | . | . | . | . | . | . | . | . |
| 21-225_50H8_HC | . | . | R | . | . | . | . | . | . | . | . | . | . | . | . | . | . | . | . | . | . | . | . | . | . | . |
| 21-225_51H10_HC | . | . | R | . | . | . | . | . | . | . | . | . | . | . | . | . | . | . | . | . | . | . | . | . | . | . |
| 21-225_5F7_HC | Y | . | . | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | S | R | I | A | V | A | G | - | - | - | - | - | - | - | - | - | - | - | - | - | - | - | - |
| 21-225_6D3_HC | | . | . | . | . | L | . | . | . | . | . | . | . | . | . | . | . | . | . | . | . | . | . | . | . | . |
| 21-225_8E12_HC | . | . | . | . | . | M | . | . | . | . | . | . | . | . | . | . | . | . | . | . | . | . | . | . | . | . |
| 21-225_8G6_HC | . | . | . | . | . | L | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | S | E | A | F | D | I | W | G | Q | G | T | M | V | T | V | S | S |
| 21-225_10F12_HC | . | | . | . | . | . | A | | . | . | . | . | . | . | . | . | . | . | |
| 21-225_11A5_HC | . | | . | . | . | . | . | . | . | . | . | . | . | . | . | . | . | . | . |
| 21-225_16H6_HC | . | | . | . | . | . | . | . | . | . | . | . | . | . | . | . | . | . | |
| 21-225_17A12_HC | . | | . | . | . | . | . | . | . | . | . | . | . | . | . | . | . | . | |
| 21-225_17B4_HC | . | | . | . | . | . | . | . | . | . | . | . | . | . | . | . | . | . | . |
| 21-225_17C6_HC | . | | . | . | . | . | . | . | . | . | . | . | . | . | . | . | . | . | |
| 21-225_17F11_HC | . | | . | . | . | . | H | . | . | . | . | . | . | . | . | . | . | . | |
| 21-225_17F7_HC | | | . | . | . | . | . | . | . | . | . | . | . | . | . | . | . | . | |
| 21-225_18E12_HC | . | | . | . | . | . | . | . | . | . | . | . | . | . | . | . | . | . | . |
| 21-225_200G1_HC | . | | Y | D | . | . | . | V | . | . | . | . | . | . | . | . | . | . | . |
| 21-225_20A4_HC | . | | . | . | . | . | . | . | . | . | . | . | . | . | . | . | . | . | |
| 21-225_21A2_HC | . | | . | . | . | . | . | . | . | . | . | . | . | . | . | . | . | . | . |
| 21-225_21F4_HC | . | | . | . | . | C | . | . | . | . | . | . | . | . | . | . | . | . | . |
| 21-225_22C5_HC | . | | . | . | . | . | . | . | . | . | . | . | . | . | . | . | . | . | . |
| 21-225_22D5_HC | . | | . | . | . | . | . | . | . | . | . | . | . | . | . | . | . | . | . |
| 21-225_22E4_HC | . | | . | . | . | . | . | . | . | . | . | . | . | . | . | . | . | . | . |
| 21-225_22G10_HC | . | | . | . | . | . | . | . | . | . | . | . | . | . | . | . | . | . | . |
| 21-225_22G3_HC | . | | . | . | . | . | . | . | . | . | . | . | . | . | . | . | . | . | . |
| 21-225_3B1_HC | . | | . | . | . | . | . | . | . | . | . | . | . | . | . | . | . | . | . |
| 21-225_3E3_HC | . | | . | . | . | . | . | . | . | . | . | . | . | . | . | . | . | . | . |
| 21-225_48C3_HC | . | | N | . | . | . | . | . | . | . | . | . | . | . | . | . | . | . | . |
| 21-225_48D1_HC | . | | N | . | . | . | . | . | . | . | . | . | . | . | . | . | . | . | . |
| 21-225_49G12_HC | . | | N | D | . | . | . | . | . | . | . | . | . | . | . | . | . | . | . |
| 21-225_4H2_HC | . | | . | . | . | . | . | . | . | . | . | . | . | . | . | . | . | . | . |
| 21-225_50H10_HC | . | | N | . | . | . | . | . | . | . | . | . | . | . | . | . | . | . | . |
| 21-225_50H8_HC | . | | N | . | . | . | . | . | . | . | . | . | . | . | . | . | . | . | . |
| 21-225_51H10_HC | . | | N | D | . | . | . | . | . | . | . | . | . | . | . | . | . | . | . |
| 21-225_5F7_HC | . | | . | . | . | . | A | . | . | . | . | . | . | . | . | . | . | . | . |
| 21-225_5G2_HC | . | | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | S | E | A | F | D | I | W | G | Q | G | T | M | V | T | V | S | S |
| 21-225_6D3_HC | . | . | . | . | . | . | . | . | . | . | . | . | . | . | . | . | . | . | . |
| 21-225_8E12_HC | . | . | . | . | . | . | . | . | . | . | . | . | . | . | . | . | . | . | . |
| 21-225_8G6_HC | . | . | . | . | . | . | . | . | . | . | . | . | . | . | . | S | . | . | . |

**FIGURE 57**

Table 60.     Consensus 26- VH3|3-23/D5|5-12|RF3/JH6 (SEQ ID NO: 50277):

EVQLLES-GGGLVQPGGSLRLSCAASG-FTFS<u>S</u>-----<u>YAMS</u>WVRQAPGKGLEWVS<u>AISGR</u>---
<u>GGSTFIIADSVKG</u>RFTISRDNSKNTLYLQMNSLRAEDTAVYYCVK<u>GELLEDY</u>---------------<u>YFYGMDV</u>WGQGTTVTVSS wherein:

S at position 33 can be substituted with N

Y at position 39 can be substituted with C

A at position 40 can be substituted with V

S at position 42 can be substituted with N

A at position 57 can be substituted with T or S

I at position 58 can be substituted with L

G at position 60 can be substituted with R or V

R at position 61 can be substituted with G

S at position 67 can be substituted with N

T at position 68 can be substituted with I

F at position 69 can be substituted with Y

H at position 70 can be substituted with Y or N

V at position 74 can be substituted with E or M

G at position 109 can be substituted with W

E at position 110 can be substituted with G

E at position 113 can be substituted with Y

D at position 114 can be substituted with S or N

**FIGURE 57**

null (-) at position 116 can be substituted with E

Y at position 132 can be substituted with S

F at position 133 can be substituted with Y

Y at position 134 can be substituted with F

G at position 135 can be substituted with A

M at position 136 can be substituted with I or L

Xaa Xaa Xaa Met Xaa (SEQ ID NO: 50034)

Xaa Xaa Ser Xaa Xaa Gly Gly Xaa Xaa Xaa Xaa Ala Asp Ser Xaa Lys Gly (SEQ ID NO: 50035)

Xaa Xaa Leu Leu Xaa Xaa Tyr Xaa Xaa Xaa Xaa Xaa Xaa Asp Val (SEQ ID NO: 50036)

Ser Tyr Ala Met Ser (SEQ ID NO: 50501)

Ala Ile Ser Gly Arg Gly Gly Ser Thr Phe His Ala Asp Ser Val Lys Gly (SEQ ID NO: 50502)

Gly Glu Leu Leu Glu Asp Tyr Tyr Phe Tyr Gly Met Asp Val (SEQ ID NO: 50503)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_12D8_HC | . | . | . | . | . | . | . | . | . | . | . | . | . | . | . | . | . | . | . | . | . | . | . | . | . | . |
| 21-225_17F12_HC | . | . | . | . | . | . | . | . | . | . | . | . | . | . | . | . | . | . | . | . | . | . | . | . | . | . |
| 21-225_21H4_HC | . | . | . | . | . | . | . | . | . | . | . | . | . | . | . | . | . | . | . | . | . | . | . | . | . | . |
| 21-225_24B1_HC | . | . | . | . | . | . | . | . | . | . | . | . | . | . | . | . | . | . | . | . | . | . | . | . | . | . |
| 21-225_30D1_HC | . | . | . | V | . | . | . | . | . | . | . | . | . | . | . | . | . | . | . | . | . | . | . | . | . | . |
| 21-225_30E3_HC | . | . | . | V | . | . | . | . | . | . | . | . | . | . | . | . | . | . | . | . | . | . | . | . | . | . |
| 21-225_30E9_HC | . | . | . | V | . | . | . | . | . | . | . | . | . | . | . | . | . | . | . | . | . | . | . | . | . | . |
| 21-225_30G11_HC | . | . | . | . | . | . | . | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | . | V | . | . | . | . | . | . | . | . | . | . | . | . | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | A | . | . | . | . | . | . | . | . | . | . | . | . | . |
| 21-225_32G12_HC | . | . | . | . | . | . | . | . | . | . | . | . | L | . | . | . | . | . | . | . | . | . | . | . | . | . |
| 21-225_33A4_HC | . | . | . | . | . | . | . | . | . | . | . | . | . | . | S | . | . | . | . | . | . | . | . | S | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | L | . | . | . | . | . | . | . | . | . | . | . | . | . |
| 21-225_33G7_HC | . | . | . | . | . | . | . | . | . | . | . | . | . | . | . | . | . | . | . | . | . | . | . | . | . | . |
| 21-225_34A6_HC | . | . | . | . | . | . | . | . | . | . | . | . | . | . | . | . | . | . | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | V | . | . | . | . | . | . | . | . | . | . | . | . | . | . | . | . | . | . | . | . | . |
| 21-225_34H11_HC | . | . | . | . | . | . | . | . | . | . | . | . | . | . | . | . | . | . | . | . | . | . | . | . | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | . | . | . | . | . | . | . | . | . | . | . | . | . | . | . | . | . | . | . | . | . | . | . | . | . | . |
| 21-225_36C5_HC | . | . | . | . | V | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_4G12_HC | . | . | . | . | . | . | . | | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | F | T | F | S | S | - | - | - | - | - | Y | A | M | S | W | V | R | Q | A | P | G | K | G | L |
| 21-225_12D8_HC | . | . | . | . | . | . | . | . | . | . | . | . | . | V | . | . | . | . | . | . | . | . | . | . | . | . |
| 21-225_17F12_HC | . | . | . | . | . | . | . | . | . | . | . | . | . | . | . | . | . | . | . | . | . | . | . | . | . | . |
| 21-225_21H4_HC | . | . | . | . | . | . | . | . | . | . | . | . | . | . | . | . | . | . | . | . | . | . | . | . | . | . |
| 21-225_24B1_HC | . | . | . | . | . | . | . | . | . | . | . | . | . | . | . | . | . | . | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | . | . | . | . | . | . | . | . | . | . | . | . | . | N | . | . | . | . | . | . | . | . | . | . |
| 21-225_30E9_HC | . | . | . | . | . | . | . | . | . | . | . | . | C | . | . | N | . | . | . | . | . | . | . | . | . | . |
| 21-225_30G11_HC | . | . | . | . | . | . | . | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | . | . | . | . | . | . | . | . | . | . | . | . | . | N | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | . | . | N | . | . | . | . | . | . | . | . | . | . | . | . | . | . | . | . | . | . | . | . |
| 21-225_33A4_HC | . | . | . | S | . | . | . | . | . | . | . | . | . | . | . | N | . | . | . | . | V | . | . | . | . | . |
| 21-225_33A7_HC | . | . | . | . | . | . | . | . | . | . | . | . | C | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . | . | . | . | . | . | . | . |
| 21-225_33G7_HC | . | . | . | . | . | . | N | . | . | . | . | . | . | . | . | . | . | . | . | . | . | . | . | . | . | . |
| 21-225_34A6_HC | . | . | . | . | N | N | . | . | . | . | . | . | . | . | . | N | . | . | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | . | . | . | . | . | . | . | . | . | . | . | . | . | N | . | . | . | . | . | . | . | E | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | G | . | . | . | N | N | . | . | . | . | . | . | . | . | . | N | . | . | . | . | . | . | . | . | . | . |
| 21-225_36C5_HC | . | . | . | . | . | . | . | . | . | . | . | . | . | . | . | N | . | . | . | . | . | . | . | . | . | . |

3983

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | A | M | S | W | V | R | Q | A | P | G | K | G | L |
| 21-225_4G12_HC | . | . | . | . | . | . | | . | . | . | . | . | . | . | . | . | . | | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | A | I | S | G | R | - | - | - | G | G | S | T | F | H | A | D | S | V | K | G | R | F |
| 21-225_12D8_HC | . | . | . | . | T | . | . | V | G | . | . | . | . | . | . | . | Y | Y | . | . | . | . | . | . | . | . |
| 21-225_17F12_HC | . | . | . | . | . | . | . | . | G | . | . | . | . | . | . | . | Y | Y | . | . | . | . | . | . | . | V |
| 21-225_21H4_HC | . | . | . | . | . | L | . | . | . | . | . | . | . | . | . | . | Y | Y | . | . | . | . | . | . | . | . |
| 21-225_24B1_HC | . | . | . | . | . | . | . | . | . | . | . | . | . | . | . | . | Y | Y | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | . | . | . | . | . | . | . | . | . | . | . | . | . | . | . | Y | . | . | . | S | . | . | . | . |
| 21-225_30E9_HC | . | . | . | . | . | . | . | . | . | . | . | . | . | . | . | . | . | Y | . | . | . | . | . | . | . | . |
| 21-225_30G11_HC | . | . | . | . | . | . | . | . | . | . | . | . | . | . | . | . | . | N | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | . | . | . | . | . | . | . | . | . | . | . | . | N | . | . | Y | . | . | . | . | . | . | . | . |
| 21-225_31H5_HC | . | . | . | . | . | . | . | . | . | . | . | . | . | . | I | . | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | S | . | . | . | . | . | . | . | . | . | . | . | . | . | . | . | . | . | . | . | . | . | . |
| 21-225_33A4_HC | . | . | . | . | . | . | R | . | . | . | . | . | . | . | . | . | . | Y | . | . | . | . | . | . | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | S | . | . | . | . | . | . | . | . | . | . | . | . | . | . | . | . | . | . | . | . | . |
| 21-225_33G7_HC | . | . | . | . | . | . | R | . | . | . | . | . | . | . | . | . | . | Y | . | . | . | . | . | . | Q | . |
| 21-225_34A6_HC | . | . | . | . | . | . | R | . | . | . | . | . | . | . | . | . | . | Y | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | . | . | . | . | . | . | . | . | . | . | . | . | . | . | . | Y | . | . | . | . | . | . | . | . |
| 21-225_35A6_HC | . | . | . | . | T | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | N | . | . | . | . |
| 21-225_36A5_HC | . | . | . | . | . | . | R | . | . | . | . | . | . | N | . | . | . | Y | . | . | . | . | . | . | . | . |
| 21-225_36C5_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | H_CDR2 | | | | | | | | | | | | | |
| CONSENSUS | E | W | V | S | A | I | S | G | R | - | - | - | G | G | S | T | F | H | A | D | S | V | K | G | R | F |
| 21-225_4G12_HC | . | . | . | . | Y | L | . | . | . | . | . | . | . | . | . | . | Y | Y | . | . | . | . | . | . | . | S |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_12D8_HC | | | | | | | | | T | | | | | | | | | | | | | | | | | |
| 21-225_17F12_HC | | | | | | | | | | | | | | | | | | | | | | | | | | |
| 21-225_21H4_HC | | | | | | | | | | | | | | | | | | | | | | | | | | |
| 21-225_24B1_HC | | | | | | | | | | | | | | | | | | | | | | | | | | |
| 21-225_30D1_HC | | | | | | | | | | | | | | | | | | | | | | | | | | |
| 21-225_30E3_HC | | | | | | | | | | | | | | | | | | | | | | | | | L | |
| 21-225_30E9_HC | | | | | | | | | | | | | | | | | | | | | | | | | | |
| 21-225_30G11_HC | | | | | | | | | T | | | | | | | | | | | | | | | | | |
| 21-225_31A8_HC | | | | | | | | | | | | | | | | | | | Q | | | | | | | |
| 21-225_31B8_HC | | | | | | | | | | | | | | | L | | | | | | Q | | | | | |
| 21-225_31H3_HC | | | | | | | | | | | | | | | | | | | | | | | | | | |
| 21-225_31H5_HC | | | | | | | | | | | | | | | | | | | | | | | | | | |
| 21-225_32G1_HC | | | | | | | | | | | | | | | | | | | | | | | | | | |
| 21-225_32G12_HC | | | | | | | | S | | | | | | | | | | | | | | | | | | |
| 21-225_33A4_HC | | | | | | | | | | | | | | | | | | | | | | | | | | |
| 21-225_33A7_HC | | | | | | | | | | | | | | | | | | | | | | | | | | |
| 21-225_33B8_HC | | | | | | | | | | | | | | | | | | | | | | | | | | |
| 21-225_33F1_HC | | | | | | | | | | | | | | | | | | | | | | | | | | |
| 21-225_33G7_HC | | | | | | | | | | | | | | | | | | | | | | | | | | |
| 21-225_34A6_HC | | | | | | | | | | | | | | | | | | | | | | | | | | |
| 21-225_34G8_HC | | | | | | | | | | | | | | | I | | | | | | | | | | | |
| 21-225_34G9_HC | | | | | | | | | | | | | | | | | | | | | | | | | | |
| 21-225_34H11_HC | | | | | | | | | | | P | | | | | | | | | | | | | | | |
| 21-225_35A6_HC | | | | | | | | | | | | | | | | | | | | | | | | | | |
| 21-225_35B7_HC | | | | | | | | | | | | | | | | | | | | | | | | | | |
| 21-225_35D1_HC | | | | | | | | N | | | | | | | | | | | | | | | | | | |
| 21-225_35F11_HC | | | | | | | | | | | | | | | | | | | | | | | | | | |
| 21-225_36A5_HC | | | | | | | | | | | | | | | | | | | | | | | | | | |
| 21-225_36C5_HC | | | | | | | | | | | | | | | | | | | | | | | | | | |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | | |
| CONSENSUS | T | I | S | R | D | N | S | K | N | T | L | Y | L | Q | M | N | S | L | R | A | E | D | T | A | V | Y |
| 21-225_4G12_HC | . | . | . | . | . | . | . | . | . | . | . | . | . | . | . | S | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | V | K | G | E | L | L | E | D | Y | - | - | - | - | - | - | - | - | - | - | - | - | - | - | - |
| 21-225_12D8_HC | . | . | A | . | W | G | R | G | Y | S | . | E | . | . | . | . | . | . | . | . | . | . | . | . | . | . |
| 21-225_17F12_HC | . | . | A | . | W | G | R | G | Y | N | . | E | . | . | . | . | . | . | . | . | . | . | . | . | . | . |
| 21-225_21H4_HC | . | . | A | . | W | G | R | G | Y | S | . | E | . | . | . | . | . | . | . | . | . | . | . | . | . | . |
| 21-225_24B1_HC | . | . | A | . | W | G | R | G | Y | S | . | E | . | . | . | . | . | . | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | A | . | . | . | . | . | . | . | . | . | . | . | . | . | . | . | . | . | . | . | . | . | . | . |
| 21-225_30E9_HC | . | . | A | . | . | . | . | . | . | . | . | . | . | . | . | . | . | . | . | . | . | . | . | . | . | . |
| 21-225_30G11_HC | . | . | . | . | . | . | . | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | A | . | . | . | . | . | . | . | . | . | . | . | . | . | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | . | . | . | . | . | . | . | . | . | . | . | . | . | . | . | . | . | . | . | . | . | . | . |
| 21-225_33A4_HC | F | . | A | . | . | . | . | . | . | . | . | . | . | . | . | . | . | . | . | . | . | . | . | . | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . | . | . | . | . | . | . | . |
| 21-225_33G7_HC | . | . | A | . | . | . | . | . | . | . | . | . | . | . | . | . | . | . | . | A | . | . | . | . | . | . |
| 21-225_34A6_HC | . | . | A | . | . | . | . | . | . | . | . | . | . | . | . | . | . | . | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | A | . | . | . | . | . | . | . | . | . | . | . | . | . | . | . | . | . | . | . | . | . | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | . | . | A | . | . | . | . | . | . | . | . | . | . | . | . | . | . | . | . | . | . | . | . | . | . | . |
| 21-225_36C5_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | V | K | G | E | L | L | E | D | Y | - | - | - | - | - | - | - | - | - | - | - | - | - | - | - |
| 21-225_4G12_HC | . | . | A | . | W | G | R | G | Y | S | . | E | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | Y | F | Y | G | M | D | V | W | G | Q | G | T | T | V | T | V | S | S |
| 21-225_12D8_HC | . | Y | . | . | . | . | . | . | . | . | . | . | . | . | . | . | . | . | . |
| 21-225_17F12_HC | . | . | Y | . | . | . | . | . | . | . | . | . | . | . | . | . | . | . | . |
| 21-225_21H4_HC | . | . | Y | . | . | . | . | . | . | . | . | . | . | . | . | . | . | . | . |
| 21-225_24B1_HC | . | . | Y | . | . | . | . | . | . | . | . | . | . | . | . | . | . | . | . |
| 21-225_30D1_HC | . | . | . | . | L | . | . | . | . | . | . | . | . | . | . | . | . | . | . |
| 21-225_30E3_HC | . | . | Y | . | . | . | . | . | . | . | . | . | . | . | . | . | . | . | . |
| 21-225_30E9_HC | . | . | Y | . | . | . | . | . | . | . | . | . | . | . | . | . | . | . | . |
| 21-225_30G11_HC | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . |
| 21-225_31H3_HC | . | . | Y | . | . | . | . | . | . | . | . | . | . | . | . | . | . | . | . |
| 21-225_31H5_HC | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . |
| 21-225_32G12_HC | . | . | . | . | . | . | . | . | . | . | . | . | . | . | . | . | . | . | . |
| 21-225_33A4_HC | . | S | Y | . | . | I | . | . | . | . | . | . | . | . | . | . | . | . | . |
| 21-225_33A7_HC | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . |
| 21-225_33G7_HC | . | S | Y | . | . | . | . | . | . | . | . | . | . | . | . | . | . | . | . |
| 21-225_34A6_HC | . | . | Y | F | A | . | . | . | . | . | . | . | . | . | . | . | . | . | . |
| 21-225_34G8_HC | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . |
| 21-225_34H11_HC | . | . | Y | . | . | . | . | . | . | . | . | . | . | . | . | . | . | . | . |
| 21-225_35A6_HC | . | . | . | . | . | . | . | . | . | . | . | . | . | . | . | . | . | . | . |
| 21-225_35B7_HC | . | . | . | . | . | . | . | . | . | . | . | . | A | . | . | . | . | . | . |
| 21-225_35D1_HC | . | . | . | . | . | . | . | . | . | . | . | . | A | . | . | . | . | . | . |
| 21-225_35F11_HC | . | . | . | . | . | . | . | . | . | . | . | . | . | . | . | . | . | . | . |
| 21-225_36A5_HC | . | . | Y | . | A | . | . | . | . | . | . | . | . | . | . | . | . | . | . |
| 21-225_36C5_HC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | Y | F | Y | G | M | D | V | W | G | Q | G | T | T | V | T | V | S | S |
| 21-225_4G12_HC | . | . | Y | . | . | . | . | . | . | . | . | . | . | . | . | . | . | . | . |

**FIGURE 57**

Table 61. Consensus 27- VH3|3-33/D4|4-23 RF2/JH6 (SEQ ID NO: 50278):

QVQLVES-GGGVVQPGRSLRLSCAASG-FTFSS-----YGMHWVRQAPGKGLEWVAVIWYD---
GSNKYYADSVKGRFTISRDNSKNTLYLQMNSLRAEDTAVYYCARDRVYCSSTSC----------PYYYYYGMDVWGQGTTVTVSS wherein:

S in position 33 can be substituted with G, D, R, H, T or N

G in position 40 can be substituted with V

M in position 41 can be substituted with L

H in position 42 can be substituted with N

V in position 57 can be substituted with L, F, I or D

I in position 58 can be substituted with F

W in position 59 can be substituted with R

Y in position 60 can be substituted with F

S in position 66 can be substituted with N or T

N in position 67 can be substituted with E, D or K

K in position 68 can be substituted with N or T

Y in position 69 can be substituted with S, D or N

Y in position 70 can be substituted with N

A in position 71 can be substituted with V

G in position 76 can be substituted with D

R in position 110 can be substituted with D, W or N

V in position 111 can be substituted with D, R, F or H

**FIGURE 57**

Y in position 112 can be substituted with S, E, G or F

C in position 113 can be substituted with G or E

S in position 114 can be substituted with G

S in position 115 can be substituted with G, R or N

T in position 116 can be substituted with null (-), S or P

S in position 117 can be substituted with P, null (-) or T

C in position 118 can be substituted with null (-)

null (-) in position 119 can be substituted with S, H, L or Y

P in position 129 can be substituted with null (-), S or Y

Y in position 130 can be substituted with null (-)

Y in position 131 can be substituted with null (-)

Y in position 132 can be substituted with null (-)

Y in position 134 can be substituted with F

G in position 135 can be substituted with A

M in position 136 can be substituted with L

Xaa Tyr Xaa Xaa Xaa (SEQ ID NO: 50037)

Xaa Xaa Xaa Xaa Asp Gly Xaa Xaa Xaa Xaa Xaa Xaa Asp Ser Val Lys Xaa (SEQ ID NO: 50038)

Asp Xaa Xaa Xaa Xaa Xaa Xaa Xaa Xaa Xaa Xaa Xaa Xaa Xaa Xaa Xaa Tyr Xaa Xaa Asp Val (SEQ ID NO: 50039)

Ser Tyr Gly Met His (SEQ ID NO: 50504)

**FIGURE 57**

Val Ile Trp Tyr Asp Gly Ser Asn Lys Tyr Tyr Ala Asp Ser Val Lys Gly (SEQ ID NO: 50505)

Asp Arg Val Tyr Cys Ser Ser Thr Ser Cys Pro Tyr Tyr Tyr Tyr Gly Met Asp Val (SEQ ID NO: 50506)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_146A10_HC | . | . | . | . | M | . | . | . | . | . | . | . | . | . | . | . | . | . | . | . | . | . | . | . | . | . |
| 21-225_147D9_HC | . | . | . | . | . | . | . | . | . | . | . | . | . | . | . | . | . | . | . | . | . | . | . | . | . | . |
| 21-225_148E10_HC | . | . | . | . | . | . | . | . | . | . | . | . | . | . | . | . | . | . | . | . | . | . | . | . | . | . |
| 21-225_149F2_HC | . | . | . | . | G | . | A | . | . | . | . | . | . | . | . | . | . | . | . | . | . | . | . | . | . | . |
| 21-225_150F11_HC | . | . | . | . | G | . | A | . | . | . | . | . | . | . | . | . | . | . | . | . | . | . | . | . | . | . |
| 21-225_151H6_HC | . | . | . | . | . | . | . | . | . | . | . | . | . | . | . | . | . | . | . | . | . | . | . | . | . | . |
| 21-225_152B12_HC | . | . | . | V | . | . | . | . | . | . | . | . | . | . | . | . | . | . | . | . | . | . | . | . | . | . |
| 21-225_152G5_HC | . | . | . | . | . | . | . | . | . | . | . | . | . | . | . | . | . | . | . | . | . | . | . | . | . | . |
| 21-225_153A8_HC | . | . | . | . | M | . | . | . | . | . | . | . | . | . | . | . | . | . | . | . | . | . | . | . | . | . |
| 21-225_153E8_HC | . | . | . | . | . | . | . | . | . | . | . | . | . | . | . | . | . | . | . | . | . | . | . | . | . | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | . | . | . | . | . | . | . | . | . | . | . | . | . | . | . | . | . | . | . | . | . | . |
| 21-225_170F1_HC | . | . | . | . | . | . | . | . | . | . | . | . | . | . | . | . | . | . | . | . | . | . | . | . | . | . |
| 21-225_195D9_HC | . | . | . | . | . | . | . | . | . | . | . | . | . | . | . | . | . | . | . | . | . | . | . | . | . | . |
| 21-225_196A10_HC | . | . | . | . | . | . | . | . | . | . | . | . | . | . | . | . | . | . | . | . | . | . | . | . | . | . |
| 21-225_225A8_HC | . | . | . | . | . | . | . | . | . | . | . | . | . | . | . | . | G | . | . | . | . | . | . | . | . | . |
| 21-225_27G6_HC | . | . | . | . | . | . | . | . | . | . | . | . | . | . | . | . | . | . | . | . | . | . | . | . | . | . |
| 21-225_27H2_HC | . | . | . | . | . | . | . | . | . | . | . | . | . | . | . | . | . | . | . | . | . | . | . | . | . | . |
| 21-225_28A11_HC | . | . | . | . | . | . | . | . | . | . | . | . | . | . | . | . | . | . | . | . | . | . | . | . | . | . |
| 21-225_52H1_HC | . | . | . | . | . | . | . | . | . | . | . | . | . | . | . | . | . | . | . | . | . | . | . | . | . | . |
| 21-225_74B3_HC | . | . | . | . | . | . | . | . | . | . | . | . | . | . | . | . | . | . | . | . | . | . | . | . | . | . |
| 21-225_74C9_HC | . | . | . | . | . | . | . | . | . | . | . | . | . | . | . | . | . | . | . | . | . | . | . | . | . | . |
| 21-225_78E9_HC | . | . | . | . | . | . | . | . | . | . | . | . | . | . | . | . | . | . | . | . | . | . | . | . | . | . |
| 21-225_79E7_HC | . | . | . | . | . | . | . | . | . | . | . | . | . | . | . | . | . | . | . | . | . | . | . | . | . | . |
| 21-225_8C12_HC | . | . | . | . | . | . | . | . | . | . | . | . | . | . | . | . | . | . | . | . | . | . | . | . | . | . |
| 21-225_92A4_HC | . | . | . | . | . | . | . | . | . | . | . | . | . | . | . | . | . | . | . | . | . | . | . | . | . | . |
| 21-225_97E6_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | F | T | F | S | S | - | - | - | - | - | Y | G | M | H | W | V | R | Q | A | P | G | K | G | L |
| 21-225_146A10_HC | | | | | | | G | | | | | | | | | | | | | | | | | | | |
| 21-225_147D9_HC | | | | | | | | | | | | | | | | | | | | | | | | | | |
| 21-225_148E10_HC | | | | | | | | | | | | | | | | | | | | | | | | | | |
| 21-225_149F2_HC | | | | | | | R | | | | | | | | | | | | | | | | | | | |
| 21-225_150F11_HC | | | | | | | R | | | | | | | | | | | | | | | | | | | |
| 21-225_151H6_HC | | | | | | | D | | | | | | | | | | | | | | | | | | | |
| 21-225_152B12_HC | | | | | | | D | | | | | | | | | | | | | | | | | | | |
| 21-225_152G5_HC | | | | | | | D | | | | | | | | | | | | | | | | | | | |
| 21-225_153A8_HC | | | | | | | D | | | | | | | | | | | | | | | | | | | |
| 21-225_153E8_HC | | | | | L | | N | | | | | | | | | | | | | | | | | | | |
| 21-225_158D10_HC | | | | | | | | | | | | | | | | | | | | | | | | | | |
| 21-225_159H8_HC | | | | | | | T | | | | | | | | | | | | | | | | | | | |
| 21-225_160B12_HC | | | | | | | | | | | | | | | | | | | | | | | | | | |
| 21-225_166G11_HC | | | | | | N | G | | | | | | | | | | | | | | | | | | | |
| 21-225_170F1_HC | | | | | | | G | | | | | | | | | N | | | | | | | | | | |
| 21-225_195D9_HC | | | | | | | H | | | | | | | V | | | | | | | | | | | | |
| 21-225_196A10_HC | | | | | | | H | | | | | | | V | | | | | | | | | | | | |
| 21-225_225A9_HC | | | | | | | T | | | | | | | | | | | | | L | | | | | | |
| 21-225_27G6_HC | | | | | | | G | | | | | | | | | | | | | | | | | | | |
| 21-225_27H2_HC | | | | | | | G | | | | | | | | | | | | | | | | | | | |
| 21-225_28A11_HC | | | | | | | G | | | | | | | | L | | | | | | | | | | | |
| 21-225_52H1_HC | | | | | | | G | | | | | | | | | | | | | | | | | | | |
| 21-225_74B3_HC | | | | | | | R | | | | | | | | | | | | | | | | | | | |
| 21-225_74C9_HC | | | | | | | | | | | | | | | | | | | | | | | | | | |
| 21-225_78E9_HC | | | | | | | | | | | | | | | | | | | | | | | | | | |
| 21-225_79E7_HC | | | | | | | | | | | | | | | | | | | | | | | | | | |
| 21-225_8C12_HC | | | | | | G | | | | | | | | | | | | | | | | | | | | |
| 21-225_92A4_HC | | | | | | | | | | | | | | | | | | | | | | | | | | |
| 21-225_97E6_HC | | | | | | | | | | | | | | | | | | | | | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | G | S | N | K | Y | Y | A | D | S | V | K | G | R | F |
| 21-225_146A10_HC | . | . | M | T | L | . | . | F | . | . | . | . | . | . | D | . | N | . | . | . | . | . | . | . | . | . |
| 21-225_147D9_HC | . | . | . | . | D | . | . | F | . | . | . | . | . | . | . | . | . | . | V | . | . | . | . | . | D | . |
| 21-225_148E10_HC | . | . | . | . | . | . | . | F | . | . | . | . | . | . | . | . | . | . | V | . | . | . | . | . | D | . |
| 21-225_149F2_HC | . | . | . | . | F | . | . | . | . | . | . | . | . | . | E | N | . | . | . | . | . | . | . | . | . | . |
| 21-225_150F11_HC | . | . | . | . | F | . | . | . | . | . | . | . | . | . | E | N | . | . | . | . | . | . | . | . | . | . |
| 21-225_151H6_HC | . | . | . | . | L | . | . | . | . | . | . | . | . | . | . | . | . | . | . | . | . | . | . | . | . | . |
| 21-225_152B12_HC | . | . | . | . | . | . | . | . | . | . | . | . | . | . | . | . | . | . | . | . | . | . | . | . | . | . |
| 21-225_152G5_HC | . | . | . | . | L | . | . | . | . | . | . | . | . | . | . | . | . | . | . | . | . | . | . | . | . | . |
| 21-225_153A8_HC | . | . | . | . | L | . | . | . | . | . | . | . | . | . | . | . | . | . | . | . | . | . | . | . | . | . |
| 21-225_153E8_HC | . | . | . | . | . | . | . | F | . | . | . | . | . | . | . | . | . | . | V | . | . | . | . | . | D | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | . | . | . | . | . | . | . | . | . | . | . | . | . | . | . | . | . | . | . | . | . | . |
| 21-225_170F1_HC | . | . | . | . | I | . | . | . | . | . | . | . | . | N | . | . | . | . | . | . | . | . | . | . | . | . |
| 21-225_195D9_HC | . | . | . | . | . | . | . | . | . | . | . | . | . | T | . | . | . | . | . | . | . | . | . | . | . | . |
| 21-225_196A10_HC | . | . | . | . | . | . | . | . | . | . | . | . | . | T | . | . | . | . | . | . | . | . | . | . | . | . |
| 21-225_225A8_HC | . | . | . | . | . | . | . | . | . | . | . | . | . | . | . | . | . | . | . | . | . | . | . | . | . | . |
| 21-225_27G6_HC | . | . | . | . | I | . | . | . | . | . | . | . | . | . | E | . | . | N | . | . | . | . | . | . | . | . |
| 21-225_27H2_HC | . | . | . | . | . | . | . | . | . | . | . | . | . | . | . | . | . | . | . | . | . | . | . | . | . | . |
| 21-225_28A11_HC | . | . | . | . | L | . | . | . | . | . | . | . | . | N | . | T | . | . | . | . | . | . | . | . | . | . |
| 21-225_52H1_HC | . | . | . | . | . | . | . | . | . | . | . | . | . | . | . | . | . | . | . | . | . | . | . | . | . | . |
| 21-225_74B3_HC | . | . | . | . | . | F | . | . | . | . | . | . | . | . | . | . | D | . | . | . | . | . | . | . | . | . |
| 21-225_74C9_HC | . | . | . | . | . | . | . | . | . | . | . | . | . | N | . | . | S | . | . | . | . | . | . | . | . | . |
| 21-225_78E9_HC | . | . | . | . | . | . | . | . | . | . | . | . | . | N | . | . | S | . | . | . | . | . | . | . | . | . |
| 21-225_79E7_HC | . | . | . | . | . | . | . | . | . | . | . | . | . | N | . | . | S | . | . | . | . | . | . | . | . | . |
| 21-225_8C12_HC | . | . | . | . | L | . | . | . | . | . | . | . | . | . | . | . | . | . | . | . | . | . | . | . | . | . |
| 21-225_92A4_HC | . | . | . | . | . | . | . | . | . | . | . | . | . | N | . | . | S | . | . | . | . | . | . | . | . | . |
| 21-225_97E6_HC | . | . | . | . | . | . | . | . | . | . | . | . | . | N | . | . | S | . | . | . | . | . | . | . | . | . |

## FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_146A10_HC | . | . | . | . | . | . | . | . | . | . | . | . | . | . | . | . | . | . | . | . | . | . | . | . | . | . |
| 21-225_147D9_HC | . | . | . | . | . | . | . | . | . | . | . | . | . | . | . | . | . | . | . | . | . | . | . | . | . | . |
| 21-225_148E10_HC | . | . | . | . | . | . | . | . | . | . | . | . | . | . | . | . | . | . | . | . | . | . | . | . | . | . |
| 21-225_149F2_HC | . | . | . | . | V | . | . | R | . | . | . | . | . | . | . | . | . | . | . | . | . | . | . | . | . | . |
| 21-225_150F11_HC | . | . | . | . | V | . | . | R | . | . | . | . | . | . | . | . | . | . | . | . | . | . | . | . | . | . |
| 21-225_151H6_HC | . | . | . | . | . | . | . | . | . | . | . | . | . | . | . | . | . | . | . | . | . | . | . | . | . | . |
| 21-225_152B12_HC | . | . | . | . | . | . | . | . | . | . | . | . | . | . | . | . | . | . | . | . | . | . | . | . | . | . |
| 21-225_152G5_HC | . | . | . | . | . | . | . | . | . | . | . | . | . | . | . | . | . | . | . | . | . | . | . | . | . | . |
| 21-225_153A8_HC | . | . | . | . | . | . | . | . | . | . | . | . | . | . | . | . | . | . | . | . | . | . | . | . | . | . |
| 21-225_153E8_HC | . | . | . | . | . | . | . | . | . | . | . | . | . | . | . | . | . | . | . | . | . | . | . | . | . | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | . | T | . | . | . | . | . | P | . | . | . | . | . | . | Y | . | . | . | . | . | . | . |
| 21-225_170F1_HC | . | . | . | . | . | . | . | . | . | . | . | . | . | . | . | . | . | . | . | . | . | . | . | . | . | . |
| 21-225_195D9_HC | . | . | . | . | . | . | . | . | . | . | . | . | . | . | . | . | . | . | . | . | . | . | . | . | . | . |
| 21-225_196A10_HC | . | . | . | . | . | . | . | . | . | . | . | . | . | . | . | . | . | . | . | . | . | . | . | . | . | . |
| 21-225_225A8_HC | . | . | . | . | . | I | . | Q | . | . | . | . | . | . | . | . | . | . | . | . | . | . | . | . | . | . |
| 21-225_27G6_HC | . | . | . | . | . | . | . | . | . | . | . | . | . | . | . | . | . | . | . | . | . | . | . | . | . | . |
| 21-225_27H2_HC | . | . | . | . | . | . | . | . | . | . | . | P | . | . | . | . | . | . | . | . | . | . | . | . | . | . |
| 21-225_28A11_HC | . | . | . | . | . | . | . | . | . | . | . | . | . | . | . | . | . | . | . | . | . | . | . | . | . | . |
| 21-225_52H1_HC | . | . | . | . | . | . | . | . | . | . | . | . | . | . | . | . | . | . | . | . | . | . | . | . | . | . |
| 21-225_74B3_HC | . | . | . | . | . | . | . | . | . | . | . | . | . | . | . | . | . | . | . | . | . | . | . | . | . | . |
| 21-225_74C9_HC | . | . | . | . | I | . | . | . | . | . | . | . | . | . | . | . | . | . | . | . | . | . | . | . | . | . |
| 21-225_78E9_HC | . | . | . | . | I | . | . | . | . | . | . | . | . | . | . | . | . | . | . | . | . | . | . | . | . | . |
| 21-225_79E7_HC | . | . | . | . | I | . | . | . | . | . | . | . | . | . | . | . | . | . | . | . | . | . | . | . | . | . |
| 21-225_8C12_HC | . | . | . | . | I | . | . | . | . | . | . | . | . | . | . | . | . | . | . | . | . | . | . | . | . | . |
| 21-225_92A4_HC | . | Y | . | . | I | . | . | . | . | . | . | . | . | . | . | . | . | . | . | . | . | . | . | . | . | . |
| 21-225_97E6_HC | . | . | . | . | I | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | V | Y | C | S | S | T | S | C | - | - | - | - | - | - | - | - | - | - | P | Y |
| 21-225_146A10_HC | | | | | | | | F | | | | | | | L | | | | | | | | | | S | |
| 21-225_147D9_HC | | | | | | | | E | G | | G | | P | - | | | | | | | | | | | - | - |
| 21-225_148E10_HC | | | | | | | | E | G | | G | | P | - | | | | | | | | | | | - | - |
| 21-225_149F2_HC | | | | | | | | E | G | | G | | P | - | | | | | | | | | | | - | - |
| 21-225_150F11_HC | | | | | | | | E | G | | G | | P | - | | | | | | | | | | | - | - |
| 21-225_151H6_HC | | | | | | D | R | S | | | R | | | | | | | | | | | | | | | |
| 21-225_152B12_HC | | | | | | D | R | S | | | R | | | | | | | | | | | | | | | |
| 21-225_152G5_HC | | | | | | D | R | S | | | R | | | | | | | | | | | | | | | |
| 21-225_153A8_HC | | | | | | D | R | S | | | R | | | | | | | | | | | | | | | |
| 21-225_153E8_HC | | | | | | | | E | G | | G | | P | - | | | | | | | | | | | - | - |
| 21-225_158D10_HC | | | | | | | | S | | | | | | | | | | | | | | | | | | |
| 21-225_159H8_HC | | | | | | | | S | | | | | | | | | | | | | | | | | | |
| 21-225_160B12_HC | | | | | | | | S | | | | | | | | | | | | | | | | | | |
| 21-225_166G11_HC | | | | | | D | R | | | | | P | T | | H | | | | | | | | | | | |
| 21-225_170F1_HC | | | | | | | | | | | | | | | H | | | | | | | | | | | |
| 21-225_195D9_HC | | | | | | W | F | G | E | G | N | - | - | - | | | | | | | | | | | - | - |
| 21-225_196A10_HC | | | | | | W | F | G | E | G | N | - | - | - | | | | | | | | | | | - | - |
| 21-225_225A9_HC | | | | | | N | H | | | | | | | | S | | | | | | | | | | | |
| 21-225_27G6_HC | | | | | | | | | | | | | | | S | | | | | | | | | | | |
| 21-225_27H2_HC | | | | | | | | | | | | | | | S | | | | | | | | | | | |
| 21-225_28A11_HC | | | | | | | | | | | | | | | S | | | | | | | | | | | |
| 21-225_52H1_HC | | | | | | | | | | | | S | | | S | | | | | | | | | | Y | |
| 21-225_74B3_HC | | | | | | D | | | | | | | | | | | | | | | | | | | | |
| 21-225_74C9_HC | | | | | | D | | | | | | | | | | | | | | | | | | | | |
| 21-225_78E9_HC | | | | | | D | | | | | | | | | | | | | | | | | | | | |
| 21-225_79E7_HC | | | | | | D | | | | | | | | | | | | | | | | | | | | |
| 21-225_8C12_HC | | | | | | | | S | | | | S | | | Y | | | | | | | | | | | |
| 21-225_92A4_HC | | | | | | D | | | | | | | | | | | | | | | | | | | | |
| 21-225_97E6_HC | | | | | | D | | | | | | | | | | | | | | | | | | | | |

4000

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | Y | Y | Y | Y | G | M | D | V | W | G | Q | G | T | T | V | T | V | S | S |
| 21-225_146A10_HC | . | . | . | . | . | . | . | . | . | . | . | . | . | . | . | . | . | . | . |
| 21-225_147D9_HC | . | . | . | . | . | . | . | . | . | . | . | . | . | . | . | . | . | . | . |
| 21-225_148E10_HC | . | . | . | . | . | . | . | . | . | . | . | . | . | . | . | . | . | . | . |
| 21-225_149F2_HC | . | . | . | . | . | . | . | . | . | . | . | . | . | . | . | . | . | . | . |
| 21-225_150F11_HC | . | . | . | . | . | . | . | . | . | . | . | . | . | . | . | . | . | . | . |
| 21-225_151H6_HC | . | . | . | . | . | L | . | . | . | . | . | . | . | . | . | } | . | . | . |
| 21-225_152B12_HC | . | . | . | . | . | L | . | . | . | . | . | . | . | . | . | . | . | . | . |
| 21-225_152G5_HC | . | . | . | . | . | L | . | . | . | . | . | . | . | . | . | } | . | . | . |
| 21-225_153A8_HC | . | . | . | . | . | L | . | . | . | . | . | . | . | . | . | } | . | . | . |
| 21-225_153E8_HC | . | . | . | . | . | . | . | . | . | . | . | . | . | . | . | . | . | . | . |
| 21-225_158D10_HC | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . |
| 21-225_166G11_HC | . | . | . | . | . | L | . | . | . | . | . | . | . | . | . | . | . | . | . |
| 21-225_170F1_HC | . | . | . | . | A | . | . | . | . | . | . | . | . | . | . | . | . | . | . |
| 21-225_195D9_HC | - | - | . | . | . | . | . | . | . | . | . | . | . | . | . | . | . | . | . |
| 21-225_196A10_HC | - | - | . | . | . | . | . | . | . | . | . | . | . | . | . | . | . | . | . |
| 21-225_225A9_HC | . | . | . | N | . | . | . | . | . | . | . | . | . | . | . | . | . | . | . |
| 21-225_27G6_HC | . | . | . | . | . | . | . | . | . | . | . | . | . | . | . | . | . | . | . |
| 21-225_27H2_HC | . | . | . | . | . | . | . | . | . | . | . | . | . | . | . | . | . | . | . |
| 21-225_28A11_HC | . | . | . | . | . | . | . | . | . | . | . | . | . | . | . | . | . | . | . |
| 21-225_52H1_HC | . | . | . | . | . | . | . | . | . | . | . | . | . | . | . | . | . | . | . |
| 21-225_74B3_HC | . | . | . | . | . | . | . | . | . | . | . | . | . | . | . | . | . | . | . |
| 21-225_74C9_HC | . | . | . | . | . | . | . | . | . | . | . | . | . | . | . | . | . | . | . |
| 21-225_78E9_HC | . | . | . | . | . | . | . | . | . | . | . | . | . | . | . | . | . | . | . |
| 21-225_79E7_HC | . | . | . | . | . | . | . | . | . | . | . | . | . | . | . | . | . | . | . |
| 21-225_8C12_HC | . | . | . | . | . | . | . | . | . | . | . | . | . | . | . | . | . | . | . |
| 21-225_92A4_HC | . | . | . | . | . | . | . | . | . | . | . | . | . | . | . | . | . | . | . |
| 21-225_97E6_HC | . | . | . | . | . | . | . | . | . | . | . | . | . | . | . | . | . | . | . |

**FIGURE 57**

Table 62.    <u>Consensus 28- VH3|3-21/D1|1-1|RF2/JH4 (SEQ ID NO: 50279)</u>:

EVQLVES-GGGLVKPGGSLRLSCAASG-FTFS<u>S</u>-----<u>YSMN</u>WVRQAPGKGLEWVS<u>SISGS</u>---SSYIYYADSVKGRFTISRDNAKNSLYLQMNSLRAEDTAVYYCAR<u>VAS</u>----------------------<u>FDY</u>WGQGTLVTVSS wherein:

S at position 33 can be substituted with N

S at position 40 can be substituted with T, K, N or R

M at position 41 can be substituted with L

I at position 58 can be substituted with T

G at position 60 can be substituted with S

S at position 61 can be substituted with G, N or T

S at position 65 can be substituted with G, D or N

S at position 66 can be substituted with T

Y at position 67 can be substituted with F, D, L, N or S

I at position 68 can be substituted with M or T

Y at position 69 can be substituted with N

A at position 71 can be substituted with T

A at position 110 can be substituted with N or S

S at position 111 can be substituted with A, G, T, L, H or N

F at position 136 can be substituted with L or N

Y at position 138 can be substituted with C or S

Xaa Tyr Xaa Xaa Asn (SEQ ID NO: 50040)

**FIGURE 57**

Ser Xaa Ser Xaa Xaa Xaa Xaa Xaa Xaa Xaa Tyr Xaa Asp Ser Val Lys Gly (SEQ ID NO: 50041)

Val Xaa Xaa Xaa Asp Xaa (SEQ ID NO: 50042)

Ser Tyr Ser Met Asn (SEQ ID NO: 50507)

Ser Ile Ser Gly Ser Ser Ser Tyr Ile Tyr Tyr Ala Asp Ser Val Lys Gly (SEQ ID NO: 50508)

Val Ala Ser Phe Asp Tyr (SEQ ID NO: 50509)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | V | E | S | - | G | G | G | L | V | K | P | G | G | S | L | R | L | S | C | A | A | S |
| 21-225_14D6_HC | . | . | . | . | . | . | . | . | . | . | . | . | . | . | . | . | . | . | . | . | . | . | . | . | . | . |
| 21-225_157E4_HC | . | . | . | . | . | . | . | . | . | . | . | . | . | . | . | . | . | . | . | . | . | . | . | . | . | . |
| 21-225_157H10_HC | . | . | . | . | . | . | . | . | . | . | . | . | . | . | . | . | . | . | . | . | . | . | . | . | . | . |
| 21-225_15A2_HC | . | . | . | . | . | . | . | . | . | . | . | . | . | . | . | . | . | . | . | . | . | . | . | . | . | . |
| 21-225_16A11_HC | . | . | . | . | . | . | . | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | . | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . | . | . | . | . | . | . | . |
| 21-225_1F1_HC | . | . | . | . | . | . | . | . | . | . | . | . | . | . | . | . | . | . | . | . | . | . | . | . | . | . |
| 21-225_20H7_HC | . | . | . | . | . | . | . | . | . | . | . | . | . | . | . | . | . | . | . | . | . | . | . | . | . | . |
| 21-225_22B12_HC | . | . | . | . | . | . | . | . | . | . | . | . | . | . | . | . | . | . | . | . | . | . | . | . | . | . |
| 21-225_23A11_HC | . | . | . | . | . | . | . | . | . | . | . | . | . | . | . | . | . | . | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | . | . | . | . | . | . | . | . | . | . | . | . | . | . | . |
| 21-225_2A4_HC | . | . | . | . | . | . | . | . | . | . | . | . | . | . | . | . | . | . | . | . | . | . | . | . | . | . |
| 21-225_2C12_HC | . | . | . | . | . | . | . | . | . | . | . | . | . | . | . | . | . | . | . | . | . | . | . | . | . | . |
| 21-225_32B3_HC | . | . | . | . | . | . | . | . | . | . | . | . | . | . | . | . | . | . | . | . | . | . | . | . | . | . |
| 21-225_35H8_HC | . | . | . | . | . | . | . | . | . | . | . | . | . | . | . | . | . | . | . | . | . | . | . | . | . | . |
| 21-225_50G10_HC | . | . | . | . | . | . | . | . | . | . | . | . | . | . | . | . | . | . | . | . | . | . | . | . | . | . |
| 21-225_52E3_HC | . | . | . | . | . | . | . | . | . | . | . | . | . | . | . | . | . | . | . | . | . | . | . | . | . | . |
| 21-225_52H12_HC | . | . | . | . | . | . | . | . | . | . | . | . | . | . | . | . | . | . | . | . | . | . | . | . | . | . |
| 21-225_56A5_HC | . | . | . | . | . | . | . | . | . | . | . | . | . | . | . | . | . | . | . | . | . | . | . | . | . | . |
| 21-225_56H1_HC | . | . | . | . | . | . | . | . | . | . | . | . | . | . | . | . | . | . | . | . | . | . | . | . | . | . |
| 21-225_57B2_HC | . | . | . | . | . | . | . | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | . |
| 21-225_5F4_HC | . | . | . | . | . | . | . | . | . | . | . | . | . | . | . | . | . | . | . | . | . | . | . | V | . | . |
| 21-225_70E12_HC | E | . | . | . | . | . | . | . | . | . | . | . | . | . | . | . | . | . | . | . | . | . | . | . | . | . |

4004

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | | H_FR2 | | |
| CONSENSUS | G | - | F | T | F | S | S | - | - | - | - | - | Y | S | M | N | W | V | R | Q | A | P | G | K | G | L |
| 21-225_14D6_HC | . | . | . | . | . | . | . | . | . | . | . | . | . | T | . | . | . | . | . | . | . | . | . | . | . | . |
| 21-225_157E4_HC | . | . | . | . | . | . | . | . | . | . | . | . | . | T | . | . | . | . | . | . | . | . | . | . | . | . |
| 21-225_157H10_HC | . | . | . | . | . | . | . | . | . | . | . | . | . | T | . | . | . | . | . | . | . | . | . | . | . | . |
| 21-225_15A2_HC | . | . | . | . | . | . | . | . | . | . | . | . | . | T | . | . | . | . | . | . | . | . | . | . | . | . |
| 21-225_16A11_HC | . | . | . | . | . | . | . | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | . | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . | . | . | . | . | ※ | . | . |
| 21-225_1F1_HC | . | . | . | . | . | . | . | . | . | . | . | . | . | N | . | . | . | . | . | . | . | . | . | . | . | . |
| 21-225_20H7_HC | . | . | . | . | . | . | . | . | . | . | . | . | . | . | . | . | . | . | . | . | . | . | . | . | . | . |
| 21-225_22B12_HC | . | . | . | . | . | . | . | . | . | . | . | . | . | . | . | . | . | . | . | . | . | . | . | . | . | . |
| 21-225_23A11_HC | . | . | . | . | . | . | . | . | . | . | . | . | . | . | . | . | . | ! | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | . | . | . | L | . | . | . | . | . | . | . | . | . | . | . |
| 21-225_2A4_HC | . | . | . | . | . | . | . | . | . | . | . | . | . | . | . | . | . | . | . | . | . | . | . | . | . | . |
| 21-225_2C12_HC | . | . | . | . | . | . | . | . | . | . | . | . | . | . | . | . | . | . | . | . | . | . | . | . | . | . |
| 21-225_32B3_HC | . | . | . | . | . | . | . | . | . | . | . | . | . | . | . | . | . | . | . | . | . | . | . | . | . | . |
| 21-225_35H8_HC | . | . | . | . | . | . | . | . | . | . | . | . | . | . | . | . | . | . | . | . | . | . | . | . | . | . |
| 21-225_50G10_HC | . | . | . | . | . | . | . | . | . | . | . | . | . | . | . | . | . | . | . | . | . | . | . | . | . | . |
| 21-225_52E3_HC | . | . | . | . | . | . | . | . | . | . | . | . | . | ※ | . | . | . | . | . | . | . | . | . | . | . | . |
| 21-225_52H12_HC | . | . | . | . | . | . | . | . | . | . | . | . | . | . | . | . | . | . | . | . | . | . | . | . | . | . |
| 21-225_56A5_HC | . | . | . | . | . | . | . | . | . | . | . | . | . | . | . | . | . | . | . | . | . | . | . | . | . | . |
| 21-225_56H1_HC | . | . | . | . | . | N | . | . | . | . | . | . | . | ※ | . | . | . | . | . | . | ※ | . | . | . | . | . |
| 21-225_57B2_HC | . | . | . | . | . | . | . | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | . |
| 21-225_5F4_HC | . | . | . | . | . | . | . | . | . | . | . | . | . | . | . | . | . | . | . | . | . | . | . | . | . | . |
| 21-225_70E12_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | S | I | S | G | S | - | - | - | S | S | Y | I | Y | Y | A | D | S | V | K | G | R | F |
| 21-225_14D6_HC | | | | | | | | | | | | | Q | | | | | | | | | | | | | |
| 21-225_157E4_HC | | | | | | | | | | | | | | | | | | | | | | | | | | |
| 21-225_157H10_HC | | | | | | | | | | | | | | | | N | | | | | | | | | | |
| 21-225_15A2_HC | | | | | | | | | | | | | Q | | | | | | | | | | | | | |
| 21-225_16A11_HC | | | | | | | | | | | | | | | L | | | | | | | | | | | |
| 21-225_16B8_HC | | | I | | | | | | | | | | | T | | | | | | | | | | | | |
| 21-225_16H1_HC | | | | | | | | | | | | | | T | | | | | | | | | | | | |
| 21-225_17H5_HC | | | | | | | | S | | | | | | T | | | | | | | | | | | | |
| 21-225_17H6_HC | | | | | | | | | | | | | | | | | | | | | | | | | | |
| 21-225_1F1_HC | | | | | | | | S | | | | | | | | | | | | | | | | | | |
| 21-225_20H7_HC | | | | | | | | | | | | | | | F | | | | | | | | | | | |
| 21-225_22B12_HC | | | | | | | | | | | | | | | | T | | | | | | | | | | |
| 21-225_23A11_HC | | | | | | | | | | | | | | T | | | | | | | | | | | | I |
| 21-225_27C5_HC | | | | | T | | | S | | | | | D | | | | | | | | | | | | | |
| 21-225_27H12_HC | | | | | | | | | | | | | | | N | | | | T | | | | | | | |
| 21-225_2A4_HC | | | | | | | | | | | | | | T | | | | | | | | | | | | I |
| 21-225_2C12_HC | | | | | | | | | | | | | | | | M | | | | | | | | | | |
| 21-225_32B3_HC | | | | | | | | | | | | | | T | | | | | | | | | | | | |
| 21-225_35H8_HC | | | | | | | | T | | | | | Q | | F | | | | | | | | | | | |
| 21-225_50G10_HC | | | | | | | | | | | | | | | | | | | | | | | | | | |
| 21-225_52E3_HC | | | | | | | S | Q | | | | | N | | S | | | | | | | | | | | |
| 21-225_52H12_HC | | | | | | | | | | | | | | T | | | | | | | | | | | | |
| 21-225_56A5_HC | | | | | | | | | | | | | | | | | | | | | | | | | | |
| 21-225_56H1_HC | | | I | | | | | | | | | | | T | Q | | | | | | | | | | | |
| 21-225_57B2_HC | | | | | | | | | | | | | | | | | N | | T | | | | | | | |
| 21-225_5E11_HC | | | | | | | | | | | | | | T | | | | | | | | | | | | |
| 21-225_5F4_HC | | | | | | | | | | | | | | | | | | | | | | | | | | |
| 21-225_70E12_HC | E | | | | | | | | N | | | | | T | | | | | T | | | | | | | |

4006

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | A | K | N | S | L | Y | L | Q | M | N | S | L | R | A | E | D | T | A | V | Y |
| 21-225_14D6_HC | . | M | . | | | | | | | | | | | | | | | | | | | | | | | |
| 21-225_157E4_HC | . | | | | | | | | | | | | | | | | | K | | | | | | | | |
| 21-225_157H10_HC | . | | | | | | | | | | | | | | | | | | | | | | | | | |
| 21-225_15A2_HC | . | | | | | | | | | | | | | | | | A | | | | | | | | | |
| 21-225_16A11_HC | . | | | | | | | | | | | | | | | | | | | | | | | | | |
| 21-225_16B8_HC | . | | | | | | | | | | | | | | | | | | | | | | | | | |
| 21-225_16H1_HC | . | | | | | | | | | | | | | | | | | | | | | | | | | |
| 21-225_17H5_HC | . | | | | | | | | | | | | | | | | | | | | | | | | | |
| 21-225_17H6_HC | . | | F | . | | | | | | | | | | | | | | | | | | | | | | |
| 21-225_1F1_HC | . | | | | | | | | | | | | | | | | | | | | | | | | | |
| 21-225_20H7_HC | . | | | | | | | | | | | | | | | | | | | | | | | | | |
| 21-225_22B12_HC | . | | | | | | | | | | | | | | | | | | | | | | | | | |
| 21-225_23A11_HC | A | | | | | | | | | | | | | | | | | | | | | | | | | |
| 21-225_27C5_HC | . | | | | | | | | | | | | | | | | | | | | | | | | | |
| 21-225_27H12_HC | . | | | | | | | | | | | F | | | | | | | | | | | | | | |
| 21-225_2A4_HC | A | | | | | | | | | | | | | | | | | | | | | | | | | |
| 21-225_2C12_HC | . | | | | | | | | D | | | | | | | | | | | | | | | | | |
| 21-225_32B3_HC | . | | | | | | | | | | | | | | | | | | | | | | | | | |
| 21-225_35H8_HC | . | | | | | | | | K | | | V | | | | | | | | | | | | | | |
| 21-225_50G10_HC | . | | | | | | | | | | | | | | | | | | | | | | | | | |
| 21-225_52E3_HC | . | | | | | | | E | | | | | | | | | | | | | | | | | | |
| 21-225_52H12_HC | . | V | | | | | | | | | | | | | V | | | | | | | | | | | |
| 21-225_56A5_HC | . | | | | | | | | | | | | | | | | | | | | | | | | | |
| 21-225_56H1_HC | . | | | | | | | | | | | | | | | | | | | | | | | | | |
| 21-225_57B2_HC | . | | | | | | | | | | | | | | | | | | | | | | | | | |
| 21-225_5E11_HC | . | | | | | | | | | | | | | | | | | | | | | | | | | F |
| 21-225_5F4_HC | . | | | | | | | | | | | F | | | | | | | | | | | | | | |
| 21-225_70E12_HC | . | | | | | | | | | | | | | | | D | | | T | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | V | A | S | - | - | - | - | - | - | - | - | - | - | - | - | - | - | - | - | - | - | - |
| 21-225_14D6_HC | . | . | . | . | . | . | A | . | . | . | . | . | . | . | . | . | . | . | . | . | . | . | . | . | . | . |
| 21-225_157E4_HC | . | . | . | . | . | . | T | . | . | . | . | . | . | . | . | . | . | . | . | . | . | . | . | . | . | . |
| 21-225_157H10_HC | . | . | . | . | . | . | H | . | . | . | . | . | . | . | . | . | . | . | . | . | . | . | . | . | . | . |
| 21-225_15A2_HC | . | . | . | . | . | . | . | . | . | . | . | . | . | . | . | . | . | . | . | . | . | . | . | . | . | . |
| 21-225_16A11_HC | . | . | . | . | . | . | A | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | A | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . | . | . | . | . | . | . | . |
| 21-225_1F1_HC | . | . | . | . | . | N | L | . | . | . | . | . | . | . | . | . | . | . | . | . | . | . | . | . | . | . |
| 21-225_20H7_HC | . | . | . | . | . | . | A | . | . | . | . | . | . | . | . | . | . | . | . | . | . | . | . | . | . | . |
| 21-225_22B12_HC | . | . | . | . | . | . | A | . | . | . | . | . | . | . | . | . | . | . | . | . | . | . | . | . | . | . |
| 21-225_23A11_HC | . | . | . | . | . | . | . | . | . | . | . | . | . | . | . | . | . | . | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | . | . | . | . | . | . | . | . | . | . | . | . | . | . | . |
| 21-225_2A4_HC | . | . | . | . | . | . | . | . | . | . | . | . | . | . | . | . | . | . | . | . | . | . | . | . | . | . |
| 21-225_2C12_HC | . | . | . | . | . | N | L | . | . | . | . | . | . | . | . | . | . | . | . | . | . | . | . | . | . | . |
| 21-225_32B3_HC | . | . | . | . | . | . | G | . | . | . | . | . | . | . | . | . | . | . | . | . | . | . | . | . | . | . |
| 21-225_35H8_HC | . | . | . | . | . | S | G | . | . | . | . | . | . | . | . | . | . | . | . | . | . | . | . | . | . | . |
| 21-225_50G10_HC | . | . | . | . | . | . | T | . | . | . | . | . | . | . | . | . | . | . | . | . | . | . | . | . | . | . |
| 21-225_52E3_HC | . | . | . | . | . | S | . | . | . | . | . | . | . | . | . | . | . | . | . | . | . | . | . | . | . | . |
| 21-225_52H12_HC | . | . | . | . | . | N | . | . | . | . | . | . | . | . | . | . | . | . | . | . | . | . | . | . | . | . |
| 21-225_56A5_HC | . | . | . | . | . | . | T | . | . | . | . | . | . | . | . | . | . | . | . | . | . | . | . | . | . | . |
| 21-225_56H1_HC | . | . | . | . | . | . | . | . | . | . | . | . | . | . | . | . | . | . | . | . | . | . | . | . | . | . |
| 21-225_57B2_HC | . | . | . | . | . | . | G | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | . |
| 21-225_5F4_HC | . | . | . | . | . | N | N | . | . | . | . | . | . | . | . | . | . | . | . | . | . | . | . | . | . | . |
| 21-225_70E12_HC | . | . | . | . | . | . | A | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | - | F | D | Y | W | G | Q | G | T | L | V | T | V | S | S |
| 21-225_14D6_HC | . | . | . | . | . | . | . | S | . | . | . | . | . | . | . | S | . | . | . |
| 21-225_157E4_HC | . | . | . | . | . | . | . | . | . | . | . | . | . | . | . | . | . | . | . |
| 21-225_157H10_HC | . | . | . | . | . | . | . | . | . | . | . | . | . | . | . | . | . | . | . |
| 21-225_15A2_HC | . | . | . | . | . | . | . | . | . | . | . | . | . | . | . | . | . | . | . |
| 21-225_16A11_HC | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . |
| 21-225_16H1_HC | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . |
| 21-225_1F1_HC | . | . | . | . | . | . | . | . | . | . | . | . | . | . | . | . | . | . | . |
| 21-225_20H7_HC | . | . | . | . | L | . | . | . | . | . | . | . | . | . | . | . | . | . | . |
| 21-225_22B12_HC | . | . | . | . | L | . | . | S | . | . | . | . | . | . | . | . | . | . | . |
| 21-225_23A11_HC | . | . | . | . | . | . | . | . | . | . | . | . | . | . | . | . | . | . | . |
| 21-225_27C5_HC | . | . | . | . | . | . | . | O | . | . | . | . | . | . | . | . | . | . | . |
| 21-225_27H12_HC | . | . | . | . | . | . | . | . | . | . | . | . | . | . | . | . | . | . | . |
| 21-225_2A4_HC | . | . | . | . | . | . | . | . | . | . | . | . | . | . | . | . | . | . | . |
| 21-225_2C12_HC | . | . | . | . | . | . | . | . | . | . | . | . | . | . | . | . | . | . | . |
| 21-225_32B3_HC | . | . | . | . | . | . | . | . | . | . | . | . | . | . | . | . | . | . | . |
| 21-225_35H8_HC | . | . | . | . | . | . | . | . | . | . | . | . | . | . | . | . | . | . | . |
| 21-225_50G10_HC | . | . | . | . | . | . | . | . | . | . | . | . | . | . | . | . | . | . | . |
| 21-225_52E3_HC | . | . | . | . | N | . | . | . | . | . | . | . | . | . | . | . | . | . | . |
| 21-225_52H12_HC | . | . | . | . | . | . | . | . | . | . | . | . | . | . | . | . | . | . | . |
| 21-225_56A5_HC | . | . | . | . | . | . | . | . | . | . | . | . | . | . | . | . | . | . | . |
| 21-225_56H1_HC | . | . | . | . | . | . | . | . | . | . | . | . | . | . | . | . | . | . | . |
| 21-225_57B2_HC | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . |
| 21-225_5F4_HC | . | . | . | . | . | . | . | . | . | . | . | . | . | . | . | . | . | . | . |
| 21-225_70E12_HC | . | . | . | . | . | . | O | . | . | . | . | . | . | . | . | . | . | . | . |

**FIGURE 57**

Table 63.      Consensus 29-VH4|4-30.1/D3|3-22|RF2/JH6 (SEQ ID NO: 50280):

QVQLQES-GPGLVKPSQTLSLTCTVSG-GSIS<u>SG---DYYWN</u>WIRQHPGKGLEWIG<u>YIFY----
SGSTYYNPSLKS</u>RVTISVDTSKNQFSLKLSSVTAADTAVYYCAR<u>GDYDGSGSY------------HYYYGMDV</u>WGQGTTVTVSS wherein:

G at position 34 can be substituted with V

D at position 38 can be substituted with V, G or S

Y at position 40 can be substituted with H

N at position 42 can be substituted with S

Y at position 57 can be substituted with N or F

I at position 58 can be substituted with L

F at position 59 can be substituted with Y or H

Y at position 60 can be substituted with H

Y at position 70 can be substituted with N

K at position 75 can be substituted with R

Y at position 132 can be substituted with F or H

Y at position 134 can be substituted with H

M at position 136 can be substituted with L

Ser Xaa Xaa Tyr Xaa Trp Xaa (SEQ ID NO: 50230)

Xaa Xaa Xaa Xaa Ser Gly Ser Thr Tyr Xaa Asn Pro Ser Leu Xaa Ser (SEQ ID NO: 50231)

Gly Asp Tyr Asp Gly Ser Gly Ser Tyr His Xaa Tyr Xaa Gly Xaa Asp Val (SEQ ID NO: 50232)

Ser Gly Asp Tyr Tyr Trp Asn (SEQ ID NO: 50510)

**FIGURE 57**

Tyr Ile Phe Tyr Ser Gly Ser Thr Tyr Tyr Asn Pro Ser Leu Lys Ser (SEQ ID NO: 50511)

Gly Asp Tyr Asp Gly Ser Gly Ser Tyr His Tyr Tyr Tyr Gly Met Asp Val (SEQ ID NO: 50512)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | | | | | | | | | | | | | | | | H_FR1 | | | | | | | | | | | |
| CONSENSUS | Q | V | Q | L | Q | E | S | - | G | P | G | L | V | K | P | S | Q | T | L | S | L | T | C | T | V | S | G |
| 21-225_190A3_HC | . | | . | | . | | . | | . | | . | | . | | . | | . | | . | | . | | . | | . | | . |
| 21-225_190A7_HC | . | | . | | . | | . | | . | | . | | . | N | . | | . | | . | | . | | . | | . | | . |
| 21-225_190B4_HC | . | | . | | . | | . | | . | | . | | . | | . | | . | | . | | . | | . | | . | | . |
| 21-225_191A3_HC | . | | . | | . | | . | | . | | . | | . | | . | | . | | . | | . | | . | | . | | . |
| 21-225_191D3_HC | . | | . | | N | | . | | . | | . | | . | | . | | . | | . | | . | | . | | . | | . |
| 21-225_191F1_HC | . | | . | | . | | . | | . | | . | | . | | . | | . | | . | | . | | . | | . | | . |
| 21-225_191H7_HC | . | | . | | . | | . | | . | | . | | . | | . | | . | | . | | . | | . | | . | | . |
| 21-225_192B3_HC | . | | . | | . | | . | | . | | . | | . | | . | | . | | . | | . | | . | | . | | . |
| 21-225_192G12_HC | . | | . | | . | | . | | . | | . | | . | | . | | . | | . | | . | | . | | . | | . |
| 21-225_192H3_HC | . | | . | | . | | . | | . | | . | | . | | . | | . | | . | | . | | . | | . | | . |
| 21-225_193A1_HC | . | | . | | . | | . | | . | | . | | . | | . | | . | | . | | . | | . | | . | | . |
| 21-225_193B1_HC | . | | . | | . | | . | | . | | . | | . | | . | | . | | . | | . | | . | | . | | . |
| 21-225_193C8_HC | . | | . | | . | | . | | . | | . | | . | | . | | . | | . | | . | | . | | . | | . |
| 21-225_193D8_HC | . | | . | | . | | . | | . | | . | | . | | . | | . | | . | | . | | . | | . | | . |
| 21-225_193F3_HC | . | | . | | . | | . | | . | | . | | . | | . | | . | | . | | . | | . | | . | | . |
| 21-225_193G8_HC | . | | . | | . | | N | | . | | . | | . | | . | | . | | . | | . | | . | | . | | . |
| 21-225_193H6_HC | . | | . | | . | | . | | . | | . | | . | | . | | . | | . | | . | | . | | . | | . |
| 21-225_194E5_HC | . | | . | | . | | . | | . | | . | | . | | . | | . | | . | | . | | . | | . | | . |
| 21-225_194E6_HC | . | | . | | . | | . | | . | | . | | . | | . | | . | | . | | . | | . | | . | | . |
| 21-225_196H12_HC | . | | . | | . | | . | | . | | . | | . | N | . | | . | | . | | . | | . | | . | | . |
| 21-225_197F4_HC | . | | . | | . | | . | | . | | . | | . | | . | | . | | . | | . | | . | | . | | . |
| 21-225_198B1_HC | . | | . | | N | | . | | . | | . | | . | | . | | . | | . | | . | | . | | . | | . |
| 21-225_198E1_HC | . | | . | | . | | . | | . | | . | | . | | . | | . | | . | | . | | . | | . | | . |
| 21-225_199C2_HC | . | | . | | . | | . | | . | | . | | . | | . | | . | | . | | . | | . | | . | | . |
| 21-225_199C7_HC | . | | . | | . | | . | | . | | . | | . | | . | | . | | . | | . | | . | | . | | . |
| 21-225_200F10_HC | . | | . | | . | | . | | . | | . | | . | | . | | . | | . | | . | | . | | . | | . |

**FIGURE 57**

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | | | | | | H_CDR1 | | | | | | | | | | | | H_FR2 | | | | | |
| CONSENSUS | - | G | S | I | S | S | G | - | - | - | D | Y | Y | W | N | W | I | R | Q | H | P | G | K | G | L | E | W |
| 21-225_190A3_HC | . | . | . | . | N | . | . | . | . | . | G | . | . | . | . | . | . | . | . | . | . | . | . | . | . | . | . |
| 21-225_190A7_HC | . | . | . | . | . | . | . | . | . | . | V | . | . | . | S | . | . | . | . | . | . | . | . | . | . | . | . |
| 21-225_190B4_HC | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . |
| 21-225_191D3_HC | . | . | . | . | N | . | . | . | . | . | . | . | . | . | . | . | . | . | . | . | . | . | . | . | . | D | . |
| 21-225_191F1_HC | . | . | . | . | N | . | . | . | . | . | G | . | . | . | . | . | . | . | . | . | . | . | . | . | . | . | . |
| 21-225_191H7_HC | . | . | . | . | N | . | . | . | . | . | . | . | . | . | . | . | . | . | . | . | . | . | . | . | . | . | . |
| 21-225_192B3_HC | . | . | . | . | . | . | . | . | . | . | . | . | . | . | . | . | . | . | . | . | . | . | . | . | . | . | . |
| 21-225_192G12_HC | . | . | . | . | . | . | . | . | . | . | V | . | N | . | S | . | . | . | . | . | . | . | . | . | . | . | . |
| 21-225_192H3_HC | . | . | . | . | . | . | V | . | . | . | S | . | . | . | S | . | . | . | . | R | . | . | . | . | . | . | . |
| 21-225_193A1_HC | . | . | . | . | . | . | . | . | . | . | V | . | . | . | S | . | . | . | . | . | . | . | . | . | . | . | . |
| 21-225_193B1_HC | . | . | . | . | . | . | . | . | . | . | V | . | . | . | S | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . |
| 21-225_193D8_HC | . | . | . | . | N | . | . | . | . | . | G | . | . | . | . | . | . | . | . | . | . | . | . | . | . | . | . |
| 21-225_193F3_HC | . | . | . | . | N | . | . | . | . | . | . | . | . | . | . | . | . | . | . | . | . | . | . | . | . | . | . |
| 21-225_193G8_HC | . | . | . | V | . | . | . | . | . | . | V | . | . | . | S | . | . | . | . | . | . | . | . | . | . | . | . |
| 21-225_193H6_HC | . | . | . | . | N | . | . | . | . | . | . | . | . | . | . | . | . | . | . | . | . | . | . | . | . | . | . |
| 21-225_194E5_HC | . | . | . | . | N | . | . | . | . | . | . | . | . | . | . | . | . | N | . | . | . | . | . | . | . | . | . |
| 21-225_194E6_HC | . | D | . | . | N | . | . | . | . | . | . | . | . | . | . | . | . | . | . | . | . | . | . | . | . | . | . |
| 21-225_196H12_HC | . | . | . | . | . | . | . | . | . | . | V | . | . | . | S | . | . | . | . | . | . | . | . | . | . | . | . |
| 21-225_197F4_HC | . | . | . | . | . | . | . | . | . | . | . | . | . | . | . | . | . | . | . | . | . | . | . | . | . | . | . |
| 21-225_198B1_HC | . | . | . | . | N | . | . | . | . | . | . | . | . | . | . | P | . | . | . | . | . | . | . | . | . | . | . |
| 21-225_198E1_HC | . | . | . | . | N | . | . | . | . | . | G | . | . | . | . | . | . | . | . | . | . | . | . | . | . | . | . |
| 21-225_199C2_HC | . | . | . | . | . | . | . | . | . | . | . | . | . | . | . | L | . | . | . | . | . | E | . | . | . | . | . |
| 21-225_199C7_HC | . | . | . | . | N | . | . | . | . | . | G | . | . | . | . | . | . | . | . | . | . | . | . | . | . | . | . |
| 21-225_200F10_HC | . | . | . | . | . | . | . | . | . | . | . | . | . | . | . | L | . | . | . | . | . | E | . | . | . | . | . |

**FIGURE 57**

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 | 82 | 83 |
|---|---|---|---|---|---|---|---|---|---|---|---|---|---|---|---|---|---|---|---|---|---|---|---|---|---|---|---|---|---|
| | | | | | | | | | | | | H_CDR2 | | | | | | | | | | | | | | | | | |
| CONSENSUS | I | G | Y | I | F | Y | - | - | - | - | S | S | T | Y | Y | N | P | S | L | K | S | R | V | T | I | S | V | D | |
| 21-225_190A3_HC | . | . | F | . | . | . | . | . | . | . | . | . | . | . | . | . | . | . | . | . | . | . | . | . | . | . | . | . | |
| 21-225_190A7_HC | . | . | N | . | Y | . | . | . | . | . | . | . | . | . | N | . | . | . | . | . | . | . | I | I | . | . | . | . | |
| 21-225_190B4_HC | . | . | . | . | . | . | . | . | . | . | . | . | . | . | . | . | . | . | . | . | . | . | . | . | . | . | . | . | |
| 21-225_191A3_HC | . | . | . | . | . | . | . | . | . | . | . | . | . | . | . | . | . | . | . | . | . | . | . | . | . | . | . | . | |
| 21-225_191D3_HC | . | . | . | . | . | . | . | . | . | . | . | . | . | . | . | . | . | . | . | . | . | . | . | . | . | . | . | . | |
| 21-225_191F1_HC | . | . | . | . | . | . | . | . | . | . | . | . | . | . | . | . | . | . | . | . | . | . | . | . | . | . | . | . | |
| 21-225_191H7_HC | . | . | . | . | . | . | . | . | . | . | . | . | . | . | . | . | . | . | . | . | . | . | . | . | . | . | . | . | |
| 21-225_192B3_HC | . | . | F | . | . | . | . | . | . | . | . | . | . | . | . | . | . | . | . | . | . | . | . | . | . | . | . | . | |
| 21-225_192G12_HC | . | . | N | . | H | . | . | . | . | . | . | . | . | . | N | . | . | . | . | . | . | . | . | . | . | . | . | . | |
| 21-225_192H3_HC | . | . | N | L | Y | . | . | . | . | . | . | . | . | . | . | . | . | . | H | . | . | A | . | . | . | . | . | . | |
| 21-225_193A1_HC | . | . | N | . | Y | . | . | . | . | . | . | . | . | . | N | . | . | . | . | . | . | L | . | . | . | . | A | . | |
| 21-225_193B1_HC | . | . | N | . | Y | . | . | . | . | . | . | . | . | . | N | . | . | . | . | . | . | . | . | . | . | . | . | . | |
| 21-225_193C8_HC | . | . | . | . | . | . | . | . | . | . | . | . | . | . | . | . | . | . | . | . | . | . | . | . | . | . | . | . | |
| 21-225_193D8_HC | . | . | F | . | . | . | . | . | . | . | . | . | . | . | . | . | . | . | . | . | . | . | . | . | S | . | . | . | |
| 21-225_193F3_HC | . | . | . | . | . | . | . | . | . | . | . | . | . | . | . | . | . | . | . | . | . | . | . | . | . | . | . | . | |
| 21-225_193G8_HC | . | . | N | . | Y | . | . | . | . | . | . | . | . | . | N | . | . | . | . | . | . | . | . | . | . | . | . | . | |
| 21-225_193H6_HC | . | . | . | . | . | . | . | . | . | . | . | . | . | . | . | . | . | . | . | . | . | . | . | . | . | . | . | . | |
| 21-225_194E5_HC | . | . | . | . | . | . | . | . | . | . | . | . | . | . | . | . | . | . | . | . | . | . | . | . | . | . | . | . | |
| 21-225_194E6_HC | . | . | F | . | . | . | . | . | . | . | . | . | . | . | . | . | . | . | . | . | . | . | . | . | . | . | I | . | |
| 21-225_196H12_HC | . | . | N | . | Y | . | . | . | . | . | . | . | . | . | N | . | . | . | . | . | . | I | I | . | . | . | . | . | |
| 21-225_197F4_HC | . | . | . | . | . | . | . | . | . | . | . | . | . | . | . | . | . | . | . | . | . | I | . | . | . | . | . | . | |
| 21-225_198B1_HC | . | . | . | . | . | H | . | . | . | . | . | . | . | . | . | . | . | . | . | . | . | . | . | . | V | . | . | . | |
| 21-225_198E1_HC | . | . | F | . | . | . | . | . | . | . | . | . | . | . | . | . | . | . | . | . | . | L | S | . | . | . | . | . | |
| 21-225_199C2_HC | . | . | . | . | . | . | . | . | . | . | . | . | . | . | . | . | . | . | . | . | . | . | . | . | . | . | . | . | |
| 21-225_199C7_HC | . | . | F | . | . | . | . | . | . | . | . | . | . | . | . | . | . | . | . | . | . | . | . | . | S | . | . | . | |
| 21-225_200F10_HC | . | . | . | . | . | . | . | . | . | . | . | . | . | . | . | . | . | . | . | . | . | I | . | . | . | . | . | . | |

FIGURE 57

| Reference # | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 | 109 | 110 |
|---|---|---|---|---|---|---|---|---|---|---|---|---|---|---|---|---|---|---|---|---|---|---|---|---|---|---|---|
| | | | | | | | | | H_FR3 | | | | | | | | | | | | | | | | | | |
| CONSENSUS | T | S | K | N | Q | F | S | L | K | L | S | S | V | T | A | A | D | T | A | V | Y | Y | C | A | R | G | D |
| 21-225_190A3_HC | . | . | . | . | . | . | . | . | . | . | N | . | . | . | . | . | . | . | . | . | . | . | . | . | . | . | . |
| 21-225_190A7_HC | . | . | . | . | . | . | . | . | . | . | . | . | . | . | . | . | . | . | . | . | . | . | . | . | . | . | . |
| 21-225_190B4_HC | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . |
| 21-225_191D3_HC | . | . | . | . | . | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . |
| 21-225_192B3_HC | . | . | . | . | . | . | . | . | . | . | . | . | . | . | V | . | . | . | . | . | . | . | . | . | . | . | . |
| 21-225_192G12_HC | . | . | . | . | . | . | . | . | . | . | . | . | . | . | . | . | . | . | . | . | . | . | . | . | . | . | . |
| 21-225_192H3_HC | . | . | . | . | . | . | . | . | . | . | . | . | . | . | . | . | . | . | . | . | . | . | . | T | . | . | . |
| 21-225_193A1_HC | . | . | . | . | . | . | . | . | . | . | . | . | . | . | . | . | . | . | . | . | . | . | . | . | . | . | . |
| 21-225_193B1_HC | . | . | . | . | . | . | . | . | . | . | . | . | . | . | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . |
| 21-225_193D8_HC | . | . | . | . | . | . | . | . | . | . | . | . | . | . | . | . | . | . | . | . | . | . | . | . | . | . | . |
| 21-225_193F3_HC | . | . | . | . | . | . | . | . | . | . | . | . | . | . | . | . | . | . | . | . | . | . | . | . | . | . | . |
| 21-225_193G8_HC | . | . | . | . | . | . | . | . | . | . | N | . | . | . | . | . | . | . | . | . | . | . | . | . | . | . | . |
| 21-225_193H6_HC | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . |
| 21-225_194E6_HC | . | . | . | . | . | . | . | . | . | . | . | . | . | N | V | . | . | . | . | . | . | . | . | . | . | . | . |
| 21-225_196H12_HC | . | . | . | . | . | . | . | . | . | . | . | . | . | . | . | . | . | . | . | . | . | . | . | . | . | . | . |
| 21-225_197F4_HC | . | . | . | . | . | . | . | . | . | . | . | . | . | . | . | . | . | . | . | . | . | . | . | . | . | . | . |
| 21-225_198B1_HC | . | . | . | . | . | . | . | . | . | . | . | . | . | . | . | . | . | . | . | . | . | . | . | . | . | . | . |
| 21-225_198E1_HC | . | . | . | . | . | . | . | . | . | . | . | . | . | . | . | . | . | . | . | . | . | . | . | . | . | . | . |
| 21-225_199C2_HC | . | . | M | T | . | . | . | . | . | . | T | . | . | . | . | . | . | . | . | . | . | . | . | . | . | . | . |
| 21-225_199C7_HC | . | . | . | . | . | . | . | . | . | . | . | . | . | . | . | . | . | . | . | . | . | . | . | . | . | . | . |
| 21-225_200F10_HC | . | . | M | T | . | . | . | . | . | . | T | . | . | . | . | . | . | . | . | . | . | . | . | . | . | . | . |

FIGURE 57

| Reference # | 111 | 112 | 113 | 114 | 115 | 116 | 117 | 118 | 119 | 120 | 121 | 122 | 123 | 124 | 125 | 126 | 127 | 128 | 129 | 130 | 133 | 134 | 133 | 136 | 137 | 138 | 139 | 140 |
|---|---|---|---|---|---|---|---|---|---|---|---|---|---|---|---|---|---|---|---|---|---|---|---|---|---|---|---|---|
| | | | | | | | | | | | | | H_CDR3 | | | | | | | | | | | | | | | |
| CONSENSUS | Y | D | G | S | G | S | Y | - | - | - | - | - | - | - | - | - | - | - | - | - | Y | Y | G | M | D | V | W | G |
| 21-225_190A3_HC | . | . | . | . | . | . | . | . | . | . | . | . | . | . | . | . | . | . | . | . | . | . | . | . | . | . | . | . |
| 21-225_190A7_HC | . | . | . | . | . | . | . | . | . | . | . | . | . | . | . | . | . | . | . | . | . | . | . | L | . | . | . | . |
| 21-225_190B4_HC | . | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . | . |
| 21-225_191D3_HC | . | . | . | . | . | . | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . | . |
| 21-225_192B3_HC | . | . | . | . | . | . | . | . | . | . | . | . | . | . | . | . | . | . | . | . | . | . | . | . | . | . | . | . |
| 21-225_192G12_HC | . | . | . | . | . | . | . | . | . | . | . | . | . | . | . | . | . | . | . | . | . | . | . | . | . | . | . | . |
| 21-225_192H3_HC | . | . | . | . | . | . | . | . | . | . | . | . | . | . | . | . | . | . | . | . | . | N | . | . | . | . | . | . |
| 21-225_193A1_HC | . | . | . | . | . | . | . | . | . | . | . | . | . | . | . | . | . | . | . | . | . | . | . | . | . | . | . | . |
| 21-225_193B1_HC | . | . | . | . | . | . | . | . | . | . | . | . | . | . | . | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . | . |
| 21-225_193D8_HC | . | . | . | . | . | . | . | . | . | . | . | . | . | . | . | . | . | . | . | . | . | . | . | . | . | . | . | . |
| 21-225_193F3_HC | . | . | . | . | . | . | . | . | . | . | . | . | . | . | . | . | . | . | . | . | . | . | . | . | . | . | . | . |
| 21-225_193G8_HC | . | . | . | . | . | . | . | . | . | . | . | . | . | . | . | . | . | . | . | . | . | . | . | L | . | . | . | . |
| 21-225_193H6_HC | . | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . | . |
| 21-225_194E6_HC | . | . | . | . | . | . | . | . | . | . | . | . | . | . | . | . | . | . | . | . | . | . | . | . | . | . | . | . |
| 21-225_196H12_HC | . | . | . | . | . | . | . | . | . | . | . | . | . | . | . | . | . | . | . | . | . | . | . | L | . | . | . | . |
| 21-225_197F4_HC | . | . | . | . | . | . | . | . | . | . | . | . | . | . | . | . | . | . | . | . | . | . | . | L | . | . | . | . |
| 21-225_198B1_HC | . | . | . | . | . | . | . | . | . | . | . | . | . | . | . | . | . | . | . | . | . | . | . | . | . | . | . | . |
| 21-225_198E1_HC | . | . | . | . | . | . | . | . | . | . | . | . | . | . | . | . | . | . | . | . | . | . | . | . | . | . | . | . |
| 21-225_199C2_HC | . | . | . | . | . | . | . | . | . | . | . | . | . | . | . | . | . | . | . | . | . | . | . | . | . | . | . | . |
| 21-225_199C7_HC | . | . | . | . | . | . | . | . | . | . | . | . | . | . | . | . | . | . | . | . | . | . | . | . | . | . | . | . |
| 21-225_200F10_HC | . | . | . | . | . | . | . | . | . | . | . | . | . | . | . | . | . | . | . | . | . | . | . | . | . | . | . | . |

**FIGURE 57**

| Reference # | 141 | 142 | 143 | 144 | 145 | 146 | 147 | 148 | 149 |
|---|---|---|---|---|---|---|---|---|---|
| | | | H_FR4 | | | | | | |
| CONSENSUS | Q | G | T | T | V | T | V | S | S |
| 21-225_190A3_HC | . | . | . | . | . | . | . | . | . |
| 21-225_190A7_HC | . | . | . | . | . | . | . | . | . |
| 21-225_190B4_HC | . | . | . | . | . | . | . | . | . |
| 21-225_191A3_HC | . | . | . | . | . | . | . | . | . |
| 21-225_191D3_HC | . | . | . | . | . | . | . | . | . |
| 21-225_191F1_HC | . | . | . | . | . | . | . | . | . |
| 21-225_191H7_HC | . | . | . | . | . | . | . | . | . |
| 21-225_192B3_HC | . | . | . | . | . | . | . | . | . |
| 21-225_192G12_HC | . | . | . | . | . | . | . | . | . |
| 21-225_192H3_HC | . | . | . | . | . | . | . | . | . |
| 21-225_193A1_HC | . | . | . | . | . | . | . | . | . |
| 21-225_193B1_HC | . | . | . | . | . | . | . | . | . |
| 21-225_193C8_HC | . | . | . | . | . | . | . | . | . |
| 21-225_193D8_HC | . | . | . | . | . | . | . | . | . |
| 21-225_193F3_HC | . | . | . | . | . | . | . | . | . |
| 21-225_193G8_HC | ⸭ | . | . | . | . | . | . | . | . |
| 21-225_193H6_HC | . | . | . | . | . | . | . | . | . |
| 21-225_194E5_HC | . | . | . | . | . | . | . | . | . |
| 21-225_194E6_HC | . | . | . | . | . | . | . | . | . |
| 21-225_196H12_HC | . | . | . | . | . | . | . | . | . |
| 21-225_197F4_HC | . | . | . | . | . | . | . | . | . |
| 21-225_198B1_HC | ⸭ | . | . | . | . | . | . | . | . |
| 21-225_198E1_HC | . | . | . | . | . | . | . | . | . |
| 21-225_199C2_HC | . | . | . | . | . | . | . | . | . |
| 21-225_199C7_HC | . | . | . | . | . | . | . | . | . |
| 21-225_200F10_HC | . | . | . | . | . | . | . | . | . |

**FIGURE 57**

Table 64.    Consensus 30- VH3|3-23/D7|7-27|RF1/JH6 (SEQ ID NO: 50281):

EVQLLES-GGGLVQPGGSLRLSCAASG-FTFSS-----YAMSWVRQAPGKGLEWVSVISGG---
GSSTYYADSVKGRFTISRDNSKNTLYLQMNSLRAEDTAVYYCAKWRGNPT-----------------DYGMDVWGQGTTVTVSS wherein:

S at position 33 can be substituted with N or T

Y at position 39 can be substituted with H

A at position 40 can be substituted with P or V

V at position 57 can be substituted with A or I

G at position 61 can be substituted with S

S at position 66 can be substituted with G or T

S at position 67 can be substituted with T

T at position 68 can be substituted with A

W at position 109 can be substituted with A

R at position 110 can be substituted with G

N at position 112 can be substituted with T

P at position 113 can be substituted with T

T at position 114 can be substituted with G

null (-) at position 115 can be substituted with S

null (-) at position 116 can be substituted with Y

null (-) at position 132 can be substituted with Y

D at position 133 can be substituted with Y

**FIGURE 57**

Y at position 134 can be substituted with N or S

Xaa Xaa Xaa Met Ser (SEQ ID NO: 50043)

Xaa Ile Ser Gly Xaa Gly Xaa Xaa Xaa Tyr Tyr Ala Asp Ser Val Lys Gly (SEQ ID NO: 50044)

Xaa Xaa Gly Xaa Xaa Xaa Xaa Xaa Xaa Xaa Xaa Gly Met Asp (SEQ ID NO: 50045)

Ser Tyr Ala Met Ser (SEQ ID NO: 50513)

Val Ile Ser Gly Gly Gly Ser Ser Thr Tyr Tyr Ala Asp Ser Val Lys Gly (SEQ ID NO: 50514)

Trp Arg Gly Asn Pro Thr Asp Tyr Gly Met Asp (SEQ ID NO: 50515)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | |
| CONSENSUS | E | V | Q | L | L | E | S | - | G | G | G | L | V | Q | P | G | G | S | L | R | L | S | C | A | A | S |
| 21-225_146A2_HC | . | | . | | . | | . | | . | . | . | . | . | . | . | . | . | . | . | . | . | . | . | . | . | . |
| 21-225_146A3_HC | | . | | . | . | . | | . | . | . | . | . | . | . | . | . | . | | . | . | . | . | . | | . | . |
| 21-225_146B11_HC | | . | | . | . | . | | . | . | . | . | . | . | . | . | . | . | | . | . | . | . | . | | . | . |
| 21-225_146C11_HC | . | | . | | . | | . | | . | . | . | . | . | . | . | . | . | . | . | . | . | . | . | . | . | . |
| 21-225_147B12_HC | . | | . | | . | | . | | . | . | . | . | . | . | . | . | . | . | . | . | . | . | . | . | . | . |
| 21-225_147E11_HC | . | | . | | . | | . | | . | . | . | . | . | . | . | . | . | . | . | . | . | . | . | . | . | . |
| 21-225_147E7_HC | . | | . | | . | | . | | . | . | . | . | . | . | . | . | . | . | . | . | . | . | . | . | . | . |
| 21-225_148C6_HC | . | | . | | . | | . | | . | . | . | . | . | . | . | . | . | . | . | . | . | . | . | . | . | . |
| 21-225_148G11_HC | . | | . | | . | | . | | . | . | . | . | . | . | . | . | . | . | . | . | . | . | . | . | . | . |
| 21-225_148G6_HC | . | | . | | . | | . | | . | . | . | . | . | . | . | . | . | . | . | . | . | . | . | . | . | . |
| 21-225_148H11_HC | . | | . | | . | | . | | . | . | . | . | . | . | . | . | . | . | . | . | . | . | . | . | . | . |
| 21-225_149C10_HC | | | . | | . | | . | | . | . | . | . | . | . | . | . | . | | . | . | ♭ | . | . | | . | . |
| 21-225_149C7_HC | | | . | | . | | . | | . | . | . | . | . | . | . | . | . | | . | . | . | . | . | | . | . |
| 21-225_150F6_HC | | | . | | . | | . | | . | . | . | . | . | . | . | . | . | | . | . | . | . | . | | . | . |
| 21-225_150H11_HC | | | . | | . | | . | | . | . | . | . | . | . | . | . | . | | . | . | . | . | . | | . | . |
| 21-225_151B9_HC | | . | | . | . | . | | . | . | . | . | . | . | . | . | . | . | | . | . | . | . | . | | . | . |
| 21-225_151H5_HC | | . | | . | . | . | | . | . | . | . | . | . | . | . | . | . | | . | . | . | . | . | | . | . |
| 21-225_151H7_HC | | . | | . | . | . | | . | . | . | . | . | . | . | . | . | . | | . | . | . | . | . | | . | . |
| 21-225_152D7_HC | . | | . | | . | | . | | . | . | . | . | . | . | . | . | . | . | . | . | . | . | . | . | . | . |
| 21-225_152G6_HC | . | | . | | . | | . | | . | . | . | . | . | . | . | . | . | . | . | . | . | . | . | . | . | . |
| 21-225_154C4_HC | . | | . | | . | | . | | . | . | . | . | ! | . | . | . | . | . | . | . | . | . | . | . | . | . |
| 21-225_154E10_HC | . | | . | | . | | . | | . | . | . | . | . | . | . | . | . | . | . | . | . | . | . | . | . | . |
| 21-225_155C10_HC | . | | . | | . | | . | | . | . | . | . | . | . | . | . | . | . | . | . | . | . | . | . | . | . |
| 21-225_207C9_HC | . | | . | | . | | . | | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | H_CDR1 | | | | | | | | | | | | | | H_FR2 | | |
| CONSENSUS | G | - | F | T | F | S | S | - | - | - | - | - | Y | A | M | S | W | V | R | Q | A | P | G | K | G | L |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | I | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | N | . | . | . | . | . | . | V | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | P | . | . | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | M | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | . | . | T | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | . | . | . | . | . | . | . | . | . | . | . | . | . | . | . | . | . | . | . | . | . | . |

# EP 4 435 105 A2

## FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
|  |  |  |  |  |  |  |  |  |  |  |  |  |  | H_CDR2 |  |  |  |  |  |  |  |  |  |  |  |  |
| CONSENSUS | E | W | V | S | V | I | S | G | G | - | - | - | G | S | S | T | Y | Y | A | D | S | V | K | G | R | F |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | T | T | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | A | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | I | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | A | . | . | . | S | . | . | . | . | . | G | . | . | . | . | . | . | . | . | . | . | . | . |

4022

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | | |
| CONSENSUS | T | I | S | R | D | N | S | K | N | T | L | Y | L | Q | M | N | S | L | R | A | E | D | T | A | V | Y |
| 21-225_146A2_HC | . | . | . | . | . | . | . | . | . | . | . | M | . | . | . | . | . | . | . | . | . | . | . | . | . | . |
| 21-225_146A3_HC | | | . | . | . | . | . | . | . | . | . | . | . | . | . | . | . | . | . | . | . | . | . | . | . | . |
| 21-225_146B11_HC | | | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | L | . | . | . | . | . | . | . | . | . | . | . | . | . | . | . | . | . | . | . | . | . | . | . | F |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | . | . | . | . | . | . | . | . | . | . | . |
| 21-225_148G6_HC | . | . | . | . | . | . | . | . | M | . | . | M | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | . | . | . | . | . | . |
| 21-225_149C7_HC | | | . | . | . | . | . | . | . | . | . | . | . | . | . | . | . | . | . | . | . | . | . | . | . | . |
| 21-225_150F6_HC | | | . | . | . | . | . | . | . | . | . | . | . | . | . | . | . | . | . | . | . | . | . | . | . | . |
| 21-225_150H11_HC | | | . | . | . | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | | H_CDR3 | | | | | | |
| CONSENSUS | Y | C | A | K | W | R | G | N | P | T | - | - | - | - | - | - | - | - | - | - | - | - | - | - | - | - |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | . | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | . | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | A | G | . | T | T | O | S | Y | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | D | Y | G | M | D | V | W | G | Q | G | T | T | V | T | V | S | S |
| 21-225_146A2_HC | | | | | | | | | | | | | | | | | | | |
| 21-225_146A3_HC | | | | | | | | | | | | | | | | | | | |
| 21-225_146B11_HC | | | | | | | | | | | | | | | | | | | |
| 21-225_146C11_HC | | | | | | | | | | | | | | | | | | | |
| 21-225_147B12_HC | | | | | | | | | | | | | | | | | | | |
| 21-225_147E11_HC | | | | | | | | | | | | | | | | | | | |
| 21-225_147E7_HC | | | | | | | | | | | | | | | | | | | |
| 21-225_148C6_HC | | | | | | | | | | | | | | | | | | | |
| 21-225_148G11_HC | | | | | | | | | | | | | | | | | | | |
| 21-225_148G6_HC | | | | | | | | | | | | | | | | | | | |
| 21-225_148H11_HC | | | | | | | | | | | | | | | | | | | |
| 21-225_149C10_HC | | | | | | | | | | | | | | | | | | | |
| 21-225_149C7_HC | | | | | | | | | | | | | | | | | | | |
| 21-225_150F6_HC | | | | | | | | | | | | | | | | | | | |
| 21-225_150H11_HC | | | | | | | | | | | | | | | | | | | |
| 21-225_151B9_HC | | | | | | | | | | | | | | | | | | | |
| 21-225_151H5_HC | | | | | | | | | | | | | | | | | | | |
| 21-225_151H7_HC | | | | | | | | | | | | | | | | | | | |
| 21-225_152D7_HC | | | | S | | | | | | | | | | | | | | | |
| 21-225_152G6_HC | | | | | | | | | | | | | | | | | | | |
| 21-225_154C4_HC | | | | | | | | | | | | | | | | | | | |
| 21-225_154E10_HC | | | | | | | | | | | | | | | | | | | |
| 21-225_155C10_HC | | | | | | | | | | | | | | | | | | | |
| 21-225_207C9_HC | | Y | Y | N | | | | | | | | | | | | | | | |

**FIGURE 57**

Table 65.   Consensus 31-VH3|3-33/D4|4-17|RF2/JH4 (SEQ ID NO: 50282):

QVQLVES-GGGVVQPGRSLRLSCAASG-FTFSS-----YGMHWVRQAPGKGLEWVAIIWYD---
GSNKYYADSVKGRFTISRDNSKNTLYLQMNSLRAEDTAVYYCARDHYDFW-----------------SGHFDYWGQGTLVTVSS  wherein:

S at position 33 can be substituted with N or T

M at position 41 can be substituted with L

I at position 57 can be substituted with V or A

I at position 58 can be substituted with M

S at position 66 can be substituted with T

N at position 67 can be substituted with Y or S

A at position 71 can be substituted with G, T or V

D at position 109 can be substituted with E

H at position 110 can be substituted with R, Q, A, G, T or Y

Y at position 111 can be substituted with G, F or II

D at position 112 can be substituted with I or F

F at position 113 can be substituted with V or L

W at position 114 can be substituted with G or null (-)

S at position 133 can be substituted with A or null (-)

G at position 134 can be substituted with T or E

H at position 135 can be substituted with Y, W or F

F at position 136 can be substituted with L or S

**FIGURE 57**

D at position 137 can be substituted with A, C or G

Y at position 138 can be substituted with F or S

Xaa Tyr Gly Xaa His (SEQ ID NO: 50253)

Xaa Xaa Trp Tyr Asp Gly Xaa Xaa Lys Tyr Tyr Xaa Asp Ser Val Lys Gly (SEQ ID NO: 50254)

Xaa Xaa Xaa Xaa Xaa Xaa Xaa Xaa Xaa Xaa Xaa Xaa (SEQ ID NO: 50255)

Ser Tyr Gly Met His (SEQ ID NO: 50516)

Ile Ile Trp Tyr Asp Gly Ser Asn Lys Tyr Tyr Ala Asp Ser Val Lys Gly (SEQ ID NO: 50517)

Asp His Tyr Asp Phe Trp Ser Gly His Phe Asp Tyr (SEQ ID NO: 50518)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A | S |
| 21-225_147E10_HC | . | . | . | . | . | . | . | . | . | . | . | . | . | . | . | . | X | . | . | . | . | . | . | . | . | . |
| 21-225_148B2_HC | | | . | | . | | . | | | . | | . | | . | | . | . | | | | . | | | | . | . |
| 21-225_148G10_HC | | | . | | . | | . | | | . | | . | | . | | . | . | | | | . | | | | . | . |
| 21-225_149F1_HC | . | | . | . | . | . | . | | . | . | | . | | . | . | . | . | | . | . | . | | | | . | . |
| 21-225_150B11_HC | . | | . | . | . | | . | . | . | . | | . | | . | . | . | . | | | | . | | | | . | . |
| 21-225_151A10_HC | . | | . | . | . | | . | . | . | . | | . | | . | . | . | . | | | | . | | | | . | . |
| 21-225_151G5_HC | . | . | . | . | M | . | . | . | . | . | | . | | . | . | . | . | | | | . | | | | . | . |
| 21-225_155A5_HC | . | . | . | . | . | . | . | . | . | . | D | . | | . | . | . | . | | | | . | | | | . | . |
| 21-225_170A1_HC | . | . | . | . | . | . | . | . | . | . | | . | | . | . | . | . | | | | . | | | | . | . |
| 21-225_176H4_HC | . | . | . | . | . | . | . | . | . | . | | . | | . | . | . | . | | | | . | | | | . | . |
| 21-225_181C10_HC | . | . | . | . | . | . | . | . | . | . | | . | | . | . | . | . | | | | . | | | | . | . |
| 21-225_181C2_HC | . | | . | | . | | . | . | . | . | | . | | . | . | . | . | | | | . | | | V | . | . |
| 21-225_186F7_HC | | | . | | . | | . | . | . | . | | . | | . | . | . | . | | | | . | | | | . | . |
| 21-225_189G2_HC | | | . | | . | | . | . | . | . | | . | | . | . | . | . | | | | | | | | . | . |
| 21-225_226A5_HC | . | . | . | . | . | . | . | . | . | . | | . | | . | . | . | . | | | | . | | | | . | . |
| 21-225_22A1_HC | . | . | . | . | . | . | . | . | . | . | | . | | . | . | . | . | | | | . | | | | . | . |
| 21-225_28G3_HC | . | . | . | . | . | . | . | . | . | . | | . | | . | . | . | . | | | | . | | | | . | . |
| 21-225_46F2_HC | . | . | . | . | . | . | . | . | . | . | | . | | . | . | . | . | | | | . | | | | . | . |
| 21-225_57H3_HC | . | . | . | . | . | . | . | . | . | . | | . | | . | . | . | . | | | | . | | | | . | . |
| 21-225_62E6_HC | . | . | . | . | . | . | . | . | . | . | | . | | . | . | . | . | | | | . | | | | . | . |
| 21-225_64H9_HC | . | . | . | . | . | . | . | . | . | . | | . | | . | . | . | . | | | | . | | | | . | . |
| 21-225_65A6_HC | . | . | . | . | . | . | . | . | . | . | | . | | . | . | . | . | | | | . | | | | . | . |
| 21-225_67C3_HC | . | . | . | . | . | . | . | . | . | . | | . | | . | . | . | . | | | | . | | | | . | . |
| 21-225_70A5_HC | . | . | . | . | . | . | . | . | . | . | | . | | . | . | . | . | | | | . | | | | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | G | M | H | W | V | R | Q | A | P | G | K | G | L |
| 21-225_147E10_HC | . | . | . | . | . | . | N | . | . | . | . | . | . | . | Ł | . | . | . | . | . | . | . | . | . | . | . |
| 21-225_148B2_HC | . | . | . | . | . | . | . | . | . | . | . | . | . | . | . | . | . | . | . | . | . | . | . | . | . | . |
| 21-225_148G10_HC | . | . | . | . | . | . | . | . | . | . | . | . | . | . | . | . | . | . | . | . | . | . | . | . | . | . |
| 21-225_149F1_HC | . | . | . | . | . | . | . | . | . | . | . | . | . | . | . | . | . | . | . | . | . | . | . | . | . | . |
| 21-225_150B11_HC | . | . | . | . | . | . | . | . | . | . | . | . | . | . | . | . | . | . | . | . | . | . | . | . | . | . |
| 21-225_151A10_HC | . | . | . | . | . | . | N | . | . | . | . | . | . | . | . | . | . | . | . | . | . | . | . | . | . | . |
| 21-225_151G5_HC | . | . | . | I | . | . | . | . | . | . | . | . | . | . | . | . | . | . | . | . | . | . | . | . | . | . |
| 21-225_155A5_HC | . | . | . | . | . | . | . | . | . | . | . | . | . | . | . | . | . | . | . | . | . | . | . | . | . | . |
| 21-225_170A1_HC | . | . | . | . | . | . | . | . | . | . | . | . | . | . | . | . | . | . | . | . | . | . | . | . | . | . |
| 21-225_176H4_HC | . | . | . | . | . | . | . | . | . | . | . | . | . | . | . | . | . | . | . | . | . | . | . | . | . | . |
| 21-225_181C10_HC | . | . | . | . | . | . | . | . | . | . | . | . | . | . | . | . | . | . | . | . | . | . | . | . | . | . |
| 21-225_181C2_HC | . | . | . | . | . | . | . | . | . | . | . | . | . | . | . | . | . | . | . | . | . | . | . | . | . | . |
| 21-225_186F7_HC | . | . | . | . | . | . | T | . | . | . | . | . | . | . | . | . | . | . | . | . | . | . | . | . | . | . |
| 21-225_189G2_HC | . | . | . | . | . | . | N | . | . | . | . | . | . | . | . | . | . | . | . | . | . | . | . | . | . | . |
| 21-225_226A5_HC | . | . | . | . | . | . | N | . | . | . | . | . | . | . | . | . | . | . | . | . | . | . | . | . | . | . |
| 21-225_22A1_HC | . | . | . | . | . | . | . | . | . | . | . | . | . | . | . | . | . | . | . | . | . | . | . | . | . | . |
| 21-225_28G3_HC | . | . | . | I | . | . | T | . | . | . | . | . | . | . | . | . | . | . | . | . | . | . | . | . | . | . |
| 21-225_46F2_HC | . | . | . | . | . | . | . | . | . | . | . | . | . | . | . | . | . | . | . | . | . | . | . | . | . | . |
| 21-225_57H3_HC | . | . | . | . | . | . | . | . | . | . | . | . | . | . | . | . | . | . | . | . | . | . | . | . | . | . |
| 21-225_62E6_HC | . | . | . | . | . | . | . | . | . | . | . | . | . | . | . | . | . | . | . | . | . | . | . | . | . | . |
| 21-225_64H9_HC | . | . | . | . | . | . | T | . | . | . | . | . | . | . | . | . | . | . | . | . | . | . | . | . | . | . |
| 21-225_65A6_HC | . | . | . | . | . | . | N | . | . | . | . | . | . | . | . | . | . | . | . | . | . | . | . | . | . | . |
| 21-225_67C3_HC | . | . | . | . | . | . | . | . | . | . | . | . | . | . | . | . | . | . | . | . | . | . | . | . | . | . |
| 21-225_70A5_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | V | A | I | I | W | Y | D | - | - | - | G | S | N | K | Y | Y | A | D | S | V | K | G | R | F |
| 21-225_147E10_HC | . | . | . | . | . | . | . | . | . | . | . | . | . | . | . | . | . | . | . | . | . | . | . | . | . | . |
| 21-225_148B2_HC | . | . | . | . | . | . | . | . | . | . | . | . | . | . | Y | . | . | . | . | . | . | . | . | . | . | . |
| 21-225_148G10_HC | . | . | . | . | . | . | . | . | . | . | . | . | . | . | . | . | . | . | . | . | . | . | . | . | . | . |
| 21-225_149F1_HC | . | . | . | . | . | . | . | . | . | . | . | . | . | . | Y | . | . | . | . | . | . | . | . | . | . | . |
| 21-225_150B11_HC | . | . | . | . | . | . | . | . | . | . | . | . | . | . | . | . | . | . | . | . | . | . | . | . | . | . |
| 21-225_151A10_HC | . | . | L | . | . | . | . | . | . | . | . | . | . | . | . | . | . | . | . | . | . | . | . | . | . | . |
| 21-225_151G5_HC | . | . | . | . | . | . | . | . | . | . | . | . | . | . | . | . | . | . | . | . | . | . | . | . | . | . |
| 21-225_155A5_HC | . | . | . | . | . | . | . | . | . | . | . | . | . | . | S | . | . | . | . | . | . | . | . | . | . | . |
| 21-225_170A1_HC | . | . | . | . | . | . | . | . | . | . | . | . | . | . | Y | . | . | . | . | . | . | . | . | . | . | . |
| 21-225_176H4_HC | . | . | . | . | . | . | . | . | . | . | . | . | . | . | Y | . | . | . | . | . | . | . | . | . | . | . |
| 21-225_181C10_HC | . | . | M | . | . | . | . | . | . | . | . | . | . | . | Y | . | . | . | . | . | . | . | . | . | . | . |
| 21-225_181C2_HC | . | . | . | T | . | . | . | . | . | . | . | . | . | . | Y | . | . | . | . | . | . | . | . | . | . | . |
| 21-225_186F7_HC | . | . | . | . | . | . | . | . | . | . | . | . | . | . | Y | . | . | . | . | . | . | . | . | . | . | . |
| 21-225_189G2_HC | . | . | . | . | . | . | . | . | . | . | . | . | . | . | Y | . | . | . | . | . | . | . | . | . | . | . |
| 21-225_226A5_HC | . | . | . | . | . | . | . | . | . | . | . | . | . | T | . | . | . | . | Y | . | . | . | . | . | . | . |
| 21-225_22A1_HC | . | . | . | . | V | M | . | . | . | . | . | . | . | . | . | . | . | . | . | . | . | . | . | . | . | . |
| 21-225_28G3_HC | . | . | . | . | V | . | . | . | . | . | . | . | . | . | . | . | . | . | . | . | . | . | . | . | . | . |
| 21-225_46F2_HC | . | . | . | . | . | . | . | . | . | . | . | . | . | . | . | . | . | . | V | . | . | . | . | . | . | . |
| 21-225_57H3_HC | . | . | . | . | A | . | . | . | . | . | . | . | . | . | . | . | . | . | . | . | . | . | . | . | . | . |
| 21-225_62E6_HC | . | . | . | . | A | . | . | . | . | . | . | . | . | . | . | . | . | . | G | . | . | . | . | . | . | V |
| 21-225_64H9_HC | . | . | . | . | . | . | . | . | . | . | . | . | . | . | . | . | . | . | . | . | . | . | . | . | . | . |
| 21-225_65A6_HC | . | . | . | . | V | . | . | . | . | . | . | . | . | . | . | . | . | . | G | . | . | . | . | . | . | . |
| 21-225_67C3_HC | . | . | . | . | A | . | . | . | . | . | . | . | . | . | . | . | . | . | G | . | . | . | . | . | . | V |
| 21-225_70A5_HC | . | . | . | . | V | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_147E10_HC | . | . | . | . | . | . | . | . | . | . | . | . | . | . | . | . | . | . | . | . | . | . | . | . | . | . |
| 21-225_148B2_HC | | | . | | . | . | . | . | . | . | . | . | . | . | . | . | . | . | S | . | | | | . | . | . |
| 21-225_148G10_HC | | | | . | . | . | . | . | . | . | . | . | . | . | . | . | . | | | | | | | . | . | . |
| 21-225_149F1_HC | . | | . | . | . | . | . | . | . | . | . | . | . | . | . | . | . | . | S | . | . | . | . | . | . | . |
| 21-225_150B11_HC | . | | . | . | . | . | . | . | . | . | . | . | . | . | . | . | . | . | . | . | . | . | . | . | . | . |
| 21-225_151A10_HC | . | | . | . | . | . | . | . | . | . | . | . | . | . | . | . | . | . | . | . | . | . | . | . | . | . |
| 21-225_151G5_HC | . | | . | . | . | . | . | . | . | . | . | . | . | . | . | . | . | . | . | . | . | . | . | . | . | . |
| 21-225_155A5_HC | . | | . | . | . | . | . | . | . | . | . | . | . | :: | . | . | . | . | . | . | . | . | . | . | . | . |
| 21-225_170A1_HC | . | | . | . | . | . | . | . | . | . | . | . | . | . | . | . | . | . | . | . | . | . | . | . | . | . |
| 21-225_176H4_HC | . | | . | . | . | . | . | . | . | . | . | . | . | . | . | S | . | . | . | . | . | . | . | . | . | . |
| 21-225_181C10_HC | . | | . | . | . | . | . | . | . | . | . | . | . | . | . | . | . | . | . | . | . | . | . | . | . | . |
| 21-225_181C2_HC | | | | . | . | . | . | . | . | . | . | . | . | . | . | . | . | . | . | . | . | . | . | . | . | . |
| 21-225_186F7_HC | | | | . | . | . | . | . | . | . | . | . | . | . | . | . | . | . | . | . | . | . | . | . | . | . |
| 21-225_189G2_HC | | | | . | . | . | . | . | . | . | . | . | . | . | . | . | . | . | . | . | . | . | . | . | . | . |
| 21-225_226A5_HC | | | | . | . | . | . | . | . | . | . | . | . | . | . | . | . | . | . | . | . | . | . | . | . | . |
| 21-225_22A1_HC | . | | . | . | . | . | . | . | . | . | . | . | . | . | . | . | . | . | . | . | . | . | . | . | . | . |
| 21-225_28G3_HC | . | | . | . | . | . | . | . | . | . | . | . | . | . | . | . | . | . | . | . | . | . | . | . | . | . |
| 21-225_46F2_HC | . | | . | . | . | . | . | . | . | . | . | . | . | . | . | . | . | . | . | . | . | . | . | . | . | . |
| 21-225_57H3_HC | . | | . | . | . | . | . | . | | | | . | . | . | . | . | . | . | . | . | . | . | . | . | . | . |
| 21-225_62E6_HC | . | . | . | . | . | . | . | . | . | S | . | :: | . | . | . | . | . | . | . | . | . | . | . | . | . | . |
| 21-225_64H9_HC | . | . | . | . | . | . | . | . | . | . | . | . | . | . | . | . | . | . | . | . | . | . | . | . | . | . |
| 21-225_65A6_HC | . | . | . | . | . | . | . | . | . | . | . | . | . | . | . | . | . | . | . | . | . | . | . | . | . | . |
| 21-225_67C3_HC | . | . | . | . | . | . | . | . | . | S | . | :: | . | . | . | . | . | . | . | . | . | . | . | . | . | . |
| 21-225_70A5_HC | . | . | . | . | . | . | . | . | . | S | . | . | . | . | . | . | . | . | S | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | | Y | D | F | W | - | - | - | - | - | - | - | - | - | - | - | - | - | - | - | - |
| 21-225_147E10_HC | . | | | . | . | H | . | . | . | . | . | . | . | . | . | . | . | . | . | . | . | . | . | . | . | . |
| 21-225_148B2_HC | . | | . | | H | F | . | . | . | . | . | . | . | . | . | . | . | . | . | . | . | . | . | . | . | . |
| 21-225_148G10_HC | . | | . | | R | . | . | . | . | . | . | . | . | . | . | . | . | . | . | . | . | . | . | . | . | . |
| 21-225_149F1_HC | . | . | . | . | . | H | F | . | . | . | . | . | . | . | . | . | . | . | . | . | . | . | . | . | . | . |
| 21-225_150B11_HC | . | . | . | . | . | R | . | . | . | . | . | . | . | . | . | . | . | . | . | . | . | . | . | . | . | . |
| 21-225_151A10_HC | . | . | . | . | . | H | . | . | . | . | . | . | . | . | . | . | . | . | . | . | . | . | . | . | . | . |
| 21-225_151G5_HC | . | . | . | . | . | R | . | . | . | . | . | . | . | . | . | . | . | . | . | . | . | . | . | . | . | . |
| 21-225_155A5_HC | . | . | . | . | . | R | . | . | . | . | . | . | . | . | . | . | . | . | . | . | . | . | . | . | . | . |
| 21-225_170A1_HC | . | . | . | . | . | A | H | . | . | . | . | . | . | . | . | . | . | . | . | . | . | . | . | . | . | . |
| 21-225_176H4_HC | . | . | . | . | . | A | H | . | . | . | . | . | . | . | . | . | . | . | . | . | . | . | . | . | . | . |
| 21-225_181C10_HC | . | . | . | . | . | H | . | . | . | . | . | . | . | . | . | . | . | . | . | . | . | . | . | . | . | . |
| 21-225_181C2_HC | . | | . | | H | . | . | . | . | . | . | . | . | . | . | . | . | . | . | . | . | . | . | . | . | . |
| 21-225_186F7_HC | | | . | | H | . | . | . | . | . | . | . | . | . | . | . | . | . | . | . | . | . | . | . | . | . |
| 21-225_189G2_HC | | | . | | H | F | . | . | . | . | . | . | . | . | . | . | . | . | . | . | . | . | . | . | . | . |
| 21-225_226A5_HC | | | . | E | G | H | . | . | . | . | . | . | . | . | . | . | . | . | . | . | . | . | . | . | . | . |
| 21-225_22A1_HC | . | . | . | . | . | G | G | F | L | - | . | . | . | . | . | . | . | . | . | . | . | . | . | . | . | . |
| 21-225_28G3_HC | . | . | . | . | . | R | . | . | . | . | . | . | . | . | . | . | . | . | . | . | . | . | . | . | . | . |
| 21-225_46F2_HC | . | . | . | . | . | R | . | . | . | . | . | . | . | . | . | . | . | . | . | . | . | . | . | . | . | . |
| 21-225_57H3_HC | . | . | . | . | . | Y | G | I | V | G | . | . | . | . | . | . | . | . | . | . | . | . | . | . | . | . |
| 21-225_62E6_HC | . | . | . | . | . | Q | G | I | V | G | . | . | . | . | . | . | . | . | . | . | . | . | . | . | . | . |
| 21-225_64H9_HC | . | . | . | . | . | T | . | . | . | . | . | . | . | . | . | . | . | . | . | . | . | . | . | . | . | . |
| 21-225_65A6_HC | . | . | . | . | . | Q | G | I | V | G | . | . | . | . | . | . | . | . | . | . | . | . | . | . | . | . |
| 21-225_67C3_HC | . | . | . | . | . | Q | G | I | V | G | . | . | . | . | . | . | . | . | . | . | . | . | . | . | . | . |
| 21-225_70A5_HC | . | . | . | . | . | Q | G | I | V | G | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  |  |  |  |  | H_FR4 |  |  |  |  |  |  |
| CONSENSUS | - | - | S | G | H | F | D | Y | W | G | Q | G | T | L | V | T | V | S | S |
| 21-225_147E10_HC | . | . | . | . | . | . | . | . | . | . | . | . | . | . | . | . | . | . | . |
| 21-225_148B2_HC | . | . | . | . | . | . | . | . | . | . | . | . | . | . | . | . | . | . | . |
| 21-225_148G10_HC | . | . | . | . | . | . | . | . | . | . | . | . | . | . | . | . | . | . | . |
| 21-225_149F1_HC | . | . | . | . | . | . | . | . | . | . | . | . | . | . | . | . | . | . | . |
| 21-225_150B11_HC | . | . | . | . | . | . | . | . | . | . | . | . | . | . | . | . | . | . | . |
| 21-225_151A10_HC | . | . | . | . | . | . | . | . | . | . | . | . | . | . | . | . | . | . | . |
| 21-225_151G5_HC | . | . | . | . | . | . | . | . | . | . | . | . | . | . | . | . | . | . | . |
| 21-225_155A5_HC | . | . | . | . | . | . | . | . | . | . | . | . | . | . | . | . | . | . | . |
| 21-225_170A1_HC | . | . | . | . | Y | . | . | S | . | . | . | . | . | . | . | . | . | . | . |
| 21-225_176H4_HC | . | . | . | . | Y | . | A | . | . | . | . | . | A | . | . | . | . | . | . |
| 21-225_181C10_HC | . | . | . | . | . | . | . | . | . | . | . | . | . | . | . | . | . | . | . |
| 21-225_181C2_HC | . | . | . | . | . | . | . | P | . | . | . | . | . | . | . | . | . | . | . |
| 21-225_186F7_HC | . | . | . | . | . | . | . | . | . | . | . | . | . | . | . | . | . | . | . |
| 21-225_189G2_HC | . | . | . | . | Y | S | . | . | . | . | . | . | . | . | . | . | . | . | . |
| 21-225_226A5_HC | . | . | . | . | P | . | G | . | . | . | . | . | . | . | . | . | . | . | . |
| 21-225_22A1_HC | . | . | - | E | W | L | . | . | . | . | . | . | . | . | . | . | . | . | . |
| 21-225_28G3_HC | . | . | . | . | Y | . | . | . | . | . | . | . | . | . | . | . | . | . | . |
| 21-225_46F2_HC | . | . | . | . | Y | . | . | . | . | . | . | . | . | . | . | . | . | . | . |
| 21-225_57H3_HC | . | . | A | T | Y | . | . | . | . | . | . | . | . | . | . | . | . | . | . |
| 21-225_62E6_HC | . | . | A | T | W | . | . | . | . | . | . | . | . | . | . | . | . | . | . |
| 21-225_64H9_HC | . | . | . | . | Y | L | G | . | . | . | . | . | . | . | . | . | . | . | . |
| 21-225_65A6_HC | . | . | A | T | W | . | . | . | . | . | . | . | . | . | . | . | . | . | . |
| 21-225_67C3_HC | . | . | A | T | W | . | . | . | . | . | . | . | . | . | . | . | . | . | . |
| 21-225_70A5_HC | . | . | A | T | W | . | . | . | . | . | . | . | . | . | . | . | . | . | . |

FIGURE 57

Table 66. Consensus 32 -VH4|4-34/D4|4-17|RF2/JH4 (SEQ ID NO: 50283):

QVQLQQW-GAGLLKPSETLSLTCAVYG-GSFS<u>G</u>-----<u>YYWS</u>WIRQPPGKGLEWIG<u>EINH</u>----
<u>SGRTNYNPSLKS</u>RVTISVDTSKNQFSLKLSSVTAADTAVYYCAR<u>DYGG</u>--------------------LDYWGQGTLVTVSS wherein:

G at position 33 can be substituted with V, P, A, D or Y

Y at position 39 can be substituted with C, S or P

Y at position 40 can be substituted with F

I at position 58 can be substituted with S or V

H at position 60 can be substituted with I or Q

R at position 67 can be substituted with S

T at position 68 can be substituted with A or S

N at position 69 can be substituted with T

Y at position 70 can be substituted with F

Xaa Xaa Xaa Trp Ser (SEQ ID NO: 50233)

Glu Xaa Asn Xaa Ser Gly Xaa Xaa Xaa Xaa Asn Pro Ser Leu Lys Ser (SEQ ID NO: 50234)

Asp Tyr Gly Gly Leu Asp Tyr (SEQ ID NO: 50235)

Gly Tyr Tyr Trp Ser (SEQ ID NO: 50519)

Glu Ile Asn His Ser Gly Arg Thr Asn Tyr Asn Pro Ser Leu Lys Ser (SEQ ID NO: 50520)

Asp Tyr Gly Gly Leu Asp Tyr (SEQ ID NO: 50521)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | Q | Q | W | - | G | A | G | L | L | K | P | S | E | T | L | S | L | T | C | A | V | Y |
| 21-225_147G8_HC | . | . | . | . | . | . | . | . | . | . | . | . | . | . | . | . | . | . | . | . | . | . | . | G | . | . |
| 21-225_192F8_HC | . | . | . | . | . | . | . | . | . | . | . | . | . | . | . | . | . | . | . | . | . | . | . | . | . | . |
| 21-225_193E6_HC | . | . | . | . | . | . | . | . | . | . | . | . | . | . | . | . | . | . | . | . | . | . | . | . | . | F |
| 21-225_195B1_HC | . | . | . | . | . | . | . | . | . | . | . | . | . | . | . | . | . | . | . | . | . | . | . | . | . | S |
| 21-225_200A1_HC | . | . | . | . | . | . | . | . | . | . | . | . | . | . | . | . | . | . | . | . | . | . | . | . | . | . |
| 21-225_201B2_HC | . | . | . | . | . | . | . | . | . | . | . | . | . | . | . | . | . | . | . | . | . | . | . | . | . | F |
| 21-225_201E1_HC | . | . | . | . | . | . | . | . | . | . | . | . | . | . | . | . | . | . | . | . | . | . | . | . | . | F |
| 21-225_202D11_HC | . | . | . | . | . | . | . | . | . | . | . | . | . | . | . | . | . | . | . | . | . | . | . | A | . | . |
| 21-225_202D9_HC | . | . | . | . | . | . | . | . | . | . | . | . | . | . | . | . | . | . | . | . | . | . | . | . | . | . |
| 21-225_205G7_HC | . | . | . | . | . | . | . | . | . | . | . | . | . | . | . | . | . | . | . | . | . | . | . | . | . | . |
| 21-225_208A9_HC | . | . | . | . | . | . | . | . | . | . | . | . | . | . | . | . | . | P | . | . | . | . | . | . | . | . |
| 21-225_208B5_HC | . | . | . | . | . | . | . | . | . | . | . | . | . | . | . | . | . | . | . | . | . | . | . | . | . | F |
| 21-225_74C7_HC | . | . | . | . | . | . | . | . | . | . | . | . | . | . | . | . | . | . | . | . | . | . | . | . | . | . |
| 21-225_74D2_HC | . | . | . | . | . | . | . | . | . | . | . | . | . | . | . | . | . | . | . | . | . | . | . | . | . | . |
| 21-225_75G3_HC | . | . | . | . | . | . | . | . | . | . | . | . | . | . | . | . | . | . | . | . | . | . | . | . | . | . |
| 21-225_75G9_HC | . | . | . | . | . | . | . | . | . | . | . | . | . | . | . | . | . | . | . | . | . | . | . | . | . | . |
| 21-225_76A7_HC | . | . | . | . | . | . | . | . | . | . | . | . | . | . | . | . | . | . | . | . | . | . | . | . | . | . |
| 21-225_78E7_HC | . | . | . | . | . | . | . | . | . | . | . | . | . | . | . | . | . | . | . | . | . | . | . | . | . | . |
| 21-225_80A6_HC | . | . | . | . | . | . | . | . | . | . | . | . | . | . | . | . | . | . | . | . | . | . | . | . | . | . |
| 21-225_80B10_HC | . | . | . | . | . | . | . | . | . | . | . | . | . | . | . | . | . | . | . | . | . | . | . | . | . | . |
| 21-225_83B6_HC | . | . | . | . | . | . | . | . | . | . | . | . | . | . | . | . | . | . | . | . | . | . | . | . | . | . |
| 21-225_83C10_HC | . | . | . | . | . | . | . | . | . | . | . | . | . | . | . | . | . | . | . | . | . | . | . | Y | . | . |
| 21-225_85G6_HC | . | . | . | . | . | . | . | . | . | . | . | . | . | . | . | . | . | . | . | . | . | . | . | . | . | . |
| 21-225_93E9_HC | . | . | . | . | . | . | . | . | . | . | . | . | . | . | . | . | . | . | . | . | . | . | . | . | . | . |

## FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | G | S | F | S | G | - | - | - | - | - | Y | W | S | W | I | R | Q | P | P | G | K | G | L | |
| 21-225_147G8_HC | . | . | . | . | . | . | A | . | . | . | . | . | Y | . | . | . | . | . | . | . | . | . | . | . | . | . |
| 21-225_192F8_HC | . | . | . | . | . | . | . | . | . | . | . | . | G | . | . | . | . | . | . | . | . | . | . | . | . | . |
| 21-225_193E6_HC | . | . | . | . | . | . | . | . | . | . | . | . | P | . | . | . | . | . | . | . | . | . | . | . | . | . |
| 21-225_195B1_HC | . | . | . | . | . | D | Y | . | . | . | . | . | Y | P | . | . | . | . | . | . | S | . | . | . | . | . |
| 21-225_200A1_HC | . | . | . | . | . | . | V | . | . | . | . | . | Y | . | . | . | . | . | . | . | . | . | . | . | . | . |
| 21-225_201B2_HC | . | . | . | . | I | . | V | . | . | . | . | . | Y | . | . | T | . | . | . | . | . | . | . | . | . | . |
| 21-225_201E1_HC | D | . | . | . | . | . | P | . | . | . | . | . | Y | . | . | . | . | . | . | . | . | . | . | . | . | . |
| 21-225_202D11_HC | . | . | . | . | . | . | . | . | . | . | . | . | P | . | . | . | . | . | . | . | . | . | . | . | . | . |
| 21-225_202D9_HC | D | . | . | . | . | . | P | . | . | . | . | . | Y | . | . | . | . | . | . | . | . | . | . | . | . | . |
| 21-225_205G7_HC | . | . | . | . | . | . | V | . | . | . | . | . | Y | . | . | . | . | . | . | . | . | . | . | . | . | . |
| 21-225_208A9_HC | . | . | . | . | . | . | V | . | . | . | . | . | S | . | . | . | . | . | . | . | . | . | . | . | . | . |
| 21-225_208B5_HC | . | . | . | . | I | . | V | . | . | . | . | . | Y | . | . | T | . | . | . | . | . | . | . | . | . | . |
| 21-225_74C7_HC | . | . | . | . | L | . | . | . | . | . | . | . | S | . | . | . | . | . | . | . | . | . | . | . | . | . |
| 21-225_74D2_HC | . | . | . | . | . | . | . | . | . | . | . | . | G | . | . | . | . | . | . | . | . | . | . | . | . | . |
| 21-225_75G3_HC | . | . | . | . | L | . | . | . | . | . | . | . | S | . | . | . | . | . | . | . | . | . | . | . | . | . |
| 21-225_75G9_HC | . | . | . | . | . | . | . | . | . | . | . | . | G | . | . | . | . | . | . | . | . | . | . | . | . | . |
| 21-225_76A7_HC | . | . | . | . | . | . | . | . | . | . | . | . | G | . | . | . | . | . | . | . | . | . | . | . | . | . |
| 21-225_78E7_HC | . | . | . | . | L | . | . | . | . | . | . | . | S | . | . | . | . | . | . | . | . | . | . | . | . | . |
| 21-225_80A6_HC | . | . | . | . | . | . | . | . | . | . | . | . | G | . | . | . | . | . | . | . | . | . | . | . | . | . |
| 21-225_80B10_HC | . | . | . | . | . | . | . | . | . | . | . | . | G | . | . | . | . | . | . | . | . | . | . | . | . | . |
| 21-225_83B6_HC | . | . | . | . | . | . | . | . | . | . | . | . | G | . | . | . | . | . | . | . | . | . | . | . | . | . |
| 21-225_83C10_HC | . | . | . | . | . | . | . | . | . | . | . | . | Y | . | . | . | . | . | . | . | . | . | . | . | . | . |
| 21-225_85G6_HC | . | . | . | . | . | . | D | . | . | . | . | . | G | . | . | . | . | . | . | . | . | . | . | . | . | . |
| 21-225_93E9_HC | . | . | . | . | . | . | . | . | . | . | . | . | G | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | E | I | N | H | - | - | - | - | - | S | G | T | N | Y | N | P | S | L | K | S | R | V |
| 21-225_147G8_HC | . | . | . | . | . | . | . | . | . | . | . | . | . | . | S | . | . | . | K | . | . | . | . | . | . | . |
| 21-225_192F8_HC | . | . | . | . | . | . | . | Q | . | . | . | . | . | . | S | S | . | . | . | . | . | . | . | . | . | . |
| 21-225_193E6_HC | . | . | . | . | S | . | . | . | . | . | . | . | . | . | S | . | . | . | . | . | . | . | . | . | . | . |
| 21-225_195B1_HC | . | . | P | . | . | . | . | . | . | . | . | . | . | . | S | . | . | . | . | . | . | . | . | . | . | . |
| 21-225_200A1_HC | . | . | . | . | . | . | . | . | . | . | . | . | . | . | S | . | . | . | . | . | . | . | . | . | . | . |
| 21-225_201B2_HC | . | . | . | . | . | . | . | . | . | . | . | . | . | . | S | . | . | . | . | . | . | . | . | . | . | . |
| 21-225_201E1_HC | . | . | . | . | . | V | . | . | . | . | . | . | . | . | S | . | . | . | . | . | . | . | . | . | . | . |
| 21-225_202D11_HC | . | . | . | . | . | . | . | . | . | . | . | . | . | . | S | . | . | . | . | . | . | . | . | . | . | . |
| 21-225_202D9_HC | . | . | . | . | . | . | . | . | . | . | . | . | . | . | S | . | . | . | . | . | . | . | . | . | . | . |
| 21-225_205G7_HC | . | . | . | . | . | S | . | Q | . | . | . | . | . | . | S | . | T | . | . | . | . | . | . | . | . | . |
| 21-225_208A9_HC | . | . | . | . | . | . | . | . | . | . | . | . | . | . | S | A | . | . | . | . | . | . | . | . | . | . |
| 21-225_208B5_HC | . | . | . | . | . | . | . | . | . | . | . | . | . | . | S | . | . | . | . | . | . | . | . | . | . | . |
| 21-225_74C7_HC | . | . | . | . | . | . | . | L | . | . | . | . | . | . | S | . | . | . | . | . | . | . | . | . | . | . |
| 21-225_74D2_HC | . | . | . | . | . | . | . | . | . | . | . | . | . | . | S | . | . | . | . | . | . | . | . | . | . | . |
| 21-225_75G3_HC | . | . | . | . | . | . | . | L | . | . | . | . | . | . | S | . | . | . | . | . | . | . | . | . | . | . |
| 21-225_75G9_HC | . | . | . | . | . | . | . | . | . | . | . | . | . | . | S | . | . | . | . | . | . | . | . | . | . | . |
| 21-225_76A7_HC | . | . | . | . | . | . | . | . | . | . | . | . | . | . | S | . | . | . | . | . | . | . | . | . | . | . |
| 21-225_78E7_HC | . | . | . | . | . | . | . | L | . | . | . | . | . | . | S | . | . | . | . | . | . | . | . | . | . | . |
| 21-225_80A6_HC | . | . | . | . | . | . | . | . | . | . | . | . | . | . | S | . | . | . | . | . | . | . | . | . | . | . |
| 21-225_80B10_HC | . | . | . | . | . | . | . | . | . | . | . | . | . | . | S | . | . | . | . | . | . | . | . | . | . | . |
| 21-225_83B6_HC | . | . | . | . | . | . | . | . | . | . | . | . | . | . | S | . | . | . | . | . | . | . | . | . | . | . |
| 21-225_83C10_HC | . | . | . | . | . | . | . | . | . | . | . | . | . | . | S | . | . | P | . | . | . | . | . | . | . | . |
| 21-225_85G6_HC | . | . | . | . | . | . | . | . | . | . | . | . | . | . | S | . | . | . | . | . | . | . | . | . | . | . |
| 21-225_93E9_HC | . | . | . | . | . | . | . | . | . | . | . | . | . | . | S | . | . | . | . | . | . | . | . | . | . | . |

4037

## FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | V | D | T | S | K | N | Q | F | S | L | K | L | S | S | V | T | A | A | D | T | A | V | Y |
| 21-225_147G8_HC | . | . | . | . | . | . | . | . | . | . | . | . | . | . | . | . | . | . | . | . | . | . | . | . | . | . |
| 21-225_192F8_HC | . | . | . | . | . | . | . | T | . | . | . | . | . | . | . | ※ | . | . | . | . | . | . | . | . | . | . |
| 21-225_193E6_HC | . | . | . | . | . | . | . | . | . | . | . | . | . | ※ | . | . | . | . | . | . | . | . | . | . | . | . |
| 21-225_195B1_HC | . | . | . | . | . | . | . | . | . | . | . | . | . | . | . | ※ | . | . | . | . | . | . | . | . | . | . |
| 21-225_200A1_HC | . | . | . | L | . | . | . | . | . | . | . | . | . | . | . | . | . | . | . | . | . | . | . | . | . | . |
| 21-225_201B2_HC | . | . | . | . | . | . | . | . | . | . | . | . | . | . | . | . | . | . | . | . | . | . | . | . | . | . |
| 21-225_201E1_HC | . | . | . | . | . | . | . | . | . | . | . | . | . | . | . | . | . | . | . | . | . | . | . | . | . | . |
| 21-225_202D11_HC | . | . | . | . | . | . | . | . | . | . | . | . | . | . | . | . | . | . | . | . | . | . | . | . | . | . |
| 21-225_202D9_HC | . | . | . | . | . | . | . | . | . | . | . | . | . | . | . | . | . | . | . | . | . | . | . | . | . | . |
| 21-225_205G7_HC | . | . | . | . | . | . | . | . | . | . | . | . | . | N | . | I | . | . | . | . | . | . | . | . | . | . |
| 21-225_208A9_HC | . | . | . | I | . | . | . | . | . | . | . | . | . | . | . | . | . | . | . | . | . | . | . | . | . | . |
| 21-225_208B5_HC | . | . | . | . | . | . | . | . | . | . | . | . | . | . | . | . | . | . | . | . | . | . | . | . | . | . |
| 21-225_74C7_HC | . | . | . | . | . | . | . | . | S | . | . | . | . | T | . | ※ | . | . | . | . | . | . | . | . | . | . |
| 21-225_74D2_HC | . | . | . | . | . | . | . | S | . | . | . | . | . | . | . | . | . | . | . | . | . | . | . | . | . | . |
| 21-225_75G3_HC | . | . | . | . | . | . | . | . | S | . | . | . | . | T | . | ※ | . | . | . | . | . | . | . | . | . | . |
| 21-225_75G9_HC | . | . | . | . | . | . | . | S | . | . | . | . | . | . | . | . | . | . | . | . | . | . | . | . | . | . |
| 21-225_76A7_HC | . | . | . | . | . | . | . | S | . | . | . | . | . | . | . | . | . | . | . | . | . | . | . | . | . | . |
| 21-225_78E7_HC | . | . | . | . | . | . | . | . | S | . | . | . | . | T | . | ※ | . | . | . | . | . | . | . | . | . | . |
| 21-225_80A6_HC | . | . | . | . | . | . | . | S | ※ | . | . | . | . | . | . | . | . | . | . | . | . | . | . | . | . | . |
| 21-225_80B10_HC | . | . | . | . | . | . | . | . | . | . | . | . | . | . | . | . | . | . | . | . | . | . | . | . | . | . |
| 21-225_83B6_HC | . | . | . | . | . | . | . | S | . | . | . | . | . | . | . | . | . | . | . | . | . | . | . | . | . | . |
| 21-225_83C10_HC | . | . | . | . | . | . | . | . | . | . | . | . | . | . | . | . | . | . | . | . | . | . | . | . | . | F |
| 21-225_85G6_HC | . | . | . | . | . | . | . | S | . | . | . | . | . | . | . | . | . | . | . | . | . | . | . | . | . | . |
| 21-225_93E9_HC | . | . | . | . | . | . | . | S | . | ※ | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | Y | G | G | - | - | - | - | - | - | - | - | - | - | - | - | - | - | - | - | - | - |
| 21-225_147G8_HC | . | . | . | . | . | . | . | V | . | . | . | . | . | . | . | . | . | . | . | . | . | . | . | . | . | . |
| 21-225_192F8_HC | . | . | . | . | . | . | . | V | . | . | . | . | . | . | . | . | . | . | . | . | . | . | . | . | . | . |
| 21-225_193E6_HC | . | . | . | . | . | . | . | . | . | . | . | . | . | . | . | . | . | . | . | . | . | . | . | . | . | . |
| 21-225_195B1_HC | . | . | . | . | . | . | . | A | . | . | . | . | . | . | . | . | . | . | . | . | . | . | . | . | . | . |
| 21-225_200A1_HC | . | . | . | . | . | . | . | V | . | . | . | . | . | . | . | . | . | . | . | . | . | . | . | . | . | . |
| 21-225_201B2_HC | . | . | . | . | . | . | . | V | . | . | . | . | . | . | . | . | . | . | . | . | . | . | . | . | . | . |
| 21-225_201E1_HC | . | . | . | . | . | . | . | . | . | . | . | . | . | . | . | . | . | . | . | . | . | . | . | . | . | . |
| 21-225_202D11_HC | . | . | . | . | . | . | . | V | . | . | . | . | . | . | . | . | . | . | . | . | . | . | . | . | . | . |
| 21-225_202D9_HC | . | . | . | . | . | . | . | . | . | . | . | . | . | . | . | . | . | . | . | . | . | . | . | . | . | . |
| 21-225_205G7_HC | . | . | . | . | . | . | . | V | . | . | . | . | . | . | . | . | . | . | . | . | . | . | . | . | . | . |
| 21-225_208A9_HC | . | . | . | . | . | . | . | V | . | . | . | . | . | . | . | . | . | . | . | . | . | . | . | . | . | . |
| 21-225_208B5_HC | . | . | . | . | . | . | . | V | . | . | . | . | . | . | . | . | . | . | . | . | . | . | . | . | . | . |
| 21-225_74C7_HC | . | . | . | . | . | . | . | . | . | . | . | . | . | . | . | . | . | . | . | . | . | . | . | . | . | . |
| 21-225_74D2_HC | . | . | . | . | . | . | . | . | . | . | . | . | . | . | . | . | . | . | . | . | . | . | . | . | . | . |
| 21-225_75G3_HC | . | . | . | . | . | . | . | . | . | . | . | . | . | . | . | . | . | . | . | . | . | . | . | . | . | . |
| 21-225_75G9_HC | . | . | . | . | . | . | . | . | . | . | . | . | . | . | . | . | . | . | . | . | . | . | . | . | . | . |
| 21-225_76A7_HC | . | . | . | . | . | . | . | . | . | . | . | . | . | . | . | . | . | . | . | . | . | . | . | . | . | . |
| 21-225_78E7_HC | . | . | . | . | . | . | . | . | . | . | . | . | . | . | . | . | . | . | . | . | . | . | . | . | . | . |
| 21-225_80A6_HC | . | . | . | . | . | . | . | . | . | . | . | . | . | . | . | . | . | . | . | . | . | . | . | . | . | . |
| 21-225_80B10_HC | . | . | . | . | . | . | . | . | . | . | . | . | . | . | . | . | . | . | . | . | . | . | . | . | . | . |
| 21-225_83B6_HC | . | . | . | . | . | . | . | . | . | . | . | . | . | . | . | . | . | . | . | . | . | . | . | . | . | . |
| 21-225_83C10_HC | . | . | . | . | . | . | . | . | . | . | . | . | . | . | . | . | . | . | . | . | . | . | . | . | . | . |
| 21-225_85G6_HC | . | . | . | . | . | . | . | . | . | . | . | . | . | . | . | . | . | . | . | . | . | . | . | . | . | . |
| 21-225_93E9_HC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | - | L | D | Y | W | G | Q | G | T | L | V | T | V | S | S |
| 21-225_147G8_HC | . | | . | . | . | P | | | | | | | | | | | | | |
| 21-225_192F8_HC | . | . | . | . | . | P | . | . | . | . | . | . | . | . | . | . | . | . | . |
| 21-225_193E6_HC | . | | . | . | . | . | . | . | . | . | . | . | . | . | . | . | . | . | . |
| 21-225_195B1_HC | . | | . | . | . | P | . | I | . | . | . | . | . | . | . | . | . | . | . |
| 21-225_200A1_HC | . | | . | . | . | P | . | . | . | . | . | . | . | . | . | . | . | . | . |
| 21-225_201B2_HC | . | | . | . | . | P | . | . | . | . | . | . | . | . | . | . | . | . | . |
| 21-225_201E1_HC | . | | . | . | . | . | . | . | . | . | . | . | A | . | . | . | . | . | . |
| 21-225_202D11_HC | . | | . | . | . | . | . | . | . | . | . | . | . | . | . | . | . | . | . |
| 21-225_202D9_HC | . | | . | . | . | . | . | . | . | . | . | . | . | . | . | . | . | . | . |
| 21-225_205G7_HC | . | | . | . | . | P | . | . | . | . | . | . | . | . | . | . | . | . | . |
| 21-225_208A9_HC | . | | . | . | . | . | . | . | . | . | . | . | . | . | . | . | . | . | . |
| 21-225_208B5_HC | . | | . | . | . | P | . | . | . | . | . | . | . | . | . | . | . | . | . |
| 21-225_74C7_HC | . | | . | . | . | . | . | . | . | . | . | . | . | . | . | . | . | . | . |
| 21-225_74D2_HC | . | | . | . | . | . | . | . | . | . | . | . | . | . | . | . | . | . | . |
| 21-225_75G3_HC | . | | . | . | . | . | . | . | . | . | . | . | . | . | . | . | . | . | . |
| 21-225_75G9_HC | . | | . | . | . | . | . | . | . | . | . | . | . | . | . | . | . | . | . |
| 21-225_76A7_HC | . | | . | . | . | . | . | . | . | . | . | . | A | . | . | . | . | . | . |
| 21-225_78E7_HC | . | | . | . | . | . | . | . | . | . | . | . | . | . | . | . | . | . | . |
| 21-225_80A6_HC | . | | . | . | . | . | . | . | . | . | . | . | . | . | . | . | . | . | . |
| 21-225_80B10_HC | . | | . | . | . | . | . | . | . | . | . | . | . | . | . | . | . | . | . |
| 21-225_83B6_HC | . | | . | . | . | . | . | . | . | . | . | . | . | . | . | . | . | . | . |
| 21-225_83C10_HC | . | | . | . | . | . | . | . | . | . | . | . | . | . | . | . | . | . | . |
| 21-225_85G6_HC | . | | . | . | . | . | . | . | . | . | . | . | . | . | . | . | . | . | . |
| 21-225_93E9_HC | . | | . | . | . | . | . | . | . | . | . | . | . | . | . | . | . | . | . |

FIGURE 57

Table 67.　　Consensus 33-VH4|4-39/D1|1-26|RF3/JH4 (SEQ ID NO: 50284):

QLQLQES-GPGLVKPSETLSLTCTVSG-GSIS<u>RS---SYYWG</u>WIRQPPGKGLEWIG<u>NIYY----
SGSTYYNPSLKS</u>RVTISVDTSKNQFSLKLSSVTAADTAVYYCAR<u>HSSSW</u>--------------------SLDYWGQGTLVTVSS wherein:

R in position 33 can be substituted with G

S in position 38 can be substituted with N

Y in position 39 can be substituted with S

N in position 57 can be substituted with S

S in position 67 can be substituted with Y, A, T or I

T in position 68 can be substituted with A, P or S

Y in position 69 can be substituted with Q, S, A or N

Y in position 70 can be substituted with C or H

N in position 71 can be substituted with I or T

H in position 109 can be substituted with L

S in position 111 can be substituted with T or G

L in position 136 can be substituted with F, I or V

Y in position 138 can be substituted with N, C, D or F

Xaa Ser Xaa Xaa Tyr Trp Gly (SEQ ID NO: 50046)

Xaa Ile Tyr Tyr Ser Gly Xaa Xaa Xaa Xaa Xaa Pro Ser Leu Lys Ser (SEQ ID NO: 50047)

Xaa Ser Xaa Ser Trp Ser Xaa Asp Xaa (SEQ ID NO: 50048)

Arg Ser Ser Tyr Tyr Trp Gly (SEQ ID NO: 50522)

**FIGURE 57**

Asn Ile Tyr Tyr Ser Gly Ser Thr Tyr Tyr Asn Pro Ser Leu Lys Ser (SEQ ID NO: 50523)

His Ser Ser Ser Trp Ser Leu Asp Tyr (SEQ ID NO: 50524)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | L | Q | L | Q | E | S | - | G | P | G | L | V | K | P | S | E | T | L | S | L | T | C | T | V |
| 21-225_11F10_HC | . | . | 8 | . | . | . | . | . | . | . | . | . | . | . | . | . | . | . | . | . | . | . | . | . | . |
| 21-225_15A1_HC | . | . | 8 | . | . | . | . | . | . | . | . | . | . | . | . | . | . | . | . | . | . | . | . | . | . |
| 21-225_15G7_HC | . | . | . | . | . | . | . | . | . | . | . | . | . | . | . | . | . | . | . | . | . | . | . | . | . |
| 21-225_15H1_HC | . | . | . | . | . | . | . | . | . | . | . | . | . | . | 8 | . | . | . | . | . | . | . | . | . | . |
| 21-225_16A1_HC | . | . | . | . | . | . | . | . | . | . | . | . | . | . | . | . | . | . | . | . | . | i | . | . | . |
| 21-225_18A3_HC | . | . | . | . | . | . | . | . | . | . | . | . | . | . | 8 | . | . | . | . | . | . | . | . | . | . |
| 21-225_18C6_HC | . | . | . | . | . | . | . | . | . | . | . | . | . | . | . | . | . | . | . | . | . | . | . | . | . |
| 21-225_20C2_HC | . | . | . | . | . | . | . | . | . | . | . | . | . | . | . | . | . | . | . | . | . | . | . | . | . |
| 21-225_20G9_HC | . | . | . | . | . | . | . | . | . | . | . | . | . | . | . | . | . | . | . | . | . | . | . | . | . |
| 21-225_21G7_HC | . | . | . | . | . | . | . | . | . | . | . | . | . | . | 8 | . | . | . | . | . | . | . | . | . | . |
| 21-225_22C1_HC | . | . | . | . | . | . | . | . | . | . | . | . | . | . | . | . | . | . | . | . | . | . | . | . | . |
| 21-225_22D12_HC | . | . | . | . | . | . | . | . | . | . | . | . | . | . | . | . | . | . | . | . | . | . | . | . | . |
| 21-225_22G9_HC | . | . | . | . | . | . | . | . | . | . | . | . | . | . | . | . | . | . | . | . | . | . | . | . | . |
| 21-225_23D1_HC | . | . | . | . | . | . | . | . | . | . | . | . | . | . | . | . | . | . | . | . | . | . | . | . | . |
| 21-225_24E5_HC | . | . | . | . | . | . | . | . | . | . | . | . | . | . | . | . | . | . | . | . | . | . | . | . | . |
| 21-225_34C4_HC | . | . | . | . | . | . | . | . | . | . | . | . | . | . | . | . | . | . | . | . | . | . | . | . | . |
| 21-225_59E6_HC | . | . | . | . | . | . | . | . | . | . | . | . | . | . | . | . | . | . | . | . | . | . | . | . | . |
| 21-225_68D12_HC | . | . | . | . | . | . | . | . | . | . | . | . | . | . | . | . | . | . | . | . | . | . | . | . | . |
| 21-225_6A6_HC | . | . | . | . | . | . | . | . | . | . | . | . | . | . | . | . | . | . | . | . | . | . | . | . | . |
| 21-225_6D4_HC | . | . | . | . | . | . | . | . | . | . | . | . | . | . | . | . | . | . | . | . | . | . | . | . | . |
| 21-225_7F4_HC | . | . | . | . | . | . | . | . | . | . | . | . | . | . | . | . | . | . | . | . | . | . | . | . | . |
| 21-225_8D12_HC | . | . | . | . | . | . | . | . | . | . | . | . | . | . | 8 | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | H_CDR1 | | | | | | | | | | | | | | | H_FR2 | | | |
| CONSENSUS | G | - | G | S | I | S | R | S | - | - | - | S | Y | Y | W | G | W | I | R | Q | P | P | G | K | G | L |
| 21-225_11F10_HC | . | . | A | . | . | . | . | . | . | . | . | . | . | . | . | . | . | . | . | . | . | . | . | . | . | . |
| 21-225_15A1_HC | . | . | . | . | . | . | . | . | . | . | . | . | . | . | . | . | . | . | . | . | . | . | . | . | . | . |
| 21-225_15G7_HC | . | . | . | . | . | . | . | . | . | . | . | . | S | . | . | . | . | . | . | . | . | . | . | . | . | . |
| 21-225_15H1_HC | . | . | . | . | . | . | . | . | . | . | . | . | . | . | . | . | . | . | . | . | . | . | . | . | . | . |
| 21-225_16A1_HC | . | . | . | . | . | D | . | . | . | . | . | . | . | . | . | . | . | . | . | . | . | . | . | . | . | . |
| 21-225_18A3_HC | . | . | . | . | . | . | . | . | . | . | . | . | . | . | . | . | . | . | . | . | . | . | . | . | . | . |
| 21-225_18C6_HC | . | . | . | . | . | . | . | . | . | . | . | . | . | . | . | . | . | . | . | . | . | . | . | . | . | . |
| 21-225_20C2_HC | . | . | . | . | . | . | . | . | . | . | . | . | . | . | . | . | . | . | . | . | . | . | . | . | . | . |
| 21-225_20G9_HC | . | . | . | . | . | . | . | . | . | . | . | . | . | . | . | . | . | . | . | . | . | . | . | . | . | . |
| 21-225_21G7_HC | . | . | . | . | . | . | . | . | . | . | . | . | . | . | . | . | . | . | . | . | . | . | . | . | . | Q |
| 21-225_22C1_HC | . | . | . | . | . | N | . | . | . | . | . | . | . | . | . | . | . | . | . | . | . | . | . | . | . | . |
| 21-225_22D12_HC | . | . | . | . | . | . | . | . | . | . | . | . | . | . | . | . | . | . | . | . | . | . | . | . | . | . |
| 21-225_22G9_HC | . | . | . | A | . | . | G | . | . | . | . | N | . | . | . | . | . | . | . | . | . | . | . | . | E | . |
| 21-225_23D1_HC | . | . | . | . | . | . | . | . | . | . | . | . | . | . | . | . | . | . | . | . | . | . | . | . | . | . |
| 21-225_24E5_HC | . | . | . | A | . | . | . | . | . | . | . | . | . | . | . | . | . | . | . | . | . | . | . | . | . | . |
| 21-225_34C4_HC | . | . | . | . | . | . | . | . | . | . | . | . | . | . | . | . | . | . | . | . | S | . | . | . | . | . |
| 21-225_59E6_HC | . | . | . | . | . | . | . | . | . | . | . | . | . | . | . | . | . | . | . | . | . | . | . | . | . | . |
| 21-225_68D12_HC | . | . | . | . | . | . | . | . | . | . | . | . | . | . | . | . | . | . | . | . | . | . | . | . | . | . |
| 21-225_6A6_HC | . | . | . | . | . | . | . | . | . | . | . | . | . | . | . | . | . | . | . | . | . | . | . | . | . | . |
| 21-225_6D4_HC | . | . | . | . | . | . | . | . | . | . | . | . | S | . | . | . | . | . | . | . | . | . | . | . | . | . |
| 21-225_7F4_HC | . | . | . | . | . | . | . | . | . | . | . | . | . | . | . | . | . | . | . | . | . | . | . | . | . | . |
| 21-225_8D12_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | N | I | Y | Y | - | - | - | - | S | G | S | T | Y | Y | N | P | S | L | K | S | R | V |
| 21-225_11F10_HC | . | . | . | . | S | . | . | . | . | . | . | . | . | . | . | A | Q | . | . | . | . | . | . | . | . | . |
| 21-225_15A1_HC | . | . | . | . | S | . | . | . | . | . | . | . | . | . | . | A | Q | . | . | . | . | . | . | . | . | . |
| 21-225_15G7_HC | . | . | . | . | S | . | . | . | . | . | . | . | . | . | Y | . | . | . | T | . | . | . | . | . | . | . |
| 21-225_15H1_HC | . | . | . | . | . | . | . | . | . | . | . | . | . | . | Y | P | . | . | . | . | . | . | . | . | . | . |
| 21-225_16A1_HC | . | . | . | . | S | . | . | . | . | . | . | . | . | . | . | A | . | R | . | . | . | . | . | . | . | G |
| 21-225_18A3_HC | . | . | . | . | . | . | . | . | . | . | . | . | . | . | Y | . | . | . | T | . | . | . | . | . | . | . |
| 21-225_18C6_HC | . | . | . | . | . | . | . | . | . | . | . | . | . | . | Y | . | . | . | . | . | . | . | . | . | . | . |
| 21-225_20C2_HC | . | . | . | . | . | . | . | . | . | . | . | . | . | . | T | A | . | C | . | S | . | . | . | . | . | . |
| 21-225_20G9_HC | . | . | . | . | S | . | . | . | . | . | . | . | . | . | . | A | S | . | . | . | . | . | . | . | . | . |
| 21-225_21G7_HC | . | . | . | . | . | . | . | . | . | . | . | . | . | . | Y | S | . | . | . | . | . | . | . | . | . | . |
| 21-225_22C1_HC | . | . | . | . | . | . | . | . | . | . | . | . | . | . | A | . | . | . | . | S | . | . | . | . | . | . |
| 21-225_22D12_HC | . | . | . | . | . | . | . | . | . | . | . | . | . | . | T | . | S | . | . | . | . | . | . | . | . | . |
| 21-225_22G9_HC | . | . | . | . | . | . | . | . | . | . | . | . | . | . | . | . | . | . | . | . | . | . | . | . | . | . |
| 21-225_23D1_HC | . | . | . | . | S | . | . | . | . | . | . | . | . | . | . | A | Q | . | . | . | . | . | . | . | . | . |
| 21-225_24E5_HC | . | . | . | . | S | . | . | . | . | . | . | . | . | . | . | A | N | . | . | . | . | . | . | . | . | . |
| 21-225_34C4_HC | . | . | . | . | S | . | . | . | . | . | . | . | . | . | . | A | . | . | . | . | . | . | . | . | . | . |
| 21-225_59E6_HC | . | . | . | . | . | . | . | . | . | . | . | . | . | . | . | . | . | . | . | . | . | . | . | . | . | . |
| 21-225_68D12_HC | . | . | . | . | . | . | . | . | . | . | . | . | . | . | . | . | . | . | I | . | . | . | . | . | . | . |
| 21-225_6A6_HC | . | . | . | . | . | . | . | . | . | . | . | . | . | . | I | P | . | . | . | . | . | . | . | . | . | . |
| 21-225_6D4_HC | . | . | . | . | . | . | . | . | . | . | . | . | . | . | . | A | . | . | I | . | . | . | . | . | . | . |
| 21-225_7F4_HC | . | . | . | . | . | . | . | . | . | . | . | . | . | . | A | . | . | . | . | . | . | . | . | . | . | . |
| 21-225_8D12_HC | . | . | . | . | . | . | . | . | . | . | . | . | . | . | Y | P | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | V | D | T | S | K | N | Q | F | S | L | K | L | S | S | V | T | A | A | D | T | A | V | Y |
| 21-225_11F10_HC | . | . | . | . | . | . | . | . | . | . | . | . | . | T | . | . | . | . | . | . | . | . | . | . | . | . |
| 21-225_15A1_HC | . | . | . | . | . | . | T | . | . | . | . | . | . | T | . | . | . | . | . | . | . | . | . | . | . | . |
| 21-225_15G7_HC | . | . | . | . | . | . | . | . | . | . | . | . | . | R | . | . | . | . | . | . | . | . | . | . | S | . |
| 21-225_15H1_HC | . | . | . | . | . | . | . | . | . | . | . | . | . | . | . | . | . | . | . | . | . | . | . | . | . | . |
| 21-225_16A1_HC | . | . | . | . | . | . | . | . | . | . | . | . | . | . | . | . | . | . | . | . | . | . | . | . | A | . |
| 21-225_18A3_HC | . | . | . | . | . | . | . | . | . | . | . | . | . | . | . | . | . | . | . | . | . | . | . | . | . | . |
| 21-225_18C6_HC | S | . | . | . | . | . | . | . | . | . | . | . | . | . | . | . | . | . | . | . | . | . | . | . | L | . |
| 21-225_20C2_HC | S | . | . | . | . | . | . | . | . | . | . | . | . | . | . | . | . | . | . | . | T | . | . | . | . | . |
| 21-225_20G9_HC | . | . | . | . | . | . | . | # | . | . | . | . | . | . | . | . | . | . | . | . | . | . | . | . | A | . |
| 21-225_21G7_HC | . | . | . | . | . | . | . | . | . | . | . | . | . | . | . | . | . | . | . | . | . | . | . | . | . | . |
| 21-225_22C1_HC | . | . | . | . | . | . | . | T | . | . | . | . | . | . | . | . | . | . | . | . | . | . | . | . | . | . |
| 21-225_22D12_HC | . | . | . | . | . | . | . | # | . | . | . | . | . | . | . | . | . | . | . | . | . | . | . | . | . | F |
| 21-225_22G9_HC | . | . | . | . | . | . | . | . | . | . | . | . | . | . | . | # | . | . | . | # | . | . | . | . | . | . |
| 21-225_23D1_HC | . | . | . | . | . | . | . | . | . | . | . | . | . | T | . | . | . | . | . | . | . | . | . | . | L | . |
| 21-225_24E5_HC | . | . | . | . | . | . | . | . | . | . | . | . | . | . | . | . | . | . | . | . | . | . | . | . | . | . |
| 21-225_34C4_HC | . | . | . | . | . | . | . | . | . | . | V | . | . | . | . | . | . | . | . | . | . | . | . | . | . | . |
| 21-225_59E6_HC | . | . | . | . | . | . | . | . | . | . | . | . | . | . | . | . | . | . | . | . | . | . | . | . | L | . |
| 21-225_68D12_HC | . | . | . | . | . | . | . | . | . | . | . | . | . | . | . | T | . | . | . | . | . | . | . | . | . | . |
| 21-225_6A6_HC | N | . | . | . | . | . | . | . | . | . | . | . | . | . | . | N | . | . | . | . | . | . | . | . | . | . |
| 21-225_6D4_HC | . | . | . | . | A | . | . | . | . | . | . | . | . | N | . | T | . | . | . | . | . | . | . | . | . | . |
| 21-225_7F4_HC | . | . | . | . | . | . | . | T | . | . | . | . | . | . | . | . | . | . | . | . | . | . | . | . | . | . |
| 21-225_8D12_HC | . | . | . | S | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | H | S | S | S | W | - | - | - | - | - | - | - | - | - | - | - | - | - | - | - | - | - |
| 21-225_11F10_HC | . | . | . | . | L | . | . | . | . | . | . | . | . | . | . | . | . | . | . | . | . | . | . | . | . | . |
| 21-225_15A1_HC | . | . | . | . | L | . | . | . | . | . | . | . | . | . | . | . | . | . | . | . | . | . | . | . | . | . |
| 21-225_15G7_HC | . | . | . | . | L | . | . | . | . | . | . | . | . | . | . | . | . | . | . | . | . | . | . | . | . | . |
| 21-225_15H1_HC | . | . | . | . | . | . | T | . | . | . | . | . | . | . | . | . | . | . | . | . | . | . | . | . | . | . |
| 21-225_16A1_HC | . | . | . | . | L | . | . | . | . | . | . | . | . | . | . | . | . | . | . | . | . | . | . | . | . | . |
| 21-225_18A3_HC | . | . | . | . | . | . | T | . | . | . | . | . | . | . | . | . | . | . | . | . | . | . | . | . | . | . |
| 21-225_18C6_HC | . | . | . | . | . | . | . | . | . | . | . | . | . | . | . | . | . | . | . | . | . | . | . | . | . | . |
| 21-225_20C2_HC | . | . | . | . | . | . | . | . | . | . | . | . | . | . | . | . | . | . | . | . | . | . | . | . | . | . |
| 21-225_20G9_HC | . | . | . | . | L | . | . | . | . | . | . | . | . | . | . | . | . | . | . | . | . | . | . | . | . | . |
| 21-225_21G7_HC | . | . | . | . | . | . | T | . | . | . | . | . | . | . | . | . | . | . | . | . | . | . | . | . | . | . |
| 21-225_22C1_HC | . | . | . | . | . | . | G | . | . | . | . | . | . | . | . | . | . | . | . | . | . | . | . | . | . | . |
| 21-225_22D12_HC | . | . | . | . | L | . | . | . | . | . | . | . | . | . | . | . | . | . | . | . | . | . | . | . | . | . |
| 21-225_22G9_HC | . | . | . | . | . | . | . | . | . | . | . | . | . | . | . | . | . | . | . | . | . | . | . | . | . | . |
| 21-225_23D1_HC | . | . | . | . | L | . | . | . | . | . | . | . | . | . | . | . | . | . | . | . | . | . | . | . | . | . |
| 21-225_24E5_HC | . | . | . | . | L | . | . | . | . | . | . | . | . | . | . | . | . | . | . | . | . | . | . | . | . | . |
| 21-225_34C4_HC | . | . | . | . | L | . | . | . | . | . | . | . | . | . | . | . | . | . | . | . | . | . | . | . | . | . |
| 21-225_59E6_HC | . | . | . | . | . | . | . | . | . | . | . | . | . | . | . | . | . | . | . | . | . | . | . | . | . | . |
| 21-225_68D12_HC | . | . | . | . | . | . | T | . | . | . | . | . | . | . | . | . | . | . | . | . | . | . | . | . | . | . |
| 21-225_6A6_HC | . | . | . | . | . | . | . | . | . | . | . | . | . | . | . | . | . | . | . | . | . | . | . | . | . | . |
| 21-225_6D4_HC | . | . | . | . | . | . | . | . | . | . | . | . | . | . | . | . | . | . | . | . | . | . | . | . | . | . |
| 21-225_7F4_HC | . | . | . | . | . | . | G | . | . | . | . | . | . | . | . | . | . | . | . | . | . | . | . | . | . | . |
| 21-225_8D12_HC | . | . | . | . | . | . | T | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | S | L | D | Y | W | G | Q | G | T | L | V | T | V | S | S |
| 21-225_11F10_HC | . | . | . | . | . | F | . | . | . | . | . | . | . | . | . | . | . | . | . |
| 21-225_15A1_HC | . | . | . | . | . | F | . | . | . | . | . | . | . | . | . | . | . | . | . |
| 21-225_15G7_HC | . | . | . | . | . | F | . | S | . | . | . | . | . | . | . | . | . | . | . |
| 21-225_15H1_HC | . | . | . | . | . | . | . | . | . | . | . | . | . | . | . | . | . | . | . |
| 21-225_16A1_HC | . | . | . | . | . | F | . | . | . | . | . | . | . | . | . | . | . | . | . |
| 21-225_18A3_HC | . | . | . | . | . | . | . | . | . | . | . | . | . | . | . | . | . | . | . |
| 21-225_18C6_HC | . | . | . | . | . | . | . | Q | . | . | . | . | . | . | . | . | . | . | . |
| 21-225_20C2_HC | . | . | . | . | . | . | . | N | . | . | . | . | . | . | . | . | . | . | . |
| 21-225_20G9_HC | . | . | . | . | . | F | . | . | . | . | . | . | . | . | . | . | . | . | . |
| 21-225_21G7_HC | . | . | . | . | . | . | . | . | . | . | . | . | . | . | . | . | . | . | . |
| 21-225_22C1_HC | . | . | . | . | . | . | . | . | . | . | . | . | . | . | . | . | . | . | . |
| 21-225_22D12_HC | . | . | . | . | . | V | . | . | . | . | . | . | . | . | . | . | . | . | . |
| 21-225_22G9_HC | . | . | . | . | . | . | . | . | . | . | . | . | . | . | . | . | . | . | . |
| 21-225_23D1_HC | . | . | . | . | . | F | . | . | . | . | . | . | . | . | . | . | . | . | . |
| 21-225_24E5_HC | . | . | . | . | . | I | . | N | . | . | . | . | . | . | . | . | . | . | . |
| 21-225_34C4_HC | . | . | . | . | . | F | . | . | . | . | . | . | . | . | . | . | . | . | . |
| 21-225_59E6_HC | . | . | . | . | . | . | . | . | . | . | . | . | . | . | . | . | . | . | . |
| 21-225_68D12_HC | . | . | . | . | . | I | . | . | . | . | . | . | . | . | . | . | . | . | . |
| 21-225_6A6_HC | . | . | . | . | . | . | . | . | . | . | . | . | . | . | . | . | . | . | . |
| 21-225_6D4_HC | . | . | . | . | . | . | . | Q | . | . | . | . | . | . | . | . | . | . | . |
| 21-225_7F4_HC | . | . | . | . | . | . | . | . | . | . | . | . | . | . | . | . | . | . | . |
| 21-225_8D12_HC | . | . | . | . | . | . | . | . | . | . | . | . | . | . | . | . | . | . | . |

**FIGURE 57**

Table 68.    Consensus 34-VH1|1-02/D5|5-18|RF3/JH5 (SEQ ID NO: 50285):

QVQLVQS-GAEVKKPGASVKVSCKASG-YTFTG-----YYIHWVRQAPGQGLEWMGWINPN---
SGGTNYAQKFQGRVTMTRDTSISTAYMELSRLRSDDTAVYYCARGYSYGY------------------NWFDPWGQGTLVTVSS wherein:

G in position 33 can be substituted with D

Y in position 39 can be substituted with H

Y in position 40 can be substituted with F

I in position 41 can be substituted with M

H in position 42 can be substituted with N

P in position 60 can be substituted with S

N in position 61 can be substituted with K

S in position 65 can be substituted with N

G in position 66 can be substituted with D

G in position 67 can be substituted with D

A in position 71 can be substituted with E

Q in position 72 can be substituted with E

G in position 76 can be substituted with D

G in position 109 can be substituted with D

Y in position 110 can be substituted with G, T or F

S in position 111 can be substituted with Y, D or R

Y in position 112 can be substituted with S

FIGURE 57

G in position 113 can be substituted with null (-) or S

Y in position 114 can be substituted with S or null (-)

null (-) in position 115 can be substituted with G

null (-) in position 116 can be substituted with S

null (-) in position 132 can be substituted with Y

null (-) in position 133 can be substituted with Y, F or H

N in position 134 can be substituted with null (-) or D

W in position 135 can be substituted with null (-), D or E

F in position 136 can be substituted with L

D in position 137 can be substituted with A

P in position 138 can be substituted with S

Xaa Xaa Xaa Xaa Xaa (SEQ ID NO: 50049)

Trp Ile Asn Xaa Xaa Xaa Xaa Xaa Thr Asn Tyr Xaa Xaa Lys Phe Gln Xaa (SEQ ID NO: 50050)

Xaa Xaa Xaa Xaa Xaa Xaa Xaa Xaa Xaa Xaa Xaa Xaa Xaa Xaa Xaa (SEQ ID NO: 50051)

Gly Tyr Tyr Ile His (SEQ ID NO: 50525)

Trp Ile Asn Pro Asn Ser Gly Gly Thr Asn Tyr Ala Gln Lys Phe Gln Gly (SEQ ID NO: 50526)

Gly Tyr Ser Tyr Gly Tyr Asn Trp Phe Asp Pro (SEQ ID NO: 50527)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | Q | S | - | G | A | E | V | K | K | P | G | A | S | V | K | V | S | C | K | A |
| 21-225_17G4_HC | . | . | . | . | . | . | . | . | . | . | . | . | . | . | . | . | . | . | . | . | . | . | . | . | T |
| 21-225_19A1_HC | . | . | . | . | . | . | . | . | . | . | . | . | . | . | . | . | . | . | . | . | . | . | . | . | . |
| 21-225_201H10_HC | . | . | . | . | . | . | . | . | . | . | . | . | . | . | . | . | . | . | . | . | . | . | . | . | . |
| 21-225_203E3_HC | . | . | . | . | . | . | . | . | . | . | . | . | . | . | . | . | . | . | . | . | . | . | . | . | . |
| 21-225_204H4_HC | . | . | . | . | . | . | . | . | . | . | . | . | . | . | . | . | . | . | . | . | . | . | . | . | . |
| 21-225_206A4_HC | . | . | . | . | . | . | . | . | . | . | . | . | . | . | . | . | . | . | . | . | . | . | . | . | . |
| 21-225_206A5_HC | . | . | . | . | . | . | . | . | . | . | . | . | . | . | . | . | . | . | . | . | . | . | . | . | . |
| 21-225_208B11_HC | . | . | . | . | . | . | . | . | . | . | . | . | . | . | . | . | . | . | . | . | . | . | . | . | . |
| 21-225_208E8_HC | . | . | . | . | . | . | . | . | . | . | . | . | . | . | . | . | . | . | . | . | . | . | . | . | . |
| 21-225_210B12_HC | . | . | . | . | . | . | . | . | . | . | . | . | . | . | . | . | . | . | . | . | ? | . | . | . | . |
| 21-225_210D11_HC | . | . | . | . | . | . | . | . | . | . | . | . | . | . | . | . | . | . | . | . | . | . | . | . | . |
| 21-225_213A7_HC | . | . | . | . | . | . | . | . | . | . | . | . | . | . | . | . | . | . | . | . | ? | . | . | ? | . |
| 21-225_214H8_HC | . | . | . | . | . | . | . | . | . | . | . | . | . | . | . | . | . | . | . | . | . | . | . | . | . |
| 21-225_215H6_HC | . | . | . | . | . | . | . | . | . | . | . | . | . | . | . | . | . | . | . | . | . | . | . | . | . |
| 21-225_226A10_HC | . | . | . | . | . | . | . | . | . | . | . | . | . | . | . | . | . | . | . | . | . | . | . | . | . |
| 21-225_226B2_HC | . | . | . | . | . | . | . | . | . | . | . | . | . | . | . | . | . | . | . | . | . | . | . | . | . |
| 21-225_226B7_HC | . | . | . | . | . | . | . | . | . | . | . | . | . | . | . | . | . | . | . | . | . | . | . | . | . |
| 21-225_32B11_HC | . | . | . | . | . | . | . | . | . | . | . | . | . | . | . | . | . | . | . | . | . | . | . | . | . |
| 21-225_34D7_HC | . | . | . | . | . | . | . | . | . | . | . | . | . | . | . | . | . | . | . | . | . | . | . | . | . |

**FIGURE 57**

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | H_CDR1 | | | | | | | | | | | | | H_FR2 | |
| CONSENSUS | S | G | - | Y | T | F | T | G | - | - | - | - | - | Y | Y | I | H | W | V | R | Q | A | P | G | Q |
| 21-225_17G4_HC | . | . | . | . | . | . | . | D | . | . | . | . | . | . | . | M | . | . | . | . | . | . | . | . | . |
| 21-225_19A1_HC | . | . | . | . | . | . | . | . | . | . | . | . | . | . | . | M | . | . | . | . | . | . | . | . | . |
| 21-225_201H10_HC | . | . | . | . | . | . | . | . | . | . | . | . | . | . | . | . | . | . | . | . | . | . | . | . | . |
| 21-225_203E3_HC | . | . | . | . | . | . | . | . | . | . | . | . | . | . | . | . | . | . | . | . | . | . | . | . | . |
| 21-225_204H4_HC | . | . | . | . | . | . | . | . | . | . | . | . | . | . | . | . | . | . | . | . | . | . | . | . | . |
| 21-225_206A4_HC | . | . | . | . | . | . | . | . | . | . | . | . | . | . | . | . | . | . | . | . | . | . | . | . | . |
| 21-225_206A5_HC | . | . | . | . | . | . | . | . | . | . | . | . | . | . | . | . | . | . | . | . | . | . | . | . | . |
| 21-225_208B11_HC | . | . | . | . | . | . | . | . | . | . | . | . | . | . | . | . | . | . | . | . | . | . | . | . | . |
| 21-225_208E8_HC | . | . | . | . | . | . | . | . | . | . | . | . | . | . | . | . | . | . | . | . | . | . | . | . | . |
| 21-225_210B12_HC | . | . | . | F | . | . | . | D | . | . | . | . | . | . | . | M | N | . | . | . | . | . | . | . | . |
| 21-225_210D11_HC | . | . | . | . | . | . | . | . | . | . | . | . | . | . | . | . | . | . | . | . | . | . | . | . | . |
| 21-225_213A7_HC | . | . | . | F | . | . | . | D | . | . | . | . | . | . | . | M | N | . | . | . | . | . | . | . | . |
| 21-225_214H8_HC | . | . | . | . | . | . | . | . | . | . | . | . | . | H | . | . | . | . | . | . | . | . | . | . | . |
| 21-225_215H6_HC | . | . | . | . | . | . | . | . | . | . | . | . | . | H | . | . | . | . | . | . | . | . | . | . | . |
| 21-225_226A10_HC | . | . | . | . | . | . | . | . | . | . | . | . | . | . | F | M | . | . | . | . | . | . | . | . | . |
| 21-225_226B2_HC | . | . | . | . | . | . | . | . | . | . | . | . | . | . | F | M | . | . | . | . | . | . | . | . | . |
| 21-225_226B7_HC | . | . | . | . | . | . | . | . | . | . | . | . | . | . | . | M | . | . | . | . | . | . | . | . | . |
| 21-225_32B11_HC | . | . | . | . | . | . | . | . | . | . | . | . | . | . | . | M | . | . | . | . | . | . | . | . | . |
| 21-225_34D7_HC | . | . | . | . | . | . | . | . | . | . | . | . | . | . | . | M | . | . | . | . | . | . | . | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | M | G | W | I | N | P | N | - | - | - | S | G | G | T | N | Y | A | Q | K | F | Q |
| 21-225_17G4_HC | . | . | . | . | . | . | . | . | . | . | . | . | . | . | . | . | . | . | . | . | E | . | . | . | . |
| 21-225_19A1_HC | . | . | . | . | . | . | . | . | . | . | . | . | . | . | . | . | . | . | . | . | . | . | . | . | . |
| 21-225_201H10_HC | . | . | . | . | . | A | . | . | . | . | . | . | . | . | . | . | . | . | . | . | . | . | . | . | . |
| 21-225_203E3_HC | . | . | . | . | . | A | . | . | . | . | . | . | . | . | . | . | . | . | . | . | . | . | . | . | . |
| 21-225_204H4_HC | . | . | . | . | . | A | . | . | . | . | . | . | . | . | . | . | . | . | . | . | . | . | . | . | . |
| 21-225_206A4_HC | . | . | . | . | . | A | . | . | . | . | . | . | . | . | . | . | . | . | . | . | . | . | . | . | . |
| 21-225_206A5_HC | . | . | . | . | . | A | . | . | . | . | . | . | . | . | . | . | . | . | . | . | . | . | . | . | . |
| 21-225_208B11_HC | . | . | . | . | . | A | . | . | . | . | . | . | . | . | . | . | . | . | . | . | . | . | . | . | . |
| 21-225_208E8_HC | . | . | . | . | . | A | . | . | . | . | . | . | . | . | . | . | . | . | . | . | . | . | . | . | . |
| 21-225_210B12_HC | . | . | . | . | . | . | . | . | . | . | . | . | . | . | . | . | . | . | . | . | . | . | . | . | . |
| 21-225_210D11_HC | . | . | . | . | . | A | . | . | . | . | . | . | . | . | . | . | . | . | . | . | . | . | . | . | . |
| 21-225_213A7_HC | . | . | . | . | . | . | . | . | . | . | K | . | . | . | N | . | . | . | . | . | . | . | . | . | . |
| 21-225_214H8_HC | . | . | . | . | . | . | . | . | S | . | . | . | . | . | . | . | . | . | . | . | . | . | . | . | . |
| 21-225_215H6_HC | . | . | . | . | . | . | . | . | S | . | . | . | . | . | . | . | . | . | . | . | . | E | . | . | . |
| 21-225_226A10_HC | . | . | . | . | . | . | . | . | . | . | . | . | . | . | . | . | D | . | . | . | . | . | . | . | . |
| 21-225_226B2_HC | . | . | . | . | . | . | . | . | . | . | . | . | . | . | . | . | D | . | . | . | . | . | . | . | . |
| 21-225_226B7_HC | . | . | . | . | . | . | . | . | . | . | . | . | . | . | . | . | . | . | . | . | . | . | . | . | . |
| 21-225_32B11_HC | . | . | . | . | . | . | . | . | . | . | . | . | . | . | . | D | . | . | . | . | . | . | . | . | . |
| 21-225_34D7_HC | . | . | . | . | . | . | . | . | . | . | . | . | . | . | . | . | . | . | . | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | V | T | M | T | R | D | T | S | I | S | T | A | Y | M | E | L | S | R | L | R | S | D | D |
| 21-225_17G4_HC | . | . | . | . | . | . | . | . | . | . | . | . | . | . | . | . | . | . | . | . | . | . | . | . | . |
| 21-225_19A1_HC | . | . | . | . | . | . | . | . | . | . | . | . | . | . | . | . | . | . | . | . | . | . | . | . | . |
| 21-225_201H10_HC | . | . | . | . | . | . | . | . | . | . | . | Y | . | Y | . | . | . | . | . | . | . | . | . | . | . |
| 21-225_203E3_HC | . | . | . | . | . | . | . | . | . | . | . | Y | . | . | . | . | . | . | . | . | . | . | . | . | . |
| 21-225_204H4_HC | . | . | . | . | . | . | . | . | . | . | . | Y | . | . | . | . | . | . | . | . | . | . | . | . | . |
| 21-225_206A4_HC | . | . | . | . | . | . | . | . | . | . | . | Y | . | . | . | . | . | . | . | . | . | . | . | . | . |
| 21-225_206A5_HC | . | . | . | . | . | . | . | . | . | . | . | Y | . | . | . | . | . | . | . | . | . | . | . | . | . |
| 21-225_208B11_HC | . | . | . | . | . | . | . | . | . | . | . | Y | . | . | . | . | . | . | . | . | . | . | . | . | . |
| 21-225_208E8_HC | . | . | . | . | . | . | . | . | . | . | . | Y | . | . | . | . | . | . | . | . | . | . | . | . | . |
| 21-225_210B12_HC | . | . | . | . | . | . | . | . | . | . | . | . | . | . | . | . | . | . | . | . | . | . | . | . | . |
| 21-225_210D11_HC | . | . | . | . | . | . | . | . | . | . | . | Y | . | . | . | . | . | . | . | . | . | . | . | . | . |
| 21-225_213A7_HC | . | . | . | . | . | . | . | . | . | . | L | . | R | . | . | . | . | . | . | . | . | . | . | . | . |
| 21-225_214H8_HC | . | . | . | . | . | . | . | . | . | . | . | . | . | . | . | . | . | . | . | . | . | . | . | . | . |
| 21-225_215H6_HC | . | . | . | . | . | . | . | . | . | . | . | . | . | . | . | . | . | . | . | . | . | . | . | . | . |
| 21-225_226A10_HC | . | . | . | . | . | . | . | . | . | . | L | . | . | . | . | . | . | . | . | . | . | . | . | . | . |
| 21-225_226B2_HC | . | . | . | . | . | . | . | . | . | . | L | N | . | . | . | . | . | . | . | . | . | . | . | . | . |
| 21-225_226B7_HC | G | . | . | . | . | . | . | . | . | . | . | . | . | . | R | . | . | . | . | . | . | . | . | . | . |
| 21-225_32B11_HC | . | . | . | . | . | . | . | . | . | . | . | . | . | . | . | . | . | . | N | . | . | . | . | . | . |
| 21-225_34D7_HC | . | . | . | . | . | . | . | . | . | . | . | . | . | . | . | . | . | . | . | . | . | . | . | . | . |

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | G | Y | S | Y | G | Y | - | - | - | - | - | - | - | - | - | - | - |
| 21-225_17G4_HC | . | . | . | . | . | . | . | S | . | . | . | . | . | . | . | . | . | . | . | . | . | . | . | . | . |
| 21-225_19A1_HC | . | . | . | . | . | . | . | S | . | . | . | . | . | . | . | . | . | . | . | . | . | . | . | . | . |
| 21-225_201H10_HC | . | . | . | . | . | . | . | . | . | . | . | . | . | . | . | . | . | . | . | . | . | . | . | . | . |
| 21-225_203E3_HC | . | . | . | . | . | . | . | . | . | . | . | . | . | . | . | . | . | . | . | . | . | . | . | . | . |
| 21-225_204H4_HC | . | . | . | . | . | . | . | . | . | . | . | . | . | . | . | . | . | . | . | . | . | . | . | . | . |
| 21-225_206A4_HC | . | . | . | . | . | . | . | . | . | . | . | . | . | . | . | . | . | . | . | . | . | . | . | . | . |
| 21-225_206A5_HC | . | . | . | . | . | . | . | . | . | . | . | . | . | . | . | . | . | . | . | . | . | . | . | . | . |
| 21-225_208B11_HC | . | . | . | . | . | . | . | . | . | . | . | . | . | . | . | . | . | . | . | . | . | . | . | . | . |
| 21-225_208E8_HC | . | . | . | . | . | . | . | . | . | . | . | . | . | . | . | . | . | . | . | . | . | . | . | . | . |
| 21-225_210B12_HC | . | . | . | . | . | . | . | . | . | . | D | S | - | - | . | . | . | . | . | . | . | . | . | . | . |
| 21-225_210D11_HC | . | . | . | . | . | . | . | . | . | . | . | . | . | . | . | . | . | . | . | . | . | . | . | . | . |
| 21-225_213A7_HC | . | . | . | . | . | . | . | R | . | . | D | S | - | - | . | . | . | . | . | . | . | . | . | . | . |
| 21-225_214H8_HC | . | . | . | . | . | . | . | R | D | S | R | . | S | . | G | . | . | . | . | . | . | . | . | . | . |
| 21-225_215H6_HC | . | . | . | . | . | . | . | R | D | S | R | . | S | . | G | . | . | . | . | . | . | . | . | . | . |
| 21-225_226A10_HC | . | . | I | . | . | . | . | . | . | T | Y | . | . | S | G | S | . | . | . | . | . | . | . | . | . |
| 21-225_226B2_HC | . | . | I | . | . | . | . | . | . | T | Y | . | . | S | G | S | . | . | . | . | . | . | . | . | . |
| 21-225_226B7_HC | . | . | . | . | . | . | . | . | . | . | Y | . | . | S | G | S | . | . | . | . | . | . | . | . | . |
| 21-225_32B11_HC | . | . | . | . | . | . | . | . | . | F | Y | . | . | S | G | S | . | . | . | . | . | . | . | . | . |
| 21-225_34D7_HC | . | . | . | . | . | . | . | . | . | . | Y | . | . | S | G | S | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | H_FR4 | | | | | | |
| CONSENSUS | - | - | - | - | - | - | - | - | N | W | F | D | P | W | G | Q | G | T | L | V | T | V | S | S |
| 21-225_17G4_HC | . | . | . | . | . | . | . | . | . | . | . | . | . | . | . | . | . | . | . | . | . | . | . | . |
| 21-225_19A1_HC | . | . | . | . | . | . | . | . | . | . | . | . | . | . | . | . | . | . | . | . | . | . | . | . |
| 21-225_201H10_HC | . | . | . | . | . | . | . | . | . | . | . | . | . | . | . | . | . | . | . | . | . | . | . | . |
| 21-225_203E3_HC | . | . | . | . | . | . | . | . | . | . | . | . | . | . | . | . | . | . | . | . | . | . | . | . |
| 21-225_204H4_HC | . | . | . | . | . | . | . | . | . | . | . | . | . | . | . | . | . | . | . | . | . | . | . | . |
| 21-225_206A4_HC | . | . | . | . | . | . | . | . | . | . | . | . | . | . | . | . | . | . | . | . | . | . | . | . |
| 21-225_206A5_HC | . | . | . | . | . | . | . | . | . | . | . | . | . | . | . | . | . | . | . | . | . | . | . | . |
| 21-225_208B11_HC | . | . | . | . | . | . | . | . | . | . | . | . | . | . | . | . | . | . | . | . | . | . | . | . |
| 21-225_208E8_HC | . | . | . | . | . | . | . | . | . | . | . | . | . | . | . | . | . | . | . | . | . | . | . | . |
| 21-225_210B12_HC | . | . | . | . | . | . | . | . | - | - | . | A | . | . | . | . | . | . | . | . | . | . | . | . |
| 21-225_210D11_HC | . | . | . | . | . | . | . | . | . | . | . | . | . | . | . | . | . | . | . | . | . | . | . | . |
| 21-225_213A7_HC | . | . | . | . | . | . | . | . | - | - | . | A | . | . | . | . | . | . | . | . | . | . | . | . |
| 21-225_214H8_HC | . | . | . | . | . | . | Y | . | D | . | . | . | . | . | . | . | . | . | . | . | . | . | . | . |
| 21-225_215H6_HC | . | . | . | . | . | . | . | . | D | . | . | . | . | . | . | . | . | . | . | . | . | . | . | . |
| 21-225_226A10_HC | . | . | . | . | . | . | Y | F | . | E | L | . | S | . | . | . | . | . | . | . | . | . | . | . |
| 21-225_226B2_HC | . | . | . | . | . | . | Y | F | . | E | L | . | S | . | . | . | . | . | . | . | . | . | . | . |
| 21-225_226B7_HC | . | . | . | . | . | . | Y | Y | . | . | . | . | S | . | . | . | . | . | . | . | . | . | . | . |
| 21-225_32B11_HC | . | . | . | . | . | . | Y | Y | . | D | L | . | . | . | . | . | . | . | . | . | . | . | . | . |
| 21-225_34D7_HC | . | . | . | . | . | . | Y | Y | . | D | L | . | . | . | . | . | . | . | . | . | . | . | . | . |

FIGURE 57

Table 69.    Consensus 35- VH3|3-33/D1|1-1|RF3/JH6 (SEQ ID NO: 50286):

QVQLVES-GGGVVQPGRSLRLSCAASG-FTFSH-----YGMHWVRQAPGKGLEWVAVIWYD---
GSNKYYADSVKGRFTISRDNSKNTLYLQMNSLRAEDTAVYYCARGDWNP------------------EGMDVWGQGTTVTVSS wherein:

H in position 33 can be substituted with N

Y in position 39 can be substituted with S

V in position 57 can be substituted with I

W in position 59 can be substituted with Y

N in position 67 can be substituted with Y

K in position 68 can be substituted with E

Y in position 70 can be substituted with C or N

G in position 109 can be substituted with D

D in position 110 can be substituted with R

W in position 111 can be substituted with H

N in position 112 can be substituted with Y

P in position 113 can be substituted with D

null (-) in position 114 can be substituted with F

null (-) in position 115 can be substituted with H

null (-) in position 116 can be substituted with V

null (-) in position 117 can be substituted with P

FIGURE 57

null (-) in position 130 can be substituted with Y

null (-) in position 131 can be substituted with Y

null (-) in position 132 can be substituted with Y

null (-) in position 133 can be substituted with Y

E in position 134 can be substituted with Y

M in position 136 can be substituted with L

Xaa Xaa Gly Met His (SEQ ID NO: 50052)

Xaa Ile Xaa Tyr Asp Gly Ser Xaa Xaa Xaa Tyr Ala Asp Ser Val Lys Gly (SEQ ID NO: 50053)

Xaa Xaa Xaa Xaa Xaa Xaa Xaa Xaa Xaa Xaa Xaa Xaa Xaa Xaa Gly Xaa Asp Val (SEQ ID NO: 50054)

His Tyr Gly Met His (SEQ ID NO: 50528)

Val Ile Trp Tyr Asp Gly Ser Asn Lys Tyr Tyr Ala Asp Ser Val Lys Gly (SEQ ID NO: 50529)

Gly Asp Trp Asn Pro Glu Gly Met Asp Val (SEQ ID NO: 50530)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_179C7_HC | | A | | . | . | . | | | . | | . | | . | | . | | . | | . | . | . | . | . | . | . |
| 21-225_210D12_HC | . | . | . | . | . | . | | | . | . | . | . | . | . | . | . | . | . | . | . | . | . | . | . | . |
| 21-225_210E12_HC | . | X | . | . | . | . | | | . | . | . | . | . | . | . | . | . | . | . | . | . | . | . | . | . |
| 21-225_211C1_HC | . | . | . | . | . | . | | | . | | . | . | . | . | . | . | . | . | . | . | . | . | . | . | . |
| 21-225_211G5_HC | . | . | . | . | . | . | | | . | . | . | . | . | . | . | . | . | . | . | . | . | . | . | . | . |
| 21-225_212A4_HC | . | X | . | . | . | . | | | . | . | . | . | . | A | . | . | . | . | . | . | . | . | . | . | . |
| 21-225_212C2_HC | . | . | . | . | . | . | | | . | . | . | . | . | . | . | . | . | . | . | . | . | . | . | . | . |
| 21-225_212F6_HC | . | . | . | . | . | . | | | . | . | . | . | . | . | . | . | . | . | . | . | . | . | . | . | . |
| 21-225_212G7_HC | . | . | . | . | . | . | | | . | . | . | . | . | . | . | . | . | . | . | . | . | . | . | . | . |
| 21-225_213D5_HC | . | . | . | . | . | . | | | . | . | . | . | . | . | . | . | . | . | . | . | . | . | . | . | . |
| 21-225_213G3_HC | . | . | . | . | . | . | | | . | . | . | . | . | . | . | . | . | . | . | . | . | . | . | . | . |
| 21-225_215D3_HC | . | T | . | . | . | . | | | . | . | . | . | . | . | . | . | . | . | . | . | . | . | . | . | . |
| 21-225_216A3_HC | . | . | . | . | . | . | | | . | . | . | . | . | . | . | . | . | . | . | . | . | . | . | . | . |
| 21-225_216G1_HC | . | X | . | . | . | . | | | . | . | . | . | . | . | . | . | . | . | . | . | . | . | . | . | . |
| 21-225_217B2_HC | . | X | . | . | . | . | | | . | . | . | . | . | . | . | . | . | . | . | . | . | . | . | . | . |
| 21-225_219A7_HC | . | . | . | . | . | . | | | . | . | . | . | . | . | . | . | . | . | . | . | . | . | . | X | . |
| 21-225_221G4_HC | . | . | . | . | . | . | | | . | . | . | . | . | . | . | . | . | . | . | . | . | . | . | . | . |
| 21-225_221H2_HC | . | . | . | . | . | . | | | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | | H_FR2 |
| CONSENSUS | S | G | - | F | T | F | S | H | - | - | - | - | - | Y | G | M | H | W | V | R | Q | A | P | G | K |
| 21-225_179C7_HC | . | | . | . | . | . | | N | . | . | . | . | . | S | . | . | . | . | | . | | . | | . | . |
| 21-225_210D12_HC | . | . | . | . | . | . | . | . | . | . | . | . | . | . | . | . | . | . | . | . | . | . | . | . | . |
| 21-225_210E12_HC | . | | . | . | . | . | . | N | . | . | . | . | . | . | . | . | . | . | . | . | . | . | . | . | . |
| 21-225_211C1_HC | . | | . | . | . | . | . | . | . | . | . | . | . | . | . | . | . | . | . | . | . | . | . | . | . |
| 21-225_211G5_HC | . | | . | . | . | . | . | . | . | . | . | . | . | . | . | . | . | . | . | . | . | . | . | . | . |
| 21-225_212A4_HC | . | | . | . | . | . | . | N | . | . | . | . | . | . | . | . | . | . | . | . | . | . | . | . | . |
| 21-225_212C2_HC | . | | . | . | . | . | . | . | . | . | . | . | . | . | . | . | . | . | . | . | . | . | . | . | . |
| 21-225_212F6_HC | . | | . | . | . | . | . | . | . | . | . | . | . | . | . | . | . | . | . | . | . | . | . | . | . |
| 21-225_212G7_HC | . | | . | . | . | . | . | . | . | . | . | . | . | . | . | . | . | . | . | . | . | . | . | . | . |
| 21-225_213D5_HC | . | | . | . | . | . | . | . | . | . | . | . | . | . | . | . | . | . | . | . | . | . | . | . | . |
| 21-225_213G3_HC | . | | . | . | . | . | . | . | . | . | . | . | . | . | . | . | . | . | . | . | . | . | . | . | . |
| 21-225_215D3_HC | . | | . | . | . | . | . | . | . | . | . | . | . | . | . | . | . | . | . | . | . | . | . | . | . |
| 21-225_216A3_HC | . | | . | . | . | . | . | . | . | . | . | . | . | . | . | . | . | . | . | . | . | . | . | . | . |
| 21-225_216G1_HC | . | | . | . | . | . | . | N | . | . | . | . | . | . | . | . | . | . | . | . | . | . | . | . | . |
| 21-225_217B2_HC | . | | . | . | . | . | . | N | . | . | . | . | . | . | . | . | . | . | . | . | . | . | . | . | . |
| 21-225_219A7_HC | . | | . | . | . | . | . | . | . | . | . | . | . | . | . | . | . | . | . | . | . | . | . | . | . |
| 21-225_221G4_HC | . | | . | . | . | . | . | . | . | . | . | . | . | . | . | . | . | . | . | . | . | . | . | . | . |
| 21-225_221H2_HC | . | | . | . | . | . | . | . | . | . | . | . | . | . | . | . | . | . | . | . | . | . | . | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | | H_CDR2 | | | | | | | | |
| CONSENSUS | G | L | E | W | V | A | V | I | W | Y | D | - | - | - | G | S | N | K | Y | Y | A | D | S | V | K |
| 21-225_179C7_HC | | . | | . | . | . | I | . | Y | . | . | | . | . | | . | Y | | . | N | . | . | . | | . |
| 21-225_210D12_HC | . | . | . | . | . | . | . | . | . | . | . | . | . | . | . | . | . | . | . | . | . | . | . | . | . |
| 21-225_210E12_HC | . | . | . | . | . | . | . | . | . | . | . | . | . | . | . | . | . | . | . | . | . | . | . | . | . |
| 21-225_211C1_HC | . | . | . | . | . | . | . | . | . | . | . | . | . | . | . | . | . | . | . | . | . | . | . | . | . |
| 21-225_211G5_HC | . | . | . | . | . | . | . | . | . | . | . | . | . | . | . | . | . | . | . | . | . | . | . | . | . |
| 21-225_212A4_HC | . | . | . | . | . | . | . | . | . | . | . | . | . | . | . | . | . | . | . | . | . | . | . | . | . |
| 21-225_212C2_HC | . | . | . | . | . | . | . | . | . | . | . | . | . | . | . | . | . | . | . | . | . | . | . | . | . |
| 21-225_212F6_HC | . | . | . | . | . | . | . | . | . | . | . | . | . | . | . | . | . | . | . | . | . | . | . | . | . |
| 21-225_212G7_HC | . | . | . | . | . | . | . | . | . | . | . | . | . | . | . | . | . | . | . | C | . | . | . | . | . |
| 21-225_213D5_HC | . | . | . | . | . | . | . | . | . | . | . | . | . | . | . | . | . | . | . | . | . | . | . | . | . |
| 21-225_213G3_HC | . | . | . | . | . | . | . | . | . | . | . | . | . | . | . | . | . | . | . | . | . | . | . | . | . |
| 21-225_215D3_HC | . | . | . | . | . | . | . | . | . | . | . | . | . | . | . | . | . | E | . | . | . | . | . | . | . |
| 21-225_216A3_HC | . | . | . | . | . | . | . | . | . | . | . | . | . | . | . | . | . | . | . | . | . | . | . | . | . |
| 21-225_216G1_HC | . | . | . | . | . | . | . | . | . | . | . | . | . | . | . | . | . | . | . | . | . | . | . | . | . |
| 21-225_217B2_HC | . | . | . | . | . | . | . | . | . | . | . | . | . | . | . | . | . | . | . | . | . | . | . | . | . |
| 21-225_219A7_HC | . | . | . | . | . | . | . | . | . | . | . | . | . | . | . | . | . | . | . | . | . | . | . | . | . |
| 21-225_221G4_HC | . | . | . | . | . | . | . | . | . | . | . | . | . | . | . | . | . | . | . | . | . | . | . | . | . |
| 21-225_221H2_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | F | T | I | S | R | D | N | S | K | N | T | L | Y | L | Q | M | N | S | L | R | A | E | D |
| 21-225_179C7_HC | . | | . | | . | | . | | . | | . | | . | | . | | . | | . | | . | | . | | . |
| 21-225_210D12_HC | . | | . | | . | | . | | . | | . | | . | | . | | . | | . | | . | | . | | . |
| 21-225_210E12_HC | . | | . | | . | | . | | . | | . | | . | | . | | . | | . | | . | | . | | . |
| 21-225_211C1_HC | . | | . | | . | | . | | . | | . | | . | | . | | . | | T | | . | | . | | . |
| 21-225_211G5_HC | . | | . | | . | | . | | . | | . | | . | | . | | . | | . | | . | | . | | . |
| 21-225_212A4_HC | . | | . | | . | | . | | . | | . | | . | | . | | . | | . | | . | | . | | . |
| 21-225_212C2_HC | . | | . | | . | | . | | . | | . | | . | | . | | . | | . | | . | | . | | . |
| 21-225_212F6_HC | . | | . | | . | | . | | . | | . | | . | | . | | . | | . | | . | | . | | . |
| 21-225_212G7_HC | . | | . | | . | | . | | . | T | . | | . | | . | | . | | . | | . | | . | | . |
| 21-225_213D5_HC | . | | . | | . | | . | | . | | . | | . | | F | | . | | . | | . | | V | | . |
| 21-225_213G3_HC | . | | . | | . | | . | | . | | . | | . | | H | | . | | . | | . | | . | | . |
| 21-225_215D3_HC | . | | . | | . | | . | | . | | . | | . | | . | | . | | . | | . | | . | | . |
| 21-225_216A3_HC | . | | . | | . | | . | | . | | . | | . | | . | | . | | . | | . | | . | | . |
| 21-225_216G1_HC | . | | . | | . | | . | | . | | . | | . | | . | | . | | . | | . | | . | | . |
| 21-225_217B2_HC | . | | . | | . | | . | | . | | . | | . | | . | | . | | . | | . | | . | | . |
| 21-225_219A7_HC | . | | . | | . | | . | | . | | N | | . | | . | | . | | . | | . | | . | | . |
| 21-225_221G4_HC | . | | . | | . | | . | | . | | . | | . | | H | | . | | . | | . | | . | | . |
| 21-225_221H2_HC | . | | . | | . | | . | | . | | . | | . | | H | | . | | . | | . | | . | | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | G | D | W | N | P | - | - | - | - | - | - | - | - | - | - | - | - |
| 21-225_179C7_HC | | . | | . | . | . | | . | D | X | X | Y | D | F | X | V | P | . | . | . | . | . | . | | . |
| 21-225_210D12_HC | . | . | . | . | . | . | . | . | . | . | . | . | . | . | . | . | . | . | . | . | . | . | . | . | . |
| 21-225_210E12_HC | . | . | . | . | . | . | . | . | . | . | . | . | . | . | . | . | . | . | . | . | . | . | . | . | . |
| 21-225_211C1_HC | . | ? | . | . | . | . | . | . | . | . | . | . | . | . | . | . | . | . | . | . | . | . | . | . | . |
| 21-225_211G5_HC | . | . | . | . | . | . | . | . | . | . | . | . | . | . | . | . | . | . | . | . | . | . | . | . | . |
| 21-225_212A4_HC | . | . | . | . | . | . | . | . | . | . | . | . | . | . | . | . | . | . | . | . | . | . | . | . | . |
| 21-225_212C2_HC | . | . | . | . | . | . | . | . | . | . | . | . | . | . | . | . | . | . | . | . | . | . | . | . | . |
| 21-225_212F6_HC | . | . | . | . | . | . | . | . | . | . | . | . | . | . | . | . | . | . | . | . | . | . | . | . | . |
| 21-225_212G7_HC | . | . | . | . | . | . | . | . | . | . | . | . | . | . | . | . | . | . | . | . | . | . | . | . | . |
| 21-225_213D5_HC | . | . | . | . | . | . | . | . | . | . | . | . | . | . | . | . | . | . | . | . | . | . | . | . | . |
| 21-225_213G3_HC | . | . | . | . | . | . | . | . | . | . | . | . | . | . | . | . | . | . | . | . | . | . | . | . | . |
| 21-225_215D3_HC | . | . | . | . | . | . | . | . | . | . | . | . | . | . | . | . | . | . | . | . | . | . | . | . | . |
| 21-225_216A3_HC | . | . | . | . | . | . | . | . | . | . | . | . | . | . | . | . | . | . | . | . | . | . | . | . | . |
| 21-225_216G1_HC | . | . | . | . | . | . | . | . | . | . | . | . | . | . | . | . | . | . | . | . | . | . | . | . | . |
| 21-225_217B2_HC | . | . | . | . | . | . | . | . | . | . | . | . | . | . | . | . | . | . | . | . | . | . | . | . | . |
| 21-225_219A7_HC | . | . | . | . | . | . | . | . | . | . | . | . | . | . | . | . | . | . | . | . | . | . | . | . | . |
| 21-225_221G4_HC | . | . | . | . | . | . | . | . | . | . | . | . | . | . | . | . | . | . | . | . | . | . | . | . | . |
| 21-225_221H2_HC | . | . | . | . | . | . | . | . | . | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | E | G | M | D | V | W | G | Q | G | T | T | V | T | V | S | S |
| 21-225_179C7_HC | | | | . | Y | Y | Y | Y | Y | . | . | | | | | | | | | | | | | |
| 21-225_210D12_HC | | | | | | | | | | | L | | | | | | | | | | | | | |
| 21-225_210E12_HC | | | | | | | | | | | | | | | | | | | | | I | | | |
| 21-225_211C1_HC | | | | | | | | | | | L | | | | | | | | | | | | | |
| 21-225_211G5_HC | | | | | | | | | | | L | | | | | | | | | | | | | |
| 21-225_212A4_HC | | | | | | | | | | | | | | | | | | | | | I | | | |
| 21-225_212C2_HC | | | | | | | | | | | | | | | | | | | S | | | | | |
| 21-225_212F6_HC | | | | | | | | | | | L | | | | | | | | | | | | | |
| 21-225_212G7_HC | | | | | | | | | | | L | | | | | | | | | | | | | |
| 21-225_213D5_HC | | | | | | | | | | | | | | | | | | | | | | | | |
| 21-225_213G3_HC | | | | | | | | | | | | | | | | | | | S | | | | | |
| 21-225_215D3_HC | | | | | | | | | | | | | | | | | | | | | | | | |
| 21-225_216A3_HC | | | | | | | | | | | L | | | | | | | | | | | | | |
| 21-225_216G1_HC | | | | | | | | | | | | | | | | | | | | | I | | | |
| 21-225_217B2_HC | | | | | | | | | | | | | | | | | | | | | I | | | |
| 21-225_219A7_HC | | | | | | | | | | | | | | | | | | | | | | | | |
| 21-225_221G4_HC | | | | | | | | | | | | | | | | | | | S | | | | | |
| 21-225_221H2_HC | | | | | | | | | | | | | | | | | | | | | | | | |

**FIGURE 57**

Table 70.    Consensus 36- VH3|3-23/D4|4-17|RF2/JH4 (SEQ ID NO: 50287):

EVQLLES-GGGLVQPGGSLRLSCAASG-FTFSS-----YAMSWVRQAPGKGLEWVSAISGS---
GGNTFYADSVKGRFTISRDNSKNTLYLQMNSLRAEDTAVYYCAKKDYDYVW---------------GSPYFDYWGQGTLVTVSS wherein:

Y in position 39 can be substituted with S

S in position 42 can be substituted with T or N

A in position 57 can be substituted with V

S in position 59 can be substituted with I

R in position 61 can be substituted with S, N or F

G in position 66 can be substituted with S

N in position 67 can be substituted with R or S

T in position 68 can be substituted with A

F in position 69 can be substituted with Y

K in position 109 can be substituted with D or R

D in position 110 can be substituted with Y, H, M or R

Y in position 111 can be substituted with G or N

D in position 112 can be substituted with I, R or Y

Y in position 113 can be substituted with null (-), S or V

V in position 114 can be substituted with null (-), G, R or S

W in position 115 can be substituted with null (-) or I

null (-) in position 116 can be substitute with A

FIGURE 57

G in position 132 can be substituted with null (-) or V

S in position 133 can be substituted with null (-), A, T or Y

P in position 134 can be substituted with null (-), G or I

Y in position 135 can be substituted with F or T

Ser Xaa Ala Met Xaa (SEQ ID NO: 50055)

Xaa Ile Xaa Gly Xaa Gly Xaa Xaa Xaa Xaa Tyr Ala Asp Ser Val Lys Gly (SEQ ID NO: 50056)

Xaa Xaa Xaa Xaa Xaa Xaa Xaa Xaa Xaa Xaa Xaa Xaa Phe Asp Tyr (SEQ ID NO: 50057)

Ser Tyr Ala Met Ser (SEQ ID NO: 50531)

Ala Ile Ser Gly Ser Gly Gly Asn Thr Phe Tyr Ala Asp Ser Val Lys Gly (SEQ ID NO: 50532)

Lys Asp Tyr Asp Tyr Val Trp Gly Ser Pro Tyr Phe Asp Tyr (SEQ ID NO: 50533)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A |
| 21-225_146A6_HC | . | . | . | . | . | . | . | . | . | . | . | . | . | . | . | . | . | . | . | . | . | . | . | . | . |
| 21-225_147D10_HC | . | . | . | . | . | . | . | . | . | . | . | . | . | . | . | . | . | . | . | . | . | . | . | . | . |
| 21-225_147D12_HC | . | . | . | . | . | . | . | . | . | . | . | . | . | . | S | . | . | . | . | . | . | . | . | . | . |
| 21-225_148E2_HC | . | . | . | . | . | . | . | . | . | . | . | . | . | . | . | . | . | . | . | . | . | . | . | . | . |
| 21-225_149C6_HC | . | . | . | . | . | . | . | . | . | . | . | . | . | . | . | . | . | . | . | . | . | . | . | . | . |
| 21-225_149D11_HC | . | . | . | . | . | . | . | . | . | . | . | . | . | . | . | . | . | . | . | . | . | . | . | . | . |
| 21-225_149F8_HC | . | . | . | . | . | . | . | . | . | . | . | . | . | . | . | . | . | . | . | . | . | . | . | . | . |
| 21-225_150C5_HC | . | . | . | . | . | . | . | . | . | . | . | . | . | . | . | . | . | . | . | . | . | . | . | . | . |
| 21-225_152G10_HC | . | . | . | . | . | . | . | . | . | . | . | . | . | . | . | . | . | . | . | . | . | . | . | . | . |
| 21-225_152H7_HC | . | . | . | . | . | . | . | . | . | . | . | . | . | . | . | . | . | . | . | . | . | . | . | . | . |
| 21-225_155A4_HC | . | . | . | . | . | . | . | . | . | . | . | . | . | . | . | . | . | . | . | . | . | . | . | . | . |
| 21-225_155B4_HC | . | . | . | . | . | . | . | . | . | . | . | . | . | . | . | . | . | . | . | . | . | . | . | . | . |
| 21-225_178F7_HC | . | . | X | . | . | . | . | . | . | . | . | . | . | . | T | . | . | . | . | . | . | . | . | . | . |
| 21-225_179G1_HC | . | . | X | . | . | . | . | . | . | . | . | . | . | . | T | . | . | . | . | . | . | . | . | . | . |
| 21-225_190D12_HC | . | . | . | . | . | . | . | . | . | . | . | . | . | . | . | . | . | . | . | . | . | . | . | . | . |
| 21-225_197C8_HC | . | . | . | . | . | . | . | . | . | . | . | . | . | . | . | . | . | . | . | . | . | . | . | . | . |
| 21-225_55E1_HC | . | . | . | . | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | |
| CONSENSUS | S | G | - | F | T | F | S | S | - | - | - | - | - | Y | A | M | S | W | V | R | Q | A | P | G | K |
| 21-225_146A6_HC | . | . | . | . | . | . | N | . | . | . | . | . | . | . | . | . | N | . | . | . | . | . | . | . | . |
| 21-225_147D10_HC | . | . | . | . | . | . | . | . | . | . | . | . | . | . | . | . | . | . | . | . | . | . | . | . | . |
| 21-225_147D12_HC | . | . | . | . | . | . | . | . | . | . | . | . | . | . | . | . | . | . | . | . | . | . | . | . | . |
| 21-225_148E2_HC | . | . | . | . | . | . | . | . | . | . | . | . | . | . | . | . | . | . | . | . | . | . | . | . | . |
| 21-225_149C6_HC | . | . | . | . | . | . | . | . | . | . | . | . | . | . | . | . | . | . | . | . | . | . | . | . | . |
| 21-225_149D11_HC | . | . | . | . | . | . | . | . | . | . | . | . | . | . | . | . | . | . | . | . | . | . | . | . | . |
| 21-225_149F8_HC | . | . | . | . | . | . | . | . | . | . | . | . | . | . | . | . | . | . | . | . | . | . | . | . | . |
| 21-225_150C5_HC | . | . | . | . | . | . | . | . | . | . | . | . | . | . | . | . | . | . | . | . | . | . | . | . | . |
| 21-225_152G10_HC | . | . | . | . | . | . | . | . | . | . | . | . | . | . | . | . | . | . | . | . | . | . | . | . | . |
| 21-225_152H7_HC | . | . | . | . | . | . | N | . | . | . | . | . | . | . | . | . | N | . | . | . | . | . | . | . | . |
| 21-225_155A4_HC | . | . | . | . | . | . | N | . | . | . | . | . | . | . | . | . | . | . | . | . | . | . | . | . | . |
| 21-225_155B4_HC | . | . | . | . | . | . | . | . | . | . | . | . | . | . | . | . | . | . | . | . | . | . | . | . | . |
| 21-225_178F7_HC | . | . | . | . | . | . | . | . | . | . | . | . | . | . | . | . | T | . | . | . | . | . | . | . | . |
| 21-225_179G1_HC | . | . | . | . | . | . | . | . | . | . | . | . | . | . | . | . | T | . | . | . | . | . | . | . | . |
| 21-225_190D12_HC | . | . | . | . | . | . | . | . | . | . | . | . | . | . | . | . | . | . | . | . | . | . | . | . | . |
| 21-225_197C8_HC | . | . | . | . | . | . | . | . | . | . | . | . | . | S | . | . | T | . | . | . | . | . | . | . | . |
| 21-225_55E1_HC | . | . | . | . | . | . | . | . | . | . | . | . | . | . | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | H_CDR2 | | | | | | | | | | |
| CONSENSUS | G | L | E | W | V | S | A | I | S | G | | - | - | - | G | G | N | T | F | Y | A | D | S | V | K |
| 21-225_146A6_HC | . | . | . | . | . | . | . | . | . | . | S | . | . | . | . | . | . | . | . | . | . | . | . | . | . |
| 21-225_147D10_HC | . | . | . | . | . | . | . | . | . | . | R | . | . | . | . | . | . | . | . | . | . | . | . | . | . |
| 21-225_147D12_HC | . | . | . | . | . | . | . | . | . | . | R | . | . | . | . | . | . | . | . | . | . | . | . | . | . |
| 21-225_148E2_HC | . | . | . | . | . | . | . | . | . | . | R | . | . | . | . | . | . | . | . | . | . | . | . | . | . |
| 21-225_149C6_HC | . | . | . | . | . | . | . | . | . | . | S | . | . | . | . | . | . | . | . | . | . | . | . | . | . |
| 21-225_149D11_HC | . | . | . | . | . | . | . | . | . | . | R | . | . | . | . | . | . | . | . | . | . | . | . | . | . |
| 21-225_149F8_HC | . | . | . | . | . | . | . | . | . | . | R | . | . | . | . | . | . | . | . | . | . | . | . | . | . |
| 21-225_150C5_HC | . | . | . | . | . | . | . | . | . | . | S | . | . | . | . | . | . | . | . | . | . | . | . | . | . |
| 21-225_152G10_HC | . | . | . | . | . | . | . | . | . | . | R | . | . | . | . | . | . | . | . | . | . | . | . | . | . |
| 21-225_152H7_HC | . | . | . | . | . | . | . | . | . | . | S | . | . | . | . | . | . | . | . | . | . | . | . | . | . |
| 21-225_155A4_HC | . | . | . | . | . | . | . | . | . | . | S | . | . | . | . | . | . | . | . | . | . | . | . | . | . |
| 21-225_155B4_HC | . | . | . | . | . | . | . | . | . | . | S | . | . | . | . | . | . | . | . | . | . | . | . | . | . |
| 21-225_178F7_HC | . | . | . | . | V | . | . | . | . | . | S | . | . | . | . | . | . | . | . | . | . | . | . | . | . |
| 21-225_179G1_HC | . | . | . | . | V | . | . | . | . | . | R | . | . | . | . | . | . | . | . | . | . | . | . | . | . |
| 21-225_190D12_HC | . | . | . | . | O | . | . | . | I | . | N | . | . | . | . | R | . | . | Y | . | . | . | . | . | . |
| 21-225_197C8_HC | . | . | . | . | . | . | . | . | I | . | N | . | . | . | . | R | A | . | Y | . | . | . | . | . | . |
| 21-225_55E1_HC | . | . | . | . | . | . | . | . | . | . | R | . | . | . | S | S | . | . | . | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | F | T | I | S | R | D | N | S | K | N | T | L | Y | L | Q | M | N | S | L | R | A | E | D |
| 21-225_146A6_HC | . | . | . | . | . | . | . | . | . | . | . | . | . | . | . | . | . | . | . | . | . | . | . | . | . |
| 21-225_147D10_HC | . | . | . | . | . | . | . | . | . | . | . | . | . | . | . | . | . | . | . | . | . | . | . | . | . |
| 21-225_147D12_HC | . | . | . | . | . | . | . | . | . | . | . | . | . | . | . | . | . | . | . | . | . | . | . | . | . |
| 21-225_148E2_HC | . | . | . | . | . | . | . | . | . | . | . | . | . | . | . | . | . | . | . | . | . | . | . | . | . |
| 21-225_149C6_HC | . | . | . | . | . | . | . | . | . | . | . | . | . | . | . | . | . | . | . | . | . | . | . | . | . |
| 21-225_149D11_HC | . | . | . | . | . | . | . | . | . | . | . | . | . | . | . | . | . | . | . | . | . | . | . | . | . |
| 21-225_149F8_HC | . | . | . | . | . | . | . | . | . | . | . | . | . | . | . | . | . | . | . | . | . | . | . | . | . |
| 21-225_150C5_HC | . | . | . | . | . | . | . | . | . | . | . | . | . | . | . | . | . | . | . | . | . | . | . | . | . |
| 21-225_152G10_HC | . | . | . | . | . | . | . | . | . | . | . | . | . | . | . | . | . | . | . | . | . | . | . | . | . |
| 21-225_152H7_HC | . | . | . | . | . | . | . | . | . | . | . | . | . | . | . | . | . | . | . | . | . | . | . | . | . |
| 21-225_155A4_HC | . | . | . | . | . | . | . | . | . | . | . | . | . | . | . | . | . | . | . | . | . | . | . | . | . |
| 21-225_155B4_HC | . | . | . | . | . | . | . | . | . | . | . | . | . | . | . | . | . | . | . | . | . | . | . | . | . |
| 21-225_178F7_HC | . | . | . | . | . | . | . | . | . | . | . | . | . | . | . | . | . | . | . | . | . | . | . | . | . |
| 21-225_179G1_HC | . | . | . | . | . | . | . | . | . | . | . | . | . | . | . | . | . | . | . | . | . | . | . | . | . |
| 21-225_190D12_HC | . | . | . | . | . | . | . | . | . | . | . | . | . | . | . | . | . | . | . | . | . | . | . | . | . |
| 21-225_197C8_HC | . | . | . | . | . | . | . | . | . | . | . | . | . | . | . | . | . | . | . | . | . | . | T | . | . |
| 21-225_55E1_HC | . | . | . | . | . | . | . | . | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | K | K | D | Y | D | Y | V | W | - | - | - | - | - | - | - | - | - | - |
| 21-225_146A6_HC | . | . | . | . | . | . | . | . | . | . | N | . | . | . | . | | | | | | | | | | |
| 21-225_147D10_HC | . | . | . | . | . | . | . | . | . | . | . | . | . | . | . | | | | | | | | | | |
| 21-225_147D12_HC | . | . | . | . | . | . | . | . | . | . | . | . | . | . | . | | | | | | | | | | |
| 21-225_148E2_HC | . | . | . | . | . | . | . | . | . | . | . | . | . | . | . | | | | | | | | | | |
| 21-225_149C6_HC | . | . | . | . | . | . | . | . | . | . | . | . | . | . | . | | | | | | | | | | |
| 21-225_149D11_HC | . | . | . | . | . | . | . | . | . | . | . | . | . | . | . | | | | | | | | | | |
| 21-225_149F8_HC | . | . | . | . | . | . | . | . | . | . | . | . | . | . | . | | | | | | | | | | |
| 21-225_150C5_HC | . | . | . | . | . | . | . | . | . | . | . | . | . | . | . | | | | | | | | | | |
| 21-225_152G10_HC | . | . | . | . | . | . | . | . | . | . | . | . | . | . | . | | | | | | | | | | |
| 21-225_152H7_HC | . | . | . | . | . | . | . | . | . | . | N | . | . | . | . | | | | | | | | | | |
| 21-225_155A4_HC | . | . | . | . | . | . | . | . | . | . | . | . | . | . | . | | | | | | | | | | |
| 21-225_155B4_HC | . | . | . | . | . | . | . | . | . | . | . | . | . | . | . | | | | | | | | | | |
| 21-225_178F7_HC | . | . | . | . | . | . | . | R | R | Y | G | | - | - | - | | | | | | | | | | |
| 21-225_179G1_HC | . | . | . | . | . | . | . | R | R | Y | G | | - | - | - | | | | | | | | | | |
| 21-225_190D12_HC | . | . | . | . | . | . | . | . | D | M | G | R | . | S | - | | | | | | | | | | |
| 21-225_197C8_HC | . | . | . | . | . | . | . | . | D | R | G | Y | S | R | I | A | | | | | | | | | |
| 21-225_55E1_HC | . | . | . | P | . | . | . | . | D | M | G | I | V | G | - | | | | | | | | | | |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | H_FR4 | | | | | | |
| CONSENSUS | - | - | - | - | - | - | G | S | P | Y | F | D | Y | W | G | Q | G | T | L | V | T | V | S | S |
| 21-225_146A6_HC | . | . | . | . | . | . | . | . | . | . | . | . | . | . | . | . | . | . | . | . | . | . | . | . |
| 21-225_147D10_HC | . | . | . | . | . | . | . | . | . | . | . | . | . | . | . | . | . | . | . | . | . | . | . | . |
| 21-225_147D12_HC | . | . | . | . | . | . | . | . | . | . | . | . | . | . | . | . | . | . | . | . | . | . | . | . |
| 21-225_148E2_HC | . | . | . | . | . | . | . | . | . | . | . | . | . | . | . | . | . | . | . | . | . | . | . | . |
| 21-225_149C6_HC | . | . | . | . | . | . | . | . | . | . | . | . | . | . | . | . | . | . | . | . | . | . | . | . |
| 21-225_149D11_HC | . | . | . | . | . | . | . | . | . | . | . | . | . | . | . | . | . | . | . | . | . | . | . | . |
| 21-225_149F8_HC | . | . | . | . | . | . | . | . | . | . | . | . | . | . | . | . | . | . | . | . | . | . | . | . |
| 21-225_150C5_HC | . | . | . | . | . | . | . | . | . | . | . | . | . | . | . | . | . | . | . | . | . | . | . | . |
| 21-225_152G10_HC | . | . | . | . | . | . | . | . | . | . | . | . | . | . | . | . | . | . | . | . | . | . | . | . |
| 21-225_152H7_HC | . | . | . | . | . | . | . | . | . | . | . | . | . | . | . | . | . | . | . | . | . | . | . | . |
| 21-225_155A4_HC | . | . | . | . | . | . | . | . | . | . | . | . | . | . | . | . | . | . | . | . | . | . | . | . |
| 21-225_155B4_HC | . | . | . | . | . | . | . | . | . | . | . | . | . | . | . | . | . | . | . | . | . | . | . | . |
| 21-225_178F7_HC | . | . | . | . | . | | - | - | - | . | . | . | . | . | . | . | . | . | . | . | . | . | . | . |
| 21-225_179G1_HC | . | . | . | . | . | | - | - | - | . | . | . | . | . | . | . | . | . | . | . | . | . | . | . |
| 21-225_190D12_HC | . | . | . | . | . | | - | Y | Q | P | . | . | . | . | . | . | . | . | . | . | . | . | . | . |
| 21-225_197C8_HC | . | . | . | . | . | | V | A | Q | T | . | . | . | . | . | . | . | . | . | . | . | . | . | . |
| 21-225_55E1_HC | . | . | . | . | . | | - | T | I | . | . | . | . | . | . | . | . | . | . | . | . | . | . | . |

**FIGURE 57**

Table 71.    Consensus 37- VH1|1-02/D3|3-22|RF2/JH4 (SEQ ID NO: 50288):

QVQLVQS-GAEVKKPGASVKVSCKASG-YTFT<u>G----YYMH</u>WVRQAPGQGLEWMG<u>WINPN---
SGGTNYAQKFQG</u>RVTMTRDTSISTAYMELSRLRSDDTAVYYCAR<u>SYYYGSGS--------------YYNEFDY</u>WGQGTLVTVSS wherein:

G in position 33 can be substituted with D

Y in position 39 can be substituted with H

Y in position 40 can be substituted with C

M in position 41 can be substituted with I

W in position 57 can be substituted with S

N in position 59 can be substituted with Y

P in position 60 can be substitute with R

N in position 61 can be substituted with K

G in position 67 can be substituted with A

T in position 68 can be substituted with A

N in position 69 can be substituted with D

Y in position 70 can be substituted with N or S

A in position 71 can be substituted with G or V

G in position 76 can be substituted with D or V

S in position 109 can be substituted with A, V or T

Y in position 110 can be substituted with F or N

Y in position 112 can be substituted with H

FIGURE 57

S in position 116 can be substituted with T

Y in position 133 can be substituted with H

E in position 135 can be substituted with G or D

Xaa Xaa Xaa Xaa His (SEQ ID NO: 50058)

Xaa Ile Xaa Xaa Xaa Ser Gly Xaa Xaa Xaa Xaa Xaa Gln Lys Phe Gln Xaa (SEQ ID NO: 50059)

Xaa Xaa Tyr Xaa Gly Ser Gly Xaa Tyr Xaa Asn Xaa Phe Asp Tyr (SEQ ID NO: 50060)

Gly Tyr Tyr Met His (SEQ ID NO: 50534)

Trp Ile Asn Pro Asn Ser Gly Gly Thr Asn Tyr Ala Gln Lys Phe Gln Gly (SEQ ID NO: 50535)

Ser Tyr Tyr Tyr Gly Ser Gly Ser Tyr Tyr Asn Glu Phe Asp Tyr (SEQ ID NO: 50536)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | | | | | | | | | | | | | | | | H_FR1 | | | | | | | | | | | |
| CONSENSUS | Q | V | Q | L | V | Q | S | - | G | A | E | V | K | K | P | G | A | S | V | K | V | S | C | K | A | S | G |
| 21-225_13F6_HC | . | | | | | | | | | | | | | | | | | | | | | | | | | | |
| 21-225_16F3_HC | | | | | | | | | | | | | | | | | | | | | | | | | | | |
| 21-225_17D3_HC | . | | | | | | | | | | | | | | | | | | | | | | | | | | |
| 21-225_18E10_HC | . | | | | | | | | | | | | | | | | | | | | | | | | | | |
| 21-225_195E9_HC | . | | V | | | | | | | | | | | | | | | | | | | | | | | | |
| 21-225_21D12_HC | . | | | | | | | | | | | | | | | | | | | | | | | | | | |
| 21-225_31D12_LC1_HC | | X | | | | | | | | | | | | | | | | | | | | | | | | | |
| 21-225_31D12_LC2_HC | . | | X | | | | | | | | | | | | | | | | | | | | | | | | |
| 21-225_33E6_HC | | | X | | | | | | | | | | | | | | | | | | | | | | | | |
| 21-225_35E1_HC | | X | | | | | | | | | | | | | | | | | | | | | | | | | |
| 21-225_4B3_HC | . | | | | | | | | | | | | | | | | | | | | | | | | | | |
| 21-225_68G6_HC | | | | | | P | | | G | | | | | | | S | | | | | | | | | | | |
| 21-225_69B11_HC | . | | | | | | | | | | | | | | | | | | | X | | | | | | | |
| 21-225_71D4_HC | | | | | | | | | | | | | | | | | | | | | | | | | | | |
| 21-225_72B4_HC | | | | | | | | | | | | | | | | | | | | | | | | | | | |
| 21-225_9G9_HC | . | | | | | | | | | | | | | | | | | | | | | | | | | | |

FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | | H_CDR1 | | | | | | | | | | | | H_FR2 | | | | | | | | | |
| CONSENSUS | - | Y | T | F | T | G | - | - | - | - | - | Y | Y | M | H | W | V | R | Q | A | P | G | Q | G | L | E | W |
| 21-225_13F6_HC | . | . | . | . | . | . | . | . | . | . | . | . | . | . | . | . | . | . | . | . | . | . | . | . | . | . | . |
| 21-225_16F3_HC | . | . | . | . | . | . | . | . | . | . | . | . | . | . | . | . | . | . | . | . | . | . | . | . | . | . | . |
| 21-225_17D3_HC | . | . | . | . | . | . | . | . | . | . | . | . | . | . | . | . | . | . | . | . | . | . | . | . | . | . | . |
| 21-225_18E10_HC | . | . | . | . | . | . | . | . | . | . | . | . | . | . | . | . | . | . | . | . | . | . | . | . | . | . | . |
| 21-225_195E9_HC | . | . | . | . | . | . | . | . | . | . | . | . | . | . | . | . | . | . | . | . | . | . | . | . | . | . | . |
| 21-225_21D12_HC | . | . | . | . | . | . | . | . | . | . | . | . | . | . | . | . | . | . | . | . | . | . | . | . | . | . | . |
| 21-225_31D12_LC1_HC | . | . | . | . | . | . | . | . | . | . | . | . | . | . | . | . | . | . | . | . | . | . | . | . | . | . | . |
| 21-225_31D12_LC2_HC | . | . | . | . | . | . | . | . | . | . | . | . | . | . | . | . | . | . | . | . | . | . | . | . | . | . | . |
| 21-225_33E6_HC | . | . | . | . | . | . | . | . | . | . | . | . | . | . | . | . | . | . | . | . | . | . | . | . | . | . | . |
| 21-225_35E1_HC | . | . | . | . | . | . | . | . | . | . | . | . | . | . | . | . | . | . | . | . | . | . | . | . | . | . | . |
| 21-225_4B3_HC | . | . | . | . | . | . | . | . | . | . | . | . | . | . | . | . | . | . | . | . | . | . | . | . | . | . | . |
| 21-225_68G6_HC | . | . | . | . | . | . | . | . | . | . | . | . | . | . | . | . | . | . | . | . | . | . | . | . | . | . | . |
| 21-225_69B11_HC | . | . | . | . | . | . | . | . | . | . | . | . | . | . | . | . | . | . | . | . | . | . | . | . | . | . | . |
| 21-225_71D4_HC | . | . | . | . | . | . | . | . | . | . | . | . | . | . | . | . | . | . | . | . | . | . | . | . | . | . | . |
| 21-225_72B4_HC | . | . | . | . | . | . | . | . | . | . | . | . | . | . | . | . | . | . | . | . | . | . | . | . | . | . | . |
| 21-225_9G9_HC | . | . | . | . | . | . | . | . | . | . | . | . | . | . | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | | | | | | | | | | H_CDR2 | | | | | | | | | | | | | | |
| CONSENSUS | M | G | W | I | N | P | N | - | - | - | S | G | G | T | N | Y | A | Q | K | F | Q | G | R | V | T | M | T |
| 21-225_13F6_HC | . | . | . | . | . | . | . | . | . | . | . | . | . | A | . | . | . | . | . | . | . | . | . | . | . | . | . |
| 21-225_16F3_HC | . | . | . | . | . | . | . | . | . | . | . | . | . | . | . | . | . | . | . | . | . | . | . | . | . | . | . |
| 21-225_17D3_HC | . | . | . | . | . | . | . | . | . | . | . | . | . | . | . | . | . | . | . | . | . | . | . | . | . | . | . |
| 21-225_18E10_HC | . | . | . | . | . | . | . | . | . | . | . | . | . | . | . | . | . | . | . | . | . | . | . | . | . | . | . |
| 21-225_195E9_HC | . | . | . | . | . | . | . | . | . | . | . | . | A | . | . | . | . | . | . | . | . | . | . | . | . | . | . |
| 21-225_21D12_HC | . | . | . | . | . | . | . | . | . | . | . | . | . | . | N | . | . | . | . | . | . | . | . | . | . | . | . |
| 21-225_31D12_LC1_HC | . | . | . | . | . | . | . | . | . | . | . | . | . | . | . | . | V | . | . | . | . | . | . | . | . | . | . |
| 21-225_31D12_LC2_HC | . | . | . | . | . | . | . | . | . | . | . | . | . | . | . | . | V | . | . | . | . | . | . | . | . | . | . |
| 21-225_33E6_HC | . | . | . | . | . | . | . | . | . | . | . | . | . | . | . | . | . | . | . | . | . | . | . | . | . | . | . |
| 21-225_35E1_HC | . | . | . | . | X | . | . | . | . | . | . | . | . | . | . | . | . | . | . | . | . | . | . | . | . | . | . |
| 21-225_4B3_HC | . | . | . | . | . | . | . | . | . | . | . | . | . | . | . | S | . | . | . | . | . | . | . | . | . | . | . |
| 21-225_68G6_HC | . | S | . | Y | R | . | . | . | . | . | . | . | . | . | . | . | . | . | . | . | . | . | . | . | . | . | . |
| 21-225_69B11_HC | . | . | . | . | . | . | . | . | . | . | . | . | . | . | . | . | G | . | . | . | . | O | . | . | . | . | . |
| 21-225_71D4_HC | . | . | . | . | . | . | . | . | . | . | . | . | . | . | . | . | . | . | . | . | . | V | . | . | . | . | . |
| 21-225_72B4_HC | . | . | . | . | . | . | . | . | . | . | . | . | . | . | . | . | G | . | . | . | . | . | . | . | . | . | . |
| 21-225_9G9_HC | . | . | . | . | . | . | . | . | . | . | . | . | . | . | D | . | . | . | . | . | . | . | . | . | . | . | . |

FIGURE 57

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | | | | | | | | | | H_FR3 | | | | | | | | | | | | | | | | | |
| CONSENSUS | R | D | T | S | I | S | T | A | Y | M | E | L | S | R | L | R | S | D | D | T | A | V | Y | Y | C | A | R |
| 21-225_13F6_HC | . | . | . | . | . | . | . | . | . | . | . | . | . | . | . | . | . | . | . | . | . | . | . | P | . | . | . |
| 21-225_16F3_HC | . | . | . | . | . | . | . | . | . | . | . | . | . | . | . | . | . | . | . | . | . | . | . | . | . | . | . |
| 21-225_17D3_HC | . | . | . | . | . | . | . | . | . | . | . | . | . | . | . | . | . | . | . | . | . | . | . | . | . | . | . |
| 21-225_18E10_HC | . | . | S | . | . | N | . | . | . | . | . | . | . | . | . | . | . | . | . | . | . | . | . | . | . | . | . |
| 21-225_195E9_HC | . | . | . | . | . | . | . | . | . | . | . | N | . | . | . | . | . | . | . | . | . | . | . | . | . | . | . |
| 21-225_21D12_HC | . | . | . | . | . | . | . | . | . | . | . | . | . | . | . | . | . | . | . | . | . | . | . | S | . | . | . |
| 21-225_31D12_LC1_HC | . | . | . | . | . | . | . | . | . | . | . | . | . | . | . | . | . | . | . | . | . | . | . | . | . | . | . |
| 21-225_31D12_LC2_HC | . | . | . | . | . | . | . | . | . | . | . | . | . | . | . | . | . | . | . | . | . | . | . | . | . | . | . |
| 21-225_33E6_HC | . | . | . | . | . | . | . | . | . | . | . | . | . | . | . | . | . | . | . | . | . | . | . | . | . | . | . |
| 21-225_35E1_HC | . | . | . | . | . | . | . | V | . | . | . | P | . | . | . | . | . | . | . | . | . | . | . | . | . | . | . |
| 21-225_4B3_HC | . | . | . | . | . | . | . | . | . | . | . | . | . | . | . | . | . | . | . | . | . | . | . | . | . | . | . |
| 21-225_68G6_HC | . | N | . | . | . | . | . | . | . | . | N | . | . | . | I | . | . | . | . | . | . | . | . | . | . | . | . |
| 21-225_69B11_HC | . | . | . | . | . | . | P | . | . | . | . | . | . | . | . | . | . | . | . | . | . | A | . | . | . | . | . |
| 21-225_71D4_HC | . | . | . | V | . | . | V | . | D | . | . | . | . | . | . | . | . | . | . | . | . | . | . | . | . | . | . |
| 21-225_72B4_HC | . | . | . | . | . | . | . | . | . | . | . | . | . | . | . | . | . | . | . | . | . | . | . | . | . | . | . |
| 21-225_9G9_HC | . | . | . | . | . | . | . | . | . | . | . | . | . | . | . | . | . | . | . | . | . | . | . | . | . | . | . |

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | | | | | | | | | | | | | | | H_CDR3 | | | | | | | | | | | | |
| CONSENSUS | S | Y | Y | Y | G | S | G | S | - | - | - | - | - | - | - | - | - | - | - | - | - | - | - | Y | Y | N | E |
| 21-225_13F6_HC | . | F | . | . | . | . | . | T | . | . | . | . | . | . | . | . | . | . | . | . | . | . | . | . | . | . | . |
| 21-225_16F3_HC | . | . | . | . | . | . | . | T | . | . | . | . | . | . | . | . | . | . | . | . | . | . | . | . | . | . | . |
| 21-225_17D3_HC | A | N | . | . | . | . | . | . | . | . | . | . | . | . | . | . | . | . | . | . | . | . | . | . | . | . | D |
| 21-225_18E10_HC | . | . | . | . | . | . | . | . | . | . | . | . | . | . | . | . | . | . | . | . | . | . | . | . | . | . | . |
| 21-225_195E9_HC | A | . | . | . | . | . | . | T | . | . | . | . | . | . | . | . | . | . | . | . | . | . | . | . | . | . | . |
| 21-225_21D12_HC | . | . | . | . | . | . | . | T | . | . | . | . | . | . | . | . | . | . | . | . | . | . | . | . | . | . | . |
| 21-225_31D12_LC1_HC | V | . | . | . | . | . | . | . | . | . | . | . | . | . | . | . | . | . | . | . | . | . | . | . | . | . | . |
| 21-225_31D12_LC2_HC | V | . | . | . | . | . | . | . | . | . | . | . | . | . | . | . | . | . | . | . | . | . | . | . | . | . | . |
| 21-225_33E6_HC | V | . | . | . | . | . | . | . | . | . | . | . | . | . | . | . | . | . | . | . | . | . | . | . | . | . | . |
| 21-225_35E1_HC | V | . | . | H | . | . | . | . | . | . | . | . | . | . | . | . | . | . | . | . | . | . | . | . | . | . | . |
| 21-225_4B3_HC | . | . | . | . | . | . | . | . | . | . | . | . | . | . | . | . | . | . | . | . | . | . | . | . | . | . | . |
| 21-225_68G6_HC | A | F | . | . | . | . | . | T | . | . | . | . | . | . | . | . | . | . | . | . | . | . | . | . | . | . | . |
| 21-225_69B11_HC | T | . | . | . | . | . | . | . | . | . | . | . | . | . | . | . | . | . | . | . | . | . | . | . | . | . | D |
| 21-225_71D4_HC | A | . | . | . | . | . | . | T | . | . | . | . | . | . | . | . | . | . | . | . | . | . | . | . | H | . | . |
| 21-225_72B4_HC | T | . | . | . | . | . | . | . | . | . | . | . | . | . | . | . | . | . | . | . | . | . | . | . | . | . | D |
| 21-225_9G9_HC | . | . | . | . | . | . | . | T | . | . | . | . | . | . | . | . | . | . | . | . | . | . | . | . | . | . | . |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | | | | H_FR4 | | | | | | | |
| CONSENSUS | F | D | Y | W | G | Q | G | T | L | V | T | V | S | S |
| 21-225_13F6_HC | . | . | . | . | . | . | . | . | . | . | . | . | . | . |
| 21-225_16F3_HC | . | . | . | . | . | . | . | . | . | . | . | . | . | . |
| 21-225_17D3_HC | . | . | . | . | . | . | . | . | . | . | . | . | . | . |
| 21-225_18E10_HC | . | . | . | . | . | . | . | . | . | . | . | . | . | . |
| 21-225_195E9_HC | . | . | . | . | . | . | . | . | . | . | . | . | . | . |
| 21-225_21D12_HC | . | . | . | . | . | . | . | . | . | . | . | . | . | . |
| 21-225_31D12_LC1_HC | . | . | . | . | . | . | . | . | . | . | . | . | . | . |
| 21-225_31D12_LC2_HC | . | . | . | . | . | . | . | . | . | . | . | . | . | . |
| 21-225_33E6_HC | . | . | . | . | . | . | . | . | . | . | . | . | . | . |
| 21-225_35E1_HC | . | . | . | . | . | . | . | . | . | . | . | . | . | . |
| 21-225_4B3_HC | . | . | . | . | . | . | . | . | . | . | . | . | . | . |
| 21-225_68G6_HC | . | . | . | . | . | . | . | . | . | . | . | . | . | . |
| 21-225_69B11_HC | . | . | . | . | . | . | . | . | . | . | . | . | . | . |
| 21-225_71D4_HC | . | . | . | . | . | . | . | S | . | . | . | . | . | . |
| 21-225_72B4_HC | . | . | . | . | . | . | . | . | . | . | . | . | . | . |
| 21-225_9G9_HC | . | . | . | . | . | . | . | . | . | . | . | . | . | . |

**FIGURE 57**

Table 72.      Consensus 38 - VH1|1-02/D4|4-23|RF2/JH6 (SEQ ID NO: 50289):

QVQLVQS-GAEVKKPGASVKVSCKASG-YTFT<u>G-----YYIH</u>WVRQAPGQGLEWMG<u>WINPY---</u>
<u>SGDTNYAQKFQG</u>RVTMTRDTSISTAYMELSRLRFDDTAVFYCAR<u>DWGGYSS---------------YYYGMDV</u>WGQGTTVTVSS wherein:

I in position 41 can be substituted with T

D in position 67 can be substituted with G

N in position 69 can be substituted with K

Y in position 70 can be substituted with S

Y in position 134 can be substituted with F

Gly Tyr Tyr Xaa His (SEQ ID NO: 50061)

Trp Ile Asn Pro Tyr Ser Gly Xaa Thr Xaa Xaa Ala Gln Lys Phe Gln Gly (SEQ ID NO: 50062)

Asp Trp Gly Gly Tyr Ser Ser Tyr Tyr Xaa Gly Met Asp Val (SEQ ID NO: 50236)

Gly Tyr Tyr Ile His (SEQ ID NO: 50537)

Trp Ile Asn Pro Tyr Ser Gly Asp Thr Asn Tyr Ala Gln Lys Phe Gln Gly (SEQ ID NO: 50538)

Asp Trp Gly Gly Tyr Ser Ser Tyr Tyr Tyr Gly Met Asp Val (SEQ ID NO: 50539)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | Q | S | - | G | A | E | V | K | K | P | G | A | S | V | K | V | S | C | K | A | S |
| 21-225_224A7_HC | . | . | . | . | . | . | . | . | . | . | . | . | . | . | . | . | . | . | . | . | . | . | . | . | . | . |
| 21-225_224C11_HC | . | . | . | . | . | . | . | . | . | . | . | . | . | . | . | . | . | . | . | . | . | . | . | . | . | . |
| 21-225_224G1_HC | . | . | . | . | . | . | . | . | . | . | . | . | . | . | . | . | . | . | . | . | . | . | . | . | . | . |
| 21-225_224H11_HC | . | . | . | . | . | . | . | . | . | . | . | . | . | . | . | . | . | . | . | . | . | . | . | . | . | . |
| 21-225_225G4_HC | . | . | . | . | . | . | . | . | . | . | . | . | . | . | . | . | . | . | . | . | . | . | . | . | . | . |
| 21-225_226E11_HC | . | . | . | . | . | . | . | . | . | . | . | . | . | . | . | . | . | . | . | . | . | . | . | . | . | . |
| 21-225_226F10_HC | . | . | . | . | . | . | . | . | Q | . | . | . | . | L | R | . | . | . | . | . | . | . | . | . | . | . |
| 21-225_226F9_HC | . | . | . | . | . | . | . | . | . | . | . | . | . | . | . | . | . | . | . | . | . | . | . | . | . | . |
| 21-225_226G8_HC | . | . | . | . | . | . | . | . | . | . | . | . | . | . | . | . | . | . | . | . | . | . | . | . | . | . |
| 21-225_226H12_HC | . | . | . | . | . | . | . | . | . | . | . | . | . | . | . | . | . | . | . | . | . | . | . | . | . | . |
| 21-225_226H9_HC | . | . | . | . | . | . | . | . | . | . | . | . | . | . | . | . | . | . | . | . | . | . | . | . | . | . |
| 21-225_227A8_HC | . | . | . | . | . | . | . | . | . | . | . | . | . | . | . | . | . | . | . | . | . | . | . | . | . | . |
| 21-225_227C1_HC | . | . | . | . | . | . | . | . | . | . | . | . | . | . | . | . | . | . | . | . | . | . | . | . | . | . |
| 21-225_227F2_HC | . | M | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | Y | T | F | T | G | - | - | - | - | - | Y | Y | I | H | W | V | R | Q | A | P | G | Q | G | L |
| 21-225_224A7_HC | | . | . | . | . | . | . | . | . | . | . | . | . | . | . | . | . | . | . | . | . | . | . | . | . | . |
| 21-225_224C11_HC | | . | . | . | . | . | . | . | . | . | . | . | . | . | . | . | . | . | . | . | . | . | . | . | . | . |
| 21-225_224G1_HC | | . | . | . | . | . | . | . | . | . | . | . | . | . | . | . | . | . | . | . | . | . | . | . | . | . |
| 21-225_224H11_HC | | . | . | . | . | . | . | . | . | . | . | . | . | . | . | . | . | . | . | . | T | . | . | . | . | . |
| 21-225_225G4_HC | | . | . | . | . | . | . | . | . | . | . | . | . | . | . | . | . | . | . | . | . | . | . | . | . | . |
| 21-225_226E11_HC | | . | . | . | . | . | . | . | . | . | . | . | . | . | . | . | . | . | . | . | . | . | . | . | . | . |
| 21-225_226F10_HC | | . | . | . | . | . | . | . | . | . | . | . | . | . | . | . | . | . | . | . | . | . | . | . | . | . |
| 21-225_226F9_HC | | . | . | . | . | . | . | . | . | . | . | . | . | . | . | . | . | . | . | . | . | . | . | . | . | . |
| 21-225_226G8_HC | | . | . | . | . | . | . | . | . | . | . | . | . | . | . | . | . | . | . | . | . | . | . | . | . | . |
| 21-225_226H12_HC | | . | . | . | . | . | . | . | . | . | . | . | . | . | . | . | . | . | . | . | . | . | . | . | . | . |
| 21-225_226H9_HC | | . | . | . | . | . | . | . | . | . | . | . | . | . | . | . | . | . | . | . | . | . | . | . | . | . |
| 21-225_227A8_HC | | . | . | . | . | . | . | . | . | . | . | . | . | . | . | . | . | . | . | . | . | . | . | . | . | . |
| 21-225_227C1_HC | | . | . | . | . | . | . | . | . | . | . | . | . | T | . | . | . | . | . | . | . | . | . | . | . | . |
| 21-225_227F2_HC | | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | M | G | W | I | N | P | Y | - | - | - | S | G | D | T | N | Y | A | Q | K | F | Q | G | R | V |
| 21-225_224A7_HC | . | . | . | . | . | . | . | . | . | . | . | . | . | . | . | . | . | . | . | . | . | . | . | . | . | . |
| 21-225_224C11_HC | . | . | . | . | . | . | . | . | . | . | . | . | . | . | . | . | . | . | . | . | . | . | . | . | . | . |
| 21-225_224G1_HC | . | . | . | . | . | . | . | . | . | . | . | . | . | . | . | . | . | . | . | . | . | . | . | . | . | . |
| 21-225_224H11_HC | . | . | . | . | . | . | . | . | . | . | . | . | . | . | . | . | . | . | . | . | . | . | . | . | . | . |
| 21-225_225G4_HC | . | . | . | . | . | . | . | . | . | . | . | . | . | . | . | . | . | . | . | . | . | . | . | . | . | . |
| 21-225_226E11_HC | . | . | . | . | . | . | . | . | . | . | . | . | . | . | . | . | . | . | . | . | . | . | . | . | . | . |
| 21-225_226F10_HC | . | . | . | . | . | . | . | . | . | . | . | . | . | . | . | . | . | . | . | . | . | . | . | . | . | . |
| 21-225_226F9_HC | . | . | . | . | . | . | . | . | . | . | . | . | . | . | . | . | × | . | . | . | . | . | . | . | . | . |
| 21-225_226G8_HC | . | . | . | . | . | . | . | . | . | . | . | . | . | . | . | . | × | . | . | . | . | . | . | . | . | . |
| 21-225_226H12_HC | . | . | . | . | . | . | . | . | . | . | . | . | . | . | . | . | . | . | . | . | . | . | . | . | . | . |
| 21-225_226H9_HC | . | . | . | . | . | . | . | . | . | . | . | . | . | . | . | . | . | S | . | . | . | . | . | . | . | . |
| 21-225_227A8_HC | . | . | . | . | . | . | . | . | . | . | . | . | . | . | . | . | . | . | . | . | . | . | . | . | . | . |
| 21-225_227C1_HC | . | . | . | . | . | . | . | . | . | . | . | . | . | . | G | . | . | . | . | . | . | . | . | . | . | . |
| 21-225_227F2_HC | . | . | . | . | . | . | . | . | . | . | . | . | . | . | . | . | × | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | M | T | R | D | T | S | I | S | T | A | Y | M | E | L | S | R | L | R | F | D | D | T | A | V | F |
| 21-225_224A7_HC | | | | | | | | | T | | | | | | | | | | | | | | | | | |
| 21-225_224C11_HC | | | | | | | | | | | | | | | | B | | | | | | | | | | |
| 21-225_224G1_HC | | | | | | | | | | | | | | | | | | | | | | | | | | |
| 21-225_224H11_HC | | | | | | | | | | | | | | | | B | | | | | | | | | | |
| 21-225_225G4_HC | | | | | | | | | | | | | | | | B | | | | | | | | | | |
| 21-225_226E11_HC | | | | | | | | | | | | | | | | | | | | | | | | | | |
| 21-225_226F10_HC | | | | | | | | V | | | | | | | | | | | L | | | | | | | |
| 21-225_226F9_HC | | | | | A | | | | | | | | | | | | | | | | | | | | | |
| 21-225_226G8_HC | | | | | | | | | | | | | | | | | | | | | | | | | | |
| 21-225_226H12_HC | | | | | | | | | | | | | | | | B | | | | | | | | | | |
| 21-225_226H9_HC | | | | | | | | | | | | | | | | | | | | | | | | | | |
| 21-225_227A8_HC | | | | | | | | V | | | | | | | | | | | | | | | | | | |
| 21-225_227C1_HC | | | | | | | | | | | | | | | | | | | | | | | | | | Y |
| 21-225_227F2_HC | | | | | | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | W | G | G | Y | S | S | - | - | - | - | - | - | - | - | - | - | - | - | - | - | - |
| 21-225_224A7_HC | . | . | . | . | . | . | . | . | . | . | . | . | . | . | . | . | . | . | . | . | . | . | . | . | . | . |
| 21-225_224C11_HC | . | . | . | . | . | . | . | . | . | . | . | . | . | . | . | . | . | . | . | . | . | . | . | . | . | . |
| 21-225_224G1_HC | . | . | . | . | . | . | . | . | . | . | . | . | . | . | . | . | . | . | . | . | . | . | . | . | . | . |
| 21-225_224H11_HC | . | . | . | . | . | . | . | . | . | . | . | . | . | . | . | . | . | . | . | . | . | . | . | . | . | . |
| 21-225_225G4_HC | . | . | . | . | . | . | . | . | . | . | . | . | . | . | . | . | . | . | . | . | . | . | . | . | . | . |
| 21-225_226E11_HC | . | . | . | . | . | . | . | . | . | . | . | . | . | . | . | . | . | . | . | . | . | . | . | . | . | . |
| 21-225_226F10_HC | . | . | . | . | . | . | . | . | . | . | . | . | . | . | . | . | . | . | . | . | . | . | . | . | . | . |
| 21-225_226F9_HC | . | . | . | . | . | . | . | . | . | . | . | . | . | . | . | . | . | . | . | . | . | . | . | . | . | . |
| 21-225_226G8_HC | . | . | . | . | . | . | . | . | . | . | . | . | . | . | . | . | . | . | . | . | . | . | . | . | . | . |
| 21-225_226H12_HC | . | . | . | . | . | . | . | . | . | . | . | . | . | . | . | . | . | . | . | . | . | . | . | . | . | . |
| 21-225_226H9_HC | . | . | . | . | . | . | . | . | . | . | . | . | . | . | . | . | . | . | . | . | . | . | . | . | . | . |
| 21-225_227A8_HC | . | . | . | . | . | . | . | . | . | . | . | . | . | . | . | . | . | . | . | . | . | . | . | . | . | . |
| 21-225_227C1_HC | . | . | . | . | . | . | . | . | . | . | . | . | . | . | . | . | . | . | . | . | . | . | . | . | . | . |
| 21-225_227F2_HC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | Y | Y | Y | G | M | D | V | W | G | Q | G | T | T | V | T | V | S | S |
| 21-225_224A7_HC | | | | | | | | | | | | | | | | | | | |
| 21-225_224C11_HC | | | | | | | | | | | | | | | | | | | |
| 21-225_224G1_HC | | | | | | | | | | | | | | | | | | | |
| 21-225_224H11_HC | | | | | | | | | | | | | | | | | | | |
| 21-225_225G4_HC | | | | | | | | | | | | | | | | | | | |
| 21-225_226E11_HC | | | | | | | | | | | | | | | | | | | |
| 21-225_226F10_HC | | | | | | | | | | | | | | | | | | | |
| 21-225_226F9_HC | | | | | | | | | | | | | | | | | | | |
| 21-225_226G8_HC | | | | | | | | | | | | | | | | | | | |
| 21-225_226H12_HC | | | | | | | | | | | | | | | | | | | |
| 21-225_226H9_HC | | | | | | | | | | | | | | | | | | | |
| 21-225_227A8_HC | | | | | | | | | | | | | | | | | | | |
| 21-225_227C1_HC | | | | | | | | | | | | | | | | | | | |
| 21-225_227F2_HC | | | | | | | | | | | | | | | | | | | |

**FIGURE 57**

Table 73.     Consensus 39 - VH3|3-21/D7|7-27|RF1/JH4 (SEQ ID NO: 50290):

EVQLVES-GGGLVKPGGSLRLSCAASG-FTFSS-----YSMNWVRQAPGKGLEWVSSISGS---
SSYIYYADSVKGRFTISRDNAKNSLYLQMNSLRAEDTAVYYCARLT-----------------------FDYWGQGTLVTVSS wherein:

S in position 33 can be substituted with T

Y in position 39 can be substituted with F

S in position 40 can be substituted with T, N or G

N in position 42 can be substituted with S or I

G in position 60 can be substituted with S

S in position 61 can be substituted with T

S in position 65 can be substituted with I or N

S in position 66 can be substituted with N, T or Y

I in position 68 can be substituted with M or S

Y in position 69 can be substituted with N

A in position 71 can be substituted with T

Xaa Xaa Xaa Met Xaa (SEQ ID NO: 50063)

Ser Ile Ser Xaa Xaa Xaa Xaa Tyr Xaa Xaa Tyr Xaa Asp Ser Val Lys Gly (SEQ ID NO: 50064)

Leu Thr Phe Asp Tyr (SEQ ID NO: 50065)

Ser Tyr Ser Met Asn (SEQ ID NO: 50540)

Ser Ile Ser Gly Ser Ser Ser Tyr Ile Tyr Tyr Ala Asp Ser Val Lys Gly (SEQ ID NO: 50541)

Leu Thr Phe Asp Tyr (SEQ ID NO: 50542)

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | V | E | S | - | G | G | G | L | V | K | P | G | G | S | L | R | L | S | C | A | A | S |
| 21-225_10C7_HC | | | | | | | | | | | | | | | | | | | | | | | | | | |
| 21-225_12D2_HC | | | | | | | | | | | | | | | | | | | | | | | | | | |
| 21-225_151F1_HC | | | | | | | | | | | | | | | | | | | | | | | | | | |
| 21-225_166H12_HC | | | | | | | | | | A | | | | | | | | | | | | | | | | |
| 21-225_227F11_HC | | | | | | | | | | | | | | | | | | | | | | | | | V | |
| 21-225_26G1_HC | | | | | | | | | | | | | | | | | | | | | | | | | | |
| 21-225_2B1_HC | | | | | | | | | | | | | | | | | | | | | | | | | | |
| 21-225_33B7_HC | | | | | | | | | | | | | | | | | | | | | | | | | | |
| 21-225_34C11_HC | | | | | | | | | | | | | | | | | | | | | | | | | | |
| 21-225_43A4_HC | | | | | | | | | | | | | | | | | | | | | | | | | | |
| 21-225_45D9_HC | | | | | | | | | | | | | | | | | | | | | | | | | | |
| 21-225_54H3_HC | | | | | | | | | | | | | | | | | | | | | | | | | | |
| 21-225_63H8_HC | | | | | | | | | | | | | | | | | | | | | | | | | | |
| 21-225_8D8_HC | | | | | | | | | | | | | | | | | | | | | | | | | | |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | H_CDR1 | | | | | | | | | | | | | | | | | H_FR2 | | | |
| CONSENSUS | G | - | F | T | F | S | S | - | - | - | - | - | Y | S | M | N | W | V | R | Q | A | P | G | K | G | L |
| 21-225_10C7_HC | | | . | . | . | | . | . | . | . | . | | . | . | . | . | . | | . | . | . | . | . | . | | . |
| 21-225_12D2_HC | | . | . | . | | . | . | . | . | . | . | | . | . | . | . | . | | . | . | . | . | . | . | . | . |
| 21-225_151F1_HC | | Y | . | . | S | . | . | . | . | . | . | S | . | . | . | . | . | | . | . | . | . | . | . | . | . |
| 21-225_166H12_HC | | . | . | . | . | | . | . | . | . | . | | . | . | . | S | . | | . | . | . | . | . | . | . | . |
| 21-225_227F11_HC | | . | . | . | . | T | . | . | . | . | . | | . | . | . | . | . | | . | . | . | . | . | . | . | . |
| 21-225_26G1_HC | | . | . | . | . | | . | . | . | . | . | | G | . | . | . | . | | . | . | . | . | . | . | . | . |
| 21-225_2B1_HC | | . | . | . | . | | . | . | . | . | . | | . | . | . | . | . | | . | . | . | . | . | . | . | . |
| 21-225_33B7_HC | | . | . | . | . | | . | . | . | . | . | | N | . | . | . | . | | . | . | . | . | . | . | . | . |
| 21-225_34C11_HC | | . | . | . | . | | . | . | . | . | . | | T | . | S | . | . | | . | . | . | . | . | . | . | . |
| 21-225_43A4_HC | | . | . | . | . | | . | . | . | . | . | | T | . | . | . | . | | . | . | V | . | . | . | . | . |
| 21-225_45D9_HC | | . | . | . | . | | . | . | . | . | . | | T | . | . | . | . | | . | . | V | . | . | . | . | . |
| 21-225_54H3_HC | | . | . | . | . | | . | . | . | . | . | | . | . | I | . | . | | . | . | . | . | . | . | . | . |
| 21-225_63H8_HC | | . | . | . | . | | . | . | . | . | . | | N | . | . | . | . | | . | . | . | . | . | . | . | . |
| 21-225_8D8_HC | | . | . | . | . | | . | . | . | . | . | | . | . | . | . | . | | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | S | I | S | G | S | - | - | - | S | S | Y | I | Y | Y | A | D | S | V | K | G | R | F |
| 21-225_10C7_HC | | | | | | | | S | | | | | N | N | | | | | | | | | | | | |
| 21-225_12D2_HC | | | | | | | | S | | | | | | | | | | | | | | | | | | |
| 21-225_151F1_HC | | | | | | | | S | | | | | | Y | | | | | | | | | | | | |
| 21-225_166H12_HC | G | | | | | | | | | | | | | | | S | | | | | | | | | | |
| 21-225_227F11_HC | | | | | | | | S | | | | | | N | | M | | | | | | | | | | |
| 21-225_26G1_HC | | | | | | | | | | | | | | | | | | | | | | | | | | |
| 21-225_2B1_HC | | | | | | | | S | | | | | | | | | | | | | | | | | | |
| 21-225_33B7_HC | | | | | | | | | | | | | | | | | | | | | | | | | | |
| 21-225_34C11_HC | | | | | | | | | | | | | | N | | | | | | | | | | | | |
| 21-225_43A4_HC | | | | | | | | | | | | | | T | | | | | | | | | | | | |
| 21-225_45D9_HC | | | | | | | | | | | | | | T | | | | | | | | | | | | |
| 21-225_54H3_HC | | | | | | T | | | | | | | | | | | | | | | | | | | | |
| 21-225_63H8_HC | | | | | | | | | | | | | | T | | M | N | | T | | | | | | | |
| 21-225_8D8_HC | | | | | | | | S | | | | | | | | | | | | | | | | | | |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | A | K | N | S | L | Y | L | Q | M | N | S | L | R | A | E | D | T | A | V | Y |
| 21-225_10C7_HC | | . | . | . | . | . | | . | . | . | . | . | . | . | . | . | | . | | . | . | . | S | | . | . |
| 21-225_12D2_HC | | . | . | . | . | . | . | . | . | . | . | . | . | . | . | . | | . | | . | . | . | . | | . | . |
| 21-225_151F1_HC | | . | . | . | . | . | . | . | . | . | . | . | . | . | . | . | | . | | . | . | . | . | | . | . |
| 21-225_166H12_HC | | . | . | . | . | . | . | . | . | . | . | . | . | . | . | . | | . | | . | . | . | . | | . | . |
| 21-225_227F11_HC | | . | . | . | . | . | . | . | . | . | . | . | . | . | . | . | | . | | . | . | . | S | | . | . |
| 21-225_26G1_HC | | . | . | . | . | . | . | . | . | . | . | . | . | . | . | . | | . | | . | . | . | . | | . | . |
| 21-225_2B1_HC | | . | . | . | . | . | . | . | . | . | . | . | . | . | . | . | | . | | . | . | . | . | | . | . |
| 21-225_33B7_HC | | . | . | . | . | . | . | . | . | . | . | . | . | . | . | . | | . | | . | . | . | . | | . | . |
| 21-225_34C11_HC | | . | . | . | . | . | . | . | . | . | . | . | . | . | . | . | | . | | . | . | . | . | | . | . |
| 21-225_43A4_HC | | . | . | . | O | . | | Q | . | . | . | . | . | . | . | . | | . | | G | . | . | . | | . | . |
| 21-225_45D9_HC | | . | . | . | O | . | | Q | . | . | . | . | . | . | . | . | | . | | G | . | . | . | | . | . |
| 21-225_54H3_HC | | . | . | . | . | . | . | . | . | . | . | . | . | . | . | . | | . | | . | . | . | . | | . | . |
| 21-225_63H8_HC | | . | . | . | . | . | . | . | . | . | . | . | . | . | . | . | | . | | . | . | . | . | | . | . |
| 21-225_8D8_HC | | . | . | . | . | . | . | . | . | . | . | . | . | . | . | . | | . | | . | . | . | . | | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | H_CDR3 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| CONSENSUS | Y | C | A | R | L | T | - | - | - | - | - | - | - | - | - | - | - | - | - | - | - | - | - | - | - | - |
| 21-225_10C7_HC | | | . | . | . | G | . | . | . | . | . | . | . | . | . | . | . | . | . | . | . | . | . | . | . | . |
| 21-225_12D2_HC | | | . | . | . | G | . | . | . | . | . | . | . | . | . | . | . | . | . | . | . | . | . | . | . | . |
| 21-225_151F1_HC | | | . | . | D | . | P | . | . | . | . | . | . | . | . | . | . | . | . | . | . | . | . | . | . | . |
| 21-225_166H12_HC | | | . | . | . | G | . | . | . | . | . | . | . | . | . | . | . | . | . | . | . | . | . | . | . | . |
| 21-225_227F11_HC | | | . | . | . | G | . | . | . | . | . | . | . | . | . | . | . | . | . | . | . | . | . | . | . | . |
| 21-225_26G1_HC | | | . | . | . | T | . | . | . | . | . | . | . | . | . | . | . | . | . | . | . | . | . | . | . | . |
| 21-225_2B1_HC | | | . | . | . | G | . | . | . | . | . | . | . | . | . | . | . | . | . | . | . | . | . | . | . | . |
| 21-225_33B7_HC | | | . | . | . | N | G | . | . | . | . | . | . | . | . | . | . | . | . | . | . | . | . | . | . | . |
| 21-225_34C11_HC | | | . | . | . | . | G | . | . | . | . | . | . | . | . | . | . | . | . | . | . | . | . | . | . | . |
| 21-225_43A4_HC | | | . | . | V | . | S | . | . | . | . | . | . | . | . | . | . | . | . | . | . | . | . | . | . | . |
| 21-225_45D9_HC | | | . | . | V | . | S | . | . | . | . | . | . | . | . | . | . | . | . | . | . | . | . | . | . | . |
| 21-225_54H3_HC | | | . | G | M | . | T | . | . | . | . | . | . | . | . | . | . | . | . | . | . | . | . | . | . | . |
| 21-225_63H8_HC | | | . | . | . | . | N | . | . | . | . | . | . | . | . | . | . | . | . | . | . | . | . | . | . | . |
| 21-225_8D8_HC | | | . | . | . | G | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | - | F | D | Y | W | G | Q | G | T | L | V | T | V | S | S |
| 21-225_10C7_HC | | . | . | . | . | - | V | . | . | . | . | . | . | . | . | . | . | . | . |
| 21-225_12D2_HC | . | . | . | . | . | - | . | . | . | . | . | . | . | . | . | A | . | . | . |
| 21-225_151F1_HC | . | . | . | . | . | L | V | . | . | . | . | . | . | . | . | . | . | . | . |
| 21-225_166H12_HC | . | . | . | . | . | . | . | . | . | . | . | . | . | . | . | . | . | . | . |
| 21-225_227F11_HC | . | . | . | . | . | - | V | . | . | . | . | . | . | . | . | . | . | . | . |
| 21-225_26G1_HC | . | . | . | . | . | . | F | . | . | . | . | . | . | . | . | . | . | . | . |
| 21-225_2B1_HC | . | . | . | . | . | - | N | . | . | . | . | . | . | . | . | . | . | . | . |
| 21-225_33B7_HC | . | . | . | . | . | . | . | . | . | . | . | . | . | . | . | . | . | . | . |
| 21-225_34C11_HC | . | . | . | . | . | . | . | . | . | . | . | . | . | . | . | . | . | . | . |
| 21-225_43A4_HC | . | . | . | . | . | . | . | . | . | . | . | . | A | . | . | . | . | . | . |
| 21-225_45D9_HC | . | . | . | . | . | . | . | . | . | . | . | . | A | . | . | . | . | . | . |
| 21-225_54H3_HC | . | . | . | . | . | - | V | I | . | . | . | . | . | . | . | . | . | . | . |
| 21-225_63H8_HC | . | . | . | . | . | . | . | . | . | . | . | . | . | . | . | . | . | . | . |
| 21-225_8D8_HC | . | . | . | . | . | - | N | . | . | . | . | . | . | . | . | . | . | . | . |

**FIGURE 57**

Table 74.  Consensus 40 - VH3|3-33/D3|3-9|RF2/JH4 (SEQ ID NO: 50291):

QVQLVES-GGGVVQPGRSLRLSCAASG-FTFST-----YGMHWVRQAPGKGLEWVAIIWYD---
GTNKYYADSVKGRFTISRDNSKNTLYLQLNSLRAEDTAVYYCARDPLRGYN----------------DPVMDYWGQGTLVTVSS wherein:

T in position 33 can be substituted with S

I in position 57 can be substituted with V

T in position 66 can be substituted with S

K in position 75 can be substituted with R

M in position 136 can be substituted with L

Xaa Tyr Gly Met His (SEQ ID NO: 50066)

Xaa Ile Trp Tyr Asp Gly Xaa Asn Lys Tyr Tyr Ala Asp Ser Val Xaa Gly (SEQ ID NO: 50067)

Asp Pro Leu Arg Gly Tyr Asn Asp Pro Val Xaa Asp Tyr (SEQ ID NO: 50068)

Thr Tyr Gly Met His (SEQ ID NO: 50543)

Ile Ile Trp Tyr Asp Gly Thr Asn Lys Tyr Tyr Ala Asp Ser Val Lys Gly (SEQ ID NO: 50544)

Asp Pro Leu Arg Gly Tyr Asn Asp Pro Val Met Asp Tyr (SEQ ID NO: 50545)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A | S |
| 21-225_169B1_HC | . | . | . | . | . | . | . | . | . | . | . | . | . | . | . | . | . | . | . | . | . | . | . | . | . | . |
| 21-225_169F9_HC | . | . | . | . | . | . | . | . | . | . | . | . | . | . | . | . | . | . | . | . | . | . | . | . | . | . |
| 21-225_170D11_HC | . | . | . | . | . | . | . | . | . | . | . | . | . | . | . | . | . | . | . | . | . | . | . | V | . | . |
| 21-225_170D5_HC | . | . | . | . | . | . | . | . | . | . | . | . | . | . | . | . | . | . | . | . | . | . | . | . | . | . |
| 21-225_170G4_HC | . | . | . | . | . | . | . | . | . | . | . | . | . | . | . | . | . | . | . | . | . | . | . | T | . | . |
| 21-225_170G5_HC | . | . | . | . | . | . | . | . | . | . | . | . | . | . | . | . | G | . | . | . | . | . | . | . | . | . |
| 21-225_171A4_HC | . | . | . | . | . | . | . | . | . | . | . | . | . | . | . | . | G | . | . | . | . | . | . | . | . | . |
| 21-225_171C3_HC | . | . | . | . | . | . | . | . | . | . | . | . | . | . | . | . | G | . | . | . | . | . | . | . | . | . |
| 21-225_172B3_HC | . | . | . | . | . | . | . | . | . | . | . | . | . | . | . | . | . | P | . | . | . | . | . | . | . | . |
| 21-225_172G8_HC | . | . | . | M | . | . | . | . | . | . | . | . | . | . | . | . | . | . | . | . | . | . | . | . | . | . |
| 21-225_173H12_HC | . | . | . | . | . | . | . | . | . | . | . | . | . | . | . | . | . | . | . | . | . | . | . | . | . | . |
| 21-225_175G1_HC | . | . | . | . | . | . | . | . | . | . | . | . | . | . | . | . | G | . | . | . | . | . | . | . | . | . |
| 21-225_176B11_HC | . | . | . | . | . | . | . | . | . | . | . | . | . | . | . | . | . | . | . | . | . | . | . | . | . | . |
| 21-225_178B10_HC | . | . | . | . | . | . | . | . | . | . | . | . | . | . | . | . | . | . | . | . | . | . | . | V | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | | H_FR2 | | |
| CONSENSUS | G | - | F | T | F | S | T | - | - | - | - | - | Y | G | M | H | W | V | R | Q | A | P | G | K | G | L |
| 21-225_169B1_HC | . | . | . | . | . | . | S | . | . | . | . | . | . | . | . | . | . | . | . | . | . | . | . | . | . | . |
| 21-225_169F9_HC | . | . | . | . | . | . | . | . | . | . | . | . | . | . | . | . | . | . | . | . | . | . | . | . | . | . |
| 21-225_170D11_HC | . | . | . | . | . | . | S | . | . | . | . | . | . | . | . | . | . | . | . | . | . | . | . | . | . | . |
| 21-225_170D5_HC | . | . | . | . | . | . | S | . | . | . | . | . | . | . | . | . | . | . | . | . | . | . | . | . | . | . |
| 21-225_170G4_HC | . | . | . | . | . | . | . | . | . | . | . | . | . | . | . | . | . | . | . | . | . | . | . | . | . | . |
| 21-225_170G5_HC | . | . | . | . | . | . | . | . | . | . | . | . | . | . | . | . | . | . | . | . | . | . | . | . | . | . |
| 21-225_171A4_HC | . | . | . | . | . | . | . | . | . | . | . | . | . | . | . | . | . | . | . | . | . | . | . | . | . | . |
| 21-225_171C3_HC | . | . | . | . | . | . | . | . | . | . | . | . | . | . | . | . | . | . | . | . | . | . | . | . | . | . |
| 21-225_172B3_HC | . | . | . | . | . | . | . | . | . | . | . | . | . | . | . | . | . | . | . | . | . | . | . | . | . | . |
| 21-225_172G8_HC | . | . | . | . | . | . | . | . | . | . | . | . | . | . | . | . | . | . | . | . | . | . | . | . | . | . |
| 21-225_173H12_HC | . | . | . | . | . | . | S | . | . | . | . | . | . | . | . | . | . | . | . | . | . | . | . | . | . | . |
| 21-225_175G1_HC | . | . | . | . | . | . | . | . | . | . | . | . | . | . | . | . | . | . | . | . | . | . | . | . | . | . |
| 21-225_176B11_HC | . | . | . | . | . | . | . | . | . | . | . | . | . | . | . | . | . | . | . | . | . | . | . | . | . | . |
| 21-225_178B10_HC | . | . | . | S | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | I | I | W | Y | D | - | - | - | G | T | N | K | Y | Y | A | D | S | V | K | G | R | F |
| 21-225_169B1_HC | . | . | . | . | V | . | . | . | . | . | . | . | . | S | . | . | . | . | . | . | . | . | R | . | . | . |
| 21-225_169F9_HC | . | . | . | S | . | . | . | . | . | . | . | . | . | . | . | . | . | . | . | . | . | . | . | . | . | . |
| 21-225_170D11_HC | . | . | . | . | . | . | . | . | . | . | . | . | . | . | . | . | . | . | . | . | . | . | . | . | . | . |
| 21-225_170D5_HC | . | . | . | . | . | . | . | . | . | . | . | . | . | . | . | . | . | . | . | . | . | . | . | . | . | . |
| 21-225_170G4_HC | . | . | S | . | . | . | . | . | . | . | . | . | . | . | . | . | . | . | . | . | . | . | . | . | . | . |
| 21-225_170G5_HC | . | . | T | . | . | . | . | . | . | . | . | . | . | . | . | . | . | . | . | . | . | . | . | . | . | . |
| 21-225_171A4_HC | . | . | . | . | . | . | . | . | . | . | . | . | . | S | . | . | . | . | . | . | . | . | . | . | . | . |
| 21-225_171C3_HC | . | . | . | T | . | . | . | . | . | . | . | . | . | . | . | . | . | . | . | . | . | . | . | . | . | . |
| 21-225_172B3_HC | . | . | . | . | V | . | . | . | . | . | . | . | . | S | . | . | . | . | . | . | . | . | . | . | . | . |
| 21-225_172G8_HC | . | M | . | . | . | . | . | . | . | . | . | . | . | . | . | . | . | . | . | . | . | . | . | . | . | . |
| 21-225_173H12_HC | . | . | . | . | . | . | . | . | . | . | . | . | . | . | . | . | . | . | . | . | . | . | . | . | . | . |
| 21-225_175G1_HC | . | . | . | . | V | . | . | . | . | . | . | . | . | S | . | . | . | . | . | . | . | . | . | . | . | . |
| 21-225_176B11_HC | . | . | . | S | . | . | . | . | . | . | . | . | . | . | . | . | . | . | . | . | . | . | . | . | . | . |
| 21-225_178B10_HC | . | . | M | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | H_FR3 | | | | | | | | | | | | | |
| CONSENSUS | T | I | S | R | D | N | S | K | N | T | L | Y | L | Q | L | N | S | L | R | A | E | D | T | A | V | Y |
| 21-225_169B1_HC | . | . | . | . | . | . | . | . | . | . | . | . | . | . | M | . | . | . | . | . | . | . | . | . | . | . |
| 21-225_169F9_HC | . | . | . | . | . | . | . | . | . | . | . | . | . | . | . | . | . | . | . | . | . | . | . | . | . | . |
| 21-225_170D11_HC | . | . | . | . | . | . | . | . | . | . | . | . | . | . | . | . | . | . | . | . | . | . | . | . | . | . |
| 21-225_170D5_HC | . | . | . | . | . | . | . | . | . | . | . | . | . | . | . | . | . | . | . | . | . | . | . | . | . | . |
| 21-225_170G4_HC | . | . | . | . | . | . | . | . | . | . | . | F | . | . | . | . | . | . | . | . | . | . | . | . | M | . |
| 21-225_170G5_HC | . | . | . | . | . | . | . | . | S | . | . | . | . | . | M | . | . | . | . | . | . | . | . | . | . | . |
| 21-225_171A4_HC | . | . | . | . | . | . | . | . | . | . | . | . | . | . | M | . | . | . | . | . | . | . | . | . | . | . |
| 21-225_171C3_HC | . | . | . | . | . | . | . | . | . | . | . | . | . | . | M | . | . | . | . | . | . | . | . | . | . | . |
| 21-225_172B3_HC | . | . | . | . | . | . | . | . | . | . | . | . | . | . | M | . | . | . | . | . | . | . | . | . | . | . |
| 21-225_172G8_HC | . | . | . | . | . | . | . | . | . | . | . | . | . | . | . | . | . | . | . | . | . | . | . | . | . | . |
| 21-225_173H12_HC | . | . | . | . | . | . | . | . | . | . | . | . | . | . | . | . | . | . | . | . | . | . | . | . | . | . |
| 21-225_175G1_HC | . | . | . | . | . | . | . | . | . | . | . | . | V | . | M | . | . | . | . | . | . | . | . | . | . | . |
| 21-225_176B11_HC | . | . | . | . | . | . | . | . | . | . | . | F | . | . | . | . | . | . | . | . | . | . | . | . | . | . |
| 21-225_178B10_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | P | L | R | G | Y | N | - | - | - | - | - | - | - | - | - | - | - | - | - | - | - |
| 21-225_169B1_HC | . | . | . | . | . | . | . | . | . | . | . | . | . | . | . | . | . | . | . | . | . | . | . | . | . | . |
| 21-225_169F9_HC | . | . | . | . | . | . | . | . | . | . | . | . | . | . | . | . | . | . | . | . | . | . | . | . | . | . |
| 21-225_170D11_HC | . | . | . | . | . | . | . | . | . | . | . | . | . | . | . | . | . | . | . | . | . | . | . | . | . | . |
| 21-225_170D5_HC | . | . | . | . | . | . | . | . | . | . | . | . | . | . | . | . | . | . | . | . | . | . | . | . | . | . |
| 21-225_170G4_HC | . | . | . | . | . | . | . | . | . | . | . | . | . | . | . | . | . | . | . | . | . | . | . | . | . | . |
| 21-225_170G5_HC | . | . | . | . | . | . | . | . | . | . | . | . | . | . | . | . | . | . | . | . | . | . | . | . | . | . |
| 21-225_171A4_HC | . | . | . | . | . | . | . | . | . | . | . | . | . | . | . | . | . | . | . | . | . | . | . | . | . | . |
| 21-225_171C3_HC | . | . | . | . | . | . | . | . | . | . | . | . | . | . | . | . | . | . | . | . | . | . | . | . | . | . |
| 21-225_172B3_HC | . | . | . | . | . | . | . | . | . | . | . | . | . | . | . | . | . | . | . | . | . | . | . | . | . | . |
| 21-225_172G8_HC | . | . | . | . | . | . | . | . | . | . | . | . | . | . | . | . | . | . | . | . | . | . | . | . | . | . |
| 21-225_173H12_HC | . | . | . | . | . | . | . | . | . | . | . | . | . | . | . | . | . | . | . | . | . | . | . | . | . | . |
| 21-225_175G1_HC | . | . | . | . | . | . | . | . | . | . | . | . | . | . | . | . | . | . | . | . | . | . | . | . | . | . |
| 21-225_176B11_HC | . | . | . | . | . | . | . | . | . | . | . | . | . | . | . | . | . | . | . | . | . | . | . | . | . | . |
| 21-225_178B10_HC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  |  |  |  |  |  | H_FR4 |  |  |  |  |  |
| CONSENSUS | - | - | D | P | V | M | D | Y | W | G | Q | G | T | L | V | T | V | S | S |
| 21-225_169B1_HC | . | | . | . | . | | | . | . | . | . | . | | . | . | . | . | . | |
| 21-225_169F9_HC | | . | . | . | . | | . | . | . | . | . | . | | . | . | . | . | | . |
| 21-225_170D11_HC | . | | . | . | . | | . | . | . | . | . | . | | . | . | . | . | . | . |
| 21-225_170D5_HC | . | | . | . | . | | | . | . | . | . | . | | . | . | . | . | . | |
| 21-225_170G4_HC | . | | . | . | . | | . | . | . | . | . | . | | . | . | . | . | . | |
| 21-225_170G5_HC | . | | . | . | . | | . | . | . | . | . | . | | . | . | . | . | . | |
| 21-225_171A4_HC | . | | . | . | . | | . | . | . | . | . | . | | . | . | . | . | . | |
| 21-225_171C3_HC | . | | . | . | . | | . | . | . | . | . | . | | . | . | . | . | . | |
| 21-225_172B3_HC | . | . | . | . | . | | . | . | . | . | . | . | | . | . | . | . | . | |
| 21-225_172G8_HC | . | . | . | . | . | | . | . | . | . | . | . | | . | . | . | . | . | |
| 21-225_173H12_HC | . | | . | . | . | | . | . | . | . | . | . | | . | . | . | . | . | |
| 21-225_175G1_HC | . | | . | . | . | | . | . | . | . | . | . | | . | . | . | . | . | |
| 21-225_176B11_HC | . | | . | . | . | L | . | . | . | . | . | . | | . | . | . | . | . | |
| 21-225_178B10_HC | . | | . | . | . | | . | . | . | . | . | . | | . | . | . | . | . | |

**FIGURE 57**

Table 75.    Consensus 41 - VH3|3-23/D4|4-17|RF2/JH5 (SEQ ID NO: 50292):

EVQLLES-GGGLVQPGGSLRLSCAASG-FTFS<u>S</u>-----<u>YAMS</u>WVRQAPGKGLEWVS<u>AISGR</u>---<u>GGNTF</u>YADSVKGRFTISRDNSKNTLYLQMNSLRAEDTAVYYCAK<u>RVTDYGG</u>----------------NDWFDPWGQGTLVTVSS wherein:

S in position 33 can be substituted with N

S in position 42 can be substituted with N or T

R in position 61 can be substituted with S

G in position 66 can be substituted with K

Xaa Tyr Ala Met Xaa (SEQ ID NO: 50069)

Ala Ile Ser Gly Xaa Gly Xaa Asn Thr Phe Tyr Ala Asp Ser Val Lys Gly (SEQ ID NO: 50070)

Arg Val Thr Asp Tyr Gly Gly Asn Asp Trp Phe Asp Pro (SEQ ID NO: 50071)

Ser Tyr Ala Met Ser (SEQ ID NO: 50546)

Ala Ile Ser Gly Arg Gly Gly Asn Thr Phe Tyr Ala Asp Ser Val Lys Gly (SEQ ID NO: 50547)

Arg Val Thr Asp Tyr Gly Gly Asn Asp Trp Phe Asp Pro (SEQ ID NO: 50548)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | |
| CONSENSUS | E | V | Q | L | L | E | S | - | G | G | G | L | V | Q | P | G | G | S | L | R | L | S | C | A | A | S |
| 21-225_146E9_HC | | | | | | | | | | | | | | | | | | | | | | | | | | |
| 21-225_146H1_HC | | | | | | | | | | | | | | | | | | | | | | | | | | |
| 21-225_148C4_HC | | | | | | | | | | | | | | | | | | | | | | | | | | |
| 21-225_148H9_HC | | | | | | | | | | | | | | | | | | | | | | | | | | |
| 21-225_149A3_HC | | | | | | | | | | | | | | | | | | | | | | | | | | |
| 21-225_150C11_HC | | | | | | | | | | | | | | | | | | | | | | | | | | |
| 21-225_150C12_HC | | | | | | | | | | | | | | | | | | | | | | | | | | |
| 21-225_151B12_HC | | | | | | | | | | | | | | | | | | | | | | | | | | |
| 21-225_152B11_HC | | | | | | | | | | | | | | | | | | | | | | | | | | |
| 21-225_152D2_HC | | | | | | | | | | | | | | | | | | | | | | | | | | |
| 21-225_152G4_HC | | | | | | | | | | | | | | | | | | | | | | | | | | |
| 21-225_155C4_HC | | | | | | | | | | | | | | | | | | | | | | | | | | |
| 21-225_156E4_HC | | | | | | | | | | | | | | | | | | | | | | | | | | |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | H_CDR1 | | | | | | | | | | | | | | H_FR2 | | |
| CONSENSUS | G | - | F | T | F | S | S | - | - | - | - | - | Y | A | M | S | W | V | R | Q | A | P | G | K | G | L |
| 21-225_146E9_HC | . | . | . | . | . | . | . | . | . | . | . | . | . | . | . | . | . | . | . | . | . | . | . | . | . | . |
| 21-225_146H1_HC | . | . | . | . | . | . | . | . | . | . | . | . | . | . | . | . | . | . | . | . | . | . | . | . | . | . |
| 21-225_148C4_HC | . | . | . | . | . | . | . | . | . | . | . | . | . | . | . | N | . | . | . | . | . | . | . | . | . | . |
| 21-225_148H9_HC | . | . | . | . | . | . | . | . | . | . | . | . | . | . | . | . | . | . | . | . | . | . | . | . | . | . |
| 21-225_149A3_HC | . | . | . | . | . | . | N | . | . | . | . | . | . | . | . | T | . | . | . | . | . | . | . | . | . | . |
| 21-225_150C11_HC | . | . | . | . | . | . | . | . | . | . | . | . | . | . | . | N | . | . | . | . | . | . | . | . | . | . |
| 21-225_150C12_HC | . | . | . | . | . | . | . | . | . | . | . | . | . | . | . | . | . | . | . | . | . | . | . | . | . | . |
| 21-225_151B12_HC | . | . | . | . | . | . | . | . | . | . | . | . | . | . | . | N | . | . | . | X | . | . | . | . | . | . |
| 21-225_152B11_HC | . | . | . | . | . | . | . | . | . | . | . | . | . | . | . | N | . | . | . | . | . | . | . | . | . | . |
| 21-225_152D2_HC | . | . | . | . | . | . | . | . | . | . | . | . | . | . | . | . | . | . | . | . | . | . | . | . | . | . |
| 21-225_152G4_HC | . | . | . | . | . | . | . | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | H_CDR2 | | | | | | | | | | | | | | |
| CONSENSUS | E | W | V | S | A | I | S | G | R | - | - | - | G | G | N | T | F | Y | A | D | S | V | K | G | R | F |
| 21-225_146E9_HC | . | . | . | . | . | . | . | . | . | . | . | . | . | . | . | . | . | . | . | . | . | . | . | . | . | . |
| 21-225_146H1_HC | . | . | . | . | . | . | . | . | . | . | . | . | . | . | . | . | . | . | . | . | . | . | . | . | . | . |
| 21-225_148C4_HC | . | . | . | . | . | . | . | . | S | . | . | . | . | . | . | . | . | . | . | . | . | . | . | . | . | . |
| 21-225_148H9_HC | . | . | . | . | . | . | . | . | . | . | . | . | . | . | . | . | . | . | . | . | . | . | . | . | . | . |
| 21-225_149A3_HC | . | . | . | . | . | . | . | . | . | . | . | . | . | . | . | . | . | . | . | . | . | . | . | . | . | . |
| 21-225_150C11_HC | . | . | . | . | . | . | . | . | S | . | . | . | . | . | . | . | . | . | . | . | . | . | . | . | . | . |
| 21-225_150C12_HC | . | . | . | . | . | . | . | . | . | . | . | . | . | . | . | . | . | . | . | . | . | . | . | . | . | . |
| 21-225_151B12_HC | . | . | . | . | . | . | . | . | S | . | . | . | . | . | . | . | . | . | . | . | . | . | . | . | . | . |
| 21-225_152B11_HC | . | . | . | . | . | . | . | . | . | . | . | . | . | . | . | . | . | . | . | . | . | . | . | . | . | . |
| 21-225_152D2_HC | . | . | . | . | . | . | . | . | . | . | . | . | . | . | . | . | . | . | . | . | . | . | . | . | . | . |
| 21-225_152G4_HC | . | . | . | . | . | . | . | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | | H_FR3 | | | | | | | | | | | |
| CONSENSUS | T | I | S | R | D | N | S | K | N | T | L | Y | L | Q | M | N | S | L | R | A | E | D | T | A | V | Y |
| 21-225_146E9_HC | . | . | . | . | . | . | . | . | . | . | . | . | . | . | . | . | . | . | . | . | . | . | . | . | . | . |
| 21-225_146H1_HC | . | . | . | . | . | . | . | . | . | . | . | . | . | . | . | . | . | . | . | . | . | . | . | . | . | . |
| 21-225_148C4_HC | . | . | . | . | . | . | . | . | . | . | . | . | . | . | . | . | . | . | . | . | . | . | . | . | . | . |
| 21-225_148H9_HC | . | . | . | . | . | . | . | . | . | . | . | . | . | . | . | . | . | . | . | . | . | . | . | . | . | . |
| 21-225_149A3_HC | . | . | . | . | . | . | . | . | . | . | . | . | . | . | . | . | . | . | . | . | . | . | . | . | . | . |
| 21-225_150C11_HC | . | . | . | . | . | . | . | . | . | . | . | N | . | . | . | S | . | . | . | . | . | . | . | . | . | . |
| 21-225_150C12_HC | . | . | . | . | . | . | . | . | . | . | . | . | . | . | . | . | . | . | . | . | . | . | . | . | . | . |
| 21-225_151B12_HC | . | . | . | . | . | . | . | . | . | . | . | . | . | . | . | . | . | . | . | . | . | . | . | . | . | . |
| 21-225_152B11_HC | . | . | . | . | . | . | . | . | . | . | . | . | . | . | . | . | . | . | . | . | . | . | . | . | . | . |
| 21-225_152D2_HC | . | . | . | . | . | . | . | . | . | . | . | . | . | . | . | . | . | . | . | . | . | . | . | . | . | . |
| 21-225_152G4_HC | . | . | . | . | . | . | . | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | K | R | V | T | D | Y | G | G | - | - | - | - | - | - | - | - | - | - | - | - | - | - | - |
| 21-225_146E9_HC | . | . | . | . | . | . | . | . | . | . | . | . | . | . | . | . | . | . | . | . | . | . | . | . | . | . |
| 21-225_146H1_HC | . | . | . | . | . | . | . | . | . | . | . | . | . | . | . | . | . | . | . | . | . | . | . | . | . | . |
| 21-225_148C4_HC | . | . | . | . | . | . | . | . | . | . | . | . | . | . | . | . | . | . | . | . | . | . | . | . | . | . |
| 21-225_148H9_HC | . | . | . | . | . | . | . | . | . | . | . | . | . | . | . | . | . | . | . | . | . | . | . | . | . | . |
| 21-225_149A3_HC | . | . | Y | . | . | . | . | . | . | . | . | . | . | . | . | . | . | . | . | . | . | . | . | . | . | . |
| 21-225_150C11_HC | . | . | . | . | . | . | . | . | . | . | . | . | . | . | . | . | . | . | . | . | . | . | . | . | . | . |
| 21-225_150C12_HC | . | . | . | . | . | . | . | . | . | . | . | . | . | . | . | . | . | . | . | . | . | . | . | . | . | . |
| 21-225_151B12_HC | . | . | . | . | . | . | . | . | . | . | . | . | . | . | . | . | . | . | . | . | . | . | . | . | . | . |
| 21-225_152B11_HC | . | . | . | . | . | . | . | . | . | . | . | . | . | . | . | . | . | . | . | . | . | . | . | . | . | . |
| 21-225_152D2_HC | . | . | . | . | . | . | . | . | . | . | . | . | . | . | . | . | . | . | . | . | . | . | . | . | . | . |
| 21-225_152G4_HC | . | . | . | . | . | . | . | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | N | D | W | F | D | P | W | G | Q | G | T | L | V | T | V | S | S |
| 21-225_146E9_HC | . | | . | . | . | . | . | . | . | . | . | . | | . | . | . | . | . | |
| 21-225_146H1_HC | . | | . | . | . | . | . | . | . | . | . | . | | . | . | . | . | . | |
| 21-225_148C4_HC | . | . | . | . | . | . | . | . | . | . | . | . | . | . | . | . | . | . | . |
| 21-225_148H9_HC | . | | . | . | . | . | . | . | . | . | . | . | | . | . | . | . | . | |
| 21-225_149A3_HC | . | | . | . | . | . | . | . | . | . | . | . | | . | . | . | . | . | |
| 21-225_150C11_HC | . | | . | . | . | . | . | . | . | . | . | . | | . | . | . | . | . | |
| 21-225_150C12_HC | . | | . | . | . | . | . | . | . | . | . | . | | . | . | . | . | . | |
| 21-225_151B12_HC | . | | . | . | . | . | . | . | . | . | . | . | | . | . | . | . | . | |
| 21-225_152B11_HC | . | | . | . | . | . | . | . | . | . | . | . | | . | . | . | . | . | |
| 21-225_152D2_HC | . | | . | . | . | . | . | . | . | . | . | . | | . | . | . | . | . | |
| 21-225_152G4_HC | . | | . | . | . | . | . | . | . | . | . | . | | . | . | . | . | . | |
| 21-225_155C4_HC | . | | . | . | . | . | . | . | . | . | . | . | | . | . | . | . | . | |
| 21-225_156E4_HC | . | | . | . | . | . | . | . | . | . | . | . | | . | . | . | . | . | |

**FIGURE 57**

Table 76.    Consensus 42 - VH3|3-33/D3|3-3|RF2/JH6 (SEQ ID NO: 50293):

QVQLVES-GGGVVQPGRSLRLSCAASG-FTFST-----YGMHWVRQAPGKGLEWVAVIWYG---
GSNKDYADSVKGRFTISRDISKNTLYLQMNSLRAEDTAVYYCARDRDYCSGGSC----------PYYYYYGMDVWGQGTTVTVSS wherein:

T in position 33 can be substituted with S

I in position 58 can be substituted with V

G in position 61 can be substituted with D

S in position 66 can be substituted with N

N in position 67 can be substituted with D or S

K in position 68 can be substituted with T

D in position 69 can be substituted with Y or S

Y in position 70 can be substituted with F

A in position 71 can be substituted with V

K in position 75 can be substituted with R or T

D in position 111 can be substituted with V

Y in position 112 can be substituted with F

G in position 116 can be substituted with T

S in position 117 can be substituted with T or N

Xaa Tyr Gly Met His (SEQ ID NO: 50072)

Val Xaa Trp Tyr Xaa Gly Xaa Xaa Xaa Xaa Xaa Xaa Asp Ser Val Xaa Gly (SEQ ID NO: 50073)

Asp Arg Xaa Xaa Cys Ser Gly Xaa Xaa Cys Pro Tyr Tyr Tyr Tyr Tyr Gly Met Asp Val (SEQ ID NO: 50074)

**FIGURE 57**

Thr Tyr Gly Met His (SEQ ID NO: 50549)

Val Ile Trp Tyr Gly Gly Ser Asn Lys Asp Tyr Ala Asp Ser Val Lys Gly (SEQ ID NO: 50550)

Asp Arg Asp Tyr Cys Ser Gly Gly Ser Cys Pro Tyr Tyr Tyr Tyr Gly Met Asp Val (SEQ ID NO: 50551)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
|  |  |  |  |  |  |  |  |  |  |  |  |  |  |  |  | H_FR1 |  |  |  |  |  |  |  |  |  |  |
| CONSENSUS | Q | V | Q | L | V | E | S | - | G | G | G | V | V | Q | P | G | R | S | L | R | L | S | C | A | A | S |
| 21-225_146B8_HC | . | . | . | . | . | . | . |  | . | . | . | . | . | . | . | . | . | . | . | . | . | . | . | . | . | . |
| 21-225_147F9_HC | . | . | . | . | . | . | . |  | . | . | . | . | . | . | . | . | . | . | . | . | . | . | . | . | . | . |
| 21-225_147G9_HC | . | . | . | . | . | . | . |  | . | . | . | . | . | . | . | . | . | . | . | . | . | . | . | . | . | . |
| 21-225_148A9_HC | . | . | . | . | . | . | . |  | . | . | . | . | . | . | . | . | . | . | . | . | . | . | . | . | . | . |
| 21-225_150D3_HC | . | . | . | . | . | . | . |  | . | . | . | . | . | . | . | . | . | . | . | . | . | . | . | . | . | . |
| 21-225_151F3_HC | . | . | . | . | . | . | . |  | . | . | . | . | . | . | . | . | . | . | . | . | . | . | . | . | . | . |
| 21-225_153D9_HC | . | . | . | . | . | . | . |  | . | . | . | . | . | . | . | . | . | . | . | . | . | . | . | . | . | . |
| 21-225_153F9_HC | . | . | N | . | . | . | . |  | . | . | . | . | . | . | . | . | . | . | . | . | . | . | . | . | . | . |
| 21-225_154C3_HC | . | . | . | . | . | . | . |  | . | . | . | . | . | . | . | . | . | . | . | . | . | . | . | T | . | . |
| 21-225_154D11_HC | . | . | . | . | . | . | . |  | . | . | . | . | . | . | . | . | . | . | . | . | . | . | . | . | . | . |
| 21-225_158E9_HC | . | . | . | . | . | . | . |  | . | . | . | . | . | . | . | . | . | . | . | . | . | . | . | . | . | . |
| 21-225_161E10_HC | . | . | . | . | . | . | . |  | . | . | . | . | . | . | . | . | . | . | . | . | . | . | . | . | . | . |
| 21-225_74A9_HC | . | . | . | . | . | . | . |  | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | F | T | F | S | T | - | - | - | - | - | Y | G | M | H | W | V | R | Q | A | P | G | K | G | L |
| 21-225_146B8_HC | . | . | . | . | . | . | . | . | . | . | . | . | . | . | . | . | . | . | . | . | . | . | . | . | . | . |
| 21-225_147F9_HC | . | . | . | . | . | . | . | . | . | . | . | . | . | . | . | . | . | . | . | . | . | . | . | . | . | . |
| 21-225_147G9_HC | . | . | . | . | . | . | . | . | . | . | . | . | . | . | . | . | . | . | . | . | . | . | . | . | . | . |
| 21-225_148A9_HC | . | . | . | . | . | . | . | . | . | . | . | . | . | . | . | . | . | . | . | . | . | . | . | . | . | . |
| 21-225_150D3_HC | . | . | . | . | . | . | . | . | . | . | . | . | . | . | . | . | . | . | . | . | . | . | . | . | . | . |
| 21-225_151F3_HC | . | . | . | . | . | . | . | . | . | . | . | . | . | . | . | . | . | . | . | . | . | . | . | . | . | . |
| 21-225_153D9_HC | . | . | . | . | V | . | . | . | . | . | . | . | . | . | . | . | . | . | . | . | . | . | . | . | . | . |
| 21-225_153F9_HC | . | . | . | . | I | . | . | . | . | . | . | . | . | . | . | . | . | . | . | . | S | . | . | . | . | . |
| 21-225_154C3_HC | . | . | . | . | . | . | . | . | . | . | . | . | . | . | . | . | . | . | . | . | . | . | . | . | . | . |
| 21-225_154D11_HC | . | . | . | N | V | . | . | . | . | . | . | . | . | . | . | . | . | . | . | . | . | . | . | . | . | . |
| 21-225_158E9_HC | . | . | . | . | . | . | S | . | . | . | . | . | . | . | . | . | . | . | . | . | . | . | . | . | . | . |
| 21-225_161E10_HC | . | . | . | . | . | . | S | . | . | . | . | . | . | . | . | . | . | . | . | . | . | . | . | . | . | . |
| 21-225_74A9_HC | . | . | . | . | . | . | S | . | . | . | . | . | . | . | . | . | . | . | . | . | . | . | . | . | . | . |

## FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | G | - | - | - | G | S | N | K | D | Y | A | D | S | V | K | G | R | F |
| 21-225_146B8_HC | . | . | . | . | . | V | . | . | . | . | . | . | . | N | D | . | . | F | . | . | . | . | T | . | . | . |
| 21-225_147F9_HC | . | . | . | . | . | . | . | . | . | . | . | . | . | . | D | . | . | . | . | . | . | . | . | . | . | . |
| 21-225_147G9_HC | . | . | . | . | . | . | . | . | . | . | . | . | . | . | D | . | . | . | . | . | . | . | . | . | . | . |
| 21-225_148A9_HC | . | . | . | . | . | . | . | . | . | . | . | . | . | . | D | . | . | . | . | . | . | . | . | . | . | . |
| 21-225_150D3_HC | . | . | . | . | . | . | . | . | . | . | . | . | . | . | . | . | . | . | . | . | . | . | . | . | . | . |
| 21-225_151F3_HC | . | . | . | . | . | . | . | . | . | . | . | . | . | . | T | . | . | . | . | . | . | . | . | . | . | . |
| 21-225_153D9_HC | . | . | . | . | . | . | . | . | . | . | . | . | . | . | . | . | . | . | . | . | . | . | . | . | . | . |
| 21-225_153F9_HC | . | . | . | . | . | . | . | . | . | . | . | . | . | D | T | . | . | . | . | . | . | % | . | . | . | . |
| 21-225_154C3_HC | . | . | . | . | . | V | . | . | . | . | . | . | . | N | . | . | . | . | . | . | . | . | . | . | . | . |
| 21-225_154D11_HC | . | . | . | . | . | . | . | . | . | . | . | . | . | . | . | . | . | . | . | . | . | . | . | . | . | . |
| 21-225_158E9_HC | . | . | . | . | . | . | . | . | D | . | . | . | . | S | . | . | Y | . | . | . | . | . | . | . | . | . |
| 21-225_161E10_HC | . | . | . | . | . | . | . | . | D | . | . | . | . | . | . | . | Y | . | V | . | . | . | . | . | . | . |
| 21-225_74A9_HC | . | . | . | . | . | . | . | . | D | . | . | . | . | N | . | . | S | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | | |
| CONSENSUS | T | I | S | R | D | I | S | K | N | T | L | Y | L | Q | M | N | S | L | R | A | E | D | T | A | V | Y |
| 21-225_146B8_HC | . | . | . | . | . | . | . | . | . | . | . | . | . | . | . | . | . | . | . | . | . | . | . | . | . | . |
| 21-225_147F9_HC | . | . | . | . | . | . | . | . | . | . | . | . | . | . | . | . | . | . | . | . | . | . | . | . | . | . |
| 21-225_147G9_HC | . | . | . | . | . | . | . | . | . | . | . | . | . | . | . | . | . | . | . | . | . | . | . | . | . | . |
| 21-225_148A9_HC | . | . | . | . | . | . | . | . | . | . | . | . | . | . | . | . | . | . | . | . | . | . | . | . | . | . |
| 21-225_150D3_HC | . | . | . | . | . | N | . | . | . | . | . | . | . | . | . | . | . | . | . | . | . | . | . | . | . | . |
| 21-225_151F3_HC | . | . | . | . | . | . | . | . | . | . | . | . | . | . | . | . | . | . | . | . | . | . | . | . | . | . |
| 21-225_153D9_HC | . | . | . | . | . | . | . | . | . | . | . | . | . | . | . | . | . | . | . | . | . | . | . | . | . | . |
| 21-225_153F9_HC | . | . | . | . | . | . | . | . | . | . | . | . | . | . | . | . | . | . | . | . | . | . | . | . | . | . |
| 21-225_154C3_HC | . | . | . | . | . | . | . | . | . | . | . | . | . | . | . | . | . | . | . | . | . | . | . | . | . | . |
| 21-225_154D11_HC | . | . | . | . | . | S | . | . | . | . | . | . | . | . | . | . | . | . | . | . | . | . | . | . | . | . |
| 21-225_158E9_HC | . | . | . | . | . | N | . | . | . | . | . | . | . | . | . | . | . | . | . | . | . | . | . | . | . | . |
| 21-225_161E10_HC | . | . | . | . | . | N | . | . | . | . | . | . | . | . | . | . | . | . | . | . | . | . | . | . | . | . |
| 21-225_74A9_HC | . | . | . | . | . | . | . | . | . | . | . | S | . | . | . | . | . | . | . | . | Q | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | H_CDR3 | | | | | | | | | | | | | |
| CONSENSUS | Y | C | A | R | D | R | D | Y | C | S | G | G | S | C | - | - | - | - | - | - | - | - | - | - | P | Y |
| 21-225_146B8_HC | . | . | . | . | . | . | . | . | . | . | . | . | . | . | . | . | . | . | . | . | . | . | . | . | . | . |
| 21-225_147F9_HC | . | . | . | . | . | . | . | . | . | . | . | . | T | . | . | . | . | . | . | . | . | . | . | . | . | . |
| 21-225_147G9_HC | . | . | . | . | . | . | . | . | . | . | . | . | . | . | . | . | . | . | . | . | . | . | . | . | . | . |
| 21-225_148A9_HC | . | . | . | . | . | . | . | . | . | . | . | . | T | . | . | . | . | . | . | . | . | . | . | . | . | . |
| 21-225_150D3_HC | . | . | . | . | . | . | . | . | . | . | . | . | T | . | . | . | . | . | . | . | . | . | . | . | . | . |
| 21-225_151F3_HC | . | . | . | . | . | . | . | . | . | . | . | T | . | . | . | . | . | . | . | . | . | . | . | . | . | . |
| 21-225_153D9_HC | . | . | . | . | . | . | . | . | . | . | . | . | . | . | . | . | . | . | . | . | . | . | . | . | . | . |
| 21-225_153F9_HC | . | . | . | . | . | . | . | . | . | . | . | . | N | . | . | . | . | . | . | . | . | . | . | . | . | . |
| 21-225_154C3_HC | . | . | . | . | . | . | . | . | . | . | . | . | . | . | . | . | . | . | . | . | . | . | . | . | . | . |
| 21-225_154D11_HC | . | . | . | . | . | . | . | . | . | . | . | . | . | . | . | . | . | . | . | . | . | . | . | . | . | . |
| 21-225_158E9_HC | . | . | . | . | . | . | V | S | . | . | . | T | . | . | . | . | . | . | . | . | . | . | . | . | . | . |
| 21-225_161E10_HC | . | . | . | . | . | . | V | S | . | . | . | T | . | . | . | . | . | . | . | . | . | . | . | . | . | . |
| 21-225_74A9_HC | . | . | . | . | . | . | . | . | . | . | . | T | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | Y | Y | Y | Y | G | M | D | V | W | G | Q | G | T | T | V | T | V | S | S |
| 21-225_146B8_HC | . | | . | . | . | . | . | . | . | . | . | . | | . | . | . | . | . | |
| 21-225_147F9_HC | . | | . | . | . | . | . | . | . | . | . | . | | . | . | . | . | . | |
| 21-225_147G9_HC | . | . | . | . | . | . | . | . | . | . | . | . | . | . | . | . | . | . | . |
| 21-225_148A9_HC | . | | . | . | . | . | . | . | . | . | . | . | | . | . | . | . | . | |
| 21-225_150D3_HC | . | | . | . | . | . | . | . | . | . | . | . | | . | . | . | . | . | |
| 21-225_151F3_HC | . | | . | . | . | . | . | . | . | . | . | . | | . | . | . | . | . | |
| 21-225_153D9_HC | . | | . | . | . | . | . | . | . | . | . | . | | . | . | . | . | . | |
| 21-225_153F9_HC | . | | . | . | . | . | . | . | . | . | . | . | | . | . | . | . | . | |
| 21-225_154C3_HC | . | | . | . | . | . | . | . | . | . | . | . | | . | . | . | . | . | |
| 21-225_154D11_HC | . | | . | . | . | . | . | . | . | . | . | . | | . | . | . | . | . | |
| 21-225_158E9_HC | . | | . | . | . | . | . | . | . | . | . | . | | . | . | . | . | . | |
| 21-225_161E10_HC | . | | . | . | . | . | . | . | . | . | . | . | | . | . | . | . | . | |
| 21-225_74A9_HC | . | | . | . | . | . | . | . | . | . | . | . | | . | . | . | . | . | |

**FIGURE 57**

Table 77.      Consensus 43 - VH4|4-39/D4|4-17|RF2/JH4 (SEQ ID NO: 50294):

QLQLQES-GPGLVKPSETLSLTCTVSG-GSIS<u>RS---SYYWG</u>WIRQPPGKGLEWIG<u>NIYY----
SGSTYYNPSLKSR</u>VTISVDTSKNQFSLKLSSVTAADTAVYYCGR<u>HGKDW</u>--------------------<u>GLDY</u>WGQGTLVTVSS wherein:

S in position 65 can be substituted with G

S in position 67 can be substituted with T or N

T in position 68 can be substituted with A

Y in position 70 can be substituted with N, D, H or T

L in position 74 can be substituted with V

S in position 76 can be substituted with G

Y in position 138 can be substituted with F or N

Arg Ser Ser Tyr Tyr Trp Gly (SEQ ID NO: 50075)

Asn Ile Tyr Tyr Xaa Gly Xaa Xaa Tyr Xaa Asn Pro Ser Xaa Lys Xaa (SEQ ID NO: 50076)

His Gly Lys Asp Trp Gly Leu Asp Xaa (SEQ ID NO: 50077)

Arg Ser Ser Tyr Tyr Trp Gly (SEQ ID NO: 50552)

Asn Ile Tyr Tyr Ser Gly Ser Thr Tyr Tyr Asn Pro Ser Leu Lys Ser (SEQ ID NO: 50553)

His Gly Lys Asp Trp Gly Leu Asp Tyr (SEQ ID NO: 50554)

FIGURE 57

| Reference # | 1 | 2 | 3 | 4 | 5 | 6 | 7 | 8 | 9 | 10 | 11 | 12 | 13 | 14 | 15 | 16 H_FR1 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| CONSENSUS | Q | L | Q | L | Q | E | S | - | G | P | G | L | V | K | P | S | E | T | L | S | L | T | C | T | V | S |
| 21-225_11F5_HC | . | . | . | . | . | . | . | . | . | . | . | . | . | . | . | . | . | . | . | . | . | . | . | . | . | . |
| 21-225_14B2_HC | . | . | . | . | . | . | . | . | . | . | . | . | . | . | . | . | . | . | . | . | . | . | . | . | . | . |
| 21-225_17F9_HC | . | . | . | . | . | . | . | . | . | . | . | . | . | . | . | . | . | . | . | . | . | . | . | . | . | . |
| 21-225_17H8_HC | . | . | . | . | . | . | . | . | . | . | . | . | . | . | . | . | . | . | . | . | . | . | . | . | . | . |
| 21-225_1F2_HC | . | . | . | . | . | . | . | . | . | . | . | . | . | . | . | . | . | . | . | . | . | . | . | . | . | . |
| 21-225_21H3_HC | . | . | . | . | . | . | . | . | . | . | . | . | . | . | . | . | . | . | . | . | . | . | . | . | . | . |
| 21-225_22G8_HC | . | . | . | . | . | . | . | . | . | . | . | . | . | . | . | . | . | . | . | . | . | . | . | . | . | . |
| 21-225_22H4_HC | . | . | . | . | . | . | . | . | . | . | . | . | . | . | . | . | . | . | . | . | . | . | . | . | . | . |
| 21-225_23A3_HC | . | . | . | . | . | . | . | . | . | . | . | . | . | . | . | . | . | . | . | . | . | . | . | . | . | . |
| 21-225_23C8_HC | . | . | . | . | . | . | . | . | . | . | . | . | . | . | . | . | . | . | . | . | . | . | . | . | . | . |
| 21-225_4F4_HC | . | . | . | . | . | . | . | . | . | . | . | . | . | . | . | . | . | . | . | . | . | . | . | . | . | . |
| 21-225_4H4_HC | . | . | . | . | . | . | . | . | . | . | . | . | . | . | . | . | . | . | . | . | . | . | . | . | . | . |
| 21-225_5H7_HC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | G | S | I | S | R | S | - | - | - | S | Y | Y | W | G | W | I | R | Q | P | P | G | K | G | L |
| 21-225_11F5_HC | . | . | . | . | . | . | . | . | . | . | . | . | . | . | . | . | . | . | . | . | . | . | . | . | . | . |
| 21-225_14B2_HC | . | . | . | . | . | N | . | . | . | . | . | . | . | . | . | . | . | . | . | . | . | . | . | . | . | . |
| 21-225_17F9_HC | . | . | . | . | . | . | . | . | . | . | . | . | . | . | . | . | . | . | . | . | . | . | . | . | . | . |
| 21-225_17H8_HC | . | . | . | . | . | . | . | . | . | . | . | . | . | . | . | . | . | . | . | . | . | . | . | R | . | . |
| 21-225_1F2_HC | . | . | . | . | . | . | . | . | . | . | . | . | . | . | . | . | . | . | . | . | . | . | . | . | . | . |
| 21-225_21H3_HC | . | . | . | . | . | . | . | . | . | . | . | . | . | . | . | . | . | . | . | . | . | . | . | . | . | . |
| 21-225_22G8_HC | . | . | . | . | . | N | . | . | . | . | . | . | . | . | . | . | . | . | . | . | . | . | . | . | . | . |
| 21-225_22H4_HC | . | . | . | . | . | . | . | . | . | . | . | . | . | . | . | . | . | . | . | . | . | . | . | . | . | . |
| 21-225_23A3_HC | . | . | . | . | . | . | . | . | . | . | . | . | . | . | . | . | . | . | . | . | . | . | . | Q | . | . |
| 21-225_23C8_HC | . | . | . | . | . | . | . | . | . | . | . | . | . | . | . | . | . | . | . | . | . | . | . | . | . | . |
| 21-225_4F4_HC | . | . | . | . | . | . | . | . | . | . | . | . | . | . | . | . | . | . | . | . | . | . | . | . | . | . |
| 21-225_4H4_HC | . | . | . | . | . | . | . | . | . | . | . | . | . | . | . | . | . | . | . | . | . | . | . | N | . | . |
| 21-225_5H7_HC | . | . | . | . | . | N | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | N | I | Y | Y | - | - | - | - | S | G | S | T | Y | Y | N | P | S | L | K | S | R | V |
| 21-225_11F5_HC | . | . | . | . | . | . | . | . | . | . | . | . | . | . | . | . | . | Y | . | . | . | . | . | . | . | . |
| 21-225_14B2_HC | . | . | . | . | . | . | . | . | . | . | . | . | . | . | T | . | . | . | . | . | . | . | . | . | . | . |
| 21-225_17F9_HC | . | . | . | . | . | . | . | . | . | . | . | . | . | . | . | . | . | . | . | . | . | . | . | . | . | . |
| 21-225_17H8_HC | . | . | . | . | . | . | . | . | . | . | . | . | G | . | N | . | . | . | . | . | . | . | . | . | . | . |
| 21-225_1F2_HC | . | . | . | . | . | . | . | . | . | . | . | . | . | . | . | A | . | N | . | . | . | . | G | . | . | . |
| 21-225_21H3_HC | . | . | . | . | . | . | . | . | . | . | . | . | . | . | . | . | . | D | . | . | . | . | . | . | . | . |
| 21-225_22G8_HC | D | . | . | . | . | . | . | . | . | . | . | . | . | . | . | . | . | . | . | . | . | V | . | . | . | F |
| 21-225_22H4_HC | . | . | . | . | . | . | . | . | . | . | . | . | . | . | . | . | . | N | . | . | . | . | . | . | . | . |
| 21-225_23A3_HC | . | . | . | . | . | . | . | . | . | . | . | . | . | . | . | . | . | N | . | . | . | . | . | . | . | . |
| 21-225_23C8_HC | . | . | . | . | . | . | . | . | . | . | . | . | . | . | T | . | . | N | . | . | . | . | . | . | . | . |
| 21-225_4F4_HC | . | . | . | . | . | . | . | . | . | . | . | . | . | . | . | . | . | D | . | . | . | . | . | . | . | . |
| 21-225_4H4_HC | . | . | . | . | . | . | . | . | . | . | . | . | . | . | N | . | . | N | . | . | . | . | . | . | . | . |
| 21-225_5H7_HC | . | . | . | . | . | . | . | . | . | . | . | . | . | . | T | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | V | D | T | S | K | N | Q | F | S | L | K | L | S | S | V | T | A | A | D | T | A | V | Y |
| 21-225_11F5_HC | . | . | . | . | . | . | . | . | . | H | . | . | . | . | . | . | . | . | . | . | . | . | . | . | . | . |
| 21-225_14B2_HC | . | . | . | . | . | . | . | . | . | . | . | . | . | N | . | . | . | . | . | . | . | . | . | . | . | . |
| 21-225_17F9_HC | . | . | . | . | S | . | . | . | . | . | . | . | . | N | . | N | . | . | . | . | . | . | . | . | . | . |
| 21-225_17H8_HC | . | . | . | . | . | . | . | . | . | . | . | . | . | . | . | . | . | . | . | . | . | . | . | . | . | . |
| 21-225_1F2_HC | . | . | . | . | . | . | . | . | . | . | . | . | . | . | . | N | . | . | . | . | . | . | . | . | . | . |
| 21-225_21H3_HC | . | . | . | . | . | . | . | . | . | . | . | . | . | . | . | . | . | . | . | . | . | . | . | . | . | . |
| 21-225_22G8_HC | . | . | . | . | . | . | . | . | . | . | . | . | . | . | . | . | . | . | . | . | . | . | . | . | . | . |
| 21-225_22H4_HC | . | . | . | . | . | . | . | M | . | . | . | . | . | . | . | T | . | . | . | . | . | . | . | . | . | . |
| 21-225_23A3_HC | S | . | . | . | . | . | . | . | . | . | . | . | . | N | . | . | . | . | . | . | . | . | . | . | . | . |
| 21-225_23C8_HC | . | . | . | . | . | . | . | . | . | H | . | . | . | . | . | . | . | . | . | . | . | . | . | . | . | . |
| 21-225_4F4_HC | . | . | . | . | . | . | . | . | . | . | . | . | . | . | . | . | . | . | . | . | . | . | . | . | . | . |
| 21-225_4H4_HC | . | . | . | . | . | . | . | . | . | . | . | . | . | . | . | N | . | . | . | . | . | . | . | . | . | . |
| 21-225_5H7_HC | . | . | . | . | . | . | . | . | . | . | . | . | . | N | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | G | R | H | G | K | D | W | - | - | - | - | - | - | - | - | - | - | - | - | - | - | - | - | - |
| 21-225_11F5_HC | . | . | . | | | | | | | | | | | | | | | | | | | | | | | |
| 21-225_14B2_HC | S | . | A | | | | | | | | | | | | | | | | | | | | | | | |
| 21-225_17F9_HC | S | . | A | | | | | | | | | | | | | | | | | | | | | | | |
| 21-225_17H8_HC | . | . | A | | | | | | | | | | | | | | | | | | | | | | | |
| 21-225_1F2_HC | . | . | | | | | | | | | | | | | | | | | | | | | | | | |
| 21-225_21H3_HC | . | . | | | | | | | | | | | | | | | | | | | | | | | | |
| 21-225_22G8_HC | . | . | A | | | | | | | | | | | | | | | | | | | | | | | |
| 21-225_22H4_HC | P | | | | | | | | | | | | | | | | | | | | | | | | | |
| 21-225_23A3_HC | . | | | | | | | | | | | | | | | | | | | | | | | | | |
| 21-225_23C8_HC | . | | | | | | | | | | | | | | | | | | | | | | | | | |
| 21-225_4F4_HC | . | | | | | | | | | | | | | | | | | | | | | | | | | |
| 21-225_4H4_HC | . | | | | | | | | | | | | | | | | | | | | | | | | | |
| 21-225_5H7_HC | S | . | A | | | | | | | | | | | | | | | | | | | | | | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | G | L | D | Y | W | G | Q | G | T | L | V | T | V | S | S |
| 21-225_11F5_HC | | | | | | | | N | | | | | | | | | | | |
| 21-225_14B2_HC | | | | | | | | | | | | | A | | | | | | |
| 21-225_17F9_HC | | | | | | | | | | | | | | | | | | | |
| 21-225_17H8_HC | | | | | | | | | | | | | | | | | | | |
| 21-225_1F2_HC | | | | | | | | | | | | | | | | | | | |
| 21-225_21H3_HC | | | | | | | | | | | | | | | | | | | |
| 21-225_22G8_HC | | | | | | | | | | | | | | | | | | | |
| 21-225_22H4_HC | | | | | | | | | | | | | | | | | | | |
| 21-225_23A3_HC | | | | | | | | S | | | | | | | | | | | |
| 21-225_23C8_HC | | | | | | | | | | | | | | | | | | | |
| 21-225_4F4_HC | | | | | | | | | | | | | | | | | | | |
| 21-225_4H4_HC | | | | | | | | | | | | | | | | I | | | |
| 21-225_5H7_HC | | | | | | | | | | | | | | | | | | | |

**FIGURE 57**

Table 78.    Consensus 44 - VH1|1-02/D7|7-27|RF1/JH4 (SEQ ID NO: 50295):

QVQLVQS-GAEVKKPGASVKVSCKASG-YTFTG-----YYMHWVRQAPGQGLEWMGWIKPN---
SGGTNQAQKFQGRVTMTRDTSISTAYMELSGLRSDDTAVYYCARAPGTAAAG--------------TWGYFDYWGQGTLVTVSS wherein:

M at position 41 can be substituted with I

K in position 59 can be substituted with N

N in position 61 can be substituted with K

Q in position 70 can be substituted with S, H, N or Y

A in position 71 can be substituted with V

T in position 112 can be substituted with K or I

A in position 113 can be substituted with V

A in position 114 can be substituted with P

A in position 115 can be substituted with T

T in position 132 can be substituted with S

Y in position 135 can be substituted with F or C

Gly Tyr Tyr Xaa His (SEQ ID NO: 50078)

Trp Ile Xaa Pro Xaa Ser Gly Gly Thr Asn Xaa Xaa Gln Lys Phe Gln Gly (SEQ ID NO: 50079)

Ala Pro Gly Xaa Xaa Xaa Xaa Gly Xaa Trp Gly Xaa Phe Asp Tyr (SEQ ID NO: 50080)

Gly Tyr Tyr Met His (SEQ ID NO: 50555)

Trp Ile Lys Pro Asn Ser Gly Gly Thr Asn Gln Ala Gln Lys Phe Gln Gly (SEQ ID NO: 50556)

Ala Pro Gly Thr Ala Ala Ala Gly Thr Trp Gly Tyr Phe Asp Tyr (SEQ ID NO: 50557)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | Q | S | - | G | A | E | V | K | K | P | G | A | S | V | K | V | S | C | K | A | S |
| 21-225_62G7_HC | . | . | . | . | . | . | . | . | . | . | . | . | . | . | . | . | . | . | . | . | . | . | . | . | . | . |
| 21-225_64H10_HC | . | . | . | . | . | . | . | . | . | . | . | . | . | . | . | . | . | . | . | . | . | . | . | . | . | . |
| 21-225_65G3_HC | . | . | . | . | . | . | . | . | . | . | . | . | . | . | . | . | . | . | . | . | . | . | . | . | . | . |
| 21-225_66A7_HC | . | . | . | . | . | . | . | . | . | . | . | . | . | . | . | . | . | . | . | . | . | . | . | . | . | . |
| 21-225_66B1_HC | . | . | . | . | . | . | . | . | . | . | . | . | . | . | . | . | . | . | . | . | . | . | . | . | . | . |
| 21-225_67H4_HC | . | . | . | . | . | . | . | . | . | . | . | . | . | . | . | . | . | . | . | . | . | . | . | . | . | . |
| 21-225_70A12_HC | . | . | . | . | . | . | . | . | . | . | . | . | . | . | . | . | . | . | . | . | . | . | . | . | . | . |
| 21-225_70D6_HC | . | . | . | . | . | . | . | . | . | . | . | . | . | . | . | . | . | . | . | . | . | . | . | . | . | . |
| 21-225_70G9_HC | . | . | . | . | . | . | . | . | . | . | . | . | . | . | . | . | . | . | . | . | . | . | . | . | . | . |
| 21-225_71A7_HC | . | . | . | . | . | . | . | . | . | . | . | . | . | . | . | . | . | . | . | . | . | . | . | . | . | . |
| 21-225_71B7_HC | . | . | . | . | . | . | . | . | . | . | . | . | . | . | . | . | . | . | . | . | . | . | . | . | . | . |
| 21-225_73A3_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | Y | T | F | T | G | - | - | - | - | - | Y | Y | M | H | W | V | R | Q | A | P | G | Q | G | L |
| 21-225_62G7_HC | . | . | . | . | . | . | . | . | . | . | . | . | . | . | . | . | . | . | . | . | . | . | . | . | . | . |
| 21-225_64H10_HC | . | . | . | . | . | . | . | . | . | . | . | . | . | . | . | . | . | . | . | . | . | . | . | . | . | . |
| 21-225_65G3_HC | . | . | . | . | . | . | . | . | . | . | . | . | . | . | ? | . | . | . | . | . | . | . | . | . | . | . |
| 21-225_66A7_HC | . | . | . | . | . | . | . | . | . | . | . | . | . | . | . | . | . | . | . | . | . | . | . | . | . | . |
| 21-225_66B1_HC | . | . | . | . | . | . | . | . | . | . | . | . | . | . | . | . | . | . | . | . | . | . | . | . | . | . |
| 21-225_67H4_HC | . | . | . | . | . | . | . | . | . | . | . | . | . | . | . | . | . | . | . | . | . | . | . | . | . | . |
| 21-225_70A12_HC | . | . | . | . | . | . | . | . | . | . | . | . | . | . | . | . | . | . | . | . | . | . | . | . | . | . |
| 21-225_70D6_HC | . | . | ? | . | . | . | . | . | . | . | . | . | . | . | . | . | . | . | . | . | . | . | . | . | . | . |
| 21-225_70G9_HC | . | . | . | . | . | . | . | . | . | . | . | . | . | . | . | . | . | . | . | . | . | . | . | . | . | . |
| 21-225_71A7_HC | . | . | . | . | . | . | . | . | . | . | . | . | . | . | . | . | . | . | . | . | . | . | . | . | . | . |
| 21-225_71B7_HC | . | . | ? | . | . | . | . | . | . | . | . | . | . | . | . | . | . | . | . | . | . | . | . | . | . | . |
| 21-225_73A3_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | M | G | W | I | | P | N | - | - | - | S | G | G | T | N | Q | A | Q | K | F | Q | G | R | V |
| 21-225_62G7_HC | . | . | . | . | . | . | X | . | X | . | . | . | . | . | . | . | . | . | . | . | . | . | . | . | . | . |
| 21-225_64H10_HC | . | . | . | . | . | . | X | . | . | . | . | . | . | . | . | . | . | . | . | . | . | . | . | . | . | . |
| 21-225_65G3_HC | . | . | . | . | . | . | N | . | . | . | . | . | . | . | . | . | . | S | . | . | . | . | . | . | . | . |
| 21-225_66A7_HC | . | . | . | . | . | . | X | . | . | . | . | . | . | . | . | . | . | . | . | . | . | . | . | . | . | . |
| 21-225_66B1_HC | . | . | . | . | . | . | N | . | . | . | . | . | . | . | . | . | . | N | . | . | . | . | . | . | . | . |
| 21-225_67H4_HC | . | . | . | . | . | . | X | . | . | . | . | . | . | . | . | . | . | . | . | . | . | . | . | . | . | . |
| 21-225_70A12_HC | . | . | . | . | . | . | X | . | . | . | . | . | . | . | . | . | . | . | . | . | . | . | . | . | . | . |
| 21-225_70D6_HC | . | . | . | . | . | . | N | . | X | . | . | . | . | . | . | . | . | S | . | . | . | . | . | . | . | . |
| 21-225_70G9_HC | . | . | . | . | . | . | X | . | . | . | . | . | . | . | . | . | . | . | . | . | . | . | . | . | . | . |
| 21-225_71A7_HC | . | . | . | . | . | . | N | . | X | . | . | . | . | . | . | . | . | Y | V | . | . | . | . | . | . | . |
| 21-225_71B7_HC | . | . | . | . | . | . | N | . | . | . | . | . | . | . | . | . | . | S | . | . | . | . | . | . | . | . |
| 21-225_73A3_HC | D | . | . | . | . | . | N | . | X | . | . | . | . | . | . | . | . | X | V | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | | |
| CONSENSUS | T | M | T | R | D | T | S | I | S | T | A | Y | M | E | L | S | G | L | R | S | D | D | T | A | V | Y |
| 21-225_62G7_HC | | | | | | | | | | | | | | | | | S | | | | | | | | | |
| 21-225_64H10_HC | | | | | | | | | | | | | | | | | | | | | | | | | | |
| 21-225_65G3_HC | | | | | | | | | | | | | | | | | R | | | | | | | | | |
| 21-225_66A7_HC | | | | | | | | | | | G | | | | | | R | | | | | | | | | |
| 21-225_66B1_HC | | | | | A | | | | | | | | | | | | R | | | | | | | | | |
| 21-225_67H4_HC | | | | | | | | | | | | | | | | | | | | | | | | | | |
| 21-225_70D6_HC | S | | | | | | | | G | | | | | | | | R | | | | | | | | E | |
| 21-225_70G9_HC | | | | | | | | | | | | | | | | | S | | | | | | | | | |
| 21-225_71A7_HC | | | | | | | | | | | | | | | | | S | | | | | | | | | |
| 21-225_71B7_HC | | | | | | | | | | G | | | | | | | | | | | | | | | | |
| 21-225_73A3_HC | A | | | | | | | | | | | | | | | | S | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | A | P | G | T | A | A | A | G | - | - | - | - | - | - | - | - | - | - | - | - | - | - |
| 21-225_62G7_HC | . | . | . | . | . | . | . | I | . | . | . | . | . | . | . | . | . | . | . | . | . | . | . | . | . | . |
| 21-225_64H10_HC | . | . | . | . | . | . | . | . | . | . | T | . | . | . | . | . | . | . | . | . | . | . | . | . | . | . |
| 21-225_65G3_HC | . | . | . | . | . | . | . | X | . | . | . | . | . | . | . | . | . | . | . | . | . | . | . | . | . | . |
| 21-225_66A7_HC | . | . | . | . | . | . | . | . | V | . | . | . | . | . | . | . | . | . | . | . | . | . | . | . | . | . |
| 21-225_66B1_HC | . | . | . | . | . | . | . | . | . | . | . | . | . | . | . | . | . | . | . | . | . | . | . | . | . | . |
| 21-225_67H4_HC | . | . | . | . | . | . | . | . | . | . | T | . | . | . | . | . | . | . | . | . | . | . | . | . | . | . |
| 21-225_70A12_HC | . | . | . | . | . | . | . | . | . | . | T | . | . | . | . | . | . | . | . | . | . | . | . | . | . | . |
| 21-225_70D6_HC | . | . | . | . | . | . | . | X | . | . | . | . | . | . | . | . | . | . | . | . | . | . | . | . | . | . |
| 21-225_70G9_HC | . | . | . | . | . | . | . | I | . | . | . | . | . | . | . | . | . | . | . | . | . | . | . | . | . | . |
| 21-225_71A7_HC | . | . | . | . | . | . | . | . | . | V | . | . | . | . | . | . | . | . | . | . | . | . | . | . | . | . |
| 21-225_71B7_HC | . | . | . | . | . | . | . | X | . | . | . | . | . | . | . | . | . | . | . | . | . | . | . | . | . | . |
| 21-225_73A3_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | T | W | G | Y | F | D | Y | W | G | Q | G | T | L | V | T | V | S | S |
| 21-225_62G7_HC | . | . | . | . | . | . | . | . | . | . | . | . | . | . | . | . | . | . | . |
| 21-225_64H10_HC | . | . | . | . | . | . | . | . | . | . | . | . | . | . | . | . | . | . | . |
| 21-225_65G3_HC | . | . | . | . | . | . | . | . | . | . | . | . | . | . | . | . | . | . | . |
| 21-225_66A7_HC | . | . | . | . | . | . | . | . | . | . | . | . | . | . | . | . | . | . | . |
| 21-225_66B1_HC | . | . | . | . | . | . | . | . | . | . | . | . | . | . | . | . | . | . | . |
| 21-225_67H4_HC | . | . | . | . | . | . | . | . | . | . | . | . | . | . | . | . | . | . | . |
| 21-225_70A12_HC | . | . | . | . | . | . | . | . | . | . | . | . | . | . | . | . | . | . | . |
| 21-225_70D6_HC | . | . | . | . | F | . | . | . | . | . | . | . | . | . | . | . | . | . | . |
| 21-225_70G9_HC | . | . | . | . | . | . | . | . | . | . | . | . | . | . | . | . | . | . | . |
| 21-225_71A7_HC | . | S | . | . | . | . | . | . | . | . | . | . | . | . | . | . | . | . | . |
| 21-225_71B7_HC | . | . | . | . | F | . | . | . | . | . | . | . | . | . | . | . | . | . | . |
| 21-225_73A3_HC | . | S | . | . | G | . | . | . | . | . | . | . | . | . | . | . | . | . | . |

FIGURE 57

Table 79.    Consensus 45 - VH2|2-05/D6|6-6|RF2/JH4 (SEQ ID NO: 50296):

QITLKES-GPTLVKPTQTLTLTCTFSG-FSLS<u>TG---GVGVG</u>WIRQPPGKALEWLAL<u>IYW----</u>
<u>DDDKRYSPSLKS</u>RLTITKDTSKNQVVLTMTNMDPVDTATYYCAH<u>LIAV--------------------</u>AFDYWGQGTLVTVSS wherein:

G in position 34 can be substituted with S

L in position 57 can be substituted with F

D in position 65 can be substituted with H, N, K or S

K in position 68 can be substituted with E

K in position 75 can be substituted with R

L in position 109 can be substituted with I or A

I in position 110 can be substituted with V or A

A in position 135 can be substituted with S

F in position 136 can be substituted with C

Thr Xaa Gly Val Gly Val Gly (SEQ ID NO: 50081)

Xaa Ile Tyr Trp Xaa Asp Asp Xaa Arg Tyr Ser Pro Ser Leu Xaa Ser (SEQ ID NO: 50082)

Xaa Xaa Ala Val Xaa Xaa Asp Tyr (SEQ ID NO: 50083)

Thr Gly Gly Val Gly Val Gly (SEQ ID NO: 50558)

Leu Ile Tyr Trp Asp Asp Asp Lys Arg Tyr Ser Pro Ser Leu Lys Ser (SEQ ID NO: 50559)

Leu Ile Ala Val Ala Phe Asp Tyr (SEQ ID NO: 50560)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | I | T | L | K | E | S | - | G | P | T | L | V | K | P | T | Q | T | L | T | L | T | C | T | F | S |
| 21-225_10B10_HC | . | . | . | . | . | . | . | . | . | . | . | . | . | . | . | . | . | . | . | . | . | . | . | . | . | . |
| 21-225_15H11_HC | . | . | . | . | . | . | . | . | . | L | . | M | . | . | . | . | . | . | . | . | . | . | . | . | . | . |
| 21-225_17F5_HC | . | . | . | . | . | . | . | . | . | . | S | . | . | . | . | . | . | . | . | . | . | . | . | . | . | . |
| 21-225_30H6_HC | . | . | . | . | . | . | . | . | . | . | . | . | . | . | . | . | . | . | . | . | . | . | . | . | . | . |
| 21-225_31C2_HC | . | . | . | . | . | . | . | . | . | . | . | . | . | . | . | . | . | . | . | . | . | . | . | . | . | . |
| 21-225_57D9_HC | . | . | . | . | . | . | . | . | . | . | M | . | . | . | . | . | . | . | . | . | . | . | . | . | . | . |
| 21-225_60D6_HC | . | . | . | . | . | . | . | . | . | . | . | . | . | . | . | . | . | . | . | . | . | . | . | . | . | . |
| 21-225_74G9_HC | . | . | . | . | . | . | . | . | . | . | . | . | . | . | . | . | . | . | . | . | . | . | . | . | . | . |
| 21-225_75F11_HC | . | . | . | . | . | . | . | . | . | . | . | . | . | . | . | . | . | . | . | . | . | . | . | . | . | . |
| 21-225_76B4_HC | . | . | . | . | . | . | . | . | . | . | . | . | . | . | . | . | . | . | . | . | . | . | . | . | . | . |
| 21-225_77A2_HC | . | . | . | . | . | . | . | . | . | . | S | . | . | . | . | . | . | . | . | . | . | . | . | . | . | . |
| 21-225_96B5_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | H_CDR1 | | | | | | | | | | | | | | | H_FR2 | | | |
| CONSENSUS | G | - | F | S | L | S | T | G | - | - | - | G | V | G | V | G | W | I | R | Q | P | P | G | K | A | L |
| 21-225_10B10_HC | . | . | . | . | . | . | . | . | . | . | . | . | . | . | . | . | . | . | . | . | . | . | . | . | . | . |
| 21-225_15H11_HC | . | . | . | . | . | . | . | . | . | . | . | . | . | . | . | . | . | . | . | . | . | . | . | . | . | . |
| 21-225_17F5_HC | . | . | . | . | . | . | . | S | . | . | . | . | . | . | . | . | . | . | . | . | . | . | . | . | . | . |
| 21-225_30H6_HC | . | . | . | . | . | . | . | . | . | . | . | . | . | . | . | . | . | . | . | . | . | . | . | . | . | . |
| 21-225_31C2_HC | . | . | . | . | . | N | . | . | . | . | . | . | . | . | . | . | . | . | . | . | . | . | . | . | . | . |
| 21-225_57D9_HC | . | . | . | . | . | . | . | S | . | . | . | . | . | . | . | . | . | . | . | . | . | . | . | . | . | . |
| 21-225_60D6_HC | . | . | . | . | . | . | . | S | . | . | . | . | . | . | . | . | . | . | . | . | . | . | . | . | . | . |
| 21-225_74G9_HC | . | . | . | . | . | . | . | . | . | . | . | . | . | . | . | . | . | . | . | . | . | . | . | . | . | . |
| 21-225_75F11_HC | . | . | . | . | . | . | . | . | . | . | . | . | . | . | . | . | . | . | . | . | . | . | . | . | . | . |
| 21-225_76B4_HC | . | . | . | . | . | . | . | . | . | . | . | . | . | . | . | . | . | . | . | . | . | . | . | . | . | . |
| 21-225_77A2_HC | . | . | . | . | . | . | . | S | . | . | . | . | . | . | . | . | . | . | . | . | . | . | . | . | . | . |
| 21-225_96B5_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | L | A | L | I | Y | W | - | - | - | - | D | D | D | K | R | Y | S | P | S | L | K | S | R | L |
| 21-225_10B10_HC | . | . | . | V | S | . | . | . | . | . | . | . | S | . | . | . | . | . | . | . | . | . | . | . | . | . |
| 21-225_15H11_HC | . | . | . | . | . | . | . | . | . | . | . | . | N | . | . | . | . | . | . | . | . | . | R | . | . | . |
| 21-225_17F5_HC | . | . | . | . | . | . | . | . | . | . | . | . | . | . | . | . | . | . | . | . | . | . | . | . | . | . |
| 21-225_30H6_HC | . | . | . | . | . | . | . | . | . | . | . | . | N | . | . | . | . | . | . | . | . | . | . | . | . | . |
| 21-225_31C2_HC | . | . | . | . | . | . | . | . | . | . | . | . | N | . | . | E | . | . | . | . | . | . | . | . | . | . |
| 21-225_57D9_HC | . | . | . | . | . | . | . | . | . | . | . | . | N | . | . | . | . | . | . | . | . | . | . | . | . | . |
| 21-225_60D6_HC | . | . | . | . | . | . | . | . | . | . | . | . | K | . | . | E | . | . | . | . | . | . | . | . | . | . |
| 21-225_74G9_HC | . | . | . | . | . | . | . | . | . | . | . | . | . | . | . | . | . | . | . | . | . | . | . | . | . | . |
| 21-225_75F11_HC | . | . | . | . | . | . | . | . | . | . | . | . | . | . | . | . | . | . | . | . | . | . | . | . | . | . |
| 21-225_76B4_HC | . | . | . | . | . | . | . | . | . | . | . | . | . | . | . | . | . | . | . | . | . | . | . | . | . | . |
| 21-225_77A2_HC | . | . | . | V | S | . | . | . | . | . | . | . | . | . | . | . | . | . | . | . | . | . | . | . | . | . |
| 21-225_96B5_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | H_FR3 | | | | | | | | | | | | | |
| CONSENSUS | T | I | T | K | D | T | S | K | N | Q | V | V | L | T | M | T | N | M | D | P | V | D | T | A | T | Y |
| 21-225_10B10_HC | S | . | . | . | . | . | . | . | . | . | . | . | . | . | . | . | . | . | . | . | . | . | . | . | . | . |
| 21-225_15H11_HC | . | . | . | . | . | . | . | . | . | . | . | . | . | . | . | . | . | . | . | . | . | . | . | . | . | . |
| 21-225_17F5_HC | . | . | . | . | . | . | . | . | . | . | . | . | . | . | . | . | . | . | . | . | . | . | . | . | . | . |
| 21-225_30H6_HC | A | . | . | . | . | . | . | . | . | . | . | . | . | I | . | . | . | . | . | . | . | . | . | . | . | . |
| 21-225_31C2_HC | . | . | . | . | . | . | . | . | . | . | . | . | . | . | . | . | . | . | . | L | . | . | . | . | S | . |
| 21-225_57D9_HC | . | . | E | . | . | . | . | . | . | . | . | . | . | . | . | . | . | . | . | . | . | . | . | . | . | . |
| 21-225_60D6_HC | . | . | . | . | . | . | . | . | . | . | . | . | . | . | . | . | . | . | . | . | . | . | . | . | . | . |
| 21-225_74G9_HC | . | . | . | . | . | . | P | . | . | . | . | . | . | . | . | . | . | . | . | . | . | . | . | . | . | . |
| 21-225_75F11_HC | . | . | . | . | . | . | P | . | . | . | . | . | . | . | . | . | . | . | . | . | . | . | . | . | . | . |
| 21-225_76B4_HC | . | . | . | . | . | . | P | . | . | . | . | . | . | . | . | . | . | . | . | . | . | . | . | . | . | . |
| 21-225_77A2_HC | . | . | . | . | . | . | . | . | . | . | . | . | . | . | . | . | . | . | . | . | . | . | . | . | . | . |
| 21-225_96B5_HC | . | . | . | . | . | . | P | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | H | L | I | A | V | - | - | - | - | - | - | - | - | - | - | - | - | - | - | - | - | - | - |
| 21-225_10B10_HC | . | . | . | R | I | A | . | . | . | . | . | . | . | . | . | . | . | . | . | . | . | . | . | . | . | . |
| 21-225_15H11_HC | . | . | . | R | I | V | . | . | . | . | . | . | . | . | . | . | . | . | . | . | . | . | . | . | . | . |
| 21-225_17F5_HC | S | . | . | . | I | . | . | . | . | . | . | . | . | . | . | . | . | . | . | . | . | . | . | . | . | . |
| 21-225_30H6_HC | . | . | . | . | . | . | . | . | . | . | . | . | . | . | . | . | . | . | . | . | . | . | . | . | . | . |
| 21-225_31C2_HC | . | . | . | . | . | . | . | . | . | . | . | . | . | . | . | . | . | . | . | . | . | . | . | . | . | . |
| 21-225_57D9_HC | . | . | . | . | A | V | . | . | . | . | . | . | . | . | . | . | . | . | . | . | . | . | . | . | . | . |
| 21-225_60D6_HC | . | . | . | . | A | V | . | . | . | . | . | . | . | . | . | . | . | . | . | . | . | . | . | . | . | . |
| 21-225_74G9_HC | . | . | . | . | . | . | . | . | . | . | . | . | . | . | . | . | . | . | . | . | . | . | . | . | . | . |
| 21-225_75F11_HC | . | . | . | . | . | . | . | . | . | . | . | . | . | . | . | . | . | . | . | . | . | . | . | . | . | . |
| 21-225_76B4_HC | . | . | . | . | . | . | . | . | . | . | . | . | . | . | . | . | . | . | . | . | . | . | . | . | . | . |
| 21-225_77A2_HC | . | . | . | . | . | . | . | . | . | . | . | . | . | . | . | . | . | . | . | . | . | . | . | . | . | . |
| 21-225_96B5_HC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | A | F | D | Y | W | G | Q | G | T | L | V | T | V | S | S |
| 21-225_10B10_HC | | | | | | | | | | | | | | | | | | | |
| 21-225_15H11_HC | | | | | | | | | | | | | | | | | | | |
| 21-225_17F5_HC | | | | | | | | | | | | | | | | | | | |
| 21-225_30H6_HC | | | | | | | | | | | | | | | | | | | |
| 21-225_31C2_HC | | | | | S | | | | | | | | S | | | | | | |
| 21-225_57D9_HC | | | | | S | | | | | | | | | | | | | | |
| 21-225_60D6_HC | | | | | S | | | | | | | | | | | | | | |
| 21-225_74G9_HC | | | | | | | | | | | | | | | | | | | |
| 21-225_75F11_HC | | | | | | | | | | | | | | | | | | | |
| 21-225_76B4_HC | | | | | | | | | | | | | | | | | | | |
| 21-225_77A2_HC | | | | | | | | | | | | | | | | | | | |
| 21-225_96B5_HC | | | | | | | | | | | | | | | | | | | |

**FIGURE 57**

Table 80.    Consensus 46 - VH3|3-33/D3|3-22|RF2/JH4 (SEQ ID NO: 50297):

QVQLVES-GGGVVQPGRSLRLSCAASG-FTFS<u>S</u>-----<u>YGMH</u>WVRQAPGKGLEWVA<u>II</u>WYD---<u>GSYKYY</u>ADSVKGRFTISRDNSKNTLYLQMNSLRAEDTAVYYCAR<u>EAYDFW</u>----------------SGYFDYWGQGTLVTVSS wherein:

S at position 33 can be substituted with N or T

I at position 57 can be substituted with V

Y at position 67 can be substituted with N

A at position 71 can be substituted with V

A at position 110 can be substituted with G, R, or N

Y at position 135 can be substituted with F or H

F at position 136 can be substituted with L, Y, or W

D at position 137 can be substituted with G

Y at position 138 can be substituted with S

Xaa Tyr Gly Met His (SEQ ID NO: 50084)

Xaa Ile Trp Tyr Asp Gly Ser Xaa Lys Tyr Tyr Xaa Asp Ser Val Lys Gly (SEQ ID NO: 50085)

Glu Xaa Tyr Asp Phe Trp Ser Gly Xaa Xaa Xaa Xaa (SEQ ID NO: 50086)

Ser Tyr Gly Met His (SEQ ID NO: 50561)

Ile Ile Trp Tyr Asp Gly Ser Tyr Lys Tyr Tyr Ala Asp Ser Val Lys Gly (SEQ ID NO: 50562)

Glu Ala Tyr Asp Phe Trp Ser Gly Tyr Phe Asp Tyr (SEQ ID NO: 50563)

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_152C11_HC | . | . | . | . | . | . | . | . | . | . | . | . | . | . | . | . | . | . | . | . | . | . | . | . | . | . |
| 21-225_152F6_HC | . | . | . | . | . | . | . | . | . | . | . | . | . | . | . | . | . | . | . | . | . | . | . | . | . | . |
| 21-225_153D2_HC | . | . | . | . | . | . | . | . | . | . | . | . | . | . | . | . | . | . | . | . | . | . | . | . | . | . |
| 21-225_157G8_HC | . | . | . | . | . | . | . | . | . | . | . | . | . | . | . | . | . | . | . | . | . | . | . | . | . | . |
| 21-225_160B1_HC | . | . | . | . | . | . | . | . | . | . | . | . | . | . | . | . | . | . | . | . | . | . | . | . | . | . |
| 21-225_172B7_HC | . | . | . | . | . | . | . | . | . | . | . | . | . | . | . | . | . | . | . | . | . | . | . | . | . | . |
| 21-225_173A11_HC | . | . | . | . | . | . | . | . | . | . | . | . | . | . | . | . | . | . | . | . | . | . | . | . | . | . |
| 21-225_178G10_HC | . | . | . | . | . | . | . | . | . | . | . | . | . | . | . | . | . | . | . | . | . | . | . | . | . | . |
| 21-225_188G11_HC | . | . | . | . | . | . | . | . | . | . | . | . | . | . | . | . | . | . | . | . | . | . | . | . | . | . |
| 21-225_226E7_HC | . | . | . | . | . | . | . | . | . | . | . | . | . | . | . | . | . | . | . | . | . | . | . | . | . | . |
| 21-225_25H9_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | G | M | H | W | V | R | Q | A | P | G | K | G | L |
| 21-225_152C11_HC | . | . | . | . | . | . | N | . | . | . | . | . | . | . | . | . | . | . | . | . | . | . | . | . | . | . |
| 21-225_152F6_HC | . | . | . | . | . | . | . | . | . | . | . | . | . | . | . | . | . | . | . | . | . | . | . | . | . | . |
| 21-225_153D2_HC | . | . | . | . | . | . | . | . | . | . | . | . | . | . | . | . | . | . | . | . | . | . | . | . | . | . |
| 21-225_157G8_HC | . | . | . | . | . | . | N | . | . | . | . | . | . | . | . | . | . | . | . | . | . | . | . | . | . | . |
| 21-225_160B1_HC | . | . | . | . | . | . | . | . | . | . | . | . | . | . | . | . | . | . | . | . | . | . | . | . | . | . |
| 21-225_172B7_HC | . | . | . | . | . | . | N | . | . | . | . | . | . | . | . | . | . | . | . | . | . | . | . | . | . | . |
| 21-225_173A11_HC | . | . | . | . | . | . | . | . | . | . | . | . | . | . | . | . | . | . | . | . | . | . | . | . | . | . |
| 21-225_178G10_HC | . | . | . | . | . | . | T | . | . | . | . | . | . | . | . | . | . | . | . | . | . | . | . | . | . | . |
| 21-225_188G11_HC | . | . | . | . | . | . | . | . | . | . | . | . | . | . | . | . | . | . | . | . | . | . | . | . | . | . |
| 21-225_226E7_HC | . | . | . | . | . | . | T | . | . | . | . | . | . | . | . | . | . | . | . | . | . | . | . | . | . | . |
| 21-225_25H9_HC | . | . | . | . | . | N | N | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | I | I | W | Y | D | - | - | - | G | S | Y | K | Y | Y | A | D | S | V | K | G | R | F |
| 21-225_152C11_HC | . | . | . | . | . | . | . | . | . | . | . | . | . | . | N | . | . | . | . | . | . | . | . | . | . | . |
| 21-225_152F6_HC | . | . | . | . | . | . | . | . | . | . | . | . | . | . | . | . | . | . | . | . | . | . | . | . | . | . |
| 21-225_153D2_HC | . | . | . | . | . | . | . | . | . | . | . | . | . | . | . | . | . | . | . | . | . | . | . | . | . | . |
| 21-225_157G8_HC | . | . | . | . | . | . | . | . | . | . | . | . | . | . | . | . | . | . | . | . | . | . | . | . | . | . |
| 21-225_160B1_HC | . | . | . | . | V | . | . | . | . | . | . | . | . | . | . | . | . | . | . | . | . | . | . | . | . | . |
| 21-225_172B7_HC | . | . | . | . | . | . | . | . | . | . | . | . | . | . | N | . | . | . | . | . | V | . | . | . | . | . |
| 21-225_173A11_HC | . | . | . | . | . | . | . | . | . | . | . | . | . | . | N | . | . | . | . | . | . | . | . | . | . | . |
| 21-225_178G10_HC | . | . | . | . | V | . | . | . | . | . | . | . | . | . | N | . | . | . | . | . | . | . | . | . | . | . |
| 21-225_188G11_HC | . | . | . | T | . | . | . | . | . | . | . | . | . | . | . | . | . | . | . | . | . | . | . | . | . | . |
| 21-225_226E7_HC | . | . | . | . | . | . | . | . | . | . | . | . | . | . | N | . | . | . | . | . | . | . | . | . | . | . |
| 21-225_25H9_HC | . | . | . | S | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_152C11_HC | . | . | . | . | . | . | . | . | . | . | . | . | . | . | . | . | . | . | . | . | . | . | . | . | . | . |
| 21-225_152F6_HC | . | . | . | . | . | . | . | . | . | . | . | . | . | . | . | . | . | . | . | . | . | . | . | . | . | . |
| 21-225_153D2_HC | . | . | . | . | . | . | . | . | . | . | V | . | . | . | . | . | . | . | . | . | . | . | . | . | . | . |
| 21-225_157G8_HC | . | V | . | . | . | . | . | . | . | . | . | . | . | . | . | . | . | . | . | . | . | . | . | . | . | . |
| 21-225_160B1_HC | . | . | . | . | . | . | . | . | . | . | . | . | . | . | . | . | . | . | . | . | . | . | . | . | . | . |
| 21-225_172B7_HC | . | . | . | . | . | . | . | . | . | . | . | . | . | . | . | . | . | . | . | . | . | . | . | . | . | . |
| 21-225_173A11_HC | . | . | . | . | . | . | . | . | . | . | . | . | . | . | . | . | . | . | . | . | . | . | . | . | . | . |
| 21-225_178G10_HC | . | . | . | . | . | . | R | . | . | . | . | . | . | . | . | . | . | . | . | . | . | . | . | . | . | . |
| 21-225_188G11_HC | . | . | . | . | . | . | . | . | . | . | . | P | . | . | . | . | . | . | . | . | . | . | . | . | . | . |
| 21-225_226E7_HC | . | . | . | . | . | . | P | . | . | . | . | . | . | . | M | . | . | . | . | . | O | . | . | . | . | . |
| 21-225_25H9_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
|  |  |  |  |  |  |  |  |  |  |  |  |  |  |  |  |  |  |  | H_CDR3 |  |  |  |  |  |  |  |
| CONSENSUS | Y | C | A | R | E |  | Y | D | F | W | - | - | - | - | - | - | - | - | - | - | - | - | - | - | - | - |
| 21-225_152C11_HC | . | . | . | . | . | A | . | . | . | . | . | . | . | . | . | . | . | . | . | . | . | . | . | . | . | . |
| 21-225_152F6_HC | . | . | . | . | . | G | . | . | . | . | . | . | . | . | . | . | . | . | . | . | . | . | . | . | . | . |
| 21-225_153D2_HC | . | . | . | . | . | G | . | . | . | . | . | . | . | . | . | . | . | . | . | . | . | . | . | . | . | . |
| 21-225_157G8_HC | . | . | . | . | . | G | . | . | . | . | . | . | . | . | . | . | . | . | . | . | . | . | . | . | . | . |
| 21-225_160B1_HC | . | . | . | . | . | A | . | . | . | . | . | . | . | . | . | . | . | . | . | . | . | . | . | . | . | . |
| 21-225_172B7_HC | . | . | . | . | . | A | . | . | . | . | . | . | . | . | . | . | . | . | . | . | . | . | . | . | . | . |
| 21-225_173A11_HC | . | . | . | . | . | A | . | . | . | . | . | . | . | . | . | . | . | . | . | . | . | . | . | . | . | . |
| 21-225_178G10_HC | . | . | . | . | . | X | . | . | . | . | . | . | . | . | . | . | . | . | . | . | . | . | . | . | . | . |
| 21-225_188G11_HC | . | . | . | . | . | X | . | . | . | . | . | . | . | . | . | . | . | . | . | . | . | . | . | . | . | . |
| 21-225_226E7_HC | . | . | . | . | . | N | . | . | . | . | . | . | . | . | . | . | . | . | . | . | . | . | . | . | . | . |
| 21-225_25H9_HC | . | . | . | . | . | G | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  | H_FR4 |  |  |  |  |  |  |  |  |  |  |
| CONSENSUS | - | - | S | G | Y | F | D | Y | W | G | Q | G | T | L | V | T | V | S | S |
| 21-225_152C11_HC | . | . | . | . | . | . | . |  | . | . | . | . | I | . | . | . | . | . | . |
| 21-225_152F6_HC | . | . | . | . | . | L | G | . | . | . | . | . | . | . | . | . | . | . | . |
| 21-225_153D2_HC | . | . | . | . | . | L | G | . | . | . | . | . | . | . | . | . | . | . | . |
| 21-225_157G8_HC | . | . | . | . | P | . | . | S | . | . | . | . | . | . | . | . | . | . | . |
| 21-225_160B1_HC | . | . | . | . | . | Y | . | . | . | . | . | . | . | . | . | . | . | . | . |
| 21-225_172B7_HC | . | . | . | . | . | W | . | . | . | . | . | . | . | . | . | . | . | . | . |
| 21-225_173A11_HC | . | . | . | . | P | . | . | S | . | . | . | . | . | . | . | . | . | . | . |
| 21-225_178G10_HC | . | . | . | . | X | . | . | . | . | . | . | . | . | . | . | . | . | . | . |
| 21-225_188G11_HC | . | . | . | . | X | . | . | . | . | . | . | . | . | . | . | . | . | . | . |
| 21-225_226E7_HC | . | . | . | . | . | Y | G | . | . | . | . | . | . | . | . | . | . | . | . |
| 21-225_25H9_HC | . | . | . | . | P | . | . | S | . | . | . | . | . | . | . | . | . | . | . |

**FIGURE 57**

Table 81.        Consensus 47 -VH3|3-33/D5|5-18|RF3/JH6 (SEQ ID NO: 50298):

QVQLVES-GGGVVQPGRSLRLSCAASG-FTFSS-----YGMHWVRQAPGKGLEWVAVIWYD---
GSNKYYADSVKGRFTISRDNSKNTLYLQMNSLRAEDTAVYYCARDRDYGD------------------YGMDVWGQGTTVTVSS wherein:

M at position 41 can be substituted with L

Y at position 69 can be substituted with N

A at position 71 can be substituted with E

D at position 109 can be substituted with W

R at position 110 can be substituted with R, G or Y

D at position 111 can be substituted with S or Y

G at position 113 can be substituted with Y

D at position 114 can be substituted with Y

null (-) at position 115 can be substituted with P

null (-) at position 116 can be substituted with P

null (-) at position 117 can be substituted with Y

null (-) at position 131 can be substituted with Y

null (-) at position 132 can be substituted with Y

null (-) at position 133 can be substituted with Y

Y at position 134 can be substituted with D

Ser Tyr Gly Xaa His (SEQ ID NO: 50087)

Val Ile Trp Tyr Asp Gly Ser Asn Lys Xaa Tyr Xaa Asp Ser Val Lys Gly (SEQ ID NO: 50088)

**FIGURE 57**

Xaa Xaa Xaa Tyr Xaa Xaa Xaa Xaa Xaa Xaa Xaa Xaa Xaa Gly Met Asp Val (SEQ ID NO: 50089)

Ser Tyr Gly Met His (SEQ ID NO: 50564)

Val Ile Trp Tyr Asp Gly Ser Asn Lys Tyr Tyr Ala Asp Ser Val Lys Gly (SEQ ID NO: 50565)

Asp Arg Asp Tyr Gly Asp Tyr Gly Met Asp Val (SEQ ID NO: 50566)

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_147B9_HC | . | . | . | . | . | . | . | . | . | . | . | . | . | . | . | . | . | . | . | . | . | . | . | . | . | . |
| 21-225_148C8_HC | . | . | . | . | . | . | . | . | . | . | . | . | . | . | . | . | . | . | . | . | . | . | . | . | . | . |
| 21-225_149A11_HC | . | . | . | . | . | . | . | . | . | . | . | . | . | . | . | . | . | . | . | . | . | . | . | . | . | . |
| 21-225_155E10_HC | . | . | . | . | . | . | . | . | . | . | . | . | . | . | . | . | . | . | . | . | . | . | . | . | . | . |
| 21-225_190C3_HC | . | . | . | . | . | . | . | . | . | . | . | . | . | . | . | . | . | . | . | . | . | . | . | . | . | . |
| 21-225_190D8_HC | . | . | . | . | . | . | . | . | . | . | D | . | . | . | . | . | . | . | . | . | . | . | . | . | E | . |
| 21-225_211A3_HC | . | . | . | . | . | . | . | . | . | . | . | . | . | . | . | . | . | . | . | . | . | . | . | . | V | . |
| 21-225_212H11_HC | . | . | . | . | . | . | . | . | . | . | . | . | . | . | . | . | . | . | . | . | . | . | . | . | V | . |
| 21-225_213E5_HC | . | . | . | . | . | . | . | . | . | . | . | . | . | . | . | . | . | . | . | . | . | . | . | . | V | . |
| 21-225_217B10_HC | . | . | . | . | . | . | . | . | . | . | . | . | . | . | . | . | . | . | . | . | . | . | . | . | V | . |
| 21-225_218G4_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | H_CDR1 | | | | | | | | | | | | | | | H_FR2 | | | |
| CONSENSUS | G | - | F | T | F | S | S | - | - | - | - | - | Y | G | M | H | W | V | R | Q | A | P | G | K | G | L |
| 21-225_147B9_HC | . | . | . | . | . | . | . | . | . | . | . | . | . | . | . | . | . | . | . | . | . | . | . | . | . | . |
| 21-225_148C8_HC | . | . | . | . | . | . | . | . | . | . | . | . | . | . | . | . | . | . | . | . | . | . | . | . | . | . |
| 21-225_149A11_HC | . | . | . | . | . | . | . | . | . | . | . | . | . | . | . | . | . | . | . | . | . | . | . | . | . | . |
| 21-225_155E10_HC | . | . | . | . | . | . | . | . | . | . | . | . | . | . | . | . | . | . | . | . | . | . | . | . | . | . |
| 21-225_190C3_HC | . | . | . | . | . | . | . | . | . | . | . | . | . | . | . | . | . | . | . | . | . | . | . | . | . | . |
| 21-225_190D8_HC | . | . | . | . | . | . | . | . | . | . | . | . | . | L | . | . | . | . | . | . | . | . | . | . | . | . |
| 21-225_211A3_HC | . | . | I | . | . | . | . | . | . | . | . | . | . | . | . | . | . | A | . | . | . | . | . | . | . | . |
| 21-225_212H11_HC | . | . | I | . | . | . | . | . | . | . | . | . | . | . | . | . | . | A | . | . | . | . | . | . | . | . |
| 21-225_213E5_HC | . | . | I | . | . | . | . | . | . | . | . | . | . | . | . | . | . | A | . | . | . | . | . | . | . | . |
| 21-225_217B10_HC | . | . | I | . | . | . | . | . | . | . | . | . | . | . | . | . | . | A | . | . | . | . | . | . | . | . |
| 21-225_218G4_HC | . | . | . | . | . | . | . | . | . | . | . | . | . | L | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | G | S | N | K | Y | Y | A | D | S | V | K | G | R | F |
| 21-225_147B9_HC | . | . | . | . | . | . | . | . | . | . | . | . | . | . | . | . | . | . | . | . | . | . | . | . | . | . |
| 21-225_148C8_HC | . | . | . | . | . | . | . | . | . | . | . | . | . | . | . | . | . | . | . | . | . | . | . | . | . | . |
| 21-225_149A11_HC | . | . | . | . | . | . | . | . | . | . | . | . | . | . | . | . | . | . | . | . | . | . | . | . | . | . |
| 21-225_155E10_HC | . | . | . | . | . | . | . | . | . | . | . | . | . | . | . | . | . | . | . | . | . | . | . | . | . | . |
| 21-225_190C3_HC | . | . | . | . | . | . | . | . | . | . | . | . | . | . | . | . | . | . | . | . | . | . | . | . | . | . |
| 21-225_190D8_HC | . | . | . | . | . | . | . | . | . | . | . | . | . | . | . | . | . | . | . | . | . | . | . | . | . | . |
| 21-225_211A3_HC | . | . | . | . | . | . | . | . | . | . | . | . | . | . | . | . | N | . | S | . | . | . | . | . | . | . |
| 21-225_212H11_HC | . | . | . | . | . | . | . | . | . | . | . | . | . | . | . | . | N | . | S | . | . | . | . | . | . | . |
| 21-225_213E5_HC | . | . | . | . | . | . | . | . | . | . | . | . | . | . | . | . | N | . | S | . | . | . | . | . | . | . |
| 21-225_217B10_HC | . | . | . | . | . | . | . | . | . | . | . | . | . | . | . | . | N | . | S | . | . | . | . | . | . | . |
| 21-225_218G4_HC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_147B9_HC | . | . | . | . | . | . | . | . | . | . | . | . | . | . | . | . | . | . | . | . | . | . | . | . | . | . |
| 21-225_148C8_HC | . | . | . | . | . | . | . | . | . | . | . | . | . | . | . | . | . | . | . | . | . | . | . | . | . | . |
| 21-225_149A11_HC | . | . | . | . | . | . | . | . | . | . | . | . | . | . | . | . | . | . | . | . | . | . | . | . | . | . |
| 21-225_155E10_HC | . | . | . | . | . | . | . | . | . | . | . | . | . | . | . | . | . | . | . | . | . | . | . | . | . | . |
| 21-225_190C3_HC | . | . | . | . | . | . | . | . | . | . | . | . | . | . | . | . | . | . | . | . | . | . | . | . | . | . |
| 21-225_190D8_HC | . | . | . | . | . | . | . | . | . | . | . | . | . | . | . | . | . | . | . | . | . | . | . | . | . | . |
| 21-225_211A3_HC | . | . | . | . | . | . | . | . | . | . | . | . | . | . | . | . | . | . | . | . | . | . | . | . | . | . |
| 21-225_212H11_HC | . | . | . | . | . | . | . | . | . | . | . | . | . | . | . | . | . | . | . | . | . | . | . | . | . | . |
| 21-225_213E5_HC | . | . | . | . | . | . | . | . | . | . | . | . | . | . | . | . | . | . | . | . | . | . | . | . | . | . |
| 21-225_217B10_HC | . | . | . | . | . | . | . | . | . | . | . | . | . | . | . | . | . | . | . | . | . | . | . | . | . | . |
| 21-225_218G4_HC | . | . | . | . | . | . | . | . | . | . | . | . | . | . | . | . | . | . | V | . | . | . | . | . | . | . |

4144

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | | | Y | G | | - | - | - | - | - | - | - | - | - | - | - | - | - | - | - | - |
| 21-225_147B9_HC | . | . | . | . | . | R | G | . | . | D | P | P | Y | . | . | . | . | . | . | . | . | . | . | . | . | . |
| 21-225_148C8_HC | . | . | . | . | . | R | G | . | . | D | P | P | Y | . | . | . | . | . | . | . | . | . | . | . | . | . |
| 21-225_149A11_HC | . | . | . | . | . | R | G | . | . | D | P | P | Y | . | . | . | . | . | . | . | . | . | . | . | . | . |
| 21-225_155E10_HC | . | . | . | . | . | R | G | . | . | D | P | P | Y | . | . | . | . | . | . | . | . | . | . | . | . | . |
| 21-225_190C3_HC | . | . | . | N | W | Y | Y | . | Y | - | . | . | . | . | . | . | . | . | . | . | . | . | . | . | . | . |
| 21-225_190D8_HC | . | . | . | N | W | Y | Y | . | Y | - | . | . | . | . | . | . | . | . | . | . | . | . | . | . | . | . |
| 21-225_211A3_HC | . | . | . | . | . | G | S | . | . | Y | . | . | . | . | . | . | . | . | . | . | . | . | . | . | . | . |
| 21-225_212H11_HC | . | . | . | . | . | G | S | . | . | Y | . | . | . | . | . | . | . | . | . | . | . | . | . | . | . | . |
| 21-225_213E5_HC | . | . | . | . | . | G | S | . | . | Y | . | . | . | . | . | . | . | . | . | . | . | . | . | . | . | . |
| 21-225_217B10_HC | . | . | . | . | . | G | S | . | . | Y | . | . | . | . | . | . | . | . | . | . | . | . | . | . | . | . |
| 21-225_218G4_HC | . | . | . | N | W | Y | Y | . | Y | - | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | Y | G | M | D | V | W | G | Q | G | T | T | V | T | V | S | S |
| 21-225_147B9_HC | Y | Y | Y | . | . | . | . | . | . | . | . | . | . | . | . | . | . | . | . |
| 21-225_148C8_HC | Y | Y | Y | . | . | . | . | . | . | . | . | . | . | . | . | . | . | . | . |
| 21-225_149A11_HC | Y | Y | Y | . | . | . | . | . | . | . | . | . | . | . | . | . | . | . | . |
| 21-225_155E10_HC | Y | Y | Y | . | . | . | . | . | . | . | . | . | . | . | . | . | . | . | . |
| 21-225_190C3_HC | . | . | . | . | . | . | . | . | . | . | . | . | . | . | . | . | . | . | . |
| 21-225_190D8_HC | . | . | . | . | . | . | . | . | . | . | . | . | . | . | . | . | . | . | . |
| 21-225_211A3_HC | . | . | . | D | . | . | . | . | . | . | . | . | . | . | . | . | . | . | . |
| 21-225_212H11_HC | . | . | . | D | . | . | . | . | . | . | . | . | . | . | . | . | . | . | . |
| 21-225_213E5_HC | . | . | . | D | . | . | . | . | . | . | . | . | . | . | . | . | . | . | . |
| 21-225_217B10_HC | . | . | . | D | . | . | . | . | . | . | . | . | . | . | . | . | . | . | . |
| 21-225_218G4_HC | . | . | . | . | . | . | . | . | . | . | . | . | . | . | . | . | . | . | . |

FIGURE 57

Table 82.     Consensus 48 -VH3|3-23/D4|4-17|RF2/JH6 (SEQ ID NO: 50299):

EVQLLES-GGGLVQPGGSLRLSCAASG-FTFSS-----YAMSWVRQAPGKGLEWVSAISGS---
GGNTFYADSVKGRFTISRDNSKNTLYLQMNSLRAEDTAVYYCAKLGKDYY----------------YYGMDVWGQGTTVTVSS wherein:

S at position 33 can be substituted with N or T

Y at position 39 can be substituted with S

A at position 40 can be substituted with V

S at position 42 can be substituted with T or N

A at position 57 can be substituted with G

I at position 58 can be substituted with S or V

S at position 59 can be substituted with V

S at position 61 can be substituted with R

G at position 66 can be substituted with A, S or V

N at position 67 can be substituted with R or K

F at position 69 can be substituted with Y

Y at position 70 can be substituted with N

A at position 71 can be substituted with T

K at position 75 can be substituted with T

L at position 109 can be substituted with D or E

G at position 110 can be substituted with R

K at position 111 can be substituted with G or I

FIGURE 57

D at position 112 can be substituted with Q or Y

Y at position 113 can be substituted with W

Y at position 114 can be substituted with H or L

Y at position 133 can be substituted with null (-), I, or L

Y at position 134 can be substituted with G

M at position 136 can be substituted with V

Xaa Xaa Xaa Met Xaa (SEQ ID NO: 50090)

Xaa Xaa Xaa Gly Xaa Gly Xaa Xaa Thr Xaa Xaa Xaa Asp Ser Val Xaa Gly (SEQ ID NO: 50091)

Xaa Xaa Xaa Xaa Xaa Xaa Xaa Xaa Gly Xaa Asp Val (SEQ ID NO: 50092)

Ser Tyr Ala Met Ser (SEQ ID NO: 50567)

Ala Ile Ser Gly Ser Gly Gly Asn Thr Phe Tyr Ala Asp Ser Val Lys Gly (SEQ ID NO: 50568)

Leu Gly Lys Asp Tyr Tyr Tyr Tyr Gly Met Asp Val (SEQ ID NO: 50569)

FIGURE 57

| Reference # | | | | | | | | | | | | | | | H_FR1 | | | | | | | | | |
|---|---|---|---|---|---|---|---|---|---|---|---|---|---|---|---|---|---|---|---|---|---|---|---|---|
| CONSENSUS | E | V | Q | L | L | E | S | - | G | G | G | L | V | Q | P | G | G | S | L | R | L | S | C | A | A | S |
| 21-225_171A9_HC | . | . | . | . | . | . | . | . | . | . | . | A | . | . | . | . | . | . | . | . | . | . | . | . | . |
| 21-225_224B1_HC | . | . | V | . | . | . | . | . | . | . | . | . | . | . | . | S | . | . | . | . | . | . | . | . | . |
| 21-225_44E10_HC | . | . | . | . | . | . | . | . | . | . | . | . | . | . | . | S | . | . | . | . | . | . | . | . | . |
| 21-225_47C7_HC | . | . | . | . | . | . | . | . | . | . | . | . | . | . | . | . | . | . | . | . | . | . | . | . | . |
| 21-225_54G3_HC | . | . | . | . | . | . | . | . | . | . | . | . | . | . | . | . | . | . | . | . | . | . | . | . | . |
| 21-225_57F8_HC | . | . | . | . | . | . | . | . | . | . | . | . | . | . | . | . | . | . | . | . | . | . | . | V | V |
| 21-225_5A4_HC | . | . | V | . | . | . | . | . | . | . | . | . | . | . | . | . | . | . | . | . | . | . | . | . | . |
| 21-225_60A11_HC | . | . | . | . | . | . | . | . | . | . | . | . | . | . | . | . | . | . | . | . | . | . | . | . | . |
| 21-225_62F7_HC | . | . | . | . | . | . | . | . | . | . | . | . | . | . | . | . | . | . | . | . | . | . | . | . | . |
| 21-225_66C10_HC | . | . | . | . | . | . | . | . | . | . | . | . | . | . | . | . | . | . | . | . | . | . | . | . | . |

| Reference # | | | | | | | | H_CDR1 | | | | | | | | | | H_FR2 | | | | | | |
|---|---|---|---|---|---|---|---|---|---|---|---|---|---|---|---|---|---|---|---|---|---|---|---|---|
| CONSENSUS | G | - | F | T | F | S | S | - | - | - | - | Y | A | M | S | W | V | R | Q | A | P | G | K | G | L |
| 21-225_171A9_HC | . | . | . | . | . | . | . | . | . | . | . | . | . | . | T | . | . | . | . | . | . | . | . | . | . |
| 21-225_224B1_HC | . | . | . | . | . | . | . | . | . | . | . | . | . | . | N | . | . | . | . | . | . | . | . | . | . |
| 21-225_44E10_HC | . | . | . | . | . | . | . | . | . | . | . | . | . | . | N | . | . | . | . | . | . | . | . | . | . |
| 21-225_47C7_HC | . | . | . | . | . | N | . | . | . | . | . | . | . | . | . | . | . | . | . | . | . | . | . | . | . |
| 21-225_54G3_HC | . | . | . | . | . | T | . | . | . | . | . | . | . | . | . | . | . | . | . | . | . | . | . | . | . |
| 21-225_57F8_HC | . | . | . | . | . | N | . | . | . | . | . | . | . | . | . | . | . | . | . | . | . | . | . | . | . |
| 21-225_5A4_HC | . | L | . | . | . | . | . | . | . | . | . | S | . | . | . | . | . | . | . | . | . | . | . | . | . |
| 21-225_60A11_HC | . | . | . | . | . | N | . | . | . | . | . | . | . | . | T | . | . | . | . | . | . | . | . | . | . |
| 21-225_62F7_HC | . | . | . | . | . | . | . | . | . | . | . | . | V | . | . | . | . | . | . | . | . | . | . | . | . |
| 21-225_66C10_HC | . | . | . | . | . | . | . | . | . | . | . | . | V | . | . | . | . | . | . | . | . | . | . | . | . |

**FIGURE 57**

| Reference # | | | | | | | | | | | | | | | | H_CDR2 | | | | | | | | | |
|---|---|---|---|---|---|---|---|---|---|---|---|---|---|---|---|---|---|---|---|---|---|---|---|---|---|
| CONSENSUS | E | W | V | S | A | I | S | G | S | - | - | - | G | G | N | T | | Y | A | D | S | V | K | G | R | F |
| 21-225_171A9_HC | . | . | . | . | . | . | . | . | . | . | . | . | . | . | . | . | F | N | . | . | . | . | . | . | . |
| 21-225_224B1_HC | . | . | . | . | . | . | . | . | . | . | . | . | . | . | . | . | Y | . | . | . | . | . | . | . | . |
| 21-225_44E10_HC | . | . | . | G | V | V | . | . | . | . | . | . | . | . | R | . | Y | . | . | . | . | . | . | . | . |
| 21-225_47C7_HC | . | . | . | G | . | . | . | . | . | . | . | . | S | R | . | Y | . | . | . | . | . | . | . | . |
| 21-225_54G3_HC | . | . | . | . | . | . | . | . | . | . | . | . | V | . | . | F | . | . | . | . | . | . | . | . |
| 21-225_57F8_HC | . | . | . | . | S | . | . | . | . | . | . | . | . | . | . | F | . | . | . | . | . | T | . | . | V |
| 21-225_5A4_HC | . | . | . | . | . | . | . | R | . | . | . | . | . | . | F | . | T | . | . | . | . | . | . | . |
| 21-225_60A11_HC | . | . | . | . | . | . | . | . | . | . | . | . | . | . | F | . | . | . | . | . | T | . | . | V |
| 21-225_62F7_HC | . | . | . | G | . | . | . | . | . | . | . | . | A | R | . | Y | . | . | . | . | . | . | . | . |
| 21-225_66C10_HC | . | . | . | G | . | . | . | . | . | . | . | . | A | R | . | Y | . | . | . | . | . | . | . | . |

| Reference # | | | | | | | | | | | | | H_FR3 | | | | | | | | | | | |
|---|---|---|---|---|---|---|---|---|---|---|---|---|---|---|---|---|---|---|---|---|---|---|---|---|
| CONSENSUS | T | I | S | R | D | N | S | K | N | T | L | Y | L | Q | M | N | S | L | R | A | E | D | T | A | V | Y |
| 21-225_171A9_HC | . | . | . | . | . | . | . | . | . | . | . | . | . | . | . | . | . | . | . | . | . | . | . | . | . | . |
| 21-225_224B1_HC | . | . | . | . | . | . | . | . | . | . | . | . | . | N | . | . | . | . | . | . | . | . | . | . | . | . |
| 21-225_44E10_HC | I | . | . | . | . | . | . | . | . | . | . | . | . | . | . | . | . | . | . | . | . | . | . | . | . | . |
| 21-225_47C7_HC | . | . | . | . | . | . | . | . | . | . | . | . | . | . | . | . | . | . | . | . | . | . | . | . | . | . |
| 21-225_54G3_HC | . | . | . | . | . | . | . | . | . | . | . | F | . | . | . | . | . | . | . | . | . | . | . | . | . | . |
| 21-225_57F8_HC | . | . | . | . | . | . | . | . | . | . | . | . | . | . | . | . | . | . | . | . | . | . | . | . | . | . |
| 21-225_5A4_HC | . | . | . | . | . | . | . | . | . | . | . | . | . | . | . | . | . | . | . | . | . | . | . | . | . | . |
| 21-225_60A11_HC | . | . | . | . | . | . | . | . | . | . | . | . | . | . | . | . | . | . | . | . | . | . | . | . | . | . |
| 21-225_62F7_HC | . | . | . | . | . | . | . | . | . | . | . | . | . | . | . | . | . | . | . | . | . | . | . | . | . | . |
| 21-225_66C10_HC | . | . | . | . | . | . | . | . | . | . | . | . | . | . | . | . | . | . | . | . | . | . | . | . | . | . |

## FIGURE 57

| Reference # | | | | | | | | | | | | | | | | | | | H_CDR3 | | | | | | |
|---|---|---|---|---|---|---|---|---|---|---|---|---|---|---|---|---|---|---|---|---|---|---|---|---|---|
| CONSENSUS | Y | C | A | K | L | G | K | D | Y | Y | - | - | - | - | - | - | - | - | - | - | - | - | - | - | - |
| 21-225_171A9_HC | . | | . | . | . | . | ! | . | | | | | | | | | | | | | | | | | |
| 21-225_224B1_HC | . | | . | Q | E | . | Q | Y | | | | | | | | | | | | | | | | | |
| 21-225_44E10_HC | | | . | D | R | G | Q | W | L | | | | | | | | | | | | | | | | |
| 21-225_47C7_HC | . | | . | O | R | G | Q | W | L | | | | | | | | | | | | | | | | |
| 21-225_54G3_HC | . | | . | . | . | . | . | . | | | | | | | | | | | | | | | | | |
| 21-225_57F8_HC | . | | . | . | . | . | . | X | | | | | | | | | | | | | | | | | |
| 21-225_5A4_HC | . | | . | . | . | . | . | . | | | | | | | | | | | | | | | | | |
| 21-225_60A11_HC | . | | . | . | . | . | . | H | | | | | | | | | | | | | | | | | |
| 21-225_62F7_HC | . | | E | . | . | ! | . | . | | | | | | | | | | | | | | | | | |
| 21-225_66C10_HC | . | | E | . | . | ! | . | . | | | | | | | | | | | | | | | | | |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | H_FR4 | | | | | | | | | | | | |
| CONSENSUS | - | - | Y | Y | G | M | D | V | W | G | Q | G | T | T | V | T | V | S | S |
| 21-225_171A9_HC | . | . | . | . | . | . | . | . | . | . | . | . | . | . | . | . | . | . | . |
| 21-225_224B1_HC | . | . | . | . | V | . | . | . | . | . | . | . | . | . | . | . | . | . | . |
| 21-225_44E10_HC | . | . | L | G | . | . | . | . | . | . | . | . | . | . | . | . | . | . | . |
| 21-225_47C7_HC | . | . | I | G | . | . | . | . | . | . | . | . | . | . | . | . | . | . | . |
| 21-225_54G3_HC | . | . | . | . | . | . | . | . | . | . | . | . | . | . | . | . | . | . | . |
| 21-225_57F8_HC | . | . | . | . | . | . | . | . | . | . | . | . | . | . | . | . | . | . | . |
| 21-225_5A4_HC | . | . | . | . | . | . | . | . | . | . | . | . | . | . | . | . | . | . | . |
| 21-225_60A11_HC | . | . | . | . | . | . | . | . | . | . | . | . | . | . | . | . | . | . | . |
| 21-225_62F7_HC | . | . | - | . | . | . | . | . | . | . | . | . | . | . | . | . | . | . | . |
| 21-225_66C10_HC | . | . | - | . | . | . | . | . | . | . | . | . | . | . | . | . | . | . | . |

**FIGURE 57**

Table 83.      Consensus 49 -VH3|3-23/D7|7-27|RF1/JH4 (SEQ ID NO: 50300):

EVQLLES-GGGLVQPGGSLRLSCAASG-FTFSS-----YVMSWVRQAPGKGLEWVSAMSGS---
GGRTYYADSVKGRFTISRDNSKNTLYLQMNSLRAEDTAVYYCAKLTA----------------------FDYWGQGTLVTVSS wherein:

V at position 40 can be substituted with A

S at position 42 can be substituted with N

A at position 57 can be substituted with G, T or S

M at position 58 can be substituted with I or T

G at position 66 can be substituted with N or V

R at position 67 can be substituted with N or W

Y at position 69 can be substituted with F or N

K at position 75 can be substituted with N

G at position 76 can be substituted with D

L at position 109 can be substituted with V, Y or F

T at position 110 can be substituted with E, F or G

A at position 111 can be substituted with G, L, W, null (-) or F

null (-) at position 112 can be substituted with G or M

null (-) at position 113 can be substituted with V

null (-) at position 114 can be substituted with G

null (-) at position 133 can be substituted with A

null (-) at position 134 can be substituted with G

**FIGURE 57**

null (-) at position 135 can be substituted with I or F

F at position 136 can be substituted with N or null (-)

D at position 137 can be substituted with G or I

Y at position 138 can be substituted with D

Ser Tyr Xaa Met Xaa (SEQ ID NO: 50093)

Xaa Xaa Ser Gly Ser Gly Xaa Xaa Thr Xaa Tyr Ala Asp Ser Val Xaa Xaa (SEQ ID NO: 50094)

Xaa Xaa Xaa Xaa Xaa Xaa Xaa Xaa Xaa Xaa Xaa Xaa (SEQ ID NO: 50095)

Ser Tyr Val Met Ser (SEQ ID NO: 50570)

Ala Met Ser Gly Ser Gly Gly Arg Thr Tyr Tyr Ala Asp Ser Val Lys Gly (SEQ ID NO: 50571)

Leu Thr Ala Phe Asp Tyr (SEQ ID NO: 50572)

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_157G5_HC | . | | . | . | . | . | . | | . | . | . | . | . | . | | . | . | . | . | . | . | . | . | . | . | . |
| 21-225_167D12_HC | | . | . | . | . | . | . | . | . | . | . | . | . | . | | . | . | | . | . | . | . | . | . | . | . |
| 21-225_177D3_HC | . | | . | . | . | . | . | . | . | . | . | . | . | . | | . | . | . | . | . | . | . | . | . | . | . |
| 21-225_51C5_HC | . | . | . | . | . | . | . | . | . | . | . | . | . | . | . | . | . | . | . | . | . | . | . | . | . | . |
| 21-225_51D5_HC | . | . | . | . | . | . | . | . | . | . | . | . | . | . | . | . | . | . | . | . | . | . | . | . | . | . |
| 21-225_57B8_HC | . | . | . | . | . | . | . | . | . | . | . | . | . | . | . | . | . | . | . | . | . | . | . | . | . | . |
| 21-225_58F11_HC | . | . | . | . | . | . | . | . | . | . | . | . | . | . | . | . | . | . | . | . | . | . | . | . | . | . |
| 21-225_60A4_HC | . | . | . | . | . | . | . | . | . | . | . | . | . | . | . | . | . | . | . | . | . | . | . | . | . | . |
| 21-225_60F7_HC | . | . | . | . | . | . | . | . | . | . | . | . | . | . | . | . | . | . | . | . | . | . | . | . | . | . |
| 21-225_61E6_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | H_CDR1 | | | | | | | | | | | | | | | | H_FR2 | | |
| CONSENSUS | G | - | F | T | F | S | S | - | - | - | - | - | Y | V | M | S | W | V | R | Q | A | P | G | K | G | L |
| 21-225_157G5_HC | . | . | . | . | . | . | . | . | . | . | . | . | . | . | . | . | . | . | . | . | . | . | . | . | . | . |
| 21-225_167D12_HC | . | . | . | . | . | . | . | . | . | . | . | . | . | . | . | . | . | . | . | . | . | . | . | . | . | . |
| 21-225_177D3_HC | . | . | . | . | . | . | . | . | . | . | . | . | . | . | . | N | . | . | . | . | . | . | . | . | . | . |
| 21-225_51C5_HC | . | . | . | . | . | . | . | . | . | . | . | . | . | . | . | . | . | . | . | . | Y | . | . | . | . | . |
| 21-225_51D5_HC | . | . | . | . | . | . | . | . | . | . | . | . | . | . | . | . | . | . | . | . | Y | . | . | . | . | . |
| 21-225_57B8_HC | . | . | . | . | . | : | . | . | . | . | . | . | . | A | . | . | . | . | . | . | . | . | . | . | . | . |
| 21-225_58F11_HC | . | . | . | . | . | . | . | . | . | . | . | . | . | A | . | . | . | . | . | . | . | . | . | . | . | . |
| 21-225_60A4_HC | . | . | . | . | . | . | . | . | . | . | . | . | . | . | . | . | . | . | . | . | . | . | . | . | . | . |
| 21-225_60F7_HC | . | . | . | . | . | . | . | . | . | . | . | . | . | . | . | . | . | . | . | . | . | . | . | . | . | . |
| 21-225_61E6_HC | . | . | . | . | . | : | . | . | . | . | . | . | . | A | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | A | M | S | G | S | - | - | - | G | G | R | T | Y | Y | A | D | S | V | K | G | R | F |
| 21-225_157G5_HC | O | | | | . | . | . | . | . | . | . | . | . | . | . | . | . | . | . | . | . | . | . | . | . | . |
| 21-225_167D12_HC | | | . | . | . | . | . | . | . | . | . | . | . | . | . | | . | . | . | . | . | . | . | . | . | . |
| 21-225_177D3_HC | | | . | . | G | . | . | . | . | . | . | . | . | . | . | | . | . | . | . | . | . | . | O | . | . |
| 21-225_51C5_HC | | | . | . | T | . | . | . | . | . | . | . | . | . | . | | . | . | . | . | . | . | N | . | . | . |
| 21-225_51D5_HC | | | . | . | T | . | . | . | . | . | . | . | . | . | . | | . | . | . | . | . | . | N | . | . | . |
| 21-225_57B8_HC | | | . | . | . | I | . | . | . | . | . | . | . | . | N | | F | . | . | . | . | . | . | . | . | . |
| 21-225_58F11_HC | | | . | . | . | I | . | . | . | . | . | . | . | . | N | | F | . | . | . | . | . | . | . | . | . |
| 21-225_60A4_HC | | . | . | . | S | I | . | . | . | . | . | . | . | . | W | | N | . | . | . | . | . | . | . | . | . |
| 21-225_60F7_HC | | | . | . | G | I | . | . | . | . | . | . | . | N | . | | . | . | . | . | . | . | . | . | . | . |
| 21-225_61E6_HC | | | . | . | . | T | . | . | . | . | . | . | . | V | N | | F | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_157G5_HC | . | . | . | . | . | . | . | . | . | . | . | . | . | . | . | . | . | . | . | . | . | . | . | . | . | . |
| 21-225_167D12_HC | . | . | . | . | . | . | . | . | . | . | . | . | . | . | . | . | . | . | . | . | . | . | . | . | . | . |
| 21-225_177D3_HC | . | . | . | . | . | . | . | . | . | . | . | S | . | . | . | . | . | . | . | . | . | . | . | . | . | . |
| 21-225_51C5_HC | . | V | . | . | . | . | . | . | . | . | . | . | . | . | . | S | . | . | . | . | . | . | . | . | . | . |
| 21-225_51D5_HC | . | V | . | . | . | . | . | . | . | . | . | . | . | . | . | S | . | . | . | . | . | . | . | . | . | . |
| 21-225_57B8_HC | . | . | . | . | . | . | . | . | . | . | . | . | . | . | . | . | . | . | . | . | . | . | . | . | . | . |
| 21-225_58F11_HC | . | . | . | . | Y | . | . | . | . | . | . | . | . | . | . | . | . | . | . | . | . | . | . | . | . | . |
| 21-225_60A4_HC | . | T | . | . | . | . | . | . | . | . | . | . | . | . | . | . | . | . | . | . | . | . | . | . | . | . |
| 21-225_60F7_HC | . | . | . | . | . | . | . | . | . | . | . | . | . | . | . | . | . | . | . | . | . | . | . | S | . | . |
| 21-225_61E6_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | K | L | T | - | - | - | - | - | - | - | - | - | - | - | - | - | - | - | - | - | - | - | - |
| 21-225_157G5_HC | . | . | . | . | Y | . | W | . | . | . | . | . | . | . | . | . | . | . | . | . | . | . | . | . | . | . |
| 21-225_167D12_HC | . | . | . | . | Y | . | W | . | . | . | . | . | . | . | . | . | . | . | . | . | . | . | . | . | . | . |
| 21-225_177D3_HC | . | . | R | V | . | . | F | . | . | . | . | . | . | . | . | . | . | . | . | . | . | . | . | . | . | . |
| 21-225_51C5_HC | . | . | R | V | . | . | A | . | . | . | . | . | . | . | . | . | . | . | . | . | . | . | . | . | . | . |
| 21-225_51D5_HC | . | . | R | V | . | . | A | . | . | . | . | . | . | . | . | . | . | . | . | . | . | . | . | . | . | . |
| 21-225_57B8_HC | . | . | . | . | . | E | L | G | . | . | . | . | . | . | . | . | . | . | . | . | . | . | . | . | . | . |
| 21-225_58F11_HC | . | . | . | . | F | F | G | M | V | G | . | . | . | . | . | . | . | . | . | . | . | . | . | . | . | . |
| 21-225_60A4_HC | . | . | . | R | . | . | G | . | . | . | . | . | . | . | . | . | . | . | . | . | . | . | . | . | . | . |
| 21-225_60F7_HC | . | . | G | S | . | G | - | . | . | . | . | . | . | . | . | . | . | . | . | . | . | . | . | . | . | . |
| 21-225_61E6_HC | . | . | . | . | . | E | L | G | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | - | F | D | Y | W | G | Q | G | T | L | V | T | V | S | S |
| 21-225_157G5_HC | . | . | . | . | . | N | G | . | . | . | . | . | . | . | . | . | . | . | . |
| 21-225_167D12_HC | . | . | . | . | . | N | G | . | . | . | . | . | . | . | . | . | . | . | . |
| 21-225_177D3_HC | . | . | . | . | . | . | . | . | . | . | . | . | . | . | . | . | . | . | . |
| 21-225_51C5_HC | . | . | . | . | . | . | . | . | . | . | . | . | . | . | . | . | . | . | . |
| 21-225_51D5_HC | . | . | . | . | . | . | . | . | . | . | . | . | . | . | . | . | . | . | . |
| 21-225_57B8_HC | . | . | . | . | I | . | . | . | . | . | . | . | . | . | . | . | . | . | . |
| 21-225_58F11_HC | . | . | A | G | F | . | . | . | . | . | . | . | . | . | . | . | . | . | . |
| 21-225_60A4_HC | . | . | . | . | . | . | . | . | . | . | . | . | . | . | . | . | . | . | . |
| 21-225_60F7_HC | . | . | . | . | . | - | I | D | . | . | . | . | . | . | . | . | . | . | . |
| 21-225_61E6_HC | . | . | . | . | I | . | . | . | . | . | . | . | . | . | . | . | . | . | . |

4155

**FIGURE 57**

Table 84.　　Consensus 50 -VH3|3-30.3/D5|5-24|RF3/JH6 (SEQ ID NO: 50301):

QVQLVES-GGGVVQPGRSLRLSCAASG-FTFS<u>S</u>-----<u>YGMH</u>WVRQAPGKGLEWVA<u>IISYA</u>---
<u>GSNKYY</u>ADSVKGRFTISRDNSKNTLYLQMNSLRAEDTAVYYCVR<u>RGYSYG</u>-----------------G<u>YGMDV</u>WGQGTTVTVSS wherein:

S at position 33 can be substituted with Y

M at position 41 can be substituted with L

I at position 57 can be substituted with V

A at position 61 can be substituted with G, D, S or V

S at position 66 can be substituted with I, N, R or T

K at position 68 can be substituted with N or Q

Y at position 69 can be substituted with S, D or H

Y at position 70 can be substituted with S

R at position 109 can be substituted with E

G at position 110 can be substituted with D

Y at position 111 can be substituted with R

S at position 112 can be substituted with Y

Y at position 113 can be substituted with C

G at position 114 can be substituted with S

null (-) at position 115 can be substituted with G

null (-) at position 116 can be substituted with T

null (-) at position 117 can be substituted with S

FIGURE 57

null (-) at position 118 can be substituted with C

null (-) at position 129 can be substituted with P

null (-) at position 130 can be substituted with Y

null (-) at position 131 can be substituted with Y

null (-) at position 132 can be substituted with Y

G at position 133 can be substituted with Y

Xaa Tyr Gly Xaa His (SEQ ID NO: 50096)

Xaa Ile Ser Tyr Xaa Gly Xaa Asn Xaa Xaa Xaa Ala Asp Ser Val Lys Gly (SEQ ID NO: 50097)

Xaa Xaa Xaa Xaa Xaa Xaa Xaa Xaa Xaa Xaa Xaa Xaa Xaa Xaa Xaa Tyr Gly Met Asp Val (SEQ ID NO: 50098)

Ser Tyr Gly Met His (SEQ ID NO: 50573)

Ile Ile Ser Tyr Ala Gly Ser Asn Lys Tyr Tyr Ala Asp Ser Val Lys Gly (SEQ ID NO: 50574)

Arg Gly Tyr Ser Tyr Gly Gly Tyr Gly Met Asp Val (SEQ ID NO: 50575)

# EP 4 435 105 A2

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_11A2_HC | . | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | . | . | . | . | . | . | V | . |
| 21-225_1B12_HC | . | . | . | . | . | . | . | . | . | . | . | . | . | . | . | . | . | . | . | . | . | . | . | . | . |
| 21-225_225B11_HC | . | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | . | . | . | . |
| 21-225_45F6_HC | . | . | . | . | . | . | . | . | . | . | . | . | . | . | . | . | . | . | . | . | . | . | . | . | . |
| 21-225_5E6_HC | . | . | . | . | . | . | . | . | . | . | . | . | . | . | . | . | . | . | . | . | . | . | . | . | . |

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | | | | | | | H_FR2 | |
| CONSENSUS | S | G | - | F | T | F | S | S | - | - | - | - | - | Y | G | M | H | W | V | R | Q | A | P | G | K |
| 21-225_11A2_HC | . | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | . | . | . | . | . | . | . | . |
| 21-225_1B12_HC | . | . | . | . | . | . | . | . | . | . | . | . | . | . | . | . | . | . | . | . | . | . | . | . | . |
| 21-225_225B11_HC | . | . | . | . | . | . | . | . | . | . | . | . | . | . | . | . | . | . | . | . | V | . | . | . | . |
| 21-225_24D6_HC | . | . | . | . | . | . | . | . | . | . | . | . | . | . | . | . | . | . | . | . | V | . | . | . | . |
| 21-225_45F6_HC | . | . | . | . | . | . | Y | . | . | . | . | . | . | . | L | . | . | . | . | . | . | . | . | . | . |
| 21-225_5E6_HC | . | . | . | . | . | . | . | . | . | . | . | . | . | . | . | . | . | . | . | . | . | . | . | . | . |

4158

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | V | A | | I | S | Y | A | - | - | - | G | S | N | K | Y | Y | A | D | S | V | K |
| 21-225_11A2_HC | . | . | . | . | . | . | V | . | . | . | S | . | . | . | . | . | . | . | D | S | . | . | . | . | . |
| 21-225_13A1_HC | . | . | . | . | . | . | V | . | . | . | . | . | . | . | . | T | . | Q | . | . | . | . | . | . | . |
| 21-225_15C2_HC | . | . | . | . | . | S | I | . | . | . | G | . | . | . | . | . | . | N | . | . | . | . | . | . | . |
| 21-225_15F10_HC | . | . | . | . | . | S | V | . | . | . | G | . | . | . | . | . | . | N | . | . | . | . | . | . | . |
| 21-225_16B3_HC | . | . | . | . | . | . | V | . | . | . | . | . | . | . | . | I | . | . | S | . | . | . | . | . | . |
| 21-225_1B12_HC | . | . | . | . | . | . | I | . | . | . | . | . | . | . | . | . | . | . | S | . | . | . | . | . | . |
| 21-225_225B11_HC | . | . | . | . | . | T | I | . | . | . | S | . | . | . | . | . | . | . | S | . | . | . | . | . | . |
| 21-225_24D6_HC | . | . | . | . | . | . | I | . | . | . | . | . | . | . | . | . | . | . | S | . | . | . | . | . | . |
| 21-225_45F6_HC | . | . | . | . | . | . | V | . | . | . | G | . | . | . | . | . | . | . | . | . | . | . | . | . | . |
| 21-225_5E6_HC | . | . | . | . | . | . | I | . | . | . | V | . | . | . | . | N | . | . | . | . | . | . | . | . | . |

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | F | T | I | S | R | D | N | S | K | N | T | L | Y | L | Q | M | N | S | L | R | A | E | D |
| 21-225_11A2_HC | . | . | I | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | N | . | . | . | . | . | . | . | . | . | . | . | . | . | . |
| 21-225_1B12_HC | . | . | . | . | . | . | . | . | . | . | . | . | M | . | . | . | . | . | . | . | . | . | . | . | . |
| 21-225_225B11_HC | . | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | . | . | . | . |
| 21-225_45F6_HC | . | . | . | . | . | . | . | . | . | . | . | . | . | . | . | . | . | . | N | . | . | . | . | . | . |
| 21-225_5E6_HC | . | . | . | . | . | . | . | . | . | . | . | . | . | . | . | . | . | . | . | . | . | . | V | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | V | R | R | G | Y | S | Y | G | - | - | - | - | - | - | - | - | - | - | - |
| 21-225_11A2_HC | . | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | . | . | . | . | . | . | . | . |
| 21-225_1B12_HC | . | . | . | . | . | . | . | . | . | . | . | . | . | . | . | . | . | . | . | . | . | . | . | . | . |
| 21-225_225B11_HC | . | . | . | . | . | . | A | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | . | . | . | . |
| 21-225_45F6_HC | . | . | . | F | . | . | A | . | E | D | R | Y | C | S | A | T | S | C | . | . | . | . | . | . | . |
| 21-225_5E6_HC | . | . | . | . | . | . | A | . | . | . | . | . | . | . | . | . | . | . | . | . | . | . | . | . | . |

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | G | Y | G | M | D | V | W | G | Q | G | T | T | V | T | V | S | S |
| 21-225_11A2_HC | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | A | . | . | . | . | . | . |
| 21-225_1B12_HC | . | . | . | . | . | . | . | . | . | . | . | . | . | . | . | . | . | A | . | . | . | . | . | . |
| 21-225_225B11_HC | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | A | . | . | . | . | . | . |
| 21-225_45F6_HC | . | . | . | P | Y | Y | Y | Y | . | . | . | . | . | . | . | . | . | . | . | . | . | . | . | . |
| 21-225_5E6_HC | . | . | . | . | . | . | . | . | . | . | . | . | . | . | . | . | . | . | . | . | . | . | . | . |

**FIGURE 57**

Table 85.        Consensus 51 -VH3|3-33/D3|3-10|RF2/JH6 (SEQ ID NO: 50302):

QVQLVES-GGGVVQPGRSLRLSCAASG-FTFS<u>D-----YVMH</u>WVRQAPGKGLEWVA<u>VIWYD---</u>
<u>GSNKYYADSVKG</u>RFTISRDNSKNTLYLQMNSLRAEDTAVYYCAR<u>EPYTSGW</u>---------------<u>YDYGMDV</u>WGQGTTVTVSS wherein:

D at position 33 can be substituted with S

Y at position 39 can be substituted with C

V at position 40 can be substituted with G

H at position 42 can be substituted with Q

V at position 57 can be substituted with I

G at position 76 can be substituted with V

P at position 110 can be substituted with R

T at position 112 can be substituted with N

Y at position 132 can be substituted with H

M at position 136 can be substituted with L

Xaa Xaa Xaa Met Xaa (SEQ ID NO: 50099)

Xaa Ile Trp Tyr Asp Gly Ser Asn Lys Tyr Tyr Ala Asp Ser Val Lys Xaa (SEQ ID NO: 50100)

Glu Xaa Tyr Xaa Ser Gly Trp Xaa Asp Tyr Gly Xaa Asp Val (SEQ ID NO: 50101)

Asp Tyr Val Met His (SEQ ID NO: 50576)

Val Ile Trp Tyr Asp Gly Ser Asn Lys Tyr Tyr Ala Asp Ser Val Lys Gly (SEQ ID NO: 50577)

Glu Pro Tyr Thr Ser Gly Trp Tyr Asp Tyr Gly Met Asp Val (SEQ ID NO: 50578)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_158D4_HC | | | | | | | | | | | | | | | | | | | | | | | | | |
| 21-225_159A3_HC | | | | | | | | | | | | | | | | | | | | | | | | | |
| 21-225_159C8_HC | | | | | | | | | | | | | | | | | | | | | | | | | |
| 21-225_160C4_HC | | | | | | | | | | | | | | | | | | | | | | | | | |
| 21-225_161G12_HC | | | | | | | | | | | | | | | | | | | | | | | | | |
| 21-225_20B11_HC | | | | | | | | | | | | | | | | | | | | | | | | | |
| 21-225_226F7_HC | | | | | | | | | | | | | | | | | | | | | | | | | |
| 21-225_2B10_LC1_HC | | | | | | | | | | | | | | | | | | | | | | | | | |
| 21-225_2B10_LC2_HC | | | | | | | | | | | | | | | | | | | | | | | | | |
| 21-225_7F8_HC | | | | | | | | | | | | | | | | | | | | | | | | | |

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | H_CDR1 | | | | | | | | | | | | | | | H_FR2 |
| CONSENSUS | S | G | - | F | T | F | S | - | - | - | - | - | - | Y | V | M | H | W | V | R | Q | A | P | G | K |
| 21-225_158D4_HC | | | | | | | | D | | | | | | | | | | | | | | | | | |
| 21-225_159A3_HC | | | | | | | | D | | | | | | | | | Q | | | | | | | | |
| 21-225_159C8_HC | | | | | | | | D | | | | | | | | | Q | | | | | | | | |
| 21-225_160C4_HC | | | | | | | | D | | | | | | | | | Q | | | | | | | | |
| 21-225_161G12_HC | | | | | | | | D | | | | | | | | | Q | | | | | | | | |
| 21-225_20B11_HC | | | | | | | | S | | | | | | | | | | | | | | | | | |
| 21-225_226F7_HC | | | | | | | | S | | | | | | G | | | | | | | | | | | |
| 21-225_2B10_LC1_HC | | | | | | | | S | | | | | | | | | | | | | | | | | |
| 21-225_2B10_LC2_HC | | | | | | | | S | | | | | | | | | | | | | | | | | |
| 21-225_7F8_HC | | | | | | | | S | | | | | C | | | | | | | | | | | | |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | V | A | V | I | W | Y | D | - | - | - | G | S | N | K | Y | Y | A | D | S | V | K |
| 21-225_158D4_HC | . | . | . | . | . | S | . | . | . | . | . | . | . | . | . | . | . | . | . | . | . | . | . | . | . |
| 21-225_159A3_HC | . | . | . | . | . | . | . | . | . | . | . | . | . | . | . | . | . | . | . | . | . | . | . | . | . |
| 21-225_159C8_HC | . | . | . | . | . | . | . | . | . | . | . | . | . | . | . | . | . | . | . | . | . | . | . | . | . |
| 21-225_160C4_HC | . | . | . | . | . | . | . | . | . | . | . | . | . | . | . | . | . | . | . | . | . | . | . | . | . |
| 21-225_161G12_HC | . | . | . | . | . | . | . | . | . | . | . | . | . | . | . | . | . | . | . | . | . | . | . | . | . |
| 21-225_20B11_HC | . | . | . | . | . | . | . | . | . | . | . | . | . | . | . | . | . | . | . | . | . | . | . | . | . |
| 21-225_226F7_HC | . | . | . | . | . | . | . | . | . | . | . | . | . | . | . | . | . | . | . | . | . | . | . | . | . |
| 21-225_2B10_LC1_HC | . | . | . | . | . | . | . | . | . | . | . | . | . | . | . | . | . | . | . | . | . | . | . | . | . |
| 21-225_2B10_LC2_HC | . | . | . | . | . | . | . | . | . | . | . | . | . | . | . | . | . | . | . | . | . | . | . | . | . |
| 21-225_7F8_HC | . | . | . | . | . | . | . | . | . | . | . | . | . | . | . | . | . | . | . | . | . | . | . | . | . |

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | F | T | I | S | R | D | N | S | K | N | T | L | Y | L | Q | M | N | S | L | R | A | E | D |
| 21-225_158D4_HC | . | . | . | . | . | . | . | . | . | . | . | . | . | . | . | . | . | . | . | . | . | . | . | . | . |
| 21-225_159A3_HC | . | . | . | . | . | . | . | . | . | . | . | . | . | . | . | . | . | . | . | . | . | . | . | . | . |
| 21-225_159C8_HC | . | . | . | . | . | . | . | . | . | . | . | . | . | . | . | . | . | . | . | . | . | . | . | . | . |
| 21-225_160C4_HC | . | . | . | . | . | . | . | . | . | . | . | . | . | . | . | . | . | . | . | . | . | . | . | . | . |
| 21-225_161G12_HC | . | . | . | . | . | . | . | . | . | . | . | . | . | . | . | . | . | . | . | . | . | . | . | . | . |
| 21-225_20B11_HC | V | . | . | . | V | . | . | . | . | . | . | . | . | . | S | . | . | . | . | . | . | . | . | . | . |
| 21-225_226F7_HC | . | . | . | . | . | . | . | . | . | . | . | . | . | . | . | . | . | . | . | . | . | . | . | . | . |
| 21-225_2B10_LC1_HC | V | . | . | . | V | . | . | . | . | . | . | . | . | . | S | . | . | . | . | . | . | . | . | . | . |
| 21-225_2B10_LC2_HC | V | . | . | . | V | . | . | . | . | . | . | . | . | . | S | . | . | . | . | . | . | . | . | . | . |
| 21-225_7F8_HC | . | . | . | . | . | . | . | . | . | . | N | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 | Refe |
|---|---|---|---|---|---|---|---|---|---|---|---|---|---|---|---|---|---|---|---|---|---|---|---|---|---|
| | | | | | | | | | | | | | | | | | | | H_FR4 | | | | | | |
| CONSENSUS | - | - | - | - | - | - | Y | D | Y | G | M | D | V | W | G | Q | G | T | T | V | T | V | S | S | |
| 21-225_158D4_HC | | | | | | | | | | | | | | | | | | | | | | | | | |
| 21-225_159A3_HC | | | | | | | | | | | | | | | | | | | | | | | | | |
| 21-225_159C8_HC | | | | | | | | | | | | | | | | | | | | | | | | | |
| 21-225_160C4_HC | | | | | | | | | | | | | | | | | | | | | | | | | |
| 21-225_161G12_HC | | | | | | | | | | | | | | | | | | | | | | | | | |
| 21-225_20B11_HC | | | | | | | | | | | | | | | | | | | | | | | | | |
| 21-225_226F7_HC | | | | | | | | | | | | | | | | | | | | | | | | | |
| 21-225_2B10_LC1_HC | | | | | | | | | | | | | | | | | | | | | | | | | |
| 21-225_2B10_LC2_HC | | | | | | | | | | | | | | | | | | | | | | | | | |
| 21-225_7F8_HC | | | | | | | | | | | | | | | | | | | | | | | | | |

| Reference # | rence # |
|---|---|
| CONSENSUS | |
| 21-225_158D4_HC | |
| 21-225_159A3_HC | |
| 21-225_159C8_HC | |
| 21-225_160C4_HC | |
| 21-225_161G12_HC | |
| 21-225_20B11_HC | |
| 21-225_226F7_HC | |
| 21-225_2B10_LC1_HC | |
| 21-225_2B10_LC2_HC | |
| 21-225_7F8_HC | |

**FIGURE 57**

Table 86.     Consensus 52 -VH1|1-18/D3|3-3|RF2/JH6 (SEQ ID NO: 50303):

QVQLVQS-GAEVKKPGASVKVSCKASG-YTFNS-----YGISWVRQAPGQGLEWMGWISAY---
NGNTKYAQKLQGRVTMTTDTSTSTAYMELRSLRSDDTAVYYCARHDFWSGY----------------YKGMDVWGQGTTVTVSS wherein:

I in position 41 can be substituted with F or V

T in position 68 can be substituted with R

Y in position 70 can be substituted with N, E or F

K in position 73 can be substituted with R

L in position 74 can be substituted with F

Ser Tyr Gly Xaa Ser (SEQ ID NO: 50102)

Trp Ile Ser Ala Tyr Asn Gly Asn Xaa Lys Xaa Ala Gln Xaa Xaa Gln Gly (SEQ ID NO: 50103)

His Asp Phe Trp Ser Gly Tyr Tyr Lys Gly Met Asp Val (SEQ ID NO: 50227)

Ser Tyr Gly Ile Ser (SEQ ID NO: 50579)

Trp Ile Ser Ala Tyr Asn Gly Asn Thr Lys Tyr Ala Gln Lys Leu Gln Gly (SEQ ID NO: 50580)

His Asp Phe Trp Ser Gly Tyr Tyr Lys Gly Met Asp Val (SEQ ID NO: 50581)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | Q | S | - | G | A | E | V | K | K | P | G | A | S | V | K | V | S | C | K | A | S |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . | . | . | . | . | . | . | . |
| 21-225_52F1_HC | . | . | . | . | . | . | . | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . | . | . | . | . | . | . | . |
| 21-225_56E3_HC | . | . | . | . | . | . | . | . | . | . | . | . | . | . | . | . | . | . | . | . | . | . | . | . | . | . |
| 21-225_56G1_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | Y | T | F | N | S | - | - | - | - | - | Y | G | I | S | W | V | R | Q | A | P | G | Q | G | L |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . | L | . | . | . | . | . | S |
| 21-225_51E3_HC | . | . | . | . | . | . | . | . | . | . | . | . | . | . | . | . | . | . | . | L | . | . | . | . | . | . |
| 21-225_51F4_HC | . | . | . | . | . | . | . | . | . | . | . | . | . | . | . | . | . | . | . | . | . | . | . | . | . | . |
| 21-225_52F1_HC | . | . | . | . | . | . | . | . | . | . | . | . | . | . | V | . | . | . | . | . | . | . | . | . | . | . |
| 21-225_53E8_HC | . | . | . | . | . | . | . | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . | L | . | . | . | . | . | . |
| 21-225_56E3_HC | . | . | . | . | . | . | . | . | . | . | . | . | . | . | F | . | . | . | . | . | . | . | . | . | . | . |
| 21-225_56G1_HC | . | . | . | . | . | . | . | . | . | . | . | . | . | . | . | . | . | . | . | . | . | . | . | . | . | . |

## FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | | |
| CONSENSUS | E | W | M | G | W | I | S | A | Y | - | - | - | N | G | N | T | K | Y | A | Q | K | L | Q | G | R | V |
| 21-225_49C5_HC | | | | | | | | | | | | | | | | | | | | | | | | | | |
| 21-225_49D10_HC | | | | | | | | | | | | | | | | | | N | | | | | | | | |
| 21-225_51E3_HC | | | | | | | | | | | | | | | | | | N | | | | | | | | |
| 21-225_51F4_HC | | | | | | | | | | | | | | | | | | | | | | | | | | |
| 21-225_52F1_HC | | | | | | | | | | | | | | | | | | | | | | | | | | |
| 21-225_53E8_HC | | | | | | | | | | | | | | | | | | | | | | | | | | |
| 21-225_54E9_HC | | | | | | | | | | | | | | | | | | N | | | | | | | | |
| 21-225_56E3_HC | | | | | | | | | | | | | | | | | | | | | | | | | | |
| 21-225_56G1_HC | | | | | | | | | | | | | | | | | | | | | | | | | | |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | M | T | T | D | T | S | T | S | T | A | Y | M | E | L | R | S | L | R | S | D | D | T | A | V | Y |
| 21-225_49C5_HC | | | | | | | | | | | | | | | | | | | | | | | | | | |
| 21-225_49D10_HC | | | | | | | | | | | | | | | | | | | | | | | | | | |
| 21-225_51E3_HC | | | | | | | | | | | | | | | | | | | | | | | | | | |
| 21-225_51F4_HC | | | | | | | | | | | | | | | | | | | | | | | | | | |
| 21-225_52F1_HC | S | | | | | | | | | | | | | | | | | | | | | | | | | |
| 21-225_53E8_HC | | | | | | | | | | | | | | | | | | | | | | | | | | |
| 21-225_54E9_HC | | | | | | | | | | | | | | | | | | | | | | | | | | |
| 21-225_56E3_HC | | | | | | | | | | | | | | | | | | | | A | | | | | | |
| 21-225_56G1_HC | | | | | | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | H | D | F | W | S | G | Y | - | - | - | - | - | - | - | - | - | - | - | - | - | - | - |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . | . | . | . | . | . | . | . |
| 21-225_52F1_HC | . | . | . | . | . | . | . | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . | . | . | . | . | . | . | . |
| 21-225_56E3_HC | . | . | . | . | . | . | . | . | . | . | . | . | . | . | . | . | . | . | . | . | . | . | . | . | . | . |
| 21-225_56G1_HC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | Y | K | G | M | D | V | W | G | Q | G | T | T | V | T | V | S | S |
| 21-225_49C5_HC | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . |
| 21-225_52F1_HC | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . |
| 21-225_56E3_HC | . | . | . | . | . | . | . | . | . | . | . | . | . | . | . | . | . | . | . |
| 21-225_56G1_HC | . | . | . | . | . | . | . | . | . | . | . | . | . | . | . | . | . | . | . |

**FIGURE 57**

Table 87. Consensus 53 - VH3|3-23/D6|6-6|RF1/JH4 (SEQ ID NO: 50304):

EVQLLES-GGGLVQPGGSLRLSCAASG-FTFS<u>S</u>-----<u>YAMS</u>WVRQAPGKGLEWVS<u>AISGR</u>---<u>GG</u>
<u>NTFDADSVKG</u>RFTISRDNSKNTLYLQMNSLRAEDTAVYYCAK<u>ERSGS</u>-------------------<u>YFDY</u>WGQGTLVTVSS wherein:

Y at position 39 can be substituted with S

A at position 57 can be substituted with V or S

R at position 61 can be substituted with S

G at position 66 can be substituted with I or V

N at position 67 can be substituted with S

D at position 70 can be substituted with Y

A at position 71 can be substituted with T

E at position 109 can be substituted with S

R at position 110 can be substituted with N

G at position 112 can be substituted with S

S at position 113 can be substituted with G

Y at position 135 can be substituted with W

Xaa Xaa Ala Met Ser (SEQ ID NO: 50104)

Xaa Ile Ser Gly Xaa Gly Xaa Xaa Thr Phe Xaa Xaa Asp Ser Val Lys Gly (SEQ ID NO: 50105)

Xaa Xaa Ser Xaa Xaa Xaa Phe Asp Tyr (SEQ ID NO: 50106)

Ser Tyr Ala Met Ser (SEQ ID NO: 50582)

Ala Ile Ser Gly Arg Gly Gly Asn Thr Phe Asp Ala Asp Ser Val Lys Gly (SEQ ID NO: 50583)

**FIGURE 57**

Glu Arg Ser Gly Ser Tyr Phe Asp Tyr (SEQ ID NO: 50584)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_171A8_HC | . | . | . | . | . | . | . | . | . | . | . | . | . | . | . | . | . | . | . | . | . | . | . | . | . | . |
| 21-225_174G7_HC | . | . | . | . | . | . | . | . | . | . | . | . | . | . | . | . | . | . | . | . | . | . | . | . | . | . |
| 21-225_175C10_HC | . | . | . | . | . | . | . | . | . | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | A | M | S | W | V | R | Q | A | P | G | K | G | L |
| 21-225_171A8_HC | . | . | . | . | ? | . | . | . | . | . | . | . | ? | . | . | . | . | . | . | . | . | . | . | . | . | . |
| 21-225_174G7_HC | . | . | . | . | ? | . | . | . | . | . | . | . | ? | . | . | . | . | . | . | . | . | . | . | . | . | . |
| 21-225_175C10_HC | . | . | . | . | . | . | . | . | . | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | H_CDR2 | | | | | | | | | | | | | |
| CONSENSUS | E | W | V | S | A | I | S | G | R | - | - | - | G | | N | T | F | D | A | D | S | V | K | G | R | F |
| 21-225_171A8_HC | . | . | . | . | V | . | . | . | S | . | . | . | . | S | S | . | . | Y | T | . | . | . | . | . | . | . |
| 21-225_174G7_HC | . | . | . | . | V | . | . | . | S | . | . | . | . | S | S | . | . | Y | T | . | . | . | . | . | . | . |
| 21-225_175C10_HC | . | . | . | . | S | . | . | . | . | . | . | . | . | S | S | . | . | Y | . | . | . | . | . | . | . | . |
| 21-225_43C2_HC | . | . | . | . | . | . | . | . | . | . | . | . | . | V | . | . | . | . | . | . | . | . | . | . | . | . |
| 21-225_43E8_HC | . | . | . | . | . | . | . | . | . | . | . | . | . | I | . | . | . | . | . | . | . | . | . | . | . | . |
| 21-225_43H4_HC | . | . | . | . | . | . | . | . | . | . | . | . | . | I | . | . | . | . | . | . | . | . | . | . | . | . |
| 21-225_44D10_HC | . | . | . | . | . | . | . | . | . | . | . | . | . | V | . | . | . | . | . | . | . | . | . | . | . | . |
| 21-225_45F8_HC | . | . | . | . | . | . | . | . | . | . | . | . | . | V | . | . | . | . | . | . | . | . | . | . | . | . |
| 21-225_46A6_HC | . | . | . | . | . | . | . | . | . | . | . | . | . | I | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_171A8_HC | . | . | . | . | . | . | . | . | . | . | . | . | . | . | . | . | . | . | . | . | . | . | . | . | . | . |
| 21-225_174G7_HC | . | . | . | . | . | . | . | . | . | . | . | . | . | . | . | . | . | . | . | . | . | . | . | . | . | . |
| 21-225_175C10_HC | . | V | . | . | . | . | . | . | . | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
|  |  |  |  |  |  |  |  |  |  |  |  |  |  |  |  |  |  |  | H_CDR3 |  |  |  |  |  |  |  |
| CONSENSUS | Y | C | A | K | E | R | S | G | S | - | - | - | - | - | - | - | - | - | - | - | - | - | - | - | - | - |
| 21-225_171A8_HC |  |  |  |  | S | N | . | S | G |  |  |  |  |  |  |  |  |  |  |  |  |  |  |  |  |  |
| 21-225_174G7_HC |  |  |  |  | S | N | . | S | G |  |  |  |  |  |  |  |  |  |  |  |  |  |  |  |  |  |
| 21-225_175C10_HC |  |  |  |  | S | N |  | S | G |  |  |  |  |  |  |  |  |  |  |  |  |  |  |  |  |  |
| 21-225_43C2_HC |  |  |  |  |  |  |  |  |  |  |  |  |  |  |  |  |  |  |  |  |  |  |  |  |  |  |
| 21-225_43E8_HC |  |  |  |  |  |  |  |  |  |  |  |  |  |  |  |  |  |  |  |  |  |  |  |  |  |  |
| 21-225_43H4_HC |  |  |  |  |  |  |  |  |  |  |  |  |  |  |  |  |  |  |  |  |  |  |  |  |  |  |
| 21-225_44D10_HC |  |  |  |  |  |  |  |  |  |  |  |  |  |  |  |  |  |  |  |  |  |  |  |  |  |  |
| 21-225_45F8_HC |  |  |  |  |  |  |  |  |  |  |  |  |  |  |  |  |  |  |  |  |  |  |  |  |  |  |
| 21-225_46A6_HC |  |  |  |  |  |  |  |  |  |  |  |  |  |  |  |  |  |  |  |  |  |  |  |  |  |  |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  |  |  |  |  | H_FR4 |  |  |  |  |  |  |
| CONSENSUS | - | - | - | - | Y | F | D | Y | W | G | Q | G | T | L | V | T | V | S | S |
| 21-225_171A8_HC |  |  |  |  | W | . |  |  |  |  |  |  |  |  |  |  |  |  |  |
| 21-225_174G7_HC |  |  |  |  | W | . |  |  |  |  |  |  |  |  |  |  |  |  |  |  |
| 21-225_175C10_HC |  |  |  |  | W | . |  |  |  |  |  |  |  |  |  |  |  |  |  |  |
| 21-225_43C2_HC |  |  |  |  |  |  |  |  |  |  |  |  |  |  |  |  |  |  |  |
| 21-225_43E8_HC |  |  |  |  |  |  |  |  |  |  |  |  |  |  |  |  |  |  |  |  |
| 21-225_43H4_HC |  |  |  |  |  |  |  |  |  |  |  |  |  |  |  |  |  |  |  |  |
| 21-225_44D10_HC |  |  |  |  |  |  |  |  |  |  |  |  |  |  |  |  |  |  |  |  |
| 21-225_45F8_HC |  |  |  |  |  |  |  |  |  |  |  |  |  |  |  |  |  |  |  |  |
| 21-225_46A6_HC |  |  |  |  |  |  |  |  |  |  |  |  |  |  |  |  |  |  |  |  |

FIGURE 57

Table 88.     Consensus 54 -VH3|3-33/D6|6-6|RF1/JH4 (SEQ ID NO: 50305):

QVQLVES-GGGVVQPGRSLRLSCAASG-FTFS<u>N-----YGMH</u>WVRQAPGKGLEWVA<u>VIWHD---</u>
<u>GSNKYYADSVKG</u>RFTISRDNSKNTLYLQMNSLRAEDTAVYYCAR<u>ENSSS-------------------YYFDY</u>WGQGTLVTVSS wherein:

N in position 33 can be substituted with S, Y or H

G in position 40 can be substituted with V

V in position 57 can be substituted with L

H in position 60 can be substituted with Y

S in position 66 can be substituted with T

N in position 67 can be substituted with D

K in position 68 can be substituted with A

A in position 71 can be substituted with V or G

Y in position 134 can be substituted with F

Xaa Tyr Xaa Met His (SEQ ID NO: 50107)

Xaa Ile Trp Xaa Asp Gly Xaa Xaa Xaa Tyr Tyr Xaa Asp Ser Val Lys Gly (SEQ ID NO: 50108)

Glu Asn Ser Ser Ser Xaa Tyr Phe Asp Tyr Trp Gly Gln Gly Thr Leu Val Thr Val Ser Ser (SEQ ID NO: 50109)

Asn Tyr Gly Met His (SEQ ID NO: 50585)

Val Ile Trp His Asp Gly Ser Asn Lys Tyr Tyr Ala Asp Ser Val Lys Gly (SEQ ID NO: 50586)

Glu Asn Ser Ser Ser Tyr Tyr Phe Asp Tyr Trp Gly Gln Gly Thr Leu Val Thr Val Ser Ser (SEQ ID NO: 50587)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_30G1_HC | . | . | . | . | . | . | . | . | . | . | . | . | . | . | . | . | . | . | . | . | . | . | . | . | . | . |
| 21-225_30G4_HC | . | . | . | . | . | . | . | . | . | . | . | . | . | . | . | . | . | . | . | . | . | . | . | . | . | . |
| 21-225_32D6_HC | . | . | . | . | . | . | . | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | . | . | . | . | . | . | . | . | . | . | . | . | . | . | . | . | . | . | . |
| 21-225_34C2_HC | . | . | . | . | . | . | . | . | . | . | . | . | . | . | . | . | . | . | . | . | . | . | . | . | . | . |
| 21-225_34D3_HC | . | . | . | . | . | . | . | . | . | . | . | . | . | . | . | . | . | . | . | . | . | . | . | . | . | . |
| 21-225_34D8_HC | . | . | . | . | . | . | . | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | H_FR2 | | | | |
| CONSENSUS | G | - | F | T | F | S | N | - | - | - | - | - | Y | G | M | H | W | V | R | Q | A | P | G | K | G | L |
| 21-225_30G1_HC | . | . | . | . | . | . | Y | . | . | . | . | . | . | . | . | . | . | . | . | . | . | . | . | . | . | . |
| 21-225_30G4_HC | . | . | . | . | . | . | S | . | . | . | . | . | . | . | . | . | . | . | . | . | . | . | . | X | . | . |
| 21-225_32D6_HC | . | . | . | . | . | . | S | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | X | . | . | . | . | . | . | . | . | . | . | . | . | . | . | . | . | . | . | . |
| 21-225_34C2_HC | . | . | . | . | . | . | . | . | . | . | . | . | V | . | . | . | . | . | . | . | . | . | . | . | . | . |
| 21-225_34D3_HC | . | . | . | . | . | . | Y | . | . | . | . | . | . | . | . | . | . | . | . | . | . | . | . | . | . | . |
| 21-225_34D8_HC | . | . | . | . | . | . | . | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | V | A | V | I | W | H | D | - | - | - | G | S | N | K | Y | Y | A | D | S | V | K | G | R | F |
| 21-225_30G1_HC | . | . | . | . | . | . | . | . | . | . | . | . | . | . | . | . | . | . | V | . | . | . | . | . | . | . |
| 21-225_30G4_HC | . | . | . | . | . | . | . | Y | . | . | . | . | . | T | Q | . | . | . | V | . | . | . | . | . | . | . |
| 21-225_32D6_HC | . | . | T | . | . | . | . | . | . | . | . | . | . | . | . | A | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | Y | . | . | . | . | . | . | . | . | . | . | . | . | . | . | . | . | . | . |
| 21-225_34C2_HC | . | . | . | . | . | . | . | Y | . | . | . | . | . | . | . | . | . | . | . | . | . | . | . | . | . | . |
| 21-225_34D3_HC | . | . | . | . | . | . | . | . | . | . | . | . | . | . | . | . | . | . | V | . | . | . | . | . | . | . |
| 21-225_34D8_HC | . | . | . | . | . | . | . | Y | . | . | . | . | . | . | . | . | . | . | . | . | . | . | . | . | . | . |
| 21-225_36B8_HC | . | . | . | . | . | . | . | . | . | . | . | . | . | . | . | . | . | . | Q | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | | H_FR3 | | | | | | | | | | | |
| CONSENSUS | T | I | S | R | D | N | S | K | N | T | L | Y | L | Q | M | N | S | L | R | A | E | D | T | A | V | Y |
| 21-225_30G1_HC | . | . | . | . | . | . | . | . | . | . | . | . | . | . | . | . | . | . | . | . | . | . | . | . | . | . |
| 21-225_30G4_HC | . | . | . | . | . | . | . | . | . | . | . | . | . | . | . | . | . | . | . | . | . | . | S | . | . | . |
| 21-225_32D6_HC | . | . | . | . | . | . | . | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | . | . | . | . | . | . | . | . | . | . | . | . | . | . | . | . | . | . | . |
| 21-225_34C2_HC | . | . | . | . | . | . | . | . | . | . | . | . | . | . | . | . | . | . | . | . | . | . | . | . | . | . |
| 21-225_34D3_HC | . | . | . | . | . | . | . | . | . | . | . | . | . | . | . | . | . | . | . | . | . | . | . | . | . | . |
| 21-225_34D8_HC | . | . | . | . | . | . | . | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | N | S | S | S | - | - | - | - | - | - | - | - | - | - | - | - | - | - | - | - | - |
| 21-225_30G1_HC | | | | | | | | | | | | | | | | | | | | | | | | | | |
| 21-225_30G4_HC | | | | | | | | | | | | | | | | | | | | | | | | | | |
| 21-225_32D6_HC | | | | | | | | | | | | | | | | | | | | | | | | | | |
| 21-225_33B2_HC | | | | | | | | | | | | | | | | | | | | | | | | | | |
| 21-225_33C12_HC | | | | | | | | | | | | | | | | | | | | | | | | | | |
| 21-225_34C2_HC | | | | | | | | | | | | | | | | | | | | | | | | | | |
| 21-225_34D3_HC | | | | | | | | | | | | | | | | | | | | | | | | | | |
| 21-225_34D8_HC | | | | | | | | | | | | | | | | | | | | | | | | | | |
| 21-225_36B8_HC | | | | | | | | | | | | | | | | | | | | | | | | | | |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | Y | Y | F | D | Y | W | G | Q | G | T | L | V | T | V | S | S |
| 21-225_30G1_HC | | | | | | | | | | | | | | | | | | | |
| 21-225_30G4_HC | | | | | | | | | | | | | | | | | | | |
| 21-225_32D6_HC | | | | | | | | | | | | | | | | | | | |
| 21-225_33B2_HC | | | | | | | | | | | | | | | | | | | |
| 21-225_33C12_HC | | | | | | | | | | | | | | | | | | | |
| 21-225_34C2_HC | | | | | | | | | | | | | | | | | | | |
| 21-225_34D3_HC | | | | | | | | | | | | | | | | | | | |
| 21-225_34D8_HC | | | | | | | | | | | | | | | | | | | |
| 21-225_36B8_HC | | | | | | | | | | | | | | | | | | | |

FIGURE 57

Table 89.    Consensus 55 - VH2|2-05/D1|1-1|RF1/JH3 (SEQ ID NO: 50306):

QITLKES-GPTLVKPTQTLTLTCTFSG-FSLS<u>TS</u>---<u>GVGVG</u>WIRQPPGKALEWLA<u>LINW</u>----
<u>NDDKRYSPSLKS</u>RFTITRDTSKDQVVLTMTNMDPVDTATYYCAH<u>KATWV</u>-------------------AFDIWGQGTMVTVSS wherein:

Y in position 70 can be substituted with F

A in position 110 can be substituted with T

Thr Ser Gly Val Gly Val Gly (SEQ ID NO: 50110)

Leu Ile Asn Trp Asn Asp Asp Lys Arg Xaa Ser Pro Ser Leu Lys Ser (SEQ ID NO: 50111)

Lys Xaa Thr Trp Val Ala Phe Asp Ile (SEQ ID NO: 50112)

Thr Ser Gly Val Gly Val Gly (SEQ ID NO: 50588)

Leu Ile Asn Trp Asn Asp Asp Lys Arg Tyr Ser Pro Ser Leu Lys Ser (SEQ ID NO: 50589)

Lys Ala Thr Trp Val Ala Phe Asp Ile (SEQ ID NO: 50590)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | I | T | L | K | E | S | - | G | P | T | L | V | K | P | T | Q | T | L | T | L | T | C | T | F | S |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | S | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | S | L | S | T | S | - | - | - | G | V | G | V | G | W | I | R | Q | P | P | G | K | A | L |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | N | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | L | A | L | I | N | W | - | - | - | - | N | D | D | K | R | Y | S | P | S | L | K | S | R | F |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | H_FR3 | | | | | | | | | | | | | |
| CONSENSUS | T | I | T | R | D | T | S | K | D | Q | V | V | L | T | M | T | N | M | D | P | V | D | T | A | T | Y |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | H | K | A | T | W | V | - | - | - | - | - | - | - | - | - | - | - | - | - | - | - | - | - |
| 21-225_62A12_HC | . | . | . | . | . | Y | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | Y | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | A | F | D | I | W | G | Q | G | T | M | V | T | V | S | S |
| 21-225_62A12_HC | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . |

**FIGURE 57**

Table 90.       Consensus 56 - VH3|3-23/D4|4-11|RF3/JH4 (SEQ ID NO: 50307):

EVQLLES-GGGLVQPGGSLRLSCAASG-FTFSS-----YVMNWVRQAPGKGLEWVSAISGS---
GGRTYYADSVKGRFTISRDNSKNTLYLQMNSLRAEDTAVYYCARTAT-----------------------FDYWGQGTLVTVSS wherein:

V at position 40 can be substituted with A

M at position 41 can be substituted with I or L

N at position 42 can be substituted with S or R

A at position 57 can be substituted with D

I at position 58 can be substituted with M

G at position 66 can be substituted with D or V

R at position 67 can be substituted with S, F or T

A at position 71 can be substituted with V

A at position 110 can be substituted with G, S, T or Y

T at position 111 can be substituted with V, null (-), H, L or G

F at position 136 can be substituted with null (-) or K

D at position 137 can be substituted with null (-)

Y at position 138 can be substituted with L

Ser Tyr Xaa Xaa Xaa (SEQ ID NO: 50113)

Xaa Xaa Ser Gly Ser Gly Xaa Xaa Thr Tyr Tyr Xaa Asp Ser Val Lys Gly (SEQ ID NO: 50114)

Thr Xaa Xaa Xaa Xaa Xaa (SEQ ID NO: 50115)

Ser Tyr Val Met Asn (SEQ ID NO: 50591)

**FIGURE 57**

Ala Ile Ser Gly Ser Gly Gly Arg Thr Tyr Tyr Ala Asp Ser Val Lys Gly (SEQ ID NO: 50592)

Thr Ala Thr Phe Asp Tyr (SEQ ID NO: 50593)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_153A1_HC | . | . | . | . | . | . | . | . | . | . | . | . | . | . | . | . | . | . | . | . | . | . | . | . | . | . |
| 21-225_157H1_HC | . | . | . | . | . | . | . | . | . | . | . | . | . | . | . | . | . | . | . | . | . | . | . | . | . | . |
| 21-225_175C4_HC | . | . | . | . | . | . | . | . | . | . | . | . | . | . | . | . | . | . | . | . | . | . | . | . | . | . |
| 21-225_23C7_HC | . | . | . | . | . | . | . | . | . | . | . | . | . | . | . | . | . | . | . | . | . | . | . | . | . | . |
| 21-225_23E7_HC | . | . | . | . | . | . | . | . | . | . | . | . | . | . | . | . | . | . | . | . | . | . | . | T | . | . |
| 21-225_30A1_HC | . | . | . | . | . | . | . | . | . | . | . | . | . | . | . | . | . | . | . | . | . | . | . | . | . | . |
| 21-225_33A5_HC | . | . | . | . | . | . | . | . | . | . | . | . | . | . | . | . | . | . | . | . | . | . | . | . | . | . |
| 21-225_56F7_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | V | M | N | W | V | R | Q | A | P | G | K | G | L |
| 21-225_153A1_HC | . | . | . | S | . | . | . | . | . | . | . | . | . | . | . | S | . | . | . | . | G | . | . | . | . | . |
| 21-225_157H1_HC | . | . | . | . | . | . | . | . | . | . | . | . | . | A | . | N | . | I | . | . | . | . | . | . | . | . |
| 21-225_175C4_HC | A | . | . | . | . | . | . | . | . | . | . | . | . | . | . | S | . | . | . | . | . | . | . | . | . | . |
| 21-225_23C7_HC | . | . | . | . | . | . | . | . | . | . | . | . | . | . | . | S | . | . | . | . | . | . | . | . | . | . |
| 21-225_23E7_HC | . | . | . | . | . | . | . | . | . | . | . | . | . | A | . | . | . | . | . | . | . | . | . | . | . | . |
| 21-225_30A1_HC | . | . | . | . | . | . | . | . | . | . | . | . | . | . | . | . | . | . | . | . | . | . | . | . | . | . |
| 21-225_33A5_HC | . | . | . | . | . | . | . | . | . | . | . | . | . | . | I | . | . | . | . | . | . | . | . | . | . | . |
| 21-225_56F7_HC | . | . | . | . | . | . | . | . | . | . | . | . | . | . | L | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | A | I | S | G | S | - | - | - | G | G | | T | Y | Y | A | D | S | V | K | G | R | F |
| 21-225_153A1_HC | . | . | . | . | . | . | . | . | . | . | . | . | . | D | N | . | . | . | . | . | . | . | . | . | . | . |
| 21-225_157H1_HC | Q | . | . | . | D | . | . | . | . | . | . | . | . | . | T | . | . | . | . | . | . | . | . | . | . | . |
| 21-225_175C4_HC | . | . | . | . | . | . | . | . | . | . | . | . | . | D | N | . | . | . | . | . | . | . | . | . | . | . |
| 21-225_23C7_HC | . | . | . | . | . | M | . | . | . | . | . | . | . | . | S | . | . | . | V | . | . | . | . | . | . | . |
| 21-225_23E7_HC | . | . | . | . | . | . | . | . | . | . | . | . | . | . | F | . | . | . | . | . | . | . | . | . | . | . |
| 21-225_30A1_HC | . | . | . | . | . | . | . | . | . | . | . | . | . | . | S | . | . | . | . | . | . | . | . | . | . | . |
| 21-225_33A5_HC | . | . | . | . | . | . | . | . | . | . | . | . | . | V | S | . | . | . | . | . | . | . | . | . | . | . |
| 21-225_56F7_HC | . | . | . | . | . | M | . | . | . | . | . | . | . | . | N | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_153A1_HC | . | . | . | . | . | . | . | . | . | . | . | . | . | . | . | . | . | . | . | . | . | . | . | . | . | . |
| 21-225_157H1_HC | . | . | . | . | . | . | . | . | . | . | . | . | . | . | . | . | . | . | . | . | . | . | . | . | . | . |
| 21-225_175C4_HC | . | . | . | . | D | . | . | N | T | . | . | . | . | . | . | . | . | . | . | . | . | . | . | . | . | . |
| 21-225_23C7_HC | . | . | . | . | . | . | . | . | . | . | . | . | . | . | . | . | . | . | . | . | . | . | . | . | . | . |
| 21-225_23E7_HC | . | . | . | . | . | . | . | . | . | . | . | . | . | . | . | . | . | . | . | . | . | . | . | . | . | . |
| 21-225_30A1_HC | . | . | . | N | . | . | . | . | . | . | . | . | . | . | . | . | . | . | . | . | . | . | . | . | . | . |
| 21-225_33A5_HC | . | . | . | . | . | . | . | . | . | . | . | . | . | . | . | . | . | . | . | . | . | . | . | . | . | . |
| 21-225_56F7_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | T | | | - | - | - | - | - | - | - | - | - | - | - | - | - | - | - | - | - | - | - |
| 21-225_153A1_HC | . | . | . | . | . | A | T | . | . | . | . | . | . | . | . | . | . | . | . | . | . | . | . | . | . | . |
| 21-225_157H1_HC | . | . | . | K | . | Y | - | . | . | . | . | . | . | . | . | . | . | . | . | . | . | . | . | . | . | . |
| 21-225_175C4_HC | . | . | . | . | . | A | G | . | . | . | . | . | . | . | . | . | . | . | . | . | . | . | . | . | . | . |
| 21-225_23C7_HC | . | . | . | . | . | G | V | . | . | . | . | . | . | . | . | . | . | . | . | . | . | . | . | . | . | . |
| 21-225_23E7_HC | . | . | . | . | . | S | G | . | . | . | . | . | . | . | . | . | . | . | . | . | . | . | . | . | . | . |
| 21-225_30A1_HC | . | . | . | . | . | G | V | . | . | . | . | . | . | . | . | . | . | . | . | . | . | . | . | . | . | . |
| 21-225_33A5_HC | . | . | . | . | . | S | L | . | . | . | . | . | . | . | . | . | . | . | . | . | . | . | . | . | . | . |
| 21-225_56F7_HC | F | . | . | M | . | Y | D | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | - | F | D | Y | W | G | Q | G | T | L | V | T | V | S | S |
| 21-225_153A1_HC | . | . | . | . | . | X | . | . | . | . | . | . | . | . | . | . | . | . | . |
| 21-225_157H1_HC | . | . | . | . | . | - | - | L | . | . | . | . | . | . | . | . | . | . | . |
| 21-225_175C4_HC | . | . | . | . | . | . | . | . | . | . | . | . | . | . | . | . | . | . | . |
| 21-225_23C7_HC | . | . | . | . | . | . | . | . | . | . | . | . | . | . | . | I | . | . | . |
| 21-225_23E7_HC | . | . | . | . | . | . | . | . | . | . | . | . | . | . | . | . | . | . | . |
| 21-225_30A1_HC | . | . | . | . | . | . | . | . | . | . | . | . | . | . | . | . | . | . | . |
| 21-225_33A5_HC | . | . | . | . | . | . | . | . | . | . | . | . | . | . | . | . | . | . | . |
| 21-225_56F7_HC | . | . | . | . | . | . | . | . | . | . | . | . | . | . | . | . | . | . | . |

**FIGURE 57**

Table 91.        Consensus 57 -VH3|3-23/D7|7-27|RF1/JH3 (SEQ ID NO: 50308):

EVQLLES-GGGLVQPGGSLRLSCAASG-FTFS<u>S-----YAMS</u>WVRQAPGKGLEWVS<u>VISGR---</u>
<u>GGTTFYADSVKG</u>RFTISRDNSKNTLYLQMNSLRAEDTAVYYCA<u>KRTPSD</u>--------------------<u>VFDI</u>WGQGTMVTVSS wherein:

Y at position 39 can be substituted with F

S at position 42 can be substituted with N

V at position 57 can be substituted with A or I

I at position 58 can be substituted with L

R at position 61 can be substituted with S or G

G at position 66 can be substituted with S or K

T at position 67 can be substituted with N or S

F at position 69 can be substituted with Y

P at position 111 can be substituted with G

S at position 112 can be substituted with D or E

V at position 135 can be substituted with A

I at position 138 can be substituted with V

Ser Xaa Ala Met Xaa (SEQ ID NO: 50116)

Xaa Xaa Ser Gly Xaa Gly Xaa Xaa Thr Xaa Tyr Ala Asp Ser Val Lys Gly (SEQ ID NO: 50117)

Lys Arg Thr Xaa Xaa Asp Xaa Phe Asp Xaa (SEQ ID NO: 50118)

Ser Tyr Ala Met Ser (SEQ ID NO: 50594)

Val Ile Ser Gly Arg Gly Gly Thr Thr Phe Tyr Ala Asp Ser Val Lys Gly (SEQ ID NO: 50595)

**FIGURE 57**

Lys Arg Thr Pro Ser Asp Val Phe Asp Ile (SEQ ID NO: 50596)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_149B6_HC | . | . | . | . | . | . | . | | . | . | . | . | . | . | . | . | . | . | . | . | . | . | . | . | . | . |
| 21-225_180A3_HC | . | . | . | . | . | . | . | | . | . | E | . | . | . | . | . | . | . | . | . | . | . | . | . | . | . |
| 21-225_182H5_HC | . | . | . | . | . | . | . | | . | . | . | . | . | . | . | . | . | . | . | . | . | . | . | . | . | . |
| 21-225_186A11_HC | . | . | . | . | . | . | . | | . | . | . | . | . | . | . | . | . | . | . | . | . | . | . | . | . | . |
| 21-225_43H9_HC | . | . | . | . | . | . | . | | . | . | . | . | . | . | . | . | . | . | . | . | . | . | . | . | . | . |
| 21-225_44F3_HC | . | . | R | . | . | . | . | | . | . | . | . | . | . | . | . | . | . | . | . | . | . | . | . | . | . |
| 21-225_45C9_HC | . | . | . | . | . | . | . | | . | . | . | . | . | . | . | . | . | . | . | . | . | . | . | . | . | . |
| 21-225_45H4_HC | . | . | . | . | . | . | . | | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | H_CDR1 | | | | | | | | | | | | | | | H_FR2 | | | |
| CONSENSUS | G | - | F | T | F | S | S | - | - | - | - | - | Y | A | M | S | W | V | R | Q | A | P | G | K | G | L |
| 21-225_149B6_HC | . | | . | . | . | . | . | . | . | . | . | . | . | . | . | . | . | . | . | . | . | . | . | . | . | . |
| 21-225_180A3_HC | . | | . | . | . | . | . | . | . | . | . | . | F | . | . | N | . | . | . | . | . | . | . | . | . | . |
| 21-225_182H5_HC | . | | . | . | . | . | . | . | . | . | . | . | . | . | . | . | . | . | . | . | . | . | . | . | . | . |
| 21-225_186A11_HC | . | | . | . | . | . | . | . | . | . | . | . | . | . | . | . | . | . | . | . | . | . | . | . | . | . |
| 21-225_43H9_HC | . | | . | . | . | . | . | . | . | . | . | . | . | . | . | . | . | . | . | . | . | . | . | R | . | . |
| 21-225_44F3_HC | . | | . | . | . | . | . | . | . | . | . | . | . | . | . | . | . | . | . | . | . | . | . | . | . | . |
| 21-225_45C9_HC | . | | . | . | N | . | . | . | . | . | . | . | . | . | . | . | . | . | . | . | . | . | . | . | . | . |
| 21-225_45H4_HC | . | | . | . | . | . | . | . | . | . | . | . | . | . | . | . | . | . | . | . | . | . | . | R | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | V | I | S | G | R | - | - | - | G | G | T | T | F | Y | A | D | S | V | K | G | R | F |
| 21-225_149B6_HC | . | | | | . | . | . | . | S | . | . | . | | S | | | . | . | . | . | . | . | . | . | . | . |
| 21-225_180A3_HC | . | | | . | . | . | . | . | . | . | . | . | . | N | | | . | . | . | . | . | . | . | . | . | . |
| 21-225_182H5_HC | . | | . | . | . | . | . | . | . | . | . | . | . | . | | | . | . | . | . | . | . | . | . | . | . |
| 21-225_186A11_HC | . | . | . | . | . | . | . | . | . | . | . | . | . | . | | | . | . | . | . | . | . | . | . | . | . |
| 21-225_43H9_HC | . | | | A | . | . | . | S | . | . | . | | S | N | | | . | . | . | . | . | . | . | . | . | . |
| 21-225_44F3_HC | . | . | . | I | L | . | . | G | . | . | . | | K | . | | Y | . | . | . | . | . | . | . | . | . | . |
| 21-225_45C9_HC | . | . | . | . | . | . | . | . | . | . | . | . | . | . | | Y | . | . | . | . | . | . | . | . | . | . |
| 21-225_45H4_HC | . | . | . | A | . | . | . | S | . | . | . | | S | N | . | | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | H_FR3 | | | | | | | | | | | | | | | | |
| CONSENSUS | T | I | S | R | D | N | S | K | N | T | L | Y | L | Q | M | N | S | L | R | A | E | D | T | A | V | Y |
| 21-225_149B6_HC | . | . | . | . | . | . | . | . | . | . | . | F | . | . | . | . | . | . | . | . | . | . | . | . | . | . |
| 21-225_180A3_HC | . | . | . | . | . | . | . | . | . | . | . | F | . | . | . | . | . | . | . | . | . | . | . | . | . | . |
| 21-225_182H5_HC | . | . | . | . | . | . | . | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . | . | . | . | . | . | . | . |
| 21-225_43H9_HC | . | . | . | . | . | . | . | . | . | . | . | . | H | . | . | . | . | . | . | . | . | . | . | . | . | . |
| 21-225_44F3_HC | . | . | . | . | . | . | . | . | . | . | . | F | . | . | . | . | . | . | . | . | . | . | . | . | . | . |
| 21-225_45C9_HC | . | . | . | . | . | . | . | . | . | . | . | . | . | . | . | . | . | . | . | . | . | . | . | . | . | . |
| 21-225_45H4_HC | . | . | . | . | . | . | . | . | . | . | . | H | . | . | . | . | . | . | . | . | . | . | . | . | . | . |

## FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | K | R | T | P | S | D | - | - | - | - | - | - | - | - | - | - | - | - | - | - | - | - | - |
| 21-225_149B6_HC | . | | | | . | . | . | E | . | . | . | . | . | . | . | . | . | . | . | . | . | . | . | . | . | . |
| 21-225_180A3_HC | F | | | | . | . | G | D | . | . | . | . | . | . | . | . | . | . | . | . | . | . | . | . | . | . |
| 21-225_182H5_HC | . | | | | . | . | G | D | . | . | . | . | . | . | . | . | . | . | . | . | . | . | . | . | . | . |
| 21-225_186A11_HC | . | . | | | . | . | G | D | . | . | . | . | . | . | . | . | . | . | . | . | . | . | . | . | . | . |
| 21-225_43H9_HC | F | | | | . | . | . | . | . | . | . | . | . | . | . | . | . | . | . | . | . | . | . | . | . | . |
| 21-225_44F3_HC | . | | | | . | . | . | . | . | . | . | . | . | . | . | . | . | . | . | . | . | . | . | . | . | . |
| 21-225_45C9_HC | . | | | | . | . | . | . | . | . | . | . | . | . | . | . | . | . | . | . | . | . | . | . | . | . |
| 21-225_45H4_HC | F | | | | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | V | F | D | I | W | G | Q | G | T | M | V | T | V | S | S |
| 21-225_149B6_HC | . | . | . | . | . | . | . | . | . | . | . | . | . | . | . | . | . | . | . |
| 21-225_180A3_HC | . | . | . | . | . | . | . | V | . | . | . | . | . | . | . | . | . | . | . |
| 21-225_182H5_HC | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . |
| 21-225_43H9_HC | . | . | . | . | . | . | . | . | . | . | . | . | . | . | . | . | . | . | . |
| 21-225_44F3_HC | . | . | . | . | A | . | . | . | . | . | . | . | . | . | . | . | . | . | . |
| 21-225_45C9_HC | . | . | . | . | A | . | . | . | . | . | . | . | . | . | . | . | . | . | . |
| 21-225_45H4_HC | . | . | . | . | . | . | . | . | . | . | . | . | . | . | . | . | . | . | . |

**FIGURE 57**

Table 92.    Consensus 58 -VH3|3-33/D4|4-11|RF2/JH4 (SEQ ID NO: 50309):

QVQLVES-GGGVVQPGRSLRLSCAASG-FTFSS-----YGMHWVRQAPGKGLEWVAVIWYD---
GSNKYYADSVKGRFTISRDNSKNTLYLQMNSLRAEDTAVYYCARDRPRS-------------------SAF DYWGQGTLVTVSS wherein:

S at position 33 can be substituted with N or T

Y at position 39 can be substituted with F

G at position 40 can be substituted with D or N

V at position 57 can be substituted with A

Y at position 60 can be substituted with H

S at position 66 can be substituted with R

N at position 67 can be substituted with D or H

K at position 68 can be substituted with R

Y at position 70 can be substituted with C or S

A at position 71 can be substituted with E or T

R at position 110 can be substituted with D or H

P at position 111 can be substituted with S or A

I at position 112 can be substituted with R or Y

S at position 113 can be substituted with L, V or W

Null (-) at position 114 can be substituted with G

FIGURE 57

Null (-) at position 133 can be substituted with A

S at position 134 can be substituted with T or A

A at position 135 can be substituted with F, S or Y

F at position 136 can be substituted with G or S

Y at position 138 can be substituted with F

Xaa Xaa Xaa Met His (SEQ ID NO: 50237)

Xaa Ile Trp Xaa Asp Gly Xaa Xaa Xaa Tyr Xaa Xaa Asp Ser Val Lys Gly (SEQ ID NO: 50238)

Asp Xaa Xaa Xaa Xaa Xaa Xaa Xaa Xaa Xaa Asp Xaa (SEQ ID NO: 50239)

Ser Tyr Gly Met His (SEQ ID NO: 50597)

Val Ile Trp Tyr Asp Gly Ser Asn Lys Tyr Tyr Ala Asp Ser Val Lys Gly (SEQ ID NO: 50598)

Asp Arg Pro Arg Ser Ser Ala Phe Asp Tyr (SEQ ID NO: 50599)

EP 4 435 105 A2

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_180F8_HC | | | | | | | | | | | | | | | | | | | | | | | | | | |
| 21-225_19B3_HC | | | | | | | | | | | | | | | | | | | | | | | | | | |
| 21-225_212C10_HC | | | | | | | | | | | | | | | | | | | | | | | | | | |
| 21-225_63C10_HC | | | | | | | | | | | | | | | | | | | | | | | | | | |
| 21-225_70H6_HC | | | | | | | | | | | | | | | | | | | | | | | | | | |
| 21-225_74C3_HC | | | | | | | | | | | | | | | | | | | | | | | | | | |
| 21-225_77H5_HC | | | | | | | | | | | | | | | | | | | | | | | | | | |
| 21-225_8H11_HC | | | | | | | | | | | | | | | | | | | | | | | | | | |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | G | M | H | W | V | R | Q | A | P | G | K | G | L |
| 21-225_180F8_HC | | | | | | | | | | | | | | | | | | | | | | | | | | |
| 21-225_19B3_HC | | | | | | | | | | | | | D | | | | | | | | | | | | | |
| 21-225_212C10_HC | | | | | | | | | | | | | | | | | | | | | | | | | | |
| 21-225_63C10_HC | | | | | | | | | | | | | | | | | | | | | | | | | | |
| 21-225_70H6_HC | | | | | | | | | | | | | | | | | | | | | T | | | | | |
| 21-225_74C3_HC | | | | | | | | | | | | | F | | | | | A | | | | | | | | |
| 21-225_77H5_HC | | | | | | N | | | | | | | | | | | | | | | | | | | | |
| 21-225_8H11_HC | | | | | | Y | | | | | | | N | | | | | | | | | | | | | |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | G | S | N | K | Y | Y | A | D | S | V | K | G | R | F |
| 21-225_180F8_HC | . | . | . | S | . | . | . | . | . | . | . | . | . | . | D | . | . | . | E | . | . | . | . | . | . | . |
| 21-225_19B3_HC | . | . | . | . | . | . | . | . | . | . | . | . | . | . | D | . | . | . | . | . | . | . | . | . | . | . |
| 21-225_212C10_HC | . | . | . | T | A | . | . | . | . | . | . | . | . | . | N | . | . | . | T | . | . | . | . | . | . | . |
| 21-225_63C10_HC | D | . | . | . | . | . | . | . | . | . | . | . | . | . | . | . | . | . | . | . | . | . | . | . | . | . |
| 21-225_70H6_HC | D | . | . | . | . | . | . | N | . | . | . | . | . | . | . | . | . | . | . | . | . | . | . | . | . | . |
| 21-225_74C3_HC | . | . | . | . | A | . | . | . | . | . | . | . | . | . | . | . | . | . | C | . | . | . | . | . | . | . |
| 21-225_77H5_HC | . | . | . | . | . | . | . | . | . | . | . | . | . | A | . | . | . | . | . | . | . | . | . | . | . | . |
| 21-225_8H11_HC | . | . | . | . | . | . | . | . | . | . | . | . | . | . | D | N | . | . | S | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | H_FR3 | | | | | | | | | | | | | |
| CONSENSUS | T | I | S | R | D | N | S | K | N | T | L | Y | L | Q | M | N | S | L | R | A | E | D | T | A | V | Y |
| 21-225_180F8_HC | . | . | . | . | . | . | . | . | . | . | . | . | . | . | . | . | . | . | . | . | . | . | . | . | . | . |
| 21-225_19B3_HC | . | . | . | . | . | . | . | . | . | . | . | F | . | . | . | . | . | . | . | . | . | . | . | . | . | . |
| 21-225_212C10_HC | . | . | . | . | . | . | . | . | . | . | . | . | . | . | . | . | . | . | . | . | . | . | . | . | . | . |
| 21-225_63C10_HC | . | . | . | . | . | . | . | . | . | . | . | . | . | . | . | . | . | . | . | . | . | . | . | . | . | . |
| 21-225_70H6_HC | . | . | . | . | . | . | . | . | . | . | . | . | . | . | . | . | . | . | . | . | . | . | . | . | . | . |
| 21-225_74C3_HC | . | . | . | . | . | . | . | . | . | . | . | S | . | . | . | . | . | . | . | . | . | . | . | S | . | . |
| 21-225_77H5_HC | . | . | . | . | . | . | . | . | . | . | . | . | . | . | . | . | . | . | . | . | . | . | . | . | L | . |
| 21-225_8H11_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | | | S | - | - | - | - | - | - | - | - | - | - | - | - | - | - | - | - | - |
| 21-225_180F8_HC | | | | | | H | P | R | W | . | . | . | . | . | . | . | . | . | . | . | . | . | . | . | . | . |
| 21-225_19B3_HC | | | | | | . | A | Y | . | . | . | . | . | . | . | . | . | . | . | . | . | . | . | . | . | . |
| 21-225_212C10_HC | | | | | | . | S | I | V | Q | . | . | . | . | . | . | . | . | . | . | . | . | . | . | . | . |
| 21-225_63C10_HC | | | | | | D | P | R | . | . | . | . | . | . | . | . | . | . | . | . | . | . | . | . | . | . |
| 21-225_70H6_HC | | | | | | D | P | R | . | . | . | . | . | . | . | . | . | . | . | . | . | . | . | . | . | . |
| 21-225_74C3_HC | | | | | | . | S | I | L | Q | . | . | . | . | . | . | . | . | . | . | . | . | . | . | . | . |
| 21-225_77H5_HC | | | | | | . | S | I | L | Q | . | . | . | . | . | . | . | . | . | . | . | . | . | . | . | . |
| 21-225_8H11_HC | | | | | | . | A | Y | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | S | | | D | Y | W | G | Q | G | T | L | V | T | V | S | S |
| 21-225_180F8_HC | . | . | . | . | Y | Q | . | . | . | . | . | . | . | . | . | . | . | . | . |
| 21-225_19B3_HC | . | . | . | . | S | S | . | . | . | . | . | . | . | . | . | . | . | . | . |
| 21-225_212C10_HC | . | . | A | T | Y | F | . | . | . | . | . | . | . | . | . | . | . | . | . |
| 21-225_63C10_HC | . | . | . | . | A | S | . | . | . | . | . | . | . | . | . | . | . | . | . |
| 21-225_70H6_HC | . | . | . | . | A | S | . | . | . | . | . | . | . | . | . | . | . | . | . |
| 21-225_74C3_HC | . | . | A | A | F | F | . | . | . | . | . | . | . | . | . | . | . | . | . |
| 21-225_77H5_HC | . | . | A | T | F | F | . | . | . | . | . | . | . | . | . | . | . | . | . |
| 21-225_8H11_HC | . | . | . | . | S | S | . | F | . | . | . | . | . | . | . | . | . | . | . |

**FIGURE 57**

Table 93.    Consensus 59 - VH3|3-48/D4|4-11|RF2/JH6 (SEQ ID NO: 50310):

EVQLVES-GGGLVQPGGSLRLSCAASG-FTFS<u>S-----YNMN</u>WVRQAPGKGLEWVS<u>YISRS---</u>
<u>SNTKYYADS</u>VKGRFTISRDNAKNSLYLQMNSLRDEDTAVYYCAR<u>DRSGSYGY</u>-------------FYYYGL<u>D</u>VWGQGTTVTVSS wherein:

S at position 33 can be substituted with N

N at position 40 can be substituted with S

R at position 60 can be substituted with S

S at position 65 can be substituted with G

N at position 66 can be substituted with S

K at position 68 can be substituted with T

Y at position 69 can be substituted with H

A at position 71 can be substituted with V

K at position 75 can be substituted with R, E or Q

R at position 110 can be substituted with S

S at position 111 can be substituted with R

G at position 112 can be substituted with K

S at position 113 can be substituted with G

Y at position 114 can be substituted with F

G at position 115 can be substituted with null (-)

Y at position 116 can be substituted with null (-)

FIGURE 57

F at position 131 can be substituted with null (-)

Y at position 132 can be substituted with null (-)

L at position 136 can be substituted with M

Xaa Tyr Xaa Met Asn (SEQ ID NO: 50240)

Tyr Ile Ser Xaa Ser Xaa Xaa Thr Xaa Xaa Tyr Xaa Asp Ser Val Xaa Gly (SEQ ID NO: 50241)

Asp Xaa Xaa Xaa Xaa Xaa Xaa Xaa Xaa Xaa Tyr Tyr Gly Xaa Asp Val (SEQ ID NO: 50242)

Ser Tyr Asn Met Asn (SEQ ID NO: 50600)

Tyr Ile Ser Arg Ser Ser Asn Thr Lys Tyr Tyr Ala Asp Ser Val Lys Gly (SEQ ID NO: 50601)

Asp Arg Ser Gly Ser Tyr Gly Tyr Phe Tyr Tyr Tyr Gly Leu Asp Val (SEQ ID NO: 50602)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | V | E | S | - | G | G | G | L | V | Q | P | G | G | S | L | R | L | S | C | A | A | S |
| 21-225_146A8_HC | . | . | . | . | . | . | . | . | . | . | . | . | . | . | . | . | E | . | . | . | . | . | . | V | . | . |
| 21-225_146B6_HC | . | . | . | . | . | . | . | . | . | . | . | . | . | . | . | . | . | . | . | . | . | . | . | . | . | . |
| 21-225_146D8_HC | . | . | . | . | . | . | . | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . | . | . | . | . | . | . | . |
| 21-225_150F9_HC | . | M | . | . | . | . | . | . | . | . | . | . | . | . | . | . | . | . | . | . | . | . | . | . | . | . |
| 21-225_156H2_HC | . | . | . | . | . | . | . | . | . | . | . | . | . | . | . | . | . | . | . | . | . | . | . | . | . | . |
| 21-225_170E4_HC | . | . | . | . | . | . | . | . | . | . | . | S | . | . | . | . | . | . | . | . | . | . | . | . | . | . |
| 21-225_179D10_HC | . | . | . | . | . | . | . | . | . | . | . | . | . | . | . | . | . | . | . | . | S | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | H_CDR1 | | | | | | | | | | | | | | H_FR2 | | | | |
| CONSENSUS | G | - | F | T | F | S | S | - | - | - | - | - | Y | N | M | N | W | V | R | Q | A | P | G | K | G | L |
| 21-225_146A8_HC | . | . | . | . | . | . | N | . | . | . | . | . | . | . | . | . | . | . | . | . | . | . | . | . | . | . |
| 21-225_146B6_HC | . | . | . | . | . | . | . | . | . | . | . | . | . | . | . | . | . | . | . | . | . | . | . | . | . | . |
| 21-225_146D8_HC | . | . | . | . | . | . | N | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . | . | . | . | . | . | . | . |
| 21-225_150F9_HC | . | . | . | . | . | . | . | . | . | . | . | . | . | . | . | . | . | . | . | . | V | . | . | . | . | . |
| 21-225_156H2_HC | . | . | . | . | . | . | N | . | . | . | . | . | . | . | . | . | . | . | . | . | . | . | . | . | . | . |
| 21-225_170E4_HC | . | . | . | . | . | . | . | . | . | . | . | . | . | S | . | . | . | . | . | . | . | . | . | . | . | . |
| 21-225_179D10_HC | . | . | . | . | . | . | . | . | . | . | . | . | . | S | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | Y | I | S | R | S | - | - | - | S | N | T | K | Y | Y | A | D | S | V | K | G | R | F |
| 21-225_146A8_HC | | | | | | | | | | | | | | | | | | | | | | | | | | |
| 21-225_146B6_HC | | | | | | | | | | | | | | | | | | | | | | | | | | |
| 21-225_146D8_HC | | | | | | | | | | | | | | | | | | | V | | | | | | | |
| 21-225_149A1_HC | | | | | | | | | | | | | | | | | | | | | | | | | | |
| 21-225_150F9_HC | | | | | | | | | | | | | | | | | | | | | | | | | | |
| 21-225_156H2_HC | | | | | | | | | | | | | | | | | | | | | | | | | | |
| 21-225_170E4_HC | | | | | | | | S | | | | | G | S | | | | | | | | | | | | |
| 21-225_179D10_HC | | | | A | | | | S | | | | | G | S | | Y | | | | | | | Q | | | |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | A | K | N | S | L | Y | L | Q | M | N | S | L | R | D | E | D | T | A | V | Y |
| 21-225_146A8_HC | | | | | | | | | | | | | | | | | | | | | | | | | | |
| 21-225_146B6_HC | | | | | | | | | | | | | | | | D | | | | | | | | | | |
| 21-225_146D8_HC | | | | | | | | | | | | | | | | | | | | | | | | | | |
| 21-225_149A1_HC | | | | | | | | | | | | | | | | | | | | | | | | | | |
| 21-225_150F9_HC | | | | | | | | | | | | | | | | | | | | | | | | | | |
| 21-225_156H2_HC | | | | | | | | | | | | | | | | | | | | | | | | | | |
| 21-225_170E4_HC | | | | | | | | | | | | D | | | | | | | | | | | | | | |
| 21-225_179D10_HC | | | | | | | | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | S | G | S | Y | G | Y | - | - | - | - | - | - | - | - | - | - | - | - | - | - |
| 21-225_146A8_HC | | | | | | | | | | | | | | | | | | | | | | | | | | |
| 21-225_146B6_HC | | | | | | | | | | | | | | | | | | | | | | | | | | |
| 21-225_146D8_HC | | | | | | | | | | | | | | | | | | | | | | | | | | |
| 21-225_149A1_HC | | | | | | | | | | | | | | | | | | | | | | | | | | |
| 21-225_150F9_HC | | | | | | | | | | | | | | | | | | | | | | | | | | |
| 21-225_156H2_HC | | | | | | | | | | | | | | | | | | | | | | | | | | |
| 21-225_170E4_HC | | | | | | S | R | K | Q | Y | - | - | | | | | | | | | | | | | | |
| 21-225_179D10_HC | | | | | | S | R | K | Q | Y | - | - | | | | | | | | | | | | | | |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | F | Y | Y | Y | G | L | D | V | W | G | Q | G | T | T | V | T | V | S | S |
| 21-225_146A8_HC | | | | | | | | | | | | | | | | | | | |
| 21-225_146B6_HC | | | | | | | | | | | | | | | | | | | |
| 21-225_146D8_HC | | | | | | | | | | | | | | | | | | | |
| 21-225_149A1_HC | | | | | | | | | | | | | | | | | | | |
| 21-225_150F9_HC | | | | | | | | | | | | | | | | | | | |
| 21-225_156H2_HC | | | | | | | | | | | | | | | | | | | |
| 21-225_170E4_HC | - | - | | | | | | | | | | | | | | | | | |
| 21-225_179D10_HC | - | - | | | | | | | | | | | | | | | | | |

4198

FIGURE 57

Table 94.     Consensus 60 - VH4|4-30.1/D4|4-11|RF2/JH6 (SEQ ID NO: 50311):

QVQLQES-GPGLVKPSQTLSLTCTVSG-GSIR<u>SG---GDYWS</u>WIRQHPGKGLEWIG<u>YIYY----</u>
<u>SGSTYYNPSLKSR</u>VTISVDTSKNQFSLKLSSVTAADTAVYYCAR<u>DSSSY</u>-------------------<u>GMDV</u>WGQGTTVTVSS wherein:

S at position 67 can be substituted with I or P

S at position 110 can be substituted with G or H

S at position 111 can be substituted with A

S at position 112 can be substituted with L or R

Y at position 113 can be substituted with R or H

Ser Gly Gly Asp Tyr Trp Ser (SEQ ID NO: 50119)

Tyr Ile Tyr Tyr Ser Gly Xaa Thr Tyr Tyr Asn Pro Ser Leu Lys Ser (SEQ ID NO: 50120)

Asp Xaa Xaa Xaa Xaa Gly Met Asp Val (SEQ ID NO: 50121)

Ser Gly Gly Asp Tyr Trp Ser (SEQ ID NO: 50603)

Tyr Ile Tyr Tyr Ser Gly Ser Thr Tyr Tyr Asn Pro Ser Leu Lys Ser (SEQ ID NO: 50604)

Asp Ser Ser Ser Tyr Gly Met Asp Val (SEQ ID NO: 50605)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | |
| CONSENSUS | Q | V | Q | L | Q | E | S | - | G | P | G | L | V | K | P | S | Q | T | L | S | L | T | C | T | V | S |
| 21-225_190F10_HC | . | . | . | . | . | . | | | | | | | . | . | . | . | . | . | . | . | . | . | . | . | . | . |
| 21-225_191A2_HC | . | | . | | | | | | | | | . | | | | | | | | | | | | | | |
| 21-225_192H8_HC | . | | | | | | | | | | | V | | . | | | | | | | | | | | | |
| 21-225_194D12_HC | . | | . | | | | | | | | | | | | | | | | | | | . | | | | |
| 21-225_194G5_HC | . | | . | | | | | | | | | | | | | | | | | | | ? | | | | |
| 21-225_198B8_HC | . | | | | | | | | | | | | | | | | | | | | | | | | | |
| 21-225_212H12_HC | . | | | | | | | | | | | | | | | | | | | | | | | | | |
| 21-225_214H3_HC | . | | | | | | | | | | | | | | | | | | | | | | | . | | |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | H_CDR1 | | | | | | | | | | | | | | | | | H_FR2 | | | |
| CONSENSUS | G | - | G | S | I | R | S | G | - | - | - | G | D | Y | W | S | W | I | R | Q | H | P | G | K | G | L |
| 21-225_190F10_HC | | | | . | | . | . | . | | | | . | . | | | . | | | | | | | . | S | . | . |
| 21-225_191A2_HC | | | | | | . | . | . | | | | . | | | | . | | | | | | | | S | | |
| 21-225_192H8_HC | | | | | S | | . | . | | | | . | | | | | | | | . | | . | | | . | |
| 21-225_194D12_HC | | | | | S | | . | . | | | | . | | | | . | | | | | | | | | | . |
| 21-225_194G5_HC | | | | | | | . | | | | | | | | | | | | | | | | | | | |
| 21-225_198B8_HC | | | | | | | . | . | | | | | | | | | | | | | | | | S | | |
| 21-225_212H12_HC | | | | | | | . | | | | | | | | | | | | | | | | | | | |
| 21-225_214H3_HC | | | | | | | | | | | | | | | | | | | | | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | Y | I | Y | Y | - | - | - | - | S | G | S | T | Y | Y | N | P | S | L | K | S | R | V |
| 21-225_190F10_HC | . | . | . | . | . | . | . | . | . | . | . | . | . | . | . | . | . | . | . | . | . | . | . | . | . | . |
| 21-225_191A2_HC | . | . | . | . | . | . | . | . | . | . | . | . | . | . | . | . | . | . | . | . | . | . | . | . | . | . |
| 21-225_192H8_HC | . | . | . | . | . | . | . | . | . | . | . | . | . | . | P | . | . | . | . | . | . | . | . | . | . | L |
| 21-225_194D12_HC | . | . | . | . | . | . | . | . | . | . | . | . | . | . | . | . | . | . | . | . | . | . | . | . | . | . |
| 21-225_194G5_HC | . | . | . | . | . | . | . | . | . | . | . | . | . | . | . | . | . | . | . | . | . | . | . | . | . | . |
| 21-225_198B8_HC | . | . | . | . | . | . | . | . | . | . | . | . | . | . | . | . | . | . | . | . | . | . | . | . | . | . |
| 21-225_212H12_HC | . | . | . | . | . | . | . | . | . | . | . | . | . | . | . | . | . | . | . | . | . | . | . | . | . | . |
| 21-225_214H3_HC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | H_FR3 | | | | | | | | | | | | | | |
| CONSENSUS | T | I | S | V | D | T | S | K | N | Q | F | S | L | K | L | S | S | V | T | A | A | D | T | A | V | Y |
| 21-225_190F10_HC | . | . | . | . | . | . | . | . | . | . | . | . | . | . | . | . | . | . | . | . | . | . | . | . | . | . |
| 21-225_191A2_HC | . | . | . | . | . | . | . | . | . | . | . | . | . | . | . | . | . | . | . | . | . | . | . | . | . | . |
| 21-225_192H8_HC | . | M | . | A | . | . | . | . | . | . | . | . | . | . | . | . | . | . | . | . | . | . | . | . | . | . |
| 21-225_194D12_HC | . | . | . | . | . | . | . | . | . | . | . | . | . | . | . | . | . | . | . | . | . | . | . | . | . | . |
| 21-225_194G5_HC | . | . | . | . | . | . | . | . | . | . | . | . | . | . | . | . | . | . | . | . | . | . | . | . | . | . |
| 21-225_198B8_HC | . | . | . | . | . | . | . | . | . | . | . | . | . | . | . | . | . | . | . | . | . | . | . | . | . | . |
| 21-225_212H12_HC | . | . | . | G | . | . | . | . | . | . | . | . | . | . | . | . | . | . | . | . | . | . | . | . | . | . |
| 21-225_214H3_HC | . | . | . | G | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | S | S | S | Y | - | - | - | - | - | - | - | - | - | - | - | - | - | - | - | - | - |
| 21-225_190F10_HC | . | . | . | . | . | . | . | . | ※ | . | . | . | . | . | . | . | . | . | . | . | . | . | . | . | . | . |
| 21-225_191A2_HC | . | . | . | . | . | . | . | . | ※ | . | . | . | . | . | . | . | . | . | . | . | . | . | . | . | . | . |
| 21-225_192H8_HC | . | . | . | . | . | . | . | L | . | . | . | . | . | . | . | . | . | . | . | . | . | . | . | . | . | . |
| 21-225_194D12_HC | . | . | . | . | . | G | A | ※ | ※ | . | . | . | . | . | . | . | . | . | . | . | . | . | . | . | . | . |
| 21-225_194G5_HC | . | . | . | . | . | ※ | . | L | . | . | . | . | . | . | . | . | . | . | . | . | . | . | . | . | . | . |
| 21-225_198B8_HC | . | . | . | . | . | . | . | . | ※ | . | . | . | . | . | . | . | . | . | . | . | . | . | . | . | . | . |
| 21-225_212H12_HC | . | . | . | . | . | . | . | . | . | . | . | . | . | . | . | . | . | . | . | . | . | . | . | . | . | . |
| 21-225_214H3_HC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | G | M | D | V | W | G | Q | G | T | T | V | T | V | S | S |
| 21-225_190F10_HC | . | . | . | . | . | . | . | . | . | . | . | . | . | . | . | . | . | . | . |
| 21-225_191A2_HC | . | . | . | . | . | . | . | . | . | . | . | . | . | . | . | . | . | . | . |
| 21-225_192H8_HC | . | . | . | . | . | . | . | . | . | . | . | . | . | . | . | . | . | . | . |
| 21-225_194D12_HC | . | . | . | . | . | . | . | . | . | . | . | . | . | . | . | . | . | . | . |
| 21-225_194G5_HC | . | . | . | . | . | . | . | . | . | . | . | . | . | . | . | . | . | . | . |
| 21-225_198B8_HC | . | . | . | . | . | . | . | . | . | . | . | . | . | . | . | . | . | . | . |
| 21-225_212H12_HC | . | . | . | . | . | . | . | . | . | . | . | . | . | . | . | . | . | . | . |
| 21-225_214H3_HC | . | . | . | . | . | . | . | . | . | . | . | . | . | . | . | . | . | . | . |

**FIGURE 57**

Table 95.      Consensus 15 -VK1|A30/JK4 (SEQ ID NO: 50312):

DIQMTQSPSSLSASVGDRVTITC<u>RAS</u>--<u>QGIR</u>------<u>NDLGWY</u>QQKPGKAPKRLIY<u>A</u>--------<u>ASSLQS</u>GVPSRFSGSGSG--
TEFTLTISSLQPEDFATYYC<u>LQHNS</u>---------------------<u>YPLT</u>FGGGTKVEIKR wherein:

R at position 24 can be substituted with L

A at position 25 can be substituted with T

Q at position 29 can be substituted with R

G at position 30 can be substituted with D, A or V

I at position 31 can be substituted with M or V

R at position 32 can be substituted with E, K, N or S

N at position 39 can be substituted with S, D, T, K or I

D at position 40 can be substituted with N or A

L at position 41 can be substituted with F or V

G at position 42 can be substituted with D or N

A at position 58 can be substituted with T, S, V, G, D or R

A at position 67 can be substituted with T, V or E

S at position 68 can be substituted with F, C or Y

S at position 69 can be substituted with N, T, F, R or I

L at position 70 can be substituted with V, F or S

Q at position 71 can be substituted with H or E

S at position 72 can be substituted with R, N, T or G

**4203**

FIGURE 57

L at position 107 can be substituted with V or I

Q at position 108 can be substituted with H

H at position 109 can be substituted with D, Y, N or R

N at position 110 can be substituted with S, Y, T, D, K, A, I, E, H, P or R

S at position 111 can be substituted with I, N, R, T, D, A, L or V

Y at position 135 can be substituted with F, H or S

P at position 136 can be substituted with A or M

L at position 137 can be substituted with P, F, N or V

T at position 138 can be substituted with K or I

Xaa Xaa Ser Xaa Xaa Xaa Xaa Xaa Xaa Xaa Xaa (SEQ ID NO: 50122)

Xaa Xaa Xaa Xaa Xaa Xaa Xaa (SEQ ID NO: 50123)

Xaa Xaa Xaa Xaa Xaa Xaa Xaa Xaa Xaa (SEQ ID NO: 50124)

Arg Ala Ser Gln Gly Ile Arg Asn Asp Leu Gly (SEQ ID NO: 50606)

Ala Ala Ser Ser Leu Gln Ser (SEQ ID NO: 50607)

Leu Gln His Asn Ser Tyr Pro Leu Thr (SEQ ID NO: 50608)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_10G5_LC | . | . | . | . | . | . | . | . | . | . | . | . | . | . | . | . | . | . | . | . | . | . | . | . | . | . |
| 21-225_10H12_LC | | | | | | | | | | | | | | | | | | | | | | | | | | |
| 21-225_11F10_LC | . | . | . | . | . | . | . | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . | . | . | . | . | . | . | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | | |
| 21-225_13E6_LC | . | . | . | V | . | . | . | . | . | . | . | . | . | . | . | . | . | . | . | . | . | . | S | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | . | . | . | . | . | . | . |
| 21-225_146F1_LC | | | | | | | | | | | | | | | | | | | | . | . | . | . | . | . | . |
| 21-225_146G11_LC | | | | | | | | | | | | | | | | | | | | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | . | . | . | . | . | . | . | . | . | . | . | . | . | . | . | . | . | . | . | . | . | . | . |
| 21-225_148F12_LC | . | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | . | . | . | . | . | . | . | . | . | . |
| 21-225_149G1_LC | . | . | . | . | . | . | . | . | . | . | . | . | . | . | . | . | A | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | . | . | . | . | Y | . | . | . | . | . | . | . | . | . | . | . | . | . | . | . | . | . |
| 21-225_14B2_LC | | | | | . | . | . | . | . | . | . | . | . | . | . | . | . | . | . | . | . | . | . | . | . | . |
| 21-225_14C2_LC | | | | I | . | . | . | . | . | . | S | . | C | . | . | . | . | . | . | I | . | . | . | . | . | . |
| 21-225_14E3_LC | . | . | . | . | . | . | . | . | . | . | . | . | . | . | . | . | . | . | . | . | . | . | . | . | . | . |
| 21-225_150E7_LC | . | . | . | . | . | . | . | . | . | . | . | . | . | . | . | . | . | . | . | . | . | . | . | . | . | . |
| 21-225_152H9_LC | . | . | . | . | . | . | . | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . | . | . | . | . | . | . | . |
| 21-225_158H5_LC | . | . | . | I | . | . | . | . | S | . | S | . | . | . | . | . | . | . | . | . | . | . | . | . | . | . |
| 21-225_15A1_LC | . | . | . | . | . | . | . | . | . | . | . | . | . | . | . | . | . | . | . | . | . | . | . | . | . | . |
| 21-225_15C9_LC | | | | . | . | . | . | . | . | . | . | . | . | . | . | . | . | . | . | . | . | . | . | . | . | . |
| 21-225_15E3_LC | | | | . | . | . | . | . | . | . | . | . | . | . | . | . | . | . | . | . | . | . | . | . | . | . |
| 21-225_15G7_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_15H1_LC | . | . | . | . | . | . | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | . | . | . | . | . | ~ | . | . | . | . | . | . | . | . | . | . | . | . | . | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | . | . | . | . | . | . | . | . | . | . | . | . | . | . | . | . | . | . | . | . | . |
| 21-225_169F9_LC | . | . | . | . | . | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | . | . | ! | . | . | . | . | . | ~ | . | . | . | . | . | . | . | . | . | . | . | . | . | . | . |
| 21-225_170D11_LC | . | . | . | . | . | . | . | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . | . | . | . | . | . | . | . |
| 21-225_170G4_LC | . | . | . | . | . | . | . | . | . | . | . | . | . | . | . | . | . | . | . | . | . | . | . | . | . | . |
| 21-225_170G5_LC | . | . | . | . | . | . | . | . | . | . | . | . | . | . | . | . | . | . | . | . | . | . | . | . | . | . |
| 21-225_171A4_LC | . | . | . | . | . | . | . | . | . | . | . | . | . | . | . | . | . | . | . | . | . | . | . | . | . | . |
| 21-225_171C3_LC | . | . | . | . | . | . | . | . | . | . | . | . | . | . | . | . | . | . | . | . | . | . | . | . | . | . |
| 21-225_172B3_LC | . | . | . | . | . | . | . | . | . | . | . | . | . | . | . | . | . | . | . | . | . | . | . | . | . | . |
| 21-225_172G8_LC | . | . | . | . | . | . | . | . | . | . | . | . | . | . | . | . | . | . | . | . | . | . | . | . | . | . |
| 21-225_173H12_LC | . | . | . | . | . | . | . | . | . | . | . | . | . | . | . | . | . | . | . | . | . | . | . | . | . | . |
| 21-225_175G1_LC | . | . | . | . | . | . | . | . | . | . | . | . | . | . | . | . | . | . | . | . | . | . | . | . | . | . |
| 21-225_176B11_LC | . | . | . | . | . | . | . | . | . | . | . | . | . | . | . | . | . | . | . | . | . | . | . | . | . | . |
| 21-225_178B10_LC | . | . | . | . | . | . | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . | . | . | . | . | . | . | . |
| 21-225_17F9_LC | . | . | . | . | . | . | . | . | . | . | . | . | . | . | . | . | . | . | . | . | . | . | . | . | . | . |
| 21-225_17H12_LC | . | . | . | . | . | . | . | . | . | . | . | . | . | . | . | . | . | . | . | . | . | . | . | . | . | . |
| 21-225_17H8_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | K_FR1 | | | | | | | | | | | | | | | | | |
| CONSENSUS | D | I | Q | M | T | Q | S | P | S | S | L | S | A | S | V | G | D | R | V | T | I | T | C | R | A | S |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | . | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | T | . |
| 21-225_18E8_LC | . | . | . | . | . | . | A | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | . | . |
| 21-225_193A5_LC | . | . | . | . | . | . | . | . | . | . | . | S | . | . | . | . | . | . | . | I | . | S | . | . | . | . |
| 21-225_193E7_LC | . | . | . | . | . | . | . | . | . | . | . | . | . | . | . | . | . | . | . | . | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | . | . | . | . | . | . | . | . | . | . | . | . | . | . | . | . | . | . | . | . | . | . | . |
| 21-225_19D11_LC | . | . | . | . | . | . | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | . | . | . | . | . | . | . | . | . | . | S | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | . | . |
| 21-225_200F12_LC | . | . | . | . | . | . | . | . | . | . | . | . | . | . | . | . | . | . | . | . | . | . | . | . | . | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | . | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | . | . | . |
| 21-225_204H8_LC | . | . | . | . | . | . | . | . | . | . | . | . | . | . | . | . | . | . | . | . | . | . | . | . | . | . |
| 21-225_205H8_LC | . | . | . | . | . | . | . | . | . | . | . | . | . | . | . | . | . | . | . | . | . | . | . | . | . | . |
| 21-225_20A10_LC | . | . | . | . | . | . | . | . | . | . | . | . | . | . | . | . | . | . | . | . | . | . | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | . | . | . | . | . | . | . | . | . | . | . | Q | . | . | . | . | . | . | . | . | . | . |
| 21-225_20C8_LC | . | . | . | . | . | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_20G9_LC | . | | | | | | | | | | | | | | | | | | | | | | | | | |
| 21-225_20H10_LC | Q | . | | | | | | | | | | | | | | | | | | | | | | | | |
| 21-225_20H12_LC | . | | . | | | | | | | | . | | | | | . | | | | | | | | . | | |
| 21-225_21E5_LC | . | | . | | | | | | | | . | | | | | . | | | | | | | | . | | |
| 21-225_21F3_LC | . | | . | | | | | | | | . | | | | | . | | | | | | | | . | | |
| 21-225_21G7_LC | . | | . | | | | | | | | . | | | | | . | | | | | | | | . | | |
| 21-225_21H3_LC | . | | . | | | | | | | | . | | | | | . | | | | | | | | . | | |
| 21-225_224A1_LC | . | | . | | | | | | | | . | | | | | . | | | | | | | | . | | |
| 21-225_224B1_LC | . | | . | | | | | | | | . | | | | | . | | | | | | | | . | | |
| 21-225_224C3_LC | . | | . | | | | | | | | . | | | | | . | | | | | | | | . | | |
| 21-225_224D10_LC | . | . | L | . | | | | | | | . | | | | | . | | | | | | | | | | |
| 21-225_22B7_LC | . | | . | | | | | | | | . | | | | | . | | | | | | | | . | | |
| 21-225_22C1_LC | . | | . | | | | | | | | . | | | | | . | | | | | | | | . | Y | . |
| 21-225_22D12_LC | . | | . | | | | | | | | . | | | | | . | | | | | | | | . | | |
| 21-225_22F2_LC | . | | . | | | | | | | | . | | | | | . | | | | | | | | . | | |
| 21-225_22F9_LC | . | | . | | | | | | | | . | | | | | . | | | | | | | | . | | |
| 21-225_22G8_LC | . | | | | S | | | | | | | S | | | | . | | | | | | | | . | Y | . |
| 21-225_22G9_LC | . | | . | | | | | | | | . | | | | | . | | | | | | | | . | | |
| 21-225_22H4_LC | . | | . | | | | | | | | . | | | | | . | | | | | | | | . | | |
| 21-225_23A3_LC | . | | . | | | | | | | | . | | | | | . | | | | | | | | . | | |
| 21-225_23C8_LC | . | | . | | | | | | | S | . | | | | | . | | | | | | | | . | | |
| 21-225_23D1_LC | . | | . | | | | | | | | . | | | | | . | | | | | | | | | | |
| 21-225_23H11_LC | . | | . | | | | | | | | . | | | | | . | | | | | | | | | | |
| 21-225_24E5_LC | . | | . | | | | | | | | . | | | | | . | | | | | | | | | | |
| 21-225_25E6_LC | . | | . | | | | | | | | . | | | | | . | | | | | | | | | | |
| 21-225_25H10_LC | . | | . | | | | | | | | . | | | | | . | | | | | | | | | | |
| 21-225_25H11_LC | . | | . | | | | | | | | . | | | | | . | | | | | | | | . | | |
| 21-225_27C3_LC | . | | . | | | | | | | | . | | | | | . | | | | | | | | . | . | . |
| 21-225_27F2_LC | . | | . | | | | | | | | . | | | | | . | | | | | | | | . | . | . |

**FIGURE 57**

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_35A6_LC | . | . | . | . | . | . | . | . | . | . | . | . | . | . | . | . | . | . | . | . | . | . | . | . | . | . |
| 21-225_35B7_LC | . | . | . | . | . | . | . | . | . | . | . | . | . | . | . | . | . | . | ı | . | . | . | . | . | . | . |
| 21-225_35D1_LC | . | . | . | . | . | . | . | . | . | . | . | . | . | . | . | . | . | . | . | . | . | . | . | . | . | . |
| 21-225_35F11_LC | . | . | . | . | . | . | . | . | . | . | . | . | . | . | . | . | . | . | . | . | . | . | . | . | . | . |
| 21-225_36A5_LC | . | . | . | . | . | . | . | . | . | . | . | . | . | . | . | . | . | . | . | . | . | . | . | . | T | . |
| 21-225_36C5_LC | . | . | . | . | . | . | . | . | . | . | . | . | . | . | . | . | . | . | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | . | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | T | . |
| 21-225_46B1_LC | . | . | . | . | . | . | . | . | . | . | . | . | . | . | . | . | . | . | . | . | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | . | . | . | . | . | . | . | . | . | . | . | . | . | . | . |
| 21-225_46F3_LC | . | . | . | . | . | . | . | . | . | . | . | . | . | . | . | . | . | . | . | . | . | . | . | . | . | . |
| 21-225_47A1_LC | . | . | . | . | . | . | . | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . | . | . | . | . | . | . | . |
| 21-225_48H4_LC | . | . | . | . | . | . | S | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . | . | . | . | . | . | . | . |
| 21-225_4C8_LC | . | . | . | . | ı | . | . | . | S | . | S | . | C | . | . | . | . | . | . | . | . | . | . | . | . | . |
| 21-225_4E10_LC | . | . | . | . | . | . | . | . | . | . | . | S | . | . | . | . | . | . | . | . | . | . | . | . | . | . |
| 21-225_4F12_LC | . | . | . | . | . | . | . | . | . | . | . | . | . | . | . | . | . | . | . | . | . | . | . | . | . | . |
| 21-225_4F4_LC | . | . | . | . | . | . | . | . | . | . | . | . | . | . | . | . | . | . | . | . | . | . | . | . | . | . |
| 21-225_4H4_LC | . | . | . | . | . | . | . | . | . | . | . | . | . | . | . | . | . | . | . | . | . | . | . | . | . | . |
| 21-225_50G10_LC | . | . | . | . | . | . | . | . | . | . | . | . | . | . | . | . | . | . | . | . | . | . | . | . | . | . |
| 21-225_51F11_LC | . | . | . | . | . | . | . | . | . | A | . | . | . | . | . | . | . | . | . | . | . | . | . | . | . | . |
| 21-225_52B2_LC | . | . | . | . | . | . | . | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | . | A | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_54C6_LC | . | . | . | . | . | . | . | . | . | . | . | . | . | . | . | . | A | . | . | . | . | . | . | . | . | . |
| 21-225_55B8_LC | . | . | . | . | . | . | . | . | . | . | . | . | S | . | . | . | . | . | . | . | . | . | . | . | . | . |
| 21-225_55H6_LC | . | . | . | . | . | . | . | . | . | . | . | . | . | . | . | . | . | . | . | . | . | . | . | . | . | . |
| 21-225_56F1_LC | . | . | . | . | . | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | T | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | . | . | . | . | . | . | . | . | . | . | . | . | . | . | . | S | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | . | . | . | . | . | . | G | . | . | . | . | . | . | . |
| 21-225_6D4_LC | . | . | . | . | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | . | . | . | . | . | . | T | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | . | . | . | . | . | . | . | . | . | . |
| 21-225_75H7_LC | . | . | . | . | . | . | . | . | . | . | R | Y | . | . | . | . | . | . | . | . | . | N | S | . | . | . |
| 21-225_7C4_LC | . | . | . | . | . | . | . | . | . | . | . | F | . | . | . | . | . | . | . | . | . | . | . | . | . | . |
| 21-225_7E9_LC | . | . | . | . | . | . | . | . | . | . | . | F | . | . | . | . | . | . | . | . | . | . | . | . | . | . |
| 21-225_7F4_LC | . | . | . | . | . | . | . | . | . | . | . | . | . | . | . | . | . | . | . | . | . | . | . | . | T | . |
| 21-225_7H11_LC | . | . | . | . | . | . | . | . | . | . | A | . | . | . | . | . | V | . | . | . | I | . | L | . | . | . |
| 21-225_7H7_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_8C9_LC | . | . | . | . | . | . | . | . | . | . | . | . | . | . | ? | . | . | . | . | . | . | . | . | . | . | . |
| 21-225_8D12_LC | . | . | . | . | . | . | . | . | . | . | . | . | . | . | . | . | . | . | . | . | . | . | . | . | . | . |
| 21-225_8F11_LC | . | . | . | . | . | . | . | . | . | . | . | . | . | . | . | . | . | . | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | . | . | . | . | . | . | . | . | . | . |
| 21-225_9E8_LC | . | . | . | . | . | . | . | . | . | . | . | ? | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_10G5_LC | | | | | | | | | | | | | | | | | | | | | | | | | | |
| 21-225_10H12_LC | | | | | | | | | | | | | | | | | | | | | | | | | | |
| 21-225_11F10_LC | | | | | | | | | | | | | | | | | | | | | | | | | | |
| 21-225_11F5_LC | | | | | | | | | | | | | | | | | | | | | | | | | | |
| 21-225_11G7_LC | | | | | | | | | | | | | | | | | | | | | | | | | | |
| 21-225_12A1_LC | | | | | | | | | | | | | | | | | | | | | | | | | | |
| 21-225_13E6_LC | | | | | | | | | | | | | | | | | | | | | | | | | | |
| 21-225_13H3_LC | | | | | | | | | | | | | | | | | | | | | | | | | | |
| 21-225_13H5_LC | | | | | | | | | | | | | | | | | | | | | | | | | | |
| 21-225_146F1_LC | | | | | | | | | | | | | | | | | | | | | | | | | | |
| 21-225_146G11_LC | | | | O | | | | | | | | | | N | S | | | | | | | | | | | |
| 21-225_146H9_LC | | | | | | | | | | | | | | | | | | | | | | | | | | |
| 21-225_148E11_LC | | | | | | | | | | | | | | | | | | | | | | | | | | |
| 21-225_148F12_LC | | | | | | | | | | | | | | A | | | | | | | | | | | | |
| 21-225_149F12_LC | | | | | | | | | | | | | | | | | | | | | | | | | | |
| 21-225_149G1_LC | | | | | | | | | | | | | | | | | | | | | | | | | | |
| 21-225_149G5_LC | | | | | | | | | | | | | | A | | | | P | | | | | | | | |
| 21-225_14B1_LC1_LC | | | | O | | | | | | | | | O | N | | | | | | | | | | | | |
| 21-225_14B2_LC | | | | | | | | | | | | | | | | | | | | | | | | | | |
| 21-225_14C2_LC | | | | | | | | | | | | | | | O | | | | | | | | | | | |
| 21-225_14E3_LC | | | | | | | | | | | | | | | | | | | | | | | | | | |
| 21-225_150E7_LC | | | | | | | | | | | | | | | | | | | | | | | | | | |
| 21-225_152H9_LC | | | | | | | | | | | | | | | | | | | | | | | | | | |
| 21-225_156G1_LC | | | | | | | | | | | | | | | | | | | | | | | | | | |
| 21-225_158H5_LC | | | | | W | | | | | | | | I | | | | | | | | | | | | | |
| 21-225_15A1_LC | | | | | | | | | | | | | | | | | | | | | | | | | | |
| 21-225_15C9_LC | | | | | | | | | | | | | | | | | | | | | | | | | | |
| 21-225_15E3_LC | | | | | | | | | | | | | | | | | | | | | | | | | | |
| 21-225_15G7_LC | | | | | | | | | | | | | | | | | | | | | | | | | | |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_15H1_LC | . | . | . | . | . | . | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | . | . | . | . | . | . | . | . | . | . | . | . | . | . | . | . | . | . | T | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | . | . | . | . | . | . | . | . | . | . | . | . | . | . | . | . | . | . | . | . | . |
| 21-225_169F9_LC | . | . | . | . | . | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | . | . | . | . | . | . | . | . | . | . | . | . | . | . | . | . | . | . | . | S | . | . | . | . |
| 21-225_170D11_LC | . | . | . | . | . | . | . | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . | E | . | . | . | . | . | . |
| 21-225_170G4_LC | . | . | . | . | . | . | . | . | . | . | . | . | . | . | . | . | . | . | M | . | . | . | . | . | . | . |
| 21-225_170G5_LC | . | . | . | . | . | . | . | . | . | . | . | . | T | . | . | . | . | F | . | . | . | . | . | . | . | . |
| 21-225_171A4_LC | . | . | . | . | . | . | . | . | . | . | . | . | . | . | . | . | . | . | . | . | . | . | . | . | . | . |
| 21-225_171C3_LC | . | . | . | . | . | . | . | . | . | . | . | . | T | . | . | . | . | F | . | . | . | . | . | . | . | . |
| 21-225_172B3_LC | . | . | . | . | . | . | . | . | . | . | . | . | . | . | . | . | . | . | . | . | . | . | . | . | . | . |
| 21-225_172G8_LC | . | . | . | V | . | . | . | . | . | . | . | . | . | . | . | . | . | . | . | . | . | . | . | . | . | . |
| 21-225_173H12_LC | . | . | . | . | . | . | . | . | . | . | . | . | . | . | . | . | . | . | . | . | . | . | . | . | . | . |
| 21-225_175G1_LC | . | . | . | . | . | . | . | . | . | . | . | . | T | . | . | . | . | . | . | . | . | . | . | . | . | . |
| 21-225_176B11_LC | . | . | . | . | . | . | . | . | . | . | . | . | . | . | . | . | . | F | . | . | . | . | . | . | . | . |
| 21-225_178B10_LC | . | . | . | . | . | . | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . | . | . | . | . | . | . | . |
| 21-225_17F9_LC | . | . | . | V | . | . | . | . | . | . | . | . | . | . | . | . | . | . | . | . | . | . | . | . | . | . |
| 21-225_17H12_LC | . | . | . | G | . | . | . | . | . | . | . | . | . | . | . | . | . | . | . | . | . | A | . | . | . | . |
| 21-225_17H8_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | . | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | . | . |
| 21-225_193A5_LC | . | . | . | . | . | . | . | . | . | . | . | . | . | . | . | . | . | . | . | . | Y | . | . | . | . | . |
| 21-225_193E7_LC | . | . | . | . | . | . | . | . | . | . | . | . | . | . | . | . | . | . | . | . | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | . | . | . | . | . | . | . | . | . | . | . | . | . | . | . | . | . | . | . | . | . | . | . |
| 21-225_19D11_LC | . | . | . | . | . | . | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | . | . | . | . | . | . | . | . | . | . | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | . | . |
| 21-225_200F12_LC | . | . | . | . | . | . | . | . | . | . | . | . | . | . | . | . | . | . | . | . | . | . | . | ※ | . | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | . | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | ※ | . | . |
| 21-225_204H8_LC | . | . | . | . | . | . | . | . | . | . | . | . | . | . | . | . | . | . | . | . | . | . | . | . | . | . |
| 21-225_205H8_LC | . | . | . | . | . | . | . | . | . | . | . | . | . | . | . | . | . | . | . | . | Q | . | . | . | . | . |
| 21-225_20A10_LC | . | . | . | . | . | . | . | . | . | . | . | . | . | . | . | . | . | . | . | . | Q | . | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | . | . | . | . | . | . | . | . | . | . | . | . | . | . | . | . | . | . | . | . | . | . |
| 21-225_20C8_LC | . | . | . | . | . | . | . | . | . | . | . | . | . | . | . | . | . | . | ※ | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_20G9_LC | . | . | . | . | . | . | . | . | . | . | . | . | . | . | . | . | . | . | . | . | . | . | . | . | . | . |
| 21-225_20H10_LC | . | . | . | . | . | K | . | . | . | . | . | . | . | N | . | . | . | . | . | . | . | . | . | G | . | . |
| 21-225_20H12_LC | . | . | . | . | . | . | . | . | . | . | . | . | . | . | . | D | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | . | . | . | . | . | . | . | . | . | . | . | . | . | . | . | . | . | . | . | . | . |
| 21-225_21G7_LC | . | . | . | . | V | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | . | . | . | . | . | . | . | . |
| 21-225_224A1_LC | . | . | . | . | . | E | . | . | . | . | . | . | . | . | . | . | . | . | . | . | . | . | . | . | . | . |
| 21-225_224B1_LC | . | . | . | . | . | S | . | . | . | . | . | . | . | . | . | . | . | . | . | . | . | . | . | . | . | . |
| 21-225_224C3_LC | . | . | . | D | . | . | . | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | . | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | . | . |
| 21-225_22D12_LC | . | . | . | . | . | . | . | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . | . | . | . | . | . | . | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | . | . |
| 21-225_23A3_LC | . | . | . | . | . | . | . | . | . | . | . | . | . | . | . | . | . | . | . | . | . | . | . | . | . | . |
| 21-225_23C8_LC | . | . | . | D | . | . | . | . | . | . | . | . | . | . | . | . | . | . | . | . | . | . | R | . | . | . |
| 21-225_23D1_LC | . | . | . | . | . | . | . | . | . | . | . | . | . | . | . | . | . | . | . | . | . | . | . | . | . | . |
| 21-225_23H11_LC | . | . | . | . | . | . | . | . | . | . | . | . | . | . | . | D | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | R | . | . | . | . | . | . |
| 21-225_25H10_LC | . | R | . | . | . | . | . | . | . | . | . | . | . | . | . | . | . | . | . | . | . | . | . | . | . | . |
| 21-225_25H11_LC | . | . | . | . | . | . | . | . | . | . | . | . | T | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_29B8_LC | | | | | | | | | | | | | | | | | | | | | | | | | | |
| 21-225_29G4_LC | | | | | | | | | | | | | | | | | | | | | | | | | | |
| 21-225_29G8_LC | | | | | | | | | | | | | | | | | | | | | L | | | | | |
| 21-225_29H6_LC | | | | | | | | | | | | | | | | | | | | | | S | | | T | |
| 21-225_2A11_LC | | | | | | | | | | | | | | | | | | | | | | | | | | |
| 21-225_2B2_LC | | | | | | | | | | | | | | | | | | | | | | | | Q | | |
| 21-225_2G9_LC | | | | | | | | | | | | | | | | | | | | | | | | | | |
| 21-225_30D1_LC | | | | D | | | | | | | | | S | | | | | | | | | | | | | |
| 21-225_30E3_LC | | | | D | | | | | | | | | C | | | | | | | | | | | | | |
| 21-225_30E9_LC | | | | D | | | | | | | | | D | | | | | | | | | | | | | |
| 21-225_30F3_LC | | | | | | | | | | | | | | | | | | | | | | | | | | |
| 21-225_30G11_LC | | | | D | | | | | | | | | S | | | | | | | | | | | | | |
| 21-225_31A8_LC | | | | D | | | | | | | | | S | | | | | | | | | | | | | |
| 21-225_31B8_LC | | | | Q | | | | | | | | | S | | | | | | | | | | | | | |
| 21-225_31H3_LC | | | | D | | | | | | | | | D | | | | | | | | | | | | | |
| 21-225_31H5_LC | | | | D | | | | | | | | | S | | | | | | | | | | | | | H |
| 21-225_32G1_LC | | | | D | M | | | | | | | | S | | | | | | | | | | | | | |
| 21-225_32G12_LC | | | | Q | | | | | | | | | S | | | | | | | | K | | | | | |
| 21-225_33A4_LC | | | | D | | | | | | | | | | | V | | | | | | | | | | | |
| 21-225_33A7_LC | | | | D | | | | | | | | | S | | | | | | | | | | | | | |
| 21-225_33B1_LC | | | | | | | | | | | | | | | | | | P | | | | | | | | |
| 21-225_33B8_LC | | | | D | | | | | | | | | S | | | | | | | | | | | | | |
| 21-225_33F1_LC | | | | D | | | | | | | | | S | | | | | | | | | | | | | |
| 21-225_33G7_LC | | | | | | | | | | | | | | | | | | | | | | | | | | |
| 21-225_34A6_LC | | | | D | | | | | | | | | | | | | | | | | | | | | | |
| 21-225_34D11_LC | | | | D | | | | | | | | | S | | | | | | | | | | | | | |
| 21-225_34G8_LC | | | | D | | | | | | | | | S | | | | | | | | | | | | | |
| 21-225_34G9_LC | | | | D | | | | | | | | | S | | | | | | | | | | | | | |
| 21-225_34H11_LC | | | | Q | | | | | | | | | | | | | | | | | | | | | | |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_35A6_LC | . | . | . | G | . | . | . | . | . | . | . | . | S | . | . | . | . | . | . | . | . | . | . | . | . | . |
| 21-225_35B7_LC | . | . | . | G | . | . | . | . | . | . | . | . | S | . | . | . | . | . | . | . | . | . | . | . | . | . |
| 21-225_35D1_LC | . | . | . | G | . | . | . | . | . | . | . | . | S | . | . | . | . | . | . | . | . | . | . | . | . | . |
| 21-225_35F11_LC | . | . | . | G | . | . | . | . | . | . | . | . | S | . | . | . | . | . | . | . | . | . | . | . | . | . |
| 21-225_36A5_LC | . | . | . | G | . | . | . | . | . | . | . | . | . | . | . | . | . | . | . | . | . | . | . | . | . | . |
| 21-225_36C5_LC | . | . | . | G | . | . | . | . | . | . | . | . | S | . | . | . | . | . | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | . | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . | . | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | . | X | . | . | . | . | . | . | . | . | . | . | . | . | . |
| 21-225_46F3_LC | . | . | . | . | . | . | . | . | . | . | . | . | X | . | . | . | . | . | . | . | . | . | . | . | . | . |
| 21-225_47A1_LC | . | . | . | G | . | . | . | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . | . | . | . | . | . | . | . |
| 21-225_48H4_LC | . | . | X | G | . | . | . | . | . | . | . | . | . | . | . | . | . | . | . | . | . | . | . | . | . | . |
| 21-225_49A1_LC | . | . | . | . | . | . | . | . | . | . | . | . | . | . | . | G | . | . | . | . | . | . | . | . | . | . |
| 21-225_49C6_LC | . | . | . | . | . | . | . | . | . | . | . | . | . | . | . | . | . | . | . | . | . | . | . | . | . | . |
| 21-225_4C8_LC | . | . | . | . | . | . | . | . | . | . | . | . | . | . | . | . | . | . | . | . | . | . | . | . | . | . |
| 21-225_4E10_LC | . | . | . | . | . | . | . | . | . | . | . | . | . | . | . | . | . | . | . | . | . | . | . | . | . | . |
| 21-225_4F12_LC | . | . | . | . | . | . | . | . | . | . | . | . | . | . | . | . | . | . | . | . | . | . | . | . | . | . |
| 21-225_4F4_LC | . | . | . | . | . | . | . | . | . | . | . | . | . | . | . | . | . | . | . | . | . | . | . | . | . | . |
| 21-225_4H4_LC | . | . | . | . | . | . | . | . | . | . | . | . | . | . | . | . | . | . | . | . | . | . | . | . | . | . |
| 21-225_50G10_LC | . | . | . | . | . | . | . | . | . | . | . | . | . | N | . | . | . | . | . | . | . | . | . | . | V | . |
| 21-225_51F11_LC | . | . | . | . | . | . | . | . | . | . | . | . | . | . | . | . | . | . | . | . | . | . | . | . | . | . |
| 21-225_52B2_LC | . | . | . | . | . | . | . | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | . | . | . | . | . | N | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_54C6_LC | . | . | . | . | . | . | . | . | . | . | . | . | . | . | . | . | . | . | . | . | N | . | . | . | . | . |
| 21-225_55B8_LC | . | . | . | A | . | . | . | . | . | . | . | . | . | . | . | . | . | . | . | . | . | . | . | . | . | . |
| 21-225_55H6_LC | . | . | . | . | . | . | . | . | . | . | . | . | . | . | . | . | . | . | . | . | . | . | . | . | . | . |
| 21-225_56F1_LC | . | . | . | O | . | . | . | . | . | . | . | . | . | N | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | . | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | . | . | . | . | . | . | . | . | . | . | . | . | S | . | . | . | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | S | N | . | . | . | . | . | . | . | . | . | . | . | . |
| 21-225_6D4_LC | . | . | . | A | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | N | . | . | . | . | . | . | . | N | . | N | . | S | . | . | . | . | . | . | . | . |
| 21-225_70E8_LC | . | . | . | . | . | . | . | . | . | . | . | . | K | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | . | . | . | . | . | . | . | . | . | . |
| 21-225_75H7_LC | . | . | . | . | . | . | . | . | . | . | . | . | . | . | . | . | . | . | . | . | . | . | . | . | S | . |
| 21-225_7C4_LC | . | . | . | . | . | . | . | . | . | . | . | . | . | . | . | . | . | . | . | . | . | . | . | . | . | X |
| 21-225_7E9_LC | . | . | . | . | . | . | . | . | . | . | . | . | . | . | . | . | . | X | . | . | . | . | . | . | . | . |
| 21-225_7F4_LC | . | . | . | . | . | . | . | . | . | . | . | . | . | . | . | . | . | . | . | . | . | . | . | . | . | . |
| 21-225_7H11_LC | . | . | . | . | . | . | . | . | . | . | . | . | . | . | . | . | . | . | . | . | . | . | . | . | . | . |
| 21-225_7H7_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | G | I | R | - | - | - | - | - | - | N | D | L | G | W | Y | Q | Q | K | P | G | K | A | P |
| 21-225_8C9_LC | . | . | . | A | . | . | . | . | . | . | . | . | . | . | . | D | . | . | . | . | . | . | . | . | . | . |
| 21-225_8D12_LC | . | . | . | . | . | . | . | . | . | . | . | . | . | . | . | . | . | . | . | . | . | . | . | . | . | . |
| 21-225_8F11_LC | . | . | . | . | . | . | . | . | . | . | . | . | S | . | . | . | . | F | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | . | . | . | . | . | . | . | . | . | . |
| 21-225_9E8_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | G | . | . | . | . | . | . | . | . | . | . | . | . | . | . | . | . | . | . | . | . |
| 21-225_11F10_LC | . | . | . | . | . | . | . | . | . | . | . | . | . | . | . | . | . | . | . | . | . | . | . | . | . | . |
| 21-225_11F5_LC | . | . | . | . | . | . | . | . | . | . | . | . | . | . | . | . | V | . | . | . | . | . | . | . | . | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | . | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | T | . | . | . | . | . | . |
| 21-225_146F1_LC | . | . | . | . | . | . | . | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . | . | . | . | . | L | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | . | S | . | . | . | . | . | . | . | . | . | . | . | . | . | . | . | . | . | . | . | . | . |
| 21-225_148F12_LC | . | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | N | . | . | . | . | . | . | . | . | . |
| 21-225_149G1_LC | . | . | . | . | . | . | . | . | . | . | . | . | . | . | . | . | N | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | T | . | . | . | . | . | . | . | . | . | . | Y | . | . | . | . | . | . | . | . | . | . |
| 21-225_14B2_LC | . | . | . | . | V | . | . | . | . | . | . | . | . | . | T | . | . | . | . | G | . | . | . | . | . | . |
| 21-225_14C2_LC | . | . | . | . | . | . | . | . | . | . | . | . | . | . | . | . | . | . | . | . | . | . | . | . | . | . |
| 21-225_14E3_LC | . | . | I | . | S | . | . | . | . | . | . | . | . | . | . | . | . | . | . | . | . | . | . | . | . | . |
| 21-225_150E7_LC | . | . | . | . | . | . | . | . | . | . | . | . | . | . | . | . | N | . | . | . | . | . | . | L | . | . |
| 21-225_152H9_LC | . | . | . | . | . | . | . | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . | . | . | . | . | . | . | . |
| 21-225_158H5_LC | . | . | . | . | R | . | . | . | . | . | . | . | . | . | . | . | . | . | . | . | . | . | . | . | . | . |
| 21-225_15A1_LC | . | . | . | . | . | . | . | . | . | . | . | . | . | . | . | . | . | . | . | . | . | . | . | . | . | . |
| 21-225_15C9_LC | . | . | . | . | . | . | . | . | . | . | . | . | . | . | . | . | . | . | . | . | . | . | . | . | . | . |
| 21-225_15E3_LC | . | . | . | . | . | . | . | . | . | . | . | . | . | . | . | . | S | . | . | . | . | . | . | . | . | . |
| 21-225_15G7_LC | . | . | . | . | . | . | . | . | . | . | . | . | . | . | . | . | . | . | . | . | . | I | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_15H1_LC | . | . | . | . | . | T | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | F | . | . | . | . | . | . | . | . | . | . | . | . | . | . | . | . | . | . | . | . | . |
| 21-225_160B10_LC | . | . | . | . | . | . | . | . | . | . | . | . | . | . | . | . | . | . | . | . | . | . | . | . | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | G | . | . | . | . | . | . | . | . | . | S | . | . | . | . | . | . | . | . | . | . | . |
| 21-225_169F9_LC | . | . | . | . | F | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | V | . | . | . | . | . | . | . | . |
| 21-225_16F4_LC | . | . | . | . | . | V | . | . | . | . | . | . | . | . | . | . | . | . | . | . | . | . | . | . | . | . |
| 21-225_170D11_LC | . | R | . | . | . | . | . | . | . | . | . | . | . | . | . | . | . | . | . | N | . | . | . | . | . | . |
| 21-225_170D5_LC | . | R | . | . | . | . | . | . | . | . | . | . | . | . | . | . | . | . | . | N | . | . | . | . | . | . |
| 21-225_170G4_LC | . | . | . | . | . | . | . | . | . | . | . | . | . | . | . | . | T | . | . | . | . | . | . | . | . | . |
| 21-225_170G5_LC | . | . | . | . | . | T | . | . | . | . | . | . | . | . | . | . | . | . | . | . | . | . | . | . | . | . |
| 21-225_171A4_LC | . | . | . | . | . | T | . | . | . | . | . | . | . | . | . | . | . | . | . | . | . | . | . | . | . | . |
| 21-225_171C3_LC | . | . | . | . | . | T | . | . | . | . | . | . | . | . | . | . | . | . | . | . | . | . | . | . | . | . |
| 21-225_172B3_LC | . | . | . | . | G | . | . | . | . | . | . | . | . | . | S | . | . | . | . | . | . | . | . | . | . | . |
| 21-225_172G8_LC | . | R | . | . | . | . | . | . | . | . | . | . | . | . | . | . | . | . | . | N | . | . | . | . | . | . |
| 21-225_173H12_LC | . | . | . | . | . | T | . | . | . | . | . | . | . | . | T | . | . | . | . | . | . | . | . | . | . | . |
| 21-225_175G1_LC | . | . | . | . | . | T | . | . | . | . | . | . | . | . | . | . | . | . | . | . | . | . | . | . | . | . |
| 21-225_176B11_LC | . | . | . | . | . | . | . | . | . | . | . | . | . | . | . | . | . | . | . | . | . | . | . | . | . | . |
| 21-225_178B10_LC | . | R | . | . | . | . | . | . | . | . | . | . | . | . | . | . | . | . | . | N | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | N | . | . | . | . | . | . | . | . | . |
| 21-225_17F9_LC | . | . | . | . | . | . | . | . | . | . | . | . | . | . | V | . | . | . | . | . | . | . | . | . | . | . |
| 21-225_17H12_LC | . | . | . | N | . | . | . | . | . | . | . | . | . | . | . | . | . | . | . | . | . | . | . | . | . | . |
| 21-225_17H8_LC | . | . | . | . | . | G | . | . | . | . | . | . | . | . | . | . | . | . | . | . | . | . | . | . | . | . |

4222

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | V | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | . | . |
| 21-225_193A5_LC | . | V | . | . | . | . | . | . | . | . | . | . | . | . | . | . | . | . | . | . | . | . | . | . | . | . |
| 21-225_193E7_LC | . | . | . | . | . | S | . | . | . | . | . | . | . | . | . | . | . | . | . | R | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | S | S | . | . | . | . | . | . | . | . | . | . | . | . | . | . | R | . | . | . | . | . | . |
| 21-225_19D11_LC | . | . | . | . | . | . | . | . | . | . | . | . | . | . | T | . | . | . | . | . | . | . | . | . | . | . |
| 21-225_19E1_LC | . | . | . | . | . | . | . | . | . | . | . | . | . | . | . | . | N | . | . | . | . | . | . | . | . | . |
| 21-225_19F3_LC | . | . | . | . | . | . | . | . | . | . | . | . | . | . | V | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | V | . | . | . | . | . | . | . | . |
| 21-225_200F12_LC | . | . | . | . | . | S | . | . | . | . | . | . | . | . | . | . | . | . | M | T | . | . | . | . | . | . |
| 21-225_200H9_LC | . | . | . | . | . | . | . | . | . | . | . | . | . | . | . | . | . | . | . | . | . | I | . | . | . | . |
| 21-225_201A1_LC | . | . | . | . | . | S | . | . | . | . | . | . | . | . | . | . | . | I | . | R | . | . | . | . | . | . |
| 21-225_201A10_LC | . | . | . | . | . | S | . | . | . | . | . | . | . | . | T | . | . | . | M | . | . | . | . | . | . | . |
| 21-225_204H8_LC | . | . | . | . | . | S | . | . | . | . | . | . | . | . | . | . | . | . | . | . | . | . | . | . | . | . |
| 21-225_205H8_LC | . | L | . | . | . | S | . | . | . | . | . | . | . | . | . | . | . | F | . | R | . | . | . | . | . | . |
| 21-225_20A10_LC | . | . | . | . | . | . | . | . | . | . | . | . | . | . | . | . | . | . | . | . | . | . | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | F | . | . | . | . | . | . | . | . | . | . | . | . | . | . | . | . | . | . | . | . | . |
| 21-225_20C8_LC | . | . | . | . | N | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | F | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | F | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_20G9_LC | . | . | . | . | . | . | . | . | . | . | . | . | . | . | . | . | . | . | . | . | . | . | . | . | . | . |
| 21-225_20H10_LC | . | O | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | T | . | . | . | . | . | . | . | . | . | . | . |
| 21-225_21F3_LC | . | . | . | . | . | . | . | . | . | . | . | . | . | . | . | . | . | . | . | . | . | . | . | . | . | . |
| 21-225_21G7_LC | Q | . | . | . | . | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | V | . | T | . | . | . | . | . | . |
| 21-225_224A1_LC | . | . | . | . | . | . | . | . | . | . | . | . | . | . | . | . | . | . | . | . | . | . | . | . | . | . |
| 21-225_224B1_LC | . | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | T | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | T | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | . | . |
| 21-225_22D12_LC | . | . | . | . | . | . | . | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . | . | . | . | . | . | N | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | V | . | . | . | . | . | . | . | . |
| 21-225_23A3_LC | . | . | . | . | . | . | . | . | . | . | . | . | . | . | . | . | . | V | . | . | . | . | . | . | . | . |
| 21-225_23C8_LC | . | . | . | N | . | G | . | . | . | . | . | . | . | . | . | . | . | V | . | . | . | . | . | . | . | . |
| 21-225_23D1_LC | . | . | . | . | . | . | . | . | . | . | . | . | . | . | . | . | . | . | . | . | . | . | . | . | . | . |
| 21-225_23H11_LC | . | . | . | . | . | . | . | . | . | . | . | . | . | . | . | . | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | . | . | . | . | . | . | . |
| 21-225_25H10_LC | . | . | . | . | . | . | . | . | . | . | . | . | . | . | P | . | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | V | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | Q | . | . | . | S | . | . | . | . | . | . | . | . | . | . | . | . | . | . | . | . | . | . | . | . | . |
| 21-225_2B2_LC | . | . | . | . | . | . | . | . | . | . | . | . | . | . | . | . | . | . | . | . | . | . | . | . | . | . |
| 21-225_2G9_LC | Q | . | . | . | . | . | . | . | . | . | . | . | . | . | . | . | . | . | . | . | . | . | . | . | . | . |
| 21-225_30D1_LC | S | . | . | . | . | . | . | . | . | . | . | . | . | . | . | . | . | . | . | . | . | . | . | . | . | . |
| 21-225_30E3_LC | R | . | . | . | . | . | . | . | . | . | . | . | . | . | F | . | . | . | . | . | . | . | . | . | . | . |
| 21-225_30E9_LC | R | . | . | . | . | G | . | . | . | . | . | . | . | . | F | . | . | . | . | . | . | . | . | . | . | . |
| 21-225_30F3_LC | . | . | . | . | . | T | . | . | . | . | . | . | . | . | . | . | . | . | . | . | . | . | . | . | . | . |
| 21-225_30G11_LC | . | . | . | . | . | . | . | . | . | . | . | . | . | . | . | . | . | . | . | . | . | . | . | . | . | . |
| 21-225_31A8_LC | . | . | . | . | . | . | . | . | . | . | . | . | . | . | V | . | . | . | . | . | . | . | . | . | . | . |
| 21-225_31B8_LC | . | . | . | . | . | G | . | . | . | . | . | . | . | . | . | . | . | . | . | . | . | . | . | . | . | . |
| 21-225_31H3_LC | R | . | . | . | . | . | . | . | . | . | . | . | . | . | F | . | . | . | . | . | . | . | . | . | . | . |
| 21-225_31H5_LC | . | I | . | . | . | . | . | . | . | . | . | . | . | . | . | . | . | . | . | . | . | . | . | . | . | . |
| 21-225_32G1_LC | . | . | . | . | . | . | . | . | . | . | . | . | . | . | . | . | . | . | . | . | . | . | . | . | . | . |
| 21-225_32G12_LC | . | . | . | . | . | . | . | . | . | . | . | . | . | . | . | . | . | . | . | . | . | . | . | . | . | . |
| 21-225_33A4_LC | R | . | . | . | . | . | . | . | . | . | . | . | . | . | . | . | . | . | . | R | . | . | . | . | . | . |
| 21-225_33A7_LC | . | . | . | . | . | V | . | . | . | . | . | . | . | . | . | . | . | . | . | . | . | . | . | . | . | . |
| 21-225_33B1_LC | . | . | . | . | . | T | . | . | . | . | . | . | . | . | . | . | . | . | . | . | . | . | . | . | . | . |
| 21-225_33B8_LC | . | . | . | . | . | . | . | . | . | . | . | . | . | . | . | . | . | . | . | . | . | . | . | . | . | . |
| 21-225_33F1_LC | . | . | . | . | . | . | . | . | . | . | . | . | . | . | . | . | . | . | . | . | . | . | . | . | . | . |
| 21-225_33G7_LC | . | . | . | . | . | . | . | . | . | . | . | . | . | . | . | . | . | . | . | . | . | . | . | . | . | . |
| 21-225_34A6_LC | . | . | . | . | F | . | . | . | . | . | . | . | . | . | . | . | . | . | . | . | . | . | . | . | . | . |
| 21-225_34D11_LC | . | . | . | . | . | . | . | . | . | . | . | . | . | . | . | . | . | . | . | . | . | . | . | . | . | . |
| 21-225_34G8_LC | . | . | . | . | . | . | . | . | . | . | . | . | . | . | . | . | . | . | . | . | . | . | . | . | . | . |
| 21-225_34G9_LC | . | . | . | . | . | . | . | . | . | . | . | . | . | . | . | . | . | . | . | . | . | . | . | . | . | . |
| 21-225_34H11_LC | . | . | . | . | . | . | . | . | . | . | . | . | . | T | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_35A6_LC | . | . | . | . | . | . | . | . | . | . | . | . | . | . | T | . | . | . | . | . | . | . | . | . | . | . |
| 21-225_35B7_LC | S | . | . | . | . | . | . | . | . | . | . | . | . | . | . | . | . | . | . | . | . | . | . | . | . | . |
| 21-225_35D1_LC | . | . | . | . | . | . | . | . | . | . | . | . | . | . | . | . | . | . | . | . | . | . | . | . | . | . |
| 21-225_35F11_LC | . | . | . | . | . | . | . | . | . | . | . | . | . | . | . | . | . | . | . | . | . | . | . | . | . | . |
| 21-225_36A5_LC | . | . | . | . | S | . | . | . | . | . | . | . | . | . | . | . | . | . | . | . | . | . | . | . | . | . |
| 21-225_36C5_LC | . | . | . | . | . | . | . | . | . | . | . | . | . | . | . | . | . | . | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | . | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . | G | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | . | . | . | . | . | . | . | . | . | . | . | . | . | . | . |
| 21-225_46F3_LC | . | . | . | . | . | . | . | . | . | . | . | . | . | . | . | . | . | . | . | . | . | . | . | . | . | . |
| 21-225_47A1_LC | . | . | . | . | . | . | . | . | . | . | . | . | . | . | S | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | T | . | . | . | . | . | . | . | . | . | I |
| 21-225_48H4_LC | . | . | . | . | . | . | . | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . | . | . | S | . | . | . | . |
| 21-225_4C8_LC | . | . | . | . | . | . | . | . | . | . | . | . | . | . | . | . | . | . | . | . | . | . | . | . | . | . |
| 21-225_4E10_LC | . | . | . | . | . | . | . | . | . | . | . | . | . | . | . | . | . | . | . | . | . | . | . | . | . | . |
| 21-225_4F12_LC | . | . | . | . | . | . | . | . | . | . | . | . | . | . | . | . | . | . | . | . | . | . | . | . | . | . |
| 21-225_4F4_LC | . | . | . | . | . | . | . | . | . | . | . | . | . | . | . | . | . | V | . | T | . | . | . | . | . | . |
| 21-225_4H4_LC | . | . | . | . | . | . | . | . | . | . | . | . | . | . | . | . | V | . | . | . | . | . | . | . | . | . |
| 21-225_50G10_LC | . | . | . | . | . | . | . | . | . | . | . | . | . | . | . | . | . | . | . | . | . | . | . | . | . | . |
| 21-225_51F11_LC | . | . | . | . | . | . | . | . | . | . | . | . | . | . | . | . | . | . | . | . | . | . | . | . | . | . |
| 21-225_52B2_LC | S | . | . | . | . | . | . | . | . | . | . | . | . | . | . | S | . | . | . | . | . | . | . | . | T | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | N | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_54C6_LC | . | . | . | . | . | . | . | . | . | . | . | . | . | . | . | . | N | . | . | . | . | . | . | . | . | . |
| 21-225_55B8_LC | . | . | . | . | H | . | . | . | . | . | . | . | . | . | . | F | . | . | . | . | . | . | . | . | . | . |
| 21-225_55H6_LC | . | . | . | . | G | . | . | . | . | . | . | . | . | . | . | . | . | . | . | . | . | . | . | . | . | . |
| 21-225_56F1_LC | . | . | . | . | N | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | . | . | . | . | . | . | . | . | . | . | . |
| 21-225_5H7_LC | . | . | . | . | . | V | . | . | . | . | . | . | . | . | T | . | . | . | . | G | . | . | . | . | . | . |
| 21-225_60E8_LC | . | . | . | . | . | . | . | . | . | . | . | . | . | . | . | . | . | . | . | . | . | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | F | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | . | . | . | . | . | . | . | . | . | . | . | . | . | . |
| 21-225_6D4_LC | . | . | . | . | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | T | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | . | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | T | . | . | . | . | . | . | . | . | . |
| 21-225_75H7_LC | Q | . | . | . | . | D | . | . | . | . | . | . | . | . | . | . | . | . | . | . | . | . | . | . | . | . |
| 21-225_7C4_LC | . | . | . | . | . | . | . | . | . | . | . | . | . | . | . | . | . | . | . | . | . | . | . | . | . | . |
| 21-225_7E9_LC | . | . | . | . | T | . | . | . | . | . | . | . | . | . | . | . | . | . | . | . | . | . | . | . | . | . |
| 21-225_7F4_LC | . | . | . | . | . | . | . | . | . | . | . | . | . | . | . | . | N | . | . | . | . | . | . | . | . | . |
| 21-225_7H11_LC | . | . | I | . | . | V | . | . | . | . | . | . | . | . | C | . | F | . | . | . | . | . | . | . | . | . |
| 21-225_7H7_LC | . | . | . | . | . | . | . | . | . | . | . | . | . | . | . | . | . | . | . | . | . | . | T | . | . | . |

4227

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_8C9_LC | . | . | . | . | . | . | . | . | . | . | . | . | . | . | . | . | . | . | . | . | . | . | . | . | . | . |
| 21-225_8D12_LC | . | . | . | . | . | . | . | . | . | . | . | . | . | . | . | . | . | . | . | . | . | . | . | . | . | . |
| 21-225_8F11_LC | . | . | . | . | F | Q | . | . | . | . | . | . | . | . | . | . | . | V | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | T | . | . | . | . | . | . | . | . | . |
| 21-225_9E8_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . | . | . | . | . | . | . | . |
| 21-225_11F10_LC | . | . | . | . | . | . | . | . | . | . | . | . | . | . | V | . | . | . | . | . | . | . | . | . | . | . |
| 21-225_11F5_LC | . | . | . | . | . | . | . | . | . | . | . | . | . | . | . | . | . | . | . | . | . | . | . | . | I | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | . | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | I | . | . | . | . | . | . | . | . | . | . | . | . | . | . | . |
| 21-225_146F1_LC | . | . | G | . | . | . | . | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | T | . | . | . | . | . | . | . | . | . | F | . | . | . | . | . | . | V | . | . | . | . | . | . | . | . |
| 21-225_148F12_LC | . | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | . | . | . | . | . | . | . | . | I | . |
| 21-225_149G1_LC | . | . | . | . | . | . | . | . | . | . | . | . | . | . | . | . | . | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | . | . | . | . | . | . | . | . | . | . | . | . | . | . | . | . | . | . | . | . | . | . |
| 21-225_14B2_LC | . | . | . | . | . | . | . | . | . | . | . | . | . | . | . | . | . | . | . | . | . | . | . | . | . | . |
| 21-225_14C2_LC | . | . | . | . | . | . | . | . | . | . | . | . | . | . | . | . | . | V | . | R | . | . | . | . | . | . |
| 21-225_14E3_LC | I | . | . | G | . | . | . | . | . | . | . | . | . | . | . | . | . | . | . | R | . | . | . | . | A | . |
| 21-225_150E7_LC | . | . | . | . | . | . | . | . | O | . | . | . | . | . | . | . | . | . | . | . | . | . | . | . | A | . |
| 21-225_152H9_LC | T | . | . | . | . | . | . | . | . | . | . | . | F | . | . | . | . | . | . | . | . | . | . | . | . | . |
| 21-225_156G1_LC | . | . | . | . | . | . | . | . | . | . | . | . | F | . | . | . | . | . | . | . | . | . | . | . | . | . |
| 21-225_158H5_LC | . | . | . | C | . | . | . | . | . | . | . | . | . | . | . | . | . | V | . | R | . | . | . | . | S | . |
| 21-225_15A1_LC | . | . | . | . | . | . | . | . | . | . | . | . | . | . | V | . | . | . | . | . | . | . | . | . | S | . |
| 21-225_15C9_LC | . | . | . | . | . | . | . | . | . | . | . | . | . | . | V | . | . | . | . | . | . | . | . | . | . | . |
| 21-225_15E3_LC | . | . | I | . | . | . | . | . | . | . | . | . | . | . | . | . | . | . | . | . | . | . | L | . | A | . |
| 21-225_15G7_LC | . | . | . | F | . | . | . | . | . | . | . | . | . | . | . | . | . | . | . | . | . | . | . | . | . | . |

4229

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_15H1_LC | N | . | . | . | . | . | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | S | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | . | . | . | . | . | . | . | . | . | . | N | . | . | . | . | . | . | . | . | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | . | . | . | . | . | . | . | . | . | . | G | . | . | . | . | . | . | . | . | . | . |
| 21-225_169F9_LC | . | . | . | . | . | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | . | . | . | . | . | . | . | . | . | . | I | . | . | . | . | V | . | . | . | . | . | . | . | . |
| 21-225_170D11_LC | . | . | . | . | . | . | . | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . | . | . | . | . | . | . | . |
| 21-225_170G4_LC | . | . | . | . | . | . | . | . | . | . | . | . | . | . | . | . | . | . | . | . | . | . | . | . | . | . |
| 21-225_170G5_LC | . | . | . | . | . | . | . | . | . | . | . | . | . | . | . | . | . | . | . | . | . | . | . | . | . | . |
| 21-225_171A4_LC | . | . | . | . | . | . | . | . | . | . | . | . | . | . | . | . | . | . | . | . | . | . | . | . | . | . |
| 21-225_171C3_LC | . | . | . | . | . | . | . | . | . | . | . | . | . | . | . | . | . | . | . | . | . | . | . | . | . | . |
| 21-225_172B3_LC | . | . | . | . | . | . | . | . | . | . | . | . | . | . | . | G | . | . | . | . | . | . | . | . | . | . |
| 21-225_172G8_LC | . | . | . | . | . | . | . | . | . | . | . | . | . | . | . | . | . | . | . | . | . | . | . | . | . | . |
| 21-225_173H12_LC | . | . | . | . | . | . | . | . | . | . | . | . | . | . | . | . | . | . | . | . | . | . | . | . | S | . |
| 21-225_175G1_LC | . | . | . | . | . | . | . | . | . | . | . | . | . | . | . | . | . | . | . | . | . | . | . | . | . | . |
| 21-225_176B11_LC | . | . | . | . | . | . | . | . | . | . | . | . | . | . | L | . | . | . | . | . | . | . | . | . | . | . |
| 21-225_178B10_LC | . | . | . | . | . | . | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | S | S | . | . | . | . | . | . | . | . | . | . | . | . | . | . | . |
| 21-225_17F9_LC | . | . | . | . | . | . | . | . | . | . | . | . | . | . | . | . | N | . | . | . | . | . | . | . | . | . |
| 21-225_17H12_LC | . | . | I | . | . | . | . | . | . | . | . | . | . | . | . | . | T | G | . | . | . | . | . | . | . | . |
| 21-225_17H8_LC | . | . | . | . | . | . | . | . | . | . | . | . | . | . | . | . | . | . | . | . | . | . | . | T | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | E | F | T | L | T | I | S | S | L | Q | P | E | D | F | A | T | Y |
| 21-225_18A1_LC | . | . | . | . | . | . | . | . | . | . | . | . | . | . | . | . | . | . | . | . | . | . | . | . | . | . |
| 21-225_18A3_LC | N | . | . | . | . | . | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | M | . |
| 21-225_193A5_LC | . | . | . | . | S | . | . | . | . | . | . | . | I | . | . | . | . | V | . | . | . | . | . | . | M | . |
| 21-225_193E7_LC | . | . | . | . | . | . | . | . | . | . | . | . | . | . | . | . | . | . | . | . | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | . | . | . | . | . | . | . | . | . | . | . | . | . | . | . | . | . | . | . | . | . | . | . |
| 21-225_19D11_LC | . | . | . | . | . | . | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | . | . | . | . | . | . | . | . | . | . | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | I | . |
| 21-225_200F12_LC | . | . | . | . | . | . | . | . | . | . | . | . | . | . | . | . | . | . | . | . | . | . | . | . | . | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | . | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | . | . | . |
| 21-225_204H8_LC | . | . | S | . | . | . | . | . | . | . | . | . | . | . | . | V | . | . | . | . | . | . | . | . | . | . |
| 21-225_205H8_LC | . | . | . | . | . | . | . | . | . | . | . | . | . | . | . | . | . | . | . | . | . | . | S | . | A | . |
| 21-225_20A10_LC | . | . | . | . | . | . | . | . | . | . | . | . | . | . | . | . | . | . | . | . | . | . | . | . | . | . |
| 21-225_20A7_LC | . | . | . | . | . | . | . | . | . | . | S | . | . | . | . | . | . | . | . | . | . | . | . | . | . | . |
| 21-225_20C2_LC | . | . | . | . | . | . | . | . | . | . | . | . | . | . | . | . | . | . | . | . | . | . | . | . | . | . |
| 21-225_20C8_LC | . | . | . | . | . | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | S | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_20G9_LC | . | . | . | . | . | . | . | . | . | . | . | . | . | . | . | . | . | . | . | . | . | . | . | . | . | . |
| 21-225_20H10_LC | . | . | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | . | . | . | . | O | . | I | . | . | . | . | . | . | . | . | . | . | . | . | . | . |
| 21-225_21G7_LC | N | . | . | . | . | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | A | . | . | . | L | . | I | . |
| 21-225_224A1_LC | . | . | . | . | . | . | . | . | . | . | . | . | . | . | . | . | . | . | . | . | . | . | . | . | . | . |
| 21-225_224B1_LC | . | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | . | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | . | . |
| 21-225_22D12_LC | . | . | . | . | . | . | . | . | . | O | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | S | . | . | . | . | . | . | . | . | . | . | . | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | L | . |
| 21-225_23A3_LC | . | . | . | . | . | . | . | . | . | . | . | . | . | . | . | . | . | . | . | . | . | . | . | . | I | . |
| 21-225_23C8_LC | . | . | . | . | . | . | . | . | . | . | . | . | . | . | . | . | . | . | . | . | . | . | . | V | I | . |
| 21-225_23D1_LC | . | . | . | . | . | . | . | . | . | . | . | . | . | . | . | V | . | . | . | . | . | . | . | . | . | . |
| 21-225_23H11_LC | . | . | . | . | . | . | . | . | . | . | . | . | . | . | . | . | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | R | . | . | . | . | . | . | . | . | . | . | . | . | . | . | N | . | . | . | . | . | . | . | . | . | . |
| 21-225_25H10_LC | . | . | . | . | . | . | . | . | . | . | . | . | . | . | . | . | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_29B8_LC | . | . | . | X | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | . | . | . | . | . | . | . | . | . | Q | . | . | . | . | . | . | . | . | . | . | . | . | . | . | . | . |
| 21-225_2B2_LC | . | . | . | . | . | . | . | . | . | . | . | . | . | . | . | . | . | . | . | . | . | . | . | L | . | . |
| 21-225_2G9_LC | . | . | . | F | . | . | . | . | . | . | . | . | . | . | . | . | . | . | L | . | . | . | . | . | . | . |
| 21-225_30D1_LC | . | . | . | . | . | . | . | . | . | . | . | . | . | . | . | . | . | . | . | . | . | . | . | . | T | . |
| 21-225_30E3_LC | . | . | . | . | . | . | . | . | . | . | . | . | . | . | . | . | . | . | . | . | . | . | . | . | . | . |
| 21-225_30E9_LC | . | . | . | . | . | . | . | . | . | . | . | . | . | . | . | . | . | . | . | . | . | . | . | . | . | . |
| 21-225_30F3_LC | . | . | . | . | . | . | . | . | . | Q | . | . | . | . | . | . | . | . | . | . | . | . | . | . | S | . |
| 21-225_30G11_LC | . | . | . | . | . | . | . | . | . | . | . | . | . | . | . | Q | . | . | . | . | . | . | . | . | . | . |
| 21-225_31A8_LC | . | . | . | . | . | . | . | . | . | . | . | . | . | . | . | . | . | . | . | . | . | . | . | . | . | . |
| 21-225_31B8_LC | . | . | . | . | . | . | . | . | . | . | . | . | . | . | . | . | . | . | . | . | . | . | . | . | . | . |
| 21-225_31H3_LC | . | . | . | . | . | . | . | . | . | . | . | . | . | . | . | . | . | . | . | . | . | . | . | . | . | . |
| 21-225_31H5_LC | . | V | . | . | . | . | . | . | . | . | . | F | . | . | . | . | . | M | . | . | D | . | . | S | N | . |
| 21-225_32G1_LC | . | . | . | . | . | . | . | . | . | . | . | . | . | . | . | . | . | . | . | . | . | . | . | . | . | . |
| 21-225_32G12_LC | . | . | . | . | . | . | . | . | . | . | . | . | . | . | . | . | . | . | . | . | . | . | . | . | . | . |
| 21-225_33A4_LC | . | . | . | . | . | . | . | . | . | . | . | . | . | . | . | . | . | . | . | . | . | . | . | . | X | . |
| 21-225_33A7_LC | . | . | . | . | . | . | . | . | . | . | . | . | . | . | . | . | . | . | . | . | . | . | . | . | A | . |
| 21-225_33B1_LC | . | . | . | . | . | . | . | . | . | . | . | . | . | . | . | . | . | . | . | . | . | . | . | . | . | . |
| 21-225_33B8_LC | . | . | . | . | . | . | . | . | . | . | . | . | . | . | . | . | . | . | . | . | . | . | . | . | . | . |
| 21-225_33F1_LC | . | . | . | . | . | . | . | . | . | . | . | . | . | . | . | . | . | . | . | . | . | . | . | . | I | . |
| 21-225_33G7_LC | . | . | . | . | . | . | . | . | . | . | . | . | . | . | . | . | . | . | . | . | . | . | . | . | . | . |
| 21-225_34A6_LC | . | . | . | . | . | . | . | . | . | . | . | . | . | . | V | . | . | . | . | . | . | . | . | . | . | . |
| 21-225_34D11_LC | . | . | . | . | . | . | . | . | . | . | . | . | . | . | V | I | . | . | . | . | . | . | . | . | . | . |
| 21-225_34G8_LC | . | . | . | . | . | . | . | . | . | . | . | . | . | . | . | . | . | . | . | . | . | . | . | . | . | . |
| 21-225_34G9_LC | T | . | . | . | . | . | . | . | . | . | . | . | . | . | . | . | . | . | . | . | . | A | . | . | I | . |
| 21-225_34H11_LC | . | . | . | . | . | . | . | . | . | . | . | . | . | . | . | . | . | . | . | . | . | . | . | . | . | . |

4233

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_35A6_LC | . | . | . | . | . | . | . | . | . | . | . | . | . | . | . | . | . | . | . | . | . | . | . | . | . | . |
| 21-225_35B7_LC | . | . | . | . | . | . | . | . | . | . | . | . | . | . | . | . | . | . | . | . | . | . | . | . | . | . |
| 21-225_35D1_LC | . | . | . | . | . | . | . | . | . | . | . | . | . | . | . | . | . | . | . | . | . | . | . | . | . | . |
| 21-225_35F11_LC | . | . | . | . | . | . | . | . | . | . | . | . | . | . | . | . | . | . | . | . | . | . | . | . | . | . |
| 21-225_36A5_LC | . | . | . | . | . | . | . | . | . | . | . | . | . | . | . | . | . | . | . | . | . | . | . | . | . | . |
| 21-225_36C5_LC | Y | . | . | . | . | . | . | . | . | . | . | . | . | . | . | . | . | V | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | G | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . | . | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | S | . | . | . | . | . | . | . | . | . | . | . | . | . | . |
| 21-225_46F3_LC | . | . | . | . | . | . | . | . | . | . | . | S | . | . | . | . | . | . | . | . | . | . | . | . | . | . |
| 21-225_47A1_LC | . | . | . | R | . | . | . | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . | . | . | . | . | . | . | . |
| 21-225_48H4_LC | . | . | . | . | . | . | . | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . | . | . | . | . | . | . | . |
| 21-225_4C8_LC | . | . | . | V | . | . | . | . | . | . | . | . | . | . | . | . | . | . | . | . | . | . | . | . | . | . |
| 21-225_4E10_LC | . | . | . | . | P | . | . | . | . | . | . | . | . | . | . | . | . | . | . | L | . | . | . | . | . | . |
| 21-225_4F12_LC | . | . | G | . | . | . | . | . | . | I | . | . | . | . | . | . | . | . | . | . | . | . | . | . | . | . |
| 21-225_4F4_LC | . | . | . | . | . | . | . | . | . | . | . | . | . | . | . | . | . | . | . | . | . | . | . | . | . | I |
| 21-225_4H4_LC | . | . | . | . | . | . | . | . | . | . | . | . | . | . | . | . | . | . | . | . | . | . | . | . | . | I |
| 21-225_50G10_LC | . | . | . | . | . | . | . | . | . | . | . | . | . | . | . | . | . | . | . | . | . | . | . | . | . | . |
| 21-225_51F11_LC | . | . | . | . | . | . | . | . | . | . | . | . | . | . | . | . | . | . | . | . | . | . | . | . | . | . |
| 21-225_52B2_LC | . | . | . | . | . | . | . | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | E | F | T | L | T | I | S | S | L | Q | P | E | D | F | A | T | Y |
| 21-225_54C6_LC | . | . | . | . | . | . | . | . | . | . | . | . | . | . | . | . | . | . | . | . | . | . | . | . | . | . |
| 21-225_55B8_LC | . | . | . | . | . | . | . | . | . | . | . | . | . | . | . | . | . | . | . | . | . | . | . | . | . | . |
| 21-225_55H6_LC | . | . | . | . | . | . | . | . | . | . | . | . | . | . | . | . | . | . | . | . | . | . | . | . | A | . |
| 21-225_56F1_LC | . | . | . | . | . | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | S | . | . | . | . | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | . | . | . | . | . | . | . | . | . | . | . | . | . | . | . | . | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | R | . | . | . | . | . | . | . | . | . | . | . | . | . | . | . | . | . | . | L | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | P | . | . | . | . | S | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | . | . | . | . | . | . | . | . | . | . | . | . | . | . |
| 21-225_6D4_LC | . | . | . | . | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | . | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | F | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | G | . | . | . | . | . | . | . | . | . |
| 21-225_75H7_LC | . | . | . | . | . | . | . | . | . | . | . | . | . | . | . | . | . | V | . | . | . | . | . | G | V | . |
| 21-225_7C4_LC | . | . | . | . | . | . | . | . | . | . | . | . | . | . | . | . | . | . | . | . | . | . | . | . | . | . |
| 21-225_7E9_LC | . | . | . | . | . | . | . | . | . | . | . | . | . | . | . | . | . | . | . | . | . | . | . | . | . | . |
| 21-225_7F4_LC | . | . | . | . | . | . | . | . | . | . | . | . | . | . | . | . | . | . | . | . | . | . | . | . | . | . |
| 21-225_7H11_LC | . | . | . | Y | . | . | . | . | . | . | . | . | . | . | . | I | M | . | . | . | . | . | . | . | . | . |
| 21-225_7H7_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_8C9_LC | . | . | . | . | . | . | . | . | . | . | . | . | . | . | . | . | . | . | . | T | . | . | . | . | . | . |
| 21-225_8D12_LC | N | . | . | . | . | . | . | . | . | . | . | . | . | . | . | . | . | . | . | . | . | . | . | . | . | . |
| 21-225_8F11_LC | . | . | . | . | . | . | . | . | . | . | . | . | . | . | . | . | . | . | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | . | . | . | . | . | . | . | . | . | . |
| 21-225_9E8_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | K_CDR3 | | | | | | | |
| CONSENSUS | Y | C | L | Q | H | N | S | - | - | - | - | - | - | - | - | - | - | - | - | - | - | - | - | - | - | - |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . | . | . | . | . | . | . | . |
| 21-225_11F10_LC | . | . | . | . | . | S | . | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . | . | . | . | . | . | . | . |
| 21-225_11G7_LC | . | . | . | . | . | S | N | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | V | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | . | . | . | . | . | . | . |
| 21-225_146F1_LC | . | . | . | . | . | S | T | . | . | . | . | . | . | . | . | . | . | . | . | . | . | . | . | . | . | . |
| 21-225_146G11_LC | . | . | . | . | . | O | . | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | . | . | . | N | . | . | . | . | . | . | . | . | . | . | . | . | . | . | . | . | . | . | . |
| 21-225_148F12_LC | F | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | . | . | . | . | . | . | . | . | . | . |
| 21-225_149G1_LC | . | . | . | . | . | . | . | . | . | . | . | . | . | . | . | . | . | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | . | Y | N | . | . | . | . | . | . | . | . | . | . | . | . | . | . | . | . | . | . | . |
| 21-225_14B2_LC | . | . | . | . | . | . | . | . | . | . | . | . | . | . | . | . | . | . | . | . | . | . | . | . | . | . |
| 21-225_14C2_LC | . | . | V | . | . | . | . | . | . | . | . | . | . | . | . | . | . | . | . | . | . | . | . | . | . | . |
| 21-225_14E3_LC | . | S | V | . | . | Y | . | . | . | . | . | . | . | . | . | . | . | . | . | . | . | . | . | . | . | . |
| 21-225_150E7_LC | . | . | . | . | . | . | . | . | . | . | . | . | . | . | . | . | . | . | . | . | . | . | . | . | . | . |
| 21-225_152H9_LC | . | . | . | . | . | S | N | . | . | . | . | . | . | . | . | . | . | . | . | . | . | . | . | . | . | . |
| 21-225_156G1_LC | . | . | . | . | . | S | N | . | . | . | . | . | . | . | . | . | . | . | . | . | . | . | . | . | . | . |
| 21-225_158H5_LC | . | . | V | . | . | . | . | . | . | . | . | . | . | . | . | . | . | . | . | . | . | . | . | . | . | . |
| 21-225_15A1_LC | . | . | . | . | N | S | . | . | . | . | . | . | . | . | . | . | . | . | . | . | . | . | . | . | . | . |
| 21-225_15C9_LC | . | . | . | . | . | T | . | . | . | . | . | . | . | . | . | . | . | . | . | . | . | . | . | . | . | . |
| 21-225_15E3_LC | . | . | . | . | . | . | . | . | . | . | . | . | . | . | . | . | . | . | . | . | . | . | . | . | . | . |
| 21-225_15G7_LC | . | . | . | . | . | S | N | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_15H1_LC | . | . | . | . | . | S | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | S | . | . | . | . | . | . | . | . | . | . | . | . | . | . | . | . | . | . | . | . |
| 21-225_160H3_LC | . | . | . | . | . | S | N | . | . | . | . | . | . | . | . | . | . | . | . | . | . | . | . | . | . | . |
| 21-225_169B1_LC | . | . | . | . | . | Y | . | . | . | . | . | . | . | . | . | . | . | . | . | . | . | . | . | . | . | . |
| 21-225_169F9_LC | . | . | . | . | . | Y | . | . | . | . | . | . | . | . | . | . | . | . | . | . | . | . | . | . | . | . |
| 21-225_16A1_LC | . | . | . | H | . | S | N | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | I | . | . | . | . | . | . | . | . | . | . | . | . | . | . | . | . | . | . | . | . | . | . | . |
| 21-225_170D11_LC | . | . | . | . | . | Y | . | . | . | . | . | . | . | . | . | . | . | . | . | . | . | . | . | . | . | . |
| 21-225_170D5_LC | . | . | . | . | . | Y | . | . | . | . | . | . | . | . | . | . | . | . | . | . | . | . | . | . | . | . |
| 21-225_170G4_LC | . | . | . | . | . | Y | . | . | . | . | . | . | . | . | . | . | . | . | . | . | . | . | . | . | . | . |
| 21-225_170G5_LC | . | . | . | . | . | Y | . | . | . | . | . | . | . | . | . | . | . | . | . | . | . | . | . | . | . | . |
| 21-225_171A4_LC | . | . | . | . | . | Y | . | . | . | . | . | . | . | . | . | . | . | . | . | . | . | . | . | . | . | . |
| 21-225_171C3_LC | . | . | . | . | . | Y | . | . | . | . | . | . | . | . | . | . | . | . | . | . | . | . | . | . | . | . |
| 21-225_172B3_LC | . | . | . | . | . | Y | . | . | . | . | . | . | . | . | . | . | . | . | . | . | . | . | . | . | . | . |
| 21-225_172G8_LC | . | . | . | H | . | Y | . | . | . | . | . | . | . | . | . | . | . | . | . | . | . | . | . | . | . | . |
| 21-225_173H12_LC | . | . | . | . | . | Y | . | . | . | . | . | . | . | . | . | . | . | . | . | . | . | . | . | . | . | . |
| 21-225_175G1_LC | . | . | . | . | . | Y | . | . | . | . | . | . | . | . | . | . | . | . | . | . | . | . | . | . | . | . |
| 21-225_176B11_LC | . | . | . | . | . | Y | . | . | . | . | . | . | . | . | . | . | . | . | . | . | . | . | . | . | . | . |
| 21-225_178B10_LC | . | . | . | H | . | Y | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . | . | . | . | . | . | . | . |
| 21-225_17F9_LC | . | . | . | . | . | . | T | . | . | . | . | . | . | . | . | . | . | . | . | . | . | . | . | . | . | . |
| 21-225_17H12_LC | . | . | . | . | . | Y | . | . | . | . | . | . | . | . | . | . | . | . | . | . | . | . | . | . | . | . |
| 21-225_17H8_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_20G9_LC | . | . | . | . | . | S | T | . | . | . | . | . | . | . | . | . | . | . | . | . | . | . | . | . | . | . |
| 21-225_20H10_LC | . | . | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | S | . | . | . | . | . | . | . | . | . | . | . | . | . | . | . | . | . | . | . | . |
| 21-225_21G7_LC | . | . | . | . | . | S | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | . | . | . | . | . | . | . | . |
| 21-225_224A1_LC | . | . | . | . | . | . | . | . | . | . | . | . | . | . | . | . | . | . | . | . | . | . | . | . | . | . |
| 21-225_224B1_LC | H | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | . | T | . | . | . | . | . | . | . | . | . | . | . | . | . | . | . | . | . | . | . |
| 21-225_22C1_LC | . | . | . | . | . | S | T | . | . | . | . | . | . | . | . | . | . | . | . | . | . | . | . | . | . | . |
| 21-225_22D12_LC | . | . | . | . | . | S | T | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . | . | . | . | . | . | . | . |
| 21-225_22G9_LC | . | . | . | . | . | A | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | . | . |
| 21-225_23A3_LC | F | . | . | . | . | . | . | . | . | . | . | . | . | . | . | . | . | . | . | . | . | . | . | . | . | . |
| 21-225_23C8_LC | . | . | . | . | . | . | . | . | . | . | . | . | . | . | . | . | . | . | . | . | . | . | . | . | . | . |
| 21-225_23D1_LC | . | . | . | . | . | S | . | . | . | . | . | . | . | . | . | . | . | . | . | . | . | . | . | . | . | . |
| 21-225_23H11_LC | . | . | . | . | . | . | R | . | . | . | . | . | . | . | . | . | . | . | . | . | . | . | . | . | . | . |
| 21-225_24E5_LC | . | . | . | . | . | S | N | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | . | . | . | . | . | . | . |
| 21-225_25H10_LC | . | . | . | . | . | . | . | . | . | . | . | . | . | . | . | . | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | R | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | N | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | . | . | . | . | . | . | . | . | . | . | . | . | . | . | . | . | . | . | . | . | . | . | . | . | . | . |
| 21-225_2B2_LC | . | . | . | . | . | . | . | . | . | . | . | . | . | . | . | . | . | . | . | . | . | . | . | . | . | . |
| 21-225_2G9_LC | . | . | . | . | . | . | . | . | . | . | . | . | . | . | . | . | . | . | . | . | . | . | . | . | . | . |
| 21-225_30D1_LC | . | . | . | . | . | T | } | . | . | . | . | . | . | . | . | . | . | . | . | . | . | . | . | . | . | . |
| 21-225_30E3_LC | . | . | . | . | . | T | . | . | . | . | . | . | . | . | . | . | . | . | . | . | . | . | . | . | . | . |
| 21-225_30E9_LC | . | . | . | . | . | T | . | . | . | . | . | . | . | . | . | . | . | . | . | . | . | . | . | . | . | . |
| 21-225_30F3_LC | . | . | . | . | . | . | . | . | . | . | . | . | . | . | . | . | . | . | . | . | . | . | . | . | . | . |
| 21-225_30G11_LC | . | . | . | . | . | T | } | . | . | . | . | . | . | . | . | . | . | . | . | . | . | . | . | . | . | . |
| 21-225_31A8_LC | . | . | . | . | . | T | } | . | . | . | . | . | . | . | . | . | . | . | . | . | . | . | . | . | . | . |
| 21-225_31B8_LC | . | . | . | . | . | } | } | . | . | . | . | . | . | . | . | . | . | . | . | . | . | . | . | . | . | . |
| 21-225_31H3_LC | . | . | . | . | . | T | . | . | . | . | . | . | . | . | . | . | . | . | . | . | . | . | . | . | . | . |
| 21-225_31H5_LC | . | } | . | . | . | } | } | . | . | . | . | . | . | . | . | . | . | . | . | . | . | . | . | . | . | . |
| 21-225_32G1_LC | . | . | . | . | . | T | } | . | . | . | . | . | . | . | . | . | . | . | . | . | . | . | . | . | . | . |
| 21-225_32G12_LC | . | . | . | . | . | T | } | . | . | . | . | . | . | . | . | . | . | . | . | . | . | . | . | . | . | . |
| 21-225_33A4_LC | . | . | . | . | . | Y | . | . | . | . | . | . | . | . | . | . | . | . | . | . | . | . | . | . | . | . |
| 21-225_33A7_LC | . | . | . | . | . | T | } | . | . | . | . | . | . | . | . | . | . | . | . | . | . | . | . | . | . | . |
| 21-225_33B1_LC | . | . | . | . | . | . | . | . | . | . | . | . | . | . | . | . | . | . | . | . | . | . | . | . | . | . |
| 21-225_33B8_LC | . | . | . | . | . | T | } | . | . | . | . | . | . | . | . | . | . | . | . | . | . | . | . | . | . | . |
| 21-225_33F1_LC | . | . | . | . | . | T | } | . | . | . | . | . | . | . | . | . | . | . | . | . | . | . | . | . | . | . |
| 21-225_33G7_LC | . | . | . | . | . | Y | . | . | . | . | . | . | . | . | . | . | . | . | . | . | . | . | . | . | . | . |
| 21-225_34A6_LC | . | . | . | . | . | . | . | . | . | . | . | . | . | . | . | . | . | . | . | . | . | . | . | . | . | . |
| 21-225_34D11_LC | . | . | . | . | . | T | } | . | . | . | . | . | . | . | . | . | . | . | . | . | . | . | . | . | . | . |
| 21-225_34G8_LC | . | . | . | . | . | T | } | . | . | . | . | . | . | . | . | . | . | . | . | . | . | . | . | . | . | . |
| 21-225_34G9_LC | . | . | . | . | . | T | } | . | . | . | . | . | . | . | . | . | . | . | . | . | . | . | . | . | . | . |
| 21-225_34H11_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_54C6_LC | F | . | . | . | . | . | R | . | . | . | . | . | . | . | . | . | . | . | . | . | . | . | . | . | . | . |
| 21-225_55B8_LC | . | . | . | . | . | . | . | . | . | . | . | . | . | . | . | . | . | . | . | . | . | . | . | . | . | . |
| 21-225_55H6_LC | . | . | . | . | R | D | . | . | . | . | . | . | . | . | . | . | . | . | . | . | . | . | . | . | . | . |
| 21-225_56F1_LC | . | . | . | . | D | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | . | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | S | . | . | . | . | . | . | . | . | . | . | . | . | . | . | . | . | . | . | . | . |
| 21-225_62E2_LC | . | . | . | . | . | S | N | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | S | T | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | S | . | . | . | . | . | . | . | . | . | . | . | . | . | . | . | . | . | . | . | . |
| 21-225_6C12_LC | F | . | . | . | D | . | . | . | . | . | . | . | . | . | . | . | . | . | . | . | . | . | . | . | . | . |
| 21-225_6D4_LC | . | . | . | . | . | S | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | R | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | D | . | . | . | . | . | . | . | . | . | . | . | . | . | . | . | . | . | . | . | . |
| 21-225_74D7_LC | . | . | . | . | . | S | N | . | . | . | . | . | . | . | . | . | . | . | . | . | . | . | . | . | . | . |
| 21-225_74E4_LC | H | . | . | . | . | S | N | . | . | . | . | . | . | . | . | . | . | . | . | . | . | . | . | . | . | . |
| 21-225_75H7_LC | . | . | I | . | . | S | N | . | . | . | . | . | . | . | . | . | . | . | . | . | . | . | . | . | . | . |
| 21-225_7C4_LC | . | . | . | . | . | . | . | . | . | . | . | . | . | . | . | . | . | . | . | . | . | . | . | . | . | . |
| 21-225_7E9_LC | . | . | . | . | . | . | . | . | . | . | . | . | . | . | . | . | . | . | . | . | . | . | . | . | . | . |
| 21-225_7F4_LC | . | . | . | . | . | S | A | . | . | . | . | . | . | . | . | . | . | . | . | . | . | . | . | . | . | . |
| 21-225_7H11_LC | . | . | . | . | . | . | L | . | . | . | . | . | . | . | . | . | . | . | . | . | . | . | . | . | . | . |
| 21-225_7H7_LC | . | . | . | . | . | S | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_8C9_LC | . | . | . | . | . | . | . | . | . | . | . | . | . | . | . | . | . | . | . | . | . | . | . | . | . | . |
| 21-225_8D12_LC | . | . | . | . | . | S | . | . | . | . | . | . | . | . | . | . | . | . | . | . | . | . | . | . | . | . |
| 21-225_8F11_LC | . | . | . | . | . | Y | T | . | . | . | . | . | . | . | . | . | . | . | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | S | N | . | . | . | . | . | . | . | . | . | . | . | . | . | . | . | . | . | . | . |
| 21-225_9E8_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_10G5_LC | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . |
| 21-225_11F10_LC | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . |
| 21-225_11G7_LC | . | . | . | . | . | . | . | . | . | . | . | . | . | M | . | . | . | . | . |
| 21-225_12A1_LC | . | . | . | . | . | . | . | . | . | . | . | . | . | . | . | . | . | . | . |
| 21-225_13E6_LC | . | . | . | . | . | . | . | . | . | . | . | . | . | . | . | A | . | . | . |
| 21-225_13H3_LC | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . |
| 21-225_146F1_LC | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . |
| 21-225_148E11_LC | . | . | . | . | . | . | . | . | . | . | . | . | . | . | . | . | . | . | . |
| 21-225_148F12_LC | . | . | . | . | . | . | . | . | . | . | . | . | . | . | . | . | . | . | . |
| 21-225_149F12_LC | . | . | . | . | . | . | . | . | . | . | . | . | . | . | . | . | . | . | . |
| 21-225_149G1_LC | . | . | . | . | . | . | . | . | . | . | . | . | . | . | . | . | . | . | . |
| 21-225_149G5_LC | . | . | . | . | . | . | . | . | . | . | . | . | . | . | . | . | . | . | . |
| 21-225_14B1_LC1_LC | . | . | . | . | . | . | . | . | . | . | . | . | . | . | . | . | . | . | . |
| 21-225_14B2_LC | . | . | . | . | . | . | . | . | . | . | . | . | . | . | . | . | . | . | . |
| 21-225_14C2_LC | . | . | . | . | . | . | . | . | . | . | . | . | . | . | . | . | . | . | . |
| 21-225_14E3_LC | . | . | . | . | . | . | . | . | . | . | . | . | . | . | . | . | . | . | . |
| 21-225_150E7_LC | . | . | . | . | . | . | . | . | . | . | . | . | . | . | . | . | . | . | . |
| 21-225_152H9_LC | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . |
| 21-225_158H5_LC | . | . | . | . | . | . | . | . | . | . | . | . | . | . | . | . | . | . | . |
| 21-225_15A1_LC | . | . | . | . | . | . | V | . | . | . | . | . | . | . | . | . | . | T | G |
| 21-225_15C9_LC | . | . | . | . | . | . | . | . | . | . | . | . | . | . | . | . | . | . | . |
| 21-225_15E3_LC | . | . | . | . | . | . | . | . | . | . | . | . | . | . | . | . | Y | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_15H1_LC | . | . | . | . | . | . | . | . | . | . | . | . | . | . | . | . | . | . | . |
| 21-225_15H10_LC | . | . | . | . | . | . | . | . | . | . | . | . | . | . | . | . | . | N | . |
| 21-225_15H8_LC | . | . | . | . | . | . | . | . | . | . | . | . | . | . | . | . | . | . | . |
| 21-225_160B10_LC | . | . | . | . | . | . | . | . | . | . | . | . | . | . | . | . | . | . | . |
| 21-225_160H3_LC | . | . | . | . | . | . | . | . | . | . | . | . | . | Q | . | . | S | . | . |
| 21-225_169B1_LC | . | . | . | . | . | . | . | . | . | . | . | . | . | . | . | . | . | . | . |
| 21-225_169F9_LC | . | . | . | . | . | . | . | . | . | . | . | . | . | . | . | . | . | . | . |
| 21-225_16A1_LC | . | . | . | . | . | . | . | . | . | . | . | . | . | . | . | . | . | E | . |
| 21-225_16B7_LC | . | . | . | . | . | . | . | . | . | . | . | . | . | . | . | . | . | N | . |
| 21-225_16E12_LC | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . |
| 21-225_16F4_LC | . | . | . | . | . | . | . | . | . | . | . | . | . | . | . | . | . | . | . |
| 21-225_170D11_LC | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . |
| 21-225_170G4_LC | . | . | . | . | . | . | . | . | . | . | . | . | . | . | . | . | . | . | . |
| 21-225_170G5_LC | . | . | . | . | . | . | . | . | . | . | . | . | . | . | . | . | . | . | . |
| 21-225_171A4_LC | . | . | . | . | . | . | . | . | . | . | . | . | . | . | . | . | . | . | . |
| 21-225_171C3_LC | . | . | . | . | . | . | . | . | . | . | . | . | . | . | . | . | . | . | . |
| 21-225_172B3_LC | . | . | . | . | . | . | . | . | . | . | . | . | . | . | . | . | . | . | . |
| 21-225_172G8_LC | . | . | . | . | . | . | . | . | . | . | . | . | . | . | . | . | . | . | . |
| 21-225_173H12_LC | . | . | . | . | . | . | N | . | . | . | . | . | . | . | . | . | . | . | . |
| 21-225_175G1_LC | . | . | . | . | . | . | . | . | . | . | . | . | . | . | . | . | . | . | . |
| 21-225_176B11_LC | . | . | . | . | . | . | . | . | . | . | . | . | . | . | . | . | . | . | . |
| 21-225_178B10_LC | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . |
| 21-225_17F9_LC | . | . | . | . | . | . | . | . | . | . | . | . | . | . | . | . | . | . | . |
| 21-225_17H12_LC | . | . | . | . | . | . | . | . | . | . | . | . | . | . | . | . | . | . | . |
| 21-225_17H8_LC | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | | | Y | G | - | - | - | - | - | - | - | - | - | - | - | - | - | - | - | - | - |
| 21-225_147B9_HC | . | . | . | . | . | R | G | . | . | D | P | P | Y | . | . | . | . | . | . | . | . | . | . | . | . | . |
| 21-225_148C8_HC | . | . | . | . | . | R | G | . | . | D | P | P | Y | . | . | . | . | . | . | . | . | . | . | . | . | . |
| 21-225_149A11_HC | . | . | . | . | . | R | G | . | . | D | P | P | Y | . | . | . | . | . | . | . | . | . | . | . | . | . |
| 21-225_155E10_HC | . | . | . | . | . | R | D | . | . | D | P | P | Y | . | . | . | . | . | . | . | . | . | . | . | . | . |
| 21-225_190C3_HC | . | . | . | N | W | Y | Y | . | Y | - | . | . | . | . | . | . | . | . | . | . | . | . | . | . | . | . |
| 21-225_190D8_HC | . | . | . | N | W | Y | Y | . | Y | - | . | . | . | . | . | . | . | . | . | . | . | . | . | . | . | . |
| 21-225_211A3_HC | . | . | . | . | . | G | S | . | . | Y | . | . | . | . | . | . | . | . | . | . | . | . | . | . | . | . |
| 21-225_212H11_HC | . | . | . | . | . | G | S | . | . | Y | . | . | . | . | . | . | . | . | . | . | . | . | . | . | . | . |
| 21-225_213E5_HC | . | . | . | . | . | G | S | . | . | Y | . | . | . | . | . | . | . | . | . | . | . | . | . | . | . | . |
| 21-225_217B10_HC | . | . | . | . | . | G | S | . | . | Y | . | . | . | . | . | . | . | . | . | . | . | . | . | . | . | . |
| 21-225_218G4_HC | . | . | . | N | W | Y | Y | . | Y | - | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | Y | G | M | D | V | W | G | Q | G | T | T | V | T | V | S | S |
| 21-225_147B9_HC | Y | Y | Y | . | . | . | . | . | . | . | . | . | . | . | . | . | . | . | . |
| 21-225_148C8_HC | Y | Y | Y | . | . | . | . | . | . | . | . | . | . | . | . | . | . | . | . |
| 21-225_149A11_HC | Y | Y | Y | . | . | . | . | . | . | . | . | . | . | . | . | . | . | . | . |
| 21-225_155E10_HC | Y | Y | Y | . | . | . | . | . | . | . | . | . | . | . | . | . | . | . | . |
| 21-225_190C3_HC | . | . | . | . | . | . | . | . | . | . | . | . | . | . | . | . | . | . | . |
| 21-225_190D8_HC | . | . | . | . | . | . | . | . | . | . | . | . | . | . | . | . | . | . | . |
| 21-225_211A3_HC | . | . | . | D | . | . | . | . | . | . | . | . | . | . | . | . | . | . | . |
| 21-225_212H11_HC | . | . | . | D | . | . | . | . | . | . | . | . | . | . | . | . | . | . | . |
| 21-225_213E5_HC | . | . | . | D | . | . | . | . | . | . | . | . | . | . | . | . | . | . | . |
| 21-225_217B10_HC | . | . | . | D | . | . | . | . | . | . | . | . | . | . | . | . | . | . | . |
| 21-225_218G4_HC | . | . | . | . | . | . | . | . | . | . | . | . | . | . | . | . | . | . | . |

**FIGURE 57**

Table 82.    Consensus 48 -VH3|3-23/D4|4-17|RF2/JH6 (SEQ ID NO: 50299):

EVQLLES-GGGLVQPGGSLRLSCAASG-FTFSS-----YAMSWVRQAPGKGLEWVSAISGS---
GGNTFYADSVKGRFTISRDNSKNTLYLQMNSLRAEDTAVYYCAKLGKDYY-----------------YYGMDVWGQGTTVTVSS wherein:

S at position 33 can be substituted with N or T

Y at position 39 can be substituted with S

A at position 40 can be substituted with V

S at position 42 can be substituted with T or N

A at position 57 can be substituted with G

I at position 58 can be substituted with S or V

S at position 59 can be substituted with V

S at position 61 can be substituted with R

G at position 66 can be substituted with A, S or V

N at position 67 can be substituted with R or K

F at position 69 can be substituted with Y

Y at position 70 can be substituted with N

A at position 71 can be substituted with T

K at position 75 can be substituted with T

L at position 109 can be substituted with D or E

G at position 110 can be substituted with R

K at position 111 can be substituted with G or I

FIGURE 57

D at position 112 can be substituted with Q or Y

Y at position 113 can be substituted with W

Y at position 114 can be substituted with H or L

Y at position 133 can be substituted with null (-), I, or L

Y at position 134 can be substituted with G

M at position 136 can be substituted with V

Xaa Xaa Xaa Met Xaa (SEQ ID NO: 50090)

Xaa Xaa Xaa Gly Xaa Gly Xaa Xaa Thr Xaa Xaa Xaa Asp Ser Val Xaa Gly (SEQ ID NO: 50091)

Xaa Xaa Xaa Xaa Xaa Xaa Xaa Xaa Gly Xaa Asp Val (SEQ ID NO: 50092)

Ser Tyr Ala Met Ser (SEQ ID NO: 50567)

Ala Ile Ser Gly Ser Gly Gly Asn Thr Phe Tyr Ala Asp Ser Val Lys Gly (SEQ ID NO: 50568)

Leu Gly Lys Asp Tyr Tyr Tyr Tyr Gly Met Asp Val (SEQ ID NO: 50569)

FIGURE 57

FIGURE 57

FIGURE 57

**FIGURE 57**

Table 83.    Consensus 49 -VH3|3-23/D7|7-27|RF1/JH4 (SEQ ID NO: 50300):

EVQLLES-GGGLVQPGGSLRLSCAASG-FTFS<u>S-----YVMS</u>WVRQAPGKGLEWVS<u>AMSGS---</u>
<u>GGRTYYADSVKG</u>RFTISRDNSKNTLYLQMNSLRAEDTAVYYCAK<u>LTA----------------------FDY</u>WGQGTLVTVSS wherein:

V at position 40 can be substituted with A

S at position 42 can be substituted with N

A at position 57 can be substituted with G, T or S

M at position 58 can be substituted with I or T

G at position 66 can be substituted with N or V

R at position 67 can be substituted with N or W

Y at position 69 can be substituted with F or N

K at position 75 can be substituted with N

G at position 76 can be substituted with D

L at position 109 can be substituted with V, Y or F

T at position 110 can be substituted with E, F or G

A at position 111 can be substituted with G, L, W, null (-) or F

null (-) at position 112 can be substituted with G or M

null (-) at position 113 can be substituted with V

null (-) at position 114 can be substituted with G

null (-) at position 133 can be substituted with A

null (-) at position 134 can be substituted with G

**FIGURE 57**

null (-) at position 135 can be substituted with I or F

F at position 136 can be substituted with N or null (-)

D at position 137 can be substituted with G or I

Y at position 138 can be substituted with D

Ser Tyr Xaa Met Xaa (SEQ ID NO: 50093)

Xaa Xaa Ser Gly Ser Gly Xaa Xaa Thr Xaa Tyr Ala Asp Ser Val Xaa Xaa (SEQ ID NO: 50094)

Xaa Xaa Xaa Xaa Xaa Xaa Xaa Xaa Xaa Xaa Xaa Xaa (SEQ ID NO: 50095)

Ser Tyr Val Met Ser (SEQ ID NO: 50570)

Ala Met Ser Gly Ser Gly Gly Arg Thr Tyr Tyr Ala Asp Ser Val Lys Gly (SEQ ID NO: 50571)

Leu Thr Ala Phe Asp Tyr (SEQ ID NO: 50572)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_157G5_HC | . | . | . | . | . | . | . | . | . | . | . | . | . | . | . | . | . | . | . | . | . | . | . | . | . | . |
| 21-225_167D12_HC | . | . | . | . | . | . | . | . | . | . | . | . | . | . | . | . | . | . | . | . | . | . | . | . | . | . |
| 21-225_177D3_HC | . | . | . | . | . | . | . | . | . | . | . | . | . | . | . | . | . | . | . | . | . | . | . | . | . | . |
| 21-225_51C5_HC | . | . | . | . | . | . | . | . | . | . | . | . | . | . | . | . | . | . | . | . | . | . | . | . | . | . |
| 21-225_51D5_HC | . | . | . | . | . | . | . | . | . | . | . | . | . | . | . | . | . | . | . | . | . | . | . | . | . | . |
| 21-225_57B8_HC | . | . | . | . | . | . | . | . | . | . | . | . | . | . | . | . | . | . | . | . | . | . | . | . | . | . |
| 21-225_58F11_HC | . | . | . | . | . | . | . | . | . | . | . | . | . | . | . | . | . | . | . | . | . | . | . | . | . | . |
| 21-225_60A4_HC | . | . | . | . | . | . | . | . | . | . | . | . | . | . | . | . | . | . | . | . | . | . | . | . | . | . |
| 21-225_60F7_HC | . | . | . | . | . | . | . | . | . | . | . | . | . | . | . | . | . | . | . | . | . | . | . | . | . | . |
| 21-225_61E6_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | H_CDR1 | | | | | | | | | | | | | | | | | H_FR2 | | | |
| CONSENSUS | G | - | F | T | F | S | S | - | - | - | - | - | Y | V | M | S | W | V | R | Q | A | P | G | K | G | L |
| 21-225_157G5_HC | . | . | . | . | . | . | . | . | . | . | . | . | . | . | . | . | . | . | . | . | . | . | . | . | . | . |
| 21-225_167D12_HC | . | . | . | . | . | . | . | . | . | . | . | . | . | . | . | . | . | . | . | . | . | . | . | . | . | . |
| 21-225_177D3_HC | . | . | . | . | . | . | . | . | . | . | . | . | . | . | . | N | . | . | . | . | . | . | . | . | . | . |
| 21-225_51C5_HC | . | . | . | . | . | . | . | . | . | . | . | . | . | . | . | . | . | . | . | . | Y | . | . | . | . | . |
| 21-225_51D5_HC | . | . | . | . | . | . | . | . | . | . | . | . | . | . | . | . | . | . | . | . | Y | . | . | . | . | . |
| 21-225_57B8_HC | . | . | . | . | . | . | . | . | . | . | . | . | . | A | . | . | . | . | . | . | . | . | . | . | . | . |
| 21-225_58F11_HC | . | . | . | . | . | . | . | . | . | . | . | . | . | A | . | . | . | . | . | . | . | . | . | . | . | . |
| 21-225_60A4_HC | . | . | . | . | . | . | . | . | . | . | . | . | . | . | . | . | . | . | . | . | . | . | . | . | . | . |
| 21-225_60F7_HC | . | . | . | . | . | . | . | . | . | . | . | . | . | . | . | . | . | . | . | . | . | . | . | . | . | . |
| 21-225_61E6_HC | . | . | . | . | . | . | . | . | . | . | . | . | . | A | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | A | M | S | G | S | - | - | - | G | G | R | T | Y | Y | A | D | S | V | K | G | R | F |
| 21-225_157G5_HC | Q | . | . | . | . | . | . | . | . | . | . | . | . | . | . | . | . | . | . | . | . | . | . | . | . | . |
| 21-225_167D12_HC | | . | . | . | . | . | . | . | . | . | . | . | . | . | . | . | . | . | . | . | . | . | . | . | . | . |
| 21-225_177D3_HC | | . | . | . | G | . | . | . | . | . | . | . | . | . | . | . | . | . | . | . | . | . | . | Q | . | . |
| 21-225_51C5_HC | | . | . | . | T | . | . | . | . | . | . | . | . | . | . | . | . | . | . | . | . | . | N | . | . | . |
| 21-225_51D5_HC | | . | . | . | T | . | . | . | . | . | . | . | . | . | . | . | . | . | . | . | . | . | N | . | . | . |
| 21-225_57B8_HC | | . | . | . | . | I | . | . | . | . | . | . | . | . | N | . | F | . | . | . | . | . | . | . | . | . |
| 21-225_58F11_HC | | . | . | . | . | I | . | . | . | . | . | . | . | . | N | . | F | . | . | . | . | . | . | . | . | . |
| 21-225_60A4_HC | | . | . | . | S | I | . | . | . | . | . | . | . | W | . | N | . | . | . | . | . | . | . | . | . | . |
| 21-225_60F7_HC | | . | . | . | G | I | . | . | . | . | . | . | N | . | . | . | . | . | . | . | . | . | . | . | . | . |
| 21-225_61E6_HC | | . | . | . | . | T | . | . | . | . | . | . | V | N | . | F | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_157G5_HC | . | . | . | . | . | . | . | . | . | . | . | . | . | . | . | . | . | . | . | . | . | . | . | . | . | . |
| 21-225_167D12_HC | . | . | . | . | . | . | . | . | . | . | . | . | . | . | . | . | . | . | . | . | . | . | . | . | . | . |
| 21-225_177D3_HC | . | . | . | . | . | . | . | . | . | . | . | S | . | . | . | . | . | . | . | . | . | . | . | . | . | . |
| 21-225_51C5_HC | . | V | . | . | . | . | . | . | . | . | . | . | . | . | . | S | . | . | . | . | . | . | . | . | . | . |
| 21-225_51D5_HC | . | V | . | . | . | . | . | . | . | . | . | . | . | . | . | S | . | . | . | . | . | . | . | . | . | . |
| 21-225_57B8_HC | . | . | . | . | . | . | . | . | . | . | . | . | . | . | . | . | . | . | . | . | . | . | . | . | . | . |
| 21-225_58F11_HC | . | . | . | . | Y | . | . | . | . | . | . | . | . | . | . | . | . | . | . | . | . | . | . | . | . | . |
| 21-225_60A4_HC | . | T | . | . | . | . | . | . | . | . | . | . | . | . | . | . | . | . | . | . | . | . | . | . | . | . |
| 21-225_60F7_HC | . | . | . | . | . | . | . | . | . | . | . | . | . | . | . | . | . | . | . | . | . | . | . | S | . | . | . |
| 21-225_61E6_HC | . | . | . | . | . | . | . | . | . | . | . | . | . | . | . | . | . | . | . | . | . | . | . | . | . | . |

## FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | K | L | T | - | - | - | - | - | - | - | - | - | - | - | - | - | - | - | - | - | - | - | - |
| 21-225_157G5_HC | | | | | Y | . | W | . | . | . | . | . | . | . | . | . | . | . | . | . | . | . | . | . | . | . |
| 21-225_167D12_HC | | | | | Y | . | W | . | . | . | . | . | . | . | . | . | . | . | . | . | . | . | . | . | . | . |
| 21-225_177D3_HC | | | . | R | V | . | F | . | . | . | . | . | . | . | . | . | . | . | . | . | . | . | . | . | . | . |
| 21-225_51C5_HC | | | . | R | V | . | A | . | . | . | . | . | . | . | . | . | . | . | . | . | . | . | . | . | . | . |
| 21-225_51D5_HC | | | . | R | V | . | A | . | . | . | . | . | . | . | . | . | . | . | . | . | . | . | . | . | . | . |
| 21-225_57B8_HC | | | . | . | . | E | L | G | . | . | . | . | . | . | . | . | . | . | . | . | . | . | . | . | . | . |
| 21-225_58F11_HC | | | . | . | F | F | G | M | V | G | . | . | . | . | . | . | . | . | . | . | . | . | . | . | . | . |
| 21-225_60A4_HC | | | . | R | . | . | G | . | . | . | . | . | . | . | . | . | . | . | . | . | . | . | . | . | . | . |
| 21-225_60F7_HC | | . | G | S | . | G | - | . | . | . | . | . | . | . | . | . | . | . | . | . | . | . | . | . | . | . |
| 21-225_61E6_HC | | | . | . | . | E | L | G | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | - | F | D | Y | W | G | Q | G | T | L | V | T | V | S | S |
| 21-225_157G5_HC | . | . | . | . | . | N | G | . | . | . | . | . | . | . | . | . | . | . | . |
| 21-225_167D12_HC | . | . | . | . | . | N | G | . | . | . | . | . | . | . | . | . | . | . | . |
| 21-225_177D3_HC | . | . | . | . | . | . | . | . | . | . | . | . | . | . | . | . | . | . | . |
| 21-225_51C5_HC | . | . | . | . | . | . | . | . | . | . | . | . | . | . | . | . | . | . | . |
| 21-225_51D5_HC | . | . | . | . | . | . | . | . | . | . | . | . | . | . | . | . | . | . | . |
| 21-225_57B8_HC | . | . | . | . | i | . | . | . | . | . | . | . | . | . | . | . | . | . | . |
| 21-225_58F11_HC | . | . | A | G | F | . | . | . | . | . | . | . | . | . | . | . | . | . | . |
| 21-225_60A4_HC | . | . | . | . | . | . | . | . | . | . | . | . | . | . | . | . | . | . | . |
| 21-225_60F7_HC | . | . | . | . | . | - | I | D | . | . | . | . | . | . | . | . | . | . | . |
| 21-225_61E6_HC | . | . | . | . | i | . | . | . | . | . | . | . | . | . | . | . | . | . | . |

**FIGURE 57**

Table 84.      Consensus 50 -VH3|3-30.3/D5|5-24|RF3/JH6 (SEQ ID NO: 50301):

QVQLVES-GGGVVQPGRSLRLSCAASG-FTFS<u>S</u>-----<u>YGMH</u>WVRQAPGKGLEWVA<u>IIS</u><u>Y</u><u>A</u>---
GSNKYYADSVKGRFTISRDNSKNTLYLQMNSLRAEDTAVYYCVR<u>RGYSYG</u>-----------------<u>GYGMD</u>VWGQGTTVTVSS wherein:

S at position 33 can be substituted with Y

M at position 41 can be substituted with L

I at position 57 can be substituted with V

A at position 61 can be substituted with G, D, S or V

S at position 66 can be substituted with I, N, R or T

K at position 68 can be substituted with N or Q

Y at position 69 can be substituted with S, D or H

Y at position 70 can be substituted with S

R at position 109 can be substituted with E

G at position 110 can be substituted with D

Y at position 111 can be substituted with R

S at position 112 can be substituted with Y

Y at position 113 can be substituted with C

G at position 114 can be substituted with S

null (-) at position 115 can be substituted with G

null (-) at position 116 can be substituted with T

null (-) at position 117 can be substituted with S

**FIGURE 57**

null (-) at position 118 can be substituted with C

null (-) at position 129 can be substituted with P

null (-) at position 130 can be substituted with Y

null (-) at position 131 can be substituted with Y

null (-) at position 132 can be substituted with Y

G at position 133 can be substituted with Y

Xaa Tyr Gly Xaa His (SEQ ID NO: 50096)

Xaa Ile Ser Tyr Xaa Gly Xaa Asn Xaa Xaa Xaa Ala Asp Ser Val Lys Gly (SEQ ID NO: 50097)

Xaa Xaa Xaa Xaa Xaa Xaa Xaa Xaa Xaa Xaa Xaa Xaa Xaa Xaa Xaa Tyr Gly Met Asp Val (SEQ ID NO: 50098)

Ser Tyr Gly Met His (SEQ ID NO: 50573)

Ile Ile Ser Tyr Ala Gly Ser Asn Lys Tyr Tyr Ala Asp Ser Val Lys Gly (SEQ ID NO: 50574)

Arg Gly Tyr Ser Tyr Gly Gly Tyr Gly Met Asp Val (SEQ ID NO: 50575)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_11A2_HC | . | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | . | . | . | . | . | . | V | . |
| 21-225_1B12_HC | . | . | . | . | . | . | . | . | . | . | . | . | . | . | . | . | . | . | . | . | . | . | . | . | . |
| 21-225_225B11_HC | . | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | . | . | . | . |
| 21-225_45F6_HC | . | . | . | . | . | . | . | . | . | . | . | . | . | . | . | . | . | . | . | . | . | . | . | . | . |
| 21-225_5E6_HC | . | . | . | . | . | . | . | . | . | . | . | . | . | . | . | . | . | . | . | . | . | . | . | . | . |

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | | | | | | | H_FR2 | |
| CONSENSUS | S | G | - | F | T | F | S | S | - | - | - | - | - | Y | G | M | H | W | V | R | Q | A | P | G | K |
| 21-225_11A2_HC | . | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | . | . | . | . | . | . | . | . |
| 21-225_1B12_HC | . | . | . | . | . | . | . | . | . | . | . | . | . | . | . | . | . | . | . | . | . | . | . | . | . |
| 21-225_225B11_HC | . | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | V | . | . | . |
| 21-225_45F6_HC | . | . | . | . | . | . | . | Y | . | . | . | . | . | . | . | L | . | . | . | . | . | . | . | . | . |
| 21-225_5E6_HC | . | . | . | . | . | . | . | . | . | . | . | . | . | . | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | V | A | | I | S | Y | A | - | - | - | G | S | N | K | Y | Y | A | D | S | V | K |
| 21-225_11A2_HC | . | . | . | . | . | . | V | . | . | . | S | . | . | . | . | . | . | . | D | S | . | . | . | . | . |
| 21-225_13A1_HC | . | . | . | . | . | . | V | . | . | . | . | . | . | . | . | T | . | Q | . | . | . | . | . | . | . |
| 21-225_15C2_HC | . | . | . | . | . | S | I | . | . | . | G | . | . | . | . | . | . | N | . | . | . | . | . | . | . |
| 21-225_15F10_HC | . | . | . | . | . | S | V | . | . | . | G | . | . | . | . | . | . | N | . | . | . | . | . | . | . |
| 21-225_16B3_HC | . | . | . | . | . | . | V | . | . | . | . | . | . | . | . | I | . | . | . | S | . | . | . | . | . |
| 21-225_1B12_HC | . | . | . | . | . | . | I | . | . | . | . | . | . | . | . | . | . | . | . | S | . | . | . | . | . |
| 21-225_225B11_HC | . | . | . | . | . | T | I | . | . | . | S | . | . | . | . | . | . | . | . | . | . | . | . | . | . |
| 21-225_24D6_HC | . | . | . | . | . | . | I | . | . | . | . | . | . | . | . | . | . | . | . | S | . | . | . | . | . |
| 21-225_45F6_HC | . | . | . | . | . | . | V | . | . | . | G | . | . | . | . | . | . | . | . | . | . | . | . | . | . |
| 21-225_5E6_HC | . | . | . | . | . | . | I | . | . | . | V | . | . | . | . | N | . | . | . | . | . | . | . | . | . |

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | H_FR3 | | | | | | | | | | | | | | |
| CONSENSUS | G | R | F | T | I | S | R | D | N | S | K | N | T | L | Y | L | Q | M | N | S | L | R | A | E | D |
| 21-225_11A2_HC | . | . | S | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | N | . | . | . | . | . | . | . | . | . | . | . | . | . | . |
| 21-225_1B12_HC | . | . | . | . | . | . | . | . | . | . | . | . | M | . | . | . | . | . | . | . | . | . | . | . | . |
| 21-225_225B11_HC | . | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | . | . | . | . |
| 21-225_45F6_HC | . | . | . | . | . | . | . | . | . | . | . | . | . | . | . | . | . | . | N | . | . | . | . | . | . |
| 21-225_5E6_HC | . | . | . | . | . | . | . | . | . | . | . | . | . | . | . | . | . | . | . | . | . | . | V | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | V | R | R | G | Y | S | Y | G | - | - | - | - | - | - | - | - | - | - | - |
| 21-225_11A2_HC | . | | . | . | . | . | . | | . | . | | . | . | . | | . | | . | . | . | | . | . | . | . |
| 21-225_13A1_HC | . | . | | . | | . | | . | | . | | . | | . | | . | | . | . | . | | . | . | . | . |
| 21-225_15C2_HC | . | . | | . | | . | | . | | . | | . | | . | | . | | . | . | . | | . | . | . | . |
| 21-225_15F10_HC | . | . | | . | | . | | . | | . | | . | | . | | . | | . | . | . | | . | . | . | . |
| 21-225_16B3_HC | . | . | | . | | . | | . | | . | | . | | . | | . | | . | . | . | | . | . | . | . |
| 21-225_1B12_HC | . | . | | . | | . | | . | | . | | . | | . | | . | | . | . | . | | . | . | . | . |
| 21-225_225B11_HC | . | . | | . | . | | A | . | | . | | . | | . | | . | | . | . | . | | . | . | . | . |
| 21-225_24D6_HC | . | . | | . | | . | . | | . | . | | . | | . | | . | | . | . | . | | . | . | . | . |
| 21-225_45F6_HC | . | . | | Y | . | . | A | . | E | D | R | Y | C | S | S | T | S | C | | . | | . | . | . | . |
| 21-225_5E6_HC | . | . | | . | | . | A | . | | . | | . | | . | | . | | . | . | . | | . | . | . | . |

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | G | Y | G | M | D | V | W | G | Q | G | T | T | V | T | V | S | S |
| 21-225_11A2_HC | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | A | . | . | . | . | . | . |
| 21-225_1B12_HC | . | . | . | . | . | . | . | . | . | . | . | . | . | . | . | . | . | A | . | . | . | . | . | . |
| 21-225_225B11_HC | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | A | . | . | . | . | . | . |
| 21-225_45F6_HC | . | . | . | S | Y | Y | Y | Y | . | . | . | . | . | . | . | . | . | . | . | . | . | . | . | . |
| 21-225_5E6_HC | . | . | . | . | . | . | . | . | . | . | . | . | . | . | . | . | . | . | . | . | . | . | . | . |

**FIGURE 57**

Table 85.     Consensus 51 -VH3|3-33/D3|3-10|RF2/JH6 (SEQ ID NO: 50302):

QVQLVES-GGGVVQPGRSLRLSCAASG-FTFS<u>D</u>-----<u>YVMH</u>WVRQAPGKGLEWVA<u>VIWYD</u>---
GSNKYYADSVKG<u>R</u>FTISRDNSKNTLYLQMNSLRAEDTAVYYCAR<u>EPYTSGW</u>---------------<u>YDYGMD</u>VWGQGTTVTVSS wherein:

D at position 33 can be substituted with S

Y at position 39 can be substituted with C

V at position 40 can be substituted with G

H at position 42 can be substituted with Q

V at position 57 can be substituted with I

G at position 76 can be substituted with V

P at position 110 can be substituted with R

T at position 112 can be substituted with N

Y at position 132 can be substituted with H

M at position 136 can be substituted with L

Xaa Xaa Xaa Met Xaa (SEQ ID NO: 50099)

Xaa Ile Trp Tyr Asp Gly Ser Asn Lys Tyr Tyr Ala Asp Ser Val Lys Xaa (SEQ ID NO: 50100)

Glu Xaa Tyr Xaa Ser Gly Trp Xaa Asp Tyr Gly Xaa Asp Val (SEQ ID NO: 50101)

Asp Tyr Val Met His (SEQ ID NO: 50576)

Val Ile Trp Tyr Asp Gly Ser Asn Lys Tyr Tyr Ala Asp Ser Val Lys Gly (SEQ ID NO: 50577)

Glu Pro Tyr Thr Ser Gly Trp Tyr Asp Tyr Gly Met Asp Val (SEQ ID NO: 50578)

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_158D4_HC | . | . | . | . | . | . | . | . | . | . | . | . | . | . | . | . | . | . | . | . | . | . | . | . | . |
| 21-225_159A3_HC | . | . | . | . | . | . | . | . | . | . | . | . | . | . | . | . | . | . | . | . | . | . | . | . | . |
| 21-225_159C8_HC | . | . | . | . | . | . | . | . | . | . | . | . | . | . | . | . | . | . | . | . | . | . | . | . | . |
| 21-225_160C4_HC | . | . | . | . | . | . | . | . | . | . | . | . | . | . | . | . | . | . | . | . | . | . | . | . | . |
| 21-225_161G12_HC | . | . | . | . | . | . | . | . | . | . | . | . | . | . | . | . | . | . | . | . | . | . | . | . | . |
| 21-225_20B11_HC | . | . | . | . | . | . | . | . | . | . | . | . | . | . | . | . | . | . | . | . | . | . | . | . | . |
| 21-225_226F7_HC | . | . | . | . | . | . | . | . | . | . | . | . | . | . | . | . | . | . | . | . | . | . | . | . | . |
| 21-225_2B10_LC1_HC | . | . | . | . | . | . | . | . | . | . | . | . | . | . | . | . | . | . | . | . | . | . | . | . | . |
| 21-225_2B10_LC2_HC | . | . | . | . | . | . | . | . | . | . | . | . | . | . | . | . | . | . | . | . | . | . | . | . | . |
| 21-225_7F8_HC | . | . | . | . | . | . | . | . | . | . | . | . | . | . | . | . | . | . | . | . | . | . | . | . | . |

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | | - | - | - | - | - | Y | V | M | H | W | V | R | Q | A | P | G | K |
| 21-225_158D4_HC | . | . | . | . | . | . | . | D | . | . | . | . | . | . | . | . | . | . | . | . | . | . | . | . | . |
| 21-225_159A3_HC | . | . | . | . | . | . | . | D | . | . | . | . | . | . | . | . | Q | . | . | . | . | . | . | . | . |
| 21-225_159C8_HC | . | . | . | . | . | . | . | D | . | . | . | . | . | . | . | . | Q | . | . | . | . | . | . | . | . |
| 21-225_160C4_HC | . | . | . | . | . | . | . | D | . | . | . | . | . | . | . | . | Q | . | . | . | . | . | . | . | . |
| 21-225_161G12_HC | . | . | . | . | . | . | . | D | . | . | . | . | . | . | . | . | Q | . | . | . | . | . | . | . | . |
| 21-225_20B11_HC | . | . | . | . | . | . | . | S | . | . | . | . | . | . | . | . | . | . | . | . | . | . | . | . | . |
| 21-225_226F7_HC | . | . | . | . | . | . | . | S | . | . | . | . | . | G | . | . | . | . | . | . | . | . | . | . | . |
| 21-225_2B10_LC1_HC | . | . | . | . | . | . | . | S | . | . | . | . | . | . | . | . | . | . | . | . | . | . | . | . | . |
| 21-225_2B10_LC2_HC | . | . | . | . | . | . | . | S | . | . | . | . | . | . | . | . | . | . | . | . | . | . | . | . | . |
| 21-225_7F8_HC | . | . | . | . | . | . | . | S | . | . | . | . | C | . | . | . | . | . | . | . | . | . | . | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | V | A | V | I | W | Y | D | - | - | - | G | S | N | K | Y | Y | A | D | S | V | K |
| 21-225_158D4_HC | . | . | . | . | . | S | . | . | . | . | . | . | . | . | . | . | . | . | . | . | . | . | . | . | . |
| 21-225_159A3_HC | . | . | . | . | . | . | . | . | . | . | . | . | . | . | . | . | . | . | . | . | . | . | . | . | . |
| 21-225_159C8_HC | . | . | . | . | . | . | . | . | . | . | . | . | . | . | . | . | . | . | . | . | . | . | . | . | . |
| 21-225_160C4_HC | . | . | . | . | . | . | . | . | . | . | . | . | . | . | . | . | . | . | . | . | . | . | . | . | . |
| 21-225_161G12_HC | . | . | . | . | . | . | . | . | . | . | . | . | . | . | . | . | . | . | . | . | . | . | . | . | . |
| 21-225_20B11_HC | . | . | . | . | . | . | . | . | . | . | . | . | . | . | . | . | . | . | . | . | . | . | . | . | . |
| 21-225_226F7_HC | . | . | . | . | . | ! | . | . | . | . | . | . | . | . | . | . | . | . | . | . | . | . | . | . | . |
| 21-225_2B10_LC1_HC | . | . | . | . | . | . | . | . | . | . | . | . | . | . | . | . | . | . | . | . | . | . | . | . | . |
| 21-225_2B10_LC2_HC | . | . | . | . | . | . | . | . | . | . | . | . | . | . | . | . | . | . | . | . | . | . | . | . | . |
| 21-225_7F8_HC | . | . | . | . | . | . | . | . | . | . | . | . | . | . | . | . | . | . | . | . | . | . | . | . | . |

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | | | | | | | | | | | | | | | | H_FR3 | | | | | | | | |
| CONSENSUS | G | R | F | T | I | S | R | D | N | S | K | N | T | L | Y | L | Q | M | N | S | L | R | A | E | D |
| 21-225_158D4_HC | . | . | . | . | . | . | . | . | . | . | . | . | . | . | . | . | . | . | . | . | . | . | . | . | . |
| 21-225_159A3_HC | . | . | . | . | . | . | . | . | ! | . | . | . | . | . | . | . | . | . | . | . | . | . | . | . | . |
| 21-225_159C8_HC | . | . | . | . | . | . | . | . | . | . | . | . | . | . | . | . | . | . | . | . | . | . | . | . | . |
| 21-225_160C4_HC | . | . | . | . | . | . | . | . | . | . | . | . | . | . | . | . | . | . | . | . | . | . | . | . | . |
| 21-225_161G12_HC | . | . | . | . | . | . | . | . | . | . | . | . | . | . | . | . | . | . | . | . | . | . | . | . | . |
| 21-225_20B11_HC | V | . | . | . | V | . | . | . | . | . | . | . | . | . | S | . | . | . | . | . | . | . | . | . | . |
| 21-225_226F7_HC | . | . | . | . | . | . | . | . | . | . | . | . | . | . | . | . | . | . | . | . | . | . | . | . | . |
| 21-225_2B10_LC1_HC | V | . | . | . | V | . | . | . | . | . | . | . | . | . | S | . | . | . | . | . | . | . | . | . | . |
| 21-225_2B10_LC2_HC | V | . | . | . | V | . | . | . | . | . | . | . | . | . | S | . | . | . | . | . | . | . | . | . | . |
| 21-225_7F8_HC | . | . | . | . | . | . | . | . | . | . | N | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 | Refe |
|---|---|---|---|---|---|---|---|---|---|---|---|---|---|---|---|---|---|---|---|---|---|---|---|---|---|
| | | | | | | | | | | | | | | | | | | | H_FR4 | | | | | | |
| CONSENSUS | - | - | - | - | - | - | Y | D | Y | G | M | D | V | W | G | Q | G | T | T | V | T | V | S | S | |
| 21-225_158D4_HC | | | | | | | | | | | | | | | | | | | | | | | | | |
| 21-225_159A3_HC | | | | | | | | | | | | | | | | | | | | | | | | | |
| 21-225_159C8_HC | | | | | | | | | | | | | | | | | | | | | | | | | |
| 21-225_160C4_HC | | | | | | | | | | | | | | | | | | | | | | | | | |
| 21-225_161G12_HC | | | | | | | | | | | | | | | | | | | | | | | | | |
| 21-225_20B11_HC | | | | | | | | | | | | | | | | | | | | | | | | | |
| 21-225_226F7_HC | | | | | | | | | | | | | | | | | | | | | | | | | |
| 21-225_2B10_LC1_HC | | | | | | | | | | | | | | | | | | | | | | | | | |
| 21-225_2B10_LC2_HC | | | | | | | | | | | | | | | | | | | | | | | | | |
| 21-225_7F8_HC | | | | | | | | | | | | | | | | | | | | | | | | | |

| Reference # | rence # |
|---|---|
| CONSENSUS | |
| 21-225_158D4_HC | |
| 21-225_159A3_HC | |
| 21-225_159C8_HC | |
| 21-225_160C4_HC | |
| 21-225_161G12_HC | |
| 21-225_20B11_HC | |
| 21-225_226F7_HC | |
| 21-225_2B10_LC1_HC | |
| 21-225_2B10_LC2_HC | |
| 21-225_7F8_HC | |

FIGURE 57

Table 86.        Consensus 52 -VH1|1-18/D3|3-3|RF2/JH6 (SEQ ID NO: 50303):

QVQLVQS-GAEVKKPGASVKVSCKASG-YTFN<u>S</u>-----<u>YGIS</u>WVRQAPGQGLEWMG<u>WISAY</u>---
NGNTKYAQKLQGRVTMTTDTSTSTAYMELRSLRSDDTAVYYCAR<u>HDFWSGY</u>----------------YKGMDVWGQGTTVTVSS wherein:

I in position 41 can be substituted with F or V

T in position 68 can be substituted with R

Y in position 70 can be substituted with N, E or F

K in position 73 can be substituted with R

L in position 74 can be substituted with F

Ser Tyr Gly Xaa Ser (SEQ ID NO: 50102)

Trp Ile Ser Ala Tyr Asn Gly Asn Xaa Lys Xaa Ala Gln Xaa Xaa Gln Gly (SEQ ID NO: 50103)

His Asp Phe Trp Ser Gly Tyr Tyr Lys Gly Met Asp Val (SEQ ID NO: 50227)

Ser Tyr Gly Ile Ser (SEQ ID NO: 50579)

Trp Ile Ser Ala Tyr Asn Gly Asn Thr Lys Tyr Ala Gln Lys Leu Gln Gly (SEQ ID NO: 50580)

His Asp Phe Trp Ser Gly Tyr Tyr Lys Gly Met Asp Val (SEQ ID NO: 50581)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | Q | S | - | G | A | E | V | K | K | P | G | A | S | V | K | V | S | C | K | A | S |
| 21-225_49C5_HC | | | | | | | | | | | | | | | | | | | | | | | | | | |
| 21-225_49D10_HC | | | | | | | | | | | | | | | | | | | | | | | | | | |
| 21-225_51E3_HC | | | | | | | | | | | | | | | | | | | | | | | | | | |
| 21-225_51F4_HC | | | | | | | | | | | | | | | | | | | | | | | | | | |
| 21-225_52F1_HC | | | | | | | | | | | | | | | | | | | | | | | | | | |
| 21-225_53E8_HC | | | | | | | | | | | | | | | | | | | | | | | | | | |
| 21-225_54E9_HC | | | | | | | | | | | | | | | | | | | | | | | | | | |
| 21-225_56E3_HC | | | | | | | | | | | | | | | | | | | | | | | | | | |
| 21-225_56G1_HC | | | | | | | | | | | | | | | | | | | | | | | | | | |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | Y | T | F | N | S | - | - | - | - | - | Y | G | I | S | W | V | R | Q | A | P | G | Q | G | L |
| 21-225_49C5_HC | | | | | | | | | | | | | | | | | | | | | | | | | | |
| 21-225_49D10_HC | | | | | | | | | | | | | | | | | | | | L | | | | | | M |
| 21-225_51E3_HC | | | | | | | | | | | | | | | | | | | | L | | | | | | |
| 21-225_51F4_HC | | | | | | | | | | | | | | | V | | | | | | | | | | | |
| 21-225_52F1_HC | | | | | | | | | | | | | | | V | | | | | | | | | | | |
| 21-225_53E8_HC | | | | | | | | | | | | | | | | | | | | | | | | | | |
| 21-225_54E9_HC | | | | | | | | | | | | | | | | | | | | L | | | | | | |
| 21-225_56E3_HC | | | | | | | | | | | | | | | F | | | | | | | | | | | |
| 21-225_56G1_HC | | | | | | | | | | | | | | | | | | | | | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | M | G | W | I | S | A | Y | - | - | - | N | G | N | T | K | Y | A | Q | K | L | Q | G | R | V |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | N | . | . | . | . | . | . | . | . |
| 21-225_51E3_HC | . | . | . | . | . | . | . | . | . | . | . | . | . | . | . | . | . | N | . | . | N | S | . | . | . | . |
| 21-225_51F4_HC | . | . | . | . | . | . | . | . | . | . | . | . | . | . | . | . | . | . | . | . | . | . | . | . | . | . |
| 21-225_52F1_HC | . | . | . | . | . | . | . | . | . | . | . | . | . | . | . | R | . | . | . | . | . | . | . | . | . | . |
| 21-225_53E8_HC | . | . | . | . | . | . | . | . | . | . | . | . | . | . | . | . | . | F | . | . | . | . | . | . | . | . |
| 21-225_54E9_HC | . | . | . | . | . | . | . | . | . | . | . | . | . | . | . | . | . | N | . | . | . | . | . | . | . | . |
| 21-225_56E3_HC | . | . | . | . | . | . | . | . | . | . | . | . | . | . | . | . | . | R | . | . | . | S | . | . | . | . |
| 21-225_56G1_HC | . | . | . | . | . | . | . | . | . | . | . | . | . | . | . | . | . | . | . | . | . | S | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | M | T | T | D | T | S | T | S | T | A | Y | M | E | L | R | S | L | R | S | D | D | T | A | V | Y |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . | . | . | . | . | . | . | . |
| 21-225_52F1_HC | S | . | . | . | . | . | . | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . | . | . | . | . | . | . | . |
| 21-225_56E3_HC | . | . | . | . | . | . | . | . | . | . | . | . | . | . | . | . | . | . | A | . | . | . | . | . | . | . |
| 21-225_56G1_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | H | D | F | W | S | G | Y | - | - | - | - | - | - | - | - | - | - | - | - | - | - | - |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . | . | . | . | . | . | . | . |
| 21-225_52F1_HC | . | . | . | . | . | . | . | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . | . | . | . | . | . | . | . |
| 21-225_56E3_HC | . | . | . | . | . | . | . | . | . | . | . | . | . | . | . | . | . | . | . | . | . | . | . | . | . | . |
| 21-225_56G1_HC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | Y | K | G | M | D | V | W | G | Q | G | T | T | V | T | V | S | S |
| 21-225_49C5_HC | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . |
| 21-225_52F1_HC | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . |
| 21-225_56E3_HC | . | . | . | . | . | . | . | . | . | . | . | . | . | . | . | . | . | . | . |
| 21-225_56G1_HC | . | . | . | . | . | . | . | . | . | . | . | . | . | . | . | . | . | . | . |

FIGURE 57

Table 87. Consensus 53 - VH3|3-23/D6|6-6|RF1/JH4 (SEQ ID NO: 50304):

EVQLLES-GGGLVQPGGSLRLSCAASG-FTFS<u>S</u>-----<u>YAMS</u>WVRQAPGKGLEWVS<u>AISGR---GG
NTFDADSVKG</u>RFTISRDNSKNTLYLQMNSLRAEDTAVYYCAK<u>ERSGS</u>-------------------<u>YFDY</u>WGQGTLVTVSS wherein:

Y at position 39 can be substituted with S

A at position 57 can be substituted with V or S

R at position 61 can be substituted with S

G at position 66 can be substituted with I or V

N at position 67 can be substituted with S

D at position 70 can be substituted with Y

A at position 71 can be substituted with T

E at position 109 can be substituted with S

R at position 110 can be substituted with N

G at position 112 can be substituted with S

S at position 113 can be substituted with G

Y at position 135 can be substituted with W

Xaa Xaa Ala Met Ser (SEQ ID NO: 50104)

Xaa Ile Ser Gly Xaa Gly Xaa Xaa Thr Phe Xaa Xaa Asp Ser Val Lys Gly (SEQ ID NO: 50105)

Xaa Xaa Ser Xaa Xaa Xaa Phe Asp Tyr (SEQ ID NO: 50106)

Ser Tyr Ala Met Ser (SEQ ID NO: 50582)

Ala Ile Ser Gly Arg Gly Gly Asn Thr Phe Asp Ala Asp Ser Val Lys Gly (SEQ ID NO: 50583)

FIGURE 57

Glu Arg Ser Gly Ser Tyr Phe Asp Tyr (SEQ ID NO: 50584)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_171A8_HC | . | . | . | . | . | . | . | . | . | . | . | . | . | . | . | . | . | . | . | . | . | . | . | . | . | . |
| 21-225_174G7_HC | . | . | . | . | . | . | . | . | . | . | . | . | . | . | . | . | . | . | . | . | . | . | . | . | . | . |
| 21-225_175C10_HC | . | . | . | . | . | . | . | . | . | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | H_CDR1 | | | | | | | | | | | | | | | | | H_FR2 | | | |
| CONSENSUS | G | - | F | T | F | S | S | - | - | - | - | - | Y | A | M | S | W | V | R | Q | A | P | G | K | G | L |
| 21-225_171A8_HC | . | . | . | . | . | § | . | . | . | . | . | . | § | . | . | . | . | . | . | . | . | . | . | . | . | . |
| 21-225_174G7_HC | . | . | . | . | . | § | . | . | . | . | . | . | § | . | . | . | . | . | . | . | . | . | . | . | . | . |
| 21-225_175C10_HC | . | . | . | . | . | . | . | . | . | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | H_CDR2 | | | | | | | | | | | | | |
| CONSENSUS | E | W | V | S | A | I | S | G | R | - | - | - | G | | N | T | F | D | A | D | S | V | K | G | R | F |
| 21-225_171A8_HC | . | . | . | . | V | . | . | . | S | . | . | . | . | S | S | . | . | Y | T | . | . | . | . | . | . | . |
| 21-225_174G7_HC | . | . | . | . | V | . | . | . | S | . | . | . | . | S | S | . | . | Y | T | . | . | . | . | . | . | . |
| 21-225_175C10_HC | . | . | . | . | S | . | . | . | . | . | . | . | . | S | S | . | . | Y | . | . | . | . | . | . | . | . |
| 21-225_43C2_HC | . | . | . | . | . | . | . | . | . | . | . | . | . | V | . | . | . | . | . | . | . | . | . | . | . | . |
| 21-225_43E8_HC | . | . | . | . | . | . | . | . | . | . | . | . | . | I | . | . | . | . | . | . | . | . | . | . | . | . |
| 21-225_43H4_HC | . | . | . | . | . | . | . | . | . | . | . | . | . | I | . | . | . | . | . | . | . | . | . | . | . | . |
| 21-225_44D10_HC | . | . | . | . | . | . | . | . | . | . | . | . | . | V | . | . | . | . | . | . | . | . | . | . | . | . |
| 21-225_45F8_HC | . | . | . | . | . | . | . | . | . | . | . | . | . | V | . | . | . | . | . | . | . | . | . | . | . | . |
| 21-225_46A6_HC | . | . | . | . | . | . | . | . | . | . | . | . | . | I | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_171A8_HC | . | . | . | . | . | . | . | . | . | . | . | . | . | . | . | . | . | . | . | . | . | . | . | . | . | . |
| 21-225_174G7_HC | . | . | . | . | . | . | . | . | . | . | . | . | . | . | . | . | . | . | . | . | . | . | . | . | . | . |
| 21-225_175C10_HC | . | V | . | . | . | . | . | . | . | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | K | E | R | S | G | S | - | - | - | - | - | - | - | - | - | - | - | - | - | - | - | - | - |
| 21-225_171A8_HC | . | . | . | . | S | N | . | S | G | . | . | . | . | . | . | . | . | . | . | . | . | . | . | . | . | . |
| 21-225_174G7_HC | . | . | . | . | S | N | . | S | G | . | . | . | . | . | . | . | . | . | . | . | . | . | . | . | . | . |
| 21-225_175C10_HC | . | . | . | . | S | N | . | S | G | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | Y | F | D | Y | W | G | Q | G | T | L | V | T | V | S | S |
| 21-225_171A8_HC | . | . | . | . | W | . | . | . | . | . | . | . | . | . | . | . | . | . | . |
| 21-225_174G7_HC | . | . | . | . | W | . | . | . | . | . | . | . | . | . | . | . | . | . | . |
| 21-225_175C10_HC | . | . | . | . | W | . | . | . | . | . | . | . | . | . | . | . | . | . | . |
| 21-225_43C2_HC | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . |

FIGURE 57

Table 88.    Consensus 54 -VH3|3-33/D6|6-6|RF1/JH4 (SEQ ID NO: 50305):

QVQLVES-GGGVVQPGRSLRLSCAASG-FTFSN-----YGMHWVRQAPGKGLEWVAVIWHD---
GSNKYYADSVKGRFTISRDNSKNTLYLQMNSLRAEDTAVYYCARENSSS------------------YYFDYWGQGTLVTVSS wherein:

N in position 33 can be substituted with S, Y or H

G in position 40 can be substituted with V

V in position 57 can be substituted with L

H in position 60 can be substituted with Y

S in position 66 can be substituted with T

N in position 67 can be substituted with D

K in position 68 can be substituted with A

A in position 71 can be substituted with V or G

Y in position 134 can be substituted with F

Xaa Tyr Xaa Met His (SEQ ID NO: 50107)

Xaa Ile Trp Xaa Asp Gly Xaa Xaa Xaa Tyr Tyr Xaa Asp Ser Val Lys Gly (SEQ ID NO: 50108)

Glu Asn Ser Ser Ser Xaa Tyr Phe Asp Tyr Trp Gly Gln Gly Thr Leu Val Thr Val Ser Ser (SEQ ID NO: 50109)

Asn Tyr Gly Met His (SEQ ID NO: 50585)

Val Ile Trp His Asp Gly Ser Asn Lys Tyr Tyr Ala Asp Ser Val Lys Gly (SEQ ID NO: 50586)

Glu Asn Ser Ser Ser Tyr Tyr Phe Asp Tyr Trp Gly Gln Gly Thr Leu Val Thr Val Ser Ser (SEQ ID NO: 50587)

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_30G1_HC | . | . | . | . | . | . | . | | . | . | . | . | . | . | . | . | . | . | . | . | . | . | . | . | . | . |
| 21-225_30G4_HC | . | . | . | . | . | . | . | | . | . | . | . | . | . | . | . | . | . | . | . | . | . | . | . | . | . |
| 21-225_32D6_HC | . | . | . | . | . | . | . | | . | . | . | . | . | . | . | . | . | . | . | . | . | . | . | . | . | . |
| 21-225_33B2_HC | . | . | . | . | . | . | . | | . | . | . | . | . | . | . | . | . | . | . | . | . | . | . | . | . | . |
| 21-225_33C12_HC | . | . | . | . | . | . | . | | . | . | . | . | . | . | . | . | . | . | . | . | . | . | . | . | . | . |
| 21-225_34C2_HC | . | . | . | . | . | . | . | | . | . | . | . | . | . | . | . | . | . | . | . | . | . | . | . | . | . |
| 21-225_34D3_HC | . | . | . | . | . | . | . | | . | . | . | . | . | . | . | . | . | . | . | . | . | . | . | . | . | . |
| 21-225_34D8_HC | . | . | . | . | . | . | . | | . | . | . | . | . | . | . | . | . | . | . | . | . | . | . | . | . | . |
| 21-225_36B8_HC | . | . | . | . | . | . | . | | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | N | - | - | - | - | - | Y | G | M | H | W | V | R | Q | A | P | G | K | G | L |
| 21-225_30G1_HC | . | . | . | . | . | . | Y | | | | | | . | . | . | . | . | . | . | . | . | . | . | . | . | . |
| 21-225_30G4_HC | . | . | . | . | . | . | S | | | | | | . | . | . | . | . | . | . | . | . | . | . | . | . | . |
| 21-225_32D6_HC | . | . | . | . | . | . | S | | | | | | . | . | . | . | . | . | . | . | . | . | . | . | . | . |
| 21-225_33B2_HC | . | . | . | . | . | . | . | | | | | | . | . | . | . | . | . | . | . | . | . | . | . | . | . |
| 21-225_33C12_HC | . | . | . | . | . | . | . | | | | | | . | . | . | . | . | . | . | . | . | . | . | . | . | . |
| 21-225_34C2_HC | . | . | . | . | . | . | . | | | | | | . | V | . | . | . | . | . | . | . | . | . | . | . | . |
| 21-225_34D3_HC | . | . | . | . | . | . | Y | | | | | | . | . | . | . | . | . | . | . | . | . | . | . | . | . |
| 21-225_34D8_HC | . | . | . | . | . | . | . | | | | | | . | . | . | . | . | . | . | . | . | . | . | . | . | . |
| 21-225_36B8_HC | . | . | . | . | . | . | . | | | | | | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | H | D | - | - | - | G | S | N | K | Y | Y | A | D | S | V | K | G | R | F |
| 21-225_30G1_HC | . | . | . | . | . | . | . | . | . | . | . | . | . | . | . | . | . | . | V | . | . | . | . | . | . | . |
| 21-225_30G4_HC | . | . | . | . | . | . | . | Y | . | . | . | . | . | T | Q | . | . | . | V | . | . | . | . | . | . | . |
| 21-225_32D6_HC | . | . | T | . | . | . | . | . | . | . | . | . | . | . | . | A | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | Y | . | . | . | . | . | . | . | . | . | . | . | . | . | . | . | . | . | . |
| 21-225_34C2_HC | . | . | . | . | L | . | . | Y | . | . | . | . | . | . | . | . | . | . | . | . | . | . | . | . | . | . |
| 21-225_34D3_HC | . | . | . | . | . | . | . | . | . | . | . | . | . | . | . | . | . | . | V | . | . | . | . | . | . | . |
| 21-225_34D8_HC | . | . | . | . | . | . | . | Y | . | . | . | . | . | . | . | . | . | . | . | . | . | . | . | . | . | . |
| 21-225_36B8_HC | . | . | . | . | . | . | . | . | . | . | . | . | . | . | . | . | . | . | Q | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_30G1_HC | . | . | . | . | . | . | . | . | . | . | . | . | . | . | . | . | . | . | . | . | . | . | . | . | . | . |
| 21-225_30G4_HC | . | . | . | . | . | . | . | . | . | . | . | . | . | . | . | . | . | . | . | . | . | S | . | . | . | . |
| 21-225_32D6_HC | . | . | . | . | . | . | . | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | . | . | . | . | . | . | . | . | . | . | . | . | . | . | . | . | . | . | . |
| 21-225_34C2_HC | . | . | . | . | . | . | . | . | . | . | . | . | . | . | . | . | . | . | . | . | . | . | . | . | . | . |
| 21-225_34D3_HC | . | . | . | . | . | . | . | . | . | . | . | . | . | . | . | . | . | . | . | . | . | . | . | . | . | . |
| 21-225_34D8_HC | . | . | . | . | . | . | . | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
|  |  |  |  |  |  |  |  |  |  |  |  |  |  |  |  |  |  |  | H_CDR3 |  |  |  |  |  |  |  |
| CONSENSUS | Y | C | A | R | E | N | S | S | S | - | - | - | - | - | - | - | - | - | - | - | - | - | - | - | - | - |
| 21-225_30G1_HC | . | . | . | . | . | . | . | . | . | . | . | . | . | . | . | . | . | . | . | . | . | . | . | . | . | . |
| 21-225_30G4_HC | . | . | . | . | . | . | . | . | . | . | . | . | . | . | . | . | . | . | . | . | . | . | . | . | . | . |
| 21-225_32D6_HC | . | . | . | . | . | . | . | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | . | . | . | . | . | . | . | . | . | . | . | . | . | . | . | . | . | . | . |
| 21-225_34C2_HC | . | . | . | . | . | . | . | . | . | . | . | . | . | . | . | . | . | . | . | . | . | . | . | . | . | . |
| 21-225_34D3_HC | . | . | . | . | . | . | . | . | . | . | . | . | . | . | . | . | . | . | . | . | . | . | . | . | . | . |
| 21-225_34D8_HC | . | . | . | . | . | . | . | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  | H_FR4 |  |  |  |  |  |  |  |  |  |  |
| CONSENSUS | - | - | - | Y | Y | F | D | Y | W | G | Q | G | T | L | V | T | V | S | S |
| 21-225_30G1_HC | . | . | . | . | . | . | . | . | . | . | . | . | . | . | . | . | . | . | . |
| 21-225_30G4_HC | . | . | . | . | . | . | . | . | . | . | . | . | . | . | . | . | . | . | . |
| 21-225_32D6_HC | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . |
| 21-225_33C12_HC | . | . | . | . | . | . | . | . | . | . | . | . | . | . | . | . | . | . | . |
| 21-225_34C2_HC | . | . | . | . | . | . | . | . | . | . | . | . | . | . | . | . | . | . | . |
| 21-225_34D3_HC | . | . | . | F | . | . | . | . | . | . | . | . | . | . | . | . | . | . | . |
| 21-225_34D8_HC | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . |

**FIGURE 57**

Table 89.     Consensus 55 - VH2|2-05/D1|1-1|RF1/JH3 (SEQ ID NO: 50306):

QITLKES-GPTLVKPTQTLTLTCTFSG-FSLS<u>TS---GVGVG</u>WIRQPPGKALEWLA<u>LINW----</u>
<u>NDDKRYSPSLKS</u>RFTITRDTSKDQVVLTMTNMDPVDTATYYCAH<u>KATWV</u>--------------------AFDIWGQGTMVTVSS wherein:

Y in position 70 can be substituted with F

A in position 110 can be substituted with T

Thr Ser Gly Val Gly Val Gly (SEQ ID NO: 50110)

Leu Ile Asn Trp Asn Asp Asp Lys Arg Xaa Ser Pro Ser Leu Lys Ser (SEQ ID NO: 50111)

Lys Xaa Thr Trp Val Ala Phe Asp Ile (SEQ ID NO: 50112)

Thr Ser Gly Val Gly Val Gly (SEQ ID NO: 50588)

Leu Ile Asn Trp Asn Asp Asp Lys Arg Tyr Ser Pro Ser Leu Lys Ser (SEQ ID NO: 50589)

Lys Ala Thr Trp Val Ala Phe Asp Ile (SEQ ID NO: 50590)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | I | T | L | K | E | S | - | G | P | T | L | V | K | P | T | Q | T | L | T | L | T | C | T | F | S |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | S | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | S | L | S | T | S | - | - | - | G | V | G | V | G | W | I | R | Q | P | P | G | K | A | L |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | N | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | L | A | L | I | N | W | - | - | - | - | N | D | D | K | R | Y | S | P | S | L | K | S | R | F |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | T | R | D | T | S | K | D | Q | V | V | L | T | M | T | N | M | D | P | V | D | T | A | T | Y |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

## FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | H | K | A | T | W | V | - | - | - | - | - | - | - | - | - | - | - | - | - | - | - | - | - |
| 21-225_62A12_HC | . | . | . | . | . | Y | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | Y | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | A | F | D | I | W | G | Q | G | T | M | V | T | V | S | S |
| 21-225_62A12_HC | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . |

FIGURE 57

Table 90.    Consensus 56 - VH3|3-23/D4|4-11|RF3/JH4 (SEQ ID NO: 50307):

EVQLLES-GGGLVQPGGSLRLSCAASG-FTFS<u>S-----YVMN</u>WVRQAPGKGLEWVS<u>AISGS---</u>
<u>GGRTYY</u>ADSVKGRFTISRDNSKNTLYLQMNSLRAEDTAVYYCAR<u>TAT-----------------------FDY</u>WGQGTLVTVSS wherein:

V at position 40 can be substituted with A

M at position 41 can be substituted with I or L

N at position 42 can be substituted with S or R

A at position 57 can be substituted with D

I at position 58 can be substituted with M

G at position 66 can be substituted with D or V

R at position 67 can be substituted with S, F or T

A at position 71 can be substituted with V

A at position 110 can be substituted with G, S, T or Y

T at position 111 can be substituted with V, null (-), H, L or G

F at position 136 can be substituted with null (-) or K

D at position 137 can be substituted with null (-)

Y at position 138 can be substituted with L

Ser Tyr Xaa Xaa Xaa (SEQ ID NO: 50113)

Xaa Xaa Ser Gly Ser Gly Xaa Xaa Thr Tyr Tyr Xaa Asp Ser Val Lys Gly (SEQ ID NO: 50114)

Thr Xaa Xaa Xaa Xaa Xaa (SEQ ID NO: 50115)

Ser Tyr Val Met Asn (SEQ ID NO: 50591)

**FIGURE 57**

Ala Ile Ser Gly Ser Gly Gly Arg Thr Tyr Tyr Ala Asp Ser Val Lys Gly (SEQ ID NO: 50592)

Thr Ala Thr Phe Asp Tyr (SEQ ID NO: 50593)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_153A1_HC | . | . | . | . | . | . | . | | . | . | . | . | . | . | . | . | . | . | . | . | . | . | . | . | . | . |
| 21-225_157H1_HC | . | . | . | . | . | . | . | | . | . | . | . | . | . | . | . | . | . | . | . | . | . | . | . | . | . |
| 21-225_175C4_HC | . | . | . | . | . | . | . | | . | . | . | . | . | . | . | . | . | . | . | . | . | . | . | . | . | . |
| 21-225_23C7_HC | . | . | . | . | . | . | . | | . | . | . | . | . | . | . | . | . | . | . | . | . | . | . | . | . | . |
| 21-225_23E7_HC | . | . | . | . | . | . | . | | . | . | . | . | . | . | . | . | . | . | . | . | . | . | . | Y | . | . |
| 21-225_30A1_HC | . | . | . | . | . | . | . | | . | . | . | . | . | . | . | . | . | . | . | . | . | . | . | . | . | . |
| 21-225_33A5_HC | . | . | . | . | . | . | . | | . | . | . | . | . | . | . | . | . | . | . | . | . | . | . | . | . | . |
| 21-225_56F7_HC | . | . | . | . | . | . | . | | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | H_CDR1 | | | | | | | | | | | | | | | H_FR2 | | | |
| CONSENSUS | G | - | F | T | F | S | S | - | - | - | - | - | Y | V | M | N | W | V | R | Q | A | P | G | K | G | L |
| 21-225_153A1_HC | . | . | . | S | . | . | . | | | | | | . | . | . | S | . | . | . | . | Q | . | . | . | . | . |
| 21-225_157H1_HC | . | . | . | . | . | . | . | | | | | | . | A | . | N | . | I | . | . | . | . | . | . | . | . |
| 21-225_175C4_HC | A | . | . | . | . | . | . | | | | | | . | . | . | S | . | . | . | . | . | . | . | . | . | . |
| 21-225_23C7_HC | . | . | . | . | . | . | . | | | | | | . | . | . | S | . | . | . | . | . | . | . | . | . | . |
| 21-225_23E7_HC | . | . | . | . | . | . | . | | | | | | . | A | . | . | . | . | . | . | . | . | . | . | . | . |
| 21-225_30A1_HC | . | . | . | . | . | . | . | | | | | | . | . | . | . | . | . | . | . | . | . | . | . | . | . |
| 21-225_33A5_HC | . | . | . | . | . | . | . | | | | | | . | . | . | I | . | . | . | . | . | . | . | . | . | . |
| 21-225_56F7_HC | . | . | . | . | . | . | . | | | | | | . | . | . | L | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | A | I | S | G | S | - | - | - | G | G | T | Y | Y | A | D | S | V | K | G | R | F |
| 21-225_153A1_HC | . | . | . | . | . | . | . | . | . | . | . | . | . | D | N | . | . | . | . | . | . | . | . | . | . | . |
| 21-225_157H1_HC | Q | . | . | . | D | . | . | . | . | . | . | . | . | . | T | . | . | . | . | . | . | . | . | . | . | . |
| 21-225_175C4_HC | . | . | . | . | . | . | . | . | . | . | . | . | . | D | N | . | . | . | . | . | . | . | . | . | . | . |
| 21-225_23C7_HC | . | . | . | . | M | . | . | . | . | . | . | . | . | . | S | . | . | . | V | . | . | . | . | . | . | . |
| 21-225_23E7_HC | . | . | . | . | . | . | . | . | . | . | . | . | . | . | F | . | . | . | . | . | . | . | . | . | . | . |
| 21-225_30A1_HC | . | . | . | . | . | . | . | . | . | . | . | . | . | . | S | . | . | . | . | . | . | . | . | . | . | . |
| 21-225_33A5_HC | . | . | . | . | . | . | . | . | . | . | . | . | . | V | S | . | . | . | . | . | . | . | . | . | . | . |
| 21-225_56F7_HC | . | . | . | . | M | . | . | . | . | . | . | . | . | . | N | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | | H_FR3 | | | | | | | | | | | |
| CONSENSUS | T | I | S | R | D | N | S | K | N | T | L | Y | L | Q | M | N | S | L | R | A | E | D | T | A | V | Y |
| 21-225_153A1_HC | . | . | . | . | . | . | . | . | . | . | . | . | . | . | . | . | . | . | . | . | . | . | . | . | . | . |
| 21-225_157H1_HC | . | . | . | . | . | . | . | . | . | . | . | . | . | . | . | . | . | . | . | . | . | . | . | . | . | . |
| 21-225_175C4_HC | . | . | . | . | D | . | N | T | . | . | . | . | . | . | . | . | . | . | . | . | . | . | . | . | . | . |
| 21-225_23C7_HC | . | . | . | . | . | . | . | . | . | . | . | . | . | . | . | . | . | . | . | . | . | . | . | . | . | . |
| 21-225_23E7_HC | . | . | . | . | . | . | R | . | . | . | . | . | . | . | . | . | . | . | . | . | . | . | . | . | . | . |
| 21-225_30A1_HC | . | . | . | . | N | . | . | . | . | . | . | . | . | . | . | . | . | . | . | . | . | . | . | . | . | . |
| 21-225_33A5_HC | . | . | . | . | . | . | . | . | . | . | . | . | . | . | . | . | . | . | . | . | . | . | . | . | . | . |
| 21-225_56F7_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | T | | | - | - | - | - | - | - | - | - | - | - | - | - | - | - | - | - | - | - | - |
| 21-225_153A1_HC | . | . | . | . | . | A | T | . | . | . | . | . | . | . | . | . | . | . | . | . | . | . | . | . | . | . |
| 21-225_157H1_HC | . | . | . | K | . | Y | - | . | . | . | . | . | . | . | . | . | . | . | . | . | . | . | . | . | . | . |
| 21-225_175C4_HC | . | . | . | . | . | A | G | . | . | . | . | . | . | . | . | . | . | . | . | . | . | . | . | . | . | . |
| 21-225_23C7_HC | . | . | . | . | . | G | V | . | . | . | . | . | . | . | . | . | . | . | . | . | . | . | . | . | . | . |
| 21-225_23E7_HC | . | . | . | . | . | S | G | . | . | . | . | . | . | . | . | . | . | . | . | . | . | . | . | . | . | . |
| 21-225_30A1_HC | . | . | . | . | . | G | V | . | . | . | . | . | . | . | . | . | . | . | . | . | . | . | . | . | . | . |
| 21-225_33A5_HC | . | . | . | . | . | S | L | . | . | . | . | . | . | . | . | . | . | . | . | . | . | . | . | . | . | . |
| 21-225_56F7_HC | F | . | . | M | . | T | H | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | H_FR4 | | | | | | | | | |
| CONSENSUS | - | - | - | - | - | F | D | Y | W | G | Q | G | T | L | V | T | V | S | S |
| 21-225_153A1_HC | . | . | . | . | . | K | . | . | . | . | . | . | . | . | . | . | . | . | . |
| 21-225_157H1_HC | . | . | . | . | . | - | - | L | . | . | . | . | . | . | . | . | . | . | . |
| 21-225_175C4_HC | . | . | . | . | . | . | . | . | . | . | . | . | . | . | . | . | . | . | . |
| 21-225_23C7_HC | . | . | . | . | . | . | . | . | . | . | . | . | . | . | . | I | . | . | . |
| 21-225_23E7_HC | . | . | . | . | . | . | . | . | . | . | . | . | . | . | . | . | . | . | . |
| 21-225_30A1_HC | . | . | . | . | . | . | . | . | . | . | . | . | . | . | . | . | . | . | . |
| 21-225_33A5_HC | . | . | . | . | . | . | . | . | . | . | . | . | . | . | . | . | . | . | . |
| 21-225_56F7_HC | . | . | . | . | . | . | . | . | . | . | . | . | . | . | . | . | . | . | . |

**FIGURE 57**

Table 91.        Consensus 57 -VH3|3-23/D7|7-27|RF1/JH3 (SEQ ID NO: 50308):

EVQLLES-GGGLVQPGGSLRLSCAASG-FTFS<u>S-----YAMS</u>WVRQAPGKGLEWVS<u>VISGR---</u>
<u>GGTTFYADSVKG</u>RFTISRDNSKNTLYLQMNSLRAEDTAVYYCA<u>KRTPSD</u>--------------------<u>VFDI</u>WGQGTMVTVSS wherein:

Y at position 39 can be substituted with F

S at position 42 can be substituted with N

V at position 57 can be substituted with A or I

I at position 58 can be substituted with L

R at position 61 can be substituted with S or G

G at position 66 can be substituted with S or K

T at position 67 can be substituted with N or S

F at position 69 can be substituted with Y

P at position 111 can be substituted with G

S at position 112 can be substituted with D or E

V at position 135 can be substituted with A

I at position 138 can be substituted with V

Ser Xaa Ala Met Xaa (SEQ ID NO: 50116)

Xaa Xaa Ser Gly Xaa Gly Xaa Xaa Thr Xaa Tyr Ala Asp Ser Val Lys Gly (SEQ ID NO: 50117)

Lys Arg Thr Xaa Xaa Asp Xaa Phe Asp Xaa (SEQ ID NO: 50118)

Ser Tyr Ala Met Ser (SEQ ID NO: 50594)

Val Ile Ser Gly Arg Gly Gly Thr Thr Phe Tyr Ala Asp Ser Val Lys Gly (SEQ ID NO: 50595)

FIGURE 57

Lys Arg Thr Pro Ser Asp Val Phe Asp Ile (SEQ ID NO: 50596)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | L | E | S | - | G | G | G | L | V | Q | P | G | G | S | L | R | L | S | C | A | A | S |
| 21-225_149B6_HC | . | . | . | . | . | . | . | . | . | . | . | . | . | . | . | . | . | . | . | . | . | . | . | . | . | . |
| 21-225_180A3_HC | . | . | . | . | . | . | . | . | . | . | . | . | K | . | . | . | . | . | . | . | . | . | . | . | . | . |
| 21-225_182H5_HC | . | . | . | . | . | . | . | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . | . | . | . | . | . | . | . |
| 21-225_43H9_HC | . | . | . | . | . | . | . | . | . | . | . | . | . | . | . | . | . | . | . | . | . | . | . | . | . | . |
| 21-225_44F3_HC | . | . | K | . | . | . | . | . | . | . | . | . | . | . | . | . | . | . | . | . | . | . | . | . | . | . |
| 21-225_45C9_HC | . | . | . | . | . | . | . | . | . | . | . | . | . | . | . | . | . | . | . | . | . | . | . | . | . | . |
| 21-225_45H4_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | H_CDR1 | | | | | | | | | | | | | | | | H_FR2 | | |
| CONSENSUS | G | - | F | T | F | S | S | - | - | - | - | - | Y | A | M | S | W | V | R | Q | A | P | G | K | G | L |
| 21-225_149B6_HC | . | . | . | . | . | . | . | . | . | . | . | . | . | . | . | . | . | . | . | . | . | . | . | . | . | . |
| 21-225_180A3_HC | . | . | . | . | . | . | . | . | . | . | . | . | N | . | . | N | . | . | . | . | . | . | . | . | . | . |
| 21-225_182H5_HC | . | . | . | . | . | . | . | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . | . | . | . | . | . | . | . |
| 21-225_43H9_HC | . | . | . | . | . | . | . | . | . | . | . | . | . | . | . | . | . | . | . | . | . | . | . | N | . | . |
| 21-225_44F3_HC | . | . | . | . | . | . | . | . | . | . | . | . | . | . | . | . | . | . | . | . | . | . | . | . | . | . |
| 21-225_45C9_HC | . | . | . | . | . | N | . | . | . | . | . | . | . | . | . | . | . | . | . | . | . | . | . | . | . | . |
| 21-225_45H4_HC | . | . | . | . | . | . | . | . | . | . | . | . | . | . | . | . | . | . | . | . | . | . | . | N | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | V | I | S | G | R | - | - | - | G | G | T | T | F | Y | A | D | S | V | K | G | R | F |
| 21-225_149B6_HC | . | | | . | . | . | . | . | S | . | . | . | . | . | S | | . | . | . | . | . | . | . | . | . | . |
| 21-225_180A3_HC | . | | | . | . | . | . | . | . | . | . | . | . | . | N | | . | . | . | . | . | . | . | . | . | . |
| 21-225_182H5_HC | . | | | . | . | . | . | . | . | . | . | . | . | . | . | | . | . | . | . | . | . | . | . | . | . |
| 21-225_186A11_HC | . | | | . | . | . | . | . | . | . | . | . | . | . | . | | . | . | . | . | . | . | . | . | . | . |
| 21-225_43H9_HC | . | | | . | A | . | . | . | S | . | . | . | . | S | N | | . | . | . | . | . | . | . | . | . | . |
| 21-225_44F3_HC | . | | | I | L | . | . | . | G | . | . | . | . | X | . | | Y | . | . | . | . | . | . | . | . | . |
| 21-225_45C9_HC | . | | | . | . | . | . | . | . | . | . | . | . | . | . | | Y | . | . | . | . | . | . | . | . | . |
| 21-225_45H4_HC | . | | | A | . | . | . | . | S | . | . | . | . | S | N | | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_149B6_HC | . | . | . | . | . | . | . | . | . | . | . | F | . | . | . | . | . | . | . | . | . | . | . | . | . | . |
| 21-225_180A3_HC | . | . | . | . | . | . | . | . | . | . | . | F | . | . | . | . | . | . | . | . | . | . | . | . | . | . |
| 21-225_182H5_HC | . | . | . | . | . | . | . | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . | . | . | . | . | . | . | . |
| 21-225_43H9_HC | . | . | . | . | . | . | . | . | . | . | . | . | . | M | . | . | . | . | . | . | . | . | . | . | . | . |
| 21-225_44F3_HC | . | . | . | . | . | . | . | . | . | . | . | F | . | . | . | . | . | . | . | . | . | . | . | . | . | . |
| 21-225_45C9_HC | . | . | . | . | . | . | . | . | . | . | . | . | . | . | . | . | . | . | . | . | . | . | . | . | . | . |
| 21-225_45H4_HC | . | . | . | . | . | . | . | . | . | . | . | . | . | M | . | . | . | . | . | . | . | . | . | . | . | . |

EP 4 435 105 A2

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | K | R | T | P | S | D | - | - | - | - | - | - | - | - | - | - | - | - | - | - | - | - | - |
| 21-225_149B6_HC | . | | | | . | . | . | E | . | . | . | . | . | . | . | . | . | . | . | . | . | . | . | . | . | . |
| 21-225_180A3_HC | P | | | | . | . | Q | D | . | . | . | . | . | . | . | . | . | . | . | . | . | . | . | . | . | . |
| 21-225_182H5_HC | . | | | | . | . | Q | D | . | . | . | . | . | . | . | . | . | . | . | . | . | . | . | . | . | . |
| 21-225_186A11_HC | . | . | | | . | . | Q | D | . | . | . | . | . | . | . | . | . | . | . | . | . | . | . | . | . | . |
| 21-225_43H9_HC | P | | | | . | . | . | . | . | . | . | . | . | . | . | . | . | . | . | . | . | . | . | . | . | . |
| 21-225_44F3_HC | . | | | | . | . | . | . | . | . | . | . | . | . | . | . | . | . | . | . | . | . | . | . | . | . |
| 21-225_45C9_HC | . | | | | . | . | . | . | . | . | . | . | . | . | . | . | . | . | . | . | . | . | . | . | . | . |
| 21-225_45H4_HC | P | | | | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | V | F | D | I | W | G | Q | G | T | M | V | T | V | S | S |
| 21-225_149B6_HC | . | . | . | . | . | . | . | . | . | . | . | . | . | . | . | . | . | . | . |
| 21-225_180A3_HC | . | . | . | . | . | . | . | V | . | . | . | . | . | . | . | . | . | . | . |
| 21-225_182H5_HC | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . |
| 21-225_43H9_HC | . | . | . | . | . | . | . | . | . | . | . | . | . | . | . | . | . | . | . |
| 21-225_44F3_HC | . | . | . | . | A | . | . | . | . | . | . | . | . | . | . | . | . | . | . |
| 21-225_45C9_HC | . | . | . | . | A | . | . | . | . | . | . | . | . | . | . | . | . | . | . |
| 21-225_45H4_HC | . | . | . | . | . | . | . | . | . | . | . | . | . | . | . | . | . | . | . |

**FIGURE 57**

Table 92.      Consensus 58 -VH3|3-33/D4|4-11|RF2/JH4 (SEQ ID NO: 50309):

QVQLVES-GGGVVQPGRSLRLSCAASG-FTFSS-----YGMHWVRQAPGKGLEWVAVIWYD---
GSNKYYADSVKGRFTISRDNSKNTLYLQMNSLRAEDTAVYYCARDRPRS------------------SAF DYWGQGTLVTVSS wherein:

S at position 33 can be substituted with N or T

Y at position 39 can be substituted with F

G at position 40 can be substituted with D or N

V at position 57 can be substituted with A

Y at position 60 can be substituted with H

S at position 66 can be substituted with R

N at position 67 can be substituted with D or H

K at position 68 can be substituted with R

Y at position 70 can be substituted with C or S

A at position 71 can be substituted with E or T

R at position 110 can be substituted with D or H

P at position 111 can be substituted with S or A

I at position 112 can be substituted with R or Y

S at position 113 can be substituted with L, V or W

Null (-) at position 114 can be substituted with G

**FIGURE 57**

Null (-) at position 133 can be substituted with A

S at position 134 can be substituted with T or A

A at position 135 can be substituted with F, S or Y

F at position 136 can be substituted with G or S

Y at position 138 can be substituted with F

Xaa Xaa Xaa Met His (SEQ ID NO: 50237)

Xaa Ile Trp Xaa Asp Gly Xaa Xaa Xaa Tyr Xaa Xaa Asp Ser Val Lys Gly (SEQ ID NO: 50238)

Asp Xaa Xaa Xaa Xaa Xaa Xaa Xaa Xaa Xaa Asp Xaa (SEQ ID NO: 50239)

Ser Tyr Gly Met His (SEQ ID NO: 50597)

Val Ile Trp Tyr Asp Gly Ser Asn Lys Tyr Tyr Ala Asp Ser Val Lys Gly (SEQ ID NO: 50598)

Asp Arg Pro Arg Ser Ser Ala Phe Asp Tyr (SEQ ID NO: 50599)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | V | E | S | - | G | G | G | V | V | Q | P | G | R | S | L | R | L | S | C | A | A | S |
| 21-225_180F8_HC | . | . | . | . | . | . | . | | . | . | . | . | . | . | | . | . | . | . | . | . | . | . | . | . | . |
| 21-225_19B3_HC | . | . | . | . | . | . | . | | . | . | . | . | . | . | | . | . | . | . | . | . | . | . | . | . | . |
| 21-225_212C10_HC | . | . | . | . | . | . | . | | . | . | . | . | . | . | | . | . | . | . | . | . | . | . | . | . | . |
| 21-225_63C10_HC | . | . | . | . | . | . | . | | . | . | . | . | . | . | | . | . | . | . | . | . | . | . | . | . | . |
| 21-225_70H6_HC | . | . | . | . | . | . | . | | . | . | . | . | . | . | | . | . | . | . | . | . | . | . | . | . | . |
| 21-225_74C3_HC | . | . | . | . | . | . | . | | . | . | . | . | . | . | | . | . | . | . | . | . | . | . | . | . | . |
| 21-225_77H6_HC | . | . | . | . | . | . | . | | . | . | . | . | . | . | | . | . | . | . | . | . | . | . | . | . | . |
| 21-225_8H11_HC | . | . | . | . | . | . | . | | . | . | . | . | . | . | | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | H_FR2 | | | |
| CONSENSUS | G | - | F | T | F | S | S | - | - | - | - | - | Y | G | M | H | W | V | R | Q | A | P | G | K | G | L |
| 21-225_180F8_HC | . | . | . | . | . | . | . | | . | . | . | . | . | . | . | . | . | . | . | . | . | . | . | . | . | . |
| 21-225_19B3_HC | . | . | . | . | . | . | . | | . | . | . | . | D | . | . | . | . | . | . | . | . | . | . | . | . | . |
| 21-225_212C10_HC | . | . | . | . | . | . | . | | . | . | . | . | . | . | . | . | . | . | . | . | . | . | . | . | . | . |
| 21-225_63C10_HC | . | . | . | . | . | . | . | | . | . | . | . | . | . | . | . | . | . | . | . | . | . | . | . | . | . |
| 21-225_70H6_HC | . | . | . | . | . | . | . | | . | . | . | . | . | . | . | . | . | . | . | . | T | . | . | . | . | . |
| 21-225_74C3_HC | . | . | . | . | . | . | . | | . | . | S | . | . | . | . | . | A | . | . | . | . | . | . | . | . | . |
| 21-225_77H6_HC | . | . | . | . | . | N | . | | . | . | . | . | . | . | . | . | . | . | . | . | . | . | . | . | . | . |
| 21-225_8H11_HC | . | . | . | . | . | Y | . | | . | . | . | . | N | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | G | S | N | K | Y | Y | A | D | S | V | K | G | R | F |
| 21-225_180F8_HC | . | . | . | S | . | . | . | . | . | . | . | . | . | . | D | . | . | . | E | . | . | . | . | . | . | . |
| 21-225_19B3_HC | . | . | . | . | . | . | . | . | . | . | . | . | . | . | D | . | . | . | . | . | . | . | . | . | . | . |
| 21-225_212C10_HC | . | . | . | Y | A | . | . | . | . | . | . | . | . | . | . | . | . | . | T | . | . | . | . | . | . | . |
| 21-225_63C10_HC | D | . | . | . | . | . | . | . | . | . | . | . | . | . | . | . | . | . | . | . | . | . | . | . | . | . |
| 21-225_70H6_HC | D | . | . | . | . | . | . | . | . | . | . | . | . | . | . | . | . | . | . | . | . | . | . | . | . | . |
| 21-225_74C3_HC | . | . | . | A | . | . | . | . | . | . | . | . | . | . | . | . | . | C | . | . | . | . | . | . | . | . |
| 21-225_77H5_HC | . | . | . | . | . | . | . | . | . | . | . | . | . | . | . | . | . | . | . | . | . | . | . | . | . | . |
| 21-225_8H11_HC | . | . | . | . | . | . | . | . | . | . | . | . | . | . | D | . | . | S | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | H_FR3 | | | | | | | | | | | | | | |
| CONSENSUS | T | I | S | R | D | N | S | K | N | T | L | Y | L | Q | M | N | S | L | R | A | E | D | T | A | V | Y |
| 21-225_180F8_HC | . | . | . | . | . | . | . | . | . | . | . | . | . | . | . | . | . | . | . | . | . | . | . | . | . | . |
| 21-225_19B3_HC | . | . | . | . | . | . | . | . | . | . | . | F | . | . | . | . | . | . | . | . | . | . | . | . | . | . |
| 21-225_212C10_HC | . | . | . | . | . | . | . | . | . | . | . | . | . | . | . | . | . | . | . | . | . | . | . | . | . | . |
| 21-225_63C10_HC | . | . | . | . | . | . | . | . | . | . | . | . | . | . | . | . | . | . | . | . | . | . | . | . | . | . |
| 21-225_70H6_HC | . | . | . | . | . | . | . | . | . | . | . | . | . | . | . | . | . | . | . | . | . | . | . | . | . | . |
| 21-225_74C3_HC | . | . | . | . | . | . | . | . | . | . | . | S | . | . | . | . | . | . | . | . | . | . | . | S | . | . |
| 21-225_77H5_HC | . | . | . | . | . | . | . | . | . | . | . | . | . | . | . | . | . | . | . | . | . | . | . | . | L | . |
| 21-225_8H11_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | H_CDR3 | | | | | | | | |
| CONSENSUS | Y | C | A | R | D | R | | | | S | - | - | - | - | - | - | - | - | - | - | - | - | - | - | - | - |
| 21-225_180F8_HC | | | | | | X | P | X | W | . | . | . | . | . | . | . | . | . | . | . | . | . | . | . | . | . |
| 21-225_19B3_HC | | | | | | . | A | Y | . | . | . | . | . | . | . | . | . | . | . | . | . | . | . | . | . | . |
| 21-225_212C10_HC | | | | | | . | S | I | V | Q | . | . | . | . | . | . | . | . | . | . | . | . | . | . | . | . |
| 21-225_63C10_HC | | | | | | D | P | X | . | . | . | . | . | . | . | . | . | . | . | . | . | . | . | . | . | . |
| 21-225_70H6_HC | | | | | | D | P | X | . | . | . | . | . | . | . | . | . | . | . | . | . | . | . | . | . | . |
| 21-225_74C3_HC | | | | | | . | S | I | L | Q | . | . | . | . | . | . | . | . | . | . | . | . | . | . | . | . |
| 21-225_77H5_HC | | | | | | . | S | I | L | Q | . | . | . | . | . | . | . | . | . | . | . | . | . | . | . | . |
| 21-225_8H11_HC | | | | | | . | A | Y | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | H_FR4 | | | | | | | | | | |
| CONSENSUS | - | - | - | S | | | D | Y | W | G | Q | G | T | L | V | T | V | S | S |
| 21-225_180F8_HC | . | . | . | . | Y | Q | . | . | . | . | . | . | . | . | . | . | . | . | . |
| 21-225_19B3_HC | . | . | . | . | S | S | . | . | . | . | . | . | . | . | . | . | . | . | . |
| 21-225_212C10_HC | . | . | A | T | Y | F | . | . | . | . | . | . | . | . | . | . | . | . | . |
| 21-225_63C10_HC | . | . | . | . | A | S | . | . | . | . | . | . | . | . | . | . | . | . | . |
| 21-225_70H6_HC | . | . | . | . | A | S | . | . | . | . | . | . | . | . | . | . | . | . | . |
| 21-225_74C3_HC | . | . | A | A | F | F | . | . | . | . | . | . | . | . | . | . | . | . | . |
| 21-225_77H5_HC | . | . | A | T | F | F | . | . | . | . | . | . | . | . | . | . | . | . | . |
| 21-225_8H11_HC | . | . | . | . | S | S | . | F | . | . | . | . | . | . | . | . | . | . | . |

**FIGURE 57**

Table 93. Consensus 59 - VH3|3-48/D4|4-11|RF2/JH6 (SEQ ID NO: 50310):

EVQLVES-GGGLVQPGGSLRLSCAASG-FTFSS-----YNMNWVRQAPGKGLEWVSYISRS---SNTKYYADSVKGRFTISRDNAKNSLYLQMNSLRDEDTAVYYCARDRSGSYGY-------------FYYYGLDVWGQGTTVTVSS wherein:

S at position 33 can be substituted with N

N at position 40 can be substituted with S

R at position 60 can be substituted with S

S at position 65 can be substituted with G

N at position 66 can be substituted with S

K at position 68 can be substituted with T

Y at position 69 can be substituted with H

A at position 71 can be substituted with V

K at position 75 can be substituted with R, E or Q

R at position 110 can be substituted with S

S at position 111 can be substituted with R

G at position 112 can be substituted with K

S at position 113 can be substituted with G

Y at position 114 can be substituted with F

G at position 115 can be substituted with null (-)

Y at position 116 can be substituted with null (-)

FIGURE 57

F at position 131 can be substituted with null (-)

Y at position 132 can be substituted with null (-)

L at position 136 can be substituted with M

Xaa Tyr Xaa Met Asn (SEQ ID NO: 50240)

Tyr Ile Ser Xaa Ser Xaa Xaa Thr Xaa Xaa Tyr Xaa Asp Ser Val Xaa Gly (SEQ ID NO: 50241)

Asp Xaa Xaa Xaa Xaa Xaa Xaa Xaa Xaa Xaa Tyr Tyr Gly Xaa Asp Val (SEQ ID NO: 50242)

Ser Tyr Asn Met Asn (SEQ ID NO: 50600)

Tyr Ile Ser Arg Ser Ser Asn Thr Lys Tyr Tyr Ala Asp Ser Val Lys Gly (SEQ ID NO: 50601)

Asp Arg Ser Gly Ser Tyr Gly Tyr Phe Tyr Tyr Tyr Gly Leu Asp Val (SEQ ID NO: 50602)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | E | V | Q | L | V | E | S | - | G | G | G | L | V | Q | P | G | G | S | L | R | L | S | C | A | A | S |
| 21-225_146A8_HC | | | R | . | . | . | . | . | . | . | . | . | . | . | . | E | . | . | . | . | . | . | . | V | . | . |
| 21-225_146B6_HC | . | . | . | . | . | . | . | . | . | . | . | . | . | . | . | . | . | . | . | . | . | . | . | . | . | . |
| 21-225_146D8_HC | . | . | . | . | . | . | . | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . | . | . | . | . | . | . | . |
| 21-225_150F9_HC | M | . | . | . | . | . | . | . | . | . | . | . | . | . | . | . | . | . | . | . | . | . | . | . | . | . |
| 21-225_156H2_HC | . | . | . | . | . | . | . | . | . | . | . | . | I | . | . | . | . | . | . | . | . | . | . | . | . | . |
| 21-225_170E4_HC | . | . | . | . | . | . | . | . | . | . | S | . | . | . | . | . | . | . | . | . | . | . | . | . | . | . |
| 21-225_179D10_HC | . | . | . | . | . | . | . | . | . | . | . | . | . | . | . | . | . | . | . | . | N | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | N | M | N | W | V | R | Q | A | P | G | K | G | L |
| 21-225_146A8_HC | . | . | . | . | . | . | N | . | . | . | . | . | . | . | . | . | . | . | . | . | . | . | . | . | . | . |
| 21-225_146B6_HC | . | . | . | . | . | . | . | . | . | . | . | . | . | . | . | . | . | . | . | . | . | . | . | . | . | . |
| 21-225_146D8_HC | . | . | . | . | . | . | N | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . | . | . | . | . | . | . | . |
| 21-225_150F9_HC | . | . | . | . | . | . | . | . | . | . | . | . | . | . | . | . | . | . | . | . | V | . | . | . | . | . |
| 21-225_156H2_HC | . | . | . | . | . | . | N | . | . | . | . | . | . | . | . | . | . | . | . | . | . | . | . | . | . | . |
| 21-225_170E4_HC | . | . | . | . | . | . | . | . | . | . | . | . | S | . | . | . | . | . | . | . | . | . | . | . | . | . |
| 21-225_179D10_HC | . | . | . | . | . | . | . | . | . | . | . | . | S | . | . | . | . | . | . | . | . | . | . | . | . | . |

## FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
|  |  |  |  |  |  |  |  |  |  |  |  |  |  | H_CDR2 |  |  |  |  |  |  |  |  |  |  |  |  |
| CONSENSUS | E | W | V | S | Y | I | S | R | S | - | - | - | S | N | T | K | Y | Y | A | D | S | V | K | G | R | F |
| 21-225_146A8_HC | . | . | . | . | . | . | . | . | . | . | . | . | . | . | . | . | . | . | . | . | . | . | R | . | . | . |
| 21-225_146B6_HC | . | . | . | . | . | . | . | . | . | . | . | . | . | . | . | . | H | . | . | . | . | . | . | . | . | . |
| 21-225_146D8_HC | . | . | . | . | . | . | . | . | . | . | . | . | . | . | . | . | . | . | V | . | . | . | . | . | . | . |
| 21-225_149A1_HC | . | . | . | . | . | . | . | . | . | . | . | . | . | . | . | . | H | . | . | . | . | . | . | . | . | . |
| 21-225_150F9_HC | . | . | . | . | . | . | . | . | . | . | . | . | . | . | . | . | H | . | . | . | . | . | . | . | . | . |
| 21-225_156H2_HC | . | . | . | . | . | . | . | . | . | . | . | . | . | . | . | . | . | . | . | . | . | . | . | . | . | . |
| 21-225_170E4_HC | . | . | . | . | . | . | . | S | . | . | . | . | G | S | . | . | . | . | . | . | . | . | S | . | . | . |
| 21-225_179D10_HC | . | . | . | A | . | . | . | S | . | . | . | . | G | S | . | Y | . | . | . | . | . | . | Q | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
|  |  |  |  |  |  |  |  |  |  |  |  |  |  | H_FR3 |  |  |  |  |  |  |  |  |  |  |  |  |
| CONSENSUS | T | I | S | R | D | N | A | K | N | S | L | Y | L | Q | M | N | S | L | R | D | E | D | T | A | V | Y |
| 21-225_146A8_HC | . | . | . | . | . | . | . | . | . | . | . | . | . | . | . | . | . | . | . | . | . | . | . | . | . | . |
| 21-225_146B6_HC | . | . | . | . | . | . | . | . | . | . | . | . | . | . | . | D | . | . | . | . | . | . | . | . | . | . |
| 21-225_146D8_HC | . | . | . | . | . | . | . | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . | . | . | . | . | . | . | . |
| 21-225_150F9_HC | . | . | . | . | . | . | . | . | . | . | . | . | . | . | . | . | . | . | . | . | . | . | . | . | . | . |
| 21-225_156H2_HC | . | . | . | . | . | . | . | . | . | . | . | . | . | . | . | . | . | . | . | . | . | . | . | . | . | . |
| 21-225_170E4_HC | . | . | . | . | . | . | . | . | . | . | . | D | . | . | . | . | . | . | . | . | . | . | . | . | . | . |
| 21-225_179D10_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | S | G | S | Y | G | Y | - | - | - | - | - | - | - | - | - | - | - | - | - | - |
| 21-225_146A8_HC | | | | | | | | | | | | | | | | | | | | | | | | | | |
| 21-225_146B6_HC | | | | | | | | | | | | | | | | | | | | | | | | | | |
| 21-225_146D8_HC | | | | | | | | | | | | | | | | | | | | | | | | | | |
| 21-225_149A1_HC | | | | | | | | | | | | | | | | | | | | | | | | | | |
| 21-225_150F9_HC | | | | | | | | | | | | | | | | | | | | | | | | | | |
| 21-225_156H2_HC | | | | | | | | | | | | | | | | | | | | | | | | | | |
| 21-225_170E4_HC | | | | | | S | R | K | Q | F | - | - | | | | | | | | | | | | | | |
| 21-225_179D10_HC | | | | | | S | R | K | Q | F | - | - | | | | | | | | | | | | | | |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | F | Y | Y | Y | G | L | D | V | W | G | Q | G | T | T | V | T | V | S | S |
| 21-225_146A8_HC | | | | | | | | | | | | | | | | | | | |
| 21-225_146B6_HC | | | | | | | | | | | | | | | | | | | |
| 21-225_146D8_HC | | | | | | | | | | | | | | | | | | | |
| 21-225_149A1_HC | | | | | | | | | | | | | | | | | | | |
| 21-225_150F9_HC | | | | | | | | | | | | | | | | | | | |
| 21-225_156H2_HC | | | | | | | | | | | | | | | | | | | |
| 21-225_170E4_HC | - | - | | | | | | | | | | | | | | | | | |
| 21-225_179D10_HC | - | - | | | | | | | | | | | | | | | | | |

**FIGURE 57**

Table 94.       Consensus 60 - VH4|4-30.1/D4|4-11|RF2/JH6 (SEQ ID NO: 50311):

QVQLQES-GPGLVKPSQTLSLTCTVSG-GSIR<u>SG---GDYWS</u>WIRQHPGKGLEWIG<u>YIYY----
SGSTYYNPSLKS</u>RVTISVDTSKNQFSLKLSSVTAADTAVYYCARD<u>SSSY</u>--------------------GMDVWGQGTTVTVSS wherein:

S at position 67 can be substituted with I or P

S at position 110 can be substituted with G or H

S at position 111 can be substituted with A

S at position 112 can be substituted with L or R

Y at position 113 can be substituted with R or H

Ser Gly Gly Asp Tyr Trp Ser (SEQ ID NO: 50119)

Tyr Ile Tyr Tyr Ser Gly Xaa Thr Tyr Tyr Asn Pro Ser Leu Lys Ser (SEQ ID NO: 50120)

Asp Xaa Xaa Xaa Xaa Gly Met Asp Val (SEQ ID NO: 50121)

Ser Gly Gly Asp Tyr Trp Ser (SEQ ID NO: 50603)

Tyr Ile Tyr Tyr Ser Gly Ser Thr Tyr Tyr Asn Pro Ser Leu Lys Ser (SEQ ID NO: 50604)

Asp Ser Ser Ser Tyr Gly Met Asp Val (SEQ ID NO: 50605)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | H_FR1 | | | | | | | | | | |
| CONSENSUS | Q | V | Q | L | Q | E | S | - | G | P | G | L | V | K | P | S | Q | T | L | S | L | T | C | T | V | S |
| 21-225_190F10_HC | . | . | . | . | . | . | . | | . | . | . | . | . | . | . | . | . | . | . | . | . | . | . | . | . | . |
| 21-225_191A2_HC | . | . | . | . | . | . | . | | . | . | . | . | . | . | . | . | . | . | . | . | . | . | . | . | . | . |
| 21-225_192H8_HC | . | . | . | . | . | . | . | | . | . | V | . | . | . | . | . | . | . | . | . | . | . | . | . | . | . |
| 21-225_194D12_HC | . | . | . | . | . | . | . | | . | . | . | . | . | . | . | . | . | . | . | . | . | . | . | . | . | . |
| 21-225_194G5_HC | . | . | . | . | . | . | . | | . | . | . | . | . | . | . | . | . | . | . | . | . | . | . | ? | . | . |
| 21-225_198B8_HC | . | . | . | . | . | . | . | | . | . | . | . | . | . | . | . | . | . | . | . | . | . | . | . | . | . |
| 21-225_212H12_HC | . | . | . | . | . | . | . | | . | . | . | . | . | . | . | . | . | . | . | . | . | . | . | . | . | . |
| 21-225_214H3_HC | . | . | . | . | . | . | . | | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | | | | | | | | H_FR2 | | |
| CONSENSUS | G | - | G | S | I | R | S | G | - | - | - | G | D | Y | W | S | W | I | R | Q | H | P | G | K | G | L |
| 21-225_190F10_HC | . | | . | . | . | . | . | . | | | | . | . | . | . | . | . | . | . | . | . | . | . | R | . | . |
| 21-225_191A2_HC | . | | . | . | . | . | . | . | | | | . | . | . | . | . | . | . | . | . | . | . | . | R | . | . |
| 21-225_192H8_HC | . | | . | . | . | S | . | . | | | | . | . | . | . | . | . | . | . | . | . | . | . | . | . | . |
| 21-225_194D12_HC | . | | . | . | . | S | . | . | | | | . | . | . | . | . | . | . | . | . | . | . | . | . | . | . |
| 21-225_194G5_HC | . | | . | . | . | . | . | . | | | | . | . | . | . | . | . | . | . | . | . | . | . | . | . | . |
| 21-225_198B8_HC | . | | . | . | . | . | . | . | | | | . | . | . | . | . | . | . | . | . | . | . | . | R | . | . |
| 21-225_212H12_HC | . | | . | . | . | . | . | . | | | | . | . | . | . | . | . | . | . | . | . | . | . | . | . | . |
| 21-225_214H3_HC | . | | . | . | . | . | . | . | | | | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | Y | I | Y | Y | - | - | - | - | S | G | S | T | Y | Y | N | P | S | L | K | S | R | V |
| 21-225_190F10_HC | | | | | | | | | | | | | | | | | | | | | | | | | | |
| 21-225_191A2_HC | | | | | | | | | | | | | | | | | | | | | | | | | | |
| 21-225_192H8_HC | | | | | | | | | | | | | | | | | | | | | | | | | | L |
| 21-225_194D12_HC | | | | | | | | | | | | | | | | | | | | | | | | | | |
| 21-225_194G5_HC | | | | | | | | | | | | | | | | | | | | | | | | | | |
| 21-225_198B8_HC | | | | | | | | | | | | | | | | | | | | | | | | | | |
| 21-225_212H12_HC | | | | | | | | | | | | | | | | | | | | | | | | | | |
| 21-225_214H3_HC | | | | | | | | | | | | | | | | | | | | | | | | | | |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | V | D | T | S | K | N | Q | F | S | L | K | L | S | S | V | T | A | A | D | T | A | V | Y |
| 21-225_190F10_HC | | | | | | | | | | | | | | | | | | | | | | | | | | |
| 21-225_191A2_HC | | | | | | | | | | | | | | | | | | | | | | | | | | |
| 21-225_192H8_HC | | M | | A | | | | | | | | | | | | | | | | | | | | | | |
| 21-225_194D12_HC | | | | | | | | | | | | | | | | | | | | | | | | | | |
| 21-225_194G5_HC | | | | | | | | | | | | | | | | | | | | | | | | | | |
| 21-225_198B8_HC | | | | | | | | | | | | | | | | | | | | | | | | | | |
| 21-225_212H12_HC | | | | G | | | | | | | | | | | | | | | | | | | | | | |
| 21-225_214H3_HC | | | | G | | | | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | S | S | S | Y | - | - | - | - | - | - | - | - | - | - | - | - | - | - | - | - | - |
| 21-225_190F10_HC | | | | | | | | | . | | | | | | | | | | | | | | | | | |
| 21-225_191A2_HC | | | | | | | | | . | | | | | | | | | | | | | | | | | |
| 21-225_192H8_HC | | | | | . | . | . | L | . | | | | | | | | | | | | | | | | | |
| 21-225_194D12_HC | | | | | G | A | . | . | | | | | | | | | | | | | | | | | | |
| 21-225_194G5_HC | | | | | . | . | L | . | | | | | | | | | | | | | | | | | | |
| 21-225_198B8_HC | | | | | | | | | . | | | | | | | | | | | | | | | | | |
| 21-225_212H12_HC | | | | | | | | | | | | | | | | | | | | | | | | | | |
| 21-225_214H3_HC | | | | | | | | | | | | | | | | | | | | | | | | | | |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | H_FR4 | | | | | | |
| CONSENSUS | - | - | - | - | G | M | D | V | W | G | Q | G | T | T | V | T | V | S | S |
| 21-225_190F10_HC | . | | | | | | | | | | | | | | | | | | |
| 21-225_191A2_HC | . | | | | | | | | | | | | | | | | | | |
| 21-225_192H8_HC | . | | | | | | | | | | | | | | | | | | |
| 21-225_194D12_HC | . | | | | | | | | | | | | | | | | | | |
| 21-225_194G5_HC | . | | | | | | | | | | | | | | | | | | |
| 21-225_198B8_HC | . | | | | | | | | | | | | | | | | | | |
| 21-225_212H12_HC | . | | | | | | | | | | | | | | | | | | |
| 21-225_214H3_HC | . | | | | | | | | | | | | | | | | | | |

**FIGURE 57**

Table 95.    Consensus 15 -VK1|A30/JK4 (SEQ ID NO: 50312):

DIQMTQSPSSLSASVGDRVTITCRAS--QGIR------NDLGWYQQKPGKAPKRLIYA--------ASSLQSGVPSRFSGSGSG--
TEFTLTISSLQPEDFATYYCLQHNS---------------------YPLTFGGGTKVEIKR wherein:

 R at position 24 can be substituted with L

A at position 25 can be substituted with T

Q at position 29 can be substituted with R

G at position 30 can be substituted with D, A or V

I at position 31 can be substituted with M or V

R at position 32 can be substituted with E, K, N or S

N at position 39 can be substituted with S, D, T, K or I

D at position 40 can be substituted with N or A

L at position 41 can be substituted with F or V

G at position 42 can be substituted with D or N

A at position 58 can be substituted with T, S, V, G, D or R

A at position 67 can be substituted with T, V or E

S at position 68 can be substituted with F, C or Y

S at position 69 can be substituted with N, T, F, R or I

L at position 70 can be substituted with V, F or S

Q at position 71 can be substituted with H or E

S at position 72 can be substituted with R, N, T or G

FIGURE 57

L at position 107 can be substituted with V or I

Q at position 108 can be substituted with H

H at position 109 can be substituted with D, Y, N or R

N at position 110 can be substituted with S, Y, T, D, K, A, I, E, H, P or R

S at position 111 can be substituted with I, N, R, T, D, A, L or V

Y at position 135 can be substituted with F, H or S

P at position 136 can be substituted with A or M

L at position 137 can be substituted with P, F, N or V

T at position 138 can be substituted with K or I

Xaa Xaa Ser Xaa Xaa Xaa Xaa Xaa Xaa Xaa Xaa (SEQ ID NO: 50122)

Xaa Xaa Xaa Xaa Xaa Xaa Xaa (SEQ ID NO: 50123)

Xaa Xaa Xaa Xaa Xaa Xaa Xaa Xaa Xaa (SEQ ID NO: 50124)

Arg Ala Ser Gln Gly Ile Arg Asn Asp Leu Gly (SEQ ID NO: 50606)

Ala Ala Ser Ser Leu Gln Ser (SEQ ID NO: 50607)

Leu Gln His Asn Ser Tyr Pro Leu Thr (SEQ ID NO: 50608)

FIGURE 57

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | K_FR1 | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_15H1_LC | . | . | . | . | . | . | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | . | . | . | . | . | ~ | . | . | . | . | . | . | . | . | . | . | . | . | . | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | . | . | . | . | . | . | . | . | . | . | . | . | . | . | . | . | . | . | . | . | . |
| 21-225_169F9_LC | . | . | . | . | . | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | . | . | ! | . | . | . | . | . | ~ | . | . | . | . | . | . | . | . | . | . | . | . | . | . | . |
| 21-225_170D11_LC | . | . | . | . | . | . | . | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . | . | . | . | . | . | . | . |
| 21-225_170G4_LC | . | . | . | . | . | . | . | . | . | . | . | . | . | . | . | . | . | . | . | . | . | . | . | . | . | . |
| 21-225_170G5_LC | . | . | . | . | . | . | . | . | . | . | . | . | . | . | . | . | . | . | . | . | . | . | . | . | . | . |
| 21-225_171A4_LC | . | . | . | . | . | . | . | . | . | . | . | . | . | . | . | . | . | . | . | . | . | . | . | . | . | . |
| 21-225_171C3_LC | . | . | . | . | . | . | . | . | . | . | . | . | . | . | . | . | . | . | . | . | . | . | . | . | . | . |
| 21-225_172B3_LC | . | . | . | . | . | . | . | . | . | . | . | . | . | . | . | . | . | . | . | . | . | . | . | . | . | . |
| 21-225_172G8_LC | . | . | . | . | . | . | . | . | . | . | . | . | . | . | . | . | . | . | . | . | . | . | . | . | . | . |
| 21-225_173H12_LC | . | . | . | . | . | . | . | . | . | . | . | . | . | . | . | . | . | . | . | . | . | . | . | . | . | . |
| 21-225_175G1_LC | . | . | . | . | . | . | . | . | . | . | . | . | . | . | . | . | . | . | . | . | . | . | . | . | . | . |
| 21-225_176B11_LC | . | . | . | . | . | . | . | . | . | . | . | . | . | . | . | . | . | . | . | . | . | . | . | . | . | . |
| 21-225_178B10_LC | . | . | . | . | . | . | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . | . | . | . | . | . | . | . |
| 21-225_17F9_LC | . | . | . | . | . | . | . | . | . | . | . | . | . | . | . | . | . | . | . | . | . | . | . | . | . | . |
| 21-225_17H12_LC | . | . | . | . | . | . | . | . | . | . | . | . | . | . | . | . | . | . | . | . | . | . | . | . | . | . |
| 21-225_17H8_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | . | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | T | . |
| 21-225_18E8_LC | . | . | . | . | . | . | A | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | . | . |
| 21-225_193A5_LC | . | . | . | . | . | . | . | . | . | . | . | S | . | . | . | . | . | . | . | . | I | . | S | . | . | . |
| 21-225_193E7_LC | . | . | . | . | . | . | . | . | . | . | . | . | . | . | . | . | . | . | . | . | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | . | . | . | . | . | . | . | . | . | . | . | . | . | . | . | . | . | . | . | . | . | . | . |
| 21-225_19D11_LC | . | . | . | . | . | . | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | . | . | . | . | . | . | . | . | . | . | S | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | . | . |
| 21-225_200F12_LC | . | . | . | . | . | . | . | . | . | . | . | . | . | . | . | . | . | . | . | . | . | . | . | . | . | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | . | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | . | . | . |
| 21-225_204H8_LC | . | . | . | . | . | . | . | . | . | . | . | . | . | . | . | . | . | . | . | . | . | . | . | . | . | . |
| 21-225_205H8_LC | . | . | . | . | . | . | . | . | . | . | . | . | . | . | . | . | . | . | . | . | . | . | . | . | . | . |
| 21-225_20A10_LC | . | . | . | . | . | . | . | . | . | . | . | . | . | . | . | . | . | . | . | . | . | . | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | . | . | . | . | . | . | . | . | . | . | . | Q | . | . | . | . | . | . | . | . | . | . |
| 21-225_20C8_LC | . | . | . | . | . | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_20G9_LC | . | | | | | | | | | | | | | | | | | | | | | | | | . | . |
| 21-225_20H10_LC | G | . | . | . | . | . | . | . | . | . | . | . | . | . | . | . | . | . | . | . | . | . | . | . | . | . |
| 21-225_20H12_LC | . | | | | | | | | | | | | | | | | | | | | | | | | . | . |
| 21-225_21E5_LC | . | | | | | | | | | | | | | | | | | | | | | | | | . | . |
| 21-225_21F3_LC | . | | | | | | | | | | | | | | | | | | | | | | | | . | . |
| 21-225_21G7_LC | . | | | | | | | | | | | | | | | | | | | | | | | | . | . |
| 21-225_21H3_LC | . | | | | | | | | | | | | | | | | | | | | | | | | . | . |
| 21-225_224A1_LC | . | | | | | | | | | | | | | | | | | | | | | | | | | |
| 21-225_224B1_LC | . | | | | | | | | | | | | | | | | | | | | | | | | | |
| 21-225_224C3_LC | . | | . | | | | | | | | | | | | | | | | | | | | | | | |
| 21-225_224D10_LC | . | | L | | | | | | | | | | | | | | | | | | | | | | | |
| 21-225_22B7_LC | . | | | | | | | | | | | | | | | | | | | | | | | | | |
| 21-225_22C1_LC | . | | | | | | | | | | | | | | | | | | | | | | | | Y | . |
| 21-225_22D12_LC | . | | | | | | | | | | | | | | | | | | | | | | | | . | . |
| 21-225_22F2_LC | . | | | | | | | | | | | | | | | | | | | | | | | | . | . |
| 21-225_22F9_LC | . | | | | | | | | | | | | | | | | | | | | | | | | . | . |
| 21-225_22G8_LC | . | | | | I | | | | | | | S | | | | | | | | | | | | | Y | . |
| 21-225_22G9_LC | . | | | | | | | | | | | | | | | | | | | | | | | | . | . |
| 21-225_22H4_LC | . | | | | | | | | | | | | | | | | | | | | | | | | . | . |
| 21-225_23A3_LC | . | | | | | | | | | | | | | | | | | | | | | | | | . | . |
| 21-225_23C8_LC | . | | | | | | | | | R | | | | | | | | | | | | | | | . | . |
| 21-225_23D1_LC | . | | | | | | | | | | | | | | | | | | | | | | | | . | . |
| 21-225_23H11_LC | . | | | | | | | | | | | | | | | | | | | | | | | | . | . |
| 21-225_24E5_LC | . | | | | | | | | | | | | | | | | | | | | | | | | . | . |
| 21-225_25E6_LC | . | | | | | | | | | | | | | | | | | | | | | | | | . | . |
| 21-225_25H10_LC | . | | | | | | | | | | | | | | | | | | | | | | | | . | . |
| 21-225_25H11_LC | . | | | | | | | | | | | | | | | | | | | | | | | | . | . |
| 21-225_27C3_LC | . | | | | | | | | | | | | | | | | | | | | | | | | . | . |
| 21-225_27F2_LC | . | | | | | | | | | | | | | | | | | | | | | | | | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | . | . | . | . | . | . | . | . | . | . | . | . | S | . | . | . | . | . | . | . | . | . | . | . | . | . |
| 21-225_2B2_LC | . | . | . | . | . | . | . | . | . | . | . | . | . | . | . | . | . | . | . | . | . | . | . | . | . | . |
| 21-225_2G9_LC | . | . | . | . | . | . | . | . | . | . | . | P | . | . | . | . | . | . | . | . | . | . | . | . | . | . |
| 21-225_30D1_LC | . | . | . | . | . | . | . | . | . | . | . | . | . | . | . | . | . | . | . | . | . | . | . | . | . | . |
| 21-225_30E3_LC | . | . | . | . | . | . | . | . | . | . | . | . | . | . | . | . | . | . | . | . | . | . | . | . | . | . |
| 21-225_30E9_LC | . | . | . | . | . | . | . | . | . | . | . | . | . | . | . | . | . | . | . | . | . | . | . | . | . | . |
| 21-225_30F3_LC | . | . | . | . | . | . | . | . | . | . | . | . | . | . | . | . | . | . | . | . | . | . | . | . | . | . |
| 21-225_30G11_LC | . | . | . | . | . | . | . | . | . | . | . | . | . | . | . | . | . | . | . | . | . | . | . | . | . | . |
| 21-225_31A8_LC | . | . | . | . | . | . | . | . | . | . | . | . | . | . | . | . | . | . | . | . | . | . | . | . | . | . |
| 21-225_31B8_LC | . | . | . | . | I | . | . | . | . | . | . | . | . | . | . | . | . | . | . | . | . | . | . | . | . | . |
| 21-225_31H3_LC | . | . | . | . | . | . | . | . | . | . | . | . | . | . | . | . | . | . | . | . | . | . | . | . | . | . |
| 21-225_31H5_LC | . | . | . | . | . | . | . | . | . | . | . | . | . | . | . | . | . | . | P | . | . | S | P | . | . | . |
| 21-225_32G1_LC | . | . | . | . | . | . | . | . | . | . | . | . | . | . | . | . | . | . | . | . | . | . | . | . | . | . |
| 21-225_32G12_LC | . | . | . | . | . | . | . | . | . | . | . | . | . | . | . | . | . | . | . | . | . | . | . | . | . | . |
| 21-225_33A4_LC | . | . | . | . | . | . | . | . | . | . | . | . | . | . | . | . | . | . | . | . | . | . | . | . | . | . |
| 21-225_33A7_LC | . | . | . | . | . | . | . | . | . | . | . | . | . | . | . | . | . | . | . | . | . | . | . | . | . | . |
| 21-225_33B1_LC | . | . | . | . | . | . | . | . | . | . | . | . | . | . | . | . | . | . | . | . | . | . | . | . | T | . |
| 21-225_33B8_LC | . | . | . | . | . | . | . | . | . | . | . | . | . | . | . | . | . | . | . | . | . | . | . | . | . | . |
| 21-225_33F1_LC | . | . | . | . | . | . | . | . | . | . | . | . | . | . | . | . | . | . | . | . | . | . | . | . | T | . |
| 21-225_33G7_LC | . | . | . | . | . | . | . | . | . | . | . | . | . | . | . | . | . | . | . | . | . | . | . | . | . | . |
| 21-225_34A6_LC | . | . | . | . | . | . | . | . | . | . | . | . | . | . | . | . | . | . | . | . | . | . | . | . | T | . |
| 21-225_34D11_LC | . | . | . | . | . | . | . | . | . | . | . | . | . | . | . | . | . | . | . | . | . | . | . | . | . | . |
| 21-225_34G8_LC | . | . | . | . | . | . | . | . | . | . | . | . | . | . | . | . | . | . | . | . | . | . | . | . | . | . |
| 21-225_34G9_LC | . | . | . | . | . | . | . | . | . | . | . | . | . | . | . | . | . | . | . | . | . | . | . | . | . | . |
| 21-225_34H11_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_35A6_LC | . | . | . | . | . | . | . | . | . | . | . | . | . | . | . | . | . | . | . | . | . | . | . | . | . | . |
| 21-225_35B7_LC | . | . | . | . | . | . | . | . | . | . | . | . | . | . | . | . | . | . | ? | . | . | . | . | . | . | . |
| 21-225_35D1_LC | . | . | . | . | . | . | . | . | . | . | . | . | . | . | . | . | . | . | . | . | . | . | . | . | . | . |
| 21-225_35F11_LC | . | . | . | . | . | . | . | . | . | . | . | . | . | . | . | . | . | . | . | . | . | . | . | . | . | . |
| 21-225_36A5_LC | . | . | . | . | . | . | . | . | . | . | . | . | . | . | . | . | . | . | . | . | . | . | . | . | T | . |
| 21-225_36C5_LC | . | . | . | . | . | . | . | . | . | . | . | . | . | . | . | . | . | . | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | . | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | T | . |
| 21-225_46B1_LC | . | . | . | . | . | . | . | . | . | . | . | . | . | . | . | . | . | . | . | . | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | . | . | . | . | . | . | . | . | . | . | . | . | . | . | . |
| 21-225_46F3_LC | . | . | . | . | . | . | . | . | . | . | . | . | . | . | . | . | . | . | . | . | . | . | . | . | . | . |
| 21-225_47A1_LC | . | . | . | . | . | . | . | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . | . | . | . | . | . | . | . |
| 21-225_48H4_LC | . | . | . | . | . | ? | . | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . | . | . | . | . | . | . | . |
| 21-225_4C8_LC | . | . | . | i | . | . | . | . | S | . | S | . | C | . | . | . | . | . | . | . | . | . | . | . | . | . |
| 21-225_4E10_LC | . | . | . | . | . | . | . | . | . | . | S | . | . | . | . | . | . | . | . | . | . | . | . | . | . | . |
| 21-225_4F12_LC | . | . | . | . | . | . | . | . | . | . | . | . | . | . | . | . | . | . | . | . | . | . | . | . | . | . |
| 21-225_4F4_LC | . | . | . | . | . | . | . | . | . | . | . | . | . | . | . | . | . | . | . | . | . | . | . | . | . | . |
| 21-225_4H4_LC | . | . | . | . | . | . | . | . | . | . | . | . | . | . | . | . | . | . | . | . | . | . | . | . | . | . |
| 21-225_50G10_LC | . | . | . | . | . | . | . | . | . | . | . | . | . | . | . | . | . | . | . | . | . | . | . | . | . | . |
| 21-225_51F11_LC | . | . | . | . | . | . | . | . | A | . | . | . | . | . | . | . | . | . | . | . | . | . | . | . | . | . |
| 21-225_52B2_LC | . | . | . | . | . | . | . | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | A | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_54C6_LC | . | . | . | . | . | . | . | . | . | . | . | . | . | . | . | . | A | . | . | . | . | . | . | . | . | . |
| 21-225_55B8_LC | . | . | . | . | . | . | . | . | . | . | . | . | S | . | . | . | . | . | . | . | . | . | . | . | . | . |
| 21-225_55H6_LC | . | . | . | . | . | . | . | . | . | . | . | . | . | . | . | . | . | . | . | . | . | . | . | . | . | . |
| 21-225_56F1_LC | . | . | . | . | . | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | T | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | . | . | . | . | . | . | . | . | . | . | . | . | . | . | . | S | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | . | . | . | . | . | . | G | . | . | . | . | . | . | . |
| 21-225_6D4_LC | . | . | . | . | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | . | . | . | . | . | T | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | . | . | . | . | . | . | . | . | . | . |
| 21-225_75H7_LC | . | . | . | . | . | . | . | . | . | . | R | Y | . | . | . | . | . | . | . | . | . | N | S | . | . | . |
| 21-225_7C4_LC | . | . | . | . | . | . | . | . | . | . | . | S | . | . | . | . | . | . | . | . | . | . | . | . | . | . |
| 21-225_7E9_LC | . | . | . | . | . | . | . | . | . | . | . | S | . | . | . | . | . | . | . | . | . | . | . | . | . | . |
| 21-225_7F4_LC | . | . | . | . | . | . | . | . | . | . | . | . | . | . | . | . | . | . | . | . | . | . | . | . | T | . |
| 21-225_7H11_LC | . | . | . | . | . | . | . | . | . | . | A | . | . | . | . | . | . | V | . | . | . | I | . | L | . | . |
| 21-225_7H7_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_8C9_LC | . | . | . | . | . | . | . | . | . | . | . | . | . | . | ? | . | . | . | . | . | . | . | . | . | . | . |
| 21-225_8D12_LC | . | . | . | . | . | . | . | . | . | . | . | . | . | . | . | . | . | . | . | . | . | . | . | . | . | . |
| 21-225_8F11_LC | . | . | . | . | . | . | . | . | . | . | . | . | . | . | . | . | . | . | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | . | . | . | . | . | . | . | . | . | . |
| 21-225_9E8_LC | . | . | . | . | . | . | . | . | . | . | ? | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | G | I | R | - | - | - | - | - | - | N | D | L | G | W | Y | Q | Q | K | P | G | K | A | P |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . | . | . | . | . | . | . | . |
| 21-225_11F10_LC | . | . | . | . | . | . | . | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . | . | . | . | . | . | . | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | . | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | . | . | . | . | . | . | . |
| 21-225_146F1_LC | . | . | . | . | . | . | . | . | . | . | . | . | . | . | . | . | . | . | . | . | . | . | . | . | . | . |
| 21-225_146G11_LC | . | . | . | O | . | . | . | . | . | . | . | . | . | N | P | . | . | . | . | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | . | . | . | . | . | . | . | . | . | . | . | . | . | . | . | . | . | . | . | . | . | . | . |
| 21-225_148F12_LC | . | . | . | . | . | . | . | . | . | . | . | . | . | A | . | . | . | . | . | . | . | . | . | . | . | . |
| 21-225_149F12_LC | . | . | . | . | . | . | . | . | . | . | . | . | . | . | . | . | . | . | . | . | . | . | . | . | . | . |
| 21-225_149G1_LC | . | . | . | . | . | . | . | . | . | . | . | . | . | . | . | . | . | . | . | . | . | . | . | . | . | . |
| 21-225_149G5_LC | . | . | . | . | . | . | . | . | . | . | . | . | . | A | . | . | . | P | . | . | . | . | . | . | . | . |
| 21-225_14B1_LC1_LC | . | . | . | O | . | . | . | . | . | . | . | . | O | N | . | . | . | . | . | . | . | . | . | . | . | . |
| 21-225_14B2_LC | . | . | . | . | . | . | . | . | . | . | . | . | . | . | . | . | . | . | . | . | . | . | . | . | . | . |
| 21-225_14C2_LC | . | . | . | . | . | . | . | . | . | . | . | . | . | . | O | . | . | . | . | . | . | . | . | . | . | . |
| 21-225_14E3_LC | . | . | . | . | . | . | . | . | . | . | . | . | . | . | . | . | . | . | . | . | . | . | . | . | . | . |
| 21-225_150E7_LC | . | . | . | . | . | . | . | . | . | . | . | . | . | . | . | . | . | . | . | . | . | . | . | . | . | . |
| 21-225_152H9_LC | . | . | . | . | . | . | . | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . | . | . | . | . | . | . | . |
| 21-225_158H5_LC | . | . | . | . | M | . | . | . | . | . | . | . | I | . | . | . | . | . | . | . | . | . | . | . | . | . |
| 21-225_15A1_LC | . | . | . | . | . | . | . | . | . | . | . | . | . | . | . | . | . | . | . | . | . | . | . | . | . | . |
| 21-225_15C9_LC | . | . | . | . | . | . | . | . | . | . | . | . | . | . | . | . | . | . | . | . | . | . | . | . | . | . |
| 21-225_15E3_LC | . | . | . | . | . | . | . | . | . | . | . | . | . | . | . | . | . | . | . | . | . | . | . | . | . | . |
| 21-225_15G7_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_15H1_LC | . | . | . | . | . | . | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | . | . | . | . | . | . | . | . | . | . | . | . | . | . | . | . | . | . | T | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | . | . | . | . | . | . | . | . | . | . | . | . | . | . | . | . | . | . | . | . | . |
| 21-225_169F9_LC | . | . | . | . | . | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | . | . | . | . | . | . | . | . | . | . | . | . | . | . | . | . | . | . | . | S | . | . | . | . |
| 21-225_170D11_LC | . | . | . | . | . | . | . | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . | E | . | . | . | . | . | . |
| 21-225_170G4_LC | . | . | . | . | . | . | . | . | . | . | . | . | . | . | . | . | . | . | M | . | . | . | . | . | . | . |
| 21-225_170G5_LC | . | . | . | . | . | . | . | . | . | . | . | . | T | . | . | . | . | F | . | . | . | . | . | . | . | . |
| 21-225_171A4_LC | . | . | . | . | . | . | . | . | . | . | . | . | . | . | . | . | . | . | . | . | . | . | . | . | . | . |
| 21-225_171C3_LC | . | . | . | . | . | . | . | . | . | . | . | . | T | . | . | . | . | F | . | . | . | . | . | . | . | . |
| 21-225_172B3_LC | . | . | . | . | . | . | . | . | . | . | . | . | . | . | . | . | . | . | . | . | . | . | . | . | . | . |
| 21-225_172G8_LC | . | . | . | V | . | . | . | . | . | . | . | . | . | . | . | . | . | . | . | . | . | . | . | . | . | . |
| 21-225_173H12_LC | . | . | . | . | . | . | . | . | . | . | . | . | . | . | . | . | . | . | . | . | . | . | . | . | . | . |
| 21-225_175G1_LC | . | . | . | . | . | . | . | . | . | . | . | . | T | . | . | . | . | . | . | . | . | . | . | . | . | . |
| 21-225_176B11_LC | . | . | . | . | . | . | . | . | . | . | . | . | . | . | . | . | . | F | . | . | . | . | . | . | . | . |
| 21-225_178B10_LC | . | . | . | . | . | . | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . | . | . | . | . | . | . | . |
| 21-225_17F9_LC | . | . | . | V | . | . | . | . | . | . | . | . | . | . | . | . | . | . | . | . | . | . | . | . | . | . |
| 21-225_17H12_LC | . | . | . | D | . | . | . | . | . | . | . | . | . | . | . | . | . | . | . | . | . | A | . | . | . | . |
| 21-225_17H8_LC | . | . | . | . | . | . | . | . | . | . | . | . | . | . | . | . | . | . | . | . | . | . | . | . | . | . |

4314

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | . | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | . | . |
| 21-225_193A5_LC | . | . | . | . | . | . | . | . | . | . | . | . | . | . | . | . | . | . | . | . | Y | . | . | . | . | . |
| 21-225_193E7_LC | . | . | . | . | . | . | . | . | . | . | . | . | . | . | . | . | . | . | . | . | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | . | . | . | . | . | . | . | . | . | . | . | . | . | . | . | . | . | . | . | . | . | . | . |
| 21-225_19D11_LC | . | . | . | . | . | . | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | . | . | . | . | . | . | . | . | . | . | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | . | . |
| 21-225_200F12_LC | . | . | . | . | . | . | . | . | . | . | . | . | . | . | . | . | . | . | . | . | . | . | . | . | ※ | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | . | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | . | ※ | . |
| 21-225_204H8_LC | . | . | . | . | . | . | . | . | . | . | . | . | . | . | . | . | . | . | . | . | . | . | . | . | . | . |
| 21-225_205H8_LC | . | . | . | . | . | . | . | . | . | . | . | . | . | . | . | . | . | . | . | . | . | Q | . | . | . | . |
| 21-225_20A10_LC | . | . | . | . | . | . | . | . | . | . | . | . | . | . | . | . | . | . | . | . | . | Q | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | . | . | . | . | . | . | . | . | . | . | . | . | . | . | . | . | . | . | . | . | . | . |
| 21-225_20C8_LC | . | . | . | . | . | . | . | . | . | . | . | . | . | . | . | . | . | . | . | ※ | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_20G9_LC | . | . | . | . | . | . | . | . | . | . | . | . | . | . | . | . | . | . | . | . | . | . | . | . | . | . |
| 21-225_20H10_LC | . | . | . | . | . | ~ | . | . | . | . | . | . | N | . | . | . | . | . | . | . | . | . | . | ~ | . | . |
| 21-225_20H12_LC | . | . | . | . | . | . | . | . | . | . | . | . | . | D | . | . | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | . | . | . | . | . | . | . | . | . | . | . | . | . | . | . | . | . | . | . | . | . |
| 21-225_21G7_LC | . | . | . | V | . | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | . | . | . | . | . | . | . | . |
| 21-225_224A1_LC | . | . | . | . | . | E | . | . | . | . | . | . | . | . | . | . | . | . | . | . | . | . | . | . | . | . |
| 21-225_224B1_LC | . | . | . | . | . | S | . | . | . | . | . | . | . | . | . | . | . | . | . | . | . | . | . | . | . | . |
| 21-225_224C3_LC | . | . | . | D | . | . | . | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | . | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | . | . |
| 21-225_22D12_LC | . | . | . | . | . | . | . | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . | . | . | . | . | . | . | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | . | . |
| 21-225_23A3_LC | . | . | . | . | . | . | . | . | . | . | . | . | . | . | . | . | . | . | . | . | . | . | . | . | . | . |
| 21-225_23C8_LC | . | . | . | D | . | . | . | . | . | . | . | . | . | . | . | . | . | . | . | . | . | . | . | ~ | . | . |
| 21-225_23D1_LC | . | . | . | . | . | . | . | . | . | . | . | . | . | . | . | . | . | . | . | . | . | . | . | . | . | . |
| 21-225_23H11_LC | . | . | . | . | . | . | . | . | . | . | . | . | . | . | . | D | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | ~ | . | . | . | . | . | . |
| 21-225_25H10_LC | . | . | ~ | . | . | . | . | . | . | . | . | . | . | . | . | . | . | . | . | . | . | . | . | . | . | . |
| 21-225_25H11_LC | . | . | . | . | . | . | . | . | . | . | . | . | T | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | G | I | R | - | - | - | - | - | - | N | D | L | G | W | Y | Q | Q | K | P | G | K | A | P |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | L | . | . | . | . | . | . |
| 21-225_29H6_LC | . | . | . | . | . | . | . | . | . | . | . | . | . | . | . | . | . | . | . | . | . | S | . | . | T | . |
| 21-225_2A11_LC | . | . | . | . | . | . | . | . | . | . | . | . | . | . | . | . | . | . | . | . | . | . | . | . | . | . |
| 21-225_2B2_LC | . | . | . | . | . | . | . | . | . | . | . | . | . | . | . | . | . | . | . | . | . | . | Q | . | . | . |
| 21-225_2G9_LC | . | . | . | . | . | . | . | . | . | . | . | . | . | . | . | . | . | . | . | . | . | . | . | . | . | . |
| 21-225_30D1_LC | . | . | . | D | . | . | . | . | . | . | . | . | S | . | . | . | . | . | . | . | . | . | . | . | . | . |
| 21-225_30E3_LC | . | . | . | D | . | . | . | . | . | . | . | . | D | . | . | . | . | . | . | . | . | . | . | . | . | . |
| 21-225_30E9_LC | . | . | . | D | . | . | . | . | . | . | . | . | D | . | . | . | . | . | . | . | . | . | . | . | . | . |
| 21-225_30F3_LC | . | . | . | . | . | . | . | . | . | . | . | . | . | . | . | . | . | . | . | . | . | . | . | . | . | . |
| 21-225_30G11_LC | . | . | . | D | . | . | . | . | . | . | . | . | S | . | . | . | . | . | . | . | . | . | . | . | . | . |
| 21-225_31A8_LC | . | . | . | D | . | . | . | . | . | . | . | . | S | . | . | . | . | . | . | . | . | . | . | . | . | . |
| 21-225_31B8_LC | . | . | . | D | . | . | . | . | . | . | . | . | S | . | . | . | . | . | . | . | . | . | . | . | . | . |
| 21-225_31H3_LC | . | . | . | D | . | . | . | . | . | . | . | . | D | . | . | . | . | . | . | . | . | . | . | . | . | . |
| 21-225_31H5_LC | . | . | . | D | . | . | . | . | . | . | . | . | S | . | . | . | . | . | . | . | . | . | . | . | . | X |
| 21-225_32G1_LC | . | . | . | D | M | . | . | . | . | . | . | . | S | . | . | . | . | . | . | . | . | . | . | . | . | . |
| 21-225_32G12_LC | . | . | . | D | . | . | . | . | . | . | . | . | S | . | . | . | . | . | . | X | . | . | . | . | . | . |
| 21-225_33A4_LC | . | . | . | D | . | . | . | . | . | . | . | . | . | . | V | . | . | . | . | . | . | . | . | . | . | . |
| 21-225_33A7_LC | . | . | . | D | . | . | . | . | . | . | . | . | S | . | . | . | . | . | . | . | . | . | . | . | . | . |
| 21-225_33B1_LC | . | . | . | . | . | . | . | . | . | . | . | . | . | . | . | . | . | P | . | . | . | . | . | . | . | . |
| 21-225_33B8_LC | . | . | . | D | . | . | . | . | . | . | . | . | S | . | . | . | . | . | . | . | . | . | . | . | . | . |
| 21-225_33F1_LC | . | . | . | D | . | . | . | . | . | . | . | . | S | . | . | . | . | . | . | . | . | . | . | . | . | . |
| 21-225_33G7_LC | . | . | . | . | . | . | . | . | . | . | . | . | . | . | . | . | . | . | . | . | . | . | . | . | . | . |
| 21-225_34A6_LC | . | . | . | D | . | . | . | . | . | . | . | . | . | . | . | . | . | . | . | . | . | . | . | . | . | . |
| 21-225_34D11_LC | . | . | . | D | . | . | . | . | . | . | . | . | S | . | . | . | . | . | . | . | . | . | . | . | . | . |
| 21-225_34G8_LC | . | . | . | D | . | . | . | . | . | . | . | . | S | . | . | . | . | . | . | . | . | . | . | . | . | . |
| 21-225_34G9_LC | . | . | . | D | . | . | . | . | . | . | . | . | S | . | . | . | . | . | . | . | . | . | . | . | . | . |
| 21-225_34H11_LC | . | . | . | D | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | G | I | R | - | - | - | - | - | - | N | D | L | G | W | Y | Q | Q | K | P | G | K | A | P |
| 21-225_35A6_LC | . | . | . | G | . | . | . | . | . | . | . | . | S | . | . | . | . | . | . | . | . | . | . | . | . | . |
| 21-225_35B7_LC | . | . | . | G | . | . | . | . | . | . | . | . | S | . | . | . | . | . | . | . | . | . | . | . | . | . |
| 21-225_35D1_LC | . | . | . | G | . | . | . | . | . | . | . | . | S | . | . | . | . | . | . | . | . | . | . | . | . | . |
| 21-225_35F11_LC | . | . | . | G | . | . | . | . | . | . | . | . | S | . | . | . | . | . | . | . | . | . | . | . | . | . |
| 21-225_36A5_LC | . | . | . | G | . | . | . | . | . | . | . | . | . | . | . | . | . | . | . | . | . | . | . | . | . | . |
| 21-225_36C5_LC | . | . | . | G | . | . | . | . | . | . | . | . | S | . | . | . | . | . | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | . | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . | . | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | . | X | . | . | . | . | . | . | . | . | . | . | . | . | . |
| 21-225_46F3_LC | . | . | . | . | . | . | . | . | . | . | . | . | X | . | . | . | . | . | . | . | . | . | . | . | . | . |
| 21-225_47A1_LC | . | . | . | G | . | . | . | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . | . | . | . | . | . | . | . |
| 21-225_48H4_LC | . | . | X | G | . | . | . | . | . | . | . | . | . | . | . | . | . | . | . | . | . | . | . | . | . | . |
| 21-225_49A1_LC | . | . | . | . | . | . | . | . | . | . | . | . | . | . | . | G | . | . | . | . | . | . | . | . | . | . |
| 21-225_49C6_LC | . | . | . | . | . | . | . | . | . | . | . | . | . | . | . | . | . | . | . | . | . | . | . | . | . | . |
| 21-225_4C8_LC | . | . | . | . | . | . | . | . | . | . | . | . | . | . | . | . | . | . | . | . | . | . | . | . | . | . |
| 21-225_4E10_LC | . | . | . | . | . | . | . | . | . | . | . | . | . | . | . | . | . | . | . | . | . | . | . | . | . | . |
| 21-225_4F12_LC | . | . | . | . | . | . | . | . | . | . | . | . | . | . | . | . | . | . | . | . | . | . | . | . | . | . |
| 21-225_4F4_LC | . | . | . | . | . | . | . | . | . | . | . | . | . | . | . | . | . | . | . | . | . | . | . | . | . | . |
| 21-225_4H4_LC | . | . | . | . | . | . | . | . | . | . | . | . | . | . | . | . | . | . | . | . | . | . | . | . | . | . |
| 21-225_50G10_LC | . | . | . | . | . | . | . | . | . | . | . | . | . | N | . | . | . | . | . | . | . | . | . | . | V | . |
| 21-225_51F11_LC | . | . | . | . | . | . | . | . | . | . | . | . | . | . | . | . | . | . | . | . | . | . | . | . | . | . |
| 21-225_52B2_LC | . | . | . | . | . | . | . | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | . | . | . | . | N | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_54C6_LC | . | . | . | . | . | . | . | . | . | . | . | . | . | . | . | . | . | . | . | . | N | . | . | . | . | . |
| 21-225_55B8_LC | . | . | . | A | . | . | . | . | . | . | . | . | . | . | . | . | . | . | . | . | . | . | . | . | . | . |
| 21-225_55H6_LC | . | . | . | . | . | . | . | . | . | . | . | . | . | . | . | . | . | . | . | . | . | . | . | . | . | . |
| 21-225_56F1_LC | . | . | . | Q | . | . | . | . | . | . | . | . | . | N | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | . | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | . | . | . | . | . | . | . | . | . | . | . | . | S | . | . | . | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | S | N | . | . | . | . | . | . | . | . | . | . | . | . |
| 21-225_6D4_LC | . | . | . | A | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | N | . | . | . | . | . | . | . | N | . | N | . | S | . | . | . | . | . | . | . | . |
| 21-225_70E8_LC | . | . | . | . | . | . | . | . | . | . | . | . | X | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | . | . | . | . | . | . | . | . | . | . |
| 21-225_75H7_LC | . | . | . | . | . | . | . | . | . | . | . | . | . | . | . | . | . | . | . | . | . | . | . | . | S | . |
| 21-225_7C4_LC | . | . | . | . | . | . | . | . | . | . | . | . | . | . | . | . | . | . | . | . | . | . | . | . | . | X |
| 21-225_7E9_LC | . | . | . | . | . | . | . | . | . | . | . | . | . | . | . | . | . | S | . | . | . | . | . | . | . | . |
| 21-225_7F4_LC | . | . | . | . | . | . | . | . | . | . | . | . | . | . | . | . | . | . | . | . | . | . | . | . | . | . |
| 21-225_7H11_LC | . | . | . | . | . | . | . | . | . | . | . | . | . | . | . | . | . | . | . | . | . | . | . | . | . | . |
| 21-225_7H7_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | K_CDR1 | | | | | | | | | | | | | | | | | | | K_FR2 | | | |
| CONSENSUS | - | - | Q | G | I | R | - | - | - | - | - | - | N | D | L | G | W | Y | Q | Q | K | P | G | K | A | P |
| 21-225_8C9_LC | . | . | . | A | . | . | . | . | . | . | . | . | . | . | . | D | . | . | . | . | . | . | . | . | . | . |
| 21-225_8D12_LC | . | . | . | . | . | . | . | . | . | . | . | . | . | . | . | . | . | . | . | . | . | . | . | . | . | . |
| 21-225_8F11_LC | . | . | . | . | . | . | . | . | . | . | . | . | S | . | . | . | . | Y | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | . | . | . | . | . | . | . | . | . | . |
| 21-225_9E8_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | G | . | . | . | . | . | . | . | . | . | . | . | . | . | . | . | . | . | . | . | . |
| 21-225_11F10_LC | . | . | . | . | . | . | . | . | . | . | . | . | . | . | . | . | . | . | . | . | . | . | . | . | . | . |
| 21-225_11F5_LC | . | . | . | . | . | . | . | . | . | . | . | . | . | . | . | . | . | V | . | . | . | . | . | . | . | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | . | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | T | . | . | . | . | . | . |
| 21-225_146F1_LC | . | . | . | . | . | . | . | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . | . | . | . | . | L | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | S | . | . | . | . | . | . | . | . | . | . | . | . | . | . | . | . | . | . | . | . | . | . |
| 21-225_148F12_LC | . | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | N | . | . | . | . | . | . | . | . | . |
| 21-225_149G1_LC | . | . | . | . | . | . | . | . | . | . | . | . | . | . | . | . | N | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | T | . | . | . | . | . | . | . | . | . | . | Y | . | . | . | . | . | . | . | . | . | . |
| 21-225_14B2_LC | . | . | . | . | V | . | . | . | . | . | . | . | . | . | T | . | . | . | . | G | . | . | . | . | . | . |
| 21-225_14C2_LC | . | . | . | . | . | . | . | . | . | . | . | . | . | . | . | . | . | . | . | . | . | . | . | . | . | . |
| 21-225_14E3_LC | . | . | I | . | S | . | . | . | . | . | . | . | . | . | . | . | . | . | . | . | . | . | . | . | . | . |
| 21-225_150E7_LC | . | . | . | . | . | . | . | . | . | . | . | . | . | . | . | . | N | . | . | . | . | . | . | L | . | . |
| 21-225_152H9_LC | . | . | . | . | . | . | . | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . | . | . | . | . | . | . | . |
| 21-225_158H5_LC | . | . | . | . | R | . | . | . | . | . | . | . | . | . | . | . | . | . | . | . | . | . | . | . | . | . |
| 21-225_15A1_LC | . | . | . | . | . | . | . | . | . | . | . | . | . | . | . | . | . | . | . | . | . | . | . | . | . | . |
| 21-225_15C9_LC | . | . | . | . | . | . | . | . | . | . | . | . | . | . | . | . | . | . | . | . | . | . | . | . | . | . |
| 21-225_15E3_LC | . | . | . | . | . | . | . | . | . | . | . | . | . | . | . | . | . | S | . | . | . | . | . | . | . | . |
| 21-225_15G7_LC | . | . | . | . | . | . | . | . | . | . | . | . | . | . | . | . | . | . | . | . | . | I | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_15H1_LC | . | . | . | . | . | T | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | F | . | . | . | . | . | . | . | . | . | . | . | . | . | . | . | . | . | . | . | . | . | . |
| 21-225_160B10_LC | . | . | . | . | . | . | . | . | . | . | . | . | . | . | . | . | . | . | . | . | . | . | . | . | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | G | . | . | . | . | . | . | . | . | S | . | . | . | . | . | . | . | . | . | . | . |
| 21-225_169F9_LC | . | . | . | . | F | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | V | . | . | . | . | . | . | . |
| 21-225_16F4_LC | . | . | . | . | . | V | . | . | . | . | . | . | . | . | . | . | . | . | . | . | . | . | . | . | . | . |
| 21-225_170D11_LC | . | R | . | . | . | . | . | . | . | . | . | . | . | . | . | . | . | . | . | . | N | . | . | . | . | . |
| 21-225_170D5_LC | . | R | . | . | . | . | . | . | . | . | . | . | . | . | . | . | . | . | . | . | N | . | . | . | . | . |
| 21-225_170G4_LC | . | . | . | . | . | . | . | . | . | . | . | . | . | . | . | . | T | . | . | . | . | . | . | . | . | . |
| 21-225_170G5_LC | . | . | . | . | . | T | . | . | . | . | . | . | . | . | . | . | . | . | . | . | . | . | . | . | . | . |
| 21-225_171A4_LC | . | . | . | . | . | T | . | . | . | . | . | . | . | . | . | . | . | . | . | . | . | . | . | . | . | . |
| 21-225_171C3_LC | . | . | . | . | . | T | . | . | . | . | . | . | . | . | . | . | . | . | . | . | . | . | . | . | . | . |
| 21-225_172B3_LC | . | . | . | G | . | . | . | . | . | . | . | . | . | . | S | . | . | . | . | . | . | . | . | . | . | . |
| 21-225_172G8_LC | . | R | . | . | . | . | . | . | . | . | . | . | . | . | . | . | . | . | . | . | N | . | . | . | . | . |
| 21-225_173H12_LC | . | . | . | . | . | T | . | . | . | . | . | . | . | . | T | . | . | . | . | . | . | . | . | . | . | . |
| 21-225_175G1_LC | . | . | . | . | . | T | . | . | . | . | . | . | . | . | . | . | . | . | . | . | . | . | . | . | . | . |
| 21-225_176B11_LC | . | . | . | . | . | . | . | . | . | . | . | . | . | . | . | . | . | . | . | . | . | . | . | . | . | . |
| 21-225_178B10_LC | . | R | . | . | . | . | . | . | . | . | . | . | . | . | . | . | . | . | . | . | N | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | N | . | . | . | . | . | . | . | . | . |
| 21-225_17F9_LC | . | . | . | . | . | . | . | . | . | . | . | . | . | . | V | . | . | . | . | . | . | . | . | . | . | . |
| 21-225_17H12_LC | . | . | . | N | . | . | . | . | . | . | . | . | . | . | . | . | . | . | . | . | . | . | . | . | . | . |
| 21-225_17H8_LC | . | . | . | . | . | G | . | . | . | . | . | . | . | . | . | . | . | . | . | . | . | . | . | . | . | . |

4322

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | V | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | . | . |
| 21-225_193A5_LC | . | V | . | . | . | . | . | . | . | . | . | . | . | . | . | . | . | . | . | . | . | . | . | . | . | . |
| 21-225_193E7_LC | . | . | . | . | . | S | . | . | . | . | . | . | . | . | . | . | . | . | . | ~ | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | S | S | . | . | . | . | . | . | . | . | . | . | . | . | . | . | ~ | . | . | . | . | . | . |
| 21-225_19D11_LC | . | . | . | . | . | . | . | . | . | . | . | . | . | . | T | . | . | . | . | . | . | . | . | . | . | . |
| 21-225_19E1_LC | . | . | . | . | . | . | . | . | . | . | . | . | . | . | . | . | N | . | . | . | . | . | . | . | . | . |
| 21-225_19F3_LC | . | . | . | . | . | . | . | . | . | . | . | . | . | . | V | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | V | . | . | . | . | . | . | . | . |
| 21-225_200F12_LC | . | . | . | . | . | S | . | . | . | . | . | . | . | . | . | . | . | . | M | T | . | . | . | . | . | . |
| 21-225_200H9_LC | . | . | . | . | . | . | . | . | . | . | . | . | . | . | . | . | . | . | . | . | . | ! | . | . | . | . |
| 21-225_201A1_LC | . | . | . | . | . | S | . | . | . | . | . | . | . | . | . | . | ! | . | . | ~ | . | . | . | . | . | . |
| 21-225_201A10_LC | . | . | . | . | . | S | . | . | . | . | . | . | . | . | T | . | . | . | M | . | . | . | . | . | . | . |
| 21-225_204H8_LC | . | . | . | . | . | S | . | . | . | . | . | . | . | . | . | . | . | . | . | . | . | . | . | . | . | . |
| 21-225_205H8_LC | . | L | . | . | . | S | . | . | . | . | . | . | . | . | . | . | F | . | . | ~ | . | . | . | . | . | . |
| 21-225_20A10_LC | . | . | . | . | . | . | . | . | . | . | . | . | . | . | . | . | . | . | . | . | . | . | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | F | . | . | . | . | . | . | . | . | . | . | . | . | . | . | . | . | . | . | . | . | . |
| 21-225_20C8_LC | . | . | . | N | . | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | F | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | F | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_20G9_LC | . | . | . | . | . | . | . | . | . | . | . | . | . | . | . | . | . | . | . | . | . | . | . | . | . | . |
| 21-225_20H10_LC | . | Q | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | T | . | . | . | . | . | . | . | . | . | . | . |
| 21-225_21F3_LC | . | . | . | . | . | . | . | . | . | . | . | . | . | . | . | . | . | . | . | . | . | . | . | . | . | . |
| 21-225_21G7_LC | Q | . | . | . | . | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | V | . | T | . | . | . | . | . | . |
| 21-225_224A1_LC | . | . | . | . | . | . | . | . | . | . | . | . | . | . | . | . | . | . | . | . | . | . | . | . | . | . |
| 21-225_224B1_LC | . | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | T | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | T | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | . | . |
| 21-225_22D12_LC | . | . | . | . | . | . | . | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . | . | . | . | . | . | N | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | V | . | . | . | . | . | . | . | . |
| 21-225_23A3_LC | . | . | . | . | . | . | . | . | . | . | . | . | . | . | . | . | . | V | . | . | . | . | . | . | . | . |
| 21-225_23C8_LC | . | . | . | N | G | . | . | . | . | . | . | . | . | . | . | . | . | V | . | . | . | . | . | . | . | . |
| 21-225_23D1_LC | . | . | . | . | . | . | . | . | . | . | . | . | . | . | . | . | . | . | . | . | . | . | . | . | . | . |
| 21-225_23H11_LC | . | . | . | . | . | . | . | . | . | . | . | . | . | . | . | . | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | . | . | . | . | . | . | . |
| 21-225_25H10_LC | . | . | . | . | . | . | . | . | . | . | . | . | . | . | P | . | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | V | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | Q | . | . | . | . | S | . | . | . | . | . | . | . | . | . | . | . | . | . | . | . | . | . | . | . | . |
| 21-225_2B2_LC | . | . | . | . | . | . | . | . | . | . | . | . | . | . | . | . | . | . | . | . | . | . | . | . | . | . |
| 21-225_2G9_LC | Q | . | . | . | . | . | . | . | . | . | . | . | . | . | . | . | . | . | . | . | . | . | . | . | . | . |
| 21-225_30D1_LC | . | . | . | . | . | . | . | . | . | . | . | . | . | . | . | . | . | . | . | . | . | . | . | . | . | . |
| 21-225_30E3_LC | . | . | . | . | . | . | . | . | . | . | . | . | . | . | . | . | . | . | . | . | . | . | . | . | . | . |
| 21-225_30E9_LC | . | . | . | . | . | G | . | . | . | . | . | . | . | . | . | . | . | . | . | . | . | . | . | . | . | . |
| 21-225_30F3_LC | . | . | . | . | . | T | . | . | . | . | . | . | . | . | . | . | . | . | . | . | . | . | . | . | . | . |
| 21-225_30G11_LC | . | . | . | . | . | . | . | . | . | . | . | . | . | . | . | . | . | . | . | . | . | . | . | . | . | . |
| 21-225_31A8_LC | . | . | . | . | . | . | . | . | . | . | . | . | . | . | V | . | . | . | . | . | . | . | . | . | . | . |
| 21-225_31B8_LC | . | . | . | . | . | G | . | . | . | . | . | . | . | . | . | . | . | . | . | . | . | . | . | . | . | . |
| 21-225_31H3_LC | . | . | . | . | . | . | . | . | . | . | . | . | . | . | . | . | . | . | . | . | . | . | . | . | . | . |
| 21-225_31H5_LC | . | I | . | . | . | . | . | . | . | . | . | . | . | . | . | . | . | . | . | . | . | . | . | . | . | . |
| 21-225_32G1_LC | . | . | . | . | . | . | . | . | . | . | . | . | . | . | . | . | . | . | . | . | . | . | . | . | . | . |
| 21-225_32G12_LC | . | . | . | . | . | . | . | . | . | . | . | . | . | . | . | . | . | . | . | . | . | . | . | . | . | . |
| 21-225_33A4_LC | . | . | . | . | . | . | . | . | . | . | . | . | . | . | . | . | . | . | . | . | . | . | . | . | . | . |
| 21-225_33A7_LC | . | . | . | . | . | V | . | . | . | . | . | . | . | . | . | . | . | . | . | . | . | . | . | . | . | . |
| 21-225_33B1_LC | . | . | . | . | . | T | . | . | . | . | . | . | . | . | . | . | . | . | . | . | . | . | . | . | . | . |
| 21-225_33B8_LC | . | . | . | . | . | . | . | . | . | . | . | . | . | . | . | . | . | . | . | . | . | . | . | . | . | . |
| 21-225_33F1_LC | . | . | . | . | . | . | . | . | . | . | . | . | . | . | . | . | . | . | . | . | . | . | . | . | . | . |
| 21-225_33G7_LC | . | . | . | . | . | . | . | . | . | . | . | . | . | . | . | . | . | . | . | . | . | . | . | . | . | . |
| 21-225_34A6_LC | . | . | . | . | F | . | . | . | . | . | . | . | . | . | . | . | . | . | . | . | . | . | . | . | . | . |
| 21-225_34D11_LC | . | . | . | . | . | . | . | . | . | . | . | . | . | . | . | . | . | . | . | . | . | . | . | . | . | . |
| 21-225_34G8_LC | . | . | . | . | . | . | . | . | . | . | . | . | . | . | . | . | . | . | . | . | . | . | . | . | . | . |
| 21-225_34G9_LC | . | . | . | . | . | . | . | . | . | . | . | . | . | . | . | . | . | . | . | . | . | . | . | . | . | . |
| 21-225_34H11_LC | . | . | . | . | . | . | . | . | . | . | . | . | . | T | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_35A6_LC | . | . | . | . | . | . | . | . | . | . | . | . | . | . | T | . | . | . | . | . | . | . | . | . | . | . |
| 21-225_35B7_LC | S | . | . | . | . | . | . | . | . | . | . | . | . | . | . | . | . | . | . | . | . | . | . | . | . | . |
| 21-225_35D1_LC | . | . | . | . | . | . | . | . | . | . | . | . | . | . | . | . | . | . | . | . | . | . | . | . | . | . |
| 21-225_35F11_LC | . | . | . | . | . | . | . | . | . | . | . | . | . | . | . | . | . | . | . | . | . | . | . | . | . | . |
| 21-225_36A5_LC | . | . | . | . | S | . | . | . | . | . | . | . | . | . | . | . | . | . | . | . | . | . | . | . | . | . |
| 21-225_36C5_LC | . | . | . | . | . | . | . | . | . | . | . | . | . | . | . | . | . | . | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | . | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . | G | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | . | . | . | . | . | . | . | . | . | . | . | . | . | . | . |
| 21-225_46F3_LC | . | . | . | . | . | . | . | . | . | . | . | . | . | . | . | . | . | . | . | . | . | . | . | . | . | . |
| 21-225_47A1_LC | . | . | . | . | . | . | . | . | . | . | . | . | . | . | S | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | T | . | . | . | . | . | . | . | . | I |
| 21-225_48H4_LC | . | . | . | . | . | . | . | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . | . | . | S | . | . | . | . |
| 21-225_4C8_LC | . | . | . | . | . | . | . | . | . | . | . | . | . | . | . | . | . | . | . | . | . | . | . | . | . | . |
| 21-225_4E10_LC | . | . | . | . | . | . | . | . | . | . | . | . | . | . | . | . | . | . | . | . | . | . | . | . | . | . |
| 21-225_4F12_LC | . | . | . | . | . | . | . | . | . | . | . | . | . | . | . | . | . | . | . | . | . | . | . | . | . | . |
| 21-225_4F4_LC | . | . | . | . | . | . | . | . | . | . | . | . | . | . | . | . | . | V | . | T | . | . | . | . | . | . |
| 21-225_4H4_LC | . | . | . | . | . | . | . | . | . | . | . | . | . | . | . | . | . | V | . | . | . | . | . | . | . | . |
| 21-225_50G10_LC | . | . | . | . | . | . | . | . | . | . | . | . | . | . | . | . | . | . | . | . | . | . | . | . | . | . |
| 21-225_51F11_LC | . | . | . | . | . | . | . | . | . | . | . | . | . | . | . | . | . | . | . | . | . | . | . | . | . | . |
| 21-225_52B2_LC | S | . | . | . | . | . | . | . | . | . | . | . | . | . | . | . | S | . | . | . | . | . | . | . | T | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | N | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_54C6_LC | . | . | . | . | . | . | . | . | . | . | . | . | . | . | . | . | N | . | . | . | . | . | . | . | . | . |
| 21-225_55B8_LC | . | . | . | . | H | . | . | . | . | . | . | . | . | . | . | F | . | . | . | . | . | . | . | . | . | . |
| 21-225_55H6_LC | . | . | . | . | . | G | . | . | . | . | . | . | . | . | . | . | . | . | . | . | . | . | . | . | . | . |
| 21-225_56F1_LC | . | . | . | . | N | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | . | . | . | . | . | . | . | . | . | . | . |
| 21-225_5H7_LC | . | . | . | . | . | V | . | . | . | . | . | . | . | . | T | . | . | . | . | G | . | . | . | . | . | . |
| 21-225_60E8_LC | . | . | . | . | . | . | . | . | . | . | . | . | . | . | . | . | . | . | . | . | . | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | F | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | . | . | . | . | . | . | . | . | . | . | . | . | . | . |
| 21-225_6D4_LC | . | . | . | . | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | T | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | . | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | T | . | . | . | . | . | . | . | . | . |
| 21-225_75H7_LC | Q | . | . | . | . | D | . | . | . | . | . | . | . | . | . | . | . | . | . | . | . | . | . | . | . | . |
| 21-225_7C4_LC | . | . | . | . | . | . | . | . | . | . | . | . | . | . | . | . | . | . | . | . | . | . | . | . | . | . |
| 21-225_7E9_LC | . | . | . | . | T | . | . | . | . | . | . | . | . | . | . | . | . | . | . | . | . | . | . | . | . | . |
| 21-225_7F4_LC | . | . | . | . | . | . | . | . | . | . | . | . | . | . | . | N | . | . | . | . | . | . | . | . | . | . |
| 21-225_7H11_LC | . | . | I | . | . | V | . | . | . | . | . | . | . | . | C | . | F | . | . | . | . | . | . | . | . | . |
| 21-225_7H7_LC | . | . | . | . | . | . | . | . | . | . | . | . | . | . | . | . | . | . | . | . | . | . | T | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_8C9_LC | . | . | . | . | . | . | . | . | . | . | . | . | . | . | . | . | . | . | . | . | . | . | . | . | . | . |
| 21-225_8D12_LC | . | . | . | . | . | . | . | . | . | . | . | . | . | . | . | . | . | . | . | . | . | . | . | . | . | . |
| 21-225_8F11_LC | . | . | . | . | F | Q | . | . | . | . | . | . | . | . | . | . | . | V | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | T | . | . | . | . | . | . | . | . | . |
| 21-225_9E8_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | E | F | T | L | T | I | S | S | L | Q | P | E | D | F | A | T | Y |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . | . | . | . | . | . | . | . |
| 21-225_11F10_LC | . | . | . | . | . | . | . | . | . | . | . | . | . | . | V | . | . | . | . | . | . | . | . | . | . | . |
| 21-225_11F5_LC | . | . | . | . | . | . | . | . | . | . | . | . | . | . | . | . | . | . | . | . | . | . | . | . | i | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | . | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | i | . | . | . | . | . | . | . | . | . | . | . | . | . | . | . |
| 21-225_146F1_LC | . | . | G | . | . | . | . | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | T | . | . | . | . | . | . | . | . | . | . | . | F | . | . | . | . | V | . | . | . | . | . | . | . | . |
| 21-225_148F12_LC | . | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | . | . | . | . | . | . | . | . | i | . |
| 21-225_149G1_LC | . | . | . | . | . | . | . | . | . | . | . | . | . | . | . | . | . | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | . | . | . | . | . | . | . | . | . | . | . | . | . | . | . | . | . | . | . | . | . | . |
| 21-225_14B2_LC | . | . | . | . | . | . | . | . | . | . | . | . | . | . | . | . | . | . | . | . | . | . | . | . | . | . |
| 21-225_14C2_LC | . | . | . | . | . | . | . | . | . | . | . | . | . | . | . | . | . | V | . | R | . | . | . | . | . | . |
| 21-225_14E3_LC | i | . | . | . | G | . | . | . | . | . | . | . | . | . | . | . | . | . | . | R | . | . | . | . | A | . |
| 21-225_150E7_LC | . | . | . | . | . | . | . | . | G | . | . | . | . | . | . | . | . | . | . | . | . | . | . | . | A | . |
| 21-225_152H9_LC | T | . | . | . | . | . | . | . | . | . | . | . | . | F | . | . | . | . | . | . | . | . | . | . | . | . |
| 21-225_156G1_LC | . | . | . | . | . | . | . | . | . | . | . | . | . | F | . | . | . | . | . | . | . | . | . | . | . | . |
| 21-225_158H5_LC | . | . | . | . | C | . | . | . | . | . | . | . | . | . | . | . | . | V | . | R | . | . | . | . | S | . |
| 21-225_15A1_LC | . | . | . | . | . | . | . | . | . | . | . | . | . | . | V | . | . | . | . | . | . | . | . | . | S | . |
| 21-225_15C9_LC | . | . | . | . | . | . | . | . | . | . | . | . | . | . | V | . | . | . | . | . | . | . | . | . | . | . |
| 21-225_15E3_LC | . | . | . | : | . | . | . | . | . | . | . | . | . | . | . | . | . | . | . | . | . | . | . | L | A | . |
| 21-225_15G7_LC | . | . | . | . | F | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | E | F | T | L | T | I | S | S | L | Q | P | E | D | F | A | T | Y |
| 21-225_15H1_LC | N | . | . | . | . | . | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | S | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | . | . | . | . | . | . | . | . | . | . | . | N | . | . | . | . | . | . | . | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | . | . | . | . | . | . | . | . | . | . | . | G | . | . | . | . | . | . | . | . | . |
| 21-225_169F9_LC | . | . | . | . | . | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | . | . | . | . | . | . | . | . | . | . | I | . | . | . | . | V | . | . | . | . | . | . | . | . |
| 21-225_170D11_LC | . | . | . | . | . | . | . | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . | . | . | . | . | . | . | . |
| 21-225_170G4_LC | . | . | . | . | . | . | . | . | . | . | . | . | . | . | . | . | . | . | . | . | . | . | . | . | . | . |
| 21-225_170G5_LC | . | . | . | . | . | . | . | . | . | . | . | . | . | . | . | . | . | . | . | . | . | . | . | . | . | . |
| 21-225_171A4_LC | . | . | . | . | . | . | . | . | . | . | . | . | . | . | . | . | . | . | . | . | . | . | . | . | . | . |
| 21-225_171C3_LC | . | . | . | . | . | . | . | . | . | . | . | . | . | . | . | . | . | . | . | . | . | . | . | . | . | . |
| 21-225_172B3_LC | . | . | . | . | . | . | . | . | . | . | . | . | . | . | . | . | G | . | . | . | . | . | . | . | . | . |
| 21-225_172G8_LC | . | . | . | . | . | . | . | . | . | . | . | . | . | . | . | . | . | . | . | . | . | . | . | . | . | . |
| 21-225_173H12_LC | . | . | . | . | . | . | . | . | . | . | . | . | . | . | . | . | . | . | . | . | . | . | . | . | S | . |
| 21-225_175G1_LC | . | . | . | . | . | . | . | . | . | . | . | . | . | . | . | . | . | . | . | . | . | . | . | . | . | . |
| 21-225_176B11_LC | . | . | . | . | . | . | . | . | . | . | . | . | . | . | L | . | . | . | . | . | . | . | . | . | . | . |
| 21-225_178B10_LC | . | . | . | . | . | . | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | S | S | . | . | . | . | . | . | . | . | . | . | . | . | . | . |
| 21-225_17F9_LC | . | . | . | . | . | . | . | . | . | . | . | . | . | . | . | . | N | . | . | . | . | . | . | . | . | . |
| 21-225_17H12_LC | . | . | I | . | . | . | . | . | . | . | . | . | . | . | . | T | G | . | . | . | . | . | . | . | . | . |
| 21-225_17H8_LC | . | . | . | . | . | . | . | . | . | . | . | . | . | . | . | . | . | . | . | . | . | . | . | T | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_18A1_LC | . | . | . | . | . | . | | | | | | | | | | | | | | | | | | | | |
| 21-225_18A3_LC | N | . | . | . | . | . | . | . | . | . | . | . | . | . | . | . | . | . | . | . | . | . | . | . | . | . |
| 21-225_18C6_LC | . | | . | | . | | | | . | | | | | | | | | | | | . | | | . | . | . |
| 21-225_18E8_LC | . | | . | | . | | | | . | | | | | | | | | | | | . | | | . | . | . |
| 21-225_190C8_LC | . | | . | | . | | | | . | | | | | | | | | | | | . | | | M | . | . |
| 21-225_193A5_LC | . | | . | | S | | | | . | | | | I | | | | | V | | | . | | | M | . | . |
| 21-225_193E7_LC | . | | . | | . | | | | . | | | | | | | | | | | | . | | | . | . | . |
| 21-225_196C10_LC | . | | . | | . | | | | . | | | | | | | | | | | | . | | | . | . | . |
| 21-225_199E3_LC | . | | . | | . | | | | . | | | | | | | | | | | | . | | | . | . | . |
| 21-225_19D11_LC | . | | . | | . | | | | . | | | | | | | | | | | | . | | | . | . | . |
| 21-225_19E1_LC | . | | . | | . | | | | . | | | | | | | | | | | | . | | | . | . | . |
| 21-225_19F3_LC | . | | . | | . | | | | . | | | | | | | | | | | | . | | | . | . | . |
| 21-225_1A2_LC | . | | . | | . | | | | . | | | | | | | | | | | | . | | | . | . | . |
| 21-225_1C12_LC | . | | . | | . | | | | . | | | | | | | | | | | | . | | | . | . | . |
| 21-225_1F2_LC | . | | . | | . | | | | . | | | | | | | | | | | | . | | | . | I | . |
| 21-225_200F12_LC | . | | . | | . | | | | . | | | | | | | | | | | | . | | | . | . | . |
| 21-225_200H9_LC | . | | . | | . | | | | . | | | | | | | | | | | | . | | | . | . | . |
| 21-225_201A1_LC | . | | . | | . | | | | . | | | | | | | | | | | | . | | | . | . | . |
| 21-225_201A10_LC | . | | . | | . | | | | . | | | | | | | | | | | | . | | | . | . | . |
| 21-225_204H8_LC | . | S | . | | . | | | | . | | | | | V | | | | | | | . | | | . | . | . |
| 21-225_205H8_LC | . | | . | | . | | | | . | | | | | | | | | | | | . | | S | . | A | . |
| 21-225_20A10_LC | . | | . | | . | | | | . | | | | | | | | | | | | . | | | . | . | . |
| 21-225_20A7_LC | . | | . | | . | | | | . | | S | | | | | | | | | | . | | | . | . | . |
| 21-225_20C2_LC | . | | . | | . | | | | . | | | | | | | | | | | | . | | | . | . | . |
| 21-225_20C8_LC | . | | . | | . | | | | . | | | | | | | | | | | | . | | | . | . | . |
| 21-225_20D5_LC | . | | . | | . | | | | . | | | | | | | | | | | | . | | | . | . | . |
| 21-225_20E12_LC | . | | . | | . | | | | . | | | | | | | | | | | | . | | | . | . | . |
| 21-225_20E5_LC | . | | . | | . | | | | . | | | | | | | | | | | | . | | | . | . | . |
| 21-225_20G2_LC | . | | . | | . | | | | . | | | | | | | | | | | | . | | | . | S | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_20G9_LC | . | . | . | . | . | . | . | . | . | . | . | . | . | . | . | . | . | . | . | . | . | . | . | . | . | . |
| 21-225_20H10_LC | . | . | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | . | . | . | . | O | . | I | . | . | . | . | . | . | . | . | . | . | . | . | . | . |
| 21-225_21G7_LC | N | . | . | . | . | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | A | . | . | L | . | I | . | . |
| 21-225_224A1_LC | . | . | . | . | . | . | . | . | . | . | . | . | . | . | . | . | . | . | . | . | . | . | . | . | . | . |
| 21-225_224B1_LC | . | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | . | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | . | . |
| 21-225_22D12_LC | . | . | . | . | . | . | . | . | . | O | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | S | . | . | . | . | . | . | . | . | . | . | . | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | L | . |
| 21-225_23A3_LC | . | . | . | . | . | . | . | . | . | . | . | . | . | . | . | . | . | . | . | . | . | . | . | . | I | . |
| 21-225_23C8_LC | . | . | . | . | . | . | . | . | . | . | . | . | . | . | . | . | . | . | . | . | . | . | . | V | I | . |
| 21-225_23D1_LC | . | . | . | . | . | . | . | . | . | . | . | . | . | . | V | . | . | . | . | . | . | . | . | . | . | . |
| 21-225_23H11_LC | . | . | . | . | . | . | . | . | . | . | . | . | . | . | . | . | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | R | . | . | . | . | . | . | . | . | . | . | . | . | . | . | N | . | . | . | . | . | . | . | . | . | . |
| 21-225_25H10_LC | . | . | . | . | . | . | . | . | . | . | . | . | . | . | . | . | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_29B8_LC | . | . | . | R | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | . | . | . | . | . | . | . | . | . | Q | . | . | . | . | . | . | . | . | . | . | . | . | . | . | . | . |
| 21-225_2B2_LC | . | . | . | . | . | . | . | . | . | . | . | . | . | . | . | . | . | . | . | . | . | . | . | L | . | . |
| 21-225_2G9_LC | . | . | . | F | . | . | . | . | . | . | . | . | . | . | . | . | . | . | L | . | . | . | . | . | . | . |
| 21-225_30D1_LC | . | . | . | . | . | . | . | . | . | . | . | . | . | . | . | . | . | . | . | . | . | . | . | Y | . | . |
| 21-225_30E3_LC | . | . | . | . | . | . | . | . | . | . | . | . | . | . | . | . | . | . | . | . | . | . | . | . | . | . |
| 21-225_30E9_LC | . | . | . | . | . | . | . | . | . | . | . | . | . | . | . | . | . | . | . | . | . | . | . | . | . | . |
| 21-225_30F3_LC | . | . | . | . | . | . | . | . | . | Q | . | . | . | . | . | . | . | . | . | . | . | . | . | . | S | . |
| 21-225_30G11_LC | . | . | . | . | . | . | . | . | . | . | . | . | . | . | . | G | . | . | . | . | . | . | . | . | . | . |
| 21-225_31A8_LC | . | . | . | . | . | . | . | . | . | . | . | . | . | . | . | . | . | . | . | . | . | . | . | . | . | . |
| 21-225_31B8_LC | . | . | . | . | . | . | . | . | . | . | . | . | . | . | . | . | . | . | . | . | . | . | . | . | . | . |
| 21-225_31H3_LC | . | . | . | . | . | . | . | . | . | . | . | . | . | . | . | . | . | . | . | . | . | . | . | . | . | . |
| 21-225_31H5_LC | . | V | . | . | . | . | . | . | . | . | . | F | . | . | . | . | M | . | . | . | D | . | . | S | N | . |
| 21-225_32G1_LC | . | . | . | . | . | . | . | . | . | . | . | . | . | . | . | . | . | . | . | . | . | . | . | . | . | . |
| 21-225_32G12_LC | . | . | . | . | . | . | . | . | . | . | . | . | . | . | . | . | . | . | . | . | . | . | . | . | . | . |
| 21-225_33A4_LC | . | . | . | . | . | . | . | . | . | . | . | . | . | . | . | . | . | . | . | . | . | . | . | . | R | . |
| 21-225_33A7_LC | . | . | . | . | . | . | . | . | . | . | . | . | . | . | . | . | . | . | . | . | . | . | . | . | A | . |
| 21-225_33B1_LC | . | . | . | . | . | . | . | . | . | . | . | . | . | . | . | . | . | . | . | . | . | . | . | . | . | . |
| 21-225_33B8_LC | . | . | . | . | . | . | . | . | . | . | . | . | . | . | . | . | . | . | . | . | . | . | . | . | . | . |
| 21-225_33F1_LC | . | . | . | . | . | . | . | . | . | . | . | . | . | . | . | . | . | . | . | . | . | . | . | . | I | . |
| 21-225_33G7_LC | . | . | . | . | . | . | . | . | . | . | . | . | . | . | . | . | . | . | . | . | . | . | . | . | . | . |
| 21-225_34A6_LC | . | . | . | . | . | . | . | . | . | . | . | . | . | . | V | . | . | . | . | . | . | . | . | . | . | . |
| 21-225_34D11_LC | . | . | . | . | . | . | . | . | . | . | . | . | . | . | V | I | . | . | . | . | . | . | . | . | . | . |
| 21-225_34G8_LC | . | . | . | . | . | . | . | . | . | . | . | . | . | . | . | . | . | . | . | . | . | . | . | . | . | . |
| 21-225_34G9_LC | Y | . | . | . | . | . | . | . | . | . | . | . | . | . | . | . | . | . | . | . | . | . | A | . | I | . |
| 21-225_34H11_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_35A6_LC | . | . | . | . | . | . | . | . | . | . | . | . | . | . | . | . | . | . | . | . | . | . | . | . | . | . |
| 21-225_35B7_LC | . | . | . | . | . | . | . | . | . | . | . | . | . | . | . | . | . | . | . | . | . | . | . | . | . | . |
| 21-225_35D1_LC | . | . | . | . | . | . | . | . | . | . | . | . | . | . | . | . | . | . | . | . | . | . | . | . | . | . |
| 21-225_35F11_LC | . | . | . | . | . | . | . | . | . | . | . | . | . | . | . | . | . | . | . | . | . | . | . | . | . | . |
| 21-225_36A5_LC | . | . | . | . | . | . | . | . | . | . | . | . | . | . | . | . | . | . | . | . | . | . | . | . | . | . |
| 21-225_36C5_LC | Y | . | . | . | . | . | . | . | . | . | . | . | . | . | . | . | . | V | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | G | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . | . | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | S | . | . | . | . | . | . | . | . | . | . | . | . | . | . |
| 21-225_46F3_LC | . | . | . | . | . | . | . | . | . | . | . | S | . | . | . | . | . | . | . | . | . | . | . | . | . | . |
| 21-225_47A1_LC | . | . | . | R | . | . | . | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . | . | . | . | . | . | . | . |
| 21-225_48H4_LC | . | . | . | . | . | . | . | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . | . | . | . | . | . | . | . |
| 21-225_4C8_LC | . | . | . | V | . | . | . | . | . | . | . | . | . | . | . | . | . | . | . | . | . | . | . | . | . | . |
| 21-225_4E10_LC | . | . | . | . | P | . | . | . | . | . | . | . | . | . | . | . | . | . | . | L | . | . | . | . | . | . |
| 21-225_4F12_LC | . | . | G | . | . | . | . | . | . | I | . | . | . | . | . | . | . | . | . | . | . | . | . | . | . | . |
| 21-225_4F4_LC | . | . | . | . | . | . | . | . | . | . | . | . | . | . | . | . | . | . | . | . | . | . | . | . | . | I |
| 21-225_4H4_LC | . | . | . | . | . | . | . | . | . | . | . | . | . | . | . | . | . | . | . | . | . | . | . | . | . | I |
| 21-225_50G10_LC | . | . | . | . | . | . | . | . | . | . | . | . | . | . | . | . | . | . | . | . | . | . | . | . | . | . |
| 21-225_51F11_LC | . | . | . | . | . | . | . | . | . | . | . | . | . | . | . | . | . | . | . | . | . | . | . | . | . | . |
| 21-225_52B2_LC | . | . | . | . | . | . | . | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_54C6_LC | . | . | . | . | . | . | . | . | . | . | . | . | . | . | . | . | . | . | . | . | . | . | . | . | . | . |
| 21-225_55B8_LC | . | . | . | . | . | . | . | . | . | . | . | . | . | . | . | . | . | . | . | . | . | . | . | . | . | . |
| 21-225_55H6_LC | . | . | . | . | . | . | . | . | . | . | . | . | . | . | . | . | . | . | . | . | . | . | . | . | A | . |
| 21-225_56F1_LC | . | . | . | . | . | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | S | . | . | . | . | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | . | . | . | . | . | . | . | . | . | . | . | . | . | . | . | . | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | R | . | . | . | . | . | . | . | . | . | . | . | . | . | . | . | . | . | . | L | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | P | . | . | . | . | S | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | . | . | . | . | . | . | . | . | . | . | . | . | . | . |
| 21-225_6D4_LC | . | . | . | . | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | . | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | F | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | G | . | . | . | . | . | . | . | . | . |
| 21-225_75H7_LC | . | . | . | . | . | . | . | . | . | . | . | . | . | . | . | . | . | V | . | . | . | . | . | G | V | . |
| 21-225_7C4_LC | . | . | . | . | . | . | . | . | . | . | . | . | . | . | . | . | . | . | . | . | . | . | . | . | . | . |
| 21-225_7E9_LC | . | . | . | . | . | . | . | . | . | . | . | . | . | . | . | . | . | . | . | . | . | . | . | . | . | . |
| 21-225_7F4_LC | . | . | . | . | . | . | . | . | . | . | . | . | . | . | . | . | . | . | . | . | . | . | . | . | . | . |
| 21-225_7H11_LC | . | . | . | Y | . | . | . | . | . | . | . | . | . | . | . | I | M | . | . | . | . | . | . | . | . | . |
| 21-225_7H7_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_8C9_LC | . | . | . | . | . | . | . | . | . | . | . | . | . | . | . | . | . | . | . | Ÿ | . | . | . | . | . | . |
| 21-225_8D12_LC | N | . | . | . | . | . | . | . | . | . | . | . | . | . | . | . | . | . | . | . | . | . | . | . | . | . |
| 21-225_8F11_LC | . | . | . | . | . | . | . | . | . | . | . | . | . | . | . | . | . | . | . | . | . | . | . | . | ⁚ | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | ⊚ | . | . | ▨ | . | . | . | . | . | . |
| 21-225_9E8_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . | . | . | . | . | . | . | . |
| 21-225_11F10_LC | . | . | . | . | . | S | . | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . | . | . | . | . | . | . | . |
| 21-225_11G7_LC | . | . | . | . | . | S | N | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | V | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | . | . | . | . | . | . | . |
| 21-225_146F1_LC | . | . | . | . | . | S | T | . | . | . | . | . | . | . | . | . | . | . | . | . | . | . | . | . | . | . |
| 21-225_146G11_LC | . | . | . | . | . | O | . | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | . | . | . | N | . | . | . | . | . | . | . | . | . | . | . | . | . | . | . | . | . | . | . |
| 21-225_148F12_LC | F | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | . | . | . | . | . | . | . | . | . | . |
| 21-225_149G1_LC | . | . | . | . | . | . | . | . | . | . | . | . | . | . | . | . | . | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | . | Y | N | . | . | . | . | . | . | . | . | . | . | . | . | . | . | . | . | . | . | . |
| 21-225_14B2_LC | . | . | . | . | . | . | . | . | . | . | . | . | . | . | . | . | . | . | . | . | . | . | . | . | . | . |
| 21-225_14C2_LC | . | . | V | . | . | . | . | . | . | . | . | . | . | . | . | . | . | . | . | . | . | . | . | . | . | . |
| 21-225_14E3_LC | . | S | V | . | . | Y | . | . | . | . | . | . | . | . | . | . | . | . | . | . | . | . | . | . | . | . |
| 21-225_150E7_LC | . | . | . | . | . | . | . | . | . | . | . | . | . | . | . | . | . | . | . | . | . | . | . | . | . | . |
| 21-225_152H9_LC | . | . | . | . | . | S | N | . | . | . | . | . | . | . | . | . | . | . | . | . | . | . | . | . | . | . |
| 21-225_156G1_LC | . | . | . | . | . | S | N | . | . | . | . | . | . | . | . | . | . | . | . | . | . | . | . | . | . | . |
| 21-225_158H5_LC | . | . | V | . | . | . | . | . | . | . | . | . | . | . | . | . | . | . | . | . | . | . | . | . | . | . |
| 21-225_15A1_LC | . | . | . | . | N | S | . | . | . | . | . | . | . | . | . | . | . | . | . | . | . | . | . | . | . | . |
| 21-225_15C9_LC | . | . | . | . | . | T | . | . | . | . | . | . | . | . | . | . | . | . | . | . | . | . | . | . | . | . |
| 21-225_15E3_LC | . | . | . | . | . | . | . | . | . | . | . | . | . | . | . | . | . | . | . | . | . | . | . | . | . | . |
| 21-225_15G7_LC | . | . | . | . | . | S | N | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_15H1_LC | . | . | . | . | . | S | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | S | . | . | . | . | . | . | . | . | . | . | . | . | . | . | . | . | . | . | . | . |
| 21-225_160H3_LC | . | . | . | . | . | S | N | . | . | . | . | . | . | . | . | . | . | . | . | . | . | . | . | . | . | . |
| 21-225_169B1_LC | . | . | . | . | . | Y | . | . | . | . | . | . | . | . | . | . | . | . | . | . | . | . | . | . | . | . |
| 21-225_169F9_LC | . | . | . | . | . | Y | . | . | . | . | . | . | . | . | . | . | . | . | . | . | . | . | . | . | . | . |
| 21-225_16A1_LC | . | . | . | :: | . | S | N | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | ? | . | . | . | . | . | . | . | . | . | . | . | . | . | . | . | . | . | . | . | . | . | . | . |
| 21-225_170D11_LC | . | . | . | . | . | Y | . | . | . | . | . | . | . | . | . | . | . | . | . | . | . | . | . | . | . | . |
| 21-225_170D5_LC | . | . | . | . | . | Y | . | . | . | . | . | . | . | . | . | . | . | . | . | . | . | . | . | . | . | . |
| 21-225_170G4_LC | . | . | . | . | . | Y | . | . | . | . | . | . | . | . | . | . | . | . | . | . | . | . | . | . | . | . |
| 21-225_170G5_LC | . | . | . | . | . | Y | . | . | . | . | . | . | . | . | . | . | . | . | . | . | . | . | . | . | . | . |
| 21-225_171A4_LC | . | . | . | . | . | Y | . | . | . | . | . | . | . | . | . | . | . | . | . | . | . | . | . | . | . | . |
| 21-225_171C3_LC | . | . | . | . | . | Y | . | . | . | . | . | . | . | . | . | . | . | . | . | . | . | . | . | . | . | . |
| 21-225_172B3_LC | . | . | . | . | . | Y | . | . | . | . | . | . | . | . | . | . | . | . | . | . | . | . | . | . | . | . |
| 21-225_172G8_LC | . | . | . | :: | . | Y | . | . | . | . | . | . | . | . | . | . | . | . | . | . | . | . | . | . | . | . |
| 21-225_173H12_LC | . | . | . | . | . | Y | . | . | . | . | . | . | . | . | . | . | . | . | . | . | . | . | . | . | . | . |
| 21-225_175G1_LC | . | . | . | . | . | Y | . | . | . | . | . | . | . | . | . | . | . | . | . | . | . | . | . | . | . | . |
| 21-225_176B11_LC | . | . | . | . | . | Y | . | . | . | . | . | . | . | . | . | . | . | . | . | . | . | . | . | . | . | . |
| 21-225_178B10_LC | . | . | . | :: | . | Y | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . | . | . | . | . | . | . | . |
| 21-225_17F9_LC | . | . | . | . | . | . | T | . | . | . | . | . | . | . | . | . | . | . | . | . | . | . | . | . | . | . |
| 21-225_17H12_LC | . | . | . | . | . | Y | . | . | . | . | . | . | . | . | . | . | . | . | . | . | . | . | . | . | . | . |
| 21-225_17H8_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_20G9_LC | . | . | . | . | . | S | T | . | . | . | . | . | . | . | . | . | . | . | . | . | . | . | . | . | . | . |
| 21-225_20H10_LC | . | . | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | S | . | . | . | . | . | . | . | . | . | . | . | . | . | . | . | . | . | . | . | . |
| 21-225_21G7_LC | . | . | . | . | . | S | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | . | . | . | . | . | . | . | . |
| 21-225_224A1_LC | . | . | . | . | . | . | . | . | . | . | . | . | . | . | . | . | . | . | . | . | . | . | . | . | . | . |
| 21-225_224B1_LC | . | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | . | T | . | . | . | . | . | . | . | . | . | . | . | . | . | . | . | . | . | . | . |
| 21-225_22C1_LC | . | . | . | . | . | S | T | . | . | . | . | . | . | . | . | . | . | . | . | . | . | . | . | . | . | . |
| 21-225_22D12_LC | . | . | . | . | . | S | T | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . | . | . | . | . | . | . | . |
| 21-225_22G9_LC | . | . | . | . | . | A | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | . | . |
| 21-225_23A3_LC | . | . | . | . | . | . | . | . | . | . | . | . | . | . | . | . | . | . | . | . | . | . | . | . | . | . |
| 21-225_23C8_LC | . | . | . | . | . | . | . | . | . | . | . | . | . | . | . | . | . | . | . | . | . | . | . | . | . | . |
| 21-225_23D1_LC | . | . | . | . | . | S | . | . | . | . | . | . | . | . | . | . | . | . | . | . | . | . | . | . | . | . |
| 21-225_23H11_LC | . | . | . | . | . | . | N | . | . | . | . | . | . | . | . | . | . | . | . | . | . | . | . | . | . | . |
| 21-225_24E5_LC | . | . | . | . | . | S | N | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | . | . | . | . | . | . | . |
| 21-225_25H10_LC | . | . | . | . | . | . | . | . | . | . | . | . | . | . | . | . | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | N | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | . | . | . | . | . | . | . | . | . | . | . | . | . | . | . | . | . | . | . | . | . | . | . | . | . | . |
| 21-225_2B2_LC | H | . | . | . | . | . | . | . | . | . | . | . | . | . | . | . | . | . | . | . | . | . | . | . | . | . |
| 21-225_2G9_LC | . | . | . | . | . | S | . | . | . | . | . | . | . | . | . | . | . | . | . | . | . | . | . | . | . | . |
| 21-225_30D1_LC | . | . | . | . | . | T | S | . | . | . | . | . | . | . | . | . | . | . | . | . | . | . | . | . | . | . |
| 21-225_30E3_LC | . | . | . | . | . | T | . | . | . | . | . | . | . | . | . | . | . | . | . | . | . | . | . | . | . | . |
| 21-225_30E9_LC | . | . | . | . | . | T | . | . | . | . | . | . | . | . | . | . | . | . | . | . | . | . | . | . | . | . |
| 21-225_30F3_LC | . | . | . | . | . | . | . | . | . | . | . | . | . | . | . | . | . | . | . | . | . | . | . | . | . | . |
| 21-225_30G11_LC | F | . | . | . | . | T | S | . | . | . | . | . | . | . | . | . | . | . | . | . | . | . | . | . | . | . |
| 21-225_31A8_LC | . | . | . | . | . | T | S | . | . | . | . | . | . | . | . | . | . | . | . | . | . | . | . | . | . | . |
| 21-225_31B8_LC | . | . | . | . | . | S | S | . | . | . | . | . | . | . | . | . | . | . | . | . | . | . | . | . | . | . |
| 21-225_31H3_LC | . | . | . | . | . | T | . | . | . | . | . | . | . | . | . | . | . | . | . | . | . | . | . | . | . | . |
| 21-225_31H5_LC | . | S | . | . | . | S | S | . | . | . | . | . | . | . | . | . | . | . | . | . | . | . | . | . | . | . |
| 21-225_32G1_LC | . | . | . | . | . | T | S | . | . | . | . | . | . | . | . | . | . | . | . | . | . | . | . | . | . | . |
| 21-225_32G12_LC | . | . | . | . | . | T | S | . | . | . | . | . | . | . | . | . | . | . | . | . | . | . | . | . | . | . |
| 21-225_33A4_LC | . | . | . | . | . | Y | . | . | . | . | . | . | . | . | . | . | . | . | . | . | . | . | . | . | . | . |
| 21-225_33A7_LC | . | . | . | . | . | T | S | . | . | . | . | . | . | . | . | . | . | . | . | . | . | . | . | . | . | . |
| 21-225_33B1_LC | . | . | . | . | . | . | . | . | . | . | . | . | . | . | . | . | . | . | . | . | . | . | . | . | . | . |
| 21-225_33B8_LC | F | . | . | . | . | T | S | . | . | . | . | . | . | . | . | . | . | . | . | . | . | . | . | . | . | . |
| 21-225_33F1_LC | . | . | . | . | . | T | S | . | . | . | . | . | . | . | . | . | . | . | . | . | . | . | . | . | . | . |
| 21-225_33G7_LC | . | . | . | . | . | Y | . | . | . | . | . | . | . | . | . | . | . | . | . | . | . | . | . | . | . | . |
| 21-225_34A6_LC | . | . | . | . | . | . | . | . | . | . | . | . | . | . | . | . | . | . | . | . | . | . | . | . | . | . |
| 21-225_34D11_LC | . | . | . | . | . | T | S | . | . | . | . | . | . | . | . | . | . | . | . | . | . | . | . | . | . | . |
| 21-225_34G8_LC | . | . | . | . | . | T | S | . | . | . | . | . | . | . | . | . | . | . | . | . | . | . | . | . | . | . |
| 21-225_34G9_LC | . | . | . | . | . | T | S | . | . | . | . | . | . | . | . | . | . | . | . | . | . | . | . | . | . | . |
| 21-225_34H11_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
|  |  |  |  |  |  |  |  |  |  |  |  |  |  |  |  |  | K_CDR3 |  |  |  |  |  |  |  |  |  |
| CONSENSUS | Y | C | L | Q | H | N | S | - | - | - | - | - | - | - | - | - | - | - | - | - | - | - | - | - | - | - |
| 21-225_54C6_LC | F | . | . | . | . | . | S | . | . | . | . | . | . | . | . | . | . | . | . | . | . | . | . | . | . | . |
| 21-225_55B8_LC | . | . | . | . | . | . | . | . | . | . | . | . | . | . | . | . | . | . | . | . | . | . | . | . | . | . |
| 21-225_55H6_LC | . | . | . | . | N | O | . | . | . | . | . | . | . | . | . | . | . | . | . | . | . | . | . | . | . | . |
| 21-225_56F1_LC | . | . | . | . | O | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | . | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | S | . | . | . | . | . | . | . | . | . | . | . | . | . | . | . | . | . | . | . | . |
| 21-225_62E2_LC | . | . | . | . | . | S | N | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | S | T | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | S | . | . | . | . | . | . | . | . | . | . | . | . | . | . | . | . | . | . | . | . |
| 21-225_6C12_LC | F | . | . | O | . | . | . | . | . | . | . | . | . | . | . | . | . | . | . | . | . | . | . | . | . | . |
| 21-225_6D4_LC | . | . | . | . | . | S | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | N | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | D | . | . | . | . | . | . | . | . | . | . | . | . | . | . | . | . | . | . | . | . | . |
| 21-225_74D7_LC | . | . | . | . | . | S | N | . | . | . | . | . | . | . | . | . | . | . | . | . | . | . | . | . | . | . |
| 21-225_74E4_LC | N | . | . | . | . | S | N | . | . | . | . | . | . | . | . | . | . | . | . | . | . | . | . | . | . | . |
| 21-225_75H7_LC | . | . | I | . | . | S | N | . | . | . | . | . | . | . | . | . | . | . | . | . | . | . | . | . | . | . |
| 21-225_7C4_LC | . | . | . | . | . | . | . | . | . | . | . | . | . | . | . | . | . | . | . | . | . | . | . | . | . | . |
| 21-225_7E9_LC | . | . | . | . | . | . | . | . | . | . | . | . | . | . | . | . | . | . | . | . | . | . | . | . | . | . |
| 21-225_7F4_LC | . | . | . | . | . | S | A | . | . | . | . | . | . | . | . | . | . | . | . | . | . | . | . | . | . | . |
| 21-225_7H11_LC | . | . | . | . | . | . | L | . | . | . | . | . | . | . | . | . | . | . | . | . | . | . | . | . | . | . |
| 21-225_7H7_LC | . | . | . | . | . | S | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_8C9_LC | . | . | . | . | . | . | . | . | . | . | . | . | . | . | . | . | . | . | . | . | . | . | . | . | . | . |
| 21-225_8D12_LC | . | . | . | . | . | S | . | . | . | . | . | . | . | . | . | . | . | . | . | . | . | . | . | . | . | . |
| 21-225_8F11_LC | . | . | . | . | . | Y | T | . | . | . | . | . | . | . | . | . | . | . | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | S | N | . | . | . | . | . | . | . | . | . | . | . | . | . | . | . | . | . | . | . |
| 21-225_9E8_LC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | L | T | F | G | G | G | T | K | V | E | I | K | R |
| 21-225_10G5_LC | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . |
| 21-225_11F10_LC | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . |
| 21-225_11G7_LC | . | . | . | . | . | . | . | . | . | . | . | . | . | M | . | . | . | ℜ | . |
| 21-225_12A1_LC | . | . | . | . | . | . | . | . | . | . | . | . | . | . | . | . | . | . | . |
| 21-225_13E6_LC | . | . | . | . | . | . | . | . | . | . | . | . | . | . | . | A | . | . | . |
| 21-225_13H3_LC | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . |
| 21-225_146F1_LC | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . |
| 21-225_148E11_LC | . | . | . | . | . | . | . | . | . | . | . | . | . | . | . | . | . | . | . |
| 21-225_148F12_LC | . | . | . | . | . | . | . | ! | . | . | . | . | . | . | . | . | . | . | . |
| 21-225_149F12_LC | . | . | . | . | . | . | . | . | . | . | . | . | . | . | . | . | . | . | . |
| 21-225_149G1_LC | . | . | . | . | . | . | . | . | . | . | . | . | . | . | . | . | . | . | . |
| 21-225_149G5_LC | . | . | . | . | . | . | . | . | . | . | . | . | . | . | . | . | . | . | . |
| 21-225_14B1_LC1_LC | . | . | . | . | . | . | . | . | . | . | . | . | . | . | . | . | . | . | . |
| 21-225_14B2_LC | . | . | . | . | . | . | . | . | . | . | . | . | . | . | . | . | . | . | . |
| 21-225_14C2_LC | . | . | . | . | . | . | . | . | . | . | . | . | . | . | . | . | . | ℜ | . |
| 21-225_14E3_LC | . | . | . | . | . | P | . | . | . | . | . | . | . | . | . | . | . | . | . |
| 21-225_150E7_LC | . | . | . | . | . | . | . | . | . | . | . | . | . | . | . | . | . | . | . |
| 21-225_152H9_LC | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . |
| 21-225_158H5_LC | . | . | . | . | . | . | . | . | . | . | . | . | . | . | . | . | . | . | . |
| 21-225_15A1_LC | . | . | . | . | . | V | ℜ | . | . | . | . | I | . | . | . | . | . | T | G |
| 21-225_15C9_LC | . | . | . | . | . | . | . | . | . | . | . | . | . | . | . | . | . | . | . |
| 21-225_15E3_LC | . | . | . | . | . | . | . | . | . | . | . | . | E | . | . | . | . | Y | . |
| 21-225_15G7_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_15H1_LC | . | . | . | . | . | . | . | . | . | . | . | . | . | . | . | . | . | . | . |
| 21-225_15H10_LC | . | . | . | . | . | . | . | . | . | . | . | . | . | . | . | . | . | N | . |
| 21-225_15H8_LC | . | . | . | . | . | . | . | . | . | . | . | . | . | . | . | . | . | R | . |
| 21-225_160B10_LC | . | . | . | . | . | . | . | . | . | . | . | . | . | . | . | . | . | . | . |
| 21-225_160H3_LC | . | . | . | . | . | . | . | . | . | . | . | . | . | Q | . | . | . | S | . |
| 21-225_169B1_LC | . | . | . | . | . | . | . | . | . | . | . | . | . | . | . | . | . | . | . |
| 21-225_169F9_LC | . | . | . | . | . | . | . | . | . | . | . | . | . | . | . | . | . | . | . |
| 21-225_16A1_LC | . | . | . | . | . | . | . | . | . | . | . | . | . | . | . | . | . | E | . |
| 21-225_16B7_LC | . | . | . | . | . | . | . | . | . | . | . | . | . | . | . | . | . | N | . |
| 21-225_16E12_LC | . | . | . | . | . | . | . | . | . | . | . | . | . | . | . | . | R | N | . |
| 21-225_16F11_LC | . | . | . | . | . | . | . | . | . | . | . | . | . | . | . | . | . | . | . |
| 21-225_16F4_LC | . | . | . | . | . | R | . | . | . | . | . | . | . | . | . | . | . | . | . |
| 21-225_170D11_LC | . | . | . | . | R | . | . | . | . | . | . | . | . | . | . | . | . | R | . |
| 21-225_170D5_LC | . | . | . | . | R | . | . | . | . | . | . | . | . | . | . | . | . | . | . |
| 21-225_170G4_LC | . | . | . | . | . | . | . | . | . | . | . | . | . | . | . | . | . | . | . |
| 21-225_170G5_LC | . | . | . | . | . | . | . | . | . | . | . | . | . | . | . | . | . | . | . |
| 21-225_171A4_LC | . | . | . | . | . | . | . | . | . | . | . | . | . | . | . | . | . | . | . |
| 21-225_171C3_LC | . | . | . | . | . | . | . | . | . | . | . | . | . | . | . | . | . | . | . |
| 21-225_172B3_LC | . | . | . | . | . | . | . | . | . | . | . | . | . | . | . | . | . | . | . |
| 21-225_172G8_LC | . | . | . | . | R | . | . | . | . | . | . | . | . | . | . | . | . | . | . |
| 21-225_173H12_LC | . | . | . | . | R | . | N | . | . | . | . | . | . | . | . | . | . | . | . |
| 21-225_175G1_LC | . | . | . | . | . | . | . | . | . | . | . | . | . | . | . | . | . | . | . |
| 21-225_176B11_LC | . | . | . | . | . | . | . | . | . | . | . | . | . | . | . | . | . | . | . |
| 21-225_178B10_LC | . | . | . | . | R | . | . | . | . | . | . | . | . | . | . | . | . | . | . |
| 21-225_17A10_LC | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . |
| 21-225_17F9_LC | . | . | . | . | . | . | . | . | . | . | . | . | . | . | . | . | R | . | . |
| 21-225_17H12_LC | . | . | . | . | M | R | . | . | . | . | . | . | . | . | . | . | . | . | . |
| 21-225_17H8_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_18A1_LC | | | | | | | | | | | | | | | | | | | |
| 21-225_18A3_LC | | | | | | | | | | | | | | | | | | | |
| 21-225_18C6_LC | | | | | | | | | | | | | | | | | | | |
| 21-225_18E8_LC | | | | | | | | | | | | | | | | | | | |
| 21-225_190C8_LC | | | | | | | L | | | | | | | | | | | | |
| 21-225_193A5_LC | | | | | Y | | L | | | | | | | | | | | | |
| 21-225_193E7_LC | | | | | | | | | | | | | | | | | | | |
| 21-225_196C10_LC | | | | | | | | | | | | | | | | | | | |
| 21-225_199E3_LC | | | | | | | | | | | | | | | | | | | |
| 21-225_19D11_LC | | | | | | | | | | | | | | E | | | | | |
| 21-225_19E1_LC | | | | | Y | | | | | | | | | | | | | | |
| 21-225_19F3_LC | | | | | | | | | | | | | | | | | | | |
| 21-225_1A2_LC | | | | | N | | | | | | | | | | | | | | |
| 21-225_1C12_LC | | | | | | | | | | | | | | | | A | | | |
| 21-225_1F2_LC | | | | | | | | | | | | | | | | | | | |
| 21-225_200F12_LC | | | | | | | | | | | | | | | | | | | |
| 21-225_200H9_LC | | | | | | | | | | | | | | | | | | | |
| 21-225_201A1_LC | | | | | | | | | | | | | | | | | V | | |
| 21-225_201A10_LC | | | | | | | | | | | | | | | | | | | |
| 21-225_204H8_LC | | | | | | | | | | | | | | | | | | | |
| 21-225_205H8_LC | | | | | | | | | | | | | | T | | E | | | |
| 21-225_20A10_LC | | | | | | | | | | | | | | | | | | | |
| 21-225_20A7_LC | | | | | | | | | | | | | | | | | | | |
| 21-225_20C2_LC | | | | | | | | | | | | | | | | | | E | |
| 21-225_20C8_LC | | | | | | | L | | | | | | | | | | | T | |
| 21-225_20D5_LC | | | | | | | L | | | | | | | | | | | | |
| 21-225_20E12_LC | | | | | | | L | | | | | | | | | | | | |
| 21-225_20E5_LC | | | | | | | | | | | | | | | | | | | |
| 21-225_20G2_LC | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_20G9_LC | | | | | | | | | | | | | | | | | | | |
| 21-225_20H10_LC | | | | | | | | | | | | | | | | | | | |
| 21-225_20H12_LC | | | | | | | | | | | | | | | | | | | |
| 21-225_21E5_LC | | | | | | | | | | | | | | | | | | | |
| 21-225_21F3_LC | | | | | | | | | | | | | | | | | | | |
| 21-225_21G7_LC | | | | | | | | | | | | | | | | | | | |
| 21-225_21H3_LC | | | | | | | | | | | | | | | | | | | |
| 21-225_224A1_LC | | | | | | | | | | | | | | | | | | | |
| 21-225_224B1_LC | | | | | | | | | | | | | | | | | | | |
| 21-225_224C3_LC | | | | | | | | | | | | | | | | | | | |
| 21-225_224D10_LC | | | | | | | | | | | | | | | | | | | |
| 21-225_22B7_LC | | | | | | | | | | | | | | | | | | | |
| 21-225_22C1_LC | | | | | | | | | | | | | | | | | | | |
| 21-225_22D12_LC | | | | | | | | | | | | | | | | | | | |
| 21-225_22F2_LC | | | | | | | | | | | | | | | | | | | |
| 21-225_22F9_LC | | | | | | | | | | | | | | | | | | | |
| 21-225_22G8_LC | | | | | | | | | | | | | | | | | | | |
| 21-225_22G9_LC | | | | | | | | | | | | | | | | | | | |
| 21-225_22H4_LC | | | | | | | | | | | | | | | | | | | |
| 21-225_23A3_LC | | | | | | | | | | | | | | | | | | | |
| 21-225_23C8_LC | | | | | | | | | | | | | | | | | | | |
| 21-225_23D1_LC | | | | | | | | | | | | | | | | | | | |
| 21-225_23H11_LC | | | | | | | | | | | | | | | | | | | |
| 21-225_24E5_LC | | | | | | | | | | | | | | | | | | | |
| 21-225_25E6_LC | | | | | | | | | | | | | | | | | | | |
| 21-225_25H10_LC | | | | | | | | | | | | | | | | | | | |
| 21-225_25H11_LC | | | | | | | | | | | | | | | | | | | |
| 21-225_27C3_LC | | | | | | | | | | | | | | | | | | | |
| 21-225_27F2_LC | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_29B8_LC | | | | | | | | | | | | | | | | | | | |
| 21-225_29G4_LC | | | | | | | | | | | | | | | | | | T | |
| 21-225_29G8_LC | | | | | | | | | | | | | | | | | | | |
| 21-225_29H6_LC | | | | | | | | | | | | | | | | | | | |
| 21-225_2A11_LC | | | | | | | | | | | | | | | S | | | | |
| 21-225_2B2_LC | | | | | | | | | | | | | | | | | | | |
| 21-225_2G9_LC | | | | | | | | | | | | | | | | | | | |
| 21-225_30D1_LC | | | | | | | p | | | | | | | | | | | | |
| 21-225_30E3_LC | | | | | | | p | | | | | | | | | | | | |
| 21-225_30E9_LC | | | | | | | p | | | | | | | | | | | | |
| 21-225_30F3_LC | | | | | | | p | | | | | | | | | | | | |
| 21-225_30G11_LC | | | | | | | p | | | | | | | | | | | | |
| 21-225_31A8_LC | | | | | | | p | | | | | | | | | | | | |
| 21-225_31B8_LC | | | | | | | p | | | | | | | | | | | | |
| 21-225_31H3_LC | | | | | | | p | | | | | | | | | | | | |
| 21-225_31H5_LC | | | | | | | p | | | | | | | | | | | | |
| 21-225_32G1_LC | | | | | S | | p | | | | | | | | | | | | |
| 21-225_32G12_LC | | | | | | | p | | | | | | | | | | | | |
| 21-225_33A4_LC | | | | | | | p | | | | | | | | | | | | |
| 21-225_33A7_LC | | | | | | | p | | | | | | | | | | | | |
| 21-225_33B1_LC | | | | | | | | | | | | | | | | | V | | |
| 21-225_33B8_LC | | | | | | | p | | | | | | | | | | | | |
| 21-225_33F1_LC | | | | | | | p | | | | | | | | | | | | |
| 21-225_33G7_LC | | | | | | | p | | | | | | | | | | | | |
| 21-225_34A6_LC | | | | | | | p | | | | | | | | | | | | |
| 21-225_34D11_LC | | | | | | | p | | | | | | | | G | | | | |
| 21-225_34G8_LC | | | | | | | p | | | | | | | | | | | | |
| 21-225_34G9_LC | | | | | | | p | | | | | | | | W | | | | |
| 21-225_34H11_LC | | | | | | | p | | | | | | | | | | | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_35A6_LC | . | . | . | . | . | . | P | . | . | . | . | . | . | . | . | . | . | . | . |
| 21-225_35B7_LC | . | . | . | . | . | . | P | . | . | . | . | . | . | . | . | . | . | . | . |
| 21-225_35D1_LC | . | . | . | . | . | . | P | . | . | . | . | . | . | . | . | . | . | . | . |
| 21-225_35F11_LC | . | . | . | . | . | . | P | . | . | . | . | . | . | . | . | . | . | . | . |
| 21-225_36A5_LC | . | . | . | . | . | . | P | K | . | . | . | . | I | . | . | . | . | T | Q |
| 21-225_36C5_LC | . | . | . | . | . | . | P | . | . | . | . | . | . | . | . | W | . | . | . |
| 21-225_43C3_LC | . | . | . | . | . | . | . | . | . | . | . | . | . | . | . | Q | . | T | . |
| 21-225_44C3_LC | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . |
| 21-225_46C6_LC | . | . | . | . | . | . | . | . | . | . | . | . | . | . | . | . | . | T | . |
| 21-225_46D8_LC | . | . | . | . | . | . | . | . | . | . | . | . | . | . | . | . | . | . | . |
| 21-225_46F3_LC | . | . | . | . | . | . | . | . | . | . | . | . | . | . | . | . | . | . | . |
| 21-225_47A1_LC | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . |
| 21-225_48H4_LC | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . |
| 21-225_4C8_LC | . | . | . | . | . | . | . | . | . | . | . | . | . | . | . | . | . | . | . |
| 21-225_4E10_LC | . | . | . | . | . | . | . | . | . | . | . | . | . | . | . | . | . | . | . |
| 21-225_4F12_LC | . | . | . | . | A | . | . | . | . | . | . | . | . | . | . | . | . | . | . |
| 21-225_4F4_LC | . | . | . | . | . | . | . | . | . | . | . | . | . | . | . | . | . | . | . |
| 21-225_4H4_LC | . | . | . | . | . | . | . | . | . | . | . | . | . | . | . | . | . | . | . |
| 21-225_50G10_LC | . | . | . | . | . | . | R | . | . | . | . | . | . | . | . | . | S | . | . |
| 21-225_51F11_LC | . | . | . | . | . | . | R | . | . | . | . | . | . | . | . | . | . | . | . |
| 21-225_52B2_LC | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . |
| 21-225_53E6_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_54C6_LC | . | . | . | . | . | . | . | . | . | . | . | . | . | . | . | . | . | . | . |
| 21-225_55B8_LC | . | . | . | . | . | . | . | . | . | . | . | . | . | . | . | G | . | . | . |
| 21-225_55H6_LC | . | . | . | . | . | . | F | . | . | . | . | . | . | . | . | . | . | . | . |
| 21-225_56F1_LC | . | . | . | . | F | . | F | . | . | . | . | . | . | . | . | . | . | . | . |
| 21-225_59E6_LC | . | . | . | . | . | . | . | . | . | . | . | . | R | . | . | . | . | . | . |
| 21-225_5H2_LC | . | . | . | . | . | . | . | . | . | . | . | . | . | . | . | . | . | . | . |
| 21-225_5H7_LC | . | . | . | . | . | . | . | . | . | . | . | . | . | . | . | . | . | . | . |
| 21-225_60E8_LC | . | . | . | . | . | . | . | . | . | . | . | . | . | . | . | . | . | . | . |
| 21-225_62E2_LC | . | . | . | . | . | . | . | . | . | . | . | . | R | . | . | . | . | . | . |
| 21-225_62E4_LC | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . |
| 21-225_68F11_LC | . | . | . | . | . | . | F | . | . | . | . | . | . | . | . | . | . | . | . |
| 21-225_6A6_LC | . | . | . | . | . | . | . | . | . | . | . | . | . | . | . | . | . | . | . |
| 21-225_6C12_LC | . | . | . | . | . | . | F | . | . | . | . | . | . | . | . | . | . | . | . |
| 21-225_6D4_LC | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . |
| 21-225_70E12_LC | . | . | . | . | . | . | . | . | . | . | . | . | S | . | . | . | . | . | . |
| 21-225_70E8_LC | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . |
| 21-225_74A1_LC | . | . | . | . | . | . | . | . | . | . | . | . | A | . | . | . | . | . | . |
| 21-225_74D7_LC | . | . | . | . | . | . | . | . | . | . | . | . | . | . | . | . | . | . | . |
| 21-225_74E4_LC | . | . | . | . | . | . | . | . | . | . | . | . | . | . | . | . | . | . | . |
| 21-225_75H7_LC | . | . | . | . | . | . | . | . | . | . | . | . | . | . | . | . | . | . | . |
| 21-225_7C4_LC | . | . | . | . | . | . | . | . | . | . | . | . | . | . | . | . | . | . | . |
| 21-225_7E9_LC | . | . | . | . | . | . | . | . | . | . | . | . | . | . | . | . | . | . | . |
| 21-225_7F4_LC | . | . | . | . | . | . | . | . | . | . | . | . | . | . | . | . | . | . | . |
| 21-225_7H11_LC | . | . | . | . | . | . | . | . | . | . | . | . | . | . | . | . | . | . | . |
| 21-225_7H7_LC | . | . | . | . | . | . | F | . | . | V | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | L | T | F | G | G | G | T | K | V | E | I | K | R |
| 21-225_8C9_LC | | | | | | | | | | | | | | | | | | | |
| 21-225_8D12_LC | | | | | | | | | | | | | | | | | | | |
| 21-225_8F11_LC | | | | | | | | | | | | | | | | | | | |
| 21-225_8H7_LC | | | | | | | | | | | | | | | | | | | |
| 21-225_96A3_LC | | | | | | | | | | | | | | | | | | | |
| 21-225_9E8_LC | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | S | . | . | . | . | . | . | . | . | . | . | . | . | . | . | . | . | . | . | . | . |
| 21-225_18C6_LC | . | . | . | . | . | S | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | O | . | . | . | . | . | . | . | . | . | . | . | . | . | . | . | . | . | . | . |
| 21-225_193A5_LC | . | . | . | . | . | . | O | . | . | . | . | . | . | . | . | . | . | . | . | . | . | . | . | . | . | . |
| 21-225_193E7_LC | . | . | . | . | . | K | R | . | . | . | . | . | . | . | . | . | . | . | . | . | . | . | . | . | . | . |
| 21-225_196C10_LC | . | . | . | . | Y | . | . | . | . | . | . | . | . | . | . | . | . | . | . | . | . | . | . | . | . | . |
| 21-225_199E3_LC | . | . | . | . | . | K | P | . | . | . | . | . | . | . | . | . | . | . | . | . | . | . | . | . | . | . |
| 21-225_19D11_LC | . | . | . | . | Y | Y | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | A | . | . | . | . | . | . | . | . | . | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | . | . |
| 21-225_200F12_LC | . | . | . | . | . | K | . | . | . | . | . | . | . | . | . | . | . | . | . | . | . | . | . | . | . | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | K | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | . | . | . |
| 21-225_204H8_LC | . | . | . | . | . | . | . | . | . | . | . | . | . | . | . | . | . | . | . | . | . | . | . | . | . | . |
| 21-225_205H8_LC | . | . | . | . | . | . | . | . | . | . | . | . | . | . | . | . | . | . | . | . | . | . | . | . | . | . |
| 21-225_20A10_LC | . | . | . | . | . | . | P | . | . | . | . | . | . | . | . | . | . | . | . | . | . | . | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | . | A | . | . | . | . | . | . | . | . | . | . | . | . | . | . | . | . | . | . | . | . |
| 21-225_20C8_LC | . | . | . | . | O | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | . | . |

FIGURE 57

Table 96.      Consensus 16 - VL3|3r/JL2 (SEQ ID NO: 50313):

SYELTQP-PSVSVSPGQTASITCSGD---KLGD-----KYACWYQQKPGQSPVLVIYQ-------DRKRPSGIPERFSGSNSG--
NTATLTISGTQAMDEADYYCQAWDS---------------------STVVFGGGTKLTVLG wherein:

D at position 26 can be substituted with N, E, Y or S

K at position 30 can be substituted with R, N, E or T

L at position 31 can be substituted with M or S

D at position 33 can be substituted with N, E, G, Y, T, H or V

K at position 39 can be substituted with R

Y at position 40 can be substituted with F or S

A at position 41 can be substituted with V, T, D or S

C at position 42 can be substituted with S, Y, W or H

Q at position 58 can be substituted with E or K

D at position 67 can be substituted with N

R at position 68 can be substituted with S, N, K, Y, M, T, G or I

K at position 69 can be substituted with R or Q

P at position 71 can be substituted with S

S at position 72 can be substituted with L

Q at position 107 can be substituted with L or K

A at position 108 can be substituted with T

FIGURE 57

W at position 109 can be substituted with R

D at position 110 can be substituted with H, V, N or G

S at position 111 can be substituted with N, null (-), I, R, K or T

null (-) at position 134 can be substituted with S, N or R

S at position 135 can be substituted with T, N, R, G, F, I V or Y

T at position 136 can be substituted with S, Y, A, F, P, N, K, I or R

V at position 137 can be substituted with A, T, M L or G

V at position 138 can be substituted with I, L, A or M

Ser Gly Xaa Xaa Xaa Gly Xaa Xaa Xaa Xaa Xaa (SEQ ID NO: 50125)

Xaa Xaa Xaa Xaa Arg Xaa Xaa (SEQ ID NO: 50126)

Xaa Xaa Xaa Xaa Xaa Xaa Xaa Xaa Xaa Xaa (SEQ ID NO: 50228)

Ser Gly Asp Lys Leu Gly Asp Lys Tyr Ala Cys (SEQ ID NO: 50609)

Gln Asp Arg Lys Arg Pro Ser (SEQ ID NO: 50610)

Gln Ala Trp Asp Ser Ser Thr Val Val (SEQ ID NO: 50611)

FIGURE 57

**FIGURE 57**

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | | | | | | | | | | | | L_FR1 | | | | | | | | | | | | | | | | |
| CONSENSUS | S | Y | E | L | T | Q | P | - | P | S | V | S | V | S | P | G | Q | T | A | S | I | T | C | S | G | D | - |
| 21-225_166G11_LC | . | . | . | . | S | . | . | . | . | . | . | . | . | . | . | . | . | . | . | . | . | . | . | . | . | . | . |
| 21-225_169A6_LC | . | . | . | . | . | . | . | . | . | . | . | . | . | . | . | . | . | . | . | . | . | . | . | . | . | . | . |
| 21-225_169B7_LC | A | . | D | . | . | . | . | . | . | . | . | . | . | . | . | . | . | . | . | R | . | . | . | . | . | . | . |
| 21-225_169C1_LC | . | . | . | . | . | . | . | . | . | . | . | . | . | . | . | . | . | . | . | . | . | . | . | . | . | . | . |
| 21-225_169G11_LC | . | . | . | . | . | . | . | . | . | . | . | . | . | . | . | . | . | . | . | . | . | . | . | . | . | . | . |
| 21-225_16D2_LC | . | . | . | . | . | . | . | . | . | . | . | . | . | . | . | . | . | . | . | . | . | . | . | . | . | . | . |
| 21-225_16E4_LC | P | . | . | . | . | . | . | . | . | . | . | . | . | . | . | . | . | . | . | . | . | . | . | . | . | . | . |
| 21-225_16G8_LC | . | . | . | . | S | . | . | . | . | . | . | . | . | . | . | . | . | . | . | . | . | . | . | . | . | . | . |
| 21-225_170A5_LC | . | . | G | . | . | . | . | . | . | . | . | . | . | . | . | . | . | . | . | . | . | . | . | . | . | . | . |
| 21-225_170F1_LC | . | . | . | . | S | . | . | . | . | . | . | . | . | . | . | . | . | . | . | . | . | . | . | . | . | . | . |
| 21-225_171E12_LC | . | . | . | . | . | . | . | . | . | . | . | . | . | . | . | . | . | . | . | . | . | . | . | . | . | . | . |
| 21-225_171F5_LC | A | . | G | . | . | . | . | . | . | . | . | . | . | . | . | . | . | . | . | . | . | . | . | . | . | . | . |
| 21-225_173F8_LC | . | . | . | . | . | . | . | . | . | . | . | . | . | . | . | . | . | . | . | . | . | . | . | . | . | N | . |
| 21-225_175C8_LC | . | . | D | . | . | . | . | . | . | . | . | . | . | . | . | . | . | . | . | . | . | . | . | . | . | . | . |
| 21-225_179C7_LC | . | . | . | . | . | . | . | . | . | . | . | . | . | . | . | . | . | . | . | . | . | . | . | . | . | . | . |
| 21-225_17D3_LC | . | . | . | . | . | . | S | . | . | . | . | . | . | . | . | . | . | . | . | . | . | . | . | . | . | . | . |
| 21-225_17F5_LC | . | . | . | . | . | . | . | . | . | . | . | . | . | . | . | . | . | . | . | . | . | . | . | . | . | . | . |
| 21-225_180C5_LC | . | . | . | . | . | . | . | . | . | . | . | . | . | . | . | . | . | . | . | . | . | . | . | . | . | . | . |
| 21-225_180D4_LC | . | . | . | . | . | . | T | . | . | . | . | . | . | . | . | . | . | . | . | . | . | . | . | . | . | . | . |
| 21-225_180G5_LC | . | . | . | . | . | . | . | . | . | . | . | . | . | Y | . | . | . | . | . | . | . | . | . | . | . | . | . |
| 21-225_181H1_LC | . | . | . | . | . | . | T | . | . | . | . | . | . | . | . | . | . | . | . | . | . | . | . | . | . | . | . |
| 21-225_182D12_LC | . | . | . | . | . | . | . | . | . | . | . | . | . | . | . | . | . | . | . | . | . | . | . | . | . | . | . |
| 21-225_183F4_LC | . | . | . | . | . | . | . | . | . | . | . | . | . | . | . | . | . | . | . | . | . | . | . | . | . | . | . |
| 21-225_185D2_LC | . | . | . | . | . | . | T | . | . | . | . | . | . | . | . | . | . | . | . | . | . | . | . | . | . | . | . |
| 21-225_185G7_LC | . | . | . | . | . | . | . | . | . | . | . | . | . | . | . | . | . | . | . | . | . | . | . | . | . | . | . |
| 21-225_186H6_LC | . | . | . | . | . | . | . | . | . | . | . | . | . | . | . | . | . | . | . | . | . | . | . | . | . | . | . |
| 21-225_18E10_LC | . | . | . | . | . | . | . | . | . | . | . | . | . | . | . | . | . | . | . | . | . | . | . | . | . | . | . |
| 21-225_18G5_LC | . | . | . | . | . | . | . | . | . | . | . | . | . | . | . | . | . | . | . | . | . | . | . | . | . | R | . |
| 21-225_190G9_LC | . | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | | | | | | | | | | | L_FR1 | | | | | | | | | | | | | | | | |
| CONSENSUS | S | Y | E | L | T | Q | P | - | P | S | V | S | V | S | P | G | Q | T | A | S | I | T | C | S | G | D | - |
| 21-225_190H1_LC | . | . | . | . | . | . | . | . | . | . | . | . | . | . | . | . | . | . | . | . | . | . | . | . | . | . | . |
| 21-225_193H5_LC | . | . | . | . | . | . | . | . | S | . | . | . | . | . | . | . | . | . | . | . | . | . | . | . | . | . | . |
| 21-225_195E3_LC | . | . | . | . | . | . | . | . | . | . | . | . | . | . | . | . | . | . | . | . | . | . | . | . | . | . | . |
| 21-225_195E9_LC | . | . | . | . | . | . | . | . | . | . | . | . | . | . | . | . | . | . | . | . | . | . | . | . | . | . | . |
| 21-225_19F6_LC | . | . | . | . | . | . | . | . | . | . | . | . | . | . | . | . | . | V | . | . | . | . | . | . | . | . | . |
| 21-225_201G11_LC | . | . | . | . | . | . | . | . | . | . | . | . | . | . | . | . | . | . | . | . | . | . | . | . | . | . | . |
| 21-225_203G6_LC | . | . | . | . | . | . | . | . | . | . | . | . | . | . | . | . | . | . | . | . | . | . | . | . | . | . | . |
| 21-225_215B3_LC | . | . | . | . | . | . | . | . | . | . | . | . | . | . | . | . | . | . | . | . | . | . | . | . | . | . | . |
| 21-225_21D12_LC | . | . | . | . | . | . | . | . | . | . | . | . | . | . | . | . | . | . | . | . | . | . | . | . | . | . | . |
| 21-225_224H2_LC | . | . | . | . | . | . | . | . | S | . | . | . | . | . | . | . | . | . | . | . | . | . | . | . | . | . | . |
| 21-225_225E9_LC | . | . | D | . | . | . | . | . | . | . | . | . | . | . | . | . | . | . | . | . | . | . | . | . | . | . | . |
| 21-225_226A10_LC | . | . | . | . | . | . | . | . | . | . | . | . | . | . | . | . | . | . | . | . | . | . | . | . | . | . | . |
| 21-225_226B2_LC | . | . | . | . | . | . | . | . | . | . | . | . | . | . | . | . | . | . | . | . | . | . | . | . | . | . | . |
| 21-225_227E12_LC | . | . | . | . | . | . | . | . | . | . | . | . | . | . | . | . | . | . | . | . | . | . | . | . | . | . | . |
| 21-225_227E5_LC | . | . | . | . | S | . | . | . | . | . | . | . | . | . | . | . | . | . | . | . | . | . | . | . | . | . | . |
| 21-225_22E6_LC | . | . | . | . | . | . | . | . | . | . | . | . | . | . | . | . | . | . | . | . | . | . | . | . | . | . | . |
| 21-225_23D7_LC | . | . | . | . | . | . | . | . | . | . | . | . | . | . | . | . | . | . | . | . | . | . | . | . | . | E | . |
| 21-225_26C10_LC | . | . | . | V | . | . | . | . | . | M | . | . | . | . | . | . | . | . | . | . | . | . | . | . | . | . | . |
| 21-225_27D9_LC | . | . | . | . | . | . | . | . | . | M | . | . | . | . | . | . | . | . | . | . | . | . | . | . | . | Y | . |
| 21-225_27E7_LC | . | . | . | . | . | . | . | . | . | . | . | . | . | . | . | . | . | . | . | . | . | . | . | . | . | . | . |
| 21-225_27G6_LC | . | . | . | . | . | . | . | . | . | . | . | . | . | . | . | . | . | . | . | . | . | . | . | . | . | . | . |
| 21-225_27H2_LC | . | . | . | . | . | . | . | . | . | . | . | . | . | . | . | . | . | . | . | . | . | . | . | . | . | . | . |
| 21-225_28A11_LC | . | . | . | . | . | . | . | . | . | . | . | . | . | . | . | . | . | . | . | . | . | . | . | . | . | . | . |
| 21-225_28I_LC | . | . | . | . | . | . | . | . | . | . | . | . | . | . | . | . | . | V | . | . | . | . | . | . | . | . | . |
| 21-225_30D9_LC2_LC | . | . | . | . | . | . | . | . | . | . | . | . | . | . | . | . | . | . | . | . | . | . | . | . | . | . | . |
| 21-225_30H6_LC | . | . | . | . | . | . | . | . | . | . | . | . | . | . | . | . | . | . | . | . | . | . | . | . | . | . | . |
| 21-225_31C2_LC | . | . | . | . | . | . | . | . | . | . | . | . | . | . | . | . | . | . | . | . | . | . | . | . | . | . | . |
| 21-225_31D12_LC2_LC | . | . | . | . | . | . | . | . | . | . | . | . | . | . | . | . | . | . | . | . | . | . | . | . | . | . | . |
| 21-225_32B11_LC | . | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | | | | | | | | | | | | L_FR1 | | | | | | | | | | | | | | | |
| CONSENSUS | S | Y | E | L | T | Q | P | - | P | S | V | S | V | S | P | G | Q | T | A | S | I | T | C | S | G | D | - |
| 21-226_33A1_LC | . | . | . | . | . | . | . | . | . | . | . | . | . | . | . | . | . | . | . | . | . | . | . | . | . | . | . |
| 21-225_33E6_LC | . | . | . | . | . | . | . | . | . | . | . | . | . | . | . | . | . | . | . | . | . | . | . | . | . | . | . |
| 21-225_34B11_LC | . | . | . | . | . | . | . | . | . | . | . | . | . | . | . | . | . | . | . | . | . | . | . | . | . | . | . |
| 21-225_34B9_LC | . | . | . | . | . | A | . | . | . | . | . | . | . | . | . | . | . | . | . | . | . | . | . | . | . | . | . |
| 21-225_34D7_LC | . | . | . | . | . | . | . | . | . | . | . | . | . | . | . | . | . | . | . | . | . | . | . | . | . | . | . |
| 21-225_35E1_LC | . | . | . | . | . | . | . | . | . | . | . | . | . | . | . | . | . | . | . | . | . | . | . | . | . | . | . |
| 21-225_3B12_LC | . | . | S | . | . | . | . | . | . | . | . | . | . | . | . | . | . | . | . | . | . | . | . | . | . | . | . |
| 21-226_3H10_LC | . | . | S | . | . | . | . | . | . | . | . | . | . | . | . | . | . | . | . | . | . | . | . | . | . | . | . |
| 21-225_45F6_LC | . | . | . | . | . | . | . | . | . | . | . | . | . | . | . | . | . | . | . | . | . | . | . | . | . | . | . |
| 21-225_4B3_LC | . | . | . | . | . | . | . | . | . | . | . | . | . | . | R | . | V | . | . | . | . | . | . | . | . | . | . |
| 21-225_50G9_LC | . | . | . | . | . | . | . | . | . | . | . | . | . | . | . | . | . | . | . | . | . | . | . | . | . | . | . |
| 21-225_52H1_LC | . | . | . | . | . | . | . | . | . | P | . | . | . | . | . | . | . | . | . | . | . | S | . | . | . | . | . |
| 21-225_53D10_LC | . | . | . | . | . | . | . | . | . | . | . | . | . | . | . | . | . | . | . | . | . | . | . | . | . | . | . |
| 21-225_57D9_LC | . | . | . | . | . | . | . | . | . | . | . | . | . | . | . | . | . | . | . | . | . | . | . | . | . | N | . |
| 21-225_57F1_LC | . | . | . | . | . | . | . | . | A | . | . | . | . | . | . | . | . | . | . | . | . | . | . | . | . | . | . |
| 21-225_57H11_LC | . | A | . | . | . | . | . | . | A | . | . | . | . | . | . | . | . | . | . | . | . | . | . | . | . | . | . |
| 21-225_58C1_LC | . | . | . | . | . | . | . | . | . | . | . | . | . | . | . | . | . | . | N | . | . | . | . | . | . | . | . |
| 21-225_58E7_LC | . | . | . | . | . | S | . | . | . | . | . | . | . | . | . | . | . | . | . | . | . | . | . | . | . | . | . |
| 21-225_58F7_LC | . | . | . | . | . | . | . | . | . | . | . | . | . | . | . | . | . | . | . | . | . | . | . | . | . | . | . |
| 21-225_58F8_LC | . | . | . | . | . | . | . | . | . | . | . | . | . | . | . | . | . | . | . | . | . | . | . | . | . | . | . |
| 21-225_58G11_LC | . | . | . | . | . | . | . | . | . | . | . | . | . | . | . | . | . | . | . | . | . | . | . | . | . | . | . |
| 21-225_59B11_LC | . | . | . | . | . | . | . | . | . | . | . | . | . | . | . | . | . | . | . | . | . | . | . | . | . | . | . |
| 21-225_59F2_LC | . | . | . | . | . | . | . | . | . | . | . | . | . | . | . | . | . | . | . | . | . | . | . | . | . | . | . |
| 21-225_5G7_LC | . | . | . | . | . | . | . | . | . | . | . | . | . | . | . | . | . | . | . | . | . | . | . | . | . | . | . |
| 21-226_60A12_LC | . | . | . | . | . | . | . | . | . | . | . | . | . | . | . | . | . | . | N | . | . | . | . | . | . | . | . |
| 21-225_60B1_LC | . | . | . | . | . | . | . | . | A | . | . | . | . | . | . | . | . | . | . | . | . | . | . | . | . | . | . |
| 21-225_60D2_LC | . | . | . | . | . | . | . | . | . | . | . | . | . | . | . | . | . | . | . | . | . | . | . | . | . | N | . |
| 21-226_61F5_LC | . | . | . | . | . | . | . | . | . | . | . | . | . | . | . | . | . | . | . | . | . | . | . | . | . | . | . |
| 21-225_62A12_LC | . | D | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | | | | | | | | | | | | L FR1 | | | | | | | | | | | | | | | |
| CONSENSUS | S | Y | E | L | T | Q | P | - | P | S | V | S | V | S | P | G | Q | T | A | S | I | T | C | S | G | D | - |
| 21-225_62B12_LC | | | D | | | | | | | | | | | | | | | | | | | | | | | | |
| 21-225_62D10_LC | | | D | | | | | | | | | | | | | | | | | | | | | | | | |
| 21-225_62E3_LC | | | D | | | | | | | | | | | | | | | | | | | | | | | | |
| 21-225_62E6_LC | | | D | | | | | | | | | | | | | | | | | | | | | | | | |
| 21-225_65E9_LC | | | | | | | | | | | | | | | | | | | | | | | | | | | |
| 21-225_66G9_LC | | | D | | | | | | | | | Y | | | | | | | | | | | | | | | |
| 21-225_67F10_LC | | | | | | | | | | | | | | | | | | | | | | | | | | | |
| 21-225_68D8_LC | | | | | | | | | | | | | | | | | | | | | | | | | | | |
| 21-225_68G6_LC | | | | V | | | | | | | | | | | | | | | | | | | | | | | |
| 21-225_69B11_LC | | | | | | | | | | | | | | | | | | A | | | | | | | | | |
| 21-225_69B9_LC | | | | | | | | | | | | | | | | | | | | | | | | | | | |
| 21-225_6B4_LC | | | | | | | | | | | | | | | | R | | | V | | | | | | | | |
| 21-225_6D9_LC | | | | | | | | | | | | | | | | | | | | | | | | | | | |
| 21-225_71D4_LC | | | | | | | | | | | | | | | | | | | | | | | | | | | |
| 21-225_72B4_LC | | | | | | | | | | | | | | | | | | | | | | | | | | | |
| 21-225_72D5_LC | | | D | | | | | | | | | | | | | | | | | | | | | | | | |
| 21-225_73C4_LC | | | D | | | | | | | | | | | | | | | | | | | | | | | | |
| 21-225_74A9_LC | | | | | | | | | | | | | | | | | | | | | | | | | | | |
| 21-225_74B3_LC | | | | | | | | | | | | | | | | | | | | | | | | | | | |
| 21-225_74C9_LC | | | | | | | | | | E | | | | | | | | | | | | | | | | | |
| 21-225_76B4_LC | P | | | | N | | T | | | | | | | | | | | | | | | | | | | | |
| 21-225_77A2_LC | | | | | | | | | | | | | | | | | | | | | | | | | | | |
| 21-225_78E9_LC | | | | | | | | | | E | | | | | | | | | | | | | | | | | |
| 21-225_79E7_LC | | | | | | | | | | | | | | | | | | | | | | | | | | | |
| 21-225_8D8_LC | | | | | | | | | | | | | | | | | | | | | | | | | | | |
| 21-225_92A4_LC | | | | | | | | | | | | | | | | | | | | N | | | | | | | |
| 21-225_96B5_LC | P | | | | N | | T | | | | | | | | | | | | | | | | | | | | |
| 21-225_97E6_LC | | | | | | | | | | | | | | | | | | | V | | | | | | | | |
| 21-225_9H10_LC | | | | | | | | | | | | | | | | | | | | | | | | | | | |

FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | L CDR1 | | | | | | | | | | | | | | | | | | L FR2 | | | | |
| CONSENSUS | - | - | K | L | G | D | - | - | - | - | K | Y | A | C | W | Y | Q | Q | K | P | G | Q | S | P | V | L |
| 21-225_10B10_LC | . | . | . | . | N | . | . | . | . | . | . | . | . | W | . | . | . | . | . | . | . | . | . | . | . | . |
| 21-225_12B1_LC | . | . | . | . | . | . | . | . | . | . | . | . | . | W | . | . | . | . | . | . | . | . | . | . | . | . |
| 21-225_13F6_LC | . | . | . | . | . | . | . | . | . | . | . | . | . | . | . | . | . | . | . | . | . | . | . | . | . | . |
| 21-225_146A10_LC | . | . | . | . | . | . | . | . | . | . | . | V | . | . | . | . | . | . | . | . | . | . | . | . | . | . |
| 21-225_146A2_LC | . | . | . | . | . | . | . | . | . | . | . | . | . | S | . | . | . | . | . | . | . | . | . | . | . | . |
| 21-225_146A3_LC | . | . | . | . | N | . | . | . | . | . | . | . | . | . | . | . | . | . | . | . | . | . | . | . | . | . |
| 21-225_146B11_LC | . | . | . | . | . | . | . | . | . | . | . | . | . | S | . | . | . | . | . | . | . | . | . | . | . | . |
| 21-225_146B8_LC | . | . | . | . | . | . | . | . | . | . | . | V | . | . | . | . | . | . | . | . | . | . | . | . | . | . |
| 21-225_146C11_LC | . | . | . | . | . | . | . | . | . | . | . | . | . | S | . | . | . | . | . | . | . | . | . | . | . | . |
| 21-225_146H8_LC | . | . | . | . | . | . | . | . | . | . | . | . | . | . | . | . | . | . | . | . | . | . | . | . | . | . |
| 21-225_147B12_LC | . | . | . | . | . | . | . | . | . | . | . | . | . | S | . | . | . | . | . | . | . | . | . | . | . | . |
| 21-225_147B8_LC | . | . | . | . | . | . | . | . | . | . | . | V | . | . | . | . | . | . | . | . | . | . | . | . | . | . |
| 21-225_147B9_LC | . | . | . | . | . | . | . | . | . | . | . | V | . | S | . | . | . | . | . | . | . | . | . | . | . | . |
| 21-225_147D9_LC | . | . | . | . | N | . | . | . | . | . | . | V | . | . | . | . | . | . | . | . | . | . | . | . | . | . |
| 21-225_147E11_LC | . | . | . | . | . | . | . | . | . | . | . | . | . | S | . | . | . | . | . | . | . | . | . | . | . | . |
| 21-225_147E7_LC | . | . | . | . | . | . | . | . | . | . | . | . | . | S | . | . | . | . | . | . | . | . | . | . | . | . |
| 21-225_147F9_LC | . | . | . | . | N | . | . | . | . | . | . | . | . | . | . | . | . | . | . | . | . | . | . | . | . | . |
| 21-225_147G9_LC | . | . | . | . | . | . | . | . | . | . | . | . | . | . | . | . | . | . | . | . | . | . | . | . | . | . |
| 21-225_148A9_LC | . | . | . | . | N | . | . | . | . | . | . | V | . | . | . | . | . | . | . | . | . | . | . | . | . | . |
| 21-225_148C8_LC | . | . | . | . | . | . | . | . | . | . | . | V | . | S | . | . | . | . | . | . | . | . | . | . | . | . |
| 21-225_148E10_LC | . | . | . | . | N | . | . | . | . | . | . | V | . | . | . | . | . | . | . | . | . | . | . | . | . | I |
| 21-225_148G11_LC | . | . | . | . | . | . | . | . | . | . | . | . | . | S | . | . | . | . | . | . | . | . | A | A | . | . |
| 21-225_148G6_LC | . | . | . | . | . | . | . | . | . | . | . | . | . | S | . | . | . | . | . | . | . | . | . | . | . | . |
| 21-225_149A11_LC | . | . | . | . | N | . | . | . | . | . | . | V | . | S | . | . | . | . | . | . | . | . | . | . | . | . |
| 21-225_149B5_LC | . | . | . | . | Y | . | . | . | . | . | . | V | . | . | . | . | . | . | . | . | . | . | . | . | . | . |
| 21-225_149C10_LC | . | . | . | . | . | . | . | . | . | . | . | . | . | S | . | . | . | . | . | . | . | . | . | . | . | . |
| 21-225_149C7_LC | . | . | . | . | . | . | . | . | . | . | . | . | . | S | . | . | . | . | . | . | . | . | . | . | . | . |
| 21-225_14B1_LC2_LC | . | . | . | . | . | . | . | . | . | . | . | . | . | Y | . | . | . | . | . | . | . | . | . | . | . | . |
| 21-225_14B7_LC | . | . | . | . | N | . | . | . | . | . | . | . | . | Y | . | . | . | . | . | . | . | . | . | . | . | . |

**FIGURE 57**

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | L_CDR1 | | | | | | | | | | | | | | L_FR2 | | | | | | | | |
| CONSENSUS | . | . | K | L | G | D | . | . | . | . | . | K | Y | A | C | W | Y | Q | Q | K | P | G | Q | S | P | V | L |
| 21-225_14G3_LC | . | . | . | . | . | . | . | . | . | . | . | . | . | V | . | . | . | . | . | . | . | . | . | . | . | . | . |
| 21-225_14H11_LC | . | . | . | . | . | E | . | . | . | . | . | . | . | V | . | . | . | . | . | . | . | . | . | . | . | . | . |
| 21-225_150D3_LC | . | E | . | . | N | . | . | . | . | . | . | . | . | . | . | . | . | . | . | . | . | . | . | . | . | . | . |
| 21-225_150F8_LC | . | . | . | . | . | . | . | . | . | . | . | . | . | . | S | . | . | . | . | . | . | . | . | . | . | . | . |
| 21-225_150H11_LC | . | . | . | . | . | . | . | . | . | . | . | . | . | . | S | . | . | . | . | . | . | . | . | . | . | . | . |
| 21-225_151B9_LC | . | N | . | . | . | . | . | . | . | . | . | . | . | . | S | . | . | . | . | . | . | . | . | . | . | . | . |
| 21-225_151F3_LC | . | . | . | . | . | . | . | . | . | . | . | . | . | V | . | . | . | . | . | . | . | . | . | . | . | . | . |
| 21-225_151H5_LC | . | . | . | . | . | . | . | . | . | . | . | . | . | . | S | . | . | . | . | . | . | . | . | . | . | . | . |
| 21-225_151H7_LC | . | . | . | . | . | . | . | . | . | . | . | . | . | . | S | . | . | . | . | . | . | . | . | . | . | . | . |
| 21-225_152D7_LC | . | . | . | . | . | . | . | . | . | . | . | . | . | . | S | . | . | . | . | . | . | . | . | . | . | . | . |
| 21-225_152G6_LC | . | . | . | . | . | . | . | . | . | . | . | . | . | . | S | . | . | . | . | . | . | . | . | . | . | . | . |
| 21-225_153D8_LC | . | . | . | . | . | . | . | . | . | . | . | . | . | . | . | . | . | . | . | . | . | . | . | . | . | . | . |
| 21-225_153E8_LC | . | . | . | . | . | N | . | . | . | . | . | . | . | V | . | . | . | . | . | S | . | . | . | . | . | . | . |
| 21-225_154C3_LC | . | . | . | . | . | . | . | . | . | . | . | . | . | V | . | . | . | . | . | . | . | . | . | . | . | . | . |
| 21-225_154C4_LC | . | . | . | . | . | . | . | . | . | . | . | . | . | . | S | . | . | . | . | . | . | . | . | . | . | . | . |
| 21-225_154D11_LC | . | . | . | . | . | . | . | . | . | . | . | . | . | . | . | . | . | . | . | . | . | . | . | . | . | . | . |
| 21-225_154E10_LC | . | . | . | . | . | . | . | . | . | . | . | . | . | . | S | . | . | . | . | . | . | . | . | . | . | . | . |
| 21-225_154F4_LC | . | . | . | . | . | N | . | . | . | . | . | . | . | V | . | . | . | . | . | . | . | . | . | . | . | . | N |
| 21-225_155C10_LC | . | . | . | . | . | . | . | . | . | . | . | . | . | V | S | . | . | . | . | . | . | . | . | . | . | . | . |
| 21-225_155E10_LC | . | . | . | . | . | . | . | . | . | . | . | . | . | V | S | . | . | . | . | . | . | . | . | . | . | . | . |
| 21-225_158D10_LC | . | . | . | . | . | . | . | . | . | . | . | . | . | V | . | . | . | . | . | . | . | . | . | . | . | . | . |
| 21-225_158E8_LC | . | . | . | . | . | . | . | . | . | . | . | . | . | V | . | . | . | . | . | . | . | . | . | . | . | . | . |
| 21-225_159H8_LC | . | . | . | . | . | . | . | . | . | . | . | . | . | V | . | . | . | . | . | . | . | . | . | . | . | . | . |
| 21-225_15H11_LC | . | . | . | . | . | E | . | . | . | . | . | . | . | . | . | . | . | . | . | . | . | . | . | . | . | . | V |
| 21-225_160B12_LC | . | . | . | . | . | . | . | . | . | . | . | . | . | V | . | . | . | . | . | . | . | . | . | . | . | . | . |
| 21-225_161E10_LC | . | . | . | . | . | . | . | . | . | . | . | . | . | V | . | . | . | . | . | . | . | . | . | . | . | . | . |
| 21-225_161F12_LC | . | . | . | . | . | . | . | . | . | . | . | . | . | . | . | . | . | . | . | . | . | . | . | . | . | . | . |
| 21-225_161H3_LC | . | N | . | . | . | . | . | . | . | . | . | . | . | V | . | . | . | . | . | . | . | . | . | . | . | . | . |
| 21-225_185H3_LC | . | . | . | . | . | . | . | . | . | . | . | . | . | . | . | . | . | . | . | . | . | . | . | . | . | . | . |

**FIGURE 57**

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | L_CDR1 | | | | | | | | | | | | | | | | L_FR2 | | | | | | |
| CONSENSUS | . | . | K | L | G | D | . | . | . | . | . | K | Y | A | C | W | Y | Q | Q | K | P | G | Q | S | P | V | L |
| 21-225_166G11_LC | | | | | | | | | | | | | | V | R | | | | | | | | | | | | |
| 21-225_169A6_LC | | | | | | | | | | | | | | V | | | | | R | | | | | | | | |
| 21-225_169B7_LC | | | | | G | | | | | | | | | | S | | | | | | | | | | | | |
| 21-225_169C1_LC | | | | | | | | | | | | | | V | | | | | | | | | | | | L | |
| 21-225_169G11_LC | | | | | | | | | | | | | | | | | | | | | | | | | | | |
| 21-225_16D2_LC | | | | | | | | | | | | | | | | | | | | | | | | | | | |
| 21-225_16E4_LC | | | | | | | | | | | | | | V | Y | | | | | | S | | | | | | |
| 21-225_16G8_LC | | | | | E | | | | | | | | | | | | | | | | | | | | | | |
| 21-225_170A5_LC | | | | | | | | | | | | | | | | | | | | | | | | | | I | |
| 21-225_170F1_LC | | | | | | | | | | | | | | | S | | | | | | | | | | | | |
| 21-225_171E12_LC | | | | | | | | | | | | | | | | | | | | | | | | | | | |
| 21-225_171F5_LC | | | | | G | | | | | | | | | | S | | | | | | | | | | | | |
| 21-225_173F8_LC | | | | | N | | | | | | | | | V | | | | | | R | | | | | | | |
| 21-225_175C6_LC | | | | | | | | | | | | | | | | | | | | | | | | | | I | |
| 21-225_179C7_LC | | | | | | | | | | | | | | V | | | | | R | | | | | | | | |
| 21-225_17D3_LC | | | | | | | | | | | | | | V | Y | | | | | | | | | | | | V |
| 21-225_17F5_LC | | | | | E | | | | | | | | | | | | | | | | | | | | | | |
| 21-225_180C5_LC | | | | | N | | | | | | | | | | | | | | | | | | | | | | |
| 21-225_180D4_LC | | R | | | | | | | | | | | | | | | | | | | | | | | | | |
| 21-225_180G5_LC | | | | | | | | | | | | | | V | S | | | | | | | | | | | | |
| 21-225_181H1_LC | | | | | | | | | | | | | | | | | | | | | | | | | | | |
| 21-225_182D12_LC | | | | | E | | | | | | | | | | | | | | | | S | | | | | | |
| 21-225_183F4_LC | | | | | | | | | | | | | | | | | | | | | | | | | | | |
| 21-225_185D2_LC | | | | | | | | | | | | | | | | | | | | | | | | | | | |
| 21-225_186G7_LC | | | | | E | | | | | | | | F | V | | | | | | | | | | | | | |
| 21-225_186H6_LC | | | M | | E | | | | | | | | | | | | | | | | | | | | | | |
| 21-225_18E10_LC | | R | | | | | | | | | | | | | W | | | | | | | | | | | | |
| 21-225_18G5_LC | | | | | | | | | | | | | | V | | | | | | | | | | | | | |
| 21-225_190G9_LC | | | | | | | | | | | | R | F | | Y | | | | | | | | | | | | |

FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | | L_CDR1 | | | | | | | | | | | | | L_FR2 | | | | | | | | |
| CONSENSUS | - | - | K | L | G | D | - | - | - | - | - | K | Y | A | C | W | Y | Q | Q | K | P | G | Q | S | P | V | L |
| 21-225_190H1_LC | . | . | . | . | . | . | . | . | . | . | . | R | F | . | Y | . | . | . | . | . | . | . | . | . | . | . | . |
| 21-225_193H5_LC | . | . | . | . | . | . | . | . | . | . | . | R | F | . | . | . | . | . | . | . | . | . | . | . | . | . | . |
| 21-225_196E3_LC | . | . | . | . | . | Y | . | . | . | . | . | R | S | V | . | . | . | . | . | . | . | . | . | . | . | . | . |
| 21-225_195E9_LC | . | . | . | . | . | N | . | . | . | . | . | . | . | . | Y | . | . | . | . | . | . | . | . | . | . | . | . |
| 21-225_19F6_LC | . | . | . | . | . | . | . | . | . | . | . | . | . | . | . | . | . | . | . | . | . | . | . | . | . | . | . |
| 21-225_201G11_LC | . | . | . | . | . | . | . | . | . | . | . | R | F | . | . | . | . | . | . | . | . | . | . | . | . | . | . |
| 21-225_203G6_LC | . | . | . | . | . | . | . | . | . | . | . | R | F | . | . | . | . | . | . | . | . | . | . | . | . | . | . |
| 21-225_215B3_LC | . | . | . | . | . | . | . | . | . | . | . | R | . | . | . | . | . | . | . | . | . | . | . | . | . | . | . |
| 21-225_21D12_LC | . | . | . | . | . | N | . | . | . | . | . | . | . | . | Y | . | . | . | . | . | . | . | . | . | . | . | . |
| 21-225_224H2_LC | . | . | N | . | . | V | . | . | . | . | . | . | . | T | Y | . | . | . | . | . | . | . | . | . | . | . | . |
| 21-225_225E9_LC | . | . | N | . | . | N | . | . | . | . | . | R | . | . | . | . | . | . | . | . | . | . | . | . | . | . | . |
| 21-225_226A10_LC | . | . | T | . | . | G | . | . | . | . | . | . | . | . | . | W | . | . | . | . | . | . | . | . | . | . | . |
| 21-225_226B2_LC | . | . | T | . | . | G | . | . | . | . | . | . | . | . | . | W | . | . | . | R | . | . | . | . | . | . | . |
| 21-225_227E12_LC | . | . | . | . | . | . | . | . | . | . | . | . | . | V | . | . | . | . | . | . | . | . | . | . | . | . | . |
| 21-225_227E5_LC | . | . | . | . | . | E | . | . | . | . | . | . | . | . | . | . | . | . | . | . | . | . | . | . | . | . | . |
| 21-225_22E6_LC | . | . | . | . | . | E | . | . | . | . | . | . | . | . | . | . | . | . | . | . | . | . | . | . | . | . | . |
| 21-225_23D7_LC | . | . | . | . | . | . | . | . | . | . | . | . | . | V | . | . | . | . | . | . | . | . | . | . | . | . | V |
| 21-225_26C10_LC | . | . | . | . | . | . | . | . | . | . | . | . | . | V | . | . | F | . | . | . | . | . | . | . | . | . | . |
| 21-225_27D9_LC | . | . | . | . | . | . | . | . | . | . | . | . | . | . | . | . | F | . | . | . | . | . | . | . | . | . | . |
| 21-225_27E7_LC | . | . | . | . | . | N | . | . | . | . | . | . | . | . | . | . | . | . | . | . | . | . | . | . | . | . | E |
| 21-225_27G6_LC | . | . | . | . | . | . | . | . | . | . | . | . | . | . | . | . | . | . | . | . | . | . | . | . | . | . | . |
| 21-225_27H2_LC | . | . | . | . | . | . | . | . | . | . | . | . | . | S | . | . | . | . | . | . | . | . | . | . | . | . | . |
| 21-225_28A11_LC | . | . | . | . | . | . | . | . | . | . | . | . | . | . | . | . | . | . | . | . | . | . | . | . | . | . | . |
| 21-225_2B1_LC | . | . | . | . | . | . | . | . | . | . | . | . | . | . | . | . | . | . | . | . | . | . | . | . | . | . | . |
| 21-225_30D9_LC2_LC | . | . | . | . | . | . | . | . | . | . | . | . | . | V | Y | . | F | . | . | . | . | . | . | . | . | . | . |
| 21-225_30H6_LC | . | . | . | . | . | N | . | . | . | . | . | . | . | . | . | . | . | . | . | . | . | . | . | . | . | . | . |
| 21-225_31C2_LC | . | . | . | . | . | N | . | . | . | . | . | . | . | . | . | . | . | . | . | . | . | . | . | . | . | . | . |
| 21-225_31D12_LC2_LC | . | . | . | . | . | . | . | . | . | . | . | . | . | . | Y | . | F | . | . | . | . | . | . | . | . | . | ? |
| 21-225_32B11_LC | . | . | . | . | . | . | . | . | . | . | . | . | . | . | Y | . | . | . | . | . | . | . | . | . | . | . | . |

4363

FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 |
|---|---|---|---|---|---|---|---|---|---|---|---|---|---|---|---|---|---|---|---|---|---|---|---|---|---|---|
| | | | | | L CDR1 | | | | | | | | | | | | | | | | | | L FR2 | | | |
| CONSENSUS | - | - | K | L | G | D | - | - | - | - | K | Y | A | C | W | Y | Q | Q | K | P | G | Q | S | P | V | L |
| 21-225_33A1_LC | . | . | . | . | . | . | . | . | . | . | . | F | V | Y | . | . | . | . | . | . | . | . | . | . | . | . |
| 21-225_33E6_LC | . | . | . | . | . | . | . | . | . | . | . | . | Y | . | F | . | . | . | . | . | . | . | . | . | . | I |
| 21-225_34B11_LC | . | . | . | . | . | . | . | . | . | . | . | . | . | . | . | . | . | . | . | . | . | . | . | . | . | V |
| 21-225_34B9_LC | . | . | . | . | E | . | . | . | . | . | . | V | S | . | . | . | E | . | . | . | . | . | . | . | . | . |
| 21-225_34D7_LC | . | . | . | . | E | . | . | . | . | . | . | . | Y | . | . | . | . | . | . | . | . | . | . | . | . | . |
| 21-225_35E1_LC | . | . | . | . | E | . | . | . | . | . | . | . | Y | . | F | . | . | . | . | . | . | . | . | . | . | I |
| 21-225_3B12_LC | . | . | . | . | E | . | . | . | . | . | . | . | . | . | . | . | . | . | . | . | . | . | . | . | . | . |
| 21-225_3H10_LC | . | . | . | . | E | . | . | . | . | . | . | . | . | . | . | . | . | . | . | . | . | . | . | . | . | . |
| 21-225_45F6_LC | . | . | . | . | . | . | . | . | . | . | . | . | S | . | . | . | . | . | . | . | . | . | . | . | . | . |
| 21-225_4B3_LC | . | . | . | . | . | . | . | . | . | . | . | . | . | . | . | . | . | . | . | . | . | . | . | . | . | . |
| 21-225_50G9_LC | . | . | . | . | . | . | . | . | . | . | . | . | . | . | . | . | . | . | . | . | . | . | . | . | . | . |
| 21-225_52H1_LC | . | . | . | . | . | . | . | . | . | . | . | V | S | . | . | . | . | . | . | . | . | . | . | . | . | . |
| 21-225_53D10_LC | . | . | . | . | N | . | . | . | . | . | . | . | . | . | . | . | . | . | R | . | . | . | . | . | . | . |
| 21-225_57D9_LC | . | . | . | . | E | . | . | . | . | F | . | . | . | S | . | . | . | . | . | . | . | . | . | . | . | . |
| 21-225_57F1_LC | . | . | . | . | E | . | . | . | . | . | . | . | . | . | . | . | . | . | . | . | . | . | . | . | . | . |
| 21-225_57H11_LC | . | . | . | . | E | . | . | . | . | . | . | . | . | . | . | . | . | . | . | . | . | . | . | . | . | . |
| 21-225_58C1_LC | . | . | . | . | N | . | . | . | . | . | . | . | . | . | . | . | . | . | . | . | . | . | . | . | . | . |
| 21-225_58E7_LC | . | . | . | . | . | . | . | . | . | . | T | . | . | . | . | R | . | . | . | . | . | . | . | . | . | . |
| 21-225_58F7_LC | . | . | . | . | . | . | . | . | . | . | . | . | . | . | . | . | . | . | . | . | . | . | . | . | . | . |
| 21-225_58F8_LC | . | . | . | . | N | . | . | . | . | . | . | . | . | . | . | . | . | . | . | . | . | . | . | . | . | . |
| 21-225_58G11_LC | . | . | . | . | . | . | . | . | . | . | . | . | S | . | . | . | . | . | . | . | . | . | . | . | . | . |
| 21-225_59B11_LC | . | . | . | . | . | . | . | . | . | . | . | . | . | . | . | . | . | . | . | . | . | . | . | . | . | . |
| 21-225_59F2_LC | . | . | . | . | . | . | . | . | . | . | . | . | S | . | . | . | . | R | . | . | . | . | . | . | . | . |
| 21-225_5G7_LC | . | . | . | . | N | . | . | . | . | . | . | . | W | . | . | . | . | . | . | . | . | . | . | . | . | . |
| 21-225_60A12_LC | . | . | . | . | N | . | . | . | . | . | . | . | . | . | . | . | . | . | . | . | . | . | . | . | . | . |
| 21-225_60B1_LC | . | . | . | . | E | . | . | . | . | . | . | . | . | . | . | . | . | . | . | . | . | . | . | . | . | . |
| 21-225_60D2_LC | . | . | . | . | . | . | . | . | . | . | . | . | S | . | . | . | . | R | . | . | . | . | . | . | . | . |
| 21-225_61F5_LC | . | . | . | . | E | . | . | . | . | . | . | . | . | . | . | . | . | . | . | . | . | . | . | . | . | . |
| 21-225_62A12_LC | . | . | . | . | N | . | . | . | . | . | . | . | . | . | . | . | . | . | . | . | . | . | . | . | . | V |

FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | L_CDR1 | | | | | | | | | | | | | | | | | | L_FR2 | | | | |
| CONSENSUS | . | . | K | L | G | D | . | . | . | . | . | K | Y | A | C | W | Y | Q | Q | K | P | G | Q | S | P | V | L |
| 21-225_62B12_LC | . | . | . | . | . | N | . | . | . | . | . | . | . | T | . | . | . | . | . | . | . | . | . | . | . | . | V |
| 21-225_62D10_LC | . | . | . | . | . | N | . | . | . | . | . | . | . | . | . | . | . | . | . | . | . | . | . | . | . | . | V |
| 21-225_62E3_LC | . | . | . | . | . | N | . | . | . | . | . | . | . | . | . | . | . | . | . | . | . | . | . | . | . | . | V |
| 21-225_62E6_LC | . | R | . | . | . | N | . | . | . | . | . | . | . | . | S | . | . | . | . | . | . | . | . | . | . | . | V |
| 21-225_65E9_LC | . | . | . | . | . | N | . | . | . | . | . | . | . | O | Y | . | . | . | . | . | . | . | . | . | . | . | . |
| 21-225_66G9_LC | . | . | . | . | . | N | . | . | . | . | . | . | . | . | . | . | . | . | . | . | . | . | . | . | . | . | V |
| 21-225_67F10_LC | . | . | . | . | . | Y | . | . | . | . | . | . | . | . | W | . | . | . | . | . | . | . | . | . | . | . | . |
| 21-225_68D8_LC | . | . | . | . | . | . | . | . | . | . | . | . | . | . | . | . | . | . | . | . | . | . | . | . | . | . | . |
| 21-225_68G6_LC | . | . | . | . | . | . | . | . | . | . | . | . | . | T | Y | . | . | . | . | A | . | . | . | . | . | F | . |
| 21-225_69B11_LC | . | . | . | . | . | . | . | . | . | . | . | . | . | . | W | . | . | . | . | . | . | . | . | . | . | . | . |
| 21-225_69B9_LC | . | . | . | . | . | N | . | . | . | . | . | . | . | O | Y | . | . | . | . | . | . | . | . | . | . | . | . |
| 21-225_6B4_LC | . | . | . | . | . | . | . | . | . | . | . | . | . | . | . | . | . | . | . | . | . | . | . | . | . | . | . |
| 21-225_6D9_LC | . | . | . | . | . | S | . | . | . | . | . | . | . | . | . | . | . | . | . | . | . | . | . | . | . | . | . |
| 21-225_71D4_LC | . | . | . | . | . | . | . | . | . | . | . | . | . | . | Y | . | . | . | . | . | . | . | . | . | . | . | V |
| 21-225_72B4_LC | . | . | . | . | . | . | . | . | . | . | . | . | . | . | W | . | . | . | . | A | . | . | . | . | . | . | V |
| 21-225_72D5_LC | . | . | . | . | . | N | . | . | . | . | . | . | . | . | . | . | . | . | . | . | . | . | . | . | . | . | V |
| 21-225_73C4_LC | . | . | . | . | . | N | . | . | . | . | . | . | . | . | . | . | . | . | . | . | . | . | . | . | . | . | V |
| 21-225_74A9_LC | . | . | . | . | . | N | . | . | . | . | . | . | . | V | . | . | . | . | . | . | . | . | . | . | . | . | . |
| 21-225_74B3_LC | . | . | . | . | . | . | . | . | . | . | . | . | . | . | . | . | . | . | . | . | . | . | . | . | . | . | . |
| 21-225_74C9_LC | . | . | S | . | . | N | . | . | . | . | . | . | . | V | S | . | . | . | . | . | . | . | . | . | . | . | . |
| 21-225_76B4_LC | . | R | . | . | . | Y | . | . | . | . | F | . | . | . | . | . | . | . | . | . | . | . | . | . | . | . | . |
| 21-225_77A2_LC | . | R | . | . | . | . | . | . | . | . | . | . | . | . | . | . | . | . | . | . | . | . | . | . | . | . | . |
| 21-225_78E9_LC | . | . | . | . | . | N | . | . | . | . | . | . | . | V | S | . | . | . | . | . | . | . | . | . | . | . | . |
| 21-225_79E7_LC | . | . | . | . | . | N | . | . | . | . | . | . | . | V | S | . | . | . | . | . | . | . | . | . | . | . | . |
| 21-225_8D8_LC | . | . | . | . | . | E | . | . | . | . | . | . | . | . | . | . | . | . | . | . | . | . | . | . | . | . | V |
| 21-225_92A4_LC | . | . | . | . | . | N | . | . | . | . | . | . | . | V | . | . | . | . | . | . | . | . | . | . | . | . | . |
| 21-225_96B5_LC | . | R | . | . | . | Y | . | . | . | . | F | . | . | . | . | . | . | . | . | . | . | . | . | . | . | . | . |
| 21-225_97E6_LC | . | . | . | . | . | N | . | . | . | . | . | . | . | V | S | . | . | . | . | . | . | . | . | . | . | . | . |
| 21-225_9H10_LC | . | . | . | . | . | . | . | . | . | . | . | . | . | . | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | | | | | | | L_CDR2 | | | | | | | | | | | | | | | | | |
| CONSENSUS | V | I | Y | Q | . | . | . | . | . | . | . | . | D | R | K | R | P | S | G | I | P | E | R | F | S | G | S |
| 21-225_10B10_LC | . | . | . | . | . | . | . | . | . | . | . | . | . | . | . | . | . | . | . | . | . | . | . | . | . | . | . |
| 21-225_12B1_LC | . | . | . | . | . | . | . | . | . | . | . | . | . | . | . | . | . | . | . | . | . | . | . | . | . | . | . |
| 21-225_13F6_LC | . | . | . | . | . | . | . | . | . | . | . | . | S | . | . | . | . | . | . | . | . | . | . | . | . | . | . |
| 21-225_146A10_LC | . | F | . | . | . | . | . | . | . | . | . | . | . | . | . | . | . | . | . | . | . | . | . | . | . | . | . |
| 21-225_146A2_LC | . | . | . | . | . | . | . | . | . | . | . | . | S | . | . | . | . | . | . | . | . | . | . | . | . | . | . |
| 21-225_146A3_LC | . | . | . | . | . | . | . | . | . | . | . | . | S | . | . | . | . | . | . | . | . | . | . | . | . | . | . |
| 21-225_146B11_LC | . | . | . | . | . | . | . | . | . | . | . | . | S | . | . | . | . | . | . | . | . | . | . | . | . | . | . |
| 21-225_146B8_LC | . | . | . | . | . | . | . | . | . | . | . | . | . | . | . | . | . | . | . | . | . | . | . | . | . | . | . |
| 21-225_146C11_LC | . | . | . | . | . | . | . | . | . | . | . | . | S | . | . | . | . | . | . | . | . | . | . | . | . | . | . |
| 21-225_146H8_LC | . | . | . | . | . | . | . | . | . | . | . | . | H | . | . | . | . | . | . | . | . | . | . | . | . | . | . |
| 21-225_147B12_LC | . | . | . | . | . | . | . | . | . | . | . | . | S | . | . | . | . | . | . | . | . | . | . | . | . | . | . |
| 21-225_147B8_LC | . | . | . | . | . | . | . | . | . | . | . | . | . | . | . | . | . | . | . | . | . | . | . | . | . | . | . |
| 21-225_147B9_LC | . | . | . | . | . | . | . | . | . | . | . | . | . | . | . | . | . | . | . | . | . | . | . | . | . | . | . |
| 21-225_147D9_LC | . | . | . | . | . | . | . | . | . | . | . | . | S | . | . | . | . | . | . | . | . | . | . | . | . | . | . |
| 21-225_147E11_LC | . | . | . | . | . | . | . | . | . | . | . | . | S | . | . | . | . | . | . | . | . | . | . | . | . | . | . |
| 21-225_147E7_LC | . | . | . | . | . | . | . | . | . | . | . | . | S | . | . | . | . | . | . | . | . | . | . | . | . | . | . |
| 21-225_147F9_LC | . | . | . | . | . | . | . | . | . | . | . | . | S | . | . | . | . | . | . | . | . | . | . | . | . | . | . |
| 21-225_147G9_LC | . | . | . | . | . | . | . | . | . | . | . | . | . | . | . | . | . | . | . | . | . | . | . | . | . | . | . |
| 21-225_148A9_LC | . | . | . | . | . | . | . | . | . | . | . | . | S | . | . | . | . | . | . | . | . | . | . | . | . | . | . |
| 21-225_148C8_LC | . | . | . | . | . | . | . | . | . | . | . | . | . | . | . | . | . | . | . | T | . | . | . | . | . | . | . |
| 21-225_148E10_LC | I | . | . | . | . | . | . | . | . | . | . | . | S | . | . | . | . | . | . | . | . | . | . | . | . | . | . |
| 21-225_148G11_LC | . | . | . | . | . | . | . | . | . | . | . | . | S | . | . | . | . | . | . | . | . | . | . | . | . | . | . |
| 21-225_148G6_LC | . | . | . | . | . | . | . | . | . | . | . | . | S | . | . | . | . | . | . | . | . | . | . | . | . | . | . |
| 21-225_149A11_LC | . | . | . | . | . | . | . | . | . | . | . | . | S | H | . | . | . | . | . | . | . | . | . | . | . | . | . |
| 21-225_149B5_LC | . | . | F | . | . | . | . | . | . | . | . | . | N | N | Q | . | . | . | . | . | . | . | . | . | . | . | . |
| 21-225_149C10_LC | . | . | . | . | . | . | . | . | . | . | . | . | N | . | . | . | . | . | . | . | . | . | . | . | . | . | . |
| 21-225_149C7_LC | . | . | . | . | . | . | . | . | . | . | . | . | S | . | . | . | . | . | . | . | . | . | . | . | . | . | . |
| 21-225_14B1_LC2_LC | . | . | . | . | . | . | . | . | . | . | . | . | . | . | . | . | . | . | . | . | . | . | . | . | . | . | . |
| 21-225_14B7_LC | . | . | . | . | . | . | . | . | . | . | . | . | . | . | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | | | | | | L_CDR2 | | | | | | | | | | | | | | | | | | |
| CONSENSUS | V | I | Y | Q | . | . | . | . | . | . | . | . | D | R | K | R | P | S | G | I | P | E | R | F | S | G | S |
| 21-225_14G3_LC | . | . | . | . | . | . | . | . | . | . | . | . | . | . | . | . | . | . | . | . | . | . | . | . | . | . | . |
| 21-225_14H11_LC | . | . | . | . | . | . | . | . | . | . | . | . | . | T | . | . | . | . | . | . | . | . | . | . | . | . | . |
| 21-225_150D3_LC | . | . | . | . | . | . | . | . | . | . | . | . | . | N | . | . | . | . | . | . | . | . | . | . | . | . | . |
| 21-225_150F6_LC | . | . | G | . | . | . | . | . | . | . | . | . | . | S | . | . | . | . | . | . | . | . | . | . | . | . | . |
| 21-225_150H11_LC | . | . | . | . | . | . | . | . | . | . | . | . | . | S | . | . | . | . | . | . | . | . | . | . | . | . | . |
| 21-225_151B9_LC | . | . | . | . | . | . | . | . | . | . | . | . | . | S | . | . | . | . | . | . | . | . | . | . | . | . | . |
| 21-225_151F3_LC | . | . | . | . | . | . | . | . | . | . | . | . | . | . | . | . | . | . | . | . | . | . | . | . | . | . | . |
| 21-225_151H6_LC | . | . | . | . | . | . | . | . | . | . | . | . | . | S | . | . | . | . | . | . | . | . | . | . | . | . | . |
| 21-225_151H7_LC | . | . | . | . | . | . | . | . | . | . | . | . | . | S | . | . | . | . | . | . | . | . | . | . | . | . | . |
| 21-225_152D7_LC | . | . | . | . | . | . | . | . | . | . | . | . | . | S | . | . | . | . | . | . | . | . | . | . | . | . | . |
| 21-225_152G6_LC | . | . | . | . | . | . | . | . | . | . | . | . | . | S | . | . | . | . | . | . | . | . | . | . | . | . | . |
| 21-225_153D9_LC | . | . | . | . | . | . | . | . | . | . | . | . | . | . | . | . | . | . | . | . | . | . | . | . | . | . | . |
| 21-225_153E6_LC | . | . | . | . | . | . | . | . | . | . | . | . | . | N | . | . | . | . | . | . | . | . | . | . | . | . | . |
| 21-225_154C3_LC | . | . | . | . | . | . | . | . | . | . | . | . | . | . | . | . | . | . | . | . | . | . | . | . | . | . | . |
| 21-225_154C4_LC | . | . | . | . | . | . | . | . | . | . | . | . | . | S | . | . | . | . | . | . | . | . | . | . | . | . | . |
| 21-225_154D11_LC | . | . | . | . | . | . | . | . | . | . | . | . | . | S | . | . | . | . | . | . | . | . | . | . | . | . | . |
| 21-225_154E10_LC | . | . | . | . | . | . | . | . | . | . | . | . | . | S | . | . | . | . | . | . | . | . | . | . | . | . | . |
| 21-225_154F4_LC | . | . | N | . | . | . | . | . | . | . | . | . | . | S | . | . | . | . | . | . | . | . | . | . | . | . | . |
| 21-225_155C10_LC | . | . | . | . | . | . | . | . | . | . | . | . | . | S | . | . | . | . | . | . | . | . | . | . | . | . | . |
| 21-225_155E10_LC | . | . | . | . | . | . | . | . | . | . | . | . | . | . | . | . | . | . | T | . | . | . | . | . | . | . | . |
| 21-225_158D10_LC | . | . | . | . | . | . | . | . | . | . | . | . | . | . | . | . | . | . | . | . | . | . | L | . | . | . | . |
| 21-225_158E6_LC | . | . | . | . | . | . | . | . | . | . | . | . | . | S | . | . | . | . | . | . | . | . | . | . | . | . | . |
| 21-225_159H8_LC | . | . | . | . | . | . | . | . | . | . | . | . | . | . | . | . | . | . | . | . | . | . | . | . | . | . | . |
| 21-225_15H11_LC | . | . | . | . | . | . | . | . | . | . | . | . | . | . | . | . | . | . | . | . | . | . | . | . | . | . | . |
| 21-225_160B12_LC | . | . | . | . | . | . | . | . | . | . | . | . | . | S | . | . | . | . | . | . | . | . | . | . | . | . | . |
| 21-225_161E10_LC | . | . | . | . | . | . | . | . | . | . | . | . | . | S | . | . | . | . | . | . | . | . | . | I | . | . | . |
| 21-225_161F12_LC | . | . | . | . | . | . | . | . | . | . | . | . | . | T | . | . | . | . | . | . | . | . | . | . | . | . | . |
| 21-225_161H3_LC | . | . | N | . | . | . | . | . | . | . | . | . | . | N | . | . | . | . | . | . | . | . | . | . | . | . | . |
| 21-225_165H3_LC | . | . | . | . | . | . | . | . | . | . | . | . | . | S | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | | | | | | | L_CDR2 | | | | | | | | | | | | | | | | | |
| CONSENSUS | V | I | Y | Q | . | . | . | . | . | . | . | . | D | R | K | R | P | S | G | I | P | E | R | F | S | G | S |
| 21-225_166G11_LC | . | . | . | . | . | . | . | . | . | . | . | . | . | S | . | . | . | . | . | . | . | . | . | . | . | . | . |
| 21-225_169A6_LC | . | . | . | . | . | . | . | . | . | . | . | . | . | Y | . | . | . | . | . | . | . | . | . | . | . | . | . |
| 21-225_169B7_LC | . | . | . | . | . | . | . | . | . | . | . | . | . | . | . | . | . | . | . | . | . | . | . | . | . | . | . |
| 21-225_169C1_LC | . | . | S | . | . | . | . | . | . | . | . | . | . | I | . | . | . | . | . | . | . | . | . | . | . | . | . |
| 21-225_169G11_LC | . | . | . | . | . | . | . | . | . | . | . | . | . | S | . | . | . | . | . | . | . | . | . | . | . | . | . |
| 21-225_16D2_LC | . | . | . | . | . | . | . | . | . | . | . | . | . | S | . | . | . | . | . | . | . | . | . | . | . | . | . |
| 21-225_16E4_LC | . | . | . | . | . | . | . | . | . | . | . | . | . | S | . | . | . | . | . | . | . | . | . | . | . | . | . |
| 21-225_16G8_LC | . | . | . | . | . | . | . | . | . | . | . | . | . | . | . | . | . | . | . | . | . | . | . | . | . | . | . |
| 21-225_170A5_LC | . | . | . | . | . | . | . | . | . | . | . | . | . | Y | . | . | . | . | . | . | . | . | . | . | . | . | . |
| 21-225_170F1_LC | . | . | . | . | . | . | . | . | . | . | . | . | . | S | . | . | . | . | . | . | . | A | . | . | . | . | . |
| 21-225_171E12_LC | . | . | . | . | . | . | . | . | . | . | . | . | . | . | . | . | . | . | . | . | . | . | . | . | . | . | . |
| 21-225_171F5_LC | . | . | . | . | . | . | . | . | . | . | . | . | . | . | . | . | . | . | . | . | . | . | . | . | . | . | . |
| 21-225_173F8_LC | . | . | S | . | . | . | . | . | . | . | . | . | . | S | . | . | . | . | . | . | . | . | . | . | . | . | . |
| 21-225_175C6_LC | . | . | . | . | . | . | . | . | . | . | . | . | . | Y | . | . | . | . | . | . | . | . | . | . | . | . | . |
| 21-225_179C7_LC | . | . | . | . | . | . | . | . | . | . | . | . | . | S | . | . | . | . | . | . | . | . | . | . | . | . | . |
| 21-225_17D3_LC | . | . | . | . | . | . | . | . | . | . | . | . | . | . | . | . | . | . | . | . | . | . | . | . | . | . | . |
| 21-225_17F5_LC | . | . | . | . | . | . | . | . | . | . | . | . | . | . | . | . | . | . | . | . | . | . | . | . | . | . | . |
| 21-225_180C5_LC | . | . | . | . | . | . | . | . | . | . | . | . | . | . | . | . | . | . | . | . | . | . | . | . | . | . | . |
| 21-225_180D4_LC | . | . | S | . | . | . | . | . | . | . | . | . | . | . | . | . | . | . | . | . | . | . | . | . | . | . | . |
| 21-225_180G5_LC | . | . | . | . | . | . | . | . | . | . | . | . | . | S | . | . | . | . | . | . | . | . | . | . | . | . | . |
| 21-225_181H1_LC | . | . | S | . | . | . | . | . | . | . | . | . | . | . | . | . | . | . | . | . | . | . | . | . | . | . | . |
| 21-225_182D12_LC | . | . | . | . | . | . | . | . | . | . | . | . | . | . | . | . | . | . | . | . | . | . | . | . | . | . | . |
| 21-225_183F4_LC | . | . | . | . | . | . | . | . | . | . | . | . | . | S | . | . | . | . | . | . | . | . | . | . | . | . | . |
| 21-225_185D2_LC | . | . | S | . | . | . | . | . | . | . | . | . | . | . | . | . | . | . | . | . | . | D | . | . | . | . | . |
| 21-225_185G7_LC | . | . | . | . | . | . | . | . | . | . | . | . | . | . | . | . | . | . | . | . | . | . | . | . | . | . | . |
| 21-225_186H6_LC | . | . | . | . | . | . | . | . | . | . | . | . | . | . | . | . | . | . | . | . | . | . | . | . | . | . | . |
| 21-225_18E10_LC | . | . | . | . | . | . | . | . | . | . | . | . | . | . | . | . | . | . | . | . | . | . | . | . | . | . | . |
| 21-225_18G5_LC | . | . | . | . | . | . | . | . | . | . | . | . | . | S | . | . | . | . | . | . | . | . | . | . | . | . | . |
| 21-225_190G9_LC | . | . | . | . | . | . | . | . | . | . | . | . | . | N | . | . | . | . | . | . | . | . | . | . | . | . | . |

**FIGURE 57**

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | | | | L_CDR2 | | | | | | | | | | | | | | | | | | | | |
| CONSENSUS | V | I | Y | Q | - | - | - | - | - | - | - | - | D | R | K | R | P | S | G | I | P | E | R | F | S | G | S |
| 21-225_190H1_LC | . | . | . | . | . | . | . | . | . | . | . | . | . | S | . | . | . | . | . | . | . | . | . | . | . | . | . |
| 21-225_193H5_LC | . | . | . | . | . | . | . | . | . | . | . | . | . | S | . | . | . | . | . | . | . | . | . | . | . | . | . |
| 21-225_195E3_LC | . | . | W | . | . | . | . | . | . | . | . | . | . | S | . | . | . | . | . | . | . | . | . | . | . | . | . |
| 21-225_195E9_LC | . | . | . | . | . | . | . | . | . | . | . | . | . | . | . | . | . | . | . | . | . | . | . | . | . | . | . |
| 21-225_19F8_LC | . | . | . | . | . | . | . | . | . | . | . | . | . | S | . | . | . | . | . | Y | . | . | . | . | . | . | . |
| 21-225_201G11_LC | . | . | . | . | . | . | . | . | . | . | . | . | . | S | . | . | . | . | . | . | . | . | . | . | . | . | . |
| 21-225_203G6_LC | . | . | . | . | . | . | . | . | . | . | . | . | . | N | . | . | . | . | . | . | . | . | . | . | . | . | . |
| 21-225_215B3_LC | . | . | . | . | . | . | . | . | . | . | . | . | . | S | . | . | . | . | . | . | . | . | . | . | . | . | . |
| 21-225_21D12_LC | . | . | . | . | . | . | . | . | . | . | . | . | . | . | . | . | . | . | . | . | . | . | . | . | . | . | . |
| 21-225_224H2_LC | . | V | . | . | . | . | . | . | . | . | . | . | . | S | . | . | . | . | . | . | . | . | . | . | . | . | . |
| 21-225_225E9_LC | . | M | . | . | . | . | . | . | . | . | . | . | . | . | . | . | . | . | . | . | . | . | . | . | . | . | . |
| 21-225_226A10_LC | . | . | . | . | . | . | . | . | . | . | . | . | . | . | . | . | . | . | . | . | . | . | . | . | . | . | . |
| 21-225_226B2_LC | . | . | . | . | . | . | . | . | . | . | . | . | . | . | . | . | . | . | . | . | . | . | . | . | . | . | . |
| 21-225_227E12_LC | . | . | . | . | . | . | . | . | . | . | . | . | . | S | . | . | . | . | . | . | . | . | . | . | . | . | . |
| 21-225_227E5_LC | . | . | . | . | . | . | . | . | . | . | . | . | . | . | . | . | . | . | . | . | . | . | . | . | . | . | . |
| 21-225_22E6_LC | . | . | . | . | . | . | . | . | . | . | . | . | . | . | . | . | . | . | . | . | . | . | . | Y | . | . | . |
| 21-225_23D7_LC | . | . | . | . | . | . | . | . | . | . | . | . | . | S | . | . | . | . | . | . | . | . | . | . | . | . | . |
| 21-225_26C10_LC | . | . | . | . | . | . | . | . | . | . | . | . | . | S | . | . | . | . | . | . | . | . | . | . | . | . | . |
| 21-225_27D9_LC | . | . | . | . | . | . | . | . | . | . | . | . | . | S | . | . | . | . | . | . | . | . | . | . | . | . | . |
| 21-225_27E7_LC | . | . | . | . | . | . | . | . | . | . | . | . | . | S | . | . | . | L | . | . | . | . | . | . | . | . | . |
| 21-225_27G6_LC | . | . | . | . | . | . | . | . | . | . | . | . | . | . | . | . | . | . | . | . | . | . | . | . | . | . | . |
| 21-225_27H2_LC | . | . | . | . | . | . | . | . | . | . | . | . | . | G | . | . | . | . | . | . | . | . | . | . | . | . | . |
| 21-225_28A11_LC | . | . | . | . | . | . | . | . | . | . | . | . | . | S | . | . | . | . | . | . | . | . | . | . | . | . | . |
| 21-225_2B1_LC | . | . | . | . | . | . | . | . | . | . | . | . | . | . | . | . | . | . | . | . | . | . | . | . | . | . | . |
| 21-225_30D9_LC2_LC | . | . | . | . | . | . | . | . | . | . | . | . | . | . | . | . | . | . | . | . | . | . | . | . | . | . | . |
| 21-225_30H6_LC | . | . | . | . | . | . | . | . | . | . | . | . | . | S | . | . | . | . | . | . | . | . | . | . | . | . | . |
| 21-225_31C2_LC | . | . | . | . | . | . | . | . | . | . | . | . | . | S | . | . | . | . | . | . | . | . | . | . | . | . | . |
| 21-225_31D12_LC2_LC | . | . | . | . | . | . | . | . | . | . | . | . | . | . | . | . | . | . | . | . | . | . | . | . | . | . | . |
| 21-225_32B11_LC | . | . | . | . | . | . | . | . | . | . | . | . | . | S | . | . | S | . | . | . | . | . | . | . | . | . | . |

# FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | | | | | | | | L_CDR2 | | | | | | | | | | | | | | | | |
| CONSENSUS | V | I | Y | Q | . | . | . | . | . | . | . | . | . | D | R | K | R | P | S | G | I | P | E | R | F | S | G | S |
| 21-226_33A1_LC | . | . | . | . | . | . | . | . | . | . | . | . | . | S | . | . | . | . | . | . | . | . | . | . | . | . | . |
| 21-225_33E6_LC | . | . | . | . | . | . | . | . | . | . | . | . | . | . | . | . | . | . | . | . | . | . | . | . | . | . | . |
| 21-225_34B11_LC | . | . | . | . | . | . | . | . | . | . | . | . | . | M | . | . | . | . | . | . | . | . | . | . | . | . | . |
| 21-225_34B9_LC | . | . | . | . | . | . | . | . | . | . | . | . | . | S | . | . | . | . | . | . | . | . | . | . | . | . | . |
| 21-225_34D7_LC | . | L | . | . | . | . | . | . | . | . | . | . | . | S | . | . | . | . | . | . | . | . | . | . | . | . | . |
| 21-225_35E1_LC | . | . | . | . | . | . | . | . | . | . | . | . | . | . | . | . | . | . | . | . | . | . | . | . | . | . | . |
| 21-225_3B12_LC | . | . | . | . | . | . | . | . | . | . | . | . | . | . | . | . | . | . | . | . | . | . | . | . | . | . | . |
| 21-226_3H10_LC | . | . | . | . | . | . | . | . | . | . | . | . | . | . | . | . | . | . | . | . | . | . | . | . | . | . | . |
| 21-225_45F6_LC | . | . | . | . | . | . | . | . | . | . | . | . | . | S | . | . | . | . | . | . | . | . | . | . | . | . | . |
| 21-225_4B3_LC | . | . | . | . | . | . | . | . | . | . | . | . | . | . | . | . | . | . | . | . | . | . | . | . | . | . | . |
| 21-225_50G9_LC | . | . | . | . | . | . | . | . | . | . | . | . | . | S | . | . | . | . | . | . | . | . | . | . | . | . | . |
| 21-225_52H1_LC | . | . | . | . | . | . | . | . | . | . | . | . | . | . | . | . | . | . | . | . | . | . | . | . | . | . | . |
| 21-225_53D10_LC | . | . | . | . | . | . | . | . | . | . | . | . | . | . | . | . | . | . | . | . | . | . | . | . | . | . | . |
| 21-225_57D9_LC | . | . | . | . | . | . | . | . | . | . | . | . | . | S | . | . | . | . | . | . | . | . | . | . | . | . | . |
| 21-225_57F1_LC | . | . | . | . | . | . | . | . | . | . | . | . | . | . | . | . | . | . | . | . | . | . | . | . | . | . | . |
| 21-225_57H11_LC | . | . | . | . | . | . | . | . | . | . | . | . | . | . | . | . | . | . | . | . | . | . | . | . | . | . | . |
| 21-225_58C1_LC | . | . | . | . | . | . | . | . | . | . | . | . | . | . | . | . | . | . | . | . | . | . | . | . | . | . | . |
| 21-225_58E7_LC | . | . | . | . | . | . | . | . | . | . | . | . | . | N | . | . | . | . | . | . | . | . | . | . | . | . | . |
| 21-225_58F7_LC | . | . | . | . | . | . | . | . | . | . | . | . | . | . | . | . | . | . | . | . | . | . | . | . | . | . | . |
| 21-225_58F8_LC | . | . | . | . | . | . | . | . | . | . | . | . | . | S | . | . | . | . | . | . | . | . | . | . | . | . | . |
| 21-225_58G11_LC | . | . | . | . | . | . | . | . | . | . | . | . | . | N | . | . | . | . | . | . | . | . | . | . | . | . | . |
| 21-225_59B11_LC | . | . | . | . | . | . | . | . | . | . | . | . | . | S | . | . | . | . | . | L | . | . | . | . | . | . | . |
| 21-225_59F2_LC | . | . | . | . | . | . | . | . | . | . | . | . | . | N | . | . | . | . | . | . | . | . | . | . | . | . | . |
| 2t-225_5G7_LC | . | . | . | . | . | . | . | . | . | . | . | . | . | . | . | . | . | . | . | . | . | . | . | . | . | . | . |
| 21-225_60A12_LC | . | . | . | . | . | . | . | . | . | . | . | . | . | . | . | . | . | . | . | . | . | . | . | . | . | . | . |
| 21-225_60B1_LC | . | . | . | . | . | . | . | . | . | . | . | . | . | . | . | . | . | . | . | . | . | . | . | . | . | . | . |
| 21-225_60D2_LC | . | . | . | . | . | . | . | . | . | . | . | . | . | N | . | . | . | . | . | . | . | . | . | . | . | . | . |
| 21-225_61F5_LC | . | . | . | . | . | . | . | . | . | . | . | . | . | S | . | . | . | . | . | . | . | . | . | . | . | . | . |
| 21-225_62A12_LC | . | . | . | . | . | . | . | . | . | . | . | . | . | . | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | | | | | | | L_CDR2 | | | | | | | | | | | | | | | | | |
| CONSENSUS | V | I | Y | Q | . | . | . | . | . | . | . | . | D | R | K | R | P | S | G | I | P | E | R | F | S | G | S |
| 21-225_62B12_LC | . | . | . | . | . | . | . | . | . | . | . | . | . | . | . | . | . | . | . | . | . | . | . | . | . | . | . |
| 21-225_62D10_LC | . | . | . | . | . | . | . | . | . | . | . | . | . | . | . | . | . | . | . | . | . | . | . | . | . | . | . |
| 21-225_62E3_LC | . | . | . | . | . | . | . | . | . | . | . | . | . | . | . | . | . | . | . | . | . | . | . | . | . | . | . |
| 21-225_62E6_LC | . | . | . | . | . | . | . | . | . | . | . | . | . | . | . | . | . | . | . | . | . | . | . | . | . | . | . |
| 21-225_65E9_LC | . | . | . | . | . | . | . | . | . | . | . | . | . | . | . | . | . | . | . | . | . | . | . | . | . | . | . |
| 21-225_66G9_LC | . | . | . | . | . | . | . | . | . | . | . | . | . | . | . | . | . | . | . | . | . | . | . | . | . | . | . |
| 21-225_67F10_LC | . | . | Y | S | . | . | . | . | . | . | . | . | . | . | . | . | . | . | . | . | . | . | . | . | . | . | . |
| 21-225_68D8_LC | . | . | . | . | . | . | . | . | . | . | . | . | . | S | . | . | . | . | . | . | . | . | . | . | . | . | . |
| 21-225_68G6_LC | . | . | . | . | . | . | . | . | . | . | . | . | . | . | . | . | . | . | . | . | . | D | . | . | . | . | . |
| 21-225_69B11_LC | . | . | . | S | . | . | . | . | . | . | . | . | . | . | . | . | . | . | . | . | . | . | . | . | . | . | . |
| 21-225_69B9_LC | . | . | . | . | . | . | . | . | . | . | . | . | . | . | . | . | . | . | . | . | . | . | . | . | . | . | . |
| 21-225_6B4_LC | . | . | . | . | . | . | . | . | . | . | . | . | . | . | . | . | . | . | . | . | . | . | . | . | . | . | . |
| 21-225_6D9_LC | . | . | . | . | . | . | . | . | . | . | . | . | . | X | . | . | . | . | . | . | . | . | . | . | . | . | . |
| 21-225_71D4_LC | . | . | . | . | . | . | . | . | . | . | . | . | . | . | . | . | . | . | . | . | . | . | . | . | . | . | . |
| 21-225_72B4_LC | . | . | S | . | . | . | . | . | . | . | . | . | . | . | . | . | . | . | . | . | . | . | . | . | . | . | . |
| 21-225_72D5_LC | . | . | . | . | . | . | . | . | . | . | . | . | . | . | . | . | . | . | . | . | . | . | . | . | . | . | . |
| 21-225_73C4_LC | . | . | . | . | . | . | . | . | . | . | . | . | . | M | . | . | . | . | . | . | . | . | . | . | . | . | . |
| 21-225_74A9_LC | . | . | . | . | . | . | . | . | . | . | . | . | . | N | R | . | . | . | . | . | . | . | . | . | . | . | . |
| 21-225_74B3_LC | . | . | . | . | . | . | . | . | . | . | . | . | . | S | . | . | . | . | . | . | . | . | . | . | . | . | . |
| 21-225_74C9_LC | . | . | . | . | . | . | . | . | . | . | . | . | . | N | R | . | . | . | . | . | . | . | . | . | . | . | . |
| 21-225_76B4_LC | . | . | . | . | . | . | . | . | . | . | . | . | . | S | . | . | . | . | . | . | . | . | . | . | . | . | . |
| 21-225_77A2_LC | . | . | . | . | . | . | . | . | . | . | . | . | . | . | . | . | . | . | . | . | . | . | . | . | . | . | . |
| 21-225_78E9_LC | . | . | . | . | . | . | . | . | . | . | . | . | . | N | R | . | . | . | . | . | . | . | . | . | . | . | . |
| 21-225_79E7_LC | . | . | . | . | . | . | . | . | . | . | . | . | . | N | R | . | . | . | . | . | . | . | . | . | . | . | . |
| 21-225_8D8_LC | . | . | . | . | . | . | . | . | . | . | . | . | . | . | . | . | . | . | . | . | . | . | . | . | . | . | . |
| 21-225_92A4_LC | . | . | . | . | . | . | . | . | . | . | . | . | . | N | R | . | . | . | . | . | . | . | . | . | . | . | . |
| 21-225_96B5_LC | . | . | . | . | . | . | . | . | . | . | . | . | . | S | . | . | . | . | . | . | . | . | . | . | . | . | . |
| 21-225_97E8_LC | . | . | . | . | . | . | . | . | . | . | . | . | . | N | R | . | . | . | . | . | . | . | . | . | . | . | . |
| 21-225_9H10_LC | . | . | . | . | . | . | . | . | . | . | . | . | . | S | . | . | . | . | . | . | . | . | . | . | . | . | . |

FIGURE 57

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | | | | | | | L_FR3 | | | | | | | | | | | | | | | | | | | | |
| CONSENSUS | N | S | G | - | - | N | T | A | T | L | T | I | S | G | T | Q | A | M | D | E | A | O | Y | Y | C | Q | A |
| 21-225_10B10_LC | | | | | | | | | | | | | | | | | | | | | | | | | | | |
| 21-225_12B1_LC | | | | | | | | | | | | | | | | | | | | | | | | | | | |
| 21-225_13F6_LC | | | | | | | | | | | | | | | | | | | | | | | | | | | |
| 21-225_146A10_LC | | | | | | | | | | | | | | | | | | | | | | | | | | | |
| 21-225_146A2_LC | | | | | | | | | | | | | | | | | | | | | | | | | | | |
| 21-225_146A3_LC | | | | | | | | | | | | | | | | | | | | | | | | | | | |
| 21-225_146B11_LC | | | | | | | | | | | | | | | | | | | | | | | | | | | |
| 21-225_146B8_LC | | | | | | | | | | | | | | | | | | | | | | | | | | | |
| 21-225_146C11_LC | | | | | | | | | | | | | | | | | | | | | | | | | | | |
| 21-225_146H8_LC | | | | | | | | | | | | | | | | | | | | | | | | | | | |
| 21-225_147B12_LC | | | | | | | | | | | | | | | | | | | | | | | | | | | |
| 21-225_147B8_LC | | | | | | | | | | | | | | | | | | | | | | | | | | | |
| 21-225_147B9_LC | | | | | | | | | | | | | | | | | | | | | | | | | | | |
| 21-225_147D9_LC | | | | | | | | | | | | | | | | | | | | | | | | | | | |
| 21-225_147E11_LC | | | | | | | | | | | | | | | | | | | | | | | | | | | |
| 21-225_147E7_LC | | | | | | | | | | | | | | | | | | | | | | | | | | | |
| 21-225_147F9_LC | | | | | | | | | | | | | | | | | | | | | | | | | | | |
| 21-225_147G9_LC | | | | | | | | | | | | | | | | | | | | | | | | | | | |
| 21-225_148A9_LC | | | | | | | | | | | | | | | | | | | | | | | | | | | |
| 21-225_148C8_LC | | | | | | | | | | | | | | | | | | | | | | | | | | | |
| 21-225_148E10_LC | | | | | | | | | | | | | | | | | | | | | | | | | | | |
| 21-225_148G11_LC | | | | | | | | | | | | | | | | | | | | | | | | | | | |
| 21-225_148G6_LC | | | | | | | | | | | | | | | | | | | | | | | | | | | |
| 21-225_149A11_LC | | | | | | | | | | | | | | | | | | | | | | | | | | | |
| 21-225_149B6_LC | | | | | | | | | | | | | | | | | | | | | | | | | | | |
| 21-225_149C10_LC | | | | | | | | | | | | | | | | | | | | | | | | | | | |
| 21-225_149C7_LC | | | | | | | | | | | | | | | | | | | | | | | | | | | |
| 21-225_14B1_LC2_LC | | | | | | | | | | | | | | | | | | | | | | | | | | | |
| 21-225_14B7_LC | | | | | | | | | | | | | | | | | | | | | | | | | | | |

**FIGURE 57**

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | | | | | | | | L_FR3 | | | | | | | | | | | | | | | | | | | |
| CONSENSUS | N | S | G | - | - | N | T | A | T | L | T | I | S | G | T | Q | A | M | D | E | A | O | Y | Y | C | Q | A |
| 21-225_14G3_LC | . | . | . | . | . | . | . | . | . | . | . | . | . | . | . | . | . | . | . | . | . | . | . | . | . | . | . |
| 21-225_14H11_LC | . | . | . | . | . | . | . | . | . | . | . | . | . | . | . | . | . | . | . | . | . | . | . | . | . | . | . |
| 21-225_150D3_LC | S | . | . | . | . | . | . | . | . | . | . | . | . | . | . | . | . | . | . | . | . | . | . | . | . | . | . |
| 21-225_150F6_LC | . | . | . | . | . | . | . | . | . | . | . | . | . | . | . | . | . | . | . | . | . | . | . | . | C | . | . |
| 21-225_150H11_LC | . | . | . | . | . | . | . | . | . | . | . | . | i | . | . | . | . | . | . | . | . | . | F | . | . | . | . |
| 21-225_151B9_LC | . | . | . | . | . | . | . | . | . | . | . | . | i | . | . | . | . | . | . | . | . | . | . | . | . | . | . |
| 21-225_151F3_LC | . | . | . | . | . | . | . | . | . | . | . | . | . | . | . | . | . | . | . | . | . | . | . | . | . | . | . |
| 21-225_151H5_LC | . | . | . | . | . | . | . | . | . | . | . | . | i | . | . | . | . | . | . | . | . | . | . | . | . | . | . |
| 21-225_151H7_LC | . | . | . | . | . | . | . | . | . | . | . | . | i | . | . | . | . | . | . | . | . | . | . | . | . | . | . |
| 21-225_152D7_LC | . | . | . | . | . | . | . | . | . | . | . | . | i | . | . | . | . | . | . | . | . | . | . | . | . | . | . |
| 21-225_152G6_LC | . | . | . | . | . | . | . | . | . | . | . | . | i | . | . | . | L | . | . | . | . | . | . | . | . | . | . |
| 21-225_153D9_LC | . | . | . | . | . | . | . | . | . | . | . | . | . | . | . | . | . | . | . | . | . | . | . | . | . | . | . |
| 21-225_153E8_LC | . | . | . | . | . | . | . | . | . | . | . | . | . | . | . | . | . | . | . | . | . | G | . | . | . | . | . |
| 21-225_154C3_LC | . | . | . | . | . | . | . | . | . | . | . | T | . | . | . | . | . | . | . | . | . | . | . | . | . | . | . |
| 21-225_154C4_LC | . | . | . | . | . | . | . | . | . | . | . | . | i | . | . | . | . | . | . | . | . | . | . | . | . | . | . |
| 21-225_154D11_LC | . | . | . | . | . | i | . | . | . | . | . | . | . | . | . | . | . | . | . | . | . | . | . | . | . | . | . |
| 21-225_154E10_LC | . | . | . | . | . | . | . | . | . | . | . | . | i | . | . | . | . | . | . | . | . | . | . | . | . | . | . |
| 21-225_154F4_LC | . | . | . | . | . | . | G | . | . | . | . | . | . | . | . | . | . | . | . | . | . | . | . | . | . | . | . |
| 21-225_155C10_LC | . | . | . | . | . | . | . | . | . | . | . | . | i | . | . | . | . | . | . | . | . | . | . | . | . | . | . |
| 21-225_155E10_LC | . | . | . | . | . | . | . | . | . | . | . | . | . | . | . | . | . | . | . | . | . | . | . | . | . | L | . |
| 21-225_158D10_LC | . | . | . | . | . | . | . | . | . | . | . | . | . | . | . | . | L | . | . | . | . | . | . | . | . | . | . |
| 21-225_158E9_LC | . | . | . | . | . | . | . | . | . | . | . | T | . | . | . | . | . | . | . | . | N | . | . | . | . | . | . |
| 21-225_159H8_LC | . | . | . | . | . | . | . | . | . | . | . | . | . | . | . | . | L | . | . | . | . | . | . | . | . | . | . |
| 21-225_15H11_LC | . | . | . | . | . | . | . | . | . | . | . | . | . | . | . | . | i | . | . | . | . | . | . | . | . | . | . |
| 21-225_160B12_LC | . | . | . | . | . | . | . | . | . | . | . | . | . | . | . | . | L | . | . | . | . | . | . | . | . | . | . |
| 21-225_161E10_LC | . | . | . | . | . | . | . | . | . | . | . | . | . | . | . | . | . | . | . | . | . | . | . | . | . | . | T |
| 21-225_161F12_LC | . | . | . | . | . | . | . | . | . | . | . | . | . | . | . | . | . | . | . | . | . | . | . | . | . | . | . |
| 21-225_161H3_LC | . | . | . | . | . | . | . | . | . | . | . | . | . | . | . | . | L | . | . | . | . | . | . | . | . | . | . |
| 21-225_165H3_LC | . | . | . | . | . | . | . | . | . | . | . | . | . | . | . | . | . | . | . | . | . | . | . | . | . | . | . |

**FIGURE 57**

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | | | | | | | L_FR3 | | | | | | | | | | | | | | | | | | | | | |
| CONSENSUS | N | S | G | - | ~ | N | T | A | T | L | T | I | S | G | T | Q | A | M | D | E | A | D | Y | Y | C | Q | A |
| 21-225_166G11_LC | . | . | . | . | . | . | . | . | . | . | . | . | . | . | . | . | . | . | . | . | . | . | . | . | . | . | . |
| 21-225_169A6_LC | . | . | . | . | . | . | . | . | . | . | . | . | . | . | . | . | . | . | . | . | . | . | . | . | . | . | . |
| 21-225_169B7_LC | . | . | . | . | . | . | . | . | . | . | . | . | . | . | . | . | . | . | . | . | . | . | . | . | . | . | . |
| 21-225_189C1_LC | . | . | . | . | . | . | . | . | . | . | . | . | . | . | . | . | . | . | . | . | . | . | . | . | . | . | . |
| 21-225_189G11_LC | . | . | . | . | . | . | . | . | . | . | . | . | . | . | . | . | . | . | . | . | . | . | . | . | . | . | . |
| 21-225_16D2_LC | . | . | . | . | . | . | . | . | . | . | . | . | . | . | . | . | . | . | . | . | . | . | . | . | . | . | . |
| 21-225_16E4_LC | . | . | . | . | . | . | . | . | A | . | . | . | . | . | . | . | . | . | . | . | . | . | . | . | . | . | . |
| 21-225_16G8_LC | . | . | . | . | . | . | M | . | . | . | . | . | . | . | . | . | . | . | . | . | . | . | . | . | . | . | . |
| 21-225_170A5_LC | . | . | . | . | . | . | . | . | . | . | . | . | . | . | . | . | . | . | . | . | . | . | . | . | . | . | . |
| 21-225_170F1_LC | . | . | . | . | . | . | . | . | . | . | . | . | . | . | . | . | . | . | . | . | . | . | . | . | . | . | . |
| 21-225_171E12_LC | . | . | . | . | . | . | . | . | . | . | . | . | . | . | . | . | . | . | . | . | . | . | . | . | . | . | . |
| 21-225_171F5_LC | . | . | . | . | . | . | . | . | . | . | . | . | . | . | . | . | . | . | . | . | . | . | . | . | . | . | . |
| 21-225_173F8_LC | . | . | . | . | . | . | . | . | . | . | . | . | . | . | . | . | . | . | . | . | . | . | . | . | . | . | . |
| 21-225_176C8_LC | . | . | . | . | . | . | . | . | . | . | . | . | . | . | . | . | . | . | . | . | . | . | . | . | . | . | . |
| 21-225_179C7_LC | . | . | . | . | . | . | . | . | . | . | . | . | R | . | . | . | . | . | . | . | . | . | . | . | . | . | . |
| 21-225_17D3_LC | . | . | . | . | . | . | . | . | . | . | . | . | . | . | . | . | . | . | . | . | . | . | . | . | . | . | . |
| 21-225_17F5_LC | . | . | . | . | . | . | . | . | . | . | . | . | . | . | . | . | . | . | . | . | . | . | . | . | . | . | . |
| 21-225_180C5_LC | . | . | . | . | . | . | . | . | . | . | . | . | . | . | . | . | . | . | . | . | . | . | . | . | . | . | . |
| 21-225_180D4_LC | . | . | . | . | . | . | . | . | . | . | . | . | . | . | . | . | . | . | . | . | G | . | . | . | . | . | . |
| 21-225_180G5_LC | . | . | . | . | . | N | . | . | S | . | . | . | . | . | . | . | . | . | . | . | . | . | . | . | . | . | . |
| 21-225_181H1_LC | . | . | . | . | . | . | . | . | . | . | . | . | . | . | . | . | . | . | . | . | . | . | . | . | . | . | . |
| 21-225_182D12_LC | . | . | . | . | . | . | . | . | . | . | . | . | . | . | . | . | . | . | . | . | . | . | . | . | . | . | . |
| 21-225_183F4_LC | . | . | . | . | . | . | . | . | . | . | . | . | . | . | . | . | . | . | . | . | . | . | . | . | . | . | . |
| 21-225_185D2_LC | . | . | . | . | . | . | . | . | . | . | . | . | . | . | . | . | . | . | . | . | . | . | . | . | . | . | . |
| 21-225_186G7_LC | . | . | . | . | . | . | . | . | . | . | . | . | . | . | . | . | . | . | . | . | . | . | . | . | . | . | . |
| 21-225_186H6_LC | . | . | . | . | . | . | . | . | . | . | . | . | . | . | . | . | . | . | . | . | . | . | . | . | . | . | . |
| 21-225_18E10_LC | . | . | . | . | . | . | . | . | . | . | . | . | . | . | . | . | . | . | . | . | . | . | . | . | . | . | . |
| 21-225_18G5_LC | . | . | . | . | . | . | . | . | . | . | . | . | . | . | . | . | . | . | . | . | . | . | . | . | . | . | . |
| 21-225_190G9_LC | . | . | . | . | . | . | . | . | S | . | . | . | . | . | . | . | . | . | . | . | . | . | . | . | . | . | . |

EP 4 435 105 A2

**FIGURE 57**

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | | | | | | | | L_FR3 | | | | | | | | | | | | | | | | | | | |
| CONSENSUS | N | S | G | - | - | N | T | A | T | L | T | I | S | G | T | Q | A | M | D | E | A | D | Y | Y | C | Q | A |
| 21-225_190H1_LC | . | . | . | . | . | . | . | . | . | . | . | . | . | . | . | . | . | . | . | . | . | . | . | . | . | K | . |
| 21-225_193H5_LC | . | . | . | . | . | . | . | . | . | . | . | . | . | . | . | . | . | . | . | . | . | . | . | . | . | . | . |
| 21-225_196E3_LC | . | . | . | . | . | . | . | . | . | . | . | . | . | . | . | . | . | . | . | . | . | . | . | . | . | . | . |
| 21-226_195E9_LC | . | . | . | . | . | . | G | . | . | . | . | . | . | . | . | S | . | . | . | . | . | . | . | . | . | . | . |
| 21-225_19F6_LC | . | . | . | . | . | . | . | . | . | . | . | . | . | . | . | . | . | . | . | . | . | . | . | . | . | . | . |
| 21-225_201G11_LC | . | . | . | . | . | . | . | . | . | . | . | . | . | . | . | . | . | . | . | . | . | . | . | . | . | . | . |
| 21-225_203G6_LC | . | . | . | . | . | . | . | . | . | . | . | . | . | . | . | S | . | . | . | . | . | . | . | . | . | . | . |
| 21-225_215B3_LC | . | . | . | . | . | . | . | . | . | . | . | . | . | . | . | . | . | . | . | . | . | . | . | . | . | . | . |
| 21-225_21D12_LC | . | . | . | . | . | . | . | . | . | . | . | . | . | . | . | . | . | . | . | . | N | . | . | . | . | . | . |
| 21-225_224H2_LC | . | . | . | . | . | . | . | . | . | . | . | . | . | . | . | . | . | . | . | . | . | . | . | . | . | . | . |
| 21-225_225E9_LC | . | . | . | . | . | . | . | . | . | . | . | . | . | . | . | . | . | . | . | . | . | . | . | . | . | . | . |
| 21-225_226A10_LC | S | . | . | . | . | . | . | . | . | . | . | . | . | . | . | . | . | . | . | . | . | . | . | . | . | . | . |
| 21-225_226B2_LC | S | . | . | . | . | . | . | . | . | . | . | . | . | . | . | . | . | . | . | . | . | . | . | . | . | . | . |
| 21-225_227E12_LC | . | . | . | . | . | . | . | . | . | . | . | . | . | . | . | . | . | . | . | . | . | . | . | . | . | . | . |
| 21-225_227E5_LC | . | . | . | . | . | H | . | . | . | . | . | . | . | . | . | . | . | . | . | . | . | . | . | . | . | . | . |
| 21-225_22E6_LC | . | T | . | . | . | . | . | . | . | . | . | . | . | . | . | . | . | . | . | . | . | . | . | . | . | . | . |
| 21-225_23D7_LC | . | . | . | . | . | . | . | . | . | . | . | . | . | . | . | . | . | . | . | . | . | . | . | . | . | . | . |
| 21-225_26C10_LC | . | . | . | . | . | . | . | . | . | . | . | . | . | . | . | . | . | . | . | . | . | . | . | . | . | . | . |
| 21-226_27D9_LC | . | . | . | . | . | . | . | . | . | . | . | . | . | . | . | . | . | . | . | . | . | . | . | . | . | . | . |
| 21-225_27E7_LC | . | . | . | . | . | . | . | . | . | . | . | . | . | . | . | . | . | . | . | . | . | . | . | . | . | . | . |
| 21-225_27G6_LC | . | . | . | . | . | . | . | . | . | . | . | . | . | . | . | . | T | . | . | . | . | . | . | . | . | . | . |
| 21-225_27H2_LC | . | . | . | . | . | . | . | . | . | L | . | . | . | . | . | . | . | . | . | . | . | . | . | . | . | . | . |
| 21-225_28A11_LC | . | . | . | . | . | . | . | . | . | . | . | . | . | . | . | . | . | . | . | . | . | . | . | . | . | . | . |
| 21-225_28I_LC | . | . | . | . | . | . | . | . | . | . | . | . | . | . | . | . | . | . | . | . | . | . | . | . | . | . | . |
| 21-225_30D9_LC2_LC | . | . | . | . | . | . | . | . | . | . | . | . | . | . | . | . | . | L | . | . | . | . | . | . | . | . | . |
| 21-225_30H6_LC | . | . | . | . | . | . | . | . | . | . | . | . | . | . | . | . | . | . | . | . | . | . | . | . | . | . | . |
| 21-225_31C2_LC | . | . | . | . | . | . | . | . | . | . | . | . | . | . | . | . | . | . | . | . | . | . | . | . | . | . | . |
| 21-225_31D12_LC2_LC | . | . | . | . | . | . | . | . | . | . | . | . | . | . | . | . | . | . | . | . | . | . | . | . | . | . | . |
| 21-225_32B11_LC | . | . | . | . | . | . | . | . | . | . | . | . | . | . | . | . | . | . | . | . | . | . | . | . | . | . | . |

**4375**

FIGURE 57

**FIGURE 57**

**FIGURE 57**

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | | | | | | | | | | | | | | L_CDR3 | | | | | | | | | | | | | |
| CONSENSUS | W | D | S | . | . | . | . | . | . | . | . | . | . | . | . | . | . | . | . | . | . | . | . | . | . | . | S |
| 21-225_10B10_LC | . | | N | . | . | . | . | . | . | . | . | . | . | . | . | . | . | . | . | . | . | . | . | . | . | . | |
| 21-225_12B1_LC | . | | N | . | . | . | . | . | . | . | . | . | . | . | . | . | . | . | . | . | . | . | . | . | . | . | N |
| 21-225_13F6_LC | . | G | N | . | . | . | . | . | . | . | . | . | . | . | . | . | . | . | . | . | . | . | . | . | . | S | T |
| 21-225_146A10_LC | . | | | . | . | . | . | . | . | . | . | . | . | . | . | . | . | . | . | . | . | . | . | . | . | . | . |
| 21-225_146A2_LC | . | | | . | . | . | . | . | . | . | . | . | . | . | . | . | . | . | . | . | . | . | . | . | . | . | |
| 21-225_146A3_LC | . | | | . | . | . | . | . | . | . | . | . | . | . | . | . | . | . | . | . | . | . | . | . | . | . | |
| 21-225_146B11_LC | . | | | . | . | . | . | . | . | . | . | . | . | . | . | . | . | . | . | . | . | . | . | . | . | . | |
| 21-225_146B8_LC | . | N | | . | . | . | . | . | . | . | . | . | . | . | . | . | . | . | . | . | . | . | . | . | . | . | |
| 21-225_146C11_LC | . | | | . | . | . | . | . | . | . | . | . | . | . | . | . | . | . | . | . | . | . | . | . | . | . | |
| 21-225_146H8_LC | . | | | . | . | . | . | . | . | . | . | . | . | . | . | . | . | . | . | . | . | . | . | . | . | . | N |
| 21-225_147B12_LC | . | | | . | . | . | . | . | . | . | . | . | . | . | . | . | . | . | . | . | . | . | . | . | . | . | |
| 21-225_147B8_LC | . | G | | . | . | . | . | . | . | . | . | . | . | . | . | . | . | . | . | . | . | . | . | . | . | N | T |
| 21-225_147B9_LC | . | | | . | . | . | . | . | . | . | . | . | . | . | . | . | . | . | . | . | . | . | . | . | . | S | T |
| 21-225_147D9_LC | . | | | . | . | . | . | . | . | . | . | . | . | . | . | . | . | . | . | . | . | . | . | . | . | N | T |
| 21-225_147E11_LC | . | | | . | . | . | . | . | . | . | . | . | . | . | . | . | . | . | . | . | . | . | . | . | . | . | . |
| 21-225_147E7_LC | . | | | . | . | . | . | . | . | . | . | . | . | . | . | . | . | . | . | . | . | . | . | . | . | . | . |
| 21-225_147F9_LC | . | N | | . | . | . | . | . | . | . | . | . | . | . | . | . | . | . | . | . | . | . | . | . | . | . | |
| 21-225_147G9_LC | . | N | | . | . | . | . | . | . | . | . | . | . | . | . | . | . | . | . | . | . | . | . | . | . | . | |
| 21-225_148A9_LC | . | N | | . | . | . | . | . | . | . | . | . | . | . | . | . | . | . | . | . | . | . | . | . | . | . | |
| 21-225_148C8_LC | . | | | . | . | . | . | . | . | . | . | . | . | . | . | . | . | . | . | . | . | . | . | . | . | S | T |
| 21-225_148E10_LC | . | | | . | . | . | . | . | . | . | . | . | . | . | . | . | . | . | . | . | . | . | . | . | . | N | T |
| 21-225_148G11_LC | . | | | . | . | . | . | . | . | . | . | . | . | . | . | . | . | . | . | . | . | . | . | . | . | . | |
| 21-225_148G6_LC | . | | | . | . | . | . | . | . | . | . | . | . | . | . | . | . | . | . | . | . | . | . | . | . | . | |
| 21-225_149A11_LC | . | | | . | . | . | . | . | . | . | . | . | . | . | . | . | . | . | . | . | . | . | . | . | . | S | T |
| 21-225_149B6_LC | . | | | . | . | . | . | . | . | . | . | . | . | . | . | . | . | . | . | . | . | . | . | . | . | . | . |
| 21-225_149C10_LC | . | | | . | . | . | . | . | . | . | . | . | . | . | . | . | . | . | . | . | . | . | . | . | . | . | |
| 21-225_149C7_LC | . | | | . | . | . | . | . | . | . | . | . | . | . | . | . | . | . | . | . | . | . | . | . | . | . | |
| 21-225_14B1_LC2_LC | . | . | N | . | . | . | . | . | . | . | . | . | . | . | . | . | . | . | . | . | . | . | . | . | . | . | |
| 21-225_14B7_LC | . | N | N | . | . | . | . | . | . | . | . | . | . | . | . | . | . | . | . | . | . | . | . | . | . | . | |

**FIGURE 57**

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | | | | | | | | | | | | | | L_CDR3 | | | | | | | | | | | | | |
| CONSENSUS | W | D | S | ~ | ~ | ~ | ~ | ~ | ~ | ~ | ~ | ~ | ~ | ~ | ~ | ~ | ~ | ~ | ~ | ~ | ~ | ~ | ~ | ~ | ~ | ~ | S |
| 21-225_166G11_LC | . | | N | . | | | | | | | | | | | | | | | | | | | | | | . | |
| 21-225_169A6_LC | . | | T | . | | | | | | | | | | | | | | | | | | | | | | . | N |
| 21-225_169B7_LC | . | . | K | . | | | | | | | | | | | | | | | | | | | | | | . | N |
| 21-225_169C1_LC | . | . | T | | | | | | | | | | | | | | | | | | | | | | | . | N |
| 21-225_169G11_LC | . | | N | . | | | | | | | | | | | | | | | | | | | | | | . | |
| 21-225_16D2_LC | . | . | | | | | | | | | | | | | | | | | | | | | | | | S | T |
| 21-225_16E4_LC | . | | | | | | | | | | | | | | | | | | | | | | | | | . | |
| 21-225_16G8_LC | . | V | N | . | | | | | | | | | | | | | | | | | | | | | | . | N |
| 21-225_170A5_LC | . | | N | | | | | | | | | | | | | | | | | | | | | | | . | |
| 21-225_170F1_LC | . | | | | | | | | | | | | | | | | | | | | | | | | | . | N |
| 21-225_171E12_LC | . | | I | . | | | | | | | | | | | | | | | | | | | | | | S | T |
| 21-225_171F5_LC | . | | K | . | | | | | | | | | | | | | | | | | | | | | | . | N |
| 21-225_173F8_LC | . | | | | | | | | | | | | | | | | | | | | | | | | | . | F |
| 21-225_175C5_LC | . | | N | | | | | | | | | | | | | | | | | | | | | | | . | |
| 21-225_179C7_LC | . | | | | | | | | | | | | | | | | | | | | | | | | | N | T |
| 21-225_17D3_LC | . | | | | | | | | | | | | | | | | | | | | | | | | | . | I |
| 21-225_17F5_LC | . | | ~ | | | | | | | | | | | | | | | | | | | | | | | . | |
| 21-225_180C5_LC | . | | | | | | | | | | | | | | | | | | | | | | | | | | |
| 21-225_180D4_LC | . | | | | | | | | | | | | | | | | | | | | | | | | | | K |
| 21-225_180G5_LC | . | | I | . | | | | | | | | | | | | | | | | | | | | | | | T |
| 21-225_181H1_LC | . | | | | | | | | | | | | | | | | | | | | | | | | | | F |
| 21-225_182D12_LC | . | | | | | | | | | | | | | | | | | | | | | | | | | . | R |
| 21-225_183F4_LC | . | | N | | | | | | | | | | | | | | | | | | | | | | | S | T |
| 21-225_185D2_LC | . | | | | | | | | | | | | | | | | | | | | | | | | | . | K |
| 21-225_185G7_LC | . | | ~ | | | | | | | | | | | | | | | | | | | | | | | | |
| 21-225_186H6_LC | . | | | | | | | | | | | | | | | | | | | | | | | | | | |
| 21-225_18E10_LC | . | | | | | | | | | | | | | | | | | | | | | | | | | | |
| 21-225_18G5_LC | N | | | | | | | | | | | | | | | | | | | | | | | | | | |
| 21-225_19G9_LC | . | | | | | | | | | | | | | | | | | | | | | | | | | | |

FIGURE 57

| Reference # | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|---|
| | | | | | | | | | | | | | | | L_CDR3 | | | | | | | | | | | | | |
| CONSENSUS | W | D | S | . | . | . | . | . | . | . | . | . | . | . | . | . | . | . | . | . | . | . | . | . | . | . | . | S |
| 21-225_190H1_LC | . | . | . | . | . | . | . | . | . | . | . | . | . | . | . | . | . | . | . | . | . | . | . | . | . | . | . | . |
| 21-225_193H5_LC | . | . | . | . | . | . | . | . | . | . | . | . | . | . | . | . | . | . | . | . | . | . | . | . | . | . | . | . |
| 21-225_196E3_LC | . | . | . | . | . | . | . | . | . | . | . | . | . | . | . | . | . | . | . | . | . | . | . | . | . | . | . | V |
| 21-225_195E9_LC | . | . | R | . | . | . | . | . | . | . | . | . | . | . | . | . | . | . | . | . | . | . | . | . | . | . | . | G |
| 21-225_19F6_LC | . | . | N | . | . | . | . | . | . | . | . | . | . | . | . | . | . | . | . | . | . | . | . | . | . | . | . | N |
| 21-225_201G11_LC | . | . | . | . | . | . | . | . | . | . | . | . | . | . | . | . | . | . | . | . | . | . | . | . | . | . | . | . |
| 21-225_203G6_LC | . | . | . | . | . | . | . | . | . | . | . | . | . | . | . | . | . | . | . | . | . | . | . | . | . | . | . | . |
| 21-225_215B3_LC | . | . | . | . | . | . | . | . | . | . | . | . | . | . | . | . | . | . | . | . | . | . | . | . | . | . | . | . |
| 21-225_21D12_LC | . | . | N | . | . | . | . | . | . | . | . | . | . | . | . | . | . | . | . | . | . | . | . | . | . | . | . | . |
| 21-225_224H2_LC | . | . | . | . | . | . | . | . | . | . | . | . | . | . | . | . | . | . | . | . | . | . | . | . | . | . | . | . |
| 21-225_225E9_LC | . | . | . | . | . | . | . | . | . | . | . | . | . | . | . | . | . | . | . | . | . | . | . | . | . | . | S | T |
| 21-225_226A10_LC | . | . | N | . | . | . | . | . | . | . | . | . | . | . | . | . | . | . | . | . | . | . | . | . | . | . | . | G |
| 21-225_226B2_LC | . | . | N | . | . | . | . | . | . | . | . | . | . | . | . | . | . | . | . | . | . | . | . | . | . | . | . | G |
| 21-225_227E12_LC | . | N | . | . | . | . | . | . | . | . | . | . | . | . | . | . | . | . | . | . | . | . | . | . | . | . | . | . |
| 21-225_227E5_LC | . | V | N | . | . | . | . | . | . | . | . | . | . | . | . | . | . | . | . | . | . | . | . | . | . | . | . | N |
| 21-225_22E6_LC | . | . | . | . | . | . | . | . | . | . | . | . | . | . | . | . | . | . | . | . | . | . | . | . | . | . | . | . |
| 21-225_23D7_LC | . | N | . | . | . | . | . | . | . | . | . | . | . | . | . | . | . | . | . | . | . | . | . | . | . | . | . | . |
| 21-225_26C10_LC | . | V | . | . | . | . | . | . | . | . | . | . | . | . | . | . | . | . | . | . | . | . | . | . | . | . | . | N |
| 21-225_27D9_LC | . | V | . | . | . | . | . | . | . | . | . | . | . | . | . | . | . | . | . | . | . | . | . | . | . | . | . | N |
| 21-225_27E7_LC | . | . | . | . | . | . | . | . | . | . | . | . | . | . | . | . | . | . | . | . | . | . | . | . | . | . | . | . |
| 21-225_27G6_LC | . | . | . | . | . | . | . | . | . | . | . | . | . | . | . | . | . | . | . | . | . | . | . | . | . | . | S | . |
| 21-225_27H2_LC | . | . | . | . | . | . | . | . | . | . | . | . | . | . | . | . | . | . | . | . | . | . | . | . | . | . | S | T |
| 21-225_28A11_LC | . | . | . | . | . | . | . | . | . | . | . | . | . | . | . | . | . | . | . | . | . | . | . | . | . | . | S | T |
| 21-225_28B1_LC | . | . | N | . | . | . | . | . | . | . | . | . | . | . | . | . | . | . | . | . | . | . | . | . | . | . | . | N |
| 21-225_30D9_LC2_LC | . | . | N | . | . | . | . | . | . | . | . | . | . | . | . | . | . | . | . | . | . | . | . | . | . | . | . | . |
| 21-225_30H6_LC | . | . | . | . | . | . | . | . | . | . | . | . | . | . | . | . | . | . | . | . | . | . | . | . | . | . | . | . |
| 21-225_31C2_LC | . | . | . | . | . | . | . | . | . | . | . | . | . | . | . | . | . | . | . | . | . | . | . | . | . | . | . | . |
| 21-225_31D12_LC2_LC | . | . | N | . | . | . | . | . | . | . | . | . | . | . | . | . | . | . | . | . | . | . | . | . | . | . | . | . |
| 21-225_32B11_LC | . | . | N | . | . | . | . | . | . | . | . | . | . | . | . | . | . | . | . | . | . | . | . | . | . | . | . | . |

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | | | | | | | | | | | | | | L_CDR3 | | | | | | | | | | | | | |
| CONSENSUS | W | D | S | - | - | - | - | - | - | - | - | - | - | - | - | - | - | - | - | - | - | - | - | - | - | - | S |
| 21-225_33A1_LC | . | . | . | . | . | . | . | . | . | . | . | . | . | . | . | . | . | . | . | . | . | . | . | . | . | . | T |
| 21-225_33E6_LC | . | . | N | . | . | . | . | . | . | . | . | . | . | . | . | . | . | . | . | . | . | . | . | . | . | . | . |
| 21-225_34B11_LC | . | . | N | . | . | . | . | . | . | . | . | . | . | . | . | . | . | . | . | . | . | . | . | . | . | . | . |
| 21-225_34B9_LC | . | . | - | . | . | . | . | . | . | . | . | . | . | . | . | . | . | . | . | . | . | . | . | . | . | . | . |
| 21-225_34D7_LC | . | . | N | . | . | . | . | . | . | . | . | . | . | . | . | . | . | . | . | . | . | . | . | . | . | . | T |
| 21-225_35E1_LC | . | . | N | . | . | . | . | . | . | . | . | . | . | . | . | . | . | . | . | . | . | . | . | . | . | . | . |
| 21-225_3B12_LC | . | V | N | . | . | . | . | . | . | . | . | . | . | . | . | . | . | . | . | . | . | . | . | . | . | . | N |
| 21-225_3H10_LC | . | V | N | . | . | . | . | . | . | . | . | . | . | . | . | . | . | . | . | . | . | . | . | . | . | . | N |
| 21-225_45F6_LC | . | . | N | . | . | . | . | . | . | . | . | . | . | . | . | . | . | . | . | . | . | . | . | . | . | . | N |
| 21-225_4B3_LC | . | . | N | . | . | . | . | . | . | . | . | . | . | . | . | . | . | . | . | . | . | . | . | . | . | . | N |
| 21-225_50G9_LC | . | N | . | . | . | . | . | . | . | . | . | . | . | . | . | . | . | . | . | . | . | . | . | . | . | . | N |
| 21-225_52H1_LC | . | . | N | . | . | . | . | . | . | . | . | . | . | . | . | . | . | . | . | . | . | . | . | . | . | . | . |
| 21-225_53D10_LC | . | . | . | . | . | . | . | . | . | . | . | . | . | . | . | . | . | . | . | . | . | . | . | . | . | . | . |
| 21-225_57D9_LC | . | . | . | . | . | . | . | . | . | . | . | . | . | . | . | . | . | . | . | . | . | . | . | . | . | . | . |
| 21-225_57F1_LC | . | . | . | . | . | . | . | . | . | . | . | . | . | . | . | . | . | . | . | . | . | . | . | . | . | . | . |
| 21-225_57H11_LC | . | . | . | . | . | . | . | . | . | . | . | . | . | . | . | . | . | . | . | . | . | . | . | . | . | . | . |
| 21-225_58C1_LC | . | . | - | . | . | . | . | . | . | . | . | . | . | . | . | . | . | . | . | . | . | . | . | . | . | . | . |
| 21-225_58E7_LC | N | N | . | . | . | . | . | . | . | . | . | . | . | . | . | . | . | . | . | . | . | . | . | . | . | . | Y |
| 21-225_58F7_LC | . | . | . | . | . | . | . | . | . | . | . | . | . | . | . | . | . | . | . | . | . | . | . | . | . | . | . |
| 21-225_58F8_LC | . | . | . | . | . | . | . | . | . | . | . | . | . | . | . | . | . | . | . | . | . | . | . | . | . | . | . |
| 21-225_58G11_LC | N | N | . | . | . | . | . | . | . | . | . | . | . | . | . | . | . | . | . | . | . | . | . | . | . | . | N |
| 21-225_59B11_LC | . | . | . | . | . | . | . | . | . | . | . | . | . | . | . | . | . | . | . | . | . | . | . | . | . | S | T |
| 21-225_59F2_LC | . | . | N | . | . | . | . | . | . | . | . | . | . | . | . | . | . | . | . | . | . | . | . | . | . | . | N |
| 21-225_5G7_LC | . | . | N | . | . | . | . | . | . | . | . | . | . | . | . | . | . | . | . | . | . | . | . | . | . | . | . |
| 21-225_60A12_LC | . | . | - | . | . | . | . | . | . | . | . | . | . | . | . | . | . | . | . | . | . | . | . | . | . | . | . |
| 21-225_60B1_LC | . | . | . | . | . | . | . | . | . | . | . | . | . | . | . | . | . | . | . | . | . | . | . | . | . | . | . |
| 21-225_60D2_LC | . | . | N | . | . | . | . | . | . | . | . | . | . | . | . | . | . | . | . | . | . | . | . | . | . | . | N |
| 21-225_61F5_LC | . | . | . | . | . | . | . | . | . | . | . | . | . | . | . | . | . | . | . | . | . | . | . | . | . | . | . |
| 21-225_62A12_LC | . | . | . | . | . | . | . | . | . | . | . | . | . | . | . | . | . | . | . | . | . | . | . | . | . | . | . |

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | | | | | | | | | | | | | | L_CDR3 | | | | | | | | | | | | | |
| CONSENSUS | W | D | S | . | . | . | . | . | . | . | . | . | . | . | . | . | . | . | . | . | . | . | . | . | . | . | S |
| 21-225_62B12_LC | . | . | . | . | . | . | . | . | . | . | . | . | . | . | . | . | . | . | . | . | . | . | . | . | . | . | . |
| 21-225_62D10_LC | . | . | . | . | . | . | . | . | . | . | . | . | . | . | . | . | . | . | . | . | . | . | . | . | . | . | . |
| 21-225_62E3_LC | . | . | . | . | . | . | . | . | . | . | . | . | . | . | . | . | . | . | . | . | . | . | . | . | . | . | . |
| 21-225_62E8_LC | . | . | . | . | . | . | . | . | . | . | . | . | . | . | . | . | . | . | . | . | . | . | . | . | . | . | . |
| 21-225_65E9_LC | . | N | . | . | . | . | . | . | . | . | . | . | . | . | . | . | . | . | . | . | . | . | . | . | . | . | . |
| 21-225_66G9_LC | . | ? | . | . | . | . | . | . | . | . | . | . | . | . | . | . | . | . | . | . | . | . | . | . | . | . | N |
| 21-225_67F10_LC | . | N | . | . | . | . | . | . | . | . | . | . | . | . | . | . | . | . | . | . | . | . | . | . | . | . | . |
| 21-225_68D8_LC | . | N | . | . | . | . | . | . | . | . | . | . | . | . | . | . | . | . | . | . | . | . | . | . | . | . | . |
| 21-225_68G6_LC | . | N | . | . | . | . | . | . | . | . | . | . | . | . | . | . | . | . | . | . | . | . | . | . | . | . | . |
| 21-225_69B11_LC | . | N | . | . | . | . | . | . | . | . | . | . | . | . | . | . | . | . | . | . | . | . | . | . | . | . | . |
| 21-225_89B9_LC | . | N | . | . | . | . | . | . | . | . | . | . | . | . | . | . | . | . | . | . | . | . | . | . | . | . | . |
| 21-225_6B4_LC | . | N | . | . | . | . | . | . | . | . | . | . | . | . | . | . | . | . | . | . | . | . | . | . | . | . | N |
| 21-225_6D9_LC | . | - | . | . | . | . | . | . | . | . | . | . | . | . | . | . | . | . | . | . | . | . | . | . | . | . | . |
| 21-225_71D4_LC | V | N | . | . | . | . | . | . | . | . | . | . | . | . | . | . | . | . | . | . | . | . | . | . | . | . | . |
| 21-225_72B4_LC | . | N | . | . | . | . | . | . | . | . | . | . | . | . | . | . | . | . | . | . | . | . | . | . | . | . | . |
| 21-225_72D5_LC | . | . | . | . | . | . | . | . | . | . | . | . | . | . | . | . | . | . | . | . | . | . | . | . | . | . | . |
| 21-225_73C4_LC | . | . | . | . | . | . | . | . | . | . | . | . | . | . | . | . | . | . | . | . | . | . | . | . | . | . | . |
| 21-225_74A9_LC | . | . | . | . | . | . | . | . | . | . | . | . | . | . | . | . | . | . | . | . | . | . | . | . | . | . | . |
| 21-225_74B3_LC | . | . | . | . | . | . | . | . | . | . | . | . | . | . | . | . | . | . | . | . | . | . | . | . | . | . | T |
| 21-225_74C9_LC | . | . | . | . | . | . | . | . | . | . | . | . | . | . | . | . | . | . | . | . | . | . | . | . | . | . | T |
| 21-225_76B4_LC | . | . | - | . | . | . | . | . | . | . | . | . | . | . | . | . | . | . | . | . | . | . | . | . | . | . | . |
| 21-225_77A2_LC | . | . | . | . | . | . | . | . | . | . | . | . | . | . | . | . | . | . | . | . | . | . | . | . | . | . | . |
| 21-225_78E9_LC | . | . | . | . | . | . | . | . | . | . | . | . | . | . | . | . | . | . | . | . | . | . | . | . | . | . | . |
| 21-225_79E7_LC | . | . | . | . | . | . | . | . | . | . | . | . | . | . | . | . | . | . | . | . | . | . | . | . | . | . | . |
| 21-225_8D8_LC | . | . | . | . | . | . | . | . | . | . | . | . | . | . | . | . | . | . | . | . | . | . | . | . | . | . | . |
| 21-225_92A4_LC | . | . | . | . | . | . | . | . | . | . | . | . | . | . | . | . | . | . | . | . | . | . | . | . | . | . | . |
| 21-225_96B6_LC | . | . | - | . | . | . | . | . | . | . | . | . | . | . | . | . | . | . | . | . | . | . | . | . | . | . | . |
| 21-225_97E6_LC | . | . | . | . | . | . | . | . | . | . | . | . | . | . | . | . | . | . | . | . | . | . | . | . | . | . | T |
| 21-225_9H10_LC | . | . | . | . | . | . | . | . | . | . | . | . | . | . | . | . | . | . | . | . | . | . | . | . | . | S | T |

4383

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | | | | | L_FR4 | | | | | | |
| CONSENSUS | T | V | V | F | G | G | G | T | K | L | T | V | L | G |
| 21-225_10B10_LC | . | . | . | . | . | . | . | . | . | . | . | . | . | . |
| 21-225_12B1_LC | . | . | S | . | . | . | . | . | . | . | . | . | . | . |
| 21-225_13F6_LC | A | . | . | . | . | . | . | . | . | . | . | . | . | . |
| 21-225_146A10_LC | . | . | . | . | . | . | . | . | . | V | . | . | . | . |
| 21-225_146A2_LC | . | . | . | . | . | . | . | . | . | . | . | . | . | . |
| 21-225_146A3_LC | . | . | . | . | . | . | . | . | . | . | . | . | . | . |
| 21-225_146B11_LC | . | . | . | . | . | . | . | . | . | . | . | . | . | . |
| 21-225_146B8_LC | . | . | . | . | . | . | . | . | . | . | . | . | . | . |
| 21-225_146C11_LC | . | . | . | . | . | . | . | . | . | . | . | . | . | . |
| 21-225_146H8_LC | . | . | . | . | . | . | . | . | . | . | . | . | . | . |
| 21-225_147B12_LC | . | . | . | . | . | . | . | . | . | . | . | . | . | . |
| 21-225_147B8_LC | A | . | . | . | . | . | . | . | . | . | . | . | . | . |
| 21-225_147B9_LC | F | . | . | . | . | . | . | . | . | . | . | . | . | . |
| 21-225_147D9_LC | Y | . | . | . | . | . | . | . | . | . | . | . | . | . |
| 21-225_147E11_LC | . | . | . | . | . | . | . | . | . | . | . | . | . | . |
| 21-225_147E7_LC | . | . | . | . | . | . | . | . | . | . | . | . | . | . |
| 21-225_147F9_LC | . | . | . | . | . | . | . | . | . | . | . | . | . | . |
| 21-225_147G9_LC | . | . | . | . | . | . | . | . | . | . | . | . | . | . |
| 21-225_148A9_LC | . | . | . | . | . | . | . | . | . | . | . | . | . | . |
| 21-225_148C8_LC | F | . | . | . | . | . | . | . | . | . | . | . | . | . |
| 21-225_148E10_LC | Y | . | . | . | . | . | . | . | . | . | . | . | . | . |
| 21-225_148G11_LC | . | . | . | . | . | . | . | . | . | . | . | . | . | . |
| 21-225_148G6_LC | . | . | . | . | . | . | . | . | . | . | . | . | . | . |
| 21-225_149A11_LC | S | . | . | . | . | . | . | . | . | . | . | . | . | . |
| 21-225_149B5_LC | . | . | . | . | . | . | . | . | . | . | . | . | . | . |
| 21-225_149C10_LC | . | . | . | . | . | . | . | . | . | . | . | . | . | . |
| 21-225_149C7_LC | . | . | . | . | . | . | . | . | . | . | . | . | S | . |
| 21-225_14B1_LC2_LC | . | . | . | . | . | . | . | . | . | . | . | . | . | . |
| 21-225_14B7_LC | . | . | . | . | . | . | . | . | . | . | . | . | . | S |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | | | | L_FR4 | | | | | | | |
| CONSENSUS | T | V | V | F | G | G | G | T | K | L | T | V | L | G |
| 21-225_14G3_LC | A | . | . | | . | . | . | . | . | . | . | . | . | . |
| 21-225_14H11_LC | S | T | I | . | . | . | . | . | . | . | . | . | . | . |
| 21-225_150D3_LC | . | . | . | | . | . | . | . | . | . | . | . | . | . |
| 21-225_150F6_LC | . | . | . | | . | . | . | . | . | . | . | . | . | . |
| 21-225_150H11_LC | . | . | . | | . | . | . | . | . | . | . | . | . | . |
| 21-225_151B9_LC | . | . | . | | . | . | . | . | . | . | . | . | . | . |
| 21-225_151F3_LC | . | . | . | | . | . | . | . | . | . | . | . | . | . |
| 21-225_151H5_LC | . | . | . | | . | . | . | . | . | . | . | . | . | . |
| 21-225_151H7_LC | . | . | . | | . | . | . | . | . | . | . | . | . | . |
| 21-225_152D7_LC | . | . | . | | . | . | . | . | . | . | . | . | . | . |
| 21-225_152G6_LC | . | . | . | | . | . | . | . | . | . | . | . | . | . |
| 21-225_153D9_LC | . | . | . | | . | . | . | . | . | V | . | . | . | . |
| 21-225_153E8_LC | Y | . | I | | . | . | . | . | . | . | . | . | . | . |
| 21-225_154C3_LC | . | . | . | | . | . | . | . | . | . | . | . | . | . |
| 21-225_154C4_LC | . | . | . | | . | . | . | . | . | . | . | . | . | . |
| 21-225_154D11_LC | I | . | . | | . | . | . | . | . | . | . | . | . | . |
| 21-225_154E10_LC | . | . | . | | . | . | . | . | . | . | . | . | . | . |
| 21-225_154F4_LC | . | L | . | | . | . | . | . | . | . | . | . | . | . |
| 21-225_155C10_LC | . | . | . | | . | . | . | . | . | . | . | . | . | . |
| 21-225_155E10_LC | F | . | . | | . | . | . | . | . | . | . | . | . | . |
| 21-225_158D10_LC | F | . | . | | . | . | . | . | . | . | . | . | . | . |
| 21-225_158E9_LC | F | . | L | | . | . | . | . | . | . | . | . | . | . |
| 21-225_159H8_LC | F | . | . | | . | . | . | . | . | . | . | . | . | . |
| 21-225_15H11_LC | . | A | . | | . | . | . | . | . | . | . | . | . | . |
| 21-225_160B12_LC | F | . | . | | . | . | . | . | . | . | . | . | . | . |
| 21-225_161E10_LC | F | A | L | | . | . | . | . | . | . | . | . | . | . |
| 21-225_161F12_LC | . | L | . | | . | . | . | . | . | . | . | . | . | . |
| 21-225_161H3_LC | A | . | . | | . | . | . | . | . | . | . | . | . | . |
| 21-225_165H3_LC | . | L | . | | . | . | . | . | . | . | . | . | . | . |

**FIGURE 57**

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | | | | | L_FR4 | | | | | | |
| CONSENSUS | T | V | V | F | G | G | G | T | K | L | T | V | L | G |
| 21-225_166G11_LC | . | A | . | . | . | . | . | . | . | . | . | . | . | . |
| 21-225_169A6_LC | . | . | L | . | . | . | . | . | . | . | . | . | . | . |
| 21-225_169B7_LC | . | . | . | . | . | . | . | . | . | . | . | . | . | . |
| 21-225_169C1_LC | . | . | I | . | . | . | . | . | . | . | . | . | . | . |
| 21-225_169G11_LC | . | A | . | . | . | . | . | . | . | . | . | . | . | . |
| 21-225_16D2_LC | Y | . | . | . | . | . | . | . | . | . | . | . | . | . |
| 21-225_16E4_LC | . | . | . | . | . | . | . | . | . | . | . | . | . | . |
| 21-225_16G8_LC | . | M | I | . | . | . | . | . | . | . | . | . | . | . |
| 21-225_170A6_LC | . | M | . | . | . | . | . | . | R | . | . | . | . | . |
| 21-225_170F1_LC | . | A | . | . | . | . | . | . | . | . | . | . | . | . |
| 21-225_171E12_LC | Y | . | . | . | . | . | . | . | . | . | . | . | . | . |
| 21-225_171F5_LC | . | . | . | . | . | . | . | . | . | . | . | . | . | . |
| 21-225_173F8_LC | . | . | . | . | . | . | . | . | . | . | . | . | . | . |
| 21-225_175C6_LC | . | M | . | . | . | . | . | . | R | . | . | . | . | . |
| 21-225_179C7_LC | A | . | . | . | . | . | . | . | . | . | . | . | . | . |
| 21-225_17D3_LC | . | A | . | . | . | . | . | . | . | . | . | R | . | . |
| 21-225_17F5_LC | S | T | . | . | . | . | . | . | . | . | . | . | . | . |
| 21-225_180C5_LC | . | A | . | . | . | . | . | . | . | . | . | . | . | . |
| 21-225_180D4_LC | X | . | . | . | . | . | . | . | . | . | . | . | . | . |
| 21-225_180G5_LC | . | A | . | . | . | . | . | . | . | . | . | . | . | . |
| 21-225_181H1_LC | X | . | . | . | . | . | . | . | . | . | . | . | . | . |
| 21-225_182D12_LC | . | A | . | . | . | . | . | . | . | . | . | . | . | . |
| 21-225_183F4_LC | A | . | . | . | . | . | . | . | . | . | . | . | . | S |
| 21-225_185D2_LC | X | . | . | . | . | . | . | . | . | . | . | . | . | . |
| 21-225_185G7_LC | S | T | . | . | . | . | . | . | . | . | . | . | . | . |
| 21-225_186H6_LC | . | A | . | . | . | . | . | . | . | . | . | . | . | . |
| 21-225_18E10_LC | . | . | . | . | . | . | . | . | . | . | . | . | . | . |
| 21-225_18G6_LC | N | . | . | . | . | . | . | . | . | . | . | . | . | . |
| 21-225_190G9_LC | . | . | . | . | . | . | . | . | R | . | . | . | . | R |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | | | | L_FR4 | | | | | | | |
| CONSENSUS | T | V | V | F | G | G | G | T | K | L | T | V | L | G |
| 21-225_190H1_LC | . | . | . | . | . | . | . | . | . | . | . | . | . | . |
| 21-225_193H5_LC | . | A | . | . | . | . | . | . | . | . | . | . | . | . |
| 21-225_195E3_LC | . | . | . | . | . | . | . | . | . | . | I | . | . | . |
| 21-225_195E9_LC | I | . | . | . | . | . | . | . | . | . | . | . | . | . |
| 21-225_19F6_LC | . | . | . | . | . | . | . | . | . | . | . | . | . | . |
| 21-225_201G11_LC | . | . | . | . | . | . | . | . | . | . | . | . | . | . |
| 21-225_203G6_LC | . | . | . | . | . | . | . | . | . | . | . | . | . | . |
| 21-225_215B3_LC | . | . | . | . | . | . | . | . | . | . | . | . | . | . |
| 21-225_21D12_LC | . | . | . | . | . | . | . | . | X | . | . | . | . | . |
| 21-225_224H2_LC | . | . | . | . | . | . | . | . | . | . | . | . | . | . |
| 21-225_225E9_LC | A | . | . | . | . | . | . | . | . | . | . | . | . | . |
| 21-225_226A10_LC | A | A | . | . | . | . | . | . | . | . | . | . | . | . |
| 21-225_226B2_LC | A | A | . | . | . | . | . | . | . | . | . | . | . | . |
| 21-225_227E12_LC | N | . | . | . | . | . | . | . | . | . | . | . | . | . |
| 21-225_227E5_LC | . | M | I | . | . | . | . | . | . | . | . | . | . | . |
| 21-225_22E6_LC | . | A | . | . | . | . | . | . | . | . | . | . | . | . |
| 21-225_23D7_LC | N | . | . | . | . | . | . | . | . | . | . | . | . | S |
| 21-225_26C10_LC | N | T | . | . | . | . | . | . | . | . | . | . | . | . |
| 21-225_27D9_LC | N | T | . | . | . | . | . | . | . | . | . | . | . | . |
| 21-225_27E7_LC | . | . | . | . | . | . | . | . | . | . | . | . | . | . |
| 21-225_27G6_LC | Y | . | . | . | . | . | . | . | . | . | . | . | . | . |
| 21-225_27H2_LC | Y | . | . | . | . | . | . | . | . | . | . | . | . | . |
| 21-225_28A11_LC | Y | . | . | . | . | . | . | . | . | . | . | . | . | . |
| 21-225_2B1_LC | . | . | . | . | . | . | . | . | . | . | . | . | . | . |
| 21-225_30D9_LC2_LC | . | A | . | . | . | . | . | . | . | . | . | . | . | . |
| 21-225_30H6_LC | S | T | . | . | . | . | . | . | . | . | . | . | . | . |
| 21-225_31C2_LC | S | T | . | . | . | . | . | . | . | . | . | . | . | . |
| 21-225_31D12_LC2_LC | . | A | . | . | . | . | . | . | . | . | . | . | . | . |
| 21-225_32B11_LC | . | . | . | . | . | . | . | . | . | . | . | . | . | . |

**FIGURE 57**

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | | | | | L_FR4 | | | | | | |
| CONSENSUS | T | V | V | F | G | G | G | T | K | L | T | V | L | G |
| 21-225_33A1_LC | . | . | . | . | . | . | . | . | . | . | . | . | . | . |
| 21-225_33E6_LC | . | A | . | . | . | . | . | . | . | . | . | . | . | . |
| 21-225_34B11_LC | . | A | . | . | . | . | . | . | . | . | . | . | . | . |
| 21-225_34B9_LC | S | T | . | . | . | . | . | . | . | . | . | . | . | . |
| 21-225_34D7_LC | . | . | . | . | . | . | . | . | . | . | . | . | . | . |
| 21-225_35E1_LC | . | A | . | . | . | . | . | . | . | . | . | . | . | . |
| 21-225_3B12_LC | . | M | I | . | . | . | . | . | . | . | . | . | . | . |
| 21-225_3H10_LC | . | M | I | . | . | . | . | . | . | . | . | . | . | . |
| 21-225_45F6_LC | . | M | . | . | . | . | . | . | . | . | . | . | . | . |
| 21-225_4B3_LC | . | . | I | . | . | . | . | . | . | . | . | . | . | . |
| 21-225_50G9_LC | R | G | . | . | . | . | . | . | R | . | . | . | . | . |
| 21-225_52H1_LC | . | . | . | . | . | . | . | . | . | . | . | . | . | . |
| 21-225_53D10_LC | . | . | . | . | . | . | . | . | . | . | . | . | . | . |
| 21-225_57D9_LC | . | . | . | . | . | . | . | . | . | . | . | . | . | . |
| 21-225_57F1_LC | . | . | . | . | . | . | . | . | . | . | . | . | . | . |
| 21-225_57H11_LC | . | . | . | . | . | . | . | . | . | . | . | . | . | . |
| 21-225_58C1_LC | S | T | A | . | . | . | . | . | . | . | . | . | . | . |
| 21-225_58E7_LC | . | . | . | . | . | . | . | . | . | . | . | A | . | . |
| 21-225_58F7_LC | . | . | . | . | . | . | . | . | . | . | . | . | . | . |
| 21-225_58F8_LC | . | . | . | . | . | . | . | . | . | . | . | . | . | . |
| 21-225_58G11_LC | . | . | M | . | . | . | . | . | . | . | . | . | . | . |
| 21-225_59B11_LC | Y | . | . | . | . | . | . | . | . | . | . | . | . | . |
| 21-225_59F2_LC | . | . | . | . | . | . | . | . | . | . | . | . | . | . |
| 21-225_5G7_LC | . | . | . | . | . | . | . | . | . | . | . | . | . | . |
| 21-225_60A12_LC | S | T | A | . | . | . | . | . | . | . | . | . | . | . |
| 21-225_60B1_LC | . | . | . | . | . | . | . | . | . | . | . | . | . | . |
| 21-225_60D2_LC | . | . | . | . | . | . | . | . | . | . | . | . | . | . |
| 21-225_61F5_LC | . | . | . | . | . | . | . | . | . | . | . | . | . | . |
| 21-225_62A12_LC | . | A | . | . | . | . | . | . | . | . | . | . | . | R |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | | | | L_FR4 | | | | | | | |
| CONSENSUS | T | V | V | F | G | G | G | T | K | L | T | V | L | G |
| 21-225_62B12_LC | . | A | . | . | . | . | . | . | . | . | . | . | . | . |
| 21-225_62D10_LC | . | A | . | . | . | . | . | . | . | . | . | . | . | . |
| 21-225_62E3_LC | . | A | . | . | . | . | . | . | . | . | . | . | . | . |
| 21-225_62E8_LC | . | A | . | . | . | . | . | . | . | . | . | . | . | . |
| 21-225_65E9_LC | . | . | . | . | . | . | . | . | . | . | . | . | . | . |
| 21-225_66G9_LC | . | A | . | . | . | . | . | . | . | . | . | . | . | . |
| 21-225_67F10_LC | . | . | . | . | . | . | . | . | . | . | . | . | . | . |
| 21-225_68D8_LC | . | . | . | . | . | . | . | . | . | . | . | . | . | . |
| 21-225_68G6_LC | . | A | . | . | . | . | . | . | . | . | . | . | . | . |
| 21-225_69B11_LC | . | . | . | . | . | . | . | . | . | . | . | . | . | . |
| 21-225_69B9_LC | . | . | . | . | . | . | . | . | . | . | . | . | . | . |
| 21-225_6B4_LC | . | . | I | . | . | . | . | . | . | . | . | . | . | . |
| 21-225_6D9_LC | S | T | . | . | . | . | . | . | . | . | . | . | . | . |
| 21-225_71D4_LC | . | . | . | . | . | . | . | . | . | . | . | . | . | . |
| 21-225_72B4_LC | . | . | . | . | . | . | . | . | . | . | . | . | . | . |
| 21-225_72D5_LC | . | A | . | . | . | . | . | . | . | . | . | . | . | . |
| 21-225_73C4_LC | . | A | . | . | . | . | . | . | . | . | . | . | . | . |
| 21-225_74A9_LC | P | . | I | . | . | . | . | . | . | . | . | . | . | . |
| 21-225_74B3_LC | . | . | . | . | . | . | . | . | . | . | . | . | . | . |
| 21-225_74C9_LC | P | . | I | . | . | . | . | . | . | . | . | . | . | . |
| 21-225_76B4_LC | S | T | . | . | . | . | . | . | . | . | . | . | . | . |
| 21-225_77A2_LC | . | A | . | . | . | . | . | . | . | . | . | . | . | . |
| 21-225_78E9_LC | P | . | I | . | . | . | . | . | . | . | . | . | . | . |
| 21-225_79E7_LC | P | . | I | . | . | . | . | . | . | . | . | . | . | . |
| 21-225_8D8_LC | . | A | . | . | . | . | . | . | . | . | . | . | . | S |
| 21-225_92A4_LC | P | . | I | . | . | . | . | . | . | . | . | . | . | S |
| 21-225_96B5_LC | S | T | . | . | . | . | . | . | . | . | . | . | . | . |
| 21-225_97E6_LC | P | . | I | . | . | . | . | . | . | . | . | . | . | . |
| 21-225_9H10_LC | Y | . | . | . | . | . | . | . | . | . | . | . | . | . |

**FIGURE 57**

Table 97.    Consensus 17 - VK3|A27/JK1 (SEQ ID NO: 50314):

EIVLTQSPGTLSLSPGERATLSC<u>RAS</u>--<u>QSVYS</u>-----<u>SYLA</u>WYQQKPGQAPRLLIY<u>G</u>-------<u>ASSRA</u>TGIPDRFSGSGSG--
TDFTLTISRLEPEDFAVYYC<u>QQYDN</u>---------------------<u>SPWT</u>FGQGTKVEIKR wherein:

R at position 24 can be substituted with W

A at position 25 can be substituted with T

S at position 26 can be substituted with G or R

Q at position 29 can be substituted with P

S at position 30 can be substituted with N, I or R

V at position 31 can be substituted with I or F

Y at position 32 can be substituted with R, S, N, D, F, H, G or W

S at position33 can be substituted with T, N, G, L or R

S at position 39 can be substituted with N, G, R, D, A or Y

Y at position 40 can be substituted with F or H

A at position 42 can be substituted with V or S

G at position 58 can be substituted with D or V

A at position 67 can be substituted with T, P or V

S at position 68 can be substituted with F, Y, A or T

S at position 69 can be substituted with R, N or A

A at position 71 can be substituted with S or T

T at position 72 can be substituted with P, S or A

FIGURE 57

Q at position 107 can be substituted with H

Q at position 108 can be substituted with H

Y at position 109 can be substituted with S

D at position 110 can be substituted with E, G or H

N at position 111 can be substituted with S, null (-), R, T, I, D or G

null (-) at position 134 can be substituted with S

S at position 135 can be substituted with P, N or R

P at position 136 can be substituted with S or V

W at position 137 can be substituted with R

T at position 138 can be substituted with A

Xaa Xaa Xaa Xaa Xaa Xaa Xaa Xaa Xaa Xaa Leu Xaa (SEQ ID NO: 50127)

Xaa Xaa Xaa Xaa Arg Xaa Xaa (SEQ ID NO: 50128)

Xaa Xaa Xaa Xaa Xaa Xaa Xaa Xaa Xaa Xaa (SEQ ID NO: 50129)

Arg Ala Ser Gln Ser Val Tyr Ser Ser Tyr Leu Ala (SEQ ID NO: 50612)

Gly Ala Ser Ser Arg Ala Thr (SEQ ID NO: 50613)

Gln Gln Tyr Asp Asn Ser Pro Trp Thr (SEQ ID NO: 50614)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | E | I | V | L | T | Q | S | P | G | T | L | S | L | S | P | G | E | R | A | T | L | S | C | R | A | S |
| 21-225_147G8_LC | Q | . | . | . | . | . | . | . | . | . | . | . | . | . | . | . | . | . | . | . | . | . | . | . | . | . |
| 21-225_190A3_LC | . | . | M | . | . | . | . | . | . | . | . | . | . | . | . | . | . | . | . | . | . | . | . | . | . | . |
| 21-225_190A7_LC | . | . | . | . | . | . | . | . | . | . | . | . | . | . | . | . | . | . | . | . | . | . | . | . | . | . |
| 21-225_190B4_LC | . | . | . | . | . | . | . | . | . | . | . | . | . | . | . | . | . | . | . | . | . | . | . | . | . | . |
| 21-225_190G10_LC | . | . | . | . | . | . | . | . | . | . | . | . | . | . | . | . | . | . | . | . | . | . | . | . | . | . |
| 21-225_191A3_LC | . | . | . | . | . | . | . | . | . | . | . | . | . | . | . | . | . | . | . | . | . | . | . | . | . | . |
| 21-225_191A5_LC | . | . | . | . | . | . | . | . | . | . | . | . | . | . | . | . | . | . | . | . | . | . | . | . | . | . |
| 21-225_191C3_LC | . | . | . | . | . | . | . | . | . | . | . | . | . | . | . | . | . | . | . | . | . | . | . | . | . | . |
| 21-225_191D3_LC | . | . | . | . | . | . | . | . | . | . | . | . | . | . | . | . | . | S | . | . | . | . | . | . | . | G |
| 21-225_191F1_LC | . | . | . | . | . | . | . | . | . | . | . | . | . | . | . | . | . | . | . | A | . | . | . | . | . | . |
| 21-225_191H7_LC | . | . | . | . | . | . | . | . | . | . | . | . | . | . | . | . | . | S | . | . | . | . | . | . | . | G |
| 21-225_192B3_LC | . | . | . | . | . | . | . | . | . | . | . | . | . | . | . | . | . | . | . | . | . | . | . | . | . | . |
| 21-225_192F8_LC | . | . | . | . | . | . | . | . | . | . | . | . | . | . | . | . | . | . | . | . | . | . | . | . | . | . |
| 21-225_192G12_LC | . | . | . | . | . | . | . | . | . | . | . | . | . | . | . | . | . | . | . | . | . | . | . | . | . | . |
| 21-225_192H3_LC | . | . | . | . | . | . | . | . | . | . | . | . | . | . | . | . | . | . | . | . | . | . | . | . | . | . |
| 21-225_193A1_LC | . | . | . | . | . | . | . | . | . | . | . | . | . | . | . | . | . | . | . | . | . | . | . | . | . | . |
| 21-225_193B1_LC | . | . | . | . | . | . | . | . | . | . | . | . | . | . | . | . | D | . | . | . | . | . | . | . | . | . |
| 21-225_193B10_LC | . | . | . | . | . | . | . | . | . | . | . | . | . | . | . | . | . | . | . | . | . | . | . | . | . | . |
| 21-225_193B12_LC | . | . | . | . | . | . | . | . | . | . | . | . | . | . | . | . | . | . | . | . | . | . | . | . | . | . |
| 21-225_193C8_LC | . | . | . | . | . | . | . | . | . | . | . | S | . | . | . | . | . | . | . | . | . | . | . | . | . | . |
| 21-225_193D3_LC | . | . | . | . | . | . | . | . | . | . | . | . | . | . | . | . | . | . | . | . | . | . | . | . | . | . |
| 21-225_193D8_LC | . | . | . | K | . | . | . | . | . | . | . | S | . | . | . | . | . | . | . | . | . | . | . | . | . | . |
| 21-225_193E6_LC | . | . | . | A | . | . | . | . | . | . | . | . | . | . | . | . | . | . | . | . | . | . | . | . | . | . |
| 21-225_193F3_LC | . | . | . | . | . | . | . | . | . | . | . | . | . | . | . | . | . | S | . | . | . | . | . | . | . | G |
| 21-225_193G8_LC | . | . | . | . | . | . | . | . | . | . | . | . | . | . | . | . | . | . | . | . | . | . | . | . | . | . |
| 21-225_193H6_LC | . | . | . | . | . | . | . | . | . | . | . | . | . | . | . | . | . | S | . | . | . | . | . | . | . | G |
| 21-225_194C10_LC | . | . | . | . | . | . | . | . | . | . | . | . | . | . | . | . | . | . | . | . | . | . | . | . | . | . |
| 21-225_194E5_LC | . | . | . | . | . | . | . | . | . | . | . | . | . | . | . | . | . | S | . | . | . | . | . | . | . | G |
| 21-225_194E6_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | E | I | V | L | T | Q | S | P | G | T | L | S | L | S | P | G | E | R | A | T | L | S | C | R | A | S |
| 21-225_195B1_LC | . | . | . | . | . | . | . | . | . | . | . | . | . | . | S | . | . | . | . | . | . | . | . | . | . | . |
| 21-225_196C8_LC | . | . | . | . | . | . | . | . | . | . | . | . | . | . | . | . | . | . | . | . | . | . | . | . | . | . |
| 21-225_196G10_LC | . | . | . | . | . | . | . | . | . | . | . | L | . | . | . | . | . | . | . | . | . | . | . | . | . | . |
| 21-225_196H12_LC | . | . | . | . | . | . | . | . | . | . | . | P | . | . | . | . | . | . | . | . | . | . | . | . | . | . |
| 21-225_197F4_LC | . | . | . | . | . | . | . | . | . | . | . | . | . | . | . | . | . | G | . | . | . | . | . | . | . | . |
| 21-225_198B1_LC | . | . | . | . | . | . | . | . | . | . | . | . | . | . | . | . | . | S | . | . | . | . | . | . | . | G |
| 21-225_198C2_LC | . | . | V | . | . | . | . | . | . | . | . | L | . | Y | S | E | . | . | S | . | . | . | . | . | . | . |
| 21-225_198E1_LC | . | . | . | . | . | . | . | . | . | . | . | . | . | . | . | . | . | . | . | . | . | . | . | . | . | . |
| 21-225_198G10_LC | . | . | . | . | . | . | . | . | . | . | . | . | . | . | . | . | . | . | . | . | . | . | . | . | . | . |
| 21-225_199C2_LC | . | . | . | . | . | . | . | . | . | . | . | . | . | . | . | . | . | . | . | . | . | . | . | . | . | . |
| 21-225_199C7_LC | . | . | . | . | . | . | . | . | . | . | . | . | . | . | . | . | . | . | . | . | . | . | . | . | . | . |
| 21-225_199G11_LC | . | . | V | . | . | . | . | . | . | . | . | . | . | . | . | . | . | . | . | . | . | . | . | . | . | . |
| 21-225_200A1_LC | . | . | . | . | . | . | . | . | I | . | . | . | . | Y | . | . | . | . | . | . | . | . | . | . | . | . |
| 21-225_200F10_LC | . | . | . | . | . | . | . | . | . | . | . | . | . | . | . | . | . | . | . | . | . | . | . | . | . | . |
| 21-225_201B2_LC | . | . | . | . | . | . | . | . | . | . | . | . | . | . | . | . | . | . | . | . | . | . | . | . | . | . |
| 21-225_201E1_LC | . | . | . | . | . | . | . | G | . | . | . | . | . | . | . | . | . | . | . | . | . | . | . | . | . | . |
| 21-225_202D11_LC | . | . | . | . | . | . | . | . | . | . | . | . | . | . | . | . | . | . | . | . | . | . | . | . | . | . |
| 21-225_202D9_LC | . | . | . | . | . | . | . | G | . | . | . | . | . | . | . | . | . | . | . | . | . | . | . | . | . | . |
| 21-225_205G7_LC | . | . | . | . | . | . | . | . | . | . | . | . | . | . | . | . | . | . | . | . | . | . | . | . | . | . |
| 21-225_208A9_LC | . | . | . | . | . | . | . | . | . | . | . | . | . | . | . | . | . | . | . | . | . | . | . | . | . | . |
| 21-225_208B5_LC | . | . | . | . | . | . | . | . | . | . | . | . | . | . | . | . | . | . | . | . | . | . | . | . | . | . |
| 21-225_220E1_LC | . | . | . | . | . | . | . | . | . | . | . | . | . | . | . | . | . | . | . | . | . | . | . | . | . | . |
| 21-225_222C7_LC | . | . | . | . | . | . | . | . | . | . | . | . | . | . | . | . | . | . | . | . | . | . | . | . | . | . |
| 21-225_225E7_LC | . | . | . | . | . | . | . | . | . | . | . | . | . | . | . | . | . | . | . | . | . | . | . | . | . | . |
| 21-225_23B9_LC | . | . | . | . | . | . | . | . | . | . | . | . | . | . | . | . | . | . | . | . | . | . | . | . | . | . |
| 21-225_44F6_LC | . | . | . | . | . | . | . | . | . | . | . | . | . | . | . | . | . | . | . | . | . | . | . | . | . | . |
| 21-225_59B2_LC | . | . | . | . | . | . | . | . | . | . | . | . | . | . | . | . | . | . | . | . | . | . | . | W | . | . |
| 21-225_74A3_LC | . | . | . | . | . | . | . | . | . | . | . | . | . | . | . | . | . | . | . | . | . | . | . | . | . | . |
| 21-225_74A4_LC | . | . | . | . | . | . | . | . | . | . | . | Y | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | E | I | V | L | T | Q | S | P | G | T | L | S | L | S | P | G | E | R | A | T | L | S | C | R | A | S |
| 21-225_74B12_LC | . | . | . | . | . | . | . | . | . | . | . | Y | . | . | . | . | . | . | . | . | . | . | . | . | . | . |
| 21-225_74B2_LC | . | L | . | . | . | . | . | . | . | . | . | . | . | . | . | . | . | . | . | . | . | . | . | . | . | . |
| 21-225_74C5_LC | . | . | . | . | . | . | . | . | . | . | . | . | . | . | . | . | . | . | . | . | . | . | . | . | . | . |
| 21-225_74C7_LC | . | . | . | . | . | . | . | . | . | . | . | . | . | . | . | . | . | . | . | . | . | . | . | . | T | . |
| 21-225_74D11_LC | . | . | . | . | . | . | . | . | . | . | . | . | . | . | . | . | . | . | . | . | . | . | . | . | . | . |
| 21-225_74D2_LC | . | . | . | . | . | . | . | . | . | . | . | . | . | . | . | . | . | . | . | . | . | . | . | . | . | . |
| 21-225_74D5_LC | . | . | . | . | . | . | . | . | . | . | . | . | . | . | . | . | . | . | . | . | . | . | . | . | . | . |
| 21-225_74D6_LC | . | . | . | . | . | . | . | . | . | . | . | . | . | . | . | . | . | . | . | . | . | . | . | . | . | . |
| 21-225_74F4_LC | . | . | . | . | . | . | . | . | . | . | . | . | . | . | . | . | . | . | . | . | . | . | . | . | . | . |
| 21-225_74F5_LC | . | . | . | . | . | . | . | . | . | . | . | Y | . | . | . | . | . | . | . | . | . | . | . | . | . | . |
| 21-225_74G8_LC | . | . | . | . | . | . | . | . | . | . | . | . | . | . | . | . | . | . | . | . | . | . | . | . | . | . |
| 21-225_74H5_LC | . | . | . | . | . | . | . | . | . | . | . | . | . | . | . | . | . | . | . | . | . | . | . | . | . | . |
| 21-225_74H7_LC | . | L | . | . | . | . | . | . | . | . | . | . | . | . | . | . | . | . | . | . | . | . | . | . | . | . |
| 21-225_74H8_LC | . | . | . | . | . | . | . | . | . | . | . | . | . | . | . | . | . | . | . | . | . | . | . | . | . | . |
| 21-225_75A12_LC | . | . | . | . | . | . | . | . | . | . | . | . | . | . | . | . | . | . | . | . | . | . | . | . | . | . |
| 21-225_75A5_LC | . | . | . | . | . | . | . | . | . | . | . | Y | . | . | . | . | . | . | . | . | . | . | . | . | . | . |
| 21-225_75A8_LC | . | . | . | . | . | . | . | . | . | . | . | Y | . | . | . | . | . | . | . | . | . | . | . | . | . | . |
| 21-225_75B10_LC | . | . | . | . | . | . | . | . | . | . | . | . | . | . | . | . | . | . | . | . | . | . | . | . | . | . |
| 21-225_75C2_LC | . | . | . | . | . | . | . | . | . | . | ? | Y | . | . | . | . | . | . | . | . | . | . | . | . | . | . |
| 21-225_75C3_LC | . | . | . | . | . | . | . | . | . | . | . | Y | . | . | Q | . | . | . | . | . | . | . | . | . | . | . |
| 21-225_75D3_LC | . | . | . | . | . | . | . | . | . | . | . | Y | . | . | . | . | . | . | . | . | . | . | . | . | . | . |
| 21-225_75D9_LC | . | . | . | . | . | . | . | . | . | . | . | . | . | . | . | . | . | . | . | . | . | . | . | . | . | . |
| 21-225_75F3_LC | . | . | . | . | . | . | . | . | . | . | . | Y | . | . | . | . | . | . | . | . | . | . | . | . | . | . |
| 21-225_75G12_LC | . | . | . | . | . | . | . | . | . | . | . | Y | . | . | . | . | . | . | . | . | . | . | . | . | . | . |
| 21-225_75G3_LC | . | . | . | . | . | . | . | . | . | . | . | . | . | . | . | . | . | . | . | . | . | . | . | . | . | X |
| 21-225_75G7_LC | . | . | . | . | . | . | . | . | . | . | . | . | . | . | . | . | . | . | . | . | . | . | . | . | . | X |
| 21-225_75G9_LC | . | . | A | . | . | . | . | . | . | . | . | . | . | . | . | . | . | . | . | . | . | . | . | . | . | . |
| 21-225_75H8_LC | . | P | . | . | . | . | . | . | . | . | . | . | . | . | . | . | . | . | . | . | . | . | . | . | . | . |
| 21-225_76A4_LC | . | . | . | . | . | . | . | . | . | . | . | Y | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | E | I | V | L | T | Q | S | P | G | T | L | S | L | S | P | G | E | R | A | T | L | S | C | R | A | S |
| 21-225_76A7_LC | . | . | A | . | . | . | . | . | . | . | . | . | . | . | . | . | . | . | . | . | . | . | . | . | . | . |
| 21-225_76C9_LC | . | . | . | . | . | . | . | . | . | . | . | . | . | . | . | . | . | . | . | . | . | . | . | . | . | . |
| 21-225_76D1_LC | . | . | . | . | . | . | . | . | . | . | . | . | . | . | . | . | . | . | . | . | . | . | . | . | . | . |
| 21-225_76E10_LC | . | . | . | . | . | . | . | . | . | . | . | . | . | . | . | . | . | . | . | . | . | . | . | . | . | . |
| 21-225_76G4_LC | . | . | . | . | . | . | . | . | . | . | . | . | . | . | . | . | . | . | . | . | . | . | . | . | . | . |
| 21-225_77A10_LC | . | . | . | . | . | . | . | . | . | . | . | . | . | . | . | . | . | . | . | . | . | . | . | . | . | . |
| 21-225_77C12_LC | . | . | . | . | . | . | . | . | . | . | . | . | . | . | . | . | . | . | . | . | . | . | . | . | . | . |
| 21-225_77C5_LC | . | . | . | . | . | . | . | . | . | . | . | . | . | . | . | . | . | . | . | . | . | . | . | . | . | . |
| 21-225_77F7_LC | . | . | . | . | . | . | . | . | . | . | . | Y | . | . | . | . | . | . | . | . | . | . | . | . | . | . |
| 21-225_77G1_LC | . | . | . | . | . | . | . | . | . | . | . | Y | . | . | . | . | . | . | . | . | . | . | . | . | . | . |
| 21-225_78E7_LC | . | . | . | . | . | . | . | . | . | . | . | . | . | . | . | . | . | . | . | . | . | . | . | . | . | . |
| 21-225_79F11_LC | . | . | . | . | . | . | . | . | . | . | . | . | . | . | . | . | . | . | . | . | . | . | . | . | . | . |
| 21-225_79F3_LC | . | . | . | . | . | . | . | . | . | . | . | Y | . | . | . | . | . | . | . | . | . | . | . | . | . | . |
| 21-225_79G1_LC | . | . | . | . | . | . | . | . | . | . | . | Y | . | . | S | . | . | . | . | . | . | . | . | . | . | . |
| 21-225_79G12_LC | . | . | . | . | . | . | . | . | . | . | . | Y | . | . | . | . | . | . | . | . | . | . | . | . | . | . |
| 21-225_80A1_LC | . | . | . | . | . | . | . | . | . | . | . | Y | . | . | . | . | . | . | . | . | . | . | . | . | . | . |
| 21-225_80A6_LC | . | . | . | . | . | . | . | . | . | . | . | . | . | . | . | . | . | P | . | . | . | . | . | . | . | . |
| 21-226_80B10_LC | . | . | . | . | . | . | . | . | . | . | . | . | . | . | . | . | . | . | . | . | . | . | . | . | . | . |
| 21-225_80C1_LC | . | . | . | . | . | . | . | . | . | . | . | Y | . | . | . | . | . | . | . | . | . | . | . | . | . | . |
| 21-225_80D5_LC | . | L | . | . | . | . | . | . | . | . | . | . | . | . | . | . | . | . | V | . | . | . | . | . | . | . |
| 21-225_80E12_LC | . | . | . | . | . | . | C | . | . | . | . | . | . | . | . | . | . | . | . | . | V | . | . | . | . | . |
| 21-225_80E3_LC | . | . | . | . | . | . | . | . | . | . | . | Y | . | . | S | . | . | . | . | . | . | . | . | . | . | . |
| 21-225_80H12_LC | . | . | . | . | . | . | . | . | . | . | . | . | . | . | . | . | . | . | . | . | . | . | . | . | . | . |
| 21-225_80H7_LC | . | . | . | . | . | . | . | . | . | . | . | . | . | . | . | . | . | . | . | . | . | . | . | . | . | . |
| 21-225_81C11_LC | . | . | . | . | . | . | . | . | . | . | . | Y | . | . | . | V | . | . | . | . | . | . | . | . | . | . |
| 21-225_81C5_LC | . | . | . | . | . | . | . | . | . | . | . | Y | . | . | S | . | . | . | . | . | . | . | . | . | . | . |
| 21-225_83B6_LC | . | . | . | . | . | . | . | . | . | . | . | . | . | . | . | . | . | . | . | . | . | . | . | . | . | . |
| 21-225_83B7_LC | . | . | . | . | . | . | . | . | . | . | . | Y | . | . | S | V | . | . | . | . | . | . | . | . | . | . |
| 21-225_83C10_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | E | I | V | L | T | Q | S | P | G | T | L | S | L | S | P | G | E | R | A | T | L | S | C | R | A | S |
| 21-225_83G1_LC | . | . | . | . | . | . | . | . | . | . | N | Y | . | . | . | . | . | . | . | . | . | . | . | . | . | . |
| 21-225_84E12_LC | . | . | . | . | . | . | . | . | . | . | . | . | . | . | . | . | . | . | . | . | . | . | . | . | . | . |
| 21-225_85A3_LC | . | . | . | . | . | . | . | . | . | . | . | N | . | . | . | . | . | . | . | . | . | . | . | . | . | . |
| 21-225_85B4_LC | . | . | . | . | . | . | . | . | . | . | . | N | . | . | . | . | . | . | . | . | . | . | . | . | . | . |
| 21-225_85B9_LC | . | . | . | . | . | . | . | . | . | . | . | N | . | . | . | . | . | . | . | . | . | . | . | . | . | . |
| 21-225_85D8_LC | . | . | . | . | . | . | . | . | . | . | . | N | . | . | Q | . | . | . | . | . | . | . | . | . | . | . |
| 21-225_85G10_LC | . | . | . | . | . | . | . | . | . | S | . | . | . | . | . | . | . | . | . | . | . | . | . | . | . | . |
| 21-225_85G6_LC | . | . | A | . | . | . | . | . | . | . | . | . | . | . | . | . | . | . | . | . | . | . | . | . | . | . |
| 21-225_86C1_LC | . | . | . | . | . | . | . | . | . | . | . | Y | . | . | . | . | . | . | . | . | . | . | . | . | . | . |
| 21-225_86C11_LC | . | . | . | . | . | . | . | . | . | . | . | Y | . | . | . | . | . | . | . | . | . | . | . | . | . | . |
| 21-225_86E4_LC | . | . | . | . | . | . | . | . | . | . | N | Y | . | . | . | . | . | . | . | . | . | . | . | . | . | . |
| 21-225_87A12_LC | . | . | . | . | . | . | . | . | . | . | N | Y | . | . | . | . | . | . | . | . | . | . | . | . | . | . |
| 21-225_87C9_LC | . | . | . | . | . | . | . | . | . | . | . | Y | . | . | . | V | . | . | . | . | . | . | . | . | . | . |
| 21-225_87E5_LC | . | N | . | . | . | . | . | . | . | . | . | Y | . | . | . | . | . | . | . | . | . | . | . | . | . | . |
| 21-225_87H1_LC | . | . | . | . | . | . | . | . | . | . | . | . | . | . | . | . | . | . | . | . | . | . | . | . | . | . |
| 21-225_88E7_LC | . | . | . | . | . | . | . | . | . | . | . | . | . | . | . | . | . | . | . | . | . | . | . | . | . | . |
| 21-225_88F7_LC | . | . | . | . | . | . | . | . | . | . | . | . | . | . | . | . | . | . | . | . | . | . | . | . | . | . |
| 21-225_88G9_LC | . | . | . | . | . | . | . | . | . | . | N | Y | . | . | . | . | . | . | . | . | . | . | . | . | . | . |
| 21-225_88H1_LC | . | . | . | . | . | . | . | . | . | . | . | Y | . | . | . | . | . | . | . | . | . | . | . | . | . | . |
| 21-225_89A11_LC | . | . | . | . | . | . | . | . | . | . | . | . | . | . | . | . | . | . | . | . | . | . | . | . | . | . |
| 21-225_89D5_LC | . | . | . | . | . | . | . | . | . | . | . | Y | . | . | S | . | . | . | . | . | . | . | . | . | . | . |
| 21-225_89E10_LC | . | . | . | . | . | . | . | . | . | . | . | Y | . | . | S | . | . | . | . | . | . | . | . | . | . | . |
| 21-225_89H5_LC | . | . | . | . | . | . | . | . | . | . | . | . | . | . | . | . | . | . | . | . | . | . | . | . | . | . |
| 21-225_90A5_LC | . | . | . | . | . | . | . | . | . | . | . | Y | . | . | . | . | . | . | . | . | . | . | . | . | . | . |
| 21-225_90F10_LC | . | . | . | . | . | . | . | . | . | . | . | Y | . | . | . | . | . | . | . | . | . | . | . | . | . | . |
| 21-225_90G4_LC | . | . | . | . | . | . | . | . | . | . | . | Y | . | . | . | . | . | . | . | . | . | . | . | . | . | . |
| 21-225_90G5_LC | . | . | . | . | . | . | . | . | . | . | . | Y | . | . | . | . | . | . | . | . | . | . | . | . | . | . |
| 21-225_91B2_LC | . | . | . | . | . | . | . | . | . | . | . | Y | . | . | . | . | . | . | . | . | . | . | . | . | . | . |
| 21-225_91B3_LC | . | . | . | . | . | . | . | . | . | . | . | Y | . | . | . | . | . | . | . | . | . | . | . | . | . | . |

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | | |
| CONSENSUS | E | I | V | L | T | Q | S | P | G | T | L | S | L | S | P | G | E | R | A | T | L | S | C | R | A | S |
| 21-225_91B4_LC | . | . | . | . | . | . | . | . | . | . | . | Y | . | . | . | . | . | . | . | . | . | . | . | . | . | . |
| 21-225_91E9_LC | . | . | . | . | . | . | . | . | . | . | . | Y | . | . | . | . | . | . | . | . | . | . | . | . | . | . |
| 21-225_91F3_LC | . | . | . | . | . | . | . | . | . | . | . | Y | . | . | . | . | . | . | . | . | . | . | . | . | . | . |
| 21-225_92B1_LC | . | . | . | . | . | . | . | . | . | . | . | . | . | . | . | . | . | . | . | . | . | . | . | . | . | . |
| 21-225_92D6_LC | . | . | . | . | . | . | . | . | . | . | . | Y | . | . | . | . | . | . | . | . | . | . | . | . | . | . |
| 21-225_93C2_LC | . | . | . | . | . | . | . | . | . | . | . | Y | . | . | . | . | . | . | . | . | . | . | . | . | . | . |
| 21-225_93E4_LC | . | . | . | . | . | . | . | . | . | . | . | Y | . | . | . | . | . | . | . | . | . | . | . | . | . | . |
| 21-225_93E9_LC | . | . | . | . | . | . | . | . | . | . | . | . | . | . | . | . | . | . | . | . | . | . | . | . | . | . |
| 21-225_94D3_LC | . | . | . | . | . | . | . | . | . | . | . | . | . | . | Q | . | . | . | . | . | . | . | . | . | . | . |
| 21-225_94F12_LC | . | . | . | . | . | . | . | . | . | . | . | Y | . | . | . | . | . | . | . | . | R | . | . | . | . | . |
| 21-225_94G10_LC | . | . | . | . | . | . | . | . | . | . | . | . | . | . | . | . | . | . | . | . | . | . | . | . | . | . |
| 21-225_95D2_LC | . | . | . | . | . | . | . | . | . | . | . | . | . | . | . | . | . | . | . | . | . | . | . | . | . | . |
| 21-225_95F9_LC | . | . | . | . | . | . | . | . | . | . | . | Y | . | . | . | . | . | . | . | . | . | . | . | . | . | . |
| 21-225_95G9_LC | . | . | . | . | . | . | . | . | . | . | . | Y | . | . | S | . | . | . | . | . | . | . | . | . | . | . |
| 21-225_95H10_LC | . | . | . | . | S | . | . | . | . | . | . | Y | . | . | S | . | . | . | . | . | . | . | . | . | . | . |
| 21-225_97A2_LC | . | . | . | . | . | . | . | . | . | . | . | Y | . | . | S | . | . | . | . | . | . | . | . | . | . | . |
| 21-225_97E5_LC | . | . | . | . | . | . | . | . | . | . | . | . | . | . | . | . | . | . | . | . | . | . | . | . | . | . |

**4397**

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
|  |  |  |  |  |  | K_CDR1 |  |  |  |  |  |  |  |  |  |  |  |  |  |  |  |  |  | K_FR2 |  |  |
| CONSENSUS | - | - | Q | S | V | Y | S | - | - | - | - | - | S | Y | L | A | W | Y | Q | Q | K | P | G | Q | A | P |
| 21-225_147G8_LC | . | . | R | : | F | . | . | . | . | . | . | . | N | . | . | . | . | . | . | . | . | . | . | . | . | . |
| 21-225_190A3_LC | . | . | N | : | . | R | . | . | . | . | . | . | N | F | . | . | . | . | . | . | . | . | . | . | . | . |
| 21-225_190A7_LC | . | . | . | . | . | R | . | . | . | . | . | . | N | F | . | . | . | . | . | . | . | . | . | . | . | . |
| 21-225_190B4_LC | . | . | . | : | R | . | . | . | . | . | . | . | . | F | . | . | . | . | . | . | . | . | . | . | . | . |
| 21-225_190G10_LC | . | . | F | . | . | S | . | . | . | . | . | . | R | F | . | . | . | . | . | . | . | . | . | . | . | . |
| 21-225_191A3_LC | . | . | . | : | . | R | . | . | . | . | . | . | . | F | . | . | . | . | . | . | . | . | . | . | . | . |
| 21-225_191A5_LC | . | . | . | . | . | S | . | . | . | . | . | . | R | F | . | . | . | . | . | . | . | . | . | . | . | . |
| 21-225_191C3_LC | . | . | . | . | : | R | . | . | . | . | . | . | . | F | . | . | . | . | . | . | . | . | . | . | . | . |
| 21-225_191D3_LC | . | . | . | . | : | R | T | . | . | . | . | . | N | F | . | . | . | . | . | . | Q | . | . | . | . | . |
| 21-225_191F1_LC | . | . | . | . | : | S | L | . | . | . | . | . | N | F | . | . | . | . | . | . | . | . | . | . | . | . |
| 21-225_191H7_LC | . | . | . | . | : | R | T | . | . | . | . | . | D | F | . | . | . | . | . | . | Q | . | . | . | . | . |
| 21-225_192B3_LC | . | . | . | . | . | R | . | . | . | . | . | . | . | F | . | . | . | . | . | . | . | . | . | . | . | . |
| 21-225_192F8_LC | . | . | . | . | : | S | . | . | . | . | . | . | . | . | . | . | . | . | . | . | . | . | . | . | . | . |
| 21-225_192G12_LC | . | . | . | . | . | R | . | . | . | . | . | . | D | F | . | . | . | L | . | . | . | . | . | . | . | . |
| 21-225_192H3_LC | . | . | . | . | . | S | . | . | . | . | . | . | N | F | . | . | . | . | . | . | . | . | . | . | . | . |
| 21-225_193A1_LC | . | . | . | . | . | R | . | . | . | . | . | . | N | F | . | . | . | H | . | . | . | . | . | . | . | . |
| 21-225_193B1_LC | . | . | . | . | . | R | . | . | . | . | . | . | N | F | . | . | . | H | . | . | . | . | . | . | . | . |
| 21-225_193B10_LC | . | . | F | . | . | S | . | . | . | . | . | . | . | . | . | . | . | . | . | . | . | . | . | . | . | . |
| 21-225_193B12_LC | . | . | . | . | : | R | . | . | . | . | . | . | . | F | . | . | . | . | . | . | . | . | . | . | . | . |
| 21-225_193C8_LC | . | . | . | . | : | R | . | . | . | . | . | . | . | F | . | . | . | . | . | . | . | . | . | . | . | . |
| 21-225_193D3_LC | . | . | . | . | : | R | . | . | . | . | . | . | . | F | . | . | . | . | . | . | . | . | . | . | . | . |
| 21-225_193D8_LC | . | . | . | . | : | R | T | . | . | . | . | . | N | F | . | . | . | . | . | . | . | . | . | . | . | . |
| 21-225_193E6_LC | . | . | . | . | . | R | . | . | . | . | . | . | G | . | . | . | . | . | . | . | . | . | . | . | . | . |
| 21-225_193F3_LC | . | . | . | . | : | R | T | . | . | . | . | . | D | F | . | . | V | . | . | . | Q | . | . | . | . | . |
| 21-225_193G8_LC | . | . | . | . | . | R | T | . | . | . | . | . | N | F | . | . | . | H | . | . | . | . | . | . | . | . |
| 21-225_193H6_LC | . | . | . | . | : | R | T | . | . | . | . | . | N | F | . | . | . | . | . | . | Q | . | . | . | . | . |
| 21-225_194C10_LC | . | . | F | . | . | N | . | . | . | . | . | . | G | . | . | . | . | . | . | . | . | . | . | . | Y | . |
| 21-225_194E5_LC | . | . | . | . | : | R | T | . | . | . | . | . | N | F | . | . | . | . | . | . | . | . | . | . | . | . |
| 21-225_194E6_LC | . | . | . | . | : | R | . | . | . | . | . | . | N | F | . | . | . | . | . | . | . | . | . | . | . | . |

4398

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | V | Y | S | - | - | - | - | - | S | Y | L | A | W | Y | Q | Q | K | P | G | Q | A | P |
| 21-225_195B1_LC | . | . | P | | | N | . | . | . | . | . | . | N | . | | | | | | | | | | | T | . |
| 21-225_195C8_LC | . | . | | | i | . | . | . | . | . | . | . | F | . | | | | | | | | | | | | . |
| 21-225_196G10_LC | . | . | P | | | S | N | . | . | . | . | . | F | | | | | | | | | | | | . | . |
| 21-225_196H12_LC | . | . | | | | . | . | . | . | . | . | . | N | F | . | | . | W | . | | | | | . | . | . |
| 21-225_197F4_LC | . | . | | | i | . | . | . | . | . | . | . | F | . | | | | L | . | | | | | . | . | . |
| 21-225_198B1_LC | . | . | | | i | . | T | . | . | . | . | . | N | F | . | | | | | | Q | | . | . | . | . |
| 21-225_198C2_LC | . | . | | | | . | . | . | . | . | . | | | . | | | | | | | | | | | | |
| 21-225_198E1_LC | . | . | | | i | . | . | . | . | . | . | . | N | F | . | | | | | | | | | . | . | . |
| 21-225_198G10_LC | . | . | | | i | . | . | . | . | . | . | . | . | F | . | | | | | | | | | . | . | . |
| 21-225_199C2_LC | . | . | | | i | . | . | . | . | . | . | . | . | F | . | | | | | | | | | . | . | . |
| 21-225_199C7_LC | . | . | | | i | . | T | . | . | . | . | . | N | F | . | | | | | | | | | . | . | . |
| 21-225_199G11_LC | . | . | | | . | . | . | . | . | . | . | . | . | . | | | | | | | | | | . | . | . |
| 21-225_200A1_LC | . | . | | . | . | N | . | . | . | . | . | . | N | . | | | | | | | . | | | . | . | . |
| 21-225_200F10_LC | . | . | . | N | i | . | . | . | . | . | . | . | . | F | . | | | | | | | | | . | . | . |
| 21-225_201B2_LC | . | . | . | . | . | S | . | . | . | . | . | . | N | . | | | | | | | . | | | . | . | . |
| 21-225_201E1_LC | . | . | P | . | i | N | . | . | . | . | . | . | G | F | . | | | | | | | | | . | . | . |
| 21-225_202D11_LC | . | . | . | . | i | N | . | . | . | . | . | . | N | . | | | | | . | | | | | . | . | . |
| 21-225_202D9_LC | . | . | . | . | . | N | . | . | . | . | . | . | G | . | | | | | | | | | | . | S | . |
| 21-225_205G7_LC | . | . | . | . | . | F | . | . | . | . | . | . | N | . | | | | | | | | | | . | . | . |
| 21-225_208A9_LC | . | . | . | . | . | S | N | . | . | . | . | . | N | . | | | | | | | | | | . | . | . |
| 21-225_208B5_LC | . | . | . | . | . | S | . | . | . | . | . | . | N | . | | | | | . | | | | | . | . | . |
| 21-225_220E1_LC | . | . | . | . | i | S | . | . | . | . | . | . | . | . | | V | | | | | | | N | . | . | . |
| 21-225_222C7_LC | . | . | . | . | . | . | . | . | . | . | . | . | N | . | | | | | | | . | | | | | |
| 21-225_225E7_LC | . | . | . | . | . | . | . | . | . | . | . | . | . | . | | | | | | | | | | . | . | . |
| 21-225_23B9_LC | . | . | . | N | . | . | . | . | . | . | . | . | . | . | | | | | | | | | | . | T | . |
| 21-225_44F6_LC | . | . | . | . | . | S | G | . | . | . | . | . | . | . | | | | | | | | | | . | . | . |
| 21-225_59B2_LC | . | . | . | . | . | . | . | . | . | . | . | . | . | F | . | | | F | . | | | | S | . | . | . |
| 21-225_74A3_LC | . | . | . | . | . | . | . | . | . | . | . | . | . | . | | | | | | | | | | . | . | . |
| 21-225_74A4_LC | . | . | . | . | . | . | . | . | . | . | . | . | . | . | | | | | | | | | | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | S | V | Y | S | - | - | - | - | - | S | Y | L | A | W | Y | Q | Q | K | P | G | Q | A | P |
| 21-225_74B12_LC | . | . | . | . | . | . | . | . | . | . | . | . | . | . | . | . | . | . | . | . | . | . | . | . | . | . |
| 21-225_74B2_LC | . | . | . | N | I | . | . | . | . | . | . | . | . | . | . | . | . | . | . | . | . | . | . | . | . | . |
| 21-225_74C5_LC | . | . | . | . | . | N | . | . | . | . | . | . | N | . | . | . | . | . | . | . | . | . | . | . | . | . |
| 21-225_74C7_LC | . | . | P | N | . | O | . | . | . | . | . | . | . | . | . | . | . | . | . | . | . | . | . | . | . | . |
| 21-225_74D11_LC | . | . | . | . | . | . | . | . | . | . | . | . | . | . | . | . | . | . | . | . | . | . | . | . | . | . |
| 21-225_74D2_LC | . | . | . | . | . | O | . | . | . | . | . | . | N | . | . | . | . | . | . | . | . | . | . | . | . | . |
| 21-225_74D5_LC | . | . | . | . | . | N | . | . | . | . | . | . | N | . | . | . | . | . | R | . | . | . | . | . | . | . |
| 21-225_74D6_LC | . | . | . | . | . | . | . | . | . | . | . | . | . | . | . | . | . | . | . | . | . | . | . | . | . | . |
| 21-225_74F4_LC | . | . | . | I | F | S | . | . | . | . | . | . | . | . | . | . | . | . | . | . | . | . | . | . | S | . |
| 21-225_74F5_LC | . | . | . | . | . | . | . | . | . | . | . | . | . | . | . | . | . | . | . | . | . | . | . | . | . | . |
| 21-225_74G8_LC | . | . | . | . | F | S | . | . | . | . | . | . | A | . | . | . | . | . | . | . | . | . | . | . | . | . |
| 21-225_74H5_LC | . | . | . | . | . | N | . | . | . | . | . | . | N | . | . | . | . | . | R | . | . | . | . | . | . | . |
| 21-225_74H7_LC | . | . | . | N | I | . | . | . | . | . | . | . | . | . | . | . | . | . | . | . | . | . | . | . | . | . |
| 21-225_74H8_LC | . | . | . | . | F | S | . | . | . | . | . | . | . | . | . | . | . | . | . | . | . | . | . | . | . | . |
| 21-225_75A12_LC | . | . | . | . | . | S | . | . | . | . | . | . | R | . | . | . | . | . | . | . | . | . | . | . | . | . |
| 21-225_75A5_LC | . | . | . | . | . | . | . | . | . | . | . | . | . | . | . | . | . | . | . | . | . | . | . | . | . | . |
| 21-225_75A8_LC | . | . | . | . | . | . | . | . | . | . | . | . | . | . | . | . | . | . | . | . | . | . | . | . | . | . |
| 21-225_75B10_LC | . | . | P | . | . | N | . | . | . | . | . | . | Y | . | . | . | . | . | . | . | . | . | . | . | . | . |
| 21-225_75C2_LC | . | . | . | . | . | . | . | . | . | . | . | . | . | . | . | . | . | . | . | . | . | . | . | . | . | . |
| 21-225_75C3_LC | . | . | . | . | . | S | . | . | . | . | . | . | R | . | . | . | . | . | . | . | . | . | . | . | . | . |
| 21-225_75D3_LC | . | . | . | . | . | . | . | . | . | . | . | . | . | . | . | . | . | . | . | . | . | . | . | . | . | . |
| 21-225_75D9_LC | . | . | . | . | . | S | . | . | . | . | . | . | . | . | . | . | . | . | . | . | . | R | . | . | . | . |
| 21-225_75F3_LC | . | . | . | . | . | . | . | . | . | . | . | . | . | . | . | . | . | . | . | . | . | . | . | . | . | . |
| 21-225_75G12_LC | . | . | . | . | . | . | . | . | . | . | . | . | . | . | . | . | . | . | . | . | . | . | . | . | . | . |
| 21-225_75G3_LC | . | . | . | N | . | O | . | . | . | . | . | . | . | . | . | . | . | . | . | . | . | . | . | . | . | . |
| 21-225_75G7_LC | . | . | . | . | . | O | . | . | . | . | . | . | . | . | . | . | . | . | . | . | . | R | . | . | . | . |
| 21-225_75G9_LC | . | . | P | . | . | O | . | . | . | . | . | . | . | . | . | . | . | . | . | . | . | . | . | . | . | . |
| 21-225_75H8_LC | . | . | . | N | I | . | . | . | . | . | . | . | N | . | . | . | . | . | . | . | . | . | . | . | . | . |
| 21-225_76A4_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | V | Y | S | - | - | - | - | - | S | Y | L | A | W | Y | Q | Q | K | P | G | Q | A | P |
| 21-225_76A7_LC | . | . | P | . | . | Q | . | . | . | . | . | . | . | . | . | . | . | . | . | . | R | . | . | . | . | . |
| 21-225_76C9_LC | . | . | . | . | . | S | . | . | . | . | . | . | . | . | . | S | . | . | . | . | . | . | . | . | . | . |
| 21-225_76D1_LC | . | . | . | N | I | . | . | . | . | . | . | . | N | . | . | . | . | . | . | E | . | . | . | . | . | . |
| 21-225_76E10_LC | . | . | . | . | . | N | . | . | . | . | . | . | N | . | . | . | . | . | R | . | . | . | . | . | . | . |
| 21-225_76G4_LC | . | . | . | . | . | . | . | . | . | . | . | . | . | . | . | . | . | . | . | . | . | . | . | . | . | . |
| 21-225_77A10_LC | . | . | . | . | . | H | . | . | . | . | . | . | R | . | . | . | . | . | . | . | . | . | . | . | . | . |
| 21-225_77C12_LC | . | . | . | . | . | Q | . | . | . | . | . | . | . | . | . | . | . | . | . | . | . | R | . | . | . | . |
| 21-225_77C5_LC | . | . | . | . | . | . | . | . | . | . | . | . | . | . | . | . | . | . | . | . | . | . | . | . | . | . |
| 21-225_77F7_LC | . | . | . | . | . | . | . | . | . | . | . | . | . | . | . | . | . | . | . | . | . | . | . | . | . | . |
| 21-225_77G1_LC | . | . | . | . | . | . | . | . | . | . | . | . | . | . | . | . | . | . | . | . | . | . | . | . | . | . |
| 21-225_78E7_LC | . | . | . | N | . | Q | . | . | . | . | . | . | N | . | . | . | . | . | . | . | . | . | . | . | . | . |
| 21-225_79F11_LC | . | . | . | . | . | . | . | . | . | . | . | . | . | . | . | . | . | . | . | . | . | . | . | . | . | . |
| 21-225_79F3_LC | . | . | . | . | . | . | . | . | . | . | . | . | . | . | . | . | . | . | . | . | . | . | A | . | . | . |
| 21-225_79G1_LC | . | . | . | . | . | . | . | . | . | . | . | . | . | . | . | . | . | . | . | . | . | . | A | . | . | . |
| 21-225_79G12_LC | . | . | . | . | . | . | . | . | . | . | . | . | . | . | . | . | . | . | . | . | . | . | . | . | . | . |
| 21-225_80A1_LC | . | . | . | . | . | . | . | . | . | . | . | . | . | . | . | . | . | . | . | . | . | . | . | . | . | . |
| 21-225_80A6_LC | . | . | . | . | . | Q | . | . | . | . | . | . | G | . | . | . | . | . | . | . | . | . | . | . | . | . |
| 21-225_80B10_LC | . | . | . | . | . | Q | . | . | . | . | . | . | . | . | . | . | . | . | . | . | . | . | . | . | . | . |
| 21-225_80C1_LC | . | . | . | . | . | . | . | . | . | . | . | . | . | . | . | . | . | . | . | . | . | . | . | . | . | . |
| 21-225_80D5_LC | . | . | . | N | I | . | . | . | . | . | . | . | . | . | . | . | . | . | . | . | . | . | . | . | . | . |
| 21-225_80E12_LC | . | . | . | . | . | N | . | . | . | . | . | . | N | . | . | . | . | . | R | . | . | . | D | . | . | . |
| 21-225_80E3_LC | . | . | . | . | . | . | . | . | . | . | . | . | . | . | . | . | . | . | . | . | . | . | . | . | . | . |
| 21-225_80H12_LC | . | . | . | . | . | . | . | . | . | . | . | . | . | . | . | . | . | . | . | . | . | . | . | . | . | . |
| 21-225_80H7_LC | . | . | . | . | I | N | . | . | . | . | . | . | N | . | . | . | . | . | . | . | . | . | . | . | . | . |
| 21-225_81C11_LC | . | . | . | . | . | . | . | . | . | . | . | . | . | . | . | . | . | . | . | . | . | . | . | . | . | . |
| 21-225_81C5_LC | . | . | . | . | . | . | . | . | . | . | . | . | . | . | . | . | . | . | . | . | . | . | . | . | . | . |
| 21-225_83B6_LC | . | . | . | . | . | Q | . | . | . | . | . | . | G | . | . | . | . | . | . | . | . | . | . | . | . | . |
| 21-225_83B7_LC | . | . | . | . | . | . | . | . | . | . | . | . | . | . | . | . | . | . | . | . | . | . | . | . | . | . |
| 21-225_83C10_LC | . | . | P | . | . | H | . | . | . | . | . | . | N | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | V | Y | S | - | - | - | - | - | S | Y | L | A | W | Y | Q | Q | K | P | G | Q | A | P |
| 21-225_83G1_LC | . | . | . | . | . | . | . | . | . | . | . | . | . | . | . | . | . | . | . | . | . | . | . | . | . | . |
| 21-225_84E12_LC | . | . | . | . | I | N | . | . | . | . | . | . | N | . | . | . | . | . | . | . | . | . | . | . | . | . |
| 21-225_85A3_LC | . | . | . | . | . | . | . | . | . | . | . | . | . | . | . | . | . | . | . | . | . | . | A | . | . | . |
| 21-225_85B4_LC | . | . | . | . | . | . | . | . | . | . | . | . | . | . | . | . | . | . | . | . | . | . | . | . | . | . |
| 21-225_85B9_LC | . | . | . | . | . | N | . | . | . | . | . | . | N | . | . | . | . | . | . | . | . | . | . | . | . | . |
| 21-225_85D6_LC | . | . | . | . | . | S | . | . | . | . | . | . | . | . | . | . | . | . | . | . | . | . | . | . | . | . |
| 21-225_85G10_LC | . | . | . | . | . | W | . | . | . | . | . | . | . | . | . | . | . | . | . | . | . | . | . | . | . | . |
| 21-225_85G6_LC | . | . | P | . | . | Q | . | . | . | . | . | . | . | . | . | . | . | . | . | . | . | . | . | . | . | . |
| 21-225_86C1_LC | . | . | . | . | . | . | . | . | . | . | . | . | . | . | . | . | . | . | . | . | . | . | A | . | . | . |
| 21-225_86C11_LC | . | . | . | . | . | . | . | . | . | . | . | . | . | . | . | . | . | . | . | . | . | . | . | . | . | . |
| 21-225_86E4_LC | . | . | . | . | . | . | . | . | . | . | . | . | . | . | . | . | . | . | . | . | . | . | . | . | . | . |
| 21-225_87A12_LC | . | . | . | . | . | . | . | . | . | . | . | . | . | . | . | . | . | . | . | . | . | . | . | . | . | . |
| 21-225_87C9_LC | . | . | . | . | . | . | . | . | . | . | . | . | . | . | . | . | . | . | . | . | . | . | . | . | . | . |
| 21-225_87E5_LC | . | . | . | . | . | . | . | . | . | . | . | . | . | . | . | . | . | . | . | . | . | . | . | . | . | . |
| 21-225_87H1_LC | . | . | . | . | . | Q | . | . | . | . | . | . | N | . | . | . | . | . | . | . | . | . | . | . | . | . |
| 21-225_88E7_LC | . | . | . | . | . | . | . | . | . | . | . | . | . | . | . | . | . | . | . | . | . | . | . | . | . | . |
| 21-225_88F7_LC | . | . | . | . | . | . | . | . | . | . | . | . | . | . | . | . | . | . | . | . | . | . | . | . | . | . |
| 21-225_88G9_LC | . | . | . | . | . | . | . | . | . | . | . | . | . | . | . | . | . | . | . | . | . | . | . | . | . | . |
| 21-225_88H1_LC | . | . | . | . | . | . | . | . | . | . | . | . | . | . | . | . | . | . | . | . | . | . | . | . | . | . |
| 21-225_89A11_LC | . | . | . | . | . | Q | . | . | . | . | . | . | N | P | . | . | . | . | . | . | . | . | . | . | . | . |
| 21-225_89D5_LC | . | . | . | . | . | . | . | . | . | . | . | . | . | . | . | . | . | . | . | . | . | . | . | . | . | . |
| 21-225_89E10_LC | . | . | . | . | . | . | . | . | . | . | . | . | . | . | . | . | . | . | . | . | . | . | . | . | . | . |
| 21-225_89H5_LC | . | . | . | . | . | N | . | . | . | . | . | . | N | . | . | . | . | . | . | . | . | . | . | . | . | . |
| 21-225_90A5_LC | . | . | . | . | . | . | . | . | . | . | . | . | . | . | . | . | . | . | . | . | . | . | . | . | . | . |
| 21-225_90F10_LC | . | . | . | . | . | . | . | . | . | . | . | . | . | . | . | . | . | . | . | . | . | . | . | . | . | . |
| 21-225_90G4_LC | . | . | . | . | . | . | . | . | . | . | . | . | . | . | . | . | . | . | . | . | . | . | . | . | . | . |
| 21-225_90G5_LC | . | . | . | . | . | . | . | . | . | . | . | . | . | . | . | . | . | . | . | . | . | . | . | . | . | . |
| 21-225_91B2_LC | . | . | . | . | . | . | . | . | . | . | . | . | . | . | . | . | . | . | . | . | . | . | . | . | . | . |
| 21-225_91B3_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | S | V | Y | S | - | - | - | - | - | S | Y | L | A | W | Y | Q | Q | K | P | G | Q | A | P |
| 21-225_91B4_LC | . | . | . | . | . | . | | | | | | | | . | . | . | . | . | . | . | . | . | . | . | . | . |
| 21-225_91E9_LC | . | . | . | . | . | . | | | | | | | . | . | . | . | . | . | . | . | . | . | . | . | . | . |
| 21-225_91F3_LC | . | . | . | . | . | . | | | | | | | . | . | . | . | . | . | . | . | . | . | A | . | . | . |
| 21-225_92B1_LC | . | . | . | . | . | O | | | | | . | | . | N | . | . | . | . | . | . | . | R | . | . | . | . |
| 21-225_92D6_LC | . | . | . | . | . | . | | | | | | | . | . | . | . | . | . | . | . | . | . | . | . | . | . |
| 21-225_93C2_LC | . | . | . | . | . | . | | | | | | | . | . | . | . | . | . | . | . | . | . | . | . | . | . |
| 21-225_93E4_LC | . | . | . | . | . | . | | | | | | | . | . | . | . | . | . | . | . | . | . | . | . | . | . |
| 21-225_93E9_LC | . | . | . | . | . | O | | | | | | | . | . | . | . | . | . | . | . | . | T | . | . | . | . |
| 21-225_94D3_LC | . | . | . | . | . | S | | | | | | | R | . | . | . | . | . | . | . | . | . | . | . | . | . |
| 21-225_94F12_LC | . | . | . | . | . | . | | | | | | | . | . | . | . | . | . | . | . | . | . | . | . | . | . |
| 21-225_94G10_LC | . | . | . | . | . | N | | | | | | | N | . | . | . | . | . | R | . | . | . | . | . | . | . |
| 21-225_95D2_LC | . | . | . | . | . | . | | | | | | | . | . | . | . | . | . | . | . | . | . | . | . | . | . |
| 21-225_95F9_LC | . | . | . | . | . | . | | | | | | | . | . | . | . | . | . | . | . | . | . | . | . | . | . |
| 21-225_95G9_LC | . | . | . | . | . | . | | | | | | | . | . | . | . | . | . | . | . | . | . | . | . | . | . |
| 21-225_95H10_LC | . | . | . | . | . | . | | | | | | | . | . | . | . | . | . | . | . | . | . | . | . | . | . |
| 21-225_97A2_LC | . | . | . | . | . | . | | | | | | | . | . | . | . | . | . | . | . | . | . | . | . | . | . |
| 21-225_97E5_LC | . | . | . | . | . | . | | | | | | | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | R | L | L | I | Y | G | - | - | - | - | - | - | - | - | A | S | S | R | A | T | G | I | P | D | R | F |
| 21-225_147G8_LC | . | I | | | | | . | | . | | | | | | | | | | | | | | | | . | I |
| 21-225_190A3_LC | . | | | | | | . | . | | | | | | | | | | | | | | | | | . | . |
| 21-225_190A7_LC | | | | | | | . | | . | | | | | | | Y | R | | | | | | | | . | . |
| 21-225_190B4_LC | . | | | | | | | | | | | | | | | | | | | | | | | . | . | . |
| 21-225_190G10_LC | . | | | | | | . | | | | | | | | | | | | | | | | | | . | . |
| 21-225_191A3_LC | | | | | | | | | | | | | | | | | | | | | | | | | . | . |
| 21-225_191A5_LC | . | | | | | | . | | | | | | | | | | N | | | | | | | | . | . |
| 21-225_191C3_LC | . | | | | | | . | | | | | | | | | | | | | | | | | | . | . |
| 21-225_191D3_LC | | | | | | | . | | | | | | | | | | | | | | | | | | . | . |
| 21-225_191F1_LC | . | | | | | | . | | | | | | | | | | | | | | | | | | . | . |
| 21-225_191H7_LC | . | | | | | | . | | | | | | | | P | | | | | | | | | | . | . |
| 21-225_192B3_LC | . | | | | | | . | | | | | | | | | | | | | | | | | | . | . |
| 21-225_192F8_LC | . | | | | | | . | | | | | | | | | | | | | | | | | A | . | . |
| 21-225_192G12_LC | | | | | | | . | | | | | | | | V | | R | | | | | | | | . | . |
| 21-225_192H3_LC | . | | L | F | | | . | | | | | | | | | | | | | | | | | | . | . |
| 21-225_193A1_LC | . | F | | | | | . | | | | | | | | | | R | | | | | | | | . | . |
| 21-225_193B1_LC | . | | | | | | . | | | | | | | | | | P | | | | | | | | . | . |
| 21-225_193B10_LC | . | V | | . | | | . | | | | | | | | | | R | | | | | | | | . | . |
| 21-225_193B12_LC | . | | | . | . | D | . | | | | | | | | | | . | | | | | | | | . | . |
| 21-225_193C8_LC | . | | | . | | | . | | | | | | | | | | . | | | | | | | | . | . |
| 21-225_193D3_LC | . | | | | | | . | | | | | | | | | | N | | | | | | | | . | . |
| 21-225_193D8_LC | . | | | | | | . | | | | | | | | | | . | | | | | | | | . | . |
| 21-225_193E6_LC | . | | | | | | . | | | | | | | | | | . | | | | | | | | . | . |
| 21-225_193F3_LC | . | | | | | | . | | | | | | | | | | . | | | | | | S | . | E | . |
| 21-225_193G8_LC | . | | | | | | . | | | | | | | | | | R | | | | | | | | . | . |
| 21-225_193H6_LC | . | | | . | | | . | | | | | | | | | | . | | | | | | | | . | . |
| 21-225_194C10_LC | . | | | F | | | . | | | | | | | | | | . | | | | | | | | . | . |
| 21-225_194E5_LC | . | | | . | | | . | | | | | | | | | | . | | | | | | | | . | . |
| 21-225_194E6_LC | . | | | . | | | . | | | | | | | | | | . | | | | | | | | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | K_CDR2 | | | | | | | | | | | | |
| CONSENSUS | R | L | L | I | Y | G | - | - | - | - | - | - | - | - | A | S | S | R | A | T | G | I | P | D | R | F |
| 21-225_195B1_LC | . | . | | | | . | . | . | . | . | . | . | . | . | . | . | N | . | . | . | . | V | . | . | . | . |
| 21-225_195C8_LC | . | . | | | | . | . | . | . | . | . | . | . | . | . | F | . | . | . | . | . | . | . | . | . | . |
| 21-225_196G10_LC | . | . | . | | | . | . | . | . | . | . | . | . | . | . | . | . | . | . | . | . | . | . | . | . | . |
| 21-225_196H12_LC | . | . | | | | . | . | . | . | . | . | . | . | . | . | Y | P | . | . | . | . | . | . | . | . | . |
| 21-225_197F4_LC | . | . | | . | . | . | . | . | . | . | . | . | . | . | . | . | . | . | . | . | . | . | . | . | . | . |
| 21-225_198B1_LC | . | . | | . | . | . | . | . | . | . | . | . | . | . | . | . | . | . | . | . | . | . | . | . | . | . |
| 21-225_198C2_LC | . | . | | . | G | . | . | . | . | . | . | . | . | . | . | F | . | . | . | S | . | . | . | . | . | . |
| 21-225_198E1_LC | . | . | | . | . | . | . | . | . | . | . | . | . | . | . | F | . | . | . | . | . | . | . | . | . | . |
| 21-225_198G10_LC | . | . | | . | . | . | . | . | . | . | . | . | . | . | . | . | . | . | . | . | . | . | . | . | . | . |
| 21-225_199C2_LC | . | . | | . | . | . | . | . | . | . | . | . | . | . | . | . | . | . | . | . | . | . | . | . | . | . |
| 21-225_199C7_LC | . | . | | . | . | . | . | . | . | . | . | . | . | . | . | . | . | . | . | . | . | . | . | . | . | . |
| 21-225_199G11_LC | . | . | | . | . | . | . | . | . | . | . | . | . | . | . | F | . | . | . | . | . | . | . | . | . | . |
| 21-225_200A1_LC | S | . | . | . | . | . | . | . | . | . | . | . | . | . | . | . | . | . | . | . | C | . | L | . | . | . |
| 21-225_200F10_LC | . | . | . | . | . | . | . | . | . | . | . | . | . | . | . | . | . | . | . | . | . | . | . | . | . | . |
| 21-225_201B2_LC | . | . | . | . | . | . | . | . | . | . | . | . | . | . | . | . | . | . | . | . | . | . | . | . | . | . |
| 21-225_201E1_LC | . | . | . | . | . | . | . | . | . | . | . | . | . | . | . | . | . | . | . | . | . | . | . | . | . | . |
| 21-225_202D11_LC | . | V | . | . | . | . | . | . | . | . | . | . | . | . | . | . | . | . | . | . | . | . | . | . | . | . |
| 21-225_202D9_LC | . | . | . | . | . | . | . | . | . | . | . | . | . | . | . | . | . | . | . | . | . | . | . | . | . | . |
| 21-225_205G7_LC | . | . | . | . | . | . | . | . | . | . | . | . | . | . | . | . | . | . | A | . | . | . | . | . | . | . |
| 21-225_208A9_LC | . | V | . | . | . | . | . | . | . | . | . | . | . | . | . | . | . | . | . | . | . | . | . | . | . | . |
| 21-225_208B5_LC | . | . | . | . | . | . | . | . | . | . | . | . | . | . | . | . | . | . | . | . | . | . | . | . | . | . |
| 21-225_220E1_LC | . | . | . | L | . | V | . | . | . | . | . | . | . | . | T | . | . | . | . | . | . | . | . | . | . | . |
| 21-225_222C7_LC | . | . | . | . | . | . | . | . | . | . | . | . | . | . | . | . | . | . | . | . | . | . | . | . | . | . |
| 21-225_225E7_LC | . | . | . | . | . | . | . | . | . | . | . | . | . | . | . | . | . | . | . | . | . | . | . | . | . | . |
| 21-225_23B9_LC | . | . | . | . | . | . | . | . | . | . | . | . | . | . | . | . | . | . | . | S | . | . | . | . | . | . |
| 21-225_44F6_LC | . | . | . | . | . | . | . | . | . | . | . | . | . | . | . | . | . | . | . | . | . | . | . | . | . | . |
| 21-225_59B2_LC | . | . | . | . | . | . | . | . | . | . | . | . | . | . | . | . | . | . | . | . | . | . | . | . | . | . |
| 21-225_74A3_LC | . | . | . | . | . | . | . | . | . | . | . | . | . | . | . | . | . | . | S | . | . | . | . | . | . | . |
| 21-225_74A4_LC | . | . | . | . | . | . | . | . | . | . | . | . | . | . | . | . | . | . | S | . | . | . | . | . | . | . |

4405

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | K_CDR2 | | | | | | | | | | | | |
| CONSENSUS | R | L | L | I | Y | G | - | - | - | - | - | - | - | - | A | S | S | R | A | T | G | I | P | D | R | F |
| 21-225_74B12_LC | . | . | . | . | . | . | . | . | . | . | . | . | . | . | . | . | . | . | S | . | . | . | . | . | . | . |
| 21-225_74B2_LC | . | . | . | . | . | . | . | . | . | . | . | . | . | . | . | . | . | . | . | . | . | . | . | . | . | . |
| 21-225_74C5_LC | . | . | . | . | . | . | . | . | . | . | . | . | . | . | P | . | . | . | . | . | . | . | . | . | . | V |
| 21-225_74C7_LC | . | . | . | . | . | . | . | . | . | . | . | . | . | . | . | . | . | . | . | . | . | . | . | . | . | . |
| 21-225_74D11_LC | . | . | . | . | . | . | . | . | . | . | . | . | . | . | . | . | . | . | . | . | . | . | . | . | . | . |
| 21-225_74D2_LC | . | . | . | . | . | . | . | . | . | . | . | . | . | . | . | . | . | . | P | . | . | . | . | . | . | . |
| 21-225_74D5_LC | . | . | . | . | . | . | . | . | . | . | . | . | . | . | . | . | . | . | . | . | . | . | . | . | . | . |
| 21-225_74D6_LC | . | . | . | . | . | . | . | . | . | . | . | . | . | . | . | . | . | . | . | . | . | . | . | . | . | . |
| 21-225_74F4_LC | . | . | . | . | . | . | . | . | . | . | . | . | . | . | . | . | . | . | . | . | . | . | . | . | . | . |
| 21-225_74F5_LC | . | . | . | . | . | . | . | . | . | . | . | . | . | . | . | . | . | . | S | . | . | . | . | . | . | . |
| 21-225_74G8_LC | . | . | . | . | . | . | . | . | . | . | . | . | . | . | T | . | . | . | . | . | . | . | . | . | . | . |
| 21-225_74H5_LC | . | . | . | . | . | . | . | . | . | . | . | . | . | . | . | . | . | . | . | . | . | . | . | . | . | . |
| 21-225_74H7_LC | . | . | . | . | . | . | . | . | . | . | . | . | . | . | . | . | . | . | . | . | . | . | . | . | . | . |
| 21-225_74H8_LC | . | . | . | . | . | . | . | . | . | . | . | . | . | . | . | . | . | . | . | . | . | . | . | . | . | . |
| 21-225_75A12_LC | . | . | . | . | . | . | . | . | . | . | . | . | . | . | . | . | . | . | . | . | . | . | . | . | . | . |
| 21-225_75A5_LC | . | . | . | . | . | . | . | . | . | . | . | . | . | . | . | . | . | . | . | . | . | . | . | . | . | . |
| 21-225_75A8_LC | . | . | . | . | . | . | . | . | . | . | . | . | . | . | . | . | . | . | . | . | . | . | . | . | . | . |
| 21-225_75B10_LC | . | . | . | . | . | . | . | . | . | . | . | . | . | . | . | T | . | . | . | . | . | . | . | . | . | . |
| 21-225_75C2_LC | . | . | . | . | . | . | . | . | . | . | . | . | . | . | . | . | . | . | . | . | . | . | . | . | . | . |
| 21-225_75C3_LC | . | . | . | . | . | . | . | . | . | . | . | . | . | . | . | . | . | . | . | . | . | . | . | . | . | . |
| 21-225_75D3_LC | . | . | I | . | . | . | . | . | . | . | . | . | . | . | . | . | . | . | S | . | . | . | . | . | . | . |
| 21-225_75D9_LC | . | . | . | . | . | . | . | . | . | . | . | . | . | . | . | . | . | . | . | . | . | . | . | . | . | . |
| 21-225_75F3_LC | . | . | . | . | . | . | . | . | . | . | . | . | . | . | . | . | . | . | . | . | . | . | . | . | . | . |
| 21-225_75G12_LC | . | . | . | . | . | . | . | . | . | . | . | . | . | . | . | . | . | . | . | . | . | . | . | . | . | . |
| 21-225_75G3_LC | . | . | . | . | . | . | . | . | . | . | . | . | . | . | . | . | . | . | . | . | . | . | . | . | . | . |
| 21-225_75G7_LC | . | . | . | . | . | . | . | . | . | . | . | . | . | . | . | . | . | . | . | . | . | . | . | . | . | . |
| 21-225_75G9_LC | . | . | . | . | . | . | . | . | . | . | . | . | . | . | . | . | . | . | P | . | . | . | . | . | . | . |
| 21-225_75H8_LC | . | . | . | . | . | . | . | . | . | . | . | . | . | . | . | . | . | . | . | . | . | . | . | . | . | . |
| 21-225_76A4_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | R | L | L | I | Y | G | - | - | - | - | - | - | - | - | A | S | S | R | A | T | G | I | P | D | R | F |
| 21-225_76A7_LC | . | . | . | . | . | . | . | . | . | . | . | . | . | . | . | . | . | . | . | P | . | . | . | . | . | . |
| 21-225_76C9_LC | . | . | . | . | . | . | . | . | . | . | . | . | . | . | . | . | . | . | . | . | . | . | . | . | . | . |
| 21-225_76D1_LC | . | . | . | . | . | . | . | . | . | . | . | . | . | . | . | . | . | . | . | . | . | . | . | . | . | . |
| 21-225_76E10_LC | . | . | . | . | . | . | . | . | . | . | . | . | . | . | . | . | . | . | . | . | . | . | . | . | . | . |
| 21-225_76G4_LC | . | . | . | . | . | . | . | . | . | . | . | . | . | . | . | . | . | . | . | . | . | . | . | . | . | . |
| 21-225_77A10_LC | R | . | . | . | S | . | . | . | . | . | . | . | . | . | . | . | . | . | . | . | . | . | . | . | . | . |
| 21-225_77C12_LC | . | . | . | . | . | . | . | . | . | . | . | . | . | . | . | . | . | . | . | . | . | . | . | . | . | . |
| 21-225_77C5_LC | . | . | . | . | . | . | . | . | . | . | . | . | . | . | . | . | . | . | S | . | . | . | . | . | . | . |
| 21-225_77F7_LC | . | . | I | . | . | . | . | . | . | . | . | . | . | . | . | . | . | . | . | . | . | . | . | . | . | . |
| 21-225_77G1_LC | . | . | . | . | . | . | . | . | . | . | . | . | . | . | . | . | . | . | . | . | . | . | . | . | . | . |
| 21-225_78E7_LC | . | . | . | . | . | . | . | . | . | . | . | . | . | . | . | . | . | . | . | . | . | . | . | . | . | . |
| 21-225_79F11_LC | . | . | . | . | . | . | . | . | . | . | . | . | . | . | . | . | . | . | . | . | . | . | . | . | . | . |
| 21-225_79F3_LC | . | . | . | . | . | . | . | . | . | . | . | . | . | . | . | . | . | . | S | . | . | . | . | . | . | . |
| 21-225_79G1_LC | . | . | . | . | . | . | . | . | . | . | . | . | . | . | . | . | . | . | S | . | . | . | . | . | . | . |
| 21-225_79G12_LC | . | . | . | . | . | . | . | . | . | . | . | . | . | . | . | . | . | . | S | . | . | . | . | . | . | . |
| 21-225_80A1_LC | . | . | . | . | . | . | . | . | . | . | . | . | . | . | . | . | . | . | S | . | . | . | . | . | . | . |
| 21-225_80A6_LC | . | . | . | . | . | . | . | . | . | . | . | . | . | . | . | . | . | . | . | P | . | . | . | . | . | . |
| 21-225_80B10_LC | . | . | . | . | . | . | . | . | . | . | . | . | . | . | . | . | . | . | . | P | . | . | . | . | . | . |
| 21-225_80C1_LC | . | . | . | . | . | . | . | . | . | . | . | . | . | . | . | . | . | . | S | . | . | . | . | . | . | . |
| 21-225_80D5_LC | . | . | . | . | . | . | . | . | . | . | . | . | . | . | . | . | . | . | . | . | . | . | . | . | . | . |
| 21-225_80E12_LC | . | . | . | . | . | . | . | . | . | . | . | . | . | . | . | . | . | . | . | . | . | . | . | . | . | . |
| 21-225_80E3_LC | . | . | . | . | . | . | . | . | . | . | . | . | . | . | . | . | . | . | S | . | . | . | . | . | . | . |
| 21-225_80H12_LC | . | . | . | . | . | . | . | . | . | . | . | . | . | . | . | . | . | . | S | . | . | . | . | . | . | . |
| 21-225_80H7_LC | . | . | . | . | . | . | . | . | . | . | . | . | . | . | . | . | . | . | . | . | . | . | . | . | . | . |
| 21-225_81C11_LC | . | . | . | . | . | . | . | . | . | . | . | . | . | . | . | . | . | . | S | . | . | L | . | . | . | . |
| 21-225_81C5_LC | . | . | . | . | . | . | . | . | . | . | . | . | . | . | . | . | . | . | S | . | . | . | . | . | . | . |
| 21-225_83B6_LC | . | . | . | . | . | . | . | . | . | . | . | . | . | . | . | . | . | . | Y | P | . | . | . | . | . | . |
| 21-225_83B7_LC | . | . | . | . | . | . | . | . | . | . | . | . | . | . | . | . | . | . | S | . | . | . | . | . | . | . |
| 21-225_83C10_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | R | L | L | I | Y | G | - | - | - | - | - | - | - | - | A | S | S | R | A | T | G | I | P | D | R | F |
| 21-225_83G1_LC | . | . | . | . | . | . | . | . | . | . | . | . | . | . | . | . | . | . | S | . | V | L | . | . | . | . |
| 21-225_84E12_LC | . | . | . | . | . | . | . | . | . | . | . | . | . | . | . | . | . | . | . | . | . | . | . | . | . | . |
| 21-225_85A3_LC | . | . | . | . | . | . | . | . | . | . | . | . | . | . | . | . | . | . | . | S | . | . | . | . | . | . |
| 21-225_85B4_LC | . | . | . | . | . | . | . | . | . | . | . | . | . | . | . | . | . | . | . | . | . | . | . | . | . | . |
| 21-225_85B9_LC | . | . | . | . | . | . | . | . | . | . | . | . | . | . | . | . | . | . | . | . | . | . | . | . | . | . |
| 21-225_85D6_LC | . | . | . | . | . | . | . | . | . | . | . | . | . | . | . | . | . | . | . | . | . | . | . | . | . | . |
| 21-225_85G10_LC | . | . | . | . | . | . | . | . | . | . | . | . | . | . | . | . | . | . | . | . | . | . | . | . | . | . |
| 21-225_85G6_LC | . | . | . | . | . | . | . | . | . | . | . | . | . | . | . | A | . | . | . | P | . | . | . | . | . | . |
| 21-225_86C1_LC | . | . | . | . | . | . | . | . | . | . | . | . | . | . | . | . | . | . | . | . | . | . | . | . | . | . |
| 21-225_86C11_LC | . | . | . | . | . | . | . | . | . | . | . | . | . | . | . | . | . | . | S | . | . | L | . | . | . | . |
| 21-225_86E4_LC | . | . | . | . | . | . | . | . | . | . | . | . | . | . | . | . | . | . | S | . | . | . | . | . | . | . |
| 21-225_87A12_LC | . | . | . | . | . | . | . | . | . | . | . | . | . | . | . | . | . | . | S | . | V | . | . | . | . | . |
| 21-225_87C9_LC | . | . | . | . | . | . | . | . | . | . | . | . | . | . | . | . | . | . | S | . | . | . | . | . | . | . |
| 21-225_87E5_LC | . | . | . | . | . | . | . | . | . | . | . | . | . | . | . | . | . | . | S | . | . | . | . | . | . | . |
| 21-225_87H1_LC | . | . | . | . | . | . | . | . | . | . | . | . | . | . | . | A | . | . | T | . | . | . | . | . | . | . |
| 21-225_88E7_LC | . | . | . | . | . | . | . | . | . | . | . | . | . | . | . | . | . | . | S | . | . | . | . | . | . | . |
| 21-225_88F7_LC | . | . | . | . | . | . | . | . | . | . | . | . | . | . | . | . | . | . | S | . | . | . | . | . | . | . |
| 21-225_88G9_LC | . | . | . | . | . | . | . | . | . | . | . | . | . | . | . | . | . | . | S | . | . | . | . | . | . | . |
| 21-225_88H1_LC | . | . | . | . | . | . | . | . | . | . | . | . | . | . | . | . | . | . | S | . | V | . | . | . | . | . |
| 21-225_89A11_LC | . | . | . | . | . | . | . | . | . | . | . | . | . | . | . | A | . | . | T | . | . | . | . | . | . | . |
| 21-225_89D5_LC | . | . | . | . | . | . | . | . | . | . | . | . | . | . | . | . | . | . | S | . | . | . | . | . | . | . |
| 21-225_89E10_LC | . | . | . | . | . | . | . | . | . | . | . | . | . | . | . | . | . | . | S | . | V | . | . | . | . | . |
| 21-225_89H5_LC | . | . | . | . | . | . | . | . | . | . | . | . | . | . | R | . | . | . | . | . | . | . | . | . | . | V |
| 21-225_90A5_LC | . | . | . | . | . | . | . | . | . | . | . | . | . | . | . | . | . | . | . | . | . | . | . | . | . | . |
| 21-225_90F10_LC | . | . | . | . | . | . | . | . | . | . | . | . | . | . | . | . | . | . | S | . | . | . | . | . | . | . |
| 21-225_90G4_LC | . | . | . | . | . | . | . | . | . | . | . | . | . | . | . | . | . | . | S | . | V | . | . | . | . | . |
| 21-225_90G5_LC | . | . | I | . | . | . | . | . | . | . | . | . | . | . | . | . | . | . | . | . | . | . | . | . | . | . |
| 21-225_91B2_LC | . | . | . | . | . | . | . | . | . | . | . | . | . | . | . | . | . | . | S | . | . | . | . | . | . | . |
| 21-225_91B3_LC | . | . | . | . | . | . | . | . | . | . | . | . | . | . | . | . | . | . | S | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | R | L | L | I | Y | G | - | - | - | - | - | - | - | - | A | S | S | R | A | T | G | I | P | D | R | F |
| 21-225_91B4_LC | . | . | ¦ | . | . | | | | . | | . | | . | . | . | . | . | . | S | . | . | . | . | . | . | . |
| 21-225_91E9_LC | . | . | . | . | . | | | | . | | . | | . | . | . | . | . | . | . | . | . | . | . | . | . | . |
| 21-225_91F3_LC | . | . | . | . | . | | | | . | | . | | . | . | . | . | . | . | S | . | . | . | . | . | . | . |
| 21-225_92B1_LC | . | . | . | . | . | | | . | | . | | . | | . | . | . | . | . | . | . | G | . | . | . | . | . |
| 21-225_92D6_LC | . | . | . | . | . | | | | . | | . | | . | . | . | . | . | . | S | . | V | . | . | . | . | . |
| 21-225_93C2_LC | . | . | . | . | . | | | | . | | . | | . | . | . | . | . | . | . | . | . | . | . | . | . | . |
| 21-225_93E4_LC | . | . | . | . | . | | | | . | | . | | . | . | . | . | . | . | S | . | . | . | . | . | . | . |
| 21-225_93E9_LC | . | . | . | . | . | | | . | | . | | . | | . | . | . | . | . | . | S | . | . | . | . | . | . |
| 21-225_94D3_LC | . | . | . | . | . | | | . | | . | | . | | . | . | . | . | . | . | . | . | . | . | . | . | . |
| 21-225_94F12_LC | . | . | . | . | . | | | . | | . | | . | | . | . | . | . | . | S | . | . | . | . | . | . | . |
| 21-225_94G10_LC | . | . | . | . | . | | | . | | . | | . | | . | . | . | . | . | . | . | . | . | . | . | . | . |
| 21-225_95D2_LC | . | . | . | . | . | | | . | | . | | . | | . | . | . | . | . | . | . | . | . | . | . | . | . |
| 21-225_95F9_LC | . | . | . | . | . | | | . | | . | | . | | . | . | . | . | . | S | . | . | . | . | . | . | . |
| 21-225_95G9_LC | . | . | . | . | . | | | . | | . | | . | | . | . | . | . | . | S | . | . | . | . | . | . | . |
| 21-225_95H10_LC | . | . | . | . | . | | | . | | . | | . | | . | . | . | . | . | . | . | . | . | . | . | . | . |
| 21-225_97A2_LC | . | . | . | . | . | | | . | | . | | . | | . | . | . | . | . | S | . | . | . | . | . | . | . |
| 21-225_97E5_LC | . | . | . | . | . | | | . | | . | | . | | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | R | L | E | P | E | D | F | A | V | Y |
| 21-225_147G8_LC | . | . | . | . | . | . | . | . | . | . | . | . | . | . | . | Y | . | . | . | . | . | . | . | . | . | . |
| 21-225_190A3_LC | . | . | . | V | . | . | . | . | . | . | . | . | . | . | . | N | . | . | . | . | . | . | . | . | . | . |
| 21-225_190A7_LC | . | . | . | . | . | . | . | . | . | . | . | . | . | . | . | N | . | . | . | . | . | . | . | . | . | . |
| 21-225_190B4_LC | . | . | . | . | . | . | . | . | . | . | . | . | . | . | . | . | . | . | . | . | . | . | . | . | . | . |
| 21-225_190G10_LC | . | . | . | . | . | . | . | . | . | . | . | . | . | . | N | . | . | . | . | . | . | . | . | . | . | . |
| 21-225_191A3_LC | . | . | . | . | . | . | . | . | . | . | . | . | . | . | . | . | . | . | . | . | . | . | . | . | . | . |
| 21-225_191A5_LC | . | . | . | . | . | . | . | . | . | . | . | . | . | . | . | . | . | . | . | . | . | . | . | . | . | . |
| 21-225_191C3_LC | . | . | . | . | . | . | . | . | . | . | . | . | . | . | . | . | . | . | . | . | . | . | . | . | . | . |
| 21-225_191D3_LC | . | . | . | . | . | . | . | . | . | . | . | . | . | . | . | N | . | . | . | . | . | . | . | . | . | . |
| 21-225_191F1_LC | . | . | . | . | . | . | . | . | . | . | . | . | . | . | . | N | . | . | . | . | . | . | . | . | . | . |
| 21-225_191H7_LC | . | . | . | . | . | . | . | . | . | . | . | . | . | . | . | N | . | . | . | . | . | . | . | . | . | . |
| 21-225_192B3_LC | . | . | . | . | . | . | . | . | . | . | . | . | . | . | . | . | . | . | . | . | . | . | . | . | . | . |
| 21-225_192F8_LC | . | . | . | . | . | . | . | . | . | . | . | . | . | . | . | . | . | . | . | . | . | . | . | . | . | . |
| 21-225_192G12_LC | . | . | . | . | . | . | . | . | . | . | . | . | . | . | . | N | . | . | . | . | . | . | . | . | . | . |
| 21-225_192H3_LC | . | . | . | . | . | . | . | . | . | . | . | . | . | . | . | N | . | . | . | . | . | . | . | . | . | . |
| 21-225_193A1_LC | . | . | . | . | . | . | . | . | . | . | . | . | . | . | . | N | . | . | . | . | . | . | . | . | . | . |
| 21-225_193B1_LC | . | . | . | . | . | . | . | . | . | . | . | . | . | . | . | N | . | . | . | . | . | . | . | . | . | . |
| 21-225_193B10_LC | R | . | . | . | . | . | . | . | . | . | . | . | . | . | . | . | . | . | . | . | . | . | . | . | . | . |
| 21-225_193B12_LC | . | . | . | . | . | . | . | . | . | . | . | . | . | . | . | . | . | . | . | . | . | . | . | . | . | . |
| 21-225_193C8_LC | . | . | . | . | . | . | . | . | . | . | . | . | . | . | . | . | . | . | . | . | . | . | . | . | . | . |
| 21-225_193D3_LC | . | . | . | . | . | . | . | . | . | . | . | . | . | . | . | . | . | . | . | . | . | . | . | . | . | . |
| 21-225_193D8_LC | . | . | . | . | . | . | . | . | . | . | . | . | . | . | . | N | . | . | . | . | . | . | . | . | . | . |
| 21-225_193E6_LC | G | . | . | . | . | . | . | . | . | . | . | . | . | . | . | . | . | . | . | . | . | . | . | . | . | . |
| 21-225_193F3_LC | . | . | . | . | . | . | . | . | . | . | . | . | . | . | . | N | . | . | . | . | . | . | . | . | . | . |
| 21-225_193G8_LC | . | . | . | . | . | . | . | . | . | . | . | . | . | . | . | N | . | . | . | . | . | . | . | L | . | . |
| 21-225_193H6_LC | . | . | . | . | . | . | . | . | . | . | . | . | . | . | . | N | . | . | . | . | . | . | . | . | . | . |
| 21-225_194C10_LC | . | A | . | . | . | . | . | . | A | . | . | . | . | . | . | . | . | . | . | . | . | . | . | . | . | . |
| 21-225_194E5_LC | . | . | . | . | . | . | . | . | . | . | . | . | . | . | . | N | . | . | . | K | . | . | . | . | . | . |
| 21-225_194E6_LC | . | . | . | V | . | . | . | . | . | . | . | . | . | . | . | N | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | R | L | E | P | E | D | F | A | V | Y |
| 21-225_195B1_LC | . | A | . | . | . | . | . | . | . | . | . | . | . | . | . | G | . | . | . | . | . | . | . | . | . | . |
| 21-225_195C8_LC | . | . | . | . | . | . | . | . | . | . | . | . | . | . | . | . | . | . | . | . | . | . | . | . | . | . |
| 21-225_196G10_LC | . | . | . | . | . | . | . | . | . | . | . | . | . | . | N | . | . | . | . | . | . | . | . | . | . | . |
| 21-225_196H12_LC | . | . | . | . | . | . | . | . | . | . | . | . | . | . | . | N | . | . | . | . | . | . | . | . | . | . |
| 21-225_197F4_LC | . | . | . | . | . | . | . | . | . | . | . | . | . | . | . | . | . | . | . | . | . | . | . | . | . | . |
| 21-225_198B1_LC | . | . | . | . | . | . | . | . | . | . | . | . | . | . | . | N | . | . | . | . | . | . | . | . | . | . |
| 21-225_198C2_LC | . | . | . | . | . | . | . | . | . | . | . | . | . | . | . | . | . | . | . | . | . | . | . | . | . | . |
| 21-225_198E1_LC | . | . | . | . | . | . | . | . | . | . | . | . | . | . | . | N | . | . | . | . | . | . | . | . | . | . |
| 21-225_198G10_LC | . | . | . | . | . | . | . | . | . | . | . | . | . | . | . | . | . | . | . | . | . | . | . | . | . | . |
| 21-225_199C2_LC | . | . | . | . | . | . | . | . | . | . | . | . | . | . | . | N | . | . | . | . | . | . | . | . | . | . |
| 21-225_199C7_LC | . | . | . | . | . | . | . | . | . | . | . | . | . | . | . | N | . | . | . | . | . | . | . | . | . | . |
| 21-225_199G11_LC | . | . | . | . | . | . | . | . | . | . | . | . | . | . | . | . | . | . | . | . | . | . | . | . | . | . |
| 21-225_200A1_LC | . | . | . | C | . | . | . | . | . | . | . | . | . | . | . | . | . | . | . | . | . | . | . | . | . | . |
| 21-225_200F10_LC | . | . | . | . | . | . | . | . | . | . | . | . | . | . | . | N | . | . | . | . | . | . | . | . | . | . |
| 21-225_201B2_LC | . | . | . | . | . | . | . | . | . | . | . | . | . | . | . | . | . | . | . | . | . | . | . | . | . | . |
| 21-225_201E1_LC | . | . | . | . | . | . | . | . | . | . | . | . | . | . | . | . | . | . | . | . | . | . | . | . | M | F |
| 21-225_202D11_LC | . | . | . | . | . | . | . | . | . | . | . | . | . | . | . | . | . | . | . | . | . | . | . | . | . | F |
| 21-225_202D9_LC | . | . | . | . | . | . | . | . | . | . | . | . | . | . | . | . | . | . | . | . | . | . | . | . | . | G |
| 21-225_205G7_LC | . | . | . | . | . | . | . | . | . | . | . | . | . | . | . | . | . | . | . | . | N | . | . | . | . | . |
| 21-225_208A9_LC | . | . | . | . | . | . | . | . | . | . | . | . | . | . | . | . | . | . | . | . | . | . | . | . | . | . |
| 21-225_208B5_LC | . | . | . | . | . | . | . | . | . | . | . | . | . | . | . | . | . | . | . | . | . | . | . | . | . | . |
| 21-225_220E1_LC | . | . | . | . | . | . | . | . | . | . | . | . | . | . | . | R | S | . | . | . | . | . | . | . | M | . |
| 21-225_222C7_LC | G | . | . | . | . | . | . | . | . | . | . | . | . | A | . | . | . | . | . | . | . | . | . | T | I | . |
| 21-225_225E7_LC | . | . | . | . | . | . | . | . | . | . | . | . | . | . | . | . | . | . | . | . | . | . | . | . | . | . |
| 21-225_23B9_LC | . | . | . | . | . | . | . | . | . | . | . | . | . | . | . | . | . | . | . | . | . | . | . | . | . | . |
| 21-225_44F6_LC | . | . | . | . | . | . | . | . | . | . | . | . | . | . | . | . | . | . | . | . | . | . | . | . | . | . |
| 21-225_59B2_LC | . | . | . | . | . | . | . | . | . | . | . | . | . | . | . | . | . | . | . | . | . | . | . | . | . | F |
| 21-225_74A3_LC | . | . | . | C | . | . | . | . | . | A | . | . | . | . | . | . | . | . | . | . | . | . | . | . | . | . |
| 21-225_74A4_LC | . | . | . | C | . | . | . | . | . | A | . | . | . | . | . | . | . | . | . | . | . | . | C | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | D | F | T | L | T | I | S | R | L | E | P | E | D | F | A | V | Y |
| 21-225_74B12_LC | . | . | . | . | . | . | . | . | . | . | . | A | . | . | . | . | . | . | . | . | . | . | . | . | . | . |
| 21-225_74B2_LC | . | . | . | . | . | . | . | . | . | . | . | . | . | . | . | . | . | . | . | . | . | . | . | . | . | . |
| 21-225_74C5_LC | . | . | . | . | . | . | . | . | . | . | . | N | . | I | . | . | . | . | . | . | . | . | . | . | . | . |
| 21-225_74C7_LC | . | . | . | . | . | . | . | . | . | . | . | . | . | . | . | . | . | . | . | . | . | . | . | . | . | F |
| 21-225_74D11_LC | . | . | . | . | . | . | . | . | . | . | . | A | . | . | . | . | . | . | . | . | . | . | . | . | . | . |
| 21-225_74D2_LC | . | . | . | . | . | . | . | . | . | . | . | . | . | . | . | N | . | . | . | . | . | . | . | . | . | . |
| 21-225_74D5_LC | . | . | . | . | . | . | . | . | . | . | . | I | . | I | . | . | . | . | . | . | . | . | . | . | . | . |
| 21-225_74D6_LC | . | . | . | . | C | . | . | . | . | . | . | A | . | . | . | . | . | . | . | . | . | . | . | . | . | . |
| 21-225_74F4_LC | . | . | . | . | . | . | . | . | . | . | . | . | . | . | . | . | . | . | . | . | . | . | . | . | . | . |
| 21-225_74F5_LC | . | . | . | . | . | . | . | . | . | . | . | A | . | . | . | . | . | . | . | . | . | . | . | . | . | . |
| 21-225_74G8_LC | . | . | . | . | . | . | . | . | . | . | . | . | . | . | . | G | . | . | . | . | . | . | . | . | . | . |
| 21-225_74H5_LC | . | . | . | . | . | . | . | . | . | . | . | I | . | I | . | . | . | . | . | . | . | . | . | . | . | . |
| 21-225_74H7_LC | . | . | . | . | . | . | . | . | . | . | . | . | . | . | . | . | . | . | . | . | . | . | . | . | . | . |
| 21-225_74H8_LC | . | . | . | . | . | . | . | . | . | . | . | . | . | . | . | . | . | . | . | . | . | . | . | . | . | . |
| 21-225_75A12_LC | . | . | . | . | . | . | . | . | . | . | . | . | . | . | . | . | . | . | . | . | . | A | . | . | . | . |
| 21-225_75A5_LC | . | . | . | . | . | . | . | . | . | . | . | A | . | . | . | . | . | . | . | . | . | . | . | . | . | . |
| 21-225_75A8_LC | . | . | . | . | . | . | . | . | . | . | . | A | . | . | . | . | . | V | . | . | . | . | C | . | . | . |
| 21-225_75B10_LC | . | . | . | . | . | . | . | . | . | . | . | . | . | . | . | . | . | . | . | . | . | . | . | . | . | . |
| 21-225_75C2_LC | . | . | . | . | . | . | . | . | . | . | . | A | . | . | . | . | . | . | . | . | . | . | C | . | . | . |
| 21-225_75C3_LC | . | . | . | . | . | . | . | . | . | . | . | . | . | . | . | . | . | . | . | . | . | . | . | . | . | . |
| 21-225_75D3_LC | . | . | . | . | C | . | . | . | . | . | . | A | . | . | . | . | . | V | . | H | . | . | C | . | . | . |
| 21-225_75D9_LC | . | . | . | . | . | . | . | . | . | . | . | . | . | . | . | . | . | . | . | . | . | . | . | . | . | . |
| 21-225_75F3_LC | . | . | . | . | C | . | . | . | . | . | . | A | . | . | . | . | . | . | . | H | . | . | . | . | . | . |
| 21-225_75G12_LC | . | . | . | . | . | . | . | . | . | . | . | A | . | . | . | . | . | V | . | . | . | . | C | . | . | . |
| 21-225_75G3_LC | . | . | . | . | . | . | . | . | . | . | . | . | . | . | . | . | . | . | . | . | . | . | . | . | . | F |
| 21-225_75G7_LC | . | . | . | . | . | . | . | . | . | . | . | . | . | . | . | . | . | . | . | . | . | . | . | . | . | . |
| 21-225_75G9_LC | . | . | . | . | . | . | . | . | . | . | . | . | . | . | . | . | . | . | . | . | . | . | . | . | . | . |
| 21-225_75H8_LC | . | . | . | . | . | . | . | . | . | . | . | . | . | . | . | . | . | . | . | . | . | . | . | . | . | . |
| 21-225_76A4_LC | . | . | . | . | . | . | . | . | . | . | . | A | . | . | . | . | . | . | . | . | . | . | . | . | . | . |

# EP 4 435 105 A2

## FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | R | L | E | P | E | D | F | A | V | Y |
| 21-225_76A7_LC | . | . | . | . | . | . | . | . | . | . | . | . | . | . | . | . | . | . | . | . | . | . | . | . | . | . |
| 21-225_76C9_LC | . | . | . | . | . | . | . | . | . | . | . | . | . | . | . | . | . | . | . | . | . | . | . | . | . | . |
| 21-225_76D1_LC | . | . | . | . | . | . | . | . | . | . | . | . | . | . | . | . | . | . | . | . | . | . | . | V | . | . |
| 21-225_76E10_LC | . | . | . | . | . | . | . | . | . | . | I | . | I | . | . | . | . | . | . | . | . | . | . | . | . | . |
| 21-225_76G4_LC | . | . | . | . | . | . | . | . | . | . | A | . | . | . | . | . | . | . | . | . | . | . | C | . | . | . |
| 21-225_77A10_LC | . | . | . | . | . | . | . | . | . | . | . | . | . | . | . | . | . | . | . | . | . | . | . | . | . | . |
| 21-225_77C12_LC | . | . | . | . | . | . | . | . | . | . | . | . | . | . | . | . | . | . | . | . | . | . | . | . | . | . |
| 21-225_77C5_LC | . | . | . | . | . | . | . | . | . | . | A | . | . | . | . | . | . | . | . | . | . | . | . | . | . | . |
| 21-225_77F7_LC | . | . | . | . | C | . | . | . | . | . | A | . | . | . | . | . | . | V | . | B | . | . | C | . | . | . |
| 21-225_77G1_LC | . | . | . | . | . | . | . | . | . | . | A | . | . | . | . | . | . | . | . | . | . | . | C | . | . | . |
| 21-225_78E7_LC | . | . | . | . | . | . | . | . | . | . | . | . | . | . | . | . | . | . | . | . | . | . | . | . | . | F |
| 21-225_79F11_LC | . | . | . | . | . | . | . | . | . | . | A | . | . | . | . | . | . | . | . | . | . | . | . | . | . | . |
| 21-225_79F3_LC | . | . | . | . | . | . | . | . | . | . | A | . | . | . | . | . | . | . | . | . | . | . | . | . | . | . |
| 21-225_79G1_LC | . | . | . | . | . | . | . | . | . | . | A | . | . | . | . | . | . | . | . | . | . | . | . | . | . | . |
| 21-225_79G12_LC | . | . | . | . | . | . | . | . | . | . | A | . | . | . | . | . | . | . | . | . | . | . | C | . | . | . |
| 21-225_80A1_LC | . | . | . | . | C | . | . | . | . | . | A | . | . | . | . | . | . | V | . | . | . | . | C | . | . | . |
| 21-225_80A6_LC | . | . | . | . | . | . | . | . | . | . | . | . | . | . | . | . | . | . | . | . | . | . | . | . | . | . |
| 21-225_80B10_LC | . | . | . | . | . | . | . | . | . | . | . | . | . | . | . | . | . | . | . | . | . | . | . | . | . | . |
| 21-225_80C1_LC | . | . | . | . | . | . | . | . | . | . | A | . | . | . | . | . | . | V | . | . | . | . | C | . | . | . |
| 21-225_80D5_LC | . | . | . | . | . | . | . | . | . | . | . | . | . | . | . | T | . | . | . | . | . | . | . | . | . | . |
| 21-225_80E12_LC | . | . | . | . | . | . | . | . | . | . | I | . | I | . | . | . | . | . | . | . | . | . | . | . | . | . |
| 21-225_80E3_LC | . | . | . | . | C | . | . | . | . | . | A | . | . | . | . | . | . | V | . | . | . | . | . | . | . | . |
| 21-225_80H12_LC | . | . | . | . | . | . | . | . | . | . | A | . | . | . | . | . | . | . | . | . | . | . | . | . | . | . |
| 21-225_80H7_LC | . | . | . | . | . | . | . | . | . | . | . | . | . | . | . | . | . | . | . | . | . | . | . | . | . | F |
| 21-225_81C11_LC | . | . | . | . | C | . | . | . | . | . | A | . | . | . | . | . | . | V | . | . | . | . | C | . | . | . |
| 21-225_81C5_LC | . | . | . | . | C | . | . | . | . | . | A | . | . | . | . | . | . | V | . | . | . | . | C | . | . | . |
| 21-225_83B6_LC | . | . | . | . | . | . | . | . | . | . | . | . | . | . | . | . | . | . | . | . | . | . | . | . | . | . |
| 21-225_83B7_LC | . | . | . | . | . | . | . | . | . | . | A | . | . | . | . | . | . | . | . | . | . | . | C | . | . | . |
| 21-225_83C10_LC | . | . | . | . | . | . | . | . | . | . | I | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | K_FR3 | | | | | | | | | | | | | |
| CONSENSUS | S | G | S | G | S | G | - | - | T | D | F | T | L | T | I | S | R | L | E | P | E | D | F | A | V | Y |
| 21-225_83G1_LC | . | . | . | . | . | . | . | . | . | . | . | A | . | . | . | . | . | V | . | . | . | . | C | . | . | . |
| 21-225_84E12_LC | . | . | . | . | . | . | . | . | . | . | . | . | . | . | . | . | . | . | . | . | C | . | . | . | . | F |
| 21-225_85A3_LC | . | . | . | . | . | . | . | . | . | . | . | A | . | . | . | . | . | . | . | . | . | . | . | . | . | . |
| 21-225_85B4_LC | . | . | . | . | . | . | . | . | . | . | . | A | . | . | . | . | . | . | . | . | . | . | . | . | . | . |
| 21-225_85B9_LC | . | . | . | . | . | . | . | . | . | . | I | . | . | I | . | . | . | . | . | . | . | . | . | . | . | . |
| 21-225_85D6_LC | . | . | . | . | . | . | . | . | . | . | . | . | . | . | . | . | . | . | . | . | . | . | . | . | . | . |
| 21-225_85G10_LC | . | . | . | . | . | . | . | . | . | . | . | . | . | . | . | . | . | . | . | . | . | . | . | . | . | . |
| 21-225_85G6_LC | . | . | . | . | . | . | . | . | . | . | . | . | . | . | . | . | . | . | . | . | . | . | . | . | V | . |
| 21-225_86C1_LC | . | . | . | . | . | . | . | . | . | . | . | A | . | . | . | . | . | . | . | . | . | . | C | . | . | . |
| 21-225_86C11_LC | . | . | . | . | . | . | . | . | . | . | . | A | . | . | . | . | . | . | . | . | . | . | C | . | . | . |
| 21-225_86E4_LC | . | . | . | . | . | . | . | . | . | . | . | A | . | . | . | . | . | . | . | . | . | . | C | . | . | . |
| 21-225_87A12_LC | . | . | . | . | C | . | . | . | . | . | . | A | . | . | . | . | . | V | . | . | . | . | C | . | . | . |
| 21-225_87C9_LC | . | . | . | . | . | . | . | . | . | . | . | A | . | . | . | . | . | . | . | . | . | . | C | . | . | . |
| 21-225_87E5_LC | . | . | . | . | . | . | . | . | . | . | . | A | . | . | . | . | . | V | . | . | . | . | C | . | . | . |
| 21-225_87H1_LC | . | . | . | . | . | . | . | . | . | . | . | . | . | . | . | . | . | . | . | . | . | . | . | . | . | . |
| 21-225_88E7_LC | . | . | . | . | . | . | . | . | . | . | . | A | . | . | . | . | . | V | . | . | . | . | C | . | . | . |
| 21-225_88F7_LC | . | . | . | . | . | . | . | . | . | . | . | A | . | . | . | . | . | . | . | . | . | . | C | . | . | . |
| 21-225_88G9_LC | . | . | . | . | . | . | . | . | . | . | . | A | . | . | . | . | . | V | . | . | . | . | C | . | . | . |
| 21-225_88H1_LC | . | . | . | . | G | . | . | . | . | . | . | A | . | . | . | . | . | V | . | . | . | . | C | . | . | . |
| 21-225_89A11_LC | . | . | . | . | . | . | . | . | . | . | . | . | . | . | . | . | . | . | . | . | . | . | . | . | . | . |
| 21-225_89D5_LC | . | . | . | . | . | . | . | . | . | . | . | A | . | . | . | . | . | . | . | . | . | . | . | . | . | . |
| 21-225_89E10_LC | . | . | . | . | . | . | . | . | . | . | . | A | . | . | . | . | . | V | . | . | . | . | . | . | . | . |
| 21-225_89H5_LC | . | . | . | . | . | . | . | . | . | . | . | N | . | I | . | . | . | . | . | . | . | . | . | . | . | . |
| 21-225_90A5_LC | . | . | . | . | . | . | . | . | . | . | . | A | . | . | . | . | . | V | . | H | . | . | . | . | . | . |
| 21-225_90F10_LC | . | . | . | . | . | . | . | . | . | . | . | A | . | . | . | . | . | . | . | . | . | . | C | . | . | . |
| 21-225_90G4_LC | . | . | . | . | . | . | . | . | . | . | . | A | . | . | . | . | . | V | . | . | . | . | C | . | . | . |
| 21-225_90G5_LC | . | . | . | . | . | . | . | . | . | . | . | A | . | . | . | . | . | V | . | H | . | . | . | . | . | . |
| 21-225_91B2_LC | . | . | . | . | . | . | . | . | . | . | . | A | . | . | . | . | . | V | . | . | . | . | C | . | . | . |
| 21-225_91B3_LC | . | . | . | . | . | . | . | . | . | . | . | A | . | . | . | . | . | V | . | H | . | . | C | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | D | F | T | L | T | I | S | R | L | E | P | E | D | F | A | V | Y |
| 21-225_91B4_LC | . | . | . | . | . | . | | | . | . | . | A | . | . | . | . | . | V | . | . | . | . | C | . | . | . |
| 21-225_91E9_LC | . | . | . | C | . | | | | . | . | . | A | . | . | . | . | . | V | . | B | . | . | C | . | . | . |
| 21-225_91F3_LC | . | . | . | . | . | . | | | . | . | . | A | . | . | . | . | . | V | . | . | . | . | C | . | . | . |
| 21-225_92B1_LC | . | . | . | . | . | . | | | . | . | . | . | . | . | . | . | . | . | . | . | . | . | . | . | . | . |
| 21-225_92D6_LC | . | . | . | . | C | . | | | . | . | . | A | . | . | . | . | . | . | . | . | . | . | C | . | . | . |
| 21-225_93C2_LC | . | . | . | . | . | . | | | . | . | . | A | . | . | . | . | . | . | . | B | . | . | C | . | . | . |
| 21-225_93E4_LC | . | . | . | C | . | | | | . | . | . | A | . | . | . | . | . | V | . | . | . | . | . | . | . | . |
| 21-225_93E9_LC | . | . | . | . | . | . | | | . | . | . | . | . | . | . | . | . | . | . | . | . | . | . | . | . | . |
| 21-225_94D3_LC | . | . | . | . | . | . | | | . | . | . | . | . | . | . | . | . | . | . | . | . | . | . | . | . | . |
| 21-225_94F12_LC | . | . | . | . | . | . | | | . | . | . | A | . | . | . | . | . | . | . | . | . | . | . | . | . | . |
| 21-225_94G10_LC | . | . | . | . | . | . | | | . | . | . | I | . | I | . | . | . | . | . | . | . | . | . | . | . | . |
| 21-225_95D2_LC | . | . | . | . | . | . | | | . | . | . | A | . | . | . | . | . | . | . | . | . | . | C | . | . | . |
| 21-225_95F9_LC | . | . | . | C | . | | | | . | . | . | A | . | . | . | . | . | . | . | . | . | . | . | . | . | . |
| 21-225_95G9_LC | . | . | . | . | . | | | | . | . | . | A | . | . | . | . | . | V | . | B | . | . | C | . | . | . |
| 21-225_95H10_LC | . | . | . | . | . | . | | | . | . | . | A | . | . | . | . | . | . | . | . | . | . | . | . | . | . |
| 21-225_97A2_LC | . | . | . | C | . | | | | . | . | . | A | . | . | . | . | . | . | . | . | . | . | C | . | . | . |
| 21-225_97E5_LC | . | . | . | C | . | | | | . | . | . | A | . | . | . | . | . | V | . | . | . | . | C | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | D | N | - | - | - | - | - | - | - | - | - | - | - | - | - | - | - | - | - | - | - |
| 21-225_147G8_LC | . | . | . | . | . | . | S | . | . | . | . | . | . | . | . | . | . | . | . | . | . | . | . | . | . | . |
| 21-225_190A3_LC | . | . | . | . | . | G | . | . | . | . | . | . | . | . | . | . | . | . | . | . | . | . | . | . | . | . |
| 21-225_190A7_LC | . | . | . | . | . | G | . | . | . | . | . | . | . | . | . | . | . | . | . | . | . | . | . | . | . | . |
| 21-225_190B4_LC | . | . | . | . | . | G | . | . | . | . | . | . | . | . | . | . | . | . | . | . | . | . | . | . | . | . |
| 21-225_190G10_LC | . | . | . | . | . | G | S | . | . | . | . | . | . | . | . | . | . | . | . | . | . | . | . | . | . | . |
| 21-225_191A3_LC | . | . | . | . | . | G | . | . | . | . | . | . | . | . | . | . | . | . | . | . | . | . | . | . | . | . |
| 21-225_191A5_LC | . | . | . | . | . | G | G | . | . | . | . | . | . | . | . | . | . | . | . | . | . | . | . | . | . | . |
| 21-225_191C3_LC | . | . | . | . | . | G | . | . | . | . | . | . | . | . | . | . | . | . | . | . | . | . | . | . | . | . |
| 21-225_191D3_LC | . | . | . | . | . | G | S | . | . | . | . | . | . | . | . | . | . | . | . | . | . | . | . | . | . | . |
| 21-225_191F1_LC | . | . | . | . | . | G | . | . | . | . | . | . | . | . | . | . | . | . | . | . | . | . | . | . | . | . |
| 21-225_191H7_LC | F | . | . | . | . | G | S | . | . | . | . | . | . | . | . | . | . | . | . | . | . | . | . | . | . | . |
| 21-225_192B3_LC | . | . | . | . | . | G | . | . | . | . | . | . | . | . | . | . | . | . | . | . | . | . | . | . | . | . |
| 21-225_192F8_LC | . | . | . | . | . | E | S | . | . | . | . | . | . | . | . | . | . | . | . | . | . | . | . | . | . | . |
| 21-225_192G12_LC | . | . | . | . | . | G | . | . | . | . | . | . | . | . | . | . | . | . | . | . | . | . | . | . | . | . |
| 21-225_192H3_LC | . | . | . | . | . | G | I | . | . | . | . | . | . | . | . | . | . | . | . | . | . | . | . | . | . | . |
| 21-225_193A1_LC | . | . | . | . | . | G | . | . | . | . | . | . | . | . | . | . | . | . | . | . | . | . | . | . | . | . |
| 21-225_193B1_LC | F | . | . | . | . | G | . | . | . | . | . | . | . | . | . | . | . | . | . | . | . | . | . | . | . | . |
| 21-225_193B10_LC | . | . | . | . | . | G | S | . | . | . | . | . | . | . | . | . | . | . | . | . | . | . | . | . | . | . |
| 21-225_193B12_LC | . | . | . | . | . | G | . | . | . | . | . | . | . | . | . | . | . | . | . | . | . | . | . | . | . | . |
| 21-225_193C8_LC | . | . | . | . | . | G | . | . | . | . | . | . | . | . | . | . | . | . | . | . | . | . | . | . | . | . |
| 21-225_193D3_LC | . | . | . | . | . | G | . | . | . | . | . | . | . | . | . | . | . | . | . | . | . | . | . | . | . | . |
| 21-225_193D8_LC | . | . | . | . | . | G | . | . | . | . | . | . | . | . | . | . | . | . | . | . | . | . | . | . | . | . |
| 21-225_193E6_LC | . | . | . | . | . | E | S | . | . | . | . | . | . | . | . | . | . | . | . | . | . | . | . | . | . | . |
| 21-225_193F3_LC | . | . | . | . | . | G | S | . | . | . | . | . | . | . | . | . | . | . | . | . | . | . | . | . | . | . |
| 21-225_193G8_LC | . | . | . | . | . | G | . | . | . | . | . | . | . | . | . | . | . | . | . | . | . | . | . | . | . | . |
| 21-225_193H6_LC | F | . | . | . | . | G | S | . | . | . | . | . | . | . | . | . | . | . | . | . | . | . | . | . | . | . |
| 21-225_194C10_LC | F | . | . | . | . | E | S | . | . | . | . | . | . | . | . | . | . | . | . | . | . | . | . | . | . | . |
| 21-225_194E5_LC | . | . | . | . | . | G | S | . | . | . | . | . | . | . | . | . | . | . | . | . | . | . | . | . | . | . |
| 21-225_194E6_LC | . | . | . | . | . | G | S | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | K_CDR3 | | | | | | | | | |
| CONSENSUS | Y | C | Q | Q | Y | D | N | - | - | - | - | - | - | - | - | - | - | - | - | - | - | - | - | - | - | - |
| 21-225_195B1_LC | F | . | | . | | E | S | . | . | . | . | . | . | . | . | . | . | . | . | . | . | . | . | . | . | . |
| 21-225_195C8_LC | . | . | | . | | G | . | . | . | . | . | . | . | . | . | . | . | . | . | . | . | . | . | . | . | . |
| 21-225_196G10_LC | . | . | | . | | G | S | . | . | . | . | . | . | . | . | . | . | . | . | . | . | . | . | . | . | . |
| 21-225_196H12_LC | . | . | | . | | G | . | . | . | . | . | . | . | . | . | . | . | . | . | . | . | . | . | . | . | . |
| 21-225_197F4_LC | . | . | | . | | G | . | . | . | . | . | . | . | . | . | . | . | . | . | . | . | . | . | . | . | . |
| 21-225_198B1_LC | . | . | | . | | G | S | . | . | . | . | . | . | . | . | . | . | . | . | . | . | . | . | . | . | . |
| 21-225_198C2_LC | . | . | | . | | G | . | . | . | . | . | . | . | . | . | . | . | . | . | . | . | . | . | . | . | . |
| 21-225_198E1_LC | . | . | | . | . | G | . | . | . | . | . | . | . | . | . | . | . | . | . | . | . | . | . | . | . | . |
| 21-225_198G10_LC | . | . | | . | . | G | . | . | . | . | . | . | . | . | . | . | . | . | . | . | . | . | . | . | . | . |
| 21-225_199C2_LC | . | . | | . | . | G | . | . | . | . | . | . | . | . | . | . | . | . | . | . | . | . | . | . | . | . |
| 21-225_199C7_LC | . | . | | . | . | G | . | . | . | . | . | . | . | . | . | . | . | . | . | . | . | . | . | . | . | . |
| 21-225_199G11_LC | . | . | | . | . | G | . | . | . | . | . | . | . | . | . | . | . | . | . | . | . | . | . | . | . | . |
| 21-225_200A1_LC | G | . | | . | . | E | I | . | . | . | . | . | . | . | . | . | . | . | . | . | . | . | . | . | . | . |
| 21-225_200F10_LC | . | . | | . | . | G | . | . | . | . | . | . | . | . | . | . | . | . | . | . | . | . | . | . | . | . |
| 21-225_201B2_LC | F | . | | . | . | E | . | . | . | . | . | . | . | . | . | . | . | . | . | . | . | . | . | . | . | . |
| 21-225_201E1_LC | H | . | H | . | . | E | T | . | . | . | . | . | . | . | . | . | . | . | . | . | . | . | . | . | . | . |
| 21-225_202D11_LC | . | . | | . | . | E | . | . | . | . | . | . | . | . | . | . | . | . | . | . | . | . | . | . | . | . |
| 21-225_202D9_LC | . | . | | . | . | E | T | . | . | . | . | . | . | . | . | . | . | . | . | . | . | . | . | . | . | . |
| 21-225_205G7_LC | . | . | . | . | . | E | S | . | . | . | . | . | . | . | . | . | . | . | . | . | . | . | . | . | . | . |
| 21-225_208A9_LC | . | . | . | H | . | H | S | . | . | . | . | . | . | . | . | . | . | . | . | . | . | . | . | . | . | . |
| 21-225_208B5_LC | F | . | . | H | . | E | . | . | . | . | . | . | . | . | . | . | . | . | . | . | . | . | . | . | . | . |
| 21-225_220E1_LC | . | . | . | . | . | G | S | . | . | . | . | . | . | . | . | . | . | . | . | . | . | . | . | . | . | . |
| 21-225_222C7_LC | . | . | . | . | . | E | D | . | . | . | . | . | . | . | . | . | . | . | . | . | . | . | . | . | . | . |
| 21-225_225E7_LC | . | . | . | . | . | G | T | . | . | . | . | . | . | . | . | . | . | . | . | . | . | . | . | . | . | . |
| 21-225_23B9_LC | . | . | . | . | . | G | S | . | . | . | . | . | . | . | . | . | . | . | . | . | . | . | . | . | . | . |
| 21-225_44F6_LC | . | . | . | . | . | G | S | . | . | . | . | . | . | . | . | . | . | . | . | . | . | . | . | . | . | . |
| 21-225_59B2_LC | . | . | . | . | . | G | T | . | . | . | . | . | . | . | . | . | . | . | . | . | . | . | . | . | . | . |
| 21-225_74A3_LC | . | . | . | H | S | . | . | . | . | . | . | . | . | . | . | . | . | . | . | . | . | . | . | . | . | . |
| 21-225_74A4_LC | . | . | . | H | S | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | D | N | - | - | - | - | - | - | - | - | - | - | - | - | - | - | - | - | - | - | - |
| 21-225_74B12_LC | . | . | . | M | . | . | | | | | | | | | | | | | | | | | | | | |
| 21-225_74B2_LC | Q | . | . | . | . | E | S | | | | | | | | | | | | | | | | | | | |
| 21-225_74C5_LC | . | . | . | . | . | E | S | | | | | | | | | | | | | | | | | | | |
| 21-225_74C7_LC | . | . | . | . | . | E | R | | | | | | | | | | | | | | | | | | | |
| 21-225_74D11_LC | . | . | . | M | . | . | | | | | | | | | | | | | | | | | | | | |
| 21-225_74D2_LC | F | . | . | . | . | E | S | | | | | | | | | | | | | | | | | | | |
| 21-225_74D5_LC | . | . | . | . | . | E | S | | | | | | | | | | | | | | | | | | | |
| 21-225_74D6_LC | . | . | . | M | . | . | | | | | | | | | | | | | | | | | | | | |
| 21-225_74F4_LC | . | . | . | . | . | G | - | | | | | | | | | | | | | | | | | | | |
| 21-225_74F5_LC | . | . | . | M | S | . | | | | | | | | | | | | | | | | | | | | |
| 21-225_74G8_LC | . | . | . | . | . | G | - | | | | | | | | | | | | | | | | | | | |
| 21-225_74H5_LC | . | . | . | . | . | E | S | | | | | | | | | | | | | | | | | | | |
| 21-225_74H7_LC | Q | . | . | . | . | E | S | | | | | | | | | | | | | | | | | | | |
| 21-225_74H8_LC | . | . | . | . | . | G | - | | | | | | | | | | | | | | | | | | | |
| 21-225_75A12_LC | . | . | . | M | . | . | S | | | | | | | | | | | | | | | | | | | |
| 21-225_75A6_LC | . | . | . | M | . | . | | | | | | | | | | | | | | | | | | | | |
| 21-225_75A8_LC | . | . | . | M | S | . | | | | | | | | | | | | | | | | | | | | |
| 21-225_75B10_LC | F | . | . | . | . | E | Q | | | | | | | | | | | | | | | | | | | |
| 21-225_75C2_LC | . | . | . | M | . | . | | | | | | | | | | | | | | | | | | | | |
| 21-225_75C3_LC | . | . | . | M | . | . | S | | | | | | | | | | | | | | | | | | | |
| 21-225_75D3_LC | . | . | . | M | . | . | | | | | | | | | | | | | | | | | | | | |
| 21-225_75D9_LC | . | . | . | . | . | E | S | | | | | | | | | | | | | | | | | | | |
| 21-225_75F3_LC | . | . | . | M | . | . | | | | | | | | | | | | | | | | | | | | |
| 21-225_75G12_LC | . | . | . | M | . | . | | | | | | | | | | | | | | | | | | | | |
| 21-225_75G3_LC | . | . | . | . | . | E | R | | | | | | | | | | | | | | | | | | | |
| 21-225_75G7_LC | . | . | . | . | . | E | S | | | | | | | | | | | | | | | | | | | |
| 21-225_75G9_LC | . | . | . | . | . | E | S | | | | | | | | | | | | | | | | | | | |
| 21-225_75H8_LC | Q | . | . | . | . | E | S | | | | | | | | | | | | | | | | | | | |
| 21-225_76A4_LC | . | . | . | M | S | . | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | D | N | - | - | - | - | - | - | - | - | - | - | - | - | - | - | - | - | - | - | - |
| 21-225_76A7_LC | . | . | . | . | . | E | S | . | . | . | . | . | . | . | . | . | . | . | . | . | . | . | . | . | . | . |
| 21-225_76C9_LC | . | . | . | . | . | Q | - | . | . | . | . | . | . | . | . | . | . | . | . | . | . | . | . | . | . | . |
| 21-225_76D1_LC | . | . | . | . | . | E | S | . | . | . | . | . | . | . | . | . | . | . | . | . | . | . | . | . | . | . |
| 21-225_76E10_LC | . | . | . | . | . | E | S | . | . | . | . | . | . | . | . | . | . | . | . | . | . | . | . | . | . | . |
| 21-225_76G4_LC | . | . | . | X | S | . | . | . | . | . | . | . | . | . | . | . | . | . | . | . | . | . | . | . | . | . |
| 21-225_77A10_LC | . | . | . | X | . | . | S | . | . | . | . | . | . | . | . | . | . | . | . | . | . | . | . | . | . | . |
| 21-225_77C12_LC | . | . | . | . | . | E | S | . | . | . | . | . | . | . | . | . | . | . | . | . | . | . | . | . | . | . |
| 21-225_77C5_LC | . | . | . | X | . | . | . | . | . | . | . | . | . | . | . | . | . | . | . | . | . | . | . | . | . | . |
| 21-225_77F7_LC | . | . | . | X | . | . | . | . | . | . | . | . | . | . | . | . | . | . | . | . | . | . | . | . | . | . |
| 21-225_77G1_LC | . | . | . | X | . | . | . | . | . | . | . | . | . | . | . | . | . | . | . | . | . | . | . | . | . | . |
| 21-225_78E7_LC | . | . | . | . | . | E | R | . | . | . | . | . | . | . | . | . | . | . | . | . | . | . | . | . | . | . |
| 21-225_79F11_LC | . | . | . | X | S | . | . | . | . | . | . | . | . | . | . | . | . | . | . | . | . | . | . | . | . | . |
| 21-225_79F3_LC | . | . | . | X | . | . | . | . | . | . | . | . | . | . | . | . | . | . | . | . | . | . | . | . | . | . |
| 21-225_79G1_LC | . | . | . | X | . | . | . | . | . | . | . | . | . | . | . | . | . | . | . | . | . | . | . | . | . | . |
| 21-225_79G12_LC | . | . | . | X | S | . | . | . | . | . | . | . | . | . | . | . | . | . | . | . | . | . | . | . | . | . |
| 21-225_80A1_LC | . | . | . | X | S | . | . | . | . | . | . | . | . | . | . | . | . | . | . | . | . | . | . | . | . | . |
| 21-225_80A6_LC | . | . | . | . | . | E | S | . | . | . | . | . | . | . | . | . | . | . | . | . | . | . | . | . | . | . |
| 21-225_80B10_LC | . | . | . | . | . | E | S | . | . | . | . | . | . | . | . | . | . | . | . | . | . | . | . | . | . | . |
| 21-225_80C1_LC | . | . | . | X | S | . | . | . | . | . | . | . | . | . | . | . | . | . | . | . | . | . | . | . | . | . |
| 21-225_80D5_LC | Q | . | . | . | . | E | S | . | . | . | . | . | . | . | . | . | . | . | . | . | . | . | . | . | . | . |
| 21-225_80E12_LC | . | . | . | . | . | E | S | . | . | . | . | . | . | . | . | . | . | . | . | . | . | . | . | . | . | . |
| 21-225_80E3_LC | . | . | . | X | S | . | . | . | . | . | . | . | . | . | . | . | . | . | . | . | . | . | . | . | . | . |
| 21-225_80H12_LC | . | . | . | X | S | . | . | . | . | . | . | . | . | . | . | . | . | . | . | . | . | . | . | . | . | . |
| 21-225_80H7_LC | . | . | . | . | . | E | S | . | . | . | . | . | . | . | . | . | . | . | . | . | . | . | . | . | . | . |
| 21-225_81C11_LC | . | . | . | X | S | . | . | . | . | . | . | . | . | . | . | . | . | . | . | . | . | . | . | . | . | . |
| 21-225_81C5_LC | . | . | . | X | S | . | . | . | . | . | . | . | . | . | . | . | . | . | . | . | . | . | . | . | . | . |
| 21-225_83B6_LC | . | . | . | . | . | E | S | . | . | . | . | . | . | . | . | . | . | . | . | . | . | . | . | . | . | . |
| 21-225_83B7_LC | . | . | . | X | S | . | . | . | . | . | . | . | . | . | . | . | . | . | . | . | . | . | . | . | . | . |
| 21-225_83C10_LC | . | . | . | . | . | E | S | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | D | N | - | - | - | - | - | - | - | - | - | - | - | - | - | - | - | - | - | - | - |
| 21-225_83G1_LC | . | . | . | X | S | . | | | | | | | | | | | | | | | | | | | | |
| 21-225_84E12_LC | . | . | . | . | . | N | S | | | | | | | | | | | | | | | | | | | |
| 21-225_85A3_LC | . | . | . | X | . | | | | | | | | | | | | | | | | | | | | | |
| 21-225_85B4_LC | . | . | . | X | . | | | | | | | | | | | | | | | | | | | | | |
| 21-225_85B9_LC | . | . | . | . | . | N | S | | | | | | | | | | | | | | | | | | | |
| 21-225_85D6_LC | . | . | . | X | . | . | S | | | | | | | | | | | | | | | | | | | |
| 21-225_85G10_LC | F | . | . | . | . | N | S | | | | | | | | | | | | | | | | | | | |
| 21-225_85G6_LC | . | . | . | . | . | N | S | | | | | | | | | | | | | | | | | | | |
| 21-225_86C1_LC | . | . | . | X | . | | | | | | | | | | | | | | | | | | | | | |
| 21-225_86C11_LC | . | . | . | X | S | | | | | | | | | | | | | | | | | | | | | |
| 21-225_86E4_LC | . | . | . | X | S | | | | | | | | | | | | | | | | | | | | | |
| 21-225_87A12_LC | . | . | . | X | S | | | | | | | | | | | | | | | | | | | | | |
| 21-225_87C9_LC | . | . | . | X | S | | | | | | | | | | | | | | | | | | | | | |
| 21-225_87E5_LC | . | . | . | X | S | . | | | | | | | | | | | | | | | | | | | | |
| 21-225_87H1_LC | S | . | . | . | . | N | S | | | | | | | | | | | | | | | | | | | |
| 21-225_88E7_LC | . | . | . | X | S | . | | | | | | | | | | | | | | | | | | | | |
| 21-225_88F7_LC | . | . | . | X | S | . | | | | | | | | | | | | | | | | | | | | |
| 21-225_88G9_LC | . | . | . | X | S | . | | | | | | | | | | | | | | | | | | | | |
| 21-225_88H1_LC | . | . | . | X | S | . | | | | | | | | | | | | | | | | | | | | |
| 21-225_89A11_LC | S | . | . | . | . | N | I | | | | | | | | | | | | | | | | | | | |
| 21-225_89D5_LC | . | . | . | X | . | | | | | | | | | | | | | | | | | | | | | |
| 21-225_89E10_LC | . | . | . | X | S | . | | | | | | | | | | | | | | | | | | | | |
| 21-225_89H5_LC | . | . | . | . | . | N | S | | | | | | | | | | | | | | | | | | | |
| 21-225_90A5_LC | . | . | . | X | . | | | | | | | | | | | | | | | | | | | | | |
| 21-225_90F10_LC | . | . | . | X | . | | | | | | | | | | | | | | | | | | | | | |
| 21-225_90G4_LC | . | . | . | X | S | . | | | | | | | | | | | | | | | | | | | | |
| 21-225_90G5_LC | . | . | . | X | . | | | | | | | | | | | | | | | | | | | | | |
| 21-225_91B2_LC | . | . | . | X | . | | | | | | | | | | | | | | | | | | | | | |
| 21-225_91B3_LC | . | . | . | X | . | | | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | D | N | - | - | - | - | - | - | - | - | - | - | - | - | - | - | - | - | - | - | - |
| 21-225_91B4_LC | . | . | . | ⋈ | . | . | | | . | . | . | . | . | . | . | . | . | . | . | . | . | . | . | . | . | . |
| 21-225_91E9_LC | . | . | . | ⋈ | S | . | | | . | . | . | . | . | . | . | . | . | . | . | . | . | . | . | . | . | . |
| 21-225_91F3_LC | . | . | . | ⋈ | S | . | . | | . | . | . | . | . | . | . | . | . | . | . | . | . | . | . | . | . | . |
| 21-225_92B1_LC | . | . | . | . | . | ⋈ | S | | . | . | . | . | . | . | . | . | . | . | . | . | . | . | . | . | . | . |
| 21-225_92D6_LC | . | . | . | ⋈ | . | . | | | . | . | . | . | . | . | . | . | . | . | . | . | . | . | . | . | . | . |
| 21-225_93C2_LC | . | . | . | ⋈ | . | . | | | . | . | . | . | . | . | . | . | . | . | . | . | . | . | . | . | . | . |
| 21-225_93E4_LC | . | . | . | ⋈ | S | . | | | . | . | . | . | . | . | . | . | . | . | . | . | . | . | . | . | . | . |
| 21-225_93E9_LC | . | . | . | . | . | ⋈ | S | | . | . | . | . | . | . | . | . | . | . | . | . | . | . | . | . | . | . |
| 21-225_94D3_LC | . | . | . | ⋈ | . | . | S | | . | . | . | . | . | . | . | . | . | . | . | . | . | . | . | . | . | . |
| 21-225_94F12_LC | . | . | . | ⋈ | . | . | | | . | . | . | . | . | . | . | . | . | . | . | . | . | . | . | . | . | . |
| 21-225_94G10_LC | . | . | . | . | . | ⋈ | S | | . | . | . | . | . | . | . | . | . | . | . | . | . | . | . | . | . | . |
| 21-225_95D2_LC | . | . | . | ⋈ | . | . | | | . | . | . | . | . | . | . | . | . | . | . | . | . | . | . | . | . | . |
| 21-225_95F9_LC | . | . | . | ⋈ | . | . | | | . | . | . | . | . | . | . | . | . | . | . | . | . | . | . | . | . | . |
| 21-225_95G9_LC | . | . | . | ⋈ | . | . | | | . | . | . | . | . | . | . | . | . | . | . | . | . | . | . | . | . | . |
| 21-225_95H10_LC | . | . | . | ⋈ | . | . | | | . | . | . | . | . | . | . | . | . | . | . | . | . | . | . | . | . | . |
| 21-225_97A2_LC | . | . | . | ⋈ | S | . | | | . | . | . | . | . | . | . | . | . | . | . | . | . | . | . | . | . | . |
| 21-225_97E5_LC | . | . | . | ⋈ | S | . | | | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | . | - | - | S | P | W | T | F | G | Q | G | T | K | V | E | I | K | R |
| 21-225_147G8_LC | | | | | | | | | | | | | | | | | | N | |
| 21-225_190A3_LC | | | | | | | | | | | | | | | | | | | |
| 21-225_190A7_LC | | | | | | | | | | | | | | | | | | | |
| 21-225_190B4_LC | | | | | | | | A | | | | | | | | | | | |
| 21-225_190G10_LC | | | | S | P | | | | | | | | | | | | | | |
| 21-225_191A3_LC | | | | | | | | A | | | | | | | | | V | | |
| 21-225_191A5_LC | | | | S | P | | | | | V | | | | | | | | | |
| 21-225_191C3_LC | | | | | | | | A | | | | | | | | | | | |
| 21-225_191D3_LC | | | | | | | | | | | | | | | | | | | |
| 21-225_191F1_LC | | | | | | | | | | | | | | | | | V | | |
| 21-225_191H7_LC | | | | | | | | | | | | | | | | | | N | |
| 21-225_192B3_LC | | | | | | | | A | | | | | | | | | | | |
| 21-225_192F8_LC | | | | | | | | | | | | | | | | | | | |
| 21-225_192G12_LC | | | | | | | | | | | | | | | | | | | |
| 21-225_192H3_LC | | | | | | | | | | | | | | | | | | | |
| 21-225_193A1_LC | | | | | | | | | | | | | | | | | | | |
| 21-225_193B1_LC | | | | | | | | | | | | | | | | | | | |
| 21-225_193B10_LC | | | | S | P | | | | | | | | | | | | | | |
| 21-225_193B12_LC | | | | | | | | A | | | | | | | | | | | |
| 21-225_193C8_LC | | | | | | | | A | | | | | i | | | | V | | |
| 21-225_193D3_LC | | | | | | | | A | | | | | | | | | | | |
| 21-225_193D8_LC | | | | | | | | | | | | | | | | | | | |
| 21-225_193E6_LC | | | | | | | | | | | | | | | | | | | |
| 21-225_193F3_LC | | | | | | | | | | | | | | | | | | N | |
| 21-225_193G8_LC | | | | | | | | | | | | | | | | | | | |
| 21-225_193H6_LC | | | | | | | | | | | | | | | | | | N | |
| 21-225_194C10_LC | | | | | | | | | | | | | | | | | | | |
| 21-225_194E5_LC | | | | | | | | | | | | | | | | | | | |
| 21-225_194E6_LC | | | | | | | | | | | | | | | | | | | |

4422

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
|  |  |  |  |  |  |  |  |  |  |  |  |  | K_FR4 | | | | | | |
| CONSENSUS | - | - | - | - | S | P | W | T | F | G | Q | G | T | K | V | E | I | K | R |
| 21-225_195B1_LC | . |  | . |  |  | . | . |  | . | . | . | . | . |  | . | . | . | . | . |
| 21-225_195C8_LC |  | . | . | . | . | . | . | A | . | . | . | . | . |  |  |  | . | . |  |
| 21-225_196G10_LC |  | . | . | S | P |  | . | . | . | . | . | . | . |  |  | . | L | . | . |
| 21-225_196H12_LC | . | . |  | . | . | . | . | . | . | . | . | . | . | . | . | . | . | . | . |
| 21-225_197F4_LC | . | . | . | . | . | . | . | A | . | . | . | . | . |  | . | . | . | . | . |
| 21-225_198B1_LC |  | . | . | . | . | . | . | . | . | . | . | . | . | . | . | . | . | . | . |
| 21-225_198C2_LC |  |  | . | . | . | . | . | . | L | . | M | A | . |  | . | . | . | . | . |
| 21-225_198E1_LC |  | . | . | . | . | . | . | . | . | . | . | . | . | . | . | . | . | . | . |
| 21-225_198G10_LC | . |  | . | . | . | . | . | A | . | . | . | . | . | . | . | . | . | . | . |
| 21-225_199C2_LC | . |  | . | . | . | . | . | A | . | . | . | . | . | . | . | . | . | . | . |
| 21-225_199C7_LC | . | . | . | . | . | . | . | . | . | . | . | . | . |  | . | . | . | . | . |
| 21-225_199G11_LC | . | . | . | . | . | . | . | . | . | . | M | . | . | . | . | . | . | . | . |
| 21-225_200A1_LC | . | . | . | . | . | . | . | . | . | . | . | . | . | . | . | . | S | . | . |
| 21-225_200F10_LC | . | . | . | . | . | . | . | A | . | . | . | . | . | . | . | . | . | . | . |
| 21-225_201B2_LC | . | . | . | . | . | . | . | . | . | . | . | . | . | . | . | . | . | . | . |
| 21-225_201E1_LC | . | . | . | . | . | . | . | . | . | . | . | . | . | . | . | . | . | . | . |
| 21-225_202D11_LC | . | . | . | . | . | . | . | . | . | . | . | . | . | . | . | . | . | . | . |
| 21-225_202D9_LC | . | . | . | . | . | . | . | . | . | . | . | . | . | . | . | . | . | . | . |
| 21-225_205G7_LC | . | . | . | . | . | . | . | . | . | . | . | . | . | . | . | . | . | . | . |
| 21-225_208A9_LC | . | . | . | . | . | . | . | . | . | . | . | . | . | . | . | . | . | . | . |
| 21-225_208B5_LC | . | . | . | . | . | . | . | . | . | . | . | . | . | . | . | . | . | . | . |
| 21-225_220E1_LC | . | . | . | . | . | . | . | . | . | . | . | . | . | . | . | . | . | . | . |
| 21-225_222C7_LC | . | . | . | . | . | . | . | . | . | . | . | . | . | . | . | . | . | . | . |
| 21-225_225E7_LC | . | . | . | . | . | . | . | . | . | . | . | . | . | . | . | . | . | . | . |
| 21-225_23B9_LC | . | . | . | . | . | . | . | . | . | . | . | . | . | . | . | . | . | . | . |
| 21-225_44F6_LC | . | . | . | . | . | . | . | . | . | . | . | . | . | . | . | . | . | . | . |
| 21-225_59B2_LC | . | . | . | . | . | . | . | . | . | . | . | . | . | . | . | . | . | . | . |
| 21-225_74A3_LC | . | . | . | . | . | . | . | . | . | . | . | . | . | . | . | . | . | . | . |
| 21-225_74A4_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | S | P | W | T | F | G | Q | G | T | K | V | E | I | K | R |
| 21-225_74B12_LC | . | . | . | . | . | . | . | . | . | . | . | . | . | . | . | . | . | . | . |
| 21-225_74B2_LC | . | . | . | . | . | . | . | . | . | . | . | . | . | . | . | . | . | . | . |
| 21-225_74C5_LC | . | . | . | . | . | . | . | . | . | . | . | . | . | . | . | . | . | . | . |
| 21-225_74C7_LC | . | . | . | . | . | . | . | . | . | . | . | . | . | . | . | . | . | . | . |
| 21-225_74D11_LC | . | . | . | . | . | . | . | . | . | . | . | . | . | . | . | . | . | . | . |
| 21-225_74D2_LC | . | . | . | . | . | . | . | . | . | . | . | . | . | . | . | . | . | . | . |
| 21-225_74D5_LC | . | . | . | . | . | . | . | . | . | . | . | . | . | . | . | . | . | . | . |
| 21-225_74D6_LC | . | . | . | . | . | . | . | . | . | . | . | . | . | . | . | . | . | . | . |
| 21-225_74F4_LC | . | . | . | . | . | S | . | . | . | . | . | . | . | . | . | . | . | . | . |
| 21-225_74F5_LC | . | . | . | . | . | . | . | . | . | . | . | . | . | . | . | . | . | . | . |
| 21-225_74G8_LC | . | . | . | . | N | S | . | . | . | . | . | . | . | . | . | . | . | . | . |
| 21-225_74H5_LC | . | . | . | . | . | . | . | . | . | . | . | . | . | . | . | . | . | . | . |
| 21-225_74H7_LC | . | . | . | . | . | . | . | . | . | . | . | . | . | . | . | . | . | . | . |
| 21-225_74H8_LC | . | . | . | . | . | S | . | . | . | . | . | . | . | . | . | . | . | . | . |
| 21-225_75A12_LC | . | . | . | . | . | . | . | . | . | . | . | . | . | . | . | . | . | . | . |
| 21-225_75A5_LC | . | . | . | . | . | . | . | . | . | . | . | . | . | . | . | . | . | . | . |
| 21-225_75A8_LC | . | . | . | . | . | . | . | . | . | . | . | . | . | . | . | . | . | . | . |
| 21-225_75B10_LC | . | . | . | . | . | . | . | . | . | . | . | . | . | . | . | . | . | . | . |
| 21-225_75C2_LC | . | . | . | . | . | . | . | . | . | . | . | . | . | . | . | . | . | . | T |
| 21-225_75C3_LC | . | . | . | . | . | . | . | . | . | . | . | . | . | . | . | . | . | . | . |
| 21-225_75D3_LC | . | . | . | . | . | . | . | . | . | . | . | . | . | . | . | . | . | . | . |
| 21-225_75D9_LC | . | . | . | . | . | . | . | . | . | . | . | . | . | . | . | . | . | . | . |
| 21-225_75F3_LC | . | . | . | . | . | . | . | . | . | . | . | . | . | . | . | . | . | . | . |
| 21-225_75G12_LC | . | . | . | . | . | . | . | . | . | . | . | . | . | . | . | . | . | . | . |
| 21-225_75G3_LC | . | . | . | . | . | . | . | . | . | . | . | . | . | . | . | . | . | . | . |
| 21-225_75G7_LC | . | . | . | . | . | . | . | . | . | . | . | . | . | . | . | . | . | . | . |
| 21-225_75G9_LC | . | . | . | . | . | . | . | . | . | . | . | . | . | . | . | . | . | . | . |
| 21-225_75H8_LC | . | . | . | . | . | . | . | . | . | . | . | . | . | . | . | . | . | . | . |
| 21-225_76A4_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | S | P | W | T | F | G | Q | G | T | K | V | E | I | K | R |
| 21-225_76A7_LC | . | | - | - | . | . | | . | | . | . | . | | . | | | . | . | . |
| 21-225_76C9_LC | . | . | . | . | . | S | . | . | . | . | . | . | . | . | . | . | . | . | . |
| 21-225_76D1_LC | . | . | . | . | . | . | . | . | . | . | . | . | . | . | . | . | . | . | . |
| 21-225_76E10_LC | . | . | . | . | . | . | . | . | . | . | . | . | . | . | . | . | . | . | . |
| 21-225_76G4_LC | . | . | . | . | . | . | . | . | . | . | . | . | . | . | . | . | . | . | . |
| 21-225_77A10_LC | . | . | . | . | . | . | . | . | . | . | . | . | . | . | . | . | . | . | . |
| 21-225_77C12_LC | . | . | . | . | . | . | . | . | . | . | . | . | . | . | . | . | . | . | . |
| 21-225_77C5_LC | . | . | . | . | . | . | . | . | . | . | . | . | . | . | . | . | . | . | . |
| 21-225_77F7_LC | . | . | . | . | . | . | . | . | . | . | . | . | . | . | . | . | . | . | . |
| 21-225_77G1_LC | . | . | . | . | . | . | . | . | . | . | . | . | . | . | . | . | . | . | . |
| 21-225_78E7_LC | . | . | . | . | . | . | . | . | . | . | . | . | . | . | . | . | . | . | . |
| 21-225_79F11_LC | . | . | . | . | . | . | . | . | . | . | . | . | . | . | . | . | . | . | . |
| 21-225_79F3_LC | . | . | . | . | . | . | . | . | . | . | . | . | . | . | . | . | . | . | . |
| 21-225_79G1_LC | . | . | . | . | . | . | . | . | . | . | . | . | . | . | . | . | . | . | . |
| 21-225_79G12_LC | . | . | . | . | . | . | . | . | . | . | . | . | . | . | . | . | . | . | . |
| 21-225_80A1_LC | . | . | . | . | . | . | . | . | . | . | . | . | . | . | . | . | . | . | . |
| 21-225_80A6_LC | . | . | . | . | . | . | . | . | . | . | . | . | . | . | . | . | . | . | . |
| 21-225_80B10_LC | . | . | . | . | . | . | . | . | . | . | . | . | . | . | . | . | . | . | . |
| 21-225_80C1_LC | . | . | . | . | . | . | . | . | . | . | . | . | . | . | . | . | . | . | . |
| 21-225_80D5_LC | . | . | . | . | . | . | . | . | . | . | . | . | . | . | . | . | . | . | . |
| 21-225_80E12_LC | . | . | . | . | . | . | . | . | . | . | . | . | . | . | . | . | . | . | . |
| 21-225_80E3_LC | . | . | . | . | . | . | . | . | . | . | . | . | . | . | . | . | . | . | . |
| 21-225_80H12_LC | . | . | . | . | . | . | . | . | . | . | . | . | . | . | . | . | . | . | . |
| 21-225_80H7_LC | . | . | . | . | . | . | . | . | . | . | . | . | . | . | . | . | . | . | . |
| 21-225_81C11_LC | . | . | . | . | . | . | . | . | . | . | . | . | . | . | . | . | . | . | . |
| 21-225_81C5_LC | . | . | . | . | . | . | . | . | . | . | . | . | . | . | . | . | . | . | . |
| 21-225_83B6_LC | . | . | . | . | . | . | . | . | . | . | . | . | . | . | . | . | . | . | . |
| 21-225_83B7_LC | . | . | . | . | . | . | . | . | . | . | . | . | . | . | . | . | . | . | . |
| 21-225_83C10_LC | . | . | . | . | . | . | . | . | . | . | . | . | . | . | . | . | . | . | . |

4425

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | S | P | W | T | F | G | Q | G | T | K | V | E | I | K | R |
| 21-225_83G1_LC | . | . | . | . | . | . | . | . | . | . | . | . | . | . | . | . | . | . | . |
| 21-225_84E12_LC | . | . | . | . | . | . | . | . | . | . | . | . | . | . | . | . | . | . | . |
| 21-225_85A3_LC | . | . | . | . | . | . | . | . | . | . | . | . | . | . | . | . | . | . | . |
| 21-225_85B4_LC | . | . | . | . | . | . | . | . | . | . | . | . | . | . | . | . | . | . | . |
| 21-225_85B9_LC | . | . | . | . | . | . | . | . | . | . | . | . | . | . | . | . | . | . | . |
| 21-225_85D6_LC | . | . | . | . | . | . | . | . | . | . | . | . | . | . | . | . | . | . | . |
| 21-225_85G10_LC | . | . | . | . | . | . | . | . | . | . | . | . | . | . | . | . | . | . | . |
| 21-225_85G6_LC | . | . | . | . | . | . | . | . | . | . | . | . | . | . | . | . | . | . | . |
| 21-225_86C1_LC | . | . | . | . | . | . | . | . | . | . | . | . | . | . | . | . | . | . | . |
| 21-225_86C11_LC | . | . | . | . | . | . | . | . | . | . | . | . | . | . | . | . | . | . | . |
| 21-225_86E4_LC | . | . | . | . | . | . | . | . | . | . | . | . | . | . | . | . | . | . | . |
| 21-225_87A12_LC | . | . | . | . | . | . | . | . | . | . | . | . | . | . | . | . | . | . | . |
| 21-225_87C9_LC | . | . | . | . | . | . | . | . | . | . | . | . | . | . | . | . | . | . | . |
| 21-225_87E5_LC | . | . | . | . | . | . | . | . | . | . | . | . | . | . | . | . | . | . | . |
| 21-225_87H1_LC | . | . | . | . | . | . | . | . | . | . | . | . | . | . | . | . | . | . | . |
| 21-225_88E7_LC | . | . | . | . | . | . | . | . | . | . | . | . | . | . | . | . | . | . | . |
| 21-225_88F7_LC | . | . | . | . | . | . | . | . | . | . | . | . | . | . | . | . | . | . | . |
| 21-225_88G9_LC | . | . | . | . | . | . | . | . | . | . | . | . | . | . | . | . | . | . | . |
| 21-225_88H1_LC | . | . | . | . | . | . | . | . | . | . | . | . | . | . | . | . | . | . | . |
| 21-225_89A11_LC | . | . | . | . | . | . | . | . | . | . | . | . | . | . | . | . | . | . | . |
| 21-225_89D5_LC | . | . | . | . | . | . | . | . | . | . | . | . | . | . | . | . | . | . | . |
| 21-225_89E10_LC | . | . | . | . | . | . | . | . | . | . | . | . | . | . | . | . | . | . | . |
| 21-225_89H5_LC | . | . | . | . | V | . | . | . | . | . | . | . | . | . | . | . | . | . | . |
| 21-225_90A5_LC | . | . | . | . | . | . | . | . | . | . | . | . | . | . | . | . | . | . | . |
| 21-225_90F10_LC | . | . | . | . | . | . | . | . | . | . | . | . | . | . | . | . | . | . | . |
| 21-225_90G4_LC | . | . | . | . | . | . | . | . | . | . | . | . | . | . | . | . | . | . | . |
| 21-225_90G5_LC | . | . | . | . | . | . | . | . | . | . | . | . | . | . | . | . | . | . | . |
| 21-225_91B2_LC | . | . | . | . | . | . | . | . | . | . | . | . | . | . | . | . | . | . | . |
| 21-225_91B3_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | S | P | W | T | F | G | Q | G | T | K | V | E | I | K | R |
| 21-225_91B4_LC | . | . | . | . | . | . | . | . | . | . | . | . | . | . | . | . | . | . | . |
| 21-225_91E9_LC | . | . | . | . | . | . | . | . | . | . | . | . | . | . | . | . | . | . | . |
| 21-225_91F3_LC | . | . | . | . | . | . | . | . | . | . | . | . | . | . | . | . | . | . | . |
| 21-225_92B1_LC | . | . | . | . | . | . | . | . | . | . | . | . | . | . | . | . | . | . | . |
| 21-225_92D6_LC | . | . | . | . | . | . | . | . | . | . | . | . | . | . | . | . | . | . | . |
| 21-225_93C2_LC | . | . | . | . | . | . | . | . | . | . | . | . | . | . | . | . | . | . | . |
| 21-225_93E4_LC | . | . | . | . | . | . | . | . | . | . | . | . | . | . | . | . | . | . | . |
| 21-225_93E9_LC | . | . | . | . | . | . | . | . | . | . | . | . | . | . | . | . | . | . | . |
| 21-225_94D3_LC | . | . | . | . | . | . | . | . | . | . | . | . | . | . | . | . | N | Q | N |
| 21-225_94F12_LC | . | . | . | . | . | . | . | . | . | . | . | . | . | . | . | . | . | . | . |
| 21-225_94G10_LC | . | . | . | . | . | . | . | . | . | . | . | . | . | . | . | . | . | . | . |
| 21-225_95D2_LC | . | . | . | . | . | . | . | . | . | . | . | . | . | . | . | . | . | . | . |
| 21-225_95F9_LC | . | . | . | . | . | . | . | . | . | . | . | . | . | . | . | . | . | . | . |
| 21-225_95G9_LC | . | . | . | . | . | . | . | . | . | . | . | . | . | . | . | . | . | . | . |
| 21-225_95H10_LC | . | . | . | . | . | . | . | . | . | . | . | . | . | . | . | . | . | . | . |
| 21-225_97A2_LC | . | . | . | . | . | . | . | . | . | . | . | . | . | . | . | . | . | . | . |
| 21-225_97E5_LC | . | . | . | . | . | . | . | . | . | . | . | . | . | . | . | . | . | . | . |

**FIGURE 57**

Table 98.    Consensus 18 - VK1|A30/JK1 (SEQ ID NO: 50315):

DIQMTQSPSSLSASVGDRVTITCRAS--QGIR------NDLGWYQQKPGKAPKRLIYA--------ASSLQSGVPSRFSGSGSG--
TEFTLTISSLQPEDFATYYCLQHYS---------------------YPRTFGQGTKVEIKR wherein:

A at position 25 can be substituted with T

S at position 26 can be substituted with T

Q at position 29 can be substituted with R

G at position 30 can be substituted with D, A, N or V

R at position 32 can be substituted with G

N at position 39 can be substituted with K, D, H, S, G or T

D at position 40 can be substituted with I or Y

G at position 42 can be substituted with N

A at position 58 can be substituted with T, I, V, P, G, R or S

A at position 67 can be substituted with T or S

S at position 68 can be substituted with A, F, P or Y

S at position 69 can be substituted with N, R, G or T

L at position 70 can be substituted with C, F or S

Q at position 71 can be substituted with H or E

S at position 72 can be substituted with N, G or I

L at position 107 can be substituted with V, I or H

Q at position 108 can be substituted with M, H, L or V

FIGURE 57

H at position 109 can be substituted with Q, Y, L or S

Y at position 110 can be substituted with N, H, S or T

S at position 111 can be substituted with N, T, R, D, F or G

null (-) at position 134 can be substituted with Y or F

Y at position 135 can be substituted with F, P, C, N or T

P at position 136 can be substituted with L

R at position 137 can be substituted with W, F or L

Arg Xaa Xaa Xaa Xaa Ile Xaa Xaa Xaa Leu Xaa (SEQ ID NO: 50130)

Xaa Xaa Xaa Xaa Xaa Xaa Xaa (SEQ ID NO: 50131)

Xaa Xaa Xaa Xaa Xaa Xaa Xaa Xaa Xaa Thr (SEQ ID NO: 50132)

Arg Ala Ser Gln Gly Ile Arg Asn Asp Leu Gly (SEQ ID NO: 50615)

Ala Ala Ser Ser Leu Gln Ser (SEQ ID NO: 50616)

Leu Gln His Tyr Ser Tyr Pro Arg Thr (SEQ ID NO: 50617)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_10E9_LC | . | | | . | . | . | . | . | . | . | . | . | . | . | . | . | . | . | . | . | ※ | . | . | . | . | . |
| 21-225_12B12_LC | . | | | . | . | . | . | . | . | . | . | . | . | . | . | . | . | . | . | . | . | . | . | . | . | . |
| 21-225_12E6_LC | . | . | . | . | . | . | . | . | . | . | . | . | . | . | . | . | . | . | . | . | . | . | . | . | . | . |
| 21-225_12F2_LC | . | | . | . | . | . | . | . | . | . | . | . | . | . | . | . | . | . | . | . | . | . | . | . | . | . |
| 21-225_147H5_LC | . | | | . | . | . | . | . | . | . | . | . | . | . | . | . | . | . | . | . | . | . | . | . | . | . |
| 21-225_148C9_LC | . | | . | . | . | . | . | . | . | . | . | . | . | . | . | . | . | . | . | . | . | . | . | . | . | . |
| 21-225_149D10_LC | . | | . | . | . | . | . | . | . | . | . | . | . | . | . | . | . | . | . | . | . | . | . | . | . | . |
| 21-225_14E10_LC | . | . | . | . | . | . | . | . | . | . | . | . | . | . | . | . | . | . | . | . | . | . | . | . | T | . |
| 21-225_158D4_LC | . | . | . | . | . | . | . | . | . | . | . | . | . | . | . | . | . | . | . | . | . | . | . | . | . | . |
| 21-225_158G1_LC | . | . | . | . | . | . | . | . | . | . | . | . | . | . | . | . | . | . | . | . | . | . | . | . | . | . |
| 21-225_159A3_LC | . | . | . | . | . | . | . | . | . | . | . | . | . | . | . | . | . | . | . | . | . | . | . | . | . | . |
| 21-225_159C8_LC | . | . | . | . | . | . | . | . | . | . | . | . | . | . | . | . | . | . | . | . | . | . | . | . | . | . |
| 21-225_159D8_LC | . | . | . | . | . | . | . | . | . | . | . | . | . | . | . | . | . | . | . | . | . | . | . | . | . | . |
| 21-225_15C11_LC | . | . | . | . | . | . | . | . | . | . | . | . | . | . | . | . | . | . | . | . | . | . | . | . | . | . |
| 21-225_160C4_LC | . | . | . | . | . | . | . | . | . | . | . | . | . | . | . | . | . | . | . | . | . | . | . | . | . | . |
| 21-225_160F2_LC | . | . | . | . | . | . | . | . | . | . | . | . | . | . | . | . | . | . | . | . | . | . | . | . | . | . |
| 21-225_160H1_LC | . | . | . | . | . | . | . | . | . | . | . | . | . | . | . | . | . | . | . | . | . | . | . | . | . | . |
| 21-225_161G12_LC | . | . | . | . | . | . | . | . | . | ※ | . | . | . | . | . | . | . | . | . | . | . | . | . | . | . | . |
| 21-225_169D11_LC | . | . | . | . | . | . | . | . | . | . | . | . | . | . | . | . | . | . | . | . | . | . | . | . | . | . |
| 21-225_169D7_LC | . | . | . | . | . | . | . | . | . | . | . | . | . | . | . | . | . | . | . | . | . | . | . | . | . | . |
| 21-225_16A5_LC | . | . | . | . | . | . | . | . | . | . | . | . | . | . | . | . | . | . | . | . | . | . | . | . | . | . |
| 21-225_16G7_LC | . | . | . | . | . | . | . | . | . | . | . | . | . | . | . | . | . | . | . | . | . | . | . | . | . | . |
| 21-225_16H4_LC | . | . | . | . | . | . | . | . | . | . | . | . | . | . | . | . | . | . | . | . | . | . | . | . | . | . |
| 21-225_170E4_LC | . | . | . | . | . | . | . | . | . | . | . | . | . | . | . | . | . | . | . | . | . | . | . | . | . | . |
| 21-225_170F3_LC | . | . | . | . | . | . | . | . | . | . | . | . | . | . | . | Q | . | . | . | . | . | . | . | . | . | . |
| 21-225_170H1_LC | . | . | . | . | . | . | . | . | . | . | . | . | . | . | . | . | . | . | . | . | . | . | . | . | . | . |
| 21-225_174G10_LC | . | . | . | . | . | . | . | . | . | . | . | . | . | . | . | . | . | . | . | . | . | . | . | . | . | . |
| 21-225_17C7_LC | . | . | . | . | . | . | . | . | . | . | . | . | . | . | . | . | . | . | . | . | . | . | . | . | . | . |
| 21-225_17H3_LC | . | . | . | . | . | . | . | . | . | . | . | . | . | . | . | . | . | . | ※ | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_18A5_LC | . | | | | | | | | . | . | . | | . | . | | . | | . | . | | | | | | | |
| 21-225_18C2_LC | | | | | | | . | . | . | . | . | | . | | . | . | | . | | | . | | | | | |
| 21-225_18F2_LC | . | | | | | . | . | . | . | . | . | | . | | . | . | | . | . | . | | | . | | | |
| 21-225_18H12_LC | . | | | | | . | . | . | . | . | . | | . | | . | . | | . | . | . | | | | | | |
| 21-225_190C12_LC | . | | | | | . | . | . | . | . | . | | . | | . | . | | . | . | . | | | . | | | |
| 21-225_190G3_LC | . | | | | | . | . | . | . | . | . | | . | | . | . | ※ | . | . | . | | | | | | |
| 21-225_190H5_LC | . | | | | | . | . | . | . | . | . | | . | | . | . | | . | . | . | | | | | | |
| 21-225_191A4_LC | . | | | | | . | . | . | . | . | . | | . | | . | . | | . | . | . | | | | | | . |
| 21-225_192F7_LC | . | | | | . | . | . | . | . | . | . | | . | | . | . | | . | . | . | | | | | | . |
| 21-225_196C12_LC | . | | | | | L | . | . | . | . | . | ※ | . | ○ | . | . | | . | . | l | | | | | | |
| 21-225_19A10_LC | . | | | | | . | . | . | . | . | . | | . | | . | . | | . | . | . | | | | | | . |
| 21-225_200C9_LC | . | | | | | . | . | . | . | . | . | | . | | . | . | | . | . | . | | | | | | T |
| 21-225_201C5_LC | . | | | | | . | . | . | . | . | . | | . | | . | . | | . | . | . | | | | | | . |
| 21-225_203C10_LC | . | | | | | . | . | . | . | . | . | | . | | . | . | | . | . | . | | | | | | |
| 21-225_203F7_LC | . | | | | | . | . | . | . | . | ※ | ※ | . | | . | . | | . | . | . | | | | | | . |
| 21-225_204H3_LC | . | | | | | . | . | . | . | . | . | | . | | . | . | | . | . | . | | | | | | . |
| 21-225_208B2_LC | . | | | | | . | . | . | . | . | . | | . | | . | . | | . | . | . | | | | | | . |
| 21-225_208F1_LC | . | | | | | . | . | . | . | . | . | | . | | . | . | | . | . | . | | | | | | . |
| 21-225_20B11_LC | . | | | | | . | . | . | . | . | . | | . | | . | . | | . | . | . | | | | | | . |
| 21-225_20B2_LC | . | | | | | . | . | | . | . | . | | . | | . | . | | . | . | . | N | | | | | . |
| 21-225_20D10_LC | . | | | | | . | . | | . | . | . | ※ | . | | . | . | | . | . | . | | | | | | . |
| 21-225_210G5_LC | . | | | | | . | . | | . | . | ○ | ※ | . | ※ | . | . | | . | . | . | | | | | | . |
| 21-225_210G6_LC | . | | | | | . | . | | . | . | ○ | ※ | . | ※ | . | . | | . | . | . | | | | | | . |
| 21-225_212B11_LC | . | | | | | . | . | | . | . | . | ※ | . | | . | . | | . | . | . | | | | | | . |
| 21-225_214G10_LC | . | | | | | L | ○ | | . | . | ※ | ※ | . | | . | . | | . | . | . | | | | | | . |
| 21-225_214G12_LC | . | | | | | . | . | | . | . | ※ | ※ | . | | . | . | | . | . | . | | | | | | . |
| 21-225_214H9_LC | . | | | | | . | . | | . | . | . | . | . | | . | . | | . | . | . | | | | | | . |
| 21-225_215E11_LC | . | | | | | . | ○ | | . | . | ※ | ※ | . | | . | . | | . | . | . | | | | | | . |
| 21-225_215E3_LC | . | | | | | . | . | | . | . | ※ | ※ | . | | . | . | | . | . | . | | | | | | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_216E8_LC | . | | | | | . | . | . | . | . | . | P | . | . | . | . | . | . | . | . | . | . | . | . | . | . |
| 21-225_216H12_LC | . | | | | . | L | . | . | . | . | . | P | . | . | . | . | . | . | . | . | . | . | . | . | . | . |
| 21-225_217E5_LC | . | . | . | . | . | Q | . | . | . | . | P | P | . | P | . | . | . | . | . | . | . | . | . | . | . | . |
| 21-225_217G10_LC | . | . | . | . | . | . | . | . | . | . | P | P | . | . | . | . | . | . | . | . | . | . | . | . | . | . |
| 21-225_217H12_LC | . | . | . | . | . | . | . | . | . | . | P | P | . | . | . | . | . | . | . | . | . | . | . | . | . | . |
| 21-225_218C4_LC | . | . | . | . | . | Q | . | . | . | . | P | P | . | P | . | . | . | . | . | . | . | . | . | . | . | . |
| 21-225_21F7_LC | . | . | . | . | . | . | . | . | . | . | . | . | . | . | . | . | . | . | . | . | . | . | . | . | . | . |
| 21-225_21G11_LC | . | . | . | . | . | . | . | . | . | . | . | . | . | . | . | . | . | . | . | . | . | . | . | . | . | . |
| 21-225_21G2_LC | . | . | . | . | . | . | . | . | . | . | . | . | . | . | . | . | . | . | . | . | . | . | . | . | . | . |
| 21-225_21H8_LC | . | . | . | . | . | . | . | . | . | . | . | . | . | . | . | . | . | . | . | . | . | . | . | . | . | . |
| 21-225_220F8_LC | . | . | . | . | . | L | . | . | . | . | P | P | . | P | . | . | . | . | . | . | . | . | R | . | . | . |
| 21-225_224E7_LC | . | . | . | . | . | . | . | . | . | . | . | . | . | . | . | . | . | . | . | . | . | . | . | . | . | . |
| 21-225_224F1_LC | A | . | . | . | . | . | . | . | . | . | . | . | . | . | . | . | . | . | . | . | . | . | . | . | . | . |
| 21-225_224F3_LC | . | . | . | . | . | . | . | . | . | . | . | . | . | . | . | . | . | . | . | . | . | . | . | . | . | . |
| 21-225_225A1_LC | . | . | . | . | . | . | . | . | . | . | . | . | . | . | . | . | . | . | . | . | . | . | . | . | . | . |
| 21-225_226C6_LC | . | . | . | . | . | . | . | . | . | . | . | . | . | I | . | . | . | . | . | . | . | . | . | . | . | . |
| 21-225_226F7_LC | . | . | . | . | . | . | . | . | . | . | . | . | . | . | . | . | . | . | . | . | . | . | . | . | . | . |
| 21-225_226H11_LC | . | . | . | . | . | . | . | . | . | . | . | . | . | . | . | . | . | . | . | . | . | . | . | . | T | . |
| 21-225_22A4_LC | . | . | . | . | . | . | . | . | . | . | . | . | . | . | . | . | . | . | . | . | . | . | . | . | . | . |
| 21-225_22F4_LC | . | . | . | . | . | . | . | . | . | . | . | . | . | . | . | . | . | . | . | . | . | . | . | . | . | . |
| 21-225_23A10_LC | . | . | . | . | . | . | . | . | . | . | . | . | . | . | . | . | . | . | . | . | . | . | . | . | . | . |
| 21-225_23E11_LC | . | . | . | . | . | . | . | . | . | . | . | . | . | . | . | . | . | . | I | . | . | . | . | . | . | . |
| 21-225_23F12_LC | . | . | . | . | . | . | . | . | . | . | . | . | . | . | . | . | . | . | . | . | . | . | . | . | . | . |
| 21-225_24B6_LC | . | . | . | . | . | . | . | . | . | . | . | . | . | . | . | . | . | . | . | . | . | . | . | . | . | . |
| 21-225_25D12_LC | . | . | . | . | . | . | . | . | . | . | . | . | . | . | . | . | . | . | . | . | . | . | . | . | T | . |
| 21-225_26A2_LC | . | . | . | . | . | . | . | . | . | . | . | . | . | . | . | . | . | . | . | . | . | . | . | . | . | . |
| 21-225_28C7_LC | . | . | . | . | . | . | . | . | . | . | . | . | . | . | . | . | . | . | . | . | . | . | . | . | . | . |
| 21-225_28F5_LC | . | . | . | . | . | . | . | . | . | . | . | . | . | . | . | . | . | . | . | . | . | . | . | . | . | . |
| 21-225_2G4_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | K_FR1 | | | | | | | | | | | | | |
| CONSENSUS | D | I | Q | M | T | Q | S | P | S | S | L | S | A | S | V | G | D | R | V | T | I | T | C | R | A | S |
| 21-225_32B6_LC | . | . | . | . | . | . | . | . | . | . | . | . | . | . | . | . | . | . | . | . | . | . | . | . | . | . |
| 21-225_33C2_LC | . | . | . | . | . | . | . | . | . | . | . | . | . | . | . | . | . | . | . | . | . | . | . | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | . | . | . | . | . | . | . | . | . | . | . | . | . | . |
| 21-225_44D3_LC | . | . | . | . | . | . | . | . | . | . | . | . | . | . | . | . | . | . | . | . | . | . | . | . | . | . |
| 21-225_44D5_LC | . | . | . | . | . | . | . | . | . | . | . | . | . | . | . | . | . | . | . | . | . | . | . | . | . | . |
| 21-225_44F9_LC | . | V | . | . | . | . | . | . | . | . | . | . | . | . | . | . | . | . | . | . | . | . | . | . | . | . |
| 21-225_45B4_LC | . | . | . | . | . | . | . | . | . | . | . | . | . | . | . | . | . | . | . | . | . | . | . | . | . | . |
| 21-225_45B8_LC | . | . | . | . | . | . | . | . | . | . | . | . | . | . | . | . | . | . | . | . | . | . | . | . | . | . |
| 21-225_46C3_LC | . | . | . | . | . | . | . | . | . | . | . | . | . | . | . | . | . | . | . | . | . | . | . | . | . | . |
| 21-225_46D4_LC | . | . | . | . | . | . | . | . | . | . | . | . | . | . | . | . | . | . | . | . | . | . | . | . | T | . |
| 21-225_48A9_LC | . | . | . | . | . | . | . | . | . | . | . | . | . | . | . | . | . | . | . | . | . | . | . | . | . | . |
| 21-225_48C1_LC | . | . | . | . | . | . | . | . | . | . | . | . | . | . | . | . | . | . | . | . | . | . | . | . | T | . |
| 21-225_48F2_LC | . | . | . | . | . | . | . | . | . | . | . | . | . | . | . | . | . | . | . | . | . | . | . | . | . | . |
| 21-225_4C12_LC | . | . | . | . | . | . | . | . | . | . | . | . | . | . | . | . | . | . | . | . | . | . | . | . | . | . |
| 21-225_4C5_LC | . | . | . | . | . | . | . | . | . | . | . | . | . | . | . | . | . | . | . | . | . | . | . | . | . | . |
| 21-225_50A12_LC | . | . | . | . | . | . | . | . | . | . | . | . | . | . | . | . | . | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | . | . | . | . | . | . | . | . | . | . | . | . | . | . | . | . | . | . | . | . | . | . | . | . |
| 21-225_54H3_LC | . | . | . | . | . | . | . | . | . | . | . | . | . | . | . | . | . | . | . | . | . | . | . | . | . | . |
| 21-225_56C7_LC | . | . | . | . | . | . | . | . | . | . | . | . | . | . | . | . | . | . | . | . | . | . | . | . | . | . |
| 21-225_57B8_LC | . | . | . | . | . | . | . | . | . | . | . | . | . | . | . | . | . | . | . | . | . | . | . | . | . | . |
| 21-225_59B9_LC | . | . | . | . | . | . | . | . | . | . | . | . | . | . | . | . | . | . | . | . | . | . | . | . | . | . |
| 21-225_59G7_LC | . | . | . | . | . | . | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_5E5.011_LC | . | | | | | | | | | | | | | | | | | | | | | | | | | |
| 21-225_5E5.012_LC | . | . | . | . | . | . | . | . | | | | | | | | | | | | | | | | | | |
| 21-225_5E5.015_LC | . | | . | | | . | . | | | | | | | | | | | | | | | | | | | |
| 21-225_5E5.016_LC | . | | . | | | . | . | | | | | | | | | | | | | | | | | | | |
| 21-225_5E5.017_LC | . | | . | | | | . | | | | | | | | | | | | | | | | | | | |
| 21-225_5E5.018_LC | . | | . | | . | | | | | | | | | | | | | | | | | | | | | |
| 21-225_5E5.019_LC | . | | . | | | | | | | | | | | | | | | | | | | | | | | |
| 21-225_5E5.023_LC | . | | . | | | | | | | | | | | | | | | | | | | | | | | |
| 21-225_5E5.024_LC | . | | . | | | | | | | | | | | | | | | | | | | | | | | |
| 21-225_5E5.025_LC | . | . | | . | | | | | | | | | | | | | | | | | | | | | | |
| 21-225_60C3_LC | . | | | | | | | | | | | | | | | | | | | | | | | | | |
| 21-225_60E2_LC | . | | . | | | | | | | | | | | | | | | | | | | | | | | |
| 21-225_61E6_LC | . | | . | | | | | | | | | | | | | | | ! | | | | | | | | |
| 21-225_61H1_LC | . | | . | | | | | | | | | | | | | | | | | | | | | | | |
| 21-225_62F7_LC | . | | . | | | | | | | | | | | | | | | | | | | | | | | |
| 21-225_64A4_LC | . | | | | | . | | | | | | | | | | | | | | | | | | | | |
| 21-225_64C8_LC | . | | | | | | | | | | | | | A | | | | | | | | | | | | |
| 21-225_66C10_LC | . | | | | | | | | | | | | | | | | | | | | | | | | | |
| 21-225_66D11_LC | . | | . | | | | S | | | | Q | | | | | | | | | | | | | | | |
| 21-225_6A11_LC | . | | . | | | | | | | | | | | | | | | | | | | | | | | |
| 21-225_6G2_LC | . | | . | | | | | | | R | | | | S | | | | | | | | Y | | | | |
| 21-225_71A2_LC | . | | | | | | | | | R | | | | | | | | | | | | | | | | |
| 21-225_73C9_LC | . | | | | | | | | | | | | | | | | | | | | | | | | | |
| 21-225_7C9_LC | . | | . | | | | | | | | | | | | G | | | | | | | | | | | |
| 21-225_7F8_LC | . | | | | | | | | | | | | | | | | | | | | | | | | | |
| 21-225_7G2_LC | . | | . | | | | | | | | | | | | | | | | | | | | | | | |
| 21-225_7G4_LC | . | | . | | | | | | | | | | | | | | | | | | | | | | | |
| 21-225_94F3_LC | . | . | | . | | | | | | | | | | | | | | | | | | | | | | |
| 21-225_9A1_LC | . | | . | | | | | | | B | | | | S | | | | | | | | | | | | |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_9C11_LC | . | . | . | . | . | . | . | . | . | . | . | . | . | . | . | . | . | . | . | . | . | . | . | . | . | . |
| 21-225_9D12_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | K_CDR1 | | | | | | | | | | | | | | | | | | | | K_FR2 | |
| CONSENSUS | - | - | Q | G | I | R | - | - | - | - | - | - | N | D | L | G | W | Y | Q | Q | K | P | G | K | A | P |
| 21-225_10E9_LC | . | | D | | | | | . | | . | | . | S | . | | | | | | | | | | | | |
| 21-225_12B12_LC | . | | | | | | | . | | . | | | | . | | | | | | | | | | | | |
| 21-225_12E6_LC | . | | | | | | | . | | . | | . | S | . | | | | | | | | | | | | |
| 21-225_12F2_LC | . | | | | | | | | | | | | | . | | | | | | | | | | | | |
| 21-225_147H5_LC | . | R | | | | | | . | | . | | | D | . | | | | | | | | | | | | |
| 21-225_148C9_LC | . | R | | | | | | . | | . | | | D | . | | | | | | | | | | | | |
| 21-225_149D10_LC | . | R | | | | | | . | | . | | | | | | | | | | | | | | | | |
| 21-225_14E10_LC | . | . | | . | | | | | | | | . | | . | | | | | | R | | | | Q | | . |
| 21-225_158D4_LC | . | . | D | | | | | . | | | | . | R | . | | | | | | | | | | | | . |
| 21-225_158G1_LC | . | . | | | | | | . | | | | . | | . | | | | | | | | | | | | . |
| 21-225_159A3_LC | . | . | D | | | | | . | | | | | R | . | | | | | | | | | | | | . |
| 21-225_159C8_LC | . | . | D | | | | | . | | | | | R | . | | | | | | | | | | | | . |
| 21-225_159D8_LC | . | R | D | . | G | | | . | | | | . | | | | | | | | | | | | | | . |
| 21-225_15C11_LC | . | . | . | | | | | . | | . | | . | | . | | | | | | | | | | | V | . |
| 21-225_160C4_LC | . | . | D | | | | | . | | | | . | R | . | | | | | | | | | | | | . |
| 21-225_160F2_LC | . | . | . | | | | | . | | | | . | | . | | | | | | | | | | | | . |
| 21-225_160H1_LC | . | . | D | | | | | . | | | | . | | . | | | | | | | | | | | | . |
| 21-225_161G12_LC | . | . | D | | | | | . | | | | . | R | . | | | | | | | | | | | | . |
| 21-225_169D11_LC | . | . | . | | | | | . | | | | . | D | . | | | | | | | | | | | V | . |
| 21-225_169D7_LC | . | . | . | | | | | . | | | | | | . | | | | | | | | | | | | . |
| 21-225_16A5_LC | . | . | . | | | | | | | | | | | . | | | C | | | | | | | | | . |
| 21-225_16G7_LC | . | . | . | | | | | | | | | | | . | | | | | | | | | | | | . |
| 21-225_16H4_LC | . | . | . | | | | | | | | | | | | | | | | | | | | | | | . |
| 21-225_170E4_LC | . | . | . | | | | | | | | | | | | | . | Y | . | | | | | | | | . |
| 21-225_170F3_LC | . | R | . | | | | | | | | | | | | | | | | | | | | | | | . |
| 21-225_170H1_LC | . | . | . | | | | | | | | | | | | | | | | | | | | | . | | . |
| 21-225_174G10_LC | . | . | . | | | | | | | | | . | D | . | | | | | | | | | | | V | . |
| 21-225_17C7_LC | . | . | . | | | | | | | | . | | | . | | | | | | | | | | | | . |
| 21-225_17H3_LC | . | . | . | | | | | | | . | | . | S | . | | | | | | | | | | | | . |

4436

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | K_CDR1 | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_18A5_LC | . | . | . | . | . | . | . | . | . | . | . | . | . | . | . | . | . | . | . | . | . | . | . | . | . | . |
| 21-225_18C2_LC | . | . | . | . | . | . | . | . | . | . | . | . | . | . | . | . | . | . | . | . | . | . | . | . | . | . |
| 21-225_18F2_LC | . | . | . | . | . | . | . | . | . | . | . | . | . | . | . | . | . | . | . | . | . | . | . | . | . | . |
| 21-225_18H12_LC | . | . | . | . | . | . | . | . | . | . | . | . | . | . | . | . | . | . | . | . | . | . | . | . | . | . |
| 21-225_190C12_LC | . | . | . | . | . | . | . | . | . | . | . | . | X | . | . | . | . | . | . | . | . | . | . | . | . | . |
| 21-225_190G3_LC | . | . | . | . | . | . | . | . | . | . | . | . | X | . | . | . | . | . | . | . | . | . | . | . | . | . |
| 21-225_190H5_LC | . | . | . | . | . | . | . | . | . | . | . | . | X | . | . | . | . | . | . | . | . | . | . | . | . | . |
| 21-225_191A4_LC | . | . | . | . | . | . | . | . | . | . | . | . | X | . | . | . | . | . | . | . | . | . | . | . | . | . |
| 21-225_192F7_LC | . | . | . | . | . | . | . | . | . | . | . | . | X | . | . | . | . | . | . | . | . | . | . | . | . | . |
| 21-225_196C12_LC | . | . | . | . | . | . | . | . | . | . | . | . | . | . | . | . | . | S | . | . | N | . | . | . | . | . |
| 21-225_19A10_LC | . | . | . | . | . | . | . | . | . | . | . | . | T | . | . | . | . | . | . | . | X | . | . | . | . | . |
| 21-225_200C9_LC | . | . | . | . | . | . | . | . | . | . | . | . | X | . | . | . | . | . | . | . | . | . | . | . | . | . |
| 21-225_201C5_LC | . | . | . | . | . | . | . | . | . | . | . | . | X | . | . | . | . | . | . | . | . | . | . | . | . | . |
| 21-225_203C10_LC | . | . | D | . | . | . | . | . | . | . | . | . | . | . | . | . | . | . | . | . | . | . | A | . | . | . |
| 21-225_203F7_LC | . | . | . | . | . | . | . | . | . | . | . | . | X | . | . | . | . | . | . | . | . | . | . | . | . | L |
| 21-225_204H3_LC | . | . | . | . | . | . | . | . | . | . | . | . | . | . | . | . | . | . | . | . | . | . | . | . | . | . |
| 21-225_208B2_LC | . | . | . | . | . | . | . | . | . | . | . | . | X | . | . | . | . | . | . | . | . | . | . | . | . | . |
| 21-225_208F1_LC | . | . | . | . | . | . | . | . | . | . | . | . | X | . | . | . | . | . | . | . | . | . | . | . | . | . |
| 21-225_20B11_LC | . | . | . | . | . | G | . | . | . | . | . | . | . | . | . | . | . | . | . | . | . | . | E | . | . | . |
| 21-225_20B2_LC | . | . | D | . | . | . | . | . | . | . | . | . | . | . | . | . | . | . | . | . | . | . | . | . | . | . |
| 21-225_20D10_LC | . | . | . | . | . | . | . | . | . | . | . | . | . | . | . | . | . | . | . | . | . | . | . | . | . | R |
| 21-225_210G5_LC | . | . | . | . | . | . | . | . | . | . | . | . | X | . | . | . | . | . | . | . | . | . | . | . | . | . |
| 21-225_210G6_LC | . | . | . | . | . | . | . | . | . | . | . | . | X | . | . | . | . | . | . | . | . | . | . | . | . | L |
| 21-226_212B11_LC | . | . | . | . | . | . | . | . | . | . | . | . | . | . | . | . | . | . | . | . | . | . | . | . | . | . |
| 21-225_214G10_LC | . | . | . | . | . | . | . | . | . | . | . | . | . | . | . | . | . | . | . | . | . | . | . | . | . | . |
| 21-225_214G12_LC | . | . | . | . | . | . | . | . | . | . | . | . | . | . | . | . | . | . | . | . | . | . | . | . | . | . |
| 21-225_214H9_LC | . | . | . | . | . | . | . | . | . | . | . | . | . | . | . | . | . | . | . | . | . | . | . | . | . | . |
| 21-225_215E11_LC | . | . | . | . | . | . | . | . | . | . | . | . | . | . | . | . | . | . | . | . | . | . | . | . | . | . |
| 21-225_215E3_LC | . | . | Q | . | G | I | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_216E8_LC | . | | | | | | . | . | . | . | . | | | | | | | | | | | | | | | |
| 21-225_216H12_LC | | | | | | | . | . | . | . | . | | | | | | | | | | | | | | | |
| 21-225_217E5_LC | . | | | . | | | . | . | . | . | . | | | | | | | | | | . | . | | | | |
| 21-225_217G10_LC | . | | | | | | . | . | . | . | . | | | | | | | | | | . | | | | | |
| 21-225_217H12_LC | . | | | . | | . | . | . | . | . | . | | | | | | | | | | . | . | | | | |
| 21-225_218C4_LC | . | | . | | | | . | . | . | . | . | | | | | | | | | | | | | | | |
| 21-225_21F7_LC | . | | . | D | | | . | . | . | . | . | | | | | | | | | | | | | | | |
| 21-225_21G11_LC | . | . | . | . | . | | . | . | . | . | . | | | | . | . | | | . | . | . | . | . | . | . | . |
| 21-225_21G2_LC | . | . | . | . | . | | . | . | . | . | . | | | | . | . | | | . | . | . | . | . | . | . | . |
| 21-225_21H8_LC | . | . | . | . | . | | . | . | . | . | . | | | | . | . | | | . | . | . | . | . | . | . | . |
| 21-225_220F8_LC | . | . | . | . | . | | . | . | . | . | . | | | | . | . | | | . | . | . | . | . | . | . | . |
| 21-225_224E7_LC | . | . | . | . | . | | . | . | . | . | . | | | | . | . | | | . | . | . | . | . | . | . | . |
| 21-225_224F1_LC | . | . | . | . | . | | . | . | . | . | . | | D | | . | . | | | . | . | . | . | . | . | . | . |
| 21-225_224F3_LC | . | . | . | . | . | | . | . | . | . | . | | | | . | . | | | . | . | . | . | . | . | . | . |
| 21-225_225A1_LC | . | . | . | . | . | | . | . | . | . | . | | D | | . | . | | | . | . | ! | . | . | . | . | . |
| 21-225_226C6_LC | . | . | . | . | . | | . | . | . | . | . | | | | . | . | | | . | . | . | . | . | . | . | . |
| 21-225_226F7_LC | . | . | . | . | G | | . | . | . | . | . | | | | . | . | | | . | . | . | . | . | . | . | . |
| 21-225_226H11_LC | . | . | . | . | . | | . | . | . | . | . | | | | . | . | | | . | . | . | . | . | . | . | . |
| 21-225_22A4_LC | . | . | . | . | . | | . | . | . | . | . | | . | | . | . | | | . | . | . | . | . | . | . | . |
| 21-225_22F4_LC | . | . | . | V | . | | . | . | . | . | . | | D | | . | . | | | . | . | . | . | . | . | . | . |
| 21-225_23A10_LC | . | . | . | . | . | | . | . | . | . | . | | . | | . | . | | | . | . | . | . | . | . | . | . |
| 21-225_23E11_LC | . | . | . | . | . | | . | . | . | . | . | | . | | . | . | | | . | . | . | . | . | . | . | . |
| 21-225_23F12_LC | . | . | . | . | . | | . | . | . | . | . | | . | | . | . | | | . | . | . | . | . | . | . | . |
| 21-225_24B6_LC | . | . | D | . | . | | . | . | . | . | . | | . | | . | . | | | . | . | . | . | . | . | . | . |
| 21-225_25D12_LC | . | . | . | . | . | | . | . | . | . | . | | . | | . | . | | | . | . | . | . | . | . | . | . |
| 21-225_26A2_LC | . | . | . | . | . | | . | . | . | . | . | | S | | . | . | | | . | . | . | . | . | . | . | . |
| 21-225_28C7_LC | . | . | . | . | . | | . | . | . | . | . | | . | | . | . | | | . | . | . | . | . | . | . | . |
| 21-225_28F5_LC | . | . | . | . | . | | . | . | . | . | . | | . | | . | . | | | . | . | . | . | . | . | . | . |
| 21-225_2G4_LC | . | . | . | . | . | | . | . | . | . | . | | . | | . | . | | | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_32B6_LC | . | . | . | . | . | . | . | . | . | . | . | . | . | . | . | . | . | . | . | . | . | . | . | . | . | . |
| 21-225_33C2_LC | . | . | . | . | . | . | . | . | . | . | . | . | . | . | . | . | . | . | . | . | . | . | V | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | D | . | . | . | . | . | . | . | . | . | . | . | . | . |
| 21-225_44D3_LC | . | . | . | . | . | . | . | . | . | . | . | . | D | . | . | . | . | . | . | . | . | . | . | . | . | . |
| 21-225_44D5_LC | . | . | . | G | . | . | . | . | . | . | . | . | K | . | . | . | . | . | . | . | . | . | . | . | . | . |
| 21-225_44F9_LC | . | . | . | . | . | . | . | . | . | . | . | . | . | . | . | . | . | . | . | . | . | . | . | . | . | . |
| 21-225_45B4_LC | . | . | . | . | . | . | . | . | . | . | . | . | D | . | . | . | . | . | . | . | . | . | . | . | . | . |
| 21-225_45B8_LC | . | . | . | G | . | . | . | . | . | . | . | . | D | . | . | . | . | . | . | . | . | . | . | . | . | . |
| 21-225_46C3_LC | . | . | . | . | . | . | . | . | . | . | . | . | D | . | . | . | . | . | . | . | . | . | . | . | . | . |
| 21-225_46D4_LC | . | . | . | G | . | . | . | . | . | . | . | . | K | . | . | . | . | . | . | . | . | . | V | . | . | . |
| 21-225_48A9_LC | . | . | . | . | . | . | . | . | . | . | . | . | . | . | . | . | . | . | . | . | . | . | . | . | . | . |
| 21-225_48C1_LC | . | . | . | . | . | . | . | . | . | . | . | . | . | . | . | . | . | . | . | . | . | . | . | . | . | . |
| 21-225_48F2_LC | . | . | R | . | . | . | . | . | . | . | . | . | D | . | . | . | . | . | . | . | . | . | . | . | P | . |
| 21-225_4C12_LC | . | . | . | . | . | . | . | . | . | . | . | . | . | . | . | . | . | . | . | . | . | . | . | . | . | . |
| 21-225_4C5_LC | . | . | R | . | . | . | . | . | . | . | . | . | G | . | . | . | . | . | R | . | . | . | . | . | . | . |
| 21-225_50A12_LC | . | . | . | . | . | . | . | . | . | . | . | . | . | . | . | . | . | . | . | . | . | . | . | . | . | . |
| 21-225_50H8_LC | . | . | . | . | . | . | . | . | . | . | . | . | . | . | . | . | . | . | . | . | . | . | . | . | . | . |
| 21-225_51H8_LC | . | . | . | . | . | . | . | . | . | . | . | . | . | . | . | . | . | . | . | . | . | . | . | . | . | . |
| 21-225_54H3_LC | . | . | D | . | . | . | . | . | . | . | . | . | I | . | . | . | . | . | . | . | . | . | . | . | . | . |
| 21-225_56C7_LC | . | . | . | . | . | . | . | . | . | . | . | . | . | . | . | . | . | . | . | . | . | . | . | . | . | . |
| 21-225_57B8_LC | . | . | . | . | . | G | . | . | . | . | . | . | . | . | . | . | . | . | . | . | . | . | . | . | . | . |
| 21-225_59B9_LC | . | . | D | . | . | . | . | . | . | . | . | . | . | . | . | . | . | . | . | . | M | . | . | . | . | . |
| 21-225_59G7_LC | . | . | . | . | . | . | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | Q | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | K_CDR1 | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | . | . | . | . | . | . | . | . | . | . | . | . | . | . | . | . | . | . | . | . | . |
| 21-225_60E2_LC | . | . | . | . | . | . | . | . | . | . | . | . | X | . | . | . | . | . | . | . | . | . | . | . | . | . |
| 21-225_61E6_LC | . | . | . | . | . | G | . | . | . | . | . | . | . | . | . | . | . | . | . | . | . | . | . | . | . | . |
| 21-225_61H1_LC | . | . | . | . | . | . | . | . | . | . | . | . | X | . | . | . | . | . | . | . | . | . | . | . | . | . |
| 21-225_62F7_LC | . | . | A | . | . | . | . | . | . | . | . | . | . | . | . | . | . | . | . | . | . | . | . | . | . | . |
| 21-225_64A4_LC | . | . | . | . | . | . | . | . | . | . | . | . | . | . | . | . | . | . | . | . | . | . | . | . | . | . |
| 21-225_64C8_LC | . | . | . | . | . | . | . | . | . | . | . | . | . | . | . | . | . | . | . | . | . | . | . | . | . | . |
| 21-225_66C10_LC | . | . | A | . | . | . | . | . | . | . | . | . | . | . | . | . | . | . | . | . | . | . | . | . | . | . |
| 21-225_66D11_LC | . | . | . | . | . | . | . | . | . | . | . | . | . | . | . | . | . | . | . | . | . | . | . | . | . | X |
| 21-225_6A11_LC | . | . | . | . | . | . | . | . | . | . | . | . | . | . | . | . | . | . | . | . | . | . | . | . | . | . |
| 21-225_6G2_LC | . | . | . | . | . | . | . | . | . | . | . | . | . | . | . | . | . | . | . | . | . | . | . | . | . | . |
| 21-225_71A2_LC | . | . | . | . | . | . | . | . | . | . | . | . | . | . | . | . | . | . | . | . | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | . | . | . | . | . | . | . | . | . | . | . | . | . | . | . | . | . | . | . | . | . | . |
| 21-225_7F8_LC | . | . | . | . | . | G | . | . | . | . | . | . | . | . | . | . | . | . | . | . | . | . | . | . | . | . |
| 21-225_7G2_LC | . | . | . | . | . | . | . | . | . | . | . | . | . | . | . | . | . | . | . | . | . | . | . | . | . | . |
| 21-225_7G4_LC | . | . | . | . | . | . | . | . | . | . | . | . | . | . | . | . | . | . | . | X | . | . | . | . | . | . |
| 21-225_94F3_LC | . | . | A | . | . | . | . | . | . | . | . | . | O | . | . | . | . | . | . | . | . | . | . | . | . | . |
| 21-225_9A1_LC | . | . | . | . | . | . | . | . | . | . | . | . | . | . | . | . | . | . | . | . | . | . | . | . | . | X |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | K_CDR1 | | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_9C11_LC | . | . | . | N | . | . | | | | | . | . | . | Y | . | N | . | . | | | . | S | . | N | . | . |
| 21-225_9D12_LC | . | . | . | . | . | . | | | | | . | . | . | . | . | . | . | . | . | . | . | . | . | . | . | . |

4441

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_10E9_LC | T | . | . | . | F | . | . | . | . | . | . | . | . | . | . | . | . | . | . | . | . | . | . | . | . | . |
| 21-225_12B12_LC | . | . | . | . | . | T | . | . | . | . | . | . | . | . | . | . | . | . | . | . | . | . | . | . | . | . |
| 21-225_12E6_LC | . | . | . | . | . | . | . | . | . | . | . | . | . | . | . | . | . | . | . | . | . | . | . | . | . | . |
| 21-225_12F2_LC | . | . | . | . | . | . | . | . | . | . | . | . | . | . | . | . | . | . | . | . | . | . | . | . | . | . |
| 21-225_147H5_LC | . | . | . | . | . | . | . | . | . | . | . | . | . | . | . | . | . | . | . | . | . | . | . | . | . | . |
| 21-225_148C9_LC | . | . | . | . | . | . | . | . | . | . | . | . | . | . | . | . | . | . | . | . | . | . | . | . | . | . |
| 21-225_149D10_LC | . | . | . | . | . | . | . | . | . | . | . | . | . | . | . | . | . | . | . | . | . | . | . | . | . | . |
| 21-225_14E10_LC | . | . | . | . | . | . | . | . | . | . | . | . | . | . | . | . | . | . | . | . | . | . | . | . | . | . |
| 21-225_158D4_LC | . | . | . | . | . | . | . | . | . | . | . | . | . | . | . | . | . | . | . | . | . | . | . | . | . | . |
| 21-225_158G1_LC | . | . | . | . | . | T | . | . | . | . | . | . | . | . | . | . | N | . | . | . | . | . | . | . | . | . |
| 21-225_159A3_LC | N | . | . | . | . | . | . | . | . | . | . | . | . | . | . | . | . | . | . | . | . | . | . | . | . | . |
| 21-225_159C8_LC | N | . | . | . | . | . | . | . | . | . | . | . | . | . | . | . | . | . | . | . | . | . | . | . | . | . |
| 21-225_159D8_LC | . | . | . | . | S | . | . | . | . | . | . | . | . | . | . | . | . | . | . | . | . | . | . | . | . | . |
| 21-225_15C11_LC | . | . | . | . | . | . | . | . | . | . | . | . | . | . | . | . | . | . | . | . | . | . | . | . | . | . |
| 21-225_160C4_LC | N | . | . | . | . | . | . | . | . | . | . | . | . | . | . | . | . | . | . | . | . | . | . | . | . | . |
| 21-225_160F2_LC | . | . | . | . | . | T | . | . | . | . | . | . | . | . | . | . | N | . | . | . | . | . | . | . | . | . |
| 21-225_160H1_LC | . | . | . | . | . | . | . | . | . | . | . | . | . | . | . | . | . | . | . | N | . | . | . | . | . | . |
| 21-225_161G12_LC | N | . | . | . | . | . | . | . | . | . | . | . | . | . | . | . | . | . | . | . | . | . | . | . | . | . |
| 21-225_169D11_LC | . | . | . | . | . | . | . | . | . | . | . | . | . | . | . | . | . | . | . | . | . | . | . | . | . | . |
| 21-225_169D7_LC | . | . | . | . | . | . | . | . | . | . | . | . | . | . | . | . | . | . | . | . | . | . | . | . | . | . |
| 21-225_16A5_LC | . | . | . | . | . | . | . | . | . | . | . | . | . | . | . | . | . | . | . | . | . | . | . | . | . | . |
| 21-225_16G7_LC | . | . | . | . | . | . | . | . | . | . | . | . | . | . | . | . | . | . | . | . | . | . | . | . | . | . |
| 21-225_16H4_LC | . | . | . | . | . | T | . | . | . | . | . | . | . | . | . | . | . | . | . | . | . | . | . | . | . | . |
| 21-225_170E4_LC | . | . | . | . | . | V | . | . | . | . | . | . | . | . | . | . | G | . | . | . | . | . | . | . | . | . |
| 21-225_170F3_LC | . | . | . | . | . | . | . | . | . | . | . | . | . | . | . | . | . | . | . | . | . | . | . | . | . | . |
| 21-225_170H1_LC | . | . | . | . | . | . | . | . | . | . | . | . | . | . | T | . | . | . | . | . | . | . | . | . | . | . |
| 21-225_174G10_LC | . | . | . | . | . | . | . | . | . | . | . | . | . | . | . | . | . | . | . | . | . | . | . | . | . | . |
| 21-225_17C7_LC | . | . | . | . | . | . | . | . | . | . | . | . | . | . | . | . | . | . | . | . | . | . | . | . | . | . |
| 21-225_17H3_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_18A5_LC | . | | | | . | | . | | . | | . | | . | | . | | . | | . | | . | | . | | . | |
| 21-225_18C2_LC | . | | | | . | T | . | | . | | . | | . | | . | | . | | . | | . | | . | | . | |
| 21-225_18F2_LC | . | | | | . | | . | | . | | . | | . | | . | | . | | . | | . | | . | | . | |
| 21-225_18H12_LC | . | | | | . | T | . | | . | | . | | . | | . | | N | | . | | . | | . | | . | |
| 21-225_190C12_LC | . | | | | N | T | . | | . | | . | | . | | . | | . | | . | | . | | . | L | . | |
| 21-225_190G3_LC | . | | | | N | T | . | | . | | . | | . | | . | | . | | . | | . | | . | L | . | |
| 21-225_190H5_LC | . | | | | N | T | . | | . | | . | | . | | . | | . | | . | | . | | . | L | . | |
| 21-225_191A4_LC | . | | | | N | T | . | | . | | . | | . | | . | | . | | . | N | . | | . | L | . | |
| 21-225_192F7_LC | . | | | | N | T | . | | . | | . | | . | | . | | . | | . | | . | | . | L | . | |
| 21-225_196C12_LC | . | | | | . | | . | | . | | . | | . | | . | | . | | . | | . | | . | | . | |
| 21-225_19A10_LC | . | | | | . | T | . | | . | | . | | . | | . | | . | | . | | . | | . | | . | |
| 21-225_200C9_LC | . | | | | N | T | . | | . | | . | | . | | . | | . | | . | | . | | . | L | . | |
| 21-225_201C5_LC | . | | | | . | | . | | . | | . | | . | | . | | . | | . | | . | | . | | . | |
| 21-225_203C10_LC | . | | | | . | N | . | | . | | . | | . | | . | | . | | . | | . | | . | | . | |
| 21-225_203F7_LC | . | | | | . | | . | | . | | . | | . | | . | | . | | . | | . | | . | | . | |
| 21-225_204H3_LC | E | L | | | . | | . | | . | | . | | . | | A | | . | | . | G | . | | . | | . | |
| 21-225_208B2_LC | . | | | | . | | . | | . | | . | | . | | . | | . | | . | | . | | . | | G | . |
| 21-225_208F1_LC | . | | | | F | | . | | . | | . | | . | | . | | . | | . | | . | | . | | . | |
| 21-225_20B11_LC | . | | | | . | | . | | . | | . | | . | | . | | . | | . | N | . | | . | | . | |
| 21-225_20B2_LC | . | | | | . | | . | | . | | . | | . | | . | | . | | . | | . | | . | | . | |
| 21-225_20D10_LC | . | | | | V | | . | | . | | . | | . | | Y | | . | | . | | . | | . | | . | |
| 21-225_210G5_LC | . | | | | . | | . | | . | | . | | . | | . | | . | | . | | . | | . | | G | . |
| 21-225_210G6_LC | . | | | | . | | . | | . | | . | | . | | . | | . | | S | . | | . | | . | | . | |
| 21-226_212B11_LC | . | | | | . | | . | | . | | . | | . | | . | | . | | . | | . | | . | | . | |
| 21-225_214G10_LC | . | | | | . | | . | | . | | . | | . | | . | | . | | . | | . | | . | | . | |
| 21-225_214G12_LC | . | | | | . | | . | | . | | . | | . | | . | | . | | . | | . | | . | | . | |
| 21-225_214H9_LC | . | | | | . | | . | | . | | . | | . | | . | | . | | . | | . | | . | | . | |
| 21-225_215E11_LC | . | | | | . | | . | | . | | . | | . | | . | | . | | . | | . | | . | | . | |
| 21-225_215E3_LC | . | | | | . | | . | | . | | . | | . | | . | | . | | . | | . | | . | | . | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | K CDR2 | | | | | | | | | | | | | | | |
| CONSENSUS | K | R | L | I | Y | A | - | - | - | - | - | - | - | - | A | S | S | L | Q | S | G | V | P | S | R | F |
| 21-225_216E8_LC | . | | | | | . | . | . | . | . | . | | | . | . | . | . | . | . | . | . | . | . | . | . | . |
| 21-225_216H12_LC | . | | | | . | Q | . | . | . | . | . | | | . | . | . | . | . | . | . | . | . | . | . | . | . |
| 21-225_217E5_LC | . | | . | Q | . | . | . | . | . | . | . | | | . | . | . | . | . | . | . | . | . | . | . | . | . |
| 21-225_217G10_LC | . | | | | . | . | . | . | . | . | . | | | . | . | . | . | . | . | . | . | . | . | . | . | . |
| 21-225_217H12_LC | . | | | | . | . | . | . | . | . | . | | | . | . | . | . | . | . | . | . | . | . | . | . | . |
| 21-225_218C4_LC | . | | | | . | . | . | . | . | . | . | | | . | . | . | . | . | . | . | . | . | . | . | . | . |
| 21-225_21F7_LC | . | | . | . | . | . | . | . | . | . | . | | | . | . | N | S | . | . | . | . | . | . | . | . | . |
| 21-225_21G11_LC | . | . | . | . | . | T | . | . | . | . | . | | | . | . | . | . | . | . | . | . | . | . | . | . | . |
| 21-225_21G2_LC | . | . | . | . | . | T | . | . | . | . | . | | | . | S | . | . | . | . | . | . | . | . | . | . | . |
| 21-225_21H8_LC | . | . | . | . | . | . | . | . | . | . | . | | | . | . | . | . | . | . | . | . | . | . | . | . | . |
| 21-225_220F8_LC | . | . | . | . | C | Q | . | . | . | . | . | | | . | . | . | . | . | . | . | . | . | . | . | . | . |
| 21-225_224E7_LC | T | . | . | . | . | . | . | . | . | . | . | | | . | . | . | . | . | . | . | . | . | . | . | . | . |
| 21-225_224F1_LC | . | . | . | . | . | . | . | . | . | . | . | | | . | . | . | . | . | . | . | . | . | . | . | . | S |
| 21-225_224F3_LC | E | . | . | . | . | . | . | . | . | . | . | | | . | . | . | . | . | . | . | . | . | . | . | . | . |
| 21-225_225A1_LC | . | . | . | . | . | . | . | . | . | . | . | | | . | . | . | . | . | . | . | . | . | . | . | . | . |
| 21-225_226C6_LC | . | . | . | . | . | . | . | . | . | . | . | | | . | . | . | . | . | . | . | . | L | . | . | . | . |
| 21-225_226F7_LC | . | . | . | . | . | . | . | . | . | . | . | | | . | . | . | . | . | . | . | . | . | . | . | . | . |
| 21-225_226H11_LC | . | . | . | . | . | . | . | . | . | . | . | | | . | . | . | . | . | . | . | . | . | . | . | . | . |
| 21-225_22A4_LC | . | . | . | . | . | . | . | . | . | . | . | | | . | . | . | . | . | . | . | . | . | . | . | . | . |
| 21-225_22F4_LC | . | . | . | . | . | . | . | . | . | . | . | | | . | . | . | . | . | . | . | . | . | . | . | . | . |
| 21-225_23A10_LC | . | . | . | . | . | . | . | . | . | . | . | | | . | . | . | . | . | . | . | . | . | . | . | . | . |
| 21-225_23E11_LC | . | . | . | . | . | S | . | . | . | . | . | | | . | . | . | . | . | . | . | . | . | . | . | . | . |
| 21-225_23F12_LC | . | . | . | . | . | V | . | . | . | . | . | | | . | . | . | . | . | . | . | . | . | . | . | . | . |
| 21-225_24B6_LC | . | . | . | . | . | . | . | . | . | . | . | | | . | . | . | . | . | . | H | . | . | . | . | . | . |
| 21-225_25D12_LC | . | . | . | . | . | . | . | . | . | . | . | | | . | . | . | . | . | . | . | . | . | . | . | . | . |
| 21-225_26A2_LC | . | . | . | . | . | T | . | . | . | . | . | | | . | . | . | . | . | . | . | . | . | . | . | . | . |
| 21-225_28C7_LC | . | . | . | . | . | I | . | . | . | . | . | | | . | . | . | . | . | . | . | . | . | . | . | . | . |
| 21-225_28F5_LC | . | . | . | . | . | T | . | . | . | . | . | | | . | . | . | . | . | . | . | . | . | . | . | . | . |
| 21-225_2G4_LC | T | . | . | . | . | . | . | . | . | . | . | | | . | . | . | . | . | . | H | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_32B6_LC | . | . | . | . | . | } | . | . | . | . | . | . | . | . | . | . | . | . | . | . | . | . | . | . | . | . |
| 21-225_33C2_LC | . | . | . | . | . | . | . | . | . | . | . | . | . | . | P | . | . | . | . | . | . | . | . | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | . | . | T | . | . | . | . | . | . | . | . | . | . | . |
| 21-225_44D3_LC | . | . | . | . | . | . | . | . | . | . | . | . | . | . | . | . | . | . | . | . | . | . | . | . | . | . |
| 21-225_44D5_LC | . | . | . | . | . | . | . | . | . | . | . | . | . | . | . | . | T | . | E | . | . | . | . | . | . | . |
| 21-225_44F9_LC | . | . | . | . | . | . | . | . | . | . | . | . | . | . | . | . | . | . | . | . | . | . | . | . | . | . |
| 21-225_45B4_LC | . | . | . | . | . | . | . | . | . | . | . | . | . | . | . | . | . | . | . | . | . | . | . | . | . | . |
| 21-225_45B8_LC | . | . | . | . | . | . | . | . | . | . | . | . | . | . | . | . | . | . | . | . | . | . | . | . | . | . |
| 21-225_46C3_LC | . | . | . | . | . | . | . | . | . | . | . | . | . | . | . | . | . | . | . | . | . | . | . | . | . | . |
| 21-225_46D4_LC | . | . | . | . | . | . | . | . | . | . | . | . | . | . | . | . | . | . | E | . | . | . | . | . | . | . |
| 21-225_48A9_LC | . | . | . | . | . | } | . | . | . | . | . | . | . | . | . | . | . | . | . | . | . | . | . | . | . | . |
| 21-225_48C1_LC | . | . | . | . | . | E | . | . | . | . | . | . | . | . | . | . | . | . | . | . | . | . | . | A | . | . |
| 21-225_48F2_LC | . | . | . | . | . | . | . | . | . | . | . | . | . | . | . | . | . | . | . | . | . | . | . | . | . | . |
| 21-225_4C12_LC | T | . | . | . | . | . | . | . | . | . | . | . | . | . | . | . | . | . | E | . | . | . | . | . | . | . |
| 21-225_4C5_LC | . | . | . | . | . | . | . | . | . | . | . | . | . | . | . | . | N | . | . | . | . | . | . | . | . | . |
| 21-225_50A12_LC | . | . | . | . | . | T | . | . | . | . | . | . | . | . | . | . | N | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | . | . | . | . | . | . | . | . | . | . | . | . | . | . | . | . | . | . | . | . | . | . | . | . |
| 21-225_54H3_LC | . | . | . | . | . | . | . | . | . | . | . | . | . | . | . | . | N | . | . | . | . | . | . | . | . | . |
| 21-225_56C7_LC | . | . | . | . | . | T | . | . | . | . | . | . | . | . | . | . | N | . | . | . | . | . | . | . | . | . |
| 21-225_57B8_LC | E | . | . | . | . | . | . | . | . | . | . | . | . | . | . | . | . | . | . | . | . | . | . | . | . | . |
| 21-225_59B9_LC | . | . | . | . | . | . | . | . | . | . | . | . | . | . | . | . | . | . | . | . | . | . | . | . | . | . |
| 21-225_59G7_LC | . | . | . | . | . | S | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | S | . | . | . | . | . | . | . | . | . | . | . | . | . | . | . | . | . | . | . | . |
| 21-225_60E2_LC | . | . | . | . | . | . | . | . | . | . | . | . | . | . | . | . | . | . | . | . | . | . | . | . | . | . |
| 21-225_61E6_LC | N | . | . | . | . | . | . | . | . | . | . | . | . | . | . | . | . | . | . | . | . | . | . | . | . | . |
| 21-225_61H1_LC | . | . | . | . | F | . | . | . | . | . | . | . | . | . | . | . | . | . | . | . | . | . | . | . | . | . |
| 21-225_62F7_LC | . | . | . | . | . | P | . | . | . | . | . | . | . | . | . | . | N | . | . | . | . | . | . | . | . | . |
| 21-225_64A4_LC | . | . | . | . | . | . | . | . | . | . | . | . | . | . | . | . | . | . | . | . | . | . | . | . | . | . |
| 21-225_64C8_LC | . | C | . | . | . | . | . | . | . | . | . | . | . | . | . | . | . | . | . | . | . | . | . | . | . | . |
| 21-225_66C10_LC | . | . | . | . | . | P | . | . | . | . | . | . | . | . | . | . | . | . | . | . | . | . | . | . | . | . |
| 21-225_66D11_LC | . | . | . | . | . | . | . | . | . | . | . | . | . | . | . | . | . | G | . | . | . | . | . | . | . | . |
| 21-225_6A11_LC | . | . | . | . | . | . | . | . | . | . | . | . | . | . | . | . | . | . | . | . | . | . | . | . | . | . |
| 21-225_6G2_LC | Q | . | . | . | . | . | . | . | . | . | . | . | . | . | . | . | . | . | . | . | . | . | . | . | . | . |
| 21-225_71A2_LC | . | . | . | . | . | I | . | . | . | . | . | . | . | . | F | N | . | . | . | I | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | . | . | . | . | . | . | . | . | . | . | . | . | . | . | . | . | . | . | . | . | . | . |
| 21-225_7F8_LC | . | . | . | . | . | . | . | . | . | . | . | . | . | . | . | . | . | . | . | . | . | . | . | . | . | . |
| 21-225_7G2_LC | . | . | . | . | . | T | . | . | . | . | . | . | . | . | . | . | . | . | . | . | . | . | . | . | . | . |
| 21-225_7G4_LC | . | . | . | . | . | . | . | . | . | . | . | . | . | . | . | . | . | . | . | . | . | . | . | . | . | . |
| 21-225_94F3_LC | Q | . | . | . | F | . | . | . | . | . | . | . | . | . | . | . | . | . | . | . | . | . | . | . | . | . |
| 21-225_9A1_LC | . | . | . | . | . | T | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | K_CDR2 | | | | | | | | | | | | | | | | |
| CONSENSUS | K | R | L | I | Y | A | - | - | - | - | - | - | - | - | A | S | S | L | Q | S | G | V | P | S | R | F |
| 21-225_9C11_LC | . | L | . | . | . | V | | | . | . | . | . | . | . | . | . | . | . | | . | . | . | . | . | . | . |
| 21-225_9D12_LC | . | . | . | . | . | . | | . | . | . | . | . | . | . | . | . | . | . | | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_10E9_LC | . | | | | . | | | . | | . | | | | | | N | | | | | | . | | | . | |
| 21-225_12B12_LC | | | | . | . | | . | . | . | . | | | | . | | | | | | | | | | | | |
| 21-225_12E6_LC | . | | | . | . | | . | . | . | | | | | | | | | | | | | . | | . | . | |
| 21-225_12F2_LC | . | | | . | . | | . | . | . | | | | | | | | | | | | | . | | V | . | . |
| 21-225_147H5_LC | . | | | . | . | | . | . | . | | | | | | | | | | | | | | | . | . | |
| 21-225_148C9_LC | . | | | . | . | | . | . | . | | | | | | | | | | | | | | | . | . | |
| 21-225_149D10_LC | | | | . | . | | . | . | . | | | | | | | | | | | | | | | . | . | |
| 21-225_14E10_LC | . | | | . | . | | . | . | . | | | . | | . | | . | | | | | | | | T | A | . |
| 21-225_158D4_LC | . | | | . | . | | . | . | . | | | | | . | F | . | | | | | | | | . | . | |
| 21-225_158G1_LC | . | | | C | . | | . | . | . | | | | | . | V | . | | | | | | | | . | . | |
| 21-225_159A3_LC | . | | | . | . | | . | . | . | | | | | . | F | . | | | | | | | | . | . | |
| 21-225_159C8_LC | . | | | . | . | | . | . | . | | | | | . | F | . | | | | | | | | . | . | |
| 21-225_159D8_LC | . | | | . | . | | . | . | . | | | | | . | F | . | | | | | | | | . | . | |
| 21-225_15C11_LC | . | | | . | . | | . | . | . | | | | | . | . | . | | | | | | | | . | . | |
| 21-225_160C4_LC | . | | | . | . | | . | . | . | | | | | . | F | . | | | | | | | | . | . | |
| 21-225_160F2_LC | . | | | . | . | | . | . | . | | | | | . | V | . | | | | | | | | . | . | |
| 21-225_160H1_LC | . | | | . | . | | . | . | . | | | | | . | . | . | | | | | | | | T | . | . |
| 21-225_161G12_LC | . | | | . | . | | . | . | . | | | | | . | F | . | | | | | | | | . | . | |
| 21-225_169D11_LC | . | | | . | . | | . | . | . | | | | | . | . | . | | | | | | | | . | . | |
| 21-225_169D7_LC | . | | | . | . | | . | . | . | | | | | . | . | . | | | | | | | | . | . | . |
| 21-225_16A5_LC | . | N | . | Y | . | | . | . | | | | | | | . | . | | | | | | | | . | . | |
| 21-225_16G7_LC | . | | | . | . | | . | . | | | | | | | . | . | | | | | | | | . | . | . |
| 21-225_16H4_LC | . | | | . | . | | . | . | | | | | | | . | . | | | | | | | | . | . | . |
| 21-225_170E4_LC | . | | | . | . | | . | . | | | | | | | . | . | | | | | | | | . | . | . |
| 21-225_170F3_LC | . | | | . | . | | . | . | | | | | | | . | N | . | | | | | | | . | . | . |
| 21-225_170H1_LC | . | | | . | . | | . | . | | | | | | | . | . | | | | | | | | . | . | . |
| 21-225_174G10_LC | . | | | . | . | | . | . | | | | | | | . | . | | | | | | | | . | . | . |
| 21-225_17C7_LC | . | | | . | . | | . | . | | | | | | | . | . | | | | | | | | . | . | . |
| 21-225_17H3_LC | . | | | . | . | | . | . | | | | | | | . | . | | | | | | | | . | . | . |

4448

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_18A5_LC | . | . | . | . | . | . | . | . | . | . | . | . | . | . | . | . | . | . | . | . | . | . | . | . | . | . |
| 21-225_18C2_LC | . | . | . | . | P | . | . | . | . | . | . | . | . | . | . | . | . | . | . | . | . | . | . | . | . | . |
| 21-225_18F2_LC | . | . | . | . | . | . | . | . | . | . | . | . | . | . | . | . | . | . | . | . | . | . | . | . | . | . |
| 21-225_18H12_LC | . | . | . | . | . | . | . | . | . | . | . | . | . | . | . | . | . | . | . | . | . | . | . | . | . | . |
| 21-225_190C12_LC | . | . | . | . | . | . | . | . | . | . | . | . | . | . | . | . | . | . | . | . | . | . | . | . | . | . |
| 21-225_190G3_LC | . | . | . | . | . | . | . | . | . | . | . | . | . | . | . | . | . | . | . | . | . | . | . | . | . | . |
| 21-225_190H5_LC | . | . | . | . | . | . | . | . | . | . | . | . | . | . | . | . | . | . | . | . | . | . | . | . | . | . |
| 21-225_191A4_LC | . | . | . | . | . | . | . | . | . | . | . | . | . | . | . | . | . | . | . | . | . | . | . | . | . | . |
| 21-225_192F7_LC | . | . | . | . | . | . | . | . | . | . | . | . | . | . | . | . | . | . | . | . | . | . | . | . | . | . |
| 21-225_196C12_LC | . | . | . | . | . | . | . | . | . | . | . | . | I | . | . | . | . | . | . | . | . | . | . | E | . | . |
| 21-225_19A10_LC | . | . | . | . | . | . | . | . | . | . | . | . | . | . | . | . | R | . | . | . | . | . | . | . | . | . |
| 21-225_200C9_LC | . | . | . | . | . | . | . | . | . | . | . | . | . | . | . | . | . | . | . | . | . | . | . | . | . | . |
| 21-225_201C5_LC | . | . | . | . | . | . | . | . | . | . | . | . | . | . | . | . | . | . | . | . | . | . | . | . | . | . |
| 21-225_203C10_LC | . | . | . | . | . | . | . | . | . | . | . | . | . | . | . | . | . | . | . | . | . | . | . | . | A | . |
| 21-225_203F7_LC | . | . | . | . | C | . | . | . | . | . | . | . | . | . | . | . | . | V | . | . | . | . | . | . | N | . |
| 21-225_204H3_LC | . | . | . | . | . | . | . | . | . | . | . | . | . | . | . | . | . | . | . | . | . | . | . | . | . | . |
| 21-225_208B2_LC | . | . | . | . | . | . | . | . | . | . | . | . | . | . | . | . | . | . | . | . | . | . | . | . | . | . |
| 21-225_208F1_LC | . | . | . | . | . | . | . | . | . | . | . | . | . | . | . | . | . | . | . | . | . | . | . | . | . | . |
| 21-225_20B11_LC | . | . | . | . | . | . | . | . | . | . | . | . | . | . | . | . | . | . | . | . | . | . | . | . | . | . |
| 21-225_20B2_LC | . | . | . | . | . | . | . | . | . | . | . | . | . | . | . | . | . | . | . | . | . | . | . | . | . | . |
| 21-225_20D10_LC | . | . | . | Y | . | . | . | . | . | . | . | . | . | . | . | . | . | . | . | . | . | . | . | . | . | . |
| 21-225_210G5_LC | . | . | . | . | . | . | . | . | . | . | . | . | . | . | . | . | . | . | . | . | . | . | . | . | . | . |
| 21-225_210G6_LC | . | . | . | . | C | . | . | . | . | . | . | . | . | . | . | . | . | V | . | R | . | . | . | . | N | . |
| 21-225_212B11_LC | . | . | . | . | . | . | . | . | . | . | I | . | . | . | . | . | . | . | . | . | . | . | . | . | . | . |
| 21-225_214G10_LC | . | . | . | . | . | . | . | . | . | . | I | . | . | . | . | . | . | . | . | . | . | . | . | . | . | . |
| 21-225_214G12_LC | . | . | . | . | . | . | . | . | . | . | I | . | . | . | . | . | . | . | . | . | . | . | . | . | . | . |
| 21-225_214H9_LC | . | . | . | . | . | . | . | . | . | . | . | . | . | . | . | . | . | . | . | . | . | . | . | . | . | . |
| 21-225_215E11_LC | . | . | . | . | . | . | . | . | . | . | I | . | . | . | . | . | . | V | . | R | . | . | . | . | . | . |
| 21-225_215E3_LC | . | . | . | . | . | . | . | . | . | . | I | . | . | . | . | . | . | . | . | . | . | . | . | . | . | . |

## FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_216E8_LC | . | . | . | . | . | . | . | . | . | . | ; | . | . | . | . | . | . | . | . | . | . | . | . | . | . | . |
| 21-225_216H12_LC | . | . | . | . | . | . | . | . | . | . | ; | . | . | . | . | . | . | . | . | . | . | . | . | . | . | . |
| 21-225_217E5_LC | . | . | . | . | . | . | . | . | . | . | ; | . | . | . | . | . | . | . | . | . | . | . | . | . | . | . |
| 21-225_217G10_LC | . | . | . | . | . | . | . | . | . | . | ; | . | . | . | . | . | . | . | . | . | . | . | . | . | . | . |
| 21-225_217H12_LC | . | . | . | . | . | . | . | . | . | . | ; | . | . | . | . | . | . | . | . | . | . | . | . | . | . | . |
| 21-225_218C4_LC | . | . | . | . | . | . | . | . | . | . | ; | . | . | . | . | . | . | V | . | N | . | . | . | . | . | . |
| 21-225_21F7_LC | . | . | . | . | . | . | . | . | . | . | . | . | . | . | . | . | . | . | . | . | . | . | . | . | . | . |
| 21-225_21G11_LC | . | . | . | . | . | . | . | . | . | . | . | . | . | . | . | . | . | . | . | . | . | . | . | . | . | . |
| 21-225_21G2_LC | . | . | . | . | . | . | . | . | . | . | . | . | . | . | . | . | . | . | . | . | . | . | . | . | . | . |
| 21-225_21H8_LC | ? | . | . | . | . | . | . | . | . | . | . | . | . | . | . | . | . | . | . | . | . | . | . | . | . | . |
| 21-225_220F8_LC | . | . | . | . | . | . | . | . | . | . | ; | . | . | . | . | . | . | . | . | N | . | . | . | . | . | . |
| 21-225_224E7_LC | . | . | . | . | . | . | . | . | . | . | . | . | . | . | . | . | . | . | . | . | . | . | . | . | . | . |
| 21-225_224F1_LC | . | . | . | . | . | . | . | . | . | . | . | . | . | . | . | . | . | . | . | . | . | . | . | . | ; | . |
| 21-225_224F3_LC | . | . | . | . | . | . | . | . | . | . | . | . | . | . | . | . | . | . | . | . | . | . | . | . | . | . |
| 21-225_225A1_LC | . | . | . | . | . | . | . | . | . | . | . | . | . | . | . | . | . | . | . | . | . | . | . | . | . | . |
| 21-225_226C6_LC | . | . | . | . | . | . | . | . | . | . | . | . | . | . | . | . | N | . | . | . | . | . | . | . | . | . |
| 21-225_226F7_LC | . | . | V | . | . | . | . | . | . | . | ; | . | . | . | . | . | . | . | . | . | . | . | . | . | . | . |
| 21-225_226H11_LC | . | . | . | . | . | . | . | . | O | . | . | . | . | . | . | . | . | . | . | . | . | . | . | . | . | . |
| 21-225_22A4_LC | . | . | . | . | . | . | . | . | . | . | . | . | . | . | . | . | . | . | . | . | . | . | . | . | . | . |
| 21-225_22F4_LC | . | . | N | . | . | . | . | . | O | . | . | . | . | . | . | . | . | . | . | . | . | . | . | . | . | . |
| 21-225_23A10_LC | . | . | . | . | . | . | . | . | . | . | . | . | . | . | . | G | . | . | . | . | . | . | . | . | . | . |
| 21-225_23E11_LC | . | . | . | . | . | . | . | . | . | . | . | . | . | . | . | . | . | . | . | . | . | . | . | . | . | . |
| 21-225_23F12_LC | . | . | . | . | . | . | . | . | . | . | . | . | . | . | . | . | . | . | . | . | . | . | . | S | . | . |
| 21-225_24B6_LC | . | . | . | . | . | . | . | . | . | . | . | . | . | . | . | . | . | . | . | T | . | . | . | . | . | . |
| 21-225_25D12_LC | . | . | . | . | . | . | . | . | . | . | . | . | . | . | . | . | . | . | . | . | . | . | . | . | . | . |
| 21-225_26A2_LC | . | . | . | . | . | . | . | . | . | . | . | . | . | . | F | . | . | . | . | . | . | . | . | . | . | . |
| 21-225_28C7_LC | . | . | . | . | . | . | . | . | . | . | . | . | . | . | . | . | . | . | . | . | . | . | . | . | . | . |
| 21-225_28F5_LC | . | . | . | . | . | . | . | . | . | . | . | . | . | . | . | . | . | . | . | . | . | . | . | . | . | . |
| 21-225_2G4_LC | . | . | . | . | . | . | . | . | . | . | . | . | . | . | . | . | . | . | . | . | . | . | L | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_32B6_LC | . | . | . | . | . | . | . | . | . | . | . | . | . | . | . | . | . | . | . | . | . | . | . | . | . | . |
| 21-225_33C2_LC | . | . | . | . | . | . | . | . | . | . | . | . | . | . | . | . | . | . | . | . | . | . | N | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | . | . | . | . | . | . | . | . | . | . | . | . | . | . |
| 21-225_44D3_LC | . | . | . | R | . | . | . | . | R | . | . | . | . | . | . | . | . | . | . | . | . | . | . | . | . | . |
| 21-225_44D5_LC | . | . | . | . | . | . | . | . | . | . | . | . | . | . | . | . | . | . | . | . | . | . | . | . | . | . |
| 21-225_44F9_LC | . | . | . | . | . | . | . | . | . | . | . | . | . | . | . | . | . | . | . | . | . | . | . | . | . | . |
| 21-225_45B4_LC | . | . | . | . | . | . | . | . | . | . | . | . | . | . | . | . | . | . | . | . | . | . | . | . | . | . |
| 21-225_45B8_LC | . | . | . | . | . | . | . | . | . | . | . | . | . | . | . | . | . | . | . | . | . | . | . | . | . | . |
| 21-225_46C3_LC | . | . | . | R | . | . | . | . | . | . | . | . | . | . | . | . | . | . | . | . | . | . | . | . | . | . |
| 21-225_46D4_LC | . | . | . | . | . | . | . | . | . | . | . | . | . | . | . | . | . | . | . | . | . | . | . | . | . | . |
| 21-225_48A9_LC | . | . | . | . | . | . | . | . | . | . | . | . | . | . | . | . | G | . | . | . | . | . | . | . | i | . |
| 21-225_48C1_LC | . | . | . | . | . | . | . | . | . | . | . | . | . | . | . | . | . | . | . | . | . | . | . | . | i | . |
| 21-225_48F2_LC | N | . | . | . | . | . | . | . | . | . | . | . | . | . | . | . | . | . | . | S | . | . | L | . | . | . |
| 21-225_4C12_LC | . | . | . | . | . | . | . | . | . | . | . | . | . | . | . | . | . | . | . | . | . | . | L | . | . | . |
| 21-225_4C5_LC | . | . | . | . | Y | . | . | . | . | . | . | . | . | . | . | . | G | . | . | . | . | . | . | . | . | . |
| 21-225_50A12_LC | . | . | . | . | . | . | . | . | . | . | . | . | . | . | . | . | . | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | G | . | . | . | . | . | . | R | . | . | . | . | . | . | . | . | . | . | . | . | . | . | . | . |
| 21-225_54H3_LC | . | . | . | . | . | . | . | . | . | . | . | . | . | . | . | . | . | . | . | . | . | . | . | . | . | . |
| 21-225_56C7_LC | . | . | . | . | . | . | . | . | . | . | . | . | . | . | . | . | . | . | . | . | . | . | . | . | . | . |
| 21-225_57B8_LC | . | . | . | . | . | . | . | . | . | . | . | . | . | . | . | . | . | . | . | . | . | . | . | . | . | . |
| 21-225_59B9_LC | . | . | Y | R | . | . | . | . | . | . | . | . | . | . | . | . | . | . | . | . | . | . | . | . | . | . |
| 21-225_59G7_LC | . | . | . | . | . | . | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | . | . | . | . | . | . | . | . | . | . | . | . | . | . | . | . | . | . | . | . | . |
| 21-225_60E2_LC | . | . | . | . | . | . | . | . | . | . | . | . | . | . | . | . | . | . | . | . | . | . | . | . | . | . |
| 21-225_61E6_LC | . | . | . | . | . | . | . | . | . | . | . | . | . | . | . | . | . | . | . | . | . | . | . | . | . | . |
| 21-225_61H1_LC | . | . | . | . | . | . | . | . | . | . | . | . | . | . | . | . | . | . | . | . | . | . | . | . | . | . |
| 21-225_62F7_LC | . | . | . | . | . | . | . | . | . | . | . | . | . | . | . | . | . | . | . | . | . | . | . | . | . | . |
| 21-225_64A4_LC | . | . | . | . | . | . | . | . | . | . | . | . | . | . | . | . | . | . | . | . | . | . | Ł | . | . | . |
| 21-225_64C8_LC | . | . | . | . | . | . | . | . | . | . | . | . | . | . | . | . | . | . | . | . | . | . | . | . | . | . |
| 21-225_66C10_LC | . | . | . | . | . | . | . | . | . | . | . | . | . | . | . | . | . | . | . | . | . | . | . | . | . | . |
| 21-225_66D11_LC | . | . | . | C | . | . | . | . | . | . | . | . | Ï | S | . | . | . | M | . | Ö | . | . | . | . | . | . |
| 21-225_6A11_LC | . | . | . | . | . | . | . | . | . | . | . | . | . | . | . | . | . | . | . | . | . | . | . | . | . | . |
| 21-225_6G2_LC | . | . | . | Y | . | . | . | . | . | . | N | Ï | . | . | . | . | . | . | . | . | . | . | . | Ï | . | . |
| 21-225_71A2_LC | . | . | . | . | . | . | . | . | . | . | . | . | . | . | . | . | . | . | . | . | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | . | . | . | . | . | . | . | . | . | . | . | . | . | . | . | . | . | . | . | . | . | . |
| 21-225_7F8_LC | . | . | V | . | . | . | . | . | . | . | . | . | . | P | . | . | . | . | . | . | . | . | . | . | . | . |
| 21-225_7G2_LC | . | . | . | . | . | . | . | . | . | . | . | . | . | . | . | . | . | . | . | . | . | . | . | . | . | . |
| 21-225_7G4_LC | . | . | . | . | . | . | . | . | . | . | . | . | . | . | . | . | . | . | . | . | . | . | S | . | . | . |
| 21-225_94F3_LC | . | . | . | . | . | . | . | . | . | . | . | . | . | . | . | . | . | . | . | . | . | . | . | . | . | . |
| 21-225_9A1_LC | . | . | . | Y | . | . | . | . | . | . | . | . | Ï | . | . | . | . | . | . | . | . | . | . | . | . | . |

4452

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | E | F | T | L | T | I | S | S | L | Q | P | E | D | F | A | T | Y |
| 21-225_9C11_LC | . | . | . | . | . | . | | | . | . | . | . | . | . | . | N | . | . | . | . | . | . | . | . | : | . |
| 21-225_9D12_LC | . | . | . | . | . | . | | | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | K_CDR3 | | | | | | | |
| CONSENSUS | Y | C | L | Q | H | Y | S | - | - | - | - | - | - | - | - | - | - | - | - | - | - | - | - | - | - | - |
| 21-225_10E9_LC | . | | | | | . | | | | | | | | | | | | | | | | | | | | |
| 21-225_12B12_LC | | | | | | N | | . | | | | | | | | | | | | | | | | | | |
| 21-225_12E6_LC | . | | . | | . | N | | . | | | . | | | | | | | | | | | | | | | |
| 21-225_12F2_LC | . | | | | . | N | | . | | | | | | | | | | | | | | | | | | |
| 21-225_147H5_LC | . | | | | | . | | . | | | | | | | | | | | | | | | | | | |
| 21-225_148C9_LC | . | | | | | | | . | | | | | | | | | | | | | | | | | | |
| 21-225_149D10_LC | . | | | | | | | . | | | | | | | | | | | | | | | | | | |
| 21-225_14E10_LC | . | | | . | | . | | . | | | | | | | . | | | | | | | | | | | |
| 21-225_158D4_LC | . | | | | | . | | . | | | | | | | | | | | | | | | | | | |
| 21-225_158G1_LC | . | | | . | | N | | . | | | | | | | | | | | | | | | | | | |
| 21-225_159A3_LC | . | | | | | H | | . | | | | | | | . | | | | | | | | | | | |
| 21-225_159C8_LC | . | | . | | | H | | . | | | | | | | . | | | | | | | | | | | |
| 21-225_159D8_LC | . | | . | | | . | | . | | | | | | | . | | | | | | | | | | | |
| 21-225_15C11_LC | . | | . | L | | . | | . | | | | | | | . | | | | | | | | | | | |
| 21-225_160C4_LC | . | | . | | | . | | . | | | | | | | . | | | | | | | | | | | |
| 21-225_160F2_LC | . | | . | | | N | | . | | | | | | | . | | | | | | | | | | | |
| 21-225_160H1_LC | . | | . | | | N | | . | | | | | | | . | | | | | | | | | | | |
| 21-225_161G12_LC | . | | . | | | H | | . | | | | | | | . | | | | | | | | | | | |
| 21-225_169D11_LC | . | | . | | | . | | . | | | | | | | . | | | | | | | | | | | |
| 21-225_169D7_LC | . | | . | | | H | | . | | | | | | | . | | | | | | | | | | | |
| 21-225_16A5_LC | . | | . | | | N | | . | | | | | | | . | | | | | | | | | | | |
| 21-225_16G7_LC | . | | . | | | N | | . | | | | | | | . | | | | | | | | | | | |
| 21-225_16H4_LC | . | | . | | | N | | . | | | | | | | . | | | | | | | | | | | |
| 21-225_170E4_LC | . | | . | | | N | | . | | | | | | | . | | | | | | | | | | | |
| 21-225_170F3_LC | . | | . | | | . | | . | | | | | | | . | | | | | | | | | | | |
| 21-225_170H1_LC | . | | . | | | S | | . | | | | | | | . | | | | | | | | | | | |
| 21-225_174G10_LC | . | | . | | | H | | . | | | | | | | . | | | | | | | | | | | |
| 21-225_17C7_LC | . | | . | | | N | | . | | | | | | | . | | | | | | | | | | | |
| 21-225_17H3_LC | . | | . | | Q | . | | . | | | | | | | . | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_18A5_LC | . | | | | . | N | . | . | . | . | . | . | . | . | . | . | . | . | . | . | . | . | . | . | . | . |
| 21-225_18C2_LC | . | | | | . | N | . | . | . | . | . | . | . | . | . | . | . | . | . | . | . | . | . | . | . | . |
| 21-225_18F2_LC | . | | | . | . | N | . | . | . | . | . | . | . | . | . | . | . | . | . | . | . | . | . | . | . | . |
| 21-225_18H12_LC | . | | | | . | N | N | . | . | . | . | . | . | . | . | . | . | . | . | . | . | . | . | . | . | . |
| 21-225_190C12_LC | . | | | | . | N | . | . | . | . | . | . | . | . | . | . | . | . | . | . | . | . | . | . | . | . |
| 21-225_190G3_LC | . | | | . | . | N | . | . | . | . | . | . | . | . | . | . | . | . | . | . | . | . | . | . | . | . |
| 21-225_190H5_LC | . | | | . | . | N | N | . | . | . | . | . | . | . | . | . | . | . | . | . | . | . | . | . | . | . |
| 21-225_191A4_LC | . | | . | . | . | N | . | . | . | . | . | . | . | . | . | . | . | . | . | . | . | . | . | . | . | . |
| 21-225_192F7_LC | . | | . | . | . | T | . | . | . | . | . | . | . | . | . | . | . | . | . | . | . | . | . | . | . | . |
| 21-225_196C12_LC | . | | . | . | . | N | G | . | . | . | . | . | . | . | . | . | . | . | . | . | . | . | . | . | . | . |
| 21-225_19A10_LC | . | | . | . | . | N | G | . | . | . | . | . | . | . | . | . | . | . | . | . | . | . | . | . | . | . |
| 21-225_200C9_LC | . | | . | . | . | N | . | . | . | . | . | . | . | . | . | . | . | . | . | . | . | . | . | . | . | . |
| 21-225_201C5_LC | . | | . | . | . | . | . | . | . | . | . | . | . | . | . | . | . | . | . | . | . | . | . | . | . | . |
| 21-225_203C10_LC | . | | . | . | . | N | . | . | . | . | . | . | . | . | . | . | . | . | . | . | . | . | . | . | . | . |
| 21-225_203F7_LC | . | | . | . | . | . | . | . | . | . | . | . | . | . | . | . | . | . | . | . | . | . | . | . | . | . |
| 21-225_204H3_LC | . | | . | . | . | . | . | . | . | . | . | . | . | . | . | . | . | . | . | . | . | . | . | . | . | . |
| 21-225_208B2_LC | . | | . | . | . | . | . | . | . | . | . | . | . | . | . | . | . | . | . | . | . | . | . | . | . | . |
| 21-225_208F1_LC | . | | . | . | . | . | . | . | . | . | . | . | . | . | . | . | . | . | . | . | . | . | . | . | . | . |
| 21-225_20B11_LC | . | | . | . | . | . | . | . | . | . | . | . | . | . | . | . | . | . | . | . | . | . | . | . | . | . |
| 21-225_20B2_LC | . | | . | . | . | . | N | . | . | . | . | . | . | . | . | . | . | . | . | . | . | . | . | . | . | . |
| 21-225_20D10_LC | . | . | I | . | Q | N | . | . | . | . | . | . | . | . | . | . | . | . | . | . | . | . | . | . | . | . |
| 21-225_210G5_LC | . | . | . | X | . | . | . | | | | . | . | . | . | . | . | . | . | . | . | . | . | . | . | . | . |
| 21-225_210G6_LC | . | . | V | . | . | . | . | | | | . | . | . | . | . | . | . | . | . | . | . | . | . | . | . | . |
| 21-226_212B11_LC | . | . | . | L | . | . | . | | | | . | . | . | . | . | . | . | . | . | . | . | . | . | . | . | . |
| 21-225_214G10_LC | . | . | . | L | . | N | . | . | . | . | . | . | . | . | . | . | . | . | . | . | . | . | . | . | . | . |
| 21-225_214G12_LC | . | . | V | M | . | . | . | . | . | . | . | . | . | . | . | . | . | . | . | . | . | . | . | . | . | . |
| 21-225_214H9_LC | . | . | . | . | . | . | . | . | . | . | . | . | . | . | . | . | . | . | . | . | . | . | . | . | . | . |
| 21-225_215E11_LC | . | . | V | M | . | . | . | . | . | . | . | . | . | . | . | . | . | . | . | . | . | . | . | . | . | . |
| 21-225_215E3_LC | . | . | V | M | . | N | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_216E8_LC | . | | . | M | . | . | . | . | . | . | . | . | . | . | . | . | . | . | . | . | . | . | . | . | . | . |
| 21-225_216H12_LC | . | | V | M | . | N | . | . | . | . | . | . | . | . | . | . | . | . | . | . | . | . | . | . | . | . |
| 21-225_217E5_LC | . | | V | M | . | N | . | . | . | . | . | . | . | . | . | . | . | . | . | . | . | . | . | . | . | . |
| 21-225_217G10_LC | . | | V | M | . | N | . | . | . | . | . | . | . | . | . | . | . | . | . | . | . | . | . | . | . | . |
| 21-225_217H12_LC | . | | | M | . | . | . | . | . | . | . | . | . | . | . | . | . | . | . | . | . | . | . | . | . | . |
| 21-225_218C4_LC | . | | V | M | . | . | . | . | . | . | . | . | . | . | . | . | . | . | . | . | . | . | . | . | . | . |
| 21-225_21F7_LC | . | | . | . | . | N | N | . | . | . | . | . | . | . | . | . | . | . | . | . | . | . | . | . | . | . |
| 21-225_21G11_LC | . | | . | . | . | N | N | . | . | . | . | . | . | . | . | . | . | . | . | . | . | . | . | . | . | . |
| 21-225_21G2_LC | . | | . | . | . | N | . | . | . | . | . | . | . | . | . | . | . | . | . | . | . | . | . | . | . | . |
| 21-225_21H8_LC | . | | . | . | . | N | . | . | . | . | . | . | . | . | . | . | . | . | . | . | . | . | . | . | . | . |
| 21-225_220F8_LC | . | | V | M | . | N | . | . | . | . | . | . | . | . | . | . | . | . | . | . | . | . | . | . | . | . |
| 21-225_224E7_LC | . | | . | . | . | . | . | . | . | . | . | . | . | . | . | . | . | . | . | . | . | . | . | . | . | . |
| 21-225_224F1_LC | . | | . | . | . | . | . | . | . | . | . | . | . | . | . | . | . | . | . | . | . | . | . | . | . | . |
| 21-225_224F3_LC | . | | . | . | . | . | N | . | . | . | . | . | . | . | . | . | . | . | . | . | . | . | . | . | . | . |
| 21-225_225A1_LC | . | | . | . | . | . | . | . | . | . | . | . | . | . | . | . | . | . | . | . | . | . | . | . | . | . |
| 21-225_226C6_LC | . | | . | . | . | . | N | . | . | . | . | . | . | . | . | . | . | . | . | . | . | . | . | . | . | . |
| 21-225_226F7_LC | . | | M | . | . | . | . | . | . | . | . | . | . | . | . | . | . | . | . | . | . | . | . | . | . | . |
| 21-225_226H11_LC | . | | . | . | . | . | . | . | . | . | . | . | . | . | . | . | . | . | . | . | . | . | . | . | . | . |
| 21-225_22A4_LC | . | | . | . | . | N | . | . | . | . | . | . | . | . | . | . | . | . | . | . | . | . | . | . | . | . |
| 21-225_22F4_LC | . | | M | . | . | . | . | . | . | . | . | . | . | . | . | . | . | . | . | . | . | . | . | . | . | . |
| 21-225_23A10_LC | . | | . | . | . | . | . | . | . | . | . | . | . | . | . | . | . | . | . | . | . | . | . | . | . | . |
| 21-225_23E11_LC | . | | . | . | . | N | . | . | . | . | . | . | . | . | . | . | . | . | . | . | . | . | . | . | . | . |
| 21-225_23F12_LC | . | | . | . | . | N | . | . | . | . | . | . | . | . | . | . | . | . | . | . | . | . | . | . | . | . |
| 21-225_24B6_LC | . | | . | . | . | . | . | . | . | . | . | . | . | . | . | . | . | . | . | . | . | . | . | . | . | . |
| 21-225_25D12_LC | . | | . | . | . | . | . | . | . | . | . | . | . | . | . | . | . | . | . | . | . | . | . | . | . | . |
| 21-225_26A2_LC | . | | . | . | . | N | S | . | . | . | . | . | . | . | . | . | . | . | . | . | . | . | . | . | . | . |
| 21-225_28C7_LC | . | | . | . | . | N | T | . | . | . | . | . | . | . | . | . | . | . | . | . | . | . | . | . | . | . |
| 21-225_28F5_LC | . | | . | . | . | N | T | . | . | . | . | . | . | . | . | . | . | . | . | . | . | . | . | . | . | . |
| 21-225_2G4_LC | . | | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_32B6_LC | . | . | . | . | . | N | . | | | | | | | | | | | | | | | | | | | |
| 21-225_33C2_LC | . | . | . | . | . | N | . | | | | | | | | | | | | | | | | | | | |
| 21-225_44B3_LC | . | . | . | L | . | . | N | . | | | | | | | | | | | | | | | | | | |
| 21-225_44C10_LC | . | . | . | . | . | . | . | | | | | | | | | | | | | | | | | | | |
| 21-225_44D3_LC | . | . | . | . | . | . | . | | | | | | | | | | | | | | | | | | | |
| 21-225_44D5_LC | . | . | . | . | . | . | . | | | | | | | | | | | | | | | | | | | |
| 21-225_44F9_LC | . | . | . | N | . | . | N | . | | | | | | | | | | | | | | | | | | |
| 21-225_45B4_LC | . | . | . | . | . | . | . | | | | | | | | | | | | | | | | | | | |
| 21-225_45B8_LC | . | . | . | . | . | . | N | . | | | | | | | | | | | | | | | | | | |
| 21-225_46C3_LC | . | . | . | . | . | . | . | | | | | | | | | | | | | | | | | | | |
| 21-225_46D4_LC | . | . | . | . | . | . | . | | | | | | | | | | | | | | | | | | | |
| 21-225_48A9_LC | . | . | . | . | . | N | . | | | | | | | | | | | | | | | | | | | |
| 21-225_48C1_LC | . | . | . | . | . | N | S | | | | | | | | | | | | | | | | | | | |
| 21-225_48F2_LC | . | . | . | . | Q | . | . | | | | | | | | | | | | | | | | | | | |
| 21-225_4C12_LC | . | . | . | . | . | . | . | | | | | | | | | | | | | | | | | | | |
| 21-225_4C5_LC | . | . | . | . | . | . | . | | | | | | | | | | | | | | | | | | | |
| 21-225_50A12_LC | . | . | . | . | . | N | . | | | | | | | | | | | | | | | | | | | |
| 21-225_50H4_LC | . | . | . | . | . | . | . | | | | | | | | | | | | | | | | | | | |
| 21-225_51H8_LC | . | . | . | . | . | N | . | | | | | | | | | | | | | | | | | | | |
| 21-225_54H3_LC | . | . | . | . | Y | N | . | | | | | | | | | | | | | | | | | | | |
| 21-225_56C7_LC | . | . | . | . | . | N | . | | | | | | | | | | | | | | | | | | | |
| 21-225_57B8_LC | . | . | . | . | . | N | . | | | | | | | | | | | | | | | | | | | |
| 21-225_59B9_LC | . | . | . | . | . | N | . | | | | | | | | | | | | | | | | | | | |
| 21-225_59G7_LC | . | . | . | . | . | N | . | | | | | | | | | | | | | | | | | | | |
| 21-225_5E5_LC | . | . | . | . | . | . | . | | | | | | | | | | | | | | | | | | | |
| 21-225_5E5.003_LC | . | . | . | . | . | . | . | | | | | | | | | | | | | | | | | | | |
| 21-225_5E5.004_LC | . | . | . | . | . | . | . | | | | | | | | | | | | | | | | | | | |
| 21-225_5E5.005_LC | . | . | . | . | . | . | . | | | | | | | | | | | | | | | | | | | |
| 21-225_5E5.006_LC | . | . | . | . | . | . | . | | | | | | | | | | | | | | | | | | | |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | N | . | . | . | . | . | . | . | . | . | . | . | . | . | . | . | . | . | . | . | . |
| 21-225_60E2_LC | . | . | . | . | . | N | . | . | . | . | . | . | . | . | . | . | . | . | . | . | . | . | . | . | . | . |
| 21-225_61E6_LC | . | . | . | . | . | N | . | . | . | . | . | . | . | . | . | . | . | . | . | . | . | . | . | . | . | . |
| 21-225_61H1_LC | . | . | . | . | . | N | . | . | . | . | . | . | . | . | . | . | . | . | . | . | . | . | . | . | . | . |
| 21-225_62F7_LC | . | . | . | . | Y | N | . | . | . | . | . | . | . | . | . | . | . | . | . | . | . | . | . | . | . | . |
| 21-225_64A4_LC | . | . | . | . | . | . | . | . | . | . | . | . | . | . | . | . | . | . | . | . | . | . | . | . | . | . |
| 21-225_64C8_LC | . | . | H | . | . | . | . | . | . | . | . | . | . | . | . | . | . | . | . | . | . | . | . | . | . | . |
| 21-225_66C10_LC | . | . | . | . | Y | N | . | . | . | . | . | . | . | . | . | . | . | . | . | . | . | . | . | . | . | . |
| 21-225_66D11_LC | . | . | I | V | . | N | . | . | . | . | . | . | . | . | . | . | . | . | . | . | . | . | . | . | . | . |
| 21-225_6A11_LC | . | . | . | . | . | N | . | . | . | . | . | . | . | . | . | . | . | . | . | . | . | . | . | . | . | . |
| 21-225_6G2_LC | . | . | I | . | . | N | . | . | . | . | . | . | . | . | . | . | . | . | . | . | . | . | . | . | . | . |
| 21-225_71A2_LC | . | . | I | H | . | N | . | . | . | . | . | . | . | . | . | . | . | . | . | . | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | L | . | . | . | . | . | . | . | . | . | . | . | . | . | . | . | . | . | . | . | . | . | . |
| 21-225_7F8_LC | . | . | . | . | . | . | T | . | . | . | . | . | . | . | . | . | . | . | . | . | . | . | . | . | . | . |
| 21-225_7G2_LC | . | . | . | . | . | N | . | . | . | . | . | . | . | . | . | . | . | . | . | . | . | . | . | . | . | . |
| 21-225_7G4_LC | . | . | . | H | Q | . | . | . | . | . | . | . | . | . | . | . | . | . | . | . | . | . | . | . | . | . |
| 21-225_94F3_LC | . | . | . | . | . | . | . | . | . | . | . | . | . | . | . | . | . | . | . | . | . | . | . | . | . | . |
| 21-225_9A1_LC | . | . | I | . | . | N | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_9C11_LC | H | . | H | . | S | N | | | . | . | . | . | . | . | . | . | . | | | . | . | . | . | . | . | . |
| 21-225_9D12_LC | . | . | . | . | . | N | | | . | . | . | . | . | . | . | . | . | | | . | . | . | . | . | . | . |

4459

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | R | T | F | G | Q | G | T | K | V | E | I | K | R |
| 21-225_10E9_LC | | | | | | | | | | | | | | | | | | | |
| 21-225_12B12_LC | | | | | | | W | | | | L | | | | | V | | | |
| 21-225_12E6_LC | | | | | | | W | | | | | | | | | | | | |
| 21-225_12F2_LC | | | | | | | W | | | | | | | | | | | | |
| 21-225_147H5_LC | | | | | | | | | | | | | | | | | | | |
| 21-225_148C9_LC | | | | | | | | | | | | | | | | | | | |
| 21-225_149D10_LC | | | | | | | | | | | | | | | | | | | |
| 21-225_14E10_LC | | | | | | | | | | | | | | | | | | | |
| 21-225_158D4_LC | | | | | | | | | | | | | | | | | | | |
| 21-225_158G1_LC | | | | | | | W | | | | | | | | | | | | |
| 21-225_159A3_LC | | | | | | | | | | | | | | | | | | | |
| 21-225_159C8_LC | | | | | | | | | | | | | | | | | | | |
| 21-225_159D8_LC | | | | | | | | | | | | | | | | | | | |
| 21-225_15C11_LC | | | | | | | | | | | R | | | | | | | | |
| 21-225_160C4_LC | | | | | | | | | | | | | | | | | | | |
| 21-225_160F2_LC | | | | | | | W | | | | | | | | | | | | |
| 21-225_160H1_LC | | | | | | F | W | | | | | | | | | | | | |
| 21-225_161G12_LC | | | | | | | | | | | | | | | | | | | |
| 21-225_169D11_LC | | | | | | | | | | | | | | | | | | | |
| 21-225_169D7_LC | | | | | | C | W | | | | | | | | | | | | |
| 21-225_16A5_LC | | | | | | | W | | | | | | | | | | | | |
| 21-225_16G7_LC | | | | | | | W | | | | | | | | | | | | |
| 21-225_16H4_LC | | | | | | | W | | | | | | | | | | | | |
| 21-225_170E4_LC | | | Y | F | | | W | | | | | | | | | D | | | |
| 21-225_170F3_LC | | | | | | | | | | | | | | | | | | | |
| 21-225_170H1_LC | | | | N | | | W | | | | | | | | | | S | | |
| 21-225_174G10_LC | | | | | | | | | | | | | | | | | | | |
| 21-225_17C7_LC | | | | | | | W | | | | | | | | | | | | |
| 21-225_17H3_LC | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | R | T | F | G | Q | G | T | K | V | E | I | K | R |
| 21-225_18A5_LC | | | | | | | W | | | | | | | | | | | | |
| 21-225_18C2_LC | | | | | | | W | | | | L | | | | | V | | | |
| 21-225_18F2_LC | | | | | | | W | | | | | | | | | | | | |
| 21-225_18H12_LC | | | | | | | W | | | | L | | | | | V | | | |
| 21-225_190C12_LC | | | | | | | W | | | | | | | | | | | | |
| 21-225_190G3_LC | | | | | | | W | | | | | | | | | | | | |
| 21-225_190H5_LC | | | | | | | W | | | | | | | | | | | | |
| 21-225_191A4_LC | | | | | | | W | | | | | | | | | | | | |
| 21-225_192F7_LC | | | | | | | W | | | | | | | | | | | Y | |
| 21-225_196C12_LC | | | | | P | | P | | | | R | | | | | | | | |
| 21-225_19A10_LC | | | | | | | W | | | | | | | | | | | | |
| 21-225_200C9_LC | | | | | | | W | | | | | | | | | | | | |
| 21-225_201C5_LC | | | | | P | | | | | | | | | | | | | | |
| 21-225_203C10_LC | | | | | | | W | | | | | | | | | | | | |
| 21-225_203F7_LC | | | | | P | | | | | | | | | | | | | | |
| 21-225_204H3_LC | | | | | | | | | | | | | | | | | L | Q | |
| 21-225_208B2_LC | | | | | | | | | | | | | | | | | | | |
| 21-225_208F1_LC | | | | | P | | | | | | | | | | | | | | |
| 21-225_20B11_LC | | | | | | | | | | | | | | | | | | | |
| 21-225_20B2_LC | | | | | | | | A | | | | | | | | D | | | |
| 21-225_20D10_LC | | | | | | | W | | | | | | | | | | | | |
| 21-225_210G5_LC | | | | | | | | | | | | | | | | | | | |
| 21-225_210G6_LC | | | | | P | | | | | | | | | | | | | | |
| 21-225_212B11_LC | | | | | | | | | | | | | | | | | | | |
| 21-225_214G10_LC | | | | | | | | | | | | | | | | | | | |
| 21-225_214G12_LC | | | | | | | | | | | | | | | | | | | |
| 21-225_214H9_LC | | | | | | | | | | | | | | | | | | | |
| 21-225_215E11_LC | | | | | | | | | | | | | | | | | | | |
| 21-225_215E3_LC | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | R | T | F | G | Q | G | T | K | V | E | I | K | R |
| 21-225_216E8_LC | | | | | | | | | | | | | | | | | | | |
| 21-225_216H12_LC | | | | | | | | | | | | | | | | | | | |
| 21-225_217E5_LC | | | | | | | | | | | | | | | | | | | |
| 21-225_217G10_LC | | | | | | | | | | | | | | | | | | | |
| 21-225_217H12_LC | | | | | | | | | | | | | | | | | | | |
| 21-225_218C4_LC | | | | | | | | | | | | | | | | | | | |
| 21-225_21F7_LC | | | | | | | W | | | | | | | | | | | | |
| 21-225_21G11_LC | | | | | | | W | | | | L | | | | | V | | | |
| 21-225_21G2_LC | | | | | | | W | | | | L | | | | | V | | | |
| 21-225_21H8_LC | | | | | | | W | | | | | | | | | | | | |
| 21-225_220F8_LC | | | | | | | | | | | | | | | | | | | |
| 21-225_224E7_LC | | | | | | | | | | | | | | | | | | | |
| 21-225_224F1_LC | | | | | | | | | | | | | | | | | | | |
| 21-225_224F3_LC | | | | | | | | A | | | | | | | | | | | |
| 21-225_225A1_LC | | | | | | | | | | | | | | | | | | | |
| 21-225_226C6_LC | | | | | | | | A | | | | | | | | | | Q | |
| 21-225_226F7_LC | | | | | | | | | | | | | | | | | | | |
| 21-225_226H11_LC | | | | | | | | | | | | | | | | | | | |
| 21-225_22A4_LC | | | | | | | W | | | | | | | | | | | | |
| 21-225_22F4_LC | | | | | | | | | | | | | | | | | | | |
| 21-225_23A10_LC | | | | | | | | | | | L | | | | | | | | |
| 21-225_23E11_LC | | | | | | | W | | | | | | | | | | | | |
| 21-225_23F12_LC | | | | | | | W | | | | | | | | | | | | |
| 21-225_24B6_LC | | | | | | | | | | | | | | | | | V | | |
| 21-225_25D12_LC | | | | | P | | | | | | | | | | | | | | |
| 21-225_26A2_LC | | | | | | | W | | | | | | | | | | | | |
| 21-225_28C7_LC | | | | | | | W | | | | | | | | | | | | |
| 21-225_28F5_LC | | | | | | | W | | | | | | | | | | V | | |
| 21-225_2G4_LC | | | | | P | | | | | | S | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | R | T | F | G | Q | G | T | K | V | E | I | K | R |
| 21-225_32B6_LC | . | . | . | . | . | . | W | . | . | . | . | . | . | . | . | . | . | . | . |
| 21-225_33C2_LC | . | . | . | . | . | . | W | . | . | . | . | . | . | . | . | . | . | . | . |
| 21-225_44B3_LC | . | . | . | . | . | . | . | . | . | . | . | . | . | . | . | . | . | . | . |
| 21-225_44C10_LC | . | . | . | . | . | . | . | . | . | . | . | . | . | . | . | . | . | . | . |
| 21-225_44D3_LC | . | . | . | . | . | . | . | . | . | . | . | . | . | . | . | . | . | . | . |
| 21-225_44D5_LC | . | . | . | . | F | . | W | . | . | . | . | . | . | . | . | . | . | . | . |
| 21-225_44F9_LC | . | . | . | . | . | . | . | . | . | . | . | . | . | . | . | . | . | T | . |
| 21-225_45B4_LC | . | . | . | . | . | . | . | . | . | . | . | . | . | . | . | . | . | . | . |
| 21-226_45B8_LC | . | . | . | . | . | . | . | . | . | . | . | . | . | . | . | . | . | . | . |
| 21-226_46C3_LC | . | . | . | . | . | . | . | . | . | . | . | . | . | . | . | . | . | . | . |
| 21-225_46D4_LC | . | . | . | . | F | . | W | . | . | . | . | . | . | . | . | . | . | . | . |
| 21-225_48A9_LC | . | . | . | . | . | . | W | . | . | . | . | . | . | . | . | . | . | . | . |
| 21-225_48C1_LC | . | . | . | . | . | . | W | . | . | . | . | . | . | . | . | . | . | . | . |
| 21-225_48F2_LC | . | . | . | . | . | . | . | . | . | . | . | . | . | . | . | . | . | . | . |
| 21-225_4C12_LC | . | . | . | . | F | . | . | . | . | . | . | . | . | . | . | . | . | . | . |
| 21-225_4C5_LC | . | . | . | . | . | . | . | . | . | . | . | . | . | . | . | . | . | . | . |
| 21-225_50A12_LC | . | . | . | . | . | . | W | . | . | . | . | . | . | . | . | . | . | . | . |
| 21-225_50H4_LC | . | . | . | . | . | . | . | . | . | . | . | . | . | . | . | . | . | . | . |
| 21-225_51H8_LC | . | . | . | . | . | . | W | . | . | . | . | . | . | . | . | . | . | . | . |
| 21-225_54H3_LC | . | . | . | . | . | . | W | . | . | . | . | . | . | . | . | . | . | . | . |
| 21-225_56C7_LC | . | . | . | . | . | . | W | . | . | . | . | . | . | . | . | . | . | . | . |
| 21-225_57B8_LC | . | . | F | F | . | . | W | . | . | . | . | . | . | . | . | . | . | . | . |
| 21-225_59B9_LC | . | . | . | . | . | . | W | . | . | . | . | . | . | . | . | . | . | . | . |
| 21-225_59G7_LC | . | . | . | . | . | . | W | . | . | . | . | . | . | . | . | . | . | . | . |
| 21-225_5E5_LC | . | . | . | . | . | . | . | . | . | . | . | . | . | . | . | . | V | . | . |
| 21-225_5E5.003_LC | . | . | . | . | . | . | . | . | . | . | . | . | . | . | . | . | V | . | . |
| 21-225_5E5.004_LC | . | . | . | . | . | . | . | . | . | . | . | . | . | . | . | . | V | . | . |
| 21-225_5E5.005_LC | . | . | . | . | . | . | . | . | . | . | . | . | . | . | . | . | V | . | . |
| 21-225_5E5.006_LC | . | . | . | . | . | . | . | . | . | . | . | . | . | . | . | . | V | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | R | T | F | G | Q | G | T | K | V | E | I | K | R |
| 21-225_5E5.011_LC | . | . | . | . | . | . | . | . | . | . | . | . | . | . | . | . | V | . | . |
| 21-225_5E5.012_LC | . | . | . | . | . | . | . | . | . | . | . | . | . | . | . | . | V | . | . |
| 21-225_5E5.015_LC | . | . | . | . | . | . | . | . | . | . | . | . | . | . | . | . | V | . | . |
| 21-225_5E5.016_LC | . | . | . | . | . | . | . | . | . | . | . | . | . | . | . | . | V | . | . |
| 21-225_5E5.017_LC | . | . | . | . | . | . | . | . | . | . | . | . | . | . | . | . | V | . | . |
| 21-225_5E5.018_LC | . | . | . | . | . | . | . | . | . | . | . | . | . | . | . | . | V | . | . |
| 21-225_5E5.019_LC | . | . | . | . | . | . | . | . | . | . | . | . | . | . | . | . | V | . | . |
| 21-225_5E5.023_LC | . | . | . | . | . | . | . | . | . | . | . | . | . | . | . | . | V | . | . |
| 21-225_5E5.024_LC | . | . | . | . | . | . | . | . | . | . | . | . | . | . | . | . | V | . | . |
| 21-225_5E5.025_LC | . | . | . | . | . | . | . | . | . | . | . | . | . | . | . | . | V | . | . |
| 21-225_60C3_LC | . | . | . | . | . | . | W | . | . | . | L | . | . | . | . | . | . | . | . |
| 21-225_60E2_LC | . | . | . | . | . | . | W | . | . | . | . | . | . | . | . | . | . | . | . |
| 21-225_61E6_LC | . | . | P | P | . | . | W | . | . | . | . | . | . | . | . | . | . | . | . |
| 21-225_61H1_LC | . | . | . | . | . | . | W | . | . | . | . | . | . | . | . | . | . | . | . |
| 21-225_62F7_LC | . | . | Y | P | . | . | W | . | . | . | . | . | S | . | . | . | . | . | . |
| 21-225_64A4_LC | . | . | . | . | . | . | . | . | . | . | . | . | . | . | . | . | . | . | . |
| 21-225_64C8_LC | . | . | . | . | . | . | . | . | . | . | . | . | . | . | . | . | . | . | . |
| 21-225_66C10_LC | . | . | Y | P | . | . | W | . | . | . | . | . | S | . | . | . | . | . | . |
| 21-225_66D11_LC | . | . | . | . | . | . | . | . | . | . | . | . | . | . | . | . | . | . | . |
| 21-225_6A11_LC | . | . | . | . | . | . | W | . | . | . | . | . | . | . | . | . | . | . | . |
| 21-225_6G2_LC | . | . | . | . | . | L | W | . | . | . | . | . | . | . | . | . | T | . | . |
| 21-225_71A2_LC | . | . | . | . | . | . | W | . | . | . | . | . | . | . | . | . | . | . | . |
| 21-225_73C9_LC | . | . | . | . | . | . | . | . | . | . | . | . | . | . | . | . | . | . | . |
| 21-225_7C9_LC | . | . | . | . | . | . | . | . | . | . | . | . | . | . | . | D | . | . | . |
| 21-225_7F8_LC | . | . | . | . | . | . | . | . | . | . | . | . | . | . | . | . | . | . | . |
| 21-225_7G2_LC | . | . | . | . | . | . | W | . | . | . | L | . | . | . | . | V | . | . | . |
| 21-225_7G4_LC | . | . | . | . | . | . | . | . | . | . | . | . | . | . | . | . | . | . | . |
| 21-225_94F3_LC | . | . | . | . | . | . | . | . | . | . | R | . | . | . | . | A | . | . | . |
| 21-225_9A1_LC | . | . | . | . | . | L | W | . | . | . | . | . | . | . | . | . | . | . | . |

4464

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | R | T | F | G | Q | G | T | K | V | E | I | K | R |
| 21-225_9C11_LC | . | . | . | . | T | . | L | . | . | . | . | . | . | . | . | . | . | . | . |
| 21-225_9D12_LC | . | . | . | . | . | . | W | . | . | . | . | . | . | . | . | . | . | . | . |

**FIGURE 57**

Table 99.    Consensus 19 - VK4|B3/JK1 (SEQ ID NO: 50316):

DIVMTQSPDSLAVSLGERATINCKSS--QSVLHSSNNNNYLAWYQQKPGQPPKLLIYW-------ASTRESGVPDRFSGSGSG--
TDFTLTISSLQAEDVAVYYCQQYYS---------------------TPPTFGQGTKVEIKR wherein:

K at position 24 can be substituted with R or M

S at position 26 can be substituted with G or R

S at position 30 can be substituted with T, N or I

V at position 31 can be substituted with I or A

H at position 33 can be substituted with Y, F, S, K, D, L or M

S at position 34 can be substituted with T, R, N, I or D

S at position 35 can be substituted with F or P

N at position 36 can be substituted with H

N at position 37 can be substituted with K, S, D or H

N at position 38 can be substituted with Y, K, H, R, A, F or W

N at position 39 can be substituted with H or Y

Y at position 40 can be substituted with S

L at position 41 can be substituted with F

A at position 42 can be substituted with T, V or G

A at position 67 can be substituted with T or S

S at position 68 can be substituted with F

T at position 69 can be substituted with I, K or S

FIGURE 57

R at position 70 can be substituted with W or L

E at position 71 can be substituted with K, A, D or R

S at position 72 can be substituted with T

Q at position 107 can be substituted with H or L

Q at position 108 can be substituted with H

Y at position 109 can be substituted with F

Y at position 110 can be substituted with F, H, N, L or S

S at position 111 can be substituted with N, R, D, I, T, C, E or K

T at position 135 can be substituted with S, I, V, A or Y

P at position 137 can be substituted with W, L, V, C, G, R or S

T at position 138 can be substituted with K or S

Xaa Xaa Xaa Gln Ser Xaa Leu Xaa Xaa Xaa Xaa Xaa Xaa Xaa Xaa Xaa (SEQ ID NO: 50133)

Trp Xaa Xaa Xaa Xaa Xaa Xaa (SEQ ID NO: 50229)

Xaa Xaa Xaa Xaa Xaa Xaa Pro Xaa Xaa (SEQ ID NO: 50134)

Lys Ser Ser Gln Ser Val Leu His Ser Ser Asn Asn Asn Asn Tyr Leu Ala (SEQ ID NO: 50618)

Trp Ala Ser Thr Arg Glu Ser (SEQ ID NO: 50619)

Gln Gln Tyr Tyr Ser Thr Pro Pro Thr (SEQ ID NO: 50620)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | V | M | T | Q | S | P | D | S | L | A | V | S | L | G | E | R | A | T | I | N | C | K | S | S |
| 21-225_11E5_LC | . | . | . | . | . | . | . | . | . | . | . | . | . | . | . | . | . | . | . | . | L | . | . | . | . | . |
| 21-225_12F12_LC | . | . | . | . | . | . | . | . | . | . | . | . | . | . | . | . | . | . | . | . | . | . | . | . | . | . |
| 21-225_147E4_LC | . | . | . | . | . | . | . | . | . | . | . | . | . | . | . | . | . | . | . | . | . | . | . | . | . | . |
| 21-225_148F8_LC | . | . | . | . | . | . | L | . | . | . | . | . | . | . | . | . | . | . | . | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | . | . | . | . | S | . | . | . | . |
| 21-225_149H4_LC | . | . | . | . | . | . | . | . | . | . | . | . | . | . | . | . | . | . | . | . | . | . | . | . | . | . |
| 21-225_154A11_LC | . | . | . | . | . | . | . | . | . | . | . | . | . | . | . | . | . | . | . | . | . | . | . | P | . | . |
| 21-225_158B12_LC | . | . | . | . | . | . | . | . | . | . | P | . | . | . | . | . | . | . | . | . | . | . | . | . | . | . |
| 21-225_162F6_LC | . | . | . | . | . | . | . | . | . | . | . | . | . | . | . | . | . | . | . | . | . | . | . | . | . | . |
| 21-225_164A4_LC | . | . | . | . | . | . | Y | . | . | . | R | . | . | . | . | . | . | . | . | . | . | K | . | . | . | . |
| 21-225_164A7_LC | . | . | . | . | . | . | C | . | . | . | . | . | . | . | . | . | . | . | . | . | . | . | . | . | . | . |
| 21-225_170F6_LC | . | . | . | . | . | . | . | . | . | . | . | . | . | . | . | . | A | . | . | . | . | . | . | . | . | . |
| 21-225_174G5_LC | . | . | . | . | . | . | . | . | . | . | . | . | . | . | . | . | A | . | . | . | . | . | . | . | . | . |
| 21-225_175D10_LC | . | . | . | . | . | . | . | . | . | . | . | . | . | . | . | . | . | . | . | . | . | . | . | . | . | . |
| 21-225_177B12_LC | . | . | . | . | . | . | . | . | . | . | . | . | . | . | . | . | . | . | . | . | V | . | . | . | . | . |
| 21-225_181E5_LC | . | . | . | . | . | . | . | . | . | . | . | . | . | . | . | . | . | . | . | . | . | . | . | . | . | . |
| 21-225_190D7_LC | . | . | . | . | . | . | . | . | . | . | . | . | . | . | . | . | . | . | . | . | . | . | . | . | . | . |
| 21-225_191D8_LC | . | . | M | . | . | . | . | . | . | . | . | . | . | . | . | . | . | . | . | . | I | S | . | R | . | . |
| 21-225_193G4_LC | . | . | . | . | . | . | . | . | . | . | . | . | . | . | . | . | . | . | . | . | . | . | . | . | . | . |
| 21-225_196G8_LC | . | . | . | . | . | . | . | . | . | . | T | . | . | . | . | . | . | . | . | . | V | . | . | . | . | . |
| 21-225_197C6_LC | . | . | M | . | . | . | . | . | . | . | . | . | . | . | . | . | . | . | . | . | I | S | . | P | . | . |
| 21-225_197H4_LC | . | . | M | . | . | . | . | S | . | . | T | . | . | . | . | . | . | . | . | . | I | S | . | P | . | . |
| 21-225_200G7_LC | . | . | . | . | . | . | . | . | . | . | P | Y | A | . | . | . | . | . | . | . | V | K | . | . | . | . |
| 21-225_201F5_LC | . | . | . | . | . | . | . | S | . | . | . | . | . | . | . | . | . | . | . | . | . | . | . | . | . | . |
| 21-225_213H12_LC | . | . | . | . | . | . | . | . | . | . | . | . | . | . | . | . | . | . | . | . | . | T | . | . | . | . |
| 21-225_213H7_LC | . | . | . | . | . | . | . | . | . | . | . | . | . | . | . | . | . | . | . | . | . | . | . | . | . | . |
| 21-225_222H3_LC | . | . | . | . | . | . | . | . | . | . | . | . | . | . | . | . | . | . | . | . | . | . | . | . | . | . |
| 21-225_223G10_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | S | P | D | S | L | A | V | S | L | G | E | R | A | T | I | N | C | K | S | S |
| 21-225_224D8_LC | . | . | . | . | . | . | . | . | . | . | . | . | . | . | . | . | . | . | . | A | . | . | . | . | . | . |
| 21-225_224H5_LC | . | . | . | . | . | . | . | . | . | . | . | . | . | . | . | . | . | . | . | . | . | . | . | . | . | . |
| 21-225_225F11_LC | . | . | . | . | . | . | . | . | . | . | . | . | . | . | . | . | . | . | . | . | . | . | . | . | . | . |
| 21-225_225F5_LC | . | . | . | . | . | . | . | . | . | . | . | . | . | . | . | . | . | . | . | . | . | . | . | . | . | . |
| 21-225_226A12_LC | . | . | . | . | . | . | . | . | . | . | . | . | . | . | . | . | . | . | . | . | . | . | . | . | . | . |
| 21-225_226D11_LC | . | . | . | . | . | . | . | . | . | . | . | . | . | . | . | . | . | . | . | . | . | . | . | . | . | . |
| 21-225_226F6_LC | . | . | . | . | . | . | . | . | . | . | . | . | . | . | . | . | . | . | . | . | . | . | . | . | . | . |
| 21-225_227C7_LC | . | . | . | . | . | . | . | . | . | . | . | . | . | . | . | . | . | . | . | . | . | . | . | . | . | . |
| 21-225_23A12_LC | . | . | . | . | . | . | . | . | . | . | . | . | . | . | . | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | . | . | . | . | . | . | . | . | . | . | . | . | . | . | . | . | . | . | . | . | . | . | . | . | . | . |
| 21-225_25A4_LC | . | . | . | . | . | . | S | . | . | . | . | . | . | . | . | . | . | . | . | . | . | N | . | . | . | . |
| 21-225_25A4.001_LC | . | . | . | . | . | . | S | . | . | . | . | . | . | . | . | . | . | . | . | . | . | N | . | . | . | . |
| 21-225_25A4.001.001_LC | . | . | . | . | . | . | S | . | . | . | . | . | . | . | . | . | . | . | . | . | . | N | . | . | . | . |
| 21-225_25A4.001.002_LC | . | . | . | . | . | . | S | . | . | . | . | . | . | . | . | . | . | . | . | . | . | N | . | . | . | . |
| 21-225_25A4.001.003_LC | . | . | . | . | . | . | S | . | . | . | . | . | . | . | . | . | . | . | . | . | . | N | . | . | . | . |
| 21-225_25A4.001.004_LC | . | . | . | . | . | . | S | . | . | . | . | . | . | . | . | . | . | . | . | . | . | N | . | . | . | . |
| 21-225_25A4.001.005_LC | . | . | . | . | . | . | . | . | . | . | . | . | . | . | . | . | . | . | . | . | . | N | . | . | . | . |
| 21-225_25A4.001.006_LC | . | . | . | . | . | . | . | . | . | . | . | . | . | . | . | . | . | . | . | . | . | N | . | . | . | . |
| 21-225_25A4.001.007_LC | . | . | . | . | . | . | . | . | . | . | . | . | . | . | . | . | . | . | . | . | . | N | . | . | . | . |
| 21-225_25A4.001.008_LC | . | . | . | . | . | . | . | . | . | . | . | . | . | . | . | . | . | . | . | . | . | N | . | . | . | . |
| 21-225_25A4.001.013_LC | . | . | . | . | . | . | S | . | . | . | . | . | . | . | . | . | . | . | . | . | . | N | . | . | . | . |
| 21-225_25A4.001.019_LC | . | . | . | . | . | . | . | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . | . | . | . | . | . | . | . |
| 21-225_25A4.001.029_LC | . | . | . | . | . | . | S | . | . | . | . | . | . | . | . | . | . | . | . | . | . | N | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | . | . | . | S | . | . | . | . | . | . | . | . | . | . | . | . | . | . | . | . | . | . | . |
| 21-225_29H8_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | S | P | D | S | L | A | V | S | L | G | E | R | A | T | I | N | C | K | S | S |
| 21-225_43D8_LC | . | . | . | . | . | . | . | . | . | . | . | . | . | . | . | . | . | . | . | . | . | . | . | . | . | . |
| 21-225_4A2.001.023_LC | . | . | . | . | . | . | . | . | . | . | . | . | . | . | . | . | . | . | . | . | . | . | . | . | . | . |
| 21-225_4A2.001.028_LC | . | . | . | . | . | . | . | . | . | . | . | . | . | . | . | . | . | . | . | . | . | . | . | . | . | . |
| 21-225_4A2.001.029_LC | . | . | . | . | . | . | . | . | . | . | . | . | . | . | . | . | . | . | . | . | . | . | . | . | . | . |
| 21-225_4A2.001.031_LC | . | . | . | . | . | . | . | . | . | . | . | . | . | . | . | . | . | . | . | . | . | . | . | . | . | . |
| 21-225_56A1_LC | . | . | . | . | . | . | . | . | . | . | . | . | . | . | . | . | . | . | . | . | . | . | . | . | . | . |
| 21-225_57A11_LC | . | . | . | . | . | . | . | . | . | . | T | . | . | . | . | . | . | . | . | . | . | . | . | . | . | . |
| 21-225_58B9_LC | . | . | . | . | . | . | . | . | . | . | . | . | . | . | . | . | . | . | . | . | . | . | . | . | . | s |
| 21-225_59F10_LC | . | . | . | . | . | . | P | . | . | . | . | . | . | . | . | . | . | . | . | . | . | . | . | . | . | . |
| 21-225_61B5_LC | . | . | . | . | . | . | . | . | . | . | . | . | . | . | . | . | . | . | . | . | . | . | . | . | . | . |
| 21-225_70E7_LC | . | . | . | . | . | . | . | . | . | . | . | . | . | . | . | . | . | . | . | . | . | k | . | . | . | . |
| 21-225_74A6_LC | . | . | . | . | . | . | . | . | . | . | . | . | . | . | P | . | . | . | . | . | . | . | . | . | . | . |
| 21-225_74A7_LC | . | . | . | . | . | . | . | . | . | . | . | . | . | . | . | . | . | . | . | . | . | . | . | . | . | . |
| 21-225_74B1_LC | . | . | . | . | . | . | P | . | . | . | . | . | . | . | . | . | . | . | . | . | T | . | . | . | . | . |
| 21-225_74B10_LC | . | . | . | . | . | . | . | . | . | . | . | . | . | . | . | . | . | . | . | . | . | . | . | . | . | . |
| 21-225_74B5_LC | . | . | . | . | . | . | . | . | . | . | . | . | . | . | . | . | . | . | . | . | . | . | . | . | . | . |
| 21-225_74B7_LC | . | . | . | . | . | . | . | . | . | . | . | . | . | . | . | . | . | . | . | . | . | . | . | . | . | . |
| 21-225_74B8_LC | . | S | . | . | . | . | . | . | . | . | . | . | . | . | . | . | . | . | . | . | . | . | . | . | . | . |
| 21-225_74C1_LC | . | . | . | . | . | . | C | . | . | P | . | . | . | . | . | . | . | . | . | S | . | . | . | . | . | . |
| 21-225_74F6_LC | . | . | . | . | . | . | . | . | . | . | . | . | . | . | . | . | . | . | . | . | . | . | . | . | . | . |
| 21-225_74F9_LC | . | . | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | . | . | . | . | . | . | . | . | . | . | . | . | . | . | . |
| 21-225_75A10_LC | N | . | . | . | . | . | . | . | . | . | . | . | . | . | . | . | . | A | . | . | . | . | . | . | . | . |
| 21-225_75A7_LC | . | . | . | . | . | . | . | . | . | . | . | . | . | . | . | . | . | . | . | . | . | . | . | . | . | . |
| 21-225_75C4_LC | . | . | . | . | . | . | . | . | . | . | . | . | . | . | . | . | . | . | . | . | . | . | . | . | . | . |
| 21-225_75F9_LC | . | . | . | . | . | . | . | . | . | . | P | . | . | . | . | . | . | . | . | . | V | . | . | . | . | . |
| 21-225_76E11_LC | . | . | . | . | . | . | . | . | . | . | . | . | . | . | . | . | . | G | . | . | . | . | . | . | . | X |
| 21-225_76E8_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | V | M | T | Q | S | P | D | S | L | A | V | S | L | G | E | R | A | T | I | N | C | K | S | S |
| 21-225_76F3_LC | . | . | . | . | . | . | C | . | . | . | P | . | . | . | . | . | . | . | . | . | . | . | . | . | . | . |
| 21-225_76F5_LC | V | . | . | L | . | . | . | . | . | . | . | . | . | . | . | . | . | . | . | . | . | . | . | . | . | . |
| 21-225_76G2_LC | . | . | . | . | . | . | . | . | . | . | . | . | . | . | . | . | . | . | . | . | . | . | . | . | . | . |
| 21-225_77C10_LC | . | . | . | . | . | . | . | . | . | C | . | . | . | . | . | . | . | . | . | . | . | . | . | . | . | . |
| 21-225_77C7_LC | . | . | . | . | . | . | . | . | . | . | . | . | . | . | . | . | . | . | . | . | . | . | . | . | . | . |
| 21-225_77E1_LC | . | . | . | . | . | . | . | . | . | . | . | . | . | . | . | . | . | . | . | . | . | . | . | . | . | . |
| 21-225_78C1_LC | . | . | . | . | . | . | . | . | . | C | . | . | . | . | . | . | . | . | . | . | . | . | . | . | . | . |
| 21-225_78E11_LC | . | . | . | . | . | . | . | . | . | . | . | . | . | . | . | . | . | . | . | . | . | . | . | . | . | . |
| 21-225_78H12_LC | . | . | . | . | . | . | . | . | . | . | . | . | . | . | . | . | . | . | . | . | . | . | . | . | . | . |
| 21-225_79E9_LC | . | . | . | . | . | . | . | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | . | . | . | . | . | . | . | . | . | . | . | . | . | . | . | . | . | . | . | . | . | . | . | . | . | . |
| 21-225_79G6_LC | . | . | . | . | . | . | . | . | . | C | . | . | . | . | . | . | . | . | . | . | . | S | . | M | . | . |
| 21-225_80D3_LC | . | . | . | . | . | . | . | . | . | C | . | . | . | . | . | . | . | . | . | . | . | . | . | . | . | . |
| 21-225_80E9_LC | . | . | . | . | . | . | . | . | . | C | . | . | . | . | . | . | . | . | . | . | . | . | . | . | . | . |
| 21-225_80H3_LC | . | . | . | . | . | . | . | . | . | . | . | . | . | . | . | . | . | . | . | T | . | . | . | . | . | . |
| 21-225_81E5_LC | . | . | . | . | . | . | . | . | . | . | . | . | . | . | . | . | . | . | . | . | . | . | . | . | . | . |
| 21-225_81F9_LC | . | . | . | . | . | . | . | . | . | C | . | . | . | . | . | . | . | . | . | . | . | . | . | . | . | . |
| 21-225_83F3_LC | . | . | . | . | . | . | . | . | . | C | . | . | . | . | . | . | . | . | . | . | . | . | . | . | . | . |
| 21-225_83G3_LC | . | . | . | . | . | . | . | . | . | C | . | . | . | . | . | . | . | . | . | . | . | . | . | . | . | . |
| 21-225_83G7_LC | . | . | . | . | . | . | . | . | . | . | . | . | . | . | . | . | . | . | . | . | . | I | . | . | . | . |
| 21-225_84G7_LC | . | . | . | . | . | . | . | . | . | . | P | . | . | . | . | . | . | . | . | . | . | I | . | . | . | . |
| 21-225_85H2_LC | . | S | M | . | . | . | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | T | . | . | . | . | . |
| 21-225_86E9_LC | . | . | . | . | . | . | C | . | . | . | P | . | . | . | . | . | . | . | . | . | . | . | . | . | . | . |
| 21-225_88A1_LC | N | . | . | . | . | . | . | . | . | . | . | . | . | . | . | . | A | . | . | . | . | . | . | . | . | . |
| 21-225_88B4_LC | . | . | . | . | . | . | . | . | . | . | . | . | . | . | . | . | . | . | . | . | . | . | . | . | . | . |
| 21-225_88C10_LC | . | . | . | . | . | . | . | . | . | . | . | . | . | . | . | . | . | . | . | . | . | . | . | . | . | . |
| 21-225_88G2_LC | . | . | . | . | . | . | . | . | . | C | . | . | . | . | . | . | . | . | . | . | . | . | . | . | . | . |

4471

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | V | M | T | Q | S | P | D | S | L | A | V | S | L | G | E | R | A | T | I | N | C | K | S | S |
| 21-225_90D9_LC | . | . | . | . | . | . | . | . | . | . | . | . | . | . | . | . | . | . | . | . | . | . | ¡ | . | . | . |
| 21-225_91F1_LC | . | . | . | . | . | . | . | . | . | G | . | . | . | . | . | . | . | . | . | . | . | . | . | . | . | . |
| 21-225_91G8_LC | . | . | . | . | . | . | . | . | . | G | . | . | . | . | . | . | . | . | . | . | . | . | . | . | . | . |
| 21-225_92E6_LC | . | . | . | . | . | . | . | . | . | . | . | . | A | . | . | . | . | . | . | . | . | . | . | . | . | . |
| 21-225_92F12_LC | . | . | . | . | . | . | . | . | . | . | . | . | . | . | . | . | . | . | . | . | T | . | . | . | . | . |
| 21-225_94D2_LC | . | . | . | . | . | . | . | . | . | . | . | . | . | . | . | . | . | . | . | . | . | . | . | . | . | . |
| 21-225_94E11_LC | . | . | . | . | . | . | . | . | . | . | . | . | . | . | . | . | . | . | . | . | . | . | . | . | . | . |
| 21-225_94E12_LC | . | . | . | . | . | . | . | . | . | . | . | . | . | . | . | . | . | . | . | . | T | . | . | . | . | . |
| 21-225_95E12_LC | . | . | . | . | . | . | . | . | . | . | . | . | . | . | . | . | . | . | . | . | . | . | . | . | . | . |
| 21-225_95G4_LC | . | . | . | . | . | G | . | . | . | . | . | . | . | . | . | . | . | . | . | . | . | . | . | . | . | . |
| 21-225_95H4_LC | . | . | . | . | . | G | . | . | . | . | . | . | . | . | . | . | . | . | . | . | . | . | . | . | . | . |
| 21-225_96D10_LC | . | . | . | . | . | . | . | . | . | . | . | . | . | . | . | . | . | . | . | . | . | . | . | . | . | . |
| 21-225_96D5_LC | . | . | . | . | . | . | . | . | . | . | . | . | . | . | . | . | . | . | . | . | . | . | . | . | . | . |
| 21-225_96E2_LC | . | S | . | . | . | . | . | . | . | . | . | . | . | . | . | . | . | . | . | . | . | . | . | . | . | . |
| 21-225_96H5_LC | . | . | . | . | . | . | . | . | . | . | . | . | . | . | . | . | . | . | . | . | . | . | . | . | . | . |
| 21-225_97H4_LC | . | . | . | . | . | . | . | . | . | G | . | . | . | . | . | . | . | . | . | . | . | . | . | . | . | . |

4472

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | V | L | H | S | S | N | N | N | N | Y | L | A | W | Y | Q | Q | K | P | G | Q | P | P |
| 21-225_11E5_LC | . | . | . | . | . | . | Y | . | . | . | . | . | . | . | . | T | . | . | . | . | . | . | . | . | P | . |
| 21-225_12F12_LC | . | . | T | . | . | . | S | . | . | . | . | . | H | . | . | . | . | . | . | . | . | . | . | . | . | . |
| 21-225_147E4_LC | . | . | . | . | . | . | . | . | . | . | . | Y | . | . | . | . | . | . | . | . | L | . | . | . | . | . |
| 21-225_148F8_LC | . | . | . | A | . | . | . | . | . | . | . | Y | . | . | . | . | . | . | . | . | . | . | . | . | . | . |
| 21-225_149A2_LC | . | . | . | . | . | . | Y | . | P | . | . | . | . | . | . | . | . | . | . | . | . | . | . | . | . | . |
| 21-225_149E3_LC | . | . | T | . | . | . | . | N | . | . | . | H | . | . | S | . | . | . | . | . | . | . | . | . | . | . |
| 21-225_149H4_LC | . | . | . | . | . | . | S | . | . | . | O | . | . | . | . | . | . | . | . | . | . | . | . | R | . | . |
| 21-225_154A11_LC | . | . | . | . | . | . | . | . | . | . | . | Y | . | . | . | . | . | . | . | . | . | . | . | . | . | . |
| 21-225_158B12_LC | . | . | N | . | . | . | . | . | . | . | . | . | . | . | . | T | . | . | . | . | R | . | . | . | . | . |
| 21-225_162F6_LC | . | . | N | . | . | . | . | . | . | . | . | . | . | . | . | T | . | . | . | . | R | . | . | . | . | . |
| 21-225_164A4_LC | . | . | . | . | . | . | Y | . | . | . | . | H | . | . | . | T | . | . | . | . | . | . | . | . | . | . |
| 21-225_164A7_LC | . | . | . | . | . | . | . | . | . | . | . | . | . | . | . | . | . | . | . | . | . | . | . | . | . | N |
| 21-225_170F6_LC | . | . | . | . | . | . | . | . | . | . | . | Y | . | . | . | . | . | . | . | . | R | . | . | . | . | . |
| 21-225_174G5_LC | . | . | . | . | . | . | . | . | . | . | . | Y | . | . | . | T | . | . | . | . | . | . | S | . | . | . |
| 21-225_175D10_LC | . | . | . | . | . | . | Y | . | . | . | . | . | . | . | . | . | . | . | . | . | . | . | . | . | . | . |
| 21-225_177B12_LC | . | . | . | . | . | . | . | . | . | . | . | . | . | . | . | T | . | . | . | . | . | . | . | . | . | . |
| 21-225_181E5_LC | . | . | . | . | . | . | . | . | . | . | . | Y | . | . | . | T | . | . | . | . | . | . | . | . | . | . |
| 21-225_190D7_LC | . | . | . | . | . | . | S | . | . | . | . | Y | . | . | . | V | . | . | . | . | . | . | . | . | . | . |
| 21-225_191D8_LC | . | . | . | . | . | . | . | . | . | . | . | Y | . | . | . | . | . | . | . | . | . | . | . | N | . | . |
| 21-225_193G4_LC | . | . | . | . | . | . | Y | . | . | . | . | Y | . | . | . | T | . | . | . | . | . | . | . | . | . | . |
| 21-225_196G8_LC | . | . | . | . | . | . | . | . | . | . | . | . | . | . | . | . | . | . | . | . | . | . | . | . | . | . |
| 21-225_197C6_LC | . | . | . | . | . | . | . | . | . | . | . | Y | . | . | . | . | . | . | . | . | . | . | . | R | . | . |
| 21-225_197H4_LC | . | . | . | . | . | . | . | . | P | . | . | Y | . | . | . | . | . | . | . | . | . | A | . | R | . | . |
| 21-225_200G7_LC | . | . | . | . | . | . | . | . | . | . | . | . | . | . | . | . | . | . | . | . | . | . | . | . | . | . |
| 21-225_201F5_LC | . | . | . | . | . | . | Y | . | . | . | . | H | . | . | . | V | . | . | . | . | . | . | . | . | . | . |
| 21-225_213H12_LC | . | . | N | . | . | . | K | T | . | . | . | H | . | . | . | . | . | . | . | . | . | . | . | . | . | . |
| 21-225_213H7_LC | . | . | N | . | . | . | O | I | . | . | . | K | . | S | . | G | . | P | . | . | . | . | . | . | . | . |
| 21-225_222H3_LC | . | . | . | . | . | . | H | . | . | M | . | S | . | . | . | . | . | . | . | . | . | . | . | . | . | . |
| 21-225_223G10_LC | . | . | . | I | . | . | L | . | . | . | . | H | . | . | . | . | . | R | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | V | L | H | S | S | N | N | N | N | Y | L | A | W | Y | Q | Q | K | P | G | Q | P | P |
| 21-225_224D8_LC | . | . | . | . | . | . | Y | . | . | . | . | N | . | . | . | . | . | . | . | . | . | . | . | . | . | . |
| 21-225_224H5_LC | . | . | . | . | . | . | Y | . | . | . | . | P | . | . | . | T | . | . | . | . | . | . | . | . | . | . |
| 21-225_225F11_LC | . | . | . | . | . | . | Y | . | . | . | . | Y | . | . | . | . | . | . | . | . | N | . | . | . | . | . |
| 21-225_225F5_LC | . | . | . | N | . | . | Y | N | . | . | . | . | . | . | . | . | . | . | . | . | . | . | . | . | . | . |
| 21-225_226A12_LC | . | . | . | N | . | . | Y | . | . | . | . | . | . | . | . | . | . | . | . | . | T | . | . | . | . | . |
| 21-225_226D11_LC | . | . | . | N | . | . | . | . | . | . | S | . | . | . | . | V | . | . | . | . | . | . | . | . | . | . |
| 21-225_226F6_LC | . | . | . | . | I | . | Y | . | . | . | . | Y | . | . | . | . | . | . | . | . | . | . | . | . | . | . |
| 21-225_227C7_LC | . | . | . | I | . | . | S | D | . | . | . | . | . | . | . | . | . | . | . | . | . | . | . | . | . | . |
| 21-225_23A12_LC | . | . | . | . | . | . | Y | . | . | . | . | . | . | . | . | . | . | . | . | . | . | . | . | . | . | . |
| 21-225_23G12_LC | . | . | . | . | I | . | F | . | . | . | . | . | . | . | . | . | . | . | . | . | . | . | . | . | . | . |
| 21-225_24E1_LC | . | . | . | . | . | . | Y | T | . | . | . | . | . | . | . | T | . | . | . | . | . | . | . | . | N | . |
| 21-225_25A4_LC | . | . | . | . | . | . | Y | . | . | N | . | . | . | . | . | . | . | . | . | . | . | . | . | . | . | . |
| 21-225_25A4.001_LC | . | . | . | . | . | . | Y | . | . | N | . | . | . | . | . | . | . | . | . | . | . | . | . | . | . | . |
| 21-225_25A4.001.001_LC | . | . | . | . | . | . | Y | . | . | N | . | . | . | . | . | . | . | . | . | . | . | . | . | . | . | . |
| 21-225_25A4.001.002_LC | . | . | . | . | . | . | Y | . | . | N | . | . | . | . | . | . | . | . | . | . | . | . | . | . | . | . |
| 21-225_25A4.001.003_LC | . | . | . | . | . | . | Y | . | . | N | . | . | . | . | . | . | . | . | . | . | . | . | . | . | . | . |
| 21-225_25A4.001.004_LC | . | . | . | . | . | . | Y | . | . | N | . | . | . | . | . | . | . | . | . | . | . | . | . | . | . | . |
| 21-225_25A4.001.005_LC | . | . | . | . | . | . | Y | . | . | N | . | . | . | . | . | . | . | . | . | . | . | . | . | . | . | . |
| 21-225_25A4.001.006_LC | . | . | . | . | . | . | Y | . | . | N | . | . | . | . | . | . | . | . | . | . | . | . | . | . | . | . |
| 21-225_25A4.001.007_LC | . | . | . | . | . | . | Y | . | . | N | . | . | . | . | . | . | . | . | . | . | . | . | . | . | . | . |
| 21-225_25A4.001.008_LC | . | . | . | . | . | . | Y | . | . | N | . | . | . | . | . | . | . | . | . | . | . | . | . | . | . | . |
| 21-225_25A4.001.013_LC | . | . | . | . | . | . | Y | . | . | N | . | . | . | . | . | . | . | . | . | . | . | . | . | . | . | . |
| 21-225_25A4.001.019_LC | . | . | . | . | . | . | Y | . | . | N | . | . | . | . | . | . | . | . | . | . | . | . | . | . | . | . |
| 21-225_25A4.001.020_LC | . | . | . | . | . | . | Y | . | . | N | . | . | . | . | . | . | . | . | . | . | . | . | . | . | . | . |
| 21-225_25A4.001.021_LC | . | . | . | . | . | . | Y | . | . | N | . | . | . | . | . | . | . | . | . | . | . | . | . | . | . | . |
| 21-225_25A4.001.029_LC | . | . | . | . | . | . | Y | . | . | N | . | . | . | . | . | . | . | . | . | . | . | . | . | . | . | . |
| 21-225_26C4_LC | . | . | . | . | . | . | Y | N | . | . | . | Y | . | . | . | . | . | . | . | N | . | . | . | . | . | . |
| 21-225_29E6_LC | . | . | . | . | . | . | Y | . | . | N | . | . | Y | . | . | T | . | . | . | . | . | . | . | . | . | N |
| 21-225_29H8_LC | . | . | . | T | I | . | . | . | . | . | . | Y | . | . | . | . | . | . | . | . | . | . | . | . | . | . |

4474

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | V | L | H | S | S | N | N | N | N | Y | L | A | W | Y | Q | Q | K | P | G | Q | P | P |
| 21-225_43D8_LC | . | . | . | . | . | . | M | T | . | . | D | R | . | . | . | T | . | . | . | . | R | . | . | . | . | . |
| 21-225_4A2.001.023_LC | . | . | . | . | i | . | . | . | . | . | . | . | . | . | . | . | . | F | . | . | . | . | . | . | . | . |
| 21-225_4A2.001.028_LC | . | . | . | . | i | . | . | . | . | . | . | . | . | . | . | . | . | F | . | . | . | . | . | . | . | . |
| 21-225_4A2.001.029_LC | . | . | . | . | i | . | . | . | . | . | . | . | . | . | . | . | . | F | . | . | . | . | . | . | . | . |
| 21-225_4A2.001.031_LC | . | . | . | . | i | . | . | . | . | . | . | . | . | . | . | . | . | F | . | . | . | . | . | . | . | . |
| 21-225_56A1_LC | . | . | . | . | . | . | . | . | . | . | . | R | . | . | . | V | . | . | . | . | R | . | . | . | . | . |
| 21-225_57A11_LC | . | . | . | . | . | . | . | . | . | . | . | Y | . | . | . | . | . | . | . | . | R | . | . | . | . | . |
| 21-225_58B9_LC | . | . | . | i | . | . | Y | . | . | . | . | . | . | . | . | . | . | . | . | . | . | . | . | . | . | . |
| 21-225_59F10_LC | . | . | T | . | . | . | Y | R | . | . | . | Y | . | . | . | . | . | . | . | . | . | . | . | . | . | . |
| 21-225_61B5_LC | . | . | . | . | . | . | Y | . | . | . | . | . | . | S | . | T | . | . | . | L | . | . | . | . | . | . |
| 21-225_70E7_LC | . | . | . | . | . | . | Y | . | . | . | . | . | . | . | . | . | . | . | . | . | . | . | . | . | . | . |
| 21-225_74A6_LC | . | . | . | . | . | . | . | . | . | . | . | . | . | . | . | . | . | . | . | . | . | . | . | . | . | . |
| 21-225_74A7_LC | . | . | . | . | . | . | F | . | . | . | . | Y | . | . | . | . | . | . | . | . | R | . | . | . | . | . |
| 21-225_74B1_LC | . | . | . | . | . | . | . | R | . | . | . | Y | . | . | . | . | . | . | . | . | . | . | . | . | . | . |
| 21-225_74B10_LC | . | . | . | . | . | . | . | . | . | . | . | R | . | . | . | T | . | . | . | . | . | . | . | . | . | . |
| 21-225_74B5_LC | . | . | . | . | . | . | F | . | . | . | . | Y | . | . | . | . | . | . | . | . | R | . | . | . | . | . |
| 21-225_74B7_LC | . | . | T | . | . | . | . | . | . | . | . | Y | . | . | . | . | . | . | . | . | . | . | . | . | . | . |
| 21-225_74B8_LC | . | . | . | . | . | . | . | . | . | . | K | K | . | . | . | . | . | . | . | . | . | . | . | . | . | . |
| 21-225_74C1_LC | . | . | . | . | i | . | . | . | . | . | . | Y | . | . | . | . | . | . | . | . | . | . | . | . | N | R |
| 21-225_74F6_LC | . | . | N | i | . | S | . | . | . | . | . | R | . | . | . | T | . | . | . | . | . | . | . | . | . | . |
| 21-225_74F9_LC | . | . | . | . | . | . | Y | . | . | . | . | Y | . | . | . | . | . | . | . | . | . | . | . | . | . | . |
| 21-225_74G4_LC | . | . | . | . | . | . | Y | . | . | . | . | . | . | . | . | . | . | . | . | . | . | A | . | . | . | . |
| 21-225_74H2_LC | . | . | . | . | . | . | . | . | . | . | . | K | . | . | T | . | . | . | . | . | . | . | . | . | . | N |
| 21-225_75A10_LC | . | . | . | . | . | . | N | . | . | . | . | Y | . | . | T | . | . | . | . | . | . | . | . | . | . | . |
| 21-225_75A7_LC | . | . | T | . | . | . | . | . | . | . | . | Y | . | . | . | . | . | . | . | . | . | . | . | . | . | . |
| 21-225_75C4_LC | . | . | . | i | . | . | Y | . | . | . | . | . | . | . | . | . | . | F | . | . | . | . | . | . | . | . |
| 21-225_75F9_LC | . | . | . | . | . | . | . | N | . | . | . | . | . | . | . | . | . | . | . | . | . | . | . | . | . | . |
| 21-225_76E11_LC | . | . | . | . | . | . | . | . | . | . | . | Y | . | . | . | . | . | . | . | . | . | A | . | . | . | . |
| 21-225_76E8_LC | . | . | T | . | . | . | . | . | . | . | . | Y | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | V | L | H | S | S | N | N | N | N | Y | L | A | W | Y | Q | Q | K | P | G | Q | P | P |
| 21-225_76F3_LC | . | . | . | . | . | . | F | . | . | . | . | Y | . | . | . | . | . | . | . | . | R | . | . | . | . | R |
| 21-225_76F5_LC | . | . | . | . | . | . | . | . | . | . | S | Y | . | . | . | . | . | . | . | . | . | . | . | . | S | . |
| 21-225_76G2_LC | . | . | . | . | . | . | . | . | . | . | . | Y | . | . | . | . | . | . | . | . | R | . | . | . | . | . |
| 21-225_77C10_LC | . | . | . | . | . | . | Y | T | . | . | . | . | . | . | . | . | . | . | . | . | . | . | . | . | . | . |
| 21-225_77C7_LC | . | . | T | . | . | . | . | . | . | . | . | Y | . | . | . | . | . | . | . | . | . | . | . | . | . | . |
| 21-225_77E1_LC | . | . | T | . | . | . | . | . | . | . | . | Y | . | . | . | . | . | . | . | . | . | . | . | . | . | . |
| 21-225_78C1_LC | . | . | . | . | . | . | Y | T | . | . | . | . | . | . | . | . | . | . | . | . | . | . | . | . | . | . |
| 21-225_78E11_LC | . | . | . | . | . | . | Y | . | F | . | . | Y | . | . | . | . | . | . | . | . | . | . | . | . | . | . |
| 21-225_78H12_LC | . | . | T | . | . | . | . | . | F | . | . | Y | . | . | . | . | . | . | . | . | . | . | . | . | . | . |
| 21-225_79E9_LC | . | . | . | I | . | . | Y | . | . | . | . | . | . | . | . | . | . | F | . | . | . | . | . | . | . | . |
| 21-225_79F12_LC | . | . | . | I | . | . | Y | . | . | . | . | Y | . | . | . | . | . | . | . | . | . | . | . | . | . | . |
| 21-225_79G10_LC | . | . | . | . | . | . | F | . | . | . | . | Y | . | . | . | . | . | . | . | . | R | . | . | . | . | N |
| 21-225_79G6_LC | . | . | . | . | . | . | . | F | . | . | . | K | . | . | T | . | . | . | . | . | . | . | . | . | . | . |
| 21-225_80D3_LC | . | . | . | . | . | . | Y | T | . | . | . | . | . | . | . | . | . | . | . | . | . | . | . | . | . | . |
| 21-225_80E9_LC | . | . | . | . | . | . | Y | T | . | . | . | . | . | . | . | . | . | . | . | . | . | . | . | . | . | . |
| 21-225_80H3_LC | . | . | . | . | . | . | . | R | . | . | . | Y | . | . | . | . | . | . | . | . | . | . | . | . | . | . |
| 21-225_81E5_LC | . | . | . | . | . | . | F | . | . | . | . | Y | . | . | . | . | . | . | . | . | R | . | . | . | . | . |
| 21-225_81F9_LC | . | . | . | . | . | . | Y | T | . | . | . | . | . | . | . | . | . | . | . | . | . | . | . | . | . | . |
| 21-225_83F3_LC | . | . | . | . | . | . | Y | T | . | . | . | . | . | . | . | . | . | . | . | . | . | . | . | . | . | . |
| 21-225_83G3_LC | . | . | . | . | . | . | Y | T | . | . | . | . | . | . | . | . | . | . | . | . | . | . | . | . | . | . |
| 21-225_83G7_LC | . | . | T | . | . | . | . | . | . | . | . | Y | . | . | . | . | . | . | . | . | . | . | . | . | . | . |
| 21-225_84G7_LC | . | . | T | . | . | . | . | . | . | . | . | Y | . | . | . | . | . | . | . | . | . | . | . | . | . | . |
| 21-225_85H2_LC | . | . | . | . | . | . | . | . | . | R | R | . | . | . | . | . | . | . | . | . | . | . | . | . | . | . |
| 21-225_85H9_LC | . | . | . | . | . | . | . | R | . | . | . | Y | . | . | . | . | . | . | . | . | . | . | . | . | . | . |
| 21-225_86E9_LC | . | . | . | . | . | . | . | R | . | . | . | Y | . | . | . | . | . | . | . | . | . | . | . | . | . | . |
| 21-225_88A1_LC | . | . | . | . | . | . | . | . | . | . | . | Y | . | . | . | T | . | . | . | . | . | . | . | . | . | . |
| 21-225_88B4_LC | . | . | . | . | . | . | Y | I | . | . | . | . | . | . | . | . | . | . | . | . | . | . | . | . | . | . |
| 21-225_88C10_LC | . | . | . | . | . | . | . | . | . | . | . | W | . | . | . | . | . | R | . | . | . | . | . | . | . | . |
| 21-225_88G2_LC | . | . | . | . | . | . | Y | T | . | . | . | . | . | . | . | . | . | . | . | . | . | . | . | . | . | . |

## FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | V | L | H | S | S | N | N | N | N | Y | L | A | W | Y | Q | Q | K | P | G | Q | P | P |
| 21-225_90D9_LC | . | . | . | Y | . | . | . | . | . | . | . | Y | . | . | . | . | . | . | . | . | . | . | . | . | . | . |
| 21-225_91F1_LC | . | . | . | . | . | . | Y | T | . | . | . | . | . | . | . | . | . | . | . | . | . | . | . | . | . | . |
| 21-225_91G8_LC | . | . | . | . | . | . | Y | T | . | . | . | . | . | . | . | . | . | . | . | . | . | . | . | . | . | . |
| 21-225_92E6_LC | . | . | . | N | i | . | S | . | . | . | . | R | . | . | . | T | . | . | . | . | . | . | . | . | . | . |
| 21-225_92F12_LC | . | . | . | . | . | . | . | R | . | . | . | Y | . | . | . | . | . | . | . | . | . | . | . | . | . | . |
| 21-225_94D2_LC | . | . | . | . | . | . | . | . | . | . | . | R | . | . | . | T | . | . | . | . | . | . | . | . | . | R |
| 21-225_94E11_LC | . | . | . | . | . | . | F | . | . | . | . | Y | . | . | . | . | . | . | . | . | R | . | . | . | . | . |
| 21-225_94E12_LC | . | . | . | . | . | . | . | R | . | . | . | Y | . | . | . | . | . | . | . | . | . | . | . | . | . | . |
| 21-225_95E12_LC | . | . | . | . | . | . | Y | . | . | . | . | A | . | . | . | . | . | . | . | . | . | . | . | . | . | . |
| 21-225_95G4_LC | . | . | . | . | . | . | F | R | . | . | . | Y | . | . | . | . | . | . | . | . | R | . | . | . | . | R |
| 21-225_95H4_LC | . | . | . | . | . | . | F | . | . | . | . | Y | . | . | . | . | . | . | . | . | R | . | . | . | . | R |
| 21-225_96D10_LC | . | . | . | N | i | . | S | . | . | . | . | R | . | . | . | T | . | . | . | . | . | . | . | . | . | . |
| 21-225_96D5_LC | . | . | . | . | . | . | . | . | . | . | . | Y | . | . | . | . | . | . | . | . | . | . | . | . | . | . |
| 21-225_96E2_LC | . | . | . | . | . | . | . | . | . | . | R | R | . | . | . | . | . | . | . | . | . | . | . | . | . | . |
| 21-225_96H5_LC | . | . | . | . | . | . | Y | . | . | . | S | R | . | . | . | . | . | . | . | . | . | . | . | . | . | . |
| 21-225_97H4_LC | . | . | . | . | . | . | Y | T | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | W | - | - | - | - | - | - | - | - | A | S | T | R | E | S | G | V | P | D | R | F |
| 21-225_11E5_LC | . | . | . | . | . | . | . | . | . | . | . | . | . | . | . | . | . | . | . | . | . | . | . | . | . | . |
| 21-225_12F12_LC | N | . | . | . | . | . | . | . | . | . | . | . | . | . | . | . | . | . | A | . | . | . | . | . | . | . |
| 21-225_147E4_LC | . | . | . | . | . | . | . | . | . | . | . | . | . | . | . | . | . | . | . | . | . | . | . | . | . | . |
| 21-225_148F8_LC | . | . | . | . | . | . | . | . | . | . | . | . | . | . | . | . | . | . | R | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | L | R | . | . | . | . | . | . | . |
| 21-225_149H4_LC | . | . | . | . | . | . | . | . | . | . | . | . | . | . | S | . | . | . | . | . | . | . | . | . | . | . |
| 21-225_154A11_LC | . | . | . | . | . | . | . | . | . | . | . | . | . | . | . | . | K | . | D | . | . | . | . | . | . | . |
| 21-225_158B12_LC | . | . | . | . | . | . | . | . | . | . | . | . | . | . | . | . | . | . | . | . | . | . | . | . | . | . |
| 21-225_162F6_LC | . | . | . | . | . | . | . | . | . | . | . | . | . | . | . | . | . | . | . | . | . | . | . | . | . | . |
| 21-225_164A4_LC | . | . | F | . | . | . | . | . | . | . | . | . | . | . | . | . | . | . | . | . | . | . | . | . | . | . |
| 21-225_164A7_LC | . | . | . | . | . | . | . | . | . | . | . | . | . | . | T | . | . | . | . | . | . | . | . | . | . | . |
| 21-225_170F6_LC | . | V | . | . | R | . | . | . | . | . | . | . | . | . | . | . | . | . | R | . | . | . | . | . | . | . |
| 21-225_174G5_LC | . | . | . | . | . | . | . | . | . | . | . | . | . | . | . | . | . | . | . | . | . | . | . | . | . | . |
| 21-225_175D10_LC | N | . | . | . | . | . | . | . | . | . | . | . | . | . | T | . | . | . | . | . | . | . | . | . | . | . |
| 21-225_177B12_LC | . | . | . | . | . | . | . | . | . | . | . | . | . | . | . | . | . | . | . | . | . | . | . | . | . | . |
| 21-225_181E5_LC | . | . | . | . | . | . | . | . | . | . | . | . | . | . | . | . | . | . | . | . | . | . | . | . | . | . |
| 21-225_190D7_LC | . | . | . | . | . | . | . | . | . | . | . | . | . | . | . | . | . | . | R | . | . | . | . | . | . | . |
| 21-225_191D8_LC | . | . | . | . | . | . | . | . | . | . | . | . | . | . | . | . | . | . | . | . | . | . | . | . | . | . |
| 21-225_193G4_LC | . | . | . | . | . | . | . | . | . | . | . | . | . | . | . | . | . | . | K | . | . | . | . | . | . | . |
| 21-225_196G8_LC | . | . | . | . | . | . | . | . | . | . | . | . | . | . | . | . | . | . | . | . | . | . | . | . | . | . |
| 21-225_197C6_LC | N | . | . | . | . | . | . | . | . | . | . | . | . | . | . | . | . | . | . | . | . | . | . | . | . | . |
| 21-225_197H4_LC | N | . | . | . | . | . | . | . | . | . | . | . | . | . | . | . | . | . | . | . | . | . | . | . | . | . |
| 21-225_200G7_LC | . | . | . | . | . | . | . | . | . | . | . | . | . | . | . | . | . | . | . | . | . | . | . | . | . | . |
| 21-225_201F5_LC | . | . | . | . | . | . | . | . | . | . | . | . | . | . | . | . | . | . | . | . | . | . | . | . | . | . |
| 21-225_213H12_LC | . | V | . | . | . | . | . | . | . | . | . | . | . | . | . | . | . | . | . | . | . | . | . | . | . | . |
| 21-225_213H7_LC | . | . | . | . | N | . | . | . | . | . | . | . | . | . | . | . | . | . | . | . | . | . | . | . | . | . |
| 21-225_222H3_LC | Q | . | . | . | . | . | . | . | . | . | . | . | . | . | . | . | . | . | . | T | . | . | . | . | . | . |
| 21-225_223G10_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | W | - | - | - | - | - | - | - | - | A | S | T | R | E | S | G | V | P | D | R | F |
| 21-225_224D8_LC | . | . | . | . | . | . | . | . | . | . | . | . | . | . | S | . | . | . | X | . | . | . | . | . | . | . |
| 21-225_224H5_LC | . | . | . | . | . | . | . | . | . | . | . | . | . | . | . | . | . | . | . | . | . | . | . | . | . | . |
| 21-225_225F11_LC | . | . | . | . | . | . | . | . | . | . | . | . | . | . | . | . | . | . | . | . | . | . | . | . | . | . |
| 21-225_225F5_LC | N | . | . | . | . | . | . | . | . | . | . | . | . | . | . | . | . | . | . | . | . | . | . | . | . | . |
| 21-225_226A12_LC | . | . | P | . | . | . | . | . | . | . | . | . | . | . | . | . | . | . | X | . | . | . | . | . | . | . |
| 21-225_226D11_LC | . | . | . | . | . | . | . | . | . | . | . | . | . | . | . | . | . | . | . | . | . | . | . | . | . | . |
| 21-225_226F6_LC | . | . | . | . | . | . | . | . | . | . | . | . | . | . | . | . | . | . | . | . | . | . | . | . | . | . |
| 21-225_227C7_LC | N | . | . | . | . | . | . | . | . | . | . | . | . | . | . | . | . | . | . | . | . | . | . | . | . | . |
| 21-225_23A12_LC | . | . | . | . | . | . | . | . | . | . | . | . | . | . | T | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | N | . | . | . | . | . | . | . | . | . | . | . | . | . | . | . | . | . | . | . | . | . | . | . | . | . |
| 21-225_25A4_LC | . | . | . | L | . | . | . | . | . | . | . | . | . | . | . | . | . | . | . | . | . | . | . | . | . | . |
| 21-225_25A4.001_LC | . | . | . | L | . | . | . | . | . | . | . | . | . | . | . | . | . | . | . | . | . | . | . | . | . | . |
| 21-225_25A4.001.001_LC | . | . | . | L | . | . | . | . | . | . | . | . | . | . | . | . | . | . | . | . | . | . | . | . | . | . |
| 21-225_25A4.001.002_LC | . | . | . | L | . | . | . | . | . | . | . | . | . | . | . | . | . | . | . | . | . | . | . | . | . | . |
| 21-225_25A4.001.003_LC | . | . | . | L | . | . | . | . | . | . | . | . | . | . | . | . | . | . | . | . | . | . | . | . | . | . |
| 21-225_25A4.001.004_LC | . | . | . | L | . | . | . | . | . | . | . | . | . | . | . | . | . | . | . | . | . | . | . | . | . | . |
| 21-225_25A4.001.005_LC | . | . | . | L | . | . | . | . | . | . | . | . | . | . | . | . | . | . | . | . | . | . | . | . | . | . |
| 21-225_25A4.001.006_LC | . | . | . | L | . | . | . | . | . | . | . | . | . | . | . | . | . | . | . | . | . | . | . | . | . | . |
| 21-225_25A4.001.007_LC | . | . | . | L | . | . | . | . | . | . | . | . | . | . | . | . | . | . | . | . | . | . | . | . | . | . |
| 21-225_25A4.001.008_LC | . | . | . | L | . | . | . | . | . | . | . | . | . | . | . | . | . | . | . | . | . | . | . | . | . | . |
| 21-225_25A4.001.013_LC | . | . | . | L | . | . | . | . | . | . | . | . | . | . | . | . | . | . | . | . | . | . | . | . | . | . |
| 21-225_25A4.001.019_LC | . | . | . | L | . | . | . | . | . | . | . | . | . | . | . | . | . | . | . | . | . | . | . | . | . | . |
| 21-225_25A4.001.020_LC | . | . | . | L | . | . | . | . | . | . | . | . | . | . | . | . | . | . | . | . | . | . | . | . | . | . |
| 21-225_25A4.001.021_LC | . | . | . | L | . | . | . | . | . | . | . | . | . | . | . | . | . | . | . | . | . | . | . | . | . | . |
| 21-225_25A4.001.029_LC | . | . | . | L | . | . | . | . | . | . | . | . | . | . | . | . | . | . | . | . | . | . | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | L | . | . | . | . | . | . | . | . | . | . | . | . | S | . | . | . | . | . | . | . | . | . |
| 21-225_29H8_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | W | - | - | - | - | - | - | - | - | A | S | T | R | E | S | G | V | P | D | R | F |
| 21-225_43D8_LC | . | . | . | . | . | . | . | . | . | . | . | . | . | . | . | . | . | . | . | . | . | . | . | . | . | . |
| 21-225_4A2.001.023_LC | . | . | . | L | . | . | . | . | . | . | . | . | . | . | . | . | . | . | . | . | . | . | . | . | . | . |
| 21-225_4A2.001.028_LC | . | . | . | L | . | . | . | . | . | . | . | . | . | . | . | . | . | . | . | . | . | . | . | . | . | . |
| 21-225_4A2.001.029_LC | . | . | . | L | . | . | . | . | . | . | . | . | . | . | . | . | . | . | . | . | . | . | . | . | . | . |
| 21-225_4A2.001.031_LC | . | . | . | L | . | . | . | . | . | . | . | . | . | . | . | . | . | . | . | . | . | . | . | . | . | . |
| 21-225_56A1_LC | N | . | . | . | . | . | . | . | . | . | . | . | . | . | . | . | . | . | . | . | . | . | . | . | . | . |
| 21-225_57A11_LC | . | . | V | . | . | . | . | . | . | . | . | . | . | . | . | . | . | . | A | . | . | . | . | . | . | . |
| 21-225_58B9_LC | . | . | . | . | . | . | . | . | . | . | . | . | . | . | . | . | . | . | D | . | . | . | . | A | . | . |
| 21-225_59F10_LC | . | . | . | . | . | . | . | . | . | . | . | . | . | . | . | . | . | . | . | . | . | . | . | . | . | . |
| 21-225_61B5_LC | . | . | . | . | . | . | . | . | . | . | . | . | . | . | . | . | . | . | . | . | . | . | . | . | . | . |
| 21-225_70E7_LC | . | . | . | . | . | . | . | . | . | . | . | . | . | . | . | . | . | . | . | . | . | . | . | . | . | . |
| 21-225_74A6_LC | Q | . | . | . | . | . | . | . | . | . | . | . | . | . | . | . | . | . | . | . | . | . | . | . | . | . |
| 21-225_74A7_LC | N | . | . | . | . | . | . | . | . | . | . | . | . | . | . | . | . | . | . | . | . | . | . | . | . | . |
| 21-225_74B1_LC | . | . | . | . | . | . | . | . | . | . | . | . | . | . | . | . | . | . | . | . | . | . | . | . | . | . |
| 21-225_74B10_LC | . | . | . | . | . | . | . | . | . | . | . | . | . | . | . | . | . | . | . | . | . | . | . | . | . | . |
| 21-225_74B5_LC | N | . | . | . | . | . | . | . | . | . | . | . | . | . | . | . | . | . | . | . | . | . | . | . | . | . |
| 21-225_74B7_LC | . | V | . | . | . | . | . | . | . | . | . | . | . | . | T | . | . | . | . | . | . | . | . | . | . | . |
| 21-225_74B8_LC | . | . | . | . | . | . | . | . | . | . | . | . | . | . | . | . | . | . | . | . | . | . | . | . | . | . |
| 21-225_74C1_LC | . | . | . | . | . | . | . | . | . | . | . | . | . | . | . | . | . | . | . | . | . | . | . | . | . | . |
| 21-225_74F6_LC | . | I | . | . | . | . | . | . | . | . | . | . | . | . | T | . | . | . | . | . | . | . | . | . | . | . |
| 21-225_74F9_LC | . | . | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | . | . | . | . | . | . | . | . | . | . | . | . | . | . | . |
| 21-225_75A10_LC | . | . | . | L | . | . | . | . | . | . | . | . | . | . | . | . | . | . | K | . | . | . | . | . | . | . |
| 21-225_75A7_LC | . | V | . | . | . | . | . | . | . | . | . | . | . | . | T | . | . | . | . | . | . | . | . | . | . | . |
| 21-225_75C4_LC | . | . | . | . | . | . | . | . | . | . | . | . | . | . | . | . | . | . | . | . | . | . | . | . | . | . |
| 21-225_75F9_LC | . | . | . | . | . | . | . | . | . | . | . | . | . | . | . | . | . | . | . | . | . | . | . | . | . | . |
| 21-225_76E11_LC | . | . | . | . | . | . | . | . | . | . | . | . | . | . | . | . | . | . | . | . | . | . | . | . | . | . |
| 21-225_76E8_LC | . | V | . | . | . | . | . | . | . | . | . | . | . | . | T | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | W | - | - | - | - | - | - | - | - | A | S | T | R | E | S | G | V | P | D | R | F |
| 21-225_76F3_LC | S | . | . | . | . | . | . | . | . | . | . | . | . | . | . | . | . | . | . | . | . | . | . | . | . | . |
| 21-225_76F5_LC | . | V | . | . | . | . | . | . | . | . | . | . | . | . | T | . | . | . | . | . | . | . | . | . | . | . |
| 21-225_76G2_LC | R | . | . | . | . | . | . | . | . | . | . | . | . | . | . | . | . | . | . | . | . | . | . | . | . | . |
| 21-225_77C10_LC | . | . | . | . | . | . | . | . | . | . | . | . | . | . | . | . | . | . | . | . | . | . | . | . | . | . |
| 21-225_77C7_LC | . | V | . | L | . | . | . | . | . | . | . | . | . | . | T | . | . | . | . | . | . | . | . | . | . | . |
| 21-225_77E1_LC | . | V | . | . | . | . | . | . | . | . | . | . | . | . | T | . | . | . | . | . | . | . | M | . | . | . |
| 21-225_78C1_LC | . | . | . | . | . | . | . | . | . | . | . | . | . | . | . | . | . | . | . | . | . | . | . | . | . | . |
| 21-225_78E11_LC | . | C | . | . | . | . | . | . | . | . | . | . | . | . | . | . | . | . | . | . | . | . | . | . | . | . |
| 21-225_78H12_LC | . | V | . | . | . | . | . | . | . | . | . | . | . | . | T | . | . | . | . | . | . | . | . | . | . | . |
| 21-225_79E9_LC | . | . | . | . | . | . | . | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | N | . | . | . | . | . | . | . | . | . | . | . | . | . | . | . | . | . | . | . | . | . | . | . | . | . |
| 21-225_79G6_LC | . | . | . | . | . | . | . | . | . | . | . | . | . | . | . | . | . | W | . | . | . | . | . | . | . | . |
| 21-225_80D3_LC | . | . | . | . | . | . | . | . | . | . | . | . | . | . | . | . | . | . | . | . | . | . | . | . | . | . |
| 21-225_80E9_LC | . | . | . | . | . | . | . | . | . | . | . | . | . | . | . | . | . | . | . | . | . | . | . | . | . | . |
| 21-225_80H3_LC | . | . | . | . | . | . | . | . | . | . | . | . | . | . | . | . | . | . | . | . | . | . | . | . | . | . |
| 21-225_81E5_LC | N | . | . | . | . | . | . | . | . | . | . | . | . | . | . | . | . | . | . | . | . | . | . | . | . | . |
| 21-225_81F9_LC | . | . | . | . | . | . | . | . | . | . | . | . | . | . | . | . | . | . | . | . | . | . | . | . | . | . |
| 21-225_83F3_LC | . | . | . | . | . | . | . | . | . | . | . | . | . | . | . | . | . | . | . | . | . | . | . | . | . | . |
| 21-225_83G3_LC | . | . | . | . | . | . | . | . | . | . | . | . | . | . | . | . | . | . | . | . | . | . | . | . | . | . |
| 21-225_83G7_LC | . | V | . | . | . | . | . | . | . | . | . | . | . | . | T | . | . | . | . | . | . | . | . | . | . | . |
| 21-225_84G7_LC | . | V | . | . | . | . | . | . | . | . | . | . | . | . | T | . | . | . | . | . | . | . | . | . | . | . |
| 21-225_85H2_LC | . | . | . | . | . | . | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | . | . | . | . | . | . |
| 21-225_86E9_LC | N | . | . | . | . | . | . | . | . | . | . | . | . | . | . | . | . | . | . | . | . | . | . | . | . | . |
| 21-225_88A1_LC | . | . | . | L | . | . | . | . | . | . | . | . | . | . | . | . | . | . | . | K | . | . | . | . | . | . |
| 21-225_88B4_LC | . | . | . | . | . | . | . | . | . | . | . | . | . | . | . | . | . | . | . | . | . | . | . | A | . | . |
| 21-225_88C10_LC | . | . | . | . | M | . | . | . | . | . | . | . | . | . | . | R | . | . | . | K | . | . | . | . | . | . |
| 21-225_88G2_LC | . | . | . | . | . | . | . | . | . | . | . | . | . | . | . | . | . | . | . | . | . | . | . | . | . | . |

4481

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | W | - | - | - | - | - | - | - | - | A | S | T | R | E | S | G | V | P | D | R | F |
| 21-225_90D9_LC | . | V | . | . | . | . | . | . | . | . | . | . | . | . | T | . | . | . | . | . | . | . | . | . | . | . |
| 21-225_91F1_LC | . | . | . | . | . | . | . | . | . | . | . | . | . | . | . | . | . | . | . | . | . | . | . | . | . | . |
| 21-225_91G8_LC | . | . | . | . | . | . | . | . | . | . | . | . | . | . | . | . | . | . | . | . | . | . | . | . | . | . |
| 21-225_92E6_LC | . | ; | . | . | . | . | . | . | . | . | . | . | . | . | T | . | . | . | . | . | . | . | . | . | . | . |
| 21-225_92F12_LC | . | . | . | . | . | . | . | . | . | . | . | . | . | . | . | . | . | . | . | . | . | . | . | . | . | . |
| 21-225_94D2_LC | . | . | . | . | . | . | . | . | . | . | . | . | . | . | . | . | . | . | . | . | . | . | . | . | . | . |
| 21-225_94E11_LC | N | . | . | . | . | . | . | . | . | . | . | . | . | . | . | . | . | . | . | . | . | . | . | . | . | . |
| 21-225_94E12_LC | . | . | . | . | . | . | . | . | . | . | . | . | . | . | . | . | . | . | . | . | . | . | . | . | . | . |
| 21-225_95E12_LC | N | . | F | . | . | . | . | . | . | . | . | . | . | . | . | . | . | . | . | . | . | . | . | . | . | . |
| 21-225_95G4_LC | N | . | . | . | . | . | . | . | . | . | . | . | . | . | . | . | . | . | . | . | . | . | . | . | . | . |
| 21-225_95H4_LC | N | . | . | . | . | . | . | . | . | . | . | . | . | . | . | . | . | . | . | . | . | . | . | . | . | . |
| 21-225_96D10_LC | . | ; | . | . | . | . | . | . | . | . | . | . | . | . | T | . | . | . | . | . | . | . | . | . | . | . |
| 21-225_96D5_LC | N | . | . | . | . | . | . | . | . | . | . | . | . | . | . | . | . | . | . | . | . | . | . | . | . | . |
| 21-225_96E2_LC | . | . | . | . | . | . | . | . | . | . | . | . | . | . | . | . | . | W | . | . | . | . | . | . | . | . |
| 21-225_96H5_LC | . | . | . | . | . | . | . | . | . | . | . | . | . | . | . | . | . | ; | . | . | . | . | . | . | . | . |
| 21-225_97H4_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_11E5_LC | . | . | . | . | . | . | . | . | . | N | . | . | . | . | . | . | . | . | . | . | . | . | . | . | . | . |
| 21-225_12F12_LC | . | . | . | . | . | . | . | . | . | . | . | . | . | . | . | . | . | . | . | . | . | . | . | . | . | . |
| 21-225_147E4_LC | . | A | . | . | . | . | . | . | . | . | . | . | . | . | . | . | . | . | . | . | . | . | . | . | ↑ | . |
| 21-225_148F8_LC | . | . | . | . | . | . | . | . | . | . | . | . | . | . | . | . | . | . | . | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | . | . | . | . | . | . | . | . | . |
| 21-225_149H4_LC | . | . | . | . | . | . | . | . | . | . | . | . | . | . | . | . | . | . | . | . | . | . | . | . | . | . |
| 21-225_154A11_LC | . | . | . | . | . | . | . | . | . | . | . | . | . | . | . | . | . | . | . | . | . | . | . | . | . | . |
| 21-225_158B12_LC | . | . | . | . | . | . | . | . | . | S | . | . | . | . | . | . | . | . | . | . | . | . | . | . | . | . |
| 21-225_162F6_LC | . | . | . | . | . | . | . | . | . | S | . | . | . | . | . | . | . | . | . | . | . | . | . | . | . | . |
| 21-225_164A4_LC | . | . | . | O | . | . | . | . | . | . | . | . | . | . | . | . | . | V | . | . | . | . | . | . | . | . |
| 21-225_164A7_LC | . | . | . | Y | . | . | . | . | . | . | . | S | . | . | . | A | . | . | . | . | . | . | . | . | . | . |
| 21-225_170F6_LC | . | . | V | . | . | . | . | . | . | . | . | . | . | . | . | N | . | . | . | . | . | . | . | . | . | . |
| 21-225_174G5_LC | . | . | . | . | . | . | . | . | . | . | . | . | . | . | . | . | . | . | . | . | . | . | . | . | . | . |
| 21-225_175D10_LC | . | . | . | . | . | . | . | . | . | N | . | . | . | . | . | . | . | . | . | . | . | . | . | . | . | . |
| 21-225_177B12_LC | . | . | . | . | . | . | . | . | . | . | . | . | . | . | . | . | . | . | . | . | . | . | . | . | . | . |
| 21-225_181E5_LC | . | . | . | . | . | . | . | . | . | . | . | . | . | . | . | . | . | . | . | . | . | . | . | . | . | . |
| 21-225_190D7_LC | . | . | . | . | . | . | . | . | . | . | . | . | . | . | . | . | . | . | . | . | O | . | . | . | . | . |
| 21-225_191D8_LC | . | . | . | . | . | . | . | . | . | . | . | . | . | . | . | . | . | . | . | . | . | . | . | . | . | . |
| 21-225_193G4_LC | . | . | . | . | . | . | . | . | . | . | . | . | . | . | . | . | . | . | . | . | D | . | . | . | . | . |
| 21-225_196G8_LC | . | . | . | . | . | . | . | . | . | . | . | . | . | A | . | . | . | . | . | . | . | . | . | . | . | . |
| 21-225_197C6_LC | . | . | . | . | . | . | . | . | . | . | . | . | . | . | . | . | . | . | . | . | . | . | . | . | . | . |
| 21-225_197H4_LC | . | . | . | . | . | . | . | . | . | . | . | . | . | P | . | . | . | . | . | . | . | . | . | . | . | . |
| 21-225_200G7_LC | . | . | . | . | . | . | . | . | . | . | . | . | . | A | . | . | . | . | . | . | . | . | . | . | . | . |
| 21-225_201F5_LC | . | . | . | . | . | . | . | . | . | . | . | . | . | . | . | . | . | . | . | . | . | . | . | . | . | . |
| 21-225_213H12_LC | . | . | . | . | . | . | . | . | . | . | . | . | . | I | . | . | . | . | . | . | . | . | . | . | . | . |
| 21-225_213H7_LC | . | . | . | . | . | . | . | . | . | . | . | . | . | . | . | . | . | . | . | T | . | . | . | . | . | . |
| 21-225_222H3_LC | . | . | . | . | . | . | . | . | . | . | . | . | . | . | . | . | . | . | . | . | . | . | . | S | . | . |
| 21-225_223G10_LC | . | . | . | . | . | . | . | . | . | . | . | . | . | . | . | . | . | . | . | . | . | . | . | . | . | . |

EP 4 435 105 A2

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_224D8_LC | . | . | . | . | . | . | . | . | . | . | . | . | . | . | . | . | . | . | . | . | . | . | . | . | . | . |
| 21-225_224H5_LC | . | . | . | . | . | . | . | . | . | . | . | . | . | . | . | N | . | . | . | . | . | . | . | . | . | . |
| 21-225_225F11_LC | . | . | . | . | . | . | . | . | S | . | . | . | . | . | . | . | . | . | . | . | . | . | . | . | . | . |
| 21-225_225F5_LC | . | . | . | . | . | . | . | . | . | . | . | . | . | . | . | . | . | . | . | . | . | . | . | . | . | . |
| 21-225_226A12_LC | . | . | . | . | . | . | . | . | . | . | . | . | . | . | . | . | . | . | . | . | . | . | . | . | . | . |
| 21-225_226D11_LC | . | . | . | . | . | . | . | . | . | . | . | . | . | . | . | . | . | . | . | . | . | . | . | . | . | . |
| 21-225_226F6_LC | . | . | . | . | . | . | . | . | S | . | . | . | . | . | . | . | . | . | . | . | . | . | . | . | . | . |
| 21-225_227C7_LC | . | . | . | . | . | . | . | . | . | . | . | . | . | . | . | . | . | . | . | . | . | . | . | . | . | . |
| 21-225_23A12_LC | . | . | . | . | . | . | . | . | . | . | . | . | . | . | . | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | . | . | . | . | . | . | . | . | . | . | . | . | . | . | . | . | . | . | . | . | . | . | . | . | . | . |
| 21-225_25A4_LC | . | . | . | . | . | . | . | . | . | N | . | . | . | . | . | . | . | N | . | . | . | . | . | . | . | . |
| 21-225_25A4.001_LC | . | . | . | . | . | . | . | . | . | N | . | . | . | . | . | . | . | N | . | . | . | . | . | . | . | . |
| 21-225_25A4.001.001_LC | . | . | . | . | . | . | . | . | . | N | . | . | . | . | . | . | . | N | . | . | . | . | . | . | . | . |
| 21-225_25A4.001.002_LC | . | . | . | . | . | . | . | . | . | N | . | . | . | . | . | . | . | N | . | . | . | . | . | . | . | . |
| 21-225_25A4.001.003_LC | . | . | . | . | . | . | . | . | . | N | . | . | . | . | . | . | . | N | . | . | . | . | . | . | . | . |
| 21-225_25A4.001.004_LC | . | . | . | . | . | . | . | . | . | N | . | . | . | . | . | . | . | N | . | . | . | . | . | . | . | . |
| 21-225_25A4.001.005_LC | . | . | . | . | . | . | . | . | . | N | . | . | . | . | . | . | . | . | . | . | . | . | . | . | . | . |
| 21-225_25A4.001.006_LC | . | . | . | . | . | . | . | . | . | N | . | . | . | . | . | . | . | . | . | . | . | . | . | . | . | . |
| 21-225_25A4.001.007_LC | . | . | . | . | . | . | . | . | . | N | . | . | . | . | . | . | . | . | . | . | . | . | . | . | . | . |
| 21-225_25A4.001.008_LC | . | . | . | . | . | . | . | . | . | N | . | . | . | . | . | . | . | . | . | . | . | . | . | . | . | . |
| 21-225_25A4.001.013_LC | . | . | . | . | . | . | . | . | . | N | . | . | . | . | . | . | . | . | . | . | . | . | . | . | . | . |
| 21-225_25A4.001.019_LC | . | . | . | . | . | . | . | . | . | N | . | . | . | . | . | . | . | N | . | . | . | . | . | . | . | . |
| 21-225_25A4.001.020_LC | . | . | . | . | . | . | . | . | . | N | . | . | . | . | . | . | . | . | . | . | . | . | . | . | . | . |
| 21-225_25A4.001.021_LC | . | . | . | . | . | . | . | . | . | N | . | . | . | . | . | . | . | . | . | . | . | . | . | . | . | . |
| 21-225_25A4.001.029_LC | . | . | . | . | . | . | . | . | . | N | . | . | . | . | . | . | . | . | . | . | . | . | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | . | . | . | . | . | . | N | . | . | . | . | . | . | . | . | . | . | . | . | . | . | . | . |
| 21-225_29H8_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_43D8_LC | . | . | S | . | . | . | . | . | . | . | . | . | . | . | . | . | . | G | . | . | . | . | . | . | . | . |
| 21-225_4A2.001.023_LC | . | . | . | . | . | . | . | . | . | . | . | . | . | . | . | . | . | . | . | P | . | . | . | . | . | . |
| 21-225_4A2.001.028_LC | . | . | . | . | . | . | . | . | . | . | . | . | . | . | . | . | . | . | . | P | . | . | . | . | . | . |
| 21-225_4A2.001.029_LC | . | . | . | . | . | . | . | . | . | . | . | . | . | . | . | . | . | . | . | P | . | . | . | . | . | . |
| 21-225_4A2.001.031_LC | . | . | . | . | . | . | . | . | . | . | . | . | . | . | . | . | . | . | . | P | . | . | . | . | . | . |
| 21-225_56A1_LC | . | . | . | . | . | . | . | . | . | . | . | . | . | . | . | . | . | . | . | . | . | . | . | . | . | . |
| 21-225_57A11_LC | . | . | . | . | . | . | . | . | . | . | . | . | . | . | . | . | . | . | . | . | . | . | M | . | . | . |
| 21-225_58B9_LC | . | . | . | . | . | . | . | . | . | . | . | . | . | . | . | . | . | . | . | . | . | . | . | . | . | . |
| 21-225_59F10_LC | . | . | . | . | . | . | . | . | . | . | . | . | . | . | . | . | . | . | . | . | . | . | . | . | . | . |
| 21-225_61B5_LC | . | . | . | . | . | . | . | . | . | . | . | . | . | . | . | . | . | . | . | . | . | . | . | . | . | . |
| 21-225_70E7_LC | . | . | . | . | . | . | . | . | . | . | . | . | . | . | . | . | . | . | . | . | . | . | . | . | . | . |
| 21-225_74A6_LC | . | . | . | . | . | . | . | . | . | . | . | . | . | . | . | . | . | . | . | . | . | . | . | . | . | . |
| 21-225_74A7_LC | . | . | . | . | . | . | . | . | . | . | . | . | . | . | . | . | . | . | . | . | A | . | . | . | . | . |
| 21-225_74B1_LC | . | . | . | . | . | . | . | . | . | . | . | . | . | . | . | . | . | . | . | . | . | . | . | . | . | . |
| 21-225_74B10_LC | . | . | . | . | . | . | . | . | . | . | S | . | . | . | . | G | . | . | . | . | . | . | . | . | . | . |
| 21-225_74B5_LC | . | . | . | . | . | . | . | . | . | . | . | . | . | . | . | . | . | . | . | . | A | . | . | . | . | . |
| 21-225_74B7_LC | . | . | . | . | . | . | . | . | . | . | . | . | . | . | . | . | . | . | . | . | . | . | . | . | . | . |
| 21-225_74B8_LC | . | . | . | . | . | . | . | . | . | . | . | . | . | . | . | . | . | . | . | . | . | . | . | . | . | . |
| 21-225_74C1_LC | . | . | . | Y | . | . | . | . | . | . | . | . | . | . | . | . | . | . | . | . | . | . | . | . | . | . |
| 21-225_74F6_LC | . | . | . | P | . | . | . | . | . | . | . | . | . | . | . | . | . | . | . | . | . | . | . | . | . | . |
| 21-225_74F9_LC | . | . | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | S | . | . | . | . | G | . | . | . | . | . | . | . | . | . | . |
| 21-225_75A10_LC | . | . | . | . | . | . | . | . | . | . | . | . | . | . | . | . | . | . | . | . | . | . | . | . | . | . |
| 21-225_75A7_LC | . | . | . | . | . | . | . | . | . | . | . | . | . | . | . | . | . | . | . | . | . | . | . | . | . | . |
| 21-225_75C4_LC | . | . | . | . | . | . | . | . | . | . | . | . | . | . | . | . | . | . | . | . | . | . | . | . | . | . |
| 21-225_75F9_LC | . | . | . | . | . | . | . | . | . | . | . | . | . | . | . | . | . | . | . | . | . | . | . | . | . | . |
| 21-225_76E11_LC | . | . | . | . | . | . | . | . | . | . | . | . | . | . | . | . | . | . | . | . | . | . | . | . | . | . |
| 21-225_76E8_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_76F3_LC | . | . | . | . | . | . | . | . | . | . | . | . | . | . | . | . | . | . | . | . | A | . | . | . | . | . |
| 21-225_76F5_LC | . | . | . | . | . | . | . | . | . | . | . | . | . | . | . | . | . | . | . | . | . | . | . | . | . | . |
| 21-225_76G2_LC | . | . | . | . | . | . | . | . | . | . | . | . | . | . | . | . | . | . | . | V | . | . | . | . | . | . |
| 21-225_77C10_LC | . | . | . | . | . | . | . | . | . | . | . | . | . | . | . | . | . | . | . | . | . | . | . | . | . | . |
| 21-225_77C7_LC | . | . | . | . | . | . | . | . | . | . | . | . | . | . | . | . | . | . | . | . | . | . | . | . | . | . |
| 21-225_77E1_LC | . | . | . | . | . | . | . | . | . | . | . | . | . | . | . | . | . | . | . | . | . | . | . | . | . | . |
| 21-225_78C1_LC | . | . | . | . | . | . | . | . | . | . | . | . | . | . | . | . | . | . | . | . | . | . | . | . | . | . |
| 21-225_78E11_LC | . | . | . | . | . | . | . | . | . | . | . | . | . | . | . | . | . | . | . | . | . | . | . | . | . | . |
| 21-225_78H12_LC | . | . | . | . | . | . | . | . | . | . | . | . | . | . | . | . | . | . | . | . | . | . | . | . | . | . |
| 21-225_79E9_LC | . | . | . | C | . | . | . | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | A | . | . | . | . | . |
| 21-225_79G10_LC | . | . | . | . | . | . | . | . | . | . | . | . | . | . | . | . | . | . | . | . | A | . | . | . | . | . |
| 21-225_79G6_LC | . | . | . | . | . | . | . | . | . | . | S | . | N | . | . | G | . | . | . | . | . | . | . | . | . | . |
| 21-225_80D3_LC | . | . | . | . | . | . | . | . | . | . | . | . | . | . | . | . | . | . | . | . | . | . | . | . | . | . |
| 21-225_80E9_LC | . | . | . | . | . | . | . | . | . | . | . | . | . | . | . | . | . | . | . | . | . | . | . | . | . | . |
| 21-225_80H3_LC | . | . | . | . | . | . | . | . | . | . | . | . | . | . | . | . | . | . | . | . | . | . | . | . | . | . |
| 21-225_81E5_LC | . | . | . | . | . | . | . | . | . | . | . | . | . | . | . | . | . | . | . | . | A | . | . | . | . | . |
| 21-225_81F9_LC | . | . | . | . | . | . | . | . | . | . | . | . | . | . | . | . | . | . | . | . | . | . | . | . | . | . |
| 21-225_83F3_LC | . | . | . | . | . | . | . | . | . | . | . | . | . | . | . | . | . | . | . | . | . | . | . | . | . | . |
| 21-225_83G3_LC | . | . | . | . | . | . | . | . | . | . | . | . | . | . | . | . | . | . | . | . | . | . | . | . | . | . |
| 21-225_83G7_LC | . | . | . | . | . | . | . | . | . | . | . | . | . | . | . | . | . | . | . | . | . | . | . | . | . | . |
| 21-225_84G7_LC | . | . | . | . | . | . | . | . | . | . | . | . | . | . | . | . | . | . | . | . | . | . | . | . | . | . |
| 21-225_85H2_LC | . | . | . | . | . | . | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | . | . | . | . | . | . |
| 21-225_86E9_LC | . | . | . | . | . | . | . | . | . | . | . | . | . | . | . | . | . | . | . | . | A | . | . | . | . | . |
| 21-225_88A1_LC | . | . | . | . | . | . | . | . | . | . | . | . | . | . | . | . | . | . | . | . | . | . | . | . | . | . |
| 21-225_88B4_LC | . | . | . | . | . | . | . | . | . | . | . | . | . | . | . | . | . | . | . | . | . | . | . | . | . | . |
| 21-225_88C10_LC | . | . | G | . | . | . | . | . | . | N | . | . | . | . | . | . | . | . | . | . | . | . | . | . | . | . |
| 21-225_88G2_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR5 | | | | | | | | | | | | | | | |
| CONSENSUS | S | G | S | G | S | G | - | - | T | D | F | T | L | T | I | S | S | L | Q | A | E | D | V | A | V | Y |
| 21-225_90D9_LC | . | . | . | . | . | . | . | . | . | . | . | . | . | . | . | . | . | . | . | . | . | . | . | . | . | . |
| 21-225_91F1_LC | . | . | . | . | . | . | . | . | . | . | . | . | . | . | . | . | . | . | . | . | . | . | . | . | . | . |
| 21-225_91G8_LC | . | . | . | . | . | . | . | . | . | . | . | . | . | . | . | . | . | . | . | . | . | . | . | . | . | . |
| 21-225_92E6_LC | . | . | . | . | . | . | . | . | . | . | . | . | . | . | . | . | . | . | . | . | . | . | . | . | . | . |
| 21-225_92F12_LC | . | . | . | . | . | . | . | . | . | . | . | . | . | . | . | . | . | . | . | . | . | . | . | . | . | . |
| 21-225_94D2_LC | . | . | . | . | . | . | . | . | . | . | S | . | . | . | . | Q | . | . | . | X | . | . | . | . | . | . |
| 21-225_94E11_LC | . | . | . | . | . | . | . | . | . | . | . | . | . | . | . | . | . | . | . | . | A | . | . | . | . | . |
| 21-225_94E12_LC | . | . | . | . | . | . | . | . | . | . | . | . | . | . | . | . | . | . | . | . | . | . | . | . | . | . |
| 21-225_95E12_LC | . | . | . | . | . | . | . | . | . | . | . | . | . | . | . | . | . | . | . | . | . | . | . | . | . | . |
| 21-225_95G4_LC | . | . | . | Y | . | . | . | . | . | . | . | . | . | . | . | . | . | V | . | . | A | . | . | . | . | . |
| 21-225_95H4_LC | . | . | . | . | . | . | . | . | . | . | . | . | . | . | . | . | . | . | . | . | A | . | . | . | . | . |
| 21-225_96D10_LC | . | . | . | . | . | . | . | . | . | . | . | . | . | . | . | . | . | . | . | . | . | . | . | . | . | . |
| 21-225_96D5_LC | . | . | . | . | . | . | . | . | . | . | . | . | . | . | . | . | . | . | . | . | A | . | . | . | . | . |
| 21-225_96E2_LC | . | . | . | . | . | . | . | . | . | . | . | . | . | . | . | . | . | . | . | . | . | . | . | . | . | . |
| 21-225_96H5_LC | . | . | . | . | . | . | . | . | . | . | . | . | S | . | . | . | . | M | . | . | . | . | . | . | . | . |
| 21-225_97H4_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_11E5_LC | | | | | | | | | | | | | | | | | | | | | | | | | | |
| 21-225_12F12_LC | | | | | | | | | | | | | | | | | | | | | | | | | | |
| 21-225_147E4_LC | | | | | | | | | | | | | | | | | | | | | | | | | | |
| 21-225_148F8_LC | | | | | | | | | | | | | | | | | | | | | | | | | | |
| 21-225_149A2_LC | | | | | | | | | | | | | | | | | | | | | | | | | | |
| 21-225_149E3_LC | | | | | | | | | | | | | | | | | | | | | | | | | | |
| 21-225_149H4_LC | | | N | | | | | | | | | | | | | | | | | | | | | | | |
| 21-225_154A11_LC | | | | | F | | | | | | | | | | | | | | | | | | | | | |
| 21-225_158B12_LC | | | | | | | | | | | | | | | | | | | | | | | | | | |
| 21-225_162F6_LC | | | | | | | | | | | | | | | | | | | | | | | | | | |
| 21-225_164A4_LC | | | | | F | | | | | | | | | | | | | | | | | | | | | |
| 21-225_164A7_LC | | | | | | | | | | | | | | | | | | | | | | | | | | |
| 21-225_170F6_LC | | | | | | | | | | | | | | | | | | | | | | | | | | |
| 21-225_174G5_LC | | | | | | | N | | | | | | | | | | | | | | | | | | | |
| 21-225_175D10_LC | | | | | | | | | | | | | | | | | | | | | | | | | | |
| 21-225_177B12_LC | | | | | | | | | | | | | | | | | | | | | | | | | | |
| 21-225_181E5_LC | | | N | | | F | I | | | | | | | | | | | | | | | | | | | |
| 21-225_190D7_LC | | | | | | N | | | | | | | | | | | | | | | | | | | | |
| 21-225_191D8_LC | | | | | | | | | | | | | | | | | | | | | | | | | | |
| 21-225_193G4_LC | | | | | | I | | | | | | | | | | | | | | | | | | | | |
| 21-225_196G8_LC | | | | | | N | | | | | | | | | | | | | | | | | | | | |
| 21-225_197C6_LC | | | | | | | | | | | | | | | | | | | | | | | | | | |
| 21-225_197H4_LC | | | | | | | | | | | | | | | | | | | | | | | | | | |
| 21-225_200G7_LC | | | | | | N | | | | | | | | | | | | | | | | | | | | |
| 21-225_201F5_LC | | | | | | | | | | | | | | | | | | | | | | | | | | |
| 21-225_213H12_LC | | | N | | | | | | | | | | | | | | | | | | | | | | | |
| 21-225_213H7_LC | K | | | | | N | | | | | | | | | | | | | | | | | | | | |
| 21-225_222H3_LC | S | | | | | S | | | | | | | | | | | | | | | | | | | | |
| 21-225_223G10_LC | | | L | | | F | | | | | | | | | | | | | | | | | | | | |

4488

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_224D8_LC | . | . | . | . | . | . | . | . | . | . | . | . | . | . | . | . | . | . | . | . | . | . | . | . | . | . |
| 21-225_224H5_LC | . | . | . | . | . | . | . | . | . | . | . | . | . | . | . | . | . | . | . | . | . | . | . | . | . | . |
| 21-225_225F11_LC | . | . | . | . | . | . | T | . | . | . | . | . | . | . | . | . | . | . | . | . | . | . | . | . | . | . |
| 21-225_225F5_LC | . | . | . | . | . | . | . | . | . | . | . | . | . | . | . | . | . | . | . | . | . | . | . | . | . | . |
| 21-225_226A12_LC | . | . | . | . | . | . | . | . | . | . | . | . | . | . | . | . | . | . | . | . | . | . | . | . | . | . |
| 21-225_226D11_LC | . | . | . | . | . | . | . | . | . | . | . | . | . | . | . | . | . | . | . | . | . | . | . | . | . | . |
| 21-225_226F6_LC | . | . | . | . | . | . | T | . | . | . | . | . | . | . | . | . | . | . | . | . | . | . | . | . | . | . |
| 21-225_227C7_LC | . | . | . | . | . | . | . | . | . | . | . | . | . | . | . | . | . | . | . | . | . | . | . | . | . | . |
| 21-225_23A12_LC | . | . | . | . | . | . | . | . | . | . | . | . | . | . | . | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | . | . | . | . | . | . | . | . | . | . | . | . | . | . | . | . | . | . | . | . | . | . | . | . | . | . |
| 21-225_25A4_LC | . | . | . | . | . | . | . | . | . | . | . | . | . | . | . | . | . | . | . | . | . | . | . | . | . | . |
| 21-225_25A4.001_LC | . | . | . | . | . | . | . | . | . | . | . | . | . | . | . | . | . | . | . | . | . | . | . | . | . | . |
| 21-225_25A4.001.001_LC | . | . | . | . | . | . | . | . | . | . | . | . | . | . | . | . | . | . | . | . | . | . | . | . | . | . |
| 21-225_25A4.001.002_LC | . | . | . | . | . | . | . | . | . | . | . | . | . | . | . | . | . | . | . | . | . | . | . | . | . | . |
| 21-225_25A4.001.003_LC | . | . | . | . | . | . | . | . | . | . | . | . | . | . | . | . | . | . | . | . | . | . | . | . | . | . |
| 21-225_25A4.001.004_LC | . | . | . | . | . | . | . | . | . | . | . | . | . | . | . | . | . | . | . | . | . | . | . | . | . | . |
| 21-225_25A4.001.005_LC | . | . | . | . | . | . | . | . | . | . | . | . | . | . | . | . | . | . | . | . | . | . | . | . | . | . |
| 21-225_25A4.001.006_LC | . | . | . | . | . | . | . | . | . | . | . | . | . | . | . | . | . | . | . | . | . | . | . | . | . | . |
| 21-225_25A4.001.007_LC | . | . | . | . | . | . | . | . | . | . | . | . | . | . | . | . | . | . | . | . | . | . | . | . | . | . |
| 21-225_25A4.001.008_LC | . | . | . | . | . | . | . | . | . | . | . | . | . | . | . | . | . | . | . | . | . | . | . | . | . | . |
| 21-225_25A4.001.013_LC | . | . | . | . | . | . | . | . | . | . | . | . | . | . | . | . | . | . | . | . | . | . | . | . | . | . |
| 21-225_25A4.001.019_LC | . | . | . | . | . | . | . | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . | . | . | . | . | . | . | . |
| 21-225_25A4.001.029_LC | . | . | . | . | . | . | . | . | . | . | . | . | . | . | . | . | . | . | . | . | . | . | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | . | . | . | . | . | . | . | . | . | . | . | . | . | . | . | . | . | . | . | . | . | . | . |
| 21-225_29H8_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_43D8_LC | . | . | . | . | . | . | . | . | . | . | . | . | . | . | . | . | . | . | . | . | . | . | . | . | . | . |
| 21-225_4A2.001.023_LC | . | . | . | . | . | . | . | . | . | . | . | . | . | . | . | . | . | . | . | . | . | . | . | . | . | . |
| 21-225_4A2.001.028_LC | . | . | . | . | . | . | . | . | . | . | . | . | . | . | . | . | . | . | . | . | . | . | . | . | . | . |
| 21-225_4A2.001.029_LC | . | . | . | . | . | . | . | . | . | . | . | . | . | . | . | . | . | . | . | . | . | . | . | . | . | . |
| 21-225_4A2.001.031_LC | . | . | . | . | . | . | N | . | . | . | . | . | . | . | . | . | . | . | . | . | . | . | . | . | . | . |
| 21-225_56A1_LC | . | . | . | . | . | . | . | . | . | . | . | . | . | . | . | . | . | . | . | . | . | . | . | . | . | . |
| 21-225_57A11_LC | . | . | . | . | . | . | . | . | . | . | . | . | . | . | . | . | . | . | . | . | . | . | . | . | . | . |
| 21-225_58B9_LC | . | . | . | . | . | . | . | . | . | . | . | . | . | . | . | . | . | . | . | . | . | . | . | . | . | . |
| 21-225_59F10_LC | . | . | . | . | . | S | . | . | . | . | . | . | . | . | . | . | . | . | . | . | . | . | . | . | . | . |
| 21-225_61B5_LC | . | . | . | . | . | . | . | . | . | . | . | . | . | . | . | . | . | . | . | . | . | . | . | . | . | . |
| 21-225_70E7_LC | . | . | . | . | . | . | N | . | . | . | . | . | . | . | . | . | . | . | . | . | . | . | . | . | . | . |
| 21-225_74A6_LC | . | . | . | . | . | S | . | . | . | . | . | . | . | . | . | . | . | . | . | . | . | . | . | . | . | . |
| 21-225_74A7_LC | . | . | . | . | . | S | . | . | . | . | . | . | . | . | . | . | . | . | . | . | . | . | . | . | . | . |
| 21-225_74B1_LC | . | . | . | . | . | . | . | . | . | . | . | . | . | . | . | . | . | . | . | . | . | . | . | . | . | . |
| 21-225_74B10_LC | . | . | . | . | . | . | . | . | . | . | . | . | . | . | . | . | . | . | . | . | . | . | . | . | . | . |
| 21-225_74B5_LC | . | . | . | . | . | . | . | . | . | . | . | . | . | . | . | . | . | . | . | . | . | . | . | . | . | . |
| 21-225_74B7_LC | . | . | . | . | . | S | . | . | . | . | . | . | . | . | . | . | . | . | . | . | . | . | . | . | . | . |
| 21-225_74B8_LC | . | . | . | . | . | S | . | . | . | . | . | . | . | . | . | . | . | . | . | . | . | . | . | . | . | . |
| 21-225_74C1_LC | . | . | . | . | . | . | . | . | . | . | . | . | . | . | . | . | . | . | . | . | . | . | . | . | . | . |
| 21-225_74F6_LC | . | . | . | . | . | S | . | . | . | . | . | . | . | . | . | . | . | . | . | . | . | . | . | . | . | . |
| 21-225_74F9_LC | S | . | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | . | . | . | . | . | . | . | . | . | . | . | . | . | . | . |
| 21-225_75A10_LC | S | . | . | . | . | . | . | . | . | . | . | . | . | . | . | . | . | . | . | . | . | . | . | . | . | . |
| 21-225_75A7_LC | . | . | . | . | . | S | . | . | . | . | . | . | . | . | . | . | . | . | . | . | . | . | . | . | . | . |
| 21-225_75C4_LC | . | . | . | . | . | . | I | . | . | . | . | . | . | . | . | . | . | . | . | . | . | . | . | . | . | . |
| 21-225_75F9_LC | . | . | . | . | . | . | . | . | . | . | . | . | . | . | . | . | . | . | . | . | . | . | . | . | . | . |
| 21-225_76E11_LC | . | . | . | . | . | . | . | . | . | . | . | . | . | . | . | . | . | . | . | . | . | . | . | . | . | . |
| 21-225_76E8_LC | . | . | . | . | . | S | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_76F3_LC | . | . | . | . | . | H | . | . | . | . | . | . | . | . | . | . | . | . | . | . | . | . | . | . | . | . |
| 21-225_76F5_LC | . | . | . | . | . | . | . | . | . | . | . | . | . | . | . | . | . | . | . | . | . | . | . | . | . | . |
| 21-225_76G2_LC | . | . | . | . | . | . | . | . | . | . | . | . | . | . | . | . | . | . | . | . | . | . | . | . | . | . |
| 21-225_77C10_LC | . | . | . | H | . | . | N | . | . | . | . | . | . | . | . | . | . | . | . | . | . | . | . | . | . | . |
| 21-225_77C7_LC | . | . | . | . | . | S | . | . | . | . | . | . | . | . | . | . | . | . | . | . | . | . | . | . | . | . |
| 21-225_77E1_LC | . | . | H | . | . | L | . | . | . | . | . | . | . | . | . | . | . | . | . | . | . | . | . | . | . | . |
| 21-225_78C1_LC | . | . | . | H | . | . | N | . | . | . | . | . | . | . | . | . | . | . | . | . | . | . | . | . | . | . |
| 21-225_78E11_LC | S | . | . | . | . | . | . | . | . | . | . | . | . | . | . | . | . | . | . | . | . | . | . | . | . | . |
| 21-225_78H12_LC | . | . | . | . | . | S | . | . | . | . | . | . | . | . | . | . | . | . | . | . | . | . | . | . | . | . |
| 21-225_79E9_LC | . | . | . | . | . | . | I | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | . | . | . | . | . | H | . | . | . | . | . | . | . | . | . | . | . | . | . | . | . | . | . | . | . | . |
| 21-225_79G6_LC | . | . | . | . | . | . | . | . | . | . | . | . | . | . | . | . | . | . | . | . | . | . | . | . | . | . |
| 21-225_80D3_LC | . | . | . | H | . | N | E | . | . | . | . | . | . | . | . | . | . | . | . | . | . | . | . | . | . | . |
| 21-225_80E9_LC | . | . | . | . | . | . | N | . | . | . | . | . | . | . | . | . | . | . | . | . | . | . | . | . | . | . |
| 21-225_80H3_LC | . | . | . | . | . | . | . | . | . | . | . | . | . | . | . | . | . | . | . | . | . | . | . | . | . | . |
| 21-225_81E5_LC | . | . | . | . | . | . | . | . | . | . | . | . | . | . | . | . | . | . | . | . | . | . | . | . | . | . |
| 21-225_81F9_LC | . | H | . | H | . | N | D | . | . | . | . | . | . | . | . | . | . | . | . | . | . | . | . | . | . | . |
| 21-225_83F3_LC | . | . | . | H | . | N | D | . | . | . | . | . | . | . | . | . | . | . | . | . | . | . | . | . | . | . |
| 21-225_83G3_LC | . | . | . | H | . | . | N | . | . | . | . | . | . | . | . | . | . | . | . | . | . | . | . | . | . | . |
| 21-225_83G7_LC | . | . | . | . | . | S | . | . | . | . | . | . | . | . | . | . | . | . | . | . | . | . | . | . | . | . |
| 21-225_84G7_LC | . | . | . | . | . | S | . | . | . | . | . | . | . | . | . | . | . | . | . | . | . | . | . | . | . | . |
| 21-225_85H2_LC | . | . | . | . | . | G | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | . | . | . | . | . | . |
| 21-225_86E9_LC | . | . | . | . | . | H | . | . | . | . | . | . | . | . | . | . | . | . | . | . | . | . | . | . | . | . |
| 21-225_88A1_LC | S | . | . | . | . | . | . | . | . | . | . | . | . | . | . | . | . | . | . | . | . | . | . | . | . | . |
| 21-225_88B4_LC | . | . | . | . | . | . | . | . | . | . | . | . | . | . | . | . | . | . | . | . | . | . | . | . | . | . |
| 21-225_88C10_LC | . | . | . | . | . | S | . | . | . | . | . | . | . | . | . | . | . | . | . | . | . | . | . | . | . | . |
| 21-225_88G2_LC | . | . | . | . | . | . | N | . | . | . | . | . | . | . | . | . | . | . | . | . | . | . | . | . | . | . |

4491

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
|  |  |  |  |  |  |  |  |  |  |  |  |  |  |  |  |  |  | K_CDR3 |  |  |  |  |  |  |  |  |
| CONSENSUS | Y | C | Q | Q | Y | Y | S | - | - | - | - | - | - | - | - | - | - | - | - | - | - | - | - | - | - | - |
| 21-225_90D9_LC | . | . | . | . | . | L | . | . | . | . | . | . | . | . | . | . | . | . | . | . | . | . | . | . | . | . |
| 21-225_91F1_LC | . | . | . | H | . | . | N | . | . | . | . | . | . | . | . | . | . | . | . | . | . | . | . | . | . | . |
| 21-225_91G8_LC | . | . | . | . | . | . | Y | . | . | . | . | . | . | . | . | . | . | . | . | . | . | . | . | . | . | . |
| 21-225_92E6_LC | . | . | . | . | . | H | . | . | . | . | . | . | . | . | . | . | . | . | . | . | . | . | . | . | . | . |
| 21-225_92F12_LC | . | . | . | . | . | . | . | . | . | . | . | . | . | . | . | . | . | . | . | . | . | . | . | . | . | . |
| 21-225_94D2_LC | . | . | . | . | . | H | . | . | . | . | . | . | . | . | . | . | . | . | . | . | . | . | . | . | . | . |
| 21-225_94E11_LC | . | . | . | . | . | H | . | . | . | . | . | . | . | . | . | . | . | . | . | . | . | . | . | . | . | . |
| 21-225_94E12_LC | . | . | . | . | . | . | . | . | . | . | . | . | . | . | . | . | . | . | . | . | . | . | . | . | . | . |
| 21-225_95E12_LC | . | . | . | . | . | . | . | . | . | . | . | . | . | . | . | . | . | . | . | . | . | . | . | . | . | . |
| 21-225_95G4_LC | . | . | . | . | . | H | . | . | . | . | . | . | . | . | . | . | . | . | . | . | . | . | . | . | . | . |
| 21-225_95H4_LC | . | . | . | . | . | H | . | . | . | . | . | . | . | . | . | . | . | . | . | . | . | . | . | . | . | . |
| 21-225_96D10_LC | . | . | . | . | . | H | . | . | . | . | . | . | . | . | . | . | . | . | . | . | . | . | . | . | . | . |
| 21-225_96D5_LC | . | . | . | . | . | . | . | . | . | . | . | . | . | . | . | . | . | . | . | . | . | . | . | . | . | . |
| 21-225_96E2_LC | . | . | . | . | . | . | H | . | . | . | . | . | . | . | . | . | . | . | . | . | . | . | . | . | . | . |
| 21-225_96H5_LC | . | . | . | . | . | . | . | . | . | . | . | . | . | . | . | . | . | . | . | . | . | . | . | . | . | . |
| 21-225_97H4_LC | . | . | . | H | . | N | D | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | P | T | F | G | Q | G | T | K | V | E | I | K | R |
| 21-225_11E5_LC | . | . | . | . | . | . | . | . | . | . | . | . | . | . | . | . | . | . | . |
| 21-225_12F12_LC | . | . | . | . | S | . | W | . | . | . | . | . | . | . | . | . | . | . | . |
| 21-225_147E4_LC | . | . | . | . | . | . | S | . | . | . | P | . | . | . | . | . | . | . | . |
| 21-225_148F8_LC | . | . | . | . | I | . | . | . | . | . | . | . | . | . | . | . | . | . | . |
| 21-225_149A2_LC | . | . | . | . | . | . | C | S | . | . | . | . | . | . | . | . | . | . | . |
| 21-225_149E3_LC | . | . | . | . | . | . | . | . | . | . | . | . | . | . | . | . | . | . | . |
| 21-225_149H4_LC | . | . | . | . | Y | . | . | . | . | . | . | . | . | . | . | . | . | . | . |
| 21-225_154A11_LC | . | . | . | . | S | . | . | . | . | . | . | . | . | . | . | . | . | . | . |
| 21-225_158B12_LC | . | . | . | . | . | . | . | . | . | . | . | . | . | . | . | . | . | . | . |
| 21-225_162F6_LC | . | . | . | . | . | . | . | . | . | . | . | . | . | . | . | . | . | . | . |
| 21-225_164A4_LC | . | . | . | . | . | . | W | . | . | . | . | . | . | . | . | . | . | . | . |
| 21-225_164A7_LC | . | . | . | . | I | . | L | . | . | . | . | . | . | . | . | . | . | . | . |
| 21-225_170F6_LC | . | . | . | . | . | . | W | . | . | . | . | . | . | . | . | . | . | . | . |
| 21-225_174G5_LC | . | . | . | . | . | . | W | . | . | . | . | . | . | . | . | . | . | . | . |
| 21-225_175D10_LC | . | . | . | . | . | . | . | . | . | . | . | . | . | . | . | . | . | . | . |
| 21-225_177B12_LC | . | . | . | . | S | . | . | . | . | . | . | . | . | . | . | . | . | . | . |
| 21-225_181E5_LC | . | . | . | . | . | . | W | . | . | . | . | . | . | . | . | . | . | . | . |
| 21-225_190D7_LC | . | . | . | . | . | . | W | . | . | . | . | . | . | . | . | . | . | . | . |
| 21-225_191D8_LC | . | . | . | . | . | . | . | . | . | . | L | . | . | . | . | . | . | . | . |
| 21-225_193G4_LC | . | . | . | . | . | . | W | . | . | . | . | . | . | . | . | O | . | . | . |
| 21-225_196G8_LC | . | . | . | . | . | . | . | . | . | . | . | . | . | . | . | . | . | . | . |
| 21-225_197C6_LC | . | . | . | . | . | . | . | . | . | . | L | . | . | . | . | . | . | . | . |
| 21-225_197H4_LC | . | . | . | . | . | . | . | . | . | . | L | . | . | . | . | . | . | . | . |
| 21-225_200G7_LC | . | . | . | . | . | . | . | . | . | . | . | . | . | . | . | . | . | . | . |
| 21-225_201F5_LC | . | . | . | . | . | . | . | . | . | . | . | . | . | . | . | . | . | . | . |
| 21-225_213H12_LC | . | . | . | . | I | . | W | . | . | . | . | . | . | . | . | . | . | . | . |
| 21-225_213H7_LC | . | . | . | . | I | . | . | . | . | . | R | . | . | . | . | . | . | . | . |
| 21-225_222H3_LC | . | . | . | . | I | . | W | . | . | . | . | . | . | . | . | . | . | N | . |
| 21-225_223G10_LC | . | . | . | . | . | . | W | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | P | T | F | G | Q | G | T | K | V | E | I | K | R |
| 21-225_224D8_LC | . | . | . | . | . | . | . | . | . | . | . | . | . | . | . | . | . | . | . |
| 21-225_224H5_LC | . | . | . | . | . | . | . | . | . | . | . | . | . | . | . | . | . | . | . |
| 21-225_225F11_LC | . | . | . | . | . | . | . | . | . | . | . | . | . | . | . | . | . | . | . |
| 21-225_225F5_LC | . | . | . | S | . | . | . | . | . | . | . | . | . | . | . | . | . | N | . |
| 21-225_226A12_LC | . | . | . | S | . | . | . | . | . | . | . | . | . | . | . | . | . | . | . |
| 21-225_226D11_LC | . | . | . | . | . | . | W | . | . | . | . | . | . | . | . | . | . | . | . |
| 21-225_226F6_LC | . | . | . | . | . | . | . | . | . | . | . | . | . | . | . | . | . | . | . |
| 21-225_227C7_LC | . | . | . | S | . | . | . | . | . | . | . | . | . | . | . | . | . | . | . |
| 21-225_23A12_LC | . | . | . | . | . | . | . | . | . | . | . | . | . | . | . | . | . | . | . |
| 21-225_23G12_LC | . | . | . | . | . | . | W | . | . | . | . | . | . | . | . | . | . | . | . |
| 21-225_24E1_LC | . | . | . | . | . | . | . | . | . | . | . | . | . | . | . | . | . | . | . |
| 21-225_25A4_LC | . | . | . | . | . | . | . | . | . | . | . | . | . | . | . | . | . | . | . |
| 21-225_25A4.001_LC | . | . | . | . | . | . | . | . | . | . | . | . | . | . | . | . | . | . | . |
| 21-225_25A4.001.001_LC | . | . | . | . | . | . | . | . | . | . | . | . | . | . | . | . | . | . | . |
| 21-225_25A4.001.002_LC | . | . | . | . | . | . | . | . | . | . | . | . | . | . | . | . | . | . | . |
| 21-225_25A4.001.003_LC | . | . | . | . | . | . | . | . | . | . | . | . | . | . | . | . | . | . | . |
| 21-225_25A4.001.004_LC | . | . | . | . | . | . | . | . | . | . | . | . | . | . | . | . | . | . | . |
| 21-225_25A4.001.005_LC | . | . | . | . | . | . | . | . | . | . | . | . | . | . | . | . | . | . | . |
| 21-225_25A4.001.006_LC | . | . | . | . | . | . | . | . | . | . | . | . | . | . | . | . | . | . | . |
| 21-225_25A4.001.007_LC | . | . | . | . | . | . | . | . | . | . | . | . | . | . | . | . | . | . | . |
| 21-225_25A4.001.008_LC | . | . | . | . | . | . | . | . | . | . | . | . | . | . | . | . | . | . | . |
| 21-225_25A4.001.013_LC | . | . | . | . | . | . | . | . | . | . | . | . | . | . | . | . | . | . | . |
| 21-225_25A4.001.019_LC | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . |
| 21-225_25A4.001.029_LC | . | . | . | . | . | . | . | . | . | . | . | . | . | . | . | . | . | . | . |
| 21-225_26C4_LC | . | . | . | . | . | . | . | . | . | . | . | . | . | . | . | . | . | . | . |
| 21-225_29E6_LC | . | . | . | . | . | . | . | . | . | . | . | . | . | . | . | . | . | . | . |
| 21-225_29H8_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | P | T | F | G | Q | G | T | K | V | E | I | K | R |
| 21-225_43D8_LC | . | . | . | . | . | . | . | . | . | . | . | . | . | . | . | . | . | . | . |
| 21-225_4A2.001.023_LC | . | . | . | . | . | . | V | . | . | . | . | . | . | . | . | . | . | . | . |
| 21-225_4A2.001.028_LC | . | . | . | . | . | . | V | . | . | . | . | . | . | . | . | . | . | . | . |
| 21-225_4A2.001.029_LC | . | . | . | . | . | . | V | . | . | . | . | . | . | . | . | . | . | . | . |
| 21-225_4A2.001.031_LC | . | . | . | . | . | . | V | . | . | . | P | . | . | . | . | . | . | . | . |
| 21-225_56A1_LC | . | . | . | . | . | . | . | . | . | . | . | . | . | . | . | . | . | . | . |
| 21-225_57A11_LC | . | . | . | . | . | . | W | . | . | . | . | . | . | . | . | . | P | . | . |
| 21-225_58B9_LC | . | . | . | . | . | . | . | . | . | . | . | . | S | . | . | . | . | . | . |
| 21-225_59F10_LC | . | . | . | . | . | . | . | . | . | . | . | . | . | . | . | . | . | . | . |
| 21-225_61B5_LC | . | . | . | . | . | . | . | . | . | . | . | . | S | . | . | . | . | . | . |
| 21-225_70E7_LC | . | . | . | . | I | . | . | . | . | . | . | . | . | . | . | . | . | . | . |
| 21-225_74A6_LC | . | . | . | . | . | . | W | . | . | . | . | . | . | Q | . | . | . | . | . |
| 21-225_74A7_LC | . | . | . | . | S | . | . | . | . | . | . | . | . | T | . | Q | . | . | . |
| 21-225_74B1_LC | . | . | . | . | . | . | . | . | . | . | . | . | . | . | . | . | . | . | . |
| 21-225_74B10_LC | . | . | . | . | I | . | . | . | . | . | . | . | . | . | . | . | . | . | . |
| 21-225_74B5_LC | . | . | . | . | S | . | . | . | . | . | . | . | . | T | . | Q | . | . | . |
| 21-225_74B7_LC | . | . | . | . | S | . | . | . | . | . | . | . | . | . | . | . | . | . | . |
| 21-225_74B8_LC | . | . | . | . | . | . | W | . | . | . | . | . | . | . | . | . | . | . | . |
| 21-225_74C1_LC | . | . | . | . | . | . | W | . | . | . | . | . | . | . | . | . | . | . | . |
| 21-225_74F6_LC | . | . | . | . | V | . | L | . | . | . | . | . | . | . | . | . | . | . | . |
| 21-225_74F9_LC | . | . | . | . | S | . | . | . | . | . | . | . | . | . | . | . | . | . | . |
| 21-225_74G4_LC | . | . | . | . | I | . | . | . | . | . | . | . | . | . | . | . | . | . | . |
| 21-225_74H2_LC | . | . | . | . | I | . | . | . | . | . | . | . | . | . | . | . | . | . | . |
| 21-225_75A10_LC | . | . | . | . | S | . | . | . | . | . | . | . | . | . | . | . | . | . | . |
| 21-225_75A7_LC | . | . | . | . | S | . | . | . | . | . | . | . | . | . | . | . | . | . | . |
| 21-225_75C4_LC | . | . | . | . | . | . | . | . | . | . | . | . | . | . | . | . | . | . | . |
| 21-225_75F9_LC | . | . | . | . | S | . | . | . | . | . | . | . | . | . | . | . | . | . | . |
| 21-225_76E11_LC | . | . | . | . | . | . | . | . | . | . | . | . | . | . | . | . | . | P | . |
| 21-225_76E8_LC | . | . | . | . | S | . | L | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | T | P | P | T | F | G | Q | G | T | K | V | E | I | K | R |
| 21-225_76F3_LC | . | . | . | . | S | . | L | . | . | . | . | . | . | T | . | Q | . | . | . |
| 21-225_76F5_LC | . | . | . | . | S | . | . | . | . | . | . | . | . | . | . | . | . | . | . |
| 21-225_76G2_LC | . | . | . | . | S | . | . | . | . | . | . | . | . | . | . | . | . | . | . |
| 21-225_77C10_LC | . | . | . | . | . | . | W | × | . | V | . | . | . | . | . | . | . | T | . |
| 21-225_77C7_LC | . | . | . | . | S | . | . | . | . | . | . | . | D | . | . | . | . | . | . |
| 21-225_77E1_LC | . | . | . | . | S | . | L | . | . | . | . | . | . | . | . | . | . | . | . |
| 21-225_78C1_LC | . | . | . | . | . | . | W | × | . | V | . | . | . | . | . | . | . | . | G |
| 21-225_78E11_LC | . | . | . | . | S | . | . | . | . | . | . | . | . | . | . | . | . | . | . |
| 21-225_78H12_LC | . | . | . | . | S | . | . | . | . | . | . | . | . | . | . | . | . | . | . |
| 21-225_79E9_LC | . | . | . | . | . | . | . | . | . | . | . | . | . | . | . | . | . | . | . |
| 21-225_79F12_LC | . | . | . | . | . | . | W | . | . | . | . | . | . | . | . | . | . | . | . |
| 21-225_79G10_LC | . | . | . | . | S | . | L | . | . | . | . | . | . | T | . | Q | . | . | . |
| 21-225_79G6_LC | . | . | . | . | I | . | . | . | . | . | . | . | . | . | . | . | . | . | . |
| 21-225_80D3_LC | . | . | . | . | . | . | G | × | . | V | . | V | . | . | . | . | . | T | G |
| 21-225_80E9_LC | . | . | . | . | . | . | W | . | . | V | . | . | . | . | . | . | . | . | . |
| 21-225_80H3_LC | . | . | . | . | . | . | . | . | . | . | . | . | . | . | . | . | . | . | . |
| 21-225_81E5_LC | . | . | . | . | S | . | L | . | . | . | . | . | . | T | . | Q | . | . | . |
| 21-225_81F9_LC | . | . | . | . | . | . | G | × | . | V | . | I | . | M | . | . | . | T | G |
| 21-225_83F3_LC | . | . | . | . | . | . | W | × | . | V | . | I | . | . | . | . | . | . | G |
| 21-225_83G3_LC | . | . | . | . | . | . | W | . | . | V | . | . | . | . | . | . | . | . | . |
| 21-225_83G7_LC | . | . | . | . | S | . | L | . | . | . | . | . | . | . | . | . | . | . | . |
| 21-225_84G7_LC | . | . | . | . | S | . | . | . | . | . | . | . | . | . | . | . | . | . | . |
| 21-225_85H2_LC | . | . | . | . | . | . | W | . | . | . | . | . | . | . | . | . | . | . | . |
| 21-225_85H9_LC | . | . | . | . | . | . | . | . | . | . | . | . | . | . | . | . | . | . | . |
| 21-225_86E9_LC | . | . | . | . | S | . | L | . | . | . | . | . | . | T | . | . | . | . | . |
| 21-225_88A1_LC | . | . | . | . | S | . | . | . | . | . | . | . | . | . | . | . | . | . | . |
| 21-225_88B4_LC | . | . | . | . | . | . | . | . | . | . | . | . | . | . | . | . | . | . | . |
| 21-225_88C10_LC | . | . | . | . | A | . | . | . | . | . | . | . | . | . | . | . | . | . | . |
| 21-225_88G2_LC | . | . | . | . | . | . | W | . | . | V | . | . | . | . | . | . | . | . | G |

# EP 4 435 105 A2

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K FR4 | | | | | |
| CONSENSUS | - | - | - | - | T | P | P | T | F | G | Q | G | T | K | V | E | I | K | R |
| 21-225_90D9_LC | . | . | . | . | S | . | L | . | . | . | . | . | . | . | . | . | . | . | . |
| 21-225_91F1_LC | . | . | . | . | . | . | W | K | . | V | . | . | . | . | . | . | . | . | . |
| 21-225_91G8_LC | . | . | . | . | . | . | W | . | . | . | . | . | . | . | . | . | . | . | . |
| 21-225_92E6_LC | . | . | . | . | V | . | . | . | . | . | . | . | . | . | . | . | . | . | . |
| 21-225_92F12_LC | . | . | . | . | . | . | . | . | . | . | . | . | . | . | . | . | . | . | . |
| 21-225_94D2_LC | . | . | . | . | I | . | . | . | . | . | H | . | . | . | . | . | . | . | G |
| 21-225_94E11_LC | . | . | . | . | S | . | L | . | . | . | . | . | . | T | . | Q | . | . | . |
| 21-225_94E12_LC | . | . | . | . | . | . | . | . | . | . | . | . | . | . | . | . | . | . | . |
| 21-225_95E12_LC | . | . | . | . | . | . | C | S | . | . | . | . | . | . | . | . | . | . | . |
| 21-225_95G4_LC | . | . | . | . | S | . | L | . | . | . | . | . | . | T | . | Q | . | . | . |
| 21-225_95H4_LC | . | . | . | . | S | . | L | . | . | . | . | . | . | T | . | Q | . | . | . |
| 21-225_96D10_LC | . | . | . | . | V | . | . | . | . | . | . | . | . | . | . | . | . | . | . |
| 21-225_96D5_LC | . | . | . | . | S | . | . | . | . | . | . | . | . | T | . | . | . | . | . |
| 21-225_96E2_LC | . | . | . | . | . | . | W | . | . | . | . | . | . | . | . | . | . | . | . |
| 21-225_96H5_LC | . | . | . | . | . | . | C | S | . | . | . | . | . | . | . | . | . | . | . |
| 21-225_97H4_LC | . | . | . | . | . | . | W | K | . | V | . | . | . | . | . | . | . | T | G |

4497

**FIGURE 57**

Table 100.    Consensus 20 - VK1|A30/JK3 (SEQ ID NO: 50317):

DIQMTQSPSSLSASVGDRVTITCRAS--QGIR------NDLGWYQQKPGKAPKRLIYA--------ASSLQSGVPSRFSGSGSG--
TEFTLTISSLQPEDFATYYCLQHNS----------------------YPFTFGPGTKVDIKR wherein:

A at position 25 can be substituted with T

G at position 30 can be substituted with D or V

I at position 31 can be substituted with M

R at position 32 can be substituted with S

N at position 39 can be substituted with K, S, D or T

D at position 40 can be substituted with N, H, Y, V, A, I or L

L at position 41 can be substituted with F

G at position 42 can be substituted with D

A at position 58 can be substituted with P, T, G, I, R or V

A at position 67 can be substituted with V

S at position 68 can be substituted with F or T

S at position 69 can be substituted with N, T, R or D

L at position 70 can be substituted with V

Q at position 71 can be substituted with L

S at position 72 can be substituted with N, T, G or R

L at position 107 can be substituted with I

Q at position 108 can be substituted with H or L

FIGURE 57

H at position 109 can be substituted with Y, D or L

N at position 110 can be substituted with Y, T, H, G or I

S at position 111 can be substituted with R, D, T, G or N

Y at position 135 can be substituted with F or H

P at position 136 can be substituted with L

F at position 137 can be substituted with L

T at position 138 can be substituted with K

Arg Xaa Ser Gln Xaa Xaa Xaa Xaa Xaa Xaa Xaa (SEQ ID NO: 50135)

Xaa Xaa Xaa Xaa Xaa Xaa Xaa (SEQ ID NO: 50136)

Xaa Xaa Xaa Xaa Xaa Xaa Xaa Xaa Xaa (SEQ ID NO: 50137)

Arg Ala Ser Gln Gly Ile Arg Asn Asp Leu Gly (SEQ ID NO: 50621)

Ala Ala Ser Ser Leu Gln Ser (SEQ ID NO: 50622)

Leu Gln His Asn Ser Tyr Pro Phe Thr (SEQ ID NO: 50623)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_14D6_LC | . | . | . | . | . | . | . | . | . | . | . | . | . | . | . | . | . | S | . | . | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | . | . | . | . | . | . | . | . | . | C | . | . | . | . | . | . | . | . | . | . | . | . | . | . |
| 21-225_157H12_LC | . | . | . | . | . | . | . | . | . | . | . | . | . | . | . | . | . | . | . | . | . | . | . | . | . | . |
| 21-225_157H4_LC | . | . | . | . | . | . | . | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | . | . | . | . | . | . | . | . | . | . | . | . | . | . | . | . | . | . | . | . | . | . |
| 21-225_158G8_LC | . | . | . | . | . | . | . | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . | . | . | . | . | . | T | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | . | . | . | . | . | . | P | . | . | . | . | . | . | . | . | . | . | . | . | . | . | . |
| 21-225_161D11_LC | . | . | . | . | . | . | . | . | . | . | . | . | . | . | . | . | . | . | . | . | . | . | . | . | . | . |
| 21-225_161F7_LC | . | . | . | . | . | . | . | . | . | . | . | . | . | . | . | . | . | . | . | . | . | . | . | . | . | . |
| 21-225_162F2_LC | . | . | . | . | . | . | . | . | . | . | . | C | . | . | . | . | . | . | . | . | . | . | . | . | . | . |
| 21-225_162H3_LC | . | . | . | . | . | . | . | . | . | . | . | . | . | . | . | . | . | . | . | . | . | . | . | . | . | . |
| 21-225_163E2_LC | . | . | . | . | . | . | . | . | . | . | . | . | . | . | . | . | . | . | . | . | . | . | . | . | . | . |
| 21-225_163F9_LC | . | . | . | . | . | . | . | . | . | . | . | . | . | . | . | . | . | . | . | . | . | . | . | . | . | . |
| 21-225_163G10_LC | . | . | . | . | . | . | . | . | . | . | . | . | . | . | . | . | . | . | . | . | . | . | . | . | . | . |
| 21-225_16A11_LC | . | . | . | . | . | . | . | . | . | . | . | . | . | . | . | . | . | S | . | . | . | S | . | . | . | . |
| 21-225_16H1_LC | . | . | . | . | . | . | . | . | . | . | . | . | . | . | . | . | . | . | . | . | . | . | . | . | . | . |
| 21-225_16H9_LC | . | . | . | . | . | . | . | . | . | . | . | . | . | . | . | . | . | . | . | . | . | . | . | . | . | . |
| 21-225_171A11_LC | . | . | . | . | . | . | . | . | . | . | . | . | . | . | . | . | . | . | . | . | . | . | . | . | . | . |
| 21-225_177B6_LC | . | . | . | . | . | . | . | . | . | . | . | . | . | . | . | . | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | . | . |
| 21-225_181A8_LC | . | . | . | . | . | . | . | . | . | . | . | . | . | . | . | . | . | . | . | . | . | . | . | . | . | . |
| 21-225_184D11_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | . | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | . | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | S | . | . | . | . | . | . | . | . | . | . | . | . | . | . | . | . | . | . | . | . | . |
| 21-225_203B9_LC | . | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | . | . | . | ? | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | . | . | . | . | . | . | . | . | . | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_29D9_LC | . | . | . | . | . | . | . | . | . | . | . | . | . | . | . | . | . | . | . | . | . | . | . | . | . | . |
| 21-225_30H11_LC | . | . | . | . | . | . | . | . | . | . | . | . | . | . | . | . | . | . | . | . | . | . | . | . | . | . |
| 21-225_31G9_LC | . | . | . | . | . | . | . | . | . | . | . | . | . | . | I | . | . | . | . | . | . | . | . | . | . | . |
| 21-225_43B1_LC | . | . | . | . | . | . | . | . | . | . | . | . | . | . | . | . | . | . | . | . | . | . | . | . | . | . |
| 21-225_43E1_LC | . | . | . | . | . | . | . | . | . | . | . | . | . | . | . | . | . | . | . | . | . | . | . | . | . | . |
| 21-225_43E5_LC | . | . | . | . | . | . | . | . | . | . | . | . | . | . | . | . | . | . | . | . | . | . | . | . | . | . |
| 21-225_43H8_LC | . | . | . | . | . | . | . | . | . | . | . | . | . | . | . | . | . | . | . | . | . | . | . | . | . | . |
| 21-225_44C8_LC | . | . | . | . | . | . | . | . | . | . | . | . | . | . | . | . | . | . | . | . | . | . | . | . | . | . |
| 21-225_45H2_LC | . | . | . | . | . | . | . | . | . | . | . | . | . | . | . | . | . | . | . | . | . | . | . | . | . | . |
| 21-225_46E9_LC | . | . | . | . | . | . | . | . | . | . | . | . | . | . | . | . | . | . | . | . | . | . | . | . | . | . |
| 21-225_47H7_LC | . | . | . | . | . | . | . | . | . | . | . | . | . | . | . | . | . | . | . | . | . | . | . | . | Y | . |
| 21-225_48E10_LC | . | . | . | . | . | . | . | . | . | . | . | . | . | . | . | . | . | . | . | . | . | . | . | . | . | . |
| 21-225_50C4_LC | . | . | . | . | . | . | . | . | . | . | . | . | . | . | . | . | . | . | L | . | . | . | . | . | . | . |
| 21-225_51B11_LC | A | . | . | . | . | . | . | . | . | . | . | . | . | . | . | . | . | . | . | . | . | . | . | . | Y | . |
| 21-225_51E4_LC | . | . | . | . | . | . | . | . | . | . | . | . | . | . | . | . | . | . | . | . | . | . | . | . | . | . |
| 21-225_51F9_LC | . | . | . | . | . | . | . | . | . | . | . | . | . | . | . | . | . | . | . | . | . | . | . | . | . | . |
| 21-225_52B9_LC | A | . | . | . | . | . | . | . | . | . | . | . | . | . | . | . | . | . | . | . | . | . | . | . | . | . |
| 21-225_52H12_LC | . | . | . | . | . | . | . | . | . | . | . | . | . | . | . | . | . | . | . | . | . | . | . | . | . | . |
| 21-225_53B10_LC | . | . | . | . | . | . | . | . | . | . | . | . | . | A | . | . | . | . | . | . | . | . | . | . | . | . |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | . | . | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . | . | . | . | . | . | . | . |
| 21-225_56A5_LC | . | . | . | . | . | . | . | . | . | . | . | . | . | . | . | . | . | . | . | . | . | . | . | . | . | . |
| 21-225_56H7_LC | . | . | . | L | . | . | . | . | . | . | . | . | . | . | . | . | . | . | . | . | . | . | . | . | . | . |
| 21-225_57A4_LC | . | . | . | . | . | . | . | . | . | . | . | . | . | . | . | . | . | . | . | . | . | . | . | . | . | . |
| 21-225_57B2_LC | . | . | . | . | . | . | . | . | . | . | . | V | . | . | . | . | . | . | . | . | . | . | . | . | . | . |
| 21-225_57F4_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_58D11_LC | . | | | | | | | | | | | | | | | | | | | | | | | | | |
| 21-225_58F5_LC | . | . | . | . | . | . | . | . | . | . | . | . | . | . | . | . | . | . | . | . | . | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | . | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | Ε | . | . | . | . | . | . | . | . | . | . | . | . | . | . | . | . | . | . | . | . | . | . | . |
| 21-225_64A12_LC | . | . | . | . | . | . | . | . | . | . | . | . | . | . | . | . | . | . | . | . | . | . | . | . | . | . |
| 21-225_66F9_LC | . | . | . | . | . | . | . | . | . | . | . | . | . | . | . | . | . | . | . | . | . | . | . | . | . | . |
| 21-225_67B7_LC | . | . | . | . | . | . | . | . | . | . | . | . | . | . | . | . | . | . | . | . | . | . | . | . | . | . |
| 21-225_68G8_LC | . | . | . | . | . | . | . | . | . | . | . | . | . | . | . | . | . | . | . | . | . | . | . | . | . | . |
| 21-225_71B11_LC | . | . | . | . | . | . | . | . | . | . | . | . | . | . | . | . | . | . | . | . | . | . | . | . | . | . |
| 21-225_71B6_LC | . | . | . | . | . | . | . | . | . | . | Ε | . | . | . | . | . | . | . | . | . | . | . | . | . | . | . |
| 21-225_71H6_LC | . | . | . | . | . | . | . | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . | . | . | . | . | . | . | . |
| 21-225_74A11_LC | . | . | . | . | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . | . | . | . | . | . | . | . |
| 21-225_76H10_LC | . | . | . | . | . | . | . | . | . | . | . | . | . | . | A | . | . | . | . | . | . | . | . | . | . | . |
| 21-225_76H12_LC | . | . | . | . | . | . | . | . | . | . | . | . | . | . | . | . | . | . | . | . | . | . | . | . | . | . |
| 21-225_84H12_LC | . | . | . | . | . | . | . | . | Y | Q | . | . | . | . | . | . | . | . | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_14D6_LC | . | . | . | D | . | . | . | . | . | . | . | . | . | N | . | . | . | . | . | N | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | . | . | . | . | . | . | . | . | . | . | . | . | . | . | . | . | . | . | . | . | . | . | . | . |
| 21-225_157H12_LC | . | . | . | . | . | . | . | . | . | . | . | . | . | . | F | . | . | . | . | N | . | . | . | . | . | . |
| 21-225_157H4_LC | . | . | . | . | . | . | . | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | . | . | . | . | . | . | . | . | . | . | . | . | . | . | . | . | . | . | . | . | . | . |
| 21-225_158G8_LC | . | . | . | D | . | . | . | . | . | . | . | . | . | . | . | . | . | . | . | . | . | . | . | . | . | . |
| 21-225_158H6_LC | . | . | . | . | . | . | . | . | . | . | . | . | S | . | . | . | . | . | . | . | . | . | . | . | . | . |
| 21-225_159C5_LC | . | . | . | . | . | . | . | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | . | . | . | . | . | . | . | . | . | . | . | . | . | . | . | . | . | . | . | . | . | . |
| 21-225_161D11_LC | . | . | . | . | . | . | . | . | . | . | . | . | . | . | . | . | . | . | . | . | . | . | . | E | . | . |
| 21-225_161F7_LC | . | . | . | . | . | . | . | . | . | . | . | . | . | . | . | . | . | . | . | . | . | . | . | E | . | . |
| 21-225_162F2_LC | . | . | . | . | . | . | . | . | . | . | . | . | . | . | . | . | . | . | . | . | . | . | . | . | . | . |
| 21-225_162H3_LC | . | . | . | . | . | . | . | . | . | . | . | . | . | . | . | . | . | . | . | . | . | . | . | . | . | . |
| 21-225_163E2_LC | . | . | . | D | . | . | . | . | . | . | . | . | . | . | . | . | . | . | . | . | . | . | . | . | . | . |
| 21-225_163F9_LC | . | . | . | D | . | . | . | . | . | . | . | . | D | . | . | . | . | . | . | . | . | . | . | . | . | . |
| 21-225_163G10_LC | . | . | . | D | . | . | . | . | . | . | . | . | . | N | . | . | . | . | . | . | . | . | . | . | . | . |
| 21-225_16A11_LC | . | . | . | D | . | . | . | . | . | . | . | . | . | N | . | . | . | . | . | R | N | . | . | . | . | . |
| 21-225_16H1_LC | . | . | . | . | . | . | . | . | . | . | . | . | D | I | . | . | . | . | . | . | . | . | . | . | . | . |
| 21-225_16H9_LC | . | . | . | . | . | . | . | . | . | . | . | . | . | Y | . | . | F | . | . | . | . | . | . | . | . | . |
| 21-225_171A11_LC | . | . | . | . | . | . | . | . | . | . | . | . | . | N | . | . | . | . | . | . | . | . | . | . | . | . |
| 21-225_177B6_LC | . | . | . | D | . | S | . | . | . | . | . | . | . | . | . | . | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | . | . |
| 21-225_181A8_LC | . | . | . | . | . | . | . | . | . | . | . | . | . | . | . | . | . | . | . | . | . | . | . | . | . | . |
| 21-225_184D11_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | L | . | . | . | . | . | . |
| 21-225_192F6_LC | . | . | . | . | . | . | . | . | . | . | . | . | . | . | . | . | . | . | . | L | . | . | . | . | . | . |
| 21-225_194F10_LC | . | . | . | . | . | . | . | . | . | . | . | . | . | . | . | . | . | . | . | L | . | . | . | . | . | . |
| 21-225_195B9_LC | . | . | . | . | . | . | . | . | . | . | . | . | K | . | . | . | . | . | . | . | . | . | . | . | . | . |
| 21-225_197D7_LC | . | . | . | . | . | . | . | . | . | . | . | . | K | . | . | . | . | . | . | . | . | . | . | . | . | . |
| 21-225_197G10_LC | . | . | . | . | . | . | . | . | . | . | . | . | . | . | . | . | . | . | . | . | . | . | . | . | . | . |
| 21-225_198B9_LC | . | . | . | . | . | . | . | . | . | . | . | . | K | . | . | . | . | . | . | . | . | . | . | . | . | . |
| 21-225_198E3_LC | . | . | . | . | . | . | . | . | . | . | . | . | K | . | . | . | . | . | . | . | . | . | . | . | . | . |
| 21-225_200F6_LC | . | . | . | D | . | . | . | . | . | . | . | . | . | . | . | . | . | . | . | . | . | . | . | . | . | . |
| 21-225_202A3_LC | . | . | . | . | . | . | . | . | . | . | . | . | . | . | . | . | . | . | . | . | . | . | . | . | . | . |
| 21-225_203B9_LC | . | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | M | . | . | . | . | . | . | . | K | . | . | . | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | D | . | . | . | . | . | . | . | . | . | . | . | . | . | . | . | . | . | . | . | . | . | . |
| 21-225_22H3_LC | . | . | . | D | . | . | . | . | . | . | . | . | . | N | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | D | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | N | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_29D9_LC | . | . | . | . | . | . | . | . | . | . | . | . | . | . | . | . | . | F | . | . | . | . | . | . | . | . |
| 21-225_30H11_LC | . | . | . | . | . | . | . | . | . | . | . | . | . | . | . | . | . | . | . | . | . | . | . | . | . | . |
| 21-225_31G9_LC | . | . | . | . | . | . | . | . | . | . | . | . | . | . | . | . | . | . | . | . | . | . | . | . | . | . |
| 21-225_43B1_LC | . | . | . | . | . | . | . | . | . | . | . | . | . | . | . | . | . | . | . | . | . | . | . | . | . | . |
| 21-225_43E1_LC | . | . | . | . | . | . | . | . | . | . | . | . | . | . | . | . | . | . | . | R | . | . | . | . | . | . |
| 21-225_43E5_LC | . | . | . | . | . | . | . | . | . | . | . | . | . | . | . | . | . | . | . | . | . | . | . | . | . | . |
| 21-225_43H8_LC | . | . | . | . | . | . | . | . | . | . | . | . | . | . | . | . | . | R | . | . | . | . | . | . | . | . |
| 21-225_44C8_LC | . | . | . | . | . | . | . | . | . | . | . | . | . | . | . | . | . | . | . | . | . | . | . | . | . | . |
| 21-225_45H2_LC | . | . | . | . | . | . | . | . | . | . | . | . | . | . | . | . | . | . | . | . | . | . | . | . | . | . |
| 21-225_46E9_LC | . | . | . | . | . | . | . | . | . | . | . | . | . | . | . | . | . | . | . | . | . | . | . | . | . | . |
| 21-225_47H7_LC | . | . | . | . | . | . | . | . | . | . | . | . | . | . | . | . | . | . | . | R | . | . | . | . | . | . |
| 21-225_48E10_LC | . | . | . | . | . | . | . | . | . | . | . | . | S | . | . | . | . | . | . | . | . | . | . | . | . | . |
| 21-225_50C4_LC | . | . | . | . | . | . | . | . | . | . | . | . | . | . | . | . | . | . | . | . | . | . | . | . | I | . |
| 21-225_51B11_LC | . | . | . | . | . | . | . | . | . | . | . | . | . | . | . | . | . | . | . | . | . | . | R | . | . | . |
| 21-225_51E4_LC | . | . | . | . | . | . | . | . | . | . | . | . | . | . | . | . | . | . | . | . | . | . | . | . | . | . |
| 21-225_51F9_LC | . | . | . | . | . | . | . | . | . | . | . | . | T | . | . | . | . | . | . | . | . | . | R | . | . | . |
| 21-225_52B9_LC | . | . | . | . | . | . | . | . | . | . | . | . | . | . | . | . | . | . | . | . | . | . | R | . | . | . |
| 21-225_52H12_LC | . | . | . | . | . | . | . | . | . | . | . | . | N | . | . | . | . | . | . | . | . | . | . | . | . | . |
| 21-225_53B10_LC | . | . | D | . | . | . | . | . | . | . | . | . | Y | . | . | . | . | . | . | . | . | . | . | . | . | . |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | . | . | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . | . | . | . | . | . | . | . |
| 21-225_56A5_LC | . | . | D | . | . | . | . | . | . | . | . | . | L | . | . | . | . | . | . | . | . | . | . | . | . | . |
| 21-225_56H7_LC | . | . | D | . | . | . | . | . | . | . | . | . | . | . | . | . | . | . | . | R | . | . | . | . | . | . |
| 21-225_57A4_LC | . | . | . | . | . | . | . | . | . | . | . | . | . | . | . | . | . | L | . | . | . | . | . | . | . | . |
| 21-225_57B2_LC | . | . | . | . | . | . | . | . | . | . | . | . | A | . | . | . | . | . | . | . | . | . | . | . | . | . |
| 21-225_57F4_LC | . | . | V | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_58D11_LC | . | . | . | . | . | . | . | . | . | . | . | . | S | . | . | D | . | . | . | . | . | . | . | . | . | . |
| 21-225_58F5_LC | . | . | . | . | . | . | . | . | . | . | . | . | . | . | . | . | . | F | L | . | T | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | H | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | . | D | . | . | . | . | . | . | . | . | . | N | . | . | . | . | . | . | . | . | . | . | . | . |
| 21-225_64A12_LC | . | . | . | . | . | . | . | . | . | . | . | . | . | . | . | . | . | . | . | . | . | . | . | . | . | . |
| 21-225_66F9_LC | . | . | . | D | . | . | . | . | . | . | . | . | . | V | . | . | . | . | . | . | . | . | . | . | . | . |
| 21-225_67B7_LC | . | . | . | . | . | . | . | . | . | . | . | . | . | . | . | . | . | F | . | . | . | . | . | . | . | . |
| 21-225_68G8_LC | . | . | . | . | . | . | . | . | . | . | . | . | . | N | . | . | . | . | . | . | R | . | . | . | . | . |
| 21-225_71B11_LC | . | . | . | . | . | . | . | . | . | . | . | . | . | . | . | . | D | . | . | . | Y | . | . | . | . | . |
| 21-225_71B6_LC | . | . | . | . | . | . | . | . | . | . | . | . | . | . | . | . | D | . | . | . | . | . | . | . | . | . |
| 21-225_71H6_LC | . | . | . | . | . | . | . | . | . | . | . | . | . | V | . | . | . | . | . | . | . | . | . | . | . | . |
| 21-225_73A6_LC | . | . | . | . | . | . | . | . | . | . | . | . | . | . | . | . | . | F | . | . | . | . | . | . | . | . |
| 21-225_74A11_LC | . | . | . | D | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | F | . | . | . | . | . | . | . | . |
| 21-225_76H10_LC | . | . | . | D | . | . | . | . | . | . | . | . | . | Y | . | . | . | . | . | . | . | . | . | . | . | . |
| 21-225_76H12_LC | . | . | . | . | . | . | . | . | . | . | . | . | . | . | . | . | . | F | . | . | . | . | . | . | . | . |
| 21-225_84H12_LC | . | . | . | . | . | . | . | . | . | . | . | . | . | . | . | . | . | F | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | D | . | . | . | . | . | . | . | . | . | . | . | . | . | . | . | . | . | . | . | . | . | . |

# EP 4 435 105 A2

## FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | K_CDR2 | | | | | | | | | | | | | | | | |
| CONSENSUS | K | R | L | I | Y | A | - | - | - | - | - | - | - | - | A | S | S | L | Q | S | G | V | P | S | R | F |
| 21-225_14D6_LC | . | . | . | . | . | . | . | . | . | . | . | . | . | . | . | . | . | . | . | . | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | . | . | N | V | . | . | . | . | . | . | . | . | . | . | . | . | . | . | . | . | . | . | . | . |
| 21-225_157H12_LC | . | O | . | . | H | . | . | . | . | . | . | . | . | . | . | . | . | . | . | . | . | . | . | . | . | . |
| 21-225_157H4_LC | . | . | . | . | . | P | . | . | . | . | . | . | . | . | . | . | . | . | . | T | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | N | . | . | . | . | . | . | . | . | . | . | . | . | . | . | . | . | . | . | . | . | . |
| 21-225_158G8_LC | . | . | M | . | . | T | . | . | . | . | . | . | . | . | . | . | . | . | . | . | . | . | . | . | . | . |
| 21-225_158H6_LC | . | . | . | . | . | T | . | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | N | V | . | . | . | . | . | . | . | . | . | . | . | . | . | . | . | . | . | . | . | . |
| 21-225_161D11_LC | . | . | . | . | . | . | . | . | . | . | . | . | . | . | . | . | T | . | . | . | . | . | . | . | . | . |
| 21-225_161F7_LC | . | . | . | . | . | . | . | . | . | . | . | . | . | . | . | . | T | . | . | . | . | . | . | . | . | . |
| 21-225_162F2_LC | . | . | . | . | N | . | . | . | . | . | . | . | . | . | . | . | . | . | . | . | . | . | . | . | . | . |
| 21-225_162H3_LC | . | . | . | . | N | . | . | . | . | . | . | . | . | . | . | . | . | . | . | . | . | . | . | . | . | . |
| 21-225_163E2_LC | T | . | . | . | . | P | . | . | . | . | . | . | . | . | . | . | . | . | . | . | . | . | . | . | . | . |
| 21-225_163F9_LC | . | . | . | . | . | P | . | . | . | . | . | . | . | . | . | . | . | . | . | . | . | . | . | . | . | . |
| 21-225_163G10_LC | . | . | . | . | . | P | . | . | . | . | . | . | . | . | . | . | . | . | . | . | . | . | . | . | . | . |
| 21-225_16A11_LC | . | . | . | . | . | . | . | . | . | . | . | . | . | . | . | . | . | . | . | . | . | . | . | . | . | . |
| 21-225_16H1_LC | . | . | . | . | . | . | . | . | . | . | . | . | . | . | . | . | . | . | . | . | N | . | . | . | . | . |
| 21-225_16H9_LC | . | . | . | . | . | G | . | . | . | . | . | . | . | . | . | . | . | . | . | . | . | . | . | . | . | ? |
| 21-225_171A11_LC | . | . | . | . | . | P | . | . | . | . | . | . | . | . | . | . | . | . | . | . | . | . | . | . | . | . |
| 21-225_177B6_LC | . | . | . | . | . | . | . | . | . | . | . | . | . | . | . | . | . | . | . | . | . | . | . | . | . | . |
| 21-225_17E5_LC | . | . | . | . | . | T | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | T | . | . | . | . | . | . | . | . | . | . | . | . | . | . | . | . | . | . | . | . |
| 21-225_181A8_LC | . | . | . | . | . | T | . | . | . | . | . | . | . | . | . | . | . | . | . | . | . | . | . | . | . | . |
| 21-225_184D11_LC | . | . | . | . | . | ? | . | . | . | . | . | . | . | . | . | . | . | . | . | . | . | . | . | . | . | . |

4508

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | K_CDR2 | | | | | | | | | | | | | | | |
| CONSENSUS | K | R | L | I | Y | A | - | - | - | - | - | - | - | - | A | S | S | L | Q | S | G | V | P | S | R | F |
| 21-225_184H10_LC | . | . | . | . | . | I | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | G | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | G | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | G | . | . | . | . | . | . | . | . | . | . | . | P | . | . | . | . | . | . | . | . | . |
| 21-225_203B9_LC | . | . | . | . | . | R | . | . | . | . | . | . | . | . | . | . | . | V | . | N | . | . | . | . | . | . |
| 21-225_210E4_LC | . | . | . | . | . | . | . | . | . | . | . | . | . | . | . | Y | . | . | . | . | . | . | . | . | . | . |
| 21-225_225B6_LC | . | . | . | . | . | . | . | . | . | . | . | . | . | . | T | . | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | L | G | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | P | . | . | . | . | . | . | . | . | . | . | . | . | . | T | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | R | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | N | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | T | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_184H10_LC | . | . | . | . | . | I | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | G | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | G | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | C | . | . | . | . | . | . | . | . | . | . | . | P | . | . | . | . | . | . | . | . | . |
| 21-225_203B9_LC | . | . | . | . | . | R | . | . | . | . | . | . | . | . | . | . | . | . | V | . | N | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | T | . | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | L | G | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | P | . | . | . | . | . | . | . | . | . | . | . | . | . | . | T | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | R | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | . | N | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | Y | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_29D9_LC | . | . | . | . | . | . | . | . | . | . | . | . | . | . | . | . | . | . | . | . | . | . | . | . | . | . |
| 21-225_30H11_LC | . | . | . | V | . | P | . | . | . | . | . | . | . | . | . | . | . | . | . | . | . | . | . | . | . | . |
| 21-225_31G9_LC | . | . | . | . | . | P | . | . | . | . | . | . | . | . | . | . | . | . | . | . | . | . | . | . | . | . |
| 21-225_43B1_LC | . | . | . | . | . | . | . | . | . | . | . | . | . | . | . | . | . | . | . | . | . | . | . | . | . | . |
| 21-225_43E1_LC | . | . | . | . | N | . | . | . | . | . | . | . | . | . | . | . | . | . | . | . | . | . | . | . | . | . |
| 21-225_43E5_LC | . | . | . | . | . | G | . | . | . | . | . | . | . | . | . | . | N | . | . | . | . | . | . | . | . | . |
| 21-225_43H8_LC | . | . | . | . | . | . | . | . | . | . | . | . | . | . | . | . | . | . | . | . | . | . | . | . | . | . |
| 21-225_44C8_LC | . | . | . | . | . | . | . | . | . | . | . | . | . | . | . | . | N | . | . | . | . | . | . | . | . | . |
| 21-225_45H2_LC | . | . | . | . | . | G | . | . | . | . | . | . | . | . | . | . | N | . | . | . | . | . | . | . | . | . |
| 21-225_46E9_LC | . | . | . | . | . | . | . | . | . | . | . | . | . | . | . | . | . | . | . | . | . | . | . | . | . | . |
| 21-225_47H7_LC | . | . | . | . | . | . | . | . | . | . | . | . | . | . | . | . | . | . | . | . | . | . | . | . | . | . |
| 21-225_48E10_LC | . | . | . | . | . | T | . | . | . | . | . | . | . | . | . | . | . | . | . | . | . | . | . | . | . | . |
| 21-225_50C4_LC | . | . | . | . | . | . | . | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . | . | . | . | . | . | . | . |
| 21-225_51E4_LC | Q | . | . | . | . | . | . | . | . | . | . | . | . | . | . | . | . | . | . | . | . | . | . | . | . | . |
| 21-225_51F9_LC | Q | . | . | . | . | . | . | . | . | . | . | . | . | . | . | . | . | . | . | S | . | . | . | . | . | . |
| 21-225_52B9_LC | . | . | . | . | . | . | . | . | . | . | . | . | . | . | . | . | . | . | . | . | . | . | . | L | . | . |
| 21-225_52H12_LC | . | . | . | . | . | G | . | . | . | . | . | . | . | . | . | . | . | . | . | . | . | . | . | . | . | . |
| 21-225_53B10_LC | . | . | . | . | . | G | . | . | . | . | . | . | . | . | . | . | . | . | . | . | . | . | . | . | . | . |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | P | . | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . | . | . | . | . | . | . | . |
| 21-225_56A5_LC | . | . | . | . | . | . | . | . | . | . | . | . | . | . | . | . | . | . | . | . | . | . | . | . | . | . |
| 21-225_56H7_LC | . | . | . | . | . | P | . | . | . | . | . | . | . | . | . | . | . | . | L | . | . | . | . | . | . | . |
| 21-225_57A4_LC | . | . | . | . | . | . | . | . | . | . | . | . | . | . | . | . | . | . | L | . | . | . | . | . | . | . |
| 21-225_57B2_LC | . | . | . | . | . | . | . | . | . | . | . | . | . | . | . | . | . | . | . | . | . | . | . | . | . | . |
| 21-225_57F4_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_58D11_LC | . | . | . | . | . | . | . | . | . | . | . | . | . | . | . | . | . | . | . | . | . | . | . | . | . | . |
| 21-225_58F5_LC | . | . | . | . | . | . | . | . | . | . | . | . | . | . | . | . | . | . | L | . | . | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | . | . | . | . | . | . | . | . | . | . | . | . | . |
| 21-225_60A3_LC | . | . | . | . | . | . | . | . | . | . | . | . | . | . | . | S | . | . | . | . | . | . | . | . | . | . |
| 21-225_62G3_LC | . | . | . | . | . | T | . | . | . | . | . | . | . | . | . | . | . | . | . | . | . | . | . | . | . | . |
| 21-225_64A12_LC | T | . | . | . | . | T | . | . | . | . | . | . | . | . | . | T | . | . | . | . | . | . | . | . | . | . |
| 21-225_66F9_LC | . | . | . | . | . | T | . | . | . | . | . | . | . | . | . | . | . | . | . | . | . | . | . | . | G | . |
| 21-225_67B7_LC | . | . | . | . | . | . | . | . | . | . | . | . | . | . | . | . | . | . | . | . | . | . | . | . | . | . |
| 21-225_68G8_LC | . | . | . | . | . | . | . | . | . | . | . | . | . | . | . | . | V | . | . | . | . | . | . | . | . | . |
| 21-225_71B11_LC | . | . | . | . | . | . | . | . | . | . | . | . | . | . | . | . | . | . | . | . | . | . | . | . | . | . |
| 21-225_71B6_LC | Q | . | . | . | . | . | . | . | . | . | . | . | . | . | . | . | . | . | . | . | . | . | . | . | . | . |
| 21-225_71H6_LC | . | . | . | . | . | . | . | . | . | . | . | . | . | . | . | . | . | . | . | . | . | . | . | . | . | . |
| 21-225_73A6_LC | . | . | . | . | . | . | . | . | . | . | . | . | . | . | . | N | . | . | . | . | . | . | . | . | . | . |
| 21-225_74A11_LC | . | . | . | Q | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . | . | . | . | . | . | . | . |
| 21-225_76H10_LC | . | . | . | . | . | G | . | . | . | . | . | . | . | . | . | . | . | . | . | . | . | . | . | . | . | . |
| 21-225_76H12_LC | . | . | . | . | . | . | . | . | . | . | . | . | . | . | . | R | . | . | . | . | . | . | . | . | . | . |
| 21-225_84H12_LC | . | . | . | . | . | . | . | . | . | . | . | . | . | . | . | . | . | . | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | . | . | S | . | . | . | . | . | . | . | . | V | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_14D6_LC | . | . | . | . | . | . | . | . | . | . | . | . | . | . | . | . | . | . | . | . | . | . | . | . | . | . |
| 21-225_157C3_LC | . | . | . | . | . | . | . | . | . | D | . | . | . | . | . | . | . | . | . | . | D | . | . | . | . | . |
| 21-225_157G7_LC | . | . | . | . | . | . | . | . | . | . | . | . | . | . | . | . | . | V | . | R | . | . | . | . | . | . |
| 21-225_157H12_LC | . | . | . | . | . | . | . | . | . | . | . | . | . | . | . | . | . | . | . | . | . | . | . | . | . | . |
| 21-225_157H4_LC | . | . | . | . | . | . | . | . | . | . | . | . | . | . | . | . | . | . | . | . | . | . | . | . | . | . |
| 21-225_157H8_LC | . | . | . | . | . | . | . | . | . | D | . | . | . | . | . | . | . | . | . | . | D | . | . | . | . | . |
| 21-225_158F5_LC | . | . | . | . | . | . | . | . | . | . | . | . | . | . | . | . | . | . | . | . | . | . | . | . | . | . |
| 21-225_158G8_LC | . | . | . | . | . | . | . | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . | . | . | . | . | . | . | . |
| 21-225_159C5_LC | . | . | . | . | . | . | . | . | . | D | . | . | . | . | . | I | . | . | . | . | D | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | . | . | . | . | . | . | . | . | . | . | . | . | . | V | . | . | . | . | . | . | . | . |
| 21-225_161D11_LC | . | . | . | . | . | . | . | . | A | . | . | . | . | . | . | G | . | V | . | R | . | . | . | . | . | . |
| 21-225_161F7_LC | . | . | . | . | . | . | . | . | A | . | . | . | V | . | . | G | . | V | . | R | . | . | . | . | . | . |
| 21-225_162F2_LC | . | . | . | . | . | . | . | . | . | . | . | . | . | . | . | . | . | V | . | . | . | . | . | . | . | . |
| 21-225_162H3_LC | . | . | . | . | . | . | . | . | . | . | . | . | . | . | . | . | . | . | . | . | . | . | . | . | . | . |
| 21-225_163E2_LC | . | . | . | . | . | . | . | . | . | . | . | . | . | S | . | . | . | . | . | . | . | . | . | . | . | . |
| 21-225_163F9_LC | . | . | . | . | . | . | . | . | . | . | . | . | . | . | . | . | . | . | . | . | . | . | . | . | A | . |
| 21-225_163G10_LC | . | . | . | . | . | . | . | . | . | . | . | . | . | . | . | . | . | . | . | . | . | . | . | . | . | . |
| 21-225_16A11_LC | . | . | . | . | . | . | . | . | . | . | . | . | . | . | . | . | . | . | . | . | . | . | . | . | . | . |
| 21-225_16H1_LC | . | . | . | . | . | . | . | . | . | . | . | . | . | . | . | . | . | . | . | . | . | . | . | . | . | . |
| 21-225_16H9_LC | . | . | . | . | . | . | . | . | . | . | . | . | . | . | . | . | . | . | . | . | . | . | . | . | . | . |
| 21-225_171A11_LC | . | . | . | . | . | . | . | . | . | . | . | . | . | . | . | . | . | . | . | . | . | . | . | . | . | . |
| 21-225_177B6_LC | . | . | . | . | . | . | . | . | . | . | . | . | . | . | . | N | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | S | . |
| 21-225_181A8_LC | . | . | . | . | . | . | . | . | . | D | . | . | . | . | . | . | . | . | . | . | . | . | . | . | S | . |
| 21-225_184D11_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR2 | | | | | | | | | | | | | | | |
| CONSENSUS | S | G | S | G | S | G | - | - | T | E | F | T | L | T | I | S | S | L | Q | P | E | D | F | A | T | Y |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | . | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | . | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | . | . | . | . | . | . | . | . | . | . | . | . | . | . | . | . | . | . | . | . | . | . |
| 21-225_203B9_LC | . | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | . | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | A | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | . | . | . | . | . | . | . | . | . | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | . | . | . | . | . | . | . | . | . | . | . | V | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_29D9_LC | . | . | . | . | . | . | . | . | . | . | . | . | . | . | . | . | . | . | . | . | . | . | . | . | . | . |
| 21-225_30H11_LC | . | . | . | . | . | . | . | . | . | . | . | . | . | . | . | . | . | . | . | . | . | . | . | . | . | . |
| 21-225_31G9_LC | . | . | . | . | . | . | . | . | . | . | . | . | . | . | . | . | . | . | . | . | . | . | . | . | . | . |
| 21-225_43B1_LC | . | . | . | . | . | . | . | . | . | . | . | . | . | . | V | . | . | . | . | . | . | . | . | . | . | . |
| 21-225_43E1_LC | . | . | . | . | . | . | . | . | . | . | . | . | . | . | . | . | . | . | . | . | . | . | . | . | . | . |
| 21-225_43E5_LC | . | . | . | . | . | . | . | . | . | . | . | . | . | . | V | . | . | . | . | . | . | . | . | . | . | . |
| 21-225_43H8_LC | . | . | . | . | . | . | . | . | . | . | . | . | . | . | S | . | . | . | . | . | . | . | . | . | . | . |
| 21-225_44C8_LC | . | . | R | . | . | . | . | . | . | . | . | . | . | . | . | . | . | . | . | . | . | . | . | . | . | . |
| 21-225_45H2_LC | . | . | . | . | . | . | . | . | . | . | . | . | . | . | V | . | . | . | . | . | . | . | . | . | . | . |
| 21-225_46E9_LC | . | . | . | . | . | . | . | . | . | . | . | . | . | . | V | . | . | . | . | . | . | . | . | . | . | . |
| 21-225_47H7_LC | . | . | . | . | . | . | . | . | . | . | . | . | . | . | V | . | . | . | . | . | . | . | . | . | . | . |
| 21-225_48E10_LC | . | . | . | . | . | . | . | . | . | . | . | . | . | . | . | . | . | . | . | . | . | . | . | . | . | . |
| 21-225_50C4_LC | . | . | . | . | . | . | . | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . | . | . | . | . | . | . | . |
| 21-225_51E4_LC | . | . | . | . | . | . | . | . | . | . | . | . | . | . | . | . | . | . | . | . | . | . | . | . | A | . |
| 21-225_51F9_LC | . | . | . | . | . | . | . | . | G | . | . | . | . | . | . | . | . | . | . | . | . | . | . | . | S | . |
| 21-225_52B9_LC | . | . | . | . | . | . | . | . | . | . | . | . | . | . | R | . | . | . | . | . | . | . | . | . | . | . |
| 21-225_52H12_LC | . | . | . | . | . | . | . | . | . | . | . | . | . | . | . | . | . | . | . | . | . | . | . | . | . | . |
| 21-225_53B10_LC | . | . | . | . | . | . | . | . | . | . | . | . | . | . | . | . | . | . | . | . | . | . | . | . | . | S |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | . | . | . | . | S | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | R | . | . | . | . | . | . | . | . | . | . | . | . | . | . | . | . | . | . | . | . | . | S | . |
| 21-225_56A5_LC | . | . | . | . | . | . | . | . | . | . | . | . | . | . | . | . | . | . | . | . | . | . | . | . | . | . |
| 21-225_56H7_LC | . | . | . | . | . | . | . | . | . | . | . | . | . | . | . | . | . | . | . | . | . | . | . | . | . | . |
| 21-225_57A4_LC | . | . | . | . | . | . | . | . | . | . | . | . | . | . | . | . | . | . | . | . | . | . | . | . | . | . |
| 21-225_57B2_LC | . | . | . | . | . | . | . | . | . | . | . | . | . | . | . | . | . | . | . | . | . | . | . | . | . | . |
| 21-225_57F4_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_58D11_LC | . | . | . | . | . | . | . | . | . | . | . | . | . | A | . | . | . | . | . | . | . | . | . | . | . | . |
| 21-225_58F5_LC | G | . | . | . | . | . | . | . | . | . | . | . | . | . | . | . | . | . | . | . | . | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | V | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | . | . | . | . | . | . | . | . | . | . | . | . | . | . | . | . | . | . | . | . | . | . | . | . |
| 21-225_64A12_LC | . | . | . | . | . | . | . | . | . | . | . | . | . | . | . | . | . | . | . | . | . | . | . | . | . | . |
| 21-225_66F9_LC | . | . | . | . | . | . | . | . | . | . | . | . | . | . | . | . | . | . | . | . | . | . | . | . | . | . |
| 21-225_67B7_LC | . | . | . | . | . | . | . | . | . | . | S | . | . | . | . | . | . | . | . | . | . | . | . | . | . | . |
| 21-225_68G8_LC | . | . | . | . | . | . | . | . | . | . | . | . | . | . | . | . | . | . | . | . | . | . | . | . | . | . |
| 21-225_71B11_LC | . | . | G | . | . | . | . | . | . | . | S | . | . | . | . | . | . | . | . | . | . | . | . | Y | . | . |
| 21-225_71B6_LC | . | . | . | . | . | . | . | . | . | . | . | . | . | . | . | . | . | . | . | D | . | . | . | . | . | . |
| 21-225_71H6_LC | . | . | . | . | . | . | . | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . | . | . | . | . | . | . | . |
| 21-225_74A11_LC | . | . | . | . | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . | . | . | . | . | . | . | . |
| 21-225_76H10_LC | . | . | . | . | . | . | . | . | . | . | . | . | . | . | . | . | . | . | . | . | . | . | . | . | . | S |
| 21-225_76H12_LC | . | . | . | . | . | . | . | . | . | . | . | . | . | . | . | . | . | . | . | . | . | . | . | . | . | . |
| 21-225_84H12_LC | . | . | . | . | . | . | . | . | . | . | . | . | . | . | . | . | . | . | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | . | . | . | . | . | . | . | . | . | . | . | . | . | . | . | S | . | . | . | . | . | . | . |

4516

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_14D6_LC | . | . | . | . | Y | . | . | . | . | . | . | . | . | . | . | . | . | . | . | . | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | I | . | Q | . | . | . | . | . | . | . | . | . | . | . | . | . | . | . | . | . | . | . | . | . |
| 21-225_157H12_LC | . | . | . | . | . | Y | . | . | . | . | . | . | . | . | . | . | . | . | . | . | . | . | . | . | . | . |
| 21-225_157H4_LC | . | . | . | . | Q | . | . | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | Q | . | . | . | . | . | . | . | . | . | . | . | . | . | . | . | . | . | . | . | . | . |
| 21-225_158G8_LC | . | . | . | . | . | . | . | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | I | . | Q | . | . | . | . | . | . | . | . | . | . | . | . | . | . | . | . | . | . | . | . | . |
| 21-225_161D11_LC | . | Q | . | . | Y | . | R | . | . | . | . | . | . | . | . | . | . | . | . | . | . | . | . | . | . | . |
| 21-225_161F7_LC | . | Q | . | L | Y | I | R | . | . | . | . | . | . | . | . | . | . | . | . | . | . | . | . | . | . | . |
| 21-225_162F2_LC | . | . | I | . | Q | . | . | . | . | . | . | . | . | . | . | . | . | . | . | . | . | . | . | . | . | . |
| 21-225_162H3_LC | . | . | . | . | Q | . | . | . | . | . | . | . | . | . | . | . | . | . | . | . | . | . | . | . | . | . |
| 21-225_163E2_LC | . | . | . | . | . | . | . | . | . | . | . | . | . | . | . | . | . | . | . | . | . | . | . | . | . | . |
| 21-225_163F9_LC | . | . | . | . | Q | . | . | . | . | . | . | . | . | . | . | . | . | . | . | . | . | . | . | . | . | . |
| 21-225_163G10_LC | . | . | . | . | Q | Y | . | . | . | . | . | . | . | . | . | . | . | . | . | . | . | . | . | . | . | . |
| 21-225_16A11_LC | . | . | . | . | Y | . | . | . | . | . | . | . | . | . | . | . | . | . | . | . | . | . | . | . | . | . |
| 21-225_16H1_LC | . | . | . | . | Y | R | . | . | . | . | . | . | . | . | . | . | . | . | . | . | . | . | . | . | . | . |
| 21-225_16H9_LC | . | . | . | . | Y | R | . | . | . | . | . | . | . | . | . | . | . | . | . | . | . | . | . | . | . | . |
| 21-225_171A11_LC | . | . | . | . | Q | R | . | . | . | . | . | . | . | . | . | . | . | . | . | . | . | . | . | . | . | . |
| 21-225_177B6_LC | . | . | . | . | L | . | . | . | . | . | . | . | . | . | . | . | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | . | . |
| 21-225_181A8_LC | . | . | . | . | . | . | . | . | . | . | . | . | . | . | . | . | . | . | . | . | . | . | . | . | . | . |
| 21-225_184D11_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | Y | . | . | . | . | . | . | . | . | . | . | . | . | . | . | . | . | . | . | . | . |
| 21-225_192F6_LC | . | . | . | . | . | Y | . | . | . | . | . | . | . | . | . | . | . | . | . | . | . | . | . | . | . | . |
| 21-225_194F10_LC | . | . | . | . | . | Y | . | . | . | . | . | . | . | . | . | . | . | . | . | . | . | . | . | . | . | . |
| 21-225_195B9_LC | C | . | . | . | . | Y | R | . | . | . | . | . | . | . | . | . | . | . | . | . | . | . | . | . | . | . |
| 21-225_197D7_LC | V | . | . | . | . | Y | R | . | . | . | . | . | . | . | . | . | . | . | . | . | . | . | . | . | . | . |
| 21-225_197G10_LC | . | . | . | . | . | Y | R | . | . | . | . | . | . | . | . | . | . | . | . | . | . | . | . | . | . | . |
| 21-225_198B9_LC | V | . | . | . | . | Y | R | . | . | . | . | . | . | . | . | . | . | . | . | . | . | . | . | . | . | . |
| 21-225_198E3_LC | V | . | . | . | . | Y | R | . | . | . | . | . | . | . | . | . | . | . | . | . | . | . | . | . | . | . |
| 21-225_200F6_LC | R | . | . | . | . | Y | R | . | . | . | . | . | . | . | . | . | . | . | . | . | . | . | . | . | . | . |
| 21-225_202A3_LC | . | . | . | . | . | H | D | . | . | . | . | . | . | . | . | . | . | . | . | . | . | . | . | . | . | . |
| 21-225_203B9_LC | H | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | . | . | . | . | . | . | . | . | . | . | . |
| 21-225_225D6_LC | C | . | . | . | . | . | T | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | H | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | F | . | . | . | D | . | . | . | . | . | . | . | . | . | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | R | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_29D9_LC | . | . | . | . | . | . | T | . | . | . | . | . | . | . | . | . | . | . | . | . | . | . | . | . | . | . |
| 21-225_30H11_LC | . | . | . | . | O | . | . | . | . | . | . | . | . | . | . | . | . | . | . | . | . | . | . | . | . | . |
| 21-225_31G9_LC | . | . | . | . | O | . | . | . | . | . | . | . | . | . | . | . | . | . | . | . | . | . | . | . | . | . |
| 21-225_43B1_LC | F | . | . | . | . | T | . | . | . | . | . | . | . | . | . | . | . | . | . | . | . | . | . | . | . | . |
| 21-225_43E1_LC | . | . | . | . | . | . | . | . | . | . | . | . | . | . | . | . | . | . | . | . | . | . | . | . | . | . |
| 21-225_43E5_LC | F | . | . | . | . | T | . | . | . | . | . | . | . | . | . | . | . | . | . | . | . | . | . | . | . | . |
| 21-225_43H8_LC | . | . | . | . | . | . | . | . | . | . | . | . | . | . | . | . | . | . | . | . | . | . | . | . | . | . |
| 21-225_44C8_LC | . | . | . | . | . | . | . | . | . | . | . | . | . | . | . | . | . | . | . | . | . | . | . | . | . | . |
| 21-225_45H2_LC | F | . | . | . | . | T | . | . | . | . | . | . | . | . | . | . | . | . | . | . | . | . | . | . | . | . |
| 21-225_46E9_LC | F | . | . | . | . | T | . | . | . | . | . | . | . | . | . | . | . | . | . | . | . | . | . | . | . | . |
| 21-225_47H7_LC | F | . | . | . | . | T | . | . | . | . | . | . | . | . | . | . | . | . | . | . | . | . | . | . | . | . |
| 21-225_48E10_LC | . | . | . | . | Y | . | . | . | . | . | . | . | . | . | . | . | . | . | . | . | . | . | . | . | . | . |
| 21-225_50C4_LC | . | . | . | . | . | . | R | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . | . | . | . | . | . | . | . |
| 21-225_51E4_LC | . | . | . | . | . | . | . | . | . | . | . | . | . | . | . | . | . | . | . | . | . | . | . | . | . | . |
| 21-225_51F9_LC | . | . | . | . | . | . | . | . | . | . | . | . | . | . | . | . | . | . | . | . | . | . | . | . | . | . |
| 21-225_52B9_LC | . | . | . | . | . | . | . | . | . | . | . | . | . | . | . | . | . | . | . | . | . | . | . | . | . | . |
| 21-225_52H12_LC | . | . | . | . | Y | . | . | . | . | . | . | . | . | . | . | . | . | . | . | . | . | . | . | . | . | . |
| 21-225_53B10_LC | . | . | . | . | Y | . | . | . | . | . | . | . | . | . | . | . | . | . | . | . | . | . | . | . | . | . |
| 21-225_53B12_LC | . | . | . | . | . | . | . | . | . | . | . | . | . | . | . | . | . | . | . | . | . | . | . | . | . | . |
| 21-225_53G1_LC | . | . | . | . | O | . | . | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | F | . | . | . | T | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . | . | . | . | . | . | . | . |
| 21-225_56A5_LC | . | . | . | . | Y | . | . | . | . | . | . | . | . | . | . | . | . | . | . | . | . | . | . | . | . | . |
| 21-225_56H7_LC | . | . | . | . | O | . | . | . | . | . | . | . | . | . | . | . | . | . | . | . | . | . | . | . | . | . |
| 21-225_57A4_LC | . | . | . | . | . | . | . | . | . | . | . | . | . | . | . | . | . | . | . | . | . | . | . | . | . | . |
| 21-225_57B2_LC | . | . | . | . | . | . | . | . | . | . | . | . | . | . | . | . | . | . | . | . | . | . | . | . | . | . |
| 21-225_57F4_LC | F | . | . | . | Y | O | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | K_CDR3 | | | | | | | | | |
| CONSENSUS | Y | C | L | Q | H | N | S | - | - | - | - | - | - | - | - | - | - | - | - | - | - | - | - | - | - | - |
| 21-225_58D11_LC | . | . | . | . | . | . | N | . | . | . | . | . | . | . | . | . | . | . | . | . | . | . | . | . | . | . |
| 21-225_58F5_LC | . | . | . | . | . | . | . | . | . | . | . | . | . | . | . | . | . | . | . | . | . | . | . | . | . | . |
| 21-225_59F11_LC | . | . | . | . | . | . | S | . | . | . | . | . | . | . | . | . | . | . | . | . | . | . | . | . | . | . |
| 21-225_5F4_LC | . | . | . | Y | . | . | G | . | . | . | . | . | . | . | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | . | . | Y | . | . | . | . | . | . | . | . | . | . | . | . | . | . | . | . | . | . | . | . | . |
| 21-225_64A12_LC | . | . | . | . | . | . | G | . | . | . | . | . | . | . | . | . | . | . | . | . | . | . | . | . | . | . |
| 21-225_66F9_LC | . | . | . | . | Y | . | . | . | . | . | . | . | . | . | . | . | . | . | . | . | . | . | . | . | . | . |
| 21-225_67B7_LC | . | . | . | I< | . | . | . | . | . | . | . | . | . | . | . | . | . | . | . | . | . | . | . | . | . | . |
| 21-225_68G8_LC | . | . | . | . | Y | . | . | . | . | . | . | . | . | . | . | . | . | . | . | . | . | . | . | . | . | . |
| 21-225_71B11_LC | . | . | . | . | . | . | T | . | . | . | . | . | . | . | . | . | . | . | . | . | . | . | . | . | . | . |
| 21-225_71B6_LC | . | . | . | . | . | . | . | . | . | . | . | . | . | . | . | . | . | . | . | . | . | . | . | . | . | . |
| 21-225_71H6_LC | . | . | . | . | Y | . | . | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . | . | . | . | . | . | . | . |
| 21-225_74A11_LC | F | . | . | . | . | . | G | . | . | . | . | . | . | . | . | . | . | . | . | . | . | . | . | . | . | . |
| 21-225_75C11_LC | . | . | . | . | . | . | G | . | . | . | . | . | . | . | . | . | . | . | . | . | . | . | . | . | . | . |
| 21-225_76H10_LC | . | . | . | . | Y | . | . | . | . | . | . | . | . | . | . | . | . | . | . | . | . | . | . | . | . | . |
| 21-225_76H12_LC | . | . | . | . | . | . | D | . | . | . | . | . | . | . | . | . | . | . | . | . | . | . | . | . | . | . |
| 21-225_84H12_LC | . | . | . | . | . | . | D | . | . | . | . | . | . | . | . | . | . | . | . | . | . | . | . | . | . | . |
| 21-225_86E5_LC | . | . | . | . | . | . | D | . | . | . | . | . | . | . | . | . | . | . | . | . | . | . | . | . | . | . |
| 21-225_88A5_LC | . | . | . | . | . | . | D | . | . | . | . | . | . | . | . | . | . | . | . | . | . | . | . | . | . | . |
| 21-225_96A4_LC | . | . | . | . | . | . | D | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | F | T | F | G | P | G | T | K | V | D | I | K | R |
| 21-225_14D6_LC | . | . | . | . | | | . | | | | | | | . | . | . | . | . | N |
| 21-225_157C3_LC | . | . | . | . | | | . | | | | | | | . | . | . | . | . | |
| 21-225_157G7_LC | . | . | . | . | ﹔ | | . | . | | | . | . | . | . | . | ﹕ | . | . | |
| 21-225_157H12_LC | . | . | . | . | | | . | | | | | | . | . | . | . | . | . | |
| 21-225_157H4_LC | . | . | . | . | | | . | | | | . | . | . | . | . | . | . | . | |
| 21-225_157H8_LC | . | . | . | . | | | . | | | | | | . | . | . | . | . | . | |
| 21-225_158F5_LC | . | . | . | . | ﹔ | | . | . | | | . | . | | . | . | ﹕ | . | . | |
| 21-225_158G8_LC | . | . | . | . | | | . | | | | | | . | . | . | . | . | . | |
| 21-225_158H6_LC | . | . | . | . | | | . | | | | | | . | . | . | . | . | . | |
| 21-225_159C5_LC | . | . | . | . | | | . | | | | | | . | . | . | . | . | . | |
| 21-225_15B11_LC | . | . | . | . | | | . | | | | | | . | . | . | . | . | . | |
| 21-225_160F4_LC | . | . | . | . | ﹔ | | . | | | | | | . | . | . | ﹕ | . | . | |
| 21-225_161D11_LC | . | . | . | . | | | . | | | | ﹔ | | . | . | . | . | . | . | |
| 21-225_161F7_LC | . | . | . | . | | . | . | . | | | ﹔ | | . | . | . | . | . | . | |
| 21-225_162F2_LC | . | . | . | ﹔ | | . | . | | | | | | . | . | . | ﹕ | . | . | |
| 21-225_162H3_LC | . | . | . | ﹔ | | . | . | | | | | | . | . | . | ﹕ | . | . | |
| 21-225_163E2_LC | . | . | . | ﹔ | | . | . | | | | | | . | . | . | . | . | . | |
| 21-225_163F9_LC | . | . | . | . | | . | . | | | | | | . | . | . | . | . | . | |
| 21-225_163G10_LC | . | . | . | . | | | . | | | | | | . | . | . | . | . | . | |
| 21-225_16A11_LC | . | . | . | . | | | . | | | | | | . | . | . | . | . | . | |
| 21-225_16H1_LC | . | . | . | . | | | . | | | | | | . | . | . | . | V | . | |
| 21-225_16H9_LC | . | . | . | . | ﹔ | | . | | | | | | . | . | . | . | . | . | |
| 21-225_171A11_LC | . | . | . | . | | . | . | | | | | | . | . | . | . | . | . | |
| 21-225_177B6_LC | . | . | . | . | | . | . | | | | | | . | . | . | . | . | . | |
| 21-225_17E5_LC | . | . | . | . | ﹔ | . | . | | | | | | . | . | . | V | . | . | |
| 21-225_17F4_LC | . | . | . | . | | | . | | | | | | . | . | V | . | . | . | |
| 21-225_180H7_LC | . | . | . | . | | . | . | | | | | | . | . | . | . | F | . | |
| 21-225_181A8_LC | . | . | . | . | | | . | | | | | | . | . | . | . | F | . | |
| 21-225_184D11_LC | . | . | . | . | | . | . | | | | | | . | . | . | . | . | . | |

**4521**

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | F | T | F | G | P | G | T | K | V | D | I | K | R |
| 21-225_184H10_LC | | | | | | | | | | | | | | | | | | | |
| 21-225_190D6_LC | | | | | | | | | | | | | | | | | | | |
| 21-225_192F6_LC | | | | | | | | | | | | | | | | | | | |
| 21-225_194F10_LC | | | | | | | | | | | | | | | | | | | |
| 21-225_195B9_LC | | | | | | | | | | | | | | | | | | | |
| 21-225_197D7_LC | | | | | | | | | | | | | | | | | | | |
| 21-225_197G10_LC | | | | | | | | | | | | | | | | | | | |
| 21-225_198B9_LC | | | | | | | | | | | | | | | | | | | |
| 21-225_198E3_LC | | | | | | | | | | | | | | | | | | | |
| 21-225_200F6_LC | | | | | | | | | | | | | | | | | | | |
| 21-225_202A3_LC | | | | | | | | | | | | | | | | | | | |
| 21-225_203B9_LC | | | | | | | | | | | | | | | | | | | |
| 21-225_210E4_LC | | | | | | | | | | | | | | | | | | | |
| 21-225_225B6_LC | | | | | | | | | | | | | | | | | | | |
| 21-225_225D6_LC | | | | | | | | | | | | | | | | | | | |
| 21-225_225F8_LC | | | | | | | | | | | | | | | | | | | |
| 21-225_226A9_LC | | | | | | | | | | | | | | | | | | | |
| 21-225_227C12_LC | | | | | | | | | | | | | | | | | | | |
| 21-225_227G3_LC | | | | | | | | | | | | | | | | | | | |
| 21-225_227H5_LC | | | | | | | | | | | | | | | | | | | |
| 21-225_22H3_LC | | | | | | | | | | | | | | | | | | | |
| 21-225_25A9_LC | | | | | | | | | | | | | | | | | | | |
| 21-225_25B6_LC | | | | | | | | | | | | | | | | | | | |
| 21-225_25G5_LC | | | | | | | | | | | | | | | | | | | |
| 21-225_27D5_LC | | | | | | | | | | | | | | | | | | | |
| 21-225_28A9_LC | | | | | | | | | | | | | | | | | | | |
| 21-225_28B8_LC | | | | | | | | | | | | | | | | | | N | |
| 21-225_29D7_LC | | | | | | | | | | | | | | | | | | | |
| 21-225_29D8_LC | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | F | T | F | G | P | G | T | K | V | D | I | K | R |
| 21-225_29D9_LC | . | . | . | . | . | . | . | . | . | . | . | . | . | . | . | . | . | . | . |
| 21-225_30H11_LC | . | . | . | . | . | . | . | . | . | . | . | . | . | . | . | . | . | T | . |
| 21-225_31G9_LC | . | . | . | . | . | . | . | . | . | . | . | . | . | . | . | . | . | . | . |
| 21-225_43B1_LC | . | . | . | . | . | . | . | . | . | . | . | . | . | . | . | . | . | . | . |
| 21-225_43E1_LC | . | . | . | . | . | . | . | . | . | . | . | . | . | . | . | . | . | . | . |
| 21-225_43E5_LC | . | . | . | . | . | . | . | . | . | . | . | . | . | . | . | . | . | . | . |
| 21-225_43H8_LC | . | . | . | . | . | . | . | . | . | . | . | . | . | . | . | . | . | . | . |
| 21-225_44C8_LC | . | . | . | . | . | . | . | . | . | . | . | . | . | . | . | . | . | . | . |
| 21-225_45H2_LC | . | . | . | . | . | . | . | . | . | . | . | . | . | . | . | . | . | . | . |
| 21-225_46E9_LC | . | . | . | . | . | . | . | . | . | . | . | . | . | . | . | . | . | . | . |
| 21-225_47H7_LC | . | . | . | . | . | . | . | . | . | . | . | . | . | . | . | . | . | . | . |
| 21-225_48E10_LC | . | . | . | . | . | . | . | . | . | . | . | . | . | . | . | . | . | . | . |
| 21-225_50C4_LC | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . |
| 21-225_51E4_LC | . | . | . | . | . | . | . | . | . | . | . | . | . | . | . | . | . | . | . |
| 21-225_51F9_LC | . | . | . | . | . | . | . | . | . | . | . | . | . | . | . | . | . | . | . |
| 21-225_52B9_LC | . | . | . | . | . | . | . | . | . | . | . | . | . | . | . | . | . | . | . |
| 21-225_52H12_LC | . | . | . | . | . | . | . | . | . | . | . | . | . | . | . | . | . | . | . |
| 21-225_53B10_LC | . | . | . | . | . | . | . | . | . | . | . | . | . | . | . | . | . | . | . |
| 21-225_53B12_LC | . | . | . | . | . | . | . | . | . | . | . | . | . | . | . | . | . | . | . |
| 21-225_53G1_LC | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . |
| 21-225_56A5_LC | . | . | . | . | . | . | . | . | . | . | . | . | . | . | . | . | . | . | . |
| 21-225_56H7_LC | . | . | . | . | . | . | . | . | . | . | . | . | . | . | . | S | . | . | . |
| 21-225_57A4_LC | . | . | . | . | . | . | . | . | . | . | . | . | . | . | . | . | . | . | . |
| 21-225_57B2_LC | . | . | . | . | . | . | . | . | . | . | . | . | . | . | . | . | . | . | . |
| 21-225_57F4_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | F | T | F | G | P | G | T | K | V | D | I | K | R |
| 21-225_58D11_LC | | | | | | | | | | | | | | | | | | | |
| 21-225_58F5_LC | | | | | | | | | | | | | | | | | | | |
| 21-225_59F11_LC | | | | | | | | | | | | | | | | | | | |
| 21-225_5F4_LC | | | | | | | | | | | | | | | | | | | |
| 21-225_60A3_LC | | | | | | | | | | | | | | | | | | | |
| 21-225_62G3_LC | | | | | | | | | | | | | | | | | | | |
| 21-225_64A12_LC | | | | | | | | | | | | | | | | | | | |
| 21-225_66F9_LC | | | | | | | | | | | | | | | | | | | |
| 21-225_67B7_LC | | | | | | | | | | | | | | | | | | | |
| 21-225_68G8_LC | | | | | | | | | | | | | | | | | | | |
| 21-225_71B11_LC | | | | | | | | | | | | | | | | | | | |
| 21-225_71B6_LC | | | | | | | | | | | | | | | | | | | |
| 21-225_71H6_LC | | | | | | | | | | | | | | | | | | | |
| 21-225_73A6_LC | | | | | | | | | | | | | | | | | | | |
| 21-225_74A11_LC | | | | | | | | | | | | | | | | | | | |
| 21-225_75C11_LC | | | | | | | | | | | | | | | | | | | |
| 21-225_76H10_LC | | | | | | | | | | | | | | | | | | | |
| 21-225_76H12_LC | | | | | | | | | | | | | | | | | | | |
| 21-225_84H12_LC | | | | | | | | | | | | | | | | | | | |
| 21-225_86E5_LC | | | | | | | | | | | | | | | | | | | |
| 21-225_88A5_LC | | | | | | | | | | | | | | | | | | | |
| 21-225_96A4_LC | | | | | | | | | | | | | | | | | | | |

**FIGURE 57**

Table 101.     Consensus 21 - VK1|L1/JK4 (SEQ ID NO: 50318):

DIQMTQSPSSLSASVGDRVTITCRAS--QGIS------NYLAWFQQKPGKAPKSLIYA--------ASSLQSGVPSKFSGSGSG--
TDFTLTISSLQPEDFATYYCQQYST---------------------YPLTFGGGTKVEIKR wherein:

A at position 25 can be substituted with T

S at position 26 can be substituted with N

G at position 30 can be substituted with D, A, V or S

S at position 32 can be substituted with G, N, R, A or F

N at position 39 can be substituted with K, R, H, S, T or I

Y at position 40 can be substituted with H, C or D

A at position 42 can be substituted with D, V, I or N

A at position 58 can be substituted with K, S, T, V, D or G

A at position 67 an be substituted with T or V

S at position 68 can be substituted with P

S at position 69 can be substituted with N or R

Q at position 71 can be substituted with L, E or H

S at position 72 can be substituted with G, N or T

Q at position 107 can be substituted with L or H

Q at position 108 can be substituted with H, R or Y

Y at position 109 can be substituted with S, C or T

S at position 110 can be substituted with L, N, M, D, H, V, I or Y

FIGURE 57

T at position 111 can be substituted with N, S or K

Y at position 135 can be substituted with F, I or S

L at position 137 can be substituted with V, F or N

T at position 138 can be substituted with I, Q or S

Arg Xaa Xaa Gln Xaa Ile Xaa Xaa Xaa Leu Xaa (SEQ ID NO: 50138)

Xaa Xaa Xaa Xaa Leu Xaa Xaa (SEQ ID NO: 50139)

Xaa Xaa Xaa Xaa Xaa Xaa Pro Xaa Xaa (SEQ ID NO: 50140)

Arg Ala Ser Gln Gly Ile Ser Asn Tyr Leu Ala (SEQ ID NO: 50624)

Ala Ala Ser Ser Leu Gln Ser (SEQ ID NO: 50625)

Gln Gln Tyr Ser Thr Tyr Pro Leu Thr (SEQ ID NO: 50626)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_147E9_LC | . | . | . | . | . | . | . | . | . | . | . | . | . | . | . | . | . | . | . | . | . | . | . | . | . | . |
| 21-225_150G8_LC | . | . | . | . | . | . | . | . | . | . | . | . | . | . | . | . | . | . | . | . | . | . | . | . | . | . |
| 21-225_153F9_LC | . | . | . | . | . | . | . | . | . | . | . | . | . | . | . | . | . | . | . | . | . | . | . | . | . | . |
| 21-225_157C1_LC | . | . | . | . | . | . | . | . | . | . | . | . | . | . | § | . | . | . | § | . | L | . | . | . | . | . |
| 21-225_160H4_LC | . | . | . | . | . | . | . | . | . | . | . | . | . | . | . | . | . | . | . | . | . | . | . | . | . | . |
| 21-225_163G6_LC | . | . | . | . | . | . | . | . | . | . | . | . | . | . | . | . | . | . | . | . | . | . | . | . | . | . |
| 21-225_166H12_LC | . | . | . | . | . | . | . | . | . | . | . | . | T | . | . | . | . | . | . | . | . | . | . | . | . | . |
| 21-225_169C10_LC | . | . | . | . | . | . | . | . | . | . | . | . | . | . | . | . | . | . | . | . | . | . | . | . | . | . |
| 21-225_16A12_LC | . | . | . | . | . | . | . | . | . | . | . | . | . | . | . | . | . | . | . | . | . | . | . | . | . | . |
| 21-225_16D10_LC | . | . | . | . | . | . | . | . | . | . | . | . | . | . | . | . | . | . | . | . | . | . | . | . | . | . |
| 21-225_170D1_LC | . | . | . | . | . | . | . | . | . | . | . | A | . | . | . | . | . | . | . | . | . | . | . | . | . | . |
| 21-225_170D6_LC | . | . | . | . | . | . | . | . | . | . | . | . | . | . | . | . | . | . | . | S | . | . | . | . | . | . |
| 21-225_171H12_LC | . | . | . | . | . | . | . | . | . | . | . | . | . | . | . | . | . | . | . | . | . | . | . | . | . | . |
| 21-225_175G3_LC | . | . | . | . | . | . | . | . | . | . | . | . | . | . | . | . | . | . | . | . | . | . | . | . | . | . |
| 21-225_177A5_LC | . | . | . | . | . | . | . | . | . | . | . | . | . | . | . | . | . | . | . | . | . | . | . | . | . | . |
| 21-225_178H4_LC | . | . | . | . | . | . | . | . | . | . | . | . | . | . | . | . | . | . | . | . | . | . | . | . | . | . |
| 21-225_190C11_LC | . | . | . | . | . | . | . | . | . | . | . | . | . | . | . | . | . | . | . | . | . | . | . | . | T | . |
| 21-225_190E10_LC | . | . | . | . | A | . | . | . | . | . | . | . | . | . | . | . | . | . | . | . | . | . | . | . | T | . |
| 21-225_190E6_LC | . | . | . | . | . | . | . | . | . | . | . | . | . | . | . | . | . | . | . | . | . | . | . | . | T | . |
| 21-225_190F8_LC | . | . | . | . | . | . | . | . | . | . | . | . | . | . | . | . | . | . | . | . | . | . | . | . | T | . |
| 21-225_190G11_LC | . | . | . | . | . | . | . | . | . | . | . | . | . | . | . | . | . | . | . | . | . | . | . | . | T | . |
| 21-225_190G4_LC | . | . | . | . | . | . | . | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . | . | . | . | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | . | . | . | . | . | . | . | . | . | . | . | . | . | . | . | . | . | . | . | . | . | T | . |
| 21-225_191G12_LC | . | . | . | . | . | . | . | . | . | . | . | . | . | . | . | . | . | . | . | . | . | . | . | . | T | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | § | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_192D3_LC | . | . | . | . | . | . | . | . | . | . | . | . | . | . | . | . | . | . | . | . | . | . | . | . | . | . |
| 21-225_192G6_LC | . | . | ≈ | . | . | . | . | . | . | . | . | . | . | . | . | . | . | . | . | . | . | . | . | . | T | . |
| 21-225_192G8_LC | . | . | . | . | . | . | . | . | . | . | . | . | . | . | . | . | . | . | . | . | . | . | . | . | T | . |
| 21-225_192H10_LC | . | . | . | . | . | . | . | . | . | . | . | . | . | . | . | . | . | . | . | . | . | . | . | . | . | . |
| 21-225_192H4_LC | . | . | . | . | . | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . | . | . | . | . | . | . | N |
| 21-225_197B6_LC | . | . | . | . | . | . | . | . | . | . | . | . | . | . | . | . | . | . | . | . | . | . | . | . | . | . |
| 21-225_197D3_LC | . | . | . | . | . | . | . | . | . | . | . | . | . | . | . | . | . | . | . | . | . | . | . | . | . | . |
| 21-225_197F2_LC | . | . | . | . | { | . | . | . | . | . | . | . | . | . | . | . | . | . | . | . | . | . | . | . | T | . |
| 21-225_197G12_LC | . | . | . | . | { | . | . | . | . | . | . | . | . | . | . | . | . | . | . | . | . | . | . | . | T | . |
| 21-225_198B6_LC | . | . | . | . | . | . | . | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . | . | . | . | . | . | . | . |
| 21-225_1F1_LC | . | . | . | . | . | . | . | . | . | . | . | . | . | . | . | . | . | . | . | . | . | . | . | . | . | . |
| 21-225_203H1_LC | . | . | . | . | . | . | . | . | . | . | . | . | . | . | . | . | . | . | . | . | . | . | . | . | T | . |
| 21-225_204G6_LC | . | . | ≈ | . | . | . | . | . | . | . | . | . | . | . | . | . | . | . | { | . | . | . | . | . | T | . |
| 21-225_204G8_LC | . | . | . | . | . | . | . | . | . | . | . | . | . | . | . | . | . | . | . | . | . | . | . | . | . | . |
| 21-225_205F5_LC | . | . | . | . | . | . | . | . | . | . | . | . | . | . | . | . | . | . | . | . | . | . | . | . | . | . |
| 21-225_205H3_LC | . | . | P | . | . | . | . | . | . | . | . | . | . | . | . | . | . | . | . | . | . | . | . | . | . | . |
| 21-225_207G6_LC | . | . | . | . | . | . | . | . | . | . | . | . | . | . | . | . | . | . | . | . | . | . | . | . | . | . |
| 21-225_211A11_LC | . | . | . | . | . | . | . | . | . | . | . | . | . | . | . | . | . | . | . | . | . | . | . | . | . | . |
| 21-225_211A3_LC | . | . | ≈ | . | . | . | . | . | . | . | . | . | . | . | . | . | . | . | . | . | . | . | . | . | . | . |
| 21-225_211A8_LC | . | . | E | . | . | . | . | P | . | . | . | . | . | P | . | . | . | . | . | . | . | . | . | . | . | . |
| 21-225_212D7_LC | . | . | . | . | . | . | . | . | . | . | . | . | . | . | . | . | . | . | . | . | . | . | . | . | . | . |
| 21-225_212E10_LC | . | . | . | . | . | . | . | . | . | . | . | . | . | . | . | . | . | . | . | . | . | . | . | . | . | . |

4528

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_212E6_LC | . | . | . | . | . | . | . | . | . | . | . | . | . | . | . | . | . | . | . | . | . | . | . | . | . | . |
| 21-225_212F10_LC | . | . | . | . | . | . | . | . | . | . | . | . | . | . | . | . | . | . | . | . | . | . | . | . | . | . |
| 21-225_212H11_LC | . | . | ≈ | . | . | . | . | . | . | . | . | . | . | . | . | . | . | . | . | . | . | . | . | . | . | . |
| 21-225_212H9_LC | . | . | E | . | . | . | . | . | . | . | . | . | . | . | . | . | . | . | . | . | . | . | . | . | . | . |
| 21-225_213B8_LC | . | . | . | . | . | . | . | . | . | . | . | . | . | . | . | . | . | . | . | . | . | . | . | . | . | . |
| 21-225_213C4_LC | . | . | H | . | . | . | . | . | . | . | . | . | . | . | . | . | . | . | . | . | . | . | . | . | . | . |
| 21-225_213D2_LC | . | . | . | . | . | . | . | . | . | . | . | . | . | . | . | . | . | . | . | . | . | . | . | . | . | . |
| 21-225_213E6_LC | . | . | ≈ | . | . | . | . | . | . | . | . | . | . | . | . | . | . | . | . | . | . | . | . | . | . | . |
| 21-225_215A12_LC | . | . | . | . | . | . | . | . | . | . | . | . | . | . | . | . | . | . | ↓ | . | . | . | . | . | T | . |
| 21-225_215B5_LC | . | . | . | . | . | . | . | . | . | . | . | . | . | . | . | . | . | . | . | . | . | . | . | . | . | . |
| 21-225_215D6_LC | . | . | . | . | . | . | . | . | . | . | . | . | . | . | . | . | . | . | . | . | . | . | . | . | . | . |
| 21-225_216G1_LC | . | . | E | . | . | . | . | . | . | . | . | . | . | . | . | . | . | . | . | . | . | . | . | . | . | . |
| 21-225_217B10_LC | . | . | R | . | . | . | . | . | . | . | . | . | . | . | . | . | . | . | . | . | . | . | . | . | . | . |
| 21-225_217G5_LC | . | . | L | . | . | . | . | . | . | . | . | . | . | . | . | . | . | . | . | . | . | . | . | . | . | . |
| 21-225_219H1_LC | . | . | . | . | . | . | . | . | . | Q | . | . | . | . | . | . | . | . | . | . | . | . | . | . | . | . |
| 21-225_221F6_LC | . | . | . | . | . | . | . | . | . | . | . | O | . | . | . | . | . | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | . | . | . | . | . | . | . | . | . | . |
| 21-225_22A2_LC | . | . | . | . | . | . | . | . | . | . | . | . | . | . | . | . | . | . | . | . | . | . | . | . | . | . |
| 21-225_23D11_LC | . | . | . | . | . | . | . | . | . | . | . | . | . | . | . | . | . | . | . | . | . | . | . | . | . | . |
| 21-225_32G3_LC | . | . | . | . | . | . | . | T | . | . | . | . | . | . | . | . | . | . | . | . | . | . | . | . | . | . |
| 21-225_55A11_LC | . | . | . | . | . | . | . | . | . | . | . | . | . | . | . | . | . | . | . | . | . | . | . | . | . | . |
| 21-225_55D4_LC | . | . | . | . | . | . | . | . | . | . | S | . | . | . | . | . | . | . | . | . | . | . | . | . | . | . |
| 21-225_65H11_LC | . | . | . | . | . | . | . | . | . | . | . | . | . | . | . | . | . | . | . | . | . | . | . | . | . | . |
| 21-225_70H6_LC | . | . | . | . | . | . | . | . | . | . | . | . | . | . | . | L | . | . | . | . | . | . | . | . | T | . |
| 21-225_74C8_LC | . | . | . | . | . | . | . | . | . | . | . | . | . | . | . | . | . | . | . | . | . | . | . | . | . | . |
| 21-225_74G6_LC | . | . | . | . | . | . | . | . | . | . | . | . | . | . | . | . | . | . | . | . | . | . | . | . | . | . |
| 21-225_76C5_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | K_FR1 | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_77E6_LC | . | . | . | . | . | . | . | . | . | . | . | . | . | . | . | . | . | . | . | . | . | . | . | . | . | . |
| 21-225_79A12_LC | . | . | . | . | . | . | . | . | . | . | . | . | . | . | . | . | . | . | . | . | . | . | . | . | . | . |
| 21-225_79F4_LC | . | . | . | . | . | . | . | . | . | . | . | . | . | . | . | . | . | . | . | . | . | . | . | . | . | . |
| 21-225_7F10_LC | . | . | . | . | . | . | . | . | A | . | . | . | . | . | . | . | . | . | . | . | . | . | . | . | . | . |
| 21-225_80C11_LC | . | . | . | . | . | . | . | . | . | . | . | . | . | . | . | . | . | . | . | . | . | . | . | . | . | . |

4530

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_147E9_LC | . | . | . | D | . | N | . | . | . | . | . | . | . | . | . | . | . | . | . | . | . | . | . | . | . | . |
| 21-225_150G8_LC | . | . | . | . | . | . | . | . | . | . | . | . | N | . | . | . | . | . | . | . | . | . | . | . | . | . |
| 21-225_153F9_LC | . | . | . | . | . | . | . | . | . | . | . | . | . | . | . | . | . | . | . | . | . | . | . | . | . | . |
| 21-225_157C1_LC | . | . | . | . | . | . | . | . | . | . | . | . | . | . | . | . | . | . | . | . | R | . | . | . | . | . |
| 21-225_160H4_LC | . | . | . | D | . | . | . | . | . | . | . | . | . | . | . | V | . | . | . | . | L | . | . | . | . | . |
| 21-225_163G6_LC | . | . | . | D | . | . | . | . | . | . | . | . | . | . | . | V | . | . | . | . | . | . | . | . | . | . |
| 21-225_166H12_LC | . | . | . | D | . | . | . | . | . | . | . | . | N | . | . | . | . | . | . | . | . | . | . | . | . | . |
| 21-225_169C10_LC | . | . | . | D | . | . | . | . | . | . | . | . | . | . | . | . | . | . | . | . | . | . | . | . | . | . |
| 21-225_16A12_LC | . | . | . | A | . | N | . | . | . | . | . | . | T | . | . | . | . | . | . | . | . | . | . | . | . | . |
| 21-225_16D10_LC | . | . | . | D | . | . | . | . | . | . | . | . | . | . | . | . | . | . | . | . | . | . | . | . | . | . |
| 21-225_170D1_LC | . | . | . | D | . | . | . | . | . | . | . | . | . | . | . | . | . | . | . | . | . | . | . | . | . | . |
| 21-225_170D6_LC | . | . | . | D | . | G | . | . | . | . | . | . | . | Q | . | . | . | . | . | . | . | . | . | . | . | . |
| 21-225_171H12_LC | . | . | . | D | . | . | . | . | . | . | . | . | . | . | . | . | . | . | . | . | . | . | . | . | . | . |
| 21-225_175G3_LC | . | . | . | D | . | . | . | . | . | . | . | . | . | D | . | . | . | . | . | . | . | . | . | . | . | . |
| 21-225_177A5_LC | . | . | . | D | . | . | . | . | . | . | . | . | . | . | . | . | . | . | . | . | . | . | . | . | . | . |
| 21-225_178H4_LC | . | . | . | D | . | . | . | . | . | . | . | . | . | . | . | . | . | . | . | . | . | . | . | . | . | . |
| 21-225_190C11_LC | . | . | . | . | . | G | . | . | . | . | . | . | . | . | . | . | . | . | . | . | . | . | . | . | . | . |
| 21-225_190E10_LC | . | . | . | . | . | G | . | . | . | . | . | . | K | . | . | . | . | . | . | . | . | . | . | . | . | . |
| 21-225_190E6_LC | . | . | . | . | . | G | . | . | . | . | . | . | . | . | . | . | . | . | . | . | . | . | . | . | . | . |
| 21-225_190F8_LC | . | . | . | . | . | G | . | . | . | . | . | . | . | . | . | . | . | . | . | . | . | . | . | . | . | . |
| 21-225_190G11_LC | . | . | . | . | . | G | . | . | . | . | . | . | . | . | . | . | . | . | . | . | . | . | . | . | . | . |
| 21-225_190G4_LC | . | . | . | D | . | G | . | . | . | . | . | . | K | . | . | . | . | . | . | . | . | . | . | . | . | . |
| 21-225_191A10_LC | . | . | . | . | . | G | . | . | . | . | . | . | K | . | . | . | . | . | . | . | . | . | . | . | . | . |
| 21-225_191E10_LC | . | . | . | . | . | G | . | . | . | . | . | . | K | . | . | . | . | . | . | . | . | . | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | . | . | G | . | . | . | . | . | . | K | . | . | . | . | . | . | . | . | . | . | . | . | . |
| 21-225_191G12_LC | . | . | . | . | . | G | . | . | . | . | . | . | . | . | . | . | . | . | . | . | . | . | . | . | . | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_192D3_LC | . | . | . | . | . | . | . | . | . | . | . | . | . | . | . | . | . | . | . | . | . | . | . | . | . | . |
| 21-225_192G6_LC | . | . | . | . | . | G | . | . | . | . | . | . | . | . | . | . | . | . | . | . | . | . | . | . | . | . |
| 21-225_192G8_LC | . | . | . | . | . | G | . | . | . | . | . | . | . | . | . | . | . | . | . | . | . | . | . | . | . | . |
| 21-225_192H10_LC | . | . | . | . | . | G | . | . | . | . | . | . | K | . | . | . | . | . | . | . | . | . | . | . | . | . |
| 21-225_192H4_LC | . | . | . | O | . | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | G | . | . | . | . | . | . | K | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | A | . | . | . | . | . | . | . | . | . | . | . | . | . | . | . | . | . | . | . | . | . | . |
| 21-225_197B6_LC | . | . | . | . | . | G | . | . | . | . | . | . | K | . | . | . | . | . | . | . | . | . | . | . | . | . |
| 21-225_197D3_LC | . | . | . | . | . | R | . | . | . | . | . | . | . | . | . | . | . | . | . | . | . | . | . | . | . | . |
| 21-225_197F2_LC | . | . | . | . | . | G | . | . | . | . | . | . | . | . | . | . | . | . | . | . | . | . | . | . | . | . |
| 21-225_197G12_LC | . | . | . | . | . | G | . | . | . | . | . | . | . | . | . | . | . | . | . | . | . | . | . | . | . | . |
| 21-225_198B6_LC | . | . | . | . | . | G | . | . | . | . | . | . | . | . | . | . | . | . | . | . | . | . | . | . | . | . |
| 21-225_199C5_LC | . | . | . | . | . | . | . | . | . | . | . | . | K | . | . | . | . | . | . | . | . | . | . | . | . | . |
| 21-225_1F1_LC | . | . | . | . | . | N | . | . | . | . | . | . | . | . | . | . | . | . | . | . | . | . | S | . | . | . |
| 21-225_203H1_LC | . | . | . | . | . | G | . | . | . | . | . | . | . | . | . | . | . | . | . | . | . | . | . | . | . | . |
| 21-225_204G6_LC | . | . | . | . | . | G | . | . | . | . | . | . | . | . | . | . | . | . | . | . | . | . | . | . | . | . |
| 21-225_204G8_LC | . | . | . | . | . | . | . | . | . | . | . | . | K | . | . | . | . | . | . | . | . | . | . | . | . | . |
| 21-225_205F5_LC | . | . | . | . | . | G | . | . | . | . | . | . | . | . | . | . | . | . | . | . | . | . | . | . | . | . |
| 21-225_205H3_LC | . | . | . | A | . | . | . | . | . | . | . | . | K | . | . | . | . | . | . | . | L | . | . | . | . | . |
| 21-225_207G6_LC | . | . | . | . | . | R | . | . | . | . | . | . | . | . | . | . | . | L | . | . | . | . | . | . | . | . |
| 21-225_211A11_LC | . | . | . | . | . | G | . | . | . | . | . | . | . | . | . | . | . | . | . | . | . | . | . | . | . | . |
| 21-225_211A3_LC | . | . | . | A | . | G | . | . | . | . | . | . | K | H | . | . | . | . | . | . | R | . | . | . | . | . |
| 21-225_211A8_LC | . | . | . | . | . | . | . | . | . | . | . | . | K | . | . | . | . | V | . | . | . | . | . | . | . | . |
| 21-225_212D7_LC | . | . | . | . | . | . | . | . | . | . | . | . | H | . | . | . | . | . | . | . | . | . | . | . | . | . |
| 21-225_212E10_LC | . | . | . | . | . | . | . | . | . | . | . | . | H | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_212E6_LC | | | | | | | | | | | | | | H | | | | | | | | | | Q | | |
| 21-225_212F10_LC | | | | | | | | | | | | | | | | D | | | | | | | | | | |
| 21-225_212H11_LC | | | | A | | G | | | | | | | K | H | | | | | | | R | | | | | |
| 21-225_212H9_LC | | | | | | | | | | | | | S | | | | | L | | | | | | | | |
| 21-225_213B8_LC | | | | | | | | | | | | | | H | | | | | | | | | | | | |
| 21-225_213C4_LC | | | | A | | | | | | | | | | | | | | Q | | | | | | | | |
| 21-225_213D2_LC | | | | | | | | | | | | | | H | | | | | | | | | | | | |
| 21-225_213E5_LC | | | | A | | G | | | | | | | K | H | | | | | | | R | | | | | |
| 21-225_215A12_LC | | | | D | | G | | | | | | | | | | | | | | | | | | | | |
| 21-225_215B5_LC | | | | | | | | | | | | | | H | | | | | | | | | | | | |
| 21-225_215D6_LC | | | | | | | | | | | | | | H | | | | | | | | | | | | |
| 21-225_216G1_LC | | | | | | | | | | | | | S | | | | | L | | | | | | | | |
| 21-225_217B10_LC | | | | A | | G | | | | | | | K | H | | | | | | | R | | | | | |
| 21-225_217G5_LC | | | | | | G | | | | | | | | | | | | | | | R | | | | | |
| 21-225_219H1_LC | | | | | | | | | | | | | | | | D | | | | | | | | | | |
| 21-225_221F6_LC | | | | | | | | | | | | | | | | | | | | | | | | | | |
| 21-225_222A11_LC | | | | | | | | | | | | | | | | | | | | | | | | | | |
| 21-225_222D10_LC | | | | | | | | | | | | | | | | | | | | | | | | | | |
| 21-225_223H10_LC | | | | | | | | | | | | | | | | | | | | | | | | | | |
| 21-225_22A2_LC | | | | D | | | | | | | | | | | | | | V | | | | | | | | |
| 21-225_23D11_LC | | | | | | A | | | | | | | | | | | | | | | R | | | | | |
| 21-225_32G3_LC | | | | A | | | | | | | | | | | | | | | | | | | | | | |
| 21-225_55A11_LC | | | | D | | N | | | | | | | I | | | | | | | | | | | | | |
| 21-225_55D4_LC | | | | D | | | | | | | | | | | | | | I | | | | | | | | |
| 21-225_65H11_LC | | | | D | | | | | | | | | T | | | | | | | | | | | | | |
| 21-225_70H6_LC | | | | S | | F | | | | | | | | | | | | N | | R | | | | | | |
| 21-225_74C8_LC | | | | | | G | | | | | | | K | | | | | | | | | | | | | |
| 21-225_74G6_LC | | | | | | G | | | | | | | K | | | | | | | | | | | | | |
| 21-225_76C5_LC | | | | V | | | | | | | | | K | | | | | | | | | | | | | |

4533

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_77E6_LC | . | . | . | . | . | . | . | . | . | . | . | . | K | . | . | . | . | . | . | . | . | . | . | . | . | . |
| 21-225_79A12_LC | . | . | . | V | . | . | . | . | . | . | . | . | K | . | . | . | . | . | . | . | . | . | . | . | . | . |
| 21-225_79F4_LC | . | . | . | . | . | . | . | . | . | . | . | . | K | . | . | . | . | . | . | . | . | . | . | . | . | . |
| 21-225_7F10_LC | . | . | . | S | . | N | . | . | . | . | . | . | . | . | . | . | . | . | . | . | . | . | . | . | . | . |
| 21-225_80C11_LC | . | . | . | . | . | G | . | . | . | . | . | . | K | . | . | . | . | . | . | . | . | . | . | N | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | S | L | I | Y | A | - | - | - | - | - | - | - | - | A | S | S | L | Q | S | G | V | P | S | K | F |
| 21-225_147E9_LC | . | . | . | . | V | . | . | . | . | . | . | . | . | . | . | . | . | . | . | . | . | . | . | . | . | . |
| 21-225_150G8_LC | . | . | . | . | . | V | . | . | . | . | . | . | . | . | . | . | . | . | . | N | . | . | . | . | ※ | . |
| 21-225_153F9_LC | . | . | . | . | S | . | . | . | . | . | . | . | . | . | . | . | . | . | . | . | . | . | . | . | . | . |
| 21-225_157C1_LC | . | . | . | . | . | . | . | . | . | . | . | . | . | . | . | . | . | . | L | . | . | . | . | . | . | . |
| 21-225_160H4_LC | . | . | . | . | . | V | . | . | . | . | . | . | . | . | . | . | . | . | . | . | . | . | . | . | ※ | . |
| 21-225_163G6_LC | . | . | . | . | . | . | . | . | . | . | . | . | . | . | . | . | . | . | L | . | . | . | . | . | . | . |
| 21-225_166H12_LC | . | . | . | . | . | . | . | . | . | . | . | . | . | . | . | . | . | . | . | . | . | . | . | . | . | . |
| 21-225_169C10_LC | . | . | . | . | ※ | S | . | . | . | . | . | . | . | . | . | . | . | . | . | . | . | . | . | . | . | . |
| 21-225_16A12_LC | . | . | . | . | S | . | . | . | . | . | . | . | . | . | . | . | . | . | . | . | . | . | . | . | . | . |
| 21-225_16D10_LC | . | . | . | . | . | . | . | . | . | . | . | . | . | . | T | ※ | . | . | . | . | . | . | . | . | . | . |
| 21-225_170D1_LC | . | . | . | . | . | . | . | . | . | . | . | . | . | . | . | . | . | . | . | . | . | . | . | . | ※ | . |
| 21-225_170D6_LC | . | . | . | . | . | S | . | . | . | . | . | . | . | . | . | . | . | . | . | . | . | . | . | . | . | . |
| 21-225_171H12_LC | . | . | . | . | . | S | . | . | . | . | . | . | . | . | . | . | . | . | . | . | . | . | . | . | . | . |
| 21-225_175G3_LC | . | . | . | . | ※ | S | . | . | . | . | . | . | . | . | . | . | . | . | . | . | . | . | . | . | . | . |
| 21-225_177A5_LC | . | . | . | . | . | S | . | . | . | . | . | . | . | . | . | . | . | . | . | . | . | . | . | . | . | . |
| 21-225_178H4_LC | . | . | . | . | ※ | S | . | . | . | . | . | . | . | . | . | . | . | . | . | . | . | . | . | . | . | . |
| 21-225_190C11_LC | . | . | L | . | . | ※ | . | . | . | . | . | . | . | . | T | . | . | . | . | . | . | . | . | . | . | . |
| 21-225_190E10_LC | . | . | L | . | . | ※ | . | . | . | . | . | . | . | . | . | . | . | . | . | . | G | . | . | . | . | . |
| 21-225_190E6_LC | . | . | L | . | . | ※ | . | . | . | . | . | . | . | . | . | . | . | . | . | . | . | . | . | . | . | . |
| 21-225_190F8_LC | . | . | L | . | . | ※ | . | . | . | . | . | . | . | . | . | . | . | . | . | . | . | . | . | . | . | . |
| 21-225_190G11_LC | . | . | L | . | . | ※ | . | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . | . | . | . | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | L | . | . | ※ | . | . | . | . | . | . | . | . | V | . | . | . | . | . | . | . | . | . | . | . |
| 21-225_191G12_LC | . | . | L | . | . | ※ | . | . | . | . | . | . | . | . | . | . | . | . | . | . | . | . | . | . | . | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | S | L | I | Y | A | - | - | - | - | - | - | - | - | A | S | S | L | Q | S | G | V | P | S | K | F |
| 21-225_192D3_LC | . | . | . | . | . | . | . | . | . | . | . | . | . | . | . | . | . | . | . | . | . | . | . | . | . | . |
| 21-225_192G6_LC | . | . | . | L | . | K | . | . | . | . | . | . | . | . | . | . | . | . | . | . | . | . | . | . | . | . |
| 21-225_192G8_LC | . | . | . | L | . | K | . | . | . | . | . | . | . | . | . | . | . | . | . | . | . | . | . | . | . | . |
| 21-225_192H10_LC | . | . | . | . | . | . | . | . | . | . | . | . | . | . | . | . | . | . | . | . | . | . | . | . | . | . |
| 21-225_192H4_LC | . | . | . | . | S | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . | . | . | . | . | . | . | . |
| 21-225_197B6_LC | . | . | . | . | . | G | . | . | . | . | . | . | . | . | . | . | . | . | . | . | . | . | . | . | . | . |
| 21-225_197D3_LC | . | . | . | . | . | . | . | . | . | . | . | . | . | . | . | . | . | . | . | . | . | . | . | . | . | . |
| 21-225_197F2_LC | . | . | . | L | . | K | . | . | . | . | . | . | . | . | T | . | . | . | . | . | . | . | . | . | . | . |
| 21-225_197G12_LC | . | . | . | L | . | K | . | . | . | . | . | . | . | . | T | . | . | . | . | . | . | P | . | . | . | . |
| 21-225_198B6_LC | . | . | . | . | . | . | . | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . | . | . | . | . | . | . | . |
| 21-225_1F1_LC | . | . | . | . | S | V | . | . | . | . | . | . | . | . | . | . | . | . | . | T | . | . | . | . | X | . |
| 21-225_203H1_LC | . | . | . | . | . | V | . | . | . | . | . | . | . | . | . | . | R | . | . | . | . | . | . | . | . | . |
| 21-225_204G6_LC | . | . | . | . | . | . | . | . | . | . | . | . | . | . | . | . | . | . | . | . | . | . | . | . | X | . |
| 21-225_204G8_LC | . | . | . | . | S | . | . | . | . | . | . | . | . | . | . | . | . | . | . | . | . | . | . | . | Q | . |
| 21-225_205F5_LC | . | . | . | . | . | G | . | . | . | . | . | . | . | . | V | . | . | . | . | . | . | . | . | . | . | . |
| 21-225_205H3_LC | . | . | . | . | . | . | . | . | . | . | . | . | . | . | . | . | . | . | . | . | . | . | . | . | . | . |
| 21-225_207G6_LC | . | . | . | . | R | . | . | . | . | . | . | . | . | . | . | . | . | . | . | . | . | . | . | . | . | . |
| 21-225_211A11_LC | R | . | . | . | . | D | . | . | . | . | . | . | . | . | . | . | . | . | E | . | . | . | . | . | . | . |
| 21-225_211A3_LC | . | . | . | . | . | . | . | . | . | . | . | . | . | . | . | . | R | . | . | . | . | . | . | . | . | . |
| 21-225_211A8_LC | . | . | . | . | . | . | . | . | . | . | . | . | . | . | . | . | . | . | . | . | . | . | S | . | X | . |
| 21-225_212D7_LC | . | . | . | . | R | . | . | . | . | . | . | . | . | . | . | . | . | . | . | . | . | . | . | . | . | . |
| 21-225_212E10_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | S | L | I | Y | A | - | - | - | - | - | - | - | - | A | S | S | L | Q | S | G | V | P | S | K | F |
| 21-225_212E6_LC | . | . | . | . | . | . | . | . | . | . | . | . | . | . | . | . | . | . | . | . | . | . | . | . | . | . |
| 21-225_212F10_LC | . | . | . | . | . | S | . | . | . | . | . | . | . | . | . | . | N | . | . | . | . | . | . | . | . | . |
| 21-225_212H11_LC | . | . | . | . | . | . | . | . | . | . | . | . | . | . | . | . | R | . | . | . | . | . | . | . | . | . |
| 21-225_212H9_LC | . | . | . | . | . | . | . | . | . | . | . | . | . | . | . | . | . | . | . | . | . | . | . | . | X | . |
| 21-225_213B8_LC | . | . | . | . | . | . | . | . | . | . | . | . | . | . | . | . | . | . | . | . | . | . | . | . | . | . |
| 21-225_213C4_LC | . | T | . | . | . | . | . | . | . | . | . | . | . | . | . | . | . | . | . | . | . | . | . | . | . | . |
| 21-225_213D2_LC | . | . | . | . | M | . | . | . | . | . | . | . | . | . | . | . | . | . | . | . | . | . | . | . | . | . |
| 21-225_213E5_LC | . | . | . | . | . | . | . | . | . | . | . | . | . | . | . | . | R | . | . | . | . | . | . | . | . | . |
| 21-225_215A12_LC | . | . | . | . | . | . | . | . | . | . | . | . | . | . | . | . | . | . | . | . | . | . | . | . | . | . |
| 21-225_215B5_LC | . | . | . | . | R | . | . | . | . | . | . | . | . | . | . | . | . | . | . | . | . | . | . | . | . | . |
| 21-225_215D6_LC | . | . | . | . | . | . | . | . | . | . | . | . | . | . | . | . | . | . | . | H | . | . | . | . | . | . |
| 21-225_216G1_LC | . | . | . | . | . | . | . | . | . | . | . | . | . | . | . | . | . | . | . | . | . | . | . | . | R | . |
| 21-225_217B10_LC | . | . | . | . | . | . | . | . | . | . | . | . | . | . | . | . | R | . | . | . | . | . | . | . | . | . |
| 21-225_217G5_LC | . | . | . | . | . | . | . | . | . | . | . | . | . | . | . | . | . | . | . | . | . | . | . | . | . | . |
| 21-225_219H1_LC | . | . | . | . | . | S | . | . | . | . | . | . | . | . | . | . | N | . | . | . | . | . | . | . | . | . |
| 21-225_221F6_LC | . | . | . | . | . | . | . | . | . | . | . | . | . | . | . | . | N | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | N | . | . | . | . | . | . | . | . | . |
| 21-225_22A2_LC | . | . | . | . | . | . | . | . | . | . | . | . | . | . | . | . | . | . | . | . | . | . | . | . | . | . |
| 21-225_23D11_LC | . | . | . | . | S | . | . | . | . | . | . | . | . | . | . | . | . | . | . | . | . | . | . | . | . | . |
| 21-225_32G3_LC | . | . | . | . | . | D | . | . | . | . | . | . | . | . | T | . | . | . | . | . | . | . | . | . | . | . |
| 21-225_55A11_LC | . | . | . | . | . | . | . | . | . | . | . | . | . | . | . | . | . | . | . | . | . | . | . | . | . | . |
| 21-225_55D4_LC | . | . | . | . | . | T | . | . | . | . | . | . | . | . | . | . | . | . | . | . | . | . | . | . | . | . |
| 21-225_65H11_LC | . | . | . | . | . | . | . | . | . | . | . | . | . | . | . | . | . | . | . | . | . | . | . | . | . | . |
| 21-225_70H6_LC | . | V | . | . | . | T | . | . | . | . | . | . | . | . | . | . | . | . | . | . | . | I | . | . | R | . |
| 21-225_74C8_LC | . | . | . | . | . | T | . | . | . | . | . | . | . | . | T | . | N | . | . | . | . | A | . | . | . | . |
| 21-225_74G6_LC | . | . | . | . | . | T | . | . | . | . | . | . | . | . | T | . | . | . | . | . | . | A | . | . | . | . |
| 21-225_76C5_LC | . | . | . | . | . | . | . | . | . | . | . | . | . | . | . | . | . | . | . | . | . | A | . | . | . | . |

EP 4 435 105 A2

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | S | L | I | Y | A | - | - | - | - | - | - | - | - | A | S | S | L | Q | S | G | V | P | S | K | F |
| 21-225_77E6_LC | . | . | . | . | . | . | . | . | . | . | . | . | . | . | . | . | . | . | . | G | . | A | . | . | . | . |
| 21-225_79A12_LC | . | . | . | . | . | . | . | . | . | . | . | . | . | . | . | . | . | . | . | . | . | A | . | . | . | . |
| 21-225_79F4_LC | . | . | . | . | . | . | . | . | . | . | . | . | . | . | . | . | . | . | . | G | . | A | . | . | . | . |
| 21-225_7F10_LC | . | . | . | . | S | V | . | . | . | . | . | . | . | . | . | . | . | . | . | . | . | . | . | . | S | . |
| 21-225_80C11_LC | . | . | . | . | . | T | . | . | . | . | . | . | . | . | . | . | . | . | . | . | . | A | . | . | . | . |

4538

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_147E9_LC | . | . | . | . | . | . | . | . | . | . | . | . | . | . | . | . | . | . | . | . | . | . | . | . | . | . |
| 21-225_150G8_LC | . | . | . | . | . | . | . | . | . | . | . | . | . | . | . | . | . | . | . | . | . | . | . | . | . | . |
| 21-225_153F9_LC | . | . | . | . | . | . | . | . | . | . | . | . | . | . | . | . | . | . | . | . | . | . | . | . | . | . |
| 21-225_157C1_LC | . | . | . | . | . | . | . | . | . | . | . | . | . | . | . | . | . | . | . | . | . | . | . | . | . | . |
| 21-225_160H4_LC | . | . | . | . | . | . | . | . | . | . | . | . | . | . | . | . | . | . | . | . | . | . | . | . | . | . |
| 21-225_163G6_LC | . | . | . | . | . | . | . | . | . | . | . | . | . | . | . | . | . | . | . | . | . | . | . | . | . | . |
| 21-225_166H12_LC | . | . | . | . | . | . | . | . | . | . | . | . | . | . | . | . | . | . | . | . | . | . | . | . | . | . |
| 21-225_169C10_LC | . | . | . | . | . | . | . | . | . | . | N | . | . | . | . | . | . | . | . | . | . | . | . | . | . | . |
| 21-225_16A12_LC | . | . | . | . | . | . | . | . | ▧ | . | . | . | . | . | . | . | . | . | . | . | . | . | . | . | . | . |
| 21-225_16D10_LC | . | . | . | . | . | . | . | . | ▧ | . | . | . | . | . | . | . | . | . | . | . | . | . | . | . | . | . |
| 21-225_170D1_LC | . | . | . | . | . | . | . | . | . | . | . | . | . | . | . | . | . | . | . | . | . | . | . | . | . | . |
| 21-225_170D6_LC | . | . | . | . | . | . | . | . | . | . | . | . | . | . | . | . | . | . | . | . | . | . | . | . | . | . |
| 21-225_171H12_LC | . | . | . | . | . | . | . | . | . | . | . | . | . | . | . | . | . | . | . | . | . | . | . | . | . | . |
| 21-225_175G3_LC | . | . | . | . | . | . | . | . | . | . | . | . | . | . | . | . | . | . | . | . | . | . | . | . | . | . |
| 21-225_177A5_LC | . | . | . | . | . | . | . | . | . | . | . | . | . | . | . | . | . | . | . | . | . | . | . | . | . | . |
| 21-225_178H4_LC | . | . | . | . | . | . | . | . | . | . | . | . | . | . | . | . | N | . | . | . | . | . | . | . | . | . |
| 21-225_190C11_LC | . | . | . | . | . | . | . | . | . | . | . | . | . | . | . | . | . | . | . | . | . | . | . | . | . | . |
| 21-225_190E10_LC | . | . | . | . | . | . | . | . | . | . | . | . | . | . | . | . | . | . | . | . | . | . | . | . | . | . |
| 21-225_190E6_LC | . | . | . | . | . | . | . | . | . | . | . | . | . | . | . | . | . | . | . | . | . | . | . | . | . | . |
| 21-225_190F8_LC | . | . | . | . | . | . | . | . | . | . | . | . | . | . | . | . | . | . | . | . | . | . | . | . | . | . |
| 21-225_190G11_LC | . | . | . | . | . | . | . | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . | . | . | . | N | . | . | . |
| 21-225_191A10_LC | . | . | . | . | Y | . | . | . | . | . | . | . | . | . | . | . | . | . | . | . | . | . | N | . | ; | . |
| 21-225_191E10_LC | . | . | . | . | . | . | . | . | . | . | . | . | . | . | . | . | . | . | . | . | . | . | N | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | . | . | . | . | . | . | . | . | . | . | . | . | . | . | . | . | . | . | . | . | . | . | . |
| 21-225_191G12_LC | . | . | . | . | . | . | . | . | . | . | . | . | . | . | . | . | . | . | . | . | . | . | . | . | . | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_192D3_LC | . | . | . | . | . | . | . | . | . | . | . | . | . | . | . | . | . | . | . | . | . | . | . | . | . | . |
| 21-225_192G6_LC | . | . | . | . | . | . | . | . | . | . | . | . | . | . | . | . | . | . | . | . | . | . | . | . | . | . |
| 21-225_192G8_LC | . | . | . | . | . | . | . | . | . | . | . | . | . | . | . | . | . | . | . | . | . | . | . | . | . | . |
| 21-225_192H10_LC | . | . | . | . | Y | . | . | . | . | . | . | . | . | . | . | . | . | . | . | . | N | . | . | . | . | . |
| 21-225_192H4_LC | . | . | . | :: | . | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . | . | . | . | . | . | . | . |
| 21-225_197B6_LC | . | . | . | . | . | . | . | . | . | . | . | . | . | . | . | . | . | . | . | . | N | . | . | . | . | . |
| 21-225_197D3_LC | . | . | . | . | . | . | . | . | . | . | . | . | . | . | . | . | . | . | . | . | . | . | . | . | . | . |
| 21-225_197F2_LC | . | . | . | . | . | . | . | . | . | . | . | . | . | . | . | . | . | . | . | . | . | . | . | . | . | . |
| 21-225_197G12_LC | . | . | . | . | . | . | . | . | . | . | . | . | . | . | . | . | . | . | . | . | . | . | . | . | . | . |
| 21-225_198B6_LC | . | . | N | :: | . | . | . | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . | . | . | . | . | . | A | . |
| 21-225_1F1_LC | . | . | . | . | . | . | . | . | . | . | . | . | . | . | . | . | . | . | . | . | . | . | . | . | . | . |
| 21-225_203H1_LC | . | . | T | . | . | . | . | . | S | . | . | . | . | . | . | . | . | . | . | D | . | . | . | . | . | . |
| 21-225_204G6_LC | . | . | . | . | . | . | . | . | . | . | . | . | . | . | . | . | . | . | . | . | . | . | . | . | . | . |
| 21-225_204G8_LC | . | . | . | . | . | . | . | . | . | E | . | . | . | . | . | . | . | . | . | . | . | . | . | . | . | . |
| 21-225_205F5_LC | . | . | . | . | . | . | . | . | . | . | . | . | . | . | . | . | . | . | . | . | . | . | . | . | . | . |
| 21-225_205H3_LC | . | . | . | . | . | . | . | . | S | . | . | . | . | . | . | . | . | . | . | . | . | . | . | . | . | . |
| 21-225_207G6_LC | . | . | N | :: | . | . | . | . | . | . | . | . | . | . | . | . | . | . | . | . | . | . | . | . | . | . |
| 21-225_211A11_LC | . | . | . | :: | . | . | . | . | . | . | . | . | . | . | . | G | . | . | . | . | . | . | . | . | . | . |
| 21-225_211A3_LC | . | . | . | . | . | . | . | . | . | . | . | . | . | . | . | . | . | . | . | . | . | . | . | L | . | . |
| 21-225_211A8_LC | . | . | . | . | . | . | . | . | . | . | . | . | . | . | . | . | . | . | . | . | . | . | . | . | . | . |
| 21-225_212D7_LC | . | . | N | :: | . | . | . | . | . | . | . | . | . | . | . | N | N | . | . | . | . | . | . | V | . | . |
| 21-225_212E10_LC | . | . | . | . | . | . | . | . | . | . | . | . | . | . | . | . | . | . | . | . | . | . | . | . | . | . |

4540

FIGURE 57

| Reference # | 73 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FRS | | | | | | | | | | | | | | | |
| CONSENSUS | S | G | S | G | S | G | - | - | T | D | F | T | L | T | I | S | S | L | Q | P | E | D | F | A | T | Y |
| 21-225_212E6_LC | . | . | . | R | . | . | . | . | . | . | . | . | . | . | . | . | . | . | . | . | . | . | . | . | . | . |
| 21-225_212F10_LC | . | . | . | R | . | . | . | . | . | . | . | . | . | . | . | . | . | . | . | . | . | . | . | . | . | . |
| 21-225_212H11_LC | . | . | . | . | . | . | . | . | . | . | . | . | . | . | . | . | . | . | . | . | . | . | L | . | . | . |
| 21-225_212H9_LC | . | . | . | . | . | . | . | . | . | . | . | . | . | . | . | . | . | . | . | . | . | . | . | . | . | . |
| 21-225_213B8_LC | . | . | . | . | . | . | . | . | . | . | . | . | . | . | . | . | . | . | . | . | . | . | . | . | . | . |
| 21-225_213C4_LC | . | . | . | . | . | . | . | . | . | . | . | . | . | . | . | . | . | . | . | . | . | . | . | . | . | . |
| 21-225_213D2_LC | . | . | N | R | . | . | . | . | . | . | . | . | . | . | . | N | N | . | . | . | . | . | . | V | . | . |
| 21-225_213E5_LC | . | . | . | . | . | . | . | . | . | . | . | . | . | . | . | . | . | . | . | . | . | . | L | . | . | . |
| 21-225_215A12_LC | . | . | . | R | . | . | . | . | . | . | . | . | . | . | . | . | . | . | . | S | . | . | . | . | . | . |
| 21-225_215B5_LC | . | . | N | R | . | . | . | . | . | . | . | . | . | . | . | N | N | . | . | . | . | . | . | V | . | . |
| 21-225_215D6_LC | . | . | . | R | . | . | . | . | . | . | . | . | . | . | . | . | . | . | . | . | . | . | . | . | . | . |
| 21-225_216G1_LC | . | . | . | . | . | . | . | . | . | . | . | . | . | . | . | . | . | . | . | . | . | . | . | . | . | . |
| 21-225_217B10_LC | . | . | . | . | . | . | . | . | . | . | . | . | . | . | . | . | . | V | . | . | . | . | L | . | . | . |
| 21-225_217G5_LC | . | . | T | R | . | . | . | . | . | . | . | . | . | . | . | . | . | . | . | D | . | . | . | . | . | . |
| 21-225_219H1_LC | . | . | . | R | . | . | . | . | . | . | . | . | . | . | . | . | . | . | . | . | . | . | . | . | . | . |
| 21-225_221F6_LC | . | . | . | R | . | . | . | . | . | . | . | . | . | . | . | . | . | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | . | . | . | . | . | . | . | . | S | . |
| 21-225_22A2_LC | . | . | . | . | . | . | . | . | . | . | . | . | . | . | . | . | . | . | . | . | . | . | . | . | S | . |
| 21-225_23D11_LC | . | . | . | . | . | . | . | . | E | . | . | . | . | . | . | . | . | . | . | . | . | . | . | . | . | . |
| 21-225_32G3_LC | . | . | . | . | . | . | . | . | . | . | . | . | . | . | . | . | T | . | . | . | . | . | . | . | . | . |
| 21-225_55A11_LC | . | . | . | . | . | . | . | . | . | . | . | . | . | . | . | . | . | . | . | . | . | . | . | . | . | . |
| 21-225_55D4_LC | . | . | . | . | F | . | . | . | . | . | . | . | . | . | . | . | N | . | . | . | . | . | . | . | . | . |
| 21-225_65H11_LC | . | . | . | . | . | . | . | . | . | . | . | . | . | . | . | . | . | . | . | . | . | . | . | . | . | . |
| 21-225_70H6_LC | . | . | . | . | . | . | . | . | . | . | . | . | . | . | . | . | . | . | . | . | . | . | S | . | . | . |
| 21-225_74C8_LC | . | . | . | . | . | . | . | . | . | . | . | . | . | . | . | . | . | . | . | . | Y | . | . | . | . | . |
| 21-225_74G6_LC | . | . | . | . | . | . | . | . | . | . | . | . | . | . | . | . | . | . | . | . | Y | . | . | . | . | . |
| 21-225_76C5_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | K_FR3 | | | | | | | | | | | | | | | | |
| CONSENSUS | S | G | S | G | S | G | - | - | T | D | F | T | L | T | I | S | S | L | Q | P | E | D | F | A | T | Y |
| 21-225_77E6_LC | | | | | | | | | | | | | | | | | | | | | | | | | | |
| 21-225_79A12_LC | | | | | | | | | | | | | | | | | | | | | | | | | | |
| 21-225_79F4_LC | | | | | | | | | | | | | | | | | | | | | | | | | | |
| 21-225_7F10_LC | | | | | | | | | | | | | | | | | | | | | | | | | | |
| 21-225_80C11_LC | | | | | | | | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | S | T | - | - | - | - | - | - | - | - | - | - | - | - | - | - | - | - | - | - | - |
| 21-225_147E9_LC | . | . | . | . | . | N | S | . | . | . | . | . | . | . | . | . | . | . | . | . | . | . | . | . | . | . |
| 21-225_150G8_LC | . | . | . | . | . | N | N | . | . | . | . | . | . | . | . | . | . | . | . | . | . | . | . | . | . | . |
| 21-225_153F9_LC | . | . | . | . | . | N | S | . | . | . | . | . | . | . | . | . | . | . | . | . | . | . | . | . | . | . |
| 21-225_157C1_LC | . | . | . | . | . | N | S | . | . | . | . | . | . | . | . | . | . | . | . | . | . | . | . | . | . | . |
| 21-225_160H4_LC | . | . | . | . | . | N | S | . | . | . | . | . | . | . | . | . | . | . | . | . | . | . | . | . | . | . |
| 21-225_163G6_LC | . | . | . | . | . | H | S | . | . | . | . | . | . | . | . | . | . | . | . | . | . | . | . | . | . | . |
| 21-225_166H12_LC | . | . | . | . | . | N | S | . | . | . | . | . | . | . | . | . | . | . | . | . | . | . | . | . | . | . |
| 21-225_169C10_LC | . | . | . | . | S | D | S | . | . | . | . | . | . | . | . | . | . | . | . | . | . | . | . | . | . | . |
| 21-225_16A12_LC | . | . | . | . | . | Y | S | . | . | . | . | . | . | . | . | . | . | . | . | . | . | . | . | . | . | . |
| 21-225_16D10_LC | . | . | . | . | . | N | S | . | . | . | . | . | . | . | . | . | . | . | . | . | . | . | . | . | . | . |
| 21-225_170D1_LC | . | . | . | . | C | D | S | . | . | . | . | . | . | . | . | . | . | . | . | . | . | . | . | . | . | . |
| 21-225_170D6_LC | . | . | . | . | S | D | S | . | . | . | . | . | . | . | . | . | . | . | . | . | . | . | . | . | . | . |
| 21-225_171H12_LC | . | . | . | . | S | D | S | . | . | . | . | . | . | . | . | . | . | . | . | . | . | . | . | . | . | . |
| 21-225_175G3_LC | . | . | . | . | S | D | S | . | . | . | . | . | . | . | . | . | . | . | . | . | . | . | . | . | . | . |
| 21-225_177A5_LC | . | . | . | . | S | D | S | . | . | . | . | . | . | . | . | . | . | . | . | . | . | . | . | . | . | . |
| 21-225_178H4_LC | . | . | . | . | S | N | S | . | . | . | . | . | . | . | . | . | . | . | . | . | . | . | . | . | . | . |
| 21-225_190C11_LC | . | . | . | . | . | M | . | . | . | . | . | . | . | . | . | . | . | . | . | . | . | . | . | . | . | . |
| 21-225_190E10_LC | . | . | . | . | . | M | . | . | . | . | . | . | . | . | . | . | . | . | . | . | . | . | . | . | . | . |
| 21-225_190E6_LC | . | . | . | . | . | M | . | . | . | . | . | . | . | . | . | . | . | . | . | . | . | . | . | . | . | . |
| 21-225_190F8_LC | . | . | . | . | . | M | . | . | . | . | . | . | . | . | . | . | . | . | . | . | . | . | . | . | . | . |
| 21-225_190G11_LC | . | . | . | . | . | M | . | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . | . | . | . | . | . | . | . |
| 21-225_191G1_LC | . | . | . | N | . | L | . | . | . | . | . | . | . | . | . | . | . | . | . | . | . | . | . | . | . | . |
| 21-225_191G10_LC | . | . | . | . | . | M | . | . | . | . | . | . | . | . | . | . | . | . | . | . | . | . | . | . | . | . |
| 21-225_191G12_LC | . | . | . | . | . | I | . | . | . | . | . | . | . | . | . | . | . | . | . | . | . | . | . | . | . | . |
| 21-225_192C8_LC | . | . | . | N | . | L | . | . | . | . | . | . | . | . | . | . | . | . | . | . | . | . | . | . | . | . |
| 21-225_192D2_LC | . | . | . | N | . | V | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | S | T | - | - | - | - | - | - | - | - | - | - | - | - | - | - | - | - | - | - | - |
| 21-225_192D3_LC | . | . | L | H | . | L | . | . | . | . | . | . | . | . | . | . | . | . | . | . | . | . | . | . | . | . |
| 21-225_192G6_LC | . | . | . | . | . | W | . | . | . | . | . | . | . | . | . | . | . | . | . | . | . | . | . | . | . | . |
| 21-225_192G8_LC | . | . | . | . | . | I | . | . | . | . | . | . | . | . | . | . | . | . | . | . | . | . | . | . | . | . |
| 21-225_192H10_LC | . | . | . | . | . | . | . | . | . | . | . | . | . | . | . | . | . | . | . | . | . | . | . | . | . | . |
| 21-225_192H4_LC | . | G | L | H | . | L | N | . | . | . | . | . | . | . | . | . | . | . | . | . | . | . | . | . | . | . |
| 21-225_193A10_LC | F | . | . | H | . | L | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | L | H | . | L | . | . | . | . | . | . | . | . | . | . | . | . | . | . | . | . | . | . | . | . |
| 21-225_194H11_LC | . | . | . | H | . | L | . | . | . | . | . | . | . | . | . | . | . | . | . | . | . | . | . | . | . | . |
| 21-225_195A9_LC | . | . | . | H | . | L | . | . | . | . | . | . | . | . | . | . | . | . | . | . | . | . | . | . | . | . |
| 21-225_196C7_LC | . | . | . | H | . | L | . | . | . | . | . | . | . | . | . | . | . | . | . | . | . | . | . | . | . | . |
| 21-225_197B6_LC | . | . | . | . | . | . | . | . | . | . | . | . | . | . | . | . | . | . | . | . | . | . | . | . | . | . |
| 21-225_197D3_LC | . | . | . | H | . | L | . | . | . | . | . | . | . | . | . | . | . | . | . | . | . | . | . | . | . | . |
| 21-225_197F2_LC | . | F | . | . | . | I | . | . | . | . | . | . | . | . | . | . | . | . | . | . | . | . | . | . | . | . |
| 21-225_197G12_LC | . | F | . | . | . | W | . | . | . | . | . | . | . | . | . | . | . | . | . | . | . | . | . | . | . | . |
| 21-225_198B6_LC | . | . | . | . | . | . | . | . | . | . | . | . | . | . | . | . | . | . | . | . | . | . | . | . | . | . |
| 21-225_199C5_LC | . | . | . | . | . | L | . | . | . | . | . | . | . | . | . | . | . | . | . | . | . | . | . | . | . | . |
| 21-225_1F1_LC | . | . | . | . | . | N | S | . | . | . | . | . | . | . | . | . | . | . | . | . | . | . | . | . | . | . |
| 21-225_203H1_LC | . | . | . | R | . | H | . | . | . | . | . | . | . | . | . | . | . | . | . | . | . | . | . | . | . | . |
| 21-225_204G6_LC | . | . | . | H | . | L | S | . | . | . | . | . | . | . | . | . | . | . | . | . | . | . | . | . | . | . |
| 21-225_204G8_LC | . | . | . | . | . | N | . | . | . | . | . | . | . | . | . | . | . | . | . | . | . | . | . | . | . | . |
| 21-225_205F5_LC | T | . | . | . | . | N | . | . | . | . | . | . | . | . | . | . | . | . | . | . | . | . | . | . | . | . |
| 21-225_205H3_LC | . | . | . | . | . | N | . | . | . | . | . | . | . | . | . | . | . | . | . | . | . | . | . | . | . | . |
| 21-225_207G6_LC | . | . | . | . | . | N | . | . | . | . | . | . | . | . | . | . | . | . | . | . | . | . | . | . | . | . |
| 21-225_211A11_LC | . | . | . | H | . | W | . | . | . | . | . | . | . | . | . | . | . | . | . | . | . | . | . | . | . | . |
| 21-225_211A3_LC | . | . | . | . | . | . | N | . | . | . | . | . | . | . | . | . | . | . | . | . | . | . | . | . | . | . |
| 21-225_211A8_LC | . | . | L | R | . | D | . | . | . | . | . | . | . | . | . | . | . | . | . | . | . | . | . | . | . | . |
| 21-225_212D7_LC | . | . | . | . | . | N | . | . | . | . | . | . | . | . | . | . | . | . | . | . | . | . | . | . | . | . |
| 21-225_212E10_LC | . | . | . | . | . | N | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_32B6_LC | . | . | . | . | . | I | . | . | . | . | . | . | . | . | . | . | . | . | . | . | . | . | . | . | . | . |
| 21-225_33C2_LC | . | . | . | . | . | . | . | . | . | . | . | . | . | . | . | P | . | . | . | . | . | . | . | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | . | . | T | . | . | . | . | . | . | . | . | . | . | . |
| 21-225_44D3_LC | . | . | . | . | . | . | . | . | . | . | . | . | . | . | . | . | . | . | . | . | . | . | . | . | . | . |
| 21-225_44D5_LC | . | . | . | . | . | . | . | . | . | . | . | . | . | . | . | . | T | . | S | . | . | . | . | . | . | . |
| 21-225_44F9_LC | . | . | . | . | . | . | . | . | . | . | . | . | . | . | . | . | . | . | . | . | . | . | . | . | . | . |
| 21-225_45B4_LC | . | . | . | . | . | . | . | . | . | . | . | . | . | . | . | . | . | . | . | . | . | . | . | . | . | . |
| 21-225_45B8_LC | . | . | . | . | . | . | . | . | . | . | . | . | . | . | . | . | . | . | . | . | . | . | . | . | . | . |
| 21-225_46C3_LC | . | . | . | . | . | . | . | . | . | . | . | . | . | . | . | . | . | . | . | . | . | . | . | . | . | . |
| 21-225_46D4_LC | . | . | . | . | . | . | . | . | . | . | . | . | . | . | . | . | . | . | S | . | . | . | . | . | . | . |
| 21-225_48A9_LC | . | . | . | . | . | I | . | . | . | . | . | . | . | . | . | . | . | . | . | . | . | . | . | . | . | . |
| 21-225_48C1_LC | . | . | . | . | . | R | . | . | . | . | . | . | . | . | . | . | . | . | . | . | . | . | . | A | . | . |
| 21-225_48F2_LC | . | . | . | . | . | . | . | . | . | . | . | . | . | . | . | . | . | . | . | . | . | . | . | . | . | . |
| 21-225_4C12_LC | T | . | . | . | . | . | . | . | . | . | . | . | . | . | . | . | . | . | R | . | . | . | . | . | . | . |
| 21-225_4C5_LC | . | . | . | . | . | . | . | . | . | . | . | . | . | . | . | . | N | . | . | . | . | . | . | . | . | . |
| 21-225_50A12_LC | . | . | . | . | . | T | . | . | . | . | . | . | . | . | . | . | N | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | . | . | . | . | . | . | . | . | . | . | . | . | . | . | . | . | . | . | . | . | . | . | . | . |
| 21-225_54H3_LC | . | . | . | . | . | . | . | . | . | . | . | . | . | . | . | . | N | . | . | . | . | . | . | . | . | . |
| 21-225_56C7_LC | . | . | . | . | . | T | . | . | . | . | . | . | . | . | . | . | N | . | . | . | . | . | . | . | . | . |
| 21-225_57B8_LC | R | . | . | . | . | . | . | . | . | . | . | . | . | . | . | . | . | . | . | . | . | . | . | . | . | . |
| 21-225_59B9_LC | . | . | . | . | . | . | . | . | . | . | . | . | . | . | . | . | . | . | . | . | . | . | . | . | . | . |
| 21-225_59G7_LC | . | . | . | . | . | S | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | S | . | . | . | . | . | . | . | . | . | . | . | . | . | . | . | . | . | . | . | . |
| 21-225_60E2_LC | . | . | . | . | . | . | . | . | . | . | . | . | . | . | . | . | . | . | . | . | . | . | . | . | . | . |
| 21-225_61E6_LC | S | . | . | . | . | . | . | . | . | . | . | . | . | . | . | . | . | . | . | . | . | . | . | . | . | . |
| 21-225_61H1_LC | . | . | . | . | F | . | . | . | . | . | . | . | . | . | . | . | . | . | . | . | . | . | . | . | . | . |
| 21-225_62F7_LC | . | . | . | . | . | P | . | . | . | . | . | . | . | . | . | . | N | . | . | . | . | . | . | . | . | . |
| 21-225_64A4_LC | . | . | . | . | . | . | . | . | . | . | . | . | . | . | . | . | . | . | . | . | . | . | . | . | . | . |
| 21-225_64C8_LC | . | C | . | . | . | . | . | . | . | . | . | . | . | . | . | . | . | . | . | . | . | . | . | . | . | . |
| 21-225_66C10_LC | . | . | . | . | . | P | . | . | . | . | . | . | . | . | . | . | . | . | . | . | . | . | . | . | . | . |
| 21-225_66D11_LC | . | . | . | . | . | . | . | . | . | . | . | . | . | . | . | . | . | G | . | . | . | . | . | . | . | . |
| 21-225_6A11_LC | . | . | . | . | . | . | . | . | . | . | . | . | . | . | . | . | . | . | . | . | . | . | . | . | . | . |
| 21-225_6G2_LC | Q | . | . | . | . | . | . | . | . | . | . | . | . | . | . | . | . | . | . | . | . | . | . | . | . | . |
| 21-225_71A2_LC | . | . | . | . | . | I | . | . | . | . | . | . | . | . | F | N | . | . | . | I | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | . | . | . | . | . | . | . | . | . | . | . | . | . | . | . | . | . | . | . | . | . | . |
| 21-225_7F8_LC | . | . | . | . | . | . | . | . | . | . | . | . | . | . | . | . | . | . | . | . | . | . | . | . | . | . |
| 21-225_7G2_LC | . | . | . | . | . | T | . | . | . | . | . | . | . | . | . | . | . | . | . | . | . | . | . | . | . | . |
| 21-225_7G4_LC | . | . | . | . | . | . | . | . | . | . | . | . | . | . | . | . | . | . | . | . | . | . | . | . | . | . |
| 21-225_94F3_LC | Q | . | . | . | F | . | . | . | . | . | . | . | . | . | . | . | . | . | . | . | . | . | . | . | . | . |
| 21-225_9A1_LC | . | . | . | . | . | T | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | K_CDR2 | | | | | | | | | | | | | | | |
| CONSENSUS | K | R | L | I | Y | A | - | - | - | - | - | - | - | - | A | S | S | L | Q | S | G | V | P | S | R | F |
| 21-225_9C11_LC | . | L | . | . | . | V | | | | | | . | . | . | . | . | . | . | | | . | . | . | . | . | . |
| 21-225_9D12_LC | . | . | . | . | . | . | | | | | | . | . | . | . | . | . | . | | | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_10E9_LC | . | | | | . | | . | | . | | | | | | . | N | . | | . | | . | | . | | . | |
| 21-225_12B12_LC | . | | | | . | | . | | . | | | | | | . | | . | | . | | . | | . | | . | |
| 21-225_12E6_LC | | . | | | . | | . | | . | | | | | | . | | . | | . | | . | | . | . | . | |
| 21-225_12F2_LC | . | | | | . | | . | | . | | | | | | . | | . | | . | | . | | . | V | . | . |
| 21-225_147H5_LC | . | | | | . | | . | | . | | | | | | . | | . | | . | | . | | . | | . | |
| 21-225_148C9_LC | | | | | . | | . | | . | | | | | | . | | . | | . | | . | | . | | . | |
| 21-225_149D10_LC | | | | | . | | . | | . | | | | | | . | | . | | . | | . | | . | | . | |
| 21-225_14E10_LC | . | | | | . | | . | | . | | | | | . | | . | | . | | . | | . | | T | A | . |
| 21-225_158D4_LC | . | | . | | . | | . | | . | | | | | . | F | . | | . | | . | | . | | . | | |
| 21-225_158G1_LC | . | | . | C | . | | . | | . | | | | | . | V | . | | . | | . | | . | | . | | |
| 21-225_159A3_LC | . | | . | | . | | . | | . | | | | | . | F | . | | . | | . | | . | | . | | |
| 21-225_159C8_LC | . | | . | | . | | . | | . | | | | | . | F | . | | . | | . | | . | | . | | |
| 21-225_159D8_LC | . | | . | | . | | . | | . | | | | | . | F | . | | . | | . | | . | | . | | |
| 21-225_15C11_LC | . | | . | | . | | . | | . | | | | | . | . | . | | . | | . | | . | | . | | . |
| 21-225_160C4_LC | . | | . | | . | | . | | . | | | | | . | F | . | | . | | . | | . | | . | | |
| 21-225_160F2_LC | . | | . | | . | | . | | . | | | | | . | V | . | | . | | . | | . | | . | | |
| 21-225_160H1_LC | . | | . | | . | | . | | . | | | | | . | . | . | | . | | . | | . | | T | . | . |
| 21-225_161G12_LC | . | | . | | . | | . | | . | | | | | . | F | . | | . | | . | | . | | . | | |
| 21-225_169D11_LC | . | | . | | . | | . | | . | | | | | . | . | . | | . | | . | | . | | . | | |
| 21-225_169D7_LC | . | | . | | . | | . | | . | | | | | . | . | . | | . | | . | | . | | . | | . |
| 21-225_16A5_LC | . | N | . | Y | . | | . | | . | | | | | . | . | . | | . | | . | | . | | . | | . |
| 21-225_16G7_LC | . | . | . | | . | | . | | . | | | | | . | . | . | | . | | . | | . | | . | | . |
| 21-225_16H4_LC | . | . | . | | . | | . | | . | | | | | . | . | . | | . | | . | | . | | . | | . |
| 21-225_170E4_LC | . | . | . | | . | | . | | . | | | | | . | . | . | | . | | . | | . | | . | | . |
| 21-225_170F3_LC | . | . | . | | . | | . | | . | | | | | . | . | N | . | | . | | . | | . | | . | . |
| 21-225_170H1_LC | . | . | . | | . | | . | | . | | | | | . | . | . | | . | | . | | . | | . | | . |
| 21-225_174G10_LC | . | . | . | | . | | . | | . | | | | | . | . | . | | . | | . | | . | | . | | . |
| 21-225_17C7_LC | . | . | . | | . | | . | | . | | | | | . | . | . | | . | | . | | . | | . | | . |
| 21-225_17H3_LC | . | . | . | | . | | . | | . | | | | | . | . | . | | . | | . | | . | | . | | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | E | F | T | L | T | I | S | S | L | Q | P | E | D | F | A | T | Y |
| 21-225_18A5_LC | . | | | | . | | | | | | | | | | | | | | | | | | | . | | |
| 21-225_18C2_LC | | | | | R | | | | | | | | | | | | | | | | | | | | | |
| 21-225_18F2_LC | | | | | . | | | | | | | | | | | | | | | | | | | | | |
| 21-225_18H12_LC | . | | | | . | | | | | | | | | | | | | | | | | | | | | |
| 21-225_190C12_LC | | | | | . | | | | | | | | | | | | | | | | | | | | | |
| 21-225_190G3_LC | | | | | . | | | | | | | | | | | | | | | | | | | | | |
| 21-225_190H5_LC | | | | | . | | | | | | | | | | | | | | | | | | | | | |
| 21-225_191A4_LC | | | | | . | | | | | | | | | | | | | | | | | | | | | |
| 21-225_192F7_LC | | | | | . | | | | | | | | | | | | | | | | | | | | | |
| 21-225_196C12_LC | | | | | . | | | | | | | | i | | | | | | | | | | | E | | |
| 21-225_19A10_LC | | | | | | | | | | | | | | | | | R | | | | | | | | | |
| 21-225_200C9_LC | | | | | | | | | | | | | | | | | | | | | | | | | | |
| 21-225_201C5_LC | | | | | | | | | | | | | | | | | | | | | | | | | | |
| 21-225_203C10_LC | | | | | | | | | | | | | | | | | | | | | | | | | S | |
| 21-225_203F7_LC | | | | | C | | | | | | | | | | | | | V | | | | | | | N | |
| 21-225_204H3_LC | | | | | . | | | | | | | | | | | | | | | | | | | | | |
| 21-225_208B2_LC | | | | | . | | | | | | | | | | | | | | | | | | | | | |
| 21-225_208F1_LC | | | | | . | | | | | | | | | | | | | | | | | | | | | |
| 21-225_20B11_LC | | | | | . | | | | | | | | | | | | | | | | | | | | | |
| 21-225_20B2_LC | . | | | | . | | | | | | | | | | | | | | | | | | | | | . |
| 21-225_20D10_LC | | | | Y | . | | | | | | | | | | | | | | | | | | | | | |
| 21-225_210G5_LC | | | | | . | | | | | | | | | | | | | | | | | | | | | |
| 21-225_210G6_LC | | | | | C | | | | | | | | | | | | | V | | R | | | | | N | |
| 21-225_212B11_LC | | | | | | | | | | | i | | | | | | | | | | | | | | | |
| 21-225_214G10_LC | | | | | | | | | | | i | | | | | | | | | | | | | | | |
| 21-225_214G12_LC | | | | | | | | | | | i | | | | | | | | | | | | | | | |
| 21-225_214H9_LC | | | | | | | | | | | | | | | | | | | | | | | | | | |
| 21-225_215E11_LC | | | | | | | | | | | i | | | | | | | V | | R | | | | | | |
| 21-225_215E3_LC | . | | | | . | | | | | | | | | | | | | | | | | | | | | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_216E8_LC | . | | | . | . | . | . | . | . | . | } | . | . | . | . | . | . | . | . | . | . | . | . | . | . | . |
| 21-225_216H12_LC | . | | | . | . | . | . | . | . | . | } | . | . | . | . | . | . | . | . | . | . | . | . | . | . | . |
| 21-225_217E5_LC | . | | . | . | . | . | . | . | . | . | } | . | . | . | . | . | . | . | . | . | . | . | . | . | . | . |
| 21-225_217G10_LC | . | | | . | . | . | . | . | . | . | } | . | . | . | . | . | . | . | . | . | . | . | . | . | . | . |
| 21-225_217H12_LC | . | | | . | . | . | . | . | . | . | } | . | . | . | . | . | . | . | . | . | . | . | . | . | . | . |
| 21-225_218C4_LC | . | | | . | . | . | . | . | . | . | } | . | . | . | . | . | V | . | . | ※ | . | . | . | . | . | . |
| 21-225_21F7_LC | . | | . | . | . | . | . | . | . | . | . | . | . | . | . | . | . | . | . | . | . | . | . | . | . | . |
| 21-225_21G11_LC | . | | . | . | . | . | . | . | . | . | . | . | . | . | . | . | . | . | . | . | . | . | . | . | . | . |
| 21-225_21G2_LC | . | | . | . | . | . | . | . | . | . | . | . | . | . | . | . | . | . | . | . | . | . | . | . | . | . |
| 21-225_21H8_LC | ※ | | . | . | . | . | . | . | . | . | . | . | . | . | . | . | . | . | . | . | . | . | . | . | . | . |
| 21-225_220F8_LC | . | | . | . | . | . | . | . | . | . | } | . | . | . | . | . | . | . | . | ※ | . | . | . | . | . | . |
| 21-225_224E7_LC | . | | . | . | . | . | . | . | . | . | . | . | . | . | . | . | . | . | . | . | . | . | . | . | . | . |
| 21-225_224F1_LC | . | | . | . | . | . | . | . | . | . | . | . | . | . | . | . | . | . | . | . | . | . | . | . | } | . |
| 21-225_224F3_LC | . | | . | . | . | . | . | . | . | . | . | . | . | . | . | . | . | . | . | . | . | . | . | . | . | . |
| 21-225_225A1_LC | . | | . | . | . | . | . | . | . | . | . | . | . | . | . | . | . | . | . | . | . | . | . | . | . | . |
| 21-225_226C6_LC | . | | . | . | . | . | . | . | . | . | . | . | . | . | . | . | N | . | . | . | . | . | . | . | . | . |
| 21-225_226F7_LC | . | | V | . | . | . | . | . | . | . | } | . | . | . | . | . | . | . | . | . | . | . | . | . | . | . |
| 21-225_226H11_LC | . | | . | . | . | . | . | . | D | . | . | . | . | . | . | . | . | . | . | . | . | . | . | . | . | . |
| 21-225_22A4_LC | . | | . | . | . | . | . | . | . | . | . | . | . | . | . | . | . | . | . | . | . | . | . | . | . | . |
| 21-225_22F4_LC | . | | ※ | . | . | . | . | . | D | . | . | . | . | . | . | . | . | . | . | . | . | . | . | . | . | . |
| 21-225_23A10_LC | . | | . | . | . | . | . | . | . | . | . | . | . | . | . | ※ | . | . | . | . | . | . | . | . | . | . |
| 21-225_23E11_LC | . | | . | . | . | . | . | . | . | . | . | . | . | . | . | . | . | . | . | . | . | . | . | . | . | . |
| 21-225_23F12_LC | . | | . | . | . | . | . | . | . | . | . | . | . | . | . | . | . | . | . | . | . | . | . | S | . | . |
| 21-225_24B6_LC | . | | . | . | . | . | . | . | . | . | . | . | . | . | . | . | . | . | . | T | . | . | . | . | . | . |
| 21-225_25D12_LC | . | | . | . | . | . | . | . | . | . | . | . | . | . | . | . | . | . | . | . | . | . | . | . | . | . |
| 21-225_26A2_LC | . | | . | . | . | . | . | . | . | . | . | . | . | . | ※ | . | . | . | . | . | . | . | . | . | . | . |
| 21-225_28C7_LC | . | | . | . | . | . | . | . | . | . | . | . | . | . | . | . | . | . | . | . | . | . | . | . | . | . |
| 21-225_28F5_LC | . | | . | . | . | . | . | . | . | . | . | . | . | . | . | . | . | . | . | . | . | . | . | . | . | . |
| 21-225_2G4_LC | . | | . | . | . | . | . | . | . | . | . | . | . | . | . | . | . | . | . | . | . | . | L | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_32B6_LC | . | . | . | . | . | . | . | . | . | . | . | . | . | . | . | . | . | . | . | . | . | . | . | . | . | . |
| 21-225_33C2_LC | . | . | . | . | . | . | . | . | . | . | . | . | . | . | . | . | . | . | . | . | . | . | ⋈ | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | . | . | . | . | . | . | . | . | . | . | . | . | . | . |
| 21-225_44D3_LC | . | . | . | ⋈ | . | . | . | . | ⋈ | . | . | . | . | . | . | . | . | . | . | . | . | . | . | . | . | . |
| 21-225_44D5_LC | . | . | . | . | . | . | . | . | . | . | . | . | . | . | . | . | . | . | . | . | . | . | . | . | . | . |
| 21-225_44F9_LC | . | . | . | . | . | . | . | . | . | . | . | . | . | . | . | . | . | . | . | . | . | . | . | . | . | . |
| 21-225_45B4_LC | . | . | . | . | . | . | . | . | . | . | . | . | . | . | . | . | . | . | . | . | . | . | . | . | . | . |
| 21-225_45B8_LC | . | . | . | . | . | . | . | . | . | . | . | . | . | . | . | . | . | . | . | . | . | . | . | . | . | . |
| 21-225_46C3_LC | . | . | . | ⋈ | . | . | . | . | . | . | . | . | . | . | . | . | . | . | . | . | . | . | . | . | . | . |
| 21-225_46D4_LC | . | . | . | . | . | . | . | . | . | . | . | . | . | . | . | . | . | . | . | . | . | . | . | . | . | . |
| 21-225_48A9_LC | . | . | . | . | . | . | . | . | . | . | . | . | . | . | . | . | G | . | . | . | . | . | . | . | ! | . |
| 21-225_48C1_LC | . | . | . | . | . | . | . | . | . | . | . | . | . | . | . | . | . | . | . | . | . | . | . | . | ! | . |
| 21-225_48F2_LC | N | . | . | . | . | . | . | . | . | . | . | . | . | . | . | . | . | . | . | S | . | . | L | . | . | . |
| 21-225_4C12_LC | . | . | . | . | . | . | . | . | . | . | . | . | . | . | . | . | . | . | . | . | . | . | L | . | . | . |
| 21-225_4C5_LC | . | . | . | . | Y | . | . | . | . | . | . | . | . | . | . | G | . | . | . | . | . | . | . | . | . | . |
| 21-225_50A12_LC | . | . | . | . | . | . | . | . | . | . | . | . | . | . | . | . | . | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | G | . | . | . | . | . | ⋈ | . | . | . | . | . | . | . | . | . | . | . | . | . | . | . | . | . |
| 21-225_54H3_LC | . | . | . | . | . | . | . | . | . | . | . | . | . | . | . | . | . | . | . | . | . | . | . | . | . | . |
| 21-225_56C7_LC | . | . | . | . | . | . | . | . | . | . | . | . | . | . | . | . | . | . | . | . | . | . | . | . | . | . |
| 21-225_57B8_LC | . | . | . | . | . | . | . | . | . | . | . | . | . | . | . | . | . | . | . | . | . | . | . | . | . | . |
| 21-225_59B9_LC | . | . | Y | ⋈ | . | . | . | . | . | . | . | . | . | . | . | . | . | . | . | . | . | . | . | . | . | . |
| 21-225_59G7_LC | . | . | . | . | . | . | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | . | . | . | . | . | . | . | . | . | . | . | . | . | . | . | . | . | . | . | . | . |
| 21-225_60E2_LC | . | . | . | . | . | . | . | . | . | . | . | . | . | . | . | . | . | . | . | . | . | . | . | . | . | . |
| 21-225_61E6_LC | . | . | . | . | . | . | . | . | . | . | . | . | . | . | . | . | . | . | . | . | . | . | . | . | . | . |
| 21-225_61H1_LC | . | . | . | . | . | . | . | . | . | . | . | . | . | . | . | . | . | . | . | . | . | . | . | . | . | . |
| 21-225_62F7_LC | . | . | . | . | . | . | . | . | . | . | . | . | . | . | . | . | . | . | . | . | . | . | . | . | . | . |
| 21-225_64A4_LC | . | . | . | . | . | . | . | . | . | . | . | . | . | . | . | . | . | . | . | . | . | . | L | . | . | . |
| 21-225_64C8_LC | . | . | . | . | . | . | . | . | . | . | . | . | . | . | . | . | . | . | . | . | . | . | . | . | . | . |
| 21-225_66C10_LC | . | . | . | . | . | . | . | . | . | . | . | . | . | . | . | . | . | . | . | . | . | . | . | . | . | . |
| 21-225_66D11_LC | . | . | . | C | . | . | . | . | . | . | . | . | I | S | . | . | . | M | . | S | . | . | . | . | . | . |
| 21-225_6A11_LC | . | . | . | . | . | . | . | . | . | . | . | . | . | . | . | . | . | . | . | . | . | . | . | . | . | . |
| 21-225_6G2_LC | . | . | . | Y | . | . | . | . | . | . | N | S | . | . | . | . | . | . | . | . | . | . | . | . | I | . |
| 21-225_71A2_LC | . | . | . | . | . | . | . | . | . | . | . | . | . | . | . | . | . | . | . | . | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | . | . | . | . | . | . | . | . | . | . | . | . | . | . | . | . | . | . | . | . | . | . |
| 21-225_7F8_LC | . | . | V | . | . | . | . | . | . | . | . | . | . | P | . | . | . | . | . | . | . | . | . | . | . | . |
| 21-225_7G2_LC | . | . | . | . | . | . | . | . | . | . | . | . | . | . | . | . | . | . | . | . | . | . | . | . | . | . |
| 21-225_7G4_LC | . | . | . | . | . | . | . | . | . | . | . | . | . | . | . | . | . | . | . | . | . | . | S | . | . | . |
| 21-225_94F3_LC | . | . | . | . | . | . | . | . | . | . | . | . | . | . | . | . | . | . | . | . | . | . | . | . | . | . |
| 21-225_9A1_LC | . | . | . | . | Y | . | . | . | . | . | . | . | I | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_9C11_LC | . | . | . | . | . | . | | | . | . | . | . | . | . | . | N | . | . | . | . | . | . | . | . | : | . |
| 21-225_9D12_LC | . | . | . | . | . | . | | | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | K_CDR3 | | | | | | | |
| CONSENSUS | Y | C | L | Q | H | Y | S | - | - | - | - | - | - | - | - | - | - | - | - | - | - | - | - | - | - | - |
| 21-225_10E9_LC | . | | | | | . | | | | | | | | | | | | | | | | | | | | |
| 21-225_12B12_LC | | | | | . | N | | | | | | | | | | | | | | | | | | | | |
| 21-225_12E6_LC | . | | . | | . | N | | | . | | | | | | | | | | | | | | | | | |
| 21-225_12F2_LC | . | | | | . | N | | | . | | | | | | | | | | | | | | | | | |
| 21-225_147H5_LC | . | | | | . | . | | | . | | | | | | | | | | | | | | | | | |
| 21-225_148C9_LC | . | | | | | . | | | . | | | | | | | | | | | | | | | | | |
| 21-225_149D10_LC | . | | | | | . | | | . | | | | | | | | | | | | | | | | | |
| 21-225_14E10_LC | | | | . | | . | | | . | | | | | | | | | | | | | | | | | |
| 21-225_158D4_LC | . | | | . | | . | | | . | | | | | | | | | | | | | | | | | |
| 21-225_158G1_LC | | | . | . | | N | | | . | | | | | | | | | | | | | | | | | |
| 21-225_159A3_LC | . | | . | . | | N | | | . | | | | | | | | | | | | | | | | | |
| 21-225_159C8_LC | . | | . | . | | N | | | . | | | | | | | | | | | | | | | | | |
| 21-225_159D8_LC | . | | . | | . | . | | | . | | | | | | | | | | | | | | | | | |
| 21-225_15C11_LC | . | | . | L | | . | | | . | | | | | | | | | | | | | | | | | |
| 21-225_160C4_LC | . | | . | | | . | | | . | | | | | | | | | | | | | | | | | |
| 21-225_160F2_LC | . | | . | . | | N | | | . | | | | | | | | | | | | | | | | | |
| 21-225_160H1_LC | . | | . | . | | N | | | . | | | | | | | | | | | | | | | | | |
| 21-225_161G12_LC | . | | . | . | | N | | | . | | | | | | | | | | | | | | | | | |
| 21-225_169D11_LC | . | | . | . | | . | | | . | | | | | | | | | | | | | | | | | |
| 21-225_169D7_LC | . | | . | . | | N | | | . | | | | | | | | | | | | | | | | | |
| 21-225_16A5_LC | . | | . | . | | N | | | . | | | | | | | | | | | | | | | | | |
| 21-225_16G7_LC | . | | . | . | | N | | | . | | | | | | | | | | | | | | | | | |
| 21-225_16H4_LC | . | | . | . | | N | | | . | | | | | | | | | | | | | | | | | |
| 21-225_170E4_LC | . | | . | . | | N | | | . | | | | | | | | | | | | | | | | | |
| 21-225_170F3_LC | . | | . | . | | . | | | . | | | | | | | | | | | | | | | | | |
| 21-225_170H1_LC | . | | . | . | | S | | | . | | | | | | | | | | | | | | | | | |
| 21-225_174G10_LC | . | | . | . | | N | | | . | | | | | | | | | | | | | | | | | |
| 21-225_17C7_LC | . | | . | . | | N | | | . | | | | | | | | | | | | | | | | | |
| 21-225_17H3_LC | . | | . | . | Q | . | | | . | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_18A5_LC | . | | | | . | N | . | . | . | . | . | . | . | . | . | . | . | . | . | . | . | . | . | . | . | . |
| 21-225_18C2_LC | . | | | | . | N | . | . | . | . | . | . | . | . | . | . | . | . | . | . | . | . | . | . | . | . |
| 21-225_18F2_LC | . | | . | . | . | N | . | . | . | . | . | . | . | . | . | . | . | . | . | . | . | . | . | . | . | . |
| 21-225_18H12_LC | . | | | | . | N | N | . | . | . | . | . | . | . | . | . | . | . | . | . | . | . | . | . | . | . |
| 21-225_190C12_LC | . | | | | . | N | . | . | . | . | . | . | . | . | . | . | . | . | . | . | . | . | . | . | . | . |
| 21-225_190G3_LC | . | | . | . | . | N | . | . | . | . | . | . | . | . | . | . | . | . | . | . | . | . | . | . | . | . |
| 21-225_190H5_LC | . | | . | . | . | N | N | . | . | . | . | . | . | . | . | . | . | . | . | . | . | . | . | . | . | . |
| 21-225_191A4_LC | . | | . | . | . | N | . | . | . | . | . | . | . | . | . | . | . | . | . | . | . | . | . | . | . | . |
| 21-225_192F7_LC | . | | . | . | . | T | . | . | . | . | . | . | . | . | . | . | . | . | . | . | . | . | . | . | . | . |
| 21-225_196C12_LC | . | | . | . | . | N | G | . | . | . | . | . | . | . | . | . | . | . | . | . | . | . | . | . | . | . |
| 21-225_19A10_LC | . | | . | . | . | N | G | . | . | . | . | . | . | . | . | . | . | . | . | . | . | . | . | . | . | . |
| 21-225_200C9_LC | . | | . | . | . | N | . | . | . | . | . | . | . | . | . | . | . | . | . | . | . | . | . | . | . | . |
| 21-225_201C5_LC | . | | . | . | . | . | . | . | . | . | . | . | . | . | . | . | . | . | . | . | . | . | . | . | . | . |
| 21-225_203C10_LC | . | | . | . | . | N | . | . | . | . | . | . | . | . | . | . | . | . | . | . | . | . | . | . | . | . |
| 21-225_203F7_LC | . | | . | . | . | . | . | . | . | . | . | . | . | . | . | . | . | . | . | . | . | . | . | . | . | . |
| 21-225_204H3_LC | . | | . | . | . | . | . | . | . | . | . | . | . | . | . | . | . | . | . | . | . | . | . | . | . | . |
| 21-225_208B2_LC | . | | . | . | . | . | . | . | . | . | . | . | . | . | . | . | . | . | . | . | . | . | . | . | . | . |
| 21-225_208F1_LC | . | | . | . | . | . | . | . | . | . | . | . | . | . | . | . | . | . | . | . | . | . | . | . | . | . |
| 21-225_20B11_LC | . | | . | . | . | . | . | . | . | . | . | . | . | . | . | . | . | . | . | . | . | . | . | . | . | . |
| 21-225_20B2_LC | . | | . | . | . | N | . | . | . | . | . | . | . | . | . | . | . | . | . | . | . | . | . | . | . | . |
| 21-225_20D10_LC | . | . | I | . | Q | N | . | . | . | . | . | . | . | . | . | . | . | . | . | . | . | . | . | . | . | . |
| 21-225_210G5_LC | . | . | . | X | . | . | | | | | | | | | | | | | | | | | . | . | . | . |
| 21-225_210G6_LC | . | . | V | . | . | . | | | | | | | | | | | | | | | | | . | . | . | . |
| 21-226_212B11_LC | . | . | L | . | . | . | | | | | | | | | | | | | | | | | . | . | . | . |
| 21-225_214G10_LC | . | . | L | . | N | . | | | | | | | | | | | | | | | | | . | . | . | . |
| 21-225_214G12_LC | . | . | V | M | . | . | | | | | | | | | | | | | | | | | . | . | . | . |
| 21-225_214H9_LC | . | . | . | . | . | . | | | | | | | | | | | | | | | | | . | . | . | . |
| 21-225_215E11_LC | . | . | V | M | . | . | . | . | . | . | . | . | . | . | . | . | . | . | . | . | . | . | . | . | . | . |
| 21-225_215E3_LC | . | . | V | M | . | N | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_216E8_LC | . | | . | M | . | . | | . | . | . | . | . | . | . | . | . | . | . | . | . | . | . | . | . | . | . |
| 21-225_216H12_LC | | | V | M | . | N | . | . | . | . | . | . | . | . | . | . | . | . | . | . | . | . | . | . | . | . |
| 21-225_217E5_LC | . | | V | M | . | N | . | . | . | . | . | . | . | . | . | . | . | . | . | . | . | . | . | . | . | . |
| 21-225_217G10_LC | . | | V | M | . | N | . | . | . | . | . | . | . | . | . | . | . | . | . | . | . | . | . | . | . | . |
| 21-225_217H12_LC | . | | | M | . | . | . | . | . | . | . | . | . | . | . | . | . | . | . | . | . | . | . | . | . | . |
| 21-225_218C4_LC | . | | V | M | . | . | . | . | . | . | . | . | . | . | . | . | . | . | . | . | . | . | . | . | . | . |
| 21-225_21F7_LC | . | | . | . | . | N | N | . | . | . | . | . | . | . | . | . | . | . | . | . | . | . | . | . | . | . |
| 21-225_21G11_LC | . | | . | . | . | N | N | . | . | . | . | . | . | . | . | . | . | . | . | . | . | . | . | . | . | . |
| 21-225_21G2_LC | . | | . | . | . | N | . | . | . | . | . | . | . | . | . | . | . | . | . | . | . | . | . | . | . | . |
| 21-225_21H8_LC | . | | . | . | . | N | . | . | . | . | . | . | . | . | . | . | . | . | . | . | . | . | . | . | . | . |
| 21-225_220F8_LC | . | | V | M | . | N | . | . | . | . | . | . | . | . | . | . | . | . | . | . | . | . | . | . | . | . |
| 21-225_224E7_LC | . | | . | . | . | . | . | . | . | . | . | . | . | . | . | . | . | . | . | . | . | . | . | . | . | . |
| 21-225_224F1_LC | . | | . | . | . | . | . | . | . | . | . | . | . | . | . | . | . | . | . | . | . | . | . | . | . | . |
| 21-225_224F3_LC | . | | . | . | . | . | N | . | . | . | . | . | . | . | . | . | . | . | . | . | . | . | . | . | . | . |
| 21-225_225A1_LC | . | | . | . | . | . | . | . | . | . | . | . | . | . | . | . | . | . | . | . | . | . | . | . | . | . |
| 21-225_226C6_LC | . | | . | . | . | . | N | . | . | . | . | . | . | . | . | . | . | . | . | . | . | . | . | . | . | . |
| 21-225_226F7_LC | . | | . | M | . | . | . | . | . | . | . | . | . | . | . | . | . | . | . | . | . | . | . | . | . | . |
| 21-225_226H11_LC | . | | . | . | . | . | . | . | . | . | . | . | . | . | . | . | . | . | . | . | . | . | . | . | . | . |
| 21-225_22A4_LC | . | | . | . | . | N | . | . | . | . | . | . | . | . | . | . | . | . | . | . | . | . | . | . | . | . |
| 21-225_22F4_LC | . | | . | M | . | . | . | . | . | . | . | . | . | . | . | . | . | . | . | . | . | . | . | . | . | . |
| 21-225_23A10_LC | . | | . | . | . | . | . | . | . | . | . | . | . | . | . | . | . | . | . | . | . | . | . | . | . | . |
| 21-225_23E11_LC | . | | . | . | . | N | . | . | . | . | . | . | . | . | . | . | . | . | . | . | . | . | . | . | . | . |
| 21-225_23F12_LC | . | | . | . | . | N | . | . | . | . | . | . | . | . | . | . | . | . | . | . | . | . | . | . | . | . |
| 21-225_24B6_LC | . | | . | . | . | . | . | . | . | . | . | . | . | . | . | . | . | . | . | . | . | . | . | . | . | . |
| 21-225_25D12_LC | . | | . | . | . | . | . | . | . | . | . | . | . | . | . | . | . | . | . | . | . | . | . | . | . | . |
| 21-225_26A2_LC | . | | . | . | . | N | . | . | . | . | . | . | . | . | . | . | . | . | . | . | . | . | . | . | . | . |
| 21-225_28C7_LC | . | | . | . | . | N | T | . | . | . | . | . | . | . | . | . | . | . | . | . | . | . | . | . | . | . |
| 21-225_28F5_LC | . | | . | . | . | N | T | . | . | . | . | . | . | . | . | . | . | . | . | . | . | . | . | . | . | . |
| 21-225_2G4_LC | . | | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_32B6_LC | . | . | . | . | . | N | . | | | | | | | | | | | | | | | | | | | |
| 21-225_33C2_LC | . | . | . | . | . | N | . | | | | | | | | | | | | | | | | | | | |
| 21-225_44B3_LC | . | . | . | L | . | . | N | . | | | | | | | | | | | | | | | | | | |
| 21-225_44C10_LC | . | . | . | . | . | . | . | | | | | | | | | | | | | | | | | | | |
| 21-225_44D3_LC | . | . | . | . | . | . | . | | | | | | | | | | | | | | | | | | | |
| 21-225_44D5_LC | . | . | . | . | . | . | . | | | | | | | | | | | | | | | | | | | |
| 21-225_44F9_LC | . | . | . | N | . | . | N | . | | | | | | | | | | | | | | | | | | |
| 21-225_45B4_LC | . | . | . | . | . | . | . | | | | | | | | | | | | | | | | | | | |
| 21-225_45B8_LC | . | . | . | . | . | . | N | . | | | | | | | | | | | | | | | | | | |
| 21-225_46C3_LC | . | . | . | . | . | . | . | | | | | | | | | | | | | | | | | | | |
| 21-225_46D4_LC | . | . | . | . | . | . | . | | | | | | | | | | | | | | | | | | | |
| 21-225_48A9_LC | . | . | . | . | . | N | S | . | | | | | | | | | | | | | | | | | | |
| 21-225_48C1_LC | . | . | . | . | . | N | S | . | | | | | | | | | | | | | | | | | | |
| 21-225_48F2_LC | . | . | . | Q | . | . | . | | | | | | | | | | | | | | | | | | | |
| 21-225_4C12_LC | . | . | . | . | . | . | . | | | | | | | | | | | | | | | | | | | |
| 21-225_4C5_LC | . | . | . | . | . | . | . | | | | | | | | | | | | | | | | | | | |
| 21-225_50A12_LC | . | . | . | . | . | N | . | | | | | | | | | | | | | | | | | | | |
| 21-225_50H4_LC | . | . | . | . | . | . | . | | | | | | | | | | | | | | | | | | | |
| 21-225_51H8_LC | . | . | . | . | . | N | . | | | | | | | | | | | | | | | | | | | |
| 21-225_54H3_LC | . | . | . | . | Y | N | . | | | | | | | | | | | | | | | | | | | |
| 21-225_56C7_LC | . | . | . | . | . | N | . | | | | | | | | | | | | | | | | | | | |
| 21-225_57B8_LC | . | . | . | . | . | N | . | | | | | | | | | | | | | | | | | | | |
| 21-225_59B9_LC | . | . | . | . | . | N | . | | | | | | | | | | | | | | | | | | | |
| 21-225_59G7_LC | . | . | . | . | . | N | . | | | | | | | | | | | | | | | | | | | |
| 21-225_5E5_LC | . | . | . | . | . | . | . | | | | | | | | | | | | | | | | | | | |
| 21-225_5E5.003_LC | . | . | . | . | . | . | . | | | | | | | | | | | | | | | | | | | |
| 21-225_5E5.004_LC | . | . | . | . | . | . | . | | | | | | | | | | | | | | | | | | | |
| 21-225_5E5.005_LC | . | . | . | . | . | . | . | | | | | | | | | | | | | | | | | | | |
| 21-225_5E5.006_LC | . | . | . | . | . | . | . | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_5E5.011_LC | . | . | . | . | . | . | . | . | | | | | | | | | | | | | | | | | | |
| 21-225_5E5.012_LC | . | . | . | . | . | . | | | | | | | | | | | | | | | | | | | | |
| 21-225_5E5.015_LC | . | . | . | . | . | | | | | | | | | | | | | | | | | | | | | |
| 21-225_5E5.016_LC | . | . | . | . | . | | | | | | | | | | | | | | | | | | | | | |
| 21-225_5E5.017_LC | . | . | . | . | . | | | | | | | | | | | | | | | | | | | | | |
| 21-225_5E5.018_LC | . | . | . | . | . | | | | | | | | | | | | | | | | | | | | | |
| 21-225_5E5.019_LC | . | . | . | . | . | | | | | | | | | | | | | | | | | | | | | |
| 21-225_5E5.023_LC | . | . | . | . | . | | | | | | | | | | | | | | | | | | | | | |
| 21-225_5E5.024_LC | . | . | . | . | . | | | | | | | | | | | | | | | | | | | | | |
| 21-225_5E5.025_LC | . | . | . | . | . | | | | | | | | | | | | | | | | | | | | | |
| 21-225_60C3_LC | . | . | . | . | | N | | | | | | | | | | | | | | | | | | | | |
| 21-225_60E2_LC | . | . | . | . | | N | | | | | | | | | | | | | | | | | | | | |
| 21-225_61E6_LC | . | . | . | . | | N | | | | | | | | | | | | | | | | | | | | |
| 21-225_61H1_LC | . | . | . | . | | N | | | | | | | | | | | | | | | | | | | | |
| 21-225_62F7_LC | . | . | | | Y | N | | | | | | | | | | | | | | | | | | | | |
| 21-225_64A4_LC | . | . | | | | . | | | | | | | | | | | | | | | | | | | | |
| 21-225_64C8_LC | . | . | i | | | . | | | | | | | | | | | | | | | | | | | | |
| 21-225_66C10_LC | . | . | | | Y | N | | | | | | | | | | | | | | | | | | | | |
| 21-225_66D11_LC | . | . | i | V | | N | | | | | | | | | | | | | | | | | | | | |
| 21-225_8A11_LC | . | . | | | | N | | | | | | | | | | | | | | | | | | | | |
| 21-225_6G2_LC | . | . | i | | | N | | | | | | | | | | | | | | | | | | | | |
| 21-225_71A2_LC | . | . | i | M | | N | | | | | | | | | | | | | | | | | | | | |
| 21-225_73C9_LC | . | . | . | | | . | | | | | | | | | | | | | | | | | | | | |
| 21-225_7C9_LC | . | . | . | L | | . | | | | | | | | | | | | | | | | | | | | |
| 21-225_7F8_LC | . | . | . | | | . | T | | | | | | | | | | | | | | | | | | | |
| 21-225_7G2_LC | . | . | . | | N | | | | | | | | | | | | | | | | | | | | | |
| 21-225_7G4_LC | . | . | M | Q | | . | | | | | | | | | | | | | | | | | | | | |
| 21-225_94F3_LC | . | . | . | . | | . | | | | | | | | | | | | | | | | | | | | |
| 21-225_9A1_LC | . | . | i | . | | N | | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | | |
| CONSENSUS | Y | C | L | Q | H | Y | S | - | - | - | - | - | - | - | - | - | - | - | - | - | - | - | - | - | - | - |
| 21-225_9C11_LC | H | . | H | . | S | N | | | . | . | . | . | . | . | . | . | . | . | | . | . | . | . | . | . | . |
| 21-225_9D12_LC | . | . | . | . | . | N | | | . | . | . | . | . | . | . | . | . | . | | . | . | . | . | . | . | . |

4559

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | | K_FR4 | | | | |
| CONSENSUS | - | - | - | - | Y | P | R | T | F | G | Q | G | T | K | V | E | I | K | R |
| 21-225_10E9_LC | . | . | . | . | . | . | . | . | . | . | . | . | . | . | . | . | . | . | . |
| 21-225_12B12_LC | . | . | . | . | . | . | W | . | . | . | L | . | . | . | . | V | . | . | . |
| 21-225_12E6_LC | . | . | . | . | . | . | W | . | . | . | . | . | . | . | . | . | . | . | . |
| 21-225_12F2_LC | . | . | . | . | . | . | W | . | . | . | . | . | . | . | . | . | . | . | . |
| 21-225_147H5_LC | . | . | . | . | . | . | . | . | . | . | . | . | . | . | . | . | . | . | . |
| 21-225_148C9_LC | . | . | . | . | . | . | . | . | . | . | . | . | . | . | . | . | . | . | . |
| 21-225_149D10_LC | . | . | . | . | . | . | . | . | . | . | . | . | . | . | . | . | . | . | . |
| 21-225_14E10_LC | . | . | . | . | . | . | . | . | . | . | . | . | . | . | . | . | . | . | . |
| 21-225_158D4_LC | . | . | . | . | . | . | . | . | . | . | . | . | . | . | . | . | . | . | . |
| 21-225_158G1_LC | . | . | . | . | . | . | W | . | . | . | . | . | . | . | . | . | . | . | . |
| 21-225_159A3_LC | . | . | . | . | . | . | . | . | . | . | . | . | . | . | . | . | . | . | . |
| 21-225_159C8_LC | . | . | . | . | . | . | . | . | . | . | . | . | . | . | . | . | . | . | . |
| 21-225_159D8_LC | . | . | . | . | . | . | . | . | . | . | . | . | . | . | . | . | . | . | . |
| 21-225_15C11_LC | . | . | . | . | . | . | . | . | . | . | R | . | . | . | . | . | . | . | . |
| 21-225_160C4_LC | . | . | . | . | . | . | . | . | . | . | . | . | . | . | . | . | . | . | . |
| 21-225_160F2_LC | . | . | . | . | . | . | W | . | . | . | . | . | . | . | . | . | . | . | . |
| 21-225_160H1_LC | . | . | . | . | P | . | W | . | . | . | . | . | . | . | . | . | . | . | . |
| 21-225_161G12_LC | . | . | . | . | . | . | . | . | . | . | . | . | . | . | . | . | . | . | . |
| 21-225_169D11_LC | . | . | . | . | . | . | . | . | . | . | . | . | . | . | . | . | . | . | . |
| 21-225_169D7_LC | . | . | . | . | C | . | W | . | . | . | . | . | . | . | . | . | . | . | . |
| 21-225_16A5_LC | . | . | . | . | . | . | W | . | . | . | . | . | . | . | . | . | . | . | . |
| 21-225_16G7_LC | . | . | . | . | . | . | W | . | . | . | . | . | . | . | . | . | . | . | . |
| 21-225_16H4_LC | . | . | . | . | . | . | W | . | . | . | . | . | . | . | . | . | . | . | . |
| 21-225_170E4_LC | . | . | . | Y | P | . | W | . | . | . | . | . | . | . | . | D | . | . | . |
| 21-225_170F3_LC | . | . | . | . | . | . | . | . | . | . | . | . | . | . | . | . | . | . | . |
| 21-225_170H1_LC | . | . | . | . | N | . | W | . | . | . | . | . | . | . | . | . | S | . | . |
| 21-225_174G10_LC | . | . | . | . | . | . | . | . | . | . | . | . | . | . | . | . | . | . | . |
| 21-225_17C7_LC | . | . | . | . | . | . | W | . | . | . | . | . | . | . | . | . | . | . | . |
| 21-225_17H3_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | R | T | F | G | Q | G | T | K | V | E | I | K | R |
| 21-225_18A5_LC | . | . | . | . | . | . | W | . | . | . | . | . | . | . | . | . | . | . | . |
| 21-225_18C2_LC | . | . | . | . | . | . | W | . | . | . | L | . | . | . | . | V | . | . | . |
| 21-225_18F2_LC | . | . | . | . | . | . | W | . | . | . | . | . | . | . | . | . | . | . | . |
| 21-225_18H12_LC | . | . | . | . | . | . | W | . | . | . | L | . | . | . | . | V | . | . | . |
| 21-225_190C12_LC | . | . | . | . | . | . | W | . | . | . | . | . | . | . | . | . | . | . | . |
| 21-225_190G3_LC | . | . | . | . | . | . | W | . | . | . | . | . | . | . | . | . | . | . | . |
| 21-225_190H5_LC | . | . | . | . | . | . | W | . | . | . | . | . | . | . | . | . | . | . | . |
| 21-225_191A4_LC | . | . | . | . | . | . | W | . | . | . | . | . | . | . | . | . | . | . | . |
| 21-225_192F7_LC | . | . | . | . | . | . | W | . | . | . | . | . | . | . | . | . | . | Y | . |
| 21-225_196C12_LC | . | . | . | . | F | . | F | . | . | . | R | . | . | . | . | . | . | . | . |
| 21-225_19A10_LC | . | . | . | . | . | . | W | . | . | . | . | . | . | . | . | . | . | . | . |
| 21-225_200C9_LC | . | . | . | . | . | . | W | . | . | . | . | . | . | . | . | . | . | . | . |
| 21-225_201C5_LC | . | . | . | . | F | . | . | . | . | . | . | . | . | . | . | . | . | . | . |
| 21-225_203C10_LC | . | . | . | . | . | . | W | . | . | . | . | . | . | . | . | . | . | . | . |
| 21-225_203F7_LC | . | . | . | . | F | . | . | . | . | . | . | . | . | . | . | . | . | . | . |
| 21-225_204H3_LC | . | . | . | . | . | . | . | . | . | . | . | . | . | . | . | . | L | Q | . |
| 21-225_208B2_LC | . | . | . | . | . | . | . | . | . | . | . | . | . | . | . | . | . | . | . |
| 21-225_208F1_LC | . | . | . | . | F | . | . | . | . | . | . | . | . | . | . | . | . | . | . |
| 21-225_20B11_LC | . | . | . | . | . | . | . | . | . | . | . | . | . | . | . | . | . | . | . |
| 21-225_20B2_LC | . | . | . | . | . | . | . | A | . | . | . | . | . | . | . | D | . | . | . |
| 21-225_20D10_LC | . | . | . | . | . | . | W | . | . | . | . | . | . | . | . | . | . | . | . |
| 21-225_210G5_LC | . | . | . | . | . | . | . | . | . | . | . | . | . | . | . | . | . | . | . |
| 21-225_210G6_LC | . | . | . | . | F | . | . | . | . | . | . | . | . | . | . | . | . | . | . |
| 21-225_212B11_LC | . | . | . | . | . | . | . | . | . | . | . | . | . | . | . | . | . | . | . |
| 21-225_214G10_LC | . | . | . | . | . | . | . | . | . | . | . | . | . | . | . | . | . | . | . |
| 21-225_214G12_LC | . | . | . | . | . | . | . | . | . | . | . | . | . | . | . | . | . | . | . |
| 21-225_214H9_LC | . | . | . | . | . | . | . | . | . | . | . | . | . | . | . | . | . | . | . |
| 21-225_215E11_LC | . | . | . | . | . | . | . | . | . | . | . | . | . | . | . | . | . | . | . |
| 21-225_215E3_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | R | T | F | G | Q | G | T | K | V | E | I | K | R |
| 21-225_216E8_LC | . | . | . | . | . | . | . | . | . | . | . | . | . | . | . | . | . | . | . |
| 21-225_216H12_LC | . | . | . | . | . | . | . | . | . | . | . | . | . | . | . | . | . | . | . |
| 21-225_217E5_LC | . | . | . | . | . | . | . | . | . | . | . | . | . | . | . | . | . | . | . |
| 21-225_217G10_LC | . | . | . | . | . | . | . | . | . | . | . | . | . | . | . | . | . | . | . |
| 21-225_217H12_LC | . | . | . | . | . | . | . | . | . | . | . | . | . | . | . | . | . | . | . |
| 21-225_218C4_LC | . | . | . | . | . | . | . | . | . | . | . | . | . | . | . | . | . | . | . |
| 21-225_21F7_LC | . | . | . | . | . | . | W | . | . | . | . | . | . | . | . | . | . | . | . |
| 21-225_21G11_LC | . | . | . | . | . | . | W | . | . | . | L | . | . | . | . | V | . | . | . |
| 21-225_21G2_LC | . | . | . | . | . | . | W | . | . | . | L | . | . | . | . | V | . | . | . |
| 21-225_21H8_LC | . | . | . | . | . | . | W | . | . | . | . | . | . | . | . | . | . | . | . |
| 21-225_220F8_LC | . | . | . | . | . | . | . | . | . | . | . | . | . | . | . | . | . | . | . |
| 21-225_224E7_LC | . | . | . | . | . | . | . | . | . | . | . | . | . | . | . | . | . | . | . |
| 21-225_224F1_LC | . | . | . | . | . | . | . | . | . | . | . | . | . | . | . | . | . | . | . |
| 21-225_224F3_LC | . | . | . | . | . | . | . | A | . | . | . | . | . | . | . | . | . | . | . |
| 21-225_225A1_LC | . | . | . | . | . | . | . | . | . | . | . | . | . | . | . | . | . | . | . |
| 21-225_226C6_LC | . | . | . | . | . | . | . | A | . | . | . | . | . | . | . | . | . | Q | . |
| 21-225_226F7_LC | . | . | . | . | . | . | . | . | . | . | . | . | . | . | . | . | . | . | . |
| 21-225_226H11_LC | . | . | . | . | . | . | . | . | . | . | . | . | . | . | . | . | . | . | . |
| 21-225_22A4_LC | . | . | . | . | . | . | W | . | . | . | . | . | . | . | . | . | . | . | . |
| 21-225_22F4_LC | . | . | . | . | . | . | . | . | . | . | . | . | . | . | . | . | . | . | . |
| 21-225_23A10_LC | . | . | . | . | . | . | . | . | . | . | L | . | . | . | . | . | . | . | . |
| 21-225_23E11_LC | . | . | . | . | . | . | W | . | . | . | . | . | . | . | . | . | . | . | . |
| 21-225_23F12_LC | . | . | . | . | . | . | W | . | . | . | . | . | . | . | . | . | . | . | . |
| 21-225_24B6_LC | . | . | . | . | . | . | . | . | . | . | . | . | . | . | . | . | V | . | . |
| 21-225_25D12_LC | . | . | . | . | S | . | . | . | . | . | . | . | . | . | . | . | . | . | . |
| 21-225_26A2_LC | . | . | . | . | . | . | W | . | . | . | . | . | . | . | . | . | . | . | . |
| 21-225_28C7_LC | . | . | . | . | . | . | W | . | . | . | . | . | . | . | . | . | . | . | . |
| 21-225_28F5_LC | . | . | . | . | . | . | W | . | . | . | . | . | . | . | . | . | V | . | . |
| 21-225_2G4_LC | . | . | . | . | S | . | . | . | . | S | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | R | T | F | G | Q | G | T | K | V | E | I | K | R |
| 21-225_32B6_LC | . | . | . | . | . | . | W | . | . | . | . | . | . | . | . | . | . | . | . |
| 21-225_33C2_LC | . | . | . | . | . | . | W | . | . | . | . | . | . | . | . | . | . | . | . |
| 21-225_44B3_LC | . | . | . | . | . | . | . | . | . | . | . | . | . | . | . | . | . | . | . |
| 21-225_44C10_LC | . | . | . | . | . | . | . | . | . | . | . | . | . | . | . | . | . | . | . |
| 21-225_44D3_LC | . | . | . | . | . | . | . | . | . | . | . | . | . | . | . | . | . | . | . |
| 21-225_44D5_LC | . | . | . | . | F | . | W | . | . | . | . | . | . | . | . | . | . | . | . |
| 21-225_44F9_LC | . | . | . | . | . | . | . | . | . | . | . | . | . | . | . | . | . | T | . |
| 21-225_45B4_LC | . | . | . | . | . | . | . | . | . | . | . | . | . | . | . | . | . | . | . |
| 21-225_45B8_LC | . | . | . | . | . | . | . | . | . | . | . | . | . | . | . | . | . | . | . |
| 21-225_46C3_LC | . | . | . | . | . | . | . | . | . | . | . | . | . | . | . | . | . | . | . |
| 21-225_46D4_LC | . | . | . | . | F | . | W | . | . | . | . | . | . | . | . | . | . | . | . |
| 21-225_48A9_LC | . | . | . | . | . | . | W | . | . | . | . | . | . | . | . | . | . | . | . |
| 21-225_48C1_LC | . | . | . | . | . | . | W | . | . | . | . | . | . | . | . | . | . | . | . |
| 21-225_48F2_LC | . | . | . | . | . | . | . | . | . | . | . | . | . | . | . | . | . | . | . |
| 21-225_4C12_LC | . | . | . | . | F | . | . | . | . | . | . | . | . | . | . | . | . | . | . |
| 21-225_4C5_LC | . | . | . | . | . | . | . | . | . | . | . | . | . | . | . | . | . | . | . |
| 21-225_50A12_LC | . | . | . | . | . | . | W | . | . | . | . | . | . | . | . | . | . | . | . |
| 21-225_50H4_LC | . | . | . | . | . | . | . | . | . | . | . | . | . | . | . | . | . | . | . |
| 21-225_51H8_LC | . | . | . | . | . | . | W | . | . | . | . | . | . | . | . | . | . | . | . |
| 21-225_54H3_LC | . | . | . | . | . | . | W | . | . | . | . | . | . | . | . | . | . | . | . |
| 21-225_56C7_LC | . | . | . | . | . | . | W | . | . | . | . | . | . | . | . | . | . | . | . |
| 21-225_57B8_LC | . | . | . | F | F | . | W | . | . | . | . | . | . | . | . | . | . | . | . |
| 21-225_59B9_LC | . | . | . | . | . | . | W | . | . | . | . | . | . | . | . | . | . | . | . |
| 21-225_59G7_LC | . | . | . | . | . | . | W | . | . | . | . | . | . | . | . | . | . | . | . |
| 21-225_5E5_LC | . | . | . | . | . | . | . | . | . | . | . | . | . | . | . | . | V | . | . |
| 21-225_5E5.003_LC | . | . | . | . | . | . | . | . | . | . | . | . | . | . | . | . | V | . | . |
| 21-225_5E5.004_LC | . | . | . | . | . | . | . | . | . | . | . | . | . | . | . | . | V | . | . |
| 21-225_5E5.005_LC | . | . | . | . | . | . | . | . | . | . | . | . | . | . | . | . | V | . | . |
| 21-225_5E5.006_LC | . | . | . | . | . | . | . | . | . | . | . | . | . | . | . | . | V | . | . |

FIGURE 57

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | R | T | F | G | Q | G | T | K | V | E | I | K | R |
| 21-225_9C11_LC | . | . | . | . | T | . | L | . | . | . | . | . | . | . | . | . | . | . | . |
| 21-225_9D12_LC | . | . | . | . | . | . | W | . | . | . | . | . | . | . | . | . | . | . | . |

**FIGURE 57**

Table 99.    Consensus 19 - VK4|B3/JK1 (SEQ ID NO: 50316):

DIVMTQSPDSLAVSLGERATINCKSS--QSVLHSSNNNNYLAWYQQKPGQPPKLLIYW-------ASTRESGVPDRFSGSGSG--
TDFTLTISSLQAEDVAVYYCQQYYS---------------------TPPTFGQGTKVEIKR wherein:

K at position 24 can be substituted with R or M

S at position 26 can be substituted with G or R

S at position 30 can be substituted with T, N or I

V at position 31 can be substituted with I or A

H at position 33 can be substituted with Y, F, S, K, D, L or M

S at position 34 can be substituted with T, R, N, I or D

S at position 35 can be substituted with F or P

N at position 36 can be substituted with H

N at position 37 can be substituted with K, S, D or H

N at position 38 can be substituted with Y, K, H, R, A, F or W

N at position 39 can be substituted with H or Y

Y at position 40 can be substituted with S

L at position 41 can be substituted with F

A at position 42 can be substituted with T, V or G

A at position 67 can be substituted with T or S

S at position 68 can be substituted with F

T at position 69 can be substituted with I, K or S

FIGURE 57

R at position 70 can be substituted with W or L

E at position 71 can be substituted with K, A, D or R

S at position 72 can be substituted with T

Q at position 107 can be substituted with H or L

Q at position 108 can be substituted with H

Y at position 109 can be substituted with F

Y at position 110 can be substituted with F, H, N, L or S

S at position 111 can be substituted with N, R, D, I, T, C, E or K

T at position 135 can be substituted with S, I, V, A or Y

P at position 137 can be substituted with W, L, V, C, G, R or S

T at position 138 can be substituted with K or S

Xaa Xaa Xaa Gln Ser Xaa Leu Xaa Xaa Xaa Xaa Xaa Xaa Xaa Xaa Xaa (SEQ ID NO: 50133)

Trp Xaa Xaa Xaa Xaa Xaa Xaa (SEQ ID NO: 50229)

Xaa Xaa Xaa Xaa Xaa Xaa Pro Xaa Xaa (SEQ ID NO: 50134)

Lys Ser Ser Gln Ser Val Leu His Ser Ser Asn Asn Asn Asn Tyr Leu Ala (SEQ ID NO: 50618)

Trp Ala Ser Thr Arg Glu Ser (SEQ ID NO: 50619)

Gln Gln Tyr Tyr Ser Thr Pro Pro Thr (SEQ ID NO: 50620)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | S | P | D | S | L | A | V | S | L | G | E | R | A | T | I | N | C | K | S | S |
| 21-225_11E5_LC | . | . | . | . | . | . | . | . | . | . | . | . | . | . | . | . | . | . | . | . | L | . | . | . | . | . |
| 21-225_12F12_LC | . | . | . | . | . | . | . | . | . | . | . | . | . | . | . | . | . | . | . | . | . | . | . | . | . | . |
| 21-225_147E4_LC | . | . | . | . | . | . | . | . | . | . | . | . | . | . | . | . | . | . | . | . | . | . | . | . | . | . |
| 21-225_148F8_LC | . | . | . | . | . | . | L | . | . | . | . | . | . | . | . | . | . | . | . | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | . | . | . | . | S | . | . | . | . |
| 21-225_149H4_LC | . | . | . | . | . | . | . | . | . | . | . | . | . | . | . | . | . | . | . | . | . | . | . | . | . | . |
| 21-225_154A11_LC | . | . | . | . | . | . | . | . | . | . | . | . | . | . | . | . | . | . | . | . | . | . | . | P | . | . |
| 21-225_158B12_LC | . | . | . | . | . | . | . | . | . | . | P | . | . | . | . | . | . | . | . | . | . | . | . | . | . | . |
| 21-225_162F6_LC | . | . | . | . | . | . | . | . | . | . | . | . | . | . | . | . | . | . | . | . | . | . | . | . | . | . |
| 21-225_164A4_LC | . | . | . | . | . | . | Y | . | . | . | R | . | . | . | . | . | . | . | . | . | . | K | . | . | . | . |
| 21-225_164A7_LC | . | . | . | . | . | . | C | . | . | . | . | . | . | . | . | . | . | . | . | . | . | . | . | . | . | . |
| 21-225_170F6_LC | . | . | . | . | . | . | . | . | . | . | . | . | . | . | . | . | A | . | . | . | . | . | . | . | . | . |
| 21-225_174G5_LC | . | . | . | . | . | . | . | . | . | . | . | . | . | . | . | . | A | . | . | . | . | . | . | . | . | . |
| 21-225_175D10_LC | . | . | . | . | . | . | . | . | . | . | . | . | . | . | . | . | . | . | . | . | . | . | . | . | . | . |
| 21-225_177B12_LC | . | . | . | . | . | . | . | . | . | . | . | . | . | . | . | . | . | . | . | . | V | . | . | . | . | . |
| 21-225_181E5_LC | . | . | . | . | . | . | . | . | . | . | . | . | . | . | . | . | . | . | . | . | . | . | . | . | . | . |
| 21-225_190D7_LC | . | . | . | . | . | . | . | . | . | . | . | . | . | . | . | . | . | . | . | . | . | . | . | . | . | . |
| 21-225_191D8_LC | . | . | M | . | . | . | . | . | . | . | . | . | . | . | . | . | . | . | . | I | . | S | . | R | . | . |
| 21-225_193G4_LC | . | . | . | . | . | . | . | . | . | . | . | . | . | . | . | . | . | . | . | . | . | . | . | . | . | . |
| 21-225_196G8_LC | . | . | . | . | . | . | . | . | . | . | T | . | . | . | . | . | . | . | . | . | V | . | . | . | . | . |
| 21-225_197C6_LC | . | . | M | . | . | . | . | . | . | . | . | . | . | . | . | . | . | . | . | I | . | S | . | P | . | . |
| 21-225_197H4_LC | . | . | M | . | . | . | S | . | . | . | T | . | . | . | . | . | . | . | . | I | . | S | . | P | . | . |
| 21-225_200G7_LC | . | . | . | . | . | . | . | . | . | . | P | Y | A | . | . | . | . | . | . | . | V | K | . | . | . | . |
| 21-225_201F5_LC | . | . | . | . | . | . | . | B | . | . | . | . | . | . | . | . | . | . | . | . | . | . | . | . | . | . |
| 21-225_213H12_LC | . | . | . | . | . | . | . | . | . | . | . | . | . | . | . | . | . | . | . | . | . | T | . | . | . | . |
| 21-225_213H7_LC | . | . | . | . | . | . | . | . | . | . | . | . | . | . | . | . | . | . | . | . | . | . | . | . | . | . |
| 21-225_222H3_LC | . | . | . | . | . | . | . | . | . | . | . | . | . | . | . | . | . | . | . | . | . | . | . | . | . | . |
| 21-225_223G10_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | S | P | D | S | L | A | V | S | L | G | E | R | A | T | I | N | C | K | S | S |
| 21-225_224D8_LC | . | . | | | . | | . | | | | | | | . | . | . | | | | A | . | | | . | . | . |
| 21-225_224H5_LC | . | . | . | . | . | . | . | . | . | . | . | . | . | . | . | . | . | . | . | . | . | . | . | . | . | . |
| 21-225_225F11_LC | . | . | . | . | . | . | . | . | . | . | . | . | . | . | . | . | . | . | . | . | . | . | . | . | . | . |
| 21-225_225F5_LC | . | . | . | . | . | . | . | . | . | . | . | . | . | . | . | . | . | . | . | . | . | . | . | . | . | . |
| 21-225_226A12_LC | . | . | . | . | . | . | . | . | . | . | . | . | . | . | . | . | . | . | . | . | . | . | . | . | . | . |
| 21-225_226D11_LC | . | . | . | . | . | . | . | . | . | . | . | . | . | . | . | . | . | . | . | . | . | . | . | . | . | . |
| 21-225_226F6_LC | . | . | . | . | . | . | . | . | . | . | . | . | . | . | . | . | . | . | . | . | . | . | . | . | . | . |
| 21-225_227C7_LC | . | . | . | . | . | . | . | . | . | . | . | . | . | . | . | . | . | . | . | . | . | . | . | P | . | . |
| 21-225_23A12_LC | . | . | . | . | . | . | . | . | . | . | . | . | . | . | . | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | . | . | . | . | . | . | . | . | . | . | . | . | . | . | . | . | . | . | . | . | . | . | . | . | . | . |
| 21-225_25A4_LC | . | . | . | . | . | . | S | . | . | . | . | . | . | . | . | . | . | . | . | . | . | N | . | . | . | . |
| 21-225_25A4.001_LC | . | . | . | . | . | . | S | . | . | . | . | . | . | . | . | . | . | . | . | . | . | N | . | . | . | . |
| 21-225_25A4.001.001_LC | . | . | . | . | . | . | S | . | . | . | . | . | . | . | . | . | . | . | . | . | . | N | . | . | . | . |
| 21-225_25A4.001.002_LC | . | . | . | . | . | . | S | . | . | . | . | . | . | . | . | . | . | . | . | . | . | N | . | . | . | . |
| 21-225_25A4.001.003_LC | . | . | . | . | . | . | S | . | . | . | . | . | . | . | . | . | . | . | . | . | . | N | . | . | . | . |
| 21-225_25A4.001.004_LC | . | . | . | . | . | . | S | . | . | . | . | . | . | . | . | . | . | . | . | . | . | N | . | . | . | . |
| 21-225_25A4.001.005_LC | . | . | . | . | . | . | . | . | . | . | . | . | . | . | . | . | . | . | . | . | . | N | . | . | . | . |
| 21-225_25A4.001.006_LC | . | . | . | . | . | . | . | . | . | . | . | . | . | . | . | . | . | . | . | . | . | N | . | . | . | . |
| 21-225_25A4.001.007_LC | . | . | . | . | . | . | . | . | . | . | . | . | . | . | . | . | . | . | . | . | . | N | . | . | . | . |
| 21-225_25A4.001.008_LC | . | . | . | . | . | . | . | . | . | . | . | . | . | . | . | . | . | . | . | . | . | N | . | . | . | . |
| 21-225_25A4.001.013_LC | . | . | . | . | . | . | S | . | . | . | . | . | . | . | . | . | . | . | . | . | . | N | . | . | . | . |
| 21-225_25A4.001.019_LC | . | . | . | . | . | . | . | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . | . | . | . | . | . | . | . |
| 21-225_25A4.001.029_LC | . | . | . | . | . | . | S | . | . | . | . | . | . | . | . | . | . | . | . | . | . | N | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | . | . | . | S | . | . | . | . | . | . | . | . | . | . | . | . | . | . | . | . | . | . | . |
| 21-225_29H8_LC | . | . | . | . | . | . | . | . | . | . | . | . | . | . | . | . | . | . | . | . | . | . | . | N | . | . |

4569

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | V | M | T | Q | S | P | D | S | L | A | V | S | L | G | E | R | A | T | I | N | C | K | S | S |
| 21-225_43D8_LC | . | . | . | . | . | . | . | . | . | . | . | . | . | . | . | . | . | . | . | . | . | . | . | . | . | . |
| 21-225_4A2.001.023_LC | . | . | . | . | . | . | . | . | . | . | . | . | . | . | . | . | . | . | . | . | . | . | . | . | . | . |
| 21-225_4A2.001.028_LC | . | . | . | . | . | . | . | . | . | . | . | . | . | . | . | . | . | . | . | . | . | . | . | . | . | . |
| 21-225_4A2.001.029_LC | . | . | . | . | . | . | . | . | . | . | . | . | . | . | . | . | . | . | . | . | . | . | . | . | . | . |
| 21-225_4A2.001.031_LC | . | . | . | . | . | . | . | . | . | . | . | . | . | . | . | . | . | . | . | . | . | . | . | . | . | . |
| 21-225_56A1_LC | . | . | . | . | . | . | . | . | . | . | . | . | . | . | . | . | . | . | . | . | . | . | . | . | . | . |
| 21-225_57A11_LC | . | . | . | . | . | . | . | . | . | . | T | . | . | . | . | . | . | . | . | . | . | . | . | . | . | . |
| 21-225_58B9_LC | . | . | . | . | . | . | . | . | . | . | . | . | . | . | . | . | . | . | . | . | . | . | . | . | . | S |
| 21-225_59F10_LC | . | . | . | . | . | P | . | . | . | . | . | . | . | . | . | . | . | . | . | . | . | . | . | . | . | . |
| 21-225_61B5_LC | . | . | . | . | . | . | . | . | . | . | . | . | . | . | . | . | . | . | . | . | . | . | . | . | . | . |
| 21-225_70E7_LC | . | . | . | . | . | . | . | . | . | . | . | . | . | . | . | . | . | . | . | . | L | . | . | . | . | . |
| 21-225_74A6_LC | . | . | . | . | . | . | . | . | . | . | . | . | . | . | P | . | . | . | . | . | . | . | . | . | . | . |
| 21-225_74A7_LC | . | . | . | . | . | . | . | . | . | . | . | . | . | . | . | . | . | . | . | . | . | . | . | . | . | . |
| 21-225_74B1_LC | . | . | . | . | . | . | P | . | . | . | . | . | . | . | . | . | . | . | . | T | . | . | . | . | . | . |
| 21-225_74B10_LC | . | . | . | . | . | . | . | . | . | . | . | . | . | . | . | . | . | . | . | . | . | . | . | . | . | . |
| 21-225_74B5_LC | . | . | . | . | . | . | . | . | . | . | . | . | . | . | . | . | . | . | . | . | . | . | . | . | . | . |
| 21-225_74B7_LC | . | . | . | . | . | . | . | . | . | . | . | . | . | . | . | . | . | . | . | . | . | . | . | . | . | . |
| 21-225_74B8_LC | . | S | . | . | . | . | . | . | . | . | . | . | . | . | . | . | . | . | . | . | . | . | . | . | . | . |
| 21-225_74C1_LC | . | . | . | . | . | O | . | . | . | P | . | . | . | . | . | . | . | . | . | S | . | . | . | . | . | . |
| 21-225_74F6_LC | . | . | . | . | . | . | . | . | . | . | . | . | . | . | . | . | . | . | . | . | . | . | . | . | . | . |
| 21-225_74F9_LC | . | . | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | . | . | . | . | . | . | . | . | . | . | . | . | . | . | . |
| 21-225_75A10_LC | N | . | . | . | . | . | . | . | . | . | . | . | . | . | . | . | . | A | . | . | . | . | . | . | . | . |
| 21-225_75A7_LC | . | . | . | . | . | . | . | . | . | . | . | . | . | . | . | . | . | . | . | . | . | . | . | . | . | . |
| 21-225_75C4_LC | . | . | . | . | . | . | . | . | . | . | . | . | . | . | . | . | . | . | . | . | . | . | . | . | . | . |
| 21-225_75F9_LC | . | . | . | . | . | . | . | . | . | . | P | . | . | . | . | . | . | . | . | . | . | V | . | . | . | . |
| 21-225_76E11_LC | . | . | . | . | . | . | . | . | . | . | . | . | . | . | . | . | . | O | . | . | . | . | . | . | . | S |
| 21-225_76E8_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | V | M | T | Q | S | P | D | S | L | A | V | S | L | G | E | R | A | T | I | N | C | K | S | S |
| 21-225_76F3_LC | | | | | | | C | | | | P | | | | | | | | | | | | | | | |
| 21-225_76F5_LC | V | | | L | | | | | | | | | | | | | | | | | | | | | | |
| 21-225_76G2_LC | | | | | | | | | | | | | | | | | | | | | | | | | | |
| 21-225_77C10_LC | | | | | | | | | | G | | | | | | | | | | | | | | | | |
| 21-225_77C7_LC | | | | | | | | | | | | | | | | | | | | | | | | | | |
| 21-225_77E1_LC | | | | | | | | | | | | | | | | | | | | | | | | | | |
| 21-225_78C1_LC | | | | | | | | | | G | | | | | | | | | | | | | | | | |
| 21-225_78E11_LC | | | | | | | | | | | | | | | | | | | | | | | | | | |
| 21-225_78H12_LC | | | | | | | | | | | | | | | | | | | | | | | | | | |
| 21-225_79E9_LC | | | | | | | | | | | | | | | | | | | | | | | | | | |
| 21-225_79F12_LC | | | | | | | | | | | | | | | | | | | | | | | | | | |
| 21-225_79G10_LC | | | | | | | | | | | | | | | | | | | | | | | | | | |
| 21-225_79G6_LC | | | | | | | | | | G | | | | | | | | | | | | S | | M | | |
| 21-225_80D3_LC | | | | | | | | | | C | | | | | | | | | | | | | | | | |
| 21-225_80E9_LC | | | | | | | | | | C | | | | | | | | | | | | | | | | |
| 21-225_80H3_LC | | | | | | | | | | | | | | | | | | | | | T | | | | | |
| 21-225_81E5_LC | | | | | | | | | | | | | | | | | | | | | | | | | | |
| 21-225_81F9_LC | | | | | | | | | | C | | | | | | | | | | | | | | | | |
| 21-225_83F3_LC | | | | | | | | | | C | | | | | | | | | | | | | | | | |
| 21-225_83G3_LC | | | | | | | | | | C | | | | | | | | | | | | | | | | |
| 21-225_83G7_LC | | | | | | | | | | | | | | | | | | | | | | | I | | | |
| 21-225_84G7_LC | | | | | | | | | | | P | | | | | | | | | | | | I | | | |
| 21-225_85H2_LC | | S | M | | | | | | | | | | | | | | | | | | | | | | | |
| 21-225_85H9_LC | | | | | | | | | | | | | | | | | | | | | T | | | | | |
| 21-225_86E9_LC | | | | | | | C | | | | P | | | | | | | | | | | | | | | |
| 21-225_88A1_LC | N | | | | | | | | | | | | | | | | | A | | | | | | | | |
| 21-225_88B4_LC | | | | | | | | | | | | | | | | | | | | | | | | | | |
| 21-225_88C10_LC | | | | | | | | | | | | | | | | | | | | | | | | | | |
| 21-225_88G2_LC | | | | | | | | | | C | | | | | | | | | | | | | | | | |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | S | P | D | S | L | A | V | S | L | G | E | R | A | T | I | N | C | K | S | S |
| 21-225_90D9_LC | . | . | . | . | . | . | . | . | . | . | . | . | . | . | . | . | . | . | . | . | . | : | . | . | . | . |
| 21-225_91F1_LC | . | . | . | . | . | . | . | . | . | Q | . | . | . | . | . | . | . | . | . | . | . | . | . | . | . | . |
| 21-225_91G8_LC | . | . | . | . | . | . | . | . | . | Q | . | . | . | . | . | . | . | . | . | . | . | . | . | . | . | . |
| 21-225_92E6_LC | . | . | . | . | . | . | . | . | . | . | . | A | . | . | . | . | . | . | . | . | . | . | . | . | . | . |
| 21-225_92F12_LC | . | . | . | . | . | . | . | . | . | . | . | . | . | . | . | . | . | . | . | . | T | . | . | . | . | . |
| 21-225_94D2_LC | . | . | . | . | . | . | . | . | . | . | . | . | . | . | . | . | . | . | . | . | . | . | . | . | . | . |
| 21-225_94E11_LC | . | . | . | . | . | . | . | . | . | . | . | . | . | . | . | . | . | . | . | . | . | . | . | . | . | . |
| 21-225_94E12_LC | . | . | . | . | . | . | . | . | . | . | . | . | . | . | . | . | . | . | . | . | T | . | . | . | . | . |
| 21-225_95E12_LC | . | . | . | . | . | . | . | . | . | . | . | . | . | . | . | . | . | . | . | . | . | . | . | . | . | . |
| 21-225_95G4_LC | . | . | . | . | . | . | Q | . | . | . | . | . | . | . | . | . | . | . | . | . | . | . | . | . | . | . |
| 21-225_95H4_LC | . | . | . | . | . | . | Q | . | . | . | . | . | . | . | . | . | . | . | . | . | . | . | . | . | . | . |
| 21-225_96D10_LC | . | . | . | . | . | . | . | . | . | . | . | . | . | . | . | . | . | . | . | . | . | . | . | . | . | . |
| 21-225_96D5_LC | . | . | . | . | . | . | . | . | . | . | . | . | . | . | . | . | . | . | . | . | . | . | . | . | . | . |
| 21-225_96E2_LC | . | S | . | . | . | . | . | . | . | . | . | . | . | . | . | . | . | . | . | . | . | . | . | . | . | . |
| 21-225_96H5_LC | . | . | . | . | . | . | . | . | . | . | . | . | . | . | . | . | . | . | . | . | . | . | . | . | . | . |
| 21-225_97H4_LC | . | . | . | . | . | . | . | . | . | Q | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | V | L | H | S | S | N | N | N | N | Y | L | A | W | Y | Q | Q | K | P | G | Q | P | P |
| 21-225_11E5_LC | . | . | . | . | . | . | Y | . | . | . | . | . | . | . | . | T | . | . | . | . | . | . | . | . | P | . |
| 21-225_12F12_LC | . | . | . | T | . | . | S | . | . | . | . | . | R | . | . | . | . | . | . | . | . | . | . | . | . | . |
| 21-225_147E4_LC | . | . | . | . | . | . | . | . | . | . | . | Y | . | . | . | . | . | . | . | . | L | . | . | R | . | . |
| 21-225_148F8_LC | . | . | . | . | A | . | . | . | . | . | . | Y | . | . | . | . | . | . | . | . | . | . | . | . | . | . |
| 21-225_149A2_LC | . | . | . | . | . | . | Y | . | P | . | . | . | . | . | . | . | . | . | . | . | . | . | . | . | . | . |
| 21-225_149E3_LC | . | . | . | T | . | . | . | N | . | . | . | N | . | . | S | . | . | . | . | . | . | . | . | . | . | . |
| 21-225_149H4_LC | . | . | . | . | . | . | S | . | . | . | O | . | . | . | . | . | . | . | . | . | . | . | . | R | . | . |
| 21-225_154A11_LC | . | . | . | . | . | . | . | . | . | . | . | Y | . | . | . | . | . | . | . | . | . | . | . | . | . | . |
| 21-225_158B12_LC | . | . | N | . | . | . | . | . | . | . | . | . | . | . | . | T | . | . | . | . | R | . | . | . | . | . |
| 21-225_162F6_LC | . | . | N | . | . | . | . | . | . | . | . | . | . | . | . | T | . | . | . | . | R | . | . | . | . | . |
| 21-225_164A4_LC | . | . | . | . | . | . | Y | . | . | . | . | R | . | . | . | T | . | . | . | . | . | . | . | . | . | . |
| 21-225_164A7_LC | . | . | . | . | . | . | . | . | . | . | . | . | . | . | . | . | . | . | . | . | . | . | . | . | . | N |
| 21-225_170F6_LC | . | . | . | . | . | . | . | . | . | . | . | Y | . | . | . | . | . | . | . | . | R | . | . | . | . | . |
| 21-225_174G5_LC | . | . | . | . | . | . | . | . | . | . | . | Y | . | . | . | T | . | . | . | . | . | . | S | . | . | . |
| 21-225_175D10_LC | . | . | . | . | . | . | Y | . | . | . | . | . | . | . | . | . | . | . | . | . | . | . | . | . | . | . |
| 21-225_177B12_LC | . | . | . | . | . | . | . | . | . | . | . | . | . | . | . | T | . | . | . | . | . | . | . | . | . | . |
| 21-225_181E5_LC | . | . | . | . | . | . | . | . | . | . | . | Y | . | . | . | T | . | . | . | . | . | . | . | . | . | . |
| 21-225_190D7_LC | . | . | . | . | . | . | S | . | . | . | . | Y | . | . | . | V | . | . | . | . | . | . | . | . | . | . |
| 21-225_191D8_LC | . | . | . | . | . | . | . | . | . | . | . | Y | . | . | . | . | . | . | . | . | . | . | . | R | . | . |
| 21-225_193G4_LC | . | . | . | . | . | . | Y | . | . | . | . | Y | . | . | . | T | . | . | . | . | . | . | . | . | . | . |
| 21-225_196G8_LC | . | . | . | . | . | . | . | . | . | . | . | . | . | . | . | . | . | . | . | . | . | . | . | . | . | . |
| 21-225_197C6_LC | . | . | . | . | . | . | . | . | . | . | . | Y | . | . | . | . | . | . | . | . | . | . | . | R | . | . |
| 21-225_197H4_LC | . | . | . | . | . | . | . | . | P | . | . | Y | . | . | . | . | . | . | . | . | . | A | . | R | . | . |
| 21-225_200G7_LC | . | . | . | . | . | . | . | . | . | . | . | . | . | . | . | . | . | . | . | . | . | . | . | . | . | . |
| 21-225_201F5_LC | . | . | . | . | . | . | Y | . | . | . | . | R | . | . | . | V | . | . | . | . | . | . | . | . | . | . |
| 21-225_213H12_LC | . | . | N | . | . | . | R | T | . | . | . | R | . | . | . | . | . | . | . | . | . | . | . | . | . | . |
| 21-225_213H7_LC | . | . | N | . | . | . | O | I | . | . | . | R | . | S | . | G | . | P | . | . | . | . | . | . | . | . |
| 21-225_222H3_LC | . | . | . | . | . | . | R | . | . | M | . | R | . | . | . | . | . | . | . | . | . | . | . | . | . | . |
| 21-225_223G10_LC | . | . | . | . | I | . | L | . | . | . | . | R | . | . | . | . | . | R | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | | K_FR2 | |
| CONSENSUS | - | - | Q | S | V | L | H | S | S | N | N | N | N | Y | L | A | W | Y | Q | Q | K | P | G | Q | P | P |
| 21-225_224D8_LC | . | . | . | . | . | . | Y | . | . | . | . | N | . | . | . | . | . | . | . | . | . | . | . | . | . | . |
| 21-225_224H5_LC | . | . | . | . | . | . | Y | . | . | . | . | S | . | . | . | T | . | . | . | . | . | . | . | . | . | . |
| 21-225_225F11_LC | . | . | . | . | . | . | Y | . | . | . | . | Y | . | . | . | . | . | . | . | . | N | . | . | . | . | . |
| 21-225_225F5_LC | . | . | . | N | . | . | Y | N | . | . | . | . | . | . | . | . | . | . | . | . | . | . | . | . | . | . |
| 21-225_226A12_LC | . | . | . | N | . | . | Y | . | . | . | . | . | . | . | . | . | . | . | . | . | T | . | . | . | . | . |
| 21-225_226D11_LC | . | . | . | N | . | . | . | . | . | . | S | . | . | . | . | V | . | . | . | . | . | . | . | . | . | . |
| 21-225_226F6_LC | . | . | . | . | I | . | Y | . | . | . | . | Y | . | . | . | . | . | . | . | . | . | . | . | . | . | . |
| 21-225_227C7_LC | . | . | . | I | . | . | S | D | . | . | . | . | . | . | . | . | . | . | . | . | . | . | . | . | . | . |
| 21-225_23A12_LC | . | . | . | . | . | . | Y | . | . | . | . | . | . | . | . | . | . | . | . | . | . | . | . | . | . | . |
| 21-225_23G12_LC | . | . | . | . | I | . | S | . | . | . | . | . | . | . | . | . | . | . | . | . | . | . | . | . | . | . |
| 21-225_24E1_LC | . | . | . | . | . | . | Y | T | . | . | . | . | . | . | . | T | . | . | . | . | . | . | . | . | N | . |
| 21-225_25A4_LC | . | . | . | . | . | . | Y | . | . | N | . | . | . | . | . | . | . | . | . | . | . | . | . | . | . | . |
| 21-225_25A4.001_LC | . | . | . | . | . | . | Y | . | . | N | . | . | . | . | . | . | . | . | . | . | . | . | . | . | . | . |
| 21-225_25A4.001.001_LC | . | . | . | . | . | . | Y | . | . | N | . | . | . | . | . | . | . | . | . | . | . | . | . | . | . | . |
| 21-225_25A4.001.002_LC | . | . | . | . | . | . | Y | . | . | N | . | . | . | . | . | . | . | . | . | . | . | . | . | . | . | . |
| 21-225_25A4.001.003_LC | . | . | . | . | . | . | Y | . | . | N | . | . | . | . | . | . | . | . | . | . | . | . | . | . | . | . |
| 21-225_25A4.001.004_LC | . | . | . | . | . | . | Y | . | . | N | . | . | . | . | . | . | . | . | . | . | . | . | . | . | . | . |
| 21-225_25A4.001.005_LC | . | . | . | . | . | . | Y | . | . | N | . | . | . | . | . | . | . | . | . | . | . | . | . | . | . | . |
| 21-225_25A4.001.006_LC | . | . | . | . | . | . | Y | . | . | N | . | . | . | . | . | . | . | . | . | . | . | . | . | . | . | . |
| 21-225_25A4.001.007_LC | . | . | . | . | . | . | Y | . | . | N | . | . | . | . | . | . | . | . | . | . | . | . | . | . | . | . |
| 21-225_25A4.001.008_LC | . | . | . | . | . | . | Y | . | . | N | . | . | . | . | . | . | . | . | . | . | . | . | . | . | . | . |
| 21-225_25A4.001.013_LC | . | . | . | . | . | . | Y | . | . | N | . | . | . | . | . | . | . | . | . | . | . | . | . | . | . | . |
| 21-225_25A4.001.019_LC | . | . | . | . | . | . | Y | . | . | N | . | . | . | . | . | . | . | . | . | . | . | . | . | . | . | . |
| 21-225_25A4.001.020_LC | . | . | . | . | . | . | Y | . | . | N | . | . | . | . | . | . | . | . | . | . | . | . | . | . | . | . |
| 21-225_25A4.001.021_LC | . | . | . | . | . | . | Y | . | . | N | . | . | . | . | . | . | . | . | . | . | . | . | . | . | . | . |
| 21-225_25A4.001.029_LC | . | . | . | . | . | . | Y | . | . | N | . | . | . | . | . | . | . | . | . | . | . | . | . | . | . | . |
| 21-225_26C4_LC | . | . | . | . | . | . | Y | N | . | . | . | Y | . | . | . | . | . | . | . | . | N | . | . | . | . | . |
| 21-225_29E6_LC | . | . | . | . | . | . | Y | . | . | N | . | . | Y | . | . | T | . | . | . | . | . | . | . | . | . | N |
| 21-225_29H8_LC | . | . | . | T | I | . | . | . | . | . | . | Y | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | K_FR2 | | | |
| CONSENSUS | - | - | Q | S | V | L | H | S | S | N | N | N | N | Y | L | A | W | Y | Q | Q | K | P | G | Q | P | P |
| 21-225_43D8_LC | . | . | . | . | . | . | M | T | . | . | D | N | . | . | . | T | . | . | . | . | R | . | . | . | . | . |
| 21-225_4A2.001.023_LC | . | . | . | . | I | . | . | . | . | . | . | . | . | . | . | . | . | F | . | . | . | . | . | . | . | . |
| 21-225_4A2.001.028_LC | . | . | . | . | I | . | . | . | . | . | . | . | . | . | . | . | . | F | . | . | . | . | . | . | . | . |
| 21-225_4A2.001.029_LC | . | . | . | . | I | . | . | . | . | . | . | . | . | . | . | . | . | F | . | . | . | . | . | . | . | . |
| 21-225_4A2.001.031_LC | . | . | . | . | I | . | . | . | . | . | . | . | . | . | . | . | . | F | . | . | . | . | . | . | . | . |
| 21-225_56A1_LC | . | . | . | . | . | . | . | . | . | . | . | R | . | . | . | V | . | . | . | . | R | . | . | . | . | . |
| 21-225_57A11_LC | . | . | . | . | . | . | . | . | . | . | . | Y | . | . | . | . | . | . | . | . | R | . | . | . | . | . |
| 21-225_58B9_LC | . | . | . | . | I | . | Y | . | . | . | . | . | . | . | . | . | . | . | . | . | . | . | . | . | . | . |
| 21-225_59F10_LC | . | . | T | . | . | . | Y | R | . | . | . | Y | . | . | . | . | . | . | . | . | . | . | . | . | . | . |
| 21-225_61B5_LC | . | . | . | . | . | . | Y | . | . | . | . | . | . | S | . | T | . | . | . | . | L | . | . | . | . | . |
| 21-225_70E7_LC | . | . | . | . | . | . | Y | . | . | . | . | . | . | . | . | . | . | . | . | . | . | . | . | . | . | . |
| 21-225_74A6_LC | . | . | . | . | . | . | . | . | . | . | . | . | . | . | . | . | . | . | . | . | . | . | . | . | . | . |
| 21-225_74A7_LC | . | . | . | . | . | . | F | . | . | . | . | Y | . | . | . | . | . | . | . | . | R | . | . | . | . | . |
| 21-225_74B1_LC | . | . | . | . | . | . | . | . | R | . | . | Y | . | . | . | . | . | . | . | . | . | . | . | . | . | . |
| 21-225_74B10_LC | . | . | . | . | . | . | . | . | . | . | . | R | . | . | . | T | . | . | . | . | . | . | . | . | . | . |
| 21-225_74B5_LC | . | . | . | . | . | . | F | . | . | . | . | Y | . | . | . | . | . | . | . | . | R | . | . | . | . | . |
| 21-225_74B7_LC | . | . | T | . | . | . | . | . | . | . | . | Y | . | . | . | . | . | . | . | . | . | . | . | . | . | . |
| 21-225_74B8_LC | . | . | . | . | . | . | . | . | . | . | R | R | . | . | . | . | . | . | . | . | . | . | . | . | . | . |
| 21-225_74C1_LC | . | . | . | . | I | . | . | . | . | . | . | Y | . | . | . | . | . | . | . | . | . | . | . | N | . | R |
| 21-225_74F6_LC | . | . | N | I | . | S | . | . | . | . | . | R | . | . | . | T | . | . | . | . | . | . | . | . | . | . |
| 21-225_74F9_LC | . | . | . | . | . | . | Y | . | . | . | . | Y | . | . | . | . | . | . | . | . | . | . | . | . | . | . |
| 21-225_74G4_LC | . | . | . | . | . | . | Y | . | . | . | . | . | . | . | . | . | . | . | . | . | . | A | . | . | . | . |
| 21-225_74H2_LC | . | . | . | . | . | . | . | . | . | . | . | R | . | . | . | T | . | . | . | . | . | . | . | . | . | R |
| 21-225_75A10_LC | . | . | . | . | . | . | . | N | . | . | . | Y | . | . | . | T | . | . | . | . | . | . | . | . | . | . |
| 21-225_75A7_LC | . | . | T | . | . | . | . | . | . | . | . | Y | . | . | . | . | . | . | . | . | . | . | . | . | . | . |
| 21-225_75C4_LC | . | . | . | I | . | Y | . | . | . | . | . | . | . | . | . | . | . | F | . | . | . | . | . | . | . | . |
| 21-225_75F9_LC | . | . | . | . | . | . | . | N | . | . | . | . | . | . | . | . | . | . | . | . | . | . | . | . | . | . |
| 21-225_76E11_LC | . | . | . | . | . | . | . | . | . | . | . | Y | . | . | . | . | . | . | . | . | . | A | . | . | . | . |
| 21-225_76E8_LC | . | . | T | . | . | . | . | . | . | . | . | Y | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | V | L | H | S | S | N | N | N | N | Y | L | A | W | Y | Q | Q | K | P | G | Q | P | P |
| 21-225_76F3_LC | . | . | . | . | . | . | Ϝ | . | . | . | . | Y | . | . | . | . | . | . | . | . | Ꞧ | . | . | . | . | Ꞧ |
| 21-225_76F5_LC | . | . | . | . | . | . | . | . | . | . | S | Y | . | . | . | . | . | . | . | . | . | . | . | . | S | . |
| 21-225_76G2_LC | . | . | . | . | . | . | . | . | . | . | . | Y | . | . | . | . | . | . | . | . | Ꞧ | . | . | . | . | . |
| 21-225_77C10_LC | . | . | . | . | . | . | Y | T | . | . | . | . | . | . | . | . | . | . | . | . | . | . | . | . | . | . |
| 21-225_77C7_LC | . | . | T | . | . | . | . | . | . | . | . | Y | . | . | . | . | . | . | . | . | . | . | . | . | . | . |
| 21-225_77E1_LC | . | . | Ŧ | . | . | . | . | . | . | . | . | Y | . | . | . | . | . | . | . | . | . | . | . | . | . | . |
| 21-225_78C1_LC | . | . | . | . | . | . | Y | T | . | . | . | . | . | . | . | . | . | . | . | . | . | . | . | . | . | . |
| 21-225_78E11_LC | . | . | . | . | . | . | Y | . | Ϝ | . | . | Y | . | . | . | . | . | . | . | . | . | . | . | . | . | . |
| 21-225_78H12_LC | . | . | T | . | . | . | . | . | Ϝ | . | . | Y | . | . | . | . | . | . | . | . | . | . | . | . | . | . |
| 21-225_79E9_LC | . | . | . | Ꞁ | . | . | Y | . | . | . | . | . | . | . | . | . | . | Ϝ | . | . | . | . | . | . | . | . |
| 21-225_79F12_LC | . | . | . | Ꞁ | . | . | Y | . | . | . | . | Y | . | . | . | . | . | . | . | . | . | . | . | . | . | . |
| 21-225_79G10_LC | . | . | . | . | . | . | Ϝ | . | . | . | . | Y | . | . | . | . | . | . | . | . | Ꞧ | . | . | . | . | Ꞥ |
| 21-225_79G6_LC | . | . | . | . | . | . | . | Ϝ | . | . | . | Ꞧ | . | . | T | . | . | . | . | . | . | . | . | . | . | . |
| 21-225_80D3_LC | . | . | . | . | . | . | Y | T | . | . | . | . | . | . | . | . | . | . | . | . | . | . | . | . | . | . |
| 21-225_80E9_LC | . | . | . | . | . | . | Y | T | . | . | . | . | . | . | . | . | . | . | . | . | . | . | . | . | . | . |
| 21-225_80H3_LC | . | . | . | . | . | . | . | Ꞧ | . | . | . | Y | . | . | . | . | . | . | . | . | . | . | . | . | . | . |
| 21-225_81E5_LC | . | . | . | . | . | . | Ϝ | . | . | . | . | Y | . | . | . | . | . | . | . | . | Ꞧ | . | . | . | . | . |
| 21-225_81F9_LC | . | . | . | . | . | . | Y | T | . | . | . | . | . | . | . | . | . | . | . | . | . | . | . | . | . | . |
| 21-225_83F3_LC | . | . | . | . | . | . | Y | T | . | . | . | . | . | . | . | . | . | . | . | . | . | . | . | . | . | . |
| 21-225_83G3_LC | . | . | . | . | . | . | Y | T | . | . | . | . | . | . | . | . | . | . | . | . | . | . | . | . | . | . |
| 21-225_83G7_LC | . | . | T | . | . | . | . | . | . | . | . | Y | . | . | . | . | . | . | . | . | . | . | . | . | . | . |
| 21-225_84G7_LC | . | . | T | . | . | . | . | . | . | . | . | Y | . | . | . | . | . | . | . | . | . | . | . | . | . | . |
| 21-225_85H2_LC | . | . | . | . | . | . | . | . | . | . | Ꞧ | Ꞧ | . | . | . | . | . | . | . | . | . | . | . | . | . | . |
| 21-225_85H9_LC | . | . | . | . | . | . | . | Ꞧ | . | . | . | Y | . | . | . | . | . | . | . | . | . | . | . | . | . | . |
| 21-225_86E9_LC | . | . | . | . | . | . | . | Ꞧ | . | . | . | Y | . | . | . | . | . | . | . | . | . | . | . | . | . | . |
| 21-225_88A1_LC | . | . | . | . | . | . | . | . | . | . | . | Y | . | . | . | T | . | . | . | . | . | . | . | . | . | . |
| 21-225_88B4_LC | . | . | . | . | . | . | Y | Ꞁ | . | . | . | . | . | . | . | . | . | . | . | . | . | . | . | . | . | . |
| 21-225_88C10_LC | . | . | . | . | . | . | . | . | . | . | . | W | . | . | . | . | . | . | . | Ꞧ | . | . | . | . | . | . |
| 21-225_88G2_LC | . | . | . | . | . | . | Y | T | . | . | . | . | . | . | . | . | . | . | . | . | . | . | . | . | . | . |

4576

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | V | L | H | S | S | N | N | N | N | Y | L | A | W | Y | Q | Q | K | P | G | Q | P | P |
| 21-225_90D9_LC | . | . | . | Y | . | . | . | . | . | . | . | Y | . | . | . | . | . | . | . | . | . | . | . | . | . | . |
| 21-225_91F1_LC | . | . | . | . | . | . | Y | T | . | . | . | . | . | . | . | . | . | . | . | . | . | . | . | . | . | . |
| 21-225_91G8_LC | . | . | . | . | . | . | Y | T | . | . | . | . | . | . | . | . | . | . | . | . | . | . | . | . | . | . |
| 21-225_92E6_LC | . | . | N | I | . | . | S | . | . | . | . | K | . | . | . | T | . | . | . | . | . | . | . | . | . | . |
| 21-225_92F12_LC | . | . | . | . | . | . | . | R | . | . | . | Y | . | . | . | . | . | . | . | . | . | . | . | . | . | I |
| 21-225_94D2_LC | . | . | . | . | . | . | . | . | . | . | . | R | . | . | . | T | . | . | . | . | . | . | . | . | . | R |
| 21-225_94E11_LC | . | . | . | . | . | . | F | . | . | . | . | Y | . | . | . | . | . | . | . | . | R | . | . | . | . | . |
| 21-225_94E12_LC | . | . | . | . | . | . | . | R | . | . | . | Y | . | . | . | . | . | . | . | . | . | . | . | . | . | . |
| 21-225_95E12_LC | . | . | . | . | . | . | Y | . | . | . | . | A | . | . | . | . | . | . | . | . | . | . | . | . | . | . |
| 21-225_95G4_LC | . | . | . | . | . | . | F | R | . | . | . | Y | . | . | . | . | . | . | . | . | R | . | . | . | . | R |
| 21-225_95H4_LC | . | . | . | . | . | . | F | . | . | . | . | Y | . | . | . | . | . | . | . | . | R | . | . | . | . | R |
| 21-225_96D10_LC | . | . | N | I | . | . | S | . | . | . | . | K | . | . | . | T | . | . | . | . | . | . | . | . | . | . |
| 21-225_96D5_LC | . | . | . | . | . | . | . | . | . | . | . | Y | . | . | . | . | . | . | . | . | . | . | . | . | . | . |
| 21-225_96E2_LC | . | . | . | . | . | . | . | . | . | . | K | K | . | . | . | . | . | . | . | . | . | . | . | . | . | . |
| 21-225_96H5_LC | . | . | . | . | . | . | Y | . | . | . | S | K | . | . | . | . | . | . | . | . | . | . | . | . | . | . |
| 21-225_97H4_LC | . | . | . | . | . | . | Y | T | . | . | . | . | . | . | . | . | . | . | . | . | . | . | . | . | . | . |

4577

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | W | - | - | - | - | - | - | - | - | A | S | T | R | E | S | G | V | P | D | R | F |
| 21-225_11E5_LC | . | . | | | | | | | | | | | | | | | | | | . | | | | | | |
| 21-225_12F12_LC | N | . | | | | | | | | | | | | | | | | | A | | | | | | | |
| 21-225_147E4_LC | | | | | | | | | | | | | | | | | | | | | | | | | | |
| 21-225_148F8_LC | . | | | | | | | | | | | | | | | | | | X | | | | | | | |
| 21-225_149A2_LC | . | | | | | | | | | | | | | | | | | | | | | | | | | |
| 21-225_149E3_LC | . | | | | | | | | | | | | | | | | | L | X | . | | | | | | |
| 21-225_149H4_LC | . | | | | | | | | | | | | | | S | | | | | | | | | | | |
| 21-225_154A11_LC | | | | | | | | | | | | | | | | X | . | D | | . | | | | | | |
| 21-225_158B12_LC | . | | | | | | | | | | | | | | | | | | | | | | | | | |
| 21-225_162F6_LC | . | | | | | | | | | | | | | | | | | | | | | | | | | |
| 21-225_164A4_LC | . | . | F | | | | | | | | | | | | | | | | | | | | | | | |
| 21-225_164A7_LC | | | | | | | | | | | | | | | T | | | | | | | | | | | |
| 21-225_170F6_LC | . | V | | | X | | | | | | | | | | | | | | X | | | | | | | |
| 21-225_174G5_LC | . | | | | | | | | | | | | | | | | | | | | | | | | | |
| 21-225_175D10_LC | N | . | | | | | | | | | | | | | T | . | | | | | | | | | | |
| 21-225_177B12_LC | . | | | | | | | | | | | | | | | | | | | | | | | | | |
| 21-225_181E5_LC | | | | | | | | | | | | | | | | | | | | | | | | | | |
| 21-225_190D7_LC | . | | | | | | | | | | | | | | | | | | X | | | | | | | |
| 21-225_191D8_LC | . | | | | | | | | | | | | | | | | | | | | | | | | | |
| 21-225_193G4_LC | . | | | | | | | | | | | | | | | | | | X | | | | | | | |
| 21-225_196G8_LC | . | | | | | | | | | | | | | | | | | | | | | | | | | |
| 21-225_197C6_LC | N | | | | | | | | | | | | | | | | | | | | | | | | | |
| 21-225_197H4_LC | N | | | | | | | | | | | | | | | | | | | | | | | | | |
| 21-225_200G7_LC | . | | | | | | | | | | | | | | | | | | | | | | | | | |
| 21-225_201F5_LC | . | | | | | | | | | | | | | | | | | | | | | | | | | |
| 21-225_213H12_LC | . | V | | | | | | | | | | | | | | | | | | | | | | | | |
| 21-225_213H7_LC | . | | | | N | | | | | | | | | | | | | | | | | | | | | |
| 21-225_222H3_LC | Q | . | | | | | | | | | | | | | | | | | | Y | | | | | | |
| 21-225_223G10_LC | . | . | | | | | | | | | | | | | | | | | | | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | W | - | - | - | - | - | - | - | - | A | S | T | R | E | S | G | V | P | D | R | F |
| 21-225_224D8_LC | . | . | . | . | . | . | . | . | . | . | . | . | . | . | S | . | . | . | X | . | . | . | . | . | . | . |
| 21-225_224H5_LC | . | . | . | . | . | . | . | . | . | . | . | . | . | . | . | . | . | . | . | . | . | . | . | . | . | . |
| 21-225_225F11_LC | . | . | . | . | . | . | . | . | . | . | . | . | . | . | . | . | . | . | . | . | . | . | . | . | . | . |
| 21-225_225F5_LC | N | . | . | . | . | . | . | . | . | . | . | . | . | . | . | . | . | . | . | . | . | . | . | . | . | . |
| 21-225_226A12_LC | . | . | . | P | . | . | . | . | . | . | . | . | . | . | . | . | . | . | X | . | . | . | . | . | . | . |
| 21-225_226D11_LC | . | . | . | . | . | . | . | . | . | . | . | . | . | . | . | . | . | . | . | . | . | . | . | . | . | . |
| 21-225_226F6_LC | . | . | . | . | . | . | . | . | . | . | . | . | . | . | . | . | . | . | . | . | . | . | . | . | . | . |
| 21-225_227C7_LC | N | . | . | . | . | . | . | . | . | . | . | . | . | . | . | . | . | . | . | . | . | . | . | . | . | . |
| 21-225_23A12_LC | . | . | . | . | . | . | . | . | . | . | . | . | . | . | T | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | N | . | . | . | . | . | . | . | . | . | . | . | . | . | . | . | . | . | . | . | . | . | . | . | . | . |
| 21-225_25A4_LC | . | . | . | L | . | . | . | . | . | . | . | . | . | . | . | . | . | . | . | . | . | . | . | . | . | . |
| 21-225_25A4.001_LC | . | . | . | L | . | . | . | . | . | . | . | . | . | . | . | . | . | . | . | . | . | . | . | . | . | . |
| 21-225_25A4.001.001_LC | . | . | . | L | . | . | . | . | . | . | . | . | . | . | . | . | . | . | . | . | . | . | . | . | . | . |
| 21-225_25A4.001.002_LC | . | . | . | L | . | . | . | . | . | . | . | . | . | . | . | . | . | . | . | . | . | . | . | . | . | . |
| 21-225_25A4.001.003_LC | . | . | . | L | . | . | . | . | . | . | . | . | . | . | . | . | . | . | . | . | . | . | . | . | . | . |
| 21-225_25A4.001.004_LC | . | . | . | L | . | . | . | . | . | . | . | . | . | . | . | . | . | . | . | . | . | . | . | . | . | . |
| 21-225_25A4.001.005_LC | . | . | . | L | . | . | . | . | . | . | . | . | . | . | . | . | . | . | . | . | . | . | . | . | . | . |
| 21-225_25A4.001.006_LC | . | . | . | L | . | . | . | . | . | . | . | . | . | . | . | . | . | . | . | . | . | . | . | . | . | . |
| 21-225_25A4.001.007_LC | . | . | . | L | . | . | . | . | . | . | . | . | . | . | . | . | . | . | . | . | . | . | . | . | . | . |
| 21-225_25A4.001.008_LC | . | . | . | L | . | . | . | . | . | . | . | . | . | . | . | . | . | . | . | . | . | . | . | . | . | . |
| 21-225_25A4.001.013_LC | . | . | . | L | . | . | . | . | . | . | . | . | . | . | . | . | . | . | . | . | . | . | . | . | . | . |
| 21-225_25A4.001.019_LC | . | . | . | L | . | . | . | . | . | . | . | . | . | . | . | . | . | . | . | . | . | . | . | . | . | . |
| 21-225_25A4.001.020_LC | . | . | . | L | . | . | . | . | . | . | . | . | . | . | . | . | . | . | . | . | . | . | . | . | . | . |
| 21-225_25A4.001.021_LC | . | . | . | L | . | . | . | . | . | . | . | . | . | . | . | . | . | . | . | . | . | . | . | . | . | . |
| 21-225_25A4.001.029_LC | . | . | . | L | . | . | . | . | . | . | . | . | . | . | . | . | . | . | . | . | . | . | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | L | . | . | . | . | . | . | . | . | . | . | . | . | S | . | . | . | . | . | . | . | . | . |
| 21-225_29H8_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | K_CDR2 | | | | | | | | | | | | |
| CONSENSUS | K | L | L | I | Y | W | - | - | - | - | - | - | - | - | A | S | T | R | E | S | G | V | P | D | R | F |
| 21-225_43D8_LC | . | . | . | . | . | . | . | . | . | . | . | . | . | . | . | . | . | . | . | . | . | . | . | . | . | . |
| 21-225_4A2.001.023_LC | . | . | . | L | . | . | . | . | . | . | . | . | . | . | . | . | . | . | . | . | . | . | . | . | . | . |
| 21-225_4A2.001.028_LC | . | . | . | L | . | . | . | . | . | . | . | . | . | . | . | . | . | . | . | . | . | . | . | . | . | . |
| 21-225_4A2.001.029_LC | . | . | . | L | . | . | . | . | . | . | . | . | . | . | . | . | . | . | . | . | . | . | . | . | . | . |
| 21-225_4A2.001.031_LC | . | . | . | L | . | . | . | . | . | . | . | . | . | . | . | . | . | . | . | . | . | . | . | . | . | . |
| 21-225_56A1_LC | N | . | . | . | . | . | . | . | . | . | . | . | . | . | . | . | . | . | . | . | . | . | . | . | . | . |
| 21-225_57A11_LC | . | . | V | . | . | . | . | . | . | . | . | . | . | . | . | . | . | . | A | . | . | . | . | . | . | . |
| 21-225_58B9_LC | . | K | . | . | . | . | . | . | . | . | . | . | . | . | . | . | . | . | D | . | . | . | . | A | . | . |
| 21-225_59F10_LC | . | . | . | . | . | . | . | . | . | . | . | . | . | . | . | . | . | . | . | . | . | . | . | . | . | . |
| 21-225_61B5_LC | . | K | . | . | . | . | . | . | . | . | . | . | . | . | . | . | . | . | . | . | . | . | . | . | . | . |
| 21-225_70E7_LC | . | . | . | . | . | . | . | . | . | . | . | . | . | . | . | . | . | . | . | . | . | . | . | . | . | . |
| 21-225_74A6_LC | D | . | . | . | . | . | . | . | . | . | . | . | . | . | . | . | . | . | . | . | . | . | . | . | . | . |
| 21-225_74A7_LC | N | . | . | . | . | . | . | . | . | . | . | . | . | . | . | . | . | . | . | . | . | . | . | . | . | . |
| 21-225_74B1_LC | . | . | . | . | . | . | . | . | . | . | . | . | . | . | . | . | . | . | . | . | . | . | . | . | . | . |
| 21-225_74B10_LC | . | . | . | . | . | . | . | . | . | . | . | . | . | . | . | . | . | . | . | . | . | . | . | . | . | . |
| 21-225_74B5_LC | N | . | . | . | . | . | . | . | . | . | . | . | . | . | . | . | . | . | . | . | . | . | . | . | . | . |
| 21-225_74B7_LC | . | V | . | . | . | . | . | . | . | . | . | . | . | . | T | . | . | . | . | . | . | . | . | . | . | . |
| 21-225_74B8_LC | . | . | . | . | . | . | . | . | . | . | . | . | . | . | . | . | . | . | . | . | . | . | . | . | . | . |
| 21-225_74C1_LC | . | . | . | . | . | . | . | . | . | . | . | . | . | . | . | . | . | . | . | . | . | . | . | . | . | . |
| 21-225_74F6_LC | . | I | . | . | . | . | . | . | . | . | . | . | . | . | T | . | . | . | . | . | . | . | . | . | . | . |
| 21-225_74F9_LC | . | K | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | . | . | . | . | . | . | . | . | . | . | . | . | . | . | . |
| 21-225_75A10_LC | . | . | . | L | . | . | . | . | . | . | . | . | . | . | . | . | . | . | K | . | . | . | . | . | . | . |
| 21-225_75A7_LC | . | V | . | . | . | . | . | . | . | . | . | . | . | . | T | . | . | . | . | . | . | . | . | . | . | . |
| 21-225_75C4_LC | . | . | . | . | . | . | . | . | . | . | . | . | . | . | . | . | . | . | . | . | . | . | . | . | . | . |
| 21-225_75F9_LC | . | . | . | . | . | . | . | . | . | . | . | . | . | . | . | . | . | . | . | . | . | . | . | . | . | . |
| 21-225_76E11_LC | . | . | . | . | . | . | . | . | . | . | . | . | . | . | . | . | . | . | . | . | . | . | . | . | . | . |
| 21-225_76E8_LC | . | V | . | . | . | . | . | . | . | . | . | . | . | . | T | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | W | - | - | - | - | - | - | - | - | A | S | T | R | E | S | G | V | P | D | R | F |
| 21-225_76F3_LC | S | . | . | . | . | . | . | . | . | . | . | . | . | . | . | . | . | . | . | . | . | . | . | . | . | . |
| 21-225_76F5_LC | . | V | . | . | . | . | . | . | . | . | . | . | . | . | T | . | . | . | . | . | . | . | . | . | . | . |
| 21-225_76G2_LC | R | . | . | . | . | . | . | . | . | . | . | . | . | . | . | . | . | . | . | . | . | . | . | . | . | . |
| 21-225_77C10_LC | . | . | . | . | . | . | . | . | . | . | . | . | . | . | . | . | . | . | . | . | . | . | . | . | . | . |
| 21-225_77C7_LC | . | V | . | L | . | . | . | . | . | . | . | . | . | . | T | . | . | . | . | . | . | . | . | . | . | . |
| 21-225_77E1_LC | . | V | . | . | . | . | . | . | . | . | . | . | . | . | T | . | . | . | . | . | . | . | X | . | . | . |
| 21-225_78C1_LC | . | . | . | . | . | . | . | . | . | . | . | . | . | . | . | . | . | . | . | . | . | . | . | . | . | . |
| 21-225_78E11_LC | . | C | . | . | . | . | . | . | . | . | . | . | . | . | . | . | . | . | . | . | . | . | . | . | . | . |
| 21-225_78H12_LC | . | V | . | . | . | . | . | . | . | . | . | . | . | . | T | . | . | . | . | . | . | . | . | . | . | . |
| 21-225_79E9_LC | . | . | . | . | . | . | . | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | N | . | . | . | . | . | . | . | . | . | . | . | . | . | . | . | . | . | . | . | . | . | . | . | . | . |
| 21-225_79G6_LC | . | . | . | . | . | . | . | . | . | . | . | . | . | . | . | . | W | . | . | . | . | . | . | . | . | . |
| 21-225_80D3_LC | . | . | . | . | . | . | . | . | . | . | . | . | . | . | . | . | . | . | . | . | . | . | . | . | . | . |
| 21-225_80E9_LC | . | . | . | . | . | . | . | . | . | . | . | . | . | . | . | . | . | . | . | . | . | . | . | . | . | . |
| 21-225_80H3_LC | . | . | . | . | . | . | . | . | . | . | . | . | . | . | . | . | . | . | . | . | . | . | . | . | . | . |
| 21-225_81E5_LC | N | . | . | . | . | . | . | . | . | . | . | . | . | . | . | . | . | . | . | . | . | . | . | . | . | . |
| 21-225_81F9_LC | . | . | . | . | . | . | . | . | . | . | . | . | . | . | . | . | . | . | . | . | . | . | . | . | . | . |
| 21-225_83F3_LC | . | . | . | . | . | . | . | . | . | . | . | . | . | . | . | . | . | . | . | . | . | . | . | . | . | . |
| 21-225_83G3_LC | . | . | . | . | . | . | . | . | . | . | . | . | . | . | . | . | . | . | . | . | . | . | . | . | . | . |
| 21-225_83G7_LC | . | V | . | . | . | . | . | . | . | . | . | . | . | . | T | . | . | . | . | . | . | . | . | . | . | . |
| 21-225_84G7_LC | . | V | . | . | . | . | . | . | . | . | . | . | . | . | T | . | . | . | . | . | . | . | . | . | . | . |
| 21-225_85H2_LC | . | . | . | . | . | . | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | . | . | . | . | . | . |
| 21-225_86E9_LC | N | . | . | . | . | . | . | . | . | . | . | . | . | . | . | . | . | . | . | . | . | . | . | . | . | . |
| 21-225_88A1_LC | . | . | L | . | . | . | . | . | . | . | . | . | . | . | . | . | . | . | X | . | . | . | . | . | . | . |
| 21-225_88B4_LC | . | . | . | . | . | . | . | . | . | . | . | . | . | . | . | . | . | . | . | . | . | . | . | A | . | . |
| 21-225_88C10_LC | . | . | . | M | . | . | . | . | . | . | . | . | . | . | R | . | . | . | X | . | . | . | . | . | . | . |
| 21-225_88G2_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | K_CDR2 | | | | | | | | | | | | | | | |
| CONSENSUS | K | L | L | I | Y | W | - | - | - | - | - | - | - | - | A | S | T | R | E | S | G | V | P | D | R | F |
| 21-225_90D9_LC | . | V | . | | . | . | | . | . | . | | . | | | T | . | | | . | | | . | . | . | . | . |
| 21-225_91F1_LC | . | . | . | | . | . | | . | . | . | | . | | | . | . | | | . | | | . | . | . | . | . |
| 21-225_91G8_LC | . | . | . | | . | . | | . | . | . | | . | | | . | . | | | . | | | . | . | . | . | . |
| 21-225_92E6_LC | . | ! | . | | . | . | | . | . | . | | . | | | T | . | | | . | | | . | . | . | . | . |
| 21-225_92F12_LC | . | . | . | | . | . | | . | . | . | | . | | | . | . | | | . | | | . | . | . | . | . |
| 21-225_94D2_LC | . | . | . | | . | . | | . | . | . | | . | | | . | . | | | . | | | . | . | . | . | . |
| 21-225_94E11_LC | N | . | . | | . | . | | . | . | . | | . | | | . | . | | | . | | | . | . | . | . | . |
| 21-225_94E12_LC | . | . | . | | . | . | | . | . | . | | . | | | . | . | | | . | | | . | . | . | . | . |
| 21-225_95E12_LC | N | . | F | | . | . | | . | . | . | | . | | | . | . | | | . | | | . | . | . | . | . |
| 21-225_95G4_LC | N | . | . | | . | . | | . | . | . | | . | | | . | . | | | . | | | . | . | . | . | . |
| 21-225_95H4_LC | N | . | . | | | . | | . | . | . | | . | | | T | . | | | . | | | . | . | . | . | . |
| 21-225_96D10_LC | . | ! | . | | . | . | | . | . | . | | . | | | T | . | | | . | | | . | . | . | . | . |
| 21-225_96D5_LC | N | . | . | | . | . | | . | . | . | | . | | | . | . | | | . | | | . | . | . | . | . |
| 21-225_96E2_LC | . | . | . | | . | . | | . | . | . | | . | | | . | . | | W | . | | | . | . | . | . | . |
| 21-225_96H5_LC | . | . | . | | . | . | | . | . | . | | . | | | . | . | ! | | . | | | . | . | . | . | . |
| 21-225_97H4_LC | . | . | . | | . | . | | . | . | . | | . | | | . | . | | | . | | | . | . | . | . | . |

# EP 4 435 105 A2

## FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_11E5_LC | . | . | . | . | . | . | . | . | . | N | . | . | . | . | . | . | . | . | . | . | . | . | . | . | . | . |
| 21-225_12F12_LC | . | . | . | . | . | . | . | . | . | . | . | . | . | . | . | . | . | . | . | . | . | . | . | . | . | . |
| 21-225_147E4_LC | . | S | . | . | . | . | . | . | . | . | . | . | . | . | . | . | . | . | . | . | . | . | . | ? | . | . |
| 21-225_148F8_LC | . | . | . | . | . | . | . | . | . | . | . | . | . | . | . | . | . | . | . | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | . | . | . | . | . | . | . | . | . |
| 21-225_149H4_LC | . | . | . | . | . | . | . | . | . | . | . | . | . | . | . | . | . | . | . | . | . | . | . | . | . | . |
| 21-225_154A11_LC | . | . | . | . | . | . | . | . | . | . | . | . | . | . | . | . | . | . | . | . | . | . | . | . | . | . |
| 21-225_158B12_LC | . | . | . | . | . | . | . | . | . | S | . | . | . | . | . | . | . | . | . | . | . | . | . | . | . | . |
| 21-225_162F6_LC | . | . | . | . | . | . | . | . | . | S | . | . | . | . | . | . | . | . | . | . | . | . | . | . | . | . |
| 21-225_164A4_LC | . | . | . | C | . | . | . | . | . | . | . | . | . | . | . | . | . | V | . | . | . | . | . | . | . | . |
| 21-225_164A7_LC | . | . | . | Y | . | . | . | . | . | . | . | S | . | . | . | A | . | . | . | . | . | . | . | . | . | . |
| 21-225_170F6_LC | . | . | V | . | . | . | . | . | . | . | . | . | . | . | . | N | . | . | . | . | . | . | . | . | . | . |
| 21-225_174G5_LC | . | . | . | . | . | . | . | . | . | . | . | . | . | . | . | . | . | . | . | . | . | . | . | . | . | . |
| 21-225_175D10_LC | . | . | . | . | . | . | . | . | . | N | . | . | . | . | . | . | . | . | . | . | . | . | . | . | . | . |
| 21-225_177B12_LC | . | . | . | . | . | . | . | . | . | . | . | . | . | . | . | . | . | . | . | . | . | . | . | . | . | . |
| 21-225_181E5_LC | . | . | . | . | . | . | . | . | . | . | . | . | . | . | . | . | . | . | . | . | . | . | . | . | . | . |
| 21-225_190D7_LC | . | . | . | . | . | . | . | . | . | . | . | . | . | . | . | . | . | . | . | . | O | . | . | . | . | . |
| 21-225_191D8_LC | . | . | . | . | . | . | . | . | . | . | . | . | . | . | . | . | . | . | . | . | . | . | . | . | . | . |
| 21-225_193G4_LC | . | . | . | . | . | . | . | . | . | . | . | . | . | . | . | . | . | . | . | . | D | . | . | . | . | . |
| 21-225_196G8_LC | . | . | . | . | . | . | . | . | . | . | . | . | . | A | . | . | . | . | . | . | . | . | . | . | . | . |
| 21-225_197C6_LC | . | . | . | . | . | . | . | . | . | . | . | . | . | . | . | . | . | . | . | . | . | . | . | . | . | . |
| 21-225_197H4_LC | . | . | . | . | . | . | . | . | . | . | . | . | . | P | . | . | . | . | . | . | . | . | . | . | . | . |
| 21-225_200G7_LC | . | . | . | . | . | . | . | . | . | . | . | . | . | A | . | . | . | . | . | . | . | . | . | . | . | . |
| 21-225_201F5_LC | . | . | . | . | . | . | . | . | . | . | . | . | . | . | . | . | . | . | . | . | . | . | . | . | . | . |
| 21-225_213H12_LC | . | . | . | . | . | . | . | . | . | . | . | . | . | i | . | . | . | . | . | . | . | . | . | . | . | . |
| 21-225_213H7_LC | . | . | . | . | . | . | . | . | . | . | . | . | . | . | . | . | . | . | . | T | . | . | . | . | . | . |
| 21-225_222H3_LC | . | . | . | . | . | . | . | . | . | . | . | . | . | . | . | . | . | . | . | . | . | . | . | S | . | . |
| 21-225_223G10_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | D | F | T | L | T | I | S | S | L | Q | A | E | D | V | A | V | Y |
| 21-225_224D8_LC | . | . | . | . | . | . | . | . | . | . | . | . | . | . | . | . | . | . | . | . | . | . | . | . | . | . |
| 21-225_224H5_LC | . | . | . | . | . | . | . | . | . | . | . | . | . | . | . | N | . | . | . | . | . | . | . | . | . | . |
| 21-225_225F11_LC | . | . | . | . | . | . | . | . | . | . | . | . | . | . | . | . | . | . | . | . | . | . | . | . | . | . |
| 21-225_225F5_LC | . | . | . | . | . | . | . | . | . | . | . | . | . | . | . | . | . | . | . | . | . | . | . | . | . | . |
| 21-225_226A12_LC | . | . | . | . | . | . | . | . | . | . | . | . | . | . | . | . | . | . | . | . | . | . | . | . | . | . |
| 21-225_226D11_LC | . | . | . | . | . | . | . | . | . | . | . | . | . | . | . | . | . | . | . | . | . | . | . | . | . | . |
| 21-225_226F6_LC | . | . | . | . | . | . | . | . | . | . | . | . | . | . | . | . | . | . | . | . | . | . | . | . | . | . |
| 21-225_227C7_LC | . | . | . | . | . | . | . | . | . | . | . | . | . | . | . | . | . | . | . | . | . | . | . | . | . | . |
| 21-225_23A12_LC | . | . | . | . | . | . | . | . | . | . | . | . | . | . | . | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | . | . | . | . | . | . | . | . | . | . | . | . | . | . | . | . | . | . | . | . | . | . | . | . | . | . |
| 21-225_25A4_LC | . | . | . | . | . | . | . | . | . | . | . | . | . | . | . | . | . | . | . | . | . | . | . | . | . | . |
| 21-225_25A4.001_LC | . | . | . | . | . | . | . | . | . | . | . | . | . | . | . | . | . | . | . | . | . | . | . | . | . | . |
| 21-225_25A4.001.001_LC | . | . | . | . | . | . | . | . | . | . | . | . | . | . | . | . | . | . | . | . | . | . | . | . | . | . |
| 21-225_25A4.001.002_LC | . | . | . | . | . | . | . | . | . | . | . | . | . | . | . | . | . | . | . | . | . | . | . | . | . | . |
| 21-225_25A4.001.003_LC | . | . | . | . | . | . | . | . | . | . | . | . | . | . | . | . | . | . | . | . | . | . | . | . | . | . |
| 21-225_25A4.001.004_LC | . | . | . | . | . | . | . | . | . | . | . | . | . | . | . | . | . | . | . | . | . | . | . | . | . | . |
| 21-225_25A4.001.005_LC | . | . | . | . | . | . | . | . | . | . | . | . | . | . | . | . | . | . | . | . | . | . | . | . | . | . |
| 21-225_25A4.001.006_LC | . | . | . | . | . | . | . | . | . | . | . | . | . | . | . | . | . | . | . | . | . | . | . | . | . | . |
| 21-225_25A4.001.007_LC | . | . | . | . | . | . | . | . | . | . | . | . | . | . | . | . | . | . | . | . | . | . | . | . | . | . |
| 21-225_25A4.001.008_LC | . | . | . | . | . | . | . | . | . | . | . | . | . | . | . | . | . | . | . | . | . | . | . | . | . | . |
| 21-225_25A4.001.013_LC | . | . | . | . | . | . | . | . | . | . | . | . | . | . | . | . | . | . | . | . | . | . | . | . | . | . |
| 21-225_25A4.001.019_LC | . | . | . | . | . | . | . | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . | . | . | . | . | . | . | . |
| 21-225_25A4.001.029_LC | . | . | . | . | . | . | . | . | . | . | . | . | . | . | . | . | . | . | . | . | . | . | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | . | . | . | . | . | . | . | . | . | . | . | . | . | . | . | . | . | . | . | . | . | . | . |
| 21-225_29H8_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_43D8_LC | . | . | G | . | . | . | | | . | . | | . | | | . | . | . | G | | . | . | | . | . | . | . |
| 21-225_4A2.001.023_LC | . | . | . | . | . | . | | | . | . | | . | . | . | . | . | . | . | | P | . | . | . | . | . | . |
| 21-225_4A2.001.028_LC | . | . | . | . | . | . | | | . | . | | . | . | . | . | . | . | . | | P | . | . | . | . | . | . |
| 21-225_4A2.001.029_LC | . | . | . | . | . | . | | | . | . | | . | . | . | . | . | . | . | | P | . | . | . | . | . | . |
| 21-225_4A2.001.031_LC | . | . | . | . | . | . | | | . | . | | . | . | . | . | . | . | . | | P | . | . | . | . | . | . |
| 21-225_56A1_LC | . | . | . | . | . | . | | | . | . | | . | . | . | . | . | . | . | | . | . | . | . | . | . | . |
| 21-225_57A11_LC | . | . | . | . | . | . | | | . | . | | . | . | . | . | . | . | . | | . | . | . | M | . | . | . |
| 21-225_58B9_LC | . | . | . | . | . | . | | | . | . | | . | . | . | . | . | . | . | | . | . | . | . | . | . | . |
| 21-225_59F10_LC | . | . | . | . | . | . | | | . | . | | . | . | . | . | . | . | . | | . | . | . | . | . | . | . |
| 21-225_61B5_LC | . | . | . | . | . | . | | | . | . | | . | . | . | . | . | . | . | | . | . | . | . | . | . | . |
| 21-225_70E7_LC | . | . | . | . | . | . | | | . | . | | . | . | . | . | . | . | . | | . | . | . | . | . | . | . |
| 21-225_74A6_LC | . | . | . | . | . | . | | | . | . | | . | . | . | . | . | . | . | | . | . | . | . | . | . | . |
| 21-225_74A7_LC | . | . | . | . | . | . | | | . | . | | . | . | . | . | . | . | . | | A | . | . | . | . | . | . |
| 21-225_74B1_LC | . | . | . | . | . | . | | | . | . | | . | . | . | . | . | . | . | | . | . | . | . | . | . | . |
| 21-225_74B10_LC | . | . | . | . | . | . | | | . | . | S | . | . | . | . | G | . | . | | . | . | . | . | . | . | . |
| 21-225_74B5_LC | . | . | . | . | . | . | | | . | . | | . | . | . | . | . | . | . | | A | . | . | . | . | . | . |
| 21-225_74B7_LC | . | . | . | . | . | . | | | . | . | | . | . | . | . | . | . | . | | . | . | . | . | . | . | . |
| 21-225_74B8_LC | . | . | . | . | . | . | | | . | . | | . | . | . | . | . | . | . | | . | . | . | . | . | . | . |
| 21-225_74C1_LC | . | . | . | Y | . | . | | | . | . | | . | . | . | . | . | . | . | | . | . | . | . | . | . | . |
| 21-225_74F6_LC | . | . | . | P | . | . | | | . | . | | . | . | . | . | . | . | . | | . | . | . | . | . | . | . |
| 21-225_74F9_LC | . | . | . | . | . | . | | | . | . | | . | . | . | . | . | . | . | | . | . | . | . | . | . | . |
| 21-225_74G4_LC | . | . | . | . | . | . | | | . | . | | . | . | . | . | . | . | . | | . | . | . | . | . | . | . |
| 21-225_74H2_LC | . | . | . | . | . | . | | | . | . | S | . | . | . | . | G | . | . | | . | . | . | . | . | . | . |
| 21-225_75A10_LC | . | . | . | . | . | . | | | . | . | | . | . | . | . | . | . | . | | . | . | . | . | . | . | . |
| 21-225_75A7_LC | . | . | . | . | . | . | | | . | . | | . | . | . | . | . | . | . | | . | . | . | . | . | . | . |
| 21-225_75C4_LC | . | . | . | . | . | . | | | . | . | | . | . | . | . | . | . | . | | . | . | . | . | . | . | . |
| 21-225_75F9_LC | . | . | . | . | . | . | | | . | . | | . | . | . | . | . | . | . | | . | . | . | . | . | . | . |
| 21-225_76E11_LC | . | . | . | . | . | . | | | . | . | | . | . | . | . | . | . | . | | . | . | . | . | . | . | . |
| 21-225_76E3_LC | . | . | . | . | . | . | | | . | . | | . | . | . | . | . | . | . | | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_76F3_LC | . | . | . | . | . | . | . | . | . | . | . | . | . | . | . | . | . | . | . | . | A | . | . | . | . | . |
| 21-225_76F5_LC | . | . | . | . | . | . | . | . | . | . | . | . | . | . | . | . | . | . | . | . | . | . | . | . | . | . |
| 21-225_76G2_LC | . | . | . | . | . | . | . | . | . | . | . | . | . | . | . | . | . | . | . | V | . | . | . | . | . | . |
| 21-225_77C10_LC | . | . | . | . | . | . | . | . | . | . | . | . | . | . | . | . | . | . | . | . | . | . | . | . | . | . |
| 21-225_77C7_LC | . | . | . | . | . | . | . | . | . | . | . | . | . | . | . | . | . | . | . | . | . | . | . | . | . | . |
| 21-225_77E1_LC | . | . | . | . | . | . | . | . | . | . | . | . | . | . | . | . | . | . | . | . | . | . | . | . | . | . |
| 21-225_78C1_LC | . | . | . | . | . | . | . | . | . | . | . | . | . | . | . | . | . | . | . | . | . | . | . | . | . | . |
| 21-225_78E11_LC | . | . | . | . | . | . | . | . | . | . | . | . | . | . | . | . | . | . | . | . | . | . | . | . | . | . |
| 21-225_78H12_LC | . | . | . | . | . | . | . | . | . | . | . | . | . | . | . | . | . | . | . | . | . | . | . | . | . | . |
| 21-225_79E9_LC | . | . | . | C | . | . | . | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | . | . | . | . | . | . | . | . | . | . | . | . | . | . | . | . | . | . | . | . | A | . | . | . | . | . |
| 21-225_79G6_LC | . | . | . | . | . | . | . | . | . | . | S | . | ~ | . | . | G | . | . | . | . | . | . | . | . | . | . |
| 21-225_80D3_LC | . | . | . | . | . | . | . | . | . | . | . | . | . | . | . | . | . | . | . | . | . | . | . | . | . | . |
| 21-225_80E9_LC | . | . | . | . | . | . | . | . | . | . | . | . | . | . | . | . | . | . | . | . | . | . | . | . | . | . |
| 21-225_80H3_LC | . | . | . | . | . | . | . | . | . | . | . | . | . | . | . | . | . | . | . | . | . | . | . | . | . | . |
| 21-225_81E5_LC | . | . | . | . | . | . | . | . | . | . | . | . | . | . | . | . | . | . | . | . | A | . | . | . | . | . |
| 21-225_81F9_LC | . | . | . | . | . | . | . | . | . | . | . | . | . | . | . | . | . | . | . | . | . | . | . | . | . | . |
| 21-225_83F3_LC | . | . | . | . | . | . | . | . | . | . | . | . | . | . | . | . | . | . | . | . | . | . | . | . | . | . |
| 21-225_83G3_LC | . | . | . | . | . | . | . | . | . | . | . | . | . | . | . | . | . | . | . | . | . | . | . | . | . | . |
| 21-225_83G7_LC | . | . | . | . | . | . | . | . | . | . | . | . | . | . | . | . | . | . | . | . | . | . | . | . | . | . |
| 21-225_84G7_LC | . | . | . | . | . | . | . | . | . | . | . | . | . | . | . | . | . | . | . | . | . | . | . | . | . | . |
| 21-225_85H2_LC | . | . | . | . | . | . | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | . | . | . | . | . | . |
| 21-225_86E9_LC | . | . | . | . | . | . | . | . | . | . | . | . | . | . | . | . | . | . | . | . | A | . | . | . | . | . |
| 21-225_88A1_LC | . | . | . | . | . | . | . | . | . | . | . | . | . | . | . | . | . | . | . | . | . | . | . | . | . | . |
| 21-225_88B4_LC | . | . | . | . | . | . | . | . | . | . | . | . | . | . | . | . | . | . | . | . | . | . | . | . | . | . |
| 21-225_88C10_LC | . | . | G | . | . | . | . | . | N | . | . | . | . | . | . | . | . | . | . | . | . | . | . | . | . | . |
| 21-225_88G2_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_90D9_LC | . | . | . | . | . | . | | | . | . | . | . | . | . | . | . | . | . | . | . | . | . | . | . | . | . |
| 21-225_91F1_LC | . | . | . | . | . | . | | | . | . | . | . | . | . | . | . | . | . | . | . | . | . | . | . | . | . |
| 21-225_91G8_LC | . | . | . | . | . | . | | | . | . | . | . | . | . | . | . | . | . | . | . | . | . | . | . | . | . |
| 21-225_92E6_LC | . | . | . | . | S | . | | | . | . | . | . | . | . | . | . | . | . | . | . | . | . | . | . | . | . |
| 21-225_92F12_LC | . | . | . | . | . | . | | | . | . | . | . | . | . | . | . | . | . | . | . | . | . | . | . | . | . |
| 21-225_94D2_LC | . | . | . | . | . | . | | | . | . | S | . | . | . | . | Q | . | . | X | . | . | . | . | . | . | . |
| 21-225_94E11_LC | . | . | . | . | . | . | | | . | . | . | . | . | . | . | . | . | . | . | A | . | . | . | . | . | . |
| 21-225_94E12_LC | . | . | . | . | . | . | | | . | . | . | . | . | . | . | . | . | . | . | . | . | . | . | . | . | . |
| 21-225_95E12_LC | . | . | . | . | . | . | | | . | . | . | . | . | . | . | . | . | V | . | . | . | . | . | . | . | . |
| 21-225_95G4_LC | . | . | . | . | Y | . | | | . | . | . | . | . | . | . | . | . | V | . | A | . | . | . | . | . | . |
| 21-225_95H4_LC | . | . | . | . | . | . | | | . | . | . | . | . | . | . | . | . | . | . | A | . | . | . | . | . | . |
| 21-225_96D10_LC | . | . | . | . | S | . | | | . | . | . | . | . | . | . | . | . | . | . | . | . | . | . | . | . | . |
| 21-225_96D5_LC | . | . | . | . | . | . | | | . | . | . | . | . | . | . | . | . | . | . | A | . | . | . | . | . | . |
| 21-225_96E2_LC | . | . | . | . | . | . | | | . | . | . | . | . | . | . | . | . | . | . | . | . | . | . | . | . | . |
| 21-225_96H6_LC | . | . | . | . | . | . | | | . | . | . | . | . | S | . | . | . | M | . | . | . | . | . | . | . | . |
| 21-225_97H4_LC | . | . | . | . | . | . | | | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_11E5_LC | . | . | . | . | . | . | . | . | . | . | . | . | . | . | . | . | . | . | . | . | . | . | . | . | . | . |
| 21-225_12F12_LC | . | . | . | . | . | . | . | . | . | . | . | . | . | . | . | . | . | . | . | . | . | . | . | . | . | . |
| 21-225_147E4_LC | . | . | . | . | . | . | . | . | . | . | . | . | . | . | . | . | . | . | . | . | . | . | . | . | . | . |
| 21-225_148F8_LC | . | . | . | . | . | . | . | . | . | . | . | . | . | . | . | . | . | . | . | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | . | . | . | . | . | . | . | . | . |
| 21-225_149H4_LC | . | . | H | . | . | . | . | . | . | . | . | . | . | . | . | . | . | . | . | . | . | . | . | . | . | . |
| 21-225_154A11_LC | . | . | . | . | F | . | . | . | . | . | . | . | . | . | . | . | . | . | . | . | . | . | . | . | . | . |
| 21-225_158B12_LC | . | . | . | . | . | . | . | . | . | . | . | . | . | . | . | . | . | . | . | . | . | . | . | . | . | . |
| 21-225_162F6_LC | . | . | . | . | . | . | . | . | . | . | . | . | . | . | . | . | . | . | . | . | . | . | . | . | . | . |
| 21-225_164A4_LC | . | . | . | . | F | . | . | . | . | . | . | . | . | . | . | . | . | . | . | . | . | . | . | . | . | . |
| 21-225_164A7_LC | . | . | . | . | . | . | . | . | . | . | . | . | . | . | . | . | . | . | . | . | . | . | . | . | . | . |
| 21-225_170F6_LC | . | . | . | . | . | . | . | . | . | . | . | . | . | . | . | . | . | . | . | . | . | . | . | . | . | . |
| 21-225_174G5_LC | . | . | . | . | . | H | . | . | . | . | . | . | . | . | . | . | . | . | . | . | . | . | . | . | . | . |
| 21-225_175D10_LC | . | . | . | . | . | . | . | . | . | . | . | . | . | . | . | . | . | . | . | . | . | . | . | . | . | . |
| 21-225_177B12_LC | . | . | . | . | . | . | . | . | . | . | . | . | . | . | . | . | . | . | . | . | . | . | . | . | . | . |
| 21-225_181E5_LC | . | . | H | . | F | I | . | . | . | . | . | . | . | . | . | . | . | . | . | . | . | . | . | . | . | . |
| 21-225_190D7_LC | . | . | . | . | H | . | . | . | . | . | . | . | . | . | . | . | . | . | . | . | . | . | . | . | . | . |
| 21-225_191D8_LC | . | . | . | . | . | . | . | . | . | . | . | . | . | . | . | . | . | . | . | . | . | . | . | . | . | . |
| 21-225_193G4_LC | . | . | . | . | I | . | . | . | . | . | . | . | . | . | . | . | . | . | . | . | . | . | . | . | . | . |
| 21-225_196G8_LC | . | . | . | . | N | . | . | . | . | . | . | . | . | . | . | . | . | . | . | . | . | . | . | . | . | . |
| 21-225_197C6_LC | . | . | . | . | . | . | . | . | . | . | . | . | . | . | . | . | . | . | . | . | . | . | . | . | . | . |
| 21-225_197H4_LC | . | . | . | . | . | . | . | . | . | . | . | . | . | . | . | . | . | . | . | . | . | . | . | . | . | . |
| 21-225_200G7_LC | . | . | . | . | N | . | . | . | . | . | . | . | . | . | . | . | . | . | . | . | . | . | . | . | . | . |
| 21-225_201F5_LC | . | . | . | . | . | . | . | . | . | . | . | . | . | . | . | . | . | . | . | . | . | . | . | . | . | . |
| 21-225_213H12_LC | . | . | H | . | . | . | . | . | . | . | . | . | . | . | . | . | . | . | . | . | . | . | . | . | . | . |
| 21-225_213H7_LC | H | . | . | . | N | . | . | . | . | . | . | . | . | . | . | . | . | . | . | . | . | . | . | . | . | . |
| 21-225_222H3_LC | S | . | . | S | . | . | . | . | . | . | . | . | . | . | . | . | . | . | . | . | . | . | . | . | . | . |
| 21-225_223G10_LC | . | . | L | . | F | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_224D8_LC | . | . | . | . | . | Y | . | . | . | . | . | . | . | . | . | . | . | . | . | . | . | . | . | . | . | . |
| 21-225_224H5_LC | . | . | . | . | . | . | . | . | . | . | . | . | . | . | . | . | . | . | . | . | . | . | . | . | . | . |
| 21-225_225F11_LC | . | . | . | . | . | . | T | . | . | . | . | . | . | . | . | . | . | . | . | . | . | . | . | . | . | . |
| 21-225_225F5_LC | F | . | . | . | . | . | . | . | . | . | . | . | . | . | . | . | . | . | . | . | . | . | . | . | . | . |
| 21-225_226A12_LC | . | . | . | . | . | . | . | . | . | . | . | . | . | . | . | . | . | . | . | . | . | . | . | . | . | . |
| 21-225_226D11_LC | . | . | . | . | . | . | R | . | . | . | . | . | . | . | . | . | . | . | . | . | . | . | . | . | . | . |
| 21-225_226F6_LC | . | . | . | . | . | . | T | . | . | . | . | . | . | . | . | . | . | . | . | . | . | . | . | . | . | . |
| 21-225_227C7_LC | F | . | . | . | . | . | . | . | . | . | . | . | . | . | . | . | . | . | . | . | . | . | . | . | . | . |
| 21-225_23A12_LC | . | . | . | . | . | . | Q | . | . | . | . | . | . | . | . | . | . | . | . | . | . | . | . | . | . | . |
| 21-225_23G12_LC | F | . | . | . | . | S | . | . | . | . | . | . | . | . | . | . | . | . | . | . | . | . | . | . | . | . |
| 21-225_24E1_LC | . | . | . | F | . | . | . | . | . | . | . | . | . | . | . | . | . | . | . | . | . | . | . | . | . | . |
| 21-225_25A4_LC | . | . | . | . | . | . | . | . | . | . | . | . | . | . | . | . | . | . | . | . | . | . | . | . | . | . |
| 21-225_25A4.001_LC | . | . | . | . | . | . | . | . | . | . | . | . | . | . | . | . | . | . | . | . | . | . | . | . | . | . |
| 21-225_25A4.001.001_LC | . | . | . | . | . | . | . | . | . | . | . | . | . | . | . | . | . | . | . | . | . | . | . | . | . | . |
| 21-225_25A4.001.002_LC | . | . | . | . | . | . | . | . | . | . | . | . | . | . | . | . | . | . | . | . | . | . | . | . | . | . |
| 21-225_25A4.001.003_LC | . | . | . | . | . | . | . | . | . | . | . | . | . | . | . | . | . | . | . | . | . | . | . | . | . | . |
| 21-225_25A4.001.004_LC | . | . | . | . | . | . | . | . | . | . | . | . | . | . | . | . | . | . | . | . | . | . | . | . | . | . |
| 21-225_25A4.001.005_LC | . | . | . | . | . | . | . | . | . | . | . | . | . | . | . | . | . | . | . | . | . | . | . | . | . | . |
| 21-225_25A4.001.006_LC | . | . | . | . | . | . | . | . | . | . | . | . | . | . | . | . | . | . | . | . | . | . | . | . | . | . |
| 21-225_25A4.001.007_LC | . | . | . | . | . | . | . | . | . | . | . | . | . | . | . | . | . | . | . | . | . | . | . | . | . | . |
| 21-225_25A4.001.008_LC | . | . | . | . | . | . | . | . | . | . | . | . | . | . | . | . | . | . | . | . | . | . | . | . | . | . |
| 21-225_25A4.001.013_LC | . | . | . | . | . | . | . | . | . | . | . | . | . | . | . | . | . | . | . | . | . | . | . | . | . | . |
| 21-225_25A4.001.019_LC | . | . | . | . | . | . | . | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . | . | . | . | . | . | . | . |
| 21-225_25A4.001.029_LC | . | . | . | . | . | . | . | . | . | . | . | . | . | . | . | . | . | . | . | . | . | . | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | . | . | . | . | . | . | . | . | . | . | . | . | . | . | . | . | . | . | . | . | . | . | . |
| 21-225_29H8_LC | . | . | . | . | . | . | Q | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | K_CDR3 | | | | | | | | | | |
| CONSENSUS | Y | C | Q | Q | Y | Y | S | - | - | - | - | - | - | - | - | - | - | - | - | - | - | - | - | - | - | - |
| 21-225_43D8_LC | . | . | . | . | . | . | . | . | . | . | . | . | . | . | . | . | . | . | . | . | . | . | . | . | . | . |
| 21-225_4A2.001.023_LC | . | . | . | . | . | . | . | . | . | . | . | . | . | . | . | . | . | . | . | . | . | . | . | . | . | . |
| 21-225_4A2.001.028_LC | . | . | . | . | . | . | . | . | . | . | . | . | . | . | . | . | . | . | . | . | . | . | . | . | . | . |
| 21-225_4A2.001.029_LC | . | . | . | . | . | . | . | . | . | . | . | . | . | . | . | . | . | . | . | . | . | . | . | . | . | . |
| 21-225_4A2.001.031_LC | . | . | . | . | . | . | N | . | . | . | . | . | . | . | . | . | . | . | . | . | . | . | . | . | . | . |
| 21-225_56A1_LC | . | . | . | . | . | . | . | . | . | . | . | . | . | . | . | . | . | . | . | . | . | . | . | . | . | . |
| 21-225_57A11_LC | . | . | . | . | . | . | . | . | . | . | . | . | . | . | . | . | . | . | . | . | . | . | . | . | . | . |
| 21-225_58B9_LC | . | . | . | . | . | . | . | . | . | . | . | . | . | . | . | . | . | . | . | . | . | . | . | . | . | . |
| 21-225_59F10_LC | . | . | . | . | . | N | . | . | . | . | . | . | . | . | . | . | . | . | . | . | . | . | . | . | . | . |
| 21-225_61B5_LC | . | . | . | . | . | . | . | . | . | . | . | . | . | . | . | . | . | . | . | . | . | . | . | . | . | . |
| 21-225_70E7_LC | . | . | . | . | . | . | N | . | . | . | . | . | . | . | . | . | . | . | . | . | . | . | . | . | . | . |
| 21-225_74A6_LC | . | . | . | . | . | N | . | . | . | . | . | . | . | . | . | . | . | . | . | . | . | . | . | . | . | . |
| 21-225_74A7_LC | . | . | . | . | . | N | . | . | . | . | . | . | . | . | . | . | . | . | . | . | . | . | . | . | . | . |
| 21-225_74B1_LC | . | . | . | . | . | . | . | . | . | . | . | . | . | . | . | . | . | . | . | . | . | . | . | . | . | . |
| 21-225_74B10_LC | . | . | . | . | . | . | . | . | . | . | . | . | . | . | . | . | . | . | . | . | . | . | . | . | . | . |
| 21-225_74B5_LC | . | . | . | . | . | . | . | . | . | . | . | . | . | . | . | . | . | . | . | . | . | . | . | . | . | . |
| 21-225_74B7_LC | . | . | . | . | . | N | . | . | . | . | . | . | . | . | . | . | . | . | . | . | . | . | . | . | . | . |
| 21-225_74B8_LC | . | . | . | . | . | N | . | . | . | . | . | . | . | . | . | . | . | . | . | . | . | . | . | . | . | . |
| 21-225_74C1_LC | . | . | . | . | . | . | . | . | . | . | . | . | . | . | . | . | . | . | . | . | . | . | . | . | . | . |
| 21-225_74F6_LC | . | . | . | . | . | N | . | . | . | . | . | . | . | . | . | . | . | . | . | . | . | . | . | . | . | . |
| 21-225_74F9_LC | S | . | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | . | . | . | . | . | . | . | . | . | . | . | . | . | . | . |
| 21-225_75A10_LC | N | . | . | . | . | . | . | . | . | . | . | . | . | . | . | . | . | . | . | . | . | . | . | . | . | . |
| 21-225_75A7_LC | . | . | . | . | . | N | . | . | . | . | . | . | . | . | . | . | . | . | . | . | . | . | . | . | . | . |
| 21-225_75C4_LC | . | . | . | . | . | . | I | . | . | . | . | . | . | . | . | . | . | . | . | . | . | . | . | . | . | . |
| 21-225_75F9_LC | . | . | . | . | . | . | . | . | . | . | . | . | . | . | . | . | . | . | . | . | . | . | . | . | . | . |
| 21-225_76E11_LC | . | . | . | . | . | . | . | . | . | . | . | . | . | . | . | . | . | . | . | . | . | . | . | . | . | . |
| 21-225_76E8_LC | . | . | . | . | . | N | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_76F3_LC | . | . | . | . | . | M | . | . | . | . | . | . | . | . | . | . | . | . | . | . | . | . | . | . | . | . |
| 21-225_76F5_LC | . | . | . | . | . | . | . | . | . | . | . | . | . | . | . | . | . | . | . | . | . | . | . | . | . | . |
| 21-225_76G2_LC | . | . | . | . | . | . | . | . | . | . | . | . | . | . | . | . | . | . | . | . | . | . | . | . | . | . |
| 21-225_77C10_LC | . | . | . | M | . | . | N | . | . | . | . | . | . | . | . | . | . | . | . | . | . | . | . | . | . | . |
| 21-225_77C7_LC | . | . | . | . | . | P | . | . | . | . | . | . | . | . | . | . | . | . | . | . | . | . | . | . | . | . |
| 21-225_77E1_LC | . | . | R | . | . | L | . | . | . | . | . | . | . | . | . | . | . | . | . | . | . | . | . | . | . | . |
| 21-225_78C1_LC | . | . | . | M | . | . | N | . | . | . | . | . | . | . | . | . | . | . | . | . | . | . | . | . | . | . |
| 21-225_78E11_LC | S | . | . | . | . | . | . | . | . | . | . | . | . | . | . | . | . | . | . | . | . | . | . | . | . | . |
| 21-225_78H12_LC | . | . | . | . | . | P | . | . | . | . | . | . | . | . | . | . | . | . | . | . | . | . | . | . | . | . |
| 21-225_79E9_LC | . | . | . | . | . | . | I | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | . | . | . | . | . | R | . | . | . | . | . | . | . | . | . | . | . | . | . | . | . | . | . | . | . | . |
| 21-225_79G6_LC | . | . | . | . | . | . | . | . | . | . | . | . | . | . | . | . | . | . | . | . | . | . | . | . | . | . |
| 21-225_80D3_LC | . | . | . | M | . | N | E | . | . | . | . | . | . | . | . | . | . | . | . | . | . | . | . | . | . | . |
| 21-225_80E9_LC | . | . | . | . | . | . | N | . | . | . | . | . | . | . | . | . | . | . | . | . | . | . | . | . | . | . |
| 21-225_80H3_LC | . | . | . | . | . | . | . | . | . | . | . | . | . | . | . | . | . | . | . | . | . | . | . | . | . | . |
| 21-225_81E5_LC | . | . | . | . | . | . | . | . | . | . | . | . | . | . | . | . | . | . | . | . | . | . | . | . | . | . |
| 21-225_81F9_LC | . | R | . | M | . | N | D | . | . | . | . | . | . | . | . | . | . | . | . | . | . | . | . | . | . | . |
| 21-225_83F3_LC | . | . | . | M | . | N | D | . | . | . | . | . | . | . | . | . | . | . | . | . | . | . | . | . | . | . |
| 21-225_83G3_LC | . | . | . | M | . | . | N | . | . | . | . | . | . | . | . | . | . | . | . | . | . | . | . | . | . | . |
| 21-225_83G7_LC | . | . | . | . | . | P | . | . | . | . | . | . | . | . | . | . | . | . | . | . | . | . | . | . | . | . |
| 21-225_84G7_LC | . | . | . | . | . | P | . | . | . | . | . | . | . | . | . | . | . | . | . | . | . | . | . | . | . | . |
| 21-225_85H2_LC | . | . | . | . | . | R | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | . | . | . | . | . | . |
| 21-225_86E9_LC | . | . | . | . | . | M | . | . | . | . | . | . | . | . | . | . | . | . | . | . | . | . | . | . | . | . |
| 21-225_88A1_LC | P | . | . | . | . | . | . | . | . | . | . | . | . | . | . | . | . | . | . | . | . | . | . | . | . | . |
| 21-225_88B4_LC | . | . | . | . | . | . | . | . | . | . | . | . | . | . | . | . | . | . | . | . | . | . | . | . | . | . |
| 21-225_88C10_LC | . | . | . | . | . | . | R | . | . | . | . | . | . | . | . | . | . | . | . | . | . | . | . | . | . | . |
| 21-225_88G2_LC | . | . | . | . | . | . | N | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_90D9_LC | . | . | . | . | . | L | . | . | . | . | . | . | . | . | . | . | . | . | . | . | . | . | . | . | . | . |
| 21-225_91F1_LC | . | . | . | H | . | . | N | . | . | . | . | . | . | . | . | . | . | . | . | . | . | . | . | . | . | . |
| 21-225_91G8_LC | . | . | . | . | . | . | T | . | . | . | . | . | . | . | . | . | . | . | . | . | . | . | . | . | . | . |
| 21-225_92E6_LC | . | . | . | . | . | F | . | . | . | . | . | . | . | . | . | . | . | . | . | . | . | . | . | . | . | . |
| 21-225_92F12_LC | . | . | . | . | . | . | . | . | . | . | . | . | . | . | . | . | . | . | . | . | . | . | . | . | . | . |
| 21-225_94D2_LC | . | . | . | . | . | H | . | . | . | . | . | . | . | . | . | . | . | . | . | . | . | . | . | . | . | . |
| 21-225_94E11_LC | . | . | . | . | . | H | . | . | . | . | . | . | . | . | . | . | . | . | . | . | . | . | . | . | . | . |
| 21-225_94E12_LC | . | . | . | . | . | . | . | . | . | . | . | . | . | . | . | . | . | . | . | . | . | . | . | . | . | . |
| 21-225_95E12_LC | . | . | . | . | . | . | . | . | . | . | . | . | . | . | . | . | . | . | . | . | . | . | . | . | . | . |
| 21-225_95G4_LC | . | . | . | . | . | H | . | . | . | . | . | . | . | . | . | . | . | . | . | . | . | . | . | . | . | . |
| 21-225_95H4_LC | . | . | . | . | . | H | . | . | . | . | . | . | . | . | . | . | . | . | . | . | . | . | . | . | . | . |
| 21-225_96D10_LC | . | . | . | . | . | F | . | . | . | . | . | . | . | . | . | . | . | . | . | . | . | . | . | . | . | . |
| 21-225_96D5_LC | . | . | . | . | . | . | . | . | . | . | . | . | . | . | . | . | . | . | . | . | . | . | . | . | . | . |
| 21-225_96E2_LC | . | . | . | . | . | H | . | . | . | . | . | . | . | . | . | . | . | . | . | . | . | . | . | . | . | . |
| 21-225_96H5_LC | . | . | . | . | . | . | . | . | . | . | . | . | . | . | . | . | . | . | . | . | . | . | . | . | . | . |
| 21-225_97H4_LC | . | . | . | H | . | N | D | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | T | P | P | T | F | G | Q | G | T | K | V | E | I | K | R |
| 21-225_11E5_LC | . | . | . | . | . | . | . | . | . | . | . | . | . | . | . | . | . | . | . |
| 21-225_12F12_LC | . | . | . | . | S | . | W | . | . | . | . | . | . | . | . | . | . | . | . |
| 21-225_147E4_LC | . | . | . | . | . | . | S | . | . | . | P | . | . | . | . | . | . | . | . |
| 21-225_148F8_LC | . | . | . | . | I | . | . | . | . | . | . | . | . | . | . | . | . | . | . |
| 21-225_149A2_LC | . | . | . | . | . | . | C | S | . | . | . | . | . | . | . | . | . | . | . |
| 21-225_149E3_LC | . | . | . | . | . | . | . | . | . | . | . | . | . | . | . | . | . | . | . |
| 21-225_149H4_LC | . | . | . | . | Y | . | . | . | . | . | . | . | . | . | . | . | . | . | . |
| 21-225_154A11_LC | . | . | . | . | S | . | S | . | . | . | . | . | . | . | . | . | . | . | . |
| 21-225_158B12_LC | . | . | . | . | . | . | . | . | . | . | . | . | . | . | . | . | . | . | . |
| 21-225_162F6_LC | . | . | . | . | . | . | . | . | . | . | . | . | . | . | . | . | . | . | . |
| 21-225_164A4_LC | . | . | . | . | . | . | W | . | . | . | . | . | . | . | . | . | . | . | . |
| 21-225_164A7_LC | . | . | . | . | I | . | L | . | . | . | . | . | . | . | . | . | . | . | . |
| 21-225_170F6_LC | . | . | . | . | . | . | W | . | . | . | . | . | . | . | . | . | . | . | . |
| 21-225_174G5_LC | . | . | . | . | . | . | W | . | . | . | . | . | . | . | . | . | . | . | . |
| 21-225_175D10_LC | . | . | . | . | . | . | . | . | . | . | . | . | . | . | . | . | . | . | . |
| 21-225_177B12_LC | . | . | . | . | S | . | . | . | . | . | . | . | . | . | . | . | . | . | . |
| 21-225_181E5_LC | . | . | . | . | . | . | W | . | . | . | . | . | . | . | . | . | . | . | . |
| 21-225_190D7_LC | . | . | . | . | . | . | W | . | . | . | . | . | . | . | . | . | . | . | . |
| 21-225_191D8_LC | . | . | . | . | . | . | . | . | . | . | L | . | . | . | . | . | . | . | . |
| 21-225_193G4_LC | . | . | . | . | . | . | W | . | . | . | . | . | . | . | . | O | . | . | . |
| 21-225_196G8_LC | . | . | . | . | . | . | . | . | . | . | . | . | . | . | . | . | . | . | . |
| 21-225_197C6_LC | . | . | . | . | . | . | . | . | . | . | L | . | . | . | . | . | . | . | . |
| 21-225_197H4_LC | . | . | . | . | . | . | . | . | . | . | L | . | . | . | . | . | . | . | . |
| 21-225_200G7_LC | . | . | . | . | . | . | . | . | . | . | . | . | . | . | . | . | . | . | . |
| 21-225_201F5_LC | . | . | . | . | . | . | . | . | . | . | . | . | . | . | . | . | . | . | . |
| 21-225_213H12_LC | . | . | . | . | I | . | W | . | . | . | . | . | . | . | . | . | . | . | . |
| 21-225_213H7_LC | . | . | . | . | I | . | . | . | . | . | R | . | . | . | . | . | . | . | . |
| 21-225_222H3_LC | . | . | . | . | I | . | W | . | . | . | . | . | . | . | . | . | . | N | . |
| 21-225_223G10_LC | . | . | . | . | . | . | W | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | T | P | P | T | F | G | Q | G | T | K | V | E | I | K | R |
| 21-225_224D8_LC | . | . | . | . | . | . | . | . | . | . | . | . | . | . | . | . | . | . | . |
| 21-225_224H5_LC | . | . | . | . | . | . | . | . | . | . | . | . | . | . | . | . | . | . | . |
| 21-225_225F11_LC | . | . | . | . | . | . | . | . | . | . | . | . | . | . | . | . | . | . | . |
| 21-225_225F5_LC | . | . | . | S | . | . | . | . | . | . | . | . | . | . | . | . | . | N | . |
| 21-225_226A12_LC | . | . | . | S | . | . | . | . | . | . | . | . | . | . | . | . | . | . | . |
| 21-225_226D11_LC | . | . | . | . | . | . | W | . | . | . | . | . | . | . | . | . | . | . | . |
| 21-225_226F6_LC | . | . | . | . | . | . | . | . | . | . | . | . | . | . | . | . | . | . | . |
| 21-225_227C7_LC | . | . | . | S | . | . | . | . | . | . | . | . | . | . | . | . | . | . | . |
| 21-225_23A12_LC | . | . | . | . | . | . | . | . | . | . | . | . | . | . | . | . | . | . | . |
| 21-225_23G12_LC | . | . | . | . | . | . | W | . | . | . | . | . | . | . | . | . | . | . | . |
| 21-225_24E1_LC | . | . | . | . | . | . | . | . | . | . | . | . | . | . | . | . | . | . | . |
| 21-225_25A4_LC | . | . | . | . | . | . | . | . | . | . | . | . | . | . | . | . | . | . | . |
| 21-225_25A4.001_LC | . | . | . | . | . | . | . | . | . | . | . | . | . | . | . | . | . | . | . |
| 21-225_25A4.001.001_LC | . | . | . | . | . | . | . | . | . | . | . | . | . | . | . | . | . | . | . |
| 21-225_25A4.001.002_LC | . | . | . | . | . | . | . | . | . | . | . | . | . | . | . | . | . | . | . |
| 21-225_25A4.001.003_LC | . | . | . | . | . | . | . | . | . | . | . | . | . | . | . | . | . | . | . |
| 21-225_25A4.001.004_LC | . | . | . | . | . | . | . | . | . | . | . | . | . | . | . | . | . | . | . |
| 21-225_25A4.001.005_LC | . | . | . | . | . | . | . | . | . | . | . | . | . | . | . | . | . | . | . |
| 21-225_25A4.001.006_LC | . | . | . | . | . | . | . | . | . | . | . | . | . | . | . | . | . | . | . |
| 21-225_25A4.001.007_LC | . | . | . | . | . | . | . | . | . | . | . | . | . | . | . | . | . | . | . |
| 21-225_25A4.001.008_LC | . | . | . | . | . | . | . | . | . | . | . | . | . | . | . | . | . | . | . |
| 21-225_25A4.001.013_LC | . | . | . | . | . | . | . | . | . | . | . | . | . | . | . | . | . | . | . |
| 21-225_25A4.001.019_LC | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . |
| 21-225_25A4.001.029_LC | . | . | . | . | . | . | . | . | . | . | . | . | . | . | . | . | . | . | . |
| 21-225_26C4_LC | . | . | . | . | . | . | . | . | . | . | . | . | . | . | . | . | ~ | . | . |
| 21-225_29E6_LC | . | . | . | . | . | . | . | . | . | . | . | . | . | . | . | . | . | . | . |
| 21-225_29H8_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | T | P | P | T | F | G | Q | G | T | K | V | E | I | K | R |
| 21-225_43D8_LC | . | . | . | . | . | . | . | . | . | . | . | . | . | . | . | . | . | . | . |
| 21-225_4A2.001.023_LC | . | . | . | . | . | . | V | . | . | . | . | . | . | . | . | . | . | . | . |
| 21-225_4A2.001.028_LC | . | . | . | . | . | . | V | . | . | . | . | . | . | . | . | . | . | . | . |
| 21-225_4A2.001.029_LC | . | . | . | . | . | . | V | . | . | . | . | . | . | . | . | . | . | . | . |
| 21-225_4A2.001.031_LC | . | . | . | . | . | . | V | . | . | . | P | . | . | . | . | . | . | . | . |
| 21-225_56A1_LC | . | . | . | . | . | . | . | . | . | . | . | . | . | . | . | . | . | . | . |
| 21-225_57A11_LC | . | . | . | . | . | . | W | . | . | . | . | . | . | . | . | . | P | . | . |
| 21-225_58B9_LC | . | . | . | . | . | . | . | . | . | . | . | . | S | . | . | . | . | . | . |
| 21-225_59F10_LC | . | . | . | . | . | . | . | . | . | . | . | . | . | . | . | . | . | . | . |
| 21-225_61B5_LC | . | . | . | . | . | . | . | . | . | . | . | . | S | . | . | . | . | . | . |
| 21-225_70E7_LC | . | . | . | . | I | . | . | . | . | . | . | . | . | . | . | . | . | . | . |
| 21-225_74A6_LC | . | . | . | . | . | . | W | . | . | . | . | . | . | Q | . | . | . | . | . |
| 21-225_74A7_LC | . | . | . | . | S | . | . | . | . | . | . | . | . | T | . | Q | . | . | . |
| 21-225_74B1_LC | . | . | . | . | . | . | . | . | . | . | . | . | . | . | . | . | . | . | . |
| 21-225_74B10_LC | . | . | . | . | I | . | . | . | . | . | . | . | . | . | . | . | . | . | . |
| 21-225_74B5_LC | . | . | . | . | S | . | . | . | . | . | . | . | . | T | . | Q | . | . | . |
| 21-225_74B7_LC | . | . | . | . | S | . | . | . | . | . | . | . | . | . | . | . | . | . | . |
| 21-225_74B8_LC | . | . | . | . | . | . | W | . | . | . | . | . | . | . | . | . | . | . | . |
| 21-225_74C1_LC | . | . | . | . | . | . | W | . | . | . | . | . | . | . | . | . | . | . | . |
| 21-225_74F6_LC | . | . | . | . | V | . | L | . | . | . | . | . | . | . | . | . | . | . | . |
| 21-225_74F9_LC | . | . | . | . | S | . | . | . | . | . | . | . | . | . | . | . | . | . | . |
| 21-225_74G4_LC | . | . | . | . | I | . | . | . | . | . | . | . | . | . | . | . | . | . | . |
| 21-225_74H2_LC | . | . | . | . | I | . | . | . | . | . | . | . | . | . | . | . | . | . | . |
| 21-225_75A10_LC | . | . | . | . | S | . | . | . | . | . | . | . | . | . | . | . | . | . | . |
| 21-225_75A7_LC | . | . | . | . | S | . | . | . | . | . | . | . | . | . | . | . | . | . | . |
| 21-225_75C4_LC | . | . | . | . | . | . | . | . | . | . | . | . | . | . | . | . | . | . | . |
| 21-225_75F9_LC | . | . | . | . | S | . | . | . | . | . | . | . | . | . | . | . | . | . | . |
| 21-225_76E11_LC | . | . | . | . | . | . | . | . | . | . | . | . | . | . | . | . | . | S | . |
| 21-225_76E8_LC | . | . | . | . | S | . | L | . | . | . | . | . | . | . | . | . | . | . | . |

# EP 4 435 105 A2

## FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | P | T | F | G | Q | G | T | K | V | E | I | K | R |
| 21-225_76F3_LC | . | . | . | . | S | . | L | . | . | . | . | . | . | T | . | Q | . | . | . |
| 21-225_76F5_LC | . | . | . | . | S | . | . | . | . | . | . | . | . | . | . | . | . | . | . |
| 21-225_76G2_LC | . | . | . | . | S | . | . | . | . | . | . | . | . | . | . | . | . | . | . |
| 21-225_77C10_LC | . | . | . | . | . | . | W | K | . | V | . | . | . | . | . | . | . | T | . |
| 21-225_77C7_LC | . | . | . | . | S | . | . | . | . | . | . | . | D | . | . | . | . | . | . |
| 21-225_77E1_LC | . | . | . | . | S | . | L | . | . | . | . | . | . | . | . | . | . | . | . |
| 21-225_78C1_LC | . | . | . | . | . | . | W | K | . | V | . | . | . | . | . | . | . | . | G |
| 21-225_78E11_LC | . | . | . | . | S | . | . | . | . | . | . | . | . | . | . | . | . | . | . |
| 21-225_78H12_LC | . | . | . | . | S | . | . | . | . | . | . | . | . | . | . | . | . | . | . |
| 21-225_79E9_LC | . | . | . | . | . | . | . | . | . | . | . | . | . | . | . | . | . | . | . |
| 21-225_79F12_LC | . | . | . | . | . | . | W | . | . | . | . | . | . | . | . | . | . | . | . |
| 21-225_79G10_LC | . | . | . | . | S | . | L | . | . | . | . | . | . | T | . | Q | . | . | . |
| 21-225_79G6_LC | . | . | . | . | I | . | . | . | . | . | . | . | . | . | . | . | . | . | . |
| 21-225_80D3_LC | . | . | . | . | . | . | G | K | . | V | . | V | . | . | . | . | . | T | G |
| 21-225_80E9_LC | . | . | . | . | . | . | W | . | . | V | . | . | . | . | . | . | . | . | . |
| 21-225_80H3_LC | . | . | . | . | . | . | . | . | . | . | . | . | . | . | . | . | . | . | . |
| 21-225_81E5_LC | . | . | . | . | S | . | L | . | . | . | . | . | . | T | . | Q | . | . | . |
| 21-225_81F9_LC | . | . | . | . | . | . | G | K | . | V | . | I | . | M | . | . | . | T | G |
| 21-225_83F3_LC | . | . | . | . | . | . | W | K | . | V | . | I | . | . | . | . | . | . | G |
| 21-225_83G3_LC | . | . | . | . | . | . | W | . | . | V | . | . | . | . | . | . | . | . | . |
| 21-225_83G7_LC | . | . | . | . | S | . | L | . | . | . | . | . | . | . | . | . | . | . | . |
| 21-225_84G7_LC | . | . | . | . | S | . | . | . | . | . | . | . | . | . | . | . | . | . | . |
| 21-225_85H2_LC | . | . | . | . | . | . | W | . | . | . | . | . | . | . | . | . | . | . | . |
| 21-225_85H9_LC | . | . | . | . | . | . | . | . | . | . | . | . | . | . | . | . | . | . | . |
| 21-225_86E9_LC | . | . | . | . | S | . | L | . | . | . | . | . | . | T | . | . | . | . | . |
| 21-225_88A1_LC | . | . | . | . | S | . | . | . | . | . | . | . | . | . | . | . | . | . | . |
| 21-225_88B4_LC | . | . | . | . | . | . | . | . | . | . | . | . | . | . | . | . | . | . | . |
| 21-225_88C10_LC | . | . | . | . | A | . | . | . | . | . | . | . | . | . | . | . | . | . | . |
| 21-225_88G2_LC | . | . | . | . | . | . | W | . | . | V | . | . | . | . | . | . | . | . | G |

4596

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | P | T | F | G | Q | G | T | K | V | E | I | K | R |
| 21-225_90D9_LC | . | . | | . | S | . | L | . | . | . | . | . | . | . | . | . | . | . | . |
| 21-225_91F1_LC | . | . | | . | . | . | W | . | . | V | . | . | . | . | . | . | . | . | . |
| 21-225_91G8_LC | . | . | | . | . | . | W | . | . | . | . | . | . | . | . | . | . | . | . |
| 21-225_92E6_LC | . | . | | . | V | . | . | . | . | . | . | . | . | . | . | . | . | . | . |
| 21-225_92F12_LC | . | . | | | . | . | . | . | . | . | . | . | . | . | . | . | . | . | . |
| 21-225_94D2_LC | . | . | | . | I | . | . | . | . | . | . | . | . | . | . | . | . | . | G |
| 21-225_94E11_LC | . | . | | . | S | . | L | . | . | . | . | . | . | T | . | Q | . | . | . |
| 21-225_94E12_LC | . | . | | . | . | . | . | . | . | . | . | . | . | . | . | . | . | . | . |
| 21-225_95E12_LC | . | . | | . | . | . | C | S | . | . | . | . | . | . | . | . | . | . | . |
| 21-225_95G4_LC | . | . | | . | S | . | L | . | . | . | . | . | . | T | . | Q | . | . | . |
| 21-225_95H4_LC | . | . | | . | S | . | L | . | . | . | . | . | . | T | . | Q | . | . | . |
| 21-225_96D10_LC | . | . | | . | V | . | . | . | . | . | . | . | . | . | . | . | . | . | . |
| 21-225_96D5_LC | . | . | | . | S | . | . | . | . | . | . | . | . | T | . | . | . | . | . |
| 21-225_96E2_LC | . | . | | | . | . | W | . | . | . | . | . | . | . | . | . | . | . | . |
| 21-225_96H5_LC | . | . | | . | . | . | C | S | . | . | . | . | . | . | . | . | . | . | . |
| 21-225_97H4_LC | . | . | | . | . | . | W | . | . | V | . | . | . | . | . | . | . | T | Q |

**FIGURE 57**

Table 100.    Consensus 20 - VK1|A30/JK3 (SEQ ID NO: 50317):

DIQMTQSPSSLSASVGDRVTITCRAS--QGIR------NDLGWYQQKPGKAPKRLIYA--------ASSLQSGVPSRFSGSGSG--
TEFTLTISSLQPEDFATYYCLQHNS---------------------YPFTFGPGTKVDIKR wherein:

A at position 25 can be substituted with T

G at position 30 can be substituted with D or V

I at position 31 can be substituted with M

R at position 32 can be substituted with S

N at position 39 can be substituted with K, S, D or T

D at position 40 can be substituted with N, H, Y, V, A, I or L

L at position 41 can be substituted with F

G at position 42 can be substituted with D

A at position 58 can be substituted with P, T, G, I, R or V

A at position 67 can be substituted with V

S at position 68 can be substituted with F or T

S at position 69 can be substituted with N, T, R or D

L at position 70 can be substituted with V

Q at position 71 can be substituted with L

S at position 72 can be substituted with N, T, G or R

L at position 107 can be substituted with I

Q at position 108 can be substituted with H or L

FIGURE 57

H at position 109 can be substituted with Y, D or L

N at position 110 can be substituted with Y, T, H, G or I

S at position 111 can be substituted with R, D, T, G or N

Y at position 135 can be substituted with F or H

P at position 136 can be substituted with L

F at position 137 can be substituted with L

T at position 138 can be substituted with K

Arg Xaa Ser Gln Xaa Xaa Xaa Xaa Xaa Xaa Xaa (SEQ ID NO: 50135)

Xaa Xaa Xaa Xaa Xaa Xaa Xaa (SEQ ID NO: 50136)

Xaa Xaa Xaa Xaa Xaa Xaa Xaa Xaa Xaa (SEQ ID NO: 50137)

Arg Ala Ser Gln Gly Ile Arg Asn Asp Leu Gly (SEQ ID NO: 50621)

Ala Ala Ser Ser Leu Gln Ser (SEQ ID NO: 50622)

Leu Gln His Asn Ser Tyr Pro Phe Thr (SEQ ID NO: 50623)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | K_FR1 | | | | | | | | | | | | | | | | |
| CONSENSUS | D | I | Q | M | T | Q | S | P | S | S | L | S | A | S | V | G | D | R | V | T | I | T | C | R | A | S |
| 21-225_14D6_LC | . | . | . | . | . | . | . | . | . | . | . | . | . | . | . | . | . | S | . | . | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | . | . | . | . | . | . | . | . | . | C | . | . | . | . | . | . | . | . | . | . | . | . | . | . |
| 21-225_157H12_LC | . | . | . | . | . | . | . | . | . | . | . | . | . | . | . | . | . | . | . | . | . | . | . | . | . | . |
| 21-225_157H4_LC | . | . | . | . | . | . | . | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | . | . | . | . | . | . | . | . | . | . | . | . | . | . | . | . | . | . | . | . | . | . |
| 21-225_158G8_LC | . | . | . | . | . | . | . | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . | . | . | . | . | . | T | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | . | . | . | . | . | . | A | . | . | . | . | . | . | . | . | . | . | . | . | . | . | . |
| 21-225_161D11_LC | . | . | . | . | . | . | . | . | . | . | . | . | . | . | . | . | . | . | . | . | . | . | . | . | . | . |
| 21-225_161F7_LC | . | . | . | . | . | . | . | . | . | . | . | . | . | . | . | . | . | . | . | . | . | . | . | . | . | . |
| 21-225_162F2_LC | . | . | . | . | . | . | . | . | . | . | . | C | . | . | . | . | . | . | . | . | . | . | . | . | . | . |
| 21-225_162H3_LC | . | . | . | . | . | . | . | . | . | . | . | . | . | . | . | . | . | . | . | . | . | . | . | . | . | . |
| 21-225_163E2_LC | . | . | . | . | . | . | . | . | . | . | . | . | . | . | . | . | . | . | . | . | . | . | . | . | . | . |
| 21-225_163F9_LC | . | . | . | . | . | . | . | . | . | . | . | . | . | . | . | . | . | . | . | . | . | . | . | . | . | . |
| 21-225_163G10_LC | . | . | . | . | . | . | . | . | . | . | . | . | . | . | . | . | . | . | . | . | . | . | . | . | . | . |
| 21-225_16A11_LC | . | . | . | . | . | . | . | . | . | . | . | . | . | . | . | . | . | S | . | . | . | S | . | . | . | . |
| 21-225_16H1_LC | . | . | . | . | . | . | . | . | . | . | . | . | . | . | . | . | . | . | . | . | . | . | . | . | . | . |
| 21-225_16H9_LC | . | . | . | . | . | . | . | . | . | . | . | . | . | . | . | . | . | . | . | . | . | . | . | . | . | . |
| 21-225_171A11_LC | . | . | . | . | . | . | . | . | . | . | . | . | . | . | . | . | . | . | . | . | . | . | . | . | . | . |
| 21-225_177B6_LC | . | . | . | . | . | . | . | . | . | . | . | . | . | . | . | . | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | . | . |
| 21-225_181A8_LC | . | . | . | . | . | . | . | . | . | . | . | . | . | . | . | . | . | . | . | . | . | . | . | . | . | . |
| 21-225_184D11_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_184H10_LC | . | | . | | | . | | . | | . | | . | . | . | . | . | | . | | . | . | . | . | . | . | . |
| 21-225_190D6_LC | . | | . | | | . | | . | | . | | . | . | . | . | . | | . | | . | . | . | . | . | . | . |
| 21-225_192F6_LC | . | | . | | | . | | . | | . | | . | . | . | . | . | | . | | . | . | . | . | . | . | . |
| 21-225_194F10_LC | . | | . | | | . | | . | | . | | . | . | . | . | . | | . | | . | . | . | . | . | . | . |
| 21-225_195B9_LC | . | | . | | | . | | . | | . | | . | . | . | . | . | | . | | . | . | . | . | . | . | . |
| 21-225_197D7_LC | . | | . | | | . | | . | | . | | . | . | . | . | . | | . | | . | . | . | . | . | . | . |
| 21-225_197G10_LC | . | | . | | | . | | . | | . | | . | . | . | . | . | | . | | . | . | . | . | . | . | . |
| 21-225_198B9_LC | . | | . | | | . | | . | | . | | . | . | . | . | . | | . | | . | . | . | . | . | . | . |
| 21-225_198E3_LC | . | | . | | | . | | . | | . | | . | . | . | . | . | | . | | . | . | . | . | . | . | . |
| 21-225_200F6_LC | . | | . | | | . | | . | | . | | . | . | . | . | . | | . | | . | . | . | . | . | . | . |
| 21-225_202A3_LC | . | | . | | S | . | | . | | . | | . | . | . | . | . | | . | | . | . | . | . | . | . | . |
| 21-225_203B9_LC | . | | . | | | . | | . | | . | | . | . | . | . | . | | . | | . | . | . | . | . | . | . |
| 21-225_210E4_LC | . | | . | | | . | | . | | . | | . | . | . | . | . | | . | | . | . | . | . | . | . | . |
| 21-225_225B6_LC | . | | . | | | . | | . | | . | | . | . | . | . | . | | . | : | . | . | . | . | . | . | . |
| 21-225_225D6_LC | . | | . | | | . | | . | | . | | . | . | . | . | . | | . | | . | . | . | . | . | . | . |
| 21-225_225F8_LC | . | | . | | | . | | . | | . | | . | . | . | . | . | | . | | . | . | . | . | . | . | . |
| 21-225_226A9_LC | . | | . | | | . | | . | | . | | . | . | . | . | . | | . | | . | . | . | . | . | . | . |
| 21-225_227C12_LC | . | | . | | | . | | . | | . | | . | . | . | . | . | | . | | . | . | . | . | . | . | . |
| 21-225_227G3_LC | . | | . | | | . | | . | | . | | . | . | . | . | . | | . | | . | . | . | . | . | . | . |
| 21-225_227H5_LC | . | | . | | | . | | . | | . | | . | . | . | . | . | | . | | . | . | . | . | . | . | . |
| 21-225_22H3_LC | . | | . | | | . | | . | | . | | . | . | . | . | . | | . | | . | . | . | . | . | . | . |
| 21-225_25A9_LC | . | | . | | | . | | . | | . | | . | . | . | . | . | | . | | . | . | . | . | . | . | . |
| 21-225_25B6_LC | . | | . | | | . | | . | | . | | . | . | . | . | . | | . | | . | . | . | . | . | . | . |
| 21-225_25G5_LC | . | | . | | | . | | . | | . | | . | . | . | . | . | | . | | . | . | . | . | . | . | . |
| 21-225_27D5_LC | . | | . | | | . | | . | | . | | . | . | . | . | . | | . | | . | . | . | . | . | . | . |
| 21-225_28A9_LC | . | | . | | | . | | . | | . | | . | . | . | . | . | | . | | . | . | . | . | . | . | . |
| 21-225_28B8_LC | . | | . | | | . | | . | | . | | . | . | . | . | . | | . | | . | . | . | . | . | . | . |
| 21-225_29D7_LC | . | | . | | | . | | . | | . | | . | . | . | . | . | | . | | . | . | . | . | . | . | . |
| 21-225_29D8_LC | . | | . | | | . | | . | | . | | . | . | . | . | . | | . | | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_29D9_LC | . | | | | | | | | | | | | | | | | | | | | | | | | | |
| 21-225_30H11_LC | . | . | . | . | . | . | . | . | . | . | . | . | . | . | . | . | . | . | . | . | . | . | . | . | . | . |
| 21-225_31G9_LC | . | | | | | | | | | | | | | | | | | | | | | | | | | |
| 21-225_43B1_LC | . | | | | | | | | | | | | | | | | | | | | | | | | | |
| 21-225_43E1_LC | . | | | | | | | | | | | | | | | | | | | | | | | | | |
| 21-225_43E5_LC | . | | | | | | | | | | | | | | | | | | | | | | | | | |
| 21-225_43H8_LC | . | | | | | | | | | | | | | | | | | | | | | | | | | |
| 21-225_44C8_LC | . | | | | | | | | | | | | | | | | | | | | | | | | | |
| 21-225_45H2_LC | . | | | | | | | | | | | | | | | | | | | | | | | | | |
| 21-225_46E9_LC | . | | | | | | | | | | | | | | | | | | | | | | | | | |
| 21-225_47H7_LC | . | | | | | | | | | | | | | | | | | | | | | | | | Y | |
| 21-225_48E10_LC | . | | | | | | | | | | | | | | | | | | | | | | | | | |
| 21-225_50C4_LC | . | | | | | | | | | | | | | | | | | | | | L | | | | Y | |
| 21-225_51B11_LC | A | | | | | | | | | | | | | | | | | | | | | | | | Y | |
| 21-225_51E4_LC | . | | | | | | | | | | | | | | | | | | | | | | | | | |
| 21-225_51F9_LC | . | | | | | | | | | | | | | | | | | | | | | | | | | |
| 21-225_52B9_LC | A | | | | | | | | | | | | | | | | | | | | | | | | | |
| 21-225_52H12_LC | . | | | | | | | | | | | | | | | | | | | | | | | | | |
| 21-225_53B10_LC | . | | | | | | | | | | | | | A | | | | | | | | | | | | |
| 21-225_53B12_LC | . | | | | | | | | | | | | | | | | | | | | | | | | | |
| 21-225_53G1_LC | . | | | | | | | | | | | | | | | | | | | | | | | | | |
| 21-225_54D3_LC | . | | | | | | | | | | | | | | | | | | | | | | | | | |
| 21-225_54G7_LC | . | | | | | | | | | | | | | | | | | | | | | | | | | |
| 21-225_55B3_LC | . | | | | | | | | | | | | | | | | | | | | | | | | | |
| 21-225_56A5_LC | . | | | | | | | | | | | | | | | | | | | | | | | | | |
| 21-225_56H7_LC | . | . | . | L | | | | | | | | | | | | | | | | | | | | | | |
| 21-225_57A4_LC | . | | | | | | | | | | | | | | | | | | | | | | | | | |
| 21-225_57B2_LC | . | | | | | | | | | | | | V | | | | | | | | | | | | | |
| 21-225_57F4_LC | . | | | | | | | | | | | | | | | | | | | | | | | | | |

FIGURE 57

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_14D6_LC | . | . | . | D | . | . | . | . | . | . | . | . | . | N | . | . | . | . | . | N | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | . | . | . | . | . | . | . | . | . | . | . | . | . | . | . | . | . | . | . | . | . | . | . | . |
| 21-225_157H12_LC | . | . | . | . | . | . | . | . | . | . | . | . | . | . | F | . | . | . | . | N | . | . | . | . | . | . |
| 21-225_157H4_LC | . | . | . | . | . | . | . | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | . | . | . | . | . | . | . | . | . | . | . | . | . | . | . | . | . | . | . | . | . | . |
| 21-225_158G8_LC | . | . | . | D | . | . | . | . | . | . | . | . | . | . | . | . | . | . | . | . | . | . | . | . | . | . |
| 21-225_158H6_LC | . | . | . | . | . | . | . | . | . | . | . | . | S | . | . | . | . | . | . | . | . | . | . | . | . | . |
| 21-225_159C5_LC | . | . | . | . | . | . | . | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | . | . | . | . | . | . | . | . | . | . | . | . | . | . | . | . | . | . | . | . | . | . |
| 21-225_161D11_LC | . | . | . | . | . | . | . | . | . | . | . | . | . | . | . | . | . | . | . | . | . | . | . | E | . | . |
| 21-225_161F7_LC | . | . | . | . | . | . | . | . | . | . | . | . | . | . | . | . | . | . | . | . | . | . | . | E | . | . |
| 21-225_162F2_LC | . | . | . | . | . | . | . | . | . | . | . | . | . | . | . | . | . | . | . | . | . | . | . | . | . | . |
| 21-225_162H3_LC | . | . | . | . | . | . | . | . | . | . | . | . | . | . | . | . | . | . | . | . | . | . | . | . | . | . |
| 21-225_163E2_LC | . | . | . | D | . | . | . | . | . | . | . | . | . | . | . | . | . | . | . | . | . | . | . | . | . | . |
| 21-225_163F9_LC | . | . | . | D | . | . | . | . | . | . | . | . | D | . | . | . | . | . | . | . | . | . | . | . | . | . |
| 21-225_163G10_LC | . | . | . | D | . | . | . | . | . | . | . | . | . | N | . | . | . | . | . | . | . | . | . | . | . | . |
| 21-225_16A11_LC | . | . | . | D | . | . | . | . | . | . | . | . | . | N | . | . | . | . | . | N | N | . | . | . | . | . |
| 21-225_16H1_LC | . | . | . | . | . | . | . | . | . | . | . | . | D | I | . | . | . | . | . | . | . | . | . | . | . | . |
| 21-225_16H9_LC | . | . | . | . | . | . | . | . | . | . | . | . | . | Y | . | . | . | F | . | . | . | . | . | . | . | . |
| 21-225_171A11_LC | . | . | . | . | . | . | . | . | . | . | . | . | . | N | . | . | . | . | . | . | . | . | . | . | . | . |
| 21-225_177B6_LC | . | . | . | D | . | S | . | . | . | . | . | . | . | . | . | . | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | . | . |
| 21-225_181A8_LC | . | . | . | . | . | . | . | . | . | . | . | . | . | . | . | . | . | . | . | . | . | . | . | . | . | . |
| 21-225_184D11_LC | . | . | . | . | . | . | . | . | . | . | . | . | . | . | . | . | . | . | . | . | . | . | . | . | . | . |

## FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | K_FR2 | | | | |
| CONSENSUS | - | - | Q | G | I | R | - | - | - | - | - | - | N | D | L | G | W | Y | Q | Q | K | P | G | K | A | P |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | L | . | . | . | . | . | . |
| 21-225_192F6_LC | . | . | . | . | . | . | . | . | . | . | . | . | . | . | . | . | . | . | . | L | . | . | . | . | . | . |
| 21-225_194F10_LC | . | . | . | . | . | . | . | . | . | . | . | . | . | . | . | . | . | . | . | L | . | . | . | . | . | . |
| 21-225_195B9_LC | . | . | . | . | . | . | . | . | . | . | . | . | K | . | . | . | . | . | . | . | . | . | . | . | . | . |
| 21-225_197D7_LC | . | . | . | . | . | . | . | . | . | . | . | . | K | . | . | . | . | . | . | . | . | . | . | . | . | . |
| 21-225_197G10_LC | . | . | . | . | . | . | . | . | . | . | . | . | . | . | . | . | . | . | . | . | . | . | . | . | . | . |
| 21-225_198B9_LC | . | . | . | . | . | . | . | . | . | . | . | . | K | . | . | . | . | . | . | . | . | . | . | . | . | . |
| 21-225_198E3_LC | . | . | . | . | . | . | . | . | . | . | . | . | K | . | . | . | . | . | . | . | . | . | . | . | . | . |
| 21-225_200F6_LC | . | . | . | D | . | . | . | . | . | . | . | . | . | . | . | . | . | . | . | . | . | . | . | . | . | . |
| 21-225_202A3_LC | . | . | . | . | . | . | . | . | . | . | . | . | . | . | . | . | . | . | . | . | . | . | . | . | . | . |
| 21-225_203B9_LC | . | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | M | . | . | . | . | . | . | K | . | . | . | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | D | . | . | . | . | . | . | . | . | . | . | . | . | . | . | . | . | . | . | . | . | . | . |
| 21-225_22H3_LC | . | . | . | D | . | . | . | . | . | . | . | . | . | N | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | D | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | N | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | K_FR2 | | | |
| CONSENSUS | - | - | Q | G | I | R | - | - | - | - | - | - | N | D | L | G | W | Y | Q | Q | K | P | G | K | A | P |
| 21-225_29D9_LC | . | . | . | . | . | . | . | . | . | . | . | . | . | . | . | . | . | F | . | . | . | . | . | . | . | . |
| 21-225_30H11_LC | . | . | . | . | . | . | . | . | . | . | . | . | . | . | . | . | . | . | . | . | . | . | . | . | . | . |
| 21-225_31G9_LC | . | . | . | . | . | . | . | . | . | . | . | . | . | . | . | . | . | . | . | . | . | . | . | . | . | . |
| 21-225_43B1_LC | . | . | . | . | . | . | . | . | . | . | . | . | . | . | . | . | . | . | . | . | . | . | . | . | . | . |
| 21-225_43E1_LC | . | . | . | . | . | . | . | . | . | . | . | . | . | . | . | . | . | . | . | R | . | . | . | . | . | . |
| 21-225_43E5_LC | . | . | . | . | . | . | . | . | . | . | . | . | . | . | . | . | . | . | . | . | . | . | . | . | . | . |
| 21-225_43H8_LC | . | . | . | . | . | . | . | . | . | . | . | . | . | . | . | . | . | R | . | . | . | . | . | . | . | . |
| 21-225_44C8_LC | . | . | . | . | . | . | . | . | . | . | . | . | . | . | . | . | . | . | . | . | . | . | . | . | . | . |
| 21-225_45H2_LC | . | . | . | . | . | . | . | . | . | . | . | . | . | . | . | . | . | . | . | . | . | . | . | . | . | . |
| 21-225_46E9_LC | . | . | . | . | . | . | . | . | . | . | . | . | . | . | . | . | . | . | . | . | . | . | . | . | . | . |
| 21-225_47H7_LC | . | . | . | . | . | . | . | . | . | . | . | . | . | . | . | . | . | . | . | R | . | . | . | . | . | . |
| 21-225_48E10_LC | . | . | . | . | . | . | . | . | . | . | . | . | S | . | . | . | . | . | . | . | . | . | . | . | . | . |
| 21-225_50C4_LC | . | . | . | . | . | . | . | . | . | . | . | . | . | . | . | . | . | . | . | . | . | . | . | . | I | . |
| 21-225_51B11_LC | . | . | . | . | . | . | . | . | . | . | . | . | . | . | . | . | . | . | . | . | . | . | R | . | . | . |
| 21-225_51E4_LC | . | . | . | . | . | . | . | . | . | . | . | . | . | . | . | . | . | . | . | . | . | . | . | . | . | . |
| 21-225_51F9_LC | . | . | . | . | . | . | . | . | . | . | . | . | T | . | . | . | . | . | . | . | . | . | R | . | . | . |
| 21-225_52B9_LC | . | . | . | . | . | . | . | . | . | . | . | . | . | . | . | . | . | . | . | . | . | . | R | . | . | . |
| 21-225_52H12_LC | . | . | . | . | . | . | . | . | . | . | . | . | N | . | . | . | . | . | . | . | . | . | . | . | . | . |
| 21-225_53B10_LC | . | . | D | . | . | . | . | . | . | . | . | . | Y | . | . | . | . | . | . | . | . | . | . | . | . | . |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | . | . | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . | . | . | . | . | . | . | . |
| 21-225_56A5_LC | . | . | D | . | . | . | . | . | . | . | . | . | L | . | . | . | . | . | . | . | . | . | . | . | . | . |
| 21-225_56H7_LC | . | . | D | . | . | . | . | . | . | . | . | . | . | . | . | . | . | . | . | R | . | . | . | . | . | . |
| 21-225_57A4_LC | . | . | . | . | . | . | . | . | . | . | . | . | . | . | . | . | . | . | . | . | L | . | . | . | . | . |
| 21-225_57B2_LC | . | . | . | . | . | . | . | . | . | . | . | . | A | . | . | . | . | . | . | . | . | . | . | . | . | . |
| 21-225_57F4_LC | . | . | V | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_58D11_LC | . | . | . | . | . | . | . | . | . | . | . | . | S | . | . | D | . | . | . | . | . | . | . | . | . | . |
| 21-225_58F5_LC | . | . | . | . | . | . | . | . | . | . | . | . | . | . | . | . | . | F | L | . | T | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | H | . | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | . | D | . | . | . | . | . | . | . | . | N | . | . | . | . | . | . | . | . | . | . | . | . | . |
| 21-225_64A12_LC | . | . | . | . | . | . | . | . | . | . | . | . | . | . | . | . | . | . | . | . | . | . | . | . | . | . |
| 21-225_66F9_LC | . | . | . | D | . | . | . | . | . | . | . | . | V | . | . | . | . | . | . | . | . | . | . | . | . | . |
| 21-225_67B7_LC | . | . | . | . | . | . | . | . | . | . | . | . | . | . | . | . | . | F | . | . | . | . | . | . | . | . |
| 21-225_68G8_LC | . | . | . | . | . | . | . | . | . | . | . | . | N | . | . | . | . | . | . | . | G | . | . | . | . | . |
| 21-225_71B11_LC | . | . | . | . | . | . | . | . | . | . | . | . | . | . | . | D | . | . | . | . | T | . | . | . | . | . |
| 21-225_71B6_LC | . | . | . | . | . | . | . | . | . | . | . | . | . | . | . | D | . | . | . | . | . | . | . | . | . | . |
| 21-225_71H6_LC | . | . | . | . | . | . | . | . | . | . | . | . | V | . | . | . | . | . | . | . | . | . | . | . | . | . |
| 21-225_73A6_LC | . | . | . | . | . | . | . | . | . | . | . | . | . | . | . | . | . | F | . | . | . | . | . | . | . | . |
| 21-225_74A11_LC | . | . | . | D | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | F | . | . | . | . | . | . | . | . |
| 21-225_76H10_LC | . | . | . | D | . | . | . | . | . | . | . | . | Y | . | . | . | . | . | . | . | . | . | . | . | . | . |
| 21-225_76H12_LC | . | . | . | . | . | . | . | . | . | . | . | . | . | . | . | . | . | F | . | . | . | . | . | . | . | . |
| 21-225_84H12_LC | . | . | . | . | . | . | . | . | . | . | . | . | . | . | . | . | . | F | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | D | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_14D6_LC | . | . | . | . | . | . | . | . | . | . | . | . | . | . | . | . | . | . | . | . | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | . | . | N | V | . | . | . | . | . | . | . | . | . | . | . | . | . | . | . | . | . | . | . | . |
| 21-225_157H12_LC | . | G | . | . | H | . | . | . | . | . | . | . | . | . | . | . | . | . | . | . | . | . | . | . | . | . |
| 21-225_157H4_LC | . | . | . | . | . | P | . | . | . | . | . | . | . | . | . | . | . | . | . | T | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | N | . | . | . | . | . | . | . | . | . | . | . | . | . | . | . | . | . | . | . | . | . |
| 21-225_158G8_LC | . | . | M | . | . | T | . | . | . | . | . | . | . | . | . | . | . | . | . | . | . | . | . | . | . | . |
| 21-225_158H6_LC | . | . | . | . | . | T | . | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | N | V | . | . | . | . | . | . | . | . | . | . | . | . | . | . | . | . | . | . | . | . |
| 21-225_161D11_LC | . | . | . | . | . | . | . | . | . | . | . | . | . | . | . | . | T | . | . | . | . | . | . | . | . | . |
| 21-225_161F7_LC | . | . | . | . | . | . | . | . | . | . | . | . | . | . | . | . | T | . | . | . | . | . | . | . | . | . |
| 21-225_162F2_LC | . | . | . | . | N | . | . | . | . | . | . | . | . | . | . | . | . | . | . | . | . | . | . | . | . | . |
| 21-225_162H3_LC | . | . | . | . | N | . | . | . | . | . | . | . | . | . | . | . | . | . | . | . | . | . | . | . | . | . |
| 21-225_163E2_LC | T | . | . | . | . | P | . | . | . | . | . | . | . | . | . | . | . | . | . | . | . | . | . | . | . | . |
| 21-225_163F9_LC | . | . | . | . | . | P | . | . | . | . | . | . | . | . | . | . | . | . | . | . | . | . | . | . | . | . |
| 21-225_163G10_LC | . | . | . | . | . | P | . | . | . | . | . | . | . | . | . | . | . | . | . | . | . | . | . | . | . | . |
| 21-225_16A11_LC | . | . | . | . | . | . | . | . | . | . | . | . | . | . | . | . | . | . | . | . | . | . | . | . | . | . |
| 21-225_16H1_LC | . | . | . | . | . | . | . | . | . | . | . | . | . | . | . | . | . | . | . | . | N | . | . | . | . | . |
| 21-225_16H9_LC | . | . | . | . | . | G | . | . | . | . | . | . | . | . | . | . | . | . | . | . | . | . | . | . | . | } |
| 21-225_171A11_LC | . | . | . | . | . | P | . | . | . | . | . | . | . | . | . | . | . | . | . | . | . | . | . | . | . | . |
| 21-225_177B6_LC | . | . | . | . | . | . | . | . | . | . | . | . | . | . | . | . | . | . | . | . | . | . | . | . | . | . |
| 21-225_17E5_LC | . | . | . | . | . | T | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | T | . | . | . | . | . | . | . | . | . | . | . | . | . | . | . | . | . | . | . | . |
| 21-225_181A8_LC | . | . | . | . | . | T | . | . | . | . | . | . | . | . | . | . | . | . | . | . | . | . | . | . | . | . |
| 21-225_184D11_LC | . | . | . | . | . | } | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_184H10_LC | . | . | . | . | ! | . | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | Q | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | Q | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | C | . | . | . | . | . | . | . | . | . | . | . | ® | . | . | . | . | . | . | . | . | . |
| 21-225_203B9_LC | . | . | . | . | R | . | . | . | . | . | . | . | . | . | . | . | . | V | . | N | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | T | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | L | ® | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | P | . | . | . | . | . | . | . | . | . | . | . | . | . | . | T | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | R | . | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | N | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | T | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_184H10_LC | . | . | . | . | . | I | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | G | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | G | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | C | . | . | . | . | . | . | . | . | . | . | . | . | P | . | . | . | . | . | . | . | . | . |
| 21-225_203B9_LC | . | . | . | . | . | R | . | . | . | . | . | . | . | . | . | . | . | V | . | N | . | . | . | . | . | . |
| 21-225_210E4_LC | . | . | . | . | . | . | . | . | . | . | . | . | . | . | . | T | . | . | . | . | . | . | . | . | . | . |
| 21-225_225B6_LC | . | . | . | . | . | . | . | . | . | . | . | . | . | . | T | . | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | L | G | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | P | . | . | . | . | . | . | . | . | . | . | . | . | . | T | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | R | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | N | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | T | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_29D9_LC | . | | . | . | . | . | . | . | . | . | . | . | . | . | . | . | . | . | . | . | . | . | . | . | . | . |
| 21-225_30H11_LC | . | . | . | V | . | P | . | . | . | . | . | . | . | . | . | . | . | . | . | . | . | . | . | . | . | . |
| 21-225_31G9_LC | . | | . | . | . | P | . | . | . | . | . | . | . | . | . | . | . | . | . | . | . | . | . | . | . | . |
| 21-225_43B1_LC | . | | . | . | . | . | . | . | . | . | . | . | . | . | . | . | . | . | . | . | . | . | . | . | . | . |
| 21-225_43E1_LC | . | | . | . | N | . | . | . | . | . | . | . | . | . | . | . | . | . | . | . | . | . | . | . | . | . |
| 21-225_43E5_LC | . | | . | . | . | G | . | . | . | . | . | . | . | . | . | . | N | . | . | . | . | . | . | . | . | . |
| 21-225_43H8_LC | . | | . | . | . | . | . | . | . | . | . | . | . | . | . | . | . | . | . | . | . | . | . | . | . | . |
| 21-225_44C8_LC | . | | . | . | . | . | . | . | . | . | . | . | . | . | . | . | N | . | . | . | . | . | . | . | . | . |
| 21-225_45H2_LC | . | | . | . | . | G | . | . | . | . | . | . | . | . | . | . | N | . | . | . | . | . | . | . | . | . |
| 21-225_46E9_LC | . | | . | . | . | . | . | . | . | . | . | . | . | . | . | . | . | . | . | . | . | . | . | . | . | . |
| 21-225_47H7_LC | . | | . | . | . | . | . | . | . | . | . | . | . | . | . | . | . | . | . | . | . | . | . | . | . | . |
| 21-225_48E10_LC | . | | . | . | . | T | . | . | . | . | . | . | . | . | . | . | . | . | . | . | . | . | . | . | . | . |
| 21-225_50C4_LC | . | | . | . | . | . | . | . | . | . | . | . | . | . | . | . | . | . | . | . | . | . | . | . | . | . |
| 21-225_51B11_LC | . | | . | . | . | . | . | . | . | . | . | . | . | . | . | . | . | . | . | . | . | . | . | . | . | . |
| 21-225_51E4_LC | Q | | . | . | . | . | . | . | . | . | . | . | . | . | . | . | . | . | . | . | . | . | . | . | . | . |
| 21-225_51F9_LC | Q | | . | . | . | . | . | . | . | . | . | . | . | . | . | . | . | . | . | ※ | . | . | . | . | . | . |
| 21-225_52B9_LC | . | | . | . | . | . | . | . | . | . | . | . | . | . | . | . | . | . | . | . | . | . | L | . | . | . |
| 21-225_52H12_LC | . | | . | . | . | G | . | . | . | . | . | . | . | . | . | . | . | . | . | . | . | . | . | . | . | . |
| 21-225_53B10_LC | . | | . | . | . | G | . | . | . | . | . | . | . | . | . | . | . | . | . | . | . | . | . | . | . | . |
| 21-225_53B12_LC | . | | . | . | . | . | . | . | . | . | . | . | . | . | . | . | . | . | . | . | . | . | . | . | . | . |
| 21-225_53G1_LC | . | | . | . | . | P | . | . | . | . | . | . | . | . | . | . | . | . | . | . | . | . | . | . | . | . |
| 21-225_54D3_LC | . | | . | . | . | . | . | . | . | . | . | . | . | . | . | . | . | . | . | . | . | . | . | . | . | . |
| 21-225_54G7_LC | . | | . | . | . | . | . | . | . | . | . | . | . | . | . | . | . | . | . | . | . | . | . | . | . | . |
| 21-225_55B3_LC | . | | . | . | . | . | . | . | . | . | . | . | . | . | . | . | . | . | . | . | . | . | . | . | . | . |
| 21-225_56A5_LC | . | | . | . | . | . | . | . | . | . | . | . | . | . | . | . | . | . | . | . | . | . | . | . | . | . |
| 21-225_56H7_LC | . | | . | . | . | P | . | . | . | . | . | . | . | . | . | . | . | . | L | . | . | . | . | . | . | . |
| 21-225_57A4_LC | . | | . | . | . | . | . | . | . | . | . | . | . | . | . | . | . | . | L | . | . | . | . | . | . | . |
| 21-225_57B2_LC | . | | . | . | . | . | . | . | . | . | . | . | . | . | . | . | . | . | . | . | . | . | . | . | . | . |
| 21-225_57F4_LC | . | | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_58D11_LC | . | . | . | . | . | . | . | . | . | . | . | . | . | . | . | . | . | . | . | . | . | . | . | . | . | . |
| 21-225_58F5_LC | . | . | . | . | . | . | . | . | . | . | . | . | . | . | . | . | . | . | L | . | . | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | . | . | . | . | . | . | . | . | . | . | . | . | . |
| 21-225_60A3_LC | . | . | . | . | . | . | . | . | . | . | . | . | . | . | . | S | . | . | . | . | . | . | . | . | . | . |
| 21-225_62G3_LC | . | . | . | . | . | T | . | . | . | . | . | . | . | . | . | . | . | . | . | . | . | . | . | . | . | . |
| 21-225_64A12_LC | T | . | . | . | . | T | . | . | . | . | . | . | . | . | . | T | . | . | . | . | . | . | . | . | . | . |
| 21-225_66F9_LC | . | . | . | . | . | T | . | . | . | . | . | . | . | . | . | . | . | . | . | . | . | . | . | . | O | . |
| 21-225_67B7_LC | . | . | . | . | . | . | . | . | . | . | . | . | . | . | . | . | . | . | . | . | . | . | . | . | . | . |
| 21-225_68G8_LC | . | . | . | . | . | . | . | . | . | . | . | . | . | . | . | . | V | . | . | . | . | . | . | . | . | . |
| 21-225_71B11_LC | . | . | . | . | . | . | . | . | . | . | . | . | . | . | . | . | . | . | . | . | . | . | . | . | . | . |
| 21-225_71B6_LC | Q | . | . | . | . | . | . | . | . | . | . | . | . | . | . | . | . | . | . | . | . | . | . | . | . | . |
| 21-225_71H6_LC | . | . | . | . | . | . | . | . | . | . | . | . | . | . | . | . | . | . | . | . | . | . | . | . | . | . |
| 21-225_73A6_LC | . | . | . | . | . | . | . | . | . | . | . | . | . | . | . | N | . | . | . | . | . | . | . | . | . | . |
| 21-225_74A11_LC | . | . | . | O | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . | . | . | . | . | . | . | . |
| 21-225_76H10_LC | . | . | . | . | . | G | . | . | . | . | . | . | . | . | . | . | . | . | . | . | . | . | . | . | . | . |
| 21-225_76H12_LC | . | . | . | . | . | . | . | . | . | . | . | . | . | . | . | R | . | . | . | . | . | . | . | . | . | . |
| 21-225_84H12_LC | . | . | . | . | . | . | . | . | . | . | . | . | . | . | . | . | . | . | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | . | . | G | . | . | . | . | . | . | . | . | V | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_14D6_LC | . | . | . | . | . | . | . | . | . | . | . | . | . | . | . | . | . | . | . | . | . | . | . | . | . | . |
| 21-225_157C3_LC | . | . | . | . | . | . | . | . | . | D | . | . | . | . | . | . | . | . | . | . | D | . | . | . | . | . |
| 21-225_157G7_LC | . | . | . | . | . | . | . | . | . | . | . | . | . | . | . | . | . | V | . | X | . | . | . | . | . | . |
| 21-225_157H12_LC | . | . | . | . | . | . | . | . | . | . | . | . | . | . | . | . | . | . | . | . | . | . | . | . | . | . |
| 21-225_157H4_LC | . | . | . | . | . | . | . | . | . | . | . | . | . | . | . | . | . | . | . | . | . | . | . | . | . | . |
| 21-225_157H8_LC | . | . | . | . | . | . | . | . | . | D | . | . | . | . | . | . | . | . | . | . | D | . | . | . | . | . |
| 21-225_158F5_LC | . | . | . | . | . | . | . | . | . | . | . | . | . | . | . | . | . | . | . | . | . | . | . | . | . | . |
| 21-225_158G8_LC | . | . | . | . | . | . | . | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . | . | . | . | . | . | . | . |
| 21-225_159C5_LC | . | . | . | . | . | . | . | . | . | D | . | . | . | . | . | I | . | . | . | . | D | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | . | . | . | . | . | . | . | . | . | . | . | . | . | V | . | . | . | . | . | . | . | . |
| 21-225_161D11_LC | . | . | . | . | . | . | . | . | A | . | . | . | . | . | . | G | . | V | . | X | . | . | . | . | . | . |
| 21-225_161F7_LC | . | . | . | . | . | . | . | . | A | . | . | . | V | . | . | G | . | V | . | X | . | . | . | . | . | . |
| 21-225_162F2_LC | . | . | . | . | . | . | . | . | . | . | . | . | . | . | . | . | . | V | . | . | . | . | . | . | . | . |
| 21-225_162H3_LC | . | . | . | . | . | . | . | . | . | . | . | . | . | S | . | . | . | . | . | . | . | . | . | . | . | . |
| 21-225_163E2_LC | . | . | . | . | . | . | . | . | . | . | . | . | . | S | . | . | . | . | . | . | . | . | . | . | . | . |
| 21-225_163F9_LC | . | . | . | . | . | . | . | . | . | . | . | . | . | . | . | . | . | . | . | . | . | . | . | A | . | . |
| 21-225_163G10_LC | . | . | . | . | . | . | . | . | . | . | . | . | . | . | . | . | . | . | . | . | . | . | . | . | . | . |
| 21-225_16A11_LC | . | . | . | . | . | . | . | . | . | . | . | . | . | . | . | . | . | . | . | . | . | . | . | . | . | . |
| 21-225_16H1_LC | . | . | . | . | . | . | . | . | . | . | . | . | . | . | . | . | . | . | . | . | . | . | . | . | . | . |
| 21-225_16H9_LC | . | . | . | . | . | . | . | . | . | . | . | . | . | . | . | . | . | . | . | . | . | . | . | . | . | . |
| 21-225_171A11_LC | . | . | . | . | . | . | . | . | . | . | . | . | . | . | . | . | . | . | . | . | . | . | . | . | . | . |
| 21-225_177B6_LC | . | . | . | . | . | . | . | . | . | . | . | . | . | . | . | N | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | S | . |
| 21-225_181A8_LC | . | . | . | . | . | . | . | . | . | D | . | . | . | . | . | . | . | . | . | . | . | . | . | . | S | . |
| 21-225_184D11_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_184H10_LC | . | | | | | | | | | | | | | | | | | | | | | | | | | |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | . | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | . | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | . | . | . | . | . | . | . | . | . | . | . | . | . | . | . | . | . | . | . | . | I | . |
| 21-225_203B9_LC | . | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | . | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | A | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | . | . | . | . | . | . | . | . | . | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | . | . | . | . | . | . | . | . | . | . | . | V | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_29D9_LC | . | . | . | . | . | . | . | . | . | . | . | . | . | . | . | . | . | . | . | . | . | . | . | . | . | . |
| 21-225_30H11_LC | . | . | . | . | . | . | . | . | . | . | . | . | . | . | . | . | . | . | . | . | . | . | . | . | . | . |
| 21-225_31G9_LC | . | . | . | . | . | . | . | . | . | . | . | . | . | . | . | . | . | . | . | . | . | . | . | . | . | . |
| 21-225_43B1_LC | . | . | . | . | . | . | . | . | . | . | . | . | . | . | V | . | . | . | . | . | . | . | . | . | . | . |
| 21-225_43E1_LC | . | . | . | . | . | . | . | . | . | . | . | . | . | . | . | . | . | . | . | . | . | . | . | . | . | . |
| 21-225_43E5_LC | . | . | . | . | . | . | . | . | . | . | . | . | . | . | V | . | . | . | . | . | . | . | . | . | . | . |
| 21-225_43H8_LC | . | . | . | . | . | . | . | . | . | . | . | . | . | S | . | . | . | . | . | . | . | . | . | . | . | . |
| 21-225_44C8_LC | . | . | . | R | . | . | . | . | . | . | . | . | . | . | . | . | . | . | . | . | . | . | . | . | . | . |
| 21-225_45H2_LC | . | . | . | . | . | . | . | . | . | . | . | . | . | . | V | . | . | . | . | . | . | . | . | . | . | . |
| 21-225_46E9_LC | . | . | . | . | . | . | . | . | . | . | . | . | . | . | V | . | . | . | . | . | . | . | . | . | . | . |
| 21-225_47H7_LC | . | . | . | . | . | . | . | . | . | . | . | . | . | . | V | . | . | . | . | . | . | . | . | . | . | . |
| 21-225_48E10_LC | . | . | . | . | . | . | . | . | . | . | . | . | . | . | . | . | . | . | . | . | . | . | . | . | . | . |
| 21-225_50C4_LC | . | . | . | . | . | . | . | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . | . | . | . | . | . | . | . |
| 21-225_51E4_LC | . | . | . | . | . | . | . | . | . | . | . | . | . | . | . | . | . | . | . | . | . | . | . | . | A | . |
| 21-225_51F9_LC | . | . | . | . | . | . | . | . | . | G | . | . | . | . | . | . | . | . | . | . | . | . | . | . | S | . |
| 21-225_52B9_LC | . | . | . | . | . | . | . | . | . | . | . | . | . | . | . | R | . | . | . | . | . | . | . | . | . | . |
| 21-225_52H12_LC | . | . | . | . | . | . | . | . | . | . | . | . | . | . | . | . | . | . | . | . | . | . | . | . | . | . |
| 21-225_53B10_LC | . | . | . | . | . | . | . | . | . | . | . | . | . | . | . | . | . | . | . | . | . | . | . | . | . | R |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | . | . | . | . | . | . | S | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | . | R | . | . | . | . | . | . | . | . | . | . | . | . | . | . | . | . | . | . | . | . | S | . |
| 21-225_56A5_LC | . | . | . | . | . | . | . | . | . | . | . | . | . | . | . | . | . | . | . | . | . | . | . | . | . | . |
| 21-225_56H7_LC | . | . | . | . | . | . | . | . | . | . | . | . | . | . | . | . | . | . | . | . | . | . | . | . | . | . |
| 21-225_57A4_LC | . | . | . | . | . | . | . | . | . | . | . | . | . | . | . | . | . | . | . | . | . | . | . | . | . | . |
| 21-225_57B2_LC | . | . | . | . | . | . | . | . | . | . | . | . | . | . | . | . | . | . | . | . | . | . | . | . | . | . |
| 21-225_57F4_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR4 | | | | | | | | | | | | | | | |
| CONSENSUS | S | G | S | G | S | G | - | - | T | E | F | T | L | T | I | S | S | L | Q | P | E | D | F | A | T | Y |
| 21-225_58D11_LC | . | . | . | . | . | . | . | . | . | . | . | . | . | A | . | . | . | . | % | . | . | . | . | . | . | . |
| 21-225_58F5_LC | G | . | . | . | . | . | . | . | . | . | . | . | . | . | . | . | . | . | . | . | . | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | V | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | . | . | . | . | . | . | . | . | . | . | . | . | . | . | . | . | . | . | . | . | . | . | . | . |
| 21-225_64A12_LC | . | . | . | . | . | . | . | . | . | . | . | . | . | . | . | . | . | . | . | . | . | . | . | . | . | . |
| 21-225_66F9_LC | . | . | . | . | . | . | . | . | . | . | . | . | . | . | . | . | . | . | . | . | . | . | . | . | . | . |
| 21-225_67B7_LC | . | . | . | . | . | . | . | . | . | . | S | . | . | . | . | . | . | . | . | . | . | . | . | . | . | . |
| 21-225_68G8_LC | . | . | . | . | . | . | . | . | . | . | . | . | . | . | . | . | . | . | . | . | . | . | . | . | . | . |
| 21-225_71B11_LC | . | . | G | . | . | . | . | . | . | . | S | . | . | . | . | . | . | . | . | . | . | . | . | T | . | . |
| 21-225_71B6_LC | . | . | . | . | . | . | . | . | . | . | . | . | . | . | . | . | . | . | . | D | . | . | . | . | . | . |
| 21-225_71H6_LC | . | . | . | . | . | . | . | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . | . | . | . | . | . | . | . |
| 21-225_74A11_LC | . | . | . | . | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . | . | . | . | . | . | . | . |
| 21-225_76H10_LC | . | . | . | . | . | . | . | . | . | . | . | . | . | . | . | . | . | . | . | . | . | . | . | . | . | % |
| 21-225_76H12_LC | . | . | . | . | . | . | . | . | . | . | . | . | . | . | . | . | . | . | . | . | . | . | . | . | . | . |
| 21-225_84H12_LC | . | . | . | . | . | . | . | . | . | . | . | . | . | . | . | . | . | . | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | . | . | . | . | . | . | . | . | . | . | . | . | . | . | . | % | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_14D6_LC | . | | | . | Y | . | | | | | | | | | | | | | | | | | | | | |
| 21-225_157C3_LC | . | . | | | . | . | | | | | | | | | | | | | | | | | | | | |
| 21-225_157G7_LC | . | | I | . | O | . | | | | | | | | | | | | | | | | | | | | |
| 21-225_157H12_LC | . | | | . | | Y | . | | | | | | | | | | | | | | | | | | | |
| 21-225_157H4_LC | . | | | . | O | . | | | | | | | | | | | | | | | | | | | | |
| 21-225_157H8_LC | . | | | . | . | | | | | | | | | | | | | | | | | | | | | |
| 21-225_158F5_LC | . | | | . | O | . | | | | | | | | | | | | | | | | | | | | |
| 21-225_158G8_LC | . | | | . | . | | | | | | | | | | | | | | | | | | | | | |
| 21-225_158H6_LC | . | | | . | . | | | | | | | | | | | | | | | | | | | | | |
| 21-225_159C5_LC | . | | | . | . | | | | | | | | | | | | | | | | | | | | | |
| 21-225_15B11_LC | . | | | . | . | | | | | | | | | | | | | | | | | | | | | |
| 21-225_160F4_LC | . | | I | . | O | . | | | | | | | | | | | | | | | | | | | | |
| 21-225_161D11_LC | . | O | . | . | Y | . | R | . | | | | | | | | | | | | | | | | | | |
| 21-225_161F7_LC | . | O | . | L | Y | I | R | . | | | | | | | | | | | | | | | | | | |
| 21-225_162F2_LC | . | | I | . | O | . | | | | | | | | | | | | | | | | | | | | |
| 21-225_162H3_LC | . | | | . | O | . | | | | | | | | | | | | | | | | | | | | |
| 21-225_163E2_LC | . | | | . | . | | | | | | | | | | | | | | | | | | | | | |
| 21-225_163F9_LC | . | | | . | O | . | | | | | | | | | | | | | | | | | | | | |
| 21-225_163G10_LC | . | | | . | O | Y | . | | | | | | | | | | | | | | | | | | | |
| 21-225_16A11_LC | . | | | . | Y | . | | | | | | | | | | | | | | | | | | | | |
| 21-225_16H1_LC | . | | | . | Y | R | . | | | | | | | | | | | | | | | | | | | |
| 21-225_16H9_LC | . | | | . | Y | R | . | | | | | | | | | | | | | | | | | | | |
| 21-225_171A11_LC | . | | | . | O | R | . | | | | | | | | | | | | | | | | | | | |
| 21-225_177B6_LC | . | | | . | L | . | | | | | | | | | | | | | | | | | | | | |
| 21-225_17E5_LC | . | | | . | . | | | | | | | | | | | | | | | | | | | | | |
| 21-225_17F4_LC | . | | | . | . | | | | | | | | | | | | | | | | | | | | | |
| 21-225_180H7_LC | . | | | . | . | | | | | | | | | | | | | | | | | | | | | |
| 21-225_181A8_LC | . | | | . | . | | | | | | | | | | | | | | | | | | | | | |
| 21-225_184D11_LC | . | | | . | . | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | Y | . | . | . | . | . | . | . | . | . | . | . | . | . | . | . | . | . | . | . | . |
| 21-225_192F6_LC | . | . | . | . | . | Y | . | . | . | . | . | . | . | . | . | . | . | . | . | . | . | . | . | . | . | . |
| 21-225_194F10_LC | . | . | . | . | . | Y | . | . | . | . | . | . | . | . | . | . | . | . | . | . | . | . | . | . | . | . |
| 21-225_195B9_LC | Q | . | . | . | . | Y | R | . | . | . | . | . | . | . | . | . | . | . | . | . | . | . | . | . | . | . |
| 21-225_197D7_LC | V | . | . | . | . | Y | R | . | . | . | . | . | . | . | . | . | . | . | . | . | . | . | . | . | . | . |
| 21-225_197G10_LC | . | . | . | . | . | Y | R | . | . | . | . | . | . | . | . | . | . | . | . | . | . | . | . | . | . | . |
| 21-225_198B9_LC | V | . | . | . | . | Y | R | . | . | . | . | . | . | . | . | . | . | . | . | . | . | . | . | . | . | . |
| 21-225_198E3_LC | V | . | . | . | . | Y | R | . | . | . | . | . | . | . | . | . | . | . | . | . | . | . | . | . | . | . |
| 21-225_200F6_LC | R | . | . | . | . | Y | R | . | . | . | . | . | . | . | . | . | . | . | . | . | . | . | . | . | . | . |
| 21-225_202A3_LC | . | . | . | . | . | H | D | . | . | . | . | . | . | . | . | . | . | . | . | . | . | . | . | . | . | . |
| 21-225_203B9_LC | H | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | . | . | . | . | . | . | . | . | . | . | . |
| 21-225_225D6_LC | Q | . | . | . | . | . | T | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | H | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | F | . | . | . | D | . | . | . | . | . | . | . | . | . | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | R | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_29D9_LC | . | | . | . | . | . | T | . | . | . | . | . | . | . | . | . | . | . | . | . | . | . | . | . | . | . |
| 21-225_30H11_LC | . | . | . | . | D | . | . | . | . | . | . | . | . | . | . | . | . | . | . | . | . | . | . | . | . | . |
| 21-225_31G9_LC | . | | . | . | D | . | . | . | . | . | . | . | . | . | . | . | . | . | . | . | . | . | . | . | . | . |
| 21-225_43B1_LC | F | | . | . | . | T | . | . | . | . | . | . | . | . | . | . | . | . | . | . | . | . | . | . | . | . |
| 21-225_43E1_LC | . | | . | . | . | . | . | . | . | . | . | . | . | . | . | . | . | . | . | . | . | . | . | . | . | . |
| 21-225_43E5_LC | F | | . | . | . | T | . | . | . | . | . | . | . | . | . | . | . | . | . | . | . | . | . | . | . | . |
| 21-225_43H8_LC | . | | . | . | . | . | . | . | . | . | . | . | . | . | . | . | . | . | . | . | . | . | . | . | . | . |
| 21-225_44C8_LC | . | | . | . | . | . | . | . | . | . | . | . | . | . | . | . | . | . | . | . | . | . | . | . | . | . |
| 21-225_45H2_LC | F | | . | . | . | T | . | . | . | . | . | . | . | . | . | . | . | . | . | . | . | . | . | . | . | . |
| 21-225_46E9_LC | F | | . | . | . | T | . | . | . | . | . | . | . | . | . | . | . | . | . | . | . | . | . | . | . | . |
| 21-225_47H7_LC | F | | . | . | . | T | . | . | . | . | . | . | . | . | . | . | . | . | . | . | . | . | . | . | . | . |
| 21-225_48E10_LC | . | | . | . | Y | . | . | . | . | . | . | . | . | . | . | . | . | . | . | . | . | . | . | . | . | . |
| 21-225_50C4_LC | . | | . | . | . | . | R | . | . | . | . | . | . | . | . | . | . | . | . | . | . | . | . | . | . | . |
| 21-225_51B11_LC | . | | . | . | . | . | . | . | . | . | . | . | . | . | . | . | . | . | . | . | . | . | . | . | . | . |
| 21-225_51E4_LC | . | | . | . | . | . | . | . | . | . | . | . | . | . | . | . | . | . | . | . | . | . | . | . | . | . |
| 21-225_51F9_LC | . | | . | . | . | . | . | . | . | . | . | . | . | . | . | . | . | . | . | . | . | . | . | . | . | . |
| 21-225_52B9_LC | . | | . | . | . | . | . | . | . | . | . | . | . | . | . | . | . | . | . | . | . | . | . | . | . | . |
| 21-225_52H12_LC | . | | . | . | Y | . | . | . | . | . | . | . | . | . | . | . | . | . | . | . | . | . | . | . | . | . |
| 21-225_53B10_LC | . | | . | . | Y | . | . | . | . | . | . | . | . | . | . | . | . | . | . | . | . | . | . | . | . | . |
| 21-225_53B12_LC | . | | . | . | . | . | . | . | . | . | . | . | . | . | . | . | . | . | . | . | . | . | . | . | . | . |
| 21-225_53G1_LC | . | | . | . | D | . | . | . | . | . | . | . | . | . | . | . | . | . | . | . | . | . | . | . | . | . |
| 21-225_54D3_LC | . | | . | . | . | . | . | . | . | . | . | . | . | . | . | . | . | . | . | . | . | . | . | . | . | . |
| 21-225_54G7_LC | . | | . | K | . | . | T | . | . | . | . | . | . | . | . | . | . | . | . | . | . | . | . | . | . | . |
| 21-225_55B3_LC | . | | . | . | . | . | . | . | . | . | . | . | . | . | . | . | . | . | . | . | . | . | . | . | . | . |
| 21-225_56A5_LC | . | | . | . | Y | . | . | . | . | . | . | . | . | . | . | . | . | . | . | . | . | . | . | . | . | . |
| 21-225_56H7_LC | . | | . | . | D | . | . | . | . | . | . | . | . | . | . | . | . | . | . | . | . | . | . | . | . | . |
| 21-225_57A4_LC | . | | . | . | . | . | . | . | . | . | . | . | . | . | . | . | . | . | . | . | . | . | . | . | . | . |
| 21-225_57B2_LC | . | | . | . | . | . | . | . | . | . | . | . | . | . | . | . | . | . | . | . | . | . | . | . | . | . |
| 21-225_57F4_LC | H | | . | . | Y | Q | . | . | . | . | . | . | . | . | . | . | . | . | . | . | . | . | . | . | . | . |

## FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_58D11_LC | . | . | . | . | . | . | N | . | . | . | . | . | . | . | . | . | . | . | . | . | . | . | . | . | . | . |
| 21-225_58F5_LC | . | . | . | . | . | . | . | . | . | . | . | . | . | . | . | . | . | . | . | . | . | . | . | . | . | . |
| 21-225_59F11_LC | . | . | . | . | . | . | S | . | . | . | . | . | . | . | . | . | . | . | . | . | . | . | . | . | . | . |
| 21-225_5F4_LC | . | . | . | Y | . | G | . | . | . | . | . | . | . | . | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | . | . | Y | . | . | . | . | . | . | . | . | . | . | . | . | . | . | . | . | . | . | . | . | . |
| 21-225_64A12_LC | . | . | . | . | . | G | . | . | . | . | . | . | . | . | . | . | . | . | . | . | . | . | . | . | . | . |
| 21-225_66F9_LC | . | . | . | . | Y | . | . | . | . | . | . | . | . | . | . | . | . | . | . | . | . | . | . | . | . | . |
| 21-225_67B7_LC | . | . | H | . | . | . | . | . | . | . | . | . | . | . | . | . | . | . | . | . | . | . | . | . | . | . |
| 21-225_68G8_LC | . | . | . | . | Y | . | . | . | . | . | . | . | . | . | . | . | . | . | . | . | . | . | . | . | . | . |
| 21-225_71B11_LC | . | . | . | . | . | . | T | . | . | . | . | . | . | . | . | . | . | . | . | . | . | . | . | . | . | . |
| 21-225_71B6_LC | . | . | . | . | . | . | . | . | . | . | . | . | . | . | . | . | . | . | . | . | . | . | . | . | . | . |
| 21-225_71H6_LC | . | . | . | . | Y | . | . | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . | . | . | . | . | . | . | . |
| 21-225_74A11_LC | F | . | . | . | . | . | G | . | . | . | . | . | . | . | . | . | . | . | . | . | . | . | . | . | . | . |
| 21-225_75C11_LC | . | . | . | . | . | . | G | . | . | . | . | . | . | . | . | . | . | . | . | . | . | . | . | . | . | . |
| 21-225_76H10_LC | . | . | . | . | Y | . | . | . | . | . | . | . | . | . | . | . | . | . | . | . | . | . | . | . | . | . |
| 21-225_76H12_LC | . | . | . | . | . | . | D | . | . | . | . | . | . | . | . | . | . | . | . | . | . | . | . | . | . | . |
| 21-225_84H12_LC | . | . | . | . | . | . | D | . | . | . | . | . | . | . | . | . | . | . | . | . | . | . | . | . | . | . |
| 21-225_86E5_LC | H | . | . | . | . | . | D | . | . | . | . | . | . | . | . | . | . | . | . | . | . | . | . | . | . | . |
| 21-225_88A5_LC | . | . | . | . | . | . | D | . | . | . | . | . | . | . | . | . | . | . | . | . | . | . | . | . | . | . |
| 21-225_96A4_LC | . | . | . | . | . | . | D | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | F | T | F | G | P | G | T | K | V | D | I | K | R |
| 21-225_14D6_LC | . | . | . | . | . | . | . | . | . | . | . | . | . | . | . | . | . | N | . |
| 21-225_157C3_LC | . | . | . | . | . | . | . | . | . | . | . | . | . | . | . | . | . | . | . |
| 21-225_157G7_LC | . | . | . | . | . | . | . | . | . | . | . | . | . | . | . | . | . | . | . |
| 21-225_157H12_LC | . | . | . | . | . | . | . | . | . | . | . | . | . | . | . | . | . | . | . |
| 21-225_157H4_LC | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . |
| 21-225_158F5_LC | . | . | . | . | . | . | . | . | . | . | . | . | . | . | . | . | . | . | . |
| 21-225_158G8_LC | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . |
| 21-225_160F4_LC | . | . | . | . | . | . | . | . | . | . | . | . | . | . | . | . | . | . | . |
| 21-225_161D11_LC | . | . | . | . | . | . | . | . | . | . | . | . | . | . | . | . | . | . | . |
| 21-225_161F7_LC | . | . | . | . | . | . | . | . | . | . | . | . | . | . | . | . | . | . | . |
| 21-225_162F2_LC | . | . | . | . | . | . | . | . | . | . | . | . | . | . | . | . | . | . | . |
| 21-225_162H3_LC | . | . | . | . | . | . | . | . | . | . | . | . | . | . | . | . | . | . | . |
| 21-225_163E2_LC | . | . | . | . | . | . | . | . | . | . | . | . | . | . | . | . | . | . | . |
| 21-225_163F9_LC | . | . | . | . | . | . | . | . | . | . | . | . | . | . | . | . | . | . | . |
| 21-225_163G10_LC | . | . | . | . | . | . | . | . | . | . | . | . | . | . | . | . | . | . | . |
| 21-225_16A11_LC | . | . | . | . | . | . | . | . | . | . | . | . | . | . | . | . | . | . | . |
| 21-225_16H1_LC | . | . | . | . | . | . | . | . | . | . | . | . | . | . | . | . | V | . | . |
| 21-225_16H9_LC | . | . | . | . | L | . | . | . | . | . | . | . | . | . | . | . | . | . | . |
| 21-225_171A11_LC | . | . | . | . | . | . | . | . | . | . | . | . | . | . | . | . | . | . | . |
| 21-225_177B6_LC | . | . | . | . | . | . | . | . | . | . | . | . | . | . | . | . | . | . | . |
| 21-225_17E5_LC | . | . | . | . | . | L | . | . | . | . | . | . | . | . | . | V | . | . | . |
| 21-225_17F4_LC | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . |
| 21-225_181A8_LC | . | . | . | . | . | . | . | . | . | . | . | . | . | . | . | . | . | . | . |
| 21-225_184D11_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | F | T | F | G | P | G | T | K | V | D | I | K | R |
| 21-225_184H10_LC | | | | | | | | | | | | | | | | | | | |
| 21-225_190D6_LC | | | | | . | | | | | | | | | | | | | | |
| 21-225_192F6_LC | | | | | . | | | | | | | | | | | | | | |
| 21-225_194F10_LC | | | | | . | | | | | | | | | | | | | | |
| 21-225_195B9_LC | | | | | | | | | | | | | | | | | . | | |
| 21-225_197D7_LC | | | | | | | | | | | | | | | | | . | | |
| 21-225_197G10_LC | | | | | | | | | | | | | | | | | | | |
| 21-225_198B9_LC | | | | | | | | | | | | | | | | | . | | |
| 21-225_198E3_LC | | | | | | | | | | | | | | | | | . | | |
| 21-225_200F6_LC | | | | | | | | | | | | | | | | | | | |
| 21-225_202A3_LC | | | | | | | | | | | | | | | | | | | |
| 21-225_203B9_LC | | | | | | | | | | | | | | | | | | | |
| 21-225_210E4_LC | | | | | | | | | | | | | | | | | | | |
| 21-225_225B6_LC | | | | | | | | | | | | | | | | | | | |
| 21-225_225D8_LC | | | | | | | | | | | | | | | | | | | |
| 21-225_225F8_LC | | | | | | | | | | | | | | | | | | | |
| 21-225_226A9_LC | | | | | | | | | | | | | | | | | | | |
| 21-225_227C12_LC | | | | | | | | | | | | | | | | | | | |
| 21-225_227G3_LC | | | | | | | | | | | | | | | | | | | |
| 21-225_227H5_LC | | | | | | | | | | | | | | | | | | | |
| 21-225_22H3_LC | | | | | | | | | | | | | | | | | | | |
| 21-225_25A9_LC | | | | | | | | | | | | | | | | | | | |
| 21-225_25B6_LC | | | | | | | | | | | | | | | | | | | |
| 21-225_25G5_LC | | | | | | | | | | | | | | | | | | | |
| 21-225_27D5_LC | | | | | | | | | | | | | | | | | | | |
| 21-225_28A9_LC | | | | | | | | | | | | | | | | | | | |
| 21-225_28B8_LC | | | | | | | | | | | | | | | | | | N | |
| 21-225_29D7_LC | | | | | | | | | | | | | | | | | | | |
| 21-225_29D8_LC | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | F | T | F | G | P | G | T | K | V | D | I | K | R |
| 21-225_29D9_LC | . | . | . | . | . | . | . | . | . | . | . | . | . | . | . | . | . | . | . |
| 21-225_30H11_LC | . | . | . | . | . | . | . | . | . | . | . | . | . | . | . | . | . | T | . |
| 21-225_31G9_LC | . | . | . | . | . | . | . | . | . | . | . | . | . | . | . | . | . | . | . |
| 21-225_43B1_LC | . | . | . | . | . | . | . | . | . | . | . | . | . | . | . | . | . | . | . |
| 21-225_43E1_LC | . | . | . | . | . | . | . | . | . | . | . | . | . | . | . | . | . | . | . |
| 21-225_43E5_LC | . | . | . | . | . | . | . | . | . | . | . | . | . | . | . | . | . | . | . |
| 21-225_43H8_LC | . | . | . | . | . | . | . | . | . | . | . | . | . | . | . | . | . | . | . |
| 21-225_44C8_LC | . | . | . | . | . | . | . | . | . | . | . | . | . | . | . | . | . | . | . |
| 21-225_45H2_LC | . | . | . | . | . | . | . | . | . | . | . | . | . | . | . | . | . | . | . |
| 21-225_46E9_LC | . | . | . | . | . | . | . | . | . | . | . | . | . | . | . | . | . | . | . |
| 21-225_47H7_LC | . | . | . | . | . | . | . | . | . | . | . | . | . | . | . | . | . | . | . |
| 21-225_48E10_LC | . | . | . | . | . | . | . | . | . | . | . | . | . | . | . | . | . | . | . |
| 21-225_50C4_LC | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . |
| 21-225_51E4_LC | . | . | . | . | . | . | . | . | . | . | . | . | . | . | . | . | . | . | . |
| 21-225_51F9_LC | . | . | . | . | . | . | . | . | . | . | . | . | . | . | . | . | . | . | . |
| 21-225_52B9_LC | . | . | . | . | . | . | . | . | . | . | . | . | . | . | . | . | . | . | . |
| 21-225_52H12_LC | . | . | . | . | . | . | . | . | . | . | . | . | . | . | . | . | . | . | . |
| 21-225_53B10_LC | . | . | . | . | . | . | . | . | . | . | . | . | . | . | . | . | . | . | . |
| 21-225_53B12_LC | . | . | . | . | . | . | . | . | . | . | . | . | . | . | . | . | . | . | . |
| 21-225_53G1_LC | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . |
| 21-225_56A5_LC | . | . | . | . | . | . | . | . | . | . | . | . | . | . | . | . | . | . | . |
| 21-225_56H7_LC | . | . | . | . | . | . | . | . | . | . | . | . | . | . | . | S | . | . | . |
| 21-225_57A4_LC | . | . | . | . | . | . | . | . | . | . | . | . | . | . | . | . | . | . | . |
| 21-225_57B2_LC | . | . | . | . | . | . | . | . | . | . | . | . | . | . | . | . | . | . | . |
| 21-225_57F4_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K FR4 | | | | | | |
| CONSENSUS | - | - | - | - | Y | P | F | T | F | G | P | G | T | K | V | D | I | K | R |
| 21-225_58D11_LC | . | . | . | . | Y | . | . | . | . | . | . | . | . | . | . | . | . | . | . |
| 21-225_58F5_LC | . | . | . | . | . | . | . | . | . | . | . | . | . | . | . | E | . | . | . |
| 21-225_59F11_LC | . | . | . | . | . | . | . | . | . | . | . | . | . | . | . | . | Y | . | . |
| 21-225_5F4_LC | . | . | . | . | . | . | . | . | . | . | . | . | . | . | . | . | . | . | . |
| 21-225_60A3_LC | . | . | . | . | . | . | . | . | . | . | . | . | . | . | . | . | . | . | . |
| 21-225_62G3_LC | . | . | . | . | . | . | . | . | . | . | . | . | . | . | . | . | . | . | . |
| 21-225_64A12_LC | . | . | . | . | . | . | . | . | . | . | . | . | . | . | . | . | . | . | . |
| 21-225_66F9_LC | . | . | . | . | . | . | . | . | . | . | . | . | . | . | . | . | . | . | . |
| 21-225_67B7_LC | . | . | . | . | . | . | . | S | . | . | . | . | . | . | . | . | . | . | . |
| 21-225_68G8_LC | . | . | . | . | . | . | . | . | . | . | . | . | . | . | . | . | . | . | . |
| 21-225_71B11_LC | . | . | . | . | . | . | . | . | . | . | . | . | . | . | . | . | V | . | . |
| 21-225_71B6_LC | . | . | . | . | . | . | . | . | . | . | . | . | . | . | . | . | . | . | . |
| 21-225_71H6_LC | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . |
| 21-225_74A11_LC | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . |
| 21-225_76H10_LC | . | . | . | . | . | . | . | . | . | . | . | . | . | . | . | . | . | . | . |
| 21-225_76H12_LC | . | . | . | . | . | . | . | . | . | . | . | . | . | . | . | . | . | . | . |
| 21-225_84H12_LC | . | . | . | . | . | . | . | . | . | . | . | . | . | . | . | . | . | . | . |
| 21-225_86E5_LC | . | . | . | . | . | . | . | . | . | . | . | . | . | . | E | E | . | . | . |
| 21-225_88A5_LC | . | . | . | . | . | . | . | . | . | . | . | . | . | . | E | . | . | . | . |
| 21-225_96A4_LC | . | . | . | . | . | . | . | . | . | R | . | . | . | . | . | . | . | . | . |

**FIGURE 57**

Table 101.     Consensus 21 - VK1|L1/JK4 (SEQ ID NO: 50318):

DIQMTQSPSSLSASVGDRVTITCRAS--QGIS------NYLAWFQQKPGKAPKSLIYA-------ASSLQSGVPSKFSGSGSG--
TDFTLTISSLQPEDFATYYCQQYST----------------------YPLTFGGGTKVEIKR wherein:

A at position 25 can be substituted with T

S at position 26 can be substituted with N

G at position 30 can be substituted with D, A, V or S

S at position 32 can be substituted with G, N, R, A or F

N at position 39 can be substituted with K, R, H, S, T or I

Y at position 40 can be substituted with H, C or D

A at position 42 can be substituted with D, V, I or N

A at position 58 can be substituted with K, S, T, V, D or G

A at position 67 an be substituted with T or V

S at position 68 can be substituted with P

S at position 69 can be substituted with N or R

Q at position 71 can be substituted with L, E or H

S at position 72 can be substituted with G, N or T

Q at position 107 can be substituted with L or H

Q at position 108 can be substituted with H, R or Y

Y at position 109 can be substituted with S, C or T

S at position 110 can be substituted with L, N, M, D, H, V, I or Y

**FIGURE 57**

T at position 111 can be substituted with N, S or K

Y at position 135 can be substituted with F, I or S

L at position 137 can be substituted with V, F or N

T at position 138 can be substituted with I, Q or S

Arg Xaa Xaa Gln Xaa Ile Xaa Xaa Xaa Leu Xaa (SEQ ID NO: 50138)

Xaa Xaa Xaa Xaa Leu Xaa Xaa (SEQ ID NO: 50139)

Xaa Xaa Xaa Xaa Xaa Xaa Pro Xaa Xaa (SEQ ID NO: 50140)

Arg Ala Ser Gln Gly Ile Ser Asn Tyr Leu Ala (SEQ ID NO: 50624)

Ala Ala Ser Ser Leu Gln Ser (SEQ ID NO: 50625)

Gln Gln Tyr Ser Thr Tyr Pro Leu Thr (SEQ ID NO: 50626)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_147E9_LC | . | . | . | . | . | . | . | . | . | . | . | . | . | . | . | . | . | . | . | . | . | . | . | . | . | . |
| 21-225_150G8_LC | . | . | . | . | . | . | . | . | . | . | . | . | . | . | . | . | . | . | . | . | . | . | . | . | . | . |
| 21-225_153F9_LC | . | . | . | . | . | . | . | . | . | . | . | . | . | . | . | . | . | . | . | . | . | . | . | . | . | . |
| 21-225_157C1_LC | . | . | . | . | . | . | . | . | . | . | . | . | . | I | . | . | . | . | I | . | L | . | . | . | . | . |
| 21-225_160H4_LC | . | . | . | . | . | . | . | . | . | . | . | . | . | . | . | . | . | . | . | . | . | . | . | . | . | . |
| 21-225_163G6_LC | . | . | . | . | . | . | . | . | . | . | . | . | . | . | . | . | . | . | . | . | . | . | . | . | . | . |
| 21-225_166H12_LC | . | . | . | . | . | . | . | . | . | . | . | . | T | . | . | . | . | . | . | . | . | . | . | . | . | . |
| 21-225_169C10_LC | . | . | . | . | . | . | . | . | . | . | . | . | . | . | . | . | . | . | . | . | . | . | . | . | . | . |
| 21-225_16A12_LC | . | . | . | . | . | . | . | . | . | . | . | . | . | . | . | . | . | . | . | . | . | . | . | . | . | . |
| 21-225_16D10_LC | . | . | . | . | . | . | . | . | . | . | . | . | . | . | . | . | . | . | . | . | . | . | . | . | . | . |
| 21-225_170D1_LC | . | . | . | . | . | . | . | . | . | . | . | A | . | . | . | . | . | . | . | . | . | . | . | . | . | . |
| 21-225_170D6_LC | . | . | . | . | . | . | . | . | . | . | . | . | . | . | . | . | . | . | A | . | . | . | . | . | . | . |
| 21-225_171H12_LC | . | . | . | . | . | . | . | . | . | . | . | . | . | . | . | . | . | . | . | . | . | . | . | . | . | . |
| 21-225_175G3_LC | . | . | . | . | . | . | . | . | . | . | . | . | . | . | . | . | . | . | . | . | . | . | . | . | . | . |
| 21-225_177A5_LC | . | . | . | . | . | . | . | . | . | . | . | . | . | . | . | . | . | . | . | . | . | . | . | . | . | . |
| 21-225_178H4_LC | . | . | . | . | . | . | . | . | . | . | . | . | . | . | . | . | . | . | . | . | . | . | . | . | T | . |
| 21-225_190C11_LC | . | . | . | . | . | . | . | . | . | . | . | . | . | . | . | . | . | . | . | . | . | . | . | . | T | . |
| 21-225_190E10_LC | . | . | . | . | A | . | . | . | . | . | . | . | . | . | . | . | . | . | . | . | . | . | . | . | T | . |
| 21-225_190E6_LC | . | . | . | . | . | . | . | . | . | . | . | . | . | . | . | . | . | . | . | . | . | . | . | . | T | . |
| 21-225_190F8_LC | . | . | . | . | . | . | . | . | . | . | . | . | . | . | . | . | . | . | . | . | . | . | . | . | T | . |
| 21-225_190G11_LC | . | . | . | . | . | . | . | . | . | . | . | . | . | . | . | . | . | . | . | . | . | . | . | . | T | . |
| 21-225_190G4_LC | . | . | . | . | . | . | . | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . | . | . | . | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | . | . | . | . | . | . | . | . | . | . | . | . | . | . | . | . | . | . | . | . | . | T | . |
| 21-225_191G12_LC | . | . | . | . | . | . | . | . | . | . | . | . | . | . | . | . | . | . | . | . | . | . | . | . | T | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | I | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_192D3_LC | . | . | . | . | . | . | . | . | . | . | . | . | . | . | . | . | . | . | . | . | . | . | . | . | . | . |
| 21-225_192G6_LC | . | . | % | . | . | . | . | . | . | . | . | . | . | . | . | . | . | . | . | . | . | . | . | . | T | . |
| 21-225_192G8_LC | . | . | . | . | . | . | . | . | . | . | . | . | . | . | . | . | . | . | . | . | . | . | . | . | T | . |
| 21-225_192H10_LC | . | . | . | . | . | . | . | . | . | . | . | . | . | . | . | . | . | . | . | . | . | . | . | . | . | . |
| 21-225_192H4_LC | . | . | . | . | . | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . | . | . | . | . | . | . | N |
| 21-225_197B6_LC | . | . | . | . | . | . | . | . | . | . | . | . | . | . | . | . | . | . | . | . | . | . | . | . | . | . |
| 21-225_197D3_LC | . | . | . | . | . | . | . | . | . | . | . | . | . | . | . | . | . | . | . | . | . | . | . | . | . | . |
| 21-225_197F2_LC | . | . | . | } | . | . | . | . | . | . | . | . | . | . | . | . | . | . | . | . | . | . | . | . | T | . |
| 21-225_197G12_LC | . | . | . | } | . | . | . | . | . | . | . | . | . | . | . | . | . | . | . | . | . | . | . | . | T | . |
| 21-225_198B6_LC | . | . | . | . | . | . | . | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . | . | . | . | . | . | . | . |
| 21-225_1F1_LC | . | . | . | . | . | . | . | . | . | . | . | . | . | . | . | . | . | . | . | . | . | . | . | . | . | . |
| 21-225_203H1_LC | . | . | . | . | . | . | . | . | . | . | . | . | . | . | . | . | . | . | . | . | . | . | . | . | T | . |
| 21-225_204G6_LC | . | . | % | . | . | . | . | . | . | . | . | . | . | . | . | . | . | . | } | . | . | . | . | . | T | . |
| 21-225_204G8_LC | . | . | . | . | . | . | . | . | . | . | . | . | . | . | . | . | . | . | . | . | . | . | . | . | . | . |
| 21-225_205F5_LC | . | . | . | . | . | . | . | . | . | . | . | . | . | . | . | . | . | . | . | . | . | . | . | . | . | . |
| 21-225_205H3_LC | . | . | P | . | . | . | . | . | . | . | . | . | . | . | . | . | . | . | . | . | . | . | . | . | . | . |
| 21-225_207G6_LC | . | . | . | . | . | . | . | . | . | . | . | . | . | . | . | . | . | . | . | . | . | . | . | . | . | . |
| 21-225_211A11_LC | . | . | . | . | . | . | . | . | . | . | . | . | . | . | . | . | . | . | . | . | . | . | . | . | . | . |
| 21-225_211A3_LC | . | . | % | . | . | . | . | . | . | . | . | . | . | . | . | . | . | . | . | . | . | . | . | . | . | . |
| 21-225_211A8_LC | . | . | % | . | . | . | . | P | . | . | . | . | P | . | . | . | . | . | . | . | . | . | . | . | . | . |
| 21-225_212D7_LC | . | . | . | . | . | . | . | . | . | . | . | . | . | . | . | . | . | . | . | . | . | . | . | . | . | . |
| 21-225_212E10_LC | . | . | . | . | . | . | . | . | . | . | . | . | . | . | . | . | . | . | . | . | . | . | . | . | . | . |

## FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_212E6_LC | . | . | . | . | . | . | . | . | . | . | . | . | . | . | . | . | . | . | . | . | . | . | . | . | . | . |
| 21-225_212F10_LC | . | . | . | . | . | . | . | . | . | . | . | . | . | . | . | . | . | . | . | . | . | . | . | . | . | . |
| 21-225_212H11_LC | . | . | S | . | . | . | . | . | . | . | . | . | . | . | . | . | . | . | . | . | . | . | . | . | . | . |
| 21-225_212H9_LC | . | . | S | . | . | . | . | . | . | . | . | . | . | . | . | . | . | . | . | . | . | . | . | . | . | . |
| 21-225_213B8_LC | . | . | . | . | . | . | . | . | . | . | . | . | . | . | . | . | . | . | . | . | . | . | . | . | . | . |
| 21-225_213C4_LC | . | . | S | . | . | . | . | . | . | . | . | . | . | . | . | . | . | . | . | . | . | . | . | . | . | . |
| 21-225_213D2_LC | . | . | . | . | . | . | . | . | . | . | . | . | . | . | . | . | . | . | . | . | . | . | . | . | . | . |
| 21-225_213E5_LC | . | . | S | . | . | . | . | . | . | . | . | . | . | . | . | . | . | . | . | . | . | . | . | . | . | . |
| 21-225_215A12_LC | . | . | . | . | . | . | . | . | . | . | . | . | . | . | . | . | . | . | I | . | . | . | . | . | T | . |
| 21-225_215B5_LC | . | . | . | . | . | . | . | . | . | . | . | . | . | . | . | . | . | . | . | . | . | . | . | . | . | . |
| 21-225_215D6_LC | . | . | . | . | . | . | . | . | . | . | . | . | . | . | . | . | . | . | . | . | . | . | . | . | . | . |
| 21-225_216G1_LC | . | . | S | . | . | . | . | . | . | . | . | . | . | . | . | . | . | . | . | . | . | . | . | . | . | . |
| 21-225_217B10_LC | . | . | S | . | . | . | . | . | . | . | . | . | . | . | . | . | . | . | . | . | . | . | . | . | . | . |
| 21-225_217G5_LC | . | . | L | . | . | . | . | . | . | . | . | . | . | . | . | . | . | . | . | . | . | . | . | . | . | . |
| 21-225_219H1_LC | . | . | . | . | . | . | . | . | . | . | Q | . | . | . | . | . | . | . | . | . | . | . | . | . | . | . |
| 21-225_221F6_LC | . | . | . | . | . | . | . | . | . | . | . | . | G | . | . | . | . | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | . | . | . | . | . | . | . | . | . | . |
| 21-225_22A2_LC | . | . | . | . | . | . | . | . | . | . | . | . | . | . | . | . | . | . | . | . | . | . | . | . | . | . |
| 21-225_23D11_LC | . | . | . | . | . | . | . | . | . | . | . | . | . | . | . | . | . | . | . | . | . | . | . | . | . | . |
| 21-225_32G3_LC | . | . | . | . | . | . | T | . | . | . | . | . | . | . | . | . | . | . | . | . | . | . | . | . | . | . |
| 21-225_55A11_LC | . | . | . | . | . | . | . | . | . | . | . | . | . | . | . | . | . | . | . | . | . | . | . | . | . | . |
| 21-225_55D4_LC | . | . | . | . | . | . | . | . | . | . | S | . | . | . | . | . | . | . | . | . | . | . | . | . | . | . |
| 21-225_65H11_LC | . | . | . | . | . | . | . | . | . | . | . | . | . | . | . | . | . | . | . | . | . | . | . | . | . | . |
| 21-225_70H6_LC | . | . | . | . | . | . | . | . | . | . | . | . | . | . | L | . | . | . | . | . | . | . | . | . | T | . |
| 21-225_74C8_LC | . | . | . | . | . | . | . | . | . | . | . | . | . | . | . | . | . | . | . | . | . | . | . | . | . | . |
| 21-225_74G6_LC | . | . | . | . | . | . | . | . | . | . | . | . | . | . | . | . | . | . | . | . | . | . | . | . | . | . |
| 21-225_76C5_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_77E6_LC | . | . | . | . | . | . | . | . | . | . | . | . | . | . | . | . | . | . | . | . | . | . | . | . | . | . |
| 21-225_79A12_LC | . | . | . | . | . | . | . | . | . | . | . | . | . | . | . | . | . | . | . | . | . | . | . | . | . | . |
| 21-225_79F4_LC | . | . | . | . | . | . | . | . | . | . | . | . | . | . | . | . | . | . | . | . | . | . | . | . | . | . |
| 21-225_7F10_LC | . | . | . | . | . | . | . | . | . | A | . | . | . | . | . | . | . | . | . | . | . | . | . | . | . | . |
| 21-225_80C11_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_147E9_LC | . | . | . | D | . | N | . | . | . | . | . | . | . | . | . | . | . | . | . | . | . | . | . | . | . | . |
| 21-225_150G8_LC | . | . | . | . | . | . | . | . | . | . | . | . | S | . | . | . | . | . | . | . | . | . | . | . | . | . |
| 21-225_153F9_LC | . | . | . | . | . | . | . | . | . | . | . | . | . | . | . | . | . | . | . | . | . | . | . | . | . | . |
| 21-225_157C1_LC | . | . | . | . | . | . | . | . | . | . | . | . | . | . | . | . | . | . | . | . | R | . | . | . | . | . |
| 21-225_160H4_LC | . | . | . | D | . | . | . | . | . | . | . | . | . | . | . | V | . | . | . | . | L | . | . | . | . | . |
| 21-225_163G6_LC | . | . | . | D | . | . | . | . | . | . | . | . | . | . | . | V | . | . | . | . | . | . | . | . | . | . |
| 21-225_166H12_LC | . | . | . | D | . | . | . | . | . | . | . | . | S | . | . | . | . | . | . | . | . | . | . | . | . | . |
| 21-225_169C10_LC | . | . | . | D | . | . | . | . | . | . | . | . | . | . | . | . | . | . | . | . | . | . | . | . | . | . |
| 21-225_16A12_LC | . | . | . | A | . | N | . | . | . | . | . | . | T | . | . | . | . | . | . | . | . | . | . | . | . | . |
| 21-225_16D10_LC | . | . | . | D | . | . | . | . | . | . | . | . | . | . | . | . | . | . | . | . | . | . | . | . | . | . |
| 21-225_170D1_LC | . | . | . | D | . | . | . | . | . | . | . | . | . | . | . | . | . | . | . | . | . | . | . | . | . | . |
| 21-225_170D6_LC | . | . | . | D | . | G | . | . | . | . | . | . | . | . | C | . | . | . | . | . | . | . | . | . | . | . |
| 21-225_171H12_LC | . | . | . | D | . | . | . | . | . | . | . | . | . | . | . | . | . | . | . | . | . | . | . | . | . | . |
| 21-225_175G3_LC | . | . | . | D | . | . | . | . | . | . | . | . | . | . | G | . | . | . | . | . | . | . | . | . | . | . |
| 21-225_177A5_LC | . | . | . | D | . | . | . | . | . | . | . | . | . | . | . | . | . | . | . | . | . | . | . | . | . | . |
| 21-225_178H4_LC | . | . | . | D | . | . | . | . | . | . | . | . | . | . | . | . | . | . | . | . | . | . | . | . | . | . |
| 21-225_180C11_LC | . | . | . | . | . | G | . | . | . | . | . | . | . | . | . | . | . | . | . | . | . | . | . | . | . | . |
| 21-225_190E10_LC | . | . | . | . | . | G | . | . | . | . | . | . | S | . | . | . | . | . | . | . | . | . | . | . | . | . |
| 21-225_190E6_LC | . | . | . | . | . | G | . | . | . | . | . | . | . | . | . | . | . | . | . | . | . | . | . | . | . | . |
| 21-225_190F8_LC | . | . | . | . | . | G | . | . | . | . | . | . | . | . | . | . | . | . | . | . | . | . | . | . | . | . |
| 21-225_190G11_LC | . | . | . | . | . | G | . | . | . | . | . | . | . | . | . | . | . | . | . | . | . | . | . | . | . | . |
| 21-225_190G4_LC | . | . | . | D | . | G | . | . | . | . | . | . | S | . | . | . | . | . | . | . | . | . | . | . | . | . |
| 21-225_191A10_LC | . | . | . | . | . | G | . | . | . | . | . | . | S | . | . | . | . | . | . | . | . | . | . | . | . | . |
| 21-225_191E10_LC | . | . | . | . | . | G | . | . | . | . | . | . | S | . | . | . | . | . | . | . | . | . | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | . | . | G | . | . | . | . | . | . | S | . | . | . | . | . | . | . | . | . | . | . | . | . |
| 21-225_191G12_LC | . | . | . | . | . | G | . | . | . | . | . | . | . | . | . | . | . | . | . | . | . | . | . | . | . | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_192D3_LC | . | . | . | . | . | . | . | . | . | . | . | . | . | . | . | . | . | . | . | . | . | . | . | . | . | . |
| 21-225_192G6_LC | . | . | . | . | . | G | . | . | . | . | . | . | . | . | . | . | . | . | . | . | . | . | . | . | . | . |
| 21-225_192G8_LC | . | . | . | . | . | G | . | . | . | . | . | . | . | . | . | . | . | . | . | . | . | . | . | . | . | . |
| 21-225_192H10_LC | . | . | . | . | . | G | . | . | . | . | . | . | K | . | . | . | . | . | . | . | . | . | . | . | . | . |
| 21-225_192H4_LC | . | . | . | O | . | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | G | . | . | . | . | . | . | K | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | A | . | . | . | . | . | . | . | . | . | . | . | . | . | . | . | . | . | . | . | . | . | . |
| 21-225_197B6_LC | . | . | . | . | . | G | . | . | . | . | . | . | K | . | . | . | . | . | . | . | . | . | . | . | . | . |
| 21-225_197D3_LC | . | . | . | . | . | R | . | . | . | . | . | . | . | . | . | . | . | . | . | . | . | . | . | . | . | . |
| 21-225_197F2_LC | . | . | . | . | . | G | . | . | . | . | . | . | . | . | . | . | . | . | . | . | . | . | . | . | . | . |
| 21-225_197G12_LC | . | . | . | . | . | G | . | . | . | . | . | . | . | . | . | . | . | . | . | . | . | . | . | . | . | . |
| 21-225_198B6_LC | . | . | . | . | . | G | . | . | . | . | . | . | . | . | . | . | . | . | . | . | . | . | . | . | . | . |
| 21-225_199C5_LC | . | . | . | . | . | . | . | . | . | . | . | . | K | . | . | . | . | . | . | . | . | . | . | . | . | . |
| 21-225_1F1_LC | . | . | . | . | . | N | . | . | . | . | . | . | . | . | . | . | . | . | . | . | . | . | S | . | . | . |
| 21-225_203H1_LC | . | . | . | . | . | G | . | . | . | . | . | . | . | . | . | . | . | . | . | . | . | . | . | . | . | . |
| 21-225_204G6_LC | . | . | . | . | . | G | . | . | . | . | . | . | . | . | . | . | . | . | . | . | . | . | . | . | . | . |
| 21-225_204G8_LC | . | . | . | . | . | . | . | . | . | . | . | . | . | H | . | . | . | . | . | . | . | . | . | . | . | . |
| 21-225_205F5_LC | . | . | . | . | . | G | . | . | . | . | . | . | . | . | . | . | . | . | . | . | . | . | . | . | . | . |
| 21-225_205H3_LC | . | . | . | A | . | . | . | . | . | . | . | . | . | H | . | . | . | . | . | L | . | . | . | . | . | . |
| 21-225_207G6_LC | . | . | . | . | . | R | . | . | . | . | . | . | . | . | . | . | . | L | . | . | . | . | . | . | . | . |
| 21-225_211A11_LC | . | . | . | . | . | G | . | . | . | . | . | . | . | . | . | . | . | . | . | . | . | . | . | . | . | . |
| 21-225_211A3_LC | . | . | . | A | . | G | . | . | . | . | . | . | K | H | . | . | . | . | . | R | . | . | . | . | . | . |
| 21-225_211A8_LC | . | . | . | . | . | . | . | . | . | . | . | . | R | . | . | . | . | V | . | . | . | . | . | . | . | . |
| 21-225_212D7_LC | . | . | . | . | . | . | . | . | . | . | . | . | . | H | . | . | . | . | . | . | . | . | . | . | . | . |
| 21-225_212E10_LC | . | . | . | . | . | . | . | . | . | . | . | . | . | H | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
|  |  |  |  |  |  | K_CDR1 |  |  |  |  |  |  |  |  |  |  |  |  |  |  |  |  |  | K_FR2 |  |  |
| CONSENSUS | - | - | Q | G | I | S | - | - | - | - | - | - | N | Y | L | A | W | F | Q | Q | K | P | G | K | A | P |
| 21-225_212E6_LC | . | . | . | . | . | . | . | . | . | . | . | . | . | R | . | . | . | . | . | . | . | . | . | Q | . | . |
| 21-225_212F10_LC | . | . | . | . | . | . | . | . | . | . | . | . | . | . | . | D | . | . | . | . | . | . | . | . | . | . |
| 21-225_212H11_LC | . | . | . | A | . | G | . | . | . | . | . | . | K | R | . | . | . | . | . | . | R | . | . | . | . | . |
| 21-225_212H9_LC | . | . | . | . | . | . | . | . | . | . | . | . | S | . | . | . | . | L | . | . | . | . | . | . | . | . |
| 21-225_213B8_LC | . | . | . | . | . | . | . | . | . | . | . | . | . | R | . | . | . | . | . | . | . | . | . | . | . | . |
| 21-225_213C4_LC | . | . | . | A | . | S | . | . | . | . | . | . | . | . | . | . | . | C | . | . | . | . | . | . | . | . |
| 21-225_213D2_LC | . | . | . | . | . | . | . | . | . | . | . | . | . | R | . | . | . | . | . | . | . | . | . | . | . | . |
| 21-225_213E5_LC | . | . | . | A | . | G | . | . | . | . | . | . | K | R | . | . | . | . | . | . | R | . | . | . | . | . |
| 21-225_215A12_LC | . | . | . | D | . | S | . | . | . | . | . | . | . | . | . | . | . | . | . | . | . | . | . | . | . | . |
| 21-225_215B5_LC | . | . | . | . | . | . | . | . | . | . | . | . | . | R | . | . | . | . | . | . | . | . | . | . | . | . |
| 21-225_215D6_LC | . | . | . | . | . | . | . | . | . | . | . | . | . | R | . | . | . | . | . | . | . | . | . | . | . | . |
| 21-225_216G1_LC | . | . | . | . | . | . | . | . | . | . | . | . | S | . | . | . | . | L | . | . | . | . | . | . | . | . |
| 21-225_217B10_LC | . | . | . | A | . | G | . | . | . | . | . | . | K | R | . | . | . | . | . | . | R | . | . | . | . | . |
| 21-225_217G5_LC | . | . | . | . | . | G | . | . | . | . | . | . | . | . | . | . | . | . | . | . | R | . | . | . | . | . |
| 21-225_219H1_LC | . | . | . | . | . | . | . | . | . | . | . | . | . | . | . | D | . | . | . | . | . | . | . | . | . | . |
| 21-225_221F6_LC | . | . | . | . | . | . | . | . | . | . | . | . | . | . | . | . | . | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | . | . | . | . | . | . | . | . | . | . |
| 21-225_22A2_LC | . | . | . | D | . | . | . | . | . | . | . | . | . | . | . | . | . | V | . | . | . | . | . | . | . | . |
| 21-225_23D11_LC | . | . | . | . | . | A | . | . | . | . | . | . | . | . | . | . | . | . | . | . | R | . | . | . | . | . |
| 21-225_32G3_LC | . | . | . | A | . | . | . | . | . | . | . | . | . | . | . | . | . | . | . | . | . | . | . | . | . | . |
| 21-225_55A11_LC | . | . | . | D | . | N | . | . | . | . | . | . | I | . | . | . | . | . | . | . | . | . | . | . | . | . |
| 21-225_55D4_LC | . | . | . | D | . | . | . | . | . | . | . | . | . | . | . | I | . | . | . | . | . | . | . | . | . | . |
| 21-225_65H11_LC | . | . | . | D | . | . | . | . | . | . | . | . | T | . | . | . | . | . | . | . | . | . | . | . | . | . |
| 21-225_70H6_LC | . | . | . | S | . | F | . | . | . | . | . | . | . | . | . | N | . | . | . | R | . | . | . | . | . | . |
| 21-225_74C8_LC | . | . | . | . | . | G | . | . | . | . | . | . | R | . | . | . | . | . | . | . | . | . | . | . | . | . |
| 21-225_74G6_LC | . | . | . | . | . | G | . | . | . | . | . | . | R | . | . | . | . | . | . | . | . | . | . | . | . | . |
| 21-225_76C5_LC | . | . | . | V | . | . | . | . | . | . | . | . | R | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | G | I | S | - | - | - | - | - | - | N | Y | L | A | W | F | Q | Q | K | P | G | K | A | P |
| 21-225_77E6_LC | . | . | . | . | . | . | . | . | . | . | . | . | X | . | . | . | . | . | . | . | . | . | . | . | . | . |
| 21-225_79A12_LC | . | . | . | V | . | . | . | . | . | . | . | . | X | . | . | . | . | . | . | . | . | . | . | . | . | . |
| 21-225_79F4_LC | . | . | . | . | . | . | . | . | . | . | . | . | X | . | . | . | . | . | . | . | . | . | . | . | . | . |
| 21-225_7F10_LC | . | . | . | D | . | X | . | . | . | . | . | . | . | . | . | . | . | . | . | . | . | . | . | . | . | . |
| 21-225_80C11_LC | . | . | . | . | . | G | . | . | . | . | . | . | X | . | . | . | . | . | . | . | . | . | . | X | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | S | L | I | Y | A | - | - | - | - | - | - | - | - | A | S | S | L | Q | S | G | V | P | S | K | F |
| 21-225_147E9_LC | . | . | . | . | V | . | . | . | . | . | . | . | . | . | . | . | . | . | . | . | . | . | . | . | . | . |
| 21-225_150G8_LC | . | . | . | . | V | . | . | . | . | . | . | . | . | . | . | . | . | . | . | N | . | . | . | . | S | . |
| 21-225_153F9_LC | . | . | . | . | S | . | . | . | . | . | . | . | . | . | . | . | . | . | . | . | . | . | . | . | . | . |
| 21-225_157C1_LC | . | . | . | . | . | . | . | . | . | . | . | . | . | . | . | . | . | L | . | . | . | . | . | . | . | . |
| 21-225_160H4_LC | . | . | . | . | V | . | . | . | . | . | . | . | . | . | . | . | . | . | . | . | . | . | . | . | X | . |
| 21-225_163G6_LC | . | . | . | . | . | . | . | . | . | . | . | . | . | . | . | . | . | L | . | . | . | . | . | . | . | . |
| 21-225_166H12_LC | . | . | . | . | . | . | . | . | . | . | . | . | . | . | . | . | . | . | . | . | . | . | . | . | . | . |
| 21-225_169C10_LC | . | . | . | . | F | S | . | . | . | . | . | . | . | . | . | . | . | . | . | . | . | . | . | . | . | . |
| 21-225_16A12_LC | . | . | . | . | S | . | . | . | . | . | . | . | . | . | . | . | . | . | . | . | . | . | . | . | . | . |
| 21-225_16D10_LC | . | . | . | . | . | . | . | . | . | . | . | . | . | . | T | F | . | . | . | . | . | . | . | . | . | . |
| 21-225_170D1_LC | . | . | . | . | . | . | . | . | . | . | . | . | . | . | . | . | . | . | . | . | . | . | . | . | F | . |
| 21-225_170D6_LC | . | . | . | . | . | S | . | . | . | . | . | . | . | . | . | . | . | . | . | . | . | . | . | . | . | . |
| 21-225_171H12_LC | . | . | . | . | . | S | . | . | . | . | . | . | . | . | . | . | . | . | . | . | . | . | . | . | . | . |
| 21-225_175G3_LC | . | . | . | . | F | S | . | . | . | . | . | . | . | . | . | . | . | . | . | . | . | . | . | . | . | . |
| 21-225_177A5_LC | . | . | . | . | . | S | . | . | . | . | . | . | . | . | . | . | . | . | . | . | . | . | . | . | . | . |
| 21-225_178H4_LC | . | . | . | . | F | S | . | . | . | . | . | . | . | . | . | . | . | . | . | . | . | . | . | . | . | . |
| 21-225_190C11_LC | . | . | L | . | . | X | . | . | . | . | . | . | . | . | T | . | . | . | . | . | . | . | . | . | . | . |
| 21-225_190E10_LC | . | . | L | . | . | X | . | . | . | . | . | . | . | . | . | . | . | . | . | Q | . | . | . | . | . | . |
| 21-225_190E6_LC | . | . | L | . | . | X | . | . | . | . | . | . | . | . | . | . | . | . | . | . | . | . | . | . | . | . |
| 21-225_190F8_LC | . | . | L | . | . | X | . | . | . | . | . | . | . | . | . | . | . | . | . | . | . | . | . | . | . | . |
| 21-225_190G11_LC | . | . | L | . | . | X | . | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . | . | . | . | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | L | . | X | . | . | . | . | . | . | . | . | V | . | . | . | . | . | . | . | . | . | . | . |
| 21-225_191G12_LC | . | . | . | L | . | X | . | . | . | . | . | . | . | . | . | . | . | . | . | . | . | . | . | . | . | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | S | L | I | Y | A | - | - | - | - | - | - | - | - | A | S | S | L | Q | S | G | V | P | S | K | F |
| 21-225_192D3_LC | . | . | . | . | . | . | . | . | . | . | . | . | . | . | . | . | . | . | . | . | . | . | . | . | . | . |
| 21-225_192G6_LC | . | . | . | L | . | K | . | . | . | . | . | . | . | . | . | . | . | . | . | . | . | . | . | . | . | . |
| 21-225_192G8_LC | . | . | . | L | . | K | . | . | . | . | . | . | . | . | . | . | . | . | . | . | . | . | . | . | . | . |
| 21-225_192H10_LC | . | . | . | . | . | . | . | . | . | . | . | . | . | . | . | . | . | . | . | . | . | . | . | . | . | . |
| 21-225_192H4_LC | . | . | . | . | S | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . | . | . | . | . | . | . | . |
| 21-225_197B6_LC | . | . | . | . | . | G | . | . | . | . | . | . | . | . | . | . | . | . | . | . | . | . | . | . | . | . |
| 21-225_197D3_LC | . | . | . | . | . | . | . | . | . | . | . | . | . | . | . | . | . | . | . | . | . | . | . | . | . | . |
| 21-225_197F2_LC | . | . | . | L | . | K | . | . | . | . | . | . | . | . | T | . | . | . | . | . | . | . | . | . | . | . |
| 21-225_197G12_LC | . | . | . | L | . | K | . | . | . | . | . | . | . | . | T | . | . | . | . | . | . | . | F | . | . | . |
| 21-225_198B6_LC | . | . | . | . | . | . | . | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . | . | . | . | . | . | . | . |
| 21-225_1F1_LC | . | . | . | . | S | V | . | . | . | . | . | . | . | . | . | . | . | . | . | T | . | . | . | . | X | . |
| 21-225_203H1_LC | . | . | . | . | . | V | . | . | . | . | . | . | . | . | . | . | R | . | . | . | . | . | . | . | . | . |
| 21-225_204G6_LC | . | . | . | . | . | . | . | . | . | . | . | . | . | . | . | . | . | . | . | . | . | . | . | . | X | . |
| 21-225_204G8_LC | . | . | . | . | F | . | . | . | . | . | . | . | . | . | . | . | . | . | . | . | . | . | . | . | Q | . |
| 21-225_205F5_LC | . | . | . | . | . | G | . | . | . | . | . | . | . | . | V | . | . | . | . | . | . | . | . | . | . | . |
| 21-225_205H3_LC | . | . | . | . | . | . | . | . | . | . | . | . | . | . | . | . | . | . | . | . | . | . | . | . | . | . |
| 21-225_207G6_LC | . | . | . | . | N | . | . | . | . | . | . | . | . | . | . | . | . | . | . | . | . | . | . | . | . | . |
| 21-225_211A11_LC | R | . | . | . | . | D | . | . | . | . | . | . | . | . | . | . | . | . | E | . | . | . | . | . | . | . |
| 21-225_211A3_LC | . | . | . | . | . | . | . | . | . | . | . | . | . | . | . | . | R | . | . | . | . | . | . | . | . | . |
| 21-225_211A8_LC | . | . | . | . | . | . | . | . | . | . | . | . | . | . | . | . | . | . | . | . | . | . | S | . | X | . |
| 21-225_212D7_LC | . | . | . | . | N | . | . | . | . | . | . | . | . | . | . | . | . | . | . | . | . | . | . | . | . | . |
| 21-225_212E10_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | K_CDR2 | | | | | | | | | | | | | | | | |
| CONSENSUS | K | S | L | I | Y | A | - | - | - | - | - | - | - | - | A | S | S | L | Q | S | G | V | P | S | K | F |
| 21-225_212E6_LC | . | . | . | . | . | . | . | . | . | . | . | . | . | . | . | . | . | . | . | . | . | . | . | . | . | . |
| 21-225_212F10_LC | . | . | . | . | . | S | . | . | . | . | . | . | . | . | . | . | N | . | . | . | . | . | . | . | . | . |
| 21-225_212H11_LC | . | . | . | . | . | . | . | . | . | . | . | . | . | . | . | . | R | . | . | . | . | . | . | . | . | . |
| 21-225_212H9_LC | . | . | . | . | . | . | . | . | . | . | . | . | . | . | . | . | . | . | . | . | . | . | . | . | X | . |
| 21-225_213B8_LC | . | . | . | . | . | . | . | . | . | . | . | . | . | . | . | . | . | . | . | . | . | . | . | . | . | . |
| 21-225_213C4_LC | . | T | . | . | . | . | . | . | . | . | . | . | . | . | . | . | . | . | . | . | . | . | . | . | . | . |
| 21-225_213D2_LC | . | . | . | . | M | . | . | . | . | . | . | . | . | . | . | . | . | . | . | . | . | . | . | . | . | . |
| 21-225_213E5_LC | . | . | . | . | . | . | . | . | . | . | . | . | . | . | . | . | R | . | . | . | . | . | . | . | . | . |
| 21-225_215A12_LC | . | . | . | . | . | . | . | . | . | . | . | . | . | . | . | . | . | . | . | . | . | . | . | . | . | . |
| 21-225_215B5_LC | . | . | . | . | R | . | . | . | . | . | . | . | . | . | . | . | . | . | . | . | . | . | . | . | . | . |
| 21-225_215D6_LC | . | . | . | . | . | . | . | . | . | . | . | . | . | . | . | . | . | . | . | M | . | . | . | . | . | . |
| 21-225_216G1_LC | . | . | . | . | . | . | . | . | . | . | . | . | . | . | . | . | . | . | . | . | . | . | . | . | S | . |
| 21-225_217B10_LC | . | . | . | . | . | . | . | . | . | . | . | . | . | . | . | . | R | . | . | . | . | . | . | . | . | . |
| 21-225_217G5_LC | . | . | . | . | . | . | . | . | . | . | . | . | . | . | . | . | . | . | . | . | . | . | . | . | . | . |
| 21-225_219H1_LC | . | . | . | . | . | S | . | . | . | . | . | . | . | . | . | . | N | . | . | . | . | . | . | . | . | . |
| 21-225_221F6_LC | . | . | . | . | . | . | . | . | . | . | . | . | . | . | . | . | N | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | N | . | . | . | . | . | . | . | . | . |
| 21-225_22A2_LC | . | . | . | . | . | . | . | . | . | . | . | . | . | . | . | . | . | . | . | . | . | . | . | . | . | . |
| 21-225_23D11_LC | . | . | . | . | S | . | . | . | . | . | . | . | . | . | . | . | . | . | . | . | . | . | . | . | . | . |
| 21-225_32G3_LC | . | . | . | . | . | D | . | . | . | . | . | T | . | . | . | . | . | . | . | . | . | . | . | . | . | . |
| 21-225_55A11_LC | . | . | . | . | . | . | . | . | . | . | . | . | . | . | . | . | . | . | . | . | . | . | . | . | . | . |
| 21-225_55D4_LC | . | . | . | . | . | T | . | . | . | . | . | . | . | . | . | . | . | . | . | . | . | . | . | . | . | . |
| 21-225_65H11_LC | . | . | . | . | . | . | . | . | . | . | . | . | . | . | . | . | . | . | . | . | . | . | . | . | . | . |
| 21-225_70H6_LC | . | V | . | . | . | T | . | . | . | . | . | . | . | . | . | . | . | . | . | . | i | . | . | . | S | . |
| 21-225_74C8_LC | . | . | . | . | . | T | . | . | . | . | . | T | . | . | . | . | N | . | . | . | . | A | . | . | . | . |
| 21-225_74G6_LC | . | . | . | . | . | T | . | . | . | . | . | T | . | . | . | . | . | . | . | . | . | A | . | . | . | . |
| 21-225_76C5_LC | . | . | . | . | . | . | . | . | . | . | . | . | . | . | . | . | . | . | . | . | . | A | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | S | L | I | Y | A | - | - | - | - | - | - | - | - | A | S | S | L | Q | S | G | V | P | S | K | F |
| 21-225_77E6_LC | . | . | . | . | . | . | . | . | . | . | . | . | . | . | . | . | . | . | . | G | . | A | . | . | . | . |
| 21-225_79A12_LC | . | . | . | . | . | . | . | . | . | . | . | . | . | . | . | . | . | . | . | . | . | A | . | . | . | . |
| 21-225_79F4_LC | . | . | . | . | . | . | . | . | . | . | . | . | . | . | . | . | . | . | . | G | . | A | . | . | . | . |
| 21-225_7F10_LC | . | . | . | . | S | V | . | . | . | . | . | . | . | . | . | . | . | . | . | . | . | . | . | . | S | . |
| 21-225_80C11_LC | . | . | . | . | . | T | . | . | . | . | . | . | . | . | . | . | . | . | . | . | . | A | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_147E9_LC | . | . | . | . | . | . | . | . | . | . | . | . | . | . | . | . | . | . | . | . | . | . | . | . | . | . |
| 21-225_150G8_LC | . | . | . | . | . | . | . | . | . | . | . | . | . | . | . | . | . | . | . | . | . | . | . | . | . | . |
| 21-225_153F9_LC | . | . | . | . | . | . | . | . | . | . | . | . | . | . | . | . | . | . | . | . | . | . | . | . | . | . |
| 21-225_157C1_LC | . | . | . | . | . | . | . | . | . | . | . | . | . | . | . | . | . | . | . | . | . | . | . | . | . | . |
| 21-225_160H4_LC | . | . | . | . | . | . | . | . | . | . | . | . | . | . | . | . | . | . | . | . | . | . | . | . | . | . |
| 21-225_163G6_LC | . | . | . | . | . | . | . | . | . | . | . | . | . | . | . | . | . | . | . | . | . | . | . | . | . | . |
| 21-225_166H12_LC | . | . | . | . | . | . | . | . | . | . | . | . | . | . | . | . | . | . | . | . | . | . | . | . | . | . |
| 21-225_169C10_LC | . | . | . | . | . | . | . | . | . | . | N | . | . | . | . | . | . | . | . | . | . | . | . | . | . | . |
| 21-225_16A12_LC | . | . | . | . | . | . | . | . | . | ⸫ | . | . | . | . | . | . | . | . | . | . | . | . | . | . | . | . |
| 21-225_16D10_LC | . | . | . | . | . | . | . | . | . | ⸫ | . | . | . | . | . | . | . | . | . | . | . | . | . | . | . | . |
| 21-225_170D1_LC | . | . | . | . | . | . | . | . | . | . | . | . | . | . | . | . | . | . | . | . | . | . | . | . | . | . |
| 21-225_170D6_LC | . | . | . | . | . | . | . | . | . | . | . | . | . | . | . | . | . | . | . | . | . | . | . | . | . | . |
| 21-225_171H12_LC | . | . | . | . | . | . | . | . | . | . | . | . | . | . | . | . | . | . | . | . | . | . | . | . | . | . |
| 21-225_175G3_LC | . | . | . | . | . | . | . | . | . | . | . | . | . | . | . | . | . | . | . | . | . | . | . | . | . | . |
| 21-225_177A5_LC | . | . | . | . | . | . | . | . | . | . | . | . | . | . | . | . | . | . | . | . | . | . | . | . | . | . |
| 21-225_178H4_LC | . | . | . | . | . | . | . | . | . | . | . | . | . | . | . | N | . | . | . | . | . | . | . | . | . | . |
| 21-225_190C11_LC | . | . | . | . | . | . | . | . | . | . | . | . | . | . | . | . | . | . | . | . | . | . | . | . | . | . |
| 21-225_190E10_LC | . | . | . | . | . | . | . | . | . | . | . | . | . | . | . | . | . | . | . | . | . | . | . | . | . | . |
| 21-225_190E6_LC | . | . | . | . | . | . | . | . | . | . | . | . | . | . | . | . | . | . | . | . | . | . | . | . | . | . |
| 21-225_190F8_LC | . | . | . | . | . | . | . | . | . | . | . | . | . | . | . | . | . | . | . | . | . | . | . | . | . | . |
| 21-225_190G11_LC | . | . | . | . | . | . | . | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . | . | . | N | . | . | . | . |
| 21-225_191A10_LC | . | . | . | . | Y | . | . | . | . | . | . | . | . | . | . | . | . | . | . | . | . | N | . | ⸪ | . | . |
| 21-225_191E10_LC | . | . | . | . | . | . | . | . | . | . | . | . | . | . | . | . | . | . | . | . | . | N | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | . | . | . | . | . | . | . | . | . | . | . | . | . | . | . | . | . | . | . | . | . | . | . |
| 21-225_191G12_LC | . | . | . | . | . | . | . | . | . | . | . | . | . | . | . | . | . | . | . | . | . | . | . | . | . | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | . | . | . | . | . | . | . | . | . | . | . | . | . | . | . | . | . | . | . | . | . | . |

4639

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_192D3_LC | . | . | . | . | . | . | . | . | . | . | . | . | . | . | . | . | . | . | . | . | . | . | . | . | . | . |
| 21-225_192G6_LC | . | . | . | . | . | . | . | . | . | . | . | . | . | . | . | . | . | . | . | . | . | . | . | . | . | . |
| 21-225_192G8_LC | . | . | . | . | . | . | . | . | . | . | . | . | . | . | . | . | . | . | . | . | . | . | . | . | . | . |
| 21-225_192H10_LC | . | . | . | . | Y | . | . | . | . | . | . | . | . | . | . | . | . | . | . | . | N | . | . | . | . | . |
| 21-225_192H4_LC | . | . | . | R | . | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . | . | . | . | . | . | . | . |
| 21-225_197B6_LC | . | . | . | . | . | . | . | . | . | . | . | . | . | . | . | . | . | . | . | . | N | . | . | . | . | . |
| 21-225_197D3_LC | . | . | . | . | . | . | . | . | . | . | . | . | . | . | . | . | . | . | . | . | . | . | . | . | . | . |
| 21-225_197F2_LC | . | . | . | . | . | . | . | . | . | . | . | . | . | . | . | . | . | . | . | . | . | . | . | . | . | . |
| 21-225_197G12_LC | . | . | . | . | . | . | . | . | . | . | . | . | . | . | . | . | . | . | . | . | . | . | . | . | . | . |
| 21-225_198B6_LC | . | . | N | R | . | . | . | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . | . | . | . | . | A | . | . |
| 21-225_1F1_LC | . | . | . | . | . | . | . | . | . | . | . | . | . | . | . | . | . | . | . | . | . | . | . | . | . | . |
| 21-225_203H1_LC | . | . | T | . | . | . | . | . | S | . | . | . | . | . | . | . | . | . | . | D | . | . | . | . | . | . |
| 21-225_204G6_LC | . | . | . | . | . | . | . | . | . | . | . | . | . | . | . | . | . | . | . | . | . | . | . | . | . | . |
| 21-225_204G8_LC | . | . | . | . | . | . | . | . | E | . | . | . | . | . | . | . | . | . | . | . | . | . | . | . | . | . |
| 21-225_205F5_LC | . | . | . | . | . | . | . | . | . | . | . | . | . | . | . | . | . | . | . | . | . | . | . | . | . | . |
| 21-225_205H3_LC | . | . | . | . | . | . | . | . | S | . | . | . | . | . | . | . | . | . | . | . | . | . | . | . | . | . |
| 21-225_207G6_LC | . | . | N | R | . | . | . | . | . | . | . | . | . | . | . | . | . | . | . | . | . | . | . | . | . | . |
| 21-225_211A11_LC | . | . | . | R | . | . | . | . | . | . | . | . | . | . | . | G | . | . | . | . | . | . | . | . | . | . |
| 21-225_211A3_LC | . | . | . | . | . | . | . | . | . | . | . | . | . | . | . | . | . | . | . | . | . | . | L | . | . | . |
| 21-225_211A8_LC | . | . | . | . | . | . | . | . | . | . | . | . | . | . | . | . | . | . | . | . | . | . | . | . | . | . |
| 21-225_212D7_LC | . | . | N | R | . | . | . | . | . | . | . | . | . | . | N | N | . | . | . | . | . | . | . | V | . | . |
| 21-225_212E10_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
|  |  |  |  |  |  |  |  |  |  | K_FR3 |  |  |  |  |  |  |  |  |  |  |  |  |  |  |  |  |
| CONSENSUS | S | G | S | G | S | G | - | - | T | D | F | T | L | T | I | S | S | L | Q | P | E | D | F | A | T | Y |
| 21-225_212E6_LC | . | . | . | R | . | . | . | . | . | . | . | . | . | . | . | . | . | . | . | . | . | . | . | . | . | . |
| 21-225_212F10_LC | . | . | . | R | . | . | . | . | . | . | . | . | . | . | . | . | . | . | . | . | . | . | . | . | . | . |
| 21-225_212H11_LC | . | . | . | . | . | . | . | . | . | . | . | . | . | . | . | . | . | . | . | . | . | . | . | L | . | . |
| 21-225_212H9_LC | . | . | . | . | . | . | . | . | . | . | . | . | . | . | . | . | . | . | . | . | . | . | . | . | . | . |
| 21-225_213B8_LC | . | . | . | . | . | . | . | . | . | . | . | . | . | . | . | . | . | . | . | . | . | . | . | . | . | . |
| 21-225_213C4_LC | . | . | . | . | . | . | . | . | . | . | . | . | . | . | . | . | . | . | . | . | . | . | . | . | . | . |
| 21-225_213D2_LC | . | N | . | R | . | . | . | . | . | . | . | . | . | . | . | N | N | . | . | . | . | . | . | V | . | . |
| 21-225_213E5_LC | . | . | . | . | . | . | . | . | . | . | . | . | . | . | . | . | . | . | . | . | . | . | . | L | . | . |
| 21-225_215A12_LC | . | . | . | R | . | . | . | . | . | . | . | . | . | . | . | . | . | . | . | S | . | . | . | . | . | . |
| 21-225_215B5_LC | . | . | N | R | . | . | . | . | . | . | . | . | . | . | . | N | N | . | . | . | . | . | . | V | . | . |
| 21-225_215D6_LC | . | . | . | R | . | . | . | . | . | . | . | . | . | . | . | . | . | . | . | . | . | . | . | . | . | . |
| 21-225_216G1_LC | . | . | . | . | . | . | . | . | . | . | . | . | . | . | . | . | . | . | . | . | . | . | . | . | . | . |
| 21-225_217B10_LC | . | . | . | . | . | . | . | . | . | . | . | . | . | . | . | . | . | V | . | . | . | . | . | L | . | . |
| 21-225_217G5_LC | . | . | T | R | . | . | . | . | . | . | . | . | . | . | . | . | . | . | . | D | . | . | . | . | . | . |
| 21-225_219H1_LC | . | . | . | R | . | . | . | . | . | . | . | . | . | . | . | . | . | . | . | . | . | . | . | . | . | . |
| 21-225_221F6_LC | . | . | . | R | . | . | . | . | . | . | . | . | . | . | . | . | . | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | . | . | . | . | . | . | . | . | . | . |
| 21-225_22A2_LC | . | . | . | . | . | . | . | . | . | . | . | . | . | . | . | . | . | . | . | . | . | . | . | . | S | . |
| 21-225_23D11_LC | . | . | . | . | . | . | . | . | . | E | . | . | . | . | . | . | . | . | . | . | . | . | . | . | . | . |
| 21-225_32G3_LC | . | . | . | . | . | . | . | . | . | . | . | . | . | . | . | T | . | . | . | . | . | . | . | . | . | . |
| 21-225_55A11_LC | . | . | . | . | . | . | . | . | . | . | . | . | . | . | . | . | . | . | . | . | . | . | . | . | . | . |
| 21-225_55D4_LC | . | . | . | . | P | . | . | . | . | . | . | . | . | . | . | N | . | . | . | . | . | . | . | . | . | . |
| 21-225_65H11_LC | . | . | . | . | . | . | . | . | . | . | . | . | . | . | . | . | . | . | . | . | . | . | . | . | . | . |
| 21-225_70H6_LC | . | . | . | . | . | . | . | . | . | . | . | . | . | . | . | . | . | . | . | . | . | . | S | . | . | . |
| 21-225_74C8_LC | . | . | . | . | . | . | . | . | . | . | . | . | . | . | . | . | . | . | . | . | Y | . | . | . | . | . |
| 21-225_74G6_LC | . | . | . | . | . | . | . | . | . | . | . | . | . | . | . | . | . | . | . | . | Y | . | . | . | . | . |
| 21-225_76C5_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | K_FR3 | | | | | | | | | | | | | | | | |
| CONSENSUS | S | G | S | G | S | G | - | - | T | D | F | T | L | T | I | S | S | L | Q | P | E | D | F | A | T | Y |
| 21-225_77E6_LC | | | | | | | | | | | | | | | | | | | | | | | | | | |
| 21-225_79A12_LC | | | | | | | | | | | | | | | | | | | | | | | | | | |
| 21-225_79F4_LC | | | | | | | | | | | | | | | | | | | | | | | | | | |
| 21-225_7F10_LC | | | | | | | | | | | | | | | | | | | | | | | | | | |
| 21-225_80C11_LC | | | | | | | | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | S | T | - | - | - | - | - | - | - | - | - | - | - | - | - | - | - | - | - | - | - |
| 21-225_147E9_LC | . | . | . | . | . | N | S | . | . | . | . | . | . | . | . | . | . | . | . | . | . | . | . | . | . | . |
| 21-225_150G8_LC | . | . | . | . | . | N | N | . | . | . | . | . | . | . | . | . | . | . | . | . | . | . | . | . | . | . |
| 21-225_153F9_LC | . | . | . | . | | N | S | . | . | . | . | . | . | . | . | . | . | . | . | . | . | . | . | . | . | . |
| 21-225_157C1_LC | . | . | . | . | . | N | S | . | . | . | . | . | . | . | . | . | . | . | . | . | . | . | . | . | . | . |
| 21-225_160H4_LC | . | . | . | . | | N | S | . | . | . | . | . | . | . | . | . | . | . | . | . | . | . | . | . | . | . |
| 21-225_163G6_LC | . | . | . | . | . | . | S | . | . | . | . | . | . | . | . | . | . | . | . | . | . | . | . | . | . | . |
| 21-225_166H12_LC | . | . | . | . | | N | S | . | . | . | . | . | . | . | . | . | . | . | . | . | . | . | . | . | . | . |
| 21-225_169C10_LC | . | . | . | . | S | D | S | . | . | . | . | . | . | . | . | . | . | . | . | . | . | . | . | . | . | . |
| 21-225_16A12_LC | . | . | . | . | . | Y | S | . | . | . | . | . | . | . | . | . | . | . | . | . | . | . | . | . | . | . |
| 21-225_16D10_LC | . | . | . | . | . | N | S | . | . | . | . | . | . | . | . | . | . | . | . | . | . | . | . | . | . | . |
| 21-225_170D1_LC | . | . | . | . | C | D | S | . | . | . | . | . | . | . | . | . | . | . | . | . | . | . | . | . | . | . |
| 21-225_170D6_LC | . | . | . | . | S | D | S | . | . | . | . | . | . | . | . | . | . | . | . | . | . | . | . | . | . | . |
| 21-225_171H12_LC | . | . | . | . | S | D | S | . | . | . | . | . | . | . | . | . | . | . | . | . | . | . | . | . | . | . |
| 21-225_175G3_LC | . | . | . | . | S | D | S | . | . | . | . | . | . | . | . | . | . | . | . | . | . | . | . | . | . | . |
| 21-225_177A5_LC | . | . | . | . | S | D | S | . | . | . | . | . | . | . | . | . | . | . | . | . | . | . | . | . | . | . |
| 21-225_178H4_LC | . | . | . | . | S | N | S | . | . | . | . | . | . | . | . | . | . | . | . | . | . | . | . | . | . | . |
| 21-225_180C11_LC | . | . | . | . | | M | . | . | . | . | . | . | . | . | . | . | . | . | . | . | . | . | . | . | . | . |
| 21-225_190E10_LC | . | . | . | . | | M | . | . | . | . | . | . | . | . | . | . | . | . | . | . | . | . | . | . | . | . |
| 21-225_190E6_LC | . | . | . | . | | M | . | . | . | . | . | . | . | . | . | . | . | . | . | . | . | . | . | . | . | . |
| 21-225_190F8_LC | . | . | . | . | | M | . | . | . | . | . | . | . | . | . | . | . | . | . | . | . | . | . | . | . | . |
| 21-225_190G11_LC | . | . | . | . | | M | . | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . | . | . | . | . | . | . | . |
| 21-225_191G1_LC | . | . | . | H | . | L | . | . | . | . | . | . | . | . | . | . | . | . | . | . | . | . | . | . | . | . |
| 21-225_191G10_LC | . | . | . | . | | M | . | . | . | . | . | . | . | . | . | . | . | . | . | . | . | . | . | . | . | . |
| 21-225_191G12_LC | . | . | . | . | | I | . | . | . | . | . | . | . | . | . | . | . | . | . | . | . | . | . | . | . | . |
| 21-225_192C8_LC | . | . | . | H | . | L | . | . | . | . | . | . | . | . | . | . | . | . | . | . | . | . | . | . | . | . |
| 21-225_192D2_LC | . | . | . | H | . | V | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | S | T | - | - | - | - | - | - | - | - | - | - | - | - | - | - | - | - | - | - | - |
| 21-225_192D3_LC | . | . | L | H | . | L | . | . | . | . | . | . | . | . | . | . | . | . | . | . | . | . | . | . | . | . |
| 21-225_192G6_LC | . | . | . | . | . | W | . | . | . | . | . | . | . | . | . | . | . | . | . | . | . | . | . | . | . | . |
| 21-225_192G8_LC | . | . | . | . | . | I | . | . | . | . | . | . | . | . | . | . | . | . | . | . | . | . | . | . | . | . |
| 21-225_192H10_LC | . | . | . | . | . | . | . | . | . | . | . | . | . | . | . | . | . | . | . | . | . | . | . | . | . | . |
| 21-225_192H4_LC | . | G | L | H | . | L | N | . | . | . | . | . | . | . | . | . | . | . | . | . | . | . | . | . | . | . |
| 21-225_193A10_LC | F | . | . | H | . | L | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | L | H | . | L | . | . | . | . | . | . | . | . | . | . | . | . | . | . | . | . | . | . | . | . |
| 21-225_194H11_LC | . | . | . | H | . | L | . | . | . | . | . | . | . | . | . | . | . | . | . | . | . | . | . | . | . | . |
| 21-225_195A9_LC | . | . | . | H | . | L | . | . | . | . | . | . | . | . | . | . | . | . | . | . | . | . | . | . | . | . |
| 21-225_196C7_LC | . | . | . | H | . | L | . | . | . | . | . | . | . | . | . | . | . | . | . | . | . | . | . | . | . | . |
| 21-225_197B6_LC | . | . | . | . | . | . | . | . | . | . | . | . | . | . | . | . | . | . | . | . | . | . | . | . | . | . |
| 21-225_197D3_LC | . | . | . | H | . | L | . | . | . | . | . | . | . | . | . | . | . | . | . | . | . | . | . | . | . | . |
| 21-225_197F2_LC | . | F | . | . | . | I | . | . | . | . | . | . | . | . | . | . | . | . | . | . | . | . | . | . | . | . |
| 21-225_197G12_LC | . | F | . | . | . | W | . | . | . | . | . | . | . | . | . | . | . | . | . | . | . | . | . | . | . | . |
| 21-225_198B6_LC | . | . | . | . | . | . | . | . | . | . | . | . | . | . | . | . | . | . | . | . | . | . | . | . | . | . |
| 21-225_199C5_LC | . | . | . | . | . | L | . | . | . | . | . | . | . | . | . | . | . | . | . | . | . | . | . | . | . | . |
| 21-225_1F1_LC | . | . | . | . | . | N | S | . | . | . | . | . | . | . | . | . | . | . | . | . | . | . | . | . | . | . |
| 21-225_203H1_LC | . | . | . | H | . | H | . | . | . | . | . | . | . | . | . | . | . | . | . | . | . | . | . | . | . | . |
| 21-225_204G6_LC | . | . | . | H | . | L | S | . | . | . | . | . | . | . | . | . | . | . | . | . | . | . | . | . | . | . |
| 21-225_204G8_LC | . | . | . | . | . | N | . | . | . | . | . | . | . | . | . | . | . | . | . | . | . | . | . | . | . | . |
| 21-225_205F5_LC | T | . | . | . | . | N | . | . | . | . | . | . | . | . | . | . | . | . | . | . | . | . | . | . | . | . |
| 21-225_205H3_LC | . | . | . | . | . | N | . | . | . | . | . | . | . | . | . | . | . | . | . | . | . | . | . | . | . | . |
| 21-225_207G6_LC | . | . | . | . | . | N | . | . | . | . | . | . | . | . | . | . | . | . | . | . | . | . | . | . | . | . |
| 21-225_211A11_LC | . | . | . | H | . | W | . | . | . | . | . | . | . | . | . | . | . | . | . | . | . | . | . | . | . | . |
| 21-225_211A3_LC | . | . | . | . | . | . | N | . | . | . | . | . | . | . | . | . | . | . | . | . | . | . | . | . | . | . |
| 21-225_211A8_LC | . | . | L | R | . | D | . | . | . | . | . | . | . | . | . | . | . | . | . | . | . | . | . | . | . | . |
| 21-225_212D7_LC | . | . | . | . | . | . | N | . | . | . | . | . | . | . | . | . | . | . | . | . | . | . | . | . | . | . |
| 21-225_212E10_LC | . | . | . | . | . | . | N | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | S | T | - | - | - | - | - | - | - | - | - | - | - | - | - | - | - | - | - | - | - |
| 21-225_212E6_LC | . | . | . | . | . | V | . | . | . | . | . | . | . | . | . | . | . | . | . | . | . | . | . | . | . | . |
| 21-225_212F10_LC | . | . | . | R | . | . | N | . | . | . | . | . | . | . | . | . | . | . | . | . | . | . | . | . | . | . |
| 21-225_212H11_LC | . | . | . | R | . | . | N | . | . | . | . | . | . | . | . | . | . | . | . | . | . | . | . | . | . | . |
| 21-225_212H9_LC | . | . | L | R | . | D | . | . | . | . | . | . | . | . | . | . | . | . | . | . | . | . | . | . | . | . |
| 21-225_213B8_LC | . | . | . | . | . | . | N | . | . | . | . | . | . | . | . | . | . | . | . | . | . | . | . | . | . | . |
| 21-225_213C4_LC | . | . | . | . | . | . | N | . | . | . | . | . | . | . | . | . | . | . | . | . | . | . | . | . | . | . |
| 21-225_213D2_LC | . | . | R | . | . | . | N | . | . | . | . | . | . | . | . | . | . | . | . | . | . | . | . | . | . | . |
| 21-225_213E5_LC | . | . | . | R | . | . | N | . | . | . | . | . | . | . | . | . | . | . | . | . | . | . | . | . | . | . |
| 21-225_215A12_LC | . | . | . | . | . | V | . | . | . | . | . | . | . | . | . | . | . | . | . | . | . | . | . | . | . | . |
| 21-225_215B5_LC | . | . | . | . | . | I | N | . | . | . | . | . | . | . | . | . | . | . | . | . | . | . | . | . | . | . |
| 21-225_215D6_LC | . | . | . | . | . | V | . | . | . | . | . | . | . | . | . | . | . | . | . | . | . | . | . | . | . | . |
| 21-225_216G1_LC | . | . | L | R | . | D | . | . | . | . | . | . | . | . | . | . | . | . | . | . | . | . | . | . | . | . |
| 21-225_217B10_LC | . | R | L | R | T | R | . | . | . | . | . | . | . | . | . | . | . | . | . | . | . | . | . | . | . | . |
| 21-225_217G5_LC | . | . | . | . | . | V | N | . | . | . | . | . | . | . | . | . | . | . | . | . | . | . | . | . | . | . |
| 21-225_219H1_LC | . | . | . | R | . | . | N | . | . | . | . | . | . | . | . | . | . | . | . | . | . | . | . | . | . | . |
| 21-225_221F6_LC | . | . | . | . | . | M | . | . | . | . | . | . | . | . | . | . | . | . | . | . | . | . | . | . | . | . |
| 21-225_222A11_LC | . | . | L | Y | . | L | N | . | . | . | . | . | . | . | . | . | . | . | . | . | . | . | . | . | . | . |
| 21-225_222D10_LC | . | . | L | R | . | L | N | . | . | . | . | . | . | . | . | . | . | . | . | . | . | . | . | . | . | . |
| 21-225_223H10_LC | . | . | L | R | . | L | N | . | . | . | . | . | . | . | . | . | . | . | . | . | . | . | . | . | . | . |
| 21-225_22A2_LC | . | . | . | . | . | N | S | . | . | . | . | . | . | . | . | . | . | . | . | . | . | . | . | . | . | . |
| 21-225_23D11_LC | . | . | . | . | . | R | S | . | . | . | . | . | . | . | . | . | . | . | . | . | . | . | . | . | . | . |
| 21-225_32G3_LC | . | . | . | . | . | R | S | . | . | . | . | . | . | . | . | . | . | . | . | . | . | . | . | . | . | . |
| 21-225_55A11_LC | . | . | . | . | . | N | S | . | . | . | . | . | . | . | . | . | . | . | . | . | . | . | . | . | . | . |
| 21-225_55D4_LC | . | . | . | . | . | R | S | . | . | . | . | . | . | . | . | . | . | . | . | . | . | . | . | . | . | . |
| 21-225_65H11_LC | . | . | . | . | . | N | S | . | . | . | . | . | . | . | . | . | . | . | . | . | . | . | . | . | . | . |
| 21-225_70H6_LC | . | . | . | . | S | Y | S | . | . | . | . | . | . | . | . | . | . | . | . | . | . | . | . | . | . | . |
| 21-225_74C8_LC | . | . | . | . | . | . | N | . | . | . | . | . | . | . | . | . | . | . | . | . | . | . | . | . | . | . |
| 21-225_74G6_LC | . | . | . | . | . | . | N | . | . | . | . | . | . | . | . | . | . | . | . | . | . | . | . | . | . | . |
| 21-225_76C5_LC | . | . | . | . | . | . | N | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | S | T | - | - | - | - | - | - | - | - | - | - | - | - | - | - | - | - | - | - | - |
| 21-225_77E6_LC | . | . | . | . | . | . | N | . | . | . | . | . | . | . | . | . | . | . | . | . | . | . | . | . | . | . |
| 21-225_79A12_LC | . | . | . | . | . | . | N | . | . | . | . | . | . | . | . | . | . | . | . | . | . | . | . | . | . | . |
| 21-225_79F4_LC | . | . | . | . | . | . | N | . | . | . | . | . | . | . | . | . | . | . | . | . | . | . | . | . | . | . |
| 21-225_7F10_LC | . | . | . | . | . | N | S | . | . | . | . | . | . | . | . | . | . | . | . | . | . | . | . | . | . | . |
| 21-225_80C11_LC | . | . | . | . | . | . | N | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | L | T | F | G | G | G | T | K | V | E | I | K | R |
| 21-225_147E9_LC | . | . | | | . | . | . | . | | | | | . | . | | . | . | . | . |
| 21-225_150G8_LC | . | . | . | . | . | . | . | . | . | . | . | . | . | . | . | . | . | . | . |
| 21-225_153F9_LC | . | . | | | . | . | . | S | | | | | . | . | | . | . | . | . |
| 21-225_157C1_LC | . | . | | | S | . | S | . | | | | | . | . | | . | L | . | . |
| 21-225_160H4_LC | . | . | | | . | . | . | . | | | | | . | . | | . | . | . | . |
| 21-225_163G6_LC | . | . | | | . | . | . | . | | | | | . | . | | A | . | . | . |
| 21-225_166H12_LC | . | . | | | S | . | . | . | | | | | . | . | | . | . | T | . |
| 21-225_169C10_LC | . | . | | | . | . | . | . | | | | | . | . | | . | . | . | . |
| 21-225_16A12_LC | . | . | | | . | . | . | . | | | | | . | . | | . | . | . | . |
| 21-225_16D10_LC | . | . | | | . | . | . | . | | | | | . | . | | . | . | . | . |
| 21-225_170D1_LC | . | . | | | S | . | . | . | | | | | . | . | | . | . | . | . |
| 21-225_170D6_LC | . | . | | | . | . | . | . | | | | | . | . | | . | . | . | . |
| 21-225_171H12_LC | . | . | | | . | . | . | . | | | | | . | . | | . | . | . | . |
| 21-225_175G3_LC | . | . | | | . | . | . | . | | | | | . | . | | . | . | . | . |
| 21-225_177A5_LC | . | . | | | . | . | . | . | | | | | . | . | | . | . | . | . |
| 21-225_178H4_LC | . | . | | | . | . | . | . | | | | | . | . | | . | . | . | . |
| 21-225_190C11_LC | . | . | | | . | . | . | . | | | | | . | . | | . | . | . | . |
| 21-225_190E10_LC | . | . | | | . | . | . | . | | | | | . | . | | . | . | . | . |
| 21-225_190E6_LC | . | . | | | . | . | . | . | | | | | . | . | | . | . | . | . |
| 21-225_190F8_LC | . | . | | | . | . | . | . | | | | | . | . | | . | . | . | . |
| 21-225_190G11_LC | . | . | | | . | . | . | . | | | | | . | . | | . | . | . | . |
| 21-225_190G4_LC | . | . | | | . | . | . | . | | | | | . | . | | . | . | . | . |
| 21-225_191A10_LC | . | . | | | . | . | . | . | | | | | . | . | | . | . | . | . |
| 21-225_191E10_LC | . | . | | | . | . | . | . | | | | | . | . | | . | . | . | . |
| 21-225_191G1_LC | . | . | | | . | . | . | . | | | | | . | . | | . | . | . | . |
| 21-225_191G10_LC | . | . | | | . | . | . | . | | | | | . | . | | . | . | . | . |
| 21-225_191G12_LC | . | . | | | . | . | . | . | | | | . | S | . | | . | . | . | . |
| 21-225_192C8_LC | . | . | | | . | . | . | . | | | | | . | . | | . | . | . | . |
| 21-225_192D2_LC | . | . | | | . | . | N | . | | | | | . | . | | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K FR4 | | | | | | |
| CONSENSUS | - | - | - | - | Y | P | L | T | F | G | G | G | T | K | V | E | I | K | R |
| 21-225_192D3_LC | . | . | . | . | . | . | . | . | . | . | . | . | . | . | . | . | . | . | . |
| 21-225_192G6_LC | . | . | . | . | . | . | . | . | . | . | . | . | . | . | . | . | . | . | . |
| 21-225_192G8_LC | . | . | . | . | . | . | . | . | . | . | . | . | . | . | . | . | . | . | . |
| 21-225_192H10_LC | . | . | . | . | . | . | . | . | . | . | . | . | . | . | . | . | . | . | . |
| 21-225_192H4_LC | . | . | . | . | . | . | . | . | . | . | . | . | . | ☒ | . | . | . | ☒ | . |
| 21-225_193A10_LC | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . |
| 21-225_197B6_LC | . | . | . | . | . | . | . | . | . | . | . | . | . | . | . | . | . | . | . |
| 21-225_197D3_LC | . | . | . | . | . | . | . | . | . | . | . | . | . | . | . | . | . | . | . |
| 21-225_197F2_LC | . | . | . | . | . | . | . | . | . | . | . | . | . | . | . | . | . | . | . |
| 21-225_197G12_LC | . | . | . | . | . | . | . | . | . | . | . | . | . | . | . | . | . | . | . |
| 21-225_198B6_LC | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . |
| 21-225_1F1_LC | . | . | . | . | . | . | . | . | . | . | . | . | . | . | . | . | . | . | . |
| 21-225_203H1_LC | . | . | . | . | . | . | . | . | . | . | . | . | . | . | . | . | . | . | . |
| 21-225_204G6_LC | . | . | . | . | . | . | . | . | . | . | . | . | . | . | . | . | . | . | . |
| 21-225_204G8_LC | . | . | . | . | . | V | . | . | . | . | . | . | . | . | . | . | . | . | . |
| 21-225_205F5_LC | . | . | . | . | . | . | . | . | . | . | . | . | . | . | . | D | . | ☒ | . |
| 21-225_205H3_LC | . | . | . | . | . | . | . | . | . | . | . | . | . | . | . | . | . | . | . |
| 21-225_207G6_LC | . | . | . | . | . | . | . | . | . | . | . | . | . | . | . | . | . | . | . |
| 21-225_211A11_LC | . | . | . | . | . | . | . | . | . | A | . | . | . | . | . | . | . | . | . |
| 21-225_211A3_LC | . | . | . | . | . | . | . | . | . | . | . | . | . | . | . | . | . | . | . |
| 21-225_211A8_LC | . | . | . | . | . | . | . | I | . | . | . | . | . | . | . | . | . | . | . |
| 21-225_212D7_LC | . | . | . | . | . | . | . | . | . | . | . | . | . | . | . | . | . | . | . |
| 21-225_212E10_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | L | T | F | G | G | G | T | K | V | E | I | K | R |
| 21-225_212E6_LC | . | . | . | . | . | . | . | . | . | . | . | . | . | . | . | . | . | . | . |
| 21-225_212F10_LC | . | . | . | . | . | . | . | . | . | . | . | . | . | . | . | . | . | . | . |
| 21-225_212H11_LC | . | . | . | . | . | . | . | . | V | . | . | . | . | . | . | . | . | T | G |
| 21-225_212H9_LC | . | . | . | . | . | . | . | . | . | . | . | . | . | . | . | . | . | . | . |
| 21-225_213B8_LC | . | . | . | . | . | . | . | . | . | . | . | . | . | . | . | . | . | . | . |
| 21-225_213C4_LC | . | . | . | . | . | . | . | . | . | . | . | . | . | . | . | . | . | . | . |
| 21-225_213D2_LC | . | . | . | . | . | . | . | . | . | . | . | . | . | . | . | . | . | . | . |
| 21-225_213E5_LC | . | . | . | . | . | . | . | . | . | . | . | . | . | . | . | . | . | . | . |
| 21-225_215A12_LC | . | . | . | . | . | . | . | . | . | . | . | . | . | . | . | . | . | . | . |
| 21-225_215B5_LC | . | . | . | . | . | . | . | . | . | . | . | . | . | . | . | . | . | . | . |
| 21-225_215D6_LC | . | . | . | . | . | . | . | . | . | . | . | . | . | . | . | . | . | . | . |
| 21-225_216G1_LC | . | . | . | . | . | . | . | . | . | . | . | . | . | . | . | . | . | . | . |
| 21-225_217B10_LC | . | . | . | . | S | . | V | Q | L | V | . | . | . | . | . | . | . | T | G |
| 21-225_217G5_LC | . | . | . | . | . | . | . | . | . | . | . | . | . | . | . | . | . | N | . |
| 21-225_219H1_LC | . | . | . | . | . | . | . | . | . | . | . | . | . | . | . | . | . | . | . |
| 21-225_221F6_LC | . | . | . | . | . | . | . | . | . | . | . | . | . | X | . | . | . | . | . |
| 21-225_222A11_LC | . | . | . | . | . | . | . | . | . | . | . | . | . | . | . | . | . | . | . |
| 21-225_222D10_LC | . | . | . | . | . | . | . | . | . | . | . | . | . | X | . | . | . | . | . |
| 21-225_223H10_LC | . | . | . | . | . | . | . | . | . | . | . | . | . | . | . | . | . | . | . |
| 21-225_22A2_LC | . | . | . | . | F | . | . | . | . | . | . | . | . | . | . | . | . | . | . |
| 21-225_23D11_LC | . | . | . | . | . | . | . | . | . | . | . | . | S | . | . | . | . | . | . |
| 21-225_32G3_LC | . | . | . | . | . | . | . | . | . | . | . | . | . | . | . | . | . | . | . |
| 21-225_55A11_LC | . | . | . | . | . | . | . | . | . | . | . | . | . | . | . | . | . | . | . |
| 21-225_55D4_LC | . | . | . | . | F | . | . | . | . | . | . | . | . | X | . | . | . | X | . |
| 21-225_65H11_LC | . | . | . | . | F | . | . | . | . | . | . | . | . | . | . | . | . | . | . |
| 21-225_70H6_LC | . | . | . | . | I | . | . | . | . | . | . | . | . | . | . | . | . | . | . |
| 21-225_74C8_LC | . | . | . | . | . | . | . | . | . | . | . | . | . | . | . | . | . | N | . |
| 21-225_74G6_LC | . | . | . | . | . | . | . | . | . | . | . | . | . | . | . | . | . | N | . |
| 21-225_76C5_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_77E6_LC | . | . | . | . | . | . | . | . | . | . | . | . | . | ≈ | . | . | . | . | . |
| 21-225_79A12_LC | . | . | . | . | . | . | . | . | . | . | . | . | . | . | . | . | . | . | . |
| 21-225_79F4_LC | . | . | . | . | . | . | . | . | . | . | . | . | . | ≈ | . | . | . | . | . |
| 21-225_7F10_LC | . | . | . | . | . | . | . | . | . | . | . | . | . | . | . | . | . | . | . |
| 21-225_80C11_LC | . | . | . | . | . | . | . | . | . | . | . | . | . | . | . | . | . | N | . |

**FIGURE 57**

Table 102.    Consensus 22 - VK1|O12/JK4 (SEQ ID NO: 50319):

DIQMTQSPSSLSASVGDRVTITCRAS--QNII------SYLNWYQQKPGKAPKLLIYT-------ASSLQSGVPSRFSGSGSG--TDFTLTISSLQPEDFATYYCQQSYS---------------------TPLTFGGGTKVEIKR wherein:

A at position 25 can be substituted with T

S at position 26 can be substituted with T

Q at position 29 can be substituted with H or R

N at position 30 can be substituted with S, R, T or I

I at position 31 can be substituted with V or F

I at position 32 can be substituted with S, N, Y, F, R, H, L, K or T

S at position 39 can be substituted with N, D, R, K or T

Y at position 40 can be substituted with F

N at position 42 an be substituted with H

T at position 58 can be substituted with A, V, G, I or S

A at position 67 can be substituted with T

S at position 69 can be substituted with N, R or T

L at position 70 can be substituted with F or S

Q at position 71 can be substituted with H, E or P

S at position 72 can be substituted with G, T, N or R

Q at position 108 can be substituted with L

S at position 109 can be substituted with T, N or P

<div align="center">FIGURE 57</div>

Y at position 110 can be substituted with H, C, D, F or N

S at position 111 can be substituted with null (-), I, N, F, G or T

T at position 135 can be substituted with S, P, F, N, I or L

P at position 136 can be substituted with T, I, A or S

L at position 137 can be substituted with P, Y, F or V

Arg Xaa Xaa Xaa Xaa Xaa Xaa Xaa Leu Xaa (SEQ ID NO: 50141)

Xaa Xaa Ser Xaa Xaa Xaa Xaa (SEQ ID NO: 50142)

Gln Xaa Xaa Xaa Xaa Xaa Xaa Xaa Thr (SEQ ID NO: 50143)

Arg Ala Ser Gln Asn Ile Ile Ser Tyr Leu Asn (SEQ ID NO: 50627)

Thr Ala Ser Ser Leu Gln Ser (SEQ ID NO: 50628)

Gln Gln Ser Tyr Ser Thr Pro Leu Thr (SEQ ID NO: 50629)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_10E12_LC | | . | . | . | . | . | . | . | . | . | . | . | . | . | . | . | . | . | . | . | . | . | . | . | . | . |
| 21-225_10F5_LC | | . | . | . | . | . | . | . | . | . | . | . | . | . | . | . | . | . | . | . | . | . | . | . | . | . |
| 21-225_12A10_LC | . | V | . | . | . | . | . | . | . | . | . | . | . | A | . | . | . | . | . | . | . | . | . | . | . | . |
| 21-225_13D4_LC | . | L | . | . | . | . | . | . | . | . | . | . | . | . | . | . | . | . | . | . | . | . | . | . | . | . |
| 21-225_146E9_LC | | . | . | . | . | . | . | . | . | . | . | . | . | . | . | . | . | . | . | . | . | . | . | . | . | . |
| 21-225_146H1_LC | | . | . | . | . | . | . | . | . | . | . | . | . | . | . | . | . | . | . | . | . | . | . | . | . | . |
| 21-225_148C4_LC | | . | . | . | . | . | . | . | . | . | . | . | . | . | . | . | . | . | . | . | . | . | . | . | . | . |
| 21-225_148H9_LC | | . | . | . | . | . | . | . | . | . | . | . | . | . | . | . | . | . | . | . | . | . | . | . | . | . |
| 21-225_149A3_LC | | . | . | . | . | . | . | . | . | . | . | . | . | . | . | . | . | . | . | . | . | . | . | . | . | . |
| 21-225_150C11_LC | | . | . | . | . | . | . | . | . | . | . | . | . | . | . | . | . | . | . | . | . | . | . | . | . | . |
| 21-225_150C12_LC | | . | . | . | . | . | . | . | . | . | . | . | . | . | . | . | . | . | . | . | . | . | . | . | . | . |
| 21-225_151B12_LC | | . | . | . | . | . | . | . | . | . | . | . | . | . | . | . | . | . | . | . | . | . | . | . | . | . |
| 21-225_152B11_LC | | . | . | . | . | . | . | . | . | . | . | . | . | . | . | . | . | . | . | . | . | . | . | . | . | . |
| 21-225_152D2_LC | . | . | . | . | L | . | . | . | . | . | . | . | . | . | . | . | . | . | . | . | . | . | . | . | . | . |
| 21-225_152G4_LC | | . | . | . | . | . | . | . | . | . | . | . | . | . | . | . | . | . | . | . | . | . | . | . | . | . |
| 21-225_155C4_LC | | . | . | . | . | . | . | . | . | . | . | . | . | . | . | . | . | . | . | . | . | . | . | . | . | . |
| 21-225_156E4_LC | | . | . | . | . | . | . | . | . | . | . | . | . | . | . | . | . | . | . | . | . | . | . | . | . | . |
| 21-225_159H2_LC | . | . | L | . | . | . | . | . | . | . | . | . | . | . | . | . | . | . | . | . | . | . | . | . | . | . |
| 21-225_15A3_LC | | . | . | . | . | . | . | . | . | . | . | . | . | . | . | . | . | . | . | . | . | . | . | . | . | . |
| 21-225_16D11_LC | | . | . | . | . | . | . | . | . | . | . | . | . | . | . | . | . | . | . | . | . | . | . | . | . | . |
| 21-225_16E6_LC | | . | . | . | . | . | . | . | . | . | . | . | . | . | . | . | . | . | . | . | . | . | . | . | . | . |
| 21-225_17A6_LC | | . | . | . | . | . | . | . | . | . | . | . | . | . | . | . | . | . | . | . | . | . | . | . | . | . |
| 21-225_17G4_LC | . | . | . | . | . | . | . | . | . | . | . | . | . | A | . | . | . | . | . | . | . | . | . | . | T | . |
| 21-225_18G4_LC | . | H | . | . | . | . | . | . | . | . | . | . | . | . | . | . | . | . | . | . | . | . | . | . | . | . |
| 21-225_196F4_LC | . | P | . | . | I | . | . | . | . | . | . | . | . | . | . | . | . | . | . | . | . | . | . | . | . | . |
| 21-225_19A1_LC | . | . | . | . | . | . | . | . | . | . | . | . | . | . | . | . | . | . | . | . | . | . | . | . | . | . |
| 21-225_1H12_LC | | . | . | . | . | . | . | . | . | . | . | . | . | . | . | . | . | . | . | . | . | . | . | . | . | . |
| 21-225_201H10_LC | | . | . | . | . | . | . | . | . | . | . | . | . | . | . | . | . | . | . | . | . | . | . | . | . | . |
| 21-225_203E3_LC | . | . | . | . | . | . | . | . | P | . | . | . | . | . | . | . | . | . | . | . | . | . | H | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_204D6_LC | . | S | . | . | . | . | . | . | . | . | . | . | . | . | . | . | . | . | . | . | . | . | . | . | . | . |
| 21-225_204H4_LC | . | . | . | L | . | . | . | . | . | . | S | . | S | . | . | . | . | . | . | . | . | . | . | . | . | . |
| 21-225_206A4_LC | . | . | . | L | . | . | . | . | . | . | P | . | S | . | . | . | . | . | . | . | . | . | . | . | . | . |
| 21-225_206A5_LC | . | . | . | . | . | . | . | . | . | . | P | . | . | . | . | . | . | . | . | . | . | . | . | . | . | . |
| 21-225_207C9_LC | . | N | . | . | . | . | . | . | . | . | . | . | . | . | . | . | . | . | . | . | . | . | . | . | . | . |
| 21-225_208B11_LC | . | . | . | . | . | . | . | . | . | . | P | . | . | . | . | . | . | . | . | . | . | . | . | . | . | . |
| 21-225_208E8_LC | . | . | . | . | . | . | . | . | . | . | P | . | S | . | . | . | . | . | . | . | . | . | . | . | . | . |
| 21-225_20F6_LC | . | . | . | . | . | . | . | . | . | . | . | . | . | . | . | . | . | . | . | . | . | . | . | . | . | . |
| 21-225_210D11_LC | . | . | . | . | . | . | . | . | . | . | . | . | . | . | . | . | . | . | . | . | . | . | . | . | . | . |
| 21-225_22C7_LC | . | . | . | . | . | . | . | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . | . | . | . | . | . | . | . |
| 21-225_30G4_LC | . | . | . | . | . | . | S | . | . | . | . | . | Y | . | . | . | . | . | . | . | . | . | . | . | . | Y |
| 21-225_31F1_LC | . | . | . | . | . | . | . | . | . | . | . | . | . | . | . | . | . | . | . | . | . | . | . | . | . | . |
| 21-225_32D6_LC | . | . | . | . | . | . | . | . | . | . | . | . | T | . | . | . | . | . | . | . | . | . | . | . | . | . |
| 21-225_33B2_LC | . | . | . | . | . | . | . | . | . | . | . | . | T | . | . | . | . | S | . | . | . | . | . | . | . | . |
| 21-225_33C12_LC | . | . | . | . | . | . | . | . | . | . | . | . | Y | . | . | . | . | . | . | . | . | . | . | . | . | . |
| 21-225_34C11_LC | . | . | . | . | . | . | S | . | . | . | . | . | Y | . | . | . | . | . | . | . | S | . | . | . | . | . |
| 21-225_34C2_LC | . | . | . | . | . | . | . | . | . | . | . | . | T | S | . | . | . | . | . | . | . | . | . | . | . | . |
| 21-225_34D3_LC | . | . | . | . | . | . | . | . | . | . | . | . | . | . | . | . | . | . | . | . | . | . | . | . | . | . |
| 21-225_34D8_LC | . | . | . | . | . | . | . | . | . | . | . | . | Y | . | . | . | . | . | . | . | . | . | . | . | . | . |
| 21-225_34G11_LC | . | . | . | . | . | . | . | . | . | . | . | . | . | . | . | . | . | . | . | . | . | . | . | . | . | . |
| 21-225_36B8_LC | . | . | . | . | . | . | . | . | . | . | . | . | T | . | . | . | . | . | . | . | . | . | . | . | . | . |
| 21-225_3F8_LC | . | S | . | . | . | . | . | . | . | . | . | . | . | . | . | . | . | . | . | . | . | . | . | . | . | . |
| 21-225_3G7_LC | . | . | . | . | . | . | . | . | . | . | . | . | . | . | . | . | . | . | . | . | V | . | . | . | . | . |
| 21-225_48B10_LC | . | . | . | . | . | . | . | . | . | . | . | . | . | . | . | . | . | . | . | . | . | . | . | . | . | . |
| 21-225_48F12_LC | . | . | . | . | . | . | . | . | . | . | . | . | . | . | . | . | . | . | . | . | . | . | . | . | . | . |
| 21-225_48G12_LC | . | . | . | . | . | . | . | . | . | . | . | . | . | . | . | . | . | . | . | . | . | . | . | . | . | . |
| 21-225_4G4_LC | . | . | . | . | . | . | . | . | . | S | . | . | . | . | . | . | . | . | . | . | . | . | . | . | . | . |
| 21-225_50F2_LC | . | . | . | . | . | . | . | . | . | . | . | . | . | . | . | . | . | . | I | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | K_FR1 | | | | | | | | | | | | | |
| CONSENSUS | D | I | Q | M | T | Q | S | P | S | S | L | S | A | S | V | G | D | R | V | T | I | T | C | R | A | S |
| 21-225_56B6_LC | | | | | | | | | | | | | V | | | | | | | | | | | | | |
| 21-225_62D2_LC | | | | | | | | | | | | | | | | | | | | | | ? | | | | |
| 21-225_63C10_LC | | | | | | | | | | | | | T | | | | | | | | | | | | | |
| 21-225_64A11_LC | | | | | | | | | S | | | | | | | | | | | | | | | | | |
| 21-225_64F7_LC | | | | | | | | | | | | | | | | | | | | | | | | | | |
| 21-225_6G1_LC | | | | | | | | | | | | | | | | | | | | | | | | | Y | |
| 21-225_7A10_LC | | | | | | | | | | | | | | | | | | | | | | | | | | |
| 21-225_7E11_LC | | | | | | | | | | | | | | | | | | | | | | A | | | | |
| 21-225_7E11.001_LC | | | | | | | | | | | | | | | | | | | | | | A | | | | |
| 21-225_7E11.001.001_LC | | | | | | | | | | | | | | | | | | | | | | | | | | |
| 21-225_7E11.001.002_LC | | | | | | | | | | | | | | | | | | | | | | | | | | |
| 21-225_7E11.001.003_LC | | | | | | | | | | | | | | | | | | | | | | A | | | | |
| 21-225_7E11.001.004_LC | | | | | | | | | | | | | | | | | | | | | | A | | | | |
| 21-225_7E11.001.005_LC | | | | | | | | | | | | | | | | | | | | | | A | | | | |
| 21-225_7E11.001.006_LC | | | | | | | | | | | | | | | | | | | | | | A | | | | |
| 21-225_7E11.001.007_LC | | | | | | | | | | | | | | | | | | | | | | A | | | | |
| 21-225_7E11.001.008_LC | | | | | | | | | | | | | | | | | | | | | | A | | | | |
| 21-225_7E11.001.009_LC | | | | | | | | | | | | | | | | | | | | | | A | | | | |
| 21-225_7E11.001.010_LC | | | | | | | | | | | | | | | | | | | | | | A | | | | |
| 21-225_7E11.001.011_LC | | | | | | | | | | | | | | | | | | | | | | A | | | | |
| 21-225_7E11.001.012_LC | | | | | | | | | | | | | | | | | | | | | | | | | | |
| 21-225_7E11.001.013_LC | | | | | | | | | | | | | | | | | | | | | | | | | | |
| 21-225_7E11.001.014_LC | | | | | | | | | | | | | | | | | | | | | | | | | | |
| 21-225_7E11.001.015_LC | | | | | | | | | | | | | | | | | | | | | | | | | | |
| 21-225_7E11.001.016_LC | | | | | | | | | | | | | | | | | | | | | | | | | | |
| 21-225_7E11.001.017_LC | | | | | | | | | | | | | | | | | | | | | | | | | | |
| 21-225_7E11.001.018_LC | | | | | | | | | | | | | | | | | | | | | | | | | | |
| 21-225_7E11.001.019_LC | | | | | | | | | | | | | | | | | | | | | | A | | | | |
| 21-225_7E11.001.021_LC | | | | | | | | | | | | | | | | | | | | | | A | | | | |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_7E11.001.022_LC | . | . | . | . | . | . | . | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . | . | . | A | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | N | I | I | - | - | - | - | - | - | S | Y | L | N | W | Y | Q | Q | K | P | G | K | A | P |
| 21-225_10E12_LC | . | . | . | . | . | Y | . | . | . | . | . | . | . | . | . | . | . | . | . | S | . | . | . | R | . | . |
| 21-225_10F5_LC | . | . | . | . | . | Y | . | . | . | . | . | . | ® | . | . | . | . | . | . | S | . | . | . | R | . | . |
| 21-225_12A10_LC | . | . | . | . | . | Y | . | . | . | . | . | . | . | . | . | . | . | . | . | . | . | . | . | R | . | . |
| 21-225_13D4_LC | . | . | . | S | . | F | . | . | . | . | . | . | . | . | . | . | . | . | . | . | . | . | . | . | . | . |
| 21-225_146E9_LC | . | . | . | S | . | S | . | . | . | . | . | . | Q | . | . | . | . | . | . | L | . | . | . | . | . | . |
| 21-225_146H1_LC | . | . | . | S | . | S | . | . | . | . | . | . | D | . | . | . | . | . | . | . | . | . | . | . | . | . |
| 21-225_148C4_LC | . | . | . | S | . | . | . | . | . | . | . | . | N | . | . | . | . | . | . | . | . | . | . | . | . | . |
| 21-225_148H9_LC | . | . | . | S | . | S | . | . | . | . | . | . | N | . | . | . | . | . | . | . | . | . | . | . | . | . |
| 21-225_149A3_LC | . | . | . | S | . | S | . | . | . | . | . | . | . | . | . | . | . | . | . | . | . | . | . | . | . | . |
| 21-225_150C11_LC | . | . | . | S | . | S | . | . | . | . | . | . | . | . | . | . | . | . | . | . | . | . | . | . | . | . |
| 21-225_150C12_LC | . | . | . | S | . | S | . | . | . | . | . | . | D | . | . | . | . | . | . | . | . | . | . | . | . | . |
| 21-225_151B12_LC | . | . | . | S | . | S | . | . | . | . | . | . | N | F | . | . | . | . | . | . | . | . | . | . | . | . |
| 21-225_152B11_LC | . | . | . | S | . | S | . | . | . | . | . | . | D | . | . | . | . | . | . | . | . | . | . | . | . | . |
| 21-225_152D2_LC | . | . | . | S | . | S | . | . | . | . | . | . | D | . | . | . | . | . | . | L | . | . | . | . | . | . |
| 21-225_152G4_LC | . | . | . | S | . | S | . | . | . | . | . | . | D | . | . | . | . | . | . | . | . | . | . | . | . | . |
| 21-225_155C4_LC | . | . | . | S | . | S | . | . | . | . | . | . | . | . | . | . | . | . | . | . | . | . | . | . | . | . |
| 21-225_156E4_LC | . | . | . | S | . | S | . | . | . | . | . | . | . | . | . | . | . | . | . | . | . | . | . | . | . | . |
| 21-225_159H2_LC | . | . | . | S | . | S | . | . | . | . | . | . | N | . | . | . | . | . | . | . | . | . | . | . | . | . |
| 21-225_15A3_LC | . | . | . | S | . | . | . | . | . | . | . | . | . | . | . | . | . | . | L | . | . | . | . | . | . | . |
| 21-225_16D11_LC | . | . | . | T | . | S | . | . | . | . | . | . | N | . | . | . | . | X | . | . | . | . | . | . | . | . |
| 21-225_16E6_LC | . | . | . | S | . | . | . | . | . | . | . | . | . | . | . | . | . | . | . | . | . | . | . | . | . | . |
| 21-225_17A6_LC | . | . | . | T | . | S | . | . | . | . | . | . | N | . | . | . | . | ® | . | . | . | . | . | . | . | . |
| 21-225_17G4_LC | . | . | . | . | . | S | . | . | . | . | . | . | N | . | . | . | . | . | . | . | . | . | . | . | . | . |
| 21-225_18G4_LC | . | . | . | S | . | . | . | . | . | . | . | . | . | . | . | . | . | . | . | . | . | . | . | . | . | . |
| 21-225_196F4_LC | . | . | . | R | . | R | . | . | . | . | . | . | . | . | . | . | . | . | . | . | . | . | . | . | . | . |
| 21-225_19A1_LC | . | . | ® | T | . | T | . | . | . | . | . | . | . | . | . | . | . | . | . | . | . | . | . | . | . | . |
| 21-225_1H12_LC | . | . | . | . | . | Y | . | . | . | . | . | . | ® | . | . | . | . | . | . | S | . | . | . | ® | . | . |
| 21-225_201H10_LC | . | . | N | . | . | N | . | . | . | . | . | . | . | . | . | . | . | . | . | . | . | . | S | . | . | . |
| 21-225_203E3_LC | . | . | N | . | . | N | . | . | . | . | . | . | . | . | . | . | . | . | . | . | . | . | S | . | . | . |

# EP 4 435 105 A2

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | N | I | I | - | - | - | - | - | - | S | Y | L | N | W | Y | Q | Q | K | P | G | K | A | P |
| 21-225_204D6_LC | . | . | R | S | V | N | . | . | . | . | . | . | T | . | . | . | . | . | . | . | . | . | . | . | . | . |
| 21-225_204H4_LC | . | . | N | . | . | N | . | . | . | . | . | . | . | . | . | . | . | . | . | . | . | S | . | . | . | . |
| 21-225_206A4_LC | . | . | H | . | . | N | . | . | . | . | . | . | . | . | . | . | . | . | . | . | . | S | . | . | . | . |
| 21-225_206A5_LC | . | . | H | . | . | N | . | . | . | . | . | . | . | . | . | . | . | . | . | . | . | S | . | . | . | . |
| 21-225_207C9_LC | . | . | R | P | S | . | . | . | . | . | . | . | N | . | . | . | . | . | . | . | . | . | . | . | . | . |
| 21-225_208B11_LC | . | . | H | . | . | N | . | . | . | . | . | . | . | . | . | . | . | . | . | . | . | S | . | . | . | . |
| 21-225_208E8_LC | . | . | H | . | . | N | . | . | . | . | . | . | . | . | . | . | . | . | . | . | . | S | . | . | . | . |
| 21-225_20F6_LC | . | . | S | . | . | . | . | . | . | . | . | . | T | . | . | . | . | . | . | . | . | . | . | . | . | . |
| 21-225_210D11_LC | . | . | N | . | . | N | . | . | . | . | . | . | . | . | . | . | . | . | . | . | . | S | . | . | . | . |
| 21-225_22C7_LC | . | . | . | . | . | . | . | . | . | . | . | . | . | . | . | . | . | . | . | . | . | . | E | . | . | . |
| 21-225_30G1_LC | . | . | . | . | . | Y | . | . | . | . | . | . | . | . | . | . | . | . | . | . | . | . | . | . | D | . |
| 21-225_30G4_LC | . | . | . | . | . | F | . | . | . | . | . | . | . | . | . | . | . | N | . | . | . | . | . | . | . | . |
| 21-225_31F1_LC | . | . | R | . | . | S | . | . | . | . | . | . | . | . | . | . | . | . | . | . | . | . | . | . | . | . |
| 21-225_32D6_LC | . | . | . | . | . | . | . | . | . | . | . | . | . | . | . | . | . | . | . | . | . | . | . | . | . | . |
| 21-225_33B2_LC | . | . | S | . | . | . | . | . | . | . | . | . | . | . | . | . | . | . | . | . | . | . | . | . | . | . |
| 21-225_33C12_LC | . | . | S | . | . | . | . | . | . | . | . | . | . | . | . | . | . | . | . | . | . | . | . | . | . | . |
| 21-225_34C11_LC | . | . | . | . | . | R | . | . | . | . | . | . | N | F | . | . | . | . | . | . | . | . | E | . | D | . |
| 21-225_34C2_LC | . | . | R | . | . | . | . | . | . | . | . | . | . | . | . | . | F | . | . | . | . | . | . | . | . | . |
| 21-225_34D3_LC | . | . | . | . | . | Y | . | . | . | . | . | . | . | . | . | . | . | . | . | . | . | . | . | . | D | . |
| 21-225_34D8_LC | . | . | I | . | . | . | . | . | . | . | . | . | . | . | . | . | . | . | . | . | . | . | . | . | . | . |
| 21-225_34G11_LC | . | . | . | . | . | N | . | . | . | . | . | . | R | . | . | . | . | . | . | . | . | . | . | . | . | . |
| 21-225_36B8_LC | . | . | S | . | . | . | . | . | . | . | . | . | . | . | . | . | . | . | . | . | . | . | . | . | . | . |
| 21-225_3F8_LC | . | . | S | . | L | . | . | . | . | . | . | . | . | . | . | . | . | . | R | . | . | . | . | R | . | . |
| 21-225_3G7_LC | . | . | . | . | Y | . | . | . | . | . | . | . | R | . | . | . | . | . | . | . | Q | R | . | R | . | . |
| 21-225_48B10_LC | . | . | S | . | R | . | . | . | . | . | . | . | N | . | . | . | . | . | . | . | K | T | . | . | . | . |
| 21-225_48F12_LC | . | . | S | . | R | . | . | . | . | . | . | . | R | . | . | . | . | . | . | . | K | T | . | . | . | . |
| 21-225_48G12_LC | . | . | S | . | K | . | . | . | . | . | . | . | K | . | . | . | . | . | . | . | R | T | . | . | . | . |
| 21-225_4G4_LC | . | . | T | . | . | . | . | . | . | . | . | . | . | . | . | . | . | . | . | . | . | . | . | . | . | . |
| 21-225_50F2_LC | . | . | S | . | L | . | . | . | . | . | . | . | . | . | . | . | . | . | . | . | . | . | . | . | . | . |

4658

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | K_CDR1 | | | | | | | | | | | | | | | | | | K_FR2 | | | |
| CONSENSUS | - | - | Q | N | I | I | - | - | - | - | - | - | S | Y | L | N | W | Y | Q | Q | K | P | G | K | A | P |
| 21-225_56B6_LC | . | . | . | S | . | F | . | . | . | . | . | . | X | . | . | . | . | . | . | . | . | . | . | X | . | . |
| 21-225_62D2_LC | . | . | . | S | . | . | . | . | . | . | . | . | . | . | . | . | . | . | . | . | . | . | . | . | . | . |
| 21-225_63C10_LC | . | . | . | S | . | F | . | . | . | . | . | . | . | . | . | X | . | . | . | . | . | . | . | . | . | . |
| 21-225_64A11_LC | . | . | X | S | V | S | . | . | . | . | . | . | X | . | . | . | . | . | . | . | T | L | . | . | L | L |
| 21-225_64F7_LC | . | . | . | . | . | X | . | . | . | . | . | . | X | . | . | . | . | . | . | . | . | . | . | . | . | . |
| 21-225_6G1_LC | . | . | . | . | . | . | . | . | . | . | . | . | . | . | . | . | . | . | . | . | . | . | . | . | . | . |
| 21-225_7A10_LC | . | . | . | . | . | Y | . | . | . | . | . | . | . | . | . | . | . | . | . | . | . | . | . | X | . | . |
| 21-225_7E11_LC | . | . | . | . | . | . | . | . | . | . | . | . | . | . | . | . | . | . | . | . | . | . | . | . | . | . |
| 21-225_7E11.001_LC | . | . | . | . | . | . | . | . | . | . | . | . | . | . | . | . | . | . | . | . | . | . | . | . | . | . |
| 21-225_7E11.001.001_LC | . | . | . | . | . | . | . | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . | . | . | . | . | . | . | . |
| 21-225_7E11.001.004_LC | . | . | . | . | . | . | . | . | . | . | . | . | . | . | . | . | . | . | . | . | . | . | . | . | . | . |
| 21-225_7E11.001.005_LC | . | . | . | . | . | . | . | . | . | . | . | . | . | . | . | . | . | . | . | . | . | . | . | . | . | . |
| 21-225_7E11.001.006_LC | . | . | . | . | . | . | . | . | . | . | . | . | . | . | . | . | . | . | . | . | . | . | . | . | . | . |
| 21-225_7E11.001.007_LC | . | . | . | . | . | . | . | . | . | . | . | . | . | . | . | . | . | . | . | . | . | . | . | . | . | . |
| 21-225_7E11.001.008_LC | . | . | . | . | . | . | . | . | . | . | . | . | . | . | . | . | . | . | . | . | . | . | . | . | . | . |
| 21-225_7E11.001.009_LC | . | . | . | . | . | . | . | . | . | . | . | . | . | . | . | . | . | . | . | . | . | . | . | . | . | . |
| 21-225_7E11.001.010_LC | . | . | . | . | . | . | . | . | . | . | . | . | . | . | . | . | . | . | . | . | . | . | . | . | . | . |
| 21-225_7E11.001.011_LC | . | . | . | . | . | . | . | . | . | . | . | . | . | . | . | . | . | . | . | . | . | . | . | . | . | . |
| 21-225_7E11.001.012_LC | . | . | . | . | . | . | . | . | . | . | . | . | . | . | . | . | . | . | . | . | . | . | . | . | . | . |
| 21-225_7E11.001.013_LC | . | . | . | . | . | . | . | . | . | . | . | . | . | . | . | . | . | . | . | . | . | . | . | . | . | . |
| 21-225_7E11.001.014_LC | . | . | . | . | . | . | . | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . | . | . | . | . | . | . | . |
| 21-225_7E11.001.019_LC | . | . | . | . | . | . | . | . | . | . | . | . | . | . | . | . | . | . | . | . | . | . | . | . | . | . |
| 21-225_7E11.001.021_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | N | I | I | - | - | - | - | - | - | S | Y | L | N | W | Y | Q | Q | K | P | G | K | A | P |
| 21-225_7E11.001.022_LC | . | . | . | . | . | . | . | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | T | - | - | - | - | - | - | - | - | A | S | S | L | Q | S | G | V | P | S | R | F |
| 21-225_10E12_LC | . | . | . | . | . | A | . | . | . | . | . | . | . | . | T | . | . | . | . | . | . | . | . | . | . | . |
| 21-225_10F5_LC | . | . | . | . | . | . | . | . | . | . | . | . | . | . | . | . | . | . | . | . | . | . | . | . | . | . |
| 21-225_12A10_LC | . | V | . | . | M | . | . | . | . | . | . | . | . | . | . | . | . | . | . | . | . | . | . | . | . | . |
| 21-225_13D4_LC | . | . | . | . | C | . | . | . | . | . | . | . | . | . | . | . | . | . | . | N | . | . | . | . | . | . |
| 21-225_146E9_LC | . | V | . | . | . | . | . | . | . | . | . | . | . | . | T | . | . | . | . | . | . | . | . | . | . | . |
| 21-225_146H1_LC | . | V | . | . | . | . | . | . | . | . | . | . | . | . | T | . | . | . | . | . | . | . | . | . | . | . |
| 21-225_148C4_LC | . | V | . | . | . | . | . | . | . | . | . | . | . | . | . | . | . | . | . | . | . | . | . | . | . | . |
| 21-225_148H9_LC | . | V | . | . | I | . | . | . | . | . | . | . | . | . | . | . | . | . | . | . | . | . | . | . | . | . |
| 21-225_149A3_LC | . | V | M | . | . | . | . | . | . | . | . | . | . | . | . | . | . | . | . | . | . | . | . | . | . | . |
| 21-225_150C11_LC | . | . | . | . | . | . | . | . | . | . | . | . | . | . | . | . | . | . | . | . | . | . | . | . | . | . |
| 21-225_150C12_LC | . | V | . | . | . | . | . | . | . | . | . | . | . | . | T | . | . | . | . | . | . | . | . | . | . | . |
| 21-225_151B12_LC | . | V | . | . | . | . | . | . | . | . | . | . | . | . | . | . | . | . | E | . | . | . | . | . | . | . |
| 21-225_152B11_LC | . | V | . | . | . | . | . | . | . | . | . | . | . | . | T | . | . | . | . | . | . | . | . | . | . | . |
| 21-225_152D2_LC | . | V | . | . | . | . | . | . | . | . | . | . | . | . | T | . | . | . | . | . | . | . | . | . | . | . |
| 21-225_152G4_LC | . | V | . | . | . | . | . | . | . | . | . | . | . | . | T | . | . | . | . | . | . | . | . | . | . | . |
| 21-225_155C4_LC | . | V | . | . | F | . | . | . | . | . | . | . | . | . | . | . | . | . | . | . | . | . | . | . | . | . |
| 21-225_156E4_LC | . | V | . | . | . | . | . | . | . | . | . | . | . | . | . | . | . | . | . | . | . | . | . | . | . | . |
| 21-225_159H2_LC | E | . | . | . | . | A | . | . | . | . | . | . | . | . | T | . | N | . | . | . | . | . | . | . | . | . |
| 21-225_15A3_LC | . | F | . | . | . | . | . | . | . | . | . | . | . | . | . | . | . | . | . | . | . | . | . | . | . | . |
| 21-225_16D11_LC | . | . | . | . | . | A | . | . | . | . | . | . | . | . | . | . | . | . | . | . | . | . | . | . | . | . |
| 21-225_16E6_LC | . | F | . | . | . | . | . | . | . | . | . | . | . | . | T | . | . | . | . | . | . | . | . | . | . | . |
| 21-225_17A6_LC | . | . | . | . | . | A | . | . | . | . | . | . | . | . | . | . | . | . | . | . | . | . | . | . | . | . |
| 21-225_17G4_LC | . | . | . | . | . | V | . | . | . | . | . | . | . | . | . | . | . | F | P | . | . | . | . | . | . | . |
| 21-225_18G4_LC | . | V | . | . | . | . | . | . | . | . | . | . | . | . | . | . | . | . | . | . | T | . | . | . | G | . |
| 21-225_196F4_LC | . | . | . | . | . | . | . | . | . | . | . | . | . | . | . | . | . | . | . | G | . | . | . | . | . | . |
| 21-225_19A1_LC | . | F | . | . | . | A | . | . | . | . | . | . | . | . | T | . | N | . | . | . | . | . | . | . | . | . |
| 21-225_1H12_LC | . | . | . | . | . | . | . | . | . | . | . | . | . | . | . | . | . | . | . | . | . | . | . | . | . | . |
| 21-225_201H10_LC | . | . | . | . | . | A | . | . | . | . | . | . | . | . | . | . | . | . | . | . | . | . | . | . | . | . |
| 21-225_203E3_LC | . | . | . | . | . | A | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | T | - | - | - | - | - | - | - | - | A | S | S | L | Q | S | G | V | P | S | R | F |
| 21-225_204D6_LC | . | V | . | . | . | G | . | . | . | . | . | . | . | . | . | . | . | . | . | S | . | . | . | . | . | . |
| 21-225_204H4_LC | . | . | . | . | . | A | . | . | . | . | . | . | . | . | . | . | . | . | . | . | . | . | . | . | . | . |
| 21-225_206A4_LC | . | . | . | . | O | A | . | . | . | . | . | . | . | . | . | . | . | . | . | . | . | . | . | . | . | . |
| 21-225_206A5_LC | . | . | . | . | O | A | . | . | . | . | . | . | . | . | . | . | . | . | . | . | . | . | . | . | . | . |
| 21-225_207C9_LC | . | . | . | . | . | . | . | . | . | . | . | . | . | . | . | . | . | . | . | . | . | . | . | . | . | . |
| 21-225_208B11_LC | . | . | . | . | . | A | . | . | . | . | . | . | . | . | . | . | . | . | . | . | . | . | . | . | . | . |
| 21-225_208E8_LC | . | . | . | . | . | A | . | . | . | . | . | . | . | . | . | . | . | . | . | . | . | . | . | . | . | . |
| 21-225_20F6_LC | . | V | . | . | H | . | . | . | . | . | . | . | . | . | . | . | . | . | . | . | . | . | . | . | . | . |
| 21-225_210D11_LC | . | . | . | . | . | A | . | . | . | . | . | . | . | . | . | . | . | . | . | . | . | . | . | . | . | . |
| 21-225_22C7_LC | . | . | . | . | H | I | . | . | . | . | . | . | . | . | . | . | . | . | . | . | . | . | . | . | . | . |
| 21-225_30G1_LC | . | . | . | . | . | A | . | . | . | . | . | . | . | . | . | . | . | . | . | G | . | . | . | . | . | . |
| 21-225_30G4_LC | . | . | . | . | H | . | . | . | . | . | . | . | . | . | . | . | . | . | . | G | . | . | . | . | . | . |
| 21-225_31F1_LC | . | . | . | . | F | A | . | . | . | . | . | . | . | . | . | . | . | S | . | . | . | . | . | . | . | . |
| 21-225_32D6_LC | . | . | M | . | . | V | . | . | . | . | . | . | . | . | . | . | N | . | . | . | . | . | . | . | . | . |
| 21-225_33B2_LC | . | . | . | . | . | V | . | . | . | . | . | . | . | . | . | . | . | . | . | . | . | . | . | . | . | . |
| 21-225_33C12_LC | . | . | . | . | . | V | . | . | . | . | . | . | . | . | . | . | . | . | . | G | . | . | . | . | . | . |
| 21-225_34C11_LC | . | . | Q | . | . | G | . | . | . | . | . | . | . | . | . | . | T | . | . | . | . | . | . | . | . | . |
| 21-225_34C2_LC | . | . | . | . | . | V | . | . | . | . | . | . | . | . | . | . | . | . | . | . | . | . | . | . | . | . |
| 21-225_34D3_LC | . | . | . | . | . | A | . | . | . | . | . | . | . | . | . | . | . | . | H | . | . | . | . | . | . | . |
| 21-225_34D8_LC | . | . | . | . | P | V | . | . | . | . | . | . | . | . | . | . | . | . | . | . | . | . | . | . | . | . |
| 21-225_34G11_LC | . | . | . | . | . | G | . | . | . | . | . | . | . | . | . | . | . | . | . | . | . | . | . | . | . | . |
| 21-225_36B8_LC | . | . | . | . | . | V | . | . | . | . | . | . | . | . | . | . | . | . | . | . | . | . | . | . | . | . |
| 21-225_3F8_LC | Q | V | . | . | H | . | . | . | . | . | . | . | . | . | T | . | . | . | . | . | . | . | . | . | . | . |
| 21-225_3G7_LC | . | . | M | . | . | A | . | . | . | . | . | . | . | . | . | . | . | S | . | . | . | . | . | . | . | . |
| 21-225_48B10_LC | . | . | . | . | . | A | . | . | . | . | . | . | . | . | . | . | . | . | . | . | . | . | . | . | . | . |
| 21-225_48F12_LC | . | . | . | . | . | A | . | . | . | . | . | . | . | . | . | . | . | . | . | . | . | . | . | . | . | . |
| 21-225_48G12_LC | . | . | . | . | . | A | . | . | . | . | . | . | . | . | . | . | . | . | . | . | . | . | . | . | . | . |
| 21-225_4G4_LC | . | . | . | . | H | . | . | . | . | . | . | . | . | . | . | . | . | . | . | . | . | . | . | . | . | . |
| 21-225_50F2_LC | . | . | . | . | . | V | . | . | . | . | . | . | . | . | . | . | . | P | . | . | . | . | . | . | . | . |

4662

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | T | - | - | - | - | - | - | - | - | A | S | S | L | Q | S | G | V | P | S | R | F |
| 21-225_56B6_LC | . | . | . | . | S | A | . | . | . | . | . | . | . | . | . | . | . | S | . | . | . | . | . | . | . | . |
| 21-225_62D2_LC | . | . | . | . | . | A | . | . | . | . | . | . | . | . | T | . | . | . | . | . | . | . | . | . | . | . |
| 21-225_63C10_LC | . | . | . | . | S | A | . | . | . | . | . | . | . | . | . | . | . | . | . | . | . | . | . | . | . | . |
| 21-225_64A11_LC | . | . | . | . | S | V | . | . | . | . | . | . | . | . | . | . | R | . | . | . | . | . | . | . | . | . |
| 21-225_64F7_LC | . | S | . | . | . | G | . | . | . | . | . | . | . | . | . | . | . | . | . | . | . | . | . | . | . | . |
| 21-225_6G1_LC | . | V | . | . | . | . | . | . | . | . | . | . | . | . | . | . | N | . | . | T | . | . | . | . | G | . |
| 21-225_7A10_LC | . | . | V | . | . | S | . | . | . | . | . | . | . | . | T | . | . | . | . | . | . | . | . | . | . | . |
| 21-225_7E11_LC | . | S | . | . | . | . | . | . | . | . | . | . | . | . | . | . | . | . | . | . | . | . | . | . | . | . |
| 21-225_7E11.001_LC | . | S | . | . | . | . | . | . | . | . | . | . | . | . | . | . | . | . | . | . | . | . | . | . | . | . |
| 21-225_7E11.001.001_LC | . | S | . | . | . | . | . | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | S | . | . | . | . | . | . | . | . | . | . | . | . | . | . | . | . | . | . | . | . | . | . | . | . |
| 21-225_7E11.001.004_LC | . | S | . | . | . | . | . | . | . | . | . | . | . | . | . | . | . | . | . | . | . | . | . | . | . | . |
| 21-225_7E11.001.005_LC | . | S | . | . | . | . | . | . | . | . | . | . | . | . | . | . | . | . | . | . | . | . | . | . | . | . |
| 21-225_7E11.001.006_LC | . | S | . | . | . | . | . | . | . | . | . | . | . | . | . | . | . | . | . | . | . | . | . | . | . | . |
| 21-225_7E11.001.007_LC | . | S | . | . | . | . | . | . | . | . | . | . | . | . | . | . | . | . | . | . | . | . | . | . | . | . |
| 21-225_7E11.001.008_LC | . | S | . | . | . | . | . | . | . | . | . | . | . | . | . | . | . | . | . | . | . | . | . | . | . | . |
| 21-225_7E11.001.009_LC | . | S | . | . | . | . | . | . | . | . | . | . | . | . | . | . | . | . | . | . | . | . | . | . | . | . |
| 21-225_7E11.001.010_LC | . | S | . | . | . | . | . | . | . | . | . | . | . | . | . | . | . | . | . | . | . | . | . | . | . | . |
| 21-225_7E11.001.011_LC | . | S | . | . | . | . | . | . | . | . | . | . | . | . | . | . | . | . | . | . | . | . | . | . | . | . |
| 21-225_7E11.001.012_LC | . | S | . | . | . | . | . | . | . | . | . | . | . | . | . | . | . | . | . | . | . | . | . | . | . | . |
| 21-225_7E11.001.013_LC | . | S | . | . | . | . | . | . | . | . | . | . | . | . | . | . | . | . | . | . | . | . | . | . | . | . |
| 21-225_7E11.001.014_LC | . | S | . | . | . | . | . | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | S | . | . | . | . | . | . | . | . | . | . | . | . | . | . | . | . | . | . | . | . | . | . | . | . |
| 21-225_7E11.001.019_LC | . | S | . | . | . | . | . | . | . | . | . | . | . | . | . | . | . | . | . | . | . | . | . | . | . | . |
| 21-225_7E11.001.021_LC | . | S | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | T | - | - | - | - | - | - | - | - | A | S | S | L | Q | S | G | V | P | S | R | F |
| 21-225_7E11.001.022_LC | . | S | . | . | . | . | . | . | . | . | . | . | . | . | . | . | . | . | . | . | . | . | . | . | . | . |
| 21-225_7E11.001.023_LC | | S | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_10E12_LC | G | . | . | . | . | . | . | . | . | . | . | . | . | . | . | . | . | . | . | . | . | . | . | . | . | . |
| 21-225_10F5_LC | . | . | . | D | . | . | . | . | . | . | . | . | . | . | . | . | . | . | . | . | . | . | . | . | . | . |
| 21-225_12A10_LC | . | . | . | . | . | . | . | . | A | . | . | . | . | . | . | N | . | . | . | . | . | . | . | . | . | . |
| 21-225_13D4_LC | . | . | . | . | . | . | . | . | . | . | . | . | . | . | . | . | . | . | . | . | . | . | . | . | . | . |
| 21-225_146E9_LC | . | . | . | . | . | . | . | . | . | . | . | . | . | . | . | . | . | . | . | . | . | . | . | . | . | . |
| 21-225_146H1_LC | . | . | . | . | . | . | . | . | . | . | . | . | . | . | . | . | . | . | . | . | . | . | . | . | . | . |
| 21-225_148C4_LC | . | . | . | . | . | . | . | . | . | . | . | . | . | . | . | . | . | . | . | . | . | . | . | T | . | . |
| 21-225_148H9_LC | . | . | . | . | . | . | . | . | . | . | . | . | . | . | . | . | . | . | . | . | . | . | . | . | . | . |
| 21-225_149A3_LC | . | . | . | . | . | . | . | . | . | . | . | . | . | . | . | N | . | . | . | . | . | . | . | . | . | . |
| 21-225_150C11_LC | . | . | . | . | . | . | . | . | . | . | . | . | . | . | . | . | . | . | . | . | . | . | . | . | . | . |
| 21-225_150C12_LC | . | . | . | . | . | . | . | . | . | . | . | . | . | . | . | . | . | . | . | . | . | . | . | . | . | . |
| 21-225_151B12_LC | . | . | . | E | . | . | . | . | . | . | . | . | . | . | . | . | . | . | . | . | . | . | . | . | . | . |
| 21-225_152B11_LC | . | . | . | . | . | . | . | . | . | . | . | . | . | . | . | . | . | . | . | . | . | . | . | . | . | . |
| 21-225_152D2_LC | . | . | . | . | . | . | . | . | . | . | . | . | . | . | . | . | . | . | . | . | . | . | . | . | . | . |
| 21-225_152G4_LC | . | . | . | . | . | . | . | . | . | . | . | . | . | . | . | . | . | . | . | . | . | . | . | . | . | . |
| 21-225_155C4_LC | . | . | . | . | . | . | . | . | . | . | . | . | . | . | . | . | . | . | . | . | . | . | . | . | . | . |
| 21-225_156E4_LC | . | . | . | . | . | . | . | . | . | . | . | . | . | . | . | . | . | . | . | . | . | . | . | . | . | . |
| 21-225_159H2_LC | . | . | . | . | . | . | . | . | . | . | . | . | . | . | . | . | T | . | . | . | . | . | . | V | . | . |
| 21-225_15A3_LC | . | . | . | . | . | . | . | . | . | . | . | . | . | . | . | . | . | . | . | . | . | . | . | . | . | . |
| 21-225_16D11_LC | . | . | . | . | . | . | . | . | . | . | . | . | . | . | . | . | . | . | . | . | . | . | . | . | . | . |
| 21-225_16E6_LC | . | . | . | . | . | . | . | . | . | . | . | . | . | . | . | . | . | . | . | . | . | . | . | . | . | . |
| 21-225_17A6_LC | . | . | . | . | . | . | . | . | . | . | . | . | . | . | . | . | . | . | . | . | . | . | . | . | . | . |
| 21-225_17G4_LC | . | . | . | . | . | . | . | . | E | . | . | . | . | . | . | . | . | . | . | . | . | . | . | . | . | . |
| 21-225_18G4_LC | . | . | . | . | . | . | . | . | . | . | . | . | . | . | . | . | . | . | . | . | . | . | . | . | . | . |
| 21-225_196F4_LC | . | . | . | . | . | . | . | . | . | . | . | . | . | . | . | . | . | . | . | . | . | . | . | . | . | . |
| 21-225_19A1_LC | . | . | . | . | . | . | . | . | . | . | P | . | . | . | . | . | . | . | . | . | . | . | . | . | I | . |
| 21-225_1H12_LC | . | . | . | D | . | . | . | . | . | . | . | . | . | . | . | . | . | . | . | . | . | . | . | . | . | . |
| 21-225_201H10_LC | . | . | . | . | . | . | . | . | . | . | . | . | . | . | P | . | . | . | . | . | . | . | . | . | . | . |
| 21-225_203E3_LC | . | . | . | . | . | . | . | . | . | . | . | . | . | . | P | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_204D6_LC | . | . | . | . | . | . | . | . | . | . | . | . | . | . | . | . | . | . | . | . | . | . | V | . | . | . |
| 21-225_204H4_LC | . | . | . | . | . | . | . | . | . | . | . | . | . | . | P | . | . | . | . | . | . | . | . | . | . | . |
| 21-225_206A4_LC | . | . | . | . | . | . | . | . | . | . | . | . | . | . | P | . | . | . | . | . | . | . | . | . | . | . |
| 21-225_206A5_LC | . | . | . | . | . | . | . | . | . | . | . | . | . | . | P | . | . | . | . | . | . | . | . | . | . | . |
| 21-225_207C9_LC | . | A | . | V | . | . | . | . | . | . | . | . | . | . | . | . | . | . | . | . | . | . | . | . | . | . |
| 21-225_208B11_LC | . | . | . | . | . | . | . | . | . | . | . | . | . | . | P | . | . | . | . | . | . | . | . | . | . | . |
| 21-225_208E8_LC | . | . | . | . | . | . | . | . | . | . | . | . | . | . | P | . | . | . | . | . | . | . | . | . | . | . |
| 21-225_20F6_LC | . | . | . | . | . | . | . | . | . | . | . | . | . | . | . | . | . | . | . | . | . | . | . | . | . | . |
| 21-225_210D11_LC | . | . | . | . | . | . | . | . | . | . | . | . | . | . | P | . | . | . | . | . | . | . | . | . | . | . |
| 21-225_22C7_LC | . | . | . | . | . | . | . | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . | . | . | . | . | . | . | . |
| 21-225_30G4_LC | . | . | . | . | . | . | . | . | . | . | . | . | . | . | I | . | . | M | . | . | . | . | . | S | . | . |
| 21-225_31F1_LC | . | . | . | . | . | . | . | . | . | . | . | . | . | . | . | . | . | . | . | . | . | . | . | . | . | . |
| 21-225_32D6_LC | . | . | . | . | . | . | . | . | . | . | . | . | . | . | . | . | . | . | . | A | . | . | . | . | . | . |
| 21-225_33B2_LC | . | . | . | . | . | . | . | . | . | . | . | . | . | . | . | . | . | . | . | . | . | . | . | . | . | . |
| 21-225_33C12_LC | N | . | . | . | . | . | . | . | . | . | . | . | . | . | . | . | . | . | . | . | . | . | . | . | . | . |
| 21-225_34C11_LC | . | . | . | P | . | . | . | . | . | . | . | . | . | . | . | . | . | . | . | R | . | . | . | . | . | . |
| 21-225_34C2_LC | . | . | . | . | . | . | . | . | Y | . | . | . | . | . | . | . | . | . | . | . | . | . | . | . | . | . |
| 21-225_34D3_LC | . | . | . | . | . | . | . | . | . | . | . | . | . | . | . | . | . | . | . | . | . | . | . | . | . | P |
| 21-225_34D8_LC | . | . | . | . | . | . | . | . | . | . | . | . | . | . | . | . | . | . | . | . | . | . | . | . | . | . |
| 21-225_34G11_LC | . | . | . | . | . | . | . | . | . | . | . | . | . | . | . | T | . | . | . | . | . | . | . | . | . | . |
| 21-225_36B8_LC | . | . | . | . | . | . | . | . | . | . | . | . | . | . | . | . | . | . | . | . | . | . | . | . | . | . |
| 21-225_3F8_LC | . | . | . | . | . | . | . | . | V | . | . | . | . | . | . | . | . | . | . | . | . | . | . | . | . | . |
| 21-225_3G7_LC | . | . | . | . | . | . | . | . | . | . | . | V | . | . | . | . | . | . | . | . | . | . | . | . | . | . |
| 21-225_48B10_LC | . | . | . | . | . | . | . | . | . | . | . | . | . | . | . | . | . | . | V | . | . | . | . | . | . | . |
| 21-225_48F12_LC | . | . | . | . | . | . | . | . | . | . | . | . | . | . | . | . | . | . | . | . | . | . | . | . | N | . |
| 21-225_48G12_LC | . | . | . | . | . | . | . | . | . | . | . | . | . | . | . | . | . | . | V | . | . | . | . | . | N | . |
| 21-225_4G4_LC | . | . | . | . | . | . | . | . | . | . | . | . | . | . | S | . | . | . | . | . | . | . | . | . | . | . |
| 21-225_50F2_LC | . | . | . | . | . | . | . | . | . | . | . | . | . | . | . | . | . | . | . | . | D | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR4 | | | | | | | | | | | | | | | |
| CONSENSUS | S | G | S | G | S | G | - | - | T | D | F | T | L | T | I | S | S | L | Q | P | E | D | F | A | T | Y |
| 21-225_56B6_LC | . | . | . | . | . | . | . | . | . | . | . | . | . | . | . | . | . | . | . | . | . | . | . | . | . | . |
| 21-225_62D2_LC | . | . | . | . | . | . | . | . | . | . | . | . | . | . | . | . | . | . | . | . | . | . | . | . | . | . |
| 21-225_63C10_LC | . | . | . | . | . | . | . | . | . | . | . | . | . | . | . | . | . | . | . | . | . | . | . | . | . | F |
| 21-225_64A11_LC | . | . | . | . | . | . | . | . | . | . | . | . | . | . | . | . | . | V | . | R | . | . | . | . | . | . |
| 21-225_64F7_LC | . | . | . | . | . | . | . | . | . | . | . | . | . | . | . | . | . | . | . | . | . | . | . | . | A | . |
| 21-225_6G1_LC | . | . | . | . | . | . | . | . | . | . | . | . | . | . | . | . | . | . | . | . | . | . | . | . | . | . |
| 21-225_7A10_LC | . | . | . | . | . | . | . | . | . | . | S | . | . | . | . | N | . | . | . | . | . | . | S | . | . | . |
| 21-225_7E11_LC | . | . | . | . | . | . | . | . | . | . | . | . | . | . | . | . | . | . | . | . | . | . | . | I | . | . |
| 21-225_7E11.001_LC | . | . | . | . | . | . | . | . | . | . | . | . | . | . | . | . | . | . | . | . | . | . | . | I | . | . |
| 21-225_7E11.001.001_LC | . | . | . | . | . | . | . | . | . | . | . | . | . | . | . | . | . | . | . | . | . | . | . | I | . | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . | . | . | . | . | I | . | . |
| 21-225_7E11.001.004_LC | . | . | . | . | . | . | . | . | . | . | . | . | . | . | . | . | . | . | . | . | . | . | . | I | . | . |
| 21-225_7E11.001.005_LC | . | . | . | . | . | . | . | . | . | . | . | . | . | . | . | . | . | . | . | . | . | . | . | I | . | . |
| 21-225_7E11.001.006_LC | . | . | . | . | . | . | . | . | . | . | . | . | . | . | . | . | . | . | . | . | . | . | . | I | . | . |
| 21-225_7E11.001.007_LC | . | . | . | . | . | . | . | . | . | . | . | . | . | . | . | . | . | . | . | . | . | . | . | I | . | . |
| 21-225_7E11.001.008_LC | . | . | . | . | . | . | . | . | . | . | . | . | . | . | . | . | . | . | . | . | . | . | . | I | . | . |
| 21-225_7E11.001.009_LC | . | . | . | . | . | . | . | . | . | . | . | . | . | . | . | . | . | . | . | . | . | . | . | I | . | . |
| 21-225_7E11.001.010_LC | . | . | . | . | . | . | . | . | . | . | . | . | . | . | . | . | . | . | . | . | . | . | . | I | . | . |
| 21-225_7E11.001.011_LC | . | . | . | . | . | . | . | . | . | . | . | . | . | . | . | . | . | . | . | . | . | . | . | I | . | . |
| 21-225_7E11.001.012_LC | . | . | . | . | . | . | . | . | . | . | . | . | . | . | . | . | . | . | . | . | . | . | . | I | . | . |
| 21-225_7E11.001.013_LC | . | . | . | . | . | . | . | . | . | . | . | . | . | . | . | . | . | . | . | . | . | . | . | I | . | . |
| 21-225_7E11.001.014_LC | . | . | . | . | . | . | . | . | . | . | . | . | . | . | . | . | . | . | . | . | . | . | . | I | . | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . | . | . | . | . | I | . | . |
| 21-225_7E11.001.019_LC | . | . | . | . | . | . | . | . | . | . | . | . | . | . | . | . | . | . | . | . | . | . | . | I | . | . |
| 21-225_7E11.001.021_LC | . | . | . | . | . | . | . | . | . | . | . | . | . | . | . | . | . | . | . | . | . | . | . | I | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_7E11.001.022_LC | . | . | . | . | . | . | . | . | . | . | . | . | . | . | . | . | . | . | . | . | . | . | . | . | : | . |
| 21-225_7E11.001.023_LC | . | . | . | . | . | . | . | . | . | . | . | . | . | . | . | . | . | . | . | . | . | . | . | . | : | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_10E12_LC | . | . | . | . | N | . | . | . | . | . | . | . | . | . | . | . | . | . | . | . | . | . | . | . | . | . |
| 21-225_10F5_LC | . | . | . | . | . | . | . | . | . | . | . | . | . | . | . | . | . | . | . | . | . | . | . | . | . | . |
| 21-225_12A10_LC | . | . | . | . | . | . | . | . | . | . | . | . | . | . | . | . | . | . | . | . | . | . | . | . | . | . |
| 21-225_13D4_LC | . | . | . | . | T | . | . | . | . | . | . | . | . | . | . | . | . | . | . | . | . | . | . | . | . | . |
| 21-225_146E9_LC | F | . | . | . | . | - | . | . | . | . | . | . | . | . | . | . | . | . | . | . | . | . | . | . | . | . |
| 21-225_146H1_LC | . | . | . | . | . | - | . | . | . | . | . | . | . | . | . | . | . | . | . | . | . | . | . | . | . | . |
| 21-225_148C4_LC | . | . | . | . | . | - | . | . | . | . | . | . | . | . | . | . | . | . | . | . | . | . | . | . | . | . |
| 21-225_148H9_LC | . | . | . | . | . | - | . | . | . | . | . | . | . | . | . | . | . | . | . | . | . | . | . | . | . | . |
| 21-225_149A3_LC | . | . | . | . | . | - | . | . | . | . | . | . | . | . | . | . | . | . | . | . | . | . | . | . | . | . |
| 21-225_150C11_LC | . | . | . | . | . | - | . | . | . | . | . | . | . | . | . | . | . | . | . | . | . | . | . | . | . | . |
| 21-225_150C12_LC | . | . | . | . | . | F | - | . | . | . | . | . | . | . | . | . | . | . | . | . | . | . | . | . | . | . |
| 21-225_151B12_LC | . | . | . | . | . | . | - | . | . | . | . | . | . | . | . | . | . | . | . | . | . | . | . | . | . | . |
| 21-225_152B11_LC | F | . | . | . | . | . | - | . | . | . | . | . | . | . | . | . | . | . | . | . | . | . | . | . | . | . |
| 21-225_152D2_LC | F | . | . | . | . | . | - | . | . | . | . | . | . | . | . | . | . | . | . | . | . | . | . | . | . | . |
| 21-225_152G4_LC | F | . | . | . | . | . | - | . | . | . | . | . | . | . | . | . | . | . | . | . | . | . | . | . | . | . |
| 21-225_155C4_LC | . | . | . | . | . | . | - | . | . | . | . | . | . | . | . | . | . | . | . | . | . | . | . | . | . | . |
| 21-225_156E4_LC | . | . | . | . | . | . | - | . | . | . | . | . | . | . | . | . | . | . | . | . | . | . | . | . | . | . |
| 21-225_159H2_LC | . | . | . | . | . | . | . | . | . | . | . | . | . | . | . | . | . | . | . | . | . | . | . | . | . | . |
| 21-225_15A3_LC | . | . | . | . | T | . | . | . | . | . | . | . | . | . | . | . | . | . | . | . | . | . | . | . | . | . |
| 21-225_16D11_LC | . | . | . | . | H | . | . | . | . | . | . | . | . | . | . | . | . | . | . | . | . | . | . | . | . | . |
| 21-225_16E6_LC | . | . | . | . | T | . | . | . | . | . | . | . | . | . | . | . | . | . | . | . | . | . | . | . | . | . |
| 21-225_17A6_LC | . | . | . | . | . | H | T | . | . | . | . | . | . | . | . | . | . | . | . | . | . | . | . | . | . | . |
| 21-225_17G4_LC | . | . | . | . | . | N | F | . | . | . | . | . | . | . | . | . | . | . | . | . | . | . | . | . | . | . |
| 21-225_18G4_LC | . | . | . | . | T | . | . | . | . | . | . | . | . | . | . | . | . | . | . | . | . | . | . | . | . | . |
| 21-225_196F4_LC | . | . | . | . | . | G | . | . | . | . | . | . | . | . | . | . | . | . | . | . | . | . | . | . | . | . |
| 21-225_19A1_LC | . | . | . | . | . | N | . | . | . | . | . | . | . | . | . | . | . | . | . | . | . | . | . | . | . | . |
| 21-225_1H12_LC | . | . | . | . | . | . | . | . | . | . | . | . | . | . | . | . | . | . | . | . | . | . | . | . | . | . |
| 21-225_201H10_LC | . | . | . | . | . | . | . | . | . | . | . | . | . | . | . | . | . | . | . | . | . | . | . | . | . | . |
| 21-225_203E3_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_204D6_LC | . | . | . | . | . | . | . | . | . | . | . | . | . | . | . | . | . | . | . | . | . | . | . | . | . | . |
| 21-225_204H4_LC | . | . | . | . | . | . | . | . | . | . | . | . | . | . | . | . | . | . | . | . | . | . | . | . | . | . |
| 21-225_206A4_LC | . | . | . | . | . | . | . | . | . | . | . | . | . | . | . | . | . | . | . | . | . | . | . | . | . | . |
| 21-225_206A5_LC | . | . | . | . | . | . | . | . | . | . | . | . | . | . | . | . | . | . | . | . | . | . | . | . | . | . |
| 21-225_207C9_LC | . | . | . | . | . | . | . | . | . | . | . | . | . | . | . | . | . | . | . | . | . | . | . | . | . | . |
| 21-225_208B11_LC | . | . | . | . | . | . | . | . | . | . | . | . | . | . | . | . | . | . | . | . | . | . | . | . | . | . |
| 21-225_208E8_LC | . | . | . | . | . | . | . | . | . | . | . | . | . | . | . | . | . | . | . | . | . | . | . | . | . | . |
| 21-225_20F6_LC | . | . | . | T | . | . | . | . | . | . | . | . | . | . | . | . | . | . | . | . | . | . | . | . | . | . |
| 21-225_210D11_LC | . | . | . | . | . | . | . | . | . | . | . | . | . | . | . | . | . | . | . | . | . | . | . | . | . | . |
| 21-225_22C7_LC | . | . | . | T | . | . | . | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . | . | . | . | . | . | . | . |
| 21-225_30G4_LC | . | . | λ | . | . | . | . | . | . | . | . | . | . | . | . | . | . | . | . | . | . | . | . | . | . | . |
| 21-225_31F1_LC | . | . | . | . | . | ! | . | . | . | . | . | . | . | . | . | . | . | . | . | . | . | . | . | . | . | . |
| 21-225_32D6_LC | . | . | . | . | . | . | . | . | . | . | . | . | . | . | . | . | . | . | . | . | . | . | . | . | . | . |
| 21-225_33B2_LC | . | . | . | . | . | . | . | . | . | . | . | . | . | . | . | . | . | . | . | . | . | . | . | . | . | . |
| 21-225_33C12_LC | . | . | . | . | . | . | . | . | . | . | . | . | . | . | . | . | . | . | . | . | . | . | . | . | . | . |
| 21-225_34C11_LC | . | . | . | T | C | . | . | . | . | . | . | . | . | . | . | . | . | . | . | . | . | . | . | . | . | . |
| 21-225_34C2_LC | . | . | . | . | . | . | . | . | . | . | . | . | . | . | . | . | . | . | . | . | . | . | . | . | . | . |
| 21-225_34D3_LC | . | . | . | . | . | . | . | . | . | . | . | . | . | . | . | . | . | . | . | . | . | . | . | . | . | . |
| 21-225_34D8_LC | . | . | . | . | . | . | . | . | . | . | . | . | . | . | . | . | . | . | . | . | . | . | . | . | . | . |
| 21-225_34G11_LC | . | . | . | T | . | ! | . | . | . | . | . | . | . | . | . | . | . | . | . | . | . | . | . | . | . | . |
| 21-225_36B8_LC | . | . | . | . | . | . | . | . | . | . | . | . | . | . | . | . | . | . | . | . | . | . | . | . | . | . |
| 21-225_3F8_LC | . | . | . | T | . | . | . | . | . | . | . | . | . | . | . | . | . | . | . | . | . | . | . | . | . | . |
| 21-225_3G7_LC | . | . | . | . | . | . | . | . | . | . | . | . | . | . | . | . | . | . | . | . | . | . | . | . | . | . |
| 21-225_48B10_LC | . | . | . | T | . | . | . | . | . | . | . | . | . | . | . | . | . | . | . | . | . | . | . | . | . | . |
| 21-225_48F12_LC | . | . | . | T | . | . | . | . | . | . | . | . | . | . | . | . | . | . | . | . | . | . | . | . | . | . |
| 21-225_48G12_LC | . | . | . | T | . | . | . | . | . | . | . | . | . | . | . | . | . | . | . | . | . | . | . | . | . | . |
| 21-225_4G4_LC | . | . | . | T | . | . | . | . | . | . | . | . | . | . | . | . | . | . | . | . | . | . | . | . | . | . |
| 21-225_50F2_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_56B6_LC | . | . | . | . | T | . | . | . | . | . | . | . | . | . | . | . | . | . | . | . | . | . | . | . | . | . |
| 21-225_62D2_LC | . | . | . | . | T | . | . | . | . | . | . | . | . | . | . | . | . | . | . | . | . | . | . | . | . | . |
| 21-225_63C10_LC | . | . | . | . | T | . | . | . | . | . | . | . | . | . | . | . | . | . | . | . | . | . | . | . | . | . |
| 21-225_64A11_LC | S | . | . | . | . | D | . | . | . | . | . | . | . | . | . | . | . | . | . | . | . | . | . | . | . | . |
| 21-225_64F7_LC | . | . | . | . | . | . | N | . | . | . | . | . | . | . | . | . | . | . | . | . | . | . | . | . | . | . |
| 21-225_6G1_LC | . | . | . | . | P | H | . | . | . | . | . | . | . | . | . | . | . | . | . | . | . | . | . | . | . | . |
| 21-225_7A10_LC | . | . | . | . | . | . | . | . | . | . | . | . | . | . | . | . | . | . | . | . | . | . | . | . | . | . |
| 21-225_7E11_LC | . | . | . | . | T | . | . | . | . | . | . | . | . | . | . | . | . | . | . | . | . | . | . | . | . | . |
| 21-225_7E11.001_LC | . | . | . | . | T | . | . | . | . | . | . | . | . | . | . | . | . | . | . | . | . | . | . | . | . | . |
| 21-225_7E11.001.001_LC | . | . | . | . | T | . | . | . | . | . | . | . | . | . | . | . | . | . | . | . | . | . | . | . | . | . |
| 21-225_7E11.001.002_LC | . | . | . | . | T | . | . | . | . | . | . | . | . | . | . | . | . | . | . | . | . | . | . | . | . | . |
| 21-225_7E11.001.003_LC | . | . | . | . | T | . | . | . | . | . | . | . | . | . | . | . | . | . | . | . | . | . | . | . | . | . |
| 21-225_7E11.001.004_LC | . | . | . | . | T | . | . | . | . | . | . | . | . | . | . | . | . | . | . | . | . | . | . | . | . | . |
| 21-225_7E11.001.005_LC | . | . | . | . | T | . | . | . | . | . | . | . | . | . | . | . | . | . | . | . | . | . | . | . | . | . |
| 21-225_7E11.001.006_LC | . | . | . | . | T | . | . | . | . | . | . | . | . | . | . | . | . | . | . | . | . | . | . | . | . | . |
| 21-225_7E11.001.007_LC | . | . | . | . | Y | . | . | . | . | . | . | . | . | . | . | . | . | . | . | . | . | . | . | . | . | . |
| 21-225_7E11.001.008_LC | . | . | . | . | T | . | . | . | . | . | . | . | . | . | . | . | . | . | . | . | . | . | . | . | . | . |
| 21-225_7E11.001.009_LC | . | . | . | . | T | . | . | . | . | . | . | . | . | . | . | . | . | . | . | . | . | . | . | . | . | . |
| 21-225_7E11.001.010_LC | . | . | . | . | T | . | . | . | . | . | . | . | . | . | . | . | . | . | . | . | . | . | . | . | . | . |
| 21-225_7E11.001.011_LC | . | . | . | . | T | . | . | . | . | . | . | . | . | . | . | . | . | . | . | . | . | . | . | . | . | . |
| 21-225_7E11.001.012_LC | . | . | . | . | T | . | . | . | . | . | . | . | . | . | . | . | . | . | . | . | . | . | . | . | . | . |
| 21-225_7E11.001.013_LC | . | . | . | . | T | . | . | . | . | . | . | . | . | . | . | . | . | . | . | . | . | . | . | . | . | . |
| 21-225_7E11.001.014_LC | . | . | . | . | T | . | . | . | . | . | . | . | . | . | . | . | . | . | . | . | . | . | . | . | . | . |
| 21-225_7E11.001.015_LC | . | . | . | . | T | . | . | . | . | . | . | . | . | . | . | . | . | . | . | . | . | . | . | . | . | . |
| 21-225_7E11.001.016_LC | . | . | . | . | T | . | . | . | . | . | . | . | . | . | . | . | . | . | . | . | . | . | . | . | . | . |
| 21-225_7E11.001.017_LC | . | . | . | . | T | . | . | . | . | . | . | . | . | . | . | . | . | . | . | . | . | . | . | . | . | . |
| 21-225_7E11.001.018_LC | . | . | . | . | T | . | . | . | . | . | . | . | . | . | . | . | . | . | . | . | . | . | . | . | . | . |
| 21-225_7E11.001.019_LC | . | . | . | . | T | . | . | . | . | . | . | . | . | . | . | . | . | . | . | . | . | . | . | . | . | . |
| 21-225_7E11.001.021_LC | . | . | . | . | T | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_7E11.001.022_LC | . | . | . | . | Y | . | . | . | . | . | . | . | . | . | . | . | . | . | . | . | . | . | . | . | . | . |
| 21-225_7E11.001.023_LC | . | . | . | . | T | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | L | T | F | G | G | G | T | K | V | E | I | K | R |
| 21-225_10E12_LC | . | . | . | . | P | . | . | . | . | . | . | . | . | . | . | . | . | E | . |
| 21-225_10F5_LC | . | . | . | . | P | . | . | . | . | . | . | . | . | . | . | . | . | . | . |
| 21-225_12A10_LC | . | . | . | . | P | . | . | . | . | . | . | . | . | . | . | . | . | . | . |
| 21-225_13D4_LC | . | . | . | . | L | . | . | . | . | . | . | . | . | . | . | . | . | . | . |
| 21-225_146E9_LC | . | . | . | . | S | T | P | . | . | . | . | . | . | . | . | . | . | . | . |
| 21-225_146H1_LC | . | . | . | . | S | T | P | . | . | . | . | . | . | . | . | . | . | . | . |
| 21-225_148C4_LC | . | . | . | . | S | T | P | . | . | . | . | . | . | . | . | . | . | E | . |
| 21-225_148H9_LC | . | . | . | . | S | T | P | . | . | . | . | . | . | . | . | . | . | . | . |
| 21-225_149A3_LC | . | . | . | . | S | I | P | . | . | . | . | . | . | . | . | . | . | . | . |
| 21-225_150C11_LC | . | . | . | . | S | I | Y | . | . | . | . | . | S | . | . | . | . | . | . |
| 21-225_150C12_LC | . | . | . | . | S | T | P | . | . | . | . | . | . | . | . | . | . | . | . |
| 21-225_151B12_LC | . | . | . | . | S | T | P | . | . | . | . | . | . | R | . | . | . | . | . |
| 21-225_152B11_LC | . | . | . | . | S | T | P | . | . | . | . | . | . | . | . | . | . | . | . |
| 21-225_152D2_LC | . | . | . | . | S | T | P | . | . | . | . | . | . | . | . | . | . | . | . |
| 21-225_152G4_LC | . | . | . | . | S | T | P | . | . | . | . | . | . | . | . | . | . | . | . |
| 21-225_155C4_LC | . | . | . | . | S | T | P | . | . | . | . | . | . | R | . | . | . | . | . |
| 21-225_156E4_LC | . | . | . | . | S | A | P | . | . | . | . | . | . | . | . | . | . | . | . |
| 21-225_159H2_LC | . | . | . | . | L | . | V | . | . | . | . | . | . | . | . | . | . | . | . |
| 21-225_15A3_LC | . | . | . | . | . | . | . | . | . | . | . | . | . | . | . | . | . | . | . |
| 21-225_16D11_LC | . | . | . | . | S | . | . | . | . | . | . | . | . | . | . | . | . | . | . |
| 21-225_16E6_LC | . | . | . | . | . | . | . | . | . | . | . | . | . | . | . | . | . | . | . |
| 21-225_17A6_LC | . | . | . | . | S | . | . | . | . | . | . | . | . | . | . | . | . | . | . |
| 21-225_17G4_LC | . | . | . | . | F | . | . | . | . | . | . | . | . | . | . | . | . | I | . |
| 21-225_18G4_LC | . | . | . | . | P | . | . | . | . | . | . | . | . | . | . | . | . | . | . |
| 21-225_196F4_LC | . | . | . | . | S | . | . | . | . | . | . | . | . | . | . | . | . | . | . |
| 21-225_19A1_LC | . | . | . | . | F | . | . | . | . | . | . | . | . | . | . | . | . | . | . |
| 21-225_1H12_LC | . | . | . | . | P | . | . | . | . | . | . | . | . | . | . | . | . | . | . |
| 21-225_201H10_LC | . | . | . | . | F | . | . | . | . | . | . | . | . | . | . | M | R | . | . |
| 21-225_203E3_LC | . | . | . | . | F | . | . | . | . | . | . | . | . | . | . | M | R | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | L | T | F | G | G | G | T | K | V | E | I | K | R |
| 21-225_204D6_LC | . | . | . | . | S | . | . | . | . | . | . | . | . | . | . | . | . | Q | . |
| 21-225_204H4_LC | . | . | . | . | P | . | . | . | . | . | . | . | . | . | . | . | M | R | . |
| 21-225_206A4_LC | . | . | . | . | P | . | . | . | . | . | . | . | . | . | . | . | M | R | . |
| 21-225_206A5_LC | . | . | . | . | P | . | . | . | . | . | . | . | . | . | . | . | M | R | . |
| 21-225_207C9_LC | . | . | . | . | I | . | . | . | . | . | . | . | . | . | . | . | . | . | . |
| 21-225_208B11_LC | . | . | . | . | P | . | . | . | . | . | . | . | . | . | . | . | M | R | . |
| 21-225_208E8_LC | . | . | . | . | P | . | . | . | . | . | . | . | . | . | . | . | M | R | . |
| 21-225_20F6_LC | . | . | . | . | P | . | . | . | . | . | . | . | . | . | . | . | . | . | . |
| 21-225_210D11_LC | . | . | . | . | P | . | . | . | . | . | . | . | . | . | . | . | M | R | . |
| 21-225_22C7_LC | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . |
| 21-225_30G4_LC | . | . | . | . | P | . | Y | . | . | . | . | . | . | . | . | . | . | . | . |
| 21-225_31F1_LC | . | . | . | . | . | . | . | . | . | . | . | . | . | T | . | . | . | R | . |
| 21-225_32D6_LC | . | . | . | . | . | Y | . | . | . | . | . | . | . | . | . | A | . | . | . |
| 21-225_33B2_LC | . | . | . | . | . | Y | . | . | . | . | . | . | . | . | . | . | . | . | . |
| 21-225_33C12_LC | . | . | . | . | . | Y | . | . | . | . | . | . | . | . | . | . | . | . | . |
| 21-225_34C11_LC | . | . | . | . | . | . | . | . | . | . | . | . | . | . | . | . | . | . | . |
| 21-225_34C2_LC | . | . | . | . | . | Y | . | . | . | . | . | . | . | . | M | . | . | . | . |
| 21-225_34D3_LC | . | . | . | . | . | . | . | . | . | . | . | . | . | . | . | . | . | . | . |
| 21-225_34D8_LC | . | . | . | . | . | Y | . | . | . | . | . | . | . | . | . | . | . | . | . |
| 21-225_34G11_LC | . | . | . | . | . | . | . | . | . | . | . | . | . | . | . | . | . | . | . |
| 21-225_36B8_LC | . | . | . | . | . | Y | . | . | . | . | . | . | . | . | M | . | . | . | . |
| 21-225_3F8_LC | . | . | . | . | . | . | . | . | . | . | . | . | . | R | . | . | . | N | . |
| 21-225_3G7_LC | . | . | . | . | P | . | P | . | . | . | . | . | A | . | . | . | . | Q | . |
| 21-225_48B10_LC | . | . | . | . | N | . | . | . | . | . | . | . | . | . | . | . | P | T | . |
| 21-225_48F12_LC | . | . | . | . | N | . | . | . | . | . | . | . | . | S | . | . | . | T | . |
| 21-225_48G12_LC | . | . | . | . | N | . | . | . | . | . | . | . | . | S | . | . | . | T | . |
| 21-225_4G4_LC | . | . | . | . | . | . | . | . | . | . | . | . | . | . | . | . | . | . | . |
| 21-225_50F2_LC | . | . | . | . | P | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | L | T | F | G | G | G | T | K | V | E | I | K | R |
| 21-225_56B6_LC | . | . | . | . | P | . | . | . | . | . | . | . | . | . | . | . | . | . | . |
| 21-225_62D2_LC | . | . | . | . | P | . | . | . | . | . | . | . | . | . | . | . | . | . | . |
| 21-225_63C10_LC | . | . | . | . | P | . | . | . | . | . | . | . | . | . | . | . | . | . | . |
| 21-225_64A11_LC | . | . | . | . | S | . | . | . | . | . | . | . | . | . | . | . | . | . | . |
| 21-225_64F7_LC | . | . | . | . | I | S | P | . | . | . | . | . | . | . | . | . | L | . | . |
| 21-225_6G1_LC | . | . | . | . | P | . | . | . | . | . | . | . | . | . | . | . | . | . | . |
| 21-225_7A10_LC | . | . | . | . | P | . | . | . | . | . | . | . | . | . | . | . | . | R | . |
| 21-225_7E11_LC | . | . | . | . | . | . | . | . | . | . | . | . | . | . | . | . | . | . | . |
| 21-225_7E11.001_LC | . | . | . | . | . | . | . | . | . | . | . | . | . | . | . | . | . | . | . |
| 21-225_7E11.001.001_LC | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . |
| 21-225_7E11.001.004_LC | . | . | . | . | . | . | . | . | . | . | . | . | . | . | . | . | . | . | . |
| 21-225_7E11.001.005_LC | . | . | . | . | . | . | . | . | . | . | . | . | . | . | . | . | . | . | . |
| 21-225_7E11.001.006_LC | . | . | . | . | . | . | . | . | . | . | . | . | . | . | . | . | . | . | . |
| 21-225_7E11.001.007_LC | . | . | . | . | . | . | . | . | . | . | . | . | . | . | . | . | . | . | . |
| 21-225_7E11.001.008_LC | . | . | . | . | . | . | . | . | . | . | . | . | . | . | . | . | . | . | . |
| 21-225_7E11.001.009_LC | . | . | . | . | . | . | . | . | . | . | . | . | . | . | . | . | . | . | . |
| 21-225_7E11.001.010_LC | . | . | . | . | . | . | . | . | . | . | . | . | . | . | . | . | . | . | . |
| 21-225_7E11.001.011_LC | . | . | . | . | . | . | . | . | . | . | . | . | . | . | . | . | . | . | . |
| 21-225_7E11.001.012_LC | . | . | . | . | . | . | . | . | . | . | . | . | . | . | . | . | . | . | . |
| 21-225_7E11.001.013_LC | . | . | . | . | . | . | . | . | . | . | . | . | . | . | . | . | . | . | . |
| 21-225_7E11.001.014_LC | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . |
| 21-225_7E11.001.019_LC | . | . | . | . | . | . | . | . | . | . | . | . | . | . | . | . | . | . | . |
| 21-225_7E11.001.021_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | L | T | F | G | G | G | T | K | V | E | I | K | R |
| 21-225_7E11.001.022_LC | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . |

**FIGURE 57**

Table 103.    Consensus 23 - VK1|L1/JK3 (SEQ ID NO: 50320):

DIQMTQSPSSLSASVGDRVTITC<u>RAS--QGIS------NYLA</u>WFQQKPGKAPKSLIY<u>A-------ASSLQS</u>GVPSKFSGSGSG--TDFTLTISSLQPEDFATYYC<u>QQYNS--------------------YPFT</u>FGPGTKVDIKR wherein:

R at position 24 can be substituted with P

A at position 25 can be substituted with T

S at position 26 can be substituted with N

Q at position 29 can be substituted with R

G at position 30 can be substituted with D, V or A

I at position 31 can be substituted with V

S at position 32 can be substituted with N, G, R, T or K

N at position 39 can be substituted with K, Y, H, I or T

Y at position 40 can be substituted with H

A at position 42 can be substituted with V or S

A at position 58 can be substituted with V, G or T

A at position 67 can be substituted with S or V

S at position 68 can be substituted with F

S at position 69 can be substituted with G, N or T

L at position 70 can be substituted with V

Q at position 71 can be substituted with R, H, L or E

S at position 72 can be substituted with G or T

FIGURE 57

Q at position 107 can be substituted with H, L, P or R

Q at position 108 can be substituted with R, K, H or L

Y at position 109 can be substituted with F

N at position 110 can be substituted with H, D, Y, S, K, L, M or Q

S at position 111 can be substituted with T, G, N, C or D

Y at position 135 can be substituted with F or H

F at position 137 can be substituted with V, L or I

T at position 138 can be substituted with K

Xaa Xaa Xaa Xaa Xaa Xaa Xaa Xaa Xaa Leu Xaa (SEQ ID NO: 50144)

Xaa Xaa Xaa Xaa Xaa Xaa Xaa (SEQ ID NO: 50145)

Xaa Xaa Xaa Xaa Xaa Xaa Pro Xaa Xaa (SEQ ID NO: 50146)

Arg Ala Ser Gln Gly Ile Ser Asn Tyr Leu Ala (SEQ ID NO: 50630)

Ala Ala Ser Ser Leu Gln Ser (SEQ ID NO: 50631)

Gln Gln Tyr Asn Ser Tyr Pro Phe Thr (SEQ ID NO: 50632)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | K_FR1 | | | | | | | | | | | | | |
| CONSENSUS | D | I | Q | M | T | Q | S | P | S | S | L | S | A | S | V | G | D | R | V | T | I | T | C | R | A | S |
| 21-225_148B6_LC | . | . | . | . | . | . | . | . | . | . | . | . | . | . | . | . | . | . | . | S | . | . | . | . | . | . |
| 21-225_14H8_LC | . | . | . | . | . | . | . | . | . | . | . | . | . | . | . | . | . | . | . | . | . | . | . | . | . | . |
| 21-225_153A1_LC | . | . | . | . | . | . | . | . | . | . | . | . | . | . | . | . | . | . | . | . | . | . | . | . | . | . |
| 21-225_157E4_LC | . | . | . | . | . | . | . | . | . | . | . | . | . | . | . | . | . | . | . | F | . | . | . | . | . | . |
| 21-225_157G5_LC | . | . | . | . | . | . | . | . | . | . | . | . | . | . | . | . | . | . | . | . | . | . | . | . | . | . |
| 21-225_157H1_LC | . | . | . | . | . | . | . | . | . | . | . | . | . | . | . | . | . | . | . | . | . | . | . | . | . | . |
| 21-225_157H10_LC | . | . | . | . | . | . | . | . | . | . | . | . | . | . | . | . | . | . | . | . | . | . | . | . | . | . |
| 21-225_158H12_LC | . | . | . | . | . | . | . | . | . | . | . | . | . | . | . | . | . | . | . | . | . | . | . | . | . | . |
| 21-225_159H11_LC | . | . | . | . | . | . | . | . | . | . | . | . | . | . | . | . | . | . | . | . | . | . | . | . | . | . |
| 21-225_15A2_LC | . | . | . | . | . | . | . | . | . | . | . | F | . | F | I | . | . | . | . | . | . | . | . | . | . | . |
| 21-225_15B1_LC | . | . | . | . | . | . | . | . | . | . | . | . | . | . | . | . | . | . | . | . | . | . | . | . | . | . |
| 21-225_160G1_LC | . | . | . | . | . | . | . | . | . | . | . | . | . | . | . | . | . | . | . | . | . | . | . | . | . | . |
| 21-225_160G3_LC | . | . | . | . | . | . | . | . | . | . | . | . | . | . | . | . | . | . | . | . | . | . | . | . | . | . |
| 21-225_163F1_LC | . | . | . | . | . | . | . | . | . | . | . | . | . | . | . | . | . | . | . | . | . | . | . | . | . | . |
| 21-225_167D12_LC | . | . | . | . | . | . | . | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . | . | . | . | . | . | . | . |
| 21-225_16B8_LC | . | . | . | . | . | . | . | . | . | . | . | . | . | . | . | . | . | . | . | . | . | . | . | . | . | . |
| 21-225_16C7_LC | . | . | . | . | . | . | . | . | . | . | . | . | . | . | . | . | . | . | . | . | . | . | . | . | . | . |
| 21-225_16G12_LC | . | . | . | . | . | . | . | . | . | . | . | . | . | . | . | . | . | . | . | . | . | . | . | . | . | . |
| 21-225_170E1_LC | . | . | . | . | . | . | . | . | . | . | . | . | . | . | E | . | . | . | . | . | . | . | . | . | . | . |
| 21-225_175C4_LC | . | . | . | . | . | . | . | . | . | . | . | . | . | . | . | . | . | . | . | . | . | . | . | . | . | . |
| 21-225_177D3_LC | . | . | . | . | S | . | . | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . | . | . | . | . | . | . | . |
| 21-225_17H6_LC | . | . | . | . | . | . | . | . | . | . | . | . | . | . | . | . | . | . | . | . | . | . | . | . | . | . |
| 21-225_181G2_LC | . | . | . | . | . | . | . | . | . | . | . | . | . | . | I | . | E | . | . | . | . | . | . | . | . | . |
| 21-225_184E7_LC | . | . | . | . | . | . | . | . | . | E | . | . | . | . | I | . | E | . | . | . | . | . | . | . | . | . |
| 21-225_185A1_LC | . | . | . | . | . | . | . | . | . | . | . | . | . | . | . | . | . | . | . | . | . | . | . | . | . | . |
| 21-225_185E10_LC | . | . | . | . | . | . | . | . | . | E | . | . | . | . | I | . | E | . | . | . | . | . | . | . | . | . |
| 21-225_188B9_LC | . | . | . | . | . | . | . | . | . | . | . | . | . | . | I | . | E | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | K_FR1 | | | | | | | | | | | | | |
| CONSENSUS | D | I | Q | M | T | Q | S | P | S | S | L | S | A | S | V | G | D | R | V | T | I | T | C | R | A | S |
| 21-225_188H5_LC | . | | | | | | | | | | | . | | . | ! | | ※ | | | | | | | | | |
| 21-225_18D11_LC | . | | | | | | | | | | | ⌇ | | ⌇ | | | | | | | | | | | | |
| 21-225_18G10_LC | . | . | | | | | | | | | | | | | | | | | | | | | | | | |
| 21-225_190A5_LC | . | | | | | | | | | | | | | | | | | | | | | | | | | |
| 21-225_190B1_LC | . | | | | | | | | | | | | | | | | | | | | | | | | | |
| 21-225_190F12_LC | . | | | | | | | | | | | | | | | | | | | | | | | | | |
| 21-225_190H9_LC | . | . | | | | | | | | | | | | | | | | | | | | | | | | |
| 21-225_192A2_LC | . | . | | | | | | | | | | | | | | | | | | | | | | | | ℵ |
| 21-225_192C10_LC | . | . | | | | | | | | | | | | | | | | | | | | | | | | |
| 21-225_192E4_LC | . | | | | | | | | | | | | | | | | | | | | | | | | | |
| 21-225_192H6_LC | . | | | | | | | | | | | | | | | | | | | | | | | | | |
| 21-225_193F8_LC | . | . | | ⌇ | | | | | | | | | | | | | | | | | | | | | | |
| 21-225_194H12_LC | . | . | | | | | | | | | | | | | | | | | | | | ※ | | | | |
| 21-225_197E11_LC | . | . | | | | | | | | | | | | | | | | | | | | | | | | |
| 21-225_20B9_LC | . | . | | | | | | | | | | | | | | | | | | | | | | | | |
| 21-225_20C11_LC | . | . | | | | | | | | | | | | | | | | | | | | | | | | |
| 21-225_20F10_LC | . | . | . | ⌊ | | | | | | | | | | | | | | | | | | | | | | |
| 21-225_20H7_LC | . | . | | | | | | | | | | | | | | | | | | | | | | | Y | |
| 21-225_20H9_LC | . | | | | | | | | | | | | | | | | | S | | | | | | | | |
| 21-225_211A6_LC | . | . | | | | | | | | | | | | | | | | S | | | | | | | | |
| 21-225_213B3_LC | . | . | | | | | | | | | | | | | | | | | | | | | | | | |
| 21-225_214C3_LC | . | . | | | | | | | | | | | | | | | | | | | | | | | | |
| 21-225_214E4_LC | . | . | | | | | | | | | | | | | | | | | | | | | | | | |
| 21-225_223D11_LC | . | . | | | | | | | | | | | | | | | | | | | | | | | | |
| 21-225_22B12_LC | . | . | | | | | | | | | | | | | | | | | | | | ⌇ | | | | |
| 21-225_23A11_LC | . | . | | | | | | | | | | | Y | | | | | | | | | | | | | |
| 21-225_23C7_LC | . | . | | | | | | | | | | | | | | | | | | | | | | | | |
| 21-225_23E7_LC | . | . | | | | | | | | | | | | | | | | | | | | | | | | |
| 21-225_23G1_LC | . | . | | | | | | | | | | | | | | | | | | | | | | | | |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_23G8_LC | . | . | . | . | . | . | . | . | . | . | . | . | . | . | . | . | . | . | . | . | . | . | . | . | . | . |
| 21-225_25C10_LC | . | . | . | . | . | . | . | . | . | . | . | . | . | . | . | . | . | . | . | . | . | . | . | . | . | . |
| 21-225_26G1_LC | . | . | . | . | . | . | . | . | . | . | . | . | . | . | . | . | . | . | . | . | . | . | . | . | . | . |
| 21-225_27H12_LC | . | . | . | . | . | . | . | . | . | . | . | . | . | . | . | . | . | . | . | . | . | . | . | . | . | . |
| 21-225_2A4_LC | . | . | . | . | . | . | . | . | . | . | . | F | . | . | . | . | . | . | . | . | . | . | . | . | . | . |
| 21-225_30A1_LC | . | . | . | . | . | . | . | . | . | . | . | . | . | . | . | . | . | . | . | . | . | . | . | . | . | . |
| 21-225_32B3_LC | . | . | . | . | . | . | . | . | . | . | . | . | . | I | . | . | . | . | . | . | . | . | . | . | . | . |
| 21-225_33A5_LC | . | . | . | . | . | . | . | . | . | . | . | . | . | . | . | . | . | . | . | . | . | . | . | . | . | . |
| 21-225_35H8_LC | . | . | . | . | . | . | . | . | . | . | . | . | . | . | . | . | . | . | . | . | . | . | . | . | . | . |
| 21-225_43A4_LC | . | . | . | . | . | . | . | . | . | . | . | . | . | . | . | . | . | . | . | . | . | . | . | . | . | . |
| 21-225_45D9_LC | . | . | . | . | . | . | . | . | . | . | . | . | . | . | . | . | . | . | . | . | . | . | . | . | . | . |
| 21-225_51C5_LC | . | . | . | . | . | . | . | . | . | . | . | . | . | . | . | . | . | . | . | . | . | . | . | . | . | . |
| 21-225_51D5_LC | . | . | . | . | . | . | . | . | . | . | . | . | . | . | . | . | . | . | . | . | . | . | . | . | . | . |
| 21-225_52E3_LC | . | . | . | . | . | . | . | . | . | . | . | . | . | . | . | . | . | . | . | . | . | . | . | . | . | . |
| 21-225_53E4_LC | . | . | . | . | . | . | . | . | . | . | . | . | . | . | . | . | . | . | . | . | . | . | . | . | . | . |
| 21-225_56F7_LC | . | . | L | . | . | . | . | . | . | . | . | . | . | . | . | . | . | . | . | . | . | . | . | . | . | . |
| 21-225_56H1_LC | . | . | . | . | . | . | . | . | . | . | . | . | . | . | . | . | . | . | . | . | . | . | . | . | . | . |
| 21-225_5A5_LC | . | . | . | . | . | . | . | . | . | . | . | . | . | . | . | . | . | . | . | . | . | . | . | . | . | . |
| 21-225_5E11_LC | V | . | . | . | . | . | . | . | . | . | . | . | . | . | . | . | . | . | . | . | . | . | . | . | . | . |
| 21-225_60A4_LC | . | . | . | . | . | . | . | . | . | . | . | . | . | . | . | . | . | . | . | . | . | . | . | . | . | . |
| 21-225_60F7_LC | . | . | . | . | . | . | . | . | . | . | . | . | . | . | . | . | . | . | . | . | . | S | . | . | . | . |
| 21-225_63H8_LC | . | . | . | . | . | . | . | . | . | . | . | . | . | . | . | . | . | . | . | . | . | . | . | . | . | . |
| 21-225_65D5_LC | . | . | . | . | . | . | . | . | . | . | . | . | . | . | . | . | . | . | . | . | . | . | . | . | . | . |
| 21-225_66H11_LC | . | . | . | . | . | . | F | . | . | . | . | F | . | F | . | . | . | . | . | . | . | . | . | F | . | . |
| 21-225_68E6_LC | . | . | . | . | . | . | . | . | . | . | . | . | . | . | . | . | . | . | . | . | . | . | . | . | . | . |
| 21-225_7B1_LC | . | . | . | . | . | . | S | . | . | . | . | . | . | . | . | . | . | . | . | . | . | . | . | . | . | . |
| 21-225_96C6_LC | . | . | . | . | . | . | . | . | . | . | . | . | . | . | . | . | . | . | . | . | . | . | . | . | . | . |
| 21-225_9C8_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_148B6_LC | . | . | . | . | . | . | . | . | . | . | . | . | X | . | . | . | . | . | . | . | . | . | . | . | . | . |
| 21-225_14H8_LC | . | . | . | . | . | . | . | . | . | . | . | . | Y | . | . | . | . | . | . | . | . | . | . | . | . | . |
| 21-225_153A1_LC | . | . | . | V | . | . | . | . | . | . | . | . | . | . | . | . | . | . | . | . | . | . | . | . | . | . |
| 21-225_157E4_LC | . | . | . | . | . | . | . | . | . | . | . | . | . | . | . | . | . | . | . | . | . | . | . | X | . | . |
| 21-225_157G5_LC | . | . | . | . | . | N | . | . | . | . | . | . | . | . | . | . | . | . | . | . | . | . | . | . | . | . |
| 21-225_157H1_LC | . | . | D | . | . | . | . | . | . | . | . | . | . | . | . | V | . | . | . | . | X | . | . | . | . | . |
| 21-225_157H10_LC | . | . | . | . | . | T | . | . | . | . | . | . | . | . | . | . | . | . | . | . | . | . | . | . | . | . |
| 21-225_158H12_LC | . | . | . | . | . | N | . | . | . | . | . | . | . | . | . | . | . | . | . | . | . | . | . | . | . | . |
| 21-225_159H11_LC | . | . | . | . | . | . | . | . | . | . | . | . | X | . | . | V | . | . | . | . | . | . | . | . | . | . |
| 21-225_15A2_LC | . | . | . | . | . | . | . | . | . | . | . | . | . | . | . | . | . | . | . | . | . | . | . | . | . | . |
| 21-225_15B1_LC | . | . | . | . | . | . | . | . | . | . | . | . | . | . | . | . | . | . | . | . | . | . | . | . | . | . |
| 21-225_160G1_LC | . | . | D | . | . | N | . | . | . | . | . | . | . | . | . | . | . | . | . | . | . | . | . | . | . | . |
| 21-225_160G3_LC | . | . | D | . | . | N | . | . | . | . | . | . | . | . | . | . | . | . | . | . | X | . | . | . | . | . |
| 21-225_163F1_LC | . | . | D | . | . | . | . | . | . | . | . | . | . | . | . | . | . | . | . | . | . | . | . | . | . | . |
| 21-225_167D12_LC | . | . | . | . | . | N | . | . | . | . | . | . | . | . | . | . | . | . | . | . | . | . | . | . | . | . |
| 21-225_169D10_LC | . | . | D | . | . | . | . | . | . | . | . | . | . | . | . | . | . | . | . | . | . | . | . | . | . | . |
| 21-225_16B8_LC | . | . | . | . | . | N | . | . | . | . | . | . | . | . | . | . | . | . | . | . | . | . | . | . | . | L |
| 21-225_16C7_LC | . | . | . | . | . | . | . | . | . | . | . | . | . | . | . | . | . | . | . | . | . | . | . | . | . | . |
| 21-225_16G12_LC | . | . | D | . | . | . | . | . | . | . | . | . | . | . | . | . | . | . | . | . | . | . | . | . | . | . |
| 21-225_170E1_LC | . | . | . | . | . | . | . | . | . | . | . | . | . | . | . | . | . | . | . | . | . | . | . | . | . | . |
| 21-225_175C4_LC | . | . | . | . | . | G | . | . | . | . | . | . | . | . | . | . | . | . | . | . | . | . | . | . | . | . |
| 21-225_177D3_LC | . | . | . | . | . | T | . | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . | . | . | . | . | . | . | . |
| 21-225_17H6_LC | . | X | D | . | . | . | . | . | . | . | . | . | . | . | . | . | . | . | . | . | . | . | . | . | . | . |
| 21-225_181G2_LC | . | . | . | . | . | . | . | . | . | . | . | . | . | . | . | . | . | I | . | . | . | . | . | T | . | . |
| 21-225_184E7_LC | . | . | . | . | . | . | . | . | . | . | . | . | . | . | . | . | . | I | . | . | . | . | . | T | . | . |
| 21-225_185A1_LC | . | . | . | . | . | . | . | . | . | . | . | . | . | . | . | . | . | . | . | . | T | . | . | . | . | . |
| 21-225_185E10_LC | . | . | . | . | . | . | . | . | . | . | . | . | . | . | . | . | . | I | . | . | . | . | . | T | . | . |
| 21-225_188B9_LC | . | . | . | . | . | . | . | . | . | . | . | . | . | . | . | . | . | L | . | . | . | . | . | T | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_188H5_LC | . | . | . | . | . | . | . | . | . | . | . | . | . | . | . | . | . | L | . | . | . | . | . | T | . | . |
| 21-225_18D11_LC | . | . | . | . | . | . | . | . | . | . | . | . | Y | . | . | . | . | . | . | . | . | . | . | . | . | . |
| 21-225_18G10_LC | . | . | . | D | . | . | . | . | . | . | . | . | Y | . | . | . | . | . | . | . | . | . | . | . | . | . |
| 21-225_190A5_LC | . | . | . | . | . | G | . | . | . | . | . | . | . | H | . | . | . | . | . | . | . | . | . | . | . | . |
| 21-225_190B1_LC | . | . | . | . | . | P | . | . | . | . | . | . | . | . | . | . | . | . | . | . | . | . | . | . | . | . |
| 21-225_190F12_LC | . | . | . | . | . | G | . | . | . | . | . | . | K | . | . | . | . | . | . | . | . | . | . | . | . | . |
| 21-225_190H9_LC | . | . | . | . | . | G | . | . | . | . | . | . | K | . | . | . | . | . | . | . | . | . | . | . | . | L |
| 21-225_192A2_LC | . | . | . | . | . | N | . | . | . | . | . | . | . | . | . | . | . | . | . | . | . | . | . | . | . | . |
| 21-225_192C10_LC | . | . | . | . | . | G | . | . | . | . | . | . | K | . | . | . | . | . | . | . | . | . | . | . | . | . |
| 21-225_192E4_LC | . | . | . | . | . | G | . | . | . | . | . | . | . | . | . | . | . | . | . | . | . | . | . | . | . | . |
| 21-225_192H6_LC | . | . | . | . | . | G | . | . | . | . | . | . | . | H | . | . | . | . | . | . | . | . | . | . | . | . |
| 21-225_193F8_LC | . | . | . | . | V | . | . | . | . | . | . | . | . | H | . | . | . | . | . | . | . | . | . | R | . | . |
| 21-225_194H12_LC | . | . | . | . | . | G | . | . | . | . | . | . | K | . | . | . | . | . | . | . | . | . | . | . | . | L |
| 21-225_197E11_LC | . | . | . | . | . | N | . | . | . | . | . | . | K | . | . | . | S | . | . | . | . | . | . | . | . | . |
| 21-225_20B9_LC | . | . | . | . | . | . | . | . | . | . | . | . | . | . | . | . | . | . | . | . | . | . | . | . | . | . |
| 21-225_20C11_LC | . | . | . | D | . | . | . | . | . | . | . | . | . | . | . | . | . | . | . | . | . | . | . | . | . | . |
| 21-225_20F10_LC | . | . | . | . | . | N | . | . | . | . | . | . | . | . | . | . | . | . | . | . | . | . | . | . | . | . |
| 21-225_20H7_LC | . | . | . | . | . | N | . | . | . | . | . | . | . | . | . | . | V | . | . | . | . | . | . | . | . | . |
| 21-225_20H8_LC | . | . | . | . | . | . | . | . | . | . | . | . | . | . | . | . | . | . | . | . | . | . | . | . | . | . |
| 21-225_211A6_LC | . | . | . | . | . | G | . | . | . | . | . | . | R | . | . | . | . | . | . | . | . | . | . | . | . | . |
| 21-225_213B3_LC | . | . | . | . | . | G | . | . | . | . | . | . | R | . | . | . | . | . | . | . | . | . | . | . | . | . |
| 21-225_214C3_LC | . | . | . | . | . | G | . | . | . | . | . | . | . | . | . | . | . | . | . | . | . | . | . | . | V | . |
| 21-225_214E4_LC | . | . | . | . | . | . | . | . | . | . | . | . | . | . | . | . | . | . | . | . | . | . | . | . | . | . |
| 21-225_223D11_LC | . | . | . | . | . | . | . | . | . | . | . | . | . | . | . | . | . | . | . | . | . | . | . | . | . | H |
| 21-225_22B12_LC | . | . | . | D | . | . | . | . | . | . | . | . | . | . | . | . | . | . | . | E | . | . | . | . | . | H |
| 21-225_23A11_LC | . | . | . | D | . | . | . | . | . | . | . | . | . | . | . | . | . | . | . | . | . | . | . | . | . | . |
| 21-225_23C7_LC | . | . | . | . | . | G | . | . | . | . | . | . | I | . | . | . | . | . | . | . | . | . | . | . | . | . |
| 21-225_23E7_LC | . | . | . | . | . | . | . | . | . | . | . | . | . | . | . | . | . | . | . | I | . | . | . | . | . | . |
| 21-225_23G1_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_23G8_LC | . | . | . | . | . | X | . | . | . | . | . | . | . | . | . | . | . | . | . | . | . | . | . | . | . | . |
| 21-225_25C10_LC | . | . | . | . | . | . | . | . | . | . | . | . | . | . | . | . | . | . | . | . | . | . | . | . | . | . |
| 21-225_26G1_LC | . | . | . | D | . | . | . | . | . | . | . | . | . | . | . | . | . | . | . | . | . | . | . | . | . | . |
| 21-225_27H12_LC | . | . | . | . | . | . | . | . | . | . | . | . | . | . | . | . | . | . | . | . | . | . | . | . | . | . |
| 21-225_2A4_LC | . | . | . | . | . | . | . | . | . | . | . | . | . | . | . | . | . | . | . | . | . | . | . | . | . | . |
| 21-225_30A1_LC | . | . | . | . | . | . | . | . | . | . | . | . | . | . | . | . | . | . | . | . | . | . | . | . | . | . |
| 21-225_32B3_LC | . | . | . | . | . | . | . | . | . | . | . | . | . | . | . | . | . | . | . | . | . | . | . | . | . | . |
| 21-225_33A5_LC | . | . | . | . | . | . | . | . | . | . | . | . | . | . | . | . | . | . | . | . | . | . | . | . | . | . |
| 21-225_35H8_LC | . | . | . | A | . | . | . | . | . | . | . | . | . | . | . | . | . | . | . | . | . | . | . | . | . | . |
| 21-225_43A4_LC | . | . | . | . | . | N | . | . | . | . | . | . | . | . | . | . | . | . | . | . | . | . | . | . | . | . |
| 21-225_45D9_LC | . | . | . | . | . | N | . | . | . | . | . | . | . | . | . | . | . | . | . | . | . | . | . | . | . | . |
| 21-225_51C5_LC | . | . | . | . | . | . | . | . | . | . | . | . | . | . | . | . | . | . | . | . | . | . | . | S | . | . |
| 21-225_51D5_LC | . | . | . | . | . | N | . | . | . | . | . | . | . | . | . | . | . | . | . | . | . | . | . | S | . | . |
| 21-225_52E3_LC | . | . | . | . | . | . | . | . | . | . | . | . | . | . | . | . | . | . | . | . | . | . | . | . | . | . |
| 21-225_53E4_LC | . | . | V | . | . | . | . | . | . | . | . | . | . | . | . | . | . | . | . | . | . | . | . | . | . | . |
| 21-225_56F7_LC | . | . | . | . | . | . | . | . | . | . | . | . | T | . | . | . | . | . | . | . | T | . | T | . | . | . |
| 21-225_56H1_LC | . | . | . | . | . | . | . | . | . | . | . | . | X | . | . | . | . | . | . | . | . | . | . | . | . | . |
| 21-225_5A6_LC | . | . | . | . | . | . | . | . | . | . | . | . | . | . | . | . | . | . | . | . | . | . | . | . | . | . |
| 21-225_5E11_LC | . | . | . | . | . | . | . | . | . | . | . | . | . | . | . | . | . | . | . | . | . | . | . | . | . | . |
| 21-225_60A4_LC | . | . | . | . | . | . | . | . | . | . | . | . | . | . | . | . | . | . | . | . | . | . | . | . | . | . |
| 21-225_60F7_LC | . | . | . | V | . | X | . | . | . | . | . | . | . | . | . | V | . | V | . | . | . | . | . | . | . | . |
| 21-225_63H8_LC | . | . | . | . | . | . | . | . | . | . | . | . | . | . | . | . | . | . | . | . | . | . | . | . | . | X |
| 21-225_65D5_LC | . | . | . | D | . | N | . | . | . | . | . | . | . | . | . | . | . | . | . | . | . | . | . | . | . | . |
| 21-225_66H11_LC | . | . | . | . | . | . | . | . | . | . | . | . | . | . | . | . | . | . | . | . | . | . | . | . | . | . |
| 21-225_68E6_LC | . | . | . | . | . | . | . | . | . | . | . | . | Y | . | . | . | . | . | . | . | . | . | . | X | . | . |
| 21-225_7B1_LC | . | . | . | D | . | . | . | . | . | . | . | . | . | X | . | V | . | . | . | . | . | . | . | . | . | . |
| 21-225_96C6_LC | . | . | . | . | . | . | . | . | . | . | . | . | . | . | . | . | . | . | . | . | . | . | . | . | . | . |
| 21-225_9C8_LC | . | . | . | . | . | . | . | . | . | . | . | . | Y | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
|  |  |  |  |  |  |  |  |  |  |  |  |  | K_CDR2 |  |  |  |  |  |  |  |  |  |  |  |  |  |
| CONSENSUS | K | S | L | I | Y | A | - | - | - | - | - | - | - | - | A | S | S | L | Q | S | G | V | P | S | K | F |
| 21-225_148B6_LC | . | . | . | . | P | . | . | . | . | . | . | . | . | . | . | . | . | . | . | . | . | . | . | . | . | . |
| 21-225_14H8_LC | . | . | . | . | . | V | . | . | . | . | . | . | . | . | . | . | . | . | . | . | . | . | . | . | . | . |
| 21-225_153A1_LC | . | . | . | . | S | . | . | . | . | . | . | . | . | . | . | . | . | . | P | . | . | . | . | . | N | . |
| 21-225_157E4_LC | . | . | . | . | . | . | . | . | . | . | . | . | . | . | . | . | . | . | P | . | . | . | . | . | Q | . |
| 21-225_157G5_LC | S | . | . | . | . | . | . | . | . | . | . | . | . | . | . | . | . | . | . | . | . | . | . | . | Q | . |
| 21-225_157H1_LC | X | . | . | . | . | . | . | . | . | . | . | . | . | . | . | . | . | . | . | . | . | . | . | . | . | . |
| 21-225_157H10_LC | . | . | . | . | . | T | . | . | . | . | . | . | . | . | . | . | N | . | . | . | . | . | . | . | . | . |
| 21-225_158H12_LC | . | . | . | . | S | . | . | . | . | . | . | . | . | . | . | . | . | . | . | . | . | . | . | . | . | . |
| 21-225_159H11_LC | . | . | . | . | . | . | . | . | . | . | . | . | . | . | . | . | . | . | . | . | . | . | . | . | . | . |
| 21-225_15A2_LC | . | . | . | . | P | . | . | . | . | . | . | . | . | . | . | . | . | . | . | . | . | . | . | . | . | . |
| 21-225_15B1_LC | . | . | . | . | S | . | . | . | . | . | . | . | . | . | . | . | . | . | . | . | . | . | . | . | N | . |
| 21-225_160G1_LC | T | . | . | . | . | . | . | . | . | . | . | . | . | . | S | . | . | . | . | . | . | . | . | . | . | . |
| 21-225_160G3_LC | T | . | . | . | . | . | . | . | . | . | . | . | . | . | S | . | . | . | . | . | . | . | . | . | . | . |
| 21-225_163F1_LC | . | . | . | . | . | . | . | . | . | . | . | . | . | . | . | . | . | . | . | . | . | . | . | . | . | . |
| 21-225_167D12_LC | S | . | . | . | . | . | . | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . | . | . | . | . | . | . | . |
| 21-225_16B8_LC | . | . | . | . | N | . | . | . | . | . | . | . | . | . | . | . | . | . | V | . | . | . | . | . | . | . |
| 21-225_16C7_LC | . | . | . | . | S | . | . | . | . | . | . | . | . | . | . | . | . | . | . | . | . | . | . | . | . | . |
| 21-225_16G12_LC | . | . | . | . | . | . | . | . | . | . | . | . | . | . | . | . | . | . | . | . | . | . | . | . | . | . |
| 21-225_170E1_LC | . | . | . | . | . | . | . | . | . | . | . | . | . | . | . | . | . | . | . | . | . | . | . | . | . | . |
| 21-225_175C4_LC | . | . | . | . | . | . | . | . | . | . | . | . | . | . | . | . | S | . | . | . | P | . | . | . | . | . |
| 21-225_177D3_LC | . | . | . | . | . | . | . | . | . | . | . | . | . | . | . | . | . | . | . | . | . | . | . | . | . | . |
| 21-225_17H5_LC | . | . | . | . | N | . | . | . | . | . | . | . | . | . | . | . | . | . | . | . | . | . | . | . | . | . |
| 21-225_17H6_LC | . | . | . | . | S | T | . | . | . | . | . | . | . | . | . | . | . | . | . | . | . | . | . | . | . | . |
| 21-225_181G2_LC | . | . | . | . | . | . | . | . | . | . | . | . | . | . | . | . | . | . | . | . | . | . | S | , | P | . |
| 21-225_184E7_LC | . | . | . | . | . | . | . | . | . | . | . | . | . | . | . | . | . | . | . | . | . | . | S | , | P | . |
| 21-225_185A1_LC | . | . | . | . | S | V | . | . | . | . | . | . | . | . | . | . | . | . | . | . | . | . | . | . | P | . |
| 21-225_185E10_LC | . | . | . | . | . | . | . | . | . | . | . | . | . | . | . | . | . | . | . | . | . | . | S | , | P | . |
| 21-225_188B9_LC | . | . | . | . | . | . | . | . | . | . | . | . | . | . | . | . | . | . | . | . | . | . | S | , | P | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | S | L | I | Y | A | - | - | - | - | - | - | - | - | A | S | S | L | Q | S | G | V | P | S | K | F |
| 21-225_188H5_LC | . | . | . | . | . | . | . | . | . | . | . | . | . | . | . | . | . | . | . | . | . | . | S | . | ※ | |
| 21-225_18D11_LC | . | . | . | . | V | . | . | . | . | . | . | . | . | . | . | . | . | . | . | . | . | . | . | . | . | |
| 21-225_18G10_LC | . | . | . | . | V | . | . | . | . | . | . | . | . | . | . | . | . | . | . | . | . | . | . | . | . | |
| 21-225_190A5_LC | . | . | . | ※ | . | . | . | . | . | . | . | . | . | . | . | . | . | . | . | . | . | . | . | . | . | |
| 21-225_190B1_LC | . | . | . | . | V | . | . | . | . | . | . | . | . | . | . | . | . | . | E | . | . | . | . | . | . | |
| 21-225_190F12_LC | . | . | . | . | . | . | . | . | . | . | . | . | . | . | . | . | . | . | . | . | . | . | . | . | . | |
| 21-225_190H9_LC | . | . | . | . | . | . | . | . | . | . | . | . | . | . | . | . | . | . | . | . | . | . | . | . | . | |
| 21-225_192A2_LC | . | . | . | . | . | . | . | . | . | . | . | . | . | . | . | . | . | . | . | . | . | . | . | . | . | |
| 21-225_192C10_LC | . | . | . | . | . | . | . | . | . | . | . | . | . | . | . | . | . | . | . | . | . | . | . | . | . | |
| 21-225_192E4_LC | . | . | . | . | V | . | . | . | . | . | . | . | . | . | V | . | . | . | . | . | . | . | . | . | ※ | |
| 21-225_192H6_LC | . | . | . | . | . | . | . | . | . | . | . | . | . | . | . | . | . | . | . | . | . | . | . | . | . | |
| 21-225_193F8_LC | . | . | . | . | . | . | . | . | . | . | . | . | . | . | . | . | . | . | . | . | . | . | . | . | . | |
| 21-225_194H12_LC | . | . | . | . | . | . | . | . | . | . | . | . | . | . | . | . | . | . | . | . | . | . | . | . | . | |
| 21-225_197E11_LC | . | . | . | . | . | . | . | . | . | . | . | . | . | . | . | . | . | V | . | . | . | . | . | . | . | |
| 21-225_20B9_LC | . | . | . | . | V | . | . | . | . | . | . | . | . | . | . | . | . | . | . | . | . | . | . | . | . | |
| 21-225_20C11_LC | . | . | . | . | . | . | . | . | . | . | . | . | . | . | . | . | . | . | . | . | . | . | . | . | . | |
| 21-225_20F10_LC | . | . | . | . | . | . | . | . | . | . | . | . | . | . | . | . | . | . | . | . | . | . | . | . | ※ | |
| 21-225_20H7_LC | . | ※ | . | . | . | . | . | . | . | . | . | . | . | . | . | . | . | . | . | . | . | . | . | . | . | |
| 21-225_20H9_LC | . | . | . | . | S | . | . | . | . | . | . | . | . | . | . | . | . | . | . | . | . | . | . | . | . | |
| 21-225_211A6_LC | . | . | . | . | . | . | . | . | . | . | . | . | . | . | . | . | . | . | L | . | . | . | . | . | . | |
| 21-225_213B3_LC | . | . | . | . | S | . | . | . | . | . | . | . | . | . | . | . | . | V | L | . | . | . | . | . | . | |
| 21-225_214C3_LC | . | . | . | . | . | . | . | . | . | . | . | . | . | . | . | . | . | . | . | . | . | . | . | . | . | |
| 21-225_214E4_LC | . | . | . | . | . | . | . | . | . | . | . | . | . | . | . | . | . | . | . | . | . | . | . | . | . | |
| 21-225_223D11_LC | . | . | . | . | . | . | . | . | . | . | . | . | . | . | . | . | T | . | . | . | . | . | . | . | . | |
| 21-225_22B12_LC | . | . | . | . | Q | . | . | . | . | . | . | . | . | . | . | . | . | . | ※ | . | . | . | . | . | . | |
| 21-225_23A11_LC | . | ※ | . | . | . | . | . | . | . | . | . | . | . | . | . | . | T | . | . | . | . | . | . | . | . | |
| 21-225_23C7_LC | . | . | . | . | S | . | . | . | . | . | . | . | . | . | . | . | . | . | . | . | . | . | . | . | . | |
| 21-225_23E7_LC | . | . | . | . | . | . | . | . | . | . | . | . | . | . | . | . | . | . | . | . | . | . | . | . | . | |
| 21-225_23G1_LC | . | . | . | . | S | . | . | . | . | . | . | . | . | . | . | . | . | . | . | . | . | . | . | . | Q | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | S | L | I | Y | A | - | - | - | - | - | - | - | - | A | S | S | L | Q | S | G | V | P | S | K | F |
| 21-225_23G8_LC | . | . | . | . | . | | | | | | | | | | . | . | . | . | . | | . | . | . | . | N | . |
| 21-225_25C10_LC | . | . | . | . | . | | | | | | | | | | . | . | . | . | . | | . | F | . | . | . | . |
| 21-225_26G1_LC | . | . | . | . | . | | | | | | | | | | . | . | . | . | R | | . | . | . | . | N | . |
| 21-225_27H12_LC | . | . | . | . | N | G | | | | | | | | | . | . | . | . | . | | . | . | . | . | . | . |
| 21-225_2A4_LC | . | R | . | . | . | T | | | | | | | | | . | . | . | . | . | | . | . | . | . | . | . |
| 21-225_30A1_LC | . | . | . | . | S | . | | | | | | | | | . | . | . | . | . | T | . | . | . | . | . | . |
| 21-225_32B3_LC | R | . | . | . | . | . | | | | | | | | | . | . | . | . | . | | . | . | . | . | T | . |
| 21-225_33A5_LC | . | . | . | . | S | . | | | | | | | | | . | . | . | . | . | | . | . | . | . | . | . |
| 21-225_35H8_LC | . | . | . | . | . | G | | | | | | | | | . | . | G | . | . | | . | . | . | . | . | . |
| 21-225_43A4_LC | . | . | . | . | N | . | | | | | | | | | . | . | . | . | . | | . | . | . | . | R | . |
| 21-225_45D9_LC | . | . | . | . | N | . | | | | | | | | | . | . | . | . | . | | . | . | . | . | R | . |
| 21-225_51C5_LC | . | . | . | . | . | . | | | | | | | | | . | . | . | . | R | | . | . | . | . | Q | . |
| 21-225_51D5_LC | . | . | . | . | . | . | | | | | | | | | . | . | . | . | R | | . | . | . | . | Q | . |
| 21-225_52E3_LC | . | . | . | . | N | . | | | | | | | | | . | . | . | . | . | | . | . | . | . | . | . |
| 21-225_53E4_LC | . | . | . | . | S | . | | | | | | | | | . | . | . | . | . | | . | . | . | . | . | . |
| 21-225_56F7_LC | . | . | . | . | . | . | | | | | | | | | . | . | . | . | . | G | . | . | . | . | . | . |
| 21-225_56H1_LC | Q | . | . | M | S | . | | | | | | | | | . | . | G | . | . | | . | . | . | . | . | . |
| 21-225_5A5_LC | . | . | . | . | S | . | | | | | | | | | . | . | . | . | . | | . | . | . | . | . | . |
| 21-225_5E11_LC | . | . | . | . | N | . | | | | | | | | | . | . | . | V | . | | . | . | . | . | . | . |
| 21-225_60A4_LC | . | . | . | . | . | . | | | | | | | | | . | . | . | . | . | | . | . | . | . | . | . |
| 21-225_60F7_LC | . | . | . | . | . | . | | | | | | | | | . | . | . | . | . | | . | . | . | . | T | . |
| 21-225_63H8_LC | . | . | . | . | . | . | | | | | | | | | . | . | . | . | . | | . | . | . | . | . | . |
| 21-225_65D5_LC | R | . | . | . | . | . | | | | | | | | | . | . | . | . | R | | . | . | . | . | Q | . |
| 21-225_66H11_LC | . | . | . | . | . | G | | | | | | | | | F | N | . | . | R | | . | . | . | . | . | . |
| 21-225_68E6_LC | . | . | . | . | . | V | | | | | | | | | . | . | . | . | . | | . | . | . | . | R | . |
| 21-225_7B1_LC | . | . | . | . | F | . | | | | | | | | | . | . | . | . | . | | . | . | . | . | Q | . |
| 21-225_96C6_LC | . | . | . | . | . | . | | | | | | | | | . | . | . | . | . | | . | . | . | . | . | . |
| 21-225_9C8_LC | . | . | . | . | . | V | | | | | | | | | . | . | . | . | . | | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | K_FR3 | | | | | | | | | | | | |
| CONSENSUS | S | G | S | G | S | G | - | - | T | D | F | T | L | T | I | S | S | L | Q | P | E | D | F | A | T | Y |
| 21-225_148B6_LC | . | . | . | . | . | . | . | . | . | . | . | . | . | . | . | . | . | . | . | . | . | . | . | . | . | . |
| 21-225_14H8_LC | . | . | . | . | . | . | . | . | . | . | . | . | . | . | . | . | . | . | . | . | . | . | . | . | . | . |
| 21-225_153A1_LC | . | . | . | . | . | . | . | . | . | . | . | . | . | . | . | . | . | . | . | . | . | . | . | . | . | . |
| 21-225_157E4_LC | . | . | . | . | . | . | . | . | . | . | . | . | . | . | . | N | . | . | . | . | . | . | . | . | . | . |
| 21-225_157G5_LC | . | . | . | . | . | . | . | . | . | . | . | . | . | . | . | . | . | . | . | . | . | . | . | . | . | . |
| 21-225_157H1_LC | . | . | . | . | . | . | . | . | . | . | . | . | . | . | . | . | . | . | . | . | . | . | . | . | . | . |
| 21-225_157H10_LC | . | . | . | . | . | . | . | . | . | . | . | . | . | . | . | . | . | . | . | . | . | . | . | . | . | . |
| 21-225_158H12_LC | . | . | . | . | . | . | . | . | . | . | . | . | . | S | . | . | . | . | . | . | . | . | . | . | . | . |
| 21-225_159H11_LC | . | . | . | . | . | . | . | . | . | . | . | . | . | . | . | . | N | . | . | . | . | . | . | . | . | . |
| 21-225_15A2_LC | . | . | . | S | . | . | . | . | . | . | . | . | S | S | . | . | . | . | . | . | . | . | . | . | N | . |
| 21-225_15B1_LC | . | . | . | . | . | . | . | . | . | . | . | . | . | . | . | . | . | . | . | . | . | . | . | . | . | . |
| 21-225_160G1_LC | . | . | . | . | . | . | . | . | . | . | . | . | . | . | . | . | . | . | . | . | . | . | . | . | . | . |
| 21-225_160G3_LC | . | . | . | . | . | . | . | . | . | . | . | . | . | . | . | . | . | . | . | . | . | . | . | . | . | . |
| 21-225_163F1_LC | . | . | . | . | . | . | . | . | . | . | . | . | . | . | . | . | . | . | . | . | . | . | . | . | A | . |
| 21-225_167D12_LC | . | . | . | . | . | . | . | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . | . | . | . | . | . | . | . |
| 21-225_16B8_LC | . | . | . | . | . | . | . | . | . | . | . | . | . | . | . | . | . | . | . | . | . | . | . | . | . | . |
| 21-225_16C7_LC | . | . | . | . | . | . | . | . | . | . | . | . | . | . | . | . | . | . | . | . | . | . | . | . | . | . |
| 21-225_16G12_LC | S | . | . | . | . | . | . | . | . | . | . | . | . | . | . | N | . | . | . | . | . | . | . | . | S | . |
| 21-225_170E1_LC | . | . | . | . | . | . | . | . | . | . | . | . | . | . | . | . | . | . | . | . | . | . | . | . | . | . |
| 21-225_175C4_LC | . | . | . | . | . | . | . | . | . | . | . | . | . | . | . | . | . | . | . | . | . | . | . | . | . | . |
| 21-225_177D3_LC | . | . | . | . | . | . | . | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . | . | . | . | . | . | . | . |
| 21-225_17H6_LC | . | . | . | . | . | . | . | . | . | . | . | . | . | . | . | . | . | . | . | . | . | . | . | . | I | . |
| 21-225_181G2_LC | . | . | . | S | . | . | . | . | . | . | . | . | . | . | . | . | . | . | . | . | . | . | . | . | . | . |
| 21-225_184E7_LC | . | . | . | S | . | . | . | . | . | . | . | . | I | . | . | . | . | V | . | R | . | . | . | . | . | . |
| 21-225_185A1_LC | A | . | . | . | . | . | . | . | . | . | . | . | . | . | . | . | . | . | . | . | . | . | . | . | . | . |
| 21-225_185E10_LC | . | . | . | S | . | . | . | . | . | . | . | . | . | . | . | . | . | . | . | . | . | . | . | . | . | . |
| 21-225_188B9_LC | . | . | . | S | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_188H5_LC | . | . | . | . | P | . | . | . | . | . | . | . | . | . | . | . | . | . | . | . | . | . | . | . | . | . |
| 21-225_18D11_LC | . | . | . | . | P | . | . | . | . | . | . | . | . | . | . | . | . | . | . | . | . | . | . | . | . | . |
| 21-225_18G10_LC | G | . | . | . | P | . | . | . | . | . | . | . | . | . | . | . | . | . | . | . | . | . | . | . | . | . |
| 21-225_190A5_LC | . | . | . | . | . | . | . | . | . | . | . | . | . | . | . | . | . | . | . | . | . | . | . | . | . | . |
| 21-225_190B1_LC | . | . | . | . | . | . | . | . | . | E | . | . | . | . | . | . | . | . | . | . | . | . | . | G | . | . |
| 21-225_190F12_LC | . | . | . | . | . | . | . | . | . | . | . | . | . | . | . | . | . | . | . | D | . | . | . | . | . | . |
| 21-225_190H9_LC | . | . | . | . | . | . | . | . | . | . | . | . | . | . | . | . | . | . | . | D | . | . | . | . | . | . |
| 21-225_192A2_LC | . | . | . | . | . | . | . | . | . | . | . | . | . | . | . | . | . | . | . | . | . | . | . | . | . | . |
| 21-225_192C10_LC | . | . | . | . | . | . | . | . | . | . | . | . | . | . | . | . | . | . | . | D | . | . | . | . | . | . |
| 21-225_192E4_LC | . | . | . | . | . | . | . | . | . | . | . | . | . | . | . | . | . | . | . | . | . | . | . | . | . | . |
| 21-225_192H6_LC | . | . | R | . | . | . | . | . | . | . | . | S | . | . | . | . | . | . | . | . | . | . | . | . | . | . |
| 21-225_193F8_LC | . | . | . | . | . | . | . | A | . | . | . | . | . | . | . | . | . | . | . | . | . | . | . | . | . | . |
| 21-225_194H12_LC | . | . | . | . | . | . | . | . | . | . | . | . | . | . | . | . | . | . | . | D | . | . | . | . | . | . |
| 21-225_197E11_LC | . | . | . | . | . | . | . | . | . | . | . | . | . | . | . | . | R | . | . | D | . | . | . | . | . | . |
| 21-225_20B9_LC | . | . | . | . | . | . | . | . | . | . | . | . | . | . | . | . | . | . | . | . | . | . | . | . | . | . |
| 21-225_20C11_LC | R | . | . | . | . | . | . | . | . | . | . | . | . | . | . | . | . | . | . | . | . | . | . | . | . | . |
| 21-225_20F10_LC | . | . | . | . | P | . | . | . | . | . | . | . | . | . | . | . | . | . | . | . | . | . | . | . | . | P |
| 21-225_20H7_LC | . | . | . | . | . | . | . | . | . | . | . | . | . | . | . | . | . | . | . | . | . | . | . | . | . | . |
| 21-225_20H9_LC | . | . | . | . | . | . | . | . | . | . | . | . | . | . | . | . | . | . | . | . | . | . | . | . | . | . |
| 21-225_211A6_LC | . | . | . | . | . | . | . | . | . | . | . | . | . | . | . | . | . | . | . | . | . | . | . | . | . | . |
| 21-225_213B3_LC | . | . | . | . | . | . | . | . | . | . | . | . | . | . | . | . | . | . | . | . | . | . | . | . | . | . |
| 21-225_214C3_LC | . | . | . | . | . | . | . | . | . | . | . | . | . | . | . | . | . | . | . | . | . | . | . | T | . | . |
| 21-225_214E4_LC | . | . | . | . | . | . | . | . | . | . | . | . | . | . | . | . | . | . | . | . | . | . | . | . | . | . |
| 21-225_223D11_LC | . | . | R | . | . | . | . | . | . | . | N | . | . | . | . | N | . | . | . | . | . | . | . | . | . | . |
| 21-225_22B12_LC | . | . | . | . | . | . | . | . | . | . | N | . | . | . | . | . | . | . | . | . | . | . | L | . | . | . |
| 21-225_23A11_LC | . | . | . | . | . | . | . | . | . | . | . | . | . | . | . | . | . | . | . | . | . | . | . | . | . | . |
| 21-225_23C7_LC | . | . | . | . | . | . | . | . | . | S | . | . | . | . | . | . | . | . | . | . | . | . | . | . | . | . |
| 21-225_23E7_LC | . | . | . | . | . | . | . | . | . | . | . | . | . | . | . | . | . | . | . | . | . | . | . | . | . | . |
| 21-225_23G1_LC | . | . | . | . | P | . | . | . | . | . | . | . | . | . | . | . | . | . | . | . | . | . | . | . | . | . |

4689

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | D | F | T | L | T | I | S | S | L | Q | P | E | D | F | A | T | Y |
| 21-225_23G8_LC | . | . | . | . | . | . | | | . | . | . | . | . | . | . | . | . | . | . | . | . | . | . | . | . | . |
| 21-225_25C10_LC | . | . | . | . | . | . | | | . | . | . | . | . | . | . | . | . | . | . | . | . | . | . | . | . | . |
| 21-225_26G1_LC | . | . | . | . | . | . | | | . | . | . | . | . | . | . | . | . | . | . | . | . | N | . | . | . | . |
| 21-225_27H12_LC | . | . | . | . | . | . | | | . | . | . | . | . | . | . | . | . | . | . | . | . | . | . | . | . | . |
| 21-225_2A4_LC | . | . | . | F | . | . | | | . | . | . | . | . | . | . | . | . | . | . | . | . | . | . | . | . | . |
| 21-225_30A1_LC | . | . | . | . | . | . | | | . | . | . | . | . | . | . | . | . | . | . | . | . | . | . | . | . | . |
| 21-225_32B3_LC | . | . | . | . | . | . | | | . | . | . | . | . | . | . | . | . | . | . | . | . | . | . | . | . | . |
| 21-225_33A5_LC | . | . | . | . | . | . | | | . | . | . | . | . | . | . | . | . | . | . | . | . | . | . | . | . | . |
| 21-225_35H8_LC | . | . | . | . | . | . | | | . | . | . | . | . | . | . | . | . | . | . | . | . | . | . | . | . | . |
| 21-225_43A4_LC | . | . | . | . | . | . | | | . | . | . | . | . | N | . | . | . | . | . | . | . | . | . | . | . | . |
| 21-225_45D9_LC | . | . | . | . | . | . | | | . | . | . | . | . | N | . | . | . | . | . | . | . | . | . | . | . | . |
| 21-225_51C5_LC | . | . | . | . | . | . | | | . | . | . | ? | . | . | . | . | . | . | . | . | . | . | . | . | . | . |
| 21-225_51D5_LC | . | . | . | . | . | . | | | . | . | . | ? | . | . | . | . | . | . | . | . | . | . | . | . | . | . |
| 21-225_52E3_LC | . | . | . | . | . | . | | | . | . | . | . | . | . | . | . | . | . | . | . | . | . | . | . | . | . |
| 21-225_53E4_LC | . | . | . | . | . | . | | | . | . | . | . | . | . | . | . | . | . | . | . | . | . | . | . | . | . |
| 21-225_56F7_LC | . | . | . | . | . | . | | | . | . | . | . | . | . | . | . | . | . | . | . | . | . | . | . | . | . |
| 21-225_56H1_LC | . | . | . | . | . | . | | | . | . | . | . | . | . | . | . | . | . | . | . | . | . | . | . | . | . |
| 21-225_5A5_LC | . | . | . | . | . | . | | | . | . | . | . | . | . | . | . | . | . | . | . | . | . | . | . | . | . |
| 21-225_5E11_LC | . | . | N | . | . | . | | | . | . | . | . | . | . | . | . | . | . | . | . | . | . | . | . | . | . |
| 21-225_60A4_LC | . | . | . | . | . | . | | | . | . | . | . | . | . | . | . | . | . | S | . | . | . | . | . | . | . |
| 21-225_60F7_LC | . | . | . | . | . | . | | | . | . | . | . | . | . | . | . | . | . | . | . | . | . | . | . | . | . |
| 21-225_63H8_LC | . | . | . | . | . | . | | | . | . | . | . | . | . | . | . | . | . | . | . | . | . | . | . | . | . |
| 21-225_65D5_LC | . | N | . | . | . | . | | | S | . | . | . | . | . | . | . | . | . | . | . | . | . | . | . | . | . |
| 21-225_66H11_LC | . | . | . | F | . | . | | | . | . | . | . | . | . | . | N | . | . | . | . | . | . | . | . | N | . |
| 21-225_68E6_LC | . | . | . | . | . | . | | | . | . | . | . | . | . | . | . | . | . | . | . | . | . | . | . | . | ? |
| 21-225_7B1_LC | . | . | . | . | . | . | | | . | . | . | . | . | . | . | . | . | ? | . | . | . | . | . | . | . | . |
| 21-225_96C6_LC | . | . | . | . | . | . | | | . | . | . | . | . | . | . | N | . | . | . | . | . | . | . | . | . | . |
| 21-225_9C8_LC | . | . | . | . | . | . | | | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | K_CDR3 | | | | | | | |
| CONSENSUS | Y | C | Q | Q | Y | N | S | - | - | - | - | - | - | - | - | - | - | - | - | - | - | - | - | - | - | - |
| 21-225_148B6_LC | . | . | . | . | . | . | . | . | . | . | . | . | . | . | . | . | . | . | . | . | . | . | . | | | |
| 21-225_14H8_LC | . | . | . | . | . | . | . | . | . | . | . | . | . | . | . | . | . | . | . | . | . | . | . | | | |
| 21-225_153A1_LC | . | . | . | . | . | H | . | . | . | . | . | . | . | . | . | . | . | . | . | . | . | . | . | | | |
| 21-225_157E4_LC | . | . | . | . | . | H | . | . | . | . | . | . | . | . | . | . | . | . | . | . | . | . | . | | | |
| 21-225_157G5_LC | . | . | . | . | . | . | . | . | . | . | . | . | . | . | . | . | . | . | . | . | . | . | . | | | |
| 21-225_157H1_LC | . | . | . | . | . | H | . | . | . | . | . | . | . | . | . | . | . | . | . | . | . | . | . | | | |
| 21-225_157H10_LC | . | . | . | . | . | H | . | . | . | . | . | . | . | . | . | . | . | . | . | . | . | . | . | | | |
| 21-225_158H12_LC | . | . | . | . | . | H | . | . | . | . | . | . | . | . | . | . | . | . | . | . | . | . | . | | | |
| 21-225_159H11_LC | . | . | . | . | . | . | . | . | . | . | . | . | . | . | . | . | . | . | . | . | . | . | . | | | |
| 21-225_15A2_LC | . | . | . | . | . | Y | . | . | . | . | . | . | . | . | . | . | . | . | . | . | . | . | . | | | |
| 21-225_15B1_LC | . | . | . | . | . | H | . | . | . | . | . | . | . | . | . | . | . | . | . | . | . | . | . | | | |
| 21-225_160G1_LC | . | . | . | . | . | H | . | . | . | . | . | . | . | . | . | . | . | . | . | . | . | . | . | | | |
| 21-225_160G3_LC | . | . | . | . | . | H | . | . | . | . | . | . | . | . | . | . | . | . | . | . | . | . | . | | | |
| 21-225_163F1_LC | . | . | . | . | . | . | . | . | . | . | . | . | . | . | . | . | . | . | . | . | . | . | . | | | |
| 21-225_167D12_LC | H | . | . | . | . | . | . | . | . | . | . | . | . | . | . | . | . | . | . | . | . | . | . | | | |
| 21-225_169D10_LC | . | . | . | . | . | H | . | . | . | . | . | . | . | . | . | . | . | . | . | . | . | . | . | | | |
| 21-225_16B8_LC | . | . | H | . | . | H | . | . | . | . | . | . | . | . | . | . | . | . | . | . | . | . | . | | | |
| 21-225_16C7_LC | . | . | . | . | . | . | . | . | . | . | . | . | . | . | . | . | . | . | . | . | . | . | . | | | |
| 21-225_16G12_LC | . | . | . | . | . | H | . | . | . | . | . | . | . | . | . | . | . | . | . | . | . | . | . | . | . | . |
| 21-225_170E1_LC | . | . | . | . | . | H | . | . | . | . | . | . | . | . | . | . | . | . | . | . | . | . | . | . | . | . |
| 21-225_175C4_LC | . | . | . | . | . | Y | . | . | . | . | . | . | . | . | . | . | . | . | . | . | . | . | . | . | . | . |
| 21-225_177D3_LC | . | . | . | . | . | . | . | . | . | . | . | . | . | . | . | . | . | . | . | . | . | . | . | . | . | . |
| 21-225_17H5_LC | . | . | . | . | . | H | . | . | . | . | . | . | . | . | . | . | . | . | . | . | . | . | . | . | . | . |
| 21-225_17H6_LC | . | . | . | . | . | H | . | . | . | . | . | . | . | . | . | . | . | . | . | . | . | . | . | . | . | . |
| 21-225_181G2_LC | . | . | . | . | . | G | . | . | . | . | . | . | . | . | . | . | . | . | . | . | . | . | . | . | . | . |
| 21-225_184E7_LC | . | . | . | . | . | G | . | . | . | . | . | . | . | . | . | . | . | . | . | . | . | . | . | . | . | . |
| 21-225_185A1_LC | . | . | H | . | . | H | . | . | . | . | . | . | . | . | . | . | . | . | . | . | . | . | . | . | . | . |
| 21-225_185E10_LC | . | . | . | . | . | G | . | . | . | . | . | . | . | . | . | . | . | . | . | . | . | . | . | . | . | . |
| 21-225_188B9_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | N | S | - | - | - | - | - | - | - | - | - | - | - | - | - | - | - | - | - | - | - |
| 21-225_188H5_LC | . | . | . | . | . | . | . | . | . | . | . | . | . | . | . | . | . | . | . | . | . | . | . | . | . | . |
| 21-225_18D11_LC | . | . | . | . | . | . | T | . | . | . | . | . | . | . | . | . | . | . | . | . | . | . | . | . | . | . |
| 21-225_18G10_LC | . | . | L | . | . | . | . | . | . | . | . | . | . | . | . | . | . | . | . | . | . | . | . | . | . | . |
| 21-225_190A5_LC | . | . | . | . | . | S | N | . | . | . | . | . | . | . | . | . | . | . | . | . | . | . | . | . | . | . |
| 21-225_190B1_LC | . | . | . | . | . | L | N | . | . | . | . | . | . | . | . | . | . | . | . | . | . | . | . | . | . | . |
| 21-225_190F12_LC | . | . | . | R | . | D | T | . | . | . | . | . | . | . | . | . | . | . | . | . | . | . | . | . | . | . |
| 21-225_190H9_LC | . | . | . | R | . | D | T | . | . | . | . | . | . | . | . | . | . | . | . | . | . | . | . | . | . | . |
| 21-225_192A2_LC | . | . | . | . | . | . | . | . | . | . | . | . | . | . | . | . | . | . | . | . | . | . | . | . | . | . |
| 21-225_192C10_LC | . | . | . | R | . | D | T | . | . | . | . | . | . | . | . | . | . | . | . | . | . | . | . | . | . | . |
| 21-225_192E4_LC | . | . | . | R | . | D | T | . | . | . | . | . | . | . | . | . | . | . | . | . | . | . | . | . | . | . |
| 21-225_192H6_LC | . | . | . | . | . | S | N | . | . | . | . | . | . | . | . | . | . | . | . | . | . | . | . | . | . | . |
| 21-225_193F8_LC | . | . | . | . | . | . | . | . | . | . | . | . | . | . | . | . | . | . | . | . | . | . | . | . | . | . |
| 21-225_194H12_LC | . | . | . | R | . | D | T | . | . | . | . | . | . | . | . | . | . | . | . | . | . | . | . | . | . | . |
| 21-225_197E11_LC | . | G | . | R | . | D | T | . | . | . | . | . | . | . | . | . | . | . | . | . | . | . | . | . | . | . |
| 21-225_20B9_LC | . | . | . | . | . | . | T | . | . | . | . | . | . | . | . | . | . | . | . | . | . | . | . | . | . | . |
| 21-225_20C11_LC | . | . | . | . | . | H | . | . | . | . | . | . | . | . | . | . | . | . | . | . | . | . | . | . | . | . |
| 21-225_20F10_LC | . | . | . | . | . | . | . | . | . | . | . | . | . | . | . | . | . | . | . | . | . | . | . | . | . | . |
| 21-225_20H7_LC | . | . | . | . | . | Y | . | . | . | . | . | . | . | . | . | . | . | . | . | . | . | . | . | . | . | . |
| 21-225_20H9_LC | . | . | . | . | . | . | . | . | . | . | . | . | . | . | . | . | . | . | . | . | . | . | . | . | . | . |
| 21-225_211A6_LC | . | . | . | R | . | D | T | . | . | . | . | . | . | . | . | . | . | . | . | . | . | . | . | . | . | . |
| 21-225_213B3_LC | . | . | . | R | . | D | T | . | . | . | . | . | . | . | . | . | . | . | . | . | . | . | . | . | . | . |
| 21-225_214C3_LC | . | . | . | . | . | M | T | . | . | . | . | . | . | . | . | . | . | . | . | . | . | . | . | . | . | . |
| 21-225_214E4_LC | . | . | . | . | . | L | T | . | . | . | . | . | . | . | . | . | . | . | . | . | . | . | . | . | . | . |
| 21-225_223D11_LC | . | . | . | . | . | S | G | . | . | . | . | . | . | . | . | . | . | . | . | . | . | . | . | . | . | . |
| 21-225_22B12_LC | . | . | . | . | . | H | . | . | . | . | . | . | . | . | . | . | . | . | . | . | . | . | . | . | . | . |
| 21-225_23A11_LC | . | . | . | . | . | . | . | . | . | . | . | . | . | . | . | . | . | . | . | . | . | . | . | . | . | . |
| 21-225_23C7_LC | . | . | . | . | . | . | . | . | . | . | . | . | . | . | . | . | . | . | . | . | . | . | . | . | . | . |
| 21-225_23E7_LC | . | . | . | . | F | Y | . | . | . | . | . | . | . | . | . | . | . | . | . | . | . | . | . | . | . | . |
| 21-225_23G1_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | K_CDR3 | | | | | | | |
| CONSENSUS | Y | C | Q | Q | Y | N | S | - | - | - | - | - | - | - | - | - | - | - | - | - | - | - | - | - | - | - |
| 21-225_23G8_LC | . | . | . | . | . | . | | . | . | . | . | . | . | . | . | . | . | . | . | . | . | . | . | . | . | . |
| 21-225_25C10_LC | . | . | . | . | . | H | | . | . | . | . | . | . | . | . | . | . | . | . | . | . | . | . | . | . | . |
| 21-225_26G1_LC | . | . | . | . | . | H | | . | . | . | . | . | . | . | . | . | . | . | . | . | . | . | . | . | . | . |
| 21-225_27H12_LC | . | . | . | . | . | Y | | . | . | . | . | . | . | . | . | . | . | . | . | . | . | . | . | . | . | . |
| 21-225_2A4_LC | . | . | . | . | . | . | | . | . | . | . | . | . | . | . | . | . | . | . | . | . | . | . | . | . | . |
| 21-225_30A1_LC | . | . | . | . | . | . | | . | . | . | . | . | . | . | . | . | . | . | . | . | . | . | . | . | . | . |
| 21-225_32B3_LC | . | . | . | . | . | . | | . | . | . | . | . | . | . | . | . | . | . | . | . | . | . | . | . | . | . |
| 21-225_33A5_LC | . | . | . | . | . | . | | . | . | . | . | . | . | . | . | . | . | . | . | . | . | . | . | . | . | . |
| 21-225_35H8_LC | . | . | . | . | . | . | | . | . | . | . | . | . | . | . | . | . | . | . | . | . | . | . | . | . | . |
| 21-225_43A4_LC | . | . | . | . | . | Y | | . | . | . | . | . | . | . | . | . | . | . | . | . | . | . | . | . | . | . |
| 21-225_45D9_LC | . | . | . | H | . | Y | | . | . | . | . | . | . | . | . | . | . | . | . | . | . | . | . | . | . | . |
| 21-225_51C5_LC | . | . | . | . | . | Y | | . | . | . | . | . | . | . | . | . | . | . | . | . | . | . | . | . | . | . |
| 21-225_51D5_LC | . | . | . | . | . | Y | | . | . | . | . | . | . | . | . | . | . | . | . | . | . | . | . | . | . | . |
| 21-225_52E3_LC | . | . | . | . | . | . | | . | . | . | . | . | . | . | . | . | . | . | . | . | . | . | . | . | . | . |
| 21-225_53E4_LC | . | . | . | . | . | H | | . | . | . | . | . | . | . | . | . | . | . | . | . | . | . | . | . | . | . |
| 21-225_56F7_LC | . | . | H | . | . | . | | . | . | . | . | . | . | . | . | . | . | . | . | . | . | . | . | . | . | . |
| 21-225_56H1_LC | . | . | . | . | . | Q | N | . | . | . | . | . | . | . | . | . | . | . | . | . | . | . | . | . | . | . |
| 21-225_5A5_LC | . | . | . | . | . | . | | . | . | . | . | . | . | . | . | . | . | . | . | . | . | . | . | . | . | . |
| 21-225_5E11_LC | . | . | H | . | . | H | | . | . | . | . | . | . | . | . | . | . | . | . | . | . | . | . | . | . | . |
| 21-225_60A4_LC | . | . | . | . | . | K | | . | . | . | . | . | . | . | . | . | . | . | . | . | . | . | . | . | . | . |
| 21-225_60F7_LC | . | . | L | . | P | H | | . | . | . | . | . | . | . | . | . | . | . | . | . | . | . | . | . | . | . |
| 21-225_63H8_LC | . | . | . | . | . | H | | . | . | . | . | . | . | . | . | . | . | . | . | . | . | . | . | . | . | . |
| 21-225_65D5_LC | . | . | P | L | . | K | | . | . | . | . | . | . | . | . | . | . | . | . | . | . | . | . | . | . | . |
| 21-225_66H11_LC | . | . | . | . | . | S | O | . | . | . | . | . | . | . | . | . | . | . | . | . | . | . | . | . | . | . |
| 21-225_68E6_LC | . | . | . | . | . | O | | . | . | . | . | . | . | . | . | . | . | . | . | . | . | . | . | . | . | . |
| 21-225_7B1_LC | . | . | H | . | . | . | | . | . | . | . | . | . | . | . | . | . | . | . | . | . | . | . | . | . | . |
| 21-225_96C6_LC | . | . | H | . | . | . | O | . | . | . | . | . | . | . | . | . | . | . | . | . | . | . | . | . | . | . |
| 21-225_9C8_LC | . | . | . | . | . | O | | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | | K_FR4 | | | | |
| CONSENSUS | - | - | - | - | Y | P | F | T | F | G | P | G | T | K | V | D | I | K | R |
| 21-225_148B6_LC | . | . | . | . | . | . | . | . | . | . | . | . | . | . | . | . | . | . | . |
| 21-225_14H8_LC | . | . | . | . | . | . | . | . | . | . | . | . | . | . | . | . | . | N | . |
| 21-225_153A1_LC | . | . | . | . | . | . | . | . | . | . | . | . | . | . | . | . | . | . | . |
| 21-225_157E4_LC | . | . | . | . | . | . | . | . | . | . | . | . | . | . | . | . | . | . | . |
| 21-225_157G5_LC | . | . | . | . | . | . | . | . | . | . | . | . | . | . | . | . | . | . | . |
| 21-225_157H1_LC | . | . | . | . | . | . | . | . | . | . | . | . | . | . | . | . | . | . | . |
| 21-225_157H10_LC | . | . | . | . | . | . | . | . | . | . | . | . | . | . | . | . | . | . | . |
| 21-225_158H12_LC | . | . | . | . | . | . | . | . | . | . | . | . | . | . | . | . | . | . | . |
| 21-225_159H11_LC | . | . | . | . | . | . | . | . | . | . | . | . | . | . | . | . | . | . | . |
| 21-225_15A2_LC | . | . | . | . | . | . | . | . | . | . | . | . | . | Q | . | . | V | . | . |
| 21-225_15B1_LC | . | . | . | . | . | . | . | . | . | . | . | . | . | . | . | . | . | . | . |
| 21-225_160G1_LC | . | . | . | . | . | . | . | . | . | . | . | . | . | . | . | . | . | . | . |
| 21-225_160G3_LC | . | . | . | . | . | . | . | . | . | . | . | . | . | . | . | . | . | . | . |
| 21-225_163F1_LC | . | . | . | . | . | . | . | . | . | . | . | . | . | Q | . | . | . | Q | . |
| 21-225_167D12_LC | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . |
| 21-225_16B8_LC | . | . | . | . | . | . | . | . | . | . | . | . | . | . | . | . | V | . | . |
| 21-225_16C7_LC | . | . | . | . | . | . | . | . | . | . | . | . | . | . | . | . | . | . | . |
| 21-225_16G12_LC | . | . | . | . | . | . | . | . | . | . | . | . | . | . | . | . | . | . | . |
| 21-225_170E1_LC | . | . | . | . | . | . | . | . | . | . | . | . | . | . | . | . | . | . | . |
| 21-225_175C4_LC | . | . | . | . | . | . | . | . | . | . | . | . | . | . | . | . | . | . | . |
| 21-225_177D3_LC | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . |
| 21-225_17H6_LC | . | . | . | . | . | . | . | . | . | . | . | . | . | . | . | . | . | Q | . |
| 21-225_181G2_LC | . | . | . | . | . | . | . | . | . | . | . | . | . | . | . | . | . | . | . |
| 21-225_184E7_LC | . | . | . | . | . | . | . | . | . | . | . | . | . | . | . | . | . | . | . |
| 21-225_185A1_LC | . | . | . | . | . | . | . | . | . | . | . | . | . | . | . | . | . | . | . |
| 21-225_185E10_LC | . | . | . | . | . | . | . | . | . | . | . | . | . | . | . | . | . | . | . |
| 21-225_188B9_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | F | T | F | G | P | G | T | K | V | D | I | K | R |
| 21-225_188H5_LC | . | . | . | . | . | . | . | . | . | . | . | . | . | . | . | . | . | . | . |
| 21-225_18D11_LC | . | . | . | . | . | . | . | . | . | . | . | . | . | T | . | . | . | . | . |
| 21-225_18G10_LC | . | . | . | . | . | . | . | . | . | . | . | . | . | . | . | . | . | . | . |
| 21-225_190A5_LC | . | . | . | . | . | . | V | . | . | . | . | . | . | . | . | . | . | . | . |
| 21-225_190B1_LC | . | . | . | . | . | . | V | . | . | . | . | . | . | . | . | . | . | S | . |
| 21-225_190F12_LC | . | . | . | . | . | . | . | . | . | . | . | . | . | . | . | . | . | . | . |
| 21-225_190H9_LC | . | . | . | . | . | . | . | . | . | . | . | . | . | . | . | . | . | . | . |
| 21-225_192A2_LC | . | . | . | . | . | . | . | . | . | . | . | . | . | . | . | . | . | . | . |
| 21-225_192C10_LC | . | . | . | . | . | . | . | . | . | . | . | . | . | . | . | . | S | . | . |
| 21-225_192E4_LC | . | . | . | . | . | . | . | . | . | . | . | . | . | . | . | . | . | . | . |
| 21-225_192H6_LC | . | . | . | . | . | . | V | . | . | . | . | . | . | . | . | . | . | . | . |
| 21-225_193F8_LC | . | . | . | . | . | . | . | . | . | . | . | . | . | . | . | . | . | . | . |
| 21-225_194H12_LC | . | . | . | . | . | . | . | . | . | . | . | . | . | . | . | . | . | . | . |
| 21-225_197E11_LC | . | . | . | . | . | . | . | . | . | . | . | . | . | . | . | . | . | . | . |
| 21-225_20B9_LC | . | . | . | . | . | . | . | . | . | . | . | . | . | . | . | . | . | . | . |
| 21-225_20C11_LC | . | . | . | . | S | . | . | . | . | . | . | . | . | . | . | . | V | . | . |
| 21-225_20F10_LC | . | . | . | . | . | . | . | . | . | . | . | . | . | . | . | . | N | . | . |
| 21-225_20H7_LC | . | . | . | . | . | . | . | . | . | . | . | . | . | . | S | . | S | . | . |
| 21-225_20H9_LC | . | . | . | . | . | . | . | . | . | . | . | . | . | . | . | . | . | . | . |
| 21-225_211A6_LC | . | . | . | . | . | . | . | . | . | . | . | . | . | . | . | . | . | . | . |
| 21-225_213B3_LC | . | . | . | . | . | . | . | . | . | . | . | . | . | . | . | . | . | . | . |
| 21-225_214C3_LC | . | . | . | . | . | . | . | . | . | . | . | . | . | . | . | . | . | . | . |
| 21-225_214E4_LC | . | . | . | . | . | . | . | . | . | . | . | . | . | . | . | . | . | . | . |
| 21-225_223D11_LC | . | . | . | . | . | . | . | . | . | . | . | . | . | . | . | . | T | . | . |
| 21-225_22B12_LC | . | . | . | . | . | . | . | . | . | . | . | . | . | . | . | . | S | . | . |
| 21-225_23A11_LC | . | . | . | . | . | . | . | . | . | . | . | . | . | . | . | . | L | . | . |
| 21-225_23C7_LC | . | . | . | . | . | . | . | . | . | . | . | . | . | . | . | . | . | . | . |
| 21-225_23E7_LC | . | . | . | . | . | . | . | . | . | . | . | . | . | . | . | . | . | N | . |
| 21-225_23G1_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | K_FR4 | | | | | | | |
| CONSENSUS | - | - | - | - | Y | P | F | T | F | G | P | G | T | K | V | D | I | K | R |
| 21-225_23G8_LC | . | . | . | . | . | . | . | . | . | . | . | . | . | . | . | . | . | . | . |
| 21-225_25C10_LC | . | . | . | . | . | . | . | . | . | . | . | . | . | . | . | . | . | . | . |
| 21-225_26G1_LC | . | . | . | . | . | . | . | . | . | . | . | . | . | . | . | . | . | . | . |
| 21-225_27H12_LC | . | . | . | . | . | . | . | . | . | . | . | . | . | . | . | N | . | N | . |
| 21-225_2A4_LC | . | . | . | . | . | . | . | . | . | . | . | . | . | . | . | . | . | . | . |
| 21-225_30A1_LC | . | . | . | . | . | . | . | . | . | . | . | . | . | . | . | . | . | . | . |
| 21-225_32B3_LC | . | . | . | . | . | . | . | . | . | . | . | . | . | . | . | . | . | . | . |
| 21-225_33A5_LC | . | . | . | . | . | . | . | . | . | . | . | . | . | N | . | . | . | . | . |
| 21-225_35H8_LC | . | . | . | . | . | . | . | . | . | . | . | . | . | . | . | . | . | . | . |
| 21-225_43A4_LC | . | . | . | . | . | . | . | . | . | . | . | . | . | . | . | . | . | . | . |
| 21-225_45D9_LC | . | . | . | . | . | . | . | . | . | . | . | . | . | . | . | . | . | . | . |
| 21-225_51C5_LC | . | . | . | . | . | . | . | . | . | . | . | . | . | . | . | . | . | . | . |
| 21-225_51D5_LC | . | . | . | . | . | . | . | . | . | . | . | . | . | . | . | . | . | . | . |
| 21-225_52E3_LC | . | . | . | . | . | . | . | . | . | . | . | . | . | . | . | . | . | . | . |
| 21-225_53E4_LC | . | . | . | . | . | . | . | . | . | . | . | . | . | . | . | . | . | . | . |
| 21-225_56F7_LC | . | . | . | . | . | . | . | . | . | . | . | . | . | . | . | . | . | Y | G |
| 21-225_56H1_LC | . | . | . | . | . | . | . | . | . | . | . | . | . | . | . | . | . | . | . |
| 21-225_5A5_LC | . | . | . | . | . | . | . | . | . | . | . | . | . | . | . | . | . | . | . |
| 21-225_5E11_LC | . | . | . | . | . | . | . | . | . | . | . | . | . | . | . | . | . | . | . |
| 21-225_60A4_LC | . | . | . | . | . | . | . | . | . | . | . | . | . | . | . | . | . | . | . |
| 21-225_60F7_LC | . | . | . | . | . | . | . | . | . | . | . | . | . | . | M | . | . | . | . |
| 21-225_63H8_LC | . | . | . | . | . | . | . | . | . | . | . | . | . | . | . | . | . | . | . |
| 21-225_65D5_LC | . | . | . | . | . | . | . | . | . | . | . | . | . | . | . | . | . | . | . |
| 21-225_66H11_LC | . | . | . | . | . | . | . | . | . | . | . | . | . | . | . | . | . | . | . |
| 21-225_68E6_LC | . | . | . | . | . | . | . | . | . | . | . | . | . | . | . | . | . | . | . |
| 21-225_7B1_LC | . | . | . | . | . | . | . | . | . | . | . | . | . | . | . | . | . | . | . |
| 21-225_96C6_LC | . | . | . | . | . | . | . | . | . | . | . | . | . | . | . | . | . | . | . |
| 21-225_9C8_LC | . | . | . | . | . | . | . | . | . | . | . | . | . | . | . | . | . | . | . |

FIGURE 57

Table 104.    Consensus 24 - VK1|L5/JK3 (SEQ ID NO: 50321):

DIQMTQSPSSVSASVGDRVTITC<u>RAS--QGIS</u>------<u>RWLA</u>WYQQKPGKAPKLLIY<u>A</u>-------<u>ASSLQSG</u>VPSRFSGSGSG--
TDFTLTISSLQPEDFATYYC<u>QQANS</u>--------------------FPFTFGPGTKVDIKR wherein:

A at position 25 can be substituted with V, E or G

S at position 26 can be substituted with G

Q at position 29 can be substituted with R

G at position 30 can be substituted with D, N, A, L or V

I at position 31 can be substituted with V or F

S at position 32 can be substituted with N, T, R or I

R at position 39 can be substituted with S, N, K, T, D, I or G

W at position 40 can be substituted with Y

L at position 41 can be substituted with I

A at position 42 can be substituted with T or V

A at position 58 can be substituted with G, T, D or V

A at position 67 can be substituted with T or V

S at position 68 can be substituted with Y

S at position 69 can be substituted with R, N, T, G or I

L at position 70 can be substituted with F

Q at position 71 can be substituted with E

**FIGURE 57**

S at position 72 can be substituted with G, N or D

Q at position 107 can be substituted with H

A at position 109 can be substituted with T, G, S, V or D

N at position 110 can be substituted with D, K or S

S at position 111 can be substituted with I

F at position 135 can be substituted with L, I or V

F at position 137 can be substituted with I

Arg Xaa Xaa Xaa Xaa Xaa Xaa Xaa Xaa Xaa (SEQ ID NO: 50147)

Xaa Xaa Xaa Xaa Xaa Xaa Xaa (SEQ ID NO: 50148)

Xaa Gln Xaa Xaa Xaa Xaa Pro Xaa Thr (SEQ ID NO: 50149)

Arg Ala Ser Gln Gly Ile Ser Arg Trp Leu Ala (SEQ ID NO: 50633)

Ala Ala Ser Ser Leu Gln Ser (SEQ ID NO: 50634)

Gln Gln Ala Asn Ser Phe Pro Phe Thr (SEQ ID NO: 50635)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | V | S | A | S | V | G | D | R | V | T | I | T | C | R | A | S |
| 21-225_10G6_LC | . | | | . | | . | | . | . | . | | . | | . | | . | . | . | . | . | . | . | . | . | . | . |
| 21-225_12E9_LC | | | | . | | . | . | . | . | | . | . | | . | | 8 | . | . | . | . | . | . | . | . | . | . |
| 21-225_146A6_LC | | | | . | | . | . | . | A | . | | . | | . | | . | . | . | . | . | . | . | . | . | . | . |
| 21-225_146E1_LC | . | 8 | | . | | . | . | . | . | . | | . | | . | | . | . | . | . | . | . | . | . | . | . | . |
| 21-225_147D10_LC | | | | . | | . | . | . | A | . | | . | | . | | . | . | . | . | . | . | . | . | . | . | . |
| 21-225_147D12_LC | | | | . | | . | . | . | A | . | | . | | . | | . | . | . | . | . | . | . | . | . | . | . |
| 21-225_148E2_LC | | | | . | | . | . | . | A | . | | . | | . | | . | . | . | . | . | . | . | . | . | . | . |
| 21-225_149B6_LC | . | | | . | | . | . | . | . | | . | . | | . | | . | . | . | . | . | . | . | . | . | . | . |
| 21-225_149C6_LC | . | | | . | | . | . | . | A | . | | . | | . | | . | . | . | . | . | . | . | . | . | . | . |
| 21-225_149D11_LC | . | | | . | | . | . | . | A | . | | . | | . | | . | . | . | . | . | . | . | . | . | . | . |
| 21-225_149F8_LC | . | | | . | | . | . | . | A | . | | . | | . | | . | . | . | . | . | . | . | . | . | . | . |
| 21-225_150C5_LC | . | | | . | | . | . | . | G | . | | . | | . | | . | . | . | . | . | . | . | . | . | . | . |
| 21-225_152G10_LC | . | | | . | | . | . | . | A | . | | . | | . | | . | . | . | . | . | . | . | . | . | . | . |
| 21-225_152H7_LC | . | | | . | | . | . | . | A | . | | . | | . | | . | . | . | . | . | . | . | . | . | . | . |
| 21-225_155A4_LC | . | | | . | | . | . | . | A | . | | . | | . | | . | . | . | . | . | . | . | . | . | . | . |
| 21-225_155B4_LC | . | | | . | | . | . | . | A | . | | . | | . | | . | . | . | . | . | . | . | . | . | . | . |
| 21-225_180A3_LC | . | | | . | | . | . | . | . | | . | . | | . | | . | . | . | . | . | . | . | . | . | . | . |
| 21-225_182H5_LC | . | | | . | | . | . | . | . | . | Y | . | | . | | . | . | . | . | . | . | . | . | . | . | . |
| 21-225_186A11_LC | . | | | . | | . | . | . | . | | . | . | | . | | . | . | . | . | . | . | . | . | . | . | . |
| 21-225_192H2_LC | . | | | . | | . | . | . | . | | . | . | | . | | . | . | . | . | . | . | . | . | . | . | . |
| 21-225_196F2_LC | . | | | . | | . | . | . | . | | . | . | | . | | . | . | . | . | . | . | . | . | . | . | . |
| 21-225_210H11_LC | . | | | . | | . | . | . | . | | . | . | | . | | . | . | . | . | . | . | . | . | . | . | . |
| 21-225_221A6_LC | . | | | . | | . | . | . | . | | . | . | | . | | . | . | . | . | . | . | . | . | . | . | . |
| 21-225_221F12_LC | . | | | . | | . | . | . | . | | . | . | | . | | . | . | . | . | . | . | . | . | . | . | . |
| 21-225_222C12_LC | . | | | . | | . | C | . | . | | . | . | | . | | . | . | . | . | . | . | . | . | . | V | . |
| 21-225_222E1_LC | . | | | . | | . | . | . | . | | . | . | | . | | . | . | . | . | . | . | . | . | . | . | . |
| 21-225_222F7_LC | . | | | . | | . | . | . | . | | . | . | | . | | . | . | . | . | . | . | . | . | . | . | . |
| 21-225_23B11_LC | . | | | . | | . | . | . | . | | . | . | | . | | . | . | . | . | . | . | . | . | . | . | . |
| 21-225_25E11_LC | . | | | . | | . | . | . | . | | . | . | | . | | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | V | S | A | S | V | G | D | R | V | T | I | T | C | R | A | S |
| 21-225_28B1_LC | . | . | . | . | . | . | . | . | . | . | . | . | . | . | . | . | . | . | . | . | . | . | . | . | . | . |
| 21-225_28F11_LC | . | . | . | . | . | . | . | . | . | . | . | . | . | . | . | . | . | . | . | . | . | . | . | . | . | . |
| 21-225_30A11_LC | . | . | . | . | . | . | . | . | . | . | . | . | . | . | . | . | . | . | . | . | . | . | . | . | . | . |
| 21-225_31C4_LC | . | . | . | . | . | . | . | . | . | . | . | . | . | . | . | . | . | . | . | . | . | . | . | . | . | . |
| 21-225_31H9_LC | . | . | . | . | . | . | . | . | . | . | . | . | . | . | . | . | . | . | . | . | . | . | . | . | . | . |
| 21-225_33G1_LC | . | . | . | . | . | . | . | . | . | . | . | . | V | . | . | . | . | . | . | . | . | . | . | . | . | . |
| 21-225_33H7_LC | . | . | . | . | . | . | . | . | . | . | . | . | . | . | . | . | . | . | . | . | . | . | . | . | . | . |
| 21-225_34F3_LC | . | . | . | . | . | . | . | . | . | . | . | . | . | T | . | . | . | . | . | . | S | . | . | . | . | . |
| 21-225_34G7_LC | . | . | . | . | . | . | . | . | . | . | . | . | . | . | . | . | . | . | . | . | . | . | . | . | . | . |
| 21-225_35C6_LC | . | . | . | . | . | . | . | . | . | . | . | . | . | . | . | . | . | . | . | . | . | . | . | . | . | . |
| 21-225_35G6_LC | . | . | . | . | . | . | . | . | . | . | . | . | . | . | . | . | . | . | L | . | . | . | . | . | . | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | . | . | . | . | . | . | . | . | . | . | . | . | . | . | . | . | . | . | . | . | . | . | . | . | . | . |
| 21-225_44F3_LC | . | . | . | . | . | . | . | . | . | . | . | . | . | . | . | . | . | . | . | . | . | . | . | . | . | . |
| 21-225_45C9_LC | . | . | . | . | . | . | . | . | . | . | . | . | V | . | . | . | . | . | . | . | . | . | . | . | . | . |
| 21-225_45H4_LC | . | . | . | . | . | . | . | . | . | . | . | . | . | . | . | . | . | . | . | . | . | . | . | . | . | . |
| 21-225_49F1_LC | . | . | . | . | . | . | . | . | . | . | . | . | . | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | . | . | . | . | . | . | . | . | . | . | . | . | . | . | . | . | . | . | . | . | . | . |
| 21-225_4H6_LC | . | . | . | . | . | . | . | . | . | . | . | . | . | . | . | . | . | . | . | . | . | . | . | . | . | . |
| 21-225_4H6.004_LC | . | . | . | . | . | . | . | . | . | . | . | . | . | . | . | . | . | . | . | . | . | . | . | . | . | . |
| 21-225_4H6.005_LC | . | . | . | . | . | . | . | . | . | . | . | . | . | . | . | . | . | . | . | . | . | . | . | . | . | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | . | . |
| 21-225_4H6.008_LC | . | . | . | . | . | . | . | . | . | . | . | . | . | . | . | . | . | . | . | . | . | . | . | . | . | . |
| 21-225_4H6.009_LC | . | . | . | . | . | . | . | . | . | . | . | . | . | . | . | . | . | . | . | . | . | . | . | . | . | . |
| 21-225_4H6.010_LC | . | . | . | . | . | . | . | . | . | . | . | . | . | . | . | . | . | . | . | . | . | . | . | . | . | . |
| 21-225_4H6.011_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | V | S | A | S | V | G | D | R | V | T | I | T | C | R | A | S |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | L | . | . | . | . | . | . | . |
| 21-225_50F3_LC | . | . | . | . | . | . | . | . | . | . | . | . | . | . | . | . | . | . | . | . | . | . | . | . | . | . |
| 21-225_51C7_LC | . | . | . | . | . | . | . | . | . | . | . | . | . | . | . | . | . | . | . | . | . | . | . | . | . | . |
| 21-225_51E9_LC | . | . | . | . | . | . | . | . | . | . | . | . | . | . | . | . | . | . | . | . | . | . | . | . | . | . |
| 21-225_52B1_LC | . | . | . | . | . | . | . | . | . | . | . | T | . | F | . | . | . | . | I | . | . | . | . | . | . | . |
| 21-225_52G11_LC | . | . | . | . | . | . | . | . | . | . | . | . | . | . | . | . | . | . | . | . | . | . | . | . | . | . |
| 21-225_52H10_LC | . | . | . | . | . | . | . | . | . | . | . | . | . | . | . | . | . | . | . | . | . | . | . | . | . | . |
| 21-225_53F2_LC | . | . | . | . | . | . | . | . | . | . | . | . | . | . | . | . | . | . | . | . | . | . | . | . | . | . |
| 21-225_53F7_LC | . | . | . | S | . | . | . | . | . | . | . | . | . | . | . | . | . | . | . | . | . | . | . | . | . | . |
| 21-225_55B1_LC | . | . | . | . | . | . | . | . | . | . | . | . | . | . | . | . | . | . | . | . | . | . | . | . | . | . |
| 21-225_56C6_LC | . | . | . | . | . | . | . | . | . | . | . | . | . | . | . | . | . | . | . | . | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | . | . | . | . | . | . | . | . | . | . | G | . | Y | . | . | . | . | . | . | . | . | . | . | V | . |
| 21-225_57A7_LC | . | . | . | . | . | . | . | . | . | . | . | . | . | . | . | . | . | . | . | . | . | . | . | . | . | . |
| 21-225_57E4_LC | . | . | . | . | . | . | . | . | . | . | . | . | . | . | . | . | . | . | . | S | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | . | . | . | . | . | . | . | . | . | . | . | . | . | . | . | . | . | . | . | . | . | . | . |
| 21-225_65F10_LC | . | . | . | . | . | . | . | . | . | . | . | . | . | . | . | . | . | . | . | . | . | . | . | . | G | G |
| 21-225_66F11_LC | . | . | . | . | . | . | . | . | . | . | . | G | . | . | . | . | . | . | . | . | . | . | . | . | E | . |
| 21-225_70D1_LC | . | . | . | . | . | . | . | . | . | . | . | . | . | . | . | . | . | . | . | . | . | . | . | . | . | . |
| 21-225_7C3_LC2_LC | . | . | . | . | . | . | . | . | . | . | . | . | . | L | . | . | . | . | . | . | . | . | . | . | . | . |
| 21-225_9F12_LC1_LC | . | . | . | . | . | . | . | . | . | . | . | . | . | . | . | . | . | . | . | . | . | . | . | . | . | . |

4701

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | R | W | L | A | W | Y | Q | Q | K | P | G | K | A | P |
| 21-225_10G6_LC | . | . | . | . | . | . | . | . | . | . | . | . | X | . | . | . | . | . | . | . | . | . | . | . | . | . |
| 21-225_12E9_LC | . | . | . | . | . | . | . | . | . | . | . | . | S | . | . | . | . | . | . | . | . | . | . | E | . | . |
| 21-225_146A6_LC | . | . | . | . | . | . | . | . | . | . | . | . | N | . | . | . | . | . | . | . | . | . | . | . | . | . |
| 21-225_146E1_LC | . | . | . | . | . | N | . | . | . | . | . | . | N | . | . | V | . | . | . | . | . | . | . | . | . | . |
| 21-225_147D10_LC | . | . | N | . | . | . | . | . | . | . | . | . | N | . | . | T | . | . | . | . | . | . | . | . | . | . |
| 21-225_147D12_LC | . | . | . | . | . | . | . | . | . | . | . | . | N | . | . | . | . | . | . | S | . | . | . | . | . | . |
| 21-225_148E2_LC | . | . | . | . | . | . | . | . | . | . | . | . | N | . | . | . | . | . | . | . | . | . | . | . | . | . |
| 21-225_149B6_LC | . | . | . | . | . | I | . | . | . | . | . | . | S | . | . | . | . | . | . | . | . | . | . | . | . | . |
| 21-225_149C6_LC | . | . | . | . | . | . | . | . | . | . | . | . | N | . | . | . | . | . | . | . | . | . | . | X | . | . |
| 21-225_149D11_LC | . | . | N | . | . | . | . | . | . | . | . | . | N | . | . | T | . | . | . | . | . | . | . | . | . | . |
| 21-225_149F8_LC | . | . | . | . | . | . | . | . | . | . | . | . | N | . | . | . | . | . | . | . | . | . | . | . | . | . |
| 21-225_150C5_LC | . | . | . | . | . | N | . | . | . | . | . | . | N | . | . | . | . | . | . | . | . | . | . | . | . | . |
| 21-225_152G10_LC | . | . | . | . | . | . | . | . | . | . | . | . | N | . | . | . | . | . | . | . | . | . | . | . | . | . |
| 21-225_152H7_LC | . | . | . | D | . | . | . | . | . | . | . | . | N | . | . | . | . | . | . | . | . | . | . | . | . | . |
| 21-225_155A4_LC | . | . | . | . | . | . | . | . | . | . | . | . | N | . | . | . | . | . | X | . | . | . | . | . | . | . |
| 21-225_155B4_LC | . | . | . | D | . | . | . | . | . | . | . | . | N | . | . | . | . | . | . | . | . | . | . | . | . | . |
| 21-225_180A3_LC | . | . | . | D | . | T | . | . | . | . | . | . | S | . | . | . | . | . | . | . | . | . | . | . | . | . |
| 21-225_182H5_LC | . | . | . | D | . | T | . | . | . | . | . | . | S | . | . | . | . | . | . | . | . | . | . | . | . | . |
| 21-225_186A11_LC | . | . | . | D | . | T | . | . | . | . | . | . | S | . | . | . | . | . | . | . | . | . | . | . | . | . |
| 21-225_192H2_LC | . | . | . | . | . | . | . | . | . | . | . | . | S | . | I | . | . | . | . | . | . | . | . | . | . | . |
| 21-225_196F2_LC | . | . | . | . | . | . | . | . | . | . | . | . | S | . | . | . | . | . | . | . | . | . | . | . | . | . |
| 21-225_210H11_LC | . | . | . | . | . | . | . | . | . | . | . | . | I | . | . | . | . | . | . | . | . | . | . | . | . | . |
| 21-225_221A6_LC | . | . | . | . | . | . | . | . | . | . | . | . | S | . | . | . | . | . | . | . | . | . | . | . | . | . |
| 21-225_221F12_LC | . | . | . | . | . | . | . | . | . | . | . | . | S | . | . | . | . | . | . | . | . | . | . | . | . | . |
| 21-225_222C12_LC | . | . | . | . | . | . | . | . | . | . | . | . | S | . | . | . | . | . | . | . | . | . | . | . | . | L |
| 21-225_222E1_LC | . | . | . | . | . | . | . | . | . | . | . | . | S | . | . | . | . | . | . | . | . | . | . | . | . | . |
| 21-225_222F7_LC | . | . | . | . | . | . | . | . | . | . | . | . | S | . | . | . | . | . | . | . | . | . | . | . | . | . |
| 21-225_23B11_LC | . | . | . | . | . | T | . | . | . | . | . | . | X | . | . | . | . | . | . | . | . | . | . | . | . | . |
| 21-225_25E11_LC | . | . | . | . | . | . | . | . | . | . | . | . | N | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | R | W | L | A | W | Y | Q | Q | K | P | G | K | A | P |
| 21-225_28B1_LC | . | . | . | A | . | N | . | | . | | | . | D | . | | | . | . | . | . | . | . | . | . | . | . |
| 21-225_28F11_LC | . | . | . | . | . | . | . | | | . | | | N | . | | . | . | . | . | . | . | . | . | . | . | . |
| 21-225_30A11_LC | . | . | . | . | . | . | | . | . | . | | . | . | . | . | . | . | . | . | . | . | . | . | R | . | . |
| 21-225_31C4_LC | . | . | . | . | . | . | . | . | . | . | | . | X | . | . | . | . | . | . | . | . | . | . | . | . | . |
| 21-225_31H9_LC | . | . | . | . | . | Y | . | . | . | . | . | . | S | . | . | T | . | . | . | . | R | . | . | . | . | . |
| 21-225_33G1_LC | . | . | . | . | . | . | . | . | . | . | . | . | . | . | . | . | . | . | . | . | . | . | . | . | . | . |
| 21-225_33H7_LC | . | . | . | . | . | . | . | . | . | . | . | . | . | . | . | . | . | . | . | . | . | V | . | . | V | . |
| 21-225_34F3_LC | . | . | . | . | . | . | . | . | . | . | . | . | X | . | . | . | . | . | . | . | . | . | . | . | . | . |
| 21-225_34G7_LC | . | . | . | L | . | . | . | . | . | . | . | . | X | . | . | . | . | . | . | . | . | . | . | . | . | . |
| 21-225_35C6_LC | . | . | . | . | . | . | . | . | . | . | . | . | X | . | . | . | . | . | . | . | . | . | . | . | . | . |
| 21-225_35G6_LC | . | . | . | . | . | . | . | . | . | . | . | . | . | . | . | . | . | . | . | . | . | V | . | . | V | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | . | . | . | D | . | . | . | . | . | . | . | . | S | . | . | . | . | . | . | X | . | . | . | . | . | . |
| 21-225_44F3_LC | . | . | . | . | . | . | . | . | . | . | . | . | Q | . | . | . | . | . | . | . | . | . | . | . | . | . |
| 21-225_45C9_LC | . | . | . | D | . | . | . | . | . | . | . | . | Q | . | . | . | . | . | . | . | R | . | . | . | . | . |
| 21-225_45H4_LC | . | . | . | D | . | . | . | . | . | . | . | . | S | . | . | . | . | . | . | . | X | . | . | . | . | . |
| 21-225_49F1_LC | . | . | R | D | . | N | . | . | . | . | . | . | G | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | P | . | . | . | . | . | . | . | T | . | . | . | . | P | . | . | . | . | . | . | . | . |
| 21-225_4H6_LC | . | . | . | . | . | . | . | . | . | . | . | . | . | . | . | . | . | . | . | . | . | . | . | . | . | . |
| 21-225_4H6.004_LC | . | . | . | . | . | . | . | . | . | . | . | . | . | . | . | . | . | . | . | . | . | . | . | . | . | . |
| 21-225_4H6.005_LC | . | . | . | . | . | . | . | . | . | . | . | . | . | . | . | . | . | . | . | . | . | . | . | . | . | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | . | . |
| 21-225_4H6.008_LC | . | . | . | . | . | . | . | . | . | . | . | . | . | . | . | . | . | . | . | . | . | . | . | . | . | . |
| 21-225_4H6.009_LC | . | . | . | . | . | . | . | . | . | . | . | . | . | . | . | . | . | . | . | . | . | . | . | . | . | . |
| 21-225_4H6.010_LC | . | . | . | . | . | . | . | . | . | . | . | . | . | . | . | . | . | . | . | . | . | . | . | . | . | . |
| 21-225_4H6.011_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | R | W | L | A | W | Y | Q | Q | K | P | G | K | A | P |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | . | . | . | . | . | . | . | . |
| 21-225_50F3_LC | . | . | D | . | . | . | . | . | . | . | . | . | S | . | . | . | . | F | . | . | . | . | . | . | . | . |
| 21-225_51C7_LC | . | . | D | V | N | . | . | . | . | . | . | . | N | Y | . | . | . | F | . | . | . | . | . | . | . | . |
| 21-225_51E9_LC | . | . | . | . | . | . | . | . | . | . | . | . | S | . | . | . | . | . | . | . | . | . | . | . | . | . |
| 21-225_52B1_LC | . | . | D | . | . | R | . | . | . | . | . | . | T | . | . | . | . | . | . | . | . | . | . | . | . | . |
| 21-225_52G11_LC | . | . | . | . | . | . | . | . | . | . | . | . | K | . | . | . | . | . | . | . | . | . | . | . | . | . |
| 21-225_52H10_LC | . | . | D | . | N | . | . | . | . | . | . | . | S | . | . | . | . | . | . | . | . | . | . | . | . | . |
| 21-225_53F2_LC | . | . | . | . | . | . | . | . | . | . | . | . | K | . | . | . | . | . | . | . | . | . | . | . | . | . |
| 21-225_53F7_LC | . | . | . | . | . | . | . | . | . | . | . | . | . | . | . | . | . | . | . | . | . | . | . | . | . | . |
| 21-225_55B1_LC | . | . | . | V | . | . | . | . | . | . | . | . | . | . | . | . | . | . | . | . | . | . | . | . | . | . |
| 21-225_56C6_LC | . | . | . | . | . | . | . | . | . | . | . | . | S | . | . | . | . | . | . | . | . | S | . | N | . | . |
| 21-225_56E5_LC | . | . | . | . | . | R | . | . | . | . | . | . | K | . | . | . | . | . | . | . | . | . | . | . | . | . |
| 21-225_56F6_LC | . | . | D | . | . | . | . | . | . | . | . | . | K | . | . | . | . | F | . | . | . | . | . | . | . | . |
| 21-225_57A7_LC | . | . | . | . | . | . | . | . | . | . | . | . | . | . | . | . | . | . | . | . | . | . | . | . | . | . |
| 21-225_57E4_LC | . | . | D | . | . | . | . | . | . | . | . | . | N | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | N | . | T | . | . | . | . | . | . | . | T | . | . | . | . | . | . | . | . | . | . | . | . | . |
| 21-225_65F10_LC | . | . | . | . | . | . | . | . | . | . | . | . | T | . | . | . | . | . | . | . | . | . | . | . | . | . |
| 21-225_66F11_LC | . | . | . | . | . | . | . | . | . | . | . | . | I | . | . | . | . | . | . | . | . | . | . | . | . | . |
| 21-225_70D1_LC | . | . | . | V | . | . | . | . | . | . | . | . | . | . | . | . | . | . | . | . | . | . | . | . | . | . |
| 21-225_7C3_LC2_LC | . | . | . | . | . | . | . | . | . | . | . | . | S | . | . | . | . | . | . | . | Q | . | . | . | . | . |
| 21-225_9F12_LC1_LC | . | . | . | . | . | . | . | . | . | . | . | . | . | . | . | . | . | . | . | . | . | . | . | . | . | . |

4704

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_10G6_LC | . | ¦ | . | . | . | . | . | . | . | . | . | . | . | . | . | . | Y | . | . | . | . | . | . | A | . | . |
| 21-225_12E9_LC | . | . | . | . | . | . | . | . | . | . | . | . | . | . | . | ◌ | . | . | . | . | . | . | . | . | . | . |
| 21-225_146A6_LC | . | . | . | . | . | . | . | . | . | . | . | . | . | . | . | . | . | . | . | ◌ | . | . | . | . | . | . |
| 21-225_146E1_LC | . | . | . | . | . | . | . | . | . | . | . | . | . | . | . | . | . | . | . | . | . | . | . | . | . | . |
| 21-225_147D10_LC | . | . | . | . | . | . | . | . | . | . | . | . | . | . | . | . | . | . | . | . | . | . | . | . | . | . |
| 21-225_147D12_LC | . | . | . | . | . | . | . | . | . | . | . | . | . | . | . | . | . | . | . | . | . | . | . | . | . | . |
| 21-225_148E2_LC | . | . | . | . | . | . | . | . | . | . | . | . | . | . | . | . | . | . | . | . | . | . | . | . | . | . |
| 21-225_149B6_LC | . | . | . | . | . | . | . | . | . | . | . | . | . | . | . | . | . | . | . | . | . | . | . | . | . | . |
| 21-225_149C6_LC | . | . | . | . | . | . | . | . | . | . | . | . | . | . | . | . | . | . | . | ◌ | . | . | . | . | . | . |
| 21-225_149D11_LC | . | . | . | . | . | . | . | . | . | . | . | . | . | . | . | . | . | . | . | . | . | . | . | . | . | . |
| 21-225_149F8_LC | . | . | . | . | . | . | . | . | . | . | . | . | . | . | . | . | . | . | . | . | . | . | . | . | . | . |
| 21-225_150C5_LC | . | . | . | . | . | . | . | . | . | . | . | . | . | . | . | . | . | . | . | ◌ | . | . | . | . | . | . |
| 21-225_152G10_LC | . | . | . | S | . | . | . | . | . | . | . | . | . | . | . | . | . | . | . | . | . | . | . | . | . | . |
| 21-225_152H7_LC | . | . | . | . | . | . | . | . | . | . | . | . | . | . | . | . | . | . | . | ◌ | . | . | . | . | . | . |
| 21-225_155A4_LC | . | . | . | . | . | . | . | . | . | . | . | . | . | . | . | . | . | . | . | ◌ | . | . | . | . | . | . |
| 21-225_155B4_LC | . | . | . | . | . | . | . | . | . | . | . | . | . | . | . | . | . | . | . | ◌ | . | . | . | . | . | . |
| 21-225_180A3_LC | . | . | . | . | . | . | . | . | . | . | . | . | . | . | . | . | . | . | . | . | . | . | . | . | . | . |
| 21-225_182H5_LC | . | . | . | . | . | . | . | . | . | . | . | . | . | . | . | . | . | . | . | . | . | . | . | . | . | . |
| 21-225_186A11_LC | . | . | . | . | . | . | . | . | . | . | . | . | . | . | . | . | . | . | . | . | . | . | . | . | . | . |
| 21-225_192H2_LC | . | . | . | . | ◌ | . | . | . | . | . | . | . | . | . | . | . | . | . | . | . | . | . | . | . | . | . |
| 21-225_196F2_LC | . | . | . | . | . | . | . | . | . | . | . | . | . | . | . | . | . | . | . | . | . | . | . | . | . | . |
| 21-225_210H11_LC | N | . | . | . | . | . | . | . | . | . | . | . | . | . | . | . | . | . | . | . | . | . | . | . | . | . |
| 21-225_221A6_LC | . | . | . | . | . | T | . | . | . | . | . | . | . | . | . | N | . | . | . | . | . | . | . | . | . | . |
| 21-225_221F12_LC | . | . | . | . | . | T | . | . | . | . | . | . | . | . | . | N | . | . | . | . | . | . | . | . | . | . |
| 21-225_222C12_LC | . | . | V | . | . | T | . | . | . | . | . | . | . | . | . | N | . | . | . | . | . | . | . | . | . | . |
| 21-225_222E1_LC | . | . | . | . | . | T | . | . | . | . | . | . | . | . | . | N | . | . | . | . | . | . | . | . | . | . |
| 21-225_222F7_LC | . | . | . | . | . | T | . | . | . | . | . | . | . | . | . | N | . | . | . | . | . | . | . | . | . | . |
| 21-225_23B11_LC | . | V | . | . | . | . | . | . | . | . | . | . | . | . | . | . | . | . | . | . | ◌ | . | . | . | . | . |
| 21-225_25E11_LC | . | . | . | . | . | T | . | . | . | . | . | . | . | . | . | . | . | . | . | ◌ | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_28B1_LC | . | . | . | . | . | . | . | . | . | . | . | . | . | . | . | . | . | P | . | . | . | . | . | . | . | . |
| 21-225_28F11_LC | . | . | . | . | S | . | . | . | . | . | . | . | . | . | . | . | N | . | . | . | . | . | . | . | . | . |
| 21-225_30A11_LC | S | . | . | . | . | . | . | . | . | . | . | . | . | . | . | . | R | . | . | . | . | . | S | . | . | . |
| 21-225_31C4_LC | . | P | . | . | . | . | . | . | . | . | . | . | . | . | . | . | . | . | . | . | . | . | . | T | . | . |
| 21-225_31H9_LC | . | . | . | . | . | D | . | . | . | . | . | . | . | . | T | . | . | . | . | . | . | . | . | . | . | . |
| 21-225_33G1_LC | . | . | . | . | . | G | . | . | . | . | . | . | . | . | . | Y | . | . | . | . | . | . | . | . | . | . |
| 21-225_33H7_LC | . | . | . | . | . | . | . | . | . | . | . | . | . | . | . | . | R | . | . | . | . | . | . | . | . | . |
| 21-225_34F3_LC | . | . | . | . | . | . | . | . | . | . | . | . | . | . | . | . | . | . | . | . | . | . | . | . | . | . |
| 21-225_34G7_LC | . | . | . | . | . | . | . | . | . | . | . | . | . | . | . | . | . | . | . | . | . | . | . | L | . | . |
| 21-225_35C6_LC | . | P | . | . | . | . | . | . | . | . | . | . | . | . | . | . | . | . | . | . | . | . | . | T | . | . |
| 21-225_35G6_LC | . | . | . | . | . | . | . | . | . | . | . | . | . | . | . | . | R | . | . | . | . | . | . | . | . | . |
| 21-225_35H12_LC | S | . | . | . | . | . | . | . | . | . | . | . | . | . | . | . | R | . | . | . | I | . | . | . | . | . |
| 21-225_36H5_LC | S | . | . | . | . | . | . | . | . | . | . | . | . | . | . | . | R | . | . | . | I | . | . | . | . | . |
| 21-225_43H9_LC | . | . | . | . | . | D | . | . | . | . | . | . | . | . | . | . | . | . | . | . | . | . | . | . | . | . |
| 21-225_44F3_LC | . | . | . | . | . | . | . | . | . | . | . | . | . | . | . | . | . | . | . | . | . | . | . | . | . | . |
| 21-225_45C9_LC | . | . | . | . | . | . | . | . | . | . | . | . | . | . | . | . | . | . | R | . | . | . | . | . | . | . |
| 21-225_45H4_LC | . | Y | . | . | . | D | . | . | . | . | . | . | . | . | . | . | . | . | . | . | . | . | . | . | . | . |
| 21-225_49F1_LC | . | . | . | . | . | T | . | . | . | . | . | . | . | . | V | . | . | . | . | . | . | . | . | . | . | . |
| 21-225_49F10_LC | . | V | . | . | . | . | . | . | . | . | . | . | . | . | . | . | . | T | . | . | . | . | . | . | . | . |
| 21-225_49H5_LC | . | . | . | . | . | . | . | . | . | . | . | . | . | . | . | . | . | . | . | D | . | . | . | . | . | . |
| 21-225_4H6_LC | . | . | . | . | . | G | . | . | . | . | . | . | . | . | . | . | . | . | . | . | . | . | . | . | . | . |
| 21-225_4H6.004_LC | . | . | . | . | . | G | . | . | . | . | . | . | . | . | . | . | . | . | . | . | . | . | . | . | . | . |
| 21-225_4H6.005_LC | . | . | . | . | . | G | . | . | . | . | . | . | . | . | . | . | . | . | . | . | . | . | . | . | . | . |
| 21-225_4H6.006_LC | . | . | . | . | . | G | . | . | . | . | . | . | . | . | . | . | . | . | . | . | . | . | . | . | . | . |
| 21-225_4H6.007_LC | . | . | . | . | . | G | . | . | . | . | . | . | . | . | . | . | . | . | . | . | . | . | . | . | . | . |
| 21-225_4H6.008_LC | . | . | . | . | . | G | . | . | . | . | . | . | . | . | . | . | . | . | . | . | . | . | . | . | . | . |
| 21-225_4H6.009_LC | . | . | . | . | . | G | . | . | . | . | . | . | . | . | . | . | . | . | . | . | . | . | . | . | . | . |
| 21-225_4H6.010_LC | . | . | . | . | . | G | . | . | . | . | . | . | . | . | . | . | . | . | . | . | . | . | . | . | . | . |
| 21-225_4H6.011_LC | . | . | . | . | . | G | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_4H6.014_LC | . | . | . | . | . | S | . | . | . | . | . | . | . | . | . | . | . | . | . | . | . | . | . | . | . | . |
| 21-225_50D4_LC | . | V | . | . | . | . | . | . | . | . | . | . | . | . | . | . | T | . | . | . | . | . | . | . | . | . |
| 21-225_50F3_LC | . | . | . | . | . | . | . | . | . | . | . | . | . | . | . | . | . | . | . | N | . | . | . | . | . | . |
| 21-225_51C7_LC | . | . | . | . | . | . | . | . | . | . | . | . | . | . | . | . | . | . | . | N | . | . | . | . | . | . |
| 21-225_51E9_LC | . | . | . | . | . | V | . | . | . | . | . | . | . | . | . | . | . | . | . | . | . | . | . | . | . | . |
| 21-225_52B1_LC | . | . | . | . | . | . | . | . | . | . | . | . | . | . | . | . | . | . | . | N | . | . | . | . | . | . |
| 21-225_52G11_LC | . | . | . | L | . | . | . | . | . | . | . | . | . | . | . | . | . | . | . | . | . | . | . | . | . | . |
| 21-225_52H10_LC | . | . | . | . | . | V | . | . | . | . | . | . | . | . | . | . | . | . | . | . | . | A | . | . | . | . |
| 21-225_53F2_LC | N | . | . | L | . | . | . | . | . | . | . | . | . | . | . | . | . | . | . | . | . | . | . | . | X | . |
| 21-225_53F7_LC | N | . | . | . | . | . | . | . | . | . | . | . | . | . | . | . | . | . | . | . | . | . | . | . | . | . |
| 21-225_55B1_LC | N | . | . | . | . | . | . | . | . | . | . | . | . | . | . | . | . | . | . | . | . | . | . | . | . | . |
| 21-225_56C6_LC | . | . | . | . | . | . | . | . | . | . | . | . | . | . | . | . | R | . | . | . | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | F | . | . | . | V | . | . | . | . | . | . | . | . | . | . | G | . | . | . | . | . | . | . | . | . |
| 21-225_57A7_LC | N | . | . | . | . | . | . | . | . | . | . | . | . | . | . | . | N | . | . | . | . | . | . | . | . | . |
| 21-225_57E4_LC | . | . | . | . | . | . | . | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | . | S | . | . | . | . | . | . | . | . | . | . | . | . | . | . | . | . | . | . | . | . | . |
| 21-225_65F10_LC | . | . | . | . | . | . | . | . | . | . | . | . | . | . | . | . | I | . | . | . | . | . | . | . | . | . |
| 21-225_66F11_LC | . | . | . | . | . | . | . | . | . | . | . | . | . | . | . | . | . | . | . | . | . | . | . | . | . | . |
| 21-225_70D1_LC | . | . | . | . | . | . | . | . | . | . | . | . | . | . | . | . | . | . | . | . | . | . | . | . | . | . |
| 21-225_7C3_LC2_LC | . | . | . | . | F | . | . | . | . | . | . | . | . | . | . | . | R | . | . | . | . | A | . | . | . | . |
| 21-225_9F12_LC1_LC | . | . | . | . | . | . | . | . | . | . | . | . | . | . | . | . | R | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_10G6_LC | . | | | | . | . | . | . | . | . | . | . | . | . | S | . | . | . | . | . | . | . | . | . | | |
| 21-225_12E9_LC | . | | | | . | . | . | . | . | . | . | . | . | . | . | . | . | . | . | . | . | . | . | . | . | |
| 21-225_146A6_LC | . | | | | . | . | . | . | . | . | . | . | . | S | . | . | . | . | . | . | . | . | . | . | . | |
| 21-225_146E1_LC | N | | | | . | . | . | . | . | . | . | . | . | . | . | . | . | . | . | . | . | . | . | . | . | |
| 21-225_147D10_LC | . | | | | . | . | . | . | . | . | . | . | . | S | . | . | . | . | . | . | . | . | . | . | . | |
| 21-225_147D12_LC | . | | | | . | . | . | . | . | . | . | . | . | S | . | . | . | . | . | . | . | . | . | . | . | |
| 21-225_148E2_LC | . | | N | S | . | . | . | . | . | . | . | . | . | S | . | . | . | . | . | . | . | . | . | . | . | |
| 21-225_149B6_LC | . | | | | . | . | . | . | . | . | . | . | . | . | . | . | . | . | . | . | . | . | . | . | . | |
| 21-225_149C6_LC | . | | | | . | . | . | . | . | Y | . | . | . | S | . | . | . | . | . | . | . | . | . | . | . | |
| 21-225_149D11_LC | . | | | | . | . | . | . | . | . | . | . | . | S | . | . | . | . | . | . | . | . | . | . | . | |
| 21-225_149F8_LC | . | | N | S | . | . | . | . | . | . | . | . | . | S | . | . | . | . | . | . | . | . | . | I | . | |
| 21-225_150C5_LC | . | | | | . | . | . | . | . | . | . | . | . | S | . | . | . | . | . | . | . | . | . | . | . | |
| 21-225_152G10_LC | . | | N | S | . | . | . | . | . | . | . | . | . | S | . | . | . | . | . | . | . | . | . | . | . | |
| 21-225_152H7_LC | . | | | | . | . | . | . | . | . | . | . | . | S | . | . | T | . | . | . | . | . | . | . | . | |
| 21-225_155A4_LC | . | | | | . | . | . | . | . | . | . | . | . | S | . | . | . | . | . | . | . | . | . | . | . | |
| 21-225_155B4_LC | N | | | | . | . | . | . | . | . | . | . | . | S | . | . | . | . | . | . | . | . | . | . | . | |
| 21-225_180A3_LC | . | | | | . | . | . | . | . | . | I | . | . | . | . | . | . | . | . | . | . | . | . | . | S | |
| 21-225_182H5_LC | . | | | | . | . | . | . | . | . | I | . | . | . | . | T | . | V | . | . | Q | . | . | . | . | |
| 21-225_186A11_LC | . | | | | . | . | . | . | . | . | I | . | . | . | . | T | . | V | . | . | Q | . | . | . | . | |
| 21-225_192H2_LC | . | | | | . | . | . | . | . | . | . | . | . | . | . | . | . | . | . | . | . | . | . | . | . | |
| 21-225_196F2_LC | . | | | | . | . | . | . | . | . | . | . | . | . | . | . | . | . | . | . | . | . | . | . | . | |
| 21-225_210H11_LC | . | | R | | . | . | . | . | . | . | . | . | . | . | . | . | . | . | . | . | . | . | . | . | . | |
| 21-225_221A6_LC | . | | | | . | . | . | . | . | . | . | . | . | . | . | . | . | . | . | . | . | . | . | . | . | |
| 21-225_221F12_LC | . | | | | . | . | . | . | . | . | . | . | . | . | . | . | . | . | . | S | . | . | . | . | . | |
| 21-225_222C12_LC | . | | | | . | . | . | . | . | . | . | . | . | . | . | . | . | V | . | S | . | . | . | . | . | |
| 21-225_222E1_LC | . | | | | . | . | . | . | . | . | . | . | . | . | . | . | . | . | . | . | . | . | . | . | . | |
| 21-225_222F7_LC | . | | | | . | . | . | . | . | . | . | . | . | . | . | . | . | V | . | . | . | . | . | . | . | |
| 21-225_23B11_LC | . | | | R | . | . | . | . | . | . | . | . | . | . | . | . | . | . | . | . | . | . | . | . | . | |
| 21-225_25E11_LC | . | | | | . | . | . | . | . | . | . | I | S | . | . | . | . | . | . | . | . | . | . | . | . | |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_28B1_LC | . | . | . | . | . | . | . | . | . | . | . | . | . | . | . | T | . | . | . | . | . | . | . | . | . | . |
| 21-225_28F11_LC | R | . | . | . | . | . | . | . | . | . | . | . | . | . | . | T | . | . | . | . | . | . | . | . | . | . |
| 21-225_30A11_LC | . | . | . | . | . | . | . | . | . | . | . | . | . | . | . | . | . | . | . | . | . | . | . | . | . | . |
| 21-225_31C4_LC | . | . | . | . | . | . | . | . | . | . | . | . | . | . | . | . | . | . | . | . | . | . | . | . | S | . |
| 21-225_31H9_LC | . | . | . | . | . | . | . | . | . | . | S | F | . | . | . | . | . | . | . | . | . | . | . | . | . | . |
| 21-225_33G1_LC | . | . | . | . | . | . | . | . | . | . | . | . | . | . | . | . | . | . | . | . | . | . | . | . | . | . |
| 21-225_33H7_LC | . | . | . | . | . | . | . | . | . | . | . | . | . | . | . | G | . | . | . | . | . | . | . | . | . | . |
| 21-225_34F3_LC | . | . | . | . | . | . | . | . | . | . | . | . | . | . | . | . | . | . | . | . | . | . | S | . | . | . |
| 21-225_34G7_LC | . | . | . | . | . | . | . | . | . | . | . | . | . | . | . | . | . | . | . | . | . | . | . | . | . | . |
| 21-225_35C6_LC | . | . | . | . | . | . | . | . | . | . | . | . | . | . | . | . | . | . | . | . | . | . | . | . | S | . |
| 21-225_35G6_LC | . | . | . | . | . | . | . | . | . | A | . | . | . | . | . | G | . | . | . | . | . | . | . | . | . | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | . | . | . | . | . | . | . | . | . | . | . | S | . | . | . | . | . | . | . | . | . | . | . | . | . | . |
| 21-225_44F3_LC | . | . | . | . | S | . | . | . | . | . | . | . | . | . | . | . | . | . | . | . | . | . | . | . | . | . |
| 21-225_45C9_LC | . | . | . | . | . | . | . | . | . | . | . | . | . | . | . | . | . | . | . | . | . | . | . | . | . | . |
| 21-225_45H4_LC | . | . | . | . | . | . | . | . | . | . | . | S | . | . | . | . | . | . | . | . | . | . | . | . | S | . |
| 21-225_49F1_LC | . | . | . | . | . | . | . | . | . | . | . | . | . | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | . | . | . | . | . | . | . | . | . | . | . | . | . | . | . | . | . | . | . | . | . | . |
| 21-225_4H6_LC | . | . | . | . | . | . | . | . | . | . | . | . | . | . | . | . | . | . | . | . | . | . | . | . | S | . |
| 21-225_4H6.004_LC | . | . | . | . | . | . | . | . | . | . | . | . | . | . | . | . | . | . | . | . | . | . | . | . | S | . |
| 21-225_4H6.005_LC | . | . | . | . | . | . | . | . | . | . | . | . | . | . | . | . | . | . | . | . | . | . | . | . | S | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | S | . |
| 21-225_4H6.008_LC | . | . | . | . | . | . | . | . | . | . | . | . | . | . | . | . | . | . | . | . | . | . | . | . | S | . |
| 21-225_4H6.009_LC | . | . | . | . | . | . | . | . | . | . | . | . | . | . | . | . | . | . | . | . | . | . | . | . | S | . |
| 21-225_4H6.010_LC | . | . | . | . | . | . | . | . | . | . | . | . | . | . | . | . | . | . | . | . | . | . | . | . | S | . |
| 21-225_4H6.011_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | . | . | . | . | . | . | . | . |
| 21-225_50F3_LC | . | . | . | . | . | . | . | . | . | . | . | . | . | . | . | . | . | . | . | . | . | . | . | . | . | . |
| 21-225_51C7_LC | . | . | . | . | . | . | . | . | . | . | . | . | . | . | . | . | . | . | L | . | . | . | . | . | . | . |
| 21-225_51E9_LC | . | . | . | . | . | . | . | . | . | . | . | . | . | . | . | R | . | . | . | . | . | . | . | . | . | . |
| 21-225_52B1_LC | . | . | . | . | . | . | . | . | . | . | . | . | . | . | . | . | . | . | . | . | . | . | . | . | . | . |
| 21-225_52G11_LC | . | A | . | . | . | . | . | . | . | . | . | . | . | . | . | . | . | . | . | . | . | . | . | . | . | . |
| 21-225_52H10_LC | . | . | . | . | . | . | . | . | . | . | . | . | . | . | . | R | . | . | . | . | . | . | . | . | . | . |
| 21-225_53F2_LC | . | . | . | . | . | . | . | . | . | . | . | . | . | . | . | . | . | . | . | . | . | . | . | . | . | . |
| 21-225_53F7_LC | . | . | . | . | . | . | . | . | . | . | . | . | . | . | . | . | . | . | . | . | . | . | . | . | I | . |
| 21-225_55B1_LC | . | . | . | . | . | . | . | . | . | . | . | . | . | . | . | . | . | . | . | . | . | . | . | . | . | . |
| 21-225_56C6_LC | . | . | . | . | . | . | . | . | Y | . | . | . | I | . | . | . | . | . | . | S | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | . | . | . | . | . | . | . | . | . | . | F | . | . | . | . | . | . | . | . | . | . | . | . | . | . |
| 21-225_57A7_LC | . | . | . | . | . | . | . | . | . | . | . | . | . | . | . | . | . | . | . | . | . | . | . | . | . | . |
| 21-225_57E4_LC | . | . | . | . | . | . | . | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | . | . | . | . | . | . | . | . | . | . | . | . | . | . | . | . | . | . | . | . | . | . | . |
| 21-225_65F10_LC | R | . | R | . | . | . | . | . | . | . | . | . | . | . | . | . | . | . | . | . | . | . | . | . | . | . |
| 21-225_66F11_LC | . | . | . | . | Y | . | . | . | . | . | . | . | . | . | . | . | . | . | V | . | . | . | . | . | . | . |
| 21-225_70D1_LC | . | . | . | . | . | . | . | . | . | . | . | . | V | . | . | . | . | . | . | . | . | . | . | . | . | . |
| 21-225_7C3_LC2_LC | . | . | . | . | . | . | . | . | . | . | . | . | . | . | . | . | . | . | . | . | . | . | . | . | . | . |
| 21-225_9F12_LC1_LC | . | . | . | . | . | . | . | . | . | . | . | . | . | . | . | . | R | . | L | . | . | . | . | . | I | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | A | N | S | - | - | - | - | - | - | - | - | - | - | - | - | - | - | - | - | - | - | - |
| 21-225_10G6_LC | . | | | | | | | | | | | | | | | | | | | | | | | | | |
| 21-225_12E9_LC | . | | | | . | . | | | | | | | | | | | | | | | | | | | | |
| 21-225_146A6_LC | . | | | . | T | D | . | . | . | . | . | . | . | . | . | . | . | . | . | . | . | . | . | . | . | . |
| 21-225_146E1_LC | . | | | | . | . | | | | | | | | | | | | | | | | | | | | |
| 21-225_147D10_LC | . | | | | T | D | . | . | . | . | . | . | . | . | . | . | . | . | . | . | . | . | . | . | . | . |
| 21-225_147D12_LC | . | | | | T | D | . | . | . | . | . | . | . | . | . | . | . | . | . | . | . | . | . | . | . | . |
| 21-225_148E2_LC | . | | | | T | D | . | . | . | . | . | . | . | . | . | . | . | . | . | . | . | . | . | . | . | . |
| 21-225_149B6_LC | . | | | . | S | . | . | . | . | . | . | . | . | . | . | . | . | . | . | . | . | . | . | . | . | . |
| 21-225_149C6_LC | . | | | . | T | D | . | . | . | . | . | . | . | . | . | . | . | . | . | . | . | . | . | . | . | . |
| 21-225_149D11_LC | . | | | . | T | D | . | . | . | . | . | . | . | . | . | . | . | . | . | . | . | . | . | . | . | . |
| 21-225_149F8_LC | . | | | . | T | D | . | . | . | . | . | . | . | . | . | . | . | . | . | . | . | . | . | . | . | . |
| 21-225_150C5_LC | . | | | . | T | D | . | . | . | . | . | . | . | . | . | . | . | . | . | . | . | . | . | . | . | . |
| 21-225_152G10_LC | . | | | . | T | D | . | . | . | . | . | . | . | . | . | . | . | . | . | . | . | . | . | . | . | . |
| 21-225_152H7_LC | . | | . | . | T | D | . | . | . | . | . | . | . | . | . | . | . | . | . | . | . | . | . | . | . | . |
| 21-225_155A4_LC | . | . | X | . | T | D | . | . | . | . | . | . | . | . | . | . | . | . | . | . | . | . | . | . | . | . |
| 21-225_155B4_LC | . | . | X | . | T | D | . | . | . | . | . | . | . | . | . | . | . | . | . | . | . | . | . | . | . | . |
| 21-225_180A3_LC | X | . | . | . | . | . | . | . | . | . | . | . | . | . | . | . | . | . | . | . | . | . | . | . | . | . |
| 21-225_182H5_LC | . | . | . | . | V | . | . | . | . | . | . | . | . | . | . | . | . | . | . | . | . | . | . | . | . | . |
| 21-225_186A11_LC | . | . | . | . | V | . | . | . | . | . | . | . | . | . | . | . | . | . | . | . | . | . | . | . | . | . |
| 21-225_192H2_LC | . | . | . | . | S | . | . | . | . | . | . | . | . | . | . | . | . | . | . | . | . | . | . | . | . | . |
| 21-225_196F2_LC | . | . | . | . | T | . | . | . | . | . | . | . | . | . | . | . | . | . | . | . | . | . | . | . | . | . |
| 21-225_210H11_LC | . | . | . | . | X | . | . | . | . | . | . | . | . | . | . | . | . | . | . | . | . | . | . | . | . | . |
| 21-225_221A6_LC | . | . | . | . | . | | | | | | | | | | | | | | | | | | | | | |
| 21-225_221F12_LC | . | . | . | . | . | | | | | | | | | | | | | | | | | | | | | |
| 21-225_222C12_LC | . | . | . | . | D | . | . | . | . | . | . | . | . | . | . | . | . | . | . | . | . | . | . | . | . | . |
| 21-225_222E1_LC | . | . | . | . | . | | | | | | | | | | | | | | | | | | | | | |
| 21-225_222F7_LC | . | . | . | . | . | | | | | | | | | | | | | | | | | | | | | |
| 21-225_23B11_LC | . | . | . | . | . | | | | | | | | | | | | | | | | | | | | | |
| 21-225_25E11_LC | . | . | . | . | . | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | A | N | S | - | - | - | - | - | - | - | - | - | - | - | - | - | - | - | - | - | - | - |
| 21-225_28B1_LC | . | . | . | . | . | S | . | . | . | . | . | . | . | . | . | . | . | . | . | . | . | . | . | . | . | . |
| 21-225_28F11_LC | C | . | . | . | . | . | . | . | . | . | . | . | . | . | . | . | . | . | . | . | . | . | . | . | . | . |
| 21-225_30A11_LC | . | . | . | . | . | . | . | . | . | . | . | . | . | . | . | . | . | . | . | . | . | . | . | . | . | . |
| 21-225_31C4_LC | . | . | . | . | . | . | . | . | . | . | . | . | . | . | . | . | . | . | . | . | . | . | . | . | . | . |
| 21-225_31H9_LC | . | . | . | . | G | . | . | . | . | . | . | . | . | . | . | . | . | . | . | . | . | . | . | . | . | . |
| 21-225_33G1_LC | . | . | . | . | . | . | . | . | . | . | . | . | . | . | . | . | . | . | . | . | . | . | . | . | . | . |
| 21-225_33H7_LC | . | . | . | . | . | I | . | . | . | . | . | . | . | . | . | . | . | . | . | . | . | . | . | . | . | . |
| 21-225_34F3_LC | . | . | . | . | . | . | . | . | . | . | . | . | . | . | . | . | . | . | . | . | . | . | . | . | . | . |
| 21-225_34G7_LC | . | . | . | . | . | . | . | . | . | . | . | . | . | . | . | . | . | . | . | . | . | . | . | . | . | . |
| 21-225_35C6_LC | . | . | . | . | . | . | . | . | . | . | . | . | . | . | . | . | . | . | . | . | . | . | . | . | . | . |
| 21-225_35G6_LC | . | . | . | . | . | . | . | . | . | . | . | . | . | . | . | . | . | . | . | . | . | . | . | . | . | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | F | . | . | . | . | . | . | . | . | . | . | . | . | . | . | . | . | . | . | . | . | . | . | . | . | . |
| 21-225_44F3_LC | . | . | . | . | . | . | . | . | . | . | . | . | . | . | . | . | . | . | . | . | . | . | . | . | . | . |
| 21-225_45C9_LC | . | . | . | . | . | . | . | . | . | . | . | . | . | . | . | . | . | . | . | . | . | . | . | . | . | . |
| 21-225_45H4_LC | F | . | . | . | . | . | . | . | . | . | . | . | . | . | . | . | . | . | . | . | . | . | . | . | . | . |
| 21-225_49F1_LC | . | . | . | . | S | . | . | . | . | . | . | . | . | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | T | . | . | . | . | . | . | . | . | . | . | . | . | . | . | . | . | . | . | . | . | . |
| 21-225_4H6_LC | . | . | . | . | . | . | . | . | . | . | . | . | . | . | . | . | . | . | . | . | . | . | . | . | . | . |
| 21-225_4H6.004_LC | . | . | . | . | . | . | . | . | . | . | . | . | . | . | . | . | . | . | . | . | . | . | . | . | . | . |
| 21-225_4H6.005_LC | . | . | . | . | . | . | . | . | . | . | . | . | . | . | . | . | . | . | . | . | . | . | . | . | . | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | . | . |
| 21-225_4H6.008_LC | . | . | . | . | . | . | . | . | . | . | . | . | . | . | . | . | . | . | . | . | . | . | . | . | . | . |
| 21-225_4H6.009_LC | . | . | . | . | . | . | . | . | . | . | . | . | . | . | . | . | . | . | . | . | . | . | . | . | . | . |
| 21-225_4H6.010_LC | . | . | . | . | . | . | . | . | . | . | . | . | . | . | . | . | . | . | . | . | . | . | . | . | . | . |
| 21-225_4H6.011_LC | . | . | . | . | . | . | . | . | . | . | . | . | . | . | . | . | . | . | . | . | . | . | . | . | . | . |

## FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | A | N | S | - | - | - | - | - | - | - | - | - | - | - | - | - | - | - | - | - | - | - |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | . | . | . | . | . | . | . | . |
| 21-225_50F3_LC | . | . | . | . | T | . | . | . | . | . | . | . | . | . | . | . | . | . | . | . | . | . | . | . | . | . |
| 21-225_51C7_LC | . | . | . | . | T | . | . | . | . | . | . | . | . | . | . | . | . | . | . | . | . | . | . | . | . | . |
| 21-225_51E9_LC | . | . | . | . | . | K | . | . | . | . | . | . | . | . | . | . | . | . | . | . | . | . | . | . | . | . |
| 21-225_52B1_LC | F | . | . | . | . | K | . | . | . | . | . | . | . | . | . | . | . | . | . | . | . | . | . | . | . | . |
| 21-225_52G11_LC | . | . | . | . | . | . | . | . | . | . | . | . | . | . | . | . | . | . | . | . | . | . | . | . | . | . |
| 21-225_52H10_LC | . | . | . | . | . | K | . | . | . | . | . | . | . | . | . | . | . | . | . | . | . | . | . | . | . | . |
| 21-225_53F2_LC | . | . | . | . | . | . | . | . | . | . | . | . | . | . | . | . | . | . | . | . | . | . | . | . | . | . |
| 21-225_53F7_LC | . | . | . | . | . | . | . | . | . | . | . | . | . | . | . | . | . | . | . | . | . | . | . | . | . | . |
| 21-225_55B1_LC | . | . | . | . | . | . | . | . | . | . | . | . | . | . | . | . | . | . | . | . | . | . | . | . | . | . |
| 21-225_56C6_LC | . | . | . | . | . | . | . | . | . | . | . | . | . | . | . | . | . | . | . | . | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | . | . | . | . | . | . | . | . | . | . | . | . | . | . | . | . | . | . | . | . | . | . | . | . | . |
| 21-225_57A7_LC | . | . | . | . | . | . | . | . | . | . | . | . | . | . | . | . | . | . | . | . | . | . | . | . | . | . |
| 21-225_57E4_LC | S | . | . | . | Q | . | . | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | I | . | . | . | . | . | . | . | . | . | . | . | . | . | . | . | . | . | . | . | . | . | . | . | . | . |
| 21-225_65F10_LC | . | . | . | . | Q | . | I | . | . | . | . | . | . | . | . | . | . | . | . | . | . | . | . | . | . | . |
| 21-225_66F11_LC | . | . | . | . | . | . | . | . | . | . | . | . | . | . | . | . | . | . | . | . | . | . | . | . | . | . |
| 21-225_70D1_LC | . | . | . | . | . | . | . | . | . | . | . | . | . | . | . | . | . | . | . | . | . | . | . | . | . | . |
| 21-225_7C3_LC2_LC | . | . | . | . | . | . | . | . | . | . | . | . | . | . | . | . | . | . | . | . | . | . | . | . | . | . |
| 21-225_9F12_LC1_LC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | F | P | F | T | F | G | P | G | T | K | V | D | I | K | R |
| 21-225_10G6_LC | | | | | | | | | | | | | | | | | | | |
| 21-225_12E9_LC | | | | | | | | | | | | | | | | | | | |
| 21-225_146A6_LC | | | | | | | | | | | | | | | | | | | |
| 21-225_146E1_LC | | | | | | | | | | | | | | | | | | | |
| 21-225_147D10_LC | | | | | | | | | | | | | | | | | V | | |
| 21-225_147D12_LC | | | | | | | | | | | | | | | | | V | | |
| 21-225_148E2_LC | | | | | | | | | | | | | | | | | V | | |
| 21-225_149B6_LC | | | | | | | | | | | | | | | | | | | |
| 21-225_149C6_LC | | | | | | | | | | | | | | | | | | | |
| 21-225_149D11_LC | | | | | | | | | | | | | | | | | V | | |
| 21-225_149F8_LC | | | | | | | | | | | | | | | | | V | | |
| 21-225_150C5_LC | | | | | | | | | | | | | | | | | | | |
| 21-225_152G10_LC | | | | | | | | | | | | | | | | | V | | |
| 21-225_152H7_LC | | | | | | | | | | | | | | | | | | | |
| 21-225_155A4_LC | | | | | | | | | | | | | | | | | | | |
| 21-225_155B4_LC | | | | | | | | | | | | | | | | | | | |
| 21-225_180A3_LC | | | | | | | | | | | | | | | | | | N | |
| 21-225_182H5_LC | | | | | | | | | | | | | | | | | | | |
| 21-225_186A11_LC | | | | | | | | | | | | | | | | | | | |
| 21-225_192H2_LC | | | | | | | | | | | | | | | | | V | | |
| 21-225_196F2_LC | | | | V | | | | | | | | | | | | | | | |
| 21-225_210H11_LC | | | | | | | | | | | | | | | | | | | |
| 21-225_221A6_LC | | | | | | | | | | | | | | | | | | | |
| 21-225_221F12_LC | | | | | | | | | | | | | | | | | | | |
| 21-225_222C12_LC | | | | | | | | | | | | | | | | | | | |
| 21-225_222E1_LC | | | | | | | | | | | | | | | | | | | |
| 21-225_222F7_LC | | | | | | | | | | | | | | | | | | | |
| 21-225_23B11_LC | | | | | | | | | | | | | | | | | | | |
| 21-225_25E11_LC | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | F | P | F | T | F | G | P | G | T | K | V | D | I | K | R |
| 21-225_28B1_LC | | | | | | | | | | | | | | | | | | | |
| 21-225_28F11_LC | | | | | L | | | | | | | | | | | | | | |
| 21-225_30A11_LC | | | | | | | | | | | | | | | | | N | | |
| 21-225_31C4_LC | | | | | | | | | | | | | | | | | | | |
| 21-225_31H9_LC | | | | | | | | | | | Q | | | | | | | | |
| 21-225_33G1_LC | | | | | | | | | | | | | | | | | | | |
| 21-225_33H7_LC | | | | | | | | | | | | | | | | | L | | |
| 21-225_34F3_LC | | | | | | | | | | | | | | | | | | | |
| 21-225_34G7_LC | | | | | | | | | | | | | | | | | | | |
| 21-225_35C6_LC | | | | | | | | | | | | | | | | | | | |
| 21-225_35G6_LC | | | | | | | | | | | | | | | | | L | | |
| 21-225_35H12_LC | | | | | | | | | | | | | | | | | | | |
| 21-225_36H5_LC | | | | | | | | | | | | | | | | | | | |
| 21-225_43H9_LC | | | | | L | | | | | | | | | | | | | | |
| 21-225_44F3_LC | | | | | | | | | | | | | | | | | | | |
| 21-225_45C9_LC | | | | | | | | | | | | | | | | | | | |
| 21-225_45H4_LC | | | | | L | | | | | | | | | | | | | | |
| 21-225_49F1_LC | | | | | | | | | | | | | | | | | | | |
| 21-225_49F10_LC | | | | | | | | | | | | | | | | | | | |
| 21-225_49H5_LC | | | | | | | | | | | | | | | | | | | |
| 21-225_4H6_LC | | | | | | | | | | | | | | | | | | | G |
| 21-225_4H6.004_LC | | | | | | | | | | | | | | | | | | | |
| 21-225_4H6.005_LC | | | | | | | | | | | Q | | | | | | | | |
| 21-225_4H6.006_LC | | | | | | | | | | | Q | | | | | | | | |
| 21-225_4H6.007_LC | | | | | | | | | | | | | | | | | | | |
| 21-225_4H6.008_LC | | | | | | | | | | | | | | | | | | | |
| 21-225_4H6.009_LC | | | | | | | | | | | Q | | | | | | | | |
| 21-225_4H6.010_LC | | | | | | | | | | | Q | | | | | | | | |
| 21-225_4H6.011_LC | | | | | | | | | | | Q | | | | | | | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | F | P | F | T | F | G | P | G | T | K | V | D | I | K | R |
| 21-225_4H6.014_LC | . | . | . | . | . | . | . | . | . | . | . | . | . | . | . | . | . | . | . |
| 21-225_50D4_LC | . | . | . | . | . | . | . | . | . | . | . | . | . | . | . | . | . | R | . |
| 21-225_50F3_LC | . | . | . | . | . | . | . | . | . | . | . | . | . | . | . | . | . | . | . |
| 21-225_51C7_LC | . | . | . | . | . | . | . | . | . | . | . | . | . | . | . | . | . | . | . |
| 21-225_51E9_LC | . | . | . | . | . | . | . | . | . | . | . | . | . | . | . | . | . | . | . |
| 21-225_52B1_LC | . | . | . | . | . | . | . | . | . | . | . | . | . | . | . | . | . | . | . |
| 21-225_52G11_LC | . | . | . | . | . | . | . | . | . | . | . | . | . | . | . | . | V | . | . |
| 21-225_52H10_LC | . | . | . | . | . | . | . | . | . | . | . | . | . | . | . | . | . | . | . |
| 21-225_53F2_LC | . | . | . | . | . | . | . | . | . | . | . | . | . | . | . | . | V | . | . |
| 21-225_53F7_LC | . | . | . | . | . | . | . | . | . | . | . | . | . | . | . | . | . | . | . |
| 21-225_55B1_LC | . | . | . | . | . | . | . | . | . | . | . | . | . | . | . | . | L | . | . |
| 21-225_56C6_LC | . | . | . | . | . | . | . | . | . | . | . | . | . | . | . | . | . | . | . |
| 21-225_56E5_LC | . | . | . | . | . | . | . | . | . | . | . | . | . | . | . | . | . | . | . |
| 21-225_56F6_LC | . | . | . | . | . | . | . | . | . | . | . | . | . | . | . | . | . | . | . |
| 21-225_57A7_LC | . | . | . | . | . | . | . | . | . | . | . | . | . | . | . | . | . | . | . |
| 21-225_57E4_LC | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . |
| 21-225_64G12_LC | . | . | . | . | . | . | . | . | . | . | . | . | . | . | L | . | . | . | . |
| 21-225_65F10_LC | . | . | . | . | . | . | . | . | R | . | . | . | . | . | . | . | . | . | . |
| 21-225_66F11_LC | . | . | . | . | . | . | . | . | . | . | . | . | . | . | . | . | . | . | . |
| 21-225_70D1_LC | . | . | . | . | . | . | . | . | . | . | . | . | . | . | . | . | . | . | . |
| 21-225_7C3_LC2_LC | . | . | . | . | . | . | . | . | . | . | . | . | . | . | . | . | . | . | . |
| 21-225_9F12_LC1_LC | . | . | . | . | . | . | . | . | . | . | . | . | . | . | . | . | V | . | . |

**FIGURE 57**

Table 105.    Consensus 25 - VK2|A18/JK1 (SEQ ID NO: 50322):

DIVMTQTPLSLSVTPGQPASISC<u>KSS</u>--<u>QSLLIIGD-GKTYLY</u>WYLQKPGQPPQLLIY<u>E</u>-------VSNRFSGVPDRFSGSGSG--
TDFTLKISRVEAEDVGVYYC<u>MQSIQ</u>---------------------LPWTFGQGTKVEIKR wherein:

K at position 24 can be substituted with M, R or T

S at position 26 can be substituted with G or T

Q at position 29 can be substituted with K

S at position 30 can be substituted with R, N or T

L at position 32 can be substituted with R or V

H at position 33 can be substituted with Y

G at position 34 can be substituted with S

D at position 35 can be substituted with E or G

K at position 38 can be substituted with R

Y at position 40 can be substituted with F

L at position 41 can be substituted with F

Y at position 42 can be substituted with F, T, C or S

E at position 58 can be substituted with A

V at position 67 can be substituted with I, L or T

N at position 69 can be substituted with K, H, I or S

F at position 71 can be substituted with L

S at position 72 can be substituted with A, T, C or P

FIGURE 57

M at position 107 can be substituted with K

S at position 109 can be substituted with T

I at position 110 can be substituted with T, F or L

Q at position 111 can be substituted with H

L at position 135 can be substituted with I, V or F

W at position 137 can be substituted with R

Xaa Ser Xaa Xaa Xaa Leu Xaa Xaa Xaa Xaa Gly Xaa Thr Xaa Xaa Xaa (SEQ ID NO: 50150)

Xaa Xaa Ser Xaa Arg Xaa Xaa (SEQ ID NO: 50151)

Xaa Gln Xaa Xaa Xaa Xaa Pro Xaa Thr (SEQ ID NO: 50152)

Lys Ser Ser Gln Ser Leu Leu His Gly Asp Gly Lys Thr Tyr Leu Tyr (SEQ ID NO: 50636)

Glu Val Ser Asn Arg Phe Ser (SEQ ID NO: 50637)

Met Gln Ser Ile Gln Leu Pro Trp Thr (SEQ ID NO: 50638)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | T | P | L | S | L | S | V | T | P | G | Q | P | A | S | I | S | C | K | S | S |
| 21-225_146B3_LC | . | . | . | . | . | . | . | . | . | . | . | . | . | . | . | . | . | . | . | . | . | . | . | . | . | . |
| 21-225_147E10_LC | A | . | . | . | . | . | . | . | . | . | . | . | . | . | . | . | . | . | . | . | . | . | . | . | . | . |
| 21-225_148B2_LC | A | . | . | . | . | . | . | . | . | . | . | . | . | . | . | . | . | . | . | . | . | . | . | . | . | . |
| 21-225_148G10_LC | . | . | . | . | . | . | . | . | . | . | . | . | . | . | . | . | . | . | . | . | . | . | . | . | . | . |
| 21-225_149F1_LC | . | . | . | . | . | . | S | . | . | . | P | . | . | . | . | . | . | . | . | . | . | . | Y | . | . | . |
| 21-225_150B11_LC | . | . | . | . | . | . | . | . | . | . | . | . | . | . | . | . | . | . | . | . | . | . | . | . | . | . |
| 21-225_151A10_LC | . | . | . | . | . | . | . | . | . | . | . | . | . | . | . | . | . | . | . | . | . | . | . | . | . | . |
| 21-225_151G5_LC | . | . | . | . | . | . | . | . | . | . | . | . | . | . | . | . | . | . | . | . | . | . | . | . | . | . |
| 21-225_152C11_LC | . | . | . | . | . | . | A | . | . | . | . | . | . | . | . | . | . | . | . | . | . | . | . | . | . | . |
| 21-225_152F6_LC | . | . | . | . | . | . | . | . | . | . | . | . | . | . | . | . | . | . | . | . | . | . | . | . | . | . |
| 21-225_153D2_LC | . | . | . | . | . | . | . | . | . | . | . | . | . | . | . | . | . | . | . | . | . | . | . | . | . | . |
| 21-225_155A5_LC | . | . | . | . | . | . | . | . | . | . | . | . | . | . | . | . | . | . | . | . | . | . | . | . | . | . |
| 21-225_157G8_LC | . | . | . | . | . | . | . | . | . | . | . | . | . | . | . | . | . | . | . | . | . | . | . | . | . | . |
| 21-225_160B1_LC | . | . | . | . | . | . | . | . | . | . | . | . | . | . | . | . | . | . | . | . | . | . | . | . | . | . |
| 21-225_160G10_LC | A | . | . | . | . | . | . | . | . | . | . | . | . | . | . | . | . | . | . | . | . | . | . | . | . | . |
| 21-225_162H6_LC | . | . | . | . | . | . | . | . | . | . | . | . | . | . | . | . | . | . | . | . | . | . | . | . | . | Y |
| 21-225_167E2_LC | . | . | . | . | . | . | . | . | . | . | . | . | . | . | . | . | . | . | . | . | . | . | . | . | . | . |
| 21-225_167H10_LC | . | . | . | . | . | . | . | . | . | . | . | . | . | . | . | . | . | . | . | . | . | . | . | . | . | . |
| 21-225_170A1_LC | S | . | . | . | . | . | . | . | P | . | . | . | . | . | . | . | . | . | . | . | . | . | . | . | . | . |
| 21-225_172B7_LC | . | . | . | . | . | . | . | . | . | . | . | . | . | . | . | . | . | . | . | . | . | . | . | . | . | . |
| 21-225_173A11_LC | . | . | . | . | . | . | . | . | . | . | . | . | . | . | . | . | . | . | . | . | . | . | . | . | . | . |
| 21-225_176H4_LC | . | . | . | . | . | . | . | . | . | . | . | . | . | . | . | . | . | . | . | . | . | . | . | . | . | . |
| 21-225_177B11_LC | . | . | . | . | . | . | . | . | . | . | . | . | . | . | . | . | . | . | . | . | . | . | . | . | . | . |
| 21-225_178G10_LC | . | . | . | . | . | . | . | . | . | . | . | . | . | . | . | . | . | . | . | . | . | . | . | . | . | . |
| 21-225_180C4_LC | . | . | . | . | . | . | . | . | . | . | . | . | . | . | . | . | . | . | . | . | . | . | . | . | . | . |
| 21-225_181C10_LC | . | . | . | . | . | . | . | . | . | . | . | . | . | . | . | . | . | . | . | . | . | . | . | . | . | . |
| 21-225_181C2_LC | S | . | . | . | . | . | . | . | . | . | . | . | . | . | . | . | . | . | . | . | . | . | . | . | . | . |
| 21-225_186F7_LC | . | V | . | . | A | . | . | . | . | . | . | . | . | . | . | . | . | . | . | . | . | . | . | . | . | . |
| 21-225_188G11_LC | . | . | . | . | . | . | . | . | . | . | . | . | . | . | . | . | . | . | . | . | . | . | M | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | V | M | T | Q | T | P | L | S | L | S | V | T | P | G | Q | P | A | S | I | S | C | K | S | S |
| 21-225_189G2_LC | . | . | . | . | . | . | . | . | . | . | . | . | . | . | . | . | . | . | . | . | . | . | . | . | . | . |
| 21-225_190E7_LC | . | . | . | L | . | . | . | . | . | . | . | . | . | . | . | . | . | . | . | . | . | . | . | . | . | . |
| 21-225_190H11_LC | . | . | . | . | . | . | . | . | . | . | . | . | . | . | . | . | . | . | . | . | . | . | . | . | . | . |
| 21-225_191B1_LC | . | . | . | . | . | . | . | . | . | . | . | . | . | . | . | . | . | . | . | . | . | . | . | . | . | . |
| 21-225_191E3_LC | . | . | . | . | . | . | . | . | . | . | . | . | . | . | . | . | . | . | . | . | . | . | . | . | . | . |
| 21-225_191E6_LC | . | . | . | . | . | . | . | . | . | . | . | . | . | . | . | . | . | . | . | . | . | . | . | . | . | . |
| 21-225_192F9_LC | . | . | . | . | . | . | . | . | . | . | . | . | . | . | . | . | . | . | . | . | . | . | . | . | . | . |
| 21-225_194F4_LC | . | . | . | . | . | . | . | . | . | . | . | . | . | . | . | . | . | . | . | . | . | . | . | . | . | . |
| 21-225_197G8_LC | E | . | . | . | . | . | . | . | . | . | . | . | I | . | . | . | . | . | . | . | . | . | . | . | . | . |
| 21-225_198A10_LC | . | . | . | L | . | . | . | . | . | . | . | . | . | . | . | . | . | . | . | . | . | . | . | . | . | . |
| 21-225_224A7_LC | . | . | . | . | . | . | . | . | . | . | . | . | . | . | . | . | . | . | . | . | . | . | . | . | . | . |
| 21-225_224C11_LC | . | S | . | . | . | . | . | . | . | . | . | . | . | . | . | . | . | . | . | . | . | . | . | . | . | . |
| 21-225_224G1_LC | . | . | . | . | . | . | . | . | . | . | . | . | . | . | . | . | . | . | . | . | . | . | . | . | . | G |
| 21-225_224H11_LC | . | . | . | . | . | . | . | . | . | . | . | . | . | . | . | . | . | . | . | . | . | . | . | . | . | . |
| 21-225_225G4_LC | . | . | . | . | . | . | . | . | . | . | . | . | . | . | . | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | . | . | . | . | . | . |
| 21-225_226E11_LC | . | . | . | . | . | . | . | . | . | . | . | . | . | . | . | . | . | . | . | . | . | . | . | . | . | . |
| 21-225_226E7_LC | . | . | . | . | . | . | . | . | . | . | . | . | . | . | . | . | . | . | . | . | . | . | . | . | . | . |
| 21-225_226F10_LC | . | . | . | . | . | . | . | . | . | . | . | . | . | . | . | . | . | . | . | . | . | . | . | . | . | . |
| 21-225_226F9_LC | . | . | . | . | . | . | . | . | . | . | . | . | . | . | . | . | . | . | . | . | . | . | . | . | . | . |
| 21-225_226G8_LC | . | . | . | . | . | . | . | . | . | . | . | . | . | . | . | . | . | . | . | . | . | . | . | . | . | . |
| 21-225_226H12_LC | . | . | . | . | . | . | . | . | . | . | . | . | . | . | . | . | . | . | . | . | . | . | . | . | . | . |
| 21-225_226H9_LC | . | . | . | . | . | . | . | . | . | . | . | . | I | . | . | . | . | . | . | . | . | . | . | . | . | . |
| 21-225_227A8_LC | . | . | . | . | . | . | . | . | . | . | . | . | . | . | . | . | . | . | . | . | . | . | . | . | . | . |
| 21-225_227C1_LC | . | . | . | . | . | . | . | . | . | . | . | . | . | . | . | . | . | . | . | . | . | . | . | . | . | . |
| 21-225_227F2_LC | . | . | . | . | . | . | . | . | . | . | . | . | . | . | . | . | . | . | . | . | . | . | . | . | . | . |
| 21-225_22A1_LC | . | . | . | . | . | . | . | . | . | . | . | . | . | . | . | . | . | . | . | . | . | . | . | . | . | . |
| 21-225_25H9_LC | . | . | . | . | . | . | . | . | . | . | . | . | . | . | . | . | . | . | . | . | . | . | . | . | . | . |

EP 4 435 105 A2

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | T | P | L | S | L | S | V | T | P | G | Q | P | A | S | I | S | C | K | S | S |
| 21-225_26F8_LC | . | . | . | . | . | . | . | . | . | . | . | . | . | . | . | . | . | . | . | . | . | . | . | . | . | . |
| 21-225_28G3_LC | E | . | . | . | . | . | . | . | . | . | . | . | . | . | . | . | . | . | . | . | . | . | . | . | . | . |
| 21-225_43E11_LC | . | . | . | . | . | . | . | . | . | . | . | . | . | . | . | . | . | . | . | . | . | . | . | . | . | . |
| 21-225_46F2_LC | . | . | . | . | . | . | . | . | . | . | . | T | . | . | . | . | . | . | . | . | . | . | . | . | . | . |
| 21-225_47C1_LC | . | . | . | . | . | . | . | . | . | . | . | . | . | . | . | . | . | . | . | . | . | . | . | T | . | . |
| 21-225_47E7_LC | . | . | . | . | . | . | . | . | . | . | . | . | . | . | . | . | . | . | . | . | . | . | . | . | . | . |
| 21-225_64H9_LC | E | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | S | L | L | H | G | D | - | G | K | T | Y | L | Y | W | Y | L | Q | K | P | G | Q | P | P |
| 21-225_146B3_LC | . | . | . | . | . | . | . | . | . | . | . | . | . | . | . | . | . | . | . | . | . | . | . | . | . | . |
| 21-225_147E10_LC | . | . | . | R | . | . | . | . | . | . | . | . | . | . | . | . | . | . | . | . | . | . | . | . | . | . |
| 21-225_148B2_LC | . | . | . | . | . | . | . | . | . | . | . | . | . | . | . | P | . | . | . | . | . | . | . | . | . | . |
| 21-225_148G10_LC | . | . | . | . | . | . | . | . | . | . | . | . | . | . | . | . | . | . | . | . | R | . | . | . | . | . |
| 21-225_149F1_LC | . | . | . | . | . | . | . | . | . | . | . | . | . | . | . | P | . | . | . | . | . | . | . | . | . | . |
| 21-225_150B11_LC | . | . | . | . | . | V | . | . | . | . | . | . | . | . | . | . | . | . | . | . | R | . | . | . | . | . |
| 21-225_151A10_LC | . | . | . | . | . | . | . | . | . | . | . | . | . | . | . | . | . | . | . | . | . | . | . | . | . | . |
| 21-225_151G5_LC | . | . | . | R | . | . | . | . | . | . | . | . | . | . | . | . | . | . | . | . | . | . | . | . | . | . |
| 21-225_152C11_LC | . | . | . | . | . | . | . | . | . | . | . | . | . | . | . | . | . | . | . | . | . | . | . | . | . | . |
| 21-225_152F6_LC | . | . | . | . | . | R | . | . | . | . | . | . | . | . | . | T | . | . | . | . | R | . | . | . | . | . |
| 21-225_153D2_LC | . | . | . | . | . | R | . | . | . | . | . | . | . | . | . | T | . | . | . | . | R | . | . | . | . | . |
| 21-225_155A5_LC | . | . | . | . | . | . | . | . | . | . | . | . | . | . | . | . | . | . | . | . | . | . | . | . | . | . |
| 21-225_157G8_LC | . | . | . | . | . | . | . | . | . | . | . | . | . | . | . | . | . | . | . | . | . | . | . | . | S | . |
| 21-225_160B1_LC | . | . | . | . | . | . | . | . | . | . | . | . | . | . | P | . | . | . | . | . | . | . | . | . | . | . |
| 21-225_160G10_LC | . | . | . | . | . | . | . | . | . | . | . | . | . | . | . | . | . | . | . | . | . | . | . | . | . | . |
| 21-225_162H6_LC | . | . | . | . | . | . | . | . | . | . | . | . | . | . | . | . | . | . | . | . | . | . | . | . | . | . |
| 21-225_167E2_LC | . | . | . | . | . | . | . | . | . | . | . | . | . | . | . | . | . | . | . | . | . | . | . | . | . | . |
| 21-225_167H10_LC | . | . | . | . | . | . | . | . | . | . | . | . | . | . | . | . | . | . | . | . | . | . | . | . | . | . |
| 21-225_170A1_LC | . | . | . | . | . | . | . | . | . | . | . | . | . | . | . | P | . | . | . | . | . | . | . | . | . | . |
| 21-225_172B7_LC | . | . | . | . | . | . | . | . | . | . | . | . | . | . | P | . | . | . | . | . | . | . | . | . | . | . |
| 21-225_173A11_LC | . | . | . | . | . | . | . | . | . | . | . | . | . | . | . | . | . | . | . | . | . | . | . | . | . | . |
| 21-225_176H4_LC | . | . | . | . | . | . | . | . | . | . | . | . | . | . | . | . | . | . | . | . | . | . | . | . | . | . |
| 21-225_177B11_LC | . | . | R | . | . | . | . | . | . | . | . | . | . | . | . | . | . | . | . | . | . | . | . | . | . | . |
| 21-225_178G10_LC | . | . | . | . | . | . | . | . | . | . | . | . | . | . | . | . | . | . | . | . | . | . | . | . | . | . |
| 21-225_180C4_LC | . | . | . | . | . | . | . | . | . | . | . | . | . | . | . | . | . | . | . | . | . | . | . | . | . | . |
| 21-225_181C10_LC | . | . | . | . | . | . | . | . | . | . | . | . | . | . | . | . | . | . | . | . | . | . | . | . | . | . |
| 21-225_181C2_LC | . | . | . | . | . | . | . | . | . | . | . | . | . | . | . | . | . | . | . | . | . | . | . | . | . | . |
| 21-225_186F7_LC | . | . | . | . | . | . | . | . | . | . | . | . | . | . | C | . | . | . | . | . | . | . | . | . | . | . |
| 21-225_188G11_LC | . | . | . | . | . | . | . | . | . | . | . | . | . | . | . | . | . | . | . | . | . | . | . | . | . | . |

## FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | L | L | H | G | D | - | G | K | T | Y | L | Y | W | Y | L | Q | K | P | G | Q | P | P |
| 21-225_189G2_LC | . | . | . | . | . | . | . | . | . | . | . | . | . | . | . | S | . | . | . | . | . | . | . | . | . | . |
| 21-225_190E7_LC | . | . | . | . | . | . | . | S | . | . | . | R | . | . | . | . | . | . | . | . | . | . | . | . | . | . |
| 21-225_190H11_LC | . | . | . | . | . | . | . | S | . | . | . | R | . | . | . | . | . | . | . | . | . | . | . | . | . | . |
| 21-225_191B1_LC | . | . | . | . | . | . | . | S | . | . | . | . | . | . | . | S | . | . | . | . | . | . | . | . | . | . |
| 21-225_191E3_LC | . | . | . | . | . | . | . | S | . | . | . | R | . | . | . | . | . | . | . | . | . | . | . | . | . | . |
| 21-225_191E6_LC | . | . | . | . | . | . | . | S | . | . | . | R | . | . | . | . | . | . | . | . | . | . | . | . | . | . |
| 21-225_192F9_LC | . | . | . | . | . | . | . | S | . | . | . | R | . | . | . | . | . | . | . | . | R | . | . | . | . | . |
| 21-225_194F4_LC | . | . | . | . | . | . | . | S | . | . | . | R | . | . | . | . | . | . | . | . | . | . | . | . | . | . |
| 21-225_197G8_LC | . | . | N | . | . | . | . | S | . | . | . | . | . | . | . | . | . | . | . | . | . | . | . | . | . | . |
| 21-225_198A10_LC | . | . | . | . | . | . | Y | S | . | . | . | R | . | . | . | . | . | . | . | . | . | . | . | . | . | . |
| 21-225_224A7_LC | . | . | . | . | . | . | . | S | . | . | . | . | . | S | . | . | . | S | . | . | . | . | . | . | . | . |
| 21-225_224C11_LC | . | . | . | . | . | . | . | S | . | . | . | . | . | S | . | . | . | . | . | . | . | . | . | . | . | . |
| 21-225_224G1_LC | . | . | . | . | . | . | . | S | . | . | . | . | . | S | . | . | S | . | . | . | . | . | . | . | . | . |
| 21-225_224H11_LC | . | . | . | . | . | . | . | S | . | . | . | . | . | S | . | . | . | . | . | . | . | . | . | . | . | . |
| 21-225_225G4_LC | . | . | . | . | . | . | . | S | . | . | . | . | . | S | . | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | R | . | . | . | . | . |
| 21-225_226E11_LC | . | . | . | . | . | . | . | S | . | . | . | . | . | S | . | . | . | . | . | R | . | . | . | . | . | . |
| 21-225_226E7_LC | . | . | . | . | . | . | . | . | . | . | . | . | . | . | . | . | . | . | Q | . | . | . | . | . | . | . |
| 21-225_226F10_LC | . | . | . | . | . | . | . | S | . | . | . | . | . | S | . | . | . | . | . | . | . | . | . | . | . | . |
| 21-225_226F9_LC | . | . | . | . | . | . | . | S | . | . | . | . | . | S | . | . | . | . | . | . | . | . | . | . | . | . |
| 21-225_226G8_LC | . | . | . | . | . | . | . | S | . | . | . | . | . | S | . | . | . | . | . | . | . | . | . | . | . | . |
| 21-225_226H12_LC | . | . | R | T | . | . | . | S | . | . | . | . | . | S | . | . | . | . | . | . | . | . | . | . | . | . |
| 21-225_226H9_LC | . | . | . | . | . | . | . | S | . | . | . | . | . | S | . | . | . | . | . | . | R | . | . | . | . | . |
| 21-225_227A8_LC | . | . | . | . | . | . | . | S | . | . | . | . | . | S | . | . | . | . | . | . | . | . | . | . | . | . |
| 21-225_227C1_LC | . | . | . | . | . | . | . | S | . | . | . | . | . | S | . | . | . | . | . | . | . | . | . | . | . | . |
| 21-225_227F2_LC | . | . | . | . | . | . | . | S | . | . | . | . | . | S | . | . | . | . | . | . | . | . | . | . | . | . |
| 21-225_22A1_LC | . | . | . | . | . | . | . | S | G | . | . | . | . | . | . | . | . | . | . | . | . | . | . | . | . | . |
| 21-225_25H9_LC | . | . | . | . | . | . | . | . | . | . | . | . | . | S | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | S | L | L | H | G | D | - | G | K | T | Y | L | Y | W | Y | L | Q | K | P | G | Q | P | P |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | . | . | . |
| 21-225_43E11_LC | . | . | . | . | . | . | . | S | . | . | . | R | . | . | . | . | . | . | . | . | . | . | . | . | . | . |
| 21-225_46F2_LC | . | . | . | . | . | . | . | . | . | . | . | . | . | . | . | . | . | . | . | . | . | . | . | . | . | . |
| 21-225_47C1_LC | . | . | . | . | . | . | . | S | E | . | . | . | . | . | . | . | . | . | . | . | . | . | . | . | . | . |
| 21-225_47E7_LC | . | . | . | . | . | . | . | S | . | . | . | R | . | . | . | . | . | . | . | . | . | . | . | . | . | . |
| 21-225_64H9_LC | . | . | . | . | . | . | . | . | . | . | . | . | . | . | . | F | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | Q | L | L | I | Y | E | - | - | - | - | - | - | - | - | V | S | N | R | F | S | G | V | P | D | R | F |
| 21-225_146B3_LC | . | . | . | . | . | . | . | . | . | . | . | . | . | . | . | . | . | . | . | . | . | . | . | . | . | . |
| 21-225_147E10_LC | N | . | . | . | S | . | . | . | . | . | . | . | . | . | . | . | . | . | . | . | . | . | . | . | . | . |
| 21-225_148B2_LC | . | . | . | . | . | . | . | . | . | . | . | . | . | . | . | . | N | . | . | . | . | . | . | . | . | . |
| 21-225_148G10_LC | . | . | . | . | . | . | . | . | . | . | . | . | . | . | . | . | . | . | . | . | . | . | . | . | . | . |
| 21-225_149F1_LC | . | . | . | . | . | . | . | . | . | . | . | . | . | . | . | . | N | . | . | . | . | . | . | . | . | . |
| 21-225_150B11_LC | . | . | . | . | . | . | . | . | . | . | . | . | . | . | . | . | . | . | . | . | . | . | . | . | . | . |
| 21-225_151A10_LC | . | . | . | . | . | . | . | . | . | . | . | . | . | . | . | . | N | . | . | . | . | . | . | . | . | . |
| 21-225_151G5_LC | . | . | . | L | . | . | . | . | . | . | . | . | . | . | . | . | . | . | . | . | . | . | . | . | . | . |
| 21-225_152C11_LC | . | I | . | . | . | . | . | . | . | . | . | . | . | . | . | . | . | . | . | . | . | . | . | . | . | . |
| 21-225_152F6_LC | . | V | . | . | N | . | . | . | . | . | . | . | . | . | I | . | N | . | . | T | . | . | . | . | . | . |
| 21-225_153D2_LC | . | V | . | . | N | . | . | . | . | . | . | . | . | . | . | . | N | . | . | T | . | . | . | . | . | . |
| 21-225_155A5_LC | . | . | . | . | . | . | . | . | . | . | . | . | . | . | . | . | . | . | . | . | . | . | . | . | . | . |
| 21-225_157G8_LC | . | . | . | . | . | . | . | . | . | . | . | . | . | . | I | . | N | . | . | . | . | . | . | . | . | . |
| 21-225_160B1_LC | . | V | . | . | . | . | . | . | . | . | . | . | . | . | . | . | N | . | . | . | . | . | S | N | . | . |
| 21-225_160G10_LC | N | . | . | . | . | . | . | . | . | . | . | . | . | . | . | . | N | . | . | . | . | . | . | . | . | . |
| 21-225_162H6_LC | . | . | . | . | . | . | . | . | . | . | . | . | . | . | . | . | N | . | . | . | . | . | . | N | . | . |
| 21-225_167E2_LC | N | . | . | . | . | . | . | . | . | . | . | . | . | . | . | . | N | . | . | . | . | . | . | . | . | . |
| 21-225_167H10_LC | N | . | . | . | . | . | . | . | . | . | . | . | . | . | . | . | N | . | . | . | . | . | . | . | . | . |
| 21-225_170A1_LC | . | V | . | . | . | . | . | . | . | . | . | . | . | . | . | . | . | . | . | . | . | . | . | . | . | . |
| 21-225_172B7_LC | . | V | . | L | N | . | . | . | . | . | . | . | . | . | . | . | N | . | . | . | . | . | . | . | . | . |
| 21-225_173A11_LC | . | . | . | . | . | . | . | . | . | . | . | . | . | . | . | . | . | . | . | . | . | . | . | . | . | . |
| 21-225_176H4_LC | . | . | . | . | . | . | . | . | . | . | . | . | . | . | . | . | . | . | . | . | . | . | . | . | . | . |
| 21-225_177B11_LC | . | I | . | . | . | . | . | . | . | . | . | . | . | . | . | . | . | . | . | . | . | . | . | . | . | . |
| 21-225_178G10_LC | . | . | . | . | . | . | . | . | . | . | . | . | . | . | . | . | . | . | . | . | . | . | . | . | . | L |
| 21-225_180C4_LC | . | . | . | . | . | A | . | . | . | . | . | . | . | . | T | . | . | . | . | P | . | . | S | . | . | . |
| 21-225_181C10_LC | . | . | . | . | . | . | . | . | . | . | . | . | . | . | . | . | . | . | . | . | . | . | . | . | . | . |
| 21-225_181C2_LC | . | . | . | . | N | . | . | . | . | . | . | . | . | . | . | . | . | . | . | . | . | . | . | . | . | . |
| 21-225_186F7_LC | . | . | . | . | . | . | . | . | . | . | . | . | . | . | . | . | K | . | . | . | . | . | . | . | . | . |
| 21-225_188G11_LC | . | . | . | . | . | . | . | . | . | . | . | . | . | . | . | . | . | . | . | . | . | . | . | . | T | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | K_CDR2 | | | | | | | | | | | | |
| CONSENSUS | Q | L | L | I | Y | E | - | - | - | - | - | - | - | - | V | S | N | R | F | S | G | V | P | D | R | F |
| 21-225_189G2_LC | . | . | . | . | Y | . | . | . | . | . | . | . | . | . | . | . | . | . | . | . | . | . | S | . | . | . |
| 21-225_190E7_LC | . | V | . | . | C | . | . | . | . | . | . | . | . | . | . | . | . | . | . | . | . | . | . | . | . | . |
| 21-225_190H11_LC | . | . | . | . | C | . | . | . | . | . | . | . | . | . | . | . | . | . | . | A | . | . | T | . | . | . |
| 21-225_191B1_LC | . | V | . | . | . | . | . | . | . | . | . | . | . | . | L | . | . | . | . | . | . | . | . | . | . | . |
| 21-225_191E3_LC | . | . | . | . | C | . | . | . | . | . | . | . | . | . | . | . | . | . | . | A | . | . | T | . | . | . |
| 21-225_191E6_LC | . | . | . | . | C | . | . | . | . | . | . | . | . | . | . | . | . | . | . | A | . | . | T | . | . | . |
| 21-225_192F9_LC | . | . | . | . | C | . | . | . | . | . | . | . | . | . | . | . | . | . | . | A | . | . | T | . | . | . |
| 21-225_194F4_LC | . | . | . | . | C | . | . | . | . | . | . | . | . | . | . | . | . | . | . | . | . | . | . | . | . | . |
| 21-225_197G8_LC | . | V | . | . | . | . | . | . | . | . | . | . | . | . | . | . | . | . | . | . | . | . | . | . | . | . |
| 21-225_198A10_LC | . | V | . | . | C | . | . | . | . | . | . | . | . | . | . | . | . | . | . | . | . | . | . | . | . | . |
| 21-225_224A7_LC | . | . | . | . | . | . | . | . | . | . | . | . | . | . | | | . | . | . | . | . | . | . | . | . | . |
| 21-225_224C11_LC | . | . | . | . | . | . | . | . | . | . | . | . | . | . | | | . | . | . | . | . | . | . | . | . | . |
| 21-225_224G1_LC | . | . | . | . | . | . | . | . | . | . | . | . | . | . | | | . | . | . | . | . | . | . | . | . | . |
| 21-225_224H11_LC | . | . | . | . | . | . | . | . | . | . | . | . | . | . | | | . | . | . | . | . | . | . | . | . | . |
| 21-225_225G4_LC | . | . | . | . | . | . | . | . | . | . | . | . | . | . | | | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | N | . | . | . | . | . | . | . | . | . | . | . | | | . | . | . | . | . | . | . | . |
| 21-225_226E11_LC | . | . | . | . | . | . | . | . | . | . | . | . | . | . | | | . | . | . | . | . | . | . | . | . | . |
| 21-225_226E7_LC | . | I | . | . | . | . | . | . | . | . | . | . | . | . | . | . | . | . | . | . | . | . | . | . | . | . |
| 21-225_226F10_LC | . | . | . | . | . | . | . | . | . | . | . | . | . | . | | | . | . | . | . | . | . | . | . | . | . |
| 21-225_226F9_LC | . | . | . | . | . | . | . | . | . | . | . | . | . | . | | | . | . | . | . | . | . | . | . | . | . |
| 21-225_226G8_LC | . | . | . | . | . | . | . | . | . | . | . | . | . | . | | | . | . | . | . | . | . | . | . | . | . |
| 21-225_226H12_LC | . | P | . | . | . | . | . | . | . | . | . | . | . | . | | | . | . | . | . | . | . | . | . | . | . |
| 21-225_226H9_LC | . | . | . | . | . | . | . | . | . | . | . | . | . | . | . | . | . | . | . | . | . | . | . | . | . | . |
| 21-225_227A8_LC | . | . | . | . | . | . | . | . | . | . | . | . | . | . | | | . | . | . | . | . | . | . | . | . | . |
| 21-225_227C1_LC | . | . | . | . | . | . | . | . | . | . | . | . | . | . | | | . | . | . | . | . | . | . | . | . | . |
| 21-225_227F2_LC | . | . | . | . | . | . | . | . | . | . | . | . | . | . | | | . | . | . | . | . | . | . | . | . | . |
| 21-225_22A1_LC | . | . | . | . | . | . | . | . | . | . | . | . | . | . | . | . | . | . | . | . | . | L | . | . | . | . |
| 21-225_25H9_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | Q | L | L | I | Y | E | - | - | - | - | - | - | - | - | V | S | N | R | F | S | G | V | P | D | R | F |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | S | . | . |
| 21-225_43E11_LC | . | . | . | . | . | . | . | . | . | . | . | . | . | . | . | . | . | . | . | . | . | . | . | . | . | . |
| 21-225_46F2_LC | . | V | . | . | . | . | . | . | . | . | . | . | . | . | . | . | . | . | . | . | . | . | . | . | . | . |
| 21-225_47C1_LC | . | . | . | . | . | . | . | . | . | . | . | . | . | . | . | . | . | . | . | . | . | . | . | . | . | . |
| 21-225_47E7_LC | . | . | . | . | . | . | . | . | . | . | . | . | . | . | . | . | S | . | . | . | . | . | . | . | . | . |
| 21-225_64H9_LC | . | V | . | . | . | . | . | . | . | . | . | . | . | . | . | . | . | . | L | C | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | K | I | S | R | V | E | A | E | D | V | G | V | Y |
| 21-225_146B3_LC | . | . | . | . | . | . | . | . | . | . | . | . | . | . | . | . | . | . | . | . | . | . | . | . | L | . |
| 21-225_147E10_LC | . | . | . | . | . | . | . | . | . | . | . | . | . | . | . | . | . | . | . | . | . | . | . | . | . | . |
| 21-225_148B2_LC | . | . | . | . | . | . | . | . | . | . | . | . | . | . | . | . | . | . | . | . | . | . | . | . | . | . |
| 21-225_148G10_LC | . | . | . | . | . | . | . | . | . | . | . | . | . | . | . | . | . | . | . | . | . | . | . | . | . | . |
| 21-225_149F1_LC | . | . | . | . | . | . | . | . | . | . | . | . | . | . | . | . | . | . | . | . | . | . | . | . | . | . |
| 21-225_150B11_LC | . | . | . | . | . | . | . | . | . | . | . | . | . | . | . | . | . | . | . | . | . | . | . | . | . | . |
| 21-225_151A10_LC | . | . | . | . | . | . | . | . | . | . | . | . | . | . | . | . | . | . | . | . | . | . | . | . | . | . |
| 21-225_151G5_LC | . | . | . | . | . | . | . | . | . | . | . | . | . | . | . | . | . | . | . | . | . | . | . | . | . | . |
| 21-225_152C11_LC | . | . | . | . | . | . | . | . | . | . | . | . | . | N | . | . | . | . | . | . | . | . | S | . | S | . |
| 21-225_152F6_LC | . | . | . | . | . | . | . | . | . | . | . | . | . | N | . | . | . | . | . | . | . | . | . | . | S | . |
| 21-225_153D2_LC | . | . | . | . | . | . | . | . | . | . | . | . | . | N | . | . | . | . | . | . | . | . | . | . | S | . |
| 21-225_155A5_LC | . | . | . | . | . | . | . | . | . | . | . | . | . | . | . | . | . | . | . | . | . | . | . | . | S | . |
| 21-225_157G8_LC | . | . | . | . | . | . | . | . | . | . | . | . | . | . | . | . | . | . | . | . | . | . | . | . | . | . |
| 21-225_160B1_LC | . | . | . | . | . | . | . | . | . | . | . | . | . | . | . | . | . | . | . | . | . | . | . | . | . | . |
| 21-225_160G10_LC | . | . | . | . | . | . | . | . | . | . | . | . | . | . | . | . | . | . | . | . | . | . | . | . | L | . |
| 21-225_162H6_LC | . | . | . | . | . | . | . | . | . | . | . | . | . | . | . | . | . | . | . | . | . | . | . | . | . | . |
| 21-225_167E2_LC | . | . | . | . | . | . | . | . | . | . | . | . | . | . | . | . | . | . | . | . | . | . | . | . | L | . |
| 21-225_167H10_LC | . | . | . | . | . | . | . | . | . | . | . | . | . | . | . | . | . | . | . | . | . | . | . | . | L | . |
| 21-225_170A1_LC | . | . | . | . | . | . | . | . | . | . | . | . | . | . | . | . | . | . | . | . | . | . | . | . | . | . |
| 21-225_172B7_LC | . | G | . | . | . | . | . | . | . | . | . | . | . | . | . | . | . | . | . | . | . | . | . | . | . | S |
| 21-225_173A11_LC | . | . | . | . | . | . | . | . | . | . | . | . | . | . | . | . | . | . | . | . | . | . | . | . | . | S |
| 21-225_176H4_LC | . | . | . | . | . | . | . | . | . | . | . | . | . | . | . | . | . | . | . | . | . | . | . | . | I | . |
| 21-225_177B11_LC | . | . | . | . | . | . | . | . | S | . | . | . | . | . | . | . | . | . | . | . | . | . | . | . | . | . |
| 21-225_178G10_LC | . | G | . | . | . | . | . | . | . | . | . | . | . | . | . | . | . | . | . | . | . | . | . | . | I | . |
| 21-225_180C4_LC | . | . | . | . | . | . | . | . | . | . | . | . | . | . | . | . | . | . | . | . | . | . | . | . | . | . |
| 21-225_181C10_LC | . | . | . | . | . | . | . | . | . | . | . | . | . | . | . | . | . | . | . | . | . | . | . | . | . | . |
| 21-225_181C2_LC | . | . | . | . | . | . | . | . | . | . | . | . | . | . | . | . | . | . | . | . | . | . | . | . | . | . |
| 21-225_186F7_LC | . | . | . | . | . | . | . | . | . | . | . | . | . | . | . | . | . | . | . | . | . | . | . | . | . | . |
| 21-225_188G11_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | K | I | S | R | V | E | A | E | D | V | G | V | Y |
| 21-225_189G2_LC | . | . | . | . | . | . | | | . | . | . | . | . | . | . | . | . | . | . | . | . | . | . | . | . | . |
| 21-225_190E7_LC | . | . | . | . | . | . | | | . | . | . | . | . | . | . | . | . | . | . | . | G | . | . | . | . | . |
| 21-225_190H11_LC | . | . | . | . | . | . | | | . | . | . | . | . | . | . | . | . | . | . | . | G | . | . | . | . | . |
| 21-225_191B1_LC | . | . | . | . | . | . | | | . | . | . | . | . | . | . | . | . | . | . | . | . | . | . | . | . | . |
| 21-225_191E3_LC | . | . | . | . | . | . | | | . | . | . | . | . | . | . | . | . | . | . | . | G | . | . | . | . | . |
| 21-225_191E6_LC | . | . | . | . | . | . | | | . | . | . | . | . | . | . | . | . | . | . | . | G | . | . | . | } | . |
| 21-225_192F9_LC | . | . | . | . | . | . | | | . | . | . | . | . | . | . | . | . | . | . | . | G | . | . | . | . | . |
| 21-225_194F4_LC | . | . | . | . | . | . | | | . | . | . | . | . | . | . | . | . | . | . | . | G | . | . | . | . | . |
| 21-225_197G8_LC | . | . | . | . | . | . | | | . | . | . | . | . | . | . | . | . | . | . | . | . | . | . | . | . | . |
| 21-225_198A10_LC | . | . | . | . | . | . | | | . | . | . | . | . | . | . | . | . | . | . | . | G | . | . | . | . | . |
| 21-225_224A7_LC | . | . | . | . | . | . | | | . | . | . | . | . | . | . | . | . | . | . | . | . | . | . | . | } | . |
| 21-225_224C11_LC | . | . | . | . | . | . | | | . | . | . | . | . | . | . | . | . | . | . | . | . | . | . | . | } | . |
| 21-225_224G1_LC | . | . | . | . | . | . | | | . | . | . | . | . | . | . | . | . | . | . | . | . | . | . | . | } | ? |
| 21-225_224H11_LC | . | . | . | . | . | . | | | . | . | . | . | . | . | . | . | . | . | . | . | . | . | . | . | } | . |
| 21-225_225G4_LC | . | . | . | . | . | . | | | . | . | . | . | . | . | . | . | . | . | . | . | . | . | . | . | } | . |
| 21-225_226A5_LC | . | . | . | . | . | . | | | . | . | . | . | . | . | . | . | . | . | . | . | . | . | . | . | . | . |
| 21-225_226B1_LC | . | . | . | . | . | . | | | . | . | . | . | . | . | . | . | . | . | . | . | . | . | . | . | . | . |
| 21-225_226E11_LC | . | . | . | . | . | . | | | . | . | . | . | . | . | . | . | . | . | . | . | . | . | . | . | } | . |
| 21-225_226E7_LC | . | . | . | . | . | . | | | . | . | . | . | . | . | . | . | . | . | . | . | . | . | . | . | . | . |
| 21-225_226F10_LC | . | . | . | . | . | . | | | . | . | . | . | . | . | . | . | . | . | . | . | . | . | . | . | } | . |
| 21-225_226F9_LC | . | . | . | . | . | . | | | . | . | . | . | . | . | . | . | . | . | . | . | . | . | . | . | } | . |
| 21-225_226G8_LC | . | . | . | . | . | . | | | . | . | . | . | . | . | . | . | . | . | . | . | . | . | . | . | } | . |
| 21-225_226H12_LC | . | . | . | . | . | . | | | . | . | . | . | . | . | . | . | . | . | . | . | . | . | . | . | } | . |
| 21-225_226H9_LC | . | . | . | . | . | . | | | . | . | . | . | . | . | . | . | . | . | . | . | . | . | . | . | } | . |
| 21-225_227A8_LC | . | . | . | . | . | . | | | . | . | . | . | . | . | . | . | . | . | . | . | . | . | . | . | } | . |
| 21-225_227C1_LC | . | . | . | . | . | . | | | . | . | . | . | . | . | . | . | . | . | . | . | . | . | . | . | } | . |
| 21-225_227F2_LC | . | . | . | . | . | . | | | . | . | . | . | . | . | . | . | . | . | . | . | . | . | . | . | } | . |
| 21-225_22A1_LC | . | . | . | . | . | . | | | . | . | . | . | . | . | . | . | . | . | . | A | . | . | . | . | . | . |
| 21-225_25H9_LC | . | . | . | . | . | . | | | . | . | . | . | . | . | . | . | . | . | . | A | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | K | I | S | R | V | E | A | E | D | V | G | V | Y |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | . | . | . |
| 21-225_43E11_LC | . | . | . | . | . | . | . | . | . | . | . | . | . | . | . | . | . | . | . | . | . | . | . | . | . | . |
| 21-225_46F2_LC | . | . | . | . | . | . | . | . | . | . | . | . | . | . | L | . | . | . | . | . | . | . | . | . | . | . |
| 21-225_47C1_LC | . | . | . | . | . | . | . | . | . | . | . | . | . | . | . | . | . | . | . | . | . | . | . | . | . | R |
| 21-225_47E7_LC | . | . | . | . | . | . | . | . | . | . | A | . | . | . | . | . | . | . | . | . | . | . | . | . | . | . |
| 21-225_64H9_LC | . | . | . | . | . | . | . | . | . | . | S | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | M | Q | S | I | Q | - | - | - | - | - | - | - | - | - | - | - | - | - | - | - | - | - | - | - |
| 21-225_146B3_LC | | | | | | | | | | | | | | | | | | | | | | | | | | |
| 21-225_147E10_LC | | | | | | | | | | | | | | | | | | | | | | | | | | |
| 21-225_148B2_LC | | | | | | | | | | | | | | | | | | | | | | | | | | |
| 21-225_148G10_LC | | | | | | | | | | | | | | | | | | | | | | | | | | |
| 21-225_149F1_LC | | | | | | | | | | | | | | | | | | | | | | | | | | |
| 21-225_150B11_LC | | | | | | | | | | | | | | | | | | | | | | | | | | |
| 21-225_151A10_LC | | | | | | | | | | | | | | | | | | | | | | | | | | |
| 21-225_151G5_LC | | | | | | | | | | | | | | | | | | | | | | | | | | |
| 21-225_152C11_LC | | | | | | | | | | | | | | | | | | | | | | | | | | |
| 21-225_152F6_LC | | | | | | | | | | | | | | | | | | | | | | | | | | |
| 21-225_153D2_LC | | | | | | | | | | | | | | | | | | | | | | | | | | |
| 21-225_155A5_LC | | | | | | | | | | | | | | | | | | | | | | | | | | |
| 21-225_157G8_LC | | | | | | | | | | | | | | | | | | | | | | | | | | |
| 21-225_160B1_LC | | | | | | | | | | | | | | | | | | | | | | | | | | |
| 21-225_160G10_LC | | | | | | | | | | | | | | | | | | | | | | | | | | |
| 21-225_162H6_LC | | | M | | | | | | | | | | | | | | | | | | | | | | | |
| 21-225_167E2_LC | | | | | | | | | | | | | | | | | | | | | | | | | | |
| 21-225_167H10_LC | | | | | | | | | | | | | | | | | | | | | | | | | | |
| 21-225_170A1_LC | | | | | | | | | | | | | | | | | | | | | | | | | | |
| 21-225_172B7_LC | | | | | | | | | | | | | | | | | | | | | | | | | | |
| 21-225_173A11_LC | | | | | | | | | | | | | | | | | | | | | | | | | | |
| 21-225_176H4_LC | | | | | | | | | | | | | | | | | | | | | | | | | | |
| 21-225_177B11_LC | | | | | | | | | | | | | | | | | | | | | | | | | | |
| 21-225_178G10_LC | | | | | | | | | | | | | | | | | | | | | | | | | | |
| 21-225_180C4_LC | | | | | | | | | | | | | | | | | | | | | | | | | | |
| 21-225_181C10_LC | | | | | | | | | | | | | | | | | | | | | | | | | | |
| 21-225_181C2_LC | | | | | | | | | | | | | | | | | | | | | | | | | | |
| 21-225_186F7_LC | | | | | | | | | | | | | | | | | | | | | | | | | | |
| 21-225_188G11_LC | | | | | | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | M | Q | S | I | Q | - | - | - | - | - | - | - | - | - | - | - | - | - | - | - | - | - | - | - |
| 21-225_189G2_LC | . | . | . | . | . | . | . | . | . | . | . | . | . | . | . | . | . | . | . | . | . | . | . | . | . | . |
| 21-225_190E7_LC | . | . | . | . | . | . | . | . | . | . | . | . | . | . | . | . | . | . | . | . | . | . | . | . | . | . |
| 21-225_190H11_LC | . | . | . | . | . | . | . | . | . | . | . | . | . | . | . | . | . | . | . | . | . | . | . | . | . | . |
| 21-225_191B1_LC | . | . | . | . | . | F | . | . | . | . | . | . | . | . | . | . | . | . | . | . | . | . | . | . | . | . |
| 21-225_191E3_LC | . | . | . | . | . | . | . | . | . | . | . | . | . | . | . | . | . | . | . | . | . | . | . | . | . | . |
| 21-225_191E6_LC | . | . | . | . | . | . | H | . | . | . | . | . | . | . | . | . | . | . | . | . | . | . | . | . | . | . |
| 21-225_192F9_LC | . | . | . | . | . | . | . | . | . | . | . | . | . | . | . | . | . | . | . | . | . | . | . | . | . | . |
| 21-225_194F4_LC | . | . | . | . | . | . | . | . | . | . | . | . | . | . | . | . | . | . | . | . | . | . | . | . | . | . |
| 21-225_197G8_LC | S | . | . | . | . | . | . | . | . | . | . | . | . | . | . | . | . | . | . | . | . | . | . | . | . | . |
| 21-225_198A10_LC | . | . | . | . | . | . | . | . | . | . | . | . | . | . | . | . | . | . | . | . | . | . | . | . | . | . |
| 21-225_224A7_LC | . | . | . | . | . | T | . | . | . | . | . | . | . | . | . | . | . | . | . | . | . | . | . | . | . | . |
| 21-225_224C11_LC | . | . | . | . | . | T | . | . | . | . | . | . | . | . | . | . | . | . | . | . | . | . | . | . | . | . |
| 21-225_224G1_LC | . | . | . | . | . | T | . | . | . | . | . | . | . | . | . | . | . | . | . | . | . | . | . | . | . | . |
| 21-225_224H11_LC | . | . | . | . | . | T | . | . | . | . | . | . | . | . | . | . | . | . | . | . | . | . | . | . | . | . |
| 21-225_225G4_LC | . | . | . | . | . | T | . | . | . | . | . | . | . | . | . | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | . | . | . | . | . | . |
| 21-225_226E11_LC | . | . | . | . | . | T | . | . | . | . | . | . | . | . | . | . | . | . | . | . | . | . | . | . | . | . |
| 21-225_226E7_LC | . | . | . | . | . | . | . | . | . | . | . | . | . | . | . | . | . | . | . | . | . | . | . | . | . | . |
| 21-225_226F10_LC | . | . | . | . | . | T | . | . | . | . | . | . | . | . | . | . | . | . | . | . | . | . | . | . | . | . |
| 21-225_226F9_LC | . | . | . | . | . | T | . | . | . | . | . | . | . | . | . | . | . | . | . | . | . | . | . | . | . | . |
| 21-225_226G8_LC | . | . | . | . | . | T | . | . | . | . | . | . | . | . | . | . | . | . | . | . | . | . | . | . | . | . |
| 21-225_226H12_LC | . | . | . | . | . | T | . | . | . | . | . | . | . | . | . | . | . | . | . | . | . | . | . | . | . | . |
| 21-225_226H9_LC | . | . | . | . | . | T | . | . | . | . | . | . | . | . | . | . | . | . | . | . | . | . | . | . | . | . |
| 21-225_227A8_LC | . | . | . | . | . | T | . | . | . | . | . | . | . | . | . | . | . | . | . | . | . | . | . | . | . | . |
| 21-225_227C1_LC | . | . | . | . | . | T | . | . | . | . | . | . | . | . | . | . | . | . | . | . | . | . | . | . | . | . |
| 21-225_227F2_LC | . | . | . | . | . | T | . | . | . | . | . | . | . | . | . | . | . | . | . | . | . | . | . | . | . | . |
| 21-225_22A1_LC | . | . | . | T | L | H | . | . | . | . | . | . | . | . | . | . | . | . | . | . | . | . | . | . | . | . |
| 21-225_25H9_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | M | Q | S | I | Q | - | - | - | - | - | - | - | - | - | - | - | - | - | - | - | - | - | - | - |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | . | . | . |
| 21-225_43E11_LC | . | . | . | . | . | . | . | . | . | . | . | . | . | . | . | . | . | . | . | . | . | . | . | . | . | . |
| 21-225_46F2_LC | . | . | . | . | . | . | . | . | . | . | . | . | . | . | . | . | . | . | . | . | . | . | . | . | . | . |
| 21-225_47C1_LC | . | . | . | . | . | . | . | . | . | . | . | . | . | . | . | . | . | . | . | . | . | . | . | . | . | . |
| 21-225_47E7_LC | . | . | . | . | . | T | . | . | . | . | . | . | . | . | . | . | . | . | . | . | . | . | . | . | . | . |
| 21-225_64H9_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | L | P | W | T | F | G | Q | G | T | K | V | E | I | K | R |
| 21-225_146B3_LC | . | . | | . | . | . | . | | . | . | . | . | . | . | . | . | . | . | |
| 21-225_147E10_LC | . | . | . | . | V | . | . | . | . | . | . | . | . | . | . | . | . | . | . |
| 21-225_148B2_LC | . | . | | . | I | . | . | . | . | . | . | . | . | . | . | . | . | . | |
| 21-225_148G10_LC | . | . | | . | . | . | . | . | . | . | . | . | . | . | . | . | . | . | . |
| 21-225_149F1_LC | . | . | | . | I | . | . | . | . | . | . | . | . | . | . | . | . | . | |
| 21-225_150B11_LC | . | . | | . | . | . | . | . | . | . | . | . | . | . | . | . | . | . | |
| 21-225_151A10_LC | . | . | | . | I | . | . | . | . | . | . | . | . | . | . | . | . | . | |
| 21-225_151G5_LC | . | . | | . | V | . | . | . | . | . | . | . | . | . | . | . | . | . | |
| 21-225_152C11_LC | . | . | | . | I | . | . | . | . | . | . | . | . | . | . | D | . | . | |
| 21-225_152F6_LC | . | . | | . | V | . | . | . | . | . | . | . | . | . | . | . | . | . | |
| 21-225_153D2_LC | . | . | | . | V | . | . | . | . | . | . | . | . | . | . | . | . | . | |
| 21-225_155A5_LC | . | . | | . | V | . | . | . | . | . | . | . | . | . | . | . | . | . | |
| 21-225_157G8_LC | . | . | | . | V | . | . | . | . | . | . | . | . | . | . | . | . | . | |
| 21-225_160B1_LC | . | . | | . | I | . | . | . | | . | . | . | . | . | . | . | . | . | |
| 21-225_160G10_LC | . | . | | | . | . | . | . | . | V | . | . | . | . | . | . | . | T | |
| 21-225_162H6_LC | . | . | | . | . | . | . | . | . | . | . | . | . | . | . | . | . | . | |
| 21-225_167E2_LC | . | . | | . | . | . | . | . | . | . | . | . | . | . | . | . | . | . | |
| 21-225_167H10_LC | . | . | | . | . | . | . | . | . | . | . | . | . | . | . | . | . | . | |
| 21-225_170A1_LC | . | . | | . | . | . | . | . | . | . | . | . | . | . | . | . | . | . | |
| 21-225_172B7_LC | . | . | | . | I | . | . | . | . | . | . | . | . | D | . | D | . | . | |
| 21-225_173A11_LC | . | . | | . | V | . | . | . | . | . | . | . | . | . | . | . | . | . | |
| 21-225_176H4_LC | . | . | | . | I | . | . | . | . | . | . | . | . | R | . | . | . | . | |
| 21-225_177B11_LC | . | . | | . | I | . | . | . | . | . | . | . | . | . | . | . | . | . | |
| 21-225_178G10_LC | . | . | | . | V | . | . | . | . | . | . | . | . | . | . | . | . | . | |
| 21-225_180C4_LC | . | . | | . | V | . | . | . | . | . | . | . | . | . | . | . | . | . | |
| 21-225_181C10_LC | . | . | | . | I | . | . | . | . | . | . | . | . | . | . | . | . | . | |
| 21-225_181C2_LC | . | . | | . | V | . | . | . | . | . | . | . | . | . | . | . | . | . | |
| 21-225_186F7_LC | . | . | | . | V | . | . | . | . | . | . | . | . | . | . | . | . | . | |
| 21-225_188G11_LC | . | . | | . | I | . | . | . | . | . | . | . | . | . | . | . | . | . | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | K_FR4 | | | | | | | |
| CONSENSUS | - | - | - | - | L | P | W | T | F | G | Q | G | T | K | V | E | I | K | R |
| 21-225_189G2_LC | . | . | . | . | } | . | . | . | . | . | . | . | . | . | . | . | . | . | . |
| 21-225_190E7_LC | . | . | . | . | . | . | . | . | . | . | . | . | . | . | . | . | . | . | . |
| 21-225_190H11_LC | . | . | . | . | S | . | . | . | . | . | . | . | . | . | . | . | . | . | . |
| 21-225_191B1_LC | . | . | . | . | . | . | . | . | . | . | . | . | . | . | . | . | . | N | . |
| 21-225_191E3_LC | . | . | . | . | . | . | . | . | . | . | . | . | . | . | . | . | . | . | . |
| 21-225_191E6_LC | . | . | . | . | S | . | . | . | . | . | . | . | . | . | . | . | . | . | . |
| 21-225_192F9_LC | . | . | . | . | . | . | . | . | . | . | . | . | . | . | . | . | . | . | . |
| 21-225_194F4_LC | . | . | . | . | . | . | . | . | . | . | . | . | . | . | . | . | . | . | . |
| 21-225_197G8_LC | . | . | . | . | . | . | . | . | A | . | . | . | S | . | . | . | . | . | . |
| 21-225_198A10_LC | . | . | . | . | . | . | . | . | . | . | . | . | . | . | . | . | . | . | . |
| 21-225_224A7_LC | . | . | . | . | . | . | S | . | . | . | . | . | . | . | . | . | . | . | . |
| 21-225_224C11_LC | . | . | . | . | . | . | S | . | . | . | . | . | . | . | . | . | . | . | . |
| 21-225_224G1_LC | . | . | . | . | . | . | S | . | . | . | . | . | . | . | . | . | . | . | . |
| 21-225_224H11_LC | . | . | . | . | . | . | S | . | . | . | . | . | . | . | . | . | . | . | . |
| 21-225_225G4_LC | . | . | . | . | . | . | S | . | . | . | . | . | . | . | . | . | . | . | . |
| 21-225_226A5_LC | . | . | . | . | } | . | . | . | . | . | . | . | . | . | . | . | . | . | . |
| 21-225_226B1_LC | . | . | . | . | } | . | . | . | . | . | . | . | . | . | . | C | . | . | . |
| 21-225_226E11_LC | . | . | . | . | . | . | S | . | . | . | . | . | . | . | . | . | . | . | . |
| 21-225_226E7_LC | . | . | . | . | V | . | . | . | . | . | . | . | . | . | . | . | . | . | . |
| 21-225_226F10_LC | . | . | . | . | . | . | S | . | . | . | . | . | . | . | . | . | . | . | . |
| 21-225_226F9_LC | . | . | . | . | . | . | S | . | . | . | . | . | . | . | . | . | . | . | . |
| 21-225_226G8_LC | . | . | . | . | . | . | S | . | . | . | . | . | . | . | . | . | . | . | . |
| 21-225_226H12_LC | . | . | . | . | . | . | S | . | . | . | . | . | . | . | . | . | . | . | . |
| 21-225_226H9_LC | . | . | . | . | . | . | S | . | . | . | . | . | . | . | . | . | . | . | . |
| 21-225_227A8_LC | . | . | . | . | . | . | S | . | . | . | . | . | S | . | . | . | . | . | . |
| 21-225_227C1_LC | . | . | . | . | . | . | S | . | . | . | . | . | . | . | . | . | . | . | . |
| 21-225_227F2_LC | . | . | . | . | . | . | S | . | . | . | . | . | . | . | . | . | . | . | . |
| 21-225_22A1_LC | . | . | . | . | . | . | . | . | . | . | . | . | . | . | . | . | . | . | . |
| 21-225_25H9_LC | . | . | . | . | . | . | . | . | . | . | . | . | . | . | . | . | . | . | . |

**EP 4 435 105 A2**

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | L | P | W | T | F | G | Q | G | T | K | V | E | I | K | R |
| 21-225_26F8_LC | . | | . | . | . | | | | . | . | . | . | . | . | . | . | . | | |
| 21-225_28G3_LC | . | | . | . | I | . | . | . | . | . | . | . | . | . | . | . | . | . | . |
| 21-225_43E11_LC | . | . | . | . | . | . | . | . | . | . | . | . | . | . | . | . | . | . | . |
| 21-225_46F2_LC | . | . | . | . | . | | . | . | . | . | . | . | . | . | . | . | . | | |
| 21-225_47C1_LC | . | . | . | . | . | . | . | . | . | . | . | . | . | . | . | . | . | . | . |
| 21-225_47E7_LC | . | . | . | . | . | . | . | . | . | . | . | . | . | . | A | . | . | . | . |
| 21-225_64H9_LC | . | . | . | . | V | . | . | . | . | . | . | . | . | . | . | . | . | Y | . |

**FIGURE 57**

Table 106. Consensus 26 - VK1|O12/JK3 (SEQ ID NO: 50323):

DIQMTQSPSSLSASVGDRVTITCRAS--QSIS------SYLNWYQQKPGKAPKLLIYA-------ASSLQSGVPSRFSGSGSG--TDFTLTISSLQPEDFATYYCQQSYS--------------------PPFTFGPGTKVDIKR wherein:

A at position 25 can be substituted with S or T

S at position 26 can be substituted with G or I

Q at position 29 can be substituted with H or R

S at position 30 can be substituted with N or T

I at position 31 can be substituted with F

S at position 32 can be substituted with I, F, N, R, Y, T, A, G or L

S at position 39 can be substituted with N, T, H or K

Y at position 40 can be substituted with F or H

L at position 41 can be substituted with V

N at position 42 can be substituted with I, M, H or Y

A at position 58 can be substituted with G, T, V or I

A at position 67 can be substituted with T, V or S

S at position 68 can be substituted with F

S at position 69 can be substituted with N, T or V

Q at position 71 can be substituted with II

S at position 72 can be substituted with N, G, H, I or T

S at position 109 can be substituted with T or Y

Y at position 110 can be substituted with N, F or H

FIGURE 57

S at position 111 can be substituted with R, N, F or I

null (-) at position 134 can be substituted with A or S

P at position 135 can be substituted with T, I, F, A, L or V

P at position 136 can be substituted with L, F, or S

T at position 138 can be substituted with A or S

Arg Xaa Xaa Xaa Xaa Xaa Xaa Xaa Xaa Xaa Xaa (SEQ ID NO: 50153)

Xaa Xaa Xaa Xaa Leu Xaa Xaa (SEQ ID NO: 50154)

Gln Gln Xaa Xaa Xaa Xaa Xaa Xaa Phe Xaa (SEQ ID NO: 50155)

Arg Ala Ser Gln Ser Ile Ser Ser Tyr Leu Asn (SEQ ID NO: 50639)

Ala Ala Ser Ser Leu Gln Ser (SEQ ID NO: 50640)

Gln Gln Ser Tyr Ser Pro Pro Phe Thr (SEQ ID NO: 50641)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_10F12_LC | . | . | . | . | . | . | . | . | . | . | . | . | . | . | . | . | . | . | . | . | . | . | . | . | . | . |
| 21-225_11A5_LC | . | . | . | . | . | . | . | . | . | . | . | . | . | . | . | . | . | . | . | . | . | . | . | . | . | . |
| 21-225_14A11_LC | . | . | . | . | . | . | . | . | . | . | . | . | . | I | . | . | . | . | . | . | . | . | . | . | . | . |
| 21-225_158F4_LC | . | . | . | . | . | . | . | . | . | . | . | P | . | . | . | . | . | . | . | . | . | . | . | . | . | . |
| 21-225_16H6_LC | . | . | . | . | . | . | . | . | . | . | . | . | . | . | . | . | . | . | . | . | . | . | . | . | . | . |
| 21-225_17A12_LC | . | . | . | . | . | . | . | . | . | . | . | . | . | . | . | . | . | . | . | . | . | . | . | . | . | . |
| 21-225_17B4_LC | . | . | . | . | . | . | . | . | . | . | . | . | . | . | . | . | . | . | . | . | . | . | . | . | . | . |
| 21-225_17C6_LC | . | . | . | . | . | . | . | . | . | . | . | . | . | . | . | . | . | . | . | . | . | . | . | . | . | . |
| 21-225_17F11_LC | . | . | . | . | . | . | . | . | . | . | . | . | . | I | . | . | . | . | . | S | . | . | . | . | . | . |
| 21-225_17F7_LC | . | . | . | . | . | . | . | . | . | . | . | . | . | . | . | . | . | . | . | . | . | . | . | . | . | . |
| 21-225_180F8_LC | . | . | . | . | . | . | . | . | . | . | . | . | . | . | . | . | G | . | . | . | . | . | . | . | . | . |
| 21-225_18E12_LC | . | . | . | . | . | . | . | . | . | . | . | . | . | . | . | . | . | . | . | . | . | . | . | . | . | . |
| 21-225_19A5_LC | . | . | . | . | . | . | . | . | . | . | . | P | . | . | . | . | . | . | . | S | . | . | . | . | . | . |
| 21-225_19B3_LC | . | . | . | . | . | . | . | . | . | . | . | . | . | . | . | . | . | I | . | . | . | . | . | . | . | G |
| 21-225_20A4_LC | . | . | . | . | . | . | . | A | . | . | . | . | . | . | . | . | . | . | . | . | . | . | . | . | . | . |
| 21-225_20F3_LC | . | . | . | . | . | . | . | . | . | . | . | . | . | . | . | . | . | . | . | . | . | . | . | . | . | . |
| 21-225_21A2_LC | . | . | . | . | . | . | . | . | . | . | . | . | . | . | . | . | . | . | . | . | . | . | . | . | . | . |
| 21-225_21A6_LC | . | . | . | . | . | . | . | . | . | G | . | . | . | . | . | . | . | . | . | . | . | . | . | . | . | . |
| 21-225_21F4_LC | . | . | . | . | . | . | . | . | . | . | . | . | . | . | . | . | . | . | . | . | . | . | . | . | . | . |
| 21-225_222H4_LC | . | . | . | N | . | . | . | . | . | . | . | . | . | . | . | . | . | . | . | . | . | . | . | . | . | . |
| 21-225_222H8_LC | . | . | . | N | . | . | . | . | . | . | . | . | . | . | . | . | . | . | . | . | . | . | . | . | . | . |
| 21-225_22C5_LC | . | . | . | . | . | . | . | A | . | . | . | . | . | . | . | . | . | . | . | . | . | . | . | . | . | . |
| 21-225_22D5_LC | . | . | . | . | . | . | . | . | . | . | . | . | . | . | . | . | . | . | . | . | . | . | . | . | . | . |
| 21-225_22E4_LC | . | . | . | . | . | . | . | . | . | . | . | . | . | . | . | . | . | . | . | . | . | . | . | . | . | . |
| 21-225_22G10_LC | . | . | . | . | . | . | . | . | . | . | . | . | . | . | . | . | . | . | . | . | . | . | . | . | . | . |
| 21-225_22G3_LC | . | . | . | . | . | . | . | . | . | . | . | . | . | . | . | . | . | . | . | . | . | . | . | . | . | . |
| 21-225_22G5_LC | . | . | . | . | . | . | . | . | . | . | . | . | . | . | . | . | . | . | . | . | . | . | . | . | . | . |
| 21-225_30E11_LC | . | . | . | . | . | . | . | . | . | . | . | . | . | . | . | . | . | . | . | . | . | . | . | . | . | . |
| 21-225_33G12_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_3B1_LC | . | . | . | . | . | . | . | . | . | . | . | . | . | . | . | . | . | . | . | . | . | . | . | . | . | . |
| 21-225_3E3_LC | . | . | . | . | . | . | . | . | . | . | P | . | . | . | . | . | . | . | . | . | . | . | P | . | . | . |
| 21-225_48C3_LC | . | . | . | . | . | . | . | . | . | . | . | . | . | . | . | . | . | . | . | N | . | . | . | . | . | . |
| 21-225_48D1_LC | . | . | . | . | . | . | . | . | . | . | . | . | . | . | . | . | . | . | . | N | . | . | . | . | . | . |
| 21-225_49G12_LC | . | . | . | . | . | . | . | . | . | . | . | . | . | . | . | . | . | . | . | . | . | . | . | . | S | . |
| 21-225_4B1_LC | . | . | . | . | . | . | . | . | . | . | . | . | . | . | . | . | . | . | . | . | . | . | . | . | . | . |
| 21-225_4H2_LC | . | . | . | . | . | L | . | . | . | . | . | . | . | . | . | . | . | . | . | . | . | . | . | . | . | I |
| 21-225_50B12_LC | . | . | . | . | . | . | . | . | . | . | . | . | . | . | . | . | . | . | . | . | . | . | . | . | . | . |
| 21-225_50H10_LC | . | . | . | . | . | . | . | . | . | . | . | . | . | . | . | . | . | . | . | N | . | . | . | . | . | . |
| 21-225_50H8_LC | . | . | . | . | . | . | . | . | . | . | . | . | . | . | . | . | . | . | . | N | . | . | . | . | . | . |
| 21-225_51H10_LC | . | . | . | . | . | . | . | . | . | . | . | . | . | . | . | . | . | . | . | . | . | . | . | . | . | . |
| 21-225_53E2_LC | . | . | . | . | . | . | . | . | . | . | . | . | . | . | . | . | . | . | . | . | . | . | . | . | . | . |
| 21-225_56B2_LC | . | . | . | . | . | . | . | . | . | . | . | . | . | . | . | . | . | . | . | . | . | . | . | . | . | . |
| 21-225_57F2_LC | . | . | . | . | . | . | . | . | . | . | . | . | . | . | . | . | . | . | . | . | . | . | . | . | . | . |
| 21-225_5F7_LC | . | . | . | . | . | . | . | . | . | . | P | . | . | . | . | . | . | . | . | . | . | . | P | . | . | . |
| 21-225_5G2_LC | . | . | . | . | . | . | . | . | . | . | P | . | . | . | . | . | . | . | . | . | . | . | P | . | . | . |
| 21-225_60E12_LC | . | . | . | . | . | . | . | . | . | . | . | . | . | . | . | . | . | . | . | . | . | . | . | . | . | . |
| 21-225_61A1_LC | . | . | . | . | . | . | . | . | . | . | . | . | . | . | . | . | . | . | . | . | . | . | . | . | . | . |
| 21-225_62H1_LC | . | . | . | . | . | . | . | . | . | . | . | . | . | Y | . | . | . | . | . | . | . | . | . | . | . | . |
| 21-225_62H10_LC | . | . | . | . | . | . | . | . | . | . | . | . | . | . | . | . | . | . | . | . | . | . | . | . | . | . |
| 21-225_62H8_LC | . | . | . | . | . | . | . | . | . | . | . | . | . | . | . | . | . | . | . | . | . | . | . | . | . | . |
| 21-225_66A9_LC | . | . | . | . | . | . | . | . | . | . | . | . | . | . | . | . | . | . | . | . | . | . | . | . | . | . |
| 21-225_69C8_LC | . | . | . | . | . | . | . | . | . | . | . | . | . | . | . | . | . | . | . | . | P | . | . | . | . | G |
| 21-225_6D3_LC | . | . | . | . | . | . | . | . | . | . | . | . | . | . | . | . | . | . | . | . | . | . | . | Y | . | . |
| 21-225_72G9_LC | . | . | . | . | . | . | . | . | . | . | . | . | . | . | . | . | . | . | . | . | . | . | . | . | . | . |
| 21-225_8E12_LC | . | . | . | . | . | . | . | . | . | . | . | . | . | . | . | . | . | . | . | . | . | . | . | . | . | . |
| 21-225_8G6_LC | . | . | . | . | . | . | . | . | . | . | . | . | . | . | . | . | . | . | . | . | . | . | . | . | . | . |
| 21-225_8H11_LC | . | . | . | . | . | . | . | . | . | . | . | . | . | P | . | . | . | . | . | . | . | . | . | . | . | . |
| 21-225_9E5_LC | . | . | . | . | . | . | Q | . | . | . | . | . | . | . | . | . | . | . | . | . | . | . | . | . | . | . |

4740

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | I | S | - | - | - | - | - | - | S | Y | L | N | W | Y | Q | Q | K | P | G | K | A | P |
| 21-225_10F12_LC | . | . | . | N | . | . | . | . | . | . | . | . | . | . | . | . | . | . | . | . | . | . | . | . | . | . |
| 21-225_11A5_LC | . | . | . | N | . | Y | . | . | . | . | . | . | . | . | . | . | . | . | . | . | . | . | . | . | . | . |
| 21-225_14A11_LC | . | . | . | . | . | . | . | . | . | . | . | . | . | . | . | . | . | . | . | . | . | . | . | . | . | . |
| 21-225_158F4_LC | . | . | . | N | . | R | . | . | . | . | . | . | K | . | . | K | . | . | . | . | P | L | . | . | . | . |
| 21-225_16H6_LC | . | . | . | N | . | . | . | . | . | . | . | . | . | . | . | . | . | . | . | . | . | . | . | . | . | . |
| 21-225_17A12_LC | . | . | . | . | . | . | . | . | . | . | . | . | . | . | . | . | . | . | . | K | . | . | . | . | . | . |
| 21-225_17B4_LC | . | . | . | N | . | . | . | . | . | . | . | . | . | . | . | . | . | . | . | . | . | . | . | . | . | . |
| 21-225_17C6_LC | . | . | . | . | . | N | . | . | . | . | . | . | Y | . | . | . | . | . | . | . | . | . | . | . | . | . |
| 21-225_17F11_LC | . | . | K | T | . | N | . | . | . | . | . | . | N | . | . | . | . | . | . | . | . | . | . | . | . | . |
| 21-225_17F7_LC | . | . | . | T | . | . | . | . | . | . | . | . | . | P | . | . | . | . | . | . | . | . | . | . | . | . |
| 21-225_180F8_LC | . | . | . | T | . | L | . | . | . | . | . | . | . | . | . | . | . | . | . | . | . | . | . | . | . | . |
| 21-225_18E12_LC | . | . | . | . | . | . | . | . | . | . | . | . | . | . | . | . | . | . | . | . | . | . | . | . | . | . |
| 21-225_19A5_LC | . | . | . | N | . | I | . | . | . | . | . | . | N | . | . | . | . | . | . | . | . | . | . | . | . | . |
| 21-225_19B3_LC | . | . | . | N | . | I | . | . | . | . | . | . | N | . | . | . | . | . | . | . | . | . | . | . | . | . |
| 21-225_20A4_LC | . | . | . | . | . | I | . | . | . | . | . | . | N | . | . | . | . | . | . | . | K | . | . | . | S | . |
| 21-225_20F3_LC | . | . | . | . | . | . | . | . | . | . | . | . | . | . | . | Y | . | . | . | . | . | . | . | . | . | . |
| 21-225_21A2_LC | . | . | . | . | . | . | . | . | . | . | . | . | . | . | . | . | . | . | . | . | . | . | . | . | . | . |
| 21-225_21A5_LC | . | . | . | T | . | . | . | . | . | . | . | . | . | K | . | . | . | . | . | K | . | . | . | . | . | . |
| 21-225_21F4_LC | . | . | . | N | . | G | . | . | . | . | . | . | . | . | . | . | . | . | . | . | . | . | . | . | . | . |
| 21-225_222H4_LC | . | . | . | N | . | . | . | . | . | . | . | . | N | . | V | . | . | . | . | . | . | . | . | . | . | . |
| 21-225_222H8_LC | . | . | . | N | . | . | . | . | . | . | . | . | N | . | V | . | . | . | . | . | . | . | . | . | . | . |
| 21-225_22C5_LC | . | . | . | N | . | . | . | . | . | . | . | . | . | . | . | . | . | . | . | . | . | . | . | . | . | . |
| 21-225_22D5_LC | . | . | . | N | . | P | . | . | . | . | . | . | . | . | . | . | . | . | . | K | . | . | . | . | . | . |
| 21-225_22E4_LC | . | . | . | N | . | . | . | . | . | . | . | . | . | . | . | . | . | . | . | . | . | . | . | . | . | . |
| 21-225_22G10_LC | . | . | . | . | . | P | . | . | . | . | . | . | . | . | . | . | . | . | . | . | . | . | . | . | . | . |
| 21-225_22G3_LC | . | . | . | . | . | . | . | . | . | . | . | . | N | . | . | . | . | . | . | . | . | . | . | . | . | . |
| 21-225_22G5_LC | . | . | . | N | . | . | . | . | . | . | . | . | . | . | . | . | . | . | . | . | . | Q | . | . | . | . |
| 21-225_30E11_LC | . | . | . | . | . | . | . | . | . | . | . | . | N | . | . | . | . | . | . | . | Q | T | . | . | . | . |
| 21-225_33G12_LC | . | . | . | . | . | . | . | . | . | . | . | . | N | . | . | . | . | . | . | . | Q | T | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | K_CDR1 | | | | | | | | | | | | | | | | | | K_FR2 | | | |
| CONSENSUS | - | - | Q | S | I | S | - | - | - | - | - | - | S | Y | L | N | W | Y | Q | Q | K | P | G | K | A | P |
| 21-225_3B1_LC | . | . | . | . | . | N | . | . | . | . | . | . | N | . | . | . | . | . | . | . | . | . | . | . | . | . |
| 21-225_3E3_LC | . | . | . | . | . | R | . | . | . | . | . | . | . | . | . | . | . | . | . | . | . | . | . | . | . | R |
| 21-225_48C3_LC | . | . | . | . | . | I | . | . | . | . | . | . | . | . | . | I | . | . | . | . | . | . | . | . | . | . |
| 21-225_48D1_LC | . | . | . | . | . | . | . | . | . | . | . | . | . | . | . | I | . | . | . | . | . | . | . | . | . | . |
| 21-225_49G12_LC | . | . | . | . | . | . | . | . | . | . | . | . | T | . | . | M | . | . | . | . | . | . | . | . | . | . |
| 21-225_4B1_LC | . | . | N | T | . | R | . | . | . | . | . | . | T | . | . | . | . | . | . | . | . | . | . | . | . | . |
| 21-225_4H2_LC | . | . | . | N | . | . | . | . | . | . | . | . | . | . | . | . | . | . | . | . | . | . | . | . | . | . |
| 21-225_50B12_LC | . | . | . | . | . | . | . | . | . | . | . | . | . | . | . | . | . | . | . | N | . | . | . | . | . | . |
| 21-225_50H10_LC | . | . | . | . | . | Y | . | . | . | . | . | . | . | . | . | I | . | . | . | . | . | . | . | . | . | . |
| 21-225_50H8_LC | . | . | . | . | . | . | . | . | . | . | . | . | . | . | . | I | . | . | . | . | . | . | . | . | . | . |
| 21-225_51H10_LC | . | . | . | . | . | . | . | . | . | . | . | . | T | . | . | M | . | . | . | . | . | . | . | . | . | . |
| 21-225_53E2_LC | . | . | . | . | F | . | . | . | . | . | . | . | M | . | . | . | . | . | . | . | . | . | . | . | . | . |
| 21-225_56B2_LC | . | . | . | . | F | . | . | . | . | . | . | . | M | . | . | . | . | . | . | . | . | . | . | . | . | . |
| 21-225_57F2_LC | . | . | . | . | . | . | . | . | . | . | . | . | . | . | . | . | . | . | . | . | . | . | . | . | . | . |
| 21-225_5F7_LC | . | . | . | T | . | . | . | . | . | . | . | . | . | . | . | . | . | . | . | . | . | . | . | . | . | . |
| 21-225_5G2_LC | . | . | . | N | . | I | . | . | . | . | . | . | . | . | . | . | . | . | . | . | . | . | . | . | . | R |
| 21-225_60E12_LC | . | . | . | . | . | . | . | . | . | . | . | . | . | . | . | . | . | . | . | . | . | . | . | . | . | . |
| 21-225_61A1_LC | . | . | . | . | . | . | . | . | . | . | . | . | . | . | . | . | . | . | . | . | . | . | . | . | . | . |
| 21-225_62H1_LC | . | . | . | N | . | F | . | . | . | . | . | . | . | . | . | . | . | . | . | . | . | . | . | . | . | . |
| 21-225_62H10_LC | . | . | . | . | . | T | . | . | . | . | . | . | . | . | . | . | . | . | . | . | . | . | . | . | V | . |
| 21-225_62H8_LC | . | . | . | . | . | F | . | . | . | . | . | . | . | . | . | . | . | . | . | . | N | . | . | . | . | . |
| 21-225_66A9_LC | . | . | . | N | . | F | . | . | . | . | . | . | . | . | . | . | . | . | . | . | . | . | . | . | . | . |
| 21-225_69C8_LC | . | . | . | T | . | Y | . | . | . | . | . | . | N | . | . | . | . | . | . | . | . | . | . | . | . | . |
| 21-225_6D3_LC | . | . | . | . | . | . | . | . | . | . | . | . | T | . | . | . | . | . | . | . | . | . | . | . | . | . |
| 21-225_72G9_LC | . | . | . | . | . | T | . | . | . | . | . | . | . | . | . | . | . | . | . | . | . | . | . | . | . | . |
| 21-225_8E12_LC | . | . | . | . | . | . | . | . | . | . | . | . | N | . | . | . | . | . | . | . | . | . | . | . | . | . |
| 21-225_8G6_LC | . | . | . | . | . | . | . | . | . | . | . | . | . | . | . | . | . | . | . | . | . | . | . | . | . | . |
| 21-225_8H11_LC | . | . | . | N | . | I | . | . | . | . | . | . | . | . | . | . | . | . | . | . | . | . | . | . | . | . |
| 21-225_9E5_LC | . | . | . | . | . | A | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | A | - | - | - | - | - | - | - | - | A | S | S | L | Q | S | G | V | P | S | R | F |
| 21-225_10F12_LC | | | | | | | | | | | | | | | | | | | | | | | | | | |
| 21-225_11A5_LC | | | | | | | | | | | | | | | | | | | | | | | | | | |
| 21-225_14A11_LC | | | | | F | | | | | | | | | | | | | | | N | | | | | | |
| 21-225_158F4_LC | | | | | | T | | | | | | | | | | | T | | | | | | | | | |
| 21-225_16H6_LC | | | | | | | | | | | | | | | | | | | | | | | | | | |
| 21-225_17A12_LC | | | | | | | | | | | | | | | | | | | | | | | | | | |
| 21-225_17B4_LC | | | | | F | Q | | | | | | | | | | | | | | T | | | | | | |
| 21-225_17C6_LC | | | | | | | | | | | | | | | | | | | | | | | | | | |
| 21-225_17F11_LC | | | | | F | | | | | | | | | | Y | | | | | | | | | | | |
| 21-225_17F7_LC | | | | | | | | | | | | | | | | | | | | | | | | | | |
| 21-225_180F8_LC | | | | | | G | | | | | | | | | V | | | | | | | | | | | |
| 21-225_18E12_LC | T | | | | | | | | | | | | | | | | | | | | | | | | | |
| 21-225_19A5_LC | | | | | Q | V | | | | | | | | | | | N | | | Q | | | | | | |
| 21-225_19B3_LC | N | | | | | V | | | | | | | | | | | | | | | | | | | | |
| 21-225_20A4_LC | | | | | | | | | | | | | | | | | | | | M | | | | | | |
| 21-225_20F3_LC | | | | | | | | | | | | | | | | | | | | | | | | | | |
| 21-225_21A2_LC | | | | | | | | | | | | | | | | | | | | | | | | | | |
| 21-225_21A5_LC | | | | | | | | | | | | | | | | | | | | | | | | | | |
| 21-225_21F4_LC | | | | | F | | | | | | | | | | | | | | | | | | | | | |
| 21-225_222H4_LC | | F | | | | T | | | | | | | | | | | | | | | | | S | | | |
| 21-225_222H8_LC | | F | | | | I | | | | | | | | | | | | | | | | | S | | | |
| 21-225_22C5_LC | | | | | | | | | | | | | | | | | V | | | M | | I | | | | |
| 21-225_22D5_LC | | | | | | | | | | | | | | | | | | | | | | | | | | |
| 21-225_22E4_LC | | | | | | | | | | | | | | | | | | | | | | | | | | |
| 21-225_22G10_LC | | | | | | | | | | | | | | | | | | | | | | | | | | |
| 21-225_22G3_LC | | | | | F | | | | | | | | | | | | | | | | | | | | | |
| 21-225_22G5_LC | | | | | | | | | | | | | | | | | | | | N | | | | | | |
| 21-225_30E11_LC | | F | | | | | | | | | | | | | | | | | | | | | | | | |
| 21-225_33G12_LC | | F | | | | | | | | | | | | | | | | | | | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_3B1_LC | . | . | . | . | Y | . | . | . | . | . | . | . | . | . | T | . | . | . | . | N | . | . | . | . | . | . |
| 21-225_3E3_LC | . | . | V | . | . | G | . | . | . | . | . | . | . | . | T | . | . | . | . | . | . | . | . | . | . | . |
| 21-225_48C3_LC | R | . | . | . | . | . | . | . | . | . | . | . | . | . | . | . | . | . | . | . | . | . | . | . | . | . |
| 21-225_48D1_LC | . | . | . | . | . | . | . | . | . | . | . | . | . | . | . | . | . | . | . | . | . | . | . | . | . | . |
| 21-225_49G12_LC | . | . | . | . | . | . | . | . | . | . | . | . | . | . | . | . | . | . | I | . | . | . | . | . | E | . |
| 21-225_4B1_LC | . | . | . | . | . | . | . | . | . | . | . | . | . | . | . | . | . | . | . | . | . | . | . | . | . | I |
| 21-225_4H2_LC | . | . | V | . | . | . | . | . | . | . | . | . | . | . | . | . | . | . | . | . | . | . | . | . | . | . |
| 21-225_50B12_LC | R | . | . | . | . | . | . | . | . | . | . | . | . | . | . | . | . | . | . | . | . | . | . | . | . | . |
| 21-225_50H10_LC | . | . | . | . | . | . | . | . | . | . | . | . | . | . | . | . | . | . | . | . | . | . | . | . | . | . |
| 21-225_50H8_LC | . | . | . | . | . | . | . | . | . | . | . | . | . | . | . | . | . | . | . | . | . | . | . | . | . | . |
| 21-225_51H10_LC | . | . | . | . | . | . | . | . | . | . | . | . | . | . | . | . | . | . | . | . | . | . | . | . | E | . |
| 21-225_53E2_LC | N | . | . | . | F | . | . | . | . | . | . | . | . | . | V | . | . | . | . | . | . | . | . | . | . | . |
| 21-225_56B2_LC | N | . | . | . | F | . | . | . | . | . | . | . | . | . | V | . | . | . | . | . | . | . | . | . | . | . |
| 21-225_57F2_LC | . | . | . | . | . | . | . | . | . | . | . | . | . | . | . | . | . | . | . | . | . | . | . | . | . | . |
| 21-225_5F7_LC | . | . | . | . | S | . | . | . | . | . | . | . | . | . | . | . | . | . | . | . | . | . | . | . | . | . |
| 21-225_5G2_LC | . | . | V | . | . | G | . | . | . | . | . | . | . | . | . | . | . | . | . | . | . | . | . | . | . | . |
| 21-225_60E12_LC | . | . | . | . | . | . | . | . | . | . | . | . | . | . | . | . | . | . | . | . | . | . | . | . | . | . |
| 21-225_61A1_LC | . | . | . | . | . | . | . | . | . | . | . | . | . | . | . | . | . | . | . | . | . | . | . | . | . | . |
| 21-225_62H1_LC | . | V | . | . | . | . | . | . | . | . | . | . | . | . | V | P | . | . | . | . | . | . | . | . | . | . |
| 21-225_62H10_LC | . | . | . | . | S | T | . | . | . | . | . | . | . | . | . | . | . | . | . | . | . | . | . | . | . | . |
| 21-225_62H8_LC | . | . | . | . | . | . | . | . | . | . | . | . | . | . | S | . | . | . | . | . | . | . | . | . | . | L |
| 21-225_66A9_LC | . | . | . | . | F | . | . | . | . | . | . | . | . | . | . | . | . | . | . | . | . | . | . | . | . | . |
| 21-225_69C8_LC | . | . | . | . | K | V | . | . | . | . | . | . | . | . | . | . | . | . | . | . | . | . | . | . | . | . |
| 21-225_6D3_LC | . | . | . | . | F | . | . | . | . | . | . | . | . | . | . | . | . | . | . | . | . | . | . | . | . | . |
| 21-225_72G9_LC | . | . | . | . | . | T | . | . | . | . | . | . | . | . | . | . | . | . | . | . | . | . | . | . | . | . |
| 21-225_8E12_LC | . | . | . | . | . | . | . | . | . | . | . | . | . | . | . | . | . | . | . | . | . | . | . | . | . | . |
| 21-225_8G6_LC | . | . | . | . | F | . | . | . | . | . | . | . | . | . | . | . | . | . | . | . | . | . | . | . | . | . |
| 21-225_8H11_LC | N | . | M | . | . | V | . | . | . | . | . | . | . | . | T | . | . | . | . | R | . | . | . | . | . | . |
| 21-225_9E5_LC | . | . | . | . | . | G | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 K_CDR3 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| CONSENSUS | Y | C | Q | Q | Y | S | T | - | - | - | - | - | - | - | - | - | - | - | - | - | - | - | - | - | - | - |
| 21-225_212E6_LC | . | . | . | . | . | V | . | . | . | . | . | . | . | . | . | . | . | . | . | . | . | . | . | . | . | . |
| 21-225_212F10_LC | . | . | . | H | . | . | N | . | . | . | . | . | . | . | . | . | . | . | . | . | . | . | . | . | . | . |
| 21-225_212H11_LC | . | . | . | H | . | . | N | . | . | . | . | . | . | . | . | . | . | . | . | . | . | . | . | . | . | . |
| 21-225_212H9_LC | . | . | L | H | . | D | . | . | . | . | . | . | . | . | . | . | . | . | . | . | . | . | . | . | . | . |
| 21-225_213B8_LC | . | . | . | . | . | . | N | . | . | . | . | . | . | . | . | . | . | . | . | . | . | . | . | . | . | . |
| 21-225_213C4_LC | . | . | . | . | . | . | N | . | . | . | . | . | . | . | . | . | . | . | . | . | . | . | . | . | . | . |
| 21-225_213D2_LC | . | . | H | . | . | . | N | . | . | . | . | . | . | . | . | . | . | . | . | . | . | . | . | . | . | . |
| 21-225_213E5_LC | . | . | . | H | . | . | N | . | . | . | . | . | . | . | . | . | . | . | . | . | . | . | . | . | . | . |
| 21-225_215A12_LC | . | . | . | . | . | V | . | . | . | . | . | . | . | . | . | . | . | . | . | . | . | . | . | . | . | . |
| 21-225_215B5_LC | . | . | . | . | . | I | N | . | . | . | . | . | . | . | . | . | . | . | . | . | . | . | . | . | . | . |
| 21-225_215D6_LC | . | . | . | . | . | V | . | . | . | . | . | . | . | . | . | . | . | . | . | . | . | . | . | . | . | . |
| 21-225_216G1_LC | . | . | L | H | . | D | . | . | . | . | . | . | . | . | . | . | . | . | . | . | . | . | . | . | . | . |
| 21-225_217B10_LC | . | R | . | H | T | . | K | . | . | . | . | . | . | . | . | . | . | . | . | . | . | . | . | . | . | . |
| 21-225_217G5_LC | . | . | . | . | . | V | N | . | . | . | . | . | . | . | . | . | . | . | . | . | . | . | . | . | . | . |
| 21-225_219H1_LC | . | . | . | H | . | . | N | . | . | . | . | . | . | . | . | . | . | . | . | . | . | . | . | . | . | . |
| 21-225_221F6_LC | . | . | . | . | . | M | . | . | . | . | . | . | . | . | . | . | . | . | . | . | . | . | . | . | . | . |
| 21-225_222A11_LC | . | . | L | Y | . | L | N | . | . | . | . | . | . | . | . | . | . | . | . | . | . | . | . | . | . | . |
| 21-225_222D10_LC | . | . | L | H | . | L | N | . | . | . | . | . | . | . | . | . | . | . | . | . | . | . | . | . | . | . |
| 21-225_223H10_LC | . | . | L | H | . | L | N | . | . | . | . | . | . | . | . | . | . | . | . | . | . | . | . | . | . | . |
| 21-225_22A2_LC | . | . | . | . | . | N | S | . | . | . | . | . | . | . | . | . | . | . | . | . | . | . | . | . | . | . |
| 21-225_23D11_LC | . | . | . | . | . | H | S | . | . | . | . | . | . | . | . | . | . | . | . | . | . | . | . | . | . | . |
| 21-225_32G3_LC | . | . | . | . | . | H | S | . | . | . | . | . | . | . | . | . | . | . | . | . | . | . | . | . | . | . |
| 21-225_55A11_LC | . | . | . | . | . | N | S | . | . | . | . | . | . | . | . | . | . | . | . | . | . | . | . | . | . | . |
| 21-225_55D4_LC | . | . | . | . | . | H | S | . | . | . | . | . | . | . | . | . | . | . | . | . | . | . | . | . | . | . |
| 21-225_65H11_LC | . | . | . | . | . | N | S | . | . | . | . | . | . | . | . | . | . | . | . | . | . | . | . | . | . | . |
| 21-225_70H6_LC | . | . | . | . | S | Y | S | . | . | . | . | . | . | . | . | . | . | . | . | . | . | . | . | . | . | . |
| 21-225_74C8_LC | . | . | . | . | . | . | N | . | . | . | . | . | . | . | . | . | . | . | . | . | . | . | . | . | . | . |
| 21-225_74G6_LC | . | . | . | . | . | . | N | . | . | . | . | . | . | . | . | . | . | . | . | . | . | . | . | . | . | . |
| 21-225_76C5_LC | . | . | . | . | . | . | N | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | S | T | - | - | - | - | - | - | - | - | - | - | - | - | - | - | - | - | - | - | - |
| 21-225_77E6_LC | . | . | . | . | . | . | N | . | . | . | . | . | . | . | . | . | . | . | . | . | . | . | . | . | . | . |
| 21-225_79A12_LC | . | . | . | . | . | . | N | . | . | . | . | . | . | . | . | . | . | . | . | . | . | . | . | . | . | . |
| 21-225_79F4_LC | . | . | . | . | . | . | N | . | . | . | . | . | . | . | . | . | . | . | . | . | . | . | . | . | . | . |
| 21-225_7F10_LC | . | . | . | . | . | N | S | . | . | . | . | . | . | . | . | . | . | . | . | . | . | . | . | . | . | . |
| 21-225_80C11_LC | . | . | . | . | . | . | N | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | L | T | F | G | G | G | T | K | V | E | I | K | R |
| 21-225_147E9_LC | . | . | | | . | . | . | . | | | | | . | | | . | . | . | . |
| 21-225_150G8_LC | . | . | | | . | . | . | . | | | | | . | | | . | . | . | . |
| 21-225_153F9_LC | . | . | | | . | . | . | S | | | | | . | | | . | . | . | . |
| 21-225_157C1_LC | . | . | | | S | . | S | . | | | | | . | | | . | L | . | . |
| 21-225_160H4_LC | . | . | | | . | . | . | . | | | | | . | | | . | . | . | . |
| 21-225_163G6_LC | . | . | | | . | . | . | . | | | | | . | | | A | . | . | . |
| 21-225_166H12_LC | . | . | | | S | . | . | . | | | | | . | | | . | . | T | . |
| 21-225_169C10_LC | . | . | | | . | . | . | . | | | | | . | | | . | . | . | . |
| 21-225_16A12_LC | . | . | | | . | . | . | . | | | | | . | | | . | . | . | . |
| 21-225_16D10_LC | . | . | | | . | . | . | . | | | | | . | | | . | . | . | . |
| 21-225_170D1_LC | . | . | | | S | . | . | . | | | | | . | | | . | . | . | . |
| 21-225_170D6_LC | . | . | | | . | . | . | . | | | | | . | | | . | . | . | . |
| 21-225_171H12_LC | . | . | | | . | . | . | . | | | | | . | | | . | . | . | . |
| 21-225_175G3_LC | . | . | | | . | . | . | . | | | | | . | | | . | . | . | . |
| 21-225_177A5_LC | . | . | | | . | . | . | . | | | | | . | | | . | . | . | . |
| 21-225_178H4_LC | . | . | | | . | . | . | . | | | | | . | | | . | . | . | . |
| 21-225_190C11_LC | . | . | | | . | . | . | . | | | | | . | | | . | . | . | . |
| 21-225_190E10_LC | . | . | | | . | . | . | . | | | | | . | | | . | . | . | . |
| 21-225_190E6_LC | . | . | | | . | . | . | . | | | | | . | | | . | . | . | . |
| 21-225_190F8_LC | . | . | | | . | . | . | . | | | | | . | | | . | . | . | . |
| 21-225_190G11_LC | . | . | | | . | . | . | . | | | | | . | | | . | . | . | . |
| 21-225_190G4_LC | . | . | | | . | . | . | . | | | | | . | | | . | . | . | . |
| 21-225_191A10_LC | . | . | | | . | . | . | . | | | | | . | | | . | . | . | . |
| 21-225_191E10_LC | . | . | | | . | . | . | . | | | | | . | | | . | . | . | . |
| 21-225_191G1_LC | . | . | | | . | . | . | . | | | | | . | | | . | . | . | . |
| 21-225_191G10_LC | . | . | | | . | . | . | . | | | | | . | | | . | . | . | . |
| 21-225_191G12_LC | . | . | | | . | . | . | . | | | | | S | | | . | . | . | . |
| 21-225_192C8_LC | . | . | | | . | . | . | . | | | | | . | | | . | . | . | . |
| 21-225_192D2_LC | . | . | | | . | . | N | . | | | | | . | | | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_192D3_LC | . | . | . | . | . | . | . | . | . | . | . | . | . | . | . | . | . | . | . |
| 21-225_192G6_LC | . | . | . | . | . | . | . | . | . | . | . | . | . | . | . | . | . | . | . |
| 21-225_192G8_LC | . | . | . | . | . | . | . | . | . | . | . | . | . | . | . | . | . | . | . |
| 21-225_192H10_LC | . | . | . | . | . | . | . | . | . | . | . | . | . | . | . | . | . | . | . |
| 21-225_192H4_LC | . | . | . | . | . | . | . | . | . | . | . | . | . | ≈ | . | . | . | ≈ | . |
| 21-225_193A10_LC | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . |
| 21-225_197B6_LC | . | . | . | . | . | . | . | . | . | . | . | . | . | . | . | . | . | . | . |
| 21-225_197D3_LC | . | . | . | . | . | . | . | . | . | . | . | . | . | . | . | . | . | . | . |
| 21-225_197F2_LC | . | . | . | . | . | . | . | . | . | . | . | . | . | . | . | . | . | . | . |
| 21-225_197G12_LC | . | . | . | . | . | . | . | . | . | . | . | . | . | . | . | . | . | . | . |
| 21-225_198B6_LC | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . |
| 21-225_1F1_LC | . | . | . | . | . | . | . | . | . | . | . | . | . | . | . | . | . | . | . |
| 21-225_203H1_LC | . | . | . | . | . | . | . | . | . | . | . | . | . | . | . | . | . | . | . |
| 21-225_204G6_LC | . | . | . | . | . | . | . | . | . | . | . | . | . | . | . | . | . | . | . |
| 21-225_204G8_LC | . | . | . | . | . | V | . | . | . | . | . | . | . | . | . | . | . | . | . |
| 21-225_205F5_LC | . | . | . | . | . | . | . | . | . | . | . | . | . | . | . | D | . | ≈ | . |
| 21-225_205H3_LC | . | . | . | . | . | . | . | . | . | . | . | . | . | . | . | . | . | . | . |
| 21-225_207G6_LC | . | . | . | . | . | . | . | . | . | . | . | . | . | . | . | . | . | . | . |
| 21-225_211A11_LC | . | . | . | . | . | . | . | . | . | . | A | . | . | . | . | . | . | . | . |
| 21-225_211A3_LC | . | . | . | . | . | . | . | . | . | . | . | . | . | . | . | . | . | . | . |
| 21-225_211A8_LC | . | . | . | . | . | . | i | . | . | . | . | . | . | . | . | . | . | . | . |
| 21-225_212D7_LC | . | . | . | . | . | . | . | . | . | . | . | . | . | . | . | . | . | . | . |
| 21-225_212E10_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_212E6_LC | . | . | | | | . | . | . | | . | | . | | . | | | | . | . |
| 21-225_212F10_LC | . | . | | | | . | . | . | | . | | . | | . | | . | | . | . |
| 21-225_212H11_LC | . | . | | | | . | . | . | V | . | | . | | . | | | . | T | G |
| 21-225_212H9_LC | . | . | | | | . | . | . | | . | | . | | . | | | | . | . |
| 21-225_213B8_LC | . | . | | | | . | . | . | | . | | . | | . | | . | | . | . |
| 21-225_213C4_LC | . | . | | | | . | . | . | | . | | . | | . | | . | | . | . |
| 21-225_213D2_LC | . | . | | | | . | . | . | | . | | . | | . | | . | | . | . |
| 21-225_213E5_LC | . | . | | | | . | . | . | | . | | . | | . | | . | | . | . |
| 21-225_215A12_LC | . | . | | | | . | . | . | | . | | . | | . | | . | | . | . |
| 21-225_215B5_LC | . | . | | | | . | . | . | | . | | . | | . | | . | | . | . |
| 21-225_215D6_LC | . | . | | | | . | . | . | | . | | . | | . | | . | | . | . |
| 21-225_216G1_LC | . | . | | | | . | . | . | | . | | . | | . | | . | | . | . |
| 21-225_217B10_LC | . | . | | | S | . | V | Q | L | V | | . | | . | | | . | T | G |
| 21-225_217G5_LC | . | . | | | | . | . | . | | . | | . | | . | | . | | N | . |
| 21-225_219H1_LC | . | . | | | | . | . | . | | . | | . | | . | | . | | . | . |
| 21-225_221F6_LC | . | . | | | | . | . | . | | . | | . | | R | | . | | . | . |
| 21-225_222A11_LC | . | . | | | | . | . | . | | . | | . | | . | | . | | . | . |
| 21-225_222D10_LC | . | . | | | | . | . | . | | . | | . | | R | | . | | . | . |
| 21-225_223H10_LC | . | . | | | | . | . | . | | . | | . | | . | | . | | . | . |
| 21-225_22A2_LC | . | . | | | F | . | . | . | | . | | . | | . | | . | | . | . |
| 21-225_23D11_LC | . | . | | | | . | . | . | | . | | . | S | . | | . | | . | . |
| 21-225_32G3_LC | . | . | | | | . | . | . | | . | | . | | . | | . | | . | . |
| 21-225_55A11_LC | . | . | | | | . | . | . | | . | | . | | . | | . | | . | . |
| 21-225_55D4_LC | . | . | | | F | . | . | . | | . | | . | | R | | . | | R | . |
| 21-225_65H11_LC | . | . | | | F | . | . | . | | . | | . | | . | | . | | . | . |
| 21-225_70H6_LC | . | . | | | I | . | . | . | | . | | . | | . | | . | | . | . |
| 21-225_74C8_LC | . | . | | | | . | . | . | | . | | . | | . | | . | | N | . |
| 21-225_74G6_LC | . | . | | | | . | . | . | | . | | . | | . | | . | | N | . |
| 21-225_76C5_LC | . | . | | | | . | . | . | | . | | . | | . | | . | | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | L | T | F | G | G | G | T | K | V | E | I | K | R |
| 21-225_77E6_LC | . | . | . | . | . | . | . | . | . | . | . | . | . | . | . | . | . | . | . |
| 21-225_79A12_LC | . | . | . | . | . | . | . | . | . | . | . | . | . | . | . | . | . | . | . |
| 21-225_79F4_LC | . | . | . | . | . | . | . | . | . | . | . | . | . | . | . | . | . | . | . |
| 21-225_7F10_LC | . | . | . | . | . | . | . | . | . | . | . | . | . | . | . | . | . | . | . |
| 21-225_80C11_LC | . | . | . | . | . | . | . | . | . | . | . | . | . | . | . | . | . | N | . |

**FIGURE 57**

Table 102. Consensus 22 - VK1|O12/JK4 (SEQ ID NO: 50319):

DIQMTQSPSSLSASVGDRVTITCRAS--QNII------SYLNWYQQKPGKAPKLLIYT--------ASSLQSGVPSRFSGSGSG--TDFTLTISSLQPEDFATYYCQQSYS----------------------TPLTFGGGTKVEIKR wherein:

A at position 25 can be substituted with T

S at position 26 can be substituted with T

Q at position 29 can be substituted with H or R

N at position 30 can be substituted with S, R, T or I

I at position 31 can be substituted with V or F

I at position 32 can be substituted with S, N, Y, F, R, H, L, K or T

S at position 39 can be substituted with N, D, R, K or T

Y at position 40 can be substituted with F

N at position 42 an be substituted with H

T at position 58 can be substituted with A, V, G, I or S

A at position 67 can be substituted with T

S at position 69 can be substituted with N, R or T

L at position 70 can be substituted with F or S

Q at position 71 can be substituted with H, E or P

S at position 72 can be substituted with G, T, N or R

Q at position 108 can be substituted with L

S at position 109 can be substituted with T, N or P

FIGURE 57

Y at position 110 can be substituted with H, C, D, F or N

S at position 111 can be substituted with null (-), I, N, F, G or T

T at position 135 can be substituted with S, P, F, N, I or L

P at position 136 can be substituted with T, I, A or S

L at position 137 can be substituted with P, Y, F or V

Arg Xaa Xaa Xaa Xaa Xaa Xaa Xaa Leu Xaa (SEQ ID NO: 50141)

Xaa Xaa Ser Xaa Xaa Xaa Xaa (SEQ ID NO: 50142)

Gln Xaa Xaa Xaa Xaa Xaa Xaa Xaa Thr (SEQ ID NO: 50143)

Arg Ala Ser Gln Asn Ile Ile Ser Tyr Leu Asn (SEQ ID NO: 50627)

Thr Ala Ser Ser Leu Gln Ser (SEQ ID NO: 50628)

Gln Gln Ser Tyr Ser Thr Pro Leu Thr (SEQ ID NO: 50629)

FIGURE 57

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_204D6_LC | | S | | | | | | | | | | | | | | | | | | | | | | | | |
| 21-225_204H4_LC | | | | L | | | | | | | S | | S | | | | | | | | | | R | | | |
| 21-225_206A4_LC | | | | L | | | | | | | P | | S | | | | | | | | | | S | | | |
| 21-225_206A5_LC | | | | | | | | | | | P | | | | | | | | | | | | S | | | |
| 21-225_207C9_LC | | N | | | | | | | | | | | | | | | | | | | | | | | | |
| 21-225_208B11_LC | | | | | | | | | | | P | | | | | | | | | | | | | | | |
| 21-225_208E8_LC | | | | | | | | | | | P | | S | | | | | | | | | | S | | | |
| 21-225_20F6_LC | | | | | | | | | | | | | | | | | | | | | | | | | | |
| 21-225_210D11_LC | | | | | | | | | | | | | | | | | | | | | | | | | | |
| 21-225_22C7_LC | | | | | | | | | | | | | | | | | | | | | | | | | | |
| 21-225_30G1_LC | | | | | | | | | | | | | | | | | | | | | | | | | | |
| 21-225_30G4_LC | | | | | | | P | | | | | | Y | | | | | | | | | | | | | Y |
| 21-225_31F1_LC | | | | | | | | | | | | | | | | | | | | | | | | | | |
| 21-225_32D6_LC | | | | | | | | | | | | | Y | | | | | | | | | | | | | |
| 21-225_33B2_LC | | | | | | | | | | | | | T | | | | | S | | | | | | | | |
| 21-225_33C12_LC | | | | | | | | | | | | | Y | | | | | | | | | | | | | |
| 21-225_34C11_LC | | | | | | | S | | | | | | Y | | | | | | | | S | | | | | |
| 21-225_34C2_LC | | | | | | | | | | | | | T | S | | | | | | | | | | | | |
| 21-225_34D3_LC | | | | | | | | | | | | | | | | | | | | | | | | | | |
| 21-225_34D8_LC | | | | | | | | | | | | | Y | | | | | | | | | | | | | |
| 21-225_34G11_LC | | | | | | | | | | | | | | | | | | | | | | | | | | |
| 21-225_36B8_LC | | | | | | | | | | | | | T | | | | | | | | | | | | | |
| 21-225_3F8_LC | S | | | | | | | | | | | | | | | | | | | | | | | | | |
| 21-225_3G7_LC | | | | | | | | | | | | | | | | | | | | | V | | | | | |
| 21-225_48B10_LC | | | | | | | | | | | | | | | | | | | | | | | | | | |
| 21-225_48F12_LC | | | | | | | | | | | | | | | | | | | | | | | | | | |
| 21-225_48G12_LC | | | | | | | | | | | | | | | | | | | | | | | | | | |
| 21-225_4G4_LC | | | | | | | | | | | S | | | | | | | | | | | | | | | |
| 21-225_50F2_LC | | | | | | | | | | | | | | | | | | | | T | | | | | | |

4754

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_56B6_LC | . | . | . | . | . | . | . | . | . | . | . | V | . | . | . | . | . | . | . | . | . | . | . | . | . | . |
| 21-225_62D2_LC | . | . | . | . | . | . | . | . | . | . | . | . | . | . | . | . | . | . | . | . | . | : | . | . | . | . |
| 21-225_63C10_LC | . | . | . | . | . | . | . | . | . | . | . | . | T | . | . | . | . | . | . | . | . | . | . | . | . | . |
| 21-225_64A11_LC | . | . | . | . | . | . | . | . | S | . | . | . | . | . | . | . | . | . | . | . | . | . | . | . | . | . |
| 21-225_64F7_LC | . | . | . | . | . | . | . | . | . | . | . | . | . | . | . | . | . | . | . | . | . | . | . | . | . | . |
| 21-225_6G1_LC | . | . | . | . | . | . | . | . | . | . | . | . | . | . | . | . | . | . | . | . | . | . | . | . | T | . |
| 21-225_7A10_LC | . | . | . | . | . | . | . | . | . | . | . | . | . | . | . | . | . | . | . | . | . | . | . | . | . | . |
| 21-225_7E11_LC | . | . | . | . | . | . | . | . | . | . | . | . | . | . | . | . | . | . | . | . | . | A | . | . | . | . |
| 21-225_7E11.001_LC | . | . | . | . | . | . | . | . | . | . | . | . | . | . | . | . | . | . | . | . | . | A | . | . | . | . |
| 21-225_7E11.001.001_LC | . | . | . | . | . | . | . | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . | . | . | A | . | . | . | . |
| 21-225_7E11.001.004_LC | . | . | . | . | . | . | . | . | . | . | . | . | . | . | . | . | . | . | . | . | . | A | . | . | . | . |
| 21-225_7E11.001.005_LC | . | . | . | . | . | . | . | . | . | . | . | . | . | . | . | . | . | . | . | . | . | A | . | . | . | . |
| 21-225_7E11.001.006_LC | . | . | . | . | . | . | . | . | . | . | . | . | . | . | . | . | . | . | . | . | . | A | . | . | . | . |
| 21-225_7E11.001.007_LC | . | . | . | . | . | . | . | . | . | . | . | . | . | . | . | . | . | . | . | . | . | A | . | . | . | . |
| 21-225_7E11.001.008_LC | . | . | . | . | . | . | . | . | . | . | . | . | . | . | . | . | . | . | . | . | . | A | . | . | . | . |
| 21-225_7E11.001.009_LC | . | . | . | . | . | . | . | . | . | . | . | . | . | . | . | . | . | . | . | . | . | A | . | . | . | . |
| 21-225_7E11.001.010_LC | . | . | . | . | . | . | . | . | . | . | . | . | . | . | . | . | . | . | . | . | . | A | . | . | . | . |
| 21-225_7E11.001.011_LC | . | . | . | . | . | . | . | . | . | . | . | . | . | . | . | . | . | . | . | . | . | A | . | . | . | . |
| 21-225_7E11.001.012_LC | . | . | . | . | . | . | . | . | . | . | . | . | . | . | . | . | . | . | . | . | . | . | . | . | . | . |
| 21-225_7E11.001.013_LC | . | . | . | . | . | . | . | . | . | . | . | . | . | . | . | . | . | . | . | . | . | . | . | . | . | . |
| 21-225_7E11.001.014_LC | . | . | . | . | . | . | . | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . | . | . | . | . | . | . | . |
| 21-225_7E11.001.019_LC | . | . | . | . | . | . | . | . | . | . | . | . | . | . | . | . | . | . | . | . | . | A | . | . | . | . |
| 21-225_7E11.001.021_LC | . | . | . | . | . | . | . | . | . | . | . | . | . | . | . | . | . | . | . | . | . | A | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_7E11.001.022_LC | . | . | . | . | . | . | . | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . | . | . | A | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | N | I | I | - | - | - | - | - | - | S | Y | L | N | W | Y | Q | Q | K | P | G | K | A | P |
| 21-225_10E12_LC | . | . | . | . | . | Y | . | . | . | . | . | . | . | . | . | . | . | . | . | S | . | . | . | R | . | . |
| 21-225_10F5_LC | . | . | . | . | . | Y | . | . | . | . | . | . | D | . | . | . | . | . | . | S | . | . | . | R | . | . |
| 21-225_12A10_LC | . | . | . | . | . | Y | . | . | . | . | . | . | . | . | . | . | . | . | . | . | . | . | . | R | . | . |
| 21-225_13D4_LC | . | . | . | S | . | F | . | . | . | . | . | . | . | . | . | . | . | . | . | . | . | . | . | . | . | . |
| 21-225_146E9_LC | . | . | . | S | . | S | . | . | . | . | . | . | G | . | . | . | . | . | . | L | . | . | . | . | . | . |
| 21-225_146H1_LC | . | . | . | S | . | S | . | . | . | . | . | . | D | . | . | . | . | . | . | . | . | . | . | . | . | . |
| 21-225_148C4_LC | . | . | . | S | . | . | . | . | . | . | . | . | N | . | . | . | . | . | . | . | . | . | . | . | . | . |
| 21-225_148H9_LC | . | . | . | S | . | S | . | . | . | . | . | . | N | . | . | . | . | . | . | . | . | . | . | . | . | . |
| 21-225_149A3_LC | . | . | . | S | . | S | . | . | . | . | . | . | . | . | . | . | . | . | . | . | . | . | . | . | . | . |
| 21-225_150C11_LC | . | . | . | S | . | S | . | . | . | . | . | . | . | . | . | . | . | . | . | . | . | . | . | . | . | . |
| 21-225_150C12_LC | . | . | . | S | . | S | . | . | . | . | . | . | D | . | . | . | . | . | . | . | . | . | . | . | . | . |
| 21-225_151B12_LC | . | . | . | S | . | S | . | . | . | . | . | . | N | F | . | . | . | . | . | . | . | . | . | . | . | . |
| 21-225_152B11_LC | . | . | . | S | . | S | . | . | . | . | . | . | D | . | . | . | . | . | . | . | . | . | . | . | . | . |
| 21-225_152D2_LC | . | . | . | S | . | S | . | . | . | . | . | . | D | . | . | . | . | . | . | L | . | . | . | . | . | . |
| 21-225_152G4_LC | . | . | . | S | . | S | . | . | . | . | . | . | D | . | . | . | . | . | . | . | . | . | . | . | . | . |
| 21-225_155C4_LC | . | . | . | S | . | S | . | . | . | . | . | . | . | . | . | . | . | . | . | . | . | . | . | . | . | . |
| 21-225_156E4_LC | . | . | . | S | . | S | . | . | . | . | . | . | . | . | . | . | . | . | . | . | . | . | . | . | . | . |
| 21-225_159H2_LC | . | . | . | S | . | S | . | . | . | . | . | . | N | . | . | . | . | . | . | . | . | . | . | . | . | . |
| 21-225_15A3_LC | . | . | . | S | . | . | . | . | . | . | . | . | . | . | . | . | . | . | L | . | . | . | . | . | . | . |
| 21-225_16D11_LC | . | . | . | T | . | S | . | . | . | . | . | . | N | . | . | . | . | . | . | X | . | . | . | . | . | . |
| 21-225_16E6_LC | . | . | . | S | . | . | . | . | . | . | . | . | . | . | . | . | . | . | . | . | . | . | . | . | . | . |
| 21-225_17A6_LC | . | . | . | T | . | S | . | . | . | . | . | . | N | . | . | . | . | . | D | . | . | . | . | . | . | . |
| 21-225_17G4_LC | . | . | . | . | . | S | . | . | . | . | . | . | N | . | . | . | . | . | . | . | . | . | . | . | . | . |
| 21-225_18G4_LC | . | . | . | S | . | . | . | . | . | . | . | . | . | . | . | . | . | . | . | . | . | . | . | . | . | . |
| 21-225_196F4_LC | . | . | . | X | . | H | . | . | . | . | . | . | . | . | . | . | . | . | . | . | . | . | . | . | . | . |
| 21-225_19A1_LC | . | . | H | T | . | T | . | . | . | . | . | . | . | . | . | . | . | . | . | . | . | . | . | . | . | . |
| 21-225_1H12_LC | . | . | . | . | . | Y | . | . | . | . | . | . | D | . | . | . | . | . | . | S | . | . | . | D | . | . |
| 21-225_201H10_LC | . | . | N | . | . | N | . | . | . | . | . | . | . | . | . | . | . | . | . | . | . | S | . | . | . | . |
| 21-225_203E3_LC | . | . | H | . | . | N | . | . | . | . | . | . | . | . | . | . | . | . | . | . | . | S | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | N | I | I | - | - | - | - | - | - | S | Y | L | N | W | Y | Q | Q | K | P | G | K | A | P |
| 21-225_204D6_LC | . | . | R | S | V | N | . | . | . | . | . | . | T | . | . | . | . | . | . | . | . | . | . | . | . | . |
| 21-225_204H4_LC | . | . | N | . | . | N | . | . | . | . | . | . | . | . | . | . | . | . | . | . | . | S | . | . | . | . |
| 21-225_206A4_LC | . | . | H | . | . | N | . | . | . | . | . | . | . | . | . | . | . | . | . | . | . | S | . | . | . | . |
| 21-225_206A5_LC | . | . | H | . | . | N | . | . | . | . | . | . | . | . | . | . | . | . | . | . | . | S | . | . | . | . |
| 21-225_207C9_LC | . | . | R | P | S | . | . | . | . | . | . | . | N | . | . | . | . | . | . | . | . | . | . | . | . | . |
| 21-225_208B11_LC | . | . | H | . | . | N | . | . | . | . | . | . | . | . | . | . | . | . | . | . | . | S | . | . | . | . |
| 21-225_208E8_LC | . | . | H | . | . | N | . | . | . | . | . | . | . | . | . | . | . | . | . | . | . | S | . | . | . | . |
| 21-225_20F6_LC | . | . | S | . | . | . | . | . | . | . | . | . | T | . | . | . | . | . | . | . | . | . | . | . | . | . |
| 21-225_210D11_LC | . | . | M | . | . | N | . | . | . | . | . | . | . | . | . | . | . | . | . | . | . | S | . | . | . | . |
| 21-225_22C7_LC | . | . | . | . | . | . | . | . | . | . | . | . | . | . | . | . | . | . | . | . | . | . | E | . | . | . |
| 21-225_30G1_LC | . | . | . | . | . | Y | . | . | . | . | . | . | . | . | . | . | . | . | . | . | . | . | . | . | D | . |
| 21-225_30G4_LC | . | . | . | . | . | P | . | . | . | . | . | . | . | . | . | . | . | M | . | . | . | . | . | . | . | . |
| 21-225_31F1_LC | . | . | R | . | . | S | . | . | . | . | . | . | . | . | . | . | . | . | . | . | . | . | . | . | . | . |
| 21-225_32D6_LC | . | . | . | . | . | . | . | . | . | . | . | . | . | . | . | . | . | . | . | . | . | . | . | . | . | . |
| 21-225_33B2_LC | . | . | S | . | . | . | . | . | . | . | . | . | . | . | . | . | . | . | . | . | . | . | . | . | . | . |
| 21-225_33C12_LC | . | . | S | . | . | . | . | . | . | . | . | . | . | . | . | . | . | . | . | . | . | . | . | . | . | . |
| 21-225_34C11_LC | . | . | . | . | . | R | . | . | . | . | . | . | N | P | . | . | . | . | . | . | . | . | E | . | D | . |
| 21-225_34C2_LC | . | . | R | . | . | . | . | . | . | . | . | . | . | . | . | . | . | P | . | . | . | . | . | . | . | . |
| 21-225_34D3_LC | . | . | . | . | . | Y | . | . | . | . | . | . | . | . | . | . | . | . | . | . | . | . | . | . | D | . |
| 21-225_34D8_LC | . | . | I | . | . | . | . | . | . | . | . | . | . | . | . | . | . | . | . | . | . | . | . | . | . | . |
| 21-225_34G11_LC | . | . | . | . | . | N | . | . | . | . | . | . | R | . | . | . | . | . | . | . | . | . | . | . | . | . |
| 21-225_36B8_LC | . | . | S | . | . | . | . | . | . | . | . | . | . | . | . | . | . | . | . | . | . | . | . | . | . | . |
| 21-225_3F8_LC | . | . | S | . | L | . | . | . | . | . | . | . | . | . | . | . | . | . | R | . | . | . | . | R | . | . |
| 21-225_3G7_LC | . | . | . | . | Y | . | . | . | . | . | . | . | R | . | . | . | . | . | . | Q | R | . | . | R | . | . |
| 21-225_48B10_LC | . | . | S | . | R | . | . | . | . | . | . | . | N | . | . | . | . | . | . | K | T | . | . | . | . | . |
| 21-225_48F12_LC | . | . | S | . | R | . | . | . | . | . | . | . | K | . | . | . | . | . | . | K | T | . | . | . | . | . |
| 21-225_48G12_LC | . | . | S | . | R | . | . | . | . | . | . | . | K | . | . | . | . | . | . | R | T | . | . | . | . | . |
| 21-225_4G4_LC | . | . | T | . | . | . | . | . | . | . | . | . | . | . | . | . | . | . | . | . | . | . | . | . | . | . |
| 21-225_50F2_LC | . | . | S | . | L | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | N | I | I | - | - | - | - | - | - | S | Y | L | N | W | Y | Q | Q | K | P | G | K | A | P |
| 21-225_56B6_LC | . | . | . | S | . | F | . | . | . | . | . | . | X | . | . | . | . | . | . | . | . | . | . | X | . | . |
| 21-225_62D2_LC | . | . | . | S | . | . | . | . | . | . | . | . | . | . | . | . | . | . | . | . | . | . | . | . | . | . |
| 21-225_63C10_LC | . | . | . | S | . | F | . | . | . | . | . | . | . | . | . | X | . | . | . | . | . | . | . | . | . | . |
| 21-225_64A11_LC | . | . | X | S | V | S | . | . | . | . | . | . | X | . | . | . | . | . | . | . | T | L | . | . | . | L |
| 21-225_64F7_LC | . | . | . | . | . | X | . | . | . | . | . | . | X | . | . | . | . | . | . | . | . | . | . | . | . | . |
| 21-225_6G1_LC | . | . | . | . | . | . | . | . | . | . | . | . | . | . | . | . | . | . | . | . | . | . | . | . | . | . |
| 21-225_7A10_LC | . | . | . | . | . | Y | . | . | . | . | . | . | . | . | . | . | . | . | . | . | . | . | . | X | . | . |
| 21-225_7E11_LC | . | . | . | . | . | . | . | . | . | . | . | . | . | . | . | . | . | . | . | . | . | . | . | . | . | . |
| 21-225_7E11.001_LC | . | . | . | . | . | . | . | . | . | . | . | . | . | . | . | . | . | . | . | . | . | . | . | . | . | . |
| 21-225_7E11.001.001_LC | . | . | . | . | . | . | . | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . | . | . | . | . | . | . | . |
| 21-225_7E11.001.004_LC | . | . | . | . | . | . | . | . | . | . | . | . | . | . | . | . | . | . | . | . | . | . | . | . | . | . |
| 21-225_7E11.001.005_LC | . | . | . | . | . | . | . | . | . | . | . | . | . | . | . | . | . | . | . | . | . | . | . | . | . | . |
| 21-225_7E11.001.006_LC | . | . | . | . | . | . | . | . | . | . | . | . | . | . | . | . | . | . | . | . | . | . | . | . | . | . |
| 21-225_7E11.001.007_LC | . | . | . | . | . | . | . | . | . | . | . | . | . | . | . | . | . | . | . | . | . | . | . | . | . | . |
| 21-225_7E11.001.008_LC | . | . | . | . | . | . | . | . | . | . | . | . | . | . | . | . | . | . | . | . | . | . | . | . | . | . |
| 21-225_7E11.001.009_LC | . | . | . | . | . | . | . | . | . | . | . | . | . | . | . | . | . | . | . | . | . | . | . | . | . | . |
| 21-225_7E11.001.010_LC | . | . | . | . | . | . | . | . | . | . | . | . | . | . | . | . | . | . | . | . | . | . | . | . | . | . |
| 21-225_7E11.001.011_LC | . | . | . | . | . | . | . | . | . | . | . | . | . | . | . | . | . | . | . | . | . | . | . | . | . | . |
| 21-225_7E11.001.012_LC | . | . | . | . | . | . | . | . | . | . | . | . | . | . | . | . | . | . | . | . | . | . | . | . | . | . |
| 21-225_7E11.001.013_LC | . | . | . | . | . | . | . | . | . | . | . | . | . | . | . | . | . | . | . | . | . | . | . | . | . | . |
| 21-225_7E11.001.014_LC | . | . | . | . | . | . | . | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . | . | . | . | . | . | . | . |
| 21-225_7E11.001.019_LC | . | . | . | . | . | . | . | . | . | . | . | . | . | . | . | . | . | . | . | . | . | . | . | . | . | . |
| 21-225_7E11.001.021_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | K_CDR1 | | | | | | | | | | | | | | | | | | K_FR2 | | | |
| CONSENSUS | - | - | Q | N | I | I | - | - | - | - | - | - | S | Y | L | N | W | Y | Q | Q | K | P | G | K | A | P |
| 21-225_7E11.001.022_LC | . | . | . | . | . | . | . | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | K_CDR2 | | | | | | | | | | | | | | | | |
| CONSENSUS | K | L | L | I | Y | T | - | - | - | - | - | - | - | - | A | S | S | L | Q | S | G | V | P | S | R | F |
| 21-225_10E12_LC | . | . | . | . | . | A | . | . | . | . | . | . | . | . | T | . | . | . | . | . | . | . | . | . | . | . |
| 21-225_10F5_LC | . | . | . | . | . | . | . | . | . | . | . | . | . | . | . | . | . | . | . | . | . | . | . | . | . | . |
| 21-225_12A10_LC | . | V | . | . | M | . | . | . | . | . | . | . | . | . | . | . | . | . | . | . | . | . | . | . | . | . |
| 21-225_13D4_LC | . | . | . | . | C | . | . | . | . | . | . | . | . | . | . | . | . | . | . | N | . | . | . | . | . | . |
| 21-225_146E9_LC | . | V | . | . | . | . | . | . | . | . | . | . | . | . | T | . | . | . | . | . | . | . | . | . | . | . |
| 21-225_146H1_LC | . | V | . | . | . | . | . | . | . | . | . | . | . | . | T | . | . | . | . | . | . | . | . | . | . | . |
| 21-225_148C4_LC | . | V | . | . | . | . | . | . | . | . | . | . | . | . | . | . | . | . | . | . | . | . | . | . | . | . |
| 21-225_148H9_LC | . | V | . | . | . | I | . | . | . | . | . | . | . | . | . | . | . | . | . | . | . | . | . | . | . | . |
| 21-225_149A3_LC | . | V | M | . | . | . | . | . | . | . | . | . | . | . | . | . | . | . | . | . | . | . | . | . | . | . |
| 21-225_150C11_LC | . | . | . | . | . | . | . | . | . | . | . | . | . | . | . | . | . | . | . | . | . | . | . | . | . | . |
| 21-225_150C12_LC | . | V | . | . | . | . | . | . | . | . | . | . | . | . | T | . | . | . | . | . | . | . | . | . | . | . |
| 21-225_151B12_LC | . | V | . | . | . | . | . | . | . | . | . | . | . | . | . | . | . | . | . | E | . | . | . | . | . | . |
| 21-225_152B11_LC | . | V | . | . | . | . | . | . | . | . | . | . | . | . | T | . | . | . | . | . | . | . | . | . | . | . |
| 21-225_152D2_LC | . | V | . | . | . | . | . | . | . | . | . | . | . | . | T | . | . | . | . | . | . | . | . | . | . | . |
| 21-225_152G4_LC | . | V | . | . | . | . | . | . | . | . | . | . | . | . | T | . | . | . | . | . | . | . | . | . | . | . |
| 21-225_155C4_LC | . | V | . | . | F | . | . | . | . | . | . | . | . | . | . | . | . | . | . | . | . | . | . | . | . | . |
| 21-225_156E4_LC | . | V | . | . | . | . | . | . | . | . | . | . | . | . | . | . | . | . | . | . | . | . | . | . | . | . |
| 21-225_159H2_LC | E | . | . | . | . | A | . | . | . | . | . | . | . | . | T | . | N | . | . | . | . | . | . | . | . | . |
| 21-225_15A3_LC | . | F | . | . | . | . | . | . | . | . | . | . | . | . | . | . | . | . | . | . | . | . | . | . | . | . |
| 21-225_16D11_LC | . | . | . | . | . | A | . | . | . | . | . | . | . | . | . | . | . | . | . | . | . | . | . | . | . | . |
| 21-225_16E6_LC | . | F | . | . | . | . | . | . | . | . | . | . | . | . | T | . | . | . | . | . | . | . | . | . | . | . |
| 21-225_17A6_LC | . | . | . | . | . | A | . | . | . | . | . | . | . | . | . | . | . | . | . | . | . | . | . | . | . | . |
| 21-225_17G4_LC | . | . | . | . | . | V | . | . | . | . | . | . | . | . | . | . | . | F | P | . | . | . | . | . | . | . |
| 21-225_18G4_LC | . | V | . | . | . | . | . | . | . | . | . | . | . | . | . | . | . | . | . | T | . | . | . | . | G | . |
| 21-225_196F4_LC | . | . | . | . | . | . | . | . | . | . | . | . | . | . | . | . | . | . | . | Q | . | . | . | . | . | . |
| 21-225_19A1_LC | . | F | . | . | . | A | . | . | . | . | . | . | . | . | T | . | N | . | . | . | . | . | . | . | . | . |
| 21-225_1H12_LC | . | . | . | . | . | . | . | . | . | . | . | . | . | . | . | . | . | . | . | . | . | . | . | . | . | . |
| 21-225_201H10_LC | . | . | . | . | . | A | . | . | . | . | . | . | . | . | . | . | . | . | . | . | . | . | . | . | . | . |
| 21-225_203E3_LC | . | . | . | . | . | A | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
|  |  |  |  |  |  |  |  |  |  | K_CDR2 |  |  |  |  |  |  |  |  |  |  |  |  |  |  |  |  |
| CONSENSUS | K | L | L | I | Y | T | - | - | - | - | - | - | - | - | A | S | S | L | Q | S | G | V | P | S | R | F |
| 21-225_204D6_LC | . | V | . | . | . | G | . | . | . | . | . | . | . | . | . | . | . | . | . | . | . | . | . | . | . | . |
| 21-225_204H4_LC | . | . | . | . | . | A | . | . | . | . | . | . | . | . | . | . | . | . | . | . | . | . | . | . | . | . |
| 21-225_206A4_LC | . | . | . | Q | . | A | . | . | . | . | . | . | . | . | . | . | . | . | . | . | . | . | . | . | . | . |
| 21-225_206A5_LC | . | . | . | . | Q | A | . | . | . | . | . | . | . | . | . | . | . | . | . | . | . | . | . | . | . | . |
| 21-225_207C9_LC | . | . | . | . | . | . | . | . | . | . | . | . | . | . | . | . | . | . | . | . | . | . | . | . | . | . |
| 21-225_208B11_LC | . | . | . | . | . | A | . | . | . | . | . | . | . | . | . | . | . | . | . | . | . | . | . | . | . | . |
| 21-225_208E8_LC | . | . | . | . | . | A | . | . | . | . | . | . | . | . | . | . | . | . | . | . | . | . | . | . | . | . |
| 21-225_20F6_LC | . | V | . | . | . | . | . | . | . | . | . | . | . | . | . | . | . | . | . | . | . | . | . | . | . | . |
| 21-225_210D11_LC | . | . | . | . | . | A | . | . | . | . | . | . | . | . | . | . | . | . | . | . | . | . | . | . | . | . |
| 21-225_22C7_LC | . | . | . | . | . | I | . | . | . | . | . | . | . | . | . | . | . | . | . | . | . | . | . | . | . | . |
| 21-225_30G1_LC | . | . | . | . | . | A | . | . | . | . | . | . | . | . | . | . | . | . | . | . | . | . | . | . | . | . |
| 21-225_30G4_LC | . | . | . | . | . | . | . | . | . | . | . | . | . | . | . | . | . | . | . | . | . | G | . | . | . | . |
| 21-225_31F1_LC | . | . | . | . | P | A | . | . | . | . | . | . | . | . | . | . | . | S | . | . | . | . | . | . | . | . |
| 21-225_32D6_LC | . | . | M | . | . | V | . | . | . | . | . | . | . | . | . | . | . | . | . | . | . | . | . | . | . | . |
| 21-225_33B2_LC | . | . | . | . | . | V | . | . | . | . | . | . | . | . | . | . | . | . | . | . | . | . | . | . | . | . |
| 21-225_33C12_LC | . | . | . | . | . | V | . | . | . | . | . | . | . | . | . | . | . | . | . | G | . | . | . | . | . | . |
| 21-225_34C11_LC | . | . | Q | . | . | G | . | . | . | . | . | . | . | . | . | Y | . | . | . | . | . | . | . | . | . | . |
| 21-225_34C2_LC | . | . | . | . | . | V | . | . | . | . | . | . | . | . | . | . | . | . | . | . | . | . | . | . | . | . |
| 21-225_34D3_LC | . | . | . | . | . | A | . | . | . | . | . | . | . | . | . | . | . | . | . | . | . | . | . | . | . | . |
| 21-225_34D8_LC | . | . | . | . | P | V | . | . | . | . | . | . | . | . | . | . | . | . | . | . | . | . | . | . | . | . |
| 21-225_34G11_LC | . | . | . | . | . | G | . | . | . | . | . | . | . | . | . | . | . | . | . | . | . | . | . | . | . | . |
| 21-225_36B8_LC | . | . | . | . | . | V | . | . | . | . | . | . | . | . | . | . | . | . | . | . | . | . | . | . | . | . |
| 21-225_3F8_LC | Q | V | . | . | . | . | . | . | . | . | . | . | . | . | Y | . | . | . | . | . | . | . | . | . | . | . |
| 21-225_3G7_LC | . | . | M | . | . | A | . | . | . | . | . | . | . | . | . | . | . | S | . | . | . | . | . | . | . | . |
| 21-225_48B10_LC | . | . | . | . | . | A | . | . | . | . | . | . | . | . | . | . | . | . | . | . | . | . | . | . | . | . |
| 21-225_48F12_LC | . | . | . | . | . | A | . | . | . | . | . | . | . | . | . | . | . | . | . | . | . | . | . | . | . | . |
| 21-225_48G12_LC | . | . | . | . | . | A | . | . | . | . | . | . | . | . | . | . | . | . | . | . | . | . | . | . | . | . |
| 21-225_4G4_LC | . | . | . | . | . | . | . | . | . | . | . | . | . | . | . | . | . | . | . | . | . | . | . | . | . | . |
| 21-225_50F2_LC | . | . | . | . | . | V | . | . | . | . | . | . | . | . | . | . | . | . | P | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | T | - | - | - | - | - | - | - | - | A | S | S | L | Q | S | G | V | P | S | R | F |
| 21-225_56B6_LC | . | . | . | . | S | A | . | . | . | . | . | . | . | . | . | . | . | S | . | . | . | . | . | . | . | . |
| 21-225_62D2_LC | . | . | . | . | . | A | . | . | . | . | . | . | . | . | T | . | . | . | . | . | . | . | . | . | . | . |
| 21-225_63C10_LC | . | . | . | . | S | A | . | . | . | . | . | . | . | . | . | . | . | . | . | . | . | . | . | . | . | . |
| 21-225_64A11_LC | . | . | . | . | S | V | . | . | . | . | . | . | . | . | . | . | R | . | . | . | . | . | . | . | . | . |
| 21-225_64F7_LC | . | F | . | . | . | G | . | . | . | . | . | . | . | . | . | . | . | . | . | . | . | . | . | . | . | . |
| 21-225_6G1_LC | . | V | . | . | . | . | . | . | . | . | . | . | . | . | . | . | N | . | . | T | . | . | . | . | G | . |
| 21-225_7A10_LC | . | . | V | . | . | S | . | . | . | . | . | . | . | . | T | . | . | . | . | . | . | . | . | . | . | . |
| 21-225_7E11_LC | . | F | . | . | . | . | . | . | . | . | . | . | . | . | . | . | . | . | . | . | . | . | . | . | . | . |
| 21-225_7E11.001_LC | . | F | . | . | . | . | . | . | . | . | . | . | . | . | . | . | . | . | . | . | . | . | . | . | . | . |
| 21-225_7E11.001.001_LC | . | F | . | . | . | . | . | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | F | . | . | . | . | . | . | . | . | . | . | . | . | . | . | . | . | . | . | . | . | . | . | . | . |
| 21-225_7E11.001.004_LC | . | F | . | . | . | . | . | . | . | . | . | . | . | . | . | . | . | . | . | . | . | . | . | . | . | . |
| 21-225_7E11.001.005_LC | . | F | . | . | . | . | . | . | . | . | . | . | . | . | . | . | . | . | . | . | . | . | . | . | . | . |
| 21-225_7E11.001.006_LC | . | F | . | . | . | . | . | . | . | . | . | . | . | . | . | . | . | . | . | . | . | . | . | . | . | . |
| 21-225_7E11.001.007_LC | . | F | . | . | . | . | . | . | . | . | . | . | . | . | . | . | . | . | . | . | . | . | . | . | . | . |
| 21-225_7E11.001.008_LC | . | F | . | . | . | . | . | . | . | . | . | . | . | . | . | . | . | . | . | . | . | . | . | . | . | . |
| 21-225_7E11.001.009_LC | . | F | . | . | . | . | . | . | . | . | . | . | . | . | . | . | . | . | . | . | . | . | . | . | . | . |
| 21-225_7E11.001.010_LC | . | F | . | . | . | . | . | . | . | . | . | . | . | . | . | . | . | . | . | . | . | . | . | . | . | . |
| 21-225_7E11.001.011_LC | . | F | . | . | . | . | . | . | . | . | . | . | . | . | . | . | . | . | . | . | . | . | . | . | . | . |
| 21-225_7E11.001.012_LC | . | F | . | . | . | . | . | . | . | . | . | . | . | . | . | . | . | . | . | . | . | . | . | . | . | . |
| 21-225_7E11.001.013_LC | . | F | . | . | . | . | . | . | . | . | . | . | . | . | . | . | . | . | . | . | . | . | . | . | . | . |
| 21-225_7E11.001.014_LC | . | F | . | . | . | . | . | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | F | . | . | . | . | . | . | . | . | . | . | . | . | . | . | . | . | . | . | . | . | . | . | . | . |
| 21-225_7E11.001.019_LC | . | F | . | . | . | . | . | . | . | . | . | . | . | . | . | . | . | . | . | . | . | . | . | . | . | . |
| 21-225_7E11.001.021_LC | . | F | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | T | - | - | - | - | - | - | - | - | A | S | S | L | Q | S | G | V | P | S | R | F |
| 21-225_7E11.001.022_LC | . | S | . | . | . | . | . | . | . | . | . | . | . | . | . | . | . | . | . | . | . | . | . | . | . | . |
| 21-225_7E11.001.023_LC | | S | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_10E12_LC | G | . | . | . | . | . | . | . | . | . | . | . | . | . | . | . | . | . | . | . | . | . | . | . | . | . |
| 21-225_10F5_LC | . | . | . | D | . | . | . | . | . | . | . | . | . | . | . | . | . | . | . | . | . | . | . | . | . | . |
| 21-225_12A10_LC | . | . | . | . | . | . | . | . | A | . | . | . | . | . | . | N | . | . | . | . | . | . | . | . | . | . |
| 21-225_13D4_LC | . | . | . | . | . | . | . | . | . | . | . | . | . | . | . | . | . | . | . | . | . | . | . | . | . | . |
| 21-225_146E9_LC | . | . | . | . | . | . | . | . | . | . | . | . | . | . | . | . | . | . | . | . | . | . | . | . | . | . |
| 21-225_146H1_LC | . | . | . | . | . | . | . | . | . | . | . | . | . | . | . | . | . | . | . | . | . | . | . | . | . | . |
| 21-225_148C4_LC | . | . | . | . | . | . | . | . | . | . | . | . | . | . | . | . | . | . | . | . | . | . | . | T | . | . |
| 21-225_148H9_LC | . | . | . | . | . | . | . | . | . | . | . | . | . | . | . | . | . | . | . | . | . | . | . | . | . | . |
| 21-225_149A3_LC | . | . | . | . | . | . | . | . | . | . | . | . | . | . | . | N | . | . | . | . | . | . | . | . | . | . |
| 21-225_150C11_LC | . | . | . | . | . | . | . | . | . | . | . | . | . | . | . | . | . | . | . | . | . | . | . | . | . | . |
| 21-225_150C12_LC | . | . | . | . | . | . | . | . | . | . | . | . | . | . | . | . | . | . | . | . | . | . | . | . | . | . |
| 21-225_151B12_LC | . | . | . | E | . | . | . | . | . | . | . | . | . | . | . | . | . | . | . | . | . | . | . | . | . | . |
| 21-225_152B11_LC | . | . | . | . | . | . | . | . | . | . | . | . | . | . | . | . | . | . | . | . | . | . | . | . | . | . |
| 21-225_152D2_LC | . | . | . | . | . | . | . | . | . | . | . | . | . | . | . | . | . | . | . | . | . | . | . | . | . | . |
| 21-225_152G4_LC | . | . | . | . | . | . | . | . | . | . | . | . | . | . | . | . | . | . | . | . | . | . | . | . | . | . |
| 21-225_155C4_LC | . | . | . | . | . | . | . | . | . | . | . | . | . | . | . | . | . | . | . | . | . | . | . | . | . | . |
| 21-225_156E4_LC | . | . | . | . | . | . | . | . | . | . | . | . | . | . | . | . | . | . | . | . | . | . | . | . | . | . |
| 21-225_159H2_LC | . | . | . | . | . | . | . | . | . | . | . | . | . | . | . | . | T | . | . | . | . | . | . | V | . | . |
| 21-225_15A3_LC | . | . | . | . | . | . | . | . | . | . | . | . | . | . | . | . | . | . | . | . | . | . | . | . | . | . |
| 21-225_16D11_LC | . | . | . | . | . | . | . | . | . | . | . | . | . | . | . | . | . | . | . | . | . | . | . | . | . | . |
| 21-225_16E6_LC | . | . | . | . | . | . | . | . | . | . | . | . | . | . | . | . | . | . | . | . | . | . | . | . | . | . |
| 21-225_17A6_LC | . | . | . | . | . | . | . | . | . | . | . | . | . | . | . | . | . | . | . | . | . | . | . | . | . | . |
| 21-225_17G4_LC | . | . | . | . | . | . | . | . | E | . | . | . | . | . | . | . | . | . | . | . | . | . | . | . | . | . |
| 21-225_18G4_LC | . | . | . | . | . | . | . | . | . | . | . | . | . | . | . | . | . | . | . | . | . | . | . | . | . | . |
| 21-225_196F4_LC | . | . | . | . | . | . | . | . | . | . | . | . | . | . | . | . | . | . | . | . | . | . | . | . | . | . |
| 21-225_19A1_LC | . | . | . | . | . | . | . | . | . | . | . | P | . | . | . | . | . | . | . | . | . | . | . | . | I | . |
| 21-225_1H12_LC | . | . | . | D | . | . | . | . | . | . | . | . | . | . | . | . | . | . | . | . | . | . | . | . | . | . |
| 21-225_201H10_LC | . | . | . | . | . | . | . | . | . | . | . | . | . | . | P | . | . | . | . | . | . | . | . | . | . | . |
| 21-225_203E3_LC | . | . | . | . | . | . | . | . | . | . | . | . | . | . | P | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
|  |  |  |  |  |  |  |  |  |  | K_FR3 |  |  |  |  |  |  |  |  |  |  |  |  |  |  |  |  |
| CONSENSUS | S | G | S | G | S | G | - | - | T | D | F | T | L | T | I | S | S | L | Q | P | E | D | F | A | T | Y |
| 21-225_204D6_LC | . | . | . | . | . | . | . | . | . | . | . | . | . | . | . | . | . | . | . | . | . | . | V | . | . | . |
| 21-225_204H4_LC | . | . | . | . | . | . | . | . | . | . | . | . | . | . | F | . | . | . | . | . | . | . | . | . | . | . |
| 21-225_206A4_LC | . | . | . | . | . | . | . | . | . | . | . | . | . | . | F | . | . | . | . | . | . | . | . | . | . | . |
| 21-225_206A5_LC | . | . | . | . | . | . | . | . | . | . | . | . | . | . | F | . | . | . | . | . | . | . | . | . | . | . |
| 21-225_207C9_LC | . | A | . | V | . | . | . | . | . | . | . | . | . | . | . | . | . | . | . | . | . | . | . | . | . | . |
| 21-225_208B11_LC | . | . | . | . | . | . | . | . | . | . | . | . | . | . | F | . | . | . | . | . | . | . | . | . | . | . |
| 21-225_208E8_LC | . | . | . | . | . | . | . | . | . | . | . | . | . | . | F | . | . | . | . | . | . | . | . | . | . | . |
| 21-225_20F6_LC | . | . | . | . | . | . | . | . | . | . | . | . | . | . | . | . | . | . | . | . | . | . | . | . | . | . |
| 21-225_210D11_LC | . | . | . | . | . | . | . | . | . | . | . | . | . | . | F | . | . | . | . | . | . | . | . | . | . | . |
| 21-225_22C7_LC | . | . | . | . | . | . | . | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . | . | . | . | . | . | . | . |
| 21-225_30G4_LC | . | . | . | . | . | . | . | . | . | . | . | . | . | . | i | . | M | . | . | . | . | . | . | S | . | . |
| 21-225_31F1_LC | . | . | . | . | . | . | . | . | . | . | . | . | . | . | . | . | . | . | . | . | . | . | . | . | . | . |
| 21-225_32D6_LC | . | . | . | . | . | . | . | . | . | . | . | . | . | . | . | . | . | . | A | . | . | . | . | . | . | . |
| 21-225_33B2_LC | . | . | . | . | . | . | . | . | . | . | . | . | . | . | . | . | . | . | . | . | . | . | . | . | . | . |
| 21-225_33C12_LC | N | . | . | . | . | . | . | . | . | . | . | . | . | . | . | . | . | . | . | . | . | . | . | . | . | . |
| 21-225_34C11_LC | . | . | . | F | . | . | . | . | . | . | . | . | . | . | . | . | . | . | K | . | . | . | . | . | . | . |
| 21-225_34C2_LC | . | . | . | . | . | . | . | . | Y | . | . | . | . | . | . | . | . | . | . | . | . | . | . | . | . | . |
| 21-225_34D3_LC | . | . | . | . | . | . | . | . | . | . | . | . | . | . | . | . | . | . | . | . | . | . | . | . | . | F |
| 21-225_34D8_LC | . | . | . | . | . | . | . | . | . | . | . | . | . | . | . | . | . | . | . | . | . | . | . | . | . | . |
| 21-225_34G11_LC | . | . | . | . | . | . | . | . | . | . | . | . | . | . | . | T | . | . | . | . | . | . | . | . | . | . |
| 21-225_36B8_LC | . | . | . | . | . | . | . | . | . | . | . | . | . | . | . | . | . | . | . | . | . | . | . | . | . | . |
| 21-225_3F8_LC | . | . | . | . | . | . | . | . | V | . | . | . | . | . | . | . | . | . | . | . | . | . | . | . | . | . |
| 21-225_3G7_LC | . | . | . | . | . | . | . | . | . | . | . | . | V | . | . | . | . | . | . | . | . | . | . | . | . | . |
| 21-225_48B10_LC | . | . | . | . | . | . | . | . | . | . | . | . | . | . | . | . | . | V | . | . | . | . | . | . | . | . |
| 21-225_48F12_LC | . | . | . | . | . | . | . | . | . | . | . | . | . | . | . | . | . | . | . | . | . | . | . | . | N | . |
| 21-225_48G12_LC | . | . | . | . | . | . | . | . | . | . | . | . | . | . | . | . | . | V | . | . | . | . | . | . | N | . |
| 21-225_4G4_LC | . | . | . | . | . | . | . | . | . | . | . | . | . | S | . | . | . | . | . | . | . | . | . | . | . | . |
| 21-225_50F2_LC | . | . | . | . | . | . | . | . | . | . | . | . | . | . | . | . | . | . | . | D | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR4 | | | | | | | | | | | | | | | |
| CONSENSUS | S | G | S | G | S | G | - | - | T | D | F | T | L | T | I | S | S | L | Q | P | E | D | F | A | T | Y |
| 21-225_56B6_LC | . | . | . | . | . | . | . | . | . | . | . | . | . | . | . | . | . | . | . | . | . | . | . | . | . | . |
| 21-225_62D2_LC | . | . | . | . | . | . | . | . | . | . | . | . | . | . | . | . | . | . | . | . | . | . | . | . | . | . |
| 21-225_63C10_LC | . | . | . | . | . | . | . | . | . | . | . | . | . | . | . | . | . | . | . | . | . | . | . | . | . | F |
| 21-225_64A11_LC | . | . | . | . | . | . | . | . | . | . | . | . | . | . | . | . | V | . | R | . | . | . | . | . | . | . |
| 21-225_64F7_LC | . | . | . | . | . | . | . | . | . | . | . | . | . | . | . | . | . | . | . | . | . | . | . | . | A | . |
| 21-225_6G1_LC | . | . | . | . | . | . | . | . | . | . | . | . | . | . | . | . | . | . | . | . | . | . | . | . | . | . |
| 21-225_7A10_LC | . | . | . | . | . | . | . | . | . | . | S | . | . | . | . | N | . | . | . | . | . | . | . | S | . | . |
| 21-225_7E11_LC | . | . | . | . | . | . | . | . | . | . | . | . | . | . | . | . | . | . | . | . | . | . | . | . | } | . |
| 21-225_7E11.001_LC | . | . | . | . | . | . | . | . | . | . | . | . | . | . | . | . | . | . | . | . | . | . | . | . | } | . |
| 21-225_7E11.001.001_LC | . | . | . | . | . | . | . | . | . | . | . | . | . | . | . | . | . | . | . | . | . | . | . | . | } | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . | . | . | . | . | . | } | . |
| 21-225_7E11.001.004_LC | . | . | . | . | . | . | . | . | . | . | . | . | . | . | . | . | . | . | . | . | . | . | . | . | } | . |
| 21-225_7E11.001.005_LC | . | . | . | . | . | . | . | . | . | . | . | . | . | . | . | . | . | . | . | . | . | . | . | . | } | . |
| 21-225_7E11.001.006_LC | . | . | . | . | . | . | . | . | . | . | . | . | . | . | . | . | . | . | . | . | . | . | . | . | } | . |
| 21-225_7E11.001.007_LC | . | . | . | . | . | . | . | . | . | . | . | . | . | . | . | . | . | . | . | . | . | . | . | . | } | . |
| 21-225_7E11.001.008_LC | . | . | . | . | . | . | . | . | . | . | . | . | . | . | . | . | . | . | . | . | . | . | . | . | } | . |
| 21-225_7E11.001.009_LC | . | . | . | . | . | . | . | . | . | . | . | . | . | . | . | . | . | . | . | . | . | . | . | . | } | . |
| 21-225_7E11.001.010_LC | . | . | . | . | . | . | . | . | . | . | . | . | . | . | . | . | . | . | . | . | . | . | . | . | } | . |
| 21-225_7E11.001.011_LC | . | . | . | . | . | . | . | . | . | . | . | . | . | . | . | . | . | . | . | . | . | . | . | . | } | . |
| 21-225_7E11.001.012_LC | . | . | . | . | . | . | . | . | . | . | . | . | . | . | . | . | . | . | . | . | . | . | . | . | } | . |
| 21-225_7E11.001.013_LC | . | . | . | . | . | . | . | . | . | . | . | . | . | . | . | . | . | . | . | . | . | . | . | . | } | . |
| 21-225_7E11.001.014_LC | . | . | . | . | . | . | . | . | . | . | . | . | . | . | . | . | . | . | . | . | . | . | . | . | } | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . | . | . | . | . | . | } | . |
| 21-225_7E11.001.019_LC | . | . | . | . | . | . | . | . | . | . | . | . | . | . | . | . | . | . | . | . | . | . | . | . | } | . |
| 21-225_7E11.001.021_LC | . | . | . | . | . | . | . | . | . | . | . | . | . | . | . | . | . | . | . | . | . | . | . | . | } | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_7E11.001.022_LC | . | . | . | . | . | . | . | . | . | . | . | . | . | . | . | . | . | . | . | . | . | . | . | . | : | . |
| 21-225_7E11.001.023_LC | . | . | . | . | . | . | . | . | . | . | . | . | . | . | . | . | . | . | . | . | . | . | . | . | : | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_10E12_LC | . | . | . | . | N | . | . | . | . | . | . | . | . | . | . | . | . | . | . | . | . | . | . | . | . | . |
| 21-225_10F5_LC | . | . | . | . | . | . | . | . | . | . | . | . | . | . | . | . | . | . | . | . | . | . | . | . | . | . |
| 21-225_12A10_LC | . | . | . | . | . | . | . | . | . | . | . | . | . | . | . | . | . | . | . | . | . | . | . | . | . | . |
| 21-225_13D4_LC | . | . | . | . | T | . | . | . | . | . | . | . | . | . | . | . | . | . | . | . | . | . | . | . | . | . |
| 21-225_146E9_LC | F | . | . | . | . | . | - | . | . | . | . | . | . | . | . | . | . | . | . | . | . | . | . | . | . | . |
| 21-225_146H1_LC | . | . | . | . | . | . | - | . | . | . | . | . | . | . | . | . | . | . | . | . | . | . | . | . | . | . |
| 21-225_148C4_LC | . | . | . | . | . | . | - | . | . | . | . | . | . | . | . | . | . | . | . | . | . | . | . | . | . | . |
| 21-225_148H9_LC | . | . | . | . | . | . | - | . | . | . | . | . | . | . | . | . | . | . | . | . | . | . | . | . | . | . |
| 21-225_149A3_LC | . | . | . | . | . | . | - | . | . | . | . | . | . | . | . | . | . | . | . | . | . | . | . | . | . | . |
| 21-225_150C11_LC | . | . | . | . | . | . | - | . | . | . | . | . | . | . | . | . | . | . | . | . | . | . | . | . | . | . |
| 21-225_150C12_LC | . | . | . | . | . | S | - | . | . | . | . | . | . | . | . | . | . | . | . | . | . | . | . | . | . | . |
| 21-225_151B12_LC | . | . | . | . | . | . | - | . | . | . | . | . | . | . | . | . | . | . | . | . | . | . | . | . | . | . |
| 21-225_152B11_LC | F | . | . | . | . | . | - | . | . | . | . | . | . | . | . | . | . | . | . | . | . | . | . | . | . | . |
| 21-225_152D2_LC | F | . | . | . | . | . | - | . | . | . | . | . | . | . | . | . | . | . | . | . | . | . | . | . | . | . |
| 21-225_152G4_LC | F | . | . | . | . | . | - | . | . | . | . | . | . | . | . | . | . | . | . | . | . | . | . | . | . | . |
| 21-225_155C4_LC | . | . | . | . | . | . | - | . | . | . | . | . | . | . | . | . | . | . | . | . | . | . | . | . | . | . |
| 21-225_156E4_LC | . | . | . | . | . | . | - | . | . | . | . | . | . | . | . | . | . | . | . | . | . | . | . | . | . | . |
| 21-225_159H2_LC | . | . | . | . | . | . | . | . | . | . | . | . | . | . | . | . | . | . | . | . | . | . | . | . | . | . |
| 21-225_15A3_LC | . | . | . | . | T | . | . | . | . | . | . | . | . | . | . | . | . | . | . | . | . | . | . | . | . | . |
| 21-225_16D11_LC | . | . | . | . | R | . | . | . | . | . | . | . | . | . | . | . | . | . | . | . | . | . | . | . | . | . |
| 21-225_16E6_LC | . | . | . | T | . | . | . | . | . | . | . | . | . | . | . | . | . | . | . | . | . | . | . | . | . | . |
| 21-225_17A6_LC | . | . | . | . | R | T | . | . | . | . | . | . | . | . | . | . | . | . | . | . | . | . | . | . | . | . |
| 21-225_17G4_LC | . | . | . | . | N | S | . | . | . | . | . | . | . | . | . | . | . | . | . | . | . | . | . | . | . | . |
| 21-225_18G4_LC | . | . | . | T | . | . | . | . | . | . | . | . | . | . | . | . | . | . | . | . | . | . | . | . | . | . |
| 21-225_196F4_LC | . | . | . | . | . | G | . | . | . | . | . | . | . | . | . | . | . | . | . | . | . | . | . | . | . | . |
| 21-225_19A1_LC | . | . | . | . | . | N | . | . | . | . | . | . | . | . | . | . | . | . | . | . | . | . | . | . | . | . |
| 21-225_1H12_LC | . | . | . | . | . | . | . | . | . | . | . | . | . | . | . | . | . | . | . | . | . | . | . | . | . | . |
| 21-225_201H10_LC | . | . | . | . | . | . | . | . | . | . | . | . | . | . | . | . | . | . | . | . | . | . | . | . | . | . |
| 21-225_203E3_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_204D6_LC | . | . | . | . | . | . | . | . | . | . | . | . | . | . | . | . | . | . | . | . | . | . | . | . | . | . |
| 21-225_204H4_LC | . | . | . | . | . | . | . | . | . | . | . | . | . | . | . | . | . | . | . | . | . | . | . | . | . | . |
| 21-225_206A4_LC | . | . | . | . | . | . | . | . | . | . | . | . | . | . | . | . | . | . | . | . | . | . | . | . | . | . |
| 21-225_206A5_LC | . | . | . | . | . | . | . | . | . | . | . | . | . | . | . | . | . | . | . | . | . | . | . | . | . | . |
| 21-225_207C9_LC | . | . | . | . | . | . | . | . | . | . | . | . | . | . | . | . | . | . | . | . | . | . | . | . | . | . |
| 21-225_208B11_LC | . | . | . | . | . | . | . | . | . | . | . | . | . | . | . | . | . | . | . | . | . | . | . | . | . | . |
| 21-225_208E8_LC | . | . | . | . | . | . | . | . | . | . | . | . | . | . | . | . | . | . | . | . | . | . | . | . | . | . |
| 21-225_20F6_LC | . | . | . | . | T | . | . | . | . | . | . | . | . | . | . | . | . | . | . | . | . | . | . | . | . | . |
| 21-225_210D11_LC | . | . | . | . | . | . | . | . | . | . | . | . | . | . | . | . | . | . | . | . | . | . | . | . | . | . |
| 21-225_22C7_LC | . | . | . | . | T | . | . | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . | . | . | . | . | . | . | . |
| 21-225_30G4_LC | . | . | . | Λ | . | . | . | . | . | . | . | . | . | . | . | . | . | . | . | . | . | . | . | . | . | . |
| 21-225_31F1_LC | . | . | . | . | . | . | I | . | . | . | . | . | . | . | . | . | . | . | . | . | . | . | . | . | . | . |
| 21-225_32D6_LC | . | . | . | . | . | . | . | . | . | . | . | . | . | . | . | . | . | . | . | . | . | . | . | . | . | . |
| 21-225_33B2_LC | . | . | . | . | . | . | . | . | . | . | . | . | . | . | . | . | . | . | . | . | . | . | . | . | . | . |
| 21-225_33C12_LC | . | . | . | . | . | . | . | . | . | . | . | . | . | . | . | . | . | . | . | . | . | . | . | . | . | . |
| 21-225_34C11_LC | . | . | . | . | T | C | . | . | . | . | . | . | . | . | . | . | . | . | . | . | . | . | . | . | . | . |
| 21-225_34C2_LC | . | . | . | . | . | . | . | . | . | . | . | . | . | . | . | . | . | . | . | . | . | . | . | . | . | . |
| 21-225_34D3_LC | . | . | . | . | . | . | . | . | . | . | . | . | . | . | . | . | . | . | . | . | . | . | . | . | . | . |
| 21-225_34D8_LC | . | . | . | . | . | . | . | . | . | . | . | . | . | . | . | . | . | . | . | . | . | . | . | . | . | . |
| 21-225_34G11_LC | . | . | . | . | T | . | I | . | . | . | . | . | . | . | . | . | . | . | . | . | . | . | . | . | . | . |
| 21-225_36B8_LC | . | . | . | . | . | . | . | . | . | . | . | . | . | . | . | . | . | . | . | . | . | . | . | . | . | . |
| 21-225_3F8_LC | . | . | . | . | T | . | . | . | . | . | . | . | . | . | . | . | . | . | . | . | . | . | . | . | . | . |
| 21-225_3G7_LC | . | . | . | . | . | . | . | . | . | . | . | . | . | . | . | . | . | . | . | . | . | . | . | . | . | . |
| 21-225_48B10_LC | . | . | . | . | T | . | . | . | . | . | . | . | . | . | . | . | . | . | . | . | . | . | . | . | . | . |
| 21-225_48F12_LC | . | . | . | . | T | . | . | . | . | . | . | . | . | . | . | . | . | . | . | . | . | . | . | . | . | . |
| 21-225_48G12_LC | . | . | . | . | T | . | . | . | . | . | . | . | . | . | . | . | . | . | . | . | . | . | . | . | . | . |
| 21-225_4G4_LC | . | . | . | . | T | . | . | . | . | . | . | . | . | . | . | . | . | . | . | . | . | . | . | . | . | . |
| 21-225_50F2_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_56B6_LC | . | . | . | . | T | . | . | . | . | . | . | . | . | . | . | . | . | . | . | . | . | . | . | . | . | . |
| 21-225_62D2_LC | . | . | . | . | T | . | . | . | . | . | . | . | . | . | . | . | . | . | . | . | . | . | . | . | . | . |
| 21-225_63C10_LC | . | . | . | . | T | . | . | . | . | . | . | . | . | . | . | . | . | . | . | . | . | . | . | . | . | . |
| 21-225_64A11_LC | S | . | . | . | . | D | . | . | . | . | . | . | . | . | . | . | . | . | . | . | . | . | . | . | . | . |
| 21-225_64F7_LC | . | . | . | . | . | . | N | . | . | . | . | . | . | . | . | . | . | . | . | . | . | . | . | . | . | . |
| 21-225_6G1_LC | . | . | . | . | P | H | . | . | . | . | . | . | . | . | . | . | . | . | . | . | . | . | . | . | . | . |
| 21-225_7A10_LC | . | . | . | . | . | . | . | . | . | . | . | . | . | . | . | . | . | . | . | . | . | . | . | . | . | . |
| 21-225_7E11_LC | . | . | . | . | T | . | . | . | . | . | . | . | . | . | . | . | . | . | . | . | . | . | . | . | . | . |
| 21-225_7E11.001_LC | . | . | . | . | T | . | . | . | . | . | . | . | . | . | . | . | . | . | . | . | . | . | . | . | . | . |
| 21-225_7E11.001.001_LC | . | . | . | . | T | . | . | . | . | . | . | . | . | . | . | . | . | . | . | . | . | . | . | . | . | . |
| 21-225_7E11.001.002_LC | . | . | . | . | T | . | . | . | . | . | . | . | . | . | . | . | . | . | . | . | . | . | . | . | . | . |
| 21-225_7E11.001.003_LC | . | . | . | . | T | . | . | . | . | . | . | . | . | . | . | . | . | . | . | . | . | . | . | . | . | . |
| 21-225_7E11.001.004_LC | . | . | . | . | T | . | . | . | . | . | . | . | . | . | . | . | . | . | . | . | . | . | . | . | . | . |
| 21-225_7E11.001.005_LC | . | . | . | . | T | . | . | . | . | . | . | . | . | . | . | . | . | . | . | . | . | . | . | . | . | . |
| 21-225_7E11.001.006_LC | . | . | . | . | T | . | . | . | . | . | . | . | . | . | . | . | . | . | . | . | . | . | . | . | . | . |
| 21-225_7E11.001.007_LC | . | . | . | . | T | . | . | . | . | . | . | . | . | . | . | . | . | . | . | . | . | . | . | . | . | . |
| 21-225_7E11.001.008_LC | . | . | . | . | T | . | . | . | . | . | . | . | . | . | . | . | . | . | . | . | . | . | . | . | . | . |
| 21-225_7E11.001.009_LC | . | . | . | . | T | . | . | . | . | . | . | . | . | . | . | . | . | . | . | . | . | . | . | . | . | . |
| 21-225_7E11.001.010_LC | . | . | . | . | T | . | . | . | . | . | . | . | . | . | . | . | . | . | . | . | . | . | . | . | . | . |
| 21-225_7E11.001.011_LC | . | . | . | . | T | . | . | . | . | . | . | . | . | . | . | . | . | . | . | . | . | . | . | . | . | . |
| 21-225_7E11.001.012_LC | . | . | . | . | T | . | . | . | . | . | . | . | . | . | . | . | . | . | . | . | . | . | . | . | . | . |
| 21-225_7E11.001.013_LC | . | . | . | . | T | . | . | . | . | . | . | . | . | . | . | . | . | . | . | . | . | . | . | . | . | . |
| 21-225_7E11.001.014_LC | . | . | . | . | T | . | . | . | . | . | . | . | . | . | . | . | . | . | . | . | . | . | . | . | . | . |
| 21-225_7E11.001.015_LC | . | . | . | . | T | . | . | . | . | . | . | . | . | . | . | . | . | . | . | . | . | . | . | . | . | . |
| 21-225_7E11.001.016_LC | . | . | . | . | T | . | . | . | . | . | . | . | . | . | . | . | . | . | . | . | . | . | . | . | . | . |
| 21-225_7E11.001.017_LC | . | . | . | . | T | . | . | . | . | . | . | . | . | . | . | . | . | . | . | . | . | . | . | . | . | . |
| 21-225_7E11.001.018_LC | . | . | . | . | T | . | . | . | . | . | . | . | . | . | . | . | . | . | . | . | . | . | . | . | . | . |
| 21-225_7E11.001.019_LC | . | . | . | . | T | . | . | . | . | . | . | . | . | . | . | . | . | . | . | . | . | . | . | . | . | . |
| 21-225_7E11.001.021_LC | . | . | . | . | T | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_7E11.001.022_LC | . | . | . | . | T | . | . | . | . | . | . | . | . | . | . | . | . | . | . | . | . | . | . | . | . | . |
| 21-225_7E11.001.023_LC | . | . | . | . | T | . | . | . | . | . | . | . | . | . | . | . | . | . | . | . | . | . | . | . | . | . |

EP 4 435 105 A2

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | K_FR4 | | | | | | | | | | | |
| CONSENSUS | - | - | - | - | T | P | L | T | F | G | G | G | T | K | V | E | I | K | R |
| 21-225_10E12_LC | . | . | . | . | P | . | . | . | . | . | . | . | . | . | . | . | . | E | . |
| 21-225_10F5_LC | . | . | . | . | P | . | . | . | . | . | . | . | . | . | . | . | . | . | . |
| 21-225_12A10_LC | . | . | . | . | P | . | . | . | . | . | . | . | . | . | . | . | . | . | . |
| 21-225_13D4_LC | . | . | . | . | L | . | . | . | . | . | . | . | . | . | . | . | . | . | . |
| 21-225_146E9_LC | . | . | . | . | S | T | P | . | . | . | . | . | . | . | . | . | . | . | . |
| 21-225_146H1_LC | . | . | . | . | S | T | P | . | . | . | . | . | . | . | . | . | . | . | . |
| 21-225_148C4_LC | . | . | . | . | S | T | P | . | . | . | . | . | . | . | . | . | . | E | . |
| 21-225_148H9_LC | . | . | . | . | S | T | P | . | . | . | . | . | . | . | . | . | . | . | . |
| 21-225_149A3_LC | . | . | . | . | S | I | P | . | . | . | . | . | . | . | . | . | . | . | . |
| 21-225_150C11_LC | . | . | . | . | S | I | Y | . | . | . | . | . | S | . | . | . | . | . | . |
| 21-225_150C12_LC | . | . | . | . | S | T | P | . | . | . | . | . | . | . | . | . | . | . | . |
| 21-225_151B12_LC | . | . | . | . | S | T | P | . | . | . | . | . | . | R | . | . | . | . | . |
| 21-225_152B11_LC | . | . | . | . | S | T | P | . | . | . | . | . | . | . | . | . | . | . | . |
| 21-225_152D2_LC | . | . | . | . | S | T | P | . | . | . | . | . | . | . | . | . | . | . | . |
| 21-225_152G4_LC | . | . | . | . | S | T | P | . | . | . | . | . | . | . | . | . | . | . | . |
| 21-225_155C4_LC | . | . | . | . | S | T | P | . | . | . | . | . | . | R | . | . | . | . | . |
| 21-225_156E4_LC | . | . | . | . | S | A | P | . | . | . | . | . | . | . | . | . | . | . | . |
| 21-225_159H2_LC | . | . | . | . | L | . | V | . | . | . | . | . | . | . | . | . | . | . | . |
| 21-225_15A3_LC | . | . | . | . | . | . | . | . | . | . | . | . | . | . | . | . | . | . | . |
| 21-225_16D11_LC | . | . | . | . | S | . | . | . | . | . | . | . | . | . | . | . | . | . | . |
| 21-225_16E6_LC | . | . | . | . | . | . | . | . | . | . | . | . | . | . | . | . | . | . | . |
| 21-225_17A6_LC | . | . | . | . | S | . | . | . | . | . | . | . | . | . | . | . | . | . | . |
| 21-225_17G4_LC | . | . | . | . | F | . | . | . | . | . | . | . | . | . | . | . | . | I | . |
| 21-225_18G4_LC | . | . | . | . | P | . | . | . | . | . | . | . | . | . | . | . | . | . | . |
| 21-225_196F4_LC | . | . | . | . | S | . | . | . | . | . | . | . | . | . | . | . | . | . | . |
| 21-225_19A1_LC | . | . | . | . | F | . | . | . | . | . | . | . | . | . | . | . | . | . | . |
| 21-225_1H12_LC | . | . | . | . | P | . | . | . | . | . | . | . | . | . | . | . | . | . | . |
| 21-225_201H10_LC | . | . | . | . | F | . | . | . | . | . | . | . | . | . | . | . | M | R | . |
| 21-225_203E3_LC | . | . | . | . | F | . | . | . | . | . | . | . | . | . | . | . | M | R | . |

4773

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | T | P | L | T | F | G | G | G | T | K | V | E | I | K | R |
| 21-225_204D6_LC | . | . | . | . | S | . | . | . | . | . | . | . | . | . | . | . | . | Q | . |
| 21-225_204H4_LC | . | . | . | . | F | . | . | . | . | . | . | . | . | . | . | . | M | R | . |
| 21-225_206A4_LC | . | . | . | . | F | . | . | . | . | . | . | . | . | . | . | . | M | R | . |
| 21-225_206A5_LC | . | . | . | . | F | . | . | . | . | . | . | . | . | . | . | . | M | R | . |
| 21-225_207C9_LC | . | . | . | . | I | . | . | . | . | . | . | . | . | . | . | . | . | . | . |
| 21-225_208B11_LC | . | . | . | . | F | . | . | . | . | . | . | . | . | . | . | . | M | R | . |
| 21-225_208E8_LC | . | . | . | . | F | . | . | . | . | . | . | . | . | . | . | . | M | R | . |
| 21-225_20F6_LC | . | . | . | . | P | . | . | . | . | . | . | . | . | . | . | . | . | . | . |
| 21-225_210D11_LC | . | . | . | . | F | . | . | . | . | . | . | . | . | . | . | . | M | R | . |
| 21-225_22C7_LC | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . |
| 21-225_30G4_LC | . | . | . | . | P | . | Y | . | . | . | . | . | . | . | . | . | . | . | . |
| 21-225_31F1_LC | . | . | . | . | . | . | . | . | . | . | . | . | . | T | . | . | . | R | . |
| 21-225_32D6_LC | . | . | . | . | . | Y | . | . | . | . | . | . | . | . | A | . | . | . | . |
| 21-225_33B2_LC | . | . | . | . | . | Y | . | . | . | . | . | . | . | . | . | . | . | . | . |
| 21-225_33C12_LC | . | . | . | . | . | Y | . | . | . | . | . | . | . | . | . | . | . | . | . |
| 21-225_34C11_LC | . | . | . | . | . | . | . | . | . | . | . | . | . | . | . | . | . | . | . |
| 21-225_34C2_LC | . | . | . | . | . | Y | . | . | . | . | . | . | . | M | . | . | . | . | . |
| 21-225_34D3_LC | . | . | . | . | . | . | . | . | . | . | . | . | . | . | . | . | . | . | . |
| 21-225_34D8_LC | . | . | . | . | . | Y | . | . | . | . | . | . | . | . | . | . | . | . | . |
| 21-225_34G11_LC | . | . | . | . | . | . | . | . | . | . | . | . | . | . | . | . | . | . | . |
| 21-225_36B8_LC | . | . | . | . | . | Y | . | . | . | . | . | . | . | M | . | . | . | . | . |
| 21-225_3F8_LC | . | . | . | . | . | . | . | . | . | . | . | . | . | R | . | . | . | N | . |
| 21-225_3G7_LC | . | . | . | . | P | . | P | . | . | . | . | . | A | . | . | . | . | D | . |
| 21-225_48B10_LC | . | . | . | . | N | . | . | . | . | . | . | . | . | . | . | . | P | T | . |
| 21-225_48F12_LC | . | . | . | . | N | . | . | . | . | . | . | . | . | S | . | . | . | T | . |
| 21-225_48G12_LC | . | . | . | . | N | . | . | . | . | . | . | . | . | S | . | . | . | T | . |
| 21-225_4G4_LC | . | . | . | . | . | . | . | . | . | . | . | . | . | . | . | . | . | . | . |
| 21-225_50F2_LC | . | . | . | . | P | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | L | T | F | G | G | G | T | K | V | E | I | K | R |
| 21-225_56B6_LC | . | . | . | . | P | . | . | . | . | . | . | . | . | . | . | . | . | . | . |
| 21-225_62D2_LC | . | . | . | . | P | . | . | . | . | . | . | . | . | . | . | . | . | . | . |
| 21-225_63C10_LC | . | . | . | . | P | . | . | . | . | . | . | . | . | . | . | . | . | . | . |
| 21-225_64A11_LC | . | . | . | . | F | . | . | . | . | . | . | . | . | . | . | . | . | . | . |
| 21-225_64F7_LC | . | . | . | . | I | S | P | . | . | . | . | . | . | . | . | . | L | . | . |
| 21-225_6G1_LC | . | . | . | . | P | . | . | . | . | . | . | . | . | . | . | . | . | . | . |
| 21-225_7A10_LC | . | . | . | . | P | . | . | . | . | . | . | . | . | . | . | . | . | R | . |
| 21-225_7E11_LC | . | . | . | . | . | . | . | . | . | . | . | . | . | . | . | . | . | . | . |
| 21-225_7E11.001_LC | . | . | . | . | . | . | . | . | . | . | . | . | . | . | . | . | . | . | . |
| 21-225_7E11.001.001_LC | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . |
| 21-225_7E11.001.004_LC | . | . | . | . | . | . | . | . | . | . | . | . | . | . | . | . | . | . | . |
| 21-225_7E11.001.005_LC | . | . | . | . | . | . | . | . | . | . | . | . | . | . | . | . | . | . | . |
| 21-225_7E11.001.006_LC | . | . | . | . | . | . | . | . | . | . | . | . | . | . | . | . | . | . | . |
| 21-225_7E11.001.007_LC | . | . | . | . | . | . | . | . | . | . | . | . | . | . | . | . | . | . | . |
| 21-225_7E11.001.008_LC | . | . | . | . | . | . | . | . | . | . | . | . | . | . | . | . | . | . | . |
| 21-225_7E11.001.009_LC | . | . | . | . | . | . | . | . | . | . | . | . | . | . | . | . | . | . | . |
| 21-225_7E11.001.010_LC | . | . | . | . | . | . | . | . | . | . | . | . | . | . | . | . | . | . | . |
| 21-225_7E11.001.011_LC | . | . | . | . | . | . | . | . | . | . | . | . | . | . | . | . | . | . | . |
| 21-225_7E11.001.012_LC | . | . | . | . | . | . | . | . | . | . | . | . | . | . | . | . | . | . | . |
| 21-225_7E11.001.013_LC | . | . | . | . | . | . | . | . | . | . | . | . | . | . | . | . | . | . | . |
| 21-225_7E11.001.014_LC | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . |
| 21-225_7E11.001.019_LC | . | . | . | . | . | . | . | . | . | . | . | . | . | . | . | . | . | . | . |
| 21-225_7E11.001.021_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | L | T | F | G | G | G | T | K | V | E | I | K | R |
| 21-225_7E11.001.022_LC | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . |

**FIGURE 57**

Table 103. Consensus 23 - VK1|L1/JK3 (SEQ ID NO: 50320):

DIQMTQSPSSLSASVGDRVTITC<u>RAS</u>--<u>QGIS</u>------<u>NYLA</u>WFQQKPGKAPKSLIY<u>A</u>--------<u>ASSLQS</u>GVPSKFSGSGSG--TDFTLTISSLQPEDFATYYC<u>QQYNS</u>---------------------<u>YPFT</u>FGPGTKVDIKR wherein:

R at position 24 can be substituted with P

A at position 25 can be substituted with T

S at position 26 can be substituted with N

Q at position 29 can be substituted with R

G at position 30 can be substituted with D, V or A

I at position 31 can be substituted with V

S at position 32 can be substituted with N, G, R, T or K

N at position 39 can be substituted with K, Y, H, I or T

Y at position 40 can be substituted with H

A at position 42 can be substituted with V or S

A at position 58 can be substituted with V, G or T

A at position 67 can be substituted with S or V

S at position 68 can be substituted with F

S at position 69 can be substituted with G, N or T

L at position 70 can be substituted with V

Q at position 71 can be substituted with R, H, L or E

S at position 72 can be substituted with G or T

**FIGURE 57**

Q at position 107 can be substituted with H, L, P or R

Q at position 108 can be substituted with R, K, H or L

Y at position 109 can be substituted with F

N at position 110 can be substituted with H, D, Y, S, K, L, M or Q

S at position 111 can be substituted with T, G, N, C or D

Y at position 135 can be substituted with F or H

F at position 137 can be substituted with V, L or I

T at position 138 can be substituted with K

Xaa Xaa Xaa Xaa Xaa Xaa Xaa Xaa Xaa Leu Xaa (SEQ ID NO: 50144)

Xaa Xaa Xaa Xaa Xaa Xaa Xaa (SEQ ID NO: 50145)

Xaa Xaa Xaa Xaa Xaa Xaa Pro Xaa Xaa (SEQ ID NO: 50146)

Arg Ala Ser Gln Gly Ile Ser Asn Tyr Leu Ala (SEQ ID NO: 50630)

Ala Ala Ser Ser Leu Gln Ser (SEQ ID NO: 50631)

Gln Gln Tyr Asn Ser Tyr Pro Phe Thr (SEQ ID NO: 50632)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | K_FR1 | | | | | | | | | | | | |
| CONSENSUS | D | I | Q | M | T | Q | S | P | S | S | L | S | A | S | V | G | D | R | V | T | I | T | C | R | A | S |
| 21-225_148B6_LC | . | . | . | . | . | . | . | . | . | . | . | . | . | . | . | . | . | . | . | S | . | . | . | . | . | . |
| 21-225_14H8_LC | . | . | . | . | . | . | . | . | . | . | . | . | . | . | . | . | . | . | . | . | . | . | . | . | . | . |
| 21-225_153A1_LC | . | . | . | . | . | . | . | . | . | . | . | . | . | . | . | . | . | . | . | . | . | . | . | . | . | . |
| 21-225_157E4_LC | . | . | . | . | . | . | . | . | . | . | . | . | . | . | . | . | . | . | . | P | . | . | . | . | . | . |
| 21-225_157G5_LC | . | . | . | . | . | . | . | . | . | . | . | . | . | . | . | . | . | . | . | . | . | . | . | . | . | . |
| 21-225_157H1_LC | . | . | . | . | . | . | . | . | . | . | . | . | . | . | . | . | . | . | . | . | . | . | . | . | . | . |
| 21-225_157H10_LC | . | . | . | . | . | . | . | . | . | . | . | . | . | . | . | . | . | . | . | . | . | . | . | . | . | . |
| 21-225_158H12_LC | . | . | . | . | . | . | . | . | . | . | . | . | . | . | . | . | . | . | . | . | . | . | . | . | . | . |
| 21-225_159H11_LC | . | . | . | . | . | . | . | . | . | . | . | . | . | . | . | . | . | . | . | . | . | . | . | . | . | . |
| 21-225_15A2_LC | . | . | . | . | . | . | . | . | . | . | . | P | . | P | I | . | . | . | . | . | . | . | . | . | . | . |
| 21-225_15B1_LC | . | . | . | . | . | . | . | . | . | . | . | . | . | . | . | . | . | . | . | . | . | . | . | . | . | . |
| 21-225_160G1_LC | . | . | . | . | . | . | . | . | . | . | . | . | . | . | . | . | . | . | . | . | . | . | . | . | . | . |
| 21-225_160G3_LC | . | . | . | . | . | . | . | . | . | . | . | . | . | . | . | . | . | . | . | . | . | . | . | . | . | . |
| 21-225_163F1_LC | . | . | . | . | . | . | . | . | . | . | . | . | . | . | . | . | . | . | . | . | . | . | . | . | . | . |
| 21-225_167D12_LC | . | . | . | . | . | . | . | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . | . | . | . | . | . | . | . |
| 21-225_16B8_LC | . | . | . | . | . | . | . | . | . | . | . | . | . | . | . | . | . | . | . | . | . | . | . | . | . | . |
| 21-225_16C7_LC | . | . | . | . | . | . | . | . | . | . | . | . | . | . | . | . | . | . | . | . | . | . | . | . | . | . |
| 21-225_16G12_LC | . | . | . | . | . | . | . | . | . | . | . | . | . | . | . | . | . | . | . | . | . | . | . | . | . | . |
| 21-225_170E1_LC | . | . | . | . | . | . | . | . | . | . | . | . | . | . | . | E | . | . | . | . | . | . | . | . | . | . |
| 21-225_175C4_LC | . | . | . | . | . | . | . | . | . | . | . | . | . | . | . | . | . | . | . | . | . | . | . | . | . | . |
| 21-225_177D3_LC | . | . | . | . | S | . | . | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . | . | . | . | . | . | . | . |
| 21-225_17H6_LC | . | . | . | . | . | . | . | . | . | . | . | . | . | . | . | . | . | . | . | . | . | . | . | . | . | . |
| 21-225_181G2_LC | . | . | . | . | . | . | . | . | . | . | . | . | . | . | I | . | E | . | . | . | . | . | . | . | . | . |
| 21-225_184E7_LC | . | . | . | . | . | . | . | . | . | . | E | . | . | . | I | . | E | . | . | . | . | . | . | . | . | . |
| 21-225_185A1_LC | . | . | . | . | . | . | . | . | . | . | . | . | . | . | . | . | . | . | . | . | . | . | . | . | . | . |
| 21-225_185E10_LC | . | . | . | . | . | . | . | . | . | . | E | . | . | . | I | . | E | . | . | . | . | . | . | . | . | . |
| 21-225_188B9_LC | . | . | . | . | . | . | . | . | . | . | . | . | . | . | I | . | E | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_188H5_LC | . | | | | | | | | | | | . | . | . | . | | . | | . | . | . | . | . | . | . | . |
| 21-225_18D11_LC | . | | . | | | . | | . | | . | . | . | | . | . | | . | . | . | | . | . | . | . | . | . |
| 21-225_18G10_LC | . | . | . | | | | . | | . | . | . | . | | . | | . | . | . | . | . | . | . | . | . | . | . |
| 21-225_190A5_LC | . | . | . | | . | . | . | | . | . | . | . | . | . | . | . | . | . | . | . | . | . | . | . | . | . |
| 21-225_190B1_LC | . | . | . | | . | . | . | | . | . | . | . | . | . | . | . | . | . | . | . | . | . | . | . | . | . |
| 21-225_190F12_LC | . | . | . | | . | . | . | | . | . | . | . | . | . | . | . | . | . | . | . | . | . | . | . | . | . |
| 21-225_190H9_LC | . | . | . | | . | . | . | . | . | . | . | . | . | . | . | . | . | . | . | . | . | . | . | . | . | . |
| 21-225_192A2_LC | . | . | . | | . | . | . | . | . | . | . | . | . | . | . | . | . | . | . | . | . | . | . | . | . | N |
| 21-225_192C10_LC | . | . | . | | . | . | . | . | . | . | . | . | . | . | . | . | . | . | . | . | . | . | . | . | . | . |
| 21-225_192E4_LC | . | . | . | | . | . | . | . | . | . | . | . | . | . | . | . | . | . | . | . | . | . | . | . | . | . |
| 21-225_192H6_LC | . | . | . | | . | . | . | . | . | . | . | . | . | . | . | . | . | . | . | . | . | . | . | . | . | . |
| 21-225_193F8_LC | . | . | . | I | . | . | . | . | . | . | . | . | . | . | . | . | . | . | . | . | . | . | . | . | . | . |
| 21-225_194H12_LC | . | . | . | | . | . | . | . | . | . | . | . | . | . | . | . | . | . | . | . | . | . | . | . | . | . |
| 21-225_197E11_LC | . | . | . | | . | . | . | . | . | . | . | . | . | . | . | . | . | . | . | . | . | . | . | . | . | . |
| 21-225_20B9_LC | . | . | . | | . | . | . | . | . | . | . | . | . | . | . | . | . | . | . | . | . | . | . | . | . | . |
| 21-225_20C11_LC | . | . | . | | . | . | . | . | . | . | . | . | . | . | . | . | . | . | . | . | . | . | . | . | . | . |
| 21-225_20F10_LC | . | . | . | L | . | . | . | . | . | . | . | . | . | . | . | . | . | . | . | . | . | . | . | . | . | . |
| 21-225_20H7_LC | . | . | . | | . | . | . | . | . | . | . | . | . | . | . | . | . | . | . | . | . | . | . | Y | . | . |
| 21-225_20H9_LC | . | . | . | | . | . | . | . | . | . | . | . | . | . | . | . | . | S | . | . | . | . | . | . | . | . |
| 21-225_211A6_LC | . | . | . | | . | . | . | . | . | . | . | . | . | . | . | . | . | S | . | . | . | . | . | . | . | . |
| 21-225_213B3_LC | . | . | . | | . | . | . | . | . | . | . | . | . | . | . | . | . | . | . | . | . | . | . | . | . | . |
| 21-225_214C3_LC | . | . | . | | . | . | . | . | . | . | . | . | . | . | . | . | . | . | . | . | . | . | . | . | . | . |
| 21-225_214E4_LC | . | . | . | | . | . | . | . | . | . | . | . | . | . | . | . | . | . | . | . | . | . | . | . | . | . |
| 21-225_223D11_LC | . | . | . | | . | . | . | . | . | . | . | . | . | . | . | . | . | . | . | . | . | . | . | . | . | . |
| 21-225_22B12_LC | . | . | . | | . | . | . | . | . | . | . | . | . | . | . | . | . | . | . | . | . | I | . | . | . | . |
| 21-225_23A11_LC | . | . | . | | . | . | . | . | . | . | . | . | Y | . | . | . | . | . | . | . | . | . | . | . | . | . |
| 21-225_23C7_LC | . | . | . | | . | . | . | . | . | . | . | . | . | . | . | . | . | . | . | . | . | . | . | . | . | . |
| 21-225_23E7_LC | . | . | . | | . | . | . | . | . | . | . | . | . | . | . | . | . | . | . | . | . | . | . | . | . | . |
| 21-225_23G1_LC | . | . | . | | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_23G8_LC | | | | | | | | | | | | | | | | | | | | | | | | | | |
| 21-225_25C10_LC | | | | | | | | | | | | | | | | | | | | | | | | | | |
| 21-225_26G1_LC | | | | | | | | | | | | | | | | | | | | | | | | | | |
| 21-225_27H12_LC | | | | | | | | | | | | | | | | | | | | | | | | | | |
| 21-225_2A4_LC | | | | | | | | | | | | | | | | | | | | | | | | | | |
| 21-225_30A1_LC | | | | | | | | | | | | | | | | | | | | | | | | | | |
| 21-225_32B3_LC | | | | | | | | | | | | | | I | | | | | | | | | | | | |
| 21-225_33A5_LC | | | | | | | | | | | | | | | | | | | | | | | | | | |
| 21-225_35H8_LC | | | | | | | | | | | | | | | | | | | | | | | | | | |
| 21-225_43A4_LC | | | | | | | | | | | | | | | | | | | | | | | | | | |
| 21-225_45D9_LC | | | | | | | | | | | | | | | | | | | | | | | | | | |
| 21-225_51C5_LC | | | | | | | | | | | | | | | | | | | | | | | | | | |
| 21-225_51D5_LC | | | | | | | | | | | | | | | | | | | | | | | | | | |
| 21-225_52E3_LC | | | | | | | | | | | | | | | | | | | | | | | | | | |
| 21-225_53E4_LC | | | | | | | | | | | | | | | | | | | | | | | | | | |
| 21-225_56F7_LC | | | L | | | | | | | | | | | | | | | | | | | | | | | |
| 21-225_58H1_LC | | | | | | | | | | | | | | | | | | | | | | | | | | |
| 21-225_5A5_LC | | | | | | | | | | | | | | | | | | | | | | | | | | |
| 21-225_5E11_LC | V | | | | | | | | | | | | | | | | | | | | | | | | | |
| 21-225_60A4_LC | | | | | | | | | | | | | | | | | | | | | | | | | | |
| 21-225_60F7_LC | | | | | | | | | | | | | | | | | | | | | | S | | | | |
| 21-225_63H8_LC | | | | | | | | | | | | | | | | | | | | | | | | | | |
| 21-225_65D5_LC | | | | | | | | | | | | | | | | | | | | | | | | | | |
| 21-225_66H11_LC | | | | | | | S | | | | | S | | S | | | | | | | | | | S | | |
| 21-225_68E6_LC | | | | | | | | | | | | | | | | | | | | | | | | | | |
| 21-225_7B1_LC | | | | | | S | | | | | | | | | | | | | | | | | | | | |
| 21-225_96C6_LC | | | | | | | | | | | | | | | | | | | | | | | | | | |
| 21-225_9C8_LC | | | | | | | | | | | | | | | | | | | | | | | | | | |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_148B6_LC | . | . | . | . | . | . | . | . | . | . | . | . | X | . | . | . | . | . | . | . | . | . | . | . | . | . |
| 21-225_14H8_LC | . | . | . | . | . | . | . | . | . | . | . | . | Y | . | . | . | . | . | . | . | . | . | . | . | . | . |
| 21-225_153A1_LC | . | . | . | V | . | . | . | . | . | . | . | . | . | . | . | . | . | . | . | . | . | . | . | . | . | . |
| 21-225_157E4_LC | . | . | . | . | . | . | . | . | . | . | . | . | . | . | . | . | . | . | . | . | . | . | . | X | . | . |
| 21-225_157G5_LC | . | . | . | . | . | N | . | . | . | . | . | . | . | . | . | . | . | . | . | . | . | . | . | . | . | . |
| 21-225_157H1_LC | . | . | D | . | . | . | . | . | . | . | . | . | . | . | . | V | . | . | . | . | X | . | . | . | . | . |
| 21-225_157H10_LC | . | . | . | . | . | T | . | . | . | . | . | . | . | . | . | . | . | . | . | . | . | . | . | . | . | . |
| 21-225_158H12_LC | . | . | . | . | . | N | . | . | . | . | . | . | . | . | . | . | . | . | . | . | . | . | . | . | . | . |
| 21-225_159H11_LC | . | . | . | . | . | . | . | . | . | . | . | . | X | . | . | V | . | . | . | . | . | . | . | . | . | . |
| 21-225_15A2_LC | . | . | . | . | . | . | . | . | . | . | . | . | . | . | . | . | . | . | . | . | . | . | . | . | . | . |
| 21-225_15B1_LC | . | . | . | . | . | . | . | . | . | . | . | . | . | . | . | . | . | . | . | . | . | . | . | . | . | . |
| 21-225_160G1_LC | . | . | D | . | . | N | . | . | . | . | . | . | . | . | . | . | . | . | . | . | . | . | . | . | . | . |
| 21-225_160G3_LC | . | . | D | . | . | N | . | . | . | . | . | . | . | . | . | . | . | . | . | X | . | . | . | . | . | . |
| 21-225_163F1_LC | . | . | D | . | . | . | . | . | . | . | . | . | . | . | . | . | . | . | . | . | . | . | . | . | . | . |
| 21-225_167D12_LC | . | . | . | . | . | N | . | . | . | . | . | . | . | . | . | . | . | . | . | . | . | . | . | . | . | . |
| 21-225_169D10_LC | . | . | D | . | . | . | . | . | . | . | . | . | . | . | . | . | . | . | . | . | . | . | . | . | . | . |
| 21-225_16B8_LC | . | . | . | . | . | N | . | . | . | . | . | . | . | . | . | . | . | . | . | . | . | . | . | . | . | L |
| 21-225_16C7_LC | . | . | . | . | . | . | . | . | . | . | . | . | . | . | . | . | . | . | . | . | . | . | . | . | . | . |
| 21-225_16G12_LC | . | . | D | . | . | . | . | . | . | . | . | . | . | . | . | . | . | . | . | . | . | . | . | . | . | . |
| 21-225_170E1_LC | . | . | . | . | . | . | . | . | . | . | . | . | . | . | . | . | . | . | . | . | . | . | . | . | . | . |
| 21-225_175C4_LC | . | . | . | . | . | G | . | . | . | . | . | . | . | . | . | . | . | . | . | . | . | . | . | . | . | . |
| 21-225_177D3_LC | . | . | . | . | . | T | . | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . | . | . | . | . | . | . | . |
| 21-225_17H6_LC | . | X | D | . | . | . | . | . | . | . | . | . | . | . | . | . | . | . | . | . | . | . | . | . | . | . |
| 21-225_181G2_LC | . | . | . | . | . | . | . | . | . | . | . | . | . | . | . | . | . | I | . | . | . | . | . | T | . | . |
| 21-225_184E7_LC | . | . | . | . | . | . | . | . | . | . | . | . | . | . | . | . | . | I | . | . | . | . | . | T | . | . |
| 21-225_185A1_LC | . | . | . | . | . | . | . | . | . | . | . | . | . | . | . | . | . | . | . | . | T | . | . | . | . | . |
| 21-225_185E10_LC | . | . | . | . | . | . | . | . | . | . | . | . | . | . | . | . | . | I | . | . | . | . | . | T | . | . |
| 21-225_188B9_LC | . | . | . | . | . | . | . | . | . | . | . | . | . | . | . | . | . | L | . | . | . | . | . | T | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_188H5_LC | . | . | . | . | . | . | . | . | . | . | . | . | . | . | . | . | . | L | . | . | . | . | . | T | . | . |
| 21-225_18D11_LC | . | . | . | . | . | . | . | . | . | . | . | . | Y | . | . | . | . | . | . | . | . | . | . | . | . | . |
| 21-225_18G10_LC | . | . | . | D | . | . | . | . | . | . | . | . | Y | . | . | . | . | . | . | . | . | . | . | . | . | . |
| 21-225_190A5_LC | . | . | . | . | . | G | . | . | . | . | . | . | . | H | . | . | . | . | . | . | . | . | . | . | . | . |
| 21-225_190B1_LC | . | . | . | . | . | P | . | . | . | . | . | . | . | . | . | . | . | . | . | . | . | . | . | . | . | . |
| 21-225_190F12_LC | . | . | . | . | . | G | . | . | . | . | . | . | K | . | . | . | . | . | . | . | . | . | . | . | . | . |
| 21-225_190H9_LC | . | . | . | . | . | G | . | . | . | . | . | . | K | . | . | . | . | . | . | . | . | . | . | . | . | L |
| 21-225_192A2_LC | . | . | . | . | . | N | . | . | . | . | . | . | . | . | . | . | . | . | . | . | . | . | . | . | . | . |
| 21-225_192C10_LC | . | . | . | . | . | G | . | . | . | . | . | . | K | . | . | . | . | . | . | . | . | . | . | . | . | . |
| 21-225_192E4_LC | . | . | . | . | . | G | . | . | . | . | . | . | . | . | . | . | . | . | . | . | . | . | . | . | . | . |
| 21-225_192H6_LC | . | . | . | . | . | G | . | . | . | . | . | . | . | H | . | . | . | . | . | . | . | . | . | . | . | . |
| 21-225_193F8_LC | . | . | . | . | V | . | . | . | . | . | . | . | . | H | . | . | . | . | . | . | . | . | . | R | . | . |
| 21-225_194H12_LC | . | . | . | . | . | G | . | . | . | . | . | . | K | . | . | . | . | . | . | . | . | . | . | . | . | L |
| 21-225_197E11_LC | . | . | . | . | . | N | . | . | . | . | . | . | K | . | . | . | S | . | . | . | . | . | . | . | . | . |
| 21-225_20B9_LC | . | . | . | . | . | . | . | . | . | . | . | . | . | . | . | . | . | . | . | . | . | . | . | . | . | . |
| 21-225_20C11_LC | . | . | . | D | . | . | . | . | . | . | . | . | . | . | . | . | . | . | . | . | . | . | . | . | . | . |
| 21-225_20F10_LC | . | . | . | . | . | N | . | . | . | . | . | . | . | . | . | . | . | . | . | . | . | . | . | . | . | . |
| 21-225_20H7_LC | . | . | . | . | . | N | . | . | . | . | . | . | . | . | . | . | V | . | . | . | . | . | . | . | . | . |
| 21-225_20H9_LC | . | . | . | . | . | . | . | . | . | . | . | . | . | . | . | . | . | . | . | . | . | . | . | . | . | . |
| 21-225_211A6_LC | . | . | . | . | . | G | . | . | . | . | . | . | K | . | . | . | . | . | . | . | . | . | . | . | . | . |
| 21-225_213B3_LC | . | . | . | . | . | G | . | . | . | . | . | . | K | . | . | . | . | . | . | . | . | . | . | . | . | . |
| 21-225_214C3_LC | . | . | . | . | . | G | . | . | . | . | . | . | . | . | . | . | . | . | . | . | . | . | . | . | V | . |
| 21-225_214E4_LC | . | . | . | . | . | . | . | . | . | . | . | . | . | . | . | . | . | . | . | . | . | . | . | . | . | . |
| 21-225_223D11_LC | . | . | . | . | . | . | . | . | . | . | . | . | . | . | . | . | . | . | . | . | . | . | . | . | . | . |
| 21-225_22B12_LC | . | . | . | D | . | . | . | . | . | . | . | . | . | . | . | . | . | . | . | . | E | . | . | . | . | K |
| 21-225_23A11_LC | . | . | . | D | . | . | . | . | . | . | . | . | . | . | . | . | . | . | . | . | . | . | . | . | . | . |
| 21-225_23C7_LC | . | . | . | . | . | G | . | . | . | . | . | . | I | . | . | . | . | . | . | . | . | . | . | . | . | . |
| 21-225_23E7_LC | . | . | . | . | . | . | . | . | . | . | . | . | . | . | . | . | . | . | . | . | . | I | . | . | . | . |
| 21-225_23G1_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_23G8_LC | . | . | . | . | . | X | . | . | . | . | . | . | . | . | . | . | . | . | . | . | . | . | . | . | . | . |
| 21-225_25C10_LC | . | . | . | . | . | . | . | . | . | . | . | . | . | . | . | . | . | . | . | . | . | . | . | . | . | . |
| 21-225_26G1_LC | . | . | . | D | . | . | . | . | . | . | . | . | . | . | . | . | . | . | . | . | . | . | . | . | . | . |
| 21-225_27H12_LC | . | . | . | . | . | . | . | . | . | . | . | . | . | . | . | . | . | . | . | . | . | . | . | . | . | . |
| 21-225_2A4_LC | . | . | . | . | . | . | . | . | . | . | . | . | . | . | . | . | . | . | . | . | . | . | . | . | . | . |
| 21-225_30A1_LC | . | . | . | . | . | . | . | . | . | . | . | . | . | . | . | . | . | . | . | . | . | . | . | . | . | . |
| 21-225_32B3_LC | . | . | . | . | . | . | . | . | . | . | . | . | . | . | . | . | . | . | . | . | . | . | . | . | . | . |
| 21-225_33A5_LC | . | . | . | . | . | . | . | . | . | . | . | . | . | . | . | . | . | . | . | . | . | . | . | . | . | . |
| 21-225_35H8_LC | . | . | . | A | . | . | . | . | . | . | . | . | . | . | . | . | . | . | . | . | . | . | . | . | . | . |
| 21-225_43A4_LC | . | . | . | . | . | N | . | . | . | . | . | . | . | . | . | . | . | . | . | . | . | . | . | . | . | . |
| 21-225_45D9_LC | . | . | . | . | . | N | . | . | . | . | . | . | . | . | . | . | . | . | . | . | . | . | . | . | . | . |
| 21-225_51C5_LC | . | . | . | . | . | . | . | . | . | . | . | . | . | . | . | . | . | . | . | . | . | . | . | S | . | . |
| 21-225_51D5_LC | . | . | . | . | . | N | . | . | . | . | . | . | . | . | . | . | . | . | . | . | . | . | . | S | . | . |
| 21-225_52E3_LC | . | . | . | . | . | . | . | . | . | . | . | . | . | . | . | . | . | . | . | . | . | . | . | . | . | . |
| 21-225_53E4_LC | . | . | . | V | . | . | . | . | . | . | . | . | . | . | . | . | . | . | . | . | . | . | . | . | . | . |
| 21-225_56F7_LC | . | . | . | . | . | . | . | . | . | . | . | . | T | . | . | . | . | . | . | . | T | . | . | T | . | . |
| 21-225_56H1_LC | . | . | . | . | . | . | . | . | . | . | . | . | X | . | . | . | . | . | . | . | . | . | . | . | . | . |
| 21-225_5A5_LC | . | . | . | . | . | . | . | . | . | . | . | . | . | . | . | . | . | . | . | . | . | . | . | . | . | . |
| 21-225_5E11_LC | . | . | . | . | . | . | . | . | . | . | . | . | . | . | . | . | . | . | . | . | . | . | . | . | . | . |
| 21-225_60A4_LC | . | . | . | . | . | . | . | . | . | . | . | . | . | . | . | . | . | . | . | . | . | . | . | . | . | . |
| 21-225_60F7_LC | . | . | . | V | . | X | . | . | . | . | . | . | . | . | . | V | . | V | . | . | . | . | . | . | . | X |
| 21-225_63H8_LC | . | . | . | . | . | . | . | . | . | . | . | . | . | . | . | . | . | . | . | . | . | . | . | . | . | X |
| 21-225_65D5_LC | . | . | . | D | . | N | . | . | . | . | . | . | . | . | . | . | . | . | . | . | . | . | . | . | . | . |
| 21-225_66H11_LC | . | . | . | . | . | . | . | . | . | . | . | . | . | . | . | . | . | . | . | . | . | . | . | . | . | . |
| 21-225_68E6_LC | . | . | . | . | . | . | . | . | . | . | . | . | Y | . | . | . | . | . | . | . | . | . | . | X | . | . |
| 21-225_7B1_LC | . | . | . | D | . | . | . | . | . | . | . | . | . | X | . | V | . | . | . | . | . | . | . | . | . | . |
| 21-225_96C6_LC | . | . | . | . | . | . | . | . | . | . | . | . | . | . | . | . | . | . | . | . | . | . | . | . | . | . |
| 21-225_9C8_LC | . | . | . | . | . | . | . | . | . | . | . | . | Y | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
|  |  |  |  |  |  |  |  |  |  |  |  | K_CDR2 |  |  |  |  |  |  |  |  |  |  |  |  |  |  |
| CONSENSUS | K | S | L | I | Y | A | - | - | - | - | - | - | - | - | A | S | S | L | Q | S | G | V | P | S | K | F |
| 21-225_148B6_LC | . | . | . | . | S | . | . | . | . | . | . | . | . | . | . | . | . | . | . | . | . | . | . | . | . | . |
| 21-225_14H8_LC | . | . | . | . | . | V | . | . | . | . | . | . | . | . | . | . | . | . | . | . | . | . | . | . | . | . |
| 21-225_153A1_LC | . | . | . | . | S | . | . | . | . | . | . | . | . | . | . | . | . | . | . | . | . | . | . | . | N | . |
| 21-225_157E4_LC | . | . | . | . | . | . | . | . | . | . | . | . | . | . | . | . | . | . | . | . | . | . | . | . | Q | . |
| 21-225_157G5_LC | S | . | . | . | . | . | . | . | . | . | . | . | . | . | . | . | . | . | . | . | . | . | . | . | Q | . |
| 21-225_157H1_LC | X | . | . | . | . | . | . | . | . | . | . | . | . | . | . | . | . | . | . | . | . | . | . | . | . | . |
| 21-225_157H10_LC | . | . | . | . | . | T | . | . | . | . | . | . | . | . | . | . | N | . | . | . | . | . | . | . | . | . |
| 21-225_158H12_LC | . | . | . | . | S | . | . | . | . | . | . | . | . | . | . | . | . | . | . | . | . | . | . | . | . | . |
| 21-225_159H11_LC | . | . | . | . | . | . | . | . | . | . | . | . | . | . | . | . | . | . | . | . | . | . | . | . | . | . |
| 21-225_15A2_LC | . | . | . | . | S | . | . | . | . | . | . | . | . | . | . | . | . | . | . | . | . | . | . | . | . | . |
| 21-225_15B1_LC | . | . | . | . | S | . | . | . | . | . | . | . | . | . | . | . | . | . | . | . | . | . | . | . | N | . |
| 21-225_160G1_LC | T | . | . | . | . | . | . | . | . | . | . | . | . | . | S | . | . | . | . | . | . | . | . | . | . | . |
| 21-225_160G3_LC | T | . | . | . | . | . | . | . | . | . | . | . | . | . | S | . | . | . | . | . | . | . | . | . | . | . |
| 21-225_163F1_LC | . | . | . | . | . | . | . | . | . | . | . | . | . | . | . | . | . | . | . | . | . | . | . | . | . | . |
| 21-225_167D12_LC | S | . | . | . | . | . | . | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . | . | . | . | . | . | . | . |
| 21-225_16B8_LC | . | . | . | . | N | . | . | . | . | . | . | . | . | . | . | . | . | . | V | . | . | . | . | . | . | . |
| 21-225_16C7_LC | . | . | . | . | S | . | . | . | . | . | . | . | . | . | . | . | . | . | . | . | . | . | . | . | . | . |
| 21-225_16G12_LC | . | . | . | . | . | . | . | . | . | . | . | . | . | . | . | . | . | . | . | . | . | . | . | . | . | . |
| 21-225_170E1_LC | . | . | . | . | . | . | . | . | . | . | . | . | . | . | . | . | . | . | . | . | . | . | . | . | . | . |
| 21-225_175C4_LC | . | . | . | . | . | . | . | . | . | . | . | . | . | . | . | . | S | . | . | . | . | P | . | . | . | . |
| 21-225_177D3_LC | . | . | . | . | . | . | . | . | . | . | . | . | . | . | . | . | . | . | . | . | . | . | . | . | . | . |
| 21-225_17H5_LC | . | . | . | . | N | . | . | . | . | . | . | . | . | . | . | . | . | . | . | . | . | . | . | . | . | . |
| 21-225_17H6_LC | . | . | . | . | S | T | . | . | . | . | . | . | . | . | . | . | . | . | . | . | . | . | . | . | . | . |
| 21-225_181G2_LC | . | . | . | . | . | . | . | . | . | . | . | . | . | . | . | . | . | . | . | . | . | . | S | . | S | . |
| 21-225_184E7_LC | . | . | . | . | . | . | . | . | . | . | . | . | . | . | . | . | . | . | . | . | . | . | S | . | S | . |
| 21-225_185A1_LC | . | . | . | . | S | V | . | . | . | . | . | . | . | . | . | . | . | . | . | . | . | . | . | . | S | . |
| 21-225_185E10_LC | . | . | . | . | . | . | . | . | . | . | . | . | . | . | . | . | . | . | . | . | . | . | S | . | S | . |
| 21-225_188B9_LC | . | . | . | . | . | . | . | . | . | . | . | . | . | . | . | . | . | . | . | . | . | . | S | . | S | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | K_CDR2 | | | | | | | | | | | | | | | |
| CONSENSUS | K | S | L | I | Y | A | - | - | - | - | - | - | - | - | A | S | S | L | Q | S | G | V | P | S | K | F |
| 21-225_188H5_LC | . | . | . | . | . | . | . | . | . | . | . | . | . | . | . | . | . | . | . | . | . | . | S | . | . | . |
| 21-225_18D11_LC | . | . | . | . | . | V | . | . | . | . | . | . | . | . | . | . | . | . | . | . | . | . | . | . | . | . |
| 21-225_18G10_LC | . | . | . | . | . | V | . | . | . | . | . | . | . | . | . | . | . | . | . | . | . | . | . | . | . | . |
| 21-225_190A5_LC | . | . | . | . | . | . | . | . | . | . | . | . | . | . | . | . | . | . | . | . | . | . | . | . | . | . |
| 21-225_190B1_LC | . | . | . | . | . | V | . | . | . | . | . | . | . | . | . | . | . | . | . | . | . | . | . | . | . | . |
| 21-225_190F12_LC | . | . | . | . | . | . | . | . | . | . | . | . | . | . | . | . | . | . | . | . | . | . | . | . | . | . |
| 21-225_190H9_LC | . | . | . | . | . | . | . | . | . | . | . | . | . | . | . | . | . | . | . | . | . | . | . | . | . | . |
| 21-225_192A2_LC | . | . | . | . | . | . | . | . | . | . | . | . | . | . | . | . | . | . | . | . | . | . | . | . | . | . |
| 21-225_192C10_LC | . | . | . | . | . | . | . | . | . | . | . | . | . | . | . | . | . | . | . | . | . | . | . | . | . | . |
| 21-225_192E4_LC | . | . | . | . | . | V | . | . | . | . | . | . | . | . | V | . | . | . | . | . | . | . | . | . | . | . |
| 21-225_192H6_LC | . | . | . | . | . | . | . | . | . | . | . | . | . | . | . | . | . | . | . | . | . | . | . | . | . | . |
| 21-225_193F8_LC | . | . | . | . | . | . | . | . | . | . | . | . | . | . | . | . | . | . | . | . | . | . | . | . | . | . |
| 21-225_194H12_LC | . | . | . | . | . | . | . | . | . | . | . | . | . | . | . | . | . | . | . | . | . | . | . | . | . | . |
| 21-225_197E11_LC | . | . | . | . | . | . | . | . | . | . | . | . | . | . | . | . | . | V | . | . | . | . | . | . | . | . |
| 21-225_20B9_LC | . | . | . | . | . | V | . | . | . | . | . | . | . | . | . | . | . | . | . | . | . | . | . | . | . | . |
| 21-225_20C11_LC | . | . | . | . | . | . | . | . | . | . | . | . | . | . | . | . | . | . | . | . | . | . | . | . | . | . |
| 21-225_20F10_LC | . | . | . | . | . | . | . | . | . | . | . | . | . | . | . | . | . | . | . | . | . | . | . | . | . | . |
| 21-225_20H7_LC | . | . | . | . | . | . | . | . | . | . | . | . | . | . | . | . | . | . | . | . | . | . | . | . | . | . |
| 21-225_20H9_LC | . | . | . | . | S | . | . | . | . | . | . | . | . | . | . | . | . | . | . | . | . | . | . | . | . | . |
| 21-225_211A6_LC | . | . | . | . | . | . | . | . | . | . | . | . | . | . | . | . | . | . | L | . | . | . | . | . | . | . |
| 21-225_213B3_LC | . | . | . | . | S | . | . | . | . | . | . | . | . | . | . | . | . | V | L | . | . | . | . | . | . | . |
| 21-225_214C3_LC | . | . | . | . | . | . | . | . | . | . | . | . | . | . | . | . | . | . | . | . | . | . | . | . | . | . |
| 21-225_214E4_LC | . | . | . | . | . | . | . | . | . | . | . | . | . | . | . | . | . | . | . | . | . | . | . | . | . | . |
| 21-225_223D11_LC | . | . | . | . | . | . | . | . | . | . | . | . | . | . | . | . | T | . | . | . | . | . | . | . | . | . |
| 21-225_22B12_LC | . | . | . | . | Q | . | . | . | . | . | . | . | . | . | . | . | . | . | . | . | . | . | . | . | . | . |
| 21-225_23A11_LC | . | . | . | . | . | . | . | . | . | . | . | . | . | . | . | . | T | . | . | . | . | . | . | . | . | . |
| 21-225_23C7_LC | . | . | . | . | S | . | . | . | . | . | . | . | . | . | . | . | . | . | . | . | . | . | . | . | . | . |
| 21-225_23E7_LC | . | . | . | . | . | . | . | . | . | . | . | . | . | . | . | . | . | . | . | . | . | . | . | . | . | . |
| 21-225_23G1_LC | . | . | . | . | S | . | . | . | . | . | . | . | . | . | . | . | . | . | . | . | . | . | . | . | Q | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | S | L | I | Y | A | - | - | - | - | - | - | - | - | A | S | S | L | Q | S | G | V | P | S | K | F |
| 21-225_23G8_LC | . | . | . | . | . | . | | | . | . | . | . | . | . | . | . | . | . | . | . | . | . | . | . | N | . |
| 21-225_25C10_LC | . | . | . | . | . | . | | | . | . | . | . | . | . | . | . | . | . | . | . | . | F | . | . | . | . |
| 21-225_26G1_LC | . | . | . | . | . | . | | | . | . | . | . | . | . | . | . | . | . | R | . | . | . | . | . | N | . |
| 21-225_27H12_LC | . | . | . | . | N | G | | | . | . | . | . | . | . | . | . | . | . | . | . | . | . | . | . | . | . |
| 21-225_2A4_LC | . | R | . | . | . | T | | | . | . | . | . | . | . | . | . | . | . | . | . | . | . | . | . | . | . |
| 21-225_30A1_LC | . | . | . | . | S | . | | | . | . | . | . | . | . | . | . | . | . | . | . | T | . | . | . | . | . |
| 21-225_32B3_LC | R | . | . | . | . | . | | | . | . | . | . | . | . | . | . | . | . | . | . | . | . | . | . | T | . |
| 21-225_33A5_LC | . | . | . | . | S | . | | | . | . | . | . | . | . | . | . | . | . | . | . | . | . | . | . | . | . |
| 21-225_35H8_LC | . | . | . | . | . | G | | | . | . | . | . | . | . | . | . | G | . | . | . | . | . | . | . | . | . |
| 21-225_43A4_LC | . | . | . | . | N | . | | | . | . | . | . | . | . | . | . | . | . | . | . | . | . | . | . | R | . |
| 21-225_45D9_LC | . | . | . | . | N | . | | | . | . | . | . | . | . | . | . | . | . | . | . | . | . | . | . | R | . |
| 21-225_51C5_LC | . | . | . | . | . | . | | | . | . | . | . | . | . | . | . | . | . | R | . | . | . | . | . | Q | . |
| 21-225_51D5_LC | . | . | . | . | . | . | | | . | . | . | . | . | . | . | . | . | . | R | . | . | . | . | . | Q | . |
| 21-225_52E3_LC | . | . | . | . | N | . | | | . | . | . | . | . | . | . | . | . | . | . | . | . | . | . | . | . | . |
| 21-225_53E4_LC | . | . | . | . | S | . | | | . | . | . | . | . | . | . | . | . | . | . | . | . | . | . | . | . | . |
| 21-225_56F7_LC | . | . | . | . | . | . | | | . | . | . | . | . | . | . | . | . | . | . | G | . | . | . | . | . | . |
| 21-225_56H1_LC | Q | . | . | M | S | . | | | . | . | . | . | . | . | . | . | G | . | . | . | . | . | . | . | . | . |
| 21-225_5A5_LC | . | . | . | . | S | . | | | . | . | . | . | . | . | . | . | . | . | . | . | . | . | . | . | . | . |
| 21-225_5E11_LC | . | . | . | . | N | . | | | . | . | . | . | . | . | . | . | . | V | . | . | . | . | . | . | . | . |
| 21-225_60A4_LC | . | . | . | . | . | . | | | . | . | . | . | . | . | . | . | . | . | . | . | . | . | . | . | . | . |
| 21-225_60F7_LC | . | . | . | . | . | . | | | . | . | . | . | . | . | . | . | . | . | . | . | . | . | . | . | T | . |
| 21-225_63H8_LC | . | . | . | . | . | . | | | . | . | . | . | . | . | . | . | . | . | . | . | . | . | . | . | . | . |
| 21-225_65D5_LC | R | . | . | . | . | . | | | . | . | . | . | . | . | . | . | . | . | R | . | . | . | . | . | Q | . |
| 21-225_66H11_LC | . | . | . | . | . | G | | | . | . | . | . | . | . | F | N | . | . | R | . | . | . | . | . | . | . |
| 21-225_68E6_LC | . | . | . | . | . | V | | | . | . | . | . | . | . | . | . | . | . | . | . | . | . | . | . | R | . |
| 21-225_7B1_LC | . | . | . | . | F | . | | | . | . | . | . | . | . | . | . | . | . | . | . | . | . | . | . | Q | . |
| 21-225_96C6_LC | . | . | . | . | . | . | | | . | . | . | . | . | . | . | . | . | . | . | . | . | . | . | . | . | . |
| 21-225_9C8_LC | . | . | . | . | . | V | | | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_148B6_LC | . | . | . | . | . | . | . | . | . | . | . | . | . | . | . | . | . | . | . | . | . | . | . | . | . | . |
| 21-225_14H8_LC | . | . | . | . | . | . | . | . | . | . | . | . | . | . | . | . | . | . | . | . | . | . | . | . | . | . |
| 21-225_153A1_LC | . | . | . | . | . | . | . | . | . | . | . | . | . | . | . | . | . | . | . | . | . | . | . | . | . | . |
| 21-225_157E4_LC | . | . | . | . | . | . | . | . | . | . | . | . | . | . | . | N | . | . | . | . | . | . | . | . | . | . |
| 21-225_157G5_LC | . | . | . | . | . | . | . | . | . | . | . | . | . | . | . | . | . | . | . | . | . | . | . | . | . | . |
| 21-225_157H1_LC | . | . | . | . | . | . | . | . | . | . | . | . | . | . | . | . | . | . | . | . | . | . | . | . | . | . |
| 21-225_157H10_LC | . | . | . | . | . | . | . | . | . | . | . | . | . | . | . | . | . | . | . | . | . | . | . | . | . | . |
| 21-225_158H12_LC | . | . | . | . | . | . | . | . | . | . | . | . | . | S | . | . | . | . | . | . | . | . | . | . | . | . |
| 21-225_159H11_LC | . | . | . | . | . | . | . | . | . | . | . | . | . | . | . | . | N | . | . | . | . | . | . | . | . | . |
| 21-225_15A2_LC | . | . | . | . | P | . | . | . | . | . | . | . | P | P | . | . | . | . | . | . | . | . | . | . | N | . |
| 21-225_15B1_LC | . | . | . | . | . | . | . | . | . | . | . | . | . | . | . | . | . | . | . | . | . | . | . | . | . | . |
| 21-225_160G1_LC | . | . | . | . | . | . | . | . | . | . | . | . | . | . | . | . | . | . | . | . | . | . | . | . | . | . |
| 21-225_160G3_LC | . | . | . | . | . | . | . | . | . | . | . | . | . | . | . | . | . | . | . | . | . | . | . | . | . | . |
| 21-225_163F1_LC | . | . | . | . | . | . | . | . | . | . | . | . | . | . | . | . | . | . | . | . | . | . | . | . | A | . |
| 21-225_167D12_LC | . | . | . | . | . | . | . | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . | . | . | . | . | . | . | . |
| 21-225_16B8_LC | . | . | . | . | . | . | . | . | . | . | . | . | . | . | . | . | . | . | . | . | . | . | . | . | . | . |
| 21-225_16C7_LC | . | . | . | . | . | . | . | . | . | . | . | . | . | . | . | . | . | . | . | . | . | . | . | . | . | . |
| 21-225_16G12_LC | R | . | . | . | . | . | . | . | . | . | . | . | . | . | . | N | . | . | . | . | . | . | . | . | S | . |
| 21-225_170E1_LC | . | . | . | . | . | . | . | . | . | . | . | . | . | . | . | . | . | . | . | . | . | . | . | . | . | . |
| 21-225_175C4_LC | . | . | . | . | . | . | . | . | . | . | . | . | . | . | . | . | . | . | . | . | . | . | . | . | . | . |
| 21-225_177D3_LC | . | . | . | . | . | . | . | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . | . | . | . | . | . | . | . |
| 21-225_17H6_LC | . | . | . | . | . | . | . | . | . | . | . | . | . | . | . | . | . | . | . | . | . | . | . | . | I | . |
| 21-225_181G2_LC | . | . | . | . | P | . | . | . | . | . | . | . | . | . | . | . | . | . | . | . | . | . | . | . | . | . |
| 21-225_184E7_LC | . | . | . | . | P | . | . | . | . | . | . | . | I | . | . | . | . | V | . | R | . | . | . | . | . | . |
| 21-225_185A1_LC | A | . | . | . | . | . | . | . | . | . | . | . | . | . | . | . | . | . | . | . | . | . | . | . | . | . |
| 21-225_185E10_LC | . | . | . | . | P | . | . | . | . | . | . | . | . | . | . | . | . | . | . | . | . | . | . | . | . | . |
| 21-225_188B9_LC | . | . | . | . | P | . | . | . | . | . | . | . | . | . | . | . | . | . | . | . | . | . | . | . | . | . |

## FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| **K_FR3** | | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_188H5_LC | . | . | . | . | S | . | . | . | . | . | . | . | . | . | . | . | . | . | . | . | . | . | . | . | . | . |
| 21-225_18D11_LC | . | . | . | . | S | . | . | . | . | . | . | . | . | . | . | . | . | . | . | . | . | . | . | . | . | . |
| 21-225_18G10_LC | G | . | . | . | S | . | . | . | . | . | . | . | . | . | . | . | . | . | . | . | . | . | . | . | . | . |
| 21-225_190A5_LC | . | . | . | . | . | . | . | . | . | . | . | . | . | . | . | . | . | . | . | . | . | . | . | . | . | . |
| 21-225_190B1_LC | . | . | . | . | . | . | . | . | . | H | . | . | . | . | . | . | . | . | . | . | . | . | . | G | . | . |
| 21-225_190F12_LC | . | . | . | . | . | . | . | . | . | . | . | . | . | . | . | . | . | . | . | D | . | . | . | . | . | . |
| 21-225_190H9_LC | . | . | . | . | . | . | . | . | . | . | . | . | . | . | . | . | . | . | . | D | . | . | . | . | . | . |
| 21-225_192A2_LC | . | . | . | . | . | . | . | . | . | . | . | . | . | . | . | . | . | . | . | . | . | . | . | . | . | . |
| 21-225_192C10_LC | . | . | . | . | . | . | . | . | . | . | . | . | . | . | . | . | . | . | . | D | . | . | . | . | . | . |
| 21-225_192E4_LC | . | . | . | . | . | . | . | . | . | . | . | . | . | . | . | . | . | . | . | . | . | . | . | . | . | . |
| 21-225_192H6_LC | . | . | R | . | . | . | . | . | . | . | . | S | . | . | . | . | . | . | . | . | . | . | . | . | . | . |
| 21-225_193F8_LC | . | . | . | . | . | . | . | A | . | . | . | . | . | . | . | . | . | . | . | . | . | . | . | . | . | . |
| 21-225_194H12_LC | . | . | . | . | . | . | . | . | . | . | . | . | . | . | . | . | . | . | . | D | . | . | . | . | . | . |
| 21-225_197E11_LC | . | . | . | . | . | . | . | . | . | . | . | . | . | . | . | . | . | . | . | D | . | . | . | . | . | . |
| 21-225_20B9_LC | . | . | . | . | . | . | . | . | . | . | . | . | . | . | . | . | . | . | . | . | . | . | . | . | . | . |
| 21-225_20C11_LC | R | . | . | . | . | . | . | . | . | . | . | . | . | . | . | . | . | . | . | . | . | . | . | . | . | . |
| 21-225_20F10_LC | . | . | . | . | S | . | . | . | . | . | . | . | . | . | . | . | . | . | . | . | . | . | . | . | . | S |
| 21-225_20H7_LC | . | . | . | . | . | . | . | . | . | . | . | . | . | . | . | . | . | . | . | . | . | . | . | . | . | . |
| 21-225_20H9_LC | . | . | . | . | . | . | . | . | . | . | . | . | . | . | . | . | . | . | . | . | . | . | . | . | . | . |
| 21-225_211A6_LC | . | . | . | . | . | . | . | . | . | . | . | . | . | . | . | . | . | . | . | . | . | . | . | . | . | . |
| 21-225_213B3_LC | . | . | . | . | . | . | . | . | . | . | . | . | . | . | . | . | . | . | . | . | . | . | . | . | . | . |
| 21-225_214C3_LC | . | . | . | . | . | . | . | . | . | . | . | . | . | . | . | . | . | . | . | . | . | . | . | T | . | . |
| 21-225_214E4_LC | . | . | . | . | . | . | . | . | . | . | . | . | . | . | . | . | . | . | . | . | . | . | . | . | . | . |
| 21-225_223D11_LC | . | . | R | . | . | . | . | . | . | . | N | . | . | . | . | N | . | . | . | . | . | . | . | . | . | . |
| 21-225_22B12_LC | . | . | . | . | . | . | . | . | . | . | N | . | . | . | . | . | . | . | . | . | . | . | L | . | . | . |
| 21-225_23A11_LC | . | . | . | . | . | . | . | . | . | . | . | . | . | . | . | . | . | . | . | . | . | . | . | . | . | . |
| 21-225_23C7_LC | . | . | . | . | . | . | . | . | . | H | . | . | . | . | . | . | . | . | . | . | . | . | . | . | . | . |
| 21-225_23E7_LC | . | . | . | . | . | . | . | . | . | . | . | . | . | . | . | . | . | . | . | . | . | . | . | . | . | . |
| 21-225_23G1_LC | . | . | . | . | S | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_23G8_LC | . | . | . | . | . | . | | | . | . | . | . | . | . | . | . | . | . | . | . | . | . | . | . | . | . |
| 21-225_25C10_LC | . | . | . | . | . | . | | | . | . | . | . | . | . | . | . | . | . | . | . | . | . | . | . | . | . |
| 21-225_26G1_LC | . | . | . | . | . | . | | | . | . | . | . | . | . | . | . | . | . | . | . | . | N | . | . | . | . |
| 21-225_27H12_LC | . | . | . | . | . | . | | | . | . | . | . | . | . | . | . | . | . | . | . | . | . | . | . | . | . |
| 21-225_2A4_LC | . | . | . | F | . | . | | | . | . | . | . | . | . | . | . | . | . | . | . | . | . | . | . | . | . |
| 21-225_30A1_LC | . | . | . | . | . | . | | | . | . | . | . | . | . | . | . | . | . | . | . | . | . | . | . | . | . |
| 21-225_32B3_LC | . | . | . | . | . | . | | | . | . | . | . | . | . | . | . | . | . | . | . | . | . | . | . | . | . |
| 21-225_33A5_LC | . | . | . | . | . | . | | | . | . | . | . | . | . | . | . | . | . | . | . | . | . | . | . | . | . |
| 21-225_35H8_LC | . | . | . | . | . | . | | | . | . | . | . | . | . | . | . | . | . | . | . | . | . | . | . | . | . |
| 21-225_43A4_LC | . | . | . | . | . | . | | | . | . | . | . | . | N | . | . | . | . | . | . | . | . | . | . | . | . |
| 21-225_45D9_LC | . | . | . | . | . | . | | | . | . | . | . | . | N | . | . | . | . | . | . | . | . | . | . | . | . |
| 21-225_51C5_LC | . | . | . | . | . | . | | | . | . | ? | . | . | . | . | . | . | . | . | . | . | . | . | . | . | . |
| 21-225_51D5_LC | . | . | . | . | . | . | | | . | . | ? | . | . | . | . | . | . | . | . | . | . | . | . | . | . | . |
| 21-225_52E3_LC | . | . | . | . | . | . | | | . | . | . | . | . | . | . | . | . | . | . | . | . | . | . | . | . | . |
| 21-225_53E4_LC | . | . | . | . | . | . | | | . | . | . | . | . | . | . | . | . | . | . | . | . | . | . | . | . | . |
| 21-225_56F7_LC | . | . | . | . | . | . | | | . | . | . | . | . | . | . | . | . | . | . | . | . | . | . | . | . | . |
| 21-225_56H1_LC | . | . | . | . | . | . | | | . | . | . | . | . | . | . | . | . | . | . | . | . | . | . | . | . | . |
| 21-225_5A5_LC | . | . | . | . | . | . | | | . | . | . | . | . | . | . | . | . | . | . | . | . | . | . | . | . | . |
| 21-225_5E11_LC | . | . | N | . | . | . | | | . | . | . | . | . | . | . | . | . | . | . | . | . | . | . | . | . | . |
| 21-225_60A4_LC | . | . | . | . | . | . | | | . | . | . | . | . | . | . | . | . | S | . | . | . | . | . | . | . | . |
| 21-225_60F7_LC | . | . | . | . | . | . | | | . | . | . | . | . | . | . | . | . | . | . | . | . | . | . | . | . | . |
| 21-225_63H8_LC | . | . | . | . | . | . | | | . | . | . | . | . | . | . | . | . | . | . | . | . | . | . | . | . | . |
| 21-225_65D5_LC | . | N | . | . | . | . | | | S | . | . | . | . | . | . | . | . | . | . | . | . | . | . | . | . | . |
| 21-225_66H11_LC | . | . | . | F | . | . | | | . | . | . | . | . | . | . | N | . | . | . | . | . | . | . | . | N | . |
| 21-225_68E6_LC | . | . | . | . | . | . | | | . | . | . | . | . | . | . | . | . | . | . | . | . | . | . | . | ? | . |
| 21-225_7B1_LC | . | . | . | . | . | . | | | . | . | . | . | . | . | . | . | . | ? | . | . | . | . | . | . | . | . |
| 21-225_96C6_LC | . | . | . | . | . | . | | | . | . | . | . | . | . | . | N | . | . | . | . | . | . | . | . | . | . |
| 21-225_9C8_LC | . | . | . | . | . | . | | | . | . | . | . | . | . | . | . | . | . | . | . | . | . | . | . | . | . |

## FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | N | S | - | - | - | - | - | - | - | - | - | - | - | - | - | - | - | - | - | - | - |
| 21-225_148B6_LC | . | . | . | . | . | . | . | . | . | . | . | . | . | . | . | . | . | . | . | . | . | . | . | . | . | |
| 21-225_14H8_LC | . | . | . | . | . | . | . | . | . | . | . | . | . | . | . | . | . | . | . | . | . | . | . | . | . | |
| 21-225_153A1_LC | . | . | . | . | . | N | . | . | . | . | . | . | . | . | . | . | . | . | . | . | . | . | . | . | . | |
| 21-225_157E4_LC | . | . | . | . | . | N | . | . | . | . | . | . | . | . | . | . | . | . | . | . | . | . | . | . | . | |
| 21-225_157G5_LC | . | . | . | . | . | . | . | . | . | . | . | . | . | . | . | . | . | . | . | . | . | . | . | . | . | |
| 21-225_157H1_LC | . | . | . | . | . | N | . | . | . | . | . | . | . | . | . | . | . | . | . | . | . | . | . | . | . | |
| 21-225_157H10_LC | . | . | . | . | . | N | . | . | . | . | . | . | . | . | . | . | . | . | . | . | . | . | . | . | . | |
| 21-225_158H12_LC | . | . | . | . | . | N | . | . | . | . | . | . | . | . | . | . | . | . | . | . | . | . | . | . | . | |
| 21-225_159H11_LC | . | . | . | . | . | . | . | . | . | . | . | . | . | . | . | . | . | . | . | . | . | . | . | . | . | |
| 21-225_15A2_LC | . | . | . | . | . | Y | . | . | . | . | . | . | . | . | . | . | . | . | . | . | . | . | . | . | . | |
| 21-225_15B1_LC | . | . | . | . | . | N | . | . | . | . | . | . | . | . | . | . | . | . | . | . | . | . | . | . | . | |
| 21-225_160G1_LC | . | . | . | . | . | N | . | . | . | . | . | . | . | . | . | . | . | . | . | . | . | . | . | . | . | |
| 21-225_160G3_LC | . | . | . | . | . | N | . | . | . | . | . | . | . | . | . | . | . | . | . | . | . | . | . | . | . | |
| 21-225_163F1_LC | . | . | . | . | . | . | . | . | . | . | . | . | . | . | . | . | . | . | . | . | . | . | . | . | . | |
| 21-225_167D12_LC | N | . | . | . | . | . | . | . | . | . | . | . | . | . | . | . | . | . | . | . | . | . | . | . | . | |
| 21-225_169D10_LC | . | . | . | . | . | N | . | . | . | . | . | . | . | . | . | . | . | . | . | . | . | . | . | . | . | |
| 21-225_16B8_LC | . | . | N | . | . | N | . | . | . | . | . | . | . | . | . | . | . | . | . | . | . | . | . | . | . | |
| 21-225_16C7_LC | . | . | . | . | . | . | . | . | . | . | . | . | . | . | . | . | . | . | . | . | . | . | . | . | . | |
| 21-225_16G12_LC | . | . | . | . | . | N | . | . | . | . | . | . | . | . | . | . | . | . | . | . | . | . | . | . | . | . |
| 21-225_170E1_LC | . | . | . | . | . | N | . | . | . | . | . | . | . | . | . | . | . | . | . | . | . | . | . | . | . | . |
| 21-225_175C4_LC | . | . | . | . | . | Y | . | . | . | . | . | . | . | . | . | . | . | . | . | . | . | . | . | . | . | . |
| 21-225_177D3_LC | . | . | . | . | . | . | . | . | . | . | . | . | . | . | . | . | . | . | . | . | . | . | . | . | . | . |
| 21-225_17H5_LC | . | . | . | . | . | N | . | . | . | . | . | . | . | . | . | . | . | . | . | . | . | . | . | . | . | . |
| 21-225_17H6_LC | . | . | . | . | . | N | . | . | . | . | . | . | . | . | . | . | . | . | . | . | . | . | . | . | . | . |
| 21-225_181G2_LC | . | . | . | . | . | G | . | . | . | . | . | . | . | . | . | . | . | . | . | . | . | . | . | . | . | . |
| 21-225_184E7_LC | . | . | . | . | . | G | . | . | . | . | . | . | . | . | . | . | . | . | . | . | . | . | . | . | . | . |
| 21-225_185A1_LC | . | . | N | . | . | N | . | . | . | . | . | . | . | . | . | . | . | . | . | . | . | . | . | . | . | . |
| 21-225_185E10_LC | . | . | . | . | . | G | . | . | . | . | . | . | . | . | . | . | . | . | . | . | . | . | . | . | . | . |
| 21-225_188B9_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | N | S | - | - | - | - | - | - | - | - | - | - | - | - | - | - | - | - | - | - | - |
| 21-225_188H5_LC | . | . | . | . | . | . | . | . | . | . | . | . | . | . | . | . | . | . | . | . | . | . | . | . | . | . |
| 21-225_18D11_LC | . | . | . | . | . | . | T | . | . | . | . | . | . | . | . | . | . | . | . | . | . | . | . | . | . | . |
| 21-225_18G10_LC | . | . | L | . | . | . | . | . | . | . | . | . | . | . | . | . | . | . | . | . | . | . | . | . | . | . |
| 21-225_190A5_LC | . | . | . | . | . | S | N | . | . | . | . | . | . | . | . | . | . | . | . | . | . | . | . | . | . | . |
| 21-225_190B1_LC | . | . | . | . | . | L | N | . | . | . | . | . | . | . | . | . | . | . | . | . | . | . | . | . | . | . |
| 21-225_190F12_LC | . | . | . | R | . | D | T | . | . | . | . | . | . | . | . | . | . | . | . | . | . | . | . | . | . | . |
| 21-225_190H9_LC | . | . | . | R | . | D | T | . | . | . | . | . | . | . | . | . | . | . | . | . | . | . | . | . | . | . |
| 21-225_192A2_LC | . | . | . | . | . | . | . | . | . | . | . | . | . | . | . | . | . | . | . | . | . | . | . | . | . | . |
| 21-225_192C10_LC | . | . | . | R | . | D | T | . | . | . | . | . | . | . | . | . | . | . | . | . | . | . | . | . | . | . |
| 21-225_192E4_LC | . | . | . | R | . | D | T | . | . | . | . | . | . | . | . | . | . | . | . | . | . | . | . | . | . | . |
| 21-225_192H6_LC | . | . | . | . | . | S | N | . | . | . | . | . | . | . | . | . | . | . | . | . | . | . | . | . | . | . |
| 21-225_193F8_LC | . | . | . | . | . | . | . | . | . | . | . | . | . | . | . | . | . | . | . | . | . | . | . | . | . | . |
| 21-225_194H12_LC | . | . | . | R | . | D | T | . | . | . | . | . | . | . | . | . | . | . | . | . | . | . | . | . | . | . |
| 21-225_197E11_LC | . | G | . | R | . | D | T | . | . | . | . | . | . | . | . | . | . | . | . | . | . | . | . | . | . | . |
| 21-225_20B9_LC | . | . | . | . | . | . | T | . | . | . | . | . | . | . | . | . | . | . | . | . | . | . | . | . | . | . |
| 21-225_20C11_LC | . | . | . | . | . | H | . | . | . | . | . | . | . | . | . | . | . | . | . | . | . | . | . | . | . | . |
| 21-225_20F10_LC | . | . | . | . | . | . | . | . | . | . | . | . | . | . | . | . | . | . | . | . | . | . | . | . | . | . |
| 21-225_20H7_LC | . | . | . | . | . | Y | . | . | . | . | . | . | . | . | . | . | . | . | . | . | . | . | . | . | . | . |
| 21-225_20H9_LC | . | . | . | . | . | . | . | . | . | . | . | . | . | . | . | . | . | . | . | . | . | . | . | . | . | . |
| 21-225_211A6_LC | . | . | . | R | . | D | T | . | . | . | . | . | . | . | . | . | . | . | . | . | . | . | . | . | . | . |
| 21-225_213B3_LC | . | . | . | R | . | D | T | . | . | . | . | . | . | . | . | . | . | . | . | . | . | . | . | . | . | . |
| 21-225_214C3_LC | . | . | . | . | . | M | T | . | . | . | . | . | . | . | . | . | . | . | . | . | . | . | . | . | . | . |
| 21-225_214E4_LC | . | . | . | . | . | L | T | . | . | . | . | . | . | . | . | . | . | . | . | . | . | . | . | . | . | . |
| 21-225_223D11_LC | . | . | . | . | . | S | G | . | . | . | . | . | . | . | . | . | . | . | . | . | . | . | . | . | . | . |
| 21-225_22B12_LC | . | . | . | . | . | H | . | . | . | . | . | . | . | . | . | . | . | . | . | . | . | . | . | . | . | . |
| 21-225_23A11_LC | . | . | . | . | . | . | . | . | . | . | . | . | . | . | . | . | . | . | . | . | . | . | . | . | . | . |
| 21-225_23C7_LC | . | . | . | . | . | . | . | . | . | . | . | . | . | . | . | . | . | . | . | . | . | . | . | . | . | . |
| 21-225_23E7_LC | . | . | . | . | P | Y | . | . | . | . | . | . | . | . | . | . | . | . | . | . | . | . | . | . | . | . |
| 21-225_23G1_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | K_CDR3 | | | | | | | |
| CONSENSUS | Y | C | Q | Q | Y | N | S | - | - | - | - | - | - | - | - | - | - | - | - | - | - | - | - | - | - | - |
| 21-225_23G8_LC | . | . | . | . | . | | | | | | | | | | | | | | | | | | | | | |
| 21-225_25C10_LC | . | . | . | . | . | M | | | | | | | | | | | | | | | | | | | | |
| 21-225_26G1_LC | . | . | . | . | . | M | | | | | | | | | | | | | | | | | | | | |
| 21-225_27H12_LC | . | . | . | . | . | Y | | | | | | | | | | | | | | | | | | | | |
| 21-225_2A4_LC | . | . | . | . | . | . | | | | | | | | | | | | | | | | | | | | |
| 21-225_30A1_LC | . | . | . | . | . | . | | | | | | | | | | | | | | | | | | | | |
| 21-225_32B3_LC | . | . | . | . | . | . | | | | | | | | | | | | | | | | | | | | |
| 21-225_33A5_LC | . | . | . | . | . | . | | | | | | | | | | | | | | | | | | | | |
| 21-225_35H8_LC | . | . | . | . | . | . | | | | | | | | | | | | | | | | | | | | |
| 21-225_43A4_LC | . | . | . | . | . | Y | | | | | | | | | | | | | | | | | | | | |
| 21-225_45D9_LC | . | . | . | M | . | Y | | | | | | | | | | | | | | | | | | | | |
| 21-225_51C5_LC | . | . | . | . | . | Y | | | | | | | | | | | | | | | | | | | | |
| 21-225_51D5_LC | . | . | . | . | . | Y | | | | | | | | | | | | | | | | | | | | |
| 21-225_52E3_LC | . | . | . | . | . | . | | | | | | | | | | | | | | | | | | | | |
| 21-225_53E4_LC | . | . | . | . | . | M | | | | | | | | | | | | | | | | | | | | |
| 21-225_56F7_LC | . | . | M | . | . | . | | | | | | | | | | | | | | | | | | | | |
| 21-225_56H1_LC | . | . | . | . | . | Q | N | | | | | | | | | | | | | | | | | | | |
| 21-225_5A5_LC | . | . | . | . | . | . | | | | | | | | | | | | | | | | | | | | |
| 21-225_5E11_LC | . | . | M | . | . | M | | | | | | | | | | | | | | | | | | | | |
| 21-225_60A4_LC | . | . | . | . | . | K | | | | | | | | | | | | | | | | | | | | |
| 21-225_60F7_LC | . | . | L | . | P | M | | | | | | | | | | | | | | | | | | | | |
| 21-225_63H8_LC | . | . | . | . | . | M | | | | | | | | | | | | | | | | | | | | |
| 21-225_65D5_LC | . | . | P | L | . | K | | | | | | | | | | | | | | | | | | | | |
| 21-225_66H11_LC | . | . | . | . | . | S | Q | | | | | | | | | | | | | | | | | | | |
| 21-225_68E6_LC | . | . | . | . | . | O | | | | | | | | | | | | | | | | | | | | |
| 21-225_7B1_LC | . | . | M | . | . | . | | | | | | | | | | | | | | | | | | | | |
| 21-225_96C6_LC | . | . | M | . | . | . | O | | | | | | | | | | | | | | | | | | | |
| 21-225_9C8_LC | . | . | . | . | . | O | | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | F | T | F | G | P | G | T | K | V | D | I | K | R |
| 21-225_148B6_LC | . | | . | . | | . | | | . | . | | . | . | . | | . | . | . | |
| 21-225_14H8_LC | . | | | . | | . | | | . | . | | . | | | . | . | | N | |
| 21-225_153A1_LC | . | | | . | . | . | | | . | . | . | . | | | . | . | | | . |
| 21-225_157E4_LC | . | | . | | . | . | | | . | | . | . | | | . | . | . | | |
| 21-225_157G5_LC | . | | . | | . | . | | | . | . | | . | | | . | . | . | | . |
| 21-225_157H1_LC | . | | . | | . | . | | | . | . | | . | | | . | . | . | | . |
| 21-225_157H10_LC | . | | . | | . | . | | | . | . | | . | | | . | . | . | . | . |
| 21-225_158H12_LC | . | | . | | . | . | | | . | . | | . | | | . | . | . | . | . |
| 21-225_159H11_LC | . | | . | | . | . | | | . | . | | . | . | | . | . | . | . | . |
| 21-225_15A2_LC | . | | . | . | . | . | | | . | | . | . | . | Q | . | V | . | . | . |
| 21-225_15B1_LC | . | | . | . | | . | | | . | . | | . | | | . | . | . | . | . |
| 21-225_160G1_LC | . | | . | . | . | . | | | . | . | | . | | | . | . | . | | . |
| 21-225_160G3_LC | . | | . | . | . | . | | | . | . | | . | | | . | . | . | | . |
| 21-225_163F1_LC | . | | . | . | | . | | | . | . | | . | . | Q | . | | | Q | . |
| 21-225_167D12_LC | . | | . | | . | . | | | . | . | | . | | | . | . | . | . | . |
| 21-225_169D10_LC | . | | . | . | | . | | | . | . | | . | . | . | . | . | . | . | . |
| 21-225_16B8_LC | . | | . | . | . | . | | | . | . | | . | | | . | . | V | . | . |
| 21-225_16C7_LC | . | | . | . | . | . | | | . | . | | . | | | . | . | . | . | . |
| 21-225_16G12_LC | . | | . | P | . | . | | | . | . | | . | | | . | . | . | . | . |
| 21-225_170E1_LC | . | | . | . | . | . | | | . | . | | . | | | . | . | . | . | . |
| 21-225_175C4_LC | . | | . | . | . | . | | | . | . | | . | | | . | . | . | . | . |
| 21-225_177D3_LC | . | | . | . | . | . | | | . | . | | . | | | . | . | . | . | . |
| 21-225_17H5_LC | . | | . | . | . | . | | | . | . | | . | | | . | . | . | . | . |
| 21-225_17H6_LC | . | | . | . | . | . | | | . | . | | . | | | . | . | . | Q | . |
| 21-225_181G2_LC | . | | . | . | . | . | | | . | . | | . | | | . | . | . | . | . |
| 21-225_184E7_LC | . | | . | . | . | . | | | | . | | . | | | . | . | . | . | . |
| 21-225_185A1_LC | . | | . | . | . | . | | | . | . | | . | | | . | . | . | . | . |
| 21-225_185E10_LC | . | | . | . | . | . | | | . | . | | . | | | . | . | . | . | . |
| 21-225_188B9_LC | . | | . | . | . | . | | | . | . | | . | | | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | F | T | F | G | P | G | T | K | V | D | I | K | R |
| 21-225_188H5_LC | . | . | . | . | . | . | . | . | . | . | . | . | . | . | . | . | . | . | . |
| 21-225_18D11_LC | . | . | . | . | . | . | . | . | . | . | . | . | . | T | . | . | . | . | . |
| 21-225_18G10_LC | . | . | . | . | . | . | . | . | . | . | . | . | . | . | . | . | . | . | . |
| 21-225_190A5_LC | . | . | . | . | . | . | V | . | . | . | . | . | . | . | . | . | . | . | . |
| 21-225_190B1_LC | . | . | . | . | . | . | V | . | . | . | . | . | . | . | . | . | . | . | . |
| 21-225_190F12_LC | . | . | . | . | . | . | . | . | . | . | . | . | . | . | . | . | . | . | . |
| 21-225_190H9_LC | . | . | . | . | . | . | . | . | . | . | . | . | . | . | . | . | . | . | . |
| 21-225_192A2_LC | . | . | . | . | . | . | . | . | . | . | . | . | . | . | . | . | . | . | . |
| 21-225_192C10_LC | . | . | . | . | . | . | . | . | . | . | . | . | . | . | . | . | . | . | . |
| 21-225_192E4_LC | . | . | . | . | . | . | . | . | . | . | . | . | . | . | . | . | . | . | . |
| 21-225_192H6_LC | . | . | . | . | . | . | V | . | . | . | . | . | . | . | . | . | . | . | . |
| 21-225_193F8_LC | . | . | . | . | . | . | . | . | . | . | . | . | . | . | . | . | . | . | . |
| 21-225_194H12_LC | . | . | . | . | . | . | . | . | . | . | . | . | . | . | . | . | . | . | . |
| 21-225_197E11_LC | . | . | . | . | . | . | . | . | . | . | . | . | . | . | . | . | . | . | . |
| 21-225_20B9_LC | . | . | . | . | . | . | . | . | . | . | . | . | . | . | . | . | . | . | . |
| 21-225_20C11_LC | . | . | . | . | . | . | . | . | . | . | . | . | . | . | . | . | V | . | . |
| 21-225_20F10_LC | . | . | . | . | . | . | . | . | . | . | . | . | . | . | . | . | N | . | . |
| 21-225_20H7_LC | . | . | . | . | . | . | . | . | . | . | . | . | . | . | . | . | . | . | . |
| 21-225_20H9_LC | . | . | . | . | . | . | . | . | . | . | . | . | . | . | . | . | . | . | . |
| 21-225_211A6_LC | . | . | . | . | . | . | . | . | . | . | . | . | . | . | . | . | . | . | . |
| 21-225_213B3_LC | . | . | . | . | . | . | . | . | . | . | . | . | . | . | . | . | . | . | . |
| 21-225_214C3_LC | . | . | . | . | . | . | . | . | . | . | . | . | . | . | . | . | . | . | . |
| 21-225_214E4_LC | . | . | . | . | . | . | . | . | . | . | . | . | . | . | . | . | . | . | . |
| 21-225_223D11_LC | . | . | . | . | . | . | . | . | . | . | . | . | . | . | . | . | T | . | . |
| 21-225_22B12_LC | . | . | . | . | . | . | . | . | . | . | . | . | . | . | . | . | . | . | . |
| 21-225_23A11_LC | . | . | . | . | . | . | . | . | . | . | . | . | . | . | . | . | . | . | . |
| 21-225_23C7_LC | . | . | . | . | . | . | . | . | . | . | . | . | . | . | . | . | . | . | . |
| 21-225_23E7_LC | . | . | . | . | . | . | . | . | . | . | . | . | . | . | . | . | . | N | . |
| 21-225_23G1_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | F | T | F | G | P | G | T | K | V | D | I | K | R |
| 21-225_23G8_LC | . | . | . | . | . | . | . | . | ş | . | . | . | . | . | . | . | . | . | . |
| 21-225_25C10_LC | . | . | . | . | . | . | . | . | . | . | . | . | . | . | . | . | . | . | . |
| 21-225_26G1_LC | . | . | . | . | . | . | . | . | . | . | . | . | . | . | . | . | . | . | . |
| 21-225_27H12_LC | . | . | . | . | . | . | . | . | . | . | . | . | . | . | . | N | . | N | . |
| 21-225_2A4_LC | . | . | . | . | . | . | . | . | . | . | . | . | . | . | . | . | . | . | . |
| 21-225_30A1_LC | . | . | . | . | . | . | . | . | . | . | . | . | . | . | . | . | . | . | . |
| 21-225_32B3_LC | . | . | . | . | . | . | . | . | . | . | . | . | . | . | . | . | . | . | . |
| 21-225_33A5_LC | . | . | . | . | . | . | . | . | . | . | . | . | . | N | . | . | . | . | . |
| 21-225_35H8_LC | . | . | . | . | . | . | . | . | . | . | . | . | . | . | . | . | ℱ | . | . |
| 21-225_43A4_LC | . | . | . | . | . | . | . | . | . | . | . | . | . | . | . | . | . | . | . |
| 21-225_45D9_LC | . | . | . | . | . | . | . | . | . | . | ℛ | . | . | . | . | . | . | . | . |
| 21-225_51C5_LC | . | . | . | . | . | . | . | . | . | . | . | . | . | . | . | . | . | . | . |
| 21-225_51D5_LC | . | . | . | . | . | . | . | . | . | . | . | . | . | . | . | . | . | . | . |
| 21-225_52E3_LC | . | . | . | . | ℱ | . | . | . | . | . | . | . | . | . | . | . | . | . | . |
| 21-225_53E4_LC | . | . | . | . | . | . | Ł | . | . | . | ℛ | . | . | . | . | . | . | . | . |
| 21-225_56F7_LC | . | . | . | . | ℱ | . | . | ℋ | . | . | ℛ | . | . | . | . | . | . | Y | Ĝ |
| 21-225_56H1_LC | . | . | . | . | . | . | . | . | . | . | . | . | . | . | . | . | . | . | . |
| 21-225_5A5_LC | . | . | . | . | . | . | . | . | . | . | . | . | . | . | . | . | . | . | . |
| 21-225_5E11_LC | . | . | . | . | . | . | . | . | . | . | . | . | . | . | . | . | . | . | . |
| 21-225_60A4_LC | . | . | . | . | M | . | . | . | . | . | . | . | . | . | . | . | . | . | . |
| 21-225_60F7_LC | . | . | . | . | . | . | . | . | . | . | . | . | . | . | M | . | . | . | . |
| 21-225_63H8_LC | . | . | . | . | . | . | . | . | . | . | . | . | . | . | . | . | ℛ | . | . |
| 21-225_65D5_LC | . | . | . | . | . | . | Ł | . | . | . | . | . | . | . | . | . | . | . | . |
| 21-225_66H11_LC | . | . | . | . | . | . | . | . | . | . | ℋ | . | . | . | . | . | . | . | . |
| 21-225_68E6_LC | . | . | . | . | . | . | . | . | . | . | . | . | . | . | . | . | ℛ | . | . |
| 21-225_7B1_LC | . | . | . | . | . | . | . | . | . | . | . | . | . | . | . | . | ℱ | . | . |
| 21-225_96C6_LC | . | . | . | . | . | . | . | . | . | . | . | . | . | . | . | . | . | . | . |
| 21-225_9C8_LC | . | . | . | . | . | . | . | . | . | . | . | . | . | . | . | . | . | . | . |

**FIGURE 57**

Table 104.     Consensus 24 - VK1|L5/JK3 (SEQ ID NO: 50321):

DIQMTQSPSSVSASVGDRVTITCRAS--QGIS------RWLAWYQQKPGKAPKLLIYA-------ASSLQSGVPSRFSGSGSG--
TDFTLTISSLQPEDFATYYCQQANS---------------------FPFTFGPGTKVDIKR wherein:

A at position 25 can be substituted with V, E or G

S at position 26 can be substituted with G

Q at position 29 can be substituted with R

G at position 30 can be substituted with D, N, A, L or V

I at position 31 can be substituted with V or F

S at position 32 can be substituted with N, T, R or I

R at position 39 can be substituted with S, N, K, T, D, I or G

W at position 40 can be substituted with Y

L at position 41 can be substituted with I

A at position 42 can be substituted with T or V

A at position 58 can be substituted with G, T, D or V

A at position 67 can be substituted with T or V

S at position 68 can be substituted with Y

S at position 69 can be substituted with R, N, T, G or I

L at position 70 can be substituted with F

Q at position 71 can be substituted with E

**FIGURE 57**

S at position 72 can be substituted with G, N or D

Q at position 107 can be substituted with H

A at position 109 can be substituted with T, G, S, V or D

N at position 110 can be substituted with D, K or S

S at position 111 can be substituted with I

F at position 135 can be substituted with L, I or V

F at position 137 can be substituted with I

Arg Xaa Xaa Xaa Xaa Xaa Xaa Xaa Xaa Xaa Xaa (SEQ ID NO: 50147)

Xaa Xaa Xaa Xaa Xaa Xaa Xaa (SEQ ID NO: 50148)

Xaa Gln Xaa Xaa Xaa Xaa Pro Xaa Thr (SEQ ID NO: 50149)

Arg Ala Ser Gln Gly Ile Ser Arg Trp Leu Ala (SEQ ID NO: 50633)

Ala Ala Ser Ser Leu Gln Ser (SEQ ID NO: 50634)

Gln Gln Ala Asn Ser Phe Pro Phe Thr (SEQ ID NO: 50635)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | V | S | A | S | V | G | D | R | V | T | I | T | C | R | A | S |
| 21-225_10G6_LC | . | . | . | . | . | . | . | . | . | . | . | . | . | . | . | . | . | . | . | . | . | . | . | . | . | . |
| 21-225_12E9_LC | . | . | . | . | . | . | . | . | . | . | . | . | . | . | . | ⁂ | . | . | . | . | . | . | . | . | . | . |
| 21-225_146A6_LC | . | . | . | . | . | . | . | . | A | . | . | . | . | . | . | . | . | . | . | . | . | . | . | . | . | . |
| 21-225_146E1_LC | . | ⁂ | . | . | . | . | . | . | . | . | . | . | . | . | . | . | . | . | . | . | . | . | . | . | . | . |
| 21-225_147D10_LC | . | . | . | . | . | . | . | . | A | . | . | . | . | . | . | . | . | . | . | . | . | . | . | . | . | . |
| 21-225_147D12_LC | . | . | . | . | . | . | . | . | A | . | . | . | . | . | . | . | . | . | . | . | . | . | . | . | . | . |
| 21-225_148E2_LC | . | . | . | . | . | . | . | . | A | . | . | . | . | . | . | . | . | . | . | . | . | . | . | . | . | . |
| 21-225_149B6_LC | . | . | . | . | . | . | . | . | . | . | . | . | . | . | . | . | . | . | . | . | . | . | . | . | . | . |
| 21-225_149C6_LC | . | . | . | . | . | . | . | . | A | . | . | . | . | . | . | . | . | . | . | . | . | . | . | . | . | . |
| 21-225_149D11_LC | . | . | . | . | . | . | . | . | A | . | . | . | . | . | . | . | . | . | . | . | . | . | . | . | . | . |
| 21-225_149F8_LC | . | . | . | . | . | . | . | . | A | . | . | . | . | . | . | . | . | . | . | . | . | . | . | . | . | . |
| 21-225_150C5_LC | . | . | . | . | . | . | . | . | G | . | . | . | . | . | . | . | . | . | . | . | . | . | . | . | . | . |
| 21-225_152G10_LC | . | . | . | . | . | . | . | . | A | . | . | . | . | . | . | . | . | . | . | . | . | . | . | . | . | . |
| 21-225_152H7_LC | . | . | . | . | . | . | . | . | A | . | . | . | . | . | . | . | . | . | . | . | . | . | . | . | . | . |
| 21-225_155A4_LC | . | . | . | . | . | . | . | . | A | . | . | . | . | . | . | . | . | . | . | . | . | . | . | . | . | . |
| 21-225_155B4_LC | . | . | . | . | . | . | . | . | A | . | . | . | . | . | . | . | . | . | . | . | . | . | . | . | . | . |
| 21-225_180A3_LC | . | . | . | . | . | . | . | . | . | . | . | . | . | . | . | . | . | . | . | . | . | . | . | . | . | . |
| 21-225_182H5_LC | . | . | . | . | . | . | . | . | . | . | Y | . | . | . | . | . | . | . | . | . | . | . | . | . | . | . |
| 21-225_186A11_LC | . | . | . | . | . | . | . | . | . | . | . | . | . | . | . | . | . | . | . | . | . | . | . | . | . | . |
| 21-225_192H2_LC | . | . | . | . | . | . | . | . | . | . | . | . | . | . | . | . | . | . | . | . | . | . | . | . | . | . |
| 21-225_196F2_LC | . | . | . | . | . | . | . | . | . | . | . | . | . | . | . | . | . | . | . | . | . | . | . | . | . | . |
| 21-225_210H11_LC | . | . | . | . | . | . | . | . | . | . | . | . | . | . | . | . | . | . | . | . | . | . | . | . | . | . |
| 21-225_221A6_LC | . | . | . | . | . | . | . | . | . | . | . | . | . | . | . | . | . | . | . | . | . | . | . | . | . | . |
| 21-225_221F12_LC | . | . | . | . | . | . | . | . | . | . | . | . | . | . | . | . | . | . | . | . | . | . | . | . | . | . |
| 21-225_222C12_LC | . | . | . | . | . | C | . | . | . | . | . | . | . | . | . | . | . | . | . | . | . | . | . | . | V | . |
| 21-225_222E1_LC | . | . | . | . | . | . | . | . | . | . | . | . | . | . | . | . | . | . | . | . | . | . | . | . | . | . |
| 21-225_222F7_LC | . | . | . | . | . | . | . | . | . | . | . | . | . | . | . | . | . | . | . | . | . | . | . | . | . | . |
| 21-225_23B11_LC | . | . | . | . | . | . | . | . | . | . | . | . | . | . | . | . | . | . | . | . | . | . | . | . | . | . |
| 21-225_25E11_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | V | S | A | S | V | G | D | R | V | T | I | T | C | R | A | S |
| 21-225_28B1_LC | . | . | . | . | . | . | . | . | . | . | . | . | . | . | . | . | . | . | . | . | . | . | . | . | . | . |
| 21-225_28F11_LC | . | . | . | . | . | . | . | . | . | . | . | . | . | . | . | . | . | . | . | . | . | . | . | . | . | . |
| 21-225_30A11_LC | . | . | . | . | . | . | . | . | . | . | . | . | . | . | . | . | . | . | . | . | . | . | . | . | . | . |
| 21-225_31C4_LC | . | . | . | . | . | . | . | . | . | . | . | . | . | . | . | . | . | . | . | . | . | . | . | . | . | . |
| 21-225_31H9_LC | . | . | . | . | . | . | . | . | . | . | . | . | . | . | . | . | . | . | . | . | . | . | . | . | . | . |
| 21-225_33G1_LC | . | . | . | . | . | . | . | . | . | . | . | . | V | . | . | . | . | . | . | . | . | . | . | . | . | . |
| 21-225_33H7_LC | . | . | . | . | . | . | . | . | . | . | . | . | . | . | . | . | . | . | . | . | . | . | . | . | . | . |
| 21-225_34F3_LC | . | . | . | . | . | . | . | . | . | . | . | . | . | T | . | . | . | . | . | . | S | . | . | . | . | . |
| 21-225_34G7_LC | . | . | . | . | . | . | . | . | . | . | . | . | . | . | . | . | . | . | . | . | . | . | . | . | . | . |
| 21-225_35C6_LC | . | . | . | . | . | . | . | . | . | . | . | . | . | . | . | . | . | . | . | . | . | . | . | . | . | . |
| 21-225_35G6_LC | . | . | . | . | . | . | . | . | . | . | . | . | . | . | . | . | . | . | L | . | . | . | . | . | . | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | . | . | . | . | . | . | . | . | . | . | . | . | . | . | . | . | . | . | . | . | . | . | . | . | . | . |
| 21-225_44F3_LC | . | . | . | . | . | . | . | . | . | . | . | . | . | . | . | . | . | . | . | . | . | . | . | . | . | . |
| 21-225_45C9_LC | . | . | . | . | . | . | . | . | . | . | . | . | V | . | . | . | . | . | . | . | . | . | . | . | . | . |
| 21-225_45H4_LC | . | . | . | . | . | . | . | . | . | . | . | . | . | . | . | . | . | . | . | . | . | . | . | . | . | . |
| 21-225_49F1_LC | . | . | . | . | . | . | . | . | . | . | . | . | . | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | . | . | . | . | . | . | . | . | . | . | . | . | . | . | . | . | . | . | . | . | . | . |
| 21-225_4H6_LC | . | . | . | . | . | . | . | . | . | . | . | . | . | . | . | . | . | . | . | . | . | . | . | . | . | . |
| 21-225_4H6.004_LC | . | . | . | . | . | . | . | . | . | . | . | . | . | . | . | . | . | . | . | . | . | . | . | . | . | . |
| 21-225_4H6.005_LC | . | . | . | . | . | . | . | . | . | . | . | . | . | . | . | . | . | . | . | . | . | . | . | . | . | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | . | . |
| 21-225_4H6.008_LC | . | . | . | . | . | . | . | . | . | . | . | . | . | . | . | . | . | . | . | . | . | . | . | . | . | . |
| 21-225_4H6.009_LC | . | . | . | . | . | . | . | . | . | . | . | . | . | . | . | . | . | . | . | . | . | . | . | . | . | . |
| 21-225_4H6.010_LC | . | . | . | . | . | . | . | . | . | . | . | . | . | . | . | . | . | . | . | . | . | . | . | . | . | . |
| 21-225_4H6.011_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | V | S | A | S | V | G | D | R | V | T | I | T | C | R | A | S |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | L | . | . | . | . | . | . | . |
| 21-225_50F3_LC | . | . | . | . | . | . | . | . | . | . | . | . | . | . | . | . | . | . | . | . | . | . | . | . | . | . |
| 21-225_51C7_LC | . | . | . | . | . | . | . | . | . | . | . | . | . | . | . | . | . | . | . | . | . | . | . | . | . | . |
| 21-225_51E9_LC | . | . | . | . | . | . | . | . | . | . | . | . | . | . | . | . | . | . | . | . | . | . | . | . | . | . |
| 21-225_52B1_LC | . | . | . | . | . | . | . | . | . | . | T | S | . | . | . | . | . | . | i | . | . | . | . | . | . | . |
| 21-225_52G11_LC | . | . | . | . | . | . | . | . | . | . | . | . | . | . | . | . | . | . | . | . | . | . | . | . | . | . |
| 21-225_52H10_LC | . | . | . | . | . | . | . | . | . | . | . | . | . | . | . | . | . | . | . | . | . | . | . | . | . | . |
| 21-225_53F2_LC | . | . | . | . | . | . | . | . | . | . | . | . | . | . | . | . | . | . | . | . | . | . | . | . | . | . |
| 21-225_53F7_LC | . | . | . | . | S | . | . | . | . | . | . | . | . | . | . | . | . | . | . | . | . | . | . | . | . | . |
| 21-225_55B1_LC | . | . | . | . | . | . | . | . | . | . | . | . | . | . | . | . | . | . | . | . | . | . | . | . | . | . |
| 21-225_56C6_LC | . | . | . | . | . | . | . | . | . | . | . | . | . | . | . | . | . | . | . | . | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | . | . | . | . | . | . | . | . | . | . | G | . | Y | . | . | . | . | . | . | . | . | . | . | V | . |
| 21-225_57A7_LC | . | . | . | . | . | . | . | . | . | . | . | . | . | . | . | . | . | . | . | . | . | . | . | . | . | . |
| 21-225_57E4_LC | . | . | . | . | . | . | . | . | . | . | . | . | . | . | . | . | . | . | . | S | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | . | . | . | . | . | . | . | . | . | . | . | . | . | . | . | . | . | . | . | . | . | . | . |
| 21-225_65F10_LC | . | . | . | . | . | . | . | . | . | . | . | . | . | . | . | . | . | . | . | . | . | . | . | . | G | G |
| 21-225_66F11_LC | . | . | . | . | . | . | . | . | . | . | . | G | . | . | . | . | . | . | . | . | . | . | . | . | E | . |
| 21-225_70D1_LC | . | . | . | . | . | . | . | . | . | . | . | . | . | . | . | . | . | . | . | . | . | . | . | . | . | . |
| 21-225_7C3_LC2_LC | . | . | . | . | . | . | . | . | . | . | . | . | . | L | . | . | . | . | . | . | . | . | . | . | . | . |
| 21-225_9F12_LC1_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | R | W | L | A | W | Y | Q | Q | K | P | G | K | A | P |
| 21-225_10G6_LC | . | . | . | . | . | . | . | . | . | . | . | . | x | . | . | . | . | . | . | . | . | . | . | . | . | . |
| 21-225_12E9_LC | . | . | . | . | . | . | . | . | . | . | . | . | S | . | . | . | . | . | . | . | . | . | . | S | . | . |
| 21-225_146A6_LC | . | . | . | . | . | . | . | . | . | . | . | . | N | . | . | . | . | . | . | . | . | . | . | . | . | . |
| 21-225_146E1_LC | . | . | . | . | . | N | . | . | . | . | . | . | N | . | . | V | . | . | . | . | . | . | . | . | . | . |
| 21-225_147D10_LC | . | . | N | . | . | . | . | . | . | . | . | . | N | . | . | T | . | . | . | . | . | . | . | . | . | . |
| 21-225_147D12_LC | . | . | . | . | . | . | . | . | . | . | . | . | N | . | . | . | . | . | . | S | . | . | . | . | . | . |
| 21-225_148E2_LC | . | . | . | . | . | . | . | . | . | . | . | . | N | . | . | . | . | . | . | . | . | . | . | . | . | . |
| 21-225_149B6_LC | . | . | . | . | I | . | . | . | . | . | . | . | S | . | . | . | . | . | . | . | . | . | . | . | . | . |
| 21-225_149C6_LC | . | . | . | . | . | . | . | . | . | . | . | . | N | . | . | . | . | . | . | . | . | . | . | S | . | . |
| 21-225_149D11_LC | . | . | N | . | . | . | . | . | . | . | . | . | N | . | . | T | . | . | . | . | . | . | . | . | . | . |
| 21-225_149F8_LC | . | . | . | . | . | . | . | . | . | . | . | . | N | . | . | . | . | . | . | . | . | . | . | . | . | . |
| 21-225_150C5_LC | . | . | . | . | . | N | . | . | . | . | . | . | N | . | . | . | . | . | . | . | . | . | . | . | . | . |
| 21-225_152G10_LC | . | . | . | . | . | . | . | . | . | . | . | . | N | . | . | . | . | . | . | . | . | . | . | . | . | . |
| 21-225_152H7_LC | . | . | D | . | . | . | . | . | . | . | . | . | N | . | . | . | . | . | . | . | . | . | . | . | . | . |
| 21-225_155A4_LC | . | . | . | . | . | . | . | . | . | . | . | . | N | . | . | . | . | . | S | . | . | . | . | . | . | . |
| 21-225_155B4_LC | . | . | D | . | . | . | . | . | . | . | . | . | N | . | . | . | . | . | . | . | . | . | . | . | . | . |
| 21-225_180A3_LC | . | . | D | . | . | T | . | . | . | . | . | . | S | . | . | . | . | . | . | . | . | . | . | . | . | . |
| 21-225_182H5_LC | . | . | D | . | . | T | . | . | . | . | . | . | S | . | . | . | . | . | . | . | . | . | . | . | . | . |
| 21-225_186A11_LC | . | . | D | . | . | T | . | . | . | . | . | . | S | . | . | . | . | . | . | . | . | . | . | . | . | . |
| 21-225_192H2_LC | . | . | . | . | . | . | . | . | . | . | . | . | S | . | I | . | . | . | . | . | . | . | . | . | . | . |
| 21-225_196F2_LC | . | . | . | . | . | . | . | . | . | . | . | . | S | . | . | . | . | . | . | . | . | . | . | . | . | . |
| 21-225_210H11_LC | . | . | . | . | . | . | . | . | . | . | . | . | I | . | . | . | . | . | . | . | . | . | . | . | . | . |
| 21-225_221A6_LC | . | . | . | . | . | . | . | . | . | . | . | . | S | . | . | . | . | . | . | . | . | . | . | . | . | . |
| 21-225_221F12_LC | . | . | . | . | . | . | . | . | . | . | . | . | S | . | . | . | . | . | . | . | . | . | . | . | . | . |
| 21-225_222C12_LC | . | . | . | . | . | . | . | . | . | . | . | . | S | . | . | . | . | . | . | . | . | . | . | . | . | L |
| 21-225_222E1_LC | . | . | . | . | . | . | . | . | . | . | . | . | S | . | . | . | . | . | . | . | . | . | . | . | . | . |
| 21-225_222F7_LC | . | . | . | . | . | . | . | . | . | . | . | . | S | . | . | . | . | . | . | . | . | . | . | . | . | . |
| 21-225_23B11_LC | . | . | . | . | . | T | . | . | . | . | . | . | x | . | . | . | . | . | . | . | . | . | . | . | . | . |
| 21-225_25E11_LC | . | . | . | . | . | . | . | . | . | . | . | . | N | . | . | . | . | . | . | . | . | . | . | . | . | . |

## FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | R | W | L | A | W | Y | Q | Q | K | P | G | K | A | P |
| 21-225_28B1_LC | . | . | . | A | . | N | . | | . | | | | D | . | | | . | . | | | | | . | . | . | . |
| 21-225_28F11_LC | . | . | . | . | . | . | . | | | | | | N | | | | | | | | | | . | . | . | . |
| 21-225_30A11_LC | . | . | . | . | . | . | . | | | | | . | . | . | . | . | . | . | . | . | . | . | . | R | . | . |
| 21-225_31C4_LC | . | . | . | . | . | . | . | | | | | . | K | . | . | . | . | . | . | . | . | . | | | | . |
| 21-225_31H9_LC | . | . | . | . | . | T | . | | | | | . | S | . | . | T | . | . | . | . | R | . | . | . | . | . |
| 21-225_33G1_LC | . | . | . | . | . | . | . | . | . | . | . | . | . | . | . | . | . | . | . | . | . | . | . | . | . | . |
| 21-225_33H7_LC | . | . | . | . | . | . | . | . | . | . | . | . | . | . | . | . | . | . | . | . | . | V | . | . | V | . |
| 21-225_34F3_LC | . | . | . | . | . | . | . | . | . | . | . | . | K | . | . | . | . | . | . | . | . | . | . | . | . | . |
| 21-225_34G7_LC | . | . | . | L | . | . | . | . | . | . | . | . | K | . | . | . | . | . | . | . | . | . | . | . | . | . |
| 21-225_35C6_LC | . | . | . | . | . | . | . | . | . | . | . | . | K | . | . | . | . | . | . | . | . | . | . | . | . | . |
| 21-225_35G6_LC | . | . | . | . | . | . | . | . | . | . | . | . | . | . | . | . | . | . | . | . | . | V | . | . | V | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | . | . | . | D | . | . | . | . | . | . | . | . | S | . | . | . | . | . | . | R | . | . | . | . | . | . |
| 21-225_44F3_LC | . | . | . | . | . | . | . | . | . | . | . | . | Q | . | . | . | . | . | . | . | . | . | . | . | . | . |
| 21-225_45C9_LC | . | . | . | D | . | . | . | . | . | . | . | . | Q | . | . | . | . | . | . | . | R | . | . | . | . | . |
| 21-225_45H4_LC | . | . | . | D | . | . | . | . | . | . | . | . | S | . | . | . | . | . | . | R | . | . | . | . | . | . |
| 21-225_49F1_LC | . | . | R | D | . | N | . | . | . | . | . | . | G | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | P | . | . | . | . | . | . | . | T | . | . | . | . | P | . | . | . | . | . | . | . | . |
| 21-225_4H6_LC | . | . | . | . | . | . | . | . | . | . | . | . | . | . | . | . | . | . | . | . | . | . | . | . | . | . |
| 21-225_4H6.004_LC | . | . | . | . | . | . | . | . | . | . | . | . | . | . | . | . | . | . | . | . | . | . | . | . | . | . |
| 21-225_4H6.005_LC | . | . | . | . | . | . | . | . | . | . | . | . | . | . | . | . | . | . | . | . | . | . | . | . | . | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | . | . |
| 21-225_4H6.008_LC | . | . | . | . | . | . | . | . | . | . | . | . | . | . | . | . | . | . | . | . | . | . | . | . | . | . |
| 21-225_4H6.009_LC | . | . | . | . | . | . | . | . | . | . | . | . | . | . | . | . | . | . | . | . | . | . | . | . | . | . |
| 21-225_4H6.010_LC | . | . | . | . | . | . | . | . | . | . | . | . | . | . | . | . | . | . | . | . | . | . | . | . | . | . |
| 21-225_4H6.011_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | K_FR2 | | | | | | | | |
| CONSENSUS | - | - | Q | G | I | S | - | - | - | - | - | - | R | W | L | A | W | Y | Q | Q | K | P | G | K | A | P |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | . | . | . | . | . | . | . | . |
| 21-225_50F3_LC | . | . | . | D | . | . | . | . | . | . | . | . | S | . | . | . | . | F | . | . | . | . | . | . | . | . |
| 21-225_51C7_LC | . | . | . | D | V | N | . | . | . | . | . | . | N | Y | . | . | . | F | . | . | . | . | . | . | . | . |
| 21-225_51E9_LC | . | . | . | . | . | . | . | . | . | . | . | . | S | . | . | . | . | . | . | . | . | . | . | . | . | . |
| 21-225_52B1_LC | . | . | . | D | . | R | . | . | . | . | . | . | T | . | . | . | . | . | . | . | . | . | . | . | . | . |
| 21-225_52G11_LC | . | . | . | . | . | R | . | . | . | . | . | . | K | . | . | . | . | . | . | . | . | . | . | . | . | . |
| 21-225_52H10_LC | . | . | . | D | . | N | . | . | . | . | . | . | S | . | . | . | . | . | . | . | . | . | . | . | . | . |
| 21-225_53F2_LC | . | . | . | . | . | . | . | . | . | . | . | . | K | . | . | . | . | . | . | . | . | . | . | . | . | . |
| 21-225_53F7_LC | . | . | . | . | . | . | . | . | . | . | . | . | . | . | . | . | . | . | . | . | . | . | . | . | . | . |
| 21-225_55B1_LC | . | . | V | . | . | . | . | . | . | . | . | . | . | . | . | . | . | . | . | . | . | . | . | . | . | . |
| 21-225_56C6_LC | . | . | . | . | . | . | . | . | . | . | . | . | S | . | . | . | . | . | . | . | . | S | . | N | . | . |
| 21-225_56E5_LC | . | . | . | . | . | R | . | . | . | . | . | . | K | . | . | . | . | . | . | . | . | . | . | . | . | . |
| 21-225_56F6_LC | . | . | . | D | . | . | . | . | . | . | . | . | K | . | . | . | . | F | . | . | . | . | . | . | . | . |
| 21-225_57A7_LC | . | . | . | . | . | . | . | . | . | . | . | . | . | . | . | . | . | . | . | . | . | . | . | . | . | . |
| 21-225_57E4_LC | . | . | . | D | . | . | . | . | . | . | . | . | N | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | N | . | T | . | . | . | . | . | . | . | T | . | . | . | . | . | . | . | . | . | . | . | . | . |
| 21-225_65F10_LC | . | . | . | . | . | . | . | . | . | . | . | . | T | . | . | . | . | . | . | . | . | . | . | . | . | . |
| 21-225_66F11_LC | . | . | . | . | . | . | . | . | . | . | . | . | I | . | . | . | . | . | . | . | . | . | . | . | . | . |
| 21-225_70D1_LC | . | . | . | V | . | . | . | . | . | . | . | . | . | . | . | . | . | . | . | . | . | . | . | . | . | . |
| 21-225_7C3_LC2_LC | . | . | . | . | . | . | . | . | . | . | . | . | S | . | . | . | . | . | . | . | . | Q | . | . | . | . |
| 21-225_9F12_LC1_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_10G6_LC | . | ¦ | . | . | . | . | . | . | . | . | . | . | . | . | . | . | Y | . | . | . | . | . | . | A | . | . |
| 21-225_12E9_LC | | | | | | . | | . | . | . | . | . | . | . | . | ⊕ | . | . | . | . | . | . | . | . | . | . |
| 21-225_146A6_LC | . | . | . | . | . | . | . | . | . | . | . | . | . | . | . | . | . | . | . | ⊗ | . | . | . | . | . | . |
| 21-225_146E1_LC | . | . | . | . | . | . | . | . | . | . | . | . | . | . | . | . | . | . | . | . | . | . | . | . | . | . |
| 21-225_147D10_LC | . | . | . | . | . | . | . | . | . | . | . | . | . | . | . | . | . | . | . | . | . | . | . | . | . | . |
| 21-225_147D12_LC | . | . | . | . | . | . | . | . | . | . | . | . | . | . | . | . | . | . | . | . | . | . | . | . | . | . |
| 21-225_148E2_LC | . | . | . | . | . | . | . | . | . | . | . | . | . | . | . | . | . | . | . | . | . | . | . | . | . | . |
| 21-225_149B6_LC | . | . | . | . | . | . | . | . | . | . | . | . | . | . | . | . | . | . | . | . | . | . | . | . | . | . |
| 21-225_149C6_LC | . | . | . | . | . | . | . | . | . | . | . | . | . | . | . | . | . | . | . | ⊗ | . | . | . | . | . | . |
| 21-225_149D11_LC | . | . | . | . | . | . | . | . | . | . | . | . | . | . | . | . | . | . | . | . | . | . | . | . | . | . |
| 21-225_149F8_LC | . | . | . | . | . | . | . | . | . | . | . | . | . | . | . | . | . | . | . | . | . | . | . | . | . | . |
| 21-225_150C5_LC | . | . | . | . | . | . | . | . | . | . | . | . | . | . | . | . | . | . | . | ⊗ | . | . | . | . | . | . |
| 21-225_152G10_LC | . | . | . | S | . | . | . | . | . | . | . | . | . | . | . | . | . | . | . | . | . | . | . | . | . | . |
| 21-225_152H7_LC | . | . | . | . | . | . | . | . | . | . | . | . | . | . | . | . | . | . | . | ⊗ | . | . | . | . | . | . |
| 21-225_155A4_LC | . | . | . | . | . | . | . | . | . | . | . | . | . | . | . | . | . | . | . | ⊗ | . | . | . | . | . | . |
| 21-225_155B4_LC | . | . | . | . | . | . | . | . | . | . | . | . | . | . | . | . | . | . | . | ⊗ | . | . | . | . | . | . |
| 21-225_180A3_LC | . | . | . | . | . | . | . | . | . | . | . | . | . | . | . | . | . | . | . | . | . | . | . | . | . | . |
| 21-225_182H5_LC | . | . | . | . | . | . | . | . | . | . | . | . | . | . | . | . | . | . | . | . | . | . | . | . | . | . |
| 21-225_186A11_LC | . | . | . | . | . | . | . | . | . | . | . | . | . | . | . | . | . | . | . | . | . | . | . | . | . | . |
| 21-225_192H2_LC | . | . | . | . | ⊗ | . | . | . | . | . | . | . | . | . | . | . | . | . | . | . | . | . | . | . | . | . |
| 21-225_196F2_LC | . | . | . | . | . | . | . | . | . | . | . | . | . | . | . | . | . | . | . | . | . | . | . | . | . | . |
| 21-225_210H11_LC | N | . | . | . | . | . | . | . | . | . | . | . | . | . | . | . | . | . | . | . | . | . | . | . | . | . |
| 21-225_221A6_LC | . | . | . | . | . | T | . | . | . | . | . | . | . | . | . | . | N | . | . | . | . | . | . | . | . | . |
| 21-225_221F12_LC | . | . | . | . | . | T | . | . | . | . | . | . | . | . | . | . | N | . | . | . | . | . | . | . | . | . |
| 21-225_222C12_LC | . | . | V | . | . | T | . | . | . | . | . | . | . | . | . | . | N | . | . | . | . | . | . | . | . | . |
| 21-225_222E1_LC | . | . | . | . | . | T | . | . | . | . | . | . | . | . | . | . | N | . | . | . | . | . | . | . | . | . |
| 21-225_222F7_LC | . | . | . | . | . | T | . | . | . | . | . | . | . | . | . | . | N | . | . | . | . | . | . | . | . | . |
| 21-225_23B11_LC | . | V | . | . | . | . | . | . | . | . | . | . | . | . | . | . | . | . | . | . | ⊗ | . | . | . | . | . |
| 21-225_25E11_LC | . | . | . | . | . | T | . | . | . | . | . | . | . | . | . | . | . | . | . | ⊗ | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | A | - | - | - | - | - | - | - | - | A | S | S | L | Q | S | G | V | P | S | R | F |
| 21-225_28B1_LC | . | . | . | . | . | . | . | | | | | | | | | . | . | P | . | . | . | . | . | . | . | . |
| 21-225_28F11_LC | . | . | . | . | S | . | . | | | | | | | | | . | N | . | . | . | . | . | . | . | . | . |
| 21-225_30A11_LC | S | . | . | . | . | . | . | | | | | | | | | . | R | . | . | . | . | S | . | . | . | . |
| 21-225_31C4_LC | . | P | . | . | . | . | . | | | | | | | | | . | . | . | . | . | . | . | . | T | . | . |
| 21-225_31H9_LC | . | . | . | . | . | D | . | | | | | | | | T | . | . | . | . | . | . | . | . | . | . | . |
| 21-225_33G1_LC | . | . | . | . | . | G | . | | | | | | | | . | Y | . | . | . | . | . | . | . | . | . | . |
| 21-225_33H7_LC | . | . | . | . | . | . | . | | | | | | | | . | . | R | . | . | . | . | . | . | . | . | . |
| 21-225_34F3_LC | . | . | . | . | . | . | . | | | | | | | | . | . | . | . | . | . | . | . | . | . | . | . |
| 21-225_34G7_LC | . | . | . | . | . | . | . | | | | | | | | . | . | . | . | . | . | . | . | . | L | . | . |
| 21-225_35C6_LC | . | P | . | . | . | . | . | | | | | | | | . | . | . | . | . | . | . | . | . | T | . | . |
| 21-225_35G6_LC | . | . | . | . | . | . | . | | | | | | | | . | . | R | . | . | . | . | . | . | . | . | . |
| 21-225_35H12_LC | S | . | . | . | . | . | . | | | | | | | | . | . | R | . | . | . | . | S | . | . | . | . |
| 21-225_36H5_LC | S | . | . | . | . | . | . | | | | | | | | . | . | R | . | . | . | . | S | . | . | . | . |
| 21-225_43H9_LC | . | . | . | . | . | D | . | | | | | | | | . | . | . | . | . | . | . | . | . | . | . | . |
| 21-225_44F3_LC | . | . | . | . | . | . | . | | | | | | | | . | . | . | . | . | . | . | . | . | . | . | . |
| 21-225_45C9_LC | . | . | . | . | . | . | . | | | | | | | | . | . | . | . | E | . | . | . | . | . | . | . |
| 21-225_45H4_LC | . | Y | . | . | . | D | . | | | | | | | | . | . | . | . | . | . | . | . | . | . | . | . |
| 21-225_49F1_LC | . | . | . | . | . | T | . | | | | | | | | V | . | . | . | . | . | . | . | . | . | . | . |
| 21-225_49F10_LC | . | V | . | . | . | . | . | | | | | | | | . | . | . | T | . | . | . | . | . | . | . | . |
| 21-225_49H5_LC | . | . | . | . | . | . | . | | | | | | | | . | . | . | . | . | D | . | . | . | . | . | . |
| 21-225_4H6_LC | . | . | . | . | . | G | . | | | | | | | | . | . | . | . | . | . | . | . | . | . | . | . |
| 21-225_4H6.004_LC | . | . | . | . | . | G | . | | | | | | | | . | . | . | . | . | . | . | . | . | . | . | . |
| 21-225_4H6.005_LC | . | . | . | . | . | G | . | | | | | | | | . | . | . | . | . | . | . | . | . | . | . | . |
| 21-225_4H6.006_LC | . | . | . | . | . | G | . | | | | | | | | . | . | . | . | . | . | . | . | . | . | . | . |
| 21-225_4H6.007_LC | . | . | . | . | . | G | . | | | | | | | | . | . | . | . | . | . | . | . | . | . | . | . |
| 21-225_4H6.008_LC | . | . | . | . | . | G | . | | | | | | | | . | . | . | . | . | . | . | . | . | . | . | . |
| 21-225_4H6.009_LC | . | . | . | . | . | G | . | | | | | | | | . | . | . | . | . | . | . | . | . | . | . | . |
| 21-225_4H6.010_LC | . | . | . | . | . | G | . | | | | | | | | . | . | . | . | . | . | . | . | . | . | . | . |
| 21-225_4H6.011_LC | . | . | . | . | . | G | . | | | | | | | | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
|  |  |  |  |  |  |  |  |  |  |  | K_CDR2 |  |  |  |  |  |  |  |  |  |  |  |  |  |  |  |
| CONSENSUS | K | L | L | I | Y | A | - | - | - | - | - | - | - | - | - | A | S | S | L | Q | S | G | V | P | S | R | F |
| 21-225_4H6.014_LC | . | . | . | . | . | S | . | . | . | . | . | . | . | . | . | . | . | . | . | . | . | . | . | . | . | . |
| 21-225_50D4_LC | . | V | . | . | . | . | . | . | . | . | . | . | . | . | . | T | . | . | . | . | . | . | . | . | . | . |
| 21-225_50F3_LC | . | . | . | . | . | . | . | . | . | . | . | . | . | . | . | . | . | N | . | . | . | . | . | . | . | . |
| 21-225_51C7_LC | . | . | . | . | . | . | . | . | . | . | . | . | . | . | . | . | . | N | . | . | . | . | . | . | . | . |
| 21-225_51E9_LC | . | . | . | . | . | V | . | . | . | . | . | . | . | . | . | . | . | . | . | . | . | . | . | . | . | . |
| 21-225_52B1_LC | . | . | . | . | . | . | . | . | . | . | . | . | . | . | . | . | . | N | . | . | . | . | . | . | . | . |
| 21-225_52G11_LC | . | . | . | L | . | . | . | . | . | . | . | . | . | . | . | . | . | . | . | . | . | . | . | . | . | . |
| 21-225_52H10_LC | . | . | . | . | . | V | . | . | . | . | . | . | . | . | . | . | . | . | . | . | . | A | . | . | . | . |
| 21-225_53F2_LC | N | . | . | L | . | . | . | . | . | . | . | . | . | . | . | . | . | . | . | . | . | . | . | . | X | . |
| 21-225_53F7_LC | N | . | . | . | . | . | . | . | . | . | . | . | . | . | . | . | . | . | . | . | . | . | . | . | . | . |
| 21-225_55B1_LC | N | . | . | . | . | . | . | . | . | . | . | . | . | . | . | . | . | . | . | . | . | . | . | . | . | . |
| 21-225_56C6_LC | . | . | . | . | . | . | . | . | . | . | . | . | . | . | . | . | R | . | . | . | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | F | . | . | . | V | . | . | . | . | . | . | . | . | . | . | G | . | . | . | . | . | . | . | . | . |
| 21-225_57A7_LC | N | . | . | . | . | . | . | . | . | . | . | . | . | . | . | . | N | . | . | . | . | . | . | . | . | . |
| 21-225_57E4_LC | . | . | . | . | . | . | . | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | . | S | . | . | . | . | . | . | . | . | . | . | . | . | . | . | . | . | . | . | . | . | . |
| 21-225_65F10_LC | . | . | . | . | . | . | . | . | . | . | . | . | . | . | . | . | I | . | . | . | . | . | . | . | . | . |
| 21-225_66F11_LC | . | . | . | . | . | . | . | . | . | . | . | . | . | . | . | . | . | . | . | . | . | . | . | . | . | . |
| 21-225_70D1_LC | . | . | . | . | . | . | . | . | . | . | . | . | . | . | . | . | . | . | . | . | . | . | . | . | . | . |
| 21-225_7C3_LC2_LC | . | . | . | . | F | . | . | . | . | . | . | . | . | . | . | . | R | . | . | . | . | A | . | . | . | . |
| 21-225_9F12_LC1_LC | . | . | . | . | . | . | . | . | . | . | . | . | . | . | . | . | R | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_10G6_LC | . | | | | . | . | . | . | . | . | . | . | . | . | . | ~ | . | . | . | . | . | . | . | . | . | . |
| 21-225_12E9_LC | . | | | | . | . | . | . | . | . | . | . | . | . | . | . | . | . | . | . | . | . | . | . | . | . |
| 21-225_146A6_LC | . | | | | . | . | . | . | . | . | . | . | . | S | . | . | . | . | . | . | . | . | . | . | . | . |
| 21-225_146E1_LC | ~ | | | | . | . | . | . | . | . | . | . | . | . | . | . | . | . | . | . | . | . | . | . | . | . |
| 21-225_147D10_LC | . | | | | . | . | . | . | . | . | . | . | . | S | . | . | . | . | . | . | . | . | . | . | . | . |
| 21-225_147D12_LC | . | | | | . | . | . | . | . | . | . | . | . | S | . | . | . | . | . | . | . | . | . | . | . | . |
| 21-225_148E2_LC | . | | N | ~ | . | . | . | . | . | . | . | . | . | S | . | . | . | . | . | . | . | . | . | . | . | . |
| 21-225_149B6_LC | . | | | | . | . | . | . | . | . | . | . | . | . | . | . | . | . | . | . | . | . | . | . | . | . |
| 21-225_149C6_LC | . | | | | . | . | . | . | . | Y | . | . | . | S | . | . | . | . | . | . | . | . | . | . | . | . |
| 21-225_149D11_LC | . | | | | . | . | . | . | . | . | . | . | . | S | . | . | . | . | . | . | . | . | . | . | . | . |
| 21-225_149F8_LC | . | | N | ~ | . | . | . | . | . | . | . | . | . | S | . | . | . | . | . | . | . | . | . | . | ! | . |
| 21-225_150C5_LC | . | | | | . | . | . | . | . | . | . | . | . | S | . | . | . | . | . | . | . | . | . | . | . | . |
| 21-225_152G10_LC | . | | N | ~ | . | . | . | . | . | . | . | . | . | S | . | . | . | . | . | . | . | . | . | . | . | . |
| 21-225_152H7_LC | . | | | | . | . | . | . | . | . | . | . | . | S | . | . | T | . | . | . | . | . | . | . | . | . |
| 21-225_155A4_LC | . | | | | . | . | . | . | . | . | . | . | . | S | . | . | . | . | . | . | . | . | . | . | . | . |
| 21-225_155B4_LC | N | | | | . | . | . | . | . | . | . | . | . | S | . | . | . | . | . | . | . | . | . | ~ | . | . |
| 21-225_180A3_LC | . | | | | . | . | . | . | . | . | ! | . | . | . | . | . | . | . | . | . | . | . | . | ~ | . | . |
| 21-225_182H5_LC | . | | | | . | . | . | . | . | . | ! | . | . | . | . | T | . | V | . | O | . | . | . | . | . | . |
| 21-225_186A11_LC | . | | | | . | . | . | . | . | . | ! | . | . | . | . | T | . | V | . | O | . | . | . | . | . | . |
| 21-225_192H2_LC | . | | | | . | . | . | . | . | . | . | . | . | . | . | . | . | . | . | . | . | . | . | . | . | . |
| 21-225_196F2_LC | . | | | | . | . | . | . | . | . | . | . | . | . | . | . | . | . | . | . | . | . | . | . | . | . |
| 21-225_210H11_LC | . | | ~ | | . | . | . | . | . | . | . | . | . | . | . | . | . | . | . | . | . | . | . | . | . | . |
| 21-225_221A6_LC | . | | | | . | . | . | . | . | . | . | . | . | . | . | . | . | . | . | . | . | . | . | . | . | . |
| 21-225_221F12_LC | . | | | | . | . | . | . | . | . | . | . | . | . | . | . | . | . | . | ~ | . | . | . | . | . | . |
| 21-225_222C12_LC | . | | | | . | . | . | . | . | . | . | . | . | . | . | . | . | V | . | ~ | . | . | . | . | . | . |
| 21-225_222E1_LC | . | | | | . | . | . | . | . | . | . | . | . | . | . | . | . | . | . | . | . | . | . | . | . | . |
| 21-225_222F7_LC | . | | | | . | . | . | . | . | . | . | . | . | . | . | . | . | V | . | . | . | . | . | . | . | . |
| 21-225_23B11_LC | . | | | ~ | . | . | . | . | . | . | . | . | . | . | . | . | . | . | . | . | . | . | . | . | . | . |
| 21-225_25E11_LC | . | | | | . | . | . | . | . | . | . | ! | . | S | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_28B1_LC | . | . | . | . | . | . | . | . | . | . | . | . | . | . | . | T | . | . | . | . | . | . | . | . | . | . |
| 21-225_28F11_LC | R | . | . | . | . | . | . | . | . | . | . | . | . | . | . | T | . | . | . | . | . | . | . | . | . | . |
| 21-225_30A11_LC | . | . | . | . | . | . | . | . | . | . | . | . | . | . | . | . | . | . | . | . | . | . | . | . | . | . |
| 21-225_31C4_LC | . | . | . | . | . | . | . | . | . | . | . | . | . | . | . | . | . | . | . | . | . | . | . | . | ? | . |
| 21-225_31H9_LC | . | . | . | . | . | . | . | . | . | . | ? | ? | . | . | . | . | . | . | . | . | . | . | . | . | . | . |
| 21-225_33G1_LC | . | . | . | . | . | . | . | . | . | . | . | . | . | . | . | . | . | . | . | . | . | . | . | . | . | . |
| 21-225_33H7_LC | . | . | . | . | . | . | . | . | . | . | . | . | . | . | . | G | . | . | . | . | . | . | . | . | . | . |
| 21-225_34F3_LC | . | . | . | . | . | . | . | . | . | . | . | . | . | . | . | . | . | . | . | . | . | . | S | . | . | . |
| 21-225_34G7_LC | . | . | . | . | . | . | . | . | . | . | . | . | . | . | . | . | . | . | . | . | . | . | . | . | . | . |
| 21-225_35C6_LC | . | . | . | . | . | . | . | . | . | . | . | . | . | . | . | . | . | . | . | . | . | . | . | . | ? | . |
| 21-225_35G6_LC | . | . | . | . | . | . | . | . | A | . | . | . | . | . | . | G | . | . | . | . | . | . | . | . | . | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | . | . | . | . | . | . | . | . | . | . | ? | . | . | . | . | . | . | . | . | . | . | . | . | . | . | . |
| 21-225_44F3_LC | . | . | . | S | . | . | . | . | . | . | . | . | . | . | . | . | . | . | . | . | . | . | . | . | . | . |
| 21-225_45C9_LC | . | . | . | . | . | . | . | . | . | . | . | . | . | . | . | . | . | . | . | . | . | . | . | . | . | . |
| 21-225_45H4_LC | . | . | . | . | . | . | . | . | . | . | ? | . | . | . | . | . | . | . | . | . | . | . | . | . | ? | . |
| 21-225_49F1_LC | . | . | . | . | . | . | . | . | . | . | . | . | . | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | . | . | . | . | . | . | . | . | . | . | . | . | . | . | . | . | . | . | . | . | . | . |
| 21-225_4H6_LC | . | . | . | . | . | . | . | . | . | . | . | . | . | . | . | . | . | . | . | . | . | . | . | . | ? | . |
| 21-225_4H6.004_LC | . | . | . | . | . | . | . | . | . | . | . | . | . | . | . | . | . | . | . | . | . | . | . | . | ? | . |
| 21-225_4H6.005_LC | . | . | . | . | . | . | . | . | . | . | . | . | . | . | . | . | . | . | . | . | . | . | . | . | ? | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | ? | . |
| 21-225_4H6.008_LC | . | . | . | . | . | . | . | . | . | . | . | . | . | . | . | . | . | . | . | . | . | . | . | . | ? | . |
| 21-225_4H6.009_LC | . | . | . | . | . | . | . | . | . | . | . | . | . | . | . | . | . | . | . | . | . | . | . | . | ? | . |
| 21-225_4H6.010_LC | . | . | . | . | . | . | . | . | . | . | . | . | . | . | . | . | . | . | . | . | . | . | . | . | ? | . |
| 21-225_4H6.011_LC | . | . | . | . | . | . | . | . | . | . | . | . | . | . | . | . | . | . | . | . | . | . | . | . | ? | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | D | F | T | L | T | I | S | S | L | Q | P | E | D | F | A | T | Y |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | . | . | . | . | . | . | . | . |
| 21-225_50F3_LC | . | . | . | . | . | . | . | . | . | . | . | . | . | . | . | . | . | . | . | . | . | . | . | . | . | . |
| 21-225_51C7_LC | . | . | . | . | . | . | . | . | . | . | . | . | . | . | . | . | . | L | . | . | . | . | . | . | . | . |
| 21-225_51E9_LC | . | . | . | . | . | . | . | . | . | . | . | . | . | . | . | R | . | . | . | . | . | . | . | . | . | . |
| 21-225_52B1_LC | . | . | . | . | . | . | . | . | . | . | . | . | . | . | . | . | . | . | . | . | . | . | . | . | . | . |
| 21-225_52G11_LC | . | A | . | . | . | . | . | . | . | . | . | . | . | . | . | . | . | . | . | . | . | . | . | . | . | . |
| 21-225_52H10_LC | . | . | . | . | . | . | . | . | . | . | . | . | . | . | . | R | . | . | . | . | . | . | . | . | . | . |
| 21-225_53F2_LC | . | . | . | . | . | . | . | . | . | . | . | . | . | . | . | . | . | . | . | . | . | . | . | . | . | . |
| 21-225_53F7_LC | . | . | . | . | . | . | . | . | . | . | . | . | . | . | . | . | . | . | . | . | . | . | . | . | I | . |
| 21-225_55B1_LC | . | . | . | . | . | . | . | . | . | . | . | . | . | . | . | . | . | . | . | . | . | . | . | . | . | . |
| 21-225_56C6_LC | . | . | . | . | . | . | . | . | Y | . | . | . | I | . | . | . | . | . | . | S | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | . | . | . | . | . | . | . | . | . | . | . | R | . | . | . | . | . | . | . | . | . | . | . | . | . |
| 21-225_57A7_LC | . | . | . | . | . | . | . | . | . | . | . | . | . | . | . | . | . | . | . | . | . | . | . | . | . | . |
| 21-225_57E4_LC | . | . | . | . | . | . | . | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | . | . | . | . | . | . | . | . | . | . | . | . | . | . | . | . | . | . | . | . | . | . | . |
| 21-225_65F10_LC | R | . | R | . | . | . | . | . | . | . | . | . | . | . | . | . | . | . | . | . | . | . | . | . | . | . |
| 21-225_66F11_LC | . | . | . | . | Y | . | . | . | . | . | . | . | . | . | . | . | . | . | V | . | . | . | . | . | . | . |
| 21-225_70D1_LC | . | . | . | . | . | . | . | . | . | . | . | . | V | . | . | . | . | . | . | . | . | . | . | . | . | . |
| 21-225_7C3_LC2_LC | . | . | . | . | . | . | . | . | . | . | . | . | . | . | . | . | . | . | . | . | . | . | . | . | . | . |
| 21-225_9F12_LC1_LC | . | . | . | . | . | . | . | . | . | . | . | . | . | . | . | . | . | G | . | L | . | . | . | . | I | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | A | N | S | - | - | - | - | - | - | - | - | - | - | - | - | - | - | - | - | - | - | - |
| 21-225_10G6_LC | . | | | | | | | | | | | | | | | | | | | | | | | | | |
| 21-225_12E9_LC | . | | | | | . | | | | | | | | | | | | | | | | | | | | |
| 21-225_146A6_LC | . | | | . | T | D | . | | | | | | | | | | | | | | | | | | | |
| 21-225_146E1_LC | . | | | | | . | | | | | | | | | | | | | | | | | | | | |
| 21-225_147D10_LC | . | | | | T | D | . | | | | | | | | | | | | | | | | | | | |
| 21-225_147D12_LC | . | | | | T | D | . | | | | | | | | | | | | | | | | | | | |
| 21-225_148E2_LC | . | | | | T | D | . | | | | | | | | | | | | | | | | | | | |
| 21-225_149B6_LC | . | | | . | G | . | | | | | | | | | | | | | | | | | | | | |
| 21-225_149C6_LC | . | | | . | T | D | . | | | | | | | | | | | | | | | | | | | |
| 21-225_149D11_LC | . | | | | T | D | . | | | | | | | | | | | | | | | | | | | |
| 21-225_149F8_LC | . | | | | T | D | . | | | | | | | | | | | | | | | | | | | |
| 21-225_150C5_LC | . | | | . | T | D | . | | | | | | | | | | | | | | | | | | | |
| 21-225_152G10_LC | . | | | . | T | D | . | | | | | | | | | | | | | | | | | | | |
| 21-225_152H7_LC | . | | . | . | T | D | . | | | | | | | | | | | | | | | | | | | |
| 21-225_155A4_LC | . | . | X | . | T | D | . | | | | | | | | | | | | | | | | | | | |
| 21-225_155B4_LC | . | . | X | . | T | D | . | | | | | | | | | | | | | | | | | | | |
| 21-225_180A3_LC | X | | | | . | | | | | | | | | | | | | | | | | | | | | |
| 21-225_182H5_LC | . | | . | . | V | . | | | | | | | | | | | | | | | | | | | | |
| 21-225_186A11_LC | . | | . | . | V | . | | | | | | | | | | | | | | | | | | | | |
| 21-225_192H2_LC | . | | . | . | S | . | | | | | | | | | | | | | | | | | | | | |
| 21-225_196F2_LC | . | | . | . | T | . | | | | | | | | | | | | | | | | | | | | |
| 21-225_210H11_LC | . | | . | . | X | . | | | | | | | | | | | | | | | | | | | | |
| 21-225_221A6_LC | . | | . | . | . | | | | | | | | | | | | | | | | | | | | | |
| 21-225_221F12_LC | . | | . | . | . | | | | | | | | | | | | | | | | | | | | | |
| 21-225_222C12_LC | . | | . | . | G | . | | | | | | | | | | | | | | | | | | | | |
| 21-225_222E1_LC | . | | . | . | . | | | | | | | | | | | | | | | | | | | | | |
| 21-225_222F7_LC | . | | . | . | . | | | | | | | | | | | | | | | | | | | | | |
| 21-225_23B11_LC | . | | . | . | . | | | | | | | | | | | | | | | | | | | | | |
| 21-225_25E11_LC | . | | . | . | . | | | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | A | N | S | - | - | - | - | - | - | - | - | - | - | - | - | - | - | - | - | - | - | - |
| 21-225_28B1_LC | . | . | . | . | . | S | . | | | | | | | | | | | | | | | | | | | |
| 21-225_28F11_LC | C | . | . | . | . | . | . | | | | | | | | | | | | | | | | | | | |
| 21-225_30A11_LC | . | . | . | . | . | . | . | | | | | | | | | | | | | | | | | | | |
| 21-225_31C4_LC | . | . | . | . | . | . | . | | | | | | | | | | | | | | | | | | | |
| 21-225_31H9_LC | . | . | . | . | G | . | . | | | | | | | | | | | | | | | | | | | |
| 21-225_33G1_LC | . | . | . | . | . | . | . | | | | | | | | | | | | | | | | | | | |
| 21-225_33H7_LC | . | . | . | . | . | I | . | | | | | | | | | | | | | | | | | | | |
| 21-225_34F3_LC | . | . | . | . | . | . | . | | | | | | | | | | | | | | | | | | | |
| 21-225_34G7_LC | . | . | . | . | . | . | . | | | | | | | | | | | | | | | | | | | |
| 21-225_35C6_LC | . | . | . | . | . | . | . | | | | | | | | | | | | | | | | | | | |
| 21-225_35G6_LC | . | . | . | . | . | . | . | | | | | | | | | | | | | | | | | | | |
| 21-225_35H12_LC | . | . | . | . | . | . | . | | | | | | | | | | | | | | | | | | | |
| 21-225_36H5_LC | . | . | . | . | . | . | . | | | | | | | | | | | | | | | | | | | |
| 21-225_43H9_LC | F | . | . | . | . | . | . | | | | | | | | | | | | | | | | | | | |
| 21-225_44F3_LC | . | . | . | . | . | . | . | | | | | | | | | | | | | | | | | | | |
| 21-225_45C9_LC | . | . | . | . | . | . | . | | | | | | | | | | | | | | | | | | | |
| 21-225_45H4_LC | F | . | . | . | . | . | . | | | | | | | | | | | | | | | | | | | |
| 21-225_49F1_LC | . | . | . | . | S | . | . | | | | | | | | | | | | | | | | | | | |
| 21-225_49F10_LC | . | . | . | . | . | . | . | | | | | | | | | | | | | | | | | | | |
| 21-225_49H5_LC | . | . | . | . | T | . | . | | | | | | | | | | | | | | | | | | | |
| 21-225_4H6_LC | . | . | . | . | . | . | . | | | | | | | | | | | | | | | | | | | |
| 21-225_4H6.004_LC | . | . | . | . | . | . | . | | | | | | | | | | | | | | | | | | | |
| 21-225_4H6.005_LC | . | . | . | . | . | . | . | | | | | | | | | | | | | | | | | | | |
| 21-225_4H6.006_LC | . | . | . | . | . | . | . | | | | | | | | | | | | | | | | | | | |
| 21-225_4H6.007_LC | . | . | . | . | . | . | . | | | | | | | | | | | | | | | | | | | |
| 21-225_4H6.008_LC | . | . | . | . | . | . | . | | | | | | | | | | | | | | | | | | | |
| 21-225_4H6.009_LC | . | . | . | . | . | . | . | | | | | | | | | | | | | | | | | | | |
| 21-225_4H6.010_LC | . | . | . | . | . | . | . | | | | | | | | | | | | | | | | | | | |
| 21-225_4H6.011_LC | . | . | . | . | . | . | . | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | A | N | S | - | - | - | - | - | - | - | - | - | - | - | - | - | - | - | - | - | - | - |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | . | . | . | . | . | . | . | . |
| 21-225_50F3_LC | . | . | . | . | T | . | . | . | . | . | . | . | . | . | . | . | . | . | . | . | . | . | . | . | . | . |
| 21-225_51C7_LC | . | . | . | . | T | . | . | . | . | . | . | . | . | . | . | . | . | . | . | . | . | . | . | . | . | . |
| 21-225_51E9_LC | . | . | . | . | . | K | . | . | . | . | . | . | . | . | . | . | . | . | . | . | . | . | . | . | . | . |
| 21-225_52B1_LC | F | . | . | . | . | K | . | . | . | . | . | . | . | . | . | . | . | . | . | . | . | . | . | . | . | . |
| 21-225_52G11_LC | . | . | . | . | . | . | . | . | . | . | . | . | . | . | . | . | . | . | . | . | . | . | . | . | . | . |
| 21-225_52H10_LC | . | . | . | . | . | K | . | . | . | . | . | . | . | . | . | . | . | . | . | . | . | . | . | . | . | . |
| 21-225_53F2_LC | . | . | . | . | . | . | . | . | . | . | . | . | . | . | . | . | . | . | . | . | . | . | . | . | . | . |
| 21-225_53F7_LC | . | . | . | . | . | . | . | . | . | . | . | . | . | . | . | . | . | . | . | . | . | . | . | . | . | . |
| 21-225_55B1_LC | . | . | . | . | . | . | . | . | . | . | . | . | . | . | . | . | . | . | . | . | . | . | . | . | . | . |
| 21-225_56C6_LC | . | . | . | . | . | . | . | . | . | . | . | . | . | . | . | . | . | . | . | . | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | . | . | . | . | . | . | . | . | . | . | . | . | . | . | . | . | . | . | . | . | . | . | . | . | . |
| 21-225_57A7_LC | . | . | . | . | . | . | . | . | . | . | . | . | . | . | . | . | . | . | . | . | . | . | . | . | . | . |
| 21-225_57E4_LC | S | . | . | . | Q | . | . | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | I | . | . | . | . | . | . | . | . | . | . | . | . | . | . | . | . | . | . | . | . | . | . | . | . | . |
| 21-225_65F10_LC | . | . | . | . | Q | . | I | . | . | . | . | . | . | . | . | . | . | . | . | . | . | . | . | . | . | . |
| 21-225_66F11_LC | . | . | . | . | . | . | . | . | . | . | . | . | . | . | . | . | . | . | . | . | . | . | . | . | . | . |
| 21-225_70D1_LC | . | . | . | . | . | . | . | . | . | . | . | . | . | . | . | . | . | . | . | . | . | . | . | . | . | . |
| 21-225_7C3_LC2_LC | . | . | . | . | . | . | . | . | . | . | . | . | . | . | . | . | . | . | . | . | . | . | . | . | . | . |
| 21-225_9F12_LC1_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | F | P | F | T | F | G | P | G | T | K | V | D | J | K | R |
| 21-225_10G6_LC | | | | | | | | | | | | | | | | | | | |
| 21-225_12E9_LC | | | | | | | | | | | | | | | | | | | |
| 21-225_146A6_LC | | | | | | | | | | | | | | | | | | | |
| 21-225_146E1_LC | | | | | | | | | | | | | | | | | | | |
| 21-225_147D10_LC | | | | | | | | | | | | | | | | | V | | |
| 21-225_147D12_LC | | | | | | | | | | | | | | | | | V | | |
| 21-225_148E2_LC | | | | | | | | | | | | | | | | | V | | |
| 21-225_149B6_LC | | | | | | | | | | | | | | | | | | | |
| 21-225_149C6_LC | | | | | | | | | | | | | | | | | | | |
| 21-225_149D11_LC | | | | | | | | | | | | | | | | | V | | |
| 21-225_149F8_LC | | | | | | | | | | | | | | | | | V | | |
| 21-225_150C5_LC | | | | | | | | | | | | | | | | | | | |
| 21-225_152G10_LC | | | | | | | | | | | | | | | | | V | | |
| 21-225_152H7_LC | | | | | | | | | | | | | | | | | | | |
| 21-225_155A4_LC | | | | | | | | | | | | | | | | | | | |
| 21-225_155B4_LC | | | | | | | | | | | | | | | | | | | |
| 21-225_180A3_LC | | | | | I | | | | | | | | | | | | | N | |
| 21-225_182H5_LC | | | | | | | | | | X | | | | | | | | | |
| 21-225_186A11_LC | | | | | | | | | | X | | | | | | | | | |
| 21-225_192H2_LC | | | | | | | | | | | | | | | | | V | | |
| 21-225_196F2_LC | | | | V | | | | | | | | | | | | | | | |
| 21-225_210H11_LC | | | | | | | | | | | | | | | | | | | |
| 21-225_221A6_LC | | | | | | | | | | | | | | | | | | | |
| 21-225_221F12_LC | | | | | | | | | | | | | | | | | | | |
| 21-225_222C12_LC | | | | | | | | | | X | | | | | | | | | |
| 21-225_222E1_LC | | | | | | | | | | X | | | | | | | | | |
| 21-225_222F7_LC | | | | | | | | | | | | | | | | | | | |
| 21-225_23B11_LC | | | | | | | | | | | | | | | | | | | |
| 21-225_25E11_LC | | | | | | I | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | F | P | F | T | F | G | P | G | T | K | V | D | I | K | R |
| 21-225_28B1_LC | | | | | | | | | | | | | | | | | | | |
| 21-225_28F11_LC | | | | | L | | | | | | | | | | | | | | |
| 21-225_30A11_LC | | | | | | | | | | | | | | | | | N | | |
| 21-225_31C4_LC | | | | | | | | | | | | | | | | | | | |
| 21-225_31H9_LC | | | | | | | | | | | Q | | | | | | | | |
| 21-225_33G1_LC | | | | | | | | | | | | | | | | | | | |
| 21-225_33H7_LC | | | | | | | | | | | | | | | | | L | | |
| 21-225_34F3_LC | | | | | | | | | | | | | | | | | | | |
| 21-225_34G7_LC | | | | | | | | | | | | | | | | | | | |
| 21-225_35C6_LC | | | | | | | | | | | | | | | | | | | |
| 21-225_35G6_LC | | | | | | | | | | | | | | | | | L | | |
| 21-225_35H12_LC | | | | | | | | | | | | | | | | | | | |
| 21-225_36H5_LC | | | | | | | | | | | | | | | | | | | |
| 21-225_43H9_LC | | | | | L | | | | | | | | | | | | | | |
| 21-225_44F3_LC | | | | | | | | | | | | | | | | | | | |
| 21-225_45C9_LC | | | | | | | | | | | | | | | | | | | |
| 21-225_45H4_LC | | | | | L | | | | | | | | | | | | | | |
| 21-225_49F1_LC | | | | | | | | | | | | | | | | | | | |
| 21-225_49F10_LC | | | | | | | | | | | | | | | | | | | |
| 21-225_49H5_LC | | | | | | | | | | | | | | | | | | | |
| 21-225_4H6_LC | | | | | | | | | | | | | | | | | | | ⊗ |
| 21-225_4H6.004_LC | | | | | | | | | | | | | | | | | | | |
| 21-225_4H6.005_LC | | | | | | | | | | | Q | | | | | | | | |
| 21-225_4H6.006_LC | | | | | | | | | | | Q | | | | | | | | |
| 21-225_4H6.007_LC | | | | | | | | | | | | | | | | | | | |
| 21-225_4H6.008_LC | | | | | | | | | | | | | | | | | | | |
| 21-225_4H6.009_LC | | | | | | | | | | | Q | | | | | | | | |
| 21-225_4H6.010_LC | | | | | | | | | | | Q | | | | | | | | |
| 21-225_4H6.011_LC | | | | | | | | | | | Q | | | | | | | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | F | P | F | T | F | G | P | G | T | K | V | D | I | K | R |
| 21-225_4H6.014_LC | . | . | . | . | . | . | . | . | . | . | . | . | . | . | . | . | . | . | . |
| 21-225_50D4_LC | . | . | . | . | . | . | . | . | . | . | . | . | . | . | . | . | . | R | . |
| 21-225_50F3_LC | . | . | . | . | . | . | . | . | . | . | . | . | . | . | . | . | . | . | . |
| 21-225_51C7_LC | . | . | . | . | . | . | . | . | . | . | . | . | . | . | . | . | . | . | . |
| 21-225_51E9_LC | . | . | . | . | . | . | . | . | . | . | . | . | . | . | . | . | . | . | . |
| 21-225_52B1_LC | . | . | . | . | . | . | . | . | . | . | . | . | . | . | . | . | . | . | . |
| 21-225_52G11_LC | . | . | . | . | . | . | . | . | . | . | . | . | . | . | . | . | V | . | . |
| 21-225_52H10_LC | . | . | . | . | . | . | . | . | . | . | . | . | . | . | . | . | . | . | . |
| 21-225_53F2_LC | . | . | . | . | . | . | . | . | . | . | . | . | . | . | . | . | V | . | . |
| 21-225_53F7_LC | . | . | . | . | . | . | . | . | . | . | . | . | . | . | . | . | . | . | . |
| 21-225_55B1_LC | . | . | . | . | . | . | . | . | . | . | . | . | . | . | . | . | L | . | . |
| 21-225_56C6_LC | . | . | . | . | . | . | . | . | . | . | . | . | . | . | . | . | . | . | . |
| 21-225_56E5_LC | . | . | . | . | . | . | . | . | . | . | . | . | . | . | . | . | . | . | . |
| 21-225_56F6_LC | . | . | . | . | . | . | . | . | . | . | . | . | . | . | . | . | . | . | . |
| 21-225_57A7_LC | . | . | . | . | . | . | . | . | . | . | . | . | . | . | . | . | . | . | . |
| 21-225_57E4_LC | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . |
| 21-225_64G12_LC | . | . | . | . | . | . | . | . | . | . | . | . | . | L | . | . | . | . | . |
| 21-225_65F10_LC | . | . | . | . | . | . | . | . | . | . | . | . | . | . | . | . | . | . | . |
| 21-225_66F11_LC | . | . | . | . | . | . | . | . | . | . | . | . | . | . | . | . | . | . | . |
| 21-225_70D1_LC | . | . | . | . | . | . | . | . | . | . | . | . | . | . | . | . | . | . | . |
| 21-225_7C3_LC2_LC | . | . | . | . | . | . | . | . | . | . | . | . | . | . | . | . | . | . | . |
| 21-225_9F12_LC1_LC | . | . | . | . | . | . | . | . | . | . | . | . | . | . | . | . | P | . | . |

**FIGURE 57**

Table 105.     Consensus 25 - VK2|A18/JK1 (SEQ ID NO: 50322):

DIVMTQTPLSLSVTPGQPASISC<u>KSS--QSLLHGD-GKTYLY</u>WYLQKPGQPPQLLIY<u>E-------VSNRF</u>SGVPDRFSGSGSG--
TDFTLKISRVEAEDVGVYYC<u>MQSIQ--------------------LPWT</u>FGQGTKVEIKR wherein:

K at position 24 can be substituted with M, R or T

S at position 26 can be substituted with G or T

Q at position 29 can be substituted with K

S at position 30 can be substituted with R, N or T

L at position 32 can be substituted with R or V

H at position 33 can be substituted with Y

G at position 34 can be substituted with S

D at position 35 can be substituted with E or G

K at position 38 can be substituted with R

Y at position 40 can be substituted with F

L at position 41 can be substituted with F

Y at position 42 can be substituted with F, T, C or S

E at position 58 can be substituted with A

V at position 67 can be substituted with I, L or T

N at position 69 can be substituted with K, H, I or S

F at position 71 can be substituted with L

S at position 72 can be substituted with A, T, C or P

FIGURE 57

M at position 107 can be substituted with K

S at position 109 can be substituted with T

I at position 110 can be substituted with T, F or L

Q at position 111 can be substituted with H

L at position 135 can be substituted with I, V or F

W at position 137 can be substituted with R

Xaa Ser Xaa Xaa Xaa Leu Xaa Xaa Xaa Xaa Gly Xaa Thr Xaa Xaa Xaa (SEQ ID NO: 50150)

Xaa Xaa Ser Xaa Arg Xaa Xaa (SEQ ID NO: 50151)

Xaa Gln Xaa Xaa Xaa Xaa Pro Xaa Thr (SEQ ID NO: 50152)

Lys Ser Ser Gln Ser Leu Leu His Gly Asp Gly Lys Thr Tyr Leu Tyr (SEQ ID NO: 50636)

Glu Val Ser Asn Arg Phe Ser (SEQ ID NO: 50637)

Met Gln Ser Ile Gln Leu Pro Trp Thr (SEQ ID NO: 50638)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | K_FR1 | | | | | | | | | | | | | | | | | | | |
| CONSENSUS | D | I | V | M | T | Q | T | P | L | S | L | S | V | T | P | G | Q | P | A | S | I | S | C | K | S | S |
| 21-225_146B3_LC | . | . | . | . | . | . | . | . | . | . | . | . | . | . | . | . | . | . | . | . | . | . | . | . | . | . |
| 21-225_147E10_LC | A | . | . | . | . | . | . | . | . | . | . | . | . | . | . | . | . | . | . | . | . | . | . | . | . | . |
| 21-225_148B2_LC | A | . | . | . | . | . | . | . | . | . | . | . | . | . | . | . | . | . | . | . | . | . | . | . | . | . |
| 21-225_148G10_LC | . | . | . | . | . | . | . | . | . | . | . | . | . | . | . | . | . | . | . | . | . | . | . | . | . | . |
| 21-225_149F1_LC | . | . | . | . | . | . | S | . | . | . | . | F | . | . | . | . | . | . | . | . | . | . | Y | . | . | . |
| 21-225_150B11_LC | . | . | . | . | . | . | . | . | . | . | . | . | . | . | . | . | . | . | . | . | . | . | . | . | . | . |
| 21-225_151A10_LC | . | . | . | . | . | . | . | . | . | . | . | . | . | . | . | . | . | . | . | . | . | . | . | . | . | . |
| 21-225_151G5_LC | . | . | . | . | . | . | . | . | . | . | . | . | . | . | . | . | . | . | . | . | . | . | . | . | . | . |
| 21-225_152C11_LC | . | . | . | . | . | . | A | . | . | . | . | . | . | . | . | . | . | . | . | . | . | . | . | . | . | . |
| 21-225_152F6_LC | . | . | . | . | . | . | . | . | . | . | . | . | . | . | . | . | . | . | . | . | . | . | . | . | . | . |
| 21-225_153D2_LC | . | . | . | . | . | . | . | . | . | . | . | . | . | . | . | . | . | . | . | . | . | . | . | . | . | . |
| 21-225_155A5_LC | . | . | . | . | . | . | . | . | . | . | . | . | . | . | . | . | . | . | . | . | . | . | . | . | . | . |
| 21-225_157G8_LC | . | . | . | . | . | . | . | . | . | . | . | . | . | . | . | . | . | . | . | . | . | . | . | . | . | . |
| 21-225_160B1_LC | . | . | . | . | . | . | . | . | . | . | . | . | . | . | . | . | . | . | . | . | . | . | . | . | . | . |
| 21-225_160G10_LC | A | . | . | . | . | . | . | . | . | . | . | . | . | . | . | . | . | . | . | . | . | . | . | . | . | Y |
| 21-225_162H6_LC | . | . | . | . | . | . | . | . | . | . | . | . | . | . | . | . | . | . | . | . | . | . | . | . | . | Y |
| 21-225_167E2_LC | . | . | . | . | . | . | . | . | . | . | . | . | . | . | . | . | . | . | . | . | . | . | . | . | . | . |
| 21-225_167H10_LC | . | . | . | . | . | . | . | . | . | . | . | . | . | . | . | . | . | . | . | . | . | . | . | . | . | . |
| 21-225_170A1_LC | S | . | . | . | . | . | . | . | F | . | . | . | . | . | . | . | . | . | . | . | . | . | . | . | . | . |
| 21-225_172B7_LC | . | . | . | . | . | . | . | . | . | . | . | . | . | . | . | . | . | . | . | . | . | . | . | . | . | . |
| 21-225_173A11_LC | . | . | . | . | . | . | . | . | . | . | . | . | . | . | . | . | . | . | . | . | . | . | . | . | . | . |
| 21-225_176H4_LC | . | . | . | . | . | . | . | . | . | . | . | . | . | . | . | . | . | . | . | . | . | . | . | . | . | . |
| 21-225_177B11_LC | . | . | . | . | . | . | . | . | . | . | . | . | . | . | . | . | . | . | . | . | . | . | . | . | . | . |
| 21-225_178G10_LC | . | . | . | . | . | . | . | . | . | . | . | . | . | . | . | . | . | . | . | . | . | . | . | . | . | . |
| 21-225_180C4_LC | . | . | . | . | . | . | . | . | . | . | . | . | . | . | . | . | . | . | . | . | . | . | . | . | . | . |
| 21-225_181C10_LC | . | . | . | . | . | . | . | . | . | . | . | . | . | . | . | . | . | . | . | . | . | . | . | . | . | . |
| 21-225_181C2_LC | E | . | . | . | . | . | . | . | . | . | . | . | . | . | . | . | . | . | . | . | . | . | . | . | . | . |
| 21-225_186F7_LC | . | V | . | . | A | . | . | . | . | . | . | . | . | . | . | . | . | . | . | . | . | . | . | . | . | . |
| 21-225_188G11_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | V | M | T | Q | T | P | L | S | L | S | V | T | P | G | Q | P | A | S | I | S | C | K | S | S |
| 21-225_189G2_LC | . | . | . | . | . | . | . | . | . | . | . | . | . | . | . | . | . | . | . | . | . | . | . | . | . | . |
| 21-225_190E7_LC | . | . | . | L | . | . | . | . | . | . | . | . | . | . | . | . | . | . | . | . | . | . | . | . | . | . |
| 21-225_190H11_LC | . | . | . | . | . | . | . | . | . | . | . | . | . | . | . | . | . | . | . | . | . | . | . | . | . | . |
| 21-225_191B1_LC | . | . | . | . | . | . | . | . | . | . | . | . | . | . | . | . | . | . | . | . | . | . | . | * | . | . |
| 21-225_191E3_LC | . | . | . | . | . | . | . | . | . | . | . | . | . | . | . | . | . | . | . | . | . | . | . | . | . | . |
| 21-225_191E6_LC | . | . | . | . | . | . | . | . | . | . | . | . | . | . | . | . | . | . | . | . | . | . | . | . | . | . |
| 21-225_192F9_LC | . | . | . | . | . | . | . | . | . | . | . | . | . | . | . | . | . | . | . | . | . | . | . | . | . | . |
| 21-225_194F4_LC | . | . | . | . | . | . | . | . | . | . | . | . | . | . | . | . | . | . | . | . | . | . | . | . | . | . |
| 21-225_197G8_LC | E | . | . | . | . | . | . | . | . | . | . | . | I | . | . | . | . | . | . | . | . | . | . | . | . | . |
| 21-225_198A10_LC | . | . | . | L | . | . | . | . | . | . | . | . | . | . | . | . | . | . | . | . | . | . | . | . | . | . |
| 21-225_224A7_LC | . | . | . | . | . | . | . | . | . | . | . | . | . | . | . | . | . | . | . | . | . | . | . | . | . | . |
| 21-225_224C11_LC | . | N | . | . | . | . | . | . | . | . | . | . | . | . | . | . | . | . | . | . | . | . | . | . | . | . |
| 21-225_224G1_LC | . | . | . | . | . | . | . | . | . | . | . | . | . | . | . | . | . | . | . | . | . | . | . | . | . | G |
| 21-225_224H11_LC | . | . | . | . | . | . | . | . | . | . | . | . | . | . | . | . | . | . | . | . | . | . | . | . | . | . |
| 21-225_225G4_LC | . | . | . | . | . | . | . | . | . | . | . | . | . | . | . | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | . | . | . | . | . | . |
| 21-225_226E11_LC | . | . | . | . | . | . | . | . | . | . | . | . | . | . | . | . | . | . | . | . | . | . | . | . | . | . |
| 21-225_226E7_LC | . | . | . | . | . | . | . | . | . | . | . | . | . | . | . | . | . | . | . | . | . | . | . | . | . | . |
| 21-225_226F10_LC | . | . | . | . | . | . | . | . | . | . | . | . | . | . | . | . | . | . | . | . | . | . | . | . | . | . |
| 21-225_226F9_LC | . | . | . | . | . | . | . | . | . | . | . | . | . | . | . | . | . | . | . | . | . | . | . | . | . | . |
| 21-225_226G8_LC | . | . | . | . | . | . | . | . | . | . | . | . | . | . | . | . | . | . | . | . | . | . | . | . | . | . |
| 21-225_226H12_LC | . | . | . | . | . | . | . | . | . | . | . | . | . | . | . | . | . | . | . | . | . | . | . | . | . | . |
| 21-225_226H9_LC | . | . | . | . | . | . | . | . | . | . | . | . | I | . | . | . | . | . | . | . | . | . | . | . | . | . |
| 21-225_227A8_LC | . | . | . | . | . | . | . | . | . | . | . | . | . | . | . | . | . | . | . | . | . | . | . | . | . | . |
| 21-225_227C1_LC | . | . | . | . | . | . | . | . | . | . | . | . | . | . | . | . | . | . | . | . | . | . | . | . | . | . |
| 21-225_227F2_LC | . | . | . | . | . | . | . | . | . | . | . | . | . | . | . | . | . | . | . | . | . | . | . | . | . | . |
| 21-225_22A1_LC | . | . | . | . | . | . | . | . | . | . | . | . | . | . | . | . | . | . | . | . | . | . | . | . | . | . |
| 21-225_25H9_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | T | P | L | S | L | S | V | T | P | G | Q | P | A | S | I | S | C | K | S | S |
| 21-225_26F8_LC | . | . | . | . | . | . | . | . | . | . | . | . | . | . | . | . | . | . | . | . | . | . | . | . | . | . |
| 21-225_28G3_LC | E | . | . | . | . | . | . | . | . | . | . | . | . | . | . | . | . | . | . | . | . | . | . | . | . | . |
| 21-225_43E11_LC | . | . | . | . | . | . | . | . | . | . | . | . | . | . | . | . | . | . | . | . | . | . | . | . | . | . |
| 21-225_46F2_LC | . | . | . | . | . | . | . | . | . | . | . | T | . | . | . | . | . | . | . | . | . | . | . | . | . | . |
| 21-225_47C1_LC | . | . | . | . | . | . | . | . | . | . | . | . | . | . | . | . | . | . | . | . | . | . | . | T | . | . |
| 21-225_47E7_LC | . | . | . | . | . | . | . | . | . | . | . | . | . | . | . | . | . | . | . | . | . | . | . | . | . | . |
| 21-225_64H9_LC | E | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | L | L | H | G | D | - | G | K | T | Y | L | Y | W | Y | L | Q | K | P | G | Q | P | P |
| 21-225_146B3_LC | | | | | | | | | | | | | | | | | | | | | | | | | | |
| 21-225_147E10_LC | | | | R | | | | | | | | | | | | | | | | | | | | | | |
| 21-225_148B2_LC | | | | | | | | | | | | | | | | P | | | | | | | | | | |
| 21-225_148G10_LC | | | | | | | | | | | | | | | | | | | | | R | | | | | |
| 21-225_149F1_LC | | | | | | | | | | | | | | | | P | | | | | | | | | | |
| 21-225_150B11_LC | | | | | | V | | | | | | | | | | | | | | | R | | | | | |
| 21-225_151A10_LC | | | | | | | | | | | | | | | | | | | | | | | | | | |
| 21-225_151G5_LC | | | | R | | | | | | | | | | | | | | | | | | | | | | |
| 21-225_152C11_LC | | | | | | | | | | | | | | | | | | | | | | | | | | |
| 21-225_152F6_LC | | | | | | R | | | | | | | | | T | | | | | | R | | | | | |
| 21-225_153D2_LC | | | | | | R | | | | | | | | | T | | | | | | R | | | | | |
| 21-225_155A5_LC | | | | | | | | | | | | | | | | | | | | | | | | | | |
| 21-225_157G8_LC | | | | | | | | | | | | | | | | | | | | | | | | | S | |
| 21-225_160B1_LC | | | | | | | | | | | | | | | P | | | | | | | | | | | |
| 21-225_160G10_LC | | | | | | | | | | | | | | | | | | | | | | | | | | |
| 21-225_162H6_LC | | | | | | | | | | | | | | | | | | | | | | | | | | |
| 21-225_167E2_LC | | | | | | | | | | | | | | | | | | | | | | | | | | |
| 21-225_167H10_LC | | | | | | | | | | | | | | | | | | | | | | | | | | |
| 21-225_170A1_LC | | | | | | | | | | | | | | | | P | | | | | | | | | | |
| 21-225_172B7_LC | | | | | | | | | | | | | | | P | | | | | | | | | | | |
| 21-225_173A11_LC | | | | | | | | | | | | | | | | | | | | | | | | | | |
| 21-225_176H4_LC | | | | | | | | | | | | | | | | | | | | | | | | | | |
| 21-225_177B11_LC | | | R | | | | | | | | | | | | | | | | | | | | | | | |
| 21-225_178G10_LC | | | | | | | | | | | | | | | | | | | | | | | | | | |
| 21-225_180C4_LC | | | | | | | | | | | | | | | | | | | | | | | | | | |
| 21-225_181C10_LC | | | | | | | | | | | | | | | | | | | | | | | | | | |
| 21-225_181C2_LC | | | | | | | | | | | | | | | | | | | | | | | | | | |
| 21-225_186F7_LC | | | | | | | | | | | | | | | C | | | | | | | | | | | |
| 21-225_188G11_LC | | | | | | | | | | | | | | | | | | | | | | | | | | |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | L | L | H | G | D | - | G | K | T | Y | L | Y | W | Y | L | Q | K | P | G | Q | P | P |
| 21-225_189G2_LC | . | . | . | . | . | . | . | . | . | . | . | . | . | . | . | S | . | . | . | . | . | . | . | . | . | . |
| 21-225_190E7_LC | . | . | . | . | . | . | . | S | . | . | . | R | . | . | . | . | . | . | . | . | . | . | . | . | . | . |
| 21-225_190H11_LC | . | . | . | . | . | . | . | S | . | . | . | R | . | . | . | . | . | . | . | . | . | . | . | . | . | . |
| 21-225_191B1_LC | . | . | . | . | . | . | . | S | . | . | . | . | . | . | . | S | . | . | . | . | . | . | . | . | . | . |
| 21-225_191E3_LC | . | . | . | . | . | . | . | S | . | . | . | R | . | . | . | . | . | . | . | . | . | . | . | . | . | . |
| 21-225_191E6_LC | . | . | . | . | . | . | . | S | . | . | . | R | . | . | . | . | . | . | . | . | . | . | . | . | . | . |
| 21-225_192F9_LC | . | . | . | . | . | . | . | S | . | . | . | R | . | . | . | . | . | . | . | . | R | . | . | . | . | . |
| 21-225_194F4_LC | . | . | . | . | . | . | . | S | . | . | . | R | . | . | . | . | . | . | . | . | . | . | . | . | . | . |
| 21-225_197G8_LC | . | . | N | . | . | . | . | S | . | . | . | . | . | . | . | . | . | . | . | . | . | . | . | . | . | . |
| 21-225_198A10_LC | . | . | . | . | . | . | Y | S | . | . | . | R | . | . | . | . | . | . | . | . | . | . | . | . | . | . |
| 21-225_224A7_LC | . | . | . | . | . | . | . | S | . | . | . | . | . | S | . | . | . | S | . | . | . | . | . | . | . | . |
| 21-225_224C11_LC | . | . | . | . | . | . | . | S | . | . | . | . | . | S | . | . | . | . | . | . | . | . | . | . | . | . |
| 21-225_224G1_LC | . | . | . | . | . | . | . | S | . | . | . | . | . | S | . | . | S | . | . | . | . | . | . | . | . | . |
| 21-225_224H11_LC | . | . | . | . | . | . | . | S | . | . | . | . | . | S | . | . | . | . | . | . | . | . | . | . | . | . |
| 21-225_225G4_LC | . | . | . | . | . | . | . | S | . | . | . | . | . | S | . | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | R | . | . | . | . | . |
| 21-225_226E11_LC | . | . | . | . | . | . | . | S | . | . | . | . | . | S | . | . | . | . | . | R | . | . | . | . | . | . |
| 21-225_226E7_LC | . | . | . | . | . | . | . | . | . | . | . | . | . | . | . | . | . | . | Q | . | . | . | . | . | . | . |
| 21-225_226F10_LC | . | . | . | . | . | . | . | S | . | . | . | . | . | S | . | . | . | . | . | . | . | . | . | . | . | . |
| 21-225_226F9_LC | . | . | . | . | . | . | . | S | . | . | . | . | . | S | . | . | . | . | . | . | . | . | . | . | . | . |
| 21-225_226G8_LC | . | . | . | . | . | . | . | S | . | . | . | . | . | S | . | . | . | . | . | . | . | . | . | . | . | . |
| 21-225_226H12_LC | . | R | T | . | . | . | . | S | . | . | . | . | . | S | . | . | . | . | . | . | . | . | . | . | . | . |
| 21-225_226H9_LC | . | . | . | . | . | . | . | S | . | . | . | . | . | S | . | . | . | . | . | . | R | . | . | . | . | . |
| 21-225_227A8_LC | . | . | . | . | . | . | . | S | . | . | . | . | . | S | . | . | . | . | . | . | . | . | . | . | . | . |
| 21-225_227C1_LC | . | . | . | . | . | . | . | S | . | . | . | . | . | S | . | . | . | . | . | . | . | . | . | . | . | . |
| 21-225_227F2_LC | . | . | . | . | . | . | . | S | . | . | . | . | . | S | . | . | . | . | . | . | . | . | . | . | . | . |
| 21-225_22A1_LC | . | . | . | . | . | . | . | S | G | . | . | . | . | . | . | . | . | . | . | . | . | . | . | . | . | . |
| 21-225_25H9_LC | . | . | . | . | . | . | . | . | . | . | . | . | . | S | . | . | . | . | . | . | . | . | . | . | . | . |

4823

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | L | L | H | G | D | - | G | K | T | Y | L | Y | W | Y | L | Q | K | P | G | Q | P | P |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | . | . | . |
| 21-225_43E11_LC | . | . | . | . | . | . | . | S | . | . | . | R | . | . | . | . | . | . | . | . | . | . | . | . | . | . |
| 21-225_46F2_LC | . | . | . | . | . | . | . | . | . | . | . | . | . | . | . | . | . | . | . | . | . | . | . | . | . | . |
| 21-225_47C1_LC | . | . | . | . | . | . | . | S | E | . | . | . | . | . | . | . | . | . | . | . | . | . | . | . | . | . |
| 21-225_47E7_LC | . | . | . | . | . | . | . | S | . | . | . | R | . | . | . | . | . | . | . | . | . | . | . | . | . | . |
| 21-225_64H9_LC | . | . | . | . | . | . | . | . | . | . | . | . | . | . | . | F | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | Q | L | L | I | Y | E | - | - | - | - | - | - | - | - | V | S | N | R | F | S | G | V | P | D | R | F |
| 21-225_146B3_LC | . | . | . | . | . | . | . | . | . | . | . | . | . | . | . | . | . | . | . | . | . | . | . | . | . | . |
| 21-225_147E10_LC | X | . | . | . | S | . | . | . | . | . | . | . | . | . | . | . | . | . | . | . | . | . | . | . | . | . |
| 21-225_148B2_LC | . | . | . | . | . | . | . | . | . | . | . | . | . | . | . | . | X | . | . | . | . | . | . | . | . | . |
| 21-225_148G10_LC | . | . | . | . | . | . | . | . | . | . | . | . | . | . | . | . | . | . | . | . | . | . | . | . | . | . |
| 21-225_149F1_LC | . | . | . | . | . | . | . | . | . | . | . | . | . | . | . | . | X | . | . | . | . | . | . | . | . | . |
| 21-225_150B11_LC | . | . | . | . | . | . | . | . | . | . | . | . | . | . | . | . | . | . | . | . | . | . | . | . | . | . |
| 21-225_151A10_LC | . | . | . | . | . | . | . | . | . | . | . | . | . | . | . | . | X | . | . | . | . | . | . | . | . | . |
| 21-225_151G5_LC | . | . | . | L | . | . | . | . | . | . | . | . | . | . | . | . | . | . | . | . | . | . | . | . | . | . |
| 21-225_152C11_LC | . | I | . | . | . | . | . | . | . | . | . | . | . | . | . | . | . | . | . | . | . | . | . | . | . | . |
| 21-225_152F6_LC | . | V | . | . | X | . | . | . | . | . | . | . | . | . | I | . | X | . | . | T | . | . | . | . | . | . |
| 21-225_153D2_LC | . | V | . | . | X | . | . | . | . | . | . | . | . | . | . | . | X | . | . | T | . | . | . | . | . | . |
| 21-225_155A5_LC | . | . | . | . | . | . | . | . | . | . | . | . | . | . | . | . | . | . | . | . | . | . | . | . | . | . |
| 21-225_157G8_LC | . | . | . | . | . | . | . | . | . | . | . | . | . | . | I | . | X | . | . | . | . | . | . | . | . | . |
| 21-225_160B1_LC | . | V | . | . | . | . | . | . | . | . | . | . | . | . | . | . | X | . | . | . | . | . | S | X | . | . |
| 21-225_160G10_LC | X | . | . | . | . | . | . | . | . | . | . | . | . | . | . | . | X | . | . | . | . | . | . | . | . | . |
| 21-225_162H6_LC | . | . | . | . | . | . | . | . | . | . | . | . | . | . | . | . | X | . | . | . | . | . | . | X | . | . |
| 21-225_167E2_LC | X | . | . | . | . | . | . | . | . | . | . | . | . | . | . | . | X | . | . | . | . | . | . | . | . | . |
| 21-225_167H10_LC | X | . | . | . | . | . | . | . | . | . | . | . | . | . | . | . | X | . | . | . | . | . | . | . | . | . |
| 21-225_170A1_LC | . | V | . | . | . | . | . | . | . | . | . | . | . | . | . | . | . | . | . | . | . | . | . | . | . | . |
| 21-225_172B7_LC | . | V | . | L | X | . | . | . | . | . | . | . | . | . | . | . | X | . | . | . | . | . | . | . | . | . |
| 21-225_173A11_LC | . | . | . | . | . | . | . | . | . | . | . | . | . | . | . | . | . | . | . | . | . | . | . | . | . | . |
| 21-225_176H4_LC | . | . | . | . | . | . | . | . | . | . | . | . | . | . | . | . | . | . | . | . | . | . | . | . | . | . |
| 21-225_177B11_LC | . | I | . | . | . | . | . | . | . | . | . | . | . | . | . | . | . | . | . | . | . | . | . | . | . | . |
| 21-225_178G10_LC | . | . | . | . | . | . | . | . | . | . | . | . | . | . | . | . | . | . | . | . | . | . | . | . | . | L |
| 21-225_180C4_LC | . | . | . | . | . | A | . | . | . | . | . | . | . | . | T | . | . | . | . | P | . | . | S | . | . | . |
| 21-225_181C10_LC | . | . | . | . | . | . | . | . | . | . | . | . | . | . | . | . | . | . | . | . | . | . | . | . | . | . |
| 21-225_181C2_LC | . | . | . | . | X | . | . | . | . | . | . | . | . | . | . | . | . | . | . | . | . | . | . | . | . | . |
| 21-225_186F7_LC | . | . | . | . | . | . | . | . | . | . | . | . | . | . | . | . | X | . | . | . | . | . | . | . | . | . |
| 21-225_188G11_LC | . | . | . | . | . | . | . | . | . | . | . | . | . | . | . | . | . | . | . | . | . | . | . | . | T | . |

4825

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | Q | L | L | I | Y | E | - | - | - | - | - | - | - | - | V | S | N | R | F | S | G | V | P | D | R | F |
| 21-225_189G2_LC | . | . | . | . | . | . | . | . | . | . | . | . | . | . | . | . | . | . | . | . | . | . | S | . | . | . |
| 21-225_190E7_LC | . | V | . | . | C | . | . | . | . | . | . | . | . | . | . | . | . | . | . | . | . | . | . | . | . | . |
| 21-225_190H11_LC | . | . | . | . | C | . | . | . | . | . | . | . | . | . | . | . | . | . | . | A | . | . | T | . | . | . |
| 21-225_191B1_LC | . | V | . | . | . | . | . | . | . | . | . | . | . | . | L | . | . | . | . | . | . | . | . | . | . | . |
| 21-225_191E3_LC | . | . | . | . | C | . | . | . | . | . | . | . | . | . | . | . | . | . | . | A | . | . | T | . | . | . |
| 21-225_191E6_LC | . | . | . | . | C | . | . | . | . | . | . | . | . | . | . | . | . | . | . | A | . | . | T | . | . | . |
| 21-225_192F9_LC | . | . | . | . | C | . | . | . | . | . | . | . | . | . | . | . | . | . | . | A | . | . | T | . | . | . |
| 21-225_194F4_LC | . | . | . | . | C | . | . | . | . | . | . | . | . | . | . | . | . | . | . | . | . | . | . | . | . | . |
| 21-225_197G8_LC | . | V | . | . | . | . | . | . | . | . | . | . | . | . | . | . | . | . | . | . | . | . | . | . | . | . |
| 21-225_198A10_LC | . | V | . | . | C | . | . | . | . | . | . | . | . | . | . | . | . | . | . | . | . | . | . | . | . | . |
| 21-225_224A7_LC | . | . | . | . | . | . | . | . | . | . | . | . | . | . | I | . | . | . | . | . | . | . | . | . | . | . |
| 21-225_224C11_LC | . | . | . | . | . | . | . | . | . | . | . | . | . | . | I | . | . | . | . | . | . | . | . | . | . | . |
| 21-225_224G1_LC | . | . | . | . | . | . | . | . | . | . | . | . | . | . | I | . | . | . | . | . | . | . | . | . | . | . |
| 21-225_224H11_LC | . | . | . | . | . | . | . | . | . | . | . | . | . | . | I | . | . | . | . | . | . | . | . | . | . | . |
| 21-225_225G4_LC | . | . | . | . | . | . | . | . | . | . | . | . | . | . | I | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | N | . | . | . | . | . | . | . | . | . | . | . | . | . | I | . | . | . | . | . | . | . |
| 21-225_226E11_LC | X | . | . | . | . | . | . | . | . | . | . | . | . | . | I | . | . | . | . | . | . | . | . | . | . | . |
| 21-225_226E7_LC | . | I | . | . | . | . | . | . | . | . | . | . | . | . | . | . | . | . | . | . | . | . | . | . | . | . |
| 21-225_226F10_LC | . | . | . | . | . | . | . | . | . | . | . | . | . | . | I | . | . | . | . | . | . | . | . | . | . | . |
| 21-225_226F9_LC | . | . | . | . | . | . | . | . | . | . | . | . | . | . | I | . | . | . | . | . | . | . | . | . | . | . |
| 21-225_226G8_LC | . | . | . | . | . | . | . | . | . | . | . | . | . | . | I | . | . | . | . | . | . | . | . | . | . | . |
| 21-225_226H12_LC | . | P | . | . | . | . | . | . | . | . | . | . | . | . | I | . | . | . | . | . | . | . | . | . | . | . |
| 21-225_226H9_LC | . | . | . | . | . | . | . | . | . | . | . | . | . | . | . | . | . | . | . | . | . | . | . | . | . | . |
| 21-225_227A8_LC | . | . | . | . | . | . | . | . | . | . | . | . | . | . | I | . | . | . | . | . | . | . | . | . | . | . |
| 21-225_227C1_LC | . | . | . | . | . | . | . | . | . | . | . | . | . | . | I | . | . | . | . | . | . | . | . | . | . | . |
| 21-225_227F2_LC | . | . | . | . | . | . | . | . | . | . | . | . | . | . | I | . | . | . | . | . | . | . | . | . | . | . |
| 21-225_22A1_LC | . | . | . | . | . | . | . | . | . | . | . | . | . | . | . | . | . | . | . | . | . | L | . | . | . | . |
| 21-225_25H9_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | Q | L | L | I | Y | E | - | - | - | - | - | - | - | - | V | S | N | R | F | S | G | V | P | D | R | F |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | S | . | . |
| 21-225_43E11_LC | . | . | . | . | . | . | . | . | . | . | . | . | . | . | . | . | . | . | . | . | . | . | . | . | . | . |
| 21-225_46F2_LC | . | V | . | . | . | . | . | . | . | . | . | . | . | . | . | . | . | . | . | . | . | . | . | . | . | . |
| 21-225_47C1_LC | . | . | . | . | . | . | . | . | . | . | . | . | . | . | . | . | . | . | . | . | . | . | . | . | . | . |
| 21-225_47E7_LC | . | . | . | . | . | . | . | . | . | . | . | . | . | . | . | . | S | . | . | . | . | . | . | . | . | . |
| 21-225_64H9_LC | . | V | . | . | . | . | . | . | . | . | . | . | . | . | . | . | . | . | L | C | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | K | I | S | R | V | E | A | E | D | V | G | V | Y |
| 21-225_146B3_LC | | | | | | | | | | | | | | | | | | | | | | | | | L | |
| 21-225_147E10_LC | | | | | | | | | | | | | | | | | | | | | | | | | | |
| 21-225_148B2_LC | | | | | | | | | | | | | | | | | | | | | | | | | | |
| 21-225_148G10_LC | | | | | | | | | | | | | | | | | | | | | | | | | | |
| 21-225_149F1_LC | | | | | | | | | | | | | | | | | | | | | | | | | | |
| 21-225_150B11_LC | | | | | | | | | | | | | | | | | | | | | | | | | | |
| 21-225_151A10_LC | | | | | | | | | | | | | | | | | | | | | | | | | | |
| 21-225_151G5_LC | | | | | | | | | | | | | | | | | | | | | | | | | | |
| 21-225_152C11_LC | | | | | | | | | | | | | | N | | | | | | | | | P | | P | |
| 21-225_152F6_LC | | | | | | | | | | | | | | N | | | | | | | | | | | P | |
| 21-225_153D2_LC | | | | | | | | | | | | | | N | | | | | | | | | | | P | |
| 21-225_155A5_LC | | | | | | | | | | | | | | | | | | | | | | | | | P | |
| 21-225_157G8_LC | | | | | | | | | | | | | | | | | | | | | | | | | | |
| 21-225_160B1_LC | | | | | | | | | | | | | | | | | | | | | | | | | | |
| 21-225_160G10_LC | | | | | | | | | | | | | | | | | | | | | | | | | L | |
| 21-225_162H6_LC | | | | | | | | | | | | | | | | | | | | | | | | | | |
| 21-225_167E2_LC | | | | | | | | | | | | | | | | | | | | | | | | | L | |
| 21-225_167H10_LC | | | | | | | | | | | | | | | | | | | | | | | | | L | |
| 21-225_170A1_LC | | | | | | | | | | | | | | | | | | | | | | | | | | |
| 21-225_172B7_LC | | G | | | | | | | | | | | | | | | | | | | | | | | | P |
| 21-225_173A11_LC | | | | | | | | | | | | | | | | | | | | | | | | | | P |
| 21-225_176H4_LC | | | | | | | | | | | | | | | | | | | | | | | | | I | |
| 21-225_177B11_LC | | | | | | | | | | E | | | | | | | | | | | | | | | | |
| 21-225_178G10_LC | | G | | | | | | | | | | | | | | | | | | | | | | | I | |
| 21-225_180C4_LC | | | | | | | | | | | | | | | | | | | | | | | | | | |
| 21-225_181C10_LC | | | | | | | | | | | | | | | | | | | | | | | | | | |
| 21-225_181C2_LC | | | | | | | | | | | | | | | | | | | | | | | | | | |
| 21-225_186F7_LC | | | | | | | | | | | | | | | | | | | | | | | | | | |
| 21-225_188G11_LC | | | | | | | | | | | | | | | | | | | | | | | | | | |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | K | I | S | R | V | E | A | E | D | V | G | V | Y |
| 21-225_189G2_LC | . | . | . | . | . | . | . | . | . | . | . | . | . | . | . | . | . | . | . | . | . | . | . | . | . | . |
| 21-225_190E7_LC | . | . | . | . | . | . | . | . | . | . | . | . | . | . | . | . | . | . | . | . | G | . | . | . | . | . |
| 21-225_190H11_LC | . | . | . | . | . | . | . | . | . | . | . | . | . | . | . | . | . | . | . | . | G | . | . | . | . | . |
| 21-225_191B1_LC | . | . | . | . | . | . | . | . | . | . | . | . | . | . | . | . | . | . | . | . | . | . | . | . | . | . |
| 21-225_191E3_LC | . | . | . | . | . | . | . | . | . | . | . | . | . | . | . | . | . | . | . | . | G | . | . | . | . | . |
| 21-225_191E6_LC | . | . | . | . | . | . | . | . | . | . | . | . | . | . | . | . | . | . | . | . | G | . | . | . | } | . |
| 21-225_192F9_LC | . | . | . | . | . | . | . | . | . | . | . | . | . | . | . | . | . | . | . | . | G | . | . | . | . | . |
| 21-225_194F4_LC | . | . | . | . | . | . | . | . | . | . | . | . | . | . | . | . | . | . | . | . | G | . | . | . | . | . |
| 21-225_197G8_LC | . | . | . | . | . | . | . | . | . | . | . | . | . | . | . | . | . | . | . | . | . | . | . | . | . | . |
| 21-225_198A10_LC | . | . | . | . | . | . | . | . | . | . | . | . | . | . | . | . | . | . | . | . | G | . | . | . | . | . |
| 21-225_224A7_LC | . | . | . | . | . | . | . | . | . | . | . | . | . | . | . | . | . | . | . | . | . | . | . | . | } | . |
| 21-225_224C11_LC | . | . | . | . | . | . | . | . | . | . | . | . | . | . | . | . | . | . | . | . | . | . | . | . | } | . |
| 21-225_224G1_LC | . | . | . | . | . | . | . | . | . | . | . | . | . | . | . | . | . | . | . | . | . | . | . | . | } | P |
| 21-225_224H11_LC | . | . | . | . | . | . | . | . | . | . | . | . | . | . | . | . | . | . | . | . | . | . | . | . | } | . |
| 21-225_225G4_LC | . | . | . | . | . | . | . | . | . | . | . | . | . | . | . | . | . | . | . | . | . | . | . | . | } | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | . | . | . | . | . | . |
| 21-225_226E11_LC | . | . | . | . | . | . | . | . | . | . | . | . | . | . | . | . | . | . | . | . | . | . | . | . | } | . |
| 21-225_226E7_LC | . | . | . | . | . | . | . | . | . | . | . | . | . | . | . | . | . | . | . | . | . | . | . | . | . | . |
| 21-225_226F10_LC | . | . | . | . | . | . | . | . | . | . | . | . | . | . | . | . | . | . | . | . | . | . | . | . | } | . |
| 21-225_226F9_LC | . | . | . | . | . | . | . | . | . | . | . | . | . | . | . | . | . | . | . | . | . | . | . | . | } | . |
| 21-225_226G8_LC | . | . | . | . | . | . | . | . | . | . | . | . | . | . | . | . | . | . | . | . | . | . | . | . | } | . |
| 21-225_226H12_LC | . | . | . | . | . | . | . | . | . | . | . | . | . | . | . | . | . | . | . | . | . | . | . | . | } | . |
| 21-225_226H9_LC | . | . | . | . | . | . | . | . | . | . | . | . | . | . | . | . | . | . | . | . | . | . | . | . | } | . |
| 21-225_227A8_LC | . | . | . | . | . | . | . | . | . | . | . | . | . | . | . | . | . | . | . | . | . | . | . | . | } | . |
| 21-225_227C1_LC | . | . | . | . | . | . | . | . | . | . | . | . | . | . | . | . | . | . | . | . | . | . | . | . | } | . |
| 21-225_227F2_LC | . | . | . | . | . | . | . | . | . | . | . | . | . | . | . | . | . | . | . | . | . | . | . | . | } | . |
| 21-225_22A1_LC | . | . | . | . | . | . | . | . | . | . | . | . | . | . | . | . | . | . | . | A | . | . | . | . | . | . |
| 21-225_25H9_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | | |
| CONSENSUS | S | G | S | G | S | G | - | - | T | D | F | T | L | K | I | S | R | V | E | A | E | D | V | G | V | Y |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | . | . | . |
| 21-225_43E11_LC | . | . | . | . | . | . | . | . | . | . | . | . | . | . | . | . | . | . | . | . | . | . | . | . | . | . |
| 21-225_46F2_LC | . | . | . | . | . | . | . | . | . | . | . | . | . | . | L | . | . | . | . | . | . | . | . | . | . | . |
| 21-225_47C1_LC | . | . | . | . | . | . | . | . | . | . | . | . | . | . | . | . | . | . | . | . | . | . | . | . | . | R |
| 21-225_47E7_LC | . | . | . | . | . | . | . | . | . | . | A | . | . | . | . | . | . | . | . | . | . | . | . | . | . | . |
| 21-225_64H9_LC | . | . | . | . | . | . | . | . | . | . | S | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | M | Q | S | I | Q | - | - | - | - | - | - | - | - | - | - | - | - | - | - | - | - | - | - | - |
| 21-225_146B3_LC | | | | | | | | | | | | | | | | | | | | | | | | | | |
| 21-225_147E10_LC | | | | | | | | | | | | | | | | | | | | | | | | | | |
| 21-225_148B2_LC | | | | | | | | | | | | | | | | | | | | | | | | | | |
| 21-225_148G10_LC | | | | | | | | | | | | | | | | | | | | | | | | | | |
| 21-225_149F1_LC | | | | | | | | | | | | | | | | | | | | | | | | | | |
| 21-225_150B11_LC | | | | | | | | | | | | | | | | | | | | | | | | | | |
| 21-225_151A10_LC | | | | | | | | | | | | | | | | | | | | | | | | | | |
| 21-225_151G5_LC | | | | | | | | | | | | | | | | | | | | | | | | | | |
| 21-225_152C11_LC | | | | | | | | | | | | | | | | | | | | | | | | | | |
| 21-225_152F6_LC | | | | | | | | | | | | | | | | | | | | | | | | | | |
| 21-225_153D2_LC | | | | | | | | | | | | | | | | | | | | | | | | | | |
| 21-225_155A5_LC | | | | | | | | | | | | | | | | | | | | | | | | | | |
| 21-225_157G8_LC | | | | | | | | | | | | | | | | | | | | | | | | | | |
| 21-225_160B1_LC | | | | | | | | | | | | | | | | | | | | | | | | | | |
| 21-225_160G10_LC | | | | | | | | | | | | | | | | | | | | | | | | | | |
| 21-225_162H6_LC | | | X | | | | | | | | | | | | | | | | | | | | | | | |
| 21-225_167E2_LC | X | | | | | | | | | | | | | | | | | | | | | | | | | |
| 21-225_167H10_LC | X | | | | | | | | | | | | | | | | | | | | | | | | | |
| 21-225_170A1_LC | | | | | | | | | | | | | | | | | | | | | | | | | | |
| 21-225_172B7_LC | | | | | | | | | | | | | | | | | | | | | | | | | | |
| 21-225_173A11_LC | X | | | | | | | | | | | | | | | | | | | | | | | | | |
| 21-225_176H4_LC | | | | | | | | | | | | | | | | | | | | | | | | | | |
| 21-225_177B11_LC | | | | | | | | | | | | | | | | | | | | | | | | | | |
| 21-225_178G10_LC | | | | | | | | | | | | | | | | | | | | | | | | | | |
| 21-225_180C4_LC | | | | | | | | | | | | | | | | | | | | | | | | | | |
| 21-225_181C10_LC | X | | | | | | | | | | | | | | | | | | | | | | | | | |
| 21-225_181C2_LC | | | | | | | | | | | | | | | | | | | | | | | | | | |
| 21-225_186F7_LC | | | | | | | | | | | | | | | | | | | | | | | | | | |
| 21-225_188G11_LC | | | | | | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | M | Q | S | I | Q | - | - | - | - | - | - | - | - | - | - | - | - | - | - | - | - | - | - | - |
| 21-225_189G2_LC | | | | | | | | | | | | | | | | | | | | | | | | | | |
| 21-225_190E7_LC | | | | | | | | | | | | | | | | | | | | | | | | | | |
| 21-225_190H11_LC | | | | | | | | | | | | | | | | | | | | | | | | | | |
| 21-225_191B1_LC | | | | | | F | | | | | | | | | | | | | | | | | | | | |
| 21-225_191E3_LC | | | | | | | | | | | | | | | | | | | | | | | | | | |
| 21-225_191E6_LC | | | | | | | M | | | | | | | | | | | | | | | | | | | |
| 21-225_192F9_LC | | | | | | | | | | | | | | | | | | | | | | | | | | |
| 21-225_194F4_LC | | | | | | | | | | | | | | | | | | | | | | | | | | |
| 21-225_197G8_LC | S | | | | | | | | | | | | | | | | | | | | | | | | | |
| 21-225_198A10_LC | | | | | | | | | | | | | | | | | | | | | | | | | | |
| 21-225_224A7_LC | | | | | | T | | | | | | | | | | | | | | | | | | | | |
| 21-225_224C11_LC | | | | | | T | | | | | | | | | | | | | | | | | | | | |
| 21-225_224G1_LC | | | | | | T | | | | | | | | | | | | | | | | | | | | |
| 21-225_224H11_LC | | | | | | T | | | | | | | | | | | | | | | | | | | | |
| 21-225_225G4_LC | | | | | | T | | | | | | | | | | | | | | | | | | | | |
| 21-225_226A5_LC | | | | | | | | | | | | | | | | | | | | | | | | | | |
| 21-225_226B1_LC | | | | | | | | | | | | | | | | | | | | | | | | | | |
| 21-225_226E11_LC | | | | | | T | | | | | | | | | | | | | | | | | | | | |
| 21-225_226E7_LC | | | | | | | | | | | | | | | | | | | | | | | | | | |
| 21-225_226F10_LC | | | | | | T | | | | | | | | | | | | | | | | | | | | |
| 21-225_226F9_LC | | | | | | T | | | | | | | | | | | | | | | | | | | | |
| 21-225_226G8_LC | | | | | | T | | | | | | | | | | | | | | | | | | | | |
| 21-225_226H12_LC | | | | | | T | | | | | | | | | | | | | | | | | | | | |
| 21-225_226H9_LC | | | | | | T | | | | | | | | | | | | | | | | | | | | |
| 21-225_227A8_LC | | | | | | T | | | | | | | | | | | | | | | | | | | | |
| 21-225_227C1_LC | | | | | | T | | | | | | | | | | | | | | | | | | | | |
| 21-225_227F2_LC | | | | | | T | | | | | | | | | | | | | | | | | | | | |
| 21-225_22A1_LC | | | T | | L | M | | | | | | | | | | | | | | | | | | | | |
| 21-225_25H9_LC | | | | | | | | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | M | Q | S | I | Q | - | - | - | - | - | - | - | - | - | - | - | - | - | - | - | - | - | - | - |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | . | . | . |
| 21-225_43E11_LC | . | . | . | . | . | . | . | . | . | . | . | . | . | . | . | . | . | . | . | . | . | . | . | . | . | . |
| 21-225_46F2_LC | . | . | . | . | . | . | . | . | . | . | . | . | . | . | . | . | . | . | . | . | . | . | . | . | . | . |
| 21-225_47C1_LC | . | . | . | . | . | . | . | . | . | . | . | . | . | . | . | . | . | . | . | . | . | . | . | . | . | . |
| 21-225_47E7_LC | . | . | . | . | . | T | . | . | . | . | . | . | . | . | . | . | . | . | . | . | . | . | . | . | . | . |
| 21-225_64H9_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | L | P | W | T | F | G | Q | G | T | K | V | E | I | K | R |
| 21-225_146B3_LC | . | . | | . | . | . | . | | | . | | | . | | | . | . | . | |
| 21-225_147E10_LC | . | . | | . | V | . | | . | . | . | . | . | . | . | . | . | . | . | . |
| 21-225_148B2_LC | . | . | | . | I | . | . | | . | . | . | . | | | . | . | . | . | |
| 21-225_148G10_LC | . | . | | . | . | . | . | . | . | . | . | . | . | . | . | . | . | . | . |
| 21-225_149F1_LC | . | . | | . | I | . | . | . | . | . | . | . | . | . | . | . | . | . | . |
| 21-225_150B11_LC | . | . | | . | . | . | . | . | . | . | . | . | . | . | . | . | . | . | . |
| 21-225_151A10_LC | . | . | | . | I | . | . | . | . | . | . | . | . | . | . | . | . | . | . |
| 21-225_151G5_LC | . | . | | . | V | . | . | . | . | . | . | . | . | . | . | . | . | . | . |
| 21-225_152C11_LC | . | . | | . | I | . | . | . | . | . | . | . | . | . | . | D | . | . | . |
| 21-225_152F6_LC | . | . | | . | V | . | . | . | . | . | . | . | . | . | . | . | . | . | . |
| 21-225_153D2_LC | . | . | | . | V | . | . | . | . | . | . | . | . | . | . | . | . | . | . |
| 21-225_155A5_LC | . | . | | . | V | . | . | . | . | . | . | . | . | . | . | . | . | . | . |
| 21-225_157G8_LC | . | . | | . | V | . | . | . | . | . | . | . | . | . | . | . | . | . | . |
| 21-225_160B1_LC | . | . | | . | I | . | . | . | . | . | . | . | . | . | . | . | . | . | . |
| 21-225_160G10_LC | . | . | | . | . | . | . | . | . | V | . | . | . | . | . | . | . | T | |
| 21-225_162H6_LC | . | . | | . | . | . | . | . | . | . | . | . | . | . | . | . | . | . | . |
| 21-225_167E2_LC | . | . | | . | . | . | . | . | . | . | . | . | . | . | . | . | . | . | . |
| 21-225_167H10_LC | . | . | | . | . | . | . | . | . | . | . | . | . | . | . | . | . | . | . |
| 21-225_170A1_LC | . | . | | . | . | . | . | . | . | . | . | . | . | . | . | . | . | . | . |
| 21-225_172B7_LC | . | . | | . | I | . | . | . | . | . | . | . | D | . | . | D | . | . | . |
| 21-225_173A11_LC | . | . | | . | V | . | . | . | . | . | . | . | . | . | . | . | . | . | . |
| 21-225_176H4_LC | . | . | | . | I | . | . | . | . | . | . | . | R | . | . | . | . | . | . |
| 21-225_177B11_LC | . | . | | . | I | . | . | . | . | . | . | . | . | . | . | . | . | . | . |
| 21-225_178G10_LC | . | . | | . | V | . | . | . | . | . | . | . | . | . | . | . | . | . | . |
| 21-225_180C4_LC | . | . | | . | V | . | . | . | . | . | . | . | . | . | . | . | . | . | . |
| 21-225_181C10_LC | . | . | | . | I | . | . | . | . | . | . | . | . | . | . | . | . | . | . |
| 21-225_181C2_LC | . | . | | . | V | . | . | . | . | . | . | . | . | . | . | . | . | . | . |
| 21-225_186F7_LC | . | . | | . | V | . | . | . | . | . | . | . | . | . | . | . | . | . | . |
| 21-225_188G11_LC | . | . | | . | I | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | K_FR4 | | | | | | | |
| CONSENSUS | - | - | - | - | L | P | W | T | F | G | Q | G | T | K | V | E | I | K | R |
| 21-225_189G2_LC | . | . | . | . | } | . | . | . | . | . | . | . | . | . | . | . | . | . | . |
| 21-225_190E7_LC | . | . | . | . | . | . | . | . | . | . | . | . | . | . | . | . | . | . | . |
| 21-225_190H11_LC | . | . | . | . | P | . | . | . | . | . | . | . | . | . | . | . | . | . | . |
| 21-225_191B1_LC | . | . | . | . | . | . | . | . | . | . | . | . | . | . | . | . | . | N | . |
| 21-225_191E3_LC | . | . | . | . | . | . | . | . | . | . | . | . | . | . | . | . | . | . | . |
| 21-225_191E6_LC | . | . | . | . | P | . | . | . | . | . | . | . | . | . | . | . | . | . | . |
| 21-225_192F9_LC | . | . | . | . | . | . | . | . | . | . | . | . | . | . | . | . | . | . | . |
| 21-225_194F4_LC | . | . | . | . | . | . | . | . | . | . | . | . | . | . | . | . | . | . | . |
| 21-225_197G8_LC | . | . | . | . | . | . | . | . | A | . | . | . | S | . | . | . | . | . | . |
| 21-225_198A10_LC | . | . | . | . | . | . | . | . | . | . | . | . | . | . | . | . | . | . | . |
| 21-225_224A7_LC | . | . | . | . | . | . | . | . | . | . | . | . | . | . | . | . | . | . | . |
| 21-225_224C11_LC | . | . | . | . | . | . | . | . | . | . | . | . | . | . | . | . | . | . | . |
| 21-225_224G1_LC | . | . | . | . | . | . | . | . | . | . | . | . | . | . | . | . | . | . | . |
| 21-225_224H11_LC | . | . | . | . | . | . | . | . | . | . | . | . | . | . | . | . | . | . | . |
| 21-225_225G4_LC | . | . | . | . | . | . | . | . | . | . | . | . | . | . | . | . | . | . | . |
| 21-225_226A5_LC | . | . | . | . | } | . | . | . | . | . | . | . | . | . | . | . | . | . | . |
| 21-225_226B1_LC | . | . | . | . | } | . | . | . | . | . | . | . | . | . | . | C | . | . | . |
| 21-225_226E11_LC | . | . | . | . | . | . | . | . | . | . | . | . | . | . | . | . | . | . | . |
| 21-225_226E7_LC | . | . | . | . | V | . | . | . | . | . | . | . | . | . | . | . | . | . | . |
| 21-225_226F10_LC | . | . | . | . | . | . | . | . | . | . | . | . | . | . | . | . | . | . | . |
| 21-225_226F9_LC | . | . | . | . | . | . | . | . | . | . | . | . | . | . | . | . | . | . | . |
| 21-225_226G8_LC | . | . | . | . | . | . | . | . | . | . | . | . | . | . | . | . | . | . | . |
| 21-225_226H12_LC | . | . | . | . | . | . | . | . | . | . | . | . | . | . | . | . | . | . | . |
| 21-225_226H9_LC | . | . | . | . | . | . | . | . | . | . | . | . | . | . | . | . | . | . | . |
| 21-225_227A8_LC | . | . | . | . | . | . | . | . | . | . | . | . | S | . | . | . | . | . | . |
| 21-225_227C1_LC | . | . | . | . | . | . | . | . | . | . | . | . | . | . | . | . | . | . | . |
| 21-225_227F2_LC | . | . | . | . | . | . | . | . | . | . | . | . | . | . | . | . | . | . | . |
| 21-225_22A1_LC | . | . | . | . | . | . | . | . | . | . | . | . | . | . | . | . | . | . | . |
| 21-225_25H9_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | L | P | W | T | F | G | Q | G | T | K | V | E | I | K | R |
| 21-225_26F8_LC | | | | | | | | | | | | | | | | | | | |
| 21-225_28G3_LC | | | | | I | | | | | | | | | | | | | | |
| 21-225_43E11_LC | | | | | | | | | | | | | | | | | | | |
| 21-225_46F2_LC | | | | | | | | | | | | | | | | | | | |
| 21-225_47C1_LC | | | | | | | | | | | | | | | | | | | |
| 21-225_47E7_LC | | | | | | | | | | | | | | A | | | | | |
| 21-225_64H9_LC | | | | | V | | | | | | | | | | | | | T | |

**FIGURE 57**

Table 106. Consensus 26 - VK1|O12/JK3 (SEQ ID NO: 50323):

DIQMTQSPSSLSASVGDRVTITCRAS--QSIS------SYLNWYQQKPGKAPKLLIYA-------ASSLQSGVPSRFSGSGSG--TDFTLTISSLQPEDFATYYCQQSYS--------------------PPFTFGPGTKVDIKR wherein:

A at position 25 can be substituted with S or T

S at position 26 can be substituted with G or I

Q at position 29 can be substituted with H or R

S at position 30 can be substituted with N or T

I at position 31 can be substituted with F

S at position 32 can be substituted with I, F, N, R, Y, T, A, G or L

S at position 39 can be substituted with N, T, H or K

Y at position 40 can be substituted with F or H

L at position 41 can be substituted with V

N at position 42 can be substituted with I, M, H or Y

A at position 58 can be substituted with G, T, V or I

A at position 67 can be substituted with T, V or S

S at position 68 can be substituted with F

S at position 69 can be substituted with N, T or V

Q at position 71 can be substituted with II

S at position 72 can be substituted with N, G, H, I or T

S at position 109 can be substituted with T or Y

Y at position 110 can be substituted with N, F or H

**FIGURE 57**

S at position 111 can be substituted with R, N, F or I

null (-) at position 134 can be substituted with A or S

P at position 135 can be substituted with T, I, F, A, L or V

P at position 136 can be substituted with L, F, or S

T at position 138 can be substituted with A or S

Arg Xaa Xaa Xaa Xaa Xaa Xaa Xaa Xaa Xaa (SEQ ID NO: 50153)

Xaa Xaa Xaa Xaa Leu Xaa Xaa (SEQ ID NO: 50154)

Gln Gln Xaa Xaa Xaa Xaa Xaa Xaa Phe Xaa (SEQ ID NO: 50155)

Arg Ala Ser Gln Ser Ile Ser Ser Tyr Leu Asn (SEQ ID NO: 50639)

Ala Ala Ser Ser Leu Gln Ser (SEQ ID NO: 50640)

Gln Gln Ser Tyr Ser Pro Pro Phe Thr (SEQ ID NO: 50641)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_10F12_LC | | | | | | | | | | | | | | | | | | | | | | | | | | |
| 21-225_11A5_LC | | | | | | | | | | | | | | | | | | | | | | | | | | |
| 21-225_14A11_LC | | | | | | | | | | | | | | I | | | | | | | | | | | | |
| 21-225_158F4_LC | | | | | | | | | | | | P | | | | | | | | | | | | | | |
| 21-225_16H6_LC | | | | | | | | | | | | | | | | | | | | | | | | | | |
| 21-225_17A12_LC | | | | | | | | | | | | | | | | | | | | | | | | | | |
| 21-225_17B4_LC | | | | | | | | | | | | | | | | | | | | | | | | | | |
| 21-225_17C6_LC | | | | | | | | | | | | | | | | | | | | | | | | | | |
| 21-225_17F11_LC | | | | | | | | | | | | | | I | | | | | | S | | | | | | |
| 21-225_17F7_LC | | | | | | | | | | | | | | | | | | | | | | | | | | |
| 21-225_180F8_LC | | | | | | | | | | | | | | | | | | G | | | | | | | | |
| 21-225_18E12_LC | | | | | | | | | | | | | | | | | | | | | | | | | | |
| 21-225_19A5_LC | | | | | | | | | | | | P | | | | | | | | S | | | | | | |
| 21-225_19B3_LC | | | | | | | | | | | | | | | | | | | I | | | | | | | G |
| 21-225_20A4_LC | | | | | | | | A | | | | | | | | | | | | | | | | | | |
| 21-225_20F3_LC | | | | | | | | | | | | | | | | | | | | | | | | | | |
| 21-225_21A2_LC | | | | | | | | | | | | | | | | | | | | | | | | | | |
| 21-225_21A6_LC | | | | | | | | | | | G | | | | | | | | | | | | | | | |
| 21-225_21F4_LC | | | | | | | | | | | | | | | | | | | | | | | | | | |
| 21-225_222H4_LC | | | | K | | | | | | | | | | | | | | | | | | | | | | |
| 21-225_222H8_LC | | | | K | | | | | | | | | | | | | | | | | | | | | | |
| 21-225_22C5_LC | | | | | | | | A | | | | | | | | | | | | | | | | | | |
| 21-225_22D5_LC | | | | | | | | | | | | | | | | | | | | | | | | | | |
| 21-225_22E4_LC | | | | | | | | | | | | | | | | | | | | | | | | | | |
| 21-225_22G10_LC | | | | | | | | | | | | | | | | | | | | | | | | | | |
| 21-225_22G3_LC | | | | | | | | | | | | | | | | | | | | | | | | | | |
| 21-225_22G5_LC | | | | | | | | | | | | | | | | | | | | | | | | | | |
| 21-225_30E11_LC | | | | | | | | | | | | | | | | | | | | | | | | | | |
| 21-225_33G12_LC | | | | | | | | | | | | | | | | | | | | | | | | | | |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_3B1_LC | . | . | . | . | . | . | . | . | . | . | . | . | . | . | . | . | . | . | . | . | . | . | . | . | . | . |
| 21-225_3E3_LC | . | . | . | . | . | . | . | . | . | . | P | . | . | . | . | . | . | . | . | . | . | . | P | . | . | . |
| 21-225_48C3_LC | . | . | . | . | . | . | . | . | . | . | . | . | . | . | . | . | . | . | . | N | . | . | . | . | . | . |
| 21-225_48D1_LC | . | . | . | . | . | . | . | . | . | . | . | . | . | . | . | . | . | . | . | N | . | . | . | . | . | . |
| 21-225_49G12_LC | . | . | . | . | . | . | . | . | . | . | . | . | . | . | . | . | . | . | . | . | . | . | . | . | S | . |
| 21-225_4B1_LC | . | . | . | . | . | . | . | . | . | . | . | . | . | . | . | . | . | . | . | . | . | . | . | . | . | . |
| 21-225_4H2_LC | . | . | . | . | L | . | . | . | . | . | . | . | . | . | . | . | . | . | . | . | . | . | . | . | . | I |
| 21-225_50B12_LC | . | . | . | . | . | . | . | . | . | . | . | . | . | . | . | . | . | . | . | . | . | . | . | . | . | . |
| 21-225_50H10_LC | . | . | . | . | . | . | . | . | . | . | . | . | . | . | . | . | . | . | . | N | . | . | . | . | . | . |
| 21-225_50H8_LC | . | . | . | . | . | . | . | . | . | . | . | . | . | . | . | . | . | . | . | N | . | . | . | . | . | . |
| 21-225_51H10_LC | . | . | . | . | . | . | . | . | . | . | . | . | . | . | . | . | . | . | . | . | . | . | . | . | . | . |
| 21-225_53E2_LC | . | . | . | . | . | . | . | . | . | . | . | . | . | . | . | . | . | . | . | . | . | . | . | . | . | . |
| 21-225_56B2_LC | . | . | . | . | . | . | . | . | . | . | . | . | . | . | . | . | . | . | . | . | . | . | . | . | . | . |
| 21-225_57F2_LC | . | . | . | . | . | . | . | . | . | . | . | . | . | . | . | . | . | . | . | . | . | . | . | . | . | . |
| 21-225_5F7_LC | . | . | . | . | . | . | . | . | . | . | P | . | . | . | . | . | . | . | . | . | . | . | P | . | . | . |
| 21-225_5G2_LC | . | . | . | . | . | . | . | . | . | . | P | . | . | . | . | . | . | . | . | . | . | . | P | . | . | . |
| 21-225_60E12_LC | . | . | . | . | . | . | . | . | . | . | . | . | . | . | . | . | . | . | . | . | . | . | . | . | . | . |
| 21-225_61A1_LC | . | . | . | . | . | . | . | . | . | . | . | . | . | . | . | . | . | . | . | . | . | . | . | . | . | . |
| 21-225_62H1_LC | . | . | . | . | . | . | . | . | . | . | . | . | . | Y | . | . | . | . | . | . | . | . | . | . | . | . |
| 21-225_62H10_LC | . | . | . | . | . | . | . | . | . | . | . | . | . | . | . | . | . | . | . | . | . | . | . | . | . | . |
| 21-225_62H8_LC | . | . | . | . | . | . | . | . | . | . | . | . | . | . | . | . | . | . | . | . | . | . | . | . | . | . |
| 21-225_66A9_LC | . | . | . | . | . | . | . | . | . | . | . | . | . | . | . | . | . | . | . | . | . | . | . | . | . | . |
| 21-225_69C8_LC | . | . | . | . | . | . | . | . | . | . | . | . | . | . | . | . | . | . | . | P | . | . | . | . | . | G |
| 21-225_6D3_LC | . | . | . | . | . | . | . | . | . | . | . | . | . | . | . | . | . | . | . | . | . | . | . | . | T | . |
| 21-225_72G9_LC | . | . | . | . | . | . | . | . | . | . | . | . | . | . | . | . | . | . | . | . | . | . | . | . | . | . |
| 21-225_8E12_LC | . | . | . | . | . | . | . | . | . | . | . | . | . | . | . | . | . | . | . | . | . | . | . | . | . | . |
| 21-225_8G6_LC | . | . | . | . | . | . | . | . | . | . | . | . | . | . | . | . | . | . | . | . | . | . | . | . | . | . |
| 21-225_8H11_LC | . | . | . | . | . | . | . | . | . | . | . | . | . | P | . | . | . | . | . | . | . | . | . | . | . | . |
| 21-225_9E5_LC | . | . | . | . | . | . | Q | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | K_CDR1 | | | | | | | | | | | | | | | | | | K_FR2 | | |
| CONSENSUS | - | - | Q | S | I | S | - | - | - | - | - | - | S | Y | L | N | W | Y | Q | Q | K | P | G | K | A | P |
| 21-225_10F12_LC | . | . | . | N | . | . | . | . | . | . | . | . | . | . | . | . | . | . | . | . | . | . | . | . | . | . |
| 21-225_11A5_LC | . | . | . | N | . | Y | . | . | . | . | . | . | . | . | . | . | . | . | . | . | . | . | . | . | . | . |
| 21-225_14A11_LC | . | . | . | . | . | . | . | . | . | . | . | . | . | . | . | . | . | . | . | . | . | . | . | . | . | . |
| 21-225_158F4_LC | . | . | . | N | . | R | . | . | . | . | . | . | K | . | . | K | . | . | . | . | P | L | . | . | . | . |
| 21-225_16H6_LC | . | . | . | N | . | . | . | . | . | . | . | . | . | . | . | . | . | . | . | . | . | . | . | . | . | . |
| 21-225_17A12_LC | . | . | . | . | . | . | . | . | . | . | . | . | . | . | . | . | . | . | K | . | . | . | . | . | . | . |
| 21-225_17B4_LC | . | . | . | N | . | . | . | . | . | . | . | . | . | . | . | . | . | . | . | . | . | . | . | . | . | . |
| 21-225_17C6_LC | . | . | . | . | . | N | . | . | . | . | . | . | Y | . | . | . | . | . | . | . | . | . | . | . | . | . |
| 21-225_17F11_LC | . | K | T | . | . | N | . | . | . | . | . | . | N | . | . | . | . | . | . | . | . | . | . | . | . | . |
| 21-225_17F7_LC | . | . | T | . | . | . | . | . | . | . | . | . | . | F | . | . | . | . | . | . | . | . | . | . | . | . |
| 21-225_180F8_LC | . | . | . | T | . | L | . | . | . | . | . | . | . | . | . | . | . | . | . | . | . | . | . | . | . | . |
| 21-225_18E12_LC | . | . | . | . | . | . | . | . | . | . | . | . | . | . | . | . | . | . | . | . | . | . | . | . | . | . |
| 21-225_19A5_LC | . | . | . | N | . | I | . | . | . | . | . | . | N | . | . | . | . | . | . | . | . | . | . | . | . | . |
| 21-225_19B3_LC | . | . | . | N | . | I | . | . | . | . | . | . | N | . | . | . | . | . | . | . | . | . | . | . | . | . |
| 21-225_20A4_LC | . | . | . | . | . | I | . | . | . | . | . | . | N | . | . | . | . | . | . | . | N | . | . | . | S | . |
| 21-225_20F3_LC | . | . | . | . | . | . | . | . | . | . | . | . | . | . | . | Y | . | . | . | . | . | . | . | . | . | . |
| 21-225_21A2_LC | . | . | . | . | . | . | . | . | . | . | . | . | . | . | . | . | . | . | . | . | . | . | . | . | . | . |
| 21-225_21A6_LC | . | . | T | . | . | . | . | . | . | . | . | . | R | . | . | . | . | . | . | R | . | . | . | . | . | . |
| 21-225_21F4_LC | . | . | . | N | . | G | . | . | . | . | . | . | . | . | . | . | . | . | . | . | . | . | . | . | . | . |
| 21-225_222H4_LC | . | . | . | N | . | . | . | . | . | . | . | . | N | . | V | . | . | . | . | . | . | . | . | . | . | . |
| 21-225_222H8_LC | . | . | . | N | . | . | . | . | . | . | . | . | N | . | V | . | . | . | . | . | . | . | . | . | . | . |
| 21-225_22C5_LC | . | . | . | N | . | . | . | . | . | . | . | . | . | . | . | . | . | . | . | . | . | . | . | . | . | . |
| 21-225_22D5_LC | . | . | . | N | . | F | . | . | . | . | . | . | . | . | . | . | . | . | . | K | . | . | . | . | . | . |
| 21-225_22E4_LC | . | . | . | N | . | . | . | . | . | . | . | . | . | . | . | . | . | . | . | . | . | . | . | . | . | . |
| 21-225_22G10_LC | . | . | . | . | . | F | . | . | . | . | . | . | . | . | . | . | . | . | . | . | . | . | . | . | . | . |
| 21-225_22G3_LC | . | . | . | . | . | . | . | . | . | . | . | . | N | . | . | . | . | . | . | . | . | . | . | . | . | . |
| 21-225_22G5_LC | . | . | . | N | . | . | . | . | . | . | . | . | . | . | . | . | . | . | . | . | . | Q | . | . | . | . |
| 21-225_30E11_LC | . | . | . | . | . | . | . | . | . | . | . | . | N | . | . | . | . | . | . | . | Q | T | . | . | . | . |
| 21-225_33G12_LC | . | . | . | . | . | . | . | . | . | . | . | . | N | . | . | . | . | . | . | . | Q | T | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | I | S | - | - | - | - | - | - | S | Y | L | N | W | Y | Q | Q | K | P | G | K | A | P |
| 21-225_3B1_LC | . | . | . | . | . | N | . | . | . | . | . | . | N | . | . | . | . | . | . | . | . | . | . | . | . | . |
| 21-225_3E3_LC | . | . | . | . | . | R | . | . | . | . | . | . | . | . | . | . | . | . | . | . | . | . | . | . | . | R |
| 21-225_48C3_LC | . | . | . | . | . | I | . | . | . | . | . | . | . | . | . | I | . | . | . | . | . | . | . | . | . | . |
| 21-225_48D1_LC | . | . | . | . | . | . | . | . | . | . | . | . | . | . | . | I | . | . | . | . | . | . | . | . | . | . |
| 21-225_49G12_LC | . | . | . | . | . | . | . | . | . | . | . | . | T | . | . | M | . | . | . | . | . | . | . | . | . | . |
| 21-225_4B1_LC | . | . | H | T | . | R | . | . | . | . | . | . | T | . | . | . | . | . | . | . | . | . | . | . | . | . |
| 21-225_4H2_LC | . | . | . | N | . | . | . | . | . | . | . | . | . | . | . | . | . | . | . | . | . | . | . | . | . | . |
| 21-225_50B12_LC | . | . | . | . | . | . | . | . | . | . | . | . | . | . | . | . | . | . | . | M | . | . | . | . | . | . |
| 21-225_50H10_LC | . | . | . | . | . | Y | . | . | . | . | . | . | . | . | . | I | . | . | . | . | . | . | . | . | . | . |
| 21-225_50H8_LC | . | . | . | . | . | . | . | . | . | . | . | . | . | . | . | I | . | . | . | . | . | . | . | . | . | . |
| 21-225_51H10_LC | . | . | . | . | . | . | . | . | . | . | . | . | T | . | . | M | . | . | . | . | . | . | . | . | . | . |
| 21-225_53E2_LC | . | . | . | . | F | . | . | . | . | . | . | . | M | . | . | . | . | . | . | . | . | . | . | . | . | . |
| 21-225_56B2_LC | . | . | . | . | F | . | . | . | . | . | . | . | M | . | . | . | . | . | . | . | . | . | . | . | . | . |
| 21-225_57F2_LC | . | . | . | . | . | . | . | . | . | . | . | . | . | . | . | . | . | . | . | . | . | . | . | . | . | . |
| 21-225_5F7_LC | . | . | . | T | . | . | . | . | . | . | . | . | . | . | . | . | . | . | . | . | . | . | . | . | . | . |
| 21-225_5G2_LC | . | . | . | N | . | I | . | . | . | . | . | . | . | . | . | . | . | . | . | . | . | . | . | . | . | R |
| 21-225_60E12_LC | . | . | . | . | . | . | . | . | . | . | . | . | . | . | . | . | . | . | . | . | . | . | . | . | . | . |
| 21-225_61A1_LC | . | . | . | . | . | . | . | . | . | . | . | . | . | . | . | . | . | . | . | . | . | . | . | . | . | . |
| 21-225_62H1_LC | . | . | . | N | . | F | . | . | . | . | . | . | . | . | . | . | . | . | . | . | . | . | . | . | . | . |
| 21-225_62H10_LC | . | . | . | . | . | T | . | . | . | . | . | . | . | . | . | . | . | . | . | . | . | . | . | . | Y | . |
| 21-225_62H8_LC | . | . | . | . | . | F | . | . | . | . | . | . | . | . | . | . | . | . | . | . | N | . | . | . | . | . |
| 21-225_66A9_LC | . | . | . | N | . | F | . | . | . | . | . | . | . | . | . | . | . | . | . | . | . | . | . | . | . | . |
| 21-225_69C8_LC | . | . | . | T | . | Y | . | . | . | . | . | . | N | . | . | . | . | . | . | . | . | . | . | . | . | . |
| 21-225_6D3_LC | . | . | . | . | . | . | . | . | . | . | . | . | T | . | . | . | . | . | . | . | . | . | . | . | . | . |
| 21-225_72G9_LC | . | . | . | . | . | T | . | . | . | . | . | . | . | . | . | . | . | . | . | . | . | . | . | . | . | . |
| 21-225_8E12_LC | . | . | . | . | . | . | . | . | . | . | . | . | N | . | . | . | . | . | . | . | . | . | . | . | . | . |
| 21-225_8G6_LC | . | . | . | . | . | . | . | . | . | . | . | . | . | . | . | . | . | . | . | . | . | . | . | . | . | . |
| 21-225_8H11_LC | . | . | . | N | . | I | . | . | . | . | . | . | . | . | . | . | . | . | . | . | . | . | . | . | . | . |
| 21-225_9E5_LC | . | . | . | . | . | A | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_10F12_LC | . | . | . | . | . | . | . | . | . | . | . | . | . | . | . | . | . | . | . | . | . | . | . | . | . | . |
| 21-225_11A5_LC | . | . | . | . | . | . | . | . | . | . | . | . | . | . | . | . | . | . | . | . | . | . | . | . | . | . |
| 21-225_14A11_LC | . | . | . | . | F | . | . | . | . | . | . | . | . | . | . | . | . | . | . | N | . | . | . | . | . | . |
| 21-225_158F4_LC | . | . | . | . | . | T | . | . | . | . | . | . | . | . | . | . | T | . | . | . | . | . | . | . | . | . |
| 21-225_16H6_LC | . | . | . | . | . | . | . | . | . | . | . | . | . | . | . | . | . | . | . | . | . | . | . | . | . | . |
| 21-225_17A12_LC | . | . | . | . | . | . | . | . | . | . | . | . | . | . | . | . | . | . | . | . | . | . | . | . | . | . |
| 21-225_17B4_LC | . | . | . | . | F | Q | . | . | . | . | . | . | . | . | . | . | . | . | . | T | . | . | . | . | . | . |
| 21-225_17C6_LC | . | . | . | . | . | . | . | . | . | . | . | . | . | . | . | . | . | . | . | . | . | . | . | . | . | . |
| 21-225_17F11_LC | . | . | . | . | F | . | . | . | . | . | . | . | . | . | Y | . | . | . | . | . | . | . | . | . | . | . |
| 21-225_17F7_LC | . | . | . | . | . | . | . | . | . | . | . | . | . | . | . | . | . | . | . | . | . | . | . | . | . | . |
| 21-225_180F8_LC | . | . | . | . | . | G | . | . | . | . | . | . | . | . | V | . | . | . | . | . | . | . | . | . | . | . |
| 21-225_18E12_LC | T | . | . | . | . | . | . | . | . | . | . | . | . | . | . | . | . | . | . | . | . | . | . | . | . | . |
| 21-225_19A5_LC | . | . | . | . | G | V | . | . | . | . | . | . | . | . | . | . | N | . | . | G | . | . | . | . | . | . |
| 21-225_19B3_LC | N | . | . | . | . | V | . | . | . | . | . | . | . | . | . | . | . | . | . | . | . | . | . | . | . | . |
| 21-225_20A4_LC | . | . | . | . | . | . | . | . | . | . | . | . | . | . | . | . | . | . | M | . | . | . | . | . | . | . |
| 21-225_20F3_LC | . | . | . | . | . | . | . | . | . | . | . | . | . | . | . | . | . | . | . | . | . | . | . | . | . | . |
| 21-225_21A2_LC | . | . | . | . | . | . | . | . | . | . | . | . | . | . | . | . | . | . | . | . | . | . | . | . | . | . |
| 21-225_21A5_LC | . | . | . | . | . | . | . | . | . | . | . | . | . | . | . | . | . | . | . | . | . | . | . | . | . | . |
| 21-225_21F4_LC | . | . | . | . | F | . | . | . | . | . | . | . | . | . | . | . | . | . | . | . | . | . | . | . | . | . |
| 21-225_222H4_LC | . | F | . | . | . | T | . | . | . | . | . | . | . | . | . | . | . | . | . | . | . | . | . | S | . | . |
| 21-225_222H8_LC | . | F | . | . | . | I | . | . | . | . | . | . | . | . | . | . | . | . | . | . | . | . | . | S | . | . |
| 21-225_22C5_LC | . | . | . | . | . | . | . | . | . | . | . | . | . | . | . | . | V | . | . | M | . | I | . | . | . | . |
| 21-225_22D5_LC | . | . | . | . | . | . | . | . | . | . | . | . | . | . | . | . | . | . | . | . | . | . | . | . | . | . |
| 21-225_22E4_LC | . | . | . | . | . | . | . | . | . | . | . | . | . | . | . | . | . | . | . | . | . | . | . | . | . | . |
| 21-225_22G10_LC | . | . | . | . | . | . | . | . | . | . | . | . | . | . | . | . | . | . | . | . | . | . | . | . | . | . |
| 21-225_22G3_LC | . | . | . | . | F | . | . | . | . | . | . | . | . | . | . | . | . | . | . | . | . | . | . | . | . | . |
| 21-225_22G5_LC | . | . | . | . | . | . | . | . | . | . | . | . | . | . | . | . | . | . | . | N | . | . | . | . | . | . |
| 21-225_30E11_LC | . | F | . | . | . | . | . | . | . | . | . | . | . | . | . | . | . | . | . | . | . | . | . | . | . | . |
| 21-225_33G12_LC | . | F | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_3B1_LC | . | . | . | . | . | . | . | . | . | . | . | . | . | . | T | . | . | . | . | N | . | . | . | . | . | . |
| 21-225_3E3_LC | . | . | V | . | . | G | . | . | . | . | . | . | . | . | T | . | . | . | . | . | . | . | . | . | . | . |
| 21-225_48C3_LC | R | . | . | . | . | . | . | . | . | . | . | . | . | . | . | . | . | . | . | . | . | . | . | . | . | . |
| 21-225_48D1_LC | . | . | . | . | . | . | . | . | . | . | . | . | . | . | . | . | . | . | . | . | . | . | . | . | . | . |
| 21-225_49G12_LC | . | . | . | . | . | . | . | . | . | . | . | . | . | . | . | . | . | . | . | I | . | . | . | . | E | . |
| 21-225_4B1_LC | . | . | . | . | . | . | . | . | . | . | . | . | . | . | . | . | . | . | . | . | . | . | . | . | . | I |
| 21-225_4H2_LC | . | . | V | . | . | . | . | . | . | . | . | . | . | . | . | . | . | . | . | . | . | . | . | . | . | . |
| 21-225_50B12_LC | R | . | . | . | . | . | . | . | . | . | . | . | . | . | . | . | . | . | . | . | . | . | . | . | . | . |
| 21-225_50H10_LC | . | . | . | . | . | . | . | . | . | . | . | . | . | . | . | . | . | . | . | . | . | . | . | . | . | . |
| 21-225_50H8_LC | . | . | . | . | . | . | . | . | . | . | . | . | . | . | . | . | . | . | . | . | . | . | . | . | . | . |
| 21-225_51H10_LC | . | . | . | . | . | . | . | . | . | . | . | . | . | . | . | . | . | . | . | . | . | . | . | . | E | . |
| 21-225_53E2_LC | N | . | . | . | F | . | . | . | . | . | . | . | . | . | V | . | . | . | . | . | . | . | . | . | . | . |
| 21-225_56B2_LC | N | . | . | . | F | . | . | . | . | . | . | . | . | . | V | . | . | . | . | . | . | . | . | . | . | . |
| 21-225_57F2_LC | . | . | . | . | . | . | . | . | . | . | . | . | . | . | . | . | . | . | . | . | . | . | . | . | . | . |
| 21-225_5F7_LC | . | . | . | . | S | . | . | . | . | . | . | . | . | . | . | . | . | . | . | . | . | . | . | . | . | . |
| 21-225_5G2_LC | . | . | V | . | . | G | . | . | . | . | . | . | . | . | . | . | . | . | . | . | . | . | . | . | . | . |
| 21-225_60E12_LC | . | . | . | . | . | . | . | . | . | . | . | . | . | . | . | . | . | . | . | . | . | . | . | . | . | . |
| 21-225_61A1_LC | . | . | . | . | . | . | . | . | . | . | . | . | . | . | . | . | . | . | . | . | . | . | . | . | . | . |
| 21-225_62H1_LC | . | V | . | . | . | . | . | . | . | . | . | . | . | . | V | P | . | . | . | . | . | . | . | . | . | . |
| 21-225_62H10_LC | . | . | . | S | T | . | . | . | . | . | . | . | . | . | . | . | . | . | . | . | . | . | . | . | . | . |
| 21-225_62H8_LC | . | . | . | . | . | . | . | . | . | . | . | . | . | . | S | . | . | . | . | . | . | . | . | . | . | L |
| 21-225_66A9_LC | . | . | F | . | . | . | . | . | . | . | . | . | . | . | . | . | . | . | . | . | . | . | . | . | . | . |
| 21-225_69C8_LC | . | . | . | S | V | . | . | . | . | . | . | . | . | . | . | . | . | . | . | . | . | . | . | . | . | . |
| 21-225_6D3_LC | . | . | . | F | . | . | . | . | . | . | . | . | . | . | . | . | . | . | . | . | . | . | . | . | . | . |
| 21-225_72G9_LC | . | . | . | . | T | . | . | . | . | . | . | . | . | . | . | . | . | . | . | . | . | . | . | . | . | . |
| 21-225_8E12_LC | . | . | . | . | . | . | . | . | . | . | . | . | . | . | . | . | . | . | . | . | . | . | . | . | . | . |
| 21-225_8G6_LC | . | . | . | . | F | . | . | . | . | . | . | . | . | . | . | . | . | . | . | . | . | . | . | . | . | . |
| 21-225_8H11_LC | N | . | M | . | . | V | . | . | . | . | . | . | . | . | T | . | . | . | R | . | . | . | . | . | . | . |
| 21-225_9E5_LC | . | . | . | . | . | G | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | D | F | T | L | T | I | S | S | L | Q | P | E | D | F | A | T | Y |
| 21-225_10F12_LC | . | . | . | . | . | . | . | . | . | . | . | . | . | . | . | . | . | . | . | . | . | . | . | . | . | . |
| 21-225_11A5_LC | . | . | . | . | . | . | . | . | . | . | . | . | . | . | . | . | . | . | . | . | . | . | . | . | . | . |
| 21-225_14A11_LC | . | . | . | . | . | . | . | . | . | . | . | . | . | . | . | . | . | . | . | . | . | . | . | . | . | . |
| 21-225_158F4_LC | . | . | . | . | . | . | . | . | . | . | . | . | . | . | . | . | . | . | . | . | . | . | . | . | . | . |
| 21-225_16H6_LC | . | . | P | . | . | . | . | . | . | . | . | . | . | . | . | . | . | . | . | . | . | . | . | . | . | . |
| 21-225_17A12_LC | . | . | . | . | . | . | . | . | . | . | . | . | . | . | . | . | N | . | . | . | . | . | . | . | . | . |
| 21-225_17B4_LC | . | . | . | . | . | . | . | . | . | . | . | . | . | . | . | N | . | . | . | . | . | . | . | . | . | . |
| 21-225_17C6_LC | . | . | . | . | . | . | . | . | . | . | . | . | . | . | . | . | . | . | . | . | . | . | . | . | . | . |
| 21-225_17F11_LC | . | . | . | . | . | . | . | . | . | . | . | . | . | . | . | . | . | . | . | . | . | . | . | . | . | . |
| 21-225_17F7_LC | . | . | . | . | . | . | . | . | . | . | . | . | . | . | . | . | G | . | . | . | . | . | . | . | . | . |
| 21-225_180F8_LC | . | . | . | . | . | . | . | . | . | . | S | . | . | . | . | . | . | . | . | . | . | . | . | . | . | . |
| 21-225_18E12_LC | . | . | . | . | . | . | . | . | . | . | . | . | . | . | . | N | . | . | . | . | . | . | . | . | . | . |
| 21-225_19A5_LC | . | . | . | . | . | . | . | . | . | . | . | . | . | . | . | . | . | . | . | . | . | . | . | . | . | . |
| 21-225_19B3_LC | N | . | . | . | . | . | . | . | . | . | . | . | . | . | . | . | . | . | . | . | . | . | . | . | . | . |
| 21-225_20A4_LC | . | . | R | . | . | . | . | . | . | . | . | . | . | . | . | . | . | . | . | . | . | . | . | . | . | . |
| 21-225_20F3_LC | . | . | . | . | . | . | . | . | . | . | . | . | . | . | . | . | . | . | . | . | . | . | . | . | . | . |
| 21-225_21A2_LC | . | . | . | . | . | . | . | . | . | . | . | . | . | . | . | . | . | . | . | . | . | . | . | . | . | . |
| 21-225_21A5_LC | . | . | . | . | . | . | . | . | . | . | . | . | . | . | . | . | . | V | . | . | . | . | . | . | . | . |
| 21-225_21F4_LC | . | . | . | . | . | . | . | . | . | . | . | . | . | . | . | . | . | . | . | . | . | . | . | . | . | F |
| 21-225_222H4_LC | . | . | . | . | . | . | . | . | . | . | . | . | . | . | . | . | . | V | M | R | Q | . | . | I | . | . |
| 21-225_222H8_LC | . | . | . | . | . | . | . | . | . | . | . | . | . | . | . | . | . | V | M | R | Q | . | . | I | . | . |
| 21-225_22C5_LC | . | . | P | . | . | . | . | . | . | . | . | . | I | . | . | . | . | . | . | . | . | . | . | . | . | . |
| 21-225_22D5_LC | . | . | . | . | . | . | . | . | . | . | . | . | . | . | . | . | . | . | . | . | . | . | . | . | . | . |
| 21-225_22E4_LC | . | . | . | . | . | . | . | . | . | . | . | . | . | . | . | . | . | . | . | . | . | . | . | . | . | . |
| 21-225_22G10_LC | . | . | . | . | . | . | . | . | . | . | . | . | . | . | . | . | . | . | . | . | . | . | . | . | . | . |
| 21-225_22G3_LC | . | . | . | . | . | . | . | . | . | . | . | . | . | . | . | . | G | . | . | . | . | . | . | . | . | . |
| 21-225_22G5_LC | . | . | R | . | . | . | . | . | . | . | . | . | . | . | . | . | . | . | . | . | . | . | . | . | . | . |
| 21-225_30E11_LC | . | . | . | . | . | . | . | . | . | . | . | . | . | . | . | . | . | . | . | . | . | . | . | . | . | . |
| 21-225_33G12_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | | |
| CONSENSUS | S | G | S | G | S | G | - | - | T | D | F | T | L | T | I | S | S | L | Q | P | E | D | F | A | T | Y |
| 21-225_3B1_LC | . | . | . | . | . | . | . | . | . | . | . | . | . | . | . | . | . | . | . | . | . | . | . | . | . | . |
| 21-225_3E3_LC | . | . | . | . | . | . | . | . | . | . | . | . | . | . | . | . | . | . | . | . | . | . | . | . | . | . |
| 21-225_48C3_LC | . | A | . | . | . | . | . | . | . | . | . | . | . | . | . | . | . | . | . | . | . | . | . | . | S | . |
| 21-225_48D1_LC | . | A | . | . | . | . | . | . | . | . | . | . | . | . | . | . | . | . | . | . | . | . | . | . | S | . |
| 21-225_49G12_LC | . | A | . | . | . | . | . | . | . | . | . | . | . | . | . | . | . | . | . | . | . | . | . | . | . | . |
| 21-225_4B1_LC | N | . | . | . | . | . | . | . | . | . | . | . | . | . | . | T | N | . | . | . | . | . | . | . | . | . |
| 21-225_4H2_LC | . | . | R | . | . | . | . | . | . | . | . | . | . | . | . | Q | C | V | . | R | . | . | . | . | . | . |
| 21-225_50B12_LC | . | . | . | E | . | . | . | . | . | . | . | . | I | . | . | . | . | . | . | . | . | . | . | T | . | . |
| 21-225_50H10_LC | . | A | . | . | . | . | . | . | . | . | . | . | . | . | . | . | . | . | . | . | . | . | . | . | S | . |
| 21-225_50H8_LC | . | A | . | . | . | . | . | . | . | . | . | . | . | . | . | . | . | . | . | . | . | . | . | . | S | . |
| 21-225_51H10_LC | . | A | . | . | . | . | . | . | . | . | . | . | . | . | . | . | . | . | . | . | . | . | . | . | . | . |
| 21-225_53E2_LC | . | . | . | . | . | . | . | . | S | . | . | . | . | P | . | . | . | . | . | . | . | . | . | . | I | . |
| 21-225_56B2_LC | . | . | . | . | . | . | . | . | . | . | . | . | . | . | . | . | . | . | . | . | . | . | . | . | I | . |
| 21-225_57F2_LC | . | . | . | . | . | . | . | . | . | . | . | . | . | . | . | . | . | . | . | . | . | . | . | . | P | . |
| 21-225_5F7_LC | . | . | . | . | . | . | . | . | . | . | . | . | . | . | . | . | . | . | . | . | . | . | . | . | . | . |
| 21-225_5G2_LC | . | . | . | . | . | . | . | . | . | . | . | . | . | N | . | R | . | . | . | . | . | . | . | . | . | . |
| 21-225_60E12_LC | . | . | . | . | . | . | . | . | . | . | . | . | . | . | . | . | . | . | . | . | . | . | . | . | P | . |
| 21-225_61A1_LC | . | . | . | . | . | . | . | . | . | . | . | . | . | . | . | . | . | . | . | . | . | . | . | . | P | . |
| 21-225_62H1_LC | . | . | . | . | . | . | . | . | . | . | . | . | . | . | . | . | . | . | . | . | . | . | . | . | . | . |
| 21-225_62H10_LC | . | . | . | . | . | . | . | . | . | . | . | . | . | . | . | . | . | . | . | . | . | . | . | . | . | . |
| 21-225_62H8_LC | . | N | . | . | . | . | . | . | . | . | . | I | . | . | . | . | . | . | . | . | . | . | . | . | . | . |
| 21-225_66A9_LC | . | . | . | . | . | . | . | . | . | . | . | . | . | . | . | . | . | . | . | . | . | . | . | . | . | . |
| 21-225_69C8_LC | . | . | . | . | . | . | . | . | . | . | . | . | V | . | . | . | . | . | . | . | . | . | . | . | . | . |
| 21-225_6D3_LC | . | . | . | . | . | . | . | . | . | . | . | . | . | . | . | . | . | . | . | . | . | . | . | . | . | . |
| 21-225_72G9_LC | . | . | . | . | . | . | . | . | . | . | . | . | . | . | . | . | . | . | . | . | . | . | . | . | . | . |
| 21-225_8E12_LC | . | . | . | . | . | . | . | . | . | . | . | . | . | . | . | . | . | . | . | . | . | . | . | . | . | . |
| 21-225_8G6_LC | . | . | . | . | . | . | . | . | . | . | . | . | . | . | . | . | . | . | . | . | . | . | . | . | . | . |
| 21-225_8H11_LC | . | . | . | . | . | . | . | . | . | S | . | . | . | . | . | . | . | . | . | . | . | . | . | . | . | . |
| 21-225_9E5_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_10F12_LC | . | . | . | . | . | . | N | . | . | . | . | . | . | . | . | . | . | . | . | . | . | . | . | . | . | . |
| 21-225_11A5_LC | . | . | . | . | . | . | N | . | . | . | . | . | . | . | . | . | . | . | . | . | . | . | . | . | . | . |
| 21-225_14A11_LC | . | . | . | . | T | . | . | . | . | . | . | . | . | . | . | . | . | . | . | . | . | . | . | . | . | . |
| 21-225_158F4_LC | . | . | . | . | . | . | N | . | . | . | . | . | . | . | . | . | . | . | . | . | . | . | . | . | . | . |
| 21-225_16H6_LC | . | . | . | . | . | . | X | . | . | . | . | . | . | . | . | . | . | . | . | . | . | . | . | . | . | . |
| 21-225_17A12_LC | . | . | . | . | . | . | N | . | . | . | . | . | . | . | . | . | . | . | . | . | . | . | . | . | . | . |
| 21-225_17B4_LC | F | . | . | . | . | . | X | . | . | . | . | . | . | . | . | . | . | . | . | . | . | . | . | . | . | . |
| 21-225_17C6_LC | F | . | . | . | . | . | X | . | . | . | . | . | . | . | . | . | . | . | . | . | . | . | . | . | . | . |
| 21-225_17F11_LC | . | . | . | . | T | . | . | . | . | . | . | . | . | . | . | . | . | . | . | . | . | . | . | . | . | . |
| 21-225_17F7_LC | F | . | . | . | . | . | X | . | . | . | . | . | . | . | . | . | . | . | . | . | . | . | . | . | . | . |
| 21-225_180F8_LC | . | . | . | . | . | . | . | . | . | . | . | . | . | . | . | . | . | . | . | . | . | . | . | . | . | . |
| 21-225_18E12_LC | F | . | . | . | . | . | X | . | . | . | . | . | . | . | . | . | . | . | . | . | . | . | . | . | . | . |
| 21-225_19A5_LC | . | . | . | . | . | . | . | . | . | . | . | . | . | . | . | . | . | . | . | . | . | . | . | . | . | . |
| 21-225_19B3_LC | . | . | . | . | . | . | . | . | . | . | . | . | . | . | . | . | . | . | . | . | . | . | . | . | . | . |
| 21-225_20A4_LC | F | . | . | . | . | . | X | . | . | . | . | . | . | . | . | . | . | . | . | . | . | . | . | . | . | . |
| 21-225_20F3_LC | . | . | . | . | . | . | . | . | . | . | . | . | . | . | . | . | . | . | . | . | . | . | . | . | . | . |
| 21-225_21A2_LC | F | . | . | . | . | . | X | . | . | . | . | . | . | . | . | . | . | . | . | . | . | . | . | . | . | . |
| 21-225_21A5_LC | . | . | . | . | . | . | N | . | . | . | . | . | . | . | . | . | . | . | . | . | . | . | . | . | . | . |
| 21-225_21F4_LC | F | . | . | . | . | . | X | . | . | . | . | . | . | . | . | . | . | . | . | . | . | . | . | . | . | . |
| 21-225_222H4_LC | . | . | . | Y | . | . | F | . | . | . | . | . | . | . | . | . | . | . | . | . | . | . | . | . | . | . |
| 21-225_222H8_LC | . | . | . | Y | . | . | F | . | . | . | . | . | . | . | . | . | . | . | . | . | . | . | . | . | . | . |
| 21-225_22C5_LC | . | . | . | . | . | . | X | . | . | . | . | . | . | . | . | . | . | . | . | . | . | . | . | . | . | . |
| 21-225_22D5_LC | . | . | . | . | . | . | . | . | . | . | . | . | . | . | . | . | . | . | . | . | . | . | . | . | . | . |
| 21-225_22E4_LC | . | . | . | . | . | . | N | . | . | . | . | . | . | . | . | . | . | . | . | . | . | . | . | . | . | . |
| 21-225_22G10_LC | F | . | . | . | . | F | X | . | . | . | . | . | . | . | . | . | . | . | . | . | . | . | . | . | . | . |
| 21-225_22G3_LC | F | . | . | . | . | F | X | . | . | . | . | . | . | . | . | . | . | . | . | . | . | . | . | . | . | . |
| 21-225_22G5_LC | . | . | . | . | . | . | X | . | . | . | . | . | . | . | . | . | . | . | . | . | . | . | . | . | . | . |
| 21-225_30E11_LC | . | . | . | . | . | . | . | . | . | . | . | . | . | . | . | . | . | . | . | . | . | . | . | . | . | . |
| 21-225_33G12_LC | . | . | . | . | . | . | . | . | . | . | . | . | . | . | . | . | . | . | . | . | . | . | . | . | . | . |

4847

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_3B1_LC | . | . | . | . | . | . | . | . | . | . | . | . | . | . | . | . | . | . | . | . | . | . | . | . | . | . |
| 21-225_3E3_LC | . | . | . | . | . | . | . | . | . | . | . | . | . | . | . | . | . | . | . | . | . | . | . | . | . | . |
| 21-225_48C3_LC | | | . | | T | N | . | . | . | . | . | . | . | . | . | . | . | . | . | . | . | . | . | . | | . |
| 21-225_48D1_LC | | | . | | . | N | . | . | . | . | . | . | . | . | . | . | . | . | . | . | . | . | . | . | | |
| 21-225_49G12_LC | | | | | . | . | . | . | . | . | . | . | . | . | . | . | . | . | . | . | . | . | . | . | | |
| 21-225_4B1_LC | | | . | | . | . | N | . | . | . | . | . | . | . | . | . | . | . | . | . | . | . | . | . | . | . |
| 21-225_4H2_LC | | | . | | . | . | N | . | . | . | . | . | . | . | . | . | . | . | . | . | . | . | . | . | . | . |
| 21-225_50B12_LC | . | | . | . | . | . | I | . | . | . | . | . | . | . | . | . | . | . | . | . | . | . | . | . | . | . |
| 21-225_50H10_LC | . | . | . | . | . | N | . | . | . | . | . | . | . | . | . | . | . | . | . | . | . | . | . | . | . | . |
| 21-225_50H8_LC | . | . | . | . | . | N | . | . | . | . | . | . | . | . | . | . | . | . | . | . | . | . | . | . | . | . |
| 21-225_51H10_LC | . | | . | . | . | . | . | . | . | . | . | . | . | . | . | . | . | . | . | . | . | . | . | . | . | . |
| 21-225_53E2_LC | F | . | . | | . | F | | | | | | | | | | | | | | | | | | | | |
| 21-225_56B2_LC | F | . | . | | . | . | . | | | | | | | | | | | | | | | | | | | |
| 21-225_57F2_LC | . | . | . | . | . | . | N | . | . | . | . | . | . | . | . | . | . | . | . | . | . | . | . | . | . | . |
| 21-225_5F7_LC | . | . | . | . | . | . | N | . | . | . | . | . | . | . | . | . | . | . | . | . | . | . | . | . | . | . |
| 21-225_5G2_LC | . | . | . | . | . | . | . | . | . | . | . | . | . | . | . | . | . | . | . | . | . | . | . | . | . | . |
| 21-225_60E12_LC | . | . | . | . | . | . | N | . | . | . | . | . | . | . | . | . | . | . | . | . | . | . | . | . | . | . |
| 21-225_61A1_LC | . | . | . | . | . | . | N | . | . | . | . | . | . | . | . | . | . | . | . | . | . | . | . | . | . | . |
| 21-225_62H1_LC | . | | . | | . | . | . | . | . | . | . | . | . | . | . | . | . | . | . | . | . | . | . | . | . | . |
| 21-225_62H10_LC | . | . | . | | . | N | . | . | . | . | . | . | . | . | . | . | . | . | . | . | . | . | . | . | . | . |
| 21-225_62H8_LC | | | | | | . | . | . | . | . | . | . | . | . | . | . | . | . | . | . | . | . | . | . | . | . |
| 21-225_66A9_LC | | | . | T | . | . | . | . | . | . | . | . | . | . | . | . | . | . | . | . | . | . | . | . | . | . |
| 21-225_69C8_LC | . | | . | . | . | . | . | . | . | . | . | . | . | . | . | . | . | . | . | . | . | . | . | . | . | . |
| 21-225_6D3_LC | F | . | . | . | . | N | . | . | . | . | . | . | . | . | . | . | . | . | . | . | . | . | . | . | . | . |
| 21-225_72G9_LC | | | . | | N | . | . | . | . | . | . | . | . | . | . | . | . | . | . | . | . | . | . | . | . | . |
| 21-225_8E12_LC | F | . | . | . | . | N | . | . | . | . | . | . | . | . | . | . | . | . | . | . | . | . | . | . | . | . |
| 21-225_8G6_LC | F | . | . | . | . | N | . | . | . | . | . | . | . | . | . | . | . | . | . | . | . | . | . | . | . | . |
| 21-225_8H11_LC | . | . | . | . | . | . | . | . | . | . | . | . | . | . | . | . | . | . | . | . | . | . | . | . | . | . |
| 21-225_9E5_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | P | P | F | T | F | G | P | G | T | K | V | D | I | K | R |
| 21-225_10F12_LC | | | | T | . | S | | | | | | | | | | | | | |
| 21-225_11A5_LC | | | | T | . | L | | | | | | | | | | | | | |
| 21-225_14A11_LC | | | | S | . | . | | | | A | | | | | | | | | |
| 21-225_158F4_LC | | | | . | I | S | | | | | | | | | | | | | |
| 21-225_16H8_LC | | | | T | . | L | | | | | | | | | | | P | | |
| 21-225_17A12_LC | | | | T | . | L | | | | | | | | | | | | | |
| 21-225_17B4_LC | | | | T | . | S | | | | | | | | | | | | | |
| 21-225_17C6_LC | | | | T | . | S | | | | | | | | | | | | | |
| 21-225_17F11_LC | | | | T | . | L | A | | | | | | | | | | | | |
| 21-225_17F7_LC | | | | T | . | S | | | | | | | | | | | | | |
| 21-225_180F8_LC | | | | . | T | . | | | | | | | | | | | | | |
| 21-225_18E12_LC | | | | T | . | S | | | | | | | | | | | | | |
| 21-225_19A5_LC | | | | . | T | . | | | | | | | | | | | | | |
| 21-225_19B3_LC | | | | . | T | . | | | | | | | | | | | | | |
| 21-225_20A4_LC | | | | T | . | L | | | | | | | | | | | P | | |
| 21-225_20F3_LC | | | | A | . | . | | | | | | | | | | | | | |
| 21-225_21A2_LC | | | | T | . | S | | | | | | | | | | | | | |
| 21-225_21A5_LC | | | | . | I | S | | | | | | | | | | | | | |
| 21-225_21F4_LC | | | | T | . | S | | | | | | | | | | | P | | |
| 21-225_222H4_LC | | | | . | T | . | | | | | S | | | | | | | | |
| 21-225_222H8_LC | | | | . | T | . | | | | | S | | | | | | | | |
| 21-225_22C5_LC | | | | T | . | L | | | | | | | | | | | P | | |
| 21-225_22D5_LC | | | | T | . | L | | | | | | | | | | | | | |
| 21-225_22E4_LC | | | | T | . | L | | | | | | | | | | | | | |
| 21-225_22G10_LC | | | | T | . | L | | | | | | | | | | | P | | |
| 21-225_22G3_LC | | | | T | . | S | | | | | | | | | | | | | |
| 21-225_22G5_LC | | | | T | . | L | | | | | | | | | | | P | | |
| 21-225_30E11_LC | | | | . | T | . | | | | | | | | | | | | | |
| 21-225_33G12_LC | | | | . | T | . | | | | | | | | | | | | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | P | P | F | T | F | G | P | G | T | K | V | D | I | K | R |
| 21-225_3B1_LC | . | | . | T | . | L | . | . | . | . | . | . | . | . | . | . | . | N | . |
| 21-225_3E3_LC | . | | . | T | . | L | . | . | . | . | . | . | . | . | . | . | . | . | . |
| 21-225_48C3_LC | . | | . | . | I | . | . | . | . | . | . | . | . | . | . | . | . | . | . |
| 21-225_48D1_LC | . | | . | . | I | . | . | . | . | . | . | . | . | . | . | . | . | | |
| 21-225_49G12_LC | . | | . | . | I | . | . | . | . | . | . | . | . | . | . | . | . | . | |
| 21-225_4B1_LC | . | | . | . | I | S | . | . | . | . | . | . | . | . | . | . | . | . | . |
| 21-225_4H2_LC | . | | . | T | . | L | . | . | . | . | . | . | . | . | . | . | . | . | . |
| 21-225_50B12_LC | . | | . | . | A | . | . | . | . | . | . | . | . | . | . | . | . | . | . |
| 21-225_50H10_LC | . | | . | . | I | . | . | . | . | . | . | . | . | . | . | . | . | . | . |
| 21-225_50H8_LC | . | | . | . | L | . | . | . | . | . | . | . | . | . | . | . | . | . | . |
| 21-225_51H10_LC | . | | . | . | I | . | . | . | . | . | . | . | . | . | . | . | . | . | . |
| 21-225_53E2_LC | . | | . | . | T | . | . | . | . | . | . | . | . | . | . | . | . | . | . |
| 21-225_56B2_LC | . | | . | . | T | . | . | . | . | . | . | . | . | . | . | . | . | . | . |
| 21-225_57F2_LC | . | | . | . | I | S | . | . | . | . | . | . | . | . | . | . | . | . | . |
| 21-225_5F7_LC | . | | . | T | . | L | . | . | . | . | . | . | . | . | . | V | . | . | . |
| 21-225_5G2_LC | . | | . | T | . | L | . | . | . | . | . | . | . | . | . | . | . | . | . |
| 21-225_60E12_LC | . | | . | . | I | S | . | . | . | . | . | . | . | . | . | . | . | . | . |
| 21-225_61A1_LC | . | | . | . | I | S | . | . | . | . | . | . | . | . | . | . | . | . | . |
| 21-225_62H1_LC | . | | . | . | T | . | . | . | . | . | . | . | . | . | . | . | . | . | . |
| 21-225_62H10_LC | . | | . | . | P | . | . | . | . | . | . | . | . | N | . | . | P | . | . |
| 21-225_62H8_LC | . | | . | . | T | . | . | . | . | . | . | . | . | . | . | . | . | . | . |
| 21-225_66A9_LC | . | | . | . | V | . | . | . | . | . | . | . | . | . | . | . | P | . | . |
| 21-225_69C8_LC | . | | . | . | T | . | . | . | . | . | . | . | . | . | . | . | . | . | . |
| 21-225_6D3_LC | . | | . | T | . | P | . | . | . | . | . | . | . | . | . | . | . | . | . |
| 21-225_72G9_LC | . | | . | . | P | . | . | . | . | . | . | . | . | . | . | . | . | . | . |
| 21-225_8E12_LC | . | | . | T | . | P | . | . | . | . | . | . | . | . | . | . | . | . | . |
| 21-225_8G6_LC | . | | . | T | . | P | . | . | . | . | . | . | . | . | . | . | . | . | . |
| 21-225_8H11_LC | . | | . | . | T | . | . | . | . | . | . | . | . | . | . | . | . | . | . |
| 21-225_9E5_LC | . | | . | T | . | L | . | S | . | . | . | . | . | . | . | . | . | . | . |

**FIGURE 57**

Table 107.    Consensus 27 - VK4|B3/JK2 (SEQ ID NO: 50324):

DIVMTQSPDSLAVSLGERATINCKSS--QSVLYSSNNNNYLAWYQQKPGQPPKLLIYW--------ASTRESGVPDRFSGSGSG--
TDFTLTISSLQAEDVAVYYCQQYYS---------------------TPCSFGQGTKLEIKR wherein:

K at position 24 can be substituted with R or T

S at position 26 can be substituted with I

S at position 30 can be substituted with N

V at position 31 can be substituted with I

Y at position 33 can be substituted with H, S or F

S at position 34 can be substituted with N, I, R or H

N at position 36 can be substituted with II

N at position 37 can be substituted with S or D

N at position 38 can be substituted with Y, K, H, A, M or Q

N at position 39 can be substituted with K

Y at position 40 can be substituted with F

A at position 42 can be substituted with T or D

A at position 67 can be substituted with T, G or S

S at position 68 can be substituted with F

T at position 69 can be substituted with I

E at position 71 can be substituted with K or D

S at position 72 can be substituted with F

Q at position 107 can be substituted with H

FIGURE 57

Q at position 108 can be substituted with H

Y at position 109 can be substituted with S

Y at position 110 can be substituted with F, K, or N

S at position 111 can be substituted with T, I, null (-) or N

T at position 135 can be substituted with S, I, R, A, G or N

P at position 136 can be substituted with S

C at position 137 can be substituted with Y, G, L or P

S at position 138 can be substituted with K or N

Xaa Ser Xaa Gln Xaa Xaa Leu Xaa Xaa Ser Xaa Xaa Xaa Xaa Xaa Leu Xaa (SEQ ID NO: 50156)

Trp Xaa Xaa Xaa Arg Xaa Xaa (SEQ ID NO: 50157)

Xaa Xaa Xaa Xaa Xaa Xaa Xaa Xaa Xaa (SEQ ID NO: 50158)

Lys Ser Ser Gln Ser Val Leu Tyr Ser Ser Asn Asn Asn Asn Tyr Leu Ala (SEQ ID NO: 50642)

Trp Ala Ser Thr Arg Glu Ser (SEQ ID NO: 50643)

Gln Gln Tyr Tyr Ser Thr Pro Cys Ser (SEQ ID NO: 50644)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | V | M | T | Q | S | P | D | S | L | A | V | S | L | G | E | R | A | T | I | N | C | K | S | S |
| 21-225_146C9_LC | . | . | . | . | . | . | . | . | . | . | . | . | . | . | . | . | . | . | . | . | . | . | . | . | . | . |
| 21-225_146D4_LC | . | . | . | . | . | . | . | . | . | . | . | . | . | . | . | . | . | . | . | . | . | . | . | . | . | . |
| 21-225_146F9_LC | . | . | . | . | . | . | . | . | . | . | . | . | . | . | . | . | . | . | . | . | . | . | . | . | . | . |
| 21-225_147D5_LC | . | . | . | . | . | . | . | . | . | . | . | . | . | . | . | . | . | . | . | . | . | . | . | . | . | . |
| 21-225_150D2_LC | . | . | . | . | . | . | . | . | . | . | . | . | . | . | . | . | . | . | . | . | . | K | . | . | . | . |
| 21-225_152D10_LC | G | . | . | . | . | . | . | . | . | . | . | . | . | . | . | . | A | . | . | . | . | D | . | . | . | . |
| 21-225_152E12_LC | A | V | . | . | . | . | . | . | . | . | . | . | . | . | . | . | . | . | . | . | . | . | . | T | . | . |
| 21-225_152H3_LC | . | . | . | . | . | . | . | . | . | . | . | . | . | . | . | . | K | . | . | . | . | . | . | . | . | . |
| 21-225_153B9_LC | G | . | . | . | . | . | P | . | . | . | . | . | . | . | . | . | . | . | . | . | . | . | . | . | . | . |
| 21-225_153F11_LC | . | . | . | . | . | . | . | . | . | . | . | . | . | . | . | . | . | . | . | . | . | . | . | . | . | . |
| 21-225_154H6_LC | . | . | . | . | . | . | . | . | . | . | . | . | . | . | . | . | . | . | . | . | . | . | . | . | . | . |
| 21-225_155B6_LC | . | . | . | . | . | . | . | . | . | . | . | . | . | . | . | . | . | . | . | . | . | . | . | . | . | . |
| 21-225_155E5_LC | . | . | . | . | . | . | . | . | . | . | . | . | . | . | . | . | . | . | . | . | . | . | . | . | . | . |
| 21-225_156H1_LC | . | . | . | . | . | . | . | . | . | . | . | . | . | . | . | . | . | . | . | . | . | . | . | . | . | . |
| 21-225_160A4_LC | . | . | . | . | . | . | . | . | . | . | . | . | . | . | . | . | . | . | . | . | . | . | . | . | . | . |
| 21-225_160A7_LC | . | . | L | . | . | . | . | . | . | P | . | . | . | . | . | . | . | . | . | . | . | . | . | . | . | . |
| 21-225_172E11_LC | . | . | . | . | . | . | . | . | . | . | . | . | . | . | . | . | . | . | . | . | . | . | . | . | . | . |
| 21-225_224F11_LC | N | . | . | . | . | . | . | . | . | . | . | . | . | . | . | D | . | . | . | . | . | . | . | . | . | . |
| 21-225_225B9_LC | . | . | . | . | . | . | . | . | . | . | . | . | . | . | . | . | . | . | . | . | . | . | . | . | . | . |
| 21-225_225F12_LC | . | . | . | . | . | . | . | . | . | . | . | . | . | . | . | . | . | . | . | . | . | . | . | . | . | . |
| 21-225_225H12_LC | . | . | . | . | . | . | . | . | . | . | . | . | . | . | . | . | . | . | . | . | . | . | . | . | . | . |
| 21-225_226D6_LC | . | . | . | . | . | . | . | . | . | . | . | . | . | . | . | . | . | . | . | . | . | . | . | R | . | . |
| 21-225_227D11_LC | . | . | . | . | . | . | . | . | . | . | . | . | . | . | . | O | . | . | . | . | . | . | . | . | . | . |
| 21-225_227E6_LC | . | . | . | . | . | . | . | . | . | . | T | . | . | . | . | . | . | . | . | . | . | . | . | . | . | . |
| 21-225_227G9_LC | . | . | . | . | . | . | . | . | . | . | . | . | . | . | . | . | . | . | . | . | . | . | . | . | . | . |
| 21-225_22D2_LC | . | . | . | . | . | . | . | . | . | . | . | . | . | . | . | . | . | . | . | . | . | T | . | . | . | . |
| 21-225_25E12_LC | . | . | L | . | . | . | . | P | . | . | . | M | . | . | . | . | . | . | . | . | . | . | . | . | . | . |
| 21-225_32A1_LC | . | . | . | . | . | . | . | . | . | . | . | . | . | . | . | . | . | . | . | . | . | . | . | . | . | . |
| 21-225_32A5_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | S | P | D | S | L | A | V | S | L | G | E | R | A | T | I | N | C | K | S | S |
| 21-225_33D1_LC | . | . | . | . | . | . | . | . | . | . | . | S | . | . | . | . | . | . | . | . | . | . | . | . | . | ; |
| 21-225_34H7_LC | . | . | . | . | . | . | . | . | . | . | . | . | . | . | . | . | . | . | . | . | . | . | . | . | . | . |
| 21-225_57F12_LC | . | . | . | . | . | . | . | . | . | . | . | . | . | . | . | . | . | . | . | . | . | . | . | . | . | . |
| 21-225_59E1_LC | . | . | . | . | . | . | . | . | . | . | . | . | . | . | . | . | . | . | . | . | . | . | . | . | . | . |
| 21-225_60F3_LC | . | . | . | . | . | . | . | . | . | . | . | T | . | . | . | . | . | . | . | . | . | . | . | . | . | . |
| 21-225_61E3_LC | . | . | . | . | . | . | . | . | . | . | . | . | . | . | . | . | . | . | . | . | . | . | . | . | . | . |
| 21-225_63F4_LC | . | . | . | . | . | . | . | . | . | . | . | . | . | . | . | . | . | . | . | . | . | . | . | . | . | . |
| 21-225_71F3_LC | . | . | . | . | . | . | . | . | . | . | . | . | . | . | . | . | A | . | . | . | . | . | . | . | . | . |
| 21-225_71G3_LC | . | . | . | . | . | . | . | . | . | . | . | . | . | . | . | . | . | . | V | A | . | . | . | . | . | . |
| 21-225_74A8_LC | . | . | . | . | . | . | . | . | . | . | . | . | . | . | . | . | . | . | . | . | . | . | . | . | . | . |
| 21-225_74C10_LC | . | . | . | . | . | . | . | . | . | . | . | . | . | . | . | . | . | . | . | . | . | . | . | . | . | . |
| 21-225_74C12_LC | . | . | . | . | . | . | . | . | . | . | . | . | . | . | . | . | . | . | . | . | . | . | . | . | . | . |
| 21-225_77D12_LC | . | . | . | . | . | . | . | . | . | . | . | . | . | . | . | . | A | . | . | . | . | . | . | X | . | . |
| 21-225_78E6_LC | . | . | . | . | . | . | . | . | . | . | . | . | . | . | . | . | . | . | . | . | . | . | . | . | . | . |
| 21-225_79G7_LC | . | . | . | . | . | . | . | . | . | . | . | . | . | . | . | . | . | . | . | . | . | . | . | . | . | . |
| 21-225_85C11_LC | . | . | . | . | . | . | . | . | . | . | . | . | . | . | . | . | . | . | . | . | . | . | . | . | . | . |
| 21-225_87E10_LC | . | . | . | . | . | . | . | . | . | . | . | . | . | . | . | . | . | . | . | . | . | X | . | . | . | . |
| 21-225_8B11_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | K_FR2 | | | |
| CONSENSUS | - | - | Q | S | V | L | Y | S | S | N | N | N | N | Y | L | A | W | Y | Q | Q | K | P | G | Q | P | P |
| 21-225_146C9_LC | . | . | . | . | . | . | H | . | . | . | . | Y | . | . | . | . | . | . | . | . | . | . | . | . | . | . |
| 21-225_146D4_LC | . | . | . | . | . | . | . | . | . | . | . | . | . | . | . | . | . | . | . | . | . | . | . | . | . | . |
| 21-225_146F9_LC | . | . | N | I | . | . | H | . | . | . | . | . | . | . | . | . | . | . | . | . | . | . | . | . | . | . |
| 21-225_147D5_LC | . | . | . | . | . | . | . | . | . | . | . | . | . | . | . | . | . | . | . | . | . | . | . | . | . | . |
| 21-225_150D2_LC | . | . | . | . | . | . | . | R | . | . | S | R | R | . | . | T | . | . | . | . | . | . | . | . | . | . |
| 21-225_152D10_LC | . | . | . | . | . | . | . | . | . | . | . | K | . | . | . | . | . | . | . | . | . | . | . | . | . | . |
| 21-225_152E12_LC | . | . | . | I | . | . | H | . | . | . | . | Y | . | . | . | . | . | . | . | . | . | . | . | . | . | . |
| 21-225_152H3_LC | . | . | . | . | . | . | H | . | . | . | . | Y | . | . | . | T | . | . | . | . | . | . | . | . | . | . |
| 21-225_153B9_LC | . | . | . | . | . | . | . | . | . | . | . | K | . | . | . | . | . | . | . | . | . | . | . | . | . | H |
| 21-225_153F11_LC | . | . | . | . | . | . | . | . | . | . | . | Y | K | . | . | . | . | . | . | . | . | . | . | . | . | . |
| 21-225_154H6_LC | . | . | . | . | . | . | H | . | . | . | . | Y | . | . | . | . | . | . | . | . | . | . | . | . | . | . |
| 21-225_155B6_LC | . | . | . | . | . | . | H | . | . | . | . | Y | . | . | . | . | . | . | . | . | . | . | . | . | . | . |
| 21-225_155E5_LC | . | . | . | . | . | . | S | . | . | . | . | . | . | . | . | . | . | . | . | . | . | . | . | . | . | . |
| 21-225_156H1_LC | . | . | . | . | . | . | . | . | . | . | . | . | . | . | . | . | . | . | . | . | . | . | . | . | . | . |
| 21-225_160A4_LC | . | . | . | . | . | . | H | . | . | . | . | . | . | . | . | . | . | . | . | . | . | . | . | . | . | . |
| 21-225_160A7_LC | . | . | . | . | . | . | S | N | . | H | . | . | . | . | . | . | . | . | . | . | R | . | . | H | . | H |
| 21-225_172E11_LC | . | . | . | . | . | . | H | . | . | . | . | . | . | . | . | . | . | . | . | . | . | . | . | . | . | . |
| 21-225_224F11_LC | . | . | . | . | . | . | S | . | . | . | . | H | . | . | . | . | . | . | . | . | . | . | . | . | . | . |
| 21-225_225B9_LC | . | . | . | . | . | . | . | . | . | . | . | . | . | . | . | . | . | . | . | . | . | . | . | . | . | . |
| 21-225_225F12_LC | . | . | . | . | . | . | . | . | . | . | . | Q | . | . | . | . | . | . | . | . | . | . | . | . | . | . |
| 21-225_225H12_LC | . | . | . | . | . | . | . | N | . | . | . | . | . | . | . | . | . | . | . | . | . | . | . | . | . | . |
| 21-225_226D6_LC | . | . | . | I | . | . | I | . | . | . | . | K | . | . | . | . | . | . | . | . | . | . | . | . | . | . |
| 21-225_227D11_LC | . | . | . | . | . | . | H | . | . | . | . | . | . | . | . | . | . | . | . | . | . | . | . | . | . | . |
| 21-225_227E6_LC | . | . | . | . | . | . | . | . | . | . | S | K | . | . | . | . | . | . | . | . | . | . | . | . | . | . |
| 21-225_227G9_LC | . | . | . | . | . | . | H | . | . | . | . | . | . | . | . | . | . | . | . | . | . | . | . | . | . | . |
| 21-225_22D2_LC | . | . | . | . | . | . | H | . | . | . | . | . | . | . | . | . | . | . | . | . | . | . | . | . | . | . |
| 21-225_25E12_LC | . | . | . | . | . | . | R | . | . | . | . | Y | . | . | . | . | . | . | . | . | . | . | R | . | . | . |
| 21-225_32A1_LC | . | . | . | . | . | . | . | . | . | . | . | Y | . | . | . | . | . | . | . | L | . | . | . | . | . | . |
| 21-225_32A5_LC | . | . | . | . | . | . | H | . | . | . | . | K | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | K_FR2 | | | |
| CONSENSUS | - | - | Q | S | V | L | Y | S | S | N | N | N | N | Y | L | A | W | Y | Q | Q | K | P | G | Q | P | P |
| 21-225_33D1_LC | . | . | . | . | . | . | R | . | . | . | . | K | . | . | . | T | . | . | . | . | . | . | . | . | . | R |
| 21-225_34H7_LC | . | . | . | . | . | . | . | . | . | . | . | Y | . | . | . | . | . | . | . | L | . | . | . | . | . | . |
| 21-225_57F12_LC | . | . | . | . | . | . | F | . | . | . | . | Y | . | . | . | . | . | . | . | . | T | . | . | . | . | . |
| 21-225_59E1_LC | . | . | . | . | . | . | . | . | . | . | . | . | . | . | . | . | . | . | . | R | . | . | . | . | . | . |
| 21-225_60F3_LC | . | . | . | . | . | . | . | . | . | . | . | K | . | . | . | . | . | . | . | . | . | . | . | . | . | . |
| 21-225_61E3_LC | . | . | . | . | . | . | H | I | . | . | . | . | . | . | . | . | . | . | . | Q | . | . | . | . | . | . |
| 21-225_63F4_LC | . | . | . | . | . | . | S | . | . | . | . | . | . | . | . | . | . | . | . | . | . | . | . | . | . | . |
| 21-225_71F3_LC | . | . | . | . | . | . | S | N | . | . | . | . | . | . | . | . | . | . | . | . | . | . | . | . | . | . |
| 21-225_71G3_LC | . | . | . | . | . | . | R | . | . | . | . | . | . | . | . | D | . | . | . | . | . | . | . | . | L | . |
| 21-225_74A8_LC | . | . | . | . | . | . | . | . | . | . | S | H | . | . | . | . | . | . | . | . | . | . | . | . | . | . |
| 21-225_74C10_LC | . | . | . | . | . | . | . | . | . | . | . | A | . | . | . | . | . | . | . | . | . | . | . | . | . | . |
| 21-225_74C12_LC | . | . | . | . | . | . | . | . | . | . | . | A | . | . | . | . | . | . | . | . | . | . | . | . | . | . |
| 21-225_77D12_LC | . | . | . | . | . | . | . | . | . | . | . | Y | . | . | . | . | . | . | . | . | . | . | . | . | . | . |
| 21-225_78E6_LC | . | . | . | . | . | . | . | . | . | . | S | H | . | . | . | . | . | . | . | . | . | . | . | . | . | . |
| 21-225_79G7_LC | . | . | . | . | . | . | . | . | . | . | S | H | . | . | . | . | . | . | . | . | . | . | . | . | . | . |
| 21-225_85C11_LC | . | . | . | . | . | . | . | . | . | . | S | H | . | F | . | . | . | . | . | . | N | . | . | . | . | . |
| 21-225_87E10_LC | . | . | . | . | . | . | R | . | . | . | . | M | . | . | . | . | . | . | . | . | . | . | . | . | . | . |
| 21-225_8B11_LC | . | . | . | N | . | . | . | N | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | W | - | - | - | - | - | - | - | - | A | S | T | R | E | S | G | V | P | D | R | F |
| 21-225_146C9_LC | . | V | . | . | . | . | . | . | . | . | . | . | . | . | . | . | . | . | K | . | . | . | . | . | . | . |
| 21-225_146D4_LC | . | . | V | . | . | . | . | . | . | . | . | . | . | . | . | . | . | . | . | . | . | . | . | . | . | . |
| 21-225_146F9_LC | Y | . | . | . | . | . | . | . | . | . | . | . | . | . | . | . | . | . | . | . | . | . | . | . | . | . |
| 21-225_147D5_LC | . | . | . | . | . | . | . | . | . | . | . | . | . | . | . | . | . | . | . | . | . | . | . | . | . | . |
| 21-225_150D2_LC | . | . | F | . | . | . | . | . | . | . | . | . | . | . | . | . | . | . | K | . | . | . | . | . | . | . |
| 21-225_152D10_LC | . | . | . | . | . | . | . | . | . | . | . | . | . | . | . | . | . | . | . | . | . | . | . | . | . | . |
| 21-225_152E12_LC | . | . | . | . | . | . | . | . | . | . | . | . | . | . | . | . | . | . | . | . | . | . | . | . | . | . |
| 21-225_152H3_LC | . | . | . | . | . | . | . | . | . | . | . | . | . | . | . | . | . | . | K | . | . | . | . | . | . | . |
| 21-225_153B9_LC | . | . | . | . | . | . | . | . | . | . | . | . | . | . | . | . | . | . | . | F | . | . | . | . | . | . |
| 21-225_153F11_LC | N | . | . | . | . | . | . | . | . | . | . | . | . | . | . | . | . | . | K | . | . | . | . | . | . | . |
| 21-225_154H6_LC | . | . | . | . | . | . | . | . | . | . | . | . | . | . | T | . | . | . | K | . | . | . | . | . | . | . |
| 21-225_155B6_LC | . | . | . | . | . | . | . | . | . | . | . | . | . | . | T | . | . | . | . | . | . | . | . | . | . | . |
| 21-225_155E5_LC | . | . | . | . | . | . | . | . | . | . | . | . | . | . | . | . | . | . | K | . | . | . | . | . | . | . |
| 21-225_156H1_LC | . | . | . | . | . | . | . | . | . | . | . | . | . | . | . | . | . | . | . | . | . | . | . | A | . | . |
| 21-225_160A4_LC | . | . | . | . | . | . | . | . | . | . | . | . | . | . | . | . | . | . | . | . | . | . | . | . | . | . |
| 21-225_160A7_LC | . | . | . | . | F | . | . | . | . | . | . | . | . | . | . | . | . | . | . | . | . | . | . | . | . | . |
| 21-225_172E11_LC | . | . | . | . | . | . | . | . | . | . | . | . | . | . | . | . | . | . | . | . | . | . | . | . | . | . |
| 21-225_224F11_LC | . | M | . | . | . | . | . | . | . | . | . | . | . | . | . | . | . | . | . | . | . | . | . | . | . | . |
| 21-225_225B9_LC | . | . | . | . | F | . | . | . | . | . | . | . | . | . | . | . | . | . | . | . | . | . | . | . | . | . |
| 21-225_225F12_LC | K | . | . | . | . | . | . | . | . | . | . | . | . | . | T | . | . | . | . | . | . | . | . | . | . | . |
| 21-225_225H12_LC | N | . | . | . | . | . | . | . | . | . | . | . | . | . | . | . | . | . | . | . | . | . | . | . | . | . |
| 21-225_226D6_LC | . | M | . | . | . | . | . | . | . | . | . | . | . | . | . | . | . | . | . | . | . | . | . | . | . | . |
| 21-225_227D11_LC | . | . | . | . | . | . | . | . | . | . | . | . | . | . | . | . | . | . | . | . | . | : | . | . | . | . |
| 21-225_227E6_LC | . | M | . | . | . | . | . | . | . | . | . | . | . | . | . | . | . | . | . | . | . | . | . | . | . | . |
| 21-225_227G9_LC | . | . | . | . | . | . | . | . | . | . | . | . | . | . | G | . | . | . | . | . | . | . | . | . | . | . |
| 21-225_22D2_LC | . | . | . | . | . | . | . | . | . | . | . | . | . | . | . | . | . | . | K | . | . | . | . | . | . | . |
| 21-225_25E12_LC | . | . | . | . | . | . | . | . | . | . | . | . | . | . | . | . | . | . | . | . | . | . | . | . | . | . |
| 21-225_32A1_LC | . | . | . | . | . | . | . | . | . | . | . | . | . | . | . | . | . | . | K | . | . | . | . | . | . | . |
| 21-225_32A5_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | W | - | - | - | - | - | - | - | - | A | S | T | R | E | S | G | V | P | D | R | F |
| 21-225_33D1_LC | . | . | F | . | . | . | . | . | . | . | . | . | . | . | . | . | . | . | . | . | . | . | L | . | . | . |
| 21-225_34H7_LC | . | . | . | . | . | . | . | . | . | . | . | . | . | . | . | . | . | . | K | . | . | . | . | . | . | . |
| 21-225_57F12_LC | . | . | I | . | . | . | . | . | . | . | . | . | . | . | . | . | . | . | . | . | . | . | . | . | . | . |
| 21-225_59E1_LC | . | . | . | . | . | . | . | . | . | . | . | . | . | S | . | . | . | . | . | . | . | . | . | . | . | . |
| 21-225_60F3_LC | . | . | . | . | . | . | . | . | . | . | . | . | . | . | . | . | . | . | . | . | . | . | . | . | . | . |
| 21-225_61E3_LC | . | . | . | . | . | . | . | . | . | . | . | . | . | . | . | . | I | . | . | . | . | . | . | . | . | . |
| 21-225_63F4_LC | . | . | . | . | . | . | . | . | . | . | . | . | . | . | . | . | . | . | . | . | . | I | . | . | . | . |
| 21-225_71F3_LC | . | . | . | . | . | . | . | . | . | . | . | . | . | . | . | . | . | . | . | . | . | I | . | . | . | . |
| 21-225_71G3_LC | . | . | . | F | . | . | . | . | . | . | . | . | . | . | . | . | . | . | F | . | . | . | . | . | . | . |
| 21-225_74A8_LC | . | . | . | . | . | . | . | . | . | . | . | . | . | . | . | . | . | . | . | . | . | . | . | . | . | . |
| 21-225_74C10_LC | N | . | . | . | . | . | . | . | . | . | . | . | . | . | . | . | . | . | . | . | . | . | . | . | . | . |
| 21-225_74C12_LC | N | . | . | . | . | . | . | . | . | . | . | . | . | . | . | . | . | . | . | . | . | . | . | . | . | . |
| 21-225_77D12_LC | . | . | . | . | . | . | . | . | . | . | . | . | . | . | . | . | . | . | G | . | . | . | . | . | . | . |
| 21-225_78E6_LC | . | . | . | . | . | . | . | . | . | . | . | . | . | . | . | I | . | . | . | . | . | . | . | . | . | . |
| 21-225_79G7_LC | . | . | . | . | F | . | . | . | . | . | . | . | . | . | . | I | . | . | . | . | . | . | . | . | . | . |
| 21-225_85C11_LC | . | . | . | . | F | . | . | . | . | . | . | . | . | . | F | I | . | . | . | . | . | . | . | E | G | . |
| 21-225_87E10_LC | . | . | . | . | . | . | . | . | . | . | . | . | . | . | . | . | . | . | K | . | . | . | . | . | . | . |
| 21-225_8B11_LC | . | . | . | . | . | . | . | . | . | . | . | . | . | . | . | . | . | . | K | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_146C9_LC | . | . | . | . | . | . | . | . | . | . | . | . | . | . | . | . | . | . | . | . | . | . | . | . | . | . |
| 21-225_146D4_LC | . | . | . | . | . | . | . | . | . | . | . | . | . | . | . | . | . | . | . | . | . | . | . | . | . | . |
| 21-225_146F9_LC | . | . | . | . | . | . | . | . | . | . | . | . | . | . | . | T | . | . | . | . | . | . | . | . | . | . |
| 21-225_147D5_LC | . | . | . | . | . | . | . | . | . | . | . | . | . | S | . | . | . | . | . | . | . | . | . | . | . | . |
| 21-225_150D2_LC | . | . | . | . | . | . | . | . | . | . | . | . | . | . | . | . | N | . | . | . | . | . | . | . | . | . |
| 21-225_152D10_LC | . | . | . | . | . | . | . | . | . | . | . | . | . | . | . | Y | . | . | . | . | . | . | . | . | . | . |
| 21-225_152E12_LC | . | . | . | . | . | . | . | . | . | . | . | . | . | . | . | . | . | . | . | . | . | . | . | . | . | . |
| 21-225_152H3_LC | . | . | . | . | . | . | . | . | . | . | . | . | . | . | . | . | . | . | . | . | . | . | . | . | . | . |
| 21-225_153B9_LC | . | . | . | . | G | . | . | . | . | . | . | . | . | . | . | Y | . | V | . | . | . | . | . | . | . | . |
| 21-225_153F11_LC | . | . | . | . | . | . | . | . | . | . | . | . | . | . | . | . | . | . | . | . | . | . | . | . | . | . |
| 21-225_154H6_LC | . | . | . | . | . | . | . | . | N | . | . | . | . | S | . | . | . | . | . | . | . | . | . | . | . | . |
| 21-225_155B6_LC | . | . | . | . | . | . | . | . | . | . | . | . | . | . | . | . | . | . | . | . | . | . | . | . | . | . |
| 21-225_155E5_LC | . | . | . | . | . | . | . | . | . | . | . | . | . | . | . | . | . | . | . | . | . | . | . | . | . | . |
| 21-225_156H1_LC | . | . | . | . | . | . | . | . | . | . | . | . | . | . | . | . | . | . | . | . | . | . | . | . | . | . |
| 21-225_160A4_LC | . | . | . | . | . | . | . | . | . | . | . | . | . | . | . | . | . | . | . | . | . | . | . | . | . | . |
| 21-225_160A7_LC | . | . | Y | . | . | . | . | . | . | . | . | . | . | . | . | . | . | . | . | . | . | . | . | . | . | . |
| 21-225_172E11_LC | . | . | . | . | . | . | . | . | . | . | S | . | . | . | . | . | G | . | . | . | . | . | . | . | . | . |
| 21-225_224F11_LC | . | . | . | . | . | . | . | . | . | . | . | . | . | . | . | . | . | . | . | . | . | . | . | . | . | . |
| 21-225_225B9_LC | . | . | . | . | . | . | . | . | . | . | . | . | . | . | . | . | . | . | . | . | . | . | . | . | . | . |
| 21-225_225F12_LC | . | . | . | . | . | . | . | . | . | . | . | . | . | . | . | . | N | . | . | . | . | . | . | . | . | . |
| 21-225_225H12_LC | . | . | . | . | . | . | . | . | . | . | . | . | . | . | . | . | . | . | . | Y | . | . | . | . | . | . |
| 21-225_226D6_LC | . | . | . | . | . | . | . | . | . | . | . | . | . | . | . | . | . | . | . | . | . | . | . | . | . | . |
| 21-225_227D11_LC | . | . | . | . | . | . | . | . | . | . | . | . | . | . | . | . | . | . | . | . | . | . | . | . | . | . |
| 21-225_227E6_LC | . | . | . | . | . | . | . | . | . | . | . | . | . | . | . | . | . | . | . | . | . | . | . | . | . | . |
| 21-225_227G9_LC | . | . | . | . | . | . | . | . | . | . | . | . | . | . | . | . | . | . | . | . | . | . | . | . | . | . |
| 21-225_22D2_LC | . | . | . | . | . | . | . | . | . | . | . | . | . | . | . | . | . | . | . | . | . | . | . | . | . | . |
| 21-225_25E12_LC | . | . | . | . | . | . | . | . | Y | . | . | . | . | . | . | . | . | . | . | . | . | . | . | . | . | . |
| 21-225_32A1_LC | . | . | . | . | . | . | . | . | . | . | . | . | . | . | . | . | . | . | . | T | . | . | . | . | L | . |
| 21-225_32A5_LC | . | . | . | . | . | . | . | . | . | . | . | A | . | . | . | . | . | . | . | . | . | . | . | . | L | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_33D1_LC | . | . | . | O | . | . | . | . | . | . | . | . | . | . | . | I | . | . | . | . | . | . | . | . | L | . |
| 21-225_34H7_LC | . | . | . | . | . | . | . | . | . | . | . | . | . | . | . | . | . | . | . | T | . | . | . | . | L | . |
| 21-225_57F12_LC | . | . | . | . | . | . | . | . | . | . | . | . | . | . | . | . | . | . | . | . | . | . | . | . | I | . |
| 21-225_59E1_LC | . | . | . | . | . | . | . | . | . | . | . | . | . | . | . | . | . | . | . | . | . | . | . | . | . | . |
| 21-225_60F3_LC | . | . | . | . | . | . | . | . | . | . | . | . | . | . | . | . | . | . | . | . | . | . | . | . | I | . |
| 21-225_61E3_LC | . | . | . | . | . | . | . | . | . | . | . | . | . | . | . | . | . | . | . | . | . | . | . | . | . | . |
| 21-225_63F4_LC | . | . | . | . | . | . | . | . | G | . | . | . | . | . | . | . | . | . | . | . | . | . | . | . | . | . |
| 21-225_71F3_LC | . | . | . | . | . | . | . | . | . | . | . | . | . | . | . | N | . | . | . | . | . | . | . | . | . | . |
| 21-225_71G3_LC | . | . | . | F | . | . | . | . | . | . | . | . | . | . | . | . | . | . | . | . | . | . | . | . | D | . |
| 21-225_74A8_LC | . | . | . | . | . | . | . | . | . | . | . | . | . | . | . | . | . | . | . | . | . | . | . | . | . | . |
| 21-225_74C10_LC | . | . | . | . | . | . | . | . | . | . | . | . | . | . | . | . | . | . | . | . | . | . | . | . | . | . |
| 21-225_74C12_LC | . | . | . | . | . | . | . | . | . | . | . | . | . | . | . | . | . | . | . | . | . | . | . | . | . | . |
| 21-225_77D12_LC | . | . | . | . | . | . | . | . | . | . | . | . | . | . | . | . | . | . | . | . | . | . | . | . | . | . |
| 21-225_78E6_LC | . | . | . | . | . | . | . | . | . | . | . | . | . | S | . | . | . | . | . | . | . | . | . | . | . | . |
| 21-225_79G7_LC | . | . | . | . | . | . | . | . | . | . | . | . | . | S | . | . | . | M | . | . | . | . | . | . | . | . |
| 21-225_85C11_LC | . | . | . | . | . | . | . | . | A | . | . | . | . | S | . | . | O | . | . | . | . | . | . | . | . | . |
| 21-225_87E10_LC | . | . | T | . | . | . | . | . | . | . | . | . | . | . | . | . | . | . | . | . | . | . | . | . | . | . |
| 21-225_8B11_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_146C9_LC | . | . | . | . | . | . | . | | | | | | | | | | | | | | | | | | | |
| 21-225_146D4_LC | . | . | . | . | . | . | . | | | | | | | | | | | | | | | | | | | |
| 21-225_146F9_LC | . | . | . | . | . | . | Y | | | | | | | | | | | | | | | | | | | |
| 21-225_147D5_LC | . | . | H | . | . | . | . | | | | | | | | | | | | | | | | | | | |
| 21-225_150D2_LC | F | . | . | . | . | F | . | | | | | | | | | | | | | | | | | | | |
| 21-225_152D10_LC | . | . | . | . | . | . | - | | | | | | | | | | | | | | | | | | | |
| 21-225_152E12_LC | . | . | . | . | . | . | . | | | | | | | | | | | | | | | | | | | |
| 21-225_152H3_LC | . | . | . | . | . | . | . | | | | | | | | | | | | | | | | | | | |
| 21-225_153B9_LC | . | . | . | . | N | - | . | | | | | | | | | | | | | | | | | | | |
| 21-225_153F11_LC | . | . | . | . | . | . | . | | | | | | | | | | | | | | | | | | | |
| 21-225_154H6_LC | . | . | H | . | . | . | . | | | | | | | | | | | | | | | | | | | |
| 21-225_155B6_LC | . | . | . | . | . | . | . | | | | | | | | | | | | | | | | | | | |
| 21-225_155E5_LC | . | . | . | . | . | . | . | | | | | | | | | | | | | | | | | | | |
| 21-225_156H1_LC | . | . | H | . | . | . | . | | | | | | | | | | | | | | | | | | | |
| 21-225_160A4_LC | . | . | . | . | . | N | . | | | | | | | | | | | | | | | | | | | |
| 21-225_160A7_LC | . | . | . | . | . | . | . | | | | | | | | | | | | | | | | | | | |
| 21-225_172E11_LC | . | . | . | . | . | F | Y | | | | | | | | | | | | | | | | | | | |
| 21-225_224F11_LC | . | . | . | . | . | . | Y | | | | | | | | | | | | | | | | | | | |
| 21-225_225B9_LC | . | . | H | S | H | . | . | | | | | | | | | | | | | | | | | | | |
| 21-225_225F12_LC | . | . | . | . | . | . | S | | | | | | | | | | | | | | | | | | | |
| 21-225_225H12_LC | . | . | . | . | . | . | S | | | | | | | | | | | | | | | | | | | |
| 21-225_226D6_LC | . | . | . | . | . | . | . | | | | | | | | | | | | | | | | | | | |
| 21-225_227D11_LC | . | . | . | . | . | N | . | | | | | | | | | | | | | | | | | | | |
| 21-225_227E6_LC | . | . | . | . | . | . | S | | | | | | | | | | | | | | | | | | | |
| 21-225_227G9_LC | . | . | . | . | . | . | . | | | | | | | | | | | | | | | | | | | |
| 21-225_22D2_LC | F | . | . | . | . | . | . | | | | | | | | | | | | | | | | | | | |
| 21-225_25E12_LC | . | . | . | . | . | . | . | | | | | | | | | | | | | | | | | | | |
| 21-225_32A1_LC | . | . | . | . | . | . | T | | | | | | | | | | | | | | | | | | | |
| 21-225_32A5_LC | H | . | . | . | . | . | . | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_33D1_LC | . | . | . | . | . | . | . | . | . | . | . | . | . | . | . | . | . | . | . | . | . | . | . | . | . | . |
| 21-225_34H7_LC | . | . | . | . | . | . | T | . | . | . | . | . | . | . | . | . | . | . | . | . | . | . | . | . | . | . |
| 21-225_57F12_LC | . | . | . | . | . | . | . | . | . | . | . | . | . | . | . | . | . | . | . | . | . | . | . | . | . | . |
| 21-225_59E1_LC | . | . | . | . | . | F | . | . | . | . | . | . | . | . | . | . | . | . | . | . | . | . | . | . | . | . |
| 21-225_60F3_LC | . | . | . | . | . | . | . | . | . | . | . | . | . | . | . | . | . | . | . | . | . | . | . | . | . | . |
| 21-225_61E3_LC | . | . | . | . | . | . | . | . | . | . | . | . | . | . | . | . | . | . | . | . | . | . | . | . | . | . |
| 21-225_63F4_LC | . | . | . | . | . | . | T | . | . | . | . | . | . | . | . | . | . | . | . | . | . | . | . | . | . | . |
| 21-225_71F3_LC | . | . | . | . | . | . | T | . | . | . | . | . | . | . | . | . | . | . | . | . | . | . | . | . | . | . |
| 21-225_71G3_LC | . | . | . | . | . | . | ! | . | . | . | . | . | . | . | . | . | . | . | . | . | . | . | . | . | . | . |
| 21-225_74A8_LC | . | . | . | . | . | . | . | . | . | . | . | . | . | . | . | . | . | . | . | . | . | . | . | . | . | . |
| 21-225_74C10_LC | . | . | . | . | . | . | . | . | . | . | . | . | . | . | . | . | . | . | . | . | . | . | . | . | . | . |
| 21-225_74C12_LC | . | . | . | . | . | . | . | . | . | . | . | . | . | . | . | . | . | . | . | . | . | . | . | . | . | . |
| 21-225_77D12_LC | . | . | . | . | . | . | T | . | . | . | . | . | . | . | . | . | . | . | . | . | . | . | . | . | . | . |
| 21-225_78E6_LC | . | . | . | . | . | . | . | . | . | . | . | . | . | . | . | . | . | . | . | . | . | . | . | . | . | . |
| 21-225_79G7_LC | . | . | . | . | . | . | . | . | . | . | . | . | . | . | . | . | . | . | . | . | . | . | . | . | . | . |
| 21-225_85C11_LC | . | . | . | . | . | . | . | . | . | . | . | . | . | . | . | . | . | . | . | . | . | . | . | . | . | . |
| 21-225_87E10_LC | . | . | . | . | . | . | . | . | . | . | . | . | . | . | . | . | . | . | . | . | . | . | . | . | . | . |
| 21-225_8B11_LC | . | . | . | . | . | . | T | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | C | S | F | G | Q | G | T | K | L | E | I | K | R |
| 21-225_146C9_LC | . | . | . | . | . | . | . | . | . | . | . | . | . | . | . | . | . | . | . |
| 21-225_146D4_LC | . | . | . | . | . | . | . | . | . | . | . | . | . | . | . | . | . | . | . |
| 21-225_146F9_LC | . | . | . | . | . | . | . | . | . | . | . | . | . | . | . | . | . | T | . |
| 21-225_147D5_LC | . | . | . | . | . | . | . | . | . | . | . | . | . | . | . | . | . | . | . |
| 21-225_150D2_LC | . | . | . | . | . | . | Y | N | . | . | . | . | . | . | R | . | . | . | . |
| 21-225_152D10_LC | . | . | . | . | R | S | F | . | . | . | . | . | . | . | . | . | . | . | . |
| 21-225_152E12_LC | . | . | . | . | G | . | . | . | . | . | . | . | . | . | . | . | . | . | . |
| 21-225_152H3_LC | . | . | . | . | . | . | . | . | . | . | . | . | . | . | . | . | . | . | . |
| 21-225_153B9_LC | . | . | . | . | R | S | L | . | . | . | . | . | . | . | . | . | . | . | . |
| 21-225_153F11_LC | . | . | . | . | . | . | . | . | . | . | . | . | . | . | . | . | . | . | . |
| 21-225_154H6_LC | . | . | . | . | . | . | . | . | . | . | . | . | . | . | . | . | . | . | . |
| 21-225_155B6_LC | . | . | . | . | . | . | . | . | . | . | . | . | . | . | . | . | . | . | . |
| 21-225_155E5_LC | . | . | . | . | . | . | . | . | . | . | . | . | . | . | . | . | . | . | . |
| 21-225_156H1_LC | . | . | . | . | . | . | . | . | . | . | . | . | . | . | . | . | . | . | . |
| 21-225_160A4_LC | . | . | . | . | S | . | . | . | . | . | . | . | . | . | . | . | . | . | . |
| 21-225_160A7_LC | . | . | . | . | . | . | . | . | . | . | . | . | . | . | . | . | . | . | . |
| 21-225_172E11_LC | . | . | . | . | . | . | . | . | . | . | . | . | . | . | . | . | . | . | . |
| 21-225_224F11_LC | . | . | . | . | . | . | . | . | . | . | . | . | . | . | . | . | . | . | . |
| 21-225_225B9_LC | . | . | . | . | I | . | Q | R | . | . | . | I | . | . | . | . | . | Q | G |
| 21-225_225F12_LC | . | . | . | . | . | . | . | . | . | . | . | . | . | . | . | . | . | . | . |
| 21-225_225H12_LC | . | . | . | . | . | . | . | . | . | . | . | . | . | . | . | . | . | . | . |
| 21-225_226D6_LC | . | . | . | . | S | . | . | . | . | . | . | . | . | . | . | . | . | . | . |
| 21-225_227D11_LC | . | . | . | . | . | . | . | . | . | . | . | . | . | . | . | . | . | . | . |
| 21-225_227E6_LC | . | . | . | . | . | . | . | . | . | . | . | . | . | . | . | . | . | . | . |
| 21-225_227G9_LC | . | . | . | . | A | . | Y | . | . | . | . | . | . | . | . | . | . | . | . |
| 21-225_22D2_LC | . | . | . | . | . | . | . | . | . | . | . | . | . | . | . | . | . | . | . |
| 21-225_25E12_LC | . | . | . | . | . | . | . | . | . | . | . | . | . | N | . | . | . | . | . |
| 21-225_32A1_LC | . | . | . | . | S | . | . | . | . | . | . | . | . | . | . | . | . | . | . |
| 21-225_32A5_LC | . | . | . | . | S | . | . | . | . | . | . | . | G | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | C | S | F | G | Q | G | T | K | L | E | I | K | R |
| 21-225_33D1_LC | . | | . | . | S | . | | | . | . | . | . | . | . | . | . | . | . | . |
| 21-225_34H7_LC | . | | . | . | S | . | | . | . | . | . | . | . | . | . | . | . | . | . |
| 21-225_57F12_LC | . | | . | . | N | . | | . | . | . | . | . | . | . | . | . | . | . | . |
| 21-225_59E1_LC | . | | . | . | i | . | | . | . | . | . | . | . | . | . | . | . | . | . |
| 21-225_60F3_LC | . | | . | . | . | . | | . | . | . | . | . | . | . | . | . | . | N | . |
| 21-225_61E3_LC | . | | . | . | i | . | | . | . | . | . | . | . | . | . | . | . | . | . |
| 21-225_63F4_LC | . | | . | . | . | . | | . | . | . | . | . | . | . | . | . | . | N | . |
| 21-225_71F3_LC | . | | . | . | . | . | | . | . | . | . | . | . | . | . | . | . | . | . |
| 21-225_71G3_LC | . | | . | . | . | . | | . | . | . | . | . | . | . | . | . | . | . | . |
| 21-225_74A8_LC | . | | . | . | . | . | | . | . | . | . | . | . | . | . | . | . | . | . |
| 21-225_74C10_LC | . | | . | . | . | . | | . | . | . | . | . | . | . | . | . | . | . | . |
| 21-225_74C12_LC | . | | . | . | . | . | | . | . | . | . | . | . | . | . | . | . | . | . |
| 21-225_77D12_LC | . | | . | . | . | . | | . | . | . | . | . | . | . | . | . | . | . | . |
| 21-225_78E6_LC | . | | . | . | . | . | | . | . | . | . | . | . | . | . | . | . | . | . |
| 21-225_79G7_LC | . | | . | . | . | . | | . | . | . | . | . | . | . | . | . | . | . | . |
| 21-225_85C11_LC | . | | . | . | . | . | | . | . | . | . | . | . | . | . | . | . | . | . |
| 21-225_87E10_LC | . | | . | . | S | . | | . | . | . | . | . | . | . | . | X | . | . | . |
| 21-225_8B11_LC | . | | . | . | . | . | | . | . | . | . | . | . | . | . | . | . | . | . |

**FIGURE 57**

Table 108.    Consensus 28 - VK1|L5/JK1 (SEQ ID NO: 50325):

DIQMTQSPSSVSASVGDRVTITC<u>RAS--QGIS------NWLA</u>WYQQKPGKAPKLLIY<u>A-------ASSLQS</u>GVPSRFSGSGSG--

TDFTLTISSLQPEDFATYYC<u>QQANS--------------------FPWT</u>FGQGTKVEIKR wherein:

S at position 26 can be substituted with N

G at position 30 can be substituted with D, F, N or V

I at position 31 can be substituted with L or V

S at position 32 can be substituted with N, T, I, F or G

N at position 39 can be substituted with S, D, T or R

W at position 40 can be substituted with C

A at position 58 can be substituted with G, D, T or S

A at position 67 can be substituted with V, P or T

S at position 68 can be substituted with F

S at position 69 can be substituted with N

S at position 72 can be substituted with G or N

Q at position 107 can be substituted with L

A at position 109 can be substituted with T, S, G, V or Y

N at position 110 can be substituted with D, H or Y

F at position 135 can be substituted with L

W at position 137 can be substituted with R or P

Arg Ala Xaa Gln Xaa Xaa Xaa Xaa Xaa Leu Ala (SEQ ID NO: 50159)

Xaa Xaa Xaa Xaa Leu Gln Xaa (SEQ ID NO: 50160)

**FIGURE 57**

Xaa Gln Xaa Xaa Ser Xaa Pro Xaa Thr (SEQ ID NO: 50161)

Arg Ala Ser Gln Gly Ile Ser Asn Trp Leu Ala (SEQ ID NO: 50645)

Ala Ala Ser Ser Leu Gln Ser (SEQ ID NO: 50646)

Gln Gln Ala Asn Ser Phe Pro Trp Thr (SEQ ID NO: 50647)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | | |
| CONSENSUS | D | I | Q | M | T | Q | S | P | S | S | V | S | A | S | V | G | D | R | V | T | I | T | C | R | A | S |
| 21-225_149G7_LC | . | | | | | | | | | | | | | | | | | | | | | | | | | |
| 21-225_154E8_LC | | | | | | . | . | . | . | . | . | . | . | . | S | | . | . | . | . | . | . | . | | | |
| 21-225_171A8_LC | A | | | | | | | | | | | | | | | | | | | | | | | | | |
| 21-225_171A9_LC | . | | | | | | | | | | | | | | | | | | | | | | | | | |
| 21-225_171G4_LC | | | | | | . | . | . | . | . | . | . | . | . | . | . | . | . | . | . | . | . | . | . | | |
| 21-225_174G7_LC | A | | | | | . | | | | | | | | | | | | | | | | | | | | |
| 21-225_175C10_LC | . | | | | | | | | | | | | | | | | | | | | | | | | | |
| 21-225_178A5_LC | . | . | . | . | . | . | . | . | . | . | . | . | . | . | . | . | . | . | . | . | . | . | . | . | . | . |
| 21-225_178F7_LC | . | . | . | . | . | . | . | . | . | . | . | . | . | . | . | . | . | . | . | . | . | . | . | . | . | . |
| 21-225_179G1_LC | . | . | . | . | . | . | . | . | . | . | . | . | . | . | . | . | . | . | . | . | . | . | . | . | . | . |
| 21-225_17A4_LC | . | . | . | . | . | . | . | . | . | . | . | . | . | . | . | . | . | . | . | . | . | . | . | . | . | . |
| 21-225_188E8_LC | . | . | . | . | . | . | . | . | . | . | . | . | . | . | . | . | . | . | . | . | . | . | . | . | . | N |
| 21-225_190B9_LC | . | . | . | . | . | . | . | . | . | . | . | . | . | . | . | . | . | . | . | . | . | . | . | . | . | . |
| 21-225_190H3_LC | . | . | . | . | . | . | . | . | . | . | . | . | . | . | . | . | . | . | . | . | . | . | . | . | . | . |
| 21-225_193F2_LC | . | . | . | . | . | . | . | . | . | . | . | . | . | . | . | . | . | . | . | . | . | . | . | . | . | . |
| 21-225_194F7_LC | . | . | . | . | . | . | . | . | . | . | . | . | . | . | . | . | . | . | . | . | . | . | . | . | . | . |
| 21-225_195G12_LC | . | . | . | . | . | . | . | . | . | . | . | . | . | . | . | . | . | . | . | . | . | . | . | . | . | . |
| 21-225_196B9_LC | . | . | . | . | . | . | . | . | . | . | . | . | . | . | . | . | . | . | . | . | . | . | . | . | . | . |
| 21-225_197C9_LC | . | . | . | . | . | . | . | . | . | . | . | . | . | . | . | . | . | . | . | . | . | . | . | . | . | . |
| 21-225_20F7_LC | . | . | . | . | . | . | . | . | . | . | . | . | . | . | . | . | G | . | . | . | . | . | . | . | . | . |
| 21-225_224D6_LC | . | . | . | . | . | . | . | . | . | . | . | . | . | . | . | . | . | . | . | . | . | . | . | . | . | . |
| 21-225_227E4_LC | . | . | L | . | . | . | . | . | . | . | . | . | . | . | . | . | . | . | . | . | . | . | . | . | . | . |
| 21-225_25B3_LC | . | . | . | . | . | . | . | . | . | . | . | . | . | . | . | . | . | . | . | . | . | . | . | . | . | . |
| 21-225_25D7_LC | . | . | . | . | . | . | . | . | . | . | . | . | . | . | . | . | . | . | . | . | F | . | . | . | . | . |
| 21-225_26D12_LC | . | . | . | . | . | . | . | . | . | . | . | . | . | . | . | . | . | . | . | . | . | . | . | . | . | . |
| 21-225_27A11_LC | . | . | . | . | . | . | . | . | . | . | . | . | . | . | . | . | . | . | . | . | . | . | . | . | . | . |
| 21-225_27C5_LC | . | . | . | . | . | . | . | . | . | . | . | . | . | . | . | . | . | . | . | . | . | . | . | . | . | . |
| 21-225_43C2_LC | . | . | . | . | . | . | . | . | . | . | . | . | . | . | . | . | . | . | . | . | . | . | . | . | . | . |
| 21-225_43E8_LC | . | . | . | . | N | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | V | S | A | S | V | G | D | R | V | T | I | T | C | R | A | S |
| 21-225_43H4_LC | . | . | . | . | . | . | . | . | . | | . | . | . | . | . | . | . | . | | . | . | . | . | . | . | . |
| 21-225_44C12_LC | . | . | . | . | . | . | | | . | | . | . | . | . | . | . | . | . | | . | . | . | . | . | . | . |
| 21-225_44D10_LC | . | . | . | . | . | . | . | | | | . | . | . | . | . | . | . | . | | . | . | . | . | . | . | . |
| 21-225_45F8_LC | . | . | . | . | . | | | | . | | . | . | . | . | . | . | . | . | | . | . | . | . | . | . | . |
| 21-225_46A6_LC | . | . | . | . | N | . | | | . | | . | . | . | . | . | . | . | . | | N | . | . | . | . | . | . |
| 21-225_47G7_LC | . | . | . | . | . | . | | | . | | . | . | . | . | . | . | . | . | | . | . | . | . | . | . | . |
| 21-225_48D7_LC | . | . | . | . | . | . | | | . | | . | . | . | . | . | . | . | . | | . | . | . | . | . | . | . |
| 21-225_51G7_LC | . | . | . | . | . | . | | | . | | . | . | . | . | . | . | . | . | | . | . | . | . | . | . | . |
| 21-225_52H2_LC | . | . | . | . | . | . | | | . | | . | . | . | . | . | . | . | . | | . | . | . | . | . | . | . |
| 21-225_54G3_LC | . | . | . | . | . | . | | | . | | . | . | . | . | . | . | . | . | | . | . | . | . | . | . | . |
| 21-225_57F8_LC | . | . | . | . | . | . | | | . | | . | . | . | . | . | . | . | . | | . | . | . | . | . | . | . |
| 21-225_5A4_LC | . | . | . | . | . | . | | | . | | . | . | . | . | . | . | . | . | | . | . | . | . | . | . | . |
| 21-225_60A11_LC | . | . | . | . | . | . | | | . | | . | . | . | . | . | . | . | . | | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | N | W | L | A | W | Y | Q | Q | K | P | G | K | A | P |
| 21-225_149G7_LC | | | | F | | | | | | | | | S | | | | | | | | | | | | | |
| 21-225_154E8_LC | | | | F | | | | | | | | | S | | | | | | | | | | | | | |
| 21-225_171A8_LC | | | | | | | | | | | | | | | | | | | | | | | | | | |
| 21-225_171A9_LC | | | | D | | T | | | | | | | T | | | | | | | | | | | | | |
| 21-225_171G4_LC | | | | | V | N | | | | | | | D | | | | | | | | | | | R | | |
| 21-225_174G7_LC | | | | | | | | | | | | | | | | | | | | | | | | | | |
| 21-225_175C10_LC | | | | | | T | | | | | | | D | | | | | | | | | | | | | |
| 21-225_178A5_LC | | | | | | | | | | | | | | | | | | | | | | | | | | |
| 21-225_178F7_LC | | | | D | | T | | | | | | | D | | | | | | | | | | | | | |
| 21-225_179G1_LC | | | | D | | | | | | | | | D | | | | | | | | | S | | | | |
| 21-225_17A4_LC | | | | | | G | | | | | | | | | | | | | | | | | | | | |
| 21-225_188E8_LC | | | | D | | | | | | | | | S | | | | | | | | | | | | | |
| 21-225_190B9_LC | | | | | | N | | | | | | | | | | | | | | | | | | | | |
| 21-225_190H3_LC | | | | | L | N | | | | | | | | | | | | | | L | | | | | | |
| 21-225_193F2_LC | | | | | | N | | | | | | | | | | | | | | | | | | | | |
| 21-225_194F7_LC | | | | | | | | | | | | | D | | | | | | F | | | | | | | |
| 21-225_195G12_LC | | | | | | N | | | | | | | | | | | | | | | | | | | | |
| 21-225_196B9_LC | | | | | | | | | | | | | | | G | | | | | | | | | | | |
| 21-225_197C9_LC | | | | | | | | | | | | | | | | | | | F | | | | | | | |
| 21-225_20F7_LC | | | | | | | | | | | | | S | | | | | | | | | | | | | |
| 21-225_224D6_LC | | | | | | | | | | | | | S | | | | | | | | | | | | | |
| 21-225_227E4_LC | | | | | I | | | | | | | | | | | | | | | | | | | | | |
| 21-225_25B3_LC | | | | | | | | | | | | | S | | | | | | | | | | | | | |
| 21-225_25D7_LC | | | | D | | F | | | | | | | D | | | | | | | | | | | T | | |
| 21-225_26D12_LC | | | | | | | | | | | | | S | | | | | | | | | | | | | |
| 21-225_27A11_LC | | | | | | | | | | | | | S | | | | | | | | | | | | | |
| 21-225_27C5_LC | | | | | | | | | | | | | S | | | | | | | | | | | | | |
| 21-225_43C2_LC | | | | | | | | | | | | | D | | | | | | | | | | | | | |
| 21-225_43E8_LC | | | | | | | | | | | | | | | | | | | | | | | | R | | |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | K_FR2 | | | | | |
| CONSENSUS | - | - | Q | G | I | S | - | - | - | - | - | - | N | W | L | A | W | Y | Q | Q | K | P | G | K | A | P |
| 21-225_43H4_LC | . | . | . | . | . | I | | | | | | . | . | . | . | . | . | . | | | | | | | | |
| 21-225_44C12_LC | . | . | . | . | . | . | | | | | . | . | . | . | . | . | . | . | | | | | | | | |
| 21-225_44D10_LC | . | . | . | . | . | . | | | | | . | . | D | . | . | . | . | . | | | | | | | | |
| 21-225_45F8_LC | . | . | . | . | . | . | | | | | . | . | D | . | . | . | . | . | | | | | | | | |
| 21-225_46A6_LC | . | . | . | . | . | . | | | | | . | . | . | . | . | . | . | . | | | | | | . | V | . |
| 21-225_47G7_LC | . | . | . | . | . | . | | | | | . | . | . | . | . | . | . | . | | | | | | | | |
| 21-225_48D7_LC | . | . | . | N | . | T | | | | | . | . | S | . | . | . | . | . | | | | | | | | |
| 21-225_51G7_LC | . | . | . | Q | . | . | | | | | . | . | S | . | . | . | . | . | | | | | | | | |
| 21-225_52H2_LC | . | . | . | N | . | T | | | | | . | . | . | . | . | . | . | N | | | | | . | R | . | . |
| 21-225_54G3_LC | . | . | . | . | . | . | | | | | . | . | S | . | . | . | . | . | | | | | | | | |
| 21-225_57F8_LC | . | . | . | . | . | . | | | | | . | . | . | . | . | . | . | . | | | | | | | | |
| 21-225_5A4_LC | . | . | . | . | . | . | | | | | . | . | T | . | . | . | . | . | | | | | | | | |
| 21-225_60A11_LC | . | . | . | V | . | . | | | | | . | . | . | . | . | . | . | . | | L | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_149G7_LC | . | F | . | . | . | . | . | . | . | . | . | . | . | . | . | . | . | . | . | . | . | . | . | . | . | . |
| 21-225_154E8_LC | . | . | . | . | . | T | . | . | . | . | . | . | . | . | . | . | . | . | . | . | . | . | . | . | . | . |
| 21-225_171A8_LC | N | . | N | . | H | . | . | . | . | . | . | . | . | . | F | . | . | . | . | . | Q | . | . | . | . | . |
| 21-225_171A9_LC | . | . | . | . | . | D | . | . | . | . | . | . | . | . | . | . | . | . | . | . | . | A | . | . | . | . |
| 21-225_171G4_LC | . | . | . | . | . | D | . | . | . | . | . | . | . | . | . | . | . | . | . | . | . | . | . | . | . | . |
| 21-225_174G7_LC | N | . | . | . | H | . | . | . | . | . | . | . | . | . | F | . | . | . | . | . | Q | . | . | . | . | . |
| 21-225_175C10_LC | . | . | . | . | . | . | . | . | . | . | . | . | . | . | . | . | . | . | . | . | . | . | . | . | . | . |
| 21-225_178A5_LC | . | . | . | . | . | . | . | . | . | . | . | . | . | . | . | . | . | . | . | . | . | . | . | . | . | . |
| 21-225_178F7_LC | . | . | . | . | S | . | . | . | . | . | . | . | . | . | . | . | . | . | . | . | . | . | . | . | . | . |
| 21-225_179G1_LC | . | . | . | . | S | . | . | . | . | . | . | . | . | . | . | . | . | . | . | . | . | . | . | . | . | . |
| 21-225_17A4_LC | . | . | . | . | F | . | . | . | . | . | . | . | . | . | . | . | . | . | . | . | . | . | . | . | . | . |
| 21-225_188E8_LC | . | . | . | . | . | . | . | . | . | . | . | . | . | . | . | N | . | . | . | . | . | . | . | . | G | . |
| 21-225_190B9_LC | . | . | . | . | . | Q | . | . | . | . | . | V | . | . | . | . | . | . | . | . | . | . | . | . | . | . |
| 21-225_190H3_LC | . | . | . | . | . | . | . | . | . | . | . | V | . | . | . | . | . | . | . | . | . | . | . | . | . | . |
| 21-225_193F2_LC | . | . | . | . | . | Q | . | . | . | . | . | V | . | . | . | . | . | . | . | . | . | . | . | . | . | . |
| 21-225_194F7_LC | . | I | . | . | . | . | . | . | . | . | . | . | . | . | . | . | . | . | . | . | . | . | . | . | . | . |
| 21-225_195G12_LC | . | . | . | . | . | Q | . | . | . | . | . | V | . | . | . | . | . | . | . | . | . | . | . | . | . | . |
| 21-225_196B9_LC | . | F | . | . | C | . | . | . | . | . | . | . | . | . | . | . | . | . | . | . | . | A | . | . | . | . |
| 21-225_197C9_LC | Q | . | . | . | . | . | . | . | . | . | . | . | . | . | . | . | . | . | . | . | . | . | . | . | . | . |
| 21-225_20F7_LC | . | . | . | . | . | . | . | . | . | . | . | . | . | . | . | . | . | . | . | . | . | . | . | . | . | . |
| 21-225_224D6_LC | . | . | . | . | . | . | . | . | . | . | . | . | . | . | . | . | . | . | . | . | . | . | . | . | . | . |
| 21-225_227E4_LC | . | . | . | . | . | . | . | . | . | . | . | . | . | . | . | . | . | . | . | . | . | . | . | . | . | . |
| 21-225_25B3_LC | . | . | . | . | . | G | . | . | . | . | . | . | . | . | . | . | . | . | . | . | . | . | . | . | . | . |
| 21-225_25D7_LC | . | . | . | . | . | . | . | . | . | . | . | . | . | . | . | . | . | . | . | . | . | . | . | . | . | . |
| 21-225_26D12_LC | . | . | . | . | . | G | . | . | . | . | . | . | . | . | . | . | . | . | . | . | . | . | . | . | . | . |
| 21-225_27A11_LC | . | . | . | . | . | G | . | . | . | . | . | . | . | . | . | . | . | . | . | . | . | . | . | . | . | . |
| 21-225_27C5_LC | . | F | . | . | . | G | . | . | . | . | . | . | . | . | . | . | . | . | . | . | . | . | . | . | . | . |
| 21-225_43C2_LC | R | . | . | . | . | . | . | . | . | . | . | . | . | . | . | . | . | . | . | . | . | . | . | . | . | . |
| 21-225_43E8_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_43H4_LC | . | . | . | . | Y | A | | | | | | | | | . | . | . | . | . | . | . | . | . | . | . | . |
| 21-225_44C12_LC | . | . | . | . | S | . | | | | | | | | | . | P | . | . | . | . | . | . | . | . | . | . |
| 21-225_44D10_LC | R | . | . | . | . | . | | | | | | | | | . | . | . | . | . | . | . | . | . | . | K | . |
| 21-225_45F8_LC | R | . | . | . | . | G | | | | | | | | | . | . | . | . | . | . | . | . | . | . | . | . |
| 21-225_46A6_LC | . | . | . | . | . | . | | | | | | | | | . | . | . | . | . | . | . | . | . | . | . | . |
| 21-225_47G7_LC | . | . | . | . | S | . | | | | | | | | | . | . | N | . | . | . | . | . | . | . | . | . |
| 21-225_48D7_LC | . | . | . | . | . | S | | | | | | | | | . | . | . | . | . | N | . | . | . | . | . | . |
| 21-225_51G7_LC | . | V | . | . | . | . | | | | | | | | | P | . | N | . | . | A | . | . | . | . | . | . |
| 21-225_52H2_LC | . | . | . | . | . | T | | | | | | | | | T | . | . | . | . | . | . | . | . | . | . | . |
| 21-225_54G3_LC | . | . | . | . | . | Q | | | | | | | | | . | . | . | . | . | . | . | . | . | . | . | . |
| 21-225_57F8_LC | . | . | . | . | . | T | | | | | | | | | . | . | . | . | . | . | . | . | . | . | . | . |
| 21-225_5A4_LC | . | . | . | . | . | Q | | | | | | | | | . | . | . | . | . | . | . | . | . | . | . | . |
| 21-225_60A11_LC | . | . | . | . | . | T | | | | | | | | | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_149G7_LC | . | . | . | . | . | . | . | . | . | . | . | . | . | . | . | . | . | . | . | . | . | . | . | . | . | . |
| 21-225_154E8_LC | . | . | . | . | . | . | . | . | . | . | . | . | . | . | . | . | . | . | . | . | . | . | . | . | . | . |
| 21-225_171A8_LC | . | . | . | . | . | . | . | . | . | . | . | . | . | . | . | . | . | . | . | . | . | . | . | . | . | . |
| 21-225_171A9_LC | . | . | . | . | . | . | . | . | . | . | . | . | . | . | V | . | . | . | . | . | . | . | . | . | . | . |
| 21-225_171G4_LC | . | . | . | . | . | . | . | . | . | . | . | . | . | . | . | . | . | . | . | . | . | . | . | . | . | . |
| 21-225_174G7_LC | . | . | . | . | . | . | . | . | . | . | . | . | . | . | . | . | . | . | . | . | . | . | . | . | . | . |
| 21-225_175C10_LC | . | . | . | . | . | . | . | . | . | . | . | . | . | . | F | . | . | . | . | . | Q | . | . | . | . | . |
| 21-225_178A5_LC | N | . | . | . | . | . | . | . | . | . | . | . | . | . | . | . | . | . | . | . | . | . | . | . | . | . |
| 21-225_178F7_LC | . | . | . | . | . | . | . | . | . | . | . | . | . | A | . | . | . | . | . | . | . | . | . | . | . | . |
| 21-225_179G1_LC | G | . | . | . | . | . | . | . | . | . | . | . | . | . | . | . | . | . | . | . | . | . | . | . | . | . |
| 21-225_17A4_LC | . | . | . | . | . | . | . | . | . | . | . | . | . | . | . | . | . | . | . | . | . | . | . | V | . | . |
| 21-225_188E8_LC | . | . | . | . | . | . | . | . | . | . | . | . | . | . | . | . | . | . | . | . | . | . | . | . | . | . |
| 21-225_190B9_LC | . | . | . | . | . | . | . | . | . | . | . | . | . | . | . | . | . | . | . | . | . | . | . | . | . | . |
| 21-225_190H3_LC | . | . | . | . | . | . | . | . | S | E | . | . | . | . | . | . | . | . | . | . | . | . | . | . | . | . |
| 21-225_193F2_LC | . | . | . | . | . | . | . | . | . | . | . | . | . | . | . | . | . | . | . | . | . | . | . | . | . | . |
| 21-225_194F7_LC | . | . | . | . | . | . | . | . | . | . | . | . | . | . | . | . | . | . | . | . | . | . | . | . | . | . |
| 21-225_195G12_LC | . | . | . | . | . | . | . | . | . | . | . | . | . | . | . | . | . | . | . | . | . | . | . | . | . | . |
| 21-225_196B9_LC | . | . | . | . | . | . | . | . | . | . | . | . | . | . | . | . | . | . | . | . | . | . | . | . | S | . |
| 21-225_197C9_LC | . | . | . | . | . | . | . | . | . | S | . | . | . | . | . | . | . | . | . | . | . | . | . | . | . | . |
| 21-225_20F7_LC | . | . | . | . | . | . | . | . | . | . | . | . | . | . | . | . | . | . | . | . | . | . | . | . | . | . |
| 21-225_224D6_LC | . | . | . | . | . | . | . | . | . | . | . | . | . | . | . | . | . | . | . | . | . | . | . | . | . | . |
| 21-225_227E4_LC | . | . | . | . | . | . | . | . | . | . | . | . | . | . | . | . | . | . | . | . | . | . | . | . | I | . |
| 21-225_25B3_LC | . | A | . | . | . | . | . | . | . | . | . | . | . | . | . | . | . | . | . | . | . | . | . | . | . | . |
| 21-225_25D7_LC | . | . | . | . | . | . | . | . | N | . | . | . | . | . | V | . | G | . | . | . | . | . | . | . | . | . |
| 21-225_26D12_LC | . | A | . | . | . | . | . | . | . | . | . | . | . | . | . | . | . | . | . | . | . | . | . | . | . | . |
| 21-225_27A11_LC | . | A | . | . | . | . | . | . | . | . | . | . | . | . | . | . | . | . | . | . | . | . | . | . | . | . |
| 21-225_27C5_LC | . | A | . | . | . | . | . | . | . | E | . | . | . | . | . | . | . | . | . | . | . | . | . | . | . | . |
| 21-225_43C2_LC | . | . | . | . | . | . | . | . | . | . | . | . | . | . | . | N | . | . | . | . | . | . | . | . | . | . |
| 21-225_43E8_LC | . | . | . | . | . | . | . | . | . | . | . | . | . | . | . | N | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_43H4_LC | . | . | . | . | . | . | | | | | | | . | . | . | . | N | . | | | | | | | | |
| 21-225_44C12_LC | . | . | . | . | . | . | | | | | | . | . | N | . | . | . | . | | | | | | | | |
| 21-225_44D10_LC | . | . | . | . | . | . | | | | | . | . | . | . | . | . | N | . | | | | | | | | |
| 21-225_45F8_LC | . | . | . | . | . | . | | | | | . | . | . | . | . | . | N | . | | | | | | | | |
| 21-225_46A6_LC | . | . | . | . | . | . | | | | | . | . | . | . | . | . | N | . | | | | | | | | |
| 21-225_47G7_LC | . | . | . | . | . | . | | | | | . | . | . | . | . | . | . | . | | | A | . | | . | . | . |
| 21-225_48D7_LC | . | . | . | . | . | . | | | | | . | . | . | . | . | . | . | . | | | | | | V | . | . |
| 21-225_51G7_LC | . | . | . | . | . | . | | | | | . | . | . | . | . | . | . | . | | | | | | | | |
| 21-225_52H2_LC | . | . | . | . | . | . | | | | | . | . | . | . | . | . | . | . | | | | | | | | |
| 21-225_54G3_LC | . | . | . | . | . | N | | | | | . | . | . | . | . | . | . | . | | | | | | | | |
| 21-225_57F8_LC | . | . | N | N | . | . | | | | | . | . | . | . | . | . | . | . | | | | | | | | |
| 21-225_5A4_LC | . | . | . | . | . | . | | | | | . | . | . | . | . | . | . | . | | | | | | | | |
| 21-225_60A11_LC | . | . | N | N | . | . | | | | | . | . | . | . | . | . | . | . | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | A | N | S | - | - | - | - | - | - | - | - | - | - | - | - | - | - | - | - | - | - | - |
| 21-225_149G7_LC | | | | | | | | | | | | | | | | | | | | | | | | | | |
| 21-225_154E8_LC | Q | | | | | | | | | | | | | | | | | | | | | | | | | |
| 21-225_171A8_LC | | | | | T | | | | | | | | | | | | | | | | | | | | | |
| 21-225_171A9_LC | | | L | | T | | | | | | | | | | | | | | | | | | | | | |
| 21-225_171G4_LC | | | | | | | | | | | | | | | | | | | | | | | | | | |
| 21-225_174G7_LC | | | | | T | | | | | | | | | | | | | | | | | | | | | |
| 21-225_175C10_LC | | | | | T | | | | | | | | | | | | | | | | | | | | | |
| 21-225_178A5_LC | Q | | | | | | | | | | | | | | | | | | | | | | | | | |
| 21-225_178F7_LC | | | | | | | | | | | | | | | | | | | | | | | | | | |
| 21-225_179G1_LC | | | | | | | | | | | | | | | | | | | | | | | | | | |
| 21-225_17A4_LC | | | | | | | | | | | | | | | | | | | | | | | | | | |
| 21-225_188E8_LC | | | | | | | | | | | | | | | | | | | | | | | | | | |
| 21-225_190B9_LC | S | | | | | | | | | | | | | | | | | | | | | | | | | |
| 21-225_190H3_LC | H | | | | | | | | | | | | | | | | | | | | | | | | | |
| 21-225_193F2_LC | S | | | | | | | | | | | | | | | | | | | | | | | | | |
| 21-225_194F7_LC | | | | | | | | | | | | | | | | | | | | | | | | | | |
| 21-225_195G12_LC | S | | | | | | | | | | | | | | | | | | | | | | | | | |
| 21-225_196B9_LC | | | | | G | G | | | | | | | | | | | | | | | | | | | | |
| 21-225_197C9_LC | | | | | | | | | | | | | | | | | | | | | | | | | | |
| 21-225_20F7_LC | | | | | | | | | | | | | | | | | | | | | | | | | | |
| 21-225_224D6_LC | | | | | | | | | | | | | | | | | | | | | | | | | | |
| 21-225_227E4_LC | | | | | | | | | | | | | | | | | | | | | | | | | | |
| 21-225_25B3_LC | Q | | | | S | G | | | | | | | | | | | | | | | | | | | | |
| 21-225_25D7_LC | | | | | | Y | | | | | | | | | | | | | | | | | | | | |
| 21-225_26D12_LC | Q | | | | S | G | | | | | | | | | | | | | | | | | | | | |
| 21-225_27A11_LC | S | | | | S | G | | | | | | | | | | | | | | | | | | | | |
| 21-225_27C5_LC | Q | | | | Y | G | | | | | | | | | | | | | | | | | | | | |
| 21-225_43C2_LC | | | | | T | | | | | | | | | | | | | | | | | | | | | |
| 21-225_43E8_LC | | | | | T | | | | | | | | | | | | | | | | | | | | | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | A | N | S | - | - | - | - | - | - | - | - | - | - | - | - | - | - | - | - | - | - | - |
| 21-225_43H4_LC | . | . | . | . | T | . | | . | . | . | . | . | . | . | . | . | . | . | . | . | . | . | . | . | . | . |
| 21-225_44C12_LC | . | . | . | . | S | . | | . | . | . | . | . | . | . | . | . | . | . | . | . | . | . | . | . | . | . |
| 21-225_44D10_LC | . | . | . | . | T | . | | . | . | . | . | . | . | . | . | . | . | . | . | . | . | . | . | . | . | . |
| 21-225_45F8_LC | . | . | . | . | T | . | | . | . | . | . | . | . | . | . | . | . | . | . | . | . | . | . | . | . | . |
| 21-225_46A6_LC | . | . | . | . | T | . | | . | . | . | . | . | . | . | . | . | . | . | . | . | . | . | . | . | . | . |
| 21-225_47G7_LC | Q | . | . | . | V | . | | . | . | . | . | . | . | . | . | . | . | . | . | . | . | . | . | . | . | . |
| 21-225_48D7_LC | Q | . | . | . | | . | | . | . | . | . | . | . | . | . | . | . | . | . | . | . | . | . | . | . | . |
| 21-225_51G7_LC | . | . | . | . | . | >: | | . | . | . | . | . | . | . | . | . | . | . | . | . | . | . | . | . | . | . |
| 21-225_52H2_LC | Q | . | . | . | T | . | | . | . | . | . | . | . | . | . | . | . | . | . | . | . | . | . | . | . | . |
| 21-225_54G3_LC | . | . | . | . | . | | | . | . | . | . | . | . | . | . | . | . | . | . | . | . | . | . | . | . | . |
| 21-225_57F8_LC | . | . | . | . | . | | | . | . | . | . | . | . | . | . | . | . | . | . | . | . | . | . | . | . | . |
| 21-225_5A4_LC | . | . | . | . | . | | | . | . | . | . | . | . | . | . | . | . | . | . | . | . | . | . | . | . | . |
| 21-225_60A11_LC | . | . | . | . | . | | | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | F | P | W | T | F | G | Q | G | T | K | V | E | I | K | R |
| 21-225_149G7_LC | . | . | . | . | . | . | . | . | . | . | . | . | . | . | . | . | . | N | . |
| 21-225_154E8_LC | . | . | . | . | . | . | . | . | . | . | . | . | . | . | . | . | ₪ | N | . |
| 21-225_171A8_LC | . | . | . | . | . | . | . | . | . | . | . | . | . | . | . | . | . | . | . |
| 21-225_171A9_LC | . | . | . | . | . | . | . | . | . | . | . | . | . | . | . | . | . | . | . |
| 21-225_171G4_LC | . | . | . | . | . | . | . | . | . | . | . | . | . | . | . | . | . | . | . |
| 21-225_174G7_LC | . | . | . | . | . | . | . | . | . | . | . | . | . | . | . | . | . | . | . |
| 21-225_175C10_LC | . | . | . | . | . | . | . | . | . | . | . | . | . | . | . | . | . | . | . |
| 21-225_178A5_LC | . | . | . | . | ι | . | . | . | . | . | . | . | . | . | . | . | . | . | . |
| 21-225_178F7_LC | . | . | . | . | ι | . | . | . | . | . | . | . | . | . | . | . | . | . | . |
| 21-225_179G1_LC | . | . | . | . | ι | . | . | . | . | . | . | . | . | . | . | . | . | . | . |
| 21-225_17A4_LC | . | . | . | . | . | . | ₨ | . | . | . | . | . | . | . | . | . | . | . | . |
| 21-225_188E8_LC | . | . | . | . | . | . | . | . | . | . | . | . | . | . | . | . | . | . | . |
| 21-225_190B9_LC | . | . | . | . | . | . | . | . | . | . | . | . | . | . | . | . | . | ₨ | . |
| 21-225_190H3_LC | . | . | . | . | ι | . | . | . | . | . | . | . | . | . | . | . | . | . | . |
| 21-225_193F2_LC | . | . | . | . | . | . | . | . | . | . | . | . | . | . | . | . | . | ₨ | . |
| 21-225_194F7_LC | . | . | . | . | . | . | . | . | . | . | . | . | . | . | . | . | . | . | . |
| 21-225_195G12_LC | . | . | . | . | . | . | . | . | . | ₨ | . | . | . | . | . | . | N | Q | T |
| 21-225_196B9_LC | . | . | . | . | . | . | ₨ | . | . | . | . | . | . | . | . | . | ₨ | ₨ | . |
| 21-225_197C9_LC | . | . | . | . | . | . | . | . | . | . | . | . | . | . | . | . | . | . | . |
| 21-225_20F7_LC | . | . | . | . | . | . | ₨ | . | . | . | . | . | . | . | . | . | . | . | . |
| 21-225_224D6_LC | . | . | . | . | . | . | . | . | . | ₨ | . | . | . | . | . | . | . | . | . |
| 21-225_227E4_LC | . | . | . | . | . | . | . | . | . | . | . | . | . | . | . | . | . | . | . |
| 21-225_25B3_LC | . | . | . | . | . | . | ₨ | . | . | . | . | . | . | . | . | . | . | . | . |
| 21-225_25D7_LC | . | . | . | . | . | . | . | . | . | . | . | . | . | . | . | . | . | . | . |
| 21-225_26D12_LC | . | . | . | . | . | . | ₨ | . | . | . | . | . | . | . | . | . | . | . | . |
| 21-225_27A11_LC | . | . | . | . | . | . | ₨ | . | . | . | . | . | . | . | . | . | . | . | . |
| 21-225_27C5_LC | . | . | . | . | . | . | ₨ | . | . | . | . | . | . | . | . | . | . | . | . |
| 21-225_43C2_LC | . | . | . | . | . | . | . | . | . | . | . | . | . | . | . | . | . | . | . |
| 21-225_43E8_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | F | P | W | T | F | G | Q | G | T | K | V | E | I | K | R |
| 21-225_43H4_LC | | | | | | | | | | | | | | | | | | | |
| 21-225_44C12_LC | | | | | | | | | | | | | | | | | | | |
| 21-225_44D10_LC | | | | | | | | | | | | | | | | | | | |
| 21-225_45F8_LC | | | | | | | | | | | | | | | | | | | |
| 21-225_46A6_LC | | | | | | | | | | | | | | | | | | | |
| 21-225_47G7_LC | | | | | | | | | | | | | | | | | | | |
| 21-225_48D7_LC | | | | | | | | | | | | | | | | | | | |
| 21-225_51G7_LC | | | | | | | | | | | | | | | | | | | |
| 21-225_52H2_LC | | | | | | | | | | | R | | | | | | V | | |
| 21-225_54G3_LC | | | | | | | | | | | | | | | | | | | |
| 21-225_57F8_LC | | | | | | | | | | | | | | | | | | | |
| 21-225_5A4_LC | | | | | | | | | | | | | | | | | | | |
| 21-225_60A11_LC | | | | | | | | | | | | | | | | | | | |

**FIGURE 57**

Table 109.    Consensus 29 - VK4|B3/JK3 (SEQ ID NO: 50326):

DIVMTQSPDSLAVSLGERATINCKSS--QSVLIISSNNNNYLAWYQQKPGQPPKLLIYW--------ASTRESGVPDRFSGSGSG--

TDFTLTISSLQAEDVAVYYCQQYYS---------------------TPVTFGPGTKVDIKR wherein:

S at position 26 can be substituted with N

S at position 30 can be substituted with R or N

V at position 31 can be substituted with I or L

L at position 32 can be substituted with F

H at position 33 can be substituted with F, Y, K or S

S at position 34 can be substituted with N or H

N at position 36 can be substituted with II

N at position 37 can be substituted with S

N at position 38 can be substituted with K, Y or H

N at position 39 can be substituted with R or S

A at position 42 can be substituted with T or V

T at position 69 can be substituted with A, I or S

R at position 70 can be substituted with L

E at position 71 can be substituted with K or D

Q at position 107 can be substituted with H

Y at position 109 can be substituted with S

Y at position 110 can be substituted with C, F, S or H

S at position 111 can be substituted with N, Q, D or T

**FIGURE 57**

T at position 135 can be substituted with L, I, A, F or S

V at position 137 can be substituted with F or P

Lys Ser Xaa Gln Xaa Xaa Xaa Xaa Xaa Ser Xaa Xaa Xaa Xaa Tyr Leu Xaa (SEQ ID NO: 50162)

Trp Ala Ser Xaa Xaa Xaa Ser (SEQ ID NO: 50163)

Xaa Gln Xaa Xaa Xaa Xaa Pro Xaa Thr (SEQ ID NO: 50164)

Lys Ser Ser Gln Ser Val Leu His Ser Ser Asn Asn Asn Asn Tyr Leu Ala (SEQ ID NO: 50648)

Trp Ala Ser Thr Arg Glu Ser (SEQ ID NO: 50649)

Gln Gln Tyr Tyr Ser Thr Pro Val Thr (SEQ ID NO: 50650)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | S | P | D | S | L | A | V | S | L | G | E | R | A | T | I | N | C | K | S | S |
| 21-225_11E2_LC | . | . | . | . | . | . | . | . | . | . | . | . | . | . | . | . | . | . | . | . | . | . | . | . | . | N |
| 21-225_12F11_LC | . | . | . | . | . | . | . | . | . | . | . | . | . | . | . | . | . | . | . | . | . | . | . | . | . | . |
| 21-225_147G6_LC | . | . | . | . | . | . | . | . | . | . | . | . | . | . | . | . | . | . | . | . | . | . | . | . | . | . |
| 21-225_148G3_LC | . | . | . | . | . | . | . | . | . | . | . | . | . | . | . | . | . | . | . | . | . | H | . | . | . | . |
| 21-225_150B7_LC | . | . | . | . | . | . | . | . | . | . | . | . | . | . | . | . | . | . | . | . | . | . | . | . | . | . |
| 21-225_152E3_LC | . | . | . | . | . | . | . | . | . | . | . | . | . | . | . | . | . | . | . | . | . | . | . | . | . | . |
| 21-225_152F4_LC | . | . | . | . | . | . | . | . | . | . | . | . | . | . | . | . | . | . | . | . | . | . | . | . | . | . |
| 21-225_164B11_LC | . | . | . | . | . | . | . | . | . | . | . | . | . | . | . | . | . | . | . | . | . | . | . | . | . | . |
| 21-225_165H2_LC | . | . | . | . | . | . | . | . | . | . | . | T | . | . | . | P | . | . | . | . | . | . | . | . | . | . |
| 21-225_190E2_LC | . | . | . | . | . | . | . | . | . | . | . | . | . | . | . | . | . | . | . | . | . | . | . | . | . | . |
| 21-225_190H4_LC | A | N | . | . | . | . | . | . | . | . | . | . | . | . | . | . | . | . | T | . | . | . | . | . | . | . |
| 21-225_190H6_LC | . | . | . | . | . | . | . | . | . | . | . | . | . | . | . | . | . | . | . | . | . | . | . | . | . | . |
| 21-225_191B12_LC | . | . | . | . | . | . | . | . | . | . | . | . | . | . | . | . | . | . | . | . | . | . | . | . | . | . |
| 21-225_191G3_LC | . | . | . | . | . | . | . | . | . | . | . | . | . | . | . | . | . | . | . | . | G | . | . | . | . | . |
| 21-225_195G11_LC | . | . | . | . | . | . | . | . | . | . | . | . | . | . | . | . | . | . | . | . | . | . | . | . | . | . |
| 21-225_196B1_LC | . | . | . | . | . | . | . | . | . | . | . | . | . | P | . | . | . | . | . | . | . | . | . | . | . | . |
| 21-225_196C1_LC | . | . | . | . | . | . | . | . | . | . | . | . | . | . | . | . | . | . | . | . | . | . | . | . | . | . |
| 21-225_197C8_LC | . | . | . | . | P | . | . | . | . | . | . | . | . | . | . | . | . | . | . | . | . | . | . | . | . | . |
| 21-225_203F10_LC | . | . | . | . | . | . | . | . | . | . | . | . | . | . | . | . | . | . | . | . | . | . | . | . | . | . |
| 21-225_225F4_LC | . | . | . | . | . | . | . | . | . | . | . | . | . | . | . | . | . | . | . | . | . | . | . | . | . | . |
| 21-225_4A2_LC | . | . | . | . | . | . | . | . | . | . | . | . | . | . | . | . | . | . | . | . | . | . | . | . | . | . |
| 21-225_4A2.001_LC | . | . | . | . | . | . | . | . | . | . | . | . | . | . | . | . | . | . | . | . | . | . | . | . | . | . |
| 21-225_4A2.001.001_LC | . | . | . | . | . | . | . | . | . | . | . | . | . | . | . | . | . | . | . | . | . | . | . | . | . | . |
| 21-225_4A2.001.002_LC | . | . | . | . | . | . | . | . | . | . | . | . | . | . | . | . | . | . | . | . | . | . | . | . | . | . |
| 21-225_4A2.001.003_LC | . | . | . | . | . | . | . | . | . | . | . | . | . | . | . | . | . | . | . | . | . | . | . | . | . | . |
| 21-225_4A2.001.004_LC | . | . | . | . | . | . | . | . | . | . | . | . | . | . | . | . | . | . | . | . | . | . | . | . | . | . |
| 21-225_4A2.001.005_LC | . | . | . | . | . | . | . | . | . | . | . | . | . | . | . | . | . | . | . | . | . | . | . | . | . | . |
| 21-225_4A2.001.006_LC | . | . | . | . | . | . | . | . | . | . | . | . | . | . | . | . | . | . | . | . | . | . | . | . | . | . |
| 21-225_4A2.001.007_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | | |
| CONSENSUS | D | I | V | M | T | Q | S | P | D | S | L | A | V | S | L | G | E | R | A | T | I | N | C | K | S | S |
| 21-225_4A2.001.008_LC | | | | | | | | | | | | | | | | | | | | | | | | | | |
| 21-225_4A2.001.009_LC | | | | | | | | | | | | | | | | | | | | | | | | | | |
| 21-225_4A2.001.010_LC | | | | | | | | | | | | | | | | | | | | | | | | | | |
| 21-225_4A2.001.011_LC | | | | | | | | | | | | | | | | | | | | | | | | | | |
| 21-225_4A2.001.012_LC | | | | | | | | | | | | | | | | | | | | | | | | | | |
| 21-225_4A2.001.022_LC | | | | | | | | | | | | | | | | | | | | | | | | | | |
| 21-225_4A2.001.024_LC | | | | | | | | | | | | | | | | | | | | | | | | | | |
| 21-225_6G6_LC | | | | | | | | | | | | | | | | | | | | | | | | | | |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | K_FR2 | | | |
| CONSENSUS | - | - | Q | S | V | L | H | S | S | N | N | N | N | Y | L | A | W | Y | Q | Q | K | P | G | Q | P | P |
| 21-225_11E2_LC | . | . | . | R | . | . | S | . | . | . | . | K | . | . | . | . | . | . | . | . | R | . | . | . | . | . |
| 21-225_12F11_LC | . | . | . | . | . | . | . | . | . | . | . | Y | . | . | . | . | . | . | . | . | . | . | . | . | . | . |
| 21-225_147G6_LC | . | . | . | . | . | . | Y | . | . | . | S | . | . | . | . | . | . | . | . | . | . | . | . | . | . | . |
| 21-225_148G3_LC | . | . | . | R | . | . | . | . | . | . | . | Y | . | . | . | . | . | . | . | . | . | . | . | . | . | . |
| 21-225_150B7_LC | . | . | . | N | . | . | Y | . | . | R | . | . | . | . | . | . | . | . | . | . | . | . | . | . | . | . |
| 21-225_152E3_LC | . | . | . | . | . | . | . | . | . | . | . | Y | . | . | . | V | . | . | . | . | . | . | . | . | S | . |
| 21-225_152F4_LC | . | . | . | . | . | . | Y | . | . | . | . | Y | . | . | . | . | . | . | . | . | . | . | . | . | . | . |
| 21-225_164B11_LC | . | . | . | . | L | . | H | . | . | . | . | R | S | . | . | S | . | . | . | . | . | . | . | . | L | . |
| 21-225_165H2_LC | . | . | . | . | . | . | . | . | . | . | . | K | . | . | T | . | . | . | . | . | . | . | . | . | . | . |
| 21-225_190E2_LC | . | . | . | . | . | . | F | N | . | . | . | K | . | . | . | . | . | . | . | . | . | . | . | . | . | . |
| 21-225_190H4_LC | . | . | . | . | . | . | . | . | . | . | . | Y | . | . | . | . | . | . | R | . | . | . | . | . | . | . |
| 21-225_190H6_LC | . | . | . | . | . | . | F | N | . | . | . | K | . | . | . | . | . | . | . | . | . | . | . | . | . | . |
| 21-225_191B12_LC | . | . | . | . | . | . | F | N | . | . | . | K | . | . | . | . | . | . | . | . | . | . | . | . | . | . |
| 21-225_191G3_LC | . | . | . | . | . | . | . | . | . | . | S | Y | . | . | . | . | . | . | . | . | L | . | . | . | . | . |
| 21-225_195G11_LC | . | . | . | . | . | . | F | N | . | . | . | K | . | . | . | . | . | . | . | . | . | . | . | . | . | . |
| 21-225_196B1_LC | . | . | . | I | . | . | F | . | . | . | . | K | R | . | . | . | . | . | . | . | . | . | . | . | . | . |
| 21-225_196C1_LC | . | . | . | . | . | . | F | N | . | . | . | K | . | . | . | . | . | . | . | . | . | . | . | . | L | . |
| 21-225_197C8_LC | . | . | . | . | . | . | . | . | . | . | . | K | . | . | . | . | . | . | . | . | . | . | . | . | . | . |
| 21-225_203F10_LC | . | . | . | . | . | F | F | R | . | . | . | K | . | . | . | . | . | . | . | . | . | . | . | . | . | . |
| 21-225_225F4_LC | . | . | . | . | . | . | Y | . | . | . | . | K | . | . | . | . | . | . | . | . | . | . | . | . | . | . |
| 21-225_4A2_LC | . | . | . | I | . | . | . | . | . | . | . | . | . | . | . | . | F | . | . | . | . | . | . | . | . | . |
| 21-225_4A2.001_LC | . | . | . | I | . | . | . | . | . | . | . | . | . | . | . | . | F | . | . | . | . | . | . | . | . | . |
| 21-225_4A2.001.001_LC | . | . | . | I | . | . | . | . | . | . | . | . | . | . | . | . | F | . | . | . | . | . | . | . | . | . |
| 21-225_4A2.001.002_LC | . | . | . | I | . | . | . | . | . | . | . | . | . | . | . | . | F | . | . | . | . | . | . | . | . | . |
| 21-225_4A2.001.003_LC | . | . | . | I | . | . | . | . | . | . | . | . | . | . | . | . | F | . | . | . | . | . | . | . | . | . |
| 21-225_4A2.001.004_LC | . | . | . | I | . | . | . | . | . | . | . | . | . | . | . | . | F | . | . | . | . | . | . | . | . | . |
| 21-225_4A2.001.005_LC | . | . | . | I | . | . | . | . | . | . | . | . | . | . | . | . | F | . | . | . | . | . | . | . | . | . |
| 21-225_4A2.001.006_LC | . | . | . | I | . | . | . | . | . | . | . | . | . | . | . | . | F | . | . | . | . | . | . | . | . | . |
| 21-225_4A2.001.007_LC | . | . | . | I | . | . | . | . | . | . | . | . | . | . | . | . | F | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | S | V | L | H | S | S | N | N | N | N | Y | L | A | W | Y | Q | Q | K | P | G | Q | P | P |
| 21-225_4A2.001.008_LC | . | . | . | . | . | . | . | . | . | . | . | . | . | . | . | . | . | . | . | . | . | . | . | . | . | . |
| 21-225_4A2.001.009_LC | . | . | . | . | . | . | . | . | . | . | . | . | . | . | . | . | . | . | . | . | . | . | . | . | . | . |
| 21-225_4A2.001.010_LC | . | . | . | . | . | . | . | . | . | . | . | . | . | . | . | . | . | . | . | . | . | . | . | . | . | . |
| 21-225_4A2.001.011_LC | . | . | . | . | . | . | . | . | . | . | . | . | . | . | . | . | . | . | . | . | . | . | . | . | . | . |
| 21-225_4A2.001.012_LC | . | . | . | . | . | . | . | . | . | . | . | . | . | . | . | . | . | . | . | . | . | . | . | . | . | . |
| 21-225_4A2.001.022_LC | . | . | . | . | . | . | . | . | . | . | . | . | . | . | . | . | . | . | . | . | . | . | . | . | . | . |
| 21-225_4A2.001.024_LC | . | . | . | . | . | . | . | . | . | . | . | . | . | . | . | . | . | . | . | . | . | . | . | . | . | . |
| 21-225_6G6_LC | . | . | . | . | . | . | N | . | . | . | Y | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | W | - | - | - | - | - | - | - | - | A | S | T | R | E | S | G | V | P | D | R | F |
| 21-225_11E2_LC | . | . | . | . | . | . | . | . | . | . | . | . | . | . | . | . | . | . | . | . | . | . | . | . | . | . |
| 21-225_12F11_LC | N | . | . | . | F | . | . | . | . | . | . | . | . | . | . | . | . | . | . | . | . | . | . | . | . | . |
| 21-225_147G6_LC | . | . | . | . | . | . | . | . | . | . | . | . | . | . | . | . | A | . | . | . | . | . | . | . | . | . |
| 21-225_148G3_LC | . | . | . | . | . | . | . | . | . | . | . | . | . | . | . | . | . | D | . | . | . | . | . | . | . | . |
| 21-225_150B7_LC | . | . | . | . | . | . | . | . | . | . | . | . | . | . | . | . | . | X | . | . | . | . | . | . | . | . |
| 21-225_152E3_LC | . | R | . | . | . | . | . | . | . | . | . | . | . | . | . | . | . | . | . | . | . | . | . | . | . | . |
| 21-225_152F4_LC | . | . | . | . | . | . | . | . | . | . | . | . | . | . | . | . | . | . | . | . | . | . | . | . | . | . |
| 21-225_164B11_LC | . | . | . | . | . | . | . | . | . | . | . | . | . | . | . | . | S | . | . | . | . | . | . | . | . | . |
| 21-225_165H2_LC | . | . | . | . | . | . | . | . | . | . | . | . | . | . | . | . | . | . | . | . | . | . | . | V | . | . |
| 21-225_190E2_LC | N | . | . | . | . | . | . | . | . | . | . | . | . | . | . | . | . | . | . | . | . | . | . | . | . | . |
| 21-225_190H4_LC | . | . | . | . | . | . | . | . | . | . | . | . | . | . | . | . | . | . | . | . | . | . | . | . | . | . |
| 21-225_190H6_LC | N | . | . | . | . | . | . | . | . | . | . | . | . | . | . | . | . | . | . | . | . | . | . | . | . | . |
| 21-225_191B12_LC | . | . | . | . | . | . | . | . | . | . | . | . | . | . | . | . | . | . | . | . | . | . | . | . | . | . |
| 21-225_191G3_LC | . | . | . | . | . | . | . | . | . | . | . | . | . | . | . | . | . | X | . | . | . | . | . | . | . | . |
| 21-225_195G11_LC | N | . | . | . | . | . | . | . | . | . | . | . | . | . | . | . | . | L | . | . | . | . | . | . | . | . |
| 21-225_196B1_LC | . | . | . | . | . | . | . | . | . | . | . | . | . | . | . | . | I | . | . | . | . | . | . | . | . | . |
| 21-225_196C1_LC | N | . | . | . | . | . | . | . | . | . | . | . | . | . | . | . | . | L | . | . | . | . | . | . | . | . |
| 21-225_197C8_LC | . | . | . | . | . | . | . | . | . | . | . | . | . | . | . | . | . | . | . | . | . | . | . | . | . | . |
| 21-225_203F10_LC | . | . | . | . | . | . | . | . | . | . | . | . | . | . | . | . | . | . | . | . | . | . | . | . | . | . |
| 21-225_225F4_LC | . | . | . | . | . | . | . | . | . | . | . | . | . | . | . | . | . | . | . | . | . | . | . | . | . | . |
| 21-225_4A2_LC | . | . | L | . | . | . | . | . | . | . | . | . | . | . | . | . | . | . | . | . | . | . | . | . | . | . |
| 21-225_4A2.001_LC | . | . | L | . | . | . | . | . | . | . | . | . | . | . | . | . | . | . | . | . | . | . | . | . | . | . |
| 21-225_4A2.001.001_LC | . | . | L | . | . | . | . | . | . | . | . | . | . | . | . | . | . | . | . | . | . | . | . | . | . | . |
| 21-225_4A2.001.002_LC | . | . | L | . | . | . | . | . | . | . | . | . | . | . | . | . | . | . | . | . | . | . | . | . | . | . |
| 21-225_4A2.001.003_LC | . | . | L | . | . | . | . | . | . | . | . | . | . | . | . | . | . | . | . | . | . | . | . | . | . | . |
| 21-225_4A2.001.004_LC | . | . | L | . | . | . | . | . | . | . | . | . | . | . | . | . | . | . | . | . | . | . | . | . | . | . |
| 21-225_4A2.001.005_LC | . | . | L | . | . | . | . | . | . | . | . | . | . | . | . | . | . | . | . | . | . | . | . | . | . | . |
| 21-225_4A2.001.006_LC | . | . | L | . | . | . | . | . | . | . | . | . | . | . | . | . | . | . | . | . | . | . | . | . | . | . |
| 21-225_4A2.001.007_LC | . | . | L | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | K_CDR2 | | | | | | | | | | | | | | | |
| CONSENSUS | K | L | L | I | Y | W | - | - | - | - | - | - | - | - | A | S | T | R | E | S | G | V | P | D | R | F |
| 21-225_4A2.001.008_LC | . | . | . | L | . | . | . | . | . | . | . | . | . | . | . | . | . | . | . | . | . | . | . | . | . | . |
| 21-225_4A2.001.009_LC | . | . | . | L | . | . | . | . | . | . | . | . | . | . | . | . | . | . | . | . | . | . | . | . | . | . |
| 21-225_4A2.001.010_LC | . | . | . | L | . | . | . | . | . | . | . | . | . | . | . | . | . | . | . | . | . | . | . | . | . | . |
| 21-225_4A2.001.011_LC | . | . | . | L | . | . | . | . | . | . | . | . | . | . | . | . | . | . | . | . | . | . | . | . | . | . |
| 21-225_4A2.001.012_LC | . | . | . | L | . | . | . | . | . | . | . | . | . | . | . | . | . | . | . | . | . | . | . | . | . | . |
| 21-225_4A2.001.022_LC | . | . | . | L | . | . | . | . | . | . | . | . | . | . | . | . | . | . | . | . | . | . | . | . | . | . |
| 21-225_4A2.001.024_LC | . | . | . | L | . | . | . | . | . | . | . | . | . | . | . | . | . | . | . | . | . | . | . | . | . | . |
| 21-225_6G6_LC | N | . | . | . | F | . | . | . | . | . | . | . | . | . | . | . | . | . | . | . | . | . | . | . | . | . |

## FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
|  |  |  |  |  |  |  |  | K_FR3 |  |  |  |  |  |  |  |  |  |  |  |  |  |  |  |  |  |  |
| CONSENSUS | S | G | S | G | S | G | - | - | T | D | F | T | L | T | I | S | S | L | Q | A | E | D | V | A | V | Y |
| 21-225_11E2_LC | . | . | . | . | . | . | . | . | . | . | . | . | . | . | . | . | . | . | . | . | . | . | . | . | . | . |
| 21-225_12F11_LC | . | . | . | . | P | . | . | . | . | . | . | . | . | N | . | . | . | . | . | . | . | . | . | . | . | . |
| 21-225_147G6_LC | . | . | . | . | . | . | . | . | . | . | . | . | . | . | . | . | . | . | . | . | . | . | . | . | . | . |
| 21-225_148G3_LC | . | . | . | . | . | . | . | A | . | . | . | . | . | . | . | . | . | . | . | . | . | . | . | . | L | . |
| 21-225_150B7_LC | . | . | . | . | . | . | . | . | . | . | . | . | . | . | . | T | . | . | . | . | . | . | . | . | . | . |
| 21-225_152E3_LC | . | . | . | . | . | . | . | . | . | . | S | . | . | . | . | . | . | . | . | . | . | . | . | . | . | . |
| 21-225_152F4_LC | . | . | . | . | . | . | . | . | . | . | . | . | . | . | V | . | . | . | . | . | . | . | . | . | . | . |
| 21-225_164B11_LC | . | . | . | . | . | . | . | . | . | . | . | . | . | . | . | . | . | . | . | . | . | . | . | . | L | . |
| 21-225_165H2_LC | . | . | . | . | . | . | . | . | . | . | . | . | V | P | . | . | . | M | . | D | D | . | . | . | . | . |
| 21-225_190E2_LC | . | . | . | . | . | . | . | . | . | . | . | . | . | . | . | . | . | M | . | . | . | . | . | . | . | . |
| 21-225_190H4_LC | . | . | . | . | . | . | . | . | . | . | . | . | . | . | . | . | . | . | . | . | . | . | . | . | . | . |
| 21-225_190H6_LC | . | . | . | G | . | . | . | . | . | . | . | . | . | . | . | . | . | . | . | . | . | . | . | . | . | . |
| 21-225_191B12_LC | . | . | . | . | . | . | . | . | . | . | . | . | . | . | . | . | N | . | . | . | . | . | . | . | I | . |
| 21-225_191G3_LC | . | . | . | . | . | . | . | . | . | . | . | . | . | . | . | . | . | . | . | . | . | . | . | . | F | . |
| 21-225_195G11_LC | . | . | . | G | . | . | . | . | . | . | . | . | . | . | . | . | . | M | . | . | . | . | . | . | . | . |
| 21-225_196B1_LC | . | . | . | . | . | . | . | . | . | . | . | . | . | . | . | . | . | . | . | . | . | . | . | . | . | . |
| 21-225_196C1_LC | . | . | . | G | . | . | . | . | . | . | . | . | . | . | . | . | . | M | . | . | . | . | . | . | . | . |
| 21-225_197C8_LC | . | . | . | . | . | . | . | . | . | . | . | . | S | . | . | . | . | . | . | . | . | . | . | . | . | . |
| 21-225_203F10_LC | . | . | . | . | . | . | . | . | . | . | . | . | . | . | . | . | . | . | . | . | . | . | . | T | . | . |
| 21-225_225F4_LC | T | . | . | . | . | . | . | . | . | . | . | . | . | . | . | . | C | V | . | P | . | . | . | . | . | . |
| 21-225_4A2_LC | . | . | . | . | . | . | . | . | . | . | . | . | . | . | . | . | . | . | . | P | . | . | . | . | . | . |
| 21-225_4A2.001_LC | . | . | . | . | . | . | . | . | . | . | . | . | . | . | . | . | . | . | . | P | . | . | . | . | . | . |
| 21-225_4A2.001.001_LC | . | . | . | . | . | . | . | . | . | . | . | . | . | . | . | . | . | . | . | P | . | . | . | . | . | . |
| 21-225_4A2.001.002_LC | . | . | . | . | . | . | . | . | . | . | . | . | . | . | . | . | . | . | . | P | . | . | . | . | . | . |
| 21-225_4A2.001.003_LC | . | . | . | . | . | . | . | . | . | . | . | . | . | . | . | . | . | . | . | P | . | . | . | . | . | . |
| 21-225_4A2.001.004_LC | . | . | . | . | . | . | . | . | . | . | . | . | . | . | . | . | . | . | . | P | . | . | . | . | . | . |
| 21-225_4A2.001.005_LC | . | . | . | . | . | . | . | . | . | . | . | . | . | . | . | . | . | . | . | P | . | . | . | . | . | . |
| 21-225_4A2.001.006_LC | . | . | . | . | . | . | . | . | . | . | . | . | . | . | . | . | . | . | . | P | . | . | . | . | . | . |
| 21-225_4A2.001.007_LC | . | . | . | . | . | . | . | . | . | . | . | . | . | . | . | . | . | . | . | P | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | A | E | D | V | A | V | Y |
| 21-225_4A2.001.008_LC | . | . | . | . | . | . | . | . | . | . | . | . | . | . | . | . | . | . | . | S | . | . | . | . | . | . |
| 21-225_4A2.001.009_LC | . | . | . | . | . | . | . | . | . | . | . | . | . | . | . | . | . | . | . | S | . | . | . | . | . | . |
| 21-225_4A2.001.010_LC | . | . | . | . | . | . | . | . | . | . | . | . | . | . | . | . | . | . | . | S | . | . | . | . | . | . |
| 21-225_4A2.001.011_LC | . | . | . | . | . | . | . | . | . | . | . | . | . | . | . | . | . | . | . | S | . | . | . | . | . | . |
| 21-225_4A2.001.012_LC | . | . | . | . | . | . | . | . | . | . | . | . | . | . | . | . | . | . | . | S | . | . | . | . | . | . |
| 21-225_4A2.001.022_LC | . | . | . | . | . | . | . | . | . | . | . | . | . | . | . | . | . | . | . | S | . | . | . | . | . | . |
| 21-225_4A2.001.024_LC | . | . | . | . | . | . | . | . | . | . | . | . | . | . | . | . | . | . | . | S | . | . | . | . | . | . |
| 21-225_6G6_LC | . | . | . | . | S | . | . | . | . | . | . | . | N | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_11E2_LC | . | . | . | . | . | P | . | . | . | . | . | . | . | . | . | . | . | . | . | . | . | . | . | . | . | . |
| 21-225_12F11_LC | . | . | . | . | . | O | . | . | . | . | . | . | . | . | . | . | . | . | . | . | . | . | . | . | . | . |
| 21-225_147G6_LC | . | . | . | . | . | T | . | . | . | . | . | . | . | . | . | . | . | . | . | . | . | . | . | . | . | . |
| 21-225_148G3_LC | . | . | . | . | . | . | . | . | . | . | . | . | . | . | . | . | . | . | . | . | . | . | . | . | . | . |
| 21-225_150B7_LC | . | . | . | . | . | . | . | . | . | . | . | . | . | . | . | . | . | . | . | . | . | . | . | . | . | . |
| 21-225_152E3_LC | . | . | . | . | . | . | . | . | . | . | . | . | . | . | . | . | . | . | . | . | . | . | . | . | . | . |
| 21-225_152F4_LC | . | . | . | . | . | P | N | . | . | . | . | . | . | . | . | . | . | . | . | . | . | . | . | . | . | . |
| 21-225_164B11_LC | . | . | . | . | . | . | . | . | . | . | . | . | . | . | . | . | . | . | . | . | . | . | . | . | . | . |
| 21-225_165H2_LC | . | P | . | . | S | . | . | . | . | . | . | . | . | . | . | . | . | . | . | . | . | . | . | . | . | . |
| 21-225_190E2_LC | . | . | . | . | . | C | . | . | . | . | . | . | . | . | . | . | . | . | . | . | . | . | . | . | . | . |
| 21-225_190H4_LC | . | . | . | . | . | . | . | . | . | . | . | . | . | . | . | . | . | . | . | . | . | . | . | . | . | . |
| 21-225_190H6_LC | . | . | . | . | . | C | . | . | . | . | . | . | . | . | . | . | . | . | . | . | . | . | . | . | . | . |
| 21-225_191B12_LC | . | . | . | . | . | S | . | . | . | . | . | . | . | . | . | . | . | . | . | . | . | . | . | . | . | . |
| 21-225_191G3_LC | . | . | . | . | . | . | . | . | . | . | . | . | . | . | . | . | . | . | . | . | . | . | . | . | . | . |
| 21-225_195G11_LC | . | . | . | . | . | C | . | . | . | . | . | . | . | . | . | . | . | . | . | . | . | . | . | . | . | . |
| 21-225_196B1_LC | . | . | . | . | . | S | . | . | . | . | . | . | . | . | . | . | . | . | . | . | . | . | . | . | . | . |
| 21-225_196C1_LC | . | . | . | . | . | C | . | . | . | . | . | . | . | . | . | . | . | . | . | . | . | . | . | . | . | . |
| 21-225_197C8_LC | . | . | . | . | S | . | T | . | . | . | . | . | . | . | . | . | . | . | . | . | . | . | . | . | . | . |
| 21-225_203F10_LC | . | . | . | . | . | P | . | . | . | . | . | . | . | . | . | . | . | . | . | . | . | . | . | . | . | . |
| 21-225_225F4_LC | . | P | . | . | . | P | N | . | . | . | . | . | . | . | . | . | . | . | . | . | . | . | . | . | . | . |
| 21-225_4A2_LC | . | . | . | . | . | . | N | . | . | . | . | . | . | . | . | . | . | . | . | . | . | . | . | . | . | . |
| 21-225_4A2.001_LC | . | . | . | . | . | . | N | . | . | . | . | . | . | . | . | . | . | . | . | . | . | . | . | . | . | . |
| 21-225_4A2.001.001_LC | . | . | . | . | . | . | . | . | . | . | . | . | . | . | . | . | . | . | . | . | . | . | . | . | . | . |
| 21-225_4A2.001.002_LC | . | . | . | . | . | . | N | . | . | . | . | . | . | . | . | . | . | . | . | . | . | . | . | . | . | . |
| 21-225_4A2.001.003_LC | . | . | . | . | . | . | Q | . | . | . | . | . | . | . | . | . | . | . | . | . | . | . | . | . | . | . |
| 21-225_4A2.001.004_LC | . | . | . | . | . | . | N | . | . | . | . | . | . | . | . | . | . | . | . | . | . | . | . | . | . | . |
| 21-225_4A2.001.005_LC | . | . | . | . | . | . | N | . | . | . | . | . | . | . | . | . | . | . | . | . | . | . | . | . | . | . |
| 21-225_4A2.001.006_LC | . | . | . | . | . | . | N | . | . | . | . | . | . | . | . | . | . | . | . | . | . | . | . | . | . | . |
| 21-225_4A2.001.007_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_4A2.001.008_LC | . | . | . | . | . | . | . | . | . | . | . | . | . | . | . | . | . | . | . | . | . | . | . | . | . | . |
| 21-225_4A2.001.009_LC | . | . | . | . | . | . | N | . | . | . | . | . | . | . | . | . | . | . | . | . | . | . | . | . | . | . |
| 21-225_4A2.001.010_LC | . | . | . | . | . | . | Q | . | . | . | . | . | . | . | . | . | . | . | . | . | . | . | . | . | . | . |
| 21-225_4A2.001.011_LC | . | . | . | . | . | . | Q | . | . | . | . | . | . | . | . | . | . | . | . | . | . | . | . | . | . | . |
| 21-225_4A2.001.012_LC | . | . | . | . | . | . | Q | . | . | . | . | . | . | . | . | . | . | . | . | . | . | . | . | . | . | . |
| 21-225_4A2.001.022_LC | . | . | . | . | . | . | . | . | . | . | . | . | . | . | . | . | . | . | . | . | . | . | . | . | . | . |
| 21-225_4A2.001.024_LC | . | . | . | . | . | . | . | . | . | . | . | . | . | . | . | . | . | . | . | . | . | . | . | . | . | . |
| 21-225_6G6_LC | . | . | . | . | . | . | D | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K FR4 | | | | | | |
| CONSENSUS | - | - | - | - | T | P | V | T | F | G | P | G | T | K | V | D | I | K | R |
| 21-225_11E2_LC | . | . | . | . | . | . | S | . | . | . | . | . | . | . | . | . | . | . | . |
| 21-225_12F11_LC | . | . | . | . | . | . | S | . | . | . | . | . | . | . | . | . | . | . | . |
| 21-225_147G6_LC | . | . | . | . | . | . | P | . | . | . | . | . | . | . | . | . | . | . | . |
| 21-225_148G3_LC | . | . | . | . | . | . | S | . | . | . | . | . | . | . | . | . | . | . | . |
| 21-225_150B7_LC | . | . | . | . | . | . | P | . | . | . | . | . | . | . | . | . | . | . | . |
| 21-225_152E3_LC | . | . | . | . | . | . | S | . | . | . | . | . | . | . | . | . | . | . | . |
| 21-225_152F4_LC | . | . | . | . | . | . | P | . | . | . | . | . | . | . | . | . | . | . | . |
| 21-225_164B11_LC | . | . | . | . | I | . | P | . | . | . | M | . | . | N | . | . | . | T | . |
| 21-225_165H2_LC | . | . | . | . | I | . | P | . | . | . | . | . | . | N | . | . | . | . | . |
| 21-225_190E2_LC | . | . | . | . | L | . | S | . | . | . | . | . | . | . | . | . | . | R | . |
| 21-225_190H4_LC | . | . | . | . | I | . | P | . | . | . | . | . | . | . | . | . | . | . | . |
| 21-225_190H6_LC | . | . | . | . | L | . | P | . | . | . | . | . | . | . | . | . | . | R | . |
| 21-225_191B12_LC | . | . | . | . | L | . | P | . | . | . | . | . | . | . | . | . | . | . | . |
| 21-225_191G3_LC | . | . | . | . | S | . | P | . | . | . | . | . | . | . | M | . | . | . | . |
| 21-225_195G11_LC | . | . | . | . | P | . | P | . | . | . | . | . | . | . | . | . | . | R | . |
| 21-225_196B1_LC | . | . | . | . | L | . | S | . | . | . | . | . | . | . | . | . | . | . | . |
| 21-225_196C1_LC | . | . | . | . | P | . | P | . | . | . | . | . | . | . | . | . | . | R | . |
| 21-225_197C8_LC | . | . | . | . | I | . | P | . | . | . | . | . | . | . | . | . | N | . | . |
| 21-225_203F10_LC | . | . | . | . | L | . | S | . | . | . | . | . | . | . | . | . | . | T | . |
| 21-225_225F4_LC | . | . | . | . | S | . | P | . | . | . | M | . | . | E | . | . | . | . | . |
| 21-225_4A2_LC | . | . | . | . | . | . | . | . | . | . | . | . | . | . | . | G | . | . | . |
| 21-225_4A2.001_LC | . | . | . | . | . | . | . | . | . | . | . | . | . | . | . | G | . | . | . |
| 21-225_4A2.001.001_LC | . | . | . | . | . | . | . | . | . | . | . | . | . | . | . | G | . | . | . |
| 21-225_4A2.001.002_LC | . | . | . | . | A | . | . | . | . | . | . | . | . | . | . | G | . | . | . |
| 21-225_4A2.001.003_LC | . | . | . | . | . | . | . | . | . | . | . | . | . | . | . | G | . | . | . |
| 21-225_4A2.001.004_LC | . | . | . | . | . | . | . | . | . | . | . | . | . | . | . | G | . | . | . |
| 21-225_4A2.001.005_LC | . | . | . | . | . | . | . | . | . | . | . | . | . | . | . | G | . | . | . |
| 21-225_4A2.001.006_LC | . | . | . | . | . | . | . | . | . | . | . | . | . | . | . | G | . | . | . |
| 21-225_4A2.001.007_LC | . | . | . | . | . | . | . | . | . | . | . | . | . | . | . | G | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | T | P | V | T | F | G | P | G | T | K | V | D | I | K | R |
| 21-225_4A2.001.003_LC | | | | | | | | | | | | | | | | Q | | | |
| 21-225_4A2.001.009_LC | | | | | A | | | | | | | | | | | Q | | | |
| 21-225_4A2.001.010_LC | | | | | | | | | | | | | | | | Q | | | |
| 21-225_4A2.001.011_LC | | | | | | | | | | | | | | | | Q | | | |
| 21-225_4A2.001.012_LC | | | | | | | | | | | | | | | | Q | | | |
| 21-225_4A2.001.022_LC | | | | | | | | | | | | | | | | Q | | | |
| 21-225_4A2.001.024_LC | | | | | | | | | | | | | | | | Q | | | |
| 21-225_6G6_LC | | | | | | | F | | | | H | | | | | | | | |

**FIGURE 57**

Table 110.    Consensus 30 - VL7|7a/JL2 (SEQ ID NO: 50327):

QTVVTQE-PSLTVSPGGTVTLTC<u>ASST-GAVTSG</u>----<u>YYPNWFQQKPGQAPRALIY</u><u>S--------TSNKIIS</u>WTPARFSGSLLG--

GKAALTLSGVQPEDEAEYYC<u>LLYYG--------------------GAQLV</u>FGGGTKLTVLG wherein:

A at position 24 can be substituted with V or G

S at position 25 can be substituted with F or L

S at position 26 can be substituted with N

G at position 29 can be substituted with E

A at position 30 can be substituted with S or T

G at position 34 can be substituted with A

Y at position 39 can be substituted with S, N or F

Y at position 40 can be substituted with F

N at position 42 can be substituted with S or Q

S at position 58 can be substituted with H or N

S at position 68 can be substituted with N, D, I or T

K at position 70 can be substituted with R

L at position 107 can be substituted with M

L at position 108 can be substituted with I or F

Y at position 109 can be substituted with F

Y at position 110 can be substituted with C, F or S

G at position 111 can be substituted with D

A at position 135 can be substituted with V

FIGURE 57

Q at position 136 can be substituted with H

L at position 137 can be substituted with V or M

V at position 138 can be substituted with A, I, G or M

Xaa Xaa Xaa Thr Xaa Xaa Val Thr Ser Xaa Xaa Xaa Pro Xaa (SEQ ID NO: 50165)

Xaa Thr Xaa Asn Xaa His Ser (SEQ ID NO: 50166)

Xaa Xaa Xaa Xaa Xaa Gly Xaa Xaa Xaa Xaa (SEQ ID NO: 50167)

Ala Ser Ser Thr Gly Ala Val Thr Ser Gly Tyr Tyr Pro Asn (SEQ ID NO: 50651)

Ser Thr Ser Asn Lys His Ser (SEQ ID NO: 50652)

Leu Leu Tyr Tyr Gly Gly Ala Gln Leu Val (SEQ ID NO: 50653)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | L_FR1 | | | | | | | | | | | | | | |
| CONSENSUS | Q | T | V | V | T | Q | E | - | P | S | L | T | V | S | P | G | G | T | V | T | L | T | C | A | S | S |
| 21-225_190D1_LC | . | . | . | . | . | . | . | . | . | . | . | . | . | . | . | . | . | . | . | . | . | . | . | . | . | |
| 21-225_190F10_LC | . | . | . | . | . | . | . | . | . | . | . | . | . | . | . | . | . | . | . | . | . | . | . | V | P | |
| 21-225_191A2_LC | . | . | . | . | . | . | . | . | . | . | . | . | . | . | . | . | . | . | . | . | . | . | . | V | L | . |
| 21-225_191H9_LC | . | . | . | . | . | . | . | . | . | . | . | . | . | . | . | . | . | . | . | . | . | . | . | . | . | |
| 21-225_192E3_LC | . | . | . | . | . | . | . | . | . | . | . | . | . | . | . | . | . | . | . | . | . | . | . | . | P | . |
| 21-225_192G7_LC | . | . | . | . | . | . | . | . | . | . | . | . | . | . | . | . | . | . | . | . | . | . | . | . | . | |
| 21-225_192H8_LC | . | . | . | . | . | . | . | . | . | . | . | . | . | . | . | . | . | . | . | . | . | . | . | . | P | |
| 21-225_194D12_LC | . | . | . | . | . | . | . | . | . | . | . | . | . | . | . | . | . | . | . | . | . | . | . | . | . | |
| 21-225_194G5_LC | . | . | . | . | . | . | . | . | . | . | . | . | . | . | . | . | . | . | . | . | . | . | . | . | . | |
| 21-225_196E7_LC | . | . | . | . | . | . | . | . | . | . | . | . | . | . | . | . | . | . | . | . | . | . | . | . | P | |
| 21-225_197B11_LC | . | . | . | . | . | . | . | . | . | . | . | . | . | . | . | . | . | . | . | . | . | . | . | . | P | |
| 21-225_197C11_LC | . | . | . | . | . | . | . | . | . | . | . | . | . | . | . | . | . | . | . | . | . | . | . | . | . | |
| 21-225_198B8_LC | . | . | . | . | . | . | . | S | . | . | . | . | . | . | . | . | . | . | . | . | . | . | . | V | L | |
| 21-225_200A11_LC | . | . | . | . | . | . | . | . | . | . | . | . | . | . | . | . | . | . | . | . | . | . | . | . | . | |
| 21-225_200G9_LC | . | . | . | . | . | . | . | . | . | . | . | . | . | . | . | . | . | . | . | . | . | . | . | . | . | N |
| 21-225_200H1_LC | . | . | . | . | . | . | . | . | . | . | . | . | . | . | . | . | . | . | . | . | . | . | . | . | . | N |
| 21-225_210F11_LC | . | . | . | . | . | . | . | . | . | . | . | . | . | . | . | . | . | . | . | . | . | . | . | . | P | |
| 21-225_211C9_LC | . | . | . | . | . | . | . | . | . | . | . | . | . | . | . | . | . | . | . | . | . | . | . | G | . | |
| 21-225_211E8_LC | . | . | . | . | . | . | . | . | . | . | . | . | . | . | . | . | . | . | . | . | . | . | . | . | . | . |
| 21-225_211H7_LC | . | . | . | . | . | . | . | . | . | . | . | . | . | . | . | . | . | . | . | . | . | . | . | . | P | . |
| 21-225_212A9_LC | . | . | . | . | . | . | . | . | . | . | . | . | . | . | . | . | . | . | . | . | . | . | . | . | . | . |
| 21-225_212H12_LC | . | . | . | . | . | . | . | . | . | . | . | . | . | . | . | . | . | . | . | . | . | . | . | . | . | . |
| 21-225_213F5_LC | . | . | . | . | . | . | . | . | . | . | . | . | . | . | . | . | . | . | . | . | . | . | . | G | . | . |
| 21-225_214E12_LC | . | . | . | . | . | . | . | . | . | . | . | . | . | . | . | . | . | . | . | . | . | . | . | . | . | . |
| 21-225_214H3_LC | . | . | . | . | . | . | . | . | . | . | . | . | . | . | . | . | . | . | . | . | . | . | . | . | . | . |
| 21-225_215A3_LC | . | . | . | . | . | . | . | . | . | . | . | . | . | . | . | . | . | . | . | . | . | . | . | . | . | . |
| 21-225_215H3_LC | . | . | . | . | . | . | . | . | . | . | . | . | . | . | . | . | . | . | . | . | . | . | . | . | . | . |
| 21-225_216A7_LC | . | . | . | . | . | . | . | . | . | . | . | . | . | . | . | . | . | . | . | . | . | . | . | . | . | . |
| 21-225_216H11_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | L_FR1 | | | | | | | | | | | | | |
| CONSENSUS | Q | T | V | V | T | Q | E | - | P | S | L | T | V | S | P | G | G | T | V | T | L | T | C | A | S | S |
| 21-225_74E5_LC | . | . | . | . | . | . | . | | . | . | . | . | . | . | . | . | . | . | . | . | . | . | . | . | . | . |
| 21-225_78D10_LC | . | . | . | . | . | . | | | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | L_CDR1 | | | | | | | | | | | | | L_FR2 | | | | | | | | |
| CONSENSUS | T | - | G | A | V | T | S | G | - | - | - | - | Y | Y | P | N | W | F | Q | Q | K | P | G | Q | A | P |
| 21-225_190D1_LC | . | . | . | . | . | . | . | . | . | . | . | . | S | . | | | | | | | | | | | | |
| 21-225_190F10_LC | . | . | . | . | . | . | . | . | . | . | . | . | S | F | | | | | | | | | | | | |
| 21-225_191A2_LC | . | . | . | . | . | . | . | . | . | . | . | . | S | F | | | | | | | | | | | | |
| 21-225_191H9_LC | . | . | . | . | . | . | . | A | . | . | . | . | . | . | | | | L | | | | | | | | |
| 21-225_192E3_LC | . | . | . | S | . | . | . | . | . | . | . | . | S | . | | | | | | | | | | | | |
| 21-225_192G7_LC | . | . | . | . | . | . | . | . | . | . | . | . | N | . | | Q | | | | | | | | | | |
| 21-225_192H8_LC | . | . | . | T | . | . | . | . | . | . | . | . | F | . | | | | | | | | | | | | |
| 21-225_194D12_LC | . | . | . | . | . | . | . | . | . | . | . | . | S | F | | S | | | | | | | | | | |
| 21-225_194G5_LC | . | . | . | . | . | . | . | . | . | . | . | . | N | . | | | | | | | | | | | T | |
| 21-225_196E7_LC | . | . | . | . | . | . | . | A | . | . | . | . | . | . | | | | L | | | | | | | | |
| 21-225_197B11_LC | . | . | . | S | . | . | . | . | . | . | . | . | S | . | | | | | | | | | | | | |
| 21-225_197C11_LC | . | . | . | . | . | . | . | A | . | . | . | . | . | . | | | | L | | | | | | | | |
| 21-225_198B8_LC | . | . | . | . | . | . | . | . | . | . | . | . | S | F | | | | | | | | | | | | |
| 21-225_200A11_LC | . | . | . | . | . | . | . | . | . | . | . | . | . | . | | | | | | | | | | | V | |
| 21-225_200G9_LC | . | . | . | . | . | . | . | . | . | . | . | . | S | . | | | | L | | | | | | | | |
| 21-225_200H1_LC | . | . | . | . | . | . | . | . | . | . | . | . | S | . | | | | | | | | | | | | |
| 21-225_210F11_LC | . | . | . | . | . | . | . | . | . | . | . | . | N | . | | | | | | | | | | | | |
| 21-225_211C9_LC | . | . | . | S | . | . | . | . | . | . | . | . | . | F | | | | | | | | | | | | |
| 21-225_211E9_LC | . | . | . | . | . | . | . | . | . | . | . | . | N | . | | | | | | | | | | | | |
| 21-225_211H7_LC | . | . | . | . | . | . | . | . | . | . | . | . | N | . | | . | | S | | | | | | | V | |
| 21-225_212A9_LC | . | . | . | . | . | . | . | . | . | . | . | . | . | . | | | | | | | | | | | | |
| 21-225_212H12_LC | . | . | . | . | . | . | . | . | . | . | . | . | . | . | | | | | | | | | | | | |
| 21-225_213F5_LC | . | . | . | S | . | . | . | . | . | . | . | . | . | F | | | | | | | | | | | T | |
| 21-225_214E12_LC | . | . | . | . | . | . | . | . | . | . | . | . | . | . | | | | | | | | | | | | |
| 21-225_214H3_LC | . | . | . | . | . | . | . | . | . | . | . | . | . | . | | | | | | | | | | | | |
| 21-225_215A3_LC | . | ≈ | . | . | . | . | . | . | . | . | . | . | N | . | | . | | S | | | | | | | | |
| 21-225_215H3_LC | . | . | . | . | . | . | . | . | . | . | . | . | . | . | | | | | | | | | | | | |
| 21-225_216A7_LC | . | . | . | . | . | . | . | . | . | . | . | . | . | . | | | | | | | | | | | | |
| 21-225_216H11_LC | . | . | . | . | . | . | . | . | . | . | . | . | . | . | | | | | | | | | | | | |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | L_CDR1 | | | | | | | | | | | | | | | | | | L_FR2 | | |
| CONSENSUS | T | - | G | A | V | T | S | G | - | - | - | - | Y | Y | P | N | W | F | Q | Q | K | P | G | Q | A | P |
| 21-225_74E5_LC | . | . | E | T | . | . | | | | | . | . | S | . | . | . | . | . | | | . | . | . | . | . | . |
| 21-225_78D10_LC | . | . | . | . | . | . | | | . | | . | . | . | F | . | . | . | . | | | | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | L_CDR2 | | | | | | | | | | | | | | | | |
| CONSENSUS | R | A | L | I | Y | S | - | - | - | - | - | - | - | - | T | S | N | K | H | S | W | T | P | A | R | F |
| 21-225_190D1_LC | . | . | . | . | . | . | . | . | . | . | . | . | . | . | . | . | . | . | . | . | . | . | . | . | . | . |
| 21-225_190F10_LC | . | . | . | . | . | . | . | . | . | . | . | . | . | . | . | . | . | . | . | . | . | . | . | . | . | . |
| 21-225_191A2_LC | . | . | . | . | . | . | . | . | . | . | . | . | . | . | . | . | . | . | . | . | . | . | . | . | . | . |
| 21-225_191H9_LC | . | T | . | . | . | . | . | . | . | . | . | . | . | . | . | N | . | . | . | . | . | . | . | . | . | . |
| 21-225_192E3_LC | . | . | . | . | . | . | . | . | . | . | . | . | . | . | . | N | . | . | . | . | . | . | . | . | . | . |
| 21-225_192G7_LC | . | . | . | . | . | . | . | . | . | . | . | . | . | . | . | Y | . | . | P | . | . | . | . | . | . | . |
| 21-225_192H8_LC | . | . | . | . | . | N | . | . | . | . | . | . | . | . | . | . | . | . | P | . | . | . | . | . | . | . |
| 21-225_194D12_LC | . | . | . | . | . | . | . | . | . | . | . | . | . | . | . | . | . | . | P | . | . | S | . | . | . | . |
| 21-225_194G5_LC | . | . | . | . | . | . | . | . | . | . | . | . | . | . | . | . | . | . | . | . | . | . | . | . | . | . |
| 21-225_196E7_LC | . | T | . | . | . | . | . | . | . | . | . | . | . | . | . | N | . | . | . | . | . | . | . | . | . | . |
| 21-225_197B11_LC | . | . | . | . | . | . | . | . | . | . | . | . | . | . | . | N | . | . | . | . | . | . | . | . | . | . |
| 21-225_197C11_LC | . | T | . | . | . | . | . | . | . | . | . | . | . | . | . | . | . | . | . | . | . | . | . | . | . | . |
| 21-225_198B8_LC | . | . | . | . | . | . | . | . | . | . | . | . | . | . | . | . | . | . | . | . | . | . | . | . | . | . |
| 21-225_200A11_LC | . | . | . | . | . | . | . | . | . | . | . | . | . | . | . | N | . | . | . | . | . | . | . | . | . | . |
| 21-225_200G9_LC | . | . | . | . | . | N | . | . | . | . | . | . | . | . | . | D | . | . | . | . | . | . | . | . | . | . |
| 21-225_200H1_LC | . | . | . | . | . | N | . | . | . | . | . | . | . | . | . | N | . | . | . | . | . | . | . | . | . | . |
| 21-225_210F11_LC | . | . | . | . | . | . | . | . | . | . | . | . | . | . | . | . | . | . | . | . | . | . | . | . | . | . |
| 21-225_211C9_LC | . | P | . | . | . | . | . | . | . | . | . | . | . | . | . | N | . | . | . | . | . | . | . | . | . | . |
| 21-225_211E8_LC | . | . | . | . | . | . | . | . | . | . | . | . | . | . | i | . | . | . | . | . | G | . | . | . | . | . |
| 21-225_211H7_LC | . | . | . | . | . | . | . | . | . | . | . | . | . | . | . | . | . | N | . | . | . | . | . | . | . | . |
| 21-225_212A9_LC | . | . | . | . | . | . | . | . | . | . | . | . | . | . | . | N | . | . | . | . | . | . | . | . | . | . |
| 21-225_212H12_LC | . | . | . | . | . | . | . | . | . | . | . | . | . | . | . | . | . | . | . | . | . | . | . | . | . | . |
| 21-225_213F5_LC | . | P | . | . | . | . | . | . | . | . | . | . | . | . | . | N | . | . | . | . | . | . | . | . | . | . |
| 21-225_214E12_LC | . | . | . | . | . | . | . | . | . | . | . | . | . | . | . | . | . | . | . | . | . | . | . | . | . | . |
| 21-225_214H3_LC | . | . | . | . | . | . | . | . | . | . | . | . | . | . | . | . | . | . | . | . | C | . | . | . | . | . |
| 21-225_215A3_LC | . | . | . | . | . | . | . | . | . | . | . | . | . | . | . | . | . | . | . | . | G | . | . | . | . | . |
| 21-225_215H3_LC | . | . | . | . | . | . | . | . | . | . | . | . | . | . | . | N | . | . | . | . | C | G | . | . | . | . |
| 21-225_216A7_LC | . | . | . | . | . | . | . | . | . | . | . | . | . | . | . | N | . | . | . | . | C | . | . | . | . | . |
| 21-225_216H11_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | L_CDR2 | | | | | | | | | | | | | | | |
| CONSENSUS | R | A | L | I | Y | S | - | - | - | - | - | - | - | - | T | S | N | K | H | S | W | T | P | A | R | F |
| 21-225_74E5_LC | . | . | . | . | . | . | | | | | . | . | . | . | . | . | . | | | . | . | . | . | . | . | . |
| 21-225_78D10_LC | . | . | . | . | . | . | | | . | . | . | . | . | . | C | . | . | | | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| CONSENSUS | S | G | S | L | L | G | - | - | G | K | A | A | L | T | L | S | G | V | Q | P | E | D | E | A | E | Y |
| 21-225_190D1_LC | . | . | . | . | . | . | . | . | . | . | . | . | . | . | . | . | . | . | . | . | . | . | . | . | O | |
| 21-225_190F10_LC | . | . | . | . | . | . | . | . | . | . | . | . | . | . | . | . | . | . | . | . | . | . | . | . | O | |
| 21-225_191A2_LC | . | . | . | . | . | . | . | . | . | . | . | . | . | . | . | . | . | . | . | . | . | . | . | . | O | |
| 21-225_191H9_LC | . | . | . | . | . | . | . | . | . | . | . | . | . | . | . | . | O | . | . | . | . | . | . | . | . | |
| 21-225_192E3_LC | . | . | . | . | . | . | . | . | . | . | . | . | . | . | . | . | . | . | . | R | . | . | . | . | . | |
| 21-225_192G7_LC | . | . | . | . | . | . | . | . | . | . | . | . | . | . | . | . | . | . | . | . | . | . | . | . | . | |
| 21-225_192H8_LC | . | . | . | . | . | . | . | . | . | . | M | . | . | . | . | . | . | . | . | . | . | . | . | . | O | |
| 21-225_194D12_LC | . | . | . | . | . | . | . | . | . | . | . | . | . | . | . | . | . | . | . | . | . | . | . | . | O | |
| 21-225_194G5_LC | . | . | . | . | . | . | . | . | . | . | O | . | . | . | . | . | . | . | . | . | . | . | . | . | . | |
| 21-225_196E7_LC | . | . | . | . | . | . | . | . | . | . | . | . | . | . | . | . | O | . | . | . | . | . | . | . | . | |
| 21-225_197B11_LC | . | . | . | . | . | . | . | . | . | . | . | . | . | . | . | . | . | . | . | . | . | . | . | . | . | |
| 21-225_197C11_LC | . | . | . | . | . | . | . | . | . | . | . | . | . | . | . | . | O | . | . | . | . | . | . | . | . | |
| 21-225_198B8_LC | . | . | . | . | . | . | . | . | . | . | . | . | . | . | . | . | . | . | . | . | . | . | . | . | O | . |
| 21-225_200A11_LC | . | . | . | . | . | . | . | . | . | . | . | . | . | . | . | . | . | . | . | . | . | . | . | . | O | . |
| 21-225_200G9_LC | . | . | . | . | . | . | . | . | . | . | . | . | . | . | . | . | . | A | . | . | . | . | . | . | . | . |
| 21-225_200H1_LC | . | . | . | . | . | . | . | . | . | . | . | . | . | . | . | . | . | . | . | . | . | . | . | . | . | . |
| 21-225_210F11_LC | . | . | . | . | . | . | . | . | . | . | . | . | . | . | . | . | . | . | . | . | . | . | . | . | . | . |
| 21-225_211C9_LC | . | . | . | . | . | . | . | . | . | . | . | . | . | . | . | . | O | . | . | . | . | . | . | . | O | . |
| 21-225_211E8_LC | T | . | R | . | . | . | . | . | . | . | . | . | . | . | . | . | . | . | . | . | . | . | . | . | . | . |
| 21-225_211H7_LC | . | . | . | . | . | . | . | . | . | . | . | . | . | . | . | . | . | . | . | . | . | . | . | . | . | . |
| 21-225_212A9_LC | . | . | . | . | . | . | . | . | . | . | . | . | . | . | . | . | . | . | . | O | . | . | . | . | O | . |
| 21-225_212H12_LC | . | . | . | . | . | . | . | . | . | . | . | . | . | . | . | . | . | . | . | . | . | . | . | . | . | . |
| 21-225_213F5_LC | . | . | . | . | . | . | . | . | . | . | T | . | . | . | . | . | O | . | . | . | . | . | . | . | O | . |
| 21-225_214E12_LC | . | . | . | . | . | . | . | . | . | . | . | . | . | . | . | . | O | . | . | . | . | . | . | . | . | . |
| 21-225_214H3_LC | . | . | . | . | . | . | . | . | . | . | . | . | . | . | . | . | . | . | . | . | . | . | . | . | . | . |
| 21-225_215A3_LC | T | . | . | . | . | . | . | . | . | . | . | . | . | . | . | . | . | . | . | . | . | . | . | . | . | . |
| 21-225_215H3_LC | . | . | . | . | . | . | . | . | . | . | . | . | . | . | . | . | . | . | . | O | . | . | . | . | O | . |
| 21-225_216A7_LC | . | . | . | . | . | . | . | . | . | . | . | . | . | . | . | . | . | . | . | O | . | . | . | . | O | . |
| 21-225_216H11_LC | . | . | . | . | . | . | . | . | . | . | . | . | . | . | . | . | O | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | L FR3 | | | | | | | | | | | | | | | |
| CONSENSUS | S | G | S | L | L | G | - | - | G | K | A | A | L | T | L | S | G | V | Q | P | E | D | E | A | E | Y |
| 21-225_74E5_LC | . | . | . | . | . | . | | | . | . | . | . | . | . | . | . | O | . | . | . | . | . | . | . | . | . |
| 21-225_78D10_LC | . | . | . | . | . | . | | | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | L_CDR3 | | | | | | | | |
| CONSENSUS | Y | C | L | L | Y | Y | G | - | - | - | - | - | - | - | - | - | - | - | - | - | - | - | - | - | - | - |
| 21-225_190D1_LC | . | . | . | . | . | . | . | . | . | . | . | . | . | . | . | . | . | . | . | . | . | . | . | . | | |
| 21-225_190F10_LC | . | . | . | . | . | C | . | . | . | . | . | . | . | . | . | . | . | . | . | . | . | . | . | . | | |
| 21-225_191A2_LC | . | . | M | . | . | C | . | . | . | . | . | . | . | . | . | . | . | . | . | . | . | . | . | . | | |
| 21-225_191H9_LC | . | . | . | I | F | . | . | . | . | . | . | . | . | . | . | . | . | . | . | . | . | . | . | . | | |
| 21-225_192E3_LC | . | . | . | . | . | . | . | . | . | . | . | . | . | . | . | . | . | . | . | . | . | . | . | . | | |
| 21-225_192G7_LC | . | . | . | F | . | . | . | . | . | . | . | . | . | . | . | . | . | . | . | . | . | . | . | . | | |
| 21-225_192H8_LC | . | . | . | . | . | . | . | . | . | . | . | . | . | . | . | . | . | . | . | . | . | . | . | . | | |
| 21-225_194D12_LC | . | . | . | I | . | . | . | . | . | . | . | . | . | . | . | . | . | . | . | . | . | . | . | . | | |
| 21-225_194G5_LC | . | . | . | I | . | . | . | . | . | . | . | . | . | . | . | . | . | . | . | . | . | . | . | . | | |
| 21-225_196E7_LC | . | . | . | I | F | . | . | . | . | . | . | . | . | . | . | . | . | . | . | . | . | . | . | . | | |
| 21-225_197B11_LC | . | . | . | . | . | . | . | . | . | . | . | . | . | . | . | . | . | . | . | . | . | . | . | . | | |
| 21-225_197C11_LC | . | . | . | I | F | . | . | . | . | . | . | . | . | . | . | . | . | . | . | . | . | . | . | . | | |
| 21-225_198B8_LC | . | F | M | . | . | S | . | . | . | . | . | . | . | . | . | . | . | . | . | . | . | . | . | . | | |
| 21-225_200A11_LC | . | . | . | . | . | . | . | . | . | . | . | . | . | . | . | . | . | . | . | . | . | . | . | . | | |
| 21-225_200G9_LC | . | . | . | I | . | . | . | . | . | . | . | . | . | . | . | . | . | . | . | . | . | . | . | . | | |
| 21-225_200H1_LC | . | . | . | I | . | . | . | . | . | . | . | . | . | . | . | . | . | . | . | . | . | . | . | . | | |
| 21-225_210F11_LC | . | . | . | . | . | . | . | . | . | . | . | . | . | . | . | . | . | . | . | . | . | . | . | . | | |
| 21-225_211C9_LC | . | . | . | . | . | . | . | . | . | . | . | . | . | . | . | . | . | . | . | . | . | . | . | . | | |
| 21-225_211E8_LC | . | . | . | . | . | . | . | . | . | . | . | . | . | . | . | . | . | . | . | . | . | . | . | . | . | . |
| 21-225_211H7_LC | . | . | . | . | . | . | . | . | . | . | . | . | . | . | . | . | . | . | . | . | . | . | . | . | . | . |
| 21-225_212A9_LC | . | . | . | . | . | . | . | . | . | . | . | . | . | . | . | . | . | . | . | . | . | . | . | . | . | . |
| 21-225_212H12_LC | . | . | . | . | . | . | . | . | . | . | . | . | . | . | . | . | . | . | . | . | . | . | . | . | . | . |
| 21-225_213F5_LC | . | . | . | . | . | F | . | . | . | . | . | . | . | . | . | . | . | . | . | . | . | . | . | . | . | . |
| 21-225_214E12_LC | . | . | . | . | . | C | D | . | . | . | . | . | . | . | . | . | . | . | . | . | . | . | . | . | . | . |
| 21-225_214H3_LC | . | . | . | . | . | . | . | . | . | . | . | . | . | . | . | . | . | . | . | . | . | . | . | . | . | . |
| 21-225_215A3_LC | . | . | . | . | . | . | . | . | . | . | . | . | . | . | . | . | . | . | . | . | . | . | . | . | . | . |
| 21-225_215H3_LC | . | . | . | . | . | . | . | . | . | . | . | . | . | . | . | . | . | . | . | . | . | . | . | . | . | . |
| 21-225_216A7_LC | . | . | . | . | . | . | . | . | . | . | . | . | . | . | . | . | . | . | . | . | . | . | . | . | . | . |
| 21-225_216H11_LC | . | . | . | . | . | C | D | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | L_CDR3 | | | | | | | | | |
| CONSENSUS | Y | C | L | L | Y | Y | G | - | - | - | - | - | - | - | - | - | - | - | - | - | - | - | - | - | - | - |
| 21-225_74E5_LC | . | . | . | . | . | . | | | . | . | . | . | . | . | . | . | | . | . | . | . | . | . | . | . | . |
| 21-225_78D10_LC | . | . | . | . | . | . | | . | . | . | . | . | . | . | . | . | | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | L_FR4 | | | | | | | | | | | |
| CONSENSUS | - | - | - | G | A | Q | L | V | F | G | G | G | T | K | L | T | V | L | G |
| 21-225_190D1_LC | . | . | . | . | . | . | V | A | . | . | . | . | . | . | . | . | . | . | . |
| 21-225_190F10_LC | . | . | . | . | . | . | . | . | . | . | . | . | . | . | . | . | . | . | . |
| 21-225_191A2_LC | . | . | . | . | . | . | . | . | . | . | . | . | . | . | . | . | . | . | . |
| 21-225_191H9_LC | . | . | . | . | V | H | V | I | . | . | . | . | . | . | . | . | . | . | . |
| 21-225_192E3_LC | . | . | . | . | . | . | . | . | . | . | . | . | . | . | . | . | . | . | . |
| 21-225_192G7_LC | . | . | . | . | . | . | V | I | . | . | . | . | . | . | . | . | . | . | . |
| 21-225_192H8_LC | . | . | . | . | . | . | . | M | . | . | . | . | . | . | . | . | . | . | . |
| 21-225_194D12_LC | . | . | . | . | . | . | . | A | . | . | . | . | . | . | . | . | . | . | S |
| 21-225_194G5_LC | . | . | . | . | . | . | . | M | . | . | . | . | . | . | . | . | . | . | . |
| 21-225_196E7_LC | . | . | . | . | V | H | V | I | . | . | . | . | . | . | . | . | . | . | . |
| 21-225_197B11_LC | . | . | . | . | . | . | . | . | . | . | . | . | . | . | . | . | . | . | . |
| 21-225_197C11_LC | . | . | . | . | V | H | V | I | . | . | . | . | . | . | . | . | . | . | . |
| 21-225_198B8_LC | . | . | . | . | . | . | M | . | . | . | . | . | . | . | . | . | . | . | . |
| 21-225_200A11_LC | . | . | . | . | . | H | . | A | . | . | . | . | . | . | . | . | . | . | . |
| 21-225_200G9_LC | . | . | . | . | . | . | . | . | . | . | . | . | . | . | . | . | . | . | . |
| 21-225_200H1_LC | . | . | . | . | . | . | . | . | . | . | . | . | . | . | . | . | . | . | . |
| 21-225_210F11_LC | . | . | . | . | . | . | . | . | . | . | . | . | . | . | . | . | . | . | . |
| 21-225_211C9_LC | . | . | . | . | . | . | . | A | . | . | . | . | . | . | . | . | . | . | . |
| 21-225_211E9_LC | . | . | . | . | . | . | . | A | . | . | . | . | . | . | . | . | . | . | . |
| 21-225_211H7_LC | . | . | . | . | . | . | . | . | . | . | . | . | . | . | . | . | . | . | . |
| 21-225_212A9_LC | . | . | . | . | . | . | V | G | . | . | . | . | . | . | . | . | . | . | S |
| 21-225_212H12_LC | . | . | . | . | . | H | V | . | . | . | . | . | . | . | . | . | . | . | . |
| 21-225_213F5_LC | . | . | . | . | . | . | . | A | . | . | . | . | . | . | . | . | . | . | . |
| 21-225_214E12_LC | . | . | . | . | . | . | . | . | . | . | . | . | . | . | . | . | . | . | . |
| 21-225_214H3_LC | . | . | . | . | . | H | V | . | . | . | . | . | . | . | . | . | . | . | . |
| 21-225_215A3_LC | . | . | . | . | . | . | . | A | . | . | . | . | . | . | . | A | . | . | . |
| 21-225_215H3_LC | . | . | . | . | . | . | V | G | . | . | . | . | . | . | . | . | . | . | S |
| 21-225_216A7_LC | . | . | . | . | . | . | V | G | . | . | . | . | . | . | . | . | . | . | S |
| 21-225_216H11_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | L_FR4 | | | | | |
| CONSENSUS | - | - | - | G | A | Q | L | V | F | G | G | G | T | K | L | T | V | L | G |
| 21-225_74E5_LC | . | . | . | . | . | . | . | . | . | . | . | . | . | . | . | . | . | . | . |
| 21-225_78D10_LC | . | . | . | . | . | . | . | . | . | . | . | . | . | . | . | . | . | . | . |

**FIGURE 57**

Table 111.    Consensus 31 - VK3|L2/JK2 (SEQ ID NO: 50328):

EIVMTQSPATLSVSPGERATLSC<u>RAS</u>--<u>QSVN</u>------<u>SNLA</u>WYQQKPGQAPRLLIY<u>G</u>-------<u>ASTRATGI</u>PARFSGSGSG--
TEFTLTISSLQSEDFAVYYC<u>QQYND</u>----------------------WP<u>CS</u>FGQGTKLEIKR wherein:

A at position 25 can be substituted with S

Q at position 29 can be substituted with L, M or V

S at position 30 can be substituted with N, D, R or T

V at position 31 can be substituted with I

N at position 32 can be substituted with K, S, V, A, I or L

S at position 39 can be substituted with N or T

N at position 40 can be substituted with S or Y

G at position 58 can be substituted with I, F or V

A at position 67 can be substituted with T

T at position 69 can be substituted with I

Q at position 108 can be substituted with E

Y at position 109 can be substituted with F

N at position 110 can be substituted with Y or D

D at position 111 can be substituted with N

Null (-) at position 134 can be substituted with W

W at position 135 can be substituted with P

P at position 136 can be substituted with L or M

FIGURE 57

Arg Xaa Ser Xaa Xaa Xaa Xaa Xaa Xaa Leu Ala (SEQ ID NO: 50168)

Xaa Xaa Ser Xaa Arg Ala Thr (SEQ ID NO: 50169)

Gln Xaa Xaa Xaa Xaa Xaa Xaa Xaa Cys Ser (SEQ ID NO: 50170)

Arg Ala Ser Gln Ser Val Asn Ser Asn Leu Ala (SEQ ID NO: 50654)

Gly Ala Ser Thr Arg Ala Thr (SEQ ID NO: 50655)

Gln Gln Tyr Asn Asp Trp Pro Cys Ser (SEQ ID NO: 50656)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | E | I | V | M | T | Q | S | P | A | T | L | S | V | S | P | G | E | R | A | T | L | S | C | R | A | S |
| 21-225_162A10_LC | . | . | . | . | . | . | . | . | . | . | . | . | . | . | . | . | . | . | . | . | . | . | . | . | . | . |
| 21-225_193G12_LC | . | . | . | . | . | . | . | . | . | . | . | . | . | . | . | . | . | . | . | . | . | . | . | . | . | . |
| 21-225_201A4_LC | . | . | . | . | . | . | . | . | . | . | . | . | . | . | . | . | . | S | . | . | . | . | . | . | S | . |
| 21-225_201F2_LC | . | . | . | . | . | . | . | . | . | . | . | . | . | . | . | . | . | . | . | . | . | . | . | . | . | . |
| 21-225_201F7_LC | . | . | . | . | . | . | . | . | . | . | . | . | . | . | . | . | . | . | . | . | . | . | . | . | . | . |
| 21-225_201H4_LC | . | . | . | . | . | . | . | . | . | . | . | . | . | . | . | . | . | . | . | . | . | . | . | . | . | . |
| 21-225_202A8_LC | . | . | . | . | . | . | . | . | . | . | . | . | . | . | . | . | . | . | . | . | . | . | . | . | . | . |
| 21-225_202F12_LC | . | . | . | . | . | . | . | . | . | . | . | . | . | . | . | . | . | . | . | . | . | . | . | . | . | . |
| 21-225_205G4_LC | . | . | . | . | . | . | . | . | . | . | . | . | . | . | . | . | . | . | . | . | . | . | . | . | . | . |
| 21-225_210H10_LC | . | . | . | . | . | . | . | . | . | . | . | . | . | . | . | . | . | . | . | . | . | . | . | . | . | . |
| 21-225_212C10_LC | . | . | . | . | . | . | . | . | . | . | . | . | . | . | . | . | . | . | . | . | . | . | . | . | . | . |
| 21-225_216B10_LC | . | . | . | . | . | M | . | . | . | . | . | . | . | . | . | . | . | . | . | . | . | . | . | . | . | . |
| 21-225_24H3_LC | . | . | . | . | . | . | . | . | . | . | . | . | . | . | . | . | . | . | . | . | . | . | . | . | . | . |
| 21-225_57H3_LC | . | . | . | . | . | . | . | . | . | . | . | . | . | . | . | . | . | . | . | . | . | . | . | . | . | . |
| 21-225_62E6_LC | . | . | . | . | . | . | . | . | . | . | . | . | . | . | . | . | . | . | . | . | . | . | . | . | . | . |
| 21-225_65A6_LC | . | . | . | . | . | . | . | . | V | . | . | P | . | . | . | . | . | . | . | . | . | . | . | . | . | . |
| 21-225_67C3_LC | . | . | . | . | . | . | . | . | . | . | . | . | . | . | . | . | . | . | . | . | . | . | . | . | . | . |
| 21-225_70A5_LC | . | . | . | . | . | . | . | . | . | . | . | . | . | . | . | . | . | . | . | . | . | . | . | . | . | . |
| 21-225_74C3_LC | . | . | . | . | . | . | . | . | . | . | . | . | . | . | . | . | . | . | . | . | . | . | . | . | . | . |
| 21-225_74E11_LC | . | . | . | . | . | . | . | . | . | . | . | . | . | . | . | . | . | . | . | . | . | . | . | . | . | . |
| 21-225_74E3_LC | . | . | . | . | . | . | . | . | . | . | . | L | . | . | . | . | . | . | . | . | . | . | . | . | . | . |
| 21-225_76D2_LC | . | . | . | . | . | . | . | . | S | . | . | . | . | . | . | . | . | . | . | . | . | . | . | . | . | . |
| 21-225_77H5_LC | . | . | . | . | . | . | . | . | V | . | . | . | . | . | . | . | . | . | . | . | . | . | . | . | . | . |
| 21-225_92H5_LC | . | . | . | . | . | . | . | . | . | . | . | . | . | . | . | . | . | . | . | . | . | . | . | . | . | . |
| 21-225_95G2_LC | . | . | . | . | . | . | . | . | . | . | . | L | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | V | N | - | - | - | - | - | - | S | N | L | A | W | Y | Q | Q | K | P | G | Q | A | P |
| 21-225_162A10_LC | . | . | . | T | . | . | . | . | . | . | . | . | . | . | . | . | . | . | . | . | . | . | S | . | . | . |
| 21-225_193G12_LC | . | . | . | . | . | S | . | . | . | . | . | . | . | S | . | . | . | . | . | . | . | . | . | . | . | . |
| 21-225_201A4_LC | . | . | . | N | S | X | . | . | . | . | . | . | . | . | . | . | . | . | . | . | . | . | . | . | . | . |
| 21-225_201F2_LC | . | . | . | N | S | X | . | . | . | . | . | . | . | . | . | . | . | . | . | . | . | . | . | . | . | . |
| 21-225_201F7_LC | . | . | . | N | S | X | . | . | . | . | . | . | N | . | . | . | . | . | . | . | . | . | . | . | . | . |
| 21-225_201H4_LC | . | . | . | . | . | . | . | . | . | . | . | . | . | Y | . | . | . | . | . | . | . | . | . | . | . | . |
| 21-225_202A8_LC | . | . | . | R | S | . | . | . | . | . | . | . | N | . | . | . | . | . | . | . | N | . | . | . | . | . |
| 21-225_202F12_LC | . | . | . | . | . | L | . | . | . | . | . | . | N | . | . | . | . | . | . | . | R | . | . | . | . | . |
| 21-225_205G4_LC | . | . | . | N | S | X | . | . | . | . | . | . | . | . | . | . | . | . | . | . | . | . | . | . | . | . |
| 21-225_210H10_LC | . | . | . | . | . | R | . | . | . | . | . | . | . | . | . | . | . | . | . | . | . | . | . | . | . | . |
| 21-225_212C10_LC | . | . | . | . | . | A | . | . | . | . | . | . | . | S | . | . | . | . | . | . | . | . | . | . | . | . |
| 21-225_216B10_LC | . | . | . | . | . | . | . | . | . | . | . | . | N | . | . | . | . | . | R | . | . | . | . | . | . | . |
| 21-225_24H3_LC | . | . | . | . | . | S | . | . | . | . | . | . | . | . | . | . | . | . | . | . | . | . | . | . | . | . |
| 21-225_57H3_LC | . | . | . | . | . | S | . | . | . | . | . | . | . | S | . | . | . | . | . | . | . | . | . | . | . | . |
| 21-225_62E6_LC | . | . | . | . | . | . | . | . | . | . | . | . | . | . | . | . | . | . | . | . | . | . | . | . | . | . |
| 21-225_65A6_LC | . | . | . | . | . | . | . | . | . | . | . | . | . | . | . | . | . | . | . | . | . | . | . | . | . | . |
| 21-225_67C3_LC | . | . | . | . | . | . | . | . | . | . | . | . | . | . | . | . | . | . | . | . | . | . | . | . | . | . |
| 21-225_70A5_LC | . | . | . | . | . | . | . | . | . | . | . | . | . | . | . | . | . | . | . | L | . | . | . | . | . | . |
| 21-225_74C3_LC | . | . | . | . | . | A | . | . | . | . | . | . | . | S | . | . | . | . | . | . | . | . | . | . | . | . |
| 21-225_74E11_LC | . | . | L | . | . | V | . | . | . | . | . | . | N | S | . | . | . | . | . | . | . | . | . | . | . | . |
| 21-225_74E3_LC | . | . | L | . | . | V | . | . | . | . | . | . | N | S | . | . | . | . | . | . | . | . | . | . | . | . |
| 21-225_76D2_LC | . | . | V | . | . | V | . | . | . | . | . | . | N | S | . | . | . | . | . | . | . | . | . | . | . | . |
| 21-225_77H5_LC | . | . | . | . | . | S | . | . | . | . | . | . | . | S | . | . | . | . | . | . | . | . | . | . | L | . |
| 21-225_92H5_LC | . | . | D | . | . | I | . | . | . | . | . | . | T | Y | . | . | . | . | . | . | . | . | . | . | . | . |
| 21-225_95G2_LC | . | . | M | . | . | V | . | . | . | . | . | . | N | S | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | R | L | L | I | Y | G | - | - | - | - | - | - | - | - | A | S | T | R | A | T | G | I | P | A | R | F |
| 21-225_162A10_LC | . | . | . | . | F | . | . | . | . | . | . | . | . | . | . | . | . | . | . | . | V | . | . | . | . | I |
| 21-225_193G12_LC | . | I | . | . | S | . | . | . | . | . | . | . | . | . | . | . | . | . | . | . | . | . | . | . | . | . |
| 21-225_201A4_LC | . | . | . | . | F | . | . | . | . | . | . | . | . | . | . | . | . | . | . | . | . | . | . | . | . | . |
| 21-225_201F2_LC | . | . | . | . | F | . | . | . | . | . | . | . | . | . | . | . | . | . | . | . | . | . | . | . | . | . |
| 21-225_201F7_LC | . | . | . | . | F | . | . | . | . | . | . | . | . | . | . | . | . | . | . | . | . | . | . | . | . | . |
| 21-225_201H4_LC | . | . | . | . | . | . | . | . | . | . | . | . | . | . | . | . | . | . | . | . | . | . | . | . | . | . |
| 21-225_202A8_LC | . | . | . | . | . | . | . | . | . | . | . | . | . | . | . | . | . | . | . | . | . | V | . | . | . | . |
| 21-225_202F12_LC | . | . | . | . | . | . | . | . | . | . | . | . | . | . | . | . | . | . | . | . | . | . | . | . | . | . |
| 21-225_205G4_LC | . | . | . | . | F | . | . | . | . | . | . | . | . | . | . | . | . | . | . | . | . | . | . | . | . | . |
| 21-225_210H10_LC | . | . | . | . | . | . | . | . | . | . | . | . | . | . | . | . | . | . | . | . | . | . | . | . | . | . |
| 21-225_212C10_LC | . | . | . | . | R | . | . | . | . | . | . | . | . | . | T | . | . | . | . | . | D | V | . | . | . | . |
| 21-225_216B10_LC | . | . | . | . | . | . | . | . | . | . | . | . | . | . | . | . | . | . | . | . | . | . | . | P | . | . |
| 21-225_24H3_LC | . | . | . | . | F | . | . | . | . | . | . | . | . | . | . | . | I | . | . | . | . | . | . | . | . | . |
| 21-225_57H3_LC | . | . | . | . | . | . | . | . | . | . | . | . | . | . | . | . | . | . | . | . | . | F | . | . | . | . |
| 21-225_62E6_LC | . | . | . | . | S | V | . | . | . | . | . | . | . | . | . | . | . | . | . | . | . | . | . | . | . | . |
| 21-225_65A6_LC | . | . | . | . | . | . | . | . | . | . | . | . | . | . | . | . | . | . | . | . | . | . | . | . | . | . |
| 21-225_67C3_LC | . | . | . | . | S | I | . | . | . | . | . | . | . | . | . | . | . | . | . | . | . | . | . | . | . | . |
| 21-225_70A5_LC | . | . | . | . | S | I | . | . | . | . | . | . | . | . | . | . | . | . | . | . | . | . | . | P | . | . |
| 21-225_74C3_LC | . | . | . | . | F | . | . | . | . | . | . | . | . | . | T | . | . | . | . | . | . | . | . | . | . | . |
| 21-225_74E11_LC | . | . | . | . | R | . | . | . | . | . | . | . | . | . | . | . | . | . | . | . | . | . | . | . | . | . |
| 21-225_74E3_LC | . | . | . | . | . | . | . | . | . | . | . | . | . | . | . | . | . | . | . | . | . | . | . | . | . | . |
| 21-225_76D2_LC | . | . | . | . | R | . | . | . | . | . | . | . | . | . | . | . | . | . | . | . | . | . | . | . | . | . |
| 21-225_77H5_LC | . | . | . | . | . | . | . | . | . | . | . | . | . | . | . | . | . | . | . | . | . | . | . | . | . | . |
| 21-225_92H5_LC | . | . | . | . | . | . | . | . | . | . | . | . | . | . | . | . | . | . | . | . | . | V | . | . | . | . |
| 21-225_95G2_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
|  |  |  |  |  |  |  |  |  |  | K_FR3 |  |  |  |  |  |  |  |  |  |  |  |  |  |  |  |  |
| CONSENSUS | S | G | S | G | S | G | - | - | T | E | F | T | L | T | I | S | S | L | Q | S | E | D | F | A | V | Y |
| 21-225_162A10_LC | N | . | . | . | . | . | . | . | . | . | . | . | . | . | . | . | . | . | . | . | . | . | . | . | . | . |
| 21-225_193G12_LC | . | . | . | . | . | . | . | . | . | . | . | . | . | . | . | . | . | . | . | . | A | . | . | . | . | . |
| 21-225_201A4_LC | . | . | . | . | . | . | . | . | . | . | . | . | . | . | . | . | . | . | . | . | . | . | . | . | . | . |
| 21-225_201F2_LC | . | . | . | . | . | . | . | . | . | . | . | . | . | . | . | . | . | . | . | . | . | . | . | . | . | . |
| 21-225_201F7_LC | . | . | . | . | . | . | . | . | . | . | . | . | . | . | . | . | . | . | . | . | . | . | . | . | . | . |
| 21-225_201H4_LC | . | . | . | . | . | . | . | . | . | . | . | . | . | . | . | . | . | . | . | . | . | . | . | . | . | . |
| 21-225_202A8_LC | . | . | . | . | . | . | . | . | . | . | . | . | . | . | . | . | . | . | . | . | . | . | . | T | . | . |
| 21-225_202F12_LC | . | . | . | . | . | . | . | . | . | . | . | . | . | . | . | . | . | . | . | . | . | . | . | . | . | . |
| 21-225_205G4_LC | . | . | . | . | . | . | . | . | . | . | . | . | . | . | . | . | . | . | . | . | . | . | . | . | . | . |
| 21-225_210H10_LC | . | . | . | . | . | . | . | . | . | . | . | . | . | . | . | . | . | . | . | . | . | . | . | . | . | . |
| 21-225_212C10_LC | . | . | F | . | . | . | . | . | S | G | . | . | . | . | . | . | . | . | . | . | . | . | . | . | . | . |
| 21-225_216B10_LC | . | . | . | . | . | . | . | . | . | . | . | . | . | S | . | . | . | . | . | . | . | . | . | . | . | . |
| 21-225_24H3_LC | . | . | . | . | . | . | . | . | . | . | . | . | . | . | . | . | . | . | . | . | . | . | . | . | . | . |
| 21-225_57H3_LC | N | . | . | . | . | W | . | . | . | . | . | . | . | . | . | . | . | . | . | . | . | . | . | . | I | . |
| 21-225_62E6_LC | N | . | . | . | . | . | . | . | . | . | . | . | . | . | . | . | . | . | . | . | . | . | . | . | . | . |
| 21-225_65A6_LC | N | . | . | . | . | . | . | . | . | . | . | . | . | . | . | . | . | . | . | . | . | . | . | . | . | . |
| 21-225_67C3_LC | N | . | . | . | . | . | . | . | . | . | . | . | . | . | . | . | . | . | . | . | . | . | . | . | . | . |
| 21-225_70A5_LC | N | . | . | . | . | . | . | . | . | . | . | . | . | . | . | . | . | . | . | . | . | . | . | . | . | . |
| 21-225_74C3_LC | . | . | . | . | . | . | . | . | . | . | . | . | . | . | I | . | . | . | . | . | . | . | . | . | . | . |
| 21-225_74E11_LC | . | . | . | . | . | . | . | . | . | . | . | . | . | . | . | . | . | . | . | . | . | . | . | . | I | . |
| 21-225_74E3_LC | . | . | . | . | . | . | . | . | . | . | . | . | . | . | . | . | . | . | . | . | . | . | . | . | I | . |
| 21-225_76D2_LC | . | . | . | . | . | . | . | . | . | . | . | . | . | . | . | . | . | V | . | . | . | . | . | . | I | . |
| 21-225_77H5_LC | . | . | . | . | . | . | . | . | . | . | . | S | P | . | . | . | . | . | . | . | . | . | . | . | . | . |
| 21-225_92H5_LC | . | . | . | . | . | . | . | . | . | . | . | . | . | . | . | T | . | . | . | . | . | . | . | . | L | . |
| 21-225_95G2_LC | . | . | . | . | . | . | . | . | . | . | . | . | . | . | . | . | . | . | . | . | . | . | . | . | I | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | N | D | - | - | - | - | - | - | - | - | - | - | - | - | - | - | - | - | - | - | - |
| 21-225_162A10_LC | S | . | . | . | . | . | . | . | . | . | . | . | . | . | . | . | . | . | . | . | . | . | . | . | . | . |
| 21-225_193G12_LC | . | . | . | . | . | . | N | . | . | . | . | . | . | . | . | . | . | . | . | . | . | . | . | . | . | . |
| 21-225_201A4_LC | . | . | . | . | S | Y | N | . | . | . | . | . | . | . | . | . | . | . | . | . | . | . | . | . | . | . |
| 21-225_201F2_LC | . | . | . | . | S | Y | N | . | . | . | . | . | . | . | . | . | . | . | . | . | . | . | . | . | . | . |
| 21-225_201F7_LC | . | . | . | . | S | Y | N | . | . | . | . | . | . | . | . | . | . | . | . | . | . | . | . | . | . | . |
| 21-225_201H4_LC | . | . | . | E | . | . | . | . | . | . | . | . | . | . | . | . | . | . | . | . | . | . | . | . | . | . |
| 21-225_202A8_LC | . | . | . | . | . | Y | N | . | . | . | . | . | . | . | . | . | . | . | . | . | . | . | . | . | . | . |
| 21-225_202F12_LC | . | . | . | . | . | D | N | . | . | . | . | . | . | . | . | . | . | . | . | . | . | . | . | . | . | . |
| 21-225_205G4_LC | . | . | . | . | S | Y | N | . | . | . | . | . | . | . | . | . | . | . | . | . | . | . | . | . | . | . |
| 21-225_210H10_LC | S | . | . | . | . | Y | N | . | . | . | . | . | . | . | . | . | . | . | . | . | . | . | . | . | . | . |
| 21-225_212C10_LC | S | . | . | . | . | . | . | . | . | . | . | . | . | . | . | . | . | . | . | . | . | . | . | . | . | . |
| 21-225_216B10_LC | . | . | . | . | . | . | . | . | . | . | . | . | . | . | . | . | . | . | . | . | . | . | . | . | . | . |
| 21-225_24H3_LC | . | . | . | . | . | . | N | . | . | . | . | . | . | . | . | . | . | . | . | . | . | . | . | . | . | . |
| 21-225_57H3_LC | . | . | . | . | . | . | . | . | . | . | . | . | . | . | . | . | . | . | . | . | . | . | . | . | . | . |
| 21-225_62E6_LC | . | . | . | . | . | . | . | . | . | . | . | . | . | . | . | . | . | . | . | . | . | . | . | . | . | . |
| 21-225_65A6_LC | . | . | . | . | . | . | . | . | . | . | . | . | . | . | . | . | . | . | . | . | . | . | . | . | . | . |
| 21-225_67C3_LC | . | . | . | . | . | . | . | . | . | . | . | . | . | . | . | . | . | . | . | . | . | . | . | . | . | . |
| 21-225_70A5_LC | . | . | . | . | . | . | . | . | . | . | . | . | . | . | . | . | . | . | . | . | . | . | . | . | . | . |
| 21-225_74C3_LC | . | . | . | . | . | . | . | . | . | . | . | . | . | . | . | . | . | . | . | . | . | . | . | . | . | . |
| 21-225_74E11_LC | . | . | . | . | . | . | . | . | . | . | . | . | . | . | . | . | . | . | . | . | . | . | . | . | . | . |
| 21-225_74E3_LC | . | . | . | . | . | . | . | . | . | . | . | . | . | . | . | . | . | . | . | . | . | . | . | . | . | . |
| 21-225_76D2_LC | . | . | . | . | . | . | . | . | . | . | . | . | . | . | . | . | . | . | . | . | . | . | . | . | . | . |
| 21-225_77H5_LC | S | . | . | . | . | . | . | . | . | . | . | . | . | . | . | . | . | . | . | . | . | . | . | . | . | . |
| 21-225_92H5_LC | . | . | . | E | . | . | . | . | . | . | . | . | . | . | . | . | . | . | . | . | . | . | . | . | . | . |
| 21-225_95G2_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | W | P | C | S | F | G | Q | G | T | K | L | E | I | K | R |
| 21-225_162A10_LC | . | . | . | . | . | . | . | . | . | . | . | . | . | . | . | . | . | . | . |
| 21-225_193G12_LC | . | . | . | . | . | . | . | . | . | . | . | . | . | . | . | . | . | . | T |
| 21-225_201A4_LC | . | . | . | . | . | L | . | . | . | . | . | . | . | . | . | . | L | . | . |
| 21-225_201F2_LC | . | . | . | . | . | L | . | . | . | . | . | . | . | . | . | . | L | . | . |
| 21-225_201F7_LC | . | . | . | . | . | L | . | . | . | . | . | . | . | . | . | . | L | . | . |
| 21-225_201H4_LC | . | . | . | . | . | . | . | . | . | . | . | . | . | N | . | . | . | . | . |
| 21-225_202A8_LC | . | . | . | . | . | L | . | . | . | . | . | . | . | . | . | . | . | X | . |
| 21-225_202F12_LC | . | . | . | W | P | . | . | . | . | . | . | . | . | . | . | . | . | . | . |
| 21-225_205G4_LC | . | . | . | . | . | L | . | . | . | . | . | . | . | . | . | . | L | . | . |
| 21-225_210H10_LC | . | . | . | . | . | L | . | . | . | . | . | . | . | . | . | . | . | . | . |
| 21-225_212C10_LC | . | . | . | . | . | . | . | . | . | . | . | . | . | . | . | . | . | . | . |
| 21-225_216B10_LC | . | . | . | . | . | . | . | . | . | . | . | . | . | . | . | . | . | X | . |
| 21-225_24H3_LC | . | . | . | W | P | M | . | . | . | . | . | . | . | . | . | . | . | . | . |
| 21-225_57H3_LC | . | . | . | . | . | . | . | . | . | . | . | . | . | . | . | . | . | . | . |
| 21-225_62E6_LC | . | . | . | . | . | . | . | . | . | . | . | . | . | . | . | . | . | . | . |
| 21-225_65A6_LC | . | . | . | . | . | . | . | . | . | . | L | E | . | . | . | . | . | . | . |
| 21-225_67C3_LC | . | . | . | . | . | . | . | . | . | . | . | . | . | . | . | . | . | . | . |
| 21-225_70A5_LC | . | . | . | . | . | . | . | . | . | . | . | . | . | . | . | . | . | . | . |
| 21-225_74C3_LC | . | . | . | . | . | . | . | . | . | . | L | . | . | . | . | . | . | . | . |
| 21-225_74E11_LC | . | . | . | . | . | . | . | . | . | . | . | . | . | . | . | . | . | . | . |
| 21-225_74E3_LC | . | . | . | . | . | . | . | . | . | . | . | . | . | . | . | . | . | . | . |
| 21-225_76D2_LC | . | . | . | . | . | . | . | . | . | . | . | . | . | . | . | . | . | . | . |
| 21-225_77H5_LC | . | . | . | . | . | . | . | . | . | . | . | . | S | . | . | . | . | Q | . |
| 21-225_92H5_LC | . | . | . | . | . | . | . | . | . | . | . | . | . | . | . | . | . | . | . |
| 21-225_95G2_LC | . | . | . | . | . | . | . | . | . | . | . | . | . | . | . | . | . | . | . |

FIGURE 57

Table 112. Consensus 32 - VK3|A27/JK4 (SEQ ID NO: 50329):

EFMLTQSPGTLSLSPGERATLSCRAS--QSVSS-----SYLVWYQQKPGQAPRLLIYG--------ASTRATGIPDRFSGSGSG--
TDFTLTISRLEPEYFAVYYCQQYGC----------------------SPLTFGGGTKVEITR wherein:

A at position 25 can be substituted with S

Q at position 29 can be substituted with E

S at position 30 can be substituted with R

V at position 31 can be substituted with I

S at position 32 can be substituted with T

S at position 33 can be substituted with T

S at position 39 can be substituted with N

Y at position 40 can be substituted with A

V at position 42 can be substituted with S

T at position 69 can be substituted with S

T at position 72 can be substituted with S or I

G at position 110 can be substituted with V

C at position 111 can be substituted with N or S

P at position 136 can be substituted with L

Arg Xaa Ser Xaa Xaa Xaa Xaa Xaa Xaa Xaa Leu Xaa (SEQ ID NO: 50171)

Gly Ala Ser Xaa Arg Ala Xaa (SEQ ID NO: 50172)

**FIGURE 57**

Gln Gln Tyr Xaa Xaa Ser Xaa Leu Thr (SEQ ID NO: 50173)

Arg Ala Ser Gln Ser Val Ser Ser Ser Tyr Leu Val (SEQ ID NO: 50657)

Gly Ala Ser Thr Arg Ala Thr (SEQ ID NO: 50658)

Gln Gln Tyr Gly Cys Ser Pro Leu Thr (SEQ ID NO: 50659)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | E | F | M | L | T | Q | S | P | G | T | L | S | L | S | P | G | E | R | A | T | L | S | C | R | A | S |
| 21-225_211G3_LC | . | I | V | . | . | . | . | . | . | . | . | . | . | . | . | . | . | . | . | . | . | . | . | . | . | . |
| 21-225_215C7_LC | . | I | V | . | . | . | . | . | . | . | . | . | . | . | . | . | . | . | V | . | . | . | . | . | . | . |
| 21-225_215H12_LC | . | I | V | . | . | . | . | . | . | . | . | . | . | . | . | . | . | . | . | . | . | . | . | . | . | . |
| 21-225_74A5_LC | . | . | . | . | . | . | . | . | . | . | . | . | . | . | . | . | . | . | . | . | . | . | . | . | . | . |
| 21-225_74B6_LC | . | I | V | . | . | . | . | . | . | . | . | . | . | . | . | . | . | . | . | . | . | . | . | . | . | . |
| 21-225_74G3_LC | . | . | . | . | . | . | . | . | . | . | . | C | W | . | . | . | . | . | . | . | . | . | . | . | . | . |
| 21-225_74G9_LC | . | . | . | . | . | . | . | . | . | . | . | . | . | . | . | . | . | . | . | . | . | . | . | . | . | . |
| 21-225_75B1_LC | . | . | . | . | . | . | . | . | . | . | . | Y | W | . | . | . | . | . | . | . | I | . | S | . | . | . |
| 21-225_76A6_LC | . | I | V | . | . | . | . | . | . | . | X | . | W | . | . | . | . | . | . | . | . | . | . | . | . | . |
| 21-225_76B9_LC | . | . | . | . | . | . | . | . | . | . | . | . | W | . | . | . | . | . | . | . | . | . | . | . | . | . |
| 21-225_76H1_LC | . | . | . | . | . | . | . | . | . | . | . | Y | W | . | . | . | . | . | . | . | . | . | . | . | . | . |
| 21-225_77D11_LC | . | . | . | . | . | . | . | . | . | . | . | Y | W | . | . | . | . | . | . | . | I | . | . | . | . | . |
| 21-225_80A2_LC | . | . | . | . | . | . | . | . | . | . | . | Y | . | . | . | . | . | . | . | . | . | . | . | . | . | . |
| 21-225_80C12_LC | . | I | V | . | . | . | . | . | . | . | . | Y | . | . | . | . | . | . | . | . | . | . | . | . | . | . |
| 21-225_82A5_LC | . | I | V | . | . | . | . | . | . | . | . | . | W | . | . | . | . | . | . | . | . | . | . | . | . | . |
| 21-225_82C12_LC | . | I | V | . | . | . | . | . | . | . | . | . | W | . | . | . | . | . | . | . | . | . | . | . | . | . |
| 21-225_82D9_LC | . | . | . | . | . | . | . | . | . | . | . | . | W | . | . | . | . | . | . | . | . | . | . | . | . | . |
| 21-225_82G5_LC | . | . | . | . | . | . | . | . | . | . | . | Y | W | . | . | . | . | . | . | . | . | . | . | S | . | . |
| 21-225_83C2_LC | . | . | . | . | . | . | . | . | . | . | . | C | . | . | . | . | . | . | . | . | . | . | . | . | . | . |
| 21-225_83C5_LC | . | . | . | . | . | . | . | . | . | . | . | C | W | . | . | . | . | . | . | . | . | . | . | . | . | . |
| 21-225_85B5_LC | . | . | . | . | . | . | . | . | . | . | . | . | . | . | . | . | . | . | . | . | . | . | . | . | S | . |
| 21-225_85D11_LC | . | . | . | . | . | . | . | . | . | . | . | . | . | T | . | . | . | . | . | . | . | . | . | . | S | . |
| 21-225_87A10_LC | . | . | V | . | . | . | . | . | . | . | . | Y | . | . | . | . | . | . | . | . | . | . | . | . | . | . |
| 21-225_96G1_LC | . | I | V | . | . | . | . | . | . | . | . | . | . | T | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | V | S | S | - | - | - | - | - | S | Y | L | V | W | Y | Q | Q | K | P | G | Q | A | P |
| 21-225_211G3_LC | . | . | . | . | . | . | . | . | . | . | . | . | . | P | . | A | . | . | . | . | . | . | . | . | . | . |
| 21-225_215C7_LC | . | . | . | S | I | T | T | . | . | . | . | . | N | P | . | A | . | . | . | . | . | . | . | . | . | . |
| 21-225_215H12_LC | . | . | . | . | . | . | . | . | . | . | . | . | . | P | . | A | . | . | . | . | . | . | . | . | . | . |
| 21-225_74A5_LC | . | . | . | . | . | . | . | . | . | . | . | . | . | . | . | . | . | . | . | . | . | . | . | . | . | . |
| 21-225_74B6_LC | . | . | . | . | . | . | . | . | . | . | . | . | . | . | . | A | . | . | . | . | . | . | . | . | . | . |
| 21-225_74G3_LC | . | . | . | . | . | . | . | . | . | . | . | . | . | . | . | . | . | . | . | . | . | . | . | . | . | . |
| 21-225_74G9_LC | . | . | . | . | . | . | . | . | . | . | . | . | . | . | . | . | . | . | . | . | . | . | . | . | . | . |
| 21-225_75B1_LC | . | . | . | . | . | . | . | . | . | . | . | . | . | . | . | . | . | . | . | . | . | . | . | . | . | . |
| 21-225_76A6_LC | . | . | . | . | . | . | . | . | . | . | . | . | . | . | . | A | . | . | . | . | . | . | . | . | . | . |
| 21-225_76B9_LC | . | . | . | . | . | . | . | . | . | . | . | . | . | . | . | . | . | . | . | . | . | . | . | . | . | . |
| 21-225_76H1_LC | . | . | . | . | . | . | . | . | . | . | . | . | . | . | . | . | . | . | . | . | . | . | . | . | . | . |
| 21-225_77D11_LC | . | . | . | . | . | . | . | . | . | . | . | . | . | . | . | . | . | . | . | . | . | . | . | . | . | . |
| 21-225_80A2_LC | . | . | . | . | . | . | . | . | . | . | . | . | . | . | . | . | . | . | . | . | . | . | . | . | . | . |
| 21-225_80C12_LC | . | . | . | . | . | . | . | . | . | . | . | . | . | . | . | A | . | . | . | . | . | . | . | . | . | . |
| 21-225_82A5_LC | . | . | . | . | . | . | . | . | . | . | . | . | . | . | . | A | . | . | . | . | . | . | . | . | . | . |
| 21-225_82C12_LC | . | . | . | . | . | . | . | . | . | . | . | . | . | . | . | A | . | . | . | . | . | . | . | . | . | . |
| 21-225_82D9_LC | . | . | E | . | . | . | . | . | . | . | . | . | . | . | . | . | . | . | . | . | . | . | . | . | . | . |
| 21-225_82G6_LC | . | . | E | . | . | . | . | . | . | . | . | . | . | . | . | . | . | . | . | . | . | . | . | . | . | . |
| 21-225_83C2_LC | . | . | . | . | . | . | . | . | . | . | . | . | . | . | . | . | . | . | . | . | . | . | . | . | . | . |
| 21-225_83C5_LC | . | . | E | . | . | . | . | . | . | . | . | . | . | . | . | . | . | . | . | . | . | . | . | . | . | . |
| 21-225_85B5_LC | . | . | . | . | . | . | . | . | . | . | . | . | . | . | . | . | . | . | . | . | . | . | . | . | . | . |
| 21-225_85D11_LC | . | . | . | . | . | . | . | . | . | . | . | . | . | . | . | . | . | . | . | . | . | . | . | . | . | . |
| 21-225_87A10_LC | . | . | . | . | . | . | . | . | . | . | . | . | . | . | . | A | . | . | . | . | . | . | . | . | . | . |
| 21-225_96G1_LC | . | . | . | . | . | . | . | . | . | . | . | . | . | . | . | A | . | . | . | . | . | . | . | . | P | . |

4918

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | R | L | L | I | Y | G | - | - | - | - | - | - | - | - | A | S | T | R | A | T | G | I | P | D | R | F |
| 21-225_211G3_LC | . | . | . | . | . | . | . | . | . | . | . | . | . | . | . | . | S | . | . | . | . | . | . | . | . | . |
| 21-225_215C7_LC | . | . | . | . | . | . | . | . | . | . | . | . | . | . | . | . | S | . | . | . | . | . | . | . | . | . |
| 21-225_215H12_LC | . | . | M | . | . | . | . | . | . | . | . | . | . | . | . | . | S | . | . | I | . | . | . | . | . | . |
| 21-225_74A5_LC | . | . | . | . | . | . | . | . | . | . | . | . | . | . | . | . | . | . | . | . | . | . | . | . | . | . |
| 21-225_74B6_LC | . | . | . | . | . | . | . | . | . | . | . | . | . | . | . | . | . | . | . | . | . | . | . | . | . | . |
| 21-225_74G3_LC | . | . | . | . | . | . | . | . | . | . | . | . | . | . | . | . | . | . | . | . | . | . | . | . | . | . |
| 21-225_74G9_LC | . | . | . | . | . | . | . | . | . | . | . | . | . | . | . | . | . | . | . | . | . | . | . | . | . | . |
| 21-225_75B1_LC | . | . | . | . | . | . | . | . | . | . | . | . | . | . | . | . | . | . | . | . | . | . | . | . | . | . |
| 21-225_76A6_LC | . | . | . | . | . | . | . | . | . | . | . | . | . | . | . | . | . | . | . | . | . | . | . | . | . | . |
| 21-225_76B9_LC | . | . | . | . | . | . | . | . | . | . | . | . | . | . | . | . | . | . | . | . | . | . | . | . | . | . |
| 21-225_76H1_LC | . | . | . | . | . | . | . | . | . | . | . | . | . | . | . | . | . | . | . | . | . | . | . | . | . | . |
| 21-225_77D11_LC | . | . | . | . | . | . | . | . | . | . | . | . | . | . | . | . | . | . | . | . | . | . | . | . | . | . |
| 21-225_80A2_LC | . | . | . | . | . | . | . | . | . | . | . | . | . | . | . | . | . | . | . | . | . | . | . | . | . | . |
| 21-225_80C12_LC | . | . | . | . | . | . | . | . | . | . | . | . | . | . | . | . | . | . | . | . | . | . | . | . | . | . |
| 21-225_82A5_LC | . | . | . | . | . | . | . | . | . | . | . | . | . | . | . | . | . | . | . | . | . | . | . | . | . | . |
| 21-225_82C12_LC | . | . | . | . | . | . | . | . | . | . | . | . | . | . | . | . | . | . | . | S | . | . | . | . | . | . |
| 21-225_82D9_LC | . | . | . | . | . | . | . | . | . | . | . | . | . | . | . | . | . | . | . | . | . | . | . | . | . | . |
| 21-225_82G5_LC | . | . | . | . | . | . | . | . | . | . | . | . | . | . | . | . | . | . | . | . | . | . | . | . | . | . |
| 21-225_83C2_LC | . | . | . | . | . | . | . | . | . | . | . | . | . | . | . | . | . | . | . | . | . | . | . | . | . | . |
| 21-225_83C5_LC | . | . | . | . | . | . | . | . | . | . | . | . | . | . | . | . | . | . | . | . | . | . | . | . | . | . |
| 21-225_85B5_LC | . | . | . | . | . | . | . | . | . | . | . | . | . | . | . | . | . | . | . | . | . | . | . | . | . | . |
| 21-225_85D11_LC | . | . | . | . | . | . | . | . | . | . | . | . | . | . | . | . | . | . | . | . | . | . | . | . | . | . |
| 21-225_87A10_LC | . | . | . | . | . | . | . | . | . | . | . | . | . | . | . | . | . | . | . | S | . | . | . | . | . | . |
| 21-225_96G1_LC | . | . | . | . | . | . | . | . | . | . | . | . | . | . | . | . | . | . | . | S | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | R | L | E | P | E | Y | F | A | V | Y |
| 21-225_211G3_LC | . | . | . | . | . | . | . | . | . | . | . | . | . | . | . | . | . | . | . | . | . | D | . | . | . | . |
| 21-225_215C7_LC | . | . | . | . | . | . | . | . | . | . | . | . | . | . | . | . | . | . | . | S | . | D | . | . | . | . |
| 21-225_215H12_LC | . | . | . | . | . | . | . | . | . | . | . | . | . | . | . | . | . | . | . | . | . | D | . | . | . | . |
| 21-225_74A5_LC | . | . | . | . | . | . | . | . | . | . | . | . | . | . | . | . | . | . | . | . | . | . | . | . | . | . |
| 21-225_74B6_LC | . | . | . | . | . | . | . | . | . | . | . | . | . | . | . | . | . | . | . | . | . | . | . | . | . | . |
| 21-225_74G3_LC | . | . | . | . | . | . | . | . | . | . | . | . | . | . | . | . | . | . | . | . | . | . | . | . | . | . |
| 21-225_74G9_LC | . | . | . | . | . | . | . | . | . | . | . | . | . | . | . | . | . | . | . | . | . | . | . | . | . | . |
| 21-225_75B1_LC | . | . | . | . | . | . | . | . | . | . | . | . | . | . | . | . | . | . | . | . | . | . | . | . | . | . |
| 21-225_76A6_LC | . | . | . | . | . | . | . | . | . | . | . | . | . | . | . | . | . | . | . | . | . | . | . | . | . | . |
| 21-225_76B9_LC | . | . | . | . | . | . | . | . | . | . | . | . | . | . | . | . | . | . | . | . | . | . | . | . | . | . |
| 21-225_76H1_LC | . | . | . | . | . | . | . | . | . | . | . | . | . | . | . | . | . | . | . | . | . | . | . | . | . | . |
| 21-225_77D11_LC | . | . | . | . | . | . | . | . | . | . | . | . | . | . | . | . | . | . | . | . | . | . | . | . | . | . |
| 21-225_80A2_LC | . | . | . | . | . | . | . | . | . | . | . | . | . | . | . | . | . | . | . | . | . | . | . | . | . | . |
| 21-225_80C12_LC | . | . | . | . | . | . | . | . | . | . | . | . | . | . | . | . | . | . | . | . | . | . | . | . | . | . |
| 21-225_82A5_LC | . | . | . | . | . | . | . | . | . | . | . | . | . | . | . | . | . | . | . | . | . | . | . | . | . | . |
| 21-225_82C12_LC | . | . | . | . | . | . | . | . | . | . | . | . | . | . | . | . | . | . | . | . | . | . | . | . | . | . |
| 21-225_82D9_LC | . | . | . | . | . | . | . | . | . | . | . | . | . | . | . | . | . | . | . | . | . | . | . | . | . | . |
| 21-225_82G5_LC | . | . | . | . | . | . | . | . | . | . | . | . | . | . | . | . | . | . | . | . | . | . | . | . | . | . |
| 21-225_83C2_LC | . | . | . | . | . | . | . | . | . | . | . | . | . | . | . | . | . | . | . | . | . | . | . | . | . | . |
| 21-225_83C5_LC | . | . | . | . | . | . | . | . | . | . | . | . | . | . | . | . | . | . | . | . | . | . | . | . | . | . |
| 21-225_85B5_LC | . | . | . | . | . | . | . | . | . | . | . | . | . | . | . | . | . | . | . | . | . | . | . | . | . | . |
| 21-225_85D11_LC | . | . | . | . | . | . | . | . | . | . | . | . | . | . | . | . | . | . | . | . | . | . | . | . | . | . |
| 21-225_87A10_LC | . | . | . | . | . | . | . | . | . | . | . | . | . | . | . | . | . | . | . | . | . | . | . | . | . | . |
| 21-225_96G1_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | G | C | - | - | - | - | - | - | - | - | - | - | - | - | - | - | - | - | - | - | - |
| 21-225_211G3_LC | . | . | . | . | . | V | S | . | . | . | . | . | . | . | . | . | . | . | . | . | . | . | . | . | | |
| 21-225_215C7_LC | . | . | . | . | . | V | S | . | . | . | . | . | . | . | . | . | . | . | . | . | . | . | . | . | | |
| 21-225_215H12_LC | . | . | . | . | . | V | S | . | . | . | . | . | . | . | . | . | . | . | . | . | . | . | . | . | | |
| 21-225_74A5_LC | . | . | . | . | . | . | . | . | . | . | . | . | . | . | . | . | . | . | . | . | . | . | . | . | | |
| 21-225_74B6_LC | . | . | . | . | . | . | N | . | . | . | . | . | . | . | . | . | . | . | . | . | . | . | . | . | | |
| 21-225_74G3_LC | . | . | . | . | . | . | . | . | . | . | . | . | . | . | . | . | . | . | . | . | . | . | . | . | | |
| 21-225_74G9_LC | . | . | . | . | . | . | . | . | . | . | . | . | . | . | . | . | . | . | . | . | . | . | . | . | | |
| 21-225_75B1_LC | . | . | . | . | . | . | . | . | . | . | . | . | . | . | . | . | . | . | . | . | . | . | . | . | | |
| 21-225_76A6_LC | . | . | . | . | . | . | N | . | . | . | . | . | . | . | . | . | . | . | . | . | . | . | . | . | | |
| 21-225_76B9_LC | . | . | . | . | . | . | . | . | . | . | . | . | . | . | . | . | . | . | . | . | . | . | . | . | | |
| 21-225_76H1_LC | . | . | . | . | . | . | . | . | . | . | . | . | . | . | . | . | . | . | . | . | . | . | . | . | | |
| 21-225_77D11_LC | . | . | . | . | . | . | . | . | . | . | . | . | . | . | . | . | . | . | . | . | . | . | . | . | | |
| 21-225_80A2_LC | . | . | . | . | . | . | . | . | . | . | . | . | . | . | . | . | . | . | . | . | . | . | . | . | | |
| 21-225_80C12_LC | . | . | . | . | . | . | N | . | . | . | . | . | . | . | . | . | . | . | . | . | . | . | . | . | | |
| 21-225_82A5_LC | . | . | . | . | . | . | N | . | . | . | . | . | . | . | . | . | . | . | . | . | . | . | . | . | | |
| 21-225_82C12_LC | . | . | . | . | . | . | N | . | . | . | . | . | . | . | . | . | . | . | . | . | . | . | . | . | | |
| 21-225_82D9_LC | . | . | . | . | . | . | . | . | . | . | . | . | . | . | . | . | . | . | . | . | . | . | . | . | | |
| 21-225_82G5_LC | . | . | . | . | . | . | . | . | . | . | . | . | . | . | . | . | . | . | . | . | . | . | . | . | | |
| 21-225_83C2_LC | . | . | . | . | . | . | . | . | . | . | . | . | . | . | . | . | . | . | . | . | . | . | . | . | | |
| 21-225_83C5_LC | . | . | . | . | . | . | . | . | . | . | . | . | . | . | . | . | . | . | . | . | . | . | . | . | | |
| 21-225_85B5_LC | . | . | . | . | . | . | . | . | . | . | . | . | . | . | . | . | . | . | . | . | . | . | . | . | | |
| 21-225_85D11_LC | . | . | . | . | . | . | . | . | . | . | . | . | . | . | . | . | . | . | . | . | . | . | . | . | | |
| 21-225_87A10_LC | . | . | . | . | . | . | N | . | . | . | . | . | . | . | . | . | . | . | . | . | . | . | . | . | | |
| 21-225_96G1_LC | . | . | . | . | . | . | N | . | . | . | . | . | . | . | . | . | . | . | . | . | . | . | . | . | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | S | P | L | T | F | G | G | G | T | K | V | E | I | T | R |
| 21-225_211G3_LC | . | . | . | . | . | . | . | . | . | . | . | . | . | . | . | . | . | . | . |
| 21-225_215C7_LC | . | . | . | . | . | . | . | . | . | . | . | . | . | . | . | . | . | . | . |
| 21-225_215H12_LC | . | . | . | . | . | . | . | . | . | . | . | . | . | . | . | . | . | . | . |
| 21-225_74A5_LC | . | . | . | . | . | . | . | . | . | . | . | . | . | . | . | . | . | . | . |
| 21-225_74B6_LC | . | . | . | . | . | . | . | . | . | . | . | . | . | . | . | . | . | . | . |
| 21-225_74G3_LC | . | . | . | . | . | . | . | . | . | . | . | . | . | . | . | . | . | . | . |
| 21-225_74G9_LC | . | . | . | . | . | . | . | . | . | . | . | . | . | . | . | . | . | . | . |
| 21-225_75B1_LC | . | . | . | . | . | . | . | . | . | . | . | . | . | . | . | . | . | . | . |
| 21-225_76A6_LC | . | . | . | . | . | . | . | . | . | . | . | . | . | . | . | . | . | . | . |
| 21-225_76B9_LC | . | . | . | . | . | . | . | . | . | . | . | . | . | . | . | . | . | . | . |
| 21-225_76H1_LC | . | . | . | . | . | . | . | . | . | . | . | . | . | . | . | . | . | . | . |
| 21-225_77D11_LC | . | . | . | . | . | . | . | . | . | . | . | . | . | . | . | . | . | . | . |
| 21-225_80A2_LC | . | . | . | . | . | . | . | . | . | . | . | . | . | . | . | . | . | . | . |
| 21-225_80C12_LC | . | . | . | . | . | . | . | . | . | . | . | . | . | . | . | . | . | . | . |
| 21-225_82A5_LC | . | . | . | . | . | . | . | . | . | . | . | . | . | . | . | . | . | . | . |
| 21-225_82C12_LC | . | . | . | . | . | . | . | . | . | . | . | . | . | . | . | . | . | . | . |
| 21-225_82D9_LC | . | . | . | . | . | . | . | . | . | . | . | . | . | . | . | . | . | . | . |
| 21-225_82G5_LC | . | . | . | . | . | . | . | . | . | . | . | . | . | . | . | . | . | . | . |
| 21-225_83C2_LC | . | . | . | . | . | . | . | . | . | . | . | . | . | . | . | . | . | . | . |
| 21-225_83C5_LC | . | . | . | . | . | . | . | . | . | . | . | . | . | . | . | . | . | . | . |
| 21-225_85B5_LC | . | . | . | . | . | . | . | . | . | . | . | . | . | . | . | . | . | . | . |
| 21-225_85D11_LC | . | . | . | . | . | . | . | . | . | . | . | . | . | . | . | . | . | . | . |
| 21-225_87A10_LC | . | . | . | . | . | . | . | . | . | . | . | . | . | . | . | . | . | . | . |
| 21-225_96G1_LC | . | . | . | . | . | . | . | . | . | . | . | . | . | . | . | . | . | . | . |

**FIGURE 57**

Table 113.    Consensus 33 - VK2|A18/JK4 (SEQ ID NO: 50330):

DIVMTQTPLSLSVTPGQPASISCKSS--QSLLHSE-GKTYLYWYLQKPGQPPQLLIYE--------VSNRFSGVPDRFSGSGSG--
TDFTLKISRVEAEDVGVYYCMQSIQ---------------------LPLTFGGGTKVEIKR wherein:

S at position 30 can be substituted with T

L at position 32 can be substituted with Q

H at position 33 can be substituted with R

S at position 34 can be substituted with G

E at position 35 can be substituted with D

K at position 38 can be substituted with R

Y at position 40 can be substituted with H or F

Y at position 42 can be substituted with N

V at position 67 can be substituted with I

N at position 69 can be substituted with Y or H

F at position 71 can be substituted with I, V or L

M at position 107 can be substituted with F

Q at position 108 can be substituted with H

S at position 109 can be substituted with G or N

I at position 110 can be substituted with K or T

Q at position 111 can be substituted with null (-), K or H

FIGURE 57

L at position 135 can be substituted with Q, Y, F or H

P at position 136 can be substituted with L or V

L at position 137 can be substituted with F or P

T at position 138 can be substituted with P or S

Lys Ser Ser Gln Xaa Leu Xaa Xaa Xaa Xaa Gly Xaa Thr Xaa Leu Xaa (SEQ ID NO: 50174)

Glu Xaa Ser Xaa Arg Xaa Ser (SEQ ID NO: 50175)

Xaa Xaa Xaa Xaa Xaa Xaa Xaa Xaa Xaa (SEQ ID NO: 50176)

Lys Ser Ser Gln Ser Leu Leu His Ser Glu Gly Lys Thr Tyr Leu Tyr (SEQ ID NO: 50660)

Glu Val Ser Asn Arg Phe Ser (SEQ ID NO: 50661)

Met Gln Ser Ile Gln Leu Pro Leu Thr (SEQ ID NO: 50662)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | T | P | L | S | L | S | V | T | P | G | Q | P | A | S | I | S | C | K | S | S |
| 21-225_148F5_LC | . | . | . | . | . | . | . | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . | . | . | . | . | . | . | . |
| 21-225_150D11_LC | . | . | . | . | . | . | . | . | . | . | . | . | . | . | . | . | . | . | . | . | . | . | . | . | . | . |
| 21-225_153G9_LC | . | . | . | . | . | . | . | . | . | . | . | . | . | . | . | . | . | . | . | . | . | . | . | . | . | . |
| 21-225_16F10_LC | . | . | . | . | . | . | . | . | . | . | . | . | . | . | . | . | . | . | . | . | . | . | . | . | . | . |
| 21-225_173C11_LC | . | . | . | . | . | . | . | . | . | . | . | . | . | . | . | . | . | . | . | . | . | . | . | . | . | . |
| 21-225_179C2_LC | . | . | . | . | . | . | . | . | . | . | . | . | . | . | . | . | . | . | . | . | . | . | . | . | . | . |
| 21-225_22H8_LC | . | . | . | . | . | . | . | . | . | . | . | . | . | . | . | . | . | . | . | . | . | . | . | . | . | . |
| 21-225_25A2_LC | . | . | . | N | . | . | . | . | . | . | . | . | . | . | . | . | . | . | . | . | . | . | . | . | . | . |
| 21-225_26G11_LC | . | . | . | . | . | . | . | . | . | . | . | . | . | . | . | . | . | . | . | . | . | . | . | . | . | . |
| 21-225_26G7_LC | . | . | L | . | . | . | . | . | . | . | . | . | . | . | . | . | . | . | . | . | . | . | . | . | . | . |
| 21-225_29H4_LC | . | . | . | . | . | . | . | . | . | . | . | . | . | . | . | . | . | . | S | . | . | . | . | . | . | . |
| 21-225_34D2_LC | . | . | . | . | . | . | . | . | . | . | . | . | . | . | . | . | . | . | . | . | . | . | . | . | . | . |
| 21-225_44B10_LC | . | . | . | . | . | . | . | . | . | . | . | . | . | . | . | . | . | . | . | . | . | . | . | . | . | . |
| 21-225_46B9_LC | . | . | . | . | . | . | . | . | . | . | . | . | . | . | . | . | . | . | . | . | . | . | . | . | . | . |
| 21-225_58H11_LC | . | . | . | . | . | . | . | . | . | . | . | . | . | . | . | . | . | . | . | . | . | . | . | . | . | . |
| 21-225_59A12_LC | . | . | . | . | . | . | . | . | . | . | . | . | . | . | . | . | . | . | . | . | . | . | . | . | . | . |
| 21-225_60C12_LC | . | . | . | . | . | . | . | . | . | . | . | . | . | . | . | . | . | . | . | . | . | . | . | . | . | . |
| 21-225_60G2_LC | . | . | . | . | . | . | . | . | . | . | . | . | . | . | . | . | . | S | . | . | . | . | . | . | . | . |
| 21-225_61B3_LC | . | . | . | . | . | . | . | . | . | . | . | . | . | . | . | . | . | . | . | . | . | . | . | . | . | . |
| 21-225_71A6_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | S | L | L | H | S | E | - | G | K | T | Y | L | Y | W | Y | L | Q | K | P | G | Q | P | P |
| 21-225_148F5_LC | . | . | . | . | . | . | . | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . | . | . | . | . | . | . | . |
| 21-225_150D11_LC | . | . | . | . | . | . | . | . | . | . | . | . | . | . | . | . | . | . | . | . | . | . | . | . | . | . |
| 21-225_153G9_LC | . | . | . | . | . | . | . | . | D | . | . | . | . | . | . | . | . | . | . | . | . | . | . | . | . | . |
| 21-225_16F10_LC | . | . | . | . | . | . | . | . | D | . | . | . | . | H | . | N | . | . | . | . | . | . | . | . | . | . |
| 21-225_173C11_LC | . | . | . | . | . | . | . | . | . | . | . | . | . | . | . | . | . | . | . | . | . | . | . | . | . | . |
| 21-225_179C2_LC | . | . | . | . | . | . | . | . | . | . | . | . | . | . | . | . | . | . | . | . | . | . | . | . | . | . |
| 21-225_22H8_LC | . | . | . | . | . | . | . | . | D | . | . | . | . | F | . | . | . | . | . | . | . | . | . | H | . | . |
| 21-225_25A2_LC | . | . | . | . | . | . | . | . | . | . | . | . | . | . | . | . | . | . | . | . | . | . | . | . | . | . |
| 21-225_26G11_LC | . | . | . | T | . | . | . | G | . | . | . | . | . | . | . | . | . | . | . | . | . | . | . | . | . | . |
| 21-225_26G7_LC | . | . | . | . | . | . | . | . | . | . | . | . | . | . | . | . | . | . | . | . | . | . | . | . | . | . |
| 21-225_29H4_LC | . | . | . | . | . | . | . | . | D | . | . | . | . | . | . | . | . | . | . | . | . | . | . | . | . | . |
| 21-225_34D2_LC | . | . | . | . | . | . | . | . | D | . | . | . | . | . | . | . | . | . | . | . | . | . | . | . | . | . |
| 21-225_44B10_LC | . | . | . | . | . | . | . | . | . | . | . | R | . | . | . | . | . | . | . | . | . | . | . | . | . | . |
| 21-225_46B9_LC | . | . | . | . | . | . | . | . | . | . | . | . | . | . | . | . | . | . | . | . | . | . | . | . | . | . |
| 21-225_58H11_LC | . | . | . | . | . | . | . | . | . | . | . | . | . | . | . | . | . | . | V | . | . | . | . | . | . | . |
| 21-225_59A12_LC | . | . | . | . | . | Q | . | G | . | . | . | . | . | . | . | . | . | . | V | . | . | . | . | . | . | . |
| 21-225_60C12_LC | . | . | . | . | . | . | . | . | . | . | . | . | . | . | . | . | . | . | . | . | . | . | . | . | . | . |
| 21-225_60G2_LC | . | . | . | . | . | . | . | . | . | . | . | . | . | . | . | . | . | . | . | . | . | . | . | . | . | . |
| 21-225_61B3_LC | . | . | . | . | . | . | . | . | . | . | . | . | . | . | . | . | . | . | . | . | . | . | . | . | . | . |
| 21-225_71A6_LC | . | . | . | . | . | . | R | . | D | . | . | . | . | H | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | Q | L | L | I | Y | E | - | - | - | - | - | - | - | - | V | S | N | R | F | S | G | V | P | D | R | F |
| 21-225_148F5_LC | . | . | . | . | . | . | . | . | . | . | . | . | . | . | . | . | Y | . | V | . | . | . | . | . | . | . |
| 21-225_148H10_LC | . | . | . | . | . | . | . | . | . | . | . | . | . | . | . | . | Y | . | V | . | . | . | . | . | . | . |
| 21-225_150D11_LC | . | . | . | . | . | . | . | . | . | . | . | . | . | . | . | . | Y | . | . | . | . | . | . | . | . | . |
| 21-225_153G9_LC | . | S | . | . | . | . | . | . | . | . | . | . | . | . | . | . | . | . | . | . | . | . | . | . | . | . |
| 21-225_16F10_LC | . | . | . | . | . | . | . | . | . | . | . | . | . | . | . | . | Y | . | . | . | . | . | . | . | . | . |
| 21-225_173C11_LC | . | . | . | . | . | . | . | . | . | . | . | . | . | . | . | . | S | . | . | . | . | . | . | . | . | . |
| 21-225_179C2_LC | . | . | . | . | . | . | . | . | . | . | . | . | . | . | . | . | S | . | . | . | . | . | . | . | . | . |
| 21-225_22H8_LC | . | . | . | . | . | . | . | . | . | . | . | . | . | . | . | . | . | . | . | . | . | . | . | . | . | . |
| 21-225_25A2_LC | . | . | . | . | . | . | . | . | . | . | . | . | . | . | I | . | . | . | . | . | . | . | . | A | . | L |
| 21-225_26G11_LC | N | . | . | . | . | . | . | . | . | . | . | . | . | . | . | . | . | . | . | . | . | . | . | . | . | . |
| 21-225_26G7_LC | . | S | . | . | . | . | . | . | . | . | . | . | . | . | . | . | S | . | L | . | . | . | . | . | . | . |
| 21-225_29H4_LC | . | . | . | . | S | . | . | . | . | . | . | . | . | . | . | . | S | . | . | . | . | L | . | . | . | . |
| 21-225_34D2_LC | . | . | . | . | . | . | . | . | . | . | . | . | . | . | . | . | . | . | . | . | . | . | . | . | . | . |
| 21-225_44B10_LC | . | . | . | . | . | . | . | . | . | . | . | . | . | . | I | . | S | . | . | . | . | . | . | . | . | . |
| 21-225_46B9_LC | . | . | . | . | . | . | . | . | . | . | . | . | . | . | . | . | Y | . | . | . | . | . | . | . | . | . |
| 21-225_58H11_LC | . | . | . | . | . | . | . | . | . | . | . | . | . | . | . | . | Y | . | . | . | . | . | . | . | . | . |
| 21-225_59A12_LC | . | . | . | . | . | . | . | . | . | . | . | . | . | . | . | . | Y | . | . | . | . | . | . | . | . | . |
| 21-225_60C12_LC | . | S | . | . | . | . | . | . | . | . | . | . | . | . | . | . | . | . | . | . | . | . | . | . | . | . |
| 21-225_60G2_LC | . | . | . | . | . | . | . | . | . | . | . | . | . | . | . | . | . | . | . | I | . | . | . | . | . | . |
| 21-225_61B3_LC | . | . | . | . | . | . | . | . | . | . | . | . | . | . | . | . | . | . | . | I | . | . | . | . | . | . |
| 21-225_71A6_LC | . | . | . | . | . | . | . | . | . | . | . | . | . | . | . | . | . | . | . | . | . | . | S | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | K | I | S | R | V | E | A | E | D | V | G | V | Y |
| 21-225_148F5_LC | . | . | . | . | . | . | . | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . | . | . | . | . | . | . | . |
| 21-225_150D11_LC | . | . | . | . | . | . | . | . | . | . | . | . | . | . | . | . | . | . | . | . | . | . | . | . | . | . |
| 21-225_153G9_LC | . | . | . | . | . | . | . | . | . | . | . | . | . | . | . | . | . | . | . | D | . | . | . | . | . | . |
| 21-225_16F10_LC | . | . | . | . | . | . | . | . | V | . | . | . | . | E | . | . | . | . | . | A | . | . | . | . | . | . |
| 21-225_173C11_LC | . | . | . | . | . | . | . | . | . | . | . | . | . | . | . | . | . | . | . | . | . | . | . | . | . | . |
| 21-225_179C2_LC | . | . | . | . | . | . | . | . | . | . | . | . | . | . | . | . | . | . | . | . | . | . | . | . | . | . |
| 21-225_22H8_LC | . | . | . | . | . | . | . | . | . | . | . | . | . | . | . | . | . | . | . | . | . | . | . | . | I | . |
| 21-225_25A2_LC | . | . | . | . | . | . | . | . | . | . | . | . | . | . | . | . | . | . | . | . | . | . | . | . | . | . |
| 21-225_26G11_LC | . | . | . | . | . | . | . | . | . | . | . | . | . | . | . | . | . | . | . | . | . | . | . | . | I | . |
| 21-225_26G7_LC | . | . | . | . | . | . | . | . | . | . | . | . | . | . | . | . | . | . | . | . | . | . | . | . | . | . |
| 21-225_29H4_LC | . | . | . | . | . | . | . | . | . | . | . | . | . | . | . | . | . | . | . | . | . | . | . | . | . | . |
| 21-225_34D2_LC | . | . | . | . | . | . | . | . | . | . | . | . | . | . | . | . | . | . | . | . | . | . | . | . | . | . |
| 21-225_44B10_LC | . | . | . | . | . | . | . | . | . | . | . | . | . | . | . | . | . | . | . | . | . | . | . | . | . | . |
| 21-225_46B9_LC | . | . | . | . | . | . | . | . | . | . | . | . | . | . | . | . | . | M | . | . | . | . | . | . | . | . |
| 21-225_58H11_LC | . | . | . | . | . | . | . | . | . | . | . | . | . | . | . | . | . | . | . | . | . | . | . | . | . | . |
| 21-225_59A12_LC | . | . | . | . | . | . | . | . | . | . | . | . | . | . | . | . | . | . | V | . | . | . | . | . | . | . |
| 21-225_60C12_LC | . | . | . | . | . | . | . | . | . | . | . | . | . | . | . | . | . | . | . | . | . | . | . | . | I | . |
| 21-225_60G2_LC | . | . | . | . | . | . | . | . | . | . | . | . | . | . | . | . | . | . | . | . | . | . | . | . | . | . |
| 21-225_61B3_LC | . | . | . | . | . | . | . | . | . | . | . | . | . | . | . | . | . | . | . | . | . | . | . | . | . | . |
| 21-225_71A6_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | M | Q | S | I | Q | - | - | - | - | - | - | - | - | - | - | - | - | - | - | - | - | - | - | - |
| 21-225_148F5_LC | * | . | . | . | . | . | . | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . | . | . | . | . | . | . | . |
| 21-225_150D11_LC | . | . | . | . | G | . | . | . | . | . | . | . | . | . | . | . | . | . | . | . | . | . | . | . | . | . |
| 21-225_153G9_LC | . | . | . | . | N | . | * | . | . | . | . | . | . | . | . | . | . | . | . | . | . | . | . | . | . | . |
| 21-225_16F10_LC | . | . | * | . | . | . | . | . | . | . | . | . | . | . | . | . | . | . | . | . | . | . | . | . | . | . |
| 21-225_173C11_LC | . | . | . | . | . | . | - | . | . | . | . | . | . | . | . | . | . | . | . | . | . | . | . | . | . | . |
| 21-225_179C2_LC | . | . | . | . | . | . | - | . | . | . | . | . | . | . | . | . | . | . | . | . | . | . | . | . | . | . |
| 21-225_22H8_LC | . | . | . | . | . | . | . | . | . | . | . | . | . | . | . | . | . | . | . | . | . | . | . | . | . | . |
| 21-225_25A2_LC | . | . | . | . | . | T | . | . | . | . | . | . | . | . | . | . | . | . | . | . | . | . | . | . | . | . |
| 21-225_26G11_LC | . | . | . | . | . | . | * | . | . | . | . | . | . | . | . | . | . | . | . | . | . | . | . | . | . | . |
| 21-225_26G7_LC | . | . | . | . | . | . | . | . | . | . | . | . | . | . | . | . | . | . | . | . | . | . | . | . | . | . |
| 21-225_29H4_LC | . | . | . | . | . | . | . | . | . | . | . | . | . | . | . | . | . | . | . | . | . | . | . | . | . | . |
| 21-225_34D2_LC | . | . | . | . | . | * | - | . | . | . | . | . | . | . | . | . | . | . | . | . | . | . | . | . | . | . |
| 21-225_44B10_LC | . | . | . | . | . | . | * | . | . | . | . | . | . | . | . | . | . | . | . | . | . | . | . | . | . | . |
| 21-225_46B9_LC | . | . | . | * | . | . | . | . | . | . | . | . | . | . | . | . | . | . | . | . | . | . | . | . | . | . |
| 21-225_58H11_LC | . | . | . | . | . | . | . | . | . | . | . | . | . | . | . | . | . | . | . | . | . | . | . | . | . | . |
| 21-225_59A12_LC | . | . | . | . | . | T | . | . | . | . | . | . | . | . | . | . | . | . | . | . | . | . | . | . | . | . |
| 21-225_60C12_LC | . | . | . | . | . | . | * | . | . | . | . | . | . | . | . | . | . | . | . | . | . | . | . | . | . | . |
| 21-225_60G2_LC | . | . | . | . | . | . | . | . | . | . | . | . | . | . | . | . | . | . | . | . | . | . | . | . | . | . |
| 21-225_61B3_LC | . | . | . | . | . | . | . | . | . | . | . | . | . | . | . | . | . | . | . | . | . | . | . | . | . | . |
| 21-225_71A6_LC | . | . | . | . | . | * | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | L | P | L | T | F | G | G | G | T | K | V | E | I | K | R |
| 21-225_148F5_LC | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . |
| 21-225_150D11_LC | . | . | . | . | . | . | . | . | . | . | . | . | . | . | . | . | . | . | . |
| 21-225_153G9_LC | . | . | . | . | Y | . | . | . | . | . | . | . | . | . | . | . | . | . | . |
| 21-225_16F10_LC | . | . | . | . | . | . | . | . | . | . | . | . | . | . | . | . | . | . | . |
| 21-225_173C11_LC | . | . | . | . | Q | L | . | . | . | . | . | . | . | . | . | . | . | . | . |
| 21-225_179C2_LC | . | . | . | . | Q | V | . | . | . | . | . | . | . | . | . | . | . | . | . |
| 21-225_22H8_LC | . | . | . | . | . | . | . | S | . | . | . | . | . | . | . | . | . | N | . |
| 21-225_25A2_LC | . | . | . | . | F | . | . | . | . | . | . | . | . | . | . | . | . | . | . |
| 21-225_26G11_LC | . | . | . | . | . | . | . | . | . | . | . | . | . | . | . | . | . | . | . |
| 21-225_26G7_LC | . | . | . | . | . | . | . | . | . | . | . | . | . | . | . | . | . | . | . |
| 21-225_29H4_LC | . | . | . | . | H | . | P | . | . | . | . | . | . | R | . | . | . | . | . |
| 21-225_34D2_LC | . | . | . | . | Q | L | P | P | . | . | . | . | . | . | . | . | . | R | . |
| 21-225_44B10_LC | . | . | . | . | . | . | P | . | . | . | . | . | . | . | . | . | . | . | . |
| 21-225_46B9_LC | . | . | . | . | Y | . | . | . | . | . | . | . | . | . | . | . | . | Q | . |
| 21-225_58H11_LC | . | . | . | . | . | . | . | . | . | . | . | . | . | . | . | . | . | . | . |
| 21-225_59A12_LC | . | . | . | . | . | . | . | . | . | . | . | . | . | . | . | . | . | . | . |
| 21-225_60C12_LC | . | . | . | . | Y | . | . | . | . | . | . | . | . | . | . | . | . | . | . |
| 21-225_60G2_LC | . | . | . | . | . | . | . | . | . | . | . | . | . | . | . | . | . | . | . |
| 21-225_61B3_LC | . | . | . | . | . | . | . | . | . | . | . | . | . | . | . | . | P | . | . |
| 21-225_71A6_LC | . | . | . | . | . | . | . | . | . | . | . | . | . | . | . | . | P | . | . |

**FIGURE 57**

Table 114.    Consensus 34 - VL2|2a2/JL3b (SEQ ID NO: 50331):

QSALTQP-ASVSGSPGQSITISC<u>TGTS-SDVGGY</u>----NYVSWYQQIIPGKAPKLMIY<u>E</u>-------VSNRPSGVSNRFSGSKSG--
NTASLTISGLQAEDEADYYC<u>NSYTR</u>--------------------SITWVFGGGTKLTVLG wherein:

V at position 31 can be substituted with I

G at position 33 can be substituted with S

Y at position 40 can be substituted with F

S at position 68 can be substituted with R

N at position 107 can be substituted with G, C or S

T at position 110 can be substituted with V or K

R at position 111 can be substituted with S or K

S at position 134 can be substituted with G, N or R

I at position 135 can be substituted with S or Y

Thr Gly Thr Ser Ser Asp Xaa Gly Xaa Tyr Asn Xaa Val Ser (SEQ ID NO: 50177)

Glu Val Xaa Asn Arg Pro Ser (SEQ ID NO: 50178)

Xaa Ser Tyr Xaa Xaa Xaa Xaa Thr Trp Val (SEQ ID NO: 50179)

Thr Gly Thr Ser Ser Asp Val Gly Gly Tyr Asn Tyr Val Ser (SEQ ID NO: 50663)

Glu Val Ser Asn Arg Pro Ser (SEQ ID NO: 50664)

Asn Ser Tyr Thr Arg Ser Ile Thr Trp Val (SEQ ID NO: 50665)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | L_FR1 | | | | | | | | | | | | | | |
| CONSENSUS | Q | S | A | L | T | Q | P | - | A | S | V | S | G | S | P | G | Q | S | I | T | I | S | C | T | G | T |
| 21-225_178H8_LC | . | . | . | . | . | . | S | . | . | . | . | . | . | . | . | . | . | . | . | . | . | . | . | . | . | . |
| 21-225_210D12_LC | . | . | . | . | . | . | . | . | . | . | . | . | . | . | . | . | . | . | . | . | . | . | . | . | . | . |
| 21-225_210E12_LC | . | . | . | . | . | . | . | . | . | . | . | . | . | . | . | . | . | . | . | . | . | . | . | . | . | . |
| 21-225_211C1_LC | . | . | . | . | . | . | . | . | . | . | . | . | . | . | . | . | . | . | . | . | . | . | . | . | . | . |
| 21-225_211G5_LC | . | . | . | . | . | . | . | . | . | . | . | . | . | . | . | . | . | . | . | . | . | . | . | . | . | . |
| 21-225_212A4_LC | . | . | . | . | . | . | . | . | . | . | . | . | . | . | . | . | . | . | . | . | . | . | . | . | . | . |
| 21-225_212C2_LC | . | . | . | . | . | . | . | . | . | . | . | . | . | . | . | . | . | . | . | . | . | . | . | . | . | . |
| 21-225_212F6_LC | . | . | . | . | . | . | . | . | . | . | . | . | . | . | . | . | . | . | . | . | . | . | . | . | . | . |
| 21-225_212G7_LC | . | . | . | . | . | . | . | . | . | . | . | . | . | . | . | . | . | . | . | . | . | . | . | . | . | . |
| 21-225_213D5_LC | . | . | . | . | . | . | . | . | . | . | . | . | . | . | . | . | . | . | . | . | . | . | . | . | . | . |
| 21-225_213G3_LC | L | . | . | . | . | . | . | . | . | . | . | . | . | . | . | . | . | . | . | . | . | . | . | . | . | . |
| 21-225_215D3_LC | . | . | . | . | . | . | . | . | . | . | . | . | . | . | . | . | . | . | . | . | . | . | . | . | . | . |
| 21-225_216A3_LC | . | . | . | . | . | . | . | . | . | . | . | . | . | . | . | . | . | . | . | . | . | . | . | . | . | . |
| 21-225_217B2_LC | . | . | . | . | . | . | . | . | . | . | . | . | . | . | . | . | . | . | . | . | . | . | . | . | . | . |
| 21-225_219A7_LC | . | . | . | . | . | . | . | . | . | . | . | . | . | . | . | . | . | . | . | . | . | . | . | . | . | . |
| 21-225_221G4_LC | . | . | . | . | . | . | . | . | . | . | . | . | . | . | . | . | . | . | . | . | . | . | . | . | . | . |
| 21-225_221H2_LC | L | . | . | . | . | . | . | . | . | . | . | . | . | . | . | . | . | . | . | . | . | . | . | . | . | . |
| 21-225_52H4_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | L_CDR1 | | | | | | | | | | | | | | | | | | L_FR2 | | |
| CONSENSUS | S | - | S | D | V | G | G | Y | - | - | - | - | N | Y | V | S | W | Y | Q | Q | H | P | G | K | A | P |
| 21-225_178H8_LC | . | . | . | . | . | . | . | . | . | . | . | . | F | . | . | . | . | . | . | . | . | . | . | . | V | . |
| 21-225_210D12_LC | . | . | . | . | . | . | . | . | . | . | . | . | . | . | . | . | . | . | . | . | . | . | V | . | . | . |
| 21-225_210E12_LC | . | . | . | . | . | . | . | . | . | . | . | . | . | . | . | . | . | . | . | . | . | . | . | . | . | . |
| 21-225_211C1_LC | . | . | . | . | . | . | S | . | . | . | . | . | . | . | . | . | . | . | . | . | Y | . | . | . | . | . |
| 21-225_211G5_LC | . | . | . | . | . | . | . | . | . | . | . | . | . | . | . | . | . | . | . | . | . | . | . | . | . | . |
| 21-225_212A4_LC | . | . | . | . | . | . | . | . | . | . | . | . | . | . | . | . | . | . | . | . | . | . | . | . | . | . |
| 21-225_212C2_LC | . | . | . | . | . | . | . | . | . | . | . | . | . | . | . | . | . | . | . | . | . | . | . | . | . | . |
| 21-225_212F6_LC | . | . | . | . | . | . | . | . | . | . | . | . | . | . | . | . | . | . | . | . | Y | . | . | . | . | . |
| 21-225_212G7_LC | . | . | . | . | . | . | . | . | . | . | . | . | . | . | . | . | . | . | . | . | . | . | . | . | T | . |
| 21-225_213D5_LC | . | . | . | . | . | . | . | . | . | . | . | . | . | . | . | . | . | . | . | . | . | . | . | . | . | . |
| 21-225_213G3_LC | . | . | . | . | . | . | . | . | . | . | . | . | . | . | . | . | . | . | . | . | . | . | . | . | . | . |
| 21-225_215D3_LC | . | . | . | S | . | . | . | . | . | . | . | . | . | . | . | . | . | . | . | . | . | . | . | . | . | . |
| 21-225_216A3_LC | . | . | . | . | . | . | . | . | . | . | . | . | . | . | . | . | . | . | . | . | . | . | . | . | . | . |
| 21-225_217B2_LC | . | . | . | . | . | . | . | . | . | . | . | . | . | . | . | . | . | . | . | . | . | . | . | . | . | . |
| 21-225_219A7_LC | . | . | . | . | . | . | . | . | . | . | . | . | . | . | . | . | . | . | . | . | . | . | . | . | . | . |
| 21-225_221G4_LC | . | . | . | . | . | . | . | . | . | . | . | . | . | . | . | . | . | . | . | . | . | . | . | . | . | . |
| 21-225_221H2_LC | . | . | . | . | . | . | . | . | . | . | . | . | . | . | . | . | . | . | . | . | . | . | . | . | . | . |
| 21-225_52H4_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | L_CDR2 | | | | | | | | | | | | | | | |
| CONSENSUS | K | L | M | I | Y | E | - | - | - | - | - | - | - | - | V | S | N | R | P | S | G | V | S | N | R | F |
| 21-225_178H8_LC | . | S | . | . | . | . | . | . | . | . | . | . | . | . | . | . | . | . | . | . | . | . | S | . | . | . |
| 21-225_210D12_LC | . | . | L | . | . | . | . | . | . | . | . | . | . | . | . | . | . | . | . | . | . | . | . | . | . | . |
| 21-225_210E12_LC | . | . | . | . | . | . | . | . | . | . | . | . | . | . | . | . | . | . | . | . | . | . | . | . | . | . |
| 21-225_211C1_LC | . | . | . | . | . | . | . | . | . | . | . | . | . | . | . | . | . | . | . | . | . | . | . | . | . | . |
| 21-225_211G5_LC | . | . | . | . | . | . | . | . | . | . | . | . | . | . | . | . | . | . | . | . | . | . | . | . | . | . |
| 21-225_212A4_LC | . | . | . | . | . | . | . | . | . | . | . | . | . | . | . | . | . | . | . | . | . | . | . | . | . | . |
| 21-225_212C2_LC | . | . | . | . | . | . | . | . | . | . | . | . | . | . | . | S | . | . | . | . | . | . | . | . | . | . |
| 21-225_212F6_LC | . | . | . | . | . | . | . | . | . | . | . | . | . | . | . | . | . | . | . | . | . | . | . | . | . | . |
| 21-225_212G7_LC | . | . | . | . | . | . | . | . | . | . | . | . | . | . | . | . | . | . | . | . | . | . | . | . | . | . |
| 21-225_213D5_LC | . | . | V | . | . | . | . | . | . | . | . | . | . | . | . | S | . | . | . | . | . | . | . | . | T | . |
| 21-225_213G3_LC | . | . | . | S | . | . | . | . | . | . | . | . | . | . | . | S | . | . | . | . | . | . | . | . | . | . |
| 21-225_215D3_LC | T | . | . | . | . | . | . | . | . | . | . | . | . | . | . | . | . | . | . | . | . | . | . | . | . | . |
| 21-225_216A3_LC | . | . | . | . | . | . | . | . | . | . | . | . | . | . | . | . | . | . | . | . | . | . | . | . | . | . |
| 21-225_217B2_LC | . | . | L | . | . | . | . | . | . | . | . | . | . | . | . | . | . | . | . | . | . | . | Y | . | . | . |
| 21-225_219A7_LC | . | . | . | . | . | . | . | . | . | . | . | . | . | . | . | S | . | . | . | . | . | . | . | . | . | . |
| 21-225_221G4_LC | . | . | . | . | . | . | . | . | . | . | . | . | . | . | . | S | . | . | . | . | . | . | . | . | . | . |
| 21-225_221H2_LC | . | . | . | S | . | . | . | . | . | . | . | . | . | . | . | S | . | . | . | . | . | . | . | . | . | . |
| 21-225_52H4_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | L_FR3 | | | | | | | | | | | | | | | | |
| CONSENSUS | S | G | S | K | S | G | - | - | N | T | A | S | L | T | I | S | G | L | Q | A | E | D | E | A | D | Y |
| 21-225_178H8_LC | . | . | | | | | | | | . | . | . | . | | . | | | . | . | . | . | . | . | . | . | |
| 21-225_210D12_LC | . | . | | | | | | . | . | . | . | . | . | | . | | | . | . | . | . | . | . | . | . | |
| 21-225_210E12_LC | . | . | | | | | | . | . | . | . | . | . | | . | | | . | . | . | . | . | . | . | . | |
| 21-225_211C1_LC | . | . | | | | | . | . | . | . | . | . | . | | . | | | . | . | . | . | . | . | . | . | |
| 21-225_211G5_LC | . | . | | | | | . | . | . | . | . | . | . | | . | | | . | . | . | . | . | . | . | . | |
| 21-225_212A4_LC | . | . | | | | | . | . | . | . | . | . | . | | . | | | . | . | . | . | . | . | . | . | |
| 21-225_212C2_LC | . | . | | | | | . | . | . | . | . | . | . | | . | | | . | . | . | . | . | . | . | . | |
| 21-225_212F6_LC | . | . | | | | | . | . | . | . | . | . | . | | . | | | . | . | . | . | . | . | . | . | |
| 21-225_212G7_LC | . | . | | | | | . | . | . | . | . | . | . | | . | | | . | . | . | . | . | . | . | . | |
| 21-225_213D5_LC | . | . | | | | . | . | . | . | 5 | . | . | . | | . | | | . | . | . | . | . | . | . | . | |
| 21-225_213G3_LC | . | . | | | | . | . | . | . | . | . | . | . | | . | | | . | . | . | . | . | . | . | . | |
| 21-225_215D3_LC | . | . | | | | | . | . | . | . | . | . | . | | . | | | . | . | . | . | . | . | . | . | |
| 21-225_216A3_LC | . | . | | | | | . | . | . | . | . | . | . | | . | | | . | . | . | . | . | . | . | . | |
| 21-225_217B2_LC | . | . | | | | | . | . | . | . | . | . | . | | . | | | . | . | . | . | . | . | . | . | |
| 21-225_219A7_LC | . | . | | | | | . | . | . | . | . | . | . | | . | | | . | . | . | . | . | . | . | . | |
| 21-225_221G4_LC | . | . | | | | . | . | . | . | . | . | . | . | | . | | | . | . | . | . | . | . | . | . | |
| 21-225_221H2_LC | . | . | | | | | . | . | . | . | . | . | . | | . | | | . | . | . | . | . | . | . | . | |
| 21-225_52H4_LC | . | . | | | | | . | . | . | . | . | . | . | | . | | | . | . | . | . | . | . | . | . | |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | L_CDR3 | | | | | | | |
| CONSENSUS | Y | C | N | S | Y | T | R | - | - | - | - | - | - | - | - | - | - | - | - | - | - | - | - | - | - | - |
| 21-225_178H8_LC | . | . | S | . | . | . | S | . | . | . | . | . | . | . | . | . | . | . | . | . | . | . | . | . | . | . |
| 21-225_210D12_LC | . | . | . | . | . | . | S | . | . | . | . | . | . | . | . | . | . | . | . | . | . | . | . | . | . | . |
| 21-225_210E12_LC | . | . | G | . | . | V | R | . | . | . | . | . | . | . | . | . | . | . | . | . | . | . | . | . | . | . |
| 21-225_211C1_LC | . | . | . | . | . | . | S | . | . | . | . | . | . | . | . | . | . | . | . | . | . | . | . | . | . | . |
| 21-225_211G5_LC | . | . | . | . | . | . | . | . | . | . | . | . | . | . | . | . | . | . | . | . | . | . | . | . | . | . |
| 21-225_212A4_LC | . | . | G | . | . | V | R | . | . | . | . | . | . | . | . | . | . | . | . | . | . | . | . | . | . | . |
| 21-225_212C2_LC | . | . | S | . | . | . | . | . | . | . | . | . | . | . | . | . | . | . | . | . | . | . | . | . | . | . |
| 21-225_212F6_LC | . | . | . | . | . | . | S | . | . | . | . | . | . | . | . | . | . | . | . | . | . | . | . | . | . | . |
| 21-225_212G7_LC | . | . | . | . | . | . | S | . | . | . | . | . | . | . | . | . | . | . | . | . | . | . | . | . | . | . |
| 21-225_213D5_LC | . | . | C | . | . | . | . | . | . | . | . | . | . | . | . | . | . | . | . | . | . | . | . | . | . | . |
| 21-225_213G3_LC | . | . | . | . | . | . | . | . | . | . | . | . | . | . | . | . | . | . | . | . | . | . | . | . | . | . |
| 21-225_215D3_LC | . | . | . | . | . | R | . | . | . | . | . | . | . | . | . | . | . | . | . | . | . | . | . | . | . | . |
| 21-225_216A3_LC | . | . | . | . | . | . | S | . | . | . | . | . | . | . | . | . | . | . | . | . | . | . | . | . | . | . |
| 21-225_217B2_LC | . | . | G | . | . | V | R | . | . | . | . | . | . | . | . | . | . | . | . | . | . | . | . | . | . | . |
| 21-225_219A7_LC | . | . | C | . | . | . | . | . | . | . | . | . | . | . | . | . | . | . | . | . | . | . | . | . | . | . |
| 21-225_221G4_LC | . | . | . | . | . | . | . | . | . | . | . | . | . | . | . | . | . | . | . | . | . | . | . | . | . | . |
| 21-225_221H2_LC | . | . | . | . | . | . | . | . | . | . | . | . | . | . | . | . | . | . | . | . | . | . | . | . | . | . |
| 21-225_52H4_LC | . | . | . | . | . | . | S | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | L_FR4 | | | | | | |
| CONSENSUS | - | - | - | S | I | T | W | V | F | G | G | G | T | K | L | T | V | L | G |
| 21-225_178H8_LC | . | . | . | . | Y | . | . | . | . | . | . | . | . | . | . | . | . | . | . |
| 21-225_210D12_LC | . | . | . | . | . | . | . | . | . | . | . | . | . | . | . | . | . | . | . |
| 21-225_210E12_LC | . | . | . | G | . | . | . | . | . | . | . | . | . | S | . | . | . | . | . |
| 21-225_211C1_LC | . | . | . | . | . | . | . | . | . | . | . | . | . | . | . | . | . | . | . |
| 21-225_211G5_LC | . | . | . | . | . | . | . | . | . | . | . | . | . | . | . | . | . | . | . |
| 21-225_212A4_LC | . | . | . | G | . | . | . | . | . | . | . | . | . | S | . | . | . | . | . |
| 21-225_212C2_LC | . | . | . | . | . | . | . | . | . | . | . | . | . | . | . | . | . | . | . |
| 21-225_212F6_LC | . | . | . | . | . | . | . | . | . | . | . | . | . | . | . | . | . | . | . |
| 21-225_212G7_LC | . | . | . | . | . | . | . | . | . | . | . | . | . | . | . | . | . | . | . |
| 21-225_213D5_LC | . | . | . | N | . | . | . | . | . | . | . | . | . | . | . | . | . | . | S |
| 21-225_213G3_LC | . | . | . | . | . | . | . | . | . | . | . | . | . | . | . | . | . | . | . |
| 21-225_215D3_LC | . | . | . | G | S | . | . | . | . | . | . | . | . | . | V | . | . | . | . |
| 21-225_216A3_LC | . | . | . | . | . | . | . | . | . | . | . | . | . | . | . | . | . | . | . |
| 21-225_217B2_LC | . | . | . | G | . | . | . | . | . | . | . | . | . | S | . | . | . | . | . |
| 21-225_219A7_LC | . | . | . | . | . | . | . | . | . | . | . | . | . | . | . | . | . | . | . |
| 21-225_221G4_LC | . | . | . | . | . | . | . | . | . | . | . | . | . | . | . | . | . | . | . |
| 21-225_221H2_LC | . | . | . | . | . | . | . | . | . | . | . | . | . | . | . | . | . | . | . |
| 21-225_52H4_LC | . | . | . | N | . | . | . | . | . | . | . | . | . | . | . | . | . | . | . |

**FIGURE 57**

Table 115.    Consensus 35 - VK1|O12/JK1 (SEQ ID NO: 50332):

DIQMTQSPSSLSASVGDRVTITC<u>RAS</u>--<u>QSIS</u>------NYLNWYQQKPGKAPKLLIY<u>A</u>--------<u>ASSLQS</u>GVPSRFSGSGSG--
TDFTLTISSLQPEDFATYYC<u>QQSYS</u>---------------------TPTWTFGQGTKVEIKR wherein:

A at position 25 can be substituted with S

Q at position 29 can be substituted with H or R

S at position 30 can be substituted with N, T or H

S at position 32 can be substituted with N, G or T

N at position 39 can be substituted with S or R

Y at position 40 can be substituted with F

A at position 58 can be substituted with T, S or V

A at position 67 can be substituted with T, E or V

S at position 68 can be substituted with L

S at position 69 can be substituted with N

Q at position 71 can be substituted with H

S at position 72 can be substituted with I

S at position 109 can be substituted with G or T

S at position 111 can be substituted with T, N or R

T at position 134 can be substituted with S or null (-)

P at position 135 can be substituted with I

T at position 136 can be substituted with P, Q or L

**FIGURE 57**

Arg Xaa Ser Xaa Xaa Ile Xaa Xaa Xaa Leu Asn (SEQ ID NO: 50180)

Xaa Xaa Xaa Xaa Leu Xaa Xaa (SEQ ID NO: 50181)

Gln Gln Xaa Tyr Xaa Xaa Xaa Xaa Trp Thr (SEQ ID NO: 50182)

Arg Ala Ser Gln Ser Ile Ser Asn Tyr Leu Asn (SEQ ID NO: 50666)

Ala Ala Ser Ser Leu Gln Ser (SEQ ID NO: 50667)

Gln Gln Ser Tyr Ser Thr Pro Thr Trp Thr (SEQ ID NO: 50668)

EP 4 435 105 A2

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | R | A | S |
| 21-225_157F3_LC | . | | | . | . | . | . | . | . | . | . | | . | . | . | . | . | . | . | . | . | . | . | . | | . |
| 21-225_173E7_LC | . | | | . | . | . | . | . | . | 米 | . | | C | 1 | . | . | . | A | . | . | . | . | Y | . | . | |
| 21-225_175G10_LC | . | | | . | . | . | . | . | . | . | . | | . | . | . | . | . | 1 | . | . | . | . | . | . | . | |
| 21-225_17A1_LC | . | | | . | . | . | . | . | . | . | . | | . | . | . | . | . | . | . | . | . | . | . | . | . | |
| 21-225_17B10_LC | . | | | . | . | . | . | . | . | . | . | | . | . | . | . | . | . | . | . | . | . | . | . | S | . |
| 21-225_17B12_LC | . | | | . | . | . | . | . | . | . | . | | . | . | . | . | . | S | . | . | . | . | . | . | . | |
| 21-225_181G3_LC | . | | | . | . | . | . | . | . | . | . | | . | . | . | . | . | N | . | . | . | . | . | . | . | |
| 21-225_183A12_LC | . | | | . | . | . | . | . | . | C | . | | . | . | . | . | . | . | . | . | . | . | . | . | . | |
| 21-225_192G10_LC | . | | | . | . | . | . | . | . | . | . | | . | . | . | . | . | . | . | . | . | . | . | . | . | |
| 21-225_200G1_LC | . | | | . | . | . | . | . | . | . | . | | . | . | . | . | . | . | . | . | . | . | . | . | . | |
| 21-225_20C10_LC | . | 米 | | . | . | . | . | . | . | . | . | | . | . | . | 米 | . | S | . | . | . | . | . | . | . | |
| 21-225_20F8_LC | . | | | . | . | . | . | . | . | . | . | | . | . | . | . | . | . | . | . | . | . | . | . | . | |
| 21-225_23H4_LC | . | | | . | . | . | . | . | . | . | . | | . | . | . | . | . | . | . | . | . | . | . | . | . | |
| 21-225_26A10_LC | . | | | . | . | . | . | . | . | . | . | | . | . | . | . | . | 1 | . | . | . | . | . | . | . | |
| 21-225_54B1_LC | . | | | . | . | . | . | . | . | . | . | | 1 | . | . | . | . | . | . | . | . | . | . | . | . | |
| 21-225_58F1_LC | . | | | . | . | . | . | . | . | . | . | | . | . | . | . | . | . | . | . | . | . | . | . | . | |
| 21-225_7G12_LC | . | | | . | . | . | . | . | . | . | . | | . | . | . | . | . | S | . | . | . | . | . | . | . | |

4940

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | I | S | - | - | - | - | - | - | N | Y | L | N | W | Y | Q | Q | K | P | G | K | A | P |
| 21-225_157F3_LC | | | | N | . | . | . | . | . | . | . | . | S | . | | | | | | | L | . | | | | |
| 21-225_173E7_LC | | | . | T | . | . | . | . | . | . | . | . | . | . | | | | | | | . | . | | | | |
| 21-225_175G10_LC | | | . | T | . | G | . | . | . | . | . | . | . | . | | | | | | | . | . | | | R | . |
| 21-225_17A1_LC | | | | N | . | N | . | . | . | . | . | . | . | . | | | | | | | . | . | | | . | G |
| 21-225_17B10_LC | | | . | N | . | N | . | . | . | . | . | . | S | . | | | | | | | . | . | | | . | . |
| 21-225_17B12_LC | | . | . | N | . | N | . | . | . | . | . | . | . | . | | | | | | | . | . | | | . | G |
| 21-225_181G3_LC | | . | M | . | . | . | . | . | . | . | . | . | . | . | | | | | | | A | . | | | . | . |
| 21-225_183A12_LC | | . | R | N | . | . | . | . | . | . | . | . | . | . | | | | | | | . | . | | | . | . |
| 21-225_192G10_LC | | . | . | . | . | . | . | . | . | . | . | . | . | . | | | | | . | | T | . | | | . | . |
| 21-225_200G1_LC | | . | . | . | . | . | . | . | . | . | . | . | S | . | | | | F | . | | | . | . | | | . | . |
| 21-225_20C10_LC | | . | M | M | . | . | . | . | . | . | . | . | . | . | | | | | | | . | . | | | . | G |
| 21-225_20F8_LC | | . | . | . | . | . | . | . | . | . | . | . | R | . | | | | | | | . | . | | | . | . |
| 21-225_23H4_LC | | . | . | . | . | . | . | . | . | . | . | . | R | . | | | | | | | . | . | | | R | . |
| 21-225_26A10_LC | | . | . | . | . | . | . | . | . | . | . | . | S | . | | | | | | | . | . | | | . | . |
| 21-225_54B1_LC | | . | . | N | . | . | . | . | . | . | . | . | S | . | | | | | | | . | . | | | . | . |
| 21-225_58F1_LC | | . | . | . | . | T | . | . | . | . | . | . | . | F | | | | | | | . | . | | | . | . |
| 21-225_7G12_LC | | . | . | N | . | N | . | . | . | . | . | . | . | . | | | | | | | . | . | | | . | G |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_157F3_LC | . | | | | . | T | . | . | . | . | . | | | . | . | . | . | . | . | . | . | . | . | . | . | . |
| 21-225_173E7_LC | . | | . | . | . | . | . | . | . | . | . | | | . | . | . | . | . | . | I | . | . | . | . | . | . |
| 21-225_175G10_LC | . | | . | . | . | . | . | . | . | . | . | | | . | . | . | . | . | N | . | . | . | . | . | G | . |
| 21-225_17A1_LC | . | V | | . | F | T | . | . | . | . | . | | | . | T | . | . | . | . | . | . | . | . | . | . | . |
| 21-225_17B10_LC | . | | | . | F | . | . | . | . | . | . | | | . | . | . | . | . | N | . | . | . | . | . | . | . |
| 21-225_17B12_LC | . | V | . | . | L | T | . | . | . | . | . | | | . | . | . | . | . | . | . | . | . | . | . | . | . |
| 21-225_181G3_LC | N | . | . | . | . | T | . | . | . | . | . | | | . | T | L | N | . | . | . | . | . | . | . | . | . |
| 21-225_183A12_LC | . | . | . | . | . | V | . | . | . | . | . | | | . | V | . | . | . | . | . | . | . | . | . | . | . |
| 21-225_192G10_LC | . | . | . | . | . | . | . | . | . | . | . | | | . | E | . | . | . | . | . | . | . | . | . | . | . |
| 21-225_200G1_LC | . | . | . | . | . | . | . | . | . | . | . | | | . | E | . | . | . | . | . | . | . | . | . | . | . |
| 21-225_20C10_LC | . | V | . | . | L | T | . | . | . | . | . | | | . | . | . | . | . | . | . | . | . | . | . | . | . |
| 21-225_20F8_LC | E | . | . | . | . | . | . | . | . | . | . | | | . | . | . | . | . | . | . | . | . | . | . | . | . |
| 21-225_23H4_LC | . | . | . | . | C | S | . | . | . | . | . | | | . | T | . | . | . | . | . | . | . | . | . | . | . |
| 21-225_26A10_LC | . | V | . | . | . | . | . | . | . | . | . | | | . | . | . | . | . | . | . | . | . | . | . | . | . |
| 21-225_54B1_LC | . | F | . | . | . | . | . | . | . | . | . | | | . | . | . | . | . | N | . | . | . | . | P | . | . |
| 21-225_58F1_LC | . | . | . | . | F | . | . | . | . | . | . | | | . | . | . | . | . | . | . | I | . | . | . | . | . |
| 21-225_7G12_LC | . | V | . | . | L | T | . | . | . | . | . | | | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_157F3_LC | | | | | | | | | | | | | | | | | | | | | | | | | | |
| 21-225_173E7_LC | | | | | | | | | | | | | | | | | V | | | | | | | | | |
| 21-225_175G10_LC | | | | | | | | | | | | | | | | | | | | | | | | | | |
| 21-225_17A1_LC | | | | | | | | | | | | | | | | | | | | | | | | | | |
| 21-225_17B10_LC | | | | | | | | | | | | | | | | | | | | | | | | | | |
| 21-225_17B12_LC | | | | | | | | | | | | | | | | | | | | | | | | | | |
| 21-225_181G3_LC | | | | | | | | | | | | | | | | | | | | | | | | | | |
| 21-225_183A12_LC | | | | | | | | | | | | | | | | | | | | | | | | | | |
| 21-225_192G10_LC | | | | | | | | | | | | | | | | | | | | | | | | | | |
| 21-225_200G1_LC | | | | | | | | | | | | | | | | | | | | | | | | | | |
| 21-225_20C10_LC | | | | | | | | | | | | | | | | | | | | | | | | | | |
| 21-225_20F8_LC | | | | | | | | | | | | | | | | | | | | | | | | | | |
| 21-225_23H4_LC | | | | | | | | | | | | | | | | | | | | | | | | | | |
| 21-225_26A10_LC | | | | | | | | | | | | | | | | | | | | | | | | | | |
| 21-225_54B1_LC | | | | | | | | | | | | | | | | | | | | | | | | | | |
| 21-225_58F1_LC | | | | | | | | | | | | | | | | | | | | | | | | | | |
| 21-225_7G12_LC | | | | | | | | | | | | | | | | N | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_157F3_LC | . | | | | . | . | N | . | . | . | . | . | . | . | . | . | . | . | . | . | . | . | . | . | . | . |
| 21-225_173E7_LC | F | | . | . | . | . | R | . | . | . | . | . | . | . | . | . | . | . | . | . | . | . | . | . | . | . |
| 21-225_175G10_LC | F | | . | . | . | . | R | . | . | . | . | . | . | . | . | . | . | . | . | . | . | . | . | . | . | . |
| 21-225_17A1_LC | . | | | . | . | . | T | . | . | . | . | . | . | . | . | . | . | . | . | . | . | . | . | . | . | . |
| 21-225_17B10_LC | . | | . | . | Q | . | N | . | . | . | . | . | . | . | . | . | . | . | . | . | . | . | . | . | . | . |
| 21-225_17B12_LC | . | | . | . | T | . | T | . | . | . | . | . | . | . | . | . | . | . | . | . | . | . | . | . | . | . |
| 21-225_181G3_LC | . | | . | . | . | . | . | . | . | . | . | . | . | . | . | . | . | . | . | . | . | . | . | . | . | . |
| 21-225_183A12_LC | . | | . | . | . | . | . | . | . | . | . | . | . | . | . | . | . | . | . | . | . | . | . | . | . | . |
| 21-225_192G10_LC | S | | . | . | . | . | . | . | . | . | . | . | . | . | . | . | . | . | . | . | . | . | . | . | . | . |
| 21-225_200G1_LC | S | | . | . | . | . | . | . | . | . | . | . | . | . | . | . | . | . | . | . | . | . | . | . | . | . |
| 21-225_20C10_LC | . | | . | . | . | . | T | . | . | . | . | . | . | . | . | . | . | . | . | . | . | . | . | . | . | . |
| 21-225_20F8_LC | . | | . | . | . | . | N | . | . | . | . | . | . | . | . | . | . | . | . | . | . | . | . | . | . | . |
| 21-225_23H4_LC | . | | . | . | . | . | . | . | . | . | . | . | . | . | . | . | . | . | . | . | . | . | . | . | . | . |
| 21-225_26A10_LC | . | | . | . | . | . | . | . | . | . | . | . | . | . | . | . | . | . | . | . | . | . | . | . | . | . |
| 21-225_54B1_LC | . | | . | . | . | . | R | . | . | . | . | . | . | . | . | . | . | . | . | . | . | . | . | . | . | . |
| 21-225_58F1_LC | . | | . | . | . | . | . | . | . | . | . | . | . | . | . | . | . | . | . | . | . | . | . | . | . | . |
| 21-225_7G12_LC | . | | . | . | . | . | T | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | T | P | T | W | T | F | G | Q | G | T | K | V | E | I | K | R |
| 21-225_157F3_LC | . | . | . | . | . | . | . | . | . | . | . | . | . | . | . | . | . | . | . |
| 21-225_173E7_LC | . | . | . | . | . | Q | . | . | . | . | . | . | . | . | . | . | . | . | . |
| 21-225_175G10_LC | . | . | . | . | . | Q | . | . | . | . | . | . | . | . | . | . | . | . | . |
| 21-225_17A1_LC | . | . | . | . | . | . | . | . | . | . | . | . | . | . | . | . | . | . | . |
| 21-225_17B10_LC | . | . | . | . | . | . | . | . | . | . | . | . | . | . | . | . | . | N | . |
| 21-225_17B12_LC | . | . | . | . | . | . | . | . | . | . | . | . | . | . | . | . | . | . | . |
| 21-225_181G3_LC | . | . | . | S | . | P | . | . | . | . | . | . | . | . | . | . | . | . | . |
| 21-225_183A12_LC | . | . | . | S | . | P | . | . | . | . | . | . | . | . | S | . | . | . | . |
| 21-225_192G10_LC | . | . | . | S | . | P | . | . | . | . | . | . | . | . | . | . | . | . | . |
| 21-225_200G1_LC | . | . | . | S | . | P | . | . | . | . | . | . | . | . | . | . | P | . | . |
| 21-225_20C10_LC | . | . | . | . | . | . | . | . | . | . | . | . | . | . | . | . | . | . | . |
| 21-225_20F8_LC | . | . | . | . | . | . | . | . | . | . | . | . | . | . | . | . | . | . | . |
| 21-225_23H4_LC | . | . | . | . | . | . | . | . | . | . | . | . | . | . | . | . | . | R | . |
| 21-225_26A10_LC | . | . | . | . | . | . | . | . | . | . | . | . | . | . | . | . | . | . | . |
| 21-225_54B1_LC | . | . | . | . | . | L | . | . | . | . | . | . | . | . | . | . | . | . | . |
| 21-225_58F1_LC | . | . | . | - | I | P | . | . | . | . | . | . | . | . | . | . | . | . | . |
| 21-225_7G12_LC | . | . | . | . | . | . | . | . | . | . | . | . | . | . | . | . | . | . | . |

**FIGURE 57**

Table 116.    Consensus 36 - VK1|L5/JK4 (SEQ ID NO: 50333):

DIQMTQSPSSVSASVGDRVTITC<u>RAS--QGIS-----SWLA</u>WYQQKPGKAPKLLIY<u>A--------ASSLQS</u>GVPSRFSGSGSG--
TDFTLTISSLQPEDFATYYC<u>QQINS----------------------FPLT</u>FGGGTKVEIKR wherein:

G at position 30 can be substituted with D

S at position 39 can be substituted with N, I or K

W at position 40 can be substituted with Y

S at position 72 can be substituted with G

I at position 109 can be substituted with T, V, A or G

N at position 110 can be substituted with K

Arg Ala Ser Gln Xaa Ile Ser Xaa Xaa Leu Ala (SEQ ID NO: 50183)

Ala Ala Ser Ser Leu Gln Xaa (SEQ ID NO: 50184)

Gln Gln Xaa Xaa Ser Phe Pro Leu Thr (SEQ ID NO: 50185)

Arg Ala Ser Gln Gly Ile Ser Ser Trp Leu Ala (SEQ ID NO: 50669)

Ala Ala Ser Ser Leu Gln Ser (SEQ ID NO: 50670)

Gln Gln Ile Asn Ser Phe Pro Leu Thr (SEQ ID NO: 50671)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | V | S | A | S | V | G | D | R | V | T | I | T | C | R | A | S |
| 21-225_154E9_LC | . | . | . | . | L | . | | | . | | . | Y | . | . | . | . | . | . | . | . | . | . | . | . | . | . |
| 21-225_62G7_LC | . | . | . | . | . | . | | | . | | . | . | . | . | . | . | . | . | . | . | N | . | . | . | . | . |
| 21-225_63C9_LC | . | . | . | . | . | . | | | . | | . | . | . | . | . | . | . | . | . | . | . | . | . | . | . | . |
| 21-225_63E1_LC | . | . | . | . | . | . | | | . | | . | Y | . | . | . | . | . | . | . | . | . | . | . | . | . | . |
| 21-225_64H10_LC | . | . | . | . | . | . | | | . | | . | . | . | . | . | . | . | . | . | . | . | . | . | . | . | . |
| 21-225_65G3_LC | . | . | . | . | . | . | | | . | | . | . | . | . | . | . | . | . | . | . | . | . | . | . | . | . |
| 21-225_66A7_LC | . | . | . | . | . | . | | | . | | . | . | . | . | . | . | . | . | . | . | . | . | . | . | . | . |
| 21-225_66B1_LC | . | . | . | . | . | . | | | . | | . | . | . | . | . | . | . | . | . | . | . | . | . | . | . | . |
| 21-225_67H4_LC | . | . | . | . | . | . | | | . | | . | . | . | . | . | . | . | . | . | . | . | . | . | . | . | . |
| 21-225_70A12_LC | . | . | . | . | . | . | | | . | | . | . | . | . | . | . | . | . | . | . | . | . | . | . | . | . |
| 21-225_70D6_LC | . | . | . | . | . | . | | | . | | . | . | . | . | . | . | . | . | . | . | . | . | . | . | . | . |
| 21-225_70G9_LC | . | . | . | . | . | . | | | . | | . | . | . | . | . | . | . | . | . | . | . | . | . | . | . | . |
| 21-225_71A7_LC | . | . | . | . | . | . | | | . | | . | . | . | . | . | . | . | . | . | . | . | . | . | . | . | . |
| 21-225_71B7_LC | . | . | . | . | . | . | | | . | | . | . | . | . | . | . | . | . | . | . | . | . | . | . | . | . |
| 21-225_73A3_LC | . | . | . | . | . | . | | | . | | . | . | . | . | . | . | . | . | . | . | . | . | . | . | . | . |
| 21-225_75C10_LC | . | . | . | . | . | . | | | . | | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | S | - | - | - | - | - | - | S | W | L | A | W | Y | Q | Q | K | P | G | K | A | P |
| 21-225_154E9_LC | . | . | . | D | . | . | . | . | . | . | . | . | N | . | . | . | . | . | . | . | P | . | . | . | . | . |
| 21-225_62G7_LC | . | . | . | D | . | . | . | . | . | . | . | . | . | . | . | . | . | . | . | . | N | . | . | . | . | . |
| 21-225_63C9_LC | . | . | . | . | . | . | . | . | . | . | . | . | . | . | . | . | . | . | . | . | . | . | . | . | . | . |
| 21-225_63E1_LC | . | . | . | . | . | . | . | . | . | . | . | . | . | Y | . | . | . | . | . | . | . | . | . | . | . | . |
| 21-225_64H10_LC | . | . | . | . | . | . | . | . | . | . | . | . | . | . | . | . | . | . | . | . | . | . | . | . | . | L |
| 21-225_65G3_LC | . | . | . | . | . | . | . | . | . | . | . | . | . | . | . | . | . | . | . | . | . | . | . | . | . | . |
| 21-225_66A7_LC | . | . | . | . | . | . | . | . | . | . | . | . | . | . | . | . | . | . | . | . | . | . | . | . | . | . |
| 21-225_66B1_LC | . | . | . | . | . | . | . | . | . | . | . | . | . | . | . | . | . | . | . | . | . | . | . | . | . | L |
| 21-225_67H4_LC | . | . | . | . | . | . | . | . | . | . | . | . | . | . | . | . | . | . | . | . | . | . | . | . | . | . |
| 21-225_70A12_LC | . | . | . | . | . | . | . | . | . | . | . | . | . | . | . | . | . | . | . | . | . | . | . | . | . | L |
| 21-225_70D6_LC | . | . | . | . | . | . | . | . | . | . | . | . | N | . | . | . | . | . | . | . | N | . | . | . | . | L |
| 21-225_70G9_LC | . | . | . | D | . | . | . | . | . | . | . | . | . | . | . | . | . | . | . | . | N | . | . | . | . | . |
| 21-225_71A7_LC | . | . | . | . | . | . | . | . | . | . | . | . | . | . | . | . | . | . | . | . | . | . | . | . | . | . |
| 21-225_71B7_LC | . | . | . | . | . | . | . | . | . | . | . | . | . | . | . | . | . | . | . | . | N | . | . | S | . | . |
| 21-225_73A3_LC | . | . | . | . | . | . | . | . | . | . | . | . | N | . | . | . | . | . | . | . | . | . | . | . | . | . |
| 21-225_75C10_LC | . | . | . | . | . | . | . | . | . | . | . | . | I | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | A | - | - | - | - | - | - | - | - | A | S | S | L | Q | S | G | V | P | S | R | F |
| 21-225_154E9_LC | . | V | . | . | . | . | . | . | . | . | . | . | . | . | . | . | . | . | . | . | . | . | . | . | . | . |
| 21-225_62G7_LC | . | . | . | . | . | . | . | . | . | . | . | . | . | . | . | . | . | . | . | . | . | . | . | . | . | . |
| 21-225_63C9_LC | N | . | . | . | . | . | . | . | . | . | . | . | . | . | . | . | . | . | . | . | . | . | . | . | . | . |
| 21-225_63E1_LC | . | . | . | . | . | . | . | . | . | . | . | . | . | . | . | . | . | . | . | . | . | . | . | . | . | . |
| 21-225_64H10_LC | . | . | . | . | . | . | . | . | . | . | . | . | . | . | . | . | . | . | . | . | . | . | . | . | . | . |
| 21-225_65G3_LC | . | . | . | . | . | . | . | . | . | . | . | . | . | . | . | . | . | . | . | . | . | . | . | . | . | . |
| 21-225_66A7_LC | . | . | . | . | . | . | . | . | . | . | . | . | . | . | . | . | . | . | . | . | . | . | . | . | . | . |
| 21-225_66B1_LC | . | . | . | . | . | . | . | . | . | . | . | . | . | . | . | . | . | . | . | . | . | . | . | . | . | . |
| 21-225_67H4_LC | . | . | . | . | . | . | . | . | . | . | . | . | . | . | . | . | . | . | . | . | . | . | . | . | . | . |
| 21-225_70A12_LC | . | . | . | . | . | . | . | . | . | . | . | . | . | . | . | . | . | . | . | . | . | . | . | . | . | . |
| 21-225_70D6_LC | . | . | . | . | R | . | . | . | . | . | . | . | . | . | . | . | . | . | . | . | . | . | . | . | . | . |
| 21-225_70G9_LC | . | . | . | . | . | . | . | . | . | . | . | . | . | . | . | . | . | . | . | . | . | . | . | . | . | . |
| 21-225_71A7_LC | . | . | . | . | . | . | . | . | . | . | . | . | . | . | . | . | . | . | . | . | . | . | . | . | . | . |
| 21-225_71B7_LC | . | . | . | . | . | . | . | . | . | . | . | . | . | . | . | . | . | . | . | G | . | . | . | . | . | . |
| 21-225_73A3_LC | . | . | . | . | . | . | . | . | . | . | . | . | . | . | . | . | . | . | . | . | . | . | . | . | . | . |
| 21-225_75C10_LC | . | . | . | . | . | . | . | . | . | . | . | . | . | . | . | . | . | . | . | . | . | . | L | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_154E9_LC | . | . | . | . | . | . | | | | | | | . | . | . | . | . | V | | | | . | . | . | . | . |
| 21-225_62G7_LC | . | . | . | . | . | . | | | | N | | | . | . | . | . | . | . | | | | . | . | . | . | . |
| 21-225_63C9_LC | . | . | . | . | . | . | | | | | . | . | . | . | . | G | . | | | | . | . | . | . | . |
| 21-225_63E1_LC | . | . | . | . | . | . | | | | | . | . | . | . | . | . | . | | | | . | . | . | . | . |
| 21-225_64H10_LC | . | . | . | . | . | . | | | | | . | . | . | . | . | . | . | | | | . | . | . | . | . |
| 21-225_65G3_LC | . | . | . | . | . | . | | | | | . | . | . | . | . | . | . | | | | . | . | . | . | S |
| 21-225_66A7_LC | . | . | . | . | . | . | | | | | . | . | . | . | . | . | . | | | | . | . | . | . | . |
| 21-225_66B1_LC | . | . | . | . | . | . | | | | | . | . | . | . | . | . | . | | | | . | . | . | . | . |
| 21-225_67H4_LC | . | . | . | . | . | . | | | | | . | . | . | . | . | . | . | | | | . | . | . | . | . |
| 21-225_70A12_LC | . | . | . | . | . | . | | | | | . | . | . | . | . | . | V | | | | . | . | . | . | . |
| 21-225_70D6_LC | . | . | . | . | . | . | | | | | . | . | . | . | . | . | . | | | | . | . | . | N | . |
| 21-225_70G9_LC | . | . | . | . | . | . | | | | | . | . | . | . | . | . | . | | | | . | . | . | . | . |
| 21-225_71A7_LC | . | . | . | . | . | . | | | | | . | . | . | . | . | . | . | | | | . | . | . | . | . |
| 21-225_71B7_LC | . | . | . | . | . | . | | | | | . | . | . | . | . | . | . | | | | . | . | . | N | . |
| 21-225_73A3_LC | . | . | . | . | . | S | . | | | | . | . | . | . | . | . | . | | | | . | . | . | . | . |
| 21-225_75C10_LC | . | . | . | . | . | . | | | | | . | . | . | . | . | . | . | | | | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | | N | S | - | - | - | - | - | - | - | - | - | - | - | - | - | - | - | - | - | - | - |
| 21-225_154E9_LC | . | . | . | . | G | . | | | | | | | | | | | | | | | | | | | | |
| 21-225_62G7_LC | . | . | . | . | T | . | | | | | | | | | | | | | | | | | | | | |
| 21-225_63C9_LC | K | . | . | . | I | . | | | | | | | | | | | | | | | | | | | | |
| 21-225_63E1_LC | . | . | . | . | A | . | | | | | | | | | | | | | | | | | | | | |
| 21-225_64H10_LC | . | . | . | . | I | . | | | | | | | | | | | | | | | | | | | | |
| 21-225_65G3_LC | . | . | . | . | V | . | | | | | | | | | | | | | | | | | | | | |
| 21-225_66A7_LC | . | . | . | . | I | . | | | | | | | | | | | | | | | | | | | | |
| 21-225_66B1_LC | . | . | . | . | T | . | | | | | | | | | | | | | | | | | | | | |
| 21-225_67H4_LC | . | . | . | . | I | . | | | | | | | | | | | | | | | | | | | | |
| 21-225_70A12_LC | . | . | . | . | I | . | | | | | | | | | | | | | | | | | | | | |
| 21-225_70D6_LC | . | . | . | . | T | . | | | | | | | | | | | | | | | | | | | | |
| 21-225_70G9_LC | . | . | . | . | T | . | | | | | | | | | | | | | | | | | | | | |
| 21-225_71A7_LC | . | . | . | . | V | . | | | | | | | | | | | | | | | | | | | | |
| 21-225_71B7_LC | . | . | . | . | T | . | | | | | | | | | | | | | | | | | | | | |
| 21-225_73A3_LC | . | . | . | . | V | . | | | | | | | | | | | | | | | | | | | | |
| 21-225_75C10_LC | . | . | . | . | A | K | | | | | | | | | | | | | | | | | | | | |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | F | P | L | T | F | G | G | G | T | K | V | E | I | K | R |
| 21-225_154E9_LC | . | . | . | . | . | . | . | . | . | . | . | . | . | . | . | D | . | . | . |
| 21-225_62G7_LC | . | . | . | . | . | . | . | . | . | . | . | . | . | . | . | . | . | . | . |
| 21-225_63C9_LC | . | . | . | . | . | . | . | . | . | . | . | . | . | . | A | . | . | . | . |
| 21-225_63E1_LC | . | . | . | . | . | . | . | . | . | . | . | . | . | . | . | . | . | . | . |
| 21-225_64H10_LC | . | . | . | . | . | . | . | . | . | . | . | . | . | . | . | . | . | . | . |
| 21-225_65G3_LC | . | . | . | . | . | . | . | . | . | . | . | . | . | . | . | . | . | . | . |
| 21-225_66A7_LC | . | . | . | . | . | . | . | . | . | . | . | . | . | . | . | . | . | . | . |
| 21-225_66B1_LC | . | . | . | . | . | . | . | . | . | . | . | . | . | . | . | . | . | . | . |
| 21-225_67H4_LC | . | . | . | . | . | . | . | . | . | . | . | . | . | . | . | . | . | . | . |
| 21-225_70A12_LC | . | . | . | . | . | . | . | . | . | . | . | . | . | . | . | . | . | . | . |
| 21-225_70D6_LC | . | . | . | . | . | . | . | . | . | . | . | . | . | . | . | . | . | . | . |
| 21-225_70G9_LC | . | . | . | . | . | . | . | . | . | . | . | . | . | . | . | . | . | . | . |
| 21-225_71A7_LC | . | . | . | . | . | . | . | . | . | . | . | . | . | . | . | . | L | . | . |
| 21-225_71B7_LC | . | . | . | . | . | . | . | . | . | . | . | . | . | . | . | . | . | . | . |
| 21-225_73A3_LC | . | . | . | . | . | . | . | . | . | . | . | . | . | . | . | . | . | . | . |
| 21-225_75C10_LC | . | . | . | . | . | . | . | . | . | . | . | . | . | . | . | . | . | . | . |

**FIGURE 57**

Table 117.    Consensus 37 - VK1|O18/JK5 (SEQ ID NO: 50334):

DIQMTQSPSSLSASVGDRVTITC<u>QAS--QDIN</u>------NYLNWYQQKPGKAPKLLIY<u>D-------ASNLETG</u>VPSRFSGSGSG--
TDFTFTISSLQPEDIATYYC<u>QQYDN</u>----------------------LPITFGQGTRLEIKR wherein:

S at position 26 can be substituted with N

D at position 30 can be substituted with Y

N at position 32 can be substituted with S, T, F or Y

N at position 39 can be substituted with D

Y at position 40 can be substituted with F

A at position 67 can be substituted with G

N at position 69 can be substituted with T, D or S

Y at position 109 can be substituted with F

D at position 110 can be substituted with E

N at position 111 can be substituted with null (-) or I

L at position 135 can be substituted with N or V

P at position 136 can be substituted with L

Gln Ala Xaa Gln Xaa Ile Xaa Xaa Xaa Leu Asn (SEQ ID NO: 50186)

Asp Xaa Ser Xaa Leu Glu Thr (SEQ ID NO: 50187)

Gln Gln Xaa Xaa Xaa Xaa Xaa Ile Thr (SEQ ID NO: 50188)

Gln Ala Ser Gln Asp Ile Asn Asn Tyr Leu Asn (SEQ ID NO: 50672)

Asp Ala Ser Asn Leu Glu Thr (SEQ ID NO: 50673)

## FIGURE 57

Gln Gln Tyr Asp Asn Leu Pro Ile Thr (SEQ ID NO: 50674)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | D | I | Q | M | T | Q | S | P | S | S | L | S | A | S | V | G | D | R | V | T | I | T | C | Q | A | S |
| 21-225_11A2_LC | . | . | . | . | . | . | . | . | . | . | . | . | . | . | . | . | . | . | . | . | . | . | . | . | . | . |
| 21-225_13A1_LC | . | . | . | . | . | . | . | . | . | . | . | . | . | . | . | . | . | . | . | . | . | . | . | . | . | . |
| 21-225_159C4_LC | . | . | . | . | . | . | . | . | . | . | . | . | . | . | . | . | . | . | . | . | . | . | . | . | . | . |
| 21-225_15C2_LC | . | . | . | . | . | . | . | . | . | . | . | . | . | . | . | . | . | . | . | . | . | . | . | . | . | . |
| 21-225_15F10_LC | . | . | . | . | . | . | . | . | . | . | . | . | . | . | . | . | . | . | . | . | . | . | . | . | . | N |
| 21-225_161G4_LC | . | . | . | . | . | . | . | . | . | . | . | . | . | . | . | . | . | . | . | . | . | . | . | . | . | . |
| 21-225_16B3_LC | . | . | . | . | . | . | . | . | . | . | . | . | . | . | . | . | . | . | . | . | . | . | . | . | . | . |
| 21-225_16F6_LC | . | . | . | . | . | . | . | . | . | . | . | . | . | . | . | . | . | . | . | . | . | . | . | . | . | N |
| 21-225_1B12_LC | . | . | . | . | . | . | . | . | . | . | . | . | . | . | . | . | . | . | . | . | . | . | . | . | . | . |
| 21-225_225B11_LC | . | . | . | . | . | . | . | . | . | . | . | . | . | I | . | . | . | . | . | . | . | . | . | . | . | . |
| 21-225_24D6_LC | . | . | . | . | . | . | . | . | . | . | . | . | . | . | . | . | . | . | . | . | . | . | . | . | . | . |
| 21-225_30E2_LC | . | . | . | . | . | . | . | . | N | . | . | . | . | . | . | . | . | . | . | . | . | . | . | . | . | . |
| 21-225_35E3_LC | . | . | . | . | . | . | . | . | . | . | . | . | . | . | . | . | . | . | . | . | . | . | . | . | . | . |
| 21-225_50G4_LC | . | . | . | . | . | . | . | Y | . | . | . | . | . | . | . | . | . | . | . | . | . | . | . | . | . | . |
| 21-225_5E6_LC | . | . | . | . | . | . | . | . | . | . | . | . | . | . | . | . | . | . | . | . | . | . | . | . | . | . |
| 21-225_6G7_LC | . | . | . | . | . | . | . | . | . | N | . | . | . | . | . | . | . | . | . | . | . | . | . | . | . | . |

4954

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | D | I | N | - | - | - | - | - | - | N | Y | L | N | W | Y | Q | Q | K | P | G | K | A | P |
| 21-225_11A2_LC | . | . | . | . | . | S | . | . | . | . | . | . | . | . | . | . | . | . | . | . | . | . | . | . | . | . |
| 21-225_13A1_LC | . | . | . | . | . | . | . | . | . | . | . | . | . | . | . | . | . | . | . | . | . | . | L | . | T | . |
| 21-225_159C4_LC | . | . | . | . | . | S | . | . | . | . | . | . | Q | . | . | . | . | . | . | . | . | . | . | . | . | . |
| 21-225_15C2_LC | . | . | . | . | . | T | . | . | . | . | . | . | . | . | . | . | . | . | . | . | . | . | . | . | . | . |
| 21-225_15F10_LC | . | . | . | . | . | T | . | . | . | . | . | . | . | F | . | . | . | . | . | . | L | . | . | . | . | . |
| 21-225_161G4_LC | . | . | . | . | . | Y | . | . | . | . | . | . | . | . | . | . | . | . | . | . | . | . | . | . | . | . |
| 21-225_16B3_LC | . | . | . | . | . | . | . | . | . | . | . | . | . | . | . | . | . | . | . | . | . | . | . | . | . | . |
| 21-225_16F6_LC | . | . | . | . | . | T | . | . | . | . | . | . | . | F | . | . | . | . | . | . | L | . | . | . | . | . |
| 21-225_1B12_LC | . | . | . | . | . | . | . | . | . | . | . | . | . | . | . | . | . | . | . | . | . | . | . | . | . | . |
| 21-225_225B11_LC | . | . | . | . | . | F | . | . | . | . | . | . | . | . | . | . | . | . | . | . | . | . | . | . | . | . |
| 21-225_24D6_LC | . | . | . | . | . | . | . | . | . | . | . | . | . | . | . | . | . | . | . | . | . | . | . | . | . | L |
| 21-225_30E2_LC | . | . | Y | . | . | S | . | . | . | . | . | . | . | . | . | . | . | . | . | . | . | S | . | . | . | . |
| 21-225_35E3_LC | . | . | . | . | . | F | . | . | . | . | . | . | . | . | . | . | . | . | . | . | . | . | . | . | . | . |
| 21-225_50G4_LC | . | . | . | . | . | T | . | . | . | . | . | . | . | F | . | . | . | . | . | . | . | . | . | . | . | . |
| 21-225_5E6_LC | . | . | . | . | . | . | . | . | . | . | . | . | . | . | . | . | . | . | C | . | . | . | . | . | . | . |
| 21-225_6G7_LC | . | . | . | . | . | S | . | . | . | . | . | . | . | . | . | . | . | . | C | . | . | . | . | . | . | L |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | D | - | - | - | - | - | - | - | - | A | S | N | L | E | T | G | V | P | S | R | F |
| 21-225_11A2_LC | . | . | . | . | . | . | . | . | . | . | . | . | . | . | . | . | . | . | . | . | . | . | . | . | . | . |
| 21-225_13A1_LC | . | . | . | . | . | . | . | . | . | . | . | . | . | . | G | . | . | . | . | . | . | . | . | . | . | . |
| 21-225_159C4_LC | . | . | . | . | . | . | . | . | . | . | . | . | . | . | . | . | Y | . | . | . | . | . | . | . | . | . |
| 21-225_15C2_LC | N | . | . | . | . | . | . | . | . | . | . | . | . | . | . | . | . | . | . | . | . | . | . | . | . | . |
| 21-225_15F10_LC | N | . | . | . | S | . | . | . | . | . | . | . | . | . | . | . | . | . | . | . | . | . | . | . | . | . |
| 21-225_161G4_LC | . | . | . | . | . | . | . | . | . | . | . | . | . | . | . | . | . | . | . | . | . | . | . | . | . | . |
| 21-225_16B3_LC | . | . | . | . | . | . | . | . | . | . | . | . | . | . | . | . | . | . | . | . | . | . | . | . | . | . |
| 21-225_16F6_LC | N | . | . | . | S | . | . | . | . | . | . | . | . | . | . | . | . | . | . | . | . | . | . | . | . | . |
| 21-225_1B12_LC | . | . | . | . | . | . | . | . | . | . | . | . | . | . | . | . | . | . | . | . | . | . | . | . | . | . |
| 21-225_225B11_LC | . | . | . | . | . | . | . | . | . | . | . | . | . | . | . | . | Y | . | . | . | . | . | . | . | . | . |
| 21-225_24D6_LC | . | . | . | . | . | . | . | . | . | . | . | . | . | . | . | . | D | . | . | . | . | . | . | . | . | . |
| 21-225_30E2_LC | . | . | . | . | . | . | . | . | . | . | . | . | . | . | G | . | S | . | . | . | . | . | . | . | . | . |
| 21-225_35E3_LC | N | . | . | . | . | . | . | . | . | . | . | . | . | . | . | . | . | . | . | . | . | . | . | . | . | . |
| 21-225_50G4_LC | . | . | . | . | . | . | . | . | . | . | . | . | . | . | . | . | . | . | . | . | . | . | . | . | . | . |
| 21-225_5E6_LC | . | . | . | . | . | . | . | . | . | . | . | . | . | . | . | . | . | . | . | . | . | . | . | . | . | . |
| 21-225_6G7_LC | . | . | . | . | . | . | . | . | . | . | . | . | . | . | . | . | Y | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | F | T | I | S | S | L | Q | P | E | D | I | A | T | Y |
| 21-225_11A2_LC | . | . | . | . | . | . | . | . | . | . | . | . | . | . | . | . | . | . | . | . | . | . | V | . | . | . |
| 21-225_13A1_LC | . | . | . | . | . | . | . | . | . | . | . | . | . | . | . | . | . | . | . | . | . | . | . | . | . | . |
| 21-225_159C4_LC | . | . | . | . | . | . | . | . | . | . | . | . | . | . | . | . | . | . | . | . | . | . | . | . | . | . |
| 21-225_15C2_LC | . | . | . | . | . | . | . | . | . | . | . | . | . | . | . | . | . | . | . | . | . | . | . | . | . | . |
| 21-225_15F10_LC | . | . | . | . | . | . | . | . | . | . | . | . | . | . | . | . | . | . | . | . | . | . | V | . | . | . |
| 21-225_161G4_LC | . | . | . | . | . | . | . | . | . | . | . | . | . | A | . | . | . | . | . | . | . | . | . | . | . | . |
| 21-225_16B3_LC | . | . | . | . | . | . | . | . | . | . | . | . | . | . | . | . | . | . | . | . | . | . | . | . | . | . |
| 21-225_16F6_LC | . | . | . | . | . | . | . | . | . | . | . | . | . | . | . | . | . | . | . | . | . | . | V | . | . | . |
| 21-225_1B12_LC | . | . | . | . | . | . | . | . | . | . | . | . | . | . | . | . | . | . | . | . | . | . | . | T | . | . |
| 21-225_225B11_LC | . | . | . | . | . | . | . | . | . | . | . | . | . | . | . | . | . | . | . | . | . | . | . | . | . | . |
| 21-225_24D6_LC | . | . | . | . | . | . | . | . | . | . | . | . | . | . | . | . | . | . | . | . | . | . | . | . | I | . |
| 21-225_30E2_LC | . | . | . | . | . | . | . | . | . | . | . | . | . | . | . | . | . | . | . | . | . | L | . | . | . | . |
| 21-225_35E3_LC | . | . | . | . | . | . | . | . | . | . | . | . | . | . | . | . | . | . | . | . | . | . | . | . | . | . |
| 21-225_50G4_LC | . | . | . | . | . | . | . | . | . | . | . | . | . | . | . | . | . | . | . | . | . | . | . | . | . | . |
| 21-225_5E6_LC | . | . | . | . | . | . | . | . | . | . | . | . | . | . | . | . | . | . | . | . | . | . | . | . | . | . |
| 21-225_6G7_LC | . | . | . | . | . | . | . | . | . | . | . | . | . | . | . | . | V | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | D | N | - | - | - | - | - | - | - | - | - | - | - | - | - | - | - | - | - | - | - |
| 21-225_11A2_LC | | | | | | | | | | | | | | | | | | | | | | | | | | |
| 21-225_13A1_LC | | | | | | | | | | | | | | | | | | | | | | | | | | |
| 21-225_159C4_LC | | | | | | | | | | | | | | | | | | | | | | | | | | |
| 21-225_15C2_LC | | | | | | | | | | | | | | | | | | | | | | | | | | |
| 21-225_15F10_LC | | | | | | | | | | | | | | | | | | | | | | | | | | |
| 21-225_161G4_LC | | | | | | | | | | | | | | | | | | | | | | | | | | |
| 21-225_16B3_LC | | | | | | | | | | | | | | | | | | | | | | | | | | |
| 21-225_16F6_LC | | | | | | | | | | | | | | | | | | | | | | | | | | |
| 21-225_1B12_LC | | | | | | | | | | | | | | | | | | | | | | | | | | |
| 21-225_225B11_LC | | | | | | | | | | | | | | | | | | | | | | | | | | |
| 21-225_24D6_LC | | | | | | | | | | | | | | | | | | | | | | | | | | |
| 21-225_30E2_LC | | | | | | | | | | | | | | | | | | | | | | | | | | |
| 21-225_35E3_LC | | | | | | | | | | | | | | | | | | | | | | | | | | |
| 21-225_50G4_LC | | | | | | | - | | | | | | | | | | | | | | | | | | | |
| 21-225_5E6_LC | | | | | | | | | | | | | | | | | | | | | | | | | | |
| 21-225_6G7_LC | | | | | | | | | | | | | | | | | | | | | | | | | | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | L | P | I | T | F | G | Q | G | T | R | L | E | I | K | R |
| 21-225_11A2_LC | . | . | . | . | . | . | . | . | . | . | . | . | . | . | . | D | . | . | . |
| 21-225_13A1_LC | . | . | . | . | . | . | . | . | . | . | . | . | . | . | . | . | . | . | . |
| 21-225_159C4_LC | . | . | . | . | . | . | . | . | . | . | . | . | . | . | . | . | . | . | . |
| 21-225_15C2_LC | . | . | . | . | . | . | . | . | . | A | . | . | . | . | . | . | . | . | . |
| 21-225_15F10_LC | . | . | . | . | . | . | . | . | . | . | . | . | . | . | . | . | . | . | . |
| 21-225_161G4_LC | . | . | . | . | . | . | . | . | . | . | . | . | . | . | . | . | . | . | . |
| 21-225_16B3_LC | . | . | . | . | . | . | . | . | . | . | . | . | . | . | . | . | . | . | . |
| 21-225_16F6_LC | . | . | . | . | . | . | . | . | . | . | . | . | . | . | . | . | . | . | . |
| 21-225_1B12_LC | . | . | . | . | . | . | . | . | . | . | . | . | . | . | . | . | . | . | . |
| 21-225_225B11_LC | . | . | . | . | . | . | . | . | . | . | . | . | . | . | . | . | . | . | . |
| 21-225_24D6_LC | . | . | . | . | . | . | . | . | . | . | . | . | . | . | . | . | . | . | . |
| 21-225_30E2_LC | . | . | . | . | . | . | . | . | . | . | . | . | . | . | . | . | . | . | . |
| 21-225_35E3_LC | . | . | . | . | . | . | . | . | . | . | . | . | . | . | . | D | . | A | . |
| 21-225_50G4_LC | . | . | . | . | N | L | . | . | . | . | . | . | . | . | . | . | . | . | . |
| 21-225_5E6_LC | . | . | . | . | . | . | . | . | . | . | . | . | . | . | . | . | . | . | . |
| 21-225_6G7_LC | . | . | . | . | V | . | . | . | . | . | . | . | . | . | . | . | . | . | . |

FIGURE 57

Table 118.    Consensus 38 - VL1|1b/JL2 (SEQ ID NO: 50335):

QSVLTQP-PSVSAAPGQKVTISC<u>SGSS-SNIGN</u>-----NYVSWYQQLPGTAPKLLIY<u>D</u>--------<u>NNKRPS</u>GIPDRFSGSKSG--
TSATLGITGLQTGDEADYYC<u>GTWDSS</u>--------------------LSVGVFGGGTKLTVLG wherein:

I at position 31 can be substituted with L

N at position 33 can be substituted with S

N at position 39 can be substituted with H or K

Y at position 40 can be substituted with F

V at position 41 can be substituted with L

N at position 67 can be substituted with S

N at position 68 can be substituted with Y or S

T at position 108 can be substituted with A or I

S at position 111 can be substituted with G, I or R

S at position 112 can be substituted with R

S at position 135 can be substituted with N

V at position 136 can be substituted with A or T

G at position 137 can be substituted with V or M

Ser Gly Ser Ser Ser Asn Xaa Gly Xaa Xaa Xaa Xaa Ser (SEQ ID NO: 50189)

Asp Xaa Xaa Lys Arg Pro Ser (SEQ ID NO: 50190)

Gly Xaa Trp Asp Xaa Xaa Leu Xaa Xaa Xaa Val (SEQ ID NO: 50191)

Ser Gly Ser Ser Ser Asn Ile Gly Asn Asn Tyr Val Ser (SEQ ID NO: 50675)

**FIGURE 57**

Asp Asn Asn Lys Arg Pro Ser (SEQ ID NO: 50676)

Gly Thr Trp Asp Ser Ser Leu Ser Val Gly Val (SEQ ID NO: 50677)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | L_FR1 | | | | | | | | | | | | | | |
| CONSENSUS | Q | S | V | L | T | Q | P | - | P | S | V | S | A | A | P | G | Q | K | V | T | I | S | C | S | G | S |
| 21-225_190B10_LC | . | . | . | . | . | . | . | . | . | . | . | . | . | . | . | . | . | . | . | . | . | . | . | . | . | . |
| 21-225_190C3_LC | . | . | . | . | . | . | . | . | . | . | . | . | . | . | . | . | . | . | . | . | . | . | . | . | . | . |
| 21-225_190D10_LC | . | . | . | . | . | . | . | . | . | . | . | . | . | . | . | . | . | . | . | . | . | . | . | . | . | . |
| 21-225_190D8_LC | . | . | . | . | . | . | . | . | . | . | . | . | . | . | . | . | . | . | . | . | . | . | . | . | . | . |
| 21-225_190H7_LC | . | . | . | . | . | . | . | . | . | . | . | . | . | . | . | . | . | . | . | . | . | . | . | . | . | . |
| 21-225_191A1_LC | . | . | . | . | . | . | . | . | . | . | . | . | . | . | . | . | . | R | . | . | . | . | . | . | . | . |
| 21-225_192G2_LC | . | . | . | . | . | . | . | . | . | . | . | . | . | . | . | . | . | . | . | . | . | . | . | . | . | . |
| 21-225_194F11_LC | . | . | . | . | . | . | . | . | . | . | . | . | . | . | . | . | . | . | . | . | . | . | . | . | . | . |
| 21-225_194G12_LC | . | . | . | . | . | . | . | . | . | . | . | . | . | . | . | . | . | . | . | . | . | . | . | . | . | . |
| 21-225_197E8_LC | . | . | . | . | . | . | . | . | . | . | . | . | . | . | . | . | . | . | . | . | . | . | . | . | . | . |
| 21-225_198D2_LC | . | . | . | . | . | . | . | . | . | . | . | . | . | . | . | . | . | . | . | . | . | . | . | . | . | . |
| 21-225_200G8_LC | . | . | . | . | . | . | . | . | . | . | . | . | . | . | . | . | . | R | . | . | . | . | . | . | . | . |
| 21-225_209A8_LC | . | . | . | . | . | . | . | . | . | . | . | . | . | . | . | . | . | . | . | . | . | . | . | . | . | . |
| 21-225_214D8_LC | . | . | . | . | . | . | . | . | . | . | . | . | . | . | . | . | . | . | . | . | . | . | . | . | . | . |
| 21-225_218G4_LC | . | . | . | . | . | . | . | . | . | . | . | . | . | . | . | . | . | . | . | . | . | . | . | . | . | . |
| 21-225_58C5_LC | . | . | . | . | . | . | . | . | . | . | . | . | . | . | . | . | . | . | . | . | . | . | . | . | . | . |

## FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | L_CDR1 | | | | | | | | | | L_FR2 | | | | | | | | | |
| CONSENSUS | S | - | S | N | I | G | N | - | - | - | - | - | N | Y | V | S | W | Y | Q | Q | L | P | G | T | A | P |
| 21-225_190B10_LC | . | | | | . | . | . | . | . | . | . | . | . | . | . | . | . | . | . | ~ | . | . | . | . | . | . |
| 21-225_190C3_LC | . | | | | . | . | . | . | . | . | . | . | . | . | . | . | . | . | . | . | . | . | . | . | . | . |
| 21-225_190D10_LC | . | | | . | . | . | S | . | . | . | . | . | . | . | . | . | . | . | . | . | . | . | . | . | V | . |
| 21-225_190D8_LC | . | | | | . | . | . | . | . | . | . | . | ~ | . | . | . | . | . | . | . | . | . | . | . | . | . |
| 21-225_190H7_LC | . | | | | . | . | S | . | . | . | . | . | . | . | . | . | . | . | . | . | . | . | . | . | . | . |
| 21-225_191A1_LC | . | | | . | L | . | . | . | . | . | . | . | ~ | . | . | . | . | . | . | . | ~ | . | . | . | . | . |
| 21-225_192G2_LC | . | | | | . | . | . | . | . | . | . | . | . | . | . | . | . | . | . | . | . | . | . | . | . | . |
| 21-225_194F11_LC | . | | | | . | . | . | . | . | . | . | . | . | . | . | . | . | . | . | . | . | . | . | . | . | . |
| 21-225_194G12_LC | . | | | | . | . | . | . | . | . | . | . | . | . | . | . | . | . | . | . | . | . | . | . | . | . |
| 21-225_197E8_LC | . | | | | . | . | . | . | . | . | . | . | ~ | . | . | . | . | . | . | . | ~ | . | . | . | . | . |
| 21-225_198D2_LC | . | | | | . | . | S | . | . | . | . | . | . | . | . | . | . | . | . | . | . | . | . | . | . | . |
| 21-225_200G8_LC | . | | | . | L | . | . | . | . | . | . | . | . | ~ | . | . | . | . | . | . | ~ | . | . | . | . | . |
| 21-225_209A8_LC | . | | | | . | . | S | . | . | . | . | . | . | ~ | L | . | . | . | . | . | . | . | . | . | . | . |
| 21-225_214D8_LC | . | | | | . | . | . | . | . | . | . | . | . | . | . | . | . | . | . | . | ~ | . | . | . | . | . |
| 21-225_218G4_LC | . | | | | . | . | . | . | . | . | . | . | . | . | . | . | . | . | . | . | . | . | . | . | . | . |
| 21-225_58C5_LC | . | | | | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | L_CDR2 | | | | | | | | | | | | | |
| CONSENSUS | K | L | L | I | Y | D | - | - | - | - | - | - | - | - | N | N | K | R | P | S | G | I | P | D | R | F |
| 21-225_190B10_LC | | | | | | | | | | | | | | | | | | | | | | | | | | |
| 21-225_190C3_LC | | | | L | | | | | | | | | | | S | | | | | | | | | G | | |
| 21-225_190D10_LC | | V | | | | | | | | | | | | | | | | | | | | | | | | |
| 21-225_190D8_LC | | | | | | | | | | | | | | | S | S | | | | | E | | | | | |
| 21-225_190H7_LC | | V | | | | | | | | | | | | | | Y | | | | | | | | | | |
| 21-225_191A1_LC | | | | | | | | | | | | | | | | Y | | | | | | | | | | |
| 21-225_192G2_LC | | | | | | | | | | | | | | | | | | | | | R | | | | | |
| 21-225_194F11_LC | | | | | | | | | | | | | | | | | | | | | | | | | | |
| 21-225_194G12_LC | | | | | | | | | | | | | | | | | | | | | R | | | | | |
| 21-225_197E8_LC | | | | | | | | | | | | | | | | | | | | | K | | | | | |
| 21-225_198D2_LC | | V | | | | | | | | | | | | | | | | | | | | | | | | |
| 21-225_200G8_LC | | | | | | | | | | | | | | | | Y | | | | | | | | | | |
| 21-225_209A8_LC | | | | | | | | | | | | | | | | | | | | | | | | | | |
| 21-225_214D8_LC | | | | | N | | | | | | | | | | | | | | | | | | | | | |
| 21-225_218G4_LC | | | | L | | | | | | | | | | | S | | | | | | | | | A | | |
| 21-225_58C5_LC | | | | | | | | | | | | | | | | | | | | | | | | | | |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | L_FR3 | | | | | | | | | | | | | | |
| CONSENSUS | S | G | S | K | S | G | - | - | T | S | A | T | L | G | I | T | G | L | Q | T | G | D | E | A | D | Y |
| 21-225_190B10_LC | . | | | | . | | . | . | | | | | | | | . | | | . | | . | . | . | | | |
| 21-225_190C3_LC | . | | | . | | . | . | | | | | | | | . | | | | . | . | . | . | . | . | . | . |
| 21-225_190D10_LC | . | | . | | | . | . | | | | | | | | . | | . | | . | . | . | . | . | . | . | . |
| 21-225_190D8_LC | . | | . | | | . | . | | | | | | | | . | | . | | . | . | . | . | . | . | . | |
| 21-225_190H7_LC | . | . | | | | . | . | | | | | | | | . | | . | | . | . | . | . | . | . | | |
| 21-225_191A1_LC | . | V | . | | | . | . | | | | | | | | . | | . | | . | . | . | . | . | . | . | . |
| 21-225_192G2_LC | . | | . | | | . | . | | | | | | | | . | | . | | . | . | . | . | . | . | . | . |
| 21-225_194F11_LC | . | | . | | | . | . | | | | | | | | . | | . | | . | . | . | . | . | . | . | . |
| 21-225_194G12_LC | . | | . | | | . | . | | | | | | | | . | | . | | . | | ° | . | . | . | . | . |
| 21-225_197E8_LC | . | | . | | | . | . | | | | | | | | . | | . | | L | . | . | . | . | . | . | . |
| 21-225_198D2_LC | . | | . | | | . | . | | | | | | | | . | | . | | . | . | . | . | . | . | . | . |
| 21-225_200G8_LC | . | V | . | | | . | . | | | | | | | | . | | . | | . | . | . | . | . | . | . | . |
| 21-225_209A8_LC | . | | . | | | . | . | | | | | | | | . | | . | | . | . | . | . | . | . | . | . |
| 21-225_214D8_LC | . | | . | | | . | . | | | | | | | | . | | . | | . | . | . | . | . | . | . | . |
| 21-225_218G4_LC | . | | . | | | . | . | | | | | | | | . | | . | | . | . | . | . | . | . | . | . |
| 21-225_58C5_LC | . | | . | | | . | . | | | | | | | | . | | . | | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | L_CDR3 | | | | | | | | |
| CONSENSUS | Y | C | G | T | W | D | S | S | - | - | - | - | - | - | - | - | - | - | - | - | - | - | - | - | - | - |
| 21-225_190B10_LC | . | . | . | . | . | . | . | . | . | . | . | . | . | . | . | . | . | . | . | . | . | . | . | . | . | . |
| 21-225_190C3_LC | . | . | . | . | . | . | . | . | . | . | . | . | . | . | . | . | . | . | . | . | . | . | . | . | . | . |
| 21-225_190D10_LC | . | . | . | . | . | . | . | ※ | . | . | . | . | . | . | . | . | . | . | . | . | . | . | . | . | . | . |
| 21-225_190D8_LC | . | . | . | . | . | . | . | . | . | . | . | . | . | . | . | . | . | . | . | . | . | . | . | . | . | . |
| 21-225_190H7_LC | . | . | . | . | . | . | ※ | ※ | . | . | . | . | . | . | . | . | . | . | . | . | . | . | . | . | . | . |
| 21-225_191A1_LC | . | . | . | . | . | . | . | . | . | . | . | . | . | . | . | . | . | . | . | . | . | . | . | . | . | . |
| 21-225_192G2_LC | . | . | . | . | . | . | . | . | . | . | . | . | . | . | . | . | . | . | . | . | . | . | . | . | . | . |
| 21-225_194F11_LC | . | . | . | . | . | . | . | . | . | . | . | . | . | . | . | . | . | . | . | . | . | . | . | . | . | . |
| 21-225_194G12_LC | . | . | . | . | . | . | . | . | . | . | . | . | . | . | . | . | . | . | . | . | . | . | . | . | . | . |
| 21-225_197E8_LC | . | . | ¦ | . | . | . | ※ | . | . | . | . | . | . | . | . | . | . | . | . | . | . | . | . | . | . | . |
| 21-225_198D2_LC | . | . | . | . | . | . | . | ※ | . | . | . | . | . | . | . | . | . | . | . | . | . | . | . | . | . | . |
| 21-225_200G8_LC | . | . | . | . | . | . | . | . | . | . | . | . | . | . | . | . | . | . | . | . | . | . | . | . | . | . |
| 21-225_209A8_LC | . | . | . | . | . | . | . | . | . | . | . | . | . | . | . | . | . | . | . | . | . | . | . | . | . | . |
| 21-225_214D8_LC | . | . | A | . | . | . | . | . | . | . | . | . | . | . | . | . | . | . | . | . | . | . | . | . | . | . |
| 21-225_218G4_LC | . | . | . | . | . | . | . | . | . | . | . | . | . | . | . | . | . | . | . | . | . | . | . | . | . | . |
| 21-225_58C5_LC | . | . | . | . | . | . | ¦ | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | L_FR4 | | | | | | |
| CONSENSUS | - | - | - | L | S | V | G | V | F | G | G | G | T | K | L | T | V | L | G |
| 21-225_190B10_LC | . | . | . | . | . | A | . | . | . | . | . | . | . | . | . | . | . | . | . |
| 21-225_190C3_LC | . | . | . | . | . | T | V | . | . | . | . | . | . | . | . | . | . | . | . |
| 21-225_190D10_LC | . | . | . | . | N | . | . | . | . | . | . | . | . | . | . | . | . | . | . |
| 21-225_190D8_LC | . | . | . | . | . | T | V | . | . | . | . | . | . | . | . | . | . | . | . |
| 21-225_190H7_LC | . | . | . | . | N | . | . | . | . | . | . | . | . | . | . | . | . | . | . |
| 21-225_191A1_LC | . | . | . | . | N | T | . | . | . | . | . | . | . | . | . | . | . | . | . |
| 21-225_192G2_LC | . | . | . | . | . | A | . | . | . | . | . | . | . | . | . | . | . | . | . |
| 21-225_194F11_LC | . | . | . | . | . | A | . | . | . | . | . | . | . | . | . | . | . | . | . |
| 21-225_194G12_LC | . | . | . | . | . | . | . | . | . | . | . | . | . | . | . | . | . | . | . |
| 21-225_197E8_LC | . | . | . | . | . | . | M | . | . | . | . | . | . | . | . | . | . | . | . |
| 21-225_198D2_LC | . | . | . | . | N | . | . | . | . | . | . | . | . | . | . | . | . | . | . |
| 21-225_200G8_LC | . | . | . | . | N | T | . | . | . | . | . | . | . | . | . | . | . | . | . |
| 21-225_209A8_LC | . | . | . | . | . | A | . | . | . | . | . | . | . | . | . | . | . | . | . |
| 21-225_214D8_LC | . | . | . | . | . | A | V | . | I | . | . | . | S | . | . | . | . | . | . |
| 21-225_218G4_LC | . | . | . | . | N | T | V | . | . | . | . | . | . | . | . | . | . | . | S |
| 21-225_58C5_LC | . | . | . | . | . | . | . | . | . | . | . | . | . | . | . | . | . | . | . |

4966

**FIGURE 57**

Table 119.    Consensus 39 - VK2|A19/JK4 (SEQ ID NO: 50336):

DIVMTQSPLSLPVTPGEPASISC<u>RSS--QSLLIISN-GYNYLD</u>WYLQKPGQSPQLLIY<u>L-------GSNRAS</u>GVPDRFSGSGSG--
TDFTLKISRVEAEDVGVYYC<u>MQALH---------------------PPLT</u>FGGGTKVEIKR wherein:

S at position 25 can be substituted with Y

L at position 32 can be substituted with V

H at position 33 can be substituted with Y

S at position 34 can be substituted with N or H

N at position 35 can be substituted with S

G at position 37 can be substituted with K or R

Y at position 38 can be substituted with H or N

Y at position 40 can be substituted with H or S

L at position 58 can be substituted with V

N at position 69 can be substituted with H

A at position 109 can be substituted with P, T or V

H at position 111 can be substituted with Q or null (-)

Null (-) at position 134 can be substituted with T

P at position 135 can be substituted with Q, T or I

P at position 136 can be substituted with T

L at position 137 can be substituted with P or F

Arg Xaa Ser Gln Ser Leu Xaa Xaa Xaa Xaa Xaa Xaa Asn Xaa Leu Asp (SEQ ID NO: 50243)

FIGURE 57

Xaa Gly Ser Xaa Arg Ala Ser (SEQ ID NO: 50244)

Met Gln Xaa Leu Xaa Xaa Xaa Xaa Xaa Thr (SEQ ID NO: 50245)

Arg Ser Ser Gln Ser Leu Leu His Ser Asn Gly Tyr Asn Tyr Leu Asp (SEQ ID NO: 50678)

Leu Gly Ser Asn Arg Ala Ser (SEQ ID NO: 50679)

Met Gln Ala Leu His Pro Pro Leu Thr (SEQ ID NO: 50680)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | K_FR1 | | | | | | | | | | | | | |
| CONSENSUS | D | I | V | M | T | Q | S | P | L | S | L | P | V | T | P | G | E | P | A | S | I | S | C | R | S | S |
| 21-225_171D7_LC | . | . | . | . | . | . | . | . | . | . | . | . | . | . | . | . | . | . | . | . | . | . | . | . | . | . |
| 21-225_17B8_LC | . | . | . | . | . | . | . | . | . | . | . | . | . | . | . | . | . | . | . | . | . | . | . | . | . | . |
| 21-225_201F3_LC | . | . | . | . | . | . | . | . | . | . | . | . | . | . | . | . | . | . | . | . | . | . | . | . | . | . |
| 21-225_201G6_LC | . | . | . | . | . | . | . | . | . | . | . | . | . | . | . | . | . | . | . | . | . | . | . | . | . | . |
| 21-225_202C12_LC | . | . | . | L | . | . | . | . | . | . | . | . | . | . | . | . | . | . | . | . | . | . | . | . | . | . |
| 21-225_208G3_LC | . | . | . | L | . | . | . | . | . | . | . | . | . | . | . | . | . | . | . | . | . | . | . | . | . | . |
| 21-225_74A2_LC | . | . | . | . | . | . | . | . | . | . | . | . | . | . | . | . | . | . | . | . | . | . | . | . | . | . |
| 21-225_74D8_LC | . | . | . | . | . | . | . | . | . | . | . | . | . | . | . | . | . | . | . | . | . | . | . | . | . | . |
| 21-225_75A1_LC | . | . | . | . | . | . | . | . | . | . | . | . | . | . | . | . | . | . | . | . | . | . | . | . | . | . |
| 21-225_75D8_LC | . | . | . | . | . | . | . | . | . | . | . | . | . | . | . | . | . | . | . | . | . | . | . | . | . | . |
| 21-225_89G4_LC | . | . | . | . | . | . | . | . | . | . | . | . | . | . | . | . | . | . | . | . | . | . | . | . | . | . |
| 21-225_90C11_LC | . | . | . | . | . | . | . | . | . | . | . | . | . | . | . | . | . | . | . | . | . | . | . | . | Y | . |
| 21-225_92B2_LC | . | . | . | . | . | . | . | . | . | . | . | . | . | . | . | . | . | . | . | . | . | . | . | . | . | . |
| 21-225_97H3_LC | . | . | . | I | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | L | L | H | S | N | - | G | Y | N | Y | L | D | W | Y | L | Q | K | P | G | Q | S | P |
| 21-225_171D7_LC | . | . | . | . | . | . | Y | H | . | . | . | . | . | . | . | . | . | . | . | . | . | T | . | . | . | . |
| 21-225_17B8_LC | . | . | . | . | . | . | . | . | . | . | . | N | . | S | . | . | . | . | . | . | . | . | . | . | . | . |
| 21-225_201F3_LC | . | . | . | . | . | . | . | N | . | . | H | . | . | H | . | . | . | . | . | . | . | . | . | . | . | . |
| 21-225_201G6_LC | . | . | . | . | . | . | . | N | . | . | H | . | . | H | . | . | . | . | . | . | . | . | . | . | . | . |
| 21-225_202C12_LC | . | . | . | . | . | . | . | N | . | . | H | . | . | H | . | . | . | . | . | . | . | . | . | . | . | . |
| 21-225_208G3_LC | . | . | . | . | . | . | . | N | . | . | H | . | . | H | . | . | . | . | . | . | . | . | . | . | . | . |
| 21-225_74A2_LC | . | . | . | . | . | . | . | . | . | . | . | H | . | . | . | . | . | . | . | . | . | . | . | H | . | . |
| 21-225_74D8_LC | . | . | . | . | . | V | . | . | S | . | . | . | . | . | . | . | . | . | . | . | . | . | . | . | . | . |
| 21-225_75A1_LC | . | . | . | . | . | . | . | . | . | . | . | H | . | . | . | . | . | . | . | . | . | . | . | H | . | . |
| 21-225_75D8_LC | . | . | . | . | . | . | . | . | S | . | . | . | . | . | . | . | . | . | . | . | . | . | . | . | . | . |
| 21-225_89G4_LC | . | . | . | . | . | . | . | . | S | . | . | . | . | . | . | . | . | . | . | . | . | . | . | . | . | . |
| 21-225_90C11_LC | . | . | . | . | . | V | . | . | S | . | . | . | . | . | . | . | . | . | . | . | . | . | . | . | . | . |
| 21-225_92B2_LC | . | . | . | . | . | V | . | . | S | . | . | . | . | . | . | . | . | . | . | . | . | . | . | . | . | . |
| 21-225_97H3_LC | . | . | . | . | . | . | . | . | . | . | . | H | . | . | . | . | . | . | . | . | . | . | . | H | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | Q | L | L | I | Y | L | - | - | - | - | - | - | - | - | G | S | N | R | A | S | G | V | P | D | R | F |
| 21-225_171D7_LC | . | . | . | . | . | V | . | . | . | . | . | . | . | . | . | . | . | . | . | . | . | . | . | . | . | . |
| 21-225_17B8_LC | . | . | . | . | . | . | . | . | . | . | . | . | . | . | . | . | ß | . | . | . | . | . | . | . | . | . |
| 21-225_201F3_LC | . | . | . | . | . | . | . | . | . | . | . | . | . | . | . | . | . | . | . | . | . | . | . | . | . | . |
| 21-225_201G6_LC | . | . | . | . | . | . | . | . | . | . | . | . | . | . | . | . | . | . | . | . | . | . | . | . | . | . |
| 21-225_202C12_LC | . | . | . | . | . | . | . | . | . | . | . | . | . | . | . | . | . | . | . | . | . | . | . | . | . | . |
| 21-225_208G3_LC | . | . | . | . | . | . | . | . | . | . | . | . | . | . | . | . | . | . | . | . | . | . | . | . | . | . |
| 21-225_74A2_LC | . | . | . | . | . | . | . | . | . | . | . | . | . | . | . | . | . | . | . | . | . | . | . | ß | . | . |
| 21-225_74D8_LC | . | . | V | . | . | . | . | . | . | . | . | . | . | . | . | . | . | . | . | . | . | . | . | . | . | . |
| 21-225_75A1_LC | . | . | . | . | . | . | . | . | . | . | . | . | . | . | . | . | . | . | . | . | . | . | . | ß | . | . |
| 21-225_75D8_LC | . | . | . | . | . | . | . | . | . | . | . | . | . | . | . | . | . | . | . | . | . | . | . | . | . | . |
| 21-225_89G4_LC | . | . | . | . | . | . | . | . | . | . | . | . | . | . | . | . | . | . | . | . | . | . | . | . | . | . |
| 21-225_90C11_LC | . | . | V | . | . | . | . | . | . | . | . | . | . | . | . | . | . | . | . | . | . | . | . | . | . | . |
| 21-225_92B2_LC | . | . | V | . | . | . | . | . | . | . | . | . | . | . | . | . | . | . | . | . | . | . | . | . | . | . |
| 21-225_97H3_LC | . | . | . | . | . | . | . | . | . | . | . | . | . | . | . | . | . | . | . | . | . | . | . | ß | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | K | I | S | R | V | E | A | E | D | V | G | V | Y |
| 21-225_171D7_LC | . | . | . | . | . | . | . | . | . | . | . | . | . | . | . | . | . | . | . | . | . | . | . | . | . | . |
| 21-225_17B8_LC | . | Q | . | . | . | . | . | . | . | . | . | . | . | . | . | . | . | . | . | . | . | . | . | . | . | . |
| 21-225_201F3_LC | . | . | . | . | . | . | . | . | . | . | . | . | . | . | . | . | . | . | . | . | . | . | . | . | . | . |
| 21-225_201G6_LC | . | . | . | . | . | . | . | . | . | . | . | . | . | . | . | . | . | . | . | . | . | . | . | . | . | . |
| 21-225_202C12_LC | . | . | . | . | . | . | . | . | . | . | . | . | . | . | . | . | . | . | . | . | . | . | . | . | . | . |
| 21-225_208G3_LC | . | . | . | . | . | . | . | . | . | . | . | . | . | . | . | . | . | . | . | . | . | . | . | . | . | . |
| 21-225_74A2_LC | . | . | . | . | . | . | . | . | . | . | . | . | . | . | . | . | . | . | . | . | . | . | . | . | . | . |
| 21-225_74D8_LC | . | . | . | . | . | . | . | . | S | . | . | . | . | . | . | . | . | . | . | . | . | . | . | . | L | . |
| 21-225_75A1_LC | . | . | . | . | . | . | . | . | . | . | . | . | . | . | . | . | . | . | . | . | . | . | . | . | . | . |
| 21-225_75D8_LC | . | . | . | . | . | . | . | . | S | . | . | . | . | . | . | . | . | . | . | . | . | . | . | . | L | . |
| 21-225_89G4_LC | . | . | . | . | . | . | . | . | . | . | . | . | . | . | . | . | . | . | . | . | . | . | I | . | . | . |
| 21-225_90C11_LC | . | . | . | . | . | . | . | . | S | . | . | . | . | . | . | . | . | . | . | . | . | . | . | . | L | . |
| 21-225_92B2_LC | . | . | . | . | . | V | . | . | . | . | . | . | . | R | . | . | . | . | . | . | . | . | I | . | I | . |
| 21-225_97H3_LC | . | . | . | . | . | . | . | . | . | . | . | . | . | . | . | . | . | . | . | . | . | . | . | . | . | . |

EP 4 435 105 A2

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | M | Q | A | L | - | - | - | - | - | - | - | - | - | - | - | - | - | - | - | - | - | - | - | - |
| 21-225_171D7_LC | . | . | . | . | T | . | Q | . | . | . | . | . | . | . | . | . | . | . | . | . | . | . | . | . | . | . |
| 21-225_17B8_LC | . | . | . | . | V | . | Q | . | . | . | . | . | . | . | . | . | . | . | . | . | . | . | . | . | . | . |
| 21-225_201F3_LC | . | . | . | . | . | . | - | . | . | . | . | . | . | . | . | . | . | . | . | . | . | . | . | . | . | . |
| 21-225_201G6_LC | . | . | . | . | . | . | - | . | . | . | . | . | . | . | . | . | . | . | . | . | . | . | . | . | . | . |
| 21-225_202C12_LC | . | . | . | . | . | . | - | . | . | . | . | . | . | . | . | . | . | . | . | . | . | . | . | . | . | . |
| 21-225_208G3_LC | . | . | . | . | . | . | - | . | . | . | . | . | . | . | . | . | . | . | . | . | . | . | . | . | . | . |
| 21-225_74A2_LC | . | . | . | . | P | . | Q | . | . | . | . | . | . | . | . | . | . | . | . | . | . | . | . | . | . | . |
| 21-225_74D8_LC | . | . | . | . | . | . | H | . | . | . | . | . | . | . | . | . | . | . | . | . | . | . | . | . | . | . |
| 21-225_75A1_LC | . | . | . | . | P | . | Q | . | . | . | . | . | . | . | . | . | . | . | . | . | . | . | . | . | . | . |
| 21-225_75D8_LC | . | . | . | . | . | . | H | . | . | . | . | . | . | . | . | . | . | . | . | . | . | . | . | . | . | . |
| 21-225_89G4_LC | . | . | . | . | . | . | H | . | . | . | . | . | . | . | . | . | . | . | . | . | . | . | . | . | . | . |
| 21-225_90C11_LC | . | . | . | . | . | . | H | . | . | . | . | . | . | . | . | . | . | . | . | . | . | . | . | . | . | . |
| 21-225_92B2_LC | . | . | . | . | . | . | H | . | . | . | . | . | . | . | . | . | . | . | . | . | . | . | . | . | . | . |
| 21-225_97H3_LC | . | . | . | . | P | . | Q | . | . | . | . | . | . | . | . | . | . | . | . | . | . | . | . | . | . | . |

4972

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | P | L | T | F | G | G | G | T | K | V | E | I | K | R | |
| 21-225_171D7_LC | . | . | . | . | T | . | . | . | . | . | . | . | . | . | . | . | . | . | . |
| 21-225_17B8_LC | . | . | . | T | P | . | . | . | . | . | . | . | . | . | . | . | . | . | . |
| 21-225_201F3_LC | . | . | . | . | Q | T | P | . | . | . | . | . | . | . | . | . | . | . | G |
| 21-225_201G6_LC | . | . | . | . | Q | T | P | . | . | . | . | . | . | . | . | . | . | . | G |
| 21-225_202C12_LC | . | . | . | . | Q | T | P | . | . | . | . | . | . | . | . | . | . | . | . |
| 21-225_208G3_LC | . | . | . | . | Q | T | P | . | . | . | . | . | . | . | . | . | . | . | . |
| 21-225_74A2_LC | . | . | . | . | T | . | P | . | . | . | . | . | . | . | . | . | . | . | . |
| 21-225_74D8_LC | . | . | . | . | P | . | . | . | . | . | . | . | . | . | . | . | . | . | . |
| 21-225_75A1_LC | . | . | . | . | T | . | P | . | . | . | . | . | . | . | . | . | . | . | . |
| 21-225_75D8_LC | . | . | . | . | P | . | . | . | . | . | . | . | . | . | . | . | . | . | . |
| 21-225_89G4_LC | . | . | . | . | I | . | . | . | . | . | . | . | . | . | . | . | . | . | . |
| 21-225_90C11_LC | . | . | . | . | P | . | . | . | . | . | . | . | . | . | . | . | . | . | . |
| 21-225_92B2_LC | . | . | . | . | I | . | . | . | . | . | . | . | . | . | . | . | . | . | . |
| 21-225_97H3_LC | . | . | . | . | T | . | P | . | . | . | . | . | . | . | . | . | . | . | . |

FIGURE 57

Table 120.    Consensus 40 - VL1|1c/JL2 (SEQ ID NO: 50337):

QSVLTQP-PSASGTPGQRVTISC<u>SGSS-SNIGS</u>-----NTVNWYQQLPGTAPKLLIY<u>S</u>--------<u>NNQRPSG</u>VPDRFSGSKSG--
TSASLAISGLQSEDEADYYC<u>AAWDDS</u>--------------------LNGVVFGGGTKLTVLG wherein:

S at position 26 can be substituted with T

S at position 27 can be substituted with N

N at position 30 can be substituted with Y

S at position 33 can be substituted with N

N at position 39 can be substituted with Y

T at position 40 can be substituted with A or S

V at position 41 can be substituted with I

N at position 42 can be substituted with D or S

S at position 58 can be substituted with I

N at position 67 can be substituted with S

N at position 68 can be substituted with D or S

Q at position 69 can be substituted with H

A at position 107 can be substituted with E

S at position 112 can be substituted with null (-)

null (-) at position 133 can be substituted with L

L at position 134 can be substituted with N, M or S

N at position 135 can be substituted with G, K or L

**FIGURE 57**

G at position 136 can be substituted with H or N

V at position 137 can be substituted with P or G

V at position 138 can be substituted with P

Ser Gly Xaa Xaa Ser Xaa Ile Gly Xaa Xaa Xaa Xaa Xaa (SEQ ID NO: 50192)

Xaa Xaa Xaa Xaa Arg Pro Ser (SEQ ID NO: 50193)

Xaa Ala Trp Asp Asp Xaa Xaa Xaa Xaa Xaa Xaa Xaa (SEQ ID NO: 50194)

Ser Gly Ser Ser Ser Asn Ile Gly Ser Asn Thr Val Asn (SEQ ID NO: 50681)

Ser Asn Asn Gln Arg Pro Ser (SEQ ID NO: 50682)

Ala Ala Trp Asp Asp Ser Leu Asn Gly Val Val (SEQ ID NO: 50683)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | L_FR1 | | | | | | | | | | | | | |
| CONSENSUS | Q | S | V | L | T | Q | P | - | P | S | A | S | G | T | P | G | Q | R | V | T | I | S | C | S | G | S |
| 21-225_146A8_LC | . | | | | . | . | . | . | . | . | . | . | . | . | . | . | . | . | . | . | . | . | . | . | . | . |
| 21-225_146B6_LC | . | | | . | . | . | . | . | . | . | . | . | . | . | . | . | . | . | . | . | . | . | . | . | . | . |
| 21-225_146D8_LC | . | | . | . | . | . | . | . | . | T | . | . | . | . | . | . | . | . | . | . | . | . | . | . | . | . |
| 21-225_147H12_LC | . | | . | . | . | . | . | . | . | . | . | . | . | . | . | . | . | . | . | . | . | . | . | . | . | . |
| 21-225_149A1_LC | . | | . | . | . | . | . | . | . | . | . | . | . | . | . | . | . | . | . | . | . | . | . | . | . | . |
| 21-225_150F9_LC | . | | . | . | . | . | . | . | . | . | . | . | . | . | . | . | . | . | . | . | . | . | . | . | . | . |
| 21-225_154G12_LC | . | | . | . | . | . | . | . | . | . | . | . | . | . | . | . | . | . | . | . | . | . | . | . | . | . |
| 21-225_156H2_LC | . | . | . | V | . | . | . | . | . | . | . | . | . | . | . | . | . | . | . | . | . | . | . | . | . | . |
| 21-225_197F9_LC | . | | . | . | . | . | . | . | . | . | . | . | . | . | . | . | . | . | . | . | . | . | . | . | . | . |
| 21-225_199C3_LC | . | | . | . | . | . | . | . | . | . | . | . | . | . | . | . | . | . | . | . | . | . | . | . | . | . |
| 21-225_4G12_LC | . | | . | . | . | . | . | . | . | . | . | . | . | . | . | . | . | . | . | N | M | . | . | . | . | T |
| 21-225_60D6_LC | . | | . | . | . | . | . | . | . | . | . | . | . | . | . | . | . | . | . | . | . | . | . | . | . | . |
| 21-225_9G9_LC | . | | . | . | . | . | . | . | . | . | . | . | . | . | . | . | . | . | . | N | M | . | . | . | . | T |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | L_CDR1 | | | | | | | | | | | | | | | | | L_FR2 | | | |
| CONSENSUS | S | - | S | N | I | G | S | - | - | - | - | - | N | T | V | N | W | Y | Q | Q | L | P | G | T | A | P |
| 21-225_146A8_LC | . | | | | | . | . | . | . | . | . | . | . | S | I | . | . | . | . | . | . | . | R | . | . | . |
| 21-225_146B6_LC | | | . | | | . | . | . | . | . | . | . | . | A | . | . | . | . | . | . | . | . | . | . | . | . |
| 21-225_146D8_LC | . | | . | Y | . | . | . | . | . | . | . | . | . | . | . | O | . | . | . | . | . | . | . | . | . | . |
| 21-225_147H12_LC | | | . | . | . | . | . | . | . | . | . | . | Y | A | . | . | . | . | . | . | . | . | . | . | . | . |
| 21-225_149A1_LC | . | | | | . | . | . | . | . | . | . | . | . | A | . | . | . | . | . | . | . | . | . | . | . | . |
| 21-225_150F9_LC | . | | | | . | . | . | . | . | . | . | . | . | A | . | . | . | . | . | . | . | . | . | . | . | . |
| 21-225_154G12_LC | | | . | . | . | . | N | . | . | . | . | . | . | A | . | S | . | . | . | . | . | . | . | . | . | . |
| 21-225_156H2_LC | . | | . | . | . | . | . | . | . | . | . | . | . | . | . | . | . | . | . | . | . | . | . | . | . | . |
| 21-225_197F9_LC | . | | . | . | . | . | . | . | . | . | . | . | . | . | . | . | . | . | . | . | . | . | . | . | . | . |
| 21-225_199C3_LC | . | | . | . | . | . | . | . | . | . | . | . | . | . | . | . | . | . | . | . | . | . | . | . | . | . |
| 21-225_4G12_LC | N | | . | . | . | . | . | . | . | . | . | . | Y | . | . | . | . | . | . | . | . | . | . | . | . | . |
| 21-225_60D6_LC | . | | . | . | . | . | . | . | . | . | . | . | . | . | . | . | . | . | . | . | . | . | . | . | . | . |
| 21-225_9G9_LC | N | | . | . | . | . | . | . | . | . | . | . | Y | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | L_CDR2 | | | | | | | | | | | | | | |
| CONSENSUS | K | L | L | I | Y | S | - | - | - | - | - | - | - | - | N | N | Q | R | P | S | G | V | P | D | R | F |
| 21-225_146A8_LC | . | | | | . | | . | . | . | . | . | . | . | | | O | . | . | . | . | . | . | . | . | . | . |
| 21-225_146B6_LC | . | | | | . | | . | . | . | . | . | . | . | . | . | . | . | . | . | . | . | . | . | . | . | . |
| 21-225_146D8_LC | . | | | | . | | . | . | . | . | . | . | . | . | . | . | . | . | . | . | . | . | . | . | . | . |
| 21-225_147H12_LC | . | | | | . | | . | . | . | . | . | . | . | . | . | . | H | . | . | . | . | . | . | . | . | . |
| 21-225_149A1_LC | . | | | | . | | . | . | . | . | . | . | . | . | . | . | . | . | . | . | . | . | . | . | . | . |
| 21-225_150F9_LC | . | | | | . | | . | . | . | . | . | . | . | . | S | . | . | . | . | . | . | . | . | . | . | . |
| 21-225_154G12_LC | . | | | | . | | . | . | . | . | . | . | . | . | . | O | H | . | . | . | . | . | . | . | . | . |
| 21-225_156H2_LC | . | | | | . | | . | . | . | . | . | . | . | S | . | . | . | . | . | . | . | . | . | . | . | L |
| 21-225_197F9_LC | . | | | | . | | . | . | . | . | . | . | . | . | . | . | . | . | . | . | . | . | . | . | . | . |
| 21-225_199C3_LC | . | | | | . | | . | . | . | . | . | . | . | . | . | . | . | . | . | . | . | . | . | . | . | . |
| 21-225_4G12_LC | . | | | | I | | . | . | . | . | . | . | . | . | . | . | . | . | . | . | . | . | . | . | . | . |
| 21-225_60D6_LC | . | | | | . | | . | . | . | . | . | . | . | . | . | . | . | . | . | . | . | . | . | . | . | . |
| 21-225_9G9_LC | . | | | | I | | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | L_FR3 | | | | | | | | | | | | | | | |
| CONSENSUS | S | G | S | K | S | G | - | - | T | S | A | S | L | A | I | S | G | L | Q | S | E | D | E | A | D | Y |
| 21-225_146A8_LC | | | | | | | | | | | | | | | | | | | | | | | | | | |
| 21-225_146B6_LC | | | | | | | | | | | | | | | | | | | | | | | | | | |
| 21-225_146D8_LC | | | | | | | | | | | | | | | | | | | | | | | | | | |
| 21-225_147H12_LC | | | | | | | | | | | | | | | | | | | | | | | | | | |
| 21-225_149A1_LC | | | | | | | | | | | | | | | | | | | | | | | | | | |
| 21-225_150F9_LC | | | | | | | | | | | | | | | | | | | | | | | | | | |
| 21-225_154G12_LC | | | | | | | | | | | | | | | | | | | | | | | | | | |
| 21-225_156H2_LC | | | | | | | | | | | | | | | | | | | | | | | | | | |
| 21-225_197F9_LC | | | | | | | | | | | | | | | | | | | | | | | | | | |
| 21-225_199C3_LC | | | | | | | | | | | | | | | | | | | | | | | | | | |
| 21-225_4G12_LC | | | | | | | | | | | | | | | | | | | | | | | | | | |
| 21-225_60D6_LC | | | | | | | | | | | | | | | | | | | | | | | | | | |
| 21-225_9G9_LC | | | | | | | | | | | | | | | | | | | | | | | | | | |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | L_CDR3 | | | | | | | | |
| CONSENSUS | Y | C | A | A | W | D | D | S | - | - | - | - | - | - | - | - | - | - | - | - | - | - | - | - | - | - |
| 21-225_146A8_LC | . | | | . | . | . | . | . | . | . | . | . | . | . | . | . | . | . | . | . | . | . | . | . | . | |
| 21-225_146B6_LC | . | | | . | . | . | . | . | . | . | . | . | . | . | . | . | . | . | . | . | . | . | . | . | . | |
| 21-225_146D8_LC | . | | . | . | . | . | . | . | . | . | . | . | . | . | . | . | . | . | . | . | . | . | . | . | . | |
| 21-225_147H12_LC | . | | ::: | . | . | . | . | . | . | . | . | . | . | . | . | . | . | . | . | . | . | . | . | . | . | |
| 21-225_149A1_LC | . | | . | . | . | . | . | . | . | . | . | . | . | . | . | . | . | . | . | . | . | . | . | . | . | |
| 21-225_150F9_LC | ::: | | . | . | . | . | . | . | . | . | . | . | . | . | . | . | . | . | . | . | . | . | . | . | . | |
| 21-225_154G12_LC | . | | . | . | . | . | . | . | . | . | . | . | . | . | . | . | . | . | . | . | . | . | . | . | . | |
| 21-225_156H2_LC | . | | . | . | . | . | . | . | . | . | . | . | . | . | . | . | . | . | . | . | . | . | . | . | . | |
| 21-225_197F9_LC | . | | . | . | . | . | . | . | . | . | . | . | . | . | . | . | . | . | . | . | . | . | . | . | . | |
| 21-225_199C3_LC | . | | . | . | . | . | . | . | . | . | . | . | . | . | . | . | . | . | . | . | . | . | . | . | . | |
| 21-225_4G12_LC | . | | . | . | . | . | . | . | . | . | . | . | . | . | . | . | . | . | . | . | . | . | . | . | . | |
| 21-225_60D6_LC | . | | . | . | . | . | - | . | . | . | . | . | . | . | . | . | . | . | . | . | . | . | . | . | . | |
| 21-225_9G9_LC | . | | . | . | . | . | . | . | . | . | . | . | . | . | . | . | . | . | . | . | . | . | . | . | . | |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | L_FR4 | | | | | |
| CONSENSUS | - | - | - | L | N | G | V | V | F | G | G | G | T | K | L | T | V | L | G |
| 21-225_146A8_LC | . | | . | . | . | . | . | . | . | . | . | | | . | . | . | . | | . |
| 21-225_146B6_LC | . | | . | . | . | . | . | . | . | . | . | . | . | . | . | . | . | | S |
| 21-225_146D8_LC | . | | . | . | . | . | . | . | . | . | . | . | . | . | . | . | . | . | . |
| 21-225_147H12_LC | . | | . | . | . | . | P | . | . | . | . | . | . | . | . | . | . | . | . |
| 21-225_149A1_LC | . | | . | . | . | . | . | . | . | . | . | . | . | . | . | . | . | . | S |
| 21-225_150F9_LC | . | | . | . | . | . | . | . | . | . | . | . | . | . | . | . | . | . | S |
| 21-225_154G12_LC | . | . | . | . | X | . | P | . | . | . | . | . | . | . | . | . | . | . | . |
| 21-225_156H2_LC | . | | . | . | . | . | . | . | . | . | . | . | . | . | V | . | . | . | . |
| 21-225_197F9_LC | . | . | . | M | . | . | P | . | . | . | . | . | . | . | . | . | . | . | . |
| 21-225_199C3_LC | . | . | . | . | . | . | P | . | . | . | . | . | . | . | . | . | . | . | . |
| 21-225_4G12_LC | . | . | L | N | G | R | . | . | . | . | . | R | . | . | . | . | . | . | . |
| 21-225_60D6_LC | . | . | . | S | L | N | G | P | V | . | . | . | . | . | . | . | . | . | . |
| 21-225_9G9_LC | . | . | L | N | G | R | . | . | | . | | R | . | . | . | . | . | . | . |

4981

FIGURE 57

Table 121.    Consensus 41 - VL1|1c/JL3b (SEQ ID NO: 50338):

QSVLTQP-PSASGTPGQRVTISC<u>SGSS-SNIGS</u>-----NIVTWYQQLPGTAPKLLIY<u>S</u>-------NDQRPSGVPDRFSGSKSG--
TSASLAISGLQSEDEADYYC<u>AAWDDS</u>--------------------<u>LNGWV</u>FGGGTTLTVLG wherein:

S at position 27 can be substituted with N or C

S at position 33 can be substituted with N

N at position 39 can be substituted with H

I at position 40 can be substituted with T

T at position 42 can be substituted with N

S at position 58 can be substituted with G, N or V

D at position 68 can be substituted with K, N or Y

A at position 107 can be substituted with T

A at position 108 can be substituted with T or V

N at position 135 can be substituted with I or S

G at position 136 can be substituted with D or V

Ser Gly Ser Xaa Ser Asn Ile Gly Xaa Xaa Xaa Val Xaa (SEQ ID NO: 50195)

Xaa Asn Xaa Gln Arg Pro Ser (SEQ ID NO: 50196)

Xaa Xaa Trp Asp Asp Ser Leu Xaa Xaa Trp Val (SEQ ID NO: 50197)

Ser Gly Ser Ser Ser Asn Ile Gly Ser Asn Ile Val Thr (SEQ ID NO: 50684)

Ser Asn Asp Gln Arg Pro Ser (SEQ ID NO: 50685)

Ala Ala Trp Asp Asp Ser Leu Asn Gly Trp Val (SEQ ID NO: 50686)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | L_FR1 | | | | | | | | | | | | | | | |
| CONSENSUS | Q | S | V | L | T | Q | P | - | P | S | A | S | G | T | P | G | Q | R | V | T | I | S | C | S | G | S |
| 21-225_192G3_LC | . | . | . | . | . | . | . | | . | . | . | . | . | . | . | . | . | . | . | . | N | . | . | . | . | . |
| 21-225_195H6_LC | . | . | . | . | . | . | . | | . | . | . | . | . | . | . | . | . | . | . | . | . | . | . | . | . | . |
| 21-225_49C5_LC | . | . | . | . | . | . | . | | . | . | . | . | . | . | . | . | . | . | . | . | . | . | . | . | . | . |
| 21-225_49D10_LC | . | . | . | . | . | . | . | | . | . | . | . | . | . | . | . | . | . | . | . | . | . | . | . | . | . |
| 21-225_51E3_LC | . | . | . | . | . | . | . | | . | . | . | . | . | . | . | . | . | . | . | . | . | . | . | . | . | . |
| 21-225_51F4_LC | . | . | . | . | . | . | . | | . | . | . | . | . | . | . | . | . | . | . | . | . | . | . | . | . | . |
| 21-225_52F1_LC | . | . | . | . | . | . | . | | . | . | . | . | . | . | . | . | . | . | . | . | . | . | . | . | . | . |
| 21-225_53E8_LC | . | . | . | . | . | . | . | | . | . | . | . | . | . | . | . | . | . | . | . | . | . | . | . | . | . |
| 21-225_54E9_LC | . | . | . | . | . | . | . | | . | . | . | . | . | . | . | . | . | . | . | . | . | . | . | . | . | . |
| 21-225_56E3_LC | . | . | . | . | . | . | . | | . | . | . | . | . | . | . | . | . | . | . | . | . | . | . | . | . | . |
| 21-225_56G1_LC | . | . | . | . | . | . | . | | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | L_CDR1 | | | | | | | | | | | | | | | | | | L_FR2 | | |
| CONSENSUS | S | - | S | N | I | G | S | - | - | - | - | - | N | I | V | T | W | Y | Q | Q | L | P | G | T | A | P |
| 21-225_192G3_LC | . | . | . | . | . | . | . | | | | . | . | . | T | . | N | . | . | . | . | P | . | . | . | . | . |
| 21-225_195H6_LC | . | . | . | . | . | . | . | | | | . | . | H | T | . | N | . | . | . | . | . | . | . | . | . | . |
| 21-225_49C5_LC | . | . | . | . | . | . | . | | | | . | . | . | . | . | . | . | . | . | . | . | . | . | . | . | . |
| 21-225_49D10_LC | N | . | . | . | . | . | . | | | | . | . | . | . | . | . | . | . | . | . | . | . | . | . | . | . |
| 21-225_51E3_LC | N | . | . | . | . | . | . | | | | . | . | . | . | . | . | . | . | . | . | . | . | . | . | . | . |
| 21-225_51F4_LC | . | . | . | . | . | . | . | | | | . | . | . | . | . | . | . | . | . | . | . | . | . | . | . | . |
| 21-225_52F1_LC | . | . | . | . | . | . | . | | | | . | . | . | . | . | . | . | . | . | . | . | . | . | . | . | . |
| 21-225_53E8_LC | G | . | . | . | . | . | . | | | | . | . | . | . | . | . | . | . | . | . | . | . | . | . | . | . |
| 21-225_54E9_LC | N | . | . | . | . | . | N | | | | . | . | . | . | . | . | . | . | . | . | . | . | . | . | . | . |
| 21-225_56E3_LC | . | . | . | . | . | . | . | | | | . | . | . | . | . | . | . | . | . | . | . | . | . | . | . | . |
| 21-225_56G1_LC | . | . | . | . | . | . | . | | | | . | . | H | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | L_CDR2 | | | | | | | | | | | | | | | |
| CONSENSUS | K | L | L | I | Y | S | - | - | - | - | - | - | - | - | N | D | Q | R | P | S | G | V | P | D | R | F |
| 21-225_192G3_LC | . | . | . | . | . | G | . | . | . | . | . | . | . | . | . | K | . | . | . | . | R | . | . | . | . | . |
| 21-225_195H6_LC | . | . | . | . | . | N | . | . | . | . | . | . | . | . | . | Y | . | . | . | . | . | . | . | . | . | . |
| 21-225_49C5_LC | . | . | . | . | . | . | . | . | . | . | . | . | . | . | . | . | . | . | . | . | . | . | . | . | . | . |
| 21-225_49D10_LC | . | . | . | . | . | . | . | . | . | . | . | . | . | . | . | . | . | . | . | . | . | . | . | . | . | . |
| 21-225_51E3_LC | . | . | . | . | . | . | . | . | . | . | . | . | . | . | . | . | . | . | . | . | . | . | . | . | . | . |
| 21-225_51F4_LC | . | . | . | . | . | . | . | . | . | . | . | . | . | . | . | . | . | . | . | . | . | . | . | . | . | . |
| 21-225_52F1_LC | . | . | . | . | . | . | . | . | . | . | . | . | . | . | . | . | . | . | . | . | . | . | . | . | . | . |
| 21-225_53E8_LC | . | . | . | . | . | . | . | . | . | . | . | . | . | . | . | . | . | . | . | . | . | . | . | . | . | . |
| 21-225_54E9_LC | . | . | . | . | . | V | . | . | . | . | . | . | . | . | . | . | . | . | . | . | . | . | . | . | . | . |
| 21-225_56E3_LC | . | . | . | . | . | . | . | . | . | . | . | . | . | . | . | N | . | . | . | . | . | . | . | . | . | . |
| 21-225_56G1_LC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | L_FR3 | | | | | | | | | | | | | | | |
| CONSENSUS | S | G | S | K | S | G | - | - | T | S | A | S | L | A | I | S | G | L | Q | S | E | D | E | A | D | Y |
| 21-225_192G3_LC | . | . | . | . | . | . | . | . | . | . | . | . | . | . | . | . | . | . | . | . | . | . | . | T | . | . |
| 21-225_195H6_LC | . | . | . | . | . | . | . | . | . | . | . | . | . | T | . | . | . | . | . | . | . | . | . | . | . | . |
| 21-225_49C5_LC | . | . | . | . | . | . | . | . | . | . | . | . | . | . | . | . | . | . | . | . | . | . | . | . | . | . |
| 21-225_49D10_LC | . | . | . | . | . | . | . | . | . | . | . | . | . | . | . | . | . | . | . | . | . | . | . | . | . | . |
| 21-225_51E3_LC | . | . | . | . | . | . | . | . | . | . | . | . | . | . | . | . | . | . | . | . | . | . | . | . | . | . |
| 21-225_51F4_LC | . | . | . | . | . | . | . | . | . | . | . | . | . | . | . | . | . | . | . | . | . | . | . | T | . | . |
| 21-225_52F1_LC | . | . | . | . | . | . | . | . | . | . | . | . | . | . | . | . | . | . | . | . | . | . | . | . | . | . |
| 21-225_53E8_LC | . | . | . | . | . | . | . | . | . | . | . | . | . | . | . | . | . | . | . | . | . | . | . | . | . | . |
| 21-225_54E9_LC | . | . | . | . | . | . | . | . | . | . | . | . | . | . | . | . | . | . | . | . | . | . | . | . | . | . |
| 21-225_56E3_LC | . | . | . | . | . | . | . | . | . | . | . | . | . | . | . | . | . | . | . | . | . | . | . | . | . | . |
| 21-225_56G1_LC | . | . | . | . | . | . | . | . | . | . | . | . | . | . | . | . | . | . | . | . | . | . | . | . | . | . |

## FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | L_CDR3 | | | | | | | | |
| CONSENSUS | Y | C | A | A | W | D | D | S | - | - | - | - | - | - | - | - | - | - | - | - | - | - | - | - | - | - |
| 21-225_192G3_LC | . | . | . | . | . | . | . | . | . | . | . | . | . | . | . | . | . | . | . | . | . | . | . | . | . | . |
| 21-225_195H6_LC | . | . | . | T | . | . | . | . | . | . | . | . | . | . | . | . | . | . | . | . | . | . | . | . | . | . |
| 21-225_49C5_LC | . | . | T | . | . | . | . | . | . | . | . | . | . | . | . | . | . | . | . | . | . | . | . | . | . | . |
| 21-225_49D10_LC | . | . | . | . | . | . | . | . | . | . | . | . | . | . | . | . | . | . | . | . | . | . | . | . | . | . |
| 21-225_51E3_LC | . | . | T | . | . | . | . | . | . | . | . | . | . | . | . | . | . | . | . | . | . | . | . | . | . | . |
| 21-225_51F4_LC | . | . | T | . | . | . | . | . | . | . | . | . | . | . | . | . | . | . | . | . | . | . | . | . | . | . |
| 21-225_52F1_LC | . | . | . | . | . | . | . | . | . | . | . | . | . | . | . | . | . | . | . | . | . | . | . | . | . | . |
| 21-225_53E8_LC | . | . | T | . | . | . | . | . | . | . | . | . | . | . | . | . | . | . | . | . | . | . | . | . | . | . |
| 21-225_54E9_LC | . | . | . | . | . | . | . | . | . | . | . | . | . | . | . | . | . | . | . | . | . | . | . | . | . | . |
| 21-225_56E3_LC | C | . | . | . | . | . | . | . | . | . | . | . | . | . | . | . | . | . | . | . | . | . | . | . | . | . |
| 21-225_56G1_LC | . | . | . | V | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | L_FR4 | | | | | | | |
| CONSENSUS | - | - | - | L | N | G | W | V | F | G | G | G | T | T | L | T | V | L | G |
| 21-225_192G3_LC | . | . | . | . | . | . | . | . | . | . | . | . | . | K | . | . | . | . | . |
| 21-225_195H6_LC | . | . | . | . | S | V | . | . | . | . | . | . | . | K | V | . | . | . | . |
| 21-225_49C5_LC | . | . | . | . | . | . | . | . | . | . | . | . | . | . | . | . | . | . | . |
| 21-225_49D10_LC | . | . | . | . | . | . | . | . | . | . | . | . | . | . | . | . | . | . | . |
| 21-225_51E3_LC | . | . | . | . | . | . | . | . | . | . | . | . | . | . | . | . | . | . | . |
| 21-225_51F4_LC | . | . | . | . | . | . | . | . | . | . | . | . | . | . | . | . | . | . | . |
| 21-225_52F1_LC | . | . | . | . | . | . | . | . | . | . | . | . | . | . | . | . | . | . | . |
| 21-225_53E8_LC | . | . | . | . | D | . | . | . | . | . | . | . | . | . | . | . | . | . | . |
| 21-225_54E9_LC | . | . | . | . | . | . | . | . | . | . | . | . | . | . | . | . | . | . | . |
| 21-225_56E3_LC | . | . | . | . | . | . | . | . | . | . | . | . | . | . | . | . | . | . | . |
| 21-225_56G1_LC | . | . | . | . | I | . | . | . | . | . | . | . | . | . | . | . | . | . | . |

FIGURE 57

Table 122.    Consensus 42 - VK1|A30/JK5 (SEQ ID NO: 50339):

DIQMTQSPSSLSASVGDRVTITCRAS--QGIR------NDLGWYQQKPGKAPKRLIYA--------ASSLQSGVPSRFSGSGSG--
TEFTLTISSLQPEDFATYYCLQHNS---------------------YPITFGQGTRLEIKR wherein:

A at position 25 can be substituted with T

G at position 30 can be substituted with D or R

I at position 31 can be substituted with V

N at position 39 can be substituted with S

A at position 58 can be substituted with D, I or T

S at position 69 can be substituted with N

Q at position 71 can be substituted with E, F or L

L at position 107 can be substituted with I

Q at position 108 can be substituted with H

H at position 109 can be substituted with Y

N a position 110 can be substituted with H or S

S at position 111 can be substituted with N

null (-) at position 134 can be substituted with Y

Y at position 135 can be substituted with L, F or P

I at position 137 can be substituted with P or L

Arg Xaa Ser Gln Xaa Xaa Arg Xaa Asp Leu Gly (SEQ ID NO: 50198)

**FIGURE 57**

Xaa Ala Ser Xaa Leu Xaa Ser (SEQ ID NO: 50199)

Xaa Xaa Xaa Xaa Xaa Xaa Xaa Pro Xaa Thr (SEQ ID NO: 50200)

Arg Ala Ser Gln Gly Ile Arg Asn Asp Leu Gly (SEQ ID NO: 50687)

Ala Ala Ser Ser Leu Gln Ser (SEQ ID NO: 50688)

Leu Gln His Asn Ser Tyr Pro Ile Thr (SEQ ID NO: 50689)

**FIGURE 57**

FIGURE 57

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | K_CDR3 | | | | | | | | | |
| CONSENSUS | Y | C | L | Q | H | N | S | - | - | - | - | - | - | - | - | - | - | - | - | - | - | - | - | - | - | - |
| 21-225_159F1_LC | . | . | . | . | . | H | . | . | . | . | . | . | . | . | . | . | . | . | . | . | . | . | . | . | . | . |
| 21-225_18D4_LC | . | . | . | H | . | . | N | . | . | . | . | . | . | . | . | . | . | . | . | . | . | . | . | . | . | . |
| 21-225_207F12_LC | F | . | . | . | . | . | . | . | . | . | . | . | . | . | . | . | . | . | . | . | . | . | . | . | . | . |
| 21-225_208A2_LC | . | . | . | . | Y | . | . | . | . | . | . | . | . | . | . | . | . | . | . | . | . | . | . | . | . | . |
| 21-225_210G10_LC | . | . | . | . | Y | . | . | . | . | . | . | . | . | . | . | . | . | . | . | . | . | . | . | . | . | . |
| 21-225_34C4_LC | . | . | . | . | S | . | . | . | . | . | . | . | . | . | . | . | . | . | . | . | . | . | . | . | . | . |
| 21-225_73H8_LC | . | S | ? | . | . | . | . | . | . | . | . | . | . | . | . | . | . | . | . | . | . | . | . | . | . | . |
| 21-225_7C3_LC1_LC | . | . | . | . | . | . | . | . | . | . | . | . | . | . | . | . | . | . | . | . | . | . | . | . | . | . |
| 21-225_90H5_LC | . | . | . | . | . | . | . | . | . | . | . | . | . | . | . | . | . | . | . | . | . | . | . | . | . | . |
| 21-225_9F12_LC2_LC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | Y | P | I | T | F | G | Q | G | T | R | L | E | I | K | R |
| 21-225_159F1_LC | . | . | . | . | F | . | . | . | . | . | . | . | . | . | . | . | . | . | . |
| 21-225_18D4_LC | . | . | . | Y | L | . | . | . | . | . | . | . | . | . | . | . | . | . | . |
| 21-225_207F12_LC | . | . | . | . | . | L | . | . | . | . | . | . | . | . | . | . | . | . | . |
| 21-225_208A2_LC | . | . | . | . | . | P | . | . | . | . | . | . | . | . | . | . | . | . | . |
| 21-225_210G10_LC | . | . | . | . | . | P | . | . | . | . | . | . | . | . | . | . | . | . | . |
| 21-225_34C4_LC | . | . | . | . | . | . | . | . | . | . | . | . | . | . | . | . | . | . | . |
| 21-225_73H8_LC | . | . | . | Y | P | . | . | . | V | . | . | . | . | . | . | . | . | . | . |
| 21-225_7C3_LC1_LC | . | . | . | Y | L | . | . | . | . | . | . | . | . | . | . | . | . | . | . |
| 21-225_90H5_LC | . | . | . | . | . | . | . | . | . | . | . | . | . | . | . | . | . | . | . |
| 21-225_9F12_LC2_LC | . | . | . | Y | L | . | . | . | . | . | . | . | . | . | . | . | . | . | . |

Table 123.     Consensus 43 - VK1|O12/JK5 (SEQ ID NO: 50340):

**FIGURE 57**

DIQMTQSPSSLSASVGDRVTITCRAS--QSIS-----SYLNWYQQKPGKAPKLLIYA-------ASSLQSGVPSRFSGSGSG--
TDFTLTISSLQPEDFATYYCQQSYS---------------------IPITFGQGTRLEIKR wherein:

A at position 25 can be substituted with T

S at position 30 can be substituted with N or Y

I at position 31 can be substituted with S or F

S at position 32 can be substituted with F, N, R or T

S at position 39 can be substituted with D, G or R

L at position 41 can be substituted with S

N at position 42 can be substituted with S

A at position 58 can be substituted with D, G or S

A at position 67 can be substituted with T

S at position 68 can be substituted with Y

S at position 69 can be substituted with T

L at position 70 can be substituted with F

Q at position 71 can be substituted with E or K

S at position 72 can be substituted with T

Q at position 107 can be substituted with H

Q at position 108 can be substituted with E

S at position 109 can be substituted with T

Y at position 110 can be substituted with F

FIGURE 57

S at position 111 can be substituted with G or N

I at position 135 can be substituted with T, L, N or S

P at position 136 can be substituted with S, R or T

I at position 137 can be substituted with F or P

T at position 138 can be substituted with A

Arg Xaa Ser Gln Xaa Xaa Xaa Xaa Tyr Xaa Xaa (SEQ ID NO: 50201)

Xaa Xaa Xaa Xaa Xaa Xaa Xaa (SEQ ID NO: 50202)

Xaa Xaa Xaa Xaa Xaa Xaa Xaa Xaa Xaa (SEQ ID NO: 50203)

Arg Ala Ser Gln Ser Ile Ser Ser Tyr Leu Asn (SEQ ID NO: 50690)

Ala Ala Ser Ser Leu Gln Ser (SEQ ID NO: 50691)

Gln Gln Ser Tyr Ser Ile Pro Ile Thr (SEQ ID NO: 50692)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | K_FR1 | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_157E7_LC | . | | . | | | | | | | | | | | | | | | | | | | | | | | |
| 21-225_21D3_LC | . | | | | | | | | | | | | | | | | | | | | | | | | | |
| 21-225_22D8_LC | . | | | | | | | | | | | | | | | | | | | | | | | | T | |
| 21-225_33A9_LC | . | | . | | | | | | | | | | | | | | | | | | | | | | | |
| 21-225_33D12_LC | . | V | | | | | | | | | | | | | L | | | | | | | | Y | | | |
| 21-225_35E5_LC | . | | . | | I | | | | | | | | | | | | | | | | | | | | | |
| 21-225_53C6_LC | . | | | | | | | | | | | | | | | | | | | | | | | | | |
| 21-225_59E8_LC | . | | | | | | | | | | | | | | | | | | | | | | | | | |
| 21-225_63G6_LC | . | | | | | | | | | | | | | | | | | | | | I | | S | | | |
| 21-225_72F5_LC | . | | | | | | | | | | | | | | | | | | | | I | | P | | | |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | I | S | - | - | - | - | - | - | S | Y | L | N | W | Y | Q | Q | K | P | G | K | A | P |
| 21-225_157E7_LC | | | . | | P | . | | | | | | | | | | | | | | | | | | I | | |
| 21-225_21D3_LC | | | | | | T | | | | | | | Q | | S | | | | | | | | | | T | |
| 21-225_22D8_LC | | | | N | S | N | | | | | | | | | | | S | | | | | | | | | |
| 21-225_33A9_LC | | | | | | | | | | | | | R | | | S | | | | | | | | R | | |
| 21-225_33D12_LC | | | | | | R | | | | | | | | | | | | | | | | | | | | |
| 21-225_35E5_LC | | | | | | | | | | | | | | | | | | | | | | | A | | | |
| 21-225_53C6_LC | | | | Y | S | | | | | | | | Q | | | | | | | | | | | | | |
| 21-225_59E8_LC | | | | | | | | | | | | | | | | | | | | U | | | | | | |
| 21-225_63G6_LC | | | | | | P | | | | | | | | | | | | | | U | | | | | | |
| 21-225_72F5_LC | | | | N | | | | | | | | | | | | | | | | | | | | | | |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | K_CDR2 | | | | | | | | | | | | | | | |
| CONSENSUS | K | L | L | I | Y | A | - | - | - | - | - | - | - | - | A | S | S | L | Q | S | G | V | P | S | R | F |
| 21-225_157E7_LC | . | . | . | I | . | . | | | | | | | | | . | . | . | . | . | . | . | . | . | . | . | . |
| 21-225_21D3_LC | . | . | . | . | F | . | | | | | | | | . | T | Y | . | . | E | . | . | . | . | . | . | . |
| 21-225_22D8_LC | . | . | Q | . | F | . | | | | | | | | . | . | Y | . | . | E | . | . | . | . | . | . | . |
| 21-225_33A9_LC | N | . | Q | . | . | . | | | | | | | | | | | | | | | | | | | | |
| 21-225_33D12_LC | N | . | . | . | . | S | | | | | | | | | | | | | | | | | | | | |
| 21-225_35E5_LC | . | . | M | . | . | G | | | | | | | | | | | | | F | . | . | W | . | . | . | . |
| 21-225_53C6_LC | . | V | . | . | F | . | | | | | | | | | | | | | M | . | . | . | . | . | . | . |
| 21-225_59E8_LC | . | . | . | . | . | . | | | | | | | | | | | | | | | | | | | | |
| 21-225_63G6_LC | . | . | . | . | O | . | | | | | | | | | T | . | Y | . | . | Y | . | . | . | . | . | . |
| 21-225_72F5_LC | . | . | . | . | . | . | | | | | | | | | | | | | | | | | | | | |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_157E7_LC | . | . | . | . | . | . | . | . | . | . | . | . | . | . | . | . | . | . | . | . | . | . | . | . | . | . |
| 21-225_21D3_LC | . | . | . | F | . | . | . | . | N | . | . | . | . | . | . | T | . | . | . | . | . | . | . | . | . | . |
| 21-225_22D8_LC | . | . | N | R | . | . | . | . | S | . | . | . | . | . | . | . | . | . | . | . | . | . | . | . | . | . |
| 21-225_33A9_LC | . | . | . | . | . | . | . | . | . | . | . | . | . | . | . | . | . | . | . | . | . | . | . | . | . | . |
| 21-225_33D12_LC | . | . | . | . | . | . | . | . | . | . | . | . | . | . | . | . | . | . | . | . | . | . | . | . | N | . |
| 21-225_35E5_LC | . | . | . | . | . | S | . | . | . | . | . | . | . | . | . | I | . | M | . | . | O | . | Y | . | . | . |
| 21-225_53C6_LC | . | . | . | . | . | . | . | . | . | . | . | . | . | . | . | . | . | E | . | . | . | . | . | . | . | . |
| 21-225_59E8_LC | . | . | . | . | . | . | . | . | . | . | . | . | . | . | . | . | . | . | . | . | . | . | . | . | . | . |
| 21-225_63G6_LC | . | . | . | . | . | . | . | . | . | . | . | . | . | . | N | . | . | . | . | . | . | . | . | . | . | . |
| 21-225_72F5_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | S | Y | S | - | - | - | - | - | - | - | - | - | - | - | - | - | - | - | - | - | - | - |
| 21-225_157E7_LC | . | . | . | S | . | . | . | | | | | | | | | | | | | | | | | | | |
| 21-225_21D3_LC | . | . | . | . | . | . | . | | | | | | | | | | | | | | | | | | | |
| 21-225_22D8_LC | . | . | . | . | T | . | G | | | | | | | | | | | | | | | | | | | |
| 21-225_33A9_LC | . | . | . | . | . | . | N | | | | | | | | | | | | | | | | | | | |
| 21-225_33D12_LC | . | . | . | . | . | . | . | | | | | | | | | | | | | | | | | | | |
| 21-225_35E5_LC | . | . | M | . | . | . | N | | | | | | | | | | | | | | | | | | | |
| 21-225_53C6_LC | F | . | . | . | . | . | . | | | | | | | | | | | | | | | | | | | |
| 21-225_59E8_LC | F | . | . | . | . | F | . | | | | | | | | | | | | | | | | | | | |
| 21-225_63G6_LC | . | . | . | . | . | . | G | | | | | | | | | | | | | | | | | | | |
| 21-225_72F5_LC | S | . | . | . | T | . | - | | | | | | | | | | | | | | | | | | | |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | I | P | I | T | F | G | Q | G | T | R | L | E | I | K | R |
| 21-225_157E7_LC | . | . | . | . | S | F | A | . | . | . | . | . | . | . | . | . | . | . | . |
| 21-225_21D3_LC | . | . | . | T | S | . | . | . | . | . | . | . | . | . | . | . | . | . | . |
| 21-225_22D8_LC | . | . | . | T | S | . | . | . | . | . | . | . | . | . | . | . | . | . | . |
| 21-225_33A9_LC | . | . | . | . | . | . | . | . | . | . | . | . | . | . | . | . | . | . | . |
| 21-225_33D12_LC | . | . | . | . | . | . | . | . | . | . | . | . | . | . | . | . | . | . | . |
| 21-225_35E5_LC | . | . | . | L | . | . | . | . | . | . | . | . | . | . | . | . | . | . | . |
| 21-225_53C6_LC | . | . | . | T | . | F | . | . | . | . | . | . | . | . | . | . | . | . | . |
| 21-225_59E8_LC | . | . | . | N | S | . | . | . | . | . | . | . | . | . | . | . | . | . | . |
| 21-225_63G6_LC | . | . | . | . | . | . | . | . | . | . | . | . | . | . | . | Q | . | | |
| 21-225_72F5_LC | . | . | . | S | T | F | . | . | . | . | . | . | . | D | . | N | . | | |

FIGURE 57

Table 124.    Consensus 44 - VK2|A18/JK5 (SEQ ID NO: 50341):

DIVMTQTPLSLSVTPGQPASISCKSS--QSLLHSE-GKTYLYWYLQKPGQPPQLLIYE-------VSNRFSGVPDRFSGSGSG--
TDFTLKISRVEAEDVGVYYCMQSIQ---------------------LPITFGQGTRLEIKR wherein:

K at position 24 can be substituted with R

S at position 25 can be substituted with T

S at position 26 can be substituted with N

S at position 30 can be substituted with I

L at position 31 can be substituted with F

L at position 32 can be substituted with V

S at position 34 can be substituted with N or R

E at position 35 can be substituted with D

K at position 38 can be substituted with R

V at position 67 can be substituted with L

N at position 69 can be substituted with K or H

F at position 71 can be substituted with L or V

M at position 107 can be substituted with I or L

I at position 110 can be substituted with M

Q at position 111 can be substituted with null (-) or L

L at position 135 can be substituted with Y, I or Q

**FIGURE 57**

P at position 136 can be substituted with L

T at position 138 can be substituted with I

Xaa Xaa Xaa Gln Xaa Xaa Xaa His Xaa Xaa Gly Xaa Thr Tyr Leu Tyr (SEQ ID NO: 50204)

Glu Xaa Ser Xaa Arg Xaa Ser (SEQ ID NO: 50205)

Xaa Gln Ser Xaa Xaa Xaa Xaa Ile Xaa (SEQ ID NO: 50246)

Lys Ser Ser Gln Ser Leu Leu His Ser Glu Gly Lys Thr Tyr Leu Tyr (SEQ ID NO: 50693)

Glu Val Ser Asn Arg Phe Ser (SEQ ID NO: 50694)

Met Gln Ser Ile Gln Leu Pro Ile Thr (SEQ ID NO: 50695)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | T | P | L | S | L | S | V | T | P | G | Q | P | A | S | I | S | C | K | S | S |
| 21-225_147A8_LC | . | . | . | . | . | . | . | . | . | . | . | . | . | . | . | . | . | . | . | . | . | . | . | . | T | . |
| 21-225_32H2_LC | . | . | . | . | . | . | . | . | . | . | . | . | . | . | . | . | . | . | . | . | . | . | . | R | . | . |
| 21-225_49C1_LC | . | . | . | . | . | . | . | . | . | . | . | . | . | . | . | . | . | . | . | . | . | . | . | . | . | . |
| 21-225_49E7_LC | . | . | . | . | . | . | . | . | . | . | . | . | . | . | . | . | . | . | . | F | M | . | . | . | . | . |
| 21-225_49F9_LC | . | . | . | . | . | . | . | . | . | . | . | . | . | . | . | . | . | . | . | . | . | . | . | . | . | N |
| 21-225_49G3_LC | . | . | . | . | . | . | . | . | . | . | . | . | . | . | . | . | . | . | . | . | M | . | . | . | . | . |
| 21-225_52D10_LC | . | V | M | . | . | . | I | . | . | . | . | . | . | . | . | . | . | . | . | . | . | . | . | . | . | . |
| 21-225_52G8_LC | . | . | . | . | . | . | . | . | . | . | . | . | . | . | . | . | . | . | . | . | M | . | . | . | . | . |
| 21-225_55C2_LC | . | V | M | . | . | . | I | . | . | . | . | . | . | . | . | . | . | . | . | . | . | . | . | . | . | . |
| 21-225_55F9_LC | . | . | . | . | . | . | . | . | . | . | . | . | . | . | . | . | . | S | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | L | L | H | S | E | - | G | K | T | Y | L | Y | W | Y | L | Q | K | P | G | Q | P | P |
| 21-225_147A8_LC | . | . | . | . | . | . | . | . | . | . | . | . | . | . | . | . | . | . | . | . | . | . | . | . | . | . |
| 21-225_32H2_LC | . | . | . | . | . | . | . | Q | . | . | R | . | . | . | . | . | . | . | . | . | . | . | . | . | . | . |
| 21-225_49C1_LC | . | . | . | . | . | . | R | . | . | . | . | . | . | . | . | . | . | . | . | . | . | . | . | S | . | . |
| 21-225_49E7_LC | . | . | . | I | S | . | . | . | . | . | . | . | . | . | . | . | . | . | . | . | T | . | . | . | . | . |
| 21-225_49F9_LC | . | . | . | . | . | V | . | N | . | . | . | . | . | . | . | . | . | . | . | . | . | . | . | . | . | . |
| 21-225_49G3_LC | . | . | . | . | . | . | . | . | . | . | . | . | . | . | . | . | . | . | . | . | T | . | . | . | . | . |
| 21-225_52D10_LC | . | . | . | . | . | . | . | . | . | . | . | . | . | . | . | . | . | . | . | . | . | . | . | . | . | . |
| 21-225_52G8_LC | . | . | . | . | . | . | . | . | . | . | . | . | . | . | . | . | . | . | . | . | T | . | . | . | . | . |
| 21-225_55C2_LC | . | . | . | . | . | V | . | . | . | . | . | . | . | . | . | . | . | . | . | . | . | . | . | . | . | . |
| 21-225_55F9_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | Q | L | L | I | Y | E | - | - | - | - | - | - | - | - | V | S | N | R | F | S | G | V | P | D | R | F |
| 21-225_147A8_LC | . | . | . | . | . | . | | | | | | | | | | | | | | | . | . | . | . | . | . |
| 21-225_32H2_LC | . | . | . | . | . | . | | | | | | | L | . | | | | | | | . | . | . | . | . | . |
| 21-225_49C1_LC | . | F | . | . | F | . | | | | | | | | | | | H | . | | | . | . | . | . | . | . |
| 21-225_49E7_LC | H | . | . | . | . | . | | | | | | | | | | | K | . | L | | . | . | . | . | . | . |
| 21-225_49F9_LC | . | . | . | . | F | . | | | | | | | | | | | | | | | . | . | . | . | . | . |
| 21-225_49G3_LC | H | . | . | . | . | . | | | | | | | | | | | | | L | | . | . | . | . | . | . |
| 21-225_52D10_LC | . | . | . | . | F | . | | | | | | | | | | | | | V | | . | . | . | . | . | . |
| 21-225_52G8_LC | H | . | . | . | . | . | | | | | | | | | | | K | . | L | | . | . | . | . | . | . |
| 21-225_55C2_LC | . | . | . | . | F | . | | | | | | | | | | | | | V | | . | . | . | . | . | . |
| 21-225_55F9_LC | . | . | . | . | . | . | | | | | | | | | | | | | | | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | K_FR3 | | | | | | | | | | | | | | | | |
| CONSENSUS | S | G | S | G | S | G | - | - | T | D | F | T | L | K | I | S | R | V | E | A | E | D | V | G | V | Y |
| 21-225_147A8_LC | . | . | . | . | . | . | | | . | . | . | . | . | . | . | . | . | . | . | . | . | . | . | . | . | . |
| 21-225_32H2_LC | . | . | . | . | . | . | | | . | . | . | . | . | . | . | . | . | . | . | . | . | . | . | . | I | . |
| 21-225_49C1_LC | . | . | . | . | . | . | | | . | . | . | . | . | . | . | . | . | . | . | . | . | . | . | . | . | . |
| 21-225_49E7_LC | . | . | . | . | . | . | | | . | . | . | . | . | . | . | . | . | M | . | . | . | . | . | . | . | . |
| 21-225_49F9_LC | . | . | . | . | . | . | | | . | . | . | . | . | . | . | . | . | . | . | . | . | . | . | . | . | . |
| 21-225_49G3_LC | . | . | . | . | . | . | | | . | . | . | . | . | . | . | . | . | M | . | . | . | . | . | . | . | . |
| 21-225_52D10_LC | . | . | R | . | . | . | | | . | . | . | . | . | . | . | . | . | . | . | . | . | . | . | . | . | . |
| 21-225_52G8_LC | . | . | . | . | . | . | | | . | . | . | . | . | . | . | . | . | M | . | . | . | . | . | . | . | . |
| 21-225_55C2_LC | . | . | R | . | . | . | | | . | . | . | . | . | . | . | . | . | . | . | . | . | . | . | . | . | . |
| 21-225_55F9_LC | . | . | . | . | . | . | | | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | K_CDR3 | | | | | | | |
| CONSENSUS | Y | C | M | Q | S | I | Q | - | - | - | - | - | - | - | - | - | - | - | - | - | - | - | - | - | - | - |
| 21-225_147A8_LC | . | . | . | . | . | | - | | | | . | . | . | . | . | . | . | . | . | . | . | . | . | . | . | - |
| 21-225_32H2_LC | S | . | L | . | . | . | | | | | . | . | . | . | . | . | . | . | . | . | . | . | . | . | . | |
| 21-225_49C1_LC | . | . | . | . | . | | | | | | . | . | . | . | . | . | . | . | . | . | . | . | . | . | . | |
| 21-225_49E7_LC | . | . | . | . | . | M | | | | | . | . | . | . | . | . | . | . | . | . | . | . | . | . | . | |
| 21-225_49F9_LC | . | . | . | . | . | | | | | | . | . | . | . | . | . | . | . | . | . | . | . | . | . | . | |
| 21-225_49G3_LC | S | . | . | . | . | M | | | | | . | . | . | . | . | . | . | . | . | . | . | . | . | . | . | |
| 21-225_52D10_LC | . | . | . | . | . | | | | | | . | . | . | . | . | . | . | . | . | . | . | . | . | . | . | |
| 21-225_52G8_LC | . | . | . | . | . | M | | | | | . | . | . | . | . | . | . | . | . | . | . | . | . | . | . | |
| 21-225_55C2_LC | . | . | . | . | . | L | | | | | . | . | . | . | . | . | . | . | . | . | . | . | . | . | . | |
| 21-225_55F9_LC | . | . | I | . | . | | | | | | . | . | . | . | . | . | . | . | . | . | . | . | . | . | . | |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | P | I | T | F | G | Q | G | T | R | L | E | I | K | R | |
| 21-225_147A8_LC | . | . | . | . | Q | L | . | . | . | . | . | . | . | . | . | . | . | . | |
| 21-225_32H2_LC | . | . | . | . | Y | . | . | . | . | . | . | . | . | . | . | . | . | . | |
| 21-225_49C1_LC | . | . | . | . | I | . | . | L | . | . | . | . | . | . | . | . | . | . | |
| 21-225_49E7_LC | . | . | . | . | L | . | I | . | . | . | . | . | . | . | . | . | . | . | |
| 21-225_49F9_LC | . | . | . | . | Y | . | . | . | . | . | . | . | . | . | . | . | . | . | |
| 21-225_49G3_LC | . | . | . | . | L | . | . | . | . | . | . | . | . | . | . | . | . | . | |
| 21-225_52D10_LC | . | . | . | . | Y | . | . | . | . | . | . | . | . | . | . | . | . | . | |
| 21-225_52G8_LC | . | . | . | . | L | . | I | . | . | . | . | . | . | . | . | . | . | . | |
| 21-225_55C2_LC | . | . | . | . | Y | . | . | . | . | . | . | . | . | . | . | . | . | . | |
| 21-225_55F9_LC | . | . | . | . | L | . | . | . | . | . | . | . | . | . | . | . | . | . | |

**FIGURE 57**

Table 125.    Consensus 45 - VK3|A27/JK3 (SEQ ID NO: 50342):

EIVLTQSPGTLSLFPGERATLSCRAS--QSVIS-----SYLAWYQQKPGQAPRLLIFG--------VSSRATGIPDRFSGSGSG--
TDFTLTISRLEPEDFAVYYCQQYGR----------------------SPFNFGPGTKVDIKR wherein:

Q at position 29 can be substituted with R

S at position 30 can be substituted with G or N

V at position 31 can be substituted with I

I at position 32 can be substituted with S or G

S at position 33 can be substituted with N

S at position 39 can be substituted with I or N

Y at position 40 can be substituted with F

V at position 67 can be substituted with A or T

S at position 69 can be substituted with N or T

R at position 70 can be substituted with W

Q at position 107 can be substituted with H

Q at position 108 can be substituted with H

Y at position 109 can be substituted with N

G at position 110 can be substituted with D

R at position 111 can be substituted with null (-), N or Y

P at position 136 can be substituted with L or M

**FIGURE 57**

N at position 138 can be substituted with T

Arg Ala Ser Xaa Xaa Xaa Xaa Xaa Xaa Leu Ala (SEQ ID NO: 50206)

Gly Xaa Ser Xaa Xaa Ala Thr (SEQ ID NO: 50207)

Xaa Xaa Xaa Xaa Xaa Ser Xaa Xaa Asn (SEQ ID NO: 50208)

Arg Ala Ser Gln Ser Val Ile Ser Ser Tyr Leu Ala (SEQ ID NO: 50696)

Gly Val Ser Ser Arg Ala Thr (SEQ ID NO: 50697)

Gln Gln Tyr Gly Arg Ser Pro Phe Asn (SEQ ID NO: 50698)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | E | I | V | L | T | Q | S | P | G | T | L | S | L | | P | G | E | R | A | T | L | S | C | R | A | S |
| 21-225_146G4_LC | . | . | . | . | . | . | . | . | . | . | . | . | P | . | . | . | . | . | . | . | . | . | . | . | . | . |
| 21-225_147D2_LC | . | . | . | . | . | . | . | . | . | . | . | . | S | . | . | . | . | . | . | . | . | . | . | . | . | . |
| 21-225_147E3_LC | . | . | . | . | . | . | . | . | . | . | . | . | S | . | . | . | . | . | . | . | . | . | . | . | . | . |
| 21-225_149D7_LC | . | . | . | . | . | . | . | . | . | . | . | . | P | . | . | . | . | . | V | . | . | . | . | . | . | . |
| 21-225_149D8_LC | . | . | . | . | . | . | . | . | . | . | . | . | P | . | . | . | . | . | . | . | . | . | . | . | . | . |
| 21-225_152E7_LC | . | . | . | . | . | . | . | . | . | . | . | . | P | . | . | . | . | . | . | . | . | . | . | . | . | . |
| 21-225_153A6_LC | . | . | . | . | . | . | . | . | . | . | . | . | P | . | . | . | . | . | . | P | . | . | . | . | . | . |
| 21-225_194A4_LC | . | . | . | . | . | . | . | . | . | . | . | . | S | . | . | . | . | . | . | . | . | . | . | . | . | . |
| 21-225_216F10_LC | . | V | . | . | . | . | . | . | . | . | . | . | S | . | . | . | . | . | . | . | . | . | . | . | . | . |
| 21-225_59H5_LC | . | . | . | . | . | . | . | . | . | . | . | . | S | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | K_CDR1 | | | | | | | | | | | | | | | | | | | K_FR2 | | |
| CONSENSUS | - | - | Q | S | V | I | S | - | - | - | - | - | S | Y | L | A | W | Y | Q | Q | K | P | G | Q | A | P |
| 21-225_146G4_LC | . | . | N | I | . | . | . | . | . | . | . | . | . | . | . | . | . | . | . | . | . | . | . | . | . | . |
| 21-225_147D2_LC | . | . | . | . | . | G | . | . | . | . | . | . | . | . | . | . | . | . | . | . | . | . | . | . | . | . |
| 21-225_147E3_LC | . | . | . | . | . | . | . | . | . | . | . | . | . | . | . | . | . | . | . | . | . | . | . | . | . | . |
| 21-225_149D7_LC | . | . | . | I | . | . | . | . | . | . | . | . | N | . | . | . | . | . | . | . | . | . | . | . | . | . |
| 21-225_149D8_LC | . | . | . | . | . | . | . | . | . | . | . | . | . | . | . | . | . | . | . | . | . | . | . | . | . | . |
| 21-225_152E7_LC | . | . | . | . | . | . | . | . | . | . | . | . | . | . | . | . | . | . | . | . | . | . | . | . | . | . |
| 21-225_153A6_LC | . | . | . | . | . | . | . | . | . | . | . | . | . | . | . | . | . | . | . | . | . | . | . | . | . | . |
| 21-225_194A4_LC | . | . | R | G | . | S | N | . | . | . | . | . | I | . | . | . | . | . | . | . | . | . | . | . | . | . |
| 21-225_216F10_LC | . | . | . | . | . | S | . | . | . | . | . | . | . | P | . | . | . | . | . | . | . | . | . | . | . | . |
| 21-225_59H5_LC | . | . | . | . | . | S | . | . | . | . | . | . | I | . | . | . | P | L | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | R | L | L | I | | G | - | - | - | - | - | - | - | - | V | S | S | R | A | T | G | I | P | D | R | F |
| 21-225_146G4_LC | . | . | . | . | S | . | | | | | | | | | . | . | . | | | | | . | . | . | . | . |
| 21-225_147D2_LC | . | . | . | . | Y | . | | | | | | | | . | A | . | . | | | | | . | . | . | . | . |
| 21-225_147E3_LC | . | . | . | . | Y | . | | | | | | | | . | A | . | . | | | | A | . | . | . | . | . |
| 21-225_149D7_LC | . | . | . | . | S | . | | | | | | | | | . | . | . | | | | | . | . | . | . | . |
| 21-225_149D8_LC | . | . | . | . | S | . | | | | | | | | | . | . | . | W | | | | . | . | . | . | . |
| 21-225_152E7_LC | . | . | . | . | S | . | | | | | | | | | . | . | . | | | | | . | . | . | . | . |
| 21-225_153A6_LC | . | . | . | . | S | . | | | | | | | | | . | . | . | | | | | . | . | . | . | . |
| 21-225_194A4_LC | . | . | . | . | Y | . | | | | | | | | . | A | . | N | | | | | . | . | . | . | . |
| 21-225_216F10_LC | . | . | . | . | Y | . | | | | | | | | . | T | . | T | | | | | . | . | . | . | . |
| 21-225_59H5_LC | . | . | . | . | Y | . | | | | | | | | . | A | . | . | | | | | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | D | F | T | L | T | I | S | R | L | E | P | E | D | F | A | V | Y |
| 21-225_146G4_LC | . | . | . | . | . | . | . | . | . | . | . | . | . | . | . | . | . | . | . | . | . | . | . | . | . | . |
| 21-225_147D2_LC | . | . | . | . | . | . | . | . | . | . | . | . | . | . | . | . | . | . | . | . | . | . | . | . | . | . |
| 21-225_147E3_LC | . | . | . | . | . | . | . | . | . | . | . | . | . | . | . | . | . | . | . | . | . | . | . | . | . | . |
| 21-225_149D7_LC | . | . | . | . | . | . | . | . | . | . | . | . | . | . | . | . | . | . | . | . | . | . | . | . | . | . |
| 21-225_149D8_LC | . | . | . | . | . | . | . | . | . | . | . | . | . | . | . | . | . | . | . | . | . | . | . | . | . | . |
| 21-225_152E7_LC | . | . | . | . | . | . | . | . | . | . | . | . | . | . | . | . | . | . | . | . | . | . | . | . | . | . |
| 21-225_153A6_LC | . | . | . | . | . | . | . | . | . | . | . | . | . | . | . | . | . | . | . | . | . | . | . | . | . | . |
| 21-225_194A4_LC | . | . | . | . | . | . | . | . | . | . | . | . | . | . | . | . | . | . | . | . | . | . | . | . | . | . |
| 21-225_216F10_LC | . | . | . | . | . | . | . | . | . | . | S | . | . | . | . | . | . | . | . | . | . | . | . | . | . | . |
| 21-225_59H5_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | G | R | - | - | - | - | - | - | - | - | - | - | - | - | - | - | - | - | - | - | - |
| 21-225_146G4_LC | . | . | . | . | . | . | | . | . | . | . | . | . | . | . | . | . | . | . | . | . | . | . | . | . | . |
| 21-225_147D2_LC | . | . | . | . | . | . | - | | . | . | . | . | . | . | . | . | . | . | . | . | . | . | . | . | . | . |
| 21-225_147E3_LC | . | . | . | . | . | . | | . | . | . | . | . | . | . | . | . | . | . | . | . | . | . | . | . | . | . |
| 21-225_149D7_LC | . | . | . | . | . | . | | . | . | . | . | . | . | . | . | . | . | . | . | . | . | . | . | . | . | . |
| 21-225_149D8_LC | . | . | . | . | . | . | | . | . | . | . | . | . | . | . | . | . | . | . | . | . | . | . | . | . | . |
| 21-225_152E7_LC | . | . | . | . | . | . | | . | . | . | . | . | . | . | . | . | . | . | . | . | . | . | . | . | . | . |
| 21-225_153A6_LC | . | . | . | . | . | . | | . | . | . | . | . | . | . | . | . | . | . | . | . | . | . | . | . | . | . |
| 21-225_194A4_LC | . | . | . | M | N | O | Y | . | . | . | . | . | . | . | . | . | . | . | . | . | . | . | . | . | . | . |
| 21-225_216F10_LC | . | . | . | . | . | O | . | . | . | . | . | . | . | . | . | . | . | . | . | . | . | . | . | . | . | . |
| 21-225_59H5_LC | . | . | N | . | . | . | N | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | S | P | F | N | F | G | P | G | T | K | V | D | I | K | R |
| 21-225_146G4_LC | . | . | . | . | . | . | . | . | . | . | . | . | . | . | . | . | . | N | . |
| 21-225_147D2_LC | . | . | . | . | L | . | T | . | . | . | . | . | . | . | . | . | . | . | . |
| 21-225_147E3_LC | . | . | . | . | . | . | . | . | . | . | . | . | . | . | . | . | . | . | . |
| 21-225_149D7_LC | . | . | . | . | . | . | . | . | . | . | . | . | . | . | . | . | . | . | . |
| 21-225_149D8_LC | . | . | . | . | . | . | . | . | . | . | . | . | . | . | . | . | . | . | . |
| 21-225_152E7_LC | . | . | . | . | . | . | . | . | . | . | . | . | . | . | . | . | . | . | . |
| 21-225_153A6_LC | . | . | . | . | . | . | . | . | . | . | . | . | . | . | . | . | . | . | . |
| 21-225_194A4_LC | . | . | . | . | . | M | . | T | . | . | . | . | . | . | . | . | . | . | . |
| 21-225_216F10_LC | . | . | . | . | . | . | . | T | . | . | . | . | . | . | . | . | . | . | . |
| 21-225_59H5_LC | . | . | . | . | . | . | . | T | . | . | . | . | . | . | . | . | F | . | . |

FIGURE 57

Table 126.    Consensus 46 - VK3|L2/JK1 (SEQ ID NO: 50343):

EIVMTQSPATLSVSPGERATLSCRAS--QSVS------SNLAWYQQKPGQAPRLLIYG-------ASTRATGIPARFSGSGSG--
TEFTLTISSLQSEDFAVYYCQQYND----------------------WPWTFGQGTKVEIKR wherein:

A at position 25 can be substituted with S

S at position 30 can be substituted with D or T

V at position 31 can be substituted with I

S at position 32 can be substituted with N, R or I

S at position 39 can be substituted with I or T

N at position 40 can be substituted with Y

L at position 41 can be substituted with I

T at position 72 can be substituted with S

Q at position 108 can be substituted with E

Y at position 109 can be substituted with S

N at position 110 can be substituted with D, F or H

D at position 111 can be substituted with N, null (-) or T

null (-) at position 134 can be substituted with W

W at position 135 can be substituted with P, C or N

P at position 136 can be substituted with L or W

W at position 137 can be substituted with L, C, P or R

**FIGURE 57**

T at position 138 can be substituted with P or S

Arg Xaa Ser Gln Xaa Xaa Xaa Xaa Xaa Xaa Ala (SEQ ID NO: 50209)

Gly Ala Ser Thr Arg Ala Xaa (SEQ ID NO: 50210)

Gln Xaa Xaa Xaa Xaa Xaa Xaa Xaa Xaa (SEQ ID NO: 50211)

Arg Ala Ser Gln Ser Val Ser Ser Asn Leu Ala (SEQ ID NO: 50699)

Gly Ala Ser Thr Arg Ala Thr (SEQ ID NO: 50700)

Gln Gln Tyr Asn Asp Trp Pro Trp Thr (SEQ ID NO: 50701)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | | |
| CONSENSUS | E | I | V | M | T | Q | S | P | A | T | L | S | V | S | P | G | E | R | A | T | L | S | C | R | A | S |
| 21-225_13D3_LC | . | . | . | . | . | . | . | . | . | . | . | . | . | . | . | . | . | S | . | . | . | . | . | . | . | . |
| 21-225_151F1_LC | . | M | . | . | . | . | . | . | . | . | . | . | . | . | . | . | . | . | V | . | . | . | . | . | . | . |
| 21-225_155H9_LC | . | . | . | . | . | . | . | . | . | . | . | . | . | . | . | . | . | . | . | . | . | . | . | . | . | . |
| 21-225_161A4_LC | . | . | . | . | . | . | . | . | . | . | . | . | . | . | . | . | . | . | S | . | . | . | . | S | . | . |
| 21-225_17D8_LC | . | . | . | . | . | . | . | . | S | . | . | . | . | . | . | . | . | . | . | . | . | . | . | . | . | . |
| 21-225_191C8_LC | . | . | . | . | . | . | . | . | . | . | . | . | . | . | . | . | . | . | . | . | . | . | . | . | . | . |
| 21-225_20B8_LC | . | . | . | . | . | . | . | . | S | . | . | . | . | . | . | . | . | . | V | . | . | . | . | . | . | . |
| 21-225_25B8_LC | . | . | . | . | . | . | . | . | . | . | . | . | . | . | . | . | . | . | . | . | . | . | . | . | . | . |
| 21-225_28C5_LC | . | . | . | . | . | . | . | . | . | . | . | . | . | . | . | . | . | . | . | . | . | . | . | . | . | . |
| 21-225_85H1_LC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | K_FR2 | | | |
| CONSENSUS | - | - | Q | S | V | S | - | - | - | - | - | - | S | N | L | A | W | Y | Q | Q | K | P | G | Q | A | P |
| 21-225_13D3_LC | . | . | . | T | . | . | . | . | . | . | . | . | . | . | . | . | . | P | . | . | . | . | . | . | . | . |
| 21-225_151F1_LC | . | . | . | . | I | N | . | . | . | . | . | . | I | . | I | . | . | . | . | . | . | . | . | . | . | . |
| 21-225_155H9_LC | . | . | . | . | . | . | . | . | . | . | . | . | . | . | . | . | . | . | . | . | . | . | . | . | . | . |
| 21-225_161A4_LC | . | . | . | . | . | N | . | . | . | . | . | . | . | . | . | . | . | . | . | . | R | . | . | . | . | L |
| 21-225_17D8_LC | . | . | . | . | . | . | . | . | . | . | . | . | . | . | . | . | . | P | . | . | . | . | . | . | . | . |
| 21-225_191C8_LC | . | . | . | . | . | R | . | . | . | . | . | . | . | . | . | . | . | . | . | . | R | . | . | . | . | . |
| 21-225_20B8_LC | . | . | . | . | . | . | . | . | . | . | . | . | . | . | . | . | . | P | . | . | . | . | . | . | . | . |
| 21-225_25B8_LC | . | . | . | . | . | R | . | . | . | . | . | . | . | . | . | . | . | . | . | . | . | . | . | . | . | . |
| 21-225_28C5_LC | . | . | . | . | . | . | . | . | . | . | . | . | . | . | . | . | . | R | . | . | . | . | . | . | . | . |
| 21-225_85H1_LC | . | . | . | D | . | I | . | . | . | . | . | . | T | Y | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | R | L | L | I | Y | G | - | - | - | - | - | - | - | - | A | S | T | R | A | T | G | I | P | A | R | F |
| 21-225_13D3_LC | . | . | . | . | N | . | | | | | | | | | . | . | . | . | . | . | . | . | . | . | . | . |
| 21-225_151F1_LC | . | . | . | . | . | . | | | | | | | | | . | . | . | . | . | . | . | . | . | . | . | . |
| 21-225_155H9_LC | . | . | . | . | . | . | | | | | | | | | . | . | . | . | . | . | . | . | . | . | . | . |
| 21-225_161A4_LC | . | . | . | . | . | . | | | | | | | | | . | . | i | . | . | . | D | . | . | . | . | . |
| 21-225_17D8_LC | . | . | . | . | N | . | | | | | | | | | . | . | . | . | . | S | . | . | . | . | . | . |
| 21-225_191C8_LC | . | . | . | . | . | . | | | | | | | | | . | . | . | . | . | . | . | . | . | . | . | . |
| 21-225_20B8_LC | . | . | . | . | N | . | | | | | | | | | . | . | . | . | . | S | . | . | . | . | . | . |
| 21-225_25B8_LC | . | . | . | . | . | . | | | | | | | | | . | . | . | . | . | . | . | . | . | . | . | . |
| 21-225_28C5_LC | . | . | . | . | . | . | | | | | | | | | . | . | . | . | . | . | . | . | . | . | . | . |
| 21-225_85H1_LC | . | . | . | . | . | . | | | | | | | | | . | . | . | . | . | . | . | V | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | K_FR3 | | | | | | | | | | | | | | | | | |
| CONSENSUS | S | G | S | G | S | G | - | - | T | E | F | T | L | T | I | S | S | L | Q | S | E | D | F | A | V | Y |
| 21-225_13D3_LC | . | . | . | . | . | . | | | . | . | . | . | . | . | . | . | . | . | . | . | . | . | . | . | . | . |
| 21-225_151F1_LC | . | . | . | . | . | . | | | . | . | . | . | . | . | . | . | . | . | . | . | . | . | . | . | . | . |
| 21-225_155H9_LC | . | . | . | . | . | . | | | . | . | . | . | . | . | . | . | . | . | . | . | . | . | . | . | . | . |
| 21-225_161A4_LC | N | . | . | . | . | . | | | . | . | . | . | . | . | . | . | . | . | . | . | . | . | . | . | . | . |
| 21-225_17D8_LC | . | . | . | . | . | . | | | . | . | . | . | . | . | . | . | . | . | . | . | . | . | . | . | . | . |
| 21-225_191C8_LC | . | . | . | . | . | . | | | . | . | . | . | . | . | . | . | . | . | . | . | . | . | . | . | . | . |
| 21-225_20B8_LC | . | . | . | . | . | . | | | . | . | . | . | . | . | . | . | . | . | . | . | . | . | . | . | . | . |
| 21-225_25B8_LC | . | . | . | . | . | . | | | . | . | . | . | . | . | . | . | . | . | . | . | . | . | . | . | L | . |
| 21-225_28C5_LC | . | . | . | . | . | . | | | . | . | . | . | . | . | . | . | . | . | . | . | . | . | . | . | L | . |
| 21-225_85H1_LC | . | . | . | . | . | . | | | . | . | . | . | . | . | . | . | . | . | . | . | . | . | . | . | L | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | N | - | - | - | - | - | - | - | - | - | - | - | - | - | - | - | - | - | - | - | - |
| 21-225_13D3_LC | F | . | . | . | . | H | O | | . | . | . | . | . | . | . | . | . | . | . | . | . | . | . | . | . | . |
| 21-225_151F1_LC | . | . | . | . | . | . | O | | . | . | . | . | . | . | . | . | . | . | . | . | . | . | . | . | . | . |
| 21-225_155H9_LC | . | . | . | . | . | O | O | | . | . | . | . | . | . | . | . | . | . | . | . | . | . | . | . | . | . |
| 21-225_161A4_LC | F | . | . | . | . | . | - | | . | . | . | . | . | . | . | . | . | . | . | . | . | . | . | . | . | . |
| 21-225_17D8_LC | . | . | . | . | . | . | N | | . | . | . | . | . | . | . | . | . | . | . | . | . | . | . | . | . | . |
| 21-225_191C8_LC | . | . | . | . | S | F | T | | . | . | . | . | . | . | . | . | . | . | . | . | . | . | . | . | . | . |
| 21-225_20B8_LC | . | . | . | . | . | . | N | | . | . | . | . | . | . | . | . | . | . | . | . | . | . | . | . | . | . |
| 21-225_25B8_LC | . | . | . | . | . | . | N | | . | . | . | . | . | . | . | . | . | . | . | . | . | . | . | . | . | . |
| 21-225_28C5_LC | . | . | . | . | . | . | N | | . | . | . | . | . | . | . | . | . | . | . | . | . | . | . | . | . | . |
| 21-225_85H1_LC | . | . | . | S | . | . | O | | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | K_FR4 | | | | | | | | | | | |
| CONSENSUS | - | - | - | - | | P | W | T | F | G | Q | G | T | K | V | E | I | K | R |
| 21-225_13D3_LC | . | . | . | . | W | . | F | . | . | . | . | . | . | . | . | . | . | . | . |
| 21-225_151F1_LC | . | . | . | W | P | . | . | . | . | . | . | . | . | . | . | . | . | . | . |
| 21-225_155H9_LC | . | . | . | W | P | . | . | . | . | . | . | . | . | . | . | . | . | . | . |
| 21-225_161A4_LC | . | . | . | . | N | W | . | . | . | . | . | . | T | . | . | . | . | . | . |
| 21-225_17D8_LC | . | . | . | . | W | . | L | . | . | . | . | . | . | . | . | . | . | . | . |
| 21-225_191C8_LC | . | . | . | . | W | L | S | . | . | . | . | . | . | . | . | . | . | . | . |
| 21-225_20B8_LC | . | . | . | . | C | . | L | . | . | . | . | . | . | . | . | . | . | . | . |
| 21-225_25B8_LC | . | . | . | W | P | . | . | P | . | . | . | . | . | . | . | . | . | . | . |
| 21-225_28C5_LC | . | . | . | W | P | . | . | P | . | . | . | . | . | . | . | . | . | . | . |
| 21-225_85H1_LC | . | . | . | . | W | . | C | S | . | . | . | . | . | . | . | . | . | . | . |

FIGURE 57

Table 127.    Consensus 47 - VK6|A26/JK1 (SEQ ID NO: 50344):

EIVLTQSPDFQSVTPKEKVTITCRAS--QSIG------SSLHWYQQKPDQSPKLLIKY--------ASQSFSGVPSRFSGSGSG--
TDFTLTINSLEAEDAATYYCHQSSS--------------------LPWTFGQGTKVEIKR wherein:

S at position 30 can be substituted with N

S at position 39 can be substituted with N

S at position 40 can be substituted with N or T

Y at position 58 can be substituted with S

H at position 107 can be substituted with Q

S at position 110 can be substituted with G or R

L at position 135 can be substituted with F

W at position 137 can be substituted with R or Q

Arg Ala Ser Gln Xaa Ile Gly Xaa Xaa Leu His (SEQ ID NO: 50256)

Xaa Ala Ser Gln Ser Phe Ser (SEQ ID NO: 50257)

Xaa Gln Ser Xaa Ser Xaa Pro Xaa Thr (SEQ ID NO: 50258)

Arg Ala Ser Gln Ser Ile Gly Ser Ser Leu His (SEQ ID NO: 50702)

Tyr Ala Ser Gln Ser Phe Ser (SEQ ID NO: 50703)

His Gln Ser Ser Ser Leu Pro Trp Thr (SEQ ID NO: 50704)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | E | I | V | L | T | Q | S | P | D | F | Q | S | V | T | P | K | E | K | V | T | I | T | C | R | A | S |
| 21-225_190B11_LC | . | . | . | . | . | . | . | . | . | . | . | . | . | A | . | . | . | . | . | . | . | . | . | . | . | . |
| 21-225_190D5_LC | . | . | . | . | . | . | . | . | . | . | . | . | . | A | . | . | . | . | . | . | . | . | . | . | . | . |
| 21-225_190D9_LC | . | . | . | . | . | . | S | . | . | S | . | . | . | . | . | . | . | . | . | . | . | . | . | . | . | . |
| 21-225_190F11_LC | . | . | . | . | . | . | S | . | . | S | . | . | . | . | . | . | . | . | . | . | . | . | . | . | . | . |
| 21-225_190H8_LC | . | . | . | . | . | . | S | . | . | S | . | . | . | . | . | . | . | . | . | . | . | . | . | . | . | . |
| 21-225_191E5_LC | . | . | . | . | . | . | S | . | . | S | . | . | . | . | . | . | . | . | . | . | . | . | . | . | . | . |
| 21-225_194C3_LC | . | . | . | . | . | . | . | . | . | . | . | . | . | A | . | . | . | . | . | . | . | . | . | . | . | . |
| 21-225_200B7_LC | . | . | . | . | . | . | . | . | . | . | . | . | . | . | . | . | . | . | . | . | . | . | . | . | . | . |
| 21-225_200F8_LC | . | . | . | . | . | . | . | . | . | . | . | . | . | A | . | . | . | . | . | . | . | . | . | . | . | . |
| 21-225_217A3_LC | . | . | . | . | . | . | . | . | . | . | K | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | K_CDR1 | | | | | | | | | | | | | | | | | K_FR2 | | | | |
| CONSENSUS | - | - | Q | S | I | G | - | - | - | - | - | - | S | S | L | H | W | Y | Q | Q | K | P | D | Q | S | P |
| 21-225_190B11_LC | . | . | . | . | . | . | . | . | . | . | . | . | . | N | . | . | . | . | . | . | . | . | . | . | . | . |
| 21-225_190D5_LC | . | . | . | . | . | . | . | . | . | . | . | . | . | N | . | . | . | . | . | . | . | . | . | . | . | . |
| 21-225_190D9_LC | . | . | . | . | . | . | . | . | . | . | . | . | . | . | . | . | . | . | . | . | . | . | . | . | . | . |
| 21-225_190F11_LC | . | . | . | N | . | . | . | . | . | . | . | . | . | . | . | . | . | . | . | . | . | . | S | . | . | . |
| 21-225_190H8_LC | . | . | . | . | . | . | . | . | . | . | . | . | . | . | . | . | . | . | . | . | . | . | . | . | . | . |
| 21-225_191E5_LC | . | . | . | . | . | . | . | . | . | . | . | . | . | . | . | . | . | . | . | . | . | . | . | . | . | . |
| 21-225_194C3_LC | . | . | . | . | . | . | . | . | . | . | . | . | . | N | . | . | . | . | . | . | . | . | . | . | . | . |
| 21-225_200B7_LC | . | . | . | N | . | . | . | . | . | . | . | . | N | T | . | . | . | . | . | . | . | . | . | . | . | . |
| 21-225_200F8_LC | . | . | . | . | . | . | . | . | . | . | . | . | . | N | . | . | . | . | . | . | . | . | . | . | . | . |
| 21-225_217A3_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | K_CDR2 | | | | | | | | | | | | | | | | |
| CONSENSUS | K | L | L | I | K | Y | - | - | - | - | - | - | - | - | A | S | Q | S | F | S | G | V | P | S | R | F |
| 21-225_190B11_LC | . | . | . | . | . | S | . | . | . | . | . | . | . | . | . | . | . | . | . | . | . | . | . | . | . | . |
| 21-225_190D5_LC | . | . | . | . | . | S | . | . | . | . | . | . | . | . | . | . | . | . | . | . | . | . | . | . | . | . |
| 21-225_190D9_LC | . | . | . | . | . | . | . | . | . | . | . | . | . | . | . | . | . | . | . | . | . | . | . | . | . | . |
| 21-225_190F11_LC | . | V | . | . | . | . | . | . | . | . | . | . | . | . | . | . | . | . | . | . | . | . | . | . | . | . |
| 21-225_190H8_LC | . | . | . | . | . | . | . | . | . | . | . | . | . | . | . | . | . | . | . | . | . | . | . | . | . | . |
| 21-225_191E5_LC | . | . | . | . | . | . | . | . | . | . | . | . | . | . | . | . | . | . | . | . | . | . | . | . | . | . |
| 21-225_194C3_LC | . | . | . | . | . | S | . | . | . | . | . | . | . | . | . | . | . | . | . | . | . | . | . | . | . | . |
| 21-225_200B7_LC | . | . | . | . | . | . | . | . | . | . | . | . | . | . | . | . | . | . | . | . | . | . | . | . | . | . |
| 21-225_200F8_LC | . | . | . | . | . | S | . | . | . | . | . | . | . | . | . | . | . | . | . | . | . | . | . | . | . | . |
| 21-225_217A3_LC | . | . | . | V | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | N | S | L | E | A | E | D | A | A | T | Y |
| 21-225_190B11_LC | . | . | . | . | . | . | . | . | . | . | . | . | . | . | . | . | . | . | . | T | . | . | . | . | . | . |
| 21-225_190D5_LC | . | . | . | . | . | . | . | . | . | . | . | . | . | . | . | . | . | . | . | T | . | . | . | . | . | . |
| 21-225_190D9_LC | . | . | . | . | . | . | . | . | . | . | . | . | . | . | . | . | . | . | . | . | . | . | . | . | . | . |
| 21-225_190F11_LC | . | . | . | . | . | . | . | . | . | . | . | . | . | . | . | . | . | . | . | . | . | . | . | . | . | . |
| 21-225_190H8_LC | . | . | . | . | . | . | . | . | . | . | . | . | . | . | . | . | . | . | . | . | . | . | . | . | . | . |
| 21-225_191E5_LC | . | . | . | . | . | . | . | . | . | . | . | . | . | . | . | . | . | . | . | . | . | . | . | . | . | . |
| 21-225_194C3_LC | . | . | . | . | . | . | . | . | . | . | . | . | . | . | . | . | . | . | . | T | . | . | . | . | . | . |
| 21-225_200B7_LC | . | . | . | . | . | . | . | . | . | . | ? | . | . | . | . | . | . | . | . | . | . | . | . | . | . | . |
| 21-225_200F8_LC | . | . | . | . | . | . | . | . | . | . | . | . | . | . | . | . | . | . | . | T | . | . | . | . | . | . |
| 21-225_217A3_LC | . | . | . | . | . | . | . | . | . | . | . | . | . | . | . | . | . | . | . | . | . | . | Q | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | H | Q | S | S | S | - | - | - | - | - | - | - | - | - | - | - | - | - | - | - | - | - | - | - |
| 21-225_190B11_LC | . | . | Q | . | | | | | | | | | | | | | | | | | | | | | | |
| 21-225_190D5_LC | . | . | Q | . | | | | | | | | | | | | | | | | | | | | | | |
| 21-225_190D9_LC | . | | | | | | | | | | | | | | | | | | | | | | | | | |
| 21-225_190F11_LC | . | | | | | | | | | | | | | | | | | | | | | | | | | |
| 21-225_190H8_LC | . | | | | | | | | | | | | | | | | | | | | | | | | | |
| 21-225_191E5_LC | . | | | | | | | | | | | | | | | | | | | | | | | | | |
| 21-225_194C3_LC | . | | Q | | | | | | | | | | | | | | | | | | | | | | | |
| 21-225_200B7_LC | . | | | | Q | | | | | | | | | | | | | | | | | | | | | |
| 21-225_200F8_LC | . | | Q | | | | | | | | | | | | | | | | | | | | | | | |
| 21-225_217A3_LC | . | | | | | | | | | | | | | | | | | | | | | | | | | |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | L | P | W | T | F | G | Q | G | T | K | V | E | I | K | R |
| 21-225_190B11_LC | . | . | . | . | | | | | | | | | | | | | | | |
| 21-225_190D5_LC | . | . | . | . | | | | | | | | | | | | | | | |
| 21-225_190D9_LC | . | . | . | . | | | | | | | | | | | | | | | |
| 21-225_190F11_LC | . | . | . | . | | | | | | | | | | | | | | | |
| 21-225_190H8_LC | . | . | . | . | | | | | | | | | | | | | | | |
| 21-225_191E5_LC | . | . | . | . | | | | | | | | | | | | | | | |
| 21-225_194C3_LC | . | . | . | . | | | | | | | | | | | | | | | |
| 21-225_200B7_LC | . | . | . | . | | | Q | | | | | | | | | | | | |
| 21-225_200F8_LC | . | . | . | . | | | | | | | | | | | | | | | |
| 21-225_217A3_LC | . | . | . | . | | | | | | | | | | | | | | | |

**FIGURE 57**

Table 128.    Consensus 48 - VK1|A30/JK2 (SEQ ID NO: 50345):

DIQMTQSPSSLSASVGDRVTITCRAS--QGIR------NDLGWYQQKPGKAPKRLIYA-------ASSLQSGVPSRFSGSGSG--
TEFTLTISSLQPEDFATYYCLQHYS---------------------YPRSFGQGTKLEIKR wherein:

G at position 30 can be substituted with A

R at position 32 can be substituted with G

N at position 39 can be substituted with D

A at position 67 can be substituted with T

Y at position 110 can be substituted with N

S at position 111 can be substituted with N

Y at position 135 can be substituted with F

R at position 137 can be substituted with Y

Arg Ala Ser Gln Xaa Ile Xaa Xaa Asp Leu Gly (SEQ ID NO: 50247)

Ala Xaa Ser Ser Leu Gln Ser (SEQ ID NO: 50248)

Leu Gln His Xaa Xaa Xaa Pro Xaa Ser (SEQ ID NO: 50249)

Arg Ala Ser Gln Gly Ile Arg Asn Asp Leu Gly (SEQ ID NO: 50705)

Ala Ala Ser Ser Leu Gln Ser (SEQ ID NO: 50706)

Leu Gln His Tyr Ser Tyr Pro Arg Ser (SEQ ID NO: 50707)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_150E2_LC | . | . | . | . | . | . | . | . | . | . | . | . | . | . | . | . | . | . | . | . | . | S | . | . | . | . |
| 21-225_152F7_LC | . | . | . | . | . | . | . | . | . | . | . | . | . | . | . | . | . | . | . | . | . | . | . | . | . | . |
| 21-225_176H12_LC | . | . | . | . | . | . | . | . | . | . | . | . | . | . | . | . | . | . | . | . | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | . | . | . | . | . | . | . | . | . | . | . | . | . | . | . | . | . | . | V | . | . | . | . | . |
| 21-225_26A11_LC | . | . | . | . | . | . | . | . | I | . | . | . | . | . | . | . | . | . | . | . | . | . | . | . | . | . |
| 21-225_28H2_LC | . | . | . | . | . | . | . | . | . | . | . | . | . | I | . | . | . | . | . | . | . | . | . | . | . | . |
| 21-225_29E2_LC | . | . | . | . | . | . | . | . | . | . | . | . | . | . | . | . | . | . | . | . | V | . | . | . | . | . |
| 21-225_61F2_LC | . | . | . | . | . | . | . | . | . | . | . | . | . | . | . | . | . | . | . | . | V | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_150E2_LC | . | . | . | . | . | G | . | . | . | . | . | . | . | . | . | . | . | . | . | . | . | . | . | . | . | . |
| 21-225_152F7_LC | . | . | . | . | . | . | . | . | . | . | . | . | . | . | . | . | . | . | . | . | . | . | . | . | . | . |
| 21-225_176H12_LC | . | . | . | . | . | . | . | . | . | . | . | . | . | . | . | . | . | . | . | . | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | A | . | . | . | . | . | . | . | . | . | D | . | . | . | . | . | . | . | S | . | . | . | . | . |
| 21-225_26A11_LC | . | . | A | . | . | . | . | . | . | . | . | . | . | . | . | . | . | . | . | . | S | . | . | . | . | . |
| 21-225_28H2_LC | . | . | . | . | . | . | . | . | . | . | . | . | . | . | . | . | . | . | . | . | . | . | . | . | . | . |
| 21-225_29E2_LC | . | . | A | . | . | . | . | . | . | . | . | . | . | . | . | . | . | . | . | . | S | . | . | . | . | . |
| 21-225_61F2_LC | . | . | . | . | . | . | . | . | . | . | . | . | D | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_150E2_LC | T | . | . | . | . | . | . | . | . | . | . | . | . | . | . | . | . | . | . | . | . | . | . | . | . | . |
| 21-225_152F7_LC | . | . | . | . | . | . | . | . | . | . | . | . | . | . | Y | . | . | . | . | . | . | . | . | . | . | . |
| 21-225_176H12_LC | N | . | . | . | . | . | . | . | . | . | . | . | . | . | . | . | . | . | . | . | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | . | . | . | . | . | . | . | . | . | . | . | . | . | . | . | . | . | . | . | . | . | . | . | . |
| 21-225_26A11_LC | . | . | . | . | . | . | . | . | . | . | . | . | . | . | . | . | . | . | . | . | . | . | . | . | . | . |
| 21-225_28H2_LC | . | . | . | . | . | . | . | . | . | . | . | . | . | . | . | . | . | . | . | . | . | . | . | . | . | . |
| 21-225_29E2_LC | . | . | . | . | . | . | . | . | . | . | . | . | . | . | . | . | . | . | . | . | . | . | . | . | . | . |
| 21-225_61F2_LC | N | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | K_FR3 | | | | | | | | | | | | | | | | |
| CONSENSUS | S | G | S | G | S | G | - | - | T | E | F | T | L | T | I | S | S | L | Q | P | E | D | F | A | T | Y |
| 21-225_150E2_LC | . | . | . | . | . | . | . | . | . | . | . | . | . | . | . | . | . | . | . | . | A | . | . | . | . | . |
| 21-225_152F7_LC | . | . | . | . | . | . | . | . | . | . | . | . | . | . | . | . | . | . | . | . | . | . | . | . | . | . |
| 21-225_176H12_LC | . | . | . | . | . | . | . | . | . | . | . | . | . | . | . | . | . | . | S | . | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | . | . | . | . | . | . | . | . | F | . | . | . | . | . | . | . | . | . | . | . | . | . | . | . |
| 21-225_26A11_LC | . | . | . | . | . | . | . | . | . | . | F | . | . | . | . | . | . | . | . | . | . | . | . | . | . | . |
| 21-225_28H2_LC | . | . | . | . | . | . | . | . | . | . | . | . | . | . | . | . | . | . | . | . | . | . | . | . | . | . |
| 21-225_29E2_LC | . | . | . | . | . | . | . | . | . | . | F | . | . | . | . | . | . | . | . | . | . | . | . | . | . | . |
| 21-225_61F2_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_150E2_LC | . | . | . | . | . | . | . | . | . | . | . | . | . | . | . | . | . | . | . | . | . | . | . | . | . | . |
| 21-225_152F7_LC | . | . | . | . | . | . | N | . | . | . | . | . | . | . | . | . | . | . | . | . | . | . | . | . | . | . |
| 21-225_176H12_LC | . | Ø | . | . | . | N | . | . | . | . | . | . | . | . | . | . | . | . | . | . | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | . | . | . | . | N | . | . | . | . | . | . | . | . | . | . | . | . | . | . | . | . | . | . | . |
| 21-225_26A11_LC | . | . | . | . | . | . | N | . | . | . | . | . | . | . | . | . | . | . | . | . | . | . | . | . | . | . |
| 21-225_28H2_LC | . | . | . | . | . | . | . | . | . | . | . | . | . | . | . | . | . | . | . | . | . | . | . | . | . | . |
| 21-225_29E2_LC | . | . | . | . | . | . | N | . | . | . | . | . | . | . | . | . | . | . | . | . | . | . | . | . | . | . |
| 21-225_61F2_LC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | R | S | F | G | Q | G | T | K | L | E | I | K | R |
| 21-225_150E2_LC | . | . | . | . | N | . | Y | . | . | . | . | . | . | . | . | . | . | . | . |
| 21-225_152F7_LC | . | . | . | . | N | . | Y | . | . | . | . | . | . | . | . | . | . | . | . |
| 21-225_176H12_LC | . | . | . | . | . | . | . | . | . | . | . | . | . | . | . | . | . | . | . |
| 21-225_177B4_LC | . | . | . | . | N | . | . | . | . | . | . | . | . | . | . | . | . | . | . |
| 21-225_25A12_LC | . | . | . | . | . | . | . | . | . | . | . | . | . | . | . | . | . | . | . |
| 21-225_26A11_LC | . | . | . | . | . | . | . | . | . | . | . | . | . | . | . | . | . | N | . |
| 21-225_28H2_LC | . | . | . | . | . | . | . | . | . | . | . | . | . | . | . | . | . | . | . |
| 21-225_29E2_LC | . | . | . | . | . | . | . | . | . | . | . | . | . | . | . | . | . | . | . |
| 21-225_61F2_LC | . | . | . | . | . | . | . | . | . | . | . | . | . | . | . | . | . | . | . |

**FIGURE 57**

Table 129.    Consensus 49 - VK1|L1/JK5 (SEQ ID NO: 50346):

DIQMTQSPSSLSASVGDRVTITCRAS--QGIS------NYLAWFQQKPGKAPKSLIYA--------ASSLQSGVPSKFSGSGSG--
TDFTLTISSLQPEDFATYYCQQYLS----------------------YPITFGQGTRLEIKR wherein:

R at position 24 can be substituted with Q

G at position 30 can be substituted with D

S at position 32 can be substituted with N

N at position 39 can be substituted with K

Y at position 40 can be substituted with F

A at position 42 can be substituted with N or V

A at position 58 can be substituted with D, G, or T

S at position 68 can be substituted with T

S at position 69 can be substituted with R or N

Q at position 71 can be substituted with H, L. or V

S at position 72 can be substituted with T

Q at position 107 can be substituted with H or L

Q at position 108 can be substituted with H or L

L at position 110 can be substituted with H, D, K, N, or Y

S at position 111 can be substituted with N, H, or T

Y at position 135 can be substituted with L

**FIGURE 57**

I at position 137 can be substituted with L

Xaa Ala Ser Gln Xaa Ile Xaa Xaa Xaa Leu Xaa (SEQ ID NO: 50212)

Xaa Ala Xaa Xaa Leu Xaa Xaa (SEQ ID NO: 50213)

Xaa Xaa Tyr Xaa Xaa Xaa Pro Xaa Thr (SEQ ID NO: 50214)

Arg Ala Ser Gln Gly Ile Ser Asn Tyr Leu Ala (SEQ ID NO: 50708)

Ala Ala Ser Ser Leu Gln Ser (SEQ ID NO: 50709)

Gln Gln Tyr Leu Ser Tyr Pro Ile Thr (SEQ ID NO: 50710)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_151C9_LC | . | . | . | . | . | . | . | . | . | . | . | . | . | . | . | . | . | . | . | . | . | . | . | . | . | . |
| 21-225_157H7_LC | . | . | . | . | . | . | . | . | . | . | . | . | . | . | . | . | . | . | . | . | . | . | . | . | . | . |
| 21-225_194B7_LC | . | . | . | . | . | . | . | . | . | . | . | . | . | . | . | . | . | . | . | . | . | . | . | . | . | . |
| 21-225_196D4_LC | . | . | . | . | . | . | . | . | . | . | . | . | . | . | . | . | . | . | . | . | . | . | . | . | . | . |
| 21-225_20A8_LC | . | . | . | . | . | . | . | . | . | . | . | . | . | . | . | . | . | . | . | . | . | . | . | . | . | . |
| 21-225_2C12_LC | . | . | . | . | . | . | . | . | . | . | . | . | . | . | . | . | . | . | . | . | . | . | . | . | . | . |
| 21-225_33B7_LC | . | . | . | . | . | . | . | . | . | . | . | . | . | . | . | . | . | . | . | . | . | . | . | . | . | . |
| 21-225_33H3_LC | . | . | . | . | . | . | . | . | . | . | . | . | . | . | . | . | . | . | . | . | . | . | . | Q | . | . |
| 21-225_87B7_LC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | K_CDR1 | | | | | | | | | | | | | | | | | | K_FR2 | | | |
| CONSENSUS | - | - | Q | G | I | S | - | - | - | - | - | - | N | Y | L | A | W | F | Q | Q | K | P | G | K | A | P |
| 21-225_151C9_LC | . | . | . | . | . | . | . | . | . | . | . | . | K | . | . | . | . | . | . | . | . | . | . | . | . | . |
| 21-225_157H7_LC | . | . | D | . | . | . | . | . | . | . | . | . | . | F | . | . | . | . | . | . | . | . | . | . | . | . |
| 21-225_194B7_LC | . | . | . | . | . | . | . | . | . | . | . | . | K | . | . | . | . | . | . | . | . | . | . | . | . | . |
| 21-225_196D4_LC | . | . | . | . | . | . | . | . | . | . | . | . | . | . | . | . | . | . | . | . | N | . | . | . | . | . |
| 21-225_20A8_LC | . | . | . | . | N | . | . | . | . | . | . | . | . | . | V | . | . | . | . | . | . | . | . | . | . | . |
| 21-225_2C12_LC | . | . | . | . | . | . | . | . | . | . | . | . | . | . | . | . | . | . | . | . | . | S | . | . | . | . |
| 21-225_33B7_LC | . | . | D | . | . | . | . | . | . | . | . | . | . | . | . | . | . | . | . | . | . | . | . | . | . | . |
| 21-225_33H3_LC | . | . | D | . | . | . | . | . | . | . | . | . | F | . | N | . | . | R | . | . | . | . | . | . | . | . |
| 21-225_87B7_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | K_CDR2 | | | | | | | | | | | | | | | |
| CONSENSUS | K | S | L | I | Y | A | - | - | - | - | - | - | - | - | A | S | S | L | Q | S | G | V | P | S | K | F |
| 21-225_151C9_LC | . | . | . | . | . | G | | | | | | . | . | | . | . | % | . | | | | | | | | |
| 21-225_157H7_LC | . | . | . | V | S | T | | | | | | . | . | | . | . | . | . | | | | | | | | |
| 21-225_194B7_LC | . | . | . | . | . | G | | | | | | . | . | | . | . | % | . | | | | | | | | |
| 21-225_196D4_LC | . | . | . | . | . | . | | | | | | . | . | | . | . | . | . | | | | | | | | |
| 21-225_20A8_LC | . | . | . | . | . | . | | | | | | . | . | | . | . | . | . | L | | | | | | | |
| 21-225_2C12_LC | . | . | . | . | S | . | | | | | | . | . | | . | T | . | . | | | | | | | Q | . |
| 21-225_33B7_LC | T | . | . | . | . | . | | | | | | . | . | | . | . | . | . | | | | | | | | |
| 21-225_33H3_LC | N | . | . | . | . | G | | | | | | . | . | | . | N | . | . | V | T | | | | | % | |
| 21-225_87B7_LC | . | . | . | . | . | . | | | | | | . | . | | . | . | % | . | | | | | | | | |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_151C9_LC | . | . | . | . | . | . | . | . | . | . | . | . | . | . | . | . | . | . | . | . | . | . | . | . | G | . |
| 21-225_157H7_LC | . | . | . | . | . | . | . | . | . | . | . | . | . | . | . | . | . | . | . | . | . | . | . | . | . | . |
| 21-225_194B7_LC | . | . | . | . | . | . | . | . | . | . | . | . | . | . | . | . | . | . | . | . | . | . | . | . | . | . |
| 21-225_196D4_LC | . | . | . | . | C | . | . | . | . | . | . | . | . | . | . | . | . | % | . | . | . | . | V | . | . | . |
| 21-225_20A8_LC | . | . | . | . | . | . | . | . | . | . | . | . | . | . | . | . | . | . | . | . | . | . | . | . | . | . |
| 21-225_2C12_LC | . | . | . | . | . | . | . | . | . | . | . | . | . | . | . | . | . | . | . | . | . | . | . | . | . | % |
| 21-225_33B7_LC | . | . | . | . | . | . | . | . | . | . | . | . | . | . | . | . | . | . | . | . | . | . | . | . | . | % |
| 21-225_33H3_LC | . | . | % | . | . | . | . | . | . | . | . | % | . | . | . | . | . | . | . | . | . | . | . | . | . | . |
| 21-225_87B7_LC | . | . | . | . | . | . | . | . | . | . | . | . | . | . | . | . | . | . | . | . | . | . | S | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | L | S | - | - | - | - | - | - | - | - | - | - | - | - | - | - | - | - | - | - | - |
| 21-225_151C9_LC | . | . | M | . | . | K | H | . | . | . | . | . | . | . | . | . | . | . | . | . | . | . | . | . | . | . |
| 21-225_157H7_LC | . | . | . | . | . | H | . | . | . | . | . | . | . | . | . | . | . | . | . | . | . | . | . | . | . | . |
| 21-225_194B7_LC | . | . | . | M | . | . | N | . | . | . | . | . | . | . | . | . | . | . | . | . | . | . | . | . | . | . |
| 21-225_196D4_LC | . | . | . | M | . | . | N | . | . | . | . | . | . | . | . | . | . | . | . | . | . | . | . | . | . | . |
| 21-225_20A8_LC | . | . | . | . | . | N | . | . | . | . | . | . | . | . | . | . | . | . | . | . | . | . | . | . | . | . |
| 21-225_2C12_LC | . | . | . | . | . | Y | . | . | . | . | . | . | . | . | . | . | . | . | . | . | . | . | . | . | . | . |
| 21-225_33B7_LC | . | . | . | . | . | H | . | . | . | . | . | . | . | . | . | . | . | . | . | . | . | . | . | . | . | . |
| 21-225_33H3_LC | . | . | . | . | . | O | N | . | . | . | . | . | . | . | . | . | . | . | . | . | . | . | . | . | . | . |
| 21-225_87B7_LC | F | . | L | L | . | . | T | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | K_FR4 | | | | | | | |
| CONSENSUS | - | - | - | - | Y | P | I | T | F | G | Q | G | T | R | L | E | I | K | R |
| 21-225_151C9_LC | . | . | . | . | . | . | . | . | . | . | . | . | . | . | . | . | . | . | . |
| 21-225_157H7_LC | . | . | . | . | . | . | . | . | . | . | . | . | . | . | . | . | . | . | . |
| 21-225_194B7_LC | . | . | . | . | . | . | L | . | . | . | . | . | . | . | . | . | . | . | . |
| 21-225_196D4_LC | . | . | . | . | . | . | L | . | . | . | . | . | . | . | . | . | . | . | . |
| 21-225_20A8_LC | . | . | . | . | . | . | . | . | . | . | . | . | . | . | . | . | . | . | . |
| 21-225_2C12_LC | . | . | . | . | . | . | . | . | . | . | . | . | . | . | . | . | . | . | . |
| 21-225_33B7_LC | . | . | . | . | . | . | L | . | . | . | . | . | . | . | . | . | . | . | . |
| 21-225_33H3_LC | . | . | . | . | L | . | . | . | . | . | . | . | . | . | . | . | . | . | . |
| 21-225_87B7_LC | . | . | . | . | . | . | L | . | . | . | . | . | . | . | . | . | . | . | . |

**FIGURE 57**

Table 130. Consensus 50 - VK6|A26/JK4 (SEQ ID NO: 50347):

EIVLTQSPDFQSVTPKEKVTITCRAS--QSIG------SSLHWYQQKPDQSPKLLIKY-------ASQSFSGVPSRFSGSGSG--
TDFTLTINSLEAEDAATYYCHQSRR----------------------LPLTFGGGTKVEIKR wherein:

S at position 26 can be substituted with N

S at position 30 can be substituted with N

S at position 39 can be substituted with R

F at position 71 can be substituted with L

S at position 109 can be substituted with T

R at position 110 can be substituted with G or S

R at position 111 can be substituted with S or T

Xaa Ala Xaa Gln Ser Xaa Gly Ser Ser Leu His (SEQ ID NO: 50215)

Tyr Ala Ser Gln Ser Xaa Ser (SEQ ID NO: 50216)

His Gln Xaa Xaa Xaa Leu Pro Leu Thr (SEQ ID NO: 50217)

Arg Ala Ser Gln Ser Ile Gly Ser Ser Leu His (SEQ ID NO: 50711)

Tyr Ala Ser Gln Ser Phe Ser (SEQ ID NO: 50712)

His Gln Ser Arg Arg Leu Pro Leu Thr (SEQ ID NO: 50713)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | K_FR1 | | | | | | | | | | | | | |
| CONSENSUS | E | I | V | L | T | Q | S | P | D | F | Q | S | V | T | P | K | E | K | V | T | I | T | C | R | A | S |
| 21-225_190B12_LC | . | . | . | . | . | . | . | . | . | . | . | . | . | . | . | . | . | . | . | . | . | . | . | . | . | . |
| 21-225_191C9_LC | . | . | . | . | . | . | . | . | . | . | . | . | . | . | . | . | . | . | . | . | . | . | . | . | . | . |
| 21-225_191H4_LC | . | . | . | . | . | . | . | . | . | . | . | . | . | . | . | . | . | . | . | . | . | . | . | . | . | N |
| 21-225_192E5_LC | . | . | . | . | . | . | . | . | . | . | . | . | . | . | . | . | . | . | . | . | . | . | . | . | . | . |
| 21-225_193G9_LC | . | . | . | . | . | . | . | . | . | . | . | . | . | . | . | . | . | . | . | . | . | . | . | . | . | . |
| 21-225_195B10_LC | . | . | . | . | . | . | . | . | . | . | . | . | . | . | . | . | . | . | . | . | . | . | . | . | . | . |
| 21-225_195F2_LC | . | . | . | . | . | . | . | . | . | . | . | . | . | . | . | . | . | . | . | . | . | . | . | . | . | . |
| 21-225_201E8_LC | . | . | . | . | . | . | . | A | . | . | . | . | . | . | . | . | . | . | . | . | . | . | . | . | . | . |
| 21-225_206G4_LC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | K_CDR1 | | | | | | | | | | | | | | | | K_FR2 | | | | | | | |
| CONSENSUS | - | - | Q | S | I | G | - | - | - | - | - | - | S | S | L | H | W | Y | Q | Q | K | P | D | Q | S | P |
| 21-225_190B12_LC | . | . | . | . | . | . | . | . | . | . | . | . | . | . | . | . | . | . | . | . | . | . | . | . | . | . |
| 21-225_191C9_LC | . | . | . | . | . | . | . | . | . | . | . | . | . | . | . | . | . | . | . | . | . | . | . | . | . | . |
| 21-225_191H4_LC | . | . | . | . | . | . | . | . | . | . | . | . | . | . | . | . | . | . | . | . | . | . | . | . | . | . |
| 21-225_192E5_LC | . | . | . | . | . | . | . | . | . | . | . | . | R | . | . | . | . | . | . | . | . | . | . | . | . | . |
| 21-225_193G9_LC | . | . | . | . | . | . | . | . | . | . | . | . | P | . | . | . | . | . | . | . | S | . | . | . | . | L |
| 21-225_195B10_LC | . | . | . | . | . | . | . | . | . | . | . | . | . | . | . | . | . | . | . | . | . | . | . | . | . | . |
| 21-225_195F2_LC | . | . | . | . | . | . | . | . | . | . | . | . | . | . | . | . | . | . | . | . | . | . | . | . | . | . |
| 21-225_201E8_LC | . | . | N | . | . | . | . | . | . | . | . | . | R | . | . | . | . | . | . | . | . | . | . | . | . | . |
| 21-225_206G4_LC | . | . | . | . | . | . | . | . | . | . | . | . | R | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | K | Y | - | - | - | - | - | - | - | - | A | S | Q | S | F | S | G | V | P | S | R | F |
| 21-225_190B12_LC | . | . | . | . | . | . | | . | . | . | . | . | . | . | . | . | . | . | . | . | . | D | . | . | . | . |
| 21-225_191C9_LC | . | . | . | . | . | . | | . | . | . | . | . | . | . | . | . | . | . | . | . | . | D | . | . | . | . |
| 21-225_191H4_LC | . | . | . | . | . | . | | . | . | . | . | . | . | . | . | . | . | . | L | . | . | . | . | . | . | . |
| 21-225_192E5_LC | . | . | . | . | . | . | | . | . | . | . | . | . | . | . | . | . | . | . | . | . | . | . | . | . | . |
| 21-225_193G9_LC | . | . | . | . | . | . | | . | . | . | . | . | . | . | . | . | . | . | . | . | . | . | . | . | . | . |
| 21-225_195B10_LC | . | . | . | . | . | . | | . | . | . | . | . | . | . | . | . | . | . | . | . | . | . | . | . | . | . |
| 21-225_195F2_LC | . | . | . | . | . | . | | . | . | . | . | . | . | . | . | . | . | . | . | . | . | . | . | . | . | . |
| 21-225_201E8_LC | . | . | . | . | . | . | | . | . | . | . | . | . | . | . | . | . | . | . | . | . | . | . | . | . | . |
| 21-225_206G4_LC | . | . | . | . | . | . | | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | N | S | L | E | A | E | D | A | A | T | Y |
| 21-225_190B12_LC | . | . | . | . | . | . | | | . | . | . | . | . | . | . | . | . | . | . | . | . | . | . | . | . | . |
| 21-225_191C9_LC | . | . | . | . | . | . | | | . | . | . | . | . | . | . | . | . | . | . | . | . | . | . | . | . | . |
| 21-225_191H4_LC | A | . | . | . | . | . | | | . | . | . | . | . | . | . | . | . | . | D | . | . | . | . | . | . | . |
| 21-225_192E5_LC | . | . | . | . | . | . | | | . | . | . | . | . | . | . | . | . | . | . | . | . | . | . | . | . | . |
| 21-225_193G9_LC | . | . | . | . | . | . | | | . | . | . | . | . | . | . | . | . | . | . | . | . | . | . | . | . | . |
| 21-225_195B10_LC | . | . | . | . | . | . | | | . | . | . | . | . | . | . | . | . | . | . | . | . | . | . | . | . | . |
| 21-225_195F2_LC | . | . | . | . | . | . | | | . | . | A | . | . | . | . | S | . | . | . | . | . | . | . | . | . | . |
| 21-225_201E8_LC | . | . | . | . | . | . | | | N | . | . | . | . | . | . | . | . | . | . | . | . | . | . | V | . | . |
| 21-225_206G4_LC | . | . | . | . | . | . | | | . | . | . | . | . | . | . | . | . | . | . | . | . | . | . | . | . | . |

# EP 4 435 105 A2

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | H | Q | S | R | - | - | - | - | - | - | - | - | - | - | - | - | - | - | - | - | - | - | - | - |
| 21-225_190B12_LC | . | . | . | . | Y | . | S | . | . | . | . | . | . | . | . | . | . | . | . | . | . | . | . | . | . | . |
| 21-225_191C9_LC | . | . | . | . | Y | . | S | . | . | . | . | . | . | . | . | . | . | . | . | . | . | . | . | . | . | . |
| 21-225_191H4_LC | . | . | . | . | Y | G | R | . | . | . | . | . | . | . | . | . | . | . | . | . | . | . | . | . | . | . |
| 21-225_192E5_LC | . | . | . | . | . | S | R | . | . | . | . | . | . | . | . | . | . | . | . | . | . | . | . | . | . | . |
| 21-225_193G9_LC | R | . | . | . | . | S | R | . | . | . | . | . | . | . | . | . | . | . | . | . | . | . | . | . | . | . |
| 21-225_195B10_LC | . | . | . | . | . | G | R | . | . | . | . | . | . | . | . | . | . | . | . | . | . | . | . | . | . | . |
| 21-225_195F2_LC | . | . | . | . | . | . | Y | . | . | . | . | . | . | . | . | . | . | . | . | . | . | . | . | . | . | . |
| 21-225_201E8_LC | . | . | . | . | . | . | S | . | . | . | . | . | . | . | . | . | . | . | . | . | . | . | . | . | . | . |
| 21-225_206G4_LC | . | . | . | . | . | . | S | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | L | P | L | T | F | G | G | G | T | K | V | E | I | K | R |
| 21-225_190B12_LC | . | . | . | . | . | . | . | . | . | . | . | . | . | . | . | . | . | . | . |
| 21-225_191C9_LC | . | . | . | . | . | . | . | . | . | . | . | . | . | . | . | . | . | . | . |
| 21-225_191H4_LC | . | . | . | . | . | . | . | . | . | . | . | . | . | . | . | . | . | . | . |
| 21-225_192E5_LC | . | . | . | . | . | . | . | . | . | . | . | . | . | . | . | . | . | . | . |
| 21-225_193G9_LC | . | . | . | . | . | . | . | . | . | . | . | . | . | . | . | . | . | . | . |
| 21-225_195B10_LC | . | . | . | . | . | . | . | . | . | . | . | . | . | . | . | . | . | . | . |
| 21-225_195F2_LC | . | . | . | . | . | . | . | . | . | . | . | . | . | . | . | . | . | . | . |
| 21-225_201E8_LC | . | . | . | . | . | . | . | . | . | . | . | . | . | . | . | . | . | R | . |
| 21-225_206G4_LC | . | . | . | . | . | . | . | . | . | . | . | . | . | . | . | . | . | . | . |

5027

FIGURE 57

Table 131.    Consensus 51 - VL3|3l/JL2 (SEQ ID NO: 50348):

SSELTQD-PAVSVALGQTVRITCQGD---SLRP-----YYASWYQQKPGQAPVLVIYG--------KNNRPSGIPDRFSGSSSG--
NTASLTITGAQAEDEADYYCNSRDSS-------------------GNHLVVFGGGTKLTVLG wherein:

S at position 30 can be substituted with T or K

P at position 33 can be substituted with N, S, or T

A at position 41 can be substituted with V

S at position 42 can be substituted with N

G at position 58 can be substituted with A or T

N at position 69 can be substituted with S

S at position 112 can be substituted with C

G at position 133 can be substituted with null (-)

N at position 134 can be substituted with G

H at position 135 can be substituted with N or S

L at position 136 can be substituted with H

V at position 137 can be substituted with L

V at position 138 can be substituted with L

Gln Gly Asp Xaa Leu Arg Xaa Tyr Tyr Xaa Xaa (SEQ ID NO: 50218)

Xaa Lys Asn Xaa Arg Pro Ser (SEQ ID NO: 50219)

Asn Ser Arg Asp Ser Xaa Xaa Xaa Xaa Xaa Xaa Xaa (SEQ ID NO: 50220)

**FIGURE 57**

Gln Gly Asp Ser Leu Arg Pro Tyr Tyr Ala Ser (SEQ ID NO: 50714)

Gly Lys Asn Asn Arg Pro Ser (SEQ ID NO: 50715)

Asn Ser Arg Asp Ser Ser Gly Asn His Leu Val Val (SEQ ID NO: 50716)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | L_FR1 | | | | | | | | | | | | | | | | | | |
| CONSENSUS | S | S | E | L | T | Q | D | - | P | A | V | S | V | A | L | G | Q | T | V | R | I | T | C | Q | G | D |
| 21-225_171D12_LC | . | . | . | . | . | . | . | | . | . | . | . | . | . | . | . | . | . | . | . | . | . | . | . | . | . |
| 21-225_172B12_LC | . | . | . | . | . | . | . | | . | . | . | . | . | . | . | . | . | . | . | . | . | . | . | . | . | . |
| 21-225_172C3_LC | . | . | . | . | . | . | . | | . | . | . | . | . | . | . | . | . | . | . | . | . | . | . | . | . | . |
| 21-225_190B3_LC | . | . | . | . | . | . | . | | . | . | . | . | . | . | . | . | . | . | . | . | . | . | . | . | . | . |
| 21-225_190B8_LC | . | . | . | . | . | . | . | | . | . | . | . | . | . | . | . | . | . | . | . | . | . | . | . | . | . |
| 21-225_190C10_LC | . | . | . | . | . | . | . | | . | . | . | . | . | . | . | . | . | . | . | . | . | . | . | . | . | . |
| 21-225_191A9_LC | . | . | . | . | . | . | . | | . | . | . | . | . | . | . | . | . | . | . | . | . | . | . | . | . | . |
| 21-225_191B8_LC | . | . | . | . | . | . | . | | . | . | . | . | . | . | . | . | . | . | . | . | . | . | . | . | . | . |
| 21-225_193A6_LC | . | . | . | . | . | . | . | | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | L_CDR1 | | | | | | | | | | | | | | | | | L_FR2 | | | |
| CONSENSUS | - | - | - | S | L | R | P | - | - | - | - | - | Y | Y | A | S | W | Y | Q | Q | K | P | G | Q | A | P |
| 21-225_171D12_LC | . | . | . | . | . | . | S | . | . | . | . | . | . | . | . | . | . | . | . | . | . | . | . | . | . | . |
| 21-225_172B12_LC | . | . | . | . | . | . | N | . | . | . | . | . | . | . | . | . | . | . | . | . | . | . | . | . | . | . |
| 21-225_172C3_LC | . | . | . | . | . | . | N | . | . | . | . | . | . | . | V | . | . | . | . | . | . | . | . | . | . | . |
| 21-225_190B3_LC | . | . | K | . | . | . | T | . | . | . | . | . | . | . | . | . | . | . | . | . | . | . | . | . | . | . |
| 21-225_190B8_LC | . | . | . | T | . | . | . | . | . | . | . | . | . | . | V | . | . | . | . | . | . | . | . | . | . | . |
| 21-225_190C10_LC | . | . | . | . | . | . | . | . | . | . | . | . | . | . | . | . | . | . | . | . | . | . | . | . | . | . |
| 21-225_191A9_LC | . | . | . | . | . | . | . | . | . | . | . | . | . | . | . | . | . | . | . | . | . | . | . | . | . | . |
| 21-225_191B8_LC | . | . | . | . | . | T | . | . | . | . | . | . | . | . | . | . | . | . | . | . | . | . | . | . | . | . |
| 21-225_193A6_LC | . | . | . | . | . | . | S | . | . | . | . | . | . | . | . | N | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | L_CDR2 | | | | | | | | | | | | | | |
| CONSENSUS | V | L | V | I | Y | G | - | - | - | - | - | - | - | - | K | N | N | R | P | S | G | I | P | D | R | F |
| 21-225_171D12_LC | I | . | . | . | . | A | . | . | . | . | . | . | . | . | . | . | . | . | . | . | . | . | . | . | . | . |
| 21-225_172B12_LC | I | . | . | . | . | T | . | . | . | . | . | . | . | . | . | . | S | . | . | . | . | . | . | . | . | . |
| 21-225_172C3_LC | I | . | . | . | . | T | . | . | . | . | . | . | . | . | . | . | S | . | . | . | . | . | . | . | . | . |
| 21-225_190B3_LC | . | . | . | V | . | . | . | . | . | . | . | . | . | . | . | . | . | . | . | . | . | . | . | . | . | . |
| 21-225_190B8_LC | . | . | . | . | . | . | . | . | . | . | . | . | . | . | . | . | . | . | . | . | . | . | . | . | . | . |
| 21-225_190C10_LC | . | . | . | . | . | . | . | . | . | . | . | . | . | . | . | . | . | . | . | . | . | . | . | . | . | . |
| 21-225_191A9_LC | . | . | . | . | . | . | . | . | . | . | . | . | . | . | . | . | . | . | . | . | . | . | . | . | . | . |
| 21-225_191B8_LC | . | . | . | . | . | . | . | . | . | . | . | . | . | . | . | . | . | . | . | . | . | . | S | . | . | . |
| 21-225_193A6_LC | . | . | S | . | . | A | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | L_FR3 | | | | | | | | | | | | | | | |
| CONSENSUS | S | G | S | S | S | G | - | - | N | T | A | S | L | T | I | T | G | A | Q | A | E | D | E | A | D | Y |
| 21-225_171D12_LC | . | . | N | . | . | . | . | . | . | . | . | . | . | . | . | . | . | . | . | . | . | . | . | . | . | . |
| 21-225_172B12_LC | . | . | T | . | . | . | . | . | . | . | . | . | . | . | . | . | . | . | . | . | . | . | . | . | . | . |
| 21-225_172C3_LC | . | . | T | . | . | . | . | . | . | . | . | . | . | . | . | . | . | T | . | . | . | . | . | . | . | . |
| 21-225_190B3_LC | . | . | . | . | . | . | . | . | . | . | . | . | . | . | . | . | . | . | . | . | . | . | . | . | . | . |
| 21-225_190B8_LC | . | . | . | . | . | . | . | . | . | . | . | . | . | . | . | . | . | . | . | . | . | . | . | . | . | . |
| 21-225_190C10_LC | . | . | . | . | . | . | . | . | . | . | . | . | . | . | . | . | S | . | . | . | . | . | . | . | . | . |
| 21-225_191A9_LC | . | . | . | . | . | . | . | . | . | . | . | . | . | . | . | . | S | . | . | . | . | . | . | . | . | . |
| 21-225_191B8_LC | . | . | . | . | . | . | . | . | . | . | . | . | . | . | . | . | . | . | . | . | . | . | . | . | . | . |
| 21-225_193A6_LC | . | . | N | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | L_CDR3 | | | | | | | |
| CONSENSUS | Y | C | N | S | R | D | S | S | - | - | - | - | - | - | - | - | - | - | - | - | - | - | - | - | - | - |
| 21-225_171D12_LC | . | . | . | . | . | . | . | . | . | . | . | . | . | . | . | . | . | . | . | . | . | . | . | . | . | . |
| 21-225_172B12_LC | . | . | . | . | . | . | . | . | . | . | . | . | . | . | . | . | . | . | . | . | . | . | . | . | . | . |
| 21-225_172C3_LC | . | . | . | . | . | . | . | . | . | . | . | . | . | . | . | . | . | . | . | . | . | . | . | . | . | . |
| 21-225_190B3_LC | . | . | . | . | . | . | . | . | . | . | . | . | . | . | . | . | . | . | . | . | . | . | . | . | . | . |
| 21-225_190B8_LC | . | . | . | . | . | . | . | . | . | . | . | . | . | . | . | . | . | . | . | . | . | . | . | . | . | . |
| 21-225_190C10_LC | . | . | . | . | . | . | . | . | . | . | . | . | . | . | . | . | . | . | . | . | . | . | . | . | . | . |
| 21-225_191A9_LC | . | . | . | . | . | . | . | G | . | . | . | . | . | . | . | . | . | . | . | . | . | . | . | . | . | . |
| 21-225_191B8_LC | . | . | . | . | . | . | . | . | . | . | . | . | . | . | . | . | . | . | . | . | . | . | . | . | . | . |
| 21-225_193A6_LC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | L_FR4 | | | | | | |
| CONSENSUS | - | - | G | N | H | L | V | V | F | G | G | G | T | K | L | T | V | L | G |
| 21-225_171D12_LC | . | . | - | G | S | H | . | . | . | . | . | . | . | . | . | . | . | . | S |
| 21-225_172B12_LC | . | . | - | G | N | H | . | . | . | . | . | . | . | . | . | . | . | . | . |
| 21-225_172C3_LC | . | . | - | G | N | H | . | . | . | . | . | . | . | . | . | . | . | . | . |
| 21-225_190B3_LC | . | . | . | . | . | . | L | . | . | . | . | . | . | . | . | . | . | . | S |
| 21-225_190B8_LC | . | . | . | . | . | . | . | . | . | . | . | . | . | . | . | . | . | . | . |
| 21-225_190C10_LC | . | . | . | . | . | . | . | . | . | . | . | . | . | . | . | . | . | . | . |
| 21-225_191A9_LC | . | . | . | . | . | . | . | . | . | . | . | . | . | . | . | . | . | . | . |
| 21-225_191B8_LC | . | . | . | . | . | . | . | . | . | . | . | . | . | . | . | . | . | . | . |
| 21-225_193A6_LC | . | . | - | G | N | H | L | . | . | . | . | . | . | . | . | . | . | . | . |

FIGURE 57

Table 132.　Consensus 52 - VK2|A17/JK4 (SEQ ID NO: 50349):

DVVMTQSPLSLPVTLGQPASISCRSS--QSLVYSD-GNTYLNWFQQRPGQSPRRLIYK--------VSNWDSGVPDRFSGSGSG--
TDFTLKISRVEAEDVGVYYCMQGTH---------------------WPLTFGGGTKVEIKR wherein:

S at position 26 can be substituted with G

Y at position 40 can be substituted with S

K at position 58 can be substituted with E

N at position 69 can be substituted with K

S at position 72 can be substituted with Y

T at position 110 can be substituted with I

H at position 111 can be substituted with null (-)

W at position 135 can be substituted with H

P at position 136 can be substituted with L, S or W

L at position 137 can be substituted with P

Arg Ser Xaa Gln Ser Leu Val Tyr Ser Asp Gly Asn Thr Xaa Leu Asn (SEQ ID NO: 50221)

Xaa Val Ser Xaa Trp Asp Xaa (SEQ ID NO: 50222)

Met Gln Gly Xaa Xaa Xaa Xaa Xaa Thr (SEQ ID NO: 50223)

Arg Ser Ser Gln Ser Leu Val Tyr Ser Asp Gly Asn Thr Tyr Leu Asn (SEQ ID NO: 50717)

Lys Val Ser Asn Trp Asp Ser (SEQ ID NO: 50718)

Met Gln Gly Thr His Trp Pro Leu Thr (SEQ ID NO: 50719)

EP 4 435 105 A2

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | V | V | M | T | Q | S | P | L | S | L | P | V | T | L | G | Q | P | A | S | I | S | C | R | S | S |
| 21-225_148C6_LC | . | . | . | . | . | . | . | . | . | . | . | . | . | . | . | . | . | . | . | . | . | . | . | . | . | . |
| 21-225_148H11_LC | . | . | . | . | . | . | . | . | . | . | . | . | . | . | . | . | . | . | . | . | . | . | . | . | . | . |
| 21-225_149F2_LC | . | . | . | . | . | . | . | . | . | . | . | . | . | . | . | . | . | . | . | . | . | . | . | . | . | . |
| 21-225_150F11_LC | . | . | . | . | S | . | Y | . | . | . | . | . | S | . | . | . | . | . | . | . | . | . | . | . | . | . |
| 21-225_177D2_LC | . | . | . | . | . | . | . | . | . | . | . | . | . | . | . | . | . | . | . | . | . | . | . | . | . | . |
| 21-225_32G4_LC | . | . | . | . | . | . | . | . | . | . | S | . | . | . | . | . | . | . | . | . | . | . | . | . | . | . |
| 21-225_43F11_LC1_LC | . | . | . | . | . | . | . | . | . | . | . | . | . | . | . | . | . | . | . | . | . | . | . | . | . | . |
| 21-225_72C4_LC | . | . | . | . | . | . | . | . | . | . | . | . | . | . | . | . | . | . | . | . | . | . | . | . | . | S |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | L | V | Y | S | D | - | G | N | T | Y | L | N | W | F | Q | Q | R | P | G | Q | S | P |
| 21-225_148C6_LC | . | . | . | . | . | . | . | . | . | . | . | . | . | . | . | . | . | . | . | . | . | . | . | . | . | . |
| 21-225_148H11_LC | . | . | . | . | . | . | . | . | . | . | . | . | . | . | . | . | . | . | . | . | . | . | . | . | . | . |
| 21-225_149F2_LC | . | . | . | . | . | . | . | . | . | . | . | . | . | S | . | . | . | . | . | . | . | . | . | . | . | . |
| 21-225_150F11_LC | . | . | . | . | . | . | . | . | . | . | . | . | . | S | . | . | . | . | . | . | . | . | . | . | Y | . |
| 21-225_177D2_LC | . | . | . | . | . | . | . | . | . | . | . | . | . | . | . | . | . | . | . | . | . | . | . | . | . | . |
| 21-225_32G4_LC | . | . | . | . | . | . | . | . | . | . | . | . | . | . | . | . | . | . | . | . | . | . | . | . | . | . |
| 21-225_43F11_LC1_LC | . | . | . | . | . | . | . | . | . | . | . | . | . | . | . | . | . | . | . | . | . | . | . | . | . | . |
| 21-225_72C4_LC | . | . | . | . | . | . | . | . | . | . | . | . | . | . | . | . | . | . | . | . | . | . | . | . | . | . |

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | K_CDR2 | | | | | | | | | | | | | | |
| CONSENSUS | R | R | L | I | Y | K | - | - | - | - | - | - | - | - | V | S | N | W | D | S | G | V | P | D | R | F |
| 21-225_148C6_LC | . | . | . | . | . | . | . | . | . | . | . | . | . | . | . | . | . | . | . | . | . | . | . | . | . | . |
| 21-225_148H11_LC | . | . | . | . | . | . | . | . | . | . | . | . | . | . | . | . | . | . | . | . | . | . | . | . | . | . |
| 21-225_149F2_LC | . | . | . | . | . | . | . | . | . | . | . | . | . | . | . | . | . | . | . | . | . | . | . | V | . | . |
| 21-225_150F11_LC | . | . | . | . | . | . | . | . | . | . | . | . | . | . | . | . | . | . | Y | . | . | . | . | V | . | . |
| 21-225_177D2_LC | . | . | . | . | . | . | . | . | . | . | . | . | . | . | . | . | . | . | . | . | . | . | . | . | . | . |
| 21-225_32G4_LC | . | . | . | . | . | . | . | . | . | . | . | . | . | . | . | . | . | . | . | . | . | . | . | . | . | . |
| 21-225_43F11_LC1_LC | . | . | . | . | . | . | . | . | . | . | . | . | . | . | . | . | . | . | . | . | . | . | . | . | . | . |
| 21-225_72C4_LC | . | . | . | . | . | E | . | . | . | . | . | . | . | . | . | . | S | . | . | . | . | . | . | . | . | . |

FIGURE 57

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | W | P | L | T | F | G | G | G | T | K | V | E | I | K | R |
| 21-225_148C6_LC | . | . | . | . | | S | . | . | . | . | . | . | . | . | . | . | . | . | . |
| 21-225_148H11_LC | . | . | . | . | . | L | . | . | . | . | . | . | . | . | . | . | . | . | . |
| 21-225_149F2_LC | . | . | . | . | | | P | . | . | . | . | . | . | . | . | . | . | . | . |
| 21-225_150F11_LC | . | . | . | . | | | P | . | . | . | . | . | . | . | . | . | . | . | T |
| 21-225_177D2_LC | . | . | . | . | . | . | . | . | . | . | . | . | . | . | . | . | . | . | . |
| 21-225_32G4_LC | . | . | . | . | H | W | . | . | . | . | . | . | . | . | . | . | . | . | . |
| 21-225_43F11_LC1_LC | . | . | . | . | . | . | . | . | . | . | . | . | . | . | . | . | . | . | . |
| 21-225_72C4_LC | . | . | . | . | . | . | . | . | . | . | . | . | . | . | . | . | . | . | . |

**FIGURE 57**

Table 133.    Consensus 53 - VK3|L2/JK4 (SEQ ID NO: 50350):

EIVMTQSPATLSVSPGERATLSCRAS--QSVS------RNLAWYQQKPGQAPRLLIYG-------ASTRATGIPARFSGSGSG--
TEFTLTISSLQSEDFAVYYCQQYNN----------------------WPLTFGGGTKVEIKR wherein:

A at position 25 can be substituted with P or T

V at position 31 can be substituted with F

S at position 32 can be substituted with R or W

R at position 39 can be substituted with I or S

N at position 40 can be substituted with D or S

L at position 41 can be substituted with V

G at position 58 can be substituted with D

S at position 68 can be substituted with A

T at position 69 can be substituted with I or A

N at position 110 can be substituted with Y

N at position 111 can be substituted with T or Y

null(-) at position 134 can be substituted with W

W at position 135 can be substituted with P

Arg Xaa Ser Gln Ser Xaa Xaa Xaa Xaa Xaa Ala (SEQ ID NO: 50224)

Xaa Ala Xaa Xaa Arg Ala Thr (SEQ ID NO: 50225)

Gln Gln Tyr Xaa Xaa Xaa Xaa Pro Leu Thr (SEQ ID NO: 50226)

Arg Ala Ser Gln Ser Val Ser Arg Asn Leu Ala (SEQ ID NO: 50720)

**FIGURE 57**

Gly Ala Ser Thr Arg Ala Thr (SEQ ID NO: 50721)

Gln Gln Tyr Asn Asn Trp Pro Leu Thr (SEQ ID NO: 50722)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | E | I | V | M | T | Q | S | P | A | T | L | S | V | S | P | G | E | R | A | T | L | S | C | R | A | S |
| 21-225_190E11_LC | . | . | . | . | . | . | . | . | . | . | . | . | . | . | . | . | . | . | . | . | . | . | . | . | . | . |
| 21-225_191E8_LC | . | . | . | . | . | . | . | . | . | . | . | . | . | . | . | . | . | . | . | . | . | . | . | . | S | . |
| 21-225_191G11_LC | . | . | . | . | . | . | . | . | . | . | . | . | . | . | . | . | . | . | N | . | . | . | . | . | T | . |
| 21-225_199A6_LC | D | . | . | . | . | . | . | . | . | . | . | . | . | . | . | . | D | . | . | . | . | . | . | . | S | . |
| 21-225_55E1_LC | . | . | . | . | . | . | . | . | . | . | . | . | . | . | . | . | . | . | . | . | . | . | . | . | . | . |
| 21-225_65C12_LC | . | . | . | . | . | . | . | . | . | . | . | . | . | P | . | . | Q | . | . | . | . | . | . | . | . | . |
| 21-225_74D1_LC | . | . | . | . | . | . | . | . | . | . | . | . | . | . | . | . | . | . | . | . | . | . | . | . | . | . |
| 21-225_75F11_LC | . | . | . | . | . | . | . | . | . | . | . | P | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | K_CDR1 | | | | | | | | | | | | | | | | | | K_FR2 | | |
| CONSENSUS | - | - | Q | S | V | S | - | - | - | - | - | - | R | N | L | A | W | Y | Q | Q | K | P | G | Q | A | P |
| 21-225_190E11_LC | . | . | . | . | P | R | . | . | . | . | . | . | I | . | . | . | . | . | . | . | R | . | . | . | . | . |
| 21-225_191E8_LC | . | . | . | . | P | . | . | . | . | . | . | . | . | . | . | . | . | . | . | . | . | . | . | . | . | . |
| 21-225_191G11_LC | . | . | . | . | W | . | . | . | . | . | . | . | I | S | V | . | . | . | H | . | . | . | . | . | . | . |
| 21-225_199A6_LC | . | . | . | . | P | . | . | . | . | . | . | . | I | . | . | . | . | . | . | . | . | . | . | . | . | . |
| 21-225_55E1_LC | . | . | . | . | . | . | . | . | . | . | . | . | S | D | . | . | . | . | . | L | . | . | . | . | . | . |
| 21-225_65C12_LC | . | . | . | . | . | . | . | . | . | . | . | . | I | . | . | . | . | . | . | . | . | . | . | . | . | . |
| 21-225_74D1_LC | . | . | . | . | . | . | . | . | . | . | . | . | . | . | . | . | P | . | . | . | . | . | . | . | . | . |
| 21-225_75F11_LC | . | . | . | . | . | . | . | . | . | . | . | . | . | . | . | . | P | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | R | L | L | I | Y | G | - | - | - | - | - | - | - | - | A | S | T | R | A | T | G | I | P | A | R | F |
| 21-225_190E11_LC | . | . | . | . | . | . | . | . | . | . | . | . | . | . | . | . | . | . | . | . | . | . | . | . | . | . |
| 21-225_191E8_LC | . | . | . | . | . | . | . | . | . | . | . | . | . | . | . | . | . | . | . | . | . | . | . | S | . | . |
| 21-225_191G11_LC | . | . | . | . | . | . | . | . | . | . | . | . | . | . | . | A | . | . | . | . | . | . | . | . | . | . |
| 21-225_199A6_LC | . | . | . | . | . | . | . | . | . | . | . | . | . | . | . | . | . | . | . | . | . | . | . | . | . | . |
| 21-225_55E1_LC | . | . | . | . | . | D | . | . | . | . | . | . | . | . | . | A | . | . | . | . | . | . | . | . | . | . |
| 21-225_65C12_LC | . | . | . | . | . | . | . | . | . | . | . | . | . | . | . | . | . | . | . | . | . | . | . | . | . | . |
| 21-225_74D1_LC | . | . | . | . | . | . | . | . | . | . | . | . | . | . | . | . | I | . | . | . | . | . | . | . | . | . |
| 21-225_75F11_LC | . | . | . | S | . | . | . | . | . | . | . | . | . | . | . | . | I | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | K_FR3 | | | | | | | | | | | | | | |
| CONSENSUS | S | G | S | G | S | G | - | - | T | E | F | T | L | T | I | S | S | L | Q | S | E | D | F | A | V | Y |
| 21-225_190E11_LC | . | . | . | . | . | . | . | . | . | . | . | . | . | . | . | . | . | . | . | . | . | . | . | . | . | . |
| 21-225_191E8_LC | . | . | . | . | . | . | . | . | . | . | . | . | . | . | . | . | . | . | E | . | . | . | . | . | . | . |
| 21-225_191G11_LC | . | . | . | . | . | . | . | . | . | . | . | . | . | . | . | . | . | . | . | . | . | . | . | . | . | . |
| 21-225_199A6_LC | . | . | . | . | . | . | . | . | . | . | . | . | . | . | . | . | . | . | E | Q | . | . | . | . | . | . |
| 21-225_55E1_LC | . | . | . | . | . | . | . | . | . | . | . | . | . | . | . | . | . | . | . | . | . | . | . | . | P | . |
| 21-225_65C12_LC | N | A | . | . | . | . | . | . | . | . | . | . | . | S | . | . | . | . | . | . | . | . | N | . | . | . |
| 21-225_74D1_LC | . | . | . | . | . | . | . | . | . | . | . | . | . | . | . | Y | . | . | . | . | . | . | . | . | . | . |
| 21-225_75F11_LC | . | . | . | . | . | . | . | . | . | . | . | . | . | . | . | Y | . | . | . | Y | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | N | N | - | - | - | - | - | - | - | - | - | - | - | - | - | - | - | - | - | - | - |
| 21-225_190E11_LC | . | | | | | | | . | . | . | . | . | . | . | . | . | . | . | . | . | . | . | . | . | . | . |
| 21-225_191E8_LC | . | | | . | . | | . | . | . | . | . | . | . | . | . | . | . | . | . | . | . | . | . | . | . | . |
| 21-225_191G11_LC | . | | | . | . | Y | . | . | . | . | . | . | . | . | . | . | . | . | . | . | . | . | . | . | . | . |
| 21-225_199A6_LC | . | | | . | . | | . | . | . | . | . | . | . | . | . | . | . | . | . | . | . | . | . | . | . | . |
| 21-225_55E1_LC | . | | | . | Y | . | . | . | . | . | . | . | . | . | . | . | . | . | . | . | . | . | . | . | . | . |
| 21-225_65C12_LC | . | | | . | . | T | . | . | . | . | . | . | . | . | . | . | . | . | . | . | . | . | . | . | . | . |
| 21-225_74D1_LC | . | | | . | . | | . | . | . | . | . | . | . | . | . | . | . | . | . | . | . | . | . | . | . | . |
| 21-225_75F11_LC | . | | | . | . | | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | K_FR4 | | | | | | |
| CONSENSUS | - | - | - | - | W | P | L | T | F | G | G | G | T | K | V | E | I | K | R |
| 21-225_190E11_LC | . | . | . | . | . | . | . | . | . | . | . | . | . | . | . | . | . | . | . |
| 21-225_191E8_LC | . | . | . | . | . | . | . | . | . | . | . | i | . | . | . | . | . | . | . |
| 21-225_191G11_LC | . | . | . | . | . | . | . | . | . | . | . | . | . | . | . | . | . | . | . |
| 21-225_199A6_LC | . | . | . | . | . | . | . | . | . | . | . | . | . | . | . | . | . | . | . |
| 21-225_55E1_LC | . | . | . | . | . | . | . | . | . | . | . | . | . | . | . | . | . | . | . |
| 21-225_65C12_LC | . | . | . | . | . | . | . | . | . | . | . | . | . | . | . | . | . | . | . |
| 21-225_74D1_LC | . | . | . | W | P | . | . | . | . | . | . | . | . | . | . | . | . | . | . |
| 21-225_75F11_LC | . | . | . | W | P | . | . | . | . | . | . | . | . | . | . | . | . | . | . |

**FIGURE 57**

Table 134.    Consensus 54 - VL3|3j/JL2 (SEQ ID NO: 50351):

SYELTQP-LSVSVALGQTARITCGGN---NIGR-----KNVIIWYQQKPGQAPVLVIYR-------DSDRPSGIPERFSGSNSG--NTATLTISRAQAGDEADYYCQVWDS---------------------STVVFGGGTKLTVLG wherein:

N at position 26 can be substituted with D

G at position 32 can be substituted with R

R at position 33 can be substituted with S

K at position 39 can be substituted with R

N at position 40 can be substituted with A

R at position 58 can be substituted with S

S at position 68 can be substituted with R

D at position 69 can be substituted with N or Y

P at position 71 can be substituted with S

V at position 108 can be substituted with D

null (-) at position 112 can be substituted with S

null (-) at position 134 can be substituted with S

S at position 135 can be substituted with D or G

T at position 136 can be substituted with H

V at position 137 can be substituted with A or G

Gly Gly Xaa Asn Ile Xaa Xaa Xaa Xaa Val His (SEQ ID NO: 50250)

Xaa Asp Xaa Xaa Arg Xaa Ser (SEQ ID NO: 50251)

**FIGURE 57**

Gln Xaa Trp Asp Ser Xaa Xaa Xaa Xaa Xaa Val (SEQ ID NO: 50252)

Gly Gly Asn Asn Ile Gly Arg Lys Asn Val His (SEQ ID NO: 50723)

Arg Asp Ser Asp Arg Pro Ser (SEQ ID NO: 50724)

Gln Val Trp Asp Ser Ser Thr Val Val (SEQ ID NO: 50725)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | L_FR1 | | | | | | | | | | | | | |
| CONSENSUS | S | Y | E | L | T | Q | P | - | L | S | V | S | V | A | L | G | Q | T | A | R | I | T | C | G | G | N |
| 21-225_203B2_LC | . | . | . | . | . | . | . | . | . | . | . | . | . | . | . | . | . | . | . | . | . | . | . | . | . | . |
| 21-225_205E12_LC | . | . | . | . | . | . | . | . | . | . | . | . | A | . | . | . | . | . | . | . | . | . | . | . | . | . |
| 21-225_206B5_LC | . | . | . | . | . | . | . | . | . | . | . | . | . | . | . | . | . | . | . | . | . | . | A | . | . | . |
| 21-225_209H10_LC | . | . | . | . | . | . | . | . | . | . | . | . | . | . | . | . | . | . | . | . | A | . | . | . | . | . |
| 21-225_211H2_LC | . | . | . | . | . | . | . | . | . | . | . | . | . | . | . | . | . | . | . | . | . | . | . | . | . | . |
| 21-225_216B12_LC | . | . | . | . | . | . | . | . | . | . | . | . | . | . | . | . | . | . | . | . | . | . | . | . | . | D |
| 21-225_3E10_LC | . | . | . | . | . | . | . | . | M | . | . | . | . | T | A | . | M | . | . | . | . | . | . | . | . | . |
| 21-225_8C12_LC | . | . | . | . | . | . | . | . | M | . | . | . | . | T | A | . | M | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | L_CDR1 | | | | | | | | | | | | | | | | | | L_FR2 | | | |
| CONSENSUS | - | - | - | N | I | G | R | - | - | - | - | - | K | N | V | H | W | Y | Q | Q | K | P | G | Q | A | P |
| 21-225_203B2_LC | . | . | . | . | . | . | . | . | . | . | . | . | . | . | . | . | . | . | . | . | . | . | . | . | S | . |
| 21-225_205E12_LC | . | . | . | . | . | . | . | . | . | . | . | . | . | . | . | . | . | . | . | . | . | . | . | . | S | . |
| 21-225_206B5_LC | . | . | . | . | . | . | . | . | . | . | . | . | . | . | . | . | . | . | . | . | . | . | L | . | . | . |
| 21-225_209H10_LC | . | . | . | . | . | . | . | . | . | . | . | . | . | . | . | . | . | . | . | . | . | . | L | . | . | . |
| 21-225_211H2_LC | . | . | . | . | . | . | . | . | . | . | . | . | S | . | . | . | . | . | . | . | . | . | . | . | . | . |
| 21-225_216B12_LC | . | . | . | S | . | . | . | . | . | . | . | . | S | . | . | . | . | . | . | . | . | . | . | . | . | . |
| 21-225_3E10_LC | . | . | . | . | S | . | . | . | . | . | . | . | A | . | . | . | . | . | . | . | . | . | . | . | D | . |
| 21-225_8C12_LC | . | . | . | . | S | . | . | . | . | . | . | . | A | . | . | . | . | . | . | . | . | . | . | . | D | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | L_CDR2 | | | | | | | | | | | | | | |
| CONSENSUS | V | L | V | I | Y | R | - | - | - | - | - | - | - | - | - | D | S | R | P | S | G | I | P | E | R | F |
| 21-225_203B2_LC | . | . | i | . | X | . | . | . | . | . | . | . | . | . | . | . | D | . | . | . | . | . | . | . | . | . |
| 21-225_205E12_LC | . | . | i | . | X | . | . | . | . | . | . | . | . | . | . | . | D | . | . | . | . | . | . | . | . | . |
| 21-225_206B5_LC | . | P | . | . | X | . | . | . | . | . | . | . | . | . | . | . | Y | . | S | . | . | . | . | D | . | . |
| 21-225_209H10_LC | . | P | . | . | L | . | . | . | . | . | . | . | . | . | . | . | Y | . | S | . | . | . | . | D | . | . |
| 21-225_211H2_LC | i | . | . | . | . | . | . | . | . | . | . | . | . | . | . | X | D | . | . | . | . | . | . | . | . | . |
| 21-225_216B12_LC | . | . | . | . | . | . | . | . | . | . | . | . | . | . | . | . | N | . | . | . | . | . | . | . | . | . |
| 21-225_3E10_LC | . | . | . | . | . | S | . | . | . | . | . | . | . | . | . | . | N | . | . | . | . | . | . | . | . | . |
| 21-225_8C12_LC | . | . | . | . | . | S | . | . | . | . | . | . | . | . | . | . | N | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | L_FR3 | | | | | | | | | | | | | | | | | |
| CONSENSUS | S | G | S | N | S | G | - | - | N | T | A | T | L | T | I | S | R | A | Q | A | G | D | E | A | D | Y |
| 21-225_203B2_LC | . | . | . | . | . | . | . | . | . | . | . | . | . | . | . | . | . | . | . | . | . | . | . | . | . | . |
| 21-225_205E12_LC | . | . | . | . | . | . | . | . | . | . | . | . | . | . | . | . | . | . | . | . | . | . | . | . | . | . |
| 21-225_206B5_LC | . | . | . | . | C | . | . | . | . | T | . | . | V | . | . | . | . | . | . | . | . | . | E | . | E | . |
| 21-225_209H10_LC | . | . | . | . | W | . | . | . | . | . | . | . | V | . | . | . | . | . | . | . | . | . | E | . | E | . |
| 21-225_211H2_LC | . | . | . | . | . | . | . | . | . | . | . | . | . | . | . | . | . | . | . | . | . | . | . | . | . | . |
| 21-225_216B12_LC | . | . | . | . | . | . | . | . | . | . | . | . | . | . | . | . | . | . | . | . | . | . | . | . | . | . |
| 21-225_3E10_LC | . | . | . | . | P | . | . | . | . | . | . | . | . | . | . | . | . | i | E | . | . | . | . | . | . | . |
| 21-225_8C12_LC | . | . | . | . | P | . | . | . | . | . | . | . | . | . | . | . | . | i | E | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | L_CDR3 | | | | | | | |
| CONSENSUS | Y | C | Q | V | W | D | S | - | - | - | - | - | - | - | - | - | - | - | - | - | - | - | - | - | - | - |
| 21-225_203B2_LC | . | . | . | . | . | . | . | . | . | . | . | . | . | . | . | . | . | . | . | . | . | . | . | . | . | . |
| 21-225_205E12_LC | . | . | . | . | . | . | . | . | . | . | . | . | . | . | . | . | . | . | . | . | . | . | . | . | . | . |
| 21-225_206B5_LC | . | . | . | G | . | . | . | . | . | . | . | . | . | . | . | . | . | . | . | . | . | . | . | . | . | . |
| 21-225_209H10_LC | . | . | . | G | . | . | . | . | . | . | . | . | . | . | . | . | . | . | . | . | . | . | . | . | . | . |
| 21-225_211H2_LC | F | . | . | . | . | . | . | . | . | . | . | . | . | . | . | . | . | . | . | . | . | . | . | . | . | . |
| 21-225_216B12_LC | . | . | . | . | . | . | . | . | . | . | . | . | . | . | . | . | . | . | . | . | . | . | . | . | . | . |
| 21-225_3E10_LC | . | . | . | . | . | . | S | . | . | . | . | . | . | . | . | . | . | . | . | . | . | . | . | . | . | . |
| 21-225_8C12_LC | . | . | . | . | . | . | S | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | L_FR4 | | | | | | |
| CONSENSUS | - | - | - | - | S | T | V | V | F | G | G | G | T | K | L | T | V | L | G |
| 21-225_203B2_LC | . | . | . | . | G | . | A | . | . | . | . | . | . | . | . | P | . | . | . |
| 21-225_205E12_LC | . | . | . | . | G | . | A | . | . | . | . | . | . | . | . | P | . | . | . |
| 21-225_206B5_LC | . | . | . | . | . | . | . | . | . | . | . | . | . | . | . | . | . | . | . |
| 21-225_209H10_LC | . | . | . | . | . | . | . | . | . | . | . | . | . | . | . | . | . | . | . |
| 21-225_211H2_LC | . | . | . | . | . | . | A | . | . | . | . | . | . | . | . | . | . | . | . |
| 21-225_216B12_LC | . | . | . | . | . | . | G | . | . | . | . | . | . | . | . | . | . | . | . |
| 21-225_3E10_LC | . | . | . | S | G | X | . | . | . | . | . | . | . | . | . | . | . | . | . |
| 21-225_8C12_LC | . | . | . | S | G | X | . | . | . | . | . | . | . | . | . | . | . | . | . |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62319740
- US 62259553
- US 62234546
- US 8030457 B, Jackson
- EP 404097 A
- WO 9311161 A
- WO 0024782 A
- US 20030133939 A
- US 6846634 B
- US 6696245 B
- US 050202512
- US 040202995
- US 040038291
- US 040009507
- US 030039958
- US 6703199 B
- US 20050238646 A
- US 7695963 B
- WO 09089004 A
- US 6054297 A
- US 5886152 A
- US 5877293 A
- US 5869619 A
- US 5225539 A
- US 5821337 A
- US 5859205 A
- US 6881557 B
- US 5814318 A
- US 5569825 A
- US 5545806 A
- US 5877397 A
- US 050118643 B
- WO 05694879 A
- WO 9824838 A
- WO 0076310 A
- US 7064244 B
- US 4464456 A
- WO 9202551 A
- US 5627052 A
- US 5223409 A
- US 5698426 A
- US 8507748 B
- US 8502014 B
- US 20090285805 A1
- US 20090169548 A1
- US 20090307787 A1
- US 20110314563 A1
- US 20120151610 A1
- WO 2008122886 A2
- WO 2009013620 A2
- US 4946778 A
- US 4331647 A
- US 4518584 A
- US 4737462 A
- US 5011912 A
- US 20090028856 A
- US 20090274713 A
- US 20070031402 A
- WO 2005084390 A
- WO 2009099728 A
- US 5208020 A
- US 5416064 A
- US 5475092 A
- US 5585499 A
- US 6436931 B
- US 6372738 B
- US 6340701 B
- WO 9310151 A
- US 5457035 A
- US 4751180 A
- US 4935233 A
- WO 9410308 A
- US 4640835 A
- US 4496689 A
- US 4301144 A
- US 4670417 A
- US 4791192 A
- US 4179337 A
- US 6133426 A
- US 6210924 B
- WO 9321232 A
- EP 439095 A
- US 5474981 A
- US 5624821 A
- US 6602684 B
- US 7029872 B
- US 20060067930 A1
- US 20050272128 A1
- US 20050079605 A1
- US 20050123546 A1
- US 20040072290 A1
- US 20060257399 A1
- US 20040261148 A1
- US 20070092521 A1
- US 20060040325 A1
- US 20060039904 A1
- WO 04029207 A
- WO 03011878 A

- WO 05044859 A
- WO 06071856 A
- WO 06071280 A
- US 20040185045 A
- WO 2004016750 A
- WO 9958572 A, Armour
- WO 9951642 A, Idusogie
- US 6395272 B, Deo
- US 20010036459 A
- WO 0179299 A, Ravetch
- WO 2004063351 A, Stavenhagen
- WO 9823289 A
- WO 9734631 A
- US 6277375 B
- WO 8705330 A
- US 6506559 B
- US 8394628 B
- US 7056704 B
- US 7078196 B
- US 6107094 A
- US 5898031 A
- US 6573099 B
- EP 1144623 A
- US 20150259689
- US 20150197746
- US 20110092565

- US 8877917 B
- US 8507455 B
- US 7579451 B
- US 5539082 A
- US 5714331 A
- US 5719262 A
- US 6693187 B
- US 9181551 B
- US 20160122761 A
- US 6194389 B
- US 6168587 B
- US 6472375 B
- US 6471996 B
- US 4522811 A
- US 4737456 A
- US 3720760 A, Bennich
- NL 5156
- NL 15
- NL 5537
- US 7192933 B
- US 6114598 A
- US 6162963 A
- US 6833268 B
- US 7049426 B
- US 2012028596 W, Kannan, G.

**Non-patent literature cited in the description**

- **SAMBROOK et al.** Molecular Cloning: A Laboratory Manual. Cold Spring Harbor Laboratory Press, 1989
- **AUSUBEL et al.** Current Protocols in Molecular Biology. Greene Publishing Associates, 1992
- **HARLOW ; LANE.** Antibodies: A Laboratory Manual. Cold Spring Harbor Laboratory Press, 1990
- **GOEDDEL.** Gene Expression Technology: Methods in Enzymology. Academic Press, 1990, 185
- **BARON et al.** *Nucleic Acids Res.,* 1995, vol. 23, 3605-06
- **GUDBJARTSSON et al.** *Nature Genetics,* May 2015, vol. 47 (5), 435-444
- **SABRAUTZKI et al.** *Mamm. Genome,* 2012, vol. 23, 416-430
- **GREWAL.** Methods in Enzymology. 2010, vol. 479, 223-241
- **ROGGENBUCK et al.** *Autoimmune Highlights,* 2012, vol. 3, 119-125
- **LIU J ; HU B ; YANG Y et al.** A new splice variant of the major subunit of human asialoglycoprotein receptor encodes a secreted form in hepatocytes. *PloS one,* 2010, vol. 5, e12934
- **WEI et al.** *Int J Nanomedicine,* 2015, vol. 10, 5123-37

- **D'SOUZA et al.** *J Control Release,* 2015, vol. 203, 126-39
- **RAJEEV et al.** *Chembiochem,* 2015, vol. 16 (6), 903-8
- **WEIGEL PH ; YIK JH.** Glycans as endocytosis signals: the cases of the asialoglycoprotein and hyaluronan/chondroitin sulfate receptors. *Biochimica et biophysica acta,* 2002, vol. 1572, 341-63
- **TOZAWA R ; ISHIBASHI S ; OSUGA J et al.** Asialoglycoprotein receptor deficiency in mice lacking the major receptor subunit. Its obligate requirement for the stable expression of oligomeric receptor. *The Journal of Biological Chemistry,* 2001, vol. 276, 12624-8
- **TUIN A ; HUIZINGA-VAN DER VLAG A ; VAN LOENEN-WEEMAES AM ; MEIJER DK ; POELSTRA K.** On the role and fate of LPS-dephosphorylating activity in the rat liver. *American Journal of Physiology Gastrointestinal and Liver Physiology,* 2006, vol. 290, G377-85
- **FURGER E ; FEDOSOV SN ; LILDBALLE DL et al.** Comparison of recombinant human haptocorrin expressed in human embryonic kidney cells and native haptocorrin. *PloS one,* 2012, vol. 7, e37421

- **BURGER RL ; SCHNEIDER RJ ; MEHLMAN CS ; ALLEN RH.** Human plasma R-type vitamin B12-binding proteins. II. The role of transcobalamin I, transcobalamin III, and the normal granulocyte vitamin B12-binding protein in the plasma transport of vitamin B12. *The Journal of Biological Chemistry,* 1975, vol. 250, 7707-13
- **STEIRER LM ; PARK EI ; TOWNSEND RR ; BAENZIGER JU.** The asialoglycoprotein receptor regulates levels of plasma glycoproteins terminating with sialic acid alpha2,6-galactose. *The Journal of Biological Chemistry,* 2009, vol. 284, 3777-83
- **YANG A ; GYULAY G ; MITCHELL M ; WHITE E ; TRIGATTI BL ; IGDOURA SA.** Hypomorphic sialidase expression decreases serum cholesterol by downregulation of VLDL production in mice. *Journal of Lipid Research,* 2012, vol. 53, 2573-2585
- **CORTEZ-RETAMOZO et al.** *Cancer Research,* 2004, vol. 64, 2853-57
- **KORNDORFER et al.** *Proteins: Structure, Function, and Bioinformatics,* 2003, vol. 53 (1), 121-129
- **ROQUE et al.** *Biotechnol. Prog.,* 2004, vol. 20, 639-654
- **HOLLINGER et al.** *Proc. Natl. Acad. Sci. USA,* 1993, vol. 90, 6444-6448
- **WARD et al.** *Nature,* 1989, vol. 341, 544-546
- **FREDERICKS et al.** *Protein Engineering, Design & Selection,* 2004, vol. 17, 95-106
- **POWERS et al.** *Journal of Immunological Methods,* 2001, vol. 251, 123-135
- **OLAFSEN et al.** *Protein Eng Des Sel.,* 2004, vol. 17, 315-23
- **ZAPATA et al.** *Protein Eng.,* 1995, vol. 8, 1057-1062
- Fundamental Immunology. Raven Press, 1989
- **HOLLINGER ; HUDSON.** *Nature Biotechnology,* 2005, vol. 23 (9), 1126-1136
- **BIRD et al.** *Science,* 1988, vol. 242, 423-26
- **HUSTON et al.** *Proc. Natl. Acad. Sci. USA,* 1988, vol. 85, 5879-83
- **HOLLIGER et al.** *Proc. Natl. Acad. Sci. USA,* 1993, vol. 90, 6444-48
- **POLJAK et al.** *Structure,* 1994, vol. 2, 1121-23
- **KABAT.** Sequences of Proteins of Immunological Interest. NIH, 1987
- **CHOTHIA ; LESK.** *J. Mol. Biol.,* 1987, vol. 196, 901-917
- **CHOTHIA et al.** *Nature,* 1989, vol. 342, 878-883
- **LEFRANC et al.** *Dev. Comp. Immunol.,* 2005, vol. 29, 185-203
- **HONEGGER ; PLUCKTHUN.** *J. Mol. Biol.,* 2001, vol. 309 (3), 657-670
- **NYGREN ; UHLEN.** *Current Opinion in Structural Biology,* 1997, vol. 7, 463-469
- **SONGSIVILAI ; LACHMANN.** *Clin. Exp. Immunol.,* 1990, vol. 79, 315-321
- **KOSTELNY et al.** *J. Immunol.,* 1992, vol. 148, 1547-1553
- **STAHLI et al.** *Methods in Enzymology,* 1983, vol. 9, 242-253
- **KIRKLAND et al.** *J. Immunol.,* 1986, vol. 137, 3614-3619
- **HARLOW ; LANE.** Antibodies, A Laboratory Manual. Cold Spring Harbor Press, 1988
- **MOREL et al.** *Molec. Immunol.,* 1988, vol. 25, 7-15
- **CHEUNG et al.** *Virology,* 1990, vol. 176, 546-552
- **MOLDENHAUER et al.** *Scand. J. Immunol.,* 1990, vol. 32, 7-82
- **THOMPSON et al.** *Nucleic Acids Res.,* 1994, vol. 22, 4673-4680
- **SAITOU ; NEI.** *Molecular Biology and Evolution,* 1987, vol. 4, 406-425
- **M. MEIER et al.** *JMB,* 2000, vol. 300, 857-865
- **MILLER et al.** Epitope binning of murine monoclonal antibodies by a multiplexed pairing assay. *J Immunol Methods,* 2011, vol. 365, 118-25
- **ALBERT et al.** The B-cell Epitope of the Monoclonal Anti-Factor VIII Antibody ESH8 Characterized by Peptide Array Analysis. *Thromb Haemost,* 2008, vol. 99, 634-7
- **SONG et al.** Epitope Mapping of Ibalizumab, a Humanized Anti-CD4 Monoclonal Antibody with Anti-HIV-1 Activity in Infected Patients. *J. Virol.,* 2010, vol. 84, 6935-6942
- **CUNNINGHAM ; WELLS.** High-resolution epitope mapping of HGH-receptor interactions by alanine-scanning mutagenesis. *Science,* 1989, vol. 244, 1081-1085
- **LIM et al.** A diversity of antibody epitopes can induce signaling through the erythropoietin receptor. *Biochemistry,* 2010, vol. 49, 3797-3804
- **COATES et al.** Epitope mapping by amide hydrogen/deuterium exchange coupled with immobilization of antibody, on-line proteolysis, liquid chromatography and mass spectrometry. *Rapid Commun. Mass Spectrom.,* 2009, vol. 23, 639-647
- **MORGAN et al.** Precise epitope mapping of malaria parasite inhibitory antibodies by TROSY NMR cross-saturation. *Biochemistry,* 2005, vol. 44, 518-23
- **GERHARDT et al.** Structure of IL-17A in complex with a potent, fully human neutralizing antibody. *J. Mol. Biol,* 2009, vol. 394, 905-21
- **JIA et al.** A novel method of Multiplexed Competitive Antibody Binning for the characterization of monoclonal antibodies. *J. Immunological Methods,* 2004, vol. 288, 91-98
- **NANEVICZ, T. et al.** *J. Biol. Chem.,* 1995, vol. 270 (37), 21619-21625

- **ZUPNICK, A. et al.** *J. Biol. Chem.,* 2006, vol. 281 (29), 20464-20473
- **RATHANASWAMI et al.** *Analytical Biochemistry,* 2008, vol. 373, 52-60
- **MOULT. J., Curr. Op. in Biotech.,** 1996, vol. 7 (4), 422-427
- **CHOU et al.** *Biochemistry,* 1974, vol. 13 (2), 222-245
- **CHOU et al.** *Biochemistry,* 1974, vol. 113 (2), 211-222
- **CHOU et al.** *Adv. Enzymol. Relat. Areas Mol. Biol.,* 1978, vol. 47, 45-148
- **CHOU et al.** *Ann. Rev. Biochem.,* vol. 47, 251-276
- **CHOU et al.** *Biophys. J.,* 1979, vol. 26, 367-384
- **HOLM et al.** *Nucl. Acid. Res.,* 1999, vol. 27 (1), 244-247
- **JONES, D.** *Curr. Opin. Struct. Biol.,* 1997, vol. 7 (3), 377-87
- **SIPPL et al.** *Structure,* 1996, vol. 4 (1), 15-19
- **BOWIE et al.** *Science,* 1991, vol. 253, 164-170
- **GRIBSKOV et al.** *Meth. Enzym,* 1990, vol. 183, 146-159
- **GRIBSKOV et al.** *Proc. Nat. Acad. Sci.,* 1987, vol. 84 (13), 4355-4358
- Proteins, Structures and Molecular Principles. W. H. Freeman and Company, 1984
- Introduction to Protein Structure. Garland Publishing, 1991
- **THORNTON et al.** *Nature,* 1991, vol. 354, 105
- **LANITTO et al.** *Methods Mol. Biol.,* 2002, vol. 178, 303-16
- **BLOOM et al.** *Protein Science,* 1997, vol. 6, 407
- **HARLOW ; LANE.** Antibodies. Cold Spring Harbor Laboratory Press, 1988
- **2014.** ANTIBODIES. Cold Spring Harbor Laboratory Press
- **RIECHMANN et al.** *Nature,* 1988, vol. 332, 323
- **LIU et al.** *Proc. Nat. Acad. Sci. USA,* 1987, vol. 84, 3439
- **LARRICK et al.** *Bio/Technology,* 1989, vol. 7, 934
- **WINTER et al.** *TIPS,* 1993, vol. 14, 139
- **PADLAN et al.** *FASEB J.,* 1995, vol. 9, 133-39
- **TAMURA et al.** *J. Immunol.,* 2000, vol. 164, 1432-41
- **ZHANG, W. et al.** *Molecular Immunology.,* 2005, vol. 42 (12), 1445-1451
- **HWANG W. et al.** *Methods,* 2005, vol. 36 (1), 35-42
- **DALL'ACQUA WF et al.** *Methods,* 2005, vol. 36 (1), 43-60
- **CLARK, M.** *Immunology Today.,* 2000, vol. 21 (8), 397-402
- **BRUGGEMANN et al.** *Curr. Opin. Biotechnol.,* 1997, vol. 8, 455-58
- Production of human antibodies from transgenic mice. **DAVIS et al.** Antibody Engineering: Methods and Protocols. Humana Press, 2003, 191-200
- **KELLERMANN et al.** *Curr Opin Biotechnol.,* 2002, vol. 13, 593-97
- **RUSSEL et al.** *Infect Immun.,* 2000, vol. 68, 1820-26
- **GALLO et al.** *Eur J Immun.,* 2000, vol. 30, 534-40
- **DAVIS et al.** *Cancer Metastasis,* 1999, vol. 18, 421-25
- **GREEN.** *J Immunol Methods.,* 1999, vol. 231, 11-23
- **JAKOBOVITS.** *Advanced Drug Delivery Reviews,* 1998, vol. 31, 33-42
- **GREEN et al.** *J Exp Med.,* 1998, vol. 188, 483-95
- **JAKOBOVITS A.** *Exp. Opin. Invest. Drugs.,* 1998, vol. 7, 607-14
- **TSUDA et al.** *Genomics,* 1997, vol. 42, 413-21
- **MENDEZ et al.** *Nat Genet.,* 1997, vol. 15, 146-56
- **JAKOBOVITS.** *Curr Biol.,* 1994, vol. 4, 761-63
- **ARBONES et al.** *Immunity,* 1994, vol. 1, 247-60
- **GREEN et al.** *Nat Genet.,* 1994, vol. 7, 13-21
- **JAKOBOVITS et al.** *Nature,* 1993, vol. 362, 255-58
- **JAKOBOVITS et al.** *Proc Natl Acad Sci U S A.,* 1993, vol. 90, 2551-55
- **CHEN, J. ; M. TROUNSTINE ; F. W. ALT ; F. YOUNG ; C. KURAHARA ; J. LORING ; D. HUSZAR.** Immunoglobulin gene rearrangement in B-cell deficient mice generated by targeted deletion of the JH locus. *International Immunology,* 1993, vol. 5, 647-656
- **CHOI et al.** *Nature Genetics,* 1993, vol. 4, 117-23
- **FISHWILD et al.** *Nature Biotechnology,* 1996, vol. 14, 845-51
- **HARDING et al.** *Annals of the New York Academy of Sciences,* 1995
- **LONBERG et al.** *Nature,* 1994, vol. 368, 856-59
- Transgenic Approaches to Human Monoclonal Antibodies. **LONBERG.** Handbook of Experimental Pharmacology. 1994, vol. 113, 49-101
- **LONBERG et al.** *Internal Review of Immunology,* 1995, vol. 13, 65-93
- **NEUBERGER.** *Nature Biotechnology,* 1996, vol. 14, 826
- **TAYLOR et al.** *Nucleic Acids Research,* 1992, vol. 20, 6287-95
- **TAYLOR et al.** *International Immunology,* 1994, vol. 6, 579-91

- **TOMIZUKA et al.** *Nature Genetics,* 1997, vol. 16, 133-43
- **TOMIZUKA et al.** *Proceedings of the National Academy of Sciences USA,* 2000, vol. 97, 722-27
- **TUAILLON et al.** *Proceedings of the National Academy of Sciences USA,* 1993, vol. 90, 3720-24
- **TUAILLON et al.** *Journal of Immunology,* 1994, vol. 152, 2912-20
- **LONBERG et al.** *Nature,* 1994, vol. 368, 856
- **TAYLOR et al.** *Int. Immun.,* 1994, vol. 6, 579
- **JAKOBOVITS et al.** *Ann. N. Y. Acad. Sci.,* 1995, vol. 764, 525-35
- **GLASKY et al.** *Hybridoma,* 1989, vol. 8, 377-89
- **BOERNER et al.** *J. Immunol.,* 1991, vol. 147, 86-95
- **BABCOOK et al.** *Proc. Natl. Acad. Sci. USA,* 1996, vol. 93, 7843-48
- **WINTER et al.** *Annu. Rev. Immunol.,* 1994, vol. 12, 433-55
- **BURTON et al.** *Adv. Immunol.,* 1994, vol. 57, 191-280
- **HUSE et al.** *Science,* 1989, vol. 246, 1275-81
- **SASTRY et al.** *Proc. Natl. Acad. Sci. USA,* 1989, vol. 86, 5728-32
- **ALTING-MEES et al.** *Strategies in Molecular Biology,* 1990, vol. 3, 1-9
- **KANG et al.** *Proc. Natl. Acad. Sci. USA,* 1991, vol. 88, 4363-66
- **HOOGENBOOM et al.** *J. Molec. Biol.,* 1992, vol. 227, 381-388
- **SCHLEBUSCH et al.** *Hybridoma,* 1997, vol. 16, 47-52
- **BIRD et al.** *Science,* 1988, vol. 242, 423-426
- **HARRIS, R.J.** *Journal of Chromatography,* 1995, vol. 705, 129-134
- Purification of Immunoglobulin G (IgG). **BAINES et al.** Methods in Molecular Biology. The Humana Press, Inc, 1992, vol. 10, 79-104
- **SCHIER et al.** *J. Mol. Biol.,* 1996, vol. 263, 551
- Monoclonal Antibodies, Hybridomas: A New Dimension in Biological Analyses. Plenum Press, 1980
- Antibodies: A Laboratory Manual. Cold Spring Harbor Laboratory Press, 1988
- **YANG et al.** *J. Mol. Biol.,* 1995, vol. 254, 392-403
- **MARKS et al.** *Bio/Technology,* 1992, vol. 10, 779-783
- **LOW et al.** *J. Mol. Biol.,* 1996, vol. 250, 350-368
- **PATTEN et al.** *Curr. Opin. Biotechnol.,* 1997, vol. 8, 724-733
- **THOMPSON et al.** *J. Mol. Biol.,* 1996, vol. 256, 7-88
- **CRAMERI et al.** *Nature,* 1998, vol. 391, 288-291
- **VAUGHAN et al.** *Nature Biotechnology,* 1998, vol. 16, 535-539
- **KORTT et al.** *Prot. Eng.,* 1997, vol. 10, 423
- **KORTT et al.** *Biomol. Eng.,* 2001, vol. 18, 95-108
- **KRIANGKUM et al.** *Biomol. Eng.,* 2001, vol. 18, 31-40
- **BIRD.** *Science,* 1988, vol. 242, 423
- **HUSTON et al.** *Proc. Natl. Acad. Sci. USA,* 1988, vol. 85, 5879
- **WARD et al.** *Nature,* 1989, vol. 334, 544
- **DE GRAAF et al.** *Methods Mol Biol.,* 2002, vol. 178, 379-87
- **NISONOFF et al.** *Arch. Biochem. Biophys.,* 1960, vol. 89, 230
- **PORTER.** *Biochem. J.,* 1959, vol. 73, 119
- **EDELMAN et al.** Methods in Enzymology. Academic Press, 1967, vol. 1, 422
- **ANDREWS, S.M. ; TITUS, J.A. et al.** Current Protocols in Immunology. John Wiley & Sons, 2003
- **LARRICK et al.** *Methods: A Companion to Methods in Enzymology,* 1991, vol. 2, 106
- Genetic Manipulation of Monoclonal Antibodies. **COURTENAY-LUCK et al.** Monoclonal Antibodies: Production, Engineering and Clinical Application. Cambridge University Press, 1995, 166
- Genetic Manipulation and Expression of Antibodies. **WARD et al.** Monoclonal Antibodies: Principles and Applications. Wiley-Liss, Inc, 1995, 137
- **WALDER et al.** *Gene,* 1986, vol. 42, 133
- **BAUER et al.** *Gene,* 1985, vol. 37, 73
- **CRAIK.** *BioTechniques,* January 1985, 12-19
- **SMITH et al.** Genetic Engineering: Principles and Methods. Plenum Press, 1981
- **HOPP et al.** *Bio/Technology,* 1988, vol. 6, 1204
- **ASHKENAZI et al.** *PNAS USA,* 1991, vol. 88, 10535
- **BYRN et al.** *Nature,* 1990, vol. 344, 677
- **HOLLENBAUGH et al.** Construction of Immunoglobulin Fusion Proteins. *Current Protocols in Immunology,* 1992, (4), 10.19.1-10.19.11
- **BAUM et al.** *EMBO J.,* 1994, vol. 13, 3992-4001
- **LANDSCHULZ et al.** *Science,* 1988, vol. 240, 1759
- **HOPPE et al.** *FEES Letters,* 1994, vol. 344, 191
- **FANSLOW et al.** *Semin. Immunol.,* 1994, vol. 6, 267-78
- Current Protocols in Molecular Biology. John Wiley & Sons, 1989
- **SAMBROOK ; FRITSCH ; MANIATIS.** Molecular Cloning: A Laboratory Manual. Cold Spring Harbor Laboratory Press, 1989
- Current Protocols in Molecular Biology. John Wiley & Sons, Inc, 1995

- **VOSS et al.** *Trends Biochem. Sci.,* 1986, vol. 11, 287
- **MANIATIS et al.** *Science,* 1987, vol. 236, 1237
- **RASMUSSEN et al.** *Cytotechnology,* 1998, vol. 28, 31
- **URLAUB et al.** *Proc. Natl. Acad. Sci. USA,* 1980, vol. 77, 4216-20
- **GLUZMAN et al.** *Cell,* 1981, vol. 23, 175
- **MCMAHAN et al.** *EMBO J.,* 1991, vol. 10, 2821
- **POUWELS et al.** Cloning Vectors: A Laboratory Manual. Elsevier, 1985
- **FOECKING et al.** *Gene,* 1986, vol. 45, 101
- **COCKETT et al.** *Bio/Technology,* 1990, vol. 8, 2
- **WIGLER et al.** *Cell,* 1977, vol. 11, 223
- **SZYBALSKA ; SZYBALSKI.** *Proc. Natl. Acad. Sci. USA,* 1992, vol. 48, 202
- **LOWY et al.** *Cell,* 1980, vol. 22, 817
- **WIGLER et al.** *Proc. Natl. Acad. Sci. USA,* 1980, vol. 77, 357
- **O'HARE et al.** *Proc. Natl. Acad. Sci. USA,* 1981, vol. 78, 1527
- **MULLIGAN ; BERG.** *Proc. Natl. Acad. Sci. USA,* 1981, vol. 78, 2072
- **WU ; WU.** *Biotherapy,* 1991, vol. 3, 87-95
- **SANTERRE et al.** *Gene,* 1984, vol. 30, 147
- Current Protocols in Molecular Biology. John Wiley & Sons, 1993
- **KRIEGLER.** Gene Transfer and Expression, A Laboratory Manual. Stockton Press, 1990
- Current Protocols in Human Genetics. John Wiley & Sons, 1994
- **COLBERRE-GARAPIN et al.** *J. Mol. Biol.,* 1981, vol. 150, 1
- The use of vectors based on gene amplification for the expression of cloned genes in mammalian cells. **BEBBINGTON ; HENTSCHEL.** DNA Cloning. Academic Press, 1987, vol. 3
- **CROUSE et al.** *Mol. Cell. Biol.,* 1983, vol. 3, 257
- **PROUDFOOT.** *Nature,* 1986, vol. 322, 52
- **KOHLER.** *Proc. Natl. Acad. Sci. USA,* 1980, vol. 77, 2197
- **NARAMURA et al.** *Immunol. Lett.,* 1994, vol. 39, 91-99
- **GILLIES et al.** *Proc. Natl. Acad. Sci.,* 1992, vol. 89, 1428-1432
- **FELL et al.** *J. Immunol.,* 1991, vol. 146, 2446-2452
- **GENTZ et al.** *Proc. Natl. Acad. Sci. USA,* 1989, vol. 86, 821-824
- **WILSON et al.** *Cell,* 1984, vol. 37, 767
- **T. E. CREIGHTON.** Proteins: Structure and Molecular Properties. W.H. Freeman & Co, 1983, 79-86
- **APLIN ; WRISTON.** *CRC Crit. Rev. Biochem.,* 1981, 259-306
- COMPUTATIONAL MOLECULAR BIOLOGY. Oxford University Press, 1988
- BIOCOMPUTING: INFORMATICS AND GENOME PROJECTS. Academic Press, 1993
- COMPUTER ANALYSIS OF SEQUENCE DATA. Humana Press, 1994
- **VON HEINJE, G.** SEQUENCE ANALYSIS IN MOLECULAR BIOLOGY. Academic Press, 1987
- SEQUENCE ANALYSIS PRIMER. M Stockton Press, 1991
- **H. CARILLO ; D. LIPTON.** *SIAM J. Applied Math.,* 1988, vol. 48, 1073
- Guide to Huge Computers. Academic Press, 1994
- **J. DEVEREUX et al.** *Nucl. Acids Res.,* 1984, vol. 12 (1), 387
- **DELEAVEY ; DAMHA.** *Chemistry and Biology,* 2012, vol. 19, 937-954
- **HAMAJIMA et al.** *Clin. Immunol. Immunopathol.,* 1998, vol. 88 (2), 205-10
- **SUBBARAO et al.** *Biochemistry,* 1987, vol. 26, 2964-2972
- **VOGEL et al.** *J. Am. Chem. Soc.,* 1996, vol. 118, 1581-1586
- **TURK et al.** *Biochem. Biophys. Acta,* 2002, vol. 1559, 56-68
- **LAM et al.** *Nature,* 1991, vol. 354, 82-84
- **AOKI et al.** *Cancer Gene Therapy,* 2001, vol. 8, 783-787
- **HAUBNER et al.** *Jour. Nucl. Med.,* 2001, vol. 42, 326-336
- **SIMEONI et al.** *Nucl. Acids Res.,* 2003, vol. 31, 2717-2724
- **TRUFERT et al.** *Tetrahedron,* 1996, vol. 52, 3005
- Oligonucleotide Conjugates in Antisense Technology. **MANOHARAN.** Antisense Drug Technology. Marcel Dekker, Inc, 2001
- **R. LAROCK.** Comprehensive Organic Transformations. VCH Publishers, 1989
- **T. W. GREENE ; P. G. M. WUTS.** Protective Groups in Organic Synthesis. John Wiley and Sons, 1991
- **L. FIESER ; M. FIESER.** Fieser and Fieser's Reagents for Organic Synthesis. John Wiley and Sons, 1994
- Encyclopedia of Reagents for Organic Synthesis. John Wiley and Sons, 1995
- **TOZAWA et al.** *J Biol Chem,* 2001, vol. 276, 12624-12628
- **STOCKERT et al.** *Science,* 1977, vol. 197, 667-668
- **HARDONK MJ ; SCHOLTENS HB.** *Histochemistry,* 1980, vol. 69 (3), 289-97
- **SCHOLTENS HB ; MEIJER DK ; HARDONK MJ.** *Liver,* March 1982, vol. 2 (1), 14-21

- **WINDLER et al.** *Biochem J,* 1991, vol. 276, 79-87
- **ROTUNDO et al.** *Hepatology,* 1998, vol. 28, 475-485
- **STOCKERT et al.** *PNAS,* 1982, vol. 79, 6229-6231
- **FRIEDEWALD, W. T. ; LEVY, R. I. ; FREDRICKSON, D. S.** Estimation of the concentration of low-density lipoprotein cholesterol in plasma, without use of the preparative ultracentrifuge. *Clin. Chem.,* 1972, vol. 18, 499-502
- **GUDBJARTSSON et al.** Large-scale whole-genome sequencing of the Icelandic population. *Nature genetics,* 2015
- **SAMANI NJ et al.** Genomewide association analysis of coronary artery disease. *The New England journal of medicine,* 2007, vol. 357, 443-53
- **GRETARSDOTTIR S et al.** Genome-wide association study identifies a sequence variant within the DAB2IP gene conferring susceptibility to abdominal aortic aneurysm. *Nature genetics,* 2010, vol. 42, 692
- **OLSEN MH et al.** N-terminal pro-brain natriuretic peptide, but not high sensitivity C-reactive protein, improves cardiovascular risk prediction in the general population. *European heart journal,* 2007, vol. 28, 1374-81
- **JORGENSEN AB et al.** Loss-of-function mutations in APOC3 and risk of ischemic vascular disease. *The New England journal of medicine,* 2014, vol. 371, 32-41
- **HOOGENDOORN EH et al.** Thyroid function and prevalence of anti-thyroperoxidase antibodies in a population with borderline sufficient iodine intake: influences of age and sex. *Clinical chemistry,* 2006, vol. 52, 104-11
- **GUDBARTSSOON, DF et al.** Large Scale whole-genome sequencing of the Icelandic population. *Nature Genetics,* 2015
- **DI ANGELANTONIO E et al.** Major lipids, apolipoproteins, and risk of vascular disease. *Jama,* 2009, vol. 302, 1993-2000
- **GUDBJARTSSON DF et al.** Large-scale whole-genome sequencing of the Icelandic population. *Nature genetics,* 2015
- **STEINTHORSDOTTIR V et al.** Identification of low-frequency and rare sequence variants associated with elevated or reduced risk of type 2 diabetes. *Nature genetics,* 2014, vol. 46, 294-8
- **WILLER CJ et al.** METAL: fast and efficient meta-analysis of genomewide association scans. *Bioinformatics,* 2010, vol. 26, 2190-1
- **GRETARSDOTTIR S et al.** The gene encoding phosphodiesterase 4D confers risk of ischemic stroke. *Nature genetics,* 2003, vol. 35, 131-8
- **MORELL AG ; GREGORIADIS G ; SCHEINBERG IH ; HICKMAN J ; ASHWELL G.** The role of sialic acid in determining the survival of glycoproteins in the circulation. *The Journal of biological chemistry,* 1971, vol. 246, 1461-7
- **VAN DEN HAMER CJ ; MORELL AG ; SCHEINBERG IH ; HICKMAN J ; ASHWELL G.** Physical and chemical studies on ceruloplasmin. IX. The role of galactosyl residues in the clearance of ceruloplasmin from the circulation. *The Journal of biological chemistry,* 1970, vol. 245, 4397-402
- **ASHWELL G ; HARFORD J.** Carbohydrate-specific receptors of the liver. *Annual review of biochemistry,* 1982, vol. 51, 531-54
- **WEIGEL PH.** Galactosyl and N-acetylgalactosaminyl homeostasis: a function for mammalian asialoglycoprotein receptors. *BioEssays: news and reviews in molecular, cellular and developmental biology,* 1994, vol. 16, 519-24
- **HADDAD L ; DAY IN ; HUNT S ; WILLIAMS RR ; HUMPHRIES SE ; HOPKINS PN.** Evidence for a third genetic locus causing familial hypercholesterolemia. A non-LDLR, non-APOB kindred. *Journal of lipid research,* 1999, vol. 40, 1113-22
- **TIMMS KM ; WAGNER S ; SAMUELS ME et al.** A mutation in PCSK9 causing autosomal-dominant hypercholesterolemia in a Utah pedigree. *Human genetics,* 2004, vol. 114, 349-53
- **VARRET M ; RABES JP ; SAINT-JORE B et al.** A third major locus for autosomal dominant hypercholesterolemia maps to 1p34.1-p32. *American journal of human genetics,* 1999, vol. 64, 1378-87
- **HUNT SC ; HOPKINS PN ; BULKA K et al.** Genetic localization to chromosome 1p32 of the third locus for familial hypercholesterolemia in a Utah kindred. *Arterioscler Thromb Vase Biol,* 2000, vol. 20, 1089-93
- **DO R ; WILLER CJ ; SCHMIDT EM et al.** Common variants associated with plasma triglycerides and risk for coronary artery disease. *Nature genetics,* 2013, vol. 45, 1345-52
- **CHAMBERS JC ; ZHANG W ; SEHMI J et al.** Genome-wide association study identifies loci influencing concentrations of liver enzymes in plasma. *Nature genetics,* 2011, vol. 43, 1131-8
- **WILLER CJ ; SCHMIDT EM ; SENGUPTA S et al.** Discovery and refinement of loci associated with lipid levels. *Nature genetics,* 2013, vol. 45, 1274-83
- **HEIGADOTTIR A ; THORLEIFSSON G ; MANOLESCU A et al.** A common variant on chromosom e 9p21 affects the risk of myocardial infarction. *Science (New York, NY),* 2007, vol. 316, 1 491-3
- **HEIGADOTTIR A ; MANOLESCU A ; THORLEIFSSON G et al.** The gene encoding 5-lipoxygenas e activating protein confers risk of myocardial infarction and stroke. *Nature genetics,* 2004, vol. 36, 233-9

- Genome-wide association study of 14,000 cases of seven common diseases and 3,000 shared controls. *Nature,* 2007, vol. 447, 6 61-78
- **SAMANI NJ ; ERDMANN J ; HALL AS et al.** Genom-ewid e association analysis of coronary artery disease. *The New England journal of medicine,* 2007, vol. 357, 4 43-53
- **GRETARSDOTTI R S ; BAAS AF ; THORLEIFS-SON G et al.** Genome-wide association study identifies a sequence variant within the DAB2IP gene conferring susceptibilit y to abdominal aortic aneurysm. *Nature genetics,* 2010, vol. 42, 69 2-7
- **OLSEN MH ; HANSEN TW ; CHRISTENSEN MK et al.** N-terminal pro-brain natriuretic peptide, but not high sensitivity C-reactive protein, improves cardiovascu lar risk prediction in the general population. *European heart journal,* 2007, vol. 28, 13 74-81
- **JORG ENSEN AB ; FRIKKE-SCHMIDT R ; NORD-ESTGAA RD BG ; TYBJAERG-HANSEN A.** Loss-of-function mutations in APOC3 and risk of ischemic vascular disease. *THE NEW ENGLAND JOURNAL OF MEDICINE,* 2014, vol. 371, 32-41
- **JORGENSEN et al.** *The Danish Inter99 study,* 2003
- **VAN DEN DONK et al.** *The Danish Addition study,* 2011
- **HOOGENDOOM et al.** *The Nijmegen Biomedical Study,* 2006
- **WETZELS et al.** *The Nijmegen Biomedical Study,* 2007
- **JØRGENSEN et al.** *The Danish Inter99 study,* 2003
- **LAURITZEN et al.** *The Danish Addition study,* 2000
- **GREEN et al.** *Nature Genetics,* 1994, vol. 7, 13-21
- **MENDEZ et al.** *Nature Genetics,* 1997, vol. 15, 146-156
- **GREEN ; JAKOBOVITIS.** *J. Ex. Med,* 1998, vol. 188, 483-495
- **KELLERMAN ; GREEN.** *Current Opinion in Biotechnology,* 2002, vol. 13, 593-597
- **JANSSENS et al.** *PNAS,* 2006, vol. 103, 15130-15135
- **MEIER, M. ; BIDER, M. D. ; MALASHKEVICH, V. N. ; SPIESS, M. ; BURKHARD, P.** Crystal structure of the carbohydrate recognition domain of the H1 subunit of the asialoglycoprotein receptor. *Journal of molecular biology,* 2000, vol. 300, 857-865
- **BATTYE, T. G. ; KONTOGIANNIS, L. ; JOHNSON, O. ; POWELL, H. R. ; LESLIE, A. G.** iMOSFLM: a new graphical interface for diffraction-image processing with MOSFLM. *Acta crystallographica,* 2011, vol. 67, 271-281
- **EVANS, P.** Scaling and assessment of data quality. *Acta crystallographica,* 2006, vol. 62, 72-82
- The CCP4 suite: programs for protein crystallography. *Acta crystallographic a,* 1994, vol. 50, 760-763
- **MCCOY, A. J. ; GROSSE-KUNSTLEVE, R. W. ; AD-AMS, P. D. ; WINN, M. D. ; STORONI, L. C. ; READ, R. J.** Phaser crystallographic software. *Journal of applied crystallography,* 2007, vol. 40, 658-674
- **EMSLEY, P. ; LOHKAMP, B. ; SCOTT, W. G. ; COWTAN, K.** Features and development of Coot. *Acta crystallographica,* 2010, vol. 66, 486-501
- **ADAMS, P. D. ; AFONINE, P. V. ; BUNKOCZI, G. ; CHEN, V. B. ; DAVIS, I. W. ; ECHOLS, N. ; HEADD, J. J. ; HUNG, L. W. ; KAPRAL, G. J. ; GROSSE-KUNSTLEVE, R. W.** PHENIX: a comprehensive Python-based system for macromolecular structure solution. *Acta crystallographic a,* 2010, vol. 66, 213-221
- **DELANO, W. L.** *The PyMOL Molecular Graphics System. Palo Alto,* 2002
- **BATTYE, T.G. ; KONTOGIANNIS, L. ; JOHNSON, O. ; POWELL, H.R. ; LESLIE, A.G.** iMOSFLM: a new graphical interface for diffraction-image processing with MOSFLM. *Acta Crystallogr D Biol Crystallogr,* 2011, vol. 67, 271-81
- The CCP4 suite: programs for protein crystallography. *Acta Crystallogr D Biol Crystallogr,* 1994, vol. 50, 760-3
- **EMSLEY, P. ; LOHKAMP, B. ; SCOTT, W.G. ; COWTAN, K.** Features and development of Coot. *Acta Crystallogr D Biol Crystallogr,* 2010, vol. 66, 486-501
- **ADAMS, P.D. et al.** PHENIX: a comprehensive Python-based system for macromolecular structure solution. *Acta Crystallogr D Biol Crystallogr,* 2010, vol. 66, 213-21
- **DELANO, W.L.** *The PyMOL Molecular Graphics System. (Palo Alto,* 2002
- Molecular Operating Environment (MOE). Chemical Computing Group, Inc, August 2013
- **NIOI, P. et al.** Variant ASGR1 Associated with a Reduced Risk of Coronary Artery Disease. *The New England journal of medicine,* 2016, vol. 374, 2131-2141
- **GOLD, L. et al.** Aptamer-based multiplexed proteomic technology for biomarker discovery. *PLoS One,* 2010, vol. 5, e15004
- **SHAM, P. C. ; PURCELL, S. M.** Statistical power and significance testing in large-scale genetic studies. *Nature reviews. Genetics,* 2014, vol. 15, 335-346